# EUROPEAN PATENT APPLICATION

(11) **EP 2 080 769 A2**
(43) Date of publication of application: **22.07.2009**
(21) Application number: 09156897.2
(22) Date of filing: 29.06.2005
(51) Int. Cl.: C07K 14/425, C07K 14/395, C12P 1/00, C12P 1/04

(54) **Process for the production of fine chemicals**

(30) Priority: 02.07.2004 EP 04015608; 15.07.2004 EP 04016615; 05.08.2004 EP 04018543; 23.08.2004 EP 04105689; 27.08.2004 EP 04105535; 03.11.2004 EP 04026008; 03.11.2004 EP 04026007; 04.11.2004 EP 04026057; 04.11.2004 EP 04026056; 03.12.2004 EP 04028670; 03.12.2004 EP 04028671; 17.12.2004 EP 04106931; 18.12.2004 EP 04030100; 18.12.2004 EP 04030101; 22.12.2004 EP 04030391; 23.12.2004 EP 04107024; 28.12.2004 EP 04107025; 10.01.2005 EP 05100166; 26.01.2005 EP 05100704; 14.03.2005 EP 05101970; 20.04.2005 EP 05103164; 22.04.2005 EP 05103455; 22.04.2005 EP 05103449; 26.04.2005 EP 05103283; 27.04.2005 EP 05103428; 25.05.2005 EP 05104479; 25.05.2005 EP 05104496; 27.05.2005 EP 05104781; 30.05.2005 EP 05104630; 01.06.2005 EP 05104761; 02.06.2005 EP 05104818; 02.06.2005 EP 05104811; 03.06.2005 EP 05104874; 06.06.2005 EP 05105001; 08.06.2005 EP 05105021; 08.06.2005 EP 05105028; 10.06.2005 EP 05105345; 13.06.2005 EP 05105136; 17.06.2005 EP 05105405; 17.06.2005 EP 05105401; 17.06.2005 EP 05105406; 21.06.2005 EP 05105508; 21.06.2005 EP 05105510; 22.06.2005 EP 05105575; 22.06.2005 EP 05105571; 22.06.2005 EP 05105570; 23.06.2005 EP 05105643; 23.06.2005 EP 05105624; 27.06.2005 EP 05105992; 27.06.2005 EP 05105993
(62) Divisional of application: 05850205.5
(71) Applicant: Metanomics GmbH, 10589 Berlin (DE)
(72) Inventor: Plesch, Gunnar, 14482 Potsdam (DE); Puzio, Piotr, 9030 Mariakerke (BE); Blau, Astrid, 14532 Stahnsdorf (DE); Looser, Ralf, 13158 Berlin (DE); Wendel, Birgit, 12163 Berlin (DE); Kamlage, Beate, 10711 Berlin (DE); Schmitz, Oliver, 14624 Dallgow-Döberitz (DE)
(74) Representative: Fitzner, Uwe

(57) **Abstract**

The present invention relates to a process for the production leucine and/or isoleucine and/or valine in a microorganism, a plant cell, a plant, a plant tissue or in one or more parts thereof. The invention furthermore relates to nucleic acid molecules, polypeptides, nucleic acid constructs, vectors, antisense molecules, antibodies, host cells, plant tissue, propagation material, harvested material, plants, microorganisms as well as agricultural compositions and to their use.

SEQUENCE LISTING

<110> Metanomics GmbH & Co.KGaA

<120> Process for the production of fine chemicals

<130> PF 55709

<140> Current patent application

<141> 2005 06 28

<160> 100181

<170> Biomax PatentTool according to PatentIN 3.1 format

<210> 1
<211> 1612
<212> DNA
<213> Saccharomyces cerevisiae

<220>
<221> CDS
<222> (167)..(1330)

<400> 1
tgtggtagta cgagtttttt ctccgcaatc tcggagttag tgcagccgcg cccgctgctg     60

caccctttag catatatata aagtgcgtaa tgtgcgtgct aaaaaagctc actagtgcgt     120

actctgctta tctctgcgta tccgccgatc aacatccgcg gctaaa atg gaa cag       175
                                                   Met Glu Gln
                                                   1
aac agg ttc aag aaa gaa acg aaa act tgt agc gct agc tgg ccg cgc       223
Asn Arg Phe Lys Lys Glu Thr Lys Thr Cys Ser Ala Ser Trp Pro Arg
    5                   10                  15
gca ccg cag tcg acg ctg tgt gcg aca gat cgc ctc gag ctc aca tac       271
Ala Pro Gln Ser Thr Leu Cys Ala Thr Asp Arg Leu Glu Leu Thr Tyr
20                  25                  30                  35
gat gtg tac act agc gca gag cgg caa cgc cgc tct cgc act gcc act       319
Asp Val Tyr Thr Ser Ala Glu Arg Gln Arg Arg Ser Arg Thr Ala Thr
                40                  45                  50
agg ctt aac ctt gtg ttt ttg cac ggc agc ggc atg agt aag gtg gta       367
Arg Leu Asn Leu Val Phe Leu His Gly Ser Gly Met Ser Lys Val Val
            55                  60                  65
tgg gag tac tat ttg ccg cgt ctg gta gcc gcc gat gcg gag ggc aat       415
Trp Glu Tyr Tyr Leu Pro Arg Leu Val Ala Ala Asp Ala Glu Gly Asn
        70                  75                  80
tat gcc atc gac aag gtt ttg ttg atc gac cag gtt aac cac ggt gat       463
Tyr Ala Ile Asp Lys Val Leu Leu Ile Asp Gln Val Asn His Gly Asp
    85                  90                  95
tct gcg gta cgc aac cgc ggc aga ctc ggc acc aat ttc aac tgg atc       511
Ser Ala Val Arg Asn Arg Gly Arg Leu Gly Thr Asn Phe Asn Trp Ile
100                 105                 110                 115
gac ggg gcc cgc gac gtg ctc aag att gcc aca tgc gag ttg ggc agt       559
Asp Gly Ala Arg Asp Val Leu Lys Ile Ala Thr Cys Glu Leu Gly Ser
                120                 125                 130
att gac agc cac ccg gca cta aac gta gtc atc ggc cac tcg atg ggc       607
Ile Asp Ser His Pro Ala Leu Asn Val Val Ile Gly His Ser Met Gly
            135                 140                 145
ggg ttc cag gct ctt gcg tgt gac gtc ctg cag ccc aat ctg ttt cat       655
Gly Phe Gln Ala Leu Ala Cys Asp Val Leu Gln Pro Asn Leu Phe His
        150                 155                 160
ttg ctc atc ttg atc gag cct gta gta atc aca cgg aaa gcc atc ggc       703
Leu Leu Ile Leu Ile Glu Pro Val Val Ile Thr Arg Lys Ala Ile Gly
    165                 170                 175
gcc ggg agg cca ggg ctt ccg ccc gat tct ccg cag atc cca gaa aat       751
Ala Gly Arg Pro Gly Leu Pro Pro Asp Ser Pro Gln Ile Pro Glu Asn
180                 185                 190                 195

ctt tat aac tcc cta cgt tta aag acg tgc gac cat ttc gct aac gag     799
Leu Tyr Asn Ser Leu Arg Leu Lys Thr Cys Asp His Phe Ala Asn Glu
                200                 205                 210
tcc gaa tat gta aaa tat atg agg aac ggt tcc ttt ttt acc aat gcg     847
Ser Glu Tyr Val Lys Tyr Met Arg Asn Gly Ser Phe Phe Thr Asn Ala
            215                 220                 225
cac agc caa atc ctg caa aac atc atc gat ttt gag agg aca aaa gcc     895
His Ser Gln Ile Leu Gln Asn Ile Ile Asp Phe Glu Arg Thr Lys Ala
        230                 235                 240
tct gga gac gat gaa gat ggt gga ccc gtt cgc acg aag atg gag cag     943
Ser Gly Asp Asp Glu Asp Gly Gly Pro Val Arg Thr Lys Met Glu Gln
    245                 250                 255
gct caa aat ctc ctc tgc tac atg aac atg cag act ttt gcg cct ttc     991
Ala Gln Asn Leu Leu Cys Tyr Met Asn Met Gln Thr Phe Ala Pro Phe
260                 265                 270                 275
ctg atc agc aat gtg aag ttt gtg agg aag cgg act atc cac att gtg     1039
Leu Ile Ser Asn Val Lys Phe Val Arg Lys Arg Thr Ile His Ile Val
                280                 285                 290
ggc gcg cgc tcc aac tgg tgt cct ccg caa aat cag ctg ttt ttg cag     1087
Gly Ala Arg Ser Asn Trp Cys Pro Pro Gln Asn Gln Leu Phe Leu Gln
            295                 300                 305
aaa act cta cag aac tac cat ctc gat gtc att ccc ggc ggc tcc cac     1135
Lys Thr Leu Gln Asn Tyr His Leu Asp Val Ile Pro Gly Gly Ser His
        310                 315                 320
ttg gtc aac gtt gag gct ccg gac ctg gtg atc gag agg att aat cac     1183
Leu Val Asn Val Glu Ala Pro Asp Leu Val Ile Glu Arg Ile Asn His
    325                 330                 335
cac atc cac gag ttt gtc ctt acc tcc ccg ctg cag tcc tca cac att     1231
His Ile His Glu Phe Val Leu Thr Ser Pro Leu Gln Ser Ser His Ile
340                 345                 350                 355
ccg caa ttg acc ctt gaa gaa aga gcg gtg atg ttt gac cgg gct ttc     1279
Pro Gln Leu Thr Leu Glu Glu Arg Ala Val Met Phe Asp Arg Ala Phe
                360                 365                 370
gac tcg ttc aag aat gaa gct ttg gtt aaa acg act aaa caa aaa ctg     1327
Asp Ser Phe Lys Asn Glu Ala Leu Val Lys Thr Thr Lys Gln Lys Leu
            375                 380                 385
taaacacttg tgtacactat tacatatatt attcaataat aattaactga cgcaatctat     1387

ggcactggcg tggtttcttt ttcttttgtt ttccgtgcaa gcgcgatgca actatatcgt     1447

cgaggacgaa tgtgtacccg tctttccgcc agacaggtaa caagtacttc aattgactta     1507

gaagaaaaac attaaggaac tttaacgtag gagcaacaca ataccaggca cacgctcttc     1567

gaacactgaa ccacacgcgt ccgcatcaaa ctcttcctcc caaac     1612

<210> 2
<211> 387
<212> PRT
<213> Saccharomyces cerevisiae

<400> 2
Met Glu Gln Asn Arg Phe Lys Lys Glu Thr Lys Thr Cys Ser Ala Ser
1               5                   10                  15
Trp Pro Arg Ala Pro Gln Ser Thr Leu Cys Ala Thr Asp Arg Leu Glu
            20                  25                  30
Leu Thr Tyr Asp Val Tyr Thr Ser Ala Glu Arg Gln Arg Arg Ser Arg
        35                  40                  45
Thr Ala Thr Arg Leu Asn Leu Val Phe Leu His Gly Ser Gly Met Ser
    50                  55                  60
Lys Val Val Trp Glu Tyr Tyr Leu Pro Arg Leu Val Ala Ala Asp Ala
65                  70                  75                  80
Glu Gly Asn Tyr Ala Ile Asp Lys Val Leu Leu Ile Asp Gln Val Asn
                85                  90                  95
His Gly Asp Ser Ala Val Arg Asn Arg Gly Arg Leu Gly Thr Asn Phe
            100                 105                 110

Asn Trp Ile Asp Gly Ala Arg Asp Val Leu Lys Ile Ala Thr Cys Glu
        115                 120                 125
Leu Gly Ser Ile Asp Ser His Pro Ala Leu Asn Val Val Ile Gly His
    130                 135                 140
Ser Met Gly Gly Phe Gln Ala Leu Ala Cys Asp Val Leu Gln Pro Asn
145                 150                 155                 160
Leu Phe His Leu Leu Ile Leu Ile Glu Pro Val Val Ile Thr Arg Lys
                165                 170                 175
Ala Ile Gly Ala Gly Arg Pro Gly Leu Pro Pro Asp Ser Pro Gln Ile
            180                 185                 190
Pro Glu Asn Leu Tyr Asn Ser Leu Arg Leu Lys Thr Cys Asp His Phe
        195                 200                 205
Ala Asn Glu Ser Glu Tyr Val Lys Tyr Met Arg Asn Gly Ser Phe Phe
    210                 215                 220
Thr Asn Ala His Ser Gln Ile Leu Gln Asn Ile Ile Asp Phe Glu Arg
225                 230                 235                 240
Thr Lys Ala Ser Gly Asp Asp Glu Asp Gly Gly Pro Val Arg Thr Lys
                245                 250                 255
Met Glu Gln Ala Gln Asn Leu Leu Cys Tyr Met Asn Met Gln Thr Phe
            260                 265                 270
Ala Pro Phe Leu Ile Ser Asn Val Lys Phe Val Arg Lys Arg Thr Ile
        275                 280                 285
His Ile Val Gly Ala Arg Ser Asn Trp Cys Pro Pro Gln Asn Gln Leu
    290                 295                 300
Phe Leu Gln Lys Thr Leu Gln Asn Tyr His Leu Asp Val Ile Pro Gly
305                 310                 315                 320
Gly Ser His Leu Val Asn Val Glu Ala Pro Asp Leu Val Ile Glu Arg
                325                 330                 335
Ile Asn His His Ile His Glu Phe Val Leu Thr Ser Pro Leu Gln Ser
            340                 345                 350
Ser His Ile Pro Gln Leu Thr Leu Glu Glu Arg Ala Val Met Phe Asp
        355                 360                 365
Arg Ala Phe Asp Ser Phe Lys Asn Glu Ala Leu Val Lys Thr Thr Lys
    370                 375                 380
Gln Lys Leu
385

<210> 3
<211> 25
<212> DNA
<213> Primer

<400> 3
atggaacaga acaggttcaa gaaag                                          25

<210> 4
<211> 27
<212> DNA
<213> Primer

<400> 4
ttacagtttt tgtttagtcg ttttaac                                        27

<210> 5
<211> 1032
<212> DNA
<213> Saccharomyces cerevisiae

<220>
<221> CDS
<222> (1)..(1032)

<400> 5
atg agt aat gca aac aat agt gct atg aac cac atc acg ttg cct ccc      48
Met Ser Asn Ala Asn Asn Ser Ala Met Asn His Ile Thr Leu Pro Pro
1               5                   10                  15
atc tcg tca ttt gac aac ctt atc aag gca gcc gag aga caa tat aat      96
Ile Ser Ser Phe Asp Asn Leu Ile Lys Ala Ala Glu Arg Gln Tyr Asn
            20                  25                  30
ggc gag gct tcg tcc gcc tcg aca cat ccg aca ctg cct aat atg aac      144
Gly Glu Ala Ser Ser Ala Ser Thr His Pro Thr Leu Pro Asn Met Asn
        35                  40                  45

att agt aac ggc agt ggc agt gcg ggc gca tca tcg tcg atg ctg agc 192
Ile Ser Asn Gly Ser Gly Ser Ala Gly Ala Ser Ser Ser Met Leu Ser
    50                  55                  60
tat cag ttg ctt ccg cac tcc aat gat gtg tcc cgg tcg aac tcc agc 240
Tyr Gln Leu Leu Pro His Ser Asn Asp Val Ser Arg Ser Asn Ser Ser
65                  70                  75                  80
tcg agc ttc ctt ccc tcc gtg cag cag cct acg gaa ggt tcc gct tct 288
Ser Ser Phe Leu Pro Ser Val Gln Gln Pro Thr Glu Gly Ser Ala Ser
                85                  90                  95
gca tcg gaa act tct tca tct gca tca cct tca cgt tcc att tca cca 336
Ala Ser Glu Thr Ser Ser Ser Ala Ser Pro Ser Arg Ser Ile Ser Pro
            100                 105                 110
att tta aaa gtg gct ggt cct tca tct gtt ggc ggt gcc ggg gtg agt 384
Ile Leu Lys Val Ala Gly Pro Ser Ser Val Gly Gly Ala Gly Val Ser
        115                 120                 125
acc ccc cat agc acc aaa atc aat aag cca agg aaa aag aag caa tgt 432
Thr Pro His Ser Thr Lys Ile Asn Lys Pro Arg Lys Lys Lys Gln Cys
    130                 135                 140
cca att tgt cgt aat ttt tat gcc aat ctg acc acg cac aag gca acg 480
Pro Ile Cys Arg Asn Phe Tyr Ala Asn Leu Thr Thr His Lys Ala Thr
145                 150                 155                 160
cac ttg acg cca gag gat cgg cct cac aag tgt ccc atc tgc cac cgc 528
His Leu Thr Pro Glu Asp Arg Pro His Lys Cys Pro Ile Cys His Arg
                165                 170                 175
ggg ttc gcc agg aac aat gac tta ttg agg cat aag aaa aga cat tgg 576
Gly Phe Ala Arg Asn Asn Asp Leu Leu Arg His Lys Lys Arg His Trp
            180                 185                 190
aag gat gag ata ctg tcc caa tcg gga gta ctt tcg aac cat aat gat 624
Lys Asp Glu Ile Leu Ser Gln Ser Gly Val Leu Ser Asn His Asn Asp
        195                 200                 205
ggt aag gga gga tcc gtt tct ccc aat gat gat gat acc cat gaa aaa 672
Gly Lys Gly Gly Ser Val Ser Pro Asn Asp Asp Asp Thr His Glu Lys
    210                 215                 220
atg aca ccg atg aat tct gtc aca gat tat gca caa ttg aaa tcg ttg 720
Met Thr Pro Met Asn Ser Val Thr Asp Tyr Ala Gln Leu Lys Ser Leu
225                 230                 235                 240
cat caa atc aag ggc aca ttc aag tgc cca ttc aac tcc acc tta ata 768
His Gln Ile Lys Gly Thr Phe Lys Cys Pro Phe Asn Ser Thr Leu Ile
                245                 250                 255
caa ttg gac atg gat atg tac ccg tat aaa ctg aaa cct tta aat ttt 816
Gln Leu Asp Met Asp Met Tyr Pro Tyr Lys Leu Lys Pro Leu Asn Phe
            260                 265                 270
gag act tcc aat tgt cac cag acg ggg gtg ttc tca cgt tgc gac acc 864
Glu Thr Ser Asn Cys His Gln Thr Gly Val Phe Ser Arg Cys Asp Thr
        275                 280                 285
ttc aaa aac cat tta aag gct ttg cat ttt gaa tac ccg cca ggc acg 912
Phe Lys Asn His Leu Lys Ala Leu His Phe Glu Tyr Pro Pro Gly Thr
    290                 295                 300
aag aaa aag gat aga aat gtt gtt cct ggt aga tgt aag cat tgc ggc 960
Lys Lys Lys Asp Arg Asn Val Val Pro Gly Arg Cys Lys His Cys Gly
305                 310                 315                 320
ctg aag ttc gag aac gtt gac gtt tgg ctc aac gaa cac gta ggt aaa 1008
Leu Lys Phe Glu Asn Val Asp Val Trp Leu Asn Glu His Val Gly Lys
                325                 330                 335
caa tgc ggc tat aaa tac cat tga 1032
Gln Cys Gly Tyr Lys Tyr His
            340

<210> 6
<211> 343
<212> PRT
<213> Saccharomyces cerevisiae

<400> 6
Met Ser Asn Ala Asn Asn Ser Ala Met Asn His Ile Thr Leu Pro Pro
1               5                   10                  15
Ile Ser Ser Phe Asp Asn Leu Ile Lys Ala Ala Glu Arg Gln Tyr Asn
            20                  25                  30
Gly Glu Ala Ser Ser Ala Ser Thr His Pro Thr Leu Pro Asn Met Asn
        35                  40                  45
Ile Ser Asn Gly Ser Gly Ser Ala Gly Ala Ser Ser Ser Met Leu Ser
    50                  55                  60

Tyr Gln Leu Leu Pro His Ser Asn Asp Val Ser Arg Ser Asn Ser Ser
65                  70                  75                  80
Ser Ser Phe Leu Pro Ser Val Gln Gln Pro Thr Glu Gly Ser Ala Ser
                85                  90                  95
Ala Ser Glu Thr Ser Ser Ser Ala Ser Pro Ser Arg Ser Ile Ser Pro
            100                 105                 110
Ile Leu Lys Val Ala Gly Pro Ser Ser Val Gly Gly Ala Gly Val Ser
        115                 120                 125
Thr Pro His Ser Thr Lys Ile Asn Lys Pro Arg Lys Lys Lys Gln Cys
    130                 135                 140
Pro Ile Cys Arg Asn Phe Tyr Ala Asn Leu Thr Thr His Lys Ala Thr
145                 150                 155                 160
His Leu Thr Pro Glu Asp Arg Pro His Lys Cys Pro Ile Cys His Arg
                165                 170                 175
Gly Phe Ala Arg Asn Asn Asp Leu Leu Arg His Lys Lys Arg His Trp
            180                 185                 190
Lys Asp Glu Ile Leu Ser Gln Ser Gly Val Leu Ser Asn His Asn Asp
        195                 200                 205
Gly Lys Gly Gly Ser Val Ser Pro Asn Asp Asp Asp Thr His Glu Lys
    210                 215                 220
Met Thr Pro Met Asn Ser Val Thr Asp Tyr Ala Gln Leu Lys Ser Leu
225                 230                 235                 240
His Gln Ile Lys Gly Thr Phe Lys Cys Pro Phe Asn Ser Thr Leu Ile
                245                 250                 255
Gln Leu Asp Met Asp Met Tyr Pro Tyr Lys Leu Lys Pro Leu Asn Phe
            260                 265                 270
Glu Thr Ser Asn Cys His Gln Thr Gly Val Phe Ser Arg Cys Asp Thr
        275                 280                 285
Phe Lys Asn His Leu Lys Ala Leu His Phe Glu Tyr Pro Pro Gly Thr
    290                 295                 300
Lys Lys Lys Asp Arg Asn Val Val Pro Gly Arg Cys Lys His Cys Gly
305                 310                 315                 320
Leu Lys Phe Glu Asn Val Asp Val Trp Leu Asn Glu His Val Gly Lys
                325                 330                 335
Gln Cys Gly Tyr Lys Tyr His
            340

<210> 7
<211> 960
<212> DNA
<213> Eremothecium gossypii

<220>
<221> CDS
<222> (1)..(960)

<400> 7
atg gcg cag gac aac cac atc acg ctg cca ccg atc tcg tcg ttc gac       48
Met Ala Gln Asp Asn His Ile Thr Leu Pro Pro Ile Ser Ser Phe Asp
1               5                   10                  15
aat ctg atc cgg gca gcg acg gag cag gac ttc cac ggc ggc gca gcg       96
Asn Leu Ile Arg Ala Ala Thr Glu Gln Asp Phe His Gly Gly Ala Ala
            20                  25                  30
ccg gcc gcg cac ggg cgc ggg gcg agc ggc ggc ggc agc gcg agc gag      144
Pro Ala Ala His Gly Arg Gly Ala Ser Gly Gly Gly Ser Ala Ser Glu
        35                  40                  45
tcg ctg ctg tcc tac cag tta tcg aac gtg agc cgg acg ggg tcg atg      192
Ser Leu Leu Ser Tyr Gln Leu Ser Asn Val Ser Arg Thr Gly Ser Met
    50                  55                  60
tcg ggg tcg ctg tcg ccg gac cac aac aac tac ttc atg ccg ttc cgg      240
Ser Gly Ser Leu Ser Pro Asp His Asn Asn Tyr Phe Met Pro Phe Arg
65                  70                  75                  80
cat gcg tcg ctg gat ggc ggg cgg ctg ggg ggg agc cag ccg tcg tcg      288
His Ala Ser Leu Asp Gly Gly Arg Leu Gly Gly Ser Gln Pro Ser Ser
                85                  90                  95
gtg gac ggg tcg ccg tcg ctg aag gcg cag ctg ggt ctg ccg cac ggg      336
Val Asp Gly Ser Pro Ser Leu Lys Ala Gln Leu Gly Leu Pro His Gly
            100                 105                 110
acc ggg gcg aag gtg tac aag acg aac aag ccg cgc aag aag aag gag      384
Thr Gly Ala Lys Val Tyr Lys Thr Asn Lys Pro Arg Lys Lys Lys Glu
        115                 120                 125
tgc ccg gtg tgc cac aac ttc tat gcg aac ctg tcc acg cat aag tcg      432

Cys Pro Val Cys His Asn Phe Tyr Ala Asn Leu Ser Thr His Lys Ser
    130.                135                 140
acg cac ctg gac ccg gag gcg cgg ccg cac aag tgc gac ata tgc tcg       480
Thr His Leu Asp Pro Glu Ala Arg Pro His Lys Cys Asp Ile Cys Ser
145                 150                 155                 160
cgg ggc ttc gcg cgg tcg aac gac ctg cag cgg cac aag aag cgg cac       528
Arg Gly Phe Ala Arg Ser Asn Asp Leu Gln Arg His Lys Lys Arg His
                165                 170                 175
tgg aag gac gag ttc tcg ccg agc cag gag ctg gcc aag gcg gac ggg       576
Trp Lys Asp Glu Phe Ser Pro Ser Gln Glu Leu Ala Lys Ala Asp Gly
            180                 185                 190
gac gag cag cag cag caa ggc gcg ggc agc aag ctg gag agc agc atc       624
Asp Glu Gln Gln Gln Gln Gly Ala Gly Ser Lys Leu Glu Ser Ser Ile
        195                 200                 205
ccc acc gag caa cta aaa agt tta cat atg ata cag ggt acg tac aag       672
Pro Thr Glu Gln Leu Lys Ser Leu His Met Ile Gln Gly Thr Tyr Lys
    210                 215                 220
tgc ccg ttc aac tcg acg ctg atc aag ctg gac atg gag atc tac ccc       720
Cys Pro Phe Asn Ser Thr Leu Ile Lys Leu Asp Met Glu Ile Tyr Pro
225                 230                 235                 240
acg aag ggg cgg cgg ctg gcg ttc gag acc tcc aac tgt cat atc acc       768
Thr Lys Gly Arg Arg Leu Ala Phe Glu Thr Ser Asn Cys His Ile Thr
                245                 250                 255
ggc gtt ttc tcg cgc tgc gac acg tac aag aac cat ctt aag gcg ctg       816
Gly Val Phe Ser Arg Cys Asp Thr Tyr Lys Asn His Leu Lys Ala Leu
            260                 265                 270
cac ttc gag tac ccg ccg ggc acg aag aag cgc gag cgc gcc aag atc       864
His Phe Glu Tyr Pro Pro Gly Thr Lys Lys Arg Glu Arg Ala Lys Ile
        275                 280                 285
cac ggc cgc tgc aag cac tgt ggc atg aag ttc gac aac gtg gac atg       912
His Gly Arg Cys Lys His Cys Gly Met Lys Phe Asp Asn Val Asp Met
    290                 295                 300
tgg ctg aac gag cat gtc ggc aag gtg tgc ggg tac ttc tac cac           957
Trp Leu Asn Glu His Val Gly Lys Val Cys Gly Tyr Phe Tyr His
305                 310                 315
tga                                                                   960

<210> 8
<211> 319
<212> PRT
<213> Eremothecium gossypii

<400> 8
   Met Ala Gln Asp Asn His Ile Thr Leu Pro Pro Ile Ser Ser Phe Asp
   1 5 10 15
   Asn Leu Ile Arg Ala Ala Thr Glu Gln Asp Phe His Gly Gly Ala Ala
   20 25 30
   Pro Ala Ala His Gly Arg Gly Ala Ser Gly Gly Gly Ser Ala Ser Glu
   35 40 45
   Ser Leu Leu Ser Tyr Gln Leu Ser Asn Val Ser Arg Thr Gly Ser Met
   50 55 60
   Ser Gly Ser Leu Ser Pro Asp His Asn Asn Tyr Phe Met Pro Phe Arg
   65 70 75 80
   His Ala Ser Leu Asp Gly Gly Arg Leu Gly Gly Ser Gln Pro Ser Ser
   85 90 95
   Val Asp Gly Ser Pro Ser Leu Lys Ala Gln Leu Gly Leu Pro His Gly
   100 105 110
   Thr Gly Ala Lys Val Tyr Lys Thr Asn Lys Pro Arg Lys Lys Lys Glu
   115 120 125
   Cys Pro Val Cys His Asn Phe Tyr Ala Asn Leu Ser Thr His Lys Ser
   130 135 140
   Thr His Leu Asp Pro Glu Ala Arg Pro His Lys Cys Asp Ile Cys Ser
   145 150 155 160
   Arg Gly Phe Ala Arg Ser Asn Asp Leu Gln Arg His Lys Lys Arg His
   165 170 175
   Trp Lys Asp Glu Phe Ser Pro Ser Gln Glu Leu Ala Lys Ala Asp Gly
   180 185 190
   Asp Glu Gln Gln Gln Gln Gly Ala Gly Ser Lys Leu Glu Ser Ser Ile
   195 200 205
   Pro Thr Glu Gln Leu Lys Ser Leu His Met Ile Gln Gly Thr Tyr Lys
       210                 215                 220
   Cys Pro Phe Asn Ser Thr Leu Ile Lys Leu Asp Met Glu Ile Tyr Pro
   225                 230                 235                 240
   Thr Lys Gly Arg Arg Leu Ala Phe Glu Thr Ser Asn Cys His Ile Thr
                   245                 250                 255
   Gly Val Phe Ser Arg Cys Asp Thr Tyr Lys Asn His Leu Lys Ala Leu
               260                 265                 270
   His Phe Glu Tyr Pro Pro Gly Thr Lys Lys Arg Glu Arg Ala Lys Ile
           275                 280                 285
   His Gly Arg Cys Lys His Cys Gly Met Lys Phe Asp Asn Val Asp Met
       290                 295                 300
   Trp Leu Asn Glu His Val Gly Lys Val Cys Gly Tyr Phe Tyr His
   305                 310                 315

<210> 9
<211> 1473
<212> DNA
<213> Saccharomyces cerevisiae

<220>
<221> CDS
<222> (1)..(1473)

<400> 9
atg ctg gta tca tca tct ttt gca tca agc att gac tca gta atg tcc       48
Met Leu Val Ser Ser Ser Phe Ala Ser Ser Ile Asp Ser Val Met Ser
1               5                   10                  15
cat gag acg atg tct tta cgg aga aac cct ccc ttt ata gat act cca       96
His Glu Thr Met Ser Leu Arg Arg Asn Pro Pro Phe Ile Asp Thr Pro
            20                  25                  30
gaa aaa atg cca aac cca acg gct tct ccc aat ggc acc att cac cat      144
Glu Lys Met Pro Asn Pro Thr Ala Ser Pro Asn Gly Thr Ile His His
        35                  40                  45
ttg ata gat cca tca ctg cca ttg ttg tcc agt act acg tcg tct tcg      192
Leu Ile Asp Pro Ser Leu Pro Leu Leu Ser Ser Thr Thr Ser Ser Ser
    50                  55                  60
cgc tct acc ctc agt tct aca ctg aat tct cct cca cct ccc cca tta      240
Arg Ser Thr Leu Ser Ser Thr Leu Asn Ser Pro Pro Pro Pro Pro Leu
65                  70                  75                  80
act aca tca tac tct tct tat aac tcc tct gca tgc cag tct atc acg      288
Thr Thr Ser Tyr Ser Ser Tyr Asn Ser Ser Ala Cys Gln Ser Ile Thr
                85                  90                  95
agt tcg cct aca gac aat aca gcc ttg gca cat aat tcc aaa tgc tat      336
Ser Ser Pro Thr Asp Asn Thr Ala Leu Ala His Asn Ser Lys Cys Tyr
            100                 105                 110
ttc cct cat agc ctt tct cca aca ccg ctg tca tcc aac agc tca agc      384
Phe Pro His Ser Leu Ser Pro Thr Pro Leu Ser Ser Asn Ser Ser Ser
        115                 120                 125
cat gtc att ttg cca ccc ata tcc tcc ttc acc aat tta att aca gtt      432
His Val Ile Leu Pro Pro Ile Ser Ser Phe Thr Asn Leu Ile Thr Val
    130                 135                 140
gca gaa aga gaa ttt aac ggc aga tca aat tcc ttg cat gcc aat ttt      480
Ala Glu Arg Glu Phe Asn Gly Arg Ser Asn Ser Leu His Ala Asn Phe
145                 150                 155                 160
aca tca ccg gtg cca aga aca gtg cta gat cat cac aga cat gag cta      528
Thr Ser Pro Val Pro Arg Thr Val Leu Asp His His Arg His Glu Leu
                165                 170                 175
aca ttt tgc aac cct aac aac aca acg ggt ttc aaa acc atc acc ccc      576
Thr Phe Cys Asn Pro Asn Asn Thr Thr Gly Phe Lys Thr Ile Thr Pro
            180                 185                 190
tca cca cca act caa cac cag agt ata ttg cca acc gcg gta gat aac      624
Ser Pro Pro Thr Gln His Gln Ser Ile Leu Pro Thr Ala Val Asp Asn
        195                 200                 205
gta ccg aga tca aaa tcc gtg tcc agc cta ccc gtt agt gga ttc cca      672
Val Pro Arg Ser Lys Ser Val Ser Ser Leu Pro Val Ser Gly Phe Pro
    210                 215                 220
cct ttg atc gtc aaa caa caa cag caa cag caa ctc aac agt agt agt      720
Pro Leu Ile Val Lys Gln Gln Gln Gln Gln Gln Leu Asn Ser Ser Ser
225                 230                 235                 240
agt gcc tct gca cta ccg tct att cat tcc cca ttg act aat gaa cat      768
Ser Ala Ser Ala Leu Pro Ser Ile His Ser Pro Leu Thr Asn Glu His

                245                 250                 255
aca agc agg tat tca tcg tct tta aag gat tcc gcc aag ata acg aag        816
Thr Ser Arg Tyr Ser Ser Ser Leu Lys Asp Ser Ala Lys Ile Thr Lys
            260                 265                 270
caa aga aag aaa aag gaa tgc ccc atc tgc cac aac ttc tac gcc aac        864
Gln Arg Lys Lys Lys Glu Cys Pro Ile Cys His Asn Phe Tyr Ala Asn
        275                 280                 285
cta tct acg cat aaa tcc act cat tta act ccg gag gac aga ccg cat        912
Leu Ser Thr His Lys Ser Thr His Leu Thr Pro Glu Asp Arg Pro His
    290                 295                 300
aag tgc cct atc tgt caa cgc ggc ttt gcc agg aac aat gat ctg ata        960
Lys Cys Pro Ile Cys Gln Arg Gly Phe Ala Arg Asn Asn Asp Leu Ile
305                 310                 315                 320
agg cac aag aag cgc cat tgg aaa gat gaa ttt atg caa ata tac gca        1008
Arg His Lys Lys Arg His Trp Lys Asp Glu Phe Met Gln Ile Tyr Ala
                325                 330                 335
agg gag tcc gac aac aat agt ggt gct gac gat caa gat gac act gcc        1056
Arg Glu Ser Asp Asn Asn Ser Gly Ala Asp Asp Gln Asp Asp Thr Ala
            340                 345                 350
cgc acc tcc gca aac aac gac agc gac gac tca aac gat aaa ctc gct        1104
Arg Thr Ser Ala Asn Asn Asp Ser Asp Asp Ser Asn Asp Lys Leu Ala
        355                 360                 365
gcc tca tcc tct tcc gaa gaa aca aaa cta ctg aag aaa aac caa ttg        1152
Ala Ser Ser Ser Ser Glu Glu Thr Lys Leu Leu Lys Lys Asn Gln Leu
    370                 375                 380
aaa tca cta tac aag att aaa ggt gca ttc aag tgt cct tac aac tct        1200
Lys Ser Leu Tyr Lys Ile Lys Gly Ala Phe Lys Cys Pro Tyr Asn Ser
385                 390                 395                 400
act ctg atc aat ttg gat atg gag gtg tat cca cac aag tct aga tca        1248
Thr Leu Ile Asn Leu Asp Met Glu Val Tyr Pro His Lys Ser Arg Ser
                405                 410                 415
ctc tat ttc gag cca ata aac tgt cat caa acg ggc gtt ttt tcg cgc        1296
Leu Tyr Phe Glu Pro Ile Asn Cys His Gln Thr Gly Val Phe Ser Arg
            420                 425                 430
tgt gat act ttc aag aat cac cta aag gct ttg cat ttc gag tac ccg        1344
Cys Asp Thr Phe Lys Asn His Leu Lys Ala Leu His Phe Glu Tyr Pro
        435                 440                 445
ccc aag acc aag aaa gaa gat aga ggt gtt gtg ccg ggg aag tgc aag        1392
Pro Lys Thr Lys Lys Glu Asp Arg Gly Val Val Pro Gly Lys Cys Lys
    450                 455                 460
cat tgt gga ctg caa ttt ccg aac gtt gac gtc tgg cta aat aag cac        1440
His Cys Gly Leu Gln Phe Pro Asn Val Asp Val Trp Leu Asn Lys His
465                 470                 475                 480
gtg ggg aaa gga tgt ggc tac tca tat cac taa                            1473
Val Gly Lys Gly Cys Gly Tyr Ser Tyr His
                485                 490

<210> 10
<211> 490
<212> PRT
<213> Saccharomyces cerevisiae

<400> 10
   Met Leu Val Ser Ser Ser Phe Ala Ser Ser Ile Asp Ser Val Met Ser
   1               5                   10                  15
   His Glu Thr Met Ser Leu Arg Arg Asn Pro Pro Phe Ile Asp Thr Pro
               20                  25                  30
   Glu Lys Met Pro Asn Pro Thr Ala Ser Pro Asn Gly Thr Ile His His
           35                  40                  45
   Leu Ile Asp Pro Ser Leu Pro Leu Leu Ser Ser Thr Thr Ser Ser Ser
       50                  55                  60
   Arg Ser Thr Leu Ser Ser Thr Leu Asn Ser Pro Pro Pro Pro Pro Leu
   65                  70                  75                  80
   Thr Thr Ser Tyr Ser Ser Tyr Asn Ser Ser Ala Cys Gln Ser Ile Thr
                   85                  90                  95
   Ser Ser Pro Thr Asp Asn Thr Ala Leu Ala His Asn Ser Lys Cys Tyr
               100                 105                 110
   Phe Pro His Ser Leu Ser Pro Thr Pro Leu Ser Ser Asn Ser Ser Ser
           115                 120                 125
   His Val Ile Leu Pro Pro Ile Ser Ser Phe Thr Asn Leu Ile Thr Val
       130                 135                 140
   Ala Glu Arg Glu Phe Asn Gly Arg Ser Asn Ser Leu His Ala Asn Phe
   145                 150                 155                 160
   Thr Ser Pro Val Pro Arg Thr Val Leu Asp His His Arg His Glu Leu
                   165                 170                 175
   Thr Phe Cys Asn Pro Asn Asn Thr Thr Gly Phe Lys Thr Ile Thr Pro
               180                 185                 190
   Ser Pro Pro Thr Gln His Gln Ser Ile Leu Pro Thr Ala Val Asp Asn
           195                 200                 205
   Val Pro Arg Ser Lys Ser Val Ser Ser Leu Pro Val Ser Gly Phe Pro
       210                 215                 220
   Pro Leu Ile Val Lys Gln Gln Gln Gln Gln Gln Leu Asn Ser Ser Ser
   225                 230                 235                 240
   Ser Ala Ser Ala Leu Pro Ser Ile His Ser Pro Leu Thr Asn Glu His
                   245                 250                 255
   Thr Ser Arg Tyr Ser Ser Ser Leu Lys Asp Ser Ala Lys Ile Thr Lys
               260                 265                 270
   Gln Arg Lys Lys Lys Glu Cys Pro Ile Cys His Asn Phe Tyr Ala Asn
           275                 280                 285
   Leu Ser Thr His Lys Ser Thr His Leu Thr Pro Glu Asp Arg Pro His
       290                 295                 300
   Lys Cys Pro Ile Cys Gln Arg Gly Phe Ala Arg Asn Asn Asp Leu Ile
   305                 310                 315                 320
   Arg His Lys Lys Arg His Trp Lys Asp Glu Phe Met Gln Ile Tyr Ala
                   325                 330                 335
   Arg Glu Ser Asp Asn Asn Ser Gly Ala Asp Asp Gln Asp Asp Thr Ala
               340                 345                 350
   Arg Thr Ser Ala Asn Asn Asp Ser Asp Asp Ser Asn Asp Lys Leu Ala
           355                 360                 365
   Ala Ser Ser Ser Ser Glu Glu Thr Lys Leu Leu Lys Lys Asn Gln Leu
       370                 375                 380
   Lys Ser Leu Tyr Lys Ile Lys Gly Ala Phe Lys Cys Pro Tyr Asn Ser
   385                 390                 395                 400
   Thr Leu Ile Asn Leu Asp Met Glu Val Tyr Pro His Lys Ser Arg Ser
                   405                 410                 415
   Leu Tyr Phe Glu Pro Ile Asn Cys His Gln Thr Gly Val Phe Ser Arg
               420                 425                 430
   Cys Asp Thr Phe Lys Asn His Leu Lys Ala Leu His Phe Glu Tyr Pro
           435                 440                 445
   Pro Lys Thr Lys Lys Glu Asp Arg Gly Val Val Pro Gly Lys Cys Lys
       450                 455                 460
   His Cys Gly Leu Gln Phe Pro Asn Val Asp Val Trp Leu Asn Lys His
   465                 470                 475                 480
   Val Gly Lys Gly Cys Gly Tyr Ser Tyr His
                   485                 490

<210> 11
<211> 26
<212> DNA
<213> Primer

<400> 11
atgagtaatg caaacaatag tgctat                                          26

<210> 12
<211> 25
<212> DNA
<213> Primer

<400> 12
tcaatggtat ttatagccgc attgt                                           25

<210> 13
<211> 1980
<212> DNA
<213> Saccharomyces cerevisiae

<220>
<221> CDS
<222> (1)..(1980)

<400> 13
atg gat agt acg aat ttg aac aaa cgg ccc tta tta caa tat agt ctc       48

Met Asp Ser Thr Asn Leu Asn Lys Arg Pro Leu Leu Gln Tyr Ser Leu
1               5                   10                  15
agt tca ttg ggc tcg caa ata aca aaa tgg agc tca tct aga ccg act      96
Ser Ser Leu Gly Ser Gln Ile Thr Lys Trp Ser Ser Ser Arg Pro Thr
            20                  25                  30
tcg cca gtt cgt aag gcg aga agc act gaa aat gac ttt ctt tcc aag      144
Ser Pro Val Arg Lys Ala Arg Ser Thr Glu Asn Asp Phe Leu Ser Lys
        35                  40                  45
caa gat acg tct agt atc ctc cca agt atc aac gac gac ggc ggt gaa      192
Gln Asp Thr Ser Ser Ile Leu Pro Ser Ile Asn Asp Asp Gly Gly Glu
    50                  55                  60
cag tgg tac gaa aag ttc aag ccc aat tgt ttg gag caa gtg gcc ata      240
Gln Trp Tyr Glu Lys Phe Lys Pro Asn Cys Leu Glu Gln Val Ala Ile
65                  70                  75                  80
cat aaa aga aaa ctt aaa gat gta caa gaa gct tta gat gcc atg ttt      288
His Lys Arg Lys Leu Lys Asp Val Gln Glu Ala Leu Asp Ala Met Phe
                85                  90                  95
tta cct aac gcc aag cat agg atc cta cta ctg tct ggc ccc agt gga      336
Leu Pro Asn Ala Lys His Arg Ile Leu Leu Leu Ser Gly Pro Ser Gly
            100                 105                 110
tgc tct aaa agt acg gtc ata aaa gaa ctc tca aaa atc tta gtt cct      384
Cys Ser Lys Ser Thr Val Ile Lys Glu Leu Ser Lys Ile Leu Val Pro
        115                 120                 125
aaa tac aga caa aac agc aac gga acg tcc ttt cga agc acc ccg aac      432
Lys Tyr Arg Gln Asn Ser Asn Gly Thr Ser Phe Arg Ser Thr Pro Asn
    130                 135                 140
gag cat aaa gtg acc gag ttt aga ggt gat tgt ata gtc aac gat ctt      480
Glu His Lys Val Thr Glu Phe Arg Gly Asp Cys Ile Val Asn Asp Leu
145                 150                 155                 160
cct cag atg gaa agc ttt agt gag ttc tta aaa ggc gca cgg tat ctt      528
Pro Gln Met Glu Ser Phe Ser Glu Phe Leu Lys Gly Ala Arg Tyr Leu
                165                 170                 175
gtg atg tcc aac ctg tca tta ata ctt atc gag gac ctt ccc aac gtc      576
Val Met Ser Asn Leu Ser Leu Ile Leu Ile Glu Asp Leu Pro Asn Val
            180                 185                 190
ttc cat ata gat acc aga cgt cga ttt caa caa ctt ata tta cag tgg      624
Phe His Ile Asp Thr Arg Arg Arg Phe Gln Gln Leu Ile Leu Gln Trp
        195                 200                 205
cta tat agt tcg gag cct cta tta cct ccc ctt gtt ata tgt ata act      672
Leu Tyr Ser Ser Glu Pro Leu Leu Pro Pro Leu Val Ile Cys Ile Thr
    210                 215                 220
gaa tgt gaa att cca gag aac gat aat aat tat cgc aaa ttt ggt att      720
Glu Cys Glu Ile Pro Glu Asn Asp Asn Asn Tyr Arg Lys Phe Gly Ile
225                 230                 235                 240
gat tat aca ttt agt gca gaa acc ata atg aac aaa gaa ata ttg atg      768
Asp Tyr Thr Phe Ser Ala Glu Thr Ile Met Asn Lys Glu Ile Leu Met
                245                 250                 255
cat cca agg ttg aaa aga att aag ttt aat cca att aac agc act tta      816
His Pro Arg Leu Lys Arg Ile Lys Phe Asn Pro Ile Asn Ser Thr Leu
            260                 265                 270
tta aaa aag cac ttg aaa ttt att tgt gta cag aat atg aaa atg ttg      864
Leu Lys Lys His Leu Lys Phe Ile Cys Val Gln Asn Met Lys Met Leu
        275                 280                 285
aag gag aaa aat aaa tgg aat aaa aga cag gaa gtc ata gat tat att      912
Lys Glu Lys Asn Lys Trp Asn Lys Arg Gln Glu Val Ile Asp Tyr Ile
    290                 295                 300
gcg caa gag act ggt gat att agg tcg gcc att acg acc ctt caa ttt      960
Ala Gln Glu Thr Gly Asp Ile Arg Ser Ala Ile Thr Thr Leu Gln Phe
305                 310                 315                 320
tgg gcg aca tca agt gga agt ttg ccg att tca acc cga gaa tcc acc      1008
Trp Ala Thr Ser Ser Gly Ser Leu Pro Ile Ser Thr Arg Glu Ser Thr
                325                 330                 335
ata tca tac ttt cat gcc att ggg aag gtg ata cat ggt tcc cat agc      1056
Ile Ser Tyr Phe His Ala Ile Gly Lys Val Ile His Gly Ser His Ser
            340                 345                 350
acg aat aac gat aac gaa atg att aat aac ctc ttc gaa aat tcg aac      1104
Thr Asn Asn Asp Asn Glu Met Ile Asn Asn Leu Phe Glu Asn Ser Asn
        355                 360                 365
aat ttg tta tcg aaa gag gat ttc aaa tta gga ata tta gag aac tat      1152
Asn Leu Leu Ser Lys Glu Asp Phe Lys Leu Gly Ile Leu Glu Asn Tyr
    370                 375                 380
aac aca ttt aat aaa ggc gaa ttc agc att tct gat gca tca tca att      1200

Asn Thr Phe Asn Lys Gly Glu Phe Ser Ile Ser Asp Ala Ser Ser Ile
385                 390                 395                 400
gtg gat tgc ctg agc gag tgt gat aat atg aat ggt cta cca gaa tcc      1248
Val Asp Cys Leu Ser Glu Cys Asp Asn Met Asn Gly Leu Pro Glu Ser
                405                 410                 415
aat gag tat ggt tta cga gaa gtg cgc aaa acc ttt cgt aac atc agt      1296
Asn Glu Tyr Gly Leu Arg Glu Val Arg Lys Thr Phe Arg Asn Ile Ser
            420                 425                 430
aaa caa ggc cat aat cat gga acg gtt tat ttt cca aga gaa tgg aaa      1344
Lys Gln Gly His Asn His Gly Thr Val Tyr Phe Pro Arg Glu Trp Lys
        435                 440                 445
gta aga aaa tta caa aat tca ttt aaa gtt caa gct gaa gat tgg tta      1392
Val Arg Lys Leu Gln Asn Ser Phe Lys Val Gln Ala Glu Asp Trp Leu
    450                 455                 460
aat gtt agt ctt tat aag tac aac gcg gta cat tct ttc agg aat ata      1440
Asn Val Ser Leu Tyr Lys Tyr Asn Ala Val His Ser Phe Arg Asn Ile
465                 470                 475                 480
act cta gaa ttt ggc tac tac gca cct cta att aga aag tgt cag agt      1488
Thr Leu Glu Phe Gly Tyr Tyr Ala Pro Leu Ile Arg Lys Cys Gln Ser
                485                 490                 495
tat aaa aaa aaa tac att ctc tat tat ttg aag aat ctt ccg agt ggc      1536
Tyr Lys Lys Lys Tyr Ile Leu Tyr Tyr Leu Lys Asn Leu Pro Ser Gly
            500                 505                 510
tcc tcg ggg ccc aaa caa acc atg gac aaa ttt agt gat ata atg aaa      1584
Ser Ser Gly Pro Lys Gln Thr Met Asp Lys Phe Ser Asp Ile Met Lys
        515                 520                 525
gtt gag aac gga atc gac gtt gtg gat cgg ata ggc ggg cct atc gaa      1632
Val Glu Asn Gly Ile Asp Val Val Asp Arg Ile Gly Gly Pro Ile Glu
    530                 535                 540
gca cta tct gtg gag gat gga cta gca cca ttg atg gat aat gat agc      1680
Ala Leu Ser Val Glu Asp Gly Leu Ala Pro Leu Met Asp Asn Asp Ser
545                 550                 555                 560
aat aat tgt gac cat tta gag gat caa aaa aag gaa agg gac aga agg      1728
Asn Asn Cys Asp His Leu Glu Asp Gln Lys Lys Glu Arg Asp Arg Arg
                565                 570                 575
ctt cgc atg ttg att gac caa tat gaa aga aat gtg atg atg gct aac      1776
Leu Arg Met Leu Ile Asp Gln Tyr Glu Arg Asn Val Met Met Ala Asn
            580                 585                 590
gac gat ctt gaa gac gaa gaa act tct ttt aat gat gac cct att gtc      1824
Asp Asp Leu Glu Asp Glu Glu Thr Ser Phe Asn Asp Asp Pro Ile Val
        595                 600                 605
gat agc gat agc gat aac agc aat aat att ggc aat gaa aca ttt ggt      1872
Asp Ser Asp Ser Asp Asn Ser Asn Asn Ile Gly Asn Glu Thr Phe Gly
    610                 615                 620
aga agc gac gaa gac gag tct cta tgt gaa att ctg tcc cag aga cag      1920
Arg Ser Asp Glu Asp Glu Ser Leu Cys Glu Ile Leu Ser Gln Arg Gln
625                 630                 635                 640
ccg cgt aaa gcg cca gtt atc agt gag tcc ctt tca gat tca gat ctg      1968
Pro Arg Lys Ala Pro Val Ile Ser Glu Ser Leu Ser Asp Ser Asp Leu
                645                 650                 655
gaa ata ctc taa                                                      1980
Glu Ile Leu

<210> 14
<211> 659
<212> PRT
<213> Saccharomyces cerevisiae

<400> 14
   Met Asp Ser Thr Asn Leu Asn Lys Arg Pro Leu Leu Gln Tyr Ser Leu
   1               5                   10                  15
   Ser Ser Leu Gly Ser Gln Ile Thr Lys Trp Ser Ser Ser Arg Pro Thr
               20                  25                  30
   Ser Pro Val Arg Lys Ala Arg Ser Thr Glu Asn Asp Phe Leu Ser Lys
           35                  40                  45
   Gln Asp Thr Ser Ser Ile Leu Pro Ser Ile Asn Asp Asp Gly Gly Glu
       50                  55                  60
   Gln Trp Tyr Glu Lys Phe Lys Pro Asn Cys Leu Glu Gln Val Ala Ile
   65                  70                  75                  80
   His Lys Arg Lys Leu Lys Asp Val Gln Glu Ala Leu Asp Ala Met Phe
                   85                  90                  95
   Leu Pro Asn Ala Lys His Arg Ile Leu Leu Leu Ser Gly Pro Ser Gly
   100 105 110
   Cys Ser Lys Ser Thr Val Ile Lys Glu Leu Ser Lys Ile Leu Val Pro
   115 120 125
   Lys Tyr Arg Gln Asn Ser Asn Gly Thr Ser Phe Arg Ser Thr Pro Asn
   130 135 140
   Glu His Lys Val Thr Glu Phe Arg Gly Asp Cys Ile Val Asn Asp Leu
   145 150 155 160
   Pro Gln Met Glu Ser Phe Ser Glu Phe Leu Lys Gly Ala Arg Tyr Leu
   165 170 175
   Val Met Ser Asn Leu Ser Leu Ile Leu Ile Glu Asp Leu Pro Asn Val
   180 185 190
   Phe His Ile Asp Thr Arg Arg Arg Phe Gln Gln Leu Ile Leu Gln Trp
   195 200 205
   Leu Tyr Ser Ser Glu Pro Leu Leu Pro Pro Leu Val Ile Cys Ile Thr
   210 215 220
   Glu Cys Glu Ile Pro Glu Asn Asp Asn Asn Tyr Arg Lys Phe Gly Ile
   225 230 235 240
   Asp Tyr Thr Phe Ser Ala Glu Thr Ile Met Asn Lys Glu Ile Leu Met
   245 250 255
   His Pro Arg Leu Lys Arg Ile Lys Phe Asn Pro Ile Asn Ser Thr Leu
   260 265 270
   Leu Lys Lys His Leu Lys Phe Ile Cys Val Gln Asn Met Lys Met Leu
   275 280 285
   Lys Glu Lys Asn Lys Trp Asn Lys Arg Gln Glu Val Ile Asp Tyr Ile
   290 295 300
   Ala Gln Glu Thr Gly Asp Ile Arg Ser Ala Ile Thr Thr Leu Gln Phe
   305 310 315 320
   Trp Ala Thr Ser Ser Gly Ser Leu Pro Ile Ser Thr Arg Glu Ser Thr
   325 330 335
   Ile Ser Tyr Phe His Ala Ile Gly Lys Val Ile His Gly Ser His Ser
   340 345 350
   Thr Asn Asn Asp Asn Glu Met Ile Asn Asn Leu Phe Glu Asn Ser Asn
   355 360 365
   Asn Leu Leu Ser Lys Glu Asp Phe Lys Leu Gly Ile Leu Glu Asn Tyr
   370 375 380
   Asn Thr Phe Asn Lys Gly Glu Phe Ser Ile Ser Asp Ala Ser Ser Ile
   385 390 395 400
   Val Asp Cys Leu Ser Glu Cys Asp Asn Met Asn Gly Leu Pro Glu Ser
   405 410 415
   Asn Glu Tyr Gly Leu Arg Glu Val Arg Lys Thr Phe Arg Asn Ile Ser
   420 425 430
   Lys Gln Gly His Asn His Gly Thr Val Tyr Phe Pro Arg Glu Trp Lys
   435 440 445
   Val Arg Lys Leu Gln Asn Ser Phe Lys Val Gln Ala Glu Asp Trp Leu
   450 455 460
   Asn Val Ser Leu Tyr Lys Tyr Asn Ala Val His Ser Phe Arg Asn Ile
   465 470 475 480
   Thr Leu Glu Phe Gly Tyr Tyr Ala Pro Leu Ile Arg Lys Cys Gln Ser
   485 490 495
   Tyr Lys Lys Lys Tyr Ile Leu Tyr Tyr Leu Lys Asn Leu Pro Ser Gly
   500 505 510
   Ser Ser Gly Pro Lys Gln Thr Met Asp Lys Phe Ser Asp Ile Met Lys
   515 520 525
   Val Glu Asn Gly Ile Asp Val Val Asp Arg Ile Gly Gly Pro Ile Glu
   530 535 540
   Ala Leu Ser Val Glu Asp Gly Leu Ala Pro Leu Met Asp Asn Asp Ser
   545 550 555 560
   Asn Asn Cys Asp His Leu Glu Asp Gln Lys Lys Glu Arg Asp Arg Arg
   565 570 575
   Leu Arg Met Leu Ile Asp Gln Tyr Glu Arg Asn Val Met Met Ala Asn
   580 585 590
   Asp Asp Leu Glu Asp Glu Glu Thr Ser Phe Asn Asp Asp Pro Ile Val
   595 600 605
   Asp Ser Asp Ser Asp Asn Ser Asn Asn Ile Gly Asn Glu Thr Phe Gly
   610 615 620
   Arg Ser Asp Glu Asp Glu Ser Leu Cys Glu Ile Leu Ser Gln Arg Gln
   625 630 635 640
   Pro Arg Lys Ala Pro Val Ile Ser Glu Ser Leu Ser Asp Ser Asp Leu
   645 650 655
   Glu Ile Leu

<210> 15
<211> 26
<212> DNA
<213> Primer

<400> 15
atggatagta cgaatttgaa caaacg                                          26

<210> 16
<211> 26
<212> DNA
<213> Primer

<400> 16
ttagagtatt tccagatctg aatctg                                          26

<210> 17
<211> 1581
<212> DNA
<213> Saccharomyces cerevisiae

<220>
<221> CDS
<222> (1)..(1581)

<400> 17
atg aca att tct aat ttg tta aag cag aga gtt agg tat gct ccc tat       48
Met Thr Ile Ser Asn Leu Leu Lys Gln Arg Val Arg Tyr Ala Pro Tyr
1               5                   10                  15
ctg aaa aaa gtt aag gaa gct cac gag ctt att cca ttg ttc aag aat       96
Leu Lys Lys Val Lys Glu Ala His Glu Leu Ile Pro Leu Phe Lys Asn
            20                  25                  30
ggt cag tac ctt ggg tgg tcc ggt ttt aca gga gtg ggt act ccc aag      144
Gly Gln Tyr Leu Gly Trp Ser Gly Phe Thr Gly Val Gly Thr Pro Lys
        35                  40                  45
gca gtg ccg gag gca ctg ata gat cac gtg gag aag aac aat tta caa      192
Ala Val Pro Glu Ala Leu Ile Asp His Val Glu Lys Asn Asn Leu Gln
    50                  55                  60
ggg aag ttg aga ttc aac ctt ttt gtt gga gct tct gct ggt cca gag      240
Gly Lys Leu Arg Phe Asn Leu Phe Val Gly Ala Ser Ala Gly Pro Glu
65                  70                  75                  80
gaa aac cgt tgg gct gaa cac gac atg atc att aag aga gcc cct cat      288
Glu Asn Arg Trp Ala Glu His Asp Met Ile Ile Lys Arg Ala Pro His
                85                  90                  95
caa gta ggg aaa ccc att gca aag gca att aac cag ggt aga att gag      336
Gln Val Gly Lys Pro Ile Ala Lys Ala Ile Asn Gln Gly Arg Ile Glu
            100                 105                 110
ttc ttt gat aaa cat ctg tcc atg ttc cct cag gat ctg aca tac ggg      384
Phe Phe Asp Lys His Leu Ser Met Phe Pro Gln Asp Leu Thr Tyr Gly
        115                 120                 125
ttc tac acc agg gaa aga aaa gac aac aaa atc ctt gat tat act ata      432
Phe Tyr Thr Arg Glu Arg Lys Asp Asn Lys Ile Leu Asp Tyr Thr Ile
    130                 135                 140
atc gag gca acg gcc att aaa gag gac ggg tct atc gtc cca ggt ccc      480
Ile Glu Ala Thr Ala Ile Lys Glu Asp Gly Ser Ile Val Pro Gly Pro
145                 150                 155                 160
tct gtc ggt ggt tct cca gaa ttc att aca gtc agt gat aaa gtg att      528
Ser Val Gly Gly Ser Pro Glu Phe Ile Thr Val Ser Asp Lys Val Ile
                165                 170                 175
att gag gtt aac acg gct acg cct tcg ttc gag ggt att cac gat ata      576
Ile Glu Val Asn Thr Ala Thr Pro Ser Phe Glu Gly Ile His Asp Ile
            180                 185                 190
gac atg ccc gtg aac cca cct ttc agg aaa cca tac cca tat ctg aaa      624
Asp Met Pro Val Asn Pro Pro Phe Arg Lys Pro Tyr Pro Tyr Leu Lys
        195                 200                 205
gtg gac gac aag tgt ggt gtt gac tcc atc ccg gtt gat cct gaa aag      672
Val Asp Asp Lys Cys Gly Val Asp Ser Ile Pro Val Asp Pro Glu Lys
    210                 215                 220
gtt gtt gcg att gtg gag tcc acc atg agg gac cag gtc cca cca aat      720
Val Val Ala Ile Val Glu Ser Thr Met Arg Asp Gln Val Pro Pro Asn
225                 230                 235                 240

acg ccc tct gac gac atg tcc agg gct att gca ggt cat ttg gtc gag     768
Thr Pro Ser Asp Asp Met Ser Arg Ala Ile Ala Gly His Leu Val Glu
            245                 250                 255
ttt ttc aga aac gag gta aaa cat ggt agg cta cct gaa aac ctg ctg     816
Phe Phe Arg Asn Glu Val Lys His Gly Arg Leu Pro Glu Asn Leu Leu
        260                 265                 270
cct tta caa agt ggt ata ggt aac att gct aac gct gtc att gaa ggg     864
Pro Leu Gln Ser Gly Ile Gly Asn Ile Ala Asn Ala Val Ile Glu Gly
        275                 280                 285
ctt gct ggc gcc caa ttc aag cac ttg act gta tgg acg gaa gtg ctg     912
Leu Ala Gly Ala Gln Phe Lys His Leu Thr Val Trp Thr Glu Val Leu
    290                 295                 300
cag gac tcg ttc ttg gat ctt ttc gag aac gga tct ttg gac tac gcc     960
Gln Asp Ser Phe Leu Asp Leu Phe Glu Asn Gly Ser Leu Asp Tyr Ala
305                 310                 315                 320
act gct act tcc gtg aga ttg act gaa aag ggt ttc gac aga gcc ttt     1008
Thr Ala Thr Ser Val Arg Leu Thr Glu Lys Gly Phe Asp Arg Ala Phe
                325                 330                 335
gca aac tgg gaa aat ttc aaa cac aga ttg tgt ttg aga tct caa gtt     1056
Ala Asn Trp Glu Asn Phe Lys His Arg Leu Cys Leu Arg Ser Gln Val
            340                 345                 350
gtc tcg aac aat ccg gaa atg atc cgt aga ttg ggt gtc atc gcc atg     1104
Val Ser Asn Asn Pro Glu Met Ile Arg Arg Leu Gly Val Ile Ala Met
        355                 360                 365
aat acc cca gta gaa gtt gac att tac gcg cac gcc aat tct aca aat     1152
Asn Thr Pro Val Glu Val Asp Ile Tyr Ala His Ala Asn Ser Thr Asn
    370                 375                 380
gtg aat ggt tcc cgt atg ttg aac ggg ttg ggt gga tct gct gat ttc     1200
Val Asn Gly Ser Arg Met Leu Asn Gly Leu Gly Gly Ser Ala Asp Phe
385                 390                 395                 400
ttg aga aat gca aag ttg tcc atc atg cat gcc ccc tct gca aga cca     1248
Leu Arg Asn Ala Lys Leu Ser Ile Met His Ala Pro Ser Ala Arg Pro
                405                 410                 415
act aaa gta gac cct acc ggt atc tct acc att gtt cct atg gcc tct     1296
Thr Lys Val Asp Pro Thr Gly Ile Ser Thr Ile Val Pro Met Ala Ser
            420                 425                 430
cat gta gat caa act gag cat gac ctg gac atc ttg gtc act gac caa     1344
His Val Asp Gln Thr Glu His Asp Leu Asp Ile Leu Val Thr Asp Gln
        435                 440                 445
ggt ttg gcg gat cta aga ggt cta tcg cct aag gaa aga gcc cgt gaa     1392
Gly Leu Ala Asp Leu Arg Gly Leu Ser Pro Lys Glu Arg Ala Arg Glu
    450                 455                 460
atc atc aac aag tgt gct cat ccc gat tat caa gct ttg ttg acc gat     1440
Ile Ile Asn Lys Cys Ala His Pro Asp Tyr Gln Ala Leu Leu Thr Asp
465                 470                 475                 480
tac ttg gac aga gca gag cat tac gct aaa aag cac aat tgc ttg cat     1488
Tyr Leu Asp Arg Ala Glu His Tyr Ala Lys Lys His Asn Cys Leu His
                485                 490                 495
gaa cca cac atg cta aag aat gct ttc aag ttc cac acc aac tta gct     1536
Glu Pro His Met Leu Lys Asn Ala Phe Lys Phe His Thr Asn Leu Ala
            500                 505                 510
gaa aag ggt aca atg aag gtc gac agc tgg gaa cca gtt gac tag         1581
Glu Lys Gly Thr Met Lys Val Asp Ser Trp Glu Pro Val Asp
        515                 520                 525

<210> 18
<211> 526
<212> PRT
<213> Saccharomyces cerevisiae

<400> 18
Met Thr Ile Ser Asn Leu Leu Lys Gln Arg Val Arg Tyr Ala Pro Tyr
1               5                   10                  15
Leu Lys Lys Val Lys Glu Ala His Glu Leu Ile Pro Leu Phe Lys Asn
            20                  25                  30
Gly Gln Tyr Leu Gly Trp Ser Gly Phe Thr Gly Val Gly Thr Pro Lys
        35                  40                  45
Ala Val Pro Glu Ala Leu Ile Asp His Val Glu Lys Asn Asn Leu Gln
    50                  55                  60
Gly Lys Leu Arg Phe Asn Leu Phe Val Gly Ala Ser Ala Gly Pro Glu
65                  70                  75                  80
Glu Asn Arg Trp Ala Glu His Asp Met Ile Ile Lys Arg Ala Pro His

85                  90                  95
Gln Val Gly Lys Pro Ile Ala Lys Ala Ile Asn Gln Gly Arg Ile Glu
            100                 105                 110
Phe Phe Asp Lys His Leu Ser Met Phe Pro Gln Asp Leu Thr Tyr Gly
        115                 120                 125
Phe Tyr Thr Arg Glu Arg Lys Asp Asn Lys Ile Leu Asp Tyr Thr Ile
    130                 135                 140
Ile Glu Ala Thr Ala Ile Lys Glu Asp Gly Ser Ile Val Pro Gly Pro
145                 150                 155                 160
Ser Val Gly Gly Ser Pro Glu Phe Ile Thr Val Ser Asp Lys Val Ile
                165                 170                 175
Ile Glu Val Asn Thr Ala Thr Pro Ser Phe Glu Gly Ile His Asp Ile
            180                 185                 190
Asp Met Pro Val Asn Pro Pro Phe Arg Lys Pro Tyr Pro Tyr Leu Lys
        195                 200                 205
Val Asp Asp Lys Cys Gly Val Asp Ser Ile Pro Val Asp Pro Glu Lys
    210                 215                 220
Val Val Ala Ile Val Glu Ser Thr Met Arg Asp Gln Val Pro Pro Asn
225                 230                 235                 240
Thr Pro Ser Asp Asp Met Ser Arg Ala Ile Ala Gly His Leu Val Glu
                245                 250                 255
Phe Phe Arg Asn Glu Val Lys His Gly Arg Leu Pro Glu Asn Leu Leu
            260                 265                 270
Pro Leu Gln Ser Gly Ile Gly Asn Ile Ala Asn Ala Val Ile Glu Gly
        275                 280                 285
Leu Ala Gly Ala Gln Phe Lys His Leu Thr Val Trp Thr Glu Val Leu
    290                 295                 300
Gln Asp Ser Phe Leu Asp Leu Phe Glu Asn Gly Ser Leu Asp Tyr Ala
305                 310                 315                 320
Thr Ala Thr Ser Val Arg Leu Thr Glu Lys Gly Phe Asp Arg Ala Phe
                325                 330                 335
Ala Asn Trp Glu Asn Phe Lys His Arg Leu Cys Leu Arg Ser Gln Val
            340                 345                 350
Val Ser Asn Asn Pro Glu Met Ile Arg Arg Leu Gly Val Ile Ala Met
        355                 360                 365
Asn Thr Pro Val Glu Val Asp Ile Tyr Ala His Ala Asn Ser Thr Asn
    370                 375                 380
Val Asn Gly Ser Arg Met Leu Asn Gly Leu Gly Gly Ser Ala Asp Phe
385                 390                 395                 400
Leu Arg Asn Ala Lys Leu Ser Ile Met His Ala Pro Ser Ala Arg Pro
                405                 410                 415
Thr Lys Val Asp Pro Thr Gly Ile Ser Thr Ile Val Pro Met Ala Ser
            420                 425                 430
His Val Asp Gln Thr Glu His Asp Leu Asp Ile Leu Val Thr Asp Gln
        435                 440                 445
Gly Leu Ala Asp Leu Arg Gly Leu Ser Pro Lys Glu Arg Ala Arg Glu
    450                 455                 460
Ile Ile Asn Lys Cys Ala His Pro Asp Tyr Gln Ala Leu Leu Thr Asp
465                 470                 475                 480
Tyr Leu Asp Arg Ala Glu His Tyr Ala Lys Lys His Asn Cys Leu His
                485                 490                 495
Glu Pro His Met Leu Lys Asn Ala Phe Lys Phe His Thr Asn Leu Ala
            500                 505                 510
Glu Lys Gly Thr Met Lys Val Asp Ser Trp Glu Pro Val Asp
        515                 520                 525

<210> 19
<211> 1578
<212> DNA
<213> Neurospora crassa

<220>
<221> CDS
<222> (1)..(1578)

<400> 19
atg gcg tcg ccc att gcc tcg gcg gcc ctc agg gcg cgt caa ggc cct       48
Met Ala Ser Pro Ile Ala Ser Ala Ala Leu Arg Ala Arg Gln Gly Pro
1               5                   10                  15
tcc atg ctc aag aag ctc tgc aac ccc gag gac atg ctc cag cac ttc       96
Ser Met Leu Lys Lys Leu Cys Asn Pro Glu Asp Met Leu Gln His Phe
            20                  25                  30

ccc aat ggc gca tac atc ggc tgg tcc ggt ttc acc gcg tcg gct acc 144
Pro Asn Gly Ala Tyr Ile Gly Trp Ser Gly Phe Thr Ala Ser Ala Thr
        35                  40                  45
cca aga agg tcc tac tat ctc gct gac cac gtc gag aag aac gcc tcc 192
Pro Arg Arg Ser Tyr Tyr Leu Ala Asp His Val Glu Lys Asn Ala Ser
    50                  55                  60
agg cca gct caa gta cag cct ctt cgt cgg tgc ctc cgc cgg cgc cga 240
Arg Pro Ala Gln Val Gln Pro Leu Arg Arg Cys Leu Arg Arg Arg Arg
65                  70                  75                  80
gac cga gaa cgc tgg gct gct ctc gac atg atc gcc cat gat cgc cgt 288
Asp Arg Glu Arg Trp Ala Ala Leu Asp Met Ile Ala His Asp Arg Arg
                85                  90                  95
cgc gct cct cac cag gtc ggc aag aac att gcc aag ggc atc aac gag 336
Arg Ala Pro His Gln Val Gly Lys Asn Ile Ala Lys Gly Ile Asn Glu
            100                 105                 110
ggc cgc atc aac ttc ttc gac aag cac ttg tcc atg ttc ccc gtt gac 384
Gly Arg Ile Asn Phe Phe Asp Lys His Leu Ser Met Phe Pro Val Asp
        115                 120                 125
ctt gtc tac ggc tac tac acc aag gac cgc cag aac aag aac ctc gat 432
Leu Val Tyr Gly Tyr Tyr Thr Lys Asp Arg Gln Asn Lys Asn Leu Asp
    130                 135                 140
gtc gtt tgc gtt gaa gct acc gag atc aag gag gac ggt tcc atc gtc 480
Val Val Cys Val Glu Ala Thr Glu Ile Lys Glu Asp Gly Ser Ile Val
145                 150                 155                 160
ctc ggt gcc tcg gtc ggt gcc acc ccc gag ctc atc cag atg gcc gac 528
Leu Gly Ala Ser Val Gly Ala Thr Pro Glu Leu Ile Gln Met Ala Asp
                165                 170                 175
aag gtc atc att gag gtc aac acg gcc atc ccc agc ttc gac ggt ctc 576
Lys Val Ile Ile Glu Val Asn Thr Ala Ile Pro Ser Phe Asp Gly Leu
            180                 185                 190
cac gac atc acc ttc tcc gac ctc cct ccc aac cgc aag ccc tac ctc 624
His Asp Ile Thr Phe Ser Asp Leu Pro Pro Asn Arg Lys Pro Tyr Leu
        195                 200                 205
atc cag cag tgc agg gac cgc atc ggt act act tcg gtc ccc gtc gac 672
Ile Gln Gln Cys Arg Asp Arg Ile Gly Thr Thr Ser Val Pro Val Asp
    210                 215                 220
ccc gag aag gtt gtc ggt atc atc gag tgc acc acc ccc gat cag acc 720
Pro Glu Lys Val Val Gly Ile Ile Glu Cys Thr Thr Pro Asp Gln Thr
225                 230                 235                 240
ctc ccc aac tcc ccc gcc gac gaa acg gct acg gcc att gcc ggc cac 768
Leu Pro Asn Ser Pro Ala Asp Glu Thr Ala Thr Ala Ile Ala Gly His
                245                 250                 255
ctg att gag ttc ttc gag cac gag gtc gcc cac ggc cgt ctg ccc aag 816
Leu Ile Glu Phe Phe Glu His Glu Val Ala His Gly Arg Leu Pro Lys
            260                 265                 270
aac ctt ctc cct ctg cag tcc ggt atc ggc aac atc gcc aac gcc gtc 864
Asn Leu Leu Pro Leu Gln Ser Gly Ile Gly Asn Ile Ala Asn Ala Val
        275                 280                 285
att ggt ggt ctc gag acc tcc aac ttc aag aac ctc aac gtc tgg act 912
Ile Gly Gly Leu Glu Thr Ser Asn Phe Lys Asn Leu Asn Val Trp Thr
    290                 295                 300
gag gtt atc cag gat acc ttc ctc gac ctc ttc gac tcc ggc aag ctt 960
Glu Val Ile Gln Asp Thr Phe Leu Asp Leu Phe Asp Ser Gly Lys Leu
305                 310                 315                 320
gac ttt gct acc gcc acc tcg atc cgc ttc tcg ccc acc ggc ttc gag 1008
Asp Phe Ala Thr Ala Thr Ser Ile Arg Phe Ser Pro Thr Gly Phe Glu
                325                 330                 335
cgc ttc tac aag aac tgg gac aac tac tat gac aag ctc ctc ctc cgc 1056
Arg Phe Tyr Lys Asn Trp Asp Asn Tyr Tyr Asp Lys Leu Leu Leu Arg
            340                 345                 350
tct cag tcc gtc tcc aac gct ccc gag atc atc cgc cgt ctc ggt gtt 1104
Ser Gln Ser Val Ser Asn Ala Pro Glu Ile Ile Arg Arg Leu Gly Val
        355                 360                 365
atc ggc atg aac acc ccc gtc gaa gtc gac atc tac gcc cac gcc aac 1152
Ile Gly Met Asn Thr Pro Val Glu Val Asp Ile Tyr Ala His Ala Asn
    370                 375                 380
tcc acc aac gtc atg ggc tcc cgc atg ctc aac ggt ctc ggt ggt tcc 1200
Ser Thr Asn Val Met Gly Ser Arg Met Leu Asn Gly Leu Gly Gly Ser
385                 390                 395                 400
gcc gat ttc ctc cgc aac tcc aag tac tcc atc atg cac acc ccc tcc 1248
Ala Asp Phe Leu Arg Asn Ser Lys Tyr Ser Ile Met His Thr Pro Ser
                405                 410                 415

acc cgt ccc tcc aag acc gac gcc cac ggt gtt tcg tgc att gtc ccc 1296
Thr Arg Pro Ser Lys Thr Asp Ala His Gly Val Ser Cys Ile Val Pro
            420                 425                 430
atg tgc acc cac gtc gac cag acc gag cac gat ctc gac gtc atc gtt 1344
Met Cys Thr His Val Asp Gln Thr Glu His Asp Leu Asp Val Ile Val
        435                 440                 445
acc gag aac ggt ctc gcc gat gtc cgc ggc ctc agc ccc cgc gag cgt 1392
Thr Glu Asn Gly Leu Ala Asp Val Arg Gly Leu Ser Pro Arg Glu Arg
    450                 455                 460
gcc cgc gtc atc att gac aag tgc gcc cac gac gtc tac aag ccc atc 1440
Ala Arg Val Ile Ile Asp Lys Cys Ala His Asp Val Tyr Lys Pro Ile
465                 470                 475                 480
ctc aag gcc tac ttt gag aag gcc gag ttc gag tgc ctt cgc aag ggt 1488
Leu Lys Ala Tyr Phe Glu Lys Ala Glu Phe Glu Cys Leu Arg Lys Gly
                485                 490                 495
atg ggc cac gag ccc cac ctt ctc ttc aac tct ttc gac atg cac aag 1536
Met Gly His Glu Pro His Leu Leu Phe Asn Ser Phe Asp Met His Lys
            500                 505                 510
gct ctt gtc gag gag ggc tcc atg gcc aag gtc aag ttc taa 1578
Ala Leu Val Glu Glu Gly Ser Met Ala Lys Val Lys Phe
        515                 520                 525

<210> 20
<211> 525
<212> PRT
<213> Neurospora crassa

<400> 20
Met Ala Ser Pro Ile Ala Ser Ala Ala Leu Arg Ala Arg Gln Gly Pro
1               5                   10                  15
Ser Met Leu Lys Lys Leu Cys Asn Pro Glu Asp Met Leu Gln His Phe
            20                  25                  30
Pro Asn Gly Ala Tyr Ile Gly Trp Ser Gly Phe Thr Ala Ser Ala Thr
        35                  40                  45
Pro Arg Arg Ser Tyr Tyr Leu Ala Asp His Val Glu Lys Asn Ala Ser
    50                  55                  60
Arg Pro Ala Gln Val Gln Pro Leu Arg Arg Cys Leu Arg Arg Arg Arg
65                  70                  75                  80
Asp Arg Glu Arg Trp Ala Ala Leu Asp Met Ile Ala His Asp Arg Arg
                85                  90                  95
Arg Ala Pro His Gln Val Gly Lys Asn Ile Ala Lys Gly Ile Asn Glu
            100                 105                 110
Gly Arg Ile Asn Phe Phe Asp Lys His Leu Ser Met Phe Pro Val Asp
        115                 120                 125
Leu Val Tyr Gly Tyr Tyr Thr Lys Asp Arg Gln Asn Lys Asn Leu Asp
    130                 135                 140
Val Val Cys Val Glu Ala Thr Glu Ile Lys Glu Asp Gly Ser Ile Val
145                 150                 155                 160
Leu Gly Ala Ser Val Gly Ala Thr Pro Glu Leu Ile Gln Met Ala Asp
                165                 170                 175
Lys Val Ile Ile Glu Val Asn Thr Ala Ile Pro Ser Phe Asp Gly Leu
            180                 185                 190
His Asp Ile Thr Phe Ser Asp Leu Pro Pro Asn Arg Lys Pro Tyr Leu
        195                 200                 205
Ile Gln Gln Cys Arg Asp Arg Ile Gly Thr Thr Ser Val Pro Val Asp
    210                 215                 220
Pro Glu Lys Val Val Gly Ile Ile Glu Cys Thr Thr Pro Asp Gln Thr
225                 230                 235                 240
Leu Pro Asn Ser Pro Ala Asp Glu Thr Ala Thr Ala Ile Ala Gly His
                245                 250                 255
Leu Ile Glu Phe Phe Glu His Glu Val Ala His Gly Arg Leu Pro Lys
            260                 265                 270
Asn Leu Leu Pro Leu Gln Ser Gly Ile Gly Asn Ile Ala Asn Ala Val
        275                 280                 285
Ile Gly Gly Leu Glu Thr Ser Asn Phe Lys Asn Leu Asn Val Trp Thr
    290                 295                 300
Glu Val Ile Gln Asp Thr Phe Leu Asp Leu Phe Asp Ser Gly Lys Leu
305                 310                 315                 320
Asp Phe Ala Thr Ala Thr Ser Ile Arg Phe Ser Pro Thr Gly Phe Glu
                325                 330                 335
Arg Phe Tyr Lys Asn Trp Asp Asn Tyr Tyr Asp Lys Leu Leu Leu Arg

                340                 345                 350
Ser Gln Ser Val Ser Asn Ala Pro Glu Ile Ile Arg Arg Leu Gly Val
        355                 360                 365
Ile Gly Met Asn Thr Pro Val Glu Val Asp Ile Tyr Ala His Ala Asn
    370                 375                 380
Ser Thr Asn Val Met Gly Ser Arg Met Leu Asn Gly Leu Gly Gly Ser
385                 390                 395                 400
Ala Asp Phe Leu Arg Asn Ser Lys Tyr Ser Ile Met His Thr Pro Ser
                405                 410                 415
Thr Arg Pro Ser Lys Thr Asp Ala His Gly Val Ser Cys Ile Val Pro
            420                 425                 430
Met Cys Thr His Val Asp Gln Thr Glu His Asp Leu Asp Val Ile Val
        435                 440                 445
Thr Glu Asn Gly Leu Ala Asp Val Arg Gly Leu Ser Pro Arg Glu Arg
    450                 455                 460
Ala Arg Val Ile Ile Asp Lys Cys Ala His Asp Val Tyr Lys Pro Ile
465                 470                 475                 480
Leu Lys Ala Tyr Phe Glu Lys Ala Glu Phe Glu Cys Leu Arg Lys Gly
                485                 490                 495
Met Gly His Glu Pro His Leu Leu Phe Asn Ser Phe Asp Met His Lys
            500                 505                 510
Ala Leu Val Glu Glu Gly Ser Met Ala Lys Val Lys Phe
        515                 520                 525

<210> 21
<211> 1491
<212> DNA
<213> Brucella melitensis

<220>
<221> CDS
<222> (1)..(1491)

<400> 21
atg ttg gaa gca cgt atc cgt aac agt tca ctg cga aac aag gtt atc      48
Met Leu Glu Ala Arg Ile Arg Asn Ser Ser Leu Arg Asn Lys Val Ile
1               5                   10                  15
act gcg gag caa gcg gca agc ttt atc aag gat ggc atg att gtt ggc      96
Thr Ala Glu Gln Ala Ala Ser Phe Ile Lys Asp Gly Met Ile Val Gly
            20                  25                  30
atg agc ggc ttt acg cgc gcg ggc gat gcc aag gcc gtt ccg gtc gcc      144
Met Ser Gly Phe Thr Arg Ala Gly Asp Ala Lys Ala Val Pro Val Ala
        35                  40                  45
atg gcc gcg cgg gcg gca acc gat cca ttc cag atc acc ctg att aca      192
Met Ala Ala Arg Ala Ala Thr Asp Pro Phe Gln Ile Thr Leu Ile Thr
    50                  55                  60
ggg gcc tcc ctc gga cat gac gtc gat aaa ctg ttg acc gaa gcg cat      240
Gly Ala Ser Leu Gly His Asp Val Asp Lys Leu Leu Thr Glu Ala His
65                  70                  75                  80
att ctt tct cgc cgt atg ccg ttt cag gcc gac cgc aca ttg cgc agc      288
Ile Leu Ser Arg Arg Met Pro Phe Gln Ala Asp Arg Thr Leu Arg Ser
                85                  90                  95
gcg atc aac cgc ggc gaa gtc atg ttt atc gac cag cat ctt tcc gaa      336
Ala Ile Asn Arg Gly Glu Val Met Phe Ile Asp Gln His Leu Ser Glu
            100                 105                 110
aca gtc gag caa ctt cgt tcc aac cag atc ggc ccc atc gac tgt gcc      384
Thr Val Glu Gln Leu Arg Ser Asn Gln Ile Gly Pro Ile Asp Cys Ala
        115                 120                 125
gtg gta gag gca ttg gcg att acc gaa agc ggc gga atc atc ccg acg      432
Val Val Glu Ala Leu Ala Ile Thr Glu Ser Gly Gly Ile Ile Pro Thr
    130                 135                 140
act tcg gtc ggc aat tcg gcc agc ctc gcc att ctt gcc gaa aag gtt      480
Thr Ser Val Gly Asn Ser Ala Ser Leu Ala Ile Leu Ala Glu Lys Val
145                 150                 155                 160
atc gtc gag gtc aat ctg aac cag ccc atg gcg ctg gaa ggc ctg cac      528
Ile Val Glu Val Asn Leu Asn Gln Pro Met Ala Leu Glu Gly Leu His
                165                 170                 175
gat att tat ata ccg acg aag cgc cct tcc cgc gat cct atc ccg atc      576
Asp Ile Tyr Ile Pro Thr Lys Arg Pro Ser Arg Asp Pro Ile Pro Ile
            180                 185                 190
atg gct tgc gat agc cgg atc gga ctg ccc tat att ccc atc gcg ccg      624
Met Ala Cys Asp Ser Arg Ile Gly Leu Pro Tyr Ile Pro Ile Ala Pro

195                 200                 205
gaa aaa atc gcc ggt atc gtc atc acg cag gaa aat gac agt gcg tca        672
Glu Lys Ile Ala Gly Ile Val Ile Thr Gln Glu Asn Asp Ser Ala Ser
    210                 215                 220
cct gtc gag ccc gca gat cac gaa acg gtt gcg att gcc ggg cat ctg        720
Pro Val Glu Pro Ala Asp His Glu Thr Val Ala Ile Ala Gly His Leu
225                 230                 235                 240
atc gaa ttc ttc aag ggg gaa gtc gta aag ggt cgt ctg gat ctg gcg        768
Ile Glu Phe Phe Lys Gly Glu Val Val Lys Gly Arg Leu Asp Leu Ala
                245                 250                 255
ctt aat cca ttg cag gcg ggc atc ggc acc att gcc aat gcg gtt ttg        816
Leu Asn Pro Leu Gln Ala Gly Ile Gly Thr Ile Ala Asn Ala Val Leu
            260                 265                 270
acc ggc ttt gca gat agt cct ttc cac aat ctg cgc atg tat agc gag        864
Thr Gly Phe Ala Asp Ser Pro Phe His Asn Leu Arg Met Tyr Ser Glu
        275                 280                 285
gta ttg cag gat agc acg ttc gat ctg ttc gat gcg ggc aag ctg gat        912
Val Leu Gln Asp Ser Thr Phe Asp Leu Phe Asp Ala Gly Lys Leu Asp
    290                 295                 300
ttt gcc tcc ggc tca tcg atc act ctc agc ccc gct tgc ggc gag cgc        960
Phe Ala Ser Gly Ser Ser Ile Thr Leu Ser Pro Ala Cys Gly Glu Arg
305                 310                 315                 320
gtt ttc aat aat ata gac cgc tat cgt gac agg ctt gtc ctg cgc ccg        1008
Val Phe Asn Asn Ile Asp Arg Tyr Arg Asp Arg Leu Val Leu Arg Pro
                325                 330                 335
cag gaa atc agc aac cat ccg gaa gtc atc cgc cgt ctt ggt att atc        1056
Gln Glu Ile Ser Asn His Pro Glu Val Ile Arg Arg Leu Gly Ile Ile
            340                 345                 350
ggc atc aat acc gca ctc gaa ttc gat atc tac ggc aac gtc aac tca        1104
Gly Ile Asn Thr Ala Leu Glu Phe Asp Ile Tyr Gly Asn Val Asn Ser
        355                 360                 365
acc cat gtt gat gga acc aat atg atg aac ggc att ggc ggt tcc ggc        1152
Thr His Val Asp Gly Thr Asn Met Met Asn Gly Ile Gly Gly Ser Gly
    370                 375                 380
gat ttt gcg cgc aac gcc tat att tcg atc ttc gtc agc aag tcg gaa        1200
Asp Phe Ala Arg Asn Ala Tyr Ile Ser Ile Phe Val Ser Lys Ser Glu
385                 390                 395                 400
gcc aag aat ggc gcg atc tcc tcg gtc gtg ccc atg gta aca cat gtg        1248
Ala Lys Asn Gly Ala Ile Ser Ser Val Val Pro Met Val Thr His Val
                405                 410                 415
gac cac acg gaa cat gac gtg gat atc ctc gtg acg gaa cag gga ctt        1296
Asp His Thr Glu His Asp Val Asp Ile Leu Val Thr Glu Gln Gly Leu
            420                 425                 430
gcc gat ttg cgc ggg ctt gca cca cgc gag cga gcg ccc gtt atc att        1344
Ala Asp Leu Arg Gly Leu Ala Pro Arg Glu Arg Ala Pro Val Ile Ile
        435                 440                 445
gaa aat tgc gca cat ccc gat tac cgg gac cag ctc atg gat tat tac        1392
Glu Asn Cys Ala His Pro Asp Tyr Arg Asp Gln Leu Met Asp Tyr Tyr
    450                 455                 460
gag cga gcc tgc aaa cgc ggc ggc cac acg cct cat ctt ctg gaa gag        1440
Glu Arg Ala Cys Lys Arg Gly Gly His Thr Pro His Leu Leu Glu Glu
465                 470                 475                 480
gca ttc agt tgg cac ctg cgc cgt cag gaa aat ggc agt atg cgc aaa        1488
Ala Phe Ser Trp His Leu Arg Arg Gln Glu Asn Gly Ser Met Arg Lys
                485                 490                 495
taa                                                                    1491

<210> 22
<211> 496
<212> PRT
<213> Brucella melitensis

<400> 22
Met Leu Glu Ala Arg Ile Arg Asn Ser Ser Leu Arg Asn Lys Val Ile
1               5                   10                  15
Thr Ala Glu Gln Ala Ala Ser Phe Ile Lys Asp Gly Met Ile Val Gly
            20                  25                  30
Met Ser Gly Phe Thr Arg Ala Gly Asp Ala Lys Ala Val Pro Val Ala
        35                  40                  45

Met Ala Ala Arg Ala Ala Thr Asp Pro Phe Gln Ile Thr Leu Ile Thr
    50                  55                  60
Gly Ala Ser Leu Gly His Asp Val Asp Lys Leu Leu Thr Glu Ala His
65                  70                  75                  80
Ile Leu Ser Arg Arg Met Pro Phe Gln Ala Asp Arg Thr Leu Arg Ser
                85                  90                  95
Ala Ile Asn Arg Gly Glu Val Met Phe Ile Asp Gln His Leu Ser Glu
            100                 105                 110
Thr Val Glu Gln Leu Arg Ser Asn Gln Ile Gly Pro Ile Asp Cys Ala
        115                 120                 125
Val Val Glu Ala Leu Ala Ile Thr Glu Ser Gly Gly Ile Ile Pro Thr
    130                 135                 140
Thr Ser Val Gly Asn Ser Ala Ser Leu Ala Ile Leu Ala Glu Lys Val
145                 150                 155                 160
Ile Val Glu Val Asn Leu Asn Gln Pro Met Ala Leu Glu Gly Leu His
                165                 170                 175
Asp Ile Tyr Ile Pro Thr Lys Arg Pro Ser Arg Asp Pro Ile Pro Ile
            180                 185                 190
Met Ala Cys Asp Ser Arg Ile Gly Leu Pro Tyr Ile Pro Ile Ala Pro
        195                 200                 205
Glu Lys Ile Ala Gly Ile Val Ile Thr Gln Glu Asn Asp Ser Ala Ser
    210                 215                 220
Pro Val Glu Pro Ala Asp His Glu Thr Val Ala Ile Ala Gly His Leu
225                 230                 235                 240
Ile Glu Phe Phe Lys Gly Glu Val Val Lys Gly Arg Leu Asp Leu Ala
                245                 250                 255
Leu Asn Pro Leu Gln Ala Gly Ile Gly Thr Ile Ala Asn Ala Val Leu
            260                 265                 270
Thr Gly Phe Ala Asp Ser Pro Phe His Asn Leu Arg Met Tyr Ser Glu
        275                 280                 285
Val Leu Gln Asp Ser Thr Phe Asp Leu Phe Asp Ala Gly Lys Leu Asp
    290                 295                 300
Phe Ala Ser Gly Ser Ser Ile Thr Leu Ser Pro Ala Cys Gly Glu Arg
305                 310                 315                 320
Val Phe Asn Asn Ile Asp Arg Tyr Arg Asp Arg Leu Val Leu Arg Pro
                325                 330                 335
Gln Glu Ile Ser Asn His Pro Glu Val Ile Arg Arg Leu Gly Ile Ile
            340                 345                 350
Gly Ile Asn Thr Ala Leu Glu Phe Asp Ile Tyr Gly Asn Val Asn Ser
        355                 360                 365
Thr His Val Asp Gly Thr Asn Met Met Asn Gly Ile Gly Gly Ser Gly
    370                 375                 380
Asp Phe Ala Arg Asn Ala Tyr Ile Ser Ile Phe Val Ser Lys Ser Glu
385                 390                 395                 400
Ala Lys Asn Gly Ala Ile Ser Ser Val Val Pro Met Val Thr His Val
                405                 410                 415
Asp His Thr Glu His Asp Val Asp Ile Leu Val Thr Glu Gln Gly Leu
            420                 425                 430
Ala Asp Leu Arg Gly Leu Ala Pro Arg Glu Arg Ala Pro Val Ile Ile
        435                 440                 445
Glu Asn Cys Ala His Pro Asp Tyr Arg Asp Gln Leu Met Asp Tyr Tyr
    450                 455                 460
Glu Arg Ala Cys Lys Arg Gly Gly His Thr Pro His Leu Leu Glu Glu
465                 470                 475                 480
Ala Phe Ser Trp His Leu Arg Arg Gln Glu Asn Gly Ser Met Arg Lys
                485                 490                 495

<210> 23
<211> 1497
<212> DNA
<213> Agrobacterium tumefaciens

<220>
<221> CDS
<222> (1)..(1497)

<400> 23
atg ctt gaa aag cgt atc cgc aac gcg tcc ctc ttg aac agg atc gtc        48
Met Leu Glu Lys Arg Ile Arg Asn Ala Ser Leu Leu Asn Arg Ile Val
1               5                   10                  15
agc gcc gaa gag gcc gcc agt ctc att cag gat ggc atg acg gtg ggc        96
Ser Ala Glu Glu Ala Ala Ser Leu Ile Gln Asp Gly Met Thr Val Gly

                20                  25                  30
atg agc ggt ttt acg cgc gcc ggt gaa gcc aag gca gtg ccg ctg gcg        144
Met Ser Gly Phe Thr Arg Ala Gly Glu Ala Lys Ala Val Pro Leu Ala
        35                  40                  45
ctg gcc gag cgc gcc aag acc aac ccc atg aaa atc acc ctg atg acc        192
Leu Ala Glu Arg Ala Lys Thr Asn Pro Met Lys Ile Thr Leu Met Thr
    50                  55                  60
ggc gcc tcc ctc ggc aac gat ctc gac aag acc atg gtc gag gcc ggc        240
Gly Ala Ser Leu Gly Asn Asp Leu Asp Lys Thr Met Val Glu Ala Gly
65                  70                  75                  80
atg ctg gcg cgc cgc atg ccg ttc cag tcc gat ccg gct ttg cgc aag        288
Met Leu Ala Arg Arg Met Pro Phe Gln Ser Asp Pro Ala Leu Arg Lys
                85                  90                  95
gcg atc aat gac ggc agg gtg atg ttc atc gat cag cat ctt tcc gaa        336
Ala Ile Asn Asp Gly Arg Val Met Phe Ile Asp Gln His Leu Ser Glu
            100                 105                 110
acg gtc gag caa ttg cgc ggc ggt cag atc cat ggc gtt gat atc gcc        384
Thr Val Glu Gln Leu Arg Gly Gly Gln Ile His Gly Val Asp Ile Ala
        115                 120                 125
att gtc gag gcg gtg gca atc acg gcg gaa ggc ggc atc gta ccc acc        432
Ile Val Glu Ala Val Ala Ile Thr Ala Glu Gly Gly Ile Val Pro Thr
    130                 135                 140
acc tcg gtt ggt aat tcc gca agt ttc gcc att ctg gcc gat aag gtc        480
Thr Ser Val Gly Asn Ser Ala Ser Phe Ala Ile Leu Ala Asp Lys Val
145                 150                 155                 160
atc gtc gag atc aac ctg tcg cag ccc gag gtg ctg gaa ggg ctg cat        528
Ile Val Glu Ile Asn Leu Ser Gln Pro Glu Val Leu Glu Gly Leu His
                165                 170                 175
gat atc ttc att ccc gcc aag cgc ccg acc cgt atg ccc atc ccc gtc        576
Asp Ile Phe Ile Pro Ala Lys Arg Pro Thr Arg Met Pro Ile Pro Val
            180                 185                 190
gtg gcg acg gat agc cgt gtc ggc ctg cct ttc att ccg gtg ccg ccg        624
Val Ala Thr Asp Ser Arg Val Gly Leu Pro Phe Ile Pro Val Pro Pro
        195                 200                 205
gaa aag atc gct gcc att gtg ctg agc gac aag agc gac agt ttt tcg        672
Glu Lys Ile Ala Ala Ile Val Leu Ser Asp Lys Ser Asp Ser Phe Ser
    210                 215                 220
aca gtg ctg ccg ccg gat gac gag acg aag gcg att gcc ggc cat ctc        720
Thr Val Leu Pro Pro Asp Asp Glu Thr Lys Ala Ile Ala Gly His Leu
225                 230                 235                 240
acc gaa ttc ctg ctg aat gag gtc cgt cat ggc cgc atg acg cat gaa        768
Thr Glu Phe Leu Leu Asn Glu Val Arg His Gly Arg Met Thr His Glu
                245                 250                 255
ctc cag ccg ttg cag gca ggc atc ggc acc atc gcc aat gcc gtg atg        816
Leu Gln Pro Leu Gln Ala Gly Ile Gly Thr Ile Ala Asn Ala Val Met
            260                 265                 270
cac ggg ttc atc gat acg ccg ttt cac gat ctc aaa atg tat tcg gag        864
His Gly Phe Ile Asp Thr Pro Phe His Asp Leu Lys Met Tyr Ser Glu
        275                 280                 285
gtc ctg cag gat tcg acc ttc gaa ctg ttc gat gcg ggc aag ctg acc        912
Val Leu Gln Asp Ser Thr Phe Glu Leu Phe Asp Ala Gly Lys Leu Thr
    290                 295                 300
ttc gcc tcc ggt tcg tca atc acg ctc tcc tcc gcc atg aac gag cag        960
Phe Ala Ser Gly Ser Ser Ile Thr Leu Ser Ser Ala Met Asn Glu Gln
305                 310                 315                 320
gtc atg cca cga ttg tcc gag tat aag agc aga ctg atc ctt cgc ccg        1008
Val Met Pro Arg Leu Ser Glu Tyr Lys Ser Arg Leu Ile Leu Arg Pro
                325                 330                 335
cag gag gtc agc aac cat ccg gaa gtc atc cgc cgc ctc ggc atc atc        1056
Gln Glu Val Ser Asn His Pro Glu Val Ile Arg Arg Leu Gly Ile Ile
            340                 345                 350
ggc atc aac aca gct ctg gaa ttc gat att tac gga aat gtc aat tcc        1104
Gly Ile Asn Thr Ala Leu Glu Phe Asp Ile Tyr Gly Asn Val Asn Ser
        355                 360                 365
act cat gtc ggt ggc act cac atg atg aac ggc atc ggc ggt tcg ggt        1152
Thr His Val Gly Gly Thr His Met Met Asn Gly Ile Gly Gly Ser Gly
    370                 375                 380
gat ttt gcc cgc aac gct tac atg tcc atc ttc gtg acg aaa tcc atc        1200
Asp Phe Ala Arg Asn Ala Tyr Met Ser Ile Phe Val Thr Lys Ser Ile
385                 390                 395                 400
gcc aag ggc ggt gcc att tcc agc gtc gtg ccc atg gtc agc cat gtc        1248
Ala Lys Gly Gly Ala Ile Ser Ser Val Val Pro Met Val Ser His Val

PF 55709
                405                 410                 415
gac cat acc gaa cac gat gtc gat att ctc gtc aca gaa acg ggc cta        1296
Asp His Thr Glu His Asp Val Asp Ile Leu Val Thr Glu Thr Gly Leu
            420                 425                 430
gcc gat ctg cgc ggt ctg gcc ccg cgc gag cgc gca gcg gtc atc atc        1344
Ala Asp Leu Arg Gly Leu Ala Pro Arg Glu Arg Ala Ala Val Ile Ile
        435                 440                 445
gca aac tgc gtg cat ccc agc tat cgc gat gcg ctg acg gat tat tac        1392
Ala Asn Cys Val His Pro Ser Tyr Arg Asp Ala Leu Thr Asp Tyr Tyr
    450                 455                 460
cag cga gca gcg gcg cgg ggc ggg cac acg ccg cat ctc atc gaa gaa        1440
Gln Arg Ala Ala Ala Arg Gly Gly His Thr Pro His Leu Ile Glu Glu
465                 470                 475                 480
gcg ctg tca tgg cac aat gcg ctt cgg gaa aga ggc acc atg ctg ccg        1488
Ala Leu Ser Trp His Asn Ala Leu Arg Glu Arg Gly Thr Met Leu Pro
                485                 490                 495
cag cga tag                                                            1497
Gln Arg

<210> 24
<211> 498
<212> PRT
<213> Agrobacterium tumefaciens

<400> 24
Met Leu Glu Lys Arg Ile Arg Asn Ala Ser Leu Leu Asn Arg Ile Val
1               5                   10                  15
Ser Ala Glu Glu Ala Ala Ser Leu Ile Gln Asp Gly Met Thr Val Gly
            20                  25                  30
Met Ser Gly Phe Thr Arg Ala Gly Glu Ala Lys Ala Val Pro Leu Ala
        35                  40                  45
Leu Ala Glu Arg Ala Lys Thr Asn Pro Met Lys Ile Thr Leu Met Thr
    50                  55                  60
Gly Ala Ser Leu Gly Asn Asp Leu Asp Lys Thr Met Val Glu Ala Gly
65                  70                  75                  80
Met Leu Ala Arg Arg Met Pro Phe Gln Ser Asp Pro Ala Leu Arg Lys
                85                  90                  95
Ala Ile Asn Asp Gly Arg Val Met Phe Ile Asp Gln His Leu Ser Glu
            100                 105                 110
Thr Val Glu Gln Leu Arg Gly Gly Gln Ile His Gly Val Asp Ile Ala
        115                 120                 125
Ile Val Glu Ala Val Ala Ile Thr Ala Glu Gly Gly Ile Val Pro Thr
    130                 135                 140
Thr Ser Val Gly Asn Ser Ala Ser Phe Ala Ile Leu Ala Asp Lys Val
145                 150                 155                 160
Ile Val Glu Ile Asn Leu Ser Gln Pro Glu Val Leu Glu Gly Leu His
                165                 170                 175
Asp Ile Phe Ile Pro Ala Lys Arg Pro Thr Arg Met Pro Ile Pro Val
            180                 185                 190
Val Ala Thr Asp Ser Arg Val Gly Leu Pro Phe Ile Pro Val Pro Pro
        195                 200                 205
Glu Lys Ile Ala Ala Ile Val Leu Ser Asp Lys Ser Asp Ser Phe Ser
    210                 215                 220
Thr Val Leu Pro Pro Asp Asp Glu Thr Lys Ala Ile Ala Gly His Leu
225                 230                 235                 240
Thr Glu Phe Leu Leu Asn Glu Val Arg His Gly Arg Met Thr His Glu
                245                 250                 255
Leu Gln Pro Leu Gln Ala Gly Ile Gly Thr Ile Ala Asn Ala Val Met
            260                 265                 270
His Gly Phe Ile Asp Thr Pro Phe His Asp Leu Lys Met Tyr Ser Glu
        275                 280                 285
Val Leu Gln Asp Ser Thr Phe Glu Leu Phe Asp Ala Gly Lys Leu Thr
    290                 295                 300
Phe Ala Ser Gly Ser Ser Ile Thr Leu Ser Ser Ala Met Asn Glu Gln
305                 310                 315                 320
Val Met Pro Arg Leu Ser Glu Tyr Lys Ser Arg Leu Ile Leu Arg Pro
                325                 330                 335
Gln Glu Val Ser Asn His Pro Glu Val Ile Arg Arg Leu Gly Ile Ile
            340                 345                 350
Gly Ile Asn Thr Ala Leu Glu Phe Asp Ile Tyr Gly Asn Val Asn Ser

355                 360                 365
Thr His Val Gly Gly Thr His Met Met Asn Gly Ile Gly Gly Ser Gly
    370                 375                 380
Asp Phe Ala Arg Asn Ala Tyr Met Ser Ile Phe Val Thr Lys Ser Ile
385                 390                 395                 400
Ala Lys Gly Gly Ala Ile Ser Ser Val Val Pro Met Val Ser His Val
                405                 410                 415
Asp His Thr Glu His Asp Val Asp Ile Leu Val Thr Glu Thr Gly Leu
            420                 425                 430
Ala Asp Leu Arg Gly Leu Ala Pro Arg Glu Arg Ala Ala Val Ile Ile
        435                 440                 445
Ala Asn Cys Val His Pro Ser Tyr Arg Asp Ala Leu Thr Asp Tyr Tyr
    450                 455                 460
Gln Arg Ala Ala Ala Arg Gly Gly His Thr Pro His Leu Ile Glu Glu
465                 470                 475                 480
Ala Leu Ser Trp His Asn Ala Leu Arg Glu Arg Gly Thr Met Leu Pro
                485                 490                 495
Gln Arg

<210> 25
<211> 1545
<212> DNA
<213> Caulobacter crescentus

<220>
<221> CDS
<222> (1)..(1545)

<400> 25
atg cgg gtg aag caa gcg cgt ggg gcg agc atg agc gga tca cgg atc       48
Met Arg Val Lys Gln Ala Arg Gly Ala Ser Met Ser Gly Ser Arg Ile
1               5                   10                  15
ggg ctg gac gcg ctg aag gcc aag gtg atg tcg ccc gag gac gcg gcg       96
Gly Leu Asp Ala Leu Lys Ala Lys Val Met Ser Pro Glu Asp Ala Ala
            20                  25                  30
gcg ctg atc ggt cca ggc gag acc gtc ggc atg agc ggc ttc acc gga      144
Ala Leu Ile Gly Pro Gly Glu Thr Val Gly Met Ser Gly Phe Thr Gly
        35                  40                  45
tcg ggc tat ccc aag gcc gtt ccc aca gcc ctg gcg cgc cgc atc gag      192
Ser Gly Tyr Pro Lys Ala Val Pro Thr Ala Leu Ala Arg Arg Ile Glu
    50                  55                  60
gcc gag cac gcc gct ggg cgt ccc ttc aag ctg cgc gtc tgg acc ggc      240
Ala Glu His Ala Ala Gly Arg Pro Phe Lys Leu Arg Val Trp Thr Gly
65                  70                  75                  80
gcc tcg acg gga ccc gag ctg gac ggg gcc ctg gcc aag gcc aac ggc      288
Ala Ser Thr Gly Pro Glu Leu Asp Gly Ala Leu Ala Lys Ala Asn Gly
                85                  90                  95
atc gag ttc cgc cta ccc tac aat tcc gac cct atc gcc cgg gaa atg      336
Ile Glu Phe Arg Leu Pro Tyr Asn Ser Asp Pro Ile Ala Arg Glu Met
            100                 105                 110
atc aat cgc ggc gag atg gac tat ttc gac atg cac ctg tcg cag gtg      384
Ile Asn Arg Gly Glu Met Asp Tyr Phe Asp Met His Leu Ser Gln Val
        115                 120                 125
gcg ccg atg gcc tgg caa ggc ttt ctg ggt ccg ctc gac acc gcc ctg      432
Ala Pro Met Ala Trp Gln Gly Phe Leu Gly Pro Leu Asp Thr Ala Leu
    130                 135                 140
atc gag gtc acc ggg atc cgg tcg gac ggt tcg ctg atc ccg tcc tcg      480
Ile Glu Val Thr Gly Ile Arg Ser Asp Gly Ser Leu Ile Pro Ser Ser
145                 150                 155                 160
tcc gtg ggc aac aac aag acc tgg atc gaa cgg gcc tcg aaa gtg atc      528
Ser Val Gly Asn Asn Lys Thr Trp Ile Glu Arg Ala Ser Lys Val Ile
                165                 170                 175
ctt gag gtg aac cgt tgg cag aac ccc gcc ttg gag ggg atg cac gat      576
Leu Glu Val Asn Arg Trp Gln Asn Pro Ala Leu Glu Gly Met His Asp
            180                 185                 190
atc tat tac ggc acg gcc ttg ccg ccg gac cgg cgc cct att ccg ttg      624
Ile Tyr Tyr Gly Thr Ala Leu Pro Pro Asp Arg Arg Pro Ile Pro Leu
        195                 200                 205
acg cga ccg gac cag cgg atc ggc gag cca tat ttc cgg tgc gac ccg      672
Thr Arg Pro Asp Gln Arg Ile Gly Glu Pro Tyr Phe Arg Cys Asp Pro
    210                 215                 220

gcg aag gtc gtg gcg gtg gtc gaa acc gac acc ccc gat cgc aat ctg     720
Ala Lys Val Val Ala Val Val Glu Thr Asp Thr Pro Asp Arg Asn Leu
225                 230                 235                 240
ccg ttc gat gcg ccg ggc gag gat cac cgc gcc atc gcc ggc cac ctt     768
Pro Phe Asp Ala Pro Gly Glu Asp His Arg Ala Ile Ala Gly His Leu
                245                 250                 255
ctg gag ttt ttc cgc cac gag gtg gcg cgg ggt cgc ctg ccg gcc aac     816
Leu Glu Phe Phe Arg His Glu Val Ala Arg Gly Arg Leu Pro Ala Asn
            260                 265                 270
ctg ctg ccg ctg cag tcg ggc gtc ggc aac atc gcc aat gcg gtg atg     864
Leu Leu Pro Leu Gln Ser Gly Val Gly Asn Ile Ala Asn Ala Val Met
        275                 280                 285
ttc ggc ctg gtc gat ggg ccg ttc gag ggt acg acc gcc ttt acc gag     912
Phe Gly Leu Val Asp Gly Pro Phe Glu Gly Thr Thr Ala Phe Thr Glu
    290                 295                 300
gtc ctg cag gat ggg atg ctg gcg ctg ctg gag tcg ggc agg ctg ctc     960
Val Leu Gln Asp Gly Met Leu Ala Leu Leu Glu Ser Gly Arg Leu Leu
305                 310                 315                 320
agc gcc tcg gcc acg gcc ttc tct ctc agc ccc gag gcc gcc cat acg     1008
Ser Ala Ser Ala Thr Ala Phe Ser Leu Ser Pro Glu Ala Ala His Thr
                325                 330                 335
ctg aac gcc gac ctc gac aaa ctc cgc gac cgc ctg gtc ctg cga ccg     1056
Leu Asn Ala Asp Leu Asp Lys Leu Arg Asp Arg Leu Val Leu Arg Pro
            340                 345                 350
cag gag atc agc aat cac ccc gag ctg atc cga cgc ctg ggc tgc ctc     1104
Gln Glu Ile Ser Asn His Pro Glu Leu Ile Arg Arg Leu Gly Cys Leu
        355                 360                 365
gcc atg aac ggc atg atc gag gcc gac atc cac ggg gcg gtg aac tcg     1152
Ala Met Asn Gly Met Ile Glu Ala Asp Ile His Gly Ala Val Asn Ser
    370                 375                 380
acc cac ctg atg ggc tcg cgc atc cag aac ggc atc ggc ggc agc ggc     1200
Thr His Leu Met Gly Ser Arg Ile Gln Asn Gly Ile Gly Gly Ser Gly
385                 390                 395                 400
gac ttc gcc cgc aac gcc tat gtc tcg att ttc atg gcc ccc tcg acg     1248
Asp Phe Ala Arg Asn Ala Tyr Val Ser Ile Phe Met Ala Pro Ser Thr
                405                 410                 415
gcc aag ggc ggc aag atc tcg acc att gtg ccc aag acc ccg cac gtc     1296
Ala Lys Gly Gly Lys Ile Ser Thr Ile Val Pro Lys Thr Pro His Val
            420                 425                 430
gat cac atc aac cag gac gtc cag gtc ctg gtc acc gag cag ggc ctg     1344
Asp His Ile Asn Gln Asp Val Gln Val Leu Val Thr Glu Gln Gly Leu
        435                 440                 445
gcc gat ctg cgg gcc ctg tcg ccg cgg cag cgg gcc gag gtg atc atc     1392
Ala Asp Leu Arg Ala Leu Ser Pro Arg Gln Arg Ala Glu Val Ile Ile
    450                 455                 460
gag aac tgc gcc cat cca gac tat cga ccg gcc ttg aag gac tat ctg     1440
Glu Asn Cys Ala His Pro Asp Tyr Arg Pro Ala Leu Lys Asp Tyr Leu
465                 470                 475                 480
acc cgc gcc cgc cgc gac tcc tac ggc cag cag tcg ccg acc ctg ctg     1488
Thr Arg Ala Arg Arg Asp Ser Tyr Gly Gln Gln Ser Pro Thr Leu Leu
                485                 490                 495
atg gaa gcc ttc gcc tgg cac cag cgg ttt atc gag acc ggc gat atg     1536
Met Glu Ala Phe Ala Trp His Gln Arg Phe Ile Glu Thr Gly Asp Met
            500                 505                 510
cgc ggg tga                                                         1545
Arg Gly

<210> 26
<211> 514
<212> PRT
<213> Caulobacter crescentus

<400> 26
Met Arg Val Lys Gln Ala Arg Gly Ala Ser Met Ser Gly Ser Arg Ile
1               5                   10                  15
Gly Leu Asp Ala Leu Lys Ala Lys Val Met Ser Pro Glu Asp Ala Ala
            20                  25                  30
Ala Leu Ile Gly Pro Gly Glu Thr Val Gly Met Ser Gly Phe Thr Gly
        35                  40                  45
Ser Gly Tyr Pro Lys Ala Val Pro Thr Ala Leu Ala Arg Arg Ile Glu

PF 55709
50                  55                  60
Ala Glu His Ala Ala Gly Arg Pro Phe Lys Leu Arg Val Trp Thr Gly
65                  70                  75                  80
Ala Ser Thr Gly Pro Glu Leu Asp Gly Ala Leu Ala Lys Ala Asn Gly
                85                  90                  95
Ile Glu Phe Arg Leu Pro Tyr Asn Ser Asp Pro Ile Ala Arg Glu Met
            100                 105                 110
Ile Asn Arg Gly Glu Met Asp Tyr Phe Asp Met His Leu Ser Gln Val
        115                 120                 125
Ala Pro Met Ala Trp Gln Gly Phe Leu Gly Pro Leu Asp Thr Ala Leu
    130                 135                 140
Ile Glu Val Thr Gly Ile Arg Ser Asp Gly Ser Leu Ile Pro Ser Ser
145                 150                 155                 160
Ser Val Gly Asn Asn Lys Thr Trp Ile Glu Arg Ala Ser Lys Val Ile
                165                 170                 175
Leu Glu Val Asn Arg Trp Gln Asn Pro Ala Leu Glu Gly Met His Asp
            180                 185                 190
Ile Tyr Tyr Gly Thr Ala Leu Pro Pro Asp Arg Arg Pro Ile Pro Leu
        195                 200                 205
Thr Arg Pro Asp Gln Arg Ile Gly Glu Pro Tyr Phe Arg Cys Asp Pro
    210                 215                 220
Ala Lys Val Val Ala Val Val Glu Thr Asp Thr Pro Asp Arg Asn Leu
225                 230                 235                 240
Pro Phe Asp Ala Pro Gly Glu Asp His Arg Ala Ile Ala Gly His Leu
                245                 250                 255
Leu Glu Phe Phe Arg His Glu Val Ala Arg Gly Arg Leu Pro Ala Asn
            260                 265                 270
Leu Leu Pro Leu Gln Ser Gly Val Gly Asn Ile Ala Asn Ala Val Met
        275                 280                 285
Phe Gly Leu Val Asp Gly Pro Phe Glu Gly Thr Thr Ala Phe Thr Glu
    290                 295                 300
Val Leu Gln Asp Gly Met Leu Ala Leu Leu Glu Ser Gly Arg Leu Leu
305                 310                 315                 320
Ser Ala Ser Ala Thr Ala Phe Ser Leu Ser Pro Glu Ala Ala His Thr
                325                 330                 335
Leu Asn Ala Asp Leu Asp Lys Leu Arg Asp Arg Leu Val Leu Arg Pro
            340                 345                 350
Gln Glu Ile Ser Asn His Pro Glu Leu Ile Arg Arg Leu Gly Cys Leu
        355                 360                 365
Ala Met Asn Gly Met Ile Glu Ala Asp Ile His Gly Ala Val Asn Ser
    370                 375                 380
Thr His Leu Met Gly Ser Arg Ile Gln Asn Gly Ile Gly Gly Ser Gly
385                 390                 395                 400
Asp Phe Ala Arg Asn Ala Tyr Val Ser Ile Phe Met Ala Pro Ser Thr
                405                 410                 415
Ala Lys Gly Gly Lys Ile Ser Thr Ile Val Pro Lys Thr Pro His Val
            420                 425                 430
Asp His Ile Asn Gln Asp Val Gln Val Leu Val Thr Glu Gln Gly Leu
        435                 440                 445
Ala Asp Leu Arg Ala Leu Ser Pro Arg Gln Arg Ala Glu Val Ile Ile
    450                 455                 460
Glu Asn Cys Ala His Pro Asp Tyr Arg Pro Ala Leu Lys Asp Tyr Leu
465                 470                 475                 480
Thr Arg Ala Arg Arg Asp Ser Tyr Gly Gln Gln Ser Pro Thr Leu Leu
                485                 490                 495
Met Glu Ala Phe Ala Trp His Gln Arg Phe Ile Glu Thr Gly Asp Met
            500                 505                 510
Arg Gly


<210> 27
<211> 1479
<212> DNA
<213> Escherichia coli

<220>
<221> CDS
<222> (1)..(1479)

<400> 27
atg gaa act cag tgg aca agg atg acc gcc aat gaa gcg gca gaa att        48
Met Glu Thr Gln Trp Thr Arg Met Thr Ala Asn Glu Ala Ala Glu Ile

PF 55709
1               5                   10                  15
atc cag cat aac gac atg gtg gca ttt agc ggc ttt acc ccg gcg ggt          96
Ile Gln His Asn Asp Met Val Ala Phe Ser Gly Phe Thr Pro Ala Gly
            20                  25                  30
tcg ccg aaa gcc cta ccc acc gcg att gcc cgc aga gct aac gaa cag          144
Ser Pro Lys Ala Leu Pro Thr Ala Ile Ala Arg Arg Ala Asn Glu Gln
        35                  40                  45
cat gag gcc aaa aag ccg tat caa att cgc ctt ctg acg ggt gcg tca          192
His Glu Ala Lys Lys Pro Tyr Gln Ile Arg Leu Leu Thr Gly Ala Ser
    50                  55                  60
atc agc gcc gcc gct gac gat gta ctt tct gac gcc gat gct gtt tcc          240
Ile Ser Ala Ala Ala Asp Asp Val Leu Ser Asp Ala Asp Ala Val Ser
65                  70                  75                  80
tgg cgt gcg cca tat caa aca tcg tcc ggt tta cgt aaa aag atc aat          288
Trp Arg Ala Pro Tyr Gln Thr Ser Ser Gly Leu Arg Lys Lys Ile Asn
                85                  90                  95
cag ggc gcg gtg agt ttc gtt gac ctg cat ttg agc gaa gtg gcg caa          336
Gln Gly Ala Val Ser Phe Val Asp Leu His Leu Ser Glu Val Ala Gln
            100                 105                 110
atg gtc aat tac ggt ttc ttc ggc gac att gat gtt gcc gtc att gaa          384
Met Val Asn Tyr Gly Phe Phe Gly Asp Ile Asp Val Ala Val Ile Glu
        115                 120                 125
gca tcg gca ctg gca ccg gat ggt cga gtc tgg tta acc agc ggg atc          432
Ala Ser Ala Leu Ala Pro Asp Gly Arg Val Trp Leu Thr Ser Gly Ile
    130                 135                 140
ggt aat gcg ccg acc tgg ctg ctg cgg gcg aag aaa gtg atc att gaa          480
Gly Asn Ala Pro Thr Trp Leu Leu Arg Ala Lys Lys Val Ile Ile Glu
145                 150                 155                 160
ctc aat cac tat cac gat ccg cgc gtt gca gaa ctg gcg gat att gtg          528
Leu Asn His Tyr His Asp Pro Arg Val Ala Glu Leu Ala Asp Ile Val
                165                 170                 175
att cct ggc gcg cca ccg cgg cgc aat agc gtg tcg atc ttc cat gca          576
Ile Pro Gly Ala Pro Pro Arg Arg Asn Ser Val Ser Ile Phe His Ala
            180                 185                 190
atg gat cgc gtc ggt acc cgc tat gtg caa atc gat ccg aaa aag att          624
Met Asp Arg Val Gly Thr Arg Tyr Val Gln Ile Asp Pro Lys Lys Ile
        195                 200                 205
gtc gcc gtc gtg gaa acc aac ttg ccc gac gcc ggt aat atg ctg gat          672
Val Ala Val Val Glu Thr Asn Leu Pro Asp Ala Gly Asn Met Leu Asp
    210                 215                 220
aag caa aat ccc atg tgc cag cag att gcc gat aac gtg gtc acg ttc          720
Lys Gln Asn Pro Met Cys Gln Gln Ile Ala Asp Asn Val Val Thr Phe
225                 230                 235                 240
tta ttg cag gaa atg gcg cat ggg cgt att ccg ccg gaa ttt ctg ccg          768
Leu Leu Gln Glu Met Ala His Gly Arg Ile Pro Pro Glu Phe Leu Pro
                245                 250                 255
ctg caa agt ggc gtg ggc aat atc aat aat gcg gta atg gcg cgt ctg          816
Leu Gln Ser Gly Val Gly Asn Ile Asn Asn Ala Val Met Ala Arg Leu
            260                 265                 270
ggg gaa aac ccg gta att cct ccg ttt atg atg tat tcg gaa gtg cta          864
Gly Glu Asn Pro Val Ile Pro Pro Phe Met Met Tyr Ser Glu Val Leu
        275                 280                 285
cag gaa tcg gtg gtg cat tta ctg gaa acc ggc aaa atc agc ggg gcc          912
Gln Glu Ser Val Val His Leu Leu Glu Thr Gly Lys Ile Ser Gly Ala
    290                 295                 300
agc gcc tcc agc ctg aca atc tcg gcc gat tcc ctg cgc aag att tac          960
Ser Ala Ser Ser Leu Thr Ile Ser Ala Asp Ser Leu Arg Lys Ile Tyr
305                 310                 315                 320
gac aat atg gat tac ttt gcc agc cgc att gtg ttg cgt ccg cag gag          1008
Asp Asn Met Asp Tyr Phe Ala Ser Arg Ile Val Leu Arg Pro Gln Glu
                325                 330                 335
att tcc aat aac ccg gaa atc atc cgt cgt ctg ggc gtc atc gct ctg          1056
Ile Ser Asn Asn Pro Glu Ile Ile Arg Arg Leu Gly Val Ile Ala Leu
            340                 345                 350
aac gtc ggc ctg gag ttt gat att tac ggg cat gcc aac tca aca cac          1104
Asn Val Gly Leu Glu Phe Asp Ile Tyr Gly His Ala Asn Ser Thr His
        355                 360                 365
gta gcc ggg gtc gat ctg atg aac ggc atc ggc ggc agc ggt gat ttt          1152
Val Ala Gly Val Asp Leu Met Asn Gly Ile Gly Gly Ser Gly Asp Phe
    370                 375                 380
gaa cgc aac gcg tat ctg tcg atc ttt atg gcc ccg tcg att gct aaa          1200
Glu Arg Asn Ala Tyr Leu Ser Ile Phe Met Ala Pro Ser Ile Ala Lys

385                 390                 395                 400
gaa ggc aag atc tca acc gtc gtg cca atg tgc agc cat gtt gat cac      1248
Glu Gly Lys Ile Ser Thr Val Val Pro Met Cys Ser His Val Asp His
                405                 410                 415
agc gaa cac agc gtc aaa gtg atc atc acc gaa caa ggg atc gcc gat      1296
Ser Glu His Ser Val Lys Val Ile Ile Thr Glu Gln Gly Ile Ala Asp
            420                 425                 430
ctg cgc ggt ctt tcc ccg ctt caa cgc gcc cgc act atc att gat aat      1344
Leu Arg Gly Leu Ser Pro Leu Gln Arg Ala Arg Thr Ile Ile Asp Asn
        435                 440                 445
tgt gca cat cct atg tat cgg gat tat ctg cat cgc tat ctg gaa aat      1392
Cys Ala His Pro Met Tyr Arg Asp Tyr Leu His Arg Tyr Leu Glu Asn
    450                 455                 460
gcg cct ggc gga cat att cac cac gat ctt agc cac gtc ttc gac tta      1440
Ala Pro Gly Gly His Ile His His Asp Leu Ser His Val Phe Asp Leu
465                 470                 475                 480
cac cgt aat tta att gca acc ggc tcg atg ctg ggt taa                  1479
His Arg Asn Leu Ile Ala Thr Gly Ser Met Leu Gly
                485                 490

<210> 28
<211> 492
<212> PRT
<213> Escherichia coli

<400> 28
Met Glu Thr Gln Trp Thr Arg Met Thr Ala Asn Glu Ala Ala Glu Ile
1               5                   10                  15
Ile Gln His Asn Asp Met Val Ala Phe Ser Gly Phe Thr Pro Ala Gly
            20                  25                  30
Ser Pro Lys Ala Leu Pro Thr Ala Ile Ala Arg Arg Ala Asn Glu Gln
        35                  40                  45
His Glu Ala Lys Lys Pro Tyr Gln Ile Arg Leu Leu Thr Gly Ala Ser
    50                  55                  60
Ile Ser Ala Ala Ala Asp Asp Val Leu Ser Asp Ala Asp Ala Val Ser
65                  70                  75                  80
Trp Arg Ala Pro Tyr Gln Thr Ser Ser Gly Leu Arg Lys Lys Ile Asn
                85                  90                  95
Gln Gly Ala Val Ser Phe Val Asp Leu His Leu Ser Glu Val Ala Gln
            100                 105                 110
Met Val Asn Tyr Gly Phe Phe Gly Asp Ile Asp Val Ala Val Ile Glu
        115                 120                 125
Ala Ser Ala Leu Ala Pro Asp Gly Arg Val Trp Leu Thr Ser Gly Ile
    130                 135                 140
Gly Asn Ala Pro Thr Trp Leu Leu Arg Ala Lys Lys Val Ile Ile Glu
145                 150                 155                 160
Leu Asn His Tyr His Asp Pro Arg Val Ala Glu Leu Ala Asp Ile Val
                165                 170                 175
Ile Pro Gly Ala Pro Pro Arg Arg Asn Ser Val Ser Ile Phe His Ala
            180                 185                 190
Met Asp Arg Val Gly Thr Arg Tyr Val Gln Ile Asp Pro Lys Lys Ile
        195                 200                 205
Val Ala Val Val Glu Thr Asn Leu Pro Asp Ala Gly Asn Met Leu Asp
    210                 215                 220
Lys Gln Asn Pro Met Cys Gln Gln Ile Ala Asp Asn Val Val Thr Phe
225                 230                 235                 240
Leu Leu Gln Glu Met Ala His Gly Arg Ile Pro Pro Glu Phe Leu Pro
                245                 250                 255
Leu Gln Ser Gly Val Gly Asn Ile Asn Asn Ala Val Met Ala Arg Leu
            260                 265                 270
Gly Glu Asn Pro Val Ile Pro Pro Phe Met Met Tyr Ser Glu Val Leu
        275                 280                 285
Gln Glu Ser Val Val His Leu Leu Glu Thr Gly Lys Ile Ser Gly Ala
    290                 295                 300
Ser Ala Ser Ser Leu Thr Ile Ser Ala Asp Ser Leu Arg Lys Ile Tyr
305                 310                 315                 320
Asp Asn Met Asp Tyr Phe Ala Ser Arg Ile Val Leu Arg Pro Gln Glu
                325                 330                 335
Ile Ser Asn Asn Pro Glu Ile Ile Arg Arg Leu Gly Val Ile Ala Leu
            340                 345                 350
Asn Val Gly Leu Glu Phe Asp Ile Tyr Gly His Ala Asn Ser Thr His

355                 360                 365
Val Ala Gly Val Asp Leu Met Asn Gly Ile Gly Gly Ser Gly Asp Phe
    370                 375                 380
Glu Arg Asn Ala Tyr Leu Ser Ile Phe Met Ala Pro Ser Ile Ala Lys
385                 390                 395                 400
Glu Gly Lys Ile Ser Thr Val Val Pro Met Cys Ser His Val Asp His
                405                 410                 415
Ser Glu His Ser Val Lys Val Ile Ile Thr Glu Gln Gly Ile Ala Asp
            420                 425                 430
Leu Arg Gly Leu Ser Pro Leu Gln Arg Ala Arg Thr Ile Ile Asp Asn
        435                 440                 445
Cys Ala His Pro Met Tyr Arg Asp Tyr Leu His Arg Tyr Leu Glu Asn
    450                 455                 460
Ala Pro Gly Gly His Ile His His Asp Leu Ser His Val Phe Asp Leu
465                 470                 475                 480
His Arg Asn Leu Ile Ala Thr Gly Ser Met Leu Gly
                485                 490

<210> 29
<211> 1476
<212> DNA
<213> Halobacterium sp. NRC-1

<220>
<221> CDS
<222> (1)..(1476)

<400> 29
atg aca gca ccg cgg acg gcc ggc gtg gcg ggg cgc gtg aac ggc gac      48
Met Thr Ala Pro Arg Thr Ala Gly Val Ala Gly Arg Val Asn Gly Asp
1               5                   10                  15
tgt ccg gtc aaa gac gcc acc acc gtg gcg gcc ggc atc gcc gac gac      96
Cys Pro Val Lys Asp Ala Thr Thr Val Ala Ala Gly Ile Ala Asp Asp
            20                  25                  30
gcg acg ctc gcg gtg tct ggg ttc ggg agc gtc ggc gac ccg aag gcg      144
Ala Thr Leu Ala Val Ser Gly Phe Gly Ser Val Gly Asp Pro Lys Ala
        35                  40                  45
gtg cca cgg gcg ctg gcc gcg gcc gcg cgc gac ggc cgc gac ctc gcg      192
Val Pro Arg Ala Leu Ala Ala Ala Ala Arg Asp Gly Arg Asp Leu Ala
    50                  55                  60
ctc acc gtc gtc tcc ggg ggg agc gtc ggc gac cca ctg gac acc gat      240
Leu Thr Val Val Ser Gly Gly Ser Val Gly Asp Pro Leu Asp Thr Asp
65                  70                  75                  80
ctc gtg gcg gcg ggc ggc gtc gcg cgg cgg tac ccg tat cag gcg acg      288
Leu Val Ala Ala Gly Gly Val Ala Arg Arg Tyr Pro Tyr Gln Ala Thr
                85                  90                  95
acg gcc gcg cgg acg gcc gcg aac gag cgg gag ttg gcg ttc gcg gat      336
Thr Ala Ala Arg Thr Ala Ala Asn Glu Arg Glu Leu Ala Phe Ala Asp
            100                 105                 110
cgc ggg atc gcc ggg ctg agc gac gag gtg cgg ttc ggg cgg ctg gtg      384
Arg Gly Ile Ala Gly Leu Ser Asp Glu Val Arg Phe Gly Arg Leu Val
        115                 120                 125
gac ccc gac gtg gcg gtc gtg gag gcg gtg gcg gtc ggg ccg ggg tgg      432
Asp Pro Asp Val Ala Val Val Glu Ala Val Ala Val Gly Pro Gly Trp
    130                 135                 140
ctg atc ccc tcg acg tcc gtg ggg cag acg ccg gcg tac gtg cgg gcg      480
Leu Ile Pro Ser Thr Ser Val Gly Gln Thr Pro Ala Tyr Val Arg Ala
145                 150                 155                 160
gcc gac gag gtg gtg gtg gag gtc aac gac gcc cag ccg atg ggg ctg      528
Ala Asp Glu Val Val Val Glu Val Asn Asp Ala Gln Pro Met Gly Leu
                165                 170                 175
tcg gcg ttc cac gac gtg gtg gtg cgt gac ccg ccg ccc gac cgg ggg      576
Ser Ala Phe His Asp Val Val Val Arg Asp Pro Pro Pro Asp Arg Gly
            180                 185                 190
ccg ctg gcg gtg tcc gcg ccg ggc gag cgg gtg ggc gag tcg gcc gtg      624
Pro Leu Ala Val Ser Ala Pro Gly Glu Arg Val Gly Glu Ser Ala Val
        195                 200                 205
cga ttc gac ccc gag aag ctg cgc gcg gtc gtg cgc acg gac cgc cgt      672
Arg Phe Asp Pro Glu Lys Leu Arg Ala Val Val Arg Thr Asp Arg Arg
    210                 215                 220
gat gat ccc tac gag ttc cgt gac ccg acc gcg act gac cgc cgc atc      720
Asp Asp Pro Tyr Glu Phe Arg Asp Pro Thr Ala Thr Asp Arg Arg Ile

225                 230                 235                 240
gcc gac aac gtc ttg acc gtg ttg gag cgc gcc gtc gac cgt gat ccg       768
Ala Asp Asn Val Leu Thr Val Leu Glu Arg Ala Val Asp Arg Asp Pro
                245                 250                 255
gtg atc ggg gcg gca ccg acg ctc cag ttc ggc gtc ggc agc ctc ggg       816
Val Ile Gly Ala Ala Pro Thr Leu Gln Phe Gly Val Gly Ser Leu Gly
            260                 265                 270
aac gcg ttg atg ggc gcg ctg tcg ggg tcg tcg ctc gcc gac ggt gag       864
Asn Ala Leu Met Gly Ala Leu Ser Gly Ser Ser Leu Ala Asp Gly Glu
        275                 280                 285
ctg cgg tat tac ggc gag gtg atc cag gac ggc ctg ttg gac ctg ctt       912
Leu Arg Tyr Tyr Gly Glu Val Ile Gln Asp Gly Leu Leu Asp Leu Leu
    290                 295                 300
gcg gac ggc gac ctg gcg gtg gcg agc gcg acc tcg ctg gcg ctc tca       960
Ala Asp Gly Asp Leu Ala Val Ala Ser Ala Thr Ser Leu Ala Leu Ser
305                 310                 315                 320
gtg gac gga cag gat cgc gtg ttc gac gcg ccc gag cgg tac gcc gag      1008
Val Asp Gly Gln Asp Arg Val Phe Asp Ala Pro Glu Arg Tyr Ala Glu
                325                 330                 335
gac gtg gtg ctc cgc ccg tcg aac gtc tcg aac gcg ccg tcg ctg atc      1056
Asp Val Val Leu Arg Pro Ser Asn Val Ser Asn Ala Pro Ser Leu Ile
            340                 345                 350
gac cgg ttc ggg gtg gtc gcg gtc aac agc gcg gtc ggc gtg gat ctc      1104
Asp Arg Phe Gly Val Val Ala Val Asn Ser Ala Val Gly Val Asp Leu
        355                 360                 365
tac gga cac gtc aac tcc aca cac gtc tca ggg gcg cgg ctc atc aac      1152
Tyr Gly His Val Asn Ser Thr His Val Ser Gly Ala Arg Leu Ile Asn
    370                 375                 380
ggc atc ggc ggc agc gtc gat ttc aat cgg aac gcc cac ctc act gtg      1200
Gly Ile Gly Gly Ser Val Asp Phe Asn Arg Asn Ala His Leu Thr Val
385                 390                 395                 400
gtg gcg ctg ccc gcg acc gcg gcc ggc ggc gac gtg tcg acg gtc gtg      1248
Val Ala Leu Pro Ala Thr Ala Ala Gly Gly Asp Val Ser Thr Val Val
                405                 410                 415
ccg ctg gcg agc cac gtc gac cac acc gaa cac gac acc gac gtg gtg      1296
Pro Leu Ala Ser His Val Asp His Thr Glu His Asp Thr Asp Val Val
            420                 425                 430
gtg acc gag cac ggc gtc gcg gac ctg cgc ggg aag cca ccc gtc gcg      1344
Val Thr Glu His Gly Val Ala Asp Leu Arg Gly Lys Pro Pro Val Ala
        435                 440                 445
cgc gcc gag gcg atc atc gag tgc gcc gcc ccc gcg ttc agg ccg gat      1392
Arg Ala Glu Ala Ile Ile Glu Cys Ala Ala Pro Ala Phe Arg Pro Asp
    450                 455                 460
ctg cgg gcg tac ctg gat cgc gcc cgg gag tca tcg ggg cac ctc cca      1440
Leu Arg Ala Tyr Leu Asp Arg Ala Arg Glu Ser Ser Gly His Leu Pro
465                 470                 475                 480
cac gac ttg tcg acg gcg ttc gac tgg cag tcg tag                      1476
His Asp Leu Ser Thr Ala Phe Asp Trp Gln Ser
                485                 490

<210> 30
<211> 491
<212> PRT
<213> Halobacterium sp. NRC-1

<400> 30
Met Thr Ala Pro Arg Thr Ala Gly Val Ala Gly Arg Val Asn Gly Asp
1               5                   10                  15
Cys Pro Val Lys Asp Ala Thr Thr Val Ala Ala Gly Ile Ala Asp Asp
            20                  25                  30
Ala Thr Leu Ala Val Ser Gly Phe Gly Ser Val Gly Asp Pro Lys Ala
        35                  40                  45
Val Pro Arg Ala Leu Ala Ala Ala Ala Arg Asp Gly Arg Asp Leu Ala
    50                  55                  60
Leu Thr Val Val Ser Gly Gly Ser Val Gly Asp Pro Leu Asp Thr Asp
65                  70                  75                  80
Leu Val Ala Ala Gly Gly Val Ala Arg Arg Tyr Pro Tyr Gln Ala Thr
                85                  90                  95
Thr Ala Ala Arg Thr Ala Ala Asn Glu Arg Glu Leu Ala Phe Ala Asp
            100                 105                 110
Arg Gly Ile Ala Gly Leu Ser Asp Glu Val Arg Phe Gly Arg Leu Val

115                 120                 125
Asp Pro Asp Val Ala Val Val Glu Ala Val Ala Val Gly Pro Gly Trp
    130                 135                 140
Leu Ile Pro Ser Thr Ser Val Gly Gln Thr Pro Ala Tyr Val Arg Ala
145                 150                 155                 160
Ala Asp Glu Val Val Val Glu Val Asn Asp Ala Gln Pro Met Gly Leu
                165                 170                 175
Ser Ala Phe His Asp Val Val Val Arg Asp Pro Pro Pro Asp Arg Gly
            180                 185                 190
Pro Leu Ala Val Ser Ala Pro Gly Glu Arg Val Gly Glu Ser Ala Val
        195                 200                 205
Arg Phe Asp Pro Glu Lys Leu Arg Ala Val Val Arg Thr Asp Arg Arg
    210                 215                 220
Asp Asp Pro Tyr Glu Phe Arg Asp Pro Thr Ala Thr Asp Arg Arg Ile
225                 230                 235                 240
Ala Asp Asn Val Leu Thr Val Leu Glu Arg Ala Val Asp Arg Asp Pro
                245                 250                 255
Val Ile Gly Ala Ala Pro Thr Leu Gln Phe Gly Val Gly Ser Leu Gly
            260                 265                 270
Asn Ala Leu Met Gly Ala Leu Ser Gly Ser Ser Leu Ala Asp Gly Glu
        275                 280                 285
Leu Arg Tyr Tyr Gly Glu Val Ile Gln Asp Gly Leu Leu Asp Leu Leu
    290                 295                 300
Ala Asp Gly Asp Leu Ala Val Ala Ser Ala Thr Ser Leu Ala Leu Ser
305                 310                 315                 320
Val Asp Gly Gln Asp Arg Val Phe Asp Ala Pro Glu Arg Tyr Ala Glu
                325                 330                 335
Asp Val Val Leu Arg Pro Ser Asn Val Ser Asn Ala Pro Ser Leu Ile
            340                 345                 350
Asp Arg Phe Gly Val Val Ala Val Asn Ser Ala Val Gly Val Asp Leu
        355                 360                 365
Tyr Gly His Val Asn Ser Thr His Val Ser Gly Ala Arg Leu Ile Asn
    370                 375                 380
Gly Ile Gly Gly Ser Val Asp Phe Asn Arg Asn Ala His Leu Thr Val
385                 390                 395                 400
Val Ala Leu Pro Ala Thr Ala Ala Gly Gly Asp Val Ser Thr Val Val
                405                 410                 415
Pro Leu Ala Ser His Val Asp His Thr Glu His Asp Thr Asp Val Val
            420                 425                 430
Val Thr Glu His Gly Val Ala Asp Leu Arg Gly Lys Pro Pro Val Ala
        435                 440                 445
Arg Ala Glu Ala Ile Ile Glu Cys Ala Ala Pro Ala Phe Arg Pro Asp
    450                 455                 460
Leu Arg Ala Tyr Leu Asp Arg Ala Arg Glu Ser Ser Gly His Leu Pro
465                 470                 475                 480
His Asp Leu Ser Thr Ala Phe Asp Trp Gln Ser
                485                 490

<210> 31
<211> 1672
<212> DNA
<213> Acetobacter aceti

<220>
<221> CDS
<222> (61)..(1536)

<400> 31
aaaggcagga agccctctca tgacagagcg cattcggaac gtcgcgctgc gcagtaaagt      60

atg tcc ggc aga aac cgc atc cga gtt gat caa gca cgg aga cgt ggt      108
Met Ser Gly Arg Asn Arg Ile Arg Val Asp Gln Ala Arg Arg Arg Gly
1               5                   10                  15
ggg gac cag cgg ttt tac cgg ggc agg tta ccc caa gga agt acc caa      156
Gly Asp Gln Arg Phe Tyr Arg Gly Arg Leu Pro Gln Gly Ser Thr Gln
            20                  25                  30
ggc gct ggc cca gcg gat gga act gcg cat gat cgg ggc gaa aaa tac      204
Gly Ala Gly Pro Ala Asp Gly Thr Ala His Asp Arg Gly Glu Lys Tyr
        35                  40                  45
cag atc agc ctg att acg ggc gca tcc acg ggc cct cag ctg gat ggt      252
Gln Ile Ser Leu Ile Thr Gly Ala Ser Thr Gly Pro Gln Leu Asp Gly

50                  55                  60
gag ctg gcc aag gcc aac ggg gtg tat ttc cgc tcg cca ttc aat acg          300
Glu Leu Ala Lys Ala Asn Gly Val Tyr Phe Arg Ser Pro Phe Asn Thr
65                  70                  75                  80
gat gcc acc atg cgc aac cgt att aat gcg ggt gaa acg gaa tat ttt          348
Asp Ala Thr Met Arg Asn Arg Ile Asn Ala Gly Glu Thr Glu Tyr Phe
                85                  90                  95
gat aac cat ctg ggg cag gtg gct ggc cgt gcc gtg cag ggc aat tac          396
Asp Asn His Leu Gly Gln Val Ala Gly Arg Ala Val Gln Gly Asn Tyr
            100                 105                 110
ggc aag ttc aat att gcg ctg gtg gaa gcc aca gcc att acg gaa gat          444
Gly Lys Phe Asn Ile Ala Leu Val Glu Ala Thr Ala Ile Thr Glu Asp
        115                 120                 125
ggc ggc att gtg ccc aca tct tct gtc ggc aat tcc cag aca ttc ctc          492
Gly Gly Ile Val Pro Thr Ser Ser Val Gly Asn Ser Gln Thr Phe Leu
    130                 135                 140
aat ctg gcc gaa aag gtc att atc gag gta aat gaa tgg cag aat ccg          540
Asn Leu Ala Glu Lys Val Ile Ile Glu Val Asn Glu Trp Gln Asn Pro
145                 150                 155                 160
atg ctg gaa ggc atc cat gat atc tgg gat ggc aac gta agt ggc gtg          588
Met Leu Glu Gly Ile His Asp Ile Trp Asp Gly Asn Val Ser Gly Val
                165                 170                 175
cct acg cgt gat att gtg ccg att gta cgg gca gat cag cgc gtg ggt          636
Pro Thr Arg Asp Ile Val Pro Ile Val Arg Ala Asp Gln Arg Val Gly
            180                 185                 190
ggc ccg gtt tta cgg gtg aac ccg gac aag att gct gcc att gta cgc          684
Gly Pro Val Leu Arg Val Asn Pro Asp Lys Ile Ala Ala Ile Val Arg
        195                 200                 205
acg aat gac cgg gac aga aat gcc ccg ttt gcc gcg ccg gat gaa aca          732
Thr Asn Asp Arg Asp Arg Asn Ala Pro Phe Ala Ala Pro Asp Glu Thr
    210                 215                 220
gca aaa gct att gcg ggc tat ttg ctt gat ttc ttt ggg cat gaa gtc          780
Ala Lys Ala Ile Ala Gly Tyr Leu Leu Asp Phe Phe Gly His Glu Val
225                 230                 235                 240
aaa cag aac cgt ttg ccg ccc tca ctt ctg ccg ctc cag tct ggt gtg          828
Lys Gln Asn Arg Leu Pro Pro Ser Leu Leu Pro Leu Gln Ser Gly Val
                245                 250                 255
ggc aat gtg gcc aac gcc gtg ctg gaa ggg ctg aag gaa ggc cca ttt          876
Gly Asn Val Ala Asn Ala Val Leu Glu Gly Leu Lys Glu Gly Pro Phe
            260                 265                 270
gaa aat ctg gtg ggc tat agt gag gtg att cag gat ggc atg ttg gcc          924
Glu Asn Leu Val Gly Tyr Ser Glu Val Ile Gln Asp Gly Met Leu Ala
        275                 280                 285
atg ctg gat tct ggc cgt atg cgt att gcc tct gcc tcg tct ttc tcg          972
Met Leu Asp Ser Gly Arg Met Arg Ile Ala Ser Ala Ser Ser Phe Ser
    290                 295                 300
ctg agc ccg gaa gct gcg gaa gaa atc aat aac cgg atg gat ttc ttc          1020
Leu Ser Pro Glu Ala Ala Glu Glu Ile Asn Asn Arg Met Asp Phe Phe
305                 310                 315                 320
cgc agc aag atc att ctg cgc cag cag gat gtc agc aac agc ccc ggc          1068
Arg Ser Lys Ile Ile Leu Arg Gln Gln Asp Val Ser Asn Ser Pro Gly
                325                 330                 335
att atc cgc cgt tta ggc tgc att gcc atg aac ggc atg att gag gcg          1116
Ile Ile Arg Arg Leu Gly Cys Ile Ala Met Asn Gly Met Ile Glu Ala
            340                 345                 350
gat att tac gga aac gtg aat tct acc cgc gtg atg ggt tcc aaa atg          1164
Asp Ile Tyr Gly Asn Val Asn Ser Thr Arg Val Met Gly Ser Lys Met
        355                 360                 365
atg aat ggt att ggg gga tcg ggt gat ttt gcc cgg agt tcc tac ctg          1212
Met Asn Gly Ile Gly Gly Ser Gly Asp Phe Ala Arg Ser Ser Tyr Leu
    370                 375                 380
tcc atc ttc ctg tct ccc tcc aca gcc aaa ggt ggc aag att tca gcc          1260
Ser Ile Phe Leu Ser Pro Ser Thr Ala Lys Gly Gly Lys Ile Ser Ala
385                 390                 395                 400
att gtg ccg atg gct gcg cat gtg gac cac atc atg cag gat gcg cag          1308
Ile Val Pro Met Ala Ala His Val Asp His Ile Met Gln Asp Ala Gln
                405                 410                 415
att ttt gta acc gag cag ggg ctg gcg gat ctg cgt ggg ctt tca ccg          1356
Ile Phe Val Thr Glu Gln Gly Leu Ala Asp Leu Arg Gly Leu Ser Pro
            420                 425                 430
gtg caa cgt gcg cgt gaa att att tcc aaa tgt gcg cat ccg gat tac          1404
Val Gln Arg Ala Arg Glu Ile Ile Ser Lys Cys Ala His Pro Asp Tyr

435                 440                 445
cgg ccg atg ttg cag gat tat ttt gat cgt gcg ctt aaa aat tcc ttt      1452
Arg Pro Met Leu Gln Asp Tyr Phe Asp Arg Ala Leu Lys Asn Ser Phe
    450                 455                 460
ggt aag cac aca ccg cat ctg ctg acg gaa gct ctg tct tgg cat cag      1500
Gly Lys His Thr Pro His Leu Leu Thr Glu Ala Leu Ser Trp His Gln
465                 470                 475                 480
cgg ttt att gat acg ggc acc atg ctc cca tca taaaaaggg gtggatttcc     1553
Arg Phe Ile Asp Thr Gly Thr Met Leu Pro Ser
                485                 490
acctcttttt ttcggcttct tcccatgttt cttatgaggg tgggaggaag ccttttttac     1613

aatcagcttt tattggtggt tgtatcggcg agttgcgtgg gttgttgggt aatcatgat     1672

<210> 32
<211> 491
<212> PRT
<213> Acetobacter aceti

<400> 32
Met Ser Gly Arg Asn Arg Ile Arg Val Asp Gln Ala Arg Arg Arg Gly
1               5                   10                  15
Gly Asp Gln Arg Phe Tyr Arg Gly Arg Leu Pro Gln Gly Ser Thr Gln
            20                  25                  30
Gly Ala Gly Pro Ala Asp Gly Thr Ala His Asp Arg Gly Glu Lys Tyr
        35                  40                  45
Gln Ile Ser Leu Ile Thr Gly Ala Ser Thr Gly Pro Gln Leu Asp Gly
    50                  55                  60
Glu Leu Ala Lys Ala Asn Gly Val Tyr Phe Arg Ser Pro Phe Asn Thr
65                  70                  75                  80
Asp Ala Thr Met Arg Asn Arg Ile Asn Ala Gly Glu Thr Glu Tyr Phe
                85                  90                  95
Asp Asn His Leu Gly Gln Val Ala Gly Arg Ala Val Gln Gly Asn Tyr
            100                 105                 110
Gly Lys Phe Asn Ile Ala Leu Val Glu Ala Thr Ala Ile Thr Glu Asp
        115                 120                 125
Gly Gly Ile Val Pro Thr Ser Ser Val Gly Asn Ser Gln Thr Phe Leu
    130                 135                 140
Asn Leu Ala Glu Lys Val Ile Ile Glu Val Asn Glu Trp Gln Asn Pro
145                 150                 155                 160
Met Leu Glu Gly Ile His Asp Ile Trp Asp Gly Asn Val Ser Gly Val
                165                 170                 175
Pro Thr Arg Asp Ile Val Pro Ile Val Arg Ala Asp Gln Arg Val Gly
            180                 185                 190
Gly Pro Val Leu Arg Val Asn Pro Asp Lys Ile Ala Ala Ile Val Arg
        195                 200                 205
Thr Asn Asp Arg Asp Arg Asn Ala Pro Phe Ala Ala Pro Asp Glu Thr
    210                 215                 220
Ala Lys Ala Ile Ala Gly Tyr Leu Leu Asp Phe Phe Gly His Glu Val
225                 230                 235                 240
Lys Gln Asn Arg Leu Pro Pro Ser Leu Leu Pro Leu Gln Ser Gly Val
                245                 250                 255
Gly Asn Val Ala Asn Ala Val Leu Glu Gly Leu Lys Glu Gly Pro Phe
            260                 265                 270
Glu Asn Leu Val Gly Tyr Ser Glu Val Ile Gln Asp Gly Met Leu Ala
        275                 280                 285
Met Leu Asp Ser Gly Arg Met Arg Ile Ala Ser Ala Ser Ser Phe Ser
    290                 295                 300
Leu Ser Pro Glu Ala Ala Glu Glu Ile Asn Asn Arg Met Asp Phe Phe
305                 310                 315                 320
Arg Ser Lys Ile Ile Leu Arg Gln Gln Asp Val Ser Asn Ser Pro Gly
                325                 330                 335
Ile Ile Arg Arg Leu Gly Cys Ile Ala Met Asn Gly Met Ile Glu Ala
            340                 345                 350
Asp Ile Tyr Gly Asn Val Asn Ser Thr Arg Val Met Gly Ser Lys Met
        355                 360                 365
Met Asn Gly Ile Gly Gly Ser Gly Asp Phe Ala Arg Ser Ser Tyr Leu
    370                 375                 380

Ser Ile Phe Leu Ser Pro Ser Thr Ala Lys Gly Gly Lys Ile Ser Ala
385                 390                 395                 400
Ile Val Pro Met Ala Ala His Val Asp His Ile Met Gln Asp Ala Gln
                405                 410                 415
Ile Phe Val Thr Glu Gln Gly Leu Ala Asp Leu Arg Gly Leu Ser Pro
            420                 425                 430
Val Gln Arg Ala Arg Glu Ile Ile Ser Lys Cys Ala His Pro Asp Tyr
        435                 440                 445
Arg Pro Met Leu Gln Asp Tyr Phe Asp Arg Ala Leu Lys Asn Ser Phe
    450                 455                 460
Gly Lys His Thr Pro His Leu Leu Thr Glu Ala Leu Ser Trp His Gln
465                 470                 475                 480
Arg Phe Ile Asp Thr Gly Thr Met Leu Pro Ser
                485                 490

<210> 33
<211> 1566
<212> DNA
<213> Schizosaccharomyces pombe

<220>
<221> CDS
<222> (1)..(1566)

<400> 33
atg ccg tct ctt ctg agt tca cgc att cgc aat gcc gaa ttt gcc aaa      48
Met Pro Ser Leu Leu Ser Ser Arg Ile Arg Asn Ala Glu Phe Ala Lys
1               5                   10                  15
aaa att gtt acg gat cct gcc aag ttg gtt ccg tac ttt aaa aat ggg      96
Lys Ile Val Thr Asp Pro Ala Lys Leu Val Pro Tyr Phe Lys Asn Gly
            20                  25                  30
gac tat gtt ggt tgg tct ggt ttt act ggt gtc ggt tat cct aaa atg      144
Asp Tyr Val Gly Trp Ser Gly Phe Thr Gly Val Gly Tyr Pro Lys Met
        35                  40                  45
att cca aac gcg tta gca aga cat gtc gaa gag aac aat ttg caa gga      192
Ile Pro Asn Ala Leu Ala Arg His Val Glu Glu Asn Asn Leu Gln Gly
    50                  55                  60
aaa ttg cgt ttt aaa ttg ttc gtt ggt gcc tcg gga ggt gct gat acg      240
Lys Leu Arg Phe Lys Leu Phe Val Gly Ala Ser Gly Gly Ala Asp Thr
65                  70                  75                  80
gaa tgc aag tgg gct gaa ttg aat atg att gag cgc cgg tgt cct cat      288
Glu Cys Lys Trp Ala Glu Leu Asn Met Ile Glu Arg Arg Cys Pro His
                85                  90                  95
caa gta gga aag cca att tct aaa gga att aac tct ggc aga att cag      336
Gln Val Gly Lys Pro Ile Ser Lys Gly Ile Asn Ser Gly Arg Ile Gln
            100                 105                 110
ttt ttc gac aag cat tta tcc atg ttt ccc caa gat tta ctt tat ggc      384
Phe Phe Asp Lys His Leu Ser Met Phe Pro Gln Asp Leu Leu Tyr Gly
        115                 120                 125
tac tat act cgt aat cgt gaa agt aat aca att gac aca gcc att att      432
Tyr Tyr Thr Arg Asn Arg Glu Ser Asn Thr Ile Asp Thr Ala Ile Ile
    130                 135                 140
gag gcc aca gcc att acc gag gac ggc gga att gtt ccc ggc gca tca      480
Glu Ala Thr Ala Ile Thr Glu Asp Gly Gly Ile Val Pro Gly Ala Ser
145                 150                 155                 160
gtt ggt gcg agt ccg gaa ctt atg caa ctt gcg gaa aag att atc atc      528
Val Gly Ala Ser Pro Glu Leu Met Gln Leu Ala Glu Lys Ile Ile Ile
                165                 170                 175
gag gtg aac act gcc att cct tct ttt gaa gga ctt cat gac att gtc      576
Glu Val Asn Thr Ala Ile Pro Ser Phe Glu Gly Leu His Asp Ile Val
            180                 185                 190
gaa att ccc aac ccc cca cat cgt act cca ata aac atc aat cga gtt      624
Glu Ile Pro Asn Pro Pro His Arg Thr Pro Ile Asn Ile Asn Arg Val
        195                 200                 205
gac gat cgt att gga aag cca tac atc aaa gtg gat ccc aaa aag gtt      672
Asp Asp Arg Ile Gly Lys Pro Tyr Ile Lys Val Asp Pro Lys Lys Val
    210                 215                 220
att gga ata gtg gag gct gat tat gga gac atc aca tgc gct aac tct      720
Ile Gly Ile Val Glu Ala Asp Tyr Gly Asp Ile Thr Cys Ala Asn Ser
225                 230                 235                 240
ccg caa gat gag act tcg cag gct att gca ggc cat tta gtt gac ttt      768
Pro Gln Asp Glu Thr Ser Gln Ala Ile Ala Gly His Leu Val Asp Phe

                245                 250                 255
ttc cat cat gaa gta tct gta ggg cgt ctt ccc aaa aat ctt cat cct 816
Phe His His Glu Val Ser Val Gly Arg Leu Pro Lys Asn Leu His Pro
            260                 265                 270
ttg caa tcc ggt att gga aac att gcc aac gct att atc ggt ggt ctt 864
Leu Gln Ser Gly Ile Gly Asn Ile Ala Asn Ala Ile Ile Gly Gly Leu
        275                 280                 285
gcc cat tct ccc ttt aaa gat ctc gaa gtg tgg aca gaa gtc tta caa 912
Ala His Ser Pro Phe Lys Asp Leu Glu Val Trp Thr Glu Val Leu Gln
    290                 295                 300
gat act ttc ttg cct ctc ttt gac tct gaa aag tta cgt ttt gct act 960
Asp Thr Phe Leu Pro Leu Phe Asp Ser Glu Lys Leu Arg Phe Ala Thr
305                 310                 315                 320
gct acc tca acc cgt ttt tct cct cag gga ttt gaa aaa ttc tac tca 1008
Ala Thr Ser Thr Arg Phe Ser Pro Gln Gly Phe Glu Lys Phe Tyr Ser
                325                 330                 335
aac tat gat tac tac aag gaa aga att ttg tta cgc cct caa gta att 1056
Asn Tyr Asp Tyr Tyr Lys Glu Arg Ile Leu Leu Arg Pro Gln Val Ile
            340                 345                 350
tcc aat cac cct gaa att atc cgc cgt ctt ggt tgc atc gct atg aat 1104
Ser Asn His Pro Glu Ile Ile Arg Arg Leu Gly Cys Ile Ala Met Asn
        355                 360                 365
act cct gtg gaa gcc gat att tac gct cat gcc aat agt aca aat gtt 1152
Thr Pro Val Glu Ala Asp Ile Tyr Ala His Ala Asn Ser Thr Asn Val
    370                 375                 380
ttg gga agc aga atg ttg aac ggt tta ggt ggt tct gct gat ttc ttg 1200
Leu Gly Ser Arg Met Leu Asn Gly Leu Gly Gly Ser Ala Asp Phe Leu
385                 390                 395                 400
cgt aat gcg aaa tta agt att atg cat aca cca tcg gtt cgt cct tcg 1248
Arg Asn Ala Lys Leu Ser Ile Met His Thr Pro Ser Val Arg Pro Ser
                405                 410                 415
aag aag gat cct act ggt atc act tgt att gtt cct atg gct acc cat 1296
Lys Lys Asp Pro Thr Gly Ile Thr Cys Ile Val Pro Met Ala Thr His
            420                 425                 430
gtt gac caa acc gaa cat gat ttg gat att ttg gtt aca gag caa gga 1344
Val Asp Gln Thr Glu His Asp Leu Asp Ile Leu Val Thr Glu Gln Gly
        435                 440                 445
ttg gct gat tta cga ggc ttg tct cct acc gaa cgt gct cgt gag gtt 1392
Leu Ala Asp Leu Arg Gly Leu Ser Pro Thr Glu Arg Ala Arg Glu Val
    450                 455                 460
att gac aag tgt gct cat cct gac tat aag cct ctg ctc aga gag tat 1440
Ile Asp Lys Cys Ala His Pro Asp Tyr Lys Pro Leu Leu Arg Glu Tyr
465                 470                 475                 480
ttc gat att gcc gaa aac tac tgt tta gct aga gga gct ggt cat gaa 1488
Phe Asp Ile Ala Glu Asn Tyr Cys Leu Ala Arg Gly Ala Gly His Glu
                485                 490                 495
ccc cat att ctt gcc aat gct ttc aag atg cag ctc aac ctt tta gaa 1536
Pro His Ile Leu Ala Asn Ala Phe Lys Met Gln Leu Asn Leu Leu Glu
            500                 505                 510
aaa gga acg atg aag atc gat cat tgg taa 1566
Lys Gly Thr Met Lys Ile Asp His Trp
        515                 520

<210> 34
<211> 521
<212> PRT
<213> Schizosaccharomyces pombe

<400> 34
Met Pro Ser Leu Leu Ser Ser Arg Ile Arg Asn Ala Glu Phe Ala Lys
1               5                   10                  15
Lys Ile Val Thr Asp Pro Ala Lys Leu Val Pro Tyr Phe Lys Asn Gly
            20                  25                  30
Asp Tyr Val Gly Trp Ser Gly Phe Thr Gly Val Gly Tyr Pro Lys Met
        35                  40                  45
Ile Pro Asn Ala Leu Ala Arg His Val Glu Glu Asn Asn Leu Gln Gly
    50                  55                  60
Lys Leu Arg Phe Lys Leu Phe Val Gly Ala Ser Gly Gly Ala Asp Thr
65                  70                  75                  80
Glu Cys Lys Trp Ala Glu Leu Asn Met Ile Glu Arg Arg Cys Pro His
                85                  90                  95

Gln Val Gly Lys Pro Ile Ser Lys Gly Ile Asn Ser Gly Arg Ile Gln
            100                 105                 110
Phe Phe Asp Lys His Leu Ser Met Phe Pro Gln Asp Leu Leu Tyr Gly
        115                 120                 125
Tyr Tyr Thr Arg Asn Arg Glu Ser Asn Thr Ile Asp Thr Ala Ile Ile
    130                 135                 140
Glu Ala Thr Ala Ile Thr Glu Asp Gly Gly Ile Val Pro Gly Ala Ser
145                 150                 155                 160
Val Gly Ala Ser Pro Glu Leu Met Gln Leu Ala Glu Lys Ile Ile Ile
                165                 170                 175
Glu Val Asn Thr Ala Ile Pro Ser Phe Glu Gly Leu His Asp Ile Val
            180                 185                 190
Glu Ile Pro Asn Pro Pro His Arg Thr Pro Ile Asn Ile Asn Arg Val
        195                 200                 205
Asp Asp Arg Ile Gly Lys Pro Tyr Ile Lys Val Asp Pro Lys Lys Val
    210                 215                 220
Ile Gly Ile Val Glu Ala Asp Tyr Gly Asp Ile Thr Cys Ala Asn Ser
225                 230                 235                 240
Pro Gln Asp Glu Thr Ser Gln Ala Ile Ala Gly His Leu Val Asp Phe
                245                 250                 255
Phe His His Glu Val Ser Val Gly Arg Leu Pro Lys Asn Leu His Pro
            260                 265                 270
Leu Gln Ser Gly Ile Gly Asn Ile Ala Asn Ala Ile Ile Gly Gly Leu
        275                 280                 285
Ala His Ser Pro Phe Lys Asp Leu Glu Val Trp Thr Glu Val Leu Gln
    290                 295                 300
Asp Thr Phe Leu Pro Leu Phe Asp Ser Glu Lys Leu Arg Phe Ala Thr
305                 310                 315                 320
Ala Thr Ser Thr Arg Phe Ser Pro Gln Gly Phe Glu Lys Phe Tyr Ser
                325                 330                 335
Asn Tyr Asp Tyr Tyr Lys Glu Arg Ile Leu Leu Arg Pro Gln Val Ile
            340                 345                 350
Ser Asn His Pro Glu Ile Ile Arg Arg Leu Gly Cys Ile Ala Met Asn
        355                 360                 365
Thr Pro Val Glu Ala Asp Ile Tyr Ala His Ala Asn Ser Thr Asn Val
    370                 375                 380
Leu Gly Ser Arg Met Leu Asn Gly Leu Gly Gly Ser Ala Asp Phe Leu
385                 390                 395                 400
Arg Asn Ala Lys Leu Ser Ile Met His Thr Pro Ser Val Arg Pro Ser
                405                 410                 415
Lys Lys Asp Pro Thr Gly Ile Thr Cys Ile Val Pro Met Ala Thr His
            420                 425                 430
Val Asp Gln Thr Glu His Asp Leu Asp Ile Leu Val Thr Glu Gln Gly
        435                 440                 445
Leu Ala Asp Leu Arg Gly Leu Ser Pro Thr Glu Arg Ala Arg Glu Val
    450                 455                 460
Ile Asp Lys Cys Ala His Pro Asp Tyr Lys Pro Leu Leu Arg Glu Tyr
465                 470                 475                 480
Phe Asp Ile Ala Glu Asn Tyr Cys Leu Ala Arg Gly Ala Gly His Glu
                485                 490                 495
Pro His Ile Leu Ala Asn Ala Phe Lys Met Gln Leu Asn Leu Leu Glu
            500                 505                 510
Lys Gly Thr Met Lys Ile Asp His Trp
        515                 520

<210> 35
<211> 1857
<212> DNA
<213> Clostridium thermosaccharolyticum, Clostridium tartarivorum

<220>
<221> CDS
<222> (1)..(1497)

<400> 35
atg aat ttt gaa gag cgg att aga tgc aat ata att aaa aat aaa gta      48
Met Asn Phe Glu Glu Arg Ile Arg Cys Asn Ile Ile Lys Asn Lys Val
1               5                   10                  15
atg tcg gca gaa gaa gca gct tta ttg ata aaa gat ggt atg aca ata      96
Met Ser Ala Glu Glu Ala Ala Leu Leu Ile Lys Asp Gly Met Thr Ile
            20                  25                  30
ggt aca agt ggt ttt gta aca ggt ggt cct aaa gct gta cca ttg gca      144

Gly Thr Ser Gly Phe Val Thr Gly Gly Pro Lys Ala Val Pro Leu Ala
        35                  40                  45
att gct gaa aga tat aaa aat gaa aag ata aag tta aat ctt tat aca 192
Ile Ala Glu Arg Tyr Lys Asn Glu Lys Ile Lys Leu Asn Leu Tyr Thr
    50                  55                  60
ggt gct ata gta ggt gat gaa tta gat ggt gct ttc gca cgg gaa ggt 240
Gly Ala Ile Val Gly Asp Glu Leu Asp Gly Ala Phe Ala Arg Glu Gly
65                  70                  75                  80
tta gtc aat aaa aga att ccg tat caa aca aat aaa gac atg aga aat 288
Leu Val Asn Lys Arg Ile Pro Tyr Gln Thr Asn Lys Asp Met Arg Asn
                85                  90                  95
gct ata aat aat ggt tca gtt aaa ttt att gat atg cat cta agt cat 336
Ala Ile Asn Asn Gly Ser Val Lys Phe Ile Asp Met His Leu Ser His
            100                 105                 110
atg cca cag caa ata aga tat ggt ttt tta gga aaa att gat atc gcc 384
Met Pro Gln Gln Ile Arg Tyr Gly Phe Leu Gly Lys Ile Asp Ile Ala
        115                 120                 125
att att gaa gca act gct ata aca gaa gat gga ggc att att cct acc 432
Ile Ile Glu Ala Thr Ala Ile Thr Glu Asp Gly Gly Ile Ile Pro Thr
    130                 135                 140
atg tct gtc ggt aat tca ccg aca ttt gta aat atg gcc gat aaa gtc 480
Met Ser Val Gly Asn Ser Pro Thr Phe Val Asn Met Ala Asp Lys Val
145                 150                 155                 160
ata gta gaa ata aat aca tca cag cct att gaa ctt gaa gga atg cac 528
Ile Val Glu Ile Asn Thr Ser Gln Pro Ile Glu Leu Glu Gly Met His
                165                 170                 175
gat ata tac gag cca aaa gat cca cca tat aga gag ccg ata cca ata 576
Asp Ile Tyr Glu Pro Lys Asp Pro Pro Tyr Arg Glu Pro Ile Pro Ile
            180                 185                 190
tcg aaa gtt agt gat agg ata ggt gtt ccg tat att cca tgc aat cca 624
Ser Lys Val Ser Asp Arg Ile Gly Val Pro Tyr Ile Pro Cys Asn Pro
        195                 200                 205
gat aaa ata gct gca gtt gta ttt tca gat atg aaa gat aca acg tta 672
Asp Lys Ile Ala Ala Val Val Phe Ser Asp Met Lys Asp Thr Thr Leu
    210                 215                 220
caa ctt aca agt ata aat gaa aca tca aaa aag att tca gat tat ctt 720
Gln Leu Thr Ser Ile Asn Glu Thr Ser Lys Lys Ile Ser Asp Tyr Leu
225                 230                 235                 240
ata gat ttt ctt caa act gaa ata aaa cac gga aga ctt cct aaa aca 768
Ile Asp Phe Leu Gln Thr Glu Ile Lys His Gly Arg Leu Pro Lys Thr
                245                 250                 255
ttt ctt cca ttg cag tca ggt att gga aat gta gcc aat gct gta cta 816
Phe Leu Pro Leu Gln Ser Gly Ile Gly Asn Val Ala Asn Ala Val Leu
            260                 265                 270
ggc ggt ttt att gaa tta aaa tac aac aac ttg aat tta tat tct gaa 864
Gly Gly Phe Ile Glu Leu Lys Tyr Asn Asn Leu Asn Leu Tyr Ser Glu
        275                 280                 285
gta ata caa gat gct gtt ctc gat ttg ata gat gct gat aag gta gaa 912
Val Ile Gln Asp Ala Val Leu Asp Leu Ile Asp Ala Asp Lys Val Glu
    290                 295                 300
aaa gta tca gga act gca ttg acc ctt tca gaa gaa gga ctt aag aga 960
Lys Val Ser Gly Thr Ala Leu Thr Leu Ser Glu Glu Gly Leu Lys Arg
305                 310                 315                 320
ttt tat acc aat ata aat aaa tat aaa gac aaa ata ata ttg cgt cca 1008
Phe Tyr Thr Asn Ile Asn Lys Tyr Lys Asp Lys Ile Ile Leu Arg Pro
                325                 330                 335
caa gag ctc agt aat aat cct gaa ata atc aga aga tta gga gtt ata 1056
Gln Glu Leu Ser Asn Asn Pro Glu Ile Ile Arg Arg Leu Gly Val Ile
            340                 345                 350
tca att aac aca gca ata gaa gtg gat ata tac ggt aat gta aat tct 1104
Ser Ile Asn Thr Ala Ile Glu Val Asp Ile Tyr Gly Asn Val Asn Ser
        355                 360                 365
aca cat ata tta ggc aca aaa atg atg aac ggc ata ggt ggt tca gca 1152
Thr His Ile Leu Gly Thr Lys Met Met Asn Gly Ile Gly Gly Ser Ala
    370                 375                 380
gat ttt act aga aat gct tat ata tca att ttt gct acg cca tca aca 1200
Asp Phe Thr Arg Asn Ala Tyr Ile Ser Ile Phe Ala Thr Pro Ser Thr
385                 390                 395                 400
gca aaa gat gat aat att tca tgc ata gtg ccg atg gta tca cat gta 1248
Ala Lys Asp Asp Asn Ile Ser Cys Ile Val Pro Met Val Ser His Val
                405                 410                 415
gat cat aca gag cat gat gtc atg gtg gta gta act gag caa gga ctg 1296

Asp His Thr Glu His Asp Val Met Val Val Val Thr Glu Gln Gly Leu
            420                 425                 430
gct gat tta aga ggt tta agt ccg aag gaa aga gct ata gca att ata 1344
Ala Asp Leu Arg Gly Leu Ser Pro Lys Glu Arg Ala Ile Ala Ile Ile
        435                 440                 445
aaa aat tgt gca cat ccc gac tat aaa gac gtt tta atg gag tat tat 1392
Lys Asn Cys Ala His Pro Asp Tyr Lys Asp Val Leu Met Glu Tyr Tyr
    450                 455                 460
gaa aga gcg att aaa aaa cat ggc gct gtg caa aca ccg aat tta ctc 1440
Glu Arg Ala Ile Lys Lys His Gly Ala Val Gln Thr Pro Asn Leu Leu
465                 470                 475                 480
gat gaa gca ttt tta tgg cat aca aga tat tca aat aca ggt agt atg 1488
Asp Glu Ala Phe Leu Trp His Thr Arg Tyr Ser Asn Thr Gly Ser Met
                485                 490                 495
aaa ata tgataaattt atattattac ctgaaaatta tagttgttag tcttcatttc 1544
Lys Ile

taaattaaag tgaattatat ttttataaaa gattttaaaa aacttttatc gtaaaagcat 1604

aaaagagaga gtcaaccagt ttaatacgga tgaagttgat ttccatgtaa gtcgtgacaa 1664

aaaattgaat aatgataaaa aaatataaag aagcttagaa taagaaaatt actgaactat 1724

ctacgataga tatttgattt ctttataaat attttttaac aaaaggcttg aaagaagtct 1784

cccatcctcc ataggtggga gatgaatttc tatatttttt gtagggaagg ttcctcccga 1844

agttacgcct atg 1857

<210> 36
<211> 498
<212> PRT
<213> Clostridium thermosaccharolyticum, Clostridium tartarivorum

<400> 36
Met Asn Phe Glu Glu Arg Ile Arg Cys Asn Ile Ile Lys Asn Lys Val
1               5                   10                  15
Met Ser Ala Glu Glu Ala Ala Leu Leu Ile Lys Asp Gly Met Thr Ile
            20                  25                  30
Gly Thr Ser Gly Phe Val Thr Gly Gly Pro Lys Ala Val Pro Leu Ala
        35                  40                  45
Ile Ala Glu Arg Tyr Lys Asn Glu Lys Ile Lys Leu Asn Leu Tyr Thr
    50                  55                  60
Gly Ala Ile Val Gly Asp Glu Leu Asp Gly Ala Phe Ala Arg Glu Gly
65                  70                  75                  80
Leu Val Asn Lys Arg Ile Pro Tyr Gln Thr Asn Lys Asp Met Arg Asn
                85                  90                  95
Ala Ile Asn Asn Gly Ser Val Lys Phe Ile Asp Met His Leu Ser His
            100                 105                 110
Met Pro Gln Gln Ile Arg Tyr Gly Phe Leu Gly Lys Ile Asp Ile Ala
        115                 120                 125
Ile Ile Glu Ala Thr Ala Ile Thr Glu Asp Gly Gly Ile Ile Pro Thr
    130                 135                 140
Met Ser Val Gly Asn Ser Pro Thr Phe Val Asn Met Ala Asp Lys Val
145                 150                 155                 160
Ile Val Glu Ile Asn Thr Ser Gln Pro Ile Glu Leu Glu Gly Met His
                165                 170                 175
Asp Ile Tyr Glu Pro Lys Asp Pro Pro Tyr Arg Glu Pro Ile Pro Ile
            180                 185                 190
Ser Lys Val Ser Asp Arg Ile Gly Val Pro Tyr Ile Pro Cys Asn Pro
        195                 200                 205
Asp Lys Ile Ala Ala Val Val Phe Ser Asp Met Lys Asp Thr Thr Leu
    210                 215                 220
Gln Leu Thr Ser Ile Asn Glu Thr Ser Lys Lys Ile Ser Asp Tyr Leu

225                 230                 235                 240
Ile Asp Phe Leu Gln Thr Glu Ile Lys His Gly Arg Leu Pro Lys Thr
                245                 250                 255
Phe Leu Pro Leu Gln Ser Gly Ile Gly Asn Val Ala Asn Ala Val Leu
            260                 265                 270
Gly Gly Phe Ile Glu Leu Lys Tyr Asn Asn Leu Asn Leu Tyr Ser Glu
        275                 280                 285
Val Ile Gln Asp Ala Val Leu Asp Leu Ile Asp Ala Asp Lys Val Glu
    290                 295                 300
Lys Val Ser Gly Thr Ala Leu Thr Leu Ser Glu Glu Gly Leu Lys Arg
305                 310                 315                 320
Phe Tyr Thr Asn Ile Asn Lys Tyr Lys Asp Lys Ile Ile Leu Arg Pro
                325                 330                 335
Gln Glu Leu Ser Asn Asn Pro Glu Ile Ile Arg Arg Leu Gly Val Ile
            340                 345                 350
Ser Ile Asn Thr Ala Ile Glu Val Asp Ile Tyr Gly Asn Val Asn Ser
        355                 360                 365
Thr His Ile Leu Gly Thr Lys Met Met Asn Gly Ile Gly Gly Ser Ala
    370                 375                 380
Asp Phe Thr Arg Asn Ala Tyr Ile Ser Ile Phe Ala Thr Pro Ser Thr
385                 390                 395                 400
Ala Lys Asp Asp Asn Ile Ser Cys Ile Val Pro Met Val Ser His Val
                405                 410                 415
Asp His Thr Glu His Asp Val Met Val Val Val Thr Glu Gln Gly Leu
            420                 425                 430
Ala Asp Leu Arg Gly Leu Ser Pro Lys Glu Arg Ala Ile Ala Ile Ile
        435                 440                 445
Lys Asn Cys Ala His Pro Asp Tyr Lys Asp Val Leu Met Glu Tyr Tyr
    450                 455                 460
Glu Arg Ala Ile Lys Lys His Gly Ala Val Gln Thr Pro Asn Leu Leu
465                 470                 475                 480
Asp Glu Ala Phe Leu Trp His Thr Arg Tyr Ser Asn Thr Gly Ser Met
                485                 490                 495
Lys Ile

<210> 37
<211> 1494
<212> DNA
<213> Pseudomonas aeruginosa

<220>
<221> CDS
<222> (1)..(1494)

<400> 37
atg tac cgt gat cgc gtg cgc ctg ccc tcc ctg ctc gac aag gtg atg      48
Met Tyr Arg Asp Arg Val Arg Leu Pro Ser Leu Leu Asp Lys Val Met
1               5                   10                  15
agt gcg gcc gaa gcc gcc gac ctg atc cag gac ggc atg acc gtc ggc      96
Ser Ala Ala Glu Ala Ala Asp Leu Ile Gln Asp Gly Met Thr Val Gly
            20                  25                  30
atg agc ggt ttc acc cgc gcc ggc gag gcc aag gcc gta ccc cag gcg      144
Met Ser Gly Phe Thr Arg Ala Gly Glu Ala Lys Ala Val Pro Gln Ala
        35                  40                  45
ctg gcc atg cgc gcc aag gaa cgt ccg ctg cgt atc agc ctg atg acc      192
Leu Ala Met Arg Ala Lys Glu Arg Pro Leu Arg Ile Ser Leu Met Thr
    50                  55                  60
ggc gcc agc ctc ggc aac gac ctc gac aag cag ctc acc gag gcc ggc      240
Gly Ala Ser Leu Gly Asn Asp Leu Asp Lys Gln Leu Thr Glu Ala Gly
65                  70                  75                  80
gtg ctc gcc cgg cgc atg ccg ttc cag gtc gac agc acc ctg cgc aag      288
Val Leu Ala Arg Arg Met Pro Phe Gln Val Asp Ser Thr Leu Arg Lys
                85                  90                  95
gcg atc aac gcc ggc gaa gtg atg ttc atc gac cag cac ctc tcg gaa      336
Ala Ile Asn Ala Gly Glu Val Met Phe Ile Asp Gln His Leu Ser Glu
            100                 105                 110
acc gtc gag cag ttg cgc aac cac cag ctg aag ctg ccg gac atc gcc      384
Thr Val Glu Gln Leu Arg Asn His Gln Leu Lys Leu Pro Asp Ile Ala
        115                 120                 125
gtc atc gaa gcc gcc gcg atc acc gag cag ggc cac atc gtg ccg acc      432
Val Ile Glu Ala Ala Ala Ile Thr Glu Gln Gly His Ile Val Pro Thr

130                 135                 140
acc tcg gtg ggc aac tcg gcg agc ttc gcg atc ttc gcc aag cag gtc     480
Thr Ser Val Gly Asn Ser Ala Ser Phe Ala Ile Phe Ala Lys Gln Val
145                 150                 155                 160
atc gtc gag atc aac ctg gcc cac agc acc aac ctg gaa ggc ctg cac     528
Ile Val Glu Ile Asn Leu Ala His Ser Thr Asn Leu Glu Gly Leu His
                165                 170                 175
gac atc tat atc ccg acc tac cgg ccg acc cgc acg ccg atc ccg ctg     576
Asp Ile Tyr Ile Pro Thr Tyr Arg Pro Thr Arg Thr Pro Ile Pro Leu
            180                 185                 190
acc cgc gtc gac gac cgc atc ggc agc acg gcg atc ccg atc ccg ccg     624
Thr Arg Val Asp Asp Arg Ile Gly Ser Thr Ala Ile Pro Ile Pro Pro
        195                 200                 205
gag aag atc gtc gcc atc gtc atc aac gac cag ccg gac tcg cca tcc     672
Glu Lys Ile Val Ala Ile Val Ile Asn Asp Gln Pro Asp Ser Pro Ser
    210                 215                 220
acc gtg ctg ccg ccg gac ggc gag acc cag gcc atc gcc aac cac ctg     720
Thr Val Leu Pro Pro Asp Gly Glu Thr Gln Ala Ile Ala Asn His Leu
225                 230                 235                 240
atc gat ttc ttc aag cgc gag gtc gac gcc ggg cgc atg agc aac agc     768
Ile Asp Phe Phe Lys Arg Glu Val Asp Ala Gly Arg Met Ser Asn Ser
                245                 250                 255
ctc ggc ccg ctg cag gcc ggt ttc ggc agc atc ccc aac gcg gtg atg     816
Leu Gly Pro Leu Gln Ala Gly Phe Gly Ser Ile Pro Asn Ala Val Met
            260                 265                 270
tgc ggc ctg atc gag tcg ccg ttc gag aac ctg acc atg tac tcc gaa     864
Cys Gly Leu Ile Glu Ser Pro Phe Glu Asn Leu Thr Met Tyr Ser Glu
        275                 280                 285
gtg ctg cag gac tcg acc ttc gac ctg atc gac gcc ggc aag ctg cgc     912
Val Leu Gln Asp Ser Thr Phe Asp Leu Ile Asp Ala Gly Lys Leu Arg
    290                 295                 300
ttc gcc tcg ggc agc tcg atc acc ctg tcg ccg cgg cgc aac gcc gac     960
Phe Ala Ser Gly Ser Ser Ile Thr Leu Ser Pro Arg Arg Asn Ala Asp
305                 310                 315                 320
gtg ttc ggt aac ctg gag cgc tac aag gac aag ctg gtg ctg cgc ccg     1008
Val Phe Gly Asn Leu Glu Arg Tyr Lys Asp Lys Leu Val Leu Arg Pro
                325                 330                 335
cag gaa atc tcc aac cac ccc gag gtg gtc cgt cgc ctg ggc atc atc     1056
Gln Glu Ile Ser Asn His Pro Glu Val Val Arg Arg Leu Gly Ile Ile
            340                 345                 350
ggc atc aat acg gcg ctg gag ttc gac atc tac ggc aac gtc aac tcg     1104
Gly Ile Asn Thr Ala Leu Glu Phe Asp Ile Tyr Gly Asn Val Asn Ser
        355                 360                 365
acc cac gtc ggc ggc acc aag atg atg aac ggc atc ggc ggc tcc ggc     1152
Thr His Val Gly Gly Thr Lys Met Met Asn Gly Ile Gly Gly Ser Gly
    370                 375                 380
gac ttc gcc cgc aac gcg cac ctg gcg atc ttc gtc acc aag tcg atc     1200
Asp Phe Ala Arg Asn Ala His Leu Ala Ile Phe Val Thr Lys Ser Ile
385                 390                 395                 400
gcc aag ggc ggc aac att tcc agc gtg gtg ccg atg gtc agc cac gtc     1248
Ala Lys Gly Gly Asn Ile Ser Ser Val Val Pro Met Val Ser His Val
                405                 410                 415
gac cac acc gag cac gac gtc gac atc ctc gtc acc gag cag ggc ctc     1296
Asp His Thr Glu His Asp Val Asp Ile Leu Val Thr Glu Gln Gly Leu
            420                 425                 430
gcc gac ctg cgc ggc ctg gcg cct cgc gaa cgg gcc cgg gtg atc atc     1344
Ala Asp Leu Arg Gly Leu Ala Pro Arg Glu Arg Ala Arg Val Ile Ile
        435                 440                 445
gag aac tgt gtc cac ccg tcc tac cag gca ccg ctg ctg gac tac ttc     1392
Glu Asn Cys Val His Pro Ser Tyr Gln Ala Pro Leu Leu Asp Tyr Phe
    450                 455                 460
gag gcg gct tgc gcg aaa ggc ggc cac acg ccg cat ctg ttg cgc gag     1440
Glu Ala Ala Cys Ala Lys Gly Gly His Thr Pro His Leu Leu Arg Glu
465                 470                 475                 480
gcg ctg gcc tgg cac ctg aac ctg gaa gaa cgc gga cac atg ctg gcc     1488
Ala Leu Ala Trp His Leu Asn Leu Glu Glu Arg Gly His Met Leu Ala
                485                 490                 495
ggc tga                                                             1494
Gly

PF 55709

<210> 38
<211> 497
<212> PRT
<213> Pseudomonas aeruginosa

<400> 38
Met Tyr Arg Asp Arg Val Arg Leu Pro Ser Leu Leu Asp Lys Val Met
1               5                   10                  15
Ser Ala Ala Glu Ala Ala Asp Leu Ile Gln Asp Gly Met Thr Val Gly
            20                  25                  30
Met Ser Gly Phe Thr Arg Ala Gly Glu Ala Lys Ala Val Pro Gln Ala
        35                  40                  45
Leu Ala Met Arg Ala Lys Glu Arg Pro Leu Arg Ile Ser Leu Met Thr
    50                  55                  60
Gly Ala Ser Leu Gly Asn Asp Leu Asp Lys Gln Leu Thr Glu Ala Gly
65                  70                  75                  80
Val Leu Ala Arg Arg Met Pro Phe Gln Val Asp Ser Thr Leu Arg Lys
                85                  90                  95
Ala Ile Asn Ala Gly Glu Val Met Phe Ile Asp Gln His Leu Ser Glu
            100                 105                 110
Thr Val Glu Gln Leu Arg Asn His Gln Leu Lys Leu Pro Asp Ile Ala
        115                 120                 125
Val Ile Glu Ala Ala Ala Ile Thr Glu Gln Gly His Ile Val Pro Thr
    130                 135                 140
Thr Ser Val Gly Asn Ser Ala Ser Phe Ala Ile Phe Ala Lys Gln Val
145                 150                 155                 160
Ile Val Glu Ile Asn Leu Ala His Ser Thr Asn Leu Glu Gly Leu His
                165                 170                 175
Asp Ile Tyr Ile Pro Thr Tyr Arg Pro Thr Arg Thr Pro Ile Pro Leu
            180                 185                 190
Thr Arg Val Asp Asp Arg Ile Gly Ser Thr Ala Ile Pro Ile Pro Pro
        195                 200                 205
Glu Lys Ile Val Ala Ile Val Ile Asn Asp Gln Pro Asp Ser Pro Ser
    210                 215                 220
Thr Val Leu Pro Pro Asp Gly Glu Thr Gln Ala Ile Ala Asn His Leu
225                 230                 235                 240
Ile Asp Phe Phe Lys Arg Glu Val Asp Ala Gly Arg Met Ser Asn Ser
                245                 250                 255
Leu Gly Pro Leu Gln Ala Gly Phe Gly Ser Ile Pro Asn Ala Val Met
            260                 265                 270
Cys Gly Leu Ile Glu Ser Pro Phe Glu Asn Leu Thr Met Tyr Ser Glu
        275                 280                 285
Val Leu Gln Asp Ser Thr Phe Asp Leu Ile Asp Ala Gly Lys Leu Arg
    290                 295                 300
Phe Ala Ser Gly Ser Ser Ile Thr Leu Ser Pro Arg Arg Asn Ala Asp
305                 310                 315                 320
Val Phe Gly Asn Leu Glu Arg Tyr Lys Asp Lys Leu Val Leu Arg Pro
                325                 330                 335
Gln Glu Ile Ser Asn His Pro Glu Val Val Arg Arg Leu Gly Ile Ile
            340                 345                 350
Gly Ile Asn Thr Ala Leu Glu Phe Asp Ile Tyr Gly Asn Val Asn Ser
        355                 360                 365
Thr His Val Gly Gly Thr Lys Met Met Asn Gly Ile Gly Gly Ser Gly
    370                 375                 380
Asp Phe Ala Arg Asn Ala His Leu Ala Ile Phe Val Thr Lys Ser Ile
385                 390                 395                 400
Ala Lys Gly Gly Asn Ile Ser Ser Val Val Pro Met Val Ser His Val
                405                 410                 415
Asp His Thr Glu His Asp Val Asp Ile Leu Val Thr Glu Gln Gly Leu
            420                 425                 430
Ala Asp Leu Arg Gly Leu Ala Pro Arg Glu Arg Ala Arg Val Ile Ile
        435                 440                 445
Glu Asn Cys Val His Pro Ser Tyr Gln Ala Pro Leu Leu Asp Tyr Phe
    450                 455                 460
Glu Ala Ala Cys Ala Lys Gly Gly His Thr Pro His Leu Leu Arg Glu
465                 470                 475                 480
Ala Leu Ala Trp His Leu Asn Leu Glu Glu Arg Gly His Met Leu Ala
                485                 490                 495
Gly

<210> 39

<211> 1912
<212> DNA
<213> Porphyromonas gingivalis

<220>
<221> CDS
<222> (136)..(1431)

<400> 39
tttgagtctt ccaaaaagat gttgtagaaa gatttaccat agcgcgaaaa agttatttta     60

gtttccactt gagtggattt acctttcata ctttattcat cctttttaat aaaaatcatc     120

tgtaagaata ttact atg caa tgg caa gaa ctt tac cgt cag cgc gtt tgc     171
                 Met Gln Trp Gln Glu Leu Tyr Arg Gln Arg Val Cys
                 1               5                   10
tct gca gac gaa gct gtc gtg gac tct ctt aaa ccg gga acg aaa gtt     219
Ser Ala Asp Glu Ala Val Val Asp Ser Leu Lys Pro Gly Thr Lys Val
        15                  20                  25
gta ttc ggt cat gct gct gct gcg cct gtc cgt ttc tct cag gct atg     267
Val Phe Gly His Ala Ala Ala Ala Pro Val Arg Phe Ser Gln Ala Met
    30                  35                  40
tac cgc cag cgt gaa aag ttg gag aat atc aca gtt ttc cac atg ttg     315
Tyr Arg Gln Arg Glu Lys Leu Glu Asn Ile Thr Val Phe His Met Leu
45                  50                  55                  60
tat ttc ggc gac gcg ccg cac ctt gct ccc gaa atg cgt tcg cat gta     363
Tyr Phe Gly Asp Ala Pro His Leu Ala Pro Glu Met Arg Ser His Val
                65                  70                  75
cac ccg act ctc aac ttc ctt gag ggc aac tcc cgt ccg gca agc cgt     411
His Pro Thr Leu Asn Phe Leu Glu Gly Asn Ser Arg Pro Ala Ser Arg
            80                  85                  90
gac cgt cgt gtc gat ttc att ccc tgc cac ttc cac gag gta ccg gaa     459
Asp Arg Arg Val Asp Phe Ile Pro Cys His Phe His Glu Val Pro Glu
        95                  100                 105
ctg ttt cgt cag gga ttc ttt cca ttg gat gta gcc gta gtg cag gta     507
Leu Phe Arg Gln Gly Phe Phe Pro Leu Asp Val Ala Val Val Gln Val
    110                 115                 120
tct act cct aac gaa gag ggt tat tgc tct ttc gga gtt tcc tgc gac     555
Ser Thr Pro Asn Glu Glu Gly Tyr Cys Ser Phe Gly Val Ser Cys Asp
125                 130                 135                 140
tac aca aag gct gcc gcc gag tgc gct ccg gta gta gta gcc gag gtg     603
Tyr Thr Lys Ala Ala Ala Glu Cys Ala Pro Val Val Val Ala Glu Val
                145                 150                 155
aac aag caa atg cca ttc atc ggt ggt gaa aac ctg att cac atc tcc     651
Asn Lys Gln Met Pro Phe Ile Gly Gly Glu Asn Leu Ile His Ile Ser
            160                 165                 170
aaa ctg acc cat atc atc gaa gtg gac gag ccg att gca gaa gta ttg     699
Lys Leu Thr His Ile Ile Glu Val Asp Glu Pro Ile Ala Glu Val Leu
        175                 180                 185
cct cct gct atc agc gac ctt gaa ctg agg ata ggt cag aat tgt gcc     747
Pro Pro Ala Ile Ser Asp Leu Glu Leu Arg Ile Gly Gln Asn Cys Ala
    190                 195                 200
tca ctg atc aaa gac ggc gat acc ctc cag ttg ggt atc ggc ggt atc     795
Ser Leu Ile Lys Asp Gly Asp Thr Leu Gln Leu Gly Ile Gly Gly Ile
205                 210                 215                 220
ccc gac gct gtg ttg cgt gca ttg gaa ggg cat aaa gat ctc ggt att     843
Pro Asp Ala Val Leu Arg Ala Leu Glu Gly His Lys Asp Leu Gly Ile
                225                 230                 235
cac acg gaa atg ttt acc gac ggg gtg atg cgt atg att cgc aag ggg     891
His Thr Glu Met Phe Thr Asp Gly Val Met Arg Met Ile Arg Lys Gly
            240                 245                 250
att atc aac ggg aag aaa aaa aca ttg cat ccc gaa aaa gtc gtt acc     939
Ile Ile Asn Gly Lys Lys Lys Thr Leu His Pro Glu Lys Val Val Thr
        255                 260                 265
tcg cta atc ttc gga tcg aaa gaa ttg tac gat ttt gtc aat aac aat     987
Ser Leu Ile Phe Gly Ser Lys Glu Leu Tyr Asp Phe Val Asn Asn Asn
    270                 275                 280
ccg gtg ata gaa tgc tat ccg gtg gat tat atc aac aac ccc gat gtt     1035
Pro Val Ile Glu Cys Tyr Pro Val Asp Tyr Ile Asn Asn Pro Asp Val
285                 290                 295                 300

atc ggt aag aat gac cgc atg gtt tct atc aat tcc tgc ttg gag atg 1083
Ile Gly Lys Asn Asp Arg Met Val Ser Ile Asn Ser Cys Leu Glu Met
                305                 310                 315
gat ctc atg ggg cag gca gct tct gag tcg atc ggg tac gaa cag ttc 1131
Asp Leu Met Gly Gln Ala Ala Ser Glu Ser Ile Gly Tyr Glu Gln Phe
            320                 325                 330
agt gga tcc gga ggt caa gtc gat ttc ctt cgt ggg gcc aag cgt tcc 1179
Ser Gly Ser Gly Gly Gln Val Asp Phe Leu Arg Gly Ala Lys Arg Ser
        335                 340                 345
aag gga gga atc tcc att atg gct ttc ccc agt acg gcc aag aaa ggg 1227
Lys Gly Gly Ile Ser Ile Met Ala Phe Pro Ser Thr Ala Lys Lys Gly
    350                 355                 360
act gag agt cgc atc gtt ccc att ctg aaa gag ggt gct tgt gtc acg 1275
Thr Glu Ser Arg Ile Val Pro Ile Leu Lys Glu Gly Ala Cys Val Thr
365                 370                 375                 380
acc ggc cgt aac gaa gtg gac tat gtg gtg acg gaa tat ggc gta gcg 1323
Thr Gly Arg Asn Glu Val Asp Tyr Val Val Thr Glu Tyr Gly Val Ala
                385                 390                 395
cgt ctg cgt ggc gca acg ctt cgt cag cgt gct gaa gcc ttg act gct 1371
Arg Leu Arg Gly Ala Thr Leu Arg Gln Arg Ala Glu Ala Leu Thr Ala
            400                 405                 410
ata gca cat ccc gat ttc cga ccg gcc ctt gag gag gaa atc cgc cga 1419
Ile Ala His Pro Asp Phe Arg Pro Ala Leu Glu Glu Glu Ile Arg Arg
        415                 420                 425
cgc ttc gaa taagtcggaa ggattttagg ccttatataa acggattcga agccatgtac 1478
Arg Phe Glu
    430
aactacattg tgtacatggc ttcgtatgtt taacgtgagt tcaatgtaag cgatatggtg 1538

ccgattctct cttgatggga tattgactat aaccgaatca gaaaatgaaa gggaaaaagt 1598

ttgacgtctc atcggacgtc ggagcctctc gaagctgaaa cctctcaaca gacaaatgaa 1658

atacattgac aaacagcata ttgcatgttt ttcttggatc gaatccgcat tgttttatag 1718

ccgttcggaa catacgccga aagggggtcg agctacgccc tacagcgact cgggctacgc 1778

cgtagagcgt accgagctgc gctctacggc tcttcgagct acgctgtaga gcgtacctcg 1838

ccgagctcta cagcgtagct cgaagagccg tacgggatag ctcgtcatcc tctatggtgt 1898

accgccatct tgcc 1912

<210> 40
<211> 431
<212> PRT
<213> Porphyromonas gingivalis

<400> 40
Met Gln Trp Gln Glu Leu Tyr Arg Gln Arg Val Cys Ser Ala Asp Glu
1               5                   10                  15
Ala Val Val Asp Ser Leu Lys Pro Gly Thr Lys Val Val Phe Gly His
            20                  25                  30
Ala Ala Ala Ala Pro Val Arg Phe Ser Gln Ala Met Tyr Arg Gln Arg
        35                  40                  45
Glu Lys Leu Glu Asn Ile Thr Val Phe His Met Leu Tyr Phe Gly Asp
    50                  55                  60
Ala Pro His Leu Ala Pro Glu Met Arg Ser His Val His Pro Thr Leu
65                  70                  75                  80
Asn Phe Leu Glu Gly Asn Ser Arg Pro Ala Ser Arg Asp Arg Arg Val
                85                  90                  95
Asp Phe Ile Pro Cys His Phe His Glu Val Pro Glu Leu Phe Arg Gln

100                 105                 110
Gly Phe Phe Pro Leu Asp Val Ala Val Val Gln Val Ser Thr Pro Asn
        115                 120                 125
Glu Glu Gly Tyr Cys Ser Phe Gly Val Ser Cys Asp Tyr Thr Lys Ala
    130                 135                 140
Ala Ala Glu Cys Ala Pro Val Val Val Ala Glu Val Asn Lys Gln Met
145                 150                 155                 160
Pro Phe Ile Gly Gly Glu Asn Leu Ile His Ile Ser Lys Leu Thr His
                165                 170                 175
Ile Ile Glu Val Asp Glu Pro Ile Ala Glu Val Leu Pro Pro Ala Ile
            180                 185                 190
Ser Asp Leu Glu Leu Arg Ile Gly Gln Asn Cys Ala Ser Leu Ile Lys
        195                 200                 205
Asp Gly Asp Thr Leu Gln Leu Gly Ile Gly Gly Ile Pro Asp Ala Val
    210                 215                 220
Leu Arg Ala Leu Glu Gly His Lys Asp Leu Gly Ile His Thr Glu Met
225                 230                 235                 240
Phe Thr Asp Gly Val Met Arg Met Ile Arg Lys Gly Ile Ile Asn Gly
                245                 250                 255
Lys Lys Lys Thr Leu His Pro Glu Lys Val Val Thr Ser Leu Ile Phe
            260                 265                 270
Gly Ser Lys Glu Leu Tyr Asp Phe Val Asn Asn Asn Pro Val Ile Glu
        275                 280                 285
Cys Tyr Pro Val Asp Tyr Ile Asn Asn Pro Asp Val Ile Gly Lys Asn
    290                 295                 300
Asp Arg Met Val Ser Ile Asn Ser Cys Leu Glu Met Asp Leu Met Gly
305                 310                 315                 320
Gln Ala Ala Ser Glu Ser Ile Gly Tyr Glu Gln Phe Ser Gly Ser Gly
                325                 330                 335
Gly Gln Val Asp Phe Leu Arg Gly Ala Lys Arg Ser Lys Gly Gly Ile
            340                 345                 350
Ser Ile Met Ala Phe Pro Ser Thr Ala Lys Lys Gly Thr Glu Ser Arg
        355                 360                 365
Ile Val Pro Ile Leu Lys Glu Gly Ala Cys Val Thr Thr Gly Arg Asn
    370                 375                 380
Glu Val Asp Tyr Val Val Thr Glu Tyr Gly Val Ala Arg Leu Arg Gly
385                 390                 395                 400
Ala Thr Leu Arg Gln Arg Ala Glu Ala Leu Thr Ala Ile Ala His Pro
                405                 410                 415
Asp Phe Arg Pro Ala Leu Glu Glu Glu Ile Arg Arg Arg Phe Glu
            420                 425                 430

<210> 41
<211> 1497
<212> DNA
<213> Porphyromonas gingivalis

<220>
<221> CDS
<222> (1)..(1497)

<400> 41
atg gct cta aga ttt atc act gca gaa gaa gca gcg gag ttt gtg cat       48
Met Ala Leu Arg Phe Ile Thr Ala Glu Glu Ala Ala Glu Phe Val His
1               5                   10                  15
cac aac gac aat gtc ggc ttt agc ggt ttt acc cct gca ggc aac cct       96
His Asn Asp Asn Val Gly Phe Ser Gly Phe Thr Pro Ala Gly Asn Pro
            20                  25                  30
aag gta gta cct gcg gca ata gcc aag cga gct att gca gcg cat gaa      144
Lys Val Val Pro Ala Ala Ile Ala Lys Arg Ala Ile Ala Ala His Glu
        35                  40                  45
aaa ggc aat ccc ttc aag atc ggt atg ttc acc ggt gcc agt acc ggt      192
Lys Gly Asn Pro Phe Lys Ile Gly Met Phe Thr Gly Ala Ser Thr Gly
    50                  55                  60
gct cgg ttg gac ggt gtc ttg gca cag gcc gat gct gtg aag ttt cgt      240
Ala Arg Leu Asp Gly Val Leu Ala Gln Ala Asp Ala Val Lys Phe Arg
65                  70                  75                  80
act cca tat cag tcc aat aag gat ctg cgc aat ctt atc aac aat ggc      288
Thr Pro Tyr Gln Ser Asn Lys Asp Leu Arg Asn Leu Ile Asn Asn Gly
                85                  90                  95
agc act tcc tat ttc gat ctt cat ctc tct act ttg gct cag gat ctg      336
Ser Thr Ser Tyr Phe Asp Leu His Leu Ser Thr Leu Ala Gln Asp Leu

            100                 105                 110
cgc tat ggc ttt tac ggc aag gtg gat gtg gcc atc ata gag gtg gca 384
Arg Tyr Gly Phe Tyr Gly Lys Val Asp Val Ala Ile Ile Glu Val Ala
        115                 120                 125
gac gtt acc gaa gac ggt aag atc ctg cct act aca ggt gta ggt atc 432
Asp Val Thr Glu Asp Gly Lys Ile Leu Pro Thr Thr Gly Val Gly Ile
    130                 135                 140
ctg cct act att tgc cgt ctg gca gac cga atc atc gtg gag ctt aac 480
Leu Pro Thr Ile Cys Arg Leu Ala Asp Arg Ile Ile Val Glu Leu Asn
145                 150                 155                 160
gac aag cac ccc aaa gaa atc atg ggt atg cac gac ttg tgc gaa cct 528
Asp Lys His Pro Lys Glu Ile Met Gly Met His Asp Leu Cys Glu Pro
                165                 170                 175
ctc gat ccg cct gcc cgt cgc gag ctg cct gtc tat act cct tcg gat 576
Leu Asp Pro Pro Ala Arg Arg Glu Leu Pro Val Tyr Thr Pro Ser Asp
            180                 185                 190
cgt atc ggc aag cct tat gta cag gtg gat ccg gcc aag ata gtc ggt 624
Arg Ile Gly Lys Pro Tyr Val Gln Val Asp Pro Ala Lys Ile Val Gly
        195                 200                 205
gta gtg cgt acg agc gaa cct aat gac gag agc gat ttt gcg ccc ctt 672
Val Val Arg Thr Ser Glu Pro Asn Asp Glu Ser Asp Phe Ala Pro Leu
    210                 215                 220
gac cct gta acg cag gct att ggg gac aat gtg gct gct ttc ctc gtg 720
Asp Pro Val Thr Gln Ala Ile Gly Asp Asn Val Ala Ala Phe Leu Val
225                 230                 235                 240
agc gaa atg aaa gcc gga cga ata ccc aaa gat ttt ctt ccc ctg caa 768
Ser Glu Met Lys Ala Gly Arg Ile Pro Lys Asp Phe Leu Pro Leu Gln
                245                 250                 255
agc ggc gta ggc aat gtg gct aat gcc gta ctg gga gcg ttg ggt gac 816
Ser Gly Val Gly Asn Val Ala Asn Ala Val Leu Gly Ala Leu Gly Asp
            260                 265                 270
aat ccc gat att ccg gct ttc aac atg tac aca gag gta att cag gat 864
Asn Pro Asp Ile Pro Ala Phe Asn Met Tyr Thr Glu Val Ile Gln Asp
        275                 280                 285
gcc gtt atc gct ctg atg aag aaa gga cgc atc aag ttc gcc agc gga 912
Ala Val Ile Ala Leu Met Lys Lys Gly Arg Ile Lys Phe Ala Ser Gly
    290                 295                 300
tgt tcg ctc tct gtg agt cgc tcc gtg ata cag gac atc tac gcc aat 960
Cys Ser Leu Ser Val Ser Arg Ser Val Ile Gln Asp Ile Tyr Ala Asn
305                 310                 315                 320
ctg gat ttc ttc aag gac aag atc ctc ctt cgt ccg cag gaa tac tcc 1008
Leu Asp Phe Phe Lys Asp Lys Ile Leu Leu Arg Pro Gln Glu Tyr Ser
                325                 330                 335
aac aat ccc gag atc gtc cgc cgg tta ggt gtt atc acc atc aat acg 1056
Asn Asn Pro Glu Ile Val Arg Arg Leu Gly Val Ile Thr Ile Asn Thr
            340                 345                 350
gct ctc gaa gcg gat att ttc ggc aat atc aac tct acg cac gtt agc 1104
Ala Leu Glu Ala Asp Ile Phe Gly Asn Ile Asn Ser Thr His Val Ser
        355                 360                 365
ggt acg cgt atg atg aac ggt atc gga ggt tcg ggc gac ttc act cgc 1152
Gly Thr Arg Met Met Asn Gly Ile Gly Gly Ser Gly Asp Phe Thr Arg
    370                 375                 380
aac agc tac gtc tcc atc ttc act aca ccg tcc gtt atg aag gac ggc 1200
Asn Ser Tyr Val Ser Ile Phe Thr Thr Pro Ser Val Met Lys Asp Gly
385                 390                 395                 400
aag atc agt tct ttc gtc ccg atg gtg gcg cac cac gat cat agc gag 1248
Lys Ile Ser Ser Phe Val Pro Met Val Ala His His Asp His Ser Glu
                405                 410                 415
cac tcc gtg aaa gtc atc att tcc gaa tgg ggc gtt gca gac ttg cga 1296
His Ser Val Lys Val Ile Ile Ser Glu Trp Gly Val Ala Asp Leu Arg
            420                 425                 430
ggc aag aac cca cgt gag cgt gct cat gag atc atc gac aaa tgt gtg 1344
Gly Lys Asn Pro Arg Glu Arg Ala His Glu Ile Ile Asp Lys Cys Val
        435                 440                 445
cat ccc gac tat cgt ccc ctc ctt cgt cag tat ctg gag ctt gga gtc 1392
His Pro Asp Tyr Arg Pro Leu Leu Arg Gln Tyr Leu Glu Leu Gly Val
    450                 455                 460
aaa gga caa aca ccc cag aat ctg gac tgc tgc ttt gcc ttc cat cag 1440
Lys Gly Gln Thr Pro Gln Asn Leu Asp Cys Cys Phe Ala Phe His Gln
465                 470                 475                 480
gaa ttg gcc aag agc ggc gac atg cgc aat gta cgc tgg gaa gac tac 1488
Glu Leu Ala Lys Ser Gly Asp Met Arg Asn Val Arg Trp Glu Asp Tyr

485                 490                 495
atg aag taa                                                       1497
Met Lys

<210> 42
<211> 498
<212> PRT
<213> Porphyromonas gingivalis

<400> 42
Met Ala Leu Arg Phe Ile Thr Ala Glu Glu Ala Ala Glu Phe Val His
1               5                   10                  15
His Asn Asp Asn Val Gly Phe Ser Gly Phe Thr Pro Ala Gly Asn Pro
            20                  25                  30
Lys Val Val Pro Ala Ala Ile Ala Lys Arg Ala Ile Ala Ala His Glu
        35                  40                  45
Lys Gly Asn Pro Phe Lys Ile Gly Met Phe Thr Gly Ala Ser Thr Gly
    50                  55                  60
Ala Arg Leu Asp Gly Val Leu Ala Gln Ala Asp Ala Val Lys Phe Arg
65                  70                  75                  80
Thr Pro Tyr Gln Ser Asn Lys Asp Leu Arg Asn Leu Ile Asn Asn Gly
                85                  90                  95
Ser Thr Ser Tyr Phe Asp Leu His Leu Ser Thr Leu Ala Gln Asp Leu
            100                 105                 110
Arg Tyr Gly Phe Tyr Gly Lys Val Asp Val Ala Ile Ile Glu Val Ala
        115                 120                 125
Asp Val Thr Glu Asp Gly Lys Ile Leu Pro Thr Thr Gly Val Gly Ile
    130                 135                 140
Leu Pro Thr Ile Cys Arg Leu Ala Asp Arg Ile Ile Val Glu Leu Asn
145                 150                 155                 160
Asp Lys His Pro Lys Glu Ile Met Gly Met His Asp Leu Cys Glu Pro
                165                 170                 175
Leu Asp Pro Pro Ala Arg Arg Glu Leu Pro Val Tyr Thr Pro Ser Asp
            180                 185                 190
Arg Ile Gly Lys Pro Tyr Val Gln Val Asp Pro Ala Lys Ile Val Gly
        195                 200                 205
Val Val Arg Thr Ser Glu Pro Asn Asp Glu Ser Asp Phe Ala Pro Leu
    210                 215                 220
Asp Pro Val Thr Gln Ala Ile Gly Asp Asn Val Ala Ala Phe Leu Val
225                 230                 235                 240
Ser Glu Met Lys Ala Gly Arg Ile Pro Lys Asp Phe Leu Pro Leu Gln
                245                 250                 255
Ser Gly Val Gly Asn Val Ala Asn Ala Val Leu Gly Ala Leu Gly Asp
            260                 265                 270
Asn Pro Asp Ile Pro Ala Phe Asn Met Tyr Thr Glu Val Ile Gln Asp
        275                 280                 285
Ala Val Ile Ala Leu Met Lys Lys Gly Arg Ile Lys Phe Ala Ser Gly
    290                 295                 300
Cys Ser Leu Ser Val Ser Arg Ser Val Ile Gln Asp Ile Tyr Ala Asn
305                 310                 315                 320
Leu Asp Phe Phe Lys Asp Lys Ile Leu Leu Arg Pro Gln Glu Tyr Ser
                325                 330                 335
Asn Asn Pro Glu Ile Val Arg Arg Leu Gly Val Ile Thr Ile Asn Thr
            340                 345                 350
Ala Leu Glu Ala Asp Ile Phe Gly Asn Ile Asn Ser Thr His Val Ser
        355                 360                 365
Gly Thr Arg Met Met Asn Gly Ile Gly Gly Ser Gly Asp Phe Thr Arg
    370                 375                 380
Asn Ser Tyr Val Ser Ile Phe Thr Thr Pro Ser Val Met Lys Asp Gly
385                 390                 395                 400
Lys Ile Ser Ser Phe Val Pro Met Val Ala His His Asp His Ser Glu
                405                 410                 415
His Ser Val Lys Val Ile Ile Ser Glu Trp Gly Val Ala Asp Leu Arg
            420                 425                 430
Gly Lys Asn Pro Arg Glu Arg Ala His Glu Ile Ile Asp Lys Cys Val
        435                 440                 445
His Pro Asp Tyr Arg Pro Leu Leu Arg Gln Tyr Leu Glu Leu Gly Val
    450                 455                 460
Lys Gly Gln Thr Pro Gln Asn Leu Asp Cys Cys Phe Ala Phe His Gln
465                 470                 475                 480

Glu Leu Ala Lys Ser Gly Asp Met Arg Asn Val Arg Trp Glu Asp Tyr
                485                 490                 495
Met Lys

<210> 43
<211> 1296
<212> DNA
<213> Porphyromonas gingivalis

<220>
<221> CDS
<222> (1)..(1296)

<400> 43
atg aaa gac gta tta gcg gaa tat gcc tcc cga att gtt tcg gcc gaa      48
Met Lys Asp Val Leu Ala Glu Tyr Ala Ser Arg Ile Val Ser Ala Glu
1               5                   10                  15
gaa gcc gta aaa cat atc aaa aat gga gaa cgg gta gct ttg tca cat      96
Glu Ala Val Lys His Ile Lys Asn Gly Glu Arg Val Ala Leu Ser His
            20                  25                  30
gct gcc gga gtt cct cag agt tgt gtt gat gca ctg gta caa cag gcc      144
Ala Ala Gly Val Pro Gln Ser Cys Val Asp Ala Leu Val Gln Gln Ala
        35                  40                  45
gac ctt ttc cag aat gtc gaa att tat cac atg ctt tgt ctc ggc gaa      192
Asp Leu Phe Gln Asn Val Glu Ile Tyr His Met Leu Cys Leu Gly Glu
    50                  55                  60
gga aaa tat atg gca cct gaa atg gcc cct cac ttc cga cac ata acc      240
Gly Lys Tyr Met Ala Pro Glu Met Ala Pro His Phe Arg His Ile Thr
65                  70                  75                  80
aat ttt gta ggt ggt aat tct cgt aaa gca gtt gag gaa aat aga gcc      288
Asn Phe Val Gly Gly Asn Ser Arg Lys Ala Val Glu Glu Asn Arg Ala
                85                  90                  95
gac ttc att ccg gta ttc ttt tat gaa gtg cca tca atg att cgc aaa      336
Asp Phe Ile Pro Val Phe Phe Tyr Glu Val Pro Ser Met Ile Arg Lys
            100                 105                 110
gac atc ctt cac ata gat gtc gcc atc gtt cag ctt tca atg cct gat      384
Asp Ile Leu His Ile Asp Val Ala Ile Val Gln Leu Ser Met Pro Asp
        115                 120                 125
gag aat ggt tac tgt agt ttt gga gta tct tgc gat tat agc aaa ccg      432
Glu Asn Gly Tyr Cys Ser Phe Gly Val Ser Cys Asp Tyr Ser Lys Pro
    130                 135                 140
gca gca gaa agc gct cat tta gtt ata ggg gaa atc aac cgt caa atg      480
Ala Ala Glu Ser Ala His Leu Val Ile Gly Glu Ile Asn Arg Gln Met
145                 150                 155                 160
cca tat gta cat ggc gac aac ttg att cac ata tcg aag ttg gat tac      528
Pro Tyr Val His Gly Asp Asn Leu Ile His Ile Ser Lys Leu Asp Tyr
                165                 170                 175
atc gtg atg gca gac tac cct atc tat tct ctt gca aag ccc aaa atc      576
Ile Val Met Ala Asp Tyr Pro Ile Tyr Ser Leu Ala Lys Pro Lys Ile
            180                 185                 190
gga gaa gta gaa gaa gct atc ggg cgt aat tgt gcc gag ctt att gaa      624
Gly Glu Val Glu Glu Ala Ile Gly Arg Asn Cys Ala Glu Leu Ile Glu
        195                 200                 205
gat ggt gcc aca ctc caa ctc ggt atc ggc gcg att cct gat gca gcc      672
Asp Gly Ala Thr Leu Gln Leu Gly Ile Gly Ala Ile Pro Asp Ala Ala
    210                 215                 220
ctg tta ttc ctc aag gac aaa aaa gat ctg ggg atc cat acc gag atg      720
Leu Leu Phe Leu Lys Asp Lys Lys Asp Leu Gly Ile His Thr Glu Met
225                 230                 235                 240
ttc tcc gat ggt gtt gtc gaa tta gtt cgc agt gga gta att aca gga      768
Phe Ser Asp Gly Val Val Glu Leu Val Arg Ser Gly Val Ile Thr Gly
                245                 250                 255
aag aaa aag aca ctt cac ccc gga aag atg gtc gca acc ttc tta atg      816
Lys Lys Lys Thr Leu His Pro Gly Lys Met Val Ala Thr Phe Leu Met
            260                 265                 270
gga agc gaa gac gta tat cat ttc atc gac aaa aat ccc gat gta gaa      864
Gly Ser Glu Asp Val Tyr His Phe Ile Asp Lys Asn Pro Asp Val Glu
        275                 280                 285
ctt tat ccg gta gat tac gtc aat gat ccg cga gta atc gct caa aat      912
Leu Tyr Pro Val Asp Tyr Val Asn Asp Pro Arg Val Ile Ala Gln Asn
    290                 295                 300

gat aat atg gtc agc atc aat agc tgt atc gaa atc gat ctt atg gga 960
Asp Asn Met Val Ser Ile Asn Ser Cys Ile Glu Ile Asp Leu Met Gly
305                 310                 315                 320
caa gtc gtg tcc gaa tgt ata gga agc aag caa ttc agc gga acc ggc 1008
Gln Val Val Ser Glu Cys Ile Gly Ser Lys Gln Phe Ser Gly Thr Gly
                325                 330                 335
ggt caa gta gat tat gtt cgt gga gca gca tgg tct aaa aac ggc aaa 1056
Gly Gln Val Asp Tyr Val Arg Gly Ala Ala Trp Ser Lys Asn Gly Lys
            340                 345                 350
agc atc atg gca att ccc tca aca gcc aaa aac ggt act gca tct cga 1104
Ser Ile Met Ala Ile Pro Ser Thr Ala Lys Asn Gly Thr Ala Ser Arg
        355                 360                 365
att gta cct ata att gca gag gga gct gct gta aca acc ctc cgc aac 1152
Ile Val Pro Ile Ile Ala Glu Gly Ala Ala Val Thr Thr Leu Arg Asn
    370                 375                 380
gaa gtc gat tac gtt gta acc gaa tac ggt ata gca caa ctc aaa gga 1200
Glu Val Asp Tyr Val Val Thr Glu Tyr Gly Ile Ala Gln Leu Lys Gly
385                 390                 395                 400
aag agt ttg cgc cag cga gca gaa gct ctt att gcc ata gcc cac ccg 1248
Lys Ser Leu Arg Gln Arg Ala Glu Ala Leu Ile Ala Ile Ala His Pro
                405                 410                 415
gat ttc aga gag gaa cta acg aaa cat ctc cgc aaa cgt ttc gga 1293
Asp Phe Arg Glu Glu Leu Thr Lys His Leu Arg Lys Arg Phe Gly
            420                 425                 430
taa 1296

<210> 44
<211> 431
<212> PRT
<213> Porphyromonas gingivalis

<400> 44
Met Lys Asp Val Leu Ala Glu Tyr Ala Ser Arg Ile Val Ser Ala Glu
1               5                   10                  15
Glu Ala Val Lys His Ile Lys Asn Gly Glu Arg Val Ala Leu Ser His
            20                  25                  30
Ala Ala Gly Val Pro Gln Ser Cys Val Asp Ala Leu Val Gln Gln Ala
        35                  40                  45
Asp Leu Phe Gln Asn Val Glu Ile Tyr His Met Leu Cys Leu Gly Glu
    50                  55                  60
Gly Lys Tyr Met Ala Pro Glu Met Ala Pro His Phe Arg His Ile Thr
65                  70                  75                  80
Asn Phe Val Gly Gly Asn Ser Arg Lys Ala Val Glu Glu Asn Arg Ala
                85                  90                  95
Asp Phe Ile Pro Val Phe Phe Tyr Glu Val Pro Ser Met Ile Arg Lys
            100                 105                 110
Asp Ile Leu His Ile Asp Val Ala Ile Val Gln Leu Ser Met Pro Asp
        115                 120                 125
Glu Asn Gly Tyr Cys Ser Phe Gly Val Ser Cys Asp Tyr Ser Lys Pro
    130                 135                 140
Ala Ala Glu Ser Ala His Leu Val Ile Gly Glu Ile Asn Arg Gln Met
145                 150                 155                 160
Pro Tyr Val His Gly Asp Asn Leu Ile His Ile Ser Lys Leu Asp Tyr
                165                 170                 175
Ile Val Met Ala Asp Tyr Pro Ile Tyr Ser Leu Ala Lys Pro Lys Ile
            180                 185                 190
Gly Glu Val Glu Glu Ala Ile Gly Arg Asn Cys Ala Glu Leu Ile Glu
        195                 200                 205
Asp Gly Ala Thr Leu Gln Leu Gly Ile Gly Ala Ile Pro Asp Ala Ala
    210                 215                 220
Leu Leu Phe Leu Lys Asp Lys Lys Asp Leu Gly Ile His Thr Glu Met
225                 230                 235                 240
Phe Ser Asp Gly Val Val Glu Leu Val Arg Ser Gly Val Ile Thr Gly
                245                 250                 255
Lys Lys Lys Thr Leu His Pro Gly Lys Met Val Ala Thr Phe Leu Met
            260                 265                 270
Gly Ser Glu Asp Val Tyr His Phe Ile Asp Lys Asn Pro Asp Val Glu
        275                 280                 285
Leu Tyr Pro Val Asp Tyr Val Asn Asp Pro Arg Val Ile Ala Gln Asn

290                 295                 300
Asp Asn Met Val Ser Ile Asn Ser Cys Ile Glu Ile Asp Leu Met Gly
305                 310                 315                 320
Gln Val Val Ser Glu Cys Ile Gly Ser Lys Gln Phe Ser Gly Thr Gly
                325                 330                 335
Gly Gln Val Asp Tyr Val Arg Gly Ala Ala Trp Ser Lys Asn Gly Lys
            340                 345                 350
Ser Ile Met Ala Ile Pro Ser Thr Ala Lys Asn Gly Thr Ala Ser Arg
        355                 360                 365
Ile Val Pro Ile Ile Ala Glu Gly Ala Ala Val Thr Thr Leu Arg Asn
    370                 375                 380
Glu Val Asp Tyr Val Val Thr Glu Tyr Gly Ile Ala Gln Leu Lys Gly
385                 390                 395                 400
Lys Ser Leu Arg Gln Arg Ala Glu Ala Leu Ile Ala Ile Ala His Pro
                405                 410                 415
Asp Phe Arg Glu Glu Leu Thr Lys His Leu Arg Lys Arg Phe Gly
            420                 425                 430

<210> 45
<211> 1317
<212> DNA
<213> Mycoplasma gallisepticum

<220>
<221> CDS
<222> (1)..(1317)

<400> 45
atg aat tat ttt aaa gca tat aaa aat aaa tta gtt tct gtc aat cag      48
Met Asn Tyr Phe Lys Ala Tyr Lys Asn Lys Leu Val Ser Val Asn Gln
1               5                   10                  15
gcc att tca cta ata aaa aat aac tcg aat att tgg ttt gat tat ctg      96
Ala Ile Ser Leu Ile Lys Asn Asn Ser Asn Ile Trp Phe Asp Tyr Leu
            20                  25                  30
cag cca aaa cta ctt tta cag ggg cta aaa gat aca tta gaa act aat      144
Gln Pro Lys Leu Leu Leu Gln Gly Leu Lys Asp Thr Leu Glu Thr Asn
        35                  40                  45
agc tac tct aat tta aat ttt tat tat tat gct tct agc tct gat tgt      192
Ser Tyr Ser Asn Leu Asn Phe Tyr Tyr Tyr Ala Ser Ser Ser Asp Cys
    50                  55                  60
gaa gaa aca atc ttt tta gac aaa ttt aat caa ata gtt aaa aga cat      240
Glu Glu Thr Ile Phe Leu Asp Lys Phe Asn Gln Ile Val Lys Arg His
65                  70                  75                  80
tgt ttt ttc tac aga gct aat gaa aga aat gct tta aag aaa tat caa      288
Cys Phe Phe Tyr Arg Ala Asn Glu Arg Asn Ala Leu Lys Lys Tyr Gln
                85                  90                  95
aac gga ata ctt caa aat aac gtt gaa tac tat cca ttt cat ttc tct      336
Asn Gly Ile Leu Gln Asn Asn Val Glu Tyr Tyr Pro Phe His Phe Ser
            100                 105                 110
gaa tct act aag att ctt aat aat ttt gct ata gat acg gtt att tta      384
Glu Ser Thr Lys Ile Leu Asn Asn Phe Ala Ile Asp Thr Val Ile Leu
        115                 120                 125
ata act act gat att gat gaa aac ggt tat ttt aat tta ggc tta tcc      432
Ile Thr Thr Asp Ile Asp Glu Asn Gly Tyr Phe Asn Leu Gly Leu Ser
    130                 135                 140
act tgt tat gat tta gat tta att aaa aaa gca aaa caa gta att att      480
Thr Cys Tyr Asp Leu Asp Leu Ile Lys Lys Ala Lys Gln Val Ile Ile
145                 150                 155                 160
gaa ctc aat aaa aat atg cct cat att atg ggc gat aag cat caa att      528
Glu Leu Asn Lys Asn Met Pro His Ile Met Gly Asp Lys His Gln Ile
                165                 170                 175
cac att aat gag gta gcc gca ata gtc aaa tct gac tat caa cta act      576
His Ile Asn Glu Val Ala Ala Ile Val Lys Ser Asp Tyr Gln Leu Thr
            180                 185                 190
gaa gca tta agt gtt gat cca agt cca gaa cac ttg aaa ata gca gat      624
Glu Ala Leu Ser Val Asp Pro Ser Pro Glu His Leu Lys Ile Ala Asp
        195                 200                 205
tat gta gct tca tta att caa gat cgt tct acg att caa ttg ggt ata      672
Tyr Val Ala Ser Leu Ile Gln Asp Arg Ser Thr Ile Gln Leu Gly Ile
    210                 215                 220
ggt gca att ccc aac atg gtg tgc aaa aaa ctt gta aat aaa aaa gac      720
Gly Ala Ile Pro Asn Met Val Cys Lys Lys Leu Val Asn Lys Lys Asp

225                 230                 235                 240
cta gga att cat aca gaa atc cta ggt act ggg atg gtc gaa ctt atc         768
Leu Gly Ile His Thr Glu Ile Leu Gly Thr Gly Met Val Glu Leu Ile
                245                 250                 255
aaa ctt ggg gtg gtt aac aac aaa tat aaa gaa att gac caa ggg gta         816
Lys Leu Gly Val Val Asn Asn Lys Tyr Lys Glu Ile Asp Gln Gly Val
            260                 265                 270
tca acg ttt gta ttt gct gtt gga gac caa gat atg tac aaa tat ctt         864
Ser Thr Phe Val Phe Ala Val Gly Asp Gln Asp Met Tyr Lys Tyr Leu
        275                 280                 285
gat cat aac gat aaa ttt aaa att gca cga ttt gat tat gta aat aac         912
Asp His Asn Asp Lys Phe Lys Ile Ala Arg Phe Asp Tyr Val Asn Asn
    290                 295                 300
cca tta aat att gcc aaa ttc gaa aac ttt att tcg gtt aat tcc gct         960
Pro Leu Asn Ile Ala Lys Phe Glu Asn Phe Ile Ser Val Asn Ser Ala
305                 310                 315                 320
atc gaa ata gat tta tta ggt aat gtt aac gct gaa caa att cag cat         1008
Ile Glu Ile Asp Leu Leu Gly Asn Val Asn Ala Glu Gln Ile Gln His
                325                 330                 335
aac caa tat tcg ggt tct ggt gga caa gca gat ttt att aga ggt gct         1056
Asn Gln Tyr Ser Gly Ser Gly Gly Gln Ala Asp Phe Ile Arg Gly Ala
            340                 345                 350
agc tac tct aaa aat ggt aag agt atc att gca atg aaa tca aca act         1104
Ser Tyr Ser Lys Asn Gly Lys Ser Ile Ile Ala Met Lys Ser Thr Thr
        355                 360                 365
agt gat tgc aaa att tct aag att gta acc aaa ctt aaa ggg gtt aca         1152
Ser Asp Cys Lys Ile Ser Lys Ile Val Thr Lys Leu Lys Gly Val Thr
    370                 375                 380
acg aca tta aga aat gat tta gat tat gta atc acc gaa tat gga att         1200
Thr Thr Leu Arg Asn Asp Leu Asp Tyr Val Ile Thr Glu Tyr Gly Ile
385                 390                 395                 400
att agc tta aaa gct aaa tca gta aaa caa cgc gca aaa ttg tta att         1248
Ile Ser Leu Lys Ala Lys Ser Val Lys Gln Arg Ala Lys Leu Leu Ile
                405                 410                 415
agt att gct cat cct aag ttt aga tct tat tta act aga gtt gct aga         1296
Ser Ile Ala His Pro Lys Phe Arg Ser Tyr Leu Thr Arg Val Ala Arg
            420                 425                 430
tct aaa cta ctt att gat taa                                             1317
Ser Lys Leu Leu Ile Asp
        435


<210> 46
<211> 438
<212> PRT
<213> Mycoplasma gallisepticum

<400> 46
Met Asn Tyr Phe Lys Ala Tyr Lys Asn Lys Leu Val Ser Val Asn Gln
1               5                   10                  15
Ala Ile Ser Leu Ile Lys Asn Asn Ser Asn Ile Trp Phe Asp Tyr Leu
            20                  25                  30
Gln Pro Lys Leu Leu Leu Gln Gly Leu Lys Asp Thr Leu Glu Thr Asn
        35                  40                  45
Ser Tyr Ser Asn Leu Asn Phe Tyr Tyr Tyr Ala Ser Ser Ser Asp Cys
    50                  55                  60
Glu Glu Thr Ile Phe Leu Asp Lys Phe Asn Gln Ile Val Lys Arg His
65                  70                  75                  80
Cys Phe Phe Tyr Arg Ala Asn Glu Arg Asn Ala Leu Lys Lys Tyr Gln
                85                  90                  95
Asn Gly Ile Leu Gln Asn Asn Val Glu Tyr Tyr Pro Phe His Phe Ser
            100                 105                 110
Glu Ser Thr Lys Ile Leu Asn Asn Phe Ala Ile Asp Thr Val Ile Leu
        115                 120                 125
Ile Thr Thr Asp Ile Asp Glu Asn Gly Tyr Phe Asn Leu Gly Leu Ser
    130                 135                 140
Thr Cys Tyr Asp Leu Asp Leu Ile Lys Lys Ala Lys Gln Val Ile Ile
145                 150                 155                 160
Glu Leu Asn Lys Asn Met Pro His Ile Met Gly Asp Lys His Gln Ile
                165                 170                 175
His Ile Asn Glu Val Ala Ala Ile Val Lys Ser Asp Tyr Gln Leu Thr
            180                 185                 190

Glu Ala Leu Ser Val Asp Pro Ser Pro Glu His Leu Lys Ile Ala Asp
        195                 200                 205
Tyr Val Ala Ser Leu Ile Gln Asp Arg Ser Thr Ile Gln Leu Gly Ile
    210                 215                 220
Gly Ala Ile Pro Asn Met Val Cys Lys Lys Leu Val Asn Lys Lys Asp
225                 230                 235                 240
Leu Gly Ile His Thr Glu Ile Leu Gly Thr Gly Met Val Glu Leu Ile
                245                 250                 255
Lys Leu Gly Val Val Asn Asn Lys Tyr Lys Glu Ile Asp Gln Gly Val
            260                 265                 270
Ser Thr Phe Val Phe Ala Val Gly Asp Gln Asp Met Tyr Lys Tyr Leu
        275                 280                 285
Asp His Asn Asp Lys Phe Lys Ile Ala Arg Phe Asp Tyr Val Asn Asn
    290                 295                 300
Pro Leu Asn Ile Ala Lys Phe Glu Asn Phe Ile Ser Val Asn Ser Ala
305                 310                 315                 320
Ile Glu Ile Asp Leu Leu Gly Asn Val Asn Ala Glu Gln Ile Gln His
                325                 330                 335
Asn Gln Tyr Ser Gly Ser Gly Gly Gln Ala Asp Phe Ile Arg Gly Ala
            340                 345                 350
Ser Tyr Ser Lys Asn Gly Lys Ser Ile Ile Ala Met Lys Ser Thr Thr
        355                 360                 365
Ser Asp Cys Lys Ile Ser Lys Ile Val Thr Lys Leu Lys Gly Val Thr
    370                 375                 380
Thr Thr Leu Arg Asn Asp Leu Asp Tyr Val Ile Thr Glu Tyr Gly Ile
385                 390                 395                 400
Ile Ser Leu Lys Ala Lys Ser Val Lys Gln Arg Ala Lys Leu Leu Ile
                405                 410                 415
Ser Ile Ala His Pro Lys Phe Arg Ser Tyr Leu Thr Arg Val Ala Arg
            420                 425                 430
Ser Lys Leu Leu Ile Asp
        435

<210> 47
<211> 1578
<212> DNA
<213> Neurospora crassa

<220>
<221> CDS
<222> (1)..(1578)

<400> 47
atg gcg tcg ccc att gcc tcg gcg gcc ctc agg gcc cgc gtc aag cgc       48
Met Ala Ser Pro Ile Ala Ser Ala Ala Leu Arg Ala Arg Val Lys Arg
1               5                   10                  15
cct tcc atg ctc aag aag ctc tgc aac ccc gag gac atg ctc cag cac       96
Pro Ser Met Leu Lys Lys Leu Cys Asn Pro Glu Asp Met Leu Gln His
            20                  25                  30
ttc ccc aat ggc gca tac atc ggc tgg tcc ggt ttc acc ggc gtc ggc      144
Phe Pro Asn Gly Ala Tyr Ile Gly Trp Ser Gly Phe Thr Gly Val Gly
        35                  40                  45
tac ccc aag aag gtc cct act atg ctc gct gac cac gtc gag aag aac      192
Tyr Pro Lys Lys Val Pro Thr Met Leu Ala Asp His Val Glu Lys Asn
    50                  55                  60
ggc ctc cag ggc cag ctc aag tac agc ctc ttc gtc ggt gcc tcc gcc      240
Gly Leu Gln Gly Gln Leu Lys Tyr Ser Leu Phe Val Gly Ala Ser Ala
65                  70                  75                  80
ggc gcc gag acc gag aac cgc tgg gct gct ctc gac atg atc gcc cgt      288
Gly Ala Glu Thr Glu Asn Arg Trp Ala Ala Leu Asp Met Ile Ala Arg
                85                  90                  95
cgc gct cct cac cag gtc ggc aag aac att gcc aag ggc atc aac gag      336
Arg Ala Pro His Gln Val Gly Lys Asn Ile Ala Lys Gly Ile Asn Glu
            100                 105                 110
ggc cgc atc aac ttc ttc gac aag cac ttg tcc atg ttc ccc gtt gac      384
Gly Arg Ile Asn Phe Phe Asp Lys His Leu Ser Met Phe Pro Val Asp
        115                 120                 125
ctt gtc tac ggc tac tac acc aag gac cgc cag aac aag aac ctc gat      432
Leu Val Tyr Gly Tyr Tyr Thr Lys Asp Arg Gln Asn Lys Asn Leu Asp
    130                 135                 140
gtc gtt tgc gtt gaa gct acc gag atc aag gag gac ggt tcc atc gtc      480
Val Val Cys Val Glu Ala Thr Glu Ile Lys Glu Asp Gly Ser Ile Val

145                 150                 155                 160
ctc ggt gcc tcg gtc ggt gcc acc ccc gag ctc atc cag atg gcc gac       528
Leu Gly Ala Ser Val Gly Ala Thr Pro Glu Leu Ile Gln Met Ala Asp
                165                 170                 175
aag gtc atc att gag gtc aac acg gcc atc ccc agc ttc gac ggt ctc       576
Lys Val Ile Ile Glu Val Asn Thr Ala Ile Pro Ser Phe Asp Gly Leu
            180                 185                 190
cac gac atc acc ttc tcc gac ctc cct ccc aac cgc aag ccc tac ctc       624
His Asp Ile Thr Phe Ser Asp Leu Pro Pro Asn Arg Lys Pro Tyr Leu
        195                 200                 205
atc cag cag tgc agg gac cgc atc ggt act act tcg gtc ccc gtc gac       672
Ile Gln Gln Cys Arg Asp Arg Ile Gly Thr Thr Ser Val Pro Val Asp
    210                 215                 220
ccc gag aag gtt gtc ggt atc atc gag tgc acc acc ccc gat cag acc       720
Pro Glu Lys Val Val Gly Ile Ile Glu Cys Thr Thr Pro Asp Gln Thr
225                 230                 235                 240
ctc ccc aac tcc ccc gcc gac gaa acg gct acg gcc att gcc ggc cac       768
Leu Pro Asn Ser Pro Ala Asp Glu Thr Ala Thr Ala Ile Ala Gly His
                245                 250                 255
ctg att gag ttc ttc gag cac gag gtc gcc cac ggc cgt ctg ccc aag       816
Leu Ile Glu Phe Phe Glu His Glu Val Ala His Gly Arg Leu Pro Lys
            260                 265                 270
aac ctt ctc cct ctg cag tcc ggt atc ggc aac atc gcc aac gcc gtc       864
Asn Leu Leu Pro Leu Gln Ser Gly Ile Gly Asn Ile Ala Asn Ala Val
        275                 280                 285
att ggt ggt ctc gag acc tcc aac ttc aag aac ctc aac gtc tgg act       912
Ile Gly Gly Leu Glu Thr Ser Asn Phe Lys Asn Leu Asn Val Trp Thr
    290                 295                 300
gag gtt atc cag gat acc ttc ctc gac ctc ttc gac tcc ggc aag ctt       960
Glu Val Ile Gln Asp Thr Phe Leu Asp Leu Phe Asp Ser Gly Lys Leu
305                 310                 315                 320
gac ttt gct acc gcc acc tcg atc cgc ttc tcg ccc acc ggc ttc gag       1008
Asp Phe Ala Thr Ala Thr Ser Ile Arg Phe Ser Pro Thr Gly Phe Glu
                325                 330                 335
cgc ttc tac aag aac tgg gac aac tac tat gac aag ctc ctc ctc cgc       1056
Arg Phe Tyr Lys Asn Trp Asp Asn Tyr Tyr Asp Lys Leu Leu Leu Arg
            340                 345                 350
tct cag tcc gtc tcc aac gct ccc gag atc atc cgc cgt ctc ggt gtt       1104
Ser Gln Ser Val Ser Asn Ala Pro Glu Ile Ile Arg Arg Leu Gly Val
        355                 360                 365
atc ggc atg aac acc ccc gtc gaa gtc gac atc tac gcc cac gcc aac       1152
Ile Gly Met Asn Thr Pro Val Glu Val Asp Ile Tyr Ala His Ala Asn
    370                 375                 380
tcc acc aac gtc atg ggc tcc cgc atg ctc aac ggt ctc ggt ggt tcc       1200
Ser Thr Asn Val Met Gly Ser Arg Met Leu Asn Gly Leu Gly Gly Ser
385                 390                 395                 400
gcc gat ttc ctc cgc aac tcc aag tac tcc atc atg cac acc ccc tcc       1248
Ala Asp Phe Leu Arg Asn Ser Lys Tyr Ser Ile Met His Thr Pro Ser
                405                 410                 415
acc cgt ccc tcc aag acc gac gcc cac ggt gtt tcg tgc att gtc ccc       1296
Thr Arg Pro Ser Lys Thr Asp Ala His Gly Val Ser Cys Ile Val Pro
            420                 425                 430
atg tgc acc cac gtc gac cag acc gag cac gat ctc gac gtc atc gtt       1344
Met Cys Thr His Val Asp Gln Thr Glu His Asp Leu Asp Val Ile Val
        435                 440                 445
acc gag aac ggt ctc gcc gat gtc cgc ggc ctc agc ccc cgc gag cgt       1392
Thr Glu Asn Gly Leu Ala Asp Val Arg Gly Leu Ser Pro Arg Glu Arg
    450                 455                 460
gcc cgc gtc atc att gac aag tgc gcc cac gac gtc tac aag ccc atc       1440
Ala Arg Val Ile Ile Asp Lys Cys Ala His Asp Val Tyr Lys Pro Ile
465                 470                 475                 480
ctc aag gcc tac ttt gag aag gcc gag ttc gag tgc ctt cgc aag ggt       1488
Leu Lys Ala Tyr Phe Glu Lys Ala Glu Phe Glu Cys Leu Arg Lys Gly
                485                 490                 495
atg ggc cac gag ccc cac ctt ctc ttc aac tct ttc gac atg cac aag       1536
Met Gly His Glu Pro His Leu Leu Phe Asn Ser Phe Asp Met His Lys
            500                 505                 510
gct ctt gtc gag gag ggc tcc atg gcc aag gtc aag ttc taa               1578
Ala Leu Val Glu Glu Gly Ser Met Ala Lys Val Lys Phe
        515                 520                 525

<210> 48
<211> 525
<212> PRT
<213> Neurospora crassa

<400> 48
Met Ala Ser Pro Ile Ala Ser Ala Ala Leu Arg Ala Arg Val Lys Arg
1               5                   10                  15
Pro Ser Met Leu Lys Lys Leu Cys Asn Pro Glu Asp Met Leu Gln His
            20                  25                  30
Phe Pro Asn Gly Ala Tyr Ile Gly Trp Ser Gly Phe Thr Gly Val Gly
        35                  40                  45
Tyr Pro Lys Lys Val Pro Thr Met Leu Ala Asp His Val Glu Lys Asn
    50                  55                  60
Gly Leu Gln Gly Gln Leu Lys Tyr Ser Leu Phe Val Gly Ala Ser Ala
65                  70                  75                  80
Gly Ala Glu Thr Glu Asn Arg Trp Ala Ala Leu Asp Met Ile Ala Arg
                85                  90                  95
Arg Ala Pro His Gln Val Gly Lys Asn Ile Ala Lys Gly Ile Asn Glu
            100                 105                 110
Gly Arg Ile Asn Phe Phe Asp Lys His Leu Ser Met Phe Pro Val Asp
        115                 120                 125
Leu Val Tyr Gly Tyr Tyr Thr Lys Asp Arg Gln Asn Lys Asn Leu Asp
    130                 135                 140
Val Val Cys Val Glu Ala Thr Glu Ile Lys Glu Asp Gly Ser Ile Val
145                 150                 155                 160
Leu Gly Ala Ser Val Gly Ala Thr Pro Glu Leu Ile Gln Met Ala Asp
                165                 170                 175
Lys Val Ile Ile Glu Val Asn Thr Ala Ile Pro Ser Phe Asp Gly Leu
            180                 185                 190
His Asp Ile Thr Phe Ser Asp Leu Pro Pro Asn Arg Lys Pro Tyr Leu
        195                 200                 205
Ile Gln Gln Cys Arg Asp Arg Ile Gly Thr Thr Ser Val Pro Val Asp
    210                 215                 220
Pro Glu Lys Val Val Gly Ile Ile Glu Cys Thr Thr Pro Asp Gln Thr
225                 230                 235                 240
Leu Pro Asn Ser Pro Ala Asp Glu Thr Ala Thr Ala Ile Ala Gly His
                245                 250                 255
Leu Ile Glu Phe Phe Glu His Glu Val Ala His Gly Arg Leu Pro Lys
            260                 265                 270
Asn Leu Leu Pro Leu Gln Ser Gly Ile Gly Asn Ile Ala Asn Ala Val
        275                 280                 285
Ile Gly Gly Leu Glu Thr Ser Asn Phe Lys Asn Leu Asn Val Trp Thr
    290                 295                 300
Glu Val Ile Gln Asp Thr Phe Leu Asp Leu Phe Asp Ser Gly Lys Leu
305                 310                 315                 320
Asp Phe Ala Thr Ala Thr Ser Ile Arg Phe Ser Pro Thr Gly Phe Glu
                325                 330                 335
Arg Phe Tyr Lys Asn Trp Asp Asn Tyr Tyr Asp Lys Leu Leu Leu Arg
            340                 345                 350
Ser Gln Ser Val Ser Asn Ala Pro Glu Ile Ile Arg Arg Leu Gly Val
        355                 360                 365
Ile Gly Met Asn Thr Pro Val Glu Val Asp Ile Tyr Ala His Ala Asn
    370                 375                 380
Ser Thr Asn Val Met Gly Ser Arg Met Leu Asn Gly Leu Gly Gly Ser
385                 390                 395                 400
Ala Asp Phe Leu Arg Asn Ser Lys Tyr Ser Ile Met His Thr Pro Ser
                405                 410                 415
Thr Arg Pro Ser Lys Thr Asp Ala His Gly Val Ser Cys Ile Val Pro
            420                 425                 430
Met Cys Thr His Val Asp Gln Thr Glu His Asp Leu Asp Val Ile Val
        435                 440                 445
Thr Glu Asn Gly Leu Ala Asp Val Arg Gly Leu Ser Pro Arg Glu Arg
    450                 455                 460
Ala Arg Val Ile Ile Asp Lys Cys Ala His Asp Val Tyr Lys Pro Ile
465                 470                 475                 480
Leu Lys Ala Tyr Phe Glu Lys Ala Glu Phe Glu Cys Leu Arg Lys Gly
                485                 490                 495
Met Gly His Glu Pro His Leu Leu Phe Asn Ser Phe Asp Met His Lys
            500                 505                 510
Ala Leu Val Glu Glu Gly Ser Met Ala Lys Val Lys Phe
        515                 520                 525

<210> 49
<211> 1533
<212> DNA
<213> Bordetella pertussis

<220>
<221> CDS
<222> (1)..(1533)

<400> 49
atg tct gga gac agc atg cat ctc gaa cgt att cgc cat ccc ggc ctg 48
Met Ser Gly Asp Ser Met His Leu Glu Arg Ile Arg His Pro Gly Leu
1               5                   10                  15
cgc gcc cgg ttg gct tcc ccc cag gaa gcg gcc cgg ctg gtc cac gac 96
Arg Ala Arg Leu Ala Ser Pro Gln Glu Ala Ala Arg Leu Val His Asp
            20                  25                  30
ggc atg acc gtc ggc atg agt gag ttc acg cgc gcg ggc gac tgc aaa 144
Gly Met Thr Val Gly Met Ser Glu Phe Thr Arg Ala Gly Asp Cys Lys
        35                  40                  45
tcg gtg ccg gcg gcg ctg gcc gaa aga gcc gcc gcc gag ccg ctg cgc 192
Ser Val Pro Ala Ala Leu Ala Glu Arg Ala Ala Ala Glu Pro Leu Arg
    50                  55                  60
atc acg ctg ctg acc ggc gcc tcg ctg ggg cat gac acc gac aag ctg 240
Ile Thr Leu Leu Thr Gly Ala Ser Leu Gly His Asp Thr Asp Lys Leu
65                  70                  75                  80
ctg gcg cag gcc ggc gtt ctg gcg cgc cgc atg ccg ttc cag gtc gac 288
Leu Ala Gln Ala Gly Val Leu Ala Arg Arg Met Pro Phe Gln Val Asp
                85                  90                  95
acc acg ctg cgg cgc aag atc aac cag ggc gag atc gcc ttt gtc gac 336
Thr Thr Leu Arg Arg Lys Ile Asn Gln Gly Glu Ile Ala Phe Val Asp
            100                 105                 110
cag cac ttg tcc gaa acc gtc gag cag ttg cgc gcc ggc cat atc ggc 384
Gln His Leu Ser Glu Thr Val Glu Gln Leu Arg Ala Gly His Ile Gly
        115                 120                 125
ccc atc gac atc gcc atc gtc gag gcg gct gcc atc acc gaa gac ggc 432
Pro Ile Asp Ile Ala Ile Val Glu Ala Ala Ala Ile Thr Glu Asp Gly
    130                 135                 140
ggc atc gtg ccg acc atg tcg gtc ggc aac tcg gcc tcg ttc gtc gag 480
Gly Ile Val Pro Thr Met Ser Val Gly Asn Ser Ala Ser Phe Val Glu
145                 150                 155                 160
cag gcc cgc ctg gtc atc gtc gag ctc aat ctc ggc gtg ccc gcg gcc 528
Gln Ala Arg Leu Val Ile Val Glu Leu Asn Leu Gly Val Pro Ala Ala
                165                 170                 175
atc gaa ggc ctg cac gac atc gtg atc ccg ggc gcg cgg ccc ggc cgc 576
Ile Glu Gly Leu His Asp Ile Val Ile Pro Gly Ala Arg Pro Gly Arg
            180                 185                 190
cag ccc ctg ccg ctg gtg tcg gtc gac cag cgc atc ggc gcg ccc tgc 624
Gln Pro Leu Pro Leu Val Ser Val Asp Gln Arg Ile Gly Ala Pro Cys
        195                 200                 205
atc gcg gtg gac ccc gac aag atc gcc gcc atc gtc gtc acc aac gaa 672
Ile Ala Val Asp Pro Asp Lys Ile Ala Ala Ile Val Val Thr Asn Glu
    210                 215                 220
ccg gac agt ccc tca aac gcc ttg ccg ccc gac gag gag atc ggc gcc 720
Pro Asp Ser Pro Ser Asn Ala Leu Pro Pro Asp Glu Glu Ile Gly Ala
225                 230                 235                 240
atc gcc ggc cac atc gtc ggc ttc ctg cgc cag gaa gtg aac cag ggg 768
Ile Ala Gly His Ile Val Gly Phe Leu Arg Gln Glu Val Asn Gln Gly
                245                 250                 255
cgc ctg agc cgc tcg ctg ctg ccc ctg cag gcc ggc atc ggc acg atc 816
Arg Leu Ser Arg Ser Leu Leu Pro Leu Gln Ala Gly Ile Gly Thr Ile
            260                 265                 270
gcc aat gcc gtg ctg cac ggg ttc ggc gag tcg gaa ttc gag ggg ctc 864
Ala Asn Ala Val Leu His Gly Phe Gly Glu Ser Glu Phe Glu Gly Leu
        275                 280                 285
acc atg tac tcg gaa gtg ctg cag gac agc gcg atc gaa ctc ctg gac 912
Thr Met Tyr Ser Glu Val Leu Gln Asp Ser Ala Ile Glu Leu Leu Asp
    290                 295                 300
cag ggc aag ctg agt ttc gcc tcg gcc tcg tcc atc acg gtc tcg ctg 960
Gln Gly Lys Leu Ser Phe Ala Ser Ala Ser Ser Ile Thr Val Ser Leu
305                 310                 315                 320
ccg gtg tac gaa cgc atc ctg ggc aac ctc gag cac tac cgc cag cgc 1008

Pro Val Tyr Glu Arg Ile Leu Gly Asn Leu Glu His Tyr Arg Gln Arg
                325                 330                 335
atc gtg ctg cgc ccg cag gaa atc agc aac gcg ccc gag ctg gtg cgc      1056
Ile Val Leu Arg Pro Gln Glu Ile Ser Asn Ala Pro Glu Leu Val Arg
            340                 345                 350
cgc ctg ggc gtg att gcc atc aac acg gcg ctg gag ttc gac atc tac      1104
Arg Leu Gly Val Ile Ala Ile Asn Thr Ala Leu Glu Phe Asp Ile Tyr
        355                 360                 365
ggc aac gtc aac tcc acc cat gtg ggc ggc acc cat atg atg aac ggc      1152
Gly Asn Val Asn Ser Thr His Val Gly Gly Thr His Met Met Asn Gly
    370                 375                 380
atc ggc ggt tcg ggg gac ttt gtg cgc aac gcg cac ctg tcc atc ttc      1200
Ile Gly Gly Ser Gly Asp Phe Val Arg Asn Ala His Leu Ser Ile Phe
385                 390                 395                 400
gtg acc aag tcc atg gcc agg aac ggc gat att tcc agc gtg gtg ccc      1248
Val Thr Lys Ser Met Ala Arg Asn Gly Asp Ile Ser Ser Val Val Pro
                405                 410                 415
atg gtc tcg cat gtg gac cac aac gag cac gac gtc gat atc ctg gtg      1296
Met Val Ser His Val Asp His Asn Glu His Asp Val Asp Ile Leu Val
            420                 425                 430
acc gag tgc ggg ctg gcg gac ctg cgc ggc ctg gcg ccg cgc gag cgc      1344
Thr Glu Cys Gly Leu Ala Asp Leu Arg Gly Leu Ala Pro Arg Glu Arg
        435                 440                 445
gct cgc gcc atc atc gcc cat tgc gtg cat ccc tcg tac cgc gat gcg      1392
Ala Arg Ala Ile Ile Ala His Cys Val His Pro Ser Tyr Arg Asp Ala
    450                 455                 460
ctg cgc gat tac ttc gag cgc gcc tgc agg cgc ggc ggg caa acg tcc      1440
Leu Arg Asp Tyr Phe Glu Arg Ala Cys Arg Arg Gly Gly Gln Thr Ser
465                 470                 475                 480
cat ctg ctg gag gag gcc ttc tcc tgg cac cag cgc tac aac gaa acc      1488
His Leu Leu Glu Glu Ala Phe Ser Trp His Gln Arg Tyr Asn Glu Thr
                485                 490                 495
gac acg atg cgg ctg gcg gca ccg gcg gcc cgg cgc gtg gcc tga          1533
Asp Thr Met Arg Leu Ala Ala Pro Ala Ala Arg Arg Val Ala
            500                 505                 510


<210> 50
<211> 510
<212> PRT
<213> Bordetella pertussis

<400> 50
Met Ser Gly Asp Ser Met His Leu Glu Arg Ile Arg His Pro Gly Leu
1               5                   10                  15
Arg Ala Arg Leu Ala Ser Pro Gln Glu Ala Ala Arg Leu Val His Asp
            20                  25                  30
Gly Met Thr Val Gly Met Ser Glu Phe Thr Arg Ala Gly Asp Cys Lys
        35                  40                  45
Ser Val Pro Ala Ala Leu Ala Glu Arg Ala Ala Ala Glu Pro Leu Arg
    50                  55                  60
Ile Thr Leu Leu Thr Gly Ala Ser Leu Gly His Asp Thr Asp Lys Leu
65                  70                  75                  80
Leu Ala Gln Ala Gly Val Leu Ala Arg Arg Met Pro Phe Gln Val Asp
                85                  90                  95
Thr Thr Leu Arg Arg Lys Ile Asn Gln Gly Glu Ile Ala Phe Val Asp
            100                 105                 110
Gln His Leu Ser Glu Thr Val Glu Gln Leu Arg Ala Gly His Ile Gly
        115                 120                 125
Pro Ile Asp Ile Ala Ile Val Glu Ala Ala Ala Ile Thr Glu Asp Gly
    130                 135                 140
Gly Ile Val Pro Thr Met Ser Val Gly Asn Ser Ala Ser Phe Val Glu
145                 150                 155                 160
Gln Ala Arg Leu Val Ile Val Glu Leu Asn Leu Gly Val Pro Ala Ala
                165                 170                 175
Ile Glu Gly Leu His Asp Ile Val Ile Pro Gly Ala Arg Pro Gly Arg
            180                 185                 190
Gln Pro Leu Pro Leu Val Ser Val Asp Gln Arg Ile Gly Ala Pro Cys
        195                 200                 205
Ile Ala Val Asp Pro Asp Lys Ile Ala Ala Ile Val Val Thr Asn Glu
    210                 215                 220
Pro Asp Ser Pro Ser Asn Ala Leu Pro Pro Asp Glu Glu Ile Gly Ala

225                 230                 235                 240
Ile Ala Gly His Ile Val Gly Phe Leu Arg Gln Glu Val Asn Gln Gly
                245                 250                 255
Arg Leu Ser Arg Ser Leu Leu Pro Leu Gln Ala Gly Ile Gly Thr Ile
            260                 265                 270
Ala Asn Ala Val Leu His Gly Phe Gly Glu Ser Glu Phe Glu Gly Leu
        275                 280                 285
Thr Met Tyr Ser Glu Val Leu Gln Asp Ser Ala Ile Glu Leu Leu Asp
    290                 295                 300
Gln Gly Lys Leu Ser Phe Ala Ser Ala Ser Ser Ile Thr Val Ser Leu
305                 310                 315                 320
Pro Val Tyr Glu Arg Ile Leu Gly Asn Leu Glu His Tyr Arg Gln Arg
                325                 330                 335
Ile Val Leu Arg Pro Gln Glu Ile Ser Asn Ala Pro Glu Leu Val Arg
            340                 345                 350
Arg Leu Gly Val Ile Ala Ile Asn Thr Ala Leu Glu Phe Asp Ile Tyr
        355                 360                 365
Gly Asn Val Asn Ser Thr His Val Gly Gly Thr His Met Met Asn Gly
    370                 375                 380
Ile Gly Gly Ser Gly Asp Phe Val Arg Asn Ala His Leu Ser Ile Phe
385                 390                 395                 400
Val Thr Lys Ser Met Ala Arg Asn Gly Asp Ile Ser Ser Val Val Pro
                405                 410                 415
Met Val Ser His Val Asp His Asn Glu His Asp Val Asp Ile Leu Val
            420                 425                 430
Thr Glu Cys Gly Leu Ala Asp Leu Arg Gly Leu Ala Pro Arg Glu Arg
        435                 440                 445
Ala Arg Ala Ile Ile Ala His Cys Val His Pro Ser Tyr Arg Asp Ala
    450                 455                 460
Leu Arg Asp Tyr Phe Glu Arg Ala Cys Arg Arg Gly Gly Gln Thr Ser
465                 470                 475                 480
His Leu Leu Glu Glu Ala Phe Ser Trp His Gln Arg Tyr Asn Glu Thr
                485                 490                 495
Asp Thr Met Arg Leu Ala Ala Pro Ala Ala Arg Arg Val Ala
            500                 505                 510

<210> 51
<211> 1515
<212> DNA
<213> Bacillus cereus

<220>
<221> CDS
<222> (1)..(1515)

<400> 51
atg gag aac aat tta gat aga atc aga gat caa cgt cta aaa gat cgg      48
Met Glu Asn Asn Leu Asp Arg Ile Arg Asp Gln Arg Leu Lys Asp Arg
1               5                   10                  15
gta gtt aca cct gaa gaa gca gct tcc tgg att caa agt gga atg act      96
Val Val Thr Pro Glu Glu Ala Ala Ser Trp Ile Gln Ser Gly Met Thr
            20                  25                  30
tta ggc tta agt ggg ttt aca cgt gca ggt gat gta aaa gca gtc cca      144
Leu Gly Leu Ser Gly Phe Thr Arg Ala Gly Asp Val Lys Ala Val Pro
        35                  40                  45
ttt gcg ctt gta aac cga gtt aag aat gat gaa tct ttt aaa gta aat      192
Phe Ala Leu Val Asn Arg Val Lys Asn Asp Glu Ser Phe Lys Val Asn
    50                  55                  60
gtt tat act ggg gct tct tta gga tcg gat gta gac aaa tta ttt gct      240
Val Tyr Thr Gly Ala Ser Leu Gly Ser Asp Val Asp Lys Leu Phe Ala
65                  70                  75                  80
gag gca gga att tta ggg aaa aga tta cct ttc caa gct gac gct act      288
Glu Ala Gly Ile Leu Gly Lys Arg Leu Pro Phe Gln Ala Asp Ala Thr
                85                  90                  95
atg cga aaa gga att aat aac gga gac ttt tta ttt gtg gat caa cac      336
Met Arg Lys Gly Ile Asn Asn Gly Asp Phe Leu Phe Val Asp Gln His
            100                 105                 110
ttg tct cat aca gca gag tta ctc cgt gct gac gtt ctg gat ata gat      384
Leu Ser His Thr Ala Glu Leu Leu Arg Ala Asp Val Leu Asp Ile Asp
        115                 120                 125
ttc gct att ttg gaa gcg gtt gcg att aca gag gac gga atg att att      432
Phe Ala Ile Leu Glu Ala Val Ala Ile Thr Glu Asp Gly Met Ile Ile

130                 135                 140
cca acc act tca att gga aat tcc tta gcg ttt tcc tta aat gct aag        480
Pro Thr Thr Ser Ile Gly Asn Ser Leu Ala Phe Ser Leu Asn Ala Lys
145                 150                 155                 160
tct att att att gaa atg aat atg gct caa tcc gcg caa tta gag ggg        528
Ser Ile Ile Ile Glu Met Asn Met Ala Gln Ser Ala Gln Leu Glu Gly
                165                 170                 175
cta cat gat cta tat gaa cca ggt aaa caa ggg gag agg ctt cca att        576
Leu His Asp Leu Tyr Glu Pro Gly Lys Gln Gly Glu Arg Leu Pro Ile
            180                 185                 190
ccg ctt gtg aaa aca gat gac cga att gga aca atc ggt att cca att        624
Pro Leu Val Lys Thr Asp Asp Arg Ile Gly Thr Ile Gly Ile Pro Ile
        195                 200                 205
gat cct gaa aaa gta aag gga att gtg ttt acg aat caa ttg gat tcg        672
Asp Pro Glu Lys Val Lys Gly Ile Val Phe Thr Asn Gln Leu Asp Ser
    210                 215                 220
cca tcg aca att gtt cct ccg gat gaa gaa act gtt att atg gca cag        720
Pro Ser Thr Ile Val Pro Pro Asp Glu Glu Thr Val Ile Met Ala Gln
225                 230                 235                 240
cat tta ata gaa ttc ctt cga gaa gaa gtc aaa gta ggc cga tta aca        768
His Leu Ile Glu Phe Leu Arg Glu Glu Val Lys Val Gly Arg Leu Thr
                245                 250                 255
aat cgt tta gcg ccg tta caa tca gga att ggt tca gtg gct aac gcc        816
Asn Arg Leu Ala Pro Leu Gln Ser Gly Ile Gly Ser Val Ala Asn Ala
            260                 265                 270
gta ttg cat gga atg tta gat tca gaa ttt gaa gat tta gaa gta tat        864
Val Leu His Gly Met Leu Asp Ser Glu Phe Glu Asp Leu Glu Val Tyr
        275                 280                 285
tcc gag gtt tta caa gat gca gtt ttt gat ctt atg gat gct gga aaa        912
Ser Glu Val Leu Gln Asp Ala Val Phe Asp Leu Met Asp Ala Gly Lys
    290                 295                 300
gtc aat ttt gct tct tgt tgt tct atc acg ctt tct gaa gag aaa atg        960
Val Asn Phe Ala Ser Cys Cys Ser Ile Thr Leu Ser Glu Glu Lys Met
305                 310                 315                 320
caa caa gta ttt tct aac ttt gaa aaa tat cgt gac aaa tta atg atg        1008
Gln Gln Val Phe Ser Asn Phe Glu Lys Tyr Arg Asp Lys Leu Met Met
                325                 330                 335
cgt cca caa gag att tcc aat cat cct gaa att att cgt cgc ctt gga        1056
Arg Pro Gln Glu Ile Ser Asn His Pro Glu Ile Ile Arg Arg Leu Gly
            340                 345                 350
tta atc tca att aat act gca tta gaa tta gat ata tac gga aat gtt        1104
Leu Ile Ser Ile Asn Thr Ala Leu Glu Leu Asp Ile Tyr Gly Asn Val
        355                 360                 365
aac tct act cac gtt tta ggt aca aaa atg atg aat ggt att ggt ggg        1152
Asn Ser Thr His Val Leu Gly Thr Lys Met Met Asn Gly Ile Gly Gly
    370                 375                 380
tct ggt gat ttt gca aga aat gct cgc cta gct atc ttt gtt act aaa        1200
Ser Gly Asp Phe Ala Arg Asn Ala Arg Leu Ala Ile Phe Val Thr Lys
385                 390                 395                 400
tcg att gcg aaa ggt ggt aac att tca agt atc gtt cct ttc gtt tct        1248
Ser Ile Ala Lys Gly Gly Asn Ile Ser Ser Ile Val Pro Phe Val Ser
                405                 410                 415
cat gta gac cat act gaa cat gat gta gat gtt atc gtc act gaa cag        1296
His Val Asp His Thr Glu His Asp Val Asp Val Ile Val Thr Glu Gln
            420                 425                 430
gga tat gcc gac tta agg gga ctt gcg cca aga gaa aga gtg gaa ctt        1344
Gly Tyr Ala Asp Leu Arg Gly Leu Ala Pro Arg Glu Arg Val Glu Leu
        435                 440                 445
att att gag aat tgc gca cat cca atg tat cgt gaa cag cta cga gct        1392
Ile Ile Glu Asn Cys Ala His Pro Met Tyr Arg Glu Gln Leu Arg Ala
    450                 455                 460
tat tac gaa gaa gca aaa ata aga ggt gga caa act cct cat att tta        1440
Tyr Tyr Glu Glu Ala Lys Ile Arg Gly Gly Gln Thr Pro His Ile Leu
465                 470                 475                 480
gaa aaa gct ttt tct tgg cat acg aac tat gct aaa aat gga aca atg        1488
Glu Lys Ala Phe Ser Trp His Thr Asn Tyr Ala Lys Asn Gly Thr Met
                485                 490                 495
ctt gaa gcg gta gta gaa act gta tag                                    1515
Leu Glu Ala Val Val Glu Thr Val
            500

<210> 52
<211> 504
<212> PRT
<213> Bacillus cereus

<400> 52
Met Glu Asn Asn Leu Asp Arg Ile Arg Asp Gln Arg Leu Lys Asp Arg
1               5                   10                  15
Val Val Thr Pro Glu Glu Ala Ala Ser Trp Ile Gln Ser Gly Met Thr
            20                  25                  30
Leu Gly Leu Ser Gly Phe Thr Arg Ala Gly Asp Val Lys Ala Val Pro
        35                  40                  45
Phe Ala Leu Val Asn Arg Val Lys Asn Asp Glu Ser Phe Lys Val Asn
    50                  55                  60
Val Tyr Thr Gly Ala Ser Leu Gly Ser Asp Val Asp Lys Leu Phe Ala
65                  70                  75                  80
Glu Ala Gly Ile Leu Gly Lys Arg Leu Pro Phe Gln Ala Asp Ala Thr
                85                  90                  95
Met Arg Lys Gly Ile Asn Asn Gly Asp Phe Leu Phe Val Asp Gln His
            100                 105                 110
Leu Ser His Thr Ala Glu Leu Leu Arg Ala Asp Val Leu Asp Ile Asp
        115                 120                 125
Phe Ala Ile Leu Glu Ala Val Ala Ile Thr Glu Asp Gly Met Ile Ile
    130                 135                 140
Pro Thr Thr Ser Ile Gly Asn Ser Leu Ala Phe Ser Leu Asn Ala Lys
145                 150                 155                 160
Ser Ile Ile Ile Glu Met Asn Met Ala Gln Ser Ala Gln Leu Glu Gly
                165                 170                 175
Leu His Asp Leu Tyr Glu Pro Gly Lys Gln Gly Glu Arg Leu Pro Ile
            180                 185                 190
Pro Leu Val Lys Thr Asp Asp Arg Ile Gly Thr Ile Gly Ile Pro Ile
        195                 200                 205
Asp Pro Glu Lys Val Lys Gly Ile Val Phe Thr Asn Gln Leu Asp Ser
    210                 215                 220
Pro Ser Thr Ile Val Pro Pro Asp Glu Glu Thr Val Ile Met Ala Gln
225                 230                 235                 240
His Leu Ile Glu Phe Leu Arg Glu Glu Val Lys Val Gly Arg Leu Thr
                245                 250                 255
Asn Arg Leu Ala Pro Leu Gln Ser Gly Ile Gly Ser Val Ala Asn Ala
            260                 265                 270
Val Leu His Gly Met Leu Asp Ser Glu Phe Glu Asp Leu Glu Val Tyr
        275                 280                 285
Ser Glu Val Leu Gln Asp Ala Val Phe Asp Leu Met Asp Ala Gly Lys
    290                 295                 300
Val Asn Phe Ala Ser Cys Cys Ser Ile Thr Leu Ser Glu Glu Lys Met
305                 310                 315                 320
Gln Gln Val Phe Ser Asn Phe Glu Lys Tyr Arg Asp Lys Leu Met Met
                325                 330                 335
Arg Pro Gln Glu Ile Ser Asn His Pro Glu Ile Ile Arg Arg Leu Gly
            340                 345                 350
Leu Ile Ser Ile Asn Thr Ala Leu Glu Leu Asp Ile Tyr Gly Asn Val
        355                 360                 365
Asn Ser Thr His Val Leu Gly Thr Lys Met Met Asn Gly Ile Gly Gly
    370                 375                 380
Ser Gly Asp Phe Ala Arg Asn Ala Arg Leu Ala Ile Phe Val Thr Lys
385                 390                 395                 400
Ser Ile Ala Lys Gly Gly Asn Ile Ser Ser Ile Val Pro Phe Val Ser
                405                 410                 415
His Val Asp His Thr Glu His Asp Val Asp Val Ile Val Thr Glu Gln
            420                 425                 430
Gly Tyr Ala Asp Leu Arg Gly Leu Ala Pro Arg Glu Arg Val Glu Leu
        435                 440                 445
Ile Ile Glu Asn Cys Ala His Pro Met Tyr Arg Glu Gln Leu Arg Ala
    450                 455                 460
Tyr Tyr Glu Glu Ala Lys Ile Arg Gly Gly Gln Thr Pro His Ile Leu
465                 470                 475                 480
Glu Lys Ala Phe Ser Trp His Thr Asn Tyr Ala Lys Asn Gly Thr Met
                485                 490                 495
Leu Glu Ala Val Val Glu Thr Val
            500

<210> 53

<211> 1515
<212> DNA
<213> Bacillus anthracis

<220>
<221> CDS
<222> (1)..(1515)

<400> 53
atg gag aat aat ttg gat aga att agg gat caa cgt ctg aaa gat cgc 48
Met Glu Asn Asn Leu Asp Arg Ile Arg Asp Gln Arg Leu Lys Asp Arg
1               5                   10                  15
gta gtt aca cct gaa gaa gca gct tca tgg att gaa agt gga atg act 96
Val Val Thr Pro Glu Glu Ala Ala Ser Trp Ile Glu Ser Gly Met Thr
            20                  25                  30
tta ggc tta agt ggg ttt aca cgt gca ggt gat gtg aaa gca gtc cca 144
Leu Gly Leu Ser Gly Phe Thr Arg Ala Gly Asp Val Lys Ala Val Pro
        35                  40                  45
ttt gcg ctt gta aac cga gtt aag aat gat aca tct ttt aaa gta aat 192
Phe Ala Leu Val Asn Arg Val Lys Asn Asp Thr Ser Phe Lys Val Asn
    50                  55                  60
gtt tat act ggt gcc tct tta ggt tct gat gta gat aaa tta ttt gcg 240
Val Tyr Thr Gly Ala Ser Leu Gly Ser Asp Val Asp Lys Leu Phe Ala
65                  70                  75                  80
gaa gca gga att tta ggg aaa aga ttg cct ttc caa gct gat gcg act 288
Glu Ala Gly Ile Leu Gly Lys Arg Leu Pro Phe Gln Ala Asp Ala Thr
                85                  90                  95
atg cga aaa gga att aat aac gga gat ttt tta ttt gta gac cag cac 336
Met Arg Lys Gly Ile Asn Asn Gly Asp Phe Leu Phe Val Asp Gln His
            100                 105                 110
tta tct cat aca gca gag tta ctt cgc gct gac gtt atg gat gta gat 384
Leu Ser His Thr Ala Glu Leu Leu Arg Ala Asp Val Met Asp Val Asp
        115                 120                 125
ttt gct att ttg gga gcg gtt gcg att act gaa gat ggc atg att att 432
Phe Ala Ile Leu Gly Ala Val Ala Ile Thr Glu Asp Gly Met Ile Ile
    130                 135                 140
cca aca act tca ata gga aat tct tta gcg ttt tct tta aat gct aag 480
Pro Thr Thr Ser Ile Gly Asn Ser Leu Ala Phe Ser Leu Asn Ala Lys
145                 150                 155                 160
tct atc att att gaa atg aat atg gcc caa tcc gcg cag cta gaa ggg 528
Ser Ile Ile Ile Glu Met Asn Met Ala Gln Ser Ala Gln Leu Glu Gly
                165                 170                 175
cta cat gat tta tat gaa cca ggt aaa caa gga gaa aga ctt cca att 576
Leu His Asp Leu Tyr Glu Pro Gly Lys Gln Gly Glu Arg Leu Pro Ile
            180                 185                 190
cca ctc gta aaa aca aat gat cga att ggg acg att ggc att ccg att 624
Pro Leu Val Lys Thr Asn Asp Arg Ile Gly Thr Ile Gly Ile Pro Ile
        195                 200                 205
gat gtt gaa aaa gtg aaa ggg att gta ttt acg aat caa ttg gat tca 672
Asp Val Glu Lys Val Lys Gly Ile Val Phe Thr Asn Gln Leu Asp Ser
    210                 215                 220
cca tca aca att gtt cct cca gat gaa gaa act gtt att atg gca cag 720
Pro Ser Thr Ile Val Pro Pro Asp Glu Glu Thr Val Ile Met Ala Gln
225                 230                 235                 240
cat tta ata gaa ttt ctt cga aaa gaa gtc gaa gta ggc cga tta aca 768
His Leu Ile Glu Phe Leu Arg Lys Glu Val Glu Val Gly Arg Leu Thr
                245                 250                 255
aat cgt tta gca ccg tta caa tcg gga att ggt tca gta gct aat gca 816
Asn Arg Leu Ala Pro Leu Gln Ser Gly Ile Gly Ser Val Ala Asn Ala
            260                 265                 270
gtt ctt cat gga atg tta gat tcg gag ttt gaa gat tta gaa gtg tat 864
Val Leu His Gly Met Leu Asp Ser Glu Phe Glu Asp Leu Glu Val Tyr
        275                 280                 285
tct gaa gtt tta cag gat gcg gtc ttt gat ctt atg gat gct gga aaa 912
Ser Glu Val Leu Gln Asp Ala Val Phe Asp Leu Met Asp Ala Gly Lys
    290                 295                 300
gtc aat ttt gct tca tgc tgt tcg atc aca ctt tct gag gag aaa atg 960
Val Asn Phe Ala Ser Cys Cys Ser Ile Thr Leu Ser Glu Glu Lys Met
305                 310                 315                 320
caa aaa gta ttt tcc aac ttt gaa aaa tat cgt gac aaa tta atg atg 1008
Gln Lys Val Phe Ser Asn Phe Glu Lys Tyr Arg Asp Lys Leu Met Met
                325                 330                 335

cgc ccg caa gag att tcc aat cat cct gaa att att cgc cgc ctc gga          1056
Arg Pro Gln Glu Ile Ser Asn His Pro Glu Ile Ile Arg Arg Leu Gly
            340                 345                 350
tta atc tcg att aat act gct tta gaa tta gat ata tac gga aat gtg          1104
Leu Ile Ser Ile Asn Thr Ala Leu Glu Leu Asp Ile Tyr Gly Asn Val
        355                 360                 365
aac tct act cac gtt tta ggt aca aaa atg atg aac ggt atc ggt ggt          1152
Asn Ser Thr His Val Leu Gly Thr Lys Met Met Asn Gly Ile Gly Gly
    370                 375                 380
tct ggt gac ttt gca aga aat gct cgt ttg gct atc ttt gtt act aaa          1200
Ser Gly Asp Phe Ala Arg Asn Ala Arg Leu Ala Ile Phe Val Thr Lys
385                 390                 395                 400
tcg att gcg aaa ggt ggt aac att tca agt atc gtt cct ttc gcc tca          1248
Ser Ile Ala Lys Gly Gly Asn Ile Ser Ser Ile Val Pro Phe Ala Ser
                405                 410                 415
cat ata gac cat acg gaa cat gat gta gat gtt atc gtc act gag caa          1296
His Ile Asp His Thr Glu His Asp Val Asp Val Ile Val Thr Glu Gln
            420                 425                 430
gga tat gct gac tta aga gga ctt gcg cca agt gaa aga gtg gaa ctc          1344
Gly Tyr Ala Asp Leu Arg Gly Leu Ala Pro Ser Glu Arg Val Glu Leu
        435                 440                 445
att att gag aat tgt gca cat cta atg tat cgt gat caa cta cga gct          1392
Ile Ile Glu Asn Cys Ala His Leu Met Tyr Arg Asp Gln Leu Arg Ala
    450                 455                 460
tat tat gag gaa gcg aaa aca aga ggt gga caa act cct cat att tta          1440
Tyr Tyr Glu Glu Ala Lys Thr Arg Gly Gly Gln Thr Pro His Ile Leu
465                 470                 475                 480
gag aaa gct ttt tct tgg cat acg aat tat gct aaa aat gga acg atg          1488
Glu Lys Ala Phe Ser Trp His Thr Asn Tyr Ala Lys Asn Gly Thr Met
                485                 490                 495
ctt gaa gag gta gta gaa act gta tag                                      1515
Leu Glu Glu Val Val Glu Thr Val
            500

<210> 54
<211> 504
<212> PRT
<213> Bacillus anthracis

<400> 54
Met Glu Asn Asn Leu Asp Arg Ile Arg Asp Gln Arg Leu Lys Asp Arg
1               5                   10                  15
Val Val Thr Pro Glu Glu Ala Ala Ser Trp Ile Glu Ser Gly Met Thr
            20                  25                  30
Leu Gly Leu Ser Gly Phe Thr Arg Ala Gly Asp Val Lys Ala Val Pro
        35                  40                  45
Phe Ala Leu Val Asn Arg Val Lys Asn Asp Thr Ser Phe Lys Val Asn
    50                  55                  60
Val Tyr Thr Gly Ala Ser Leu Gly Ser Asp Val Asp Lys Leu Phe Ala
65                  70                  75                  80
Glu Ala Gly Ile Leu Gly Lys Arg Leu Pro Phe Gln Ala Asp Ala Thr
                85                  90                  95
Met Arg Lys Gly Ile Asn Asn Gly Asp Phe Leu Phe Val Asp Gln His
            100                 105                 110
Leu Ser His Thr Ala Glu Leu Leu Arg Ala Asp Val Met Asp Val Asp
        115                 120                 125
Phe Ala Ile Leu Gly Ala Val Ala Ile Thr Glu Asp Gly Met Ile Ile
    130                 135                 140
Pro Thr Thr Ser Ile Gly Asn Ser Leu Ala Phe Ser Leu Asn Ala Lys
145                 150                 155                 160
Ser Ile Ile Ile Glu Met Asn Met Ala Gln Ser Ala Gln Leu Glu Gly
                165                 170                 175
Leu His Asp Leu Tyr Glu Pro Gly Lys Gln Gly Glu Arg Leu Pro Ile
            180                 185                 190
Pro Leu Val Lys Thr Asn Asp Arg Ile Gly Thr Ile Gly Ile Pro Ile
        195                 200                 205
Asp Val Glu Lys Val Lys Gly Ile Val Phe Thr Asn Gln Leu Asp Ser
    210                 215                 220
Pro Ser Thr Ile Val Pro Pro Asp Glu Glu Thr Val Ile Met Ala Gln
225                 230                 235                 240
His Leu Ile Glu Phe Leu Arg Lys Glu Val Glu Val Gly Arg Leu Thr

                245                 250                 255
Asn Arg Leu Ala Pro Leu Gln Ser Gly Ile Gly Ser Val Ala Asn Ala
            260                 265                 270
Val Leu His Gly Met Leu Asp Ser Glu Phe Glu Asp Leu Glu Val Tyr
        275                 280                 285
Ser Glu Val Leu Gln Asp Ala Val Phe Asp Leu Met Asp Ala Gly Lys
    290                 295                 300
Val Asn Phe Ala Ser Cys Cys Ser Ile Thr Leu Ser Glu Glu Lys Met
305                 310                 315                 320
Gln Lys Val Phe Ser Asn Phe Glu Lys Tyr Arg Asp Lys Leu Met Met
                325                 330                 335
Arg Pro Gln Glu Ile Ser Asn His Pro Glu Ile Ile Arg Arg Leu Gly
            340                 345                 350
Leu Ile Ser Ile Asn Thr Ala Leu Glu Leu Asp Ile Tyr Gly Asn Val
        355                 360                 365
Asn Ser Thr His Val Leu Gly Thr Lys Met Met Asn Gly Ile Gly Gly
    370                 375                 380
Ser Gly Asp Phe Ala Arg Asn Ala Arg Leu Ala Ile Phe Val Thr Lys
385                 390                 395                 400
Ser Ile Ala Lys Gly Gly Asn Ile Ser Ser Ile Val Pro Phe Ala Ser
                405                 410                 415
His Ile Asp His Thr Glu His Asp Val Asp Val Ile Val Thr Glu Gln
            420                 425                 430
Gly Tyr Ala Asp Leu Arg Gly Leu Ala Pro Ser Glu Arg Val Glu Leu
        435                 440                 445
Ile Ile Glu Asn Cys Ala His Leu Met Tyr Arg Asp Gln Leu Arg Ala
    450                 455                 460
Tyr Tyr Glu Glu Ala Lys Thr Arg Gly Gly Gln Thr Pro His Ile Leu
465                 470                 475                 480
Glu Lys Ala Phe Ser Trp His Thr Asn Tyr Ala Lys Asn Gly Thr Met
                485                 490                 495
Leu Glu Glu Val Val Glu Thr Val
            500

<210> 55
<211> 1494
<212> DNA
<213> Pseudomonas syringae

<220>
<221> CDS
<222> (1)..(1494)

<400> 55
atg tac cgt gac cgt atc cgc ctg ccc tcc ctg atg agc aag gtt atg      48
Met Tyr Arg Asp Arg Ile Arg Leu Pro Ser Leu Met Ser Lys Val Met
1               5                   10                  15
agc gca gcc gat gct gct gca ttg att gaa gac ggc atg acc gtc ggc      96
Ser Ala Ala Asp Ala Ala Ala Leu Ile Glu Asp Gly Met Thr Val Gly
            20                  25                  30
atg agc ggc ttc acc cgc gca ggc gaa gcc aag gcc gtg cct cat gcg      144
Met Ser Gly Phe Thr Arg Ala Gly Glu Ala Lys Ala Val Pro His Ala
        35                  40                  45
ctg gcc gag cgc gcc aag gtg aca ccc ctg aaa atc agc ctg atg acg      192
Leu Ala Glu Arg Ala Lys Val Thr Pro Leu Lys Ile Ser Leu Met Thr
    50                  55                  60
ggt gcc agc ctg ggc aat gac ctg gac aag caa ctg acc gaa tcc ggc      240
Gly Ala Ser Leu Gly Asn Asp Leu Asp Lys Gln Leu Thr Glu Ser Gly
65                  70                  75                  80
gtg ctg tcc cgg cgc atg ccg ttt cag gtc gac agc act ctg cgc aag      288
Val Leu Ser Arg Arg Met Pro Phe Gln Val Asp Ser Thr Leu Arg Lys
                85                  90                  95
gcg atc aac aac ggc acg gtg atg ttc atc gac cag cat ttg tcc gat      336
Ala Ile Asn Asn Gly Thr Val Met Phe Ile Asp Gln His Leu Ser Asp
            100                 105                 110
acc gtc gag ttg ttg cgc aat cgg cag atc aag gcc gtg gac ctg gcg      384
Thr Val Glu Leu Leu Arg Asn Arg Gln Ile Lys Ala Val Asp Leu Ala
        115                 120                 125
gtg atc gag tgt gtg gca att acc gag gaa ggc cac ctg gtg ctg agc      432
Val Ile Glu Cys Val Ala Ile Thr Glu Glu Gly His Leu Val Leu Ser
    130                 135                 140
acc tcc gtt ggc aac tcg gcc agt ttc gct att ctg gcc aaa cag gtc      480

Thr Ser Val Gly Asn Ser Ala Ser Phe Ala Ile Leu Ala Lys Gln Val
145                 150                 155                 160
atc gtg gaa atc aac ctg tcc cag cct ctg gag ctg gaa ggc ctg cat      528
Ile Val Glu Ile Asn Leu Ser Gln Pro Leu Glu Leu Glu Gly Leu His
                165                 170                 175
gac atc tat ata ccc agc tac cgg ccg acg cgc ctg ccg atc ccg gtg      576
Asp Ile Tyr Ile Pro Ser Tyr Arg Pro Thr Arg Leu Pro Ile Pro Val
            180                 185                 190
ctg gaa gcc gat tcg cgc atc ggc agc cat gcg gtg aag atc gac ccg      624
Leu Glu Ala Asp Ser Arg Ile Gly Ser His Ala Val Lys Ile Asp Pro
        195                 200                 205
gcg aag atc gtc ggc atc gtt atc agc aac cag cct gac tcg cct tcc      672
Ala Lys Ile Val Gly Ile Val Ile Ser Asn Gln Pro Asp Ser Pro Ser
    210                 215                 220
acc gtc acg cca cct gac gca gac act cag gcc atc gcc aac cac ctg      720
Thr Val Thr Pro Pro Asp Ala Asp Thr Gln Ala Ile Ala Asn His Leu
225                 230                 235                 240
gtg gag ttc ttc aag aag gag gtg cgc gaa gac cgg ctc acc gac aaa      768
Val Glu Phe Phe Lys Lys Glu Val Arg Glu Asp Arg Leu Thr Asp Lys
                245                 250                 255
ctg atg ccc ctg cag gcg ggc att ggc aac atc gcc aac gca gtc atg      816
Leu Met Pro Leu Gln Ala Gly Ile Gly Asn Ile Ala Asn Ala Val Met
            260                 265                 270
cat ggc ctg ctg gaa tcg cca ttc act gac ctg acc atg tat tcc gaa      864
His Gly Leu Leu Glu Ser Pro Phe Thr Asp Leu Thr Met Tyr Ser Glu
        275                 280                 285
gta ttg cag gac tcg aca ttc gac ctg ttc gac gcc ggc aaa ctg aca      912
Val Leu Gln Asp Ser Thr Phe Asp Leu Phe Asp Ala Gly Lys Leu Thr
    290                 295                 300
ttt gct tcc ggc agc tcc atg acc ctg tca gcg gcc aaa cac gcc gaa      960
Phe Ala Ser Gly Ser Ser Met Thr Leu Ser Ala Ala Lys His Ala Glu
305                 310                 315                 320
gta ttt gcc gac ttc aac cgc tat aaa tcc agg ctg gtg ctg cgc ccg      1008
Val Phe Ala Asp Phe Asn Arg Tyr Lys Ser Arg Leu Val Leu Arg Pro
                325                 330                 335
cag gaa ata tcc aat cac ccc gaa gtc atc cgg cgc ctg ggg att atc      1056
Gln Glu Ile Ser Asn His Pro Glu Val Ile Arg Arg Leu Gly Ile Ile
            340                 345                 350
ggg atc aat acc gcg ctg gag ttc gat ctg tac ggc aac gtc aac tcc      1104
Gly Ile Asn Thr Ala Leu Glu Phe Asp Leu Tyr Gly Asn Val Asn Ser
        355                 360                 365
acc cac atc tgt ggc acg cgg atg atg aac ggc att ggc ggc tct ggg      1152
Thr His Ile Cys Gly Thr Arg Met Met Asn Gly Ile Gly Gly Ser Gly
    370                 375                 380
gat ttt tcg cgc aac gcg cac ctg gcg ata ttc gtc acc aag tcg att      1200
Asp Phe Ser Arg Asn Ala His Leu Ala Ile Phe Val Thr Lys Ser Ile
385                 390                 395                 400
gcc aag ggc ggt gcc att tcc agc gtt gtg ccg atg gtc agc cat gtc      1248
Ala Lys Gly Gly Ala Ile Ser Ser Val Val Pro Met Val Ser His Val
                405                 410                 415
gac cac acc gag cat gac gtc gac att ctg gtg acc gag cag ggg ctg      1296
Asp His Thr Glu His Asp Val Asp Ile Leu Val Thr Glu Gln Gly Leu
            420                 425                 430
gcc gat tta cgc ggg ctg gca cct cgc gag cgg gcg cgg gtg atc atc      1344
Ala Asp Leu Arg Gly Leu Ala Pro Arg Glu Arg Ala Arg Val Ile Ile
        435                 440                 445
gat aac tgc gta cat gct gat tat cgc gat gcg ttg aac gcc tat ttc      1392
Asp Asn Cys Val His Ala Asp Tyr Arg Asp Ala Leu Asn Ala Tyr Phe
    450                 455                 460
gac gcc gcc tgc gcg ctg ggc ggg cat acg ccg cac atc ctt cgg gaa      1440
Asp Ala Ala Cys Ala Leu Gly Gly His Thr Pro His Ile Leu Arg Glu
465                 470                 475                 480
gca ctg agt tgg cac atc aac ctg gaa gaa aac ggg cgc atg ctg ccg      1488
Ala Leu Ser Trp His Ile Asn Leu Glu Glu Asn Gly Arg Met Leu Pro
                485                 490                 495
acc tga                                                              1494
Thr

<210> 56
<211> 497

<212> PRT
<213> Pseudomonas syringae

<400> 56
Met Tyr Arg Asp Arg Ile Arg Leu Pro Ser Leu Met Ser Lys Val Met
1               5                   10                  15
Ser Ala Ala Asp Ala Ala Ala Leu Ile Glu Asp Gly Met Thr Val Gly
            20                  25                  30
Met Ser Gly Phe Thr Arg Ala Gly Glu Ala Lys Ala Val Pro His Ala
        35                  40                  45
Leu Ala Glu Arg Ala Lys Val Thr Pro Leu Lys Ile Ser Leu Met Thr
    50                  55                  60
Gly Ala Ser Leu Gly Asn Asp Leu Asp Lys Gln Leu Thr Glu Ser Gly
65                  70                  75                  80
Val Leu Ser Arg Arg Met Pro Phe Gln Val Asp Ser Thr Leu Arg Lys
                85                  90                  95
Ala Ile Asn Asn Gly Thr Val Met Phe Ile Asp Gln His Leu Ser Asp
            100                 105                 110
Thr Val Glu Leu Leu Arg Asn Arg Gln Ile Lys Ala Val Asp Leu Ala
        115                 120                 125
Val Ile Glu Cys Val Ala Ile Thr Glu Glu Gly His Leu Val Leu Ser
    130                 135                 140
Thr Ser Val Gly Asn Ser Ala Ser Phe Ala Ile Leu Ala Lys Gln Val
145                 150                 155                 160
Ile Val Glu Ile Asn Leu Ser Gln Pro Leu Glu Leu Glu Gly Leu His
                165                 170                 175
Asp Ile Tyr Ile Pro Ser Tyr Arg Pro Thr Arg Leu Pro Ile Pro Val
            180                 185                 190
Leu Glu Ala Asp Ser Arg Ile Gly Ser His Ala Val Lys Ile Asp Pro
        195                 200                 205
Ala Lys Ile Val Gly Ile Val Ile Ser Asn Gln Pro Asp Ser Pro Ser
    210                 215                 220
Thr Val Thr Pro Pro Asp Ala Asp Thr Gln Ala Ile Ala Asn His Leu
225                 230                 235                 240
Val Glu Phe Phe Lys Lys Glu Val Arg Glu Asp Arg Leu Thr Asp Lys
                245                 250                 255
Leu Met Pro Leu Gln Ala Gly Ile Gly Asn Ile Ala Asn Ala Val Met
            260                 265                 270
His Gly Leu Leu Glu Ser Pro Phe Thr Asp Leu Thr Met Tyr Ser Glu
        275                 280                 285
Val Leu Gln Asp Ser Thr Phe Asp Leu Phe Asp Ala Gly Lys Leu Thr
    290                 295                 300
Phe Ala Ser Gly Ser Ser Met Thr Leu Ser Ala Ala Lys His Ala Glu
305                 310                 315                 320
Val Phe Ala Asp Phe Asn Arg Tyr Lys Ser Arg Leu Val Leu Arg Pro
                325                 330                 335
Gln Glu Ile Ser Asn His Pro Glu Val Ile Arg Arg Leu Gly Ile Ile
            340                 345                 350
Gly Ile Asn Thr Ala Leu Glu Phe Asp Leu Tyr Gly Asn Val Asn Ser
        355                 360                 365
Thr His Ile Cys Gly Thr Arg Met Met Asn Gly Ile Gly Gly Ser Gly
    370                 375                 380
Asp Phe Ser Arg Asn Ala His Leu Ala Ile Phe Val Thr Lys Ser Ile
385                 390                 395                 400
Ala Lys Gly Gly Ala Ile Ser Ser Val Val Pro Met Val Ser His Val
                405                 410                 415
Asp His Thr Glu His Asp Val Asp Ile Leu Val Thr Glu Gln Gly Leu
            420                 425                 430
Ala Asp Leu Arg Gly Leu Ala Pro Arg Glu Arg Ala Arg Val Ile Ile
        435                 440                 445
Asp Asn Cys Val His Ala Asp Tyr Arg Asp Ala Leu Asn Ala Tyr Phe
    450                 455                 460
Asp Ala Ala Cys Ala Leu Gly Gly His Thr Pro His Ile Leu Arg Glu
465                 470                 475                 480
Ala Leu Ser Trp His Ile Asn Leu Glu Glu Asn Gly Arg Met Leu Pro
                485                 490                 495
Thr

<210> 57
<211> 1494
<212> DNA

<213> Pseudomonas putida

<220>
<221> CDS
<222> (1)..(1494)

<400> 57
atg tac cgt gat cgt atc cgc ttg tcc tcc ctg cac agc aag gta atg      48
Met Tyr Arg Asp Arg Ile Arg Leu Ser Ser Leu His Ser Lys Val Met
1               5                   10                  15
agt gcg gct gat gcc gct ggt ctg atc gag gac ggc atg act gtc ggc      96
Ser Ala Ala Asp Ala Ala Gly Leu Ile Glu Asp Gly Met Thr Val Gly
            20                  25                  30
atg agc ggc ttc acc cgc gcc ggc gaa gcc aag gcc gta ccg cat gca      144
Met Ser Gly Phe Thr Arg Ala Gly Glu Ala Lys Ala Val Pro His Ala
        35                  40                  45
ttg gcc gaa cgt gcc aag caa tcg cca ctg aaa atc agc ctg atg acc      192
Leu Ala Glu Arg Ala Lys Gln Ser Pro Leu Lys Ile Ser Leu Met Thr
    50                  55                  60
ggc gcc agc ctg ggc aat gac ctg gac aaa caa ctg acc gag gcc ggt      240
Gly Ala Ser Leu Gly Asn Asp Leu Asp Lys Gln Leu Thr Glu Ala Gly
65                  70                  75                  80
gtg ttg gcc cgc cgc atg ccg ttc cag gtc gac agc acc ctg cgc aaa      288
Val Leu Ala Arg Arg Met Pro Phe Gln Val Asp Ser Thr Leu Arg Lys
                85                  90                  95
gcc atc aat gac ggc cag gtg atg ttc att gac cag cac ctg tcg gaa      336
Ala Ile Asn Asp Gly Gln Val Met Phe Ile Asp Gln His Leu Ser Glu
            100                 105                 110
acc gtc gag caa ctg cgc aac cag cag ctg aag ctg ccg gac atc gcg      384
Thr Val Glu Gln Leu Arg Asn Gln Gln Leu Lys Leu Pro Asp Ile Ala
        115                 120                 125
gtc atc gaa gcc gtg gcc atc acc gag caa ggc cac atc gtg cca acc      432
Val Ile Glu Ala Val Ala Ile Thr Glu Gln Gly His Ile Val Pro Thr
    130                 135                 140
act tcg gtg ggc aac tcg gcc agc ttc gcg atc ttc gcc aag cag gtg      480
Thr Ser Val Gly Asn Ser Ala Ser Phe Ala Ile Phe Ala Lys Gln Val
145                 150                 155                 160
att gtg gag atc aac ctc tcg cac aac acc aac ctc gaa ggc ctg cac      528
Ile Val Glu Ile Asn Leu Ser His Asn Thr Asn Leu Glu Gly Leu His
                165                 170                 175
gac atc tat atc cca acc tac cgc ccg act cgc acg cca atc ccg ctg      576
Asp Ile Tyr Ile Pro Thr Tyr Arg Pro Thr Arg Thr Pro Ile Pro Leu
            180                 185                 190
gtc aag gta gac gac cgt atc ggc agc acc gcg atc ccg atc gat ccg      624
Val Lys Val Asp Asp Arg Ile Gly Ser Thr Ala Ile Pro Ile Asp Pro
        195                 200                 205
gcc aag atc gtc ggt atc gtc atc agc gac cag ccg gac tcg ccg tcc      672
Ala Lys Ile Val Gly Ile Val Ile Ser Asp Gln Pro Asp Ser Pro Ser
    210                 215                 220
act gtg ctg ccg cca gat gat gaa aca cag ggc atc gcc gac cac ctg      720
Thr Val Leu Pro Pro Asp Asp Glu Thr Gln Gly Ile Ala Asp His Leu
225                 230                 235                 240
atc aac ttc ctc aag aag gaa gtg gat gca ggc cgt atg acc aac aag      768
Ile Asn Phe Leu Lys Lys Glu Val Asp Ala Gly Arg Met Thr Asn Lys
                245                 250                 255
ctc ggc ccg ctg caa gcc ggc att ggc agc att gcc aat gcg gtg atg      816
Leu Gly Pro Leu Gln Ala Gly Ile Gly Ser Ile Ala Asn Ala Val Met
            260                 265                 270
tgc ggc ctg atc gag tcg ccg ttc gaa gac ctg acc atg tac tcc gaa      864
Cys Gly Leu Ile Glu Ser Pro Phe Glu Asp Leu Thr Met Tyr Ser Glu
        275                 280                 285
gtg ctg cag gac tcc acc ttc gac ctg atc gat gcc ggc aag ctt agc      912
Val Leu Gln Asp Ser Thr Phe Asp Leu Ile Asp Ala Gly Lys Leu Ser
    290                 295                 300
ttc gca tcc ggc agc tcg atc acc ctg tcg acg cgc cgc aat gcc gat      960
Phe Ala Ser Gly Ser Ser Ile Thr Leu Ser Thr Arg Arg Asn Ala Asp
305                 310                 315                 320
gtg ttc ggc aac ctg gag cgc tac aag gac aag ctg gtg cta cgc ccg      1008
Val Phe Gly Asn Leu Glu Arg Tyr Lys Asp Lys Leu Val Leu Arg Pro
                325                 330                 335
cag gaa atc tcc aac cac ccg gaa gtg gta cgt cgc ctg ggc atc atc      1056
Gln Glu Ile Ser Asn His Pro Glu Val Val Arg Arg Leu Gly Ile Ile

                340                 345                 350
ggt atc aac acc gca ctg gag ttc gat atc tac ggc aac gtc aac tcc      1104
Gly Ile Asn Thr Ala Leu Glu Phe Asp Ile Tyr Gly Asn Val Asn Ser
        355                 360                 365
acc cac gtc tgt ggc acc agg atg atg aac ggc atc ggt ggc tcc ggc      1152
Thr His Val Cys Gly Thr Arg Met Met Asn Gly Ile Gly Gly Ser Gly
    370                 375                 380
gac ttc gcc cgt aac gcc cac ctg gca gtg ttc gtc acc aag tcg att      1200
Asp Phe Ala Arg Asn Ala His Leu Ala Val Phe Val Thr Lys Ser Ile
385                 390                 395                 400
gcc aaa ggc ggg gcg att tcc agc gtg gtg cca atg gtc agc cat gtc      1248
Ala Lys Gly Gly Ala Ile Ser Ser Val Val Pro Met Val Ser His Val
                405                 410                 415
gac cac acc gag cac gac gtg gac atc ctg gtc acc gag cag ggc ttg      1296
Asp His Thr Glu His Asp Val Asp Ile Leu Val Thr Glu Gln Gly Leu
            420                 425                 430
gcg gac ctg cgt ggc ctg gcg ccg cgc gaa cgg gcg cga gcg atc atc      1344
Ala Asp Leu Arg Gly Leu Ala Pro Arg Glu Arg Ala Arg Ala Ile Ile
        435                 440                 445
gac aac tgt gtg cac cca gac tat cgc gct gcg ctg aac gac tac ttc      1392
Asp Asn Cys Val His Pro Asp Tyr Arg Ala Ala Leu Asn Asp Tyr Phe
    450                 455                 460
gac cgc gcc tgc cag cgt ggc ggc cat acc ccg cac atc ctg cgc gaa      1440
Asp Arg Ala Cys Gln Arg Gly Gly His Thr Pro His Ile Leu Arg Glu
465                 470                 475                 480
gcc ctg agc tgg cac gaa aac ctg gaa gaa acc ggg cgc atg ctg gcc      1488
Ala Leu Ser Trp His Glu Asn Leu Glu Glu Thr Gly Arg Met Leu Ala
                485                 490                 495
agc tga                                                              1494
Ser

<210> 58
<211> 497
<212> PRT
<213> Pseudomonas putida

<400> 58
Met Tyr Arg Asp Arg Ile Arg Leu Ser Ser Leu His Ser Lys Val Met
1               5                   10                  15
Ser Ala Ala Asp Ala Ala Gly Leu Ile Glu Asp Gly Met Thr Val Gly
            20                  25                  30
Met Ser Gly Phe Thr Arg Ala Gly Glu Ala Lys Ala Val Pro His Ala
        35                  40                  45
Leu Ala Glu Arg Ala Lys Gln Ser Pro Leu Lys Ile Ser Leu Met Thr
    50                  55                  60
Gly Ala Ser Leu Gly Asn Asp Leu Asp Lys Gln Leu Thr Glu Ala Gly
65                  70                  75                  80
Val Leu Ala Arg Arg Met Pro Phe Gln Val Asp Ser Thr Leu Arg Lys
                85                  90                  95
Ala Ile Asn Asp Gly Gln Val Met Phe Ile Asp Gln His Leu Ser Glu
            100                 105                 110
Thr Val Glu Gln Leu Arg Asn Gln Gln Leu Lys Leu Pro Asp Ile Ala
        115                 120                 125
Val Ile Glu Ala Val Ala Ile Thr Glu Gln Gly His Ile Val Pro Thr
    130                 135                 140
Thr Ser Val Gly Asn Ser Ala Ser Phe Ala Ile Phe Ala Lys Gln Val
145                 150                 155                 160
Ile Val Glu Ile Asn Leu Ser His Asn Thr Asn Leu Glu Gly Leu His
                165                 170                 175
Asp Ile Tyr Ile Pro Thr Tyr Arg Pro Thr Arg Thr Pro Ile Pro Leu
            180                 185                 190
Val Lys Val Asp Asp Arg Ile Gly Ser Thr Ala Ile Pro Ile Asp Pro
        195                 200                 205
Ala Lys Ile Val Gly Ile Val Ile Ser Asp Gln Pro Asp Ser Pro Ser
    210                 215                 220
Thr Val Leu Pro Pro Asp Asp Glu Thr Gln Gly Ile Ala Asp His Leu
225                 230                 235                 240
Ile Asn Phe Leu Lys Lys Glu Val Asp Ala Gly Arg Met Thr Asn Lys
                245                 250                 255
Leu Gly Pro Leu Gln Ala Gly Ile Gly Ser Ile Ala Asn Ala Val Met

260                 265                 270
Cys Gly Leu Ile Glu Ser Pro Phe Glu Asp Leu Thr Met Tyr Ser Glu
        275                 280                 285
Val Leu Gln Asp Ser Thr Phe Asp Leu Ile Asp Ala Gly Lys Leu Ser
    290                 295                 300
Phe Ala Ser Gly Ser Ser Ile Thr Leu Ser Thr Arg Arg Asn Ala Asp
305                 310                 315                 320
Val Phe Gly Asn Leu Glu Arg Tyr Lys Asp Lys Leu Val Leu Arg Pro
                325                 330                 335
Gln Glu Ile Ser Asn His Pro Glu Val Val Arg Arg Leu Gly Ile Ile
            340                 345                 350
Gly Ile Asn Thr Ala Leu Glu Phe Asp Ile Tyr Gly Asn Val Asn Ser
        355                 360                 365
Thr His Val Cys Gly Thr Arg Met Met Asn Gly Ile Gly Gly Ser Gly
    370                 375                 380
Asp Phe Ala Arg Asn Ala His Leu Ala Val Phe Val Thr Lys Ser Ile
385                 390                 395                 400
Ala Lys Gly Gly Ala Ile Ser Ser Val Val Pro Met Val Ser His Val
                405                 410                 415
Asp His Thr Glu His Asp Val Asp Ile Leu Val Thr Glu Gln Gly Leu
            420                 425                 430
Ala Asp Leu Arg Gly Leu Ala Pro Arg Glu Arg Ala Arg Ala Ile Ile
        435                 440                 445
Asp Asn Cys Val His Pro Asp Tyr Arg Ala Ala Leu Asn Asp Tyr Phe
    450                 455                 460
Asp Arg Ala Cys Gln Arg Gly Gly His Thr Pro His Ile Leu Arg Glu
465                 470                 475                 480
Ala Leu Ser Trp His Glu Asn Leu Glu Glu Thr Gly Arg Met Leu Ala
                485                 490                 495
Ser

<210> 59
<211> 1314
<212> DNA
<213> Clostridium tetani

<220>
<221> CDS
<222> (1)..(1314)

<400> 59
atg aat tgg gaa gat att tac aaa agt aag cta tta tca tca aag gaa      48
Met Asn Trp Glu Asp Ile Tyr Lys Ser Lys Leu Leu Ser Ser Lys Glu
1               5                   10                  15
gct gtt tta aaa tta aaa tct gaa aac cac gta gca gtt gga cat gct      96
Ala Val Leu Lys Leu Lys Ser Glu Asn His Val Ala Val Gly His Ala
            20                  25                  30
gct agt gaa cct aga ctt tta atg aaa gct tta gtt gat aat aag gaa      144
Ala Ser Glu Pro Arg Leu Leu Met Lys Ala Leu Val Asp Asn Lys Glu
        35                  40                  45
agt ttt aga gat tta aaa ctt tat caa atg ata gct tta gaa aag agt      192
Ser Phe Arg Asp Leu Lys Leu Tyr Gln Met Ile Ala Leu Glu Lys Ser
    50                  55                  60
gaa tgt gct aaa aaa ggt atg gaa aaa cat ttt agt tat aat tct ctt      240
Glu Cys Ala Lys Lys Gly Met Glu Lys His Phe Ser Tyr Asn Ser Leu
65                  70                  75                  80
ttt gta gga gga ggg agt agg gaa gct ata aat gat gga aga gct gat      288
Phe Val Gly Gly Gly Ser Arg Glu Ala Ile Asn Asp Gly Arg Ala Asp
                85                  90                  95
tat att cct tgt ttt ttt tca agg ata cca aga ctt ttt aga gag aac      336
Tyr Ile Pro Cys Phe Phe Ser Arg Ile Pro Arg Leu Phe Arg Glu Asn
            100                 105                 110
tat ata aaa tta gat gcg gca tta ata caa gtt gca cct cca gat gag      384
Tyr Ile Lys Leu Asp Ala Ala Leu Ile Gln Val Ala Pro Pro Asp Glu
        115                 120                 125
cat gga ttt tgt agc ttt ggt gtt tct tct gat tat ata aaa cct gca      432
His Gly Phe Cys Ser Phe Gly Val Ser Ser Asp Tyr Ile Lys Pro Ala
    130                 135                 140
gca gaa tgt gca aaa ctt gtt att gca gaa gta aat gat aag atg cca      480
Ala Glu Cys Ala Lys Leu Val Ile Ala Glu Val Asn Asp Lys Met Pro
145                 150                 155                 160

aga act tta ggg gat aat ttt ata cat gtt agt gac ata gat tat ata 528
Arg Thr Leu Gly Asp Asn Phe Ile His Val Ser Asp Ile Asp Tyr Ile
                165                 170                 175

gtt gaa aat tct cga gag ata cca gag cta aaa cct tct aat ata ggt 576
Val Glu Asn Ser Arg Glu Ile Pro Glu Leu Lys Pro Ser Asn Ile Gly
            180                 185                 190

gat ata gaa agg gca ata gga gaa aat tgt gca tct ctt ata aat aat 624
Asp Ile Glu Arg Ala Ile Gly Glu Asn Cys Ala Ser Leu Ile Asn Asn
        195                 200                 205

gga gat gtt ttg caa ttg ggc ata ggt gca att cca gat gca gta ctt 672
Gly Asp Val Leu Gln Leu Gly Ile Gly Ala Ile Pro Asp Ala Val Leu
    210                 215                 220

tta ttt tta aag gat aaa aag gat tta gga ata cat tca gag atg att 720
Leu Phe Leu Lys Asp Lys Lys Asp Leu Gly Ile His Ser Glu Met Ile
225                 230                 235                 240

tct gat ggg gtg gta gat tta gta aag gca ggg gta ata aat aat aag 768
Ser Asp Gly Val Val Asp Leu Val Lys Ala Gly Val Ile Asn Asn Lys
                245                 250                 255

aaa aag act ttg cat caa ggg aag ata att gtt agt ttt tta atg gga 816
Lys Lys Thr Leu His Gln Gly Lys Ile Ile Val Ser Phe Leu Met Gly
            260                 265                 270

aca aag aaa tta tat gat ttt gtg gat aat aat cct atg gtg aga atg 864
Thr Lys Lys Leu Tyr Asp Phe Val Asp Asn Asn Pro Met Val Arg Met
        275                 280                 285

tac cct gtg gat tat gtt aat aat cct gta gtt att agt aaa aat gat 912
Tyr Pro Val Asp Tyr Val Asn Asn Pro Val Val Ile Ser Lys Asn Asp
    290                 295                 300

aat atg gtc tct ata aat tcc tgt att caa ata gat ctt atg ggt caa 960
Asn Met Val Ser Ile Asn Ser Cys Ile Gln Ile Asp Leu Met Gly Gln
305                 310                 315                 320

ata gat gca gaa acc ata ggc ttt acc caa ata agt ggt gtt ggt gga 1008
Ile Asp Ala Glu Thr Ile Gly Phe Thr Gln Ile Ser Gly Val Gly Gly
                325                 330                 335

caa ata gat ttt ata aga gga gca ggt ctt agt aaa aat gga agg tct 1056
Gln Ile Asp Phe Ile Arg Gly Ala Gly Leu Ser Lys Asn Gly Arg Ser
            340                 345                 350

atc att gca ata cct tct act gta gga aat gga aaa ata tct aaa ata 1104
Ile Ile Ala Ile Pro Ser Thr Val Gly Asn Gly Lys Ile Ser Lys Ile
        355                 360                 365

gtt cct cta tta gat aaa gga gca gca gta act acc aca aga aat gaa 1152
Val Pro Leu Leu Asp Lys Gly Ala Ala Val Thr Thr Thr Arg Asn Glu
    370                 375                 380

gtg gat tat gta gtt act gaa tac gga att gca aga ctt aaa ggt aaa 1200
Val Asp Tyr Val Val Thr Glu Tyr Gly Ile Ala Arg Leu Lys Gly Lys
385                 390                 395                 400

aat tta agg gac agg gcc cga gaa ctt att aaa ata gct cat cct gat 1248
Asn Leu Arg Asp Arg Ala Arg Glu Leu Ile Lys Ile Ala His Pro Asp
                405                 410                 415

ttt aga gag gaa tta aaa tta gaa tat aag aat aga ttt aaa gaa gag 1296
Phe Arg Glu Glu Leu Lys Leu Glu Tyr Lys Asn Arg Phe Lys Glu Glu
            420                 425                 430

ttt aaa aga ata gga taa 1314
Phe Lys Arg Ile Gly
        435

<210> 60
<211> 437
<212> PRT
<213> Clostridium tetani

<400> 60
Met Asn Trp Glu Asp Ile Tyr Lys Ser Lys Leu Leu Ser Ser Lys Glu
1               5                   10                  15
Ala Val Leu Lys Leu Lys Ser Glu Asn His Val Ala Val Gly His Ala
            20                  25                  30
Ala Ser Glu Pro Arg Leu Leu Met Lys Ala Leu Val Asp Asn Lys Glu
        35                  40                  45
Ser Phe Arg Asp Leu Lys Leu Tyr Gln Met Ile Ala Leu Glu Lys Ser
    50                  55                  60
Glu Cys Ala Lys Lys Gly Met Glu Lys His Phe Ser Tyr Asn Ser Leu
65                  70                  75                  80

Phe Val Gly Gly Gly Ser Arg Glu Ala Ile Asn Asp Gly Arg Ala Asp
                85                  90                  95
Tyr Ile Pro Cys Phe Phe Ser Arg Ile Pro Arg Leu Phe Arg Glu Asn
            100                 105                 110
Tyr Ile Lys Leu Asp Ala Ala Leu Ile Gln Val Ala Pro Pro Asp Glu
        115                 120                 125
His Gly Phe Cys Ser Phe Gly Val Ser Ser Asp Tyr Ile Lys Pro Ala
    130                 135                 140
Ala Glu Cys Ala Lys Leu Val Ile Ala Glu Val Asn Asp Lys Met Pro
145                 150                 155                 160
Arg Thr Leu Gly Asp Asn Phe Ile His Val Ser Asp Ile Asp Tyr Ile
                165                 170                 175
Val Glu Asn Ser Arg Glu Ile Pro Glu Leu Lys Pro Ser Asn Ile Gly
            180                 185                 190
Asp Ile Glu Arg Ala Ile Gly Glu Asn Cys Ala Ser Leu Ile Asn Asn
        195                 200                 205
Gly Asp Val Leu Gln Leu Gly Ile Gly Ala Ile Pro Asp Ala Val Leu
    210                 215                 220
Leu Phe Leu Lys Asp Lys Lys Asp Leu Gly Ile His Ser Glu Met Ile
225                 230                 235                 240
Ser Asp Gly Val Val Asp Leu Val Lys Ala Gly Val Ile Asn Asn Lys
                245                 250                 255
Lys Lys Thr Leu His Gln Gly Lys Ile Ile Val Ser Phe Leu Met Gly
            260                 265                 270
Thr Lys Lys Leu Tyr Asp Phe Val Asp Asn Asn Pro Met Val Arg Met
        275                 280                 285
Tyr Pro Val Asp Tyr Val Asn Asn Pro Val Val Ile Ser Lys Asn Asp
    290                 295                 300
Asn Met Val Ser Ile Asn Ser Cys Ile Gln Ile Asp Leu Met Gly Gln
305                 310                 315                 320
Ile Asp Ala Glu Thr Ile Gly Phe Thr Gln Ile Ser Gly Val Gly Gly
                325                 330                 335
Gln Ile Asp Phe Ile Arg Gly Ala Gly Leu Ser Lys Asn Gly Arg Ser
            340                 345                 350
Ile Ile Ala Ile Pro Ser Thr Val Gly Asn Gly Lys Ile Ser Lys Ile
        355                 360                 365
Val Pro Leu Leu Asp Lys Gly Ala Ala Val Thr Thr Thr Arg Asn Glu
    370                 375                 380
Val Asp Tyr Val Val Thr Glu Tyr Gly Ile Ala Arg Leu Lys Gly Lys
385                 390                 395                 400
Asn Leu Arg Asp Arg Ala Arg Glu Leu Ile Lys Ile Ala His Pro Asp
                405                 410                 415
Phe Arg Glu Glu Leu Lys Leu Glu Tyr Lys Asn Arg Phe Lys Glu Glu
            420                 425                 430
Phe Lys Arg Ile Gly
        435

<210> 61
<211> 1500
<212> DNA
<213> Bacteroides thetaiotaomicron

<220>
<221> CDS
<222> (1)..(1500)

<400> 61
atg tca ttc aat cgt att tcg gca gca gaa gct gca agc ctg atc aaa       48
Met Ser Phe Asn Arg Ile Ser Ala Ala Glu Ala Ala Ser Leu Ile Lys
1               5                   10                  15
cat ggc tac aac atc ggt ctg agc gga ttt act ccc gcc gga act gca       96
His Gly Tyr Asn Ile Gly Leu Ser Gly Phe Thr Pro Ala Gly Thr Ala
            20                  25                  30
aag gct gtt aca gct gaa ctt gca aaa att gca gaa gcg gaa cat gcc      144
Lys Ala Val Thr Ala Glu Leu Ala Lys Ile Ala Glu Ala Glu His Ala
        35                  40                  45
aaa ggt aat cca ttt caa gta gga att ttt aca ggt gct tct acc gga      192
Lys Gly Asn Pro Phe Gln Val Gly Ile Phe Thr Gly Ala Ser Thr Gly
    50                  55                  60
gaa tct tgt gac ggc gtt atg tct cgc gca aag gcc atc cgc tac cgc      240
Glu Ser Cys Asp Gly Val Met Ser Arg Ala Lys Ala Ile Arg Tyr Arg
65                  70                  75                  80

gcc cct tat acc acc aac tcc gac ttc cgc aaa gcc gtg aat aac ggt 288
Ala Pro Tyr Thr Thr Asn Ser Asp Phe Arg Lys Ala Val Asn Asn Gly
                85                  90                  95
gaa att gcc tac aac gac atc cat ctg tcg caa atg gca cag gaa gtg 336
Glu Ile Ala Tyr Asn Asp Ile His Leu Ser Gln Met Ala Gln Glu Val
            100                 105                 110
cgc tat ggc ttc atg ggc aaa gtg aac gta gcc atc atc gaa gcc tgc 384
Arg Tyr Gly Phe Met Gly Lys Val Asn Val Ala Ile Ile Glu Ala Cys
        115                 120                 125
gaa gtg act ccg gac gga aag att tat ctg act gct gcc ggt ggt att 432
Glu Val Thr Pro Asp Gly Lys Ile Tyr Leu Thr Ala Ala Gly Gly Ile
    130                 135                 140
tct ccg act atc tgc cgc ctg gcg gac cag atc atc gtt gag ttg aac 480
Ser Pro Thr Ile Cys Arg Leu Ala Asp Gln Ile Ile Val Glu Leu Asn
145                 150                 155                 160
gcc gca cac agc aaa tcc tgc atg ggt ctg cac gac gtg tac gaa ccg 528
Ala Ala His Ser Lys Ser Cys Met Gly Leu His Asp Val Tyr Glu Pro
                165                 170                 175
ctc gac ccg cct tac cgc cgc gaa atc ccc atc tac aag ccg agt gac 576
Leu Asp Pro Pro Tyr Arg Arg Glu Ile Pro Ile Tyr Lys Pro Ser Asp
            180                 185                 190
cgc atc gga ttg cct tat att cag gta gac ccg aaa aag atc gta ggc 624
Arg Ile Gly Leu Pro Tyr Ile Gln Val Asp Pro Lys Lys Ile Val Gly
        195                 200                 205
gta gta gaa acc aac tgg ccg gac gaa gca cgc tca ttt gcc gat gcc 672
Val Val Glu Thr Asn Trp Pro Asp Glu Ala Arg Ser Phe Ala Asp Ala
    210                 215                 220
gat ccg ttg act gac aaa atc ggt cag aac gtt gcc gac ttc ctc gct 720
Asp Pro Leu Thr Asp Lys Ile Gly Gln Asn Val Ala Asp Phe Leu Ala
225                 230                 235                 240
gcc gac atg aag cgt ggc atc att ccg tct act ttc ctt ccg ctg cag 768
Ala Asp Met Lys Arg Gly Ile Ile Pro Ser Thr Phe Leu Pro Leu Gln
                245                 250                 255
tcg ggc gta gga aat atc gct aac gct gtg ctc ggt gca ctg gga cgt 816
Ser Gly Val Gly Asn Ile Ala Asn Ala Val Leu Gly Ala Leu Gly Arg
            260                 265                 270
gac aag acg att ccc gca ttc gaa atg tac aca gag gtt atc cag aac 864
Asp Lys Thr Ile Pro Ala Phe Glu Met Tyr Thr Glu Val Ile Gln Asn
        275                 280                 285
tcc gtc atc gga ttg atc cgt gaa gga cgc atc aaa ttc ggt agc gcc 912
Ser Val Ile Gly Leu Ile Arg Glu Gly Arg Ile Lys Phe Gly Ser Ala
    290                 295                 300
tgc tca ctg act gtt acc aac gac tgt ctg gaa ggt att tac aac gat 960
Cys Ser Leu Thr Val Thr Asn Asp Cys Leu Glu Gly Ile Tyr Asn Asp
305                 310                 315                 320
atg gat ttc ttc cgc gac aag ctg gtt ctc cgc ccg tcg gaa atc tca 1008
Met Asp Phe Phe Arg Asp Lys Leu Val Leu Arg Pro Ser Glu Ile Ser
                325                 330                 335
aac aat ccg gaa gtg gtt cgc cga ctg ggt att atc tct atc aat aca 1056
Asn Asn Pro Glu Val Val Arg Arg Leu Gly Ile Ile Ser Ile Asn Thr
            340                 345                 350
gct atc gaa gtg gac ttg tac ggt aac gta aac tct act cat atc ggt 1104
Ala Ile Glu Val Asp Leu Tyr Gly Asn Val Asn Ser Thr His Ile Gly
        355                 360                 365
gga acg aag atg atg aac ggt atc ggt ggc tcg gga gac ttc acc cgc 1152
Gly Thr Lys Met Met Asn Gly Ile Gly Gly Ser Gly Asp Phe Thr Arg
    370                 375                 380
aat gct tat atc tct atc ttc act tgt ccg tct gtt gcc aaa gaa ggt 1200
Asn Ala Tyr Ile Ser Ile Phe Thr Cys Pro Ser Val Ala Lys Glu Gly
385                 390                 395                 400
aag atc agt gct atc gtc ccg atg gtt tct cac ctc gac cac agc gaa 1248
Lys Ile Ser Ala Ile Val Pro Met Val Ser His Leu Asp His Ser Glu
                405                 410                 415
cac tcc gtg aat atc att atc acc gag caa ggt gta gcc gac ctg cgt 1296
His Ser Val Asn Ile Ile Ile Thr Glu Gln Gly Val Ala Asp Leu Arg
            420                 425                 430
ggc aag agc ccg aag gaa cgt gca caa gcc atc atc gaa aac tgt gca 1344
Gly Lys Ser Pro Lys Glu Arg Ala Gln Ala Ile Ile Glu Asn Cys Ala
        435                 440                 445
cat ccc gac tat aaa cag ctt ctc tgg gat tat ctc aaa ctg gcc ggt 1392
His Pro Asp Tyr Lys Gln Leu Leu Trp Asp Tyr Leu Lys Leu Ala Gly
    450                 455                 460

ggc agg gcg cag act cca cac gct atc cag gcc gca ctc ggt atg cac 1440
Gly Arg Ala Gln Thr Pro His Ala Ile Gln Ala Ala Leu Gly Met His
465                 470                 475                 480
gcc gaa ctg gcc aag agc ggg gat atg aag aat acg aat tgg gcg gag 1488
Ala Glu Leu Ala Lys Ser Gly Asp Met Lys Asn Thr Asn Trp Ala Glu
                485                 490                 495
tat gcc aga taa 1500
Tyr Ala Arg

<210> 62
<211> 499
<212> PRT
<213> Bacteroides thetaiotaomicron

<400> 62
Met Ser Phe Asn Arg Ile Ser Ala Ala Glu Ala Ala Ser Leu Ile Lys
1               5                   10                  15
His Gly Tyr Asn Ile Gly Leu Ser Gly Phe Thr Pro Ala Gly Thr Ala
            20                  25                  30
Lys Ala Val Thr Ala Glu Leu Ala Lys Ile Ala Glu Ala Glu His Ala
        35                  40                  45
Lys Gly Asn Pro Phe Gln Val Gly Ile Phe Thr Gly Ala Ser Thr Gly
    50                  55                  60
Glu Ser Cys Asp Gly Val Met Ser Arg Ala Lys Ala Ile Arg Tyr Arg
65                  70                  75                  80
Ala Pro Tyr Thr Thr Asn Ser Asp Phe Arg Lys Ala Val Asn Asn Gly
                85                  90                  95
Glu Ile Ala Tyr Asn Asp Ile His Leu Ser Gln Met Ala Gln Glu Val
            100                 105                 110
Arg Tyr Gly Phe Met Gly Lys Val Asn Val Ala Ile Ile Glu Ala Cys
        115                 120                 125
Glu Val Thr Pro Asp Gly Lys Ile Tyr Leu Thr Ala Ala Gly Gly Ile
    130                 135                 140
Ser Pro Thr Ile Cys Arg Leu Ala Asp Gln Ile Ile Val Glu Leu Asn
145                 150                 155                 160
Ala Ala His Ser Lys Ser Cys Met Gly Leu His Asp Val Tyr Glu Pro
                165                 170                 175
Leu Asp Pro Pro Tyr Arg Arg Glu Ile Pro Ile Tyr Lys Pro Ser Asp
            180                 185                 190
Arg Ile Gly Leu Pro Tyr Ile Gln Val Asp Pro Lys Lys Ile Val Gly
        195                 200                 205
Val Val Glu Thr Asn Trp Pro Asp Glu Ala Arg Ser Phe Ala Asp Ala
    210                 215                 220
Asp Pro Leu Thr Asp Lys Ile Gly Gln Asn Val Ala Asp Phe Leu Ala
225                 230                 235                 240
Ala Asp Met Lys Arg Gly Ile Ile Pro Ser Thr Phe Leu Pro Leu Gln
                245                 250                 255
Ser Gly Val Gly Asn Ile Ala Asn Ala Val Leu Gly Ala Leu Gly Arg
            260                 265                 270
Asp Lys Thr Ile Pro Ala Phe Glu Met Tyr Thr Glu Val Ile Gln Asn
        275                 280                 285
Ser Val Ile Gly Leu Ile Arg Glu Gly Arg Ile Lys Phe Gly Ser Ala
    290                 295                 300
Cys Ser Leu Thr Val Thr Asn Asp Cys Leu Glu Gly Ile Tyr Asn Asp
305                 310                 315                 320
Met Asp Phe Phe Arg Asp Lys Leu Val Leu Arg Pro Ser Glu Ile Ser
                325                 330                 335
Asn Asn Pro Glu Val Val Arg Arg Leu Gly Ile Ile Ser Ile Asn Thr
            340                 345                 350
Ala Ile Glu Val Asp Leu Tyr Gly Asn Val Asn Ser Thr His Ile Gly
        355                 360                 365
Gly Thr Lys Met Met Asn Gly Ile Gly Gly Ser Gly Asp Phe Thr Arg
    370                 375                 380
Asn Ala Tyr Ile Ser Ile Phe Thr Cys Pro Ser Val Ala Lys Glu Gly
385                 390                 395                 400
Lys Ile Ser Ala Ile Val Pro Met Val Ser His Leu Asp His Ser Glu
                405                 410                 415
His Ser Val Asn Ile Ile Ile Thr Glu Gln Gly Val Ala Asp Leu Arg
            420                 425                 430
Gly Lys Ser Pro Lys Glu Arg Ala Gln Ala Ile Ile Glu Asn Cys Ala

435                 440                 445
His Pro Asp Tyr Lys Gln Leu Leu Trp Asp Tyr Leu Lys Leu Ala Gly
    450                 455                 460
Gly Arg Ala Gln Thr Pro His Ala Ile Gln Ala Ala Leu Gly Met His
465                 470                 475                 480
Ala Glu Leu Ala Lys Ser Gly Asp Met Lys Asn Thr Asn Trp Ala Glu
                485                 490                 495
Tyr Ala Arg

<210> 63
<211> 1287
<212> DNA
<213> Shewanella oneidensis

<220>
<221> CDS
<222> (1)..(1287)

<400> 63
atg cct gcg att gtg tgt caa agt gcc ctc gaa gcg gtt tcg tta atc      48
Met Pro Ala Ile Val Cys Gln Ser Ala Leu Glu Ala Val Ser Leu Ile
1               5                   10                  15
cga agt ggt gaa aca ctc tgg acc cat tcc atg ggg gca acc cca aaa      96
Arg Ser Gly Glu Thr Leu Trp Thr His Ser Met Gly Ala Thr Pro Lys
            20                  25                  30
gtg tta ttg gat gcg ctg gca aaa cat gcg ctc act ctc gat aat att      144
Val Leu Leu Asp Ala Leu Ala Lys His Ala Leu Thr Leu Asp Asn Ile
        35                  40                  45
acg tta tta cag tta cat act gaa ggt gct gag tcc tta agt cat cca      192
Thr Leu Leu Gln Leu His Thr Glu Gly Ala Glu Ser Leu Ser His Pro
    50                  55                  60
agt tta ttg gga cat tta cgc cac cgt tgt ttt ttc ggt ggt gtg cct      240
Ser Leu Leu Gly His Leu Arg His Arg Cys Phe Phe Gly Gly Val Pro
65                  70                  75                  80
aca agg ccg tta ttg caa agt ggt gat gcc gat tat gtg ccg att ttc      288
Thr Arg Pro Leu Leu Gln Ser Gly Asp Ala Asp Tyr Val Pro Ile Phe
                85                  90                  95
ctc tcg gaa gtg ccc aaa ctg ttt cgt agc ggc gag caa aaa att gat      336
Leu Ser Glu Val Pro Lys Leu Phe Arg Ser Gly Glu Gln Lys Ile Asp
            100                 105                 110
acc gcg att att caa gta tcg cca cct gat aaa cac ggc atg tgt tcc      384
Thr Ala Ile Ile Gln Val Ser Pro Pro Asp Lys His Gly Met Cys Ser
        115                 120                 125
tta ggc att tcg gtt gag gcg acc tta gcc gct tgc caa gtg gcg ggt      432
Leu Gly Ile Ser Val Glu Ala Thr Leu Ala Ala Cys Gln Val Ala Gly
    130                 135                 140
aag att att gcc cat atc aat ccg caa atg ccg cgc acc cat ggc gat      480
Lys Ile Ile Ala His Ile Asn Pro Gln Met Pro Arg Thr His Gly Asp
145                 150                 155                 160
ggc ttt atc cat atc gat cgt ttt gcc gca gtg tat gag caa agc gcc      528
Gly Phe Ile His Ile Asp Arg Phe Ala Ala Val Tyr Glu Gln Ser Ala
                165                 170                 175
agt ttg cct atc cat tcc ttt gcc aca ggc gat gcc gtg agt ttg gcc      576
Ser Leu Pro Ile His Ser Phe Ala Thr Gly Asp Ala Val Ser Leu Ala
            180                 185                 190
ata ggt cag cat gtg gcc gaa tta gtc cgc gat gga gac tgt ttg caa      624
Ile Gly Gln His Val Ala Glu Leu Val Arg Asp Gly Asp Cys Leu Gln
        195                 200                 205
atg ggc att ggc gcc att ccc gat gcg gtg ttg tcc tgt ttg act ggc      672
Met Gly Ile Gly Ala Ile Pro Asp Ala Val Leu Ser Cys Leu Thr Gly
    210                 215                 220
cac aag gac tta ggt gtt cac act gag tta ttt tcc gac ggg att ttg      720
His Lys Asp Leu Gly Val His Thr Glu Leu Phe Ser Asp Gly Ile Leu
225                 230                 235                 240
caa ttg gtt gaa aag ggc gtg att aac aac act aaa aaa cgc ttt tat      768
Gln Leu Val Glu Lys Gly Val Ile Asn Asn Thr Lys Lys Arg Phe Tyr
                245                 250                 255
ccc gga aaa ctg gtt acc ggt ttc gcc cta ggt agc cag aag tta tac      816
Pro Gly Lys Leu Val Thr Gly Phe Ala Leu Gly Ser Gln Lys Leu Tyr
            260                 265                 270
gac tat gtg gat gat aat cca gcg gtt atc ttt atg gat atc gag caa      864

Asp Tyr Val Asp Asp Asn Pro Ala Val Ile Phe Met Asp Ile Glu Gln
        275                 280                 285
gtc aac gac acg tct att atc cgt aaa aac ccc aat gta atg gcg att        912
Val Asn Asp Thr Ser Ile Ile Arg Lys Asn Pro Asn Val Met Ala Ile
    290                 295                 300
aac tcg gcg ttg cag gtc gat ctc acc ggg caa gtg tgt gcc gat tcc        960
Asn Ser Ala Leu Gln Val Asp Leu Thr Gly Gln Val Cys Ala Asp Ser
305                 310                 315                 320
att ggc act aag att tat tcg ggc gtg ggt ggc cag atg gac ttt atc        1008
Ile Gly Thr Lys Ile Tyr Ser Gly Val Gly Gly Gln Met Asp Phe Ile
                325                 330                 335
cgt ggc gcg ggc tta tct gaa ggc ggc cgc tct gtg att gcg ctg cca        1056
Arg Gly Ala Gly Leu Ser Glu Gly Gly Arg Ser Val Ile Ala Leu Pro
            340                 345                 350
agt act gcg gcg ggg ggg cgt att tct cgt atc gct agc gtg tta tct        1104
Ser Thr Ala Ala Gly Gly Arg Ile Ser Arg Ile Ala Ser Val Leu Ser
        355                 360                 365
cct ggg gct ggc gtt gta act aca cgt gcc cat gtg cat tac ata gtg        1152
Pro Gly Ala Gly Val Val Thr Thr Arg Ala His Val His Tyr Ile Val
    370                 375                 380
act gaa tat ggc gca gct aat ctg aag ggg cga tcc cta cgt gaa agg        1200
Thr Glu Tyr Gly Ala Ala Asn Leu Lys Gly Arg Ser Leu Arg Glu Arg
385                 390                 395                 400
gcg cag gcc ttg att aat atc gcc cat ccg gat ttt aga gag caa ctg        1248
Ala Gln Ala Leu Ile Asn Ile Ala His Pro Asp Phe Arg Glu Gln Leu
                405                 410                 415
agt cgc gac gcc ttt gaa gta tgg gga tta aat tta tag                    1287
Ser Arg Asp Ala Phe Glu Val Trp Gly Leu Asn Leu
            420                 425

<210> 64
<211> 428
<212> PRT
<213> Shewanella oneidensis

<400> 64
Met Pro Ala Ile Val Cys Gln Ser Ala Leu Glu Ala Val Ser Leu Ile
1               5                   10                  15
Arg Ser Gly Glu Thr Leu Trp Thr His Ser Met Gly Ala Thr Pro Lys
            20                  25                  30
Val Leu Leu Asp Ala Leu Ala Lys His Ala Leu Thr Leu Asp Asn Ile
        35                  40                  45
Thr Leu Leu Gln Leu His Thr Glu Gly Ala Glu Ser Leu Ser His Pro
    50                  55                  60
Ser Leu Leu Gly His Leu Arg His Arg Cys Phe Phe Gly Gly Val Pro
65                  70                  75                  80
Thr Arg Pro Leu Leu Gln Ser Gly Asp Ala Asp Tyr Val Pro Ile Phe
                85                  90                  95
Leu Ser Glu Val Pro Lys Leu Phe Arg Ser Gly Glu Gln Lys Ile Asp
            100                 105                 110
Thr Ala Ile Ile Gln Val Ser Pro Pro Asp Lys His Gly Met Cys Ser
        115                 120                 125
Leu Gly Ile Ser Val Glu Ala Thr Leu Ala Ala Cys Gln Val Ala Gly
    130                 135                 140
Lys Ile Ile Ala His Ile Asn Pro Gln Met Pro Arg Thr His Gly Asp
145                 150                 155                 160
Gly Phe Ile His Ile Asp Arg Phe Ala Ala Val Tyr Glu Gln Ser Ala
                165                 170                 175
Ser Leu Pro Ile His Ser Phe Ala Thr Gly Asp Ala Val Ser Leu Ala
            180                 185                 190
Ile Gly Gln His Val Ala Glu Leu Val Arg Asp Gly Asp Cys Leu Gln
        195                 200                 205
Met Gly Ile Gly Ala Ile Pro Asp Ala Val Leu Ser Cys Leu Thr Gly
    210                 215                 220
His Lys Asp Leu Gly Val His Thr Glu Leu Phe Ser Asp Gly Ile Leu
225                 230                 235                 240
Gln Leu Val Glu Lys Gly Val Ile Asn Asn Thr Lys Lys Arg Phe Tyr
                245                 250                 255
Pro Gly Lys Leu Val Thr Gly Phe Ala Leu Gly Ser Gln Lys Leu Tyr
            260                 265                 270
Asp Tyr Val Asp Asp Asn Pro Ala Val Ile Phe Met Asp Ile Glu Gln

275                 280                 285
Val Asn Asp Thr Ser Ile Ile Arg Lys Asn Pro Asn Val Met Ala Ile
    290                 295                 300
Asn Ser Ala Leu Gln Val Asp Leu Thr Gly Gln Val Cys Ala Asp Ser
305                 310                 315                 320
Ile Gly Thr Lys Ile Tyr Ser Gly Val Gly Gly Gln Met Asp Phe Ile
                325                 330                 335
Arg Gly Ala Gly Leu Ser Glu Gly Gly Arg Ser Val Ile Ala Leu Pro
            340                 345                 350
Ser Thr Ala Ala Gly Gly Arg Ile Ser Arg Ile Ala Ser Val Leu Ser
        355                 360                 365
Pro Gly Ala Gly Val Val Thr Thr Arg Ala His Val His Tyr Ile Val
    370                 375                 380
Thr Glu Tyr Gly Ala Ala Asn Leu Lys Gly Arg Ser Leu Arg Glu Arg
385                 390                 395                 400
Ala Gln Ala Leu Ile Asn Ile Ala His Pro Asp Phe Arg Glu Gln Leu
                405                 410                 415
Ser Arg Asp Ala Phe Glu Val Trp Gly Leu Asn Leu
            420                 425

<210> 65
<211> 1515
<212> DNA
<213> Oceanobacillus iheyensis

<220>
<221> CDS
<222> (1)..(1515)

<400> 65
atg gaa gaa aaa tta aag cgc ata caa aac aag gaa tta ggg caa aaa       48
Met Glu Glu Lys Leu Lys Arg Ile Gln Asn Lys Glu Leu Gly Gln Lys
1               5                   10                  15
gtg gta act gca gca gaa gca gct tct tgg att aaa gat ggt atg aca       96
Val Val Thr Ala Ala Glu Ala Ala Ser Trp Ile Lys Asp Gly Met Thr
            20                  25                  30
ttg ggg ctt agt gga ttt aca cgt gcg gga gat gca aag gcg gtt ccg      144
Leu Gly Leu Ser Gly Phe Thr Arg Ala Gly Asp Ala Lys Ala Val Pro
        35                  40                  45
act gcg ctg gtg aaa aga gcg gaa gcg gga gaa tcc ctc aaa gtc aat      192
Thr Ala Leu Val Lys Arg Ala Glu Ala Gly Glu Ser Leu Lys Val Asn
    50                  55                  60
gta tac aca ggg gca tct tta gga tct aat atc gat aag ctg ttt gca      240
Val Tyr Thr Gly Ala Ser Leu Gly Ser Asn Ile Asp Lys Leu Phe Ala
65                  70                  75                  80
gaa gca gga atc gtt aat aaa aga att cca ttt cag gct gag cca tct      288
Glu Ala Gly Ile Val Asn Lys Arg Ile Pro Phe Gln Ala Glu Pro Ser
                85                  90                  95
atg cgt aag gca ata aat gct ggg gaa cat tta ttt gtc gac caa cac      336
Met Arg Lys Ala Ile Asn Ala Gly Glu His Leu Phe Val Asp Gln His
            100                 105                 110
ctt tca cac aca tca gaa atg ctt cgt ggt gat gcg atg gag gct gtc      384
Leu Ser His Thr Ser Glu Met Leu Arg Gly Asp Ala Met Glu Ala Val
        115                 120                 125
gat tat gcc ata ata gaa gca att tct gta aca gaa aat ggt tat att      432
Asp Tyr Ala Ile Ile Glu Ala Ile Ser Val Thr Glu Asn Gly Tyr Ile
    130                 135                 140
att cca agt aca tca gta ggt aac tca gtt gca ttt gct gaa act gcc      480
Ile Pro Ser Thr Ser Val Gly Asn Ser Val Ala Phe Ala Glu Thr Ala
145                 150                 155                 160
gat aac att att att gaa ctc aat act gcg caa cca atg gaa tta gaa      528
Asp Asn Ile Ile Ile Glu Leu Asn Thr Ala Gln Pro Met Glu Leu Glu
                165                 170                 175
gga atg cat gat att tat ggt cct ggg aaa caa gga gat cgt gat ccg      576
Gly Met His Asp Ile Tyr Gly Pro Gly Lys Gln Gly Asp Arg Asp Pro
            180                 185                 190
att cca tta aaa gag gtc gat cag cgt att ggt aag gtc ggt att cag      624
Ile Pro Leu Lys Glu Val Asp Gln Arg Ile Gly Lys Val Gly Ile Gln
        195                 200                 205
gtt gat acg gat aaa att aag ggg ata gtt ttt acg cat caa cct gat      672
Val Asp Thr Asp Lys Ile Lys Gly Ile Val Phe Thr His Gln Pro Asp
    210                 215                 220

tca gct tcg aca att gtt ccg cca gat gag gag acg caa caa att gcg 720
Ser Ala Ser Thr Ile Val Pro Pro Asp Glu Glu Thr Gln Gln Ile Ala
225                 230                 235                 240
aac cat cta ctg aat ttc ttt agg gag gag att cag gca gga cga tta 768
Asn His Leu Leu Asn Phe Phe Arg Glu Glu Ile Gln Ala Gly Arg Leu
                245                 250                 255
aca gag aag ctt atg cca ttg cag tcc gga ata ggt tct gtt gct aat 816
Thr Glu Lys Leu Met Pro Leu Gln Ser Gly Ile Gly Ser Val Ala Asn
            260                 265                 270
gcc gta tta cat ggt atg cta gac tct gag ttt aaa gat ttg acg gtt 864
Ala Val Leu His Gly Met Leu Asp Ser Glu Phe Lys Asp Leu Thr Val
        275                 280                 285
tat tct gaa gtg ctt caa gat gct gtc ttc gat ttg att gat gca ggt 912
Tyr Ser Glu Val Leu Gln Asp Ala Val Phe Asp Leu Ile Asp Ala Gly
    290                 295                 300
aaa gtg agc ttt gct tct gca tgt tcc att acg tta tca gaa gac aag 960
Lys Val Ser Phe Ala Ser Ala Cys Ser Ile Thr Leu Ser Glu Asp Lys
305                 310                 315                 320
atg aag cat gtg ttt agt aat cta gcg gaa tat cag agt aag ctt gtc 1008
Met Lys His Val Phe Ser Asn Leu Ala Glu Tyr Gln Ser Lys Leu Val
                325                 330                 335
tta cgt ccg caa gaa atc tcg aat cat cca gaa atc att cgt cga ctg 1056
Leu Arg Pro Gln Glu Ile Ser Asn His Pro Glu Ile Ile Arg Arg Leu
            340                 345                 350
ggt cta att gcg att aac aca gca tta gaa ttt gat att tac ggc aat 1104
Gly Leu Ile Ala Ile Asn Thr Ala Leu Glu Phe Asp Ile Tyr Gly Asn
        355                 360                 365
gtg aac tct aca cac gtt ggc gga acg aaa atg atg aac ggt att ggt 1152
Val Asn Ser Thr His Val Gly Gly Thr Lys Met Met Asn Gly Ile Gly
    370                 375                 380
ggt tca ggc gat ttt gct aga aat gct aga ttg ggg att ttt gtt aca 1200
Gly Ser Gly Asp Phe Ala Arg Asn Ala Arg Leu Gly Ile Phe Val Thr
385                 390                 395                 400
aag tcg att gcg aaa aat ggc gat ata tct agt atc gtt cca tat gta 1248
Lys Ser Ile Ala Lys Asn Gly Asp Ile Ser Ser Ile Val Pro Tyr Val
                405                 410                 415
tcg cat aca gat cat acc gaa cac gat gta gat gta att gtt acg gaa 1296
Ser His Thr Asp His Thr Glu His Asp Val Asp Val Ile Val Thr Glu
            420                 425                 430
caa ggt tat gca gac ctt cgt gga tta gca cca cga gaa cga gtg ccg 1344
Gln Gly Tyr Ala Asp Leu Arg Gly Leu Ala Pro Arg Glu Arg Val Pro
        435                 440                 445
caa att att gaa aat tgt gtg cat cct tta tat aag gaa gcg tta tgg 1392
Gln Ile Ile Glu Asn Cys Val His Pro Leu Tyr Lys Glu Ala Leu Trp
    450                 455                 460
gag tat tac caa gaa gcg cta gaa cgg ggt ggg cag act ccg cat gtt 1440
Glu Tyr Tyr Gln Glu Ala Leu Glu Arg Gly Gly Gln Thr Pro His Val
465                 470                 475                 480
cta gaa aaa gct ttt tct tgg cat gct aac ttc cga gag cat gga aca 1488
Leu Glu Lys Ala Phe Ser Trp His Ala Asn Phe Arg Glu His Gly Thr
                485                 490                 495
atg ctg ccg gca gtg cca caa gtg taa 1515
Met Leu Pro Ala Val Pro Gln Val
            500

<210> 66
<211> 504
<212> PRT
<213> Oceanobacillus iheyensis

<400> 66
Met Glu Glu Lys Leu Lys Arg Ile Gln Asn Lys Glu Leu Gly Gln Lys
1               5                   10                  15
Val Val Thr Ala Ala Glu Ala Ala Ser Trp Ile Lys Asp Gly Met Thr
            20                  25                  30
Leu Gly Leu Ser Gly Phe Thr Arg Ala Gly Asp Ala Lys Ala Val Pro
        35                  40                  45
Thr Ala Leu Val Lys Arg Ala Glu Ala Gly Glu Ser Leu Lys Val Asn
    50                  55                  60
Val Tyr Thr Gly Ala Ser Leu Gly Ser Asn Ile Asp Lys Leu Phe Ala
65                  70                  75                  80

Glu Ala Gly Ile Val Asn Lys Arg Ile Pro Phe Gln Ala Glu Pro Ser
                85                  90                  95
Met Arg Lys Ala Ile Asn Ala Gly Glu His Leu Phe Val Asp Gln His
            100                 105                 110
Leu Ser His Thr Ser Glu Met Leu Arg Gly Asp Ala Met Glu Ala Val
        115                 120                 125
Asp Tyr Ala Ile Ile Glu Ala Ile Ser Val Thr Glu Asn Gly Tyr Ile
    130                 135                 140
Ile Pro Ser Thr Ser Val Gly Asn Ser Val Ala Phe Ala Glu Thr Ala
145                 150                 155                 160
Asp Asn Ile Ile Ile Glu Leu Asn Thr Ala Gln Pro Met Glu Leu Glu
                165                 170                 175
Gly Met His Asp Ile Tyr Gly Pro Gly Lys Gln Gly Asp Arg Asp Pro
            180                 185                 190
Ile Pro Leu Lys Glu Val Asp Gln Arg Ile Gly Lys Val Gly Ile Gln
        195                 200                 205
Val Asp Thr Asp Lys Ile Lys Gly Ile Val Phe Thr His Gln Pro Asp
    210                 215                 220
Ser Ala Ser Thr Ile Val Pro Pro Asp Glu Glu Thr Gln Gln Ile Ala
225                 230                 235                 240
Asn His Leu Leu Asn Phe Phe Arg Glu Glu Ile Gln Ala Gly Arg Leu
                245                 250                 255
Thr Glu Lys Leu Met Pro Leu Gln Ser Gly Ile Gly Ser Val Ala Asn
            260                 265                 270
Ala Val Leu His Gly Met Leu Asp Ser Glu Phe Lys Asp Leu Thr Val
        275                 280                 285
Tyr Ser Glu Val Leu Gln Asp Ala Val Phe Asp Leu Ile Asp Ala Gly
    290                 295                 300
Lys Val Ser Phe Ala Ser Ala Cys Ser Ile Thr Leu Ser Glu Asp Lys
305                 310                 315                 320
Met Lys His Val Phe Ser Asn Leu Ala Glu Tyr Gln Ser Lys Leu Val
                325                 330                 335
Leu Arg Pro Gln Glu Ile Ser Asn His Pro Glu Ile Ile Arg Arg Leu
            340                 345                 350
Gly Leu Ile Ala Ile Asn Thr Ala Leu Glu Phe Asp Ile Tyr Gly Asn
        355                 360                 365
Val Asn Ser Thr His Val Gly Gly Thr Lys Met Met Asn Gly Ile Gly
    370                 375                 380
Gly Ser Gly Asp Phe Ala Arg Asn Ala Arg Leu Gly Ile Phe Val Thr
385                 390                 395                 400
Lys Ser Ile Ala Lys Asn Gly Asp Ile Ser Ser Ile Val Pro Tyr Val
                405                 410                 415
Ser His Thr Asp His Thr Glu His Asp Val Asp Val Ile Val Thr Glu
            420                 425                 430
Gln Gly Tyr Ala Asp Leu Arg Gly Leu Ala Pro Arg Glu Arg Val Pro
        435                 440                 445
Gln Ile Ile Glu Asn Cys Val His Pro Leu Tyr Lys Glu Ala Leu Trp
    450                 455                 460
Glu Tyr Tyr Gln Glu Ala Leu Glu Arg Gly Gly Gln Thr Pro His Val
465                 470                 475                 480
Leu Glu Lys Ala Phe Ser Trp His Ala Asn Phe Arg Glu His Gly Thr
                485                 490                 495
Met Leu Pro Ala Val Pro Gln Val
            500

<210> 67
<211> 1524
<212> DNA
<213> Corynebacterium efficiens

<220>
<221> CDS
<222> (1)..(1524)

<400> 67
atg tca gat cgc att gct tcg gag aag ctg cgt tcc aag gtc atg tct 48
Met Ser Asp Arg Ile Ala Ser Glu Lys Leu Arg Ser Lys Val Met Ser
1               5                   10                  15
gcc gat gag gca gcc cag ttc gtc aac gcc ggt gac aag gtc ggt atg 96
Ala Asp Glu Ala Ala Gln Phe Val Asn Ala Gly Asp Lys Val Gly Met
            20                  25                  30
tcc ggc ttc acc ggt gcg ggc tac ccc aag gcc ctg ccc acc gcc atc 144

Ser Gly Phe Thr Gly Ala Gly Tyr Pro Lys Ala Leu Pro Thr Ala Ile
        35                  40                  45
gcc aac cgt gcg aag gaa gca cac gca gca ggc tcc gag tac gcg att         192
Ala Asn Arg Ala Lys Glu Ala His Ala Ala Gly Ser Glu Tyr Ala Ile
    50                  55                  60
gat ctg ttc acc ggc gca tcc acc gcc ccc gac tgc gat ggt gtc ctg         240
Asp Leu Phe Thr Gly Ala Ser Thr Ala Pro Asp Cys Asp Gly Val Leu
65                  70                  75                  80
gcc gag gcc gac gcc gtc aac ttc cgc atg ccc tac gca tcc gac ccg         288
Ala Glu Ala Asp Ala Val Asn Phe Arg Met Pro Tyr Ala Ser Asp Pro
                85                  90                  95
atc atg cgt aac aag atc aac gag ggc aag atg ttc tac tcg gac atc         336
Ile Met Arg Asn Lys Ile Asn Glu Gly Lys Met Phe Tyr Ser Asp Ile
            100                 105                 110
cac ctc tcc cac tcc ggc cag tac gtg gag aac ggc ttc ttc ggc aag         384
His Leu Ser His Ser Gly Gln Tyr Val Glu Asn Gly Phe Phe Gly Lys
        115                 120                 125
ctg aac gtc gcc atc gtc gag gcc acc cgc atc acc gag gag ggc cac         432
Leu Asn Val Ala Ile Val Glu Ala Thr Arg Ile Thr Glu Glu Gly His
    130                 135                 140
atc atc ccg tcc tcc tcc gtc ggc aac aac gtt gag tac ctc aac gca         480
Ile Ile Pro Ser Ser Ser Val Gly Asn Asn Val Glu Tyr Leu Asn Ala
145                 150                 155                 160
gcc gag aag atc atc atc gag gtc aac tcc tgg cag tcc gcc gat ctt         528
Ala Glu Lys Ile Ile Ile Glu Val Asn Ser Trp Gln Ser Ala Asp Leu
                165                 170                 175
gag ggc atg cac gac atc tgg tcc atg ccg aag ctg ccg aac cgc atc         576
Glu Gly Met His Asp Ile Trp Ser Met Pro Lys Leu Pro Asn Arg Ile
            180                 185                 190
gcc atc ccg atc aac aag gcc ggc gac cgc atc ggt gcc ccc tac atc         624
Ala Ile Pro Ile Asn Lys Ala Gly Asp Arg Ile Gly Ala Pro Tyr Ile
        195                 200                 205
gag atc gac acc gac aag gtc gtc gcc gtg gtg gag acc aac acc ccg         672
Glu Ile Asp Thr Asp Lys Val Val Ala Val Val Glu Thr Asn Thr Pro
    210                 215                 220
gac cgc aac gca ccg ttc aag ccc gtc gac gag gtc tcc cag aag atc         720
Asp Arg Asn Ala Pro Phe Lys Pro Val Asp Glu Val Ser Gln Lys Ile
225                 230                 235                 240
gcc ggc aac ttc ctc gac ttc ctc gag ggt gag gtc cgc gca ggc cgt         768
Ala Gly Asn Phe Leu Asp Phe Leu Glu Gly Glu Val Arg Ala Gly Arg
                245                 250                 255
ctg tcc tac gac ggt tat gtc atg cag tcc ggt gtg ggc aac gtc ccc         816
Leu Ser Tyr Asp Gly Tyr Val Met Gln Ser Gly Val Gly Asn Val Pro
            260                 265                 270
aac gcc gtg atg gct ggc ctg ctg gat tcc aag ttc gag aac atc cag         864
Asn Ala Val Met Ala Gly Leu Leu Asp Ser Lys Phe Glu Asn Ile Gln
        275                 280                 285
gcc tac acc gag gtc atc cag gac ggc atg gtc gac ctc atc gac gcc         912
Ala Tyr Thr Glu Val Ile Gln Asp Gly Met Val Asp Leu Ile Asp Ala
    290                 295                 300
ggc aag atg acc gtt gcc tcc gcc acc tcc ttc tcc ctg tcc cct gag         960
Gly Lys Met Thr Val Ala Ser Ala Thr Ser Phe Ser Leu Ser Pro Glu
305                 310                 315                 320
tac gcg gag aag atg aac aac gag gcc aag cgt tac cgc gag tcc atc         1008
Tyr Ala Glu Lys Met Asn Asn Glu Ala Lys Arg Tyr Arg Glu Ser Ile
                325                 330                 335
atc ctg cgc ccg cag cag atc tcc aac cac ccg gag gtc atc cgc cgt         1056
Ile Leu Arg Pro Gln Gln Ile Ser Asn His Pro Glu Val Ile Arg Arg
            340                 345                 350
gtc ggg ctg atc gcc acc aac ggt ctg atc gag gct gac atc tac ggc         1104
Val Gly Leu Ile Ala Thr Asn Gly Leu Ile Glu Ala Asp Ile Tyr Gly
        355                 360                 365
aac gtc aac tcc acc aac gtc tcc ggc tcc cgc gtg atg aac ggc atc         1152
Asn Val Asn Ser Thr Asn Val Ser Gly Ser Arg Val Met Asn Gly Ile
    370                 375                 380
ggt ggt tcc ggt gac ttc acc cgc aac gcc tac atc tcc tcc ttc atc         1200
Gly Gly Ser Gly Asp Phe Thr Arg Asn Ala Tyr Ile Ser Ser Phe Ile
385                 390                 395                 400
acc ccg tcc gag gcc aag ggt ggt gcg att tcc gcg atc gtt cca ttc         1248
Thr Pro Ser Glu Ala Lys Gly Gly Ala Ile Ser Ala Ile Val Pro Phe
                405                 410                 415
gcc tcc cac atc gac cac acc gag cat gac gtc atg gtt gtc atc tcc         1296

Ala Ser His Ile Asp His Thr Glu His Asp Val Met Val Val Ile Ser
            420                 425                 430
gag tac ggt tat gcc gac ctg cgc ggc ctg gca cca cgc cag cgt gtg      1344
Glu Tyr Gly Tyr Ala Asp Leu Arg Gly Leu Ala Pro Arg Gln Arg Val
        435                 440                 445
gag aag atg atc gcc ctg gct cac ccg gat tac cgt ccg ctg ctg gag      1392
Glu Lys Met Ile Ala Leu Ala His Pro Asp Tyr Arg Pro Leu Leu Glu
    450                 455                 460
gag tac tac gcc cgc gcc acc tcc ggc gac aac aag ttc atg cag acc      1440
Glu Tyr Tyr Ala Arg Ala Thr Ser Gly Asp Asn Lys Phe Met Gln Thr
465                 470                 475                 480
ccg cat gac ctg gcc acc gcg ttc gac ttc cac cgc aac ctg gcc aag      1488
Pro His Asp Leu Ala Thr Ala Phe Asp Phe His Arg Asn Leu Ala Lys
                485                 490                 495
aac ggt tcc atg aag aac cag gac cag atg gtc tag                      1524
Asn Gly Ser Met Lys Asn Gln Asp Gln Met Val
            500                 505


<210> 68
<211> 507
<212> PRT
<213> Corynebacterium efficiens

<400> 68
Met Ser Asp Arg Ile Ala Ser Glu Lys Leu Arg Ser Lys Val Met Ser
1               5                   10                  15
Ala Asp Glu Ala Ala Gln Phe Val Asn Ala Gly Asp Lys Val Gly Met
            20                  25                  30
Ser Gly Phe Thr Gly Ala Gly Tyr Pro Lys Ala Leu Pro Thr Ala Ile
        35                  40                  45
Ala Asn Arg Ala Lys Glu Ala His Ala Ala Gly Ser Glu Tyr Ala Ile
    50                  55                  60
Asp Leu Phe Thr Gly Ala Ser Thr Ala Pro Asp Cys Asp Gly Val Leu
65                  70                  75                  80
Ala Glu Ala Asp Ala Val Asn Phe Arg Met Pro Tyr Ala Ser Asp Pro
                85                  90                  95
Ile Met Arg Asn Lys Ile Asn Glu Gly Lys Met Phe Tyr Ser Asp Ile
            100                 105                 110
His Leu Ser His Ser Gly Gln Tyr Val Glu Asn Gly Phe Phe Gly Lys
        115                 120                 125
Leu Asn Val Ala Ile Val Glu Ala Thr Arg Ile Thr Glu Glu Gly His
    130                 135                 140
Ile Ile Pro Ser Ser Ser Val Gly Asn Asn Val Glu Tyr Leu Asn Ala
145                 150                 155                 160
Ala Glu Lys Ile Ile Ile Glu Val Asn Ser Trp Gln Ser Ala Asp Leu
                165                 170                 175
Glu Gly Met His Asp Ile Trp Ser Met Pro Lys Leu Pro Asn Arg Ile
            180                 185                 190
Ala Ile Pro Ile Asn Lys Ala Gly Asp Arg Ile Gly Ala Pro Tyr Ile
        195                 200                 205
Glu Ile Asp Thr Asp Lys Val Val Ala Val Val Glu Thr Asn Thr Pro
    210                 215                 220
Asp Arg Asn Ala Pro Phe Lys Pro Val Asp Glu Val Ser Gln Lys Ile
225                 230                 235                 240
Ala Gly Asn Phe Leu Asp Phe Leu Glu Gly Glu Val Arg Ala Gly Arg
                245                 250                 255
Leu Ser Tyr Asp Gly Tyr Val Met Gln Ser Gly Val Gly Asn Val Pro
            260                 265                 270
Asn Ala Val Met Ala Gly Leu Leu Asp Ser Lys Phe Glu Asn Ile Gln
        275                 280                 285
Ala Tyr Thr Glu Val Ile Gln Asp Gly Met Val Asp Leu Ile Asp Ala
    290                 295                 300
Gly Lys Met Thr Val Ala Ser Ala Thr Ser Phe Ser Leu Ser Pro Glu
305                 310                 315                 320
Tyr Ala Glu Lys Met Asn Asn Glu Ala Lys Arg Tyr Arg Glu Ser Ile
                325                 330                 335
Ile Leu Arg Pro Gln Gln Ile Ser Asn His Pro Glu Val Ile Arg Arg
            340                 345                 350
Val Gly Leu Ile Ala Thr Asn Gly Leu Ile Glu Ala Asp Ile Tyr Gly
        355                 360                 365
Asn Val Asn Ser Thr Asn Val Ser Gly Ser Arg Val Met Asn Gly Ile

370                 375                 380
Gly Gly Ser Gly Asp Phe Thr Arg Asn Ala Tyr Ile Ser Ser Phe Ile
385                 390                 395                 400
Thr Pro Ser Glu Ala Lys Gly Gly Ala Ile Ser Ala Ile Val Pro Phe
                405                 410                 415
Ala Ser His Ile Asp His Thr Glu His Asp Val Met Val Val Ile Ser
            420                 425                 430
Glu Tyr Gly Tyr Ala Asp Leu Arg Gly Leu Ala Pro Arg Gln Arg Val
        435                 440                 445
Glu Lys Met Ile Ala Leu Ala His Pro Asp Tyr Arg Pro Leu Leu Glu
    450                 455                 460
Glu Tyr Tyr Ala Arg Ala Thr Ser Gly Asp Asn Lys Phe Met Gln Thr
465                 470                 475                 480
Pro His Asp Leu Ala Thr Ala Phe Asp Phe His Arg Asn Leu Ala Lys
                485                 490                 495
Asn Gly Ser Met Lys Asn Gln Asp Gln Met Val
            500                 505

<210> 69
<211> 1509
<212> DNA
<213> Corynebacterium efficiens

<220>
<221> CDS
<222> (1)..(1509)

<400> 69
atg tct gag aga atc gca agc gca gaa ttc cga tcg aag gta atg tcc       48
Met Ser Glu Arg Ile Ala Ser Ala Glu Phe Arg Ser Lys Val Met Ser
1               5                   10                  15
gcc gag gaa gcc gcc cag ttc gtc aac aac ggc gac acc gtg ggc atg       96
Ala Glu Glu Ala Ala Gln Phe Val Asn Asn Gly Asp Thr Val Gly Met
            20                  25                  30
tcc gga ttc acc ggc gcg gcc tac ccg aag gcg ctg ccc act gcc atc      144
Ser Gly Phe Thr Gly Ala Ala Tyr Pro Lys Ala Leu Pro Thr Ala Ile
        35                  40                  45
gcc aac cgc gcc aag gag gca cac tcc aag ggt gat gag tac gcc atc      192
Ala Asn Arg Ala Lys Glu Ala His Ser Lys Gly Asp Glu Tyr Ala Ile
    50                  55                  60
aac ctc ctg acc ggt gcc tcc acc gca ccg gac tgt gat ggt gtc ctg      240
Asn Leu Leu Thr Gly Ala Ser Thr Ala Pro Asp Cys Asp Gly Val Leu
65                  70                  75                  80
gca gag gcg gat gcg atc aac tac cgc atg ccg tac cag tcc gat ccc      288
Ala Glu Ala Asp Ala Ile Asn Tyr Arg Met Pro Tyr Gln Ser Asp Pro
                85                  90                  95
gag atg cgc aac aag atc aac gcc ggc gcc atg aag tac cag gac atc      336
Glu Met Arg Asn Lys Ile Asn Ala Gly Ala Met Lys Tyr Gln Asp Ile
            100                 105                 110
cac ctg tcc cac tcg ggc ctg cag gtg gag cag ggt ttc ttc ggt cat      384
His Leu Ser His Ser Gly Leu Gln Val Glu Gln Gly Phe Phe Gly His
        115                 120                 125
ctg gat gtc gcc atc gtc gag gtc tgc cgc atc acc gag gac ggc cac      432
Leu Asp Val Ala Ile Val Glu Val Cys Arg Ile Thr Glu Asp Gly His
    130                 135                 140
ctc gtg cca tcc tcc ggt gtg ggc aac aac gtg gag tac ctg gag tcc      480
Leu Val Pro Ser Ser Gly Val Gly Asn Asn Val Glu Tyr Leu Glu Ser
145                 150                 155                 160
gcc cac aag atc atc atc gag gtc aac tcc tgg cag tcc ctg gag ctt      528
Ala His Lys Ile Ile Ile Glu Val Asn Ser Trp Gln Ser Leu Glu Leu
                165                 170                 175
gag ggc atg gcc gat atc tac cgc gtc ccc cac ctg ccc aac cgc acc      576
Glu Gly Met Ala Asp Ile Tyr Arg Val Pro His Leu Pro Asn Arg Thr
            180                 185                 190
ccc atc ccg atc aac aac gtc aat gac cgc atc ggt gag acc ttc atc      624
Pro Ile Pro Ile Asn Asn Val Asn Asp Arg Ile Gly Glu Thr Phe Ile
        195                 200                 205
cgc atc gat acc aac aag gtc gtc gcc gtc gtt gag acc gat tcc ccc      672
Arg Ile Asp Thr Asn Lys Val Val Ala Val Val Glu Thr Asp Ser Pro
    210                 215                 220
gac cgc aac agc ccc ttc aag ccg ttc gac gag acc tcc aag cag atc      720
Asp Arg Asn Ser Pro Phe Lys Pro Phe Asp Glu Thr Ser Lys Gln Ile

225                 230                 235                 240
gcc ggc aat ttc ctc gac ttc ctc gag ggg gag gtc cgc gcc ggc cgc      768
Ala Gly Asn Phe Leu Asp Phe Leu Glu Gly Glu Val Arg Ala Gly Arg
                245                 250                 255
ctc acc tac gac ggt tac atc atg cag tcc ggt gtg ggt aac gtc ccc      816
Leu Thr Tyr Asp Gly Tyr Ile Met Gln Ser Gly Val Gly Asn Val Pro
            260                 265                 270
aac gcc gtg atg gcc ggt ctg ctc gac tcg aag ttc gag aac atc aag      864
Asn Ala Val Met Ala Gly Leu Leu Asp Ser Lys Phe Glu Asn Ile Lys
        275                 280                 285
gcc tac acc gag gtg gtc cag gat ggc atg ctc gac ctc atc gat gcc      912
Ala Tyr Thr Glu Val Val Gln Asp Gly Met Leu Asp Leu Ile Asp Ala
    290                 295                 300
ggc aag atg acc cag gcc tcc gcc acc tcc ttc gcg ctg tcc ccg gag      960
Gly Lys Met Thr Gln Ala Ser Ala Thr Ser Phe Ala Leu Ser Pro Glu
305                 310                 315                 320
tac gcg gag aag atg aac cgt gag gcc agc acc tac cgc aag agc ctg      1008
Tyr Ala Glu Lys Met Asn Arg Glu Ala Ser Thr Tyr Arg Lys Ser Leu
                325                 330                 335
gtg ctg cgc cca ctg cag atc tcc aac agc ccc gag gtc atc cgc cgc      1056
Val Leu Arg Pro Leu Gln Ile Ser Asn Ser Pro Glu Val Ile Arg Arg
            340                 345                 350
atg ggt ctg atc gcc tcc aac ggc atg atc gag gcc gat atc tac ggt      1104
Met Gly Leu Ile Ala Ser Asn Gly Met Ile Glu Ala Asp Ile Tyr Gly
        355                 360                 365
aac gtc aac tcc acc cac gtg gcc ggt acc cgc atc atg aac ggc atc      1152
Asn Val Asn Ser Thr His Val Ala Gly Thr Arg Ile Met Asn Gly Ile
    370                 375                 380
ggc ggt tcc ggt gac ttc acc cgt aac gcg ttc gtg tcc tcc ttc atc      1200
Gly Gly Ser Gly Asp Phe Thr Arg Asn Ala Phe Val Ser Ser Phe Ile
385                 390                 395                 400
tcc ccg tcg gtg gcc aag ggg gat gcg atc tcc gcg gtc gtt ccg ttc      1248
Ser Pro Ser Val Ala Lys Gly Asp Ala Ile Ser Ala Val Val Pro Phe
                405                 410                 415
gcc tcc cac att gac cac acc gag cac gat gcc atg gtg gtc atc acc      1296
Ala Ser His Ile Asp His Thr Glu His Asp Ala Met Val Val Ile Thr
            420                 425                 430
gaa tat ggt tac gcc gac ctg cgt gga ctg gcc cca cgt gag cgt gcg      1344
Glu Tyr Gly Tyr Ala Asp Leu Arg Gly Leu Ala Pro Arg Glu Arg Ala
        435                 440                 445
ccg aag atg atc tcc atc gcg cac ccg gat tac cgt ccg ctg ctg gag      1392
Pro Lys Met Ile Ser Ile Ala His Pro Asp Tyr Arg Pro Leu Leu Glu
    450                 455                 460
gag tac ttc gag cgt gcc cag cag aac aag cac cag cac acc ccg cac      1440
Glu Tyr Phe Glu Arg Ala Gln Gln Asn Lys His Gln His Thr Pro His
465                 470                 475                 480
aac ctg cgc gat gcg ctg agc ttc cac atc aac ctg gat gag acc ggt      1488
Asn Leu Arg Asp Ala Leu Ser Phe His Ile Asn Leu Asp Glu Thr Gly
                485                 490                 495
tcg atg aag ggc aac gcc taa                                          1509
Ser Met Lys Gly Asn Ala
            500


<210> 70
<211> 502
<212> PRT
<213> Corynebacterium efficiens

<400> 70
Met Ser Glu Arg Ile Ala Ser Ala Glu Phe Arg Ser Lys Val Met Ser
1               5                   10                  15
Ala Glu Glu Ala Ala Gln Phe Val Asn Asn Gly Asp Thr Val Gly Met
            20                  25                  30
Ser Gly Phe Thr Gly Ala Ala Tyr Pro Lys Ala Leu Pro Thr Ala Ile
        35                  40                  45
Ala Asn Arg Ala Lys Glu Ala His Ser Lys Gly Asp Glu Tyr Ala Ile
    50                  55                  60
Asn Leu Leu Thr Gly Ala Ser Thr Ala Pro Asp Cys Asp Gly Val Leu
65                  70                  75                  80
Ala Glu Ala Asp Ala Ile Asn Tyr Arg Met Pro Tyr Gln Ser Asp Pro
                85                  90                  95

Glu Met Arg Asn Lys Ile Asn Ala Gly Ala Met Lys Tyr Gln Asp Ile
            100                 105                 110
His Leu Ser His Ser Gly Leu Gln Val Glu Gln Gly Phe Phe Gly His
        115                 120                 125
Leu Asp Val Ala Ile Val Glu Val Cys Arg Ile Thr Glu Asp Gly His
    130                 135                 140
Leu Val Pro Ser Ser Gly Val Gly Asn Asn Val Glu Tyr Leu Glu Ser
145                 150                 155                 160
Ala His Lys Ile Ile Ile Glu Val Asn Ser Trp Gln Ser Leu Glu Leu
                165                 170                 175
Glu Gly Met Ala Asp Ile Tyr Arg Val Pro His Leu Pro Asn Arg Thr
            180                 185                 190
Pro Ile Pro Ile Asn Asn Val Asn Asp Arg Ile Gly Glu Thr Phe Ile
        195                 200                 205
Arg Ile Asp Thr Asn Lys Val Val Ala Val Val Glu Thr Asp Ser Pro
    210                 215                 220
Asp Arg Asn Ser Pro Phe Lys Pro Phe Asp Glu Thr Ser Lys Gln Ile
225                 230                 235                 240
Ala Gly Asn Phe Leu Asp Phe Leu Glu Gly Glu Val Arg Ala Gly Arg
                245                 250                 255
Leu Thr Tyr Asp Gly Tyr Ile Met Gln Ser Gly Val Gly Asn Val Pro
            260                 265                 270
Asn Ala Val Met Ala Gly Leu Leu Asp Ser Lys Phe Glu Asn Ile Lys
        275                 280                 285
Ala Tyr Thr Glu Val Val Gln Asp Gly Met Leu Asp Leu Ile Asp Ala
    290                 295                 300
Gly Lys Met Thr Gln Ala Ser Ala Thr Ser Phe Ala Leu Ser Pro Glu
305                 310                 315                 320
Tyr Ala Glu Lys Met Asn Arg Glu Ala Ser Thr Tyr Arg Lys Ser Leu
                325                 330                 335
Val Leu Arg Pro Leu Gln Ile Ser Asn Ser Pro Glu Val Ile Arg Arg
            340                 345                 350
Met Gly Leu Ile Ala Ser Asn Gly Met Ile Glu Ala Asp Ile Tyr Gly
        355                 360                 365
Asn Val Asn Ser Thr His Val Ala Gly Thr Arg Ile Met Asn Gly Ile
    370                 375                 380
Gly Gly Ser Gly Asp Phe Thr Arg Asn Ala Phe Val Ser Ser Phe Ile
385                 390                 395                 400
Ser Pro Ser Val Ala Lys Gly Asp Ala Ile Ser Ala Val Val Pro Phe
                405                 410                 415
Ala Ser His Ile Asp His Thr Glu His Asp Ala Met Val Val Ile Thr
            420                 425                 430
Glu Tyr Gly Tyr Ala Asp Leu Arg Gly Leu Ala Pro Arg Glu Arg Ala
        435                 440                 445
Pro Lys Met Ile Ser Ile Ala His Pro Asp Tyr Arg Pro Leu Leu Glu
    450                 455                 460
Glu Tyr Phe Glu Arg Ala Gln Gln Asn Lys His Gln His Thr Pro His
465                 470                 475                 480
Asn Leu Arg Asp Ala Leu Ser Phe His Ile Asn Leu Asp Glu Thr Gly
                485                 490                 495
Ser Met Lys Gly Asn Ala
            500

<210> 71
<211> 1515
<212> DNA
<213> Xanthomonas campestris

<220>
<221> CDS
<222> (1)..(1515)

<400> 71
atg tcc gac gaa cgc atc ctc aac gcg ccg ctg cgc gac cac ctg gtc 48
Met Ser Asp Glu Arg Ile Leu Asn Ala Pro Leu Arg Asp His Leu Val
1               5                   10                  15
tct gcc gag gcc gct gct gcg ctg atc cag ccc ggc gag acc gtg gcg 96
Ser Ala Glu Ala Ala Ala Ala Leu Ile Gln Pro Gly Glu Thr Val Ala
            20                  25                  30
atg agc ggc ttc acc ggg tcg ggt tat ccc aag gca gtg ccg atg gcg 144
Met Ser Gly Phe Thr Gly Ser Gly Tyr Pro Lys Ala Val Pro Met Ala
        35                  40                  45

ctg gcg cag cgc atc gaa gcg gcg cac ctg cag ggc gaa gcg ttc cag 192
Leu Ala Gln Arg Ile Glu Ala Ala His Leu Gln Gly Glu Ala Phe Gln
    50                  55                  60
atc aag ctg atg acc ggc gcc tcc acc gcg ccc gag ctc gat ggc gcg 240
Ile Lys Leu Met Thr Gly Ala Ser Thr Ala Pro Glu Leu Asp Gly Ala
65                  70                  75                  80
ctg gcc aag gcc gac ggc atc gcc atg cgc atg ccg ttc cag tcc gac 288
Leu Ala Lys Ala Asp Gly Ile Ala Met Arg Met Pro Phe Gln Ser Asp
                85                  90                  95
ccg gat gcg cgc caa cgc atc aat gcc ggc acg ctg gac tac atc gat 336
Pro Asp Ala Arg Gln Arg Ile Asn Ala Gly Thr Leu Asp Tyr Ile Asp
            100                 105                 110
atc cat ctg agc cac gtt gcc cag cac gtg tgg ttc ggc ttc tac ggg 384
Ile His Leu Ser His Val Ala Gln His Val Trp Phe Gly Phe Tyr Gly
        115                 120                 125
cag atc gac acc gcg gtg gtg gaa gtg tcg gcg atc cgc gcc gat ggc 432
Gln Ile Asp Thr Ala Val Val Glu Val Ser Ala Ile Arg Ala Asp Gly
    130                 135                 140
agc ctg gtg ccg tcc acc tcc atc ggt aac aac aag acc tgg ctg gac 480
Ser Leu Val Pro Ser Thr Ser Ile Gly Asn Asn Lys Thr Trp Leu Asp
145                 150                 155                 160
ctg gcc agg aaa gtg atc atc gag gtc aac gac tgg caa ccg gcc ggc 528
Leu Ala Arg Lys Val Ile Ile Glu Val Asn Asp Trp Gln Pro Ala Gly
                165                 170                 175
ctg gat ggc atg cac gat gtg tat tac ggc acc gcg ctg cca ccg cac 576
Leu Asp Gly Met His Asp Val Tyr Tyr Gly Thr Ala Leu Pro Pro His
            180                 185                 190
cgc aaa ccg att ccg ctg ctg cac ggc gac gac cgc atc ggc gag ccc 624
Arg Lys Pro Ile Pro Leu Leu His Gly Asp Asp Arg Ile Gly Glu Pro
        195                 200                 205
agc ctg cgc tgc gac ccg gac aag atc gtg gcc gtg gtg cgt acg cat 672
Ser Leu Arg Cys Asp Pro Asp Lys Ile Val Ala Val Val Arg Thr His
    210                 215                 220
ggc ccg gac cgc aac agc ccg ttt gcc gct gcc gat gac agc agc gag 720
Gly Pro Asp Arg Asn Ser Pro Phe Ala Ala Ala Asp Asp Ser Ser Glu
225                 230                 235                 240
cgc atc gcc gaa cac ctg atc gcc ttc ctg cga cac gag gtg agc aag 768
Arg Ile Ala Glu His Leu Ile Ala Phe Leu Arg His Glu Val Ser Lys
                245                 250                 255
ggc cgg ctg ccg gcg aac ctg ctg ccg ctg caa tcg ggc gtg ggc aat 816
Gly Arg Leu Pro Ala Asn Leu Leu Pro Leu Gln Ser Gly Val Gly Asn
            260                 265                 270
att ccc aat gcg gtg ctg gcc ggc ctg gca cgt agc ggt ttc cgc gac 864
Ile Pro Asn Ala Val Leu Ala Gly Leu Ala Arg Ser Gly Phe Arg Asp
        275                 280                 285
ctg gaa gcc ttc acc gaa gtg atc cag gac ggc atg ctg gcg ctg ctg 912
Leu Glu Ala Phe Thr Glu Val Ile Gln Asp Gly Met Leu Ala Leu Leu
    290                 295                 300
cgc gat ggc gtc ttg cgt tat gcc tcg tgc acc ggt ttt gcg ctc agc 960
Arg Asp Gly Val Leu Arg Tyr Ala Ser Cys Thr Gly Phe Ala Leu Ser
305                 310                 315                 320
ccg gaa ggc aac gaa gaa ttc aag cgc aac atc gat ttc tac cgg cag 1008
Pro Glu Gly Asn Glu Glu Phe Lys Arg Asn Ile Asp Phe Tyr Arg Gln
                325                 330                 335
cgc atc gtg atg cgc acg cag gag atc tcc aac cat ccc gag ctg gtg 1056
Arg Ile Val Met Arg Thr Gln Glu Ile Ser Asn His Pro Glu Leu Val
            340                 345                 350
cgg cgg ctg ggc tgc atc ggc atg aac ggc atg atc gag gcc gac ctg 1104
Arg Arg Leu Gly Cys Ile Gly Met Asn Gly Met Ile Glu Ala Asp Leu
        355                 360                 365
tac ggc aac gtc aat tcc acc cat gtc atg ggc agc cgc atc atg aac 1152
Tyr Gly Asn Val Asn Ser Thr His Val Met Gly Ser Arg Ile Met Asn
    370                 375                 380
ggc atc ggc ggc tcc ggc gat ttc gcg cgc aac ggg ttc ctg tcg atc 1200
Gly Ile Gly Gly Ser Gly Asp Phe Ala Arg Asn Gly Phe Leu Ser Ile
385                 390                 395                 400
ttc ctc agc ccc agc atc gcc aag gcc ggc agt att tcc tca atc gtg 1248
Phe Leu Ser Pro Ser Ile Ala Lys Ala Gly Ser Ile Ser Ser Ile Val
                405                 410                 415
ccg atg gtc agc cat gtc gac cac acc gaa cac gac gtc tca gtg atc 1296
Pro Met Val Ser His Val Asp His Thr Glu His Asp Val Ser Val Ile
            420                 425                 430

gtg acc gaa caa ggc ctg gcc gac ctg cgc ggc ctg acg ccc aag caa 1344
Val Thr Glu Gln Gly Leu Ala Asp Leu Arg Gly Leu Thr Pro Lys Gln
        435                 440                 445
cgt gca cgc cag ctg cta gag cac tgc gtg cat ccg cgt tac cgc gac 1392
Arg Ala Arg Gln Leu Leu Glu His Cys Val His Pro Arg Tyr Arg Asp
    450                 455                 460
gcg ctg gaa gac tac gtg gca cgt gcc aat cgc gac agc tat ggc aag 1440
Ala Leu Glu Asp Tyr Val Ala Arg Ala Asn Arg Asp Ser Tyr Gly Lys
465                 470                 475                 480
cac acg ccg cat ctg ctg acc gaa gca ttg gcg tgg cac cag cgg tgg 1488
His Thr Pro His Leu Leu Thr Glu Ala Leu Ala Trp His Gln Arg Trp
                485                 490                 495
ctg gag act gga gat atg ttg ggg tga 1515
Leu Glu Thr Gly Asp Met Leu Gly
            500

<210> 72
<211> 504
<212> PRT
<213> Xanthomonas campestris

<400> 72
Met Ser Asp Glu Arg Ile Leu Asn Ala Pro Leu Arg Asp His Leu Val
1               5                   10                  15
Ser Ala Glu Ala Ala Ala Ala Leu Ile Gln Pro Gly Glu Thr Val Ala
            20                  25                  30
Met Ser Gly Phe Thr Gly Ser Gly Tyr Pro Lys Ala Val Pro Met Ala
        35                  40                  45
Leu Ala Gln Arg Ile Glu Ala Ala His Leu Gln Gly Glu Ala Phe Gln
    50                  55                  60
Ile Lys Leu Met Thr Gly Ala Ser Thr Ala Pro Glu Leu Asp Gly Ala
65                  70                  75                  80
Leu Ala Lys Ala Asp Gly Ile Ala Met Arg Met Pro Phe Gln Ser Asp
                85                  90                  95
Pro Asp Ala Arg Gln Arg Ile Asn Ala Gly Thr Leu Asp Tyr Ile Asp
            100                 105                 110
Ile His Leu Ser His Val Ala Gln His Val Trp Phe Gly Phe Tyr Gly
        115                 120                 125
Gln Ile Asp Thr Ala Val Val Glu Val Ser Ala Ile Arg Ala Asp Gly
    130                 135                 140
Ser Leu Val Pro Ser Thr Ser Ile Gly Asn Asn Lys Thr Trp Leu Asp
145                 150                 155                 160
Leu Ala Arg Lys Val Ile Ile Glu Val Asn Asp Trp Gln Pro Ala Gly
                165                 170                 175
Leu Asp Gly Met His Asp Val Tyr Tyr Gly Thr Ala Leu Pro Pro His
            180                 185                 190
Arg Lys Pro Ile Pro Leu Leu His Gly Asp Asp Arg Ile Gly Glu Pro
        195                 200                 205
Ser Leu Arg Cys Asp Pro Asp Lys Ile Val Ala Val Val Arg Thr His
    210                 215                 220
Gly Pro Asp Arg Asn Ser Pro Phe Ala Ala Ala Asp Asp Ser Ser Glu
225                 230                 235                 240
Arg Ile Ala Glu His Leu Ile Ala Phe Leu Arg His Glu Val Ser Lys
                245                 250                 255
Gly Arg Leu Pro Ala Asn Leu Leu Pro Leu Gln Ser Gly Val Gly Asn
            260                 265                 270
Ile Pro Asn Ala Val Leu Ala Gly Leu Ala Arg Ser Gly Phe Arg Asp
        275                 280                 285
Leu Glu Ala Phe Thr Glu Val Ile Gln Asp Gly Met Leu Ala Leu Leu
    290                 295                 300
Arg Asp Gly Val Leu Arg Tyr Ala Ser Cys Thr Gly Phe Ala Leu Ser
305                 310                 315                 320
Pro Glu Gly Asn Glu Glu Phe Lys Arg Asn Ile Asp Phe Tyr Arg Gln
                325                 330                 335
Arg Ile Val Met Arg Thr Gln Glu Ile Ser Asn His Pro Glu Leu Val
            340                 345                 350
Arg Arg Leu Gly Cys Ile Gly Met Asn Gly Met Ile Glu Ala Asp Leu
        355                 360                 365
Tyr Gly Asn Val Asn Ser Thr His Val Met Gly Ser Arg Ile Met Asn
    370                 375                 380
Gly Ile Gly Gly Ser Gly Asp Phe Ala Arg Asn Gly Phe Leu Ser Ile

385                 390                 395                 400
Phe Leu Ser Pro Ser Ile Ala Lys Ala Gly Ser Ile Ser Ser Ile Val
                405                 410                 415
Pro Met Val Ser His Val Asp His Thr Glu His Asp Val Ser Val Ile
            420                 425                 430
Val Thr Glu Gln Gly Leu Ala Asp Leu Arg Gly Leu Thr Pro Lys Gln
        435                 440                 445
Arg Ala Arg Gln Leu Leu Glu His Cys Val His Pro Arg Tyr Arg Asp
    450                 455                 460
Ala Leu Glu Asp Tyr Val Ala Arg Ala Asn Arg Asp Ser Tyr Gly Lys
465                 470                 475                 480
His Thr Pro His Leu Leu Thr Glu Ala Leu Ala Trp His Gln Arg Trp
                485                 490                 495
Leu Glu Thr Gly Asp Met Leu Gly
            500

<210> 73
<211> 1617
<212> DNA
<213> Clostridium kluyveri

<220>
<221> CDS
<222> (1)..(1617)

<400> 73
atg agt aaa ggg ata aag aat tca caa ttg aaa aaa aag aat gta aag      48
Met Ser Lys Gly Ile Lys Asn Ser Gln Leu Lys Lys Lys Asn Val Lys
1               5                   10                  15
gct agt aat gtg gca gaa aag att gaa gag aaa gtt gaa aaa aca gat      96
Ala Ser Asn Val Ala Glu Lys Ile Glu Glu Lys Val Glu Lys Thr Asp
            20                  25                  30
aag gtt gtt gaa aag gca gct gag gtt act gaa aaa cga att aga aac      144
Lys Val Val Glu Lys Ala Ala Glu Val Thr Glu Lys Arg Ile Arg Asn
        35                  40                  45
ttg aag ctt cag gaa aaa gtt gta aca gca gat gtg gca gct gat atg      192
Leu Lys Leu Gln Glu Lys Val Val Thr Ala Asp Val Ala Ala Asp Met
    50                  55                  60
ata gaa aac ggt atg att gtt gca att agc gga ttt act cct tcc ggg      240
Ile Glu Asn Gly Met Ile Val Ala Ile Ser Gly Phe Thr Pro Ser Gly
65                  70                  75                  80
tat cct aaa gaa gta cct aaa gca ttg act aaa aaa gtt aat gcc tta      288
Tyr Pro Lys Glu Val Pro Lys Ala Leu Thr Lys Lys Val Asn Ala Leu
                85                  90                  95
gag gaa gaa ttc aag gta aca ctt tat aca ggt tca tct aca gga gcc      336
Glu Glu Glu Phe Lys Val Thr Leu Tyr Thr Gly Ser Ser Thr Gly Ala
            100                 105                 110
gat ata gac gga gaa tgg gca aaa gca gga ata ata gaa aga aga att      384
Asp Ile Asp Gly Glu Trp Ala Lys Ala Gly Ile Ile Glu Arg Arg Ile
        115                 120                 125
cca tat cag aca aat tct gat atg agg aaa aaa ata aat gat ggt tct      432
Pro Tyr Gln Thr Asn Ser Asp Met Arg Lys Lys Ile Asn Asp Gly Ser
    130                 135                 140
att aag tat gct gat atg cat tta agc cat atg gct caa tat att aat      480
Ile Lys Tyr Ala Asp Met His Leu Ser His Met Ala Gln Tyr Ile Asn
145                 150                 155                 160
tat tct gta att cct aaa gta gat ata gct ata ata gag gca gta gct      528
Tyr Ser Val Ile Pro Lys Val Asp Ile Ala Ile Ile Glu Ala Val Ala
                165                 170                 175
att aca gaa gaa ggg gat att att cct tca aca gga att gga aat aca      576
Ile Thr Glu Glu Gly Asp Ile Ile Pro Ser Thr Gly Ile Gly Asn Thr
            180                 185                 190
gct act ttt gtg gaa aat gca gat aag gta ata gtg gaa att aat gag      624
Ala Thr Phe Val Glu Asn Ala Asp Lys Val Ile Val Glu Ile Asn Glu
        195                 200                 205
gct caa ccg ctt gaa ttg gaa ggt atg gca gat ata tat aca tta aaa      672
Ala Gln Pro Leu Glu Leu Glu Gly Met Ala Asp Ile Tyr Thr Leu Lys
    210                 215                 220
aac cct cca aga aga gag ccc ata cct ata gtt aat gca ggc aat agg      720
Asn Pro Pro Arg Arg Glu Pro Ile Pro Ile Val Asn Ala Gly Asn Arg
225                 230                 235                 240
ata ggg acc aca tat gtg acc tgt ggt tct gaa aaa ata tgc gct ata      768

Ile Gly Thr Thr Tyr Val Thr Cys Gly Ser Glu Lys Ile Cys Ala Ile
            245                 250                 255
gtg atg aca aat acc cag gat aaa aca aga cct ctt aca gaa gtg tct      816
Val Met Thr Asn Thr Gln Asp Lys Thr Arg Pro Leu Thr Glu Val Ser
            260                 265                 270
cct gta tct cag gct ata tcc gat aat ctt ata gga ttt tta aat aaa      864
Pro Val Ser Gln Ala Ile Ser Asp Asn Leu Ile Gly Phe Leu Asn Lys
        275                 280                 285
gag gtt gaa gag gga aaa tta cct aag aac ctg ctt cct ata cag tca      912
Glu Val Glu Glu Gly Lys Leu Pro Lys Asn Leu Leu Pro Ile Gln Ser
    290                 295                 300
gga gtt gga agt gta gca aat gca gtt ttg gcc gga ctt tgt gaa tca      960
Gly Val Gly Ser Val Ala Asn Ala Val Leu Ala Gly Leu Cys Glu Ser
305                 310                 315                 320
aat ttt aaa aat ttg agt tgt tat aca gaa gtt ata cag gat tct atg      1008
Asn Phe Lys Asn Leu Ser Cys Tyr Thr Glu Val Ile Gln Asp Ser Met
            325                 330                 335
ctg aag ctt ata aaa tgt ggt aaa gca gat gtg gtg tca ggc act tcc      1056
Leu Lys Leu Ile Lys Cys Gly Lys Ala Asp Val Val Ser Gly Thr Ser
            340                 345                 350
ata agt cct tca ccg gag atg ttg cct gag ttc ata aag gac ata aat      1104
Ile Ser Pro Ser Pro Glu Met Leu Pro Glu Phe Ile Lys Asp Ile Asn
        355                 360                 365
ttc ttt aga gaa aag ata gta tta aga cca cag gaa ata agt aat aat      1152
Phe Phe Arg Glu Lys Ile Val Leu Arg Pro Gln Glu Ile Ser Asn Asn
    370                 375                 380
cca gag ata gca aga aga ata gga gtt ata tcc ata aac act gct ttg      1200
Pro Glu Ile Ala Arg Arg Ile Gly Val Ile Ser Ile Asn Thr Ala Leu
385                 390                 395                 400
gaa gta gat ata tat ggt aat gta aac tcc act cat gtt atg gga agc      1248
Glu Val Asp Ile Tyr Gly Asn Val Asn Ser Thr His Val Met Gly Ser
            405                 410                 415
aaa atg atg aat ggt ata ggc ggt tct gga gac ttt gcc aga aat gca      1296
Lys Met Met Asn Gly Ile Gly Gly Ser Gly Asp Phe Ala Arg Asn Ala
            420                 425                 430
tat ttg act ata ttc act aca gag tct atc gcc aaa aaa gga gat ata      1344
Tyr Leu Thr Ile Phe Thr Thr Glu Ser Ile Ala Lys Lys Gly Asp Ile
        435                 440                 445
tca tct ata gtt cct atg gta tcc cat gtg gat cat aca gaa cat gat      1392
Ser Ser Ile Val Pro Met Val Ser His Val Asp His Thr Glu His Asp
    450                 455                 460
gta atg gta att gtt aca gaa cag gga gta gca gat tta aga ggt ctt      1440
Val Met Val Ile Val Thr Glu Gln Gly Val Ala Asp Leu Arg Gly Leu
465                 470                 475                 480
tct cct agg gaa aag gcc gtg gct ata ata gaa aat tgt gtt cat cct      1488
Ser Pro Arg Glu Lys Ala Val Ala Ile Ile Glu Asn Cys Val His Pro
            485                 490                 495
gat tac aag gat atg ctt atg gaa tat ttt gaa gag gct tgt aag tca      1536
Asp Tyr Lys Asp Met Leu Met Glu Tyr Phe Glu Glu Ala Cys Lys Ser
            500                 505                 510
tca ggt gga aat aca cca cat aat ctt gaa aaa gct ctt tcc tgg cat      1584
Ser Gly Gly Asn Thr Pro His Asn Leu Glu Lys Ala Leu Ser Trp His
        515                 520                 525
aca aaa ttt ata aaa act ggt agt atg aaa taa                          1617
Thr Lys Phe Ile Lys Thr Gly Ser Met Lys
    530                 535

<210> 74
<211> 538
<212> PRT
<213> Clostridium kluyveri

<400> 74
Met Ser Lys Gly Ile Lys Asn Ser Gln Leu Lys Lys Lys Asn Val Lys
1               5                   10                  15
Ala Ser Asn Val Ala Glu Lys Ile Glu Glu Lys Val Glu Lys Thr Asp
            20                  25                  30
Lys Val Val Glu Lys Ala Ala Glu Val Thr Glu Lys Arg Ile Arg Asn
        35                  40                  45
Leu Lys Leu Gln Glu Lys Val Val Thr Ala Asp Val Ala Ala Asp Met
    50                  55                  60

Ile Glu Asn Gly Met Ile Val Ala Ile Ser Gly Phe Thr Pro Ser Gly
65                  70                  75                  80
Tyr Pro Lys Glu Val Pro Lys Ala Leu Thr Lys Lys Val Asn Ala Leu
                85                  90                  95
Glu Glu Glu Phe Lys Val Thr Leu Tyr Thr Gly Ser Ser Thr Gly Ala
            100                 105                 110
Asp Ile Asp Gly Glu Trp Ala Lys Ala Gly Ile Ile Glu Arg Arg Ile
        115                 120                 125
Pro Tyr Gln Thr Asn Ser Asp Met Arg Lys Lys Ile Asn Asp Gly Ser
    130                 135                 140
Ile Lys Tyr Ala Asp Met His Leu Ser His Met Ala Gln Tyr Ile Asn
145                 150                 155                 160
Tyr Ser Val Ile Pro Lys Val Asp Ile Ala Ile Ile Glu Ala Val Ala
                165                 170                 175
Ile Thr Glu Glu Gly Asp Ile Ile Pro Ser Thr Gly Ile Gly Asn Thr
            180                 185                 190
Ala Thr Phe Val Glu Asn Ala Asp Lys Val Ile Val Glu Ile Asn Glu
        195                 200                 205
Ala Gln Pro Leu Glu Leu Glu Gly Met Ala Asp Ile Tyr Thr Leu Lys
    210                 215                 220
Asn Pro Pro Arg Arg Glu Pro Ile Pro Ile Val Asn Ala Gly Asn Arg
225                 230                 235                 240
Ile Gly Thr Thr Tyr Val Thr Cys Gly Ser Glu Lys Ile Cys Ala Ile
                245                 250                 255
Val Met Thr Asn Thr Gln Asp Lys Thr Arg Pro Leu Thr Glu Val Ser
            260                 265                 270
Pro Val Ser Gln Ala Ile Ser Asp Asn Leu Ile Gly Phe Leu Asn Lys
        275                 280                 285
Glu Val Glu Glu Gly Lys Leu Pro Lys Asn Leu Leu Pro Ile Gln Ser
    290                 295                 300
Gly Val Gly Ser Val Ala Asn Ala Val Leu Ala Gly Leu Cys Glu Ser
305                 310                 315                 320
Asn Phe Lys Asn Leu Ser Cys Tyr Thr Glu Val Ile Gln Asp Ser Met
                325                 330                 335
Leu Lys Leu Ile Lys Cys Gly Lys Ala Asp Val Val Ser Gly Thr Ser
            340                 345                 350
Ile Ser Pro Ser Pro Glu Met Leu Pro Glu Phe Ile Lys Asp Ile Asn
        355                 360                 365
Phe Phe Arg Glu Lys Ile Val Leu Arg Pro Gln Glu Ile Ser Asn Asn
    370                 375                 380
Pro Glu Ile Ala Arg Arg Ile Gly Val Ile Ser Ile Asn Thr Ala Leu
385                 390                 395                 400
Glu Val Asp Ile Tyr Gly Asn Val Asn Ser Thr His Val Met Gly Ser
                405                 410                 415
Lys Met Met Asn Gly Ile Gly Gly Ser Gly Asp Phe Ala Arg Asn Ala
            420                 425                 430
Tyr Leu Thr Ile Phe Thr Thr Glu Ser Ile Ala Lys Lys Gly Asp Ile
        435                 440                 445
Ser Ser Ile Val Pro Met Val Ser His Val Asp His Thr Glu His Asp
    450                 455                 460
Val Met Val Ile Val Thr Glu Gln Gly Val Ala Asp Leu Arg Gly Leu
465                 470                 475                 480
Ser Pro Arg Glu Lys Ala Val Ala Ile Ile Glu Asn Cys Val His Pro
                485                 490                 495
Asp Tyr Lys Asp Met Leu Met Glu Tyr Phe Glu Glu Ala Cys Lys Ser
            500                 505                 510
Ser Gly Gly Asn Thr Pro His Asn Leu Glu Lys Ala Leu Ser Trp His
        515                 520                 525
Thr Lys Phe Ile Lys Thr Gly Ser Met Lys
    530                 535

<210> 75
<211> 1563
<212> DNA
<213> Geobacter sulfurreducens

<220>
<221> CDS
<222> (1)..(1563)

<400> 75
atg tcg gaa ttg cac aga aga atc agg aag acg aat ctc cat gag cgg        48

Met Ser Glu Leu His Arg Arg Ile Arg Lys Thr Asn Leu His Glu Arg
1               5                   10                  15
atc agg acg gta gaa gag gtc atc ccc ctc ttc cag gac ggc atg aac      96
Ile Arg Thr Val Glu Glu Val Ile Pro Leu Phe Gln Asp Gly Met Asn
            20                  25                  30
ctg ggc tgg tcg ggc ttc acc ccc gcc ggc tac ccc aag gcg gta ccc      144
Leu Gly Trp Ser Gly Phe Thr Pro Ala Gly Tyr Pro Lys Ala Val Pro
        35                  40                  45
atc gcc ctg gcg gac cat gtg gag aag aac ggg ctg cag ggg aag ctg      192
Ile Ala Leu Ala Asp His Val Glu Lys Asn Gly Leu Gln Gly Lys Leu
    50                  55                  60
cgc ttc aac ctc ttc atc ggc gcc tcc gtg ggc gtc gag acg gaa gac      240
Arg Phe Asn Leu Phe Ile Gly Ala Ser Val Gly Val Glu Thr Glu Asp
65                  70                  75                  80
cgc tgg gcc tcc ctg gac atg atc gac cgc cgc tgg ccc tac cag acc      288
Arg Trp Ala Ser Leu Asp Met Ile Asp Arg Arg Trp Pro Tyr Gln Thr
                85                  90                  95
ggc aag aac atc cag gcc ggc atc aac tcc ggc cgc atc cgc atg ggt      336
Gly Lys Asn Ile Gln Ala Gly Ile Asn Ser Gly Arg Ile Arg Met Gly
            100                 105                 110
gac cgc cac ctc tca atg ttc gcc cag gat ttg ggg tac ggc ttc tac      384
Asp Arg His Leu Ser Met Phe Ala Gln Asp Leu Gly Tyr Gly Phe Tyr
        115                 120                 125
acc aag gag aac ggc ggc cgg ctg gat ctg gcc atc atc gag tgc tcc      432
Thr Lys Glu Asn Gly Gly Arg Leu Asp Leu Ala Ile Ile Glu Cys Ser
    130                 135                 140
gcc atc acc gag aac ggc ggt ctg gtg ctg acc gcc tcc tgc ggc gcc      480
Ala Ile Thr Glu Asn Gly Gly Leu Val Leu Thr Ala Ser Cys Gly Ala
145                 150                 155                 160
gtc ccc gag atc gtc cag atc gcc gac aag atc atc atc gag atc aac      528
Val Pro Glu Ile Val Gln Ile Ala Asp Lys Ile Ile Ile Glu Ile Asn
                165                 170                 175
acc gcc atc ccc aac tat gaa ggc ctg cac gac atc atc gaa ccc gtc      576
Thr Ala Ile Pro Asn Tyr Glu Gly Leu His Asp Ile Ile Glu Pro Val
            180                 185                 190
agc cct ccc aac cgg ctc ccc tac ctc atc agc cgg gtg gat gac cgg      624
Ser Pro Pro Asn Arg Leu Pro Tyr Leu Ile Ser Arg Val Asp Asp Arg
        195                 200                 205
gcc gga tct ccc tac gtc cgc gtc gat acc gac aag atc gtc gcc atc      672
Ala Gly Ser Pro Tyr Val Arg Val Asp Thr Asp Lys Ile Val Ala Ile
    210                 215                 220
gtc gag tcc acc cat ccg gac aac ggc cgc gct ttt gcc gag cag gac      720
Val Glu Ser Thr His Pro Asp Asn Gly Arg Ala Phe Ala Glu Gln Asp
225                 230                 235                 240
gac acc tcc gag gcc att gcc aac aat atc ctg gac ttc ttc cag gca      768
Asp Thr Ser Glu Ala Ile Ala Asn Asn Ile Leu Asp Phe Phe Gln Ala
                245                 250                 255
gag gtg aag gcg gga cgc ctc ccc aag aac ctg ctg ccg ctc cag tcc      816
Glu Val Lys Ala Gly Arg Leu Pro Lys Asn Leu Leu Pro Leu Gln Ser
            260                 265                 270
ggc gtc ggc tcc atc gcc aac gcc gtt atc ggc ggt ctg gcc aag ggc      864
Gly Val Gly Ser Ile Ala Asn Ala Val Ile Gly Gly Leu Ala Lys Gly
        275                 280                 285
ccc ttc tcc ggc ctg agg gtc tgg acc gag gta ctg cag gac acc atg      912
Pro Phe Ser Gly Leu Arg Val Trp Thr Glu Val Leu Gln Asp Thr Met
    290                 295                 300
ctt gac ctc ttc gac tcc ggc aag ctc gat ttc gcc tcc acc gtc tcc      960
Leu Asp Leu Phe Asp Ser Gly Lys Leu Asp Phe Ala Ser Thr Val Ser
305                 310                 315                 320
ctc tcc ttc tcc gtg gac ggc ttc aag cgc ttc tac ggc gac tgg gac      1008
Leu Ser Phe Ser Val Asp Gly Phe Lys Arg Phe Tyr Gly Asp Trp Asp
                325                 330                 335
aag tac agc gac aag gtc atc atg cgc ccc ctc tcc atc gcc aac cac      1056
Lys Tyr Ser Asp Lys Val Ile Met Arg Pro Leu Ser Ile Ala Asn His
            340                 345                 350
ccg gag ccc atc cgg cgc ctg gga tgc att gcc atg aac act ccc gtg      1104
Pro Glu Pro Ile Arg Arg Leu Gly Cys Ile Ala Met Asn Thr Pro Val
        355                 360                 365
gag ttc gac atc tac gcc cac gcc aac tcc acc ctg gtc ggc ggc acg      1152
Glu Phe Asp Ile Tyr Ala His Ala Asn Ser Thr Leu Val Gly Gly Thr
    370                 375                 380
cgc atg atc aac ggc atc ggc ggc tcc ggc gat ttc ctg cgc aac gcc      1200

Arg Met Ile Asn Gly Ile Gly Gly Ser Gly Asp Phe Leu Arg Asn Ala
385                 390                 395                 400
tac ctc tcc atc atg cac acc ccg tcg gcc cgc ccc acc aag acc gac      1248
Tyr Leu Ser Ile Met His Thr Pro Ser Ala Arg Pro Thr Lys Thr Asp
                405                 410                 415
ccc acc ggc atc acc tgc gtc gtg ccc cac gtc ccg cac gtt gac cat      1296
Pro Thr Gly Ile Thr Cys Val Val Pro His Val Pro His Val Asp His
            420                 425                 430
acc gag cac gac ctt gac gtg ctg gtc acc gag cag ggc cta gcc gac      1344
Thr Glu His Asp Leu Asp Val Leu Val Thr Glu Gln Gly Leu Ala Asp
        435                 440                 445
ctg cgg ggc ctg gcc ccc aag gac cgc gcc cgc gag atc atc gcc aag      1392
Leu Arg Gly Leu Ala Pro Lys Asp Arg Ala Arg Glu Ile Ile Ala Lys
    450                 455                 460
tgc gcc cac ccg gac tac aag ccc ctc ctc acc gag tac ctg gag atg      1440
Cys Ala His Pro Asp Tyr Lys Pro Leu Leu Thr Glu Tyr Leu Glu Met
465                 470                 475                 480
gcc acc agg gac tgc atg gcc cgc aag gcg ggg cat gag ccc cag ttg      1488
Ala Thr Arg Asp Cys Met Ala Arg Lys Ala Gly His Glu Pro Gln Leu
                485                 490                 495
ctg gac cgg gtg ttc aag atg cag gtg aac ctg gcc aag aac gga aca      1536
Leu Asp Arg Val Phe Lys Met Gln Val Asn Leu Ala Lys Asn Gly Thr
            500                 505                 510
atg aag atc gac aac tgg gat atc tag                                  1563
Met Lys Ile Asp Asn Trp Asp Ile
        515                 520

<210> 76
<211> 520
<212> PRT
<213> Geobacter sulfurreducens

<400> 76
Met Ser Glu Leu His Arg Arg Ile Arg Lys Thr Asn Leu His Glu Arg
1               5                   10                  15
Ile Arg Thr Val Glu Glu Val Ile Pro Leu Phe Gln Asp Gly Met Asn
            20                  25                  30
Leu Gly Trp Ser Gly Phe Thr Pro Ala Gly Tyr Pro Lys Ala Val Pro
        35                  40                  45
Ile Ala Leu Ala Asp His Val Glu Lys Asn Gly Leu Gln Gly Lys Leu
    50                  55                  60
Arg Phe Asn Leu Phe Ile Gly Ala Ser Val Gly Val Glu Thr Glu Asp
65                  70                  75                  80
Arg Trp Ala Ser Leu Asp Met Ile Asp Arg Arg Trp Pro Tyr Gln Thr
                85                  90                  95
Gly Lys Asn Ile Gln Ala Gly Ile Asn Ser Gly Arg Ile Arg Met Gly
            100                 105                 110
Asp Arg His Leu Ser Met Phe Ala Gln Asp Leu Gly Tyr Gly Phe Tyr
        115                 120                 125
Thr Lys Glu Asn Gly Gly Arg Leu Asp Leu Ala Ile Ile Glu Cys Ser
    130                 135                 140
Ala Ile Thr Glu Asn Gly Gly Leu Val Leu Thr Ala Ser Cys Gly Ala
145                 150                 155                 160
Val Pro Glu Ile Val Gln Ile Ala Asp Lys Ile Ile Ile Glu Ile Asn
                165                 170                 175
Thr Ala Ile Pro Asn Tyr Glu Gly Leu His Asp Ile Ile Glu Pro Val
            180                 185                 190
Ser Pro Pro Asn Arg Leu Pro Tyr Leu Ile Ser Arg Val Asp Asp Arg
        195                 200                 205
Ala Gly Ser Pro Tyr Val Arg Val Asp Thr Asp Lys Ile Val Ala Ile
    210                 215                 220
Val Glu Ser Thr His Pro Asp Asn Gly Arg Ala Phe Ala Glu Gln Asp
225                 230                 235                 240
Asp Thr Ser Glu Ala Ile Ala Asn Asn Ile Leu Asp Phe Phe Gln Ala
                245                 250                 255
Glu Val Lys Ala Gly Arg Leu Pro Lys Asn Leu Leu Pro Leu Gln Ser
            260                 265                 270
Gly Val Gly Ser Ile Ala Asn Ala Val Ile Gly Gly Leu Ala Lys Gly
        275                 280                 285
Pro Phe Ser Gly Leu Arg Val Trp Thr Glu Val Leu Gln Asp Thr Met
    290                 295                 300

Leu Asp Leu Phe Asp Ser Gly Lys Leu Asp Phe Ala Ser Thr Val Ser
305                 310                 315                 320
Leu Ser Phe Ser Val Asp Gly Phe Lys Arg Phe Tyr Gly Asp Trp Asp
                325                 330                 335
Lys Tyr Ser Asp Lys Val Ile Met Arg Pro Leu Ser Ile Ala Asn His
            340                 345                 350
Pro Glu Pro Ile Arg Arg Leu Gly Cys Ile Ala Met Asn Thr Pro Val
        355                 360                 365
Glu Phe Asp Ile Tyr Ala His Ala Asn Ser Thr Leu Val Gly Gly Thr
    370                 375                 380
Arg Met Ile Asn Gly Ile Gly Gly Ser Gly Asp Phe Leu Arg Asn Ala
385                 390                 395                 400
Tyr Leu Ser Ile Met His Thr Pro Ser Ala Arg Pro Thr Lys Thr Asp
                405                 410                 415
Pro Thr Gly Ile Thr Cys Val Val Pro His Val Pro His Val Asp His
            420                 425                 430
Thr Glu His Asp Leu Asp Val Leu Val Thr Glu Gln Gly Leu Ala Asp
        435                 440                 445
Leu Arg Gly Leu Ala Pro Lys Asp Arg Ala Arg Glu Ile Ile Ala Lys
    450                 455                 460
Cys Ala His Pro Asp Tyr Lys Pro Leu Leu Thr Glu Tyr Leu Glu Met
465                 470                 475                 480
Ala Thr Arg Asp Cys Met Ala Arg Lys Ala Gly His Glu Pro Gln Leu
                485                 490                 495
Leu Asp Arg Val Phe Lys Met Gln Val Asn Leu Ala Lys Asn Gly Thr
            500                 505                 510
Met Lys Ile Asp Asn Trp Asp Ile
        515                 520

<210> 77
<211> 1596
<212> DNA
<213> Geobacter sulfurreducens

<220>
<221> CDS
<222> (1)..(1596)

<400> 77
atg tca gag tac ggc aca ctg cag gac cgc gta cgt tgc aag tca ctg      48
Met Ser Glu Tyr Gly Thr Leu Gln Asp Arg Val Arg Cys Lys Ser Leu
1               5                   10                  15
ctc aac aag gtt atg tcg gcc gag gag act atc cag ttc ttc aag ccg      96
Leu Asn Lys Val Met Ser Ala Glu Glu Thr Ile Gln Phe Phe Lys Pro
            20                  25                  30
ggc atg aac ctt ggc tgg tcc ggc ttc acc ccg gca ggt tat cct aag      144
Gly Met Asn Leu Gly Trp Ser Gly Phe Thr Pro Ala Gly Tyr Pro Lys
        35                  40                  45
gca gtg ccc atc gca ctg gca gat cac gtt gag aag aac agc ctg cag      192
Ala Val Pro Ile Ala Leu Ala Asp His Val Glu Lys Asn Ser Leu Gln
    50                  55                  60
ggc aag atg aag ttc aac ctc ttc atc ggc gcc tcc gtc ggg gcc gaa      240
Gly Lys Met Lys Phe Asn Leu Phe Ile Gly Ala Ser Val Gly Ala Glu
65                  70                  75                  80
acc gaa gac cgc tgg gcc act ctc gac atg atc gac cgt cgc tgg ccg      288
Thr Glu Asp Arg Trp Ala Thr Leu Asp Met Ile Asp Arg Arg Trp Pro
                85                  90                  95
tat cag acc ggt aag aac atc gcc gcc ggc atc aac gag ggc cgc atc      336
Tyr Gln Thr Gly Lys Asn Ile Ala Ala Gly Ile Asn Glu Gly Arg Ile
            100                 105                 110
cgg atg ggt gac aag cac ctc tcc ctc ttt gcc cag gat ctg ggc tac      384
Arg Met Gly Asp Lys His Leu Ser Leu Phe Ala Gln Asp Leu Gly Tyr
        115                 120                 125
ggc ttc tac acc aag gac agc gaa acc ggc aag ctt gac ctc gcc atc      432
Gly Phe Tyr Thr Lys Asp Ser Glu Thr Gly Lys Leu Asp Leu Ala Ile
    130                 135                 140
atc gag gtt tcc gcc atc acc gaa gac ggc gga ctg gtc ccc acc acc      480
Ile Glu Val Ser Ala Ile Thr Glu Asp Gly Gly Leu Val Pro Thr Thr
145                 150                 155                 160
tct tgc ggc gtt att ccc gag atc ctc atg atc tgc gac aag atc atc      528
Ser Cys Gly Val Ile Pro Glu Ile Leu Met Ile Cys Asp Lys Ile Ile
                165                 170                 175

gtg gaa gtg aac acc ggt cag ccc tcc ttc gag ggg atg cac gac atc 576
Val Glu Val Asn Thr Gly Gln Pro Ser Phe Glu Gly Met His Asp Ile
            180                 185                 190
gtc gtc tgc aac cac ccc ccg aag cgc cag att ctg ggc atc acc cat 624
Val Val Cys Asn His Pro Pro Lys Arg Gln Ile Leu Gly Ile Thr His
        195                 200                 205
gcc ggt gag cgg atc ggt aca acc tac gta ccg tgc gac ccg agc aag 672
Ala Gly Glu Arg Ile Gly Thr Thr Tyr Val Pro Cys Asp Pro Ser Lys
    210                 215                 220
atc atc gcc gtg gtt gag tcc aag tac cgt gac aag ggg cgt gcc ttc 720
Ile Ile Ala Val Val Glu Ser Lys Tyr Arg Asp Lys Gly Arg Ala Phe
225                 230                 235                 240
tcc gag cag gac gac acc tcc gag gcc att gcc aac aac atc atc gac 768
Ser Glu Gln Asp Asp Thr Ser Glu Ala Ile Ala Asn Asn Ile Ile Asp
                245                 250                 255
ttc ttc agc cat gaa gta aag atg ggt cgt ctg ccc aag aac ctg ctt 816
Phe Phe Ser His Glu Val Lys Met Gly Arg Leu Pro Lys Asn Leu Leu
            260                 265                 270
ccg ctc cag tca ggc gtc ggt tcc atc gcc aac gct gta atc ggc ggc 864
Pro Leu Gln Ser Gly Val Gly Ser Ile Ala Asn Ala Val Ile Gly Gly
        275                 280                 285
ctg gcc aag ggt cct ttc tcc aac ctg acc gtt tac acc gaa gtt ctc 912
Leu Ala Lys Gly Pro Phe Ser Asn Leu Thr Val Tyr Thr Glu Val Leu
    290                 295                 300
cag gac acc atg ctc gac ctg ttc gat tcg ggc aag ctg gat gcc gct 960
Gln Asp Thr Met Leu Asp Leu Phe Asp Ser Gly Lys Leu Asp Ala Ala
305                 310                 315                 320
tcc tcc tgc tcc ctc tct ctc tcc gag acc ccg ggc ttc ccc cgt ttc 1008
Ser Ser Cys Ser Leu Ser Leu Ser Glu Thr Pro Gly Phe Pro Arg Phe
                325                 330                 335
ttc gag aac tgg gag aag tac ttc gac aag atc acc ctg cgt ccg ctc 1056
Phe Glu Asn Trp Glu Lys Tyr Phe Asp Lys Ile Thr Leu Arg Pro Leu
            340                 345                 350
tcc atc tcc aac gca ccg gag ccg atc cgc cgt ctc ggg tgc atc gcc 1104
Ser Ile Ser Asn Ala Pro Glu Pro Ile Arg Arg Leu Gly Cys Ile Ala
        355                 360                 365
atg aac acc ccg gtc gag atc gac atc tat gcc cac gcc aac tcc acg 1152
Met Asn Thr Pro Val Glu Ile Asp Ile Tyr Ala His Ala Asn Ser Thr
    370                 375                 380
ctg gtc ggc ggc aca cgg atg atc aac ggc ctc ggc ggt tcc ggc gac 1200
Leu Val Gly Gly Thr Arg Met Ile Asn Gly Leu Gly Gly Ser Gly Asp
385                 390                 395                 400
ttc ctt cgc aac ggc ttc ttg aag atc atg cac act ccg tcg agc cgt 1248
Phe Leu Arg Asn Gly Phe Leu Lys Ile Met His Thr Pro Ser Ser Arg
                405                 410                 415
cca tcc aag acc gac ccg acc ggt atc tcc tgc gtg gtg ccg cac tgc 1296
Pro Ser Lys Thr Asp Pro Thr Gly Ile Ser Cys Val Val Pro His Cys
            420                 425                 430
tcc cac atc gac cac acc gag cac gat ctg gac tgc gtg gtc acc gag 1344
Ser His Ile Asp His Thr Glu His Asp Leu Asp Cys Val Val Thr Glu
        435                 440                 445
cag ggt ctg gcc gac ctc cgt ggt ctg gca ccg aag gat cgc gca aag 1392
Gln Gly Leu Ala Asp Leu Arg Gly Leu Ala Pro Lys Asp Arg Ala Lys
    450                 455                 460
cgg att atc gag aag tgc gcc cac ccg gac tac aag ccg att ctc agc 1440
Arg Ile Ile Glu Lys Cys Ala His Pro Asp Tyr Lys Pro Ile Leu Ser
465                 470                 475                 480
gag tac ttg gca atc gcc agt gcc gat tgc ctc aag cgc aag gtg ggc 1488
Glu Tyr Leu Ala Ile Ala Ser Ala Asp Cys Leu Lys Arg Lys Val Gly
                485                 490                 495
cac gag ccg caa ctg tgg gac cgc gcc ttc aag atg cac ctc aac ctg 1536
His Glu Pro Gln Leu Trp Asp Arg Ala Phe Lys Met His Leu Asn Leu
            500                 505                 510
gag cgc aac ggc acc atg aag atc aag aac tgg gac gtg aag gtc gat 1584
Glu Arg Asn Gly Thr Met Lys Ile Lys Asn Trp Asp Val Lys Val Asp
        515                 520                 525
ctc tgc gag taa 1596
Leu Cys Glu
    530

<210> 78

<211> 531
<212> PRT
<213> Geobacter sulfurreducens

<400> 78
Met Ser Glu Tyr Gly Thr Leu Gln Asp Arg Val Arg Cys Lys Ser Leu
1               5                   10                  15
Leu Asn Lys Val Met Ser Ala Glu Glu Thr Ile Gln Phe Phe Lys Pro
            20                  25                  30
Gly Met Asn Leu Gly Trp Ser Gly Phe Thr Pro Ala Gly Tyr Pro Lys
        35                  40                  45
Ala Val Pro Ile Ala Leu Ala Asp His Val Glu Lys Asn Ser Leu Gln
    50                  55                  60
Gly Lys Met Lys Phe Asn Leu Phe Ile Gly Ala Ser Val Gly Ala Glu
65                  70                  75                  80
Thr Glu Asp Arg Trp Ala Thr Leu Asp Met Ile Asp Arg Arg Trp Pro
                85                  90                  95
Tyr Gln Thr Gly Lys Asn Ile Ala Ala Gly Ile Asn Glu Gly Arg Ile
            100                 105                 110
Arg Met Gly Asp Lys His Leu Ser Leu Phe Ala Gln Asp Leu Gly Tyr
        115                 120                 125
Gly Phe Tyr Thr Lys Asp Ser Glu Thr Gly Lys Leu Asp Leu Ala Ile
    130                 135                 140
Ile Glu Val Ser Ala Ile Thr Glu Asp Gly Gly Leu Val Pro Thr Thr
145                 150                 155                 160
Ser Cys Gly Val Ile Pro Glu Ile Leu Met Ile Cys Asp Lys Ile Ile
                165                 170                 175
Val Glu Val Asn Thr Gly Gln Pro Ser Phe Glu Gly Met His Asp Ile
            180                 185                 190
Val Val Cys Asn His Pro Pro Lys Arg Gln Ile Leu Gly Ile Thr His
        195                 200                 205
Ala Gly Glu Arg Ile Gly Thr Thr Tyr Val Pro Cys Asp Pro Ser Lys
    210                 215                 220
Ile Ile Ala Val Val Glu Ser Lys Tyr Arg Asp Lys Gly Arg Ala Phe
225                 230                 235                 240
Ser Glu Gln Asp Asp Thr Ser Glu Ala Ile Ala Asn Asn Ile Ile Asp
                245                 250                 255
Phe Phe Ser His Glu Val Lys Met Gly Arg Leu Pro Lys Asn Leu Leu
            260                 265                 270
Pro Leu Gln Ser Gly Val Gly Ser Ile Ala Asn Ala Val Ile Gly Gly
        275                 280                 285
Leu Ala Lys Gly Pro Phe Ser Asn Leu Thr Val Tyr Thr Glu Val Leu
    290                 295                 300
Gln Asp Thr Met Leu Asp Leu Phe Asp Ser Gly Lys Leu Asp Ala Ala
305                 310                 315                 320
Ser Ser Cys Ser Leu Ser Leu Ser Glu Thr Pro Gly Phe Pro Arg Phe
                325                 330                 335
Phe Glu Asn Trp Glu Lys Tyr Phe Asp Lys Ile Thr Leu Arg Pro Leu
            340                 345                 350
Ser Ile Ser Asn Ala Pro Glu Pro Ile Arg Arg Leu Gly Cys Ile Ala
        355                 360                 365
Met Asn Thr Pro Val Glu Ile Asp Ile Tyr Ala His Ala Asn Ser Thr
    370                 375                 380
Leu Val Gly Gly Thr Arg Met Ile Asn Gly Leu Gly Gly Ser Gly Asp
385                 390                 395                 400
Phe Leu Arg Asn Gly Phe Leu Lys Ile Met His Thr Pro Ser Ser Arg
                405                 410                 415
Pro Ser Lys Thr Asp Pro Thr Gly Ile Ser Cys Val Val Pro His Cys
            420                 425                 430
Ser His Ile Asp His Thr Glu His Asp Leu Asp Cys Val Val Thr Glu
        435                 440                 445
Gln Gly Leu Ala Asp Leu Arg Gly Leu Ala Pro Lys Asp Arg Ala Lys
    450                 455                 460
Arg Ile Ile Glu Lys Cys Ala His Pro Asp Tyr Lys Pro Ile Leu Ser
465                 470                 475                 480
Glu Tyr Leu Ala Ile Ala Ser Ala Asp Cys Leu Lys Arg Lys Val Gly
                485                 490                 495
His Glu Pro Gln Leu Trp Asp Arg Ala Phe Lys Met His Leu Asn Leu
            500                 505                 510
Glu Arg Asn Gly Thr Met Lys Ile Lys Asn Trp Asp Val Lys Val Asp
        515                 520                 525
Leu Cys Glu

    530

<210> 79
<211> 1515
<212> DNA
<213> Bacillus cereus

<220>
<221> CDS
<222> (1)..(1515)

<400> 79
atg gag aat aat ttg gat aga att agg gat gaa cgt ctg aaa gat cgc 48
Met Glu Asn Asn Leu Asp Arg Ile Arg Asp Glu Arg Leu Lys Asp Arg
1               5                   10                  15
gta gtt aca cct gaa gaa gca gct tca tgg att gaa agt gga atg act 96
Val Val Thr Pro Glu Glu Ala Ala Ser Trp Ile Glu Ser Gly Met Thr
            20                  25                  30
tta ggc tta agt ggg ttt aca cgt gca ggt gat gta aaa gca gtc cca 144
Leu Gly Leu Ser Gly Phe Thr Arg Ala Gly Asp Val Lys Ala Val Pro
        35                  40                  45
ttt gcg ctt gta aac cga gtt aag aat gat aca tct ttt aaa gta aat 192
Phe Ala Leu Val Asn Arg Val Lys Asn Asp Thr Ser Phe Lys Val Asn
    50                  55                  60
gtt tac act ggt gcc tct tta ggt tct gat gta gat aaa tta ttt gcg 240
Val Tyr Thr Gly Ala Ser Leu Gly Ser Asp Val Asp Lys Leu Phe Ala
65                  70                  75                  80
gaa gca gga att tta ggg aaa aga ttg cct ttc caa gct gat gcg act 288
Glu Ala Gly Ile Leu Gly Lys Arg Leu Pro Phe Gln Ala Asp Ala Thr
                85                  90                  95
atg cga aaa gga att aat aac gga gat ttt tta ttt gtg gac cag cac 336
Met Arg Lys Gly Ile Asn Asn Gly Asp Phe Leu Phe Val Asp Gln His
            100                 105                 110
tta tct cat aca gca gag tta ctt cgc gct gat gtt atg gat gta gat 384
Leu Ser His Thr Ala Glu Leu Leu Arg Ala Asp Val Met Asp Val Asp
        115                 120                 125
ttt gct att ttg gaa gcg gtt gca att act gaa gat ggc atg att att 432
Phe Ala Ile Leu Glu Ala Val Ala Ile Thr Glu Asp Gly Met Ile Ile
    130                 135                 140
ccg aca act tca ata gga aat tct tta gcg ttt tct tta aat gct aag 480
Pro Thr Thr Ser Ile Gly Asn Ser Leu Ala Phe Ser Leu Asn Ala Lys
145                 150                 155                 160
tcc att att att gaa atg aat atg gct caa tcc acg cag tta gaa gga 528
Ser Ile Ile Ile Glu Met Asn Met Ala Gln Ser Thr Gln Leu Glu Gly
                165                 170                 175
ctg cat gat tta tat gaa cca ggt aag caa gga gaa aga ctt cca att 576
Leu His Asp Leu Tyr Glu Pro Gly Lys Gln Gly Glu Arg Leu Pro Ile
            180                 185                 190
ccg att gtg aaa acg gat gat cga att gga acg att ggc att cca atc 624
Pro Ile Val Lys Thr Asp Asp Arg Ile Gly Thr Ile Gly Ile Pro Ile
        195                 200                 205
gat gtt gaa aag gtg aaa ggg att gtg ttt acg aac caa tta gac tca 672
Asp Val Glu Lys Val Lys Gly Ile Val Phe Thr Asn Gln Leu Asp Ser
    210                 215                 220
ccc tcg aca att gtt cct ccg gat gaa gaa act gtt att atg gca cag 720
Pro Ser Thr Ile Val Pro Pro Asp Glu Glu Thr Val Ile Met Ala Gln
225                 230                 235                 240
cat tta ata gag ttc ctt cga aaa gaa gtc gaa gta ggt cga tta aca 768
His Leu Ile Glu Phe Leu Arg Lys Glu Val Glu Val Gly Arg Leu Thr
                245                 250                 255
aat cgt tta gcg ccg cta caa tca gga att ggc tca gtg gct aat gca 816
Asn Arg Leu Ala Pro Leu Gln Ser Gly Ile Gly Ser Val Ala Asn Ala
            260                 265                 270
gtt ctt cat gga atg tta gat tcg gag ttt gaa gat tta gaa gtg tat 864
Val Leu His Gly Met Leu Asp Ser Glu Phe Glu Asp Leu Glu Val Tyr
        275                 280                 285
tct gaa gtt tta caa gat gcg gtc ttt gat ctt atg gat gct gga aaa 912
Ser Glu Val Leu Gln Asp Ala Val Phe Asp Leu Met Asp Ala Gly Lys
    290                 295                 300
gtc aat ttc gct tca tgc tgc tca atc aca ctt tct gag gag aaa atg 960
Val Asn Phe Ala Ser Cys Cys Ser Ile Thr Leu Ser Glu Glu Lys Met
305                 310                 315                 320

caa aaa gta ttt tcc aac ttt gaa aaa tat cgt gac aaa tta atg atg 1008
Gln Lys Val Phe Ser Asn Phe Glu Lys Tyr Arg Asp Lys Leu Met Met
                325                 330                 335
cgc ccg caa gag att tct aat cat cct gaa atc att cgt cgc ctc gga 1056
Arg Pro Gln Glu Ile Ser Asn His Pro Glu Ile Ile Arg Arg Leu Gly
            340                 345                 350
tta atc tcg att aat act gct tta gaa tta gat ata tac gga aat gtg 1104
Leu Ile Ser Ile Asn Thr Ala Leu Glu Leu Asp Ile Tyr Gly Asn Val
        355                 360                 365
aac tct act cac gtt tta ggc aca aaa atg atg aac ggt att ggt ggt 1152
Asn Ser Thr His Val Leu Gly Thr Lys Met Met Asn Gly Ile Gly Gly
    370                 375                 380
tct ggt gat ttt gcg aga aat gct cgc cta gct atc ttt gtt acg aaa 1200
Ser Gly Asp Phe Ala Arg Asn Ala Arg Leu Ala Ile Phe Val Thr Lys
385                 390                 395                 400
tcg att gcg aaa ggt ggt aac att tca agt atc gtt cct ttt gtt tca 1248
Ser Ile Ala Lys Gly Gly Asn Ile Ser Ser Ile Val Pro Phe Val Ser
                405                 410                 415
cat gtg gac cat acg gaa cac gat gta gat gtt atc gtc act gaa caa 1296
His Val Asp His Thr Glu His Asp Val Asp Val Ile Val Thr Glu Gln
            420                 425                 430
ggg tat gtt gat cta aga gga ctt gcc cca aga gaa aga gtg gaa tta 1344
Gly Tyr Val Asp Leu Arg Gly Leu Ala Pro Arg Glu Arg Val Glu Leu
        435                 440                 445
att ata gag aat tgc gca cat cca atg tat cgt gat cag cta aga gct 1392
Ile Ile Glu Asn Cys Ala His Pro Met Tyr Arg Asp Gln Leu Arg Ala
    450                 455                 460
tat tac gaa gaa gct aaa acg aga ggt gga caa act cct cat gtt tta 1440
Tyr Tyr Glu Glu Ala Lys Thr Arg Gly Gly Gln Thr Pro His Val Leu
465                 470                 475                 480
gaa aaa gct ttt tct tgg cat acg aat tat gct aaa aat gga aca atg 1488
Glu Lys Ala Phe Ser Trp His Thr Asn Tyr Ala Lys Asn Gly Thr Met
                485                 490                 495
ctt gaa gcg gta gta gaa act gta tag 1515
Leu Glu Ala Val Val Glu Thr Val
            500

<210> 80
<211> 504
<212> PRT
<213> Bacillus cereus

<400> 80
Met Glu Asn Asn Leu Asp Arg Ile Arg Asp Glu Arg Leu Lys Asp Arg
1               5                   10                  15
Val Val Thr Pro Glu Glu Ala Ala Ser Trp Ile Glu Ser Gly Met Thr
            20                  25                  30
Leu Gly Leu Ser Gly Phe Thr Arg Ala Gly Asp Val Lys Ala Val Pro
        35                  40                  45
Phe Ala Leu Val Asn Arg Val Lys Asn Asp Thr Ser Phe Lys Val Asn
    50                  55                  60
Val Tyr Thr Gly Ala Ser Leu Gly Ser Asp Val Asp Lys Leu Phe Ala
65                  70                  75                  80
Glu Ala Gly Ile Leu Gly Lys Arg Leu Pro Phe Gln Ala Asp Ala Thr
                85                  90                  95
Met Arg Lys Gly Ile Asn Asn Gly Asp Phe Leu Phe Val Asp Gln His
            100                 105                 110
Leu Ser His Thr Ala Glu Leu Leu Arg Ala Asp Val Met Asp Val Asp
        115                 120                 125
Phe Ala Ile Leu Glu Ala Val Ala Ile Thr Glu Asp Gly Met Ile Ile
    130                 135                 140
Pro Thr Thr Ser Ile Gly Asn Ser Leu Ala Phe Ser Leu Asn Ala Lys
145                 150                 155                 160
Ser Ile Ile Ile Glu Met Asn Met Ala Gln Ser Thr Gln Leu Glu Gly
                165                 170                 175
Leu His Asp Leu Tyr Glu Pro Gly Lys Gln Gly Glu Arg Leu Pro Ile
            180                 185                 190
Pro Ile Val Lys Thr Asp Asp Arg Ile Gly Thr Ile Gly Ile Pro Ile
        195                 200                 205
Asp Val Glu Lys Val Lys Gly Ile Val Phe Thr Asn Gln Leu Asp Ser
    210                 215                 220

Pro Ser Thr Ile Val Pro Pro Asp Glu Glu Thr Val Ile Met Ala Gln
225                 230                 235                 240
His Leu Ile Glu Phe Leu Arg Lys Glu Val Glu Val Gly Arg Leu Thr
                245                 250                 255
Asn Arg Leu Ala Pro Leu Gln Ser Gly Ile Gly Ser Val Ala Asn Ala
            260                 265                 270
Val Leu His Gly Met Leu Asp Ser Glu Phe Glu Asp Leu Glu Val Tyr
        275                 280                 285
Ser Glu Val Leu Gln Asp Ala Val Phe Asp Leu Met Asp Ala Gly Lys
    290                 295                 300
Val Asn Phe Ala Ser Cys Cys Ser Ile Thr Leu Ser Glu Glu Lys Met
305                 310                 315                 320
Gln Lys Val Phe Ser Asn Phe Glu Lys Tyr Arg Asp Lys Leu Met Met
                325                 330                 335
Arg Pro Gln Glu Ile Ser Asn His Pro Glu Ile Ile Arg Arg Leu Gly
            340                 345                 350
Leu Ile Ser Ile Asn Thr Ala Leu Glu Leu Asp Ile Tyr Gly Asn Val
        355                 360                 365
Asn Ser Thr His Val Leu Gly Thr Lys Met Met Asn Gly Ile Gly Gly
    370                 375                 380
Ser Gly Asp Phe Ala Arg Asn Ala Arg Leu Ala Ile Phe Val Thr Lys
385                 390                 395                 400
Ser Ile Ala Lys Gly Gly Asn Ile Ser Ser Ile Val Pro Phe Val Ser
                405                 410                 415
His Val Asp His Thr Glu His Asp Val Asp Val Ile Val Thr Glu Gln
            420                 425                 430
Gly Tyr Val Asp Leu Arg Gly Leu Ala Pro Arg Glu Arg Val Glu Leu
        435                 440                 445
Ile Ile Glu Asn Cys Ala His Pro Met Tyr Arg Asp Gln Leu Arg Ala
    450                 455                 460
Tyr Tyr Glu Glu Ala Lys Thr Arg Gly Gly Gln Thr Pro His Val Leu
465                 470                 475                 480
Glu Lys Ala Phe Ser Trp His Thr Asn Tyr Ala Lys Asn Gly Thr Met
                485                 490                 495
Leu Glu Ala Val Val Glu Thr Val
            500

<210>       81
<211>       2577
<212>       DNA
<213>       Saccharomyces cerevisiae

<220>
<221>  CDS
<222>  (1)..(2577)

<400>       81
atg tcg tcc tta tcc act tca ttt gat tca tcg tca gat tta cca agg       48
Met Ser Ser Leu Ser Thr Ser Phe Asp Ser Ser Ser Asp Leu Pro Arg
1               5                   10                  15
tca aaa tcc gtt gac aat acg gcg gct tcc atg aag aca ggc aag tac       96
Ser Lys Ser Val Asp Asn Thr Ala Ala Ser Met Lys Thr Gly Lys Tyr
            20                  25                  30
cca aaa tta gag aac tat agg cag tat tct gat gca caa cca ata cgt      144
Pro Lys Leu Glu Asn Tyr Arg Gln Tyr Ser Asp Ala Gln Pro Ile Arg
        35                  40                  45
cac gaa gcg ctt gca ttg aaa gtg gac gaa acg aaa gat tct aga cac      192
His Glu Ala Leu Ala Leu Lys Val Asp Glu Thr Lys Asp Ser Arg His
    50                  55                  60
aaa ttt agt tcc tct aac ggg gag aat agt gga gtg gaa aat gga ggc      240
Lys Phe Ser Ser Ser Asn Gly Glu Asn Ser Gly Val Glu Asn Gly Gly
65                  70                  75                  80
tat gtg gag aaa acg aat ata tcc aca agt ggc cgc atg gat ttt gaa      288
Tyr Val Glu Lys Thr Asn Ile Ser Thr Ser Gly Arg Met Asp Phe Glu
                85                  90                  95
ggt gaa gca gaa gca gaa gca gtg aaa cgt tac cag tta aga tct ttc      336
Gly Glu Ala Glu Ala Glu Ala Val Lys Arg Tyr Gln Leu Arg Ser Phe
            100                 105                 110
gcg ctt tta agt tct aat gcg agg cca tcc agg cta gca aag tct gaa      384
Ala Leu Leu Ser Ser Asn Ala Arg Pro Ser Arg Leu Ala Lys Ser Glu

        115                 120                 125
act cac caa aag cag att cat gta gag agc atc gct ccc tct ttg cca 432
Thr His Gln Lys Gln Ile His Val Glu Ser Ile Ala Pro Ser Leu Pro
    130                 135                 140
aag aat gcg gct tta gag aga ggc cat gat acg gcg tta cct gcc ggt 480
Lys Asn Ala Ala Leu Glu Arg Gly His Asp Thr Ala Leu Pro Ala Gly
145                 150                 155                 160
acg tca agt aac aga tgc aac tta gaa gcc tcc tcc tcg gca aga act 528
Thr Ser Ser Asn Arg Cys Asn Leu Glu Ala Ser Ser Ser Ala Arg Thr
                165                 170                 175
ttt aca agc gct cgt aag gct tcg cta gtt agc gca atc ttt gaa aca 576
Phe Thr Ser Ala Arg Lys Ala Ser Leu Val Ser Ala Ile Phe Glu Thr
            180                 185                 190
tcg gct gaa tca gag cat ggt acg cat ccc aaa cag gcg aaa ctt aag 624
Ser Ala Glu Ser Glu His Gly Thr His Pro Lys Gln Ala Lys Leu Lys
        195                 200                 205
aga aga acc tat tct aca ata tcg aca cat tca tca gtc aat cct aca 672
Arg Arg Thr Tyr Ser Thr Ile Ser Thr His Ser Ser Val Asn Pro Thr
    210                 215                 220
acg ttg ttg aca aga act gca tcg caa aag tcc gat atg gga aat gac 720
Thr Leu Leu Thr Arg Thr Ala Ser Gln Lys Ser Asp Met Gly Asn Asp
225                 230                 235                 240
aca cga aga att aaa ccg tta cgt atg gat agc agg gta tct ttc cat 768
Thr Arg Arg Ile Lys Pro Leu Arg Met Asp Ser Arg Val Ser Phe His
                245                 250                 255
agt gag ata tcg caa gca tcc agg gat tct caa gaa aca gaa gaa gac 816
Ser Glu Ile Ser Gln Ala Ser Arg Asp Ser Gln Glu Thr Glu Glu Asp
            260                 265                 270
gtt tgt ttt cca atg ttt cga cta tta cat aca aga gtt aat ggt gtt 864
Val Cys Phe Pro Met Phe Arg Leu Leu His Thr Arg Val Asn Gly Val
        275                 280                 285
gat ttt gat gag ttg gaa gaa tat gcc cag att tca aat gct gaa aga 912
Asp Phe Asp Glu Leu Glu Glu Tyr Ala Gln Ile Ser Asn Ala Glu Arg
    290                 295                 300
aat ctg tct ttg gcg aac cat caa agg cac agt gaa cga aca tac aat 960
Asn Leu Ser Leu Ala Asn His Gln Arg His Ser Glu Arg Thr Tyr Asn
305                 310                 315                 320
cat acc gac caa gat act gga ttt act aac tct gct tcc act tca gga 1008
His Thr Asp Gln Asp Thr Gly Phe Thr Asn Ser Ala Ser Thr Ser Gly
                325                 330                 335
tcc tca gca gct ctg aaa tat aca cct gaa att tca cgg act ctt gaa 1056
Ser Ser Ala Ala Leu Lys Tyr Thr Pro Glu Ile Ser Arg Thr Leu Glu
            340                 345                 350
aaa aat tgc agc gtt aat gag atg tac gta tct gaa aac aat gaa tct 1104
Lys Asn Cys Ser Val Asn Glu Met Tyr Val Ser Glu Asn Asn Glu Ser
        355                 360                 365
gtg agg gaa gat gat aaa cct gat cta cac cct gac gta aca ttt ggt 1152
Val Arg Glu Asp Asp Lys Pro Asp Leu His Pro Asp Val Thr Phe Gly
    370                 375                 380
aga aat aag att gaa ggt gaa aaa gaa gga aat gat tcg agt tat agc 1200
Arg Asn Lys Ile Glu Gly Glu Lys Glu Gly Asn Asp Ser Ser Tyr Ser
385                 390                 395                 400
cgg gcg tat tat acc ctc caa aat aca gaa tat caa att cct agt aga 1248
Arg Ala Tyr Tyr Thr Leu Gln Asn Thr Glu Tyr Gln Ile Pro Ser Arg
                405                 410                 415
ttt tcg ttc ttc cgc tct gaa tct gat gaa act gtg cat gct agt gat 1296
Phe Ser Phe Phe Arg Ser Glu Ser Asp Glu Thr Val His Ala Ser Asp
            420                 425                 430
atc cct tct ttg ata tcc gag gga cag acg ttt tat gag tta ttc aag 1344
Ile Pro Ser Leu Ile Ser Glu Gly Gln Thr Phe Tyr Glu Leu Phe Lys
        435                 440                 445
gga ggc gat cca acg tgg tgg ttg gat tgc agt tgt cca act gat gat 1392
Gly Gly Asp Pro Thr Trp Trp Leu Asp Cys Ser Cys Pro Thr Asp Asp
    450                 455                 460
gaa atg cgt tgc ata gca aaa aca ttt gga atc cat cct ttg acc gcg 1440
Glu Met Arg Cys Ile Ala Lys Thr Phe Gly Ile His Pro Leu Thr Ala
465                 470                 475                 480
gaa gat atc agg atg caa gaa acg cgt gaa aag gta gag ctt ttc aaa 1488
Glu Asp Ile Arg Met Gln Glu Thr Arg Glu Lys Val Glu Leu Phe Lys
                485                 490                 495
tcc tat tac ttt gtc tgt ttc cat aca ttt gaa aat gat aaa gaa tcg 1536
Ser Tyr Tyr Phe Val Cys Phe His Thr Phe Glu Asn Asp Lys Glu Ser

            500                 505                 510
gag aat tat ctt gaa cct ata aat gtt tac atc gtt gtt ttt agg tca 1584
Glu Asn Tyr Leu Glu Pro Ile Asn Val Tyr Ile Val Val Phe Arg Ser
        515                 520                 525
ggt gtc ttg act ttt cat ttt gac cca ata tca cac tgt gcc aat gta 1632
Gly Val Leu Thr Phe His Phe Asp Pro Ile Ser His Cys Ala Asn Val
    530                 535                 540
aga aga cgt gta aga caa tta cgt gac tat gtc agt gtc aat tca gat 1680
Arg Arg Arg Val Arg Gln Leu Arg Asp Tyr Val Ser Val Asn Ser Asp
545                 550                 555                 560
tgg tta tgt tat gct tta atc gat gat atc aca gat agt ttt gct ccg 1728
Trp Leu Cys Tyr Ala Leu Ile Asp Asp Ile Thr Asp Ser Phe Ala Pro
                565                 570                 575
gtt att caa tcc atc gag tat gaa gct gat tcg att gat gat tca gtg 1776
Val Ile Gln Ser Ile Glu Tyr Glu Ala Asp Ser Ile Asp Asp Ser Val
            580                 585                 590
ttt atg acc cgt gat atg gat ttt gca gcc atg tta caa agg att ggt 1824
Phe Met Thr Arg Asp Met Asp Phe Ala Ala Met Leu Gln Arg Ile Gly
        595                 600                 605
gaa agt agg cgt aag aca atg acg ctg atg aga ctc ttg agc ggt aaa 1872
Glu Ser Arg Arg Lys Thr Met Thr Leu Met Arg Leu Leu Ser Gly Lys
    610                 615                 620
gca gat gtt atc aaa atg ttc gcc aaa aga tgt caa gat gaa act aat 1920
Ala Asp Val Ile Lys Met Phe Ala Lys Arg Cys Gln Asp Glu Thr Asn
625                 630                 635                 640
ggt att ggg cca gtg ctt aaa tct caa act aac atg gtg aac tta caa 1968
Gly Ile Gly Pro Val Leu Lys Ser Gln Thr Asn Met Val Asn Leu Gln
                645                 650                 655
gcg gaa caa gag aat gtg aac cag aat aat agt aat aac caa ata agc 2016
Ala Glu Gln Glu Asn Val Asn Gln Asn Asn Ser Asn Asn Gln Ile Ser
            660                 665                 670
tta tca aat agc tat atg caa aca aca tca caa cca agg gga gat att 2064
Leu Ser Asn Ser Tyr Met Gln Thr Thr Ser Gln Pro Arg Gly Asp Ile
        675                 680                 685
gct tta tac tta ggt gat att cag gat cat ttg ttg acg atg ttt caa 2112
Ala Leu Tyr Leu Gly Asp Ile Gln Asp His Leu Leu Thr Met Phe Gln
    690                 695                 700
aat tta ttg gcg tac gaa aag att ttt tct aga tca cat gca aac tac 2160
Asn Leu Leu Ala Tyr Glu Lys Ile Phe Ser Arg Ser His Ala Asn Tyr
705                 710                 715                 720
ctg gcc caa tta caa gtg gaa tca ttt aac tct aat aat aaa gtt aca 2208
Leu Ala Gln Leu Gln Val Glu Ser Phe Asn Ser Asn Asn Lys Val Thr
                725                 730                 735
gaa atg ttg ggt aaa gtt act atg tta ggt aca atg tta gtt cca tta 2256
Glu Met Leu Gly Lys Val Thr Met Leu Gly Thr Met Leu Val Pro Leu
            740                 745                 750
aat gtt att act ggt ctg ttc ggt atg aat gta aaa gtt cca gga aga 2304
Asn Val Ile Thr Gly Leu Phe Gly Met Asn Val Lys Val Pro Gly Arg
        755                 760                 765
aat gga agt att gcg tgg tgg tat gga att ttg ggg gta ctg ctt tta 2352
Asn Gly Ser Ile Ala Trp Trp Tyr Gly Ile Leu Gly Val Leu Leu Leu
    770                 775                 780
cta gct gtc ata agc tgg ttt tta gct agc tat tgg atc aaa aaa ata 2400
Leu Ala Val Ile Ser Trp Phe Leu Ala Ser Tyr Trp Ile Lys Lys Ile
785                 790                 795                 800
gac cca cct gca acg ctc aac gaa gcc gca ggt agt ggg gcc aaa tca 2448
Asp Pro Pro Ala Thr Leu Asn Glu Ala Ala Gly Ser Gly Ala Lys Ser
                805                 810                 815
gtc ata tct agt ttt tta ccg aaa aga gat aaa aga ttt aac gat gat 2496
Val Ile Ser Ser Phe Leu Pro Lys Arg Asp Lys Arg Phe Asn Asp Asp
            820                 825                 830
tct aaa aat ggc aat gcg aga gtt ggc gtt cga agg aaa tca act gtc 2544
Ser Lys Asn Gly Asn Ala Arg Val Gly Val Arg Arg Lys Ser Thr Val
        835                 840                 845
agt cta ccc agt aga tat agc cgt tac aat taa 2577
Ser Leu Pro Ser Arg Tyr Ser Arg Tyr Asn
    850                 855

<210> 82
<211> 858
<212> PRT

<213>        Saccharomyces cerevisiae

<400>        82
Met Ser Ser Leu Ser Thr Ser Phe Asp Ser Ser Ser Asp Leu Pro Arg
1               5                   10                  15
Ser Lys Ser Val Asp Asn Thr Ala Ala Ser Met Lys Thr Gly Lys Tyr
            20                  25                  30
Pro Lys Leu Glu Asn Tyr Arg Gln Tyr Ser Asp Ala Gln Pro Ile Arg
        35                  40                  45
His Glu Ala Leu Ala Leu Lys Val Asp Glu Thr Lys Asp Ser Arg His
    50                  55                  60
Lys Phe Ser Ser Ser Asn Gly Glu Asn Ser Gly Val Glu Asn Gly Gly
65                  70                  75                  80
Tyr Val Glu Lys Thr Asn Ile Ser Thr Ser Gly Arg Met Asp Phe Glu
                85                  90                  95
Gly Glu Ala Glu Ala Glu Ala Val Lys Arg Tyr Gln Leu Arg Ser Phe
            100                 105                 110
Ala Leu Leu Ser Ser Asn Ala Arg Pro Ser Arg Leu Ala Lys Ser Glu
        115                 120                 125
Thr His Gln Lys Gln Ile His Val Glu Ser Ile Ala Pro Ser Leu Pro
    130                 135                 140
Lys Asn Ala Ala Leu Glu Arg Gly His Asp Thr Ala Leu Pro Ala Gly
145                 150                 155                 160
Thr Ser Ser Asn Arg Cys Asn Leu Glu Ala Ser Ser Ser Ala Arg Thr
                165                 170                 175
Phe Thr Ser Ala Arg Lys Ala Ser Leu Val Ser Ala Ile Phe Glu Thr
            180                 185                 190
Ser Ala Glu Ser Glu His Gly Thr His Pro Lys Gln Ala Lys Leu Lys
        195                 200                 205
Arg Arg Thr Tyr Ser Thr Ile Ser Thr His Ser Ser Val Asn Pro Thr
    210                 215                 220
Thr Leu Leu Thr Arg Thr Ala Ser Gln Lys Ser Asp Met Gly Asn Asp
225                 230                 235                 240
Thr Arg Arg Ile Lys Pro Leu Arg Met Asp Ser Arg Val Ser Phe His
                245                 250                 255
Ser Glu Ile Ser Gln Ala Ser Arg Asp Ser Gln Glu Thr Glu Glu Asp
            260                 265                 270
Val Cys Phe Pro Met Phe Arg Leu Leu His Thr Arg Val Asn Gly Val
        275                 280                 285
Asp Phe Asp Glu Leu Glu Glu Tyr Ala Gln Ile Ser Asn Ala Glu Arg
    290                 295                 300
Asn Leu Ser Leu Ala Asn His Gln Arg His Ser Glu Arg Thr Tyr Asn
305                 310                 315                 320
His Thr Asp Gln Asp Thr Gly Phe Thr Asn Ser Ala Ser Thr Ser Gly
                325                 330                 335
Ser Ser Ala Ala Leu Lys Tyr Thr Pro Glu Ile Ser Arg Thr Leu Glu
            340                 345                 350
Lys Asn Cys Ser Val Asn Glu Met Tyr Val Ser Glu Asn Asn Glu Ser
        355                 360                 365
Val Arg Glu Asp Asp Lys Pro Asp Leu His Pro Asp Val Thr Phe Gly
    370                 375                 380
Arg Asn Lys Ile Glu Gly Glu Lys Glu Gly Asn Asp Ser Ser Tyr Ser
385                 390                 395                 400
Arg Ala Tyr Tyr Thr Leu Gln Asn Thr Glu Tyr Gln Ile Pro Ser Arg
                405                 410                 415
Phe Ser Phe Phe Arg Ser Glu Ser Asp Glu Thr Val His Ala Ser Asp
            420                 425                 430
Ile Pro Ser Leu Ile Ser Glu Gly Gln Thr Phe Tyr Glu Leu Phe Lys
        435                 440                 445
Gly Gly Asp Pro Thr Trp Trp Leu Asp Cys Ser Cys Pro Thr Asp Asp
    450                 455                 460
Glu Met Arg Cys Ile Ala Lys Thr Phe Gly Ile His Pro Leu Thr Ala
465                 470                 475                 480
Glu Asp Ile Arg Met Gln Glu Thr Arg Glu Lys Val Glu Leu Phe Lys
                485                 490                 495
Ser Tyr Tyr Phe Val Cys Phe His Thr Phe Glu Asn Asp Lys Glu Ser
            500                 505                 510
Glu Asn Tyr Leu Glu Pro Ile Asn Val Tyr Ile Val Val Phe Arg Ser
        515                 520                 525
Gly Val Leu Thr Phe His Phe Asp Pro Ile Ser His Cys Ala Asn Val
    530                 535                 540
Arg Arg Arg Val Arg Gln Leu Arg Asp Tyr Val Ser Val Asn Ser Asp

545                 550                 555                 560
Trp Leu Cys Tyr Ala Leu Ile Asp Asp Ile Thr Asp Ser Phe Ala Pro
                565                 570                 575
Val Ile Gln Ser Ile Glu Tyr Glu Ala Asp Ser Ile Asp Asp Ser Val
            580                 585                 590
Phe Met Thr Arg Asp Met Asp Phe Ala Ala Met Leu Gln Arg Ile Gly
        595                 600                 605
Glu Ser Arg Arg Lys Thr Met Thr Leu Met Arg Leu Leu Ser Gly Lys
    610                 615                 620
Ala Asp Val Ile Lys Met Phe Ala Lys Arg Cys Gln Asp Glu Thr Asn
625                 630                 635                 640
Gly Ile Gly Pro Val Leu Lys Ser Gln Thr Asn Met Val Asn Leu Gln
                645                 650                 655
Ala Glu Gln Glu Asn Val Asn Gln Asn Asn Ser Asn Asn Gln Ile Ser
            660                 665                 670
Leu Ser Asn Ser Tyr Met Gln Thr Thr Ser Gln Pro Arg Gly Asp Ile
        675                 680                 685
Ala Leu Tyr Leu Gly Asp Ile Gln Asp His Leu Leu Thr Met Phe Gln
    690                 695                 700
Asn Leu Leu Ala Tyr Glu Lys Ile Phe Ser Arg Ser His Ala Asn Tyr
705                 710                 715                 720
Leu Ala Gln Leu Gln Val Glu Ser Phe Asn Ser Asn Asn Lys Val Thr
                725                 730                 735
Glu Met Leu Gly Lys Val Thr Met Leu Gly Thr Met Leu Val Pro Leu
            740                 745                 750
Asn Val Ile Thr Gly Leu Phe Gly Met Asn Val Lys Val Pro Gly Arg
        755                 760                 765
Asn Gly Ser Ile Ala Trp Trp Tyr Gly Ile Leu Gly Val Leu Leu Leu
    770                 775                 780
Leu Ala Val Ile Ser Trp Phe Leu Ala Ser Tyr Trp Ile Lys Lys Ile
785                 790                 795                 800
Asp Pro Pro Ala Thr Leu Asn Glu Ala Ala Gly Ser Gly Ala Lys Ser
                805                 810                 815
Val Ile Ser Ser Phe Leu Pro Lys Arg Asp Lys Arg Phe Asn Asp Asp
            820                 825                 830
Ser Lys Asn Gly Asn Ala Arg Val Gly Val Arg Arg Lys Ser Thr Val
        835                 840                 845
Ser Leu Pro Ser Arg Tyr Ser Arg Tyr Asn
    850                 855

<210>       83
<211>       25
<212>       DNA
<213>       Primer

<400>       83
atgtcgtcct tatccacttc atttg                                          25

<210>       84
<211>       26
<212>       DNA
<213>       Primer

<400>       84
ttaattgtaa cggctatatc tactgg                                         26

<210>       85
<211>       954
<212>       DNA
<213>       Methanococcus jannaschii

<220>
<221>  CDS
<222>  (1)..(954)

<400>       85
atg att acg gta att gct ata gct aaa gat ggc agt att gtc gag cca      48
Met Ile Thr Val Ile Ala Ile Ala Lys Asp Gly Ser Ile Val Glu Pro
1               5                   10                  15

aaa ctt gat gaa att agc ttt gaa gat tat aga ctt att tgg att gat 96
Lys Leu Asp Glu Ile Ser Phe Glu Asp Tyr Arg Leu Ile Trp Ile Asp
            20                  25                  30
tgt tat gac cca aaa gat gaa gag cta tat aaa ctc tct aaa aaa att 144
Cys Tyr Asp Pro Lys Asp Glu Glu Leu Tyr Lys Leu Ser Lys Lys Ile
        35                  40                  45
ggt att tct gtc tct gat tta caa att ggt tta gat gag cag gaa att 192
Gly Ile Ser Val Ser Asp Leu Gln Ile Gly Leu Asp Glu Gln Glu Ile
    50                  55                  60
cca agg gta gag gaa gat gag gac ttt tac tta att att tac aaa gct 240
Pro Arg Val Glu Glu Asp Glu Asp Phe Tyr Leu Ile Ile Tyr Lys Ala
65                  70                  75                  80
cca tta ttt gaa gag gat att aca aca aca tcc tta ggt atc tat att 288
Pro Leu Phe Glu Glu Asp Ile Thr Thr Thr Ser Leu Gly Ile Tyr Ile
                85                  90                  95
aag aat aac ctc cta cta aca atc cac tcg gat aaa ata aag gct att 336
Lys Asn Asn Leu Leu Leu Thr Ile His Ser Asp Lys Ile Lys Ala Ile
            100                 105                 110
gga aga ttg cat aaa tta ata tca aca aaa aaa cca aga att gta ttt 384
Gly Arg Leu His Lys Leu Ile Ser Thr Lys Lys Pro Arg Ile Val Phe
        115                 120                 125
gaa aga gga att ggc ttt tta tta tac cat ata tta aat gag att aca 432
Glu Arg Gly Ile Gly Phe Leu Leu Tyr His Ile Leu Asn Glu Ile Thr
    130                 135                 140
agg agt tat tca aga att tta atg aat tta gaa gat gag ttg gag gag 480
Arg Ser Tyr Ser Arg Ile Leu Met Asn Leu Glu Asp Glu Leu Glu Glu
145                 150                 155                 160
ttg gag gat aaa tta tta gct ggc tat gac aga gag gtt atg gaa aaa 528
Leu Glu Asp Lys Leu Leu Ala Gly Tyr Asp Arg Glu Val Met Glu Lys
                165                 170                 175
att tta gga tta aga aaa act ttg gtt tat ttt cat aaa tct tta ata 576
Ile Leu Gly Leu Arg Lys Thr Leu Val Tyr Phe His Lys Ser Leu Ile
            180                 185                 190
gct aat agg gat gtc tta gtt tta tta aag aga aag tat ctt cca ata 624
Ala Asn Arg Asp Val Leu Val Leu Leu Lys Arg Lys Tyr Leu Pro Ile
        195                 200                 205
acc aca aaa gaa gac aga gag aac ttt gaa gac ctt tac tac gac act 672
Thr Thr Lys Glu Asp Arg Glu Asn Phe Glu Asp Leu Tyr Tyr Asp Thr
    210                 215                 220
tta cag tta att gat atg tca gca acc tat aga gag gtt tta acc tca 720
Leu Gln Leu Ile Asp Met Ser Ala Thr Tyr Arg Glu Val Leu Thr Ser
225                 230                 235                 240
atg atg gat ata acc ctc tca tta gaa aat ata aag atg aac caa att 768
Met Met Asp Ile Thr Leu Ser Leu Glu Asn Ile Lys Met Asn Gln Ile
                245                 250                 255
atg aaa atc cta act atg gtt acg aca att ttc gct gtt ccc atg tgg 816
Met Lys Ile Leu Thr Met Val Thr Thr Ile Phe Ala Val Pro Met Trp
            260                 265                 270
att act gga ata tac ggg atg aac ttc tct tat tta cct tta gca aac 864
Ile Thr Gly Ile Tyr Gly Met Asn Phe Ser Tyr Leu Pro Leu Ala Asn
        275                 280                 285
aac cct caa ggt ttt tgg ctt gta atg gct ttg atg gtt gta ata att 912
Asn Pro Gln Gly Phe Trp Leu Val Met Ala Leu Met Val Val Ile Ile
    290                 295                 300
atg ata ttt gtc tat ata ttt aga agg tca gga tgg att taa 954
Met Ile Phe Val Tyr Ile Phe Arg Arg Ser Gly Trp Ile
305                 310                 315

<210> 86
<211> 317
<212> PRT
<213> Methanococcus jannaschii

<400> 86
Met Ile Thr Val Ile Ala Ile Ala Lys Asp Gly Ser Ile Val Glu Pro
1               5                   10                  15
Lys Leu Asp Glu Ile Ser Phe Glu Asp Tyr Arg Leu Ile Trp Ile Asp
            20                  25                  30
Cys Tyr Asp Pro Lys Asp Glu Glu Leu Tyr Lys Leu Ser Lys Lys Ile
        35                  40                  45
Gly Ile Ser Val Ser Asp Leu Gln Ile Gly Leu Asp Glu Gln Glu Ile

50                  55                  60
Pro Arg Val Glu Glu Asp Glu Asp Phe Tyr Leu Ile Ile Tyr Lys Ala
65                  70                  75                  80
Pro Leu Phe Glu Glu Asp Ile Thr Thr Thr Ser Leu Gly Ile Tyr Ile
                85                  90                  95
Lys Asn Asn Leu Leu Leu Thr Ile His Ser Asp Lys Ile Lys Ala Ile
            100                 105                 110
Gly Arg Leu His Lys Leu Ile Ser Thr Lys Lys Pro Arg Ile Val Phe
        115                 120                 125
Glu Arg Gly Ile Gly Phe Leu Leu Tyr His Ile Leu Asn Glu Ile Thr
    130                 135                 140
Arg Ser Tyr Ser Arg Ile Leu Met Asn Leu Glu Asp Glu Leu Glu Glu
145                 150                 155                 160
Leu Glu Asp Lys Leu Leu Ala Gly Tyr Asp Arg Glu Val Met Glu Lys
                165                 170                 175
Ile Leu Gly Leu Arg Lys Thr Leu Val Tyr Phe His Lys Ser Leu Ile
            180                 185                 190
Ala Asn Arg Asp Val Leu Val Leu Leu Lys Arg Lys Tyr Leu Pro Ile
        195                 200                 205
Thr Thr Lys Glu Asp Arg Glu Asn Phe Glu Asp Leu Tyr Tyr Asp Thr
    210                 215                 220
Leu Gln Leu Ile Asp Met Ser Ala Thr Tyr Arg Glu Val Leu Thr Ser
225                 230                 235                 240
Met Met Asp Ile Thr Leu Ser Leu Glu Asn Ile Lys Met Asn Gln Ile
                245                 250                 255
Met Lys Ile Leu Thr Met Val Thr Thr Ile Phe Ala Val Pro Met Trp
            260                 265                 270
Ile Thr Gly Ile Tyr Gly Met Asn Phe Ser Tyr Leu Pro Leu Ala Asn
        275                 280                 285
Asn Pro Gln Gly Phe Trp Leu Val Met Ala Leu Met Val Val Ile Ile
    290                 295                 300
Met Ile Phe Val Tyr Ile Phe Arg Arg Ser Gly Trp Ile
305                 310                 315

<210>   87
<211>   1164
<212>   DNA
<213>   Synechocystis sp.

<220>
<221>  CDS
<222>  (1)..(1164)

<400>   87
atg ccc caa acc caa ccc ggc att gag gat ttt ctc ctg cgc caa ctg      48
Met Pro Gln Thr Gln Pro Gly Ile Glu Asp Phe Leu Leu Arg Gln Leu
1               5                   10                  15
cgt cat ccc caa gaa gaa gag gaa gaa gaa gat tat ttt gac tac ttc      96
Arg His Pro Gln Glu Glu Glu Glu Glu Glu Asp Tyr Phe Asp Tyr Phe
            20                  25                  30
tat gac gag ccg ggt agt gaa ccg ggt acc ctc agc att gaa ccc gat      144
Tyr Asp Glu Pro Gly Ser Glu Pro Gly Thr Leu Ser Ile Glu Pro Asp
        35                  40                  45
gcg ccc cct tcc cgc att gtg ttg gtg gat tat agc ccc agc cat gcc      192
Ala Pro Pro Ser Arg Ile Val Leu Val Asp Tyr Ser Pro Ser His Ala
    50                  55                  60
gtg cga aag tcg gat att agt ccc aat gcc ctg aga cct tat tta ggt      240
Val Arg Lys Ser Asp Ile Ser Pro Asn Ala Leu Arg Pro Tyr Leu Gly
65                  70                  75                  80
aca aat act gtt tct tgg atg gat att gaa ggt tta ggc agt gaa gaa      288
Thr Asn Thr Val Ser Trp Met Asp Ile Glu Gly Leu Gly Ser Glu Glu
                85                  90                  95
gtg ctt aag gaa gtg ggg gaa att ttt aaa tta cac ccc cta ctt tta      336
Val Leu Lys Glu Val Gly Glu Ile Phe Lys Leu His Pro Leu Leu Leu
            100                 105                 110
gaa gat att gtt aac gtc ccc cag cga gca aag gta gaa gat tat aac      384
Glu Asp Ile Val Asn Val Pro Gln Arg Ala Lys Val Glu Asp Tyr Asn
        115                 120                 125
gac cat gtg atg gtt att gcc cat cgg gtg cgt cct aat cgg gaa gaa      432
Asp His Val Met Val Ile Ala His Arg Val Arg Pro Asn Arg Glu Glu
    130                 135                 140

gat ggc ttt gaa agt gag cag gtt agt ttt gtg ttg ggc aaa cgt tac 480
Asp Gly Phe Glu Ser Glu Gln Val Ser Phe Val Leu Gly Lys Arg Tyr
145                 150                 155                 160
cta tta act ttc cag gaa ggg cac att att gat tgt ttt aat ccc cta 528
Leu Leu Thr Phe Gln Glu Gly His Ile Ile Asp Cys Phe Asn Pro Leu
                165                 170                 175
agg gaa aga att cgc acc aat caa ggt aaa gtt tgc caa caa gga gcg 576
Arg Glu Arg Ile Arg Thr Asn Gln Gly Lys Val Cys Gln Gln Gly Ala
            180                 185                 190
gat tat ctt tgc tat ttg tta att gat atg tta att gat gaa tat ttt 624
Asp Tyr Leu Cys Tyr Leu Leu Ile Asp Met Leu Ile Asp Glu Tyr Phe
        195                 200                 205
ccc ctc ttg gaa gat tat gaa gaa cgc att gaa gcc cta gaa gac acc 672
Pro Leu Leu Glu Asp Tyr Glu Glu Arg Ile Glu Ala Leu Glu Asp Thr
    210                 215                 220
att att cgt aat ccc aac agc tca tta atg gag gaa att tac cat att 720
Ile Ile Arg Asn Pro Asn Ser Ser Leu Met Glu Glu Ile Tyr His Ile
225                 230                 235                 240
cgc cgg gaa ctt tta gcc ctg aga agg ttg att tgg ccc ctg cgc cat 768
Arg Arg Glu Leu Leu Ala Leu Arg Arg Leu Ile Trp Pro Leu Arg His
                245                 250                 255
gtg atg aat gtg ctg ctg cgg gac acc acc aat tcc atc gta aca gca 816
Val Met Asn Val Leu Leu Arg Asp Thr Thr Asn Ser Ile Val Thr Ala
            260                 265                 270
gac gta aga att tac ttt cga gat tgc tac gat cac att att cag gtg 864
Asp Val Arg Ile Tyr Phe Arg Asp Cys Tyr Asp His Ile Ile Gln Val
        275                 280                 285
tta gat att atc gaa gct tac cgg gaa tta gct tct agt tta atg gag 912
Leu Asp Ile Ile Glu Ala Tyr Arg Glu Leu Ala Ser Ser Leu Met Glu
    290                 295                 300
gtt tat atg act gcc atg agc aat aaa atg aat gaa gta atg aaa ttt 960
Val Tyr Met Thr Ala Met Ser Asn Lys Met Asn Glu Val Met Lys Phe
305                 310                 315                 320
ttg acc gtc att tct acc att ttt att ccc cta act ttt att gcc ggg 1008
Leu Thr Val Ile Ser Thr Ile Phe Ile Pro Leu Thr Phe Ile Ala Gly
                325                 330                 335
gtt tac ggt atg aac ttc aaa gaa atg cca gaa cta aac agc cgc tgg 1056
Val Tyr Gly Met Asn Phe Lys Glu Met Pro Glu Leu Asn Ser Arg Trp
            340                 345                 350
ggc tac tat att act tgg ata gtg atg ctc tta att gcc gga gga tcc 1104
Gly Tyr Tyr Ile Thr Trp Ile Val Met Leu Leu Ile Ala Gly Gly Ser
        355                 360                 365
ctc tat ttc ttc tgg cgt aag ggc tgg ctt agt ccc tcc tat gat ttg 1152
Leu Tyr Phe Phe Trp Arg Lys Gly Trp Leu Ser Pro Ser Tyr Asp Leu
    370                 375                 380
gga gaa aag taa 1164
Gly Glu Lys
385

<210> 88
<211> 387
<212> PRT
<213> Synechocystis sp.

<400> 88
Met Pro Gln Thr Gln Pro Gly Ile Glu Asp Phe Leu Leu Arg Gln Leu
1               5                   10                  15
Arg His Pro Gln Glu Glu Glu Glu Glu Glu Asp Tyr Phe Asp Tyr Phe
            20                  25                  30
Tyr Asp Glu Pro Gly Ser Glu Pro Gly Thr Leu Ser Ile Glu Pro Asp
        35                  40                  45
Ala Pro Pro Ser Arg Ile Val Leu Val Asp Tyr Ser Pro Ser His Ala
    50                  55                  60
Val Arg Lys Ser Asp Ile Ser Pro Asn Ala Leu Arg Pro Tyr Leu Gly
65                  70                  75                  80
Thr Asn Thr Val Ser Trp Met Asp Ile Glu Gly Leu Gly Ser Glu Glu
                85                  90                  95
Val Leu Lys Glu Val Gly Glu Ile Phe Lys Leu His Pro Leu Leu Leu
            100                 105                 110
Glu Asp Ile Val Asn Val Pro Gln Arg Ala Lys Val Glu Asp Tyr Asn
        115                 120                 125

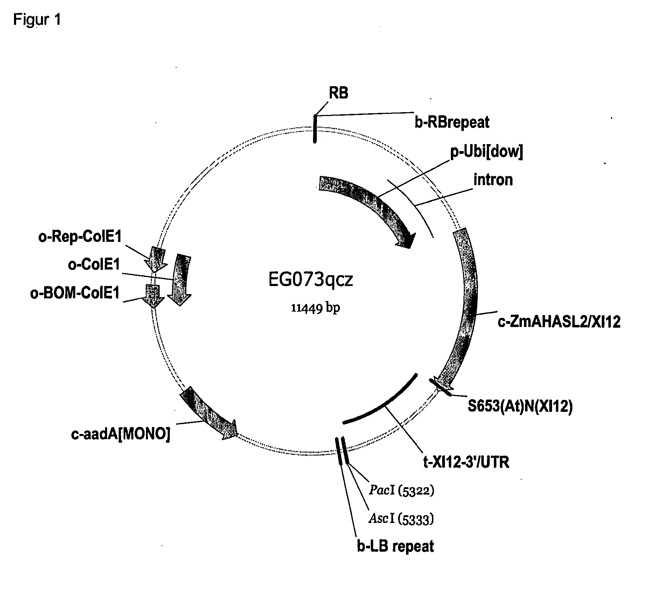

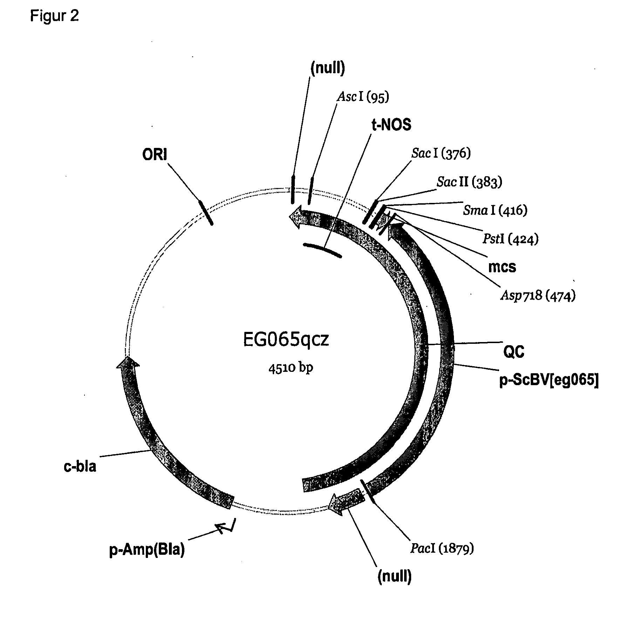

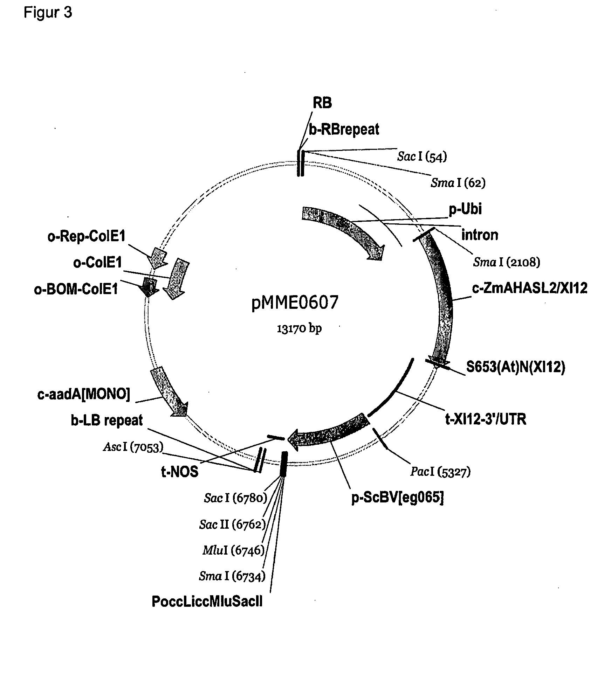

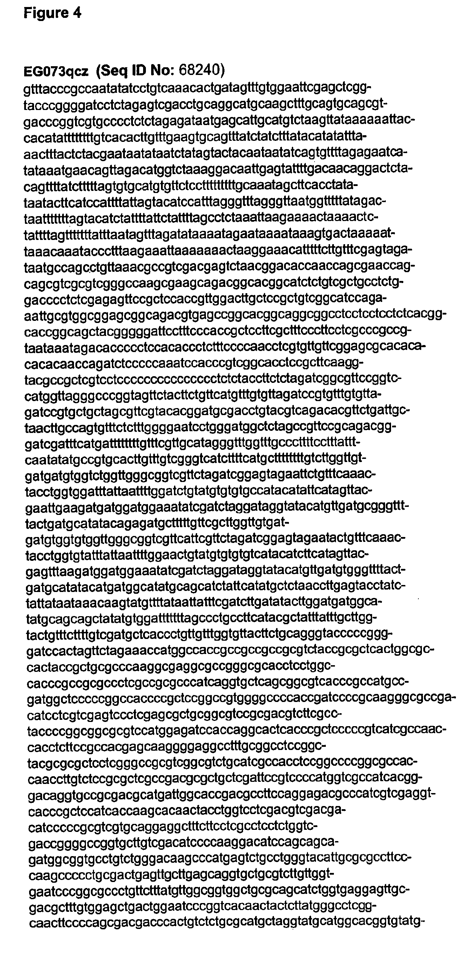

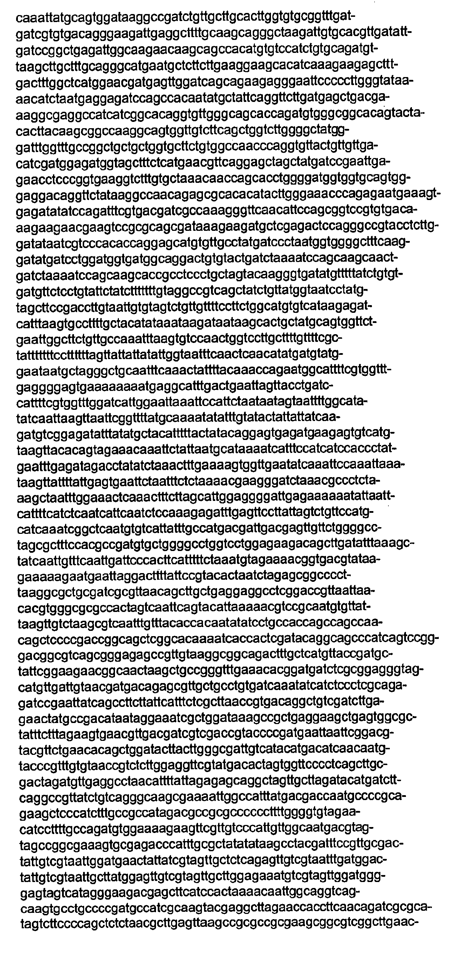

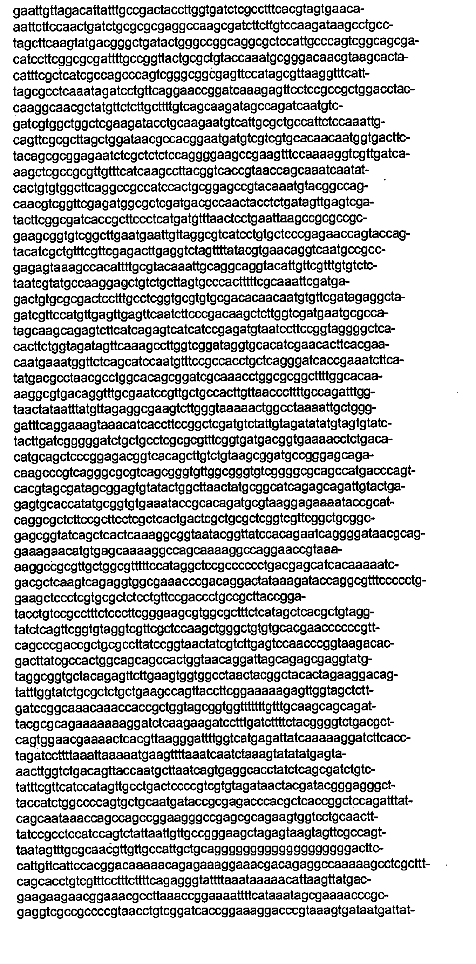

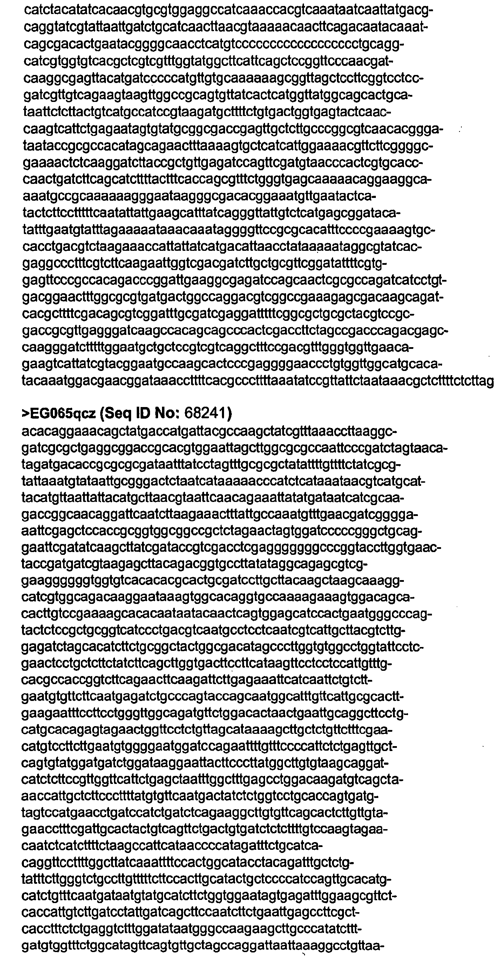

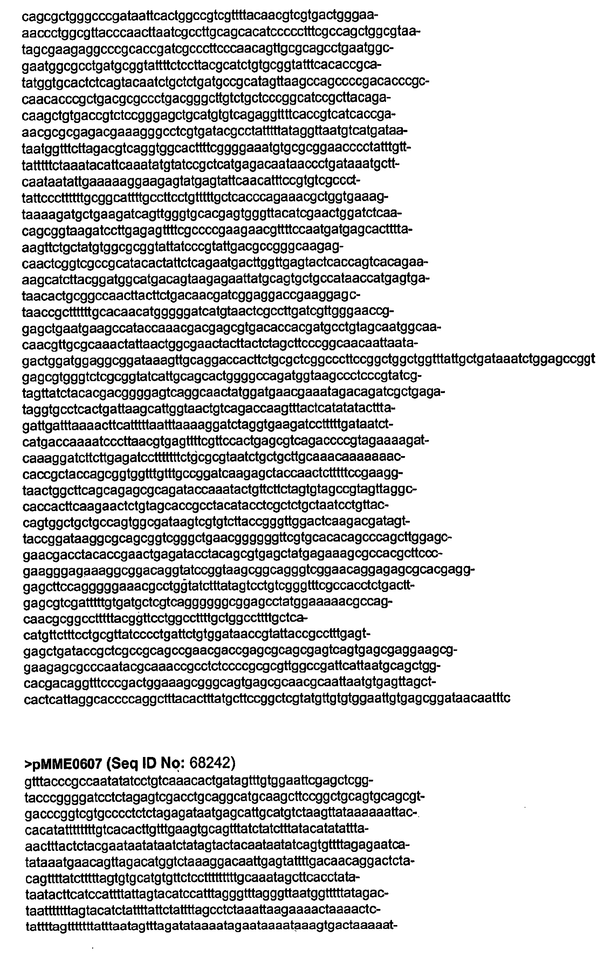

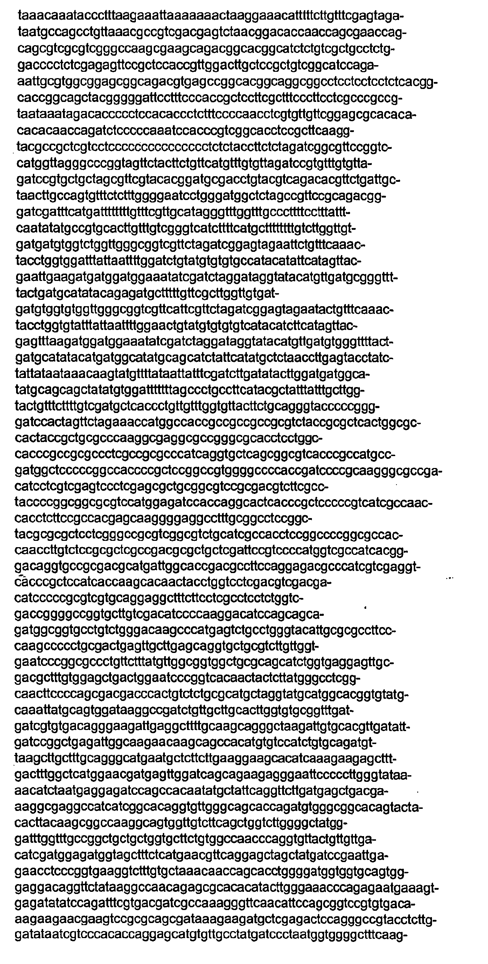

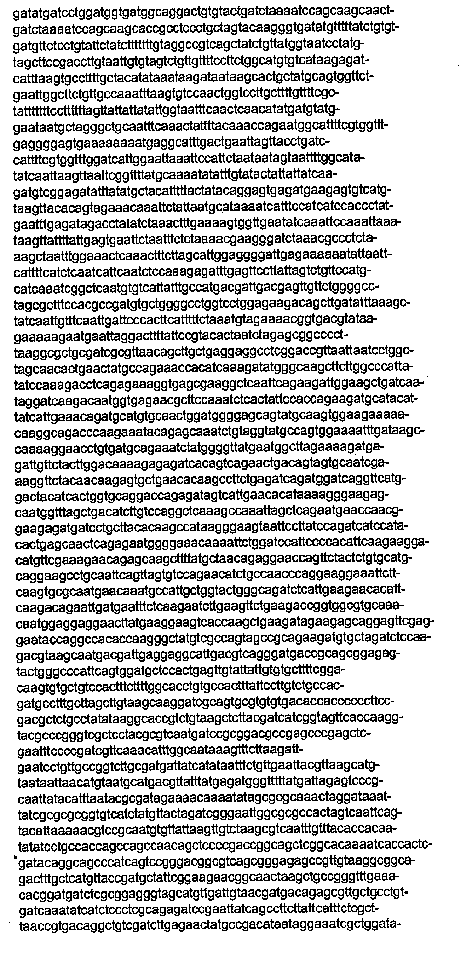

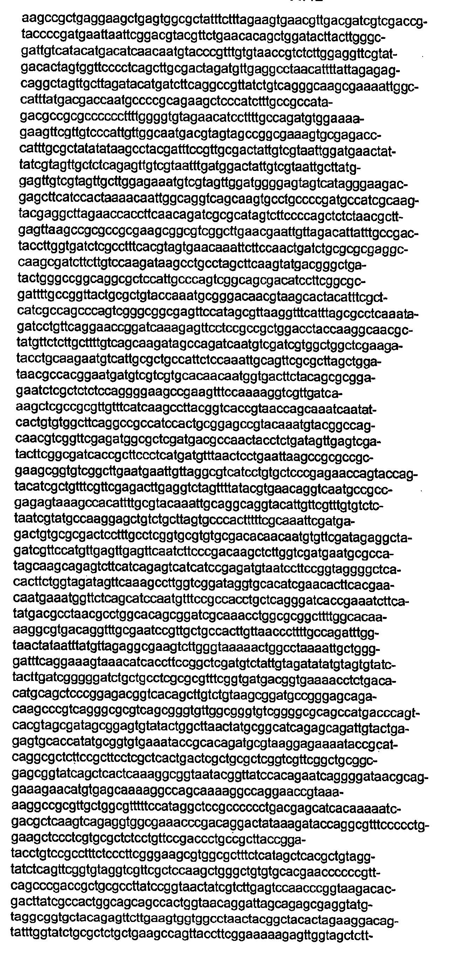

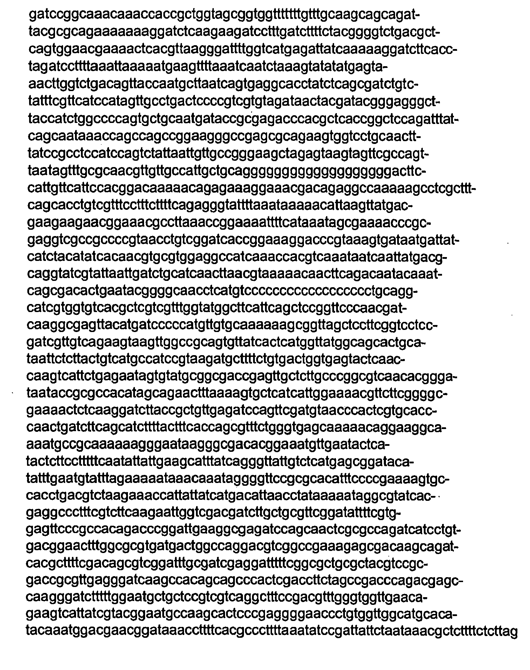

## Description

**[0001.0.0.0]** The present invention relates to a process for the production of a fine chemical in a microorgansm, a plant cell, a plant, a plant tissue or in one or more parts thereof. The in vention furthermore relates to nucleic acid molecules, polypeptides, nucleic acid constructs, vectors, antisense molecules, antibodies, host cells, plant tissue, propagtion material, harvested material, plants, microorganisms as well as agricultural compositions and to their use.

The instant application is based on and claims the benefit of prior filed European Patent Application No. 04105535.1, filed 27.08.2004, prior filed European Patent Application No. 04016615.9, filed 15.07.2004, prior filed European Patent Application No. 04015608.5, filed 02.07.2004, prior filed European Patent Application No. 04018543.1, filed 05.08.2004, prior filed European Patent Application No. 04105689.6, filed 23.08.2004, prior filed European Patent Application No. 04026008.5, filed 03.11.2004, prior filed European Patent Application No. 04026007.7, filed 03.11.2004, prior filed European Patent Application No. 04026057.2, filed 04.11.2004, prior filed European Patent Application No. 04026056.4, filed 04.11.2004, prior filed European Patent Application No. 04028670.0, filed 03.12.2004, prior filed European Patent Application No. 04028671.8, filed 03.12.2004, prior filed European Patent Application No. 04030101.2, filed 18.12.2004, prior filed European Patent Application No. 04106931.1, filed 17.12.2005, prior filed European Patent Application No. 04030391.9, filed 22.12.2004, prior filed European Patent Application No. 05100166.7, filed 10.01.2005, prior filed European Patent Application No. 05103449.4, filed 22.04.2005, prior filed European Patent Application No. 04107024.4, filed 23.12.2004, prior filed European Patent Application No. 04030100.4, filed 18.12.2004, prior filed European Patent Application No. 05101970.1, filed 14.03.2005, prior filed European Patent Application No. 04107025.1, filed 28.12.2004, prior filed European Patent Application No. 05104781.9, filed 27.05.2005, prior filed European Patent Application No. 05100704.5, filed 26.01.2005, prior filed European Patent Application No. 05103283.7, filed 26.04.2005, prior filed European Patent Application No. 05103455.1, filed 22.04.2005, prior filed European Patent Application No. 05103164.9, filed 20.04.2005, prior filed European Patent Application No. 05103428.8, filed 27.04.2005, prior filed European Patent Application No. 05104479.0, filed 25.05.2005, prior filed European Patent Application No. 05104496.4, filed 25.05.2005, prior filed European Patent Application No. 05105001.1, filed 06.06.2005, prior filed European Patent Application No. 05104874.2, filed 03.06.2005, prior filed European Patent Application No. 05105345.2, filed 10.06.2005, prior filed European Patent Application No. 05104630.8, filed 30.05.2005, prior filed European Patent Application No. 05104761.1, filed 01.06.2005, prior filed European Patent Application No. 05104811.4, filed 02.06.2005, prior filed European Patent Application No. 05104818.9, filed 02.06.2005, prior filed European Patent Application No. 05105021.9, filed 08.06.2005, prior filed European Patent Application No. 05105028.4, filed 08.06.2005, prior filed European Patent Application No. 05105136.5, filed 13.06.2005, prior filed European Patent Application No. (User number NAE419/05EP), filed 27.05.2005, prior filed European Patent Application No. 05105508.5, filed 21.06.2005, prior filed European Patent Application No. 05105575.4, filed 22.06.2005, prior filed European Patent Application No. 05105510.1, filed 21.06.2005, prior filed European Patent Application No. 05105401.3, filed 17.06.2005, prior filed European Patent Application No. 05105405.4, filed 17.06.2005, prior filed European Patent Application No. (User number NAE425_05EP), filed 27.05.2005, prior filed European Patent Application No. 05105570.5, filed 22.06.2005, prior filed European Patent Application No. 05105406.2, filed 17.06.2005, prior filed European Patent Application No. 05105624.0, filed 23.06.2005, prior filed European Patent Application No. 05105643.0, filed 23.06.2005 and prior filed European Patent Application No. 05105571.3, filed 22.06.2005. The entire content of the above-referenced patent applications are incorporated herein by this reference.

**[0002.0.0.0]** Amino acids are used in many branches of industry, including the food, animal feed, cosmetics, pharmaceutical and chemical industries. Amino acids such as D,L-methionine, L-lysine or L-threonine are used in the animal feed industry. The essential amino acids valine, leucine, isoleucine, lysine, threonine, methionine, tyrosine, phenylalanine and tryptophan are particularly important for the nutrition of humans and a number of livestock species. Glycine, L-methionine and tryptophan are all used in the pharmaceutical industry. Glutamine, valine, leucine, isoleucine, histidine, arginine, proline, serine and alanine are used in the pharmaceutical and cosmetics industries. Threonine, tryptophan and D,L-methionine are widely used feed additives (Leuchtenberger, W. (1996) Amino acids - technical production and use, pp. 466-502 in Rehm et al., (Ed.) Biotechnology vol. 6, chapter 14a, VCH Weinheim). Moreover, amino acids are suitable for the chemical industry as precursors for the synthesis of synthetic amino acids and proteins, such as N-acetylcysteine, S-carboxymethyl-L-cysteine, (S)-5-hydroxytryptophan and other substances described in Ullmann's Encyclopedia of Industrial Chemistry, vol. A2, pp. 57-97, VCH Weinheim, 1985.

**[0003.0.0.0]** Over one million tons of amino acids are currently produced annually; their market value amounts to over 2.5 billion US dollars. They are currently produced by four competing processes: Extraction from protein hydrolysates, for example L-cystine, L-leucine or L-tyrosine, chemical synthesis, for example of D-, L-methionine, conversion of chemical precursors in an enzyme or cell reactor, for example L-phenylalanine, and fermentative production by growing, on an industrial scale, bacteria which have been developed to produce and secrete large amounts of the desired molecule in question. An organism, which is particularly suitable for this purpose is Corynebacterium glutamicum, which is used for example for the production of L-lysine or L-glutamic acid. Other amino acids which are produced by fermentation are, for example, L-threonine, L-tryptophan, L-aspartic acid and L-phenylalanine.

**[0004.0.0.0]** The biosynthesis of the natural amino acids in organisms capable of producing them, for example bacteria, has been characterized thoroughly; for a review of the bacterial amino acid biosynthesis and its regulation, see Umbarger, H.E. (1978) Ann. Rev. Biochem. 47: 533 - 606.

**[0005.0.0.0]** It is known that amino acids are produced by fermentation of strains of coryneform bacteria, in particular Corynebacterium glutamicum. Due to their great importance, the production processes are constantly being improved. Process improvements can relate to measures regarding technical aspects of the fermentation, such as, for example, stirring and oxygen supply, or the nutrient media composition, such as, for example, the sugar concentration during fermentation, or to the work-up to give the product, for example by ion exchange chromatography, or to the intrinsic performance properties of the microorganism itself. Bacteria from other genera such as Escherichia or Bacillus are also used for the production of amino acids. A number of mutant strains, which produce an assortment of desirable compounds from the group of the sulfur-containing fine chemicals, have been developed via strain selection. The performance properties of said microorganisms are improved with respect to the production of a particular molecule by applying methods of mutagenesis, selection and mutant selection. Methods for the production of methionine have also been developed. In this manner, strains are obtained which are, for example, resistant to antimetabolites, such as, for example, the methionine analogues α-methylmethionine, ethionine, norleucine, N-acetyinorleucine, S-trifluoromethylhomocysteine, 2-amino-5-heprenoitic acid, selenomethionine, methionine sulfoximine, methoxine, 1-aminocyclopentanecarboxylic acid or which are auxotrophic for metabolites with regulatory importance and which produce sulfur-containing fine chemicals such as, for example, L-methionine. However, such processes developed for the production of methionine have the disadvantage that their yields are too low for being economically exploitable and that they are therefore not yet competitive with regard to chemical synthesis.

**[0006.0.0.0]** Zeh (Plant Physiol., Vol. 127, 2001: 792-802) describes increasing the methionine content in potato plants by inhibiting threonine synthase by what is known as antisense technology. This leads to a reduced threonine synthase activity without the threonine content in the plant being reduced. This technology is highly complex; the enzymatic activity must be inhibited in a very differentiated manner since otherwise auxotrophism for the amino acid occurs and the plant will no longer grow.

**[0007.0.0.0]** US 5,589,616 teaches the production of higher amounts of amino acids in plants by overexpressing a monocot storage protein in dicots. WO 96/38574, WO 97/07665, WO 97/28247, US 4,886,878, US 5,082,993 and US 5,670,635 are following this approach. That means in all the aforementioned intellectual property rights different proteins or polypeptides are expressed in plants. Said proteins or polypeptides should function as amino acid sinks. Other methods for increasing amino acids such as lysine are disclosed in WO 95/15392, WO 96/38574, WO 89/11789 or WO 93/19190. In this cases special enzymes in the amino acid biosynthetic pathway such as the diphydrodipicolinic acid synthase are deregulated. This leads to an increase in the production of lysine in the different plants. Another approach to increase the level of amino acids in plants is disclosed in EP-A-0 271 408. EP-A-0 271 408 teaches the mutagenesis of plant and selection afterwards with inhibitors of certain enzymes of amino acid biosynthetic pathway.

**[0008.0.0.0]** Methods of recombinant DNA technology have also been used for some years to improve Corynebacterium strains producing L-amino acids by amplifying individual amino acid biosynthesis genes and investigating the effect on amino acid production.

**[0009.0.0.0]** As described above, the essential amino acids are necessary for humans and many mammals, for example for livestock. L-methionine is important as methyl group donor for the biosynthesis of, for example, choline, creatine, adrenaline, bases and RNA and DNA, histidine, and for the transmethylation following the formation of S-adenosylmethionine or as a sulfhydryl group donor for the formation of cysteine. Moreover, L-methionine appears to have a positive effect in depression.

**[0010.0.0.0]** Improving the quality of foodstuffs and animal feeds is an important task of the food-and-feed industry. This is necessary since, for example, certain amino acids, which occur in plants are limited with regard to the supply of mammals.
Especially advantageous for the quality of foodstuffs and animal feeds is as balanced as possible an amino acid profile since a great excess of an amino acid above a specific concentration in the food has no further positive effect on the utilization of the food since other amino acids suddenly become limiting. A further increase in quality is only possible via addition of further amino acids, which are limiting under these conditions. The targeted addition of the limiting amino acid in the form of synthetic products must be carried out with extreme caution in order to avoid amino acid imbalance. For example, the addition of an essential amino acid stimulates protein digestion, which may cause deficiency situations for the second or third limiting amino acid, in particular. In feeding experiments, for example casein feeding experiments, the additional provision of methionine, which is limiting in casein, has revealed the fatty degeneration of liver, which could only be alleviated after the additional provision of tryptophan.

**[0011.0.0.0]** To ensure a high quality of foods and animal feeds, it is therefore necessary to add a plurality of amino acids in a balanced manner to suit the organism.

**[0012.0.0.0]** It is an object of the present invention to develop an inexpensive process for the synthesis of L-methionine. L-methionine is with lysine or threonine (depending on the organism) one of the two amino acids which are most frequently limiting

**[0013.0.0.0]** It was now found that this object is achieved by providing the process according to the invention described herein and the embodiments characterized in the claims.

**[0014.0.0.0]** Accordingly, in a first embodiment, the invention relates to a process for the production of a fine chemical, whereby the fine chemical is methionine Accordingly, in the present invention, the term "the fine chemical" as used herein relates to "methadone". Further, in another embodiment the term "the fine chemicals" as used herein also relates to compositions of fine chemicals comprising methionine.

**[0015.0.0.0]** In one embodiment, the term "the fine chemical" or "the respective fine chemical" means L-methionine. Throughout the specification the term "the fine chemical" or "the respective fine chemical" means methionine, preferably L-methionine, its salts, ester or amids in free form or bound to proteins. In a preferred embodiment, the term "the fine chemical" means L-methionine in free form or its salts or bound to proteins. In one embodiment, the term "the fine chemical" and the term "the respective fine chemical" mean at least one chemical compound with an activity of the above mentioned fine chemical.

**[0016.0.0.0]** Accordingly, the present invention relates to a process comprising
(a) increasing or generating the activity of one or more YLR375W, YBL015w, YER173w, YOR084w and/or b1829 and/or b4232, b0464, b1343, b2414, and/or b2762 protein(s) or of a protein having the sequence of a polypeptide encoded by a nucleic acid molecule indicated in Table I, columns 5 or 7, lines 1 to 5 and/or lines 334 to 338 in a non-human organism or in one or more parts thereof and
(b) growing the organism under conditions which permit the production of the fine chemical, thus, methionine or a fine chemicals comprising methionine, in said organism.
Accordingly, the present invention relates to a process for the production of a fine chemical comprising
(a) increasing or generating the activity of one or more proteins having the activity of a protein indicated in Table II, column 3, lines 1 to 5 and/or lines 334 to 338 or having the sequence of a polypeptide encoded by a nucleic acid molecule indicated in Table I, column 5 or 7, lines 1 to 5 and/or lines 334 to 338, in a non-human organism in one or more parts thereof and
(b) growing the organism under conditions which permit the production of the fine chemical, in particular methionine.

**[0016.1.0.0]** ./.

**[0017.0.0.0]** Comprises/comprising and grammatical variations thereof when used in this specification are to be taken to specify the presence of stated features, integers, steps or components or groups thereof, but not to preclude the presence or addition of one or more other features, integers, steps, components or groups thereof.
The term "Table I" used in this specification is to be taken to specify the content of Table I A and Table I B. The term "Table II" used in this specification is to be taken to specify the content of Table II A and Table II B. The term "Table I A" used in this specification is to be taken to specify the content of Table I A. The term "Table I B" used in this specification is to be taken to specify the content of Table I B. The term "Table II A" used in this specification is to be taken to specify the content of Table II A. The term "Table II B" used in this specification is to be taken to specify the content of Table II B. In one preferred embodiment, the term "Table I" means Table I B. In one preferred embodiment, the term "Table II" means Table II B.

**[0018.0.0.0]** Preferably, this process further comprises the step of recovering the fine chemical, which is synthesized by the organism from the organism and/or from the culture medium used for the growth or maintenance of the organism. The term "recovering" means the isolation of the fine chemical in different purities, that means on the one hand harvesting of the biological material, which contains the fine chemical without further purification and on the other hand purities of the fine chemical between 5% and 100% purity, preferred purities are in the range of 10% and 99%. In one embodiment, the purities are 20%, 30%, 40%, 50%, 60%, 70%, 80%, 90%, 95% or 99%.

**[0019.0.0.0]** Advantageously the process for the production of the fine chemical leads to an enhanced production of the fine chemical. The terms "enhanced" or "increase" mean at least a 10%, 20%, 30%, 40% or 50%, preferably at least 60%, 70%, 80%, 90% or 100%, more preferably 150%, 200%, 300%, 400% or 500% higher production of the fine chemical in comparison to the reference as defined below, e.g. that means in comparison to an organism without the aforementioned modification of the activity of a protein having the activity of a protein indicated in Table II, column 3, lines 1 to 5 and/or lines 334 to 338 or encoded by nucleic acid molecule indicated in Table I, columns 5 or 7, lines 1 to 5 and/or lines 334 to 338.

**[0020.0.0.0]** Surprisingly it was found, that the transgenic expression of at least one of the Saccharomyces cerevisiae protein(s) indicated in Table II, Column 3, lines 1 to 4 and/or at least one of the Escherichia coli K12 protein(s) indicated in Table II, Column 3, line 5 and/or lines 334 to 338 in Arabidopsis thaliana conferred an increase in the methionine content of the transformed plants.

**[0021.0.0.0]** In accordance with the invention, the term "organism" as understood herein relates always to a non-human organism, in particular to an animal or plant organism or to a microorganism. Further, the term "animal" as understood herein relates always to a non-human animal.
In accordance with the invention it is known to the skilled that anionic compounds such as acids are present in aqueous solutions in an equilibrium between the acid and its salts according to the pH present in the respective compartment of the cell or organism and the pK of the acid. Depending on the strength of the acid (pK) and the pH the salt or the free acid are predominant. Thus, the term "the fine chemical", the term "the respective fine chemical", or the term "acid" or the use of a denomination referring to a neutralized anionic compound relates to the anionic form as well as the neutralised status of that compound according to the milieu of the aqueous solution in which they are present.

**[0022.0.0.0]** The sequence of YLR375w from Saccharomyces cerevisiae has been published in Johnston, Nature 387 (6632 Suppl), 87-90, 1997, and Goffeau, Science 274 (5287), 546-547, 1996, and its activity is being "involved in pre-tRNA slicing and in uptake of branched-chain amino acids; YLR375wp". Accordingly, in one embodiment, the process of the present invention comprises the use of a gene product "involved in pre-tRNA slicing and in uptake of branched-chain amino skids" from Saccharomyces cerevisiae or its homolog, e.g. as shown herein, for the production of the fine chemical, meaning of methionine, in particular for increasing the amount of methionine in free or bound form in an organism or a part thereof, as mentioned.
The sequence of YBL015w from Saccharomyces cerevisiae has been published in Goffeau, Science 274 (5287), 546-547, 1996, and in Feldmann, EMBO J., 13, 5795-5809, 1994 and its activity is being defined as an "Mannose-containing glycoprotein which binds concanavalin A; Ach1p". In another reference, the activity is described as "acetyl-CoA hydrolase". Accordingly, in one embodiment, the process of the present invention comprises the use of a "Mannose-containing glycoprotein which binds concanavalin A; Ach1p" from Saccharomyces cerevisiae or its homolog, e.g. as shown herein, for the production of the fine chemical, meaning of methionin, in particular for increasing the amount of methionine in free or bound form in an organism or a part thereof, as mentioned. Accordingly, in one embodiment, the process of the present invention comprises the use of a "Acetyl-CoA hydrolase" from Saccharomyces cerevisiae or its homolog, e.g. as shown herein, for the production of the fine chemical, meaning of methionin, in particular for increasing the amount of methionine in free or bound form in an organism or a part thereof, as mentioned.
The sequence of YER 1 73w from Saccharomyces cerevisiae has been published in Dietrich, Nature 387 (6632 Suppl), 78-81, 1997, and Goffeau, Science 274 (5287), 546-547, 1996, and its activity is being defined as an "Checkpoint protein, involved in the activation of the DNA damage and meiotic pachytene checkpoints; subunit of a clamp loader that loads Rad17p-Mec3p-Dc1p onto DNA, homolog of the human and S. pompe Rad17 protein; Rad24p". Accordingly, in one embodiment, the process of the present invention comprises the use of a "Checkpoint protein, involved in the activation of the DNA damage and meiotic pachytene checkpoints" or its "subunit of a clamp loader that loads Rad17p-Mec3p-Dc1 p onto DNA" or a Rad24p from Saccharomyces cerevisiae or a Rad17 protein or its homolog, e.g. as shown herein, for the production of the fine chemical, meaning of methionin, in particular for increasing the amount of methionine in free or bound form in an organism or a part thereof, as mentioned.
The sequence of YOR084w from Saccharomyces cerevisiae has been published in Dujon, Nature 387 (6632 Suppl), 98-102, 1997, and Goffeau, Science 274 (5287), 546-547, 1996, and its activity is being defined as a putative lipase of the peroxisomal matrix. Accordingly, in one embodiment, the process of the present invention comprises the use of a putative lipase of the peroxisomal matrix" from Saccharomyces cerevisiae or its homolog, e.g. as shown herein, for the production of the fine chemical, meaning of methionin, in particular for increasing the amount of methionine in free or bound form in an organism or a part thereof, as mentioned.
The sequence of b1829 from Escherichia coli K12 has been published in Blattner, Science 277(5331), 1453-1474, 1997, and its activity is being defined as a heat shock protein. Accordingly, in one embodiment, the process of the present invention comprises the use of a "heat shock protein" from E. coli or its homolog, e.g. as shown herein, for the production of the fine chemical, meaning of methionin, in particular for increasing the amount of methionine in free or bound form in an organism or a part thereof, as mentioned. In one embodiment, in the process of the present invention the activity of a htpX heat shock protein is increased or generated, e.g. from E. coli or a homolog thereof. Homologs are also for example the htpX heat shock protein is also annotated as having a protease activity. Accordingly, in one embodiment, in the process of the present invention the activity of a protease, preferably of a heat shock protease, more preferred of a htpX protease or its homolog is increased for the production of the fine chemical, meaning of methionin, in particular for increasing the amount of methionine in free or bound form in an organism or a part thereof, as mentioned.
The sequence of b0464 from Escherichia coli K12 has been published in Blattner, Science 277(5331), 1453-1474, 1997, and its activity is being defined as a"transcriptional repressor for multidrug efflux pump (TetR/AcrR family)". Accordingly, in one embodiment, the process of the present invention comprises the use of a "transcriptional repressor for multidrug efflux pump (TetR/AcrR family)" from E. coli or its homolog, e.g. as shown herein, for the production of the fine chemical, meaning of methionine, in particular for increasing the amount of methionine in free or bound form in an organism or a part thereof, as mentioned. In one embodiment, in the process of the present invention the activity of a protein of the superfamily "probable transcription repressor mtrr", is increased or generated, preferably having activity in transcriptional control and/or DNA binding, e.g. from E. coli or a homolog thereof.. Accordingly, in one embodiment, in the process of the present invention the activity of a "transcriptional repressor for multidrug efflux pump (TetR/AcrR family)"or its homolog is increased for the production of the fine chemical, meaning of methionine, in particular for increasing the amount of methionine in free or bound form in an organism or a part thereof, as mentioned.

The sequence of b1343 from Escherichia coli K12 has been published in Blattner, Science 277(5331), 1453-1474, 1997, and its activity is being defined as an ATP-dependent RNA helicase, stimulated by 23S rRNA. Accordingly, in one embodiment, the process of the present invention comprises the use of an "ATP-dependent RNA helicase, stimulated by 23S rRNA" from E. coli or its homolog, e.g. as shown herein, for the production of the fine chemical, meaning of methionine, in particular for increasing the amount of methionine in free or bound form in an organism or a part thereof, as mentioned. In one embodiment, in the process of the present invention the activity of a protein having an activity in rRNA processing or translation is increased or generated, e.g. from E. coli or a homolog thereof. Accordingly, in one embodiment, in the process of the present invention the activity of a ATP-dependent RNA helicase, stimulated by 23S rRNA or its homolog is increased for the production of the fine chemical, meaning of methionine, in particular for increasing the amount of methionine in free or bound form in an organism or a part thereof, as mentioned.

The sequence of b2414 from Escherichia coli K12 has been published in Blattner, Science 277(5331), 1453-1474, 1997, and its activity is being defined as a subunit of cysteine synthase A and O-acetylserine sulfhydrolase A, PLP-dependent enzyme. Accordingly, in one embodiment, the process of the present invention comprises the use of a "subunit of cysteine synthase A and O-acetylserine sulfhydrolase A, PLP-dependent enzyme" from E. coli or its homolog, e.g. as shown herein, for the production of the fine chemical, meaning of methionine, in particular for increasing the amount of methionine in free or bound form in an organism or a part thereof, as mentioned. In one embodiment, in the process of the present invention the activity of a protein of the superfamily "threonine dehydratase", preferably having an activity in amino acid biosynthesis, biosynthesis of the cysteine-aromatic group, degradation of amino acids of the cysteine-aromatic group, nitrogen and sulfur utilizationbiosynthesis of the aspartate family, degradation of amino acids of the aspartate group, biosynthesis of sulfuric acid and L-cysteine derivatives, biosynthesis of secondary products derived from primary amino acids, biosynthesis of secondary products derived from glycine, L-serine and L-alanine, pyridoxal phosphate binding, more preferred having an "subunit of cysteine synthase A and O-acetylserine sulfhydrolase A, PLP-dependent enzyme"-activity is increased or generated, e.g. from E. coli or a homolog thereof.. Accordingly, in one embodiment, in the process of the present invention the activity of a "subunit of cysteine synthase A and O-acetylserine sulfhydrolase A, PLP-dependent enzyme" or its homolog is increased for the production of the fine chemical, meaning of methionine, in particular for increasing the amount of methionine in free or bound form in an organism or a part thereof, as mentioned.
The sequence of b2762 from Escherichia coli K12 has been published in Blattner, Science 277(5331), 1453-1474, 1997, and its activity is being defined as a 3'-phosphoadenosine 5'-phosphosulfate (PAPS) reductase. Accordingly, in one embodiment, the process of the present invention comprises the use of a "3'-phosphoadenosine 5'-phosphosulfate (PAPS) reductase" from E. coli or its homolog, e.g. as shown herein, for the production of the fine chemical, meaning of methionine, in particular for increasing the amount of methionine in free or bound form in an organism or a part thereof, as mentioned. In one embodiment, in the process of the present invention the activity of a protein of the superfamily "3'-phosphoadenosine 5'-phosphosulfate reductase", preferably having an activity in biosynthesis of cysteine, nitrogen and sulfur utilization, amino acid biosynthesis more preferred having an "3'-phosphoadenosine 5'-phosphosulfate (PAPS) reductase"-activity is increased or generated, e.g. from E. coli or a homolog thereof. Accordingly, in one embodiment, in the process of the present invention the activity of a "3'-phosphoadenosine 5'-phosphosulfate (PAPS) reductase" or its homolog is increased for the production of the fine chemical, meaning of methionine, in particular for increasing the amount of methionine in free or bound form in an organism or a part thereof, as mentioned.
The sequence of b4232 from Escherichia coli K12 has been published in Blattner, Science 277(5331), 1453-1474, 1997, and its activity is being defined as a fructose-1,6-bisphosphatase. Accordingly, in one embodiment, the process of the present invention comprises the use of a "fructose-1,6-bisphosphatase" from E. coli or its homolog, e.g. as shown herein, for the production of the fine chemical, meaning of methionine, in particular for increasing the amount of methionine in free or bound form in an organism or a part thereof, as mentioned. In one embodiment, in the process of the present invention the activity of a protein of the superfamily "fructose-bisphosphatase", preferably having an activity in C-compound and carbohydrate metabolism, C-compound and carbohydrate utilization, energy, glycolysis and gluconeogenesis, plastid, photosynthesis, more preferred having an "fructose-1,6-bisphosphatase"-activity, is increased or generated, e.g. from E. coli or a homolog thereof. Accordingly, in one embodiment, in the process of the present invention the activity of a "fructose-1,6-bisphosphatase" or its homolog is increased for the production of the fine chemical, meaning of methionine, in particular for increasing the amount of methionine in free or bound form in an organism or a part thereof, as mentioned.

**[0023.0.0.0]** Homologues (= homologs) of the present gene products can be derived from any organisms as long as the homologue confers the herein mentioned activity, in particular, confers an increase in the fine chemical amount or content. Further, in the present invention, the term "homologue" relates to the sequence of an organism having the highest sequence homology to the herein mentioned or listed sequences of all expressed sequences of said organism.
However, the person skilled in the art knows, that, preferably, the homologue has said the-fine-chemical-increasing activity and, if known, the same biological function or activity in the organism as at least one of the protein(s) indicated in Table I, Column 3, lines 1 to 5 and/or lines 334 to 338, e.g. having the sequence of a polypeptide encoded by a nucleic acid molecule comprising the sequence indicated in indicated in Table I, Column 5 or 7, lines 1 to 5 and/or lines 334 to 338.
In one embodiment, the homolog of any one of the polypeptides indicated in Table II, lines 1 to 4 is a homolog having the same or a similar activity, in particular an increase of activity confers an increase in the content of the fine chemical in the organisms and being derived from an Eukaryot. In one embodiment, the homolog of a polypeptide indicated in Table II, column 3, line 5 and/or lines 334 to 338 is a homolog having the same or a similar activity, in particular an increase of activity confers an increase in the content of the fine chemical in the organisms or part thereof, and being derived from bacteria. In one embodiment, the homolog of a polypeptide indicated in Table II, column 3, lines 1 to 4 is a homolog having the same or a similar activity, in particular an increase of activity confers an increase in the content of the fine chemical in an organisms or part thereof, and being derived from Fungi. In one embodiment, the homolog of a polypeptide indicated in Table II, column 3, line 5 and/or lines 334 to 338 is a homolog having the same or a similar activity, in particular an increase of activity confers an increase in the content of the fine chemical in the organisms or part thereof and being derived from Proteobacteria. In one embodiment, the homolog of a polypeptide indicated in Table II, column 3, lines 1 to 4 is a homolog having the same or a similar activity, in particular an increase of activity confers an increase in the content of the fine chemical in the organisms or a part thereof and being derived from Ascomycota. In one embodiment, the homolog of a polypeptide indicated in Table II, column 3, line 5 and/or lines 334 to 338 is a homolog having the same or a similar activity, in particular an increase of activity confers an increase in the content of the fine chemical in the organisms or part thereof, and being derived from Gammaproteobacteria. In one embodiment, the homolog of a polypeptide indicated in Table II, column 3, lines 1 to 4 is a homolog having the same or a similar activity, in particular an increase of activity confers an increase in the content of the fine chemical in the organisms or part thereof, and being derived from Saccharomycotina. In one embodiment, the homolog of a polypeptide indicated in Table II, column 3, line 5 and/or lines 334 to 338 is a homolog having the same or a similar activity, in particular an increase of activity confers an increase in the content of the fine chemical in the organisms or part thereof, and being derived from Enterobacteriales. In one embodiment, the homolog of a polypeptide indicated in Table II, column 3, lines 1 to 4 is a homolog having the same or a similar activity, in particular an increase of activity confers an increase in the content of the fine chemical in the organisms or a part thereof, and being derived from Saccharomycetes. In one embodiment, the homolog of the a polypeptide indicated in Table II, column 3, line 5 and/or lines 334 to 338 is a homolog having the same or a similar activity, in particular an increase of activity confers an increase in the content of the fine chemical in the organisms or part thereof, and being derived from Enterobacteriaceae. In one embodiment, the homolog of a polypeptide indicated in Table II, column 3, lines 1 to 4 is a homolog having the same or a similar activity, in particular an increase of activity confers an increase in the content of the fine chemical in the organisms, and being derived from Saccharomycetales. In one embodiment, the homolog of a polypeptide indicated in Table II, column 3, lines 5 and/or lines 334 to 338 is a homolog having the same or a similar activity, in particular an increase of activity confers an increase in the content of the fine chemical in the organisms or a part thereof, and being derived from Escherichia. In one embodiment, the homolog of a polypeptide indicated in Table II, column 3, lines 1 to 4 is a homolog having the same or a similar activity, in particular an increase of activity confers an increase in the content of the fine chemical in the organisms or a part thereof, and being derived from Saccharomycetaceae. In one embodiment, the homolog of a polypeptide indicated in Table II, column 3, line 1 to 4 is a homolog having the same or a similar activity, in particular an increase of activity confers an increase in the content of the fine chemical in the organisms or a part thereof, and being derived from Saccharomycetes.

**[0023.1.0.0]** Homologs of the polypeptides polypeptide indicated in Table II, column 3, lines 1 to 4 may be the polypeptides encoded by the nucleic acid molecules polypeptide indicated in Table I, column 7, lines 1 to 4 or may be the polypeptides indicated in Table II, column 7, lines 1 to 4.
Homologs of the polypeptides polypeptide indicated in Table II, column 3, line 5 and/or lines 334 to 338 may be the polypeptides encoded by the nucleic acid molecules polypeptide indicated in Table I, column 7, line 5 and/or lines 334 to 338 or may be the polypeptides indicated in Table II, column 7, lines 5 and/or lines 334 to 338.

**[0024.0.0.0]** Further homologs of are described herein below.

**[0025.0.0.0]** In accordance with the invention, a protein or polypeptide has the "activity of an protein of the invention", or of a protein as used in the invention, e.g. a protein having the activity of a protein indicated in Table II, column 3, lines 1 to 5 and/or lines 334 to 338 if its *de novo* activity, or its increased expression directly or indirectly leads to an increased methionine, preferably L-methionine level in the organism or a part thereof, preferably in a cell of said organism. In a preferred embodiment, the protein or polypeptide has the above-mentioned additional activities of a protein indicated in Table II, column 3, lines 1 to 5 and/or lines 334 to 338. During the specification the activity or preferably the biological activity of such a protein or polypeptide or an nucleic acid molecule or sequence encoding such protein or polypeptide is identical or similar if it still has the biological or enzymatic activity of any one of the proteins indicated in Table II, column 3, lines 1 to 5 and/or lines 334 to 338, i.e. if it has at least 10% of the original enzymatic activity, preferably 20%, particularly preferably 30%, most particularly preferably 40% in comparison to an any one of the proteins indicated in Table II, column 3, lines 1 to 4 of Saccharomyces cerevisiae and/or any one of the proteins indicated in Table II, column 3, line 5 and/or lines 334 to 338of E. coli K12.

**[0025.1.0.0]** In one embodiment, the polypeptide of the invention or the polypeptide used in the method of the invention confers said activity, e.g. the increase of the fine chemical in an organism or a part thereof, if it is derived from an organism, which is evolutionary distant to the organism in which it is expressed. For example origin and expressing organism are derived from different families, orders, classes or phylums.

**[0025.2.0.0]** In one embodiment, the polypeptide of the invention or the polypeptide used in the method of the invention confers said activity, e.g. the increase of the fine chemical in an organism or a part thereof, if it is derived from an organism, which is evolutionary close to the organism indicated in Table I, column 4 and is expressed in an organism, which is evolutionary distant to the origin organism. For example origin and expressing organism are derived from different families, orders, classes or phylums whereas origin and the organism indicated in Table I, column 4 are derived from the same families, orders, classes or phylums.

**[0026.0.0.0]** The terms "increased", "rose", "extended", "enhanced", "improved" or "amplified" relate to a corresponding change of a property in an organism, a part of an organism such as a tissue, seed, root, leave, flower etc. or in a cell and are interchangeable. Preferably, the overall activity in the volume is increased or enhanced in cases if the increase or enhancement is related to the increase or enhancement of an activity of a gene product, independent whether the amount of gene product or the specific activity of the gene product or both is increased or enhanced or whether the amount, stability or translation efficacy of the nucleic acid sequence or gene encoding for the gene product is increased or enhanced. The terms "reduction", "decrease" or "deletion" relate to a corresponding change of a property in an organism, a part of an organism such as a tissue, seed, root, leave, flower etc. or in a cell. Preferably, the overall activity in the volume is reduced, decreased or deleted in cases if the reduction, decrease or deletion is related to the reduction, decrease or deletion of an activity of a gene product, independent whether the amount of gene product or the specific activity of the gene product or both is reduced, decreased or deleted or whether the amount, stability or translation efficacy of the nucleic acid sequence or gene encoding for the gene product is reduced, decreased or deleted.

**[0027.0.0.0]** The terms "increase" or "decrease" relate to a corresponding change of a property an organism or in a part of an organism, such as a tissue, seed, root, leave, flower etc. or in a cell.. Preferably, the overall activity in the volume is increased in cases the increase relates to the increase of an activity of a gene product, independent whether the amount of gene product or the specific activity of the gene product or both is increased or generated or whether the amount, stability or translation efficacy of the nucleic acid sequence or gene encoding for the gene product is increased.

**[0028.0.0.0]** Under "change of a property" it is understood that the activity, expression level or amount of a gene product or the metabolite content is changed in a specific volume relative to a corresponding volume of a control, reference or wild type, including the de novo creation of the activity or expression.

**[0029.0.0.0]** The terms "increase" or "decrease" include the change or the modulation of said property in only parts of the subject of the present invention, for example, the modification can be found in compartment of a cell, like a organelle, or in a part of a plant, like tissue, seed, root, leave, flower etc. but is not detectable if the overall subject, i.e. complete cell or plant, is tested. Preferably, the increase or decrease is found cellular, thus the term "increase of an activity" or "increase of a metabolite content" relates to the cellular increase compared to the wild type cell. However, the terms increase or decrease as used herein also include the change or modulation of a property in the whole organism as mentioned.

**[0030.0.0.0]** Accordingly, the term "increase" or "decrease" means that the specific activity of an enzyme, preferably the amount of a compound or metabolite, e.g. of a polypeptide, a nucleic acid molecule or of the respective fine chemical of the invention or an encoding mRNA or DNA, can be increased or decreased in a volume.

**[0031.0.0.0]** The terms "wild type", "control" or "reference" are exchangeable and can be a cell or a part of organisms such as an organelle or a tissue, or an organism, in particular a microorganism or a plant, which was not modified or treated according to the herein described process according to the invention. Accordingly, the cell or a part of organisms such as an organelle or a tissue, or an organism, in particular a microorganism or a plant used as wild type, control or reference corresponds to the cell, organism or part thereof as much as possible and is in any other property but in the result of the process of the invention as identical to the subject matter of the invention as possible. Thus, the wild type, control, or reference is treated identically or as identical as possible, saying that only conditions or properties might be different which do not influence the quality of the tested property.

**[0032.0.0.0]** Preferably, any comparison is carried out under analogous conditions. The term "analogous conditions" means that all conditions such as, for example, culture or growing conditions, assay conditions (such as buffer composition, temperature, substrates, pathogen strain, concentrations and the like) are kept identical between the experiments to be compared.

**[0033.0.0.0]** The "reference", "control", or "wild type" is preferably a subject, e.g. an organelle, a cell, a tissue, an organism, in particular a plant or a microorganism, which was not modified or treated according to the herein described process of the invention and is in any other property as similar to the subject matter of the invention as possible. The reference, control, or wild type is in its genome, transcriptome, proteome or meta-bolome as similar as possible to the subject of the present invention. Preferably, the term "reference-" "control-" or "wild type-"-organelle, -cell, -tissue or -organism, in particular plant or microorganism, relates to an organelle, cell, tissue or organism, in particular plant or microorganism, which is nearly genetically identical to the organelle, cell, tissue or organism, in particular microorganism or plant, of the present invention or a part thereof preferably 95%, more preferred are 98%, even more preferred are 99,00%, in particular 99,10%, 99,30%, 99,50%, 99,70%, 99,90%, 99,99%, 99, 999% or more.. Most preferable the "reference", "control", or "wild type" is a subject, e.g. an organelle, a cell, a tissue, an organism, which is genetically identical to the organism, cell or organelle used according to the process of the invention except that the responsible or activity conferring nucleic acid molecules or the gene product encoded by them are amended, manipulated, exchanged or introduced according to the inventive process.

**[0034.0.0.0]** Preferably, the reference, control or wild type differs form the subject of the present invention only in the cellular activity of the polypeptide of the invention or the polypeptide used in the method of the invention, e.g. as result of an increase in the level of the nucleic acid molecule of the present invention or an increase of the specific activity of the polypeptide of the invention or the polypeptide used in the method of the invention. E.g., it differs by or in the expression level or activity of an protein having the activity of a protein as indicated in Table II, column 3, lines 1 to 5 and/or lines 334 to 338 or being encoded by a nucleic acid molecule indicated in Table I, column 5, lines 1 to 5 and/or lines 334 to 338 or its homologs, e.g. as indicated in Table I, column 7, lines 1 to 5 and/or lines 334 to 338, its biochemical or genetical causes and therefore shows the increased amount of the fine chemical.

**[0035.0.0.0]** In case, a control, reference or wild type differing from the subject of the present invention only by not being subject of the process of the invention can not be provided, a control, reference or wild type can be an organism in which the cause for the modulation of an activity conferring the increase of the fine chemical or expression of the nucleic acid molecule as described herein has been switched back or off, e.g. by knocking out the expression of responsible gene product, e.g. by antisense inhibition, by inactivation of an activator or agonist, by activation of an inhibitor or antagonist, by inhibition through adding inhibitory antibodies, by adding active compounds as e.g. hormones, by introducing negative dominant mutants, etc. A gene production can for example be knocked out by introducing inactivating point mutations, which lead to an enzymatic activity inhibition or a destabilization or an inhibition of the ability to bind to cofactors etc.

**[0036.0.0.0]** Accordingly, preferred reference subject is the starting subject of the present process of the invention. Preferably, the reference and the subject matter of the invention are compared after standardization and normalization, e.g. to the amount of total RNA, DNA, or Protein or activity or expression of reference genes, like housekeeping genes, such as ubiquitin, actin or ribosomal proteins.

**[0037.0.0.0]** A series of mechanisms exists via which a modification of a protein, e.g. the polypeptide of the invention or the polypeptide used in the method of the invention can directly or indirectly affect the yield, production and/or production efficiency of the fine chemical.

**[0038.0.0.0]** For example, the molecule number or the specific activity of the polypeptide or the nucleic acid molecule may be increased. Larger amounts of the fine chemical can be produced if the polypeptide or the nucleic acid of the invention is expressed *de novo* in an organism lacking the activity of said protein. However, it is also possible to increase the expression of the gene which is naturally present in the organisms, for example by amplifying the number of gene(s), by modifying the regulation of the gene, or by increasing the stability of the corresponding mRNA or of the corresponding gene product encoded by the nucleic acid molecule of the invention or the nucleic acid molecule used in the method of the invention, or by introducing homologous genes from other organisms which are differently regulated, e.g. not feedback sensitive.

**[0039.0.0.0]** This also applies analogously to the combined increased expression of the nucleic acid molecule of the present invention or its gene product with that of further enzymes or regulators of the biosynthesis pathways of the respective fine chemical, e.g. which are useful for the synthesis of the respective fine chemicals.

**[0040.0.0.0]** The increase, decrease or modulation according to this invention can be constitutive, e.g. due to a stable permanent transgenic expression or to a stable mutation in the corresponding endogenous gene encoding the nucleic acid molecule of the invention or the nucleic acid molecule used in the method of the invention or to a modulation of the expression or of the behaviour of a gene conferring the expression of the polypeptide of the invention or the polypeptide used in the method of the invention, or transient, e.g. due to an transient transformation or temporary addition of a modulator such as a agonist or antagonist or inducible, e.g. after transformation with a inducible construct carrying the nucleic acid molecule of the invention or the nucleic acid molecule used in the method of the invention under control of a inducible promoter and adding the inducer, e.g. tetracycline or as described herein below.

**[0041.0.0.0]** The increase in activity of the polypeptide amounts in a cell, a tissue, a organelle, an organ or an organism or a part thereof preferably to at least 5%, preferably to at least 20% or at to least 50%, especially preferably to at least 70%, 80%, 90% or more, very especially preferably are to at least 200%, most preferably are to at least 500% or more in comparison to the control, reference or wild type.

**[0042.0.0.0]** The specific activity of a polypeptide encoded by a nucleic acid molecule of the present invention or of the polypeptide of the present invention can be tested as described in the examples. In particular, the expression of a protein in question in a cell, e.g. a plant cell or a microorganism and the detection of an increase the respective fine chemical level in comparison to a control is an easy test and can be performed as described in the state of the art.

**[0043.0.0.0]** The term "increase" includes, that a compound or an activity is introduced into a cell *de novo* or that the compound or the activity has not been detectable before, in other words it is "generated".

**[0044.0.0.0]** Accordingly, in the following, the term "increasing" also comprises the term "generating" or "stimulating". The increased activity manifests itself in an increase of the fine chemical.

**[0045.0.0.0]** In one embodiment, in case the activity of the Saccharomyces cerevisiae protein YLR375W or its homologs, e.g. as indicated in Table II, columns 5 or 7, line 1, is increased; preferably, an increase of the fine chemical between 110% and 300% or more is conferred.
In one embodiment, in case the activity of the Saccharomyces cerevisiae protein YBL015w or an acetyl-CoA hydrolase, or its homologs, e.g. as indicated in Table II, columns 5 or 7, line 2, is increased; preferably, the increase of the fine chemical between 110% and 300% or more is conferred.
In one embodiment, in case the activity of the Saccharomyces cerevisiae protein YER173w or its homologs, e.g. as indicated in Table II, columns 5 or 7, line 3, e.g. a checkpoint protein, involved in the activation of the DNA damage and meiotic pychtene checkpoints; subunit of a clamp loader that loads Rad17p-Mec3p-Ddc1p onto DNA or Rad24p or its homologs, e.g. the human or S. pombe Rad17 is increased; preferably, the increase of the fine chemical between 110% and 200% or more is conferred.
In one embodiment, in case the activity of the Saccharomyces cerevisiae protein YOR084w or an putative Lipase of the peroxisomal matrix or its homologs, e.g. as indicated in Table II, columns 5 or 7, line 4, is increased; preferably, the increase of the fine chemical between 110% and 350% or more is conferred.
In one embodiment, in case the activity of the Escherichia coli K12 protein b1829 or its homologs, e.g. as indicated in Table II, columns 5 or 7, line 5, is increased, e.g. the activity of a protease is increased, preferably, the activity of a heat shock protein is increased, more preferred the activity of a htpX protein or its homolog is increased; preferably, the increase of the fine chemical between 110% and 400% or more is conferred.
In one embodiment, in case the activity of the Escherichia coli K12 protein b4232 or its homologs, e.g. as indicated in Table II, columns 5 or 7, line 338, is increased, e.g. the activity of a fructose-bisphosphatase-superfamily-protein is increased, preferably, the activity a protein involved in C-compound and carbohydrate metabolism, C-compound and carbohydrate utilization, ENERGY, glycolysis and gluconeogenesis, plastid, and/or photosynthesis is increased, more preferred the activity of a fructose-1,6-bisphosphatase or its homolog is increased. Preferably, the increase of the fine chemical around 20% or more is conferred.
In one embodiment, in case the activity of the Escherichia coli K12 protein b0464 or its homologs, e.g. as indicated in Table II, columns 5 or 7, line 334, is increased, e.g. the activity of a probable transcription repressor mtrr superfamily-protein is increased, preferably, the activity a protein involved in transcriptional control, and/or DNA binding is increased, more preferred the activity of a transcriptional repressor for multidrug efflux pump (TetR/AcrR family) or its homolog is increased preferably, an increase of the respective fine chemical around between 35% and 366% or more is conferred.
In one embodiment, in case the activity of the Escherichia coli K12 protein b1343 or its homologs, e.g. as indicated in Table II, columns 5 or 7, line 335, is increased, e.g. the activity of a protein involved in rRNA processing and/or translation is increased, preferred the activity of a ATP-dependent RNA helicase, stimulated by 23S rRNA or its homolog is increased. Preferably, an increase of the respective fine chemical around between 38% and 51% or more is conferred.
In one embodiment, in case the activity of the Escherichia coli K12 protein b2414 or its homologs, e.g. as indicated in Table II, columns 5 or 7, line 336, is increased, e.g. the activity of a protein of the threonine dehydratase-superfamily is increased preferably the activity of a protein involved in amino acid biosynthesis, biosynthesis of the cysteine-aromatic group, degradation of amino acids of the cysteine-aromatic group, nitrogen and sulfur utilizationbiosynthesis of the aspartate family, degradation of amino acids of the aspartate group, biosynthesis of sulfuric acid and L-cysteine derivatives, biosynthesis of secondary products derived from primary amino acids, biosynthesis of secondary products derived from glycine, L-serine and L-alanine, pyridoxal phosphate binding is increased, preferred the activity of a subunit of cysteine synthase A and O-acetylserine sulfhydrolase A, PLP-dependent enzyme or its homolog is increased. Preferably, an increase of the respective fine chemical around between 37% and 75% or more is conferred.
In one embodiment, in case the activity of the Escherichia coli K12 protein b2762 or its homologs, e.g. as indicated in Table II, columns 5 or 7, line 337, is increased, e.g. the activity of a 3'-phosphoadenosine 5'-phosphosulfate reductase -superfamily-protein is increased, preferably, the activity a protein involved in C-compound and carbohydrate metabolism, C-compound and carbohydrate utilization, ENERGY, glycolysis and gluconeogenesis, plastid, and/or photosynthesis is increased, more preferred the activity of a fructose-1,6-bisphosphatase or its homolog is increased. Preferably, the increase of the fine chemical around 20% or more is conferred.

**[0046.0.0.0]** In one embodiment, in case the activity of the Saccharomyces cerevisiae protein YLR375W or its homologs is increased, preferably, an increase of the fine chemical and of shikimic acid is conferred.
In one embodiment, in case the activity of the Saccharomyces cerevisiae protein YBL015w or its homologs, e.g. an Ach1p, is increased, preferably, an increase of the fine chemical and of a further amino acid, e.g. alanine is conferred.
In one embodiment, in case the activity of the Saccharomyces cerevisiae protein YER173w or its homologs, e.g. a checkpoint protein, involved in the activation of the DNA damage and meiotic pychtene checkpoints; subunit of a clamp loader that loads Rad17p-Mec3p-Ddc1p onto DNA or Rad24p or its homologs, e.g. the human or S. pombe Rad17 is increased, preferably, an increase of the fine chemical and of a further amino acid, e.g. leucine, is conferred.
In one embodiment, in case the activity of the Saccharomyces cerevisiae protein YOR084w or a putative lipase of the peroxisomal matrix or its homologs is increased, preferably, an increase of the fine chemical and of beta-sitosterol is conferred.
In one embodiment, in case the activity of the Escherichia coli K12 protein b1829 or its homologs is increased, e.g. the activity of a protease is increased, preferably, the activity of a heat shock protein is increased, more preferred the activity of a htpX protein or its homolog is increased, preferably, an increase of the fine chemical and of a further amino acid, e.g. phenylalanine, is conferred.
In one embodiment, in case the activity of the Escherichia coli K12 protein b0464 or its homologs is increased, e.g. the activity of a transcriptional repressor for multidrug efflux pump (TetR/AcrR family) or its homolog is increased, preferably in an increase of the fine chemical and of a further amino acid is conferred.
In one embodiment, in case the activity of the Escherichia coli K12 protein b1343 or its homologs is increased, e.g. the activity of a ATP-dependent RNA helicase, stimulated by 23S rRNA is increased or its homolog is increased, preferably, an increase of the fine chemical and of a further amino acid is conferred.
In one embodiment, in case the activity of the Escherichia coli K12 protein b2414 or its homologs is increased, e.g. the activity of a subunit of cysteine synthase A and O-acetylserine sulfhydrolase A, PLP-dependent enzyme is increased.
In one embodiment, in case the activity of the Escherichia coli K12 protein b2762 or its homologs is increased, e.g. the activity of a 3'-phosphoadenosine 5'-phosphosulfate (PAPS) reductase or its homolog is increased, preferably, an increase of the fine chemical and of a further amino acid is conferred.
In one embodiment, in case the activity of the Escherichia coli K12 protein b4232 or its homologs is increased, e.g. the activity of a ructose-1,6-bisphosphatase or its homolog is increased, preferably, an increase of the fine chemical and of a further amino acid is conferred.

**[0047.0.0.0]** In this context, the respective fine chemical amount in a cell, preferably in a tissue, more preferred in a organism as a plant or a microorganism or part thereof, is increased by 3% or more, especially preferably are 10% or more, very especially preferably are more than 30% and most preferably are 70% or more, such as 100%, 300% or 500%.

**[0048.0.0.0]** The respective fine chemical can be contained in the organism either in its free form and/or bound to proteins or polypeptides or mixtures thereof. Accordingly, in one embodiment, the amount of the free form in a cell, preferably in a tissue, more preferred in a organism as a plant or a microorganism or part thereof, is increased by 3% or more, especially preferably are 10% or more, very especially preferably are more than 30% and most preferably are 70% or more, such as 100%, 300% or 500%. Accordingly, in an other embodiment, the amount of the bound the respective fine chemical in a cell, preferably in a tissue, more preferred in a organism as a plant or a microorganism or part thereof, is increased by 3% or more, especially preferably are 10% or more, very especially preferably are more than 30% and most preferably are 70% or more, such as 100%, 300% or 500%.

**[0049.0.0.0]** A protein having an activity conferring an increase in the amount or level of the respective fine chemical preferably has the structure of the polypeptide described herein, in particular of a polypeptides comprising a consensus sequence as indicated in Table IV, columns 7, line 1 to 5 or lines 334 to 338 or of a polypeptide as indicated in Table II, columns 5 or 7, line 1 to 5 or lines 334 to 338 or the functional homologues thereof as described herein, or of a polypeptide which is encoded by the nucleic acid molecule characterized herein or the nucleic acid molecule according to the invention, for example by a nucleic acid molecule as indicated in Table I, columns 5 or 7, line 1 to 5 or lines 334 to 338 or its herein described functional homologues and has the herein mentioned activity.

**[0050.0.0.0]** For the purposes of the present invention, the terms "L-methionine", "methionine", "homocysteine", "S-adenosylmethionine" and "threonine" also encompass the corresponding salts, such as, for example, methionine hydrochloride or methionine sulfate. Preferably the terms methionine or threonine are intended to encompass the terms L-methionine or L-threonine.

**[0051.0.0.0]** Owing to the biological activity of the proteins which are used in the process according to the invention and which are encoded by nucleic acid molecules according to the invention, it is possible to produce compositions comprising the respective fine chemical, i.e. an increased amount of the free chemical free or bound, e.g. fine chemical compositions. Depending on the choice of the organism used for the process according to the present invention, for example a microorganism or a plant, compositions or mixtures of various fine chemicals, e.g. comprising further distinct amino acids, fatty acids, vitamins, hormones, sugars, lipids, etc. can be produced.

**[0052.0.0.0]** The term "expression" refers to the transcription and/or translation of a codogenic gene segment or gene. As a rule, the resulting product is an mRNA or a protein. However, expression products can also include functional RNAs such as, for example, antisense, nucleic acids, tRNAs, snRNAs, rRNAs, RNAi, siRNA, ribozymes etc. Expression may be systemic, local or temporal, for example limited to certain cell types, tissues organs or time periods.

**[0053.0.0.0]** In one embodiment, the process of the present invention comprises one or more of the following steps:
a) stabilizing a protein conferring the increased expression of a protein encoded by the nucleic acid molecule of the invention or of the polypeptid of the invention or the nucleic acid molecule or the polypeptide used in the method of the invention, e.g. of a polypeptide having an activity of a protein as indicated in Table II, column 3, lines 1 to 5 or lines 334 to 338 or its homologs activity, e.g. as indicated in Table II, columns 5 or 7, lines 1 to 5 or lines 334 to 338, having herein-mentioned the fine chemical-increasing activity;
b) stabilizing a mRNA conferring the increased expression of a protein encoded by the nucleic acid molecule of the invention or the nucleic acid molecule used in the method of the invention, e.g. of a polypeptide having an activity of a protein as indicated in Table II, column 3, lines 1 to 5 or lines 334 to 338 or its homologs activity, e.g. as indicated in Table II, columns 5 or 7, lines 1 to 5 or lines 334 to 338, or of a mRNA encoding the polypeptide of the present invention having herein-mentioned methionine increasing activity;
c) increasing the specific activity of a protein conferring the increased expression of a protein encoded by the nucleic acid molecule of the invention or of the polypeptide of the present invention or the nucleic acid molecule or polypeptide used in the method of the invention, having herein-mentioned methionine increasing activity, e.g. of a polypeptide having an activity of a protein as indicated in Table II, column 3, line 1 to 5 or lines 334 to 338, or its homologs activity, e.g. as indicated in Table II, columns 5 or 7, line 1 to 5 or lines 334 to 338, or decreasing the inhibitory regulation of the polypeptide of the invention or the polypeptide used in the method of the invention;
d) generating or increasing the expression of an endogenous or artificial transcription factor mediating the expression of a protein conferring the increased expression of a protein encoded by the nucleic acid molecule of the invention or the nucleic acid molecule used in the method of the invention or of the polypeptide of the invention or the polypeptide used in the method of the invention having herein-mentioned methionine increasing activity, e.g. of a polypeptide having an activity of a protein as indicated in Table II, column 3, line 1 to 5 or lines 334 to 338, or its homologs activity, e.g. as indicated in Table II, columns 5 or 7, line 1 to 5 or lines 334 to 338,;
e) stimulating activity of a protein conferring the increased expression of a protein encoded by the nucleic acid molecule of the present invention or a polypeptide of the present invention having herein-mentioned methionine increasing activity, e.g. of a polypeptide having an activity of a protein as indicated in Table II, column 3, lines 1 to 5 or lines 334 to 338, or its homologs activity, e.g. as indicated in Table II, columns 5 or 7, lines 1 to 5 or lines 334 to 338, by adding one or more exogenous inducing factors to the organism or parts thereof;
f) expressing a transgenic gene encoding a protein conferring the increased expression of a polypeptide encoded by the nucleic acid molecule of the present invention or a polypeptide of the present invention, having herein-mentioned methionine increasing activity, e.g. of a polypeptide having an activity of a protein as indicated in Table II, column 3, lines 1 to 5 or lines 334 to 338, or its homologs activity, e.g. as indicated in Table II, columns 5 or 7, lines 1 to 5 or lines 334 to 338;
g) increasing the copy number of a gene conferring the increased expression of a nucleic acid molecule encoding a polypeptide encoded by the nucleic acid molecule of the invention or the nucleic acid molecule used in the method of the invention or the polypeptide of the invention or the polypeptide used in the method of the invention having herein-mentioned methionine increasing activity, e.g. of a polypeptide having an activity of a protein as indicated in Table II, column 3, lines 1 to 5 or lines 334 to 338, or its homologs activity, e.g. as indicated in Table II, columns 5 or 7, lines 1 to 5 or lines 334 to 338;
h) Increasing the expression of the endogenous gene encoding the polypeptide of the invention or the polypeptide used in the method of the invention, e.g. a polypeptide having an activity of a protein as indicated in Table II, column 3, line 1 to 5 or lines 334 to 338, or its homologs activity, e.g. as indicated in Table II, columns 5 or 7, line 1 to 5 or lines 334 to 338, by adding positive expression or removing negative expression elements, e.g. homologous recombination can be used to either introduce positive regulatory elements like for plants the 35S enhancer into the promoter or to remove repressor elements form regulatory regions. Further gene conversion methods can be used to disrupt repressor elements or to enhance to activity of positive elements. Positive elements can be randomly introduced in plants by T-DNA or transposon mutagenesis and lines can be identified in which the positive elements have be integrated near to a gene of the invention, the expression of which is thereby enhanced;
i) Modulating growth conditions of an organism in such a manner, that the expression or activity of the gene encoding the protein of the invention or the protein itself is enhanced for example microorganisms or plants can be grown under a higher temperature regime leading to an enhanced expression of heat shock proteins, e.g. the heat shock protein of the invention, which can lead an enhanced the fine chemical production; and/or
j) selecting of organisms with especially high activity of the proteins of the invention from natural or from mutagenized resources and breeding them into the target organisms, e.g. the elite crops.

**[0054.0.0.0]** Preferably, said mRNA is the nucleic acid molecule of the present invention and/or the protein conferring the increased expression of a protein encoded by the nucleic acid molecule of the present invention or the polypeptide having the herein mentioned activity is the polypeptide of the present invention, e.g. conferring the increase of methionine after increasing the expression or activity of the encoded polypeptide or having the activity of a polypeptide having an activity of a protein as indicated in Table II, columns 5 or 7, lines 1 to 5 and/or lines 334 to 338 or its homologs activity, e.g. as indicated in Table II, columns 5 or 7, lines 1 to 5 and/or lines 334 to 338,.

**[0055.0.0.0]** In general, the amount of mRNA or polypeptide in a cell or a compartment of a organism correlates with the amount of encoded protein and thus with the overall activity of the encoded protein in said volume. Said correlation is not always linear, the activity in the volume is dependent on the stability of the molecules or the presence of activating or inhibiting co-factors. Further, product and educt inhibitions of enzymes are well known and described in Textbooks, e.g. Stryer, Biochemistry.

**[0056.0.0.0]** In general, the amount of mRNA, polynucleotide or nucleic acid molecule in a cell or a compartment of an organism correlates with the amount of encoded protein and thus with the overall activity of the encoded protein in said volume. Said correlation is not always linear, the activity in the volume is dependent on the stability of the molecules, the degradation of the molecules or the presence of activating or inhibiting co-factors. Further, product and educt inhibitions of enzymes are well known, e.g. Zinser et al. "Enzyminhibitoren"/Enzyme inhibitors".

**[0057.0.0.0]** The activity of the abovementioned proteins and/or polypeptide encoded by the nucleic acid molecule of the present invention can be increased in various ways. For example, the activity in an organism or in a part thereof, like a cell, is increased via increasing the gene product number, e.g. by increasing the expression rate, like introducing a stronger promoter, or by increasing the stability of the mRNA expressed, thus increasing the translation rate, and/or increasing the stability of the gene product, thus reducing the proteins decayed. Further, the activity or turnover of enzymes can be influenced in such a way that a reduction or increase of the reaction rate or a modification (reduction or increase) of the affinity to the substrate results, is reached. A mutation in the catalytic centre of an polypeptide of the invention or the polypeptide used in the method of the invention, e.g. as enzyme, can modulate the turn over rate of the enzyme, e.g. a knock out of an essential amino acid can lead to a reduced or completely knock out activity of the enzyme, or the deletion or mutation of regulator binding sites can reduce a negative regulation like a feedback inhibition (or a substrate inhibition, if the substrate level is also increased). The specific activity of an enzyme of the present invention can be increased such that the turn over rate is increased or the binding of a co-factor is improved. Improving the stability of the encoding mRNA or the protein can also increase the activity of a gene product. The stimulation of the activity is also under the scope of the term "increased activity".

**[0058.0.0.0]** Moreover, the regulation of the abovementioned nucleic acid sequences may be modified so that gene expression is increased. This can be achieved advantageously by means of heterologous regulatory sequences or by modifying, for example mutating, the natural regulatory sequences which are present. The advantageous methods may also be combined with each other.

**[0059.0.0.0]** In general, an activity of a gene product in an organism or part thereof, in particular in a plant cell, a plant, or a plant tissue or a part thereof or in a microorganism can be increased by increasing the amount of the specific encoding mRNA or the corresponding protein in said organism or part thereof. "Amount of protein or mRNA" is understood as meaning the molecule number of polypeptides or mRNA molecules in an organism, a tissue, a cell, or a cell compartment. "Increase" in the amount of a protein means the quantitative increase of the molecule number of said protein in an organism, a tissue, a cell or a cell compartment or part thereof - for example by one of the methods described herein below - in comparison to a wild type, control or reference.

**[0060.0.0.0]** The increase in molecule number amounts preferably to at least 1%, preferably to more than 10%, more preferably to 30% or more, especially preferably to 50%, 70% or more, very especially preferably to 100%, most preferably to 500% or more. However, a de novo expression is also regarded as subject of the present invention.

**[0061.0.0.0]** A modification, i.e. an increase or decrease, can be caused by endogenous or exogenous factors. For example, an increase in activity in an organism or a part thereof can be caused by adding a gene product or a precursor or an activator or an agonist to the media or nutrition or can be caused by introducing said subjects into a organism, transient or stable.

**[0062.0.0.0]** In one embodiment the increase in the amount of the fine chemical in the organism or a part thereof, e.g. in a cell, a tissue, a organ, an organelle etc., is achieved by increasing the endogenous level of the polypeptide of the invention or the polypeptide used in the method of the invention. Accordingly, in an embodiment of the present invention, the present invention relates to a process wherein the gene copy number of a gene encoding the polynucleotide or nucleic acid molecule of the invention or the nucleic acid molecule used in the method of the invention as herein described is increased. Further, the endogenous level of the polypeptide of the invention or the polypeptide used in the method of the invention as described can for example be increased by modifying the transcriptional or translational regulation of the polypeptide.

**[0063.0.0.0]** In one embodiment the amount of the fine chemical in the organism or part thereof can be increase by targeted or random mutagenesis of the endogenous genes of the invention. For example homologous recombination can be used to either introduce positive regulatory elements like for plants the 35S enhancer into the promoter or to remove repressor elements form regulatory regions. In addition gene conversion like methods described by Kochevenko and Willmitzer (Plant Physiol. 2003 May; 132(1): 174-84) and citations therein can be used to disrupt repressor elements or to enhance to activity of positive regulatory elements.
Furthermore positive elements can be randomly introduced in (plant) genomes by T-DNA or transposon mutagenesis and lines can be screened for, in which the positive elements has be integrated near to a gene of the invention, the expression of which is thereby enhanced. The activation of plant genes by random integrations of enhancer elements has been described by Hayashi et al., 1992 (Science 258:1350-1353) or Weigel et al., 2000 (Plant Physiol. 122, 1003-1013) and others citied therein.
Reverse genetic strategies to identify insertions (which eventually carrying the activation elements) near in genes of interest have been described for various cases e.g. Krysan et al., 1999 (Plant Cell 1999, 11, 2283-2290); Sessions et al., 2002 (Plant Cell 2002, 14, 2985-2994); Young et al., 2001, (Plant Physiol. 2001, 125, 513-518); Koprek et al., 2000 (Plant J. 2000, 24, 253-263) ; Jeon et al., 2000 (Plant J. 2000, 22, 561-570) ; Tissier et al., 1999 (Plant Cell 1999, 11, 1841-1852); Speulmann et al., 1999 (Plant Cell 1999,11 , 1853-1866). Briefly material from all plants of a large T-DNA or transposon mutagenized plant population is harvested and genomic DNA prepared. Then the genomic DNA is pooled following specific architectures as described for example in Krysan et al., 1999 (Plant Cell 1999, 11, 2283-2290). Pools of genomics DNAs are then screened by specific multiplex PCR reactions detecting the combination of the insertional mutagen (e.g. T-DNA or Transposon) and the gene of interest. Therefore PCR reactions are run on the DNA pools with specific combinations of T-DNA or transposon border primers and gene specific primers. General rules for primer design can again be taken from Krysan et al., 1999 (Plant Cell 1999, 11, 2283-2290) Rescreening of lower levels DNA pools lead to the identification of individual plants in which the gene of interest is disrupted by the insertional mutagen.
The enhancement of positive regulatory elements or the disruption or weaking of negative regulatory elements can also be achieved through common mutagenesis techniques: The production of chemically or radiation mutated populations is a common technique and known to the skilled worker. Methods for plants are described by Koorneef et al. 1982 and the citations therein and by Lightner and Caspar in "Methods in Molecular Biology" Vol 82. These techniques usually induce pointmutations that can be identified in any known gene using methods such as tilling (Colbert et al. 2001).
Accordingly, the expression level can be increased if the endogenous genes encoding a polypeptide conferring an increased expression of the polypeptide of the present invention, in particular genes comprising the nucleic acid molecule of the present invention, are modified via homologous recombination, tilling approaches or gene conversion

**[0064.0.0.0]** Regulatory sequences can be operatively linked to the coding region of an endogenous protein and control its transcription and translation or the stability or decay of the encoding mRNA or the expressed protein. In order to modify and control the expression, promoter, UTRs, splicing sites, processing signals, polyadenylation sites, terminators, enhancers, repressors, post transcriptional or posttransiational modification sites can be changed, added or amended for example, the activation of plant genes by random integrations of enhancer elements has been described by Hayashi et al., 1992 (Science 258:1350-1353) or Weigel et al., 2000 (Plant Physiol. 122, 1003-1013) and others citied therein. For example, the expression level of the endogenous protein can be modulated by replacing the endogenous promoter with a stronger transgenic promoter or by replacing the endogenous 3'UTR with a 3'UTR, which provides more stability without amending the coding region. Further, the transcriptional regulation can be modulated by introduction of an artificial transcription factor as described in the examples. Alternative promoters, terminators and UTR are described below.

**[0065.0.0.0]** The activation of an endogenous polypeptide having above-mentioned activity, of the polypeptide of the invention or the polypeptide used in the method of the invention, e.g. conferring the increase of the respective fine chemical after increase of expression or activity can also be increased by introducing a synthetic transcription factor, which binds close to the coding region of an endogenous polypeptide of the invention or the polypeptide used in the method of the invention- or used in the process of the invention or its endogenous homolog -encoding gene and the synthetic transcription factor activates its transcription. A chimeric zinc finger protein can be construed, which comprises a specific DNA-binding domain and an activation domain as e.g. the VP16 domain of Herpes Simplex virus. The specific binding domain can bind to the regulatory region of the endogenous protein coding region. The expression of the chimeric transcription factor in a organism, in particular in a plant, leads to a specific expression of an endogenous polypeptid of the invention or used in the process of the invention, in particular a plant homolog thereof, see e.g. in WO01/52620, Oriz, Proc. Natl. Acad. Sci. USA, 2002, Vol. 99, 13290 or Guan, Proc. Natl. Acad. Sci. USA, 2002, Vol. 99, 13296.

**[0066.0.0.0]** In one further embodiment of the process according to the invention, organisms are used in which one of the abovementioned genes, or one of the abovementioned nucleic acids, is mutated in a way that the activity of the encoded gene products is less influenced by cellular factors, or not at all, in comparison with the unmutated proteins. For example, well known regulation mechanism of enzymic activity are substrate inhibition or feed back regulation mechanisms. Ways and techniques for the introduction of substitutions, deletions and additions of one or more bases, nucleotides or amino acids of a corresponding sequence are described herein below in the corresponding paragraphs and the references listed there, e.g. in Sambrook et al., Molecular Cloning, Cold Spring Habour, NY, 1989. The person skilled in the art will be able to identify regulation domains and binding sites of regulators by comparing the sequence of the nucleic acid molecule of the present invention or the expression product thereof with the state of the art by computer software means which comprise algorithms for the identifying of binding sites and regulation domains or by introducing into a nucleic acid molecule or in a protein systematically mutations and assaying for those mutations which will lead to an increased specific activity or an increased activity per volume, in particular per cell.

**[0067.0.0.0]** It is therefore advantageously to express in an organism a nucleic acid molecule of the invention or the nucleic acid molecule used in the method of the invention or a polypeptide of the invention or the polypeptide used in the method of the invention derived from a evolutionary distantly related organism, as e.g. using a prokaryotic gene in an eukaryotic host, as in these cases the regulation mechanism of the host cell may not weaken the activity (cellular or specific) of the gene or its expression product

**[0068.0.0.0]** The mutation is introduced in such a way that the production of the amino acids is not adversely affected.

**[0069.0.0.0]** Less influence on the regulation of a gene or its gene product is understood as meaning a reduced regulation of the enzymatic activity leading to an increased specific or cellular activity of the gene or its product. An increase of the enzymatic activity is understood as meaning an enzymatic activity, which is increased by at least 10%, advantageously at least 20, 30 or 40%, especially advantageously by at least 50, 60 or 70% in comparison with the starting organism. This leads to an increased productivity of the desired respective fine chemical(s).

**[0070.0.0.0]** Owing to the introduction of a gene or a plurality of genes conferring the expression of the nucleic acid molecule of the invention or the nucleic acid molecule used in the method of the invention or the polypeptide of the invention or the polypeptide used in the method of the invention as described below, for example the nucleic acid construct mentioned below, into an organism alone or in combination with other genes, it is possible not only to increase the biosynthetic flux towards the end product, but also to increase, modify or create *de novo* an advantageous, preferably novel metabolites composition in the organism, e.g. an advantageous amino acid composition comprising a higher content of (from a viewpoint of nutrional physiology limited) respective fine chemicals, in particular amino acids, likewise the fine chemical.

**[0071.0.0.0]** Preferably the composition further comprises higher amounts of metabolites positively affecting or lower amounts of metabolites negatively affecting the nutrition or health of animals or humans provided with said compositions or organisms of the invention or parts thereof. Likewise, the number or activity of further genes which are required for the import or export of nutrients or metabolites, including amino acids or its precursors, required for the cell's biosynthesis of amino acids may be increased so that the concentration of necessary or relevant precursors, cofactors or intermediates within the cell(s) or within the corresponding storage compartments is increased. Owing to the increased or novel generated activity of the polypeptide of the invention or the polypeptide used in the method of the invention or owing to the increased number of nucleic acid sequences of the invention and/or to the modulation of further genes which are involved in the biosynthesis of the amino acids, e.g. by increasing the activity of enzymes synthesizing precursors or by destroying the activity of one or more genes which are involved in the breakdown of the amino acids, it is possible to increase the yield, production and/or production efficiency of amino acids in the host organism, such as the plants or the microorganisms.

**[0072.0.0.0]** By influencing the metabolism thus, it is possible to produce, in the process according to the invention, further advantageous sulfur-containing compounds, which contain at least one sulfur atom bound covalently. Examples of such compounds are, in addition to methionine, homocysteine, S-adenosylmethionine, cysteine, advantageously methionine and S-adenosylmethionine.

**[0073.0.0.0]** Accordingly, in one embodiment, the process according to the invention relates to a process which comprises:
a) providing a non-human organism, preferably a microorganism, a non-human animal, a plant or animal cell, a plant or animal tissue or a plant;
b) increasing an activity of a polypeptide of the invention or the polypeptide used in the method of the invention or a homolog thereof, e.g. as indicated in Table II, columns 5 or 7, line 1 to 5, or of a polypeptide being encoded by the nucleic acid molecule of the present invention and described below, i.e. conferring an increase of the respective fine chemical in the organism, preferably in a microorganism, a non-human animal, a plant or animal cell, a plant or animal tissue or a plant,
c) growing the organism, preferably a microorganism, a non-human animal, a plant or animal cell, a plant or animal tissue or a plant, under conditions which permit the production of the respective fine chemical in the organism, preferably the microorganism, the plant cell, the plant tissue or the plant; and
d) if desired, recovering, optionally isolating, the free and/or bound the respective fine chemical and , optionally further free and/or bound amino acids synthesized by the organism, the microorganism, the non-human animal, the plant or animal cell, the plant or animal tissue or the plant.

**[0074.0.0.0]** The organism, in particular the microorganism, non-human animal, the plant or animal cell, the plant or animal tissue or the plant is advantageously grown in such a way that it is not only possible to recover, if desired isolate the free or bound the respective fine chemical or the free and bound the fine chemical but as option it is also possible to produce, recover and, if desired isolate, other free or/and bound amino acids, in particular lysine. Galili et al., Transgenic Res., 200, 9, 2, 137-144 describes that the heterologous expression of a bacterial gene for the amino acid biosynthesis confers the increase of free as well as of protein-bound amino acids.

**[0075.0.0.0]** After the above-described increasing (which as defined above also encompasses the generating of an activity in an organism, i.e. a *de novo* activity), for example after the introduction and the expression of the nucleic acid molecules of the invention or described in the methods or processes according to the invention, the organism according to the invention, advantageously, a microorganism, a non-human animal, a plant, plant or animal tissue or plant or animal cell, is grown and subsequently harvested.

**[0076.0.0.0]** Suitable organisms or host organisms (transgenic organism) for the nucleic acid molecule used according to the invention and for the inventive process, the nucleic acid construct or the vector (both as described below) are, in principle, all organisms which are capable of synthesizing the respective fine chemical, and which are suitable for the activation, introduction or stimulation genes. Examples which may be mentioned are plants, microorganisms such as fungi, bacteria, yeasts, alga or diatom, transgenic or obtained by site directed mutagenesis or random mutagenesis combined with specific selection procedures. Preferred organisms are those which are naturally capable of synthesizing the respective fine chemical in substantial amounts, like fungi, yeasts, bactria or plants. In principle, transgenic animals, for example Caenorhabditis elegans, are also suitable as host organisms.

**[0077.0.0.0]** In the event that the transgenic organism is a microorganism, such as a eukaryotic organism, for example a fungus, an alga, diatom or a yeast in particular a fungus, alga, diatom or yeast selected from the families Chaetomiaceae, Choanephoraceae, Cryptococcaceae, Cunninghamellaceae, Demetiaceae, Moniliaceae, Mortierellaceae, Mucoraceae, Pythiaceae, Sacharomycetaceae, Saprolegniaceae, Schizosacharomycetaceae, Sodariaceae, Sporobolomycetaceae Tuberculariaceae, Adelotheciaceae, Dinophyceae, Ditrichaceae or Prasinophyceae, or a prokaryotic organism, for example a bacterium or blue alga, in particular a bacterium from the families Actinomycetaceae, Bacillaceae, Brevibacteriaceae, Corynebacteriaceae, Enterobacteriacae, Gordoniaceae, Nocardiaceae, Micrococcaceae, Mycobacteriaceae, Pseudomonaceae, Rhizobiaceae or Streptomycetaceae, this microorganism is grown on a solid or in a liquid medium which is known to the skilled worker and suits the organism. After the growing phase, the organisms can be harvested.

**[0078.0.0.0]** The microorganisms or the recovered, and if desired isolated, respective fine chemical can then be processed further directly into foodstuffs or animal feeds or for other applications, for example according to the disclosures made in EP-B-0 533 039 or EP-A-0 615 693, which are expressly incorporated herein by reference. The fermentation broth or fermentation products can be purified in the customary manner by extraction and precipitation or via ion exchangers and other methods known to the person skilled in the art and described herein below. Products of these different work-up procedures are amino acids or amino acid compositions which still comprise fermentation broth and cell components in different amounts, advantageously in the range of from 0 to 99% by weight, preferably below 80% by weight, especially preferably between below 50% by weight.

**[0079.0.0.0]** Preferred microorganisms are selected from the group consisting of Chaetomiaceae such as the genera Chaetomium e.g. the species *Chaetomidium fimeti;* Choanephoraceae such as the genera Blakeslea, Choanephora e.g. the species *Blakeslea trispora, Choanephora cucurbitarum or Choanephora infundibulifera* var. *cucurbitarum;* Cryptococcaceae such as the genera Candida, Crytococcus, Rhodotorula, Torulopsis e.g. the species *Candida albicans, Candida albomarginata, Candida antarctica, Candida bacarum, Candida bogoriensis, Candida boidinii, Candida bovina, Candida brumptii, Candida cacaoi, Candida cariosilignicola, Candida catenulata, Candida chalmersii, Candida ciferrii, Candida cylindracea, Candida edax, Candida ernobii, Candida famata, Candida freyschussii, Candida friedrichii, Candida glabrata, Candida guilliermondii, Candida haemulonii, Candida humicola, Candida inconspicua, Candida ingens, Candida intermedia, Candida kefyr, Candida krusei, Candida lactiscondensi, Candida lambica, Candida lipolytica, Candida lusitaniae, Candida macedoniensis, Candida magnoliae, Candida membranaefaciens, Candida mesenterica, Candida multigemmis, Candida mycoderma, Candida nemodendra, Candida nitratophila, Candida norvegensis, Candida norvegica, Candida parapsilosis, Candida pelliculosa, Candida peltata, Candida pini, Candida pseudotropicalis, Candida pulcherrima, Candida punicea, Candida pustula, Candida ravautii, Candida reukaufii, Candida rugosa, Candida sake, Candida silvicola, Candida solani, Candida sp., Candida spandovensis, Candida succiphila, Candida tropicalis, Candida utilis, Candida valida, Candida versatilis, Candida vini, Candida zeylanoides, Cryptococcus albidus, Cryptococcus curvatus, Cryptococcus flavus, Cryptococcus humicola, Cryptococcus hungaricus, Cryptococcus kuetzingii, Cryptococcus laurentii, Cryptococcus macerans, Cryptococcus neoformans, Cryptococcus terreus, Cryptococcus uniguttulatus, Rhodotorula acheniorum, Rhodotorula bacarum, Rhodotorula bogoriensis, Rhodotorula flava, Rhodotorula glutinis, Rhodotorula macerans, Rhodotorula minuta, Rhodotorula mucilaginosa, Rhodotorula pilimanae, Rhodotorula pustula, Rhodotorula rubra, Rhodotorula tokyoensis, Torulopsis colliculosa, Torulopsis dattila or Torulopsis neoformans;* Cunninghamellaceae such as the genera Cunninghamella e.g. the species *Cunninghamella blakesleeana, Cunninghamella echinulata, Cunninghamella echinulata* var. *elegans, Cunninghamella elegans or Cunninghamella homothallica;* Demetiaceae such as the genera Alternaria, Bipolaris, Cercospora, Chalara, Cladosporium, Curvularia, Exophilia, Helicosporium, Helminthosporium, Orbimyces, Philalophora, Pithomyces, Spilocaea, Thielaviopsis, Wangiella e.g. the species *Curvularia affinis, Curvularia clavata, Curvularia fallax, Curvularia inaequalis, Curvularia indica, Curvularia lunata, Curvularia pallescens, Curvularia verruculosa or Helminothosporium sp.;* Moniliaceae such as the genera Arthrobotrys, Aspergillus, Epidermophyton, Geotrichum, Gliocladium, Histoplasma, Microsporum, Monilia, Oedocephalum, Oidium, Penicillium, Trichoderma, Trichophyton, Thrichoteclum, Verticillium e.g. the species *Aspergillus aculeatus, Aspergillus albus, Aspergillus alliaceus, Aspergillus asperescens , Aspergillus awamori, Aspergillus candidus, Aspergillus carbonarius, Aspergillus carneus, Aspergillus chevalieri, Aspergillus chevalieri* var. *intermedius, Aspergillus clavatus, Aspergillus ficuum, Aspergillus flavipes, Aspergillus flavus, Aspergillus foetidus, Aspergillus fumigatus, Aspergillus giganteus, Aspergillus humicola, Aspergillus intermedius, Aspergillus japonicus, Aspergillus nidulans, Aspergillus niger, Aspergillus niveus, Aspergillus ochraceus, Aspergillus oryzae, Aspergillus ostianus, Aspergillus parasiticus, Aspergillus parasiticus* var. *globosus, Aspergillus penicillioides, Aspergillus phoenicis, Aspergillus rugulosus, Aspergillus sclerotiorum, Aspergillus sojae* var. *gymnosardae, Aspergillus sydowi, Aspergillus tamarii, Aspergillus terreus, Aspergillus terricola, Aspergillus toxicarius, Aspergillus unguis, Aspergillus ustus, Aspergillus versicolor, Aspergillus vitricolae, Aspergillus wentii,·Penicillium adametzi, ·Penicillium albicans, Penicillium arabicum, Penicillium arenicola, Penicillium argillaceum, Penicillium arvense, Penicillium asperosporum,·Penicillium aurantiogriseum,·Penicillium avellaneum,·Penicillium baarnense,·Penicillium bacillisporum,·Penicillium brasilianum,·Penicillium brevicompactum,·Penicillium camemberti,·Penicillium canadense,·Penicillium canescens,·Penicillium caperatum,·Penicillium capsulatum,·Penicillium caseicolum,·Penicillium chrysogenum,·Penicillium citreonigrum,·Penicillium citrinum,·Penicillium claviforme,·Penicillium commune,·Penicillium cory*/*ophilum,·Penicillium corymbiferum,·Penicillium crustosum,·Penicillium cyclopium,·Penicillium daleae,·Penicillium decumbens,·Penicillium dierckxii,·Penicillium digitatum,·Penicillium digitatum var. latum,·Penicillium divaricatum,·Penicillium diversum,·Penicillium duclauxii,·Penicillium echinosporum,·Penicillium expansum,·Penicillium fellutanum,·Penicillium frequentans,·Penicillium funiculosum,·Penicillium glabrum,·Penicillium gladioli,·Penicillium griseofulvum,·Penicillium hirsutum,·Penicillium hispanicum,·Penicillium islandicum,·Penicillium italicum,·Penicillium italicum var. avellaneum,·Penicillium janczewskii,·Penicillium janthinellum,·Penicillium japonicum,·Penicillium lavendulum,·Penicillium lilacinum,·Penicillium lividum,·Penicillium martensii, Penicillium megasporum,·Penicillium miczynskii,·Penicillium nalgiovense,·Penicillium nigricans,·Penicillium notatum,·Penicillium ochrochloron,·Penicillium odoratum,·Penicillium oxalicum,·Penicillium paraherquei,·Penicillium patulum,·Penicillium pinophilum,·Penicillium piscarium,·Penicillium pseudostromaticum,·Penicillium puberulum,·Penicillium purpurogenum,·Penicillium raciborskii,·Penicillium roqueforti,·Penicillium rotundum,·Penicillium rubrum,·Penicillium sacculum,·Penicillium simplicissimum, Penicillium sp., Penicillium spinulosum, Penicillium steckii, Penicillium stoloniferum, Penicillium striatisporum, Penicillium striatum, Penicillium tardum, Penicillium thomii, Penicillium turbatum, Penicillium variabile, Penicillium vermiculatum, Penicillium vermoesenii, Penicillium verrucosum, Penicillium verrucosum var. corymbiferum, Penicillium verrucosum var. cyclopium, Penicillium verruculosum, Penicillium vinaceum, Penicillium violaceum, Penicillium viridicatum, Penicillium vulpinum, Trichoderma hamatum, Trichoderma harzianum, Trichoderma koningii, Trichoderma longibrachiatum, Trichoderma polysporum, Trichoderma reesei, Trichoderma virens or Trichoderma viride;* Mortierellaceae such as the genera Mortierella e.g. the species *Mortierella isabellina, Mortierella polycephala, Mortierella ramanniana , Mortierella vinacea or Mortierella zonata;* Mucoraceae such as the genera Actinomucor, Mucor, Phycomyces, Rhizopus, Zygorhynchus e.g. the species *Mucor amphibiorum, Mucor circinelloides f. circinelloides, Mucor circinelloides var. griseocyanus, Mucor flavus, Mucor fuscus, Mucor griseocyanus, Mucor* heterosporus, *Mucor hiemalis, Mucor hiemalis f. hiemalis, Mucor inaequisporus, Mucor indicus, Mucor javanicus, Mucor mucedo, Mucor mucilagineus, Mucor piriformis, Mucor plasmaticus, Mucor plumbeus, Mucor* racemosus, *Mucor* racemosus *f*. racemosus, *Mucor racemosus f.* sphaerosporus, *Mucor rouxianus, Mucor rouxii, Mucor sinensis, Mucor sp., Mucor spinosus, Mucor tuberculisporus, Mucor variisporus, Mucor variosporus, Mucor wosnessenskii, Phycomyces blakesleeanus, Rhizopus achlamydosporus, Rhizopus arrhizus, Rhizopus chinensis, Rhizopus delemar, Rhizopus formosaensis*, *Rhizopus japonicus, Rhizopus javanicus, Rhizopus microsporus, Rhizopus microsporus var. chinensis, Rhizopus microsporus var. oligosporus, Rhizopus microsporus var. rhizopodiformis, Rhizopus nigricans, Rhizopus niveus, Rhizopus oligosporus, Rhizopus oryzae, Rhizopus pygmaeus, Rhizopus rhizopodiformis, Rhizopus semarangensis, Rhizopus sontii, Rhizopus stolonifer, Rhizopus thermosus, Rhizopus tonkinensis, Rhizopus tritici or Rhizopus usamii;* Pythiaceae such as the genera Phytium, Phytophthora e.g. the species *Pythium debaryanum, Pythium intermedium, Pythium irregulare, Pythium megalacanthum, Pythium paroecandrum, Pythium sylvaticum, Pythium ultimum, Phytophthora* cactorum, *Phytophthora cinnamomi, Phytophthora citricola, Phytophthora citrophthora, Phytophthora cryptogea, Phytophthora drechsleri, Phytophthora erythroseptica, Phytophthora lateralis, Phytophthora megasperma, Phytophthora nicotianae, Phytophthora nicotianae var. parasitica, Phytophthora palmivora, Phytophthora parasitica or Phytophthora syringae;* Sacharomycetaceae such as the genera Hansenula, Pichia, Saccharomyces, Saccharomycodes, Yarrowia e.g. the species *Hansenula* anomala, *Hansenula californica, Hansenula* canadensis, *Hansenula capsulata, Hansenula ciferrii, Hansenula glucozyma, Hansenula henricii, Hansenula holstii, Hansenula minuta, Hansenula nonfermentans, Hansenula philodendri, Hansenula polymorpha, Hansenula saturnus, Hansenula subpelliculosa, Hansenula wickerhamii, Hansenula wingei, Pichia alcoholophila, Pichia angusta, Pichia anomala, Pichia bispora, Pichia burtonii, Pichia canadensis*, *Pichia capsu*/*ata, Pichia carsonii, Pichia cellobiosa, Pichia ciferrii, Pichia farinosa, Pichia fermentans, Pichia finlandica, Pichia glucozyma, Pichia guilliermondii, Pichia haplophila, Pichia henricii, Pichia holstii, Pichia jadinii, Pichia lindnerii, Pichia membranaefaciens, Pichia methanolica, Pichia minuta var. minuta, Pichia minuta var. nonfermentans, Pichia norvegensis, Pichia ohmeri, Pichia* pastoris, *Pichia philodendri, Pichia pini, Pichia polymorpha, Pichia quercuum, Pichia rhodanensis, Pichia sargentensis, Pichia stipitis, Pichia strasburgensis, Pichia subpelliculosa, Pichia toletana, Pichia trehalophila, Pichia vini, Pichia xylosa, Saccharomyces aceti, Saccharomyces bailii, Saccharomyces bayanus, Saccharomyces bisporus, Saccharomyces capensis, Saccharomyces carlsbergensis, Saccharomyces cerevisiae, Saccharomyces cerevisiae var. ellipsoideus, Saccharomyces chevalieri, Saccharomyces delbrueckii, Saccharomyces diastaticus, Saccharomyces drosophilarum, Saccharomyces elegans, Saccharomyces ellipsoideus, Saccharomyces fermentati, Saccharomyces florentinus, Saccharomyces fragilis, Saccharomyces heterogenicus, Saccharomyces hienipiensis, Saccharomyces inusitatus, Saccharomyces italicus, Saccharomyces kluyveri, Saccharomyces krusei, Saccharomyces lactis, Saccharomyces marxianus, Saccharomyces microellipsoides, Saccharomyces montanus, Saccharomyces norbensis, Saccharomyces oleaceus, Saccharomyces paradoxus, Saccharomyces pastorianus, Saccharomyces pretoriensis, Saccharomyces rosei, Saccharomyces rouxii, Saccharomyces uvarum, Saccharomycodes ludwigii or Yarrowia lipolytica;* Saprolegniaceae such as the genera Saprolegnia e.g. the species *Saprolegnia ferax;* Schizosacharomycetaceae such as the genera Schizosaccharomyces e.g. the species *Schizosaccharomyces japonicus var. japonicus, Schizosaccharomyces japonicus var. versatilis, Schizosaccharomyces malidevorans, Schizosaccharomyces octosporus, Schizosaccharomyces pombe var. malidevorans or Schizosaccharomyces pombe var. pombe;* Sodariaceae such as the genera Neurospora, Sordaria e.g. the species *Neurospora africana, Neurospora crassa, Neurospora intermedia, Neurospora sitophila, Neurospora tetrasperma, Sordaria fimicola or Sordaria macrospora;* Tuberculariaceae such as the genera Epicoccum, Fusarium, Myrothecium, Sphacelia, Starkeyomyces, Tubercularia e.g. the species *Fusarium acuminatum, Fusarium anthophilum, Fusarium aquaeductuum, Fusarium aquaeductuum var. medium, Fusarium avenaceum, Fusarium buharicum, Fusarium camptoceras, Fusarium cerealis, Fusarium chiamydosporum, Fusarium ciliatum, Fusarium coccophilum, Fusarium coeruleum, Fusarium concolor, Fusarium crookwellense, Fusarium culmorum, Fusarium dimerum, Fusarium diversisporum, Fusarium equiseti, Fusarium equiseti var. bullatum, Fusarium eumartii, Fusarium flocciferum, Fusarium fujikuroi, Fusarium graminearum, Fusarium graminum, Fusarium heterosporum,Fusarium incarnatum, Fusarium inflexum, Fusarium javanicum, Fusarium lateritium, Fusarium lateritium var. majus, Fusarium longipes, Fusarium melanochlorum, Fusarium merismoides, Fusarium merismoides var. chlamydosporale, Fusarium moniliforme, Fusarium moniliforme var. anthophilum, Fusarium moniliforme var. subglutinans, Fusarium nivale, Fusarium nivale var. majus, Fusarium oxysporum, Fusarium oxysporum f. sp. aechmeae, Fusarium oxysporum f. sp. cepae, Fusarium oxysporum f. sp. conglutinans, Fusarium oxysporum f. sp. cucumerinum, Fusarium oxysporum f. sp. cyclaminis, Fusarium oxysporum f. sp. dianthi, Fusarium oxysporum f. sp. lycopersici, Fusarium oxysporum f. sp. melonis, Fusarium oxysporum f. sp. passiflorae, Fusarium oxysporum f. sp. pisi, Fusarium oxysporum f. sp. tracheiphilum, Fusarium oxysporum f. sp. tuberosi, Fusarium oxysporum f. sp. tulipae, Fusarium oxysporum f. sp. vasinfectum, Fusarium pallidoroseum, Fusarium poae, Fusarium proliferatum, Fusarium proliferatum var. minus, Fusarium redolens, Fusarium redolens f. sp. dianthi, Fusarium reticulatum, Fusarium roseum, Fusarium sacchari var. elongatum, Fusarium sambucinum, Fusarium sambucinum var. coeruleum, Fusarium semitectum, Fusarium semitectum var. majus, Fusarium solani, Fusarium solani f. sp. pisi, Fusarium sporotrichioides, Fusarium sporotrichioides var. minus, Fusarium sublunatum, Fusarium succisae, Fusarium sulphureum, Fusarium tabacinum, Fusarium tricinctum, Fusarium udum, Fusarium ventricosum, Fusarium verticillioides, Fusarium xylarioides or Fusarium zonatum;* Sporobolomycetaceae such as the genera Bullera, Sporobolomyces, Itersonilia e.g. the species *Sporobolomyces holsaticus, Sporobolomyces odorus, Sporobolomyces puniceus, Sporobolomyces salmonicolor, Sporobolomyces singularis or Sporobolomyces tsugae;* Adelotheciaceae such as the genera e.g. the species *Physcomitrella patens;* Dinophyceae such as the genera Crypthecodinium, Phaeodactylum e.g. the species *Crypthecodinium cohnii or Phaeodactylum tricornutum;* Ditrichaceae such as the genera Ceratodon, Pleuridium, Astomiopsis, Ditrichum, Philibertiella, Ceratodon, Distichium, Skottsbergia e.g. the species *Ceratodon antarcticus, Ceratodon purpureus, Ceratodon purpureus ssp. convolutes or Ceratodon purpureus ssp. stenocarpus;* Prasinophyceae such as the genera Nephroselmis, Prasinococcus, Scherffelia, Tetraselmis, Mantoniella, Ostreococcus e.g. the species *Nephroselmis olivacea, Prasinococcus capsulatus, Scherffelia dubia, Tetraselmis chui, Tetraselmis suecica, Mantoniella squamata or* Ostreococcus *tauri*; Actinomycetaceae such as the genera Actinomyces, Actinobaculum, Arcanobacterium, Mobiluncus e.g. the species *Actinomyces bernardiae, Actinomyces bovis, Actinomyces bowdenii, Actinomyces canis, Actinomyces cardiffensis, Actinomyces catuli, Actinomyces coleocanis, Actinomyces denticolens, Actinomyces* europaeus, *Actinomyces funkei, Actinomyces georgiae, Actinomyces gerencseriae, Actinomyces hordeovulneris, Actinomyces howellii, Actinomyces humiferus, Actinomyces hyovaginalis, Actinomyces israelii, Actinomyces marimammalium, Actinomyces meyeri, Actinomyces naeslundii, Actinomyces nasicola, Actinomyces neuii* subsp. *anitratus, Actinomyces neuii* subsp. *neuii, Actinomyces odontolyticus, Actinomyces oricola, Actinomyces pyogenes, Actinomyces radicidentis, Actinomyces radingae, Actinomyces slackii, Actinomyces suimastitidis, Actinomyces suis, Actinomyces turicensis, Actinomyces urogenitalis, Actinomyces vaccimaxillae, Actinomyces viscosus, Actinobaculum schaalii, Actinobaculum suis, Actinobaculum urinale, Arcanobacterium bernardiae, Arcanobacterium haemolyticum, Arcanobacterium hippocoleae, Arcanobacterium phocae, Arcanobacterium pluranimalium, Arcanobacterium pyogenes, Mobiluncus curtisii* subsp. *curtisii, Mobiluncus curtisii* subsp. *holmesii or Mobiluncus mulieris;* Bacillaceae such as the genera Amphibacillus, Anoxybacillus, Bacillus, Exiguobacterium, Gracilibacillus, Holobacillus, Saccharococcus, Salibacillus, Virgibacillus e.g. the species *Amphibacillus fermentum, Amphibacillus tropicus, Amphibacillus xylanus, Anoxybacillus flavithermus, Anoxybacillus gonensis, Anoxybacillus pushchinoensis, Bacillus acidocaldarius, Bacillus acidoterrestris, Bacillus aeolius, Bacillus agaradhaerens, Bacillus agri, Bacillus alcalophilus, Bacillus alginolyticus, Bacillus alvei, Bacillus amyloliquefaciens, Bacillus amylolyticus, Bacillus aneurinilyticus, Bacillus aquimaris, Bacillus arseniciselenatis, Bacillus atrophaeus, Bacillus azotofixans*, *Bacillus azotoformans, Bacillus badius, Bacillus barbaricus, Bacillus benzoevorans, Bacillus borstelensis, Bacillus brevis, Bacillus carboniphilus, Bacillus centrosporus, Bacillus cereus, Bacillus chitinolyticus, Bacillus chondroitinus, Bacillus choshinensis, Bacillus circulans, Bacillus clarkii, Bacillus clausii, Bacillus coagulans, Bacillus cohnii, Bacillus curdlanolyticus, Bacillus cycloheptanicus, Bacillus decolorationis, Bacillus dipsosauri, Bacillus edaphicus, Bacillus ehimensis, Bacillus endophyticus, Bacillus fastidiosus, Bacillus firmus, Bacillus flexus, Bacillus formosus, Bacillus fumarioli, Bacillus funiculus, Bacillus fusiformis, Bacillus sphaericus subsp. fusiformis, Bacillus galactophilus, Bacillus globisporus, Bacillus globisporus subsp*. *marinus, Bacillus glucanolyticus, Bacillus gordonae, Bacillus halmapalus, Bacillus haloalkaliphilus, Bacillus halodenitrificans, Bacillus halodurans, Bacillus halophilus, Bacillus horikoshii, Bacillus horti, Bacillus infernos, Bacillus insolitus, Bacillus jeotgali, Bacillus kaustophilus, Bacillus kobensis, Bacillus krulwichiae, Bacillus laevolacticus, Bacillus larvae, Bacillus laterosporus, Bacillus lautus, Bacillus lentimorbus, Bacillus lentus, Bacillus licheniformis, Bacillus luciferensis, Bacillus macerans, Bacillus macquariensis, Bacillus marinus, Bacillus marisflavi, Bacillus marismortui, Bacillus megaterium, Bacillus methanolicus, Bacillus migulanus, Bacillus mojavensis, Bacillus mucilaginosus, Bacillus mycoides, Bacillus naganoensis, Bacillus nealsonii, Bacillus neidei, Bacillus niacini, Bacillus okuhidensis, Bacillus oleronius, Bacillus pabuli, Bacillus pallidus, Bacillus pantothenticus, Bacillus parabrevis, Bacillus pasteurii, Bacillus peoriae, Bacillus polymyxa, Bacillus popilliae, Bacillus pseudalcaliphilus, Bacillus pseudofirmus, Bacillus pseudomycoides, Bacillus psychrodurans, Bacillus psychrophilus, Bacillus psychrosaccharolyticus, Bacillus psychrotolerans, Bacillus pulvifaciens, Bacillus pumilus, Bacillus pycnus, Bacillus reuszeri, Bacillus salexigens, Bacillus schlegelii, Bacillus selenitireducens, Bacillus silvestris, Bacillus simplex, Bacillus siralis, Bacillus smithii, Bacillus sonorensis, Bacillus sphaericus, Bacillus sporothermodurans, Bacillus stearothermophilus , Bacillus subterraneus, Bacillus subtilis subsp. spizizenii, Bacillus subtilis subsp. subtilis, Bacillus thermantarcticus, Bacillus thermoaerophilus, Bacillus thermoamylovorans, Bacillus thermoantarcticus, Bacillus thermocatenulatus, Bacillus thermocloacae, Bacillus thermodenitrificans, Bacillus thermoglucosidasius, Bacillus thermoleovorans, Bacillus thermoruber, Bacillus thermosphaericus, Bacillus thiaminolyticus, Bacillus thuringiensis, Bacillus tusciae, Bacillus validus, Bacillus vallismortis, Bacillus vedderi, Bacillus vulcani, Bacillus weihenstephanensis, Exiguobacterium acetylicum, Exiguobacterium antarcticum, Exiguobacterium aurantiacum, Exiguobacterium undae, Gracilibacillus dipsosauri, Gracilibacillus halotolerans, Halobacillus halophilus, Halobacillus karajensis, Halobacillus litoralis, Halobacillus salinus, Halobacillus trueperi, Saccharococcus caldoxylosilyticus, Saccharococcus thermophilus, Salibacillus marismortui, Salibacillus salexigens, Virgibacillus carmonensis, Virgibacillus marismortui, Virgibacillus necropolis, Virgibacillus pantothenticus, Virgibacillus picturae, Virgibacillus proomii or Virgibacillus salexigens,* Brevibacteriaceae such as the genera Brevibacterium e.g. the species *Brevibacterium acetylicum, Brevibacterium albidum, Brevibacterium ammoniagenes, Brevibacterium avium, Brevibacterium casei, Brevibacterium citreum, Brevibacterium divaricatum, Brevibacterium epidermidis, Brevibacterium fermentans, Brevibacterium frigoritolerans, Brevibacterium halotolerans, Brevibacterium imperiale, Brevibacterium incertum, Brevibacterium iodinum, Brevibacterium linens, Brevibacterium liquefaciens, Brevibacterium lutescens, Brevibacterium luteum, Brevibacterium lyticum, Brevibacterium mcbrellneri, Brevibacterium otitidis, Brevibacterium oxydans, Brevibacterium paucivorans, Brevibacterium protophormiae, Brevibacterium pusillum, Brevibacterium saperdae, Brevibacterium stationis, Brevibacterium testaceum or Brevibacterium vitaeruminis;* Corynebacteriaceae such as the genera Corynebacterium e.g. the species *Corynebacterium accolens, Corynebacterium afermentans* subsp. *afermentans, Corynebacterium afermentans* subsp. *lipophilum, Corynebacterium ammoniagenes, Corynebacterium amycolatum, Corynebacterium appendicis, Corynebacterium aquilae, Corynebacterium argentoratense, Corynebacterium atypicum, Corynebacterium aurimucosum, Corynebacterium auris, Corynebacterium auriscanis, Corynebacterium betae, Corynebacterium beticola, Corynebacterium bovis, Corynebacterium callunae, Corynebacterium camporealensis, Corynebacterium capitovis, Corynebacterium casei, Corynebacterium confusum, Corynebacterium coyleae*, *Corynebacterium cystitidis, Corynebacterium durum, Corynebacterium efficiens, Corynebacterium equi, Corynebacterium falsenii, Corynebacterium fascians, Corynebacterium felinum, Corynebacterium flaccumfaciens, Corynebacterium flavescens, Corynebacterium freneyi, Corynebacterium glaucum, Corynebacterium glucuronolyticum, Corynebacterium glutamicum, Corynebacterium hoagii, Corynebacterium ilicis, Corynebacterium imitans, Corynebacterium insidiosum, Corynebacterium iranicum, Corynebacterium jeikeium, Corynebacterium kroppenstedtii, Corynebacterium kutscheri, Corynebacterium lilium, Corynebacterium lipophiloflavum, Corynebacterium macginleyi, Corynebacterium mastitidis, Corynebacterium matruchotii, Corynebacterium michiganense, Corynebacterium michiganense* subsp. *tessellarius, Corynebacterium minutissimum, Corynebacterium mooreparkense, Corynebacterium mucifaciens, Corynebacterium mycetoides, Corynebacterium nebraskense, Corynebacterium oortii, Corynebacterium paurometabolum, Corynebacterium phocae, Corynebacterium pilosum, Corynebacterium poinsettiae, Corynebacterium propinquum, Corynebacterium pseudodiphtheriticum, Corynebacterium pseudotuberculosis, Corynebacterium pyogenes, Corynebacterium rathayi, Corynebacterium renale, Corynebacterium riegelii, Corynebacterium seminale, Corynebacterium sepedonicum, Corynebacterium simulans, Corynebacterium singulare, Corynebacterium sphenisci, Corynebacterium spheniscorum, Corynebacterium striatum, Corynebacterium suicordis, Corynebacterium sundsvallense, Corynebacterium terpenotabidum, Corynebacterium testudinoris, Corynebacterium thomssenii, Corynebacterium tritici, Corynebacterium ulcerans, Corynebacterium urealyticum, Corynebacterium variabile, Corynebacterium vitaeruminis or Corynebacterium* xerosis; Enterobacteriacae such as the genera Alterococcus, Arsenophonus, Brenneria, Buchnera, Budvicia, Buttiauxella, Calymmatobacterium, Cedecea, Citrobacter, Edwardsiella, Enterobacter, Erwinia, Escherichia, Ewingella, Hafnia, Klebsiella, Kluyvera, Leclercia, Leminorella, Moellerella, Morganella, Obesumbacterium, Pantoea, Pectobacterium, Photorhabdus, Plesiomonas, Pragia, Proteus, Providencia, Rahnella, Saccharobacter, Salmonella, Shigella, Serratia, Sodalis, Tatumella, Trabulsiella, Wigglesworthia, Xenorhabdus,Yersinia and Yokenella e.g. the species *Arsenophonus nasoniae, Brenneria alni, Brenneria nigrifluens, Brenneria quercina, Brenneria rubrifaciens, Brenneria salicis, Budvicia aquatica, Buttiauxella agrestis, Buttiauxella brennerae, Buttiauxella ferragutiae, Buttiauxella gaviniae, Buttiauxella izardii, Buttiauxella noackiae, Buttiauxella warmboldiae, Cedecea davisae, Cedecea lapagei, Cedecea neteri, Citrobacter amalonaticus, Citrobacter diversus, Citrobacter freundii, Citrobacter genomospecies, Citrobacter gillenii, Citrobacter intermedium, Citrobacter koseri, Citrobacter murliniae, Citrobacter sp., Edwardsiella hoshinae, Edwardsiella ictaluri, Edwardsiella tarda, Erwinia alni, Erwinia amylovora, Erwinia ananatis, Erwinia aphidicola, Erwinia billingiae, Erwinia cacticida, Erwinia cancerogena, Erwinia carnegieana, Erwinia carotovora* subsp. *atroseptica, Erwinia carotovora* subsp. *betavasculorum, Erwinia carotovora* subsp. *odorifera, Erwinia carotovora* subsp. *wasabiae, Erwinia chrysanthemi, Erwinia cypripedii, Erwinia dissolvens, Erwinia herbicola, Erwinia mallotivora, Erwinia milletiae, Erwinia nigrifluens, Erwinia nimipressuralis, Erwinia persicina, Erwinia psidii, Erwinia pyrifoliae, Erwinia quercina, Erwinia rhapontici, Erwinia rubrifaciens, Erwinia salicis, Erwinia stewartii, Erwinia tracheiphila, Erwinia uredovora, Escherichia adecarboxylata, Escherichia anindolica, Escherichia aurescens, Escherichia blattae, Escherichia coli, Escherichia coli var. communior, Escherichia coli-mutabile, Escherichia fergusonii, Escherichia hermannii, Escherichia sp., Escherichia vulneris, Ewingella americana, Hafnia alvei, Klebsiella aerogenes, Klebsiella edwardsii subsp. atlantae, Klebsiella ornithinolytica, Klebsiella oxytoca, Klebsiella planticola, Klebsiella pneumoniae, Klebsiella pneumoniae subsp. pneumoniae, Klebsiella sp., Klebsiella terrigena, Klebsiella trevisanii, Kluyvera ascorbata, Kluyvera citrophila, Kluyvera cochleae, Kluyvera cryocrescens, Kluyvera georgiana, Kluyvera noncitrophila, Kluyvera sp., Leclercia adecarboxylata, Leminorella grimontii, Leminorella richardii, Moellerella wisconsensis, Morganella morganii, Morganella morganii subsp. morganii, Morganella morganii subsp. sibonii, Obesumbaterium proteus, Pantoea agglomerans, Pantoea ananatis, Pantoea citrea, Pantoea dispersa, Pantoea punctata, Pantoea stewartii subsp. stewartii, Pantoea terrea, Pectobacterium atrosepticum, Pectobacterium carotovorum subsp. atrosepticum, Pectobacterium carotovorum subsp. carotovorum, Pectobacterium chrysanthemi, Pectobacterium cypripedii, Photorhabdus asymbiotica, Photorhabdus luminescens, Photorhabdus luminescens subsp. akhurstii, Photorhabdus luminescens subsp. laumondii, Photorhabdus luminescens subsp. luminescens, Photorhabdus sp., Photorhabdus temperata, Plesiomonas shigelloides, Pragia fontium, Proteus hauseri, Proteus ichthyosmius, Proteus inconstans, Proteus mirabilis, Proteus morganii, Proteus myxofaciens, Proteus penneri, Proteus rettgeri, Proteus shigelloides, Proteus vulgaris, Providencia alcalifaciens, Providencia friedericiana, Providencia heimbachae, Providencia rettgeri, Providencia rustigianii, Providencia stuartii, Rahnella aquatilis, Salmonella abony, Salmonella arizonae, Salmonella bongori, Salmonella choleraesuis subsp. arizonae, Salmonella choleraesuis subsp. bongori, Salmonella choleraesuis subsp. cholereasuis, Salmonella choleraesuis subsp. diarizonae, Salmonella choleraesuis subsp. houtenae, Salmonella choleraesuis subsp. indica, Salmonella choleraesuis subsp. salamae, Salmonella daressalaam, Salmonella enterica subsp. houtenae, Salmonella enterica subsp. salamae, Salmonella enteritidis, Salmonella gallinarum, Salmonella heidelberg, Salmonella panama, Salmonella senftenberg, Salmonella typhimurium, Serratia entomophila, Serratia ficaria, Serratia fonticola, Serratia grimesii, Serratia liquefaciens, Serratia marcescens, Serratia marcescens subsp. marcescens, Serratia marinorubra, Serratia odorifera, Serratia plymouthensis, Serratia plymuthica, Serratia proteamaculans, Serratia proteamaculans subsp. quinovora, Serratia quinivorans, Serratia rubidaea, Shigella boydii, Shigella flexneri, Shigella paradysenteriae, Shigella sonnei, Tatumella ptyseos, Xenorhabdus beddingii, Xenorhabdus bovienii, Xenorhabdus luminescens, Xenorhabdus nematophila, Xenorhabdus nematophila subsp. beddingii, Xenorhabdus nematophila subsp. bovienii, Xenorhabdus nematophila subsp. poinarii orXenorhabdus poinarii;* Gordoniaceae such as the genera Gordonia, Skermania e.g. the species *Gordonia aichiensis, Gordonia alkanivorans, Gordonia amarae, Gordonia amicalis, Gordonia bronchialis, Gordonia desulfuricans, Gordonia hirsuta, Gordonia hydrophobica, Gordonia namibiensis, Gordonia nitida, Gordonia paraffinivorans, Gordonia polyisoprenivorans, Gordonia rhizosphera, Gordonia rubripertincta, Gordonia sihwensis, Gordonia sinesedis, Gordonia sputi, Gordonia terrae or Gordonia westfalica;* Micrococcaceae such as the genera Micrococcus, Arthrobacter, Kocuria, Nesterenkonia, Renibacterium, Rothia, Stomatococcus e.g. the species *Micrococcus agilis, Micrococcus antarcticus, Micrococcus halobius, Micrococcus kristinae, Micrococcus luteus, Micrococcus lylae, Micrococcus nishinomiyaensis, Micrococcus roseus, Micrococcus sedentarius, Micrococcus varians, Arthrobacter agilis, Arthrobacter albus, Arthrobacter atrocyaneus, Arthrobacter aurescens, Arthrobacter chlorophenolicus, Arthrobacter citreus, Arthrobacter creatinolyticus, Arthrobacter crystallopoietes, Arthrobacter cumminsii, Arthrobacter duodecadis, Arthrobacter flavescens, Arthrobacter flavus, Arthrobacter gandavensis, Arthrobacter globiformis, Arthrobacter histidinolovorans, Arthrobacter ilicis, Arthrobacter koreensis, Arthrobacter luteolus, Arthrobacter methylotrophus, Arthrobacter mysorens, Arthrobacter nasiphocae, Arthrobacter nicotianae, Arthrobacter nicotinovorans, Arthrobacter oxydans, Arthrobacter pascens, Arthrobacter picolinophilus, Arthrobacter polychromogenes, Arthrobacter protophormiae, Arthrobacter psychrolactophilus, Arthrobacter radiotolerans, Arthrobacter ramosus, Arthrobacter rhombi, Arthrobacter roseus, Arthrobacter siderocapsulatus, Arthrobacter simplex, Arthrobacter sulfonivorans, Arthrobacter sulfureus, Arthrobacter terregens, Arthrobacter tumescens, Arthrobacter uratoxydans, Arthrobacter ureafaciens, Arthrobacter variabilis, Arthrobacter viscosus, Arthrobacter woluwensis, Kocuria erythromyxa, Kocuria kristinae, Kocuria palustris, Kocuria polaris, Kocuria rhizophila, Kocuria rosea, Kocuria varians, Nesterenkonia halobia, Nesterenkonia lacusekhoensis, Renibacterium salmoninarum, Rothia amarae, Rothia dentocariosa, Rothia mucilaginosa, Rothia nasimurium or Stomatococcus mucilaginosus;* Mycobacteriaceae such as the genera Mycobacterium e.g. the species *Mycobacterium africanum, Mycobacterium agri, Mycobacterium aichiense, Mycobacterium alvei, Mycobacterium asiaticum, Mycobacterium aurum, Mycobacterium austroafricanum, Mycobacterium bohemicum, Mycobacterium botniense, Mycobacterium brumae, Mycobacterium chelonae* subsp. *abscessus, Mycobacterium chitae, Mycobacterium chlorophenoficum, Mycobacterium chubuense, Mycobacterium confluentis, Mycobacterium cookii, Mycobacterium diernhoferi, Mycobacterium doricum, Mycobacterium duvalii, Mycobacterium fallax, Mycobacterium farcinogenes, Mycobacterium flavescens, Mycobacterium frederiksbergense, Mycobacterium gadium, Mycobacterium gilvum, Mycobacterium gordonae, Mycobacterium hassiacum, Mycobacterium hibemiae, Mycobacterium hodleri, Mycobacterium holsaticum, Mycobacterium komossense, Mycobacterium lacus, Mycobacterium madagascariense, Mycobacterium mageritense, Mycobacterium montefiorense, Mycobacterium moriokaense, Mycobacterium murale, Mycobacterium neoaurum, Mycobacterium nonchromogenicum, Mycobacterium obuense, Mycobacterium palustre, Mycobacterium parafortuitum, Mycobacterium peregrinum, Mycobacterium phlei, Mycobacterium pinnipedii, Mycobacterium poriferae, Mycobacterium pulveris, Mycobacterium rhodesiae, Mycobacterium shottsii, Mycobacterium sphagni, Mycobacterium terrae, Mycobacterium thermoresistibile, Mycobacterium tokaiense, Mycobacterium triviale, Mycobacterium tusciae or Mycobacterium vanbaalenii;* Nocardiaceae such as the genera Nocardia, Rhodococcus e.g. the species *Nocardia abscessus, Nocardia africana, Nocardia amarae, Nocardia asteroides, Nocardia autotrophica, Nocardia beijingensis, Nocardia brasiliensis, Nocardia brevicatena, Nocardia caishijiensis, Nocardia calcarea, Nocardia carnea, Nocardia cellulans, Nocardia cerradoensis, Nocardia coeliaca, Nocardia corynebacterioides, Nocardia crassostreae, Nocardia cummidelens, Nocardia cyriacigeorgica, Nocardia farcinica, Nocardia flavorosea, Nocardia fluminea, Nocardia globerula, Nocardia hydrocarbonoxydans, Nocardia ignorata, Nocardia mediterranei, Nocardia* nova, *Nocardia orientalis, Nocardia otitidis-caviarum, Nocardia otitidiscaviarum, Nocardia paucivorans, Nocardia petroleophila, Nocardia pinensis, Nocardia pseudobrasiliensis, Nocardia pseudovaccinii, Nocardia puris, Nocardia restricta, Nocardia* rugosa, *Nocardia salmonicida, Nocardia saturnea*, *Nocardia seriolae, Nocardia soli, Nocardia sulphurea, Nocardia transvalensis, Nocardia uniformis, Nocardia vaccinii, Nocardia veterana or Nocardia vinacea;*
Pseudomonaceae such as the genera Azomonas, Azotobacter, Cellvibrio, Chryseomonas, Flaviomonas, Lampropedia, Mesophilobacter, Morococcus, Oligella, Pseudomonas, Rhizobacter, Rugamonas, Serpens, Thermoleophilum, Xylophilus e.g. the species *Azomonas agilis, Azomonas insignis, Azomonas macrocytogenes, Azotobacter agilis, Azotobacter agilis subsp. armeniae, Azotobacter armeniacus, Azotobacter beijerinckii, Azotobacter chroococcum, Azotobacter indicum, Azotobacter macrocytogenes, Azotobacter miscellum, Azotobacter nigricans subsp. nigricans, Azotobacter paspali, Azotobacter salinestris, Azotobacter sp., Azotobacter vinelandii, Flavimonas oryzihabitans, Mesophilobacter marinus, Oligella urethralis, Pseudomonas acidovorans, Pseudomonas aeruginosa, Pseudomonas agarici, Pseudomonas alcaligenes, Pseudomonas aminovorans, Pseudomonas amygdali, Pseudomonas andropogonis, Pseudomonas anguilliseptica, Pseudomonas antarctica, Pseudomonas antimicrobica, Pseudomonas antimycetica, Pseudomonas aptata, Pseudomonas arvilla, Pseudomonas asplenii, Pseudomonas atlantica, Pseudomonas atrofaciens, Pseudomonas aureofaciens, Pseudomonas avellanae, Pseudomonas azelaica, Pseudomonas azotocolligans, Pseudomonas balearica, Pseudomonas barkeri, Pseudomonas bathycetes, Pseudomonas beijerinckii, Pseudomonas brassicacearum, Pseudomonas brenneri, Pseudomonas butanovora, Pseudomonas carboxydoflava, Pseudomonas carboxydohydrogena, Pseudomonas carboxydovorans, Pseudomonas carrageenovora, Pseudomonas caryophylli, Pseudomonas cepacia, Pseudomonas chloritidismutans, Pseudomonas chlororaphis, Pseudomonas cichorii, Pseudomonas citronellolis, Pseudomonas cocovenenans, Pseudomonas compransoris, Pseudomonas congelans, Pseudomonas coronafaciens, Pseudomonas corrugata, Pseudomonas dacunhae, Pseudomonas delafieldii, Pseudomonas delphinii, Pseudomonas denitrificans, Pseudomonas desmolytica, Pseudomonas diminuta, Pseudomonas doudoroffii, Pseudomonas echinoides, Pseudomonas elongata, Pseudomonas extorquens, Pseudomonas extremorientalis, Pseudomonas facilis, Pseudomonas ficuserectae, Pseudomonas flava, Pseudomonas flavescens, Pseudomonas fluorescens, Pseudomonas fragi, Pseudomonas frederiksbergensis, Pseudomonas fulgida, Pseudomonas fuscovaginae, Pseudomonas gazotropha, Pseudomonas gladioli, Pseudomonas glathei, Pseudomonas glumae, Pseudomonas graminis, Pseudomonas halophila, Pseudomonas helianthi, Pseudomonas huttiensis, Pseudomonas hydrogenothermophila, Pseudomonas hydrogenovora, Pseudomonas indica, Pseudomonas indigofera, Pseudomonas iodinum, Pseudomonas kilonensis, Pseudomonas lachrymans, Pseudomonas lapsa, Pseudomonas lemoignei, Pseudomonas lemonnieri, Pseudomonas lundensis, Pseudomonas luteola, Pseudomonas maltophilia, Pseudomonas marginalis, Pseudomonas marginata, Pseudomonas marina, Pseudomonas meliae, Pseudomonas mendocina, Pseudomonas mesophilica, Pseudomonas mixta, Pseudomonas monteilii, Pseudomonas morsprunorum, Pseudomonas multivorans, Pseudomonas natriegens, Pseudomonas nautica, Pseudomonas nitroreducens, Pseudomonas oleovorans, Pseudomonas oryzihabitans, Pseudomonas ovalis, Pseudomonas oxalaticus, Pseudomonas palleronii, Pseudomonas paucimobilis, Pseudomonas phaseolicola, Pseudomonas phenazinium, Pseudomonas pickettii, Pseudomonas pisi, Pseudomonas plantarii, Pseudomonas plecoglossicida, Pseudomonas poae, Pseudomonas primulae, Pseudomonas proteolytica, Pseudomonas pseudoalcaligenes, Pseudomonas pseudoalcaligenes* subsp. *konjaci, Pseudomonas pseudoalcaligenes* subsp. *pseudoalcaligenes, Pseudomonas pseudoflava, Pseudomonas putida, Pseudomonas putida* var. *naraensis, Pseudomonas putrefaciens, Pseudomonas pyrrocinia, Pseudomonas radiora, Pseudomonas reptilivora, Pseudomonas rhodesiae, Pseudomonas rhodos, Pseudomonas riboflavina, Pseudomonas rubescens, Pseudomonas rubrisubalbicans, Pseudomonas ruhlandii, Pseudomonas saccharophila, Pseudomonas savastanoi, Pseudomonas savastanoi* pvar. *glycinea, Pseudomonas savastanoi* pvar. *phaseolicola, Pseudomonas solanacearum, Pseudomonas* sp., *Pseudomonas spinosa, Pseudomonas stanieri, Pseudomonas stutzeri, Pseudomonas syringae, Pseudomonas syringae* pvar. *aptata, Pseudomonas syringae* pvar. *atrofaciens, Pseudomonas syringae* pvar. *coronafaciens, Pseudomonas syringae* pvar. *delphinii, Pseudomonas syringae* pvar. *glycinea, Pseudomonas syringae* pvar. *helianthi, Pseudomonas syringae* pvar. *lachrymans, Pseudomonas syringae* pvar. *lapsa, Pseudomonas syringae* pvar. *morsprunorum, Pseudomonas syringae* pvar. *phaseolicola, Pseudomonas syringae* pvar. *primulae, Pseudomonas syringae* pvar. *syringae, Pseudomonas syringae* pvar. *tabaci, Pseudomonas syringae* pvar. *tomato, Pseudomonas syringae* subsp. *glycinea, Pseudomonas syringae* subsp. *savastanoi, Pseudomonas syringae* subsp. *syringae, Pseudomonas syzygii, Pseudomonas tabaci, Pseudomonas taeniospiralis, Pseudomonas testosteroni, Pseudomonas thermocarboxydovorans, Pseudomonas thermotolerans, Pseudomonas thivervalensis, Pseudomonas tomato,* Pseudomonas *trivialis, Pseudomonas veronii, Pseudomonas vesicularis, Pseudomonas viridiflava, Pseudomonas* viscogena, *Pseudomonas woodsii,* Rhizobacter *dauci, Rhizobacter daucus or Xylophilus ampelinus;* Rhizobiaceae such as the genera Agrobacterium, Carbophilus, Chelatobacter, Ensifer, Rhizobium, Sinorhizobium e.g. the species *Agrobacterium atlanticum, Agrobacterium ferrugineum, Agrobacterium gelatinovorum, Agrobacterium larrymoorei, Agrobacterium meteori, Agrobacterium radiobacter, Agrobacterium rhizogenes, Agrobacterium rubi, Agrobacterium stellulatum, Agrobacterium tumefaciens, Agrobacterium vitis, Carbophilus carboxidus, Chelatobacter heintzii, Ensifer adhaerens, Ensifer arboris, Ensifer fredii, Ensifer kostiensis*, *Ensifer kummerowiae, Ensifer medicae, Ensifer meliloti, Ensifer saheli, Ensifer terangae, Ensifer xinjiangensis, Rhizobium ciceri Rhizobium etli, Rhizobium fredii, Rhizobium galegae, Rhizobium gallicum, Rhizobium giardinii, Rhizobium hainanense, Rhizobium huakuii, Rhizobium huautlense, Rhizobium indigoferae, Rhizobium japonicum, Rhizobium leguminosarum, Rhizobium loessense, Rhizobium loti, Rhizobium lupini, Rhizobium mediterraneum, Rhizobium meliloti, Rhizobium mongolense, Rhizobium phaseoli, Rhizobium radiobacter, Rhizobium rhizogenes, Rhizobium rubi, Rhizobium sullae, Rhizobium tianshanense, Rhizobium trifolii, Rhizobium tropici, Rhizobium undicola, Rhizobium vitis, Sinorhizobium* adhaerens, *Sinorhizobium arboris, Sinorhizobium fredii, Sinorhizobium kostiense, Sinorhizobium kummerowiae, Sinorhizobium medicae, Sinorhizobium meliloti, Sinorhizobium morelense*, *Sinorhizobium saheli or Sinorhizobium xinjiangense;* Streptomycetaceae such as the genera Kitasatosprora, Streptomyces, Streptoverticillium e.g. the species *Streptomyces abikoensis, Streptomyces aburaviensis, Streptomyces achromogenes subsp. achromogenes*, *Streptomyces achromogenes subsp. rubradiris, Streptomyces acidiscabies, Streptomyces acrimycini, Streptomyces acu*/*eo*/*atus, Streptomyces afghaniensis, Streptomyces alanosinicus, Streptomyces albaduncus, Streptomyces albiaxialis, Streptomyces albidochromogenes, Streptomyces albidoflavus, Streptomyces albireticuli, Streptomyces albofaciens, Streptomyces alboflavus, Streptomyces albogriseolus, Streptomyces albolongus, Streptomyces alboniger, Streptomyces albospinus, Streptomyces albosporeus subsp. a*/*bosporeus, Streptomyces albosporeus subsp*. *labilomyceticus, Streptomyces alboverticillatus, Streptomyces albovinaceus, Streptomyces alboviridis, Streptomyces albulus, Streptomyces albus subsp. albus, Streptomyces albus subsp. pathocidicus, Streptomyces almquistii, Streptomyces althioficus, Streptomyces amakusaensis, Streptomyces ambofaciens, Streptomyces aminophilus, Streptomyces anandii, Streptomyces anthocyanicus, Streptomyces antibioticus, Streptomyces antimycoticus, Streptomyces anulatus, Streptomyces arabicus, Streptomyces ardus, Streptomyces arenae, Streptomyces argenteolus, Streptomyces armeniacus, Streptomyces asiaticus, Streptomyces asterosporus, Streptomyces atratus, Streptomyces atroaurantiacus, Streptomyces atroolivaceus, Streptomyces atrovirens, Streptomyces aurantiacus, Streptomyces aurantiogriseus, Streptomyces aureocirculatus, Streptomyces aureofaciens, Streptomyces aureorectus, Streptomyces aureoversilis, Streptomyces aureoverticillatus, Streptomyces aureus, Streptomyces avellaneus, Streptomycesavermectinius, Streptomyces avermitilis, Streptomyces avidinii, Streptomyces azaticus, Streptomyces azureus, Streptomyces baarnensis, Streptomyces bacillaris, Streptomyces badius, Streptomyces baldaccii, Streptomyces bambergiensis, Streptomyces beijiangensis, Streptomyces bellus, Streptomyces bikiniensis, Streptomyces biverticillatus, Streptomyces blastmyceticus, Streptomyces bluensis, Streptomyces bobili, Streptomyces bottropensis, Streptomyces brasiliensis, Streptomyces bungoensis, Streptomyces cacaoi subsp. asoensis, Streptomyces cacaoi subsp. cacaoi, Streptomyces caelestis, Streptomyces caeruleus, Streptomyces californicus, Streptomyces calvus, Streptomyces canaries, Streptomyces candidus, Streptomyces canescens, Streptomyces cangkringensis, Streptomyces caniferus, Streptomyces canus, Streptomyces capillispiralis, Streptomyces capoamus, Streptomyces carpaticus, Streptomyces carpinensis, Streptomyces catenulae, Streptomyces caviscabies, Streptomyces cavourensis subsp. cavourensis, Streptomyces cavourensis subsp. washingtonensis, Streptomyces cellostaticus, Streptomyces celluloflavus, Streptomyces cellulolyticus, Streptomyces cellulosae,Streptomyces champavatii, Streptomyces chartreuses, Streptomyces chattanoogensis, Streptomyces chibaensis, Streptomyces chrestomyceticus, Streptomyces chromofuscus, Streptomyces chryseus, Streptomyces chrysomallus subsp. chrysomallus, Streptomyces chrysomallus subsp. fumigatus, Streptomyces cinereorectus, Streptomyces cinereoruber subsp. cinereoruber, Streptomyces cinereoruber subsp. fructofermentans, Streptomyces cinereospinus, Streptomyces cinereus, Streptomyces cinerochromogenes, Streptomyces cinnabarinus, Streptomyces cinnamonensis, Streptomyces cinnamoneus, Streptomyces cinnamoneus subsp. albosporus, Streptomyces cinnamoneus subsp. cinnamoneus, Streptomyces cinnamoneus subsp. lanosus, Streptomyces cinnamoneus subsp. sparsus, Streptomyces cirratus, Streptomyces ciscaucasicus, Streptomyces citreofluorescens, Streptomyces clavifer, Streptomyces clavuligerus, Streptomyces cochleatus, Streptomyces coelescens, Streptomyces coelicoflavus, Streptomyces coelicolor, Streptomyces coeruleoflavus, Streptomyces coeruleofuscus, Streptomyces coeruleoprunus, Streptomyces coeruleorubidus, Streptomyces coerulescens, Streptomyces collinus, Streptomyces colombiensis, Streptomyces corchorusii, Streptomyces costaricanus, Streptomyces cremeus, Streptomyces crystallinus, Streptomyces curacoi, Streptomyces cuspidosporus, Streptomyces cyaneofuscatus, Streptomyces cyaneus, Streptomyces cyanoalbus, Streptomyces cystargineus, Streptomyces daghestanicus, Streptomyces diastaticus subsp. ardesiacus, Streptomyces diastaticus subsp. diastaticus, Streptomyces diastatochromogenes, Streptomyces distallicus, Streptomyces djakartensis, Streptomyces durhamensis, Streptomyces echinatus, Streptomyces echinoruber, Streptomyces ederensis, Streptomyces ehimensis, Streptomyces endus, Streptomyces enissocaesilis, Streptomyces erumpens, Streptomyces erythraeus, Streptomyces erythrogriseus, Streptomyces eurocidicus, Streptomyces europaeiscabiei, Streptomyces eurythermus, Streptomyces exfoliates, Streptomyces felleus, Streptomyces fervens, Streptomyces fervens subsp. fervens, Streptomyces fervens subsp. melrosporus, Streptomyces filamentosus, Streptomyces filipinensis, Streptomyces fimbriatus, Streptomyces fimicarius, Streptomyces finlayi, Streptomyces flaveolus, Streptomyces flaveus, Streptomyces flavidofuscus, Streptomyces flavidovirens, Streptomyces flaviscleroticus, Streptomyces flavofungini, Streptomyces flavofuscus, Streptomyces flavogriseus, Streptomyces flavopersicus, Streptomyces flavotricini, Streptomyces flavovariabilis, Streptomyces flavovirens, Streptomyces flavoviridis, Streptomyces flocculus, Streptomyces floridae, Streptomyces fluorescens, Streptomyces fradiae, Streptomyces fragilis, Streptomyces fulvissimus, Streptomyces fulvorobeus, Streptomyces fumanus, Streptomyces fumigatiscleroticus, Streptomyces galbus, Streptomyces galilaeus, Streptomyces gancidicus, Streptomyces gardneri, Streptomyces gelaticus, Streptomyces geysiriensis, Streptomyces ghanaensis, Streptomyces gibsonii, Streptomyces glaucescens, Streptomyces glaucosporus, Streptomyces glaucus, Streptomyces globisporus subsp. caucasicus, Streptomyces globisporus subsp. flavofuscus, Streptomyces globisporus subsp. globisporus, Streptomyces globosus, Streptomyces glomeratus, Streptomyces glomeroaurantiacus, Streptomyces gobitricini, Streptomyces goshikiensis, Streptomyces gougerotii, Streptomyces graminearus, Streptomyces graminofaciens, Streptomyces griseinus, Streptomyces griseoaurantiacus, Streptomyces griseobrunneus, Streptomyces griseocarneus, Streptomyces griseochromogenes, Streptomyces griseoflavus, Streptomyces griseofuscus, Streptomyces griseoincarnatus, Streptomyces griseoloalbus, Streptomyces griseolosporeus, Streptomyces griseolus, Streptomyces griseoluteus, Streptomyces griseomycini, Streptomyces griseoplanus, Streptomyces griseorubens, Streptomyces griseoruber, Streptomyces griseorubiginosus, Streptomyces griseosporeus, Streptomyces griseostramineus, Streptomyces griseoverticillatus, Streptomyces griseoviridis, Streptomyces griseus subsp. alpha, Streptomyces griseus subsp. cretosus, Streptomyces griseus subsp. griseus, Streptomyces griseus subsp. solvifaciens, Streptomyces hachijoensis, Streptomyces halstedii, Streptomyces hawaiiensis, Streptomyces heliomycini, Streptomyces helvaticus, Streptomyces herbaricolor, Streptomyces hiroshimensis, Streptomyces hirsutus, Streptomyces humidus, Streptomyces humiferus, Streptomyces hydrogenans, Streptomyces hygroscopicus subsp. angustmyceticus, Streptomyces hygroscopicus subsp. decoyicus, Streptomyces hygroscopicus subsp. glebosus, Streptomyces hygroscopicus subsp. hygroscopicus, Streptomyces hygroscopicus subsp. ossamyceticus, Streptomyces iakyrus, Streptomyces indiaensis, Streptomyces indigoferus, Streptomyces indonesiensis, Streptomyces intermedius, Streptomyces inusitatus, Streptomyces ipomoeae, Streptomyces janthinus, Streptomyces javensis, Streptomyces kanamyceticus, Streptomyces kashmirensis, Streptomyces kasugaensis, Streptomyces katrae, Streptomyces kentuckensis, Streptomyces kifunensis, Streptomyces kishiwadensis, Streptomyces kunmingensis, Streptomyces kurssanovii, Streptomyces labedae, Streptomyces laceyi, Streptomyces ladakanum, Streptomyces lanatus, Streptomyces lateritius, Streptomyces laurentii, Streptomyces lavendofoliae, Streptomyces lavendulae subsp. grasserius, Streptomyces lavendulae subsp. lavendulae, Streptomyces lavenduligriseus, Streptomyces lavendulocolor, Streptomyces levis, Streptomyces libani* subsp. *libani, Streptomyces libani* subsp. *rufus, Streptomyces lienomycini, Streptomyces lilacinus, Streptomyces limosus, Streptomyces lincolnensis, Streptomyces lipmanii, Streptomyces litmocidini, Streptomyces lomondensis, Streptomyces longisporoflavus, Streptomyces longispororuber, Streptomyces longisporus, Streptomyces longwoodensis, Streptomyces lucensis, Streptomyces luridiscabiei, Streptomyces luridus, Streptomyces lusitanus, Streptomyces luteireticuli, Streptomyces luteogriseus, Streptomyces luteosporeus, Streptomyces luteoverticillatus, Streptomyces lydicus, Streptomyces macrosporus, Streptomyces malachitofuscus, Streptomyces malachitospinus, Streptomyces malaysiensis, Streptomyces mashuensis, Streptomyces massasporeus, Streptomyces matensis, Streptomyces mauvecolor, Streptomyces mediocidicus, Streptomyces mediolani, Streptomyces megasporus, Streptomyces melanogenes, Streptomyces melanosporofaciens, Streptomyces mexicanus, Streptomyces michiganensis, Streptomyces microflavus, Streptomyces minutiscleroticus, Streptomyces mirabilis, Streptomyces misakiensis, Streptomyces misionensis, Streptomyces mobaraensis, Streptomyces monomycini, Streptomyces morookaensis, Streptomyces murinus, Streptomyces mutabilis, Streptomyces mutomycini, Streptomyces naganishii, Streptomyces narbonensis, Streptomyces nashvillensis, Streptomyces netropsis, Streptomyces neyagawaensis, Streptomyces niger, Streptomyces nigrescens, Streptomyces nigrifaciens, Streptomyces nitrosporeus, Streptomyces niveiciscabiei, Streptomyces niveoruber, Streptomyces niveus, Streptomyces noboritoensis, Streptomyces nodosus, Streptomyces nogalater, Streptomyces nojiriensis, Streptomyces noursei, Streptomyces novaecaesareae, Streptomyces ochraceiscleroticus, Streptomyces odorifer, Streptomyces olivaceiscleroticus, Streptomyces olivaceoviridis, Streptomyces olivaceus, Streptomyces olivochromogenes, Streptomyces olivomycini, Streptomyces olivoreticuli, Streptomyces olivoreticuli subsp. cellulophilus, Streptomyces olivoreticuli subsp. olivoreticuli, Streptomyces olivoverticillatus, Streptomyces olivoviridis, Streptomyces omiyaensis, Streptomyces orinoci, Streptomyces pactum, Streptomyces paracochleatus, Streptomyces paradoxus, Streptomyces parvisporogenes, Streptomyces parvulus, Streptomyces parvus, Streptomyces peucetius, Streptomyces phaeochromogenes, Streptomyces phaeofaciens, Streptomyces phaeopurpureus, Streptomyces phaeoviridis, Streptomyces phosalacineus, Streptomyces pilosus, Streptomyces platensis, Streptomyces plicatus, Streptomyces pluricolorescens, Streptomyces polychromogenes, Streptomyces poonensis, Streptomyces praecox, Streptomyces prasinopilosus, Streptomyces prasinosporus, Streptomyces prasinus, Streptomyces prunicolor, Streptomyces psammoticus, Streptomyces pseudoechinosporeus, Streptomyces pseudogriseolus, Streptomyces pseudovenezuelae, Streptomyces pulveraceus, Streptomyces puniceus, Streptomyces puniciscabiei, Streptomyces purpeofuscus, Streptomyces purpurascens, Streptomyces purpureus, Streptomyces purpurogeneiscleroticus, Streptomyces racemochromogenes, Streptomyces rameus, Streptomyces ramulosus, Streptomyces rangoonensis, Streptomyces recifensis, Streptomyces rectiverticillatus, Streptomyces rectiviolaceus, Streptomyces regensis, Streptomyces resistomycificus, Streptomyces reticuliscabiei, Streptomyces rhizosphaericus, Streptomyces rimosus subsp. paromomycinus, Streptomyces rimosus subsp. rimosus, Streptomyces rishiriensis, Streptomyces rochei, Streptomyces roseiscleroticus, Streptomyces roseodiastaticus, Streptomyces roseoflavus, Streptomyces roseofulvus, Streptomyces roseolilacinus, Streptomyces roseolus, Streptomyces roseosporus, Streptomyces roseoverticillatus, Streptomyces roseoviolaceus, Streptomyces roseoviridis, Streptomyces rubber, Streptomyces rubiginosohelvolus, Streptomyces rubiginosus, Streptomyces rubrogriseus, Streptomyces rutgersensis subsp. castelarensis, Streptomyces rutgersensis subsp. rutgersensis, Streptomyces salmonis, Streptomyces sampsonii, Streptomyces sanglieri, Streptomyces sannanensis, Streptomyces sapporonensis, Streptomyces scabiei, Streptomyces sclerotialus, Streptomyces scopiformis, Streptomyces seoulensis, Streptomyces septatus, Streptomyces setae, Streptomyces setonii, Streptomyces showdoensis, Streptomyces sindenensis, Streptomyces sioyaensis, Streptomyces somaliensis, Streptomyces sparsogenes, Streptomyces spectabilis, Streptomyces speibonae, Streptomyces speleomycini, Streptomyces spheroids, Streptomyces spinoverrucosus, Streptomyces spiralis, Streptomyces spiroverticillatus, Streptomyces spitsbergensis, Streptomyces sporocinereus, Streptomyces sporoclivatus, Streptomyces spororaveus, Streptomyces sporoverrucosus, Streptomyces stelliscabiei, Streptomyces stramineus, Streptomyces subrutilus, Streptomyces sulfonofaciens, Streptomyces sulphurous, Streptomyces syringium, Streptomyces tanashiensis, Streptomyces tauricus, Streptomyces tendae, Streptomyces termitum, Streptomyces thermoalcalitolerans, Streptomyces thermoautotrophicus, Streptomyces thermocarboxydovorans, Streptomyces thermocarboxydus, Streptomyces thermocoprophilus, Streptomyces thermodiastaticus, Streptomyces thermogriseus, Streptomyces thermolineatus, Streptomyces thermonitrificans, Streptomyces thermospinosisporus, Streptomyces thermoviolaceus subsp. apingens, Streptomyces thermoviolaceus subsp. thermoviolaceus, Streptomyces thermovulgaris, Streptomyces thioluteus, Streptomyces torulosus, Streptomyces toxytricini, Streptomyces tricolor, Streptomyces tubercidicus, Streptomyces tuirus, Streptomyces turgidiscabies, Streptomyces umbrinus, Streptomyces variabilis, Streptomyces variegates, Streptomyces varsoviensis, Streptomyces vastus, Streptomyces venezuelae, Streptomyces vinaceus, Streptomyces vinaceusdrappus, Streptomyces violaceochromogenes, Streptomyces violaceolatus,Streptomyces violaceorectus, Streptomyces violaceoruber, Streptomyces violaceorubidus, Streptomyces violaceus, Streptomyces violaceusniger, Streptomyces violarus, Streptomyces violascens, Streptomyces violatus, Streptomyces violens, Streptomyces virens, Streptomyces virginiae, Streptomyces viridiflavus, Streptomyces viridiviolaceus, Streptomyces viridobrunneus, Streptomyces viridochromogenes, Streptomyces viridodiastaticus, Streptomyces viridosporus, Streptomyces vitaminophileus, Streptomyces vitaminophilus, Streptomyces wedmorensis, Streptomyces werraensis, Streptomyces willmorei, Streptomyces xanthochromogenes, Streptomyces xanthocidicus, Streptomyces xantholiticus, Streptomyces xanthophaeus, Streptomyces yatensis, Streptomyces yerevanensis, Streptomyces yogyakartensis, Streptomyces yokosukanensis, Streptomyces yunnanensis, Streptomyces zaomyceticus, Streptoverticillium abikoense, Streptoverticillium albireticuli, Streptoverticillium alboverticillatum, Streptoverticillium album, Streptoverticillium ardum, Streptoverticillium aureoversale, Streptoverticillium aureoversile, Streptoverticillium baldaccii , Streptoverticillium biverticillatum, Streptoverticillium blastmyceticum, Streptoverticillium cinnamoneum* subsp. *albosporum, Streptomyces cinnamoneus* subsp. *albosporus*, *Streptoverticillium cinnamoneum* subsp. *cinnamoneum, Streptoverticillium cinnamoneum* subsp. *lanosum, Streptoverticillium cinnamoneum* subsp. *sparsum, Streptoverticillium distallicum, Streptoverticillium ehimense, Streptoverticillium eurocidicum, Streptoverticillium fervens* subsp. *fervens, Streptoverticillium fervens* subsp. *melrosporus, Streptoverticillium flavopersicum, Streptoverticillium griseocarneum, Streptoverticillium griseoverticillatum, Streptoverticillium hachijoense, Streptoverticillium hiroshimense, Streptoverticillium kashmirense, Streptoverticillium kentuckense, Streptoverticillium kishiwadense, Streptoverticillium ladakanum, Streptoverticillium lavenduligriseum, Streptoverticillium lilacinum, Streptoverticillium luteoverticillatum, Streptoverticillium mashuense, Streptoverticillium mobaraense, Streptoverticillium morookaense, Streptoverticillium netropsis, Streptoverticillium olivomycini, Streptomyces olivomycini, Streptoverticillium olivoreticuli* subsp. *cellulophilum, Streptoverticillium olivoreticuli* subsp. *olivoreticuli, Streptoverticillium olivoreticulum, Streptoverticillium olivoreticulum* subsp. *cellulophilum, Streptoverticillium olivoverticillatum, Streptoverticillium orinoci, Streptoverticillium parvisporogenes, Streptoverticillium parvisporogenum, Streptoverticillium rectiverticillatum, Streptoverticillium reticulum* subsp. *protomycicum, Streptoverticillium roseoverticillatum, Streptoverticillium salmonis, Streptoverticillium sapporonense, Streptoverticillium septatum, Streptoverticillium syringium, Streptoverticillium thioluteum, Streptoverticillium verticillium* subsp. *quantum, Streptoverticillium verticillium* subsp. *tsukushiense or Streptoverticillium viridoflavum.*

**[0080.0.0.0]** Particular preferred strains are strains selected from the group consisting of Bacillaceae, Brevibacteriaceae, Corynebacteriaceae, Nocardiaceae, Mycobacteriaceae, Streptomycetaceae, Enterobacteriaceae such as *Bacillus circulans, Bacillus subtilis, Bacillus sp., Brevibacterium albidum, Brevibacterium album, Brevibacterium cerinum, Brevibacterium flavum, Brevibacterium glutamigenes, Brevibacterium iodinum, Brevibacterium ketoglutamicum, Brevibacterium lactofermentum, Brevibacterium linens, Brevibacterium roseum, Brevibacterium saccharolyticum, Brevibacterium sp., Corynebacterium acetoacidophilum, Corynebacterium acetoglutamicum, Corynebacterium ammoniagenes, Corynebacterium glutamicum (= Micrococcus glutamicum), Corynebacterium melassecola, Corynebacterium sp., Nocardia rhodochrous (Rhodococcus rhodochrous) , Mycobacterium rhodochrous, Streptomyces lividans* and *Escherichia coli* especially *Escherichia coli K12.*

**[0081.0.0.0]** In addition particular preferred strains are strains selected from the group consisting of Cryptococcaceae, Saccharomycetaceae, Schizosaccharomycetacease such as the genera Candida, Hansenula, Pichia, Saccharomyces and Schizosaccharomyces preferred are strains selected from the group consisting of the species *Rhodotorula rubra, Rhodotorula glutinis, Rhodotorula graminis, Yarrowia lipolytica, Sporobolomyces salmonicolor, Sporobolomyces shibatanus, Saccharomyces cerevisiae, Candida boidinii, Candida bombicola, Candida cylindracea, Candida parapsilosis, Candida rugosa, Candida tropicalis, Pichia methanolica and Pichia pastoris.*

**[0082.0.0.0]** Anacardiaceae such as the genera Pistacia, Mangifera, Anacardium e.g. the species *Pistacia vera* [pistachios, Pistazie], *Mangifer indica* [Mango] or *Anacardium occidentale* [Cashew]; Asteraceae such as the genera Calendula, Carthamus, Centaurea, Cichorium, Cynara, Helianthus, Lactuca, Locusta, Tagetes, Valeriana e.g. the species *Calendula officinalis* [Marigold], *Carthamus tinctorius* [safflower], *Centaurea cyanus* [cornflower], *Cichorium intybus* [blue daisy], *Cynara scolymus* [Artichoke], *Helianthus annus* [sunflower], Lactuca sativa, *Lactuca crispa, Lactuca esculenta, Lactuca scariola L. ssp. sativa, Lactuca scariola L. var. integrata, Lactuca scariola L. var. integrifolia, Lactuca sativa subsp. romana, Locusta communis, Valeriana locusta* [lettuce], *Tagetes lucida, Tagetes erecta or Tagetes tenuifolia* [Marigold]; Apiaceae such as the genera Daucus e.g. the species *Daucus carota* [carrot]; Betulaceae such as the genera Corylus e.g. the species *Corylus avellana* or *Corylus colurna* [hazelnut]; Boraginaceae such as the genera Borago e.g. the species *Borago officinalis* [borage]; Brassicaceae such as the genera Brassica, Melanosinapis, Sinapis, Arabadopsis e.g. the species *Brassica napus*, *Brassica rapa* ssp. [canola, oilseed rape, turnip rape], *Sinapis arvensis Brassica juncea, Brassica juncea var. juncea, Brassica juncea var. crispifolia, Brassica juncea var. foliosa, Brassica nigra, Brassica sinapioides, Melanosinapis communis* [mustard], *Brassica oleracea* [fodder beet] or Arabidopsis thaliana; Bromeliaceae such as the genera Anana, Bromelia e.g. the species *Anana comosus, Ananas ananas* or *Bromelia comosa* [pineapple]; Caricaceae such as the genera Carica e.g. the species *Carica papaya* [papaya]; Cannabaceae such as the genera Cannabis e.g. the species *Cannabis sative* [hemp], Convolvulaceae such as the genera Ipomea, Convolvulus e.g. the species *Ipomoea batatus, Ipomoea pandurata, Convolvulus batatas, Convolvulus tiliaceus, Ipomoea fastigiata, Ipomoea tiliacea, Ipomoea triloba* or *Convolvulus panduratus* [sweet potato, Man of the Earth, wild potato], Chenopodiaceae such as the genera Beta, i.e. the species *Beta vulgaris, Beta vulgaris var. altissima, Beta vulgaris var. Vulgaris, Beta maritima, Beta vulgaris var. perennis, Beta vulgaris var. conditiva* or *Beta vulgaris var. esculenta* [sugar beet]; Cucurbitaceae such as the genera Cucubita e.g. the species *Cucurbita maxima, Cucurbita mixta, Cucurbita pepo* or *Cucurbita moschata* [pumpkin, squash]; Elaeagnaceae such as the genera Elaeagnus e.g. the species *Olea europaea* [olive]; Ericaceae such as the genera Kalmia e.g. the species *Kalmia latifolia, Kalmia angustifolia, Kalmia microphylla, Kalmia polifolia, Kalmia occidentalis, Cistus chamaerhodendros* or *Kalmia lucida* [American laurel, broad-leafed laurel, calico bush, spoon wood, sheep laurel, alpine laurel, bog laurel, western bog-laurel, swamp-laurel]; Euphorbiaceae such as the genera Manihot, Janipha, Jatropha, Ricinus e.g. the species *Manihot utilissima, Janipha manihot*,, *Jatropha manihot., Manihot aipil, Manihot dulcis, Manihot manihot, Manihot melanobasis, Manihot esculenta* [manihot, arrowroot, tapioca, cassava] or *Ricinus communis* [castor bean, Castor Oil Bush, Castor Oil Plant, Palma Christi, Wonder Tree]; Fabaceae such as the genera Pisum, Albizia, Cathormion, Feuillea, Inga, Pithecolobium, Acacia, Mimosa, Medicajo, Glycine, Dolichos, Phaseolus, Soja e.g. the species *Pisum sativum, Pisum arvense, Pisum humile* [pea], *Albizia berteriana, Albizia julibrissin, Albizia lebbeck, Acacia berteriana, Acacia littoralis, Albizia berteriana, Albizzia berteriana, Cathormion berteriana, Feuillea berteriana, Inga fragrans, Pithecellobium berterianum, Pithecellobium fragrans, Pithecolobium berterianum, Pseudalbizzia berteriana, Acacia julibrissin, Acacia nemu, Albizia nemu, Feuilleea julibrissin, Mimosa julibrissin, Mimosa speciosa, Sericanrda julibrissin, Acacia lebbeck, Acacia macrophylla, Albizia lebbek, Feuilleea lebbeck, Mimosa lebbeck, Mimosa speciosa* [bastard logwood, silk tree, East Indian Walnut], *Medicago sativa, Medicago falcata, Medicago varia* [alfalfa] *Glycine max Dolichos soja, Glycine gracilis, Glycine hispida, Phaseolus max, Soja hispida* or *Soja max* [soybean]; Geraniaceae such as the genera Pelargonium, Cocos, Oleum e.g. the species Cocos *nucifera, Pelargonium grossularioides* or *Oleum cocois* [coconut]; Gramineae such as the genera Saccharum e.g. the species *Saccharum officinarum;* Juglandaceae such as the genera Juglans, Wallia e.g. the species *Juglans regia, Juglans ailanthifolia, Juglans sieboldiana, Juglans cinerea, Wallia cinerea, Juglans bixbyi, Juglans californica, Juglans hindsii, Juglans intermedia, Juglans jamaicensis, Juglans major, Juglans microcarpa, Juglans nigra* or *Wallia nigra* [walnut, black walnut, common walnut, persian walnut, white walnut, butternut, black walnut]; Lauraceae such as the genera Persea, Laurus e.g. the species laurel *Laurus nobilis* [bay, laurel, bay laurel, sweet bay], *Persea americana Persea americana, Persea gratissima* or *Persea persea* [avocado]; Leguminosae such as the genera Arachis e.g. the species *Arachis hypogaea* [peanut]; Linaceae such as the genera Linum, Adenolinum e.g. the species *Linum usitatissimum, Linum humile, Linum austriacum, Linum bienne, Linum angustifolium, Linum catharticum, Linum flavum, Linum grandiflorum, Adenolinum grandiflorum, Linum lewisii, Linum narbonense, Linum perenne, Linum perenne var. lewisii, Linum pratense* or *Linum trigynum* [flax, linseed]; Lythrarieae such as the genera Punica e.g. the species *Punica granatum* [pomegranate]; Malvaceae such as the genera Gossypium e.g. the species *Gossypium hirsutum, Gossypium arboreum, Gossypium barbadense, Gossypium herbaceum* or *Gossypium thurberi* [cotton]; Musaceae such as the genera Musa e.g. the species *Musa nana, Musa acuminata, Musa paradisiaca, Musa* spp. [banana]; Onagraceae such as the genera Camissonia, Oenothera e.g. the species *Oenothera biennis* or *Camissonia brevipes* [primrose, evening primrose]; Palmae such as the genera Elacis e.g. the species *Elaeis guineensis* [oil plam]; Papaveraceae such as the genera Papaver e.g. the species *Papaver orientale, Papaver rhoeas, Papaver dubium* [poppy, oriental poppy, corn poppy, field poppy, shirley poppies, field poppy, long-headed poppy, long-pod poppy]; Pedaliaceae such as the genera Sesamum e.g. the species *Sesamum indicum* [sesame]; Piperaceae such as the genera Piper, Artanthe, Peperomia, Steffensia e.g. the species *Piper aduncum, Piper amalago, Piper angustifolium, Piper auritum, Piper betel, Piper cubeba, Piper longum, Piper nigrum, Piper retrofractum, Artanthe adunca, Artanthe elongata, Peperomia elongata, Piper elongatum, Steffensia elongata.* [Cayenne pepper, wild pepper]; Poaceae such as the genera Hordeum, Secale, Avena, Sorghum, Andropogon, Holcus, Panicum, Oryza, Zea, Triticum e.g. the species *Hordeum vulgare, Hordeum jubatum, Hordeum murinum, Hordeum secalinum, Hordeum distichon Hordeum aegiceras, Hordeum hexastichon., Hordeum hexastichum, Hordeum irregulare, Hordeum sativum, Hordeum secalinum* [barley, pearl barley, foxtail barley, wall barley, meadow barley], Secale cereale [rye], Avena sativa, *Avena fatua, Avena byzantina, Avena fatua var. sativa, Avena hybrida* [oat], *Sorghum bicolor, Sorghum halepense, Sorghum saccharatum, Sorghum vulgare, Andropogon drummondii, Holcus bicolor, Holcus sorghum, Sorghum aethiopicum, Sorghum arundinaceum, Sorghum caffrorum, Sorghum cernuum, Sorghum dochna, Sorghum drummondii, Sorghum durra, Sorghum guineense, Sorghum lanceolatum, Sorghum nervosum, Sorghum saccharatum, Sorghum subglabrescens, Sorghum verticilliflorum, Sorghum vulgare, Holcus halepensis, Sorghum miliaceum* millet, *Panicum militaceum [Sorghum, millet], Oryza sativa, Oryza latifolia* [rice], *Zea mays* [corn, maize] *Triticum aestivum, Triticum durum, Triticum turgidum, Triticum hybernum, Triticum macha, Triticum sativum* or *Triticum vulgare* [wheat, bread wheat, common wheat], Proteaceae such as the genera Macadamia e.g. the species *Macadamia intergrifolia* [macadamia]; Rubiaceae such as the genera Coffea e.g. the species Cofea spp., *Coffea arabica, Coffea canephora* or *Coffea liberica* [coffee]; Scrophulariaceae such as the genera Verbascum e.g. the species *Verbascum blattaria, Verbascum chaixii, Verbascum densiflorum, Verbascum lagurus, Verbascum longifolium, Verbascum lychnitis, Verbascum nigrum, Verbascum olympicum, Verbascum phlomoides, Verbascum phoenicum, Verbascum pulverulentum* or *Verbascum thapsus* [mullein, white moth mullein, nettle-leaved mullein, dense-flowered mullein, silver mullein, long-leaved mullein, white mullein, dark mullein, greek mullein, orange mullein, purple mullein, hoary mullein, great mullein]; Solanaceae such as the genera Capsicum, Nicotiana, Solanum, Lycopersicon e.g. the species *Capsicum annuum, Capsicum annuum var. glabriusculum, Capsicum frutescens* [pepper], *Capsicum annuum* [paprika], *Nicotiana tabacum, Nicotiana alata, Nicotiana attenuata, Nicotiana glauca, Nicotiana langsdorffii, Nicotiana obtusifolia, Nicotiana quadrivalvis, Nicotiana repanda, Nicotiana rustica, Nicotiana sylvestris* [tobacco], *Solanum tuberosum* [potato], *Solanum melongena* [egg-plant] (*Lycopersicon esculentum, Lycopersicon lycopersicum., Lycopersicon pyriforme, Solanum integrifolium* or *Solanum lycopersicum* [tomato]; Sterculiaceae such as the genera Theobroma e.g. the species *Theobroma cacao* [cacao]; Theaceae such as the genera Camellia e.g. the species *Camellia sinensis*) [tea].
All abovementioned organisms can in princible also function as host organisms.

**[0083.0.0.0]** Particular preferred plants are plants selected from the group consisting of Asteraceae such as the genera Helianthus, Tagetes e.g. the species *Helianthus annus* [sunflower], *Tagetes lucida, Tagetes erecta or Tagetes tenuifolia* [Marigold], Brassicaceae such as the genera Brassica, Arabadopsis e.g. the species *Brassica napus*, *Brassica rapa* ssp. [canola, oilseed rape, turnip rape] or Arabidopsis thaliana. Fabaceae such as the genera Glycine e.g. the species *Glycine max, Soja hispida* or *Soja max* [soybean] (wobei ich nicht sicher bin, ob es Soja max überhaupt gibt, die heist eigentlich Glycine max). Linaceae such as the genera Linum e.g. the species *Linum usitatissimum,* [flax, linseed]; Poaceae such as the genera Hordeum, Secale, Avena, Sorghum, Oryza, Zea, Triticum e.g. the species *Hordeum vulgare* [barley]; *Secale cereale* [rye], Avena sativa, *Avena fatua, Avena byzantina, Avena fatua var. sativa, Avena hybrida* [oat], *Sorghum bicolor* [Sorghum, millet], *Oryza sativa, Oryza latifolia* [rice], *Zea mays* [corn, maize] *Triticum aestivum, Triticum durum, Triticum turgidum, Triticum hybernum, Triticum macha, Triticum sativum* or *Triticum vulgare* [wheat, bread wheat, common wheat]; Solanaceae such as the genera Solanum, Lycopersicon e.g. the species *Solanum tuberosum* [potato], *Lycopersicon esculentum, Lycopersicon lycopersicum., Lycopersicon pyriforme, Solanum integrifolium* or *Solanum lycopersicum [tomato].*

**[0084.0.0.0]** All abovementioned organisms can in princible also function as host organisms.

**[0085.0.0.0]** With regard to the nucleic acid sequence as depicted a nucleic acid construct which contains a nucleic acid sequence mentioned herein or an organism (= transgenic organism) which is transformed with said nucleic acid sequence or said nucleic acid construct, "transgene" means all those constructs which have been brought about by genetic manipulation methods,preferably in which either
a) a nucleic acid sequence as indicated in Table I, columns 5 or 7, lines 1 to 5 and/or lines 334 to 338,or a derivative thereof, or
b) a genetic regulatory element, for example a promoter, which is functionally linked to the nucleic acid sequence as indicated in Table I, columns 5 or 7, lines 1 to 5 and/or lines 334 to 338, or a derivative thereof, or
c) (a) and (b)
is/are not present in its/their natural genetic environment or has/have been modified by means of genetic manipulation methods, it being possible for the modification to be, by way of example, a substitution, addition, deletion, inversion or insertion of one or more nucleotide. "Natural genetic environment" means the natural chromosomal locus in the organism of origin or the presence in a genomic library. In the case of a genomic library, the natural, genetic environment of the nucleic acid sequence is preferably at least partially still preserved. The environment flanks the nucleic acid sequence at least on one side and has a sequence length of at least 50 bp, preferably at least 500 bp, particularly preferably at least 1000 bp, very particularly preferably at least 5000 bp.

**[0086.0.0.0]** The use of the nucleic acid sequence according to the invention or of the nucleic acid construct according to the invention for the generation of transgenic plants is therefore also subject matter of the invention.

**[0087.0.0.0]** The respective fine chemical, which is synthesized in the organism, in particular the microorganism, the cell, the tissue or the plant, of the invention can be isolated if desired. Depending on the use of the respective fine chemical, different purities resulting from the purification may be advantageous as will be described herein below.

**[0088.0.0.0]** In an advantageous embodiment of the invention, the organism takes the form of a plant whose amino acid content is modified advantageously owing to the nucleic acid molecule of the present invention expressed. This is important for plant breeders since, for example, the nutritional value of plants for monogastric animals is limited by a few essential amino acids such as lysine, threonine or methionine.

**[0088.1.0.0]** In one embodiment, after an activity of a polypeptide of the present invention or used in the process of the present invention has been increased or generated, or after the expression of a nucleic acid molecule or polypeptide according to the invention has been generated or increased, the transgenic plant generated can be grown on or in a nutrient medium or else in the soil and subsequently harvested.

**[0089.0.0.0]** The plants or parts thereof, e.g. the leaves, roots, flowers, and/or stems and/or other harvestable material as described below, can then be used directly as foodstuffs or animal feeds or else be further processed. Again, the amino acids can be purified further in the customary manner via extraction and precipitation or via ion exchangers and other methods known to the person skilled in the art and described herein below. Products which are suitable for various applications and which result from these different processing procedures are amino acids or amino acid compositions which can still comprise further plant components in different amounts, advantageously in the range of from 0 to 99% by weight, preferably from below 90% by weight, especially preferably below 80% by weight. The plants can also advantageously be used directly without further processing, e.g. as feed or for extraction.

**[0090.0.0.0]** The chemically pure respective fine chemical or chemically pure compositions comprising the respective fine chemical may also be produced by the process described above. To this end, the respective fine chemical or the compositions are isolated in the known manner from an organism according to the invention, such as the microorganisms, non-human animal or the plants, and/or their culture medium in which or on which the organisms had been grown. These chemically pure respective fine chemical or said compositions are advantageous for applications in the field of the food industry, the cosmetics industry or the pharmaceutical industry.

**[0091.0.0.0]** Thus, the content of plant components and preferably also further impurities is as low as possible, and the abovementioned respective fine chemical is obtained in as pure form as possible. In these applications, the content of plant components advantageously amounts to less than 10%, preferably 1%, more preferably 0.1 %, very especially preferably 0.01 % or less.

**[0092.0.0.0]** Accordingly, the respective fine chemical produced by the present invention is at least 0,1% by weight pure, preferably more than 1% by weight pure, more preferred 10% by weight pure, even more preferred are more than 50, 60, 70 or 80% by weight purity, even more preferred are more than 90 weight-% purity, most preferred are 95% by weight, 99% by weight or more.

**[0093.0.0.0]** In this context, the amount of the respective fine chemical in a cell of the invention may be increased according to the process of the invention by at least a factor of 1.1, preferably at least a factor of 1.5; 2; or 5, especially preferably by at least a factor of 10 or 30, very especially preferably by at least a factor of 50, in comparison with the wild type, control or reference. Preferably, said increase is found a tissue, more preferred in an organism or in a harvestable part thereof.

**[0094.0.0.0]** In principle, the respective fine chemicals produced can be increased in two ways by the process according to the invention. The pool of free respective fine chemicals, in particular of the free respective fine chemical, and/or the content of protein-bound respective fine chemicals, in particular of the protein-bound respective fine chemical may advantageously be increased.

**[0095.0.0.0]** It may be advantageous to increase the pool of free amino acids in the transgenic organisms by the process according to the invention in order to isolate high amounts of the pure respective fine chemical.

**[0096.0.0.0]** In another preferred embodiment of the invention a combination of the increased expression of the nucleic acid sequence or the protein of the invention together with the transformation of a protein or polypeptid, which functions as a sink for the desired amino acid for example methionine, lysine or threonine in the organism is useful to increase the production of the respective fine chemical (see US 5,589,616, WO 96/38574, WO 97/07665, WO 97/28247, US 4,886,878, US 5,082,993 and US 5,670,635). Galili et al., Transgenic Res. 2000 showed, that enhancing the synthesis of threonine by a feed back insensitive aspartate kinase did not lead only to in increase in free threonine but also in protein bound threonine.

**[0097.0.0.0]** In may also be advantageous to increase the content of the protein-bound respective fine chemical.

**[0098.0.0.0]** In a preferred embodiment, the respective fine chemical (methionine) and/or threonine are produced in accordance with the invention and, if desired, are isolated. The production of further amino acids such as lysine and of amino acid mixtures by the process according to the invention is advantageous.

**[0099.0.0.0]** In the case of the fermentation of microorganisms, the abovementioned amino acids may accumulate in the medium and/or the cells. If microorganisms are used in the process according to the invention, the fermentation broth can be processed after the cultivation. Depending on the requirement, all or some of the biomass can be removed from the fermentation broth by separation methods such as, for example, centrifugation, filtration, decanting or a combination of these methods, or else the biomass can be left in the fermentation broth. The fermentation broth can subsequently be reduced, or concentrated, with the aid of known methods such as, for example, rotary evaporator, thin-layer evaporator, falling film evaporator, by reverse osmosis or by nanofiltration. This concentrated fermentation broth can subsequently be processed by lyophilization, spray drying, spray granulation or by other methods.

**[0100.0.0.0]** To purify an amino acid, a product-containing fermentation broth from which the biomass has been separated may be subjected to chromatography with a suitable resin such as ion exchange resin for example anion or cation exchange resin, hydrophobic resin or hydrophilic resin for example epoxy resin, polyurethane resin or polyacrylamid resin, or resin for separation according to the molecular weight of the compounds for example polyvinyl chloride homopolymer resin or resins composed for example of polymers of acrylic acid, crosslinked with polyalkenyl ethers or divinyl glycol such as Carbopol®, Pemulen® and Noveon®. If necessary these chromatography steps may be repeated using the same or other chromatography resins. The skilled worker is familiar with the choice of suitable chromatography resins and their most effective use. The purified product may be concentrated by filtration or ultrafiltration and stored at a temperature, which ensures the maximum stability of the product.

**[0101.0.0.0]** The identity and purity of the compound(s) isolated can be determined by prior-art techniques. They encompass high-performance liquid chromatography (HPLC), gas chromatography (GC), spectroscopic methods, mass spectrometry (MS), staining methods, thin-layer chromatography, NIRS, enzyme assays or microbiological assays. These analytical methods are compiled in: Patek et al. (1994) Appl. Environ. Microbiol. 60:133-140; Malakhova et al. (1996) Biotekhnologiya 11 27-32; and Schmidt et al. (1998) Bioprocess Engineer. 19:67-70. Ulmann's Encyclopedia of Industrial Chemistry (1996) Bd. A27, VCH Weinheim, pp. 89-90, pp. 521-540, pp. 540-547, pp. 559-566, 575-581 and pp. 581-587; Michal, G (1999) Biochemical Pathways: An Atlas of Biochemistry and Molecular Biology, John Wiley and Sons; Fallon, A. et al. (1987) Applications of HPLC in Biochemistry in: Laboratory Techniques in Biochemistry and Molecular Biology, vol. 17.

**[0102.0.0.0]** Amino acids can for example be detected advantageously via HPLC separation in ethanolic extract as described by Geigenberger et al. (Plant Cell & Environ, 19, 1996: 43-55). Amino acids can be extracted with hot water. After filtration the extracts are diluted with water containing 20 mg/mL sodium acide. The separation and detection of the amino acids is performed using an anion exchange column and an electrochemical detector. Technical details can be taken from Y. Ding et al., 2002, Direct determination of free amino acids and sugars in green tea by anion-exchange chromatography with integrated pulsed amperometric detection, J Chromatogr A, (2002) 982; 237-244, or e.g. from Karchi et al., 1993, Plant J. 3: 721-727; Matthews MJ, 1997 (Lysine, threonine and methionine biosynthesis. In BK Singh, ed, Plant Amino Acids: Biochemistry and Biotechnology. Dekker, New York, pp 205-225; H Hesse and R Hoefgen. (2003) Molecular aspects of methionine biosynthesis. TIPS 8(259-262.

**[0103.0.0.0]** In a preferred embodiment, the present invention relates to a process for the production of the respective fine chemical comprising or generating in an organism or a part thereof the expression of at least one nucleic acid molecule comprising a nucleic acid molecule selected from the group consisting of:
a) nucleic acid molecule encoding, preferably at least the mature form, of a polypeptide having a sequence as indicated in Table II, columns 5 or 7, lines 1 to 5 and/or lines 334 to 338, or a fragment thereof, which confers an increase in the amount of the respective fine chemical in an organism or a part thereof;
b) nucleic acid molecule comprising, preferably at least the mature form, of a nucleic acid molecule having a sequence as indicated in Table I, columns 5 or 7, lines 1 to 5 and/or lines 334 to 338;
c) nucleic acid molecule whose sequence can be deduced from a polypeptide sequence encoded by a nucleic acid molecule of (a) or (b) as result of the degeneracy of the genetic code and conferring an increase in the amount of the respective fine chemical in an organism or a part thereof;
d) nucleic acid molecule encoding a polypeptide which has at least 50% identity with the amino acid sequence of the polypeptide encoded by the nucleic acid molecule of (a) to (c) and conferring an increase in the amount of the respective fine chemical in an organism or a part thereof;
e) nucleic acid molecule which hybridizes with a nucleic acid molecule of (a) to (c) under stringent hybridisation conditions and conferring an increase in the amount of the respective fine chemical in an organism or a part thereof;
f) nucleic acid molecule encoding a polypeptide, the polypeptide being derived by substituting, deleting and/or adding one or more amino acids of the amino acid sequence of the polypeptide encoded by the nucleic acid molecules (a) to (d), preferably to (a) to (c) and conferring an increase in the amount of the respective fine chemical in an organism or a part thereof;
g) nucleic acid molecule encoding a fragment or an epitope of a polypeptide which is encoded by one of the nucleic acid molecules of (a) to (e), preferably to (a) to (c) and conferring an increase in the amount of the respective fine chemical in an organism or a part thereof;
h) nucleic acid molecule comprising a nucleic acid molecule which is obtained by amplifying nucleic acid molecules from a cDNA library or a genomic library using the primers pairs having a sequence as indicated in Table III, columns 7, lines 1 to 5 and/or lines 334 to 338, and conferring an increase in the amount of the respective fine chemical in an organism or a part thereof;
i) nucleic acid molecule encoding a polypeptide which is isolated, e.g. from an expression library, with the aid of monoclonal antibodies against a polypeptide encoded by one of the nucleic acid molecules of (a) to (h), preferably to (a) to (c), and conferring an increase in the amount of the respective fine chemical in an organism or a part thereof;
j) nucleic acid molecule which encodes a polypeptide comprising the consensus sequence having a sequences as indicated in Table IV, columns 7, lines 1 to 5 and/or lines 334 to 338, and conferring an increase in the amount of the respective fine chemical in an organism or a part thereof ;
k) nucleic acid molecule comprising one or more of the nucleic acid molecule encoding the amino acid sequence of a polypeptide encoding a domain of a polypeptide indicated in Table II, columns 5 or 7, lines 1 to 5 and/or lines 334 to 338,and conferring an increase in the amount of the respective fine chemical in an organism or a part thereof; and
l) nucleic acid molecule which is obtainable by screening a suitable library under stringent conditions with a probe comprising one of the sequences of the nucleic acid molecule of (a) to (k), preferably to (a) to (c), or with a fragment of at least 15 nt, preferably 20 nt, 30 nt, 50 nt, 100 nt, 200 nt or 500 nt of the nucleic acid molecule characterized in (a) to (k), preferably to (a) to (c), and conferring an increase in the amount of the respective fine chemical in an organism or a part thereof;
or which comprises a sequence which is complementary thereto.

**[0103.1.0.0.]** In one embodiment, the nucleic acid molecule used in the process of the invention distinguishes over the sequence indicated in Table IA, columns 5 or 7, lines 1 to 5 and/or lines 334 to 338, by one or more nucleotides. In one embodiment, the nucleic acid molecule used in the process of the invention does not consist of the sequence shown in indicated in Table I A, columns 5 or 7, lines 1 to 5 and/or lines 334 to 338: In one embodiment, the nucleic acid molecule used in the process of the invention is less than 100%, 99,999%, 99,99%, 99,9% or 99% identical to a sequence indicated in Table I A, columns 5 or 7, lines 1 to 5 and/or lines 334 to 338. In another embodiment, the nucleic acid molecule does not encode a polypeptide of a sequence indicated in Table II A, columns 5 or 7, lines 1 to 5 and/or lines 334 to 338.

**[0103.2.0.0.]** In one embodiment, the nucleic acid molecule used in the process of the invention distinguishes over the sequence indicated in Table I B, columns 5 or 7, lines 1 to 5 and/or lines 334 to 338, by one or more nucleotides. In one embodiment, the nucleic acid molecule used in the process of the invention does not consist of the sequence shown in indicated in Table I B, columns 5 or 7, lines 1 to 5 and/or lines 334 to 338: In one embodiment, the nucleic acid molecule used in the process of the invention is less than 100%, 99,999%, 99,99%, 99,9% or 99% identical to a sequence indicated in Table I B, columns 5 or 7, lines 1 to 5 and/or lines 334 to 338. In another embodiment, the nucleic acid molecule does not encode a polypeptide of a sequence indicated in Table II B, columns 5 or 7, lines 1 to 5 and/or lines 334 to 338.

**[0104.0.0.0]** In one embodiment, the nucleic acid molecule of the invention or used in the process of the invention distinguishes over the sequence indicated in Table I, columns 5 or 7, lines 1 to 5 and/or lines 334 to 338, by one or more nucleotides. In one embodiment, the nucleic acid molecule of the invention or the nucleic acid used in the process of the invention does not consist of the sequence shown in indicated in Table I, columns 5 or 7, lines 1 to 5 and/or lines 334 to 338: In one embodiment, the nucleic acid molecule of the present invention is less than 100%, 99,999%, 99,99%, 99,9% or 99% identical to a sequence indicated in Table I, columns 5 or 7, lines 1 to 5 and/or lines 334 to 338. In another embodiment, the nucleic acid molecule does not encode a polypeptide of a sequence indicated in Table II, columns 5 or 7, lines 1 to 5 and/or lines 334 to 338.

**[0105.0.0.0]** Unless otherwise specified, the terms "polynucleotides", "nucleic acid" and "nucleic acid molecule" are interchangeably in the present context. Unless otherwise specified, the terms "peptide", "polypeptide" and "protein" are interchangeably in the present context. The term "sequence" may relate to polynucleotides, nucleic acids, nucleic acid molecules, peptides, polypeptides and proteins, depending on the context in which the term "sequence" is used. The terms "gene(s)", "polynucleotide", "nucleic acid sequence", "nucleotide sequence", or "nucleic acid molecule(s)" as used herein refers to a polymeric form of nucleotides of any length, either ribonucleotides or deoxyribonucleotides. The terms refer only to the primary structure of the molecule.

**[0106.0.0.0]** Thus, The terms "gene(s)", "polynucleotide", "nucleic acid sequence", "nucleotide sequence", or "nucleic acid molecule(s)" as used herein include double- and single-stranded DNA and RNA. They also include known types of modifications, for example, methylation, "caps", substitutions of one or more of the naturally occurring nucleotides with an analog. Preferably, the DNA or RNA sequence of the invention comprises a coding sequence encoding the herein defined polypeptide.

**[0107.0.0.0]** A "coding sequence" is a nucleotide sequence, which is transcribed into mRNA and/or translated into a polypeptide when placed under the control of appropriate regulatory sequences. The boundaries of the coding sequence are determined by a translation start codon at the 5'-terminus and a translation stop codon at the 3'-terminus. A coding sequence can include, but is not limited to mRNA, cDNA, recombinant nucleotide sequences or genomic DNA, while introns may be present as well under certain circumstances.

**[0108.0.0.0]** Nucleic acid molecules with the sequence as indicated in Table I, columns 5 or 7, lines 1 to 5 and/or lines 334 to 338, nucleic acid molecules which are derived from a amino acid sequences as indicated in Table II, columns 5 or 7, lines 1 to 5 and/or lines 334 to 338 or from polypeptides comprising the consensus sequence as indicated in Table IV, columns 7, lines 1 to 5 and/or lines 334 to 338, or their derivatives or homologues encoding polypeptides with the enzymatic or biological activity of a polypeptide as indicated in Table II, column 3, 5 or 7, lines 1 to 5 and/or lines 334 to 338 or e.g. conferring a increase of the respective fine chemical after increasing its expression or activity are advantageously increased in the process according to the invention.

**[0109.0.0.0]** In one embodiment, said sequences are cloned into nucleic acid constructs, either individually or in combination. These nucleic acid constructs enable an optimal synthesis of the respective fine chemical produced in the process according to the invention.

**[0110.0.0.0]** Nucleic acid molecules, which are advantageous for the process according to the invention and which encode polypeptides with an activity of a polypeptide of the invention or the polypeptide used in the method of the invention or used in the process of the invention, e.g. of a protein as indicated in Table II, column 5, lines 1 to 5 and/or lines 334 to 338 or being encoded by a nucleic acid molecule indicated in Table I, column 5, lines 1 to 5 and/or lines 334 to 338 or of its homologs, e.g. as indicated in Table II, column 7, lines 1 to 5 and/or lines 334 to 338, can be determined from generally accessible databases.

**[0111.0.0.0]** Those, which must be mentioned, in particular in this context are general gene databases such as the EMBL database (Stoesser G. et al., Nucleic Acids Res 2001, Vol. 29, 17-21), the GenBank database (Benson D.A. et al., Nucleic Acids Res 2000, Vol. 28,15-18), or the PIR database (Barker W. C. et al., Nucleic Acids Res. 1999, Vol. 27, 39-43). It is furthermore possible to use organism-specific gene databases for determining advantageous sequences, in the case of yeast for example advantageously the SGD database (Cherry J. M. et al., Nucleic Acids Res. 1998, Vol. 26, 73-80) or the MIPS database (Mewes H.W. et al., Nucleic Acids Res. 1999, Vol. 27, 44-48), in the case of E. coli the GenProtEC database (http://web.bham.ac.uk/bcm4ght6/res.html), and in the case of Arabidopsis the TAIR-database (Huala, E. et al., Nucleic Acids Res. 2001 Vol. 29(1), 102-5) or the MIPS database.

**[0112.0.0.0]** The nucleic acid molecules used in the process according to the invention take the form of isolated nucleic acid sequences, which encode polypeptides with an activity of a polypeptide as indicated in Table **I,** column3, lines 1 to 5 and/or lines 334 to 338 or having the sequence of a polypeptide as indicated in Table II, columns 5 and 7, lines 1 to 5 and/or lines 334 to 338 and conferring an increase of the respective fine chemical.

**[0113.0.0.0]** The nucleic acid sequence(s) used in the process for the production of the respective fine chemical in transgenic organisms originate advantageously from an eukaryote but may also originate from a prokaryote or an archebacterium, thus it can derived from e.g. a microorganism, an animal or a plant.

**[0114.0.0.0]** For the purposes of the invention, as a rule the plural is intended to encompass the singular and vice versa.

**[0115.0.0.0]** In order to improve the introduction of the nucleic acid sequences and the expression of the sequences in the transgenic organisms, which are used in the process, the nucleic acid sequences are incorporated into a nucleic acid construct and/or a vector. In addition to the herein described sequences which are used in the process according to the invention, further nucleic acid sequences, advantageously of biosynthesis genes of the respective fine chemical produced in the process according to the invention, may additionally be present in the nucleic acid construct or in the vector and may be introduced into the organism together. However, these additional sequences may also be introduced into the organisms via other, separate nucleic acid constructs or vectors.

**[0116.0.0.0]** Using the herein mentioned cloning vectors and transformation methods such as those which are published and cited in: Plant Molecular Biology and Biotechnology (CRC Press, Boca Raton, Florida), chapter 6/7, pp. 71-119 (1993); F.F. White, Vectors for Gene Transfer in Higher Plants; in: Transgenic Plants, vol. 1, Engineering and Utilization, Ed.: Kung and R. Wu, Academic Press, 1993, 15-38; B. Jenes et al., Techniques for Gene Transfer, in: Transgenic Plants, vol. 1, Engineering and Utilization, Ed.: Kung and R. Wu, Academic Press (1993), 128-143; Potrykus, Annu. Rev. Plant Physiol. Plant Molec. Biol. 42 (1991), 205-225)) and further cited below, the nucleic acids may be used for the recombinant modification of a wide range of organisms, in particular prokaryotic or eukaryotic microorganisms or plants, so that they become a better and more efficient producer of the respective fine chemical produced in the process according to the invention. This improved production, or production efficiency, of the respective fine chemical or products derived there from, such as modified proteins, can be brought about by a direct effect of the manipulation or by an indirect effect of this manipulation.

**[0117.0.0.0]** In one embodiment, the nucleic acid molecule according to the invention originates from a plant, such as a plant selected from the families Aceraceae, Anacardiaceae, Apiaceae, Asteraceae, Brassicaceae, Cactaceae, Cucurbitaceae, Euphorbiaceae, Fabaceae, Malvaceae, Nymphaeaceae, Papaveraceae, Rosaceae, Salicaceae, Solanaceae, Arecaceae, Bromeliaceae, Cyperaceae, Iridaceae, Liliaceae, Orchidaceae, Gentianaceae, Labiaceae, Magnoliaceae, Ranunculaceae, Carifolaceae, Rubiaceae, Scrophulariaceae, Caryophyllaceae, Ericaceae, Polygonaceae, Violaceae, Juncaceae or Poaceae and preferably from a plant selected from the group of the families Apiaceae, Asteraceae, Brassicaceae, Cucurbitaceae, Fabaceae, Papaveraceae, Rosaceae, Solanaceae, Liliaceae or Poaceae. Preferred are crop plants and in particular plants mentioned herein above as host plants such as the families and genera mentioned above for example preferred the species *Anacardium occidentale, Calendula officinalis, Carthamus tinctorius, Cichorium intybus, Cynara scolymus, Helianthus annus, Tagetes lucida, Tagetes erecta, Tagetes tenuifolia; Daucus carota; Corylus avellana, Corylus colurna, Borago officinalis; Brassica napus, Brassica rapa* ssp., *Sinapis arvensis Brassica juncea, Brassica juncea var. juncea, Brassica juncea var. crispifolia, Brassica juncea var. foliosa, Brassica nigra, Brassica sinapioides, Melanosinapis communis*, *Brassica oleracea*, Arabidopsis thaliana, *Anana comosus, Ananas ananas, Bromelia comosa, Carica papaya, Cannabis sative, Ipomoea batatus, Ipomoea pandurata, Convolvulus batatas, Convolvulus tiliaceus, Ipomoea fastigiata, Ipomoea tiliacea, Ipomoea triloba, Convolvulus panduratus, Beta vulgaris, Beta vulgaris var. altissima*, *Beta vulgaris var. vulgaris, Beta maritima, Beta vulgaris var. perennis, Beta vulgaris var. conditiva, Beta vulgaris var. esculenta*, *Cucurbita maxima, Cucurbita mixta, Cucurbita* pepo, *Cucurbita moschata, Olea europaea, Manihot utilissima, Janipha manihot*,, *Jatropha manihot., Manihot aipil, Manihot dulcis, Manihot manihot, Manihot melanobasis, Manihot esculenta*, *Ricinus communis, Pisum sativum, Pisum arvense, Pisum humile, Medicago sativa, Medicago falcata, Medicago varia, Glycine max Dolichos soja, Glycine gracilis, Glycine hispida, Phaseolus max, Soja hispida, Soja max*, *Cocos nucifera, Pelargonium grossularioides, Oleum cocoas, Laurus nobilis, Persea americana, Arachis hypogaea*, *Linum usitatissimum, Linum humile, Linum austriacum, Linum bienne, Linum angustifolium, Linum catharticum, Linum flavum, Linum grandiflorum, Adenolinum grandiflorum, Linum lewisii, Linum narbonense, Linum perenne, Linum perenne var. lewisii, Linum pratense, Linum trigynum, Punica granatum, Gossypium hirsutum, Gossypium arboreum, Gossypium barbadense, Gossypium herbaceum, Gossypium thurberi, Musa nana, Musa acuminata, Musa paradisiaca, Musa* spp., *Elaeis guineensis*, *Papaver orientale, Papaver rhoeas, Papaver dubium*, *Sesamum indicum*, *Piper aduncum, Piper amalago, Piper angustifolium, Piper auritum, Piper betel, Piper cubeba, Piper longum, Piper nigrum, Piper retrofractum, Artanthe adunca, Artanthe elongata, Peperomia elongata, Piper elongatum, Steffensia elongata*, , *Hordeum vulgare, Hordeum jubatum, Hordeum murinum, Hordeum secalinum, Hordeum distichon Hordeum aegiceras, Hordeum hexastichon., Hordeum hexastichum, Hordeum irregulare, Hordeum sativum, Hordeum secalinum,* Avena sativa, *Avena fatua, Avena byzantina, Avena fatua var. sativa, Avena hybrida, Sorghum bicolor, Sorghum halepense, Sorghum saccharatum, Sorghum vulgare, Andropogon drummondii, Holcus bicolor, Holcus sorghum, Sorghum aethiopicum, Sorghum arundinaceum, Sorghum caffrorum, Sorghum cernuum, Sorghum dochna, Sorghum drummondii, Sorghum durra, Sorghum guineense, Sorghum lanceolatum, Sorghum nervosum, Sorghum saccharatum, Sorghum subglabrescens, Sorghum verticilliflorum, Sorghum vulgare, Holcus halepensis, Sorghum miliaceum* millet, *Panicum militaceum, Zea mays, Triticum aestivum, Triticum durum, Triticum turgidum, Triticum hybernum, Triticum macha, Triticum sativum* or *Triticum vulgare, Cofea spp*., *Coffea arabica, Coffea canephora, Coffea liberica, Capsicum annuum, Capsicum annuum var. glabriusculum, Capsicum frutescens, Capsicum annuum, Nicotiana tabacum, Solanum tuberosum, Solanum melongena, Lycopersicon esculentum, Lycopersicon lycopersicum., Lycopersicon pyriforme, Solanum integrifolium, Solanum lycopersicum Theobroma cacao* or *Camellia sinensis.*

**[0118.0.0.0]** In one embodiment, the nucleic acid molecule sequence originates advantageously from a microorganism as mentioned above under host organism such as a fungus for example the genera Aspergillus, Penicillium or Claviceps or from yeasts such as the genera Pichia, Torulopsis, Hansenula, Schizosaccharomyces, Candida, Rhodotorula or Saccharomyces, very especially advantageously from the yeast of the family Saccharomycetaceae, such as the advantageous genus Saccharomyces and the very advantageous genus and species Saccharomyces cerevisiae for the production of the respective fine chemical in microorganims.

**[0119.0.0.0]** The skilled worker knows other suitable sources for the production of respective fine chemicals, which present also useful nucleic acid molecule sources. They include in general all prokaryotic or eukaryotic cells, preferably unicellular microorganisms, such as fungi like the genus Claviceps or Aspergillus or gram-positive bacteria such as the genera Bacillus, Corynebacterium, Micrococcus, Brevibacterium, Rhodococcus, Nocardia, Caseobacter or Arthrobacter or gram-negative bacteria such as the genera Escherichia, Flavobacterium or Salmonella, or yeasts such as the genera Rhodotorula, Hansenula or Candida.

**[0120.0.0.0]** Production strains which are especially advantageously selected in the process according to the invention are microorganisms selected from the group of the families Actinomycetaceae, Bacillaceae, Brevibacteriaceae, Corynebacteriaceae, Enterobacteriacae, Gordoniaceae, Micrococcaceae, Mycobacteriaceae, Nocardiaceae, Pseudomonaceae, Rhizobiaceae, Streptomycetaceae, Chaetomiaceae, Choanephoraceae, Cryptococcaceae, Cunninghamellaceae, Demetiaceae, Moniliaceae, Mortierellaceae, Mucoraceae, Pythiaceae, Sacharomycetaceae, Saprolegniaceae, Schizosacharomycetaceae, Sodariaceae, Sporobolomycetaceae, Tuberculariaceae, Adelotheciaceae, Dinophyceae, Ditrichaceae and Prasinophyceaeor of the genera and species consisting of Hansenula anomala, Candida utilis, Claviceps purpurea, Bacillus circulans, Bacillus subtilis, Bacillus sp., Brevibacterium albidum, Brevibacterium album, Brevibacterium cerinum, Brevibacterium flavum, Brevibacterium glutamigenes, Brevibacterium iodinum, Brevibacterium ketoglutamicum, Brevibacterium lactofermentum, Brevibacterium linens, Brevibacterium roseum, Brevibacterium saccharolyticum, Brevibacterium sp., Corynebacterium acetoacidophilum, Corynebacterium acetoglutamicum, Corynebacterium ammoniagenes, Corynebacterium glutamicum (= Micrococcus glutamicum), Corynebacterium melassecola, Corynebacterium sp. or Escherichia coli, specifically Escherichia coli K12 and its described strains.

**[0121.0.0.0]** However, it is also possible to use artificial sequences, which differ in one or more bases from the nucleic acid sequences found in organisms, or in one or more amino acid molecules from polypeptide sequences found in organisms, in particular from the polypeptide sequences indicated in Table II, columns 5 or 7, lines 1 to 5 and/or lines 334 to 338 or the functional homologues thereof as described herein, preferably conferring above-mentioned activity, i.e. conferring a increase of the respective fine chemical after increasing its activity.

**[0122.0.0.0]** In the process according to the invention nucleic acid sequences can be used, which, if appropriate, contain synthetic, non-natural or modified nucleotide bases, which can be incorporated into DNA or RNA. Said synthetic, non-natural or modified bases can for example increase the stability of the nucleic acid molecule outside or inside a cell. The nucleic acid molecules of the invention can contain the same modifications as aforementioned.

**[0123.0.0.0]** As used in the present context the term "nucleic acid molecule" may also encompass the untranslated sequence located at the 3' and at the 5' end of the coding gene region, for example at least 500, preferably 200, especially preferably 100, nucleotides of the sequence upstream of the 5' end of the coding region and at least 100, preferably 50, especially preferably 20, nucleotides of the sequence downstream of the 3' end of the coding gene region. It is often advantageous only to choose the coding region for cloning and expression purposes.

**[0124.0.0.0]** Preferably, the nucleic acid molecule used in the process according to the invention or the nucleic acid molecule of the invention is an isolated nucleic acid molecule.

**[0125.0.0.0]** An "isolated" polynucleotide or nucleic acid molecule is separated from other polynucleotides or nucleic acid molecules, which are present in the natural source of the nucleic acid molecule. An isolated nucleic acid molecule may be a chromosomal fragment of several kb, or preferably, a molecule only comprising the coding region of the gene. Accordingly, an isolated nucleic acid molecule of the invention may comprise chromosomal regions, which are adjacent 5' and 3' or further adjacent chromosomal regions, but preferably comprises no such sequences which naturally flank the nucleic acid molecule sequence in the genomic or chromosomal context in the organism from which the nucleic acid molecule originates (for example sequences which are adjacent to the regions encoding the 5'- and 3'-UTRs of the nucleic acid molecule). In various embodiments, the isolated nucleic acid molecule used in the process according to the invention may, for example comprise less than approximately 5 kb, 4 kb, 3 kb, 2 kb, 1 kb, 0.5 kb or 0.1 kb nucleotide sequences which naturally flank the nucleic acid molecule in the genomic DNA of the cell from which the nucleic acid molecule originates.

**[0126.0.0.0]** The nucleic acid molecules used in the process, for example the polynucleotides of the invention or of a part thereof can be isolated using molecular-biological standard techniques and the sequence information provided herein. Also, for example a homologous sequence or homologous, conserved sequence regions at the DNA or amino acid level can be identified with the aid of comparison algorithms. The former can be used as hybridization probes under standard hybridization techniques (for example those described in Sambrook et al., Molecular Cloning: A Laboratory Manual. 2nd Ed., Cold Spring Harbor Laboratory, Cold Spring Harbor Laboratory Press, Cold Spring Harbor, NY, 1989) for isolating further nucleic acid sequences useful in this process.

**[0127.0.0.0]** A nucleic acid molecule encompassing a complete sequence of the nucleic acid molecules used in the process, for example the polynucleotide of the invention, or a part thereof may additionally be isolated by polymerase chain reaction, oligonucleotide primers based on this sequence or on parts thereof being used. For example, a nucleic acid molecule comprising the complete sequence or part thereof can be isolated by polymerase chain reaction using oligonucleotide primers which have been generated on the basis of this sequence for example, mRNA can be isolated from cells (for example by means of the guanidinium thiocyanate extraction method of Chirgwin et al. (1979) Biochemistry 18:5294-5299) and cDNA can be generated by means of reverse transcriptase (for example Moloney MLV reverse transcriptase, available from Gibco/BRL, Bethesda, MD, or AMV reverse transcriptase, obtainable from Seikagaku America, Inc., St.Petersburg, FL).

**[0128.0.0.0]** Synthetic oligonucleotide primers for the amplification, e.g. as the pairs indicated in Table III, columns 7, lines 1 to 5 and/or lines 334 to 338, by means of polymerase chain reaction can be generated on the basis of a sequence shown herein, for example the sequence as indicated in Table I, columns 5 or 7, lines 1 to 5 and/or lines 334 to 338 or the sequences derived from sequences as indicated in Table II, columns 5 or 7, lines 1 to 5 and/or lines 334 to 338.

**[0129.0.0.0]** Moreover, it is possible to identify conserved regions from various organisms by carrying out protein sequence alignments with the polypeptide used in the process of the invention, in particular with sequences of the polypeptide of the invention or the polypeptide used in the method of the invention, from which conserved regions, and in turn, degenerate primers can be derived. Conserved region for the polypeptide of the invention or the polypeptide used in the method of the invention are indicated in the alignments shown in the figures. Conserved regions are those, which show a very little variation in the amino acid sequence in one particular position of several homologs from different origin. The consensus sequences indicated in Table IV, columns 7, lines 1 to 5 and/or lines 334 to 338 are derived from said alignments.

**[0130.0.0.0]** Degenerated primers can then be utilized by PCR for the amplification of fragments of novel proteins having above-mentioned activity, e.g. conferring the increase of the respective fine chemical after increasing its expression or activity or further functional homologs of the polypeptide of the invention or the polypeptide used in the method of the invention from other organisms.

**[0131.0.0.0]** These fragments can then be utilized as hybridization probe for isolating the complete gene sequence. As an alternative, the missing 5' and 3' sequences can be isolated by means of RACE-PCR (rapid amplification of cDNA ends). A nucleic acid molecule according to the invention can be amplified using cDNA or, as an alternative, genomic DNA as template and suitable oligonucleotide primers, following standard PCR amplification techniques. The nucleic acid molecule amplified thus can be cloned into a suitable vector and characterized by means of DNA sequence analysis. Oligonucleotides, which correspond to one of the nucleic acid molecules used in the process, can be generated by standard synthesis methods, for example using an automatic DNA synthesizer.

**[0132.0.0.0]** Nucleic acid molecules which are advantageously for the process according to the invention can be isolated based on their homology to the nucleic acid molecules disclosed herein using the sequences or part thereof as hybridization probe and following standard hybridization techniques under stringent hybridization conditions. In this context, it is possible to use, for example, isolated nucleic acid molecules of at least 15, 20, 25, 30, 35, 40, 50, 60 or more nucleotides, preferably of at least 15, 20 or 25 nucleotides in length which hybridize under stringent conditions with the above-described nucleic acid molecules, in particular with those which encompass a nucleotide sequence of the nucleic acid molecule used in the process of the invention or encoding a protein used in the invention or of the nucleic acid molecule of the invention. Nucleic acid molecules with 30, 50, 100, 250 or more nucleotides may also be used.

**[0133.0.0.0]** The term "homology" means that the respective nucleic acid molecules or encoded proteins are functionally and/or structurally equivalent. The nucleic acid molecules that are homologous to the nucleic acid molecules described above and that are derivatives of said nucleic acid molecules are, for example, variations of said nucleic acid molecules which represent modifications having the same biological function, in particular encoding proteins with the same or substantially the same biological function. They may be naturally occurring variations, such as sequences from other plant varieties or species, or mutations. These mutations may occur naturally or may be obtained by mutagenesis techniques. The allelic variations may be naturally occurring allelic variants as well as synthetically produced or genetically engineered variants. Structurally equivalents can, for example, be identified by testing the binding of said polypeptide to antibodies or computer based predictions. Structurally equivalent have the similar immunological characteristic, e.g. comprise similar epitopes.

**[0134.0.0.0]** By "hybridizing" it is meant that such nucleic acid molecules hybridize under conventional hybridization conditions, preferably under stringent conditions such as described by, e.g., Sambrook (Molecular Cloning; A Laboratory Manual, 2nd Edition, Cold Spring Harbor Laboratory Press, Cold Spring Harbor, NY (1989)) or in Current Protocols in Molecular Biology, John Wiley & Sons, N. Y. (1989), 6.3.1-6.3.6.

**[0135.0.0.0]** According to the invention, DNA as well as RNA molecules of the nucleic acid of the invention can be used as probes. Further, as template for the identification of functional homologues Northern blot assays as well as Southern blot assays can be performed. The Northern blot assay advantageously provides further information about the expressed gene product: e.g. expression pattern, occurrence of processing steps, like splicing and capping, etc. The Southern blot assay provides additional information about the chromosomal localization and organization of the gene encoding the nucleic acid molecule of the invention.

**[0136.0.0.0]** A preferred, nonlimiting example of stringent hydridization conditions are hybridizations in 6 x sodium chloride/sodium citrate (= SSC) at approximately 45°C, followed by one or more wash steps in 0.2 x SSC, 0.1 % SDS at 50 to 65°C, for example at 50°C, 55°C or 60°C. The skilled worker knows that these hybridization conditions differ as a function of the type of the nucleic acid and, for example when organic solvents are present, with regard to the temperature and concentration of the buffer. The temperature under "standard hybridization conditions" differs for example as a function of the type of the nucleic acid between 42°C and 58°C, preferably between 45°C and 50°C in an aqueous buffer with a concentration of 0.1 x 0.5 x, 1 x, 2x, 3x, 4x or 5 x SSC (pH 7.2). If organic solvent(s) is/are present in the abovementioned buffer, for example 50% formamide, the temperature under standard conditions is approximately 40°C, 42°C or 45°C. The hybridization conditions for DNA:DNA hybrids are preferably for example 0.1 x SSC and 20°C, 25°C, 30°C, 35°C, 40°C or 45°C, preferably between 30°C and 45°C. The hybridization conditions for DNA:RNA hybrids are preferably for example 0.1 x SSC and 30°C, 35°C, 40°C, 45°C, 50°C or 55°C, preferably between 45°C and 55°C. The abovementioned hybridization temperatures are determined for example for a nucleic acid approximately 100 bp (= base pairs) in length and a G + C content of 50% in the absence of formamide. The skilled worker knows to determine the hybridization conditions required with the aid of textbooks, for example the ones mentioned above, or from the following textbooks: Sambrook et al., "Molecular Cloning", Cold Spring Harbor Laboratory, 1989; Hames and Higgins (Ed.) 1985, "Nucleic Acids Hybridization: A Practical Approach", IRL Press at Oxford University Press, Oxford; Brown (Ed.) 1991, "Essential Molecular Biology: A Practical Approach", IRL Press at Oxford University Press, Oxford.

**[0137.0.0.0]** A further example of one such stringent hybridization condition is hybridization at 4XSSC at 65°C, followed by a washing in 0.1 XSSC at 65°C for one hour. Alternatively, an exemplary stringent hybridization condition is in 50 % formamide, 4XSSC at 42°C. Further, the conditions during the wash step can be selected from the range of conditions delimited by low-stringency conditions (approximately 2X SSC at 50°C) and high-stringency conditions (approximately 0.2X SSC at 50°C, preferably at 65°C) (20X SSC: 0.3M sodium citrate, 3M NaCl, pH 7.0). In addition, the temperature during the wash step can be raised from low-stringency conditions at room temperature, approximately 22°C, to higher-stringency conditions at approximately 65°C. Both of the parameters salt concentration and temperature can be varied simultaneously, or else one of the two parameters can be kept constant while only the other is varied. Denaturants, for example formamide or SDS, may also be employed during the hybridization. In the presence of 50% formamide, hybridization is preferably effected at 42°C. Relevant factors like i) length of treatment, ii) salt conditions, iii) detergent conditions, iv) competitor DNAs, v) temperature and vi) probe selection can be combined case by case so that not all possibilities can be mentioned herein.
Thus, in a preferred embodiment, Northern blots are prehybridized with Rothi-Hybri-Quick buffer (Roth, Karlsruhe) at 68°C for 2h. Hybridization with radioactive labelled probe is done overnight at 68°C. Subsequent washing steps are performed at 68°C with 1xSSC.
For Southern blot assays the membrane is prehybridized with Rothi-Hybri-Quick buffer (Roth, Karlsruhe) at 68°C for 2h. The hybridization with radioactive labelled probe is conducted over night at 68°C. Subsequently the hybridization buffer is discarded and the filter shortly washed using 2xSSC; 0,1% SDS. After discarding the washing buffer new 2xSSC; 0,1% SDS buffer is added and incubated at 68°C for 15 minutes. This washing step is performed twice followed by an additional washing step using 1xSSC; 0,1% SDS at 68°C for 10 min.

**[0138.0.0.0]** Some further examples of conditions for DNA hybridization (Southern blot assays) and wash step are shown herein below:
(1) Hybridization conditions can be selected, for example, from the following conditions:
   a) 4X SSC at 65°C,
   b) 6X SSC at 45°C,
   c) 6X SSC, 100 mg/ml denatured fragmented fish sperm DNA at 68°C,
   d) 6X SSC, 0.5% SDS, 100 mg/ml denatured salmon sperm DNA at 68°C,
   e) 6X SSC, 0.5% SDS, 100 mg/ml denatured fragmented salmon sperm DNA, 50% formamide at 42°C,
   f) 50% formamide, 4X SSC at 42°C,
   g) 50% (vol/vol) formamide, 0.1% bovine serum albumin, 0.1% Ficoll, 0.1% polyvinylpyrrolidone, 50 mM sodium phosphate buffer pH 6.5, 750 mM NaCl, 75 mM sodium citrate at 42°C,
   h) 2X or 4X SSC at 50°C (low-stringency condition), or
   i) 30 to 40% formamide, 2X or 4X SSC at 42°C (low-stringency condition).
(2) Wash steps can be selected, for example, from the following conditions:
   a) 0.015 M NaCl/0.0015 M sodium citrate/0.1% SDS at 50°C.
   b) 0.1 X SSC at 65°C.
   c) 0.1 X SSC, 0.5 % SDS at 68°C.
   d) 0.1 X SSC, 0.5% SDS, 50% formamide at 42°C.
   e) 0.2X SSC, 0.1 % SDS at 42°C.
   f) 2X SSC at 65°C (low-stringency condition).

**[0139.0.0.0]** Polypeptides having above-mentioned activity, i.e. conferring the respective fine chemical increase, derived from other organisms, can be encoded by other DNA sequences which hybridize to a sequences indicated in Table I, columns 5 or 7, lines 1 to 5 and/or lines 334 to 338, preferably of Table I B, columns 5 or 7, lines 1 to 5 and/or lines 334 to 338 under relaxed hybridization conditions and which code on expression for peptides having the methionine increasing activity.

**[0140.0.0.0]** Further, some applications have to be performed at low stringency hybridisation conditions, without any consequences for the specificity of the hybridisation. For example, a Southern blot analysis of total DNA could be probed with a nucleic acid molecule of the present invention and washed at low stringency (55°C in 2xSSPE0,1% SDS). The hybridisation analysis could reveal a simple pattern of only genes encoding polypeptides of the present invention or used in the process of the invention, e.g. having herein-mentioned activity of increasing the respective fine chemical . A further example of such low-stringent hybridization conditions is 4XSSC at 50°C or hybridization with 30 to 40% formamide at 42°C. Such molecules comprise those which are fragments, analogues or derivatives of the polypeptide of the invention or used in the process of the invention and differ, for example, by way of amino acid and/or nucleotide deletion(s), insertion(s), substitution (s), addition(s) and/or recombination (s) or any other modification(s) known in the art either alone or in combination from the above-described amino acid sequences or their underlying nucleotide sequence(s). However, it is preferred to use high stringency hybridisation conditions.

**[0141.0.0.0]** Hybridization should advantageously be carried out with fragments of at least 5, 10, 15, 20, 25, 30, 35 or 40 bp, advantageously at least 50, 60, 70 or 80 bp, preferably at least 90, 100 or 110 bp. Most preferably are fragments of at least 15, 20, 25 or 30 bp. Preferably are also hybridizations with at least 100 bp or 200, very especially preferably at least 400 bp in length. In an especially preferred embodiment, the hybridization should be carried out with the entire nucleic acid sequence with conditions described above.

**[0142.0.0.0]** The terms "fragment", "fragment of a sequence" or "part of a sequence" mean a truncated sequence of the original sequence referred to. The truncated sequence (nucleic acid or protein sequence) can vary widely in length; the minimum size being a sequence of sufficient size to provide a sequence with at least a comparable function and/or activity of the original sequence referred to or hybridising with the nucleic acid molecule of the invention or the nucleic acid molecule used in the method of the invention or used in the process of the invention under stringent conditions, while the maximum size is not critical. In some applications, the maximum size usually is not substantially greater than that required to provide the desired activity and/or function(s) of the original sequence.

**[0143.0.0.0]** Typically, the truncated amino acid sequence will range from about 5 to about 310 amino acids in length. More typically, however, the sequence will be a maximum of about 250 amino acids in length, preferably a maximum of about 200 or 100 amino acids. It is usually desirable to select sequences of at least about 10, 12 or 15 amino acids, up to a maximum of about 20 or 25 amino acids.

**[0144.0.0.0]** The term "epitope" relates to specific immunoreactive sites within an antigen, also known as antigenic determinates. These epitopes can be a linear array of monomers in a polymeric composition - such as amino acids in a protein - or consist of or comprise a more complex secondary or tertiary structure. Those of skill will recognize that immunogens (i.e., substances capable of eliciting an immune response) are antigens; however, some antigen, such as haptens, are not immunogens but may be made immunogenic by coupling to a carrier molecule. The term "antigen" includes references to a substance to which an antibody can be generated and/or to which the antibody is specifically immunoreactive.

**[0145.0.0.0]** In one embodiment the present invention relates to a epitope of the polypeptide of the present invention or used in the process of the present invention and conferring above mentioned activity, preferably conferring an increase in the respective fine chemical.

**[0146.0.0.0]** The term "one or several amino acids" relates to at least one amino acid but not more than that number of amino acids, which would result in a homology of below 50% identity. Preferably, the identity is more than 70% or 80%, more preferred are 85%, 90%, 91%, 92%, 93%, 94% or 95%, even more preferred are 96%, 97%, 98%, or 99% identity.

**[0147.0.0.0]** Further, the nucleic acid molecule of the invention comprises a nucleic acid molecule, which is a complement of one of the nucleotide sequences of above mentioned nucleic acid molecules or a portion thereof. A nucleic acid molecule which is complementary to one of the nucleotide sequences indicated in Table I, columns 5 or 7, lines 1 to 5 and/or lines 334 to 338, preferably of Table I B, columns 5 or 7, lines 1 to 5 and/or lines 334 to 338 is one which is sufficiently complementary to one of said nucleotide sequences such that it can hybridize to one of said nucleotide sequences thereby forming a stable duplex. Preferably, the hybridisation is performed under stringent hybridization conditions. However, a complement of one of the herein disclosed sequences is preferably a sequence complement thereto according to the base pairing of nucleic acid molecules well known to the skilled person. For example, the bases A and G undergo base pairing with the bases T and U or C, resp. and visa versa. Modifications of the bases can influence the base-pairing partner.

**[0148.0.0.0]** The nucleic acid molecule of the invention comprises a nucleotide sequence which is at least about 30%, 35%, 40% or 45%, preferably at least about 50%, 55%, 60% or 65%, more preferably at least about 70%, 80%, or 90%, and even more preferably at least about 95%, 97%, 98%, 99% or more homologous to a nucleotide sequence indicated in Table I, columns 5 or 7, lines 1 to 5 and/or lines 334 to 338, preferably of Table I B, columns 5 or 7, lines 1 to 5 and/or lines 334 to 338, or a functional portion thereof and preferably has above mentioned activity, in particular has the-fine-chemical-increasing activity after increasing its activity or an activity of a product of a gene encoding said sequence or its homologs.

**[0149.0.0.0]** The nucleic acid molecule of the invention or the nucleic acid molecule used in the method of the invention comprises a nucleotide sequence which hybridises, preferably hybridises under stringent conditions as defined herein, to one of the nucleotide sequences indicated in Table I, columns 5 or 7, lines 1 to 5 and/or lines 334 to 338, preferably of Table I B, columns 5 or 7, lines 1 to 5 and/or lines 334 to 338 or a portion thereof and encodes a protein having above-mentioned activity and as indicated in indicated in Table II, columns 5 or 7, lines 1 to 5 and/or lines 334 to 338, preferably of Table II B, columns 5 or 7, lines 1 to 5 and/or lines 334 to 338, e.g. conferring an increase of the respective fine chemical.

**[0149.1.0.0]** Optionally, the nucleotide sequence, which hybridises to one of the nucleotide sequences indicated in Table I, columns 5 or 7, lines 1 to 5 and/or lines 334 to 338, preferably of Table I B, columns 5 or 7, lines 1 to 5 and/or lines 334 to 338 has further one or more of the activities annotated or known for the a protein as indicated in Table II, column 3, lines 1 to 5 and/or lines 334 to 338, preferably of Table II B, columns 3, lines 1 to 5 and/or lines 334 to 338.

**[0150.0.0.0]** Moreover, the nucleic acid molecule of the invention or used in the process of the invention can comprise only a portion of the coding region of one of the sequences indicated in Table I, columns 5 or 7, lines 1 to 5 and/or lines 334 to 338, preferably of Table I B, columns 5 or 7, lines 1 to 5 and/or lines 334 to 338 , for example a fragment which can be used as a probe or primer or a fragment encoding a biologically active portion of the polypeptide of the present invention or of a polypeptide used in the process of the present invention, i.e. having above-mentioned activity, e.g. conferring an increase of methionine if its activity is increased. The nucleotide sequences determined from the cloning of the present protein-according-to-the-invention-encoding gene allows for the generation of probes and primers designed for use in identifying and/or cloning its homologues in other cell types and organisms. The probe/primer typically comprises substantially purified oligonucleotide. The oligonucleotide typically comprises a region of nucleotide sequence that hybridizes under stringent conditions to at least about 12, 15 preferably about 20 or 25, more preferably about 40, 50 or 75 consecutive nucleotides of a sense strand of one of the sequences indicated in Table I, columns 5 or 7, lines 1 to 5 and/or lines 334 to 338, an anti-sense sequence of one of the sequences indicated in Table I, columns 5 or 7, lines 1 to 5 and/or lines 334 to 338, or naturally occurring mutants thereof. Primers based on a nucleotide sequence of the invention can be used in PCR reactions to clone homologues of the polypeptide of the invention or of the polypeptide used in the process of the invention, e.g. as the primers described in the examples of the present invention, e.g. as shown in the examples. A PCR with the primer pairs indicated in Table III, column 7, lines 1 to 5 and/or lines 334 to 338 will result in a fragment of a polynucleotide sequence as indicated in Table I, columns 5 or 7, lines 1 to 5 and/or lines 334 to 338. Preferred is Table II B, columns 7, lines 1 to 5 and/or lines 334 to 338.

**[0151.0.0.0]** Primer sets are interchangeable. The person skilled in the art knows to combine said primers to result in the desired product, e.g. in a full-length clone or a partial sequence. Probes based on the sequences of the nucleic acid molecule of the invention or used in the process of the present invention can be used to detect transcripts or genomic sequences encoding the same or homologous proteins. The probe can further comprise a label group attached thereto, e.g. the label group can be a radioisotope, a fluorescent compound, an enzyme, or an enzyme co-factor. Such probes can be used as a part of a genomic marker test kit for identifying cells which express an polypeptide of the invention or used in the process of the present invention, such as by measuring a level of an encoding nucleic acid molecule in a sample of cells, e.g., detecting mRNA levels or determining, whether a genomic gene comprising the sequence of the polynucleotide of the invention or used in the processes of the present invention has been mutated or deleted.

**[0152.0.0.0]** The nucleic acid molecule of the invention or the nucleic acid molecule used in the method of the invention encodes a polypeptide or portion thereof which includes an amino acid sequence which is sufficiently homologous to an amino acid sequence as indicated in Table II, columns 5 or 7, lines 1 to 5 and/or lines 334 to 338 such that the protein or portion thereof maintains the ability to participate in the respective fine chemical production, in particular a methionine increasing activity as mentioned above or as described in the examples in plants or microorganisms is comprised.

**[0153.0.0.0]** As used herein, the language "sufficiently homologous" refers to proteins or portions thereof which have amino acid sequences which include a minimum number of identical or equivalent amino acid residues (e.g., an amino acid residue which has a similar side chain as an amino acid residue in one of the sequences of the polypeptide of the present invention) to an amino acid sequence as indicated in Table II, columns 5 or 7, lines 1 to 5 and/or lines 334 to 338 such that the protein or portion thereof is able to participate in the increase of the respective fine chemical production. In one embodiment, a protein or portion thereof as indicated in Table II, columns 5 or 7, lines 1 to 5 and/or lines 334 to 338 has for example an activity of a polypeptide indicated in Table II, column 3, lines 1 to 5 and/or lines 334 to 338.

**[0154.0.0.0]** In one embodiment, the nucleic acid molecule of the present invention comprises a nucleic acid that encodes a portion of the protein of the present invention. The protein is at least about 30%, 35%, 40%, 45% or 50%, preferably at least about 55%, 60%, 65% or 70%, and more preferably at least about 75%, 80%, 85%, 90%, 91%, 92%, 93% or 94% and most preferably at least about 95%, 97%, 98%, 99% or more homologous to an entire amino acid sequence as indicated in Table II, columns 5 or 7, lines 1 to 5 and/or lines 334 to 338 and has above-mentioned activity, e.g. conferring preferably the increase of the respective fine chemical.

**[0155.0.0.0]** Portions of proteins encoded by the nucleic acid molecule of the invention or the nucleic acid molecule used in the method of the invention are preferably biologically active, preferably having above-mentioned annotated activity, e.g. conferring a increase the respective fine chemical after increase of activity.

**[0156.0.0.0]** As mentioned herein, the term "biologically active portion" is intended to include a portion, e.g., a domain/motif, that confers increase of the respective fine chemical or has an immunological activity such that it is binds to an antibody binding specifically to the polypeptide of the present invention or a polypeptide used in the process of the present invention for producing the respective fine chemical;

**[0157.0.0.0]** The invention further relates to nucleic acid molecules that differ from one of the nucleotide sequences indicated in Table I, columns 5 or 7, lines 1 to 5 and/or lines 334 to 338 (and portions thereof) due to degeneracy of the genetic code and thus encode a polypeptide of the present invention, in particular a polypeptide having above mentioned activity, e.g. conferring an increase in the respective fine chemical in a organism, e.g. as that polypeptides comprising the consensus sequences as indicated in Table IV, columns 5 or 7, lines 1 to 5 and/or lines 334 to 338 or of the polypeptide as indicated in Table II, columns 5 or 7, lines 1 to 5 and/or lines 334 to 338 or their functional homologues. Advantageously, the nucleic acid molecule of the invention or the nucleic acid molecule used in the method of the invention comprises, or in an other embodiment has, a nucleotide sequence encoding a protein comprising, or in an other embodiment having, a consensus sequences as indicated in Table IV, columns 5 or 7, lines 1 to 5 and/or lines 334 to 338 or of the polypeptide as indicated in Table II, columns 5 or 7, lines 1 to 5 and/or lines 334 to 338 or the functional homologues. In a still further embodiment, the nucleic acid molecule of the invention or the nucleic acid molecule used in the method of the invention encodes a full length protein which is substantially homologous to an amino acid sequence comprising a consensus sequence as indicated in Table IV, columns 5 or 7, lines 1 to 5 and/or lines 334 to 338 or of a polypeptide as indicated in Table II, columns 5 or 7, lines 1 to 5 and/or lines 334 to 338 or the functional homologues thereof. However, in a preferred embodiment, the nucleic acid molecule of the present invention does not consist of a sequence as indicated in Table I, columns 5 or 7, lines 1 to 5 and/or lines 334 to 338, preferably as indicated in Table I A, columns 5 or 7, lines 1 to 5 and/or lines 334 to 338. Preferably the nucleic acid molecule of the invention is a functional homologue or identical to a nucleic acid molecule indicated in Table I B, columns 5 or 7, lines 1 to 5 and/or lines 334 to 338.

**[0158.0.0.0]** In addition, it will be appreciated by those skilled in the art that DNA sequence polymorphisms that lead to changes in the amino acid sequences may exist within a population. Such genetic polymorphism in the gene encoding the polypeptide of the invention or the polypeptide used in the method of the invention or comprising the nucleic acid molecule of the invention or the nucleic acid molecule used in the method of the invention may exist among individuals within a population due to natural variation.

**[0159.0.0.0]** As used herein, the terms "gene" and "recombinant gene" refer to nucleic acid molecules comprising an open reading frame encoding the polypeptide of the invention or the polypeptide used in the method of the invention or comprising the nucleic acid molecule of the invention or the nucleic acid molecule used in the method of the invention or encoding the polypeptide used in the process of the present invention, preferably from a crop plant or from a microorganism useful for the production of respective fine chemicals, in particular for the production of the respective fine chemical. Such natural variations can typically result in 1-5% variance in the nucleotide sequence of the gene. Any and all such nucleotide variations and resulting amino acid polymorphisms in genes encoding a polypeptide of the invention or the polypeptide used in the method of the invention or comprising a the nucleic acid molecule of the invention or the nucleic acid molecule used in the method of the invention that are the result of natural variation and that do not alter the functional activity as described are intended to be within the scope of the invention.

**[0160.0.0.0]** Nucleic acid molecules corresponding to natural variants homologues of a nucleic acid molecule of the invention or the nucleic acid molecule used in the method of the invention, which can also be a cDNA, can be isolated based on their homology to the nucleic acid molecules disclosed herein using the nucleic acid molecule of the invention or the nucleic acid molecule used in the method of the invention, or a portion thereof, as a hybridization probe according to standard hybridization techniques under stringent hybridization conditions.

**[0161.0.0.0]** Accordingly, in another embodiment, a nucleic acid molecule of the invention or the nucleic acid molecule used in the method of the invention is at least 15, 20, 25 or 30 nucleotides in length. Preferably, it hybridizes under stringent conditions to a nucleic acid molecule comprising a nucleotide sequence of the nucleic acid molecule of the present invention or used in the process of the present invention, e.g. comprising a sequence as indicated in Table I, columns 5 or 7, lines 1 to 5 and/or lines 334 to 338. The nucleic acid molecule is preferably at least 20, 30, 50, 100, 250 or more nucleotides in length.

**[0162.0.0.0]** The term "hybridizes under stringent conditions" is defined above. In one embodiment, the term "hybridizes under stringent conditions" is intended to describe conditions for hybridization and washing under which nucleotide sequences at least 30 %, 40 %, 50 % or 65% identical to each other typically remain hybridized to each other. Preferably, the conditions are such that sequences at least about 70%, more preferably at least about 75% or 80%, and even more preferably at least about 85%, 90% or 95% or more identical to each other typically remain hybridized to each other.

**[0163.0.0.0]** Preferably, the nucleic acid molecule of the invention or the nucleic acid molecule used in the method of the invention that hybridizes under stringent conditions to a sequence as indicated in Table I, columns 5 or 7, lines 1 to 5 and/or lines 334 to 338 corresponds to a naturally-occurring nucleic acid molecule of the invention. As used herein, a "naturally-occurring" nucleic acid molecule refers to an RNA or DNA molecule having a nucleotide sequence that occurs in nature (e.g., encodes a natural protein). Preferably, the nucleic acid molecule encodes a natural protein having above-mentioned activity, e.g. conferring the respective fine chemical increase after increasing the expression or activity thereof or the activity of an protein of the invention or used in the process of the invention.

**[0164.0.0.0]** In addition to naturally-occurring variants of the sequences of the polypeptide or nucleic acid molecule of the invention as well as of the polypeptide or nucleic acid molecule used in the process of the invention that may exist in the population, the skilled artisan will further appreciate that changes can be introduced by mutation into a nucleotide sequence of the nucleic acid molecule encoding the polypeptide of the invention or used in the process of the present invention, thereby leading to changes in the amino acid sequence of the encoded said polypeptide, without altering the functional ability of the polypeptide, preferably not decreasing said activity.

**[0165.0.0.0]** For example, nucleotide substitutions leading to amino acid substitutions at "non-essential" amino acid residues can be made in a sequence of the nucleic acid molecule of the invention or used in the process of the invention, e.g. as indicated in Table I, columns 5 or 7, lines 1 to 5 and/or lines 334 to 338.

**[0166.0.0.0]** A "non-essential" amino acid residue is a residue that can be altered from the wild-type sequence of one without altering the activity of said polypeptide, whereas an "essential" amino acid residue is required for an activity as mentioned above, e.g. leading to an increase in the respective fine chemical in an organism after an increase of activity of the polypeptide. Other amino acid residues, however, (e.g., those that are not conserved or only semi-conserved in the domain having said activity) may not be essential for activity and thus are likely to be amenable to alteration without altering said activity.

**[0167.0.0.0]** Further, a person skilled in the art knows that the codon usage between organism can differ. Therefore, he may adapt the codon usage in the nucleic acid molecule of the present invention to the usage of the organism in which the polynucleotide or polypeptide is expressed.

**[0168.0.0.0]** Accordingly, the invention relates to nucleic acid molecules encoding a polypeptide having above-mentioned activity, e.g. conferring an increase in the respective fine chemical in an organisms or parts thereof that contain changes in amino acid residues that are not essential for said activity. Such polypeptides differ in amino acid sequence from a sequence contained in a sequence as indicated in Table II, columns 5 or 7, lines 1 to 5 and/or lines 334 to 338, preferably of Table II B, column 7, lines 1 to 5 and/or lines 334 to 338 yet retain said activity described herein. The nucleic acid molecule can comprise a nucleotide sequence encoding a polypeptide, wherein the polypeptide comprises an amino acid sequence at least about 50% identical to an amino acid sequence as indicated in Table II, columns 5 or 7, lines 1 to 5 and/or lines 334 to 338, preferably of Table II B, column 7, lines 1 to 5 and/or lines 334 to 338 and is capable of participation in the increase of production of the respective fine chemical after increasing its activity, e.g. its expression. Preferably, the protein encoded by the nucleic acid molecule is at least about 60% identical to a sequence as indicated in Table II, columns 5 or 7, lines 1 to 5 and/or lines 334 to 338, preferably of Table II B, column 7, lines 1 to 5 and/or lines 334 to 338, more preferably at least about 70% identical to one of the sequences as indicated in Table II, columns 5 or 7, lines 1 to 5 and/or lines 334 to 338, preferably of Table II B, column 7, lines 1 to 5 and/or lines 334 to 338, even more preferably at least about 80%, 90%, or 95% homologous to a sequence as indicated in Table II, columns 5 or 7, lines 1 to 5 and/or lines 334 to 338, preferably of Table II B, column 7, lines 1 to 5 and/or lines 334 to 338, and most preferably at least about 96%, 97%, 98%, or 99% identical to the sequence as indicated in Table II, columns 5 or 7, lines 1 to 5 and/or lines 334 to 338, preferably of Table 11 B, column 7, lines 1 to 5 and/or lines 334 to 338.

**[0169.0.0.0]** To determine the percentage homology (= identity) of two amino acid sequences or of two nucleic acid molecules, the sequences are written one underneath the other for an optimal comparison (for example gaps may be inserted into the sequence of a protein or of a nucleic acid in order to generate an optimal alignment with the other protein or the other nucleic acid).

**[0170.0.0.0]** The amino acid residues or nucleic acid molecules at the corresponding amino acid positions or nucleotide positions are then compared. If a position in one sequence is occupied by the same amino acid residue or the same nucleic acid molecule as the corresponding position in the other sequence, the molecules are homologous at this position (i.e. amino acid or nucleic acid "homology" as used in the present context corresponds to amino acid or nucleic acid "identity". The percentage homology between the two sequences is a function of the number of identical positions shared by the sequences (i.e. % homology = number of identical positions/total number of positions x 100). The terms "homology" and "identity" are thus to be considered as synonyms.

**[0171.0.0.0]** For the determination of the percentage homology (=identity) of two or more amino acids or of two or more nucleotide sequences several computer software programs have been developed. The homology of two or more sequences can be calculated with for example the software fasta, which presently has been used in the version fasta 3 (W. R. Pearson and D. J. Lipman (1988), Improved Tools for Biological Sequence Comparison. PNAS 85:2444- 2448; W. R. Pearson (1990) Rapid and Sensitive Sequence Comparison with FASTP and FASTA, Methods in Enzymology 183:63 - 98; W. R. Pearson and D. J. Lipman (1988) Improved Tools for Biological Sequence Comparison. PNAS 85:2444- 2448; W. R. Pearson (1990); Rapid and Sensitive Sequence Comparison with FASTP and FASTA Methods in Enzymology 183:63 - 98). Another useful program for the calculation of homologies of different sequences is the standard blast program, which is included in the Biomax pedant software (Biomax, Munich, Federal Republic of Germany). This leads unfortunately sometimes to suboptimal results since blast does not always include complete sequences of the subject and the query. Nevertheless as this program is very efficient it can be used for the comparison of a huge number of sequences. The following settings are typically used for such a comparisons of sequences: -p Program Name [String]; -d Database [String]; default = nr; -i Query File [File In]; default = stdin; -e Expectation value (E) [Real]; default = 10.0; -m alignment view options: 0 = pairwise; 1 = query-anchored showing identities; 2 = query-anchored no identities; 3 = flat query-anchored, show identities; 4 = flat query-anchored, no identities; 5 = query-anchored no identities and blunt ends; 6 = flat query-anchored, no identities and blunt ends; 7 = XML Blast output; 8 = tabular; 9 tabular with comment lines [Integer]; default = 0; -o BLAST report Output File [File Out] Optional; default = stdout; -F Filter query sequence (DUST with blastn, SEG with others) [String]; default = T; -G Cost to open a gap (zero invokes default behavior) [Integer]; default = 0; -E Cost to extend a gap (zero invokes default behavior) [Integer]; default = 0; -X X dropoff value for gapped alignment (in bits) (zero invokes default behavior); blastn 30, megablast 20, tblastx 0, all others 15 [Integer]; default = 0; -I Show GI's in deflines [T/F]; default = F; -q Penalty for a nucleotide mismatch (blastn only) [Integer]; default = -3; -r Reward for a nucleotide match (blastn only) [Integer]; default = 1; -v Number of database sequences to show one-line descriptions for (V) [Integer]; default = 500; -b Number of database sequence to show alignments for (B) [Integer]; default = 250; -f Threshold for extending hits, default if zero; blastp 11, blastn 0, blastx 12, tblastn 13; tblastx 13, megablast 0 [Integer]; default = 0; -g Perfom gapped alignment (not available with tblastx) [T/F]; default = T; -Q Query Genetic code to use [Integer]; default = 1; -D DB Genetic code (for tblast[nx] only) [Integer]; default = 1; -a Number of processors to use [Integer]; default = 1; -O SeqAlign file [File Out] Optional; -J Believe the query defline [T/F]; default = F; -M Matrix [String]; default = BLOSUM62; - W Word size, default if zero (blastn 11, megablast 28, all others 3) [Integer]; default = 0; -z Effective length of the database (use zero for the real size) [Real]; default = 0; -K Number of best hits from a region to keep (off by default, if used a value of 100 is recommended) [Integer]; default = 0; -P 0 for multiple hit, 1 for single hit [Integer]; default = 0; -Y Effective length of the search space (use zero for the real size) [Real]; default = 0; -S Query strands to search against database (for blast[nx], and tblastx); 3 is both, 1 is top, 2 is bottom [Integer]; default = 3; -T Produce HTML output [T/F]; default = F; -I Restrict search of database to list of GI's [String] Optional; -U Use lower case filtering of FASTA sequence [T/F] Optional; default = F; -y X dropoff value for ungapped extensions in bits (0.0 invokes default behavior); blastn 20, megablast 10, all others 7 [Real]; default = 0.0; -Z X dropoff value for final gapped alignment in bits (0.0 invokes default behavior); blastn/megablast 50, tblastx 0, all others 25 [Integer]; default = 0; -R PSI-TBLASTN checkpoint file [File In] Optional; -n MegaBlast search [T/F]; default = F; -L Location on query sequence [String] Optional; -A Multiple Hits window size, default if zero (blastn/megablast 0, all others 40 [Integer]; default = 0; -w Frame shift penalty (OOF algorithm for blastx) [Integer]; default = 0; -t Length of the largest intron allowed in tblastn for linking HSPs (0 disables linking) [Integer]; default = 0.

**[0172.0.0.0]** Results of high quality are reached by using the algorithm of Needleman and Wunsch or Smith and Waterman. Therefore programs based on said algorithms are preferred. Advantageously the comparisons of sequences can be done with the program PileUp (J. Mol. Evolution., 25, 351-360, 1987, Higgins et al., CABIOS, 5 1989: 151-153) or preferably with the programs Gap and BestFit, which are respectively based on the algorithms of Needleman and Wunsch [J. Mol. Biol. 48; 443-453 (1970)] and Smith and Waterman [Adv. Appl. Math. 2; 482-489 (1981)]. Both programs are part of the GCG software-package [Genetics Computer Group, 575 Science Drive, Madison, Wisconsin, USA 53711 (1991); Altschul et al. (1997) Nucleic Acids Res. 25:3389 et seq.]. Therefore preferably the calculations to determine the percentages of sequence homology are done with the program Gap over the whole range of the sequences. The following standard adjustments for the comparison of nucleic acid sequences were used: gap weight: 50, length weight: 3, average match: 10.000, average mismatch: 0.000.

**[0173.0.0.0]** For example a sequence which has a 80% homology with sequence SEQ ID NO: 1 at the nucleic acid level is understood as meaning a sequence which, upon comparison with the sequence SEQ ID NO: 1 by the above Gap program algorithm with the above parameter set, has a 80% homology.

**[0174.0.0.0]** In the state of the art, homology between two polypeptides is also understood as meaning the identity of the amino acid sequence over in each case the entire sequence length which is calculated by comparison with the aid of the program algorithm GAP (Wisconsin Package Version 10.0, University of Wisconsin, Genetics Computer Group (GCG), Madison, USA), setting the following parameters:

| | | | |
|---|---|---|---|
| Gap weight: | 8 | Length weight: | 2 |
| Average match: | 2,912 | Average mismatch: | -2.003 |

**[0175.0.0.0]** For example a sequence which has a 80% homology with sequence SEQ ID NO: 2 at the protein level is understood as meaning a sequence which, upon comparison with the sequence SEQ ID NO: 2 by the above program algorithm with the above parameter set, has a 80% homology.

**[0176.0.0.0]** Functional equivalents derived from one of the polypeptides as indicated in Table II, columns 5 or 7, lines 1 to 5 and/or lines 334 to 338 according to the invention by substitution, insertion or deletion have at least 30%, 35%, 40%, 45% or 50%, preferably at least 55%, 60%, 65% or 70% by preference at least 80%, especially preferably at least 85% or 90%, 91%, 92%, 93% or 94%, very especially preferably at least 95%, 97%, 98% or 99% homology with one of the polypeptides as indicated in Table II, columns 5 or 7, lines 1 to 5 and/or lines 334 to 338 according to the invention and are distinguished by essentially the same properties as a polypeptide as indicated in Table II, columns 5 or 7, lines 1 to 5 and/or lines 334 to 338.

**[0177.0.0.0]** Functional equivalents derived from a nucleic acid sequence as indicated in Table I, columns 5 or 7, lines 1 to 5 and/or lines 334 to 338, preferably of Table I B, column 7, lines 1 to 5 and/or lines 334 to 338 according to the invention by substitution, insertion or deletion have at least 30%, 35%, 40%, 45% or 50%, preferably at least 55%, 60%, 65% or 70% by preference at least 80%, especially preferably at least 85% or 90%, 91%, 92%, 93% or 94%, very especially preferably at least 95%, 97%, 98% or 99% homology with one of a polypeptide as indicated in Table II, columns 5 or 7, lines 1 to 5 and/or lines 334 to 338 according to the invention and encode polypeptides having essentially the same properties as a polypeptide as indicated in Table II, columns 5 or 7, lines 1 to 5 and/or lines 334 to 338, preferably of Table II B, column 7, lines 1 to 5 and/or lines 334 to 338.

**[0178.0.0.0]** "Essentially the same properties" of a functional equivalent is above all understood as meaning that the functional equivalent has above mentioned activity, e.g. conferring an increase in the respective fine chemical amount while increasing the amount of protein, activity or function of said functional equivalent in an organism, e.g. a microorganism, a plant or plant or animal tissue, plant or animal cells or a part of the same.

**[0179.0.0.0]** A nucleic acid molecule encoding a homologous to a protein sequence of as indicated in Table II, columns 5 or 7, lines 1 to 5 and/or lines 334 to 338, preferably of Table II B, column 7, lines 1 to 5 and/or lines 334 to 338 can be created by introducing one or more nucleotide substitutions, additions or deletions into a nucleotide sequence of the nucleic acid molecule of the present invention, in particular as indicated in Table I, columns 5 or 7, lines 1 to 5 and/or lines 334 to 338 such that one or more amino acid substitutions, additions or deletions are introduced into the encoded protein. Mutations can be introduced into the encoding sequences for example into sequences as indicated in Table I, columns 5 or 7, lines 1 to 5 and/or lines 334 to 338 by standard techniques, such as site-directed mutagenesis and PCR-mediated mutagenesis.

**[0180.0.0.0]** Preferably, conservative amino acid substitutions are made at one or more predicted non-essential amino acid residues. A "conservative amino acid substitution" is one in which the amino acid residue is replaced with an amino acid residue having a similar side chain. Families of amino acid residues having similar side chains have been defined in the art. These families include amino acids with basic side chains (e.g., lysine, arginine, histidine), acidic side chains (e.g., aspartic acid, glutamic acid), uncharged polar side chains (e.g., glycine, asparagine, glutamine, serine, threonine, tyrosine, cysteine), nonpolar side chains (e.g., alanine, valine, leucine, isoleucine, proline, phenylalanine, methionine, tryptophan), beta-branched side chains (e.g., threonine, valine, isoleucine) and aromatic side chains (e.g., tyrosine, phenylalanine, tryptophan, histidine).

**[0181.0.0.0]** Thus, a predicted nonessential amino acid residue in a polypeptide of the invention or a polypeptide used in the process of the invention is preferably replaced with another amino acid residue from the same family. Alternatively, in another embodiment, mutations can be introduced randomly along all or part of a coding sequence of a nucleic acid molecule of the invention or used in the process of the invention, such as by saturation mutagenesis, and the resultant mutants can be screened for activity described herein to identify mutants that retain or even have increased above mentioned activity, e.g. conferring an increase in content of the respective fine chemical.

**[0182.0.0.0]** Following mutagenesis of one of the sequences shown herein, the encoded protein can be expressed recombinantly and the activity of the protein can be determined using, for example, assays described herein (see Examples).

**[0183.0.0.0]** The highest homology of the nucleic acid molecule used in the process according to the invention was found for the following database entries by Gap search.

**[0184.0.0.0]** Homologues of the nucleic acid sequences used, with a sequence as indicated in Table I, columns 5 or 7, lines 1 to 5 and/or lines 334 to 338, preferably of Table I B, column 7, lines 1 to 5 and/or lines 334 to 338, or of the nucleic acid sequences derived from a sequences as indicated in Table II, columns 5 or 7, lines 1 to 5 and/or lines 334 to 338, preferably of Table II B, column 7, lines 1 to 5 and/or lines 334 to 338, comprise also allelic variants with at least approximately 30%, 35%, 40% or 45% homology, by preference at least approximately 50%, 60% or 70%, more preferably at least approximately 90%, 91%, 92%, 93%, 94% or 95% and even more preferably at least approximately 96%, 97%, 98%, 99% or more homology with one of the nucleotide sequences shown or the abovementioned derived nucleic acid sequences or their homologues, derivatives or analogues or parts of these. Allelic variants encompass in particular functional variants which can be obtained by deletion, insertion or substitution of nucleotides from the sequences shown, preferably from a sequence as indicated in Table I, columns 5 or 7, lines 1 to 5 and/or lines 334 to 338, or from the derived nucleic acid sequences, the intention being, however, that the enzyme activity or the biological activity of the resulting proteins synthesized is advantageously retained or increased.

**[0185.0.0.0]** In one embodiment of the present invention, the nucleic acid molecule of the invention or used in the process of the invention comprises one or more sequences as indicated in Table I, columns 5 or 7, lines 1 to 5 and/or lines 334 to 338, preferably of Table I B, column 7, lines 1 to 5 and/or lines 334 to 338. In one embodiment, it is preferred that the nucleic acid molecule comprises as little as possible other nucleotide sequences not shown in any one of sequences as indicated in Table I, columns 5 or 7, lines 1 to 5 and/or lines 334 to 338, preferably of Table I B, column 7, lines 1 to 5 and/or lines 334 to 338. In one embodiment, the nucleic acid molecule comprises less than 500, 400, 300, 200, 100, 90, 80, 70, 60, 50 or 40 further nucleotides. In a further embodiment, the nucleic acid molecule comprises less than 30, 20 or 10 further nucleotides. In one embodiment, a nucleic acid molecule used in the process of the invention is identical to a sequences as indicated in Table I, columns 5 or 7, lines 1 to 5 and/or lines 334 to 338, preferably of Table I B, column 7, lines 1 to 5 and/or lines 334 to 338.

**[0186.0.0.0]** Also preferred is that one or more nucleic acid molecule(s) used in the process of the invention encode a polypeptide comprising a sequence as indicated in Table II, columns 5 or 7, lines 1 to 5 and/or lines 334 to 338, preferably of Table II B, column 7, lines 1 to 5 and/or lines 334 to 338. In one embodiment, the nucleic acid molecule encodes less than 150, 130, 100, 80, 60, 50, 40 or 30 further amino acids. In a further embodiment, the encoded polypeptide comprises less than 20, 15, 10, 9, 8, 7, 6 or 5 further amino acids. In one embodiment, the encoded polypeptide used in the process of the invention is identical to the sequences as indicated in Table II, columns 5 or 7, lines 1 to 5 and/or lines 334 to 338, preferably of Table II B, column 7, lines 1 to 5 and/or lines 334 to 338.

**[0187.0.0.0]** In one embodiment, the nucleic acid molecule of the invention or used in the process encodes a polypeptide comprising a sequence as indicated in Table II, columns 5 or 7, lines 1 to 5 and/or lines 334 to 338, preferably of Table II B, column 7, lines 1 to 5 and/or lines 334 to 338 comprises less than 100 further nucleotides. In a further embodiment, said nucleic acid molecule comprises less than 30 further nucleotides. In one embodiment, the nucleic acid molecule used in the process is identical to a coding sequence encoding a sequences as indicated in Table II, columns 5 or 7, lines 1 to 5 and/or lines 334 to 338, preferably of Table II B, column 7, lines 1 to 5 and/or lines 334 to 338.

**[0188.0.0.0]** Polypeptides (= proteins), which still have the essential enzymatic activity of the polypeptide of the present invention conferring an increase of the respective fine chemical i.e. whose activity is essentially not reduced, are polypeptides with at least 10% or 20%, by preference 30% or 40%, especially preferably 50% or 60%, very especially preferably 80% or 90 or more of the wild type biological activity or enzyme activity, advantageously, the activity is essentially not reduced in comparison with the activity of a polypeptide as indicated in Table II, columns 5 or 7, lines 1 to 5 and/or lines 334 to 338, preferably compared to a sequence as indicated in Table II, column 3 and 5, lines 1 to 5 and/or lines 334 to 338, and expressed under identical conditions.
In one embodiment, the polypeptide of the invention is a homolog consisting of or comprising the sequence as indicated in Table II B, columns 7, lines 1 to 5 and/or lines .334 to 338.

**[0189.0.0.0]** Homologues of a sequence as indicated in Table I, columns 5 or 7, lines 1 to 5 and/or lines 334 to 338 or of a derived sequence as indicated in Table II, columns 5 or 7, lines 1 to 5 and/or lines 334 to 338 also mean truncated sequences, cDNA, single-stranded DNA or RNA of the coding and noncoding DNA sequence. Homologues of said sequences are also understood as meaning derivatives, which comprise noncoding regions such as, for example, UTRs, terminators, enhancers or promoter variants. The promoters upstream of the nucleotide sequences stated can be modified by one or more nucleotide substitution(s), insertion(s) and/or deletion(s) without, however, interfering with the functionality or activity either of the promoters, the open reading frame (= ORF) or with the 3'-regulatory region such as terminators or other 3'regulatory regions, which are far away from the ORF. It is furthermore possible that the activity of the promoters is increased by modification of their sequence, or that they are replaced completely by more active promoters, even promoters from heterologous organisms. Appropriate promoters are known to the person skilled in the art and are mentioned herein below.

**[0190.0.0.0]** In a further embodiment, the process according to the present invention comprises the following steps:
(a) selecting an organism or a part thereof expressing the polypeptide of this invention;
(b) mutagenizing the selected organism or the part thereof;
(c) comparing the activity or the expression level of said polypeptide in the mutagenized organism or the part thereof with the activity or the expression of said polypeptide in the selected organisms or the part thereof;
(d) selecting the mutagenized organisms or parts thereof, which comprise an increased activity or expression level of said polypeptide compared to the selected organism (a) or the part thereof;
(e) optionally, growing and cultivating the organisms or the parts thereof; and
(f) recovering, and optionally isolating, the free or bound respective fine chemical produced by the selected mutated organisms or parts thereof.

**[0191.0.0.0]** The organisms or part thereof produce according to the herein mentioned process of the invention an increased level of free and/or -bound respective fine chemical compared to said control or selected organisms or parts thereof.

**[0191.1.0.0]** In one embodiment, the organisms or part thereof produce according to the herein mentioned process of the invention an increased level of protein-bound respective fine chemical compared to said control or selected organisms or parts thereof.

**[0192.0.0.0]** Advantageously the selected organisms are mutagenized according to the invention. According to the invention mutagenesis is any change of the genetic information in the genome of an organism, that means any structural or compositional change in the nucleic acid preferably DNA of an organism that is not caused by normal segregation or genetic recombination processes. Such mutations may occur spontaneously, or may be induced by mutagens as described below. Such change can be induced either randomly or selectively. In both cases the genetic information of the organism is modified. In general this lead to the situation that the activity of the gene product of the relevant genes inside the cells or inside the organism is increased.

**[0193.0.0.0]** In case of the specific or so called site directed mutagenesis a distinct gene is mutated and thereby its activity and/or the activity or the encoded gene product is repressed, reduced or increased, preferably increased. In the event of a random mutagenesis one or more genes are mutated by chance and their activities and/or the activities of their gene products are repressed, reduced or increased, preferably increased.

**[0194.0.0.0]** For the purpose of a mutagenesis of a huge population of organisms, such population can be transformed with a DNA construct, which is useful for the activation of as much as possible genes of an organism, preferably all genes. For example the construct can contain a strong promoter or one or more enhancers, which are capable of transcriptionally activate genes in the vicinity of their integration side. With this method it is possible to statistically mutagenize, e.g. activate nearly all genes of an organism by the random integration of an activation construct. Afterwards the skilled worker can identify those mutagenized lines in which a gene of the invention has been activated, which in turns leads to the desired increase in the respective fine chemical production .

**[0195.0.0.0]** The genes of the invention can also be activated by mutagenesis, either of regulatory or coding regions. In the event of a random mutagenesis a huge number of organisms are treated with a mutagenic agent. The amount of said agent and the intensity of the treatment will be chosen in such a manner that statistically nearly every gene is mutated once. The process for the random mutagenesis as well as the respective agens is well known by the skilled person. Such methods are disclosed for example by A.M. van Harten [(1998), "Mutation breeding: theory and practical applications", Cambridge University Press, Cambridge, UK], E Friedberg, G Walker, W Siede [(1995), "DNA Repair and Mutagenesis", Blackwell Publishing], or K. Sankaranarayanan, J. M. Gentile, L. R. Ferguson [(2000) "Protocols in Mutagenesis", Elsevier Health Sciences]. As the skilled worker knows the spontaneous mutation rate in the cells of an organism is very low and that a large number of chemical, physical or biological agents are available for the mutagenesis of organisms. These agents are named as mutagens or mutagenic agents. As mentioned before three different kinds of mutagens (chemical, physical or biological agents) are available.

**[0196.0.0.0]** There are different classes of chemical mutagens, which can be separated by their mode of action. For example base analogues such as 5-bromouracil, 2-amino purin. Other chemical mutagens are interacting with the DNA such as sulphuric acid, nitrous acid, hydroxylamine; or other alkylating agents such as monofunctional agents like ethyl methanesulfonate, dimethylsulfate, methyl methanesulfonate), bifunctional like dichloroethyl sulphide, Mitomycin, Nitrosoguanidine - dialkylnitrosamine, N-Nitrosoguanidin derivatives, N-alkyl-N-nitro-N-nitroso-guanidine-), ntercalating dyes like Acridine, ethidium bromide).

**[0197.0.0.0]** Physical mutagens are for example ionizing irradiation (X ray), UV irradiation. Different forms of irradiation are available and they are strong mutagens. Two main classes of irradiation can be distinguished: a) non-ionizing irradiation such as UV light or ionizing irradiation such as X ray. Biological mutagens are for example transposable elements for example IS elements such as IS100, transposons such as Tn5, Tn10, Tn916 or Tn1000 or phages like Mu^{amplac}, P1, T5, λplac etc. Methods for introducing this phage DNA into the appropriate microorganism are well known to the skilled worker (see Microbiology, Third Edition, Eds. Davis, B.D., Dulbecco, R., Eisen, H.N. and Ginsberg, H.S., Harper International Edition, 1980). The common procedure of a transposon mutagenesis is the insertion of a transposable element within a gene or nearby for example in the promotor or terminator region and thereby leading to a loss of the gene function. Procedures to localize the transposon within the genome of the organisms are well known by a person skilled in the art.

**[0198.0.0.0]** Preferably a chemical or biochemical procedure is used for the mutagenesis of the organisms. A preferred chemical method is the mutagenesis with N-methyl-N-nitro-nitrosoguanidine.

**[0199.0.0.0]** Other biological method are disclosed by Spee et al. (Nucleic Acids Research, Vol. 21, No. 3, 1993: 777 - 778). Spee et al. teaches a PCR method using dITP for the random mutagenesis. This method described by Spee et al. was further improved by Rellos et al. (Protein Expr. Purif., 5, 1994 : 270 - 277). The use of an in vitro recombination technique for molecular mutagenesis is described by Stemmer (Proc. Natl. Acad. Sci. USA, Vol. 91, 1994: 10747 - 10751). Moore et al. (Nature Biotechnology Vol. 14, 1996: 458 - 467) describe the combination of the PCR and recombination methods for increasing the enzymatic activity of an esterase toward a para-nitrobenzyl ester. Another route to the mutagenesis of enzymes is described by Greener et al. in Methods in Molecular Biology (Vol. 57, 1996: 375 - 385). Greener et al. use the specific Escherichia coli strain XL1-Red to generate Escherichia coli mutants which have increased antibiotic resistance.

**[0200.0.0.0]** In one embodiment, the protein according to the invention or the nucleic acid molecule characterized herein originates from a eukaryotic or prokaryotic organism such as a non-human animal, a plant, a microorganism such as a fungi, a yeast, an alga, a diatom or a bacterium. Nucleic acid molecules, which advantageously can be used in the process of the invention originate from yeasts, for example the family Saccharomycetaceae, in particular the genus Saccharomyces, or yeast genera such as Candida, Hansenula, Pichia, Yarrowia, Rhodotorula or Schizosaccharomyces and the especially advantageous from the species Saccharomyces cerevisiae.

**[0201.0.0.0]** In one embodiment, nucleic acid molecules, which advantageously can be used in the process of the invention originate from bacteria, for example from Proteobacteria, in particular from Gammaproteobacteria, more preferred from Enterobacteriales, e.g. from the family Enterobacteriaceae, particularly from genera Escherichia, Salmonella, Klebsiella, advantageously form the species Escherichia coli K12.

**[0202.0.0.0]** If, in the process according to the invention, plants are selected as the donor organism, this plant may, in principle, be in any phylogenetic relation of the recipient plant. Donor and recipient plant may belong to the same family, genus, species, variety or line, resulting in an increasing homology between the nucleic acids to be integrated and corresponding parts of the genome of the recipient plant. This also applies analogously to microorganisms as donor and recipient organism.
It might also be advantageously to use nuclei acids molecules from very distinct species, since these might exhibit reduced sensitivity against endogenous regulatory mechanisms and such sequences might not be recognized by endogenous silencing mechanisms.

**[0203.0.0.0]** Accordingly, one embodiment of the application relates to the use of nucleic acid molecules in the process of the invention from plants, e.g. crop plants, e.g. from: B. napus; Glycine max; sunflower linseed or maize or their homologues.

**[0204.0.0.0]** Accordingly, in one embodiment, the invention relates to a nucleic acid molecule which comprises a nucleic acid molecule selected from the group consisting of:
a) nucleic acid molecule encoding, preferably at least the mature form, of a polypeptide as indicated in Table II, columns 5 or 7, lines 1 to 5 and/or lines 334 to 338, preferably of Table II B, column 7, lines 1 to 5 and/or lines 334 to 338; or a fragment thereof conferring an increase in the amount of the respective fine chemical in an organism or a part thereof
b) nucleic acid molecule comprising, preferably at least the mature form, of a nucleic acid molecule as indicated in Table I, columns 5 or 7, lines 1 to 5 and/or lines 334 to 338, preferably of Table I B, column 7, lines 1 to 5 and/or lines 334 to 338 or a fragment thereof conferring an increase in the amount of the respective fine chemical in an organism or a part thereof;
c) nucleic acid molecule whose sequence can be deduced from a polypeptide sequence encoded by a nucleic acid molecule of (a) or (b) as result of the degeneracy of the genetic code and conferring an increase in the amount of the respective fine chemical in an organism or a part thereof;
d) nucleic acid molecule encoding a polypeptide whose sequence has at least 50% identity with the amino acid sequence of the polypeptide encoded by the nucleic acid molecule of (a) to (c) and conferring an increase in the amount of the respective fine chemical in an organism or a part thereof;
e) nucleic acid molecule which hybridizes with a nucleic acid molecule of (a) to (c) under stringent hybridisation conditions and conferring an increase in the amount of the respective fine chemical in an organism or a part thereof;
f) nucleic acid molecule encoding a polypeptide, the polypeptide being derived by substituting, deleting and/or adding one or more amino acids of the amino acid sequence of the polypeptide encoded by the nucleic acid molecules (a) to (d), preferably to (a) to (c), and conferring an increase in the amount of the respective fine chemical in an organism or a part thereof;
g) nucleic acid molecule encoding a fragment or an epitope of a polypeptide which is encoded by one of the nucleic acid molecules of (a) to (e), preferably to (a) to (c) and conferring an increase in the amount of the respective fine chemical in an organism or a part thereof ;
h) nucleic acid molecule comprising a nucleic acid molecule which is obtained by amplifying a cDNA library or a genomic library using primers or primer pairs as indicated in Table III, column 7, lines 1 to 5 and/or lines 334 to 338 and conferring an increase in the amount of the respective fine chemical in an organism or a part thereof;
i) nucleic acid molecule encoding a polypeptide which is isolated, e.g. from a expression library, with the aid of monoclonal antibodies against a polypeptide encoded by one of the nucleic acid molecules of (a) to (g), preferably to (a) to (c) and conferring an increase in the amount of the respective fine chemical in an organism or a part thereof;
j) nucleic acid molecule which encodes a polypeptide comprising a consensus sequence as indicated in Table IV, columns 7, lines 1 to 5 and/or lines 334 to 338 and conferring an increase in the amount of the respective fine chemical in an organism or a part thereof;
k) nucleic acid molecule encoding the amino acid sequence of a polypeptide encoding a domain of a polypeptide as indicated in Table II, columns 5 or 7, lines 1 to 5 and/or lines 334 to 338, preferably of Table II B, column 7, lines 1 to 5 and/or lines 334 to 338 and conferring an increase in the amount of the respective fine chemical in an organism or a part thereof; and
l) nucleic acid molecule which is obtainable by screening a suitable nucleic acid library under stringent hybridization conditions with a probe comprising one of the sequences of the nucleic acid molecule of (a) to (k) or with a fragment of at least 15 nt, preferably 20 nt, 30 nt, 50 nt, 100 nt, 200 nt or 500 nt of the nucleic acid molecule characterized in (a) to (h) or of a nucleic acid molecule as indicated in Table I, columns 5 or 7, lines 1 to 5 and/or lines 334 to 338, preferably of Table I B, column 7, lines 1 to 5 and/or lines 334 to 338 or a nucleic acid molecule encoding, preferably at least the mature form of, the polypeptide as indicated in Table II, columns 5 or 7, lines 1 to 5 and/or lines 334 to 338, preferably of Table II B, column 7, lines 1 to 5 and/or lines 334 to 338, and conferring an increase in the amount of the respective fine chemical in an organism or a part thereof;
or which encompasses a sequence which is complementary thereto; whereby, preferably, the nucleic acid molecule according to (a) to (I) distinguishes over the sequence indicated in Table IA or I B, columns 5 or 7, lines 1 to 5 and/or lines 334 to 338, by one or more nucleotides. In one embodiment, the nucleic acid molecule does not consist of the sequence shown and indicated in Table I A or I B, columns 5 or 7, lines 1 to 5 and/or lines 334 to 338: In one embodiment, the nucleic acid molecule is less than 100%, 99,999%, 99,99%, 99,9% or 99% identical to a sequence indicated in Table I A or I B, columns 5 or 7, lines 1 to 5 and/or lines 334 to 338. In another embodiment, the nucleic acid molecule does not encode a polypeptide of a sequence indicated in Table II A or II B, columns 5 or 7, lines 1 to 5 and/or lines 334 to 338. In an other embodiment, the nucleic acid molecule of the present invention is at least 30 %, 40 %, 50%, or 60% identical and less than 100%, 99,999%, 99,99%, 99,9% or 99% identical to a sequence indicated in Table I A or I B, columns 5 or 7, lines 1 to 5 and/or lines 334 to 338. In a further embodiment the nucleic acid molecule does not encode a polypeptide sequence as indicated in Table II A or II B, columns 5 or 7, lines 1 to 5 and/or lines 334 to 338. Accordingly, in one embodiment, the nucleic acid molecule of the differs at least in one or more residues from a nucleic acid molecule indicated in Table I A or I B, columns 5 or 7, lines 1 to 5 and/or lines 334 to 338. Accordingly, in one embodiment, the nucleic acid molecule of the present invention encodes a polypeptide, which differs at least in one or more amino acids from a polypeptide indicated in Table II A or I B, columns 5 or 7, lines 1 to 5 and/or lines 334 to 338. In another embodiment, a nucleic acid molecule indicated in Table I A or I B, columns 5 or 7, lines 1 to 5 and/or lines 334 to 338 does not encode a protein of a sequence indicated in Table II A or II B, columns 5 or 7, lines 1 to 5 and/or lines 334 to 338. Accordingly, in one embodiment, the protein encoded by a sequences of a nucleic acid according to (a) to (I) does not consist of a sequence as indicated in Table II A or II B, columns 5 or 7, lines 1 to 5 and/or lines 334 to 338. In a further embodiment, the protein of the present invention is at least 30 %, 40 %, 50%, or 60% identical to a protein sequence indicated in Table II A or II B, columns 5 or 7, lines 1 to 5 and/or lines 334 to 338 and less than 100%, preferably less than 99,999%, 99,99% or 99,9%, more preferably less than 99%, 98%, 97%, 96% or 95% identical to a sequence as indicated in Table I A or II B, columns 5 or 7, lines 1 to 5 and/or lines 334 to 338.

**[0205.0.0.0]** The nucleic acid sequences used in the process are advantageously introduced in a nucleic acid construct, preferably an expression cassette which makes possible the expression of the nucleic acid molecules in an organism, advantageously a plant or a microorganism.

**[0206.0.0.0]** Accordingly, the invention also relates to an nucleic acid construct, preferably to an expression construct, comprising the nucleic acid molecule of the present invention functionally linked to one or more regulatory elements or signals.

**[0207.0.0.0]** As described herein, the nucleic acid construct can also comprise further genes, which are to be introduced into the organisms or cells. It is possible and advantageous to introduce into, and express in, the host organisms regulatory genes such as genes for inductors, repressors or enzymes, which, owing to their enzymatic activity, engage in the regulation of one or more genes of a biosynthetic pathway. These genes can be of heterologous or homologous origin. Moreover, further biosynthesis genes may advantageously be present, or else these genes may be located on one or more further nucleic acid constructs. Genes, which are advantageously employed as biosynthesis genes are genes of the amino acid metabolism, of glycolysis, of the tricarboxylic acid metabolism or their combinations. As described herein, regulator sequences or factors can have a positive effect on preferably the gene expression of the genes introduced, thus increasing it. Thus, an enhancement of the regulator elements may advantageously take place at the transcriptional level by using strong transcription signals such as promoters and/or enhancers. In addition, however, an enhancement of translation is also possible, for example by increasing mRNA stability or by inserting a translation enhancer sequence.

**[0208.0.0.0]** In principle, the nucleic acid construct can comprise the herein described regulator sequences and further sequences relevant for the expression of the comprised genes. Thus, the nucleic acid construct of the invention can be used as expression cassette and thus can be used directly for introduction into the plant, or else they may be introduced into a vector. Accordingly in one embodiment the nucleic acid construct is an expression cassette comprising a microorganism promoter or a microorganism terminator or both. In another embodiment the expression cassette encompasses a plant promoter or a plant terminator or both.

**[0209.0.0.0]** Accordingly, in one embodiment, the process according to the invention comprises the following steps:
(a) introducing of a nucleic acid construct comprising the nucleic acid molecule of the invention or used in the process of the invention or encoding the polypeptide of the present invention or used in the process of the invention; or
(b) introducing of a nucleic acid molecule, including regulatory sequences or factors, which expression increases the expression of the nucleic acid molecule of the invention or used in the process of the invention or encoding the polypeptide of the present invention or used in the process of the invention;
   in a cell, or an organism or a part thereof, preferably in a plant, plant cell or a microorganism, and
(c) expressing of the gene product encoded by the nucleic acid construct or the nucleic acid molecule mentioned under (a) or (b) in the cell or the organism.

**[0210.0.0.0]** After the introduction and expression of the nucleic acid construct the transgenic organism or cell is advantageously cultured and subsequently harvested. The transgenic organism or cell may be a prokaryotic or eukaryotic organism such as a microorganism, a non-human animal and plant for example a plant or animal cell, a plant or animal tissue, preferably a crop plant, or a part thereof.

**[0211.0.0.0]** To introduce a nucleic acid molecule into a nucleic acid construct, e.g. as part of an expression cassette, the codogenic gene segment is advantageously subjected to an amplification and ligation reaction in the manner known by a skilled person. It is preferred to follow a procedure similar to the protocol for the Pfu DNA polymerase or a Pfu/Taq DNA polymerase mixture. The primers are selected according to the sequence to be amplified. The primers should expediently be chosen in such a way that the amplificate comprise the codogenic sequence from the start to the stop codon. After the amplification, the amplificate is expediently analyzed. For example, the analysis may consider quality and quantity and be carried out following separation by gel electrophoresis. Thereafter, the amplificate can be purified following a standard protocol (for example Qiagen). An aliquot of the purified amplificate is then available for the subsequent cloning step. Suitable cloning vectors are generally known to the skilled worker.

**[0212.0.0.0]** They include, in particular, vectors which are capable of replication in easy to handle cloning systems like as bacterial yeast or insect cell based (e.g. baculovirus expression) systems, that is to say especially vectors which ensure efficient cloning in E. coli, and which make possible the stable transformation of plants. Vectors, which must be mentioned in particular are various binary and cointegrated vector systems which are suitable for the T-DNA-mediated transformation. Such vector systems are generally characterized in that they contain at least the vir genes, which are required for the Agrobacterium-mediated transformation, and the T-DNA border sequences.

**[0213.0.0.0]** In general, vector systems preferably also comprise further cis-regulatory regions such as promoters and terminators and/or selection markers by means of which suitably transformed organisms can be identified. While vir genes and T-DNA sequences are located on the same vector in the case of cointegrated vector systems, binary systems are based on at least two vectors, one of which bears vir genes, but no T-DNA, while a second one bears T-DNA, but no vir gene. Owing to this fact, the last-mentioned vectors are relatively small, easy to manipulate and capable of replication in E. coli and in Agrobacterium. These binary vectors include vectors from the series pBIB-HYG, pPZP, pBecks, pGreen. Those which are preferably used in accordance with the invention are Bin19, pBI101, pBinAR, pGPTV and pCAMBIA. An overview of binary vectors and their use is given by Hellens et al, Trends in Plant Science (2000) 5, 446-451.

**[0214.0.0.0]** For a vector preparation, vectors may first be linearized using restriction endonuclease(s) and then be modified enzymatically in a suitable manner. Thereafter, the vector is purified, and an aliquot is employed in the cloning step. In the cloning step, the enzyme-cleaved and, if required, purified amplificate is cloned together with similarly prepared vector fragments, using ligase. In this context, a specific nucleic acid construct, or vector or plasmid construct, may have one or else more codogenic gene segments. The codogenic gene segments in these constructs are preferably linked operably to regulatory sequences. The regulatory sequences include, in particular, plant sequences like the above-described promoters and terminators. The constructs can advantageously be propagated stably in microorganisms, in particular Escherichia coli and/or Agrobacterium tumefaciens, under selective conditions and enable the transfer of heterologous DNA into plants or other microorganisms. In accordance with a particular embodiment, the constructs are based on binary vectors (overview of a binary vector: Hellens et al., 2000). As a rule, they contain prokaryotic regulatory sequences, such as replication origin and selection markers, for the multiplication in microorganisms such as Escherichia coli and Agrobacterium tumefaciens. Vectors can further contain agrobacterial T-DNA sequences for the transfer of DNA into plant genomes or other eukaryotic regulatory sequences for transfer into other eukaryotic cells, e.g. Saccharomyces sp. or other prokaryotic regulatory sequences for the transfer into other prokaryotic cells, e.g. Corynebacterium sp. or Bacillus sp. For the transformation of plants, the right border sequence, which comprises approximately 25 base pairs, of the total agrobacterial T-DNA sequence is advantageously included. Usually, the plant transformation vector constructs according to the invention contain T-DNA sequences both from the right and from the left border region, which contain expedient recognition sites for site-specific acting enzymes which, in turn, are encoded by some of the vir genes.

**[0215.0.0.0]** Suitable host organisms are known to the skilled worker. Advantageous organisms are described further above in the present application. They include in particular eukaryotes or eubacteria, e.g. prokaryotes or archae bacteria. Advantageously host organisms are microorganisms selected from the group consisting of Actinomycetaceae, Bacillaceae, Brevibacteriaceae, Corynebacteriaceae, Enterobacteriacae, Gordoniaceae, Micrococcaceae, Mycobacteriaceae, Nocardiaceae, Pseudomonaceae, Rhizobiaceae, Streptomycetaceae, Chaetomiaceae, Choanephoraceae, Cryptococcaceae, Cunninghamellaceae, Demetiaceae, Moniliaceae, Mortierellaceae, Mucoraceae, Pythiaceae, Sacharomycetaceae, Saprolegniaceae, Schizosacharomycetaceae, Sodariaceae, Sporobolomycetaceae, Tuberculariaceae, Adelotheciaceae, Dinophyceae, Ditrichaceae and Prasinophyceae. Preferably are unicellular, microorganisms, e.g. fungi, bacteria or protoza, such as fungi like the genus Claviceps or Aspergillus or gram-positive bacteria such as the genera Bacillus, Corynebacterium, Micrococcus, Brevibacterium, Rhodococcus, Nocardia, Caseobacter or Arthrobacter or gram-negative bacteria such as the genera Escherichia, Flavobacterium or Salmonella, or yeasts such as the genera Rhodotorula, Hansenula, Pichia, Yerrowia, Saccharomyces, Schizosaccharomyces or Candida.

**[0216.0.0.0]** Host organisms which are especially advantageously selected in the process according to the invention are microorganisms selected from the group of the genera and species consisting of Hansenula anomala, Candida utilis, Claviceps purpurea, Bacillus circulans, Bacillus subtilis, Bacillus sp., Brevibacterium albidum, Brevibacterium album, Brevibacterium cerinum, Brevibacterium flavum, Brevibacterium glutamigenes, Brevibacterium iodinum, Brevibacterium ketoglutamicum, Brevibacterium lactofermentum, Brevibacterium linens, Brevibacterium roseum, Brevibacterium saccharolyticum, Brevibacterium sp., Corynebacterium acetoacidophilum, Corynebacterium acetoglutamicum, Corynebacterium ammoniagenes, Corynebacterium glutamicum (= Micrococcus glutamicum), Corynebacterium melassecola, Corynebacterium sp. or Escherichia coli, specifically Escherichia coli K12 and its described strains.

**[0217.0.0.0]** Advantageously preferred in accordance with the invention are host organisms of the genus Agrobacterium tumefaciens or plants. Preferred plants are selected from among the families Aceraceae, Anacardiaceae, Apiaceae, Asteraceae, Apiaceae, Betulaceae, Boraginaceae, Brassicaceae, Bromeliaceae, Cactaceae, Caricaceae, Caryophyllaceae, Cannabaceae, Convolvulaceae, Chenopodiaceae, Elaeagnaceae, Geraniaceae, Gramineae, Juglandaceae, Lauraceae, Leguminosae, Linaceae, Cucurbitaceae, Cyperaceae, Euphorbiaceae, Fabaceae, Malvaceae, Nymphaeaceae, Papaveraceae, Rosaceae, Salicaceae, Solanaceae, Arecaceae, Iridaceae, Liliaceae, Orchidaceae, Gentianaceae, Labiaceae, Magnoliaceae, Ranunculaceae, Carifolaceae, Rubiaceae, Scrophulariaceae, Ericaceae, Polygonaceae, Violaceae, Juncaceae, Poaceae, perennial grass, fodder crops, vegetables and ornamentals.

**[0218.0.0.0]** Especially preferred are plants selected from the groups of the families Apiaceae, Asteraceae, Brassicaceae, Cucurbitaceae, Fabaceae, Papaveraceae, Rosaceae, Solanaceae, Liliaceae or Poaceae. Especially advantageous are, in particular, crop plants. Accordingly, an advantageous plant preferably belongs to the group of the genus peanut, oilseed rape, canola, sunflower, safflower, olive, sesame, hazelnut, almond, avocado, bay, pumpkin/squash, linseed, soya, pistachio, borage, maize, wheat, rye, oats, sorghum and millet, triticale, rice, barley, cassava, potato, sugarbeet, fodder beet, egg plant, and perennial grasses and forage plants, oil palm, vegetables (brassicas, root vegetables, tuber vegetables, pod vegetables, fruiting vegetables, onion vegetables, leafy vegetables and stem vegetables), buckwheat, Jerusalem artichoke, broad bean, vetches, lentil, alfalfa, dwarf bean, lupin, clover and lucerne.

**[0219.0.0.0]** In order to introduce, into a plant, the nucleic acid molecule of the invention or used in the process according to the invention, it has proved advantageous first to transfer them into an intermediate host, for example a bacterium or a eukaryotic unicellular cell. The transformation into E. coli, which can be carried out in a manner known per se, for example by means of heat shock or electroporation, has proved itself expedient in this context. Thus, the transformed E. coli colonies can be analysed for their cloning efficiency. This can be carried out with the aid of a PCR. Here, not only the identity, but also the integrity, of the plasmid construct can be verified with the aid of a defined colony number by subjecting an aliquot of the colonies to said PCR. As a rule, universal primers which are derived from vector sequences are used for this purpose, it being possible, for example, for a forward primer to be arranged upstream of the start ATG and a reverse primer to be arranged downstream of the stop codon of the codogenic gene segment. The amplificates are separated by electrophoresis and assessed with regard to quantity and quality.

**[0220.0.0.0]** The nucleic acid constructs, which are optionally verified, are subsequently used for the transformation of the plants or other hosts, e.g. other eukaryotic cells or other prokaryotic cells. To this end, it may first be necessary to obtain the constructs from the intermediate host. For example, the constructs may be obtained as plasmids from bacterial hosts by a method similar to conventional plasmid isolation.

**[0221.0.0.0]** The nucleic acid molecule of the invention or used in the process according to the invention can also be introduced into modified viral vectors like baculovirus vectors for expression in insect cells or plant viral vectors like tobacco mosaic virus or potato virus X-based vectors. Approaches leading to the expression of proteins from the modified viral genome including the the nucleic acid molecule of the invention or used in the process according to the invention involve for example the inoculation of tobacco plants with infectious RNA transcribed in vitro from a cDNA copy of the recombinant viral genome. Another approach utilizes the transfection of whole plants from wounds inoculated with Agrobacterium tumefaciens containing cDNA copies of recombinant plus-sense RNA viruses. Different vectors and virus are known to the skilled worker for expression in different target eg. production plants.

**[0222.0.0.0]** A large number of methods for the transformation of plants are known. Since, in accordance with the invention, a stable integration of heterologous DNA into the genome of plants is advantageous, the T-DNA-mediated transformation has proved expedient in particular. For this purpose, it is first necessary to transform suitable vehicles, in particular agrobacteria, with a codogenic gene segment or the corresponding plasmid construct comprising the nucleic acid molecule of the invention. This can be carried out in a manner known per se. For example, said nucleic acid construct of the invention, or said expression construct or said plasmid construct, which has been generated in accordance with what has been detailed above, can be transformed into competent agrobacteria by means of electroporation or heat shock. In principle, one must differentiate between the formation of cointegrated vectors on the one hand and the transformation with binary vectors on the other hand. In the case of the first alternative, the constructs, which comprise the codogenic gene segment or the nucleic acid molecule of the invention or the nucleic acid molecule used in the method of the invention have no T-DNA sequences, but the formation of the cointegrated vectors or constructs takes place in the agrobacteria by homologous recombination of the construct with T-DNA. The T-DNA is present in the agrobacteria in the form of Ti or Ri plasmids in which exogenous DNA has expediently replaced the oncogenes. If binary vectors are used, they can be transferred to agrobacteria either by bacterial conjugation or by direct transfer. These agrobacteria expediently already comprise the vector bearing the vir genes (currently referred to as helper Ti(Ri) plasmid).

**[0223.0.0.0]** One or more markers may expediently also be used together with the nucleic acid construct, or the vector of the invention and, if plants or plant cells shall be transformed together with the T-DNA, with the aid of which the isolation or selection of transformed organisms, such as agrobacteria or transformed plant cells, is possible. These marker genes enable the identification of a successful transfer of the nucleic acid molecules according to the invention via a series of different principles, for example via visual identification with the aid of fluorescence, luminescence or in the wavelength range of light which is discernible for the human eye, by a resistance to herbicides or antibiotics, via what are known as nutritive markers (auxotrophism markers) or antinutritive markers, via enzyme assays or via phytohormones. Examples of such markers which may be mentioned are GFP (= green fluorescent protein); the luciferin/luceferase system, the β-galactosidase with its colored substrates, for example X-Gal, the herbicide resistances to, for example, imidazolinone, glyphosate, phosphinothricin or sulfonylurea, the antibiotic resistances to, for example, bleomycin, hygromycin, streptomycin, kanamycin, tetracyclin, chloramphenicol, ampicillin, gentamycin, geneticin (G418), spectinomycin or blasticidin, to mention only a few, nutritive markers such as the utilization of mannose or xylose, or antinutritive markers such as the resistance to 2-deoxyglucose. This list is a small number of possible markers. The skilled worker is very familiar with such markers. Different markers are preferred, depending on the organism and the selection method.

**[0224.0.0.0]** As a rule, it is desired that the plant nucleic acid constructs are flanked by T-DNA at one or both sides of the codogenic gene segment. This is particularly useful when bacteria of the species Agrobacterium tumefaciens or Agrobacterium rhizogenes are used for the transformation. A method, which is preferred in accordance with the invention, is the transformation with the aid of Agrobacterium tumefaciens. However, biolistic methods may also be used advantageously for introducing the sequences in the process according to the invention, and the introduction by means of PEG is also possible. The transformed agrobacteria can be grown in the manner known per se and are thus available for the expedient transformation of the plants. The plants or plant parts to be transformed are grown or provided in the customary manner. The transformed agrobacteria are subsequently allowed to act on the plants or plant parts until a sufficient transformation rate is reached. Allowing the agrobacteria to act on the plants or plant parts can take different forms. For example, a culture of morphogenic plant cells or tissue may be used. After the T-DNA transfer, the bacteria are, as a rule, eliminated by antibiotics, and the regeneration of plant tissue is induced. This is done in particular using suitable plant hormones in order to initially induce callus formation and then to promote shoot development.

**[0225.0.0.0]** The transfer of foreign genes into the genome of a plant is called transformation. In doing this the methods described for the transformation and regeneration of plants from plant tissues or plant cells are utilized for transient or stable transformation. An advantageous transformation method is the transformation *in planta.* To this end, it is possible, for example, to allow the agrobacteria to act on plant seeds or to inoculate the plant meristem with agrobacteria. It has proved particularly expedient in accordance with the invention to allow a suspension of transformed agrobacteria to act on the intact plant or at least the flower primordia. The plant is subsequently grown on until the seeds of the treated plant are obtained (Clough and Bent, Plant J. (1998) 16, 735-743). To select transformed plants, the plant material obtained in the transformation is, as a rule, subjected to selective conditions so that transformed plants can be distinguished from untransformed plants. For example, the seeds obtained in the above-described manner can be planted and, after an initial growing period, subjected to a suitable selection by spraying. A further possibility consists in growing the seeds, if appropriate after sterilization, on agar plates using a suitable selection agent so that only the transformed seeds can grow into plants.
Further advantageous transformation methods, in particular for plants, are known to the skilled worker and are described hereinbelow.

**[0226.0.0.0]** Further advantageous and suitable methods are protoplast transformation by poly(ethylene glycol)-induced DNA uptake, the "biolistic" method using the gene cannon - referred to as the particle bombardment method, electroporation, the incubation of dry embryos in DNA solution, microinjection and gene transfer mediated by Agrobacterium. Said methods are described by way of example in B. Jenes et al., Techniques for Gene Transfer, in: Transgenic Plants, Vol. 1, Engineering and Utilization, eds. S.D. Kung and R. Wu, Academic Press (1993) 128-143 and in Potrykus Annu. Rev. Plant Physiol. Plant Molec. Biol. 42 (1991) 205-225). The nucleic acids or the construct to be expressed is preferably cloned into a vector, which is suitable for transforming Agrobacterium tumefaciens, for example pBin19 (Bevan et al., Nucl. Acids Res. 12 (1984) 8711). Agrobacteria transformed by such a vector can then be used in known manner for the transformation of plants, in particular of crop plants such as by way of example tobacco plants, for example by bathing bruised leaves or chopped leaves in an agrobacterial solution and then culturing them in suitable media. The transformation of plants by means of Agrobacterium tumefaciens is described, for example, by Höfgen and Willmitzer in Nucl. Acid Res. (1988) 16, 9877 or is known inter alia from F.F. White, Vectors for Gene Transfer in Higher Plants; in Transgenic Plants, Vol. 1, Engineering and Utilization, eds. S.D. Kung and R. Wu, Academic Press, 1993, pp. 15-38.

**[0227.0.0.0]** The abovementioned nucleic acid molecules can be cloned into the nucleic acid constructs or vectors according to the invention in combination together with further genes, or else different genes are introduced by transforming several nucleic acid constructs or vectors (including plasmids) into a host cell, advantageously into a plant cell or a microorganisms.

In addition to a sequence indicated in Table I, columns 5 or 7, lines 1 to 5 and/or lines 334 to 338 or its derivatives, it is advantageous additionally to express and/or mutate further genes in the organisms. Especially advantageously, additionally at least one further gene of the amino acid biosynthetic pathway such as for L-lysine, L-threonine and/or L-methionine is expressed in the organisms such as plants or microorganisms. It is also possible that the regulation of the natural genes has been modified advantageously so that the gene and/or its gene product is no longer subject to the regulatory mechanisms which exist in the organisms. This leads to an increased synthesis of the amino acids desired since, for example, feedback regulations no longer exist to the same extent or not at all. In addition it might be advantageously to combine a sequence as indicated in Table I, columns 5 or 7, lines 1 to 5 and/or lines 334 to 338 with genes which generally support or enhances to growth or yield of the target organismen, for example genes which lead to faster growth rate of microorganisms or genes which produces stress-, pathogen, or herbicide resistant plants.

**[0228.0.0.0]** In a further embodiment of the process of the invention, therefore, organisms are grown, in which there is simultaneous overexpression of at least one nucleic acid or one of the genes which code for proteins involved in the amino acid metabolism, in particular in amino acid synthesis.

**[0229.0.0.0]** A further advantageous nucleic acid sequence which can be expressed in combination with the sequences used in the process and/or the abovementioned biosynthesis genes is the sequence of the ATP/ADP translocator as described in WO 01/20009. This ATP/ADP translocator leads to an increased synthesis of the essential amino acids lysine and/or methionine. Furthermore, an advantageous nucleic acid sequence coexpressed can be threonine adlolase and/or lysine decarboxylase as described in the state of the art.

**[0230.0.0.0]** In a further advantageous embodiment of the process of the invention, the organisms used in the process are those in which simultaneously at least one of the aforementioned genes or one of the aforementioned nucleic acids is mutated so that the activity of the corresponding proteins is influenced by metabolites to a smaller extent compared with the unmutated proteins, or not at all, and that in particular the production according to the invention of the respective fine chemical is not impaired, or so that their specific enzymatic activity is increased. Less influence means in this connection that the regulation of the enzymic activity is less by at least 10%, advantageously at least 20, 30 or 40%, particularly advantageously by at least 50, 60, 70, 80 or 90%, compared with the starting organism, and thus the activity of the enzyme is increased by these figures mentioned compared with the starting organism. An increase in the enzymatic activity means an enzymatic activity which is increased by at least 10%, advantageously at least 20, 30, 40 or 50%, particularly advantageously by at least 60, 70, 80, 90, 100, 200, 300, 500 or 1000%, compared with the starting organism. This leads to an increased productivity of the desired respective fine chemical or of the desired respective fine chemicals.

**[0231.0.0.0]** In a further advantageous embodiment of the process of the invention, the organisms used in the process are those in which simultaneously a methionine degrading protein is attenuated, in particular by reducing the rate of expression of the corresponding gene.

**[0232.0.0.0]** In another embodiment of the process of the invention, the organisms used in the process are those in which simultaneously at least one of the aforementioned nucleic acids or of the aforementioned genes is mutated in such a way that the enzymatic activity of the corresponding protein is partially reduced or completely blocked. A reduction in the enzymatic activity means an enzymatic activity, which is reduced by at least 10%, advantageously at least 20, 30 or 40%, particularly advantageously by at least 50, 60 or 70%, preferably more, compared with the starting organism.

**[0233.0.0.0]** If it is intended to transform the host cell, in particular the plant cell, with several constructs or vectors, the marker of a preceding transformation must be removed or a further marker employed in a following transformation. The markers can be removed from the host cell, in particular the plant cell, as described hereinbelow via methods with which the skilled worker is familiar. In particular plants without a marker, in particular without resistance to antibiotics, are an especially preferred embodiment of the present invention.

**[0234.0.0.0]** In the process according to the invention, the nucleic acid sequences used in the process according to the invention are advantageously linked operably to one or more regulatory signals in order to increase gene expression. These regulatory sequences are intended to enable the specific expression of the genes and the expression of protein. Depending on the host organism for example plant or microorganism, this may mean, for example, that the gene is expressed and/or overexpressed after induction only, or that it is expressed and/or overexpressed constitutively. These regulatory sequences are, for example, sequences to which the inductors or repressors bind and which thus regulate the expression of the nucleic acid. In addition to these novel regulatory sequences, or instead of these sequences, the natural regulation of these sequences may still be present before the actual structural genes and, if appropriate, may have been genetically modified so that the natural regulation has been switched off and gene expression has been increased. However, the nucleic acid construct of the invention suitable as expression cassette (= expression construct = gene construct) can also be simpler in construction, that is to say no additional regulatory signals have been inserted before the nucleic acid sequence or its derivatives, and the natural promoter together with its regulation has not been removed. Instead, the natural regulatory sequence has been mutated in such a way that regulation no longer takes place and/or gene expression is increased.
These modified promoters can also be introduced on their own before the natural gene in the form of part sequences (= promoter with parts of the nucleic acid sequences according to the invention) in order to increase the activity. Moreover, the gene construct can advantageously also comprise one or more of what are known as enhancer sequences in operable linkage with the promoter, and these enable an increased expression of the nucleic acid sequence. Also, it is possible to insert additional advantageous sequences at the 3' end of the DNA sequences, such as, for example, further regulatory elements or terminators.

**[0235.0.0.0]** The nucleic acid molecules, which encode proteins according to the invention and nucleic acid molecules, which encode other polypeptides may be present in one nucleic acid construct or vector or in several ones. Advantageously, only one copy of the nucleic acid molecule of the invention or the nucleic acid molecule used in the method of the invention or its encoding genes is present in the nucleic acid construct or vector. Several vectors or nucleic acid construct or vector can be expressed together in the host organism. The nucleic acid molecule or the nucleic acid construct or vectoraccording to the invention can be inserted in a vector and be present in the cell in a free form. If a stable transformation is preferred, a vector is used, which is stably duplicated over several generations or which is else be inserted into the genome. In the case of plants, integration into the plastid genome or, in particular, into the nuclear genome may have taken place. For the insertion of more than one gene in the host genome the genes to be expressed are present together in one gene construct, for example in above-described vectors bearing a plurality of genes.

**[0236.0.0.0]** As a rule, regulatory sequences for the expression rate of a gene are located upstream (5'), within, and/or downstream (3') relative to to the coding sequence of the nucleic acid molecule of the invention or the nucleic acid molecule used in the method of the invention or another codogenic gene segment. They control in particular transcription and/or translation and/or the transcript stability. The expression level is dependent on the conjunction of further cellular regulatory systems, such as the protein biosynthesis and degradation systems of the cell.

**[0237.0.0.0]** Regulatory sequences include transcription and translation regulating sequences or signals, e.g. sequences located upstream (5'), which concern in particular the regulation of transcription or translation initiation, such as promoters or start codons, and sequences located downstream (3'), which concern in particular the regulation of transcription or translation termination and transcript stability, such as polyadenylation signals or stop codons. Regulatory sequences can also be present in transcribed coding regions as well in transcribed non-coding regions, e.g. in introns, as for example splicing sites. Promoters for the regulation of expression of the nucleic acid molecule according to the invention in a cell and which can be employed are, in principle, all those which are capable of stimulating the transcription of genes in the organisms in question, such as microorganisms or plants. Suitable promoters, which are functional in these organisms are generally known. They may take the form of constitutive or inducible promoters. Suitable promoters can enable the development- and/or tissue-specific expression in multi-celled eukaryotes; thus, leaf-, root-, flower-, seed-, stomata-, tuber- or fruit-specific promoters may advantageously be used in plants.

**[0238.0.0.0]** The regulatory sequences or factors can, as described above, have a positive effect on, the expression of the genes introduced, thus increasing their expression. Thus, an enhancement of the expression can advantageously take place at the transcriptional level by using strong transcription signals such as strong promoters and/or strong enhancers. In addition, enhancement of expression on the translational level is also possible, for example by introducing translation enhancer sequences, e.g., the Ω enhancer e.g. improving the ribosomal binding to the transcript, or by increasing the stability of the mRNA, e.g. by replacing the 3'UTR coding region by a region encoding a 3'UTR known as conferring an high stability of the transcript or by stabilization of the transcript through the elimination of transcript instability, so that the mRNA molecule is translated more often than the wild type. For example in plants AU-rich elements (AREs) and DST (downstream) elements destabilized transcripts. Mutagenesis studies have demonstrated that residues within two of the conserved domains, the ATAGAT and the GTA regions, are necessary for instability function. Therefore removal or mutation of such elements would obviously lead to more stable transcripts, higher transcript rates and higher protein activity. Translation enhancers are also the "overdrive sequence", which comprises the tobacco mosaic virus 5'-untranslated leader sequence and which increases the protein/RNA ratio (Gallie et al., 1987, Nucl. Acids Research 15:8693-8711)
Enhancers are generally defined as cis active elements, which can stimulate gene transcription independent of position and orientation. Different enhancers have been identified in plants, which can either stimulate transcription constitutively or tissue or stimuli specific. Well known examples for constitutive enhancers are the enhancer from the 35S promoter (Odell et al., 1985, Nature 313:810-812) or the ocs enhancer (Fromm et al., 1989, Plant Cell 1: 977:984) Another examples are the G-Box motif tetramer which confers high-level constitutive expression in dicot and monocot plants (Ishige et al., 1999, Plant Journal, 18, 443-448) or the petE, a A/T-rich sequence which act as quantitative enhancers of gene expression in transgenic tobacco and potato plants (Sandhu et al., 1998; Plant Mol Biol. 37(5):885-96). Beside that, a large variety of cis-active elements have been described which contribute to specific expression pattern, like organ specific expression or induced expression in response to biotic or abiotic stress. Examples are elements which provide pathogen or wound-induced expression (Rushton,_2002, Plant Cell, 14, 749-762) or guard cell-specific expression (Plesch, 2001, Plant Journal 28, 455-464).

**[0239.0.0.0]** Advantageous regulatory sequences for the expression of the nucleic acid molecule according to the invention in microorganisms are present for example in promoters such as the cos, tac, rha, trp, tet, trp-tet, lpp, lac, lpp-lac, lacl^{q-,} T7, T5, T3, gal, trc, ara, SP6, λ-P_{R} or λ-P_{L} promoter, which are advantageously used in Gram-negative bacteria. Further advantageous regulatory sequences are present for example in the Gram-positive promoters amy, dnaK, xylS and SPO2, in the yeast or fungal promoters ADC1, MFα, AC, P-60, UASH, MCB, PHO, CYC1, GAPDH, TEF, rp28, ADH. Promoters, which are particularly advantageous, are constitutive, tissue or compartment specific and inducible promoters. In general, "promoter" is understood as meaning, in the present context, a regulatory sequence in a nucleic acid molecule, which mediates the expression of a coding sequence segment of a nucleic acid molecule. In general, the promoter is located upstream to the coding sequence segment. Some elements, for example expression-enhancing elements such as enhancer may, however, also be located downstream or even in the transcribed region.

**[0240.0.0.0]** In principle, it is possible to use natural promoters together with their regulatory sequences, such as those mentioned above, for the novel process. It is also possible advantageously to use synthetic promoters, either additionally or alone, in particular when they mediate seed-specific expression such as described in, for example, WO 99/16890.

**[0241.0.0.0]** The expression of the nucleic acid molecules used in the process may be desired alone or in combination with other genes or nucleic acids. Multiple nucleic acid molecules conferring the expression of advantageous genes can be introduced via the simultaneous transformation of several individual suitable nucleic acid constructs, i.e. expression constructs, or, preferably, by combining several expression cassettes on one construct. It is also possible to transform several vectors with in each case several expression cassettes stepwise into the recipient organisms.

**[0242.0.0.0]** As described above the transcription of the genes introduced should advantageously be terminated by suitable terminators at the 3' end of the biosynthesis genes introduced (behind the stop codon). A terminator, which may be used for this purpose is, for example, the OCS1 terminator, the nos3 terminator or the 35S terminator. As is the case with the promoters, different terminator sequences should be used for each gene. Terminators, which are useful in microorganism are for example the fimA terminator, txn terminator or trp terminator. Such terminators can be rho-dependent or rho-independent.

**[0243.0.0.0]** Different plant promoters such as, for example, the USP, the LegB4-, the DC3 promoter or the ubiquitin promoter from parsley or other herein mentioned promoter and different terminators may advantageously be used in the nucleic acid construct.

**[0244.0.0.0]** In order to ensure the stable integration, into the transgenic plant, of nucleic acid molecules used in the process according to the invention in combination with further biosynthesis genes over a plurality of generations, each of the coding regions used in the process should be expressed under the control of its own, preferably unique, promoter since repeating sequence motifs may lead to recombination events or to silencing or, in plants, to instability of the T-DNA.

**[0245.0.0.0]** The nucleic acid construct is advantageously constructed in such a way that a promoter is followed by a suitable cleavage site for insertion of the nucleic acid to be expressed, advantageously in a polylinker, followed, if appropriate, by a terminator located behind the polylinker. If appropriate, this order is repeated several times so that several genes are combined in one construct and thus can be introduced into the transgenic plant in order to be expressed. The sequence is advantageously repeated up to three times. For the expression, the nucleic acid sequences are inserted via the suitable cleavage site, for example in the polylinker behind the promoter. It is advantageous for each nucleic acid sequence to have its own promoter and, if appropriate, its own terminator, as mentioned above. However, it is also possible to insert several nucleic acid sequences behind a promoter and, if appropriate, before a terminator if a polycistronic transcription is possible in the host or target cells. In this context, the insertion site, or the sequence of the nucleic acid molecules inserted, in the nucleic acid construct is not decisive, that is to say a nucleic acid molecule can be inserted in the first or last position in the cassette without this having a substantial effect on the expression. However, it is also possible to use only one promoter type in the construct. However, this may lead to undesired recombination events or silencing effects, as said.

**[0246.0.0.0]** Accordingly, in a preferred embodiment, the nucleic acid construct according to the invention confers expression of the nucleic acid molecule of the invention or the nucleic acid molecule used in the method of the invention, and, optionally further genes, in a plant and comprises one or more plant regulatory elements. Said nucleic acid construct according to the invention advantageously encompasses a plant promoter or a plant terminator or a plant promoter and a plant terminator.

**[0247.0.0.0]** A "plant" promoter comprises regulatory elements, which mediate the expression of a coding sequence segment in plant cells. Accordingly, a plant promoter need not be of plant origin, but may originate from viruses or microorganisms, in particular for example from viruses which attack plant cells.

**[0248.0.0.0]** The plant promoter can also originates from a plant cell, e.g. from the plant, which is transformed with the nucleic acid construct or vector as described herein.
This also applies to other "plant" regulatory signals, for example in "plant" terminators.

**[0249.0.0.0]** A nucleic acid construct suitable for plant expression preferably comprises regulatory elements which are capable of controlling the expression of genes in plant cells and which are operably linked so that each sequence can fulfill its function. Accordingly, the nucleic acid construct can also comprise transcription terminators. Examples for transcriptional termination arepolyadenylation signals. Preferred polyadenylation signals are those which originate from Agrobacterium tumefaciens T-DNA, such as the gene 3 of the Ti plasmid pTiACH5, which is known as octopine synthase (Gielen et al., EMBO J. 3 (1984) 835 et seq.) or functional equivalents thereof, but all the other terminators which are functionally active in plants are also suitable.

**[0250.0.0.0]** The nucleic acid construct suitable for plant expression preferably also comprises other operably linked regulatory elements such as translation enhancers, for example the overdrive sequence, which comprises the tobacco mosaic virus 5'-untranslated leader sequence, which increases the protein/RNA ratio (Gallie et al., 1987, Nucl. Acids Research 15:8693-8711).

**[0251.0.0.0]** Other preferred sequences for use in operable linkage in gene expression constructs are targeting sequences, which are required for targeting the gene product into specific cell compartments (for a review, see Kermode, Crit. Rev. Plant Sci. 15, 4 (1996) 285-423 and references cited therein), for example into the vacuole, the nucleus, all types of plastids, such as amyloplasts, chloroplasts, chromoplasts, the extracellular space, the mitochondria, the endoplasmic reticulum, elaioplasts, peroxisomes, glycosomes, and other compartments of cells or extracellular. Sequences, which must be mentioned in this context are, in particular, the signal-peptide- or transit-peptide-encoding sequences which are known per se. For example, plastid-transit-peptide-encoding sequences enable the targeting of the expression product into the plastids of a plant cellTargeting sequences are also known for eukaryotic and to a lower extent for prokaryotic organisms and can advantageously be operable linked with the nucleic acid molecule of the present invention to achieve an expression in one of said compartments or extracellular.

**[0252.0.0.0]** For expression in plants, the nucleic acid molecule must, as described above, be linked operably to or comprise a suitable promoter which expresses the gene at the right point in time and in a cell- or tissue-specific manner. Usable promoters are constitutive promoters (Benfey et al., EMBO J. 8 (1989) 2195-2202), such as those which originate from plant viruses, such as 35S CAMV (Franck et al., Cell 21 (1980) 285-294), 19S CaMV (see also US 5352605 and WO 84/02913), 34S FMV (Sanger et al., Plant. Mol. Biol., 14, 1990: 433-443), the parsley ubiquitin promoter, or plant promoters such as the Rubisco small subunit promoter described in US 4,962,028 or the plant promoters PRP1 [Ward et al., Plant. Mol. Biol. 22 (1993)], SSU, PGEL1, OCS [Leisner (1988) Proc Natl Acad Sci USA 85(5):2553-2557], lib4, usp, mas [Comai (1990) Plant Mol Biol 15 (3):373-381], STLS1, ScBV (Schenk (1999) Plant Mol Biol 39(6):1221-1230), B33, SAD1 or SAD2 (flax promoters, Jain et al., Crop Science, 39 (6), 1999: 1696-1701) or nos [Shaw et al. (1984) Nucleic Acids Res. 12(20):7831-7846]. Stable, constitutive expression of the proteins according to the invention a plant can be advantageous. However, inducible expression of the polypeptide of the invention or the polypeptide used in the method of the invention is advantageous, if a late expression before the harvest is of advantage, as metabolic manipulation may lead to a plant growth retardation.

**[0253.0.0.0]** The expression of plant genes can also be facilitated as described above via a chemical inducible promoter (for a review, see Gatz 1997, Annu. Rev. Plant Physiol. Plant Mol. Biol., 48:89-108). Chemically inducible promoters are particularly suitable when it is desired to express the gene in a time-specific manner. Examples of such promoters are a salicylic acid inducible promoter (WO 95/19443), and abscisic acid-inducible promoter (EP 335 528), a tetracyclin-inducible promoter (Gatz et al. (1992) Plant J. 2, 397-404), a cyclohexanol- or ethanol-inducible promoter (WO 93/21334) or others as described herein.

**[0254.0.0.0]** Other suitable promoters are those which react to biotic or abiotic stress conditions, for example the pathogen-induced PRP1 gene promoter (Ward et al., Plant. Mol. Biol. 22 (1993) 361-366), the tomato heat-inducible hsp80 promoter (US 5,187,267), the potato chill-inducible alpha-amylase promoter (WO 96/12814) or the wound-inducible pinll promoter (EP-A-0 375 091) or others as described herein.

**[0255.0.0.0]** Preferred promoters are in particular those which bring about gene expression in tissues and organs in which the biosynthesis of amino acids takes place, in seed cells, such as endosperm cells and cells of the developing embryo. Suitable promoters are the oilseed rape napin gene promoter (US 5,608,152), the Vicia faba USP promoter (Baeumlein et al., Mol Gen Genet, 1991, 225 (3):459-67), the Arabidopsis oleosin promoter (WO 98/45461), the Phaseolus vulgaris phaseolin promoter (US 5,504,200), the Brassica Bce4 promoter (WO 91/13980), the bean arc5 promoter, the carrot DcG3 promoter, or the Legumin B4 promoter (LeB4; Baeumlein et al., 1992, Plant Journal, 2 (2):233-9), and promoters which bring about the seed-specific expression in monocotyledonous plants such as maize, barley, wheat, rye, rice and the like. Advantageous seed-specific promoters are the sucrose binding protein promoter (WO 00/26388), the phaseolin promoter and the napin promoter. Suitable promoters which must be considered are the barley Ipt2 or Ipt1 gene promoter (WO 95/15389 and WO 95/23230), and the promoters described in WO 99/16890 (promoters from the barley hordein gene, the rice glutelin gene, the rice oryzin gene, the rice prolamin gene, the wheat gliadin gene, the wheat glutelin gene, the maize zein gene, the oat glutelin gene, the sorghum kasirin gene and the rye secalin gene). Further suitable promoters are Amy32b, Amy 6-6 and Aleurain [US 5,677,474], Bce4 (oilseed rape) [US 5,530,149], glycinin (soya) [EP 571 741], phosphoenolpyruvate carboxylase (soya) [JP 06/62870], ADR12-2 (soya) [WO 98/08962], isocitrate lyase (oilseed rape) [US 5,689,040] or α-amylase (barley) [EP 781 849]. Other promoters which are available for the expression of genes in plants are leaf-specific promoters such as those described in DE-A 19644478 or light-regulated promoters such as, for example, the pea petE promoter.

**[0256.0.0.0]** Further suitable plant promoters are the cytosolic FBPase promoter or the potato ST-LSI promoter (Stockhaus et al., EMBO J. 8, 1989, 2445), the Glycine max phosphoribosylpyrophosphate amidotransferase promoter (GenBank Accession No. U87999) or the node-specific promoter described in EP-A-0 249 676.

**[0257.0.0.0]** Other promoters, which are particularly suitable, are those which bring about plastid-specific expression. Suitable promoters such as the viral RNA polymerase promoter are described in WO 95/16783 and WO 97/06250, and the Arabidopsis clpP promoter, which is described in WO 99/46394.

**[0258.0.0.0]** Other promoters, which are used for the strong expression of heterologous sequences in as many tissues as possible, in particular also in leaves, are, in addition to several of the abovementioned viral and bacterial promoters, preferably, plant promoters of actin or ubiquitin genes such as, for example, the rice actin1 promoter. Further examples of constitutive plant promoters are the sugarbeet V-ATPase promoters (WO 01/14572). Examples of synthetic constitutive promoters are the Super promoter (WO 95/14098) and promoters derived from G-boxes (WO 94/12015). If appropriate, chemical inducible promoters may furthermore also be used, compare EP-A 388186, EP-A 335528, WO 97/06268.

**[0259.0.0.0]** As already mentioned herein, further regulatory sequences, which may be expedient, if appropriate, also include sequences, which target the transport and/or the localization of the expression products. Sequences, which must be mentioned in this context are, in particular, the signal-peptide- or transit-peptide-encoding sequences which are known per se. For example, plastid-transit-peptide-encoding sequences enable the targeting of the expression product into the plastids of a plant cell.

**[0260.0.0.0]** Preferred recipient plants are, as described above, in particular those plants, which can be transformed in a suitable manner. These include monocotyledonous and dicotyledonous plants. Plants which must be mentioned in particular are agriculturally useful plants such as cereals and grasses, for example Triticum spp., Zea mays, Hordeum vulgare, oats, Secale cereale, Oryza sativa, Pennisetum glaucum, Sorghum bicolor, Triticale, Agrostis spp., Cenchrus ciliaris, Dactylis glomerata, Festuca arundinacea, Lolium spp., Medicago spp. and Saccharum spp., legumes and oil crops, for example Brassica juncea, Brassica napus, Glycine max, Arachis hypogaea, Gossypium hirsutum, Cicer arietinum, Helianthus annuus, Lens culinaris, Linum usitatissimum, Sinapis alba, Trifolium repens and Vicia narbonensis, vegetables and fruits, for example bananas, grapes, Lycopersicon esculentum, asparagus, cabbage, watermelons, kiwi fruit, Solanum tuberosum, Beta vulgaris, cassava and chicory, trees, for example Coffea species, Citrus spp., Eucalyptus spp., Picea spp., Pinus spp. and Populus spp., medicinal plants and trees, and flowers.

**[0261.0.0.0]** One embodiment of the present invention also relates to a method for generating a vector, which comprises the insertion, into a vector, of the nucleic acid molecule characterized herein, the nucleic acid molecule according to the invention or the expression cassette according to the invention. The vector can, for example, be introduced in to a cell, e.g. a microorganism or a plant cell, as described herein for the nucleic acid construct, or below under transformation or transfection or shown in the examples. A transient or stable transformation of the host or target cell is possible, however, a stable transformation is preferred. The vector according to the invention is preferably a vector, which is suitable for expressing the polypeptide according to the invention in a plant. The method can thus also encompass one or more steps for integrating regulatory signals into the vector, in particular signals, which mediate the expression in microorganisms or plants.

**[0262.0.0.0]** Accordingly, the present invention also relates to a vector comprising the nucleic acid molecule characterized herein as part of a nucleic acid construct suitable for plant expression or the nucleic acid molecule according to the invention.

**[0263.0.0.0]** The advantageous vectors of the inventioncomprise the nucleic acid molecules which encode proteins according to the invention, nucleic acid molecules which are used in the process, or nucleic acid construct suitable for plant expression comprising the nucleic acid molecules used, either alone or in combination with further genes such as the biosynthesis or regulatory genes of the respective fine chemical metabolism e.g. with the genes mentioned herein above. In accordance with the invention, the term "vector" refers to a nucleic acid molecule, which is capable of transporting another nucleic acid to which it is linked. One type of vector is a "plasmid", which means a circular double-stranded DNA loop into which additional DNA segments can be ligated. A further type of vector is a viral vector, it being possible to ligate additional nucleic acids segments into the viral genome. Certain vectors are capable of autonomous replication in a host cell into which they have been introduced (for example bacterial vectors with bacterial replication origin). Other preferred vectors are advantageously completely or partly integrated into the genome of a host cell when they are introduced into the host cell and thus replicate together with the host genome. Moreover, certain vectors are capable of controlling the expression of genes with which they are in operable linkage. In the present context, these vectors are referred to as "expression vectors". As mentioned above, they are capable of autonomous replication or may be integrated partly or completely into the host genome. Expression vectors, which are suitable for DNA recombination techniques usually take the form of plasmids. In the present description, "plasmid" and "vector" can be used interchangeably since the plasmid is the most frequently used form of a vector. However, the invention is also intended to encompass these other forms of expression vectors, such as viral vectors, which exert similar functions. The term vector is furthermore also to encompass other vectors which are known to the skilled worker, such as phages, viruses such as SV40, CMV, TMV, transposons, IS elements, phasmids, phagemids, cosmids, and linear or circular DNA.

**[0264.0.0.0]** The recombinant expression vectors which are advantageously used in the process comprise the nucleic acid molecules according to the invention or the nucleic acid construct according to the invention in a form which is suitable for expressing, in a host cell, the nucleic acid molecules according to the invention or described herein. Accordingly, the the recombinant expression vectors comprise one or more regulatory signals selected on the basis of the host cells to be used for the expression, in operable linkage with the nucleic acid sequence to be expressed.

**[0265.0.0.0]** In a recombinant expression vector, "operable linkage" means that the nucleic acid molecule of interest is linked to the regulatory signals in such a way that expression of the nucleic acid molecule is possible: they are linked to one another in such a way that the two sequences fulfill the predicted function assigned to the sequence (for example in an in-vitro transcription/translation system, or in a host cell if the vector is introduced into the host cell).

**[0266.0.0.0]** The term "regulatory sequence" is intended to comprise promoters, enhancers and other expression control elements (for example polyadenylation signalsThese regulatory sequences are described, for example, in Goeddel: Gene Expression Technology: Methods in Enzymology 185, Academic Press, San Diego, CA (1990), or see: Gruber and Crosby, in: Methods in Plant Molecular Biology and Biotechnolgy, CRC Press, Boca Raton, Florida, Ed.: Glick and Thompson, chapter 7, 89-108, including the references cited therein. Regulatory sequences encompass those, which control the constitutive expression of a nucleotide sequence in many types of host cells and those which control the direct expression of the nucleotide sequence in specific host cells only, and under specific conditions. The skilled worker knows that the design of the expression vector may depend on factors such as the selection of the host cell to be transformed, the extent to which the desired protein is expressed, and the like. A preferred selection of regulatory sequences is described above, for example promoters, terminators, enhancers and the like. The term regulatory sequence is to be considered as being encompassed by the term regulatory signal. Several advantageous regulatory sequences, in particular promoters and terminators are described above. In general, the regulatory sequences described as advantageous for nucleic acid construct suitable for expression are also applicable for vectors.

**[0267.0.0.0]** The recombinant expression vectors used can be designed specifically for the expression, in prokaryotic and/or eukaryotic cells, of nucleic acid molecules used in the process. This is advantageous since intermediate steps of the vector construction are frequently carried out in microorganisms for the sake of simplicity. For example, the genes according to the invention and other genes can be expressed in bacterial cells, insect cells (using baculovirus expression vectors), yeast cells and other fungal cells [Romanos (1992), Yeast 8:423-488; van den Hondel, (1991), in: More Gene Manipulations in Fungi, J.W. Bennet & L.L. Lasure, Ed., pp. 396-428: Academic Press: San Diego; and van den Hondel, C.A.M.J.J. (1991), in: Applied Molecular Genetics of Fungi, Peberdy, J.F., et al., Ed., pp. 1-28, Cambridge University Press: Cambridge], algae [Falciatore et al., 1999, Marine Biotechnology.1, 3:239-251] using vectors and following a transformation method as described in WO 98/01572, and preferably in cells of multi-celled plants [see Schmidt, R. and Willmitzer, L. (1988) Plant Cell Rep.:583-586; Plant Molecular Biology and Biotechnology, C Press, Boca Raton, Florida, chapter 6/7, pp.71-119 (1993); F.F. White, in: Transgenic Plants, Bd. 1, Engineering and Utilization, Ed.: Kung and R. Wu, Academic Press (1993), 128-43; Potrykus, Annu. Rev. Plant Physiol. Plant Molec. Biol. 42 (1991), 205-225 (and references cited therein)]. Suitable host cells are furthermore discussed in Goeddel, Gene Expression Technology: Methods in Enzymology 185, Academic Press, San Diego, CA (1990). As an alternative, the sequence of the recombinant expression vector can be transcribed and translated in vitro, for example using T7 promotor-regulatory sequences and T7 polymerase.

**[0268.0.0.0]** Proteins can be expressed in prokaryotes using vectors comprising constitutive or inducible promoters, which control the expression of fusion proteins or nonfusion proteins. Typical fusion expression vectors are, inter alia, pGEX (Pharmacia Biotech Inc; Smith, D.B., and Johnson, K.S. (1988) Gene 67:31-40), pMAL (New England Biolabs, Beverly, MA) and pRIT5 (Pharmacia, Piscataway, NJ), in which glutathione-S-transferase (GST), maltose-E-binding protein or protein A is fused with the recombinant target protein. Examples of suitable inducible nonfusion E. coli expression vectors are, inter alia, pTrc (Amann et al. (1988) Gene 69:301-315) and pET 11 d [Studier et al., Gene Expression Technology: Methods in Enzymology 185, Academic Press, San Diego, California (1990) 60-89]. The target gene expression of the pTrc vector is based on the transcription of a hybrid trp-lac fusion promoter by the host RNA polymerase. The target gene expression from the pET 11 d vector is based on the transcription of a T7-gn10-lac fusion promoter, which is mediated by a coexpressed viral RNA polymerase (T7 gn1). This viral polymerase is provided by the host strains BL21 (DE3) or HMS174 (DE3) by a resident λ-prophage which harbors a T7 gn1 gene under the transcriptional control of the lacUV 5 promoter.

**[0269.0.0.0]** Other vectors which are suitable in prokaryotic organisms are known to the skilled worker; these vectors are for example in E. coli pLG338, pACYC184, the pBR series, such as pBR322, the pUC series such as pUC18 or pUC19, the M113mp series, pKC30, pRep4, pHS1, pHS2, pPLc236, pMBL24, pLG200, pUR290, pIN-III¹¹³-B1, λgt11 or pBdCl, in Streptomyces pIJ101, pIJ364, pIJ702 or pIJ361, in Bacillus pUB110, pC194 or pBD214, in Corynebacterium pSA77 or pAJ667.

**[0270.0.0.0]** In a further embodiment, the expression vector is a yeast expression vector. Examples of vectors for expression in the yeasts S. cerevisiae encompass pYeDesaturasec1 (Baldari et al. (1987) Embo J. 6:229-234), pMFa (Kurjan and Herskowitz (1982) Cell 30:933-943), pJRY88 (Schultz et al. (1987) Gene 54:113-123) and pYES2 (Invitrogen Corporation, San Diego, CA). Vectors and methods for the construction of vectors which are suitable for use in other fungi, such as the filamentous fungi, encompass those which are described in detail in: van den Hondel, C.A.M.J.J. [(1991), J.F. Peberdy, Ed., pp. 1-28, Cambridge University Press: Cambridge; or in: More Gene Manipulations in Fungi; J.W. Bennet & L.L. Lasure, Ed., pp. 396-428: Academic Press: San Diego]. Examples of other suitable yeast vectors are 2µM, pAG-1, YEp6, YEp13 or pEMBLYe23.

**[0271.0.0.0]** Further vectors, which may be mentioned by way of example, are pALS1, pIL2 or pBB116 in fungi or pLGV23, pGHlac⁺, pBIN19, pAK2004 or pDH51 in plants.

**[0272.0.0.0]** As an alternative, the nucleic acid sequences can be expressed in insect cells using baculovirus expression vectors. Baculovirus vectors, which are available for expressing proteins in cultured insect cells (for example Sf9 cells) encompass the pAc series (Smith et al. (1983) Mol. Cell Biol. 3:2156-2165) and the pVL series (Lucklow and Summers (1989) Virology 170:31-39).

**[0273.0.0.0]** The abovementioned vectors are only a small overview of potentially suitable vectors. Further plasmids are known to the skilled worker and are described, for example, in: Cloning Vectors (Ed. Pouwels, P.H., et al., Elsevier, Amsterdam-New York-Oxford, 1985, ISBN 0 444 904018). Further suitable expression systems for prokaryotic and eukaryotic cells, see the chapters 16 and 17 by Sambrook, J., Fritsch, E.F., and Maniatis, T., Molecular Cloning: A Laboratory Manual, 2nd Edition, Cold Spring Harbor Laboratory, Cold Spring Harbor Laboratory Press, Cold Spring Harbor, NY, 1989.

**[0274.0.0.0]** Accordingly, one embodiment of the invention relates to a vector where the nucleic acid molecule according to the invention is linked operably to regulatory sequences which permit the expression in a prokaryotic or eukaryotic or in a prokaryotic and eukaryotic host.

**[0275.0.0.0]** Accordingly, one embodiment of the invention relates to a host cell, which has been transformed stably or transiently with the vector according to the invention or the nucleic acid molecule according to the invention or the nucleic acid construct according to the invention.

**[0276.0.0.0]** Depending on the host organism, the organisms used in the process according to the invention are cultured or grown in a manner with which the skilled worker is familiar. As a rule, microorganisms are grown in a liquid medium comprising a carbon source, usually in the form of sugars, a nitrogen source, usually in the form of organic nitrogen sources such as yeast extract or salts such as ammonium sulfate, trace elements such as iron salts, manganese salts, magnesium salts, and, if appropriate, vitamins, at temperatures between 0°C and 100°C, preferably between 10°C and 60°C, while passing in oxygen. In the event the microorganism is anaerobe, no oxygen is blown through the culture medium. The pH value of the liquid nutrient medium may be kept constant, that is to say regulated during the culturing phase, or not. The organisms may be cultured batchwise, semibatchwise or continuously. Nutrients may be provided at the beginning of the fermentation or fed in semicontinuously or continuously.

**[0277.0.0.0]** The amino acids produced can be isolated from the organism by methods with which the skilled worker is familiar. For example via extraction, salt precipitation and/or ion-exchange chromatography. To this end, the organisms may be disrupted beforehand. The process according to the invention can be conducted batchwise, semibatchwise or continuously. A summary of known culture and isolation techniques can be found in the textbook by Chmiel [Bioprozeßtechnik 1, Einführung in die Bioverfahrenstechnik (Gustav Fischer Verlag, Stuttgart, 1991)], Demain et al. (Industrial Microbiology and Biotechnology, second edition, ASM Press, Washington, D.C., 1999, ISBN 1-55581-128-0] or in the textbook by Storhas (Bioreaktoren und periphere Einrichtungen (Vieweg Verlag, Braunschweig/Wiesbaden, 1994)). **[0278.0.0.0]** In one embodiment, the present invention relates to a polypeptide encoded by the nucleic acid molecule according to the present invention, preferably conferring an increase in the respective fine chemical content in an organism or cell after increasing the expression or activity.

**[0279.0.0.0]** The present invention also relates to a process for the production of a polypeptide according to the present invention, the polypeptide being expressed in a host cell according to the invention, preferably in a microorganism or a transgenic plant cell.

**[0280.0.0.0]** In one embodiment, the nucleic acid molecule used in the process for the production of the polypeptide is derived from a microorganism, preferably from a prokaryotic or protozoic cell with an eukaryotic organism as host cell. E.g., in one embodiment the polypeptide is produced in a plant cell or plant with a nucleic acid molecule derived from a prokaryote or a fungus or an alga or an other microorganism but not from plant.

**[0281.0.0.0]** The skilled worker knows that protein and DNA expressed in different organisms differ in many respects and properties, e.g. DNA modulation and imprinting, such as methylation or post-translational modification, as for example glucosylation, phosphorylation, acetylation, myristoylation, ADP-ribosylation, farnesylation, carboxylation, sulfation, ubiquination, etc. though having the same coding sequence. Preferably, the cellular expression control of the corresponding protein differs accordingly in the control mechanisms controlling the activity and expression of an endogenous protein or another eukaryotic protein. One major difference between proteins expressed in prokaryotic or eukaryotic organisms is the amount and pattern of glycosylation. For example in E. coli there are no glycosylated proteins. Proteins expressed in yeasts have high mannose content in the glycosylated proteins, whereas in plants the glycosylation pattern is complex.

**[0282.0.0.0]** The polypeptide of the present invention is preferably produced by recombinant DNA techniques. For example, a nucleic acid molecule encoding the protein is cloned into a vector (as described above), the vector is introduced into a host cell (as described above) and said polypeptide is expressed in the host cell. Said polypeptide can then be isolated from the cells by an appropriate purification scheme using standard protein purification techniques. Alternative to recombinant expression, the polypeptide or peptide of the present invention can be synthesized chemically using standard peptide synthesis techniques.

**[0283.0.0.0]** Moreover, a native polypeptide conferring the increase of the respective fine chemical in an organism or part thereof can be isolated from cells (e.g., endothelial cells), for example using the antibody of the present invention as described below, in particular, an antibody against a protein as indicated in Table II, column 3, lines 1 to 5 and/or lines 334 to 338. E.g. an antibody against a polypeptide as indicated in Table II, columns 5 or 7, lines 1 to 5 and/or lines 334 to 338, or an antigenic part thereof which can be produced by standard techniques utilizing polypeptides comprising or consisting of above mentioned sequences, e.g. the polypeptid of the present invention or fragment thereof. Preferred are monoclonal antibodies specifically binding to polypeptide as indicated in Table II, columns 5 or 7, lines 1 to 5 and/or lines 334 to 338.

**[0284.0.0.0]** In one embodiment, the present invention relates to a polypeptide having the amino acid sequence encoded by a nucleic acid molecule of the invention or the nucleic acid molecule used in the method of the invention or obtainable by a process of the invention. Said polypeptide confers preferably the aforementioned activity, in particular, the polypeptide confers the increase of the respective fine chemical in a cell or an organism or a part thereof after increasing the cellular activity, e.g. by increasing the expression or the specific activity of the polypeptide.

**[0285.0.0.0]** In one embodiment, the present invention relates to a polypeptide having a sequence as indicated in Table II, columns 5 or 7, lines 1 to 5 and/or lines 334 to 338 or as encoded by a nucleic acid molecule as indicated in Table I, columns 5 or 7, lines 1 to 5 and/or lines 334 to 338 or functional homologues thereof.

**[0286.0.0.0]** In one advantageous embodiment, in the method of the present invention the activity of a polypeptide is increased which comprises or consists of a consensus sequence as indicated in Table IV, column 7, lines 1 to 5 and/or lines 334 to 338 and in one another embodiment, the present invention relates to a polypeptide comprising or consisting of a consensus sequence as indicated in Table IV, column 7, lines 1 to 5 and/or lines 334 to 338, whereby 20 or less, preferably 15 or 10, preferably 9, 8, 7, or 6, more preferred 5 or 4, even more preferred 3, even more preferred 2, even more preferred 1, most preferred 0 of the amino acids positions indicated can be replaced by any amino acid or, in an further embodiment, can be replaced and/or absent. In one embodiment, the present invention relates to the method of the present invention comprising a polypeptide or to a polypeptide comprising more than one consensus sequences (of an individual line) as indicated in Table IV, column 7, lines 1 to 5 and/or lines 334 to 338.

**[0287.0.0.0]** In one embodiment not more than 15%, preferably 10%, even more preferred 5%, 4%, 3%, or 2%, most preferred 1% or 0% of the amino acid position indicated by a letter are/is replaced another amino acid or, in an other embodiment, are/is absent and/or replaced. In another embodiment the stretches of non-conserved amino acids, indicated by (X)ₙ [whereas n indicates the number of X], vary in their length by 20%, preferably by 15 or 10 %, even more preferred by 5%, 4%, 3%, 2% or most preferred by only 1 %.

**[0288.0.0.0]** In one embodiment 20 or less, preferably 15 or 10, preferably 9, 8, 7, or 6, more preferred 5 or 4, even more preferred 3, even more preferred 2, even more preferred 1, most preferred 0 amino acids are inserted into the consensus sequence or, in an other embodiment, are absent and/or replaced.

**[0289.0.0.0]** The consensus sequence shown herein was derived from a multiple alignment of the sequences as listed in table II. The consensus sequences of specified domains were derived from a multiple alignment of all sequences. The letters represent the one letter amino acid code and indicate that the amino acids are conserved in all aligned proteins. The letter X stands for amino acids, which are not conserved in all sequences.
In one example, in the cases where only a small selected subset of amino acids are possible at a certain position these amino acids are given in brackets. The number of given X indicates the distances between conserved amino acid residues, e.g. YX(21-23)F means that conserved tyrosine and phenylalanine residues are separated from each other by minimum 21 and maximum 23 amino acid residues in all investigated sequences.

**[0290.0.0.0]** The alignment was performed with the Software AlignX (sept 25, 2002) a component of Vector NTI Suite 8.0, InforMax™,Invitrogen™ life science software, U.S. Main Office, 7305 Executive Way,Frederick, MD 21704,USA with the following settings: For pairwise alignments: gap opening penality: 10,0; gap extension penality 0,1. For multiple alignments: Gap opening penalty: 10,0; Gap extension penalty: 0,1; Gap separation penalty range: 8; Residue substitution matrix: blosum62; Hydrophilic residues: G P S N D Q E K R; Transition weighting: 0,5; Consensus calculation options: Residue fraction for consensus: 0,9. Presettings were selected to allow also for the alignment of conserved amino acids.

**[0291.0.0.0]** In one advantageous embodiment, the method of the present invention comprises the increasing of a polypeptide comprising or consisting of plant or microorganism specific consensus sequences. Accordingly, in one embodiment, the present invention relates to a polypeptide comprising or consisting of plant or microorganism specific consensus sequences.
In one embodiment, said polypeptide of the invention distinguishes over a sequence as indicated in Table II A or IIB, columns 5 or 7, lines 1 to 5 and/or lines 334 to 338 by one or more amino acids. In one embodiment, polypeptide distinguishes form a sequence as indicated in Table II A or IIB, columns 5 or 7, lines 1 to 5 and/or lines 334 to 338 by more than 1, 2, 3, 4, 5, 6, 7, 8 or 9 amino acids, preferably by more than 10, 15, 20, 25 or 30 amino acids, even more preferred are more than 40, 50, or 60 amino acids and, preferably, the sequence of the polypeptide of the invention distinguishes from a sequence as indicated in Table II A or II B, columns 5 or 7, lines 1 to 5 and/or lines 334 to 338 by not more than 80% or 70% of the amino acids, preferably not more than 60% or 50%, more preferred not more than 40% or 30%, even more preferred not more than 20% or 10%. In an other embodiment, said polypeptide of the invention does not consist of a sequence as indicated in Table II A or II B, columns 5 or 7, lines 1 to 5 and/or lines 334 to 338.

**[0292.0.0.0]** In one embodiment, the polypeptide of the invention comprises any one of the sequences not known to the public before. In one embodiment, the polypeptide of the invention originates from a non-plant cell, in particular from a microorganism, and was expressed in a plant cell. In one embodiment, the present invention relates to a polypeptide encoded by the nucleic acid molecule of the invention or used in the process of the invention for which an activity has not been described yet.

**[0293.0.0.0]** In one embodiment, the invention relates to polypeptide conferring an increase in the respective fine chemical in an organism or part being encoded by the nucleic acid molecule of the invention or by a nucleic acid molecule used in the process of the invention.
In one embodiment, the polypeptide of the invention has a sequence which distinguishes from a sequence as indicated in Table II A or II B, columns 5 or 7, lines 1 to 5 and/or lines 334 to 338 by one or more amino acids. In an other embodiment, said polypeptide of the invention does not consist of the sequence as indicated in Table II A or II B, columns 5 or 7, lines 1 to 5 and/or lines 334 to 338. In a further embodiment, said polypeptide of the present invention is less than 100%, 99,999%, 99,99%, 99,9% or 99% identical. In one embodiment, said polypeptide does not consist of the sequence encoded by a nucleic acid molecules as indicated in Table I A or IB, columns 5 or 7, lines 1 to 5 and/or lines 334 to 338.

**[0294.0.0.0]** In one embodiment, the present invention relates to a polypeptide having an activity of a protein as indicated in Table II, column 3, lines 1 to 5 and/or lines 334 to 338, which distinguishes over a sequence as indicated in Table II A or II B, columns 5 or 7, lines 1 to 5 and/or lines 334 to 338 by one or more amino acids, preferably by more than 5, 6, 7, 8 or 9 amino acids, preferably by more than 10, 15, 20, 25 or 30 amino acids, even more preferred are more than 40, 50, or 60 amino acids but even more preferred by less than 70% of the amino acids, more preferred by less than 50%, even more preferred my less than 30% or 25%, more preferred are 20% or 15%, even more preferred are less than 10%.

**[0295.0.0.0]** The terms "protein" and "polypeptide" used in this application are interchangeable. "Polypeptide" refers to a polymer of amino acids (amino acid sequence) and does not refer to a specific length of the molecule. Thus peptides and oligopeptides are included within the definition of polypeptide. This term does also refer to or include post-translational modifications of the polypeptide, for example, glycosylations, acetylations, phosphorylations and the like. Included within the definition are, for example, polypeptides containing one or more analogs of an amino acid (including, for example, unnatural amino acids, etc.), polypeptides with substituted linkages, as well as other modifications known in the art, both naturally occurring and non-naturally occurring.

**[0296.0.0.0]** Preferably, the polypeptide is isolated. An "isolated" or "purified" protein or nucleic acid molecule or biologically active portion thereof is substantially free of cellular material when produced by recombinant DNA techniques or chemical precursors or other chemicals when chemically synthesized.

**[0297.0.0.0]** The language "substantially free of cellular material" includes preparations of the polypeptide of the invention in which the protein is separated from cellular components of the cells in which it is naturally or recombinantly produced. In one embodiment, the language "substantially free of cellular material" includes preparations having less than about 30% (by dry weight) of "contaminating protein", more preferably less than about 20% of "contaminating protein", still more preferably less than about 10% of "contaminating protein", and most preferably less than about 5% "contaminating protein". The term "Contaminating protein" relates to polypeptides, which are not polypeptides of the present invention. When the polypeptide of the present invention or biologically active portion thereof is recombinantly produced, it is also preferably substantially free of culture medium, i.e., culture medium represents less than about 20%, more preferably less than about 10%, and most preferably less than about 5% of the volume of the protein preparation. The language "substantially free of chemical precursors or other chemicals" includes preparations in which the polypeptide of the present invention is separated from chemical precursors or other chemicals, which are involved in the synthesis of the protein. The language "substantially free of chemical precursors or other chemicals" includes preparations having less than about 30% (by dry weight) of chemical precursors or non-polypeptide of the invention-chemicals, more preferably less than about 20% chemical precursors or non-polypeptide of the invention-chemicals, still more preferably less than about 10% chemical precursors or non-polypeptide of the invention-chemicals, and most preferably less than about 5% chemical precursors or non- polypeptide of the invention-chemicals. In preferred embodiments, isolated proteins or biologically active portions thereof lack contaminating proteins from the same organism from which the polypeptide of the present invention is derived. Typically, such proteins are produced by recombinant techniques.

**[0297.1.0.0]** Non-polypeptide of the invention-chemicals are e.g. polypeptides having not the activity and/or amino acid sequence of a polypeptide indicated in Table II, columns 3, 5 or 7, lines 1 to 5 and/or lines 334 to 338.

**[0298.0.0.0]** A polypeptide of the invention can participate in the process of the present invention. The polypeptide or a portion thereof comprises preferably an amino acid sequence which is sufficiently homologous to an amino acid sequence as indicated in Table II, columns 5 or 7, lines 1 to 5 and/or lines 334 to 338 such that the protein or portion thereof maintains the ability to confer the activity of the present invention. The portion of the protein is preferably a biologically active portion as described herein. Preferably, the polypeptide used in the process of the invention has an amino acid sequence identical to a sequence as indicated in Table II, columns 5 or 7, lines 1 to 5 and/or lines 334 to 338.

**[0299.0.0.0]** Further, the polypeptide can have an amino acid sequence which is encoded by a nucleotide sequence which hybridizes, preferably hybridizes under stringent conditions as described above, to a nucleotide sequence of the nucleic acid molecule of the present invention. Accordingly, the polypeptide has an amino acid sequence which is encoded by a nucleotide sequence that is at least about 35%, 40%, 45%, 50%, 55%, 60%, 65% or 70%, preferably at least about 75%, 80%, 85% or 90, and more preferably at least about 91%, 92%, 93%, 94% or 95%, and even more preferably at least about 96%, 97%, 98%, 99% or more homologous to one of the nucleotide sequence as indicated in Table I, columns 5 or 7, lines 1 to 5 and/or lines 334 to 338. The preferred polypeptide of the present invention preferably possesses at least one of the activities according to the invention and described herein. A preferred polypeptide of the present invention includes an amino acid sequence encoded by a nucleotide sequence which hybridizes, preferably hybridizes under stringent conditions, to a nucleotide sequence as indicated in Table I, columns 5 or 7, lines 1 to 5 and/or lines 334 to 338 or which is homologous thereto, as defined above.

**[0300.0.0.0]** Accordingly the polypeptide of the present invention can vary from a sequence as indicated in Table II, columns 5 or 7, lines 1 to 5 and/or lines 334 to 338 in amino acid sequence due to natural variation or mutagenesis, as described in detail herein. Accordingly, the polypeptide comprise an amino acid sequence which is at least about 35%, 40%, 45%, 50%, 55%, 60%, 65% or 70%, preferably at least about 75%, 80%, 85% or 90, and more preferably at least about 91%, 92%, 93%, 94% or 95%, and most preferably at least about 96%, 97%, 98%, 99% or more homologous to an entire amino acid sequence of a sequence as indicated in Table II A or IIB, columns 5 or 7, lines 1 to 5 and/or lines 334 to 338.

**[0301.0.0.0]** For the comparison of amino acid sequences the same algorithms as described above or nucleic acid sequences can be used. Results of high quality are reached by using the algorithm of Needleman and Wunsch or Smith and Waterman. Therefore programs based on said algorithms are preferred. Advantageously the comparisons of sequences can be done with the program PileUp (J. Mol. Evolution., 25, 351-360, 1987, Higgins et al., CABIOS, 5 1989: 151-153) or preferably with the programs Gap and BestFit, which are respectively based on the algorithms of Needleman and Wunsch [J. Mol. Biol. 48; 443-453 (1970)] and Smith and Waterman [Adv. Appl. Math. 2; 482-489 (1981)]. Both programs are part of the GCG software-package [Genetics Computer Group, 575 Science Drive, Madison, Wisconsin, USA 53711 (1991); Altschul et al. (1997) Nucleic Acids Res. 25:3389 et seq.]. Therefore preferably the calculations to determine the percentages of sequence homology are done with the program Gap over the whole range of the sequences. The following standard adjustments for the comparison of amino acid sequences were used: gap weight: 8, length weight: 2, average match: 2.912, average mismatch: -2.003.

**[0302.0.0.0]** Biologically active portions of an polypeptide of the present invention include peptides comprising amino acid sequences derived from the amino acid sequence of the polypeptide of the present invention or used in the process of the present invention, e.g., an amino acid sequence as indicated in Table II, columns 5 or 7, lines 1 to 5 and/or lines 334 to 338 or the amino acid sequence of a protein homologous thereto, which include fewer amino acids than a full length polypeptide of the present invention or used in the process of the present invention or the full length protein which is homologous to an polypeptide of the present invention or used in the process of the present invention depicted herein, and exhibit at least one activity of polypeptide of the present invention or used in the process of the present invention

**[0303.0.0.0]** Typically, biologically (or immunologically) active portions i.e. peptides, e.g., peptides which are, for example, 5, 10, 15, 20, 30, 35, 36, 37, 38, 39, 40, 50, 100 or more amino acids in length comprise a domain or motif with at least one activity or epitope of a polypeptide of the present invention or used in the process of the present invention. Moreover, other biologically active portions, in which other regions of the polypeptide are deleted, can be prepared by recombinant techniques and evaluated for one or more of the activities described herein.

**[0304.0.0.0]** Manipulation of the nucleic acid molecule of the invention or the nucleic acid molecule used in the method of the invention may result in the production of a protein having essentially the activity of the polypeptides as indicated in Table II, column 3, lines 1 to 5 and/or lines 334 to 338 but having differences in the sequence from said wild-type protein. These proteins may be improved in efficiency or activity, may be present in greater numbers in the cell than is usual, or may be decreased in efficiency or activity in relation to the wild type protein.

**[0305.0.0.0]** Any mutagenesis strategies for the polypeptide of the present invention or the polypeptide used in the process of the present invention to result in increasing said activity are not meant to be limiting; variations on these strategies will be readily apparent to one skilled in the art. Using such strategies, and incorporating the mechanisms disclosed herein, the nucleic acid molecule and polypeptide of the invention or the polypeptide used in the method of the invention may be utilized to generate plants or parts thereof, expressing one or more wildtype protein(s) or one or more mutated protein encoding nucleic acid molecule(s) or polypeptide molecule(s) of the invention such that the yield, production, and/or efficiency of production of a desired compound is improved.

**[0306.0.0.0]** This desired compound may be any natural product of plants, which includes the final products of biosynthesis pathways and intermediates of naturally-occurring metabolic pathways, as well as molecules which do not naturally occur in the metabolism of said cells, but which are produced by a said cells of the invention. Preferably, the compound is a composition comprising the respective fine chemical or a recovered respective fine chemical, in particular, the fine chemical, free or in protein-bound form.

**[0306.1.0.0]** Preferably, the compound is a composition comprising the methionine or a recovered methionine, in particular, the fine chemical, free or in protein-bound form.

**[0307.0.0.0]** The invention also provides chimeric or fusion proteins.

**[0308.0.0.0]** As used herein, an "chimeric protein" or "fusion protein" comprises an polypeptide operatively linked to a polypeptide which does not confer above-mentioned activity, in particular, which does not confer an increase of content of the respective fine chemical in a cell or an organism or a part thereof, if its activity is increased.

**[0309.0.0.0]** In one embodiment, an reference to a " protein (= polypeptide) of the invention" or as indicated in Table II, columns 5 or 7, lines 1 to 5 and/or lines 334 to 338 refers to a polypeptide having an amino acid sequence corresponding to the polypeptide of the invention or used in the process of the invention, whereas a "non-polypeptide of the invention" or "other polypeptide" not being indicated in Table II, columns 5 or 7, lines 1 to 5 and/or lines 334 to 338 refers to a polypeptide having an amino acid sequence corresponding to a protein which is not substantially homologous a polypeptide of the invention, preferably which is not substantially homologous to a polypeptide as indicated in Table II, columns 5 or 7, lines 1 to 5 and/or lines 334 to 338, e.g., a protein which does not confer the activity described herein or annotated or known for as indicated in Table II, column 3, lines 1 to 5 and/or lines 334 to 338, and which is derived from the same or a different organism. In one embodiment, a "non-polypeptide of the invention" or "other polypeptide" not being indicated in Table II, columns 5 or 7, lines 1 to 5 and/or lines 334 to 338 does not confer an increase of the respective fine chemical in an organism or part thereof.

**[0310.0.0.0]** Within the fusion protein, the term "operatively linked" is intended to indicate that the polypeptide of the invention or a polypeptide used in the process of the invention and the "other polypeptide" or a part thereof are fused to each other so that both sequences fulfil the proposed function addicted to the sequence used. The "other polypeptide" can be fused to the N-terminus or C-terminus of the polypeptide of the invention or used in the process of the invention. For example, in one embodiment the fusion protein is a GST-LMRP fusion protein in which the sequences of the polypeptide of the invention or the polypeptide used in the process of the invention are fused to the C-terminus of the GST sequences. Such fusion proteins can facilitate the purification of recombinant polypeptides of the invention or a polypeptide useful in the process of the invention.

**[0311.0.0.0]** In another embodiment, the fusion protein is a polypeptide of the invention or a polypeptide used in the process of the invention containing a heterologous signal sequence at its N-terminus. In certain host cells (e.g., mammalian host cells), expression and/or secretion of a polypeptide of the invention or a polypeptide used in the process of the invention can be increased through use of a heterologous signal sequence. As already mentioned above, targeting sequences, are required for targeting the gene product into specific cell compartment (for a review, see Kermode, Crit. Rev. Plant Sci. 15, 4 (1996) 285-423 and references cited therein), for example into the vacuole, the nucleus, all types of plastids, such as amyloplasts, chloroplasts, chromoplasts, the extracellular space, the mitochondria, the endoplasmic reticulum, elaioplasts, peroxisomes, glycosomes, and other compartments of cells or extracellular. Sequences, which must be mentioned in this context are, in particular, the signal-peptide- or transit-peptide-encoding sequences which are known per se. For example, plastid-transit-peptide-encoding sequences enable the targeting of the expression product into the plastids of a plant cell. Targeting sequences are also known for eukaryotic and to a lower extent for prokaryotic organisms and can advantageously be operable linked with the nucleic acid molecule of the present invention to achieve an expression in one of said compartments or extracellular.

**[0312.0.0.0]** Preferably, a chimeric or fusion protein of the invention is produced by standard recombinant DNA techniques. For example, DNA fragments coding for the different polypeptide sequences are ligated together in-frame in accordance with conventional techniques, for example by employing blunt-ended or stagger-ended termini for ligation, restriction enzyme digestion to provide for appropriate termini, filling-in of cohesive ends as appropriate, alkaline phosphatase treatment to avoid undesirable joining, and enzymatic ligation. The fusion gene can be synthesized by conventional techniques including automated DNA synthesizers. Alternatively, PCR amplification of gene fragments can be carried out using anchor primers, which give rise to complementary overhangs between two consecutive gene fragments which can subsequently be annealed and reamplified to generate a chimeric gene sequence (see, for example, Current Protocols in Molecular Biology, eds. Ausubel et al. John Wiley & Sons: 1992). Moreover, many expression vectors are commercially available that already encode a fusion moiety (e.g., a GST polypeptide). The nucleic acid molecule of the invention or the nucleic acid molecule used in the method of the invention can be cloned into such an expression vector such that the fusion moiety is linked in-frame to the encoded protein.

**[0313.0.0.0]** Furthermore, folding simulations and computer redesign of structural motifs of the protein of the invention can be performed using appropriate computer programs (Olszewski, Proteins 25 (1996), 286-299; Hoffman, Comput. Appl. Biosci. 11 (1995), 675-679). Computer modelling of protein folding can be used for the conformational and energetic analysis of detailed peptide and protein models (Monge, J. Mol. Biol. 247 (1995), 995-1012; Renouf, Adv. Exp. Med. Biol. 376 (1995), 37-45). The appropriate programs can be used for the identification of interactive sites the polypeptide of the invention or polypeptides used in the process of the invention and its substrates or binding factors or other interacting proteins by computer assistant searches for complementary peptide sequences (Fassina, Immunomethods (1994), 114-120). Further appropriate computer systems for the design of protein and peptides are described in the prior art, for example in Berry, Biochem. Soc. Trans. 22 (1994), 1033-1036; Wodak, Ann. N. Y. Acad. Sci. 501 (1987), 1-13; Pabo, Biochemistry 25 (1986), 5987-5991. The results obtained from the above-described computer analysis can be used for, e.g., the preparation of peptidomimetics of the protein of the invention or fragments thereof. Such pseudopeptide analogues of the, natural amino acid sequence of the protein may very efficiently mimic the parent protein (Benkirane, J. Biol. Chem. 271 (1996), 33218-33224). For example, incorporation of easily available achiral Q-amino acid residues into a protein of the invention or a fragment thereof results in the substitution of amide bonds by polymethylene units of an aliphatic chain, thereby providing a convenient strategy for constructing a peptidomimetic (Banerjee, Biopolymers 39 (1996), 769-777).

**[0314.0.0.0]** Superactive peptidomimetic analogues of small peptide hormones in other systems are described in the prior art (Zhang, Biochem. Biophys. Res. Commun. 224 (1996), 327-331). Appropriate peptidomimetics of the protein of the present invention can also be identified by the synthesis of peptidomimetic combinatorial libraries through successive amide alkylation and testing the resulting compounds, e.g., for their binding and immunological properties. Methods for the generation and use of peptidomimetic combinatorial libraries are described in the prior art, for example in Ostresh, Methods in Enzymology 267 (1996), 220-234 and Dorner, Bioorg. Med. Chem. 4 (1996), 709-715.

**[0315.0.0.0]** Furthermore, a three-dimensional and/or crystallographic structure of the protein of the invention can be used for the design of peptidomimetic inhibitors of the biological activity of the protein of the invention (Rose, Biochemistry 35 (1996), 12933-12944; Rutenber, Bioorg. Med. Chem. 4 (1996),1545-1558).

**[0316.0.0.0]** Furthermore, a three-dimensional and/or crystallographic structure of the protein of the invention and the identification of interactive sites the polypeptide of the invention or the polypeptide used in the method of the invention and its substrates or binding factors can be used for the identification or design of mutants with modulated binding or turn over activities. For example, the active centre of the polypeptide of the present invention can be modelled and amino acid residues participating in the catalytic reaction can be modulated to increase or decrease the binding of the substrate to activate or improve the polypeptide. The identification of the active centre and the amino acids involved in the catalytic reaction facilitates the screening for mutants having an increased activity.

**[0317.0.0.0]** The sequences shown in column 5 of the Tables I to IV herein have also been described under their Gene/ORF Locus Name as described in the Table I, II, III or IV, column 3.

**[0318.0.0.0]** In an especially preferred embodiment, the polypeptide according to the invention furthermore also does not have the sequences of those proteins which are encoded by the sequences shown in the known listed Gene/ORF.Locus Names or as described in the Tables, column 3.

**[0319.0.0.0]** One embodiment of the invention also relates to an antibody, which binds specifically to the polypeptide according to the invention or parts, i.e. specific fragments or epitopes of such a protein.

**[0320.0.0.0]** The antibodies of the invention can be used to identify and isolate the polypeptide according to the invention and encoding genes in any organism, preferably plants, prepared in plants described herein. These antibodies can be monoclonal antibodies, polyclonal antibodies or synthetic antibodies as well as fragments of antibodies, such as Fab, Fv or scFv fragments etc. Monoclonal antibodies can be prepared, for example, by the techniques as originally described in Köhler and Milstein, Nature 256 (1975), 495, and Galfr6, Meth. Enzymol. 73 (1981), 3, which comprise the fusion of mouse myeloma cells to spleen cells derived from immunized mammals.

**[0321.0.0.0]** Furthermore, antibodies or fragments thereof to the aforementioned peptides can be obtained by using methods, which are described, e.g., in Harlow and Lane "Antibodies, A Laboratory Manual", CSH Press, Cold Spring Harbor, 1988. These antibodies can be used, for example, for the immunoprecipitation and immunolocalization of proteins according to the invention as well as for the monitoring of the synthesis of such proteins, for example, in recombinant organisms, and for the identification of compounds interacting with the protein according to the invention. For example, surface plasmon resonance as employed in the BlAcore system can be used to increase the efficiency of phage antibodies selections, yielding a high increment of affinity from a single library of phage antibodies, which bind to an epitope of the protein of the invention (Schier, Human Antibodies Hybridomas 7 (1996), 97-105; Malmborg, J. Immunol. Methods 183 (1995), 7-13). In many cases, the binding phenomena of antibodies to antigens are equivalent to other ligand/anti-ligand binding.

**[0322.0.0.0]** In one embodiment, the present invention relates to an antisense nucleic acid molecule comprising the complementary sequence of the nucleic acid molecule of the present invention.

**[0323.0.0.0]** Methods to modify the expression levels and/or the activity are known to persons skilled in the art and include for instance overexpression, co-suppression, the use of ribozymes, sense and anti-sense strategies or other gene silencing approaches like RNA interference (RNAi) or promoter methylation. "Sense strand" refers to the strand of a double-stranded DNA molecule that is homologous to an mRNA transcript thereof. The "anti-sense strand" contains an inverted sequence, which is complementary to that of the "sense strand".
In addition the expression levels and/or the activity can be modified by the introduction of mutations in the regulatory or coding regions of the nucleic acids of the invention. Furthermore antibodies can be expressed which specifically binds to a polypeptide of interest and thereby blocks it activity. The protein-binding factors can, for example, also be aptamers [Famulok M and Mayer G (1999) Curr. Top Microbiol. Immunol. 243: 123-36] or antibodies or antibody fragments or single-chain antibodies. Obtaining these factors has been described, and the skilled worker is familiar therewith. For example, a cytoplasmic scFv antibody has been employed for modulating activity of the phytochrome A protein in genetically modified tobacco plants [Owen M et al. (1992) Biotechnology (NY) 10(7): 790-794; Franken E et al. (1997) Curr. Opin. Biotechnol. 8(4): 411-416; Whitelam (1996) Trend Plant Sci. 1: 286-272].

**[0324.0.0.0]** An "antisense" nucleic acid molecule comprises a nucleotide sequence, which is complementary to a "sense" nucleic acid molecule encoding a protein, e.g., complementary to the coding strand of a double-stranded cDNA molecule or complementary to an encoding mRNA sequence. Accordingly, an antisense nucleic acid molecule can bond via hydrogen bonds to a sense nucleic acid molecule. The antisense nucleic acid molecule can be complementary to an entire coding strand of a nucleic acid molecule conferring the expression of the polypeptide of the invention or used in the process of the present invention, as the nucleic acid molecule of the invention or the nucleic acid molecule used in the method of the invention coding strand, or to only a portion thereof. Accordingly, an antisense nucleic acid molecule can be antisense to a "coding region" of the coding strand of a nucleotide sequence of a nucleic acid molecule of the present invention. The term "coding region" refers to the region of the nucleotide sequence comprising codons, which are translated into amino acid residues. Further, the antisense nucleic acid molecule is antisense to a "noncoding region" of the coding strand of a nucleotide sequence encoding the polypeptide of the invention or a polypeptide used in the process of the invention. The term "noncoding region" refers to 5' and 3' sequences which flank the coding region that are not translated into a polypeptide, i.e., also referred to as 5' and 3' untranslated regions (5'-UTR or 3'-UTR).

**[0325.0.0.0]** Given the coding strand sequences encoding the polypeptide of the present invention antisense nucleic acid molecules of the invention can be designed according to the rules of Watson and Crick base pairing.

**[0326.0.0.0]** The antisense nucleic acid molecule can be complementary to the entire coding region of the mRNA encoding the nucleic acid molecule to the invention or used in the process of the present invention, but can also be an oligonucleotide which is antisense to only a portion of the coding or noncoding region of said mRNA. For example, the antisense oligonucleotide can be complementary to the region surrounding the translation start site of said mRNA. An antisense oligonucleotide can be, for example, about 5, 10, 15, 20, 25, 30, 35, 40, 45, 50, 100 or 200 nucleotides in length. An antisense nucleic acid molecule of the invention or the nucleic acid molecule used in the method of the invention can be constructed using chemical synthesis and enzymatic ligation reactions using procedures known in the art. For example, an antisense nucleic acid molecule (e.g., an antisense oligonucleotide) can be chemically synthesized using naturally occurring nucleotides or variously modified nucleotides designed to increase the biological stability of the molecules or to increase the physical stability of the duplex formed between the antisense and sense nucleic acids, e.g., phosphorothioate derivatives and acridine substituted nucleotides can be used. Examples of modified nucleotides which can be used to generate the antisense nucleic acid include 5-fluorouracil, 5-bromouracil, 5-chlorouracil, 5-iodouracil, hypoxanthine, xanthine, 4-acetylcytosine, 5-(carboxyhydroxylmethyl) uracil, 5-carboxymethylaminomethyl-2-thiouridine, 5-carboxymethylaminomethyluracil, dihydrouracil, beta-D-galactosylqueosine, inosine, N6-isopentenyladenine, 1-methylguanine, 1-methylinosine, 2,2-dimethylguanine, 2-methyladenine, 2-methylguanine, 3-methylcytosine, 5-methylcytosine, N6-adenine, 7-methylguanine, 5-methylaminomethyluracil, 5-methoxyaminomethyl-2-thiouracil, beta-D-mannosylqueosine, 5'-methoxycarboxymethyluracil, 5-methoxyuracil, 2-methylthio-N6-isopentenyladenine, uracil-5-oxyacetic acid (v), wybutoxosine, pseudouracil, queosine, 2-thiocytosine, 5-methyl-2-thiouracil, 2-thiouracil, 4-thiouracil, 5-methyluracil, uracil-5-oxyacetic acid methylester, uracil-5-oxyacetic acid (v), 5-methyl-2-thiouracil, 3-(3-amino-3-N-2-carboxypropyl) uracil, (acp3)w, and 2,6-diaminopurine. Alternatively, the antisense nucleic acid can be produced biologically using an expression vector into which a nucleic acid molecule has been subcloned in an antisense orientation (i.e., RNA transcribed from the inserted nucleic acid molecule will be of an antisense orientation to a target nucleic acid molecule of interest, described further in the following subsection).

**[0327.0.0.0]** The antisense nucleic acid molecules of the invention are typically administered to a cell or generated *in situ* such that they hybridize with or bind to cellular mRNA and/or genomic DNA encoding a polypeptide of the invention or the polypeptide used in the method of the invention having aforementioned the respective fine chemical increasing activity to thereby inhibit expression of the protein, e.g., by inhibiting transcription and/or translation.

**[0328.0.0.0]** The hybridization can be by conventional nucleotide complementarity to form a stable duplex, or, for example, in the case of an antisense nucleic acid molecule which binds to DNA duplexes, through specific interactions in the major groove of the double helix. The antisense nucleic acid molecule can also be delivered to cells using the vectors described herein. To achieve sufficient intracellular concentrations of the antisense molecules, vector in which the antisense nucleic acid molecule is placed under the control of a strong prokaryotic, viral, or eukaryotic including plant promoters are preferred.

**[0329.0.0.0]** In a further embodiment, the antisense nucleic acid molecule of the invention or the nucleic acid molecule used in the method of the invention can be an α-anomeric nucleic acid molecule. An α-anomeric nucleic acid molecule forms specific double-stranded hybrids with complementary RNA in which, contrary to the usual units, the strands run parallel to each other (Gaultier et al. (1987) Nucleic Acids. Res. 15:6625-6641). The antisense nucleic acid molecule can also comprise a 2'-o-methyl-ribonucleotide (Inoue et al. (1987) Nucleic Acids Res. 15:6131-6148) or a chimeric RNA-DNA analogue (Inoue et al. (1987) FEBS Lett. 215:327-330).

**[0330.0.0.0]** Further the antisense nucleic acid molecule of the invention or the nucleic acid molecule used in the method of the invention can be also a ribozyme.
Ribozymes are catalytic RNA molecules with ribonuclease activity, which are capable of cleaving a single-stranded nucleic acid, such as an mRNA, to which they have a complementary region. Thus, ribozymes (e.g., hammerhead ribozymes (described in Haselhoff and Gerlach (1988) Nature 334:585-591)) can be used to catalytically cleave mRNA transcripts encoding the polypeptide of the invention or the polypeptide used in the method of the invention to thereby inhibit translation of said mRNA. A ribozyme having specificity for a nucleic acid molecule encoding the polypeptide of the invention or used in the process of the invention can be designed based upon the nucleotide sequence of the nucleic acid molecule of the invention or the nucleic acid molecule used in the method of the invention or coding a protein used in the process of the invention or on the basis of a heterologous sequence to be isolated according to methods taught in this invention. For example, a derivative of a *Tetrahymena* L-19 IVS RNA can be constructed in which the nucleotide sequence of the active site is complementary to the nucleotide sequence to be cleaved in an encoding mRNA. See, e.g., Cech et al. U.S. Patent No. 4,987,071 and Cech et al. U.S. Patent No. 5,116,742. Alternatively, mRNA encoding the polypeptide of the invention or a polypeptide used in the process of the invention can be used to select a catalytic RNA having a specific ribonuclease activity from a pool of RNA molecules. See, e.g., Bartel, D. and Szostak, J.W. (1993) Science 261:1411-1418.

**[0331.0.0.0]** The antisense molecule of the present invention comprises also a nucleic acid molecule comprising a nucleotide sequences complementary to the regulatory region of an nucleotide sequence encoding the natural occurring polypeptide of the invention or the polypeptide used in the method of the invention, e.g. the polypeptide sequences shown in the sequence listing, or identified according to the methods described herein, e.g., its promoter and/or enhancers, e.g. to form triple helical structures that prevent transcription of the gene in target cells. See generally, Helene, C. (1991) Anticancer Drug Des. 6(6): 569-84; Helene, C. et al. (1992) Ann. N.Y. Acad. Sci. 660:27-36; and Maher, L.J. (1992) Bioassays 14(12): 807-15.

**[0332.0.0.0]** Furthermore the present invention relates to a double stranded RNA molecule capable for the reduction or inhibition of the activity of the gene product of a gene encoding the polypeptide of the invention, a polypeptide used in the process of the invention, the nucleic acid molecule of the invention or a nucleic acid molecule used in the process of the invention encoding.

**[0333.0.0.0]** The method of regulating genes by means of double-stranded RNA ("double-stranded RNA interference"; dsRNAi) has been described extensively for animal, yeast, fungi and plant organisms such as Neurospora, zebrafish, Drosophila, mice, planaria, humans, Trypanosoma, petunia or Arabidopsis (for example Matzke MA et al. (2000) Plant Mol. Biol. 43: 401-415; Fire A. et al. (1998) Nature 391: 806-811; WO 99/32619; WO 99/53050; WO 00/68374; WO 00/44914; WO 00/44895; WO 00/49035; WO 00/63364). In addition RNAi is also documented as an advantageously tool for the repression of genes in bacteria such as E. coli for example by Tchurikov et al. [J. Biol. Chem., 2000, 275 (34): 26523 - 26529]. Fire et al. named the phenomenon RNAi for "RNA interference". The techniques and methods described in the above references are expressly referred to. Efficient gene suppression can also be observed in the case of transient expression or following transient transformation, for example as the consequence of a biolistic transformation (Schweizer P et al. (2000) Plant J 2000 24: 895-903). dsRNAi methods are based on the phenomenon that the simultaneous introduction of complementary strand and counterstrand of a gene transcript brings about highly effective suppression of the expression of the gene in question. The resulting phenotype is very similar to that of an analogous knock-out mutant (Waterhouse PM et al. (1998) Proc. Natl. Acad. Sci. USA 95: 13959-64).

**[0334.0.0.0]** Tuschl et al. [Gens Dev., 1999, 13 (24): 3191 - 3197] was able to show that the efficiency of the RNAi method is a function of the length of the duplex, the length of the 3'-end overhangs, and the sequence in these overhangs. Based on the work of Tuschl et al. the following guidelines can be given to the skilled worker: To achieve good results the 5' and 3' untranslated regions of the used nucleic acid sequence and regions close to the start codon should be avoided as this regions are richer in regulatory protein binding sites and interactions between RNAi sequences and such regulatory proteins might lead to undesired interactions. Preferably a region of the used mRNA is selected, which is 50 to 100 nt (= nucleotides or bases) downstream of the AUG start codon. Only dsRNA (= double-stranded RNA) sequences from exons are useful for the method, as sequences from introns have no effect. The G/C content in this region should be greater than 30% and less than 70% ideally around 50%. A possible secondary structure of the target mRNA is less important for the effect of the RNAi method.

**[0335.0.0.0]** The dsRNAi method has proved to be particularly effective and advantageous for reducing the expression of a nucleic acid sequences as indicated in Table I, columns 5 or 7, lines 1 to 5 and/or lines 334 to 338 and/or homologs thereof. As described inter alia in WO 99/32619, dsRNAi approaches are clearly superior to traditional antisense approaches. The invention therefore furthermore relates to double-stranded RNA molecules (dsRNA molecules) which, when introduced into an organism, advantageously into a plant (or a cell, tissue, organ or seed derived there from), bring about altered metabolic activity by the reduction in the expression of a nucleic acid sequences as indicated in Table I, columns 5 or 7, lines 1 to 5 and/or lines 334 to 338 and/or homologs thereof. In a double-stranded RNA molecule for reducing the expression of an protein encoded by a nucleic acid sequence of one of the sequences as indicated in Table I, columns 5 or 7, lines 1 to 5 and/or lines 334 to 338 and/or homologs thereof, one of the two RNA strands is essentially identical to at least part of a nucleic acid sequence, and the respective other RNA strand is essentially identical to at least part of the complementary strand of a nucleic acid sequence.

**[0336.0.0.0]** The term "essentially identical" refers to the fact that the dsRNA sequence may also include insertions, deletions and individual point mutations in comparison to the target sequence while still bringing about an effective reduction in expression. Preferably, the homology as defined above amounts to at least 30%, preferably at least 40%, 50%, 60%, 70% or 80%, very especially preferably at least 90%, most preferably 100%, between the "sense" strand of an inhibitory dsRNA and a part-segment of a nucleic acid sequence of the invention (or between the "antisense" strand and the complementary strand of a nucleic acid sequence, respectively). The part-segment amounts to at least 10 bases, preferably at least 17, 18, 19, 20, 21, 22, 23, 24, 25, 26, 27, 28, 29 or 30 bases, especially preferably at least 40, 50, 60, 70, 80 or 90 bases, very especially preferably at least 100, 200, 300 or 400 bases, most preferably at least 500, 600, 700, 800, 900 or more bases or at least 1000 or 2000 bases or more in length. In another preferred embodiment of the invention the part-segment amounts to 17, 18, 19, 20, 21, 22, 23, 24, 25, 26 or 27 bases, preferably to 20, 21, 22, 23, 24 or 25 bases. These short sequences are preferred in animals and plants. The longer sequences preferably between 200 and 800 bases are preferred in non-mammalian animals, preferably in invertebrates, in yeast, fungi or bacteria, but they are also useable in plants. Long double-stranded RNAs are processed in the organisms into many siRNAs (= small/short interfering RNAs) for example by the protein Dicer, which is a ds-specific Rnase III enzyme. As an alternative, an "essentially identical" dsRNA may also be defined as a nucleic acid sequence, which is capable of hybridizing with part of a gene transcript (for example in 400 mM NaCl, 40 mM PIPES pH 6.4, 1 mM EDTA at 50°C or 70°C for 12 to 16 h).

**[0337.0.0.0]** The dsRNA may consist of one or more strands of polymerized ribonucleotides. Modification of both the sugar-phosphate backbone and of the nucleosides may furthermore be present. For example, the phosphodiester bonds of the natural RNA can be modified in such a way that they encompass at least one nitrogen or sulfur heteroatom. Bases may undergo modification in such a way that the activity of, for example, adenosine deaminase is restricted. These and other modifications are described herein below in the methods for stabilizing antisense RNA.

**[0338.0.0.0]** The dsRNA can be prepared enzymatically; it may also be synthesized chemically, either in full or in part.

**[0339.0.0.0]** The double-stranded structure can be formed starting from a single, self-complementary strand or starting from two complementary strands. In a single, self-complementary strand, "sense" and "antisense" sequence can be linked by a linking sequence ("linker") and form for example a hairpin structure. Preferably, the linking sequence may take the form of an intron, which is spliced out following dsRNA synthesis. The nucleic acid sequence encoding a dsRNA may contain further elements such as, for example, transcription termination signals or polyadenylation signals. If the two strands of the dsRNA are to be combined in a cell or an organism advantageously in a plant, this can be brought about in a variety of ways.

**[0340.0.0.0]** Formation of the RNA duplex can be initiated either outside the cell or within the cell. As shown in WO 99/53050, the dsRNA may also encompass a hairpin structure, by linking the "sense" and "antisense" strands by a "linker" (for example an intron). The self-complementary dsRNA structures are preferred since they merely require the expression of a construct and always encompass the complementary strands in an equimolar ratio.

**[0341.0.0.0]** The expression cassettes encoding the "antisense" or the "sense" strand of the dsRNA or the self-complementary strand of the dsRNA are preferably inserted into a vector and stably inserted into the genome of a plant, using the methods described herein below (for example using selection markers), in order to ensure permanent expression of the dsRNA.

**[0342.0.0.0]** The dsRNA can be introduced using an amount which makes possible at least one copy per cell. A larger amount (for example at least 5, 10, 100, 500 or 1 000 copies per cell) may bring about more efficient reduction.

**[0343.0.0.0]** As has already been described, 100 % sequence identity between the dsRNA and a gene transcript of a nucleic acid sequence as indicated in Table I, columns 5 or 7, lines 1 to 5 and/or lines 334 to 338 or it's homolog is not necessarily required in order to bring about effective reduction in the expression. The advantage is, accordingly, that the method is tolerant with regard to sequence deviations as may be present as a consequence of genetic mutations, polymorphisms or evolutionary divergences. Thus, for example, using the dsRNA, which has been generated starting from a sequence as indicated in Table I, columns 5 or 7, lines 1 to 5 and/or lines 334 to 338 or homologs thereof of the one organism, may be used to suppress the corresponding expression in another organism.

**[0344.0.0.0]** Due to the high degree of sequence homology between sequences from various organisms (e. g. plants), allows the conclusion that these proteins may be conserved to a high degree within, for example other, plants, it is optionally possible that the expression of a dsRNA derived from one of the disclosed sequences as shown herein or homologs thereof should also have has an advantageous effect in other plant species. Preferably the consensus sequences shown herein can be used for the construction of useful dsRNA molecules.

**[0345.0.0.0]** The dsRNA can be synthesized either in vivo or in vitro. To this end, a DNA sequence encoding a dsRNA can be introduced into an expression cassette under the control of at least one genetic control element (such as, for example, promoter, enhancer, silencer, splice donor or splice acceptor or polyadenylation signal). Suitable advantageous constructs are described herein below. Polyadenylation is not required, nor do elements for initiating translation have to be present.

**[0346.0.0.0]** A dsRNA can be synthesized chemically or enzymatically. Cellular RNA polymerases or bacteriophage RNA polymerases (such as, for example T3, T7 or SP6 RNA polymerase) can be used for this purpose. Suitable methods for the in-vitro expression of RNA are described (WO 97/32016; US 5,593,874; US 5,698,425, US 5,712,135, US 5,789,214, US 5,804,693). Prior to introduction into a cell, tissue or organism, a dsRNA which has been synthesized in vitro either chemically or enzymatically can be isolated to a higher or lesser degree from the reaction mixture, for example by extraction, precipitation, electrophoresis, chromatography or combinations of these methods. The dsRNA can be introduced directly into the cell or else be applied extracellularly (for example into the interstitial space).

**[0347.0.0.0]** Advantageously the RNAi method leads to only a partial loss of gene function and therefore enables the skilled worker to study a gene dose effect in the desired organism and to fine tune the process of the invention. Furthermore it enables a person skilled in the art to study multiple functions of a gene.

**[0348.0.0.0]** Stable transformation of the plant with an expression construct, which brings about the expression of the dsRNA is preferred, however. Suitable methods are described herein below.

**[0349.0.0.0]** A further embodiment of the invention also relates to a method for the generation of a transgenic host or host cell, e.g. a eukaryotic or prokaryotic cell, preferably a transgenic microorganism, a transgenic plant cell or a transgenic plant tissue or a transgenic plant, which comprises introducing, into the plant, the plant cell or the plant tissue, the nucleic acid construct according to the invention, the vector according to the invention, or the nucleic acid molecule according to the invention.

**[0350.0.0.0]** A further embodiment of the invention also relates to a method for the transient generation of a host or host cell, eukaryotic or prokaryotic cell, preferably a transgenic microorganism, a transgenic plant cell or a transgenic plant tissue or a transgenic plant, which comprises introducing, into the plant, the plant cell or the plant tissue, the nucleic acid construct according to the invention, the vector according to the invention, the nucleic acid molecule characterized herein as being contained in the nucleic acid construct of the invention or the nucleic acid molecule according to the invention, whereby the introduced nucleic acid molecules, nucleic acid construct and/or vector is not integrated into the genome of the host or host cell. Therefore the transformants are not stable during the propagation of the host in respect of the introduced nucleic acid molecules, nucleic acid construct and/or vector.

**[0351.0.0.0]** In the process according to the invention, transgenic organisms are also to be understood as meaning - if they take the form of plants - plant cells, plant tissues, plant organs such as root, shoot, stem, seed, flower, tuber or leaf, or intact plants which are grown for the production of the respective fine chemical.

**[0352.0.0.0]** Growing is to be understood as meaning for example culturing the transgenic plant cells, plant tissue or plant organs on or in a nutrient medium or the intact plant on or in a substrate, for example in hydroponic culture, potting compost or on a field soil.

**[0353.0.0.0]** In a further advantageous embodiment of the process, the nucleic acid molecules can be expressed in single-celled plant cells (such as algae), see Falciatore et al., 1999, Marine Biotechnology 1 (3): 239-251 and references cited therein, and plant cells from higher plants (for example spermatophytes such as crops). Examples of plant expression vectors encompass those which are described in detail herein or in: Becker, D. [(1992) Plant Mol. Biol. 20:1195-1197] and Bevan, M.W. [(1984), Nucl. Acids Res. 12:8711-8721; Vectors for Gene Transfer in Higher Plants; in: Transgenic Plants, Vol. 1, Engineering and Utilization, Ed.: Kung and R. Wu, Academic Press, 1993, pp. 15-38]. An overview of binary vectors and their use is also found in Hellens, R. [(2000), Trends in Plant Science, Vol. 5 No.10, 446-451.

**[0354.0.0.0]** Vector DNA can be introduced into prokaryotic or eukaryotic cells via conventional transformation or transfection techniques. The terms "transformation" and "transfection" include conjugation and transduction and, as used in the present context, are intended to encompass a multiplicity of prior-art methods for introducing foreign nucleic acid molecules (for example DNA) into a host cell, including calcium phosphate coprecipitation or calcium chloride coprecipitation, DEAE-dextran-mediated transfection, PEG-mediated transfection, lipofection, natural competence, chemically mediated transfer, electroporation or particle bombardment. Suitable methods for the transformation or transfection of host cells, including plant cells, can be found in Sambrook et al. (Molecular Cloning: A Laboratory Manual., 2nd Ed., Cold Spring Harbor Laboratory, Cold Spring Harbor Laboratory Press, Cold Spring Harbor, NY, 1989) and in other laboratory handbooks such as Methods in Molecular Biology, 1995, Vol. 44, Agrobacterium protocols, Ed.: Gartland and Davey, Humana Press, Totowa, New Jersey.

**[0355.0.0.0]** The above-described methods for the transformation and regeneration of plants from plant tissues or plant cells are exploited for transient or stable transformation of plants. Suitable methods are the transformation of protoplasts by polyethylene-glycol-induced DNA uptake, the biolistic method with the gene gun - known as the particle bombardment method -, electroporation, the incubation of dry embryos in DNA-containing solution, microinjection and the Agrobacterium-mediated gene transfer. The abovementioned methods are described for example in B. Jenes, Techniques for Gene Transfer, in: Transgenic Plants, Vol. 1, Engineering and Utilization, edited by S.D. Kung and R. Wu, Academic Press (1993) 128-143 and in Potrykus Annu. Rev. Plant Physiol. Plant Molec. Biol. 42 (1991) 205-225. The construct to be expressed is preferably cloned into a vector, which is suitable for transforming Agrobacterium tumefaciens, for example pBin19 (Bevan, Nucl. Acids Res. 12 (1984) 8711). Agrobacteria transformed with such a vector can then be used in the known manner for the transformation of plants, in particular crop plants, such as, for example, tobacco plants, for example by bathing scarified leaves or leaf segments in an agrobacterial solution and subsequently culturing them in suitable media. The transformation of plants with Agrobacterium tumefaciens is described for example by Höfgen and Willmitzer in Nucl. Acid Res. (1988) 16, 9877 or known from, inter alia, F.F. White, Vectors for Gene Transfer in Higher Plants; in Transgenic Plants, Vol. 1, Engineering and Utilization, edited by S.D. Kung and R. Wu, Academic Press, 1993, pp. 15-38.

**[0356.0.0.0]** To select for the successful transfer of the nucleic acid molecule, vector or nucleic acid construct of the invention according to the invention into a host organism, it is advantageous to use marker genes as have already been described above in detail. It is known of the stable or transient integration of nucleic acids into plant cells that only a minority of the cells takes up the foreign DNA and, if desired, integrates it into its genome, depending on the expression vector used and the transfection technique used. To identify and select these integrants, a gene encoding for a selectable marker (as described above, for example resistance to antibiotics) is usually introduced into the host cells together with the gene of interest. Preferred selectable markers in plants comprise those, which confer resistance to an herbicide such as glyphosate or gluphosinate. Other suitable markers are, for example, markers, which encode genes involved in biosynthetic pathways of, for example, sugars or amino acids, such as β-galactosidase, ura3 or ilv2. Markers, which encode genes such as luciferase, gfp or other fluorescence genes, are likewise suitable. These markers and the aforementioned markers can be used in mutants in whom these genes are not functional since, for example, they have been deleted by conventional methods. Furthermore, nucleic acid molecules, which encode a selectable marker, can be introduced into a host cell on the same vector as those, which encode the polypeptides of the invention or used in the process or else in a separate vector. Cells which have been transfected stably with the nucleic acid introduced can be identified for example by selection (for example, cells which have integrated the selectable marker survive whereas the other cells die).

**[0357.0.0.0]** Since the marker genes, as a rule specifically the gene for resistance to antibiotics and herbicides, are no longer required or are undesired in the transgenic host cell once the nucleic acids have been introduced successfully, the process according to the invention for introducing the nucleic acids advantageously employs techniques which enable the removal, or excision, of these marker genes. One such a method is what is known as cotransformation. The cotransformation method employs two vectors simultaneously for the transformation, one vector bearing the nucleic acid according to the invention and a second bearing the marker gene(s). A large proportion of transformants receives or, in the case of plants, comprises (up to 40% of the transformants and above), both vectors. In case of transformation with Agrobacteria, the transformants usually receive only a part of the vector, the sequence flanked by the T-DNA, which usually represents the expression cassette. The marker genes can subsequently be removed from the transformed plant by performing crosses. In another method, marker genes integrated into a transposon are used for the transformation together with desired nucleic acid (known as the Ac/Ds technology). The transformants can be crossed with a transposase resource or the transformants are transformed with a nucleic acid construct conferring expression of a transposase, transiently or stable. In some cases (approx. 10%), the transposon jumps out of the genome of the host cell once transformation has taken place successfully and is lost. In a further number of cases, the transposon jumps to a different location. In these cases, the marker gene must be eliminated by performing crosses. In microbiology, techniques were developed which make possible, or facilitate, the detection of such events. A further advantageous method relies on what are known as recombination systems, whose advantage is that elimination by crossing can be dispensed with. The best-known system of this type is what is known as the Cre/lox system. Cre1 is a recombinase, which removes the sequences located between the IoxP sequences. If the marker gene is integrated between the IoxP sequences, it is removed, once transformation has taken place successfully, by expression of the recombinase. Further recombination systems are the HIN/HIX, FLP/FRT and REP/STB system (Tribble et al., J. Biol. Chem., 275, 2000: 22255-22267; Velmurugan et al., J. Cell Biol., 149, 2000: 553-566). A site-specific integration into the plant genome of the nucleic acid sequences according to the invention is possible. Naturally, these methods can also be applied to microorganisms such as yeast, fungi or bacteria.

**[0358.0.0.0]** Agrobacteria transformed with an expression vector according to the invention may also be used in the manner known per se for the transformation of plants such as experimental plants like Arabidopsis or crop plants, such as, for example, cereals, maize, oats, rye, barley, wheat, soya, rice, cotton, sugarbeet, canola, sunflower, flax, hemp, potato, tobacco, tomato, carrot, bell peppers, oilseed rape, tapioca, cassava, arrow root, tagetes, alfalfa, lettuce and the various tree, nut, and grapevine species, in particular oil-containing crop plants such as soya, peanut, castor-oil plant, sunflower, maize, cotton, flax, oilseed rape, coconut, oil palm, safflower (Carthamus tinctorius) or cocoa beans, for example by bathing scarified leaves or leaf segments in an agrobacterial solution and subsequently growing them in suitable media.

**[0359.0.0.0]** In addition to the transformation of somatic cells, which then has to be regenerated into intact plants, it is also possible to transform the cells of plant meristems and in particular those cells which develop into gametes. In this case, the transformed gametes follow the natural plant development, giving rise to transgenic plants. Thus, for example, seeds of Arabidopsis are treated with agrobacteria and seeds are obtained from the developing plants of which a certain proportion is transformed and thus transgenic (Feldman, KA and Marks MD (1987). Mol Gen Genet 208:274-289; Feldmann K (1992). In: C Koncz, N-H Chua and J Shell, eds, Methods in Arabidopsis Research. Word Scientific, Singapore, pp. 274-289). Alternative methods are based on the repeated removal of the influorescences and incubation of the excision site in the center of the rosette with transformed agrobacteria, whereby transformed seeds can likewise be obtained at a later point in time (Chang (1994). Plant J. 5: 551-558; Katavic (1994). Mol Gen Genet, 245: 363-370). However, an especially effective method is the vacuum infiltration method with its modifications such as the "floral dip" method. In the case of vacuum infiltration of Arabidopsis, intact plants under reduced pressure are treated with an agrobacterial suspension (Bechthold, N (1993). C R Acad Sci Paris Life Sci, 316: 1194-1199), while in the case of the"floral dip" method the developing floral tissue is incubated briefly with a surfactant-treated agrobacterial suspension (Clough, SJ und Bent, AF (1998). The Plant J. 16, 735-743). A certain proportion of transgenic seeds are harvested in both cases, and these seeds can be distinguished from nontransgenic seeds by growing under the above-described selective conditions. In addition the stable transformation of plastids is of advantages because plastids are inherited maternally is most crops reducing or eliminating the risk of transgene flow through pollen. The transformation of the chloroplast genome is generally achieved by a process, which has been schematically displayed in Klaus et al., 2004 (Nature Biotechnology 22(2), 225-229). Briefly the sequences to be transformed are cloned together with a selectable marker gene between flanking sequences homologous to the chloroplast genome. These homologous flanking sequences direct site specific integration into the plastome. Plastidal transformation has been described for many different plant species and an overview can be taken from Bock (2001) Transgenic plastids in basic research and plant biotechnology. J Mol Biol. 2001 Sep 21; 312 (3): 425-38 or Maliga, P (2003) Progress towards commercialization of plastid transformation technology. Trends Biotechnol. 21, 20-28. Further biotechnological progress has recently been reported in form of marker free plastid transformants, which can be produced by a transient cointegrated maker gene (Klaus et al., 2004, Nature Biotechnology 22 (2), 225-229).

**[0360.0.0.0]** The genetically modified plant cells can be regenerated via all methods with which the skilled worker is familiar. Suitable methods can be found in the abovementioned publications by S.D. Kung and R. Wu, Potrykus or Höfgen and Willmitzer.

**[0361.0.0.0]** Accordingly, the present invention thus also relates to a plant cell comprising the nucleic acid construct according to the invention, the nucleic acid molecule according to the invention or the vector according to the invention.

**[0362.0.0.0]** Accordingly the present invention relates to any cell transgenic for any nucleic acid characterized as part of the invention, e.g. conferring the increase of the respective fine chemical in a cell or an organism or a part thereof, e.g. the nucleic acid molecule of the invention or the nucleic acid molecule used in the method of the invention, the nucleic acid construct of the invention, the antisense molecule of the invention, the vector of the invention or a nucleic acid molecule encoding the polypeptide of the invention or the polypeptide used in the method of the invention, e.g. the polypeptide as indicated in Table II, columns 5 or 7, lines 1 to 5 and/or lines 334 to 338. Due to the above mentioned activity the respective fine chemical content in a cell or an organism is increased. For example, due to modulation or manipulation, the cellular activity of the polypeptide of the invention or the polypeptide used in the method of the invention or the nucleic acid molecule of the invention or the nucleic acid molecule used in the method of the invention is increased, e.g. due to an increased expression or specific activity of the subject matters of the invention in a cell or an organism or a part thereof. In one embodiment, transgenic for a polypeptide having an activity of a polypeptide as indicated in Table II, columns 5 or 7, lines 1 to 5 and/or lines 334 to 338 means herein that due to modulation or manipulation of the genome, an activity as annotated for a polypeptide as indicated in Table II, column 3, lines 1 to 5 and/or lines 334 to 338, e.g. having a sequence as indicated in Table II, columns 5 or 7, lines 1 to 5 and/or lines 334 to 338,is increased in a cell or an organism or a part thereof. Examples are described above in context with the process of the invention

**[0363.0.0.0]** "Transgenic", for example regarding a nucleic acid molecule, an nucleic acid construct or a vector comprising said nucleic acid molecule or an organism transformed with said nucleic acid molecule, nucleic acid construct or vector, refers to all those subjects originating by recombinant methods in which either
a) the nucleic acid sequence, or
b) a genetic control sequence linked operably to the nucleic acid sequence, for example a promoter, or
c) (a) and (b)
are not located in their natural genetic environment or have been modified by recombinant methods, an example of a modification being a substitution, addition, deletion, inversion or insertion of one or more nucleotide residues. Natural genetic environment refers to the natural chromosomal locus in the organism of origin, or to the presence in a genomic library. In the case of a genomic library, the natural genetic environment of the nucleic acid sequence is preferably retained, at least in part. The environment flanks the nucleic acid sequence at least at one side and has a sequence of at least 50 bp, preferably at least 500 bp, especially preferably at least 1000 bp, very especially preferably at least 5000 bp, in length.

**[0364.0.0.0]** A naturally occurring expression cassette - for example the naturally occurring combination of a promoter of a polypeptide of the invention with the corresponding protein-encoding sequence - becomes a transgenic expression cassette when it is modified by non-natural, synthetic "artificial" methods such as, for example, mutagenization. Such methods have been described (US 5,565,350; WO 00/15815; also see above).

**[0365.0.0.0]** Further, the plant cell, plant tissue or plant can also be transformed such that further enzymes and proteins are (over)expressed which expression supports an increase of the respective fine chemical.

**[0366.0.0.0]** However, transgenic also means that the nucleic acids according to the invention are located at their natural position in the genome of an organism, but that the sequence has been modified in comparison with the natural sequence and/or that the regulatory sequences of the natural sequences have been modified. Preferably, transgenic/recombinant is to be understood as meaning the transcription of the nucleic acids used in the process according to the invention occurs at a non-natural position in the genome, that is to say the expression of the nucleic acids is homologous or, preferably, heterologous. This expression can be transiently or of a sequence integrated stably into the genome.

**[0367.0.0.0]** The term "transgenic plants" used in accordance with the invention also refers to the progeny of a transgenic plant, for example the T₁, T₂, T₃ and subsequent plant generations or the BC₁, BC₂, BC₃ and subsequent plant generations. Thus, the transgenic plants according to the invention can be raised and selfed or crossed with other individuals in order to obtain further transgenic plants according to the invention. Transgenic plants may also be obtained by propagating transgenic plant cells vegetatively. The present invention also relates to transgenic plant material, which can be derived from a transgenic plant population according to the invention. Such material includes plant cells and certain tissues, organs and parts of plants in all their manifestations, such as seeds, leaves, anthers, fibers, tubers, roots, root hairs, stems, embryo, calli, cotelydons, petioles, harvested material, plant tissue, reproductive tissue and cell cultures, which are derived from the actual transgenic plant and/or can be used for bringing about the transgenic plant.

**[0368.0.0.0]** Any transformed plant obtained according to the invention can be used in a conventional breeding scheme or in *in vitro* plant propagation to produce more transformed plants with the same characteristics and/or can be used to introduce the same characteristic in other varieties of the same or related species. Such plants are also part of the invention. Seeds obtained from the transformed plants genetically also contain the same characteristic and are part of the invention. As mentioned before, the present invention is in principle applicable to any plant and crop that can be transformed with any of the transformation method known to those skilled in the art.

**[0369.0.0.0]** In an especially preferred embodiment, the organism, the host cell, plant cell, plant, microorganism or plant tissue according to the invention is transgenic.

**[0370.0.0.0]** Accordingly, the invention therefore relates to transgenic organisms transformed with at least one nucleic acid molecule, nucleic acid construct or vector according to the invention, and to cells, cell cultures, tissues, parts - such as, for example, in the case of plant organisms, plant tissue, for example leaves, roots and the like - or propagation material derived from such organisms, or intact plants. The terms "recombinant (host)", and "transgenic (host)" are used interchangeably in this context. Naturally, these terms refer not only to the host organism or target cell in question, but also to the progeny, or potential progeny, of these organisms or cells. Since certain modifications may occur in subsequent generations owing to mutation or environmental effects, such progeny is not necessarily identical with the parental cell, but still comes within the scope of the term as used herein.

**[0371.0.0.0]** Suitable organisms for the process according to the invention or as hosts are all these eukaryotic or prokaryotic organisms, which are capable of synthesizing the respective fine chemical. The organisms used as hosts are microorganisms, such as bacteria, fungi, yeasts or algae, non-human animals, or plants, such as dictotyledonous or monocotyledonous plants.

**[0372.0.0.0]** In principle all plants can be used as host organism, especially the plants mentioned above as source organism. Preferred transgenic plants are, for example, selected from the families Aceraceae, Anacardiaceae, Apiaceae, Asteraceae, Brassicaceae, Cactaceae, Cucurbitaceae, Euphorbiaceae, Fabaceae, Malvaceae, Nymphaeaceae, Papaveraceae, Rosaceae, Salicaceae, Solanaceae, Arecaceae, Bromeliaceae, Cyperaceae, Iridaceae, Liliaceae, Orchidaceae, Gentianaceae, Labiaceae, Magnoliaceae, Ranunculaceae, Carifolaceae, Rubiaceae, Scrophulariaceae, Caryophyllaceae, Ericaceae, Polygonaceae, Violaceae, Juncaceae or Poaceae and preferably from a plant selected from the group of the families Apiaceae, Asteraceae, Brassicaceae, Cucurbitaceae, Fabaceae, Papaveraceae, Rosaceae, Solanaceae, Liliaceae or Poaceae. Preferred are crop plants such as plants advantageously selected from the group of the genus peanut, oilseed rape, canola, sunflower, safflower, olive, sesame, hazelnut, almond, avocado, bay, pumpkin/squash, linseed, soya, pistachio, borage, maize, wheat, rye, oats, sorghum and millet, triticale, rice, barley, cassava, potato, sugarbeet, egg plant, alfalfa, and perennial grasses and forage plants, oil palm, vegetables (brassicas, root vegetables, tuber vegetables, pod vegetables, fruiting vegetables, onion vegetables, leafy vegetables and stem vegetables), buckwheat, Jerusalem artichoke, broad bean, vetches, lentil, dwarf bean, lupin, clover and Lucerne for mentioning only some of them.

**[0373.0.0.0]** Preferred plant cells, plant organs, plant tissues or parts of plants originate from the under source organism mentioned plant families, preferably from the abovementioned plant genus, more preferred from abovementioned plants species.

**[0374.0.0.0]** Transgenic plants comprising the amino acids synthesized in the process according to the invention can be marketed directly without isolation of the compounds synthesized. In the process according to the invention, plants are understood as meaning all plant parts, plant organs such as leaf, stalk, root, tubers or seeds or propagation material or harvested material or the intact plant. In this context, the seed encompasses all parts of the seed such as the seed coats, epidermal cells, seed cells, endosperm or embryonic tissue. The amino acids produced in the process according to the invention may, however, also be isolated from the plant in the form of their free amino acids or bound in proteins. Amino acids produced by this process can be harvested by harvesting the organisms either from the culture in which they grow or from the field. This can be done via expressing, grinding and/or extraction, salt precipitation and/or ion-exchange chromatography of the plant parts, preferably the plant seeds, plant fruits, plant tubers and the like.

**[0375.0.0.0]** In a further embodiment, the present invention relates to a process for the generation of a microorganism, comprising the introduction, into the microorganism or parts thereof, of the nucleic acid construct of the invention, or the vector of the invention or the nucleic acid molecule of the invention or the nucleic acid molecule used in the method of the invention.

**[0376.0.0.0]** In another embodiment, the present invention relates also to a transgenic microorganism comprising the nucleic acid molecule of the invention or the nucleic acid molecule used in the method of the invention, the nucleic acid construct of the invention or the vector as of the invention. Appropriate microorganisms have been described herein before under source organism, preferred are in particular aforementioned strains suitable for the production of fine chemicals.

**[0377.0.0.0]** Accordingly, the present invention relates also to a process according to the present invention whereby the produced amino acid composition or the produced respective fine chemical is isolated.

**[0378.0.0.0]** In this manner, more than 50% by weight, advantageously more than 60% by weight, preferably more than 70% by weight, especially preferably more than 80% by weight, very especially preferably more than 90% by weight, of the fine chemicals produced in the process can be isolated. The resulting fine chemicals can, if appropriate, subsequently be further purified, if desired mixed with other active ingredients such as vitamins, amino acids, carbohydrates, antibiotics and the like, and, if appropriate, formulated.

**[0379.0.0.0]** In one embodiment, the fatty acid is the fine chemical.

**[0380.0.0.0]** The amino acids obtained in the process are suitable as starting material for the synthesis of further products of value. For example, they can be used in combination with each other or alone for the production of pharmaceuticals, foodstuffs, animal feeds or cosmetics. Accordingly, the present invention relates a method for the production of a pharmaceuticals, food stuff, animal feeds, nutrients or cosmetics comprising the steps of the process according to the invention, including the isolation of the amino acid composition produced or the fine chemical produced if desired and formulating the product with a pharmaceutical acceptable carrier or formulating the product in a form acceptable for an application in agriculture. A further embodiment according to the invention is the use of the amino acids produced in the process or of the transgenic organisms in animal feeds, foodstuffs, medicines, food supplements, cosmetics or pharmaceuticals.

**[0381.0.0.0]** In principle all microorganisms can be used as host organism especially the ones mentioned under source organism above. It is advantageous to use in the process of the invention transgenic microorganisms such as fungi such as the genus Claviceps or Aspergillus or Gram-positive bacteria such as the genera Bacillus, Corynebacterium, Micrococcus, Brevibacterium, Rhodococcus, Nocardia, Caseobacter or Arthrobacter or Gram-negative bacteria such as the genera Escherichia, Flavobacterium or Salmonella or yeasts such as the genera Rhodotorula, Hansenula or Candida. Particularly advantageous organisms are selected from the group of genera Corynebacterium, Brevibacterium, Escherichia, Bacillus, Rhodotorula, Hansenula, Candida, Claviceps or Flavobacterium. It is very particularly advantageous to use in the process of the invention microorganisms selected from the group of genera and species consisting of Hansenula anomala, Candida utilis, Claviceps purpurea, Bacillus circulans, Bacillus subtilis, Bacillus sp., Brevibacterium albidum, Brevibacterium album, Brevibacterium cerinum, Brevibacterium flavum, Brevibacterium glutamigenes, Brevibacterium iodinum, Brevibacterium ketoglutamicum, Brevibacterium lactofermentum, Brevibacterium linens, Brevibacterium roseum, Brevibacterium saccharolyticum, Brevibacterium sp., Corynebacterium acetoacidophilum, Corynebacterium acetoglutamicum, Corynebacterium ammoniagenes, Corynebacterium glutamicum (= Micrococcus glutamicum), Corynebacterium melassecola, Corynebacterium sp. or Escherichia coli, specifically Escherichia coli K12 and its described strains.

**[0382.0.0.0]** The process of the invention is, when the host organisms are microorganisms, advantageously carried out at a temperature between 0°C and 95°C, preferably between 10°C and 85°C, particularly preferably between 15°C and 75°C, very particularly preferably between 15°C and 45°C. The pH is advantageously kept at between pH 4 and 12, preferably between pH 6 and 9, particularly preferably between pH 7 and 8, during this. The process of the invention can be operated batchwise, semibatchwise or continuously. A summary of known cultivation methods is to be found in the textbook by Chmiel (Bioprozeßtechnik 1. Einführung in die Bioverfahrenstechnik (Gustav Fischer Verlag, Stuttgart, 1991)) or in the textbook by Storhas (Bioreaktoren und periphere Einrichtungen (Vieweg Verlag, Braunschweig/Wiesbaden, 1994)). The culture medium to be used must meet the requirements of the respective strains in a suitable manner. Descriptions of culture media for various microorganisms are present in the handbook "Manual of Methods for General Bacteriology" of the American Society for Bacteriology (Washington D. C., USA, 1981). These media, which can be employed according to the invention include, as described above, usually one or more carbon sources, nitrogen sources, inorganic salts, vitamins and/or trace elements. Preferred carbon sources are sugars such as mono-, di- or polysaccharides. Examples of very good carbon sources are glucose, fructose, mannose, galactose, ribose, sorbose, ribulose, lactose, maltose, sucrose, raffinose, starch or cellulose. Sugars can also be added to the media via complex compounds such as molasses, or other byproducts of sugar refining. It may also be advantageous to add mixtures of various carbon sources. Other possible carbon sources are oils and fats such as, for example, soybean oil, sunflower oil, peanut oil and/or coconut fat, fatty acids such as, for example, palmitic acid, stearic acid and/or linoleic acid, alcohols and/or polyalcohols such as, for example, glycerol, methanol and/or ethanol and/or organic acids such as, for example, acetic acid and/or lactic acid. Nitrogen sources are usually organic or inorganic nitrogen compounds or materials, which contain these compounds. Examples of nitrogen sources include ammonia in liquid or gaseous form or ammonium salts such as ammonium sulfate, ammonium chloride, ammonium phosphate, ammonium carbonate or ammonium nitrate, nitrates, urea, amino acids or complex nitrogen sources such as corn steep liquor, soybean meal, soybean protein, yeast extract, meat extract and others. The nitrogen sources may be used singly or as a mixture. Inorganic salt compounds, which may be present in the media include the chloride, phosphorus or sulfate salts of calcium, magnesium, sodium, cobalt, molybdenum, potassium, manganese, zinc, copper and iron.

**[0383.0.0.0]** For preparing sulfur-containing fine chemicals, in particular the respective fine chemical, e.g. amino acids containing sulfur it is possible to use as sulfur source inorganic sulfur-containing compounds such as, for example, sulfates, sulfites, dithionites, tetrathionates, thiosulfates, sulfides or else organic sulfur compounds such as mercaptans and thiols.

**[0384.0.0.0]** It is possible to use as phosphorus source phosphoric acid, potassium dihydrogenphosphate or dipotassium hydrogenphosphate or the corresponding sodium-containing salts. Chelating agents can be added to the medium in order to keep the metal ions in solution. Particularly suitable chelating agents include dihydroxyphenols such as catechol or protocatechuate, or organic acids such as citric acid. The fermentation media employed according to the invention for cultivating microorganisms normally also contain other growth factors such as vitamins or growth promoters, which include, for example, biotin, riboflavin, thiamine, folic acid, nicotinic acid, pantothenate and pyridoxine. Growth factors and salts are often derived from complex media components such as yeast extract, molasses, corn steep liquor and the like. Suitable precursors can moreover be added to the culture medium. The exact composition of the media compounds depends greatly on the particular experiment and is chosen individually for each specific case. Information about media optimization is obtainable from the textbook "Applied Microbiol. Physiology, A Practical Approach" (editors P.M. Rhodes, P.F. Stanbury, IRL Press (1997) pp. 53-73, ISBN 0 19 963577 3). Growth media can also be purchased from commercial suppliers such as Standard 1 (Merck) or BHI (Brain heart infusion, DIFCO) and the like. All media components are sterilized either by heat (1.5 bar and 121 °C for 20 min) or by sterilizing filtration. The components can be sterilized either together or, if necessary, separately. All media components can be present at the start of the cultivation or optionally be added continuously or batchwise. The temperature of the culture is normally between 15°C and 45°C, preferably at 25°C to 40°C, and can be kept constant or changed during the experiment. The pH of the medium should be in the range from 5 to 8.5, preferably around 7. The pH for the cultivation can be controlled during the cultivation by adding basic compounds such as sodium hydroxide, potassium hydroxide, ammonia or aqueous ammonia or acidic compounds such as phosphoric acid or sulfuric acid. Foaming can be controlled by employing antifoams such as, for example, fatty acid polyglycol esters. The stability of plasmids can be maintained by adding to the medium suitable substances having a selective effect, for example antibiotics. Aerobic conditions are maintained by introducing oxygen or oxygen-containing gas mixtures such as, for example, ambient air into the culture. The temperature of the culture is normally from 20°C to 45°C and preferably from 25°C to 40°C. The culture is continued until formation of the desired product is at a maximum. This aim is normally achieved within 10 hours to 160 hours.

**[0385.0.0.0]** The fermentation broths obtained in this way, containing in particular L-methionine, L-threonine and/or L-lysine, normally have a dry matter content of from 7.5 to 25% by weight. Sugar-limited fermentation is additional advantageous, at least at the end, but especially over at least 30% of the fermentation time. This means that the concentration of utilizable sugar in the fermentation medium is kept at, or reduced to, ≥ 0 to 3 g/l during this time. The fermentation broth is then processed further. Depending on requirements, the biomass can be removed entirely or partly by separation methods, such as, for example, centrifugation, filtration, decantation or a combination of these methods, from the fermentation broth or left completely in it. The fermentation broth can then be thickened or concentrated by known methods, such as, for example, with the aid of a rotary evaporator, thin-film evaporator, falling film evaporator, by reverse osmosis or by nanofiltration. This concentrated fermentation broth can then be worked up by freeze-drying, spray drying, spray granulation or by other processes.

**[0386.0.0.0]** However, it is also possible to purify the amino acid produced further. For this purpose, the product-containing composition is subjected to a chromatography on a suitable resin, in which case the desired product or the impurities are retained wholly or partly on the chromatography resin. These chromatography steps can be repeated if necessary, using the same or different chromatography resins. The skilled worker is familiar with the choice of suitable chromatography resins and their most effective use. The purified product can be concentrated by filtration or ultrafiltration and stored.at a temperature at which the stability of the product is a maximum.

**[0387.0.0.0]** The identity and purity of the isolated compound(s) can be determined by prior art techniques. These include high performance liquid chromatography (HPLC), spectroscopic methods, mass spectrometry (MS), staining methods, thin-layer chromatography, NIRS, enzyme assay or microbiological assays. These analytical methods are summarized in: Patek et al. (1994) Appl. Environ. Microbiol. 60:133-140; Malakhova et al. (1996) Biotekhnologiya 11 27-32; and Schmidt et al. (1998) Bioprocess Engineer. 19:67-70. Ulmann's Encyclopedia of Industrial Chemistry (1996) Vol. A27, VCH: Weinheim, pp. 89-90, pp. 521-540, pp. 540-547, pp. 559-566, 575-581 and pp. 581-587; Michal, G (1999) Biochemical Pathways: An Atlas of Biochemistry and Molecular Biology, John Wiley and Sons; Fallon, A. et al. (1987) Applications of HPLC in Biochemistry in: Laboratory Techniques in Biochemistry and Molecular Biology, Vol. 17.

**[0388.0.0.0]** In yet another aspect, the invention also relates to harvestable parts and to propagation material of the transgenic plants according to the invention which either contain transgenic plant cells expressing a nucleic acid molecule according to the invention or which contains cells which show an increased cellular activity of the polypeptide of the invention or the polypeptide used in the method of the invention, e.g. an increased expression level or higher activity of the described protein.

**[0389.0.0.0]** Harvestable parts can be in principle any useful parts of a plant, for example, flowers, pollen, seedlings, tubers, leaves, stems, fruit, seeds, roots etc. Propagation material includes, for example, seeds, fruits, cuttings, seedlings, tubers, rootstocks etc. Preferred are seeds, fruits, seedlings or tubers as harvestable or propagation material.

**[0390.0.0.0]** The invention furthermore relates to the use of the transgenic organisms according to the invention and of the cells, cell cultures, parts - such as, for example, roots, leaves and the like as mentioned above in the case of transgenic plant organisms - derived from them, and to transgenic propagation material such as seeds or fruits and the like as mentioned above, for the production of foodstuffs or feeding stuffs, pharmaceuticals or fine chemicals.

**[0391.0.0.0]** Accordingly in another embodiment, the present invention relates to the use of the nucleic acid molecule, the organism, e.g. the microorganism, the plant, plant cell or plant tissue, the vector, or the polypeptide of the present invention for making fatty acids, carotenoids, isoprenoids, vitamins, lipids, wax esters, (poly)saccharides and/or polyhydroxyalkanoates, and/or its metabolism products, in particular, steroid hormones, cholesterol, prostaglandin, triacylglycerols, bile acids and/or ketone bodies producing cells, tissues and/or plants. There are a number of mechanisms by which the yield, production, and/or efficiency of production of fatty acids, carotenoids, isoprenoids, vitamins, wax esters, lipids, (poly)saccharides and/or polyhydroxyalkanoates, and/or its metabolism products, in particular, steroid hormones, cholesterol, triacylglycerols, prostaglandin, bile acids and/or ketone bodies or further of above defined fine chemicals incorporating such an altered protein can be affected. In the case of plants, by e.g. increasing the expression of acetyl-CoA which is the basis for many products, e.g., fatty acids, carotenoids, isoprenoids, vitamines, lipids, (poly)saccharides, wax esters, and/or polyhydroxyalkanoates, and/or its metabolism products, in particular, prostaglandin, steroid hormones, cholesterol, triacylglycerols, bile acids and/or ketone bodies in a cell, it may be possible to increase the amount of the produced said compounds thus permitting greater ease of harvesting and purification or in case of plants more efficient partitioning. Further, one or more of said metabolism products, increased amounts of the cofactors, precursor molecules, and intermediate compounds for the appropriate biosynthetic pathways maybe required. Therefore, by increasing the number and/or activity of transporter proteins involved in the import of nutrients, such as carbon sources (i.e., sugars), nitrogen sources (i.e., amino acids, ammonium salts), phosphate, and sulfur, it may be possible to improve the production of acetyl CoA and its metabolism products as mentioned above, due to the removal of any nutrient supply limitations on the biosynthetic process. In particular, it may be possible to increase the yield, production, and/or efficiency of production of said compounds, e.g. fatty acids, carotenoids, isoprenoids, vitamins, was esters, lipids, (poly)saccharides, and/or polyhydroxyalkanoates, and/or its metabolism products, in particular, steroid hormones, cholesterol, prostaglandin, triacylglycerols, bile acids and/or ketone bodies molecules etc. in plants.

**[0392.0.0.0]** Furthermore preferred is a method for the recombinant production of pharmaceuticals or fine chemicals in host organisms, wherein a host organism is transformed with one of the above-described nucleic acid constructs comprising one or more structural genes which encode the desired fine chemical or catalyze the biosynthesis of the desired fine chemical, the transformed host organism is cultured, and the desired fine chemical is isolated from the culture medium. This method can be applied widely to fine chemicals such as enzymes, vitamins, amino acids, sugars, fatty acids, and natural and synthetic flavourings, aroma substances and colorants or compositions comprising these. Especially preferred is the additional production of further amino acids, tocopherols and tocotrienols and carotenoids or compositions comprising said compounds. The transformed host organisms are cultured and the products are recovered from the host organisms or the culture medium by methods known to the skilled worker or the organism itself servers as food or feed supplement. The production of pharmaceuticals such as, for example, antibodies or vaccines, is described by Hood EE, Jilka JM. Curr Opin Biotechnol. 1999 Aug; 10(4):382-6; Ma JK, Vine ND. Curr Top Microbiol Immunol. 1999; 236:275-92.

**[0393.0.0.0]** In one embodiment, the present invention relates to a method for the identification of a gene product conferring an increase in the respective fine chemical production in a cell, comprising the following steps:
(a) contacting, e.g. hybridising, the nucleic acid molecules of a sample, e.g. cells, tissues, plants or microorganisms or a nucleic acid library , which can contain a candidate gene encoding a gene product conferring an increase in the respective fine chemical after expression, with the nucleic acid molecule of the present invention;
(b) identifying the nucleic acid molecules, which hybridize under relaxed stringent conditions with the nucleic acid molecule of the present invention in particular to a nucleic acid molecule sequence as indicated in Table I, columns 5 or 7, lines 1 to 5 and/or lines 334 to 338, preferably in Table I B, columns 5 or 7, lines 1 to 5 and/or lines 334 to 338 and, optionally, isolating the full length cDNA clone or complete genomic clone;
(c) introducing the candidate nucleic acid molecules in host cells, preferably in a plant cell or a microorganism, appropriate for producing the respective fine chemical;
(d) expressing the identified nucleic acid molecules in the host cells;
(e) assaying the respective fine chemical level in the host cells; and
(f) identifying the nucleic acid molecule and its gene product which expression confers an increase in the the respective fine chemical level in the host cell after expression compared to the wild type.

**[0394.0.0.0]** Relaxed hybridisation conditions are: After standard hybridisation procedures washing steps can be performed at low to medium stringency conditions usually with washing conditions of 40°-55°C and salt conditions between 2xSSC and 0,2x SSC with 0,1% SDS in comparison to stringent washing conditions as e.g. 60°-68°C with 0,1% SDS. Further examples can be found in the references listed above for the stringent hybridization conditions. Usually washing steps are repeated with increasing stringency and length until a useful signal to noise ratio is detected and depend on many factors as the target, e.g. its purity, GC-content, size etc, the probe, e.g. its length, is it a RNA or a DNA probe, salt conditions, washing or hybridisation temperature, washing or hybridisation time etc.

**[0395.0.0.0]** In an other embodiment, the present invention relates to a method for the identification of a gene product conferring an increase in the respective fine chemical production in a cell, comprising the following steps:
(a) identifying nucleic acid molecules of an organism; which can contain a candidate gene encoding a gene product conferring an increase in the respective fine chemical after expression, which are at least 20%, preferably 25%, more preferably 30%, even more preferred are 35%. 40% or 50%, even more preferred are 60%, 70% or 80%, most preferred are 90% or 95% or more homology to the nucleic acid molecule of the present invention, for example via homology search in a data bank;
(b) introducing the candidate nucleic acid molecules in host cells, preferably in a plant cells or microorganisms, appropriate for producing the respective fine chemical;
(c) expressing the identified nucleic acid molecules in the host cells;
(d) assaying the the respective fine chemical level in the host cells; and
(e) identifying the nucleic acid molecule and its gene product which expression confers an increase in the the respective fine chemical level in the host cell after expression compared to the wild type.
   Eventually gene products conferring the increase in the respective fine chemical production can also be identify according to a identical or similar 3D structure in step (a) and by the above described method.

**[0396.0.0.0]** The nucleic acid molecules identified can then be used for the production of the respective fine chemical in the same way as the nucleic acid molecule of the present invention. Accordingly, in one embodiment, the present invention relates to a process for the production of the respective fine chemical, comprising (a) identifying a nucleic acid molecule according to aforementioned steps (a) to (f) or (a) to (e) and recovering the free or bound fine chemical from a organism having an increased cellular activity of a polypeptide encoded by the isolated nucleic acid molecule compared to a wild type.

**[0397.0.0.0]** Furthermore, in one embodiment, the present invention relates to a method for the identification of a compound stimulating production of the respective fine chemical to said plant comprising:
a) contacting cells which express the polypeptide of the present invention or its mRNA with a candidate compound under cell cultivation conditions;
b) assaying an increase in expression of said polypeptide or said mRNA;
c) comparing the expression level to a standard response made in the absence of said candidate compound; whereby, an increased expression over the standard indicates that the compound is stimulating production of the respective fine chemical.

**[0398.0.0.0]** Furthermore, in one embodiment, the present invention relates to a method for the screening for agonists or an antagonist of the activity of the polypeptide of the present invention or used in the process of the present invention, e.g. a polypeptide conferring an increase of the respective fine chemical in an organism or a part thereof after increasing the activity in an organism or a part thereof, comprising:
(a) contacting cells, tissues , plants or microorganisms which express the polypeptide according to the invention with a candidate compound or a sample comprising a plurality of compounds under conditions which permit the expression the polypeptide of the present invention or used in the process of the present invention;
(b) assaying the respective fine chemical level or the polypeptide expression level in the cell, tissue, plant or microorganism or the media the cell, tissue, plant or microorganisms is cultured or maintained in; and
(c) identifying a agonist or antagonist by comparing the measured the respective fine chemical level or polypeptide of the invention or used in the invention expression level with a standard the respective fine chemical or polypeptide expression level measured in the absence of said candidate compound or a sample comprising said plurality of compounds, whereby an increased level over the standard indicates that the compound or the sample comprising said plurality of compounds is an agonist and a decreased level over the standard indicates that the compound or the sample comprising said plurality of compounds is an antagonist.

**[0399.0.0.0]** Furthermore, in one embodiment, the present invention relates to process for the identification of a compound conferring increased the respective fine chemical production in a plant or microorganism, comprising the steps:
(a) culturing a cell or tissue or microorganism or maintaining a plant expressing the polypeptide according to the invention or a nucleic acid molecule encoding said polypeptide and a readout system capable of interacting with the polypeptide under suitable conditions which permit the interaction of the polypeptide with said readout system in the presence of a compound or a sample comprising a plurality of compounds and capable of providing a detectable signal in response to the binding of a compound to said polypeptide under conditions which permit the expression of said readout system and the polypeptide of the present invention or used in the process of the invention; and
(b) identifying if the compound is an effective agonist by detecting the presence or absence or increase of a signal produced by said readout system.
The screen for a gene product or an agonist conferring an increase in the respective fine chemical production can be performed by growth of an organism for example a microorganism in the presence of growth reducing amounts of an inhibitor of the synthesis of the respective fine chemical. Better growth, e.g. higher dividing rate or high dry mass in comparison to the control under such conditions would identify a gene or gene product or an agonist conferring an increase in fine chemical production.

**[0399.1.0.0]** One can think to screen for increased production of the respective fine chemical by for example searching for a resistance to a drug blocking the synthesis of the respective fine chemical and looking whether this effect is dependent on the activity or expression of a polypeptide as indicated in Table II, columns 5 or 7, lines 1 to 5 and/or lines 334 to 338 or a homolog thereof, e.g. comparing the phenotype of nearly identical organisms with low and high activity of a protein as indicated in Table II, columns 5 or 7, lines 1 to 5 and/or lines 334 to 338 after incubation with the drug.

**[0400.0.0.0]** Said compound may be chemically synthesized or microbiologically produced and/or comprised in, for example, samples, e.g., cell extracts from, e.g., plants, animals or microorganisms, e.g. pathogens. Furthermore, said compound(s) may be known in the art but hitherto not known to be capable of suppressing or activating the polypeptide of the present invention. The reaction mixture may be a cell free extract or may comprise a cell or tissue culture. Suitable set ups for the method of the invention are known to the person skilled in the art and are, for example, generally described in Alberts et al., Molecular Biology of the Cell, third edition (1994), in particular Chapter 17. The compounds may be, e.g., added to the reaction mixture, culture medium, injected into the cell or sprayed onto the plant.

**[0401.0.0.0]** If a sample containing a compound is identified in the method of the invention, then it is either possible to isolate the compound from the original sample identified as containing the compound capable of activating or increasing the content of the respective fine chemical in an organism or part thereof, or one can further subdivide the original sample, for example, if it consists of a plurality of different compounds, so as to reduce the number of different substances per sample and repeat the method with the subdivisions of the original sample. Depending on the complexity of the samples, the steps described above can be performed several times, preferably until the sample identified according to the method of the invention only comprises a limited number of or only one substance(s). Preferably said sample comprises substances of similar chemical and/or physical properties, and most preferably said substances are identical. Preferably, the compound identified according to the above described method or its derivative is further formulated in a form suitable for the application in plant breeding or plant cell and tissue culture.

**[0402.0.0.0]** The compounds which can be tested and identified according to a method of the invention may be expression libraries, e.g., cDNA expression libraries, peptides, proteins, nucleic acids, antibodies, small organic compounds, hormones, peptidomimetics, PNAs or the like (Milner, Nature Medicine 1 (1995), 879-880; Hupp, Cell 83 (1995), 237-245; Gibbs, Cell 79 (1994), 193-198 and references cited supra). Said compounds can also be functional derivatives or analogues of known inhibitors or activators. Methods for the preparation of chemical derivatives and analogues are well known to those skilled in the art and are described in, for example, Beilstein, Handbook of Organic Chemistry, Springer edition New York Inc., 175 Fifth Avenue, New York, N.Y. 10010 U.S.A. and Organic Synthesis, Wiley, New York, USA. Furthermore, said derivatives and analogues can be tested for their effects according to methods known in the art. Furthermore, peptidomimetics and/or computer aided design of appropriate derivatives and analogues can be used, for example, according to the methods described above. The cell or tissue that may be employed in the method of the invention preferably is a host cell, plant cell or plant tissue of the invention described in the embodiments hereinbefore.

**[0403.0.0.0]** Thus, in a further embodiment the invention relates to a compound obtained or identified according to the method for identifying an agonist of the invention said compound being an agonist of the polypeptide of the present invention or used in the process of the present invention.

**[0404.0.0.0]** Accordingly, in one embodiment, the present invention further relates to a compound identified by the method for identifying a compound of the present invention.

**[0405.0.0.0]** Said compound is, for example, a homologous of the polypeptide of the present invention. Homologues of the polypeptid of the present invention can be generated by mutagenesis, e.g., discrete point mutation or truncation of the polypeptide of the present invention. As used herein, the term "homologue" refers to a variant form of the protein, which acts as an agonist of the activity of the polypeptide of the present invention. An agonist of said protein can retain substantially the same, or a subset, of the biological activities of the polypeptide of the present invention. In particular, said agonist confers the increase of the expression level of the polypeptide of the present invention and/or the expression of said agonist in an organisms or part thereof confers the increase of free and/or bound the respective fine chemical in the organism or part thereof.

**[0406.0.0.0]** In one embodiment, the invention relates to an antibody specifically recognizing the compound or agonist of the present invention.

**[0407.0.0.0]** The invention also relates to a diagnostic composition comprising at least one of the aforementioned nucleic acid molecules, vectors, proteins, antibodies or compounds of the invention and optionally suitable means for detection.

**[0408.0.0.0]** The diagnostic composition of the present invention is suitable for the isolation of mRNA from a cell and contacting the mRNA so obtained with a probe comprising a nucleic acid probe as described above under hybridizing conditions, detecting the presence of mRNA hybridized to the probe, and thereby detecting the expression of the protein in the cell. Further methods of detecting the presence of a protein according to the present invention comprise immunotechniques well known in the art, for example enzyme linked immunosorbent assay. Furthermore, it is possible to use the nucleic acid molecules according to the invention as molecular markers or primer in plant breeding. Suitable means for detection are well known to a person skilled in the arm, e.g. buffers and solutions for hydridization assays, e.g. the aforementioned solutions and buffers, further and means for Southern-, Western-, Northern- etc. -blots, as e.g. described in Sambrook et al. are known.

**[0409.0.0.0]** In another embodiment, the present invention relates to a kit comprising the nucleic acid molecule, the vector, the host cell, the polypeptide, the antisense nucleic acid, the antibody, plant cell, the plant or plant tissue, the harvestable part, the propagation material and/or the compound or agonist or antagonists identified according to the method of the invention.

**[0410.0.0.0]** The compounds of the kit of the present invention may be packaged in containers such as vials, optionally with/in buffers and/or solution. If appropriate, one or more of said components might be packaged in one and the same container. Additionally or alternatively, one or more of said components might be adsorbed to a solid support as, e.g. a nitrocellulose filter, a glass plate, a chip, or a nylon membrane or to the well of a micro titerplate. The kit can be used for any of the herein described methods and embodiments, e.g. for the production of the host cells, transgenic plants, pharmaceutical compositions, detection of homologous sequences, identification of antagonists or agonists, as food or feed or as a supplement thereof, as supplement for the treating of plants, etc.

**[0411.0.0.0]** Further, the kit can comprise instructions for the use of the kit for any of said embodiments, in particular for the use for producing organisms or part thereof having an increased free or bound the respective fine chemical content.

**[0412.0.0.0]** In one embodiment said kit comprises further a nucleic acid molecule encoding one or more of the aforementioned protein, and/or an antibody, a vector, a host cell, an antisense nucleic acid, a plant cell or plant tissue or a plant.

**[0413.0.0.0]** In a further embodiment, the present invention relates to a method for the production of a agricultural composition providing the nucleic acid molecule, the vector or the polypeptide of the invention or the polypeptide used in the method of the invention or comprising the steps of the method according to the invention for the identification of said compound, agonist or antagonist; and formulating the nucleic acid molecule, the vector or the polypeptide of the invention or the polypeptide used in the method of the invention or the agonist, or compound identified according to the methods or processes of the present invention or with use of the subject matters of the present invention in a form applicable as plant agricultural composition.

**[0414.0.0.0]** In another embodiment, the present invention relates to a method for the production of a "the respective fine chemical"-production supporting plant culture composition comprising the steps of the method for of the present invention; and formulating the compound identified in a form acceptable as agricultural composition.

**[0415.0.0.0]** Under "acceptable as agricultural composition" is understood, that such a composition is in agreement with the laws regulating the content of fungicides, plant nutrients, herbicides, etc. Preferably such a composition is without any harm for the protected plants and the animals (humans included) fed therewith.

**[0416.0.0.0]** The present invention also pertains to several embodiments relating to further uses and methods. The nucleic acid molecule, polypeptide, protein homologues, fusion proteins, primers, vectors, host cells, described herein can be used in one or more of the following methods: identification of plants useful for the respective fine chemical production as mentioned and related organisms; mapping of genomes; identification and localization of sequences of interest; evolutionary studies; determination of regions required for function; modulation of an activity.

**[0417.0.0.0]** The nucleic acid molecule of the invention or the nucleic acid molecule used in the method of the invention, the vector of the invention or the nucleic acid construct of the invention may also be useful for the production of organisms resistant to inhibitors of the amino acid production biosynthesis pathways. In particular, the overexpression of the polypeptide of the present invention may protect plants against herbicides, which block the amino acid, in particular the respective fine chemical, synthesis in said plant. Inhibitors may inhibit one or more of the steps for the synthesis of methionine. The first committed step for the synthesis of Lys, Met and Thr is the first step, in which aspartate is phosphorylated to aspartyl-b-phosphate, catalyzed by aspartokinase: E.coli has 3 isozymes of aspartokinase that respond differently to each of the 3 amino acids, with regard to enzyme inhibition and feedback inhibition. The biosynthesis of lysine, methionine and threonine are not, then, controlled as a group. The pathway from aspartate to lysine has 10 steps. The pathway from aspartate to threonine has 5 steps. The pathway from aspartate to methionine has 7 steps. Regulation of the three pathways also occurs at the two branch points:
- b-Aspartate-semialdehyde (homoserine and lysine)
- Homoserine (threonine and methionine)

The regulation results from feedback inhibition by the amino acid products of the branches, indicated in the brackets above. One important step in the synthesis of this group of 3 amino acids is the step in which homocysteine is converted to methionine, catalyzed by the enzyme methionine synthase: 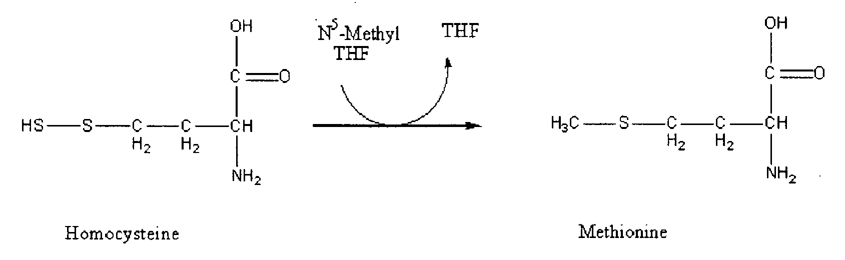 In this reaction, homocysteine is methylated to methionine, and the C1 donor is N5-methyl-THF. Thus, inhibition of one or more of the methionine synthesis enzymes, including also the provision of donor molecules, can inhibit the synthesis of methionine.
Examples of herbicides blocking the amino acid synthesis in plants are for example sulfonylurea and imidazolinone herbicides, which catalyze the first step in branched-chain amino acid biosynthesis. Inhibitors of the methionine synthesis may for example described in Danishpajooh IO, 2001 Nitric oxide inhibits methionine synthase activity in vivo and disrupts carbon flow through the folate pathway. J. Biol. Chem. 276: 27296-27303; Datko AH, 1982 Methionine biosynthesis in Lemna - inhibitor studies. Plant Physiol. 69: 1070-1076; Lavrador K, 1998 A new series of cyclic amino acids as inhibitors of S-adenosyl L-methionine synthetase. Bioorg. Med. Chem. Lett. 8: 1629-1634; Thompson GA, 1982 Methionine synthesis in Lemna - inhibition of cystathionine gamma-synthase by propargylglycine. Plant Physiol. 70: 1347-1352. In some organisms the methionine synthesis is inhibited by ethanol, lead, mercury, aluminium, thimerosal, cupper, N2O, as e.g. discussed in M. Waly, H. Oleteanu et al., 2004, Molecular Psychiatry, 1-13.
Interestingly, Arabidopsis seed germination was strongly delayed in the presence of DL-propargylglycine, a specific inhibitor of methionine synthesis. Furthermore, this compound totally inhibited seedling growth. These phenotypic effects were largely alleviated upon methionine supplementation in the germination medium. The results indicated that methionine synthase and S-adenosylmethionine synthetase are fundamental components controlling metabolism in the transition from a quiescent to a highly active state during seed germination. Moreover, the observed temporal patterns of accumulation of these proteins are consistent with an essential role of endogenous ethylene in Arabidopsis only after radicle protrusion; s. Gallarado, K., 2002, Importance of methionine biosynthesis for Arabidopsis seed germination and seedling growth, Physiolgia Plantarum, 116(2), pp 238-247. Accordingly, the overexpression of a polypeptide of the present invention in a plant may protect the plant against a herbicide inhibiting methionine synthesis.

**[0418.0.0.0]** Accordingly, the nucleic acid molecules of the present invention have a variety of uses. First, they may be used to identify an organism or a close relative thereof. Also, they may be used to identify the presence thereof or a relative thereof in a mixed population of microorganisms or plants. By probing the extracted genomic DNA of a culture of a unique or mixed population of plants under stringent conditions with a probe spanning a region of the gene of the present invention which is unique to this, one can ascertain whether the present invention has been used or whether it or a close relative is present.

**[0419.0.0.0]** Further, the nucleic acid molecule of the invention or the nucleic acid molecule used in the method of the invention may be sufficiently homologous to the sequences of related species such that these nucleic acid molecules may serve as markers for the construction of a genomic map in related organism.

**[0420.0.0.0]** Accordingly, the present invention relates to a method for breeding plants for the production of the respective fine chemical, comprising
(a) providing a first plant variety produced according to the process of the invention preferably (over)expressing the nucleic acid molecule of the invention or the nucleic acid molecule used in the method of the invention;
(b) crossing the first plant variety with a second plant variety; and
(c) selecting the offspring plants which overproduce the respective fine chemical by means of analysis the distribution of a molecular marker in the offspring representing the first plant variety and its capability to (over)produce the respective fine chemical.
Details about the use of molecular markers in breeding can be found in Kumar et al., 1999 (Biotech Adv., 17:143-182) and Peleman and van der Voort 2003 (Trends Plant Sci. 2003 Jul;8(7):330-334)
The molecular marker can e.g. relate to the nucleic acid molecule of the invention or the nucleic acid molecule used in the method of the invention and/or its expression level. Accordingly, the molecular marker can be a probe or a PCR primer set useful for identification of the genomic existence or genomic localisation of the nucleic acid molecule of the invention or the nucleic acid molecule used in the method of the invention, e.g. in a Southern blot analysis or a PCR or its expression level, i.g. in a Northern Blot analysis or a quantitative PCR.
Accordingly, in one embodiment, the present invention relates to the use of the nucleic acid molecule of the present invention or encoding the polypeptide of the present invention as molecular marker for breeding, especially for breeding for a high or low respective fine chemical production.

**[0421.0.0.0]** The nucleic acid molecules of the invention are also useful for evolutionary and protein structural studies. By comparing the sequences of the invention or used in the process of the invention to those encoding similar enzymes from other organisms, the evolutionary relatedness of the organisms can be assessed. Similarly, such a comparison permits an assessment of which regions of the sequence are conserved and which are not, which may aid in determining those regions of the protein which are essential for the functioning of the enzyme. This type of determination is of value for protein engineering studies and may give an indication of what the protein can tolerate in terms of mutagenesis without losing function.

**[0422.0.0.0]** Accordingly, the nucleic acid molecule of the invention or the nucleic acid molecule used in the method of the invention can be used for the identification of other nucleic acids conferring an increase of the respective fine chemical after expression.

**[0423.0.0.0]** Further, the nucleic acid molecule of the invention or the nucleic acid molecule used in the method of the invention or a fragment of a gene conferring the expression of the polypeptide of the invention or the polypeptide used in the method of the invention, preferably comprising the nucleic acid molecule of the invention, can be used for marker assisted breeding or association mapping of the respective fine chemical derived traits

**[0424.0.0.0]** Accordingly, the nucleic acid of the invention, the polypeptide of the invention or the polypeptide used in the method of the invention, the nucleic acid construct of the invention, the organisms, the host cell, the microorganisms, the plant, plant tissue, plant cell, or the part thereof of the invention, the vector of the invention, the agonist identified with the method of the invention, the nucleic acid molecule identified with the method of the present invention, can be used for the production of the respective fine chemical or of the fine chemical and one or more other amino acids, in particular Threoinine, Alanine, Glutamin, Glutamic acid, Valine, Asparagine, Phenylalanine, Leucine, Proline, Tryptophan Tyrosine, Valine, Isoleucine and Arginine. Accordingly, the nucleic acid of the invention, or the nucleic acid molecule identified with the method of the present invention or the complement sequences thereof, the polypeptide of the invention or the polypeptide used in the method of the invention, the nucleic acid construct of the invention, the organisms, the host cell, the microorganisms, the plant, plant tissue, plant cell, or the part thereof of the invention, the vector of the invention, the antagonist identified with the method of the invention, the antibody of the present invention, the antisense molecule of the present invention, can be used for the reduction of the respective fine chemical in a organism or part thereof, e.g. in a cell.

**[0425.0.0.0]** Further, the nucleic acid of the invention, the polypeptide of the invention or the polypeptide used in the method of the invention, the nucleic acid construct of the invention, the organisms, the host cell, the microorganisms, the plant, plant tissue, plant cell, or the part thereof of the invention, the vector of the invention, the antagonist or the agonist identified with the method of the invention, the antibody of the present invention, the antisense molecule of the present invention or the nucleic acid molecule identified with the method of the present invention, can be used for the preparation of an agricultural composition.

**[0426.0.0.0]** Furthermore, the nucleic acid of the invention, the polypeptide of the invention or the polypeptide used in the method of the invention, the nucleic acid construct of the invention, the organisms, the host cell, the microorganisms, the plant, plant tissue, plant cell, or the part thereof of the invention, the vector of the invention, antagonist or the agonist identified with the method of the invention, the antibody of the present invention, the antisense molecule of the present invention or the nucleic acid molecule identified with the method of the present invention, can be used for the identification and production of compounds capable of conferring a modulation of the respective fine chemical levels in an organism or parts thereof, preferably to identify and produce compounds conferring an increase of the respective fine chemical levels in an organism or parts thereof, if said identified compound is applied to the organism or part thereof, i.e. as part of its food, or in the growing or culture media.

**[0427.0.0.0]** These and other embodiments are disclosed and encompassed by the description and examples of the present invention. Further literature concerning any one of the methods, uses and compounds to be employed in accordance with the present invention may be retrieved from public libraries, using for example electronic devices. For example the public database "Medline" may be utilized which is available on the Internet, for example under hftp://www.ncbi.nlm.nih.gov/PubMed/medline.html. Further databases and addresses, such as hftp://www.ncbi.nlm.nih.gov/, hftp://www.infobiogen.fr/, hftp://www.fmi.ch/biology/research-tools.html, hftp://www.tigr.org/, are known to the person skilled in the art and can also be obtained using, e.g., hftp://www.lycos.com. An overview of patent information in biotechnology and a survey of relevant sources of patent information useful for retrospective searching and for current awareness is given in Berks, TIBTECH 12 (1994), 352-364.

**[0428.0.0.0]** Table 1 gives an overview about the sequences disclosed in the present invention.

| | | | |
|---|---|---|---|
| 1) | Increase of the metabolites: | | |
| | Max: maximal x-fold | (normalised to wild type)- | |
| | Min: minimal x-fold | (normalised to wild type) | |
| 2) | Decrease of the metabolites: | | |
| | Max: maximal x-fold | (normalised to wild type) | (minimal decrease) |
| | Min: minimal x-fold | (normalised to wild type) | (maximal decrease) |

**[0429.0.0.0]** The present invention is illustrated by the examples, which follow. The present examples illustrate the basic invention without being intended as limiting the subject of the invention. The content of all of the references, patent applications, patents and published patent applications cited in the present patent application is herewith incorporated by reference.

**[0430.0.0.0]** Examples

**[0431.0.0.0]** Example 1: Cloning into in Escherichia coli

**[0432.0.0.0]** A DNA polynucleotide with a sequence as indicated in Table I, column 5 and encoding a polypeptide as listed in Table 1 below, was cloned into the plasmids pBR322 (Sutcliffe, J.G. (1979) Proc. Natl Acad. Sci. USA, 75: 3737-3741); pACYC177 (Change & Cohen (1978) J. Bacteriol. 134: 1141-1156); plasmids of the pBS series (pBSSK+, pBSSK- and others; Stratagene, LaJolla, USA) or cosmids such as SuperCos1 (Stratagene, LaJolla, USA) or Lorist6 (Gibson, T.J. Rosenthal, A., and Waterson, R.H. (1987) Gene 53: 283-286) for expression in E. coli using known, well-established procedures (see, for example, Sambrook, J. et al. (1989) "Molecular Cloning: A Laboratory Manual". Cold Spring Harbor Laboratory Press or Ausubel, F.M. et al. (1994) "Current Protocols in Molecular Biology", John Wiley & Sons).

**[0433.0.0.0]** Example 2: DNA sequencing and computerized functional analysis

**[0434.0.0.0]** The DNA was sequenced by standard procedures, in particular the chain determination method, using ABI377 sequencers (see, for example, Fleischman, R.D. et al. (1995) "Whole-genome Random Sequencing and Assembly of Haemophilus Influenzae Rd., Science 269; 496-512)".

**[0435.0.0.0]** Example 3: In-vivo and in-vitro mutagenesis

**[0436.0.0.0]** An *in vivo* mutagenesis of Corynebacterium glutamicum for the production of the respective fine chemical can be carried out by passing a plasmid DNA (or another vector DNA) through E. coli and other microorganisms (for example Bacillus spp. or yeasts such as Saccharomyces cerevisiae), which are not capable of maintaining the integrity of its genetic information. Usual mutator strains have mutations in the genes for the DNA repair system [for example mutHLS, mutD, mutT and the like; for comparison, see Rupp, W.D. (1996) DNA repair mechanisms in Escherichia coli and Salmonella, pp. 2277-2294, ASM: Washington]. The skilled worker knows these strains. The use of these strains is illustrated for example in Greener, A. and Callahan, M. (1994) Strategies 7; 32-34.

**[0436.1.0.0]** In-vitro mutation methods such as increasing the spontaneous mutation rates by chemical or physical treatment are well known to the skilled person. Mutagens like 5-bromo-uracil, N-methyl-N-nitro-N-nitrosoguanidine (= NTG), ethyl methanesulfonate (= EMS), hydroxylamine and/or nitrous acid are widly used as chemical agents for random in-vitro mutagenesis. The most common physical method for mutagensis is the treatment with UV irradiation. Another random mutagenesis technique is the error-prone PCR for introducing amino acid changes into proteins. Mutations are deliberately introduced during PCR through the use of error-prone DNA polymerases and special reaction conditions known to a person skilled in the art. For this method randomized DNA sequences are cloned into expression vectors and the resulting mutant libraries screened for altered or improved protein activity as described below.
Site-directed mutagensis method such as the introduction of desired mutations with an M13 or phagemid vector and short oligonucleotides primers is a well-known approach for site-directed mutagensis. The clou of this method involves cloning of the nucleic acid sequence of the invention into an M13 or phagemid vector, which permits recovery of single-stranded recombinant nucleic acid sequence. A mutagenic oligonucleotide primer is then designed whose sequence is perfectly complementary to nucleic acid sequence in the region to be mutated, but with a single difference: at the intended mutation site it bears a base that is complementary to the desired mutant nucleotide rather than the original. The mutagenic oligonucleotide is then allowed to prime new DNA synthesis to create a complementary full-length sequence containing the desired mutation. Another site-directed mutagensis method is the PCR mismatch primer mutagensis method also known to the skilled person. DpnI site-directed mutagensis is a further known method as described for example in the Stratagene QuickchangeTM site-directed mutagenesis kit protocol. A huge number of other methods are also known and used in common practice.
Positive mutation events can be selected by screening the organisms for the production of the desired respective fine chemical.

**[0437.0.0.0]** Example 4: DNA transfer between Escherichia coli and Corynebacterium glutamicum

**[0438.0.0.0]** Several Corynebacterium and Brevibacterium species comprise endogenous plasmids (such as, for example, pHM1519 or pBL1) which replicate autonomously (for a review, see, for example, Martin, J.F. et al. (1987) Biotechnology 5: 137-146). Shuttle vectors for Escherichia coli and Corynebacterium glutamicum can be constructed easily using standard vectors for E. coli (Sambrook, J. et al., (1989), "Molecular Cloning: A Laboratory Manual", Cold Spring Harbor Laboratory Press or Ausubel, F.M. et al. (1994) "Current Protocols in Molecular Biology", John Wiley & Sons), which have a replication origin for, and suitable marker from, Corynebacterium glutamicum added. Such replication origins are preferably taken from endogenous plasmids, which have been isolated from Corynebacterium and Brevibacterium species. Genes, which are used in particular as transformation markers for these species are genes for kanamycin resistance (such as those which originate from the Tn5 or Tn-903 transposon) or for chloramphenicol resistance (Winnacker, E.L. (1987) "From Genes to Clones - Introduction to Gene Technology, VCH, Weinheim). There are many examples in the literature of the preparation of a large multiplicity of shuttle vectors which are replicated in E. coli and C. glutamicum and which can be used for various purposes including the overexpression of genes (see, for example, Yoshihama, M. et al. (1985) J. Bacteriol. 162: 591-597, Martin, J.F. et al., (1987) Biotechnology, 5: 137-146 and Eikmanns, B.J. et al. (1992) Gene 102: 93-98). Suitable vectors, which replicate in coryneform bacteria are, for example, pZ1 (Menkel et al., Appl. Environ. Microbiol., 64, 1989: 549 - 554) pEkEx1 (Eikmanns et al., Gene 102, 1991: 93 - 98) or pHS2-1 (Sonnen et al, Gene 107, 1991: 69 - 74). These vectors are based on the cryptic plasmids pHM1519, pBL1 or pGA1. Other plasmid vectors such as, for example, those based on pCG4 (US 4,489,160), pNG2 (Serwold-Davis et al., FEMS Microbiol. Lett., 66, 1990: 119 - 124) or pAG1 (US 5,158,891) can be used in the same manner.

**[0439.0.0.0]** Using standard methods, it is possible to clone a gene of interest into one of the above-described shuttle vectors and to introduce such hybrid vectors into Corynebacterium glutamicum strains. The transformation of C. glutamicum can be achieved by protoplast transformation (Kastsumata, R. et al., (1984) J. Bacteriol. 159, 306-311), electroporation (Liebl, E. et al., (1989) FEMS Microbiol. Letters, 53: 399-303) and in those cases where specific vectors are used also by conjugation (such as, for example, described in Schäfer, A., et al. (1990) J. Bacteriol. 172: 1663-1666). Likewise, it is possible to transfer the shuttle vectors for C. glutamicum to E. coli by preparing plasmid DNA from C. glutamicum (using standard methods known in the art) and transforming it into E. coli. This transformation step can be carried out using standard methods, but preferably using an Mcr-deficient E. coli strain, such as NM522 (Gough & Murray (1983) J. Mol. Biol. 166: 1-19).

**[0440.0.0.0]** If the transformed sequence(s) is/are to be integrated advantageously into the genome of the coryneform bacteria, standard techniques known to the skilled worker also exist for this purpose. Examples, which are used for this purpose are plasmid vectors as they have been described by Remscheid et al. (Appl. Environ. Microbiol., 60, 1994: 126-132) for the duplication and amplification of the hom-thrB operon. In this method, the complete gene is cloned into a plasmid vector which is capable of replication in a host such as E. coli, but not in C. glutamicum. Suitable vectors are, for example, pSUP301 (Simon et al., Bio/Technology 1, 1983: 784-791), pKIBmob or pK19mob (Schäfer et al., Gene 145, 1994: 69-73), pGEM-T (Promega Corp., Madison, WI, USA), pCR2.1-TOPO (Schuman, J. Biol. Chem., 269, 1994: 32678-32684, US 5,487,993), pCR^{®}Blunt (Invitrogen, Groningen, the Netherlands) or pEM1 (Schrumpf et al., J. Bacteriol., 173, 1991: 4510-4516).

**[0441.0.0.0]** Example 5: Determining the expression of the mutant/transgenic protein

**[0442.0.0.0]** The observations of the activity of a mutated, or transgenic, protein in a transformed host cell are based on the fact that the protein is expressed in a similar manner and in a similar quantity as the wild-type protein. A suitable method for determining the transcription quantity of the mutant, or transgenic, gene (a sign for the amount of mRNA which is available for the translation of the gene product) is to carry out a Northern blot (see, for example, Ausubel et al., (1988) Current Protocols in Molecular Biology, Wiley: New York), where a primer which is designed in such a way that it binds to the gene of interest is provided with a detectable marker (usually a radioactive or chemiluminescent marker) so that, when the total RNA of a culture of the organism is extracted, separated on a gel, applied to a stable matrix and incubated with this probe, the binding and quantity of the binding of the probe indicates the presence and also the amount of mRNA for this gene. Another method is a quantitative PCR. This information detects the extent to which the gene has been transcribed. Total cell RNA can be isolated from Corynebacterium glutamicum or other microorganisms by a variety of methods, which are known in the art, e.g. as described in Bormann, E.R. et al., (1992) Mol. Microbiol. 6: 317-326.

**[0443.0.0.0]** Standard techniques, such as Western blot, may be employed to determine the presence or relative amount of protein translated from this mRNA (see, for example, Ausubel et al. (1988) "Current Protocols in Molecular Biology", Wiley, New York). In this method, total cell proteins are extracted, separated by gel electrophoresis, transferred to a matrix such as nitrocellulose and incubated with a probe, such as an antibody, which binds specifically to the desired protein. This probe is usually provided directly or indirectly with a chemiluminescent or colorimetric marker, which can be detected readily. The presence and the observed amount of marker indicates the presence and the amount of the sought mutant protein in the cell. However, other methods are also known.

**[0444.0.0.0]** Example 6: Growth of genetically modified Corynebacterium glutamicum: media and culture conditions

**[0445.0.0.0]** Genetically modified Corynebacteria are grown in synthetic or natural growth media. A number of different growth media for Corynebacteria are known and widely available (Lieb et al. (1989) Appl. Microbiol. Biotechnol. 32: 205-210; von der Osten et al. (1998) Biotechnology Letters 11: 11-16; Patent DE 4 120 867; Liebl (1992) "The Genus Corynebacterium", in: The Procaryotes, Vol. II, Balows, A., et al., Ed. Springer-Verlag).

**[0446.0.0.0]** Said media, which can be used according to the invention usually consist of one or more carbon sources, nitrogen sources, inorganic salts, vitamins and trace elements. Preferred carbon sources are sugars such as mono-, di- or polysaccharides. Examples of very good carbon sources are glucose, fructose, mannose, galactose, ribose, sorbose, ribulose, lactose, maltose, sucrose, raffinose, starch or cellulose. Sugars may also be added to the media via complex compounds such as molasses or other by-products of sugar refining. It may also be advantageous to add mixtures of various carbon sources. Other possible carbon sources are alcohols and/or organic acids such as methanol, ethanol, acetic acid or lactic acid. Nitrogen sources are usually organic or inorganic nitrogen compounds or materials containing said compounds. Examples of nitrogen sources include ammonia gas, aqueous ammonia solutions or ammonium salts such as NH₄Cl, or (NH₄)₂SO₄, NH₄OH, nitrates, urea, amino acids or complex nitrogen sources such as cornsteep liquor, soybean flour, soybean protein, yeast extract, meat extract and others. Mixtures of the above nitrogen sources may be used advantageously.

**[0447.0.0.0]** Inorganic salt compounds, which may be included in the media comprise the chloride, phosphorus or sulfate salts of calcium, magnesium, sodium, cobalt, molybdenum, potassium, manganese, zinc, copper and iron. Chelating agents may be added to the medium in order to keep the metal ions in solution. Particularly suitable chelating agents include dihydroxyphenols such as catechol or protocatechulate or organic acids such as citric acid. The media usually also contain other growth factors such as vitamins or growth promoters, which include, for example, biotin, riboflavin, thiamine, folic acid, nicotinic acid, panthothenate and pyridoxine. Growth factors and salts are frequently derived from complex media components such as yeast extract, molasses, cornsteep liquor and the like. The exact composition of the compounds used in the media depends heavily on the particular experiment and is decided upon individually for each specific case. Information on the optimization of media can be found in the textbook "Applied Microbiol. Physiology, A Practical Approach" (Ed. P.M. Rhodes, P.F. Stanbury, IRL Press (1997) S. 53-73, ISBN 0 19 963577 3). Growth media can also be obtained from commercial suppliers, for example Standard 1 (Merck) or BHI (Brain heart infusion, DIFCO) and the like.

**[0448.0.0.0]** All media components are sterilized, either by heat (20 min at 1.5 bar und 121°C) or by filter sterilization. The components may be sterilized either together or, if required, separately. All media components may be present at the start of the cultivation or added continuously or batchwise, as desired.

**[0449.0.0.0]** The culture conditions are defined separately for each experiment. The temperature is normally between 15°C and 45°C and may be kept constant or may be altered during the experiment. The pH of the medium should be in the range from 5 to 8.5, preferably around 7.0, and can be maintained by adding buffers to the media. An example of a buffer for this purpose is a potassium phosphate buffer. Synthetic buffers such as MOPS, HEPES, ACES and the like may be used as an alternative or simultaneously. The culture pH value may also be kept constant during the culture period by addition of, for example, NaOH or NH₄OH. If complex media components such as yeast extract are used, additional buffers are required less since many complex compounds have a high buffer capacity. When using a fermenter for the culture of microorganisms, the pH value can also be regulated using gaseous ammonia.

**[0450.0.0.0]** The incubation period is generally in a range of from several hours to several days. This time period is selected in such a way that the maximum amount of product accumulates in the fermentation broth. The growth experiments, which are disclosed can be carried out in a multiplicity of containers such as microtiter plates, glass tubes, glass flasks or glass or metal fermenters of various sizes. To screen a large number of clones, the microorganisms should be grown in microtiter plates, glass tubes or shake flasks, either using simple flasks or baffle flasks. 100 ml shake flasks filled with 10% (based on the volume) of the growth medium required are preferably used. The flasks should be shaken on an orbital shaker (amplitude 25 mm) at a rate ranging from 100 to 300 rpm. Evaporation losses can be reduced by maintaining a humid atmosphere; as an alternative, a mathematical correction should be carried out for the evaporation losses.

**[0451.0.0.0]** If genetically modified clones are examined, an unmodified control clone, or a control clone, which contains the basic plasmid without insertion, should also be included in the tests. If a transgenic sequence is expressed, a control clone should advantageously again be included in these tests. The medium is advantageously inoculated to an OD600 of 0.5 to 1.5 using cells which have been grown on agar plates, such as CM plates (10 g/l glucose, 2.5 g/l NaCl, 2 g/l urea, 10 g/l polypeptone, 5 g/l yeast extract, 5 g/l meat extract, 22 g/l agar, pH value 6.8 established with 2M NaOH), which have been incubated at 30°C. The media are inoculated for example by introducing of a preculture of seed organisms..

**[0451.1.0.0]** For example, the media are inoculated by introducing of a saline solution of C. glutamicum cells from CM plates or by addition of a liquid preculture of this bacterium.

**[0452.0.0.0]** Example 7: In-vitro analysis of the function of the proteins encoded by the transformed sequences

**[0453.0.0.0]** The determination of the activities and kinetic parameters of enzymes is well known in the art. Experiments for determining the activity of a specific modified enzyme must be adapted to the specific activity of the wild-enzyme type, which is well within the capabilities of the skilled worker. Overviews of enzymes in general and specific details regarding the structure, kinetics, principles, methods, applications and examples for the determination of many enzyme activities can be found for example in the following literature: Dixon, M., and Webb, E.C: (1979) Enzymes, Longmans, London; Fersht (1985) Enzyme Structure and Mechanism, Freeman, New York; Walsh (1979) Enzymatic Reaction Mechanisms. Freeman, San Francisco; Price, N.C., Stevens, L. (1982) Fundamentals of Enzymology. Oxford Univ. Press: Oxford; Boyer, P.D: Ed. (1983) The Enzymes, 3rd Ed. Academic Press, New York; Bisswanger, H. (1994) Enzymkinetik, 2nd Ed. VCH, Weinheim (ISBN 3527300325); Bergmeyer, H.U., Bergmeyer, J., Graßl, M. Ed. (1983-1986) Methods of Enzymatic Analysis, 3rd Ed. Vol. I-XII, Verlag Chemie: Weinheim; and Ullmann's Encyclopedia of Industrial Chemistry (1987) Vol. A9, "Enzymes", VCH, Weinheim, pp. 352-363.

**[0454.0.0.0]** Example 8: Analysis of the effect of the nucleic acid molecule on the production of the amino acids

**[0455.0.0.0]** The effect of the genetic modification in C. glutamicum on the production of an amino acid can be determined by growing the modified microorganisms under suitable conditions (such as those described above) and analyzing the medium and/or the cellular components for the increased production of the amino acid. Such analytical techniques are well known to the skilled worker and encompass spectroscopy, thin-layer chromatography, various types of staining methods, enzymatic and microbiological methods and analytical chromatography such as high-performance liquid chromatography (see, for example, Ullman, Encyclopedia of Industrial Chemistry, Vol. A2, pp. 89-90 and pp. 443-613, VCH: Weinheim (1985); Fallon, A., et al., (1987) "Applications of HPLC in Biochemistry" in: Laboratory Techniques in Biochemistry and Molecular Biology, Vol. 17; Rehm et al. (1993) Biotechnology, Vol. 3, Chapter III: "Product recovery and purification", pp. 469-714, VCH: Weinheim; Belter, P.A. et al. (1988) Bioseparations: downstream processing for Biotechnology, John Wiley and Sons; Kennedy, J.F. and Cabral, J.M.S. (1992) Recovery processes for biological Materials, John Wiley and Sons; Shaeiwitz, J.A. and Henry, J.D. (1988) Biochemical Separations, in Ullmann's Encyclopedia of Industrial Chemistry, Vol. B3; chapter 11, pp. 1-27, VCH: Weinheim; and Dechow, F.J. (1989) Separation and purification techniques in biotechnology, Noyes Publications).

**[0456.0.0.0]** In addition to the determination of the fermentation end product, other components of the metabolic pathways which are used for the production of the desired compound, such as intermediates and by-products, may also be analyzed in order to determine the total productivity of the organism, the yield and/or production efficiency of the compound. The analytical methods encompass determining the amounts of nutrients in the medium (for example sugars, hydrocarbons, nitrogen sources, phosphate and other ions), determining biomass composition and growth, analyzing the production of ordinary metabolites from biosynthetic pathways and measuring gases generated during the fermentation. Standard methods for these are described in Applied Microbial Physiology; A Practical Approach, P.M. Rhodes and P.F. Stanbury, Ed. IRL Press, pp. 103-129; 131-163 and 165-192 (ISBN: 0199635773) and the references cited therein.

**[0457.0.0.0]** Example 9: Purification of the amino acid

**[0458.0.0.0]** The amino acid can be recovered from cells or from the supernatant of the above-described culture by a variety of methods known in the art. For example, the culture supernatant is recovered first. To this end, the cells are harvested from the culture by slow centrifugation. Cells can generally be disrupted or lysed by standard techniques such as mechanical force or sonication. The cell debris is removed by centrifugation and the supernatant fraction, if appropriate together with the culture supernatant, is used for the further purification of the amino acid. However, it is also possible to process the supernatant alone if the amino acid is present in the supernatant in sufficiently high a concentration. In this case, the amino acid, or the amino acid mixture, can be purified further for example via extraction and/or salt precipitation or via ion-exchange chromatography.

**[0459.0.0.0]** If required and desired, further chromatography steps with a suitable resin may follow, the amino acid, but not many contaminants in the sample, being retained on the chromatography resin or the contaminants, but not the sample with the product (amino acid), being retained on the resin. If necessary, these chromatography steps may be repeated, using identical or other chromatography resins. The skilled worker is familiar with the selection of suitable chromatography resin and the most effective use for a particular molecule to be purified. The purified product can be concentrated by filtration or ultrafiltration and stored at a temperature at which maximum product stability is ensured. Many purification methods, which are not limited to the above purification method are known in the art. They are described, for example, in Bailey, J.E. & Ollis, D.F. Biochemical Engineering Fundamentals, McGraw-Hill: New York (1986).

**[0460.0.0.0]** Identity and purity of the amino acid isolated can be determined by standard techniques of the art. They encompass high-performance liquid chromatography (HPLC), spectroscopic methods, mass spectrometry (MS), staining methods, thin-layer chromatography, NIRS, enzyme assay or microbiological assays. These analytical methods are compiled in: Patek et al. (1994) Appl. Environ. Microbiol. 60: 133-140; Malakhova et al. (1996) Biotekhnologiya 11: 27-32; and Schmidt et al. (1998) Bioprocess Engineer. 19: 67-70. Ulmann's Encyclopedia of Industrial Chemistry (1996) Vol. A27, VCH: Weinheim, pp. 89-90, pp. 521-540, pp. 540-547, pp. 559-566, 575-581 and pp. 581-587; Michal, G (1999) Biochemical Pathways: An Atlas of Biochemistry and Molecular Biology, John Wiley and Sons; Fallon, A. et al. (1987) Applications of HPLC in Biochemistry in: Laboratory Techniques in Biochemistry and Molecular Biology, Vol. 17.

**[0461.0.0.0]** Example 10: Cloning SEQ ID NO: 1 for the expression in plants

**[0462.0.0.0]** Unless otherwise specified, standard methods as described in Sambrook et al., Molecular Cloning: A laboratory manual, Cold Spring Harbor 1989, Cold Spring Harbor Laboratory Press are used.

**[0463.0.0.0]** SEQ ID NO: 1 is amplified by PCR as described in the protocol of the Pfu Turbo or DNA Herculase polymerase (Stratagene).

**[0464.0.0.0]** The composition for the protocol of the *Pfu* Turbo DNA polymerase was as follows: 1x PCR buffer (Stratagene), 0.2 mM of each dNTP, 100 ng genomic DNA of *Saccharomyces cerevisiae* (strain S288C; Research Genetics, Inc., now Invitrogen) or *Escherichia coli* (strain MG1655; *E. coli* Genetic Stock Center), 50 pmol forward primer, 50 pmol reverse primer, 2.5 u *Pfu* Turbo DNA polymerase. The amplification cycles were as follows:

**[0465.0.0.0]** 1 cycle of 3 minutes at 94-95°C, followed by 25-36 cycles of in each case 1 minute at 95°C or 30 seconds at 94°C, 45 seconds at 50°C, 30 seconds at 50°C or 30 seconds at 55°C and 210-480 seconds at 72°C, followed by 1 cycle of 8 minutes at 72°C, then 4°C. The composition for the protocol of the Herculase polymerase was as follows: 1x PCR buffer (Stratagene), 0.2 mM of each dNTP, 100 ng genomic DNA of *Saccharomyces cerevisiae* (strain S288C; Research Genetics, Inc., now Invitrogen) or *Escherichia coli* (strain MG1655; *E. coli* Genetic Stock Center), 50 pmol forward primer, 50 pmol reverse primer, 2.5 u Herculase polymerase. The amplification cycles were as follows:

**[0466.0.0.0]** 1 cycle of 2-3 minutes at 94°C, followed by 25-30 cycles of in each case 30 seconds at 94°C, 30 seconds at 55-60°C and 5-10 minutes at 72°C, followed by 1 cycle of 10 minutes at 72°C, then 4°C.

**[0467.0.0.0]** The following primer sequences were selected for the gene SEQ ID NO: 1:
i) forward primer (SEQ ID NO: 3)
   ATGGAACAGAACAGGTTCAAGAAAG
ii) reverse primer (SEQ ID NO: 4)
   TTACAGTTTTTGTTTAGTCGTTTTAAC

**[0468.0.0.0]** Thereafter, the amplificate was purified over QIAquick columns following the standard protocol (Qiagen).

**[0469.0.0.0]** For the cloning of PCR-products, produced by *Pfu* Turbo DNA polymerase, the vector DNA (30 ng) was restricted with Smal following the standard protocol (MBI Fermentas) and stopped by addition of high-salt buffer. The restricted vector fragments were purified via Nucleobond columns using the standard protocol (Macherey-Nagel). Thereafter, the linearized vector was dephosphorylated following the standard protocol (MBI Fermentas).

**[0470.0.0.0]** The PCR-products, produced by *Pfu* Turbo DNA polymerase, were directly cloned into the processed binary vector. The PCR-products, produced by *Pfu* Turbo DNA polymerase, were phosphorylated using a T4 DNA polymerase using a standard protocol (e.g. MBI Fermentas) and cloned into the processed binary vector.

**[0471.0.0.0]** The DNA termini of the PCR-products, produced by Herculase DNA polymerase, were blunted in a second synthesis reaction using Pfu Turbo DNA polymerase. The composition for the protocol of the blunting the DNA-termini was as follows: 0.2 mM blunting dTTP and 1.25 u *Pfu* Turbo DNA polymerase. The reaction was incubated at 72°C for 30 minutes. Then the PCR-products were cloned into the processed vector as well. The DNA termini of the PCR-products, produced by Herculase DNA polymerase, were blunted in a second synthesis reaction using *Pfu* Turbo DNA polymerase. The composition for the protocol of the blunting the DNA-termini was as follows: 0.2 mM blunting dTTP and 1.25 u *Pfu* Turbo DNA polymerase. The reaction was incubated at 72°C for 30 minutes. Then the PCR-products were phosphorylated using a T4 DNA polymerase using a standard protocol (e.g. MBI Fermentas) and cloned into the processed vector as well.

**[0472.0.0.0]** A binary vector comprising a selection cassette (promoter, selection marker, terminator) and an expression cassette with promoter, cloning cassette and terminator sequence between the T-DNA border sequences was used. In addition to those within the cloning cassette, the binary vector has no Smal cleavage site. Binary vectors which can be used are known to the skilled worker; an overview of binary vectors and their use can be found in Hellens, R., Mullineaux, P. and Klee H., [(2000) "A guide to Agrobacterium binary vectors", Trends in Plant Science, Vol. 5 No.10, 446-451. Depending on the vector used, cloning may advantageously also be carried out via other restriction enzymes. Suitable advantageous cleavage sites can be added to the ORF by using suitable primers for the PCR amplification.

**[0473.0.0.0]** Approximately 30 ng of prepared vector and a defined amount of prepared amplificate were mixed and ligated by addition of ligase.

**[0474.0.0.0]** The ligated vectors were transformed in the same reaction vessel by addition of competent *E. coli* cells (strain DH5alpha) and incubation for 20 minutes at 1°C followed by a heat shock for 90 seconds at 42°C and cooling to 4°C. Then, complete medium (SOC) was added and the mixture was incubated for 45 minutes at 37°C. The entire mixture was subsequently plated onto an agar plate with antibiotics (selected as a function of the binary vector used) and incubated overnight at 37°C.

**[0475.0.0.0]** The outcome of the cloning step was verified by amplification with the aid of primers which bind upstream and downstream of the integration site, thus allowing the amplification of the insertion. In addition combinations of the above mentioned gene specific primers and upstream and downstream primers were used in PCR reactions to identify clones with the correct insert orientation. The amplifications were carried as described in the protocol of *Taq* DNA polymerase (Gibco-BRL).

**[0476.0.0.0]** The amplification cycles were as follows: 1 cycle of 5 minutes at 94°C, followed by 35 cycles of in each case 15 seconds at 94°C, 15 seconds at 50-66°C and 5 minutes at 72°C, followed by 1 cycle of 10 minutes at 72°C, then 4°C.

**[0477.0.0.0]** Several colonies were checked, but only one colony for which a PCR product of the expected size was detected was used in the following steps.

**[0478.0.0.0]** A portion of this positive colony was transferred into a reaction vessel filled with complete medium (LB) and incubated overnight at 37°C. The LB medium contained an antibiotic chosen to suit the binary vector (see above) used and the resistance gene present therein in order to select the clone.

**[0479.0.0.0]** The plasmid preparation was carried out as specified in the Qiaprep standard protocol (Qiagen).

**[0480.0.0.0]** Example 11: Generation of transgenic plants which express SEQ ID NO: 1

**[0481.0.0.0]** 1 ng of the plasmid DNA isolated was transformed by electroporation into competent cells of *Agrobacterium tumefaciens*, of strain GV 3101 pMP90 (Koncz and Schell, Mol. Gen. Gent. 204, 383-396, 1986) . The choice of the agrobacterial strain depends on the choice of the binary vector. An overview of possible strains and their properties is found in Hellens, R., Mullineaux, P. and Klee H., (2000) " A guide to Agrobacterium binary vectors, Trends in Plant Science, Vol. 5 No.10, 446-451. Thereafter, complete medium (YEP) was added and the mixture was transferred into a fresh reaction vessel for 3 hours at 28°C. Thereafter, all of the reaction mixture was plated onto YEP agar plates supplemented with the respective antibiotics, for example rifampicin and gentamycin for GV31 01 pMP90, and a further antibiotic for the selection onto the binary vector, was plated, and incubated for 48 hours at 28°C.

**[0482.0.0.0]** The agrobacteria generated in Example 10, which contains the plasmid construct were then used for the transformation of plants.

**[0483.0.0.0]** A colony was picked from the agar plate with the aid of a pipette tip and taken up in 3 ml of liquid TB medium, which also contained suitable antibiotics, depending on the agrobacterial strain and the binary plasmid. The preculture was grown for 48 hours at 28°C and 120 rpm.

**[0484.0.0.0]** 400 ml of LB medium containing the same antibiotics as above were used for the main culture. The preculture was transferred into the main culture. It was grown for 18 hours at 28°C and 120 rpm. After centrifugation at 4 000 rpm, the pellet was resuspended in infiltration medium (MS medium, 10% sucrose).

**[0485.0.0.0]** In order to grow the plants for the transformation, dishes (Piki Saat 80, green, provided with a screen bottom, 30 x 20 x 4.5 cm, from Wiesauplast, Kunststofftechnik, Germany) were half-filled with a GS 90 substrate (standard soil, Werkverband E.V., Germany). The dishes were watered overnight with 0.05% Proplant solution (Chimac-Apriphar, Belgium). *Arabidopsis thaliana* C24 seeds (Nottingham Arabidopsis Stock Centre, UK ; NASC Stock N906) were scattered over the dish, approximately 1 000 seeds per dish. The dishes were covered with a hood and placed in the stratification facility (8 h, 110 µ µmol/m²/s⁻¹, 22°C; 16 h, dark, 6°C). After 5 days, the dishes were placed into the short-day controlled environment chamber (8 h 130 µmol/m²/s⁻¹, 22°C; 16 h, dark 20°C), where they remained for approximately 10 days until the first true leaves had formed.

**[0486.0.0.0]** The seedlings were transferred into pots containing the same substrate (Teku pots, 7 cm, LC series, manufactured by Pöppelmann GmbH & Co, Germany). Five plants were pricked out into each pot. The pots were then returned into the short-day controlled environment chamber for the plant to continue growing.

**[0487.0.0.0]** After 10 days, the plants were transferred into the greenhouse cabinet (supplementary illumination, 16 h, 340 µE, 22°C; 8 h, dark, 20°C), where they were allowed to grow for further 17 days.

**[0488.0.0.0]** For the transformation, 6-week-old Arabidopsis plants which had just started flowering were immersed for 10 seconds into the above-described agrobacterial suspension which had previously been treated with 10 µl Silwett L77 (Crompton S.A., Osi Specialties, Switzerland). The method in question is described in Clough and Bent, 1998 (Clough, JC and Bent, AF. 1998 Floral dip: a simplified method for Agrobacterium-mediated transformation of Arabidopsis thaliana, Plant J. 16:735-743.

**[0489.0.0.0]** The plants were subsequently placed for 18 hours into a humid chamber. Thereafter, the pots were returned to the greenhouse for the plants to continue growing. The plants remained in the greenhouse for another 10 weeks until the seeds were ready for harvesting.

**[0490.0.0.0]** Depending on the resistance marker used for the selection of the transformed plants the harvested seeds were planted in the greenhouse and subjected to a spray selection or else first sterilized and then grown on agar plates supplemented with the respective selection agent. In case of BASTA®-resistance, plantlets were sprayed four times at an interval of 2 to 3 days with 0.02 % BASTA® and transformed plants were allowed to set seeds. The seeds of the transgenic *A. thaliana* plants were stored in the freezer (at -20°C).

**[0491.0.0.0]** Example 12: Plant culture for bioanalytical analyses

**[0492.0.0.0]** For the bioanalytical analyses of the transgenic plants, the latter were grown uniformly a specific culture facility. To this end the GS-90 substrate as the compost mixture was introduced into the potting machine (Laible System GmbH, Singen, Germany) and filled into the pots. Thereafter, 35 pots were combined in one dish and treated with Previcur. For the treatment, 25 ml of Previcur were taken up in 10 I of tap water. This amount was sufficient for the treatment of approximately 200 pots. The pots were placed into the Previcur solution and additionally irrigated overhead with tap water without Previcur. They were used within four days..

**[0493.0.0.0]** For the sowing, the seeds, which had been stored in the refrigerator (at - 20°C), were removed from the Eppendorf tubes with the aid of a toothpick and transferred into the pots with the compost. In total, approximately 5 to 12 seeds were distributed in the middle of the pot.

**[0494.0.0.0]** After the seeds had been sown, the dishes with the pots were covered with matching plastic hood and placed into the stratification chamber for 4 days in the dark at 4°C. The humidity was approximately 90%. After the stratification, the test plants were grown for 22 to 23 days at a 16-h-light, 8-h-dark rhythm at 20°C, an atmospheric humidity of 60% and a CO₂ concentration of approximately 400 ppm. The light sources used were Powerstar HQI-T 250 W/D Daylight lamps from Osram, which generate a light resembling the solar color spectrum with a light intensity of approximately 220 µE/m2/s-1.

**[0495.0.0.0]** When the plants were 8, 9 and 10 days old, they were subjected to selection for the resistance marker Approximately 1400 pots with transgenic plants were treated with 1l 0,015% vol/vol of Basta® (Glufosinate-ammonium) solution in water (Aventis Cropsience, Germany). After a further 3 to 4 days, the transgenic, resistant seedlings (plantlets in the 4-leaf stage) could be distinguished clearly from the untransformed plantlets. The nontransgenic seedlings were bleached or dead. The transgenic resistance plants were thinned when they had reached the age of 14 days. The plants, which had grown best in the center of the pot were considered the target plants. All the remaining plants were removed carefully with the aid of metal tweezers and discarded.

**[0496.0.0.0]** During their growth, the plants received overhead irrigation with distilled water (onto the compost) and bottom irrigation into the placement grooves. Once the grown plants had reached the age of 23 days, they were harvested.

**[0497.0.0.0]** Example 13: Metabolic analysis of transformed plants

**[0498.0.0.0]** The modifications identified in accordance with the invention, in the content of above-described metabolites, were identified by the following procedure.

### a) sampling and storage of the samples

**[0499.0.0.0]** Sampling was performed directly in the controlled-environment chamber. The plants were cut using small laboratory scissors, rapidly weighed on laboratory scales, transferred into a pre-cooled extraction sleeve and placed into an aluminum rack cooled by liquid nitrogen. If required, the extraction sleeves can be stored in the freezer at -80°C. The time elapsing between cutting the plant to freezing it in liquid nitrogen amounted to not more than 10 to 20 seconds.

### b) Lyophilization

**[0500.0.0.0]** During the experiment, care was taken that the plants either remained in the deep-frozen state (temperatures < -40°C) or were freed from water by lyophilization until the first contact with solvents.

**[0501.0.0.0]** The aluminum rack with the plant samples in the extraction sleeves was placed into the pre-cooled (-40°C) lyophilization facility. The initial temperature during the main drying phase was-35°C and the pressure was 0.120 mbar. During the drying phase, the parameters were altered following a pressure and temperature program. The final temperature after 12 hours was +30°C and the final pressure was 0.001 to 0.004 mbar. After the vacuum pump and the refrigerating machine had been switched off, the system was flushed with air (dried via a drying tube) or argon.

### c) Extraction

**[0502.0.0.0]** Immediately after the lyophilization apparatus had been flushed, the extraction sleeves with the lyophilized plant material were transferred into the 5 ml extraction cartridges of the ASE device (Accelerated Solvent Extractor ASE 200 with Solvent Controller and AutoASE software (DIONEX)).

**[0503.0.0.0]** The 24 sample positions of an ASE device (Accelerated Solvent Extractor ASE 200 with Solvent Controller and AutoASE software (DIONEX)) were filled with plant samples, including some samples for testing quality control.

**[0504.0.0.0]** The polar substances were extracted with approximately 10 ml of methanol/water (80/20, v/v) at T = 70°C and p = 140 bar, 5 minutes heating-up phase, 1 minute static extraction. The more lipophilic substances were extracted with approximately 10 ml of methanol/dichloromethane (40/60, v/v) at T = 70°C and p = 140 bar, 5 minute heating-up phase, 1 minute static extraction. The two solvent mixtures were extracted into the same glass tubes (centrifuge tubes, 50 ml, equipped with screw cap and pierceable septum for the ASE (DIONEX)).

**[0505.0.0.0]** The solution was treated with internal standards: ribitol, L-glycine-2,2-d₂, L-alanine-2,3,3,3-d₄, methionine-methyl-d₃, and α-methylglucopyranoside and methyl nonadecanoate, methyl undecanoate, methyl tridecanoate, methyl pentadecanoate, methyl nonacosanoate.

**[0506.0.0.0]** The total extract was treated with 8 ml of water. The solid residue of the plant sample and the extraction sleeve were discarded.

**[0507.0.0.0]** The extract was shaken and then centrifuged for 5 to 10 minutes at least at 1 400 g in order to accelerate phase separation. 1 ml of the supernatant methanol/water phase ("polar phase", colorless) was removed for the further GC analysis, and 1 ml was removed for the LC analysis. The remainder of the methanol/water phase was discarded. 0.5 ml of the organic phase ("lipid phase", dark green) was removed for the further GC analysis and 0.5 ml was removed for the LC analysis. All the portions removed were evaporated to dryness using the IR Dancer infrared vacuum evaporator (Hettich). The maximum temperature during the evaporation process did not exceed 40°C. Pressure in the apparatus was not less than 10 mbar.

### d) Processing the lipid phase for the LC/MS or LC/MS/MS analysis

**[0508.0.0.0]** The lipid extract, which had been evaporated to dryness was taken up in mobile phase. The HPLC was run with gradient elution.

**[0509.0.0.0]** The polar extract, which had been evaporated to dryness was taken up in mobile phase. The HPLC was run with gradient elution.

### e) Derivatization of the lipid phase for the GC/MS analysis

**[0510.0.0.0]** For the transmethanolysis, a mixture of 140 µl of chloroform, 37 µl of hydrochloric acid (37% by weight HCl in water), 320 µl of methanol and 20 µl of toluene was added to the evaporated extract. The vessel was sealed tightly and heated for 2 hours at 100°C, with shaking. The solution was subsequently evaporated to dryness. The residue was dried completely.

**[0511.0.0.0]** The methoximation of the carbonyl groups was carried out by reaction with methoxyamine hydrochloride (5 mg/ml in pyridine, 100 µl for 1.5 hours at 60°C) in a tightly sealed vessel. 20 µl of a solution of odd-numbered, straight-chain fatty acids (solution of each 0.3 mg/mL of fatty acids from 7 to 25 carbon atoms and each 0.6 mg/mL of fatty acids with 27, 29 and 31 carbon atoms in 3/7 (v/v) pyridine/toluene) were added as time standards. Finally, the derivatization with 100 µl of N-methyl-N-(trimethylsilyl)-2,2,2-trifluoroacetamide (MSTFA) was carried out for 30 minutes at 60°C, again in the tightly sealed vessel. The final volume before injection into the GC was 220 µl.

### f) Derivatization of the polar phase for the GC/MS analysis

**[0512.0.0.0]** The methoximation of the carbonyl groups was carried out by reaction with methoxyamine hydrochloride (5 mg/ml in pyridine, 50 µl for 1.5 hours at 60°C) in a tightly sealed vessel. 10 µl of a solution of odd-numbered, straight-chain fatty acids (solution of each 0.3 mg/mL of fatty acids from 7 to 25 carbon atoms and each 0.6 mg/mL of fatty acids with 27, 29 and 31 carbon atoms in 3/7 (v/v) pyridine/toluene) were added as time standards. Finally, the derivatization with 50 µl of N-methyl-N-(trimethylsilyl)-2,2,2-trifluoroacetamide (MSTFA) was carried out for 30 minutes at 60°C, again in the tightly sealed vessel. The final volume before injection into the GC was 110 µl.

### g) Analysis of the various plant samples

**[0513.0.0.0]** The samples were measured in individual series of 20 plant samples each (also referred to as sequences), each sequence containing at least 5 wild-type plants as controls. The peak area of each analyte was divided by the peak area of the respective internal standard. The data were standardized for the fresh weight established for the plant. The values calculated thus were related to the wild-type control group by being divided by the mean of the corresponding data of the wild-type control group of the same sequence. The values obtained were referred to as ratio_by_WT, they are comparable between sequences and indicate how much the analyte concentration in the mutant differs in relation to the wild-type control. Appropiate controls were done before to proof that the vector and transformation procedure itself has no significant influence on the metabolic composition of the plants. Therefore the desribed changes in comparison with wildtypes were caused by the introduced genes.

**[0514.0.0.0]** As an alternative, the amino acids can be detected advantageously via HPLC separation in ethanolic extract as described by Geigenberger et al. (Plant Cell & Environ, 19, 1996: 43-55).
The results of the different plant analyses can be seen from the table 1 which follows:

| **ORF** | **ANNOTATION** | **Metabolite** | **Min** | **Max** | **Method** |
|---|---|---|---|---|---|
| YBL015W | acetyl-CoA hydrolase | Methionine | 1,42 | 2,16 | LC |
| YER173W | checkpoint protein, | Methionine | 1,35 | 1,60 | GC |
| YLR375W | involved in pre-tRNA splicing and in uptake of branched-chain amino acids | Methionine | 1,27 | 2,93 | LC + GC |
| YOR084W | putative peroxisomal lipase | Methionine | 3,18 | 3,18 | GC |
| b1829 | heat shock protein with protease activity | Methionine | 1,29 | 3,73 | GC |
| b4232 | fructose-1,6-bisphosphatase | Methionine | 1.20 | 1.21 | LC |
| b0464 | transcriptional repressor for multidrug efflux pump (TetR/AcrR family) | Methionine | 1.35 | 4.66 | GC |
| b1343 | ATP-dependent RNA helicase, stimulated by 23S rRNA | Methionine | 1.38 | 1.51 | GC |
| b2414 | subunit of cysteine synthase A and O-acetylserine sulfhydrolase A, PLP-dependent enzyme | Methionine | 1.37 | 1.75 | LC |
| b2762 | 3'-phosphoadenosine 5'-phosphosulfate (PAPS) reductase | Methionine | 1.43 | 1.69 | LC + GC |

**[0515.0.0.0]** Column 3 shows the metabolite/respective fine chemical analyzed. Columns 4 and 5 shows the ratio of the analyzed metabolite/respective fine chemical between the transgenic plants and the wild type; Increase of the metabolites: Max: maximal x-fold (normalised to wild type)-Min: minimal x-fold (normalised to wild type). Decrease of the metabolites: Max: maximal x-fold (normalised to wild type) (minimal decrease), Min: minimal x-fold (normalised to wild type) (maximal decrease). Column 6 indicates the analytical method.

**[0516.0.0.0]** When the analyses were repeated independently, all results proved to be significant.

**[0517.0.0.0]** Example 14a: Engineering ryegrass plants by over-expressing the polynucleotide characterized in the invention, e.g. derived from Saccharomyces cerevisiae, E. coli or plants or an other organism

**[0518.0.0.0]** Seeds of several different ryegrass varieties can be used as explant sources for transformation, including the commercial variety Gunne available from Svalof Weibull seed company or the variety Affinity. Seeds are surface-sterilized sequentially with 1% Tween-20 for 1 minute, 100 % bleach for 60 minutes, 3 rinses with 5 minutes each with de-ionized and distilled H2O, and then germinated for 3-4 days on moist, sterile filter paper in the dark. Seedlings are further sterilized for 1 minute with 1% Tween-20, 5 minutes with 75% bleach, and rinsed 3 times with ddH2O, 5 min each.

**[0519.0.0.0]** Surface-sterilized seeds are placed on the callus induction medium containing Murashige and Skoog basal salts and vitamins, 20 g/l sucrose, 150 mg/l asparagine, 500 mg/l casein hydrolysate, 3 g/I Phytagel, 10 mg/l BAP, and 5 mg/l dicamba. Plates are incubated in the dark at 25°C for 4 weeks for seed germination and embryogenic callus induction.

**[0520.0.0.0]** After 4 weeks on the callus induction medium, the shoots and roots of the seedlings are trimmed away, the callus is transferred to fresh media, is maintained in culture for another 4 weeks, and is then transferred to MSO medium in light for 2 weeks. Several pieces of callus (11-17 weeks old) are either strained through a 10 mesh sieve and put onto callus induction medium, or are cultured in 100 ml of liquid ryegrass callus induction media (same medium as for callus induction with agar) in a 250 ml flask. The flask is wrapped in foil and shaken at 175 rpm in the dark at 23°C for 1 week. Sieving the liquid culture with a 40-mesh sieve is collected the cells. The fraction collected on the sieve is plated and is cultured on solid ryegrass callus induction medium for 1 week in the dark at 25°C. The callus is then transferred to and is cultured on MS medium containing 1% sucrose for 2 weeks.

**[0521.0.0.0]** Transformation can be accomplished with either Agrobacterium or with particle bombardment methods. An expression vector is created containing a constitutive plant promoter and the cDNA of the gene in a pUC vector. The plasmid DNA is prepared from E. coli cells using with Qiagen kit according to manufacturer's instruction. Approximately 2 g of embryogenic callus is spread in the center of a sterile filter paper in a Petri dish. An aliquot of liquid MSO with 10 g/l sucrose is added to the filter paper. Gold particles (1.0 µm in size) are coated with plasmid DNA according to method of Sanford et al., 1993 and are delivered to the embryogenic callus with the following parameters: 500 µg particles and 2 µg DNA per shot, 1300 psi and a target distance of 8.5 cm from stopping plate to plate of callus and 1 shot per plate of callus.

**[0522.0.0.0]** After the bombardment, calli are transferred back to the fresh callus development medium and maintained in the dark at room temperature for a 1-week period. The callus is then transferred to growth conditions in the light at 25 °C to initiate embryo differentiation with the appropriate selection agent, e.g. 250 nM Arsenal, 5 mg/l PPT or 50 mg/L Kanamycin. Shoots resistant to the selection agent are appearing and once rooted are transferred to soil.

**[0523.0.0.0]** Samples of the primary transgenic plants (T0) are analyzed by PCR to confirm the presence of T-DNA. These results are confirmed by Southern hybridization in which DNA is electrophoresed on a 1% agarose gel and transferred to a positively charged nylon membrane (Roche Diagnostics). The PCR DIG Probe Synthesis Kit (Roche Diagnostics) is used to prepare a digoxigenin-labelled probe by PCR, and used as recommended by the manufacturer.

**[0524.0.0.0]** Transgenic T0 ryegrass plants are propagated vegetatively by excising tillers. The transplanted tillers are maintained in the greenhouse for 2 months until well established. The shoots are defoliated and allowed to grow for 2 weeks.

**[0525.0.0.0]** Example 14b: Engineering soybean plants by over-expressing the polynucleotide characterized in the invention, e.g. derived from Saccharomyces cerevisiae, E. coli or plants or another organism

**[0526.0.0.0]** Soybean can be transformed according to the following modification of the method described in the Texas A&M patent US 5,164,310. Several commercial soybean varieties are amenable to transformation by this method. The cultivar Jack (available from the Illinois Seed Foundation) is commonly used for transformation. Seeds are sterilized by immersion in 70% (v/v) ethanol for 6 min and in 25 % commercial bleach (NaOCl) supplemented with 0.1% (v/v) Tween for 20 min, followed by rinsing 4 times with sterile double distilled water. Removing the radicle, hypocotyl and one cotyledon from each seedling propagates seven-day seedlings. Then, the epicotyl with one cotyledon is transferred to fresh germination media in petri dishes and incubated at 25 °C under a 16-hr photoperiod (approx. 100 µE-m-2s-1) for three weeks. Axillary nodes (approx. 4 mm in length) are cut from 3 - 4 week-old plants. Axillary nodes are excised and incubated in Agrobacterium LBA4404 culture.

**[0527.0.0.0]** Many different binary vector systems have been described for plant transformation (e.g. An, G. in Agrobacterium Protocols. Methods in Molecular Biology vol 44, pp 47-62, Gartland KMA and MR Davey eds. Humana Press, Totowa, New Jersey). Many are based on the vector pBIN19 described by Bevan (Nucleic Acid Research. 1984. 12:8711-8721) that includes a plant gene expression cassette flanked by the left and right border sequences from the Ti plasmid of Agrobacterium tumefaciens. A plant gene expression cassette consists of at least two genes - a selection marker gene and a plant promoter regulating the transcription of the cDNA or genomic DNA of the trait gene. Various selection marker genes can be used as described above, including the Arabidopsis gene encoding a mutated acetohydroxy acid synthase (AHAS) enzyme (US patents 57673666 and 6225105). Similarly, various promoters can be used to regulate the trait gene to provide constitutive, developmental, tissue or environmental regulation of gene transcription as described above. In this example, the 34S promoter (GenBank Accession numbers M59930 and X16673) is used to provide constitutive expression of the trait gene.

**[0528.0.0.0]** After the co-cultivation treatment, the explants are washed and transferred to selection media supplemented with 500 mg/L timentin. Shoots are excised and placed on a shoot elongation medium. Shoots longer than 1 cm are placed on rooting medium for two to four weeks prior to transplanting to soil.

**[0529.0.0.0]** The primary transgenic plants (T0) are analyzed by PCR to confirm the presence of T-DNA. These results are confirmed by Southern hybridization in which DNA is electrophoresed on a 1 % agarose gel and transferred to a positively charged nylon membrane (Roche Diagnostics). The PCR DIG Probe Synthesis Kit (Roche Diagnostics) is used to prepare a digoxigenin-labelled probe by PCR, and is used as recommended by the manufacturer.

**[0530.0.0.0]** Example 14c: Engineering corn plants by over-expressing the polynucleotide characterized in the invention, e.g. derived from Saccharomyces cerevisiae, E. coli or plants or another organism.

**[0530.1.0.0]** Amplification of for example SEQ ID NO: 1 was achieved as described in example 10 except that the upstream primer SEQ ID NO:3 and the reverse primer SEQ ID NO: 4 contained the following 5'extensions:
i) forward primer: 5'- GGGTCGCTCCTACGCG-3' SEQ ID NO: 68243
ii) reverse primer 5'- CTCGGGCTCGGCGTCC-3' SEQ ID NO: 68246

### Vector construction

**[0530.2.0.0]:** The maize transformation vector for constitutive expression was constructed as follows.
As base vectors, the vectors EG073qcz (SEQ ID NO 68240) and EG065qcz (SEQ ID NO: 68241) were chosen. The MCS from EG065qcz was deleted by digestion of the vector with Asp718 and PstI, followed by blunting of the vector using T4 DNA polymerase. The blunted vector was religated. The vector generated was called EG065-MCS. The LIC cassette was cloned in the vector EG065-MCS by hybridizing the following oligos, generating a DNA fragment with ends able to ligate into a Smal and SacI digested vector. This fragment was ligated into the vector EG065-MCS that had been digested with Smal and SacI. The generated vector was called EG065-LIC. The complete expression cassette comprising ScBV (Schenk (1999) Plant Mol Biol 39(6):1221-1230) promoter, LIC cassette and terminator was cut out of EG065-LIC with AscI and PacI and ligated into the vector EG073qcz that had previously been digested with AscI and PacI. The resulting binary vector for corn transformation was called pMME0607 (SEQ ID NO: 68242).
Oligo POCCLicMluISacIIfw: gggtcgctcctacgcgtcaatgatccgcggacgccgagcccgagct
   (SEQ ID NO: 68244)
Oligo POCCLicMluISacIrev: cgggctcggcgtccgcggatcattgacgcgtaggagcgaccc
   (SEQ ID NO: 68245)
For cloning of a polynucleotide of the invention, for example the ORF of SEQ ID NO: 1, from S. *cerevisiae* the vector DNA was treated with the restriction enzyme MluI and SacII. The reaction was stopped by inactivation at 70°C for 20 minutes and purified over QIAquick columns following the standard protocol (Qiagen).
Then the PCR-product representing the amplified ORF and the vector DNA were treated with T4 DNA polymerase according to the standard protocol (MBI Fermentas) to produce single stranded overhangs with the parameters 1 unit T4 DNA polymerase at 37°C for 2-10 minutes for the vector and 1 u T4 DNA polymerase at 15°C for 10-60 minutes for the PCR product representing SEQ ID NO: 1.
The reaction was stopped by addition of high-salt buffer and purified over QIAquick columns following the standard protocol (Qiagen).
Approximately 30 ng of prepared vector and a defined amount of prepared amplificate were mixed and hybridized at 65°C for 15 minutes followed by 37°C 0,1 °C/1 seconds, followed by 37°C 10 minutes, followed by 0,1 °C/1 seconds, then 4°C.
The ligated constructs were transformed in the same reaction vessel by addition of competent *E. coli* cells (strain DH5alpha) and incubation for 20 minutes at 1°C followed by a heat shock for 90 seconds at 42°C and cooling to 4°C. Then, complete medium (SOC) was added and the mixture was incubated for 45 minutes at 37°C. The entire mixture was subsequently plated onto an agar plate with 0.05 mg/ml kanamycine and incubated overnight at 37°C.
The outcome of the cloning step was verified by amplification with the aid of primers which bind upstream and downstream of the integration site, thus allowing the amplification of the insertion. The amplifications were carried as described in the protocol of *Taq* DNA polymerase (Gibco-BRL).
The amplification cycles were as follows: 1 cycle of 5 minutes at 94°C, followed by 35 cycles of in each case 15 seconds at 94°C, 15 seconds at 50-66°C and 5 minutes at 72°C, followed by 1 cycle of 10 minutes at 72°C, then 4°C.
Several colonies were checked, but only one colony for which a PCR product of the expected size was detected was used in the following steps.
A portion of this positive colony was transferred into a reaction vessel filled with complete medium (LB) supplemented with kanamycin () and incubated overnight at 37°C.
The plasmid preparation was carried out as specified in the Qiaprep standard protocol (Qiagen).

### Example 14 c.a.: Corn Transformation

**[0530.3.0.0]** The preparation of the immature embryos and Agrobacterium were basically as stated in US 5,591,616. In brief, the Agrobacterium strain LBA4404 transformed with the plasmid by a standard method, such as the triple cross method or the electroporation, was grown on LB plates for 2 days prior to cocultivation. A loop of cells was resuspended in liquid infection media at an O.D. of approximately 1.0. Immature Embryos of about 1.5mm in size were incubated in the soln of agrobacterium for around 30 minutes. Excised embryos were removed from liquid and then co- cultivated in the dark at 22°C with Agrobacterium tumefaciens on solid MS-based callus induction medium containing 2 mg/l 2,4-D, 10um AgNO3, and 200um Acetosyringone. After several days of co-cultivation, embryos were transferred to MS-based media containing 2mg/l 2,4, 10um AgNO3 and 200mg/l Timentin the dark at 27°C for 1 week. Embryos were transferred to MS-based selection media containing imidazoline herbicide (500nM Pursuit) as a selection agent in the dark for 3 weeks. After 3 weeks putative transgenic events were transferred to an MS-based media containing 2mg/L Kinetin 500nM Pursuit, 200mg/l Timentin and incubated under cool white fluorescent light (100 uE/m2/s-1 with photoperiod of 16hrs) at 25 °Cfor 2-3 weeks, or until shoots develop. The shoots were transferred to MS-based rooting medium and incubated under light at 25 °C for 2 weeks. The rooted shoots were transplanted to 4 inch pots containing artificial soil mix. Metro-Mix® 360 in and grown in an environmental chamber for 1-2 weeks. The environmental chamber maintained 16-h-light, 8-h-dark cycles at 27°C day and 22 °C respectively. Light was supplied by a mixture of incandescent and cool white fluorescent bulbs with an intensity of ∼ 400 uE/m2/s-1. After plants were grown to 4-6 leaf stage they were moved to 14 inch pots containing Metro-Mix® 360. Supplemental metal-halide lamps were used to maintain >800uE/m2/s-1 with a 16-h-light, 8-h-dark cycles at 28°C day and 22 °C. Transplantation occurs weekly on Tuesday. Peters 20-20-20 plus micronutrients (200ppm) is used to fertilize plants 2x weekly on Monday and Thursday after sampling of T0's is performed. T1 seeds were produced from plants that exhibit tolerance to the imidazolinone herbicides and which are PCR positive for the transgenes. T0 plants with single locus insertions of the T-DNA (self-pollinated) produced T1 generation that segregated for the transgene in a 3:1 ratio. Progeny containing copies of the transgene were tolerant of imidazolinone herbicides and could be detected by PCR analysis.

**[0530.4.0.0]** Example 14 c.b.: Growth of T0 corn plants for metabolic analysis Plants were grown under the following standardized conditions to properly stage them for T0 sampling. T0 plantlets were transferred to 14" pots in the greenhouse after they grow to 4-6 leaf stage (1-3 weeks). pBSMM232 containing plants were produced carried along with each experiment to serve as controls for T0 samples. Plantlets were moved to 14" pots on Tuesday of each week. Plants were grown for 9 days until the 7-13 leaf stage is reached. On Thursday between 10am and 2 pm leaf sampling was performed on the 3rd youngest (1^{st} fully elongated). Within 30 seconds 250-500mg of leaf material (without midrib), were removed weighed and placed into pre-extracted glass thimbles in liquid nitrogen. A second sample (opposite side of the midrib) from each plant was sampled as described above for qPCR analysis.

**[0530.5.0.0]** Example 14 c.c.: Growth of T1 corn plant for metabolic analysis For the bioanalytical analyses of the transgenic plants, the latter were grown uniformly in a specific culture facility. To this end the GS-90 substrate as the compost mixture was introduced into the potting machine (Laible System GmbH, Singen, Germany) and filled into the pots. Thereafter, 26 pots were combined in one dish and treated with Previcur. For the treatment, 25 ml of Previcur were taken up in 10 I of tap water. This amount was sufficient for the treatment of approximately 150 pots. The pots were placed into the Previcur solution and additionally irrigated overhead with tap water without Previcur. They were used within four days.
For the sowing, the seeds, which had been stored at room temperature were removed from the paper-bag and transferred into the pots with the soil. In total, approximately 1 to 3 seeds were distributed in the middle of the pot.
After the seeds had been sown, the dishes with the pots were covered with matching plastic hood and placed into growth chambers for 2 days. After this time the plastic hood was removed and plants were placed on the growth table and cultivated for 22 to 24 days under following growth conditions: 16-h-light, 8-h-dark rhythm at 20°C, an atmospheric humidity of 60% and a CO₂ concentration of approximately 400 ppm. The light sources used were Powerstar HQI-T 250 W/D Daylight lamps from Osram, which generate a light resembling the solar color spectrum with a light intensity of approximately 220 µE/m2/s-1.
When the plants were 7 days old, they were subjected to select transgenic plants. For this purposes pieces of plant leaves were sampled and a PCR reaction with the respective primers for the transgene were performed. Plants exhibiting the transgene were used for the metabolic analysis. The nontransgenic seedlings were removed. The transgenic plants were thinned when they had reached the age of 18 days. The transgenic plants, which had grown best in the center of the pot were considered the target plants. All the remaining plants were removed carefully with the aid of metal tweezers and discarded.
During their growth, the plants received overhead irrigation with distilled water (onto the compost) and bottom irrigation into the placement grooves. Once the grown plants had reached the age of 24 days, they were harvested.

### Example 14 c.d.: Metabolic analysis of maize leaves.

**[0530.6.0.0]** The modifications identified in accordance with the invention, in the content of above-described metabolites, were identified by the following procedure.
a) Sampling and storage of the samples
   Sampling was performed in corridor next to the green house. The leaves were incised twice using small laboratory scissors and this part of the leave was removed manually from the middle rib. The sample was rapidly weighed on laboratory scales, transferred into a pre-cooled extraction sleeve and placed into kryo-box cooled by liquid nitrogen. The time elapsing between cutting the leave to freezing it in liquid nitrogen amounted to not more than 30 seconds. The boxes were stored in a freezer at -80 °C, an shipped on dry ice.
b) Lyophilization
   During the experiment, care was taken that the plants either remained in the deep-frozen state (temperatures < -40°C) or were freed from water by lyophilization until the first contact with solvents. Before entering the analytical process the extraction sleeves with the samples were transferred to a pre-cooled aluminium rack.
   The aluminum rack with the plant samples in the extraction sleeves was placed into the pre-cooled (-40°C) lyophilization facility. The initial temperature during the main drying phase was-35°C and the pressure was 0.120 mbar. During the drying phase, the parameters were altered following a pressure and temperature program. The final temperature after 12 hours was +30°C and the final pressure was 0.001 to 0.004 mbar. After the vacuum pump and the refrigerating machine had been switched off, the system was flushed with air (dried via a drying tube) or argon.
c) Extraction
   Immediately after the lyophilization apparatus had been flushed, the extraction sleeves with the lyophilized plant material were transferred into the 5 ml extraction cartridges of the ASE device (Accelerated Solvent Extractor ASE 200 with Solvent Controller and AutoASE software (DIONEX)).
   Immediately after the lyophilization apparatus had been flushed, the extraction sleeves with the lyophilized plant material were transferred into the 5 ml extraction cartridges of the ASE device (Accelerated Solvent Extractor ASE 200 with Solvent Controller and AutoASE software (DIONEX)).
   The 24 sample positions of an ASE device (Accelerated Solvent Extractor ASE 200 with Solvent Controller and AutoASE software (DIONEX)) were filled with plant samples, including some samples for testing quality control.
   The polar substances were extracted with approximately 10 ml of methanol/water (80/20, v/v) at T = 70°C and p = 140 bar, 5 minutes heating-up phase, 1 minute static extraction. The more lipophilic substances were extracted with approximately 10 ml of methanol/dichloromethane (40/60, v/v) at T = 70°C and p = 140 bar, 5 minute heating-up phase, 1 minute static extraction. The two solvent mixtures were extracted into the same glass tubes (centrifuge tubes, 50 ml, equipped with screw cap and pierceable septum for the ASE (DIONEX)).
   The solution was treated with internal standards: ribitol, L-glycine-2,2-d₂, L-alanine-2,3,3,3-d₄, methionine-methyl-d₃, and α-methylglucopyranoside and methyl nonadecanoate, methyl undecanoate, methyl tridecanoate, methyl pentadecanoate, methyl nonacosanoate.
   The total extract was treated with 8 ml of water. The solid residue of the plant sample and the extraction sleeve were discarded.
   The extract was shaken and then centrifuged for 5 to 10 minutes at least at 1 400 g in order to accelerate phase separation. 0.5 ml of the supernatant methanol/water phase ("polar phase", colorless) was removed for the further GC analysis, and 0.5 ml was removed for the LC analysis. The remainder of the methanol/water phase of all samples was used for additional quality controls. 0.5 ml of the organic phase ("lipid phase", dark green) was removed for the further GC analysis and 0.5 ml was removed for the LC analysis. All the portions removed were evaporated to dryness using the IR Dancer infrared vacuum evaporator (Hettich). The maximum temperature during the evaporation process did not exceed 40°C. Pressure in the apparatus was not less than 10 mbar.
d) Processing the lipid phase for the LC/MS or LC/MS/MS analysis
   The lipid extract, which had been evaporated to dryness was taken up in mobile phase. The HPLC was run with gradient elution.
   The polar extract, which had been evaporated to dryness was taken up in mobile phase. The HPLC was run with gradient elution.
e) Derivatization of the lipid phase for the GC/MS analysis
   For the transmethanolysis, a mixture of 140 µl of chloroform, 37 µl of hydrochloric acid (37% by weight HCl in water), 320 µl of methanol and 20 µl of toluene was added to the evaporated extract. The vessel was sealed tightly and heated for 2 hours at 100°C, with shaking. The solution was subsequently evaporated to dryness. The residue was dried completely.
   The methoximation of the carbonyl groups was carried out by reaction with methoxyamine hydrochloride (20 mg/ml in pyridine, 100 µl for 1.5 hours at 60°C) in a tightly sealed vessel. 20 µl of a solution of odd-numbered, straight-chain fatty acids (solution of each 0.3 mg/mL of fatty acids from 7 to 25 carbon atoms and each 0.6 mg/mL of fatty acids with 27, 29 and 31 carbon atoms in 3/7 (v/v) pyridine/toluene) were added as time standards. Finally, the derivatization with 100 µl of N-methyl-N-(trimethylsilyl)-2,2,2-trifluoroacetamide (MSTFA) was carried out for 30 minutes at 60°C, again in the tightly sealed vessel. The final volume before injection into the GC was 220 µl.
f) Derivatization of the polar phase for the GC/MS analysis
   The methoximation of the carbonyl groups was carried out by reaction with methoxyamine hydrochloride (20 mg/ml in pyridine, 50 µl for 1.5 hours at 60°C) in a tightly sealed vessel. 10 µl of a solution of odd-numbered, straight-chain fatty acids (solution of each 0.3 mg/mL of fatty acids from 7 to 25 carbon atoms and each 0.6 mg/mL of fatty acids with 27, 29 and 31 carbon atoms in 3/7 (v/v) pyridine/toluene) were added as time standards. Finally, the derivatization with 50 µl of N-methyl-N-(trimethylsilyl)-2,2,2-trifluoroacetamide (MSTFA) was carried out for 30 minutes at 60°C, again in the tightly sealed vessel. The final volume before injection into the GC was 110 µl.
g) Analysis of the various plant samples
The samples were measured in individual series of 20 plant (leaf) samples each (also referred to as sequences), each sequence containing at least 5 samples from individual control plants containing GUS. The peak area of each analyte was divided by the peak area of the respective internal standard. The data were standardized for the fresh weight established for the respective harvested sample. The values calculated were then related to the GUS-containing control group by being divided by the mean of the corresponding data of the control group of the same sequence. The values obtained were referred to as ratio_by_WT, they are comparable between sequences and indicate how much the analyte concentration in the mutant differs in relation to the control. The GUS-containing plants were chosen in order to assure that the vector and transformation procedure itself has no significant influence on the metabolic composition of the plants. Therefore the desribed changes in comparison with the controls were caused by the introduced genes.

**[0531.0.0.0]** Transformation of maize (Zea Mays L.) can also be performed with a modification of the method described by Ishida et al. (1996. Nature Biotech 14745-50). Transformation is genotype-dependent in corn and only specific genotypes are amenable to transformation and regeneration. The inbred line A188 (University of Minnesota) or hybrids with A188 as a parent are good sources of donor material for transformation (Fromm et al. 1990 Biotech 8:833-839), but other genotypes can be used successfully as well. Ears are harvested from corn plants at approximately 11 days after pollination (DAP) when the length of immature embryos is about 1 to 1.2 mm. Immature embryos are co-cultivated with Agrobacterium tumefaciens that carry "super binary" vectors and transgenic plants are recovered through organogenesis. The super binary vector system of Japan Tobacco is described in WO patents WO94/00977 and WO95/06722. Vectors can be constructed as described. Various selection marker genes can be used including the maize gene encoding a mutated acetohydroxy acid synthase (AHAS) enzyme (US patent 6025541). Similarly, various promoters can be used to regulate the trait gene to provide constitutive, developmental, tissue or environmental regulation of gene transcription. In this example, the 34S promoter (GenBank Accession numbers M59930 and X16673 can be used to provide constitutive expression of the trait gene.

**[0532.0.0.0]** Excised embryos can be grown on callus induction medium, then maize regeneration medium, containing imidazolinone as a selection agent. The Petri plates can be incubated in the light at 25 °C for 2-3 weeks, or until shoots develop. The green shoots can be transferred from each embryo to maize rooting medium and incubated at 25 °C for 2-3 weeks, until roots develop. The rooted shoots can be transplanted to soil in the greenhouse. T1 seeds can be produced from plants that exhibit tolerance to the imidazolinone herbicides and which can be PCR positive for the transgenes.

**[0533.0.0.0]** The T1 generation of single locus insertions of the T-DNA can segregate for the transgene in a 3:1 ratio. Those progeny containing one or two copies of the transgene can be tolerant of the imidazolinone herbicide. Homozygous T2 plants can exhibited similar phenotypes as the T1 plants. Hybrid plants (F1 progeny) of homozygous transgenic plants and non-transgenic plants can also exhibit increased similar phenotypes.

**[0534.0.0.0]** Example 14d: Engineering wheat plants by over-expressing the polynucleotide characterized in the invention, e.g. derived from Saccharomyces cerevisiae, E. coli or plants or another organism

**[0535.0.0.0]** Transformation of wheat can be performed with the method described by Ishida et al. (1996 Nature Biotech. 14745-50). The cultivar Bobwhite (available from CYMMIT, Mexico) can commonly be used in transformation. Immature embryos can be co-cultivated with Agrobacterium tumefaciens that carry "super binary" vectors, and transgenic plants are recovered through organogenesis. The super binary vector system of Japan Tobacco is described in WO patents WO94/00977 and WO95/06722. Vectors can be constructed as described. Various selection marker genes can be used including the maize gene encoding a mutated acetohydroxy acid synthase (AHAS) enzyme (US patent 6025541). Similarly, various promoters can be used to regulate the trait gene to provide constitutive, developmental, tissue or environmental regulation of gene transcription. The 34S promoter (GenBank Accession numbers M59930 and X16673) can be used to provide constitutive expression of the trait gene.

**[0536.0.0.0]** After incubation with Agrobacterium, the embryos can be grown on callus induction medium, then regeneration medium, containing imidazolinone as a selection agent. The Petri plates can be incubated in the light at 25 °C for 2-3 weeks, or until shoots develop. The green shoots can be transferred from each embryo to rooting medium and incubated at 25 °C for 2-3 weeks, until roots develop. The rooted shoots can be transplanted to soil in the greenhouse. T1 seeds can be produced from plants that exhibit tolerance to the imidazolinone herbicides and which are PCR positive for the transgenes.

**[0537.0.0.0]** The T1 generation of single locus insertions of the T-DNA can segregate for the transgene in a 3:1 ratio. Those progeny containing one or two copies of the transgene can be tolerant of the imidazolinone herbicide. Homozygous T2 plants exhibited similar phenotypes.

**[0538.0.0.0]** Example 14e: Engineering Rapeseed/Canola plants by over-expressing the polynucleotide characterized in the invention, e.g. derived from Saccharomyces cerevisiae, E. coli or plants or another organism

**[0539.0.0.0]** Cotyledonary petioles and hypocotyls of 5-6 day-old young seedlings can be used as explants for tissue culture and transformed according to Babic et al.(1998, Plant Cell Rep 17: 183-188). The commercial cultivar Westar (Agriculture Canada) can be the standard variety used for transformation, but other varieties can be used.

**[0540.0.0.0]** Agrobacterium tumefaciens LBA4404 containing a binary vector can be used for canola transformation. Many different binary vector systems have been described for plant transformation (e.g. An, G. in Agrobacterium Protocols. Methods in Molecular Biology vol 44, pp 47-62, Gartland KMA and MR Davey eds. Humana Press, Totowa, New Jersey). Many are based on the vector pBIN19 described by Bevan (Nucleic Acid Research. 1984. 12:8711-8721) that includes a plant gene expression cassette flanked by the left and right border sequences from the Ti plasmid of Agrobacterium tumefaciens. A plant gene expression cassette can consist of at least two genes - a selection marker gene and a plant promoter regulating the transcription of the cDNA or genomic DNA of the trait gene. Various selection marker genes can be used including the Arabidopsis gene encoding a mutated acetohydroxy acid synthase (AHAS) enzyme (US patents 57673666 and 6225105). Similarly, various promoters can be used to regulate the trait gene to provide constitutive, developmental, tissue or environmental regulation of gene transcription. The 34S promoter (GenBank Accession numbers M59930 and X16673) can be used to provide constitutive expression of the trait gene.

**[0541.0.0.0]** Canola seeds can be surface-sterilized in 70% ethanol for 2 min., and then in 30% Clorox with a drop of Tween-20 for 10 min, followed by three rinses with sterilized distilled water. Seeds can be then germinated in vitro 5 days on half strength MS medium without hormones, 1% sucrose, 0.7% Phytagar at 23oC, 16 hr. light. The cotyledon petiole explants with the cotyledon attached can be excised from the in vitro seedlings, and can be inoculated with Agrobacterium by dipping the cut end of the petiole explant into the bacterial suspension. The explants can be then cultured for 2 days on MSBAP-3 medium containing 3 mg/l BAP, 3 % sucrose, 0.7 % Phytagar at 23 °C, 16 hr light. After two days of co-cultivation with Agrobacterium, the petiole explants can be transferred to MSBAP-3 medium containing 3 mg/l BAP, cefotaxime, carbenicillin, or timentin (300 mg/l) for 7 days, and can then be cultured on MSBAP-3 medium with cefotaxime, carbenicillin, or timentin and selection agent until shoot regeneration. When the shoots are 5 - 10 mm in length, they can be cut and transferred to shoot elongation medium (MSBAP-0.5, containing 0.5 mg/l BAP). Shoots of about 2 cm in length can be transferred to the rooting medium (MS0) for root induction.

**[0542.0.0.0]** Samples of the primary transgenic plants (T0) can be analyzed by PCR to confirm the presence of T-DNA. These results can be confirmed by Southern hybridization in which DNA is electrophoresed on a 1 % agarose gel and are transferred to a positively charged nylon membrane (Roche Diagnostics). The PCR DIG Probe Synthesis Kit (Roche Diagnostics) can be used to prepare a digoxigenin-labelled probe by PCR, and used as recommended by the manufacturer.

**[0543.0.0.0]** Example 14f: Engineering alfalfa plants by over-expressing the polynucleotide characterized in the invention, e.g. derived from Saccharomyces cerevisiae or E. coli or plants or another organism.

**[0544.0.0.0]** A regenerating clone of alfalfa (Medicago sativa) can be transformed using the method of (McKersie et al., 1999 Plant Physiol 119: 839-847). Regeneration and transformation of alfalfa can be genotype dependent and therefore a regenerating plant is required. Methods to obtain regenerating plants have been described. For example, these can be selected from the cultivar Rangelander (Agriculture Canada) or any other commercial alfalfa variety as described by Brown DCW and A Atanassov (1985. Plant Cell Tissue Organ Culture 4: 111-112). Alternatively, the RA3 variety (University of Wisconsin) can be selected for use in tissue culture (Walker et al., 1978 Am J Bot 65:654-659).

**[0545.0.0.0]** Petiole explants can be cocultivated with an overnight culture of Agrobacterium tumefaciens C58C1 pMP90 (McKersie et al., 1999 Plant Physiol 119: 839-847) or LBA4404 containing a binary vector. Many different binary vector systems have been described for plant transformation (e.g. An, G. in Agrobacterium Protocols. Methods in Molecular Biology vol 44, pp 47-62, Gartland KMA and MR Davey eds. Humana Press, Totowa, New Jersey). Many are based on the vector pBIN19 described by Bevan (Nucleic Acid Research. 1984. 12:8711-8721) that includes a plant gene expression cassette flanked by the left and right border sequences from the Ti plasmid of Agrobacterium tumefaciens. A plant gene expression cassette can consist of at least two genes - a selection marker gene and a plant promoter regulating the transcription of the cDNA or genomic DNA of the trait gene. Various selection marker genes can be used including the Arabidopsis gene encoding a mutated acetohydroxy acid synthase (AHAS) enzyme (US patents 57673666 and 6225105). Similarly, various promoters can be used to regulate the trait gene that provides constitutive, developmental, tissue or environmental regulation of gene transcription. The 34S promoter (GenBank Accession numbers M59930 and X16673) can be used to provide constitutive expression of the trait gene.

**[0546.0.0.0]** The explants can be cocultivated for 3 d in the dark on SH induction medium containing 288 mg/ L Pro, 53 mg/ L thioproline, 4.35 g/ L K2SO4, and 100 µm acetosyringinone. The explants can be washed in half-strength Murashige-Skoog medium (Murashige and Skoog, 1962) and plated on the same SH induction medium without acetosyringinone but with a suitable selection agent and suitable antibiotic to inhibit Agrobacterium growth. After several weeks, somatic embryos can be transferred to BOi2Y development medium containing no growth regulators, no antibiotics, and 50 g/ L sucrose. Somatic embryos are subsequently germinated on half-strength Murashige-Skoog medium. Rooted seedlings can be transplanted into pots and grown in a greenhouse.

**[0547.0.0.0]** The T0 transgenic plants are propagated by node cuttings and rooted in Turface growth medium. The plants are defoliated and grown to a height of about 10 cm (approximately 2 weeks after defoliation).

**[0548.0.0.0]** Example 14g: Engineering alfalfa plants by over-expressing the polynucleotide characterized in the invention, derived e.g. from Saccharomyces cerevisiae, E. coli or plants or another organism

**[0549.0.0.0]** A regenerating clone of alfalfa (Medicago sativa) can be transformed using the method of (McKersie et al., 1999 Plant Physiol 119: 839-847). Regeneration and transformation of alfalfa can be genotype dependent and therefore a regenerating plant is required. Methods to obtain regenerating plants have been described. For example, these can be selected from the cultivar Rangelander (Agriculture Canada) or any other commercial alfalfa variety as described by Brown DCW and A Atanassov (1985. Plant Cell Tissue Organ Culture 4: 111-112). Alternatively, the RA3 variety (University of Wisconsin) has been selected for use in tissue culture (Walker et al., 1978 Am J Bot 65:654-659).

**[0550.0.0.0]** Petiole explants can be cocultivated with an overnight culture of Agrobacterium tumefaciens C58C1 pMP90 (McKersie et al., 1999 Plant Physiol 119: 839-847) or LBA4404 containing a binary vector. Many different binary vector systems have been described for plant transformation (e.g. An, G. in Agrobacterium Protocols. Methods in Molecular Biology vol 44, pp 47-62, Gartland KMA and MR Davey eds. Humana Press, Totowa, New Jersey). Many are based on the vector pBIN19 described by Bevan (Nucleic Acid Research. 1984. 12:8711-8721) that includes a plant gene expression cassette flanked by the left and right border sequences from the Ti plasmid of Agrobacterium tumefaciens. A plant gene expression cassette consists of at least two genes - a selection marker gene and a plant promoter regulating the transcription of the cDNA or genomic DNA of the trait gene. Various selection marker genes can be used including the Arabidopsis gene encoding a mutated acetohydroxy acid synthase (AHAS) enzyme (US patents 57673666 and 6225105). Similarly, various promoters can be used to regulate the trait gene that provides constitutive, developmental, tissue or environmental regulation of gene transcription. In this example, the 34S promoter (GenBank Accession numbers M59930 and X16673) can be used to provide constitutive expression of the trait gene.

**[0551.0.0.0]** The explants are cocultivated for 3 d in the dark on SH induction medium containing 288 mg/ L Pro, 53 mg/ L thioproline, 4.35 g/ L K2SO4, and 100 µm acetosyringinone. The explants are washed in half-strength Murashige-Skoog medium (Murashige and Skoog, 1962) and plated on the same SH induction medium without acetosyringinone but with a suitable selection agent and suitable antibiotic to inhibit Agrobacterium growth. After several weeks, somatic embryos are transferred to BOi2Y development medium containing no growth regulators, no antibiotics, and 50 g/ L sucrose. Somatic embryos are subsequently germinated on half-strength Murashige-Skoog medium. Rooted seedlings are transplanted into pots and grown in a greenhouse.

**[0552.0.0.0]** The T0 transgenic plants are propagated by node cuttings and rooted in Turface growth medium. The plants are defoliated and grown to a height of about 10 cm (approximately 2 weeks after defoliation).

**[0552.1.0.0]** ./.

**[0552.2.0.0]** Example 16: Preparation of homologous sequences from plants Different plants can be grown under standard or varying conditions in the greenhouse. RNA can be extracted following the protocol of Jones, Dunsmuir and Bedbrook (1985) EMBO J. 4: 2411-2418. Approx. 1 gram of tissue material from various organs is ground in liquid nitrogen. The powder is transferred to a 13 ml Falcon tube containing 4.5 ml NTES buffer (100 mM NaCl, 10 mM Tris/HCl pH 7.5, 1 mM EDTA, 1 % SDS; in RNase-free water) and 3 ml phenol/chloroform/isoamylalcohol (25/24/1), immediately mixed and stored on ice. The mixture is spun for 10 minutes at 7000 rpm using a centrifuge (Sorval; SM24 or SS34 rotor). The supernatant is transferred to a new tube, 1/10th volume of 3 M NaAcetate (pH 5.2; in RNase-free water) and 1 volume of isopropanol is added, mixed at stored for 1 hour or overnight at -20 °C. The mixture is spun for 10 minutes at 7000 rpm. The supernatant is discarded and the pellet washed with 70 % ethanol (v/v). The mixture is spun for 5 minutes at 7000 rpm, the supernatant is discarded and the pellet is air-dried. 1 ml RNase-free water is added and allow the DNA/RNA pellet to dissolve on ice at 4 C. The nucleic acid solution is transferred to a 2 ml Eppendorf tube and 1 ml of 4 M LiAcetate is added. After mixing the solution is kept for at least 3 hours, or overnight, at 4 C. The mixture is spun for 10 minutes at 14000 rpm, the supernatant discarded, the pellet washed with 70 % Ethanol, air-dried and dissolved in 200 µl of RNase-free water.
Total RNA can be used to construct a cDNA-library according to the manufacturer's protocol (for example using the ZAP-cDNA synthesis and cloning kit of Stratagene, La Jolla, USA). Basically, messenger RNA (mRNA) is primed in the first strand synthesis with a oligo(dT) linker-primer and is reverse-transcribed using reverse transcriptase. After second strand cDNA synthesis, the double-stranded cDNA is ligated into the Uni-ZAP XR vector. The Uni-ZAP XR vector allows in vivo excision of the pBluescript phagemid. The polylinker of the pBluescript phagemid has 21 unique cloning sites flanked by T3 and T7 promoters and a choice of 6 different primer sites for DNA sequencing. Systematic single run sequencing of the expected 5 prime end of the clones can allow preliminary annotation of the sequences for example with the help of the pedant pro Software package (Biomax, München). Clones for the nucleic acids of the invention or used in the process according to the invention can be identified based on homology search with standard algorithms like blastp or gap. Identified putative full length clones with identity or high homology can be subjected to further sequencing in order to obtain the complete sequence.
Additional new homologous sequences can be identified in a similar manner by preparing respective cDNA libraries from various plant sources as described above. Libraries can then be screened with available sequences of the invention under low stringency conditions for example as described in Sambrook et al., Molecular Cloning: A laboratory manual, Cold Spring Harbor 1989, Cold Spring Harbor Laboratory Press. Purified positive clones can be subjected to the in vivo excision and complete sequencing. A pairwise sequence alignment of the original and the new sequence using the blastp or gap program allows the identification of orthologs, meaning homologous sequences from different organisms, which should have a sequence identity of at least 30%. Furthermore the conservation of functionally important amino acid residues or domains, which can be identified by the alignment of several already available paralogs, can identify a new sequence as an new orthologs..
Alternatively libraries can be subjected to mass sequencing and obtained sequences can be stored in a sequence database, which then can be screened for putative orthologs by different search algorithms, for example the tbastn algorithm to search the obtained nucleic acid sequences with a amino acid sequence of the invention. Clones with the highest sequence identity are used for a complete sequence determination and orthologs can be identified as described above.

**[0553.0.0.0]**
1. A process for the production of methionine, which comprises
   (a) increasing or generating the activity of a protein as indicated in Table II, columns 5 or 7, lines 1 to 5 and/or lines 334 to 338 or a functional equivalent thereof in a non-human organism, or in one or more parts thereof; and
   (b) growing the organism under conditions which permit the production of methionine in said organism.
2. A process for the production of methionine, comprising the increasing or generating in an organism or a part thereof the expression of at least one nucleic acid molecule comprising a nucleic acid molecule selected from the group consisting of:
   a) nucleic acid molecule encoding of a polypeptide as indicated in Table II, columns 5 or 7, lines 1 to 5 and/or lines 334 to 338or a fragment thereof, which confers an increase in the amount of methionine in an organism or a part thereof;
   b) nucleic acid molecule comprising of a nucleic acid molecule as indicated in Table I, columns 5 or 7, lines 1 to 5 and/or lines 334 to 338;
   c) nucleic acid molecule whose sequence can be deduced from a polypeptide sequence encoded by a nucleic acid molecule of (a) or (b) as a result of the degeneracy of the genetic code and conferring an increase in the amount of methionine in an organism or a part thereof;
   d) nucleic acid molecule which encodes a polypeptide which has at least 50% identity with the amino acid sequence of the polypeptide encoded by the nucleic acid molecule of (a) to (c) and conferring an increase in the amount of methionine in an organism or a part thereof;
   e) nucleic acid molecule which hybridizes with a nucleic acid molecule of (a) to (c) under stringent hybridisation conditions and conferring an increase in the amount of methionine in an organism or a part thereof;
   f) nucleic acid molecule which encompasses a nucleic acid molecule which is obtained by amplifying nucleic acid molecules from a cDNA library or a genomic library using the primers or primer pairs as indicated in Table III, columns 7, lines 1 to 5 and/or lines 334 to 338and conferring an increase in the amount of the respective fine chemical in an organism or a part thereof;
   g) nucleic acid molecule encoding a polypeptide which is isolated with the aid of monoclonal antibodies against a polypeptide encoded by one of the nucleic acid molecules of (a) to (f) and conferring an increase in the amount of methionine in an organism or a part thereof;
   h) nucleic acid molecule encoding a polypeptide comprising a consensus sequence as indicated in Table IV, columns 7, lines 1 to 5 and/or lines 334 to 338 and conferring an increase in the amount of the respective fine chemical in an organism or a part thereof; and
   i) nucleic acid molecule which is obtainable by screening a suitable nucleic acid library under stringent hybridization conditions with a probe comprising one of the sequences of the nucleic acid molecule of (a) to (k) or with a fragment thereof having at least 15 nt, preferably 20 nt, 30 nt, 50 nt, 100 nt, 200 nt or 500 nt of the nucleic acid molecule characterized in (a) to (k) and conferring an increase in the amount of the respective fine chemical in an organism or a part thereof.
   or comprising a sequence which is complementary thereto.
3. The process of claim 1 or 2, comprising recovering of the free or bound methionine.
4. The process of any one of claim 1 to 3, comprising the following steps:
   (a) selecting an organism or a part thereof expressing a polypeptide encoded by the nucleic acid molecule characterized in claim 2;
   (b) mutagenizing the selected organism or the part thereof;
   (c) comparing the activity or the expression level of said polypeptide in the mutagenized organism or the part thereof with the activity or the expression of said polypeptide of the selected organisms or the part thereof;
   (d) selecting the mutated organisms or parts thereof, which comprise an increased activity or expression level of said polypeptide compared to the selected organism or the part thereof;
   (e) optionally, growing and cultivating the organisms or the parts thereof; and
   (f) recovering, and optionally isolating, the free or bound methionine produced by the selected mutated organisms or parts thereof.
5. The process of any one of claims 1 to 4, wherein the activity of said protein or the expression of said nucleic acid molecule is increased or generated transiently or stably.
6. An isolated nucleic acid molecule comprising a nucleic acid molecule selected from the group consisting of:
   a) nucleic acid molecule encoding of a polypeptide as indicated in Table II, columns 5 or 7, lines 1 to 5 and/or lines 334 to 338 or a fragment thereof, which confers an increase in the amount of methionine in an organism or a part thereof;
   b) nucleic acid molecule comprising of a nucleic acid molecule as indicated in Table I, columns 5 or 7, lines 1 to 5 and/or lines 334 to 338;
   c) nucleic acid molecule whose sequence can be deduced from a polypeptide sequence encoded by a nucleic acid molecule of (a) or (b) as a result of the degeneracy of the genetic code and conferring an increase in the amount of methionine in an organism or a part thereof;
   d) nucleic acid molecule which encodes a polypeptide which has at least 50% identity with the amino acid sequence of the polypeptide encoded by the nucleic acid molecule of (a) to (c) and conferring an increase in the amount of methionine in an organism or a part thereof;
   e) nucleic acid molecule which hybridizes with a nucleic acid molecule of (a) to (c) under stringent hybridisation conditions and conferring an increase in the amount of methionine in an organism or a part thereof;
   f) nucleic acid molecule which encompasses a nucleic acid molecule which is obtained by amplifying nucleic acid molecules from a cDNA library or a genomic library using the primers or primer pairs as indicated in Table III, columns 7, lines 1 to 5 and/or lines 334 to 338 and conferring an increase in the amount of the respective fine chemical in an organism or a part thereof;
   g) nucleic acid molecule encoding a polypeptide which is isolated with the aid of monoclonal antibodies against a polypeptide encoded by one of the nucleic acid molecules of (a) to (f) and conferring an increase in the amount of methionine in an organism or a part thereof;
   h) nucleic acid molecule encoding a polypeptide comprising a consensus sequence as indicated in Table IV, columns 7, lines 1 to 5 and/or lines 334 to 338 and conferring an increase in the amount of the respective fine chemical in an organism or a part thereof; and
   i) nucleic acid molecule which is obtainable by screening a suitable nucleic acid library under stringent hybridization conditions with a probe comprising one of the sequences of the nucleic acid molecule of (a) to (k) or with a fragment thereof having at least 15 nt, preferably 20 nt, 30 nt, 50 nt, 100 nt, 200 nt or 500 nt of the nucleic acid molecule characterized in (a) to (k) and conferring an increase in the amount of the respective fine chemical in an organism or a part thereof.
   whereby the nucleic acid molecule distinguishes over the sequence as indicated in Table I A, columns 5 or 7, lines 1 to 5 and/or lines 334 to 338 by one or more nucleotides.
7. A nucleic acid construct which confers the expression of the nucleic acid molecule of claim 6, comprising one or more regulatory elements.
8. A vector comprising the nucleic acid molecule as claimed in claim 6 or the nucleic acid construct of claim 7.
9. The vector as claimed in claim 8, wherein the nucleic acid molecule is in operable linkage with regulatory sequences for the expression in a prokaryotic or eukaryotic, or in a prokaryotic and eukaryotic, host.
10. A host cell, which has been transformed stably or transiently with the vector as claimed in claim 8 or 9 or the nucleic acid molecule as claimed in claim 6 or the nucleic acid construct of claim 7 or produced as described in claim any one of claims 2 to 5.
11. The host cell of claim 10, which is a transgenic host cell.
12. The host cell of claim 10 or 11, which is a plant cell, an animal cell, a microorganism, or a yeast cell, a fungus cell, a prokaryotic cell, an eukaryotic cell or an archaebacterium.
13. A process for producing a polypeptide, wherein the polypeptide is expressed in a host cell as claimed in any one of claims 10 to 12.
14. A polypeptide produced by the process as claimed in claim 13 or encoded by the nucleic acid molecule as claimed in claim 6 whereby the polypeptide distinguishes over a sequence as indicated in Table II A, columns 5 or 7, lines 1 to 5 and/or lines 334 to 338 by one or more amino acids.
15. An antibody, which binds specifically to the polypeptide as claimed in claim 14.
16. A plant tissue, propagation material, harvested material or a plant comprising the host cell as claimed in claim 12 which is plant cell or an Agrobacterium.
17. A method for screening for agonists and antagonists of the activity of a polypeptide encoded by the nucleic acid molecule of claim 6 conferring an increase in the amount of methionine in an organism or a part thereof comprising:
   (a) contacting cells, tissues , plants or microorganisms which express the a polypeptide encoded by the nucleic acid molecule of claim 5 conferring an increase in the amount of methionine in an organism or a part thereof with a candidate compound or a sample comprising a plurality of compounds under conditions which permit the expression the polypeptide;
   (b) assaying the methionine level or the polypeptide expression level in the cell, tissue, plant or microorganism or the media the cell, tissue, plant or microorganisms is cultured or maintained in; and
   (c) identifying a agonist or antagonist by comparing the measured methionine level or polypeptide expression level with a standard methionine or polypeptide expression level measured in the absence of said candidate compound or a sample comprising said plurality of compounds, whereby an increased level over the standard indicates that the compound or the sample comprising said plurality of compounds is an agonist and a decreased level over the standard indicates that the compound or the sample comprising said plurality of compounds is an antagonist.
18. A process for the identification of a compound conferring increased methionine production in a plant or microorganism, comprising the steps:
   (a) culturing a plant cell or tissue or microorganism or maintaining a plant expressing the polypeptide encoded by the nucleic acid molecule of claim 6 conferring an increase in the amount of methionine in an organism or a part thereof and a readout system capable of interacting with the polypeptide under suitable conditions which permit the interaction of the polypeptide with dais readout system in the presence of a compound or a sample comprising a plurality of compounds and capable of providing a detectable signal in response to the binding of a compound to said polypeptide under conditions which permit the expression of said readout system and of the polypeptide encoded by the nucleic acid molecule of claim 6 conferring an increase in the amount of methionine in an organism or a part thereof;
   (b) identifying if the compound is an effective agonist by detecting the presence or absence or increase of a signal produced by said readout system.
19. A method for the identification of a gene product conferring an increase in methionine production in a cell, comprising the following steps:
   (a) contacting the nucleic acid molecules of a sample, which can contain a candidate gene encoding a gene product conferring an increase in methionine after expression with the nucleic acid molecule of claim 6;
   (b) identifying the nucleic acid molecules, which hybridise under relaxed stringent conditions with the nucleic acid molecule of claim 6;
   (c) introducing the candidate nucleic acid molecules in host cells appropriate for producing methionine;
   (d) expressing the identified nucleic acid molecules in the host cells;
   (e) assaying the methionine level in the host cells; and
   (f) identifying nucleic acid molecule and its gene product which expression confers an increase in the methionine level in the host cell in the host cell after expression compared to the wild type.
20. A method for the identification of a gene product conferring an increase in methionine production in a cell, comprising the following steps:
   (a) identifying in a data bank nucleic acid molecules of an organism; which can contain a candidate gene encoding a gene product conferring an increase in the methionine amount or level in an organism or a part thereof after expression, and which are at least 20% homolog to the nucleic acid molecule of claim 6;
   (b) introducing the candidate nucleic acid molecules in host cells appropriate for producing methionine;
   (c) expressing the identified nucleic acid molecules in the host cells;
   (d) assaying the methionine level in the host cells; and
   (e) identifying nucleic acid molecule and its gene product which expression confers an increase in the methionine level in the host cell after expression compared to the wild type.
21. A method for the production of an agricultural composition comprising the steps of the method of any one of claims 17 to 20 and formulating the compound identified in any one of claims 17 to 20 in a form acceptable for an application in agriculture.
22. A composition comprising the nucleic acid molecule of claim 6, the polypeptide of claim 14, the nucleic acid construct of claim 7, the vector of any one of claims 8 or 9, an antagonist or agonist identified according to claim 17, the compound of claim 18, the gene product of claim 19 or 20, the antibody of claim 15, and optionally an agricultural acceptable carrier.
23. Use of the nucleic acid molecule as claimed in claim 6 for the identification of a nucleic acid molecule conferring an increase of methionine after expression.
24. Use of the polypeptide of claim 14 or the nucleic acid construct claim 7 or the gene product identified according to the method of claim 19 or 20 for identifying compounds capable of conferring a modulation of methionine levels in an organism.
25. Food or feed composition comprising the nucleic acid molecule of claim 6, the polypeptide of claim 14, the nucleic acid construct of claim 7, the vector of claim 8 or 9, the antagonist or agonist identified according to claim 17, the antibody of claim 15, the plant or plant tissue of claim 16, the harvested material of claim 16, the host cell of claim 10 to 12 or the gene product identified according to the method of claim 19 or 20.

### Abstract

**[0554.0.0.0]** The present invention relates to a process for the production of the fine chemical in a microorganism, a plant cell, a plant, a plant tissue or in one or more parts thereof. The invention furthermore relates to nucleic acid molecules, polypeptides, nucleic acid constructs, vectors, antisense molecules, antibodies, host cells, plant tissue, propagation material, harvested material, plants, microorganisms as well as agricultural compositions and to their use.

### Process for the production of fine chemicals

**[0000.0.0.1]** In a further embodiment, the present invention relates to a further process for the production of fine chemicals as defined below and corresponding embodiments as described herein as follows.

**[0001.0.0.1] to [0007.0.0.1]: see [0001.0.0.0] to [0007.0.0.0]**

**[0007.1.0.1]** Following the approach of deregulating specific enzymes in the amino acid biosynthetic pathway an increase of the levels of free threonine is disclosed in US 5,942,660 which is achieved by overexpression of either a wild-type or deregulated aspartate kinase, homoserine dehydrogenase or threonine synthase.

**[0008.0.0.1] see [0008.0.0.0]**

**[0009.0.1.1]** As described above, the essential amino acids are necessary for humans and many mammals, for example for livestock. Threonine is an important constituent in many body proteins and is necessary for the formation of tooth enamel protein, collagen and elastin, which both needed for healthy skin and wound healing. It is a precursor to the amino acids glycine and serine. It acts as a lipotropic in controlling fat build-up in the liver. Threonine is an immune stimulant because it promotes thymus growth and activity. It is a component of digestive enzymes and immune secretions from the gut, particularly mucins. It has been used as a supplement to help alleviate anxiety and some cases of depression. In animal production, as an important essential amino acid, threonine is normally the second limiting amino acid for pigs and the third limiting amino acid for chicken (Gallus gallus f. domestica, e.g. laying hen or broiler).

**[0010.0.0.1] see [0010.0.0.0]**

**[0011.0.0.1] see [0011.0.0.0]**

**[0012.0.1.1]** It is an object of the present invention to develop an inexpensive process for the synthesis of threonine, preferably L-threonine. Threonine is together with lysine and methionine (depending on the organism) one of the amino acids which are most frequently limiting.

**[0013.0.0.1] see [0013.0.0.0]**

**[0014.0.1.1]** Accordingly, in a first embodiment, the invention relates to a process for the production of a fine chemical, whereby the fine chemical is threonine, preferably L-threonine. Accordingly, in the present invention, the term "the fine chemical" as used herein relates to "threonine". Further, the term "the fine chemicals" as used herein also relates to fine chemicals comprising threonine.

**[0015.0.1.1]** In one embodiment, the term "the fine chemical" means threonine, preferably L-threonine. Throughout the specification the term "the fine chemical" means threonine, preferably L-threonine, its salts, ester or amids in free form or bound to proteins. In a preferred embodiment, the term "the fine chemical" means threonine, preferably L-threonine, in free form or its salts or bound to proteins.

**[0016.0.1.1]** Accordingly, the present invention relates to a process comprising
(a) increasing or generating the activity of one or more
   YFL050C, YKR057W, YIL150C, YNL046W, YNL120C, b0186, b0730, b1829, b2170, b0019, b0464, b1360, b1738, b1830, b1896, b2270, b2414, b2552, b2664, b3074, b3160, b3231, b3462, b3791, b3966, b4004, YOR245C - protein(s) in a non-human organism in one or more parts thereof and
(b) growing the organism under conditions which permit the production of the fine chemical, thus, threonine or fine chemicals comprising threonine, in said organism.
Accordingly, the present invention relates to a process for the production of a fine chemical comprising
(a) increasing or generating the activity of one or more proteins having the activity of a protein indicated in Table IIA or IIB, column 3, lines 6 to 15, 339 to 355 or having the sequence of a polypeptide encoded by a nucleic acid molecule indicated in Table IA or IB, column 5 or 7, lines 6 to 15; 339 to 355, in a non-human organism in one or more parts thereof and
(b) growing the organism under conditions which permit the production of the fine chemical, in particular threonine.

**[0017.0.0.1] see [0017.0.0.0]**

**[0018.0.0.1] see [0018.0.0.0]**

**[0019.0.1.1]** Advantageously the process for the production of the fine chemical leads to an enhanced production of the fine chemical. The terms "enhanced" or "increase" mean at least a 10%, 20%, 30%, 40% or 50%, preferably at least 60%, 70%, 80%, 90% or 100%, more preferably 150%, 200%, 300%, 400% or 500% higher production of the fine chemical in comparison to the reference as defined below, e.g. that means in comparison to an organism without the aforementioned modification of the activity of a protein having the activity of a protein indicated in Table IIA or IIB, column 3, lines 6 to 15, 339 to 355 or encoded by nucleic acid molecule indicated in Table IIA or IIB, columns 5 or 7, lines 6 to 15, 339 to 355.

**[0020.0.1.1]** Surprisingly it was found, that the transgenic expression of at least one of the Saccaromyces cerevisiae protein(s) indicated in Table IIA or IIB, Column 3, lines 6 to 10 and line 355 and/or at least one of the Escherichia coli K12 proteins indicated in Table IIA or IIB, Column 3, line 11-15, 339 to 354 in Arabidopsis thaliana conferred an increase in the threonine (or fine chemical) content of the transformed plants.

**[0021.0.0.1] see [0021.0.0.0]**

**[0022.0.1.1]** The sequence of YFL050C from Saccharomyces cerevisiae has been published in Murakami et al., Nat. Genet. 10 (3), 261-268, 1995 and Goffeau et al., Science 274 (5287), 546-547, 1996, and its activity is defined as a di- trivalent inorganic cation transporter. Accordingly, in one embodiment, the process of the present invention comprises the use of a gene product defined as di- trivalent inorganic cation transporter from Saccaromyces cerevisiae or its homolog, e.g. as shown herein, for the production of the fine chemical, meaning threonine, in particular for increasing the amount of threonine, preferably L-threonine in free or bound form in an organism or a part thereof, as mentioned.
The sequence of YKR057W from Saccharomyces cerevisiae has been published in Dujon et al., Nature 369 (6479), 371-378, 1994 and Goffeau et al., Science 274 (5287), 546-547, 1996 and its activity is being defined as an ribosomal protein, similar to S21 ribosomal proteins, involved in ribosome biogenesis and translation. Accordingly, in one embodiment, the process of the present invention comprises the use of a ribosomal protein, similar to S21 ribosomal proteins, involved in ribosome biogenesis and translation from Saccaromyces cerevisiae or its homolog, e.g. as shown herein, for the production of the fine chemical, meaning of threonine, in particular for increasing the amount of threonine, preferably L-threonine in free or bound form in an organism or a part thereof, as mentioned.
The sequence of YIL150C from Saccharomyces cerevisiae has been published in Goffeau et al., Science 274 (5287), 546-547, 1996 and Churcher et al., Nature 387 (6632 Suppl), 84-87, 1997 and its activity is being defined as a chromatin binding protein, required for S-phase (DNA synthesis) initiation or completion. Accordingly, in one embodiment, the process of the present invention comprises the use of a chromatin binding protein, required for S-phase (DNA synthesis) initiation or completion, from Saccaromyces cerevisiae or its homolog, e.g. as shown herein, for the production of the fine chemical, meaning of threonine, in particular for increasing the amount of threonine, preferably L-threonine in free or bound form in an organism or a part thereof, as mentioned.
The sequence of YNL046W from Saccharomyces cerevisiae has been published in Goffeau et al., Science 274 (5287), 546-547, 1996 and Philippsen et al., Nature 387 (6632 Suppl), 93-98, 1997 and its activity is being defined as a probable membrane protein of the endoplasmatic reticulum. Accordingly, in one embodiment, the process of the present invention comprises the use of a YNL046W, as a probable membrane protein of the endoplasmatic reticulum, from Saccaromyces cerevisiae or its homolog, e.g. as shown herein, for the production of the fine chemical, meaning of threonine, in particular for increasing the amount of threonine, preferably threonine in free or bound form in an organism or a part thereof, as mentioned.
The sequence of YNL120C from Saccharomyces cerevisiae has been published in de Antoni et al, Yeast 13:261-266, 1997, and its cellular activity has not been characterized yet. Accordingly, in one embodiment, the process of the present invention comprises the use of a YNL120C activity from Saccaromyces cerevisiae or its homolog, e.g. as shown herein, for the production of the fine chemical, meaning of threonine, in particular for increasing the amount of threonine, preferably threonine in free or bound form in an organism or a part thereof, as mentioned.
The sequence of b0186 from Escherichia coli K12 has been published in Blattner et al., Science 277(5331), 1453-1474, 1997, and its activity is being defined as a lysine decarboxylase. Accordingly, in one embodiment, the process of the present invention comprises the use of a lysine decarboxylase from E. coli or its homolog, e.g. as shown herein, for the production of the fine chemical, meaning of threonine, in particular for increasing the amount of threonine, preferably threonine in free or bound form in an organism or a part thereof, as mentioned. In one embodiment, in the process of the present invention the activity of a lysine decarboxylase is increased or generated, e.g. from E. coli or a homolog thereof.
The sequence of b0730 from Escherichia coli K12 has been published in Blattner et al., Science 277(5331), 1453-1474, 1997, and its activity is being defined as transcriptional regulator of succinylCoA synthetase operon and fatty acyl response regulator. Accordingly, in one embodiment, the process of the present invention comprises the use of a transcriptional regulator of succinylCoA synthetase operon or a fatty acid response regulator from E. coli or its homolog, e.g. as shown herein, for the production of the fine chemical, meaning of threonine, in particular for increasing the amount of threonine, preferably L-threonine in free or bound form in an organism or a part thereof, as mentioned.
The sequence of b1829 from Escherichia coli K12 has been published in Blattner et al., Science 277(5331), 1453-1474, 1997, and its activity is being defined as a heat shock protein. Accordingly, in one embodiment, the process of the present invention comprises the use of a "heat shock protein" from E. coli or its homolog, e.g. as shown herein, for the production of the fine chemical, meaning of threonine, in particular for increasing the amount of threonine in free or bound form in an organism or a part thereof, as mentioned. In one embodiment, in the process of the present invention the activity of a htpX heat shock protein is increased or generated, e.g. from E. coli or a homolog thereof. The htpX heat shock protein is also annotated as having a protease activity. Accordingly, in one embodiment, in the process of the present invention the activity of a protease, preferably of a heat shock protease, more preferred of a htpX protease or its homolog is increased for the production of the fine chemical, meaning of threonine, in particular for increasing the amount of threonine in free or bound form in an organism or a part thereof, as mentioned.
The sequence of b2170 from Escherichia coli K12 has been published in Blattner et al, Science 277(5331), 1453-1474, 1997, and its activity is being defined as a sugar efflux transporter. Accordingly, in one embodiment, the process of the present invention comprises the use of a sugar efflux transporter B from E. coli or its homolog, e.g. as shown herein, for the production of the fine chemical, meaning of threonine, in particular for increasing the amount of threonine, preferably L-threonine in free or bound form in an organism or a part thereof, as mentioned.
The sequence of b0019 from Escherichia coli K12 has been published in Blattner et al., Science 277(5331), 1453-1474, 1997, and its activity is being defined as protein for the transport; the transport of small molecules, preferably cations. In a more preferred embodiment the protein has the activity of a Na+/H+ antiporter, responsive to stress, especially to high salinity and pH. Accordingly, in one embodiment, the process of the present invention comprises the use of a protein for the transport; preferably a stress responsive Na+/H+ antiporter from E. coli or its homolog, e.g. as shown herein, for the production of the fine chemical, meaning of threonine, in particular for increasing the amount of threonine, preferably L-threonine in free or bound form in an organism or a part thereof, as mentioned.
The sequence of b0464 from Escherichia coli K12 has been published in Blattner et al., Science 277(5331), 1453-1474, 1997, and its activity is being defined as a transcriptional repressor for multidrug efflux pump (TetR/AcrR family). Accordingly, in one embodiment, the process of the present invention comprises the use of a transcriptional repressor for multidrug efflux pump (TetR/AcrR family) from E. coli or its homolog, e.g. as shown herein, for the production of the fine chemical, meaning of threonine, in particular for increasing the amount of threonine, preferably threonine in free or bound form in an organism or a part thereof, as mentioned. In one embodiment, in the process of the present invention the activity of a transcriptional repressor for multidrug efflux pump (TetR/AcrR family) is increased or generated, e.g. from E. coli or a homolog thereof.
The sequence of b1360 from Escherichia coli K12 has been published in Blattner et al., Science 277(5331), 1453-1474, 1997, and its activity is being defined as a putative DNA replication protein. Accordingly, in one embodiment, the process of the present invention comprises the use of a putative DNA replication protein from E. coli or its homolog, e.g. as shown herein, for the production of the fine chemical, meaning of threonine, in particular for increasing the amount of threonine, preferably threonine in free or bound form in an organism or a part thereof, as mentioned. In one embodiment, in the process of the present invention the activity of a putative DNA replication protein is increased or generated, e.g. from E. coli or a homolog thereof.
The sequence of b1738 from Escherichia coli K12 has been published in Blattner et al., Science 277(5331), 1453-1474, 1997, and its activity is being defined as a PEP-dependent phosphotransferase. Accordingly, in one embodiment, the process of the present invention comprises the use of a PEP-dependent phosphotransferase from E. coli or its homolog, e.g. as shown herein, for the production of the fine chemical, meaning of threonine, in particular for increasing the amount of threonine, preferably threonine in free or bound form in an organism or a part thereof, as mentioned. In one embodiment, in the process of the present invention the activity of a PEP-dependent phosphotransferase is increased or generated, e.g. from E. coli or a homolog thereof.
The sequence of b1830 from Escherichia coli K12 has been published in Blattner et al., Science 277(5331), 1453-1474, 1997, and its activity is being defined as a carboxy-terminal protease for penicillin-binding protein 4. Accordingly, in one embodiment, the process of the present invention comprises the use of a carboxy-terminal protease for penicillin-binding protein 4 from E. coli or its homolog, e.g. as shown herein, for the production of the fine chemical, meaning of threonine, in particular for increasing the amount of threonine, preferably threonine in free or bound form in an organism or a part thereof, as mentioned. In one embodiment, in the process of the present invention the activity of a carboxy-terminal protease for penicillin-binding protein 4 is increased or generated, e.g. from E. coli or a homolog thereof.
The sequence of b1896 from Escherichia coli K12 has been published in Blattner et al., Science 277(5331), 1453-1474, 1997, and its activity is being defined as a trehalose-6-phosphate synthase. Accordingly, in one embodiment, the process of the present invention comprises the use of a trehalose-6-phosphate synthase from E. coli or its homolog, e.g. as shown herein, for the production of the fine chemical, meaning of threonine, in particular for increasing the amount of threonine, preferably threonine in free or bound form in an organism or a part thereof, as mentioned. In one embodiment, in the process of the present invention the activity of a trehalose-6-phosphate synthase is increased or generated, e.g. from E. coli or a homolog thereof.
The sequence of b2414 from Escherichia coli K12 has been published in Blattner et al., Science 277(5331), 1453-1474, 1997, and its activity is being defined as a subunit of cysteine synthase A and O-acetylserine sulfhydrolase A, PLP-dependent enzyme.
Accordingly, in one embodiment, the process of the present invention comprises the use of a subunit of cysteine synthase A and O-acetylserine sulfhydrolase A, PLP-dependent enzyme from E. coli or its homolog, e.g. as shown herein, for the production of the fine chemical, meaning of threonine, in particular for increasing the amount of threonine, preferably threonine in free or bound form in an organism or a part thereof, as mentioned. In one embodiment, in the process of the present invention the activity of a subunit of cysteine synthase A and O-acetylserine sulfhydrolase A, PLP-dependent enzyme is increased or generated, e.g. from E. coli or a homolog thereof.
The sequence of b2552 from Escherichia coli K12 has been published in Blattner et al., Science 277(5331), 1453-1474, 1997, and its activity is being defined as a dihydropteridine reductase (nitric oxide dioxygenase). Accordingly, in one embodiment, the process of the present invention comprises the use of a dihydropteridine reductase (nitric oxide dioxygenase) from E. coli or its homolog, e.g. as shown herein, for the production of the fine chemical, meaning of threonine, in particular for increasing the amount of threonine, preferably threonine in free or bound form in an organism or a part thereof, as mentioned. In one embodiment, in the process of the present invention the activity of a dihydropteridine reductase (nitric oxide dioxygenase) is increased or generated, e.g. from E. coli or a homolog thereof.
The sequence of b4004 from Escherichia coli K12 has been published in Blattner et al., Science 277(5331), 1453-1474, 1997, and its activity is being defined as a transcriptional regulatory protein. Accordingly, in one embodiment, the process of the present invention comprises the use of a transcriptional regulatory protein from E. coli or its homolog, e.g. as shown herein, for the production of the fine chemical, meaning of threonine, in particular for increasing the amount of threonine, preferably threonine in free or bound form in an organism or a part thereof, as mentioned. In one embodiment, in the process of the present invention the activity of a transcriptional regulatory protein is increased or generated, e.g. from E. coli or a homolog thereof.
The sequence of b2664 from Escherichia coli K12 has been published in Blattner et al., Science 277(5331), 1453-1474, 1997, and its activity is being defined as a putative transcriptional repressor with DNA-binding Winged helix domain (GntR familiy). Accordingly, in one embodiment, the process of the present invention comprises the use of a putative transcriptional repressor with DNA-binding Winged helix domain (GntR familiy) from E. coli or its homolog, e.g. as shown herein, for the production of the fine chemical, meaning of threonine, in particular for increasing the amount of threonine, preferably threonine in free or bound form in an organism or a part thereof, as mentioned. In one embodiment, in the process of the present invention the activity of a putative transcriptional repressor with DNA-binding Winged helix domain (GntR familiy) is increased or generated, e.g. from E. coli or a homolog thereof.
The sequence of b3074 from Escherichia coli K12 has been published in Blattner et al., Science 277(5331), 1453-1474, 1997, and its activity is being defined as a putative tRNA synthetase. Accordingly, in one embodiment, the process of the present invention comprises the use of a putative tRNA synthetase from E. coli or its homolog, e.g. as shown herein, for the production of the fine chemical, meaning of threonine, in particular for increasing the amount of threonine, preferably threonine in free or bound form in an organism or a part thereof, as mentioned. In one embodiment, in the process of the present invention the activity of a putative tRNA synthetase is increased or generated, e.g. from E. coli or a homolog thereof.
The sequence of b2270 from Escherichia coli K12 has been published in Blattner et al., Science 277(5331), 1453-1474, 1997, and its activity has not been characterized yet. Accordingly, in one embodiment, the process of the present invention comprises the use of a protein b2270 from E. coli or its homolog, e.g. as shown herein, for the production of the fine chemical, meaning of threonine, in particular for increasing the amount of threonine, preferably threonine in free or bound form in an organism or a part thereof, as mentioned. In one embodiment, in the process of the present invention the activity of the protein encoded by b2270 is increased or generated, e.g. from E. coli or a homolog thereof.
The sequence of b3160 from Escherichia coli K12 has been published in Blattner et al., Science 277(5331), 1453-1474, 1997, and its activity is being defined as a putative monooxygenase with luciferase-like ATPase activity. Accordingly, in one embodiment, the process of the present invention comprises the use of a putative monooxygenase with luciferase-like ATPase activity from E. coli or its homolog, e.g. as shown herein, for the production of the fine chemical, meaning of threonine, in particular for increasing the amount of threonine, preferably threonine in free or bound form in an organism or a part thereof, as mentioned. In one embodiment, in the process of the present invention the activity of a putative monooxygenase with luciferase-like ATPase activity is increased or generated, e.g. from E. coli or a homolog thereof.
The sequence of b3231 from Escherichia coli K12 has been published in Blattner et al., Science 277(5331), 1453-1474, 1997, and its activity is being defined as a 50S ribosomal subunit protein L13. Accordingly, in one embodiment, the process of the present invention comprises the use of a 50S ribosomal subunit protein L13 from E. coli or its homolog, e.g. as shown herein, for the production of the fine chemical, meaning of threonine, in particular for increasing the amount of threonine, preferably threonine in free or bound form in an organism or a part thereof, as mentioned. In one embodiment, in the process of the present invention the activity of a 50S ribosomal subunit protein L13 is increased or generated, e.g. from E. coli or a homolog thereof.
The sequence of b3462 from Escherichia coli K12 has been published in Blattner et al., Science 277(5331), 1453-1474, 1997, and its activity is being defined as an integral membrane cell division protein. Accordingly, in one embodiment, the process of the present invention comprises the use of a integral membrane cell division protein from E. coli or its homolog, e.g. as shown herein, for the production of the fine chemical, meaning of threonine, in particular for increasing the amount of threonine, preferably threonine in free or bound form in an organism or a part thereof, as mentioned. In one embodiment, in the process of the present invention the activity of a integral membrane cell division protein is increased or generated, e.g. from E. coli or a homolog thereof.
The sequence of b3791 from Escherichia coli K12 has been published in Blattner et al., Science 277(5331), 1453-1474, 1997, and its activity is being defined as a transaminase involved in lipopolysaccharide biosynthesis. Accordingly, in one embodiment, the process of the present invention comprises the use of a transaminase involved in lipopolysaccharide biosynthesis from E. coli or its homolog, e.g. as shown herein, for the production of the fine chemical, meaning of threonine, in particular for increasing the amount of threonine, preferably threonine in free or bound form in an organism or a part thereof, as mentioned. In one embodiment, in the process of the present invention the activity of a transaminase involved in lipopolysaccharide biosynthesis is increased or generated, e.g. from E. coli or a homolog thereof.
The sequence of b3966 from Escherichia coli K12 has been published in Blattner et al., Science 277(5331), 1453-1474, 1997, and its activity is being defined as an outer membrane porin. Accordingly, in one embodiment, the process of the present invention comprises the use of a outer membrane porin from E. coli or its homolog, e.g. as shown herein, for the production of the fine chemical, meaning of threonine, in particular for increasing the amount of threonine, preferably threonine in free or bound form in an organism or a part thereof, as mentioned. In one embodiment, in the process of the present invention the activity of a outer membrane porin is increased or generated, e.g. from E. coli or a homolog thereof.
The sequence of YOR245C from Saccharomyces cerevisiae has been published in Dujon, B. et al., Nature 387 (6632 Suppl), 98-102 (1997) and its activity is defined as a acyl-CoA:diacylglycerol acyltransferase. Accordingly, in one embodiment, the process of the present invention comprises the use of a gene product defined as a acyl-CoA:diacylglycerol acyltransferase from Saccaromyces cerevisiae or its homolog, e.g. as shown herein, for the production of the fine chemical, meaning threonine, in particular for increasing the amount of threonine, preferably L-threonine in free or bound form in an organism or a part thereof, as mentioned. In one embodiment, in the process of the present invention the activity of a acyl-CoA:diacylglycerol acyltransferase is increased or generated, e.g. from Saccharomyces cerevisiae or a homolog thereof.

**[0023.0.1.1]** Homologues (= homologs) of the present gene products can be derived from any organisms as long as the homologue confers the herein mentioned activity, in particular, confers an increase in the fine chemical amount or content. Further, in the present invention, the term "homologue" relates to the sequence of an organism having the highest sequence homology to the herein mentioned or listed sequences of all expressed sequences of said organism. However, the person skilled in the art knows, that, preferably, the homologue has said the-fine-chemical-increasing activity and, if known, the same biological function or activity in the organism as at least one of the protein(s) indicated in Table IIA or IIB, Column 3, lines 6 to 15, 339 to 355, e.g. having the sequence of a polypeptide encoded by a nucleic acid molecule comprising the sequence indicated in in Table IA or IB, Column 5 or 7, lines 6 to 15, 339 to 355. In one embodiment, the homolog of any one of the polypeptides indicated in Table IIA or IIB, lines 6 to 10, 339 to 355 is a homolog having the same or a similar activity, in particular an increase of activity confers an increase in the content of the fine chemical in the organsims and being derived from an eukaryot. In one embodiment, the homolog of a polypeptide indicated in Table IIA or IIB, column 3, lines 11 to 15, 339 to 355 is a homolog having the same or a similar activity, in particular an increase of activity confers an increase in the content of the fine chemical in the organisms or part thereof, and being derived from bacteria. In one embodiment, the homolog of a polypeptide indicated in Table IIA or IIB, column 3, lines 6 to 10, 339 to 355 is a homolog having the same or a similar activity, in particular an increase of activity confers an increase in the content of the fine chemical in an organisms or part thereof, and being derived from Fungi. In one embodiment, the homolog of a polypeptide indicated in Table IIA or IIB, column 3, lines 11 to 15, 339 to 355 is a homolog having the same or a similar activity, in particular an increase of activity confers an increase in the content of the fine chemical in the organisms or part thereof and being derived from Proteobacteria. In one embodiment, the homolog of a polypeptide indicated in Table IIA or IIB, column 3, lines 6 to 10, 339 to 355 is a homolog having the same or a similar activity, in particular an increase af activity confers an increase in the content of the fine chemical in the organisms or a part thereof and being derived from Ascomyceta. In one embodiment, the homolog of a polypeptide indicated in Table IIA or IIB, column 3, lines 11 to 15, 339 to 355is a homolog having the same or a similar activity, in particular an increase of activity confers an increase in the content of the fine chemical in the organisms or part thereof, and being derived from Gammaproteobacteria. In one embodiment, the homolog of a polypeptide polypeptide indicated in Table IIA or IIB, column 3, lines 6 to 10, 339 to 355 is a homolog having the same or a similar activity, in particular an increase of activity confers an increase in the content of the fine chemical in the organisms or part thereof, and being derived from Saccharomycotina. In one embodiment, the homolog of a polypeptide indicated in Table IIA or IIB, column 3, lines 11 to 15, 339 to 355 is a homolog having the same or a similar activity, in particular an increase of activity confers an increase in the content of the fine chemical in the organisms or part thereof, and being derived from Enterobacteriales. In one embodiment, the homolog of a polypeptide indicated in Table IIA or IIB, column 3, lines 6 to 15, 339 to 355 is a homolog having the same or a similar activity, in particular an increase of activity confers an increase in the content of the fine chemical in the organisms or a part thereof, and being derived from Saccharomycetes. In one embodiment, the homolog of a polypeptide indicated in Table IIA or IIB, column 3, lines 11 to 15, 339 to 354 is a homolog having the same or a similar activity, in particular an increase of activity confers an increase in the content of the fine chemical in the organisms or part thereof, and being derived from Enterobacteriaceae. In one embodiment, the homolog of a polypeptide indicated in Table IIA or IIB, column 3, lines 6 to 10, 355 is a homolog having the same or a similar activity, in particular an increase of activity confers an increase in the content of the fine chemical in the organisms, and being derived from Saccharomycetales. In one embodiment, the homolog of a polypeptide indicated in Table IIA or IIB, column 3, lines 11 to 15, 339 to 354 is a homolog having the same or a similar activity, in particular an increase of activity confers an increase in the content of the fine chemical in the organisms or a part thereof, and being derived from Escherichia. In one embodiment, the homolog of a polypeptide indicated in Table IIA or IIB, column 3, lines 6 to 10, 355 is a homolog having the same or a similar activity, in particular an increase of activity confers an increase in the content of the fine chemical in the organisms or a part thereof, and being derived from Saccharomycetaceae. In one embodiment, the homolog of a polypeptide indicated in Table IIA or IIB, column 3, line 6 to 10, 355 is a homolog having the same or a similar activity, in particular an increase of activity confers an increase in the content of the fine chemical in the organisms or a part thereof, and being derived from Saccharomycetes.

**[0023.1.0.1]** Homologs of the polypeptides polypeptide indicated in Table IIA or IIB, column 3, lines 6 to 15, 339 to 355 may be the polypetides encoded by the nucleic acid molecules polypeptide indicated in Table IA or IB, column 7, lines 6 to 10, 339 to 355 or may be the polypeptides indicated in Table IIA or IIB, column 7, lines 6 to 10, 339 to355. Homologs of the polypeptides polypeptide indicated in Table IIA or IIB, column 3, lines 6 to 15, 339 to 355 may be the polypetides encoded by the nucleic acid molecules polypeptide indicated in Table IA or IB, column 7, lines 6 to 10, 339 to 355 or may be the polypeptides indicated in Table IIA or IIB, column 7, lines 11-15, 339 to 355.

**[0024.0.0.1]: see [0024.0.0.0]**

**[0025.0.1.1]** In accordance with the invention, a protein or polypeptide has the "activity of an protein of the invention", e.g. the activity of a protein indicated in Table IIA or IIB, column 3, lines 6 to 15, 339 to 355 if its *de novo* activity, or its increased expression directly or indirectly leads to an increased threonine level in the organism or a part thereof, preferably in a cell of said organism Throughout the specification the activity or preferably the biological activity of such a protein or polypeptide or an nucleic acid molecule or sequence encoding such protein or polypeptide is identical or similar if it still has the biological or enzymatic activity of any one of the proteins indicated in Table IIA or IIB, column 3, lines 6 to 15, 339 to 355, i.e. or which has at least 10% of the original enzymatic activity, preferably 20%, particularly preferably 30%, most particularly preferably 40% in comparison to an any one of the proteins indicated in Table IIA or IIB, column 3, lines 6 to 10, 339 to 355 and/or any one of the proteins indicated in Table IIA or IIB, column 3, lines 11 to 15, 339 to 354 .

**[0025.1.0.1]** In one embodiment, the polypeptide of the invention confers said activity, e.g. the increase of the fine chemical in an organism or a part thereof, if it is derived from an organism, which is evolutionary distant to the organism in which it is expressed. For example origin and expressing organism are derived from different families, orders, classes or phylums.

**[0026.0.0.1] to [0033.0.0.1]: see [0026.0.0.0] to [0033.0.0.0]**

**[0034.0.1.1]** Preferably, the reference, control or wild type differs form the subject of the present invention only in the cellular activity of the polypeptide of the invention, e.g. as result of an increase in the level of the nucleic acid molecule of the present invention or an increase of the specific activity of the polypeptide of the invention, e.g., it differs by or in the expression level or activity of an protein having the activity of protein as indicated in Table IIA or IIB, column 3, lines 6 to 15, 339 to 355 or being encoded by a nucleic acid molecule indicated in Table IA or IB, column 5, lines 6 to 15, 339 to 355 or its homologs, e.g. as indicated in Table IA or IB, column 7, lines 6 to 15, 339 to 355, its biochemical or genetical causes and therefore shows the increased amount of the fine chemical.

**[0035.0.0.1] to [0044.0.0.1]: see [0035.0.0.0] to [0044.0.0.0]**

**[0045.0.1.1]** In one embodiment, in case the activity of the Saccharomyces cerevisiae protein YFL050C or di- trivalent inorganic cation transporter or its homologs, e.g. as indicated in Table IA or IB, columns 5 or 7, line 6, is increased, preferably, an increase of the fine chemical threonine between 19% and 56% is conferred.
In case the activity of the Saccharomyces cerevisiae protein YKR057W or a ribosomal protein, similar to S21 ribosomal proteins, involved in ribosome biogenesis and translation or its homolog e.g. as indicated in Table IA or IB, columns 5 or 7, line 7, is increased, preferably, in one embodiment the increase of the fine chemical threonine between 34% and 142% is conferred.
In one embodiment, in case the activity of the Saccharomyces cerevisiae protein YIL150C or a "protein required for S-phase (DNA synthesis) initiation or completion " or a chromatin binding protein, required for S-phase (DNA synthesis) initiation or completion or its homologs, e.g. a cell division cycle protein e.g. as indicated in Table IA or IB, columns 5 or 7, line 8, is increased, preferably, in one embodiment the increase of the fine chemical threonine between 25% and 319% is conferred.
In case the activity of the Saccharomyces cerevisiae protein YNL046W or its homologs, e.g. a probable membrane protein of the endoplasmatic reticulum e.g. as indicated in Table IA or IB, columns 5 or 7, line 9 is increased, preferably, in one embodiment an increase of the fine chemical threonine between 18% and 53% is conferred.
In one embodment, in case the activity of the Saccharomyces cerevisiae protein YNL120C or its homologs, e.g. as indicated in Table IA or IB, Columns 5 or 7, line 10, is increased, preferably, the increase of the fine chemical threonine of 44% is conferred.
In one embodiment, in case the activity of the Escherichia coli K12 protein b0186 or a lysine decarboxylases or its homologs, e.g. as indicated in Table IA or IB, columns 5 or 7, line 11, is increased, preferably, the increase of the fine chemical threonine between 49% and 228% is conferred.
In one embodiment, in case the activity of the Escherichia coli K12 protein b0730 or a protein with the activity defined as transcriptional regulator of succinylCoA synthetase operon or its homologs, e.g. as indicated in Table IA or IB, columns 5 or 7, line 12, is increased, preferably, in one embodiment an increase of the fine chemical threonine between 53% and 177% is conferred.
In case the activity of the Escherichia coli K12 protein b1829 or its homologs is increased, e.g. the activity of a protease is increased, preferably, the activity of a heat shock protein is increased, more preferred the activity of a htpX protein or its homolog e.g. as indicated in Table IA or IB, columns 5 or 7, line 13, is increased preferably, in one embodiment the increase of the fine chemical threonine between 17% and 114% is conferred.
In case the activity of the Escherichia coli K12 protein b2170 or a sugar efflux transporter or its homologs e.g. as indicated in Table IA or IB, columns 5 or 7, line 14, is increased, preferably, in one embodiment the increase of the fine chemical threonine between 35% and 79% is conferred.
In case the activity of the Escherichia coli K12 protein b0019 or a protein for the transport of cations or its homologs, e.g. a Na⁺/H⁺ antiporter, e.g. as indicated in Table IA or IB, columns 5 or 7, line 15, is increased, preferably, in one embodiment the increase of the fine chemical threonine between 24% and 44% is conferred.
In one embodiment, in case the activity of the Escherichia coli K12 protein b0464 or a protein with the activity defined as transcriptional repressor for multidrug efflux pump (TetR/AcrR family) or its homologs, e.g. as indicated in Table IA or IB, columns 5 or 7, line 339, is increased, preferably, in one embodiment an increase of the fine chemical threonine between 23% and 43% is conferred.
In one embodiment, in case the activity of the Escherichia coli K12 protein b1360 or a protein with the activity defined as putative DNA replication protein or its homologs, e.g. as indicated in Table IA or IB, columns 5 or 7, line 340, is increased, preferably, in one embodiment an increase of the fine chemical threonine between 16% and 38% is conferred.
In one embodiment, in case the activity of the Escherichia coli K12 protein b1738 or a protein with the activity defined as PEP-dependent phosphotransferase or its homologs, e.g. as indicated in Table IA or IB, columns 5 or 7, line 341, is increased, preferably, in one embodiment an increase of the fine chemical threonine between 27% and 361 % is conferred.
In one embodiment, in case the activity of the Escherichia coli K12 protein b1830 or a protein with the activity defined as carboxy-terminal protease for penicillin-binding protein 4 or its homologs, e.g. as indicated in Table IA or IB, columns 5 or 7, line 342, is increased, preferably, in one embodiment an increase of the fine chemical threonine between 24% and 43% is conferred.
In one embodiment, in case the activity of the Escherichia coli K12 protein b1896 or a protein with the activity defined as trehalose-6-phosphate synthase or its homologs, e.g. e.g. as indicated in Table IA or IB, columns 5 or 7, line 343, is increased, preferably, in one embodiment an increase of the fine chemical threonine between 46% and 108% is conferred.
In one embodiment, in case the activity of the Escherichia coli K12 protein b2414 or a protein with the activity defined as subunit of cysteine synthase A and O-acetylserine sulfhydrolase A, PLP-dependent enzyme or its homologs, e.g. as indicated in Table IA or IB, columns 5 or 7, line 345, is increased, preferably, in one embodiment an increase of the fine chemical threonine between24% and 46% is conferred.
In one embodiment, in case the activity of the Escherichia coli K12 protein b2552 or a protein with the activity defined as dihydropteridine reductase (nitric oxide dioxygenase) or its homologs, e.g. as indicated in Table IA or IB, columns 5 or 7, line 346, is increased, preferably, in one embodiment an increase of the fine chemical threonine between 17% and 37% is conferred.
In one embodiment, in case the activity of the Escherichia coli K12 protein b4004 or a protein with the activity defined as transcriptional regulatory protein or its homologs, e.g. as indicated in Table IA or IB, columns 5 or 7, line 354, is increased, preferably, in one embodiment an increase of the fine chemical threonine between 17% and 37% is conferred.
In one embodiment, in case the activity of the Escherichia coli K12 protein b2664 or a protein with the activity defined as putative transcriptional repressor with DNA-binding Winged helix domain (GntR familiy) or its homologs, e.g. as indicated in Table IA or IB, columns 5 or 7, line 347, is increased, preferably, in one embodiment an increase of the fine chemical threonine between29% and 284% is conferred.
In one embodiment, in case the activity of the Escherichia coli K12 protein b3074 or a protein with the activity defined as putative tRNA synthetase or its homologs, e.g. as indicated in Table IA or IB, columns 5 or 7, line 348, is increased, preferably, in one embodiment an increase of the fine chemical threonine between 31% and 59% is conferred.
In one embodiment, in case the activity of the Escherichia coli K12 protein b2270 or its homologs, e.g. as indicated in Table IA or IB, columns 5 or 7, line 344, is increased, preferably, in one embodiment an increase of the fine chemical threonine between 31 % and 59% is conferred.
In one embodiment, in case the activity of the Escherichia coli K12 protein b3160 or a protein with the activity defined as putative monooxygenase with luciferase-like ATPase activity or its homologs, e.g. as indicated in Table IA or IB, columns 5 or 7, line 349, is increased, preferably, in one embodiment an increase of the fine chemical threonine between 25% and 56% is conferred.
In one embodiment, in case the activity of the Escherichia coli K12 protein b3231 or a protein with the activity defined as 50S ribosomal subunit protein L13 or its homologs, e.g. as indicated in Table IA or IB, columns 5 or 7, line 350, is increased, preferably, in one embodiment an increase of the fine chemical threonine between 17% and 32% is conferred.
In one embodiment, in case the activity of the Escherichia coli K12 protein b3462 or a protein with the activity defined as integral membrane cell division protein or its homologs, e.g. as indicated in Table IA or IB, columns 5 or 7, line 351, is increased, preferably, in one embodiment an increase of the fine chemical threonine between 18% and 51% is conferred.
In one embodiment, in case the activity of the Escherichia coli K12 protein b3791 or a protein with the activity defined as transaminase involved in lipopolysaccharide biosynthesis or its homologs, e.g. as indicated in Table IA or IB, columns 5 or 7, line 352, is increased, preferably, in one embodiment an increase of the fine chemical threonine between 38% and 44% is conferred.
In one embodiment, in case the activity of the Escherichia coli K12 protein b3966 or a protein with the activity defined as outer membrane porin or its homologs, e.g. as indicated in Table IA or IB, columns 5 or 7, line 353, is increased, preferably, in one embodiment an increase of the fine chemical threonine between 19% and 47% is conferred.
In case the activity of the Saccharomyces cerevisiae protein YOR245C or a protein with the activity defined as acyl-CoA:diacylglycerol acyltransferase or its homologs, e.g. as indicated in Table IA or IB, columns 5 or 7, line 355, is increased, preferably, in one embodment an increase of the fine chemical threonine between 18% and 81% is conferred.

**[0046.0.1.1]** In one embodiment, in case the activity of the Saccaromyces cerevisiae protein YFL050C or its homologs, e.g. di- trivalent inorganic cation transporter, is increased, preferably, an increase of the fine chemical threonine and of alanine is conferred.
In one embodiment, in case the activity of the Saccaromyces cerevisiae protein YKR057W or its homologs, e.g. an ribosomal protein, similar to S21 ribosomal proteins, involved in ribosome biogenesis and translation is increased, preferably, an increase of the fine chemical threonine and of arginine, is conferred.
In one embodiment, in case the activity of the Saccaromyces cerevisiae protein YIL150C or its homologs, e.g. "a chromatin binding protein, required for S-phase (DNA synthesis) initiation or completion" or its homologs, is increased, preferably, an increase of the fine chemical threonine and of fumaric acid is conferred.
In case the activity of the Escherichia coli K12 protein b0186or its homologs, e.g. a lysine decarboxylases or its homologs, is increased preferably, an increase of the fine chemical threonine and of methionine is conferred.
In one embodiment, in case the activity of the Escherichia coli K12 protein b0730 or its homologs, e.g. a protein with the activity defined as transcriptional regulator of succinylCoA synthetase operon and fatty acyl response regulator or its homologs is increased preferably an increase of the fine chemical threonine and of beta-carotene is conferred.
In one embodiment, in case the activity of the Escherichia coli K12 protein b1829 or its homologs is increased, e.g. the activity of a protease is increased, preferably, the activity of a heat shock protein is increased, more preferred the activity of a htpX protein or its homolog is increased preferably in an increase of the fine chemical threonine and of C18:0 is conferred.
In one embodiment, in case the activity of the Escherichia coli K12 protein b2170 or its homologs is increased, e.g. the activity of a sugar efflux transporter B is increased, preferably an increase of the fine chemical threonine and of isopentenyl pyrophosphate is conferred.
In one embodiment, in case the activity of the Escherichia coli K12 protein b0019or its homologs, e.g. a protein for the transport of cations or its homologs, e.g. a Na+/H+ antiporter, is increased, preferably an increase of the fine chemical threonine and of β-sitosterol is conferred.

**[0047.0.0.1] see [0047.0.0.0]**

**[0048.0.0.1] see [0048.0.0.0]**

**[0049.0.1.1]** A protein having an activity conferring an increase in the amount or level of the fine chemical preferably has the structure of the polypeptide described herein, in particular of a polypeptides comprising a consensus sequence as indicated in Table IV, column 7, line 6 to 15, 339 to 355 or of a polypeptide as indicaded in Table IIA or IIB, columns 5 or 7, line 6 to 15, 339 to 355 or the functional homologs thereof as described herein, or is encoded by the nucleic acid molecule characterized herein or the nucleic acid molecule according to the invention, for example by a nucleic acid molecule as indicaded in Table IA or IB, columns 5 or 7, line 6 to 15, 339 to 355 or its herein described functional homologs and has the herein mentioned activity.

**[0050.0.1.1]** For the purposes of the present invention, the term "threonine" and "L-threonine" also encompass the corresponding salts, such as, for example, threonine hydrochloride or threonine sulfate. Preferably the term threonine is intended to encompass the term L-threonine.

**[0051.0.0.1] see [0051.0.0.0]**

**[0052.0.0.1] see [0052.0.0.0]**

**[0053.0.1.1]** In one embodiment, the process of the present invention comprises one or more of the following steps
(a) stabilizing a protein conferring the increased expression of a protein encoded by the nucleic acid molecule of the invention or of the polypeptid of the invention, e.g. of a polypeptide having an activity of a protein as indicated in Table IIA or IIB, column 3, line 6 to 15, 339 to 355 or its homologs activity, e.g. as indicated in Table IIA or IIB, columns 5 or 7, line 6 to 15, 339 to 355, having herein-mentioned the fine chemical-increasing activity;
(b) stabilizing a mRNA conferring the increased expression of a protein encoded by the nucleic acid molecule of the invention, e.g. of a polypeptide having an activity of a protein as indicated in Table IIA or IIB, column 3, line 6 to 15, 339 to 355 or its homologs activity, e.g. as indicated in Table IIA or IIB, columns 5 or 7, line 6 to 15, 339 to 355 or of a mRNA encoding the polypeptide of the present invention having herein-mentioned threonine increasing activity;
(c) increasing the specific activity of a protein conferring the increasd expression of a protein encoded by the nucleic acid molecule of the invention or of the polypeptide of the present invention having herein-mentioned threonine increasing activity, e.g. of a polypeptide having an activity of a protein as indicated in Table IIA or IIB, column 3, line 6 to 15, 339 to 355 or its homologs activity, e.g. as indicated in Table IIA or IIB, columns 5 or 7, line 6 to 15, 339 to 355, or decreasing the inhibitiory regulation of the polypeptide of the invention;
(d) generating or increasing the expression of an endogenous or artificial transcription factor mediating the expression of a protein conferring the increased expression of a protein encoded by the nucleic acid molecule of the invention or of the polypeptide of the invention having herein-mentioned threonine increasing activity, e.g. of a polypeptide having an activity of a protein as indicated in Table IIA or IIB, column 3, line 6 to 15, 339 to 355 or its homologs activity, e.g. as indicated in Table IIA or IIB, columns 5 or 7, line 6 to 15, 339 to 355;
(e) stimulating activity of a protein conferring the increased expression of a protein encoded by the nucleic acid molecule of the present invention or a polypeptide of the present invention having herein-mentioned threonine increasing activity, e.g. of a polypeptide having an activity of a protein as indicated in Table IIA or IIB, column 3, line 6 to 15, 339 to 355 or its homologs activity, e.g. as indicated in Table IIA or IIB, columns 5 or 7, line 6 to 15, 339 to 355 by adding one or more exogenous inducing factors to the organismus or parts thereof;
(f) expressing a transgenic gene encoding a protein conferring the increased expression of a polypeptide encoded by the nucleic acid molecule of the present invention or a polypeptide of the present invention, having herein-mentioned threonine increasing activity, e.g. of a polypeptide having an activity of a protein as indicated in Table IIA or IIB, column 3, line 6 to 15, 339 to 355 or its homologs activity, e.g. as indicated in Table IIA or IIB, columns 5 or 7, line 6 to 15, 339 to 355;
(g) increasing the copy number of a gene conferring the increased expression of a nucleic acid molecule encoding a polypeptide encoded by the nucleic acid molecule of the invention or the polypeptide of the invention having herein-mentioned threonine increasing activity, e.g. of a polypeptide having an activity of a protein as indicated in Table IIA or IIB, column 3, line 6 to 15, 339 to 355 or its homologs activity, e.g. as indicated in Table IIA or IIB, columns 5 or 7, line 6 to 15, 339 to 355;
(h) Increasing the expression of the endogenous gene encoding the polypeptide of the invention, e.g. a polypeptide having an activity of a protein as indicated in Table IIA or IIB, column 3, line 6 to 15, 339 to 355 or its homologs activity, e.g. as indicated in Table IIA or IIB, columns 5 or 7, line 6 to 15, 339 to 355 by adding positive expression or removing negative expression elements; e.g. homologous recombination can be used to either introduce positive regulatory elements like for plants the 35S enhancer into the promoter or to remove repressor elements form regulatory regions. Further gene conversion methods can be used to disrupt repressor elements or to enhance to acitivty of positive elements. Positive elements can be randomly introduced in plants by T-DNA or transposon mutagenesis and lines can be identified in which the positive elements have be integrated near to a gene of the invention, the expression of which is thereby enhanced;
(i) Modulating growth conditions of an organism in such a manner, that the expression or activity of the gene encoding the protein of the invention or the protein itself is enhanced for example microorganisms or plants can be grown under a higher temperature regime leading to an enhanced expression of heat shock proteins, e.g. the heat shock protein of the invention, which can lead an enhanced the fine chemical production; and/or
(j) selecting of organisms with expecially high activity of the proteins of the invention from natural or from mutagenized resources and breeding them into the target organisms, eg the elite crops.

**[0054.0.1.1]** Preferably, said mRNA is the nucleic acid molecule of the present invention and/or the protein conferring the increased expression of a protein encoded by the nucleic acid molecule of the present invention or the polypeptide having the herein mentioned activity is the polypeptide of the present invention, e.g. conferring the increase of threonine after increasing the expression or activity of the encoded polypeptide or having the activity of a polypeptide having an activity of a protein as indicated in Table II, column 5, line 6 to 15, 339 to 355or its homologs activity, e.g. as indicated in Table IIA or IIB, column 7, line 6 to 15, 339 to 355.

**[0055.0.0.1] to [0064.0.0.1] see [0055.0.0.0] to [0064.0.0.0]**

**[0065.0.1.1]** The activation of an endogenous polypeptide having above-mentioned activity, of the polypeptide of the invention, e.g. conferring the increase of the fine chemical after increase of expression or activity can also be increased by introducing a synthetic transcription factor, which binds close to the coding region of an endogenous polypeptide of the invention- or its endogenous homolog-encoding gene and activates its transcription. A chimeric zinc finger protein can be construed, which comprises a specific DNA-binding domain and an activation domain as e.g. the VP16 domain of Herpes Simplex virus. The specific binding domain can bind to the regulatory region of the endogenous protein-coding region. The expression of the chimeric transcription factor in a organism, in particular in a plant, leads to a specific expression of an endogenous polypeptid of the invention, in particular a plant homolog thereof, see e.g. in WO01/52620, Oriz, Proc. Natl. Acad. Sci. USA, 2002, Vol. 99, 13290 or Guan, Proc. Natl. Acad. Sci. USA, 2002, Vol. 99, 13296.

**[0066.0.0.1] to [0069.0.0.1]: see [0066.0.0.0] to [0069.0.0.0]**

**[0070.0.1.1]** Owing to the introduction of a gene or a plurality of genes conferring the expression of the nucleic acid molecule of the invention or the polypeptide of the invention, for example the nucleic acid construct mentioned below, into an organism alone or in combination with other genes, it is possible not only to increase the biosynthetic flux towards the end product, but also to increase, modify or create *de novo* an advantageous, preferably novel metabolites composition in the organism, e.g. an advantageous amino acid composition comprising a higher content of (from a viewpoint of nutrional physiology limited) fine chemicals, in particular amino acids, likewise the fine chemical.

**[0071.0.0.1] see [0071.0.0.0]**

**[0072.0.1.1]** By influencing the metabolism thus, it is possible to produce, in the process according to the invention, further advantageous hydroxy containing compounds. Examples of such compounds are, in addition to threonine, serine, homoserine, phosphohomoserine or hydroxyproline or methionine.

**[0073.0.1.1]** Accordingly, in one embodiment, the process according to the invention relates to a process which comprises:
(a) providing a non-human organism, preferably a microorganism, a non-human animal, a plant or animal cell, a plant or animal tissue or a plant;
(b) increasing an activity of a polypeptide of the invention or a homolog thereof, e.g. as indicated in Table IIA or IIB, columns 5 or 7, line 6 to 15, 339 to 355 or of a polypeptide being encoded by the nucleic acid molecule of the present invention and described below, e.g. conferring an increase of the fine chemical in the organism, preferably in a microorganism, a non-human animal, a plant or animal cell, a plant or animal tissue or a plant,
(c) growing the organism, preferably a microorganism, a non-human animal, a plant or animal cell, a plant or animal tissue or a plant under conditions which permit the production of the fine chemical in the organism, preferably the microorganism, the plant cell, the plant tissue or the plant; and
(d) if desired, revovering, optionally isolating, the free and/or bound the fine chemical and , optionally further free and/or bound amino acids synthetized by the organism, the microorganism, the non-human animal, the plant or animal cell, the plant or animal tissue or the plant.

**[0074.0.0.1] to [0084.0.0.1]: see [0074.0.0.0] to [0084.0.0.0]**

**[0085.0.1.1]** With regard to the nucleic acid sequence as depicted a nucleic acid construct which contains a nucleic acid sequence mentioned herein or an organism (= transgenic organism) which is transformed with said nucleic acid sequence or said nucleic acid construct, "transgene" means all those constructs which have been brought about by genetic manipulation methods, preferably in which either
(a) a nucleic acid sequence as indicated in Table IA or IB, columns 5 or 7, lines 6 to 15, 339 to 355 a derivative thereof, or
(b) a genetic regulatory element, for example a promoter, which is functionally linked to the nucleic acid sequence as indicated in Table IA or IB, columns 5 or 7, lines 6 to 15, 339 to 355 or a derivative thereof, or
(c) (a) and (b)
is/are not present in its/their natural genetic environment or has/have been modified by means of genetic manipulation methods, it being possible for the modification to be, by way of example, a substitution, addition, deletion, inversion or insertion of one or more nucleotide radicals. "Natural genetic environment" means the natural chromosomal locus in the organism of origin or the presence in a genomic library. In the case of a genomic library, the natural, genetic environment of the nucleic acid sequence is preferably at least partially still preserved. The environment flanks the nucleic acid sequence at least on one side and has a sequence length of at least 50 bp, preferably at least 500 bp, particularly preferably at least 1000 bp, very particularly preferably at least 5000 bp.

**[0086.0.0.1]: see [0086.0.0.0]**

**[0087.0.0.1]: see [0087.0.0.0]**

**[0088.0.1.1]** In an advantageous embodiment of the invention, the organism takes the form of a plant whose amino acid content is modified advantageously owing to the nucleic acid molecule of the present invention expressed. This is important for plant breeders since, for example, the nutritional value of plants for monogastric animals is limited by a few essential amino acids such as lysine, threonine or methionine.

**[0088.1.1.1]** In one embodiment, after an activity of a polypeptide of the present invention has been increased or generated, or after the expression of a nucleic acid molecule or polypeptide according to the invention has been generated or increased, the transgenic plant generated can be grown on or in a nutrient medium or else in the soil and subsequently harvested.

**[0089.0.0.1] to [0097.0.0.1]: see [0089.0.0.0] to [0097.0.0.0]**

**[0098.0.1.1]** In a further embodiment, the fine chemical threonine is produced in accordance with the invention and, if desired, is isolated. The production of further amino acids such as methionine, lysine and/or mixtures of amino acid by the process according to the invention is advantageous.

**[0099.0.0.1] to [0102.0.0.1]: see [0099.0.0.0] to [0102.0.0.0]**

**[0103.0.1.1]** In a preferred embodiment, the present invention relates to a process for the production of the fine chemical threonine comprising or generating in an organism or a part thereof the expression of at least one nucleic acid molecule comprising a nucleic acid molecule selected from the group consisting of:
(a) nucleic acid molecule encoding, preferably at least the mature form, of a polypeptide having a sequence as indicated in Table IIA or IIB, columns 5 or 7, lines 6 to 15, 339 to 355;
(b) nucleic acid molecule comprising, preferably at least the mature form, of a nucleic acid molecule having a sequence as indicated in Table IA or IB, columns 5 or 7, lines 6 to 15, 339 to 355;
(c) nucleic acid molecule whose sequence can be deduced from a polypeptide sequence encoded by a nucleic acid molecule of (a) or (b) as result of the degeneracy of the genetic code and conferring an increase in the amount of the fine chemical threonine in an organism or a part thereof;
(d) nucleic acid molecule encoding a polypeptide which has at least 50% identity with the amino acid sequence of the polypeptide encoded by the nucleic acid molecule of (a) to (c) and conferring an increase in the amount of the fine chemical threonine in an organism or a part thereof;
(e) nucleic acid molecule which hybidizes with a nucleic acid molecule of (a) to (c) under under stringent hybridisation conditions and conferring an increase in the amount of the fine chemical threonine in an organism or a part thereof;
(f) nucleic acid molecule encoding a polypeptide, the polypeptide being derived by substituting, deleting and/or adding one or more amino acids of the amino acid sequence of the polypeptide encoded by the nucleic acid molecules (a) to (d), preferably to (a) to (c) and conferring an increase in the amount of the fine chemical threonine in an organism or a part thereof;
(g) nucleic acid molecule encoding a fragment or an epitope of a polypeptide which is encoded by one of the nucleic acid molecules of (a) to (e), preferably to (a) to (c) and and conferring an increase in the amount of the fine chemical threonine in an organism or a part thereof;
(h) nucleic acid molecule comprising a nucleic acid molecule which is obtained by amplifying nucleic acid molecules from a cDNA library or a genomic library using the primers pairs having a sequence as indicated in Table III, columns 7, lines 6 to 15, 339 to 355, and conferring an increase in the amount of the fine chemical threonine in an organism or a part thereof;
(i) nucleic acid molecule encoding a polypeptide which is isolated, e.g. from an expression library, with the aid of monoclonal antibodies against a polypeptide encoded by one of the nucleic acid molecules of (a) to (h), preferably to (a) to (c), and and conferring an increase in the amount of the fine chemical threonine in an organism or a part thereof;
(j) nucleic acid molecule which encodes a polypeptide comprising the consensus sequence having a sequences as indicated in Table IV, column 7, lines 6 to 15, 339 to 355 and conferring an increase in the amount of the fine chemical threonine in an organism or a part thereof;
(k) nucleic acid molecule comprising one or more of the nucleic acid molecule encoding the amino acid sequence of a polypeptide encoding a domain of a polypeptide indicated in Table IIA or IIB, columns 5 or 7, lines 6 to 15, 339 to 355 and conferring an increase in the amount of the fine chemical threonine in an organism or a part thereof; and
(l) nucleic acid molecule which is obtainable by screening a suitable library under stringent conditions with a probe comprising one of the sequences of the nucleic acid molecule of (a) to (k), preferably to (a) to (c), or with a fragment of at least 15 nt, preferably 20 nt, 30 nt, 50 nt, 100 nt, 200 nt or 500 nt of the nucleic acid molecule characterized in (a) to (k), preferably to (a) to (c), and conferring an increase in the amount of the fine chemical threonine in an organism or a part thereof;
or which comprises a sequence which is complementary thereto.

**[0104.0.1.1]** In one embodiment, the nucleic acid molecule of the invention distinguishes over the sequence indicated in Table IA or IB, columns 5 or 7, lines 6 to 15, 339 to 355 by one or more nucleotides. In one embodiment, the nucleic acid molecule of the present invention does not consist of the sequence shown in indicated in Table IA or IB, columns 5 or 7, lines 6 to 15, 339 to 355. In another embodiment, the nucleic acid molecule does not encode a polypeptide of a sequence indicated in Table IA or IB, columns 5 or 7, lines 6 to 15, 339 to 355.

**[0105.0.0.1] to [0107.0.0.1]: see [0105.0.0.0] to [0107.0.0.0]**

**[0108.0.1.1]** Nucleic acid molecules with the sequence as indicated in Table IA or IB, columns 5 or 7, lines 6 to 15, 339 to 355, nucleic acid molecules which are derived from an amino acid sequences as indicated in Table IIA or IIB, columns 5 or 7, lines 6 to 15, 339 to 355 or from polypeptides comprising the consensus sequence as indicated in Table IV, column 7, lines 6 to 15, 339 to 355 or their derivatives or homologues encoding polypeptides with the enzymatic or biological activity of a polypeptide as indicated in Table IIA or IIB, column 3, 5 or 7, lines 6 to 15, 339 to 355 or e.g. conferring a increase of the fine chemical threonine after increasing its expression or activity are advantageously increased in the process according to the invention.

**[0109.0.0.1] see [0109.0.0.0]**

**[0110.0.1.1]** Nucleic acid molecules, which are advantageous for the process according to the invention and which encode polypeptides with an activity of a polypeptide of the invention can be determined from generally accessible databases.

**[0111.0.0.1] see [0111.0.0.0]**

**[0112.0.1.1]** The nucleic acid molecules used in the process according to the invention take the form of isolated nucleic acid sequences, which encode polypeptides with an activity of a polypeptide as indicated in Table IIa or IIB, column 3, lines 6 to 15, 339 to 355 or having the sequence of a polypeptide as indicated in Table IIA or IIB, columns 5 and 7, lines 6 to 15, 339 to 355 and conferring an increase of the fine chemical threonine.

**[0113.0.0.1] to [0120.0.0.1]: see [0113.0.0.0] to [0120.0.0.0]**

**[0121.0.1.1]** However, it is also possible to use artificial sequences, which differ in one or more bases from the nucleic acid sequences found in organisms, or in one or more amino acid molecules from polypeptide sequences found in organisms, in particular from the polypeptide sequences indicated in Table IIA or IIB, columns 5 or 7, lines 6 to 15, 339 to 355 or the functional homologues thereof as described herein, preferably conferring above-mentioned activity, i.e. conferring a increase of the fine chemical threonine after increasing its activity

**[0122.0.0.1] to [127.0.0.1]: see [0122.0.0.0] to [0127.0.0.0]**

**[0128.0.1.1]** Synthetic oligonucleotide primers for the amplification, e.g. as the pairs indicated in Table III, column 7, lines 6 to 15, 339 to 355 by means of polymerase chain reaction can be generated on the basis of a sequence as indicated in Table IA or IB, columns 5 or 7, lines 6 to 15, 339 to 355 or the sequences derived from sequences as indicated in Table IIA or IIB, columns 5 or 7, lines 6 to 15, 339 to 355.

**[0129.0.1.1]** Moreover, it is possible to identify conserved regions from various organisms by carrying out protein sequence alignments with the polypeptide used in the process of the invention, in particular with sequences of the polypeptide of the invention, from which conserved regions, and in turn, degenerate primers can be derived. Conserved region for the polypeptide of the invention are indicated in the alignments shown in the figures. Conserved regions are those, which show a very little variation in the amino acid in one particular position of several homologs from different origin. The consenus sequences indicated in Table IV, column 7, lines 6 to 15, 339 to 355 are derived from said aligments.

**[0130.0.1.1]** Degenerated primers can then be utilized by PCR for the amplification of fragments of novel proteins having above-mentioned activity, e.g. conferring the increase of the fine chemical after increasing its expression or activity or further functional homologs of the polypeptide of the invention from other organisms.

**[0131.0.0.1] to [0138.0.0.1]: see [0131.0.0.0] to [0138.0.0.0]**

**[0139.0.1.1]** Polypeptides having above-mentioned activity, i.e. conferring a threonine increase, derived from other organisms, can be encoded by other DNA sequences which hybridize to a sequences indicated in Table IA or IB, columns 5 or 7, lines 6 to 15, 339 to 355 under relaxed hybridization conditions and which code on expression for peptides having the threonine increasing activity.

**[0140.0.0.1] to [0146.0.0.1]: see [0140.0.0.0] to [0146.0.0.0]**

**[0147.0.1.1]** Further, the nucleic acid molecule of the invention comprises a nucleic acid molecule, which is a complement of one of the nucleotide sequences of above mentioned nucleic acid molecules or a portion thereof. A nucleic acid molecule which is complementary to one of the nucleotide sequences indicated in Table IA or IB, columns 5 or 7, lines 6 to 15, 339 to 355 is one which is sufficiently complementary to one of said nucleotide sequences such that it can hybridize to one of said nucleotide sequences thereby forming a stable duplex. Preferably, the hybridisation is performed under stringent hybrization conditions. However, a complement of one of the herein disclosed sequences is preferably a sequence complement thereto according to the base pairing of nucleic acid molecules well known to the skilled person. For example, the bases A and G undergo base pairing with the bases T and U or C, resp. and visa versa. Modifications of the bases can influence the base-pairing partner.

**[0148.0.1.1]** The nucleic acid molecule of the invention comprises a nucleotide sequence which is at least about 30%, 35%, 40% or 45%, preferably at least about 50%, 55%, 60% or 65%, more preferably at least about 70%, 80%, or 90%, and even more preferably at least about 95%, 97%, 98%, 99% or more homologous to a nucleotide sequence indicated in Table IA or IB, columns 5 or 7, lines 6 to 15, 339 to 355 or a functional portion thereof and preferably has above mentioned activity, in particular has the-fine-chemical-increasing activity after increasing its acitivity or an activity of a product of a gene encoding said sequence or its homog's.

**[0149.0.1.1]** The nucleic acid molecule of the invention comprises a nucleotide sequence which hybridizes, preferably hybridizes under stringent conditions as defined herein, to one of the nucleotide sequences indicated in Table IA or IB, columns 5 or 7, lines 6 to 15, 339 to 355 or a portion thereof and encodes a protein having above-mentioned activity, e.g. conferring an increase of the fine chemical.

**[00149.1.1.1]** Optionally, the nucleotide sequence, which hybridises to one of the nucleotide sequences indicated in Table IA or IB, columns 5 or 7, lines 6 to 15, 339 to 355 has further one or more of the activities annotated or known for the a protein as indicated in Table IIA or IIB, column 3, lines 6 to 15, 339 to 355.

**[0150.0.1.1]** Moreover, the nucleic acid molecule of the invention can comprise only a portion of the coding region of one of the sequences indicated in Table IA or IB, columns 5 or 7, lines 6 to 15, 339 to 355 for example a fragment which can be used as a probe or primer or a fragment encoding a biologically active portion of the polypeptide of the present invention or of a polypeptide used in the process of the present invention, i.e. having above-mentioned activity, e.g.conferring an increase of fine chemical threonine if its activity is increased. The nucleotide sequences determined from the cloning of the present protein-according-to-the-invention-encoding gene allows for the generation of probes and primers designed for use in identifying and/or cloning its homologues in other cell types and organisms. The probe/primer typically comprises substantially purified oligonucleotide. The oligonucleotide typically comprises a region of nucleotide sequence that hybridizes under stringent conditions to at least about 12, 15 preferably about 20 or 25, more preferably about 40, 50 or 75 consecutive nucleotides of a sense strand of one of the sequences indicated in Table IA or IB, columns 5 or 7, lines 6 to 15, 339 to 355, an anti-sense sequence of one of the sequences indicated in Table IA or IB, columns 5 or 7, lines 6 to 15, 339 to 355 or naturally occurring mutants thereof. Primers based on a nucleotide of invention can be used in PCR reactions to clone homologues of the polypeptide of the invention or of the polypeptide used in the process of the invention, e.g. as the primers described in the examples of the present invention, e.g. as shown in the examples. A PCR with the primer pairs indicated in Table III, column 7, lines 6 to 15, 339 to 355 will result in a fragment of a polynucleotide sequence as indicated in Table IA or IB, columns 5 or 7, lines 6 to 15, 339 to 355.

**[0151.0.0.1] see [0151.0.0.0]**

**[0152.0.1.1]** The nucleic acid molecule of the invention encodes a polypeptide or portion thereof which includes an amino acid sequence which is sufficiently homologous to an amino acid sequence as indicated in Table IIA or IIB, columns 5 or 7, lines 6 to 15, 339 to 355 such that the protein or portion thereof maintains the ability to participate in threonine production, in particular a threonine increasing activity as mentioned above or as described in the examples in plants or microorganisms is comprised.

**[0153.0.1.1]** As used herein, the language "sufficiently homologous" refers to proteins or portions thereof which have amino acid sequences which include a minimum number of identical or equivalent amino acid residues (e.g., an amino acid residue which has a similar side chain as an amino acid residue in one of the sequences of the polypeptide of the present invention) to an amino acid sequence as indicated in Table IIA or IIB, columns 5 or 7, lines 6 to 15, 339 to 355 such that the protein or portion thereof is able to participate in the increase of threonine production. In one embodiment, a protein or portion thereof as indicated in Table IIA or IIB, columns 5 or 7, lines 6 to 15, 339 to 355 has for example an activity of a polypeptide indicated in Table IIA or IIB, column 3, lines 6 to 15, 339 to 355.

**[0154.0.1.1]** In one embodiment, the nucleic acid molecule of the present invention comprises a nucleic acid that encodes a portion of the protein of the present invention. The protein is at least about 30%, 35%, 40%, 45% or 50%, preferably at least about 55%, 60%, 65% or 70%, and more preferably at least about 75%, 80%, 85%, 90%, 91%, 92%, 93% or 94% and most preferably at least about 95%, 97%, 98%, 99% or more homologous to an entire amino acid sequence as indicated in Table IIA or IIB, columns 5 or 7, lines 6 to 15, 339 to 355 and has above-mentioned activity, e.g.conferring preferably the increase of the fine chemical.

**[0155.0.0.1] see [0155.0.0.0]**

**[0156.0.0.1] see [0156.0.0.0]**

**[0157.0.1.1]** The invention further relates to nucleic acid molecules that differ from one of a nucleotide sequences as indicated in Table IA or IB, columns 5 or 7, lines 6 to 15, 339 to 355 (and portions thereof) due to degeneracy of the genetic code and thus encode a polypeptide of the present invention, in particular a polypeptide having above mentioned activity, e.g. conferring an increase in threonine in an organism, e.g. as that polypeptides comprising the consensus sequences as indicated in Table IV, columns 5 or 7, lines 6 to 15, 339 to 355 or of the polypeptide as indicated in Table IIA or IIB, columns 5 or 7, lines 6 to 15, 339 to 355 or their functional homologues. Advantageously, the nucleic acid molecule of the invention comprises, or in an other embodiment has, a nucleotide sequence encoding a protein comprising, or in another embodiment having, a consensus sequences as indicated in Table IV, columns 5 or 7, lines 6 to 15, 339 to 355 or of the polypeptide as as indicated in Table IIA or IIB, columns 5 or 7, lines 6 to 15, 339 to 355 or the functional homologues. In a still further embodiment, the nucleic acid molecule of the invention encodes a full length protein which is substantially homologous to an amino acid sequence comprising a consensus sequence as indicated in Table IV, column 7, lines 6 to 15, 339 to 355, or of a polypeptide as indicated in Table IIA or IIB, columns 5 or 7, lines 6 to 15, 339 to 355 or the functional homologues thereof. However, in a preferred embodiment, the nucleic acid molecule of the present invention does not consist of a sequence as indicated in Table IA columns 5 or 7, lines 6 to 15, 339 to 355.

**[0158.0.0.1] to [0160.0.0.1]: see [0158.0.0.0] to [0160.0.0.0]**

**[0161.0.1.1]** Accordingly, in another embodiment, a nucleic acid molecule of the invention is at least 15, 20, 25 or 30 nucleotides in length. Preferably, it hybridizes under stringent conditions to a nucleic acid molecule comprising a nucleotide sequence of the nucleic acid molecule of the present invention or used in the process of the present invention, e.g. comprising a sequence as indicated in Table IA or IB, columns 5 or 7, lines 6 to 15, 339 to 355. The nucleic acid molecule is preferably at least 20, 30, 50, 100, 250 or more nucleotides in length.

**[0162.0.0.1]: see [0162.0.0.0]**

**[0163.0.1.1]** Preferably, nucleic acid molecule of the invention that hybridizes under stringent conditions to a sequence as indicated in Table IA or IB, columns 5 or 7, lines 6 to 15, 339 to 355 corresponds to a naturally-occurring nucleic acid molecule of the invention. As used herein, a "naturally-occurring" nucleic acid molecule refers to an RNA or DNA molecule having a nucleotide sequence that occurs in nature (e.g., encodes a natural protein). Preferably, the nucleic acid molecule encodes a natural protein having above-mentioned activity, e.g. conferring the fine chemical increase after increasing the expression or activity thereof or the activity of a protein of the invention or used in the process of the invention.

**[0164.0.0.1]: see [0164.0.0.0]**

**[0165.0.1.1]** For example, nucleotide substitutions leading to amino acid substitutions at "non-essential" amino acid residues can be made in a sequence of the nucleic acid molecule of the invention or used in the process of the invention, e.g. as indicated in Table IA or IB, columns 5 or 7, lines 6 to 15, 339 to 355.

**[0166.0.0.1]: see [0166.0.0.0]**

**[0167.0.0.1] see [0167.0.0.0]**

**[0168.0.1.1]** Accordingly, the invention relates to nucleic acid molecules encoding a polypeptide having above-mentioned activity, e.g. conferring an increase in the fine chemical in an organisms or parts thereof that contain changes in amino acid residues that are not essential for said activity. Such polypeptides differ in amino acid sequence from a sequence contained in a sequence as indicated in Table IIA or IIB, columns 5 or 7, lines 6 to 15, 339 to 355 yet retain said activity described herein. The nucleic acid molecule can comprise a nucleotide sequence encoding a polypeptide, wherein the polypeptide comprises an amino acid sequence at least about 50% identical to an amino acid sequence as indicated in Table IIA or IIB, columns 5 or 7, lines 6 to 15, 339 to 355 and is capable of participation in the increase of production of the fine chemical after increasing its activity, e.g. its expression. Preferably, the protein encoded by the nucleic acid molecule is at least about 60% identical to a sequence as indicated in Table IIA or IIB, columns 5 or 7, lines 6 to 15, 339 to 355 more preferably at least about 70% identical to one of the sequences as indicated in Table IIA or IIB, columns 5 or 7, lines 6 to 15, 339 to 355 even more preferably at least about 80%, 90% or 95% homologous to a sequence as indicated in Table IIA or IIB, columns 5 or 7, lines 6 to 15, 339 to 355 and most preferably at least about 96%, 97%, 98%, or 99% identical to the sequence as indicated in Table IIA or IIB, columns 5 or 7, lines 6 to 15, 339 to 355.

**[0169.0.0.1] to [0172.0.0.1]: see [0169.0.0.0] to [0172.0.0.0]**

**[0173.0.1.1]** For example a sequence which has a 80% homology with sequence SEQ ID NO: 40199 at the nucleic acid level is understood as meaning a sequence which, upon comparison with the sequence SEQ ID NO: 40199 by the above Gap program algorithm with the above parameter set, has a 80% homology.

**[0174.0.0.1]: see [0174.0.0.0]**

**[0175.0.1.1]** For example a sequence which has a 80% homology with sequence SEQ ID NO: 40200 at the protein level is understood as meaning a sequence which, upon comparison with the sequence SEQ ID NO: 40200 by the above program algorithm with the above parameter set, has a 80% homology.

**[0176.0.1.1]** Functional equivalents derived from one of the polypeptides as indicated in Table IIA or IIB, columns 5 or 7, lines 6 to 15, 339 to 355 according to the invention by substitution, insertion or deletion have at least least 30%, 35%, 40%, 45% or 50%, preferably at least 55%, 60%, 65% or 70% by preference at least 80%, especially preferably at least 85% or 90%, 91%, 92%, 93% or 94%, very especially preferably at least 95%, 97%, 98% or 99% homology with one of the polypeptides as indicated in Table IIA or IIB, columns 5 or 7, lines 6 to 15, 339 to 355 according to the invention and are distinguished by essentially the same properties as a polypeptide as indicated in Table IIA or IIB, columns 5 or 7, lines 6 to 15, 339 to 355.

**[0177.0.1.1]** Functional equivalents derived from a nucleic acid sequence as indicated in Table IA or IB, columns 5 or 7, lines 6 to 15, 339 to 355 according to the invention by substitution, insertion or deletion have at least 30%, 35%, 40%, 45% or 50%, preferably at least 55%, 60%, 65% or 70% by preference at least 80%, especially preferably at least 85% or 90%, 91%, 92%, 93% or 94%, very especially preferably at least 95%, 97%, 98% or 99% homology with one of a polypeptides as indicated in Table IIA or IIB, columns 5 or 7, lines 6 to 15, 339 to 355 according to the invention and encode polypeptides having essentially the same properties as a polypeptide as indicated in Table IIA or IIB, columns 5 or 7, lines 6 to 15, 339 to 355.

**[0178.0.0.1] see [0178.0.0.0]**

**[0179.0.1.1]** A nucleic acid molecule encoding an homologous to a protein sequence of as indicated in Table IIA or IIB, columns 5 or 7, lines 6 to 15, 339 to 355 can be created by introducing one or more nucleotide substitutions, additions or deletions into a nucleotide sequence of the nucleic acid molecule of the present invention, in particular as indicated in Table IA or IB, columns 5 or 7, lines 6 to 15, 339 to 355 such that one or more amino acid substitutions, additions or deletions are introduced into the encoded protein. Mutations can be introduced into the encoding sequences of sequences as indicated in Table IA or IB, columns 5 or 7, lines 6 to 15, 339 to 355 by standard techniques, such as site-directed mutagenesis and PCR-mediated mutagenesis.

**[0180.0.0.1] to [0183.0.0.1]: see [0180.0.0.0] to [0183.0.0.0]**

**[0184.0.1.1]** Homologues of the nucleic acid sequences used, with a sequence as indicated in Table IA or IB, columns 5 or 7, lines 6 to 15, 339 to 355, or of the nucleic acid sequences derived from a sequences as indicated in Table IIA or IIB, columns 5 or 7, lines 6 to 15, 339 to 355 comprise also allelic variants with at least approximately 30%, 35%, 40% or 45% homology, by preference at least approximately 50%, 60% or 70%, more preferably at least approximately 90%, 91%, 92%, 93%, 94% or 95% and even more preferably at least approximately 96%, 97%, 98%, 99% or more homology with one of the nucleotide sequences shown or the abovementioned derived nucleic acid sequences or their homologues, derivatives or analogues or parts of these. Allelic variants encompass in particular functional variants which can be obtained by deletion, insertion or substitution of nucleotides from the sequences shown, preferably from a sequence as indicated in Table IA or IB, columns 5 or 7, lines 6 to 15, 339 to 355 or from the derived nucleic acid sequences, the intention being, however, that the enzyme activity or the biological activity of the resulting proteins synthesized is advantageously retained or increased.

**[0185.0.1.1]** In one embodiment of the present invention, the nucleic acid molecule of the invention or used in the process of the invention comprises one more sequence as indicated in Table IA or IB, columns 5 or 7, lines 6 to 15, 339 to 355. In one embodiment it is preferred that the nucleic acid molecule comprises as little as possible other nucleotide sequences not shown in any one of sequences as indicated in Table IA or IB, columns 5 or 7, lines 6 to 15, 339 to 355. In one embodiment, the nucleic acid molecule comprises less than 500, 400, 300, 200, 100, 90, 80, 70, 60, 50 or 40 further nucleotides. In a further embodiment, the nucleic acid molecule comprises less than 30, 20 or 10 further nucleotides. In one embodiment, a nucleic acid molecule use in the process of the invention is identical to a sequence as indicated in Table IA or IB, columns 5 or 7, lines 6 to 15, 339 to 355.

**[0186.0.1.1]** Also preferred is that one or more nucleic acid molecule(s) used in the process of the invention encodes a polypeptide comprising a sequence as indicated in Table IIA or IIB, columns 5 or 7, lines 6 to 15, 339 to 355. In one embodiment, the nucleic acid molecule encodes less than 150, 130, 100, 80, 60, 50, 40 or 30 further amino acids. In a further embodiment, the encoded polypeptide comprises less than 20, 15, 10, 9, 8, 7, 6 or 5 further amino acids. In one embodiment, the encoded polynucleotide used in the process of the invention is identical to the sequences as indicated in Table IIA or IIB, columns 5 or 7, lines 6 to 15, 339 to 355.

**[0187.0.1.1]** In one embodiment, the nucleic acid molecule of the invention or used in the process encodes a polypeptide comprising a sequence as indicated in Table IIA or IIB, columns 5 or 7, lines 6 to 15, 339 to 355 comprises less than 100 further nucleotides. In a further embodiment, said nucleic acid molecule comprises less than 30 further nucleotides. In one embodiment, the nucleic acid molecule used in the process is identical to a coding sequence encoding a sequences as indicated in Table IIA or IIB, columns 5 or 7, lines 6 to 15, 339 to 355.

**[0188.0.1.1]** Polypeptides (= proteins), which still have the essential enzymatic activity of the polypeptide of the present invention conferring an increase of the fine chemical i.e. whose activity is essentially not reduced, are polypeptides with at least 10% or 20%, by preference 30% or 40%, especially preferably 50% or 60%, very especially preferably 80% or 90 or more of the wild type biological activity or enzyme activity, advantageously, the activity is essentially not reduced in comparison with the activity of a polypeptide as indicated in Table IIA or IIB, columns 5 or 7, lines 6 to 15, 339 to 355, preferably compared to a sequence as indicated in Table IIA or IIB, column 3 and 5, lines 6 to 15, 339 to 355 and expressed under identical conditions.

**[0189.0.1.1]** Homologues of sequences as indicated in Table IA or IB, columns 5 or 7, lines 6 to 15, 339 to 355 or of derived sequences as indicated in Table IIA or IIB, columns 5 or 7, lines 6 to 15, 339 to 355 also mean truncated sequences, cDNA, single-stranded DNA or RNA of the coding and noncoding DNA sequence. Homologues of said sequences are also understood as meaning derivatives which comprise noncoding regions such as, for example, UTRs, terminators, enhancers or promoter variants. The promoters upstream of the nucleotide sequences stated can be modified by one or more nucleotide substitution(s), insertion(s) and/or deletion(s) without, however, interfering with the functionality or activity either of the promoters, the open reading frame (= ORF) or with the 3'-regulatory region such as terminators or other 3'regulatory regions, which are far away from the ORF. It is furthermore possible that the activity of the promoters is increased by modification of their sequence, or that they are replaced completely by more active promoters, even promoters from heterologous organisms. Appropriate promoters are known to the person skilled in the art and are mentioned herein below.

**[0190.0.0.1] to [0203.0.0.1]: see [0190.0.0.0] to [0203.0.0.0]**

**[0204.0.1.1]** Accordingly, in one embodiment, the invention relates to a nucleic acid molecule which comprises a nucleic acid molecule selected from the group consisting of:
(a) nucleic acid molecule encoding, preferably at least the mature form, of a polypeptide as indicated in Table II, columns 5 or 7, lines 6 to 15, 339 to 355, preferably of Table IIB, column 7, lines 6 to 15, lines 339 to 355 or a fragment thereof conferring an increase in the amount of the respective fine chemical threonine in an organism or a part thereof
(b) nucleic acid molecule comprising, preferably at least the mature form, of a nucleic acid molecule as indicated in Table I, columns 5 or 7, lines 6 to 15, 339 to 355, preferably of Table IB, column 7, lines 6 to 15, lines 339 to 355 or a fragment thereof conferring an increase in the amount of the respective fine chemical threonine in an organism or a part thereof;
(c) nucleic acid molecule whose sequence can be deduced from a polypeptide sequence encoded by a nucleic acid molecule of (a) or (b) as result of the degeneracy of the genetic code and conferring an increase in the amount of the respective fine chemical threonine in an organism or a part thereof;
(d) nucleic acid molecule encoding a polypeptide whose sequence has at least 50% identity with the amino acid sequence of the polypeptide encoded by the nucleic acid molecule of (a) to (c) and conferring an increase in the amount of the respective fine chemical threonine in an organism or a part thereof;
(e) nucleic acid molecule which hybridizes with a nucleic acid molecule of (a) to (c) under under stringent hybridisation conditions and conferring an increase in the amount of the respective fine chemical threonine in an organism or a part thereof;
(f) nucleic acid molecule encoding a polypeptide, the polypeptide being derived by substituting, deleting and/or adding one or more amino acids of the amino acid sequence of the polypeptide encoded by the nucleic acid molecules (a) to (d), preferably to (a) to (c), and conferring an increase in the amount of the respective fine chemical threonine in an organism or a part thereof;
(g) nucleic acid molecule encoding a fragment or an epitope of a polypeptide which is encoded by one of the nucleic acid molecules of (a) to (e) , preferably to (a) to (c) and conferring an increase in the amount of the respective fine chemical threonine in an organism or a part thereof;
(h) nucleic acid molecule comprising a nucleic acid molecule which is obtained by amplifying a cDNA library or a genomic library using primers or primer pairs as indicated in Table IIIA or IIIB, column 7, lines 6 to 15, 339 to 355 and conferring an increase in the amount of the respective fine chemical threonine in an organism or a part thereof;
(i) nucleic acid molecule encoding a polypeptide which is isolated, e.g. from a expression library, with the aid of monoclonal antibodies against a polypeptide encoded by one of the nucleic acid molecules of (a) to (g), preferably to (a) to (c) and conferring an increase in the amount of the respective fine chemical threonine in an organism or a part thereof;
(j) nucleic acid molecule which encodes a polypeptide comprising the consensus sequence as indicated in Table IV, column 7, lines 6 to 15, 339 to 355 and conferring an increase in the amount of the respective fine chemical threonine in an organism or a part thereof;
(k) nucleic acid molecule encoding the amino acid sequence of a polypeptide encoding a domaine of a polypeptide as indicated in Table II, columns 5 or 7, lines 6 to 15, 339 to 355 , preferably of Table IIB, column 7, lines 6 to 15, lines 339 to 355 and conferring an increase in the amount of the respective fine chemical threonine in an organism or a part thereof; and
(l) nucleic acid molecule which is obtainable by screening a suitable nucleic acid library under stringent hybridization conditions with a probe comprising one of the sequences of the nucleic acid molecule of (a) to (k) or with a fragment of at least 15 nt, preferably 20 nt, 30 nt, 50 nt, 100 nt, 200 nt or 500 nt of the nucleic acid molecule characterized in (a) to (h) or of a nucleic acid molecule as indicated in Table I, columns 5 or 7, lines 6 to 15, 339 to 355, preferably of Table IB, column 7, lines 6 to 15, lines 339 to 355 or a nucleic acid molecule encoding, preferably at least the mature form of, the polypeptide as indicated in Table II, columns 5 or 7, lines 6 to 15, 339 to 355, preferably of Table IIB, column 7, lines 6 to 15, lines 339 to 355 and conferring an increase in the amount of the respective fine chemical threonine in an organism or a part thereof;
or which encompasses a sequence which is complementary thereto;
whereby, preferably, the nucleic acid molecule according to (a) to (I) distinguishes over a sequence depicted in as indicated in Table IA or IB, columns 5 or 7, lines 6 to 15, 339 to 355 by one or more nucleotides. In one embodiment, the nucleic acid molecule of the invention does not consist of a sequence as indicated in Table IA or IB, columns 5 or 7, lines 6 to 15, 339 to 355. In one embodiment, the nucleic acid molecule is less than 100%, 99,999%, 99,99%, 99,9% or 99% identical to a sequence indicated in Table I A or I B, columns 5 or 7, lines 6 to 15, 339 to 355. In another embodiment, the nucleic acid molecule does not encode a polypeptide of a sequence indicated in Table II A or II B, columns 5 or 7, lines 6 to 15, 339 to 355. In an other embodiment, the nucleic acid molecule of the present invention is at least 30 %, 40 %, 50%, or 60% identical and less than 100%, 99,999%, 99,99%, 99,9% or 99% identical to a sequence indicated in Table I A or I B, columns 5 or 7, lines 6 to 15, 339 to 355. In a further embodiment the nucleic acid molecule does not encode a polypeptide sequence as indicated in Table II A or II B, columns 5 or 7, lines 6 to 15, 339 to 355. Accordingly, in one embodiment, the nucleic acid molecule of the differs at least in one or more residues from a nucleic acid molecule indicated in Table I A or I B, columns 5 or 7, lines 6 to 15, 339 to 355. Accordingly, in one embodiment, the nucleic acid molecule of the present invention encodes a polypeptide, which differs at least in one or more amino acids from a polypeptide indicated in Table II A or I B, columns 5 or 7, lines 6 to 15, 339 to 355. In another embodiment, a nucleic acid molecule indicated in Table I A or I B, columns 5 or 7, lines 6 to 15, 339 to 355 does not encode a protein of a sequence indicated in Table II A or II B, columns 5 or 7, lines 6 to 15, 339 to 355. Accordingly, in one embodiment, the protein encoded by a sequences of a nucleic acid accoriding to (a) to (I) does not consist of a sequence as indicated in Table II A or II B, columns 5 or 7, lines 6 to 15, 339 to 355. In a further embodiment, the protein of the present invention is at least 30 %, 40 %, 50%, or 60% identical to a protein sequence indicated in Table II A or II B, columns 5 or 7, lines 6 to 15, 339 to 355 and less than 100%, preferably less than 99,999%, 99,99% or 99,9%, more preferably less than 99%, 985, 97%, 96% or 95% identical to a sequence as indicated in Table I A or II B, columns 5 or 7, lines 6 to 15, 339 to 355.

**[0205.0.0.1] to [0226.0.0.1]: see [0205.0.0.0] to [0226.0.0.0]**

**[0227.0.1.1]** The abovementioned nucleic acid molecules can be cloned into the nucleic acid constructs or vectors according to the invention in combination together with further genes, or else different genes are introduced by transforming several nucleic acid constructs or vectors (including plasmids) into a host cell, advantageously into a plant cell or a microorgansims.
In addition to a sequence as indicated in Table IA or IB, columns 5 or 7, lines 6 to 15, 339 to 355 or its derivatives, it is advantageous additionally to express and/or mutate further genes in the organisms. Especially advantageously, additionally at least one further gene of the amino acid biosynthetic pathway such as for L-lysine, L-methionine and/or L-threonine is expressed in the organisms such as plants or microorganisms. It is also possible that the regulation of the natural genes has been modified advantageously so that the gene and/or its gene product is no longer subject to the regulatory mechanisms which exist in the organisms. This leads to an increased synthesis of the amino acid desired since, for example, feedback regulations no longer exist to the same extent or not at all. In addition it might be advantageously to combine sequences as indicated in Table IA or IB, columns 5 or 7, lines 6 to 15, 339 to 355 with genes which generally support or enhances to growth or yield of the target organismen, for example genes which lead to faster growth rate of microorganisms or genes which produces stress-, pathogen, or herbicide resistant plants.

**[0228.0.0.1] to [0230.0.0.1]: see [0228.0.0.0] to [0230.0.0.0]**

**[0231.0.1.1]** In a further advantageous embodiment of the process of the invention, the organisms used in the process are those in which simultaneously a threonine degrading protein is attenuated, in particular by reducing the rate of expression of the corresponding gene.

**[0232.0.0.1] to [0282.0.0.1]: see [0232.0.0.0] to [0282.0.0.0]**

**[0283.0.1.1]** Moreover, a native polypeptide conferring the increase of the fine chemical threonine in an organism or part thereof can be isolated from cells (e.g., endothelial cells), for example using the antibody of the present invention as described below, in particular, an antibody against a protein as indicated in Table IIA or IIB, column 3, lines 6 to 15, 339 to 355. E.g. an antibody against a polypeptide as indicated in Table IIA or IIB, columns 5 or 7, lines 6 to 15, 339 to 355 which can be produced by standard techniques utilizing polypeptides comprising or consisting of above mentioned sequences, e.g. the polypeptid of the present invention or fragment thereof. Preferred are monoclonal antibodies.

**[0284.0.0.1]: see [0284.0.0.0]**

**[0285.0.1.1]** In one embodiment, the present invention relates to a polypeptide having a sequence as indicated in Table IIA or IIB, columns 5 or 7, lines 6 to 15, 339 to 355 or as coded by a nucleic acid molecule as indicated in Table IIA or IIB, columns 5 or 7, lines 6 to 15, 339 to 355 or functional homologues thereof.

**[0286.0.1.1]** In one advantageous embodiment, in the method of the present invention the activity of a polypeptide is increased which comprises or consists of a consensus sequence as indicated in Table IV, column 7, lines 6 to 15, 339 to 355 and in one other embodiment, the present invention relates to a polypeptide comprising or consisting of a consensus sequence as indicated in Table IV, column 7, lines 6 to 15, 339 to 355 whereby 20 or less, preferably 15 or 10, preferably 9, 8, 7, or 6, more preferred 5 or 4, even more preferred 3, even more preferred 2, even more preferred 1, most preferred 0 of the amino acids positions indicated can be replaced by any amino acid.

**[0287.0.0.1] to [0290.0.0.1]: see [0287.0.0.0] to [0290.0.0.0]**

**[0291.0.1.1]** In one advantageous embodiment, the method of the present invention comprises the increasing of a polypeptide comprising or consisting of plant or microorganism specific consensus sequences.
Accordingly, in one embodiment, the present invention relates to a polypeptide comprising or consisting of plant or microorganism specific consensus sequences.
In one embodiment, said polypeptide of the invention distinguishes over a sequence as indicated in Table IIA or IIB, columns 5 or 7, lines 6 to 15, 339 to 355 by one or more amino acids. In one embodiment, the polypeptide distinguishes from a sequence as indicated in Table IIA or IIB, columns 5 or 7, lines 6 to 15, 339 to 355 by more than1, 2, 3, 4, 5, 6, 7, 8 or 9 amino acids, preferably by more than 10, 15, 20, 25 or 30 amino acids, even more preferred are more than 40, 50, or 60 amino acids and, preferably, the sequence of the polypeptide of the invention distinguishes from a sequence as indicated in Table IIA or IIB, columns 5 or 7, lines 6 to 15, 339 to 355 by not more than 80% or 70% of the amino acids, preferably not more than 60% or 50%, more preferred not more than 40% or 30%, even more preferred not more than 20% or 10%. In another embodiment, said polypeptide of the invention does not consist of a sequence as indicated in Table IIA or IIB, columns 5 or 7, lines 6 to 15, 339 to 355.

**[0292.0.0.1]: see [0292.0.0.0]**

**[0293.0.1.1]** In one embodiment, the invention relates to a polypeptide conferring an increase in the fine chemical threonine in an organism or part being encoded by the nucleic acid molecule of the invention or by the nucleic acid molecule of the invention used in the process of the invention.
In one embodiment, the poylpeptide of the invention is having a sequence which distinguishes from a sequence as indicated in Table IIA or IIB, columns 5 or 7, lines 6 to 15, 339 to 355 by one or more amino acids. In another embodiment, said polypeptide of the invention does not consist of the sequence as indicated in Table IIA or IIB, columns 5 or 7, lines 6 to 15, 339 to 355. In a further embodiment, said polypeptide of the present invention is less than 100%, 99,999%, 99,99%, 99,9% or 99% identical. In one embodiment, said polypeptide does not consist of the sequence encoded by a nucleic acid molecules as indicated in Table IA or IB, columns 5 or 7, lines 6 to 15, 339 to 355.

**[0294.0.1.1]** In one embodiment, the present invention relates to a polypeptide having an activity of a protein as indicated in Table IIA or IIB, column 3, lines 6 to 15, 339 to 355, which distinguishes over a sequence as indicated in Table IIA or IIB, columns 5 or 7, lines 6 to 15, 339 to 355 by one or more amino acids, preferably by more than 5, 6, 7, 8 or 9 amino acids, preferably by more than 10, 15, 20, 25 or 30 amino acids, even more preferred are more than 40, 50, or 60 amino acids but even more preferred by less than 70% of the amino acids, more preferred by less than 50%, even more preferred my less than 30% or 25%, more preferred are 20% or 15%, even more preferred are less than 10%.

**[0295.0.0.1] to [0297.0.0.1]: see [0295.0.0.0] to [0297.0.0.0]**

**[0297.1.0.1]** Non-polypeptide of the invention-chemicals are e.g. polypeptides having not the activity of a polypeptide indicated in Table IIA or IIB, columns 3, 5 or 7, lines 6 to 15, 339 to 355.

**[0298.0.1.1]** A polypeptide of the invention can participate in the process of the present invention. The polypeptide or a portion thereof comprises preferably an amino acid sequence which is sufficiently homologous to an amino acid sequence as indicated in Table IIA or IIB, columns 5 or 7, lines 6 to 15, 339 to 355 such that the protein or portion thereof maintains the ability to confer the activity of the present invention. The portion of the protein is preferably a biologically active portion as described herein. Preferably, the polypeptide used in the process of the invention has an amino acid sequence identical to a sequence as indicated in Table IIA or IIB, columns 5 or 7, lines 6 to 15, 339 to 355.

**[0299.0.1.1]** Further, the polypeptide can have an amino acid sequence which is encoded by a nucleotide sequence which hybridizes, preferably hybridizes under stringent conditions as described above, to a nucleotide sequence of the nucleic acid molecule of the present invention. Accordingly, the polypeptide has an amino acid sequence which is encoded by a nucleotide sequence that is at least about 35%, 40%, 45%, 50%, 55%, 60%, 65% or 70%, preferably at least about 75%, 80%, 85% or 90, and more preferably at least about 91%, 92%, 93%, 94% or 95%, and even more preferably at least about 96%, 97%, 98%, 99% or more homologous to one of the nucleotide sequence as indicated in Table IA or IB, columns 5 or 7, lines 6 to 15, 339 to 355. The preferred polypeptide of the present invention preferably possesses at least one of the activities according to the invention and described herein. A preferred polypeptide of the present invention includes an amino acid sequence encoded by a nucleotide sequence which hybridizes, preferably hybridizes under stringent conditions, to a nucleotide sequence as indicated in Table IA or IB, columns 5 or 7, lines 6 to 15, 339 to 355 or which is homologous thereto, as defined above.

**[0300.0.1.1]** Accordingly the polypeptide of the present invention can vary from a sequence as indicated in Table IIA or IIB, columns 5 or 7, lines 6 to 15, 339 to 355 in the amino acid sequence due to natural variation or mutagenesis, as described in detail herein. Accordingly, the polypeptide comprises an amino acid sequence which is at least about 35%, 40%, 45%, 50%, 55%, 60%, 65% or 70%, preferably at least about 75%, 80%, 85% or 90, and more preferably at least about 91%, 92%, 93%, 94% or 95%, and most preferably at least about 96%, 97%, 98%, 99% or more homologous to an entire amino acid sequence of a sequence as indicated in Table IIA or IIB, columns 5 or 7, lines 6 to 15, 339 to 355.

**[0301.0.0.1] see [0301.0.0.0]**

**[0302.0.1.1]** Biologically active portions of a polypeptide of the present invention include peptides comprising amino acid sequences derived from the amino acid sequence of the polypeptide of the present invention or used in the process of the present invention, e.g., an amino acid sequence as indicated in Table IIA or IIB, columns 5 or 7, lines 6 to 15, 339 to 355 or the amino acid sequence of a protein homologous thereto, which include fewer amino acids than a full length polypeptide of the present invention or used in the process of the present invention or the full length protein which is homologous to a polypeptide of the present invention or used in the process of the present invention depicted herein, and exhibit at least one activity of a polypeptide of the present invention or used in the process of the present invention.

**[0303.0.0.1]: see [0303.0.0.0]**

**[0304.0.1.1]** Manipulation of the nucleic acid molecule of the invention may result in the production of a protein having essentially the activity of the polypeptides as indicated in Table IIA or IIB, column 3, lines 6 to 15, 339 to 355 but having differences in the sequence from said wild-type protein. These proteins may be improved in efficiency or activity, may be present in greater numbers in the cell than is usual, or may be decreased in efficiency or activity in relation to the wild type protein.

**[0305.0.0.1] to [0308.0.0.1]: see [0305.0.0.0] to [0308.0.0.0]**

**[0309.0.1.1]** In one embodiment, an reference to a "protein (= polypeptide)" of the invention or as indicated in Table IIA or IIB, columns 5 or 7, lines 6 to 15, 339 to 355 refers to a polypeptide having an amino acid sequence corresponding to the polypeptide of the invention or used in the process of the invention, whereas a "non-polypeptide of the invention" or "other polypeptide" not being indicated in Table IIA or IIB, columns 5 or 7, lines 6 to 15, 339 to 355 refers to a polypeptide having an amino acid sequence corresponding to a protein which is not substantially homologous to a polypeptide of the invention, preferably which is not substantially homologous to a polypeptide as indicated in Table IIA or IIB, columns 5 or 7, lines 6 to 15, 339 to 355 e.g., a protein which does not confer the activity described herein or annotated or known for as indicated in Table IIA or IIB, column 3, lines 6 to 15, 339 to 355 and which is derived from the same or a different organism. In one embodiment a "non-polypeptide of the invention" or "other polypeptide" not being indicate in Table IIA or IIB, columns 5 or 7, lines 6 to 15, 339 to 355 does not confer an increase of the fine chemical in an organism or part thereof.

**[0310.0.0.1] to [0334.0.0.1]: see [0310.0.0.0] to [0334.0.0.0]**

**[0335.0.1.1]** The dsRNAi method has proved to be particularly effective and advantageous for reducing the expression of a nucleic acid sequences as indicated in Table II A or IIB, columns 5 or 7, lines 6 to 15, 339 to 355 and/or homologs thereof. As described inter alia in WO 99/32619, dsRNAi approaches are clearly superior to traditional antisense approaches. The invention therefore furthermore relates to double-stranded RNA molecules (dsRNA molecules) which, when introduced into an organism, advantageously into a plant (or a cell, tissue, organ or seed derived therefrom), bring about altered metabolic activity by the reduction in the expression of a nucleic acid sequences as indicated in Table IA or IB, columns 5 or 7, lines 6 to 15, 339 to 355 and/or homologs thereof. In a double-stranded RNA molecule for reducing the expression of an protein encoded by a nucleic acid sequence of one of the sequences as indicated in Table IIA or IIB, columns 5 or 7, lines 6 to 15, 339 to 355 and/or homologs thereof, one of the two RNA strands is essentially identical to at least part of a nucleic acid sequence, and the respective other RNA strand is essentially identical to at least part of the complementary strand of a nucleic acid sequence.

**[0336.0.0.1] to [0342.0.0.1]: see [0336.0.0.0] to [0342.0.0.0]**

**[0343.0.1.1]** As has already been described, 100 % sequence identity between the dsRNA and a gene transcript of a nucleic acid sequence as indicated in Table IA or IB, columns 5 or 7, lines 6 to 15, 339 to 355 or it's homolog is not necessarily required in order to bring about effective reduction in the expression. The advantage is, accordingly, that the method is tolerant with regard to sequence deviations as may be present as a consequence of genetic mutations, polymorphisms or evolutionary divergences. Thus, for example, using the dsRNA, which has been generated starting from a sequence as indicated in Table IA or IB, columns 5 or 7, lines 6 to 15, 339 to 355 or homologs thereof of the one organism, may be used to suppress the corresponding expression in another organism.

**[0344.0.0.1] to [0361.0.0.1]: see [0344.0.0.0] to [0361.0.0.0]**

**[0362.0.1.1]** Accordingly the present invention relates to any cell transgenic for any nucleic acid characterized as part of the invention, e.g. conferring the increase of the fine chemical threonine in a cell or an organism or a part thereof, e.g. the nucleic acid molecule of the invention, the nucleic acid construct of the invention, the antisense molecule of the invention, the vector of the invention or a nucleic acid molecule encoding the polypeptide of the invention, e.g. the polypeptide as indicated in Table IIA or IIB, columns 5 or 7, lines 6 to 15, 339 to 355. Due to the above mentioned activity the fine chemical content in a cell or an organism is increased. For example, due to modulation or manupulation, the cellular activity of the polypeptide of the invention or nucleic acid molecule of the invention is increased, e.g. due to an increased expression or specific activity of the subject matters of the invention in a cell or an organism or a part thereof. In one embodiment transgenic for a polypeptide having an activity of a polypeptide as indicated in Table IIA or IIB, columns 5 or 7, lines 6 to 15, 339 to 355 means herein that due to modulation or manipulation of the genome, an activity as annotated for a polypeptide as indicated in Table IIA or IIB, columns 3, lines 6 to 15, 339 to 355, e.g. having a sequence as indicated in Table IIA or IIB, columns 5 or 7, lines 6 to 15, 339 to 355 is increased in a cell or an organism or a part thereof. Examples are described above in context with the process of the invention.

**[0363.0.0.1]: see [0363.0.0.0]**

**[0364.0.1.1]** A naturally occurring expression cassette - for example the naturally occurring combination of a promoter of a polypeptide of the invention with the corresponding protein-encoding-sequence - becomes a transgenic expression cassette when it is modified by non-natural, synthetic "artificial" methods such as, for example, mutagenization. Such methods have been described (US 5,565,350; WO 00/15815; also see above).

**[0365.0.0.1] to [0382.0.0.1]: see [0365.0.0.0] to [0382.0.0.0]**

**[0383.0.1.1]** For preparing hydroxy containing fine chemicals, in particular the fine chemical threonine, it is possible to use as hydroxy source organic hydroxy-containing compounds such as, for example, alcohols, hydroxy-containing organic acids, acetals, or compounds containing carbonyl or carboxyl groups to be reduced by known methods of the art.

**[0384.0.0.1] to [0392.0.0.1]: see [0384.0.0.0] to [0392.0.0.0]**

**[0393.0.1.1]** In one embodiment, the present invention relates to a method for the identification of a gene product conferring an increase in the fine chemical production in a cell, comprising the following steps:
(a) contacting,̅ e.g. hybridising, the nucleic acid molecules of a sample, e.g. cells, tissues, plants or microorganisms or a nucleic acid library, which can contain a candidate gene encoding a gene product conferring an increase in the fine chemical threonine after expression, with the nucleic acid molecule of the present invention;
(b) identifying the nucleic acid molecules, which hybridize under relaxed stringent conditions with the nucleic acid molecule of the present invention in particular to the nucleic acid molecule sequence as indicated in Table IA or IB, columns 5 or 7, lines 6 to 15, 339 to 355 and, optionally, isolating the full length cDNA clone or complete genomic clone;
(c) introducing the candidate nucleic acid molecules in host cells, preferably in a plant cell or a microorganism, appropriate for producing the fine chemical threonine;
(d) expressing the identified nucleic acid molecules in the host cells;
(e) assaying the the fine chemical level in the host cells; and
(f) identifying the nucleic acid molecule and its gene product which expression confers an increase in the the fine chemical level in the host cell after expression compared to the wild type.

**[0394.0.0.1] to [0398.0.0.1]: see [0394.0.0.0] to [0398.0.0.0]**

**[0399.0.1.1]** Furthermore, in one embodiment, the present invention relates to a process for the identification of a compound conferring increased in the fine chemical production in a plant or microorganism, comprising the steps:
(a) culturing a cell or tissue or microorganism or maintaining a plant expressing the polypeptide according to the invention or a nucleic acid molecule encoding said polypeptide and a readout system capable of interacting with the polypeptide under suitable conditions which permit the interaction of the polypeptide with said readout system in the presence of a compound or a sample comprising a plurality of compounds and capable of providing a detectable signal in response to the binding of a compound to said polypeptide under conditions which permit the expression of said readout system and the polypeptide of the present invention or used in the process of the invention; and
(b) identifying if the compound is an effective agonist by detecting the presence or absence or increase of a signal produced by said readout system.
The screen for a gene product or an agonist conferring an increase in the fine chemical production can be performed by growth of an organism for example a microorganism in the presence of growth reducing amounts of an inhibitor of the synthesis of the fine chemical. Better growth, e.g. higher dividing rate or high dry mass in comparison to the control under such conditions would identify a gene or gene product or an agonist conferring an increase in fine chemical production.

**[0399.1.1.1]** One can think to screen for increased production of the fine chemical threonine by for example searching for a resistance to a drug blocking the synthesis of the fine chemical threonine and looking whether this effect is dependent on the activity or expression of a polypeptide as indicated in Table IIA or IIB, columns 5 or 7, lines 6 to 15, 339 to 355 or a homolog thereof, e.g. comparing the phenotyp of nearly identical organisms with low and high acitivity of a protein as indicated in Table IIA or IIB, columns 5 or 7, lines 6 to 15, 339 to 355 after incubation with the drug.

**[0400.0.0.1] to [0430.0.0.1]: see [0400.0.0.1] to [0430.0.0.0]**

**[0431.0.1.1]** Example 1: Cloning SEQ ID NO: 81 or another DNA polynucleotide according the enclosed sequence listing encoding an ORF as shown in the below table in Escherichia coli

**[0432.0.1.1]** SEQ ID NO: 81 or another DNA polynucleotide according the enclosed sequence listing encoding an ORF as shown in the below table was cloned into the plasmids pBR322 (Sutcliffe, J.G. (1979) Proc. Natl Acad. Sci. USA, 75: 3737-3741); pACYC177 (Change & Cohen (1978) J. Bacteriol. 134: 1141-1156); plasmids of the pBS series (pBSSK+, pBSSK- and others; Stratagene, LaJolla, USA) or cosmids such as SuperCos1 (Stratagene, LaJolla, USA) or Lorist6 (Gibson, T.J. Rosenthal, A., and Waterson, R.H. (1987) Gene 53: 283-286) for expression in E. coli using known, well-established procedures (see, for example, Sambrook, J. et al. (1989) "Molecular Cloning: A Laboratory Manual". Cold Spring Harbor Laboratory Press or Ausubel, F.M. et al. (1994) "Current Protocols in Molecular Biology", John Wiley & Sons).

**[0433.0.0.1] to [0460.0.0.1]: see [0433.0.0.0] to [0460.0.0.0]**

**[0461.0.1.1]** Example 10: Cloning SEQ ID NO: 81 for the expression in plants

**[0462.0.0.1]: see [0462.0.0.0]**

**[0463.0.1.1]** SEQ ID NO: 81 is amplified by PCR as described in the protocol of the Pfu Turbo or DNA Herculase polymerase (Stratagene).

**[0464.0.0.1] to [0466.0.0.1]: see [0464.0.0.0] to [0466.0.0.0]**

**[0467.0.1.1]** The following primer sequences were selected for the gene SEQ ID NO: 81:
i) forward primer SEQ ID NO: 83 :
   ATGTCGTCCTTATCCACTTCATTTG
ii) reverse primer SEQ ID NO: 84 :
   TTAATTGTAACGGCTATATCTACTGG

**[0468.0.0.1] to [0479.0.0.1]: see [0468.0.0.0] to [0479.0.0.0]**

**[0480.0.1.1]** Example 11: Generation of transgenic plants which express SEQ ID NO: 81

**[0481.0.0.1] to [0513.0.0.1]: see [0481.0.0.0] to [0513.0.0.0]**

**[0514.0.1.1]** As an alternative, the amino acids can be detected advantageously via HPLC separation in ethanolic extract as described by Geigenberger et al. (Plant Cell & Environ, 19, 1996: 43-55).
*Arabidopsis thaliana* plants were engineered as described in Example 11.
The results of the different Arabidopsis plants analysed can be seen from table 1 which follows:

**Table 1**

| **ORF** | **Annotation** | **Metabolite** | **Min** | **Max** | **Methode** |
|---|---|---|---|---|---|
| YFL050C | di- trivalent inorganic cation transporter | threonine | 1,193 | 1,557 | GC |
| YKR057W | ribosomal protein, similar to S21 ribosomal proteins, involved in ribosome biogenesis and translation | threonine | 1,34 | 2,413 | GC |
| YIL150C | chromatin binding protein, required for S-phase (DNA synthesis) initiation or completion | threonine | 1,256 | 4,186 | GC |
| YNL046W | probable membrane protein of the endoplasmatic reticulum | threonine | 1,178 | 1,526 | GC |
| YNL120C | not been characterized yet | threonine | 1,44 | 1,44 | LC |
| b0186 | lysine decarboxylase | threonine | 1,495 | 3,277 | GC |
| b0730 | transcriptional regulator of succinylcoA synthetase operon and fatty acyl response regulator | threonine | 1,531 | 2,772 | LC |
| b1829 | defined as a heat shock protein with protease activity | threonine | 1,174 | 2,135 | GC |
| b2170 | sugar efflux transporter B | threonine | 1,359 | 1,792 | LC |
| b0019 | Na+/H+ antiporter | threonine | 1,244 | 1,44 | GC |

### Metabolite Profiling Info:

| **ORF** | **Metabolite** | **Method** | **Min** | **Max** |
|---|---|---|---|---|
| b0464 | Threonine | GC | 1.23 | 1.43 |
| b1360 | Threonine | GC | 1.16 | 1.38 |
| b1738 | Threonine | LC | 1.27 | 4.61 |
| b1830 | Threonine | LC | 1.24 | 1.43 |
| b1896 | Threonine | LC + GC | 1.46 | 2.08 |
| b2414 | Threonine | GC | 1.24 | 1.46 |
| b2552 | Threonine | GC | 1.17 | 1.37 |
| b4004 | Threonine | GC | 1.17 | 1.37 |
| b2664 | Threonine | LC + GC | 1.29 | 2.84 |
| b3074 | Threonine | LC | 1.31 | 1.59 |
| b2270 | Threonine | LC | 1.31 | 1.59 |
| b3160 | Threonine | LC | 1.25 | 1.56 |
| b3231 | Threonine | GC | 1.17 | 1.32 |
| b3462 | Threonine | GC | 1.18 | 1.51 |
| b3791 | Threonine | LC | 1.38 | 1.44 |
| b3966 | Threonine | GC | 1.19 | 1.47 |
| YOR245C | Threonine | GC | 1.18 | 1.81 |

**[0515.0.0.1]: to [0552.0.0.1]: see [0515.0.0.0] to [0552.0.0.0]**

**[0552.1.0.1]:** Example 15: Metabolite Profiling Info from *Zea mays*
*Zea mays* plants were engineered as described in Example 14c.
The results of the different *Zea mays* plants analysed can be seen from table 2 which follows:

**Table 2**

| ORF_ NAME | Metabolite | Min | Max |
|---|---|---|---|
| YIL150C | Threonine | 1.680 | 2.977 |
| YKR057W | Threonine | 1.589 | 5.593 |

Table 2 describes the increase in threonine in genetically modified corn plants expressing the Saccharomyces cerevisiae nucleic acid sequence YIL150c or YKR057w.
In one embodiment, in case the activity of the Saccaromyces cerevisiae protein YIL150C or its homologs, e.g. "a chromatin binding protein, required for S-phase (DNA synthesis) initiation or completion" or its homologs, is increased in corn plants, preferably, an increase of the fine chemical threonine between 1,68 and 2,977 is conferred.
In case the activity of the Saccharomyces cerevisiae protein YKR057W or a ribosomal protein, similar to S21 ribosomal proteins, involved in ribosome biogenesis and translation or its homolog, is increased in corn plants, preferably, an increase of the fine chemical threonine between 1,589 and 5,593 is conferred.

**[00552.2.0.1] see [00552.2.0.0]**

**[0553.0.1.1]**
1. A process for the production of threonine, which comprises
   (a) increasing or generating the activity of a protein as indicated in Table IIA or IIB, columns 5 or 7, lines 6 to 15, 339 to 355 or a functional equivalent thereof in a non-human organism, or in one or more parts thereof; and
   (b) growing the organism under conditions which permit the production of threonine in said organism.
2. A process for the production of threonine, comprising the increasing or generating in an organism or a part thereof the expression of at least one nucleic acid molecule comprising a nucleic acid molecule selected from the group consisting of:
   (a) nucleic acid molecule encoding of a polypeptide as indicated in Table IIA or IIB, columns 5 or 7, lines 6 to 15, 339 to 355 or a fragment thereof, which confers an increase in the amount of threonine in an organism or a part thereof;
   (b) nucleic acid molecule comprising of the nucleic acid molecule as indicated in Table IA or IB, columns 5 or 7, lines 6 to 15, 339 to 355;
   (c) nucleic acid molecule whose sequence can be deduced from a polypeptide sequence encoded by a nucleic acid molecule of (a) or (b) as a result of the degeneracy of the genetic code and conferring an increase in the amount of threonine in an organism or a part thereof;
   (d) nucleic acid molecule which encodes a polypeptide which has at least 50% identity with the amino acid sequence of the polypeptide encoded by the nucleic acid molecule of (a) to (c) and conferring an increase in the amount of threonine in an organism or a part thereof;
   (e) nucleic acid molecule which hybidizes with a nucleic acid molecule of (a) to (c) under under stringent hybridisation conditions and conferring an increase in the amount of threonine in an organism or a part thereof;
   (f) nucleic acid molecule which encompasses a nucleic acid molecule which is obtained by amplifying nucleic acid molecules from a cDNA library or a genomic library using the primers or primer pairs as indicated in Table III, columns 5 or 7, lines 6 to 15, 339 to 355 and conferring an increase in the amount of the fine chemical threonine in an organism or a part thereof;
   (g) nucleic acid molecule encoding a polypeptide which is isolated with the aid of monoclonal antibodies against a polypeptide encoded by one of the nucleic acid molecules of (a) to (f) and conferring an increase in the amount of threonine in an organism or a part thereof;
   (h) nucleic acid molecule encoding a polypeptide comprising a consensus sequence as indicated in Table IV, columns 5 or 7, lines 6 to 15, 339 to 355 and conferring an increase in the amount of the fine chemical threonine in an organism or a part thereof; and
   (i) nucleic acid molecule which is obtainable by screening a suitable nucleic acid library under stringent hybridization conditions with a probe comprising one of the sequences of the nucleic acid molecule of (a) to (k) or with a fragment thereof having at least 15 nt, preferably 20 nt, 30 nt, 50 nt, 100 nt, 200 nt or 500 nt of the nucleic acid molecule characterized in (a) to (k) and conferring an increase in the amount of the fine chemical threonine in an organism or a part thereof.
   or comprising a sequence which is complementary thereto.
3. The process of claim 1 or 2, comprising recovering of the free or bound threonine.
4. The process of any one of claim 1 to 3, comprising the following steps:
   (a) selecting an organism or a part thereof expressing a polypeptide encoded by the nucleic acid molecule characterized in claim 2;
   (b) mutagenizing the selected organism or the part thereof;
   (c) comparing the activity or the expression level of said polypeptide in the mutagenized organism or the part thereof with the activity or the expression of said polypeptide of the selected organisms or the part thereof;
   (d) selecting the mutated organisms or parts thereof, which comprise an increased activity or expression level of said polypeptide compared to the selected organism or the part thereof;
   (e) optionally, growing and cultivating the organisms or the parts thereof; and
   (f) recovering, and optionally isolating, the free or bound threonine produced by the selected mutated organisms or parts thereof.
5. The process of any one of claims 1 to 4, wherein the activity of said protein or the expression of said nucleic acid molecule is increased or generated transiently or stably.
6. An isolated nucleic acid molecule comprising a nucleic acid molecule selected from the group consisting of:
   (a) nucleic acid molecule encoding a polypeptide as indicated in Table IIA or IIB, columns 5 or 7, lines 6 to 15, 339 to 355 or a fragment thereof, which confers an increase in the amount of threonine in an organism or a part thereof;
   (b) nucleic acid molecule comprising a nucleic acid as indicated in Table IA or IB, columns 5 or 7, lines 6 to 15, 339 to 355;
   (c) nucleic acid molecule whose sequence can be deduced from a polypeptide sequence encoded by a nucleic acid molecule of (a) or (b) as a result of the degeneracy of the genetic code and conferring an increase in the amount of threonine in an organism or a part thereof;
   (d) nucleic acid molecule which encodes a polypeptide which has at least 50% identity with the amino acid sequence of the polypeptide encoded by the nucleic acid molecule of (a) to (c) and conferring an increase in the amount of threonine in an organism or a part thereof;
   (e) nucleic acid molecule which hybidizes with a nucleic acid molecule of (a) to (c) under under stringent hybridisation conditions and conferring an increase in the amount of threonine in an organism or a part thereof;
   (f) nucleic acid molecule which encompasses a nucleic acid molecule which is obtained by amplifying nucleic acid molecules from a cDNA library or a genomic library using the primers in Table III, column 8, lines 6 to 15, 339-355 and conferring an increase in the amount of the fine chemical threonine in an organism or a part thereof;
   (g) nucleic acid molecule encoding a polypeptide which is isolated with the aid of monoclonal antibodies against a polypeptide encoded by one of the nucleic acid molecules of (a) to (f) and conferring an increase in the amount of threonine in an organism or a part thereof;
   (h) nucleic acid molecule encoding a polypeptide comprising the consensus sequence shown in Table IV, column 8, lines 6 to 15, 339 to 355 and conferring an increase in the amount of the fine chemical in an organism or a part thereof; and
   (i) nucleic acid molecule which is obtainable by screening a suitable nucleic acid library under stringent hybridization conditions with a probe comprising one of the sequences of the nucleic acid molecule of (a) to (k) or with a fragment thereof having at least 15 nt, preferably 20 nt, 30 nt, 50 nt, 100 nt, 200 nt or 500 nt of the nucleic acid molecule characterized in (a) to (k) and conferring an increase in the amount of the fine chemical in an organism or a part thereof.
   whereby the nucleic acid molecule distinguishes over the sequence as indicated in Table IA or IB, columns 5 or 7, lines 6 to 15, 339 to 355 by one or more nucleotides.
7. A nucleic acid construct which confers the expression of the nucleic acid molecule of claim 6, comprising one or more regulatory elements.
8. A vector comprising the nucleic acid molecule as claimed in claim 6 or the nucleic acid construct of claim 7.
9. The vector as claimed in claim 8, wherein the nucleic acid molecule is in operable linkage with regulatory sequences for the expression in a prokaryotic or eukaryotic, or in a prokaryotic and eukaryotic, host.
10. A host cell, which has been transformed stably or transiently with the vector as claimed in claim 9 or 10 or the nucleic acid molecule as claimed in claim 6 or the nucleic acid construct of claim 7 or produced as described in claim any one of claims 2 to 5.
11. The host cell of claim 10, which is a transgenic host cell.
12. The host cell of claim 10 or 11, which is a plant cell, an animal cell, a microorganism, or a yeast cell, a fungus cell, a prokaryotic cell, an eukaryotic cell or an archaebacterium.
13. A process for producing a polypeptide, wherein the polypeptide is expressed in a host cell as claimed in any one of claims 10 to 12.
14. A polypeptide produced by the process as claimed in claim 13 or encoded by the nucleic acid molecule as claimed in claim 6 whereby the polypeptide distinguishes over a Sequence as indicated in Table IIA or IIB, columns 5 or 7, lines 6 to 15, 339 to 355 by one or more amino acids
15. An antibody, which binds specifically to the polypeptide as claimed in claim 14.
16. A plant tissue, propagation material, harvested material or a plant comprising the host cell as claimed in claim 12 which is plant cell or an Agrobacterium.
17. A method for screening for agonists and antagonists of the activity of a polypeptide encoded by the nucleic acid molecule of claim 6 conferring an increase in the amount of threonine in an organism or a part thereof comprising:
   (a) contacting cells, tissues , plants or microorganisms which express the a polypeptide encoded by the nucleic acid molecule of claim 6 conferring an increase in the amount of threonine in an organism or a part thereof with a candidate compound or a sample comprising a plurality of compounds under conditions which permit the expression the polypeptide;
   (b) assaying the threonine level or the polypeptide expression level in the cell, tissue, plant or microorganism or the media the cell, tissue, plant or microorganisms is cultured or maintained in; and
   (c) identifying a agonist or antagonist by comparing the measured threonine level or polypeptide expression level with a standard threonine or polypeptide expression level measured in the absence of said candidate compound or a sample comprising said plurality of compounds, whereby an increased level over the standard indicates that the compound or the sample comprising said plurality of compounds is an agonist and a decreased level over the standard indicates that the compound or the sample comprising said plurality of compounds is an antagonist.
18. A process for the identification of a compound conferring increased threonine production in a plant or microorganism, comprising the steps:
   (a) culturing a plant cell or tissue or microorganism or maintaining a plant expressing the polypeptide encoded by the nucleic acid molecule of claim 6 conferring an increase in the amount of threonine in an organism or a part thereof and a readout system capable of interacting with the polypeptide under suitable conditions which permit the interaction of the polypeptide with said readout system in the presence of a compound or a sample comprising a plurality of compounds and capable of providing a detectable signal in response to the binding of a compound to said polypeptide under conditions which permit the expression of said readout system and of the polypeptide encoded by the nucleic acid molecule of claim 6 conferring an increase in the amount of threonine in an organism or a part thereof;
   (b) identifying if the compound is an effective agonist by detecting the presence or absence or increase of a signal produced by said readout system.
19. A method for the identification of a gene product conferring an increase in threonine production in a cell, comprising the following steps:
   (a) contacting the nucleic acid molecules of a sample, which can contain a candidate gene encoding a gene product conferring an increase in threonine after expression with the nucleic acid molecule of claim 6;
   (b) identifying the nucleic acid molecules, which hybridise under relaxed stringent conditions with the nucleic acid molecule of claim 6;
   (c) introducing the candidate nucleic acid molecules in host cells appropriate for producing threonine;
   (d) expressing the identified nucleic acid molecules in the host cells;
   (e) assaying the threonine level in the host cells; and
   (f) identifying nucleic acid molecule and its gene product which expression confers an increase in the threonine level in the host cell after expression compared to the wild type.
20. A method for the identification of a gene product conferring an increase in threonine production in a cell, comprising the following steps:
   (a) identifiying in a data bank nucleic acid molecules of an organism; which can contain a candidate gene encoding a gene product conferring an increase in the threonine amount or level in an organism or a part thereof after expression, and which are at least 20% homolog to the nucleic acid molecule of claim 6;
   (b) introducing the candidate nucleic acid molecules in host cells appropriate for producing threonine;
   (c) expressing the identified nucleic acid molecules in the host cells;
   (d) assaying the threonine level in the host cells; and
   (e) identifying the nucleic acid molecule and its gene product which expression confers an increase in the threonine level in the host cell after expression compared to the wild type.
21. A method for the production of an agricultural composition comprising the steps of the method of any one of claims 17 to 20 and formulating the compound identified in any one of claims 17 to 20 in a form acceptable for an application in agriculture.
22. A composition comprising the nucleic acid molecule of claim 6, the polypeptide of claim 14, the nucleic acid construct of claim 7, the vector of any one of claims 8 or 9, an antagonist or agonist identified according to claim 17, the compound of claim 18, the gene product of claim 19 or 20, the antibody of claim 15, and optionally an agricultural acceptable carrier.
23. Use of the nucleic acid molecule as claimed in claim 6 for the identification of a nucleic acid molecule conferring an increase of threonine after expression.
24. Use of the polypeptide of claim 14 or the nucleic acid construct claim 7 or the gene product identified according to the method of claim 19 or 20 for identifying compounds capable of conferring a modulation of threonine levels in an organism.
25. Food or feed composition comprising the nucleic acid molecule of claim 6, the polypeptide of claim 14, the nucleic acid construct of claim 7, the vector of claim 8 or 9, the antagonist or agonist identified according to claim 17, the antibody of claim 15, the plant or plant tissue of claim 16, the harvested material of claim 16, the host cell of claim 10 to 12 or the gene product identified according to the method of claim 19 or 20.

**[0554.0.0.1] Abstract: see [0554.0.0.0]**

### Process for the production of fine chemicals

**[0000.0.0.2]:** In a further embodiment, the present invention relates to a further process for the production of fine chemicals as defined below and corresponding embodiments as described herein as follows.

**[0001.0.0.2] to [0009.0.0.2]: see [0.0.01.0.0.0] to [0008.0.0.0]**

**[0009.0.2.2]** As described above, the essential amino acids are necessary for humans and many mammals, for example for livestock. Tryptophane (L-tryptophane) is one of the most reactive amino acids. At pH 4.0-6.0 tryptophane amino group reacts with aldehydes producing Schiff-bases. On the other hand if the amino group is blocked by acetylation, tryptophane reacts with aldehydes yielding carboline derivatives (carboline 1,2,3,4-tetrahydro-carboline-3-carboxylic acid). Tryptophane plays a unique role in defense against infection because of its relative scarcity compared to other amino acids. During infection, the body induces tryptophane-catabolizing enzymes which increase tryptophane's scarcity in an attempt to starve the infecting organisms [R. R. Brown, Y. Ozaki, S. P. Datta, et al., Implications of interferon-induced tryptophane catabolism in cancer, auto-immune diseases and AIDS. In: Kynurenine and Serotonin Pathways, R. Schwarcz, et al., (Eds.), Plenum Press, New York, 1991]. In most proteins, tryptophane is the least abundant essential amino acid, comprising approximately 1% of plant proteins and 1.5% of animal proteins. Although the minimum daily requirement for tryptophane is 160 mg for women and 250 mg for men, 500-700 mg are recommended to ensure high-quality protein intake. Actual tryptophane utilization is substantially higher. Men use approximately 3.5 grams of tryptophane to make one days's worth of protein [J. C. Peters, Tryptophane Nutrition and Metabolism: an Overview. In: Kynurenine and Serotonin Pathways, R. Schwarcz, et al., (Eds.), Plenum Press, New York, 1991]. The balance is obtained by hepatic recycling of tryptophane from used (catabolized) proteins.
Dietary tryptophane is well absorbed intestinally. About 10% of the tryptophane circulating in the bloodstream is free, and 90% is bound to the protein albumin. The tryptophane binding site on albumin also has affinity for free fatty acids (FFAs), so tryptophane is displaced when FFAs rise, as when fasting.
Although tryptophane is not usually the limiting amino acid in protein synthesis, tryptophane may become insufficient for the normal functioning of other tryptophane-dependent pathways. Numerous lines of research point to tryptophane's central role in regulation of feeding and other behaviors. Tryptophane is not only typically the least abundant amino acid in the liver's free amino acid pool, but liver tryptophane-tRNA levels fall faster during food deprivation than other indispensable amino acids [Q. R. Rogers, The nutritional and metabolic effects of amino acid imbalances. In: Protein Metabolism and Nutrition, D. J. A. Cole (Ed.), Butterworths, London, 1976]. Under fasting conditions, and possibly in wasting syndromes, tryptophane may become the rate-limiting amino acid for protein synthesis [Peters, 1991].

**[0010.0.0.2] and [0011.0.0.2]: see [0010.0.0.0] and [0011.0.0.0]**

**[0012.0.2.2]** It is an object of the present invention to develop an inexpensive process for the synthesis of tryptophane, preferably L-tryptophane. Tryptophane is together with methionine, lysine and threonine (depending on the organism) one of the amino acids which are most frequently limiting.

**[0013.0.0.2]: see [0013.0.0.0]**

**[0014.0.2.2]** Accordingly, in a first embodiment, the invention relates to a process for the production of a fine chemical, whereby the fine chemical is tryptophane, preferably L-tryptophane. Accordingly, in the present invention, the term "the fine chemical" as used herein relates to "tryptophane". Further, the term "the fine chemicals" as used herein also relates to fine chemicals comprising tryptophane.

**[0015.0.2.2]** In one embodiment, the term "the fine chemical" means tryptophane, preferably L-tryptophane. Throughout the specification the term "the fine chemical" means tryptophane, preferably L-tryptophane, its salts, ester or amids in free form or bound to proteins. In a preferred embodiment, the term "the fine chemical" means tryptophane, preferably L-tryptophane, in free form or its salts or bound to proteins.
In one embodiment, the term "the fine chemical" and the term "the respective fine chemical" mean at least one chemical compound with an activity of the above mentioned fine chemical.

**[0016.0.2.2]** Accordingly, the present invention relates to a process comprising
(a) increasing or generating the activity of one or more
   YER173W, YGR104C, b0186, b0161, b0486, b1318, b2270, b3074, b3983 and/or YHR189W - protein(s) or of a protein having the sequence of a polypeptide encoded by a nucleic acid molecule indicated in Table I, columns 5 or 7, lines 16 to 18 and/or lines 356 to 362
   in a non-human organism in one or more parts thereof and
(b) growing the organism under conditions which permit the production of the fine chemical, thus, tryptophane or fine chemicals comprising tryptophane, in said organism.
Accordingly, the present invention relates to a process for the production of a fine chemical comprising
(a) increasing or generating the activity of one or more proteins having the activity of a protein indicated in Table II, column 3, lines 16 to 18 and/or lines 356 to 362 or having the sequence of a polypeptide encoded by a nucleic acid molecule indicated in Table I, column 5 or 7, lines 16 to 18 and/or lines 356 to 362, in a non-human organism in one or more parts thereof and
(b) growing the organism under conditions which permit the production of the fine chemical, in particular tryptophane.

**[0017.0.0.2] and [0018.0.0.2]: see [0017.0.0.0] and [0018.0.0.0]**

**[0019.0.2.2]** Advantageously the process for the production of the fine chemical leads to an enhanced production of the fine chemical. The terms "enhanced" or "increase" mean at least a 10%, 20%, 30%, 40% or 50%, preferably at least 60%, 70%, 80%, 90% or 100%, more preferably 150%, 200%, 300%, 400% or 500% higher production of the fine chemical in comparison to the reference as defined below, e.g. that means in comparison to an organism without the aforementioned modification of the activity of a protein having the activity of a protein indicated in Table II, column 3, lines 16 to 18 and/or lines 356 to 362 or encoded by nucleic acid molecule indicated in Table I, columns 5 or 7, lines 16 to 18 and/or lines 356 to 362.

**[0020.0.2.2]** Surprisingly it was found, that the transgenic expression of at least one of the Saccaromyces cerevisiae protein(s) indicated in Table II, Column 3, lines 16 to 17 and/or 362 and/or at least one of the Escherichia coli K12 proteins indicated in Table II, Column 3, line 18 and/or lines 356 to 361 in Arabidopsis thaliana conferred an increase in the threonine (or fine chemical) content of the transformed plants.

**[0021.0.0.2]: see [0021.0.0.0]**

**[0022.0.2.2]** The sequence of YER173W from Saccharomyces cerevisiae has been published in Dietrich, Nature 387 (6632 Suppl), 78-81, 1997, and Goffeau, Science 274 (5287), 546-547, 1996, and its activity is beeing defined as an "Checkpoint protein, involved in the activation of the DNA damage and meiotic pachytene checkpoints; subunit of a clamp loader that loads Rad17p-Mec3p-Dc1p onto DNA, homolog of the human and S. pompe Rad17 protein; Rad24p". Accordingly, in one embodiment, the process of the present invention comprises the use of a "Checkpoint protein, involved in the activation of the DNA damage and meiotic pachytene checkpoints" or its "subunit of a clamp loader that loads Rad17p-Mec3p-Dc1 p onto DNA" or a Rad24p from Saccaromyces cerevisiae or a Rad17 protein or its homolog, e.g. as shown herein, for the production of the fine chemical, meaning of tryptophane, in particular for increasing the amount of tryptophane in free or bound form in an organism or a part thereof, as mentioned.
The sequence of YGR104C from Saccharomyces cerevisiae has been published in Thompson et al., Cell 73:1361-1375, 1993, and its activity is beeing defined as an "RNA polymerase II suppressor protein SRB5 - yeast". Accordingly, in one embodiment, the process of the present invention comprises the use of a "RNA polymerase II suppressor protein (SRB5 - yeast)" or its homolog, for the production of the fine chemical, meaning of tryptophane, in particular for increasing the amount of tryptophane in free or bound form in an organism or a part thereof, as mentioned.
The sequence of b0186 from Escherichia coli K12 has been published in Blattner et al., Science 277(5331), 1453-1474, 1997, and its activity is beeing defined as a lysine decarboxylase. Accordingly, in one embodiment, the process of the present invention comprises the use of a lysine decarboxylase from E. coli or its homolog, e.g. as shown herein, for the production of the fine chemical, meaning of tryptophane, in particular for increasing the amount of tryptophane, preferably tryptophane in free or bound form in an organism or a part thereof, as mentioned. In one embodiment, in the process of the present invention the activity of a lysine decarboxylase is increased or generated, e.g. from E. coli or a homolog thereof.
The sequence of b0161 from Escherichia coli K12 has been published in Blattner et al., Science 277(5331), 1453-1474, 1997, and its activity is being defined as a periplasmic serine protease (heat shock protein). Accordingly, in one embodiment, the process of the present invention comprises the use of a gene product with an activity of the helicobacter serine proteinase superfamily, preferably a protein with a periplasmic serine protease (heat shock protein) activity or its homolog, e.g. as shown herein, from Escherichia coli K12 or its homolog, e.g. as shown herein, for the production of the fine chemical, meaning of tryptophane in free or bound form in an organism or a part thereof, as mentioned.
The sequence of b0486 from Escherichia coli K12 has been published in Blattner, Science 277(5331), 1453-1474, 1997, and its activity is beeing defined as a amino-acid/amine transport protein (of the APC family). Accordingly, in one embodiment, the process of the present invention comprises the use of a gene product with the activity of the membrane protein ybaT superfamily, preferably a protein with a amino-acid/amine transport protein (of the APC family) activity from E. coli or its homolog, e.g. as shown herein, for the production of the fine chemical, meaning of tryptophane, in particular for increasing the amount of tryptophane in free or bound form in an organism or a part thereof, as mentioned.
The sequence of b1318 from Escherichia coli K12 has been published in Blattner, Science 277(5331), 1453-1474, 1997, and its activity is beeing defined as a sugar transport protein (of the ABC superfamily). Accordingly, in one embodiment, the process of the present invention comprises the use of a gene product with the activity of the inner membrane protein malK (with ATP-binding cassette homology) superfamily, preferably a protein with a sugar transport protein (of the ABC superfamily) activity from E. coli or its homolog, e.g. as shown herein, for the production of the fine chemical, meaning of tryptophane, in particular for increasing the amount of tryptophane in free or bound form in an organism or a part thereof, as mentioned.
The sequence of b2270 from Escherichia coli K12 has been published in Blattner et al., Science 277(5331), 1453-1474, 1997, and its activity has not been characterized yet. Accordingly, in one embodiment, the process of the present invention comprises the use of a protein b2270 from E. coli or its homolog, e.g. as shown herein, for the production of the fine chemical, meaning of tryptophane, in particular for increasing the amount of tryptophane in free or bound form in an organism or a part thereof, as mentioned.
The sequence of b3074 from Escherichia coli K12 has been published in Blattner et al., Science 277(5331), 1453-1474, 1997, and its activity is being defined as a tRNA synthetase. Accordingly, in one embodiment, the process of the present invention comprises the use of a gene product with the activity of the secretion chaperone CsaA and/or methionyl-tRNA synthetase (dimer-forming) superfamily, preferably a protein with a tRNA synthetase activity from E. coli or its homolog, e.g. as shown herein, for the production of the fine chemical, meaning of tryptophane, in particular for increasing the amount of tryptophane in free or bound form in an organism or a part thereof, as mentioned.
The sequence of b3983 from Escherichia coli K12 has been published in Blattner et al., Science 277(5331), 1453-1474, 1997, and its activity is being defined as a 50S ribosomal subunit protein L12. Accordingly, in one embodiment, the process of the present invention comprises the use of a gene product with the activity of the Escherichia coli ribosomal protein L11 superfamily, preferably a protein with a 50S ribosomal subunit protein L12 activity from E. coli or its homolog, e.g. as shown herein, for the production of the fine chemical, meaning of tryptophane, in particular for increasing the amount of tryptophane in free or bound form in an organism or a part thereof, as mentioned.
The sequence of YHR189W from Saccharomyces cerevisiae has been published in and Goffeau, Science 274 (5287), 546-547, 1996 and Johnston, Nature 387 (6632 Suppl), 87-90, 1997 , and its activity is beeing defined as a peptidyl-tRNA hydrolase. Accordingly, in one embodiment, the process of the present invention comprises the use of a gene product with the activity of the peptidyl-tRNA hydrolase superfamily, preferably a protein with a t peptidyl-tRNA hydrolase activity from Saccharomyces cerevisiae or its homolog, e.g. as shown herein, for the production of the fine chemical, meaning of tryptophane, in particular for increasing the amount of tryptophane in free or bound form in an organism or a part thereof, as mentioned.

**[0023.0.2.2]** Homologues (= homologs) of the present gene products can be derived from any organisms as long as the homologue confers the herein mentioned activity, in particular, confers an increase in the fine chemical amount or content. Further, in the present invention, the term "homologue" relates to the sequence of an organism having the highest sequence homology to the herein mentioned or listed sequences of all expressed sequences of said organism.
However, the person skilled in the art knows, that, preferably, the homologue has said the-fine-chemical-increasing activity and, if known, the same biological function or activity in the organism as at least one of the protein(s) indicated in Table I, Column 3, lines 16 to 18 and/or lines 356 to 362, e.g. having the sequence of a polypeptide encoded by a nucleic acid molecule comprising the sequence indicated in indicated in Table I, Column 5 or 7, lines 16 to 18 and/or lines 356 to 362.
In one embodiment, the homolog of any one of the polypeptides indicated in Table II, lines 16 to 17 and/or line 362 is a homolog having the same or a similar acitivity, in particular an increase of activity confers an increase in the content of the fine chemical in the organsims and being derived from an Eukaryot. In one embodiment, the homolog of a polypeptide indicated in Table II, column 3, line 18 and/or lines 356 to 361 is a homolog having the same or a similar acitivity, in particular an increase of activity confers an increase in the content of the fine chemical in the organisms or part thereof, and being derived from bacteria. In one embodiment, the homolog of a polypeptide indicated in Table II, column 3, lines 16 to 17 and/or line 362 is a homolog having the same or a similar acitivity, in particular an increase of activity confers an increase in the content of the fine chemical in an organisms or part thereof, and being derived from Fungi. In one embodiment, the homolog of a polypeptide indicated in Table II, column 3, line 18 and/or lines 356 to 361 is a homolog having the same or a similar acitivity, in particular an increase of activity confers an increase in the content of the fine chemical in the organsims or part thereof and being derived from Proteobacteria. In one embodiment, the homolog of a polypeptide indicated in Table II, column 3, lines 16 to 17 and/or line 362 is a homolog having the same or a similar acitivity, in particular an increase of activity confers an increase in the content of the fine chemical in the organsims or a part thereof and being derived from Ascomycota. In one embodiment, the homolog of a polypeptide indicated in Table II, column 3, line 18 and/or lines 356 to 361 is a homolog having the same or a similar acitivity, in particular an increase of activity confers an increase in the content of the fine chemical in the organisms or part thereof, and being derived from Gammaproteobacteria. In one embodiment, the homolog of a polypeptide polypeptide indicated in Table II, column 3, lines 16 to 17 and/or line 362 is a homolog having the same or a similar activity, in particular an increase of activity confers an increase in the content of the fine chemical in the organisms or part thereof, and being derived from Saccharomycotina. In one embodiment, the homolog of a polypeptide indicated in Table II, column 3, line 18 and/or lines 356 to 361 is a homolog having the same or a similar activity, in particular an increase of activity confers an increase in the content of the fine chemical in the organisms or part thereof, and being derived from Enterobacteriales. In one embodiment, the homolog of a polypeptide indicated in Table II, column 3, lines 16 to 17 and/or line 362 is a homolog having the same or a similar activity, in particular an increase of activity confers an increase in the content of the fine chemical in the organisms or a part thereof, and being derived from Saccharomycetes. In one embodiment, the homolog of the a polypeptide indicated in Table II, column 3, line 18 and/or lines 356 to 361 is a homolog having the same or a similar activity, in particular an increase of activity confers an increase in the content of the fine chemical in the organisms or part thereof, and being derived from Enterobacteriaceae. In one embodiment, the homolog of a polypeptide indicated in Table II, column 3, lines 16 to 17 and/or line 362 is a homolog having the same or a similar activity, in particular an increase of activity confers an increase in the content of the fine chemical in the organisms, and being derived from Saccharomycetales. In one embodiment, the homolog of a polypeptide indicated in Table II, column 3, line 18 and/or line 356 to 361 is a homolog having the same or a similar activity, in particular an increase of activity confers an increase in the content of the fine chemical in the organisms or a part thereof, and being derived from Escherichia. In one embodiment, the homolog of a polypeptide indicated in Table II, column 3, lines 16 to 17 and/or line 362 is a homolog having the same or a similar activity, in particular an increase of activity confers an increase in the content of the fine chemical in the organisms or a part thereof, and being derived from Saccharomycetaceae. In one embodiment, the homolog of a polypeptide indicated in Table II, column 3, line 16 to 17 and/or line 362 is a homolog having the same or a similar acitivity, in particular an increase of activity confers an increase in the content of the fine chemical in the organisms or a part thereof, and being derived from Saccharomycetes.

**[0023.1.2.2]** Homologs of the polypeptides indicated in Table II, column 3, lines 16 to 17 and/or line 362 may be the polypetides encoded by the nucleic acid molecules polypeptide indicated in Table I, column 7, lines 16 to 17 and/or line 362 or may be the polypeptides indicated in Table II, column 7, lines 16 to 17 and/or line 362 . Homologs of the polypeptides polypeptide indicated in Table II, column 3, line 18 and/or lines 356 to 361 may be the polypetides encoded by the nucleic acid molecules polypeptide indicated in Table I, column 7, lines 18 and/or lines 356 to 361 or may be the polypeptides indicated in Table II, column 7, lines 18 and/or lines 356 to 361.

**[0024.0.0.2]: see [0024.0.0.0]**

**[0025.0.2.2]** In accordance with the invention, a protein or polypeptide has the "activity of an protein of the invention", e.g. the activity of a protein indicated in Table II, column 3, lines 16 to 18 and/or lines 356 to 362 if its *de novo* activity, or its increased expression directly or indirectly leads to an increased tryptophane level in the organism or a part thereof, preferably in a cell of said organism. In a preferred embodiment, the protein or polypeptide has the above-mentioned additional activities of a protein indicated in Table II, column 3, lines 16 to 18 and/or lines 356 to 362. Throughout the specification the activity or preferably the biological activity of such a protein or polypeptide or an nucleic acid molecule or sequence encoding such protein or polypeptide is identical or similar if it still has the biological or enzymatic activity of any one of the proteins indicated in Table II, column 3, lines 16 to 18 and/or lines 356 to 362, i.e. or which has at least 10% of the original enzymatic activity, preferably 20%, particularly preferably 30%, most particularly preferably 40% in comparison to an any one of the proteins indicated in Table II, column 3, lines 16 to 17 and/or line 362 of Saccharomyces and/or any one of the proteins indicated in Table II, column 3, line 18 and/or lines 356 to 361 of E. coli K12.

**[0025.1.0.2]: see [0025.1.0.0]**

**[0025.2.0.2]: see [0025.2.0.0]**

**[0026.0.0.2] to [0033.0.0.2]: see [0026.0.0.0] to [0033.0.0.0]**

**[0034.0.2.2]** Preferably, the reference, control or wild type differs form the subject of the present invention only in the cellular activity of the polypeptide of the invention or the polypeptide used in the method of the invention, e.g. as result of an increase in the level of the nucleic acid molecule of the present invention or an increase of the specific activity of the polypeptide of the invention or the polypeptide used in the method of the invention. E.g., it differs by or in the expression level or activity of an protein having the activity of a protein as indicated in Table II, column 3, llines 16 to 18 and/or lines 356 to 362 or being encoded by a nucleic acid molecule indicated in Table I, column 5, lines 16 to 18 and/or lines 356 to 362 or its homologs, e.g. as indicated in Table I, column 7, lines 16 to 18 and/or lines 356 to 362, its biochemical or genetical causes and therefore shows the increased amount of the fine chemical.

**[0035.0.0.2] to [0044.0.0.2]: see [0035.0.0.0] to [0044.0.0.0]**

**[0045.0.2.2]** In case the activity of the Saccaromyces cerevisiae protein YER173W or its homologs, e.g. a checkpoint protein, involved in the activation of the DNA damage and meiotic pachytene checkpoints; subunit of a clamp loader that loads Rad17p-Mec3p-Ddc1 p onto DNA or Rad24p or its homologs, e.g. the human or S. pombe Rad17, e.g. as indicated in Table I, columns 5 or 7, line 16 is increased, preferably, an increase of the fine chemical between 27% and 178% or more is conferred.
In case the activity of the Saccaromyces cerevisiae protein YGR104C or its homologs, e.g. a RNA polymerase II suppressor protein (SRB5 - yeast) e.g. as indicated in Table I, columns 5 or 7, line 17 is increased, preferably, an increase of the fine chemical between 32% and 84% or more is conferred. (s.o.)
In case the activity of the Escherichia coli K12 protein b0186 or a lysine decarboxylase or its homologs, e.g. as indicated in Table I, columns 5 or 7, line 18 is increased, preferably, an increase of the fine chemical between 32% and 146% is conferred. S.o.
In case the activity of the Escherichia coli K12 protein b0161 or its homologs, e.g. the activity of a protein of the Helicobacter serine proteinase superfamily is increased, preferably, of a protein having a periplasmic serine protease (heat shock protein) activity, e.g. as indicated in Table I, columns 5 or 7, line 356, is increased conferring an increase of the respective fine chemical, preferably tryptophane between 93% and 278% or more.
In case the activity of the Escherichia coli K12 protein b0486 or its homologs, e.g. the activity of a protein of the membrane protein ybaT superfamily, preferably a protein with a amino-acid/amine transport protein (of the APC family) activity, e.g. as indicated in Table I, columns 5 or 7, line 357, is increased conferring an increase of the respective fine chemical, preferably tryptophane between 42% and 335% or more.
In case the activity of the Escherichia coli K12 protein b1318 or its homologs, e.g. the activity of the inner membrane protein malK (with ATP-binding cassette homology) superfamily, preferably a protein with a sugar transport protein (of the ABC superfamily) activity , e.g. as indicated in Table I, columns 5 or 7, line 358, is increased conferring an increase of the respective fine chemical, preferably tryptophane between 136% and 330% or more.
In case the activity of the Escherichia coli K12 protein b2270 or its homologs, e.g. the activity of a b2270 protein of E.coli, e.g. as indicated in Table I, columns 5 or 7, line 359, is increased conferring an increase of the respective fine chemical, preferably tryptophane between 33% and 79% or more.
In case the activity of the Escherichia coli K12 protein b3074 or its homologs, e.g. the activity of a protein of the secretion chaperone CsaA and/or methionyl-tRNA synthetase (dimer-forming) superfamily, preferably a protein with a tRNA synthetase activity, e.g. as indicated in Table I, columns 5 or 7, line 360, is increased conferring an increase of the respective fine chemical, preferably tryptophane between 33% and 79% or more.
In case the activity of the Escherichia coli K12 protein b3983 or its homologs, e.g. the activity of a Escherichia coli ribosomal protein L11 superfamily, preferably a protein with a 50S ribosomal subunit protein L12 activity, e.g. as indicated in Table I, columns 5 or 7, line 361, is increased conferring an increase of the respective fine chemical, preferably tryptophane between 33% and 387% or more.
In case the activity of the Saccaromyces cerevisiae protein YHR189W or its homologs, e.g. the activity of a peptidyl-tRNA hydrolase superfamily, preferably a protein with a peptidyl-tRNA hydrolase activity, e.g. as indicated in Table I, columns 5 or 7, line 362, is increased conferring an increase of the respective fine chemical, preferably tryptophane between 31 % and 66% or more.

**[0046.0.2.2]** In one embodiment, in case the activity of the Saccaromyces cerevisiae protein YER173W or its homologs, e.g. a checkpoint protein, involved in the activation of the DNA damage and meiotic pychtene checkpoints; subunit of a clamp loader that loads Rad 17p-Mec3p-Ddc1 p onto DNA or or Rad24p or its homologs, e.g. the human or S. pombe Rad17, e.g. as indicated in Table I, columns 5 or 7, line 16, is increased, preferably, an increase of the fine chemical and of proline is conferred.
In one embodiment, in case the activity of the Saccaromyces cerevisiae protein YGR104C or its homologs, e.g. a RNA polymerase II suppressor protein (SRB5 - yeast), e.g. as indicated in Table I, columns 5 or 7, line 17, is increased, preferably, an increase of the fine chemical and glutamic acid is conferred.
In one embodiment, in case the activity of the Escherichia coli K12 protein b0186or its homologs, e.g. a lysine decarboxylase or its homologs,e.g. as indicated in Table I, columns 5 or 7, line 18, is increased preferably, an increase of the fine chemical and of methionine is conferred.
In one embodiment, in case the activity of the Escherichia coli K12 protein b0161 or its homologs is increased, e.g. the activity of a protein of the Helicobacter serine proteinase superfamily is increased, preferably, of a protein having a periplasmic serine protease (heat shock protein) activity, e.g. as indicated in Table I, columns 5 or 7, line 356, is increased an increase of the respective fine chemical, preferably of tryptophane and of further amino acid(s) is conferred.
In one embodiment, in case the activity of the Escherichia coli K12 protein b0486 or its homologs is increased, e.g. the activity of a protein of the membrane protein ybaT superfamily, preferably a protein with a amino-acid/amine transport protein (of the APC family) activity, e.g. as indicated in Table I, columns 5 or 7, line 357, is increased an increase of the respective fine chemical, preferably of tryptophane and of further amino acid(s) is conferred.
In one embodiment, in case the activity of the Escherichia coli K12 protein b1318 or its homologs is increased, e.g. the activity of the inner membrane protein malK (with ATP-binding cassette homology) superfamily, preferably a protein with a sugar transport protein (of the ABC superfamily) activity , e.g. as indicated in Table I, columns 5 or 7, line 358, is increased an increase of the respective fine chemical, preferably of tryptophane and of further amino acid(s) is conferred.
In one embodiment, in case the activity of the Escherichia coli K12 protein b2270 or its homologs is increased, e.g. the activity of a transcriptional regulator, is increased, e.g. as indicated in Table I, columns 5 or 7, line 359, is increased an increase of the respective fine chemical, preferably of tryptophane and of further amino acid(s) is conferred.
In one embodiment, in case the activity of the Escherichia coli K12 protein b3074 or its homologs is increased, e.g. the activity of a tRNA synthetase or its homologs, e.g. transcriptional regulator, e.g. as indicated in Table I, columns 5 or 7, line 360, is increased an increase of the respective fine chemical, preferably of tryptophane and of further amino acid(s) is conferred.
In one embodiment, in case the activity of the Escherichia coli K12 protein b3983 or its homologs is increased, e.g. the activity of a Escherichia coli ribosomal protein L11 superfamily, preferably a protein with a 50S ribosomal subunit protein L12 activity, e.g. as indicated in Table I, columns 5 or 7, line 361, is increased an increase of the respective fine chemical, preferably of tryptophane and of further amino acid(s) is conferred.
In one embodiment, in case the activity of the Saccaromyces cerevisiae protein YHR189W or its homologs is increased, e.g. the activity of a peptidyl-tRNA hydrolase superfamily, preferably a protein witha peptidyl-tRNA hydrolase activity, e.g. as indicated in Table I, columns 5 or 7, line 362, is increased an increase of the respective fine chemical, preferably of tryptophane and of further amino acid(s) is conferred.

**[0047.0.0.2] and [0048.0.0.2]: see [0047.0.0.0] and [0048.0.0.0]**

**[0049.0.2.2]** A protein having an activity conferring an increase in the amount or level of the fine chemical preferably has the structure of the polypeptide described herein, in particular of a polypeptides comprising a consensus sequence as indicated in Table IV, columns 7, lines 16 to 18 and/or lines 356 to 362 or of a polypeptide as indicated in Table II, columns 5 or 7, lines 16 to 18 and/or lines 356 to 362 or the functional homologues thereof as described herein, or is encoded by the nucleic acid molecule characterized herein or the nucleic acid molecule according to the invention, for example by a nucleic acid molecule as indicaded in Table I, columns 5 or 7, lines 16 to 18 and/or lines 356 to 362 or its herein described functional homologues and has the herein mentioned activity.

**[0050.0.2.2]** For the purposes of the present invention, the term "tryptophane" and "L-tryptophane" also encompass the corresponding salts, such as, for example, tryptophane hydrochloride or tryptophane sulfate. Preferably the term tryptophane is intended to encompass the term L-tryptophane.

**[0051.0.0.2] and [0052.0.0.2]: see [0051.0.0.0] and [0052.0.0.0]**

**[0053.0.2.2]** In one embodiment, the process of the present invention comprises one or more of the following steps
(a) stabilizing a protein conferring the increased expression of a protein encoded by the nucleic acid molecule of the invention or of the polypeptide of the invention or the nucleic acid molecule or the polypeptide used in the method of the invention , e.g. of a polypeptide having an activity of a protein as indicated in Table II, column 3, lines 16 to 18 and/or lines 356 to 362 or its homologs activity, e.g. as indicated in Table II, columns 5 or 7, lines 16 to 18 and/or lines 356 to 362, having herein-mentioned the fine chemical-increasing activity;
(b) stabilizing a mRNA conferring the increased expression of a protein encoded by the nucleic acid molecule of the invention or the nucleic acid molecule used in the method of the invention , e.g. of a polypeptide having an activity of a protein as indicated in Table II, column 3, lines 16 to 18 and/or lines 356 to 362 or its homologs activity, e.g. as indicated in Table II, columns 5 or 7, lines 16 to 18 and/or lines 356 to 362, or of a mRNA encoding the polypeptide of the present invention having herein-mentioned tryptophane increasing activity;
(c) increasing the specific activity of a protein conferring the increased expression of a protein encoded by the nucleic acid molecule of the invention or of the polypeptide of the present invention or the polypeptide used in the method of the invention having herein-mentioned tryptophane increasing activity, e.g. of a polypeptide having an activity of a protein as indicated in Table II, column 3, lines 16 to 18 and/or lines 356 to 362 or its homologs activity, e.g. as indicated in Table II, columns 5 or 7, lines 16 to 18 and/or lines 356 to 362, or decreasing the inhibitiory regulation of the polypeptide of the invention or the polypeptide used in the method of the invention ;
(d) generating or increasing the expression of an endogenous or artificial transcription factor mediating the expression of a protein conferring the increased expression of a protein encoded by the nucleic acid molecule of the invention or the nucleic acid molecule used in the method of the invention or of the polypeptide of the invention or the polypeptide used in the method of the invention having herein-mentioned tryptophane increasing activity, e.g. of a polypeptide having an activity of a protein as indicated in Table II, column 3, lines 16 to 18 and/or lines 356 to 362, or its homologs activity, e.g. as indicated in Table II, columns 5 or 7, lines 16 to 18 and/or lines 356 to 362;
(e) stimulating activity of a protein conferring the increased expression of a protein encoded by the nucleic acid molecule of the present invention or a polypeptide of the present invention having herein-mentioned tryptophane increasing activity, e.g. of a polypeptide having an activity of a protein as indicated in Table II, column 3, lines 16 to 18 and/or lines 356 to 362, or its homologs activity, e.g. as indicated in Table II, columns 5 or 7, lines 16 to 18 and/or lines 356 to 362, by adding one or more exogenous inducing factors to the organismus or parts thereof;
(f) expressing a transgenic gene encoding a protein conferring the increased expression of a polypeptide encoded by the nucleic acid molecule of the present invention or a polypeptide of the present invention, having herein-mentioned tryptophane increasing activity, e.g. of a polypeptide having an activity of a protein as indicated in Table II, column 3, lines 16 to 18 and/or lines 356 to 362, or its homologs activity, e.g. as indicated in Table II, columns 5 or 7, lines 16 to 18 and/or lines 356 to 362;
(g) increasing the copy number of a gene conferring the increased expression of a nucleic acid molecule encoding a polypeptide encoded by the nucleic acid molecule of the invention or the nucleic acid molecule used in the method of the invention or the polypeptide of the invention or the polypeptide used in the method of the invention having herein-mentioned tryptophane increasing activity, e.g. of a polypeptide having an activity of a protein as indicated in Table II, column 3, lines 16 to 18 and/or lines 356 to 362, or its homologs activity, e.g. as indicated in Table II, columns 5 or 7, lines 16 to 18 and/or lines 356 to 362;
(h) Increasing the expression of the endogenous gene encoding the polypeptide of the invention or the polypeptide used in the method of the invention , e.g. a polypeptide having an activity of a protein as indicated in Table II, column 3, lines 16 to 18 and/or lines 356 to 362, or its homologs activity, e.g. as indicated in Table II, columns 5 or 7, lines 16 to 18 and/or lines 356 to 362, copy by adding positive expression or removing negative expression elements; e.g. homologous recombination can be used to either introduce positive regulatory elements like for plants the 35S enhancer into the promoter or to remove repressor elements form regulatory regions. Further gene conversion methods can be used to disrupt repressor elements or to enhance to activty of positive elements. Positive elements can be randomly introduced in plants by T-DNA or transposon mutagenesis and lines can be identified in which the positive elements have be integrated near to a gene of the invention, the expression of which is thereby enhanced;
(i) Modulating growth conditions of an organism in such a manner, that the expression or activity of the gene encoding the protein of the invention or the protein itself is enhanced for example microorganisms or plants can be grown for example under a higher temperature regime leading to an enhanced expression of heat shock proteins, which can lead an enhanced the fine chemical production; and/or
(j) selecting of organisms with expecially high activity of the proteins of the invention from natural or from mutagenized resources and breeding them into the target organisms, eg the elite crops.

**[0054.0.2.2]** Preferably, said mRNA is the nucleic acid molecule of the present invention and/or the protein conferring the increased expression of a protein encoded by the nucleic acid molecule of the present invention or the polypeptide having the herein mentioned activity is the polypeptide of the present invention, e.g. conferring the increase of tryptophane after increasing the expression or activity of the encoded polypeptide or having the activity of a polypeptide having an activity of a protein as indicated in Table II, columns 5 or 7, lines 16 to 18 and/or lines 356 to 362 or its homologs activity, e.g. as indicated in Table II, columns 5 or 7, lines 16 to 18 and/or lines 356 to 362.

**[0055.0.0.2] to [0071.0.0.2]: see [0055.0.0.0] to [0071.0.0.0]**

**[0072.0.2.2]** By influencing the metabolism thus, it is possible to produce, in the process according to the invention, further advantageous compounds. Examples of such compounds are, in addition to tryptophane chorismic acid, anthralinic acid, N-5'-Phosphoribosyl-anthranilate, 1-(o-Carboxyphenylamino)-1-deoxyribulose 5-phosphate., 1-(Indol-3-yl)-glycerin-3-phosphate, and 5-hydroxytrytophane.

**[0073.0.2.2]** Accordingly, in one embodiment, the process according to the invention relates to a process which comprises:
providing a non-human organism, preferably a microorganism, a non-human animal, a plant or animal cell, a plant or animal tissue or a plant;
   (a) increasing an activity of a polypeptide of the invention or the polypeptide used in the method of the invention or a homolog thereof, e.g. as indicated in Table II, columns 5 or 7, lines 16 to 18 and/or lines 356 to 362 , or of a polypeptide being encoded by the nucleic acid molecule of the present invention and described below, e.g. conferring an increase of the respective fine chemical in the organism, preferably in a microorganism, a non-human animal, a plant or animal cell, a plant or animal tissue or a plant,
   (b) growing the organism, preferably the microorganism, the non-human animal, the plant or animal cell, the plant or animal tissue or the plant under conditions which permit the production of the respective fine chemical in the organism, preferably the microorganism, the plant cell, the plant tissue or the plant; and
   (c) if desired, recovering, optionally isolating, the free and/or bound the fine chemical and, optionally further free and/or bound amino acids synthetized by the organism, the microorganism, the non-human animal, the plant or animal cell, the plant or animal tissue or the plant.

**[0074.0.0.2] to [0084.0.0.2]: see [0074.0.0.0] to [0084.0.0.0]**

**[0085.0.2.2]** With regard to the nucleic acid sequence as depicted a nucleic acid construct which contains a nucleic acid sequence mentioned herein or an organism (= transgenic organism) which is transformed with said nucleic acid sequence or said nucleic acid construct, "transgene" means all those constructs which have been brought about by genetic manipulation methods,preferably in which either
a) a nucleic acid sequence as indicated in Table I, columns 5 or 7, lines 16 to 18 and/or lines 356 to 362 a derivative thereof, or
b) a genetic regulatory element, for example a promoter, which is functionally linked to the nucleic acid sequence as indicated in Table I, columns 5 or 7, lines 16 to 18 and/or lines 356 to 362 , or a derivative thereof, or
c) (a) and (b)
is/are not present in its/their natural genetic environment or has/have been modified by means of genetic manipulation methods, it being possible for the modification to be, by way of example, a substitution, addition, deletion, inversion or insertion of one or more nucleotide. "Natural genetic environment" means the natural chromosomal locus in the organism of origin or the presence in a genomic library. In the case of a genomic library, the natural, genetic environment of the nucleic acid sequence is preferably at least partially still preserved. The environment flanks the nucleic acid sequence at least on one side and has a sequence length of at least 50 bp, preferably at least 500 bp, particularly preferably at least 1000 bp, very particularly preferably at least 5000 bp.

**[0086.0.0.2] and [0087.0.0.2]: see [0086.0.0.0] and [0087.0.0.0]**

**[0088.0.2.2]** In an advantageous embodiment of the invention, the organism takes the form of a plant whose amino acid content is modified advantageously owing to the nucleic acid molecule of the present invention expressed. This is important for plant breeders since, for example, the nutritional value of plants for monogastric animals is limited by a few essential amino acids such as lysine, threonine or methionine or tryptophane.

**[0088.1.0.2] to [0097.0.0.2]: see [0088.1.0.0] to [0097.0.0.0]**

**[0098.0.2.2]** In a preferred embodiment, the fine chemical (tryptophane) is produced in accordance with the invention and, if desired, is isolated. The production of further amino acids such as methionine, lysine and/or threonine mixtures of amino acid by the process according to the invention is advantageous.

**[0099.0.0.2] to [0102.0.0.2]: see [0099.0.0.0] to [0102.0.0.0]**

**[0103.0.2.2]** In a preferred embodiment, the present invention relates to a process for the production of the fine chemical comprising or generating in an organism or a part thereof the expression of at least one nucleic acid molecule comprising a nucleic acid molecule selected from the group consisting of:
(a) nucleic acid molecule encoding, preferably at least the mature form, of a polypeptide having a sequence as indicated in Table II, columns 5 or 7, lines 16 to 18 and/or lines 356 to 362 ;
(b) nucleic acid molecule comprising, preferably at least the mature form, of a nucleic acid molecule having a sequence as indicated in Table I, columns 5 or 7, lines 16 to 18 and/or lines 356 to 362 ;
(c) nucleic acid molecule whose sequence can be deduced from a polypeptide sequence encoded by a nucleic acid molecule of (a) or (b) as result of the degeneracy of the genetic code and conferring an increase in the amount of the fine chemical in an organism or a part thereof;
(d) nucleic acid molecule encoding a polypeptide which has at least 50% identity with the amino acid sequence of the polypeptide encoded by the nucleic acid molecule of (a) to (c) and conferring an increase in the amount of the fine chemical in an organism or a part thereof;
(e) nucleic acid molecule which hybridizes with a nucleic acid molecule of (a) to (c) under r stringent hybridisation conditions and conferring an increase in the amount of the fine chemical in an organism or a part thereof;
(f) nucleic acid molecule encoding a polypeptide, the polypeptide being derived by substituting, deleting and/or adding one or more amino acids of the amino acid sequence of the polypeptide encoded by the nucleic acid molecules (a) to (d), preferably to (a) to (c) and conferring an increase in the amount of the fine chemical in an organism or a part thereof;
(g) nucleic acid molecule encoding a fragment or an epitope of a polypeptide which is encoded by one of the nucleic acid molecules of (a) to (e), preferably to (a) to (c) and conferring an increase in the amount of the fine chemical in an organism or a part thereof;
(h) nucleic acid molecule comprising a nucleic acid molecule which is obtained by amplifying nucleic acid molecules from a cDNA library or a genomic library using the primers pairs having a sequence as indicated in Table III, columns 7, lines 16 to 18 and/or lines 356 to 362 and conferring an increase in the amount of the fine chemical in an organism or a part thereof;
(i) nucleic acid molecule encoding a polypeptide which is isolated, e.g. from an expression library, with the aid of monoclonal antibodies against a polypeptide encoded by one of the nucleic acid molecules of (a) to (h), preferably to (a) to (c), and conferring an increase in the amount of the fine chemical in an organism or a part thereof;
(j) nucleic acid molecule which encodes a polypeptide comprising the consensus sequence having a sequences as indicated in Table IV, column7, lines 16 to 18 and/or lines 356 to 362, and conferring an increase in the amount of the fine chemical in an organism or a part thereof;
(k) nucleic acid molecule comprising one or more of the nucleic acid molecule encoding the amino acid sequence of a polypeptide encoding a domain of a polypeptide indicated in Table II, columns 5 or 7,lines 16 to 18 and/or lines 356 to 362, and conferring an increase in the amount of the fine chemical in an organism or a part thereof; and
(l) nucleic acid molecule which is obtainable by screening a suitable library under stringent conditions with a probe comprising one of the sequences of the nucleic acid molecule of (a) to (k), preferably to (a) to (c), or with a fragment of at least 15 nt, preferably 20 nt, 30 nt, 50 nt, 100 nt, 200 nt or 500 nt of the nucleic acid molecule characterized in (a) to (k), preferably to (a) to (c), and conferring an increase in the amount of the fine chemical in an organism or a part thereof;
or which comprises a sequence which is complementary thereto.

**[00103.1.0.0.]** In one embodiment, the nucleic acid molecule used in the process of the invention distinguishes over the sequence indicated in Table IA, columns 5 or 7, lines 16 to 18 and/or lines 356 to 362, by one or more nucleotides. In one embodiment, the nucleic acid molecule used in the process of the invention does not consist of the sequence indicated in Table I A, columns 5 or 7, lines 16 to 18 and/or lines 356 to 362: In one embodiment, the nucleic acid molecule used in the process of the invention is less than 100%, 99,999%, 99,99%, 99,9% or 99% identical to a sequence indicated in Table I A, columns 5 or 7, lines 16 to 18 and/or lines 356 to 362. In another embodiment, the nucleic acid molecule does not encode a polypeptide of a sequence indicated in Table II A, columns 5 or 7, lines 16 to 18 and/or lines 356 to 362.

**[00103.2.0.0.]** In one embodiment, the nucleic acid molecule used in the process of the invention distinguishes over the sequence indicated in Table I B, columns 5 or 7, lines 16 to 18 and/or lines 356 to 362, by one or more nucleotides. In one embodiment, the nucleic acid molecule used in the process of the invention does not consist of the sequence shown in indicated in Table I B, columns 5 or 7, lines 16 to 18 and/or lines 356 to 362: In one embodiment, the nucleic acid molecule used in the process of the invention is less than 100%, 99,999%, 99,99%, 99,9% or 99% identical to a sequence indicated in Table I B, columns 5 or 7, lines 16 to 18 and/or lines 356 to 362. In another embodiment, the nucleic acid molecule does not encode a polypeptide of a sequence indicated in Table II B, columns 5 or 7, lines 16 to 18 and/or lines 356 to 362.

**[0104.0.2.2]** In one embodiment, the nucleic acid molecule of the invention or used in the process of the invention distinguishes over the sequence indicated in Table I, columns 5 or 7, lines 16 to 18 and/or lines 356 to 362, by one or more nucleotides. In one embodiment, the nucleic acid molecule of the present invention or used in the process of the invention does not consist of the sequence n indicated in Table I, columns 5 or 7, lines 16 to 18 and/or lines 356 to 362. : In one embodiment, the nucleic acid molecule of the present invention is less than 100%, 99,999%, 99,99%, 99,9% or 99% identical to a sequence indicated in Table I, columns 5 or 7, lines 16 to 18 and/or lines 356 to 362 In another embodiment, the nucleic acid molecule does not encode a polypeptide of a sequence indicated in Table I, columns 5 or 7, lines 16 to 18 and/or lines 356 to 362.

**[0105.0.0.2] to [0107.0.0.2]: see [0105.0.0.0] to [0107.0.0.0]**

**[0108.0.2.2]** Nucleic acid molecules with the sequence as indicated in Table I, columns 5 or 7, lines 16 to 18 and/or lines 356 to 362 nucleic acid molecules which are derived from a amino acid sequences as indicated in Table II, columns 5 or 7, lines 16 to 18 and/or lines 356 to 362 or from polypeptides comprising the consensus sequence as indicated in Table IV, column 7, lines 16 to 18 and/or lines 356 to 362 or their derivatives or homologues encoding polypeptides with the enzymatic or biological activity of a polypeptide as indicated in Table II, column 3, 5 or 7, lines 16 to 18 and/or lines 356 to 362 or e.g. conferring a increase of the fine chemical after increasing its expression or activity are advantageously increased in the process according to the invention.

**[0109.0.0.2]: see [0109.0.0.0]**

**[0110.0.2.2]** Nucleic acid molecules, which are advantageous for the process according to the invention and which encode polypeptides with an activity of a polypeptide of the invention or the polypeptide used in the method of the invention or used in the process of the invention , e.g. of a protein as indicated in Table II, column 5, lines 16 to 18 and/or lines 356 to 362 or being encoded by a nucleic acid molecule indicated in Table I, column 5, lines 16 to 18 and/or lines 356 to 362 or of its homologs, e.g. as indicated in Table II, column 7, lines 16 to 18 and/or lines 356 to 362can be determined from generally accessible databases.

**[0111.0.0.2]: see [0111.0.0.0]**

**[0112.0.2.2]** The nucleic acid molecules used in the process according to the invention take the form of isolated nucleic acid sequences, which encode polypeptides with an activity of a polypeptide as indicated in Table II, column 3, lines 16 to 18 and/or lines 356 to 362 or having the sequence of a polypeptide as indicated in Table II, columns 5 and 7, lines 16 to 18 and/or lines 356 to 362 and conferring an tryptophane increase.

**[0113.0.0.2] to [120.0.0.2]: see [0113.0.0.0] to [0120.0.0.0]**

**[0121.0.2.2]** However, it is also possible to use artificial sequences, which differ in one or more bases from the nucleic acid sequences found in organisms, or in one or more amino acid molecules from polypeptide sequences found in organisms, in particular from the polypeptide sequences indicated in Table II, columns 5 or 7, lines 16 to 18 and/or lines 356 to 362 or the functional homologues thereof as described herein, preferably conferring above-mentioned activity, i.e. conferring a increase of the fine chemical after increasing its activity.

**[0122.0.0.2] to [0127.0.0.2]: see [0122.0.0.0] to [0127.0.0.0]**

**[0128.0.2.2]** Synthetic oligonucleotide primers for the amplification, e.g. as the pairs indicated in Table III, column 7, lines 16 to 18 and/or lines 356 to 362 by means of polymerase chain reaction can be generated on the basis of a sequence as indicated in Table I, columns 5 or 7,lines 16 to 18 and/or lines 356 to 362 or the sequences derived from sequences as indicated in Table II, columns 5 or 7, lines 16 to 18 and/or lines 356 to 362.

**[0129.0.2.2]** Moreover, it is possible to identify conserved regions from various organisms by carrying out protein sequence alignments with the polypeptide used in the process of the invention, in particular with sequences of the polypeptide of the invention or the polypeptide used in the method of the invention, from which conserved regions, and in turn, degenerate primers can be derived. Conserved region for the polypeptide of the invention or the polypeptide used in the method of the invention are indicated in the alignments shown in the figures. Conserved regions are those, which show a very little variation in the amino acid in one particular position of several homologs from different origin. The consensus sequences indicated in Table IV, column 7, lines 16 to 18 and/or lines 356 to 362 are derived from said alignments.

**[0130.0.2.2] to [0138.0.0.2]: see [0131.0.0.0] to [0138.0.0.0]**

**[0139.0.2.2]** Polypeptides having above-mentioned activity, i.e. conferring a tryptophane increase, derived from other organisms, can be encoded by other DNA sequences which hybridize to a sequences indicated in Table I, columns 5 or 7, lines 16 to 18 and/or lines 356 to 362 under relaxed hybridization conditions and which code on expression for peptides having the tryptophane increasing activity.

**[0140.0.0.2] to [0146.0.0.2]: see [0140.0.0.0] to [0146.0.0.0]**

**[0147.0.2.2]** Further, the nucleic acid molecule of the invention comprises a nucleic acid molecule, which is a complement of one of the nucleotide sequences of above mentioned nucleic acid molecules or a portion thereof. A nucleic acid molecule which is complementary to one of the nucleotide sequences indicated in Table I, columns 5 or 7, lines 16 to 18 and/or lines 356 to 362 is one which is sufficiently complementary to one of said nucleotide sequences such that it can hybridize to one of said nucleotide sequences thereby forming a stable duplex. Preferably, the hybridisation is performed under stringent hybrization conditions. However, a complement of one of the herein disclosed sequences is preferably a sequence complement thereto according to the base pairing of nucleic acid molecules well known to the skilled person. For example, the bases A and G undergo base pairing with the bases T and U or C, resp. and visa versa. Modifications of the bases can influence the base-pairing partner.

**[0148.0.2.2]** The nucleic acid molecule of the invention comprises a nucleotide sequence which is at least about 30%, 35%, 40% or 45%, preferably at least about 50%, 55%, 60% or 65%, more preferably at least about 70%, 80%, or 90%, and even more preferably at least about 95%, 97%, 98%, 99% or more homologous to a nucleotide sequence indicated in Table I, columns 5 or 7, lines 16 to 18 and/or lines 356 to 362, preferably of Table I B, columns 5 or 7, lines 16 to 18 and/or lines 356 to 362 or a functional portion thereof and preferably has above mentioned activity, in particular has the-fine-chemical-increasing activity after increasing its activity or an activity of a product of a gene encoding said sequence or its homologs.

**[0149.0.2.2]** The nucleic acid molecule of the invention comprises a nucleotide sequence which hybridizes, preferably hybridizes under stringent conditions as defined herein, to one of the nucleotide sequences indicated in Table I, columns 5 or 7, lines 16 to 18 and/or lines 356 to 362, preferably of Table I B, columns 5 or 7, lines 16 to 18 and/or lines 356 to 362 or a portion thereof and encodes a protein having above-mentioned activity and as indicated in indicated in Table II, columns 5 or 7, lines 16 to 18 and/or lines 356 to 362, preferably of Table II B, columns 5 or 7, lines 16 to 18 and/or lines 356 to 362, e.g. conferring an increase of the fine chemical.

**[0149.1.2.2]** Optionally, the nucleotide sequence, which hybridises to one of the nucleotide sequences indicated in Table I, columns 5 or 7,lines 16 to 18 and/or lines 356 to 362, preferably of Table I B, columns 5 or 7, lines 16 to 18 and/or lines 356 to 362 has further one or more of the activities annotated or known for a protein as indicated in Table II, column 3, lines 16 to 18 and/or lines 356 to 362.

**[0150.0.2.2]** Moreover, the nucleic acid molecule of the invention or used in the process of the invention can comprise only a portion of the coding region of one of the sequences indicated in Table I, columns 5 or 7, lines 16 to 18 and/or lines 356 to 362, preferably of Table I B, columns 5 or 7, lines 16 to 18 and/or lines 356 to 362, for example a fragment which can be used as a probe or primer or a fragment encoding a biologically active portion of the polypeptide of the present invention or of a polypeptide used in the process of the present invention, i.e. having above-mentioned activity, e.g. conferring an increase of tryptophane if its activity is increased. The nucleotide sequences determined from the cloning of the present protein-according-to-the-invention-encoding gene allows for the generation of probes and primers designed for use in identifying and/or cloning its homologues in other cell types and organisms. The probe/primer typically comprises substantially purified oligonucleotide. The oligonucleotide typically comprises a region of nucleotide sequence that hybridizes under stringent conditions to at least about 12, 15 preferably about 20 or 25, more preferably about 40, 50 or 75 consecutive nucleotides of a sense strand of one of the sequences indicated in Table I, columns 5 or 7, lines 16 to 18 and/or lines 356 to 362, an anti-sense sequence of one of the sequences indicated in Table I, columns 5 or 7, lines 16 to 18 and/or lines 356 to 362, or naturally occurring mutants thereof. Primers based on a nucleotide sequence of the invention can be used in PCR reactions to clone homologues of the polypeptide of the invention or of the polypeptide used in the process of the invention, e.g. as the primers described in the examples of the present invention, e.g. as shown in the examples. A PCR with the primer pairs indicated in Table III, column 7, lines 16 to 18 and/or lines 356 to 362 will result in a fragment of a polynucleotide sequence as indicated in Table I, columns 5 or 7, lines 16 to 18 and/or lines 356 to 362.

**[0151.0.0.2]: [see 0151.0.0.0]**

**[0152.0.2.2]** The nucleic acid molecule of the invention encodes a polypeptide or portion thereof which includes an amino acid sequence which is sufficiently homologous to an amino acid sequence as indicated in Table II, columns 5 or 7, lines 16 to 18 and/or lines 356 to 362 such that the protein or portion thereof maintains the ability to participate in tryptophane production, in particular a tryptophane increasing activity as mentioned above or as described in the examples in plants or microorganisms is comprised.

**[0153.0.2.2]** As used herein, the language "sufficiently homologous" refers to proteins or portions thereof which have amino acid sequences which include a minimum number of identical or equivalent amino acid residues (e.g., an amino acid residue which has a similar side chain as an amino acid residue in one of the sequences of the polypeptide of the present invention) to an amino acid sequence as indicated in Table II, columns 5 or 7,lines 16 to 18 and/or lines 356 to 362 such that the protein or portion thereof is able to participate in the increase of tryptophane production. In one embodiment, a protein or portion thereof as indicated in Table II, columns 5 or 7, lines 16 to 18 and/or lines 356 to 362 has for example an activity of a polypeptide indicated in Table II, column 3, lines 16 to 18 and/or lines 356 to 362.

**[0154.0.2.2]** In one embodiment, the nucleic acid molecule of the present invention comprises a nucleic acid that encodes a portion of the protein of the present invention. The protein is at least about 30%, 35%, 40%, 45% or 50%, preferably at least about 55%, 60%, 65% or 70%, and more preferably at least about 75%, 80%, 85%, 90%, 91%, 92%, 93% or 94% and most preferably at least about 95%, 97%, 98%, 99% or more homologous to an entire amino acid sequence as indicated in Table II, columns 5 or 7, lines 16 to 18 and/or lines 356 to 362 and has above-mentioned activity, e.g. conferring preferably the increase of the fine chemical.

**[0155.0.0.2] and [0156.0.0.2]: see [0155.0.0.0] to [0156.0.0.0]**

**[0157.0.2.2]** The invention further relates to nucleic acid molecules that differ from one of a nucleotide sequences as indicated in Table I, columns 5 or 7, lines 16 to 18 and/or lines 356 to 362 (and portions thereof) due to degeneracy of the genetic code and thus encode a polypeptide of the present invention, in particular a polypeptide having above mentioned activity, e.g. conferring an increase in tryptophane in a organism, e.g. as that polypeptides comprising the consensus sequences as indicated in Table IV, columns 5 or 7, llines 16 to 18 and/or lines 356 to 362 or of the polypeptide as indicated in Table II, columns 5 or 7, lines 16 to 18 and/or lines 356 to 362 or their functional homologues. Advantageously, the nucleic acid molecule of the invention or the nucleic acid molecule used in the method of the invention comprises, or in an other embodiment has, a nucleotide sequence encoding a protein comprising, or in an other embodiment having, a consensus sequences as indicated in Table IV, columns 5 or 7, lines 16 to 18 and/or lines 356 to 362 or of the polypeptide as indicated in Table II, columns 5 or 7, lines 16 to 18 and/or lines 356 to 362 or the functional homologues. In a still further embodiment, the nucleic acid molecule of the invention or the nucleic acid molecule used in the method of the invention encodes a full length protein which is substantially homologous to an amino acid sequence comprising a consensus sequence as indicated in Table IV, column 7, lines 16 to 18 and/or lines 356 to 362, or of a polypeptide as indicated in Table II, columns 5 or 7, lines 16 to 18 and/or lines 356 to 362 or the functional homologues thereof. However, in a preferred embodiment, the nucleic acid molecule of the present invention does not consist of a sequence as indicated in Table I, columns 5 or 7, lines 16 to 18 and/or lines 356 to 362. Preferably the nucleic acid molecule of the invention is a functional homologue or identical to a nucleic acid molecule indicated in Table I B, columns 5 or 7,lines 16 to 18 and/or lines 356 to 362.

**[0158.0.0.2] to [0160.0.0.2]: see [0158.0.0.0] to [0160.0.0.0]**

**[0161.0.2.2]** Accordingly, in another embodiment, a nucleic acid molecule of the invention is at least 15, 20, 25 or 30 nucleotides in length. Preferably, it hybridizes under stringent conditions to a nucleic acid molecule comprising a nucleotide sequence of the nucleic acid molecule of the present invention or used in the process of the present invention, e.g. comprising a sequence as indicated in Table I, columns 5 or 7, lines 16 to 18 and/or lines 356 to 362. The nucleic acid molecule is preferably at least 20, 30, 50, 100, 250 or more nucleotides in length.

**[0162.0.0.2]: see [0162.0.0.0]**

**[0163.0.2.2]** Preferably, nucleic acid molecule of the invention that hybridizes under stringent conditions to a sequence as indicated in Table I, columns 5 or 7, lines 16 to 18 and/or lines 356 to 362 corresponds to a naturally-occurring nucleic acid molecule of the invention. As used herein, a "naturally-occurring" nucleic acid molecule refers to an RNA or DNA molecule having a nucleotide sequence that occurs in nature (e.g., encodes a natural protein). Preferably, the nucleic acid molecule encodes a natural protein having above-mentioned activity, e.g. conferring the fine chemical increase after increasing the expression or activity thereof or the activity of a protein of the invention or used in the process of the invention.

**[0164.0.0.2]: see [0164.0.0.0]**

**[0165.0.2.2]** For example, nucleotide substitutions leading to amino acid substitutions at "non-essential" amino acid residues can be made in a sequence of the nucleic acid molecule of the invention or used in the process of the invention, e.g. as indicated in Table I, columns 5 or 7,lines 16 to 18 and/or lines 356 to 362.

**[0166.0.0.2] and [0167.0.0.2]: see [0166.0.0.0] and [0167.0.0.0][0168.0.2.2]**
Accordingly, the invention relates to nucleic acid molecules encoding a polypeptide having above-mentioned activity, e.g. conferring an increase in the fine chemical in an organisms or parts thereof that contain changes in amino acid residues that are not essential for said activity. Such polypeptides differ in amino acid sequence from a sequence contained in as sequence as indicated in Table II, columns 5 or 7, lines 16 to 18 and/or lines 356 to 362, preferably of Table II B, column 7, lines 16 to 18 and/or lines 356 to 362 yet retain said activity described herein. The nucleic acid molecule can comprise a nucleotide sequence encoding a polypeptide, wherein the polypeptide comprises an amino acid sequence at least about 50% identical to an amino acid sequence as indicated in Table II, columns 5 or 7, lines 16 to 18 and/or lines 356 to 362, preferably of Table II B, column 7, lines 16 to 18 and/or lines 356 to 362 and is capable of participation in the increase of production of the fine chemical after increasing its activity, e.g. its expression. Preferably, the protein encoded by the nucleic acid molecule is at least about 60% identical to a sequence as indicated in Table II, columns 5 or 7, lines 16 to 18 and/or lines 356 to 362, preferably of Table II B, column 7, lines 16 to 18 and/or lines 356 to 362 more preferably at least about 70% identical to one of the sequences as indicated in Table II, columns 5 or 7, lines 16 to 18 and/or lines 356 to 362, preferably of Table II B, column 7, lines 16 to 18 and/or lines 356 to 362 even more preferably at least about 80%, 90% or 95% homologous to a sequence as indicated in Table II, columns 5 or 7, lines 16 to 18 and/or lines 356 to 362, preferably of Table II B, column 7, lines 16 to 18 and/or lines 356 to 362 and most preferably at least about 96%, 97%, 98%, or 99% identical to the sequence as indicated in Table II, columns 5 or 7, lines 16 to 18 and/or lines 356 to 362, preferably of Table II B, column 7, lines 16 to 18 and/or lines 356 to 362.

**[0169.0.0.2] to [0172.0.0.2]: see [0169.0.0.0] to [0172.0.0.0]**

**[0173.0.2.2]** For example a sequence which has a 80% homology with sequence SEQ ID No: 732 at the nucleic acid level is understood as meaning a sequence which, upon comparison with the sequence SEQ ID No: 732 by the above Gap program algorithm with the above parameter set, has a 80% homology.

**[0174.0.0.2]: see [0174.0.0.0]**

**[0175.0.2.2]** For example a sequence which has a 80% homology with sequence SEQ ID No: 733 at the protein level is understood as meaning a sequence which, upon comparison with the sequence SEQ ID No: 733 by the above program algorithm with the above parameter set, has a 80% homology.

**[0176.0.2.2]** Functional equivalents derived from one of the polypeptides as indicated in Table II, columns 5 or 7, lines 16 to 18 and/or lines 356 to 362 according to the invention by substitution, insertion or deletion have at least 30%, 35%, 40%, 45% or 50%, preferably at least 55%, 60%, 65% or 70% by preference at least 80%, especially preferably at least 85% or 90%, 91%, 92%, 93% or 94%, very especially preferably at least 95%, 97%, 98% or 99% homology with one of the polypeptides as indicated in Table II, columns 5 or 7, lines 16 to 18 and/or lines 356 to 362 according to the invention and are distinguished by essentially the same properties as a polypeptide as indicated in Table II, columns 5 or 7, lines 16 to 18 and/or lines 356 to 362.

**[0177.0.2.2]** Functional equivalents derived from a nucleic acid sequence as indicated in Table I, columns 5 or 7, lines 16 to 18 and/or lines 356 to 362, preferably of Table I B, column 7, lines 16 to 18 and/or lines 356 to 362 according to the invention by substitution, insertion or deletion have at least 30%, 35%, 40%, 45% or 50%, preferably at least 55%, 60%, 65% or 70% by preference at least 80%, especially preferably at least 85% or 90%, 91%, 92%, 93% or 94%, very especially preferably at least 95%, 97%, 98% or 99% homology with one of a polypeptides as indicated in Table II, columns 5 or 7, lines 16 to 18 and/or lines 356 to 362, preferably of Table I B, column 7, lines 16 to 18 and/or lines 356 to 362 according to the invention and encode polypeptides having essentially the same properties as a polypeptide as indicated in Table II, columns 5 or 7, llines 16 to 18 and/or lines 356 to 362, preferably of Table I B, column 7, lines 16 to 18 and/or lines 356 to 362.

**[0178.0.0.2]: see [0178.0.0.0]**

**[0179.0.2.2]** A nucleic acid molecule encoding an homologous to a protein sequence of as indicated in Table II, columns 5 or 7, lines 16 to 18 and/or lines 356 to 362, preferably of Table II B, column 7, lines 16 to 18 and/or lines 356 to 362 can be created by introducing one or more nucleotide substitutions, additions or deletions into a nucleotide sequence of the nucleic acid molecule of the present invention, in particular as indicated in Table I, columns 5 or 7, lines 16 to 18 and/or lines 356 to 362 such that one or more amino acid substitutions, additions or deletions are introduced into the encoded protein. Mutations can be introduced into the encoding sequences of a sequences as indicated in Table I, columns 5 or 7, llines 16 to 18 and/or lines 356 to 362 by standard techniques, such as site-directed mutagenesis and PCR-mediated mutagenesis.

**[0180.0.0.2] to [0183.0.0.2]: see [0180.0.0.0] to [0183.0.0.0]**

**[0184.0.2.2]** Homologues of the nucleic acid sequences used, with a sequence as indicated in Table I, columns 5 or 7, lines 16 to 18 and/or lines 356 to 362, preferably of Table I B, column 7, lines 16 to 18 and/or lines 356 to 362, or of the nucleic acid sequences derived from a sequences as indicated in Table II, columns 5 or 7,lines 16 to 18 and/or lines 356 to 362, preferably of Table I B, column 7, lines 16 to 18 and/or lines 356 to 362 comprise also allelic variants with at least approximately 30%, 35%, 40% or 45% homology, by preference at least approximately 50%, 60% or 70%, more preferably at least approximately 90%, 91%, 92%, 93%, 94% or 95% and even more preferably at least approximately 96%, 97%, 98%, 99% or more homology with one of the nucleotide sequences shown or the abovementioned derived nucleic acid sequences or their homologues, derivatives or analogues or parts of these. Allelic variants encompass in particular functional variants which can be obtained by deletion, insertion or substitution of nucleotides from the sequences shown, preferably from a sequence as indicated in Table I, columns 5 or 7, lines 16 to 18 and/or lines 356 to 362 or from the derived nucleic acid sequences, the intention being, however, that the enzyme activity or the biological activity of the resulting proteins synthesized is advantageously retained or increased.

**[0185.0.2.2]** In one embodiment of the present invention, the nucleic acid molecule of the invention or used in the process of the invention comprises one or more sequences as indicated in Table I, columns 5 or 7, lines 16 to 18 and/or lines 356 to 362, preferably of Table I B, column 7, lines 16 to 18 and/or lines 356 to 36 . In one embodiment it is preferred that the nucleic acid molecule comprises as little as possible other nucleotide sequences not shown in any one of sequences as indicated in Table I, columns 5 or 7, lines 16 to 18 and/or lines 356 to 362, preferably of Table I B, column 7, lines 16 to 18 and/or lines 356 to 362. In one embodiment, the nucleic acid molecule comprises less than 500, 400, 300, 200, 100, 90, 80, 70, 60, 50 or 40 further nucleotides. In a further embodiment, the nucleic acid molecule comprises less than 30, 20 or 10 further nucleotides. In one embodiment, a nucleic acid molecule use in the process of the invention is identical to a sequences as indicated in Table I, columns 5 or 7, lines 16 to 18 and/or lines 356 to 362, preferably of Table I B, column 7, lines 16 to 18 and/or lines 356 to 362.

**[0186.0.2.2]** Also preferred is that one or more nucleic acid molecule(s) used in the process of the invention encodes a polypeptide comprising a sequence as indicated in Table II, columns 5 or 7, lines 16 to 18 and/or lines 356 to 362, preferably of Table II B, column 7, lines 16 to 18 and/or lines 356 to 362. In one embodiment, the nucleic acid molecule encodes less than 150, 130, 100, 80, 60, 50, 40 or 30 further amino acids. In a further embodiment, the encoded polypeptide comprises less than 20, 15, 10, 9, 8, 7, 6 or 5 further amino acids. In one embodiment, the encoded polypeptide used in the process of the invention is identical to the sequences as indicated in Table II, columns 5 or 7, lines 16 to 18 and/or lines 356 to 362, preferably of Table II B, column 7, lines 16 to 18 and/or lines 356 to 362.

**[0187.0.2.2]** In one embodiment, the nucleic acid molecule of the invention or used in the process encodes a polypeptide comprising a sequence as indicated in Table II, columns 5 or 7, lines 16 to 18 and/or lines 356 to 362, preferably of Table II B, column 7, lines 16 to 18 and/or lines 356 to 362 and comprises less than 100 further nucleotides. In a further embodiment, said nucleic acid molecule comprises less than 30 further nucleotides. In one embodiment, the nucleic acid molecule used in the process is identical to a coding sequence encoding a sequences as indicated in Table II, columns 5 or 7, lines 16 to 18 and/or lines 356 to 362, preferably of Table II B, column 7, lines 16 to 18 and/or lines 356 to 362.

**[0188.0.2.2]** Polypeptides (= proteins), which still have the essential enzymatic activity of the polypeptide of the present invention conferring an increase of the fine chemical i.e. whose activity is essentially not reduced, are polypeptides with at least 10% or 20%, by preference 30% or 40%, especially preferably 50% or 60%, very especially preferably 80% or 90 or more of the wild type biological activity or enzyme activity, advantageously, the activity is essentially not reduced in comparison with the activity of a polypeptide as indicated in Table II, columns 5 or 7, lines 16 to 18 and/or lines 356 to 362, preferably compared to a sequence as indicated in Table II, column 3 and 5, lines 16 to 18 and/or lines 356 to 362, and expressed under identical conditions. In one embodiment, the polypeptide of the invention is a homolog consisting of or comprising the sequence as indicated in Table II B, columns 7, lines 16 to 18 and/or lines 356 to 362

**[0189.0.2.2]** Homologues of a sequences as indicated in Table I, columns 5 or 7, lines 16 to 18 and/or lines 356 to 362 or of a derived sequences as indicated in Table II, columns 5 or 7, lines 16 to 18 and/or lines 356 to 362 also mean truncated sequences, cDNA, single-stranded DNA or RNA of the coding and noncoding DNA sequence. Homologues of said sequences are also understood as meaning derivatives which comprise noncoding regions such as, for example, UTRs, terminators, enhancers or promoter variants. The promoters upstream of the nucleotide sequences stated can be modified by one or more nucleotide substitution(s), insertion(s) and/or deletion(s) without, however, interfering with the functionality or activity either of the promoters, the open reading frame (= ORF) or with the 3'-regulatory region such as terminators or other 3'regulatory regions, which are far away from the ORF. It is furthermore possible that the activity of the promoters is increased by modification of their sequence, or that they are replaced completely by more active promoters, even promoters from heterologous organisms. Appropriate promoters are known to the person skilled in the art and are mentioned herein below.

**[0190.0.0.2] to [0203.0.0.2]: see [0190.0.0.0] to [0203.0.0.0]**

**[0204.0.2.2]** Accordingly, in one embodiment, the invention relates to a nucleic acid molecule which comprises a nucleic acid molecule selected from the group consisting of:
a) nucleic acid molecule encoding, preferably at least the mature form, of a polypeptide as indicated in Table II, columns 5 or 7, lines 16 to 18 and/or lines 356 to 362, preferably of Table II B, column 7, lines 16 to 18 and/or lines 356 to 362 or a fragment thereof conferring an increase in the amount of the fine chemical in an organism or a part thereof
b) nucleic acid molecule comprising, preferably at least the mature form, of a nucleic acid molecule as indicated in Table II, columns 5 or 7, lines 16 to 18 and/or lines 356 to 362, preferably of Table II B, column 7, lines 16 to 18 and/or lines 356 to 362 or a fragment thereof conferring an increase in the amount of the fine chemical in an organism or a part thereof;
c) nucleic acid molecule whose sequence can be deduced from a polypeptide sequence encoded by a nucleic acid molecule of (a) or (b) as result of the degeneracy of the genetic code and conferring an increase in the amount of the fine chemical in an organism or a part thereof;
d) nucleic acid molecule encoding a polypeptide whose sequence has at least 50% identity with the amino acid sequence of the polypeptide encoded by the nucleic acid molecule of (a) to (c) and conferring an increase in the amount of the fine chemical in an organism or a part thereof;
e) nucleic acid molecule which hybridizes with a nucleic acid molecule of (a) to (c) under stringent hybridisation conditions and conferring an increase in the amount of the fine chemical in an organism or a part thereof;
f) nucleic acid molecule encoding a polypeptide, the polypeptide being derived by substituting, deleting and/or adding one or more amino acids of the amino acid sequence of the polypeptide encoded by the nucleic acid molecules (a) to (d), preferably to (a) to (c), and conferring an increase in the amount of the fine chemical in an organism or a part thereof;
g) nucleic acid molecule encoding a fragment or an epitope of a polypeptide which is encoded by one of the nucleic acid molecules of (a) to (e), preferably to (a) to (c) and conferring an increase in the amount of the fine chemical in an organism or a part thereof;
h) nucleic acid molecule comprising a nucleic acid molecule which is obtained by amplifying a cDNA library or a genomic library using primers or primer pairs as indicated in Table III, column 7, lines 16 to 18 and/or lines 356 to 362 and conferring an increase in the amount of the fine chemical in an organism or a part thereof;
i) nucleic acid molecule encoding a polypeptide which is isolated, e.g. from a expression library, with the aid of monoclonal antibodies against a polypeptide encoded by one of the nucleic acid molecules of (a) to (g), preferably to (a) to (c) and conferring an increase in the amount of the fine chemical in an organism or a part thereof;
j) nucleic acid molecule which encodes a polypeptide comprising the consensus sequence as indicated in Table IV, column 7, lines 16 to 18 and/or lines 356 to 362 and conferring an increase in the amount of the fine chemical in an organism or a part thereof;
k) nucleic acid molecule encoding the amino acid sequence of a polypeptide encoding a domain of a polypeptide as indicated in Table II, columns 5 or 7, lines 16 to 18 and/or lines 356 to 362, preferably of Table II B, column 7, lines 16 to 18 and/or lines 356 to 362 and conferring an increase in the amount of the fine chemical in an organism or a part thereof; and
l) nucleic acid molecule which is obtainable by screening a suitable nucleic acid library under stringent hybridization conditions with a probe comprising one of the sequences of the nucleic acid molecule of (a) to (k) or with a fragment of at least 15 nt, preferably 20 nt, 30 nt, 50 nt, 100 nt, 200 nt or 500 nt of the nucleic acid molecule characterized in (a) to (h) or of a nucleic acid molecule as indicated in Table I, columns 5 or 7, lines 16 to 18 and/or lines 356 to 362, preferably of Table II B, column 7, lines 16 to 18 and/or lines 356 to 362 or a nucleic acid molecule encoding, preferably at least the mature form of, the polypeptide as indicated in Table II, columns 5 or 7, lines 16 to 18 and/or lines 356 to 362, preferably of Table II B, column 7, lines 16 to 18 and/or lines 356 to 362 and conferring an increase in the amount of the fine chemical in an organism or a part thereof;
or which encompasses a sequence which is complementary thereto;
whereby, preferably, the nucleic acid molecule according to (a) to (I)
distinguishes over the sequence indicated in Table IA columns 5 or 7, lines 16 to 18 and/or lines 356 to 362, by one or more nucleotides. In one embodiment, the nucleic acid molecule does not consist of the sequence shown and in indicated in Table I A or I B, columns 5 or 7, lines 16 to 18 and/or lines 356 to 362: In one embodiment, the nucleic acid molecule is less than 100%, 99,999%, 99,99%, 99,9% or 99% identical to a sequence indicated in Table I A or I B, columns 5 or 7, lines 16 to 18 and/or lines 356 to 362. In another embodiment, the nucleic acid molecule does not encode a polypeptide of a sequence indicated in Table II A or II B, columns 5 or 7, lines 16 to 18 and/or lines 356 to 362. In an other embodiment, the nucleic acid molecule of the present invention is at least 30 %, 40 %, 50%, or 60% identical and less than 100%, 99,999%, 99,99%, 99,9% or 99% identical to a sequence indicated in Table I A or I B, columns 5 or 7, lines 16 to 18 and/or lines 356 to 362. In a further embodiment the nucleic acid molecule does not encode a polypeptide sequence as indicated in Table II A or II B, columns 5 or 7, lines 16 to 18 and/or lines 356 to 362. Accordingly, in one embodiment, the nucleic acid molecule of the differs at least in one or more residues from a nucleic acid molecule indicated in Table I A or I B, columns 5 or 7, lines 16 to 18 and/or lines 356 to 362. Accordingly, in one embodiment, the nucleic acid molecule of the present invention encodes a polypeptide, which differs at least in one or more amino acids from a polypeptide indicated in Table II A or I B, columns 5 or 7, lines 16 to 18 and/or lines 356 to 362. In another embodiment, a nucleic acid molecule indicated in Table I A or I B, columns 5 or 7, lines 16 to 18 and/or lines 356 to 362 does not encode a protein of a sequence indicated in Table II A or II B, columns 5 or 7, lines 16 to 18 and/or lines 356 to 362. Accordingly, in one embodiment, the protein encoded by a sequences of a nucleic acid according to (a) to (I) does not consist of a sequence as indicated in Table II A or II B, columns 5 or 7, lines 16 to 18 and/or lines 356 to 362. In a further embodiment, the protein of the present invention is at least 30 %, 40 %, 50%, or 60% identical to a protein sequence indicated in Table II A or II B, columns 5 or 7, lines 16 to 18 and/or lines 356 to 362 and less than 100%, preferably less than 99,999%, 99,99% or 99,9%, more preferably less than 99%, 985, 97%, 96% or 95% identical to a sequence as indicated in Table I A or II B, columns 5 or 7, lines 16 to 18 and/or lines 356 to 362.

**[0205.0.0.2] to [0226.0.0.2]: see [0205.0.0.0] to [0226.0.0.0]**

**[0227.0.2.2]** The abovementioned nucleic acid molecules can be cloned into the nucleic acid constructs or vectors according to the invention in combination together with further genes, or else different genes are introduced by transforming several nucleic acid constructs or vectors (including plasmids) into a host cell, advantageously into a plant cell or a microorgansim.
In addition to a sequence as indicated in Table I, columns 5 or 7, lines 16 to 18 and/or lines 356 to 362 or its derivatives, it is advantageous additionally to express and/or mutate further genes in the organisms. Especially advantageously, additionally at least one further gene of the amino acid biosynthetic pathway such as for L-lysine, L-threonine and/or L-methionine or L-tryptophane is expressed in the organisms such as plants or microorganisms. It is also possible that the regulation of the natural genes has been modified advantageously so that the gene and/or its gene product is no longer subject to the regulatory mechanisms which exist in the organisms. This leads to an increased synthesis of the amino acids desired since, for example, feedback regulations no longer exist to the same extent or not at all.
In addition it might be advantageously to combine a sequences as indicated in Table I, columns 5 or 7, lines 16 to 18 and/or lines 356 to 362 with genes which generally support or enhances to growth or yield of the target organismn, for example genes which lead to faster growth rate of microorganisms or genes which produces stress-, pathogen, or herbicide resistant plants.

**[0228.0.0.2] to [0230.0.0.2]: see [0228.0.0.0] to [0230.0.0.0]**

**[0231.0.0.2]** In a further advantageous embodiment of the process of the invention, the organisms used in the process are those in which simultaneously a tryptophane degrading protein is attenuated, in particular by reducing the rate of expression of the corresponding gene.

**[0232.0.0.2] to [0282.0.0.2]: see [0232.0.0.0] to [0282.0.0.0]**

**[0283.0.2.2]** Moreover, a native polypeptide conferring the increase of the fine chemical in an organism or part thereof can be isolated from cells (e.g., endothelial cells), for example using the antibody of the present invention as described below, in particular, an antibody against a protein as indicated in Table II, column 3, lines lines 16 to 18 and/or lines 356 to 362 E.g. an antibody against a polypeptide as indicated in Table II, columns 5 or 7, lines 16 to 18 and/or lines 356 to 362, which can be produced by standard techniques utilizing polypeptides comprising or consisting of above mentioned sequences, e.g. the polypeptide of the present invention or fragment thereof, Preferred are monoclonal antibodies, specifically binding to polypeptide as indicated in Table II, columns 5 or 7, lines 16 to 18 and/or lines 356 to 362.**[0284.0.0.2] see [0284.0.0.0]**

**[0285.0.2.2]** In one embodiment, the present invention relates to a polypeptide having a sequence as indicated in Table II, columns 5 or 7, lines 16 to 18 and/or lines 356 to 362 or as encoded by a nucleic acid molecule as indicated in Table I , columns 5 or 7, lines 16 to 18 and/or lines 356 to 362 or functional homologues thereof.

**[0286.0.2.2]** In one advantageous embodiment, in the method of the present invention the activity of a polypeptide is increased which comprises or consists of a consensus sequence as indicated in Table IV, column 7, lines 16 to 18 and/or lines 356 to 362 and in one another embodiment, the present invention relates to a polypeptide comprising or consisting of a consensus sequence as indicated in Table IV, column 7, lines 16 to 18 and/or lines 356 to 362 whereby 20 or less, preferably 15 or 10, preferably 9, 8, 7, or 6, more preferred 5 or 4, even more preferred 3, even more preferred 2, even more preferred 1, most preferred 0 of the amino acids positions indicated can be replaced by any amino acid or, in an further embodiment, can be replaced and/or absent. In one embodiment, the present invention relates to the method of the present invention comprising a polypeptide or to a polypeptide comprising more than one consensus sequences as indicated in Table IV, column 7, lines 16 to 18 and/or lines 356 to 362.
amino acidamino acid**[0287.0.0.2]to [290.0.0.2]: see [0287.0.0.0] to [0290.0.0.0]**

**[0291.0.2.2]** In one advantageous embodiment, the method of the present invention comprises the increasing of a polypeptide comprising or consisting of plant or microorganism specific consensus sequences.
Accordingly, in one embodiment, the present invention relates to a polypeptide comprising or consisting of plant or microorganism specific consensus sequences.
In one embodiment, said polypeptide of the invention distinguishes over a sequence as indicated in Table II A or IIB, columns 5 or 7, lines 16 to 18 and/or lines 356 to 362 by one or more amino acids. In one embodiment, polypeptide distinguishes form a sequence as indicated in Table II A or IIB, columns 5 or 7, lines 16 to 18 and/or lines 356 to 362 by more than 1, 2, 3, 4, 5, 6, 7, 8 or 9 amino acids, preferably by more than 10, 15, 20, 25 or 30 amino acids, even more preferred are more than 40, 50, or 60 amino acids and, preferably, the sequence of the polypeptide of the invention distinguishes from a sequence as indicated in Table II A or II B, columns 5 or 7, lines 16 to 18 and/or lines 356 to 362 by not more than 80% or 70% of the amino acids, preferably not more than 60% or 50%, more preferred not more than 40% or 30%, even more preferred not more than 20% or 10%. In an other embodiment, said polypeptide of the invention does not consist of a sequence as indicated in Table II A or II B, columns 5 or 7, lines 16 to 18 and/or lines 356 to 362.

**[0292.0.0.2]: see [0292.0.0.0]**

**[0293.0.2.2]** In one embodiment, the invention relates to polypeptide conferring an increase in the respective fine chemical in an organism or part being encoded by the nucleic acid molecule of the invention or by a nucleic acid molecule used in the process of the invention.
In one embodiment, the polypeptide of the invention has a sequence which distinguishes from a sequence as indicated in Table II A or II B, columns 5 or 7, lines 16 to 18 and/or lines 356 to 362 by one or more amino acids. In an other embodiment, said polypeptide of the invention does not consist of the sequence as indicated in Table II A or II B, columns 5 or 7, lines 16 to 18 and/or lines 356 to 362. In a further embodiment, said polypeptide of the present invention is less than 100%, 99,999%, 99,99%, 99,9% or 99% identical. In one embodiment, said polypeptide does not consist of the sequence encoded by a nucleic acid molecules as indicated in Table I A or IB, columns 5 or 7, lines 16 to 18 and/or lines 356 to 362.

**[0294.0.2.2]** In one embodiment, the present invention relates to a polypeptide having an activity of a protein as indicated in Table II, column 3, lines 16 to 18 and/or lines 356 to 362, which distinguishes over a sequence as indicated in Table II A or II B, columns 5 or 7, lines 16 to 18 and/or lines 356 to 362 by one or more amino acids, preferably by more than 5, 6, 7, 8 or 9 amino acids, preferably by more than 10, 15, 20, 25 or 30 amino acids, even more preferred are more than 40, 50, or 60 amino acids but even more preferred by less than 70% of the amino acids, more preferred by less than 50%, even more preferred my less than 30% or 25%, more preferred are 20% or 15%, even more preferred are less than 10%.

**[0295.0.0.2] to [0297.0.0.2]: see [0295.0.0.0] to [0297.0.0.0]**

**[0297.1.0.2]** Non-polypeptide of the invention-chemicals are e.g. polypeptides having not the activity and/or amino acid sequence of a polypeptide indicated in Table II, columns 3, 5 or 7, lines 16 to 18 and/or lines 356 to 362.

**[0298.0.2.2]** A polypeptide of the invention can participate in the process of the present invention. The polypeptide or a portion thereof comprises preferably an amino acid sequence which is sufficiently homologous to an amino acid sequence as indicated in Table II, columns 5 or 7, lines 16 to 18 and/or lines 356 to 362 such that the protein or portion thereof maintains the ability to confer the activity of the present invention. The portion of the protein is preferably a biologically active portion as described herein. Preferably, the polypeptide used in the process of the invention has an amino acid sequence identical to a sequence as indicated in Table II, columns 5 or 7, lines 16 to 18 and/or lines 356 to 362.

**[0299.0.2.2]** Further, the polypeptide can have an amino acid sequence which is encoded by a nucleotide sequence which hybridizes, preferably hybridizes under stringent conditions as described above, to a nucleotide sequence of the nucleic acid molecule of the present invention. Accordingly, the polypeptide has an amino acid sequence which is encoded by a nucleotide sequence that is at least about 35%, 40%, 45%, 50%, 55%, 60%, 65% or 70%, preferably at least about 75%, 80%, 85% or 90, and more preferably at least about 91%, 92%, 93%, 94% or 95%, and even more preferably at least about 96%, 97%, 98%, 99% or more homologous to one of the nucleotide sequence as indicated in Table I, columns 5 or 7, Ilines 16 to 18 and/or lines 356 to 362. The preferred polypeptide of the present invention preferably possesses at least one of the activities according to the invention and described herein. A preferred polypeptide of the present invention includes an amino acid sequence encoded by a nucleotide sequence which hybridizes, preferably hybridizes under stringent conditions, to a nucleotide sequence as indicated in Table I, columns 5 or 7, lines 16 to 18 and/or lines 356 to 362 or which is homologous thereto, as defined above.

**[0300.0.2.2]** Accordingly the polypeptide of the present invention can vary from a sequence as indicated in Table II, columns 5 or 7, lines 16 to 18 and/or lines 356 to 362 in amino acid sequence due to natural variation or mutagenesis, as described in detail herein. Accordingly, the polypeptide comprise an amino acid sequence which is at least about 35%, 40%, 45%, 50%, 55%, 60%, 65% or 70%, preferably at least about 75%, 80%, 85% or 90, and more preferably at least about 91 %, 92%, 93%, 94% or 95%, and most preferably at least about 96%, 97%, 98%, 99% or more homologous to an entire amino acid sequence of a sequence as indicated in Table IIA or IIB, columns 5 or 7, lines 16 to 18 and/or lines 356 to 362.

**[0301.0.0.2]: see [0301.0.0.0]**

**[0302.0.2.2]** Biologically active portions of an polypeptide of the present invention include peptides comprising amino acid sequences derived from the amino acid sequence of the polypeptide of the present invention or used in the process of the present invention, e.g., an amino acid sequence as indicated in Table II, columns 5 or 7, lines 16 to 18 and/or lines 356 to 362 or the amino acid sequence of a protein homologous thereto, which include fewer amino acids than a full length polypeptide of the present invention or used in the process of the present invention or the full length protein which is homologous to an polypeptide of the present invention or used in the process of the present invention depicted herein, and exhibit at least one activity of polypeptide of the present invention or used in the process of the present invention.

**[0303.0.0.2]: see [0303.0.0.0]**

**[0304.0.2.2]** Manipulation of the nucleic acid molecule of the invention may result in the production of a protein having essentially the activity of the polypeptides as indicated in Table II, column 3, lines 16 to 18 and/or lines 356 to 362 but having differences in the sequence from said wild-type protein. These proteins may be improved in efficiency or activity, may be present in greater numbers in the cell than is usual, or may be decreased in efficiency or activity in relation to the wild type protein.

**[0305.0.0.2] to [0306.0.0.2]: see [0305.0.0.0] to [0306.0.0.0]**

**[00306.1.2.2]** Preferrably, the compound is a composition comrising the tryptophane or a recovered tryptophane, in particular, the fine chemical, free or in protein-bound form.

**[0307.0.0.2]: to [0308.0.0.2]: see [0305.0.0.0] to [03086.0.0.0]**

**[0309.0.2.2]** In one embodiment, a reference to a "protein (= polypeptide)" of the invention or as indicated in Table II, columns 5 or 7, lines 16 to 18 and/or lines 356 to 362 refers to a polypeptide having an amino acid sequence corresponding to the polypeptide of the invention or used in the process of the invention , whereas a "non-polypeptide of the invention" or "other polypeptide"-not being indicated in Table II, columns 5 or 7, lines 16 to 18 and/or lines 356 to 362 refers to a polypeptide having an amino acid sequence corresponding to a protein which is not substantially homologous a polypeptide of the invention, preferably which is not substantially homologous to a as indicated in Table II, columns 5 or 7, lines 16 to 18 and/or lines 356 to 362 e.g., a protein which does not confer the activity described herein or annotated or known for as indicated in Table II, column 3, lines 16 to 18 and/or lines 356 to 362 and which is derived from the same or a different organism. In one embodiment a "non-polypeptide of the invention" or "other polypeptide" not being indicate in Table II, columns 5 or 7, lines 16 to 18 and/or lines 356 to 362 does not confer an increase of the fine chemical in an organism or part thereof.

**[0310.0.0.2] to [334.0.0.2]: see [0310.0.0.0] to [0334.0.0.0]**

**[0335.0.2.2]** The dsRNAi method has proved to be particularly effective and advantageous for reducing the expression of a nucleic acid sequences as indicated in Table II, columns 5 or 7, lines 16 to 18 and/or lines 356 to 362 and/or homologs thereof. As described inter alia in WO 99/32619, dsRNAi approaches are clearly superior to traditional antisense approaches. The invention therefore furthermore relates to double-stranded RNA molecules (dsRNA molecules) which, when introduced into an organism, advantageously into a plant (or a cell, tissue, organ or seed derived therefrom), bring about altered metabolic activity by the reduction in the expression of a nucleic acid sequences as indicated in Table I, columns 5 or 7, lines 16 to 18 and/or lines 356 to 362 and/or homologs thereof. In a double-stranded RNA molecule for reducing the expression of an protein encoded by a nucleic acid sequence of one of the sequences as indicated in Table I, columns 5 or 7, lines 16 to 18 and/or lines 356 to 362 and/or homologs thereof, one of the two RNA strands is essentially identical to at least part of a nucleic acid sequence, and the respective other RNA strand is essentially identical to at least part of the complementary strand of a nucleic acid sequence.

**[0336.0.0.2] to [0342.0.0.2]: see [0336.0.0.0] to [0342.0.0.0]**

**[0343.0.2.2]** As has already been described, 100 % sequence identity between the dsRNA and a gene transcript of a nucleic acid sequence as indicated in Table I, columns 5 or 7, lines 16 to 18 and/or lines 356 to 362 or it's homolog is not necessarily required in order to bring about effective reduction in the expression. The advantage is, accordingly, that the method is tolerant with regard to sequence deviations as may be present as a consequence of genetic mutations, polymorphisms or evolutionary divergences. Thus, for example, using the dsRNA, which has been generated starting from a sequence as indicated in Table I, columns 5 or 7, lines 16 to 18 and/or lines 356 to 362 or homologs thereof of the one organism, may be used to suppress the corresponding expression in another organism.

**[0344.0.0.2] to [0361.0.0.2]: see [0344.0.0.0] to [0361.0.0.0]**

**[0362.0.2.2]** Accordingly the present invention relates to any cell transgenic for any nucleic acid characterized as part of the invention, e.g. conferring the increase of the fine chemical in a cell or an organism or a part thereof, e.g. the nucleic acid molecule of the invention or the nucleic acid molecule used in the method of the invention, the nucleic acid construct of the invention, the antisense molecule of the invention, the vector of the invention or a nucleic acid molecule encoding the polypeptide of the invention, e.g. the polypeptide as indicated in Table II, columns 5 or 7, lines 16 to 18 and/or lines 356 to 362. Due to the above mentioned activity the fine chemical content in a cell or an organism is increased. For example, due to modulation or manipulation, the cellular activity of the polypeptide of the invention or the polypeptide used in the method of the invention or nucleic acid molecule of the invention or the nucleic acid molecule used in the method of the invention is increased, e.g. due to an increased expression or specific activity of the subject matters of the invention in a cell or an organism or a part thereof. In one embodiment transgenic for a polypeptide having an activity of a polypeptide as indicated in Table II, columns 5 or 7, lines 16 to 18 and/or lines 356 to 362 means herein that due to modulation or manipulation of the genome, an activity as annotated for a polypeptide as indicated in Table II, columns 3, lines 16 to 18 and/or lines 356 to 362, e.g. having a sequence as indicated in Table II, columns 5 or 7, lines 16 to 18 and/or lines 356 to 362 is increased in a cell or an organism or a part thereof. Examples are described above in context with the process of the invention.

**[0363.0.0.2] to [0382.0.0.2]: see [0365.0.0.0] to [0382.0.0.0]**

**[0383.0.2.2]** For preparing aromatic compound-containing fine chemicals, in particular the fine chemical, it is possible to use as aromat source organic aromatic-containing compounds such as, for example, benzene, naphthaline, indole, pyrrole, furen, oxazole, imidazole, thiophene, pyrridin, pyrrimidine or else organic aromatic compounds such as benzoic acid and chorismic, shikimic, aminobenzoic, kynurenic acids or pyridoxidal.

**[0384.0.0.2]: see [0384.0.0.0]**

**[0385.0.2.2]** The fermentation broths obtained in this way, containing in particular L-tryptophane, L-methionine, L-threonine and/or L-lysine, normally have a dry matter content of from 7.5 to 25% by weight. Sugar-limited fermentation is additionally advantageous, at least at the end, but especially over at least 30% of the fermentation time. This means that the concentration of utilizable sugar in the fermentation medium is kept at, or reduced to, 0 to 3 g/l during this time. The fermentation broth is then processed further. Depending on requirements, the biomass can be removed entirely or partly by separation methods, such as, for example, centrifugation, filtration, decantation or a combination of these methods, from the fermentation broth or left completely in it. The fermentation broth can then be thickened or concentrated by known methods, such as, for example, with the aid of a rotary evaporator, thin-film evaporator, falling film evaporator, by reverse osmosis or by nanofiltration. This concentrated fermentation broth can then be worked up by freeze-drying, spray drying, spray granulation or by other processes.

**[0386.0.0.2] to [392.0.0.2]: see [0386.0.0.0] to [0392.0.0.0]**

**[0393.0.2.2]** In one embodiment, the present invention relates to a method for the identification of a gene product conferring an increase in the fine chemical production in a cell, comprising the following steps:
(a) contacting,̅ e.g. hybridising, the nucleic acid molecules of a sample, e.g. cells, tissues, plants or microorganisms or a nucleic acid library , which can contain a candidate gene encoding a gene product conferring an increase in the fine chemical after expression, with the nucleic acid molecule of the present invention;
(b) identifying the nucleic acid molecules, which hybridize under relaxed stringent conditions with the nucleic acid molecule of the present invention in particular to the nucleic acid molecule sequence as indicated in Table I, columns 5 or 7, lines 16 to 18 and/or lines 356 to 362, preferably of Table I B, column 7, lines 16 to 18 and/or lines 356 to 362 and, optionally, isolating the full length cDNA clone or complete genomic clone;
(c) introducing the candidate nucleic acid molecules in host cells, preferably in a plant cell or a microorganism, appropriate for producing the fine chemical;
(d) expressing the identified nucleic acid molecules in the host cells;
(e) assaying the fine chemical level in the host cells; and
(f) identifying the nucleic acid molecule and its gene product which expression confers an increase in the fine chemical level in the host cell after expression compared to the wild type.

**[0394.0.0.2] to [0399.0.0.2]: see [0394.0.0.0] to [0399.0.0.0]**

**[0399.1.2.2]** One can think to screen for increased production of the fine chemical by for example searching for a resistance to a drug blocking the synthesis of the fine chemical and looking whether this effect is dependent on the activity or expression of a polypeptide as indicated in Table II, columns 5 or 7, lines 16 to 18 and/or lines 356 to 362 or a homolog thereof, e.g. comparing the phenotype of nearly identical organisms with low and high activity of a protein as indicated in Table II, columns 5 or 7, lines 16 to 18 and/or lines 356 to 362 after incubation with the drug.

**[0400.0.0.2] to [0416.0.0.2]: see [0400.0.0.0] to [0416.0.0.0]**

**[0417.0.2.2]** The nucleic acid molecule of the invention, the vector of the invention or the nucleic acid construct of the invention may also be useful for the production of organisms resistant to inhibitors of the amino acid production biosynthesis pathways. In particular, the overexpression of the polypeptide of the present invention may protect plants against herbicides, which block the amino acid, in particular the fine chemical, synthesis in said plant. Examples of herbicides blocking the amino acid synthesis in plants are for example sulfonylurea and imidazolinone herbicides which catalyze the first step in branched-chain amino acid biosynthesis.

**[0418.0.0.2] to [0423.0.0.2]: see [0418.0.0.0] to [0423.0.0.0][0424.0.2.2]**
Accordingly, the nucleic acid of the invention, the polypeptide of the invention, the nucleic acid construct of the invention, the organisms, the host cell, the microorgansims, the plant, plant tissue, plant cell, or the part thereof of the invention, the vector of the invention, the agonist identified with the method of the invention, the nucleic acid molecule identified with the method of the present invention, can be used for the production of the fine chemical or of the fine chemical and one or more other amino acids, in particular methionine, threonine, alanine, glutamine, glutamic acid, valine, asparagine, phenylalanine, leucine, proline , Tryptophan tyrosine, , isoleucine and arginine.
Accordingly, the nucleic acid of the invention, or the nucleic acid molecule identified with the method of the present invention or the complement sequences thereof, the polypeptide of the invention, the nucleic acid construct of the invention, the organisms, the host cell, the microorganisms, the plant, plant tissue, plant cell, or the part thereof of the invention, the vector of the invention, the antagonist identified with the method of the invention, the antibody of the present invention, the antisense molecule of the present invention, can be used for the reduction of the fine chemical in a organism or part thereof, e.g. in a cell.

**[0425.0.0.2] to [0430.0.0.2]: see [0425.0.0.0] to [0430.0.0.0]**

**[0431.0.2.2]** Example 1: Cloning SEQ ID No: 732 in Escherichia coli

**[0432.0.0.0] to [0460.0.0.2]: see [0433.0.0.0] to [0460.0.0.0]**

**[0461.0.2.2]** Example 10: Cloning SEQ ID NO: 732 for the expression in plants

**[0462.0.0.2] see [0462.0.0.0]**

**[0463.0.2.2]** SEQ ID NO: 732 is amplified by PCR as described in the protocol of the Pfu Turbo or DNA Herculase polymerase (Stratagene).

**[0464.0.0.2] to [0466.0.0.2]: see [0464.0.0.0] to [0466.0.0.0]**

**[0467.0.2.2]** The following primer sequences were selected for the gene SEQ ID No: 732:
i) forward primer (SEQ ID No: 734)
   ATGGATAGTACGAATTTGAACAAACG
ii) reverse primer (SEQ ID No: 735)
   TTAGAGTATTTCCAGATCTGAATCTG

**[0468.0.0.2] to [0479.0.0.2]: see [0468.0.0.0] to [0479.0.0.0]**

**[0480.0.2.2]** Example 11: Generation of transgenic plants which express SEQ ID No: 732

**[0481.0.0.2] to [0513.0.0.2]: see [0481.0.0.0] to [0513.0.0.0]**

**[0514.0.2.2]** As an alternative, the amino acids can be detected advantageously via HPLC separation in ethanolic extract as described by Geigenberger et al. (Plant Cell & Environ, 19, 1996: 43-55).
The results of the different plant analyses can be seen from the table which follows:

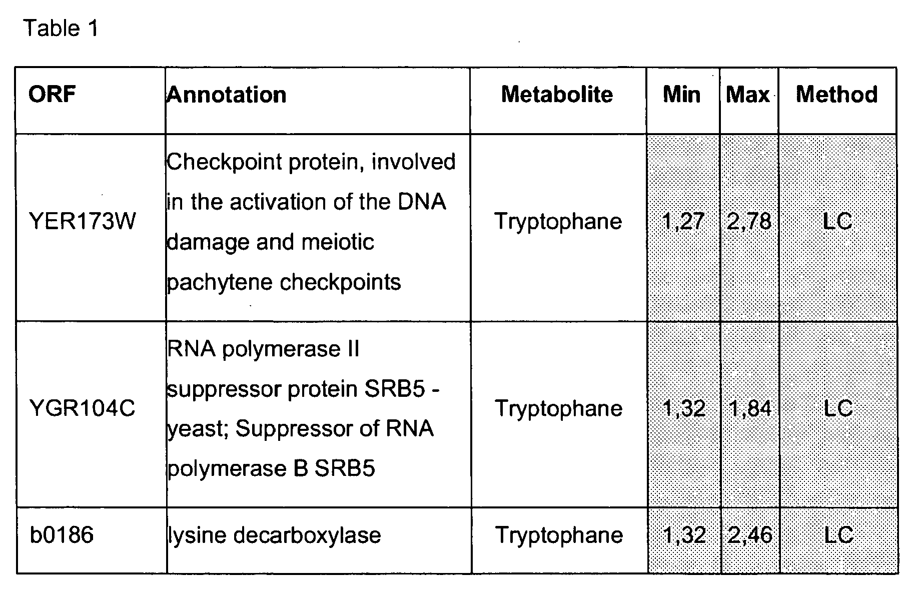

**Table 1b**

| **ORF** | **MetChemID** | **Metabolite** | **Method** | **Min** | **Max** |
|---|---|---|---|---|---|
| b0161 | 10000035 | Tryptophane | LC | 1.93 | 3.78 |
| b0486 | 10000035 | Tryptophane | LC | 1.42 | 4.35 |
| b1318 | 10000035 | Tryptophane | lC | 2.36 | 4.30 |
| b2270 | 10000035 | Tryptophane | LC | 1.33 | 1.79 |
| b3074 | 10000035 | Tryptophane | LC | 1.33 | 1.79 |
| b3983 | 10000035 / 30000016 | Tryptophane | LC + GC | 1.33 | 4.87 |
| YHR189W | 10000035 | Tryptophane | LC | 1.31 | 1.66 |

**[0515.0.0.2] to [0552.2.0.2]: see [0515.0.0.0] to [0552.2.0.0]**

**[0553.0.2.2]**
1. A process for the production of tryptophane, which comprises
   (a) increasing or generating the activity of a protein as indicated in Table II, columns 5 or 7, lines 16 to 18 and/or lines 356 to 362 or a functional equivalent thereof in a non-human organism, or in one or more parts thereof; and
   (b) growing the organism under conditions which permit the production of tryptophane in said organism.
2. A process for the production of tryptophane, comprising the increasing or generating in an organism or a part thereof the expression of at least one nucleic acid molecule comprising a nucleic acid molecule selected from the group consisting of:
   (a) nucleic acid molecule encoding of a polypeptide as indicated in Table II, columns 5 or 7, lines 16 to 18 and/or lines 356 to 362 or a fragment thereof, which confers an increase in the amount of tryptophane in an organism or a part thereof;
   (b) nucleic acid molecule comprising of the nucleic acid molecule as indicated in Table I, columns 5 or 7, lines 16 to 18 and/or lines 356 to 362;
   (c) nucleic acid molecule whose sequence can be deduced from a polypeptide sequence encoded by a nucleic acid molecule of (a) or (b) as a result of the degeneracy of the genetic code and conferring an increase in the amount of tryptophane in an organism or a part thereof;
   (d) nucleic acid molecule which encodes a polypeptide which has at least 50% identity with the amino acid sequence of the polypeptide encoded by the nucleic acid molecule of (a) to (c) and conferring an increase in the amount of tryptophane in an organism or a part thereof;
   (e) nucleic acid molecule which hybidizes with a nucleic acid molecule of (a) to (c) under stringent hybridisation conditions and conferring an increase in the amount of tryptophane in an organism or a part thereof;
   (f) nucleic acid molecule which encompasses a nucleic acid molecule which is obtained by amplifying nucleic acid molecules from a cDNA library or a genomic library using the primers or primer pairs as indicated in Table III, column 7, lines 16 to 18 and/or lines 356 to 362 and conferring an increase in the amount of the fine chemical in an organism or a part thereof;
   (g) nucleic acid molecule encoding a polypeptide which is isolated with the aid of monoclonal antibodies against a polypeptide encoded by one of the nucleic acid molecules of (a) to (f) and conferring an increase in the amount of tryptophane in an organism or a part thereof;
   (h) nucleic acid molecule encoding a polypeptide comprising a consensus sequence as indicated in Table IV, column 7, lines 16 to 18 and/or lines 356 to 362 and conferring an increase in the amount of the fine chemical in an organism or a part thereof;
   (i) nucleic acid molecule which is obtainable by screening a suitable nucleic acid library under stringent hybridization conditions with a probe comprising one of the sequences of the nucleic acid molecule of (a) to (k) or with a fragment thereof having at least 15 nt, preferably 20 nt, 30 nt, 50 nt, 100 nt, 200 nt or 500 nt of the nucleic acid molecule characterized in (a) to (k) and conferring an increase in the amount of the fine chemical in an organism or a part thereof.
   or comprising a sequence which is complementary thereto.
3. The process of claim 1 or 2, comprising recovering of the free or bound tryptophane.
4. The process of any one of claim 1 to 3, comprising the following steps:
   (a) selecting an organism or a part thereof expressing a polypeptide encoded by the nucleic acid molecule characterized in claim 2;
   (b) mutagenizing the selected organism or the part thereof;
   (c) comparing the activity or the expression level of said polypeptide in the mutagenized organism or the part thereof with the activity or the expression of said polypeptide of the selected organisms or the part thereof;
   (d) selecting the mutated organisms or parts thereof, which comprise an increased activity or expression level of said polypeptide compared to the selected organism or the part thereof;
   (e) optionally, growing and cultivating the organisms or the parts thereof; and
   (f) recovering, and optionally isolating, the free or bound tryptophane produced by the selected mutated organisms or parts thereof.
5. The process of any one of claims 1 to 4, wherein the activity of said protein or the expression of said nucleic acid molecule is increased or generated transiently or stably.
6. An isolated nucleic acid molecule comprising a nucleic acid molecule selected from the group consisting of:
   (a) nucleic acid molecule encoding of a polypeptide as indicated in Table II, columns 5 or 7, lines 16 to 18 and/or lines 356 to 362 or a fragment thereof, which confers an increase in the amount of tryptophane in an organism or a part thereof;
   (b) nucleic acid molecule comprising of a nucleic acid as indicated in Table I, columns 5 or 7, lines 16 to 18 and/or lines 356 to 362;
   (c) nucleic acid molecule whose sequence can be deduced from a polypeptide sequence encoded by a nucleic acid molecule of (a) or (b) as a result of the degeneracy of the genetic code and conferring an increase in the amount of tryptophane in an organism or a part thereof;
   (d) nucleic acid molecule which encodes a polypeptide which has at least 50% identity with the amino acid sequence of the polypeptide encoded by the nucleic acid molecule of (a) to (c) and conferring an increase in the amount of tryptophane in an organism or a part thereof;
   (e) nucleic acid molecule which hybidizes with a nucleic acid molecule of (a) to (c) under under stringent hybridisation conditions and conferring an increase in the amount of tryptophane in an organism or a part thereof;
   (f) nucleic acid molecule which encompasses a nucleic acid molecule which is obtained by amplifying nucleic acid molecules from a cDNA library or a genomic library using the primers or primer pairs as indicated in Table III, 7, lines 16 to 18 and/or lines 356 to 362 and conferring an increase in the amount of tryptophane in an organism or a part thereof;
   (g) nucleic acid molecule encoding a polypeptide which is isolated with the aid of monoclonal antibodies against a polypeptide encoded by one of the nucleic acid molecules of (a) to (f) and conferring an increase in the amount of tryptophane in an organism or a part thereof;
   (h) nucleic acid molecule encoding a polypeptide comprising the consensus sequence as indicated in Table IV, column 7, lines 16 to 18 and/or lines 356 to 362 and conferring an increase in the amount of the fine chemical in an organism or a part thereof; and
   (i) nucleic acid molecule which is obtainable by screening a suitable nucleic acid library under stringent hybridization conditions with a probe comprising one of the sequences of the nucleic acid molecule of (a) to (k) or with a fragment thereof having at least 15 nt, preferably 20 nt, 30 nt, 50 nt, 100 nt, 200 nt or 500 nt of the nucleic acid molecule characterized in (a) to (k) and conferring an increase in the amount of the fine chemical in an organism or a part thereof.
   whereby the nucleic acid molecule distinguishes over the sequence as indicated in Table IA, columns 5 or 7, lines 16 to 18 and/or lines 356 to 362 by one or more nucleotides.
7. A nucleic acid construct which confers the expression of the nucleic acid molecule of claim 6, comprising one or more regulatory elements.
8. A vector comprising the nucleic acid molecule as claimed in claim 6 or the nucleic acid construct of claim 7.
9. The vector as claimed in claim 8, wherein the nucleic acid molecule is in operable linkage with regulatory sequences for the expression in a prokaryotic or eukaryotic, or in a prokaryotic and eukaryotic host.
10. A host cell, which has been transformed stably or transiently with the vector as claimed in claim 9 or 10 or the nucleic acid molecule as claimed in claim 6 or the nucleic acid construct of claim 7 or produced as described in claim any one of claims 2 to 5.
11. The host cell of claim 10, which is a transgenic host cell.
12. The host cell of claim 10 or 11, which is a plant cell, an animal cell, a microorganism, or a yeast cell, a fungus cell, a prokaryotic cell, an eukaryotic cell or an archaebacterium.
13. A process for producing a polypeptide, wherein the polypeptide is expressed in a host cell as claimed in any one of claims 10 to 12.
14. A polypeptide produced by the process as claimed in claim 13 or encoded by the nucleic acid molecule as claimed in claim 6 whereby the polypeptide distinguishes over a sequence as indicated in Table II A, columns 5 or 7, lines 16 to 18 and/or lines 356 to 362 by one or more amino acids.
15. An antibody, which binds specifically to the polypeptide as claimed in claim 14.
16. A plant tissue, propagation material, harvested material or a plant comprising the host cell as claimed in claim 12 which is plant cell or an Agrobacterium.
17. A method for screening for agonists and antagonists of the activity of a polypeptide encoded by the nucleic acid molecule of claim 6 conferring an increase in the amount of tryptophane in an organism or a part thereof comprising:
   (a) contacting cells, tissues , plants or microorganisms which express the a polypeptide encoded by the nucleic acid molecule of claim 6 conferring an increase in the amount of tryptophane in an organism or a part thereof with a candidate compound or a sample comprising a plurality of compounds under conditions which permit the expression the polypeptide;
   (b) assaying the tryptophane level or the polypeptide expression level in the cell, tissue, plant or microorganism or the media the cell, tissue, plant or microorganisms is cultured or maintained in; and
   (c) identifying a agonist or antagonist by comparing the measured tryptophane level or polypeptide expression level with a standard tryptophane or polypeptide expression level measured in the absence of said candidate compound or a sample comprising said plurality of compounds, whereby an increased level over the standard indicates that the compound or the sample comprising said plurality of compounds is an agonist and a decreased level over the standard indicates that the compound or the sample comprising said plurality of compounds is an antagonist.
18. A method for the identification of a compound conferring increased tryptophane production in a plant or microorganism, comprising the steps:
   a) culturing a plant cell or tissue or microorganism or maintaining a plant expressing the polypeptide encoded by the nucleic acid molecule of claim 6 conferring an increase in the amount of tryptophane in an organism or a part thereof and a readout system capable of interacting with the polypeptide under suitable conditions which permit the interaction of the polypeptide with dais readout system in the presence of a compound or a sample comprising a plurality of compounds and capable of providing a detectable signal in response to the binding of a compound to said polypeptide under conditions which permit the expression of said readout system and of the polypeptide encoded by the nucleic acid molecule of claim 6 conferring an increase in the amount of tryptophane in an organism or a part thereof;
   b) identifying if the compound is an effective agonist by detecting the presence or absence or increase of a signal produced by said readout system.
19. A method for the identification of a gene product conferring an increase in tryptophane production in a cell, comprising the following steps:
   a) contacting the nucleic acid molecules of a sample, which can contain a candidate gene encoding a gene product conferring an increase in tryptophane after expression with the nucleic acid molecule of claim 6;
   b) identifying the nucleic acid molecules, which hybridise under relaxed stringent conditions with the nucleic acid molecule of claim 6;
   c) introducing the candidate nucleic acid molecules in host cells appropriate for producing tryptophane ;
   d) expressing the identified nucleic acid molecules in the host cells;
   e) assaying the tryptophane level in the host cells; and
   f)identifying nucleic acid molecule and its gene product which expression confers an increase in the tryptophane level in the host cell in the host cell after expression compared to the wild type.
20. A method for the identification of a gene product conferring an increase in tryptophane production in a cell, comprising the following steps:
   a) identifying in a data bank nucleic acid molecules of an organism; which can contain a candidate gene encoding a gene product conferring an increase in the tryptophane amount or level in an organism or a part thereof after expression, and which are at least 20% homolog to the nucleic acid molecule of claim 6;
   b) introducing the candidate nucleic acid molecules in host cells appropriate for producing tryptophane ;
   c) expressing the identified nucleic acid molecules in the host cells;
   d) assaying the tryptophane level in the host cells; and
   e) identifying nucleic acid molecule and its gene product which expression confers an increase in the tryptophane level in the host cell after expression compared to the wild type.
21. A method for the production of an agricultural composition comprising the steps of the method of any one of claims 17 to 20 and formulating the compound identified in any one of claims 17 to 20 in a form acceptable for an application in agriculture.
22. A composition comprising the nucleic acid molecule of claim 6, the polypeptide of claim 14, the nucleic acid construct of claim 7, the vector of any one of claims 8 or 9, an antagonist or agonist identified according to claim 17, the compound of claim 18, the gene product of claim 19 or 20, the antibody of claim 15, and optionally an agricultural acceptable carrier.
23. Use of the nucleic acid molecule as claimed in claim 6 for the identification of a nucleic acid molecule conferring an increase of tryptophane after expression.
24. Use of the polypeptide of claim 14 or the nucleic acid construct claim 7 or the gene product identified according to the method of claim 19 or 20 for identifying compounds capable of conferring a modulation of tryptophane levels in an organism.
25. Food or feed composition comprising the nucleic acid molecule of claim 6, the polypeptide of claim 14, the nucleic acid construct of claim 7, the vector of claim 8 or 9, the antagonist or agonist identified according to claim 17, the antibody of claim 15, the plant or plant tissue of claim 16, the harvested material of claim 17, the host cell of claim 10 to 12 or the gene product identified according to the method of claim 19 or 20.
26. Use of the nucleic acid molecule of claim 6, the polypeptide of claim 14, the nucleic acid construct of claim 7, the vector of claim 8 or 9, the antagonist or agonist identified according to claim 17, the antibody of claim 15, the plant or plant tissue of claim 16, the host cell of claim 10 to 12 or the gene product identified according to the method of claim 19 or 20 for the protection of a plant against a tryptophane synthesis inhibiting herbicide.

**[0554.0.0.2] Abstract: see [0554.0.0.0]:**
**Process for the production of fine chemicals**

**[0000.0.0.3]** In a further embodiment, the present invention relates to a further process for the production of fine chemicals as defined below and corresponding embodiments as described herein as follows.

**[0001.0.0.3] to [0008.0.0.3]: see [0001.0.0.0] to [0008.0.0.0]**

**[0009.0.3.3]** As described above, the essential amino acids are necessary for humans and many mammals, for example for livestock. The branched-chain amino acids (BCAA) leucine, isoleucine and valine are among the nine dietary indispensable amino acids for humans. BCAA accounts for 35-40% of the dietary indispensable amino acids in body protein and 14% of the total amino acids in skeletal muscle (Ferrando et al., (1995) Oral branched chain amino acids decrease whole-body proteolysis. J. Parenter. Enteral Nutr. 19: 47-54.13). They share a common membrane transport system and enzymes for their transamination and irreversible oxidation (Block, K. P. (1989) Interactions among leucine, isoleucine, and valine with special reference to the branched chain amino acid antagonism. In: Absorption and Utilization of Amino Acids (Friedman, M., ed.), pp. 229-244, CRC Press, Boca Raton, FL. and Champe, P. C. & Harvey, R. A. (1987) Amino acids: metabolism of carbon atoms. In: Biochemistry (Champ, P. C. & Harvery, P. A., eds.), pp.242-252, J. B. Lippincott, Philadelphia, PA.). Further, for patient suffering from Maple Syrup Urine Disease (MSUD) a reduced uptake of those branched-chain amino acids is essential. Dietary sources of the branched-chain amino acids are principally derived from animal and vegetable proteins. The branched-chain amino acids (BCAA) leucine, isoleucine and valine are limiting for the growth of many mammals. Therefore the branched-chain amino acids are supplemented in the feed of broiler, leg hens, turkey, swine or cattle diets.

**[0010.0.0.3] see [0010.0.0.0]**

**[0011.0.0.3] see [0011.0.0.0]**

**[0012.0.3.3]** It is an object of the present invention to develop an inexpensive process for the synthesis of leucine and/or isoleucine and/or valine, preferably L-leucine and/or L-isoleucine and/or L-valine.

**[0013.0.0.3] see [0013.0.0.0]**

**[0014.0.3.3]** Accordingly, in a first embodiment, the invention relates to a process for the production of a fine chemical, whereby the fine chemical is leucine and/or isoleucine and/or valine, preferably L- leucine and/or L-isoleucine and/or L-valine. Accordingly, in the present invention, the term "the fine chemical" as used herein relates to "leucine and/or isoleucine and/or valine". Further, the term "the fine chemicals" as used herein also relates to fine chemicals comprising leucine and/or isoleucine and/or valine.

**[0015.0.3.3]** In one embodiment, the term "the fine chemical" means leucine and/or isoleucine and/or valine, preferably L- leucine and/or L-isoleucine and/or L-valine. Throughout the specification the term "the fine chemical" means leucine and/or isoleucine and/or valine, preferably L- leucine and/or L-isoleucine and/or L-valine, its salts, ester or amids in free form or bound to proteins. In a preferred embodiment, the term "the fine chemical" means leucine and/or isoleucine and/or valine, preferably L-leucine and/or L-isoleucine and/or L-valine, in free form or its salts or bound to proteins.

**[0016.0.3.3]** Accordingly, the present invention relates to a process comprising
(a) increasing or generating the activity of a YDL127W, YDR245W, YDR271C, YER173W, YGR101W, YJL072C, YKR057W, YNL135C, YFL013C, YGR104C, YIL150C, YOR350C, YFR042W, YFL019C, b1708, b1829, b2957, b3366, b0828, b3966, b4151, b1827, b0124, b0149, b0161, b0486, b1313, b1343, b1463, b2022, b2414, b2664, b3117, b3256, b3938, b3983, b4054, and/or b4327protein in a non-human organism in one or more parts thereof and
(b) growing the organism under conditions which permit the production of the fine chemical, thus, leucine and/or isoleucine and/or valine or fine chemicals comprising leucine and/or isoleucine and/or valine, in said organism.
and/or
(a) increasing or generating the activity of one or more proteins having the activity of one or more YDL127W, YDR245W, YDR271C, YER173W, YGR101W, YJL072C, YKR057W, YNL135C, YFL013C, YGR104C, YIL150C, YOR350C, YFR042W, YFL019C, b1708, b1829, b2957, b3366, b0828, b3966, b4151, b1827, b0124, b0149, b0161, b0486, b1313, b1343, b1463, b2022, b2414, b2664, b3117, b3256, b3938, b3983, b4054, and/or b4327 protein(s) or of a protein having the sequence of a polypeptide indicated in Table IIA or IIB, column 3, lines 19 to 29, 29a to 29u, 363 to 385 or having the sequence of a polypeptide encoded by a nucleic acid molecule indicated in Table IA or IB, column 5 or 7, lines 19 to 29, 29a to 29u, 363 to 385, in a non-human organism in one or more parts thereof and
(b) growing the organism under conditions which permit the production of the fine chemical, in particular isoleucine, valine and/or leucine in said organism
and/or
(a) increasing or generating the activity of one or more YDL127W, YDR245W, YDR271C, YER173W, YGR101W, YJL072C, YKR057W, YNL135C, YFL013C, YGR104C, YIL150C, YOR350C, YFR042W, YFL019C, b1708, b1829, b2957, b3366, b0828, b3966, b4151, b1827, b0124, b0149, b0161, b0486, b1313, b1343, b1463, b2022, b2414, b2664, b3117, b3256, b3938, b3983, b4054, and/or b4327 protein(s) or of a protein having the sequence of a polypeptide encoded by a nucleic acid molecule indicated in Table II, columns 5 or 7, lines 19 to 29, 29a to 29u, 363 to 385
   in a non-human organism in one or more parts thereof and
(b) growing the organism under conditions which permit the production of the fine chemical, meaning of leucine, valine and/or isoleucine or fine chemicals comprising leucine, valine and/or isoleucine in said organism;
and/or
(a) increasing or generating the activity of one or more
   YDL127W, YDR245W, YDR271C, YER173W, YGR101W, YJL072C, YKR057W, YNL135C, YFL013C, YGR104C, YIL150C, YOR350C, YFR042W, YFL019C, b1708, b1829, b2957, b3366, b0828, b3966, b4151, b1827, b0124, b0149, b0161, b0486, b1313, b1343, b1463, b2022, b2414, b2664, b3117, b3256, b3938, b3983, b4054, and/or b4327 protein(s) or of a protein having the sequence of a polypeptide encoded by a nucleic acid molecule indicated in Table II, columns 5 or 7, lines 19 to 29, 29a to 29u, 363 to 385
   in a non-human organism in one or more parts thereof and
(b) growing the organism under conditions which permit the production of the fine chemical, meaning of valine, leucine and/or isoleucine or fine chemicals comprising valine, leucine and/or isoleucine in said organism.
Accordingly, the present invention relates to a process for the production of a fine chemical comprising
(a) increasing or generating the activity of one or more proteins having the activity of a protein indicated in Table II, column 3, lines 19 to 29, 29a to 29u, 363 to 385 or having the sequence of a polypeptide encoded by a nucleic acid molecule indicated in Table I, column 5 or 7, lines 19 to 29, 29a to 29u, 363 to 385, in a non-human organism in one or more parts thereof and
(b) growing the organism under conditions which permit the production of the fine chemical, in particular arginine and/or glutamate and/or glutamine and/or proline resp..

**[0016.1.3.3]** Accordingly, the term "the fine chemical" means in one embodiment "isoleucine" in relation to all sequences listed in Table I to IV, lines 19 to 29, 29a, 363, 367, 373, 377, 379, 384or homologs thereof and means in one embodiment "valine" in relation to all sequences listed in Tables I to IV, lines 29a to 29j, 365, 369 to 372, 375, 376, 380, 381, 382, 385 or homologs thereof and means in one embodiment "leucine" in relation to all sequences listed in Table I to IV, lines 29k to 29u, 364, 366, 368, 374, 378, 383or homologs thereof.
Accordingly, in one embodiment the term "the fine chemical" means "valine , leucine and isoleucine" in relation to all sequences listed in Table I to IV, lines363, 364, 365, 367, 368 and 369, in one embodiment the term "the fine chemical" means "leucine and isoleucine" in relation to all sequences listed in Table I to IV, lines373 and 374, 377 and 378.
Accordingly, the term "the fine chemical" can mean "leucine" and/or "isoleucine" and/or "valine" owing to circumstances and the context. In order to illustrate that the meaning of the term "the fine chemical" means "arginine", and/or "glutamate" and/or "glutamine" and/or "proline" the term "the respective fine chemical" is also used.

**[0017.0.0.3] see [0017.0.0.0]**

**[0018.0.0.3] see [0018.0.0.0]**

**[0019.0.3.3]** Advantageously the process for the production of the fine chemical leads to an enhanced production of the fine chemical. The terms "enhanced" or "increase" mean at least a 10%, 20%, 30%, 40% or 50%, preferably at least 60%, 70%, 80%, 90% or 100%, more preferably 150%, 200%, 300%, 400% or 500% higher production of the respective fine chemical in comparison to the reference as defined below, e.g. that means in comparison to an organism without the aforementioned modification of the activity of a protein indicated in Table II, column 3, lines 19 to 29, 29a to 29u, 363 to 385 or encoded by nucleic acid molecule indicated in Table I, columns 5 or 7, lines19 to 29, 29a to 29u, 363 to 385.

**[0020.0.3.3]** Surprisingly it was found, that the transgenic expression of the Saccharomyces cerevisiae protein YDL127W, YDR245W, YDR271C, YER173W, YGR101W, YJL072C, YKR057W, YNL135C, YFL013C, YGR104C, YIL150C, YOR350C, YFL019C and/or YFR042W, and/or the Escherichia coli K12 protein b1708, b1829, b2957, b3366, b0828, b3966, b4151, b1827, b0124, b0149, b0161, b0486, b1313, b1343, b1463, b2022, b2414, b2664, b3117, b3256, b3938, b3983, b4054 and/or b4327_in Arabidopsis thaliana conferred an increase in the leucine and/or isoleucine and/or valine (or fine chemical) content of the transformed plants.

**[0021.0.0.3] see [0021.0.0.0]**

**[0022.0.3.3]** The sequence of YDL127W from Saccharomyces cerevisiae has been published in Jacq et al., Nature 387 (6632 Suppl), 75-78, 1997 and Goffeau, Science 274 (5287), 546-547, 1996, and its activity is beeing defined as a "G1/S-specific cyclin PCL2 (Cyclin HCS26 homolog) protein". Accordingly, in one embodiment, the process of the present invention comprises the use of a "G1/S-specific cyclin PCL2 (Cyclin HCS26 homolog) protein" or its homolog, e.g. as shown herein, for the production of the fine chemical, meaning of leucine and/or isoleucine and/or valine, in particular for increasing the amount of leucine and/or isoleucine and/or valine, preferably leucine and/or isoleucine and/or valine in free or bound form in an organism or a part thereof, as mentioned. In one embodiment, in the process of the present invention the activity of a "G1/S-specific cyclin PCL2 (Cyclin HCS26 homolog) protein" is increased or generated, e.g. from Saccharomyces cerevisiae or a homolog thereof.
The sequence of YDR245W from Saccharomyces cerevisiae has been published in Jacq et al., Nature 387 (6632 Suppl), 75-78, 1997 and Goffeau, Science 274 (5287), 546-547, 1996, and its activity is beeing defined as an galactosyl-(mannosyl)-transferase. Accordingly, in one embodiment, the process of the present invention comprises the use of a galactosyl-(mannosyl)-transferase or its homolog, e.g. as shown herein, for the production of the fine chemical, meaning of leucine and/or isoleucine and/or valine, in particular for increasing the amount of leucine and/or isoleucine and/or valine, preferably leucine and/or isoleucine and/or valine in free or bound form in an organism or a part thereof, as mentioned. In one embodiment, in the process of the present invention the activity of a,galactosyl-(mannosyl)-transferase is increased or generated, e.g. from Saccharomyces cerevisiae or a homolog thereof. The sequence of YDR271C was submitted by Le T., Johnston M., (March-1996) to the EMBUGenBank/DDBJ databases, by Waterston R.;(MAY-1996) and Jia Y., (JUNE-1997) to the EMBL/GenBank/DDBJ databases and its cellular activity has not been characterized yet. Accordingly, in one embodiment, the process of the present invention comprises the use of a YDR271C activity from Saccaromyces cerevisiae or its homolog, e.g. as shown herein, for the production of the fine chemical, meaning of leucine and/or isoleucine and/or valine, in particular for increasing the amount of leucine and/or isoleucine and/or valine, preferably leucine and/or isoleucine and/or valine in free or bound form in an organism or a part thereof, as mentioned. In one embodiment, in the process of the present invention the activity of a YDR271C protein is increased or generated, e.g. from Saccharomyces cerevisiae or a homolog thereof. The sequence of YER173w from Saccharomyces cerevisiae has been published in Dietrich, Nature 387 (6632 Suppl), 78-81, 1997, and Goffeau, Science 274 (5287), 546-547, 1996, and its activity is beeing defined as an "Checkpoint protein, involved in the activation of the DNA damage and meiotic pachytene checkpoints;". Accordingly, in one embodiment, the process of the present invention comprises the use of a "Checkpoint protein, involved in the activation of the DNA damage and meiotic pachytene checkpoints" or its homolog, e.g. as shown herein, for the production of the fine chemical, meaning of leucine and/or isoleucine and/or valine, in particular for increasing the amount of leucine and/or isoleucine and/or valine, preferably leucine and/or isoleucine and/or valine in free or bound form in an organism or a part thereof, as mentioned. In one embodiment, in the process of the present invention the activity of a "Checkpoint protein, involved in the activation of the DNA damage and meiotic pachytene checkpoints" is increased or generated, e.g. from Saccharomyces cerevisiae or a homolog thereof.
The sequence of YGR101W from Saccharomyces cerevisiae has been published in Tettelin,H., Nature 387 (6632 Suppl), 81-84, 1997 and Goffeau,A., Science 274 (5287), 546-547, 1996, and its activity is beeing defined as a rhomboid protease. Accordingly, in one embodiment, the process of the present invention comprises the use of a rhomboid protease or its homolog, for the production of the fine chemical, meaning of leucine and/or isoleucine and/or valine, in particular for increasing the amount of leucine and/or isoleucine and/or valine, preferably leucine and/or isoleucine and/or valine in free or bound form in an organism or a part thereof, as mentioned. In one embodiment, in the process of the present invention the activity of a rhomboid protease is increased or generated, e.g. from Saccharomyces cerevisiae or a homolog thereof. The sequence of YJL072C from Saccharomyces cerevisiae has been published in Goffeau, A., Science 274 (5287), 546-547, 1996 and Galibert,F., EMBO J. 15 (9), 2031-2049, 1996, and its activity is beeing defined as a "subunit of the GINS complex required for chromosomal DNA replication". Accordingly, in one embodiment, the process of the present invention comprises the use of a "subunit of the GINS complex required for chromosomal DNA replication" or its homolog, for the production of the fine chemical, meaning of leucine and/or isoleucine and/or valine, in particular for increasing the amount of leucine and/or isoleucine and/or valine, preferably leucine and/or isoleucine and/or valine in free or bound form in an organism or a part thereof, as mentioned. In one embodiment, in the process of the present invention the activity of a "subunit of the GINS complex required for chromosomal DNA replication" is increased or generated, e.g. from Saccharomyces cerevisiae or a homolog thereof. The sequence of YKR057W from Saccharomyces cerevisiae has been published in Dujon et al., Nature 369 (6479), 371-378, 1994 and Goffeau et al., Science 274 (5287), 546-547, 1996 and its activity is beeing defined as a ribosomal protein, similar to S21A, S26A and/or YS25 ribosomal proteins, involved in ribosome biogenesis and translation. Accordingly, in one embodiment, the process of the present invention comprises the use of a ribosomal protein, similar to S21A, S26A and/or YS25 ribosomal proteins, involved in ribosome biogenesis and translation from Saccaromyces cerevisiae or its homolog, e.g. as shown herein, for the production of the fine chemical, meaning of leucine and/or isoleucine and/or valine, in particular for increasing the amount of leucine and/or isoleucine and/or valine, preferably leucine and/or isoleucine and/or valine in free or bound form in an organism or a part thereof, as mentioned. In one embodiment, in the process of the present invention the activity of a ribosomal protein, similar to S21A, S26A and/or YS25 ribosomal proteins, involved in ribosome biogenesis and translation is increased or generated, e.g. from Saccharomyces cerevisiae or a homolog thereof.
The sequence of YNL135C from Saccharomyces cerevisiae has been published in Philippsen, P., Nature 387 (6632 Suppl), 93-98, 1997 and Goffeau, A., Science 274 (5287), 546-547, 1996, and its activity is beeing defined as a peptidylprolyl isomerase. Accordingly, in one embodiment, the process of the present invention comprises the use of a peptidylprolyl isomerase or its homolog, for the production of the fine chemical, meaning of leucine and/or isoleucine and/or valine, in particular for increasing the amount of leucine and/or isoleucine and/or valine, preferably leucine and/or isoleucine and/or valine in free or bound form in an organism or a part thereof, as mentioned. In one embodiment, in the process of the present invention the activity of a peptidylprolyl isomerase is increased or generated, e.g. from Saccharomyces cerevisiae or a homolog thereof.
The sequence of YFL013C from Saccharomyces cerevisiae has been published in Goffeau, A., Science 274 (5287), 546-547, 1996 and Murakami, Y., Nat. Genet. 10 (3), 261-268, 1995, and its activity is beeing defined as a "subunit of the IN080 chromatin remodeling complex". Accordingly, in one embodiment, the process of the present invention comprises the use of a "subunit of the INO80 chromatin remodeling complex" or its homolog, for the production of the fine chemical, meaning of leucine and/or isoleucine and/or valine, in particular for increasing the amount of leucine and/or isoleucine and/or valine, preferably leucine and/or isoleucine and/or valine in free or bound form in an organism or a part thereof, as mentioned. In one embodiment, in the process of the present invention the activity of a "subunit of the INO80 chromatin remodeling complex" protein is increased or generated, e.g. from Saccharomyces cerevisiae or a homolog thereof.
The sequence of YGR104C from Saccharomyces cerevisiae has been published in Thompson et al., Cell 73:1361-1375, 1993, and its activity is beeing defined as an "RNA polymerase II suppressor protein SRB5 - yeast and/or suppressor of RNA polymerase B SRB5" ". Accordingly, in one embodiment, the process of the present invention comprises the use of a "RNA polymerase II suppressor protein SRB5 - yeast and/or suppressor of RNA polymerase B SRB5" or its homolog, for the production of the fine chemical, meaning of leucine and/or isoleucine and/or valine, in particular for increasing the amount of leucine and/or isoleucine and/or valine, preferably leucine and/or isoleucine and/or valine in free or bound form in an organism or a part thereof, as mentioned. In one embodiment, in the process of the present invention the activity of a "RNA polymerase II suppressor protein SRB5 - yeast and/or suppressor of RNA polymerase B SRB5" is increased or generated, e.g. from Saccharomyces cerevisiae or a homolog thereof.
The sequence of YIL150C from Saccharomyces cerevisiae has been published in Goffeau et al., Science 274 (5287), 546-547, 1996 and Churcher et al., Nature 387 (6632 Suppl), 84-87, 1997 and its activity is beeing defined as a chromatin binding protein, required for S-phase (DNA synthesis) initiation or completion. Accordingly, in one embodiment, the process of the present invention comprises the use of a chromatin binding protein, required for S-phase (DNA synthesis) initiation or completion, from Saccaromyces cerevisiae or its homolog, e.g. as shown herein, for the production of the fine chemical, meaning of leucine and/or isoleucine and/or valine, in particular for increasing the amount of leucine and/or isoleucine and/or valine, preferably leucine and/or isoleucine and/or valine in free or bound form in an organism or a part thereof, as mentioned. In one embodiment, in the process of the present invention the activity of a chromatin binding protein, required for S-phase (DNA synthesis) initiation or completion is increased or generated, e.g. from Saccharomyces cerevisiae or a homolog thereof.
The sequence of YOR350C from Saccharomyces cerevisiae has been published in Goffeau, A., Science 274 (5287), 546-547, 1996 and Dujon, B., Nature 387 (6632 Suppl), 98-102, 1997, and its cellular activity has not been characterized yet. Accordingly, in one embodiment, the process of the present invention comprises the use of a YOR350C protein or its homolog, for the production of the fine chemical, meaning of leucine and/or isoleucine and/or valine, in particular for increasing the amount of leucine and/or isoleucine and/or valine, preferably leucine and/or isoleucine and/or valine in free or bound form in an organism or a part thereof, as mentioned. In one embodiment, in the process of the present invention the activity of a YOR350C protein is increased or generated, e.g. from Saccharomyces cerevisiae or a homolog thereof.
The sequence of YFR042W from Saccharomyces cerevisiae has been published in Goffeau st al., Science 274 (5287), 546-547, 1996 and Murakami,Y., Nat. Genet. 10 (3), 261-268, 1995 and its activity is beeing defined as a "protein required for cell viability in yeast". Accordingly, in one embodiment, the process of the present invention comprises the use of a "protein required for cell viability in yeast", from Saccaromyces cerevisiae or its homolog, e.g. as shown herein, for the production of the fine chemical, meaning of leucine and/or isoleucine and/or valine, in particular for increasing the amount of leucine and/or isoleucine and/or valine, preferably leucine and/or isoleucine and/or valine in free or bound form in an organism or a part thereof, as mentioned. In one embodiment, in the process of the present invention the activity of a "protein required for cell viability in yeast" is increased or generated, e.g. from Saccharomyces cerevisiae or a homolog thereof.
The sequence of YFL019C from Saccharomyces cerevisiae has been published in Murakami, Y., Nat. Genet. 10 (3), 261-268, 1995 and its activity is beeing defined as a hypothetical 13.7 kDa protein in the PAU5-LPD1 intergenic region. Accordingly, in one embodiment, the process of the present invention comprises the use of the protein encoded by the nucleic acid sequence YFL019C, from Saccaromyces cerevisiae or its homolog, e.g. as shown herein, for the production of the fine chemical, meaning of leucine and/or isoleucine and/or valine, in particular for increasing the amount of leucine and/or isoleucine and/or valine, preferably leucine and/or isoleucine and/or valine in free or bound form in an organism or a part thereof, as mentioned. In one embodiment, in the process of the present invention the activity of a protein encoded by the nucleic acid sequence YFL019C is increased or generated, e.g. from Saccharomyces cerevisiae or a homolog thereof.
The sequence of b1708 from Escherichia coli K12 has been published in Blattner et al., Science 277(5331), 1453-1474, 1997, and its activity is beeing defined as a lipoprotein. Accordingly, in one embodiment, the process of the present invention comprises the use of a lipoprotein from E. coli or its homolog, e.g. as shown herein, for the production of the fine chemical, meaning of leucine and/or isoleucine and/or valine, in particular for increasing the amount of leucine and/or isoleucine and/or valine, preferably leucine and/or isoleucine and/or valine in free or bound form in an organism or a part thereof, as mentioned. In one embodiment, in the process of the present invention the activity of a lysine decarboxylase is increased or generated, e.g. from E. coli or a homolog thereof.
The sequence of b1829 from Escherichia coli K12 has been published in Blattner et al., Science 277(5331), 1453-1474, 1997, and its activity is beeing defined as a heat shock protein with protease activity (htpx). Accordingly, in one embodiment, the process of the present invention comprises the use of a heat shock protein with protease activity (htpx) from E. coli or its homolog, e.g. as shown herein, for the production of the fine chemical, meaning of leucine and/or isoleucine and/or valine, in particular for increasing the amount of leucine and/or isoleucine and/or valine, preferably leucine and/or isoleucine and/or valine in free or bound form in an organism or a part thereof, as mentioned. In one embodiment, in the process of the present invention the activity of a heat shock protein with protease (htpx) activity is increased or generated, e.g. from E. coli or a homolog thereof.
The sequence of b2957 from Escherichia coli K12 has been published in Blattner et al., Science 277(5331), 1453-1474, 1997, and its activity is beeing defined as a periplasmic L-asparaginase II. Accordingly, in one embodiment, the process of the present invention comprises the use of a periplasmic L-asparaginase II from E. coli or its homolog, e.g. as shown herein, for the production of the fine chemical, meaning of leucine and/or isoleucine and/or valine, in particular for increasing the amount of leucine and/or isoleucine and/or valine, preferably leucine and/or isoleucine and/or valine in free or bound form in an organism or a part thereof, as mentioned. In one embodiment, in the process of the present invention the activity of periplasmic L-asparaginase II is increased or generated, e.g. from E. coli or a homolog thereof.
The sequence of b3366 from Escherichia coli K12 has been published in Blattner et al., Science 277(5331), 1453-1474, 1997, and its activity is beeing defined as the a small subunit of a nitrite reductase. Accordingly, in one embodiment, the process of the present invention comprises the use of a small subunit of a nitrite reductase from E. coli or its homolog, e.g. as shown herein, for the production of the fine chemical, meaning of leucine and/or isoleucine and/or valine, in particular for increasing the amount of leucine and/or isoleucine and/or valine, preferably leucine and/or isoleucine and/or valine in free or bound form in an organism or a part thereof, as mentioned. In one embodiment, in the process of the present invention the activity of the small subunit of a nitrite reductase, preferably of the small subunit of a nitrite reductase is increased or generated, e.g. from E. coli or a homolog thereof.
The sequence of b0828 from Escherichia coli K12 has been published in Blattner et al., Science 277(5331), 1453-1474, 1997, and its activity is beeing defined as the a probable asparaginase (EC:3.5.1.1) and/or ybiK protein (L-asparagine amidohydrolase). Accordingly, in one embodiment, the process of the present invention comprises the use of a probable asparaginase (EC:3.5.1.1) and/or ybiK protein (L-asparagine amidohydrolase) from E. coli or its homolog, e.g. as shown herein, for the production of the fine chemical, meaning of leucine and/or isoleucine and/or valine, in particular for increasing the amount of leucine and/or isoleucine and/or valine, preferably leucine and/or isoleucine and/or valine in free or bound form in an organism or a part thereof, as mentioned. In one embodiment, in the process of the present invention the activity of a probable asparaginase (EC:3.5.1.1) and/or ybiK protein (L-asparagine amidohydrolase) is increased or generated, e.g. from E. coli or a homolog thereof.
The sequence of b3966 from Escherichia coli K12 has been published in R;Heller, J. Bacteriol. 161, 904-908, 1985; R;Doublet, J. Bacteriol. 174, 5772-5779, 1992 and R;Gustafsson, J. Bacteriol. 173, 1757-1764, 1991, and its activity is beeing defined as a outer membrane porin. Accordingly, in one embodiment, the process of the present invention comprises the use of a outer membrane porin from E. coli or its homolog, e.g. as shown herein, for the production of the fine chemical, meaning of leucine and/or isoleucine and/or valine, in particular for increasing the amount of leucine and/or isoleucine and/or valine, preferably leucine and/or isoleucine and/or valine in free or bound form in an organism or a part thereof, as mentioned. In one embodiment, in the process of the present invention the activity of a outer membrane porin is increased or generated, e.g. from E. coli or a homolog thereof.
The sequence of b4151 from Escherichia coli K12 has been published in Blattner et al., Science 277(5331), 1453-1474, 1997, and its activity is beeing defined as a fumarate reductase. Accordingly, in one embodiment, the process of the present invention comprises the use of a fumarate reductase from E. coli or its homolog, e.g. as shown herein, for the production of the fine chemical, meaning of leucine and/or isoleucine and/or valine, in particular for increasing the amount of leucine and/or isoleucine and/or valine, preferably leucine and/or isoleucine and/or valine in free or bound form in an organism or a part thereof, as mentioned. In one embodiment, in the process of the present invention the activity of a fumarate reductase is increased or generated, e.g. from E. coli or a homolog thereof.
The sequence of b1827 from Escherichia coli K12 has been published in Blattner et al., Science 277(5331), 1453-1474, 1997, and its activity is beeing defined as a repressor protein with a DNA-binding Winged helix domain (IcIR family). Accordingly, in one embodiment, the process of the present invention comprises the use of a repressor protein with a DNA-binding Winged helix domain (IcIR family) from E. coli or its homolog, e.g. as shown herein, for the production of the fine chemical, meaning of leucine and/or isoleucine and/or valine, in particular for increasing the amount of leucine and/or isoleucine and/or valine, preferably leucine and/or isoleucine and/or valine in free or bound form in an organism or a part thereof, as mentioned. In one embodiment, in the process of the present invention the activity of a repressor protein with a DNA-binding Winged helix domain (IcIR family)is increased or generated, e.g. from E. coli or a homolog thereof.
The sequence of b0124 from Escherichia coli K12 has been published in Blattner et al., Science 277(5331), 1453-1474, 1997, and its activity is being defined as a protein having glucose dehydrogenase activity. Accordingly, in one embodiment, the process of the present invention comprises the use of a glucose dehydrogenase from E. coli or its homolog, e.g. as shown herein, for the production of the fine chemical, meaning of leucine and/or isoleucine and/or valine, in particular for increasing the amount of leucine and/or isoleucine and/or valine, preferably leucine and/or isoleucine and/or valine in free or bound form in an organism or a part thereof, as mentioned. In one embodiment, in the process of the present invention the activity of a glucose dehydrogenase is increased or generated, e.g. from E. coli or a homolog thereof.
The sequence of b0149 from Escherichia coli K12 has been published in Blattner et al., Science 277(5331), 1453-1474, 1997, and its activity is being defined as a bifunctional penicillin g-binding protein 1b: glycosyl transferase (N-terminal); transpeptidase (C-terminal). Accordingly, in one embodiment, the process of the present invention comprises the use of a bifunctional penicillin g-binding protein 1b: glycosyl transferase (N-terminal); transpeptidase (C-terminal) from E. coli or its homolog, e.g. as shown herein, for the production of the fine chemical, meaning of leucine and/or isoleucine and/or valine, in particular for increasing the amount of leucine and/or isoleucine and/or valine, preferably leucine and/or isoleucine and/or valine in free or bound form in an organism or a part thereof, as mentioned. In one embodiment, in the process of the present invention the activity of a protein with a bifunctional penicillin g-binding protein 1b: glycosyl transferase (N-terminal); transpeptidase (C-terminal) is increased or generated, e.g. from E. coli or a homolog thereof.
The sequence of b0161 from Escherichia coli K12 has been published in Blattner et al., Science 277(5331), 1453-1474, 1997, and its activity is being defined as a periplasmic serine protease Do, heat shock protein; periplasmic serine protease Do; heat shock protein HtrA. Accordingly, in one embodiment, the process of the present invention comprises the use of a periplasmic serine protease Do, heat shock protein; periplasmic serine protease Do; heat shock protein HtrA from E. coli or its homolog, e.g. as shown herein, for the production of the fine chemical, meaning of leucine and/or isoleucine and/or valine, in particular for increasing the amount of leucine and/or isoleucine and/or valine, preferably leucine and/or isoleucine and/or valine in free or bound form in an organism or a part thereof, as mentioned. In one embodiment, in the process of the present invention the activity of a periplasmic serine protease Do, heat shock protein; periplasmic serine protease Do; heat shock protein HtrA is increased or generated, e.g. from E. coli or a homolog thereof.
The sequence of b0486 from Escherichia coli K12 has been published in Blattner et al., Science 277(5331), 1453-1474, 1997, and its activity is being defined as a putative amino acid/amine transport protein. Accordingly, in one embodiment, the process of the present invention comprises the use of a putative amino acid/amine transport protein from E. coli or its homolog, e.g. as shown herein, for the production of the fine chemical, meaning of leucine and/or isoleucine and/or valine, in particular for increasing the amount of leucine and/or isoleucine and/or valine, preferably leucine and/or isoleucine and/or valine in free.or bound form in an organism or a part thereof, as mentioned. In one embodiment, in the process of the present invention the activity of a putative amino acid/amine transport protein is increased or generated, e.g. from E. coli or a homolog thereof.
The sequence of b1313 from Escherichia coli K12 has been published in Blattner et al., Science 277(5331), 1453-1474, 1997, and its activity is being defined as a putative dehydrogenase with NAD(P)-binding and GroES domains. Accordingly, in one embodiment, the process of the present invention comprises the use of a putative dehydrogenase with NAD(P)-binding and GroES domainsprotein from E. coli or its homolog, e.g. as shown herein, for the production of the fine chemical, meaning of leucine and/or isoleucine and/or valine, in particular for increasing the amount of leucine and/or isoleucine and/or valine, preferably leucine and/or isoleucine and/or valine in free or bound form in an organism or a part thereof, as mentioned. In one embodiment, in the process of the present invention the activity of a dehydrogenase with NAD(P)-binding and GroES domainsprotein is increased or generated, e.g. from E. coli or a homolog thereof.
The sequence of b1343 from Escherichia coli K12 has been published in Blattner et al., Science 277(5331), 1453-1474, 1997, and its activity is being defined as an ATP-dependent RNA helicase. Accordingly, in one embodiment, the process of the present invention comprises the use of a protein with an ATP-dependent RNA helicase from E. coli or its homolog, e.g. as shown herein, for the production of the fine chemical, meaning of leucine and/or isoleucine and/or valine, in particular for increasing the amount of leucine and/or isoleucine and/or valine, preferably leucine and/or isoleucine and/or valine in free or bound form in an organism or a part thereof, as mentioned. In one embodiment, in the process of the present invention the activity of an ATP-dependent RNA helicase is increased or generated, e.g. from E. coli or a homolog thereof.
The sequence of b1463 from Escherichia coli K12 has been published in Blattner et al., Science 277(5331), 1453-1474, 1997, and its activity is being defined as N-hydroxyarylamine O-acetyltransferase. Accordingly, in one embodiment, the process of the present invention comprises the use of a N-hydroxyarylamine O-acetyltransferase from E. coli or its homolog, e.g. as shown herein, for the production of the fine chemical, meaning of leucine and/or isoleucine and/or valine, in particular for increasing the amount of leucine and/or isoleucine and/or valine, preferably leucine and/or isoleucine and/or valine in free or bound form in an organism or a part thereof, as mentioned. In one embodiment, in the process of the present invention the activity of a N-hydroxyarylamine O-acetyltransferase is increased or generated, e.g. from E. coli or a homolog thereof.
The sequence of b2022 from Escherichia coli K12 has been published in Blattner et al., Science 277(5331), 1453-1474, 1997, and its activity is being defined as a bifunctional: histidinol-phosphatase (N-terminal); imidazoleglycerol-phosphate dehydratase (C-terminal); imidazoleglycerolphosphate dehydratase and histidinol-phosphate phosphatase. Accordingly, in one embodiment, the process of the present invention comprises the use of a bifunctional: histidinol-phosphatase (N-terminal); imidazoleglycerol-phosphate dehydratase (C-terminal); imidazoleglycerolphosphate dehydratase and histidinol-phosphate phosphatase from E. coli or its homolog, e.g. as shown herein, for the production of the fine chemical, meaning of leucine and/or isoleucine and/or valine, in particular for increasing the amount of leucine and/or isoleucine and/or valine, preferably leucine and/or isoleucine and/or valine in free or bound form in an organism or a part thereof, as mentioned. In one embodiment, in the process of the present invention the activity of a bifunctional: histidinol-phosphatase (N-terminal); imidazoleglycerol-phosphate dehydratase (C-terminal); imidazoleglycerol-phosphate dehydratase and histidinol-phosphate phosphatase is increased or generated, e.g. from E. coli or a homolog thereof.
The sequence of b2414 from Escherichia coli K12 has been published in Blattner et al., Science 277(5331), 1453-1474, 1997, and its activity is being defined as a subunit of cysteine synthase A and O-acetylserine sulfhydrolase A, a PLP-dependent enzyme. Accordingly, in one embodiment, the process of the present invention comprises the use of a subunit of cysteine synthase A and O-acetylserine sulfhydrolase A, a PLP-dependent enzyme from E. coli or its homolog, e.g. as shown herein, for the production of the fine chemical, meaning of leucine and/or isoleucine and/or valine, in particular for increasing the amount of leucine and/or isoleucine and/or valine, preferably leucine and/or isoleucine and/or valine in free or bound form in an organism or a part thereof, as mentioned. In one embodiment, in the process of the present invention the activity of a subunit of cysteine synthase A and O-acetylserine sulfhydrolase A, a PLP-dependent enzyme is increased or generated, e.g. from E. coli or a homolog thereof.
The sequence of b2664 from Escherichia coli K12 has been published in Blattner et al., Science 277(5331), 1453-1474, 1997, and its activity is being defined as putative transcriptional repressor with DNA-binding Winged helix domain (GntR familiy) protein. Accordingly, in one embodiment, the process of the present invention comprises the use of a protein with a putative transcriptional repressor with DNA-binding Winged helix domain (GntR familiy) activity from E. coli or its homolog, e.g. as shown herein, for the production of the fine chemical, meaning of leucine and/or isoleucine and/or valine, in particular for increasing the amount of leucine and/or isoleucine and/or valine, preferably leucine and/or isoleucine and/or valine in free or bound form in an organism or a part thereof, as mentioned. In one embodiment, in the process of the present invention the activity of a putative transcriptional repressor with DNA-binding Winged helix domain (GntR familiy) is increased or generated, e.g. from E. coli or a homolog thereof.
The sequence of b3117 from Escherichia coli K12 has been published in Blattner et al., Science 277(5331), 1453-1474, 1997, and its activity is being defined as a threonine dehydratase, catabolic, PLP-dependent enzyme. Accordingly, in one embodiment, the process of the present invention comprises the use of a threonine dehydrotase, catabolic, PLP-dependent enzyme from E. coli or its homolog, e.g. as shown herein, for the production of the fine chemical, meaning of leucine and/or isoleucine and/or valine, in particular for increasing the amount of leucine and/or isoleucine and/or valine, preferably leucine and/or isoleucine and/or valine in free or bound form in an organism or a part thereof, as mentioned. In one embodiment, in the process of the present invention the activity of a threonine dehydratase, catabolic, PLP-dependent enzyme is increased or generated, e.g. from E. coli or a homolog thereof.
The sequence of b3256 from Escherichia coli K12 has been published in Blattner et al., Science 277(5331), 1453-1474, 1997, and its activity is being defined as an acetyl CoA carboxylase. Accordingly, in one embodiment, the process of the present invention comprises the use of an acetyl CoA carboxylase from E. coli or its homolog, e.g. as shown herein, for the production of the fine chemical, meaning of leucine and/or isoleucine and/or valine, in particular for increasing the amount of leucine and/or isoleucine and/or valine, preferably leucine and/or isoleucine and/or valine in free or bound form in an organism or a part thereof, as mentioned. In one embodiment, in the process of the present invention the activity of an acetyl CoA carboxylase enzyme is increased or generated, e.g. from E. coli or a homolog thereof.
The sequence of b3938 from Escherichia coli K12 has been published in Blattner et al., Science 277(5331), 1453-1474, 1997, and its activity is being defined as a transcriptional repressor for methionine biosynthesis . Accordingly, in one embodiment, the process of the present invention comprises the use of a transcriptional repressor activity for methionine biosynthesis from E. coli or its homolog, e.g. as shown herein, for the production of the fine chemical, meaning of leucine and/or isoleucine and/or valine, in particular for increasing the amount of leucine and/or isoleucine and/or valine, preferably leucine and/or isoleucine and/or valine in free or bound form in an organism or a part thereof, as mentioned. In one embodiment, in the process of the present invention the activity of a transcriptional repressor for methionine biosynthesis is increased or generated, e.g. from E. coli or a homolog thereof.
The sequence of b3983 from Escherichia coli K12 has been published in Blattner et al., Science 277(5331), 1453-1474, 1997, and its activity is being defined as a 50S ribosomal subunit protein L12. Accordingly, in one embodiment, the process of the present invention comprises the use of a 50S ribosomal subunit protein L12 from E. coli or its homolog, e.g. as shown herein, for the production of the fine chemical, meaning of leucine and/or isoleucine and/or valine, in particular for increasing the amount of leucine and/or isoleucine and/or valine, preferably leucine and/or isoleucine and/or valine in free or bound form in an organism or a part thereof, as mentioned. In one embodiment, in the process of the present invention the activity of a 50S ribosomal subunit protein L12 is increased or generated, e.g. from E. coli or a homolog thereof.
The sequence of b4054 from Escherichia coli K12 has been published in Blattner et al., Science 277(5331), 1453-1474, 1997, and its activity is being defined as a tyrosine aminotransferase, tyrosine repressible. Accordingly, in one embodiment, the process of the present invention comprises the use of a tyrosine aminotransferase, tyrosine repressible from E. coli or its homolog, e.g. as shown herein, for the production of the fine chemical, meaning of leucine and/or isoleucine and/or valine, in particular for increasing the amount of leucine and/or isoleucine and/or valine, preferably leucine and/or isoleucine and/or valine in free or bound form in an organism or a part thereof, as mentioned. In one embodiment, in the process of the present invention the activity of a tyrosine aminotransferase, tyrosine repressible is increased or generated, e.g. from E. coli or a homolog thereof.
The sequence of b4327 from Escherichia coli K12 has been published in Blattner et al., Science 277(5331), 1453-1474, 1997, and its activity is being defined as a HTH-type transcriptional regulator with periplasmic binding protein domain (LysR family). Accordingly, in one embodiment, the process of the present invention comprises the use of a HTH-type transcriptional regulator with periplasmic binding protein domain (LysR family) from E. coli or its homolog, e.g. as shown herein, for the production of the fine chemical, meaning of leucine and/or isoleucine and/or valine, in particular for increasing the amount of leucine and/or isoleucine and/or valine, preferably leucine and/or isoleucine and/or valine in free or bound form in an organism or a part thereof, as mentioned. In one embodiment, in the process of the present invention the activity of a HTH-type transcriptional regulator with periplasmic binding protein domain (LysR family) is increased or generated, e.g. from E. coli or a homolog thereof.

**[0023.0.3.3]** In one embodiment, the homolog of the YDL127W, YDR245W, YDR271C, YER173W, YGR101W, YJL072C, YKR057W, YNL135C, YFL013C, YGR104C, YIL150C, YOR350C, YFR042W and/or YFL019C is a homolog having said acitivity and being derived from an eukaryotic. In one embodiment, the homolog of the b1708, b1829, b2957, b3366, b0828, b3966, b4151,b1827, b0124, b0149, b0161, b0486, b1313, b1343, b1463, b2022, b2414, b2664, b3117, b3256, b3938, b3983, b4054 and/or b4327 is a homolog having said acitivity and being derived from bacteria. In one embodiment, the homolog of the YDL127W, YDR245W, YDR271C, YER173W, YGR101W, YJL072C, YKR057W, YNL135C, YFL013C, YGR104C, YIL150C, YOR350C, YFL019C and/or YFR042W is a homolog having said acitivity and being derived from Fungi. In one embodiment, the homolog of the b1708, b1829, b2957, b3366, b0828, b3966, b4151, b1827, b0124, b0149, b0161, b0486, b1313, b1343, b1463, b2022, b2414, b2664, b3117, b3256, b3938, b3983, b4054 and/or b4327 is a homolog having said acitivity and being derived from Proteobacteria. In one embodiment, the homolog of the YDL127W, YDR245W, YDR271C, YER173W, YGR1 01 W, YJL072C, YKR057W, YNL135C, YFL013C, YGR104C, YIL150C, YOR350C, YFL019C and/or YFR042W is a homolog having said acitivity and being derived from Ascomyceta. In one embodiment, the homolog of the b1708, b1829, b2957, b3366, b0828, b3966, b4151, b1827, b0124, b0149, b0161, b0486, b1313, b1343, b1463, b2022, b2414, b2664, b3117, b3256, b3938, b3983, b4054 and/or b4327 is a homolog having said acitivity and being derived from Gammaproteobacteria. In one embodiment, the homolog of the YDL127W, YDR245W, YDR271C, YER173W, YGR101W, YJL072C, YKR057W, YNL135C, YFL013C, YGR104C, YIL150C, YOR350C, YFL019C and/or YFR042W is a homolog having said acitivity and being derived from Saccharomycotina. In one embodiment, the homolog of the b1708, b1829, b2957, b3366, b0828, b3966, b4151, b1827, b0124, b0149, b0161, b0486, b1313, b1343, b1463, b2022, b2414, b2664, b3117, b3256, b3938, b3983, b4054 and/or b4327 is a homolog having said acitivity and being derived from Enterobacteriales. In one embodiment, the homolog of the YDL127W, YDR245W, YDR271C, YER173W, YGR101W, YJL072C, YKR057W, YNL135C, YFL013C, YGR104C, YIL150C, YOR350C, YFL019C and/or YFR042W is a homolog having said acitivity and being derived from Saccharomycetes. In one embodiment, the homolog of the b1708, b1829, b2957, b3366, b0828, b3966, b4151, b1827, b0124, b0149, b0161, b0486, b1313, b1343, b1463, b2022, b2414, b2664, b3117, b3256, b3938, b3983, b4054 and/or b4327 is a homolog having said acitivity and being derived from Enterobacteriaceae. In one embodiment, the homolog of the YDL127W, YDR245W, YDR271C, YER173W, YGR101W, YJL072C, YKR057W, YNL135C, YFL013C, YGR104C, YIL150C, YOR350C, YFL019C and/or YFR042W is a homolog having said acitivity and being derived from Saccharomycetales. In one embodiment, the homolog of the b1708, b1829, b2957, b3366, b0828, b3966, b4151 b1827, b0124, b0149, b0161, b0486, b1313, b1343, b1463, b2022, b2414, b2664, b3117, b3256, b3938, b3983, b4054 and/or b4327 is a homolog having said acitivity and being derived from Escherichia. In one embodiment, the homolog of the YDL127W, YDR245W, YDR271C, YER173W, YGR101W, YJL072C, YKR057W, YNL135C, YFL013C, YGR104C, YIL150C, YOR350C, YFL019C and/or YFR042W is a homolog having said acitivity and being derived from Saccharomycetaceae. In one embodiment, the homolog of the YDL127W, YDR245W, YDR271C, YER173W, YGR101W, YJL072C, YKR057W, YNL135C, YFL013C, YGR104C, YIL150C, YOR350C, YFL019C and/or YFR042W is a homolog having said acitivity and being derived from Saccharomycetes.

**[0023.1.0.3]** Homologs of the polypeptide indicated in Table II, column 3, lines 19 to 29, 29a to 29u, 363 to 385 may be the polypetides encoded by the nucleic acid molecules indicated in Table I, column 7, lines19 to 29, 29a to 29u, 363 to 385, resp., or may be the polypeptides indicated in Table II, column 7, lines19 to 29, 29a to 29u, 363 to 385 resp.

**[0024.0.0.3]** Further homologs of are described herein below.

**[0025.0.3.3]** In accordance with the invention, a protein or polypeptide has the "activity of an YDL127W, YDR245W, YDR271C, YER173W, YGR101W, YJL072C, YKR057W, YNL135C, YFL013C, YGR104C, YIL150C, YOR350C, YFR042W, YFL019C, b1708, b1829, b2957, b3366, b0828, b3966, b4151, b1827, b0124, b0149, b0161, b0486, b1313, b1343, b1463, b2022, b2414, b2664, b3117, b3256, b3938, b3983, b4054 and/or b4327 protein" if its *de novo* activity, or its increased expression directly or indirectly leads to an increased leucine and/or isoleucine and/or valine level in the organism or a part thereof, preferably in a cell of said organism and the protein has the above mentioned activities of a YDL127W, YDR245W, YDR271C, YER173W, YGR101W, YJL072C, YKR057W, YNL135C, YFL013C, YGR104C, YIL150C, YOR350C, YFR042W, YFL019C, b1708, b1829, b2957, b3366, b0828, b3966, b4151, b1827, b0124, b0149, b0161, b0486, b1313, b1343, b1463, b2022, b2414, b2664, b3117, b3256, b3938, b3983, b4054 and/or b4327 protein. Throughout the specification the activity or preferably the biological activity of such a protein or polypeptide or an nucleic acid molecule or sequence encoding such protein or polypeptide is identical or similar if it still has the biological or enzymatic activity of an YDL127W, YDR245W, YDR271C, YER173W, YGR101W, YJL072C, YKR057W, YNL135C, YFL013C, YGR104C, YIL150C, YOR350C, YFR042W, YFL 019C, b1708, b1829, b2957, b3366, b0828, b3966, b4151, b1827, b0124, b0149, b0161, b0486, b1313, b1343, b1463, b2022, b2414, b2664, b3117, b3256, b3938, b3983, b4054 and/or b4327 protein, or which has at least 10% of the original enzymatic activity, preferably 20%, particularly preferably 30%, most particularly preferably 40% in comparison to an YDL127W, YDR245W, YDR271C, YER173W, YGR101W, YJL072C, YKR057W, YNL135C, YFL013C, YGR104C, YIL150C, YOR350C, YFL019C and/or YFR042W of Saccharomyces cerevisiae and/or b1708, b1829, b2957, b3366, b0828, b3966, b4151, b1827, b0124, b0149, b0161, b0486, b1313, b1343, b1463, b2022, b2414, b2664, b3117, b3256, b3938, b3983, b4054 and/or b4327 protein of E. coli K12.

**[0025.1.0.3]** In one embodiment, the polypeptide of the invention or the polypeptide used in the method of the invention confers said activity, e.g. the increase of the fine chemical in an organism or a part thereof, if it is derived from an organism, which is evolutionary distant to the organism in which it is expressed. For example origin and expressing organism are derived from different families, orders, classes or phylums.

**[0025.2.0.3]** In one embodiment, the polypeptide of the invention or the polypeptide used in the method of the invention confers said activity, e.g. the increase of the fine chemical in an organism or a part thereof, if it is derived from an organism, which is evolutionary close to the organism indicated in Table I, column 4 and is expressed in an organism, which is evolutionary distant to the origin organism. For example origin and expressing organism are derived from different families, orders, classes or phylums whereas origin and the organsim indicated in Table I, column 4 are derived from the same families, orders, classes or phylums.

**[0026.0.0.3] to [0033.0.0.3]: see [0026.0.0.0] to [0033.0.0.0]**

**[0034.0.3.3]** Preferably, the reference, control or wild type differs form the subject of the present invention only in the cellular activity of the polypeptide of the invention, e.g. as result of an increase in the level of the nucleic acid molecule of the present invention or an increase of the specific activity of the polypeptide of the invention, e.g. by or in the expression level or activity of an protein having the activity of an YDL127W, YDR245W, YDR271C, YER173W, YGR101W, YJL072C, YKR057W, YNL135C, YFL013C, YGR104C, YIL150C, YOR350C, YFR042W, YFL019C, b1708, b1829, b2957, b3366, b0828, b3966, b4151, b1827, b0124, b0149, b0161, b0486, b1313, b1343, b1463, b2022, b2414, b2664, b3117, b3256, b3938, b3983, b4054 and/or b4327 or being encoded by a nucleic acid molecule indicated in Table I, column 5, 19 to 29, 29a to 29u, 363 to 385 or its homologs, e.g. as indicated in Table I, column 7, 19 to 29, 29a to 29u, 363 to 385, its biochemical or genetical causes and the increased amount of the fine chemical.

**[0035.0.0.3] to [0044.0.0.3]: see [0035.0.0.0] to [0044.0.0.0]**

**[0045.0.3.3]** In case the activity of the Saccharomyces cerevisiae protein YDL127W or its homologs, e.g. as indicated in Table IA or IB, columns 5 or 7, line 19 or a "G1/S-specific cyclin PCL2 (Cyclin HCS26 homolog) protein", involved in the mitotic cell cycle and cell cycle control is increased, preferably, in one embodiment an increase of the fine chemical, preferably of isoleucine, of 33% or more is conferred.
In case the activity of the Saccharomyces cerevisiae protein YDR245W or its homologs, e.g. as indicated in Table IA or IB, columns 5 or 7, line 29k or a galactosyl-(mannosyl)-transferase, involved in the C-compound and carbohydrate utilization, budding, cell polarity and filament formation, protein modification is increased, preferably, in one embodiment an increase of the fine chemical, preferably of leucine, between 50% and 173% or more is conferred.
In case the activity of the Saccaromyces cerevisiae protein YDR271C or its homologs is increased, preferably, in one embodiment an increase of the fine chemical, preferably of isoleucine, between 30% and 74% or more is conferred.
In case the activity of the Saccharomyces cerevisiae protein YER173w or its homologs, e.g. as indicated in Table IA or IB, columns 5 or 7, line 29b or a checkpoint protein, involved in the activation of the DNA damage and meiotic pachytene checkpoints; DNA recombination and DNA repair, cell cycle checkpoints (checkpoints of morphogenesis, DNA-damage,-replication, mitotic phase and spindle), nucleic acid binding, DNA synthesis and replication is increased, preferably, in one embodiment the increase of the fine chemical between 18% and 187%, preferably of valine btween 18% and 82% or of leucine between 93% and 187% or more is conferred.
In case the activity of the Saccharomyces cerevisiae protein YGR101W or its homologs, e.g. as indicated in Table IA or IB, columns 5 or 7, line 29c or a rhomboid protease, involved in cellular communication/signal transduction mechanism is increased, preferably, in one embodment an increase of the fine chemical, preferably of valine, between 47% and 60% or more is conferred.
In case the activity of the Saccharomyces cerevisiae protein YJL072C or its homologs, e.g. as indicated in Table IA or IB, columns 5 or 7, line 21 or a "subunit of the GINS complex required for chromosomal DNA replication" protein, involved in cell differentiation, late embryonic development, systemic regulation of / interaction with enviroment, directional cell growth (morphogenesis) is increased, preferably, in one embodiment an increase of the fine chemical, preferably of isoleucine, between 31 % and 356% or more is conferred.
In case the activity of the Saccharomyces cerevisiae protein YKR057W or its homologs, e.g. as indicated in Table IA or IB, columns 5 or 7, line 29d or a ribosomal protein, similar to S21A, S26A and/or YS25 ribosomal proteins, involved in ribosome biogenesis, cell differentiation and translation is increased, preferably, in one embodiment an increase of the fine chemical, preferably of valine, between 19% and 100% or more is conferred.
In case the activity of the Saccharomyces cerevisiae protein YNL135C or its homologs, e.g. as indicated in Table IA or IB, columns 5 or 7, line 29m or a peptidylprolyl isomerase, involved in protein folding and stabilization, protein targeting, sorting and translocation, protein synthesis is increased, preferably, in one embodiment an increase of the fine chemical, preferably of leucine, between 45% and 280% or more is conferred.
In case the activity of the Saccharomyces cerevisiae protein YFL013C or its homologs, e.g. as indicated in Table IA or IB, columns 5 or 7, line 29e or a "subunit of the INO80 chromatin remodeling complex" is increased, preferably, in one embodiment an increase of the fine chemical, preferably of valine, between 22% and 58% or more is conferred.
In case the activity of the Saccharomyces cerevisiae protein YGR104C or its homologs, e.g. as indicated in Table IA or IB, columns 5 or 7, line 22 or a "RNA polymerase II suppressor protein SRB5 - yeast and/or suppressor of RNA polymerase B SRB5" involved in transcription activities is increased, preferably, in one embodiment the increase of the fine chemical, preferably of isoleucine, between 33% and 102% or more is conferred.
In case the activity of the Saccharomyces cerevisiae protein YIL150C or its homologs e.g. as indicated in Table IA or IB, columns 5 or 7, line 23 or a chromatin binding protein, required for S-phase (DNA synthesis) initiation or completion, involved in DNA synthesis and replication, mitotic cell cycle and cell cycle control, is increased, preferably, in one embodiment the increase of the fine chemical between 18% and 535%, preferably of valine between 18% and 314% or of isoleucine of 535% is conferred.
In case the activity of the Saccharomyces cerevisiae protein YOR350C or its homologs is increased, preferably, in one embodiment the increase of the fine chemical between 101% and 348%, preferably of leucine between 247% and 348% or of isoleucine between 101% and 178% is conferred.
In case the activity of the Saccharomyces cerevisiae protein YFR042W or its homologs e.g. as indicated in Table IA or IB, columns 5 or 7, line 29n or a "protein required for cell viability in yeast" is increased, preferably, in one embodiment the increase of the fine chemical, preferably of leucine, between 43% and 325% is conferred.
In case the activity of the Saccharomyces cerevisiae protein YFL019C or its homologs e.g. as indicated in Table IA or IB, columns 5 or 7, line 385 or a hypothetical 13.7 kDa protein in PAU5-LPD1 intergenic region is increased, preferably, in one embodiment the increase of the fine chemical, preferably of valine, between 22% and 58% is conferred.
In case the activity of the Escherichia coli K12 protein b1708 or its homologs e.g. as indicated in Table IA or IB, columns 5 or 7, line 25 or a lipoprotein, involved in cell growth / morphogenesis, cytokinesis (cell division) /septum formation and sporulation, is increased, preferably, in one embodiment the increase of the fine chemical between 33% and 333%, preferably of valine between 33% and 149%, of isoleucine between 51% and 290% and of leucine between 60% and 333% is conferred. In case the activity of the Escherichia coli K12 protein b1829 or its homologs is increased, e.g. as indicated in Table IA or IB, columns 5 or 7, line 26 or the activity of a a heat shock protein with protease activity (htpx), involved in stress response, pheromone response, mating-type determination, protein modification, proteolytic degradation is increased preferably, in one embodiment the increase of the fine chemical between 16% and 1480%, preferably of isoleucine between 77% and 1480% and of leucine between 60% and 1200% is conferred.
In case the activity of the Escherichia coli K12 protein b2957 or its homologs e.g. as indicated in Table IA or IB, columns 5 or 7, line 29h or a periplasmic L-asparaginase II, involved in biosynthesis of aspartate, biosynthesis of asparagine, nitrogen and sulfur utilization, amino acid degradation (catabolism), is increased, preferably, in one embodiment the increase of the fine chemical, preferably of valine between 32% and 303% is conferred.
In case the activity of the Escherichia coli K12 protein b3366 or its homologs e.g. as indicated in Table IA or IB, columns 5 or 7, line 29r or a small subunit of a nitrite reductase is increased, preferably, in one embodiment the increase of the fine chemical between 39% and 581%, preferably of isoleucine between 39% and 327% and of leucine between 65% and 581% is conferred.
In case the activity of the Escherichia coli K12 protein b0828 or its homologs e.g. a as indicated in Table IA or IB, columns 5 or 7, line 28 or asparaginase (EC:3.5.1.1) and/or ybiK protein (L-asparagine amidohydrolase), involved in amino acid degradation (catabolism), proteolytic degradation, protein synthesis, degradation of amino acids of the aspartate group, biosynthesis of glycosides, is increased, preferably, in one embodiment the increase of the fine chemical between 20% and 102%, preferably of valine between 20% and 61% and of isoleucine between 60% and 102% is conferred. In case the activity of the Escherichia coli K12 protein b3966 or its homologs e.g. as indicated in Table IA or IB, columns 5 or 7, line 29 or a outer membrane porin, is increased, preferably, in one embodiment the increase of the fine chemical between 59% and 647%, preferably of isoleucine between 79% and 392% and of leucine between 59% and 647% is conferred.
In case the activity of the Escherichia coli K12 protein b4151 or its homologs e.g. as indicated in Table IA or IB, columns 5 or 7, line 29t or a repressor protein with a DNA-binding Winged helix domain (IcIR family), involved in transcriptional control, is increased, preferably, in one embodment the increase of the fine chemical, preferably of leucine between 31% and 46% is conferred.
In case the activity of the Escherichia coli K12 protein b1827 or its homologs e.g. as indicated in Table IA or IB, columns 5 or 7, line 29a or a fumarate reductase, is increased, preferably, in one embodiment the increase of the fine chemical between 24% and 884%, preferably of valine between 24% and 192%, of isoleucine between 81% and 582% and of leucine between 63% and 884% is conferred.
In case the activity of the Escherichia coli K12 protein b0124 or its homologs e.g. as indicated in Table IA or IB, columns 5 or 7, line 363 or a glucose dehydrogenase, is increased, preferably, in one embodiment the increase of the fine chemical, preferably of isoleucine between 43% and 638%, preferably of leucine between 56% and 570%, preferably of valine between 24% and 268% is conferred.
In case the activity of the Escherichia coli K12 protein b0149 or its homologs e.g. as indicated in Table IA or IB, columns 5 or 7, line 366 or a bifunctional penicillin g-binding protein 1b: glycosyl transferase (N-terminal); transpeptidase (C-terminal) is increased, preferably, in one embodiment the increase of the fine chemical, preferably of leucine between 62% and 99% is conferred.
In case the activity of the Escherichia coli K12 protein b0161 or its homologs e.g. as indicated in Table IA or IB, columns 5 or 7, line 367 or a periplasmic serine protease, heat shock protein, preferably, in one embodiment the increase of the fine chemical, preferably of leucine between 71 % and 1140%, preferably of valine between 29% and 399%, preferably of isoleucine between 98% and 1030% is conferred.
In case the activity of the Escherichia coli K12 protein b0486 or its homologs e.g. as indicated in Table IA or IB, columns 5 or 7, line 370 or a putative amino acid/amine transport protein is increased, preferably, in one embodiment the increase of the fine chemical, preferably of valine between 22% and 181% is conferred.
In case the activity of the Escherichia coli K12 protein b1313 or its homologs e.g. as indicated in Table IA or IB, columns 5 or 7, line 371 or a putative dehydrogenase, with NAD(P)-binding and GroES domains is increased, preferably, in one embodiment the increase of the fine chemical, preferably of valine between 22% and 50% is conferred.
In case the activity of the Escherichia coli K12 protein b1343 or its homologs, e.g. as indicated in Table IA or IB, columns 5 or 7, line 372 or an ATP-dependent RNA helicase, is increased, preferably, in one embodiment the increase of the fine chemical, preferably of valine between 21% and 23% is conferred.
In case the activity of the Escherichia coli K12 protein b1463 or its homologs e.g. a as indicated in Table IA or IB, columns 5 or 7, line 373 or N-hydroxyarylamine O-acetyltransferase is increased, preferably, in one embodiment the increase of the fine chemical, preferably of isoleucine between 39% and 119%, preferably of leucine between 66% and 157% is conferred.
In case the activity of the Escherichia coli K12 protein b2022 or its homologs e.g. as indicated in Table IA or IB, columns 5 or 7, line 375 or a bifunctional: histidinol-phosphatase (N-terminal); imidazoleglycerol-phosphate dehydratase (C-terminal); imidazoleglycerolphosphate dehydratase and histidinol-phosphate phosphatase is increased, preferably, in one embodiment the increase of the fine chemical, preferably of valine between 22% and 26% is conferred.
In case the activity of the Escherichia coli K12 protein b2414 or its homologs e.g. as indicated in Table IA or IB, columns 5 or 7, line 376 or a subunit of cysteine synthase A and O-acetylserine sulfhydrolase A, PLP-dependent enzyme is increased, preferably, in one embodiment the increase of the fine chemical, preferably of valine between 35% and 139% is conferred.
In case the activity of the Escherichia coli K12 protein b2664 or its homologs e.g. as indicated in Table IA or IB, columns 5 or 7, line 377 or a putative transcriptional repressor with DNA-binding Winged helix domain (GntR familiy) is increased, preferably, in one embodiment the increase of the fine chemical, preferably of leucine between 67% and 1050%, preferably of isoleucine between 42% and 1170% is conferred.
In case the activity of the Escherichia coli K12 protein b3117 or its homologs e.g. as indicated in Table IA or IB, columns 5 or 7, line 379 or a threonine dehydratase, catabolic, PLP-dependent is increased, preferably, in one embodiment the increase of the fine chemical, preferably of isoleucine between 47% and 656% is conferred.
In case the activity of the Escherichia coli K12 protein b3256 or its homologs e.g. as indicated in Table IA or IB, columns 5 or 7, line 380 or an acetyl CoA carboxylase is increased, preferably, in one embodiment the increase of the fine chemical, preferably of valine between 27% and 36% is conferred.
In case the activity of the Escherichia coli K12 protein b3938 or its homologs e.g. as indicated in Table IA or IB, columns 5 or 7, line 381 or a transcriptional repressor for methionine biosynthesis is increased, preferably, in one embodiment the increase of the fine chemical, preferably of valine between 24% and 42% is conferred.
In case the activity of the Escherichia coli K12 protein b3983 or its homologs e.g. as indicated in Table IA or IB, columns 5 or 7, line 382 or a 50S ribosomal subunit protein L12 is increased, preferably, in one embodiment the increase of the fine chemical, preferably of valine between 24% and 202% is conferred.
In case the activity of the Escherichia coli K12 protein b4054 or its homologs e.g. as indicated in Table IA or IB, columns 5 or 7, line 383 or a tyrosine aminotransferase, tyrosine repressible is increased, preferably, in one embodiment the increase of the fine chemical, preferably of leucine between 126% and 163% is conferred.
In case the activity of the Escherichia coli K12 protein b4327 or its homologs e.g. as indicated in Table IA or IB, columns 5 or 7, line 384 or a HTH-type transcriptional regulator with periplasmic binding protein domain(LysR family) is increased, preferably, in one embodiment the increase of the fine chemical, preferably of isoleucine between 42% and 54% is conferred.

**[0046.0.3.3]** In case the activity of the Saccharomyces cerevisiae protein YDL127W YER173w or its homologs, e.g. a "G1/S-specific cyclin PCL2 (Cyclin HCS26 homolog) protein"a checkpoint protein is increased, preferably an increase of the fine chemical and of tryptophane is conferred.
In case the activity of the Saccharomyces cerevisiae protein YDR271C or its homologs is increased, preferably an increase of the fine chemical and of proline is is conferred. In case the activity of the Saccharomyces cerevisiae protein YER173w or its homologs, e.g. a checkpoint protein is increased, preferably an increase of the fine chemical and of tryptophane is conferred.
In case the activity of the Saccharomyces cerevisiae protein YGR101 W or its homologs, e.g. a rhomboid protease is increased, preferably an increase of the fine chemical and of phenylalanine is conferred.
In case the activity of the Saccharomyces cerevisiae protein YJL072C or its homologs, e.g. a "subunit of the GINS complex required for chromosomal DNA replication" protein is increased, preferably an increase of the fine chemical and of phenylalanine is conferred.
In case the activity of the Saccharomyces cerevisiae protein YKR057W or its homologs, e.g. a ribosomal protein, similar to S21A, S26A and/or YS25 ribosomal proteins is increased, preferably an increase of the fine chemical and of threonine.
In case the activity of the Saccharomyces cerevisiae protein YFL013C or its homologs, e.g. a "subunit of the INO80 chromatin remodeling complex" is increased, preferably an increase of the fine chemical and of 2,3-Dimethyl-5-phytylquinolis conferred.
In case the activity of the Saccharomyces cerevisiae protein YGR104C or its homologs, e.g. a "RNA polymerase II suppressor protein SRB5 - yeast and/or suppressor of RNA polymerase B SRB5"RNA polymerase II suppressor protein (SRB5 - yeast) is increased, preferably an increase of the fine chemical and of glutamic acid is conferred.
In case the activity of the Saccharomyces cerevisiae protein YIL150C or its homologs e.g. a chromatin binding protein, required for S-phase (DNA synthesis) initiation or completion, involved in DNA synthesis and replication, mitotic cell cycle and cell cycle control is increased, preferably an increase of the fine chemical and of threonine is conferred.
In case the activity of the Saccharomyces cerevisiae protein YOR350C or its homologs is increased, preferably an increase of the fine chemical and of tyrosine is conferred. In case the activity of the Saccharomyces cerevisiae protein YFR042W or its homologs e.g. a "protein required for cell viability in yeast" is increased, preferably an increase of the fine chemical and of glutamine is conferred.
In case the activity of the Escherichia coli K12 protein b1708 or its homologs e.g. a lipoprotein, involved in cell growth / morphogenesis, cytokinesis (cell division) /septum formation and sporulation is increased, preferably an increase of the fine chemical and of phenylalanine is conferred.
In case the activity of the Escherichia coli K12 protein b1829 or its homologs is increased, e.g. the activity of a heat shock protein with protease activity (htpx) is increased, preferably an increase of the fine chemical and of threonine is conferred.
In case the activity of the Escherichia coli K12 protein b3966 or its homologs e.g. a outer membrane porin is increased, preferably an increase of the fine chemical and of threonine is conferred.
In case the activity of the Escherichia coli K12 protein b1827 or its homologs e.g. a fumarate reductase is increased, preferably an increase of the fine chemical and of proline is conferred.
In case the activity of the Saccharomyces cerevisiae protein YFL019C or its homologs e.g. as indicated in Table IA or IB, columns 5 or 7, line 385 or a hypothetical 13.7 kDa protein in PAU5-LPD1 intergenic region is increased, preferably, in one embodiment an increase of the fine chemical and further amino acid(s) in free or protein bound form is conferred.
In case the activity of the Escherichia coli K12 protein b0124 or its homologs e.g. as indicated in Table IA or IB, columns 5 or 7, line 363 or a glucose dehydrogenase, is increased, preferably, in one embodiment an increase of the fine chemicals and further amino acid(s) in free or protein bound form is conferred.
In case the activity of the Escherichia coli K12 protein b0149 or its homologs e.g. as indicated in Table IA or IB, columns 5 or 7, line 366 or a bifunctional penicillin g-binding protein 1b: glycosyl transferase (N-terminal); transpeptidase (C-terminal) is increased, preferably, in one embodiment an increase of the fine chemical and further amino acid(s) in free or protein bound form is conferred.
In case the activity of the Escherichia coli K12 protein b0161 or its homologs e.g. as indicated in Table IA or IB, columns 5 or 7, line 367 or a periplasmic serine protease, heat shock protein, preferably, in one embodiment an increase of the fine chemicals and further amino acid(s) in free or protein bound form is conferred In case the activity of the Escherichia coli K12 protein b0486 or its homologs e.g. as indicated in Table IA or IB, columns 5 or 7, line 370 or a putative amino acid/amine transport protein is increased, preferably, in one embodiment an increase of the fine chemical and further amino acid(s) in free or protein bound form is conferred.
In case the activity of the Escherichia coli K12 protein b1313 or its homologs e.g. as indicated in Table IA or IB, columns 5 or 7, line 371 or a putative dehydrogenase, with NAD(P)-binding and GroES domains is increased, preferably, in one embodiment an increase of the fine chemical and further amino acid(s) in free or protein bound form is conferred.
In case the activity of the Escherichia coli K12 protein b1343 or its homologs, e.g. as indicated in Table IA or IB, columns 5 or 7, line 372 or an ATP-dependent RNA helicase, is increased, preferably, in one embodiment an increase of the fine chemical and further amino acid(s) in free or protein bound form is conferred.
In case the activity of the Escherichia coli K12 protein b1463 or its homologs e.g. a as indicated in Table IA or IB, columns 5 or 7, line 373 or N-hydroxyarylamine O-acetyltransferase is increased, preferably, in one embodiment an increase of the fine chemical and further amino acid(s) in free or protein bound form is conferred.
In case the activity of the Escherichia coli K12 protein b2022 or its homologs e.g. as indicated in Table IA or IB, columns 5 or 7, line 375 or a bifunctional: histidinol-phosphatase (N-terminal); imidazoleglycerol-phosphate dehydratase is increased, preferably, in one embodiment an increase of the fine chemical and further amino acid(s) in free or protein bound form is conferred.
In case the activity of the Escherichia coli K12 protein b2414 or its homologs e.g. as indicated in Table IA or IB, columns 5 or 7, line 376 or a subunit of cysteine synthase A and O-acetylserine sulfhydrolase A, PLP-dependent enzyme is increased, preferably, in one embodiment an increase of the fine chemical and further amino acid(s) in free or protein bound form is conferred.
In case the activity of the Escherichia coli K12 protein b2664 or its homologs e.g. as indicated in Table IA or IB, columns 5 or 7, line 377 or a putative transcriptional repressor with DNA-binding Winged helix domain (GntR familiy) is increased, preferably, in one embodiment an increase of the fine chemicals and further amino acid(s) in free or protein bound form is conferred.
In case the activity of the Escherichia coli K12 protein b3117 or its homologs e.g. as indicated in Table IA or IB, columns 5 or 7, line 379 or a threonine dehydratase, catabolic, PLP-dependent is increased, preferably, in one embodiment an increase of the fine chemical and further amino acid(s) in free or protein bound form is conferred.
In case the activity of the Escherichia coli K12 protein b3256 or its homologs e.g. as indicated in Table IA or IB, columns 5 or 7, line 380 or an acetyl CoA carboxylase is increased, preferably, in one embodiment an increase of the fine chemical and further amino acid(s) in free or protein bound form is conferred.
In case the activity of the Escherichia coli K12 protein b3938 or its homologs e.g. as indicated in Table IA or IB, columns 5 or 7, line 381 or a transcriptional repressor for methionine biosynthesis is increased, preferably, in one embodiment an increase of the fine chemical and further amino acid(s) in free or protein bound form is conferred.
In case the activity of the Escherichia coli K12 protein b3983 or its homologs e.g. as indicated in Table IA or IB, columns 5 or 7, line 382 or a 50S ribosomal subunit protein L12 is increased, preferably, in one embodiment an increase of the fine chemical and further amino acid(s) in free or protein bound form is conferred.
In case the activity of the Escherichia coli K12 protein b4054 or its homologs e.g. as indicated in Table IA or IB, columns 5 or 7, line 383 or a tyrosine aminotransferase, tyrosine repressible is increased, preferably, in one embodiment an increase of the fine chemical and further amino acid(s) in free or protein bound form is conferred.
In case the activity of the Escherichia coli K12 protein b4327 or its homologs e.g. as indicated in Table IA or IB, columns 5 or 7, line 384 or a HTH-type transcriptional regulator with periplasmic binding protein domain(LysR family) is increased, preferably, in one embodiment the increase of the fine chemical and further amino acid(s) in free or protein bound form is conferred.

**[0047.0.0.3] see [0047.0.0.0]**

**[0048.0.0.3] see [0048.0.0.0]**

**[0049.0.3.3]** A protein having an activity conferring an increase in the amount or level of the fine chemical preferably has the structure of the polypeptide described herein, in particular of the polypeptides comprising the consensus sequence as indicated in Table IV, column 7, line 19 to 29, 29a to 29u, 363 to 385 or of the polypeptide as shown in Table IIA or IIB, columns 5 or 7, lines 19 to 29, 29a to 29u, 363 to 385 or the functional homologues thereof as described herein, or is encoded by the nucleic acid molecule characterized herein or the nucleic acid molecule according to the invention, for example by the nucleic acid molecule as shown in Table IA or IB, columns 5 or 7, lines 19 to 29, 29a to 29u, 363 to 385 or its herein described functional homologues and has the herein mentioned activity.

**[0050.0.3.3]** For the purposes of the present invention, the term "leucine" and/or "isoleucine" and/or "valine " and "L- leucine" and/or"L- isoleucine" and/or "L-valine" also encompass the corresponding salts, such as, for example, leucine- and/or isoleucineand/or valine- hydrochloride or leucine and/or isoleucine and/or valine sulfate. Preferably the term leucine and/or isoleucine and/or valine is intended to encompass the term L- leucine and/or L- isoleucine and/or L- valine.

**[0051.0.0.3] see [0051.0.0.0]**

**[0052.0.0.3] see [0052.0.0.0]**

**[0053.0.3.3]** In one embodiment, the process of the present invention comprises one or more of the following steps
a) stabilizing a protein conferring the increased expression of a protein encoded by the nucleic acid molecule of the invention or of the polypeptid of the invention, e.g. of a polypeptide as indicated in table II, columns 5 and 7, lines 19 to 29, 29a to 29u, 363 to 385 having an YDL127W, YDR245W, YDR271C, YER173W, YGR101W, YJL072C, YKR057W, YNL135C, YFL013C, YGR104C, YIL150C, YOR350C, YFR042W,YFL019C, b1708, b1829, b2957, b3366, b0828, b3966, b4151, b1827, b0124, b0149, b0161, b0486, b1313, b1343, b1463, b2022, b2414, b2664, b3117, b3256, b3938, b3983, b4054 and/or b4327 protein or its homologs activity having herein-mentioned leucine and/or isoleucine and/or valine increasing activity;
b) stabilizing a mRNA conferring the increased expression of a protein encoded by the nucleic acid molecule of the invention, e.g. of a polypeptide having a YDL127W, YDR245W, YDR271C, YER173W, YGR101W, YJL072C, YKR057W, YNL135C, YFL013C, YGR104C, YIL150C, YOR350C, YFR042W, YFL019C, b1708, b1829, b2957, b3366, b0828, b3966, b4151, b1827, b0124, b0149, b0161, b0486, b1313, b1343, b1463, b2022, b2414, b2664, b3117, b3256, b3938, b3983, b4054 and/or b4327 protein or its homologs activity or of a mRNA encoding the polypeptide of the present invention having herein-mentioned leucine and/or isoleucine and/or valine increasing activity;
c) increasing the specific activity of a protein conferring the increasd expression of a protein encoded by the nucleic acid molecule of the invention or of the polypeptide of the present invention having herein-mentioned leucine and/or isoleucine and/or valine increasing activity, e.g. of a polypeptide having a YDL127W, YDR245W, YDR271C, YER173W, YGR101W, YJL072C, YKR057W, YNL135C, YFL013C, YGR104C, YIL150C, YOR350C, YFR042W, YFL019C, b1708, b1829, b2957, b3366, b0828, b3966, b4151, b1827, b0124, b0149, b0161, b0486, b1313, b1343, b1463, b2022, b2414, b2664, b3117, b3256, b3938, b3983, b4054 and/or b4327 protein or its homologs activity, or decreasing the inhibitiory regulation of the polypeptide of the invention;
d) generating or increasing the expression of an endogenous or artificial transcription factor mediating the expression of a protein conferring the increased expression of a protein encoded by the nucleic acid molecule of the invention or of the polypeptide of the invention having herein-mentioned leucine and/or isoleucine and/or valine increasing activity, e.g. of a polypeptide having the YDL127W, YDR245W, YDR271C, YER173W, YGR101W, YJL072C, YKR057W, YNL135C, YFL013C, YGR104C, YIL150C, YOR350C, YFR042W, YFL019C, b1708, b1829, b2957, b3366, b0828, b3966, b4151, b1827, b0124, b0149, b0161, b0486, b1313, b1343, b1463, b2022, b2414, b2664, b3117, b3256, b3938, b3983, b4054 and/or b4327 protein or its homologs activity;
e) stimulating activity of a protein conferring the increased expression of a protein encoded by the nucleic acid molecule of the present invention or a polypeptide of the present invention having herein-mentioned leucine and/or isoleucine and/or valine increasing activity, e.g. of a polypeptide having the YDL127W, YDR245W, YDR271C, YER173W, YGR101W, YJL072C, YKR057W, YNL135C, YFL013C, YGR104C, YIL150C, YOR350C, YFR042W, YFL019C, b1708, b1829, b2957, b3366, b0828, b3966, b4151, b1827, b0124, b0149, b0161, b0486, b1313, b1343, b1463, b2022, b2414, b2664, b3117, b3256, b3938, b3983, b4054 and/or b4327 protein or its homologs activity, by adding one or more exogenous inducing factors to the organismus or parts thereof;
f) expressing a transgenic gene encoding a protein conferring the increased expression of a polypeptide encoded by the nucleic acid molecule of the present invention or a polypeptide of the present invention, having herein-mentioned leucine and/or isoleucine and/or valine increasing activity, e.g. of a polypeptide having the YDL127W, YDR245W, YDR271C, YER173W, YGR101W, YJL072C, YKR057W, YNL135C, YFL013C, YGR104C, YIL150C, YOR350C, YFR042W, YFL019C, b1708, b1829, b2957, b3366, b0828, b3966, b4151, b1827, b0124, b0149, b0161, b0486, b1313, b1343, b1463, b2022, b2414, b2664, b3117, b3256, b3938, b3983, b4054 and/or b4327 protein or its homologs activity; and/or
g) increasing the copy number of a gene conferring the increased expression of a nucleic acid molecule encoding a polypeptide encoded by the nucleic acid molecule of the invention or the polypeptide of the invention having herein-mentioned leucine and/or isoleucine and/or valine increasing activity, e.g. of a polypeptide having the YDL127W, YDR245W, YDR271C, YER173W, YGR101W, YJL072C, YKR057W, YNL135C, YFL013C, YGR104C, YIL150C, YOR350C, YFR042W, YFL019C, b1708, b1829, b2957, b3366, b0828, b3966, b4151, b1827, b0124, b0149, b0161, b0486, b1313, b1343, b1463, b2022, b2414, b2664, b3117, b3256, b3938, b3983, b4054 and/or b4327 protein or its homologs activity.
h) Increasing the expression of the endogenous gene encoding the polypeptide of the invention, e.g. a polypeptide having the YDL127W, YDR245W, YDR271C, YER173W, YGR101W, YJL072C, YKR057W, YNL135C, YFL013C, YGR104C, YIL150C, YOR350C, YFR042W, YFL019C, b1708, b1829, b2957, b3366, b0828, b3966, b4151, b1827, b0124, b0149, b0161, b0486, b1313, b1343, b1463, b2022, b2414, b2664, b3117, b3256, b3938, b3983, b4054 and/or b4327 protein or its homologs activity, by adding positive expression or removing negative expression elements, e.g. homologous recombination can be used to either introduce positive regulatory elements like for plants the 35S enhancer into the promoter or to remove repressor elements form regulatory regions. Further gene conversion methods can be used to disrupt repressor elements or to enhance to acitivty of positive elements. Positive elements can be randomly introduced in plants by T-DNA or transposon mutagenesis and lines can be identified in which the positive elements have be integrated near to a gene of the invention, the expression of which is thereby enhanced. and/or
i) Modulating growth conditions of an organism in such a manner, that the expression or activity of the gene encoding the protein of the invention or the protein itself is enhanced for example microorganisms or plants can be grown for example under a higher temperature regime leading to an enhanced expression of heat shock proteins, which can lead an enhanced the fine chemical production.
j) selecting of organisms with expecially high activity of the proteins of the invention from natural or from mutagenized resources and breeding them into the target organisms, eg the elite crops.

**[0054.0.2.3]** Preferably, said mRNA is the nucleic acid molecule of the present invention and/or the protein conferring the increased expression of a protein encoded by the nucleic acid molecule of the present invention or the polypeptide having the herein mentioned activity is the polypeptide of the present invention, e.g. conferring the increase of leucine and/or isoleucine and/or valine after increasing the expression or activity of the encoded polypeptide or having the activity of a polypeptide having an YDL127W, YDR245W, YDR271C, YER173W, YGR101W, YJL072C, YKR057W, YNL135C, YFL013C, YGR104C, YIL150C, YOR350C, YFR042W, YFL019C, b1708, b1829, b2957, b3366, b0828, b3966, b4151, b1827, b0124, b0149, b0161, b0486, b1313, b1343, b1463, b2022, b2414, b2664, b3117, b3256, b3938, b3983, b4054 and/or b4327 protein or its homologs activity as indicated in table II, columns 5 and 7, lines 19 to 29, 29a to 29u, 363 to 385.

**[0055.0.0.3] to [0064.0.0.3] see [0055.0.0.0] to [0064.0.0.0]**

**[0065.0.3.3]** The activation of an endogenous polypeptide having above-mentioned activity, e.g.having the activity of a YDL127W, YDR245W, YDR271C, YER173W, YGR101W, YJL072C, YKR057W, YNL135C, YFL013C, YGR104C, YIL150C, YOR350C, YFR042W, YFL019C, b1708, b1829, b2957, b3366, b0828, b3966, b4151, b1827, b0124, b0149, b0161, b0486, b1313, b1343, b1463, b2022, b2414, b2664, b3117, b3256, b3938, b3983, b4054 and/or b4327 protein or of the polypeptide of the invention, e.g. conferring the increase of leucine and/or isoleucine and/or valine after increase of expression or activity can also be increased by introducing a synthetic transcription factor, which binds close to the coding region of the YDL127W, YDR245W, YDR271C, YER173W, YGR101W, YJL072C, YKR057W, YNL135C, YFL013C, YGR104C, YIL150C, YOR350C, YFR042W, YFL019C, b1708, b1829, b2957, b3366, b0828, b3966, b4151, b1827, b0124, b0149, b0161, b0486, b1313, b1343, b1463, b2022, b2414, b2664, b3117, b3256, b3938, b3983, b4054 and/or b4327 protein encoding gene and activates its transcription. A chimeric zinc finger protein can be construed, which comprises a specific DNA-binding domain and an activation domain as e.g. the VP16 domain of Herpes Simplex virus. The specific binding domain can bind to the regulatory region of the YDL127W, YDR245W, YDR271C, YER173W, YGR101W, YJL072C, YKR057W, YNL135C, YFL013C, YGR104C, YIL150C, YOR350C, YFR042W, YFL019C, b1708, b1829, b2957, b3366, b0828, b3966, b4151, b1827, b0124, b0149, b0161, b0486, b1313, b1343, b1463, b2022, b2414, b2664, b3117, b3256, b3938, b3983, b4054 and/or b4327 protein encoding gene. The expression of the chimeric transcription factor in a organism, in particular in a plant, leads to a specific expression of YDL127W, YDR245W, YDR271C, YER173W, YGR101W, YJL072C, YKR057W, YNL135C, YFL013C, YGR104C, YIL150C, YOR350C, YFR042W, YFL019C, b1708, b1829, b2957, b3366, b0828, b3966, b4151, b1827, b0124, b0149, b0161, b0486, b1313, b1343, b1463, b2022, b2414, b2664, b3117, b3256, b3938, b3983, b4054 and/or b4327 protein, see e.g. in WO01/52620, Oriz, Proc. Natl. Acad. Sci. USA, 2002, Vol. 99, 13290 or Guan, Proc. Natl. Acad. Sci. USA, 2002, Vol. 99, 13296.

**[0066.0.0.3] to [0069.0.0.3]: see [0066.0.0.0] to [0069.0.0.0]**

**[0070.0.3.3]** Owing to the introduction of a gene or a plurality of genes conferring the expression of the nucleic acid molecule of the invention or the polypeptide of the invention, for example the nucleic acid construct mentioned below, or encoding the YDL127W, YDR245W, YDR271C, YER173W, YGR101W, YJL072C, YKR057W, YNL135C, YFL013C, YGR104C, YIL150C, YOR350C, YFR042W, YFL019C, b1708, b1829, b2957, b3366, b0828, b3966, b4151, b1827, b0124, b0149, b0161, b0486, b1313, b1343, b1463, b2022, b2414, b2664, b3117, b3256, b3938, b3983, b4054 and/or b4327 protein into an organism alone or in combination with other genes, it is possible not only to increase the biosynthetic flux towards the end product, but also to increase, modify or create *de novo* an advantageous, preferably novel metabolites composition in the organism, e.g. an advantageous amino acid composition comprising a higher content of (from a viewpoint of nutrional physiology limited) amino acids, like tryptophane, methionine, lysine and/or threonine.

**[0071.0.0.3] see [0071.0.0.0]**

**[0072.0.3.3]** By influencing the metabolism thus, it is possible to produce, in the process according to the invention, further advantageous compounds. Examples of such compounds are, in addition to leucine and/or isoleucine and/or valine 2-Acetolactate, 2-3-Dihydroxyisovalerate, 2-Oxoisovalerate, 3-Hydroxyisobutyrate, 3-Hydroxy-Isobutyryl-CoA, Methylacrylyl-CoA, Isobutyryl-CoA, 2-Aceto-2-hydroxybutyrate, 2:3-Di-OH-3-methylvalerate, 2-Oxo-3-methylvalerate, 2-Methylacetoacetyl-CoA, 2-Methyl-3-hydroxybutyryl-CoA, Tiglyl-CoA, 2 Methylbutyryl-CoA, 22-Isopropylmalate, 3-Isopropylmalate, Oxoleucine, Isovaleryl-CoA, 3-Methylcrotonyl-CoA and/or 3-Methylglutaconyl-CoA.

**[0073.0.3.3]** Accordingly, in one embodiment, the process according to the invention relates to a process which comprises:
e) providing a non-human organism, preferably a microorganism, a non-human animal, a plant or animal cell, a plant or animal tissue or a plant;
f) increasing the YDL127W, YDR245W, YDR271C, YER173W, YGR101W, YJL072C, YKR057W, YNL135C, YFL013C, YGR104C, YIL150C, YOR350C, YFR042W, YFL019C, b1708, b1829, b2957, b3366, b0828, b3966, b4151, b1827, b0124, b0149, b0161, b0486, b1313, b1343, b1463, b2022, b2414, b2664, b3117, b3256, b3938, b3983, b4054 and/or b4327 protein activity or of a polypeptide being encoded by the nucleic acid molecule of the present invention and described below, e.g. conferring an increase of the fine chemical in the organism, preferably in the microorganism, the non-human animal, the plant or animal cell, the plant or animal tissue or the plant,
g) growing the organism, preferably the microorganism, the non-human animal, the plant or animal cell, the plant or animal tissue or the plant under conditions which permit the production of the fine chemical in the organism, preferably the microorganism, the plant cell, the plant tissue or the plant; and
h) if desired, revovering, optionally isolating, the free and/or bound the fine chemical and , optionally further free and/or bound amino acids synthetized by the organism, the microorganism, the non-human animal, the plant or animal cell, the plant or animal tissue or the plant.

**[0074.0.0.3] to [0084.0.0.3]: see [0074.0.0.0] to [0084.0.0.0]**

**[0085.0.3.3]** With regard to the nucleic acid sequence as depicted a nucleic acid construct which contains a nucleic acid sequence mentioned herein or an organism (= transgenic organism) which is transformed with said nucleic acid sequence or said nucleic acid construct, "transgene" means all those constructs which have been brought about by genetic manipulation methods,preferably in which either
a) the nucleic acid sequence as depicted in Table IA or IB, columns 5 and 7, lines 19 to 29, 29a to 29u, 363 to 385 or a derivative thereof, or
b) a genetic regulatory element, for example a promoter, which is functionally linked to the nucleic acid sequence as depicted in Table IA or IB, columns 5 and 7, lines 19 to 29, 29a to 29u, 363 to 385 or a derivative thereof, or
c) (a) and (b)
is/are not present in its/their natural genetic environment or has/have been modified by means of genetic manipulation methods, it being possible for the modification to be, by way of example, a substitution, addition, deletion, inversion or insertion of one or more nucleotide. "Natural genetic environment" means the natural chromosomal locus in the organism of origin or the presence in a genomic library. In the case of a genomic library, the natural, genetic environment of the nucleic acid sequence is preferably at least partially still preserved. The environment flanks the nucleic acid sequence at least on one side and has a sequence length of at least 50 bp, preferably at least 500 bp, particularly preferably at least 1000 bp, very particularly preferably at least 5000 bp.

**[0086.0.0.3]: see [0086.0.0.0]**

**[0087.0.0.3]: see [0087.0.0.0]**

**[0088.0.3.3]** In an advantageous embodiment of the invention, the organism takes the form of a plant whose amino acid content is modified advantageously owing to the nucleic acid molecule of the present invention expressed. This is important for plant breeders since, for example, the nutritional value of plants for monogastric animals is limited by a few essential amino acids such as lysine, threonine or methionine or tryptophane. After the YDL127W, YDR245W, YDR271C, YER173W, YGR101W, YJL072C, YKR057W, YNL135C, YFL013C, YGR104C, YIL150C, YOR350C, YFR042W,YFL019C, b1708, b1829, b2957, b3366, b0828, b3966, b4151, b1827, b0124, b0149, b0161, b0486, b1313, b1343, b1463, b2022, b2414, b2664, b3117, b3256, b3938, b3983, b4054 and/or b4327 protein activity has been increased or generated, or after the expression of nucleic acid molecule or polypeptide according to the invention has been generated or increased, the transgenic plant generated thus is grown on or in a nutrient medium or else in the soil and subsequently harvested.

**[0089.0.0.3] to [0097.0.0.3]: see [0089.0.0.0] to [0097.0.0.0]**

**[0098.0.3.3]** In a preferred embodiment, the fine chemical (leucine and/or isoleucine and/or valine) is produced in accordance with the invention and, if desired, is isolated. The production of further amino acids such as methionine, lysine and/or threonine mixtures of amino acid by the process according to the invention is advantageous.

**[0099.0.0.3] to [0102.0.0.3]: see [0099.0.0.0] to [0102.0.0.0]**

**[0103.0.3.3]** In a preferred embodiment, the present invention relates to a process for the production of the fine chemical comprising or generating in an organism or a part thereof the expression of at least one nucleic acid molecule comprising a nucleic acid molecule selected from the group consisting of:
a) nucleic acid molecule encoding, preferably at least the mature form, of the polypeptide shown in Table IIA or IIB, columns 5 and 7, lines 19 to 29, 29a to 29u, 363 to 385 or a fragment thereof, which confers an increase in the amount of the fine chemical in an organism or a part thereof;
b) nucleic acid molecule comprising, preferably at least the mature form, of the nucleic acid molecule shown in Table IA or IB, columns 5 and 7, lines 19 to 29, 29a to 29u, 363 to 385
c) nucleic acid molecule whose sequence can be deduced from a polypeptide sequence encoded by a nucleic acid molecule of (a) or (b) as result of the degeneracy of the genetic code and conferring an increase in the amount of the fine chemical in an organism or a part thereof;
d) nucleic acid molecule encoding a polypeptide which has at least 50% identity with the amino acid sequence of the polypeptide encoded by the nucleic acid molecule of (a) to (c) and conferring an increase in the amount of the fine chemical in an organism or a part thereof;
e) nucleic acid molecule which hybidizes with a nucleic acid molecule of (a) to (c) under under stringent hybridisation conditions and conferring an increase in the amount of the fine chemical in an organism or a part thereof;
f) nucleic acid molecule encoding a polypeptide, the polypeptide being derived by substituting, deleting and/or adding one or more amino acids of the amino acid sequence of the polypeptide encoded by the nucleic acid molecules (a) to (d), preferably to (a) to (c) and conferring an increase in the amount of the fine chemical in an organism or a part thereof;
g) nucleic acid molecule encoding a fragment or an epitope of a polypeptide which is encoded by one of the nucleic acid molecules of (a) to (e), preferably to (a) to (c) and and conferring an increase in the amount of the fine chemical in an organism or a part thereof;
h) nucleic acid molecule comprising a nucleic acid molecule which is obtained by amplifying nucleic acid molecules from a cDNA library or a genomic library using the primers in Table III, column 7, lines 19 to 29, 29a to 29u, 363 to 385 and conferring an increase in the amount of the fine chemical in an organism or a part thereof;
i) nucleic acid molecule encoding a polypeptide which is isolated, e.g. from an expression library, with the aid of monoclonal antibodies against a polypeptide encoded by one of the nucleic acid molecules of (a) to (h), preferably to (a) to (c), and and conferring an increase in the amount of the fine chemical in an organism or a part thereof;
j) nucleic acid molecule which encodes a polypeptide comprising the consensus sequence shown in Table IV, column7, lines 19 to 29, 29a to 29u, 363 to 385 and conferring an increase in the amount of the fine chemical in an organism or a part thereof;
k) nucleic acid molecule comprising one or more of the nucleic acid molecule encoding the amino acid sequence of a polypeptide encoding a domain of the polypeptide shown in Table IA or IB, columns 5 and 7, lines 19 to 29, 29a to 29u, 363 to 385 and conferring an increase in the amount of the fine chemical in an organism or a part thereof; and
l) nucleic acid molecule which is obtainable by screening a suitable library under stringent conditions with a probe comprising one of the sequences of the nucleic acid molecule of (a) to (k), preferably to (a) to (c), or with a fragment of at least 15 nt, preferably 20 nt, 30 nt, 50 nt, 100 nt, 200 nt or 500 nt of the nucleic acid molecule characterized in (a) to (k), preferably to (a) to (c), and conferring an increase in the amount of the fine chemical in an organism or a part thereof;
or which comprises a sequence which is complementary thereto.

**[0104.0.3.3]** In one embodiment, the nucleic acid molecule used in the process distinguishes over the sequence depicted in Table IA or IB, columns 5 and 7, lines 19 to 29, 29a to 29u, 363 to 385 preferably over the sequences as shown in Table IA, columns 5 or 7, lines 19 to 29, 29a to 29u, 363 to 385 by one or more nucleotides or does not consist of the sequence shown in Table IA or IB, columns 5 and 7, lines 19 to 29, 29a to 29u, 363 to 385 preferably not of the sequences as shown in Table IA, columns 5 or 7, lines 19 to 29, 29a to 29u, 363 to 385. In one embodiment, the nucleic acid molecule of the present invention is less than 100%, 99,999%, 99,99%, 99,9% or 99% identical to the sequence shown in Table IA or IB, lines 19 to 29, 29a to 29u, 363 to 385, preferably to the sequences as shown in Table IA, columns 5 or 7, lines 19 to 29, 29a to 29u, 363 to 385 . In another embodiment, the nucleic acid molecule does not encode a polypeptide of the sequence shown in Table IIA or IIB, lines 19 to 29, 29a to 29u, 363 to 385, preferably of sequences as shown in Table IA, columns 5 or 7, lines 19 to 29, 29a to 29u, 363 to 385.

**[0105.0.0.3] to [0107.0.0.3]: see [0105.0.0.0] to [0107.0.0.0]**

**[0108.0.3.3]** Nucleic acid molecules with the sequence shown in Table IA or IB, lines 19 to 29, 29a to 29u, 363 to 385 , nucleic acid molecules which are derived from the amino acid sequences shown in Table IIA or IIB, , columns 5 or 7, lines 19 to 29, 29a to 29u, 363 to 385 or from polypeptides comprising the consensus sequence shown in Table IV, , columns 7, lines 19 to 29, 29a to 29u, 363 to 385, or their derivatives or homologues encoding polypeptides with the enzymatic or biological activity of an YDL127W, YDR245W, YDR271C, YER173W, YGR101W, YJL072C, YKR057W, YNL135C, YFL013C, YGR104C, YIL150C, YOR350C, YFR042W, YFL019C, b1708, b1829, b2957, b3366, b0828, b3966, b4151, b1827, b0124, b0149, b0161, b0486, b1313, b1343, b1463, b2022, b2414, b2664, b3117, b3256, b3938, b3983, b4054 and/or b4327 protein or conferring a leucine and/or isoleucine and/or valine increase after increasing its expression or activity are advantageously increased in the process according to the invention.

**[0109.0.0.3] see [0109.0.0.0]**

**[0110.0.0.3]** Nucleic acid molecules, which are advantageous for the process according to the invention and which encode polypeptides with YDL127W, YDR245W, YDR271C, YER173W, YGR101W, YJL072C, YKR057W, YNL135C, YFL013C, YGR104C, YIL150C, YOR350C, YFR042W, YFL019C, b1708, b1829, b2957, b3366, b0828, b3966, b4151, b1827, b0124, b0149, b0161, b0486, b1313, b1343, b1463, b2022, b2414, b2664, b3117, b3256, b3938, b3983, b4054 and/or b4327 protein activity can be determined from generally accessible databases.

**[0111.0.0.3] see [0111.0.0.0]**

**[0112.0.3.3]** The nucleic acid molecules used in the process according to the invention take the form of isolated nucleic acid sequences, which encode polypeptides with YDL127W, YDR245W, YDR271C, YER173W, YGR101W, YJL072C, YKR057W, YNL135C, YFL013C, YGR104C, YIL150C, YOR350C, YFR042W, YFL019C, b1708, b1829, b2957, b3366, b0828, b3966, b4151, b1827, b0124, b0149, b0161, b0486, b1313, b1343, b1463, b2022, b2414, b2664, b3117, b3256, b3938, b3983, b4054 and/or b4327 protein activity and conferring a leucine and/or isoleucine and/or valine increase.

**[0113.0.0.3] to [0120.0.0.3]: see [0113.0.0.0] to [0120.0.0.0]**

**[0121.0.3.3]** However, it is also possible to use artificial sequences, which differ in one or more bases from the nucleic acid sequences found in organisms, or in one or more amino acid molecules from polypeptide sequences found in organisms, in particular from the polypeptide sequences shown in Table IIA or IIB, columns 5 or 7, lines 19 to 29, 29a to 29u, 363 to 385 or the functional homologues thereof as described herein, preferably conferring above-mentioned activity, i.e. conferring a leucine and/or isoleucine and/or valine increase after increasing its activity, e.g. having the activity of an YDL127W, YDR245W, YDR271C, YER173W, YGR101W, YJL072C, YKR057W, YNL135C, YFL013C, YGR104C, YIL150C, YOR350C, YFR042W, YFL019C, b1708, b1829, b2957, b3366, b0828, b3966, b4151, b1827, b0124, b0149, b0161, b0486, b1313, b1343, b1463, b2022, b2414, b2664, b3117, b3256, b3938, b3983, b4054 and/or b4327 protein.

**[0122.0.0.3] to [127.0.0.3]: see [0122.0.0.0] to [0127.0.0.0]**

[0128.0.3.3] Synthetic oligonucleotide primers for the amplification, e.g. as shown in Table III, , column 7, lines 19 to 29, 29a to 29u, 363 to 385 by means of polymerase chain reaction can be generated on the basis of a sequence shown herein, for example the sequence shown in Table IA or IB, , columns 5 or 7, lines 19 to 29, 29a to 29u, 363 to 385 or the sequences derived from Table IIA or IIB, lines 19 to 29, 29a to 29u, 363 to 385.

**[0129.0.3.3]** Moreover, it is possible to identify conserved regions from various organisms by carrying out protein sequence alignments with the polypeptide used in the process of the invention, in particular with sequences of the polypeptide of the invention, from which conserved regions, and in turn, degenerate primers can be derived. Conserved region for the polypeptide of the invention are indicated in the alignments shown in the figures. Conserved regions are those, which show a very little variation in the amino acid in one particular position of several homologs from different origin. The consenus sequence shown in Table IV, column 7, lines 19 to 29, 29a to 29u, 363 to 385 is derived from said aligments.

**[0130.0.3.3]** Degenerated primers can then be utilized by PCR for the amplification of fragments of novel proteins having above-mentioned activity, e.g. conferring the increase of leucine and/or isoleucine and/or valine after increasing the expression or activity or having an YDL127W, YDR245W, YDR271C, YER173W, YGR101W, YJL072C, YKR057W, YNL135C, YFL013C, YGR104C, YIL150C, YOR350C, YFR042W, YFL019C, b1708, b1829, b2957, b3366, b0828, b3966, b4151, b1827, b0124, b0149, b0161, b0486, b1313, b1343, b1463, b2022, b2414, b2664, b3117, b3256, b3938, b3983, b4054 and/or b4327 activity or further functional homologs of the polypeptide of the invention from other organisms. **[0131.0.0.3] to [0138.0.0.3]: see [0131.0.0.0] to [0138.0.0.0]**

**[0139.0.3.3]** Polypeptides having above-mentioned activity, i.e. conferring the fine chemical increase, derived from other organisms, can be encoded by other DNA sequences which hybridize to the sequences shown in Table IA or IB, columns 5 or 7, lines 19 to 29, 29a to 29u, 363 to 385 under relaxed hybridization conditions and which code on expression for peptides having the leucine and/or isoleucine and/or valine increasing activity.

**[0140.0.0.3] to [0146.0.0.3]: see [0140.0.0.0] to [0146.0.0.0]**

**[0147.0.3.3]** Further, the nucleic acid molecule of the invention comprises a nucleic acid molecule, which is a complement of one of the nucleotide sequences of above mentioned nucleic acid molecules or a portion thereof. A nucleic acid molecule which is complementary to one of the nucleotide sequences shown in Table IA or IB, columns 5 or 7, lines 19 to 29, 29a to 29u, 363 to 385, preferably in Table I B, column 7, lines 19 to 29, 29a to 29u, 363 to 385 is one which is sufficiently complementary to one of the nucleotide sequences shown in Table IA or IB, lines 19 to 29, 29a to 29u, 363 to 385 such that it can hybridize to one of the nucleotide sequences shown in Table IA or IB, lines 19 to 29, 29a to 29u, 363 to 385 thereby forming a stable duplex. Preferably, the hybridisation is performed under stringent hybrization conditions. However, a complement of one of the herein disclosed sequences is preferably a sequence complement thereto according to the base pairing of nucleic acid molecules well known to the skilled person. For example, the bases A and G undergo base pairing with the bases T and U or C, resp. and visa versa. Modifications of the bases can influence the base-pairing partner **[0148.0.3.3]** The nucleic acid molecule of the invention comprises a nucleotide sequence which is at least about 30%, 35%, 40% or 45%, preferably at least about 50%, 55%, 60% or 65%, more preferably at least about 70%, 80%, or 90%, and even more preferably at least about 95%, 97%, 98%, 99% or more homologous to a nucleotide sequence shown in Table IA or IB, columns 5 or 7, lines 19 to 29, 29a to 29u, 363 to 385, , , preferably in Table I B, column 7, lines 19 to 29, 29a to 29u, 363 to 385, or a portion thereof and preferably has above mentioned activity, in particular having a leucine and/or isoleucine and/or valine increasing activity after increasing the acitivity or an activity of an YDL127W, YDR245W, YDR271C, YER173W, YGR101W, YJL072C, YKR057W, YNL135C, YFL013C, YGR104C, YIL150C, YOR350C, YFR042W, YFL019C, b1708, b1829, b2957, b3366, b0828, b3966, b4151, b1827, b0124, b0149, b0161, b0486, b1313, b1343, b1463, b2022, b2414, b2664, b3117, b3256, b3938, b3983, b4054 and/or b4327 gene product.

**[0149.0.3.3]** The nucleic acid molecule of the invention comprises a nucleotide sequence which hybridizes, preferably hybridizes under stringent conditions as defined herein, to one of the nucleotide sequences shown in Table IA or IB, columns 5 or 7, lines 19 to 29, 29a to 29u, 363 to 385, preferably in Table I B, column 7, lines 19 to 29, 29a to 29u, 363 to 385 or a portion thereof and encodes a protein having above-mentioned activity, e.g. conferring a leucine and/or isoleucine and/or valine increase, and optionally, the activity of YDL127W, YDR245W, YDR271C, YER173W, YGR101W, YJL072C, YKR057W, YNL135C, YFL013C, YGR104C, YIL150C, YOR350C, YFR042W, YFL019C, b1708, b1829, b2957, b3366, b0828, b3966, b4151, b1827, b0124, b0149, b0161, b0486, b1313, b1343, b1463, b2022, b2414, b2664, b3117, b3256, b3938, b3983, b4054 and/or b4327.

**[0150.0.3.3]** Moreover, the nucleic acid molecule of the invention can comprise only a portion of the coding region of one of the sequences in Table IA or IB, columns 5 or 7, lines 19 to 29, 29a to 29u, 363 to 385, preferably in Table I B, column 7, lines 19 to 29, 29a to 29u, 363 to 385 for example a fragment which can be used as a probe or primer or a fragment encoding a biologically active portion of the polypeptide of the present invention or of a polypeptide used in the process of the present invention, i.e. having above-mentioned activity, e.g.conferring an increase of leucine and/or isoleucine and/or valine if its activity is increased. The nucleotide sequences determined from the cloning of the present protein-according-to-the-invention-encoding gene allows for the generation of probes and primers designed for use in identifying and/or cloning its homologues in other cell types and organisms. The probe/primer typically comprises substantially purified oligonucleotide. The oligonucleotide typically comprises a region of nucleotide sequence that hybridizes under stringent conditions to at least about 12, 15 preferably about 20 or 25, more preferably about 40, 50 or 75 consecutive nucleotides of a sense strand of one of the sequences set forth, e.g., in Table IA or IB, columns 5 or 7, lines 19 to 29, 29a to 29u, 363 to 385 , an anti-sense sequence of one of the sequences, e.g., set forth in Table IA or IB, lines 19 to 29, 29a to 29u, 363 to 385 , or naturally occurring mutants thereof. Primers based on a nucleotide of invention can be used in PCR reactions to clone homologues of the polypeptide of the invention or of the polypeptide used in the process of the invention, e.g. as the primers described in the examples of the present invention, e.g. as shown in the examples. A PCR with the primers shown in Table III, column 7, lines 19 to 29, 29a to 29u, 363 to 385 will result in a fragment of YDL127W, YDR245W, YDR271C, YER173W, YGR101W, YJL072C, YKR057W, YNL135C, YFL013C, YGR104C, YIL150C, YOR350C, YFR042W, YFL019C, b1708, b1829, b2957, b3366, b0828, b3966, b4151, b1827, b0124, b0149, b0161, b0486, b1313, b1343, b1463, b2022, b2414, b2664, b3117, b3256, b3938, b3983, b4054 and/or b4327 gene product.

**[0151.0.0.3] see [0151.0.0.0]**

**[0152.0.3.3]** The nucleic acid molecule of the invention encodes a polypeptide or portion thereof which includes an amino acid sequence which is sufficiently homologous to the amino acid sequence of Table IIA or IIB, columns 5 or 7, lines 19 to 29, 29a to 29u, 363 to 385 such that the protein or portion thereof maintains the ability to participate in the fine chemical production, in particular a leucine and/or isoleucine and/or valine increasing the activity as mentioned above or as described in the examples in plants or microorganisms is comprised.

**[0153.0.3.3]** As used herein, the language "sufficiently homologous" refers to proteins or portions thereof which have amino acid sequences which include a minimum number of identical or equivalent amino acid residues (e.g., an amino acid residue which has a similar side chain as an amino acid residue in one of the sequences of the polypeptide of the present invention) to an amino acid sequence shown in Table IIA or IIB, columns 5 or 7, lines 19 to 29, 29a to 29u, 363 to 385 such that the protein or portion thereof is able to participate in the increase of the fine chemical production. For examples having an YDL127W, YDR245W, YDR271C, YER173W, YGR101W, YJL072C, YKR057W, YNL135C, YFL013C, YGR104C, YIL150C, YOR350C, YFR042W, YFL019C, b1708, b1829, b2957, b3366, b0828, b3966, b4151, b1827, b0124, b0149, b0161, b0486, b1313, b1343, b1463, b2022, b2414, b2664, b3117, b3256, b3938, b3983, b4054 and/or b4327 activity are described herein.

**[0154.0.3.3]** In one embodiment, the nucleic acid molecule of the present invention comprises a nucleic acid that encodes a portion of the protein of the present invention. The protein is at least about 30%, 35%, 40%, 45% or 50%, preferably at least about 55%, 60%, 65% or 70%, and more preferably at least about 75%, 80%, 85%, 90%, 91%, 92%, 93% or 94% and most preferably at least about 95%, 97%, 98%, 99% or more homologous to an entire amino acid sequence of Table IIA or IIB, columns 5 or 7, lines 19 to 29, 29a to 29u, 363 to 385 and having above-mentioned activity, e.g.conferring preferably the increase of the fine chemical.

**[0155.0.0.3] and [0156.0.0.3] : see [0155.0.0.0] and [0156.0.0.0]**

**[0157.0.3.3]** The invention further relates to nucleic acid molecules that differ from one of the nucleotide sequences shown in Table IA or IB, columns 5 or 7, lines 19 to 29, 29a to 29u, 363 to 385 (and portions thereof) due to degeneracy of the genetic code and thus encode a polypeptide of the present invention, in particular a polypeptide having above mentioned activity, e.g. conferring an increase in the fine chemical in a organism, e.g. as that polypeptides encoded by the sequence shown in Table IIA or IIB, columns 5 or 7, lines 19 to 29, 29a to 29u, 363 to 385 or the functional homologues. However, in a preferred embodiment, the nucleic acid molecule of the present invention does not consist of a sequence as indicated in Table I, columns 5 or 7, lines 19 to 29, 29a to 29u, 363 to 385, preferably as indicated in Table I A, columns 5 or 7, lines 19 to 29, 29a to 29u, 363 to 385. Preferably the nucleic acid molecule of the invention is a functional homologue or identical to a nucleic acid molecule indicated in Table I B, column 7, lines 19 to 29, 29a to 29u, 363 to 385 .

**[0158.0.0.3] to [0160.0.0.3]: see [0158.0.0.0] to [0160.0.0.0]**

**[0161.0.3.3]** Accordingly, in another embodiment, a nucleic acid molecule of the invention is at least 15, 20, 25 or 30 nucleotides in length. Preferably, it hybridizes under stringent conditions to a nucleic acid molecule comprising a nucleotide sequence of the nucleic acid molecule of the present invention or used in the process of the present invention, e.g. comprising the sequence shown in Table IA or IB, columns 5 or 7, lines 19 to 29, 29a to 29u, 363 to 385 . The nucleic acid molecule is preferably at least 20, 30, 50, 100, 250 or more nucleotides in length.

**[0162.0.0.3]: see [0162.0.0.0]**

**[0163.0.3.3]** Preferably, nucleic acid molecule of the invention that hybridizes under stringent conditions to a sequence of Table IA or IB, columns 5 or 7, lines 19 to 29, 29a to 29u, 363 to 385 corresponds to a naturally-occurring nucleic acid molecule of the invention. As used herein, a "naturally-occurring" nucleic acid molecule refers to an RNA or DNA molecule having a nucleotide sequence that occurs in nature (e.g., encodes a natural protein). Preferably, the nucleic acid molecule encodes a natural protein having above-mentioned activity, e.g. conferring the fine chemical increase after increasing the expression or activity thereof or the activity of a protein of the invention or used in the process of the invention.

**[0164.0.0.3]: see [0164.0.0.0]**

**[0165.0.3.3]** For example, nucleotide substitutions leading to amino acid substitutions at "non-essential" amino acid residues can be made in a sequence of the nucleic acid molecule of the invention or used in the process of the invention, e.g. in Table IA or IB, columns 5 or 7, lines 19 to 29, 29a to 29u, 363 to 385

**[0166.0.0.3] and [0167.0.0.3]: see [0166.0.0.0] and [0167.0.0.0]**

**[0168.0.3.3]** Accordingly, the invention relates to nucleic acid molecules encoding a polypeptide having above-mentioned activity, e.g. conferring an increase in the the fine chemical in an organisms or parts thereof that contain changes in amino acid residues that are not essential for said activity. Such polypeptides differ in amino acid sequence from a sequence contained in Table IIA or IIB, columns 5 or 7, lines 19 to 29, 29a to 29u, 363 to 385 yet retain said activity described herein. The nucleic acid molecule can comprise a nucleotide sequence encoding a polypeptide, wherein the polypeptide comprises an amino acid sequence at least about 50% identical to an amino acid sequence of Table IIA or IIB, columns 5 or 7, lines 19 to 29, 29a to 29u, 363 to 385 and is capable of participation in the increase of production of the fine chemical after increasing its activity, e.g. its expression. Preferably, the protein encoded by the nucleic acid molecule is at least about 60% identical to the sequence in Table IIA or IIB, columns 5 or 7, lines 19 to 29, 29a to 29u, 363 to 385 more preferably at least about 70% identical to one of the sequences in Table IIA or IIB, columns 5 or 7, lines 19 to 29, 29a to 29u, 363 to 385 , even more preferably at least about 80%, 90%, 95% homologous to the sequence in Table IIA or IIB, columns 5 or 7, lines 19 to 29, 29a to 29u, 363 to 385 , and most preferably at least about 96%, 97%, 98%, or 99% identical to the sequence in Table IIA or IIB, columns 5 or 7, lines 19 to 29, 29a to 29u, 363 to 385.
Accordingly, the invention relates to nucleic acid molecules encoding a polypeptide having above-mentioned activity, e.g. conferring an increase in the the respective fine chemical in an organisms or parts thereof that contain changes in amino acid residues that are not essential for said activity. Such polypeptides differ in amino acid sequence from a sequence contained in a sequence as indicated in Table II, columns 5 or 7, lines 19 to 29, 29a to 29u, 363 to 385, preferably of Table II B, column 7, 19 to 29, 29a to 29u, 363 to 385 yet retain said activity described herein. The nucleic acid molecule can comprise a nucleotide sequence encoding a polypeptide, wherein the polypeptide comprises an amino acid sequence at least about 50% identical to an amino acid sequence as indicated in Table II, columns 5 or 7, lines 19 to 29, 29a to 29u, 363 to 385, preferably of Table II B, column 7, lines 19 to 29, 29a to 29u, 363 to 385 and is capable of participation in the increase of production of the respective fine chemical after increasing its activity, e.g. its expression. Preferably, the protein encoded by the nucleic acid molecule is at least about 60% identical to a sequence as indicated in Table II, columns 5 or 7, lines 19 to 29, 29a to 29u, 363 to 385, preferably of Table II B, column 7, lines 19 to 29, 29a to 29u, 363 to 385, more preferably at least about 70% identical to one of the sequences as indicated in Table II, columns 5 or 7, lines 19 to 29, 29a to 29u, 363 to 385, preferably of Table II B, column 7, lines 19 to 29, 29a to 29u, 363 to 385, even more preferably at least about 80%, 90%, or 95% homologous to a sequence as indicated in Table II, columns 5 or 7, 19 to 29, 29a to 29u, 363 to 385, preferably of Table II B, column 7, lines 19 to 29, 29a to 29u, 363 to 385, and most preferably at least about 96%, 97%, 98%, or 99% identical to the sequence as indicated in Table II, columns 5 or 7, lines 19 to 29, 29a to 29u, 363 to 385, preferably of Table II B, column 7, lines 19 to 29, 29a to 29u, 363 to 385.

**[0169.0.0.3] to [0172.0.0.3]: see [0169.0.0.0] to [0172.0.0.0]**

**[0173.0.3.3]** For example a sequence which has a 80% homology with sequence SEQ ID No 899 at the nucleic acid level is understood as meaning a sequence which, upon comparison with the sequence SEQ ID No 899 by the above Gap program algorithm with the above parameter set, has a 80% homology.

**[0174.0.0.3]: see [0174.0.0.0]**

**[0175.0.3.3]** For example a sequence which has a 80% homology with sequence SEQ ID No 900 at the protein level is understood as meaning a sequence which, upon comparison with the sequence SEQ ID No 900 by the above program algorithm with the above parameter set, has a 80% homology.

**[0176.0.3.3]** Functional equivalents derived from one of the polypeptides as shown in Table IIA or IIB, columns 5 or 7, lines 19 to 29, 29a to 29u, 363 to 385 according to the invention by substitution, insertion or deletion have at least 30%, 35%, 40%, 45% or 50%, preferably at least 55%, 60%, 65% or 70% by preference at least 80%, especially preferably at least 85% or 90%, 91%, 92%, 93% or 94%, very especially preferably at least 95%, 97%, 98% or 99% homology with one of the polypeptides as shown in Table IIA or IIB, columns 5 or 7, lines 19 to 29, 29a to 29u, 363 to 385 according to the invention and are distinguished by essentially the same properties as the polypeptide as shown in Table IIA or IIB, columns 5 or 7, lines 19 to 29, 29a to 29u, 363 to 385.

**[0177.0.3.3]** Functional equivalents derived from the nucleic acid sequence as shown in Table IA or IB, columns 5 or 7, lines 19 to 29, 29a to 29u, 363 to 385 according to the invention by substitution, insertion or deletion have at least 30%, 35%, 40%, 45% or 50%, preferably at least 55%, 60%, 65% or 70% by preference at least 80%, especially preferably at least 85% or 90%, 91%, 92%, 93% or 94%, very especially preferably at least 95%, 97%, 98% or 99% homology with one of the polypeptides as shown in Table IIA or IIB, columns 5 or 7, lines 19 to 29, 29a to 29u, 363 to 385 according to the invention and encode polypeptides having essentially the same properties as the polypeptide as shown in Table IIA or IIB, columns 5 or 7, lines 19 to 29, 29a to 29u, 363 to 385.

**[0178.0.0.3] see [0178.0.0.0]**

**[0179.0.3.3]** A nucleic acid molecule encoding an homologous to a protein sequence of Table IIA or IIB, columns 5 or 7, lines 19 to 29, 29a to 29u, 363 to 385, preferably of Table II B, column 7, lines 19 to 29, 29a to 29u, 363 to 385, can be created by introducing one or more nucleotide substitutions, additions or deletions into a nucleotide sequence of the nucleic acid molecule of the present invention, in particular of Table IA or IB, columns 5 or 7, lines 19 to 29, 29a to 29u, 363 to 385 such that one or more amino acid substitutions, additions or deletions are introduced into the encoded protein. Mutations can be introduced into the encoding sequences of Table IA or IB, columns 5 or 7, lines 19 to 29, 29a to 29u, 363 to 385 by standard techniques, such as site-directed mutagenesis and PCR-mediated mutagenesis.

**[0180.0.0.3] to [0183.0.0.3]: see [0180.0.0.0] to [0183.0.0.0]**

**[0184.0.3.3]** Homologues of the nucleic acid sequences used, with the sequence shown in Table IA or IB, columns 5 or 7, lines 19 to 29, 29a to 29u, 363 to 385 preferably of Table I B, column 7, lines 19 to 29, 29a to 29u, 363 to 385, or of the nucleic acid sequences derived from the sequences Table IA or IB, columns 5 or 7, lines 19 to 29, 29a to 29u, 363 to 385, preferably of Table I B, column 7, lines 19 to 29, 29a to 29u, 363 to 385 , comprise also allelic variants with at least approximately 30%, 35%, 40% or 45% homology, by preference at least approximately 50%, 60% or 70%, more preferably at least approximately 90%, 91%, 92%, 93%, 94% or 95% and even more preferably at least approximately 96%, 97%, 98%, 99% or more homology with one of the nucleotide sequences shown or the abovementioned derived nucleic acid sequences or their homologues, derivatives or analogues or parts of these. Allelic variants encompass in particular functional variants which can be obtained by deletion, insertion or substitution of nucleotides from the sequences shown, preferably from Table IA or IB, columns 5 or 7, lines 19 to 29, 29a to 29u, 363 to 385 , or from the derived nucleic acid sequences, the intention being, however, that the enzyme activity or the biological activity of the resulting proteins synthesized is advantageously retained or increased.

**[0185.0.3.3]** In one embodiment of the present invention, the nucleic acid molecule of the invention or used in the process of the invention comprises the sequences shown in any of the Table IA or IB, columns 5 or 7, lines 19 to 29, 29a to 29u, 363 to 385, preferably of Table I B, column 7, lines 19 to 29, 29a to 29u, 363 to 385. It is preferred that the nucleic acid molecule comprises as little as possible other nucleotides not shown in any one of Table IA or IB, columns 5 or 7, lines 19 to 29, 29a to 29u, 363 to 385, preferably of Table I B, column 7, lines 19 to 29, 29a to 29u, 363 to 385 . In one embodiment, the nucleic acid molecule comprises less than 500, 400, 300, 200, 100, 90, 80, 70, 60, 50 or 40 further nucleotides. In a further embodiment, the nucleic acid molecule comprises less than 30, 20 or 10 further nucleotides. In one embodiment, the nucleic acid molecule use in the process of the invention is identical to the sequences shown in Table IA or IB, columns 5 or 7, lines 19 to 29, 29a to 29u, 363 to 385, preferably of Table I B, column 7, lines 19 to 29, 29a to 29u, 363 to 385.

**[0186.0.3.3]** Also preferred is that the nucleic acid molecule used in the process of the invention encodes a polypeptide comprising the sequence shown in Table IIA or IIB, lines columns 5 or 7, lines 19 to 29, 29a to 29u, 363 to 385, preferably of Table II B, column 7,19 to 29, 29a to 29u, 363 to 385. In one embodiment, the nucleic acid molecule encodes less than 150, 130, 100, 80, 60, 50, 40 or 30 further amino acids. In a further embodiment, the encoded polypeptide comprises less than 20, 15, 10, 9, 8, 7, 6 or 5 further amino acids. In one embodiment used in the inventive process, the encoded polypeptide is identical to the sequences shown in Table IIA or IIB, columns 5 or 7, lines 19 to 29, 29a to 29u, 363 to 385, preferably of Table II B, column 7, lines 19 to 29, 29a to 29u, 363 to 385.

**[0187.0.3.3]** In one embodiment, the nucleic acid molecule of the invention or used in the process encodes a polypeptide comprising the sequence shown in Table IIA or IIB, columns 5 or 7, lines 19 to 29, 29a to 29u, 363 to 385, preferably of Table II B, column 7, lines 19 to 29, 29a to 29u, 363 to 385 comprises less than 100 further nucleotides. In a further embodiment, said nucleic acid molecule comprises less than 30 further nucleotides. In one embodiment, the nucleic acid molecule used in the process is identical to a coding sequence of the sequences shown in Table IA or IB, columns 5 or 7, lines 19 to 29, 29a to 29u, 363 to 385, preferably of Table I B, column 7, lines 19 to 29, 29a to 29u, 363 to 385.

**[0188.0.3.3]** Polypeptides (= proteins), which still have the essential enzymatic activity of the polypeptide of the present invention conferring an increase of the fine chemical i.e. whose activity is essentially not reduced, are polypeptides with at least 10% or 20%, by preference 30% or 40%, especially preferably 50% or 60%, very especially preferably 80% or 90 or more of the wild type biological activity or enzyme activity, advantageously, the activity is essentially not reduced in comparison with the activity of a polypeptide shown in Table IIA or IIB, columns 5 or 7, lines 19 to 29, 29a to 29u, 363 to 385 expressed under identical conditions.

**[0189.0.3.3]** Homologues of Table IA or IB, columns 5 or 7, lines 19 to 29, 29a to 29u, 363 to 385 or of the derived sequences of Table IIA or IIB, columns 5 or 7, lines 19 to 29, 29a to 29u, 363 to 385 also mean truncated sequences, cDNA, single-stranded DNA or RNA of the coding and noncoding DNA sequence. Homologues of said sequences are also understood as meaning derivatives which comprise noncoding regions such as, for example, UTRs, terminators, enhancers or promoter variants. The promoters upstream of the nucleotide sequences stated can be modified by one or more nucleotide substitution(s), insertion(s) and/or deletion(s) without, however, interfering with the functionality or activity either of the promoters, the open reading frame (= ORF) or with the 3'-regulatory region such as terminators or other 3'regulatory regions, which are far away from the ORF. It is furthermore possible that the activity of the promoters is increased by modification of their sequence, or that they are replaced completely by more active promoters, even promoters from heterologous organisms. Appropriate promoters are known to the person skilled in the art and are mentioned herein below.

**[0190.0.0.3] to [0203.0.0.3]: see [0190.0.0.0] to [0203.0.0.0]**

**[0204.0.0.3]** Accordingly, in one embodiment, the invention relates to a nucleic acid molecule which comprises a nucleic acid molecule selected from the group consisting of:
a) nucleic acid molecule encoding, preferably at least the mature form, of the polypeptide shown in Table IIA or IIB, columns 5 or 7, lines 19 to 29, 29a to 29u, 363 to 385, preferably of Table IIB, column 7, lines 19 to 29, 29a to 29u, 363 to 385 ; or a fragment thereof conferring an increase in the amount of the fine chemical in an organism or a part thereof
b) nucleic acid molecule comprising, preferably at least the mature form, of the nucleic acid molecule shown in Table IA or IB, columns 5 or 7, lines 19 to 29, 29a to 29u, 363 to 385, preferably of Table IB, column 7, lines 19 to 29, 29a to 29u, 363 to 385 or a fragment thereof conferring an increase in the amount of the fine chemical in an organism or a part thereof;
c) nucleic acid molecule whose sequence can be deduced from a polypeptide sequence encoded by a nucleic acid molecule of (a) or (b) as result of the degeneracy of the genetic code and conferring an increase in the amount of the fine chemical in an organism or a part thereof;
d) nucleic acid molecule encoding a polypeptide whose sequence has at least 50% identity with the amino acid sequence of the polypeptide encoded by the nucleic acid molecule of (a) to (c) and conferring an increase in the amount of the fine chemical in an organism or a part thereof;
e) nucleic acid molecule which hybridizes with a nucleic acid molecule of (a) to (c) under under stringent hybridisation conditions and conferring an increase in the amount of the fine chemical in an organism or a part thereof;
f) nucleic acid molecule encoding a polypeptide, the polypeptide being derived by substituting, deleting and/or adding one or more amino acids of the amino acid sequence of the polypeptide encoded by the nucleic acid molecules (a) to (d), preferably to (a) to (c), and conferring an increase in the amount of the fine chemical in an organism or a part thereof;
g) nucleic acid molecule encoding a fragment or an epitope of a polypeptide which is encoded by one of the nucleic acid molecules of (a) to (e), preferably to (a) to (c) and conferring an increase in the amount of the fine chemical in an organism or a part thereof;
h) nucleic acid molecule comprising a nucleic acid molecule which is obtained by amplifying a cDNA library or a genomic library using the primers in Table III, column 7, lines 19 to 29, 29a to 29u, 363 to 385 and conferring an increase in the amount of the fine chemical in an organism or a part thereof;
i) nucleic acid molecule encoding a polypeptide which is isolated, e.g. from a expression library, with the aid of monoclonal antibodies against a polypeptide encoded by one of the nucleic acid molecules of (a) to (g), preferably to (a) to (c) and conferring an increase in the amount of the fine chemical in an organism or a part thereof;
j) nucleic acid molecule which encodes a polypeptide comprising the consensus sequence shown in Table IV, column 7, lines 19 to 29, 29a to 29u, 363 to 385 and conferring an increase in the amount of the fine chemical in an organism or a part thereof;
k) nucleic acid molecule encoding the amino acid sequence of a polypeptide encoding a domaine of the polypeptide shown in Table IIA or IIB, columns 5 or 7, lines 19 to 29, 29a to 29u, 363 to 385 and conferring an increase in the amount of the fine chemical in an organism or a part thereof; and
l) nucleic acid molecule which is obtainable by screening a suitable nucleic acid library under stringent hybridization conditions with a probe comprising one of the sequences of the nucleic acid molecule of (a) to (k) or with a fragment of at least 15 nt, preferably 20 nt, 30 nt, 50 nt, 100 nt, 200 nt or 500 nt of the nucleic acid molecule characterized in (a) to (h) or of the nucleic acid molecule shown in Table IA or IB, columns 5 or 7, lines 19 to 29, 29a to 29u, 363 to 385 or a nucleic acid molecule encoding, preferably at least the mature form of, the polypeptide shown in Table IIA or IIB, columns 5 or 7, lines 19 to 29, 29a to 29u, 363 to 385 , and conferring an increase in the amount of the fine chemical in an organism or a part thereof;
or which encompasses a sequence which is complementary thereto;
whereby, preferably, the nucleic acid molecule according to (a) to (I) distinguishes over the sequence depicted in Table IA, columns 5 or 7, lines 19 to 29, 29a to 29u, 363 to 385 by one or more nucleotides. In one embodiment, the nucleic acid molecule of the invention does not consist of the sequence shown in Table IA or IB, columns 5 or 7, lines 19 to 29, 29a to 29u, 363 to 385 . In another embodiment, the nucleic acid molecule of the present invention is at least 30 % identical and less than 100%, 99,999%, 99,99%, 99,9% or 99% identical to the sequence shown in Table IA or IB, columns 5 or 7, lines 19 to 29, 29a to 29u, 363 to 385 . In a further embodiment the nucleic acid molecule does not encode the polypeptide sequence shown in Table IIA or IIB, columns 5 or 7, lines 19 to 29, 29a to 29u, 363 to 385 . Accordingly, in one embodiment, the nucleic acid molecule of the present invention encodes in one embodiment a polypeptide which differs at least in one or more amino acids from the polypeptide depicted in Table IIA or IIB, columns 5 or 7, lines 19 to 29, 29a to 29u, 363 to 385 .In another embodiment, the nucleic acid molecule depicted in Table IA or IB, columns 5 or 7, lines 19 to 29, 29a to 29u, 363 to 385 does not encode a protein of the sequence shown in Table IIA or IIB, columns 5 or 7, lines 19 to 29, 29a to 29u, 363 to 385 . Accordingly, in one embodiment, the protein encoded by a sequences of a nucleic acid according to (a) to (I) does not consist of the sequence shown in Table IIA or IIB, columns 5 or 7, lines 19 to 29, 29a to 29u, 363 to 385 . In a further embodiment, the protein of the present invention is at least 30 % identical to protein sequence depicted in Table IIA or IIB, columns 5 or 7, lines 19 to 29, 29a to 29u, 363 to 385 and less than 100%, preferably less than 99,999%, 99,99% or 99,9%, more preferably less than 99%, 98%, 97%, 96% or 95% identical to the sequence shown in Table IIA or IIB, columns 5 or 7, lines 19 to 29, 29a to 29u, 363 to 385.

**[0205.0.0.3] to [0226.0.0.3]: see [0205.0.0.0] to [0226.0.0.0]**

**[0227.0.3.3]** The abovementioned nucleic acid molecules can be cloned into the nucleic acid constructs or vectors according to the invention in combination together with further genes, or else different genes are introduced by transforming several nucleic acid constructs or vectors (including plasmids) into a host cell, advantageously into a plant cell or a microorgansims.
In addition to the sequence mentioned in Table IA or IB, columns 5 or 7, lines 19 to 29, 29a to 29u, 363 to 385 or its derivatives, it is advantageous additionally to express and/or mutate further genes in the organisms. Especially advantageously, additionally at least one further gene of the amino acid biosynthetic pathway such as for L-lysine, L-threonine and/or L-methionine or L-leucine and/or isoleucine and/or valine is expressed in the organisms such as plants or microorganisms. It is also possible that the regulation of the natural genes has been modified advantageously so that the gene and/or its gene product is no longer subject to the regulatory mechanisms which exist in the organisms. This leads to an increased synthesis of the amino acids desired since, for example, feedback regulations no longer exist to the same extent or not at all. In addition it might be advantageously to combine the sequences shown in Table IA or IB, columns 5 or 7, lines 19 to 29, 29a to 29u, 363 to 385, with genes which generally support or enhances to growth or yield of the target organismen, for example genes which lead to faster growth rate of microorganisms or genes which produces stress-, pathogen, or herbicide resistant plants.

**[0228.0.0.3] to [0230.0.0.3]: see [0228.0.0.0] to [0230.0.0.0]**

**[0231.0.0.3]** In a further advantageous embodiment of the process of the invention, the organisms used in the process are those in which simultaneously a leucine and/or isoleucine and/or valine degrading protein is attenuated, in particular by reducing the rate of expression of the corresponding gene.

**[0232.0.0.3] to [0282.0.0.3]: see [0232.0.0.0] to [0282.0.0.0]**

**[0283.0.3.3]** Moreover, native polypeptide conferring the increase of the fine chemical in an organism or part thereof can be isolated from cells (e.g., endothelial cells), for example using the antibody of the present invention as described below, in particular, an anti- YDL127W, YDR245W, YDR271C, YER173W, YGR101W, YJL072C, YKR057W, YNL135C, YFL013C, YGR104C, YIL150C, YOR350C, YFR042W, YFL019C, b1708, b1829, b2957, b3366, b0828, b3966, b4151, b1827, b0124, b0149, b0161, b0486, b1313, b1343, b1463, b2022, b2414, b2664, b3117, b3256, b3938, b3983, b4054 and/or b4327 protein antibody or an antibody against polypeptides as shown in Table IIA or IIB, columns 5 or 7, lines 19 to 29, 29a to 29u, 363 to 385 , which can be produced by standard techniques utilizing the polypeptid of the present invention or fragment thereof, i.e., the polypeptide of this invention. Preferred are monoclonal antibodies.

**[0284.0.0.3]: see [0284.0.0.0]**

**[0285.0.3.3]** In one embodiment, the present invention relates to a polypeptide having the sequence shown in Table IIA or IIB, columns 5 or 7, lines 19 to 29, 29a to 29u, 363 to 385 or as coded by the nucleic acid molecule shown in Table IA or IB, columns 5 or 7, lines 19 to 29, 29a to 29u, 363 to 385 or functional homologues thereof.

**[0286.0.3.3]** In one advantageous embodiment, in the method of the present invention the activity of a polypeptide is increased comprising or consisting of the consensus sequence shown in Table IV, lines 19 to 29, 29a to 29u, 363 to 385 and in one another embodiment, the present invention relates to a polypeptide comprising or consisting of the consensus sequence shown in Table IV, lines 19 to 29, 29a to 29u, 363 to 385,
whereby 20 or less, preferably 15 or 10, preferably 9, 8, 7, or 6, more preferred 5 or 4, even more preferred 3, even more preferred 2, even more preferred 1, most preferred 0 of the amino acids positions indicated can be replaced by any amino acid or, in an further embodiment, can be replaced and/or absent.

**[0287.0.0.3] to [0290.0.0.3]: see [0287.0.0.0] to [0290.0.0.0]**

**[0291.0.3.3]** In one advantageous embodiment, the method of the present invention comprises the increasing of a polypeptide comprising or consisting of plant or microorganism specific consensus sequences.
In one embodiment, said polypeptide of the invention distinguishes over the sequence depicted in Table IIA or IIB, columns 5 or 7, lines 19 to 29, 29a to 29u, 363 to 385 by one or more amino acids. In one embodiment, polypeptide distinguishes form the sequence shown in Table IIA or IIB, columns 5 or 7, lines 19 to 29, 29a to 29u, 363 to 385 by more than 5, 6, 7, 8 or 9 amino acids, preferably by more than 10, 15, 20, 25 or 30 amino acids, evenmore preferred are more than 40, 50, or 60 amino acids and, preferably, the sequence of the polypeptide of the invention distinguishes from the sequence shown in Table IIA or IIB, columns 5 or 7, lines 19 to 29, 29a to 29u, 363 to 385 by not more than 80% or 70% of the amino acids, preferably not more than 60% or 50%, more preferred not more than 40% or 30%, even more preferred not more than 20% or 10%. In another embodiment, said polypeptide of the invention does not consist of the sequence shown in Table IIA or IIB, columns 5 or 7, lines 19 to 29, 29a to 29u, 363 to 385.

**[0292.0.0.3]: see [0292.0.0.0]**

**[0293.0.3.3]** In one embodiment, the invention relates to polypeptide conferring an increase in the fine chemical in an organism or part being encoded by the nucleic acid molecule of the invention or used in the process of the invention and having a sequence which distinguishes from the sequence as shown in Table IIA or IIB, columns 5 or 7, lines 19 to 29, 29a to 29u, 363 to 385 by one or more amino acids. In an other embodiment, said polypeptide of the invention does not consist of the sequence shown in Table IIA or IIB, columns 5 or 7, lines 19 to 29, 29a to 29u, 363 to 385. In a further embodiment, said polypeptide of the present invention is less than 100%, 99,999%, 99,99%, 99,9% or 99% identical. In one embodiment, said polypeptide does not consist of the sequence encoded by the nucleic acid molecules shown in Table IA or IB, columns 5 or 7, lines 19 to 29, 29a to 29u, 363 to 385.

**[0294.0.3.3]** In one embodiment, the present invention relates to a polypeptide having YDL127W, YDR245W, YDR271C, YER173W, YGR101W, YJL072C, YKR057W, YNL135C, YFL013C, YGR104C, YIL150C, YOR350C, YFR042W, YFL019C, b1708, b1829, b2957, b3366, b0828, b3966, b4151, b1827, b0124, b0149, b0161, b0486, b1313, b1343, b1463, b2022, b2414, b2664, b3117, b3256, b3938, b3983, b4054 and/or b4327 protein activity, which distinguishes over the sequence depicted in Table IIA or IIB, columns 5 or 7, lines 19 to 29, 29a to 29u, 363 to 385 by one or more amino acids, preferably by more than 5, 6, 7, 8 or 9 amino acids, preferably by more than 10, 15, 20, 25 or 30 amino acids, even more preferred are more than 40, 50, or 60 amino acids but even more preferred by less than 70% of the amino acids, more preferred by less than 50%, even more preferred my less than 30% or 25%, more preferred are 20% or 15%, even more preferred are less than 10%.

**[0295.0.0.3] to [0297.0.0.3]: see [0295.0.0.0] to [0297.0.0.0]**

**[0297.0.3.3]** The language "substantially free of cellular material" includes preparations of the polypeptide of the invention in which the protein is separated from cellular components of the cells in which it is naturally or recombinantly produced. In one embodiment, the language "substantially free of cellular material" includes preparations having less than about 30% (by dry weight) of "contaminating protein", more preferably less than about 20% of "contaminating protein", still more preferably less than about 10% of "contaminating protein", and most preferably less than about 5% "contaminating protein". The term "Contaminating protein" relates to polypeptides, which are not polypeptides of the present invention. When the polypeptide of the present invention or biologically active portion thereof is recombinantly produced, it is also preferably substantially free of culture medium, i.e., culture medium represents less than about 20%, more preferably less than about 10%, and most preferably less than about 5% of the volume of the protein preparation. The language "substantially free of chemical precursors or other chemicals" includes preparations in which the polypeptide of the present invention is separated from chemical precursors or other chemicals which are involved in the synthesis of the protein. The language "substantially free of chemical precursors or other chemicals" includes preparations having less than about 30% (by dry weight) of chemical precursors or non-YDL127W, YDR245W, YDR271C, YER173W, YGR101W, YJL072C, YKR057W, YNL135C, YFL013C, YGR104C, YIL150C, YOR350C, YFR042W, YFL019C, b1708, b1829, b2957, b3366, b0828, b3966, b4151, b1827, b0124, b0149, b0161, b0486, b1313, b1343, b1463, b2022, b2414, b2664, b3117, b3256, b3938, b3983, b4054 and/or b4327 chemicals, more preferably less than about 20% chemical precursors or non-YDL127W, YDR245W, YDR271C, YER173W, YGR101W, YJL072C, YKR057W, YNL135C, YFL013C, YGR104C, YIL150C, YOR350C, YFR042W, YFL019C, b1708, b1829, b2957, b3366, b0828, b3966, b4151, b1827, b0124, b0149, b0161, b0486, b1313, b1343, b1463, b2022, b2414, b2664, b3117, b3256, b3938, b3983, b4054 and/or b4327 chemicals, still more preferably less than about 10% chemical precursors or non-YDL127W, YDR245W, YDR271C, YER173W, YGR101W, YJL072C, YKR057W, YNL135C, YFL013C, YGR104C, YIL150C, YOR350C, YFR042W, YFL019C, b1708, b1829, b2957, b3366, b0828, b3966, b4151, b1827, b0124, b0149, b0161, b0486, b1313, b1343, b1463, b2022, b2414, b2664, b3117, b3256, b3938, b3983, b4054 and/or b4327 chemicals, and most preferably less than about 5% chemical precursors or non- YDL127W, YDR245W, YDR271C, YER173W, YGR101W, YJL072C, YKR057W, YNL135C, YFL013C, YGR104C, YIL150C, YOR350C, YFR042W, YFL019C, b1708, b1829, b2957, b3366, b0828, b3966, b4151, b1827, b0124, b0149, b0161, b0486, b1313, b1343, b1463, b2022, b2414, b2664, b3117, b3256, b3938, b3983, b4054 and/or b4327 chemicals. In preferred embodiments, isolated proteins or biologically active portions thereof lack contaminating proteins from the same organism from which the polypeptide of the present invention is derived. Typically, such proteins are produced by recombinant techniques.̅

**[00297.1.0.3]** Non-polypeptide of the invention-chemicals are e.g. polypeptides having not the activity and/or the amino acid sequence of a polypeptide indicated in Table II, columns 3, 5 or 7, lines 19 to 29, 29a to 29u, 363 to 385.

**[0298.0.3.3]** A polypeptide of the invention can participate in the process of the present invention. The polypeptide or a portion thereof comprises preferably an amino acid sequence which is sufficiently homologous to an amino acid sequence shown in Table IIA or IIB, columns 5 or 7, lines 19 to 29, 29a to 29u, 363 to 385 such that the protein or portion thereof maintains the ability to confer the activity of the present invention. The portion of the protein is preferably a biologically active portion as described herein. Preferably, the polypeptide used in the processof the invention has an amino acid sequence identical as shown in Table IIA or IIB, columns 5 or 7, lines 19 to 29, 29a to 29u, 363 to 385 .

**[0299.0.3.3]** Further, the polypeptide can have an amino acid sequence which is encoded by a nucleotide sequence which hybridizes, preferably hybridizes under stringent conditions as described above, to a nucleotide sequence of the nucleic acid molecule of the present invention. Accordingly, the polypeptide has an amino acid sequence which is encoded by a nucleotide sequence that is at least about 35%, 40%, 45%, 50%, 55%, 60%, 65% or 70%, preferably at least about 75%, 80%, 85% or 90, and more preferably at least about 91%, 92%, 93%, 94% or 95%, and even more preferably at least about 96%, 97%, 98%, 99% or more homologous to one of the amino acid sequences of Table IIA or IIB, columns 5 or 7, lines 19 to 29, 29a to 29u, 363 to 385 . The preferred polypeptide of the present invention preferably possesses at least one of the activities according to the invention and described herein. A preferred polypeptide of the present invention includes an amino acid sequence encoded by a nucleotide sequence which hybridizes, preferably hybridizes under stringent conditions, to a nucleotide sequence of Table IA or IB, columns 5 or 7, lines 19 to 29, 29a to 29u, 363 to 385 or which is homologous thereto, as defined above.

**[0300.0.3.3]** Accordingly the polypeptide of the present invention can vary from Table IIA or IIB, columns 5 or 7, lines 19 to 29, 29a to 29u, 363 to 385 in amino acid sequence due to natural variation or mutagenesis, as described in detail herein. Accordingly, the polypeptide comprise an amino acid sequence which is at least about 35%, 40%, 45%, 50%, 55%, 60%, 65% or 70%, preferably at least about 75%, 80%, 85% or 90%, and more preferably at least about 91%, 92%, 93%, 94% or 95%, and most preferably at least about 96%, 97%, 98%, 99% or more homologous to an entire amino acid sequence of Table IIA or IIB, columns 5 or 7, lines 19 to 29, 29a to 29u, 363 to 385.

**[0301.0.0.3] : see [0301.0.0.0]**

**[0302.0.3.3]** Biologically active portions of an polypeptide of the present invention include peptides comprising amino acid sequences derived from the amino acid sequence of the polypeptide of the present invention or used in the process of the present invention, e.g., the amino acid sequence shown in Table IIA or IIB, columns 5 or 7, lines 19 to 29, 29a to 29u, 363 to 385 or the amino acid sequence of a protein homologous thereto, which include fewer amino acids than a full length polypeptide of the present invention or used in the process of the present invention or the full length protein which is homologous to an polypeptide of the present invention or used in the process of the present invention depicted herein, and exhibit at least one activity of polypeptide of the present invention or used in the process of the present invention.

**[0303.0.0.3]: see [0303.0.0.0]**

**[0304.0.3.3]** Manipulation of the nucleic acid molecule of the invention may result in the production of YDL127W, YDR245W, YDR271C, YER173W, YGR101W, YJL072C, YKR057W, YNL135C, YFL013C, YGR104C, YIL150C, YOR350C, YFR042W, YFL019C, b1708, b1829, b2957, b3366, b0828, b3966, b4151, b1827, b0124, b0149, b0161, b0486, b1313, b1343, b1463, b2022, b2414, b2664, b3117, b3256, b3938, b3983, b4054 and/or b4327 protein having differences from the wild-type YDL127W, YDR245W, YDR271C, YER173W, YGR101W, YJL072C, YKR057W, YNL135C, YFL013C, YGR104C, YIL150C, YOR350C, YFR042W, YFL019C, b1708, b1829, b2957, b3366, b0828, b3966, b4151, b1827, b0124, b0149, b0161, b0486, b1313, b1343, b1463, b2022, b2414, b2664, b3117, b3256, b3938, b3983, b4054 and/or b4327 protein. These proteins may be improved in efficiency or activity, may be present in greater numbers in the cell than is usual, or may be decreased in efficiency or activity in relation to the wild type protein.

**[0305.0.0.3] : see [0305.0.0.0]**

[0305.0.3.3] Any mutagenesis strategies for the polypeptide of the present invention or the polypeptide used in the process of the present invention to result in increasing said activity are not meant to be limiting; variations on these strategies will be readily apparent to one skilled in the art. Using such strategies, and incorporating the mechanisms disclosed herein, the nucleic acid molecule and polypeptide of the invention may be utilized to generate plants or parts thereof, expressing wildtype YDL127W, YDR245W, YDR271C, YER173W, YGR101W, YJL072C, YKR057W, YNL135C, YFL013C, YGR104C, YIL150C, YOR350C, YFR042W, YFL019C, b1708, b1829, b2957, b3366, b0828, b3966, b4151, b1827, b0124, b0149, b0161, b0486, b1313, b1343, b1463, b2022, b2414, b2664, b3117, b3256, b3938, b3983, b4054 and/or b4327 proteins or mutated YDL127W, YDR245W, YDR271C, YER173W, YGR101W, YJL072C, YKR057W, YNL135C, YFL013C, YGR104C, YIL150C, YOR350C, YFR042W, YFL019C, b1708, b1829, b2957, b3366, b0828, b3966, b4151, b1827, b0124, b0149, b0161, b0486, b1313, b1343, b1463, b2022, b2414, b2664, b3117, b3256, b3938, b3983, b4054 and/or b4327 protein encoding nucleic acid molecules and polypeptide molecules of the invention such that the yield, production, and/or efficiency of production of a desired compound is improved.

**[0306.0.0.3] to [0308.0.0.3]: see [0306.0.0.0] to [0308.0.0.0]**

**[0309.0.3.3]** In one embodiment, an " YDL127W, YDR245W, YDR271C, YER173W, YGR101W, YJL072C, YKR057W, YNL135C, YFL013C, YGR104C, YIL150C, YOR350C, YFR042W, YFL019C, b1708, b1829, b2957, b3366, b0828, b3966, b4151 , b1827, b0124, b0149, b0161, b0486, b1313, b1343, b1463, b2022, b2414, b2664, b3117, b3256, b3938, b3983, b4054 and/or b4327 protein (= polypeptide)" refers to a polypeptide having an amino acid sequence corresponding to the polypeptide of the invention or used in the process of the invention , whereas a "non- YDL127W, YDR245W, YDR271C, YER173W, YGR101W, YJL072C, YKR057W, YNL135C, YFL013C, YGR104C, YIL150C, YOR350C, YFR042W, YFL019C, b1708, b1829, b2957, b3366, b0828, b3966, b4151, b1827, b0124, b0149, b0161, b0486, b1313, b1343, b1463, b2022, b2414, b2664, b3117, b3256, b3938, b3983, b4054 and/or b4327 polypeptide" or "other polypeptide" refers to a polypeptide having an amino acid sequence corresponding to a protein which is not substantially homologous a polypeptide of the invention, preferably which is not substantially homologous to a polypeptide having an YDL127W, YDR245W, YDR271C, YER173W, YGR101W, YJL072C, YKR057W, YNL135C, YFL013C, YGR104C, YIL150C, YOR350C, YFR042W, YFL019C, b1708, b1829, b2957, b3366, b0828, b3966, b4151, b1827, b0124, b0149, b0161, b0486, b1313, b1343, b1463, b2022, b2414, b2664, b3117, b3256, b3938, b3983, b4054 and/or b4327 protein activity, e.g., a protein which does not confer the activity described herein and which is derived from the same or a different organism.

**[0310.0.0.3] to [0334.0.0.3]: see [0310.0.0.0] to [0334.0.0.0]**

**[0335.0.3.3]** The dsRNAi method has proved to be particularly effective and advantageous for reducing the expression of the nucleic acid sequences of the Table IA or IB, columns 5 or 7, lines 19 to 29, 29a to 29u, 363 to 385 and/or homologs thereof. As described inter alia in WO 99/32619, dsRNAi approaches are clearly superior to traditional antisense approaches. The invention therefore furthermore relates to double-stranded RNA molecules (dsRNA molecules) which, when introduced into an organism, advantageously into a plant (or a cell, tissue, organ or seed derived therefrom), bring about altered metabolic activity by the reduction in the expression of the nucleic acid sequences of the Table IA or IB, columns 5 or 7, lines 19 to 29, 29a to 29u, 363 to 385 and/or homologs thereof. In a double-stranded RNA molecule for reducing the expression of an protein encoded by a nucleic acid sequence of one of the Table IA or IB, columns 5 or 7, lines 19 to 29, 29a to 29u, 363 to 385 and/or homologs thereof, one of the two RNA strands is essentially identical to at least part of a nucleic acid sequence, and the respective other RNA strand is essentially identical to at least part of the complementary strand of a nucleic acid sequence.

**[0336.0.0.3] to [0342.0.0.3]: see [0336.0.0.0] to [0342.0.0.0]**

**[0343.0.3.3]** As has already been described, 100 % sequence identity between the dsRNA and a gene transcript of a nucleic acid sequence of one of the Table IA or IB, columns 5 or 7, lines 19 to 29, 29a to 29u, 363 to 385 or it's homolog is not necessarily required in order to bring about effective reduction in the expression. The advantage is, accordingly, that the method is tolerant with regard to sequence deviations as may be present as a consequence of genetic mutations, polymorphisms or evolutionary divergences. Thus, for example, using the dsRNA, which has been generated starting from a sequence of one of Table IA or IB, columns 5 or 7, lines 19 to 29, 29a to 29u, 363 to 385 or homologs thereof of the one organism, may be used to suppress the corresponding expression in another organism.

**[0344.0.0.3] to [0361.0.0.3]: see [0344.0.0.0] to [0361.0.0.0]**

[0362.0.3.3] Accordingly the present invention relates to any cell transgenic for any nucleic acid characterized as part of the invention, e.g. conferring the increase of the fine chemical in a cell or an organism or a part thereof, e.g. the nucleic acid molecule of the invention, the nucleic acid construct of the invention, the antisense molecule of the invention, the vector of the invention or a nucleic acid molecule encoding the polypeptide of the invention, e.g. encoding a polypeptide having an YDL127W, YDR245W, YDR271C, YER173W, YGR101W, YJL072C, YKR057W, YNL135C, YFL013C, YGR104C, YIL150C, YOR350C, YFR042W, YFL019C, b1708, b1829, b2957, b3366, b0828, b3966, b4151, b1827, b0124, b0149, b0161, b0486, b1313, b1343, b1463, b2022, b2414, b2664, b3117, b3256, b3938, b3983, b4054 and/or b4327 protein activity. Due to the above mentioned activity the fine chemical content in a cell or an organism is increased. For example, due to modulation or manupulation, the cellular activity is increased, e.g. due to an increased expression or specific activity of the subject matters of the invention in a cell or an organism or a part thereof. Transgenic for a polypeptide having an YDL127W, YDR245W, YDR271C, YER173W, YGR101W, YJL072C, YKR057W, YNL135C, YFL013C, YGR104C, YIL150C, YOR350C, YFR042W, YFL019C, b1708, b1829, b2957, b3366, b0828, b3966, b4151, b1827, b0124, b0149, b0161, b0486, b1313, b1343, b1463, b2022, b2414, b2664, b3117, b3256, b3938, b3983, b4054 and/or b4327 protein or activity means herein that due to modulation or manipulation of the genome, the activity of YDL127W, YDR245W, YDR271C, YER173W, YGR101W, YJL072C, YKR057W, YNL135C, YFL013C, YGR104C, YIL150C, YOR350C, YFR042W, YFL019C, b1708, b1829, b2957, b3366, b0828, b3966, b4151, b1827, b0124, b0149, b0161, b0486, b1313, b1343, b1463, b2022, b2414, b2664, b3117, b3256, b3938, b3983, b4054 and/or b4327 or a YDL127W, YDR245W, YDR271C, YER173W, YGR101W, YJL072C, YKR057W, YNL135C, YFL013C, YGR104C, YIL150C, YOR350C, YFR042W, YFL019C, b1708, b1829, b2957, b3366, b0828, b3966, b4151, b1827, b0124, b0149, b0161, b0486, b1313, b1343, b1463, b2022, b2414, b2664, b3117, b3256, b3938, b3983, b4054 and/or b4327 -like activity is increased in the cell or organism or part thereof. Examples are described above in context with the process of the invention.

**[0363.0.0.3]: see [0363.0.0.0]**

**[0364.0.3.3]** A naturally occurring expression cassette - for example the naturally occurring combination of the YDL127W, YDR245W, YDR271C, YER173W, YGR101W, YJL072C, YKR057W, YNL135C, YFL013C, YGR104C, YIL150C, YOR350C, YFR042W,YFL019C, b1708, b1829, b2957, b3366, b0828, b3966, b4151, b1827, b0124, b0149, b0161, b0486, b1313, b1343, b1463, b2022, b2414, b2664, b3117, b3256, b3938, b3983, b4054 and/or b4327 protein promoter with the corresponding YDL127W, YDR245W, YDR271C, YER173W, YGR101 W, YJL072C, YKR057W, YNL135C, YFL013C, YGR104C, YIL150C, YOR350C, YFR042W, YFL019C, b1708, b1829, b2957, b3366, b0828, b3966, b4151, b1827, b0124, b0149, b0161, b0486, b1313, b1343, b1463, b2022, b2414, b2664, b3117, b3256, b3938, b3983, b4054 and/or b4327 protein gene - becomes a transgenic expression cassette when it is modified by non-natural, synthetic "artificial" methods such as, for example, mutagenization. Such methods have been described (US 5,565,350; WO 00/15815; also see above).

**[0365.0.0.3] to [0382.0.0.3]: see [0365.0.0.0] to [0382.0.0.0]**

**[0383.0.3.3]** For preparing branched-chain amino acid compound-containing fine chemicals, in particular the fine chemical, it is possible to use as branched-chain amino acid source organic branched-chain amino acid-containing compounds such as, for example, isovalerate, isopropylmalate, oxoisocaproate, isovaleryl-compounds, methyl-valerate, isobutyrate, methyl-butyryl-compounds, isopropyrate, isopropyl-compounds or else organic branched-chain amino acid precursor compounds.

**[0384.0.0.3] to [0392.0.0.3]: see [0384.0.0.0] to [0392.0.0.0]**

**[0393.0.3.3]** In one embodiment, the present invention relates to a method for the identification of a gene product conferring an increase in the fine chemical production in a cell, comprising the following steps:
(g) contacting,̅ e.g. hybridising, the nucleic acid molecules of a sample, e.g. cells, tissues, plants or microorganisms or a nucleic acid library , which can contain a candidate gene encoding a gene product conferring an increase in the fine chemical after expression, with the nucleic acid molecule of the present invention;
(h) identifying the nucleic acid molecules, which hybridize under relaxed stringent conditions with the nucleic acid molecule of the present invention in particular to the nucleic acid molecule sequence shown in Table IA or IB, columns 5 or 7, lines 19 to 29, 29a to 29u, 363 to 385, preferably in Table I B, column 7, lines 19 to 29, 29a to 29u, 363 to 385 and, optionally, isolating the full length cDNA clone or complete genomic clone;
(i) introducing the candidate nucleic acid molecules in host cells, preferably in a plant cell or a microorganism, appropriate for producing the fine chemical;
(j) expressing the identified nucleic acid molecules in the host cells;
(k) assaying the the fine chemical level in the host cells; and
(l) identifying the nucleic acid molecule and its gene product which expression confers an increase in the the fine chemical level in the host cell after expression compared to the wild type.

**[0394.0.0.3] to [0398.0.0.3]: see [0394.0.0.0] to [0398.0.0.0]**

**[0399.0.3.3]** Furthermore, in one embodiment, the present invention relates to process for the identification of a compound conferring increased the fine chemical production in a plant or microorganism, comprising the steps:
(c) culturing a cell or tissue or microorganism or maintaining a plant expressing the polypeptide according to the invention or a nucleic acid molecule encoding said polypeptide and a readout system capable of interacting with the polypeptide under suitable conditions which permit the interaction of the polypeptide with said readout system in the presence of a compound or a sample comprising a plurality of compounds and capable of providing a detectable signal in response to the binding of a compound to said polypeptide under conditions which permit the expression of said readout system and the polypeptide of the present invention or used in the process of the invention; and
(d) identifying if the compound is an effective agonist by detecting the presence or absence or increase of a signal produced by said readout system.
The screen for a gene product or an agonist conferring an increase in the fine chemical production can be performed by growth of an organism for example a microorganism in the presence of growth reducing amounts of an inhibitor of the synthesis of the fine chemical. Better growth, eg higher dividing rate or high dry mass in comparison to the control under such conditions would identify a gene or gene product or an agonist conferring an increase in fine chemical production.
One can think to screen for increased fine chemical production by for example resistance to drugs blocking isoleucine synthesis and looking whether this effect is YDL127W dependent eg comparing near identical organisms with low and high YDL127W acitivity.

**[0400.0.0.3] to [0430.0.0.3]: see [0400.0.0.1] to [0430.0.0.0]**

**[0431.0.3.3]** Example 1: Cloning SEQ ID No: 899 in Escherichia coli

**[0432.0.3.3]** SEQ ID No: 899 was cloned into the plasmids pBR322 (Sutcliffe, J.G. (1979) Proc. Natl Acad. Sci. USA, 75: 3737-3741); pACYC177 (Change & Cohen (1978) J. Bacteriol. 134: 1141-1156); plasmids of the pBS series (pBSSK+, pBSSK-and others; Stratagene, LaJolla, USA) or cosmids such as SuperCos1 (Stratagene, LaJolla, USA) or Lorist6 (Gibson, T.J. Rosenthal, A., and Waterson, R.H. (1987) Gene 53: 283-286) for expression in E. coli using known, well-established procedures (see, for example, Sambrook, J. et al. (1989) "Molecular Cloning: A Laboratory Manual". Cold Spring Harbor Laboratory Press or Ausubel, F.M. et al. (1994) "Current Protocols in Molecular Biology", John Wiley & Sons).

**[0433.0.0.3] to [0460.0.0.3]: see [0433.0.0.0] to [0460.0.0.0]**

**[0461.0.3.3]** Example 10: Cloning SEQ ID NO: 899 for the expression in plants

**[0462.0.0.3]: see [0462.0.0.0]**

**[0463.0.3.3]** SEQ ID NO: 899 is amplified by PCR as described in the protocol of the Pfu Turbo or DNA Herculase polymerase (Stratagene).

**[0464.0.0.3] to [0466.0.0.3]: see [0464.0.0.0] to [0466.0.0.0]**

**[0467.0.3.3]** The following primer sequences were selected for the gene SEQ ID No: 899:
i) forward primer (SEQ ID No: 911)
   atgtcaaact acgaagcctt gctg
ii) reverse primer (SEQ ID No: 912)
   tcacagggcg cgctttacta aaat

**[0468.0.0.3] to [0479.0.0.3]: see [0468.0.0.0] to [0479.0.0.0]**

**[0480.0.3.3]** Example 11: Generation of transgenic plants which express SEQ ID No: 899

**[0481.0.0.3] to [0513.0.0.3]: see [0481.0.0.0] to [0513.0.0.0]**

**[0514.0.3.3]** As an alternative, the amino acids can be detected advantageously via HPLC separation in ethanolic extract as described by Geigenberger et al. (Plant Cell & Environ, 19, 1996: 43-55).
The results of the different plant analyses can be seen from the table which follows:

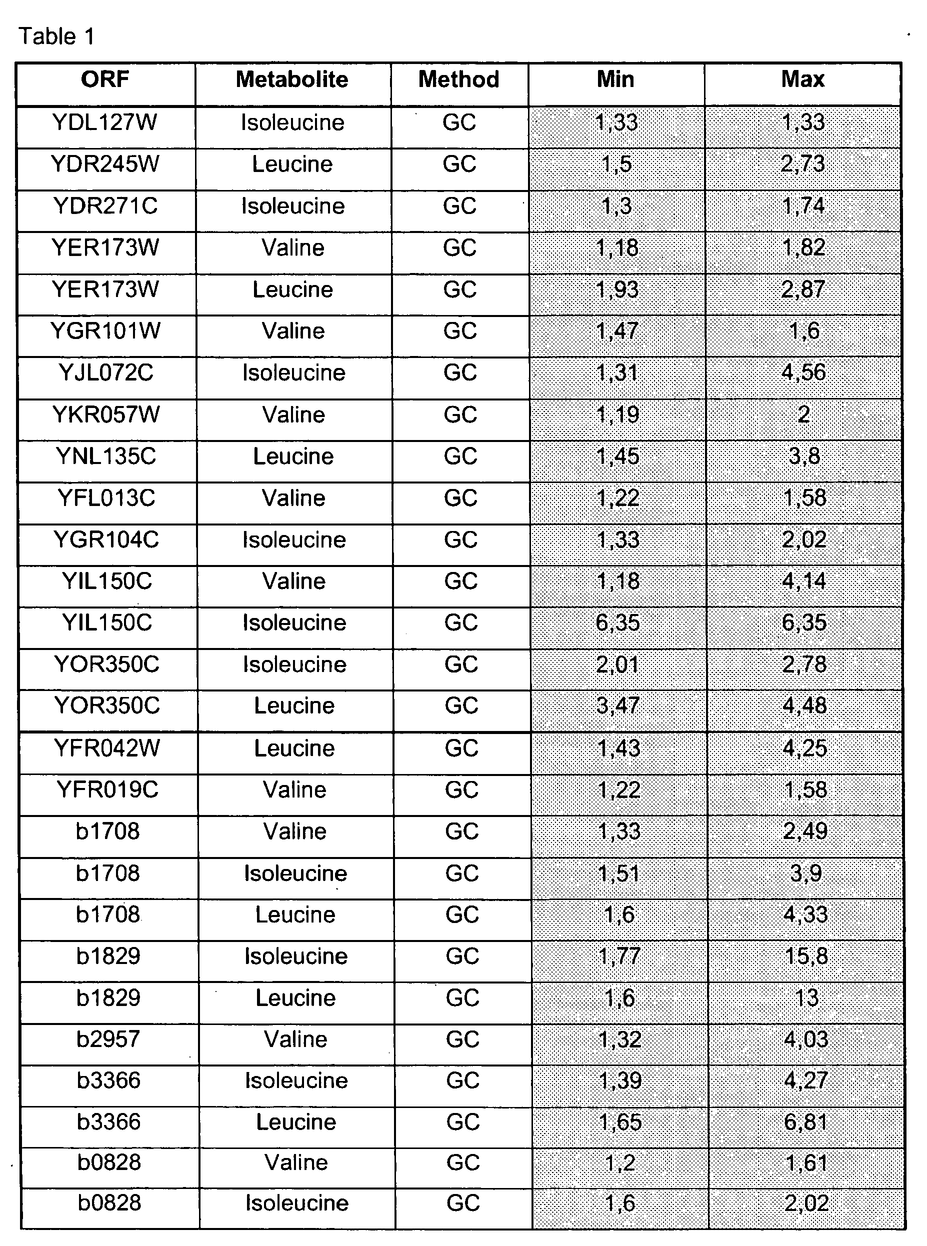

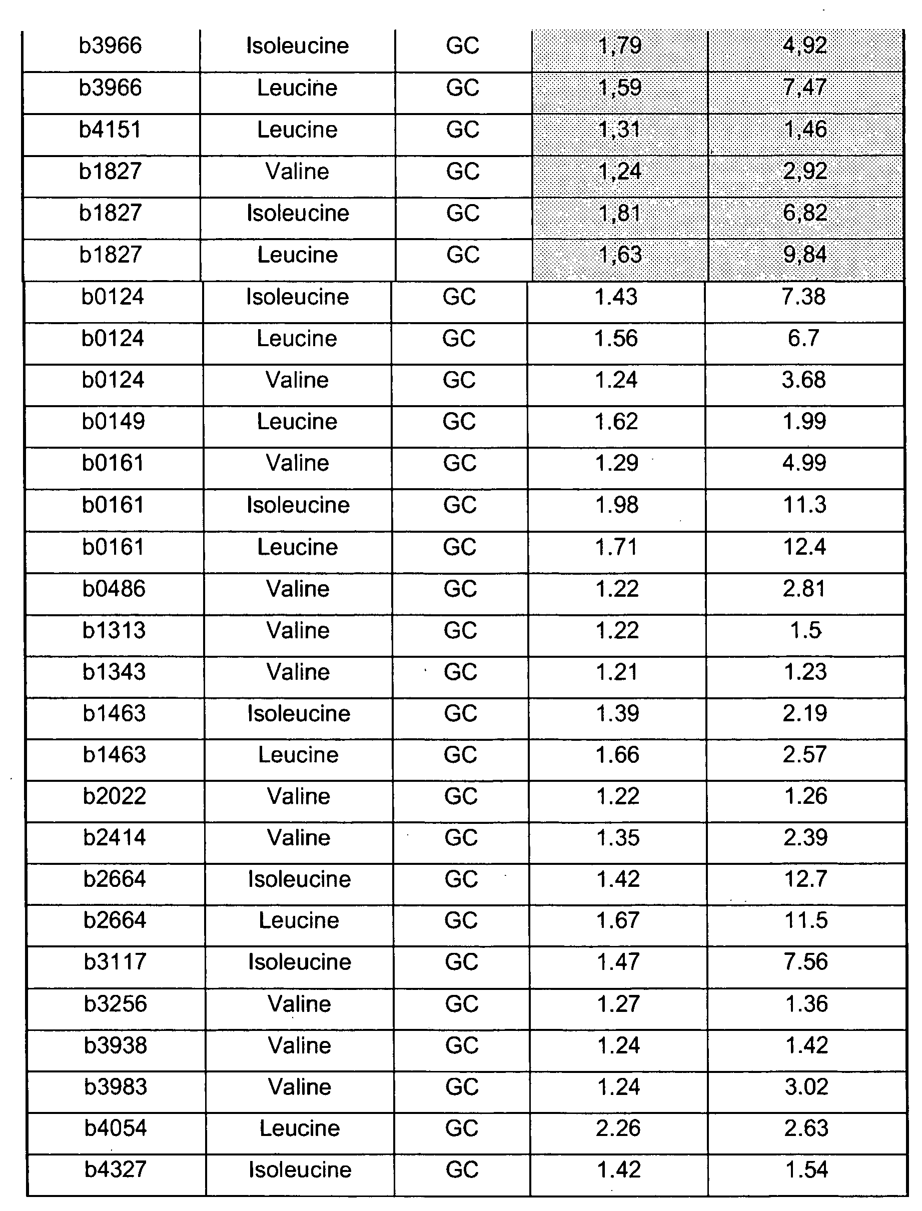

**[0515.0.0.3]:** to [0552.0.0.3]: see [0515.0.0.0] to [0552.0.0.0]

**[0552.1.0.3]:** Example 15: Metabolite Profiling Info from *Zea mays*
*Zea mays* plants were engineered as described in Example 14c.
The results of the different *Zea mays* plants analysed can be seen from table 2 which follows:

**Table 2**

| ORF_NAME | Metabolite | Min | Max |
|---|---|---|---|
| YKR057W | Valine | 1.29 | 1.62 |
| YIL150C | Valine | 1.89 | 3.26 |
| YIL150C | Isoleucine | 1.79 | 2.98 |

Table 2 describes the increase in valine and/or isoleucine in genetically modified corn plants expressing the Saccharomyces cerevisiae nucleic acid sequence YKR057W or YIL150C.
In case the activity of the Saccharomyces cerevisiae protein YKR057W or a ribosomal protein, similar to S21 ribosomal proteins, involved in ribosome biogenesis and translation or its homolog, is increased in corn plants, preferably, an increase of the fine chemical valine between 29% and 62% is conferred.
In one embodiment, in case the activity of the Saccaromyces cerevisiae protein YIL150C or its homologs, e.g. "a chromatin binding protein, required for S-phase (DNA synthesis) initiation or completion" or its homologs, is increased in corn plants, preferably, an increase of the fine chemical valine between 89% and 226% and of the fine chemical isoleucine between 79% and 198% is conferred.

**[00552.2.0.3] : see [00552.2.0.0]**

**[00553.0.3.3]**
1. A process for the production of leucine and/or isoleucine and/or valine, which comprises
   (a) increasing or generating the activity of a YDL127W, YDR245W, YDR271C, YER173W, YGR101W, YJL072C, YKR057W, YNL135C, YFL013C, YGR104C, YIL150C, YOR350C, YFR042W, YFL019C, b1708, b1829, b2957, b3366, b0828, b3966, b4151, b1827, b0124,b0149, b0161, b0486, b1313, b1343, b1463, b2022, b2414, b2664, b3117, b3256, b3938, b3983, b4054 and/or b4327 protein in a non-human organism, or in one or more parts thereof; and
   (b) growing the organism under conditions which permit the production of leucine and/or isoleucine and/or valine in said organism.
2. A process for the production of leucine and/or isoleucine and/or valine, comprising the increasing or generating in an organism or a part thereof the expression of at least one nucleic acid molecule comprising a nucleic acid molecule selected from the group consisting of:
   a) nucleic acid molecule encoding the polypeptide shown in Table IIA or IIB, columns 5 or 7, lines 19 to 29, 29a to 29u, 363 to 385 or a fragment thereof, which confers an increase in the amount of leucine and/or isoleucine and/or valine in an organism or a part thereof;
   b) nucleic acid molecule comprising of the nucleic acid molecule shown in Table IA or IB, columns 5 or 7, lines 19 to 29, 29a to 29u, 363 to 385 ;
   c) nucleic acid molecule whose sequence can be deduced from a polypeptide sequence encoded by a nucleic acid molecule of (a) or (b) as a result of the degeneracy of the genetic code and conferring an increase in the amount of leucine and/or isoleucine and/or valine in an organism or a part thereof;
   d) nucleic acid molecule which encodes a polypeptide which has at least 50% identity with the amino acid sequence of the polypeptide encoded by the nucleic acid molecule of (a) to (c) and conferring an increase in the amount of leucine and/or isoleucine and/or valine in an organism or a part thereof;
   e) nucleic acid molecule which hybidizes with a nucleic acid molecule of (a) to (c) under under stringent hybridisation conditions and conferring an increase in the amount of leucine and/or isoleucine and/or valine in an organism or a part thereof;
   f) nucleic acid molecule which encompasses a nucleic acid molecule which is obtained by amplifying nucleic acid molecules from a cDNA library or a genomic library using the primers shown in Table III, column 7, lines 19 to 29, 29a to 29u, 363 to 385 and conferring an increase in the amount of leucine and/or isoleucine and/or valine in an organism or a part thereof;
   g) nucleic acid molecule encoding a polypeptide which is isolated with the aid of monoclonal antibodies against a polypeptide encoded by one of the nucleic acid molecules of (a) to (f) and conferring an increase in the amount of leucine and/or isoleucine and/or valine in an organism or a part thereof;
   h) nucleic acid molecule encoding a polypeptide comprising the consensus sequence shown in Table IV, column 7, lines 19 to 29, 29a to 29u, 363 to 385 and conferring an increase in the amount of the fine chemical in an organism or a part thereof; and
   i) nucleic acid molecule which is obtainable by screening a suitable nucleic acid library under stringent hybridization conditions with a probe comprising one of the sequences of the nucleic acid molecule of (a) to (k) or with a fragment thereof having at least 15 nt, preferably 20 nt, 30 nt, 50 nt, 100 nt, 200 nt or 500 nt of the nucleic acid molecule characterized in (a) to (k) and conferring an increase in the amount of the fine chemical in an organism or a part thereof.
   or comprising a sequence which is complementary thereto.
3. The process of claim 1 or 2, comprising recovering of the free or bound leucine and/or isoleucine and/or valine.
4. The process of any one of claim 1 to 3, comprising the following steps:
   (a) selecting an organism or a part thereof expressing a polypeptide encoded by the nucleic acid molecule characterized in claim 2;
   (b) mutagenizing the selected organism or the part thereof;
   (c) comparing the activity or the expression level of said polypeptide in the mutagenized organism or the part thereof with the activity or the expression of said polypeptide of the selected organisms or the part thereof;
   (d) selecting the mutated organisms or parts thereof, which comprise an increased activity or expression level of said polypeptide compared to the selected organism or the part thereof;
   (e) optionally, growing and cultivating the organisms or the parts thereof; and
   (f) recovering, and optionally isolating, the free or bound leucine and/or isoleucine and/or valine produced by the selected mutated organisms or parts thereof.
5. The process of any one of claims 1 to 4, wherein the activity of said protein or the expression of said nucleic acid molecule is increased or generated transiently or stably.
6. An isolated nucleic acid molecule comprising a nucleic acid molecule selected from the group consisting of:
   a) nucleic acid molecule encoding the polypeptide shown in Table IIA or IIB, columns 5 or 7, lines 19 to 29, 29a to 29u, 363 to 385 or a fragment thereof, which confers an increase in the amount of leucine and/or isoleucine and/or valine in an organism or a part thereof;
   b) nucleic acid molecule comprising the nucleic acid molecule shown in Table IA or IB, columns 5 or 7, lines 19 to 29, 29a to 29u, 363 to 385;
   c) nucleic acid molecule whose sequence can be deduced from a polypeptide sequence encoded by a nucleic acid molecule of (a) or (b) as a result of the degeneracy of the genetic code and conferring an increase in the amount of leucine and/or isoleucine and/or valine in an organism or a part thereof;
   d) nucleic acid molecule which encodes a polypeptide which has at least 50% identity with the amino acid sequence of the polypeptide encoded by the nucleic acid molecule of (a) to (c) and conferring an increase in the amount of leucine and/or isoleucine and/or valine in an organism or a part thereof;
   e) nucleic acid molecule which hybidizes with a nucleic acid molecule of (a) to (c) under under stringent hybridisation conditions and conferring an increase in the amount of leucine and/or isoleucine and/or valine in an organism or a part thereof;
   f) nucleic acid molecule which encompasses a nucleic acid molecule which is obtained by amplifying nucleic acid molecules from a cDNA library or a genomic library using the primers shown in Table III, column 7, lines 19 to 29, 29a to 29u, 363 to 385 and conferring an increase in the amount of the fine chemical in an organism or a part thereof;
   g) nucleic acid molecule encoding a polypeptide which is isolated with the aid of monoclonal antibodies against a polypeptide encoded by one of the nucleic acid molecules of (a) to (f) and conferring an increase in the amount of leucine and/or isoleucine and/or valine in an organism or a part thereof;
   h) nucleic acid molecule encoding a polypeptide comprising the consensus sequence shown in Table IV, column 7, lines 19 to 29, 29a to 29u, 363 to 385 and conferring an increase in the amount of the fine chemical in an organism or a part thereof; and
   i) nucleic acid molecule which is obtainable by screening a suitable nucleic acid library under stringent hybridization conditions with a probe comprising one of the sequences of the nucleic acid molecule of (a) to (k) or with a fragment thereof having at least 15 nt, preferably 20 nt, 30 nt, 50 nt, 100 nt, 200 nt or 500 nt of the nucleic acid molecule characterized in (a) to (k) and conferring an increase in the amount of the fine chemical in an organism or a part thereof.
   whereby the nucleic acid molecule distinguishes over the sequence as shown in Table IA , columns 5 or 7, lines 19 to 29, 29a to 29u, 363 to 385 by one or more nucleotides.
7. A nucleic acid construct which confers the expression of the nucleic acid molecule of claim 6, comprising one or more regulatory elements.
8. A vector comprising the nucleic acid molecule as claimed in claim 6 or the nucleic acid construct of claim 7.
9. The vector as claimed in claim 8, wherein the nucleic acid molecule is in operable linkage with regulatory sequences for the expression in a prokaryotic or eukaryotic, or in a prokaryotic and eukaryotic, host.
10. A host cell, which has been transformed stably or transiently with the vector as claimed in claim 8 or 9 or the nucleic acid molecule as claimed in claim 6 or the nucleic acid construct of claim 7 or produced as described in claim any one of claims 2 to 5.
11. The host cell of claim 10, which is a transgenic host cell.
12. The host cell of claim 10 or 11, which is a plant cell, an animal cell, a microorganism, or a yeast cell, a fungus cell, a prokaryotic cell, an eukaryotic cell or an archaebacterium.
13. A process for producing a polypeptide, wherein the polypeptide is expressed in a host cell as claimed in any one of claims 10 to 12.
14. A polypeptide produced by the process as claimed in claim 13 or encoded by the nucleic acid molecule as claimed in claim 6 whereby the polypeptide distinguishes over the sequence as shown in Table IIA, columns 5 or 7, lines 19 to 29, 29a to 29u, 363 to 385 by one or more amino acids
15. An antibody, which binds specifically to the polypeptide as claimed in claim 14.
16. A plant tissue, propagation material, harvested material or a plant comprising the host cell as claimed in claim 12 which is plant cell or an Agrobacterium.
17. A method for screening for agonists and antagonists of the activity of a polypeptide encoded by the nucleic acid molecule of claim 6 conferring an increase in the amount of leucine and/or isoleucine and/or valine in an organism or a part thereof comprising:
   (a) contacting cells, tissues , plants or microorganisms which express the a polypeptide encoded by the nucleic acid molecule of claim 6 conferring an increase in the amount of leucine and/or isoleucine and/or valine in an organism or a part thereof with a candidate compound or a sample comprising a plurality of compounds under conditions which permit the expression the polypeptide;
   (b) assaying the leucine and/or isoleucine and/or valine level or the polypeptide expression level in the cell, tissue, plant or microorganism or the media the cell, tissue, plant or microorganisms is cultured or maintained in; and
   (c) identifying a agonist or antagonist by comparing the measured leucine and/or isoleucine and/or valine level or polypeptide expression level with a standard leucine and/or isoleucine and/or valine or polypeptide expression level measured in the absence of said candidate compound or a sample comprising said plurality of compounds, whereby an increased level over the standard indicates that the compound or the sample comprising said plurality of compounds is an agonist and a decreased level over the standard indicates that the compound or the sample comprising said plurality of compounds is an antagonist.
18. A process for the identification of a compound conferring increased leucine and/or isoleucine and/or valine production in a plant or microorganism, comprising the steps:
   (a) culturing a plant cell or tissue or microorganism or maintaining a plant expressing the polypeptide encoded by the nucleic acid molecule of claim 6 conferring an increase in the amount of leucine and/or isoleucine and/or valine in an organism or a part thereof and a readout system capable of interacting with the polypeptide under suitable conditions which permit the interaction of the polypeptide with dais readout system in the presence of a compound or a sample comprising a plurality of compounds and capable of providing a detectable signal in response to the binding of a compound to said polypeptide under conditions which permit the expression of said readout system and of the polypeptide encoded by the nucleic acid molecule of claim 6 conferring an increase in the amount of leucine and/or isoleucine and/or valine in an organism or a part thereof;
   (b) identifying if the compound is an effective agonist by detecting the presence or absence or increase of a signal produced by said readout system.
19. A method for the identification of a gene product conferring an increase in leucine and/or isoleucine and/or valine production in a cell, comprising the following steps:
   (a) contacting the nucleic acid molecules of a sample, which can contain a candidate gene encoding a gene product conferring an increase in leucine and/or isoleucine and/or valine after expression with the nucleic acid molecule of claim 6;
   (b) identifying the nucleic acid molecules, which hybridise under relaxed stringent conditions with the nucleic acid molecule of claim 6;
   (c) introducing the candidate nucleic acid molecules in host cells appropriate for producing leucine and/or isoleucine and/or valine;
   (d) expressing the identified nucleic acid molecules in the host cells;
   (e) assaying the leucine and/or isoleucine and/or valine level in the host cells; and
   (f) identifying nucleic acid molecule and its gene product which expression confers an increase in the leucine and/or isoleucine and/or valine level in the host cell in the host cell after expression compared to the wild type.
20. A method for the identification of a gene product conferring an increase in leucine and/or isoleucine and/or valine production in a cell, comprising the following steps:
   (a) identifiying in a data bank nucleic acid molecules of an organism; which can contain a candidate gene encoding a gene product conferring an increase in the leucine and/or isoleucine and/or valine amount or level in an organism or a part thereof after expression, and which are at least 20% homolog to the nucleic acid molecule of claim 6;
   (b) introducing the candidate nucleic acid molecules in host cells appropriate for producing leucine and/or isoleucine and/or valine ;
   (c) expressing the identified nucleic acid molecules in the host cells;
   (d) assaying the leucine and/or isoleucine and/or valine level in the host cells; and
   (e) identifying nucleic acid molecule and its gene product which expression confers an increase in the leucine and/or isoleucine and/or valine level in the host cell after expression compared to the wild type.
21. A method for the production of an agricultural composition comprising the steps of the method of any one of claims 17 to 20 and formulating the compound identified in any one of claims 17 to 20 in a form acceptable for an application in agriculture.
22. A composition comprising the nucleic acid molecule of claim 6, the polypeptide of claim 14, the nucleic acid construct of claim 7, the vector of any one of claims 8 or 9, an antagonist or agonist identified according to claim 17, the compound of cliam 18, the gene product of claim 19 or 20, the antibody of claim 15, and optionally an agricultural acceptable carrier.
23. Use of the nucleic acid molecule as claimed in claim 6 for the identification of a nucleic acid molecule conferring an increase of leucine and/or isoleucine and/or valine after expression.
24. Use of the polypeptide of claim 14 or the nucleic acid construct claim 7 or the gene product identified according to the method of claim 19 or 20 for identifying compounds capable of conferring a modulation of leucine and/or isoleucine and/or valine levels in an organism.
25. Food or feed composition comprising the nucleic acid molecule of claim 6, the polypeptide of claim 14, the nucleic acid construct of claim 7, the vector of claim 8 or 9, the antagonist or agonist identified according to claim 17, the antibody of claim 15, the plant or plant tissue of claim 16, the harvested material of claim 16, the host cell of claim 10 to 12 or the gene product identified according to the method of claim 19 or 20.
26. Use of the nucleic acid molecule of claim 6, the polypeptide of claim 14, the nucleic acid construct of claim 7, the vector of claim 8 or 9, the antagonist or agonist identified according to claim 17, the antibody of claim 15, the plant or plant tissue of claim 16, the host cell of claim 10 to 12 or the gene product identified according to the method of claim 19 or 20 for the protection of a plant against a leucine and/or isoleucine and/or valine synthesis inhibiting herbicide.

**[00554.0.0.3] Abstract: see [00554.0.0.0]**
Process for the production of fine chemicals

**[0000.0.0.4]** In a further embodiment, the present invention relates to a further process for the production of fine chemicals as defined below and corresponding embodiments as described herein as follows.

**[0001.0.0.4] to [0008.0.0.4]: see [0001.0.0.0] to [0008.0.0.0]**

**[0009.0.4.4]** As described above, the essential amino acids are necessary for humans and many mammals, for example for livestock. Arginine is a semi-essential amino acid involved in multiple areas of human physiology and metabolism. It is not considered essential because humans can synthesize it de novo from glutamine, glutamate, and proline. However, dietary intake remains the primary determinant of plasma arginine levels, since the rate of arginine biosynthesis does not increase to compensate for depletion or inadequate supply. Dietary arginine intake regulates whole body arginine synthesis from proline in the neonatal piglet. The maximal rate of arginine synthesis (0.68g/kg/d) is not enough to supply the whole body metabolic requirement for arginine in the young pig. In animals, glutamate functions as a neurotransmitter and activates glutamate receptor cation channels (iGluRs), which trigger electrical or Ca²⁺ signal cascades. In plants, amino acids are involved in signalling of both plant nitrogen status and plant nitrogen: carbon ratios. Endogenous glutamine has been implicated in feedback inhibition of root N uptake, via the suppression of transcription of genes encoding inorganic nitrogen transporters (Rawat et al., Plant Journal 19: 143-152, 1999; Zhuo et al., Plant Journal 17: 563-568, 1999). The nonessential amino acid, proline, is synthesized from L-ornithine or L-glutamate. The proline from L-ornithine is linked to protein metabolism in the urea cycle and the proline from L-glutamate is linked to carbohydrate metabolism. Collagen is the major reservoir for proline in the body. Vitamin C should be used with proline for collagen problems.

**[0010.0.0.4] to [0011.0.0.4]: see [0010.0.0.0] to [0011.0.0.0]:**

**[0012.0.4.4]** It is an object of the present invention to develop an inexpensive process for the synthesis of arginine and/or glutamate and/or glutamine and/or proline, preferably L- arginine and/or L- glutamate and/or L-glutamine and/or L- proline.

**[0013.0.0.4]:** see **[0013.0.0.0]**

**[0014.0.4.4]** Accordingly, in a first embodiment, the invention relates to a process for the production of a fine chemical, whereby the fine chemical is arginine and/or glutamate and/or glutamine and/or proline, preferably L- arginine and/or L- glutamate and/or L-glutamine and/or L- proline. Accordingly, in the present invention, the term "the fine chemical" as used herein relates to "arginine and/or glutamate and/or glutamine and/or proline". Further, the term "the fine chemicals" as used herein also relates to fine chemicals comprising arginine and/or glutamate and/or glutamine and/or proline.

**[0015.0.4.4]** In one embodiment, the term "the fine chemical" means arginine and/or glutamate and/or glutamine and/or proline, preferably L- arginine and/or L- glutamate and/or L-glutamine and/or L- proline. Throughout the specification the term "the fine chemical" means arginine and/or glutamate and/or glutamine and/or proline, preferably L- arginine and/or L- glutamate and/or L-glutamine and/or L- proline, its salts, ester or amids in free form or bound to proteins. In a preferred embodiment, the term "the fine chemical" means arginine and/or glutamate and/or glutamine and/or proline, preferably L- arginine and/or L- glutamate and/or L-glutamine and/or L- proline, in free form or its salts or bound to proteins.

**[0016.0.4.4]** Accordingly, the present invention relates to a process comprising
(a) increasing or generating the activity of one or more
   YDR316W, YHR130C, YKR057W, YNL090W, b1829, b0695, b1284, b2095, b0161, b2307 and/or b3936 - protein(s) or of a protein having the sequence of a polypeptide encoded by a nucleic acid molecule indicated in Table II, columns 5 or 7, lines 30 to 37, 390, 405 and/or 430;
   in a non-human organism in one or more parts thereof and
(b) growing the organism under conditions which permit the production of the fine chemical, meaning of arginine or fine chemicals comprising arginine in said organism;
or
(a) increasing or generating the activity of one or more
   YBR204C, YFL013C, YGR104C, YPR024W, YPR133W-A, b0730, b0050, b0057, b0161, b1343, b1693, b1736, b1738, b1896, b2307, b2710, b2818, b3074, b3116, b3169, b3619, b3791, b4346, and/or YFL019C - protein(s) or of a protein having the sequence of a polypeptide encoded by a nucleic acid molecule indicated in Table II, columns 5 or 7, lines 38 to 43, 386, 387, 391, 396, 399 to 401, 403, 406, 413, 414, 417, 418, 421, 424, 427, 434 and/or 435;
   in a non-human organism in one or more parts thereof and
(b) growing the organism under conditions which permit the production of the fine chemical, meaning of glutamate or fine chemicals comprising glutamate in said organism;
or
(a) increasing or generating the activity of one or more
   YBR030W, YDL106C, YDR271C, YEL045C, YER173W, YFL050C, YGR135W, YIL150C, YNL090W, YPR138C, b0730, b2699, b1827, b0138, b0149, b1360, b2553, b2664, b3644 and/or b3919 - protein(s) or of a protein having the sequence of a polypeptide encoded by a nucleic acid molecule indicated in Table II, columns 5 or 7, lines 44 to 56, 388, 389, 398, 411, 412, 425 and/or 429;
   in a non-human organism in one or more parts thereof and
(b) growing the organism under conditions which permit the production of the fine chemical, meaning of proline or fine chemicals comprising proline in said organism;
or
(a) increasing or generating the activity of one or more
   YER173W, YFR042W, YKR057W, b1829, b1852, b4265, b0161, b0486, b0849, b0970, b1343, b1886, b1926, b2414, b2426, b2489, b2553, b2818, b3064, b3160, b3166, b3169, b3231, b3680, b3719, b4004, b4074 and/or b4133 - protein(s) or of a protein having the sequence of a polypeptide encoded by a nucleic acid molecule indicated in Table II, columns 5 or 7, lines 57 to 62, 392 to 395, 397, 402, 404, 407 to 410, 415, 416, 419, 420, 422, 423, 426, 428 and/or 431 to 433;
   in a non-human organism in one or more parts thereof and
(b) growing the organism under conditions which permit the production of the fine chemical, meaning glutamine or fine chemicals comprising glutamine in said organism.
Accordingly, the present invention relates to a process for the production of a fine chemical comprising
(a) increasing or generating the activity of one or more proteins having the activity of a protein indicated in Table II, column 3, lines 30 to 62 and/or lines 386 to 435 or having the sequence of a polypeptide encoded by a nucleic acid molecule indicated in Table I, column 5 or 7, lines 30 to 62 and/or lines 386 to 435, in a non-human organism in one or more parts thereof and
(b) growing the organism under conditions which permit the production of the fine chemical, in particular arginine and/or glutamate and/or glutamine and/or proline resp..

**[0016.1.4.4]** Accordingly, the term "the fine chemical" means in one embodiment "arginine" in relation to all sequences listed in Table I to IV, lines 30 to 37, 390, 405 and/or 430 or homologs thereof and means in one embodiment "glutamate" in relation to all sequences listed in Tables I to IV, lines 38 to 43, 386, 387, 391, 396, 399 to 401, 403, 406, 413, 414, 417, 418, 421, 424, 427, 434 and/or 435 or homologs thereof and means in one embodiment "proline" in relation to all sequences listed in Table I to IV, lines 44 to 56, 388, 389, 398, 411, 412, 425 and/or 429 or homologs thereof and means in one embodiment "glutamine" in relation to all sequences listed in Tables I to IV, lines 57 to 62, 392 to 395, 397, 402, 404, 407 to 410, 415, 416, 419, 420, 422, 423, 426, 428 and/or 431 to 433 or homologs thereof.
Accordingly, in one embodiment the term "the fine chemical" means "glutamate" and "proline" in relation to all sequences listed in Table I to IV, lines 43 and 54, in one embodiment the term "the fine chemical" means "arginine" and "glutamine" in relation to all sequences listed in Table I to IV, lines 32 and 59, and, lines 34 and 60, and, 390 and 392,
in one embodiment the term "the fine chemical" means "glutamine" and "proline" in relation to all sequences listed in Table I to IV, lines 57 and 48, and, 410 and 411,
in one embodiment the term "the fine chemical" means "arginine" and "glutamate" in relation to all sequences listed in Table I to IV, lines 390 and 391, and, 405 and 406. in one embodiment the term "the fine chemical" means "glutamate" and "glutamine" in relation to all sequences listed in Table I to IV, lines 391 and 392, and, 396 and 397, and, 414 and 415, and, 421 and 422, and, 427 and 428,
in one embodiment the term "the fine chemical" means "arginine" and "glutamate" and "glutamine" in relation to all sequences listed in Table I to IV, lines 390 and 391 and 392.
Accordingly, the term "the fine chemical" can mean "arginine" and/or "glutamate" and/or "glutamine" and/or "proline", owing to circumstances and the context. In order to illustrate that the meaning of the term "the fine chemical" means "arginine", and/or "glutamate" and/or "glutamine" and/or "proline" the term "the respective fine chemical" is also used.

**[0017.0.0.4] to [0018.0.0.4]: see [0017.0.0.0]to [0018.0.0.0]**

**[0019.0.4.4]** Advantageously the process for the production of the fine chemical leads to an enhanced production of the fine chemical. The terms "enhanced" or "increase" mean at least a 10%, 20%, 30%, 40% or 50%, preferably at least 60%, 70%, 80%, 90% or 100%, more preferably 150%, 200%, 300%, 400% or 500% higher production of the respective fine chemical in comparison to the reference as defined below, e.g. that means in comparison to an organism without the aforementioned modification of the activity of a protein indicated in Table II, column 3, lines 30 to 62 and/or lines 386 to 435 or encoded by nucleic acid molecule indicated in Table I, columns 5 or 7, lines 30 to 62 and/or lines 386 to 435.

**[0020.0.4.4]** Surprisingly it was found, that the transgenic expression of at least one of the Saccaromyces cerevisiae protein(s) indicated in Table II, Column 3, lines 30 to 33 for arginine
and/or lines 38 to 42 and/or 435 for glutamate
and/or lines 44 to 53 for proline
and/or lines 57 to 59 for glutamine
in Arabidopsis thaliana conferred an increase in the respective fine chemical content of the transformed plants
and/or
at least one of the Escherichia coli K12 proteins indicated in Table II, Column 3, lines 34 to 37, 390, 405 and/or 430 for arginine
and/or lines 43, 386, 387, 391, 396, 399 to 401, 403, 406, 413, 414, 417, 418, 421, 424, 427 and/or 434 for glutamate
and/or lines 54 to 56, 388, 389, 398, 411, 412, 425 and/or 429 for proline
and/or lines 62, 392 to 395, 397, 402, 404, 407 to 410, 415, 416, 419, 420, 422, 423, 426, 428 and/or 431 to 433 for glutamine
in Arabidopsis thaliana conferred an increase in the respective fine chemical content of the transformed plants.
Accordingly, it was surprisingly found, that the transgenic expression of the Escherichia coli K12 protein as indicated in Table II, column 5, lines 43 and 54 in Arabidopsis thaliana conferred an increase in glutamate and/or proline (or the respective fine chemical) content of the transformed plants. Thus, in one embodiment, said protein or its homologs are used for the production of glutamate; in one embodiment, said protein or its homologs are used for the production of proline; in one embodiment, said protein or its homologs are used for the production of one or more fine chemical selected from the group consisting of: glutamate and/or proline.
Accordingly, it was surprisingly found, that the transgenic expression of the Escherichia coli K12 protein as indicated in Table II, column 5, lines 43 and 54 and/or lines 390 and 392 and/or the Saccharomyces cerevisiae protein as indicated in Table II, column 5, lines 32 and 59 in Arabidopsis thaliana conferred an increase in arginine and/or glutamine (or the respective fine chemical) content of the transformed plants. Thus, in one embodiment, said protein or its homologs are used for the production of arginine; in one embodiment, said protein or its homologs are used for the production of glutamine; in one embodiment, said protein or its homologs are used for the production of one or more fine chemical selected from the group consisting of: arginine and/or glutamine.
Surprisingly it was found, that the transgenic expression of the Saccharomyces cerevisiae protein as indicated in Table II, column 5, lines 48 and 57 and/or the Escherichia coli K12 protein as indicated in Table II, column 5, lines 411 and 410 in Arabidopsis thaliana conferred an increase in proline and/or glutamine (or the respective fine chemical) content of the transformed plants. Thus, in one embodiment, said protein or its homologs are used for the production of proline; in one embodiment, said protein or its homologs are used for the production of glutamine, in one embodiment, said protein or its homologs are used for the production of one or more fine chemical selected from the group consisting of: proline and/or glutamine.
Surprisingly it was found, that the transgenic expression of the the Escherichia coli K12 protein as indicated in Table II, column 5, lines 391 and 392 and/or lines 396 and 397 and/or lines 414 and 415 and/or lines 421 and 422 and/or lines 427 and 428 in Arabidopsis thaliana conferred an increase in glutamate and/or glutamine (or the respective fine chemical) content of the transformed plants. Thus, in one embodiment, said protein or its homologs are used for the production of glutamate; in one embodiment, said protein or its homologs are used for the production of glutamine, in one embodiment, said protein or its homologs are used for the production of one or more fine chemical selected from the group consisting of: glutamate and/or glutamine.
Surprisingly it was found, that the transgenic expression of the Escherichia coli K12 protein as indicated in Table II, column 5, lines 390 and 391, and, 405 and 406 in Arabidopsis thaliana conferred an increase in arginine and/or glutamate (or the respective fine chemical) content of the transformed plants. Thus, in one embodiment, said protein or its homologs are used for the production of arginine, in one embodiment, said protein or its homologs are used for the production of glutamate, in one embodiment, said protein or its homologs are used for the production of arginine and glutamate.
Surprisingly it was found, that the transgenic expression of the Escherichia coli K12 protein as indicated in Table II, column 5, lines 390 and 391 and 392 in Arabidopsis thaliana conferred an increase in arginine and/or glutamate and/or glutamine (or the respective fine chemical) content of the transformed plants. Thus, in one embodiment, said protein or its homologs are used for the production of arginine, in one embodiment, said protein or its homologs are used for the production of glutamate; in one embodiment, said protein or its homologs are used for the production of glutamine, in one embodiment, said protein or its homologs are used for the production of one or more fine chemical selected from the group consisting of: arginine and/or glutamate and/or glutamine.

**[0021.0.0.4]: see [0021.0.0.0]**

**[0022.0.4.4]** The sequence of b0695 from Escherichia coli K12 has been published in Blattner et al., Science 277(5331), 1453-1474, 1997, and its activity is being defined as sensory histidine kinase in two-component regulatory system. Accordingly, in one embodiment, the process of the present invention comprises the use of a gene product with an activity of the sensor histidine kinase homology superfamily, preferably a protein with a sensory histidine kinase in two-component regulatory system activity from E. coli or its homolog, e.g. as shown herein, for the production of the fine chemical, meaning of arginine , in particular for increasing the amount of arginine , preferably arginine in free or bound form in an organism or a part thereof, as mentioned.
The sequence of b0730 from Escherichia coli K12 has been published in Blattner et al., Science 277(5331), 1453-1474, 1997, and its activity is being defined as transcriptional regulator of succinylCoA synthetase operon and fatty acyl response regulator.
Accordingly, in one embodiment, the process of the present invention comprises the use of a gene product with an activity of the transcription regulator GntR superfamily, preferably a protein with a transcriptional regulator of succinylCoA synthetase operon or a fatty acid response regulator activity from E. coli or its homolog, e.g. as shown herein, for the production of the fine chemical, meaning of glutamate and/or proline, in particular for increasing the amount of glutamate and/or proline, in particular for increasing the amount of glutamate, in particular for increasing the amount of proline, in particular for increasing the amount of glutamate and proline, preferably glutamate and/or proline in free or bound form in an organism or a part thereof, as mentioned.
The sequence of b1284 from Escherichia coli K12 has been published in Blattner et al., Science 277(5331), 1453-1474, 1997, and its activity is being defined as a putative transcriptional regulator with DNA-binding Winged helix domain (DeoR family). Accordingly, in one embodiment, the process of the present invention comprises the use of a gene product with an activity of the regulatory protein gutR superfamily, preferably a protein with transcriptional regulator with DNA-binding Winged helix domain (DeoR family) activity from E. coli or its homolog, e.g. as shown herein, for the production of the fine chemical, meaning of arginine , iin particular for increasing the amount of arginine , preferably arginine in free or bound form in an organism or a part thereof, as mentioned.
The sequence of b1827 from Escherichia coli K12 has been published in Blattner et al., Science 277(5331), 1453-1474, 1997, and its activity is being defined as a putative transcriptional repressor protein with a DNA-binding Winged helix domain (IcIR family). Accordingly, in one embodiment, the process of the present invention comprises the use of a gene product with an activity of the acetate operon repressor superfamily, preferably a protein with a transcriptional repressor protein with a DNA-binding Winged helix domain (IcIR family) activity from E. coli or its homolog, e.g. as shown herein, for the production of the fine chemical, meaning of arginine , in particular for increasing the amount of arginine , preferably arginine in free or bound form in an organism or a part thereof, as mentioned.
The sequence of b1829 from Escherichia coli K12 has been published in Blattner et al., Science 277(5331), 1453-1474, 1997, and its activity is being defined as a heat shock protein with protease activity. Accordingly, in one embodiment, the process of the present invention comprises the use of a gene product with an activity of heat-shock protein htpX superfamily, preferably a protein with a "heat shock protein with protease activity" from E. coli or its homolog, e.g. as shown herein, for the production of the fine chemical, meaning of glutamine and/or proline, in particular for increasing the amount of arginine and/or glutamine, in particular for increasing the amount of proline, in particular for increasing the amount of glutamine, in particular for increasing the amount of proline and glutamine, preferably increasing the amount of proline and/or glutamine, in free or bound form in an organism or a part thereof, as mentioned. In one embodiment, in the process of the present invention the activity of a heat shock protein with protease activity is increased or generated, e.g. from E. coli or a homolog thereof.
The sequence of b1852 from Escherichia coli K12 has been published in Blattner et al., Science 277(5331), 1453-1474, 1997, and its activity is being defined as a glucose-6-phosphate dehydrogenase. Accordingly, in one embodiment, the process of the present invention comprises the use of a gene product with an activity of glucose-6-phosphate dehydrogenase superfamily, preferably a protein with a glucose-6-phosphate dehydrogenase activity from E. coli or its homolog, e.g. as shown herein, for the production of the fine chemical, meaning of glutamine , in particular for increasing the amount of glutamine , preferably increasing the amount of glutamine in free or bound form in an organism or a part thereof, as mentioned.
The sequence of b2095 from Escherichia coli K12 has been published in Blattner et al., Science 277(5331), 1453-1474, 1997, and its activity is being defined as a tagatose-6-phosphate kinase. Accordingly, in one embodiment, the process of the present invention comprises the use of a gene product with an activity of Escherichia probable tagatose 6-phosphate kinase gatZ superfamily, preferably a protein with a tagatose-6-phosphate kinase activity from E. coli or its homolog, e.g. as shown herein, for the production of the fine chemical, meaning of arginine , in particular for increasing the amount of arginine, preferably increasing the amount of arginine in free or bound form in an organism or a part thereof, as mentioned.
The sequence of b2699 from Escherichia coli K12 has been published in Blattner et al., Science 277(5331), 1453-1474, 1997, and its activity is being defined as a DNA strand exchange and recombination protein with protease and nuclease activity. Accordingly, in one embodiment, the process of the present invention comprises the use of a gene product with an activity of recombination protein recA superfamily, preferably a protein with a DNA strand exchange and recombination protein with protease and nuclease activity from E. coli or its homolog, e.g. as shown herein, for the production of the fine chemical, meaning of proline, in particular for increasing the amount of proline, preferably increasing the amount of proline in free or bound form in an organism or a part thereof, as mentioned.
The sequence of b4265 from Escherichia coli K12 has been published in Blattner et al., Science 277(5331), 1453-1474, 1997, and its activity is being defined as a L-idonate transport protein. Accordingly, in one embodiment, the process of the present invention comprises the use of a gene product with an activity of D-serine permease superfamily, preferably a protein with a L-idonate transport protein activity from E. coli or its homolog, e.g. as shown herein, for the production of the fine chemical, meaning of glutamine , in particular for increasing the amount of glutamine , preferably increasing the amount of glutamine in free or bound form in an organism or a part hereof, as mentioned.
The sequence of YBR030W from Saccharomyces cerevisiae has been published in Feldmann et al., EMBO J., 13 (24), 5795-5809 (1994) and Goffeau, Science 274 (5287), 546-547, 1996, and its cellular activity has not been characterized yet. Accordingly, in one embodiment, the process of the present invention comprises the use of a gene product with an activity of Saccharomyces cerevisiae hypothetical protein YBR030w superfamily, preferably a protein with a YBR030W activity from Saccharomyces cerevisiae or its homolog, e.g. as shown herein, for the production of the fine chemical, meaning of proline, in particular for increasing the amount of proline, preferably proline in free or bound form in an organism or a part thereof, as mentioned.
The sequence of YDL106C from Saccharomyces cerevisiae has been published in Jacq et al., Nature 387 (6632 Suppl), 75-78, 1997, and Goffeau, Science 274 (5287), 546-547, 1996, and its activity is being defined as homeobox transcription factor. Accordingly, in one embodiment, the process of the present invention comprises the use of a gene product with an activity of unassigned homeobox proteins, homeobox homology proteins superfamily, preferably a protein with a "homeobox transcription factor" activity or its homolog, e.g. as shown herein, for the production of the fine chemical, meaning of proline, in particular for increasing the amount of proline , preferably proline in free or bound form in an organism or a part thereof, as mentioned.
The sequence of YFR042W from Saccharomyces cerevisiae has been published in Goffeau st al., Science 274 (5287), 546-547, 1996 and Murakami,Y., Nat. Genet. 10 (3), 261-268, 1995 and its activity is being defined as a "protein required for cell viability in yeast". Accordingly, in one embodiment, the process of the present invention comprises the use of a gene product with an activity of Saccharomyces cerevisiae probable membrane protein YFR042w superfamily, preferably a protein with a "protein required for cell viability in yeast" activity, from Saccharomyces cerevisiae or its homolog, e.g. as shown herein, for the production of the fine chemical, meaning of glutamine , in particular for increasing the amount of glutamine, preferably glutamine in free or bound form in an organism or a part thereof, as mentioned.
The sequence of YGR135W from Saccharomyces cerevisiae has been published in Goffeau st al., Science 274 (5287), 546-547, 1996 and Tettelin et al., Nature 387 (6632 Suppl), 81-84 (1997) and its activity is being defined as a "proteasome component Y13". Accordingly, in one embodiment, the process of the present invention comprises the use of a a gene product with an activity of multicatalytic endopeptidase complex chain C9 superfamily, preferably a protein with proteasome component Y13 activity, from Saccharomyces cerevisiae or its homolog, e.g. as shown herein, for the production of the fine chemical, meaning of proline, in particular for increasing the amount of proline, preferably proline in free or bound form in an organism or a part thereof, as mentioned.
The sequence of YHR130C from Saccharomyces cerevisiae has been published in Johnston et al., Science 265:2077-2082 (1994), and its cellular activity has not been characterized yet. Accordingly, in one embodiment, the process of the present invention comprises the use of a YHR130C activity from Saccharomyces cerevisiae or its homolog, e.g. as shown herein, for the production of the fine chemical, meaning of arginine , in particular for increasing the amount of arginine , preferably arginine in free or bound form in an organism or a part thereof, as mentioned.
The sequence of YIL150C from Saccharomyces cerevisiae has been published in Goffeau st al., Science 274 (5287), 546-547, 1996 and Churcher et al., Nature 387 (6632 Suppl), 84-87, 1997 and its activity is being defined as a chromatin binding protein, required for S-phase (DNA synthesis) initiation or completion. Accordingly, in one embodiment, the process of the present invention comprises the use of a chromatin binding protein, required for S-phase (DNA synthesis) initiation or completion, from Saccharomyces cerevisiae or its homolog, e.g. as shown herein, for the production of the fine chemical, meaning of proline, in particular for increasing the amount of proline, preferably proline in free or bound form in an organism or a part thereof, as mentioned.
The sequence of YPR024W from Saccharomyces cerevisiae has been published in Goffeau st al., Science 274 (5287), 546-547, 1996 and Bussey et al., Nature 387 (6632 Suppl), 103-105 (1997) and its activity is being defined as a mitochondrial protein of the CDC48/PAS1/SEC18 family of ATPases. Accordingly, in one embodiment, the process of the present invention comprises the use of a gene product with an activity of FtsH/SEC18/CDC48-type ATP-binding domain homology; cell division protein ftsH superfamily, preferably a protein with a mitochondrial protein of the CDC48/PAS1/SEC18 family of ATPases activity, from Saccharomyces cerevisiae or its homolog, e.g. as shown herein, for the production of the fine chemical, meaning of glutamate , in particular for increasing the amount of glutamate , preferably glutamate in free or bound form in an organism or a part thereof, as mentioned.
The sequence of YPR133W-A from Saccharomyces cerevisiae has been published in Goffeau st al., Science 274 (5287), 546-547, 1996 and Bussey et al., Nature 387 (6632 Suppl), 103-105 (1997) and its activity is being defined as a translocase of the outer mitochondrial membrane. Accordingly, in one embodiment, the process of the present invention comprises the use of a translocase of the outer mitochondrial membrane, from Saccharomyces cerevisiae or its homolog, e.g. as shown herein, for the production of the fine chemical, meaning of glutamate , in particular for increasing the amount of glutamate , preferably glutamate in free or bound form in an organism or a part thereof, as mentioned.
The sequence of YPR138C from Saccharomyces cerevisiae has been published in Goffeau st al., Science 274 (5287), 546-547, 1996 and Bussey et al., Nature 387 (6632 Suppl), 103-105 (1997) and its activity is being defined as a NH⁴⁺ transporter. Accordingly, in one embodiment, the process of the present invention comprises the use of a gene product with an activity of ammonium transport protein; ammonium transporter nrgA superfamily, preferably a protein with a NH⁴⁺ transporter activity, from Saccharomyces cerevisiae or its homolog, e.g. as shown herein, for the production of the fine chemical, meaning of proline, in particular for increasing the amount of proline, preferably proline in free or bound form in an organism or a part thereof, as mentioned.
The sequence of YBR204C from Saccharomyces cerevisiae has been published in Goffeau st al., Science 274 (5287), 546-547, 1996 and Feldmann et al., EMBO J. 13 (24), 5795-5809 (1994) and its activity is being defined as a peroxisomal lipase.
Accordingly, in one embodiment, the process of the present invention comprises the use of a peroxisomal lipase, from Saccharomyces cerevisiae or its homolog, e.g. as shown herein, for the production of the fine chemical, meaning of glutamate , in particular for increasing the amount of glutamate , preferably glutamate in free or bound form in an organism or a part thereof, as mentioned.
The sequence of YDR271C was submitted by Le T., Johnston M., (March-1996) to the EMBL/GenBank/DDBJ databases, by Waterston R.;(MAY-1996) and Jia Y., (JUNE-1997) to the EMBL/GenBank/DDBJ databases and its cellular activity has not been characterized yet. Accordingly, in one embodiment, the process of the present invention comprises the use of a YDR271C activity from Saccharomyces cerevisiae or its homolog, e.g. as shown herein, for the production of the fine chemical, meaning of proline, in particular for increasing the amount of proline, preferably proline in free or bound form in an organism or a part thereof, as mentioned. I
The sequence of YDR316W from Saccharomyces cerevisiae has been published in Goffeau st al., Science 274 (5287), 546-547, 1996 and Jacq et al., Nature 387 (6632 Suppl), 75-78 (1997), and its activity is being defined as a putative S-adenosylmethionine-dependent methyltransferase of the seven beta-strand family. Accordingly, in one embodiment, the process of the present invention comprises the use of a a gene product with an activity of bioC homology superfamily, preferably a protein with putative S-adenosylmethionine-dependent methyltransferase of the seven beta-strand family activity, from Saccharomyces cerevisiae or its homolog, e.g. as shown herein, for the production of the fine chemical, meaning of arginine in particular for increasing the amount of arginine , preferably arginine in free or bound form in an organism or a part thereof, as mentioned.
The sequence of YEL045C from Saccharomyces cerevisiae was published by Dietrich et al., Nature 387:78-81(1997) and its cellular activity has not been characterized yet. Accordingly, in one embodiment, the process of the present invention comprises the use of a gene product with an activity of Saccharomyces hypothetical protein YEL045c superfamily, preferably a protein with a YEL045C activity from Saccharomyces cerevisiae or its homolog, e.g. as shown herein, for the production of the fine chemical, meaning of proline, in particular for increasing the amount of proline, preferably proline in free or bound form in an organism or a part thereof, as mentioned.
The sequence of YER173w from Saccharomyces cerevisiae has been published in Dietrich, Nature 387 (6632 Suppl), 78-81, 1997, and Goffeau, Science 274 (5287), 546-547, 1996, and its activity is being defined as an "Checkpoint protein, involved in the activation of the DNA damage and meiotic pachytene checkpoints;". Accordingly, in one embodiment, the process of the present invention comprises the use of a "Checkpoint protein, involved in the activation of the DNA damage and meiotic pachytene checkpoints" or its homolog, e.g. as shown herein, for the production of the fine chemical, meaning the amount of glutamine and/or proline, in particular for increasing the amount of glutamine and/or proline, in particular for increasing the amount of glutamine, in particular for increasing the amount of proline, in particular for increasing the amount of glutamine and proline, preferably glutamine and/or proline in free or bound form in an organism or a part thereof, as mentioned.
The sequence of YFL013C from Saccharomyces cerevisiae has been published in Goffeau, A., Science 274 (5287), 546-547, 1996 and Murakami, Y., Nat. Genet. 10 (3), 261-268, 1995, and its activity is being defined as a "subunit of the INO80 chromatin remodeling complex". Accordingly, in one embodiment, the process of the present invention comprises the use of a gene product with an activity of Saccharomyces cerevisiae probable membrane protein YFL013c superfamily, preferably a protein with a "subunit of the INO80 chromatin remodeling complex" activity or its homolog, for the production of the fine chemical, meaning of glutamate , in particular for increasing the amount of glutamate , preferably glutamate in free or bound form in an organism or a part thereof, as mentioned.
The sequence of YFL050C from Saccharomyces cerevisiae has been published in Murakami et al., , Nat. Genet. 10 (3), 261-268, 1995 , and Goffeau et al., Science 274 (5287), 546-547, 1996, and its activity is defined as a di- trivalent inorganic cation transporter. Accordingly, in one embodiment, the process of the present invention comprises the use of a a gene product with an activity of magnesium and cobalt transport protein superfamily, preferably a protein with with a di- trivalent inorganic cation transporter activity from Saccaromyces cerevisiae or its homolog, e.g. as shown herein, for the production of the fine chemical, meaning proline, in particular for increasing the amount of proline, preferably proline in free or bound form in an organism or a part thereof, as mentioned.
The sequence of YGR104C from Saccharomyces cerevisiae has been published in Thompson et al., Cell 73:1361-1375, 1993, and its activity is being defined as an "RNA polymerase II suppressor protein SRB5 - yeast and/or suppressor of RNA polymerase B SRB5" ". Accordingly, in one embodiment, the process of the present invention comprises the use of a gene product with an activity of RNA polymerase II suppressor protein SRB5 - yeast superfamily, preferably a protein with a "RNA polymerase II suppressor protein SRB5 - yeast and/or suppressor of RNA polymerase B SRB5" activity or its homolog, for the production of the fine chemical, meaning of glutamate , in particular for increasing the amount of glutamate , preferably glutamate in free or bound form in an organism or a part thereof, as mentioned.
The sequence of YKR057W from Saccharomyces cerevisiae has been published in Dujon et al., Nature 369 (6479), 371-378, 1994 and Goffeau et al., Science 274 (5287), 546-547, 1996 and its activity is being defined as a ribosomal protein, similar to S21 ribosomal proteins, involved in ribosome biogenesis and translation. Accordingly, in one embodiment, the process of the present invention comprises the use of a gene product with an activity of rat ribosomal protein S21 superfamily, preferably a protein with a ribosomal protein, similar to S21 ribosomal proteins, involved in ribosome biogenesis and translation activity from Saccharomyces cerevisiae or its homolog, e.g. as shown herein, for the production of the fine chemical, meaning of arginine and/or glutamine , in particular for increasing the amount of arginine and/or glutamine , in particular for increasing the amount of arginine, in particular for increasing the amount of glutamine, in particular for increasing the amount of arginine and glutamine, preferably arginine and/or glutamine in free or bound form in an organism or a part thereof, as mentioned.
The sequence of b0050 (Accession number NP_414592) from Escherichia coli K12 has been published in Blattner et al., Science 277 (5331), 1453-1474, 1997, and its activity is being defined as a conserved protein potentially involved in protein protein interaction. Accordingly, in one embodiment, the process of the present invention comprises the use of a gene product with an activity of apaG protein superfamily, preferably a protein with the activity of a conserved protein potentially involved in protein-protein interaction from E. coli or its homolog, e.g. as shown herein, for the production of the fine chemical, meaning of glutamate in particular for increasing the amount of glutamate, preferably glutamate in free or bound form in an organism or a part thereof, as mentioned.
The sequence of b0057 (Accession number NP_414599) from Escherichia coli K12 has been published in Blattner et al., Science 277 (5331), 1453-1474, 1997, and its activity is not been characterized yet. Accordingly, in one embodiment, the process of the present invention comprises the use of a gene product with an activity of b0057 protein from E. coli or its homolog, e.g. as shown herein, for the production of the fine chemical, meaning of glutamate in particular for increasing the amount of glutamate, preferably glutamate in free or bound form in an organism or a part thereof, as mentioned.
The sequence of b0138 (Accession number NP_414680) from Escherichia coli K12 has been published in Blattner et al., Science 277 (5331), 1453-1474, 1997, and its activity is is being defined as a fimbrial-like adhesin protein. Accordingly, in one embodiment, the process of the present invention comprises the use of a gene product with an activity of b0138 protein from E. coli or its homolog, e.g. as shown herein, for the production of the fine chemical, meaning of proline in particular for increasing the amount of proline, preferably proline in free or bound form in an organism or a part thereof, as mentioned.
The sequence of b0149 (Accession number NP_414691) from Escherichia coli K12 has been published in Blattner et al., Science 277 (5331), 1453-1474, 1997, and its activity is being defined as a bifunctional penicillin-binding protein 1 b: glycosyl transferase (N-terminal); transpeptidase (C-terminal). Accordingly, in one embodiment, the process of the present invention comprises the use of a gene product with an activity of penicillin-binding protein 1 B superfamily, preferably a protein with the activity of a bifunctional penicillin-binding protein 1 b: glycosyl transferase (N-terminal); transpeptidase (C-terminal) from E. coli or its homolog, e.g. as shown herein, for the production of the fine chemical, meaning of proline, in particular for increasing the amount of proline, preferably proline in free or bound form in an organism or a part thereof, as mentioned.
The sequence of b0161 (Accession number NP_414691) from Escherichia coli K12 has been published in Blattner et al., Science 277 (5331), 1453-1474, 1997, and its activity is being defined as a periplasmic serine protease (heat shock protein). Accordingly, in one embodiment, the process of the present invention comprises the use of a gene product with an activity of Helicobacter serine proteinase superfamily, preferably a protein with the activity of a periplasmic serine protease (heat shock protein) from E. coli or its homolog, e.g. as shown herein, for the production of the fine chemical, meaning of arginine and/or glutamate and/or glutamine, in particular for increasing the amount of arginine, in particular for increasing the amount of glutamate, in particular for increasing the amount of glutamine, in particular for increasing the amount of arginine and glutamate, in particular for increasing the amount of arginine and glutamine, in particular for increasing the amount of glutamine and glutamate, in particular for increasing the amount of arginine and glutamine and glutamate, preferably arginine and/or glutamate and/or glutamine in free or bound form in an organism or a part thereof, as mentioned.
The sequence of b0486 (Accession number NP_415019) from Escherichia coli K12 has been published in Blattner et al., Science 277 (5331), 1453-1474, 1997, and its activity is being defined as a amino-acid/amine transport protein (APC family). Accordingly, in one embodiment, the process of the present invention comprises the use of a gene product with an activity of membrane protein ybaT superfamily, preferably a protein with the activity of a amino-acid/amine transport protein (APC family) from E. coli or its homolog, e.g. as shown herein, for the production of the fine chemical, meaning of glutamine, in particular for increasing the amount of glutamine, preferably glutamine in free or bound form in an organism or a part thereof, as mentioned.
The sequence of b0849 (Accession number NP_415370) from Escherichia coli K12 has been published in Blattner et al., Science 277 (5331), 1453-1474, 1997, and its activity is being defined as a glutaredoxin 1 redox coenzyme for glutathione-dependent ribonucleotide reductase. Accordingly, in one embodiment, the process of the present invention comprises the use of a gene product with an activity of glutaredoxin superfamily, preferably a protein with the activity of a glutaredoxin 1 redox coenzyme for glutathione-dependent ribonucleotide reductase protein from E. coli or its homolog, e.g. as shown herein, for the production of the fine chemical, meaning of glutamine, in particular for increasing the amount of glutamine, preferably glutamine in free or bound form in an organism or a part thereof, as mentioned.
The sequence of b0970 (Accession number NP_415490) from Escherichia coli K12 has been published in Blattner et al., Science 277 (5331), 1453-1474, 1997, and its activity is being defined as a glutamate receptor. Accordingly, in one embodiment, the process of the present invention comprises the use of a gene product with an activity of Escherichia coli ybhL protein superfamily, preferably a protein with the activity of a glutamate receptor protein from E. coli or its homolog, e.g. as shown herein, for the production of the fine chemical, meaning of glutamine, in particular for increasing the amount of glutamine, preferably glutamine in free or bound form in an organism or a part thereof, as mentioned.
The sequence of b1343 (Accession number NP_415490) from Escherichia coli K12 has been published in Blattner et al., Science 277 (5331), 1453-1474, 1997, and its activity is being defined as a ATP-dependent RNA helicase, stimulated by 23S rRNA. Accordingly, in one embodiment, the process of the present invention comprises the use of a gene product with an activity of Escherichia coli b1343 protein, preferably a protein with the activity of a ATP-dependent RNA helicase, stimulated by 23S rRNA from E. coli or its homolog, e.g. as shown herein, for the production of the fine chemical, meaning of glutamine and/or glutamate, in particular for increasing the amount of glutamine, in particular for increasing the amount of glutamate, in particular for increasing the amount of glutamine and glutamate, preferably glutamine and/or glutamate in free or bound form in an organism or a part thereof, as mentioned.
The sequence of b1360 (Accession number NP_415878) from Escherichia coli K12 has been published in Blattner et al., Science 277 (5331), 1453-1474, 1997, and its activity is being defined as a DNA replication protein. Accordingly, in one embodiment, the process of the present invention comprises the use of a gene product with an activity of DNA replication protein dnaC superfamily, preferably a protein with the activity of a DNA replication protein from E. coli or its homolog, e.g. as shown herein, for the production of the fine chemical, meaning of proline, in particular for increasing the amount of proline, preferably proline in free or bound form in an organism or a part thereof, as mentioned.
The sequence of b1693 (Accession number NP_416208) from Escherichia coli K12 has been published in Blattner et al., Science 277 (5331), 1453-1474, 1997, and its activity is being defined as a 3-dehydroquinate dehydratase. Accordingly, in one embodiment, the process of the present invention comprises the use of a gene product with an activity of 3-dehydroquinate dehydratase superfamily, preferably a protein with the activity of a DNA replication protein from E. coli or its homolog, e.g. as shown herein, for the production of the fine chemical, meaning of glutamate, in particular for increasing the amount of glutamate, preferably glutamate in free or bound form in an organism or a part thereof, as mentioned.
The sequence of b1736 (Accession number NP_416250) from Escherichia coli K12 has been published in Blattner et al., Science 277 (5331), 1453-1474, 1997, and its activity is being defined as a PEP-dependent phosphotransferase enzyme, cellobiose/arbutin/salicin sugar-specific protein. Accordingly, in one embodiment, the process of the present invention comprises the use of a gene product with an activity of phosphotransferase system lactose-specific enzyme II, factor III superfamily, preferably a protein with the activity of a PEP-dependent phosphotransferase enzyme, cellobiose/arbutin/salicin sugar-specific protein from E. coli or its homolog, e.g. as shown herein, for the production of the fine chemical, meaning of glutamate, in particular for increasing the amount of glutamate, preferably glutamate in free or bound form in an organism or a part thereof, as mentioned.
The sequence of b1738 (Accession number NP_416252) from Escherichia coli K12 has been published in Blattner et al., Science 277 (5331), 1453-1474, 1997, and its activity is being defined as a PEP-dependent phosphotransferase. Accordingly, in one embodiment, the process of the present invention comprises the use of a gene product with an activity of phosphotransferase system enzyme II cellobiose-specific factor IIB superfamily, preferably a protein with the activity of a PEP-dependent phosphotransferase from E. coli or its homolog, e.g. as shown herein, for the production of the fine chemical, meaning of glutamate, in particular for increasing the amount of glutamate, preferably glutamate in free or bound form in an organism or a part thereof, as mentioned
The sequence of b1886 (Accession number NP_416400) from Escherichia coli K12 has been published in Blattner et al., Science 277 (5331), 1453-1474, 1997, and its activity is being defined as a methyl-accepting chemotaxis protein II, aspartate sensor receptor. Accordingly, in one embodiment, the process of the present invention comprises the use of a gene product with an activity of methyl-accepting chemotaxis protein superfamily, preferably a protein with the activity of a methyl-accepting chemotaxis protein II, aspartate sensor receptor from E. coli or its homolog, e.g. as shown herein, for the production of the fine chemical, meaning of glutamine, in particular for increasing the amount of glutamine, preferably glutamine in free or bound form in an organism or a part thereof, as mentioned.
The sequence of b1896 (Accession number NP_416410) from Escherichia coli K12 has been published in Blattner et al., Science 277 (5331), 1453-1474, 1997, and its activity is being defined as a trehalose-6-phosphate synthase. Accordingly, in one embodiment, the process of the present invention comprises the use of a gene product with an activity of alpha-trehalose-phosphate synthase (UdP-forming) superfamily, preferably a protein with the activity of a trehalose-6-phosphate synthase from E. coli or its homolog, e.g. as shown herein, for the production of the fine chemical, meaning of glutamate, in particular for increasing the amount of glutamate, preferably glutamate in free or bound form in an organism or a part thereof, as mentioned
The sequence of b1926 (Accession number NP_416436) from Escherichia coli K12 has been published in Blattner et al., Science 277 (5331), 1453-1474, 1997, and its activity is being defined as a flagellar protein fliT. Accordingly, in one embodiment, the process of the present invention comprises the use of a gene product with an activity of flagellar protein fliT from E. coli or its homolog, e.g. as shown herein, for the production of the fine chemical, meaning of glutamine, in particular for increasing the amount of glutamine, preferably glutamine in free or bound form in an organism or a part thereof, as mentioned.
The sequence of b2307 (Accession number NP_416810) from Escherichia coli K12 has been published in Blattner et al., Science 277 (5331), 1453-1474, 1997, and its activity is being defined as a histidine and lysine/arginine/ornithine transport protein (ABC superfamily, membrane). Accordingly, in one embodiment, the process of the present invention comprises the use of a gene product with an activity of histidine permease protein M superfamily, preferably a protein with the activity of a histidine and lysine/arginine/ornithine transport protein (ABC superfamily, membrane) from E. coli or its homolog, e.g. as shown herein, for the production of the fine chemical, meaning of glutamate and/or arginine, in particular for increasing the amount of glutamate, in particular for increasing the amount of arginine, in particular for increasing the amount of glutamate and arginine, preferably glutamate and/or arginine in free or bound form in an organism or a part thereof, as mentioned.
The sequence of b2414 (Accession number NP_416909) from Escherichia coli K12 has been published in Blattner et al., Science 277 (5331), 1453-1474, 1997, and its activity is being defined as a subunit of cysteine synthase A and O-acetylserine sulfhydrolase A, PLP-dependent enzyme. Accordingly, in one embodiment, the process of the present invention comprises the use of a gene product with an activity of threonine dehydratase superfamily, preferably a protein with the activity of a subunit of cysteine synthase A and O-acetylserine sulfhydrolase A, PLP-dependent enzyme from E. coli or its homolog, e.g. as shown herein, for the production of the fine chemical, meaning of glutamine, in particular for increasing the amount of glutamine, preferably glutamine in free or bound form in an organism or a part thereof, as mentioned.
The sequence of b2426 (Accession number NP_416921)from Escherichia coli K12 has been published in Blattner et al., Science 277 (5331), 1453-1474, 1997, and its activity is being defined as a putative oxidoreductase with NAD(P)-binding domain. Accordingly, in one embodiment, the process of the present invention comprises the use of a gene product with an activity of ribitol dehydrogenase, short-chain alcohol dehydrogenase homology superfamily, preferably a protein with the activity of a putative oxidoreductase with NAD(P)-binding domain from E. coli or its homolog, e.g. as shown herein, for the production of the fine chemical, meaning of glutamine, in particular for increasing the amount of glutamine, preferably glutamine in free or bound form in an organism or a part thereof, as mentioned.
The sequence of b2489 (Accession number NP_416984) from Escherichia coli K12 has been published in Blattner et al., Science 277 (5331), 1453-1474, 1997, and its activity is being defined as a hydrogenase Fe-S subunit. Accordingly, in one embodiment, the process of the present invention comprises the use of a gene product with an activity of psbG protein superfamily, preferably a protein with the activity of a hydrogenase Fe-S subunit from E. coli or its homolog, e.g. as shown herein, for the production of the fine chemical, meaning of glutamine, in particular for increasing the amount of glutamine, preferably glutamine in free or bound form in an organism or a part thereof, as mentioned.
The sequence of b2553 (Accession number NP_417048) from Escherichia coli K12 has been published in Blattner et al., Science 277 (5331), 1453-1474, 1997, and its activity is being defined as a regulatory protein P-II for glutamine synthetase. Accordingly, in one embodiment, the process of the present invention comprises the use of a gene product with an activity of regulatory protein P-II superfamily, preferably a protein with the activity of a regulatory protein P-II for glutamine synthetase from E. coli or its homolog, e.g. as shown herein, for the production of the fine chemical, meaning of proline and/or glutamine, in particular for increasing the amount of proline, in particular for increasing the amount of glutamine, in particular for increasing the amount of proline and glutamine, preferably proline and/or glutamine in free or bound form in an organism or a part thereof, as mentioned.
The sequence of b2664 (Accession number NP_417150) from Escherichia coli K12 has been published in Blattner et al., Science 277 (5331), 1453-1474, 1997, and its activity is being defined as a transcriptional repressor with DNA-binding Winged helix domain (GntR familiy). Accordingly, in one embodiment, the process of the present invention comprises the use of a gene product with an activity of transcription regulator gabP superfamily, preferably a protein with the activity of transcriptional repressor with DNA-binding Winged helix domain (GntR familiy) from E. coli or its homolog, e.g. as shown herein, for the production of the fine chemical, meaning of proline, in particular for increasing the amount of proline, preferably proline in free or bound form in an organism or a part thereof, as mentioned.
The sequence of b2710 (Accession number NP_417190) from Escherichia coli K12 has been published in Blattner et al., Science 277 (5331), 1453-1474, 1997, and its activity is being defined as a flavorubredoxin (FIRd) bifunctional NO and O₂ reductase. Accordingly, in one embodiment, the process of the present invention comprises the use of a gene product with an activity of Escherichia coli hypothetical protein b2710, rubredoxin homology, Methanobacterium flavoprotein A superfamily, preferably a protein with the activity of a a flavorubredoxin (FIRd) bifunctional NO and O₂ reductase from E. coli or its homolog, e.g. as shown herein, for the production of the fine chemical, meaning of glutamate, in particular for increasing the amount of glutamate, preferably glutamate in free or bound form in an organism or a part thereof, as mentioned.
The sequence of b2818 (Accession number NP_417295) from Escherichia coli K12 has been published in Blattner et al., Science 277 (5331), 1453-1474, 1997, and its activity is being defined as a N-acetylglutamate synthase (amino acid N-acetyltransferase). Accordingly, in one embodiment, the process of the present invention comprises the use of a gene product with an activity of amino-acid acetyltransferase, acetylglutamate kinase superfamily, preferably a protein with the activity of a a N-acetylglutamate synthase (amino acid N-acetyltransferase) from E. coli or its homolog, e.g. as shown herein, for the production of the fine chemical, meaning of glutamine and/or glutamate, in particular for increasing the amount of glutamine, in particular for increasing the amount of glutamate, in particular for increasing the amount of glutamine and glutamte, preferably glutamate and/or glutamine in free or bound form in an organism or a part thereof, as mentioned.
The sequence of b3064 (Accession number NP_417536) from Escherichia coli K12 has been published in Blattner et al., Science 277 (5331), 1453-1474, 1997, and its activity is being defined as a O-sialoglycoprotein endopeptidase, with actin-like ATPase domain. Accordingly, in one embodiment, the process of the present invention comprises the use of a gene product with an activity of O-sialoglycoprotein endopeptidase superfamily, preferably a protein with the activity of a O-sialoglycoprotein endopeptidase, with actin-like ATPase domain from E. coli or its homolog, e.g. as shown herein, for the production of the fine chemical, meaning of glutamine, in particular for increasing the amount of glutamine, preferably glutamine in free or bound form in an organism or a part thereof, as mentioned.
The sequence of b3074 (Accession number NP_417545) from Escherichia coli K12 has been published in Blattner et al., Science 277 (5331), 1453-1474, 1997, and its activity is being defined as a tRNA synthetase. Accordingly, in one embodiment, the process of the present invention comprises the use of a gene product with an activity of secretion chaperone CsaA, methionyl-tRNA synthetase, dimer-forming superfamily, preferably a protein with the activity of a tRNA synthetase from E. coli or its homolog, e.g. as shown herein, for the production of the fine chemical, meaning of glutamate, in particular for increasing the amount of glutamate, preferably glutamate in free or bound form in an organism or a part thereof, as mentioned.
The sequence of b3116 (Accession number NP_417586) from Escherichia coli K12 has been published in Blattner et al., Science 277 (5331), 1453-1474, 1997, and its activity is being defined as a L-threonine/L-serine permease, anaerobically inducible (HAAAP family). Accordingly, in one embodiment, the process of the present invention comprises the use of a gene product with an activity of threonine-serine permease superfamily, preferably a protein with the activity of a L-threonine/L-serine permease, anaerobically inducible (HAAAP family) from E. coli or its homolog, e.g. as shown herein, for the production of the fine chemical, meaning of glutamate, in particular for increasing the amount of glutamate, preferably glutamate in free or bound form in an organism or a part thereof, as mentioned.
The sequence of b3160 (Accession number NP_417629) from Escherichia coli K12 has been published in Blattner et al., Science 277 (5331), 1453-1474, 1997, and its activity is being defined as monooxygenase with luciferase-like ATPase activity. Accordingly, in one embodiment, the process of the present invention comprises the use of a gene product with an activity of ynbW protein superfamily, preferably a protein with the activity of a monooxygenase with luciferase-like ATPase activity from E. coli or its homolog, e.g. as shown herein, for the production of the fine chemical, meaning of glutamine, in particular for increasing the amount of glutamine, preferably glutamine in free or bound form in an organism or a part thereof, as mentioned.
The sequence of b3166 (Accession number NP_417635) from Escherichia coli K12 has been published in Blattner et al., Science 277 (5331), 1453-1474, 1997, and its activity is being defined as tRNA pseudouridine 5S synthase. Accordingly, in one embodiment, the process of the present invention comprises the use of a gene product with an activity of Escherichia coli protein P35 superfamily, preferably a protein with the activity of a tRNA pseudouridine 5S synthase from E. coli or its homolog, e.g. as shown herein, for the production of the fine chemical, meaning of glutamine, in particular for increasing the amount of glutamine, preferably glutamine in free or bound form in an organism or a part thereof, as mentioned.
The sequence of b3169 (Accession number NP_417638) from Escherichia coli K12 has been published in Blattner et al., Science 277 (5331), 1453-1474, 1997, and its activity is being defined as a transcription termination-antitermination factor. Accordingly, in one embodiment, the process of the present invention comprises the use of a gene product with an activity of Escherichia coli transcription factor nusA superfamily, preferably a protein with the activity of a transcription termination-antitermination factor from E. coli or its homolog, e.g. as shown herein, for the production of the fine chemical, meaning of glutamine and/or glutamate, in particular for increasing the amount of glutamine, in particular for increasing the amount of glutamate, in particular for increasing the amount of glutamine and glutamte, preferably glutamate and/or glutamine in free or bound form in an organism or a part thereof, as mentioned.
The sequence of b3231 (Accession number NP_417698) from Escherichia coli K12 has been published in Blattner et al., Science 277 (5331), 1453-1474, 1997, and its activity is being defined as a 50S ribosomal subunit protein L13. Accordingly, in one embodiment, the process of the present invention comprises the use of a gene product with an activity of Escherichia coli ribosomal protein L13 superfamily, preferably a protein with the activity of a 50S ribosomal subunit protein L13 from E. coli or its homolog, e.g. as shown herein, for the production of the fine chemical, meaning of glutamine, in particular for increasing the amount of glutamine, preferably glutamine in free or bound form in an organism or a part thereof, as mentioned.
The sequence of b3619 (Accession number NP_418076) from Escherichia coli K12 has been published in Blattner et al., Science 277 (5331), 1453-1474, 1997, and its activity is being defined as a ADP-L-glycero-D-mannoheptose-6-epimerase, NAD(P)-binding. Accordingly, in one embodiment, the process of the present invention comprises the use of a gene product with an activity of ADPglyceromanno-heptose 6-epimerase, UDPglucose 4-epimerase homology superfamily, preferably a protein with the activity of a ADP-L-glycero-D-mannoheptose-6-epimerase, NAD(P)-binding from E. coli or its homolog, e.g. as shown herein, for the production of the fine chemical, meaning of glutamate, in particular for increasing the amount of glutamate, preferably glutamate in free or bound form in an organism or a part thereof, as mentioned.
The sequence of b3644 (Accession number NP_418101) from Escherichia coli K12 has been published in Blattner et al., Science 277 (5331), 1453-1474, 1997, and its activity is being defined as a uncharacterized stress-induced protein. Accordingly, in one embodiment, the process of the present invention comprises the use of a gene product with an activity of protein HI0467 superfamily, preferably a protein with the activity of a Uncharacterized stress-induced protein from E. coli or its homolog, e.g. as shown herein, for the production of the fine chemical, meaning of proline, in particular for increasing the amount of proline, preferably proline in free or bound form in an organism or a part thereof, as mentioned.
The sequence of b3680 (Accession number NP_418136) from Escherichia coli K12 has been published in Blattner et al., Science 277 (5331), 1453-1474, 1997, and its activity is being defined as a transcriptional regulator with homeodomain-like DNA binding domain (AraC/XylS family). Accordingly, in one embodiment, the process of the present invention comprises the use of a gene product with an activity of Escherichia coli b3680 protein, preferably a protein with the activity of a transcriptional regulator with homeodomain-like DNA binding domain (AraC/XylS family) from E. coli or its homolog, e.g. as shown herein, for the production of the fine chemical, meaning of glutamine, in particular for increasing the amount of glutamine, preferably glutamine in free or bound form in an organism or a part thereof, as mentioned.
The sequence of b3791 (Accession number NP_418238) from Escherichia coli K12 has been published in Blattner et al., Science 277 (5331), 1453-1474, 1997, and its activity is being defined as a transaminase involved in lipopolysaccharide biosynthesis. Accordingly, in one embodiment, the process of the present invention comprises the use of a gene product with an activity of erythromycin resistance protein superfamily, preferably a protein with the activity of a transaminase involved in lipopolysaccharide biosynthesis from E. coli or its homolog, e.g. as shown herein, for the production of the fine chemical, meaning of glutamine and/or glutamate, in particular for increasing the amount of glutamine, in particular for increasing the amount of glutamate, in particular for increasing the amount of glutamine and glutamate, preferably glutamate and/or glutamine in free or bound form in an organism or a part thereof, as mentioned.
The sequence of b3919 (Accession number NP_418354) from Escherichia coli K12 has been published in Blattner et al., Science 277 (5331), 1453-1474, 1997, and its activity is being defined as a triosephosphate isomerase. Accordingly, in one embodiment, the process of the present invention comprises the use of a gene product with an activity of triose-phosphate isomerase superfamily, preferably a protein with the activity of a triosephosphate isomerase from E. coli or its homolog, e.g. as shown herein, for the production of the fine chemical, meaning of proline, in particular for increasing the amount of proline, preferably proline in free or bound form in an organism or a part thereof, as mentioned.
The sequence of b3936 (Accession number NP_418371) from Escherichia coli K12 has been published in Blattner et al., Science 277 (5331), 1453-1474, 1997, and its activity is being defined as a 50S ribosomal subunit protein L32. Accordingly, in one embodiment, the process of the present invention comprises the use of a gene product with an activity of Escherichia coli ribosomal protein L31 superfamily, preferably a protein with the activity of a 50S ribosomal subunit protein L32 from E. coli or its homolog, e.g. as shown herein, for the production of the fine chemical, meaning of arginine, in particular for increasing the amount of arginine, preferably arginine in free or bound form in an organism or a part thereof, as mentioned.
The sequence of b4004 (Accession number NP_418432) from Escherichia coli K12 has been published in Blattner et al., Science 277 (5331), 1453-1474, 1997, and its activity is being defined as a transcriptional regulatory protein. Accordingly, in one embodiment, the process of the present invention comprises the use of a gene product with an activity of nitrogen assimilation regulatory protein ntrC or response regulator homology, RNA polymerase sigma factor interaction domain homology superfamily, preferably a protein with the activity of a transcriptional regulatory protein from E. coli or its homolog, e.g. as shown herein, for the production of the fine chemical, meaning of glutamine, in particular for increasing the amount of glutamine, preferably glutamine in free or bound form in an organism or a part thereof, as mentioned.
The sequence of b4074 (Accession number NP_418498) from Escherichia coli K12 has been published in Blattner et al., Science 277 (5331), 1453-1474, 1997, and its activity is being defined as a cytochrome c-type biogenesis protein. Accordingly, in one embodiment, the process of the present invention comprises the use of a gene product with an activity of nrfE protein superfamily, preferably a protein with the activity of a Cytochrome c-type biogenesis protein from E. coli or its homolog, e.g. as shown herein, for the production of the fine chemical, meaning of glutamine, in particular for increasing the amount of glutamine, preferably glutamine in free or bound form in an organism or a part thereof, as mentioned.
The sequence of b4133 (Accession number NP_418557) from Escherichia coli K12 has been published in Blattner et al., Science 277 (5331), 1453-1474, 1997, and its activity is being defined as a transcriptional activator of pH response (OmpR family). Accordingly, in one embodiment, the process of the present invention comprises the use of a gene product with an activity of b4133 protein, preferably a protein with the activity of a transcriptional activator of pH response (OmpR family) from E. coli or its homolog, e.g. as shown herein, for the production of the fine chemical, meaning of glutamine, in particular for increasing the amount of glutamine, preferably glutamine in free or bound form in an organism or a part thereof, as mentioned.
The sequence of b4346 (Accession number NP_418766) from Escherichia coli K12 has been published in Blattner et al., Science 277 (5331), 1453-1474, 1997, and its activity is being defined as a component of 5-methylcytosine-specific restriction enzyme McrBC. Accordingly, in one embodiment, the process of the present invention comprises the use of a gene product with an activity of 5-methylcytosine-specific restriction enzyme B superfamily, preferably a protein with the activity of a component of 5-methylcytosine-specific restriction enzyme McrBC from E. coli or its homolog, e.g. as shown herein, for the production of the fine chemical, meaning of glutamate, in particular for increasing the amount of glutamate, preferably glutamate in free or bound form in an organism or a part thereof, as mentioned.
The sequence of YFL019C (Accession number S48324.) from Saccharomyces cerevisiae has been published in Murakami et al., Nat. Genet. 10:261-268(1995) and its activity is not been characterized yet. Accordingly, in one embodiment, the process of the present invention comprises the use of a gene product with an activity of a YFL019C protein from Saccharomyces cerevisiae or its homolog, e.g. as shown herein, for the production of the fine chemical, meaning of glutamate, in particular for increasing the amount of glutamate, preferably glutamate in free or bound form in an organism or a part thereof, as mentioned.

**[0023.0.4.4]** Homologues ( = homologs) of the present gene products can be derived from any organisms as long as the homologue confers the herein mentioned activity, in particular, confers an increase in the respective fine chemical amount or content.
In one embodiment, the homolog of the any one of the polypeptides indicated in Table II, column 3, lines 30 to 33 for arginine
and/or lines 38 to 42 and/or 435 for glutamate
and/or lines 44 to 53 for proline
and/or lines 57 to 59 for glutamine, resp. is a homolog having the same or a similar acitivity, resp. In particular an increase of activity confers an increase in the content of the respective fine chemical in the organisms. In one embodiment, the homolog is a homolog with a sequence as indicated in Table I or II, column 7, lines 30 to 33 for arginine
and/or lines 38 to 42 and/or 435 for glutamate
and/or lines 44 to 53 for proline
and/or lines 57 to 59 for glutamine, resp. In one embodiment, the homolog of one of the polypeptides indicated in Table II, column 3, lines 30 to 33 for arginine
and/or lines 38 to 42 and/or 435 for glutamate
and/or lines 44 to 53 for proline
and/or lines 57 to 59 for glutamine resp., is derived from an eukaryotic. In one embodiment, the homolog is derived from Fungi. In one embodiment, the homolog of a polypeptide indicated in Table II, column 3, lines 30 to 33 for arginine
and/or lines 38 to 42 and/or 435 for glutamate
and/or lines 44 to 53 for proline
and/or lines 57 to 59 for glutamine, resp., is derived from Ascomyceta. In one embodiment, the homolog of a polypeptide indicated in Table II, column 3, lines 30 to 33 for arginine
and/or lines 38 to 42 and/or 435 for glutamate
and/or lines 44 to 53 for proline
and/or lines 57 to 59 for glutamine, resp., is derived from Saccharomycotina. In one embodiment, the homolog of a polypeptide indicated in Table II, column 3, lines 30 to 33 for arginine
and/or lines 38 to 42 and/or 435 for glutamate
and/or lines 44 to 53 for proline
and/or lines 57 to 59 for glutamine, resp., is derived from Saccharomycetes. In one embodiment, the homolog of a polypeptide indicated in Table II, column 3, lines 30 to
33 for arginine
and/or lines 38 to 42 and/or 435 for glutamate
and/or lines 44 to 53 for proline
and/or lines 57 to 59 for glutamine, resp., is a homolog being derived from Saccharomycetales. In one embodiment, the homolog of a polypeptide indicated in Table II, column 3, lines 30 to 33 for arginine
and/or lines 38 to 42 and/or 435 for glutamate
and/or lines 44 to 53 for proline
and/or lines 57 to 59 for glutamine, resp., is a homolog having the same or a similar acitivity being derived from Saccharomycetaceae. In one embodiment, the homolog of a polypeptide indicated in Table II, column 3, lines 30 to 33 for arginine
and/or lines 38 to 42 and/or 435 for glutamate
and/or lines 44 to 53 for proline
and/or lines 57 to 59 for glutamine, resp., is a homolog having the same or a similar acitivity being derived from Saccharomycetes.
In one embodiment, the homolog of the any one of the polypeptides indicated in Table II, column 3, lines 34 to 37, 390, 405 and/or 430 for arginine
and/or lines 43, 386, 387, 391, 396, 399 to 401, 403, 406, 413, 414, 417, 418, 421, 424, 427 and/or 434 for glutamate
and/or lines 54 to 56, 388, 389, 398, 411, 412, 425 and/or 429 for proline
and/or lines 62, 392 to 395, 397, 402, 404, 407 to 410, 415, 416, 419, 420, 422, 423, 426, 428 and/or 431 to 433 for glutamine resp. is a homolog having the same or a similar acitivity. In particular an increase of activity confers an increase in the content of the respective fine chemical in the organisms. In one embodiment, the homolog is a homolog with a sequnence as indicated in Table I or II, column 7, lines 34 to 37, 390, 405 and/or 430 for arginine
and/or lines 43, 386, 387, 391, 396, 399 to 401, 403, 406, 413, 414, 417, 418, 421, 424, 427 and/or 434 for glutamate
and/or lines 54 to 56, 388, 389, 398, 411, 412, 425 and/or 429 for proline
and/or lines 62, 392 to 395, 397, 402, 404, 407 to 410, 415, 416, 419, 420, 422, 423, 426, 428 and/or 431 to 433 for glutamine, resp. In one embodiment, the homolog of one of the polypeptides indicated in Table II, column 3, lines 34 to 37, 390, 405 and/or 430 for arginine
and/or lines 43, 386, 387, 391, 396, 399 to 401, 403, 406, 413, 414, 417, 418, 421, 424, 427 and/or 434 for glutamate
and/or lines 54 to 56, 388, 389, 398, 411, 412, 425 and/or 429 for proline
and/or lines 62, 392 to 395, 397, 402, 404, 407 to 410, 415, 416, 419, 420, 422, 423, 426, 428 and/or 431 to 433 for glutamine is derived from an bacteria. In one embodiment, the homolog of a polypeptide indicated in Table II, column 3, lines 34 to 37, 390, 405 and/or 430 for arginine
and/or lines 43, 386, 387, 391, 396, 399 to 401, 403, 406, 413, 414, 417, 418, 421, 424, 427 and/or 434 for glutamate
and/or lines 54 to 56, 388, 389, 398, 411, 412, 425 and/or 429 for proline
and/or lines 62, 392 to 395, 397, 402, 404, 407 to 410, 415, 416, 419, 420, 422, 423, 426, 428 and/or 431 to 433 for glutamine is derived from Proteobacteria. In one embodiment, the homolog of a polypeptide indicated in Table II, column 3, lines 34 to 37, 390, 405 and/or 430 for arginine
and/or lines 43, 386, 387, 391, 396, 399 to 401, 403, 406, 413, 414, 417, 418, 421, 424, 427 and/or 434 for glutamate
and/or lines 54 to 56, 388, 389, 398, 411, 412, 425 and/or 429 for proline
and/or lines 62, 392 to 395, 397, 402, 404, 407 to 410, 415, 416, 419, 420, 422, 423, 426, 428 and/or 431 to 433 for glutamine is a homolog having the same or a similar acitivity being derived from Gammaproteobacteria. In one embodiment, the homolog of a polypeptide indicated in Table II, column 3, lines 34 to 37, 390, 405 and/or 430 for arginine
and/or lines 43, 386, 387, 391, 396, 399 to 401, 403, 406, 413, 414, 417, 418, 421, 424, 427 and/or 434 for glutamate
and/or lines 54 to 56, 388, 389, 398, 411, 412, 425 and/or 429 for proline
and/or lines 62, 392 to 395, 397, 402, 404, 407 to 410, 415, 416, 419, 420, 422, 423, 426, 428 and/or 431 to 433 for glutamine is derived from Enterobacteriales. In one embodiment, the homolog of a polypeptide indicated in Table II, column 3, lines 34 to 37, 390, 405 and/or 430 for arginine
and/or lines 43, 386, 387, 391, 396, 399 to 401, 403, 406, 413, 414, 417, 418, 421, 424, 427 and/or 434 for glutamate
and/or lines 54 to 56, 388, 389, 398, 411, 412, 425 and/or 429 for proline
and/or lines 62, 392 to 395, 397, 402, 404, 407 to 410, 415, 416, 419, 420, 422, 423, 426, 428 and/or 431 to 433 for glutamine is a homolog being derived from Enterobacteriaceae. In one embodiment, the homolog of a polypeptide indicated in Table II, column 3, lines 34 to 37, 390, 405 and/or 430 for arginine
and/or lines 43, 386, 387, 391, 396, 399 to 401, 403, 406, 413, 414, 417, 418, 421, 424, 427 and/or 434 for glutamate
and/or lines 54 to 56, 388, 389, 398, 411, 412, 425 and/or 429 for proline
and/or lines 62, 392 to 395, 397, 402, 404, 407 to 410, 415, 416, 419, 420, 422, 423, 426, 428 and/or 431 to 433 for glutamine is a homolog having the same or a similar acitivity and being derived from Escherichia.

**[0023.1.4.4]** Homologs of the polypeptide indicated in Table II, column 3, lines 30 to 62 and/or lines 386 to 435 may be the polypetides encoded by the nucleic acid molecules indicated in Table I, column 7, lines 30 to 62 and/or lines 386 to 435, resp., or may be the polypeptides indicated in Table II, column 7, lines 30 to 62 and/or lines 386 to 435, resp.

**[0024.0.0.4]: see [0024.0.0.0]**

**[0025.0.4.4]** In accordance with the invention, a protein or polypeptide has the "activity of an protein of the invention", e.g. the activity of a protein indicated in Table II, column 3, lines 34 to 37, 390, 405 and/or 430 for arginine
and/or lines 43, 386, 387, 391, 396, 399 to 401, 403, 406, 413, 414, 417, 418, 421, 424, 427, 434 and/or 435 for glutamate
and/or lines 54 to 56, 388, 389, 398, 411, 412, 425 and/or 429 for proline
and/or lines 62, 392 to 395, 397, 402, 404, 407 to 410, 415, 416, 419, 420, 422, 423, 426, 428 and/or 431 to 433 for glutamine resp.
, if its *de novo* activity, or its increased expression directly or indirectly leads to an increased arginine and/or glutamate and/or proline and/or glutamine, resp., in the organism or a part thereof, preferably in a cell of said organism. In a preferred embodiment, the protein or polypeptide has the above-mentioned additional activities of a protein indicated in Table II, column 3, lines 34 to 37, 390, 405 and/or 430 and/or lines 43, 386, 387, 391, 396, 399 to 401, 403, 406, 413, 414, 417, 418, 421, 424, 427, 434 and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp.. Throughout the specification the activity or preferably the biological activity of such a protein or polypeptide or an nucleic acid molecule or sequence encoding such protein or polypeptide is identical or similar if it still has the biological or enzymatic activity of any one of the proteins indicated in Table II, column 3, lines 34 to 37, 390, 405 and/or 430 and/or lines 43, 386, 387, 391, 396, 399 to 401, 403, 406, 413, 414, 417, 418, 421, 424, 427, 434 and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp. or which has at least 10% of the original enzymatic activity, preferably 20%, particularly preferably 30%, most particularly preferably 40% in comparison to any one of the proteins indicated in Table II, column 3, lines 30 to 33 and/or lines 38 to 42 and/or 435 and/or lines 44 to 53 and/or lines 57 to 59 of Saccharomyces cerevisiae and/or any one of the proteins indicated in Table II, column 3, lines 34 to 37, 390, 405 and/or 430 and/or lines 43, 386, 387, 391, 396, 399 to 401, 403, 406, 413, 414, 417, 418, 421, 424, 427 and/or 434 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 of E. coli K12.

**[0025.1.0.4] to [0033.0.0.4]: see [0025.1.0.0] to [0033.0.0.0]**

**[0034.0.4.4]** Preferably, the reference, control or wild type differs form the subject of the present invention only in the cellular activity of the polypeptide of the invention, e.g. as result of an increase in the level of the nucleic acid molecule of the present invention or an increase of the specific activity of the polypeptide of the invention. E.g., it differs by or in the expression level or activity of an protein having the activity of a protein as indicated in Table II, column 3, lines 34 to 37, 390, 405 and/or 430 and/or lines 43, 386, 387, 391, 396, 399 to 401, 403, 406, 413, 414, 417, 418, 421, 424, 427 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp.
, or being encoded by a nucleic acid molecule indicated in Table I, column 5, lines 34 to 37, 390, 405 and/or 430 and/or lines 43, 386, 387, 391, 396, 399 to 401, 403, 406, 413, 414, 417, 418, 421, 424, 427, 434 and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp.
, or its homologs, e.g. as indicated in Table I, column 7, lines 34 to 37, 390, 405 and/or 430 and/or lines 43, 386, 387, 391, 396, 399 to 401, 403, 406, 413, 414, 417, 418, 421, 424, 427, 434 and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp.
its biochemical or genetical causes and therefore shows the increased amount of the respective fine chemical.

**[0035.0.0.4] to [0044.0.0.4]: see [0035.0.0.0] to [0044.0.0.0]**

**[0045.0.4.4]** In case the activity of the Escherichia coli K12 protein b0695 or its homologs, as indicated in Table I, columns 5 or 7, line 35, e.g. a sensory histidine kinase in two-component signal transduction system (sensor kinase component), modification by phosphorylation, dephosphorylation, unspecified signal transduction, regulation of respiration, aerobic respiration, is increased, preferably, in one embodiment the increase of the fine chemical, preferably of arginine between 51 % and 319% or more is conferred.
In case the activity of the Escherichia coli K12 protein b0730 or its homologs, as indicated in Table I, columns 5 or 7, line 43 or 54, e.g. a transcriptional regulator for regulation of C-compound and carbohydrate utilization, transcriptional control, prokaryotic nucleotide, transcriptional repressor, DNA binding, is increased, preferably, in one embodiment the increase of the fine chemical between 35% and 272%, preferably of glutamate between 55% and 115% and/or of proline between 35% and 272%, or more is conferred.
In case the activity of the Escherichia coli K12 protein b1284 or its homologs, as indicated in Table I, columns 5 or 7, line 36, e.g. a transcriptional regulator for regulation of C-compound and carbohydrate utilization, transcriptional control, transcriptional repressor, DNA binding, is increased, preferably, in one embodiment the increase of the fine chemical, preferably of arginine between 47% and 183% or more is conferred.
In case the activity of the Escherichia coli K12 protein b1827 or its homologs, e.g. as indicated in Table I, columns 5 or 7, line 56, e.g. a transcriptional repressor for transcriptional control, is increased, preferably, in one embodiment the increase of the fine chemical, preferably of proline between 42% and 126%, or more is conferred.
In case the activity of the Escherichia coli K12 protein b1829 or its homologs, e.g.as indicated in Table I, columns 5 or 7, line 34 or 60, is increased, e.g. the activity of a heat shock protein with protease activity (htpx), involved in stress response, pheromone response, mating-type determination, sex-specific proteins, protein modification, proteolytic degradation is increased, preferably, in one embodiment the increase of the fine chemical between 45% and 1141%, preferably of glutamine between 50% and 68% and/or of arginine between 45% and 1141 % or more is conferred.
In case the activity of the Escherichia coli K12 protein b1852 or its homologs, e.g. as indicated in Table I, columns 5 or 7, line 61, is increased, e.g. the activity of a glucose-6-phosphate dehydrogenase, involved in pentose-phosphate pathway oxidative branch, C-compound and carbohydrate utilization, NAD/NADP binding, nucleotide metabolism, metabolism of vitamins, cofactors, and prosthetic groups, energy is increased, preferably, in one embodiment the increase of the fine chemical, preferably of glutamine between 40% and 42% or more is conferred.
In case the activity of the Escherichia coli K12 protein b2095 or its homologs, e.g. as indicated in Table I, columns 5 or 7, line 37, is increased, e.g. the activity of a tagatose-6-phosphate kinase is increased, preferably, in one embodiment the increase of the fine chemical, preferably of arginine between 55% and 59% or more is conferred.
In case the activity of the Escherichia coli K12 protein b2699 or its homologs, e.g. as indicated in Table I, columns 5 or 7, line 55, is increased, e.g. the activity of a recombination protein recA, involved in DNA recombination and DNA repair, pheromone response, mating-type determination, sex-specific proteins, nucleotide binding is increased, preferably, in one embodiment the increase of the fine chemical, preferably of proline between 32% and 141% or more is conferred.
In case the activity of the Escherichia coli K12 protein b4265 or its homologs, e.g. as indicated in Table I, columns 5 or 7, line 62, is increased, e.g. the activity of a D-serine permease, involved in C-compound and carbohydrate transports, C-compound and carbohydrate utilization is increased, preferably, in one embodiment the increase of the fine chemical, preferably of glutamine between 32% and 47% or more is conferred.
In case the activity of the Saccharomyces cerevisiae protein YBR030W or its homologs, e.g. as indicated in Table I, columns 5 or 7, line 44, e.g. a "uncharacterized protein YBR030W", involved in C-compound and carbohydrate utilization, pentose-phosphate pathway and/or transcriptional control is increased, preferably, in one embodiment an increase of the fine chemical, preferably of proline between 51% and 282% or more is conferred.
In case the activity of the Saccharomyces cerevisiae protein YDL106C or its homologs, e.g. as indicated in Table I, columns 5 or 7, line 45, e.g. a homeobox proteins, involved in regulation of nucleotide metabolism, regulation of phosphate utilization, transcriptional control, nucleus is increased, preferably, in one embodiment an increase of the fine chemical, preferably of proline between 51% and 99% or more is conferred. In case the activity of the Saccaromyces cerevisiae protein YFR042W or its homologs, e.g. as indicated in Table I, columns 5 or 7, line 58, e.g. a "protein required for cell viability in yeast" is increased, preferably, in one embodment the increase of the fine chemical, preferably of glutamine, between 41% and 43% or more is conferred.
In case the activity of the Saccharomyces cerevisiae protein YGR135W or its homologs, e.g. as indicated in Table I, columns 5 or 7, line 50, e.g. a proteasome component Y13, involved in cytoplasmic and nuclear degradation, endoplasmic reticulum, nucleus, cell differentiation, proteasomal degradation is increased, preferably, in one embodiment an increase of the fine chemical, preferably of proline between 32% and 289% or more is conferred.
In case the activity of the Saccharomyces cerevisiae protein YHR130C or its homologs, e.g. as indicated in Table I, columns 5 or 7, line 31, e.g. a "hypothetical protein YBR030W" is increased, preferably, in one embodiment an increase of the fine chemical, preferably of arginine between 67% and 85% or more is conferred.
In case the activity of the Saccharomyces cerevisiae protein YIL150C or its homologs, as indicated in Table I, columns 5 or 7, line 51, e.g. a chromatin binding protein, required for S-phase (DNA synthesis) initiation or completion, involved in DNA synthesis and replication, mitotic cell cycle and cell cycle control, is increased, preferably, in one embodiment the increase of the fine chemical, preferably of proline between 33% and 304% or more is conferred.
In case the activity of the Saccharomyces cerevisiae protein YPR024W or its homologs e.g. as indicated in Table I, columns 5 or 7, line 41, e.g. a mitochondrial protein of the CDC48/PAS1/SEC18 family of ATPases, required for assembly of protein complexes, other proteolytic degradation, mitochondrion, protein folding and stabilization, is increased, preferably, in one embodiment the increase of the fine chemical, preferably of glutamate between 26% and 43% or more is conferred.
In case the activity of the Saccharomyces cerevisiae protein YPR133W-A or its homologs, e.g. as indicated in Table I, columns 5 or 7, line 42, e.g. a translocase of the outer mitochondrial membrane, is increased, preferably, in one embodiment the increase of the fine chemical, preferably of glutamate between 34% and 68% or more is conferred.
In case the activity of the Saccharomyces cerevisiae protein YPR138C or its homologs, e.g. as indicated in Table I, columns 5 or 7, line 53, e.g. a ammonium transport protein, involved in anion transports (Cl⁻, SO₄²⁻, PO₄³⁻, etc.), other cation transports (Na⁺, K⁺, Ca²⁺, NH₄⁺, etc.), nitrogen and sulfur transport, cellular import, transport through plasma membrane, is increased, preferably, in one embodiment the increase of the fine chemical, preferably of proline between 54% and 520% or more is conferred.
In case the activity of the Saccharomyces cerevisiae protein YBR204C or its homologs, e.g. as indicated in Table I, columns 5 or 7, line 38, e.g. a peroxisomal lipase, involved in breakdown of lipids, fatty acids and isoprenoids, peroxisome, is increased, preferably, in one embodiment the increase of the fine chemical, preferably of glutamate between 55% and 76% or more is conferred.
In case the activity of the Saccharomyces cerevisiae protein YDR271C or its homologs, e.g. as indicated in Table I, columns 5 or 7, line 46, e.g. a "hypothetical protein YDR271C" is increased, preferably, in one embodiment an increase of the fine chemical, preferably of proline, between 36% and 482% or more is conferred. In case the activity of the Saccharomyces cerevisiae protein YDR316W or its homologs, e.g. as indicated in Table I, columns 5 or 7, line 30, e.g. a S-adenosylmethionine-dependent methyltransferase is increased, preferably, in one embodiment the increase of the fine chemical, preferably of arginine between 45% and 102% or more is conferred.
In case the activity of the Saccharomyces cerevisiae protein YEL045C or its homologs, e.g. as indicated in Table I, columns 5 or 7, line 47, e.g. a "hypothetical protein YBR030W" is increased, preferably, in one embodiment the increase of the fine chemical, preferably of proline between 41% and 89% or more is conferred.
In case the activity of the Saccharomyces cerevisiae protein YER173w or its homologs, e.g. as indicated in Table I, columns 5 or 7, line 48 or 57, e.g. a checkpoint protein, involved in the activation of the DNA damage and meiotic pachytene checkpoints; DNA recombination and DNA repair, cell cycle checkpoints (checkpoints of morphogenesis, DNA-damage,-replication, mitotic phase and spindle), nucleic acid binding, DNA synthesis and replication is increased, preferably, in one embodiment the increase of the fine chemical between 34% and 285%, preferably of glutamine between 86% and 285% and/or of proline between 34% and 191% or more is conferred.
In case the activity of the Saccharomyces cerevisiae protein YFL013C or its homologs, e.g. as indicated in Table I, columns 5 or 7, line 39, e.g. a "subunit of the INO80 chromatin remodeling complex" is increased, preferably, in one embodiment an increase of the fine chemical, preferably of glutamate, between 81 % and 134% or more is conferred.
In case the activity of the Saccharomyces cerevisiae protein YFL050C or its homologs, e.g. as indicated in Table I, columns 5 or 7, line 49, e.g. a di-, tri-valent inorganic cation transporte, involved in heavy metal ion transports (Cu, Fe, etc.), cellular import, detoxification, homeostasis of metal ions (Na, K, Ca etc.), transport through plasma membrane is increased, preferably, in one embodiment an increase of the fine chemical, preferably of proline, between 44% and 74% or more is conferred. In case the activity of the Saccharomyces cerevisiae protein YGR104C or its
homologs, e.g. as indicated in Table I, columns 5 or 7, line 40, e.g. a "RNA polymerase II suppressor protein SRB5 - yeast and/or suppressor of RNA polymerase B SRB5" involved in transcription activities is increased, preferably, in one embodiment the increase of the fine chemical, preferably of glutamate, between 64% and 96% or more is conferred.
In case the activity of the Saccharomyces cerevisiae protein YKR057W or its homologs, e.g. as indicated in Table I, columns 5 or 7, line 32 or 59, e.g. a ribosomal protein, similar to S21A, S26A and/or YS25 ribosomal proteins, involved in ribosome biogenesis, cell differentiation and translation is increased, preferably, in one embodiment an increase of the fine chemical between 41 % and 457%, preferably of glutamine between 41 % and 284% and/or of arginine between 57% and 457% or more is conferred. In case the activity of the Escherichia coli K12 protein b0050 or its homologs e.g. a conserved protein potentially involved in protein protein interaction e.g. as indicated in Table II, columns 5 or 7, line 386, is increased, preferably, in one embodiment the increase of the fine chemical, preferably of glutamate between 37% and 97% or more is conferred.
In case the activity of the Escherichia coli K12 protein b0057 or its homologs e.g. a protein with an activity as defined in [0022.0.4.4],e.g. as indicated in Table II, columns 5 or 7, line 387, is increased, preferably, in one embodiment the increase of the fine chemical, preferably of glutamate between 35% and 83% or more is conferred.
In case the activity of the Escherichia coli K12 protein b0138 or its homologs e.g. a fimbrial-like adhesin protein e.g. as indicated in Table II, columns 5 or 7, line 388, is increased, preferably, in one embodiment the increase of the fine chemical, preferably of proline between 50% and 180% or more is conferred.
In case the activity of the Escherichia coli K12 protein b0149 or its homologs e.g. a bifunctional penicillin-binding protein 1 b: glycosyl transferase (N-terminal); transpeptidase (C-terminal)e.g. as indicated in Table II, columns 5 or 7, line 389, is increased, preferably, in one embodiment the increase of the fine chemical, preferably of proline between 33% and 120% or more is conferred.
In case the activity of the Escherichia coli K12 protein b0161 or its homologs e.g. a periplasmic serine protease e.g. as indicated in Table II, columns 5 or 7, lines 390 to 392, is increased, preferably, in one embodiment the increase of the fine chemical, preferably of arginine between 628% and 881 % or more, preferably of glutamate between 35% and 65% or more, preferably of glutamine between 43% and 256% or more, preferably of arginine and glutamate between 35% and 881 % or more, preferably of arginine and glutamine between 43% and 881% or more, preferably of glutamate and glutamine between 35% and 256% or more, preferably of arginine and glutamate and glutamine between 35% and 881% or more is conferred.
In case the activity of the Escherichia coli K12 protein b0486 or its homologs e.g. a amino-acid/amine transport protein (APC family) e.g. as indicated in Table II, columns 5 or 7, line 393, is increased, preferably, in one embodiment the increase of the fine respective chemical, preferably of glutamine between 51 % and 128% or more, is conferred.
In case the activity of the Escherichia coli K12 protein b0849 or its homologs e.g. a glutaredoxin 1 redox coenzyme for glutathione-dependent ribonucleotide reductase e.g. as indicated in Table II, columns 5 or 7, line 394, is increased, preferably, in one embodiment the increase of the fine chemical, preferably of glutamine between 37% and 50% or more is conferred.
In case the activity of the Escherichia coli K12 protein b0970 or its homologs e.g. a glutamate receptor e.g. as indicated in Table II, columns 5 or 7, line 395, is increased, preferably, in one embodiment the increase of the fine respective chemical, preferably of glutamine between 59% and 380% or more, is conferred.
In one embodiment, in case the activity of the Escherichia coli K12 protein b1343 or its homologs, e.g. as indicated in Table II, columns 5 or 7, line 396 and 397, is increased, e.g. the activity of a protein involved in rRNA processing and/or translation is increased, preferred the activity of a ATP-dependent RNA helicase, stimulated by 23S rRNA or its homolog is increased. Preferably, an increase of the respective fine chemical preferably of glutamine between 37% and 39% or more is conferred, preferably of glutamate between 48% and 99% or more is conferred, preferably of glutamine and glutamate between 37% and 99% or more is conferred.
In case the activity of the Escherichia coli K12 protein b1360 or a protein with the activity defined as putative DNA replication protein or its homologs, e.g. transcriptional regulator, e.g. as indicated in Table II, columns 5 or 7, line 398 is increased, preferably, in one embodiment an increase of the fine chemical, preferably of proline between 33% and 70% or more is conferred.
In case the activity of the Escherichia coli K12 protein b1693 or its homologs e.g. a 3-dehydroquinate dehydratase e.g. as indicated in Table II, columns 5 or 7, line 399, is increased, preferably, in one embodiment the increase of the fine chemical, preferably of glutamate between 39% and 149% or more is conferred.
In case the activity of the Escherichia coli K12 protein b1736 or its homologs e.g. a PEP-dependent phosphotransferase enzyme, e.g. as indicated in Table II, columns 5 or 7, line 400, is increased, preferably, in one embodiment the increase of the fine chemical, preferably of glutamate between 46% and 97% or more is conferred.
In one embodiment, in case the activity of the Escherichia coli K12 protein b1738 or a protein with the activity defined as PEP-dependent phosphotransferase or its homologs, e.g. as indicated in Table II, columns 5 or 7, line 401, is increased, preferably, in one embodiment an increase of the fine chemical preferably of glutamate between 38% and 107% or more is conferred.
In one embodiment, in case the activity of the Escherichia coli K12 protein b1886 or a methyl-accepting chemotaxis protein II, aspartate sensor receptor or its homologs, e.g. as indicated in Table II, columns 5 or 7, line 402, is increased, preferably, in one embodiment an increase of the fine chemical preferably of glutamine between 36% and 124% or more is conferred.
In one embodiment, in case the activity of the Escherichia coli K12 protein b1896 or a trehalose-6-phosphate synthase or its homologs, e.g. as indicated in Table II, columns 5 or 7, line 403, is increased, preferably, in one embodiment an increase of the fine chemical preferably of glutamate between 67% and 162% or more is conferred.
In one embodiment, in case the activity of the Escherichia coli K12 protein b1926 or a flagellar protein fliT or its homologs, e.g. as indicated in Table II, columns 5 or 7, line 404, is increased, preferably, in one embodiment an increase of the fine chemical preferably of glutamine between 7% and 27% or morre is conferred.
In one embodiment, in case the activity of the Escherichia coli K12 protein b2307 or a histidine and lysine/arginine/ornithine transport protein (ABC superfamily, membrane) or its homologs, e.g. as indicated in Table II, columns 5 or 7, line 405 and 406, is increased, preferably, in one embodiment an increase of the fine chemical, preferably of arginine between 95% and 247% or more, preferably of glutamate between 35% and 89% or more, preferably of arginine and glutamatne between 35 and 247% or more is conferred.
In one embodiment, in case the activity of the Escherichia coli K12 protein b2414 or its homologs, e.g. as indicated in Table II, columns 5 or 7, line 407, is increased, e.g. the activity of a protein of the threonine dehydratase-superfamily is increased preferably the activity of a protein involved in amino acid biosynthesis, biosynthesis of the cysteine-aromatic group, degradation of amino acids of the cysteine-aromatic group, nitrogen and sulfur utilizationbiosynthesis of the aspartate family, degradation of amino acids of the aspartate group, biosynthesis of sulfuric acid and L-cysteine derivatives, biosynthesis of secondary products derived from primary amino acids, biosynthesis of secondary products derived from glycine, L-serine and L-alanine, pyridoxal phosphate binding is increased, preferred the activity of a subunit of cysteine synthase A and O-acetylserine sulfhydrolase A, PLP-dependent enzyme or its homolog is increased. Preferably, an increase of the respective fine chemical, preferably of glutamine between 30% and 56% or more is conferred.
In one embodiment, in case the activity of the Escherichia coli K12 protein b2426 or its homologs, e.g. as indicated in Table II, columns 5 or 7, line 408, is increased, e.g. the activity of a oxidoreductase with NAD(P)-binding domain is increased. Preferably, an increase of the respective fine chemical, preferably of glutamine between 31% and 62% or more is conferred.
In one embodiment, in case the activity of the Escherichia coli K12 protein b2489 or its homologs, e.g. as indicated in Table II, columns 5 or 7, line 409, is increased, e.g. the activity of a hydrogenase Fe-S subunit is increased. Preferably, an increase of the respective fine chemical, preferably of glutamine between 33% and 44% or more is conferred.
In one embodiment, in case the activity of the Escherichia coli K12 protein b2553 or its homologs, e.g. as indicated in Table II, columns 5 or 7, line 410 and 411, is increased, e.g. the activity of a regulatory protein P-II for glutamine synthetase is increased. Preferably, an increase of the respective fine chemical, preferably of glutamine between 55% and 90% or more, preferably of proline between 49% and 68% or more, preferably of glutamine and proline between 49% and 90% or more is conferred.
In one embodiment, in case the activity of the Escherichia coli K12 protein b2644 or its homologs, e.g. as indicated in Table II, columns 5 or 7, line 412, is increased, e.g. the activity of a hydrogenase Fe-S subunit is increased. Preferably, an increase of the respective fine chemical, preferably of proline between 35% and 853% or more is conferred.
In case the activity of the Escherichia coli K12 protein b2710 or its homologs e.g. a flavorubredoxin (FIRd) bifunctional NO and O₂ reductase e.g. as indicated in Table II, columns 5 or 7, line 413, is increased, preferably, in one embodiment the increase of the fine chemical, preferably of glutamate between 35% and 38% or more is conferred. In one embodiment, in case the activity of the Escherichia coli K12 protein b2818 or its homologs, e.g. as indicated in Table I, columns 5 or 7, line 414 and 415, is increased, e.g. the activity of a N-acetylglutamate synthase (amino acid N-acetyltransferase is increased. Preferably, an increase of the respective fine chemical, preferably of glutamate between 50% and 129% or more, preferably of glutamine between 45% and 519% or more, preferably of glutamate and glutamine between 45% and 519% or more is conferred.
In case the activity of the Escherichia coli K12 protein b3064 or its homologs e.g. a putative O-sialoglycoprotein endopeptidase, with actin-like ATPase domain e.g. as indicated in Table II, columns 5 or 7, line 416, is increased, preferably, in one embodiment the increase of the fine chemical, preferably of glutamine between 72% and 141% or more is conferred.
In case the activity of the Escherichia coli K12 protein b3074 or its homologs, e.g. as indicated in Table II, columns 5 or 7, line 417, is increased, e.g. the activity of a tRNA synthetase is increased, preferably, an increase of the respective fine chemical, preferably of glutamate between 34% and 85% or more is conferred.
In case the activity of the Escherichia coli K12 protein b3116 or its homologs, e.g. as indicated in Table II, columns 5 or 7, line 418, is increased, e.g. the activity of a L-threonine/L-serine permease, anaerobically inducible (HAAAP family) is increased, preferably, an increase of the respective fine chemical, preferably of glutamate between 35% and 98% or more is conferred.
In case the activity of the Escherichia coli K12 protein b3160 or its homologs, e.g. as indicated in Table II, columns 5 or 7, line 419, is increased, e.g. the activity of a monooxygenase with luciferase-like ATPase activity is increased, preferably, an increase of the respective fine chemical, preferably of glutamine between 38% and 189% or more is conferred.
In case the activity of the Escherichia coli K12 protein b3166 or its homologs e.g. a tRNA pseudouridine 5S synthase e.g. as indicated in Table II, columns 5 or 7, line 420, is increased, preferably, in one embodiment the increase of the fine chemical, preferably of glutamine between 29% and 40% or more is conferred.
In case the activity of the Escherichia coli K12 protein b3169 or its homologs e.g. a transcription termination-antitermination factor e.g. as indicated in Table II, columns 5 or 7, line 421 and 422, is increased, preferably, in one embodiment the increase of the fine chemical, preferably of glutamine between 55% and 111 % or more, preferably of glutamate between 42% and 140% or more, preferably of glutamine and glutamate between 42% and 140% or more is conferred.
In case the activity of the Escherichia coli K12 protein b3231 or its homologs e.g. a 50S ribosomal subunit protein L13 e.g. as indicated in Table II, columns 5 or 7, line 423, is increased, preferably, in one embodiment the increase of the fine chemical, preferably of glutamine between 50% and 164% or more is conferred.
In case the activity of the Escherichia coli K12 protein b3619 or its homologs e.g. a ADP-L-glycero-D-mannoheptose-6-epimerase, NAD(P)-binding e.g. as indicated in Table II, columns 5 or 7, line 424, is increased, preferably, in one embodiment the increase of the fine chemical, preferably of glutamate between 40% and 122% or more is conferred.
In case the activity of the Escherichia coli K12 protein b3644 or its homologs e.g. an uncharacterized stress-induced protein e.g. as indicated in Table II, columns 5 or 7, line 425, is increased, preferably, in one embodiment the increase of the fine chemical, preferably of proline between 32% and 241% or more is conferred.
In case the activity of the Escherichia coli K12 protein b3680 or its homologs e.g. an uncharacterized stress-induced protein e.g. as indicated in Table II, columns 5 or 7, line 426, is increased, preferably, in one embodiment the increase of the fine chemical, preferably of glutamine between 50% and 199% or more is conferred.
In case the activity of the Escherichia coli K12 protein b3791 or its homologs e.g. an uncharacterized stress-induced protein e.g. as indicated in Table II, columns 5 or 7, line 427 and 428, is increased, preferably, in one embodiment the increase of the fine chemical, preferably of glutamine between 28% and 57% or more, preferably of glutamate between 39% and 57% or more, preferably of glutamine and glutamate between 28% and 57% or more is conferred.
In case the activity of the Escherichia coli K12 protein b3919 or its homologs e.g. an triosephosphate isomerase e.g. as indicated in Table II, columns 5 or 7, line 429, is increased, preferably, in one embodiment the increase of the fine chemical, preferably of proline between 35% and 118% or more is conferred.
In case the activity of the Escherichia coli K12 protein b3936 or its homologs e.g. an 50S ribosomal subunit protein L32 e.g. as indicated in Table II, columns 5 or 7, line 430, is increased, preferably, in one embodiment the increase of the fine chemical, preferably of arginine between 120% and 398% or more is conferred.
In case the activity of the Escherichia coli K12 protein b4004 or its homologs e.g. a transcriptional regulatory protein e.g. as indicated in Table II, columns 5 or 7, line 431, is increased, preferably, in one embodiment the increase of the fine chemical, preferably of glutamine between 30% and 36% or more is conferred.
In case the activity of the Escherichia coli K12 protein b4074 or its homologs e.g. a cytochrome c-type biogenesis protein e.g. as indicated in Table II, columns 5 or 7, line 432, is increased, preferably, in one embodiment the increase of the fine chemical, preferably of glutamine between 40% and 42% or more is conferred.
In case the activity of the Escherichia coli K12 protein b4133 or its homologs e.g. a transcriptional activator of pH response (OmpR family) e.g. as indicated in Table II, columns 5 or 7, line 433, is increased, preferably, in one embodiment the increase of the fine chemical, preferably of glutamine between 59% and 212% or more is conferred.
In case the activity of the Escherichia coli K12 protein b4346 or its homologs e.g. a component of 5-methylcytosine-specific restriction enzyme McrBC e.g. as indicated in Table II, columns 5 or 7, line 434, is increased, preferably, in one embodiment the increase of the fine chemical, preferably of glutamate between 38% and 44% or more is conferred.
In case the activity of the Saccharomyces cerevisiae protein YFL019C or its homologs e.g. a protein with the activity as indicated in [0022.0.4.4] e.g. as indicated in Table II, columns 5 or 7, line 435, is increased, preferably, in one embodiment the increase of the fine chemical, preferably of glutamate between 81 % and 134% or more is conferred.

**[0046.0.4.4]** In case the activity of the Escherichia coli K12 protein b0695 or its homologs, e.g. as indicated in Table I, columns 5 or 7, line 35, e.g. a sensory histidine kinase is increased, preferably an increase of the fine chemical and of phenylalanine is conferred.
In case the activity of the Escherichia coli K12 protein b0730 or its homologs, e.g. as indicated in Table I, columns 5 or 7, line 43 or 54, e.g. a transcriptional regulator is increased, preferably an increase of the fine chemical and of fumerate is conferred.
In case the activity of the Escherichia coli K12 protein b1284 or its homologs, e.g. as indicated in Table I, columns 5 or 7, line 36, e.g. a transcriptional regulator is increased, preferably an increase of the fine chemical and of fumaric acid is conferred. In case the activity of the Escherichia coli K12 protein b1827 or its homologs, e.g. as indicated in Table I, columns 5 or 7, line 56, e.g. a transcriptional repressor is increased, preferably an increase of the fine chemical and of isoleucince is conferred. In case the activity of the Escherichia coli K12 protein b1829 or its homologs, e.g. as indicated in Table I, columns 5 or 7, line 34 or 60, is increased, e.g. the activity of a heat shock protein with protease activity (htpx is increased, preferably an increase of the fine chemical and of isoleucine is conferred.
In case the activity of the Escherichia coli K12 protein b1852 or its homologs, e.g. as indicated in Table I, columns 5 or 7, line 61, is increased, e.g. the activity of a glucose-6-phosphate dehydrogenase is increased, preferably an increase of the fine chemical and of myoinositol is conferred.
In case the activity of the Escherichia coli K12 protein b2095 or its homologs, e.g. as indicated in Table I, columns 5 or 7, line 37, is increased, e.g. the activity of a tagatose-6-phosphate kinase is increased preferably an increase of the fine chemical and of alanine is conferred.
In case the activity of the Escherichia coli K12 protein b2699 or its homologs, e.g. as indicated in Table I, columns 5 or 7, line 55, is increased, e.g. the activity of a recombination protein recA is increased, preferably an increase of the fine chemical and of fumerate is conferred.
In case the activity of the Saccaromyces cerevisiae protein YFR042W or its homologs, e.g. as indicated in Table I, columns 5 or 7, line 58, e.g. a "protein required for cell viability in yeast" is increased, preferably an increase of the fine chemical and of Leucine is conferred.
In case the activity of the Saccharomyces cerevisiae protein YHR130C or its homologs, e.g. as indicated in Table I, columns 5 or 7, line 31, e.g. a "uncharacterized protein YHR130C" is increased, preferably an increase of the fine chemical and of phenylalanine is conferred.
In case the activity of the Saccharomyces cerevisiae protein YIL150C or its homologs, e.g. as indicated in Table I, columns 5 or 7, line 51, e.g. a chromatin binding protein is increased, preferably an increase of the fine chemical and of valine is conferred.
In case the activity of the Saccharomyces cerevisiae protein YPR024W or its homologs, e.g. as indicated in Table I, columns 5 or 7, line 41, e.g. a mitochondrial protein of the CDC48/PAS1/SEC18 family of ATPases is increased, preferably an increase of the fine chemical and of fumerate is conferred.
In case the activity of the Saccharomyces cerevisiae protein YPR138C or its homologs, e.g. as indicated in Table I, columns 5 or 7, line 53, e.g. a ammonium transport protein is increased, preferably an increase of the fine chemical and of phenylalanine is conferred.
In case the activity of the Saccharomyces cerevisiae protein YBR204C or its homologs, e.g. as indicated in Table I, columns 5 or 7, line 38, e.g. a peroxisomal lipase is increased, preferably an increase of the fine chemical and of inositol is conferred.
In case the activity of the Saccharomyces cerevisiae protein YDR271C or its homologs, e.g. as indicated in Table I, columns 5 or 7, line 46, e.g. a "uncharacterized protein YDR271C" is increased, preferably an increase of the fine chemical and of isoleucine is conferred.
In case the activity of the Saccharomyces cerevisiae protein YER173W or its homologs, e.g. as indicated in Table I, columns 5 or 7, line 48 or 57, e.g. a checkpoint protein is increased, preferably an increase of the fine chemical and of valine is conferred.
In case the activity of the Saccharomyces cerevisiae protein YFL013C or its homologs, e.g. as indicated in Table I, columns 5 or 7, line 39, e.g. a "subunit of the INO80 chromatin remodeling complex" is increased, preferably an increase of the fine chemical and of valine is conferred.
In case the activity of the Saccharomyces cerevisiae protein YFL050C or its homologs, e.g. as indicated in Table I, columns 5 or 7, line 49, e.g. a di-, tri-valent inorganic cation transporter is increased, preferably an increase of the fine chemical and of threonine is conferred.
In case the activity of the Saccharomyces cerevisiae protein YGR104C or its homologs, e.g. as indicated in Table I, columns 5 or 7, line 40, e.g. a "RNA polymerase II suppressor protein SRB5 - yeast and/or suppressor of RNA polymerase B SRB5" is increased, preferably an increase of the fine chemical and of isoleucine is conferred.
In case the activity of the Saccharomyces cerevisiae protein YKR057W or its homologs, e.g. as indicated in Table I, columns 5 or 7, line 32 or 59, e.g. a ribosomal protein, similar to S21A, S26A and/or YS25 ribosomal proteins is increased, preferably an increase of the fine chemical and of threonine is conferred.
In case the activity of the Escherichia coli K12 protein b0050 or its homologs e.g. a conserved protein potentially involved in protein protein interaction e.g. as indicated in Table II, columns 5 or 7, line 386, is increased, preferably, in one embodiment the increase of the fine chemical, preferably of glutamate and of an other amino acid or more is conferred.
In case the activity of the Escherichia coli K12 protein b0057 or its homologs e.g. a protein with an activity as defined in [0022.0.4.4],e.g. as indicated in Table II, columns 5 or 7, line 387, is increased, preferably, in one embodiment the increase of the fine chemical, preferably of glutamate and of an other amino acid or more is conferred.
In case the activity of the Escherichia coli K12 protein b0138 or its homologs e.g. a fimbrial-like adhesin protein e.g. as indicated in Table II, columns 5 or 7, line 388, is increased, preferably, in one embodiment the increase of the fine chemical, preferably of proline and of one or more other amino acid(s) or more is conferred.
In case the activity of the Escherichia coli K12 protein b0149 or its homologs e.g. a bifunctional penicillin-binding protein 1 b: glycosyl transferase (N-terminal); transpeptidase (C-terminal)e.g. as indicated in Table II, columns 5 or 7, line 389, is increased, preferably, in one embodiment the increase of the fine chemical, preferably of proline and of one or more other amino acid(s)o(s) is conferred.
In case the activity of the Escherichia coli K12 protein b0161 or its homologs e.g. a periplasmic serine protease e.g. as indicated in Table II, columns 5 or 7, lines 390 to 392, is increased, preferably, in one embodiment the increase of the fine chemical, preferably of arginine and/or of glutamate and/or of glutamine and of one or more other amino acid(s) is conferred.
In case the activity of the Escherichia coli K12 protein b0486 or its homologs e.g. a amino-acid/amine transport protein (APC family) e.g. as indicated in Table II, columns 5 or 7, line 393, is increased, preferably, in one embodiment the increase of the fine respective chemical, preferably of glutamine and of one or more other amino acid(s) is conferred.
In case the activity of the Escherichia coli K12 protein b0849 or its homologs e.g. a glutaredoxin 1 redox coenzyme for glutathione-dependent ribonucleotide reductase e.g. as indicated in Table II, columns 5 or 7, line 394, is increased, preferably, in one embodiment the increase of the fine chemical, preferably of glutamine and of one or more other amino acid(s) is conferred.
In case the activity of the Escherichia coli K12 protein b0970 or its homologs e.g. a glutamate receptor e.g. as indicated in Table II, columns 5 or 7, line 395, is increased, preferably, in one embodiment the increase of the fine respective chemical, preferably of glutamine and of one or more other amino(s) acid(s) is conferred.
In one embodiment, in case the activity of the Escherichia coli K12 protein b1343 or its homologs, e.g. as indicated in Table II, columns 5 or 7, line 396 and 397, is increased, e.g. the activity of a protein involved in rRNA processing and/or translation is increased, preferred the activity of a ATP-dependent RNA helicase, stimulated by 23S rRNA or its homolog is increased. Preferably, an increase of the respective fine chemical preferably of glutamine and/or of glutamate and of one or more other amino acid(s) is conferred.
In case the activity of the Escherichia coli K12 protein b1360 or a protein with the activity defined as putative DNA replication protein or its homologs, e.g. transcriptional regulator, e.g. as indicated in Table II, columns 5 or 7, line 398 is increased, preferably, in one embodiment an increase of the fine chemical, preferably of proline between and of one or more other amino acid(s) conferred.
In case the activity of the Escherichia coli K12 protein b1693 or its homologs e.g. a 3-dehydroquinate dehydratase e.g. as indicated in Table II, columns 5 or 7, line 399, is increased, preferably, in one embodiment the increase of the fine chemical, preferably of glutamate and of one or more other amino acid(s) is conferred.
In case the activity of the Escherichia coli K12 protein b1736 or its homologs e.g. a PEP-dependent phosphotransferase enzyme, e.g. as indicated in Table II, columns 5 or 7, line 400, is increased, preferably, in one embodiment the increase of the fine chemical, preferably of glutamate and of one or more other amino acid(s) is conferred.
In one embodiment, in case the activity of the Escherichia coli K12 protein b1738 or a protein with the activity defined as PEP-dependent phosphotransferase or its homologs, e.g. as indicated in Table II, columns 5 or 7, line 401, is increased, preferably, in one embodiment an increase of the fine chemical preferably of glutamate and of one or more other amino acid(s) is conferred.
In one embodiment, in case the activity of the Escherichia coli K12 protein b1886 or a methyl-accepting chemotaxis protein II, aspartate sensor receptor or its homologs, e.g. as indicated in Table II, columns 5 or 7, line 402, is increased, preferably, in one embodiment an increase of the fine chemical preferably of glutamine and of one or more other amino acid(s) is conferred.
In one embodiment, in case the activity of the Escherichia coli K12 protein b1896 or a trehalose-6-phosphate synthase or its homologs, e.g. as indicated in Table II, columns 5 or 7, line 403, is increased, preferably, in one embodiment an increase of the fine chemical preferably of glutamate and of one or more other amino acid(s) is conferred. In one embodiment, in case the activity of the Escherichia coli K12 protein b1926 or a flagellar protein fliT or its homologs, e.g. as indicated in Table II, columns 5 or 7, line 404, is increased, preferably, in one embodiment an increase of the fine chemical preferably of glutamine and of one or more other amino acid(s) is conferred.
In one embodiment, in case the activity of the Escherichia coli K12 protein b2307 or a flagellar protein fliT or its homologs, e.g. as indicated in Table II, columns 5 or 7, line 405 and 406, is increased, preferably, in one embodiment an increase of the fine chemical, preferably of arginine and/or of glutamate and of one or more other amino acid(s) is conferred.
In one embodiment, in case the activity of the Escherichia coli K12 protein b2414 or its homologs, e.g. as indicated in Table II, columns 5 or 7, line 407, is increased, e.g. the activity of a protein of the threonine dehydratase-superfamily is increased preferably the activity of a protein involved in amino acid biosynthesis, biosynthesis of the cysteine-aromatic group, degradation of amino acids of the cysteine-aromatic group, nitrogen and sulfur utilizationbiosynthesis of the aspartate family, degradation of amino acids of the aspartate group, biosynthesis of sulfuric acid and L-cysteine derivatives, biosynthesis of secondary products derived from primary amino acids, biosynthesis of secondary products derived from glycine, L-serine and L-alanine, pyridoxal phosphate binding is increased, preferred the activity of a subunit of cysteine synthase A and O-acetylserine sulfhydrolase A, PLP-dependent enzyme or its homolog is increased. Preferably, an increase of the respective fine chemical, preferably of glutamine and of one or more other amino acid(s) is conferred.
In one embodiment, in case the activity of the Escherichia coli K12 protein b2426 or its homologs, e.g. as indicated in Table II, columns 5 or 7, line 408, is increased, e.g. the activity of a oxidoreductase with NAD(P)-binding domain is increased. Preferably, an increase of the respective fine chemical, preferably of glutamine and of one or more other amino acid(s) is conferred.
In one embodiment, in case the activity of the Escherichia coli K12 protein b2489 or its homologs, e.g. as indicated in Table II, columns 5 or 7, line 409, is increased, e.g. the activity of a hydrogenase Fe-S subunit is increased. Preferably, an increase of the respective fine chemical, preferably of glutamine and of one or more other amino acid(s) is conferred.
In one embodiment, in case the activity of the Escherichia coli K12 protein b2553 or its homolog, e.g. as indicated in Table II, columns 5 or 7, line 410 and 411, is increased, e.g. the activity of a regulatory protein P-II for glutamine synthetase is increased. Preferably, an increase of the respective fine chemical, preferably of glutamine and/or of proline and of one or more other amino acid(s) is conferred.
In one embodiment, in case the activity of the Escherichia coli K12 protein b2644 or its homologs, e.g. as indicated in Table II, columns 5 or 7, line 412, is increased, e.g. the activity of a hydrogenase Fe-S subunit is increased. Preferably, an increase of the respective fine chemical, preferably of proline and of one or more other amino acid(s) is conferred.
In case the activity of the Escherichia coli K12 protein b2710 or its homologs e.g. a flavorubredoxin (FIRd) bifunctional NO and O₂ reductase e.g. as indicated in Table II, columns 5 or 7, line 413, is increased, preferably, in one embodiment the increase of the fine chemical, preferably of glutamate and of one or more other amino acid(s) is conferred.
In one embodiment, in case the activity of the Escherichia coli K12 protein b2818 or its homologs, e.g. as indicated in Table I, columns 5 or 7, line 414 and 415, is increased, e.g. the activity of a N-acetylglutamate synthase (amino acid N-acetyltransferase is increased. Preferably, an increase of the respective fine chemical, preferably of glutamate and/or of glutamine and of one or more other amino acid(s) is conferred.
In case the activity of the Escherichia coli K12 protein b3064 or its homologs e.g. a putative O-sialoglycoprotein endopeptidase, with actin-like ATPase domain e.g. as indicated in Table II, columns 5 or 7, line 416, is increased, preferably, in one embodiment the increase of the fine chemical, preferably of glutamine and of one or more other amino acid(s) is conferred.
In case the activity of the Escherichia coli K12 protein b3074 or its homologs, e.g. as indicated in Table II, columns 5 or 7, line 417, is increased, e.g. the activity of a tRNA synthetase is increased, preferably, an increase of the respective fine chemical, preferably of glutamate and of one or more other amino acid(s) is conferred.
In case the activity of the Escherichia coli K12 protein b3116 or its homologs, e.g. as indicated in Table II, columns 5 or 7, line 418, is increased, e.g. the activity of a L-threonine/L-serine permease, anaerobically inducible (HAAAP family) is increased, preferably, an increase of the respective fine chemical, preferably of glutamate and of one or more other amino acid(s) is conferred.
In case the activity of the Escherichia coli K12 protein b3160 or its homologs, e.g. as indicated in Table II, columns 5 or 7, line 419, is increased, e.g. the activity of a monooxygenase with luciferase-like ATPase activity is increased, preferably, an increase of the respective fine chemical, preferably of glutamine and of one or more other amino acid(s) is conferred.
In case the activity of the Escherichia coli K12 protein b3166 or its homologs e.g. a tRNA pseudouridine 5S synthase e.g. as indicated in Table II, columns 5 or 7, line 420, is increased, preferably, in one embodiment the increase of the fine chemical, preferably of glutamine and of one or more other amino acid(s) is conferred.
In case the activity of the Escherichia coli K12 protein b3169 or its homologs e.g. a transcription termination-antitermination factor e.g. as indicated in Table II, columns 5 or 7, line 421 and 422, is increased, preferably, in one embodiment the increase of the fine chemical, preferably of glutamine and/or of glutamate and of one or more other amino acid(s) is conferred.
In case the activity of the Escherichia coli K12 protein b3231 or its homologs e.g. a 50S ribosomal subunit protein L13 e.g. as indicated in Table II, columns 5 or 7, line 423, is increased, preferably, in one embodiment the increase of the fine chemical, preferably of glutamine and of one or more other amino acid(s) is conferred.
In case the activity of the Escherichia coli K12 protein b3619 or its homologs e.g. a ADP-L-glycero-D-mannoheptose-6-epimerase, NAD(P)-binding e.g. as indicated in Table II, columns 5 or 7, line 424, is increased, preferably, in one embodiment the increase of the fine chemical, preferably of glutamate and of one or more other amino acid(s) is conferred.
In case the activity of the Escherichia coli K12 protein b3644 or its homologs e.g. an uncharacterized stress-induced protein e.g. as indicated in Table II, columns 5 or 7, line 425, is increased, preferably, in one embodiment the increase of the fine chemical, preferably of proline and of one or more other amino acid(s) is conferred.
In case the activity of the Escherichia coli K12 protein b3680 or its homologs e.g. an uncharacterized stress-induced protein e.g. as indicated in Table II, columns 5 or 7, line 426, is increased, preferably, in one embodiment the increase of the fine chemical, preferably of glutamine and of one or more other amino acid(s) is conferred.
In case the activity of the Escherichia coli K12 protein b3791 or its homologs e.g. an uncharacterized stress-induced protein e.g. as indicated in Table II, columns 5 or 7, line 427 and 428, is increased, preferably, in one embodiment the increase of the fine chemical, preferably of glutamine and/or of glutamate between and of one or more other amino acid(s) is conferred.
In case the activity of the Escherichia coli K12 protein b3919 or its homologs e.g. an triosephosphate isomerase e.g. as indicated in Table II, columns 5 or 7, line 429, is increased, preferably, in one embodiment the increase of the fine chemical, preferably of proline and of one or more other amino acid(s) is conferred.
In case the activity of the Escherichia coli K12 protein b3936 or its homologs e.g. an 50S ribosomal subunit protein L32 e.g. as indicated in Table II, columns 5 or 7, line 430, is increased, preferably, in one embodiment the increase of the fine chemical, preferably of arginine and of one or more other amino acid(s) is conferred.
In case the activity of the Escherichia coli K12 protein b4004 or its homologs e.g. a transcriptional regulatory protein e.g. as indicated in Table II, columns 5 or 7, line 431, is increased, preferably, in one embodiment the increase of the fine chemical, preferably of glutamine and of one or more other amino acid(s) is conferred.
In case the activity of the Escherichia coli K12 protein b4074 or its homologs e.g. a cytochrome c-type biogenesis protein e.g. as indicated in Table II, columns 5 or 7, line 432, is increased, preferably, in one embodiment the increase of the fine chemical, preferably of glutamine and of one or more other amino acid(s) is conferred.
In case the activity of the Escherichia coli K12 protein b4133 or its homologs e.g. a transcriptional activator of pH response (OmpR family) e.g. as indicated in Table II, columns 5 or 7, line 433, is increased, preferably, in one embodiment the increase of the fine chemical, preferably of glutamine and of one or more other amino acid(s) is conferred.
In case the activity of the Escherichia coli K12 protein b4346 or its homologs e.g. a component of 5-methylcytosine-specific restriction enzyme McrBC e.g. as indicated in Table II, columns 5 or 7, line 434, is increased, preferably, in one embodiment the increase of the fine chemical, preferably of glutamate and of one or more other amino acid(s) is conferred.
In case the activity of the Saccharomyces cerevisiae protein YFL019C or its homologs e.g. a protein with the activity as indicated in [0022.0.4.4] e.g. as indicated in Table II, columns 5 or 7, line 435, is increased, preferably, in one embodiment the increase of the fine chemical, preferably of glutamate and of one or more other amino acid(s) is conferred.

**[0047.0.0.4] and [0048.0.0.4]: see [0047.0.0.0] and [0048.0.0.0]**

**[0049.0.4.4]** A protein having an activity conferring an increase in the amount or level of arginine chemical preferably has the structure of the polypeptide described herein, in particular of a polypeptides comprising a consensus sequence as indicated in Table IV, columns 7, lines 30 to 37, 390, 405 and/or 430or of a polypeptide as indicaded in Table II, columns 5 or 7, lines 30 to 37, 390, 405 and/or 430or the functional homologues thereof as described herein, or is encoded by the nucleic acid molecule characterized herein or the nucleic acid molecule according to the invention, for example by a nucleic acid molecule as indicated in Table I, columns 5 or 7, lines 30 to 37, 390, 405 and/or 430 or its herein described functional homologues and has the herein mentioned activity.
A protein having an activity conferring an increase in the amount or level of glutamate preferably has the structure of the polypeptide described herein, in particular of a polypeptides comprising a consensus sequence as indicated in Table IV, column 7, lines 38 to 43, 386, 387, 391, 396, 399 to 401, 403, 406, 413, 414, 417, 418, 421, 424, 427, 434 and/or 435or of a polypeptide as indicaded in Table II, columns 5 or 7, lines 38 to 43, 386, 387, 391, 396, 399 to 401, 403, 406, 413, 414, 417, 418, 421, 424, 427, 434 and/or 435or the functional homologues thereof as described herein, or is encoded by the nucleic acid molecule characterized herein or the nucleic acid molecule according to the invention, for example by a nucleic acid molecule as indicaded in Table I, columns 5 or 7, lines 38 to 43, 386, 387, 391, 396, 399 to 401, 403, 406, 413, 414, 417, 418, 421, 424, 427, 434 and/or 435or its herein described functional homologues and has the herein mentioned activity.
A protein having an activity conferring an increase in the amount or level of proline preferably has the structure of the polypeptide described herein, in particular of a polypeptides comprising a consensus sequence as indicated in Table IV, column 7, lines 44 to 56, 388, 389, 398, 411, 412, 425 and/or 429or of a polypeptide as indicaded in Table II, columns 5 or 7, lines 44 to 56, 388, 389, 398, 411, 412, 425 and/or 429or the functional homologues thereof as described herein, or is encoded by the nucleic acid molecule characterized herein or the nucleic acid molecule according to the invention, for example by a nucleic acid molecule as indicaded in Table I, columns 5 or 7, lines 44 to 56, 388, 389, 398, 411, 412, 425 and/or 429or its herein described functional homologues and has the herein mentioned activity.
A protein having an activity conferring an increase in the amount or level of glutamine preferably has the structure of the polypeptide described herein, in particular of a polypeptides comprising a consensus sequence as indicated in Table IV, column 7, lines 57 to 62, 392 to 395, 397, 402, 404, 407 to 410, 415, 416, 419, 420, 422, 423, 426, 428 and/or 431 to 433or of a polypeptide as indicaded in Table II, columns 5 or 7, lines 57 to 62, 392 to 395, 397, 402, 404, 407 to 410, 415, 416, 419, 420, 422, 423, 426, 428 and/or 431 to 433or the functional homologues thereof as described herein, or is encoded by the nucleic acid molecule characterized herein or the nucleic acid molecule according to the invention, for example by a nucleic acid molecule as indicaded in Table I, columns 5 or 7, lines 57 to 62, 392 to 395, 397, 402, 404, 407 to 410, 415, 416, 419, 420, 422, 423, 426, 428 and/or 431 to 433or its herein described functional homologues and has the herein mentioned activity.

**[0050.0.4.4]** For the purposes of the present invention, the term "arginine" and/or "glutamate" and/or "glutamine" and/or "proline " and "L- arginine" and/or" L- glutamate" and/or "L-glutamine" and/or "L-proline" also encompass the corresponding salts, such as, for example, arginine- and/or glutamate- and/or glutamine- and/or proline-hydrochloride or arginine and/or glutamate and/or glutamine and/or proline sulfate. Preferably the term arginine and/or glutamate and/or glutamine and/or proline is intended to encompass the term L- arginine and/or L- glutamate and/or L-glutamine and/or L- proline.

**[0051.0.0.4] and [0052.0.0.4]: see [0051.0.0.0] and [0052.0.0.0]**

**[0053.0.4.4]** In one embodiment, the process of the present invention comprises one or more of the following steps
a) stabilizing a protein conferring the increased expression of a protein encoded by the nucleic acid molecule of the invention or of the polypeptid of the invention, e.g. of a polypeptide having an activity of a protein as indicated in Table II, column 3, lines 34 to 37, 390, 405 and/or 430 for arginine and/or lines 43, 386, 387, 391, 396, 399 to 401, 403, 406, 413, 414, 417, 418, 421, 424, 427, 434 and/or 435 for glutamate
   and/or lines 54 to 56, 388, 389, 398, 411, 412, 425 and/or 429 for proline
   and/or lines 62, 392 to 395, 397, 402, 404, 407 to 410, 415, 416, 419, 420, 422, 423, 426, 428 and/or 431 to 433 for glutamine resp., or its homologs, e.g. as indicated in Table II, columns 5 or 7, lines 34 to 37, 390, 405 and/or 430 for arginine
   and/or lines 43, 386, 387, 391, 396, 399 to 401, 403, 406, 413, 414, 417, 418, 421, 424, 427, 434 and/or 435 for glutamate
   and/or lines 54 to 56, 388, 389, 398, 411, 412, 425 and/or 429 for proline
   and/or lines 62, 392 to 395, 397, 402, 404, 407 to 410, 415, 416, 419, 420, 422, 423, 426, 428 and/or 431 to 433 for glutamine resp., activity having herein-mentioned the respective fine chemical-increasing activity;
b) stabilizing a mRNA conferring the increased expression of a protein encoded by the nucleic acid molecule of the invention, e.g. of a polypeptide having an activity of a protein as indicated in Table II, column 3, lines 34 to 37, 390, 405 and/or 430 and/or lines 43, 386, 387, 391, 396, 399 to 401, 403, 406, 413, 414, 417, 418, 421, 424, 427, 434 and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp.or its homologs activity, e.g. as indicated in Table II, columns 5 or 7, lines 34 to 37, 390, 405 and/or 430 and/or lines 43, 386, 387, 391, 396, 399 to 401, 403, 406, 413, 414, 417, 418, 421, 424, 427, 434 and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp or of a mRNA encoding the polypeptide of the present invention having herein-mentioned the respective fine chemical-increasing activity;
c) increasing the specific activity of a protein conferring the increased expression of a protein encoded by the nucleic acid molecule of the invention or of the polypeptide of the present invention having herein-mentioned the respective fine chemical-increasing activity, e.g. of a polypeptide having an activity of a protein as indicated in Table II, column 3, lines 34 to 37, 390, 405 and/or 430 and/or lines 43, 386, 387, 391, 396, 399 to 401, 403, 406, 413, 414, 417, 418, 421, 424, 427, 434 and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 respor its homologs activity, e.g. as indicated in Table II, columns 5 or 7, lines 34 to 37, 390, 405 and/or 430 and/or lines 43, 386, 387, 391, 396, 399 to 401, 403, 406, 413, 414, 417, 418, 421, 424, 427, 434 and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp or decreasing the inhibitiory regulation of the polypeptide of the invention;
d) generating or increasing the expression of an endogenous or artificial transcription factor mediating the expression of a protein conferring the increased expression of a protein encoded by the nucleic acid molecule of the invention or of the polypeptide of the invention having herein-mentioned the respective fine chemical-increasing activity, e.g. of a polypeptide having an activity of a protein as indicated in Table II, column 3, lines lines 34 to 37, 390, 405 and/or 430 and/or lines 43, 386, 387, 391, 396, 399 to 401, 403, 406, 413, 414, 417, 418, 421, 424, 427, 434 and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp or its homologs activity, e.g. as indicated in Table II, columns 5 or 7, lines 34 to 37, 390, 405 and/or 430 and/or lines 43, 386, 387, 391, 396, 399 to 401, 403, 406, 413, 414, 417, 418, 421, 424, 427, 434 and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp;
e) stimulating activity of a protein conferring the increased expression of a protein encoded by the nucleic acid molecule of the present invention or a polypeptide of the present invention having herein-mentioned the respective fine chemical-increasing activity, e.g. of a polypeptide having an activity of a protein as indicated in Table II, column 3, lines 34 to 37, 390, 405 and/or 430 and/or lines 43, 386, 387, 391, 396, 399 to 401, 403, 406, 413, 414, 417, 418, 421, 424, 427, 434 and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp or its homologs activity, e.g. as indicated in Table II, columns 5 or 7, lines 34 to 37, 390, 405 and/or 430 and/or lines 43, 386, 387, 391, 396, 399 to 401, 403, 406, 413, 414, 417, 418, 421, 424, 427, 434 and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp, by adding one or more exogenous inducing factors to the organismus or parts thereof;
f) expressing a transgenic gene encoding a protein conferring the increased expression of a polypeptide encoded by the nucleic acid molecule of the present invention or a polypeptide of the present invention, having herein-mentioned the respective fine chemical-increasing activity, e.g. of a polypeptide having an activity of a protein as indicated in Table II, column 3, lines 34 to 37, 390, 405 and/or 430 and/or lines 43, 386, 387, 391, 396, 399 to 401, 403, 406, 413, 414, 417, 418, 421, 424, 427, 434 and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp or its homologs activity, e.g. as indicated in Table II, columns 5 or 7, lines 34 to 37, 390, 405 and/or 430 and/or lines 43, 386, 387, 391, 396, 399 to 401, 403, 406, 413, 414, 417, 418, 421, 424, 427, 434 and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp, and/or
g) increasing the copy number of a gene conferring the increased expression of a nucleic acid molecule encoding a polypeptide encoded by the nucleic acid molecule of the invention or the polypeptide of the invention having herein-mentioned the respective fine chemical-increasing activity, e.g. of a polypeptide having an activity of a protein as indicated in Table II, column 3, lines 34 to 37, 390, 405 and/or 430 and/or lines 43, 386, 387, 391, 396, 399 to 401, 403, 406, 413, 414, 417, 418, 421, 424, 427, 434 and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp or its homologs, e.g. as indicated in Table II, columns 5 or 7, lines 34 to 37, 390, 405 and/or 430 and/or lines 43, 386, 387, 391, 396, 399 to 401, 403, 406, 413, 414, 417, 418, 421, 424, 427, 434 and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp, activity .
h) Increasing the expression of the endogenous gene encoding the polypeptide of the invention, e.g. a polypeptide having an activity of a protein as indicated in Table II, column 3, lines 34 to 37, 390, 405 and/or 430 and/or lines 43, 386, 387, 391, 396, 399 to 401, 403, 406, 413, 414, 417, 418, 421, 424, 427, 434 and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp or its homologs activity, e.g. as indicated in Table II, columns 5 or 7, lines 34 to 37, 390, 405 and/or 430 and/or lines 43, 386, 387, 391, 396, 399 to 401, 403, 406, 413, 414, 417, 418, 421, 424, 427, 434 and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp by adding positive expression or removing negative expression elements, e.g. homologous recombination can be used to either introduce positive regulatory elements like for plants the 35S enhancer into the promoter or to remove repressor elements form regulatory regions. Further gene conversion methods can be used to disrupt repressor elements or to enhance to acitivty of positive elements. Positive elements can be randomly introduced in plants by T-DNA or transposon mutagenesis and lines can be identified in which the positive elements have be integrated near to a gene of the invention, the expression of which is thereby enhanced;
i) Modulating growth conditions of an organism in such a manner, that the expression or activity of the gene encoding the protein of the invention or the protein itself is enhanced for example microorganisms or plants can be grown for example under a higher temperature regime leading to an enhanced expression of heat shock proteins, which can lead an enhanced the fine chemical production; and/or
j) selecting of organisms with expecially high activity of the proteins of the invention from natural or from mutagenized resources and breeding them into the target organisms, eg the elite crops.

**[0054.0.4.4]** Preferably, said mRNA is the nucleic acid molecule of the present invention and/or the protein conferring the increased expression of a protein encoded by the nucleic acid molecule of the present invention or the polypeptide having the herein mentioned activity is the polypeptide of the present invention, e.g. conferring the increase of arginine and/or glutamate and/or proline and/or glutamine after increasing the expression or activity of the encoded polypeptide or having the activity of a polypeptide having an activity of a protein according to Table II, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp. or its homologs activity, e.g. as indicated in Table II, columns 5 or 7, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp.

**[0055.0.0.4] to [0071.0.0.4]: see [0055.0.0.0] to [0071.0.0.0]**

**[0072.0.4.4]** By influencing the metabolism thus, it is possible to produce, in the process according to the invention, further advantageous compounds. Examples of such compounds are, in addition to arginine and/or glutamate and/or glutamine and/or proline Argininosuccinate, Citrulline, Ornithine, Urea, Pyrroline-5-carboxylate, Hydroxyproline, Hydroxypyrroline-carboxylate, 3-Hydroxypyrroline-5-carboxylate, γ-Glutamylcysteine, Glutathione, Hydroxyglutamate, 4-Hydroxyglutamate, Oxoglutarate, 4-Hydroxy-2-oxoglutarate, Glutamine.

**[0073.0.4.4]** Accordingly, in one embodiment, the process according to the invention relates to a process which comprises:
(c) providing a non-human organism, preferably a microorganism, a non-human animal, a plant or animal cell, a plant or animal tissue or a plant;
(d) increasing an activity of a polypeptide of the invention or a homolog thereof, e.g. as indicated in Table II, columns 5 or 7, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp. or of a polypeptide being encoded by the nucleic acid molecule of the present invention and described below, e.g. conferring an increase of the respective fine chemical in an organism, preferably in a microorganism, a non-human animal, a plant or animal cell, a plant or animal tissue or a plant,
(e) growing the organism, preferably the microorganism, the non-human animal, the plant or animal cell, the plant or animal tissue or the plant under conditions which permit the production of the fine chemical in the organism, preferably the microorganism, the plant cell, the plant tissue or the plant; and
(f) if desired, revovering, optionally isolating, the free and/or bound the fine chemical and , optionally further free and/or bound amino acids synthetized by the organism, the microorganism, the non-human animal, the plant or animal cell, the plant or animal tissue or the plant.

**[0074.0.0.4]** to **[0084.0.0.4]: see [0074.0.0.0]** to **[0084.0.0.0]**

**[0085.0.4.4]** With regard to the nucleic acid sequence as depicted a nucleic acid construct which contains a nucleic acid sequence mentioned herein or an organism (= transgenic organism) which is transformed with said nucleic acid sequence or said nucleic acid construct, "transgene" means all those constructs which have been brought about by genetic manipulation methods,preferably in which either
a) the nucleic acid sequence as indicated in Table I, columns 5 or 7, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp. or a derivative thereof, or
b) a genetic regulatory element, for example a promoter, which is functionally linked to the nucleic acid sequence as indicated in Table I, columns 5 or 7, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp. or a derivative thereof, or
c) (a) and (b)
is/are not present in its/their natural genetic environment or has/have been modified by means of genetic manipulation methods, it being possible for the modification to be, by way of example, a substitution, addition, deletion, inversion or insertion of one or more nucleotide. "Natural genetic environment" means the natural chromosomal locus in the organism of origin or the presence in a genomic library. In the case of a genomic library, the natural, genetic environment of the nucleic acid sequence is preferably at least partially still preserved. The environment flanks the nucleic acid sequence at least on one side and has a sequence length of at least 50 bp, preferably at least 500 bp, particularly preferably at least 1000 bp, very particularly preferably at least 5000 bp.

**[0086.0.0.4] and [0088.1.0.4]: see [0086.0.0.0] and [0088.1.0.0]**

**[0089.0.0.4]** to **[0097.0.0.4]: see [0089.0.0.0]** to **[0097.0.0.0]**

**[0098.0.4.4]** In a preferred embodiment, the fine chemical (arginine and/or glutamate and/or glutamine and/or proline) is produced in accordance with the invention and, if desired, is isolated. The production of further amino acids such as methionine, lysine and/or threonine mixtures of amino acid by the process according to the invention is advantageous.

**[0099.0.0.4] to [0102.0.0.4]: see [0099.0.0.0] to [0102.0.0.0]**

**[0103.0.4.4]** In a preferred embodiment, the present invention relates to a process for the production of the fine chemical comprising or generating in an organism or a part thereof the expression of at least one nucleic acid molecule comprising a nucleic acid molecule selected from the group consisting of:
a) nucleic acid molecule encoding, preferably at least the mature form, of the polypeptide having a sequence as indicated in Table II, columns 5 or 7, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp. or a fragment thereof, which confers an increase in the amount of the respective fine chemical in an organism or a part thereof;
b) nucleic acid molecule comprising, preferably at least the mature form, of a nucleic acid molecule having a sequence as indicated in Table I, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp.
c) nucleic acid molecule whose sequence can be deduced from a polypeptide sequence encoded by a nucleic acid molecule of (a) or (b) as result of the degeneracy of the genetic code and conferring an increase in the amount of the fine chemical in an organism or a part thereof;
d) nucleic acid molecule encoding a polypeptide which has at least 50% identity with the amino acid sequence of the polypeptide encoded by the nucleic acid molecule of (a) to (c) and conferring an increase in the amount of the fine chemical in an organism or a part thereof;
e) nucleic acid molecule which hybidizes with a nucleic acid molecule of (a) to (c) under under stringent hybridisation conditions and conferring an increase in the amount of the fine chemical in an organism or a part thereof;
f) nucleic acid molecule encoding a polypeptide, the polypeptide being derived by substituting, deleting and/or adding one or more amino acids of the amino acid sequence of the polypeptide encoded by the nucleic acid molecules (a) to (d), preferably to (a) to (c) and conferring an increase in the amount of the fine chemical in an organism or a part thereof;
g) nucleic acid molecule encoding a fragment or an epitope of a polypeptide which is encoded by one of the nucleic acid molecules of (a) to (e), preferably to (a) to (c) and and conferring an increase in the amount of the fine chemical in an organism or a part thereof;
h) nucleic acid molecule comprising a nucleic acid molecule which is obtained by amplifying nucleic acid molecules from a cDNA library or a genomic library using the primers pairs having a sequence as indicated in Table III, column 7, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp. and conferring an increase in the amount of the respective fine chemical in an organism or a part thereof;
i) nucleic acid molecule encoding a polypeptide which is isolated, e.g. from an expression library, with the aid of monoclonal antibodies against a polypeptide encoded by one of the nucleic acid molecules of (a) to (h), preferably to (a) to (c), and and conferring an increase in the amount of the fine chemical in an organism or a part thereof;
j) nucleic acid molecule which encodes a polypeptide comprising the consensus sequence having a sequences as indicated in Table IV, column 7, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp., and conferring an increase in the amount of the respective fine chemical in an organism or a part thereof ;
k) nucleic acid molecule comprising one or more of the nucleic acid molecule encoding the amino acid sequence of a polypeptide encoding a domain of the polypeptide indicated in Table II, columns 5 or 7, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp. and conferring an increase in the amount of the respective fine chemical in an organism or a part thereof; and
l) nucleic acid molecule which is obtainable by screening a suitable library under stringent conditions with a probe comprising one of the sequences of the nucleic acid molecule of (a) to (k), preferably to (a) to (c), or with a fragment of at least 15 nt, preferably 20 nt, 30 nt, 50 nt, 100 nt, 200 nt or 500 nt of the nucleic acid molecule characterized in (a) to (k), preferably to (a) to (c), and conferring an increase in the amount of the fine chemical in an organism or a part thereof;
or which comprises a sequence which is complementary thereto.

**[00103.1.0.4.]** In one embodiment, the nucleic acid molecule used in the process of the invention distinguishes over the sequence indicated in Table IA, columns 5 or 7, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp., by one or more nucleotides. In one embodiment, the nucleic acid molecule used in the process of the invention does not consist of the sequence shown in indicated in Table I A, columns 5 or 7, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp.. In one embodiment, the nucleic acid molecule used in the process of the invention is less than 100%, 99,999%, 99,99%, 99,9% or 99% identical to a sequence indicated in Table I A, columns 5 or 7, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp.. In another embodiment, the nucleic acid molecule does not encode a polypeptide of a sequence indicated in Table **II** A, columns 5 or 7, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp..

**[00103.2.0.4.]** In one embodiment, the nucleic acid molecule used in the process of the invention distinguishes over the sequence indicated in Table I B, columns 5 or 7, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp., by one or more nucleotides. In one embodiment, the nucleic acid molecule used in the process of the invention does not consist of the sequence shown in indicated in Table I B, columns 5 or 7, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp..: In one embodiment, the nucleic acid molecule used in the process of the invention is less than 100%, 99,999%, 99,99%, 99,9% or 99% identical to a sequence indicated in Table I B, columns 5 or 7, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp.. In another embodiment, the nucleic acid molecule does not encode a polypeptide of a sequence indicated in Table II B, columns 5 or 7, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp..

**[0104.0.4.4]** In one embodiment, the nucleic acid molecule used in the process distinguishes over the sequence indicated in Table I, columns 5 or 7, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp., by one or more nucleotides. In one embodiment, the nucleic acid molecule used in the process of the invention does not consist of the sequence indicated in Table I, columns 5 or 7, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp. In one embodiment, the nucleic acid molecule of the present invention is less than 100%, 99,999%, 99,99%, 99,9% or 99% identical to the sequence indicated in Table I, columns 5 or 7, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp. In another embodiment, the nucleic acid molecule does not encode a polypeptide of a sequence indicated in Table II, columns 5 or 7, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp.

**[0105.0.0.4] to [0107.0.0.4]: see [0105.0.0.0] to [0107.0.0.0]**

**[0108.0.4.4]** Nucleic acid molecules with the sequence as indicated in Table I, columns 5 or 7, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp., nucleic acid molecules which are derived from a amino acid sequences as indicated in Table II, columns 5 or 7, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp. or from polypeptides comprising the consensus sequence as indicated in Table IV, column 7, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp. or their derivatives or homologues encoding polypeptides with the enzymatic or biological activity of a polypeptide as indicated in Table I, column 3, 5 or 7, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp. or e.g. conferring a increase of the fine chemical after increasing its expression or activity are advantageously increased in the process according to the invention.

**[0109.0.0.4]: see [0109.0.0.0]**

**[0110.0.4.4]** Nucleic acid molecules, which are advantageous for the process according to the invention and which encode polypeptides with an activity of a polypeptide of the invention or the polypeptide used in the method of the invention or used in the process of the invention, e.g. of a protein as indicated in Table II, column 5, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 respor being encoded by a nucleic acid molecule indicated in Table I, column 5, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp.or of its homologs, e.g. as indicated in Table II, column 7, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp., can be determined from generally accessible databases.

**[0111.0.0.4]: see [0111.0.0.0]**

**[0112.0.4.4]** The nucleic acid molecules used in the process according to the invention take the form of isolated nucleic acid sequences, which encode polypeptides with protein activity of proteins as indicated in Table II, columns 5 or 7, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp., and conferring a arginine and/or glutamate and/or proline and/or glutamine increase.

**[0113.0.0.4] to [0120.0.0.4]: [0113.0.0.0] to [0120.0.0.0]**

**[0121.0.4.4]** However, it is also possible to use artificial sequences, which differ in one or more bases from the nucleic acid sequences found in organisms, or in one or more amino acid molecules from polypeptide sequences found in organisms, in particular from the polypeptide sequences indicated in Table II, columns 5 or 7, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp. or the functional homologues thereof as described herein, preferably conferring above-mentioned activity, i.e. conferring a increase of the respective fine chemical after increasing its activity.

**[0122.0.0.4]** to **[0127.0.0.4]: see [0122.0.0.0]** to **[0127.0.0.0]**

**[0128.0.4.4]** Synthetic oligonucleotide primers for the amplification, e.g. as the pairs indicated in Table III, column 7, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp. by means of polymerase chain reaction can be generated on the basis of a sequence shown herein, for example the sequence as indicated in Table I, columns 5 or 7, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp. or the sequences derived from a sequence as indicated in Table II, columns 5 or 7, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp.

**[0129.0.4.4]** Moreover, it is possible to identify conserved regions from various organisms by carrying out protein sequence alignments with the polypeptide used in the process of the invention, in particular with sequences of the polypeptide of the invention, from which conserved regions, and in turn, degenerate primers can be derived. Conserved region for the polypeptide of the invention are indicated in the alignments shown in the figures. Conserved regions are those, which show a very little variation in the amino acid in one particular position of several homologs from different origin. The consenus sequence indicated in Table IV, column 7, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp. is derived from said aligments.

**[0130.0.0.4] to [0138.0.0.4]: see [0130.0.0.0] to [0138.0.0.0]**

**[0139.0.4.4]** Polypeptides having above-mentioned activity, i.e. conferring the increase of the respective fine chemical level, derived from other organisms, can be encoded by other DNA sequences which hybridize to a sequences indicated in Table I, columns 5 or 7, preferably table I B, lines 34 to 37, 390, 405 and/or 430 for arginine and/or lines 43, 386, 387, 391, 396, 399 to 401, 403, 406, 413, 414, 417, 418, 421, 424, 427, 434, and/or 435 for glutamate
and/or lines 54 to 56, 388, 389, 398, 411, 412, 425 and/or 429 for proline
and/or lines 62, 392 to 395, 397, 402, 404, 407 to 410, 415, 416, 419, 420, 422, 423, 426, 428 and/or 431 to 433 for glutamine resp.,
under relaxed hybridization conditions and which code on expression for peptides having the respective fine chemical, in particular, of arginine and/or glutamate and/or proline and/or glutamine, resp., increasing activity.

**[0140.0.0.4] to [0146.0.0.4]: see [0140.0.0.0] to [0146.0.0.0]**

**[0147.0.4.4]** Further, the nucleic acid molecule of the invention comprises a nucleic acid molecule, which is a complement of one of the nucleotide sequences of above mentioned nucleic acid molecules or a portion thereof. A nucleic acid molecule which is complementary to one of the nucleotide sequences indicated in Table I, columns 5 or 7, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp., is one which is sufficiently complementary to one of said nucleotide sequences such that it can hybridize to one of said nucleotide sequences, thereby forming a stable duplex. Preferably, the hybridisation is performed under stringent hybrization conditions. However, a complement of one of the herein disclosed sequences is preferably a sequence complement thereto according to the base pairing of nucleic acid molecules well known to the skilled person. For example, the bases A and G undergo base pairing with the bases T and U or C, resp. and visa versa. Modifications of the bases can influence the base-pairing partner.

**[0148.0.4.4]** The nucleic acid molecule of the invention comprises a nucleotide sequence which is at least about 30%, 35%, 40% or 45%, preferably at least about 50%, 55%, 60% or 65%, more preferably at least about 70%, 80%, or 90%, and even more preferably at least about 95%, 97%, 98%, 99% or more homologous to a nucleotide sequence indicated in Table I, columns 5 or 7, preferably table I B, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp., preferably table I B or a portion thereof and preferably has above mentioned activity, in particular, of arginine and/or glutamate and/or proline and/or glutamine increasing activity after increasing the acitivity or an activity of a product of a gene encoding said sequences or their homologs.

**[0149.0.4.4]** The nucleic acid molecule of the invention or the nucleic acid molecule used in the method of the invention comprises a nucleotide sequence which hybridizes, preferably hybridizes under stringent conditions as defined herein, to one of the nucleotide sequences indicated in Table I, columns 5 or 7, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp., preferably of Table I B, columns 5 or 7, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp.or a portion thereof and encodes a protein having above-mentioned activity and as indicated in indicated in Table II.

**[00149.1.4.4]** Optionally, the nucleotide sequence, which hybridises to one of the nucleotide sequences indicated in Table I, columns 5 or 7, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp., preferably of Table I B, columns 5 or 7, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp. has further one or more of the activities annotated or known for the a protein as indicated in Table II, column 3, lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp..

**[0150.0.4.4]** Moreover, the nucleic acid molecule of the invention can comprise only a portion of the coding region of one of the sequences indicated in Table I, columns 5 or 7, preferably table I B, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp. for example a fragment which can be used as a probe or primer or a fragment encoding a biologically active portion of the polypeptide of the present invention or of a polypeptide used in the process of the present invention, i.e. having above-mentioned activity, e.g.conferring an increase of arginine and/or glutamate and/or proline and/or glutamine, resp., if its activity is increased. The nucleotide sequences determined from the cloning of the present protein-according-to-the-invention-encoding gene allows for the generation of probes and primers designed for use in identifying and/or cloning its homologues in other cell types and organisms. The probe/primer typically comprises substantially purified oligonucleotide. The oligonucleotide typically comprises a region of nucleotide sequence that hybridizes under stringent conditions to at least about 12, 15 preferably about 20 or 25, more preferably about 40, 50 or 75 consecutive nucleotides of a sense strand of one of the sequences set forth, e.g., as indicated in Table I, columns 5 or 7, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp. an anti-sense sequence of one of the sequences, e.g., as indicated in Table I, columns 5 or 7, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp. or naturally occurring mutants thereof. Primers based on a nucleotide of invention can be used in PCR reactions to clone homologues of the polypeptide of the invention or of the polypeptide used in the process of the invention, e.g. as the primers described in the examples of the present invention, e.g. as shown in the examples. A PCR with the primer pairs indicated in Table III, column 7, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp., will result in a fragment of a polynucleotide sequence as indicated in Table I, columns 5 or 7 lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp., or its gene product

**[0151.0.0.4]: see [0151.0.0.0]**

**[0152.0.4.4]** The nucleic acid molecule of the invention encodes a polypeptide or portion thereof which includes an amino acid sequence which is sufficiently homologous to an amino acid sequence as indicated in Table II, columns 5 or 7, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp., such that the protein or portion thereof maintains the ability to participate in the respective fine chemical production, in particular an activity increasing the level of arginine and/or glutamate and/or proline and/or glutamine, resp., as mentioned above or as described in the examples in plants or microorganisms is comprised.

**[0153.0.4.4]** As used herein, the language "sufficiently homologous" refers to proteins or portions thereof which have amino acid sequences which include a minimum number of identical or equivalent amino acid residues (e.g., an amino acid residue which has a similar side chain as an amino acid residue in one of the sequences of the polypeptide of the present invention) to an amino acid sequence as indicated in Table II, columns 5 or 7, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp., such that the protein or portion thereof is able to participate in the increase of the respective fine chemical production. In one embodiment, a protein or portion thereof as indicated in Table II, columns 5 or 7, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp., has for example an activity of a polypeptide as indicaded in Table II, column 3, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp.

**[0154.0.4.4]** In one embodiment, the nucleic acid molecule of the present invention comprises a nucleic acid that encodes a portion of the protein of the present invention. The protein is at least about 30%, 35%, 40%, 45% or 50%, preferably at least about 55%, 60%, 65% or 70%, and more preferably at least about 75%, 80%, 85%, 90%, 91%, 92%, 93% or 94% and most preferably at least about 95%, 97%, 98%, 99% or more homologous to an entire amino acid sequence as indicated in Table II, columns 5 or 7, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp., and has above-mentioned activity, e.g.conferring preferably the increase of the respective fine chemical.

**[0155.0.0.4]** and **[0156.0.0.4]: see [0155.0.0.0]** and **[0156.0.0.0]**

**[0157.0.4.4]** The invention further relates to nucleic acid molecules that differ from one of the nucleotide sequences indicated in Table I, columns 5 or 7, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp. (and portions thereof) due to degeneracy of the genetic code and thus encode a polypeptide of the present invention, in particular a polypeptide having above mentioned activity, e.g. conferring an increase in the respective fine chemical in a organism, e.g. as that polypeptides comprising the consensus sequences as indicated in Table IV, column 7 lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp.or of the polypeptide as indicated in Table II, columns 5 or 7, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp.or their functional homologues. Advantageously, the nucleic acid molecule of the invention or the nucleic acid molecule used in the method of the invention comprises, or in an other embodiment has, a nucleotide sequence encoding a protein comprising, or in an other embodiment having, a consensus sequences as indicated in Table IV, column 7, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp. or of the polypeptide as indicated in Table II, columns 5 or 7, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp. or the functional homologues. In a still further embodiment, the nucleic acid molecule of the invention or the nucleic acid molecule used in the method of the invention encodes a full length protein which is substantially homologous to an amino acid sequence comprising a consensus sequence as indicated in Table IV, column 7, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp. or of a polypeptide as indicated in Table II, columns 5 or 7, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp. or the functional homologues thereof. However, in a preferred embodiment, the nucleic acid molecule of the present invention does not consist of a sequence as indicated in Table I, columns 5 or 7 lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp., preferably as indicated in Table I A, columns 5 or 7, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp.. Preferably the nucleic acid molecule of the invention is a functional homologue or identical to a nucleic acid molecule indicated in Table I B, columns 5 or 7, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp..

**[0158.0.0.4]** to **[0160.0.0.4]: see [0158.0.0.0]** to **[0160.0.0.0]**

**[0161.0.4.4]** Accordingly, in another embodiment, a nucleic acid molecule of the invention is at least 15, 20, 25 or 30 nucleotides in length. Preferably, it hybridizes under stringent conditions to a nucleic acid molecule comprising a nucleotide sequence of the nucleic acid molecule of the present invention or used in the process of the present invention, e.g. comprising a sequence as indicated in Table I, columns 5 or 7, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp.The nucleic acid molecule is preferably at least 20, 30, 50, 100, 250 or more nucleotides in length.

**[0162.0.0.4]: see [0162.0.0.0]**

**[0163.0.4.4]** Preferably, nucleic acid molecule of the invention that hybridizes under stringent conditions to a sequence as indicated in Table I, columns 5 or 7, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp., corresponds to a naturally-occurring nucleic acid molecule of the invention. As used herein, a "naturally-occurring" nucleic acid molecule refers to an RNA or DNA molecule having a nucleotide sequence that occurs in nature (e.g., encodes a natural protein). Preferably, the nucleic acid molecule encodes a natural protein having above-mentioned activity, e.g. conferring the respective fine chemical increase after increasing the expression or activity thereof or the activity of a protein of the invention or used in the process of the invention.

**[0164.0.0.4]: see [0164.0.0.0]**

**[0165.0.4.4]** For example, nucleotide substitutions leading to amino acid substitutions at "non-essential" amino acid residues can be made in a sequence of the nucleic acid molecule of the invention or used in the process of the invention, e.g. as indicated in Table I, columns 5 or 7, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp..

**[0166.0.0.4] and [0167.0.0.4]: see [0166.0.0.0] and [0167.0.0.0]**

**[0431.0.0.0] [0168.0.4.4]** Accordingly, the invention relates to nucleic acid molecules encoding a polypeptide having above-mentioned activity, e.g. conferring an increase in the the respective fine chemical in an organisms or parts thereof that contain changes in amino acid residues that are not essential for said activity. Such polypeptides differ in amino acid sequence from a sequence contained in a sequence as indicated in Table II, columns 5 or 7, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp., preferably of Table II B, column 7, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp. yet retain said activity described herein. The nucleic acid molecule can comprise a nucleotide sequence encoding a polypeptide, wherein the polypeptide comprises an amino acid sequence at least about 50% identical to an amino acid sequence as indicated in Table II, columns 5 or 7, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp., preferably of Table II B, column 7, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp.and is capable of participation in the increase of production of the respective fine chemical after increasing its activity, e.g. its expression. Preferably, the protein encoded by the nucleic acid molecule is at least about 60% identical to a sequence as indicated in Table II, columns 5 or 7, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp., preferably of Table II B, column 7, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp., more preferably at least about 70% identical to one of the sequences as indicated in Table II, columns 5 or 7, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54. to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp., preferably of Table II B, column 7, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp., even more preferably at least about 80%, 90%, or 95% homologous to a sequence as indicated in Table II, columns 5 or 7, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp., preferably of Table II B, column 7, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp., and most preferably at least about 96%, 97%, 98%, or 99% identical to the sequence as indicated in Table II, columns 5 or 7, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp., preferably of Table II B, column 7, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp..

**[0169.0.0.4] to [0172.0.0.4]: see [0169.0.0.0] to [0172.0.0.0]**

**[0173.0.4.4]** For example a sequence which has a 80% homology with sequence SEQ ID No 1982 at the nucleic acid level is understood as meaning a sequence which, upon comparison with the sequence SEQ ID No 1982 by the above Gap program algorithm with the above parameter set, has a 80% homology.

**[0174.0.0.4]: see [0174.0.0.0]**

**[0175.0.4.4]** For example a sequence which has a 80% homology with sequence SEQ ID No 1983 at the protein level is understood as meaning a sequence which, upon comparison with the sequence SEQ ID No 1983 by the above program algorithm with the above parameter set, has a 80% homology.

**[0176.0.4.4]** Functional equivalents derived from one of the polypeptides as indicated in Table II, columns 5 or 7, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp., according to the invention by substitution, insertion or deletion have at least 30%, 35%, 40%, 45% or 50%, preferably at least 55%, 60%, 65% or 70% by preference at least 80%, especially preferably at least 85% or 90%, 91%, 92%, 93% or 94%, very especially preferably at least 95%, 97%, 98% or 99% homology with one of the polypeptides as indicated in Table II, columns 5 or 7, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp., according to the invention and are distinguished by essentially the same properties as a polypeptide as indicated in Table II, columns 5 or 7, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp..

**[0177.0.4.4]** Functional equivalents derived from a nucleic acid sequence as indicated in Table I, columns 5 or 7, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp., preferably of Table I B, column 7, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp. according to the invention by substitution, insertion or deletion have at least 30%, 35%, 40%, 45% or 50%, preferably at least 55%, 60%, 65% or 70% by preference at least 80%, especially preferably at least 85% or 90%, 91%, 92%, 93% or 94%, very especially preferably at least 95%, 97%, 98% or 99% homology with one of a polypeptide as indicated in Table II, columns 5 or 7 lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp. according to the invention and encode polypeptides having essentially the same properties as a polypeptide as indicated in Table II, columns 5 or 7, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp., preferably of Table I B, column 7, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp..

**[0178.0.0.4]: see [0178.0.0.0]**

**[0179.0.4.4]** A nucleic acid molecule encoding an homologous to a protein sequence as indicated in Table II, columns 5 or 7, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp., preferably of Table II B, column 7 lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp. can be created by introducing one or more nucleotide substitutions, additions or deletions into a nucleotide sequence of the nucleic acid molecule of the present invention, in particular as indicated in Table I, columns 5 or 7, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp., such that one or more amino acid substitutions, additions or deletions are introduced into the encoded protein. Mutations can be introduced into the encoding sequences of a sequences as indicated in Table I, columns 5 or 7, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp., by standard techniques, such as site-directed mutagenesis and PCR-mediated mutagenesis.

**[0180.0.0.4]** to **[0183.0.0.4]: see [0180.0.0.0]** to **[0183.0.0.0]**

**[0184.0.4.4]** Homologues of the nucleic acid sequences used, with a sequence as indicated in Table I, preferably table I B, columns 5 or 7, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp., or of the nucleic acid sequences derived from a sequences as indicated in Table II, columns 5 or 7, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp. comprise also allelic variants with at least approximately 30%, 35%, 40% or 45% homology, by preference at least approximately 50%, 60% or 70%, more preferably at least approximately 90%, 91 %, 92%, 93%, 94% or 95% and even more preferably at least approximately 96%, 97%, 98%, 99% or more homology with one of the nucleotide sequences shown or the abovementioned derived nucleic acid sequences or their homologues, derivatives or analogues or parts of these. Allelic variants encompass in particular functional variants which can be obtained by deletion, insertion or substitution of nucleotides from the sequences shown, preferably from a sequence as indicated in Table I, columns 5 or 7, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp., or from the derived nucleic acid sequences, the intention being, however, that the enzyme activity or the biological activity of the resulting proteins synthesized is advantageously retained or increased.

**[0185.0.4.4]** In one embodiment of the present invention, the nucleic acid molecule of the invention or used in the process of the invention comprises one or more sequences as indicated in Table I, preferably table I B, columns 5 or 7, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp.. In one embodiment, it is preferred that the nucleic acid molecule comprises as little as possible other nucleotides not shown in any one of sequences as indicated in Table I, preferably table I B, columns 5 or 7, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp.. In one embodiment, the nucleic acid molecule comprises less than 500, 400, 300, 200, 100, 90, 80, 70, 60, 50 or 40 further nucleotides. In a further embodiment, the nucleic acid molecule comprises less than 30, 20 or 10 further nucleotides. In one embodiment, a nucleic acid molecule used in the process of the invention is identical to a sequences as indicated in Table I, columns 5 or 7, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp.

**[0186.0.4.4]** Also preferred is that one or more nucleic acid molecule(s) used in the process of the invention encodes a polypeptide comprising a sequence as indicated in Table II, preferably table II B, columns 5 or 7, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp.. In one embodiment, the nucleic acid molecule encodes less than 150, 130, 100, 80, 60, 50, 40 or 30 further amino acids. In a further embodiment, the encoded polypeptide comprises less than 20, 15, 10, 9, 8, 7, 6 or 5 further amino acids. In one embodiment, the encoded polypeptide used in the process of the invention is identical to the sequences as indicated in Table II, preferably table II B, columns 5 or 7, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp..

**[0187.0.4.4]** In one embodiment, a nucleic acid molecule of the invention or used in the process encodes a polypeptide comprising the sequence as indicated in Table II, columns 5 or 7, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp., comprises less than 100 further nucleotides. In a further embodiment, said nucleic acid molecule comprises less than 30 further nucleotides. In one embodiment, the nucleic acid molecule used in the process is identical to a coding sequence as indicated in Table II, preferably table II B, columns 5 or 7, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp..
Idealerweise würde man in diesen Abschnitten nur column 7 von Table II bevorzugen, wurde auch teilweise so gemacht, andererseits erheblicher Aufwand und ggf. nicht unbedingt notwendig??

**[0188.0.4.4]** Polypeptides (= proteins), which still have the essential biological or enzymatic activity of the polypeptide of the present invention conferring an increase of the respective fine chemical i.e. whose activity is essentially not reduced, are polypeptides with at least 10% or 20%, by preference 30% or 40%, especially preferably 50% or 60%, very especially preferably 80% or 90 or more of the wild type biological activity or enzyme activity, advantageously, the activity is essentially not reduced in comparison with the activity of a polypeptide as indicated in Table II, columns 5 or 7, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp.,and is expressed under identical conditions.
In one embodiment, the polypeptide of the invention is a homolog consisting of or comprising the sequence as indicated in Table II B, columns 7, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp..

**[0189.0.4.4]** Homologues of a sequences as indicated in Table I, columns 5 or 7, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp., , or of a derived sequences as indicated in Table II, columns 5 or 7 lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp.,., also mean truncated sequences, cDNA, single-stranded DNA or RNA of the coding and noncoding DNA sequence. Homologues of said sequences are also understood as meaning derivatives, which comprise noncoding regions such as, for example, UTRs, terminators, enhancers or promoter variants. The promoters upstream of the nucleotide sequences stated can be modified by one or more nucleotide substitution(s), insertion(s) and/or deletion(s) without, however, interfering with the functionality or activity either of the promoters, the open reading frame (= ORF) or with the 3'-regulatory region such as terminators or other 3'regulatory regions, which are far away from the ORF. It is furthermore possible that the activity of the promoters is increased by modification of their sequence, or that they are replaced completely by more active promoters, even promoters from heterologous organisms. Appropriate promoters are known to the person skilled in the art and are mentioned herein below.

**[0190.0.0.4] to [0203.0.0.4]: see [0190.0.0.0] to [0203.0.0.0]**

**[0204.0.4.4]** Accordingly, in one embodiment, the invention relates to a nucleic acid molecule which comprises a nucleic acid molecule selected from the group consisting of:
(haben die 435 automatisch ergänzt, daher ggf. Hier etwas unübersichtlich)
a) nucleic acid molecule encoding, preferably at least the mature form, of a polypeptide as indicated in Table II, preferably table II B, columns 5 or 7, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp.; or a fragment thereof conferring an increase in the amount of the respective fine chemical, in particular, of arginine (lines 30 to 37, 390, 405 and/or 430) and/or glutamate (lines 38 to 43, 386, 387, 391, 396, 399 to 401, 403, 406, 413, 414, 417, 418, 421, 424, 427, 434 and/or 435) and/or proline (lines 44 to 56, 388, 389, 398, 411, 412, 425 and/or 429) and/or glutamine (lines 57 to 62, 392 to 395, 397, 402, 404, 407 to 410, 415, 416, 419, 420, 422, 423, 426, 428 and/or 431 to 433), resp., in an organism or a part thereof
b) nucleic acid molecule comprising, preferably at least the mature form, of a nucleic acid molecule as indicated in Table I, columns 5 or 7, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp., or a fragment thereof conferring an increase in the amount of the respective fine chemical in an organism or a part thereof;
c) nucleic acid molecule whose sequence can be deduced from a polypeptide sequence encoded by a nucleic acid molecule of (a) or (b) as result of the degeneracy of the genetic code and conferring an increase in the amount of the fine chemical in an organism or a part thereof;
d) nucleic acid molecule encoding a polypeptide whose sequence has at least 50% identity with the amino acid sequence of the polypeptide encoded by the nucleic acid molecule of (a) to (c) and conferring an increase in the amount of the fine chemical in an organism or a part thereof;
e) nucleic acid molecule which hybridizes with a nucleic acid molecule of (a) to (c) under under stringent hybridisation conditions and conferring an increase in the amount of the fine chemical in an organism or a part thereof;
f) nucleic acid molecule encoding a polypeptide, the polypeptide being derived by substituting, deleting and/or adding one or more amino acids of the amino acid sequence of the polypeptide encoded by the nucleic acid molecules (a) to (d), preferably to (a) to (c), and conferring an increase in the amount of the fine chemical in an organism or a part thereof;
g) nucleic acid molecule encoding a fragment or an epitope of a polypeptide which is encoded by one of the nucleic acid molecules of (a) to (e), preferably to (a) to (c) and conferring an increase in the amount of the fine chemical in an organism or a part thereof ;
h) nucleic acid molecule comprising a nucleic acid molecule which is obtained by amplifying a cDNA library or a genomic library using primers or primer pairs as indicated in Table III, column 7, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp., and conferring an increase in the amount of the respective fine chemical, in particular, of arginine (lines 30 to 37, 390, 405 and/or 430) and/or glutamate (lines 38 to 43, 386, 387, 391, 396, 399 to 401, 403, 406, 413, 414, 417, 418, 421, 424, 427, , 434 and/or 435/or proline (lines 44 to 56, 388, 389, 398, 411, 412, 425 and/or 429) and/or glutamine (lines 57 to 62, 392 to 395, 397, 402, 404, 407 to 410, 415, 416, 419, 420, 422, 423, 426, 428 and/or 431 to 433),resp. in an organism or a part thereof;
i) nucleic acid molecule encoding a polypeptide which is isolated, e.g. from a expression library, with the aid of monoclonal antibodies against a polypeptide encoded by one of the nucleic acid molecules of (a) to (g), preferably to (a) to (c) and conferring an increase in the amount of the fine chemical in an organism or a part thereof;
j) nucleic acid molecule which encodes a polypeptide comprising a consensus sequence as indicated in Table IV, columns 5 or 7, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp., and conferring an increase in the amount of the respective fine chemical, in particular, of arginine (lines 30 to 37, 390, 405 and/or 430) and/or glutamate (lines 38 to 43, 386, 387, 391, 396, 399 to 401, 403, 406, 413, 414, 417, 418, 421, 424, 427, 434 and/or, 435) and/or proline (lines 44 to 56, 388, 389, 398, 411, 412, 425 and/or 429) and/or glutamine (lines 57 to 62, 392 to 395, 397, 402, 404, 407 to 410, 415, 416, 419, 420, 422, 423, 426, 428 and/or 431 to 433),resp., in an organism or a part thereof;
k) nucleic acid molecule encoding the amino acid sequence of a polypeptide encoding a domaine of a polypeptide as indicated in Table II, columns 5 or 7, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp., and conferring an increase in the amount of the respective fine chemical, in particular, of arginine (lines 30 to 37, 390, 405 and/or 430) and/or glutamate (lines 38 to 43, 386, 387, 391, 396, 399 to 401, 403, 406, 413, 414, 417, 418, 421, 424, 427, 434 and/or 435) and/or proline (lines 44 to 56, 388, 389, 398, 411, 412, 425 and/or 429) and/or glutamine (lines 57 to 62, 392 to 395, 397, 402, 404, 407 to 410, 415, 416, 419, 420, 422, 423, 426, 428 and/or 431 to 433)" in an organism or a part thereof; and
l) nucleic acid molecule which is obtainable by screening a suitable nucleic acid library under stringent hybridization conditions with a probe comprising one of the sequences of the nucleic acid molecule of (a) to (k) or with a fragment of at least 15 nt, preferably 20 nt, 30 nt, 50 nt, 100 nt, 200 nt or 500 nt of the nucleic acid molecule characterized in (a) to (h) or of a nucleic acid molecule as indicated in Table I, columns 5 or 7, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, ' 426, 428 and/or 431 to 433 resp., or a nucleic acid molecule encoding, preferably at least the mature form of, a polypeptide as indicated in Table II, columns 5 or 7, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp. and conferring an increase in the amount of the respective fine chemical in an organism or a part thereof;
or which encompasses a sequence which is complementary thereto;
whereby, preferably, the nucleic acid molecule according to (a) to (I) distinguishes over the sequence indicated in Table IA, columns 5 or 7, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp., by one or more nucleotides. In one embodiment, the nucleic acid molecule does not consist of the sequence shown and indicated in Table I A or I B, columns 5 or 7, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp..: In one embodiment, the nucleic acid molecule is less than 100%, 99,999%, 99,99%, 99,9% or 99% identical to a sequence indicated in Table I A or I B, columns 5 or 7, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp.. In another embodiment, the nucleic acid molecule does not encode a polypeptide of a sequence indicated in Table II A or II B, columns 5 or 7, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp.. In an other embodiment, the nucleic acid molecule of the present invention is at least 30 %, 40 %, 50%, or 60% identical and less than 100%, 99,999%, 99,99%, 99,9% or 99% identical to a sequence indicated in Table I A or I B, columns 5 or 7, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp.. In a further embodiment the nucleic acid molecule does not encode a polypeptide sequence as indicated in Table II A or II B, columns 5 or 7, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp.. Accordingly, in one embodiment, the nucleic acid molecule of the differs at least in one or more residues from a nucleic acid molecule indicated in Table I A or I B, columns 5 or 7, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp.. Accordingly, in one embodiment, the nucleic acid molecule of the present invention encodes a polypeptide, which differs at least in one or more amino acids from a polypeptide indicated in Table II A or I B, columns 5 or 7, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp.. In another embodiment, a nucleic acid molecule indicated in Table I A or I B, columns 5 or 7, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp.does not encode a protein of a sequence indicated in Table II A or II B, columns 5 or 7, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp.. Accordingly, in one embodiment, the protein encoded by a sequences of a nucleic acid accoriding to (a) to (I) does not consist of a sequence as indicated in Table II A or II B, columns 5 or 7, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp.. In a further embodiment, the protein of the present invention is at least 30 %, 40 %, 50%, or 60% identical to a protein sequence indicated in Table II A or II B, columns 5 or 7, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp. and less than 100%, preferably less than 99,999%, 99,99% or 99,9%, more preferably less than 99%, 985, 97%, 96% or 95% identical to a sequence as indicated in Table I A or II B, columns 5 or 7, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp..

**[0205.0.0.4] to [0226.0.0.4]: see [0205.0.0.0] to [0226.0.0.0]**

**[0227.0.4.4]** The abovementioned nucleic acid molecules can be cloned into the nucleic acid constructs or vectors according to the invention in combination together with further genes, or else different genes are introduced by transforming several nucleic acid constructs or vectors (including plasmids) into a host cell, advantageously into a plant cell or a microorgansims.
In addition to a sequence indicated in Table I, columns 5 or 7, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp., or its derivatives, it is advantageous additionally to express and/or mutate further genes in the organisms. Especially advantageously, additionally at least one further gene of the amino acid biosynthetic pathway such as for L-lysine, L-threonine and/or L-methionine and/or L-leucine and/or isoleucine and/or valine is expressed in the organisms such as plants or microorganisms. It is also possible that the regulation of the natural genes has been modified advantageously so that the gene and/or its gene product is no longer subject to the regulatory mechanisms which exist in the organisms. This leads to an increased synthesis of the amino acids desired since, for example, feedback regulations no longer exist to the same extent or not at all. In addition it might be advantageously to combine one or more of the sequences indicated in Table I, columns 5 or 7, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp., with genes which generally support or enhances to growth or yield of the target organismen, for example genes which lead to faster growth rate of microorganisms or genes which produces stress-, pathogen, or herbicide resistant plants.

**[0228.0.0.4] to [0230.0.0.4]: see[0228.0.0.0] to [0230.0.0.0]**

**[0231.0.4.4]** In a further advantageous embodiment of the process of the invention, the organisms used in the process are those in which simultaneously an arginine and/or glutamate and/or glutamine and/or proline degrading protein is attenuated, in particular by reducing the rate of expression of the corresponding gene. **[0232.0.0.4] to [0282.0.0.4]: see [0232.0.0.0] to [0282.0.0.0]**

**[0283.0.4.4]** Moreover, a native polypeptide conferring the increase of the respective fine chemical in an organism or part thereof can be isolated from cells (e.g., endothelial cells), for example using the antibody of the present invention as described below, e.g. an antibody against a protein as indicated in Table II, column 3, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp., or an antibody against a polypeptide as indicated in Table II, columns 5 or 7, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp. which can be produced by standard techniques utilizing the polypeptid of the present invention or fragment thereof, i.e., the polypeptide of this invention. Preferred are monoclonal antibodies.

**[0284.0.0.4]: see [0284.0.0.0].**

**[0285.0.4.4]** In one embodiment, the present invention relates to a polypeptide having a sequence as indicated in Table II, columns 5 or 7, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419,
420, 422, 423, 426, 428 and/or 431 to 433 resp., or as encoded by a nucleic acid molecule as indicated in Table I, columns 5 or 7, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp., or functional homologues thereof.

**[0286.0.4.4]** In one advantageous embodiment, in the method of the present invention the activity of a polypeptide is increased which comprises or consists of a consensus sequence as indicated in Table IV, column 7, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp. In another embodiment, the present invention relates to a polypeptide comprising or consisting of a consensus sequence as indicated in Table IV, column 7, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp., whereby 20 or less, preferably 15 or 10, preferably 9, 8, 7, or 6, more preferred 5 or 4, even more preferred 3, even more preferred 2, even more preferred 1, most preferred 0 of the amino acids positions indicated can be replaced by any amino acid or, in an further embodiment, can be replaced and/or absent. In one embodiment, the present invention relates to the method of the present invention comprising a polypeptide or to a polypeptide comprising more than one consensus sequences (of an individual line) as indicated in Table IV, column 7, lines lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp

**[0287.0.0.4] to [0290.0.0.4]: see [0287.0.0.0] to [0290.0.0.0]**

**[0291.0.4.4]** In one advantageous embodiment, the method of the present invention comprises the increasing of a polypeptide comprising or consisting of plant or microorganism specific consensus sequences.
In one embodiment, said polypeptide of the invention distinguishes over a sequence as indicated in Table II A or IIB, columns 5 or 7, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp. by one or more amino acids. In one embodiment, polypeptide distinguishes form a sequence as indicated in Table II A or IIB, columns 5 or 7, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp. by more than 1, 2, 3, 4, 5, 6, 7, 8 or 9 amino acids, preferably by more than 10, 15, 20, 25 or 30 amino acids, evenmore preferred are more than 40, 50, or 60 amino acids and, preferably, the sequence of the polypeptide of the invention distinguishes from a sequence as indicated in Table II A or II B, columns 5 or 7, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp.by not more than 80% or 70% of the amino acids, preferably not more than 60% or 50%, more preferred not more than 40% or 30%, even more preferred not more than 20% or 10%. In an other embodiment, said polypeptide of the invention does not consist of a sequence as indicated in Table II A or II B, columns 5 or 7, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp..

**[0292.0.0.4]: see [0292.0.0.0]**

**[0293.0.4.4]** In one embodiment, the invention relates to polypeptide conferring an increase in the respective fine chemical in an organism or part thereof and being encoded by the nucleic acid molecule of the invention or a nucleic acid molecule used in the process of the invention. In one embodiment, the polypeptide of the invention has a sequence which distinguishes from a sequence as indicated in Table II A or II B, columns 5 or 7, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp.by one or more amino acids. In an other embodiment, said polypeptide of the invention does not consist of the sequence as indicated in Table II A or II B, columns 5 or 7, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp.. In a further embodiment, said polypeptide of the present invention is less than 100%, 99,999%, 99,99%, 99,9% or 99% identical. In one embodiment, said polypeptide does not consist of the sequence encoded by a nucleic acid molecules as indicated in Table I A or IB, columns 5 or 7, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp..

**[0294.0.4.4]** In one embodiment, the present invention relates to a polypeptide having an activity of a protein as indicated in Table II, column 3, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp., which distinguishes over a sequence as indicated in Table IIA or table II B, columns 5 or 7, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp., by one or more amino acids, preferably by more than 5, 6, 7, 8 or 9 amino acids, preferably by more than 10, 15, 20, 25 or 30 amino acids, evenmore preferred are more than 40, 50, or 60 amino acids but even more preferred by less than 70% of the amino acids, more preferred by less than 50%, even more preferred my less than 30% or 25%, more preferred are 20% or 15%, even more preferred are less than 10%.

**[0295.0.0.4] and [0297.0.0.4]: see [0295.0.0.0]and [0297.0.0.0]**

**[0297.1.4.4]** Non polypeptide of the invention-chemicals are e.g. polypeptides having not the activity of a polypeptide indicated in Table II, columns 3, 5 or 7, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp..

**[0298.0.4.4]** A polypeptide of the invention can participate in the process of the present invention. The polypeptide or a portion thereof comprises preferably an amino acid sequence which is sufficiently homologous to an amino acid sequence as indicated in Table II, columns 5 or 7, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp.. The portion of the protein is preferably a biologically active portion as described herein. Preferably, the polypeptide used in the process of the invention has an amino acid sequence identical to a sequence as indicated in Table II, columns 5 or 7, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp..

**[0299.0.4.4]** Further, the polypeptide can have an amino acid sequence which is encoded by a nucleotide sequence which hybridizes, preferably hybridizes under stringent conditions as described above, to a nucleotide sequence of the nucleic acid molecule of the present invention. Accordingly, the polypeptide has an amino acid sequence which is encoded by a nucleotide sequence that is at least about 35%, 40%, 45%, 50%, 55%, 60%, 65% or 70%, preferably at least about 75%, 80%, 85% or 90, and more preferably at least about 91%, 92%, 93%, 94% or 95%, and even more preferably at least about 96%, 97%, 98%, 99% or more homologous to one of the nucleotide sequence as indicated in Table I, columns 5 or 7, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp.. The preferred polypeptide of the present invention preferably possesses at least one of the activities according to the invention and described herein. A preferred polypeptide of the present invention includes an amino acid sequence encoded by a nucleotide sequence which hybridizes, preferably hybridizes under stringent conditions, to a nucleotide sequence as indicated in Table I, columns 5 or 7, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp., or which is homologous thereto, as defined above.

**[0300.0.4.4]** Accordingly the polypeptide of the present invention can vary from a sequence as indicated in Table II, columns 5 or 7, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp., in amino acid sequence due to natural variation or mutagenesis, as described in detail herein. Accordingly, the polypeptide comprise an amino acid sequence which is at least about 35%, 40%, 45%, 50%, 55%, 60%, 65% or 70%, preferably at least about 75%, 80%, 85% or 90, and more preferably at least about 91%, 92%, 93%, 94% or 95%, and most preferably at least about 96%, 97%, 98%, 99% or more homologous to an entire amino acid sequence of a sequence as indicated in Table II A or II B, columns 5 or 7, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp..

**[0301.0.0.4]: see [0301.0.0.0]**

**[0302.0.4.4]** Biologically active portions of an polypeptide of the present invention include peptides comprising amino acid sequences derived from the amino acid sequence of the polypeptide of the present invention or used in the process of the present invention, e.g., an amino acid sequence as indicated in Table II, columns 5 or 7, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp., or the amino acid sequence of a protein homologous thereto, which include fewer amino acids than a full length polypeptide of the present invention or used in the process of the present invention or the full length protein which is homologous to an polypeptide of the present invention or used in the process of the present invention depicted herein, and exhibit at least one activity of polypeptide of the present invention or used in the process of the present invention.

**[0303.0.0.4]: see [0303.0.0.0]**

**[0304.0.4.4]** Manipulation of the nucleic acid molecule of the invention may result in the production of a protein having essentially the activity of the polypeptides as indicated in Table II, column 3, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp., but having differences in the sequence from said wild-type protein. These proteins may be improved in efficiency or activity, may be present in greater numbers in the cell than is usual, or may be decreased in efficiency or activity in relation to the wild type protein.

**[0305.0.0.4]** to **[0308.0.0.4]: see [0306.0.0.0]** to **[0308.0.0.0]**

**[0309.0.4.4]** In one embodiment, a reference to a protein (= polypeptide) of the invention or as indicated in Table II, columns 5 or 7, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp., refers to a polypeptide having an amino acid sequence corresponding to the polypeptide of the invention or used in the process of the invention , whereas a " non-polypeptide of the invention" or "other polypeptide" not being indicated in Table II, columns 5 or 7, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp., refers to a polypeptide having an amino acid sequence corresponding to a protein which is not substantially homologous a polypeptide of the invention, preferably which is not substantially homologous to a polypeptide as indicated in Table II, columns 5 or 7, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp., e.g., a protein which does not confer the activity described herein or annotated or known for as indicated in Table II, column 3, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp., and which is derived from the same or a different organism. In one embodiment, a "non-polypeptide of the invention" or "other polypeptide" not being indicated in Table II, columns 5 or 7, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp., does not confer an increase of the respective fine chemical in an organism or part therof.

**[0310.0.0.4] to [0334.0.0.4]: see [0310.0.0.0] to [0334.0.0.0]**

**[0335.0.4.4]** The dsRNAi method has proved to be particularly effective and advantageous for reducing the expression of a nucleic acid sequences as indicated in Table I, columns 5 or 7, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp., and/or homologs thereof. As described inter alia in WO 99/32619, dsRNAi approaches are clearly superior to traditional antisense approaches. The invention therefore furthermore relates to double-stranded RNA molecules (dsRNA molecules) which, when introduced into an organism, advantageously into a plant (or a cell, tissue, organ or seed derived therefrom), bring about altered metabolic activity by the reduction in the expression of a nucleic acid sequences as indicated in Table I, columns 5 or 7, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp., and/or homologs thereof. In a double-stranded RNA molecule for reducing the expression of an protein encoded by a nucleic acid sequence sequences as indicated in Table I, columns 5 or 7, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp., and/or homologs thereof, one of the two RNA strands is essentially identical to at least part of a nucleic acid sequence, and the respective other RNA strand is essentially identical to at least part of the complementary strand of a nucleic acid sequence.

**[0336.0.0.4] to [0342.0.0.4]: see [0336.0.0.0] to [0342.0.0.0]**

**[0343.0.4.4]** As has already been described, 100 % sequence identity between the dsRNA and a gene transcript of a nucleic acid sequence as indicated in Table I, columns 5 or 7, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp., or it's homolog is not necessarily required in order to bring about effective reduction in the expression. The advantage is, accordingly, that the method is tolerant with regard to sequence deviations as may be present as a consequence of genetic mutations, polymorphisms or evolutionary divergences. Thus, for example, using the dsRNA, which has been generated starting from a sequence as indicated in Table I, columns 5 or 7, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp., or homologs thereof of the one organism, may be used to suppress the corresponding expression in another organism.

**[0344.0.0.4] to [0361.0.0.4]: see [0344.0.0.0] to [0361.0.0.0]**

**[0362.0.4.4]** Accordingly the present invention relates to any cell transgenic for any nucleic acid characterized as part of the invention, e.g. conferring the increase of the respective fine chemical in a cell or an organism or a part thereof, e.g. the nucleic acid molecule of the invention, the nucleic acid construct of the invention, the antisense molecule of the invention, the vector of the invention or a nucleic acid molecule encoding the polypeptide of the invention, e.g. the polypeptide as indicated in Table II, columns 5 or 7, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp.. e.g. encoding a polypeptide having protein activity, as indicated in Table II, columns 3, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp.. Due to the above mentioned activity the respective fine chemical content in a cell or an organism is increased. For example, due to modulation or manipulation, the cellular activity of the polypeptide of the invention or nucleic acid molecule of the invention is increased, e.g. due to an increased expression or specific activity of the subject matters of the invention in a cell or an organism or a part thereof. Transgenic for a polypeptide having an activity of a polypeptide as indicated in Table II, columns 5 or 7, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp., means herein that due to modulation or manipulation of the genome, an activity as annotated for a polypeptide as indicated in Table II, column 3, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp. e.g. having a sequence as indicated in Table II, columns 5 or 7, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp., is increased in a cell or an organism or a part thereof. Examples are described above in context with the process of the invention

**[0363.0.0.4]: to [0382.0.0.4]: see [0363.0.0.0] to [0382.0.0.0]**

**[0383.0.4.4]** For preparing arginine and/or glutamate and/or glutamine and/or proline compound-containing fine chemicals, in particular the fine chemical, it is possible to use as arginine and/or glutamate and/or glutamine and/or proline amino acid source organic compounds such as, for example, citrulline, argininosuccinate, ornithine, aspartate, 2-Oxoglutarate, glutamyl, glutamic-semialdehyde, Pyrroline-5-carboxylate, Glutamine or else organic arginine and/or glutamate and/or glutamine and/or proline acid precursor compounds.

**[0384.0.0.4]: see [0384.0.0.0]**

**[0385.0.4.4]** The fermentation broths obtained in this way, containing in particular L-arginine and/or L-glutamate and/or L-proline and/or L-tryptophane, L-methionine, L-threonine and/or L-lysine, normally have a dry matter content of from 7.5 to 25% by weight. Sugar-limited fermentation is additionally advantageous, at least at the end, but especially over at least 30% of the fermentation time. This means that the concentration of utilizable sugar in the fermentation medium is kept at, or reduced to, 0 to 3 g/l during this time. The fermentation broth is then processed further. Depending on requirements, the biomass can be removed entirely or partly by separation methods, such as, for example, centrifugation, filtration, decantation or a combination of these methods, from the fermentation broth or left completely in it. The fermentation broth can then be thickened or concentrated by known methods, such as, for example, with the aid of a rotary evaporator, thin-film evaporator, falling film evaporator, by reverse osmosis or by nanofiltration. This concentrated fermentation broth can then be worked up by freeze-drying, spray drying, spray granulation or by other processes.

**[0386.0.0.4] to [0392.0.0.4]: see [0386.0.0.0] to [0392.0.0.0]**

**[0393.0.4.4]** In one embodiment, the present invention relates to a method for the identification of a gene product conferring an increase in the fine chemical production in a cell, comprising the following steps:
d) contacting,̅ e.g. hybridising, the nucleic acid molecules of a sample, e.g. cells, tissues, plants or microorganisms or a nucleic acid library , which can contain a candidate gene encoding a gene product conferring an increase in the fine chemical after expression, with the nucleic acid molecule of the present invention;
e) identifying the nucleic acid molecules, which hybridize under relaxed stringent conditions with the nucleic acid molecule of the present invention in particular to the nucleic acid molecule sequence as indicated in Table I, preferably Table I B, columns 5 or 7, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp., and, optionally, isolating the full length cDNA clone or complete genomic clone;
f) introducing the candidate nucleic acid molecules in host cells, preferably in a plant cell or a microorganism, appropriate for producing the fine chemical;
g) expressing the identified nucleic acid molecules in the host cells;
h) assaying the the fine chemical level in the host cells; and
i) identifying the nucleic acid molecule and its gene product which expression confers an increase in the the fine chemical level in the host cell after expression compared to the wild type.

**[0394.0.0.4]** to **[0399.0.0.4]: see [0394.0.0.0] to [0399.0.0.0]**

**[00399.1.4.4]:** One can think to screen for increased production of the respective fine chemical by for example searching for a resistance to a drug blocking the synthesis of the respective fine chemical and looking whether this effect is dependent on the activity or expression of a polypeptide as indicated in Table II, columns 5 or 7, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp., or a homolog thereof, e.g. comparing the phenotyp of nearly identical organisms with low and high acitivity of a protein as indicated in Table II, columns 5 or 7, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp., after incubation with the drug.

**[0400.0.0.4] to [0423.0.0.4]: see [0400.0.0.0] to [0423.0.0.0]**

**[0424.0.0.4]: see [0424.0.0.2]**

**[0425.0.0.4] to [0460.0.0.4]: see [0425.0.0.0] to [0460.0.0.0]**

**[0461.0.4.4]** Example 10: Cloning SEQ ID NO: 1982 for the expression in plants

**[0462.0.0.4]: see [0462.0.0.0]**

**[0463.0.4.4]** SEQ ID NO: 1982 is amplified by PCR as described in the protocol of the Pfu Turbo or DNA Herculase polymerase (Stratagene).

**[0464.0.0.4] to [0466.0.0.4]: see [0464.0.0.0] to [0466.0.0.0]**

**[0467.0.4.4]** The following primer sequences were selected for the gene SEQ ID No: 1982:
i) forward primer (SEQ ID No: 2046)
   atgaataacg aacccttacg tccc
ii) reverse primer (SEQ ID No: 2047)
   ttacatatcc tcatgaaatt cttcaagt

**[0468.0.0.4] to [0479.0.0.4]: see [0468.0.0.0] to [0479.0.0.0]**

**[0480.0.4.4]** Example 11: Generation of transgenic plants which express SEQ ID No: 1982

**[0481.0.0.4] to [0513.0.0.4]: see [0481.0.0.0] to [0513.0.0.0]**

**[0514.0.4.4]** As an alternative, the amino acids can be detected advantageously via HPLC separation in ethanolic extract as described by Geigenberger et al. (Plant Cell & Environ, 19, 1996: 43-55).
The results of the different plant analyses can be seen from the table which follows:

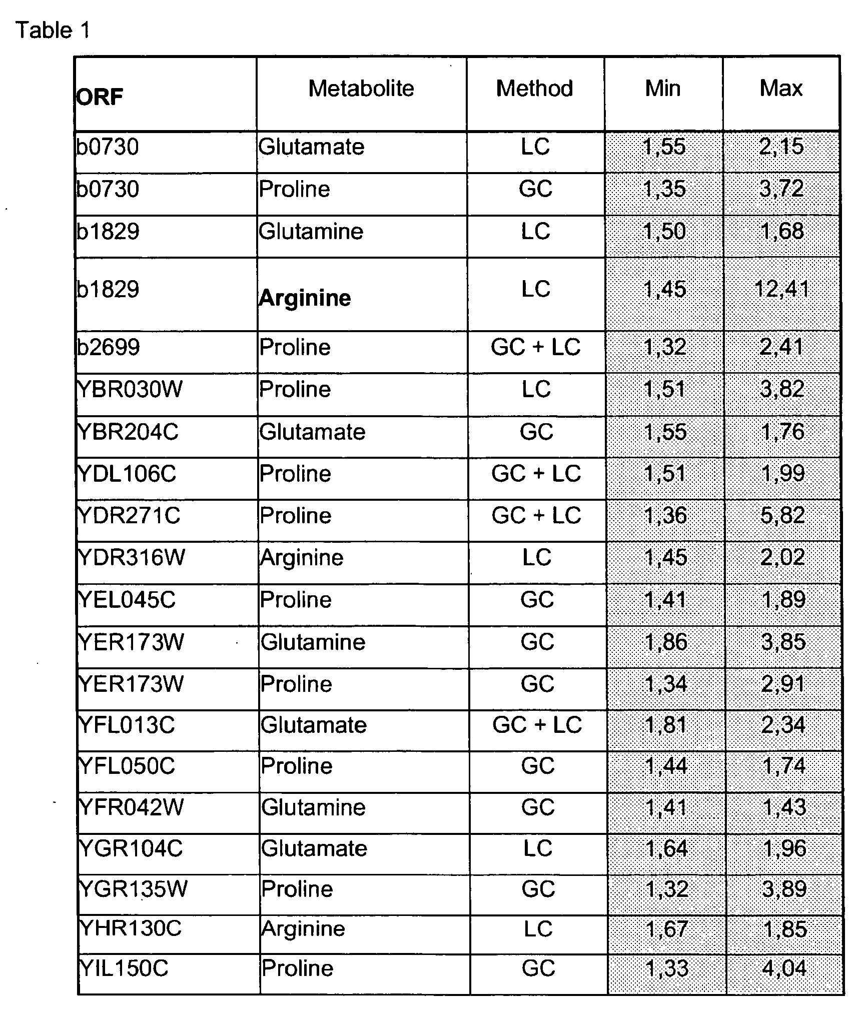

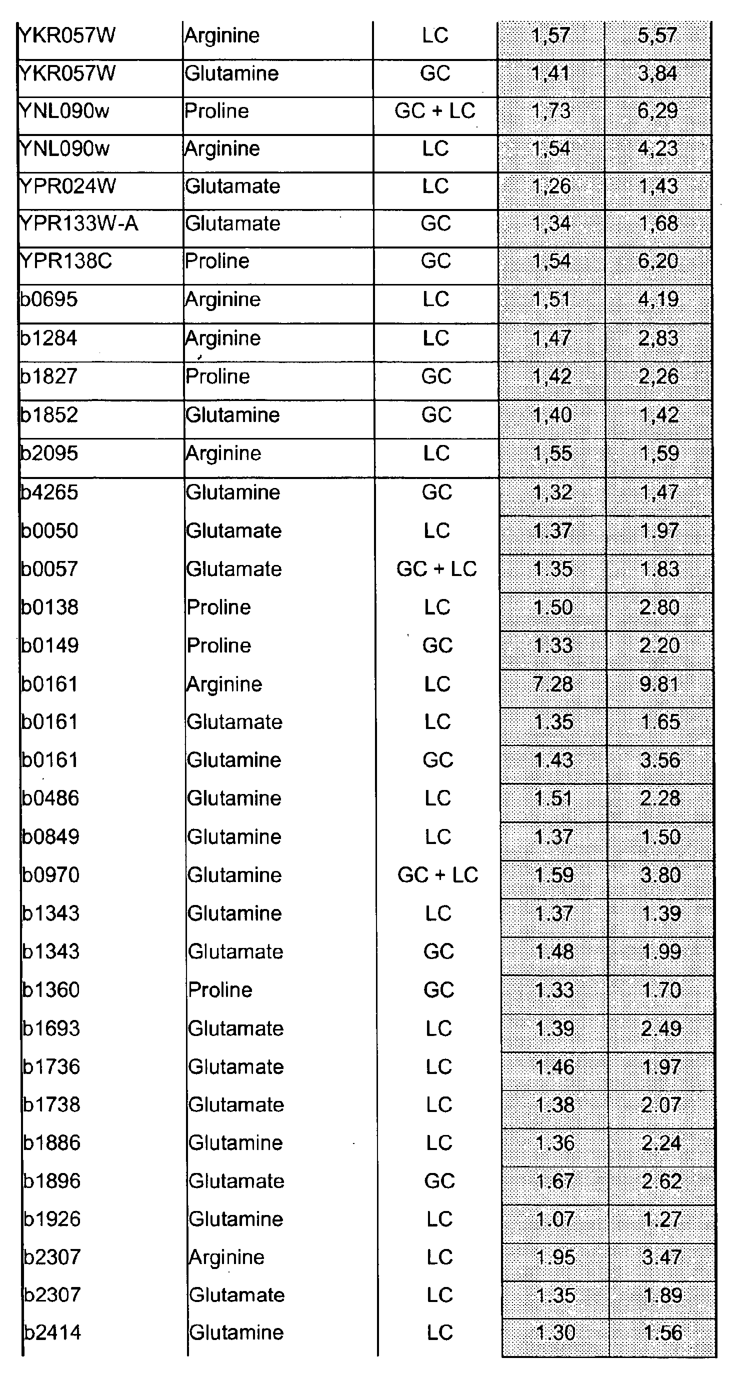

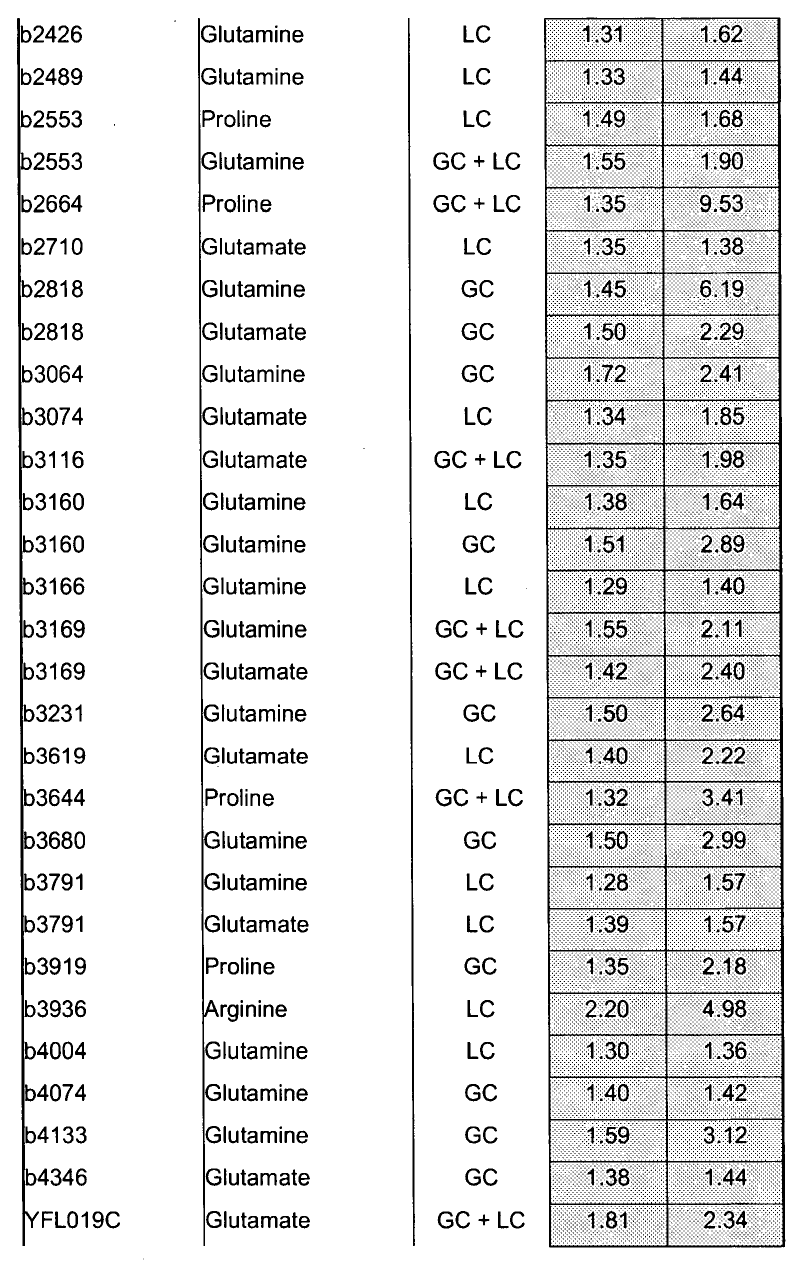

**[0515.0.0.4] to [0552.0.0.4]: see [0515.0.0.0] to [0552.0.0.0]**

**[0552.1.0.1]:** Example 15: Metabolite Profiling Info from *Zea mays*
*Zea mays* plants were engineered as described in Example 14c.
The results of the different *Zea mays* plants analysed can be seen from table 2 which follows:

**Table 2**

| ORF_NAME | Metabolite | Min | Max |
|---|---|---|---|
| YKR057W | Glutamine | **2.30** | **6.16** |
| YIL150C | Proline | **1.77** | **3.45** |

Table 2 describes the increase in proline and glutamine in genetically modified corn plants expressing the Saccharomyces cerevisiae nucleic acid sequence YIL150c or YKR057w resp..
In one embodiment, in case the activity of the Saccaromyces cerevisiae protein YIL150C or its homologs, e.g. "a chromatin binding protein, required for S-phase (DNA synthesis) initiation or completion" or its homologs, is increased in corn plants, preferably, an increase of the fine chemical proline between 77% and 245% is conferred.
In case the activity of the Saccharomyces cerevisiae protein YKR057W or a ribosomal protein, similar to S21 ribosomal proteins, involved in ribosome biogenesis and translation or its homolog, is increased in corn plants, preferably, an increase of the fine chemical glutamine between 130% and 516% is conferred.

**[0552.2.0.4]:** see **[0552.2.0.0]**

**[0553.0.4.4]**
1. A process for the production of arginine and/or glutamate and/or proline and/or glutamine, which comprises
   (a) increasing or generating the activity of a protein as indicated in Table II, columns 5 or 7, lines 34 to 37, 390, 405 and/or 430 for arginine
      and/or lines 43, 386, 387, 391, 396, 399 to 401, 403, 406, 413, 414, 417, 418, 421, 424, 427, 434, and/or 435 for glutamate
      and/or lines 54 to 56, 388, 389, 398, 411, 412, 425 and/or 429 for proline
      and/or lines 62, 392 to 395, 397, 402, 404, 407 to 410, 415, 416, 419, 420, 422, 423, 426, 428 and/or 431 to 433 for glutamine resp., or a functional equivalent thereof in a non-human organism, or in one or more parts thereof; and
   (b) growing the organism under conditions which permit the production of arginine and/or glutamate and/or proline and/or glutamine resp. in said organism.
2. A process for the production of arginine and/or glutamate and/or proline and/or glutamine, comprising the increasing or generating in an organism or a part thereof the expression of at least one nucleic acid molecule comprising a nucleic acid molecule selected from the group consisting of:
   (a) nucleic acid molecule encoding of a polypeptide as indicated in Table II, columns 5 or 7, lines 34 to 37, 390, 405 and/or 430 for arginine
      and/or lines 43, 386, 387, 391, 396, 399 to 401, 403, 406, 413, 414, 417, 418, 421, 424, 427, 434, and/or 435 for glutamate
      and/or lines 54 to 56, 388, 389, 398, 411, 412, 425 and/or 429 for proline
      and/or lines 62, 392 to 395, 397, 402, 404, 407 to 410, 415, 416, 419, 420, 422, 423, 426, 428 and/or 431 to 433 for glutamine resp., or a fragment thereof, which confers an increase in the amount of arginine and/or glutamate and/or proline and/or glutamine in an organism or a part thereof;
   b) nucleic acid molecule comprising of a nucleic acid molecule as indicated in Table I, columns 5 or 7, lines 34 to 37, 390, 405 and/or 430 for arginine and/or lines 43, 386, 387, 391, 396, 399 to 401, 403, 406, 413, 414, 417, 418, 421, 424, 427, 434, and/or 435 for glutamate
      and/or lines 54 to 56, 388, 389, 398, 411, 412, 425 and/or 429 for proline
      and/or lines 62, 392 to 395, 397, 402, 404, 407 to 410, 415, 416, 419, 420, 422, 423, 426, 428 and/or 431 to 433 for glutamine resp.;
   c) nucleic acid molecule whose sequence can be deduced from a polypeptide sequence encoded by a nucleic acid molecule of (a) or (b) as a result of the degeneracy of the genetic code and conferring an increase in the amount of arginine and/or glutamate and/or proline and/or glutamine in an organism or a part thereof;
   d) nucleic acid molecule which encodes a polypeptide which has at least 50% identity with the amino acid sequence of the polypeptide encoded by the nucleic acid molecule of (a) to (c) and conferring an increase in the amount of arginine and/or glutamate and/or proline and/or glutamine in an organism or a part thereof;
   e) nucleic acid molecule which hybidizes with a nucleic acid molecule of (a) to (c) under under stringent hybridisation conditions and conferring an increase in the amount of arginine and/or glutamate and/or proline and/or glutamine in an organism or a part thereof;
   f) nucleic acid molecule which encompasses a nucleic acid molecule which is obtained by amplifying nucleic acid molecules from a cDNA library or a genomic library using the primers or primer pairs as indicated in Table III, columns 5 or 7, lines 34 to 37, 390, 405 and/or 430 for arginine
      and/or lines 43, 386, 387, 391, 396, 399 to 401, 403, 406, 413, 414, 417, 418, 421, 424, 427, 434, and/or 435 for glutamate
      and/or lines 54 to 56, 388, 389, 398, 411, 412, 425 and/or 429 for proline and/or lines 62, 392 to 395, 397, 402, 404, 407 to 410, 415, 416, 419, 420, 422, 423, 426, 428 and/or 431 to 433 for glutamine resp., and conferring an increase in the amount of arginine and/or glutamate and/or proline and/or glutamine in an organism or a part thereof;
   g) nucleic acid molecule encoding a polypeptide which is isolated with the aid of monoclonal antibodies against a polypeptide encoded by one of the nucleic acid molecules of (a) to (f) and conferring an increase in the amount of arginine and/or glutamate and/or proline and/or glutamine in an organism or a part thereof;
   h) nucleic acid molecule encoding a polypeptide comprising a consensus as indicated in Table IV, column 7, lines 34 to 37, 390, 405 and/or 430 for arginine
      and/or lines 43, 386, 387, 391, 396, 399 to 401, 403, 406, 413, 414, 417, 418, 421, 424, 427, 434, and/or 435 for glutamate
      and/or lines 54 to 56, 388, 389, 398, 411, 412, 425 and/or 429 for proline and/or lines 62, 392 to 395, 397, 402, 404, 407 to 410, 415, 416, 419, 420, 422, 423, 426, 428 and/or 431 to 433 for glutamine resp., and conferring an increase in the amount of arginine and/or glutamate and/or proline and/or glutamine in an organism or a part thereof; and
   i) nucleic acid molecule which is obtainable by screening a suitable nucleic acid library under stringent hybridization conditions with a probe comprising one of the sequences of the nucleic acid molecule of (a) to (k) or with a fragment thereof having at least 15 nt, preferably 20 nt, 30 nt, 50 nt, 100 nt, 200 nt or 500 nt of the nucleic acid molecule characterized in (a) to (k) and conferring an increase in the amount of the fine chemical in an organism or a part thereof.
   or comprising a sequence which is complementary thereto.
3. The process of claim 1 or 2, comprising recovering of the free or bound arginine and/or glutamate and/or proline and/or glutamine.
4. The process of any one of claim 1 to 3, comprising the following steps:
   (a) selecting an organism or a part thereof expressing a polypeptide encoded by the nucleic acid molecule characterized in claim 2;
   (b) mutagenizing the selected organism or the part thereof;
   (c) comparing the activity or the expression level of said polypeptide in the mutagenized organism or the part thereof with the activity or the expression of said polypeptide of the selected organisms or the part thereof;
   (d) selecting the mutated organisms or parts thereof, which comprise an increased activity or expression level of said polypeptide compared to the selected organism or the part thereof;
   (e) optionally, growing and cultivating the organisms or the parts thereof; and
   (f) recovering, and optionally isolating, the free or bound arginine and/or glutamate and/or proline and/or glutamine produced by the selected mutated organisms or parts thereof.
5. The process of any one of claims 1 to 4, wherein the activity of said protein or the expression of said nucleic acid molecule is increased or generated transiently or stably.
6. An isolated nucleic acid molecule comprising a nucleic acid molecule selected from the group consisting of:
   a) nucleic acid molecule encoding of a polypeptide as indicated in Table II, columns 5 or 7, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp., or a fragment thereof, which confers an increase in the amount of arginine and/or glutamate and/or proline and/or glutamine in an organism or a part thereof;
   b) nucleic acid molecule comprising of a nucleic acid molecule as indicated in Table I, columns 5 or 7, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp.;
   c) nucleic acid molecule whose sequence can be deduced from a polypeptide sequence encoded by a nucleic acid molecule of (a) or (b) as a result of the degeneracy of the genetic code and conferring an increase in the amount of arginine and/or glutamate and/or proline and/or glutamine in an organism or a part thereof;
   d) nucleic acid molecule which encodes a polypeptide which has at least 50% identity with the amino acid sequence of the polypeptide encoded by the nucleic acid molecule of (a) to (c) and conferring an increase in the amount of arginine and/or glutamate and/or proline and/or glutamine in an organism or a part thereof;
   e) nucleic acid molecule which hybidizes with a nucleic acid molecule of (a) to (c) under under stringent hybridisation conditions and conferring an increase in the amount of arginine and/or glutamate and/or proline and/or glutamine in an organism or a part thereof;
   f) nucleic acid molecule which encompasses a nucleic acid molecule which is obtained by amplifying nucleic acid molecules from a cDNA library or a genomic library using the primers or primer pairs as indicated in Table III, column 7, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp., and conferring an increase in the amount of arginine and/or glutamate and/or proline and/or glutamine in an organism or a part thereof;
   g) nucleic acid molecule encoding a polypeptide which is isolated with the aid of monoclonal antibodies against a polypeptide encoded by one of the nucleic acid molecules of (a) to (f) and conferring an increase in the amount of arginine and/or glutamate and/or proline and/or glutamine in an organism or a part thereof;
   h) nucleic acid molecule encoding a polypeptide comprising a consensus as indicated in Table IV, column 7, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp., and conferring an increase in the amount of arginine and/or glutamate and/or proline and/or glutamine in an organism or a part thereof; and
   i) nucleic acid molecule which is obtainable by screening a suitable nucleic acid library under stringent hybridization conditions with a probe comprising one of the sequences of the nucleic acid molecule of (a) to (k) or with a fragment thereof having at least 15 nt, preferably 20 nt, 30 nt, 50 nt, 100 nt, 200 nt or 500 nt of the nucleic acid molecule characterized in (a) to (k) and conferring an increase in the amount of the fine chemical in an organism or a part thereof.
   whereby the nucleic acid molecule distinguishes over the sequence as indicated in Table I A, columns 5 or 7, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp., by one or more nucleotides.
7. A nucleic acid construct which confers the expression of the nucleic acid molecule of claim 6, comprising one or more regulatory elements.
8. A vector comprising the nucleic acid molecule as claimed in claim 6 or the nucleic acid construct of claim 7.
9. The vector as claimed in claim 8, wherein the nucleic acid molecule is in operable linkage with regulatory sequences for the expression in a prokaryotic or eukaryotic, or in a prokaryotic and eukaryotic, host.
10. A host cell, which has been transformed stably or transiently with the vector as claimed in claim 9 or 10 or the nucleic acid molecule as claimed in claim 6 or the nucleic acid construct of claim 7 or produced as described in claim any one of claims 2 to 5.
11. The host cell of claim 10, which is a transgenic host cell.
12. The host cell of claim 10 or 11, which is a plant cell, an animal cell, a microorganism, or a yeast cell, a fungus cell, a prokaryotic cell, an eukaryotic cell or an archaebacterium.
13. A process for producing a polypeptide, wherein the polypeptide is expressed in a host cell as claimed in any one of claims 10 to 12.
14. A polypeptide produced by the process as claimed in claim 13 or encoded by the nucleic acid molecule as claimed in claim 6 whereby the polypeptide distinguishes over a sequence as indicated in Table II A, columns 5 or 7, lines 34 to 37, 390, 405 and/or 430 and/or lines 43, 386, 387, 391, 396, 399 to 401, 403, 406, 413, 414, 417, 418, 421, 424, 427, 434, and/or 435 and/or lines 54 to 56, 388, 389, 398, 411, 412, 425 and/or 429 and/or lines 62, 392 to 395, 397, 402, 404, 407 to 410, 415, 416, 419, 420, 422, 423, 426, 428 and/or 431 to 433 resp., by one or more amino acids.
15. An antibody, which binds specifically to the polypeptide as claimed in claim 14.
16. A plant tissue, propagation material, harvested material or a plant comprising the host cell as claimed in claim 12 which is plant cell or an Agrobacterium.
17. A method for screening for agonists and antagonists of the activity of a polypeptide encoded by the nucleic acid molecule of claim 6 conferring an increase in the amount of arginine and/or glutamate and/or proline and/or glutamine in an organism or a part thereof comprising:
   (a) contacting cells, tissues , plants or microorganisms which express the a polypeptide encoded by the nucleic acid molecule of claim 6 conferring an increase in the amount of arginine and/or glutamate and/or proline and/or glutamine in an organism or a part thereof with a candidate compound or a sample comprising a plurality of compounds under conditions which permit the expression the polypeptide;
   (b) assaying the arginine and/or glutamate and/or proline and/or glutamine level or the polypeptide expression level in the cell, tissue, plant or microorganism or the media the cell, tissue, plant or microorganisms is cultured or maintained in; and
   (c) identifying a agonist or antagonist by comparing the measured arginine and/or glutamate and/or proline and/or glutamine level or polypeptide expression level with a standard arginine and/or glutamate and/or proline and/or glutamine or polypeptide expression level measured in the absence of said candidate compound or a sample comprising said plurality of compounds, whereby an increased level over the standard indicates that the compound or the sample comprising said plurality of compounds is an agonist and a decreased level over the standard indicates that the compound or the sample comprising said plurality of compounds is an antagonist.
18. A process for the identification of a compound conferring increased arginine and/or glutamate and/or proline and/or glutamine production in a plant or microorganism, comprising the steps:
   a) culturing a plant cell or tissue or microorganism or maintaining a plant expressing the polypeptide encoded by the nucleic acid molecule of claim 6 conferring an increase in the amount of arginine and/or glutamate and/or proline and/or glutamine in an organism or a part thereof and a readout system capable of interacting with the polypeptide under suitable conditions which permit the interaction of the polypeptide with dais readout system in the presence of a compound or a sample comprising a plurality of compounds and capable of providing a detectable signal in response to the binding of a compound to said polypeptide under conditions which permit the expression of said readout system and of the polypeptide encoded by the nucleic acid molecule of claim 5 conferring an increase in the amount of arginine and/or glutamate and/or proline and/or glutamine in an organism or a part thereof;
   b) identifying if the compound is an effective agonist by detecting the presence or absence or increase of a signal produced by said readout system.
19. A method for the identification of a gene product conferring an increase in arginine and/or glutamate and/or proline and/or glutamine production in a cell, comprising the following steps:
   (a) contacting the nucleic acid molecules of a sample, which can contain a candidate gene encoding a gene product conferring an increase in arginine and/or glutamate and/or proline and/or glutamine after expression with the nucleic acid molecule of claim 6;
   (b) identifying the nucleic acid molecules, which hybridise under relaxed stringent conditions with the nucleic acid molecule of claim 6;
   (c) introducing the candidate nucleic acid molecules in host cells appropriate for producing arginine and/or glutamate and/or proline and/or glutamine;
   (d) expressing the identified nucleic acid molecules in the host cells;
   (e) assaying the arginine and/or glutamate and/or proline and/or glutamine level in the host cells; and
   (f) identifying nucleic acid molecule and its gene product which expression confers an increase in the arginine and/or glutamate and/or proline and/or glutamine level in the host cell in the host cell after expression compared to the wild type.
20. A method for the identification of a gene product conferring an increase in arginine and/or glutamate and/or proline and/or glutamine production in a cell, comprising the following steps:
   (a) identifiying in a data bank nucleic acid molecules of an organism; which can contain a candidate gene encoding a gene product conferring an increase in the arginine and/or glutamate and/or proline and/or glutamine amount or level in an organism or a part thereof after expression, and which are at least 20% homolog to the nucleic acid molecule of claim 6;
   (b) introducing the candidate nucleic acid molecules in host cells appropriate for producing arginine and/or glutamate and/or proline and/or glutamine;
   (c) expressing the identified nucleic acid molecules in the host cells;
   (d) assaying the arginine and/or glutamate and/or proline and/or glutamine level in the host cells; and
   (e) identifying nucleic acid molecule and its gene product which expression confers an increase in the arginine and/or glutamate and/or proline and/or glutamine level in the host cell after expression compared to the wild type.
21. A method for the production of an agricultural composition comprising the steps of the method of any one of claims 17 to 20 and formulating the compound identified in any one of claims 17 to 20 in a form acceptable for an application in agriculture.
22. A composition comprising the nucleic acid molecule of claim 6, the polypeptide of claim 14, the nucleic acid construct of claim 7, the vector of any one of claims 8 or 9, an antagonist or agonist identified according to claim 17, the compound of claim 18, the gene product of claim 19 or 20, the antibody of claim 15, and optionally an agricultural acceptable carrier.
23. Use of the nucleic acid molecule as claimed in claim 6 for the identification of a nucleic acid molecule conferring an increase of arginine and/or glutamate and/or proline and/or glutamine after expression.
24. Use of the polypeptide of claim 14 or the nucleic acid construct claim 7 or the gene product identified according to the method of claim 19 or 20 for identifying compounds capable of conferring a modulation of arginine and/or glutamate and/or proline and/or glutamine levels in an organism.
25. Food or feed composition comprising the nucleic acid molecule of claim 6, the polypeptide of claim 14, the nucleic acid construct of claim 7, the vector of claim 8 or 9, the antagonist or agonist identified according to claim 17, the antibody of claim 14, the plant or plant tissue of claim 16, the harvested material of claim 16, the host cell of claim 10 to 12 or the gene product identified according to the method of claim 19 or 20.
26. Use of the nucleic acid molecule of claim 6, the polypeptide of claim 14, the nucleic acid construct of claim 7, the vector of claim 8 or 9, the antagonist or agonist identified according to claim 17, the antibody of claim 15, the plant or plant tissue of claim 16, the harvested material of claim 16, the host cell of claim 10 to 12 or the gene product identified according to the method of claim 19 or 20 for the protection of a plant against a arginine and/or glutamate and/or proline and/or glutamine synthesis inhibiting herbicide.

**[0554.0.0.4] Abstract: see [0554.0.0.0]**
Process for the production of fine chemicals

[0000.0.0.5] In a further embodiment, the present invention relates to a further process for the production of fine chemicals as defined below and the corresponding embodiments as described herein as follows.

**[0001.0.0.5]** for the disclosure of this paragraph see [0001.0.0.0].

**[0002.0.5.5]** Fatty acids are the building blocks of triglycerides, lipids, oils and fats. Some of the fatty acids such as linoleic or linolenic acid are "essential" because the human body is not able to synthesize them but needs them, so humans must ingest them through the diet. Other fatty acids can be synthesized by the human body, therefore they are not labeled as "essential". Nevertheless very often the amount of production of for example fatty acids such as eicosapentaenoic acid (= EPA, C_{20:5}^{Δ5,8,11,14,17}) or docosahexaenoic acid (= DHA, C_{22:6}^{Δ4,7,10,13,16,19}) in the body is not sufficient for an optimal body function. Polyunsaturated fatty acids (= PUFA) that means fatty acids, which have more than 1 double bond in the carbon chain are divided into families depending on where their end-most double bond is located. There are two main subtypes of fatty acids: the omega-3 and omega-6 fatty acids. The Omega-3's are those with their endmost double bond 3 carbons from their methyl end. The Omega-6's are those with their endmost double bond 6 carbons from their methyl end. Linoleic acid (an omega-6) and alpha-linolenic acid (an omega-3) are the only true "essential" fatty acids. Both are used inside the body as starting material to synthesize others such as EPA or DHA.

**[0003.0.5.5]** Fatty acids and triglycerides have numerous applications in the food and feed industry, in cosmetics and in the drug sector. Depending on whether they are free saturated or unsaturated fatty acids or triglycerides with an increased content of saturated or unsaturated fatty acids, they are suitable for the most varied applications; thus, for example, polyunsaturated fatty acids (= PUFAs) are added to infant formula to increase its nutritional value. The various fatty acids and triglycerides are mainly obtained from microorganisms such as fungi or from oil-producing plants including phytoplankton and algae, such as soybean, oilseed rape, sunflower and others, where they are usually obtained in the form of their triacylglycerides.

**[0004.0.5.5]** Principally microorganisms such as Mortierella or oil producing plants such as soybean, rapeseed or sunflower or algae such as Crytocodinium or Phaeodactylum are a common source for oils containing PUFAs, where they are usually obtained in the form of their triacyl glycerides. Alternatively, they are obtained advantageously from animals, such as fish. The free fatty acids are prepared advantageously by hydrolysis with a strong base such as potassium or sodium hydroxide. Higher poly unsaturated fatty acids such as DHA, EPA, ARA, Dihomo-γ-linoleic acid (C_{20:3}^{Δ8,11,14}) or Docosapentaenoic acid (= DPA, C_{22:5}^{Δ7,10,13,16,19}) are not produced by oil producing plants such as soybean, rapeseed, safflower or sunflower. A natural sources for said fatty acids are fish for example herring, salmon, sardine, redfish, eel, carp, trout, halibut, mackerel, pike-perch or tuna or algae.

**[0005.0.5.5]** Whether oils with unsaturated or with saturated fatty acids are preferred depends on the intended purpose; thus, for example, lipids with unsaturated fatty acids, specifically polyunsaturated fatty acids, are preferred in human nutrition since they have a positive effect on the cholesterol level in the blood and thus on the possibility of heart disease. They are used in a variety of dietetic foodstuffs or medicaments. In addition PUFAs are commonly used in food, feed and in the cosmetic industry. Poly unsaturated w-3- and/or ω-6-fatty acids are an important part of animal feed and human food. Because of the common composition of human food poly unsaturated ω-3-fatty acids, which are an essential component of fish oil, should be added to the food to increase the nutritional value of the food; thus, for example, polyunsaturated fatty acids such as DHA or EPA are added as mentioned above to infant formula to increase its nutritional value. The true essential fatty acids linoleic and linolenic fatty acid have a lot of positive effects in the human body such as a positive effect on healthy heart, arteries and skin. They bring for example relieve from eczema, diabetic neuropathy or PMS and cyclical breast pain.

**[0006.0.5.5]** Poly unsaturated w-3- and ω-6-fatty acids are for example precursor of a family of paracrine hormones called eicosanoids such as prostaglandins which are products of the metabolism of Dihomo-γ-linoleic acid, ARA or EPA. Eicosanoids are involved in the regulation of lipolysis, the initiation of inflammatory responses, the regulation of blood circulation and pressure and other central functions of the body. Eicosanoids comprise prostaglandins, leukotrienes, thromboxanes, and prostacyclins. ω-3-fatty acids seem to prevent artherosclerosis and cardiovascular diseases primarily by regulating the levels of different eicosanoids. Other Eicosanoids are the thromboxanes and leukotrienes, which are products of the metabolism of ARA or EPA.

**[0007.0.5.5]** On account of their positive properties there has been no shortage of attempts in the past to make available genes which participate in the synthesis of fatty acids or triglycerides for the production of oils in various organisms having a modified content of unsaturated fatty acids.

**[0008.0.5.5]** Methods of recombinant DNA technology have also been used for some years to improve the oil content in microorganisms or plants by amplifying individual fatty acid biosynthesis genes and investigating the effect on fatty acid production. For example in WO 91/13972 a Δ-9-desaturase is described, which is involved in the synthesis of polyunsaturated fatty acids. In WO 93/11245 a Δ-15-desaturase and in WO 94/11516 a Δ-12-desaturase is claimed. Other desaturases are described, for example, in EP-A-0 550 162, WO 94/18337, WO 97/30582, WO 97/21340, WO 95/18222, EP-A-0 794 250, Stukey et al., J. Biol. Chem., 265, 1990: 20144-20149, Wada et al., Nature 347, 1990: 200-203 or Huang et al., Lipids 34, 1999: 649-659. To date, however, the various desaturases have been only inadequately characterized biochemically since the enzymes in the form of membrane-bound proteins are isolable and characterizable only with very great difficulty (McKeon et al., Methods in Enzymol. 71, 1981: 12141-12147, Wang et al., Plant Physiol. Biochem., 26, 1988: 777-792). Generally, membrane-bound desaturases are characterized by introduction into a suitable organism, which is then investigated for enzyme activity by means of analysis of starting materials and products. With regard to the effectiveness of the expression of desaturases and their effect on the formation of polyunsaturated fatty acids it may be noted that through expression of a desaturases and elongases as described to date only low contents of poly-unsaturated fatty acids/lipids have been achieved. Therefore, an alternative and more effective pathway with higher product yield is desirable.

**[0009.0.5.5]** As described above, the essential fatty acids are necessary for humans and many mammals, for example for livestock. In a study of middle-aged men disclosed by Finnish researchers (International Journal of Cancer, September 1, 2004), high intake of linoleic acid seemed to lower the risk of prostate and other cancers. In another publication the positive influence on stroke is disclosed (Umemura et al., Stroke, 2002, vol. 33, pp. 2086-2093).

**[0010.0.5.5]** Therefore improving the quality of foodstuffs and animal feeds is an important task of the food-and-feed industry. This is necessary since, for example, as mentioned above certain fatty acids, which occur in plants are limited with regard to the supply of mammals. Especially advantageous for the quality of foodstuffs and animal feeds is as balanced as possible fatty acid profile in the diet since a great excess of omega-3-fatty acids above a specific concentration in the food has no positive effect unless the omega-3-fatty acid content is in balance to the omega-6-fatty acid content of the diet. A further increase in quality is only possible via addition of further fatty acids, which are limiting under these conditions. The targeted addition of the limiting fatty acid in the form of synthetic products must be carried out with extreme caution in order to avoid fatty acid imbalance.

[0011.0.5.5] To ensure a high quality of foods and animal feeds, it is therefore necessary to add a plurality of fatty acids in a balanced manner to suit the organism.

**[0012.0.5.5]** Accordingly, there is still a great demand for new and more suitable genes which encode proteins which participate in the biosynthesis of fatty acids and make it possible to produce certain fatty acids specifically on an industrial scale without unwanted byproducts forming. In the selection of genes for biosynthesis two characteristics above all are particularly important. On the one hand, there is as ever a need for improved processes for obtaining the highest possible contents of polyunsaturated fatty acids on the other hand as less as possible byproducts should be produced in the production process.

**[0013.0.0.5]** for the disclosure of this paragraph see [0013.0.0.0] above.

**[0014.0.5.5]** Accordingly, in a first embodiment, the invention relates to a process for the production of a fine chemical, whereby the fine chemical is linoleic acid or tryglycerides, lipids, oils or fats containing linoleic acid. Accordingly, in the present invention, the term "the fine chemical" as used herein relates to "linoleic acid and/or tryglycerides, lipids, oils and/or fats containing linoleic acid". Further, the term "the fine chemicals" as used herein also relates to fine chemicals comprising linoleic acid and/or triglycerides, lipids, oils and/or fats containing linoleic acid.

**[0015.0.5.5]** In one embodiment, the term "the fine chemical" or "the respective fine chemical" means linoleic acid and/or tryglycerides, lipids, oils and/or fats containing linoleic acid. Throughout the specification the term "the fine chemical" or "the respective fine chemical" means linoleic acid and/or tryglycerides, lipids, oils and/or fats containing linoleic acid, linoleic acid and its salts, ester, thioester or linoleic acid in free form or bound to other compounds such as triglycerides, glycolipids, phospholipids etc. In a preferred embodiment, the term "the fine chemical" means linoleic acid, in free form or its salts or bound to triglycerides. Triglycerides, lipids, oils, fats or lipid mixture thereof shall mean any triglyceride, lipid, oil and/or fat containing any bound or free linoleic acid for example sphingolipids, phosphoglycerides, lipids, glycolipids such as glycosphingolipids, phospholipids such as phosphatidylethanolamine, phosphatidylcholine, phosphatidylserine, phosphatidylglycerol, phosphatidylinositol or diphosphatidylglycerol, or as monoacylglyceride, diacylglyceride or triacylglyceride or other fatty acid esters such as acetyl-Coenzym A thioester, which contain further saturated or unsaturated fatty acids in the fatty acid molecule.
In one embodiment, the term "the fine chemical" and the term "the respective fine chemical" mean at least one chemical compound with an activity of the above mentioned fine chemical.

**[0016.0.5.5]** Accordingly, the present invention relates to a process comprising
(a) increasing or generating the activity of one or more b0730, b3256, YBR089C-A, YDR447C, YOR024W, b0050, b0251, b0255, b0577, b0849, b1097, b1693, b2710, b2822, b3064, b3166, b3457, b3644 and/or b4129 protein(s) or of a protein having the sequence of a polypeptide encoded by a nucleic acid molecule indicated in Table I, columns 5 or 7, lines 63 to 67 and 436 to 449 in a non-human organism or in one or more parts thereof and
(b) growing the organism under conditions which permit the production of the fine chemical, thus, linoleic acid or fine chemicals comprising linoleic acid, in said organism.
Accordingly, the present invention relates to a process for the production of a fine chemical comprising
(a) increasing or generating the activity of one or more proteins having the activity of a protein indicated in Table II, column 3, lines 63 to 67 and 436 to 449 or having the sequence of a polypeptide encoded by a nucleic acid molecule indicated in Table I, column 5 or 7, lines 63 to 67 and 436 to 449, in a non-human organism in one or more parts thereof and
(b) growing the organism under conditions which permit the production of the fine chemical, in particular linoleic acid.

**[0017.0.0.5] and [0018.0.0.5]** for the disclosure of the paragraphs [0017.0.0.5] and [0018.0.0.5] see paragraphs [0017.0.0.0] and [0018.0.0.0] above.

**[0019.0.5.5]** Advantageously the process for the production of the fine chemical leads to an enhanced production of the fine chemical. The terms "enhanced" or "increase" mean at least a 10%, 20%, 30%, 40% or 50%, preferably at least 60%, 70%, 80%, 90% or 100%, more preferably 150%, 200%, 300%, 400% or 500% higher production of the fine chemical in comparison to the reference as defined below, e.g. that means in comparison to an organism without the aforementioned modification of the activity of a protein having the activity of a protein indicated in Table II, column 3, lines 63 to 67 and 436 to 449 or encoded by nucleic acid molecule indicated in Table I, columns 5 or 7, lines 63 to 67 and 436 to 449.

**[0020.0.5.5]** Surprisingly it was found, that the transgenic expression of at least one of the Saccaromyces cerevisiae protein(s) indicated in Table II, Column 3, lines 65 to 67, and/or the Escherichia coli K12 protein(s) indicated in Table II, Column 3, lines 63 to 64 and 436 to 449 in Arabidopsis thaliana conferred an increase in the linoleic acid (or fine chemical) content of the transformed plants.

**[0021.0.0.5]** for the disclosure of this paragraph see [0021.0.0.0] above.

**[0022.0.5.5]** The sequence of b0730 from Escherichia coli K12 has been published in Blattner et al., Science 277(5331), 1453-1474, 1997, and its activity is being defined as transcriptional regulator of succinylCoA synthetase operon. Accordingly, in one embodiment, the process of the present invention comprises the use of a transcriptional regulator of succinylCoA synthetase operon from E. coli or its homolog, e.g. as shown herein, for the production of the fine chemical, meaning of linoleic acid and/or tryglycerides, lipids, oils and/or fats containing linoleic acid, in particular for increasing the amount of linoleic acid and/or tryglycerides, lipids, oils and/or fats containing linoleic acid, preferably linoleic acid in free or bound form in an organism or a part thereof, as mentioned. In one embodiment, in the process of the present invention the activity of a transcriptional regulator of succinylCoA synthetase operon is increased or generated, e.g. from E. coli or a homolog thereof.
The sequence of b3256 from Escherichia coli K12 has been published in Blattner et al., Science 277(5331), 1453-1474, 1997, and its activity is being defined as acetyl CoA carboxylase. Accordingly, in one embodiment, the process of the present invention comprises the use of a acetyl CoA carboxylase from E. coli or its homolog, e.g. as shown herein, for the production of the fine chemical, meaning of linoleic acid and/or tryglycerides, lipids, oils and/or fats containing linoleic acid, in particular for increasing the amount of linoleic acid and/or tryglycerides, lipids, oils and/or fats containing linoleic acid, preferably linoleic acid in free or bound form in an organism or a part thereof, as mentioned. In one embodiment, in the process of the present invention the activity of a acetyl CoA carboxylase is increased or generated, e.g. from E. coli or a homolog thereof.
The sequence of YBR089C-A from Saccharomyces cerevisiae has been published in Feldmann et al., EMBO J., 13 (24), 5795-5809 (1994) and Goffeau et al., Science 274 (5287), 546-547, 1996, and its cellular activity has not been characterized yet. It shows homology to mammalian high mobility group proteins 1 and 2. Its function may be redundantly with the highly homologous gene NHP6A. Furthermore it shows homology to the high-mobility group non-histone chromatin protein Nhp6bp. Accordingly, in one embodiment, the process of the present invention comprises the use of a YBR089C-A activity from Saccharomyces cerevisiae or its homolog, e.g. as shown herein, for the production of the fine chemical, meaning of linoleic acid and/or tryglycerides, lipids, oils and/or fats containing linoleic acid, in particular for increasing the amount of linoleic acid and/or tryglycerides, lipids, oils and/or fats containing linoleic acid, preferably linoleic acid in free or bound form in an organism or a part thereof, as mentioned. In one embodiment, in the process of the present invention the activity of a YBR089C-A protein is increased or generated, e.g. from Saccharomyces cerevisiae or a homolog thereof.
The sequence of YDR447C from Saccharomyces cerevisiae has been published in Jacq et al., Nature 387 (6632 Suppl), 75-78, 1997, and Goffeau et al., Science 274 (5287), 546-547, 1996, and its activity is being defined as ribosomal protein 51 (rp51) of the small (40s) subunit; nearly identical to Rps17Ap and has similarity to rat S17 ribosomal protein; Rps17bp. Accordingly, in one embodiment, the process of the present invention comprises the use of a "ribosomal protein 51" or its homolog, e.g. as shown herein, for the production of the fine chemical, meaning of linoleic acid and/or tryglycerides, lipids, oils and/or fats containing linoleic acid, in particular for increasing the amount of linoleic acid and/or tryglycerides, lipids, oils and/or fats containing linoleic acid, preferably linoleic acid in free or bound form in an organism or a part thereof, as mentioned. In one embodiment, in the process of the present invention the activity of a ribosomal protein 51 is increased or generated, e.g. from Saccharomyces cerevisiae or a homolog thereof.
The sequence of YOR024W from Saccharomyces cerevisiae has been submitted from de Haan to the EMBL Protein Sequence Database, July 1996 and its cellular activity has not been characterized yet. It is probably a membrane protein. Accordingly, in one embodiment, the process of the present invention comprises the use of YOR024W, from Saccharomyces cerevisiae or its homolog, e.g. as shown herein, for the production of the fine chemical, meaning of linoleic acid and/or tryglycerides, lipids, oils and/or fats containing linoleic acid, in particular for increasing the amount of linoleic acid and/or tryglycerides, lipids, oils and/or fats containing linoleic acid, preferably linoleic acid in free or bound form in an organism or a part thereof, as mentioned. In one embodiment, in the process of the present invention the activity of a YOR024W is increased or generated, e.g. from Saccharomyces cerevisiae or a homolog thereof.
The sequence of b0050 (Accession number NP_414592) from Escherichia coli K12 has been published in Blattner et al., Science 277 (5331). 1453-1474, 1997, and its activity is being defined as a conserved protein potentially involved in protein protein interaction. Accordingly, in one embodiment, the process of the present invention comprises the use of said protein from E. coli or its homolog, e.g. as shown herein, for the production of the fine chemical, meaning of linoleic acid and/or tryglycerides, lipids, oils and/or fats containing linoleic acid, in particular for increasing the amount of linoleic acid and/or tryglycerides, lipids, oils and/or fats containing linoleic acid, preferably linoleic acid in free or bound form in an organism or a part thereof, as mentioned. In one embodiment, in the process of the present invention the activity of said protein is increased or generated, e.g. from E. coli or a homolog thereof.
The sequence of b0251 (Accession number NP_414785) from Escherichia coli K12 has been published in Blattner et al., Science 277 (5331), 1453-1474, 1997, and its activity is being defined as putative HTH-type transcriptional regulator. Accordingly, in one embodiment, the process of the present invention comprises the use of a putative HTH-type transcriptional regulator protein from E. coli or its homolog, e.g. as shown herein, for the production of the fine chemical, meaning of linoleic acid and/or tryglycerides, lipids, oils and/or fats containing linoleic acid, in particular for increasing the amount of linoleic acid and/or tryglycerides, lipids, oils and/or fats containing linoleic acid, preferably linoleic acid in free or bound form in an organism or a part thereof, as mentioned. In one embodiment, in the process of the present invention the activity of a putative HTH-type transcriptional regulator protein is increased or generated, e.g. from E. coli or a homolog thereof.
The sequence of b0255 (Accession number NP_414789) from Escherichia coli K12 has been published in Blattner et al., Science 277 (5331), 1453-1474, 1997, and its activity is being defined as a CP4-6 prophage; IS911 homolog. Accordingly, in one embodiment, the process of the present invention comprises the use of said CP4-6 prophage; IS911 homolog protein from E. coli or its homolog, e.g. as shown herein, for the production of the fine chemical, meaning of linoleic acid and/or tryglycerides, lipids, oils and/or fats containing linoleic acid, in particular for increasing the amount of linoleic acid and/or tryglycerides, lipids, oils and/or fats containing linoleic acid, preferably linoleic acid in free or bound form in an organism or a part thereof, as mentioned. In one embodiment, in the process of the present invention the activity of said CP4-6 prophage; IS911 homolog protein is increased or generated, e.g. from E. coli or a homolog thereof.
The sequence of b0577 (Accession number NP_415109) from Escherichia coli K12 has been published in Blattner et al., Science 277 (5331), 1453-1474, 1997, and its activity is being defined as a putative transport protein. Accordingly, in one embodiment, the process of the present invention comprises the use of a putative transport protein from E. coli or its homolog, e.g. as shown herein, for the production of the fine chemical, meaning of linoleic acid and/or tryglycerides, lipids, oils and/or fats containing linoleic acid, in particular for increasing the amount of linoleic acid and/or tryglycerides, lipids, oils and/or fats containing linoleic acid, preferably linoleic acid in free or bound form in an organism or a part thereof, as mentioned. In one embodiment, in the process of the present invention the activity of a putative transport protein is increased or generated, e.g. from E. coli or a homolog thereof.
The sequence of b0849 (Accession number NP_415370) from Escherichia coli K12 has been published in Blattner et al., Science 277 (5331), 1453-1474, 1997, and its activity is being defined as a glutaredoxin 1 redox coenzyme for glutathione-dependent ribonucleotide reductase. Accordingly, in one embodiment, the process of the present invention comprises the use of a glutaredoxin 1 redox coenzyme for glutathione-dependent ribonucleotide reductase protein from E. coli or its homolog, e.g. as shown herein, for the production of the fine chemical, meaning of linoleic acid and/or tryglycerides, lipids, oils and/or fats containing linoleic acid, in particular for increasing the amount of linoleic acid and/or tryglycerides, lipids, oils and/or fats containing linoleic acid, preferably linoleic acid in free or bound form in an organism or a part thereof, as mentioned. In one embodiment, in the process of the present invention the activity of a glutaredoxin 1 redox coenzyme for glutathione-dependent ribonucleotide reductase protein is increased or generated, e.g. from E. coli or a homolog thereof.
The sequence of b1097 (Accession number NP_415615) from Escherichia coli K12 has been published in Blattner et al., Science 277 (5331), 1453-1474, 1997, and its activity is being defined as a putative thymidylate kinase. Accordingly, in one embodiment, the process of the present invention comprises the use of a putative thymidylate kinase protein from E. coli or its homolog, e.g. as shown herein, for the production of the fine chemical, meaning of linoleic acid and/or tryglycerides, lipids, oils and/or fats containing linoleic acid, in particular for increasing the amount of linoleic acid and/or tryglycerides, lipids, oils and/or fats containing linoleic acid, preferably linoleic acid in free or bound form in an organism or a part thereof, as mentioned. In one embodiment, in the process of the present invention the activity of a putative thymidylate kinase protein is increased or generated, e.g. from E. coli or a homolog thereof.
The sequence of b1693 (Accession number NP_416208) from Escherichia coli K12 has been published in Blattner et al., Science 277 (5331), 1453-1474, 1997, and its activity is being defined as a 3-dehydroquinate dehydratase. Accordingly, in one embodiment, the process of the present invention comprises the use of a 3-dehydroquinate dehydratase protein from E. coli or its homolog, e.g. as shown herein, for the production of the fine chemical, meaning of linoleic acid and/or tryglycerides, lipids, oils and/or fats containing linoleic acid, in particular for increasing the amount of linoleic acid and/or tryglycerides, lipids, oils and/or fats containing linoleic acid, preferably linoleic acid in free or bound form in an organism or a part thereof, as mentioned. In one embodiment, in the process of the present invention the activity of a 3-dehydroquinate dehydratase protein is increased or generated, e.g. from E. coli or a homolog thereof.
The sequence of b2710 (Accession number NP_417190) from Escherichia coli K12 has been published in Blattner et al., Science 277 (5331), 1453-1474, 1997, and its activity is being defined as a flavorubredoxin (FIRd) bifunctional NO and O₂ reductase. Accordingly, in one embodiment, the process of the present invention comprises the use of a flavorubredoxin (FIRd) bifunctional NO and O₂ reductase protein from E. coli or its homolog, e.g. as shown herein, for the production of the fine chemical, meaning of linoleic acid and/or tryglycerides, lipids, oils and/or fats containing linoleic acid, in particular for increasing the amount of linoleic acid and/or tryglycerides, lipids, oils and/or fats containing linoleic acid, preferably linoleic acid in free or bound form in an organism or a part thereof, as mentioned. In one embodiment, in the process of the present invention the activity of a flavorubredoxin (FIRd) bifunctional NO and O₂ reductase protein is increased or generated, e.g. from E. coli or a homolog thereof.
The sequence of b2822 (Accession number NP_417299) from Escherichia coli K12 has been published in Blattner et al., Science 277 (5331), 1453-1474, 1997, and its activity is being defined as a DNA helicase, ATP-dependent dsDNA/ssDNA exonuclease V subunit, ssDNA endonuclease. Accordingly, in one embodiment, the process of the present invention comprises the use of a DNA helicase, ATP-dependent dsDNA/ssDNA exonuclease V subunit, ssDNA endonuclease protein from E. coli or its homolog, e.g. as shown herein, for the production of the fine chemical, meaning of linoleic acid and/or tryglycerides, lipids, oils and/or fats containing linoleic acid, in particular for increasing the amount of linoleic acid and/or tryglycerides, lipids, oils and/or fats containing linoleic acid, preferably linoleic acid in free or bound form in an organism or a part thereof, as mentioned. In one embodiment, in the process of the present invention the activity of a DNA helicase, ATP-dependent dsDNA/ssDNA exonuclease V subunit, ssDNA endonuclease protein is increased or generated, e.g. from E. coli or a homolog thereof.
The sequence of b3064 (Accession number NP_417536) from Escherichia coli K12 has been published in Blattner et al., Science 277 (5331), 1453-1474, 1997, and its activity is being defined as a putative O-sialoglycoprotein endopeptidase, with actin-like ATPase domain. Accordingly, in one embodiment, the process of the present invention comprises the use of a putative O-sialoglycoprotein endopeptidase, with actin-like ATPase domain protein from E. coli or its homolog, e.g. as shown herein, for the production of the fine chemical, meaning of linoleic acid and/or tryglycerides, lipids, oils and/or fats containing linoleic acid, in particular for increasing the amount of linoleic acid and/or tryglycerides, lipids, oils and/or fats containing linoleic acid, preferably linoleic acid in free or bound form in an organism or a part thereof, as mentioned. In one embodiment, in the process of the present invention the activity of a putative O-sialoglycoprotein endopeptidase, with actin-like ATPase domain protein is increased or generated, e.g. from E. coli or a homolog thereof.
The sequence of b3166 (Accession number NP_417635) from Escherichia coli K12 has been published in Blattner et al., Science 277 (5331), 1453-1474, 1997, and its activity is being defined as a tRNA pseudouridine 5S synthase. Accordingly, in one embodiment, the process of the present invention comprises the use of a tRNA pseudouridine 5S synthase protein from E. coli or its homolog, e.g. as shown herein, for the production of the fine chemical, meaning of linoleic acid and/or tryglycerides, lipids, oils and/or fats containing linoleic acid, in particular for increasing the amount of linoleic acid and/or tryglycerides, lipids, oils and/or fats containing linoleic acid, preferably linoleic acid in free or bound form in an organism or a part thereof, as mentioned. In one embodiment, in the process of the present invention the activity of a tRNA pseudouridine 5S synthase protein is increased or generated, e.g. from E. coli or a homolog thereof.
The sequence of b3457 (Accession number NP_417914) from Escherichia coli K12 has been published in Blattner et al., Science 277 (5331), 1453-1474, 1997, and its activity is being defined as a high-affinity branched-chain amino acid transport protein (ABC superfamily). Accordingly, in one embodiment, the process of the present invention comprises the use of a high-affinity branched-chain amino acid transport protein from E. coli or its homolog, e.g. as shown herein, for the production of the fine chemical, meaning of linoleic acid and/or tryglycerides, lipids, oils and/or fats containing linoleic acid, in particular for increasing the amount of linoleic acid and/or tryglycerides, lipids, oils and/or fats containing linoleic acid, preferably linoleic acid in free or bound form in an organism or a part thereof, as mentioned. In one embodiment, in the process of the present invention the activity of a high-affinity branched-chain amino acid transport protein is increased or generated, e.g. from E. coli or a homolog thereof.
The sequence of b3644 (Accession number NP_418101) from Escherichia coli K12 has been published in Blattner et al., Science 277 (5331), 1453-1474, 1997, and its activity is being defined as an uncharacterized stress-induced protein. Accordingly, in one embodiment, the process of the present invention comprises the use of an uncharacterized stress-induced protein from E. coli or its homolog, e.g. as shown herein, for the production of the fine chemical, meaning of linoleic acid and/or tryglycerides, lipids, oils and/or fats containing linoleic acid, in particular for increasing the amount of linoleic acid and/or tryglycerides, lipids, oils and/or fats containing linoleic acid, preferably linoleic acid in free or bound form in an organism or a part thereof, as mentioned. In one embodiment, in the process of the present invention the activity of an uncharacterized stress-induced protein is increased or generated, e.g. from E. coli or a homolog thereof.
The sequence of b4129 (Accession number NP_418553) from Escherichia coli K12 has been published in Blattner et al., Science 277 (5331), 1453-1474, 1997, and its activity is being defined as a lysine tRNA synthetase. Accordingly, in one embodiment, the process of the present invention comprises the use of a lysine tRNA synthetase protein from E. coli or its homolog, e.g. as shown herein, for the production of the fine chemical, meaning of linoleic acid and/or tryglycerides, lipids, oils and/or fats containing linoleic acid, in particular for increasing the amount of linoleic acid and/or tryglycerides, lipids, oils and/or fats containing linoleic acid, preferably linoleic acid in free or bound form in an organism or a part thereof, as mentioned. In one embodiment, in the process of the present invention the activity of a lysine tRNA synthetase protein is increased or generated, e.g. from E. coli or a homolog thereof.

**[0023.0.5.5]** Homologues ( = homologs) of the present gene products can be derived from any organisms as long as the homologue confers the herein mentioned activity, in particular, confers an increase in the fine chemical amount or content. Further, in the present invention, the term "homologue" relates to the sequence of an organism having the highest sequence homology to the herein mentioned or listed sequences of all expressed sequences of said organism.
However, the person skilled in the art knows, that, preferably, the homologue has said fine-chemical increasing activity and, if known, the same biological function or activity in the organism as at least one of the protein(s) indicated in Table II, column 3, lines 63 to 67 and 436 to 449, e.g. having the sequence of a polypeptide encoded by a nucleic acid molecule comprising the sequence indicated in Table I, columns 5 or 7, lines 63 to 67 and 436 to 449.
In one embodiment, the homolog of any one of the polypeptides indicated in Table II, columns 5 or 7, lines 65 to 67 is a homolog having the same or a similar activity, in particular an increase of activity confers an increase in the content of the fine chemical in the organisms and being derived from an Eukaryot. In one embodiment, the homolog of a polypeptide indicated in Table II, column 3, lines 63, 64 and 436 to 449 is a homolog having the same or a similar activity, in particular an increase of activity confers an increase in the content of the fine chemical in the organisms or part thereof, and being derived from bacteria. In one embodiment, the homolog of a polypeptide indicated in Table II, column 3, lines 65 to 67 is a homolog having the same or a similar activity, in particular an increase of activity confers an increase in the content of the fine chemical in an organisms or part thereof, and being derived from Fungi. In one embodiment, the homolog of a polypeptide indicated in Table II, column 3, lines 63, 64 and 436 to 449 is a homolog having the same or a similar activity, in particular an increase of activity confers an increase in the content of the fine chemical in the organisms or part thereof and being derived from Proteobacteria. In one embodiment, the homolog of a polypeptide indicated in Table II, column 3, lines 65 to 67 is a homolog having the same or a similar activity, in particular an increase of activity confers an increase in the content of the fine chemical in the organisms or a part thereof and being derived from Ascomycota. In one embodiment, the homolog of a polypeptide indicated in Table II, column 3, lines 63, 64 and 436 to 449 is a homolog having the same or a similar activity, in particular an increase of activity confers an increase in the content of the fine chemical in the organisms or part thereof, and being derived from Gammaproteobacteria. In one embodiment, the homolog of a polypeptide polypeptide indicated in Table II, column 3, lines 65 to 67 is a homolog having the same or a similar activity, in particular an increase of activity confers an increase in the content of the fine chemical in the organisms or part thereof, and being derived from Saccharomycotina. In one embodiment, the homolog of a polypeptide indicated in Table II, column 3, lines 63, 64 and 436 to 449 is a homolog having the same or a similar activity, in particular an increase of activity confers an increase in the content of the fine chemical in the organisms or part thereof, and being derived from Enterobacteriales. In one embodiment, the homolog of a polypeptide indicated in Table II, column 3, lines 65 to 67 is a homolog having the same or a similar activity, in particular an increase of activity confers an increase in the content of the fine chemical in the organisms or a part thereof, and being derived from Saccharomycetes. In one embodiment, the homolog of the a polypeptide indicated in Table II, column 3, lines 63, 64 and 436 to 449 is a homolog having the same or a similar activity, in particular an increase of activity confers an increase in the content of the fine chemical in the organisms or part thereof, and being derived from Enterobacteriaceae. In one embodiment, the homolog of a polypeptide indicated in Table II, column 3, lines 65 to 67 is a homolog having the same or a similar activity, in particular an increase of activity confers an increase in the content of the fine chemical in the organisms, and being derived from Saccharomycetales. In one embodiment, the homolog of a polypeptide indicated in Table II, column 3, lines 63, 64 and 436 to 449 is a homolog having the same or a similar activity, in particular an increase of activity confers an increase in the content of the fine chemical in the organisms or a part thereof, and being derived from Escherichia. In one embodiment, the homolog of a polypeptide indicated in Table II, column 3, lines 65 to 67 is a homolog having the same or a similar activity, in particular an increase of activity confers an increase in the content of the fine chemical in the organisms or a part thereof, and being derived from Saccharomycetaceae. In one embodiment, the homolog of a polypeptide indicated in Table II, column 3, line 65 to 67 is a homolog having the same or a similar activity, in particular an increase of activity confers an increase in the content of the fine chemical in the organisms or a part thereof, and being derived from Saccharomycetes.

**[0023.1.0.5] and [0024.0.0.5]** for the disclosure of the paragraphs [0023.1.0.5] and [0024.0.0.5] see [0023.1.0.0] and [0024.0.0.0] above.

**[0025.0.5.5]** In accordance with the invention, a protein or polypeptide has the "activity of a protein as indicated in Table II, column 3, lines 63 to 67 and 436 to 449" if its *de novo* activity, or its increased expression directly or indirectly leads to an increased linoleic acid and/or tryglycerides, lipids, oils and/or fats containing linoleic acid level in the organism or a part thereof, preferably in a cell of said organism and the protein has the above mentioned activities of a protein as indicated in Table II, column 3, lines 63 to 67 and 436 to 449. Throughout the specification the activity or preferably the biological activity of such a protein or polypeptide or an nucleic acid molecule or sequence encoding such protein or polypeptide is identical or similar if it still has the biological or enzymatic activity of a protein as indicated in Table II, column 3, lines 63 to 67 and 436 to 449, or which has at least 10% of the original enzymatic activity, preferably 20%, particularly preferably 30%, most particularly preferably 40% in comparison to a protein of Saccharomyces cerevisiae as indicated in Table II, column 3, lines 65 to 67 and/or a protein of E. coli K12 as indicated in Table II, column 3, lines 63, 64 and 436 to 449.

**[0025.1.0.5] and [0025.2.0.5]** for the disclosure of the paragraphs [0025.1.0.5] and [0025.2.0.5] see paragraphs [0025.1.0.0] and [0025.2.0.0] above.

**[0026.0.0.5] to [0033.0.0.5]** for the disclosure of the paragraphs [0026.0.0.5] to [0033.0.0.5] see paragraphs [0026.0.0.0] and [0033.0.0.0] above.

**[0034.0.5.5]** Preferably, the reference, control or wild type differs form the subject of the present invention only in the cellular activity of the polypeptide of the invention or the polypeptide used in the method of the invention, e.g. as result of an increase in the level of the nucleic acid molecule of the present invention or an increase of the specific activity of the polypeptide of the invention or the polypeptide used in the method of the invention. E.g., it differs by or in the expression level or activity of an protein having the activity of a protein as indicated in Table II, column 3, lines 63 to 67 and 436 to 449 or being encoded by a nucleic acid molecule indicated in Table I, column 5, lines 63 to 67 and 436 to 449 or its homologs, e.g. as indicated in Table I, column 7, lines 63 to 67 and 436 to 449, its biochemical or genetical causes and therefore shows the increased amount of the fine chemical.

**[0035.0.0.5] to [0044.0.0.5]** for the disclosure of the paragraphs [0035.0.0.5] to [0044.0.0.5] see paragraphs [0035.0.0.0] and [0044.0.0.0] above.

**[0045.0.5.5]** In one embodiment, in case the activity of the Escherichia coli K12 protein b0730 or its homologs e.g. a transcriptional regulator of succinylCoA synthetase operon e.g. a transcriptional regulator for regulation of C-compound and carbohydrate utilization, transcriptional control, prokaryotic nucleoid, transcriptional repressor, DNA binding e.g. as indicated in Table II, columns 5 or 7, line 63, is increased, preferably, in one embodiment the increase of the fine chemical, preferably of linoleic acid between 13% and 34% or more is conferred.
In case the activity of the Escherichia coli K12 protein b3256 or its homologs e.g. a acetyl CoA carboxylase e.g. as indicated in Table II, columns 5 or 7, line 64, is increased, preferably, in one embodiment the increase of the fine chemical, preferably of linoleic acid between 13% and 20% or more is conferred.
In case the activity of the Saccharomyces cerevisiae protein YBR089C-A or its homologs, e.g. a "uncharacterized protein YBR089C-A", which shows homology to mammalian high mobility group proteins 1 and 2, gene NHP6A and to high-mobility group non-histone chromatin protein Nhp6bp, e.g. as indicated in Table II, columns 5 or 7, line 65 is increased, preferably, in one embodiment an increase of the fine chemical, preferably of linoleic acid between 36% and 70% or more is conferred.
In case the activity of the Saccharomyces cerevisiae protein YDR447C or its homologs, e.g. a ribosomal protein 51 (rp51) of the small (40s) subunit e.g. as indicated in Table II, columns 5 or 7, line 66 is increased, preferably, in one embodiment an increase of the fine chemical, preferably of linoleic acid between 18% and 140% or more is conferred.
In case the activity of the Saccaromyces cerevisiae protein YOR024W or its homologs e.g. a "uncharacterized protein YOR024W", which is probably a membrane protein e.g. as indicated in Table II, columns 5 or 7, line 67 is increased, preferably, in one embodment the increase of the fine chemical, preferably of linoleic acid, between 16% and 33% or more is conferred.
In case the activity of the Escherichia coli K12 protein b0050 or its homologs e.g. a conserved protein potentially involved in protein protein interaction e.g. as indicated in Table II, columns 5 or 7, line 436, is increased, preferably, in one embodiment the increase of the fine chemical, preferably of linoleic acid between 15% and 37% or more is conferred.
In case the activity of the Escherichia coli K12 protein b0251 or its homologs e.g. a putative HTH-type transcriptional regulator e.g. as indicated in Table II, columns 5 or 7, line 437, is increased, preferably, in one embodiment the increase of the fine chemical, preferably of linoleic acid between 14% and 31% or more is conferred.
In case the activity of the Escherichia coli K12 protein b0255 or its homologs e.g. a CP4-6 prophage; IS911 homolog e.g. as indicated in Table II, columns 5 or 7, line 438, is increased, preferably, in one embodiment the increase of the fine chemical, preferably of linoleic acid between 22% and 31% or more is conferred.
In case the activity of the Escherichia coli K12 protein b0577 or its homologs e.g. a putative transport protein e.g. as indicated in Table II, columns 5 or 7, line 439, is increased, preferably, in one embodiment the increase of the fine chemical, preferably of linoleic acid between 17% and 40% or more is conferred.
In case the activity of the Escherichia coli K12 protein b0849 or its homologs e.g. a glutaredoxin 1 redox coenzyme for glutathione-dependent ribonucleotide reductase e.g. as indicated in Table II, columns 5 or 7, line 440, is increased, preferably, in one embodiment the increase of the fine chemical, preferably of linoleic acid between 19% and 34% or more is conferred.
In case the activity of the Escherichia coli K12 protein b1097 or its homologs e.g. a putative thymidylate kinase e.g. as indicated in Table II, columns 5 or 7, line 441, is increased, preferably, in one embodiment the increase of the fine chemical, preferably of linoleic acid between 15% and 30% or more is conferred.
In case the activity of the Escherichia coli K12 protein b1693 or its homologs e.g. a 3-dehydroquinate dehydratase e.g. as indicated in Table II, columns 5 or 7, line 442, is increased, preferably, in one embodiment the increase of the fine chemical, preferably of linoleic acid between 17% and 32% or more is conferred.
In case the activity of the Escherichia coli K12 protein b2710 or its homologs e.g. a flavorubredoxin (FIRd) bifunctional NO and O₂ reductase e.g. as indicated in Table II, columns 5 or 7, line 443, is increased, preferably, in one embodiment the increase of the fine chemical, preferably of linoleic acid between 15% and 29% or more is conferred.
In case the activity of the Escherichia coli K12 protein b2822 or its homologs e.g. a DNA helicase, ATP-dependent dsDNA/ssDNA exonuclease V subunit, ssDNA endonuclease e.g. as indicated in Table II, columns 5 or 7, line 444, is increased, preferably, in one embodiment the increase of the fine chemical, preferably of linoleic acid between 15% and 27% or more is conferred.
In case the activity of the Escherichia coli K12 protein b3064 or its homologs e.g. a putative O-sialoglycoprotein endopeptidase, with actin-like ATPase domain e.g. as indicated in Table II, columns 5 or 7, line 445, is increased, preferably, in one embodiment the increase of the fine chemical, preferably of linoleic acid between 17% and 30% or more is conferred.
In case the activity of the Escherichia coli K12 protein b3166 or its homologs e.g. a tRNA pseudouridine 5S synthase e.g. as indicated in Table II, columns 5 or 7, line 446, is increased, preferably, in one embodiment the increase of the fine chemical, preferably of linoleic acid between 14% and 21% or more is conferred.
In case the activity of the Escherichia coli K12 protein b3457 or its homologs e.g. a high-affinity branched-chain amino acid transport protein (ABC superfamily) e.g. as indicated in Table II, columns 5 or 7, line 447, is increased, preferably, in one embodiment the increase of the fine chemical, preferably of linoleic acid between 20% and 44% or more is conferred.
In case the activity of the Escherichia coli K12 protein b3644 or its homologs e.g. an uncharacterized stress-induced protein e.g. as indicated in Table II, columns 5 or 7, line 448, is increased, preferably, in one embodiment the increase of the fine chemical, preferably of linoleic acid between 14% and 35% or more is conferred.
In case the activity of the Escherichia coli K12 protein b4129 or its homologs e.g. a lysine tRNA synthetase e.g. as indicated in Table II, columns 5 or 7, line 449, is increased, preferably, in one embodiment the increase of the fine chemical, preferably of linoleic acid between 20% and 47% or more is conferred.

**[0046.0.5.5]** In one embodiment, in case the activity of the Escherichia coli K12 protein b0730 or its homologs e.g. a transcriptional regulator of succinylCoA synthetase operon is increased, preferably an increase of the fine chemical and of tryglycerides, lipids, oils and/or fats containing linoleic acid is conferred.
In one embodiment, in case the activity of the Escherichia coli K12 protein b3256 or its homologs e.g. a acetyl CoA carboxylase is increased, preferably an increase of the fine chemical and of tryglycerides, lipids, oils and/or fats containing linoleic acid is conferred.
In one embodiment, in case the activity of the Saccaromyces cerevisiae protein YBR089C-A or its homologs e.g. a "uncharacterized protein YBR089C-A" is increased, preferably an increase of the fine chemical and of tryglycerides, lipids, oils and/or fats containing linoleic acid is conferred.
In one embodiment, in case the activity of the Saccharomyces cerevisiae protein YDR447C or its homologs, e.g. a ribosomal protein 51 (rp51) of the small (40s) subunit is increased, preferably an increase of the fine chemical and of tryglycerides, lipids, oils and/or fats containing linoleic acid is conferred.
In one embodiment, in case the activity of the Saccharomyces cerevisiae protein YOR024W or its homologs e.g. a "uncharacterized protein YOR024W" is increased, preferably an increase of the fine chemical and of tryglycerides, lipids, oils and/or fats containing linoleic acid is conferred.
In one embodiment, in case the activity of the Escherichia coli K12 protein b0050 or its homologs e.g. a conserved protein potentially involved in protein protein interaction is increased, preferably an increase of the fine chemical and of tryglycerides, lipids, oils and/or fats containing linoleic acid is conferred.
In one embodiment, in case the activity of the Escherichia coli K12 protein b0251 or its homologs e.g. a putative HTH-type transcriptional regulator is increased, preferably an increase of the fine chemical and of tryglycerides, lipids, oils and/or fats containing linoleic acid is conferred.
In one embodiment, in case the activity of the Escherichia coli K12 protein b0255 or its homologs e.g. a CP4-6 prophage; IS911 homolog is increased, preferably an increase of the fine chemical and of tryglycerides, lipids, oils and/or fats containing linoleic acid is conferred.
In one embodiment, in case the activity of the Escherichia coli K12 protein b0577 or its homologs e.g. a putative transport protein is increased, preferably an increase of the fine chemical and of tryglycerides, lipids, oils and/or fats containing linoleic acid is conferred.
In one embodiment, in case the activity of the Escherichia coli K12 protein b0849 or its homologs e.g. a glutaredoxin 1 redox coenzyme for glutathione-dependent ribonucleotide reductase is increased, preferably an increase of the fine chemical and of tryglycerides, lipids, oils and/or fats containing linoleic acid is conferred.
In one embodiment, in case the activity of the Escherichia coli K12 protein b1097 or its homologs e.g. a putative thymidylate kinase is increased, preferably an increase of the fine chemical and of tryglycerides, lipids, oils and/or fats containing linoleic acid is conferred.
In one embodiment, in case the activity of the Escherichia coli K12 protein b1693 or its homologs e.g. a 3-dehydroquinate dehydratase is increased, preferably an increase of the fine chemical and of tryglycerides, lipids, oils and/or fats containing linoleic acid is conferred.
In one embodiment, in case the activity of the Escherichia coli K12 protein b2710 or its homologs e.g. a flavorubredoxin (FIRd) bifunctional NO and O₂ reductase is increased, preferably an increase of the fine chemical and of tryglycerides, lipids, oils and/or fats containing linoleic acid is conferred.
In one embodiment, in case the activity of the Escherichia coli K12 protein b2822 or its homologs e.g. a DNA helicase, ATP-dependent dsDNA/ssDNA exonuclease V subunit, ssDNA endonuclease is increased, preferably an increase of the fine chemical and of tryglycerides, lipids, oils and/or fats containing linoleic acid is conferred.
In one embodiment, in case the activity of the Escherichia coli K12 protein b3064 or its homologs e.g. putative O-sialoglycoprotein endopeptidase, with actin-like ATPase domain is increased, preferably an increase of the fine chemical and of tryglycerides, lipids, oils and/or fats containing linoleic acid is conferred.
In one embodiment, in case the activity of the Escherichia coli K12 protein b3166 or its homologs e.g. a tRNA pseudouridine 5S synthase is increased, preferably an increase of the fine chemical and of tryglycerides, lipids, oils and/or fats containing linoleic acid is conferred.
In one embodiment, in case the activity of the Escherichia coli K12 protein b3457 or its homologs e.g. a high-affinity branched-chain amino acid transport protein (ABC superfamily) is increased, preferably an increase of the fine chemical and of tryglycerides, lipids, oils and/or fats containing linoleic acid is conferred.
In one embodiment, in case the activity of the Escherichia coli K12 protein b3644 or its homologs e.g. an uncharacterized stress-induced protein is increased, preferably an increase of the fine chemical and of tryglycerides, lipids, oils and/or fats containing linoleic acid is conferred.
In one embodiment, in case the activity of the Escherichia coli K12 protein b4129 or its homologs e.g. a lysine tRNA synthetase is increased, preferably an increase of the fine chemical and of tryglycerides, lipids, oils and/or fats containing linoleic acid is conferred.

**[0047.0.0.5] and [0048.0.0.5]** for the disclosure of the paragraphs [0047.0.0.5] and [0048.0.0.5] see paragraphs [0047.0.0.0] and [0048.0.0.0] above.

**[0049.0.5.5]** A protein having an activity conferring an increase in the amount or level of the respective fine chemical preferably has the structure of the polypeptide described herein, in particular a polypeptide comprising a consensus sequence as indicated in Table IV, column 7, lines 63 to 67 and 436 to 449 or of a polypeptide as indicated in Table II, columns 5 or 7, lines 63 to 67 and 436 to 449 or the functional homologues thereof as described herein, or is encoded by the nucleic acid molecule characterized herein or the nucleic acid molecule according to the invention, for example by a nucleic acid molecule as indicated in Table I, columns 5 or 7, lines 63 to 67 and 436 to 449 or its herein described functional homologues and has the herein mentioned activity.

**[0050.0.5.5]** For the purposes of the present invention, the term "linoleic acid" also encompasses the corresponding salts, such as, for example, the potassium or sodium salts of linoleic acid or the salts of linoleic acid with amines such as diethylamine.

**[0051.0.5.5] and [0052.0.0.5]** for the disclosure of the paragraphs [0051.0.5.5] and [0052.0.0.5] see paragraphs [0051.0.0.0] and [0052.0.0.0] above.

**[0053.0.5.5]** In one embodiment, the process of the present invention comprises one or more of the following steps
a) stabilizing a protein conferring the increased expression of a protein encoded by the nucleic acid molecule of the invention or of the polypeptid of the invention or the nucleic acid molecule or the polypeptide used in the method of the invention, e.g. of a polypeptide having an activity of a protein as indicated in Table II, column 3, lines 63 to 67 and 436 to 449 or its homolog's activity, e.g. as indicated in Table II, column 7, lines 63 to 67 and 436 to 449, having herein-mentioned the fine chemical increasing activity;
b) stabilizing a mRNA conferring the increased expression of a protein encoded by the nucleic acid molecule of the invention or the nucleic acid molecule used in the method of the invention, e.g. of a polypeptide having an activity of a protein as indicated in Table II, column 3, lines 63 to 67 and 436 to 449 or its homolog's activity, e.g. as indicated in Table II, column 7, lines 63 to 67 and 436 to 449 or of a mRNA encoding the polypeptide of the present invention having herein mentioned linoleic acid increasing activity;
c) increasing the specific activity of a protein conferring the increased expression of a protein encoded by the nucleic acid molecule of the invention or of the polypeptide of the present invention or the nucleic acid molecule or the polypeptide used in the method of the invention, having herein-mentioned linoleic acid increasing activity, e.g. of a polypeptide having an activity of a protein as indicated in Table II, column 3, lines 63 to 67 and 436 to 449 or its homolog's activity, e.g. as indicated in Table II, column 7, lines 63 to 67 and 436 to 449, or decreasing the inhibitory regulation of the polypeptide of the invention or of the polypeptide used in the method of the invention;
d) generating or increasing the expression of an endogenous or artificial transcription factor mediating the expression of a protein conferring the increased expression of a protein encoded by the nucleic acid molecule of the invention or the nucleic acid molecule used in the method of the invention or of the polypeptide of the invention or the polypeptide used in the method of the invention having herein-mentioned linoleic acid increasing activity, e.g. of a polypeptide having an activity of a protein as indicated in Table II, column 3, lines 63 to 67 and 436 to 449 or its homolog's activity, e.g. as indicated in Table II, column 7, lines 63 to 67 and 436 to 449;
e) stimulating activity of a protein conferring the increased expression of a protein encoded by the nucleic acid molecule of the present invention or a polypeptide of the present invention having herein-mentioned linoleic acid increasing activity, e.g. of a polypeptide having an activity of a protein as indicated in Table II, column 3, lines 63 to 67 and 436 to 449 or its homolog's activity, e.g. as indicated in Table II, column 7, lines 63 to 67 and 436 to 449, by adding one or more exogenous inducing factors to the organism or parts thereof;
f) expressing a transgenic gene encoding a protein conferring the increased expression of a polypeptide encoded by the nucleic acid molecule of the present invention or a polypeptide of the present invention, having herein-mentioned linoleic acid increasing activity, e.g. of a polypeptide having an activity of a protein as indicated in Table II, column 3, lines 63 to 67 and 436 to 449 or its homolog's activity, e.g. as indicated in Table II, column 7, lines 63 to 67 and 436 to 449,
g) increasing the copy number of a gene conferring the increased expression of a nucleic acid molecule encoding a polypeptide encoded by the nucleic acid molecule of the invention or the nucleic acid molecule used in the method of the invention or the polypeptide of the invention or the polypeptide used in the method of the invention having herein-mentioned linoleic acid increasing activity, e.g. of a polypeptide having an activity of a protein as indicated in Table II, column 3, lines 63 to 67 and 436 to 449 or its homolog's activity, e.g. as indicated in Table II, column 7, lines 63 to 67 and 436 to 449,
h) increasing the expression of the endogenous gene encoding the polypeptide of the invention or the polypeptide used in the method of the invention, e.g. a polypeptide having an activity of a protein as indicated in Table II, column 3, lines 63 to 67 and 436 to 449 or its homolog's activity, e.g. as indicated in Table II, column 7, lines 63 to 67 and 436 to 449, by adding positive expression or removing negative expression elements, e.g. homologous recombination can be used to either introduce positive regulatory elements like for plants the 35S enhancer into the promoter or to remove repressor elements form regulatory regions. Further gene conversion methods can be used to disrupt repressor elements or to enhance to acitivty of positive elements. Positive elements can be randomly introduced in plants by T-DNA or transposon mutagenesis and lines can be identified in which the positive elements have be integrated near to a gene of the invention, the expression of which is thereby enhanced;
i) Modulating growth conditions of an organism in such a manner, that the expression or activity of the gene encoding the protein of the invention or the protein itself is enhanced for example microorganisms or plants can be grown for example under a higher temperature regime leading to an enhanced expression of heat shock proteins, which can lead an enhanced fine chemical production.
j) selecting of organisms with especially high activity of the proteins of the invention from natural or from mutagenized resources and breeding them into the target organisms, eg the elite crops.

**[0054.0.5.5]** Preferably, said mRNA is the nucleic acid molecule of the present invention and/or the protein conferring the increased expression of a protein encoded by the nucleic acid molecule of the present invention or the polypeptide having the herein mentioned activity is the polypeptide of the present invention, e.g. conferring the increase of linoleic acid after increasing the expression or activity of the encoded polypeptide or having the activity of a polypeptide having an activity of a protein as indicated in Table II, column 3, lines 63 to 67 and 436 to 449 or its homolog's activity, e.g. as indicated in Table II, columns 5 or 7, lines 63 to 67 and 436 to 449.

**[0055.0.0.5] to [0067.0.0.5]** for the disclosure of the paragraphs [0055.0.0.5] to [0067.0.0.5] see paragraphs [0055.0.0.0] to [0067.0.0.0] above.

**[0068.0.5.5] and [0069.0.5.5]** for the disclosure of the paragraphs [0068.0.5.5] and [0069.0.5.5] see paragraphs [0068.0.0.0] and [0069.0.0.0] above.

**[0070.0.5.5]** Owing to the introduction of a gene or a plurality of genes conferring the expression of the nucleic acid molecule of the invention or the nucleic acid molecule used in the method of the invention or the polypeptide of the invention or the polypeptide used in the method of the invention as described below, for example the nucleic acid construct mentioned below into an organism alone or in combination with other genes, it is possible not only to increase the biosynthetic flux towards the end product, but also to increase, modify or create *de novo* an advantageous, preferably novel metabolites composition in the organism, e.g. an advantageous fatty acid composition comprising a higher content of (from a viewpoint of nutrional physiology limited) fatty acids, like palmitate, palmitoleate, stearate and/or oleate.

**[0071.0.5.5]** for the disclosure of this paragraph see [0071.0.0.0] above.

**[0072.0.5.5]** By influencing the metabolism thus, it is possible to produce, in the process according to the invention, further advantageous compounds. Examples of such compounds are, in addition to linoleic acid, triglycerides, lipids, oils and/or fats containing linoleic acid compounds such as palmitate, palmitoleate, stearate and/or oleate.

**[0073.0.5.5]** Accordingly, in one embodiment, the process according to the invention relates to a process, which comprises:
a) providing a non-human organism, preferably a microorganism, a non-human animal, a plant or animal cell, a plant or animal tissue or a plant;
b) increasing the activity of a protein having the activity of a polypeptide of the invention or the polypeptide used in the method of the invention or a homolog thereof, e.g. as shown in Table II, columns 5 or 7, lines 63 to 67 and 436 to 449 or of a polypeptide being encoded by the nucleic acid molecule of the present invention and described below, i.e. conferring an increase of the respective fine chemical in the organism, preferably a microorganism, the a non-human animal, a plant or animal cell, a plant or animal tissue or the plant,
c) growing the organism, preferably a microorganism, a non-human animal, a plant or animal cell, a plant or animal tissue or the plant under conditions which permit the production of the fine chemical in the organism, preferably a microorganism, a plant cell, a plant tissue or the plant; and
d) if desired, revovering, optionally isolating, the free and/or bound the respective fine chemical and, optionally further free and/or bound fatty acids synthetized by the organism, the microorganism, the non-human animal, the plant or animal cell, the plant or animal tissue or the plant.

**[0074.0.5.5]** The organism, in particular the microorganism, non-human animal, the plant or animal cell, the plant or animal tissue or the plant is advantageously grown in such a way that it is not only possible to recover, if desired isolate the free or bound the fine chemical or the free and bound the fine chemical but as option it is also possible to produce, recover and, if desired isolate, other free or/and bound fatty acids, in particular oleic acid.

**[0075.0.0.5] to [0084.0.0.5]** for the disclosure of the paragraphs [0075.0.0.5] to [0084.0.0.5] see paragraphs [0075.0.0.0] to [0084.0.0.0] above.

**[0085.0.5.5]** With regard to the nucleic acid sequence as depicted a nucleic acid construct which contains a nucleic acid sequence mentioned herein or an organism (= transgenic organism) which is transformed with said nucleic acid sequence or said nucleic acid construct, "transgene" means all those constructs which have been brought about by genetic manipulation methods, preferably in which either
a) the nucleic acid sequence as depicted and shown in Table I, columns 5 or 7, lines 63 to 67 and 436 to 449 or a derivative thereof, or
b) a genetic regulatory element, for example a promoter, which is functionally linked to the nucleic acid sequence as depicted and shown in Table I, columns 5 or 7, lines 63 to 67 and 436 to 449 or a derivative thereof, or c) (a) and (b)
is/are not present in its/their natural genetic environment or has/have been modified by means of genetic manipulation methods, it being possible for the modification to be, by way of example, a substitution, addition, deletion, inversion or insertion of one or more nucleotide. "Natural genetic environment" means the natural chromosomal locus in the organism of origin or the presence in a genomic library. In the case of a genomic library, the natural, genetic environment of the nucleic acid sequence is preferably at least partially still preserved. The environment flanks the nucleic acid sequence at least on one side and has a sequence length of at least 50 bp, preferably at least 500 bp, particularly preferably at least 1000 bp, very particularly preferably at least 5000 bp.

**[0086.0.0.5] and [0087.0.0.5]** for the disclosure of the paragraphs [0086.0.0.5] and [0087.0.0.5] see paragraphs [0086.0.0.0] and [0087.0.0.0] above.

**[0088.0.5.5]** In an advantageous embodiment of the invention, the organism takes the form of a plant whose fatty acid content is modified advantageously owing to the nucleic acid molecule of the present invention expressed. This is important for plant breeders since, for example, the nutritional value of plants for poultry is dependent on the abovementioned essential fatty acids and the general amount of fatty acids as energy source in feed. After the activity of a protein as shown in Table II, columns 5 or 7, lines 63 to 67 and 436 to 449 has been increased or generated, or after the expression of nucleic acid molecule or polypeptide according to the invention has been generated or increased, the transgenic plant generated thus is grown on or in a nutrient medium or else in the soil and subsequently harvested.

**[0088.1.0.5], [0089.0.0.5], [0090.0.0.5] and [0091.0.5.5]** for the disclosure of the paragraphs [0088.1.0.5], [0089.0.0.5], [0090.0.0.5] and [0091.0.5.5] see paragraphs [0088.1.0.0], [0089.0.0.0], [0090.0.0.0] and [0091.0.0.0] above.

**[0092.0.0.5] to [0094.0.0.5]** for the disclosure of the paragraphs [0092.0.0.5] to [0094.0.0.5] see paragraphs [0092.0.0.0] to [0094.0.0.0] above.

**[0095.0.5.5]** It may be advantageous to increase the pool of free fatty acids in the transgenic organisms by the process according to the invention in order to isolate high amounts of the pure fine chemical.

**[0096.0.5.5]** In another preferred embodiment of the invention a combination of the increased expression of the nucleic acid sequence or the protein of the invention together with the transformation of a protein or polypeptid for example a fatty acid transporter protein or a compound, which functions as a sink for the desired fatty acid for example for linoleic or linolenic acid in the organism is useful to increase the production of the respective fine chemical (see Bao and Ohlrogge, Plant Physiol. 1999 August; 120 (4): 1057-1062). Such fatty acid transporter protein may serve as a link between the location of fatty acid synthesis and the socalled sink tissue, in which fatty acids, triglycerides, oils and fats are stored.

**[0097.0.5.5]** for the disclosure of this paragraph see [0097.0.0.0].

**[0098.0.5.5]** In a preferred embodiment, the fine chemical (linoleic acid) is produced in accordance with the invention and, if desired, is isolated. The production of further fatty acids such as palmitic acid, stearic acid, palmitoleic acid and/or oleic acid mixtures thereof or mixtures of other fatty acids by the process according to the invention is advantageous.

**[0099.0.5.5]** In the case of the fermentation of microorganisms, the abovementioned fatty acids may accumulate in the medium and/or the cells. If microorganisms are used in the process according to the invention, the fermentation broth can be processed after the cultivation. Depending on the requirement, all or some of the biomass can be removed from the fermentation broth by separation methods such as, for example, centrifugation, filtration, decanting or a combination of these methods, or else the biomass can be left in the fermentation broth. The fermentation broth can subsequently be reduced, or concentrated, with the aid of known methods such as, for example, rotary evaporator, thin-layer evaporator, falling film evaporator, by reverse osmosis or by nanofiltration. Afterwards advantageously further compounds for formulation can be added such as corn starch or silicates. This concentrated fermentation broth advantageously together with compounds for the formulation can subsequently be processed by lyophilization, spray drying, spray granulation or by other methods. Preferably the fatty acids or the fatty acid compositions are isolated from the organisms, such as the microorganisms or plants or the culture medium in or on which the organisms have been grown, or from the organism and the culture medium, in the known manner, for example via extraction, distillation, crystallization, chromatography or a combination of these methods. These purification methods can be used alone or in combination with the aforementioned methods such as the separation and/or concentration methods.

**[0100.0.5.5]** Transgenic plants which comprise the fatty acids such as saturated or polyunsaturated fatty acids synthesized in the process according to the invention can advantageously be marketed directly without there being any need for the oils, lipids or fatty acids synthesized to be isolated. Plants for the process according to the invention are listed as meaning intact plants and all plant parts, plant organs or plant parts such as leaf, stem, seeds, root, tubers, anthers, fibers, root hairs, stalks, embryos, calli, cotelydons, petioles, harvested material, plant tissue, reproductive tissue and cell cultures which are derived from the actual transgenic plant and/or can be used for bringing about the transgenic plant. In this context, the seed comprises all parts of the seed such as the seed coats, epidermal cells, seed cells, endosperm or embryonic tissue. However, the fine chemical produced in the process according to the invention can also be isolated from the organisms, advantageously plants, in the form of their oils, fats, lipids and/or free fatty acids. Fatty acids produced by this process can be obtained by harvesting the organisms, either from the crop in which they grow, or from the field. This can be done via pressing or extraction of the plant parts, preferably the plant seeds. To increase the efficiency of oil extraction it is beneficial to clean, to temper and if necessary to hull and to flake the plant material especially the seeds. In this context, the oils, fats, lipids and/or free fatty acids can be obtained by what is known as cold beating or cold pressing without applying heat. To allow for greater ease of disruption of the plant parts, specifically the seeds, they are previously comminuted, steamed or roasted. The seeds, which have been pretreated in this manner can subsequently be pressed or extracted with solvents such as warm hexane. The solvent is subsequently removed. In the case of microorganisms, the latter are, after harvesting, for example extracted directly without further processing steps or else, after disruption, extracted via various methods with which the skilled worker is familiar. In this manner, more than 96% of the compounds produced in the process can be isolated. Thereafter, the resulting products are processed further, i.e. degummed and/or refined. In this process, substances such as the plant mucilages and suspended matter are first removed. What is known as desliming can be affected enzymatically or, for example, chemico-physically by addition of acid such as phosphoric acid. Thereafter otionally, the free fatty acids are removed by treatment with a base like alkali, for example aqueous KOH or NaOH, or acid hydrolysis, advantageously in the presence of an alcohol such as methanol or ethanol, or via enzymatic cleavage, and isolated via, for example, phase separation and subsequent acidification via, for example, H₂SO₄. The fatty acids can also be liberated directly without the above-described processing step. If desired the resulting product can be washed thoroughly with water to remove traces of soap and the alkali remaining in the product and then dried. To remove the pigment remaining in the product, the products can be subjected to bleaching, for example using filler's earth or active charcoal. At the end, the product can be deodorized, for example using steam distillation under vacuum. These chemically pure fatty acids or fatty acid compositions are advantageous for applications in the food industry sector, the cosmetic sector and especially the pharmacological industry sector.

**[0101.0.5.5]** for the disclosure of this paragraph see [0101.0.0.0].

**[0102.0.5.5]** Fatty acids can for example be detected advantageously via GC separation methods. The unambiguous detection for the presence of fatty acid products can be obtained by analyzing recombinant organisms using analytical standard methods: GC, GC-MS or TLC, as described on several occasions by Christie and the references therein (1997, in: Advances on Lipid Methodology, Fourth Edition: Christie, Oily Press, Dundee, 119-169; 1998, Gaschromatographie-Massenspektrometrie-Verfahren [Gas chromatography/mass spectrometric methods], Lipide 33:343-353). One example is the analysis of fatty acids via FAME and GC-MS or TLC (abbreviations: FAME, fatty acid methyl ester; GC-MS, gas liquid chromatography/mass spectrometry; TLC, thin-layer chromatography. The material to be analyzed can be disrupted by sonication, grinding in a glass mill, liquid nitrogen and grinding or via other applicable methods. After disruption, the material must be centrifuged. The sediment is resuspended in distilled water, heated for 10 minutes at 100°C, cooled on ice and recentrifuged, followed by extraction for one hour at 90°C in 0.5 M sulfuric acid in methanol with 2% dimethoxypropane, which leads to hydrolyzed oil and lipid compounds, which give transmethylated lipids. These fatty acid methyl esters are extracted in petroleum ether and finally subjected to a GC analysis using a capillary column (Chrompack, WCOT Fused Silica, CP-Wax-52 CB, 25 µm, 0.32 mm) at a temperature gradient of between 170°C and 240°C for 20 minutes and 5 minutes at 240°C. The identity of the resulting fatty acid methyl esters must be defined using standards which are available from commercial sources (i.e. Sigma).

**[0103.0.5.5]** In a preferred embodiment, the present invention relates to a process for the production of the respective fine chemical comprising or generating in an organism or a part thereof the expression of at least one nucleic acid molecule comprising a nucleic acid molecule selected from the group consisting of:
a) nucleic acid molecule encoding, preferably at least the mature form, of a polypeptide having a sequence as shown in Table II, columns 5 or 7, lines 63 to 67 and 436 to 449 in or a fragment thereof, which confers an increase in the amount of the respective fine chemical in an organism or a part thereof;
b) nucleic acid molecule comprising, preferably at least the mature form, of the nucleic acid molecule having a sequence as shown in Table I, columns 5 or 7, lines 63 to 67 and 436 to 449,
c) nucleic acid molecule whose sequence can be deduced from a polypeptide sequence encoded by a nucleic acid molecule of (a) or (b) as result of the degeneracy of the genetic code and conferring an increase in the amount of the respective fine chemical in an organism or a part thereof;
d) nucleic acid molecule encoding a polypeptide which has at least 50% identity with the amino acid sequence of the polypeptide encoded by the nucleic acid molecule of (a) to (c) and conferring an increase in the amount of the respective fine chemical in an organism or a part thereof;
e) nucleic acid molecule which hybidizes with a nucleic acid molecule of (a) to (c) under under stringent hybridisation conditions and conferring an increase in the amount of the respective fine chemical in an organism or a part thereof;
f) nucleic acid molecule encoding a polypeptide, the polypeptide being derived by substituting, deleting and/or adding one or more amino acids of the amino acid sequence of the polypeptide encoded by the nucleic acid molecules (a) to (d), preferably to (a) to (c) and conferring an increase in the amount of the respective fine chemical in an organism or a part thereof;
g) nucleic acid molecule encoding a fragment or an epitope of a polypeptide which is encoded by one of the nucleic acid molecules of (a) to (e), preferably of (a) to (c) and and conferring an increase in the amount of the respective fine chemical in an organism or a part thereof;
h) nucleic acid molecule comprising a nucleic acid molecule which is obtained by amplifying nucleic acid molecules from a cDNA library or a genomic library using the primer pairs having a sequence as shown in Table III, column 7, lines 63 to 67 and 436 to 449 and conferring an increase in the amount of the respective fine chemical in an organism or a part thereof;
i) nucleic acid molecule encoding a polypeptide which is isolated, e.g. from an expression library, with the aid of monoclonal antibodies against a polypeptide encoded by one of the nucleic acid molecules of (a) to (h), preferably of (a) to (c), and and conferring an increase in the amount of the respective fine chemical in an organism or a part thereof;
j) nucleic acid molecule which encodes a polypeptide comprising the consensus sequence having a sequence as shown in Table IV, column 7, lines 63 to 67 and 436 to 449 and conferring an increase in the amount of the respective fine chemical in an organism or a part thereof ;
k) nucleic acid molecule comprising one or more of the nucleic acid molecule encoding the amino acid sequence of a polypeptide encoding a domain of a polypeptide as shown in Table II, columns 5 or 7, lines 63 to 67 and 436 to 449 and conferring an increase in the amount of the respective fine chemical in an organism or a part thereof; and
l) nucleic acid molecule which is obtainable by screening a suitable library under stringent conditions with a probe comprising one of the sequences of the nucleic acid molecule of (a) to (k), preferably of (a) to (c), or with a fragment of at least 15 nt, preferably 20 nt, 30 nt, 50 nt, 100 nt, 200 nt or 500 nt of the nucleic acid molecule characterized in (a) to (k), preferably to (a) to (c), and conferring an increase in the amount of the fine chemical in an organism or a part thereof;
or which comprises a sequence which is complementary thereto.

**[0103.1.0.5] and [0103.2.0.5]** for the disclosure of the paragraphs [0103.1.0.5] and [0103.2.0.5] see paragraphs [0103.1.0.0] and [0103.2.0.0] above.

**[0104.0.5.5]** In one embodiment, the nucleic acid molecule used in the process distinguishes over the sequence as shown in Table I, columns 5 or 7, lines 63 to 67 and 436 to 449, preferably over the sequences as shown in Table IA, columns 5 or 7, lines 63 to 67 and 436 to 449. In one embodiment, the nucleic acid molecule of the present invention is less than 100%, 99,999%, 99,99%, 99,9% or 99% identical to the sequences shown in Table I, columns 5 or 7, lines 63 to 67 and 436 to 449, preferably over the sequences as shown in Table IA, columns 5 or 7, lines 63 to 67 and 436 to 449. In another embodiment, the nucleic acid molecule does not encode a polypeptide of the sequence shown in Table II, columns 5 or 7, lines 63 to 67 and 436 to 449, preferably over the sequences as shown in Table IA, columns 5 or 7, lines 63 to 67 and 436 to 449.

**[0105.0.0.5] to [0107.0.0.5]** for the disclosure of the paragraphs [0105.0.0.5] to [0107.0.0.5] see paragraphs [0105.0.0.0] and [0107.0.0.0] above.

**[0108.0.5.5]** Nucleic acid molecules with the sequence shown in Table I, columns 5 or 7, lines 63 to 67 and 436 to 449, nucleic acid molecules which are derived from the amino acid sequences shown in Table II, columns 5 or 7, lines 63 to 67 and 436 to 449 or from polypeptides comprising the consensus sequence shown in Table IV, column 7, lines 63 to 67 and 436 to 449, or their derivatives or homologues encoding polypeptides with the enzymatic or biological activity of a protein as shown in Table II, columns 5 or 7, lines 63 to 67 and 436 to 449 or e.g. conferring a linoleic acid increase after increasing its expression or activity are advantageously increased in the process according to the invention.

**[0109.0.5.5]** for the disclosure of this paragraph see [0109.0.0.0] above.

**[0110.0.5.5]** Nucleic acid molecules, which are advantageous for the process according to the invention and which encode polypeptides with an activity of a polypeptide used in the method of the invention or used in the process of the invention, e.g. of a protein as shown in Table II, columns 5 or 7, lines 63 to 67 and 436 to 449 or being encoded by a nucleic acid molecule indicated in Table I, columns 5 or 7, lines 63 to 67 and 436 to 449 or of its homologs, e.g. as indicated in Table II, columns 5 or 7, lines 63 to 67 and 436 to 449 can be determined from generally accessible databases.

**[0111.0.0.5]** for the disclosure of this paragraph see [0111.0.0.0] above.

**[0112.0.5.5]** The nucleic acid molecules used in the process according to the invention take the form of isolated nucleic acid sequences, which encode polypeptides with an activity of a polypeptide as indicated in Table II, column3, lines lines 63 to 67 and 436 to 449 or having the sequence of a polypeptide as indicated in Table II, columns 5 and 7, lines 63 to 67 and 436 to 449 and conferring an increase of the respective fine chemical.

**[0113.0.0.5] to [0120.0.0.5]** for the disclosure of the paragraphs [0113.0.0.5] to [0120.0.0.5] see paragraphs [0113.0.0.0] and [0120.0.0.0] above.

**[0121.0.5.5]** However, it is also possible to use artificial sequences, which differ in one or more bases from the nucleic acid sequences found in organisms, or in one or more amino acid molecules from polypeptide sequences found in organisms, in particular from the polypeptide sequences shown in Table II, columns 5 or 7, lines 63 to 67 and 436 to 449 or the functional homologues thereof as described herein, preferably conferring above-mentioned activity, i.e. conferring an increase of the respective fine chemical after increasing its activity.

**[0122.0.0.5] to [0127.0.0.5]** for the disclosure of the paragraphs [0122.0.0.5] to [0127.0.0.5] see paragraphs [0122.0.0.0] and [0127.0.0.0] above.

**[0128.0.5.5]** Synthetic oligonucleotide primers for the amplification, e.g. as shown in Table III, column 7, lines 63 to 67 and 436 to 449, by means of polymerase chain reaction can be generated on the basis of a sequence shown herein, for example the sequence shown in Table I, columns 5 or 7, lines 63 to 67 and 436 to 449 or the sequences as shown in Table II, columns 5 or 7, lines 63 to 67 and 436 to 449. **[0129.0.5.5]** Moreover, it is possible to identify conserved regions from various organisms by carrying out protein sequence alignments with the polypeptide used in the process of the invention, in particular with sequences of the polypeptide of the invention or the polypeptide used in the method of the invention, from which conserved regions, and in turn, degenerate primers can be derived. Conserved regions are those, which show a very little variation in the amino acid in one particular position of several homologs from different origin. The consenus sequence shown in Table IV, column 7, lines 63 to 67 and 436 to 449 is derived from said aligments and represent such conserved regions.

**[0130.0.5.5]** for the disclosure of this paragraph see [0130.0.0.0].

**[0131.0.0.5] to [0138.0.0.5]** for the disclosure of the paragraphs [0131.0.0.5] to [0138.0.0.5] see paragraphs [0131.0.0.0] to [0138.0.0.0] above.

**[0139.0.5.5]** Polypeptides having above-mentioned activity, i.e. conferring the respective fine chemical increase, derived from other organisms, can be encoded by other DNA sequences which hybridize to a sequences indicated in Table I, columns 5 or 7, lines 63 to 67 and 436 to 449, preferably of Table I B, columns 5 or 7, lines 63 to 67 and 436 to 449 under relaxed hybridization conditions and which code on expression for peptides having the linoleic asid increasing activity.

**[0140.0.0.5] to [0146.0.0.5]** for the disclosure of the paragraphs [0140.0.0.5] to [0146.0.0.5] see paragraphs [0140.0.0.0] and [0146.0.0.0] above.

**[0147.0.5.5]** Further, the nucleic acid molecule of the invention comprises a nucleic acid molecule, which is a complement of one of the nucleotide sequences of above mentioned nucleic acid molecules or a portion thereof. A nucleic acid molecule which is complementary to one of the nucleotide sequences indicated in Table I, columns 5 or 7, lines 63 to 67 and 436 to 449, preferably in Table I B, columns 5 or 7, lines 63 to 67 and 436 to 449 is one which is sufficiently complementary to one of said nucleotide sequences such that it can hybridize to one of said nucleotide sequences thereby forming a stable duplex. Preferably, the hybridisation is performed under stringent hybrization conditions. However, a complement of one of the herein disclosed sequences is preferably a sequence complement thereto according to the base pairing of nucleic acid molecules well known to the skilled person. For example, the bases A and G undergo base pairing with the bases T and U or C, resp. and visa versa. Modifications of the bases can influence the base-pairing partner.

**[0148.0.5.5]** The nucleic acid molecule of the invention comprises a nucleotide sequence which is at least about 30%, 35%, 40% or 45%, preferably at least about 50%, 55%, 60% or 65%, more preferably at least about 70%, 80%, or 90%, and even more preferably at least about 95%, 97%, 98%, 99% or more homologous to a nucleotide sequence indicated in Table I, columns 5 or 7, lines 63 to 67 and 436 to 449, preferably of Table I B, columns 5 or 7, lines 63 to 67 and 436 to 449, or a functional portion thereof and preferably has above mentioned activity, in particular has the-fine-chemical-increasing activity after increasing its activity or an activity of a product of a gene encoding said sequence or its homologs.

**[0149.0.5.5]** The nucleic acid molecule of the invention or the nucleic acid molecule used in the method of the invention comprises a nucleotide sequence which hybridizes, preferably hybridizes under stringent conditions as defined herein, to one of the nucleotide sequences indicated in Table I, columns 5 or 7, lines 63 to 67 and 436 to 449, preferably of Table I B, columns 5 or 7, lines 63 to 67 and 436 to 449 or a portion thereof and encodes a protein having above-mentioned activity and as indicated in indicated in Table II, columns 5 or 7, lines 63 to 67 and 436 to 449, preferably of Table II B, columns 5 or 7, lines 63 to 67 and 436 to 449, e.g. conferring an increase of the respective fine chemical.

**[00149.1.0.5]** Optionally, the nucleotide sequence, which hybridises to one of the nucleotide sequences indicated in Table I, columns 5 or 7, lines 63 to 67 and 436 to 449, preferably of Table I B, columns 5 or 7, lines 63 to 67 and 436 to 449 has further one or more of the activities annotated or known for the a protein as indicated in Table II, column 3, lines 63 to 67 and 436 to 449, preferably of Table II B, columns 3, lines 63 to 67 and 436 to 449.

**[0150.0.5.5]** Moreover, the nucleic acid molecule of the invention or used in the process of the invention can comprise only a portion of the coding region of one of the sequences indicated in Table I, columns 5 or 7, lines 63 to 67 and 436 to 449, preferably of Table I B, columns 5 or 7, lines 63 to 67 and 436 to 449, for example a fragment which can be used as a probe or primer or a fragment encoding a biologically active portion of the polypeptide of the present invention or of a polypeptide used in the process of the present invention, i.e. having above-mentioned activity, e.g.conferring an increase of linoleic acid if its activity is increased. The nucleotide sequences determined from the cloning of the present protein-according-to-the-invention-encoding gene allows for the generation of probes and primers designed for use in identifying and/or cloning its homologues in other cell types and organisms. The probe/primer typically comprises substantially purified oligonucleotide. The oligonucleotide typically comprises a region of nucleotide sequence that hybridizes under stringent conditions to at least about 12, 15 preferably about 20 or 25, more preferably about 40, 50 or 75 consecutive nucleotides of a sense strand of one of the sequences indicated in Table I, columns 5 or 7, lines 63 to 67 and 436 to 449, an anti-sense sequence of one of the sequences indicated in Table I, columns 5 or 7, lines 63 to 67 and 436 to 449, or naturally occurring mutants thereof. Primers based on a nucleotide sequence of the invention can be used in PCR reactions to clone homologues of the polypeptide of the invention or of the polypeptide used in the process of the invention, e.g. as the primers described in the examples of the present invention, e.g. as shown in the examples. A PCR with the primer pairs indicated in Table III, column 7, lines 63 to 67 and 436 to 449 will result in a fragment of a polynucleotide sequence as indicated in Table I, columns 5 or 7, lines 63 to 67 and 436 to 449. Preferred is Table I B, column 7, lines 63 to 67 and 436 to 449.

**[0151.0.0.5]** for the disclosure of this paragraph see [0151.0.0.0] above.

**[0152.0.5.5]** The nucleic acid molecule of the invention encodes a polypeptide or portion thereof which includes an amino acid sequence which is sufficiently homologous to the amino acid sequence as shown in Table II, columns 5 or 7, lines 63 to 67 and 436 to 449 such that the protein or portion thereof maintains the ability to participate in the respective fine chemical production, in particular a linoleic acid increasing activity as mentioned above or as described in the examples in plants or microorganisms is comprised.

**[0153.0.5.5]** As used herein, the language "sufficiently homologous" refers to proteins or portions thereof which have amino acid sequences which include a minimum number of identical or equivalent amino acid residues (e.g., an amino acid residue which has a similar side chain as an amino acid residue in one of the sequences of the polypeptide of the present invention) to an amino acid sequence shown in Table II, columns 5 or 7, lines 63 to 67 and 436 to 449 such that the protein or portion thereof is able to participate in the increase of the respective fine chemical production. In one embodiment, a protein or portion thereof as shown in Table II, columns 5 or 7, lines 63 to 67 and 436 to 449 has for example an activity of a polypeptide as indicated in Table II, columns 5 or 7, lines 63 to 67 and 436 to 449 are described herein.

**[0154.0.5.5]** In one embodiment, the nucleic acid molecule of the present invention comprises a nucleic acid that encodes a portion of the protein of the present invention. The protein is at least about 30%, 35%, 40%, 45% or 50%, preferably at least about 55%, 60%, 65% or 70%, and more preferably at least about 75%, 80%, 85%, 90%, 91%, 92%, 93% or 94% and most preferably at least about 95%, 97%, 98%, 99% or more homologous to an entire amino acid sequence as shown in Table II, columns 5 or 7, lines 63 to 67 and 436 to 449 and having above-mentioned activity, e.g.conferring preferably the increase of the respective fine chemical.

**[0155.0.0.5] and [0156.0.0.5]** for the disclosure of the paragraphs [0155.0.0.5] and [0156.0.0.5] see paragraphs [0155.0.0.0] and [0156.0.0.0] above.

**[0157.0.5.5]** The invention further relates to nucleic acid molecules that differ from one of the nucleotide sequences indicated in Table I, columns 5 or 7, lines 63 to 67 and 436 to 449 (and portions thereof) due to degeneracy of the genetic code and thus encode a polypeptide of the present invention, in particular a polypeptide having above mentioned activity, e.g. conferring an increase in the respective fine chemical in a organism, e.g. as that polypeptides comprising the consensus sequences as indicated in Table IV, column 7, lines 63 to 67 and 436 to 449 or of the polypeptide as indicated in Table II, columns 5 or 7, lines 63 to 67 and 436 to 449 or their functional homologues. Advantageously, the nucleic acid molecule of the invention or the nucleic acid molecule used in the method of the invention comprises, or in an other embodiment has, a nucleotide sequence encoding a protein comprising, or in an other embodiment having, a consensus sequences as indicated in Table IV, column 7, lines 63 to 67 and 436 to 449 or of the polypeptide as indicated in Table II, columns 5 or 7, lines 63 to 67 and 436 to 449 or the functional homologues. In a still further embodiment, the nucleic acid molecule of the invention or the nucleic acid molecule used in the method of the invention encodes a full length protein which is substantially homologous to an amino acid sequence comprising a consensus sequence as indicated in Table IV, columns 5 or 7, lines 63 to 67 and 436 to 449 or of a polypeptide as indicated in Table II, columns 5 or 7, lines 63 to 67 and 436 to 449 or the functional homologues thereof. However, in a preferred embodiment, the nucleic acid molecule of the present invention does not consist of a sequence as indicated in Table I, columns 5 or 7, lines 63 to 67 and 436 to 449, preferably as indicated in Table I A, columns 5 or 7, lines 63 to 67 and 436 to 449. Preferably the nucleic acid molecule of the invention is a functional homologue or identical to a nucleic acid molecule indicated in Table I B, columns 5 or 7, lines 63 to 67 and 436 to 449.

**[0158.0.0.5] to [0160.0.0.5]** for the disclosure of the paragraphs [0158.0.0.5] to [0160.0.0.5] see paragraphs [0158.0.0.0] to [0160.0.0.0] above.

**[0161.0.5.5]** Accordingly, in another embodiment, a nucleic acid molecule of the invention or the nucleic acid molecule used in the method of the invention is at least 15, 20, 25 or 30 nucleotides in length. Preferably, it hybridizes under stringent conditions to a nucleic acid molecule comprising a nucleotide sequence of the nucleic acid molecule of the present invention or used in the process of the present invention, e.g. comprising the sequence shown in Table I, columns 5 or 7, lines 63 to 67 and 436 to 449. The nucleic acid molecule is preferably at least 20, 30, 50, 100, 250 or more nucleotides in length.

**[0162.0.0.5]** for the disclosure of this paragraph see paragraph [0162.0.0.0] above.

**[0163.0.5.5]** Preferably, nucleic acid molecule of the invention that hybridizes under stringent conditions to a sequence as indicated in Table I, columns 5 or 7, lines 63 to 67 and 436 to 449 corresponds to a naturally-occurring nucleic acid molecule of the invention. As used herein, a "naturally-occurring" nucleic acid molecule refers to an RNA or DNA molecule having a nucleotide sequence that occurs in nature (e.g., encodes a natural protein). Preferably, the nucleic acid molecule encodes a natural protein having above-mentioned activity, e.g. conferring the respective fine chemical increase after increasing the expression or activity thereof or the activity of a protein of the invention or used in the process of the invention.

**[0164.0.0.5]** for the disclosure of this paragraph see paragraph [0164.0.0.0] above.

**[0165.0.5.5]** For example, nucleotide substitutions leading to amino acid substitutions at "non-essential" amino acid residues can be made in a sequence of the nucleic acid molecule of the invention or used in the process of the invention, e.g. as shown in Table I, columns 5 or 7, lines 63 to 67 and 436 to 449.

**[0166.0.0.5] and [0167.0.0.5]** for the disclosure of the paragraphs [0166.0.0.5] and [0167.0.0.5] see paragraphs [0166.0.0.0] and [0167.0.0.0] above.

**[0168.0.5.5]** Accordingly, the invention relates to nucleic acid molecules encoding a polypeptide having above-mentioned activity, e.g. conferring an increase in the respective fine chemical in an organism or parts thereof that contain changes in amino acid residues that are not essential for said activity. Such polypeptides differ in amino acid sequence from a sequence contained in a sequence as indicated in Table II, columns 5 or 7, lines 63 to 67 and 436 to 449 yet retain said activity described herein. The nucleic acid molecule can comprise a nucleotide sequence encoding a polypeptide, wherein the polypeptide comprises an amino acid sequence at least about 50% identical to an amino acid sequence as indicated in Table II, columns 5 or 7, lines 63 to 67 and 436 to 449 and is capable of participation in the increase of production of the respective fine chemical after increasing its activity, e.g. its expression. Preferably, the protein encoded by the nucleic acid molecule is at least about 60% identical to the sequence as indicated in Table II, columns 5 or 7, lines 63 to 67 and 436 to 449, more preferably at least about 70% identical to one of the sequences as indicated in Table II, columns 5 or 7, lines 63 to 67 and 436 to 449, even more preferably at least about 80%, 90%, 95% homologous to the sequence as indicated in Table II, columns 5 or 7, lines 63 to 67 and 436 to 449, and most preferably at least about 96%, 97%, 98%, or 99% identical to the sequence as indicated in Table II, columns 5 or 7, lines 63 to 67 and 436 to 449.
Accordingly, the invention relates to nucleic acid molecules encoding a polypeptide having above-mentioned activity, e.g. conferring an increase in the the respective fine chemical in an organisms or parts thereof that contain changes in amino acid residues that are not essential for said activity. Such polypeptides differ in amino acid sequence from a sequence contained in a sequence as indicated in Table II, columns 5 or 7, lines 63 to 67 and 436 to 449, preferably of Table II B, column 7, lines 63 to 67 and 436 to 449 yet retain said activity described herein. The nucleic acid molecule can comprise a nucleotide sequence encoding a polypeptide, wherein the polypeptide comprises an amino acid sequence at least about 50% identical to an amino acid sequence as indicated in Table II, columns 5 or 7, lines 63 to 67 and 436 to 449, preferably of Table II B, column 7, lines 63 to 67 and 436 to 449 and is capable of participation in the increase of production of the respective fine chemical after increasing its activity, e.g. its expression. Preferably, the protein encoded by the nucleic acid molecule is at least about 60% identical to a sequence as indicated in Table II, columns 5 or 7, lines 63 to 67 and 436 to 449, preferably of Table II B, column 7, lines 63 to 67 and 436 to 449, more preferably at least about 70% identical to one of the sequences as indicated in Table II, columns 5 or 7, lines 63 to 67 and 436 to 449, preferably of Table II B, column 7, lines 63 to 67 and 436 to 449, even more preferably at least about 80%, 90%, or 95% homologous to a sequence as indicated in Table II, columns 5 or 7, lines 63 to 67 and 436 to 449, preferably of Table II B, column 7, lines 63 to 67 and 436 to 449, and most preferably at least about 96%, 97%, 98%, or 99% identical to the sequence as indicated in Table II, columns 5 or 7, lines 63 to 67 and 436 to 449, preferably of Table II B, column 7, lines 63 to 67 and 436 to 449.

**[0169.0.0.5] to [0175.0.5.5]** for the disclosure of the paragraphs [0169.0.0.5] to [0175.0.5.5] see paragraphs [0169.0.0.0] to [0175.0.0.0] above.

**[0176.0.5.5]** Functional equivalents derived from one of the polypeptides as shown in Table II, columns 5 or 7, lines 63 to 67 and 436 to 449 according to the invention by substitution, insertion or deletion have at least 30%, 35%, 40%, 45% or 50%, preferably at least 55%, 60%, 65% or 70% by preference at least 80%, especially preferably at least 85% or 90%, 91%, 92%, 93% or 94%, very especially preferably at least 95%, 97%, 98% or 99% homology with one of the polypeptides as shown in Table II, columns 5 or 7, lines 63 to 67 and 436 to 449 according to the invention and are distinguished by essentially the same properties as the polypeptide as shown in Table II, columns 5 or 7, lines 63 to 67 and 436 to 449.

**[0177.0.5.5]** Functional equivalents derived from the nucleic acid sequence as shown in Table I, columns 5 or 7, lines 63 to 67 and 436 to 449 according to the invention by substitution, insertion or deletion have at least 30%, 35%, 40%, 45% or 50%, preferably at least 55%, 60%, 65% or 70% by preference at least 80%, especially preferably at least 85% or 90%, 91%, 92%, 93% or 94%, very especially preferably at least 95%, 97%, 98% or 99% homology with one of the polypeptides as shown in Table II, columns 5 or 7, lines 63 to 67 and 436 to 449 according to the invention and encode polypeptides having essentially the same properties as the polypeptide as shown in Table II, columns 5 or 7, lines 63 to 67 and 436 to 449.

**[0178.0.0.5]** for the disclosure of this paragraph see [0178.0.0.0] above.

**[0179.0.5.5]** A nucleic acid molecule encoding a homologous protein to a protein sequence of as indicated in Table II, columns 5 or 7, lines 63 to 67 and 436 to 449, preferably of Table II B, column 7, lines 63 to 67 and 436 to 449 can be created by introducing one or more nucleotide substitutions, additions or deletions into a nucleotide sequence of the nucleic acid molecule of the present invention, in particular as indicated in Table I, columns 5 or 7, lines 63 to 67 and 436 to 449 such that one or more amino acid substitutions, additions or deletions are introduced into the encoded protein. Mutations can be introduced into the encoding sequences for example into a sequences as indicated in Table I, columns 5 or 7, lines 63 to 67 and 436 to 449 by standard techniques, such as site-directed mutagenesis and PCR-mediated mutagenesis.

**[0180.0.0.5] to [0183.0.0.5]** for the disclosure of the paragraphs [0180.0.0.5] to [0183.0.0.5] see paragraphs [0180.0.0.0] to [0183.0.0.0] above.

**[0184.0.5.5]** Homologues of the nucleic acid sequences used, with a sequence as indicated in Table I, columns 5 or 7, lines 63 to 67 and 436 to 449, preferably of Table I B, column 7, lines 63 to 67 and 436 to 449, or of the nucleic acid sequences derived from a sequences as indicated in Table II, columns 5 or 7, lines 63 to 67 and 436 to 449, preferably of Table II B, column 7, lines 63 to 67 and 436 to 449, comprise also allelic variants with at least approximately 30%, 35%, 40% or 45% homology, by preference at least approximately 50%, 60% or 70%, more preferably at least approximately 90%, 91%, 92%, 93%, 94% or 95% and even more preferably at least approximately 96%, 97%, 98%, 99% or more homology with one of the nucleotide sequences shown or the abovementioned derived nucleic acid sequences or their homologues, derivatives or analogues or parts of these. Allelic variants encompass in particular functional variants which can be obtained by deletion, insertion or substitution of nucleotides from the sequences shown, preferably from a sequence as indicated in Table I, columns 5 or 7, lines 63 to 67 and 436 to 449, or from the derived nucleic acid sequences, the intention being, however, that the enzyme activity or the biological activity of the resulting proteins synthesized is advantageously retained or increased.

**[0185.0.5.5]** In one embodiment of the present invention, the nucleic acid molecule of the invention or used in the process of the invention comprises one or more sequences as indicated in Table I, columns 5 or 7, lines 63 to 67 and 436 to 449, preferably of Table I B, column 7, lines 63 to 67 and 436 to 449. In one embodiment, it is preferred that the nucleic acid molecule comprises as little as possible other nucleotide sequences not shown in any one of sequences as indicated in Table I, columns 5 or 7, lines 63 to 67 and 436 to 449, preferably of Table I B, column 7, lines 63 to 67 and 436 to 449. In one embodiment, the nucleic acid molecule comprises less than 500, 400, 300, 200, 100, 90, 80, 70, 60, 50 or 40 further nucleotides. In a further embodiment, the nucleic acid molecule comprises less than 30, 20 or 10 further nucleotides. In one embodiment, a nucleic acid molecule used in the process of the invention is identical to a sequences as indicated in Table I, columns 5 or 7, lines 63 to 67 and 436 to 449, preferably of Table I B, column 7, lines 63 to 67 and 436 to 449.

**[0186.0.5.5]** Also preferred is that one or more nucleic acid molecule(s) used in the process of the invention encode a polypeptide comprising a sequence as indicated in Table II, columns 5 or 7, lines 63 to 67 and 436 to 449, preferably of Table II B, column 7, lines 63 to 67 and 436 to 449. In one embodiment, the nucleic acid molecule encodes less than 150, 130, 100, 80, 60, 50, 40 or 30 further amino acids. In a further embodiment, the encoded polypeptide comprises less than 20, 15, 10, 9, 8, 7, 6 or 5 further amino acids. In one embodiment, the encoded polypeptide used in the process of the invention is identical to the sequences as indicated in Table II, columns 5 or 7, lines 63 to 67 and 436 to 449, preferably of Table II B, column 7, lines 63 to 67 and 436 to 449.

**[0187.0.5.5]** In one embodiment, the nucleic acid molecule of the invention or used in the process encodes a polypeptide comprising a sequence as indicated in Table II, columns 5 or 7, lines 63 to 67 and 436 to 449, preferably of Table II B, column 7, lines 63 to 67 and 436 to 449 and comprises less than 100 further nucleotides. In a further embodiment, said nucleic acid molecule comprises less than 30 further nucleotides. In one embodiment, the nucleic acid molecule used in the process is identical to a coding sequence encoding a sequences as indicated in Table II, columns 5 or 7, lines 63 to 67 and 436 to 449, preferably of Table II B, column 7, lines 63 to 67 and 436 to 449.

**[0188.0.5.5]** Polypeptides (= proteins), which still have the essential biological activity of the polypeptide of the present invention conferring an increase of the fine chemical i.e. whose activity is essentially not reduced, are polypeptides with at least 10% or 20%, by preference 30% or 40%, especially preferably 50% or 60%, very especially preferably 80% or 90 or more of the wild type biological activity or enzyme activity, advantageously, the activity is essentially not reduced in comparison with the activity of a polypeptide shown in Table II, columns 5 or 7, lines 63 to 67 and 436 to 449 expressed under identical conditions.
In one embodiment, the polypeptide of the invention is a homolog consisting of or comprising the sequence as indicated in Table II B, columns 7, lines 63 to 67 and 436 to 449.

**[0189.0.5.5]** Homologues of sequences as indicated in Table I, columns 5 or 7, lines 63 to 67 and 436 to 449 or of the derived sequences shown in Table II, columns 5 or 7, lines 63 to 67 and 436 to 449 also mean truncated sequences, cDNA, single-stranded DNA or RNA of the coding and noncoding DNA sequence. Homologues of said sequences are also understood as meaning derivatives, which comprise noncoding regions such as, for example, UTRs, terminators, enhancers or promoter variants. The promoters upstream of the nucleotide sequences stated can be modified by one or more nucleotide substitution(s), insertion(s) and/or deletion(s) without, however, interfering with the functionality or activity either of the promoters, the open reading frame (= ORF) or with the 3'-regulatory region such as terminators or other 3'regulatory regions, which are far away from the ORF. It is furthermore possible that the activity of the promoters is increased by modification of their sequence, or that they are replaced completely by more active promoters, even promoters from heterologous organisms. Appropriate promoters are known to the person skilled in the art and are mentioned herein below.

**[0190.0.0.5], [0191.0.5.5], [00191.1.0.5] and [0192.0.0.5] to [0203.0.0.5]** for the disclosure of the paragraphs [0190.0.0.5], [0191.0.5.5], [0191.1.0.5] and [0192.0.0.5] to [0203.0.0.5] see paragraphs [0190.0.0.0], [0191.0.0.0], [0191.1.0.0] and [0192.0.0.0] to [0203.0.0.0] above.

**[0204.0.5.5]** Accordingly, in one embodiment, the invention relates to a nucleic acid molecule, which comprises a nucleic acid molecule selected from the group consisting of:
a) nucleic acid molecule encoding, preferably at least the mature form, of the polypeptide shown in Table II, columns 5 or 7, lines 63 to 67 and 436 to 449; preferably of Table II B, column 7, lines 63 to 67 and 436 to 449; or a fragment thereof conferring an increase in the amount of the fine chemical in an organism or a part thereof
b) nucleic acid molecule comprising, preferably at least the mature form, of the nucleic acid molecule shown in Table I, columns 5 or 7, lines 63 to 67 and 436 to 449 preferably of Table I B, column 7, lines 63 to 67 and 436 to 449; or a fragment thereof conferring an increase in the amount of the fine chemical in an organism or a part thereof;
c) nucleic acid molecule whose sequence can be deduced from a polypeptide sequence encoded by a nucleic acid molecule of (a) or (b) as result of the degeneracy of the genetic code and conferring an increase in the amount of the respective fine chemical in an organism or a part thereof;
d) nucleic acid molecule encoding a polypeptide whose sequence has at least 50% identity with the amino acid sequence of the polypeptide encoded by the nucleic acid molecule of (a) to (c) and conferring an increase in the amount of the respective fine chemical in an organism or a part thereof;
e) nucleic acid molecule which hybridizes with a nucleic acid molecule of (a) to (c) under under stringent hybridisation conditions and conferring an increase in the amount of the respective fine chemical in an organism or a part thereof;
f) nucleic acid molecule encoding a polypeptide, the polypeptide being derived by substituting, deleting and/or adding one or more amino acids of the amino acid sequence of the polypeptide encoded by the nucleic acid molecules (a) to (d), preferably to (a) to (c), and conferring an increase in the amount of the respective fine chemical in an organism or a part thereof;
g) nucleic acid molecule encoding a fragment or an epitope of a polypeptide which is encoded by one of the nucleic acid molecules of (a) to (e), preferably to (a) to (c) and conferring an increase in the amount of the respective fine chemical in an organism or a part thereof ;
h) nucleic acid molecule comprising a nucleic acid molecule which is obtained by amplifying a cDNA library or a genomic library using the primers or primer pairs as indicated in Table III, column 7, lines 63 to 67 and 436 to 449 and conferring an increase in the amount of the respective fine chemical in an organism or a part thereof;
i) nucleic acid molecule encoding a polypeptide which is isolated, e.g. from a expression library, with the aid of monoclonal antibodies against a polypeptide encoded by one of the nucleic acid molecules of (a) to (g), preferably to (a) to (c) and conferring an increase in the amount of the respective fine chemical in an organism or a part thereof;
j) nucleic acid molecule which encodes a polypeptide comprising the consensus sequence shown in Table IV, column 7, lines 63 to 67 and 436 to 449 and conferring an increase in the amount of the respective fine chemical in an organism or a part thereof;
k) nucleic acid molecule encoding the amino acid sequence of a polypeptide encoding a domaine of the polypeptide shown in Table II, columns 5 or 7, lines 63 to 67 and 436 to 449, preferably of Table II B, column 7, lines 63 to 67 and 436 to 449; and conferring an increase in the amount of the respective fine chemical in an organism or a part thereof; and
l) nucleic acid molecule which is obtainable by screening a suitable nucleic acid library under stringent hybridization conditions with a probe comprising one of the sequences of the nucleic acid molecule of (a) to (k) or with a fragment of at least 15 nt, preferably 20 nt, 30 nt, 50 nt, 100 nt, 200 nt or 500 nt of the nucleic acid molecule characterized in (a) to (h) or of the nucleic acid molecule shown in Table I, columns 5 or 7, lines 63 to 67 and 436 to 449 or a nucleic acid molecule encoding, preferably at least the mature form of, the polypeptide shown in Table II, columns 5 or 7, lines 63 to 67 and 436 to 449, and conferring an increase in the amount of the respective fine chemical in an organism or a part thereof; or which encompasses a sequence which is complementary thereto;
whereby, preferably, the nucleic acid molecule according to (a) to (I) distinguishes over the sequence as depicted in Table IA or IB, columns 5 or 7, lines 63 to 67 and 436 to 449 by one or more nucleotides. In one embodiment, the nucleic acid molecule of the invention does not consist of the sequence shown in Table IA or IB, columns 5 or 7, lines 63 to 67 and 436 to 449. In an other embodiment, the nucleic acid molecule of the present invention is at least 30 % identical and less than 100%, 99,999%, 99,99%, 99,9% or 99% identical to the sequence shown in Table IA or IB, columns 5 or 7, lines 63 to 67 and 436 to 449. In a further embodiment the nucleic acid molecule does not encode the polypeptide sequence shown in Table IIA or IIB, columns 5 or 7, lines 63 to 67 and 436 to 449. Accordingly, in one embodiment, the nucleic acid molecule of the present invention encodes in one embodiment a polypeptide which differs at least in one or more amino acids from the polypeptide as depicted in Table IIA or IIB, columns 5 or 7, lines 63 to 67 and 436 to 449 and therefore does not encode a protein of the sequence shown in Table IIA or IIB, columns 5 or 7, lines 63 to 67 and 436 to 449. Accordingly, in one embodiment, the protein encoded by a sequence of a nucleic acid according to (a) to (I) does not consist of the sequence shown in Table IIA or IIB, columns 5 or 7, lines 63 to 67 and 436 to 449. In a further embodiment, the protein of the present invention is at least 30 % identical to protein sequence depicted in Table IIA or IIB, columns 5 or 7, lines 63 to 67 and 436 to 449 and less than 100%, preferably less than 99,999%, 99,99% or 99,9%, more preferably less than 99%, 985, 97%, 96% or 95% identical to the sequence shown in Table IIA or IIB, columns 5 or 7, lines 63 to 67 and 436 to 449.

**[0205.0.0.5] to [0226.0.0.5]** for the disclosure of the paragraphs [0205.0.0.5] to [0226.0.0.5] see paragraphs [0205.0.0.0] to [0226.0.0.0] above.

**[0227.0.5.5]** The abovementioned nucleic acid molecules can be cloned into the nucleic acid constructs or vectors according to the invention in combination together with further genes, or else different genes are introduced by transforming several nucleic acid constructs or vectors (including plasmids) into a host cell, advantageously into a plant cell or a microorganisms.
In addition to the sequence mentioned in Table I, columns 5 or 7, lines 63 to 67 and 436 to 449 or its derivatives, it is advantageous additionally to express and/or mutate further genes in the organisms. Especially advantageously, additionally at least one further gene of the fatty acid biosynthetic pathway such as for palmitate, palmitoleate, stearate and/or oleate is expressed in the organisms such as plants or microorganisms. It is also possible that the regulation of the natural genes has been modified advantageously so that the gene and/or its gene product is no longer subject to the regulatory mechanisms which exist in the organisms. This leads to an increased synthesis of the respective desired fine chemical since, for example, feedback regulations no longer exist to the same extent or not at all. In addition it might be advantageously to combine the sequences shown in Table I, columns 5 or 7, lines 63 to 67 and 436 to 449 with genes which generally support or enhances to growth or yield of the target organismen, for example genes which lead to faster growth rate of microorganisms or genes which produces stress-, pathogen, or herbicide resistant plants.

**[0228.0.5.5]** In a further embodiment of the process of the invention, therefore, organisms are grown, in which there is simultaneous overexpression of at least one nucleic acid or one of the genes which code for proteins involved in the fatty acid metabolism, in particular in fatty acid synthesis.

**[0229.0.5.5]** Further advantageous nucleic acid sequences which can be expressed in combination with the sequences used in the process and/or the abovementioned biosynthesis genes are the sequences encoding further genes of the saturated, poly unsaturated fatty acid biosynthesis such as desaturases like Δ-4-desaturases, Δ-5-desaturases, Δ-6-desaturases, Δ-8-desaturases, Δ-9-desaturases, Δ-12-desaturases, Δ-17-desaturases, ω-3-desaturases, elongases like Δ-5-elongases, Δ-6-elongases, Δ-9-elongases, acyl-CoA-dehydrogenases, acyl-ACP-desaturases, acyl-ACP-thioesterases, fatty acid acyl-transferases, acyl-CoA lysophospholipid-acyltransferases, acyl-CoA carboxylases, fatty acid synthases, fatty acid hydroxylases, acyl-CoA oxydases, acetylenases, lipoxygenases, triacyl-lipases etc. as described in WO 98/46765, WO 98/46763, WO 98/46764, WO 99/64616, WO 00/20603, WO 00/20602, WO 00/40705, US 20040172682, US 20020156254, US 6,677,145 US 20040053379 or US 20030101486. These genes lead to an increased synthesis of the essential fatty acids.

**[0230.0.5.5]** for the disclosure of this paragraph see paragraph [0230.0.0.0] above.

**[0231.0.5.5]** In a further advantageous embodiment of the process of the invention, the organisms used in the process are those in which simultaneously a linoleic acid degrading protein is attenuated, in particular by reducing the rate of expression of the corresponding gene.

**[0232.0.0.5] to [0276.0.0.5]** for the disclosure of the paragraphs [0232.0.0.5] to [0276.0.0.5] see paragraphs [0232.0.0.0] to [0276.0.0.0] above.

**[0277.0.5.5]** The fatty acids produced can be isolated from the organism by methods with which the skilled worker is familiar. For example via extraction, salt precipitation and/or different chromatography methods. The process according to the invention can be conducted batchwise, semibatchwise or continuously. The fine chemical produced in the process according to the invention can be isolated as mentioned above from the organisms, advantageously plants, in the form of their oils, fats, lipids and/or free fatty acids. Fatty acids produced by this process can be obtained by harvesting the organisms, either from the crop in which they grow, or from the field. This can be done via pressing or extraction of the plant parts, preferably the plant seeds. Hexane is preferably used as solvent in the process, in which more than 96% of the compounds produced in the process can be isolated. Thereafter, the resulting products are processed further, i.e. degummed, refined, bleached and/or deodorized.

**[0278.0.0.5] to [0282.0.0.5]** for the disclosure of the paragraphs [0278.0.0.5] to [0282.0.0.5] see paragraphs [0278.0.0.0] to [0282.0.0.0] above.

**[0283.0.5.5]** Moreover, native polypeptide conferring the increase of the respective fine chemical in an organism or part thereof can be isolated from cells (e.g., endothelial cells), for example using the antibody of the present invention as described below, in particular, an antibody against polypeptides as shown in Table II, columns 5 or 7, lines 63 to 67 and 436 to 449 or an antigenic part thereof, which can be produced by standard techniques utilizing polypeptides comprising or consisting of abovementioned sequences, e.g. the polypeptide of the present invention or fragment thereof. Preferred are monoclonal antibodies specifically binding to polypeptides as indicated in Table II, columns 5 or 7, lines 63 to 67 and 436 to 449.

**[0284.0.0.5]** for the disclosure of this paragraph see [0284.0.0.0] above.

**[0285.0.5.5]** In one embodiment, the present invention relates to a polypeptide having the sequence shown in Table II, columns 5 or 7, lines 63 to 67 and 436 to 449 or as coded by the nucleic acid molecule shown in Table I, columns 5 or 7, lines 63 to 67 and 436 to 449 or functional homologues thereof.

**[0286.0.5.5]** In one advantageous embodiment, in the method of the present invention the activity of a polypeptide is increased which comprises or consists of a consensus sequence as indicated in Table IV, column 7, lines 63 to 67 and 436 to 449 and in one another embodiment, the present invention relates to a polypeptide comprising or consisting of a consensus sequence as indicated in Table IV, column 7, lines 63 to 67 and 436 to 449, whereby 20 or less, preferably 15 or 10, preferably 9, 8, 7, or 6, more preferred 5 or 4, even more preferred 3, even more preferred 2, even more preferred 1, most preferred 0 of the amino acids positions indicated can be replaced by any amino acid or, in an further embodiment, can be replaced and/or absent. In one embodiment, the present invention relates to the method of the present invention comprising a poylpeptide or to a polypeptide comprising more than one consensus sequences as indicated in Table IV, column 7, lines 63 to 67 and 436 to 449.

**[0287.0.0.5] to [0290.0.0.5]** for the disclosure of the paragraphs [0287.0.0.5] to [0290.0.0.5] see paragraphs [0287.0.0.0] to [0290.0.0.0] above.

**[0291.0.5.5]** In one advantageous embodiment, the method of the present invention comprises the increasing of a polypeptide comprising or consisting of plant or microorganism specific consensus sequences. Accordingly, in one embodiment, the present invention relates to a polypeptide comprising or consisting of a plant or microorganism specific consensus sequences.
In one embodiment, said polypeptide of the invention distinguishes over the sequence as indicated in Table IIA or IIB, columns 5 or 7, lines 63 to 67 and 436 to 449 by one or more amino acids. In one embodiment, polypeptide distinguishes form the sequence shown in Table IIA or IIB, columns 5 or 7, lines 63 to 67 and 436 to 449 by more than 5, 6, 7, 8 or 9 amino acids, preferably by more than 10, 15, 20, 25 or 30 amino acids, evenmore preferred are more than 40, 50, or 60 amino acids and, preferably, the sequence of the polypeptide of the invention distinguishes from the sequence shown in Table IIA or IIB, columns 5 or 7, lines 63 to 67 and 436 to 449 by not more than 80% or 70% of the amino acids, preferably not more than 60% or 50%, more preferred not more than 40% or 30%, even more preferred not more than 20% or 10%. In another embodiment, said polypeptide of the invention does not consist of the sequence shown in Table IIA or IIB, columns 5 or 7, lines 63 to 67 and 436 to 449.

**[0292.0.0.5]** for the disclosure of this paragraph see [0292.0.0.0] above.

**[0293.0.5.5]** In one embodiment, the invention relates to polypeptide conferring an increase in the respective fine chemical in an organism or part being encoded by the nucleic acid molecule of the invention or by a nucleic acid molecule used in the process.
In one embodiment, the polypeptide of the invention has a sequence, which distinguishes from the sequence as shown in Table IIA or IIB, columns 5 or 7, lines 63 to 67 and 436 to 449 by one or more amino acids. In another embodiment, said polypeptide of the invention does not consist of the sequence shown in Table IIA or IIB, columns 5 or 7, lines 63 to 67 and 436 to 449. In a further embodiment, said polypeptide of the present invention is less than 100%, 99,999%, 99,99%, 99,9% or 99% identical. In one embodiment, said polypeptide does not consist of the sequence encoded by the nucleic acid molecules shown in Table IA or IB, columns 5 or 7, lines 63 to 67 and 436 to 449.

**[0294.0.5.5]** In one embodiment, the present invention relates to a polypeptide having an activity of a protein as indicated in Table IIA or IIB, column 3, lines 63 to 67 and 436 to 449, which distinguishes over the sequence as indicated in Table IIA or IIB, columns 5 or 7, lines 63 to 67 and 436 to 449 by one or more amino acids, preferably by more than 5, 6, 7, 8 or 9 amino acids, preferably by more than 10, 15, 20, 25 or 30 amino acids, evenmore preferred are more than 40, 50, or 60 amino acids but even more preferred by less than 70% of the amino acids, more preferred by less than 50%, even more preferred my less than 30% or 25%, more preferred are 20% or 15%, even more preferred are less than 10%.

**[0295.0.0.5], [0296.0.0.5] and [0297.0.5.5]** for the disclosure of the paragraphs [0295.0.0.5], [0296.0.0.5] and [0297.0.5.5] see paragraphs [0295.0.0.0] to [0297.0.0.0] above.

**[00297.1.0.5]** Non-polypeptide of the invention-chemicals are e.g. polypeptides having not the activity and/or the amino acid sequence of a polypeptide indicated in Table II, columns 3, 5 or 7, lines 63 to 67 and 436 to 449.

**[0298.0.5.5]** A polypeptide of the invention can participate in the process of the present invention. The polypeptide or a portion thereof comprises preferably an amino acid sequence which is sufficiently homologous to an amino acid sequence as indicated in Table II, columns 5 or 7, lines 63 to 67 and 436 to 449 such that the protein or portion thereof maintains the ability to confer the activity of the present invention. The portion of the protein is preferably a biologically active portion as described herein. Preferably, the polypeptide used in the process of the invention has an amino acid sequence identical to a sequence as indicated in Table II, columns 5 or 7, lines 63 to 67 and 436 to 449.

**[0299.0.5.5]** Further, the polypeptide can have an amino acid sequence which is encoded by a nucleotide sequence which hybridizes, preferably hybridizes under stringent conditions as described above, to a nucleotide sequence of the nucleic acid molecule of the present invention. Accordingly, the polypeptide has an amino acid sequence which is encoded by a nucleotide sequence that is at least about 35%, 40%, 45%, 50%, 55%, 60%, 65% or 70%, preferably at least about 75%, 80%, 85% or 90, and more preferably at least about 91%, 92%, 93%, 94% or 95%, and even more preferably at least about 96%, 97%, 98%, 99% or more homologous to one of the amino acid sequences of Table II, columns 5 or 7, lines 63 to 67 and 436 to 449. The preferred polypeptide of the present invention preferably possesses at least one of the activities according to the invention and described herein. A preferred polypeptide of the present invention includes an amino acid sequence encoded by a nucleotide sequence which hybridizes, preferably hybridizes under stringent conditions, to a nucleotide sequence of Table I, columns 5 or 7, lines 63 to 67 and 436 to 449 or which is homologous thereto, as defined above.

**[0300.0.5.5]** Accordingly the polypeptide of the present invention can vary from a sequence as indicated in Table II, columns 5 or 7, lines 63 to 67 and 436 to 449 in amino acid sequence due to natural variation or mutagenesis, as described in detail herein. Accordingly, the polypeptide comprise an amino acid sequence which is at least about 35%, 40%, 45%, 50%, 55%, 60%, 65% or 70%, preferably at least about 75%, 80%, 85% or 90, and more preferably at least about 91%, 92%, 93%, 94% or 95%, and most preferably at least about 96%, 97%, 98%, 99% or more homologous to an entire amino acid sequence of a sequence as indicated in Table IIA or IIB, columns 5 or 7, lines 63 to 67 and 436 to 449.

**[0301.0.0.5]** for the disclosure of this paragraph see [0301.0.0.0] above.

**[0302.0.5.5]** Biologically active portions of an polypeptide of the present invention include peptides comprising amino acid sequences derived from the amino acid sequence of the polypeptide of the present invention or used in the process of the present invention, e.g., an amino acid sequence shown in Table II, columns 5 or 7, lines 63 to 67 and 436 to 449 or the amino acid sequence of a protein homologous thereto, which include fewer amino acids than a full length polypeptide of the present invention or used in the process of the present invention or the full length protein which is homologous to an polypeptide of the present invention or used in the process of the present invention depicted herein, and exhibit at least one activity of polypeptide of the present invention or used in the process of the present invention.

**[0303.0.0.5]** for the disclosure of this paragraph see [0303.0.0.0] above.

**[0304.0.5.5]** Manipulation of the nucleic acid molecule of the invention or the nucleic acid molecule used in the method of the invention may result in the production of a protein having essentially the activity of the polypeptides as indicated in Table II, columns 5 or 7, lines 63 to 67 and 436 to 449 but having differences in the sequence from said wild-type protein. These proteins may be improved in efficiency or activity, may be present in greater numbers in the cell than is usual, or may be decreased in efficiency or activity in relation to the wild type protein.

**[0305.0.5.5], [0306.0.5.5] and [0306.1.0.5]** for the disclosure of the paragraphs [0305.0.5.5], [0306.0.5.5] and [0306.1.0.5] see paragraphs [0305.0.0.0], [0306.0.0.0] and [0306.1.0.0] above.

**[0307.0.0.5] and [0308.0.0.5]** for the disclosure of the paragraphs [0307.0.0.5] and [0308.0.0.5] see paragraphs [0307.0.0.0 and [0308.0.0.0] above.

**[0309.0.0.5]** In one embodiment, a reference to protein (= polypeptide)" of the invention e.g. as indicated in Table II, columns 5 or 7, lines 63 to 67 and 436 to 449 refers to a polypeptide having an amino acid sequence corresponding to the polypeptide of the invention or used in the process of the invention, whereas a "non-polypeptide" or "other polypeptide" e.g. not indicated in Table II, columns 5 or 7, lines 63 to 67 and 436 to 449 refers to a polypeptide having an amino acid sequence corresponding to a protein which is not substantially homologous to a polypeptide of the invention, preferably which is not substantially homologous to a polypeptide having a protein activity, e.g., a protein which does not confer the activity described herein and which is derived from the same or a different organism.

**[0310.0.0.5] to [0334.0.0.5]** for the disclosure of the paragraphs [0310.0.0.5] to [0334.0.0.5] see paragraphs [0310.0.0.0] to [0334.0.0.0] above.

**[0335.0.5.5]** The dsRNAi method has proved to be particularly effective and advantageous for reducing the expression of a nucleic acid sequence as indicated in Table I, columns 5 or 7, lines 63 to 67 and 436 to 449 and/or homologs thereof. As described inter alia in WO 99/32619, dsRNAi approaches are clearly superior to traditional antisense approaches. The invention therefore furthermore relates to double-stranded RNA molecules (dsRNA molecules) which, when introduced into an organism, advantageously into a plant (or a cell, tissue, organ or seed derived therefrom), bring about altered metabolic activity by the reduction in the expression of a nucleic acid sequences as indicated in Table I, columns 5 or 7, lines 63 to 67 and 436 to 449 and/or homologs thereof. In a double-stranded RNA molecule for reducing the expression of an protein encoded by a nucleic acid sequence of one of the sequences as indicated in Table I, columns 5 or 7, lines 63 to 67 and 436 to 449 and/or homologs thereof, one of the two RNA strands is essentially identical to at least part of a nucleic acid sequence, and the respective other RNA strand is essentially identical to at least part of the complementary strand of a nucleic acid sequence.

**[0336.0.0.5] to [0342.0.0.5]** for the disclosure of the paragraphs [0336.0.0.5] to [0342.0.0.5] see paragraphs [0336.0.0.0] to [0342.0.0.0] above.

**[0343.0.5.5]** As has already been described, 100 % sequence identity between the dsRNA and a gene transcript of a nucleic acid sequence as indicated in Table I, columns 5 or 7, lines 63 to 67 and 436 to 449 or it's homolog is not necessarily required in order to bring about effective reduction in the expression. The advantage is, accordingly, that the method is tolerant with regard to sequence deviations as may be present as a consequence of genetic mutations, polymorphisms or evolutionary divergences. Thus, for example, using the dsRNA, which has been generated starting from a sequence of one of the sequences as indicated in Table I, columns 5 or 7, lines 63 to 67 and 436 to 449 or homologs thereof of the one organism, may be used to suppress the corresponding expression in another organism.

**[0344.0.0.5] to [0350.0.0.5], [0351.0.5.5] and [0352.0.0.5] to [0361.0.0.5]** for the disclosure of the paragraphs [0344.0.0.5] to [0350.0.0.5], [0351.0.5.5] and [0352.0.0.5] to [0361.0.0.5] see paragraphs [0344.0.0.0] to [0361.0.0.0] above.

**[0362.0.5.5]** Accordingly the present invention relates to any cell transgenic for any nucleic acid characterized as part of the invention, e.g. conferring the increase of the respective fine chemical in a cell or an organism or a part thereof, e.g. the nucleic acid molecule of the invention or the nucleic acid molecule used in the method of the invention, the nucleic acid construct of the invention, the antisense molecule of the invention, the vector of the invention or a nucleic acid molecule encoding the polypeptide of the invention, e.g. encoding a polypeptide having an activity of a protein such as the polypeptides as indicated in Table II, column 3, lines 63 to 67 and 436 to 449. Due to the above mentioned activity the fine chemical content in a cell or an organism is increased. For example, due to modulation or manupulation, the cellular activity is increased, e.g. due to an increased expression or specific activity of the subject matters of the invention in a cell or an organism or a part thereof. Transgenic for a polypeptide having an activity of a protein such as the polypeptides as indicated in Table II, column 3, lines 63 to 67 and 436 to 449. Activity means herein that due to modulation or manipulation of the genome, the activity of polypeptide having an activity of a protein such as the polypeptides as indicated in Table II, column 3, lines 63 to 67 and 436 to 449 or a similar activity, which is increased in the cell or organism or part thereof. Examples are described above in context with the process of the invention.

**[0363.0.0.5], [0364.0.5.5] and [0365.0.0.5] to [0379.0.5.5]** for the disclosure of the paragraphs [0363.0.0.5], [0364.0.5.5] and [0365.0.0.5] to [0379.0.5.5] see paragraphs [0363.0.0.0] to [0379.0.0.0] above.

**[0380.0.5.5]** The fatty acids obtained in the process are suitable as starting material for the synthesis of further products of value. For example, they can be used in combination with each other or alone for the production of pharmaceuticals, foodstuffs, animal feeds or cosmetics. Accordingly, the present invention relates a method for the production of a pharmaceuticals, food stuff, animal feeds, nutrients or cosmetics comprising the steps of the process according to the invention, including the isolation of the fatty acid composition produced or the fine chemical produced if desired and formulating the product with a pharmaceutical acceptable carrier or formulating the product in a form acceptable for an application in agriculture. A further embodiment according to the invention is the use of the fatty acids produced in the process or of the transgenic organisms in animal feeds, foodstuffs, medicines, food supplements, cosmetics or pharmaceuticals.

**[0381.0.0.5] and [0382.0.0.5]** for the disclosure of the paragraphs [0381.0.0.5] and [0382.0.0.5] see paragraphs [0381.0.0.0] and [0382.0.0.0] above.

**[0383.0.5.5]** For preparing fatty acid compound-containing fine chemicals, in particular the fine chemical, it is possible to use as fatty acid source organic compounds such as, for example, oils, fats and/or lipids comprising fatty acids such as fatty acids having a carbon back bone between C₁₀- to C₁₆- carbon atoms and/or small organic acids such acetic acid, propionic acid or butanoic acid as precursor compounds.

**[0384.0.0.5] and [0385.0.5.5]** for the disclosure of the paragraphs [0384.0.0.5] and [0385.0.5.5] see paragraphs [0384.0.0.0] and [0385.0.0.0] above.

**[0386.0.5.5]** However, it is also possible to purify the fatty acid produced further. For this purpose, the product-containing composition is subjected for example to a thin layer chromatography on silica gel plates or to a chromatography such as a Florisil column (Bouhours J.F., J. Chromatrogr. 1979, 169, 462), in which case the desired product or the impurities are retained wholly or partly on the chromatography resin. These chromatography steps can be repeated if necessary, using the same or different chromatography resins. The skilled worker is familiar with the choice of suitable chromatography resins and their most effective use. An alternative method to purify the fatty acids is for example a crystallization in the presence of urea. These methods can be combined with each other.

**[0387.0.0.5] to [0392.0.0.5]** for the disclosure of the paragraphs [0387.0.0.5] to [0392.0.0.5] see paragraphs [0387.0.0.0] to [0392.0.0.0] above.

**[0393.0.5.5]** In one embodiment, the present invention relates to a method for the identification of a gene product conferring an increase in the respective fine chemical production in a cell, comprising the following steps:
(a) contacting, e.g. hybridising, the nucleic acid molecules of a sample, e.g. cells, tissues, plants or microorganisms or a nucleic acid library , which can contain a candidate gene encoding a gene product conferring an increase in the respective fine chemical after expression, with the nucleic acid molecule of the present invention;
(b) identifying the nucleic acid molecules, which hybridize under relaxed stringent conditions with the nucleic acid molecule of the present invention in particular to the nucleic acid molecule sequence shown in Table I, columns 5 or 7, lines 63 to 67 and 436 to 449, preferably in Table I B, columns 5 or 7, lines 63 to 67 and 436 to 449 and, optionally, isolating the full length cDNA clone or complete genomic clone;
(c) introducing the candidate nucleic acid molecules in host cells, preferably in a plant cell or a microorganism, appropriate for producing the respective fine chemical;
(d) expressing the identified nucleic acid molecules in the host cells;
(e) assaying the respective fine chemical level in the host cells; and
(f) identifying the nucleic acid molecule and its gene product which expression confers an increase in the respective fine chemical level in the host cell after expression compared to the wild type.

**[0394.0.0.5] to [0415.0.0.5] and [0416.0.5.5]** for the disclosure of the paragraphs [0394.0.0.5] to [0415.0.0.5] and [0416.0.5.5] see paragraphs [0394.0.0.0] to [0416.0.0.0] above.

**[0417.0.5.5]** The nucleic acid molecule of the invention, the vector of the invention or the nucleic acid construct of the invention may also be useful for the production of organisms resistant to inhibitors of the fatty acid production biosynthesis pathways. In particular, the overexpression of the polypeptide of the present invention may protect an organism such as a microorganism or a plant against inhibitors, which block the fatty acid, in particular the fine chemical, synthesis in said organism. Examples of inhibitors or herbicides blocking the fatty acid synthesis in organism such as microorganism or plants are for example cerulenin, Thiolactomycin, Diazoborines or Triclosan, which inhibit the fatty acids (beta-ketoacyl thioester synthetase inhibitors) and sterol biosynthesis inhibitors, aryloxyphenoxypropionates such as diclofop, fenoxaprop, haloxyfop, fluazifop or quizalofop or cyclohexanediones such as clethodim or sethoxydim [(2-[1-{ethoxyimino}butyl]-5-[2-{ethylthio}propyl]-3-hydroxy-2-cyclohexen-1-one], which inhibit the plant acetyl-coenzyme A carboxylase or thiocarbamates such as butylate, EPTC [= S-ethyl dipropylcarbamothioat] or vernolate.

**[0418.0.0.5] to [0430.0.0.5]** for the disclosure of the paragraphs [0418.0.0.5] to [0430.0.0.5] see paragraphs [0418.0.0.0] to [0430.0.0.0] above.

**[0431.0.5.5], [0432.0.5.5], [0433.0.0.5] and [0434.0.0.5]** for the disclosure of the paragraphs [0431.0.5.5], [0432.0.5.5], [0433.0.0.5] and [0434.0.0.5] see paragraphs [0431.0.0.0] to [0434.0.0.0] above.

**[0435.0.5.5]** Example 3: In-vivo and in-vitro mutagenesis

**[0436.0.5.5]** An *in vivo* mutagenesis of organisms such as Saccharomyces, Mortierella, Escherichia and others mentioned above, which are beneficial for the production of fatty acids can be carried out by passing a plasmid DNA (or another vector DNA) containing the desired nucleic acid sequence or nucleic acid sequences through E. coli and other microorganisms (for example Bacillus spp. or yeasts such as Saccharomyces cerevisiae) which are not capable of maintaining the integrity of its genetic information. Usual mutator strains have mutations in the genes for the DNA repair system [for example mutHLS, mutD, mutT and the like; for comparison, see Rupp, W.D. (1996) DNA repair mechanisms in Escherichia coli and Salmonella, pp. 2277-2294, ASM: Washington]. The skilled worker knows these strains. The use of these strains is illustrated for example in Greener, A. and Callahan, M. (1994) Strategies 7; 32-34.
In-vitro mutation methods such as increasing the spontaneous mutation rates by chemical or physical treatment are well known to the skilled person. Mutagens like 5-bromo-uracil, N-methyl-N-nitro-N-nitrosoguanidine (= NTG), ethyl methanesulfonate (= EMS), hydroxylamine and/or nitrous acid are widly used as chemical agents for random in-vitro mutagensis. The most common physical method for mutagensis is the treatment with UV irradiation. Another random mutagenesis technique is the error-prone PCR for introducing amino acid changes into proteins. Mutations are deliberately introduced during PCR through the use of error-prone DNA polymerases and special reaction conditions known to a person skilled in the art. For this method randomized DNA sequences are cloned into expression vectors and the resulting mutant libraries screened for altered or improved protein activity as described below.
Site-directed mutagensis method such as the introduction of desired mutations with an M13 or phagemid vector and short oligonucleotides primers is a well-known approach for site-directed mutagensis. The clou of this method involves cloning of the nucleic acid sequence of the invention into an M13 or phagemid vector, which permits recovery of single-stranded recombinant nucleic acid sequence. A mutagenic oligonucleotide primer is then designed whose sequence is perfectly complementary to nucleic acid sequence in the region to be mutated, but with a single difference: at the intended mutation site it bears a base that is complementary to the desired mutant nucleotide rather than the original. The mutagenic oligonucleotide is then allowed to prime new DNA synthesis to create a complementary full-length sequence containing the desired mutation. Another site-directed mutagensis method is the PCR mismatch primer mutagensis method also known to the skilled person. Dpnl site-directed mutagensis is a further known method as described for example in the Stratagene QuickchangeTM site-directed mutagenesis kit protocol. A huge number of other methods are also known and used in common practice.
Positive mutation events can be selected by screening the organisms for the production of the desired fine chemical.

**[0437.0.5.5]** Example 4: DNA transfer between Escherichia coli, Saccharomyces cerevisiae and Mortierella alpina

**[0438.0.5.5]** Shuttle vectors such as pYE22m, pPAC-ResQ, pClasper, pAUR224, pAMH10, pAML10, pAMT10, pAMU10, pGMH10, pGML10, pGMT10, pGMU10, pPGAL1, pPADH1, pTADH1, pTAex3, pNGA142, pHT3101 and derivatives thereof which alow the transfer of nucleic acid sequences between Escherichia coli, Saccharomyces cerevisiae and/or Mortierella alpina are available to the skilled worker. An easy method to isolate such shuttle vectors is disclosed by Soni R. and Murray J.A.H. [Nucleic Acid Research, vol. 20 no. 21, 1992: 5852]: If necessary such shuttle vectors can be constructed easily using standard vectors for E. coli (Sambrook, J. et al., (1989), "Molecular Cloning: A Laboratory Manual", Cold Spring Harbor Laboratory Press or Ausubel, F.M. et al. (1994) "Current Protocols in Molecular Biology", John Wiley & Sons) and/or the aforementioned vectors, which have a replication origin for, and suitable marker from, Escherichia coli, Saccharomyces cerevisiae or Mortierella alpina added. Such replication origins are preferably taken from endogenous plasmids, which have been isolated from species used in the inventive process. Genes, which are used in particular as transformation markers for these species are genes for kanamycin resistance (such as those which originate from the Tn5 or Tn-903 transposon) or for chloramphenicol resistance (Winnacker, E.L. (1987) "From Genes to Clones - Introduction to Gene Technology, VCH, Weinheim) or for other antibiotic resistance genes such as for G418, gentamycin, neomycin, hygromycin or tetracycline resistance.

**[0439.0.5.5]** Using standard methods, it is possible to clone a gene of interest into one of the above-described shuttle vectors and to introduce such hybrid vectors into the microorganism strains used in the inventive process. The transformation of Saccharomyces can be achieved for example by LiCl or sheroplast transformation (Bishop et al., Mol. Cell. Biol., 6, 1986: 3401 - 3409; Sherman et al., Methods in Yeasts in Genetics, [Cold Spring Harbor Lab. Cold Spring Harbor, N. Y.] 1982, Agatep et al., Technical Tips Online 1998, 1:51: P01525 or Gietz et al., Methods Mol. Cell. Biol. 5, 1995: 255-269) or electroporation (Delorme E., Appl. Environ. Microbiol., vol. 55, no. 9, 1989 : 2242 - 2246).

**[0440.0.5.5]** If the transformed sequence(s) is/are to be integrated advantageously into the genome of the microorganism used in the inventive process for example into the yeast or fungi genome, standard techniques known to the skilled worker also exist for this purpose. Solinger et al. (Proc Natl Acad Sci U S A., 2001 (15): 8447-8453) and Freedman et al. (Genetics, Vol. 162, 15-27, September 2002,) teaches a homolog recombination system dependent on rad 50, rad51, rad54 and rad59 in yeasts. Vectors using this system for homologous recombination are vectors derived from the Ylp series. Plasmid vectors derived for example from the 2µ-Vector are known by the skilled worker and used for the expression in yeasts. Other preferred vectors are for example pART1, pCHY21 or pEVP11 as they have been described by McLeod et al. (EMBO J. 1987, 6:729-736) and Hoffman et al. (Genes Dev. 5, 1991: :561-571.) or Russell et al. (J. Biol. Chem. 258, 1983: 143-149.). Other beneficial yeast vectors are plasmids of the REP, REP-X, pYZ or RIP series.

**[0441.0.0.5]** for the disclosure of this paragraph see [0441.0.0.0] above.

**[0442.0.5.5]** The observations of the acivity of a mutated, or transgenic, protein in a transformed host cell are based on the fact that the protein is expressed in a similar manner and in a similar quantity as the wild-type protein. A suitable method for determining the transcription quantity of the mutant, or transgenic, gene (a sign for the amount of mRNA which is available for the translation of the gene product) is to carry out a Northern blot (see, for example, Ausubel et al., (1988) Current Protocols in Molecular Biology, Wiley: New York), where a primer which is designed in such a way that it binds to the gene of interest is provided with a detectable marker (usually a radioactive or chemiluminescent marker) so that, when the total RNA of a culture of the organism is extracted, separated on a gel, applied to a stable matrix and incubated with this probe, the binding and quantity of the binding of the probe indicates the presence and also the amount of mRNA for this gene. Another method is a quantitative PCR. This information detects the extent to which the gene has been transcribed. Total cell RNA can be isolated for example from yeasts or E. coli by a variety of methods, which are known in the art, for example with the Ambion kit according to the instructions of the manufacturer or as described in Edgington et al., Promega Notes Magazine Number 41, 1993, p. 14.

**[0443.0.0.5]** for the disclosure of this paragraph see [0443.0.0.0] above.

**[0444.0.5.5]** Example 6: Growth of genetically modified organism: media and culture conditions

**[0445.0.5.5]** Genetically modified Yeast, Mortierella or Escherichia coli are grown in synthetic or natural growth media known by the skilled worker. A number of different growth media for Yeast, Mortierella or Escherichia coli are well known and widely available. A method for clulturing Mortierella is disclosed by Jang et al. [Bot. Bull. Acad. Sin. (2000) 41: 41-48]. Mortierella can be grown at 20°C in a culture medium containing: 10 g/I glucose, 5 g/I yeast extract at pH 6.5. Futhermore Jang et al. teaches a submerged basal medium containing 20 g/l soluble starch, 5 g/l Bacto yeast extract, 10 g/l KNO₃, 1 g/l KH₂PO₄, and 0.5 g/l MgSO₄•7H₂O, pH 6.5.

**[0446.0.0.5] to [0453.0.0.5]** for the disclosure of the paragraphs [0446.0.0.5] to [0453.0.0.5] see paragraphs [0446.0.0.0] to [0453.0.0.0] above.

**[0454.0.5.5]** Example 8: Analysis of the effect of the nucleic acid molecule on the production of the fatty acid

**[0455.0.5.5]** The effect of the genetic modification in plants, fungi, algae or ciliates on the production of a desired compound (such as a fatty acid) can be determined by growing the modified microorganisms or the modified plant under suitable conditions (such as those described above) and analyzing the medium and/or the cellular components for the elevated production of desired product (i.e. of the lipids or a fatty acid). These analytical techniques are known to the skilled worker and comprise spectroscopy, thin-layer chromatography, various types of staining methods, enzymatic and microbiological methods and analytical chromatography such as high-performance liquid chromatography (see, for example, Ullman, Encyclopedia of Industrial Chemistry, Vol. A2, p. 89-90 and p. 443-613, VCH: Weinheim (1985); Fallon, A., et al., (1987) "Applications of HPLC in Biochemistry" in: Laboratory Techniques in Biochemistry and Molecular Biology, Vol. 17; Rehm et al. (1993) Biotechnology, Vol. 3, Chapter III: "Product recovery and purification", p. 469-714, VCH: Weinheim; Belter, P.A., et al. (1988) Bioseparations: downstream processing for Biotechnology, John Wiley and Sons; Kennedy, J.F., and Cabral, J.M.S. (1992) Recovery processes for biological Materials, John Wiley and Sons; Shaeiwitz, J.A., and Henry, J.D. (1988) Biochemical Separations, in: Ullmann's Encyclopedia of Industrial Chemistry, Vol. B3; Chapter 11, p. 1-27, VCH: Weinheim; and Dechow, F.J. (1989) Separation and purification techniques in biotechnology, Noyes Publications).
In addition to the abovementioned processes, plant lipids are extracted from plant material as described by Cahoon et al. (1999) Proc. Natl. Acad. Sci. USA 96 (22): 12935-12940 and Browse et al. (1986) Analytic Biochemistry 152:141-145. The qualitative and quantitative analysis of lipids or fatty acids is described by Christie, William W., Advances in Lipid Methodology, Ayr/Scotland: Oily Press (Oily Press Lipid Library; 2); Christie, William W., Gas Chromatography and Lipids. A Practical Guide - Ayr, Scotland: Oily Press, 1989, Repr. 1992, IX, 307 pp. (Oily Press Lipid Library; 1); "Progress in Lipid Research, Oxford: Pergamon Press, 1 (1952) - 16 (1977) under the title: Progress in the Chemistry of Fats and Other Lipids CODEN.

**[0456.0.0.5]** for the disclosure of this paragraph see [0456.0.0.0] above.

**[0457.0.5.5]** Example 9: Purification of the fatty acid

**[0458.0.5.5]** One example is the analysis of fatty acids (abbreviations: FAME, fatty acid methyl ester; GC-MS, gas liquid chromatography/mass spectrometry; TAG, triacylglycerol; TLC, thin-layer chromatography).
The unambiguous detection for the presence of fatty acid products can be obtained by analyzing recombinant organisms using analytical standard methods: GC, GC-MS or TLC, as described on several occasions by Christie and the references therein (1997, in: Advances on Lipid Methodology, Fourth Edition: Christie, Oily Press, Dundee, 119-169; 1998, Gaschromatographie-Massenspektrometrie-Verfahren [Gas chromatography/mass spectrometric methods], Lipide 33:343-353).
The total fatty acids produced in the organism for example in yeasts used in the inventive process can be analysed for example according to the following procedure: The material such as yeasts, E. coli or plants to be analyzed can be disrupted by sonication, grinding in a glass mill, liquid nitrogen and grinding or via other applicable methods. After disruption, the material must be centrifuged (1000 x g, 10 min., 4°C) and washed once with 100 mM NaHCO₃, pH 8.0 to remove residual medium and fatty acids. For preparation of the fatty acid methyl esters (FAMES) the sediment is resuspended in distilled water, heated for 10 minutes at 100°C, cooled on ice and recentrifuged, followed by extraction for one hour at 90°C in 0.5 M sulfuric acid in methanol with 2% dimethoxypropane, which leads to hydrolyzed oil and lipid compounds, which give transmethylated lipids.
The FAMES are then extracted twice with 2 ml petrolether, washed once with 100 mM NaHCO₃, pH 8.0 and once with distilled water and dried with Na₂SO₄. The organic solvent can be evaporated under a stream of Argon and the FAMES were dissolved in 50 µl of petrolether. The samples can be separated on a ZEBRON ZB-Wax capillary column (30 m, 0,32 mm, 0,25 µm; Phenomenex) in a Hewlett Packard 6850 gas chromatograph with a flame ionisation detector. The oven temperature is programmed from 70°C (1 min. hold) to 200°C at a rate of 20°C/min., then to 250°C (5 min. hold) at a rate of 5°C/min and finally to 260°C at a rate of 5°C/min. Nitrogen is used as carrier gas (4,5 ml/min. at 70°C). The identity of the resulting fatty acid methyl esters can be identified by comparison with retention times of FAME standards, which are available from commercial sources (i.e. Sigma).
Plant material is initially homogenized mechanically by comminuting in a pestle and mortar to make it more amenable to extraction.
This is followed by heating at 100°C for 10 minutes and, after cooling on ice, by resedimentation. The cell sediment is hydrolyzed for one hour at 90°C with 1 M methanolic sulfuric acid and 2% dimethoxypropane, and the lipids are transmethylated. The resulting fatty acid methyl esters (FAMEs) are extracted in petroleum ether. The extracted FAMEs are analyzed by gas liquid chromatography using a capillary column (Chrompack, WCOT Fused Silica, CP-Wax-52 CB, 25 m, 0.32 mm) and a temperature gradient of from 170°C to 240°C in 20 minutes and 5 minutes at 240°C. The identity of the fatty acid methyl esters is confirmed by comparison with corresponding FAME standards (Sigma). The identity and position of the double bond can be analyzed further by suitable chemical derivatization of the FAME mixtures, for example to give 4,4-dimethoxyoxazoline derivatives (Christie, 1998) by means of GC-MS.
The methodology is described for example in Napier and Michaelson, 2001, Lipids. 36(8):761-766; Sayanova et al., 2001, Journal of Experimental Botany. 52(360):1581-1585, Sperling et al., 2001, Arch. Biochem. Biophys. 388(2):293-298 and Michaelson et al., 1998, FEBS Letters. 439(3):215-218.

**[0459.0.5.5]** If required and desired, further chromatography steps with a suitable resin may follow. Advantageously the fatty acids can be further purified with a so-called RTHPLC. As eluent different an acetonitrile/water or chloroform/acetonitrile mixtures are advantageously is used. For example canola oil can be separated said HPLC method using an RP-18-column (ET 250/3 Nucleosil 120-5 C₁₈ Macherey und Nagel, Düren, Germany). A chloroform/acetonitrile mixture (v/v 30:70) is used as eluent. The flow rate is beneficial 0.8 ml/min. For the analysis of the fatty acids an ELSD detector (evaporative light-scattering detector) is used. MPLC, dry-flash chromatography or thin layer chromatography are other beneficial chromatography methods for the purification of fatty acids. If necessary, these chromatography steps may be repeated, using identical or other chromatography resins. The skilled worker is familiar with the selection of suitable chromatography resin and the most effective use for a particular molecule to be purified.

**[0460.0.5.5]** In addition depending on the produced fine chemical purification is also possible with cristalisation or destilation. Both methods are well known to a person skilled in the art.

**[0461.0.5.5]** Example 10: Cloning SEQ ID NO: 4464 for the expression in plants

**[0462.0.0.5]** for the disclosure of this paragraph see [0462.0.0.0] above.

**[0463.0.5.5]** SEQ ID NO: 4464 is amplified by PCR as described in the protocol of the Pfu Turbo or DNA Herculase polymerase (Stratagene).

**[0464.0.5.5]** The composition for the protocol of the *Pfu* Turbo DNA polymerase was as follows: 1x PCR buffer (Stratagene), 0.2 mM of each dNTP, 100 ng genomic DNA of *Saccharomyces cerevisiae* (strain S288C; Research Genetics, Inc., now Invitrogen) or *Escherichia coli* (strain MG1655; *E.coli* Genetic Stock Center), 50 pmol forward primer, 50 pmol reverse primer, 2.5 u *Pfu* Turbo DNA polymerase. The amplification cycles were as follows:

**[0465.0.5.5]** 1 cycle of 3 minutes at 94-95°C, followed by 25-36 cycles of in each case 1 minute at 95°C or 30 seconds at 94°C, 45 seconds at 50°C, 30 seconds at 50°C or 30 seconds at 55°C and 210-480 seconds at 72°C, followed by 1 cycle of 8 minutes at 72°C, then 4°C.

**[0466.0.0.5]** for the disclosure of this paragraph see [0466.0.0.0] above.

**[0467.0.5.5]** The following primer sequences were selected for the gene SEQ ID NO: 4464:
i) forward primer (SEQ ID NO: 4556)
   atgggacaca agcccttata ccg
ii) reverse primer (SEQ ID NO: 4557)
   ttatcgcgat gattttcgct gcg

**[0468.0.0.5] to [0479.0.0.5]** for the disclosure of the paragraphs [0468.0.0.5] to [0479.0.0.5] see paragraphs [0468.0.0.0] to [0479.0.0.0] above.

**[0480.0.5.5]** Example 11: Generation of transgenic plants which express SEQ ID NO: 4464

**[0481.0.0.5]** for the disclosure of this paragraph see [0481.0.0.0] above.

**[0482.0.0.5] to [0513.0.0.5]** for the disclosure of the paragraphs [0482.0.0.5] to [0513.0.0.5] see paragraphs [0482.0.0.0] to [0513.0.0.0] above.

**[0514.0.5.5]** As an alternative, the fatty acids can be detected advantageously via HPLC separation in ethanolic extract as described by Geigenberger et al. (Plant Cell & Environ, 19, 1996: 43-55).
The results of the different plant analyses can be seen from the table which follows:

**Table 1**

| **ORF** | Metabolite | Method | Min | Max |
|---|---|---|---|---|
| b0730 | Linoleic Acid (C18:2 (c9,c12)) | GC | 1,13 | 1,34 |
| b3256 | Linoleic Acid (C18:2 (c9,c12)) | GC | 1,13 | 1,20 |
| YBR089C-A | Linoleic Acid (C18:2 (c9,c12)) | GC | 1,36 | 1,70 |
| YDR447C | Linoleic Acid (C18:2 (c9,c12)) | GC | 1,18 | 2,40 |
| YOR024W | Linoleic Acid (C18:2 (c9,c12)) | GC | 1,16 | 1,33 |
| b0050 | Linoleic acid (C18:cis[9,12]2) | GC | 1.15 | 1.37 |
| b0251 | Linoleic acid (C18:cis[9,12]2) | GC | 1.14 | 1.31 |
| b0255 | Linoleic acid (C18:cis[9,12]2) | GC | 1.22 | 1.31 |
| b0577 | Linoleic acid (C18:cis[9,12]2) | GC | 1.17 | 1.40 |
| b0849 | Linoleic acid (C18:cis[9,12]2) | GC | 1.19 | 1.34 |
| b1097 | Linoleic acid (C18:cis[9,12]2) | GC | 1.15 | 1.30 |
| b1693 | Linoleic acid (C18:cis[9,12]2) | GC | 1.17 | 1.32 |
| b2710 | Linoleic acid (C18:cis[9,12]2) | GC | 1.15 | 1.29 |
| b2822 | Linoleic acid (C18:cis[9,12]2) | GC | 1.15 | 1.27 |
| b3064 | Linoleic acid (C18:cis[9,12]2) | GC | 1.17 | 1.30 |
| b3166 | Linoleic acid (C18:cis[9,12]2) | GC | 1.14 | 1.21 |
| b3457 | Linoleic acid (C18:cis[9,12]2) | GC | 1.20 | 1.44 |
| b3644 | Linoleic acid (C18:cis[9,12]2) | GC | 1.14 | 1.35 |
| b4129 | Linoleic acid (C18:cis[9,12]2) | GC | 1.20 | 1.47 |

**[0515.0.5.5]** Column 2 shows the fatty acid analyzed. Columns 4 and 5 shows the ratio of the analyzed fatty acid between the transgenic plants and the wild type; Increase of the metabolites: Max: maximal x-fold (normalised to wild type)-Min: minimal x-fold (normalised to wild type). Decrease of the metabolites: Max: maximal x-fold (normalised to wild type) (minimal decrease), Min: minimal x-fold (normalised to wild type) (maximal decrease). Column 3 indicates the analytical method.

**[0516.0.0.5] and [0517.0.5.5]** for the disclosure of the paragraphs [0516.0.0.5] and [0517.0.5.5] see paragraphs [0516.0.0.0] and [0517.0.0.0] above.

**[0518.0.0.5] to [0529.0.0.5] and [0530.0.5.5]** for the disclosure of the paragraphs [0518.0.0.5] to [0529.0.0.5] and [0530.0.5.5] see paragraphs [0518.0.0.0] to [0530.0.0.0] above.

**[0530.1.0.5] to [0530.6.0.5]** for the disclosure of the paragraphs [0530.1.0.5] to [0530.6.0.5] see paragraphs [0530.1.0.0] to [0530.6.0.0] above.

**[0531.0.0.5] to [0533.0.0.5] and [0534.0.5.5]** for the disclosure of the paragraphs [0531.0.0.5] to [0533.0.0.5] and [0534.0.5.5] see paragraphs [0531.0.0.0] to [0534.0.0.0] above.

**[0535.0.0.5] to [0537.0.0.5] and [0538.0.5.5]** for the disclosure of the paragraphs [0535.0.0.5] to [0537.0.0.5] and [0538.0.5.5] see paragraphs [0535.0.0.0] to [0538.0.0.0] above.

**[0539.0.0.5] to [0542.0.0.5] and [0543.0.5.5]** for the disclosure of the paragraphs [0539.0.0.5] to [0542.0.0.5] and [0543.0.5.5] see paragraphs [0539.0.0.0] to [0543.0.0.0] above.

**[0544.0.0.5] to [0547.0.0.5] and [0548.0.5.5] to [0552.0.0.5]** for the disclosure of the paragraphs [0544.0.0.5] to [0547.0.0.5] and [0548.0.5.5] to [0552.0.0.5] see paragraphs [0544.0.0.0] to [0552.0.0.0] above.

**[0552.2.0.5]** for the disclosure of this paragraph see [0552.2.0.0] above.

**[0553.0.5.5]**
1. A process for the production of linoleic acid, which comprises
   (a) increasing or generating the activity of a protein as indicated in Table II, columns 5 or 7, lines 63 to 67 and 436 to 449 or a functional equivalent thereof in a non-human organism, or in one or more parts thereof; and
   (b) growing the organism under conditions which permit the production of linoleic acid in said organism.
2. A process for the production of linoleic acid, comprising the increasing or generating in an organism or a part thereof the expression of at least one nucleic acid molecule comprising a nucleic acid molecule selected from the group consisting of:
   a) nucleic acid molecule encoding of a polypeptide as indicated in Table II, columns 5 or 7, lines 63 to 67 and 436 to 449 or a fragment thereof, which confers an increase in the amount of linoleic acid in an organism or a part thereof;
   b) nucleic acid molecule comprising of a nucleic acid molecule as indicated in Table I, columns 5 or 7, lines 63 to 67 and 436 to 449;
   c) nucleic acid molecule whose sequence can be deduced from a polypeptide sequence encoded by a nucleic acid molecule of (a) or (b) as a result of the degeneracy of the genetic code and conferring an increase in the amount of linoleic acid in an organism or a part thereof;
   d) nucleic acid molecule which encodes a polypeptide which has at least 50% identity with the amino acid sequence of the polypeptide encoded by the nucleic acid molecule of (a) to (c) and conferring an increase in the amount of linoleic acid in an organism or a part thereof;
   e) nucleic acid molecule which hybidizes with a nucleic acid molecule of (a) to (c) under stringent hybridisation conditions and conferring an increase in the amount of linoleic acid in an organism or a part thereof;
   f) nucleic acid molecule which encompasses a nucleic acid molecule which is obtained by amplifying nucleic acid molecules from a cDNA library or a genomic library using the primers or primer pairs as indicated in Table III, column 7, lines 63 to 67 and 436 to 449 and conferring an increase in the amount of linoleic acid in an organism or a part thereof;
   g) nucleic acid molecule encoding a polypeptide which is isolated with the aid of monoclonal antibodies against a polypeptide encoded by one of the nucleic acid molecules of (a) to (f) and conferring an increase in the amount of linoleic acid in an organism or a part thereof;
   h) nucleic acid molecule encoding a polypeptide comprising a consensus sequence as indicated in Table IV, column 7, lines 63 to 67 and 436 to 449 and conferring an increase in the amount of linoleic acid in an organism or a part thereof; and
   i) nucleic acid molecule which is obtainable by screening a suitable nucleic acid library under stringent hybridization conditions with a probe comprising one of the sequences of the nucleic acid molecule of (a) to (k) or with a fragment thereof having at least 15 nt, preferably 20 nt, 30 nt, 50 nt, 100 nt, 200 nt or 500 nt of the nucleic acid molecule characterized in (a) to (k) and conferring an increase in the amount of linoleic acid in an organism or a part thereof.
   or comprising a sequence which is complementary thereto.
3. The process of claim 1 or 2, comprising recovering of the free or bound linoleic acid.
4. The process of any one of claim 1 to 3, comprising the following steps:
   (a) selecting an organism or a part thereof expressing a polypeptide encoded by the nucleic acid molecule characterized in claim 2;
   (b) mutagenizing the selected organism or the part thereof;
   (c) comparing the activity or the expression level of said polypeptide in the mutagenized organism or the part thereof with the activity or the expression of said polypeptide of the selected organisms or the part thereof;
   (d) selecting the mutated organisms or parts thereof, which comprise an increased activity or expression level of said polypeptide compared to the selected organism or the part thereof;
   (e) optionally, growing and cultivating the organisms or the parts thereof; and
   (f) recovering, and optionally isolating, the free or bound linoleic acid produced by the selected mutated organisms or parts thereof.
5. The process of any one of claims 1 to 4, wherein the activity of said protein or the expression of said nucleic acid molecule is increased or generated transiently or stably.
6. An isolated nucleic acid molecule comprising a nucleic acid molecule selected from the group consisting of:
   a) nucleic acid molecule encoding of a polypeptide as indicated in Table II, columns 5 or 7, lines 63 to 67 and 436 to 449 or a fragment thereof, which confers an increase in the amount of linoleic acid in an organism or a part thereof;
   b) nucleic acid molecule comprising of a nucleic acid molecule as indicated in Table I, columns 5 or 7, lines 63 to 67 and 436 to 449;
   c) nucleic acid molecule whose sequence can be deduced from a polypeptide sequence encoded by a nucleic acid molecule of (a) or (b) as a result of the degeneracy of the genetic code and conferring an increase in the amount of linoleic acid in an organism or a part thereof;
   d) nucleic acid molecule which encodes a polypeptide which has at least 50% identity with the amino acid sequence of the polypeptide encoded by the nucleic acid molecule of (a) to (c) and conferring an increase in the amount of linoleic acid in an organism or a part thereof;
   e) nucleic acid molecule which hybidizes with a nucleic acid molecule of (a) to (c) under stringent hybridisation conditions and conferring an increase in the amount of linoleic acid in an organism or a part thereof;
   f) nucleic acid molecule which encompasses a nucleic acid molecule which is obtained by amplifying nucleic acid molecules from a cDNA library or a genomic library using the primers or primer pairs as indicated in Table III, columns 5 or 7, lines 63 to 67 and 436 to 449 and conferring an increase in the amount of the respective fine chemical in an organism or a part thereof;
   g) nucleic acid molecule encoding a polypeptide which is isolated with the aid of monoclonal antibodies against a polypeptide encoded by one of the nucleic acid molecules of (a) to (f) and conferring an increase in the amount of linoleic acid in an organism or a part thereof;
   h) nucleic acid molecule encoding a polypeptide comprising a consensus sequence as indicated in Table IV, column 7, lines 63 to 67 and 436 to 449 and conferring an increase in the amount of linoleic acid in an organism or a part thereof; and
   i) nucleic acid molecule which is obtainable by screening a suitable nucleic acid library under stringent hybridization conditions with a probe comprising one of the sequences of the nucleic acid molecule of (a) to (k) or with a fragment thereof having at least 15 nt, preferably 20 nt, 30 nt, 50 nt, 100 nt, 200 nt or 500 nt of the nucleic acid molecule characterized in (a) to (k) and conferring an increase in the amount of linoleic acid in an organism or a part thereof.
   whereby the nucleic acid molecule distinguishes over the sequence as indicated in Table I A, columns 5 or 7, lines 63 to 67 and 436 to 449 by one or more nucleotides.
7. A nucleic acid construct which confers the expression of the nucleic acid molecule of claim 6, comprising one or more regulatory elements.
8. A vector comprising the nucleic acid molecule as claimed in claim 6 or the nucleic acid construct of claim 7.
9. The vector as claimed in claim 8, wherein the nucleic acid molecule is in operable linkage with regulatory sequences for the expression in a prokaryotic or eukaryotic, or in a prokaryotic and eukaryotic, host.
10. A host cell, which has been transformed stably or transiently with the vector as claimed in claim 8 or 9 or the nucleic acid molecule as claimed in claim 6 or the nucleic acid construct of claim 7 or produced as described in claim any one of claims 2 to 5.
11. The host cell of claim 10, which is a transgenic host cell.
12. The host cell of claim 10 or 11, which is a plant cell, an animal cell, a microorganism, or a yeast cell, a fungus cell, a prokaryotic cell, an eukaryotic cell or an archaebacterium.
13. A process for producing a polypeptide, wherein the polypeptide is expressed in a host cell as claimed in any one of claims 10 to 12.
14. A polypeptide produced by the process as claimed in claim 13 or encoded by the nucleic acid molecule as claimed in claim 6 whereby the polypeptide distinguishes over a sequence as indicated in Table II A, columns 5 or 7, lines 63 to 67 and 436 to 449 by one or more amino acids
15. An antibody, which binds specifically to the polypeptide as claimed in claim 14.
16. A plant tissue, propagation material, harvested material or a plant comprising the host cell as claimed in claim 12 which is plant cell or an Agrobacterium.
17. A method for screening for agonists and antagonists of the activity of a polypeptide encoded by the nucleic acid molecule of claim 6 conferring an increase in the amount of linoleic acid in an organism or a part thereof comprising:
   (a) contacting cells, tissues , plants or microorganisms which express the a polypeptide encoded by the nucleic acid molecule of claim 5 conferring an increase in the amount of linoleic acid in an organism or a part thereof with a candidate compound or a sample comprising a plurality of compounds under conditions which permit the expression the polypeptide;
   (b) assaying the linoleic acid level or the polypeptide expression level in the cell, tissue, plant or microorganism or the media the cell, tissue, plant or microorganisms is cultured or maintained in; and
   (c) identifying a agonist or antagonist by comparing the measured linoleic acid level or polypeptide expression level with a standard of linoleic acid or polypeptide expression level measured in the absence of said candidate compound or a sample comprising said plurality of compounds, whereby an increased level over the standard indicates that the compound or the sample comprising said plurality of compounds is an agonist and a decreased level over the standard indicates that the compound or the sample comprising said plurality of compounds is an antagonist.
18. A process for the identification of a compound conferring increased linoleic acid production in a plant or microorganism, comprising the steps:
   (a) culturing a plant cell or tissue or microorganism or maintaining a plant expressing the polypeptide encoded by the nucleic acid molecule of claim 6 conferring an increase in the amount of linoleic acid in an organism or a part thereof and a readout system capable of interacting with the polypeptide under suitable conditions which permit the interaction of the polypeptide with dais readout system in the presence of a compound or a sample comprising a plurality of compounds and capable of providing a detectable signal in response to the binding of a compound to said polypeptide under conditions which permit the expression of said readout system and of the polypeptide encoded by the nucleic acid molecule of claim 6 conferring an increase in the amount of linoleic acid in an organism or a part thereof;
   (b) identifying if the compound is an effective agonist by detecting the presence or absence or increase of a signal produced by said readout system.
19. A method for the identification of a gene product conferring an increase in linoleic acid production in a cell, comprising the following steps:
   (a) contacting the nucleic acid molecules of a sample, which can contain a candidate gene encoding a gene product conferring an increase in linoleic acid after expression with the nucleic acid molecule of claim 6;
   (b) identifying the nucleic acid molecules, which hybridise under relaxed stringent conditions with the nucleic acid molecule of claim 6;
   (c) introducing the candidate nucleic acid molecules in host cells appropriate for producing linoleic acid;
   (d) expressing the identified nucleic acid molecules in the host cells;
   (e) assaying the linoleic acid level in the host cells; and
   (f) identifying nucleic acid molecule and its gene product which expression confers an increase in the linoleic acid level in the host cell in the host cell after expression compared to the wild type.
20. A method for the identification of a gene product conferring an increase in linoleic acid production in a cell, comprising the following steps:
   (a) identifiying in a data bank nucleic acid molecules of an organism; which can contain a candidate gene encoding a gene product conferring an increase in the linoleic acid amount or level in an organism or a part thereof after expression, and which are at least 20% homolog to the nucleic acid molecule of claim 6;
   (b) introducing the candidate nucleic acid molecules in host cells appropriate for producing linoleic acid;
   (c) expressing the identified nucleic acid molecules in the host cells;
   (d) assaying the linoleic acid level in the host cells; and
   (e) identifying nucleic acid molecule and its gene product which expression confers an increase in the linoleic acid level in the host cell after expression compared to the wild type.
21. A method for the production of an agricultural composition comprising the steps of the method of any one of claims 17 to 20 and formulating the compound identified in any one of claims 17 to 20 in a form acceptable for an application in agriculture.
22. A composition comprising the nucleic acid molecule of claim 6, the polypeptide of claim 14, the nucleic acid construct of claim 7, the vector of any one of claims 8 or 9, an antagonist or agonist identified according to claim 17, the compound of claim 18, the gene product of claim 19 or 20, the antibody of claim 15, and optionally an agricultural acceptable carrier.
23. Use of the nucleic acid molecule as claimed in claim 6 for the identification of a nucleic acid molecule conferring an increase of linoleic acid after expression.
24. Use of the polypeptide of claim 14 or the nucleic acid construct claim 7 or the gene product identified according to the method of claim 19 or 20 for identifying compounds capable of conferring a modulation of linoleic acid levels in an organism.
25. Food or feed composition comprising the nucleic acid molecule of claim 6, the polypeptide of claim 14, the nucleic acid construct of claim 7, the vector of claim 8 or 9, the antagonist or agonist identified according to claim 17, the antibody of claim 15, the plant or plant tissue of claim 16, the harvested material of claim 16, the host cell of claim 10 to 12 or the gene product identified according to the method of claim 19 or 20.
26. Use of the nucleic acid molecule of claim 6, the polypeptide of claim 14, the nucleic acid construct of claim 7, the vector of claim 8 or 9, the antagonist or agonist identified according to claim 17, the antibody of claim 15, the plant or plant tissue of claim 16, the host cell of claim 10 to 12 or the gene product identified according to the method of claim 19 or 20 for the protection of a plant against a linoleic acid synthesis inhibiting herbicide.

**[0554.0.0.5] Abstract: see [0554.0.0.0]**
Process for the production of fine chemicals

**[0000.0.0.6]** In a further embodiment, the present invention relates to a further process for the production of fine chemicals as defined below and the corresponding embodiments as described herein as follows.

**[0001.0.0.6]** for the disclosure of this paragraph see [0001.0.0.0].

**[0002.0.5.6] to [0008.0.5.6]** for the disclosure of the paragraphs [0002.0.5.6] to [0008.0.5.6] see [0002.0.0.0] and [0008.0.0.0] above.

**[0009.0.6.6]** As described above, the essential fatty acids are necessary for humans and many mammals, for example for livestock. Essential fatty acids, such as alpha-linolenic acid, are extremely important for healing and maintaining good health. Compounds made from alpha-linolenic acid have been shown to decrease blood clotting and decrease inflammatory processes in the body.

**[0010.0.6.6] to [0012.0.6.6]** for the disclosure of the paragraphs [0010.0.6.6] to [0012.0.6.6] see [0010.0.0.0] and [0012.0.0.0] above.

**[0013.0.0.6]** for the disclosure of this paragraph see [0013.0.0.0] above.

**[0014.0.6.6]** Accordingly, in a first embodiment, the invention relates to a process for the production of a fine chemical, whereby the fine chemical is α-linolenic acid or tryglycerides, lipids, oils or fats containing α-linolenic acid. Accordingly, in the present invention, the term "the fine chemical" as used herein relates to "α-linolenic acid and/or tryglycerides, lipids, oils and/or fats containing α-linolenic acid". Further, the term "the fine chemicals" as used herein also relates to fine chemicals comprising α-linolenic acid and/or triglycerides, lipids, oils and/or fats containing α-linolenic acid.

**[0015.0.6.6]** In one embodiment, the term "the fine chemical" means α-linolenic acid and/or tryglycerides, lipids, oils and/or fats containing α-inolenic acid. Throughout the specification the term "the fine chemical" means α-linolenic acid and/or tryglycerides, lipids, oils and/or fats containing α-linolenic acid, α-linolenic acid and its salts, ester, thioester or α-linolenic acid in free form or bound to other compounds such as triglycerides, glycolipids, phospholipids etc. In a preferred embodiment, the term "the fine chemical" means α-linolenic acid, in free form or its salts or bound to triglycerides. Triglycerides, lipids, oils, fats or lipid mixture thereof shall mean any triglyceride, lipid, oil and/or fat containing any bound or free α-linolenic acid for example sphingolipids, phosphoglycerides, lipids, glycolipids such as glycosphingolipids, phospholipids such as phosphatidylethanolamine, phosphatidylcholine, phosphatidylserine, phosphatidylglycerol, phosphatidylinositol or diphosphatidylglycerol, or as monoacylglyceride, diacylglyceride or triacylglyceride or other fatty acid esters such as acetyl-Coenzym A thioester, which contain further saturated or unsaturated fatty acids in the fatty acid molecule.

**[0016.0.6.6]** Accordingly, the present invention relates to a process comprising
(a) increasing or generating the activity of a b2699, YER173W, YGL205W, YIL150C, b0050, b0251, b0376, b0577, b0849, b2822, b3129, b3457, b3462 and/or b3644 protein(s) or of a protein having the sequence of a polypeptide encoded by a nucleic acid molecule indicated in Table I, columns 5 or 7, lines 68 to 71 and 450 to 459 in a non-human organism in one or more parts thereof and
(b) growing the organism under conditions which permit the production of the fine chemical, thus, α-linolenic acid or fine chemicals comprising α-linolenic acid, in said organism.
Accordingly, the present invention relates to a process for the production of a fine chemical comprising
(a) increasing or generating the activity of one or more proteins having the activity of a protein indicated in Table II, column 3, lines 68 to 71 and 450 to 459 or having the sequence of a polypeptide encoded by a nucleic acid molecule indicated in Table I, column 5 or 7, lines 68 to 71 and 450 to 459, in a non-human organism in one or more parts thereof and
(b) growing the organism under conditions which permit the production of the fine chemical, in particular α-linolenic acid.

**[0017.0.0.6] and [0018.0.0.6]** for the disclosure of the paragraphs [0017.0.0.6] and [0018.0.0.6] see paragraphs [0017.0.0.0] and [0018.0.0.0] above.

**[0019.0.6.6]** Advantageously the process for the production of the fine chemical leads to an enhanced production of the fine chemical. The terms "enhanced" or "increase" mean at least a 10%, 20%, 30%, 40% or 50%, preferably at least 60%, 70%, 80%, 90% or 100%, more preferably 150%, 200%, 300%, 400% or 500% higher production of the fine chemical in comparison to the reference as defined below, e.g. that means in comparison to an organism without the aforementioned modification of the activity of a protein having the activity of a protein indicated in Table II, column 3, lines 68 to 71 and 450 to 459 or encoded by nucleic acid molecule indicated in Table I, columns 5 or 7, lines 68 to 71 and 450 to 459.

**[0020.0.6.6]** Surprisingly it was found, that the transgenic expression of at least one of the Saccaromyces cerevisiae protein(s) indicated in Table II, Column 3, lines 69 to 71, and/or the Escherichia coli K12 protein(s) indicated in Table II, Column 3, lines 68 and 450 to 459 in Arabidopsis thaliana conferred an increase in the α-linolenic acid (or fine chemical) content of the transformed plants.

**[0021.0.0.6]** for the disclosure of this paragraph see [0021.0.0.0] above.

**[0022.0.6.6]** The sequence of b2699 from Escherichia coli K12 has been published in Blattner et al., Science 277(5331), 1453-1474, 1997, and its activity is being defined as DNA-dependent ATPase or DNA- and ATP-dependent coprotease. Accordingly, in one embodiment, the process of the present invention comprises the use of a DNA-dependent ATPase or DNA- and ATP-dependent coprotease from E. coli or its homolog, e.g. as shown herein, for the production of the fine chemical, meaning of α-linolenic acid and/or tryglycerides, lipids, oils and/or fats containing α-linolenic acid, in particular for increasing the amount of α-linolenic acid and/or tryglycerides, lipids, oils and/or fats containing α-linolenic acid, preferably α-linolenic acid in free or bound form in an organism or a part thereof, as mentioned. In one embodiment, in the process of the present invention the activity of a DNA-dependent ATPase or DNA- and ATP-dependent coprotease is increased or generated, e.g. from E. coli or a homolog thereof.
The sequence of YER173W from Saccharomyces cerevisiae has been published in Dietrich et al., Nature 387 (6632 Suppl), 78-81 (1997) and Goffeau et al., Science 274 (5287), 546-547, 1996, and its cellular activity has not been characterized yet. It seams to be a "checkpoint protein, involved in the activation of the DNA damage and meiotic pachytene checkpoints; subunit of a clamp loader that loads Rad17p-Mec3p-Ddc1p onto DNA. Its properly a homolog of human and S. pombe Rad17 protein". Accordingly, in one embodiment, the process of the present invention comprises the use of a YER173W activity from Saccharomyces cerevisiae or its homolog, e.g. as shown herein, for the production of the fine chemical, meaning of α-linolenic acid and/or tryglycerides, lipids, oils and/or fats containing α-linolenic acid, in particular for increasing the amount of α-linolenic acid and/or tryglycerides, lipids, oils and/or fats containing α-linolenic acid, preferably α-linolenic acid in free or bound form in an organism or a part thereof, as mentioned. In one embodiment, in the process of the present invention the activity of a YER173W protein is increased or generated, e.g. from Saccharomyces cerevisiae or a homolog thereof.
The sequence of YGL205W from Saccharomyces cerevisiae has been published in Tettelin et al., Nature 387 (6632 Suppl), 81-84 (1997), and Goffeau et al., Science 274 (5287), 546-547, 1996, and its activity is being defined as a "fatty-acyl coenzyme A oxidase, which is involved in the fatty acid beta-oxidation pathway; localized to the peroxisomal matrix". Accordingly, in one embodiment, the process of the present invention comprises the use of a "fatty-acyl coenzyme A oxidase" or its homolog, e.g. as shown herein, for the production of the fine chemical, meaning of α-linolenic acid and/or tryglycerides, lipids, oils and/or fats containing α-linolenic acid, in particular for increasing the amount of α-linolenic acid and/or tryglycerides, lipids, oils and/or fats containing α-linolenic acid, preferably α-linolenic acid in free or bound form in an organism or a part thereof, as mentioned. In one embodiment, in the process of the present invention the activity of a,fatty-acyl coenzyme A oxidase is increased or generated, e.g. from Saccharomyces cerevisiae or a homolog thereof.
The sequence of YIL150C from Saccharomyces cerevisiae has been published in Churcher et al., Nature 387 (6632 Suppl), 84-87 (1997) and in Goffeau et al., Science 274 (5287), 546-547, 1996 and its cellular activity is being defined as "chromatin binding protein, required for S-phase (DNA synthesis) initation or completion". Accordingly, in one embodiment, the process of the present invention comprises the use of a chromatin binding protein, from Saccharomyces cerevisiae or its homolog, e.g. as shown herein, for the production of the fine chemical, meaning of α-linolenic acid and/or tryglycerides, lipids, oils and/or fats containing α-linolenic acid, in particular for increasing the amount of α-linolenic acid and/or tryglycerides, lipids, oils and/or fats containing α-linolenic acid, preferably α-linolenic acid in free or bound form in an organism or a part thereof, as mentioned. In one embodiment, in the process of the present invention the activity of a chromatin binding protein is increased or generated, e.g. from Saccharomyces cerevisiae or a homolog thereof.
The sequence of b0050 (Accession number NP_414592) from Escherichia coli K12 has been published in Blattner et al., Science 277 (5331), 1453-1474, 1997, and its activity is being defined as a conserved protein potentially involved in protein protein interaction. Accordingly, in one embodiment, the process of the present invention comprises the use of said protein from E. coli or its homolog, e.g. as shown herein, for the production of the fine chemical, meaning of α-linolenic acid and/or tryglycerides, lipids, oils and/or fats containing α-linolenic acid, in particular for increasing the amount of α-linolenic acid and/or tryglycerides, lipids, oils and/or fats containing α-linolenic acid, preferably α-linolenic acid in free or bound form in an organism or a part thereof, as mentioned. In one embodiment, in the process of the present invention the activity of said protein is increased or generated, e.g. from E. coli or a homolog thereof.
The sequence of b0251 (Accession number NP_414785) from Escherichia coli K12 has been published in Blattner et al., Science 277 (5331), 1453-1474, 1997, and its activity is being defined as putative HTH-type transcriptional regulator. Accordingly, in one embodiment, the process of the present invention comprises the use of a putative HTH-type transcriptional regulator protein from E. coli or its homolog, e.g. as shown herein, for the production of the fine chemical, meaning of α-linolenic acid and/or tryglycerides, lipids, oils and/or fats containing α-linolenic acid, in particular for increasing the amount of α-linolenic acid and/or tryglycerides, lipids, oils and/or fats containing α-linolenic acid, preferably α-linolenic acid in free or bound form in an organism or a part thereof, as mentioned. In one embodiment, in the process of the present invention the activity of a putative HTH-type transcriptional regulator protein is increased or generated, e.g. from E. coli or a homolog thereof.
The sequence of b0376 (Accession number NP_414910) from Escherichia coli K12 has been published in Blattner et al., Science 277 (5331), 1453-1474, 1997, and its activity is being defined as a beta-lactamase/D-ala carboxypeptidase, penicillin binding protein. Accordingly, in one embodiment, the process of the present invention comprises the use of a beta-lactamase/D-ala carboxypeptidase, penicillin binding protein from E. coli or its homolog, e.g. as shown herein, for the production of the fine chemical, meaning of α-linolenic acid and/or tryglycerides, lipids, oils and/or fats containing α-linolenic acid, in particular for increasing the amount of α-linolenic acid and/or tryglycerides, lipids, oils and/or fats containing α-linolenic acid, preferably α-linolenic acid in free or bound form in an organism or a part thereof, as mentioned. In one embodiment, in the process of the present invention the activity of a beta-lactamase/D-ala carboxypeptidase, penicillin binding protein is increased or generated, e.g. from E. coli or a homolog thereof.
The sequence of b0577 (Accession number NP_415109) from Escherichia coli K12 has been published in Blattner et al., Science 277 (5331), 1453-1474, 1997, and its activity is being defined as a putative transport protein. Accordingly, in one embodiment, the process of the present invention comprises the use of a putative transport protein from E. coli or its homolog, e.g. as shown herein, for the production of the fine chemical, meaning of α-linolenic acid and/or tryglycerides, lipids, oils and/or fats containing α-linolenic acid, in particular for increasing the amount of α-linolenic acid and/or tryglycerides, lipids, oils and/or fats containing α-linolenic acid, preferably α-linolenic acid in free or bound form in an organism or a part thereof, as mentioned. In one embodiment, in the process of the present invention the activity of a putative transport protein is increased or generated, e.g. from E. coli or a homolog thereof.
The sequence of b0849 (Accession number NP_415370) from Escherichia coli K12 has been published in Blattner et al., Science 277 (5331), 1453-1474, 1997, and its activity is being defined as a glutaredoxin 1 redox coenzyme for glutathione-dependent ribonucleotide reductase. Accordingly, in one embodiment, the process of the present invention comprises the use of a glutaredoxin 1 redox coenzyme for glutathione-dependent ribonucleotide reductase protein from E. coli or its homolog, e.g. as shown herein, for the production of the fine chemical, meaning of α-linolenic acid and/or tryglycerides, lipids, oils and/or fats containing α-linolenic acid, in particular for increasing the amount of α-linolenic acid and/or tryglycerides, lipids, oils and/or fats containing α-linolenic acid, preferably α-linolenic acid in free or bound form in an organism or a part thereof, as mentioned. In one embodiment, in the process of the present invention the activity of a glutaredoxin 1 redox coenzyme for glutathione-dependent ribonucleotide reductase protein is increased or generated, e.g. from E. coli or a homolog thereof.
The sequence of b2822 (Accession number NP_417299) from Escherichia coli K12 has been published in Blattner et al., Science 277 (5331), 1453-1474, 1997, and its activity is being defined as a DNA helicase, ATP-dependent dsDNA/ssDNA exonuclease V subunit, ssDNA endonuclease. Accordingly, in one embodiment, the process of the present invention comprises the use of a DNA helicase, ATP-dependent dsDNA/ssDNA exonuclease V subunit, ssDNA endonuclease protein from E. coli or its homolog, e.g. as shown herein, for the production of the fine chemical, meaning of α-linolenic acid and/or tryglycerides, lipids, oils and/or fats containing α-linolenic acid, in particular for increasing the amount of α-linolenic acid and/or tryglycerides, lipids, oils and/or fats containing α-linolenic acid, preferably α-linolenic acid in free or bound form in an organism or a part thereof, as mentioned. In one embodiment, in the process of the present invention the activity of a DNA helicase, ATP-dependent dsDNA/ssDNA exonuclease V subunit, ssDNA endonuclease protein is increased or generated, e.g. from E. coli or a homolog thereof.
The sequence of b3129 (Accession number NP_417598) from Escherichia coli K12 has been published in Blattner et al., Science 277 (5331), 1453-1474, 1997, and its activity is being defined as a putative protease; htrA suppressor protein. Accordingly, in one embodiment, the process of the present invention comprises the use of a putative protease; htrA suppressor protein from E. coli or its homolog, e.g. as shown herein, for the production of the fine chemical, meaning of α-linolenic acid and/or tryglycerides, lipids, oils and/or fats containing α-linolenic acid, in particular for increasing the amount of α-linolenic acid and/or tryglycerides, lipids, oils and/or fats containing α-linolenic acid, preferably α-linolenic acid in free or bound form in an organism or a part thereof, as mentioned. In one embodiment, in the process of the present invention the activity of a putative protease; htrA suppressor protein is increased or generated, e.g. from E. coli or a homolog thereof.
The sequence of b3457 (Accession number NP_417914) from Escherichia coli K12 has been published in Blattner et al., Science 277 (5331), 1453-1474, 1997, and its activity is being defined as a high-affinity branched-chain amino acid transport protein (ABC superfamily). Accordingly, in one embodiment, the process of the present invention comprises the use of a high-affinity branched-chain amino acid transport protein from E. coli or its homolog, e.g. as shown herein, for the production of the fine chemical, meaning of α-linolenic acid and/or tryglycerides, lipids, oils and/or fats containing α-linolenic acid, in particular for increasing the amount of α-linolenic acid and/or tryglycerides, lipids, oils and/or fats containing α-linolenic acid, preferably α-linolenic acid in free or bound form in an organism or a part thereof, as mentioned. In one embodiment, in the process of the present invention the activity of a high-affinity branched-chain amino acid transport protein is increased or generated, e.g. from E. coli or a homolog thereof.
The sequence of b3462 (Accession number NP_417919) from Escherichia coli K12 has been published in Blattner et al., Science 277 (5331), 1453-1474, 1997, and its activity is being defined as a integral membrane cell division protein. Accordingly, in one embodiment, the process of the present invention comprises the use of a integral membrane cell division protein from E. coli or its homolog, e.g. as shown herein, for the production of the fine chemical, meaning of α-linolenic acid and/or tryglycerides, lipids, oils and/or fats containing α-linolenic acid, in particular for increasing the amount of α-linolenic acid and/or tryglycerides, lipids, oils and/or fats containing α-linolenic acid, preferably α-linolenic acid in free or bound form in an organism or a part thereof, as mentioned. In one embodiment, in the process of the present invention the activity of a integral membrane cell division protein is increased or generated, e.g. from E. coli or a homolog thereof.
The sequence of b3644 (Accession number NP_418101) from Escherichia coli K12 has been published in Blattner et al., Science 277 (5331), 1453-1474, 1997, and its activity is being defined as an uncharacterized stress-induced protein. Accordingly, in one embodiment, the process of the present invention comprises the use of an uncharacterized stress-induced protein from E. coli or its homolog, e.g. as shown herein, for the production of the fine chemical, meaning of α-linolenic acid and/or tryglycerides, lipids, oils and/or fats containing α-linolenic acid, in particular for increasing the amount of α-linolenic acid and/or tryglycerides, lipids, oils and/or fats containing α-linolenic acid, preferably α-linolenic acid in free or bound form in an organism or a part thereof, as mentioned. In one embodiment, in the process of the present invention the activity of an uncharacterized stress-induced protein is increased or generated, e.g. from E. coli or a homolog thereof.
**[0023.0.6.6]** Homologues ( = homologs) of the present gene products can be derived from any organisms as long as the homologue confers the herein mentioned activity, in particular, confers an increase in the fine chemical amount or content. Further, in the present invention, the term "homologue" relates to the sequence of an organism having the highest sequence homology to the herein mentioned or listed sequences of all expressed sequences of said organism.
However, the person skilled in the art knows, that, preferably, the homologue has said fine-chemical increasing activity and, if known, the same biological function or activity in the organism as at least one of the protein(s) indicated in Table II, Column 3, lines 68 to 71 and 450 to 459, e.g. having the sequence of a polypeptide encoded by a nucleic acid molecule comprising the sequence indicated in indicated in Table I, Column 5 or 7, lines 68 to 71 and 450 to 459.
In one embodiment, the homolog of any one of the polypeptides indicated in Table II, lines 69 to 71 is a homolog having the same or a similar acitivity, in particular an increase of activity confers an increase in the content of the fine chemical in the organsims and being derived from an Eukaryot. In one embodiment, the homolog of a polypeptide indicated in Table II, column 3, lines 68 and 450 to 459 is a homolog having the same or a similar acitivity, in particular an increase of activity confers an increase in the content of the fine chemical in the organisms or part thereof, and being derived from bacteria. In one embodiment, the homolog of a polypeptide indicated in Table II, column 3, lines 69 to 71 is a homolog having the same or a similar acitivity, in particular an increase of activity confers an increase in the content of the fine chemical in an organisms or part thereof, and being derived from Fungi. In one embodiment, the homolog of a polypeptide indicated in Table II, column 3, lines 68 and 450 to 459 is a homolog having the same or a similar acitivity, in particular an increase of activity confers an increase in the content of the fine chemical in the organsims or part thereof and being derived from Proteobacteria. In one embodiment, the homolog of a polypeptide indicated in Table II, column 3, lines 69 to 71 is a homolog having the same or a similar acitivity, in particular an increase of activity confers an increase in the content of the fine chemical in the organsims or a part thereof and being derived from Ascomycota. In one embodiment, the homolog of a polypeptide indicated in Table II, column 3, lines 68 and 450 to 459 is a homolog having the same or a similar acitivity, in particular an increase of activity confers an increase in the content of the fine chemical in the organisms or part thereof, and being derived from Gammaproteobacteria. In one embodiment, the homolog of a polypeptide polypeptide indicated in Table II, column 3, lines 69 to 71 is a homolog having the same or a similar acitivity, in particular an increase of activity confers an increase in the content of the fine chemical in the organisms or part thereof, and being derived from Saccharomycotina. In one embodiment, the homolog of a polypeptide indicated in Table II, column 3, lines 68 and 450 to 459 is a homolog having the samie or a similar acitivity, in particular an increase of activity confers an increase in the content of the fine chemical in the organisms or part thereof, and being derived from Enterobacteriales. In one embodiment, the homolog of a polypeptide indicated in Table II, column 3, lines 69 to 71 is a homolog having the samie or a similar acitivity, in particular an increase of activity confers an increase in the content of the fine chemical in the organisms or a part thereof, and being derived from Saccharomycetes. In one embodiment, the homolog of the a polypeptide indicated in Table II, column 3, lines 68 and 450 to 459 is a homolog having the samie or a similar acitivity, in particular an increase of activity confers an increase in the content of the fine chemical in the organisms or part thereof, and being derived from Enterobacteriaceae. In one embodiment, the homolog of a polypeptide indicated in Table II, column 3, lines 69 to 71 is a homolog having the same or a similar acitivity, in particular an increase of activity confers an increase in the content of the fine chemical in the organisms, and being derived from Saccharomycetales. In one embodiment, the homolog of a polypeptide indicated in Table II, column 3, lines 68 and 450 to 459 is a homolog having the same or a similar acitivity, in particular an increase of activity confers an increase in the content of the fine chemical in the organisms or a part thereof, and being derived from Escherichia. In one embodiment, the homolog of a polypeptide indicated in Table II, column 3, lines 69 to 71 is a homolog having the same or a similar acitivity, in particular an increase of activity confers an increase in the content of the fine chemical in the organisms or a part thereof, and being derived from Saccharomycetaceae. In one embodiment, the homolog of a polypeptide indicated in Table II, column 3, line 69 to 71 is a homolog having the same or a similar acitivity, in particular an increase of activity confers an increase in the content of the fine chemical in the organisms or a part thereof, and being derived from Saccharomycetes.

**[0023.1.0.6] and [0024.0.0.6]** for the disclosure of the paragraphs [0023.1.0.6] and [0024.0.0.6] see [0023.1.0.0] and [0024.0.0.0] above.

**[0025.0.6.6]** In accordance with the invention, a protein or polypeptide has the "activity of a protein as indicated in Table II, column 3, lines 68 to 71 and 450 to 459" if its *de novo* activity, or its increased expression directly or indirectly leads to an increased α-linolenic acid and/or tryglycerides, lipids, oils and/or fats containing α-linolenic acid level in the organism or a part thereof, preferably in a cell of said organism and the protein has the above mentioned activities of a protein as indicated in Table II, column 3, lines 68 to 71 and 450 to 459. Throughout the specification the activity or preferably the biological activity of such a protein or polypeptide or an nucleic acid molecule or sequence encoding such protein or polypeptide is identical or similar if it still has the biological or enzymatic activity of a protein as indicated in Table II, column 3, lines 68 to 71 and 450 to 459, or which has at least 10% of the original enzymatic activity, preferably 20%, particularly preferably 30%, most particularly preferably 40% in comparison to a protein of Saccharomyces cerevisiae as indicated in Table II, column 3, lines 69 to 71 and/or a protein of E. coli K12 as indicated in Table II, column 3, lines 68 and 450 to 459.

**[0025.1.0.6] and [0025.2.0.6]** for the disclosure of the paragraphs [0025.1.0.6] and [0025.2.0.6] see paragraphs [0025.1.0.0] and [0025.2.0.0] above.

**[0026.0.0.6] to [0033.0.0.6]** for the disclosure of the paragraphs [0026.0.0.6] to [0033.0.0.6] see paragraphs [0026.0.0.0] to [0033.0.0.0] above.

**[0034.0.6.6]** Preferably, the reference, control or wild type differs form the subject of the present invention only in the cellular activity of the polypeptide of the invention or the polypeptide used in the method of the invention, e.g. as result of an increase in the level of the nucleic acid molecule of the present invention or an increase of the specific activity of the polypeptide of the invention or the polypeptide used in the method of the invention. E.g., it differs by or in the expression level or activity of an protein having the activity of a protein as indicated in Table II, column 3, lines 68 to 71 and 450 to 459 or being encoded by a nucleic acid molecule indicated in Table I, column 5, lines 68 to 71 and 450 to 459 or its homologs, e.g. as indicated in Table I, column 7, lines 68 to 71 and 450 to 459, its biochemical or genetical causes and therefore shows the increased amount of the fine chemical.

**[0035.0.0.6] to [0038.0.0.6] and [0039.0.5.6]** for the disclosure of the paragraphs [0035.0.0.6] to [0038.0.0.6] and [0039.0.5.6] see paragraphs [0035.0.0.0] to [0039.0.0.0] above.

**[0040.0.0.6] to [0044.0.0.6]** for the disclosure of the paragraphs [0040.0.0.6] to [0044.0.0.6] see paragraphs [0035.0.0.0] and [0044.0.0.0] above.

**[0045.0.6.6]** In one embodiment, in case the activity of the Escherichia coli K12 protein b2699 or its homologs e.g. a DNA-dependent ATPase or DNA- and ATP-dependent coprotease protein e.g. as indicated in Table II, columns 5 or 7, line 68, is increased, preferably, in one embodiment the increase of the fine chemical, preferably of α-linolenic acid between 13% and 32% or more is conferred.
In one embodiment, in case the activity of the Saccharomyces cerevisiae protein YER173W or its homologs, e.g. a "uncharacterized protein YER173W", which seams to be a "checkpoint protein, involved in the activation of the DNA damage and meiotic pachytene checkpoints; subunit of a clamp loader that loads Rad17p-Mec3p-Ddc1p onto DNA. Its properly a homolog of human and S. pombe Rad17 protein" e.g. as indicated in Table II, columns 5 or 7, line 69, is increased, preferably, in one embodiment an increase of the fine chemical, preferably of α-linolenic acid between 15% and 54% or more is conferred.
In one embodiment, in case the activity of the Saccharomyces cerevisiae protein YGL205W or its homologs, e.g. a fatty-acyl coenzyme A oxidase protein e.g. as indicated in Table II, columns 5 or 7, line 70, is increased, preferably, in one embodiment an increase of the fine chemical, preferably of α-linolenic acid between 13% and 24% or more is conferred.
In one embodiment, in case the activity of the Saccaromyces cerevisiae protein YIL150C or its homologs e.g. a "chromatin binding protein" e.g. as indicated in Table II, columns 5 or 7, line 71, is increased, preferably, in one embodment the increase of the fine chemical, preferably of α-linolenic acid, by at least 243 %.
In case the activity of the Escherichia coli K12 protein b0050 or its homologs e.g. a conserved protein potentially involved in protein protein interaction e.g. as indicated in Table II, columns 5 or 7, line 450, is increased, preferably, in one embodiment the increase of the fine chemical, preferably of α-linolenic acid between 11 % and 19% or more is conferred.
In case the activity of the Escherichia coli K12 protein b0251 or its homologs e.g. a putative HTH-type transcriptional regulator e.g. as indicated in Table II, columns 5 or 7, line 451, is increased, preferably, in one embodiment the increase of the fine chemical, preferably of α-linolenic acid between 14% and 29% or more is conferred.
In case the activity of the Escherichia coli K12 protein b0376 or its homologs e.g. a beta-lactamase/D-ala carboxypeptidase, penicillin binding protein e.g. as indicated in Table II, columns 5 or 7, line 452, is increased, preferably, in one embodiment the increase of the fine chemical, preferably of α-linolenic acid between 15% and 34% or more is conferred.
In case the activity of the Escherichia coli K12 protein b0577 or its homologs e.g. a putative transport protein e.g. as indicated in Table II, columns 5 or 7, line 453, is increased, preferably, in one embodiment the increase of the fine chemical, preferably of α-linolenic acid between 12% and 96% or more is conferred.
In case the activity of the Escherichia coli K12 protein b0849 or its homologs e.g. a glutaredoxin 1 redox coenzyme for glutathione-dependent ribonucleotide reductase e.g. as indicated in Table II, columns 5 or 7, line 454, is increased, preferably, in one embodiment the increase of the fine chemical, preferably of α-linolenic acid between 13% and 31% or more is conferred.
In case the activity of the Escherichia coli K12 protein b2822 or its homologs e.g. a DNA helicase, ATP-dependent dsDNA/ssDNA exonuclease V subunit, ssDNA endonuclease e.g. as indicated in Table II, columns 5 or 7, line 455, is increased, preferably, in one embodiment the increase of the fine chemical, preferably of α-linolenic acid between 15% and 20% or more is conferred.
In case the activity of the Escherichia coli K12 protein b3129 or its homologs e.g. a putative protease; htrA suppressor protein e.g. as indicated in Table II, columns 5 or 7, line 456, is increased, preferably, in one embodiment the increase of the fine chemical, preferably of α-linolenic acid between 12% and 26% or more is conferred.
In case the activity of the Escherichia coli K12 protein b3457 or its homologs e.g. a high-affinity branched-chain amino acid transport protein (ABC superfamily) e.g. as indicated in Table II, columns 5 or 7, line 457, is increased, preferably, in one embodiment the increase of the fine chemical, preferably of α-linolenic acid between 15% and 28% or more is conferred.
In case the activity of the Escherichia coli K12 protein b3462 or its homologs e.g. a integral membrane cell division protein e.g. as indicated in Table II, columns 5 or 7, line 458, is increased, preferably, in one embodiment the increase of the fine chemical, preferably of α-linolenic acid between 13% and 17% or more is conferred.
In case the activity of the Escherichia coli K12 protein b3644 or its homologs e.g. an uncharacterized stress-induced protein e.g. as indicated in Table II, columns 5 or 7, line 459, is increased, preferably, in one embodiment the increase of the fine chemical, preferably of α-linolenic acid between 13% and 36% or more is conferred.

**[0046.0.6.6]** In case the activity of the Escherichia coli K12 protein b2699 or its homologs e.g. a DNA-dependent ATPase or DNA- and ATP-dependent coprotease protein is increased, preferably an increase of the fine chemical and of tryglycerides, lipids, oils and/or fats containing α-linolenic acid is conferred.
In case the activity of the Saccaromyces cerevisiae protein YER173W or its homologs e.g. a "uncharacterized protein YER173W" is increased, preferably an increase of the fine chemical and of tryglycerides, lipids, oils and/or fats containing α-linolenic acid is conferred.
In case the activity of the Saccharomyces cerevisiae protein YGL205W or its homologs, e.g. a fatty-acyl coenzyme A oxidase protein is increased, preferably an increase of the fine chemical and of tryglycerides, lipids, oils and/or fats containing α-linolenic acid is conferred.
In case the activity of the Saccharomyces cerevisiae protein YIL150C or its homologs e.g. a "chromatin binding protein, required for S-phase (DNA synthesis) initation or completion" is increased, preferably an increase of the fine chemical and of tryglycerides, lipids, oils and/or fats containing α-linolenic acid is conferred.
In one embodiment, in case the activity of the Escherichia coli K12 protein b0050 or its homologs e.g. a conserved protein potentially involved in protein protein interaction is increased, preferably an increase of the fine chemical and of tryglycerides, lipids, oils and/or fats containing α-linolenic acid is conferred.
In one embodiment, in case the activity of the Escherichia coli K12 protein b0251 or its homologs e.g. a putative HTH-type transcriptional regulator is increased, preferably an increase of the fine chemical and of tryglycerides, lipids, oils and/or fats containing α-linolenic acid is conferred.
In one embodiment, in case the activity of the Escherichia coli K12 protein b0376 or its homologs e.g. a beta-lactamase/D-ala carboxypeptidase, penicillin binding protein is increased, preferably an increase of the fine chemical and of tryglycerides, lipids, oils and/or fats containing α-linolenic acid is conferred.
In one embodiment, in case the activity of the Escherichia coli K12 protein b0577 or its homologs e.g. a putative transport protein is increased, preferably an increase of the fine chemical and of tryglycerides, lipids, oils and/or fats containing α-linolenic acid is conferred.
In one embodiment, in case the activity of the Escherichia coli K12 protein b0849 or its homologs e.g. a glutaredoxin 1 redox coenzyme for glutathione-dependent ribonucleotide reductase is increased, preferably an increase of the fine chemical and of tryglycerides, lipids, oils and/or fats containing α-linolenic acid is conferred.
In one embodiment, in case the activity of the Escherichia coli K12 protein b2822 or its homologs e.g. a DNA helicase, ATP-dependent dsDNA/ssDNA exonuclease V subunit, ssDNA endonuclease is increased, preferably an increase of the fine chemical and of tryglycerides, lipids, oils and/or fats containing α-linolenic acid is conferred.
In one embodiment, in case the activity of the Escherichia coli K12 protein b3129 or its homologs e.g. a putative protease; htrA suppressor protein is increased, preferably an increase of the fine chemical and of tryglycerides, lipids, oils and/or fats containing α-linolenic acid is conferred.
In one embodiment, in case the activity of the Escherichia coli K12 protein b3457 or its homologs e.g. a high-affinity branched-chain amino acid transport protein (ABC superfamily) is increased, preferably an increase of the fine chemical and of tryglycerides, lipids, oils and/or fats containing α-linolenic acid is conferred.
In one embodiment, in case the activity of the Escherichia coli K12 protein b3462 or its homologs e.g. a integral membrane cell division protein is increased, preferably an increase of the fine chemical and of tryglycerides, lipids, oils and/or fats containing α-linolenic acid is conferred.
In one embodiment, in case the activity of the Escherichia coli K12 protein b3644 or its homologs e.g. an uncharacterized stress-induced protein is increased, preferably an increase of the fine chemical and of tryglycerides, lipids, oils and/or fats containing α-linolenic acid is conferred.

**[0047.0.0.6] to [0048.0.0.6]** for the disclosure of the paragraphs [0047.0.0.6] and [0048.0.0.6] see paragraphs [0047.0.0.0] and [0048.0.0.0] above.

**[0049.0.6.6]** A protein having an activity conferring an increase in the amount or level of the respective fine chemical preferably has the structure of the polypeptide described herein, in particular a polypeptide comprising a consensus sequence as indicated in Table IV, column 7, lines 68 to 71 and 450 to 459 or of the polypeptide as shown in the amino acid sequences as disclosed in table II, columns 5 and 7, lines 68 to 71 and 450 to 459 or the functional homologues thereof as described herein, or is encoded by the nucleic acid molecule characterized herein or the nucleic acid molecule according to the invention, for example by the nucleic acid molecule as shown in table I, columns 5 and 7, lines 68 to 71 and 450 to 459 or its herein described functional homologues and has the herein mentioned activity.

**[0050.0.6.6]** For the purposes of the present invention, the term "α-linolenic acid" also encompasses the corresponding salts, such as, for example, the potassium or sodium salts of α-linolenic acid or the salts of α-linolenic acid with amines such as diethylamine.

**[0051.0.5.6] and [0052.0.0.6]** for the disclosure of the paragraphs [0051.0.5.6] and [0052.0.0.6] see paragraphs [0051.0.0.0] and [0052.0.0.0] above.

**[0053.0.6.6]** In one embodiment, the process of the present invention comprises one or more of the following steps
a) stabilizing a protein conferring the increased expression of a protein encoded by the nucleic acid molecule of the invention or of the polypeptid of the invention or the nucleic acid molecule or the polypeptide used in the method of the invention, e.g. of a polypeptide having an activity of a protein as indicated in Table II, column 3, lines 68 to 71 and 450 to 459 or its homolog's activity, e.g. as indicated in Table II, column 7, lines 68 to 71 and 450 to 459, having herein-mentioned the fine chemical increasing activity;
b) stabilizing a mRNA conferring the increased expression of a protein encoded by the nucleic acid molecule of the invention or the nucleic acid molecule used in the method of the invention, e.g. of a polypeptide having an activity of a protein as indicated in Table II, column 3, lines 68 to 71 and 450 to 459 or its homologs activity, e.g. as indicated in Table II, column 7, lines 68 to 71 and 450 to 459 or of a mRNA encoding the polypeptide of the present invention having herein-mentioned α-linolenic acid increasing activity;
c) increasing the specific activity of a protein conferring the increased expression of a protein encoded by the nucleic acid molecule of the invention or of the polypeptide of the present invention or the nucleic acid molecule or the polypeptide used in the method of the invention, having herein-mentioned α-linolenic acid increasing activity, e.g. of a polypeptide having an activity of a protein as indicated in Table II, column 3, lines 68 to 71 and 450 to 459 or its homologs activity, e.g. as indicated in Table II, column 7, lines 68 to 71 and 450 to 459, or decreasing the inhibitiory regulation of the polypeptide of the invention or of the polypeptide used in the method of the invention;
d) generating or increasing the expression of an endogenous or artificial transcription factor mediating the expression of a protein conferring the increased expression of a protein encoded by the nucleic acid molecule of the invention or the nucleic acid molecule used in the method of the invention or of the polypeptide of the invention or the polypeptide used in the method of the invention having herein-mentioned α-linolenic acid increasing activity, e.g. of a polypeptide having an activity of a protein as indicated in Table II, column 3, lines 68 to 71 and 450 to 459 or its homolog's activity, e.g. as indicated in Table II, column 7, lines 68 to 71 and 450 to 459;
e) stimulating activity of a protein conferring the increased expression of a protein encoded by the nucleic acid molecule of the present invention or a polypeptide of the present invention having herein-mentioned α-linolenic acid increasing activity, e.g. of a polypeptide having an activity of a protein as indicated in Table II, column 3, lines 68 to 71 and 450 to 459 or its homolog's activity, e.g. as indicated in Table II, column 7, lines 68 to 71 and 450 to 459, by adding one or more exogenous inducing factors to the organismus or parts thereof;
f) expressing a transgenic gene encoding a protein conferring the increased expression of a polypeptide encoded by the nucleic acid molecule of the present invention or a polypeptide of the present invention, having herein-mentioned α-linolenic acid increasing activity, e.g. of a polypeptide having an activity of a protein as indicated in Table II, column 3, lines 68 to 71 and 450 to 459 or its homolog's activity, e.g. as indicated in Table II, column 7, lines 68 to 71 and 450 to 459;
g) increasing the copy number of a gene conferring the increased expression of a nucleic acid molecule encoding a polypeptide encoded by the nucleic acid molecule of the invention or the nucleic acid molecule used in the method of the invention or the polypeptide of the invention or the polypeptide used in the method of the invention having herein-mentioned α-linolenic acid increasing activity, e.g. of a polypeptide having an activity of a protein as indicated in Table II, column 3, lines 68 to 71 and 450 to 459 or its homolog's activity, e.g. as indicated in Table II, column 7, lines 68 to 71 and 450 to 459;
h) increasing the expression of the endogenous gene encoding the polypeptide of the invention or the polypeptide used in the method of the invention, e.g. a polypeptide having an activity of a protein as indicated in Table II, column 3, lines 68 to 71 and 450 to 459 or its homolog's activity, e.g. as indicated in Table II, column 7, lines 68 to 71 and 450 to 459, by adding positive expression or removing negative expression elements, e.g. homologous recombination can be used to either introduce positive regulatory elements like for plants the 35S enhancer into the promoter or to remove repressor elements form regulatory regions. Further gene conversion methods can be used to disrupt repressor elements or to enhance to acitivty of positive elements. Positive elements can be randomly introduced in plants by T-DNA or transposon mutagenesis and lines can be identified in which the positive elements have be integrated near to a gene of the invention, the expression of which is thereby enhanced;
i) Modulating growth conditions of an organism in such a manner, that the expression or activity of the gene encoding the protein of the invention or the protein itself is enhanced for example microorganisms or plants can be grown for example under a higher temperature regime leading to an enhanced expression of heat shock proteins, which can lead an enhanced fine chemical production.
j) selecting of organisms with expecially high activity of the proteins of the invention from natural or from mutagenized resources and breeding them into the target organisms, eg the elite crops.

**[0054.0.6.6]** Preferably, said mRNA is the nucleic acid molecule of the present invention and/or the protein conferring the increased expression of a protein encoded by the nucleic acid molecule of the present invention or the polypeptide having the herein mentioned activity is the polypeptide of the present invention, e.g. conferring the increase of α-linolenic acid after increasing the expression or activity of the encoded polypeptide or having the activity of a polypeptide having an activity of a protein as indicated in Table II, column 3, lines 68 to 71 and 450 to 459 or its homolog's activity, e.g. as indicated in Table II, columns 5 or 7, lines 68 to 71 and 450 to 459.

**[0055.0.0.6] to [0067.0.0.6]** for the disclosure of the paragraphs [0055.0.0.6] to [0067.0.0.6] see paragraphs [0055.0.0.0] to [0067.0.0.0] above.

**[0068.0.5.6] and [0069.0.5.6]** for the disclosure of the paragraphs [0068.0.5.6] and [0069.0.5.6] see paragraphs [0068.0.0.0] and [0069.0.0.0] above.

**[0070.0.6.6] and [0071.0.5.6]** for the disclosure of the paragraphs [0070.0.6.6] and [0071.0.5.6] see paragraphs [0070.0.5.5] and [0071.0.0.0] above.

**[0072.0.6.6]** By influencing the metabolism thus, it is possible to produce, in the process according to the invention, further advantageous compounds. Examples of such compounds are, in addition to α-linolenic acid, triglycerides, lipids, oils and/or fats containing α-linolenic acid compounds such as palmitate, palmitoleate, stearate, oleate and/or linoleic acid.

**[0073.0.6.6]** Accordingly, in one embodiment, the process according to the invention relates to a process, which comprises:
a) providing a non-human organism, preferably a microorganism, a non-human animal, a plant or animal cell, a plant or animal tissue or a plant;
b) increasing the activity of a protein having the activity of a polypeptide of the invention or the polypeptide used in the method of the invention or a homolog thereof, e.g. as shown in Table II, columns 5 or 7, lines 68 to 71 and 450 to 459 or of a polypeptide being encoded by the nucleic acid molecule of the present invention and described below, i.e. conferring an increase of the respective fine chemical in the organism, preferably a microorganism, the a non-human animal, a plant or animal cell, a plant or animal tissue or the plant,
c) growing the organism, preferably a microorganism, a non-human animal, a plant or animal cell, a plant or animal tissue or the plant under conditions which permit the production of the fine chemical in the organism, preferably a microorganism, a plant cell, a plant tissue or the plant; and
d) if desired, recovering, optionally isolating, the free and/or bound the respective fine chemical and, optionally further free and/or bound fatty acids synthetized by the organism, the microorganism, the non-human animal, the plant or animal cell, the plant or animal tissue or the plant.

**[0074.0.5.6]** for the disclosure of this paragraph see [0074.0.5.5] above.

**[0075.0.0.6] to [0084.0.0.6]** for the disclosure of the paragraphs [0075.0.0.6] to [0084.0.0.6] see paragraphs [0075.0.0.0] to [0084.0.0.0] above.

**[0085.0.6.6]** With regard to the nucleic acid sequence as depicted a nucleic acid construct which contains a nucleic acid sequence mentioned herein or an organism (= transgenic organism) which is transformed with said nucleic acid sequence or said nucleic acid construct, "transgene" means all those constructs which have been brought about by genetic manipulation methods,preferably in which either
a) the nucleic acid sequence as shown in table I, lines 68 to 71 and 450 to 459, columns 5 and 7 or a derivative thereof, or
b) a genetic regulatory element, for example a promoter, which is functionally linked to the nucleic acid sequence as shown table I, lines 68 to 71 and 450 to 459, columns 5 and 7 or a derivative thereof, or
c) (a) and (b)
is/are not present in its/their natural genetic environment or has/have been modified by means of genetic manipulation methods, it being possible for the modification to be, by way of example, a substitution, addition, deletion, inversion or insertion of one or more nucleotide. "Natural genetic environment" means the natural chromosomal locus in the organism of origin or the presence in a genomic library. In the case of a genomic library, the natural, genetic environment of the nucleic acid sequence is preferably at least partially still preserved. The environment flanks the nucleic acid sequence at least on one side and has a sequence length of at least 50 bp, preferably at least 500 bp, particularly preferably at least 1000 bp, very particularly preferably at least 5000 bp.

**[0086.0.0.6] and [0087.0.0.6]** for the disclosure of the paragraphs [0086.0.0.6] and [0087.0.0.6] see paragraphs [0086.0.0.0] and [0087.0.0.0] above.

**[0088.0.6.6]** In an advantageous embodiment of the invention, the organism takes the form of a plant whose fatty acid content is modified advantageously owing to the nucleic acid molecule of the present invention expressed. This is important for plant breeders since, for example, the nutritional value of plants for poultry is dependent on the abovementioned essential fatty acids and the general amount of fatty acids as energy source in feed. After the activity of a protein as shown in Table II, columns 5 or 7, lines 68 to 71 and 450 to 459 has been increased or generated, or after the expression of nucleic acid molecule or polypeptide according to the invention has been generated or increased, the transgenic plant generated thus is grown on or in a nutrient medium or else in the soil and subsequently harvested.

**[0088.1.0.6], [0089.0.0.6], [0090.0.0.6] and [0091.0.5.6]** for the disclosure of the paragraphs [0088.1.0.6], [0089.0.0.6], [0090.0.0.6] and [0091.0.5.6] see paragraphs [0088.1.0.0], [0089.0.0.0], [0090.0.0.0] and [0091.0.0.0] above.

**[0092.0.0.6] to [0094.0.0.6]** for the disclosure of the paragraphs [0092.0.0.6] to [0094.0.0.6] see paragraphs [0092.0.0.0] to [0094.0.0.0] above.

**[0095.0.5.6], [0096.0.5.6] and [0097.0.5.6]** for the disclosure of the paragraphs [0095.0.5.6], [0096.0.5.6] and [0097.0.5.6] see paragraphs [0095.0.5.5], [0096.0.5.5] and [0097.0.0.0] above.

**[0098.0.6.6]** In a preferred embodiment, the fine chemical (α-linolenic acid) is produced in accordance with the invention and, if desired, is isolated. The production of further fatty acids such as palmitic acid, stearic acid, palmitoleic acid, oleic acid and/or linoleic acid mixtures thereof or mixtures of other fatty acids by the process according to the invention is advantageous.

**[0099.0.5.6] and [0100.0.5.6]** for the disclosure of the paragraphs [0099.0.5.6] and [0100.0.5.6] see paragraphs [0099.0.5.5] and [0100.0.5.5] above.

**[0101.0.5.6] and [0102.0.5.6]** for the disclosure of the paragraphs [0101.0.5.6] and [0102.0.5.6] see [0101.0.0.0] and [0102.0.5.5] above.

**[0103.0.6.6]** In a preferred embodiment, the present invention relates to a process for the production of the fine chemical comprising or generating in an organism or a part thereof the expression of at least one nucleic acid molecule comprising a nucleic acid molecule selected from the group consisting of:
a) nucleic acid molecule encoding, preferably at least the mature form, of a polypeptide having a sequence as shown in Table II, columns 5 or 7, lines 68 to 71 and 450 to 459 in or a fragment thereof, which confers an increase in the amount of the respective fine chemical in an organism or a part thereof;
b) nucleic acid molecule comprising, preferably at least the mature form, of the nucleic acid molecule having a sequence as shown in Table I, columns 5 or 7, lines 68 to 71 and 450 to 459,
c) nucleic acid molecule whose sequence can be deduced from a polypeptide sequence encoded by a nucleic acid molecule of (a) or (b) as result of the degeneracy of the genetic code and conferring an increase in the amount of the fine chemical in an organism or a part thereof;
d) nucleic acid molecule encoding a polypeptide which has at least 50% identity with the amino acid sequence of the polypeptide encoded by the nucleic acid molecule of (a) to (c) and conferring an increase in the amount of the fine chemical in an organism or a part thereof;
e) nucleic acid molecule which hybidizes with a nucleic acid molecule of (a) to (c) under under stringent hybridisation conditions and conferring an increase in the amount of the fine chemical in an organism or a part thereof;
f) nucleic acid molecule encoding a polypeptide, the polypeptide being derived by substituting, deleting and/or adding one or more amino acids of the amino acid sequence of the polypeptide encoded by the nucleic acid molecules (a) to (d), preferably to (a) to (c) and conferring an increase in the amount of the respective fine chemical in an organism or a part thereof;
g) nucleic acid molecule encoding a fragment or an epitope of a polypeptide which is encoded by one of the nucleic acid molecules of (a) to (e), preferably to (a) to (c) and and conferring an increase in the amount of the respective fine chemical in an organism or a part thereof;
h) nucleic acid molecule comprising a nucleic acid molecule which is obtained by amplifying nucleic acid molecules from a cDNA library or a genomic library using the primer pairs having a sequence as shown in Table III, column 7, lines 68 to 71 and 450 to 459 and conferring an increase in the amount of the respective fine chemical in an organism or a part thereof;
i) nucleic acid molecule encoding a polypeptide which is isolated, e.g. from an expression library, with the aid of monoclonal antibodies against a polypeptide encoded by one of the nucleic acid molecules of (a) to (h), preferably to (a) to (c), and and conferring an increase in the amount of the respective fine chemical in an organism or a part thereof;
j) nucleic acid molecule which encodes a polypeptide comprising the consensus sequence having a sequence as shown in Table IV, column 7, lines 68 to 71 and 450 to 459 and conferring an increase in the amount of the respective fine chemical in an organism or a part thereof ;
k) nucleic acid molecule comprising one or more of the nucleic acid molecule encoding the amino acid sequence of a polypeptide encoding a domain of a polypeptide as shown in Table II, columns 5 or 7, lines 68 to 71 and 450 to 459 and conferring an increase in the amount of the respective fine chemical in an organism or a part thereof; and
l) nucleic acid molecule which is obtainable by screening a suitable library under stringent conditions with a probe comprising one of the sequences of the nucleic acid molecule of (a) to (k), preferably to (a) to (c), or with a fragment of at least 15 nt, preferably 20 nt, 30 nt, 50 nt, 100 nt, 200 nt or 500 nt of the nucleic acid molecule characterized in (a) to (k), preferably to (a) to (c), and conferring an increase in the amount of the fine chemical in an organism or a part thereof;
or which comprises a sequence which is complementary thereto.

**[0103.1.0.6] and [0103.2.0.6]** for the disclosure of the paragraphs [0103.1.0.6] and [0103.2.0.6] see paragraphs [0103.1.0.0] and [0103.2.0.0] above.

**[0104.0.6.6]** In one embodiment, the nucleic acid molecule used in the process distinguishes over the sequence shown in Table I, columns 5 or 7, lines 68 to 71 and 450 to 459, preferably over the sequences as shown in Table IA, columns 5 or 7, lines 68 to 71 and 450 to 459 by one or more nucleotides or does not consist of the sequence shown in Table I, columns 5 or 7, lines 68 to 71 and 450 to 459, preferably does not consist of the sequences as shown in Table IA, columns 5 or 7, lines 68 to 71 and 450 to 459. In one embodiment, the nucleic acid molecule of the present invention is less than 100%, 99,999%, 99,99%, 99,9% or 99% identical to the sequence shown in Table I, columns 5 or 7, lines 68 to 71 and 450 to 459, preferably to the sequences as shown in Table IA, columns 5 or 7, lines 68 to 71 and 450 to 459. In another embodiment, the nucleic acid molecule does not encode a polypeptide of the sequence shown in Table II, columns 5 or 7, lines 68 to 71 and 450 to 459, preferably of the sequences as shown in Table IA, columns 5 or 7, lines 68 to 71 and 450 to 459.

**[0105.0.0.6] to [0107.0.0.6]** for the disclosure of the paragraphs [0105.0.0.6] to [0107.0.0.6] see paragraphs [0105.0.0.0] and [0107.0.0.0] above.

**[0108.0.6.6]** Nucleic acid molecules with the sequence shown in Table I, columns 5 or 7, lines 68 to 71 and 450 to 459, nucleic acid molecules which are derived from the amino acid sequences shown in Table II, columns 5 or 7, lines 68 to 71 and 450 to 459 or from polypeptides comprising the consensus sequence shown in Table IV, column 7, lines 68 to 71 and 450 to 459, or their derivatives or homologues encoding polypeptides with the enzymatic or biological activity of a protein as shown in Table II, columns 5 or 7, lines 68 to 71 and 450 to 459 or e.g. conferring a α-linolenic acid increase after increasing its expression or activity are advantageously increased in the process according to the invention.

**[0109.0.5.6]** for the disclosure of this paragraph see [0109.0.0.0] above.

**[0110.0.6.6]** Nucleic acid molecules, which are advantageous for the process according to the invention and which encode polypeptides with an activity of a polypeptide used in the method of the invention or used in the process of the invention, e.g. of a protein as shown in Table II, columns 5 or 7, lines 68 to 71 and 450 to 459 or being encoded by a nucleic acid molecule indicated in Table I, columns 5 or 7, lines 68 to 71 and 450 to 459 or of its homologs, e.g. as indicated in Table II, columns 5 or 7, lines 68 to 71 and 450 to 459 can be determined from generally accessible databases.

**[0111.0.0.6]** for the disclosure of this paragraph see [0111.0.0.0] above.

**[0112.0.6.6]** The nucleic acid molecules used in the process according to the invention take the form of isolated nucleic acid sequences, which encode polypeptides with an activity of a polypeptide as indicated in Table II, column3, lines lines 68 to 71 and 450 to 459 or having the sequence of a polypeptide as indicated in Table II, columns 5 and 7, lines 68 to 71 and 450 to 459 and conferring an increase of the respective fine chemical.

**[0113.0.0.6] to [0120.0.0.6]** for the disclosure of the paragraphs [0113.0.0.6] to [0120.0.0.6] see paragraphs [0113.0.0.0] and [0120.0.0.0] above.

**[0121.0.6.6]** However, it is also possible to use artificial sequences, which differ in one or more bases from the nucleic acid sequences found in organisms, or in one or more amino acid molecules from polypeptide sequences found in organisms, in particular from the polypeptide sequences shown in Table II, columns 5 or 7, lines 68 to 71 and 450 to 459 or the functional homologues thereof as described herein, preferably conferring above-mentioned activity, i.e. conferring an increase of the respective fine chemical after increasing its activity.

**[0122.0.0.6] to [0127.0.0.6]** for the disclosure of the paragraphs [0122.0.0.6] to [0127.0.0.6] see paragraphs [0122.0.0.0] and [0127.0.0.0] above.

**[0128.0.6.6]** Synthetic oligonucleotide primers for the amplification, e.g. as shown in Table III, column 7, lines 68 to 71 and 450 to 459, by means of polymerase chain reaction can be generated on the basis of a sequence shown herein, for example the sequence shown in Table I, columns 5 or 7, lines 68 to 71 and 450 to 459 or the sequences as shown in Table II, columns 5 or 7, lines 68 to 71 and 450 to 459.

**[0129.0.6.6]** Moreover, it is possible to identify conserved regions from various organisms by carrying out protein sequence alignments with the polypeptide used in the process of the invention, in particular with sequences of the polypeptide of the invention, from which conserved regions, and in turn, degenerate primers can be derived. Conserved region for the polypeptide of the invention are indicated in the alignments shown in the figures. Conserved regions are those, which show a very little variation in the amino acid in one particular position of several homologs from different origin. The consenus sequence shown in Table IV, column 7, lines 68 to 71 and 450 to 459 is derived from said aligments.

**[0130.0.6.6]** for the disclosure of this paragraph see [0130.0.0.0].

**[0131.0.0.6] to [0138.0.0.6]** for the disclosure of the paragraphs [0131.0.0.6] to [0138.0.0.6] see paragraphs [0131.0.0.0] to [0138.0.0.0] above.

**[0139.0.6.6]** Polypeptides having above-mentioned activity, i.e. conferring the respective fine chemical increase, derived from other organisms, can be encoded by other DNA sequences which hybridize to the sequences shown in Table I, columns 5 or 7, lines 68 to 71 and 450 to 459, preferably of Table I B, columns 5 or 7, lines 68 to 71 and 450 to 459 under relaxed hybridization conditions and which code on expression for peptides having the α-linolenic acid increasing activity.

**[0140.0.0.6] to [0146.0.0.6]** for the disclosure of the paragraphs [0140.0.0.6] to [0146.0.0.6] see paragraphs [0140.0.0.0] and [0146.0.0.0] above.

**[0147.0.6.6]** Further, the nucleic acid molecule of the invention comprises a nucleic acid molecule, which is a complement of one of the nucleotide sequences of above mentioned nucleic acid molecules or a portion thereof. A nucleic acid molecule which is complementary to one of the nucleotide sequences indicated in Table I, columns 5 or 7, lines 68 to 71 and 450 to 459, preferably in Table I B, columns 5 or 7, lines 68 to 71 and 450 to 459 is one which is sufficiently complementary to one of said nucleotide sequences such that it can hybridize to one of said nucleotide sequences thereby forming a stable duplex. Preferably, the hybridisation is performed under stringent hybrization conditions. However, a complement of one of the herein disclosed sequences is preferably a sequence complement thereto according to the base pairing of nucleic acid molecules well known to the skilled person. For example, the bases A and G undergo base pairing with the bases T and U or C, resp. and visa versa. Modifications of the bases can influence the base-pairing partner.

**[0148.0.6.6]** The nucleic acid molecule of the invention comprises a nucleotide sequence which is at least about 30%, 35%, 40% or 45%, preferably at least about 50%, 55%, 60% or 65%, more preferably at least about 70%, 80%, or 90%, and even more preferably at least about 95%, 97%, 98%, 99% or more homologous to a nucleotide sequence indicated in Table I, columns 5 or 7, lines 68 to 71 and 450 to 459, preferably of Table I B, columns 5 or 7, lines 68 to 71 and 450 to 459, or a functional portion thereof and preferably has above mentioned activity, in particular has the-fine-chemical-increasing activity after increasing its acitivity or an activity of a product of a gene encoding said sequence or its homologs.

**[0149.0.6.6]** The nucleic acid molecule of the invention or the nucleic acid molecule used in the method of the invention comprises a nucleotide sequence which hybridizes, preferably hybridizes under stringentconditions as defined herein, to one of the nucleotide sequences indicated in Table I, columns 5 or 7, lines 68 to 71 and 450 to 459, preferably of Table I B, columns 5 or 7, lines 68 to 71 and 450 to 459 or a portion thereof and encodes a protein having above-mentioned activity as indicated in Table II, columns 5 or 7, lines 68 to 71 and 450 to 459, preferably of Table II B, columns 5 or 7, lines 68 to 71 and 450 to 459, e.g. conferring an increase of the respective fine chemical.

**[00149.1.0.5]** Optionally, the nucleotide sequence, which hybridises to one of the nucleotide sequences indicated in Table I, columns 5 or 7, lines 68 to 71 and 450 to 459, preferably of Table I B, columns 5 or 7, lines 68 to 71 and 450 to 459 has further one or more of the activities annotated or known for the a protein as indicated in Table II, column 3, lines 68 to 71 and 450 to 459.

**[0150.0.6.6]** Moreover, the nucleic acid molecule of the invention or used in the process of the invention can comprise only a portion of the coding region of one of the sequences indicated in Table I, columns 5 or 7, lines 68 to 71 and 450 to 459, preferably of Table I B, columns 5 or 7, lines 68 to 71 and 450 to 459, for example a fragment which can be used as a probe or primer or a fragment encoding a biologically active portion of the polypeptide of the present invention or of a polypeptide used in the process of the present invention, i.e. having above-mentioned activity, e.g.conferring an increase of methionine if its activity is increased. The nucleotide sequences determined from the cloning of the present protein-according-to-the-invention-encoding gene allows for the generation of probes and primers designed for use in identifying and/or cloning its homologues in other cell types and organisms. The probe/primer typically comprises substantially purified oligonucleotide. The oligonucleotide typically comprises a region of nucleotide sequence that hybridizes under stringent conditions to at least about 12, 15 preferably about 20 or 25, more preferably about 40, 50 or 75 consecutive nucleotides of a sense strand of one of the sequences indicated in Table I, columns 5 or 7, lines 68 to 71 and 450 to 459, an anti-sense sequence of one of the sequences indicated in Table I, columns 5 or 7, lines 68 to 71 and 450 to 459, or naturally occurring mutants thereof. Primers based on a nucleotide sequence of the invention can be used in PCR reactions to clone homologues of the polypeptide of the invention or of the polypeptide used in the process of the invention, e.g. as the primers described in the examples of the present invention, e.g. as shown in the examples. A PCR with the primer pairs indicated in Table III, column 7, lines 68 to 71 and 450 to 459 will result in a fragment of a polynucleotide sequence as indicated in Table I, columns 5 or 7, lines 68 to 71 and 450 to 459. Preferred is Table I B, column 7, lines 68 to 71 and 450 to 459.

**[0151.0.0.6]** for the disclosure of this paragraph see [0151.0.0.0] above.

**[0152.0.6.6]** The nucleic acid molecule of the invention encodes a polypeptide or portion thereof which includes an amino acid sequence which is sufficiently homologous to the amino acid sequence shown in Table II, columns 5 or 7, lines 68 to 71 and 450 to 459 such that the protein or portion thereof maintains the ability to participate in the fine chemical production, in particular a α-linolenic acid increasing the activity as mentioned above or as described in the examples in plants or microorganisms is comprised.

**[0153.0.6.6]** As used herein, the language "sufficiently homologous" refers to proteins or portions thereof which have amino acid sequences which include a minimum number of identical or equivalent amino acid residues (e.g., an amino acid residue which has a similar side chain as an amino acid residue in one of the sequences of the polypeptide of the present invention) to an amino acid sequence shown in Table II, columns 5 or 7, lines 68 to 71 and 450 to 459 such that the protein or portion thereof is able to participate in the increase of the respective fine chemical production. In one embodiment, a protein or portion thereof as shown in Table II, columns 5 or 7, lines 68 to 71 and 450 to 459 has for example an activity of a polypeptide as indicated in Table II, columns 5 or 7, lines 68 to 71 and 450 to 459.

**[0154.0.6.6]** In one embodiment, the nucleic acid molecule of the present invention comprises a nucleic acid that encodes a portion of the protein of the present invention. The protein is at least about 30%, 35%, 40%, 45% or 50%, preferably at least about 55%, 60%, 65% or 70%, and more preferably at least about 75%, 80%, 85%, 90%, 91%, 92%, 93% or 94% and most preferably at least about 95%, 97%, 98%, 99% or more homologous to an entire amino acid sequence as shown in Table II, columns 5 or 7, lines 68 to 71 and 450 to 459 and having above-mentioned activity, e.g.conferring preferably the increase of the respective fine chemical.

**[0155.0.0.6] and [0156.0.0.6]** for the disclosure of the paragraphs [0155.0.0.6] and [0156.0.0.6] see paragraphs [0155.0.0.0] and [0156.0.0.0] above.

**[0157.0.6.6]** The invention further relates to nucleic acid molecules that differ from one of the nucleotide sequences indicated in Table I, columns 5 or 7, lines 68 to 71 and 450 to 459 (and portions thereof) due to degeneracy of the genetic code and thus encode a polypeptide of the present invention, in particular a polypeptide having above mentioned activity, e.g. conferring an increase in the respective fine chemical in a organism, e.g. as that polypeptides comprising the consensus sequences as indicated in Table IV, column 7, lines 68 to 71 and 450 to 459 or of the polypeptide as indicated in Table II, columns 5 or 7, lines 68 to 71 and 450 to 459 or their functional homologues. Advantageously, the nucleic acid molecule of the invention or the nucleic acid molecule used in the method of the invention comprises, or in an other embodiment has, a nucleotide sequence encoding a protein comprising, or in an other embodiment having, a consensus sequences as indicated in Table IV, column 7, lines 68 to 71 and 450 to 459 or of the polypeptide as indicated in Table II, columns 5 or 7, lines 68 to 71 and 450 to 459 or the functional homologues. In a still further embodiment, the nucleic acid molecule of the invention or the nucleic acid molecule used in the method of the invention encodes a full length protein which is substantially homologous to an amino acid sequence comprising a consensus sequence as indicated in Table IV, column 7, lines 68 to 71 and 450 to 459 or of a polypeptide as indicated in Table II, columns 5 or 7, lines 68 to 71 and 450 to 459 or the functional homologues thereof. However, in a preferred embodiment, the nucleic acid molecule of the present invention does not consist of a sequence as indicated in Table I, columns 5 or 7, lines 68 to 71 and 450 to 459, preferably as indicated in Table I A, columns 5 or 7, lines 68 to 71 and 450 to 459. Preferably the nucleic acid molecule of the invention is a functional homologue or identical to a nucleic acid molecule indicated in Table I B, columns 5 or 7, lines 68 to 71 and 450 to 459.

**[0158.0.0.6] to [0160.0.0.6]** for the disclosure of the paragraphs [0158.0.0.6] to [0160.0.0.6] see paragraphs [0158.0.0.0] to [0160.0.0.0] above.

**[0161.0.6.6]** Accordingly, in another embodiment, a nucleic acid molecule of the invention or the nucleic acid molecule used in the method of the invention is at least 15, 20, 25 or 30 nucleotides in length. Preferably, it hybridizes under stringent conditions to a nucleic acid molecule comprising a nucleotide sequence of the nucleic acid molecule of the present invention or used in the process of the present invention, e.g. comprising the sequence shown in Table I, columns 5 or 7, lines 68 to 71 and 450 to 459. The nucleic acid molecule is preferably at least 20, 30, 50, 100, 250 or more nucleotides in length.

**[0162.0.0.6]** for the disclosure of this paragraph see paragraph [0162.0.0.0] above.

**[0163.0.6.6]** Preferably, nucleic acid molecule of the invention that hybridizes under stringent conditions to a sequence as indicated in Table I, columns 5 or 7, lines 68 to 71 and 450 to 459 corresponds to a naturally-occurring nucleic acid molecule of the invention. As used herein, a "naturally-occurring" nucleic acid molecule refers to an RNA or DNA molecule having a nucleotide sequence that occurs in nature (e.g., encodes a natural protein). Preferably, the nucleic acid molecule encodes a natural protein having above-mentioned activity, e.g. conferring the respective fine chemical increase after increasing the expression or activity thereof or the activity of a protein of the invention or used in the process of the invention.

**[0164.0.0.6]** for the disclosure of this paragraph see paragraph [0164.0.0.0] above. **[0165.0.6.6]** For example, nucleotide substitutions leading to amino acid substitutions at "non-essential" amino acid residues can be made in a sequence of the nucleic acid molecule of the invention or used in the process of the invention, e.g. as shown in Table I, columns 5 or 7, lines 68 to 71 and 450 to 459.

**[0166.0.0.6] and [0167.0.0.6]** for the disclosure of the paragraphs [0166.0.0.6] and [0167.0.0.6] see paragraphs [0166.0.0.0] and [0167.0.0.0] above.

**[0168.0.6.6]** Accordingly, the invention relates to nucleic acid molecules encoding a polypeptide having above-mentioned activity, e.g. conferring an increase in the respective fine chemical in an organism or parts thereof that contain changes in amino acid residues that are not essential for said activity. Such polypeptides differ in amino acid sequence from a sequence contained in a sequence as indicated in Table II, columns 5 or 7, lines 68 to 71 and 450 to 459 yet retain said activity described herein. The nucleic acid molecule can comprise a nucleotide sequence encoding a polypeptide, wherein the polypeptide comprises an amino acid sequence at least about 50% identical to an amino acid sequence as indicated in Table II, columns 5 or 7, lines 68 to 71 and 450 to 459 and is capable of participation in the increase of production of the respective fine chemical after increasing its activity, e.g. its expression. Preferably, the protein encoded by the nucleic acid molecule is at least about 60% identical to the sequence as indicated in Table II, columns 5 or 7, lines 68 to 71 and 450 to 459, more preferably at least about 70% identical to one of the sequences as indicated in Table II, columns 5 or 7, lines 68 to 71 and 450 to 459, even more preferably at least about 80%, 90%, 95% homologous to the sequence as indicated in Table II, columns 5 or 7, lines 68 to 71 and 450 to 459, and most preferably at least about 96%, 97%, 98%, or 99% identical to the sequence as indicated in Table II, columns 5 or 7, lines 68 to 71 and 450 to 459.
Accordingly, the invention relates to nucleic acid molecules encoding a polypeptide having above-mentioned activity, e.g. conferring an increase in the the respective fine chemical in an organisms or parts thereof that contain changes in amino acid residues that are not essential for said activity. Such polypeptides differ in amino acid sequence from a sequence contained in a sequence as indicated in Table II, columns 5 or 7, lines 68 to 71 and 450 to 459, preferably of Table II B, column 7, lines 68 to 71 and 450 to 459 yet retain said activity described herein. The nucleic acid molecule can comprise a nucleotide sequence encoding a polypeptide, wherein the polypeptide comprises an amino acid sequence at least about 50% identical to an amino acid sequence as indicated in Table II, columns 5 or 7, lines 68 to 71 and 450 to 459, preferably of Table II B, column 7, lines 68 to 71 and 450 to 459 and is capable of participation in the increase of production of the respective fine chemical after increasing its activity, e.g. its expression. Preferably, the protein encoded by the nucleic acid molecule is at least about 60% identical to a sequence as indicated in Table II, columns 5 or 7, lines 68 to 71 and 450 to 459, preferably of Table II B, column 7, lines 68 to 71 and 450 to 459, more preferably at least about 70% identical to one of the sequences as indicated in Table II, columns 5 or 7, lines 68 to 71 and 450 to 459, preferably of Table II B, column 7, lines 68 to 71 and 450 to 459, even more preferably at least about 80%, 90%, or 95% homologous to a sequence as indicated in Table II, columns 5 or 7, lines 68 to 71 and 450 to 459, preferably of Table II B, column 7, lines 68 to 71 and 450 to 459, and most preferably at least about 96%, 97%, 98%, or 99% identical to the sequence as indicated in Table II, columns 5 or 7, lines 68 to 71 and 450 to 459, preferably of Table II B, column 7, lines 68 to 71 and 450 to 459.

**[0169.0.0.6] to [0175.0.5.6]** for the disclosure of the paragraphs [0169.0.0.6] to [0175.0.5.6] see paragraphs [0169.0.0.0] to [0175.0.0.0] above.

**[0176.0.6.6]** Functional equivalents derived from one of the polypeptides as shown in Table II, columns 5 or 7, lines 68 to 71 and 450 to 459 according to the invention by substitution, insertion or deletion have at least 30%, 35%, 40%, 45% or 50%, preferably at least 55%, 60%, 65% or 70% by preference at least 80%, especially preferably at least 85% or 90%, 91%, 92%, 93% or 94%, very especially preferably at least 95%, 97%, 98% or 99% homology with one of the polypeptides as shown in Table II, columns 5 or 7, lines 68 to 71 and 450 to 459 according to the invention and are distinguished by essentially the same properties as the polypeptide as shown in Table II, columns 5 or 7, lines 68 to 71 and 450 to 459.

**[0177.0.6.6]** Functional equivalents derived from the nucleic acid sequence as shown in Table I, columns 5 or 7, lines 68 to 71 and 450 to 459 according to the invention by substitution, insertion or deletion have at least 30%, 35%, 40%, 45% or 50%, preferably at least 55%, 60%, 65% or 70% by preference at least 80%, especially preferably at least 85% or 90%, 91%, 92%, 93% or 94%, very especially preferably at least 95%, 97%, 98% or 99% homology with one of the polypeptides as shown in Table II, columns 5 or 7, lines 68 to 71 and 450 to 459 according to the invention and encode polypeptides having essentially the same properties as the polypeptide as shown in Table II, columns 5 or 7, lines 68 to 71 and 450 to 459.

**[0178.0.0.6]** for the disclosure of this paragraph see [0178.0.0.0] above. **[0179.0.6.6]** A nucleic acid molecule encoding a homologous to a protein sequence of as indicated in Table II, columns 5 or 7, lines 68 to 71 and 450 to 459, preferably of Table II B, column 7, lines 68 to 71 and 450 to 459 can be created by introducing one or more nucleotide substitutions, additions or deletions into a nucleotide sequence of the nucleic acid molecule of the present invention, in particular as indicated in Table I, columns 5 or 7, lines 68 to 71 and 450 to 459 such that one or more amino acid substitutions, additions or deletions are introduced into the encoded protein. Mutations can be introduced into the encoding sequences for example into a sequences as indicated in Table I, columns 5 or 7, lines 68 to 71 and 450 to 459 by standard techniques, such as site-directed mutagenesis and PCR-mediated mutagenesis.

**[0180.0.0.6] to [0183.0.0.6]** for the disclosure of the paragraphs [0180.0.0.6] to [0183.0.0.6] see paragraphs [0180.0.0.0] to [0183.0.0.0] above.

**[0184.0.6.6]** Homologues of the nucleic acid sequences used, with a sequence as indicated in Table I, columns 5 or 7, lines 68 to 71 and 450 to 459, preferably of Table I B, column 7, lines 68 to 71 and 450 to 459, or of the nucleic acid sequences derived from a sequences as indicated in Table II, columns 5 or 7, lines 68 to 71 and 450 to 459, preferably of Table II B, column 7, lines 68 to 71 and 450 to 459, comprise also allelic variants with at least approximately 30%, 35%, 40% or 45% homology, by preference at least approximately 50%, 60% or 70%, more preferably at least approximately 90%, 91%, 92%, 93%, 94% or 95% and even more preferably at least approximately 96%, 97%, 98%, 99% or more homology with one of the nucleotide sequences shown or the abovementioned derived nucleic acid sequences or their homologues, derivatives or analogues or parts of these. Allelic variants encompass in particular functional variants which can be obtained by deletion, insertion or substitution of nucleotides from the sequences shown, preferably from a sequence as indicated in Table I, columns 5 or 7, lines 68 to 71 and 450 to 459, or from the derived nucleic acid sequences, the intention being, however, that the enzyme activity or the biological activity of the resulting proteins synthesized is advantageously retained or increased.

**[0185.0.6.6]** In one embodiment of the present invention, the nucleic acid molecule of the invention or used in the process of the invention comprises one or more sequences as indicated in Table I, columns 5 or 7, lines 68 to 71 and 450 to 459, preferably of Table I B, column 7, lines 68 to 71 and 450 to 459. In one embodiment, it is preferred that the nucleic acid molecule comprises as little as possible other nucleotide sequences not shown in any one of sequences as indicated in Table I, columns 5 or 7, lines 68 to 71 and 450 to 459, preferably of Table I B, column 7, lines 68 to 71 and 450 to 459. In one embodiment, the nucleic acid molecule comprises less than 500, 400, 300, 200, 100, 90, 80, 70, 60, 50 or 40 further nucleotides. In a further embodiment, the nucleic acid molecule comprises less than 30, 20 or 10 further nucleotides. In one embodiment, a nucleic acid molecule used in the process of the invention is identical to a sequences as indicated in Table I, columns 5 or 7, lines 68 to 71 and 450 to 459, preferably of Table I B, column 7, lines 68 to 71 and 450 to 459.

**[0186.0.6.6]** Also preferred is that one or more nucleic acid molecule(s) used in the process of the invention encode a polypeptide comprising a sequence as indicated in Table II, columns 5 or 7, lines 68 to 71 and 450 to 459, preferably of Table II B, column 7, lines 68 to 71 and 450 to 459. In one embodiment, the nucleic acid molecule encodes less than 150, 130, 100, 80, 60, 50, 40 or 30 further amino acids. In a further embodiment, the encoded polypeptide comprises less than 20, 15, 10, 9, 8, 7, 6 or 5 further amino acids. In one embodiment, the encoded polypeptide used in the process of the invention is identical to the sequences as indicated in Table II, columns 5 or 7, lines 68 to 71 and 450 to 459, preferably of Table II B, column 7, lines 68 to 71 and 450 to 459.

**[0187.0.6.6]** In one embodiment, the nucleic acid molecule, of the invention or used in the process encodes a polypeptide comprising a sequence as indicated in Table II, columns 5 or 7, lines 68 to 71 and 450 to 459, preferably of Table II B, column 7, lines 68 to 71 and 450 to 459 comprises less than 100 further nucleotides. In a further embodiment, said nucleic acid molecule comprises less than 30 further nucleotides. In one embodiment, the nucleic acid molecule used in the process is identical to a coding sequence encoding a sequences as indicated in Table II, columns 5 or 7, lines 68 to 71 and 450 to 459, preferably of Table II B, column 7, lines 68 to 71 and 450 to 459.

**[0188.0.6.6]** Polypeptides (= proteins), which still have the essential biological activity of the polypeptide of the present invention conferring an increase of the fine chemical i.e. whose activity is essentially not reduced, are polypeptides with at least 10% or 20%, by preference 30% or 40%, especially preferably 50% or 60%, very especially preferably 80% or 90 or more of the wild type biological activity or enzyme activity, advantageously, the activity is essentially not reduced in comparison with the activity of a polypeptide shown in Table II, columns 5 or 7, lines 68 to 71 and 450 to 459 expressed under identical conditions.
In one embodiment, the polypeptide of the invention is a homolog consisting of or comprising the sequence as indicated in Table II B, columns 7, lines 68 to 71 and 450 to 459.

**[0189.0.6.6]** Homologues of sequences as indicated in Table I, columns 5 or 7, lines 68 to 71 and 450 to 459 or of the derived sequences shown in Table II, columns 5 or 7, lines 68 to 71 and 450 to 459 also mean truncated sequences, cDNA, single-stranded DNA or RNA of the coding and noncoding DNA sequence. Homologues of said sequences are also understood as meaning derivatives, which comprise noncoding regions such as, for example, UTRs, terminators, enhancers or promoter variants. The promoters upstream of the nucleotide sequences stated can be modified by one or more nucleotide substitution(s), insertion(s) and/or deletion(s) without, however, interfering with the functionality or activity either of the promoters, the open reading frame (= ORF) or with the 3'-regulatory region such as terminators or other 3'regulatory regions, which are far away from the ORF. It is furthermore possible that the activity of the promoters is increased by modification of their sequence, or that they are replaced completely by more active promoters, even promoters from heterologous organisms. Appropriate promoters are known to the person skilled in the art and are mentioned herein below.

**[0190.0.0.6], [0191.0.5.6], [00191.1.0.6] and [0192.0.0.6] to [0203.0.0.6]** for the disclosure of the paragraphs [0190.0.0.6], [0191.0.5.6], [0191.1.0.6] and [0192.0.0.6] to [0203.0.0.6] see paragraphs [0190.0.0.0], [0191.0.0.0], [0191.1.0.0] and [0192.0.0.0] to [0203.0.0.0] above.

**[0204.0.6.6]** Accordingly, in one embodiment, the invention relates to a nucleic acid molecule, which comprises a nucleic acid molecule selected from the group consisting of:
a) nucleic acid molecule encoding, preferably at least the mature form, of the polypeptide shown in Table II, columns 5 or 7, lines 68 to 71 and 450 to 459; preferably of Table II B, column 7, lines 68 to 71 and 450 to 459; or a fragment thereof conferring an increase in the amount of the fine chemical in an organism or a part thereof
b) nucleic acid molecule comprising, preferably at least the mature form, of the nucleic acid molecule shown in Table I, columns 5 or 7, lines 68 to 71 and 450 to 459 preferably of Table I B, column 7, lines 68 to 71 and 450 to 459; or a fragment thereof conferring an increase in the amount of the fine chemical in an organism or a part thereof;
c) nucleic acid molecule whose sequence can be deduced from a polypeptide sequence encoded by a nucleic acid molecule of (a) or (b) as result of the degeneracy of the genetic code and conferring an increase in the amount of the respective fine chemical in an organism or a part thereof;
d) nucleic acid molecule encoding a polypeptide whose sequence has at least 50% identity with the amino acid sequence of the polypeptide encoded by the nucleic acid molecule of (a) to (c) and conferring an increase in the amount of the respective fine chemical in an organism or a part thereof;
e) nucleic acid molecule which hybridizes with a nucleic acid molecule of (a) to (c) under under stringent hybridisation conditions and conferring an increase in the amount of the respective fine chemical in an organism or a part thereof;
f) nucleic acid molecule encoding a polypeptide, the polypeptide being derived by substituting, deleting and/or adding one or more amino acids of the amino acid sequence of the polypeptide encoded by the nucleic acid molecules (a) to (d), preferably to (a) to (c), and conferring an increase in the amount of the respective fine chemical in an organism or a part thereof;
g) nucleic acid molecule encoding a fragment or an epitope of a polypeptide which is encoded by one of the nucleic acid molecules of (a) to (e), preferably to (a) to (c) and conferring an increase in the amount of the respective fine chemical in an organism or a part thereof;
h) nucleic acid molecule comprising a nucleic acid molecule which is obtained by amplifying a cDNA library or a genomic library using the primers or primer pairs as indicated in Table III, column 7, lines 68 to 71 and 450 to 459 and conferring an increase in the amount of the respective fine chemical in an organism or a part thereof;
i) nucleic acid molecule encoding a polypeptide which is isolated, e.g. from a expression library, with the aid of monoclonal antibodies against a polypeptide encoded by one of the nucleic acid molecules of (a) to (g), preferably to (a) to (c) and conferring an increase in the amount of the respective fine chemical in an organism or a part thereof;
j) nucleic acid molecule which encodes a polypeptide comprising the consensus sequence shown in Table IV, column 7, lines 68 to 71 and 450 to 459 and conferring an increase in the amount of the respective fine chemical in an organism or a part thereof;
k) nucleic acid molecule encoding the amino acid sequence of a polypeptide encoding a domain of the polypeptide shown in Table II, columns 5 or 7, lines 68 to 71 and 450 to 459, preferably of Table II B, column 7, lines 68 to 71 and 450 to 459; and conferring an increase in the amount of the respective fine chemical in an organism or a part thereof; and
l) nucleic acid molecule which is obtainable by screening a suitable nucleic acid library under stringent hybridization conditions with a probe comprising one of the sequences of the nucleic acid molecule of (a) to (k) or with a fragment of at least 15 nt, preferably 20 nt, 30 nt, 50 nt, 100 nt, 200 nt or 500 nt of the nucleic acid molecule characterized in (a) to (h) or of the nucleic acid molecule shown in Table I, columns 5 or 7, lines 68 to 71 and 450 to 459 or a nucleic acid molecule encoding, preferably at least the mature form of, the polypeptide shown in Table II, columns 5 or 7, lines 68 to 71 and 450 to 459, and conferring an increase in the amount of the respective fine chemical in an organism or a part thereof; or which encompasses a sequence which is complementary thereto;
whereby, preferably, the nucleic acid molecule according to (a) to (I) distinguishes over the sequence as depicted in Table IA, columns 5 or 7, lines 68 to 71 and 450 to 459 by one or more nucleotides. In one embodiment, the nucleic acid molecule of the invention does not consist of the sequence shown in Table IA or IB, columns 5 or 7, lines 68 to 71 and 450 to 459. In an other embodiment, the nucleic acid molecule of the present invention is at least 30 % identical and less than 100%, 99,999%, 99,99%, 99,9% or 99% identical to the sequence shown in Table IA or IB, columns 5 or 7, lines 68 to 71 and 450 to 459. In a further embodiment the nucleic acid molecule does not encode the polypeptide sequence shown in Table IIA or IIB, columns 5 or 7, lines 68 to 71 and 450 to 459. Accordingly, in one embodiment, the nucleic acid molecule of the present invention encodes in one embodiment a polypeptide which differs at least in one or more amino acids from the polypeptide as depicted in Table IIA or IIB, columns 5 or 7, lines 68 to 71 and 450 to 459 and therefore does not encode a protein of the sequence shown in Table IIA or IIB, columns 5 or 7, lines 68 to 71 and 450 to 459. Accordingly, in one embodiment, the protein encoded by a sequence of a nucleic acid according to (a) to (I) does not consist of the sequence shown in Table IIA or IIB, columns 5 or 7, lines 68 to 71 and 450 to 459. In a further embodiment, the protein of the present invention is at least 30 % identical to protein sequence depicted in Table IIA or IIB, columns 5 or 7, lines 68 to 71 and 450 to 459 and less than 100%, preferably less than 99,999%, 99,99% or 99,9%, more preferably less than 99%, 985, 97%, 96% or 95% identical to the sequence shown in Table IIA or IIB, columns 5 or 7, lines 68 to 71 and 450 to 459.

**[0205.0.0.6] to [0226.0.0.6]** for the disclosure of the paragraphs [0205.0.0.6] to [0226.0.0.6] see paragraphs [0205.0.0.0] to [0226.0.0.0] above.

**[0227.0.6.6]** The abovementioned nucleic acid molecules can be cloned into the nucleic acid constructs or vectors according to the invention in combination together with further genes, or else different genes are introduced by transforming several nucleic acid constructs or vectors (including plasmids) into a host cell, advantageously into a plant cell or a microorgansims.
In addition to the sequence mentioned in Table I, columns 5 or 7, lines 68 to 71 and 450 to 459 or its derivatives, it is advantageous additionally to express and/or mutate further genes in the organisms. Especially advantageously, additionally at least one further gene of the fatty acid biosynthetic pathway such as for palmitate, palmitoleate, stearate and/or oleate is expressed in the organisms such as plants or microorganisms. It is also possible that the regulation of the natural genes has been modified advantageously so that the gene and/or its gene product is no longer subject to the regulatory mechanisms which exist in the organisms. This leads to an increased synthesis of the amino acids desired since, for example, feedback regulations no longer exist to the same extent or not at all. In addition it might be advantageously to combine the sequences shown in Table I, columns 5 or 7, lines 68 to 71 and 450 to 459 with genes which generally support or enhances to growth or yield of the target organismen, for example genes which lead to faster growth rate of microorganisms or genes which produces stress-, pathogen, or herbicide resistant plants.

**[0228.0.5.6] to [0230.0.5.6]** for the disclosure of the paragraphs [0228.0.5.6] to [0230.0.5.6] see paragraphs [0228.0.0.0] to [0230.0.0.0] above.

**[0231.0.6.6]** In a further advantageous embodiment of the process of the invention, the organisms used in the process are those in which simultaneously a α-linolenic acid degrading protein is attenuated, in particular by reducing the rate of expression of the corresponding gene.

**[0232.0.0.6] to [0276.0.0.6]** for the disclosure of the paragraphs [0232.0.0.6] to [0276.0.0.6] see paragraphs [0232.0.0.0] to [0276.0.0.0] above.

**[0277.0.5.6]** for the disclosure of this paragraph see paragraph [0277.0.5.5] above.

**[0278.0.0.6] to [0282.0.0.6]** for the disclosure of the paragraphs [0278.0.0.6] to [0282.0.0.6] see paragraphs [0278.0.0.0] to [0282.0.0.0] above.

**[0283.0.6.6]** Moreover, native polypeptide conferring the increase of the respective fine chemical in an organism or part thereof can be isolated from cells (e.g., endothelial cells), for example using the antibody of the present invention as described below, in particular, an antibody against polypeptides as shown in Table II, columns 5 or 7, lines 68 to 71 and 450 to 459 or an antigenic part thereof, which can be produced by standard techniques utilizing polypeptides comprising or consisting of abovementioned sequences, e.g. the polypeptid of the present invention or fragment thereof. Preferred are monoclonal antibodies specifically binding to polypeptides as indicated in Table II, columns 5 or 7, lines 68 to 71 and 450 to 459.

**[0284.0.0.6]** for the disclosure of this paragraph see [0284.0.0.0] above.

**[0285.0.6.6]** In one embodiment, the present invention relates to a polypeptide having the sequence shown in Table II, columns 5 or 7, lines 68 to 71 and 450 to 459 or as coded by the nucleic acid molecule shown in Table I, columns 5 or 7, lines 68 to 71 and 450 to 459 or functional homologues thereof.

**[0286.0.6.6]** In one advantageous embodiment, in the method of the present invention the activity of a polypeptide is increased which comprises or consists of a consensus sequence as indicated in Table IV, column 7, lines 68 to 71 and 450 to 459 and in one another embodiment, the present invention relates to a polypeptide comprising or consisting of a consensus sequence as indicated in Table IV, column 7, lines 68 to 71 and 450 to 459, whereby 20 or less, preferably 15 or 10, preferably 9, 8, 7, or 6, more preferred 5 or 4, even more preferred 3, even more preferred 2, even more preferred 1, most preferred 0 of the amino acids positions indicated can be replaced by any amino acid or, in an further embodiment, can be replaced and/or absent. In one embodiment, the present invention relates to the method of the present invention comprising a poylpeptide or to a polypeptide comprising more than one consensus sequences as indicated in Table IV, column 7, lines 68 to 71 and 450 to 459.

**[0287.0.0.6] to [0290.0.0.6]** for the disclosure of the paragraphs [0287.0.0.6] to [0290.0.0.6] see paragraphs [0287.0.0.0] to [0290.0.0.0] above.

**[0291.0.6.6]** In one advantageous embodiment, the method of the present invention comprises the increasing of a polypeptide comprising or consisting of plant or microorganism specific consensus sequences. Accordingly, in one embodiment, the present invention relates to a polypeptide comprising or consisting of a plant or microorganism specific consensus sequences.
In one embodiment, said polypeptide of the invention distinguishes over the sequence as indicated in Table IIA or IIB, columns 5 or 7, lines 68 to 71 and 450 to 459 by one or more amino acids. In one embodiment, polypeptide distinguishes form the sequence shown in Table IIA or IIB, columns 5 or 7, lines 68 to 71 and 450 to 459 by more than 5, 6, 7, 8 or 9 amino acids, preferably by more than 10, 15, 20, 25 or 30 amino acids, evenmore preferred are more than 40, 50, or 60 amino acids and, preferably, the sequence of the polypeptide of the invention distinguishes from the sequence shown in Table IIA or IIB, columns 5 or 7, lines 68 to 71 and 450 to 459 by not more than 80% or 70% of the amino acids, preferably not more than 60% or 50%, more preferred not more than 40% or 30%, even more preferred not more than 20% or 10%. In another embodiment, said polypeptide of the invention does not consist of the sequence shown in Table IIA or IIB, columns 5 or 7, lines 68 to 71 and 450 to 459.

**[0292.0.0.6]** for the disclosure of this paragraph see [0292.0.0.0] above.

**[0293.0.6.6]** In one embodiment, the invention relates to polypeptide conferring an increase in the respective fine chemical in an organism or part being encoded by the nucleic acid molecule of the invention or by a nucleic acid molecule used in the process.
In one embodiment, the polypeptide of the invention has a sequence, which distinguishes from the sequence as shown in Table IIA or IIB, columns 5 or 7, lines 68 to 71 and 450 to 459 by one or more amino acids. In another embodiment, said polypeptide of the invention does not consist of the sequence shown in Table IIA or IIB, columns 5 or 7, lines 68 to 71 and 450 to 459. In a further embodiment, said polypeptide of the present invention is less than 100%, 99,999%, 99,99%, 99,9% or 99% identical. In one embodiment, said polypeptide does not consist of the sequence encoded by the nucleic acid molecules shown in Table IA or IB, columns 5 or 7, lines 68 to 71 and 450 to 459.

**[0294.0.6.6]** In one embodiment, the present invention relates to a polypeptide having an activity of a protein as indicated in Table II, column 3, lines 68 to 71 and 450 to 459, which distinguishes over the sequence as indicated in Table IIA or IIB, columns 5 or 7, lines 68 to 71 and 450 to 459 by one or more amino acids, preferably by more than 5, 6, 7, 8 or 9 amino acids, preferably by more than 10, 15, 20, 25 or 30 amino acids, evenmore preferred are more than 40, 50, or 60 amino acids but even more preferred by less than 70% of the amino acids, more preferred by less than 50%, even more preferred my less than 30% or 25%, more preferred are 20% or 15%, even more preferred are less than 10%.

**[0295.0.0.6], [0296.0.0.6] and [0297.0.5.6]** for the disclosure of the paragraphs [0295.0.0.6], [0296.0.0.6] and [0297.0.5.6] see paragraphs [0295.0.0.0] to [0297.0.0.0] above.

**[00297.1.0.6]** Non-polypeptide of the invention-chemicals are e.g. polypeptides having not the activity and/or the amino acid sequence of a polypeptide indicated in Table II, columns 3, 5 or 7, lines 68 to 71 and 450 to 459.

**[0298.0.6.6]** A polypeptide of the invention can participate in the process of the present invention. The polypeptide or a portion thereof comprises preferably an amino acid sequence which is sufficiently homologous to an amino acid sequence as indicated in Table II, columns 5 or 7, lines 68 to 71 and 450 to 459 such that the protein or portion thereof maintains the ability to confer the activity of the present invention. The portion of the protein is preferably a biologically active portion as described herein. Preferably, the polypeptide used in the process of the invention has an amino acid sequence identical to a sequence as indicated in Table II, columns 5 or 7, lines 68 to 71 and 450 to 459.

**[0299.0.6.6]** Further, the polypeptide can have an amino acid sequence which is encoded by a nucleotide sequence which hybridizes, preferably hybridizes under stringent conditions as described above, to a nucleotide sequence of the nucleic acid molecule of the present invention. Accordingly, the polypeptide has an amino acid sequence which is encoded by a nucleotide sequence that is at least about 35%, 40%, 45%, 50%, 55%, 60%, 65% or 70%, preferably at least about 75%, 80%, 85% or 90, and more preferably at least about 91 %, 92%, 93%, 94% or 95%, and even more preferably at least about 96%, 97%, 98%, 99% or more homologous to one of the amino acid sequences of Table II, columns 5 or 7, lines 68 to 71 and 450 to 459. The preferred polypeptide of the present invention preferably possesses at least one of the activities according to the invention and described herein. A preferred polypeptide of the present invention includes an amino acid sequence encoded by a nucleotide sequence which hybridizes, preferably hybridizes under stringent conditions, to a nucleotide sequence of Table I, columns 5 or 7, lines 68 to 71 and 450 to 459 or which is homologous thereto, as defined above.

**[0300.0.6.6]** Accordingly the polypeptide of the present invention can vary from a sequence as indicated in Table II, columns 5 or 7, lines 68 to 71 and 450 to 459 in amino acid sequence due to natural variation or mutagenesis, as described in detail herein. Accordingly, the polypeptide comprise an amino acid sequence which is at least about 35%, 40%, 45%, 50%, 55%, 60%, 65% or 70%, preferably at least about 75%, 80%, 85% or 90, and more preferably at least about 91 %, 92%, 93%, 94% or 95%, and most preferably at least about 96%, 97%, 98%, 99% or more homologous to an entire amino acid sequence of a sequence as indicated in Table IIA or IIB, columns 5 or 7, lines 68 to 71 and 450 to 459.

**[0301.0.0.6]** for the disclosure of this paragraph see [0301.0.0.0] above.

**[0302.0.6.6]** Biologically active portions of an polypeptide of the present invention include peptides comprising amino acid sequences derived from the amino acid sequence of the polypeptide of the present invention or used in the process of the present invention, e.g., an amino acid sequence shown in Table II, columns 5 or 7, lines 68 to 71 and 450 to 459 or the amino acid sequence of a protein homologous thereto, which include fewer amino acids than a full length polypeptide of the present invention or used in the process of the present invention or the full length protein which is homologous to an polypeptide of the present invention or used in the process of the present invention depicted herein, and exhibit at least one activity of polypeptide of the present invention or used in the process of the present invention.

**[0303.0.0.6]** for the disclosure of this paragraph see [0303.0.0.0] above.

**[0304.0.6.6]** Manipulation of the nucleic acid molecule of the invention or the nucleic acid molecule used in the method of the invention may result in the production of a protein having essentially the activity of the polypeptides as indicated in Table II, columns 5 or 7, lines 68 to 71 and 450 to 459 but having differences in the sequence from said wild-type protein. These proteins may be improved in efficiency or activity, may be present in greater numbers in the cell than is usual, or may be decreased in efficiency or activity in relation to the wild type protein.

**[0305.0.5.6], [0306.0.5.6] and [0306.1.0.6]** for the disclosure of the paragraphs [0305.0.5.6], [0306.0.5.6] and [0306.1.0.6] see paragraphs [0305.0.0.0], [0306.0.0.0] and [0306.1.0.0] above.

**[0307.0.0.6] and [0308.0.0.6]** for the disclosure of the paragraphs [0307.0.0.6] and [0308.0.0.6] see paragraphs [0307.0.0.0 and [0308.0.0.0] above.

**[0309.0.6.6]** In one embodiment, a reference to protein (= polypeptide)" of the invention e.g. as indicated in Table II, columns 5 or 7, lines 68 to 71 and 450 to 459 refers to a polypeptide having an amino acid sequence corresponding to the polypeptide of the invention or used in the process of the invention, whereas a "non-polypeptide" or "other polypeptide" e.g. not indicated in Table II, columns 5 or 7, lines 68 to 71 and 450 to 459 refers to a polypeptide having an amino acid sequence corresponding to a protein which is not substantially homologous to a polypeptide of the invention, preferably which is not substantially homologous to a polypeptide having a protein activity, e.g., a protein which does not confer the activity described herein and which is derived from the same or a different organism.

**[0310.0.0.6] to [0334.0.0.6]** for the disclosure of the paragraphs [0310.0.0.6] to [0334.0.0.6] see paragraphs [0310.0.0.0] to [0334.0.0.0] above.

**[0335.0.6.6]** The dsRNAi method has proved to be particularly effective and advantageous for reducing the expression of a nucleic acid sequence as indicated in Table I, columns 5 or 7, lines 68 to 71 and 450 to 459 and/or homologs thereof. As described inter alia in WO 99/32619, dsRNAi approaches are clearly superior to traditional antisense approaches. The invention therefore furthermore relates to double-stranded RNA molecules (dsRNA molecules) which, when introduced into an organism, advantageously into a plant (or a cell, tissue, organ or seed derived therefrom), bring about altered metabolic activity by the reduction in the expression of a nucleic acid sequences as indicated in Table I, columns 5 or 7, lines 68 to 71 and 450 to 459 and/or homologs thereof. In a double-stranded RNA molecule for reducing the expression of an protein encoded by a nucleic acid sequence of one of the sequences as indicated in Table I, columns 5 or 7, lines 68 to 71 and 450 to 459 and/or homologs thereof, one of the two RNA strands is essentially identical to at least part of a nucleic acid sequence, and the respective other RNA strand is essentially identical to at least part of the complementary strand of a nucleic acid sequence.

**[0336.0.0.6] to [0342.0.0.6]** for the disclosure of the paragraphs [0336.0.0.6] to [0342.0.0.6] see paragraphs [0336.0.0.0] to [0342.0.0.0] above.

**[0343.0.6.6]** As has already been described, 100 % sequence identity between the dsRNA and a gene transcript of a nucleic acid sequence as indicated in Table I, columns 5 or 7, lines 68 to 71 and 450 to 459 or it's homolog is not necessarily required in order to bring about effective reduction in the expression. The advantage is, accordingly, that the method is tolerant with regard to sequence deviations as may be present as a consequence of genetic mutations, polymorphisms or evolutionary divergences. Thus, for example, using the dsRNA, which has been generated starting from a sequence of one of the sequences as indicated in Table I, columns 5 or 7, lines 68 to 71 and 450 to 459 or homologs thereof of the one organism, may be used to suppress the corresponding expression in another organism.

**[0344.0.0.6] to [0350.0.0.6], [0351.0.5.6] and [0352.0.0.6] to [0361.0.0.6]** for the disclosure of the paragraphs [0344.0.0.5] to [0350.0.0.5], [0351.0.5.5] and [0352.0.0.5] to [0361.0.0.5] see paragraphs [0344.0.0.0] to [0361.0.0.0] above.

**[0362.0.6.6]** Accordingly the present invention relates to any cell transgenic for any nucleic acid characterized as part of the invention, e.g. conferring the increase of the respective fine chemical in a cell or an organism or a part thereof, e.g. the nucleic acid molecule of the invention or the nucleic acid molecule used in the method of the invention, the nucleic acid construct of the invention, the antisense molecule of the invention, the vector of the invention or a nucleic acid molecule encoding the polypeptide of the invention, e.g. encoding a polypeptide having an activity of a protein such as the polypeptides as indicated in Table II, column 3, lines 68 to 71 and 450 to 459. Due to the above mentioned activity the fine chemical content in a cell or an organism is increased. For example, due to modulation or manupulation, the cellular activity is increased, e.g. due to an increased expression or specific activity of the subject matters of the invention in a cell or an organism or a part thereof. Transgenic for a polypeptide having an activity of a protein such as the polypeptides as indicated in Table II, column 3, lines 68 to 71 and 450 to 459. Activity means herein that due to modulation or manipulation of the genome, the activity of polypeptide having an activity of a protein such as the polypeptides as indicated in Table II, column 3, lines 68 to 71 and 450 to 459 or a similar activity, which is increased in the cell or organism or part thereof. Examples are described above in context with the process of the invention.

**[0363.0.0.6], [0364.0.5.6] and [0365.0.0.6] to [0379.0.5.6]** for the disclosure of the paragraphs [0363.0.0.6], [0364.0.5.6] and [0365.0.0.6] to [0379.0.5.6] see paragraphs [0363.0.0.0] to [0379.0.0.0] above.

**[0380.0.5.6], [0381.0.0.6] and [0382.0.0.6]** for the disclosure of the paragraphs [0380.0.5.6], [0381.0.0.6] and [0382.0.0.6] see paragraphs [0380.0.5.5], [0381.0.0.0] and [0382.0.0.0] above.

**[0383.0.5.6], [0384.0.0.6], [0385.0.5.6] and [0386.0.5.6]** for the disclosure of the paragraphs [0383.0.5.6], [0384.0.0.6], [0385.0.5.6] and [0386.0.5.6] see paragraphs [0383.0.5.5], [0384.0.0.0], [0385.0.5.5] and [0386.0.5.5] above.

**[0387.0.0.6] to [0392.0.0.6]** for the disclosure of the paragraphs [0387.0.0.6] to [0392.0.0.6] see paragraphs [0387.0.0.0] to [0392.0.0.0] above.

**[0393.0.6.6]** In one embodiment, the present invention relates to a method for the identification of a gene product conferring an increase in the fine chemical production in a cell, comprising the following steps:
(a) contacting,̅ e.g. hybridising, the nucleic acid molecules of a sample, e.g. cells, tissues, plants or microorganisms or a nucleic acid library , which can contain a candidate gene encoding a gene product conferring an increase in the respective fine chemical after expression, with the nucleic acid molecule of the present invention;
(b) identifying the nucleic acid molecules, which hybridize under relaxed stringent conditions with the nucleic acid molecule of the present invention in particular to the nucleic acid molecule sequence shown in Table I, columns 5 or 7, lines 68 to 71 and 450 to 459, preferably in Table I B, columns 5 or 7, lines 68 to 71 and 450 to 459 and, optionally, isolating the full length cDNA clone or complete genomic clone;
(c) introducing the candidate nucleic acid molecules in host cells, preferably in a plant cell or a microorganism, appropriate for producing the respective fine chemical;
(d) expressing the identified nucleic acid molecules in the host cells;
(e) assaying the respective fine chemical level in the host cells; and
(f) identifying the nucleic acid molecule and its gene product which expression confers an increase in the respective fine chemical level in the host cell after expression compared to the wild type.
   **[0394.0.0.6] to [0415.0.0.6] and [0416.0.5.6]** for the disclosure of the paragraphs [0394.0.0.6] to [0415.0.0.6] and [0416.0.5.6] see paragraphs [0394.0.0.0] to [0416.0.0.0] above.
   **[0417.0.5.6] and [0418.0.0.6] to [0430.0.0.6]** for the disclosure of the paragraphs [0417.0.5.6] and [0418.0.0.6] to [0430.0.0.6] see paragraphs [0417.0.5.5] and [0418.0.0.0] to [0430.0.0.0] above.
   **[0431.0.5.6], [0432.0.5.6], [0433.0.0.6] and [0434.0.0.6]** for the disclosure of the paragraphs [0431.0.5.6] , [0432.0.5.6], [0433.0.0.6] and [0434.0.0.6] see paragraphs [0431.0.0.0] to [0434.0.0.0] above.
   **[0435.0.5.6] to [0440.0.5.6]** for the disclosure of the paragraphs [0435.0.5.6] to [0440.0.5.6] see paragraphs [0435.0.5.5] to [0440.0.5.5] above.
   **[0441.0.0.6] and [0442.0.5.6]** for the disclosure of the paragraphs [0441.0.0.6] and [0442.0.5.6] see [0441.0.0.0] and [0442.0.5.5] above.
   **[0443.0.0.6]** for the disclosure of this paragraph see [0443.0.0.0] above.
   **[0444.0.5.6] and [0445.0.5.6]** for the disclosure of the paragraphs [0444.0.5.6] and [0445.0.5.6] see [0444.0.5.5] and [0445.0.5.5] above.
   **[0446.0.0.6] to [0453.0.0.6]** for the disclosure of the paragraphs [0446.0.0.6] to [0453.0.0.6] see paragraphs [0446.0.0.0] to [0453.0.0.0] above.
   **[0454.0.5.6] and [0455.0.5.6]** for the disclosure of the paragraphs [0454.0.5.6] and [0455.0.5.6] see [0454.0.5.5] and [0455.0.5.5] above.
   **[0456.0.0.6]** for the disclosure of this paragraph see [0456.0.0.0] above.
   **[0457.0.5.6] to [0460.0.5.6]** for the disclosure of the paragraphs [0457.0.5.6] to [0460.0.6.6] see paragraphs [0457.0.5.5] to [0460.0.5.5] above.
   **[0461.0.6.6]** Example 10: Cloning SEQ ID NO: 5304 for the expression in plants
   **[0462.0.0.6]** for the disclosure of this paragraph see [0462.0.0.0] above.
   **[0463.0.6.6]** SEQ ID NO: 5304 is amplified by PCR as described in the protocol of the Pfu Turbo or DNA Herculase polymerase (Stratagene).
   **[0464.0.5.6], [0465.0.5.6] and [0466.0.0.6]** for the disclosure of the paragraphs [0464.0.5.6], [0465.0.5.6] and [0466.0.0.6] see paragraphs [0464.0.5.5], [0465.0.5.5] and [0466.0.0.0] above.

**[0467.0.6.6]** The following primer sequences were selected for the gene SEQ ID NO: 5304:
i) forward primer (SEQ ID NO: 5640)
   atggctatcg acgaaaacaa acag
ii) reverse primer (SEQ ID NO: 5641)
   ttaaaaatct tcgttagttt ctgctac

**[0468.0.0.6] to [0479.0.0.6]** for the disclosure of the paragraphs [0468.0.0.6] to [0479.0.0.6] see paragraphs [0468.0.0.0] to [0479.0.0.0] above.

**[0480.0.6.6]** Example 11: Generation of transgenic plants which express SEQ ID NO: 5304

**[0481.0.0.6]** for the disclosure of this paragraph see [0481.0.0.0] above.

**[0482.0.0.6] to [0513.0.0.6]** for the disclosure of the paragraphs [0482.0.0.6] to [0513.0.0.6] see paragraphs [0482.0.0.0] to [0513.0.0.0] above.

**[0514.0.6.6]** As an alternative, the fatty acids can be detected advantageously via HPLC separation in ethanolic extract as described by Geigenberger et al. (Plant Cell & Environ, 19, 1996: 43-55).
The results of the different plant analyses can be seen from the table which follows:

**Table 1**

| **ORF** | Method | Metabolite | Min | Max |
|---|---|---|---|---|
| b2699 | alpha Linolenic Acid (C18:3 (c9,c12,c15)) | GC | 1,13 | 1,32 |
| YER173W | alpha Linolenic Acid (C18:3 (c9,c12,c15)) | GC | 1,15 | 1,54 |
| YGL205W | alpha Linolenic Acid (C18:3 (c9,c12,c15)) | GC | 1,13 | 1,24 |
| YIL150C | alpha Linolenic Acid (C18:3 (c9.c12.c15)) | GC | 3,43 | 3,43 |
| b0050 | alpha Linolenic Acid (C18:3 (c9.c12.c15)) | GC | 1.11 | 1.19 |
| b0251 | alpha Linolenic Acid (C18:3 (c9.c12.c15)) | GC | 1.14 | 1.29 |
| b0376 | alpha Linolenic Acid (C18:3 (c9.c12.c15)) | GC | 1.15 | 1.34 |
| b0577 | alpha Linolenic Acid (C18:3 (c9.c12.c15)) | GC | 1.12 | 1.96 |
| b0849 | alpha Linolenic Acid (C18:3 (c9.c12.c15)) | GC | 1.13 | 1.31 |
| b2822 | alpha Linolenic Acid (C18:3 (c9.c12.c15)) | GC | 1.15 | 1.20 |
| b3129 | alpha Linolenic Acid (C18:3 (c9.c12.c15)) | GC | 1.12 | 1.26 |
| b3457 | alpha Linolenic Acid (C18:3 (c9.c12.c15)) | GC | 1.15 | 1.28 |
| b3462 | alpha Linolenic Acid (C18:3 (c9.c12.c15)) | GC | 1.13 | 1.17 |
| b3644 | alpha Linolenic Acid (C18:3 (c9.c12.c15)) | GC | 1.13 | 1.36 |

**[0515.0.5.6]** Column 2 shows the fatty acid analyzed. Columns 4 and 5 shows the ratio of the analyzed fatty acid between the transgenic plants and the wild type; Increase of the metabolites: Max: maximal x-fold (normalised to wild type)-Min: minimal x-fold (normalised to wild type). Decrease of the metabolites: Max: maximal x-fold (normalised to wild type) (minimal decrease), Min: minimal x-fold (normalised to wild type) (maximal decrease). Column 3 indicates the analytical method.

**[0516.0.0.6] and [0517.0.5.6]** for the disclosure of the paragraphs [0516.0.0.6] and [0517.0.5.6] see paragraphs [0516.0.0.0] and [0517.0.0.0] above.

**[0518.0.0.6] to [0529.0.0.6] and [0530.0.5.6]** for the disclosure of the paragraphs [0518.0.0.6] to [0529.0.0.6] and [0530.0.5.6] see paragraphs [0518.0.0.0] to [0530.0.0.0] above.

**[0530.1.0.6] to [0530.6.0.6]** for the disclosure of the paragraphs [0530.1.0.6] to [0530.6.0.6] see paragraphs [0530.1.0.0] to [0530.6.0.0] above.

**[0531.0.0.6] to [0533.0.0.6] and [0534.0.5.6]** for the disclosure of the paragraphs [0531.0.0.6] to [0533.0.0.6] and [0534.0.5.6] see paragraphs [0531.0.0.0] to [0534.0.0.0] above.

**[0535.0.0.6] to [0537.0.0.6] and [0538.0.5.6]** for the disclosure of the paragraphs [0535.0.0.6] to [0537.0.0.6] and [0538.0.5.6] see paragraphs [0535.0.0.0] to [0538.0.0.0] above.

**[0539.0.0.6] to [0542.0.0.6] and [0543.0.5.6]** for the disclosure of the paragraphs [0539.0.0.6] to [0542.0.0.6] and [0543.0.5.6] see paragraphs [0539.0.0.0] to [0543.0.0.0] above.

**[0544.0.0.6] to [0547.0.0.6] and [0548.0.5.6] to [0552.0.0.6]** for the disclosure of the paragraphs [0544.0.0.6] to [0547.0.0.6] and [0548.0.5.6] to [0552.0.0.6] see paragraphs [0544.0.0.0] to [0552.0.0.0] above.

**[0552.1.0.6]:** Example 15: Metabolite Profiling Info from *Zea mays*
*Zea mays* plants were engineered as described in Example 14c.
The results of the different *Zea mays* plants analysed can be seen from table 2, which follows:

**Table 2**

| **ORF_NAME** | **Metabolite** | **Min** | **Max** |
|---|---|---|---|
| YIL150C | alpha Linolenic Acid (C18:3 (c9,c12,c15)) | 1.469 | 2.241 |

Table 2 shows the increase in α-linolenic acid in genetically modified corn plants expressing the Saccharomyces cerevisiae nucleic acid sequence YIL150c.
In one embodiment, in case the activity of the Saccaromyces cerevisiae protein YIL150C or its homologs, e.g. a "chromatin binding protein, required for S-phase (DNA synthesis) initiation or completion" or its homologs, is increased in corn plants, preferably, an increase of the fine chemical α-linolenic acid between 46% and 124% is conferred.

**[0552.2.0.6]** for the disclosure of this paragraph see [0552.2.0.0] above.

**[0553.0.6.6]**
1. A process for the production of α-linolenic acid, which comprises
   (a) increasing or generating the activity of a protein as indicated in Table II, columns 5 or 7, lines 68 to 71 and 450 to 459 or a functional equivalent thereof in a non-human organism, or in one or more parts thereof; and
   (b) growing the organism under conditions which permit the production of α-linolenic acid in said organism.
2. A process for the production of α-linolenic acid, comprising the increasing or generating in an organism or a part thereof the expression of at least one nucleic acid molecule comprising a nucleic acid molecule selected from the group consisting of:
   a) nucleic acid molecule encoding of a polypeptide as indicated in Table II, columns 5 or 7, lines 68 to 71 and 450 to 459 or a fragment thereof, which confers an increase in the amount of α-linolenic acid in an organism or a part thereof;
   b) nucleic acid molecule comprising of a nucleic acid molecule as indicated in Table I, columns 5 or 7, lines 68 to 71 and 450 to 459;
   c) nucleic acid molecule whose sequence can be deduced from a polypeptide sequence encoded by a nucleic acid molecule of (a) or (b) as a result of the degeneracy of the genetic code and conferring an increase in the amount of α-linolenic acid in an organism or a part thereof;
   d) nucleic acid molecule which encodes a polypeptide which has at least 50% identity with the amino acid sequence of the polypeptide encoded by the nucleic acid molecule of (a) to (c) and conferring an increase in the amount of α-linolenic acid in an organism or a part thereof;
   e) nucleic acid molecule which hybidizes with a nucleic acid molecule of (a) to (c) under stringent hybridisation conditions and conferring an increase in the amount of α-linolenic acid in an organism or a part thereof;
   f) nucleic acid molecule which encompasses a nucleic acid molecule which is obtained by amplifying nucleic acid molecules from a cDNA library or a genomic library using the primers or primer pairs as indicated in Table III, column 7, lines 68 to 71 and 450 to 459 and conferring an increase in the amount of α-linolenic acid in an organism or a part thereof;
   g) nucleic acid molecule encoding a polypeptide which is isolated with the aid of monoclonal antibodies against a polypeptide encoded by one of the nucleic acid molecules of (a) to (f) and conferring an increase in the amount of α-linolenic acid in an organism or a part thereof;
   h) nucleic acid molecule encoding a polypeptide comprising a consensus sequence as indicated in Table IV, column 7, lines 68 to 71 and 450 to 459 and conferring an increase in the amount of α-linolenic acid in an organism or a part thereof; and
   i) nucleic acid molecule which is obtainable by screening a suitable nucleic acid library under stringent hybridization conditions with a probe comprising one of the sequences of the nucleic acid molecule of (a) to (k) or with a fragment thereof having at least 15 nt, preferably 20 nt, 30 nt, 50 nt, 100 nt, 200 nt or 500 nt of the nucleic acid molecule characterized in (a) to (k) and conferring an increase in the amount of α-linolenic acid in an organism or a part thereof.
   or comprising a sequence which is complementary thereto.
3. The process of claim 1 or 2, comprising recovering of the free or bound α-linolenic acid.
4. The process of any one of claim 1 to 3, comprising the following steps:
   (a) selecting an organism or a part thereof expressing a polypeptide encoded by the nucleic acid molecule characterized in claim 2;
   (b) mutagenizing the selected organism or the part thereof;
   (c) comparing the activity or the expression level of said polypeptide in the mutagenized organism or the part thereof with the activity or the expression of said polypeptide of the selected organisms or the part thereof;
   (d) selecting the mutated organisms or parts thereof, which comprise an increased activity or expression level of said polypeptide compared to the selected organism or the part thereof;
   (e) optionally, growing and cultivating the organisms or the parts thereof; and
   (f) recovering, and optionally isolating, the free or bound α-linolenic acid produced by the selected mutated organisms or parts thereof.
5. The process of any one of claims 1 to 4, wherein the activity of said protein or the expression of said nucleic acid molecule is increased or generated transiently or stably.
6. An isolated nucleic acid molecule comprising a nucleic acid molecule selected from the group consisting of:
   a) nucleic acid molecule encoding of a polypeptide as indicated in Table II, columns 5 or 7, lines 68 to 71 and 450 to 459 or a fragment thereof, which confers an increase in the amount of α-linolenic acid in an organism or a part thereof;
   b) nucleic acid molecule comprising of a nucleic acid molecule as indicated in Table I, columns 5 or 7, lines 68 to 71 and 450 to 459;
   c) nucleic acid molecule whose sequence can be deduced from a polypeptide sequence encoded by a nucleic acid molecule of (a) or (b) as a result of the degeneracy of the genetic code and conferring an increase in the amount of α-linolenic acid in an organism or a part thereof;
   d) nucleic acid molecule which encodes a polypeptide which has at least 50% identity with the amino acid sequence of the polypeptide encoded by the nucleic acid molecule of (a) to (c) and conferring an increase in the amount of α-linolenic acid in an organism or a part thereof;
   e) nucleic acid molecule which hybidizes with a nucleic acid molecule of (a) to (c) under stringent hybridisation conditions and conferring an increase in the amount of α-linolenic acid in an organism or a part thereof;
   f) nucleic acid molecule which encompasses a nucleic acid molecule which is obtained by amplifying nucleic acid molecules from a cDNA library or a genomic library using the primers or primer pairs as indicated in Table III, column 7, lines 68 to 71 and 450 to 459 and conferring an increase in the amount of α-linolenic acid in an organism or a part thereof;
   g) nucleic acid molecule encoding a polypeptide which is isolated with the aid of monoclonal antibodies against a polypeptide encoded by one of the nucleic acid molecules of (a) to (f) and conferring an increase in the amount of α-linolenic acid in an organism or a part thereof;
   h) nucleic acid molecule encoding a polypeptide comprising a consensus sequence as indicated in Table IV, column 7, lines 68 to 71 and 450 to 459 and conferring an increase in the amount of α-linolenic acid in an organism or a part thereof; and
   i) nucleic acid molecule which is obtainable by screening a suitable nucleic acid library under stringent hybridization conditions with a probe comprising one of the sequences of the nucleic acid molecule of (a) to (k) or with a fragment thereof having at least 15 nt, preferably 20 nt, 30 nt, 50 nt, 100 nt, 200 nt or 500 nt of the nucleic acid molecule characterized in (a) to (k) and conferring an increase in the amount of α-linolenic acid in an organism or a part thereof.
      whereby the nucleic acid molecule distinguishes over the sequence as indicated in Table I A, columns 5 or 7, lines 68 to 71 and 450 to 459 by one or more nucleotides.
7. A nucleic acid construct which confers the expression of the nucleic acid molecule of claim 6, comprising one or more regulatory elements.
8. A vector comprising the nucleic acid molecule as claimed in claim 6 or the nucleic acid construct of claim 7.
9. The vector as claimed in claim 8, wherein the nucleic acid molecule is in operable linkage with regulatory sequences for the expression in a prokaryotic or eukaryotic, or in a prokaryotic and eukaryotic, host.
10. A host cell, which has been transformed stably or transiently with the vector as claimed in claim 8 or 9 or the nucleic acid molecule as claimed in claim 6 or the nucleic acid construct of claim 7 or produced as described in claim any one of claims 2 to 5.
11. The host cell of claim 10, which is a transgenic host cell.
12. The host cell of claim 10 or 11, which is a plant cell, an animal cell, a microorganism, or a yeast cell, a fungus cell, a prokaryotic cell, an eukaryotic cell or an archaebacterium.
13. A process for producing a polypeptide, wherein the polypeptide is expressed in a host cell as claimed in any one of claims 10 to 12.
14. A polypeptide produced by the process as claimed in claim 13 or encoded by the nucleic acid molecule as claimed in claim 6 whereby the polypeptide distinguishes over a sequence as indicated in Table II A, columns 5 or 7, lines 68 to 71 and 450 to 459 by one or more amino acids
15. An antibody, which binds specifically to the polypeptide as claimed in claim 14.
16. A plant tissue, propagation material, harvested material or a plant comprising the host cell as claimed in claim 12 which is plant cell or an Agrobacterium.
17. A method for screening for agonists and antagonists of the activity of a polypeptide encoded by the nucleic acid molecule of claim 6 conferring an increase in the amount of α-linolenic acid in an organism or a part thereof comprising:
   (a) contacting cells, tissues , plants or microorganisms which express the a polypeptide encoded by the nucleic acid molecule of claim 6 conferring an increase in the amount of α-linolenic acid in an organism or a part thereof with a candidate compound or a sample comprising a plurality of compounds under conditions which permit the expression the polypeptide;
   (b) assaying the α-linolenic acid level or the polypeptide expression level in the cell, tissue, plant or microorganism or the media the cell, tissue, plant or microorganisms is cultured or maintained in; and
   (c) identifying a agonist or antagonist by comparing the measured α-linolenic acid level or polypeptide expression level with a standard of α-linolenic acid or polypeptide expression level measured in the absence of said candidate compound or a sample comprising said plurality of compounds, whereby an increased level over the standard indicates that the compound or the sample comprising said plurality of compounds is an agonist and a decreased level over the standard indicates that the compound or the sample comprising said plurality of compounds is an antagonist.
18. A process for the identification of a compound conferring increased α-linolenic acid production in a plant or microorganism, comprising the steps:
   (a) culturing a plant cell or tissue or microorganism or maintaining a plant expressing the polypeptide encoded by the nucleic acid molecule of claim 6 conferring an increase in the amount of α-linolenic acid in an organism or a part thereof and a readout system capable of interacting with the polypeptide under suitable conditions which permit the interaction of the polypeptide with dais readout system in the presence of a compound or a sample comprising a plurality of compounds and capable of providing a detectable signal in response to the binding of a compound to said polypeptide under conditions which permit the expression of said readout system and of the polypeptide encoded by the nucleic acid molecule of claim 6 conferring an increase in the amount of α-linolenic acid in an organism or a part thereof;
   (b) identifying if the compound is an effective agonist by detecting the presence or absence or increase of a signal produced by said readout system.
19. A method for the identification of a gene product conferring an increase in α-linolenic acid production in a cell, comprising the following steps:
   (a) contacting the nucleic acid molecules of a sample, which can contain a candidate gene encoding a gene product conferring an increase in α-linolenic acid after expression with the nucleic acid molecule of claim 6;
   (b) identifying the nucleic acid molecules, which hybridise under relaxed stringent conditions with the nucleic acid molecule of claim 6;
   (c) introducing the candidate nucleic acid molecules in host cells appropriate for producing α-linolenic acid;
   (d) expressing the identified nucleic acid molecules in the host cells;
   (e) assaying the α-linolenic acid level in the host cells; and
   (f) identifying nucleic acid molecule and its gene product which expression confers an increase in the α-linolenic acid level in the host cell in the host cell after expression compared to the wild type.
20. A method for the identification of a gene product conferring an increase in α-linolenic acid production in a cell, comprising the following steps:
   (a) identifiying in a data bank nucleic acid molecules of an organism; which can contain a candidate gene encoding a gene product conferring an increase in the α-linolenic acid amount or level in an organism or a part thereof after expression, and which are at least 20% homolog to the nucleic acid molecule of claim 6;
   (b) introducing the candidate nucleic acid molecules in host cells appropriate for producing α-linolenic acid;
   (c) expressing the identified nucleic acid molecules in the host cells;
   (d) assaying the α-linolenic acid level in the host cells; and
   (e) identifying nucleic acid molecule and its gene product which expression confers an increase in the α-linolenic acid level in the host cell after expression compared to the wild type.
21. A method for the production of an agricultural composition comprising the steps of the method of any one of claims 17 to 20 and formulating the compound identified in any one of claims 17 to 20 in a form acceptable for an application in agriculture.
22. A composition comprising the nucleic acid molecule of claim 6, the polypeptide of claim 14, the nucleic acid construct of claim 7, the vector of any one of claims 8 or 9, an antagonist or agonist identified according to claim 17, the compound of claim 18, the gene product of claim 19 or 20, the antibody of claim 15, and optionally an agricultural acceptable carrier.
23. Use of the nucleic acid molecule as claimed in claim 6 for the identification of a nucleic acid molecule conferring an increase of α-linolenic acid after expression.
24. Use of the polypeptide of claim 14 or the nucleic acid construct claim 7 or the gene product identified according to the method of claim 19 or 20 for identifying compounds capable of conferring a modulation of α-linolenic acid levels in an organism.
25. Food or feed composition comprising the nucleic acid molecule of claim 6, the polypeptide of claim 14, the nucleic acid construct of claim 7, the vector of claim 8 or 9, the antagonist or agonist identified according to claim 17, the antibody of claim 15, the plant or plant tissue of claim 16, the harvested material of claim 16, the host cell of claim 10 to 12 or the gene product identified according to the method of claim 19 or 20.
26. Use of the nucleic acid molecule of claim 6, the polypeptide of claim 14, the nucleic acid construct of claim 7, the vector of claim 8 or 9, the antagonist or agonist identified according to claim 17, the antibody of claim 15, the plant or plant tissue of claim 16, the host cell of claim 10 to 12 or the gene product identified according to the method of claim 19 or 20 for the protection of a plant against a α-linolenic acid synthesis inhibiting herbicide.

**[0554.0.0.6] Abstract: see [0554.0.0.0]**
Process for the production of fine chemicals

**[0000.0.0.7]** In a further embodiment, the present invention relates to a further process for the production of fine chemicals as defined below and the corresponding embodiments as described herein as follows.

**[0001.0.0.7]** for the disclosure of this paragraph see [0001.0.0.0].

**[0002.0.7.7]** Fatty acids and triglycerides have numerous applications in the food and feed industry, in cosmetics and in the drug sector. Depending on whether they are free saturated or unsaturated fatty acids or triglycerides with an increased content of saturated or unsaturated fatty acids, they are suitable for the most varied applications; thus, for example, polyunsaturated f\atty acids (= PUFAs) are added to infant formula to increase its nutritional value. The various fatty acids and triglycerides are mainly obtained from microorganisms such as fungi or from oil-producing plants including phytoplankton and algae, such as soybean, oilseed rape, sunflower and others, where they are usually obtained in the form of their triacylglycerides.

**[0003.0.7.7]** Stearic acid (= octadecanoic acid) is one of the many useful types of saturated fatty acids that comes from many animal and vegetable fats and oils. It is a waxy solid that melts at around 70°C. Commenly stearic acid is either prepared by treating animal fat with water at a high pressure and temperature or starting with vegetable oils by hydrogenation of said oils. It is useful as an ingredient in making candles, soaps, and cosmetics and for softening rubber.

**[0004.0.7.7]** Principally microorganisms such as Mortierella or oil producing plants such as soybean, rapeseed or sunflower or algae such as Crytocodinium or Phaeodactylum are a common source for oils containing fatty acids, where they are usually obtained in the form of their triacyl glycerides. Alternatively, they are obtained advantageously from animals, such as fish. The free fatty acids are prepared advantageously by hydrolysis with a strong base such as potassium or sodium hydroxide.

**[0005.0.5.7]** for the disclosure of this paragraph see [0005.0.5.5] above.

**[0006.0.7.7]** Unlike most saturated fats, stearic acid does not seem to increase cholesterol levels in the blood, because liver enzymes convert it to an unsaturated fat during digestion.

**[0007.0.7.7]** Stearic acid is the most common one of the long-chain fatty acids. It is found in many foods, such as beef fat, and cocoa butter. It is widely used as mentioned above as a lubricant, in soaps, cosmetics, food packaging, deodorant sticks, toothpastes, and as a softener in rubber.

**[0008.0.7.7]** Encouraging research shows that stearic acid, one of the components of the fat found in the cocoa butter of chocolate, may have some positive effects on platelets. The mechanism believed to be responsible for the potential platelet activation by stearic acid involves Arachidonic metabolism, which includes thromboxane A2, a potent aggregating compound, and prostaglandin 12, a potent anti-aggregating compound.

**[0009.0.7.7]** As described above, fatty acids are used in a lot of different applications, for example in cosmetics, pharmaceuticals and in feed and food.

**[0010.0.7.7]** Therefore improving the productivity of such fatty acids and improving the quality of foodstuffs and animal feeds is an important task of the different industries.

**[0011.0.7.7]** To ensure a high productivity of certain fatty acids in plants or microorganism, it is necessary to manipulate the natural biosynthesis of fatty acids in said organism.

**[0012.0.7.7]** Accordingly, there is still a great demand for new and more suitable genes which encode enzymes which participate in the biosynthesis of fatty acids and make it possible to produce certain fatty acids specifically on an industrial scale without unwanted byproducts forming. In the selection of genes for biosynthesis two characteristics above all are particularly important. On the one hand, there is as ever a need for improved processes for obtaining the highest possible contents of fatty acids on the other hand as less as possible byproducts should be produced in the production process.

**[0013.0.0.7]** for the disclosure of this paragraph see [0013.0.0.0] above.

**[0014.0.7.7]** Accordingly, in a first embodiment, the invention relates to a process for the production of a fine chemical, whereby the fine chemical is stearic acid or tryglycerides, lipids, oils or fats containing stearic acid. Accordingly, in the present invention, the term "the fine chemical" as used herein relates to "stearic acid and/or tryglycerides, lipids, oils and/or fats containing stearic acid". Further, the term "the fine chemicals" as used herein also relates to fine chemicals comprising stearic acid and/or triglycerides, lipids, oils and/or fats containing stearic acid.

**[0015.0.7.7]** In one embodiment, the term "the fine chemical" means stearic acid and/or tryglycerides, lipids, oils and/or fats containing stearic acid. Throughout the specification the term "the fine chemical" means stearic acid and/or tryglycerides, lipids, oils and/or fats containing stearic acid, stearic acid and its salts, ester, thioester or stearic acid in free form or bound to other compounds such as triglycerides, glycolipids, phospholipids etc. In a preferred embodiment, the term "the fine chemical" means stearic acid, in free form or its salts or bound to triglycerides. Triglycerides, lipids, oils, fats or lipid mixture thereof shall mean any triglyceride, lipid, oil and/or fat containing any bound or free stearic acid for example sphingolipids, phosphoglycerides, lipids, glycolipids such as glycosphingolipids, phospholipids such as phosphatidylethanolamine, phosphatidylcholine, phosphatidylserine, phosphatidylglycerol, phosphatidylinositol or diphosphatidylglycerol, or as monoacylglyceride, diacylglyceride or triacylglyceride or other fatty acid esters such as acetyl-Coenzym A thioester, which contain further saturated or unsaturated fatty acids in the fatty acid molecule.
In one embodiment, the term "the fine chemical" and the term "the respective fine chemical" mean at least one chemical compound with an activity of the above mentioned fine chemical.

**[0016.0.7.7]** Accordingly, the present invention relates to a process comprising
(a) increasing or generating the activity of a b2699, b2095, b3256, b2699, b1093, YOR024W, YBR089C, YFR042W, YIL150C, YDR513W, YLR010C, b0161, b1896 and/or b3457 protein(s) or of a protein having the sequence of a polypeptide encoded by a nucleic acid molecule indicated in Table I, columns 5 or 7, lines 72 to 81 and 460 to 462 in a non-human organism in one or more parts thereof and
(b) growing the organism under conditions which permit the production of the fine chemical, thus, stearic acid or fine chemicals comprising stearic acid, in said organism.
Accordingly, the present invention relates to a process for the production of a fine chemical comprising
(a) increasing or generating the activity of one or more proteins having the activity of a protein indicated in Table II, column 3, lines 72 to 81 and 460 to 462 or having the sequence of a polypeptide encoded by a nucleic acid molecule indicated in Table I, column 5 or 7, lines 72 to 81 and 460 to 462, in a non-human organism in one or more parts thereof and
(b) growing the organism under conditions which permit the production of the fine chemical, in particular stearic acid.

**[0017.0.0.7] and [0018.0.0.7]** for the disclosure of the paragraphs [0017.0.0.7] and [0018.0.0.7] see paragraphs [0017.0.0.0] and [0018.0.0.0] above.

**[0019.0.7.7]** Advantageously the process for the production of the fine chemical leads to an enhanced production of the fine chemical. The terms "enhanced" or "increase" mean at least a 10%, 20%, 30%, 40% or 50%, preferably at least 60%, 70%, 80%, 90% or 100%, more preferably 150%, 200%, 300%, 400% or 500% higher production of the fine chemical in comparison to the reference as defined below, e.g. that means in comparison to an organism without the aforementioned modification of the activity of a protein having the activity of a protein indicated in Table II, column 3, lines 72 to 81 and 460 to 462 or encoded by nucleic acid molecule indicated in Table I, columns 5 or 7, lines 72 to 81 and 460 to 462.

**[0020.0.7.7]** Surprisingly it was found, that the transgenic expression of at least one of the Saccaromyces cerevisiae protein(s) indicated in Table II, Column 3, lines 75 to 80 and/or the Escherichia coli K12 protein(s) indicated in Table II, Column 3, lines 72 to 74, 81 and 460 to 462 in Arabidopsis thaliana conferred an increase in the stearic acid (or fine chemical) content of the transformed plants.

**[0021.0.0.7]** for the disclosure of this paragraph see [0021.0.0.0] above.

**[0022.0.7.7]** The sequence of b3256 from Escherichia coli K12 has been published in Blattner et al., Science 277(5331), 1453-1474, 1997, and its activity is being defined as acetyl CoA carboxylase. Accordingly, in one embodiment, the process of the present invention comprises the use of a acetyl CoA carboxylase from E. coli or its homolog, e.g. as shown herein, for the production of the fine chemical, meaning of stearic acid and/or tryglycerides, lipids, oils and/or fats containing stearic acid, in particular for increasing the amount of stearic acid and/or tryglycerides, lipids, oils and/or fats containing stearic acid, preferably stearic acid in free or bound form in an organism or a part thereof, as mentioned. In one embodiment, in the process of the present invention the activity of a acetyl CoA carboxylase is increased or generated, e.g. from E. coli or a homolog thereof.
The sequence of b2699 from Escherichia coli K12 has been published in Blattner et al., Science 277(5331), 1453-1474, 1997, and its activity is being defined as DNA-dependent ATPase or DNA- and ATP-dependent coprotease. Accordingly, in one embodiment, the process of the present invention comprises the use of a DNA-dependent ATPase or DNA- and ATP-dependent coprotease from E. coli or its homolog, e.g. as shown herein, for the production of the fine chemical, meaning of stearic acid and/or tryglycerides, lipids, oils and/or fats containing stearic acid, in particular for increasing the amount of stearic and/or tryglycerides, lipids, oils and/or fats containing stearic acid, preferably stearic acid in free or bound form in an organism or a part thereof, as mentioned. In one embodiment, in the process of the present invention the activity of a DNA-dependent ATPase or DNA- and ATP-dependent coprotease is increased or generated, e.g. from E. coli or a homolog thereof.
The sequence of b2095 from Escherichia coli K12 has been published in Blattner et al., Science 277(5331), 1453-1474, 1997, and its activity is being defined as putative tagatose-6-phosphate kinase 1. Accordingly, in one embodiment, the process of the present invention comprises the use of a putative tagatose-6-phosphate kinase 1 from E. coli or its homolog, e.g. as shown herein, for the production of the fine chemical, meaning of stearic acid and/or tryglycerides, lipids, oils and/or fats containing stearic acid, in particular for increasing the amount of stearic acid and/or tryglycerides, lipids, oils and/or fats containing stearic acid, preferably stearic acid in free or bound form in an organism or a part thereof, as mentioned. In one embodiment, in the process of the present invention the activity of a putative tagatose-6-phosphate kinase 1 is increased or generated, e.g. from E. coli or a homolog thereof.
The sequence of b1093 from Escherichia coli K12 has been published in Blattner et al., Science 277(5331), 1453-1474, 1997, and its activity is being defined as 3-oxoacyl-[acyl-carrier-protein] reductase. Accordingly, in one embodiment, the process of the present invention comprises the use of a 3-oxoacyl-[acyl-carrier-protein] reductase from E. coli or its homolog, e.g. as shown herein, for the production of the fine chemical, meaning of stearic acid and/or tryglycerides, lipids, oils and/or fats containing stearic acid, in particular for increasing the amount of stearic acid and/or tryglycerides, lipids, oils and/or fats containing stearic acid, preferably stearic acid in free or bound form in an organism or a part thereof, as mentioned. In one embodiment, in the process of the present invention the activity of a 3-oxoacyl-[acyl-carrier-protein] reductase is increased or generated, e.g. from E. coli or a homolog thereof.
The sequence of YOR024W from Saccharomyces cerevisiae has been submitted from de Haan to the EMBL Protein Sequence Database, July 1996 and its cellular activity has not been characterized yet. It is probably a membrane protein. Accordingly, in one embodiment, the process of the present invention comprises the use of YOR024W, from Saccharomyces cerevisiae or its homolog, e.g. as shown herein, for the production of the fine chemical, meaning of stearic acid and/or tryglycerides, lipids, oils and/or fats containing stearic acid, in particular for increasing the amount of stearic acid and/or tryglycerides, lipids, oils and/or fats containing stearic acid, preferably stearic acid in free or bound form in an organism or a part thereof, as mentioned. In one embodiment, in the process of the present invention the activity of a YOR024W is increased or generated, e.g. from Saccharomyces cerevisiae or a homolog thereof. The sequence of YBR089C-A from Saccharomyces cerevisiae has been published in Feldmann et al., EMBO J., 13 (24), 5795-5809 (1994) and Goffeau et al., Science 274 (5287), 546-547, 1996, and its cellular activity has not been characterized yet. It shows homology to mammalian high mobility group proteins 1 and 2. Its function may be redundantly with the highly homologous gene NHP6A. Furthermore it shows homology to the high-mobility group non-histone chromatin protein Nhp6bp. Accordingly, in one embodiment, the process of the present invention comprises the use of a YBR089C-A activity from Saccharomyces cerevisiae or its homolog, e.g. as shown herein, for the production of the fine chemical, meaning of stearic acid and/or tryglycerides, lipids, oils and/or fats containing stearic acid, in particular for increasing the amount of stearic acid and/or tryglycerides, lipids, oils and/or fats containing stearic acid, preferably stearic acid in free or bound form in an organism or a part thereof, as mentioned. In one embodiment, in the process of the present invention the activity of a YBR089C-A protein is increased or generated, e.g. from Saccharomyces cerevisiae or a homolog thereof.
The sequence of YFR042W from Saccharomyces cerevisiae has been published in Murakami et al., Nat. Genet. 10 (3), 261-268 (1995) and Goffeau et al., Science 274 (5287), 546-547, 1996, and its cellular activity has not been clearly characterized yet. It seams to be a "protein, which is required for cell viability". Accordingly, in one embodiment, the process of the present invention comprises the use of a YFR042W activity from Saccharomyces cerevisiae or its homolog, e.g. as shown herein, for the production of the fine chemical, meaning of stearic acid and/or tryglycerides, lipids, oils and/or fats containing stearic acid, in particular for increasing the amount of stearic acid and/or tryglycerides, lipids, oils and/or fats containing stearic acid, preferably stearic acid in free or bound form in an organism or a part thereof, as mentioned. In one embodiment, in the process of the present invention the activity of a YFR042W protein is increased or generated, e.g. from Saccharomyces cerevisiae or a homolog thereof. The sequence of YIL150C from Saccharomyces cerevisiae has been published in Churcher et al., Nature 387 (6632 Suppl), 84-87 (1997) and in Goffeau et al., Science 274 (5287), 546-547, 1996 and its cellular activity has not been characterized yet. It seams to be a "protein required for S-phase (DNA synthesis) initiation or completion".
Accordingly, in one embodiment, the process of the present invention comprises the use of YIL150C, from Saccharomyces cerevisiae or its homolog, e.g. as shown herein, for the production of the fine chemical, meaning of stearic acid and/or tryglycerides, lipids, oils and/or fats containing stearic acid, in particular for increasing the amount of stearic acid and/or tryglycerides, lipids, oils and/or fats containing stearic acid, preferably stearic acid in free or bound form in an organism or a part thereof, as mentioned. In one embodiment, in the process of the present invention the activity of a YIL150C protein is increased or generated, e.g. from Saccharomyces cerevisiae or a homolog thereof.
The sequence of YDR513w from Saccharomyces cerevisiae has been published in Jacq et al., Nature 387 (6632 Suppl), 75-78 (1997) and in Goffeau et al., Science 274 (5287), 546-547, 1996 and its cellular activity has characterized as glutaredoxin (thioltransferase, glutathione reductase. Accordingly, in one embodiment, the process of the present invention comprises the use of YDR513w, from Saccharomyces cerevisiae or its homolog, e.g. as shown herein, for the production of the fine chemical, meaning of stearic acid and/or tryglycerides, lipids, oils and/or fats containing stearic acid, in particular for increasing the amount of stearic acid and/or tryglycerides, lipids, oils and/or fats containing stearic acid, preferably stearic acid in free or bound form in an organism or a part thereof, as mentioned. In one embodiment, in the process of the present invention the activity of a glutaredoxin is increased or generated, e.g. from Saccharomyces cerevisiae or a homolog thereof.
The sequence of YLR010c from Saccharomyces cerevisiae has been published in Johnston et al., Nature 387 (6632 Suppl), 87-90 (1997) and in Goffeau et al., Science 274 (5287), 546-547, 1996 and its cellular activity has not been characterized yet. It seams to be a "protein involved in telomeric pathways in association with Stn1". Accordingly, in one embodiment, the process of the present invention comprises the use of YLR010C, from Saccharomyces cerevisiae or its homolog, e.g. as shown herein, for the production of the fine chemical, meaning of stearic acid and/or tryglycerides, lipids, oils and/or fats containing stearic acid, in particular for increasing the amount of stearic acid and/or tryglycerides, lipids, oils and/or fats containing stearic acid, preferably stearic acid in free or bound form in an organism or a part thereof, as mentioned. In one embodiment, in the process of the present invention the activity of a YLR010C protein is increased or generated, e.g. from Saccharomyces cerevisiae or a homolog thereof.
The sequence of b0161 (Accession number NP_414703) from Escherichia coli K12 has been published in Blattner et al., Science 277 (5331), 1453-1474, 1997, and its activity is being defined as a periplasmic serine protease (heat shock protein).
Accordingly, in one embodiment, the process of the present invention comprises the use of a periplasmic serine protease from E. coli or its homolog, e.g. as shown herein, for the production of the fine chemical, meaning of stearic acid and/or tryglycerides, lipids, oils and/or fats containing stearic acid, in particular for increasing the amount of stearic acid and/or tryglycerides, lipids, oils and/or fats containing stearic acid, preferably stearic acid in free or bound form in an organism or a part thereof, as mentioned. In one embodiment, in the process of the present invention the activity of a periplasmic serine protease is increased or generated, e.g. from E. coli or a homolog thereof.
The sequence of b1896 (Accession number NP_416410) from Escherichia coli K12 has been published in Blattner et al., Science 277 (5331), 1453-1474, 1997, and its activity is being defined as a trehalose-6-phosphate synthase. Accordingly, in one embodiment, the process of the present invention comprises the use of a trehalose-6-phosphate synthase from E. coli or its homolog, e.g. as shown herein, for the production of the fine chemical, meaning of stearic acid and/or tryglycerides, lipids, oils and/or fats containing stearic acid, in particular for increasing the amount of stearic acid and/or tryglycerides, lipids, oils and/or fats containing stearic acid, preferably stearic acid in free or bound form in an organism or a part thereof, as mentioned. In one embodiment, in the process of the present invention the activity of a trehalose-6-phosphate synthase is increased or generated, e.g. from E. coli or a homolog thereof. The sequence of b3457 (Accession number NP_417914) from Escherichia coli K12 has been published in Blattner et al., Science 277 (5331), 1453-1474, 1997, and its activity is being defined as a high-affinity branched-chain amino acid transport protein (ABC superfamily). Accordingly, in one embodiment, the process of the present invention comprises the use of a high-affinity branched-chain amino acid transport protein from E. coli or its homolog, e.g. as shown herein, for the production of the fine chemical, meaning of stearic acid and/or tryglycerides, lipids, oils and/or fats containing stearic acid, in particular for increasing the amount of stearic acid and/or tryglycerides, lipids, oils and/or fats containing stearic acid, preferably stearic acid in free or bound form in an organism or a part thereof, as mentioned. In one embodiment, in the process of the present invention the activity of a high-affinity branched-chain amino acid transport protein is increased or generated, e.g. from E. coli or a homolog thereof.

**[0023.0.7.7]** Homologues ( = homologs) of the present gene products can be derived from any organisms as long as the homologue confers the herein mentioned activity, in particular, confers an increase in the fine chemical amount or content. Further, in the present invention, the term "homologue" relates to the sequence of an organism having the highest sequence homology to the herein mentioned or listed sequences of all expressed sequences of said organism.
However, the person skilled in the art knows, that, preferably, the homologue has said fine-chemical increasing activity and, if known, the same biological function or activity in the organism as at least one of the protein(s) indicated in Table II, Column 3, lines 72 to 81 and 460 to 462, e.g. having the sequence of a polypeptide encoded by a nucleic acid molecule comprising the sequence indicated in Table I, Column 5 or 7, lines 72 to 81 and 460 to 462.
In one embodiment, the homolog of any one of the polypeptides indicated in Table II, lines 75 to 80 is a homolog having the same or a similar acitivity, in particular an increase of activity confers an increase in the content of the fine chemical in the organsims and being derived from an Eukaryot. In one embodiment, the homolog of a polypeptide indicated in Table II, column 3, lines 72 to 74, 81 and 460 to 462 is a homolog having the same or a similar acitivity, in particular an increase of activity confers an increase in the content of the fine chemical in the organisms or part thereof, and being derived from bacteria. In one embodiment, the homolog of a polypeptide indicated in Table II, column 3, lines 75 to 80 is a homolog having the same or a similar acitivity, in particular an increase of activity confers an increase in the content of the fine chemical in an organisms or part thereof, and being derived from Fungi. In one embodiment, the homolog of a polypeptide indicated in Table II, column 3, lines lines 72 to 74, 81 and 460 to 462 is a homolog having the same or a similar acitivity, in particular an increase of activity confers an increase in the content of the fine chemical in the organsims or part thereof and being derived from Proteobacteria. In one embodiment, the homolog of a polypeptide indicated in Table II, column 3, lines 75 to 80 is a homolog having the same or a similar acitivity, in particular an increase of activity confers an increase in the content of the fine chemical in the organsims or a part thereof and being derived from Ascomycota. In one embodiment, the homolog of a polypeptide indicated in Table II, column 3, lines 72 to 74, 81 and 460 to 462 is a homolog having the same or a similar acitivity, in particular an increase of activity confers an increase in the content of the fine chemical in the organisms or part thereof, and being derived from Gammaproteobacteria. In one embodiment, the homolog of a polypeptide polypeptide indicated in Table II, column 3, lines 75 to 80 is a homolog having the same or a similar acitivity, in particular an increase of activity confers an increase in the content of the fine chemical in the organisms or part thereof, and being derived from Saccharomycotina. In one embodiment, the homolog of a polypeptide indicated in Table II, column 3, lines 72 to 74, 81 and 460 to 462 is a homolog having the samie or a similar acitivity, in particular an increase of activity confers an increase in the content of the fine chemical in the organisms or part thereof, and being derived from Enterobacteriales. In one embodiment, the homolog of a polypeptide indicated in Table II, column 3, lines 75 to 80 is a homolog having the samie or a similar acitivity, in particular an increase of activity confers an increase in the content of the fine chemical in the organisms or a part thereof, and being derived from Saccharomycetes. In one embodiment, the homolog of the a polypeptide indicated in Table II, column 3, lines lines 72 to 74, 81 and 460 to 462 is a homolog having the samie or a similar acitivity, in particular an increase of activity confers an increase in the content of the fine chemical in the organisms or part thereof, and being derived from Enterobacteriaceae. In one embodiment, the homolog of a polypeptide indicated in Table II, column 3, lines 75 to 80 is a homolog having the same or a similar acitivity, in particular an increase of activity confers an increase in the content of the fine chemical in the organisms, and being derived from Saccharomycetales. In one embodiment, the homolog of a polypeptide indicated in Table II, column 3, lines 72 to 74, 81 and 460 to 462 is a homolog having the same or a similar acitivity, in particular an increase of activity confers an increase in the content of the fine chemical in the organisms or a part thereof, and being derived from Escherichia. In one embodiment, the homolog of a polypeptide indicated in Table II, column 3, lines 75 to 80 is a homolog having the same or a similar acitivity, in particular an increase of activity confers an increase in the content of the fine chemical in the organisms or a part thereof, and being derived from Saccharomycetaceae. In one embodiment, the homolog of a polypeptide indicated in Table II, column 3, line 75 to 80 is a homolog having the same or a similar acitivity, in particular an increase of activity confers an increase in the content of the fine chemical in the organisms or a part thereof, and being derived from Saccharomycetes.

**[0023.1.0.7] and [0024.0.0.7]** for the disclosure of the paragraphs [0023.1.0.7] and [0024.0.0.7] see [0023.1.0.0] and [0024.0.0.0] above.

**[0025.0.7.7]** In accordance with the invention, a protein or polypeptide has the "activity of a protein as indicated in Table II, column 3, lines 72 to 81 and 460 to 462" if its *de novo* activity, or its increased expression directly or indirectly leads to an increased stearic acid and/or tryglycerides, lipids, oils and/or fats containing stearic acid level in the organism or a part thereof, preferably in a cell of said organism and the protein has the above mentioned activities of a protein as indicated in Table II, column 3, lines 72 to 81 and 460 to 462. Throughout the specification the activity or preferably the biological activity of such a protein or polypeptide or an nucleic acid molecule or sequence encoding such protein or polypeptide is identical or similar if it still has the biological or enzymatic activity of a protein as indicated in Table II, column 3, lines 72 to 81 and 460 to 462, or which has at least 10% of the original enzymatic activity, preferably 20%, particularly preferably 30%, most particularly preferably 40% in comparison to a protein of Saccharomyces cerevisiae as indicated in Table II, column 3, lines 75 to 80 and/or a protein of E. coli K12 as indicated in Table II, column 3, lines 72 to 74, 81 and 460 to 462.

**[0025.1.0.7] and [0025.2.0.7]** for the disclosure of the paragraphs [0025.1.0.7] and [0025.2.0.7] see paragraphs [0025.1.0.0] and [0025.2.0.0] above.

**[0026.0.0.7] to [0033.0.0.7]** for the disclosure of the paragraphs [0026.0.0.7] to [0033.0.0.7] see paragraphs [0026.0.0.0] to [0033.0.0.0] above.

**[0034.0.7.7]** Preferably, the reference, control or wild type differs form the subject of the present invention only in the cellular activity of the polypeptide of the invention or the polypeptide used in the method of the invention, e.g. as result of an increase in the level of the nucleic acid molecule of the present invention or an increase of the specific activity of the polypeptide of the invention or the polypeptide used in the method of the invention. E.g., it differs by or in the expression level or activity of an protein having the activity of a protein as indicated in Table II, column 3, lines 72 to 81 and 460 to 462 or being encoded by a nucleic acid molecule indicated in Table I, column 5, lines 72 to 81 and 460 to 462 or its homologs, e.g. as indicated in Table I, column 7, lines 72 to 81 and 460 to 462, its biochemical or genetical causes and therefore shows the increased amount of the fine chemical.

**[0035.0.0.7] to [0038.0.0.7] and [0039.0.5.7]** for the disclosure of the paragraphs [0035.0.0.7] to [0038.0.0.7] and [0039.0.5.7] see paragraphs [0035.0.0.0] to [0039.0.0.0] above.

**[0040.0.0.7] to [0044.0.0.7]** for the disclosure of the paragraphs [0040.0.0.7] to [0044.0.0.7] see paragraphs [0035.0.0.0] and [0044.0.0.0] above.

**[0045.0.7.7]** In one embodiment, in case the activity of the Escherichia coli K12 protein b3256 or its homologs e.g. a acetyl CoA carboxylase protein e.g. as indicated in Table II, columns 5 or 7, line 72, is increased, preferably, in one embodiment the increase of the fine chemical, preferably of stearic acid between 17% and 25% or more is conferred.
In one embodiment, in case the activity of the Escherichia coli K12 protein b2699 or its homologs e.g. a DNA-dependent ATPase or DNA- and ATP-dependent coprotease protein e.g. as indicated in Table II, columns 5 or 7, line 73, is increased, preferably, in one embodiment the increase of the fine chemical, preferably of stearic acid between 22% and 83% or more is conferred.
In case the activity of the Escherichia coli K12 protein b2095 or its homologs e.g. a putative tagatose-6-phosphate kinase 1 e.g. as indicated in Table II, columns 5 or 7, line 74, is increased, preferably, in one embodiment the increase of the fine chemical, preferably of stearic acid between 17% and 26% or more is conferred.
In one embodiment, in case the activity of the Escherichia coli K12 protein b1093 or its homologs e.g. a 3-oxoacyl-[acyl-carrier-protein] reductase e.g. as indicated in Table II, columns 5 or 7, line 81, is increased, preferably, in one embodiment the increase of the fine chemical, preferably of stearic acid between 16% and 31 % or more is conferred.
In one embodiment, in case the activity of the Saccharomyces cerevisiae protein YOR024W or its homologs, e.g. a "uncharacterized protein YOR024W", which seams to be "probably a membrane protein" e.g. as indicated in Table II, columns 5 or 7, line 75 is increased, preferably, in one embodiment an increase of the fine chemical, preferably of stearic acid between 16% and 40% or more is conferred.
In one embodiment, in case the activity of the Saccharomyces cerevisiae protein YBR089C-A or its homologs, e.g. a "uncharacterized protein YBR089C-A", which seams to have "homology to mammalian high mobility group proteins 1 and 2" e.g. as indicated in Table II, columns 5 or 7, line 76 is increased, preferably, in one embodiment an increase of the fine chemical, preferably of stearic acid between 36% and 134% or more is conferred.
In one embodiment, in case the activity of the Saccharomyces cerevisiae protein YFR042W or its homologs, e.g. a "uncharacterized protein YFR042W", which seams to be a "protein, which is required for cell viability" e.g. as indicated in Table II, columns 5 or 7, line 77 is increased, preferably, in one embodiment an increase of the fine chemical, preferably of stearic acid between 19% and 26% or more is conferred.
In one embodiment, in case the activity of the Saccaromyces cerevisiae protein YIL150C or its homologs e.g. a "uncharacterized protein YIL150C", which is probably a "protein required for S-phase (DNA synthesis) initiation or completion" e.g. as indicated in Table II, columns 5 or 7, line 78 is increased, preferably, in one embodment the increase of the fine chemical, preferably of stearic acid between 34% and 220% or more is conferred.
In one embodiment, in case the activity of the Saccharomyces cerevisiae protein YDR513W or its homologs, e.g. a "glutaredoxin (thioltransferase, glutathione reductase)" e.g. as indicated in Table II, columns 5 or 7, line 79 is increased, preferably, in one embodiment an increase of the fine chemical, preferably of stearic acid between 16% and 51% or more is conferred.
In one embodiment, in case the activity of the Saccharomyces cerevisiae protein YLR010C or its homologs, e.g. a "uncharacterized protein YLR010C", which is probably a "protein involved in telomeric pathways in association with Stn1" e.g. as indicated in Table II, columns 5 or 7, line 80 is increased, preferably, in one embodiment an increase of the fine chemical, preferably of stearic acid between 16% and 76% or more is conferred.
In one embodiment, in case the activity of the Escherichia coli K12 protein b0161 or its homologs e.g. a periplasmic serine protease e.g. as indicated in Table II, columns 5 or 7, line 460, is increased, preferably, in one embodiment the increase of the fine chemical, preferably of stearic acid between 16% and 129% or more is conferred.
In one embodiment, in case the activity of the Escherichia coli K12 protein b1896 or its homologs e.g. a trehalose-6-phosphate synthase e.g. as indicated in Table II, columns 5 or 7, line 461, is increased, preferably, in one embodiment the increase of the fine chemical, preferably of stearic acid between 23% and 30% or more is conferred.
In one embodiment, in case the activity of the Escherichia coli K12 protein b3457 or its homologs e.g. a high-affinity branched-chain amino acid transport protein (ABC superfamily) e.g. as indicated in Table II, columns 5 or 7, line 462, is increased, preferably, in one embodiment the increase of the fine chemical, preferably of stearic acid between 18% and 34% or more is conferred.

**[0046.0.7.7]** In case the activity of the Escherichia coli K12 protein b3256 or its homologs e.g. a acetyl CoA carboxylase protein, is increased, preferably an increase of the fine chemical and of tryglycerides, lipids, oils and/or fats containing stearic acid is conferred.
In case the activity of the Escherichia coli K12 protein b2699 or its homologs e.g. a DNA-dependent ATPase or DNA- and ATP-dependent coprotease protein, is increased, preferably an increase of the fine chemical and of tryglycerides, lipids, oils and/or fats containing stearic acid is conferred.
In case the activity of the Escherichia coli K12 protein b2095 or its homologs e.g. a putative tagatose-6-phosphate kinase 1, is increased, preferably an increase of the fine chemical and of tryglycerides, lipids, oils and/or fats containing stearic acid is conferred.
In case the activity of the Escherichia coli K12 protein b1093 or its homologs e.g. a 3-oxoacyl-[acyl-carrier-protein] reductase, is increased, preferably, in one embodiment the increase of the fine chemical, preferably an increase of the fine chemical and of tryglycerides, lipids, oils and/or fats containing stearic acid is conferred.
In case the activity of the Saccharomyces cerevisiae protein YOR024W or its homologs, e.g. a "uncharacterized protein YOR024W", which seams to be "probably a membrane protein" is increased, preferably, in one embodiment an increase of the fine chemical, preferably an increase of the fine chemical and of tryglycerides, lipids, oils and/or fats containing stearic acid is conferred.
In case the activity of the Saccharomyces cerevisiae protein YBR089C-A or its homologs, e.g. a "uncharacterized protein YBR089C-A", which seams to have "homology to mammalian high mobility group proteins 1 and 2" is increased, preferably, an increase of the fine chemical and of tryglycerides, lipids, oils and/or fats containing stearic acid is conferred.
In case the activity of the Saccharomyces cerevisiae protein YFR042W or its homologs, e.g. a "uncharacterized protein YFR042W", which seams to be a "protein, which is required for cell viability" is increased, preferably, in one embodiment an increase of the fine chemical, preferably an increase of the fine chemical and of tryglycerides, lipids, oils and/or fats containing stearic acid is conferred.
In case the activity of the Saccaromyces cerevisiae protein YIL150C or its homologs e.g. a "uncharacterized protein YIL150C", which is probably a "protein required for S-phase (DNA synthesis) initiation or completion" is increased, preferably an increase of the fine chemical and of tryglycerides, lipids, oils and/or fats containing stearic acid is conferred.
In case the activity of the Saccharomyces cerevisiae protein YDR513W or its homologs, e.g. a "glutaredoxin (thioltransferase, glutathione reductase)" is increased, preferably, in one embodiment an increase of the fine chemical, preferably an increase of the fine chemical and of tryglycerides, lipids, oils and/or fats containing stearic acid is conferred.
In case the activity of the Saccharomyces cerevisiae protein YLR010C or its homologs, e.g. a "uncharacterized protein YLR010C", which is probably a "protein involved in telomeric pathways in association with Stn1" is increased, preferably, in one embodiment an increase of the fine chemical, preferably an increase of the fine chemical and of tryglycerides, lipids, oils and/or fats containing stearic acid is conferred.
In case the activity of the Escherichia coli K12 protein b0161 or its homologs e.g. a periplasmic serine protease is increased, preferably, in one embodiment an increase of the fine chemical, preferably an increase of the fine chemical and of tryglycerides, lipids, oils and/or fats containing stearic acid is conferred.
In one embodiment, in case the activity of the Escherichia coli K12 protein b1896 or its homologs e.g. a trehalose-6-phosphate synthase is increased, preferably, in one embodiment an increase of the fine chemical, preferably an increase of the fine chemical and of tryglycerides, lipids, oils and/or fats containing stearic acid is conferred.
In one embodiment, in case the activity of the Escherichia coli K12 protein b3457 or its homologs e.g. a high-affinity branched-chain amino acid transport protein (ABC superfamily) is increased, preferably, in one embodiment an increase of the fine chemical, preferably an increase of the fine chemical and of tryglycerides, lipids, oils and/or fats containing stearic acid is conferred.

**[0047.0.0.7] to [0048.0.0.7]** for the disclosure of the paragraphs [0047.0.0.7] and [0048.0.0.7] see paragraphs [0047.0.0.0] and [0048.0.0.0] above.

**[0049.0.7.7]** A protein having an activity conferring an increase in the amount or level of the respective fine chemical preferably has the structure of the polypeptide described herein, in particular a polypeptide comprising a consensus sequence as indicated in Table IV, column 7, lines 72 to 81 and 460 to 462 or of the polypeptide as shown in the amino acid sequences as disclosed in table II, columns 5 and 7, lines 72 to 81 and 460 to 462 or the functional homologues thereof as described herein, or is encoded by the nucleic acid molecule characterized herein or the nucleic acid molecule according to the invention, for example by the nucleic acid molecule as shown in table I, columns 5 and 7, lines 72 to 81 and 460 to 462 or its herein described functional homologues and has the herein mentioned activity.

**[0050.0.7.7]** For the purposes of the present invention, the term "stearic acid" also encompasses the corresponding salts, such as, for example, the potassium or sodium salts of stearic acid or the salts of stearic acid with amines such as diethylamine.

**[0051.0.5.7] and [0052.0.0.7]** for the disclosure of the paragraphs [0051.0.5.7] and [0052.0.0.7] see paragraphs [0051.0.0.0] and [0052.0.0.0] above.

**[0053.0.7.7]** In one embodiment, the process of the present invention comprises one or more of the following steps
a) stabilizing a protein conferring the increased expression of a protein encoded by the nucleic acid molecule of the invention or of the polypeptid of the invention or the nucleic acid molecule or the polypeptide used in the method of the invention, e.g. of a polypeptide having an activity of a protein as indicated in Table II, column 3, lines 72 to 81 and 460 to 462 or its homolog's activity, e.g. as indicated in Table II, column 7, lines 72 to 81 and 460 to 462, having herein-mentioned stearic acid increasing activity;
b) stabilizing a mRNA conferring the increased expression of a protein encoded by the nucleic acid molecule of the invention or the nucleic acid molecule used in the method of the invention, e.g. of a polypeptide having an activity of a protein as indicated in Table II, column 3, lines 72 to 81 and 460 to 462 or its homologs activity, e.g. as indicated in Table II, column 7, lines 72 to 81 and 460 to 462 or of a mRNA encoding the polypeptide of the present invention having herein-mentioned stearic acid increasing activity;
c) increasing the specific activity of a protein conferring the increased expression of a protein encoded by the nucleic acid molecule of the invention or of the polypeptide of the present invention or the nucleic acid molecule or the polypeptide used in the method of the invention, having herein-mentioned stearic acid increasing activity, e.g. of a polypeptide having an activity of a protein as indicated in Table II, column 3, lines 72 to 81 and 460 to 462 or its homologs activity, e.g. as indicated in Table II, column 7, lines 72 to 81 and 460 to 462, or decreasing the inhibitiory regulation of the polypeptide of the invention or of the polypeptide used in the method of the invention;
d) generating or increasing the expression of an endogenous or artificial transcription factor mediating the expression of a protein conferring the increased expression of a protein encoded by the nucleic acid molecule of the invention or the nucleic acid molecule used in the method of the invention or of the polypeptide of the invention or the polypeptide used in the method of the invention having herein-mentioned stearic acid increasing activity, e.g. of a polypeptide having an activity of a protein as indicated in Table II, column 3, lines 72 to 81 and 460 to 462 or its homolog's activity, e.g. as indicated in Table II, column 7, lines 72 to 81 and 460 to 462;
e) stimulating activity of a protein conferring the increased expression of a protein encoded by the nucleic acid molecule of the present invention or a polypeptide of the present invention having herein-mentioned stearic acid increasing activity, e.g. of a polypeptide having an activity of a protein as indicated in Table II, column 3, lines 72 to 81 and 460 to 462 or its homolog's activity, e.g. as indicated in Table II, column 7, lines 72 to 81 and 460 to 462, by adding one or more exogenous inducing factors to the organismus or parts thereof;
f) expressing a transgenic gene encoding a protein conferring the increased expression of a polypeptide encoded by the nucleic acid molecule of the present invention or a polypeptide of the present invention, having herein-mentioned stearic acid increasing activity, e.g. of a polypeptide having an activity of a protein as indicated in Table II, column 3, lines 72 to 81 and 460 to 462 or its homolog's activity, e.g. as indicated in Table II, column 7, lines 72 to 81 and 460 to 462;
g) increasing the copy number of a gene conferring the increased expression of a nucleic acid molecule encoding a polypeptide encoded by the nucleic acid molecule of the invention or the nucleic acid molecule used in the method of the invention or the polypeptide of the invention or the polypeptide used in the method of the invention having herein-mentioned stearic acid increasing activity, e.g. of a polypeptide having an activity of a protein as indicated in Table II, column 3, lines 72 to 81 and 460 to 462 or its homolog's activity, e.g. as indicated in Table II, column 7, lines 72 to 81 and 460 to 462;
h) increasing the expression of the endogenous gene encoding the polypeptide of the invention or the polypeptide used in the method of the invention, e.g. a polypeptide having an activity of a protein as indicated in Table II, column 3, lines 72 to 81 and 460 to 462 or its homolog's activity, e.g. as indicated in Table II, column 7, lines 72 to 81 and 460 to 462, by adding positive expression or removing negative expression elements, e.g. homologous recombination can be used to either introduce positive regulatory elements like for plants the 35S enhancer into the promoter or to remove repressor elements form regulatory regions. Further gene conversion methods can be used to disrupt repressor elements or to enhance to acitivty of positive elements. Positive elements can be randomly introduced in plants by T-DNA or transposon mutagenesis and lines can be identified in which the positive elements have be integrated near to a gene of the invention, the expression of which is thereby enhanced;
i) Modulating growth conditions of an organism in such a manner, that the expression or activity of the gene encoding the protein of the invention or the protein itself is enhanced for example microorganisms or plants can be grown for example under a higher temperature regime leading to an enhanced expression of heat shock proteins, which can lead an enhanced fine chemical production.
j) selecting of organisms with expecially high activity of the proteins of the invention from natural or from mutagenized resources and breeding them into the target organisms, eg the elite crops.

**[0054.0.7.7]** Preferably, said mRNA is the nucleic acid molecule of the present invention and/or the protein conferring the increased expression of a protein encoded by the nucleic acid molecule of the present invention or the polypeptide having the herein mentioned activity is the polypeptide of the present invention, e.g. conferring the increase of stearic acid after increasing the expression or activity of the encoded polypeptide or having the activity of a polypeptide having an activity of a protein as indicated in Table II, column 3, lines 72 to 81 and 460 to 462 or its homolog's activity, e.g. as indicated in Table II, columns 5 or 7, lines 72 to 81 and 460 to 462.

**[0055.0.0.7] to [0067.0.0.7]** for the disclosure of the paragraphs [0055.0.0.7] to [0067.0.0.7] see paragraphs [0055.0.0.0] to [0067.0.0.0] above.

**[0068.0.5.7] and [0069.0.5.7]** for the disclosure of the paragraphs [0068.0.5.7] and [0069.0.5.7] see paragraphs [0068.0.0.0] and [0069.0.0.0] above.

**[0070.0.6.7] and [0071.0.5.7]** for the disclosure of the paragraphs [0070.0.6.7] and **[0071.0.5.7]** see paragraphs [0070.0.5.5] and [0071.0.0.0] above.

**[0072.0.7.7]** By influencing the metabolism thus, it is possible to produce, in the process according to the invention, further advantageous compounds. Examples of such compounds are, in addition to stearic acid, triglycerides, lipids, oils and/or fats containing stearic acid compounds such as palmitate, palmitoleate, stearate, oleate, α-linolenic acid and/or linoleic acid.

**[0073.0.7.7]** Accordingly, in one embodiment, the process according to the invention relates to a process, which comprises:
a) providing a non-human organism, preferably a microorganism, a non-human animal, a plant or animal cell, a plant or animal tissue or a plant;
b) increasing the activity of a protein having the activity of a polypeptide of the invention or the polypeptide used in the method of the invention or a homolog thereof, e.g. as shown in Table II, columns 5 or 7, lines 72 to 81 and 460 to 462 or of a polypeptide being encoded by the nucleic acid molecule of the present invention and described below, i.e. conferring an increase of the respective fine chemical in the organism, preferably a microorganism, the a non-human animal, a plant or animal cell, a plant or animal tissue or the plant,
c) growing the organism, preferably a microorganism, a non-human animal, a plant or animal cell, a plant or animal tissue or the plant under conditions which permit the production of the fine chemical in the organism, preferably a microorganism, a plant cell, a plant tissue or the plant; and
d) if desired, revovering, optionally isolating, the free and/or bound the respective fine chemical and, optionally further free and/or bound fatty acids synthetized by the organism, the microorganism, the non-human animal, the plant or animal cell, the plant or animal tissue or the plant.

**[0074.0.5.7]** for the disclosure of this paragraph see [0074.0.5.5] above.

**[0075.0.0.7] to [0084.0.0.7]** for the disclosure of the paragraphs [0075.0.0.7] to [0084.0.0.7] see paragraphs [0075.0.0.0] to [0084.0.0.0] above.

**[0085.0.7.7]** With regard to the nucleic acid sequence as depicted a nucleic acid construct which contains a nucleic acid sequence mentioned herein or an organism (= transgenic organism) which is transformed with said nucleic acid sequence or said nucleic acid construct, "transgene" means all those constructs which have been brought about by genetic manipulation methods,preferably in which either
a) the nucleic acid sequence as shown in table I, lines 72 to 81 and 460 to 462, columns 5 and 7 or a derivative thereof, or
b) a genetic regulatory element, for example a promoter, which is functionally linked to the nucleic acid sequence as shown table I, lines 72 to 81 and 460 to 462, columns 5 and 7 or a derivative thereof, or
c) (a) and (b)
is/are not present in its/their natural genetic environment or has/have been modified by means of genetic manipulation methods, it being possible for the modification to be, by way of example, a substitution, addition, deletion, inversion or insertion of one or more nucleotide. "Natural genetic environment" means the natural chromosomal locus in the organism of origin or the presence in a genomic library. In the case of a genomic library, the natural, genetic environment of the nucleic acid sequence is preferably at least partially still preserved. The environment flanks the nucleic acid sequence at least on one side and has a sequence length of at least 50 bp, preferably at least 500 bp, particularly preferably at least 1000 bp, very particularly preferably at least 5000 bp.

**[0086.0.0.7] and [0087.0.0.7]** for the disclosure of the paragraphs [0086.0.0.7] and [0087.0.0.7] see paragraphs [0086.0.0.0] and [0087.0.0.0] above.

**[0088.0.7.7]** In an advantageous embodiment of the invention, the organism takes the form of a plant whose fatty acid content is modified advantageously owing to the nucleic acid molecule of the present invention expressed. This is important for plant breeders since, for example, the nutritional value of plants for poultry is dependent on the abovementioned essential fatty acids and the general amount of fatty acids as energy source in feed. After the activity of a protein as shown in Table II, columns 5 or 7, lines 72 to 81 and 460 to 462 has been increased or generated, or after the expression of nucleic acid molecule or polypeptide according to the invention has been generated or increased, the transgenic plant generated thus is grown on or in a nutrient medium or else in the soil and subsequently harvested.

**[0088.1.0.7], [0089.0.0.7], [0090.0.0.7] and [0091.0.5.7]** for the disclosure of the paragraphs [0088.1.0.7], [0089.0.0.7], [0090.0.0.7] and [0091.0.5.7] see paragraphs [0088.1.0.0], [0089.0.0.0], [0090.0.0.0] and [0091.0.0.0] above.

**[0092.0.0.7] to [0094.0.0.7]** for the disclosure of the paragraphs [0092.0.0.7] to [0094.0.0.7] see paragraphs [0092.0.0.0] to [0094.0.0.0] above.

**[0095.0.5.7], [0096.0.5.7] and [0097.0.5.7]** for the disclosure of the paragraphs [0095.0.5.7], [0096.0.5.7] and [0097.0.5.7] see paragraphs [0095.0.5.5], [0096.0.5.5] and [0097.0.0.0] above.

**[0098.0.7.7]** In a preferred embodiment, the fine chemical (stearic acid) is produced in accordance with the invention and, if desired, is isolated. The production of further fatty acids such as palmitic acid, stearic acid, palmitoleic acid, oleic acid and/or linoleic acid mixtures thereof or mixtures of other fatty acids by the process according to the invention is advantageous.

**[0099.0.5.7] and [0100.0.5.7]** for the disclosure of the paragraphs [0099.0.5.7] and [0100.0.5.7] see paragraphs [0099.0.5.5] and [0100.0.5.5] above.

**[0101.0.5.7] and [0102.0.5.7]** for the disclosure of the paragraphs [0101.0.5.7] and [0102.0.5.7] see [0101.0.0.0] and [0102.0.5.5] above.

**[0103.0.7.7]** In a preferred embodiment, the present invention relates to a process for the production of the fine chemical comprising or generating in an organism or a part thereof the expression of at least one nucleic acid molecule comprising a nucleic acid molecule selected from the group consisting of:
a) nucleic acid molecule encoding, preferably at least the mature form, of a polypeptide having a sequence as shown in Table II, columns 5 or 7, lines 72 to 81 and 460 to 462 in or a fragment thereof, which confers an increase in the amount of the respective fine chemical in an organism or a part thereof;
b) nucleic acid molecule comprising, preferably at least the mature form, of the nucleic acid molecule having a sequence as shown in Table I, columns 5 or 7, lines 72 to 81 and 460 to 462,
c) nucleic acid molecule whose sequence can be deduced from a polypeptide sequence encoded by a nucleic acid molecule of (a) or (b) as result of the degeneracy of the genetic code and conferring an increase in the amount of the fine chemical in an organism or a part thereof;
d) nucleic acid molecule encoding a polypeptide which has at least 50% identity with the amino acid sequence of the polypeptide encoded by the nucleic acid molecule of (a) to (c) and conferring an increase in the amount of the fine chemical in an organism or a part thereof;
e) nucleic acid molecule which hybidizes with a nucleic acid molecule of (a) to (c) under under stringent hybridisation conditions and conferring an increase in the amount of the fine chemical in an organism or a part thereof;
f) nucleic acid molecule encoding a polypeptide, the polypeptide being derived by substituting, deleting and/or adding one or more amino acids of the amino acid sequence of the polypeptide encoded by the nucleic acid molecules (a) to (d), preferably to (a) to (c) and conferring an increase in the amount of the respective fine chemical in an organism or a part thereof;
g) nucleic acid molecule encoding a fragment or an epitope of a polypeptide which is encoded by one of the nucleic acid molecules of (a) to (e), preferably to (a) to (c) and and conferring an increase in the amount of the respective fine chemical in an organism or a part thereof;
h) nucleic acid molecule comprising a nucleic acid molecule which is obtained by amplifying nucleic acid molecules from a cDNA library or a genomic library using the primer pairs having a sequence as shown in Table III, column 7, lines 72 to 81 and 460 to 462 and conferring an increase in the amount of the respective fine chemical in an organism or a part thereof;
i) nucleic acid molecule encoding a polypeptide which is isolated, e.g. from an expression library, with the aid of monoclonal antibodies against a polypeptide encoded by one of the nucleic acid molecules of (a) to (h), preferably to (a) to (c), and and conferring an increase in the amount of the respective fine chemical in an organism or a part thereof;
j) nucleic acid molecule which encodes a polypeptide comprising the consensus sequence having a sequence as shown in Table IV, column 7, lines 72 to 81 and 460 to 462 and conferring an increase in the amount of the respective fine chemical in an organism or a part thereof;
k) nucleic acid molecule comprising one or more of the nucleic acid molecule encoding the amino acid sequence of a polypeptide encoding a domain of a polypeptide as shown in Table II, columns 5 or 7, lines 72 to 81 and 460 to 462 and conferring an increase in the amount of the respective fine chemical in an organism or a part thereof; and
l) nucleic acid molecule which is obtainable by screening a suitable library under stringent conditions with a probe comprising one of the sequences of the nucleic acid molecule of (a) to (k), preferably to (a) to (c), or with a fragment of at least 15 nt, preferably 20 nt, 30 nt, 50 nt, 100 nt, 200 nt or 500 nt of the nucleic acid molecule characterized in (a) to (k), preferably to (a) to (c), and conferring an increase in the amount of the fine chemical in an organism or a part thereof;
or which comprises a sequence which is complementary thereto.

**[0103.1.0.7] and [0103.2.0.7]** for the disclosure of the paragraphs [0103.1.0.7] and [0103.2.0.7] see paragraphs [0103.1.0.0] and [0103.2.0.0] above.

**[0104.0.7.7]** In one embodiment, the nucleic acid molecule used in the process distinguishes over the sequence shown in Table I, columns 5 or 7, lines 72 to 81 and 460 to 462, preferably over the sequences as shown in Table IA, columns 5 or 7, lines 72 to 81 and 460 to 462 by one or more nucleotides or does not consist of the sequence shown in Table I, columns 5 or 7, lines 72 to 81 and 460 to 462, preferably over the sequences as shown in Table IA, columns 5 or 7, lines 72 to 81 and 460 to 462. In one embodiment, the nucleic acid molecule of the present invention is less than 100%, 99,999%, 99,99%, 99,9% or 99% identical to the sequence shown in Table I, columns 5 or 7, lines 72 to 81 and 460 to 462, preferably over the sequences as shown in Table IA, columns 5 or 7, lines 72 to 81 and 460 to 462. In another embodiment, the nucleic acid molecule does not encode a polypeptide of the sequence shown in Table II, columns 5 or 7, lines 72 to 81 and 460 to 462, preferably of the sequences as shown in Table IA, columns 5 or 7, lines 72 to 81 and 460 to 462.

**[0105.0.0.7] to [0107.0.0.7]** for the disclosure of the paragraphs [0105.0.0.7] to [0107.0.0.7] see paragraphs [0105.0.0.0] and [0107.0.0.0] above.

**[0108.0.7.7]** Nucleic acid molecules with the sequence shown in Table I, columns 5 or 7, lines 72 to 81 and 460 to 462, nucleic acid molecules which are derived from the amino acid sequences shown in Table II, columns 5 or 7, lines 72 to 81 and 460 to 462 or from polypeptides comprising the consensus sequence shown in Table IV, column 7, lines 72 to 81 and 460 to 462, or their derivatives or homologues encoding polypeptides with the enzymatic or biological activity of a protein as shown in Table II, columns 5 or 7, lines 72 to 81 and 460 to 462 or e.g. conferring a linoleic acid increase after increasing its expression or activity are advantageously increased in the process according to the invention.

**[0109.0.5.7]** for the disclosure of this paragraph see [0109.0.0.0] above.

**[0110.0.7.7]** Nucleic acid molecules, which are advantageous for the process according to the invention and which encode polypeptides with an activity of a polypeptide used in the method of the invention or used in the process of the invention, e.g. of a protein as shown in Table II, columns 5 or 7, lines 72 to 81 and 460 to 462 or being encoded by a nucleic acid molecule indicated in Table I, columns 5 or 7, lines 72 to 81 and 460 to 462 or of its homologs, e.g. as indicated in Table II, columns 5 or 7, lines 72 to 81 and 460 to 462 can be determined from generally accessible databases.

**[0111.0.0.7]** for the disclosure of this paragraph see [0111.0.0.0] above.

**[0112.0.7.7]** The nucleic acid molecules used in the process according to the invention take the form of isolated nucleic acid sequences, which encode polypeptides with an activity of a polypeptide as indicated in Table II, column3, lines lines 72 to 81 and 460 to 462 or having the sequence of a polypeptide as indicated in Table II, columns 5 and 7, lines 72 to 81 and 460 to 462 and conferring an increase of the respective fine chemical.

**[0113.0.0.7] to [0120.0.0.7]** for the disclosure of the paragraphs [0113.0.0.7] to [0120.0.0.7] see paragraphs [0113.0.0.0] and [0120.0.0.0] above.

**[0121.0.7.7]** However, it is also possible to use artificial sequences, which differ in one or more bases from the nucleic acid sequences found in organisms, or in one or more amino acid molecules from polypeptide sequences found in organisms, in particular from the polypeptide sequences shown in Table II, columns 5 or 7, lines 72 to 81 and 460 to 462 or the functional homologues thereof as described herein, preferably conferring above-mentioned activity, i.e. conferring an increase of the respective fine chemical after increasing its activity.

**[0122.0.0.7] to [0127.0.0.7]** for the disclosure of the paragraphs [0122.0.0.7] to [0127.0.0.7] see paragraphs [0122.0.0.0] and [0127.0.0.0] above.

**[0128.0.7.7]** Synthetic oligonucleotide primers for the amplification, e.g. as shown in Table III, column 7, lines 72 to 81 and 460 to 462, by means of polymerase chain reaction can be generated on the basis of a sequence shown herein, for example the sequence shown in Table I, columns 5 or 7, lines 72 to 81 and 460 to 462 or the sequences as shown in Table II, columns 5 or 7, lines 72 to 81 and 460 to 462.

**[0129.0.7.7]** Moreover, it is possible to identify conserved regions from various organisms by carrying out protein sequence alignments with the polypeptide used in the process of the invention, in particular with sequences of the polypeptide of the invention, from which conserved regions, and in turn, degenerate primers can be derived. Conserved region for the polypeptide of the invention are indicated in the alignments shown in the figures. Conserved regions are those, which show a very little variation in the amino acid in one particular position of several homologs from different origin. The consenus sequence shown in Table IV, column 7, lines 72 to 81 and 460 to 462 is derived from said aligments.

**[0130.0.7.7]** for the disclosure of this paragraph see [0130.0.0.0].

**[0131.0.0.7] to [0138.0.0.7]** for the disclosure of the paragraphs [0131.0.0.7] to [0138.0.0.7] see paragraphs [0131.0.0.0] to [0138.0.0.0] above.

**[0139.0.7.7]** Polypeptides having above-mentioned activity, i.e. conferring the respective fine chemical increase, derived from other organisms, can be encoded by other DNA sequences which hybridize to the sequences shown in Table I, columns 5 or 7, lines 72 to 81 and 460 to 462, preferably of Table I B, columns 5 or 7, lines 72 to 81 and 460 to 462 under relaxed hybridization conditions and which code on expression for peptides having the stearic acid increasing activity.

**[0140.0.0.7] to [0146.0.0.7]** for the disclosure of the paragraphs [0140.0.0.7] to [0146.0.0.7] see paragraphs [0140.0.0.0] and [0146.0.0.0] above.

**[0147.0.7.7]** Further, the nucleic acid molecule of the invention comprises a nucleic acid molecule, which is a complement of one of the nucleotide sequences of above mentioned nucleic acid molecules or a portion thereof. A nucleic acid molecule which is complementary to one of the nucleotide sequences indicated in Table I, columns 5 or 7, lines 72 to 81 and 460 to 462, preferably in Table I B, columns 5 or 7, lines 72 to 81 and 460 to 462 is one which is sufficiently complementary to one of said nucleotide sequences such that it can hybridize to one of said nucleotide sequences thereby forming a stable duplex. Preferably, the hybridisation is performed under stringent hybrization conditions. However, a complement of one of the herein disclosed sequences is preferably a sequence complement thereto according to the base pairing of nucleic acid molecules well known to the skilled person. For example, the bases A and G undergo base pairing with the bases T and U or C, resp. and visa versa. Modifications of the bases can influence the base-pairing partner.

**[0148.0.7.7]** The nucleic acid molecule of the invention comprises a nucleotide sequence which is at least about 30%, 35%, 40% or 45%, preferably at least about 50%, 55%, 60% or 65%, more preferably at least about 70%, 80%, or 90%, and even more preferably at least about 95%, 97%, 98%, 99% or more homologous to a nucleotide sequence indicated in Table I, columns 5 or 7, lines 72 to 81 and 460 to 462, preferably of Table I B, columns 5 or 7, lines 72 to 81 and 460 to 462, or a functional portion thereof and preferably has above mentioned activity, in particular has the-fine-chemical-increasing activity after increasing its acitivity or an activity of a product of a gene encoding said sequence or its homologs.

**[0149.0.7.7]** The nucleic acid molecule of the invention or the nucleic acid molecule used in the method of the invention comprises a nucleotide sequence which hybridizes, preferably hybridizes under stringent conditions as defined herein, to one of the nucleotide sequences indicated in Table I, columns 5 or 7, lines 72 to 81 and 460 to 462, preferably of Table I B, columns 5 or 7, lines 72 to 81 and 460 to 462 or a portion thereof and encodes a protein having above-mentioned activity as indicated in Table II, columns 5 or 7, lines 72 to 81 and 460 to 462, preferably of Table II B, columns 5 or 7, lines 72 to 81 and 460 to 462, e.g. conferring an increase of the respective fine chemical.

**[00149.1.0.7]** Optionally, the nucleotide sequence, which hybridises to one of the nucleotide sequences indicated in Table I, columns 5 or 7, lines 72 to 81 and 460 to 462, preferably of Table I B, columns 5 or 7, lines 72 to 81 and 460 to 462 has further one or more of the activities annotated or known for the a protein as indicated in Table II, column 3, lines 72 to 81 and 460 to 462.

**[0150.0.7.7]** Moreover, the nucleic acid molecule of the invention or used in the process of the invention can comprise only a portion of the coding region of one of the sequences indicated in Table I, columns 5 or 7, lines 72 to 81 and 460 to 462, preferably of Table I B, columns 5 or 7, lines 72 to 81 and 460 to 462, for example a fragment which can be used as a probe or primer or a fragment encoding a biologically active portion of the polypeptide of the present invention or of a polypeptide used in the process of the present invention, i.e. having above-mentioned activity, e.g.conferring an increase of methionine if its activity is increased. The nucleotide sequences determined from the cloning of the present protein-according-to-the-invention-encoding gene allows for the generation of probes and primers designed for use in identifying and/or cloning its homologues in other cell types and organisms. The probe/primer typically comprises substantially purified oligonucleotide. The oligonucleotide typically comprises a region of nucleotide sequence that hybridizes under stringent conditions to at least about 12, 15 preferably about 20 or 25, more preferably about 40, 50 or 75 consecutive nucleotides of a sense strand of one of the sequences indicated in Table I, columns 5 or 7, lines 72 to 81 and 460 to 462, an anti-sense sequence of one of the sequences indicated in Table I, columns 5 or 7, lines 72 to 81 and 460 to 462, or naturally occurring mutants thereof. Primers based on a nucleotide sequence of the invention can be used in PCR reactions to clone homologues of the polypeptide of the invention or of the polypeptide used in the process of the invention, e.g. as the primers described in the examples of the present invention, e.g. as shown in the examples. A PCR with the primer pairs indicated in Table III, column 7, lines 72 to 81 and 460 to 462 will result in a fragment of a polynucleotide sequence as indicated in Table I, columns 5 or 7, lines 72 to 81 and 460 to 462. Preferred is Table I B, column 7, lines 72 to 81 and 460 to 462.

**[0151.0.0.7]** for the disclosure of this paragraph see [0151.0.0.0] above.

**[0152.0.7.7]** The nucleic acid molecule of the invention encodes a polypeptide or portion thereof which includes an amino acid sequence which is sufficiently homologous to the amino acid sequence shown in Table II, columns 5 or 7, lines 72 to 81 and 460 to 462 such that the protein or portion thereof maintains the ability to participate in the fine chemical production, in particular a stearic acid increasing the activity as mentioned above or as described in the examples in plants or microorganisms is comprised.

**[0153.0.7.7]** As used herein, the language "sufficiently homologous" refers to proteins or portions thereof which have amino acid sequences which include a minimum number of identical or equivalent amino acid residues (e.g., an amino acid residue which has a similar side chain as an amino acid residue in one of the sequences of the polypeptide of the present invention) to an amino acid sequence shown in Table II, columns 5 or 7, lines 72 to 81 and 460 to 462 such that the protein or portion thereof is able to participate in the increase of the respective fine chemical production. In one embodiment, a protein or protion thereof as shown in Table II, columns 5 or 7, lines 72 to 81 and 460 to 462 has for example an activity of a polypeptide as indicated in Table II, columns 5 or 7, lines 72 to 81 and 460 to 462 are described herein.

**[0154.0.7.7]** In one embodiment, the nucleic acid molecule of the present invention comprises a nucleic acid that encodes a portion of the protein of the present invention. The protein is at least about 30%, 35%, 40%, 45% or 50%, preferably at least about 55%, 60%, 65% or 70%, and more preferably at least about 75%, 80%, 85%, 90%, 91%, 92%, 93% or 94% and most preferably at least about 95%, 97%, 98%, 99% or more homologous to an entire amino acid sequence as shown in Table II, columns 5 or 7, lines 72 to 81 and 460 to 462 and having above-mentioned activity, e.g.conferring preferably the increase of the respective fine chemical.

**[0155.0.0.7] and [0156.0.0.7]** for the disclosure of the paragraphs [0155.0.0.7] and [0156.0.0.7] see paragraphs [0155.0.0.0] and [0156.0.0.0] above.

**[0157.0.7.7]** The invention further relates to nucleic acid molecules that differ from one of the nucleotide sequences indicated in Table I, columns 5 or 7, lines 72 to 81 and 460 to 462 (and portions thereof) due to degeneracy of the genetic code and thus encode a polypeptide of the present invention, in particular a polypeptide having above mentioned activity, e.g. conferring an increase in the respective fine chemical in a organism, e.g. as that polypeptides comprising the consensus sequences as indicated in Table IV, column 7, lines 72 to 81 and 460 to 462 or of the polypeptide as indicated in Table II, columns 5 or 7, lines 72 to 81 and 460 to 462 or their functional homologues. Advantageously, the nucleic acid molecule of the invention or the nucleic acid molecule used in the method of the invention comprises, or in an other embodiment has, a nucleotide sequence encoding a protein comprising, or in an other embodiment having, a consensus sequences as indicated in Table IV, column 7, lines 72 to 81 and 460 to 462 or of the polypeptide as indicated in Table II, columns 5 or 7, lines 72 to 81 and 460 to 462 or the functional homologues. In a still further embodiment, the nucleic acid molecule of the invention or the nucleic acid molecule used in the method of the invention encodes a full length protein which is substantially homologous to an amino acid sequence comprising a consensus sequence as indicated in Table IV, column 7, lines 72 to 81 and 460 to 462 or of a polypeptide as indicated in Table II, columns 5 or 7, lines 72 to 81 and 460 to 462 or the functional homologues thereof. However, in a preferred embodiment, the nucleic acid molecule of the present invention does not consist of a sequence as indicated in Table I, columns 5 or 7, lines 72 to 81 and 460 to 462, preferably as indicated in Table I A, columns 5 or 7, lines 72 to 81 and 460 to 462. Preferably the nucleic acid molecule of the invention is a functional homologue or identical to a nucleic acid molecule indicated in Table I B, columns 5 or 7, lines 72 to 81 and 460 to 462.

**[0158.0.0.7] to [0160.0.0.7]** for the disclosure of the paragraphs [0158.0.0.7] to [0160.0.0.7] see paragraphs [0158.0.0.0] to [0160.0.0.0] above.

**[0161.0.7.7]** Accordingly, in another embodiment, a nucleic acid molecule of the invention or the nucleic acid molecule used in the method of the invention is at least 15, 20, 25 or 30 nucleotides in length. Preferably, it hybridizes under stringent conditions to a nucleic acid molecule comprising a nucleotide sequence of the nucleic acid molecule of the present invention or used in the process of the present invention, e.g. comprising the sequence shown in Table I, columns 5 or 7, lines 72 to 81 and 460 to 462. The nucleic acid molecule is preferably at least 20, 30, 50, 100, 250 or more nucleotides in length.

**[0162.0.0.7]** for the disclosure of this paragraph see paragraph [0162.0.0.0] above.

**[0163.0.7.7]** Preferably, nucleic acid molecule of the invention that hybridizes under stringent conditions to a sequence as indicated in Table I, columns 5 or 7, lines 72 to 81 and 460 to 462 corresponds to a naturally-occurring nucleic acid molecule of the invention. As used herein, a "naturally-occurring" nucleic acid molecule refers to an RNA or DNA molecule having a nucleotide sequence that occurs in nature (e.g., encodes a natural protein). Preferably, the nucleic acid molecule encodes a natural protein having above-mentioned activity, e.g. conferring the respective fine chemical increase after increasing the expression or activity thereof or the activity of a protein of the invention or used in the process of the invention.

**[0164.0.0.7]** for the disclosure of this paragraph see paragraph [0164.0.0.0] above.

**[0165.0.7.7]** For example, nucleotide substitutions leading to amino acid substitutions at "non-essential" amino acid residues can be made in a sequence of the nucleic acid molecule of the invention or used in the process of the invention, e.g. as shown in Table I, columns 5 or 7, lines 72 to 81 and 460 to 462.

**[0166.0.0.7] and [0167.0.0.7]** for the disclosure of the paragraphs [0166.0.0.7] and [0167.0.0.7] see paragraphs [0166.0.0.0] and [0167.0.0.0] above.

**[0168.0.7.7]** Accordingly, the invention relates to nucleic acid molecules encoding a polypeptide having above-mentioned activity, e.g. conferring an increase in the respective fine chemical in an organism or parts thereof that contain changes in amino acid residues that are not essential for said activity. Such polypeptides differ in amino acid sequence from a sequence contained in a sequence as indicated in Table II, columns 5 or 7, lines 72 to 81 and 460 to 462 yet retain said activity described herein. The nucleic acid molecule can comprise a nucleotide sequence encoding a polypeptide, wherein the polypeptide comprises an amino acid sequence at least about 50% identical to an amino acid sequence as indicated in Table II, columns 5 or 7, lines 72 to 81 and 460 to 462 and is capable of participation in the increase of production of the respective fine chemical after increasing its activity, e.g. its expression. Preferably, the protein encoded by the nucleic acid molecule is at least about 60% identical to the sequence as indicated in Table II, columns 5 or 7, lines 72 to 81 and 460 to 462, more preferably at least about 70% identical to one of the sequences as indicated in Table II, columns 5 or 7, lines 72 to 81 and 460 to 462, even more preferably at least about 80%, 90%, 95% homologous to the sequence as indicated in Table II, columns 5 or 7, lines 72 to 81 and 460 to 462, and most preferably at least about 96%, 97%, 98%, or 99% identical to the sequence as indicated in Table II, columns 5 or 7, lines 72 to 81 and 460 to 462.
Accordingly, the invention relates to nucleic acid molecules encoding a polypeptide having above-mentioned activity, e.g. conferring an increase in the the respective fine chemical in an organisms or parts thereof that contain changes in amino acid residues that are not essential for said activity. Such polypeptides differ in amino acid sequence from a sequence contained in a sequence as indicated in Table II, columns 5 or 7, lines 72 to 81 and 460 to 462, preferably of Table II B, column 7, lines 72 to 81 and 460 to 462 yet retain said activity described herein. The nucleic acid molecule can comprise a nucleotide sequence encoding a polypeptide, wherein the polypeptide comprises an amino acid sequence at least about 50% identical to an amino acid sequence as indicated in Table II, columns 5 or 7, lines 72 to 81 and 460 to 462, preferably of Table II B, column 7, lines 72 to 81 and 460 to 462 and is capable of participation in the increase of production of the respective fine chemical after increasing its activity, e.g. its expression. Preferably, the protein encoded by the nucleic acid molecule is at least about 60% identical to a sequence as indicated in Table II, columns 5 or 7, lines 72 to 81 and 460 to 462, preferably of Table II B, column 7, lines 72 to 81 and 460 to 462, more preferably at least about 70% identical to one of the sequences as indicated in Table II, columns 5 or 7, lines 72 to 81 and 460 to 462, preferably of Table II B, column 7, lines 72 to 81 and 460 to 462, even more preferably at least about 80%, 90%, or 95% homologous to a sequence as indicated in Table II, columns 5 or 7, lines 72 to 81 and 460 to 462, preferably of Table II B, column 7, lines 72 to 81 and 460 to 462, and most preferably at least about 96%, 97%, 98%, or 99% identical to the sequence as indicated in Table II, columns 5 or 7, lines 72 to 81 and 460 to 462, preferably of Table II B, column 7, lines 72 to 81 and 460 to 462.

**[0169.0.0.7] to [0175.0.5.7]** for the disclosure of the paragraphs [0169.0.0.7] to [0175.0.5.7] see paragraphs [0169.0.0.0] to [0175.0.0.0] above.

**[0176.0.7.7]** Functional equivalents derived from one of the polypeptides as shown in Table II, columns 5 or 7, lines 72 to 81 and 460 to 462 according to the invention by substitution, insertion or deletion have at least 30%, 35%, 40%, 45% or 50%, preferably at least 55%, 60%, 65% or 70% by preference at least 80%, especially preferably at least 85% or 90%, 91%, 92%, 93% or 94%, very especially preferably at least 95%, 97%, 98% or 99% homology with one of the polypeptides as shown in Table II, columns 5 or 7, lines 72 to 81 and 460 to 462 according to the invention and are distinguished by essentially the same properties as the polypeptide as shown in Table II, columns 5 or 7, lines 72 to 81 and 460 to 462.

**[0177.0.7.7]** Functional equivalents derived from the nucleic acid sequence as shown in Table I, columns 5 or 7, lines 72 to 81 and 460 to 462 according to the invention by substitution, insertion or deletion have at least 30%, 35%, 40%, 45% or 50%, preferably at least 55%, 60%, 65% or 70% by preference at least 80%, especially preferably at least 85% or 90%, 91%, 92%, 93% or 94%, very especially preferably at least 95%, 97%, 98% or 99% homology with one of the polypeptides as shown in Table II, columns 5 or 7, lines 72 to 81 and 460 to 462 according to the invention and encode polypeptides having essentially the same properties as the polypeptide as shown in Table II, columns 5 or 7, lines 72 to 81 and 460 to 462.

**[0178.0.0.7]** for the disclosure of this paragraph see [0178.0.0.0] above.

**[0179.0.7.7]** A nucleic acid molecule encoding a homologous to a protein sequence of as indicated in Table II, columns 5 or 7, lines 72 to 81 and 460 to 462, preferably of Table II B, column 7, lines 72 to 81 and 460 to 462 can be created by introducing one or more nucleotide substitutions, additions or deletions into a nucleotide sequence of the nucleic acid molecule of the present invention, in particular as indicated in Table I, columns 5 or 7, lines 72 to 81 and 460 to 462 such that one or more amino acid substitutions, additions or deletions are introduced into the encoded protein. Mutations can be introduced into the encoding sequences for example into a sequences as indicated in Table I, columns 5 or 7, lines 72 to 81 and 460 to 462 by standard techniques, such as site-directed mutagenesis and PCR-mediated mutagenesis.

**[0180.0.0.7] to [0183.0.0.7]** for the disclosure of the paragraphs [0180.0.0.7] to [0183.0.0.7] see paragraphs [0180.0.0.0] to [0183.0.0.0] above.

**[0184.0.7.7]** Homologues of the nucleic acid sequences used, with a sequence as indicated in Table I, columns 5 or 7, lines 72 to 81 and 460 to 462, preferably of Table I B, column 7, lines 72 to 81 and 460 to 462, or of the nucleic acid sequences derived from a sequences as indicated in Table II, columns 5 or 7, lines 72 to 81 and 460 to 462, preferably of Table II B, column 7, lines 72 to 81 and 460 to 462, comprise also allelic variants with at least approximately 30%, 35%, 40% or 45% homology, by preference at least approximately 50%, 60% or 70%, more preferably at least approximately 90%, 91%, 92%, 93%, 94% or 95% and even more preferably at least approximately 96%, 97%, 98%, 99% or more homology with one of the nucleotide sequences shown or the abovementioned derived nucleic acid sequences or their homologues, derivatives or analogues or parts of these. Allelic variants encompass in particular functional variants which can be obtained by deletion, insertion or substitution of nucleotides from the sequences shown, preferably from a sequence as indicated in Table I, columns 5 or 7, lines 72 to 81 and 460 to 462, or from the derived nucleic acid sequences, the intention being, however, that the enzyme activity or the biological activity of the resulting proteins synthesized is advantageously retained or increased.

**[0185.0.7.7]** In one embodiment of the present invention, the nucleic acid molecule of the invention or used in the process of the invention comprises one or more sequences as indicated in Table I, columns 5 or 7, lines 72 to 81 and 460 to 462, preferably of Table I B, column 7, lines 72 to 81 and 460 to 462. In one embodiment, it is preferred that the nucleic acid molecule comprises as little as possible other nucleotide sequences not shown in any one of sequences as indicated in Table I, columns 5 or 7, lines 72 to 81 and 460 to 462, preferably of Table I B, column 7, lines 72 to 81 and 460 to 462. In one embodiment, the nucleic acid molecule comprises less than 500, 400, 300, 200, 100, 90, 80, 70, 60, 50 or 40 further nucleotides. In a further embodiment, the nucleic acid molecule comprises less than 30, 20 or 10 further nucleotides. In one embodiment, a nucleic acid molecule used in the process of the invention is identical to a sequences as indicated in Table I, columns 5 or 7, lines 72 to 81 and 460 to 462, preferably of Table I B, column 7, lines 72 to 81 and 460 to 462.

**[0186.0.7.7]** Also preferred is that one or more nucleic acid molecule(s) used in the process of the invention encode a polypeptide comprising a sequence as indicated in Table II, columns 5 or 7, lines 72 to 81 and 460 to 462, preferably of Table II B, column 7, lines 72 to 81 and 460 to 462. In one embodiment, the nucleic acid molecule encodes less than 150, 130, 100, 80, 60, 50, 40 or 30 further amino acids. In a further embodiment, the encoded polypeptide comprises less than 20, 15, 10, 9, 8, 7, 6 or 5 further amino acids. In one embodiment, the encoded polypeptide used in the process of the invention is identical to the sequences as indicated in Table II, columns 5 or 7, lines 72 to 81 and 460 to 462, preferably of Table II B, column 7, lines 72 to 81 and 460 to 462.

**[0187.0.7.7]** In one embodiment, the nucleic acid molecule of the invention or used in the process encodes a polypeptide comprising a sequence as indicated in Table II, columns 5 or 7, lines 72 to 81 and 460 to 462, preferably of Table II B, column 7, lines 72 to 81 and 460 to 462 comprises less than 100 further nucleotides. In a further embodiment, said nucleic acid molecule comprises less than 30 further nucleotides. In one embodiment, the nucleic acid molecule used in the process is identical to a coding sequence encoding a sequences as indicated in Table II, columns 5 or 7, lines 72 to 81 and 460 to 462, preferably of Table II B, column 7, lines 72 to 81 and 460 to 462.

**[0188.0.7.7]** Polypeptides (= proteins), which still have the essential biological activity of the polypeptide of the present invention conferring an increase of the fine chemical i.e. whose activity is essentially not reduced, are polypeptides with at least 10% or 20%, by preference 30% or 40%, especially preferably 50% or 60%, very especially preferably 80% or 90 or more of the wild type biological activity or enzyme activity, advantageously, the activity is essentially not reduced in comparison with the activity of a polypeptide shown in Table II, columns 5 or 7, lines 72 to 81 and 460 to 462 expressed under identical conditions.
In one embodiment, the polypeptide of the invention is a homolog consisting of or comprising the sequence as indicated in Table II B, columns 7, lines 72 to 81 and 460 to 462.

**[0189.0.7.7]** Homologues of sequences as indicated in Table I, columns 5 or 7, lines 72 to 81 and 460 to 462 or of the derived sequences shown in Table II, columns 5 or 7, lines 72 to 81 and 460 to 462 also mean truncated sequences, cDNA, single-stranded DNA or RNA of the coding and noncoding DNA sequence. Homologues of said sequences are also understood as meaning derivatives, which comprise noncoding regions such as, for example, UTRs, terminators, enhancers or promoter variants. The promoters upstream of the nucleotide sequences stated can be modified by one or more nucleotide substitution(s), insertion(s) and/or deletion(s) without, however, interfering with the functionality or activity either of the promoters, the open reading frame (= ORF) or with the 3'-regulatory region such as terminators or other 3'regulatory regions, which are far away from the ORF. It is furthermore possible that the activity of the promoters is increased by modification of their sequence, or that they are replaced completely by more active promoters, even promoters from heterologous organisms. Appropriate promoters are known to the person skilled in the art and are mentioned herein below.

**[0190.0.0.7], [0191.0.5.7], [00191.1.0.7] and [0192.0.0.7] to [0203.0.0.7]** for the disclosure of the paragraphs [0190.0.0.7], [0191.0.5.7], [0191.1.0.7] and [0192.0.0.7] to [0203.0.0.7] see paragraphs [0190.0.0.0], [0191.0.0.0], [0191.1.0.0] and [0192.0.0.0] to [0203.0.0.0] above.

**[0204.0.7.7]** Accordingly, in one embodiment, the invention relates to a nucleic acid molecule, which comprises a nucleic acid molecule selected from the group consisting of:
a) nucleic acid molecule encoding, preferably at least the mature form, of the polypeptide shown in Table II, columns 5 or 7, lines 72 to 81 and 460 to 462; preferably of Table II B, column 7, lines 72 to 81 and 460 to 462; or a fragment thereof conferring an increase in the amount of the fine chemical in an organism or a part thereof
b) nucleic acid molecule comprising, preferably at least the mature form, of the nucleic acid molecule shown in Table I, columns 5 or 7, lines 72 to 81 and 460 to 462 preferably of Table I B, column 7, lines 72 to 81 and 460 to 462; or a fragment thereof conferring an increase in the amount of the fine chemical in an organism or a part thereof;
c) nucleic acid molecule whose sequence can be deduced from a polypeptide sequence encoded by a nucleic acid molecule of (a) or (b) as result of the degeneracy of the genetic code and conferring an increase in the amount of the respective fine chemical in an organism or a part thereof;
d) nucleic acid molecule encoding a polypeptide whose sequence has at least 50% identity with the amino acid sequence of the polypeptide encoded by the nucleic acid molecule of (a) to (c) and conferring an increase in the amount of the respective fine chemical in an organism or a part thereof;
e) nucleic acid molecule which hybridizes with a nucleic acid molecule of (a) to (c) under under stringent hybridisation conditions and conferring an increase in the amount of the respective fine chemical in an organism or a part thereof;
f) nucleic acid molecule encoding a polypeptide, the polypeptide being derived by substituting, deleting and/or adding one or more amino acids of the amino acid sequence of the polypeptide encoded by the nucleic acid molecules (a) to (d), preferably to (a) to (c), and conferring an increase in the amount of the respective fine chemical in an organism or a part thereof;
g) nucleic acid molecule encoding a fragment or an epitope of a polypeptide which is encoded by one of the nucleic acid molecules of (a) to (e), preferably to (a) to (c) and conferring an increase in the amount of the respective fine chemical in an organism or a part thereof ;
h) nucleic acid molecule comprising a nucleic acid molecule which is obtained by amplifying a cDNA library or a genomic library using the primers or primer pairs as indicated in Table III, column 7, lines 72 to 81 and 460 to 462 and conferring an increase in the amount of the respective fine chemical in an organism or a part thereof;
i) nucleic acid molecule encoding a polypeptide which is isolated, e.g. from a expression library, with the aid of monoclonal antibodies against a polypeptide encoded by one of the nucleic acid molecules of (a) to (g), preferably to (a) to (c) and conferring an increase in the amount of the respective fine chemical in an organism or a part thereof;
j) nucleic acid molecule which encodes a polypeptide comprising the consensus sequence shown in Table IV, column 7, lines 72 to 81 and 460 to 462 and conferring an increase in the amount of the respective fine chemical in an organism or a part thereof;
k) nucleic acid molecule encoding the amino acid sequence of a polypeptide encoding a domaine of the polypeptide shown in Table II, columns 5 or 7, lines 72 to 81 and 460 to 462, preferably of Table II B, column 7, lines 72 to 81 and 460 to 462; and conferring an increase in the amount of the respective fine chemical in an organism or a part thereof; and
l) nucleic acid molecule which is obtainable by screening a suitable nucleic acid library under stringent hybridization conditions with a probe comprising one of the sequences of the nucleic acid molecule of (a) to (k) or with a fragment of at least 15 nt, preferably 20 nt, 30 nt, 50 nt, 100 nt, 200 nt or 500 nt of the nucleic acid molecule characterized in (a) to (h) or of the nucleic acid molecule shown in Table I, columns 5 or 7, lines 72 to 81 and 460 to 462 or a nucleic acid molecule encoding, preferably at least the mature form of, the polypeptide shown in Table II, columns 5 or 7, lines 72 to 81 and 460 to 462, and conferring an increase in the amount of the respective fine chemical in an organism or a part thereof; or which encompasses a sequence which is complementary thereto;
whereby, preferably, the nucleic acid molecule according to (a) to (I) distinguishes over the sequence as depicted in Table IA, columns 5 or 7, lines 72 to 81 and 460 to 462 by one or more nucleotides. In one embodiment, the nucleic acid molecule of the invention does not consist of the sequence shown in Table IA or IB, columns 5 or 7, lines 72 to 81 and 460 to 462. In an other embodiment, the nucleic acid molecule of the present invention is at least 30 % identical and less than 100%, 99,999%, 99,99%, 99,9% or 99% identical to the sequence shown in Table IA or IB, columns 5 or 7, lines 72 to 81 and 460 to 462. In a further embodiment the nucleic acid molecule does not encode the polypeptide sequence shown in Table IIA or IIB, columns 5 or 7, lines 72 to 81 and 460 to 462. Accordingly, in one embodiment, the nucleic acid molecule of the present invention encodes in one embodiment a polypeptide which differs at least in one or more amino acids from the polypeptide as depicted in Table IIA or IIB, columns 5 or 7, lines 72 to 81 and 460 to 462 and therefore does not encode a protein of the sequence shown in Table IIA or IIB, columns 5 or 7, lines 72 to 81 and 460 to 462. Accordingly, in one embodiment, the protein encoded by a sequence of a nucleic acid according to (a) to (I) does not consist of the sequence shown in Table IIA or IIB, columns 5 or 7, lines 72 to 81 and 460 to 462. In a further embodiment, the protein of the present invention is at least 30 % identical to protein sequence depicted in Table IIA or IIB, columns 5 or 7, lines 72 to 81 and 460 to 462 and less than 100%, preferably less than 99,999%, 99,99% or 99,9%, more preferably less than 99%, 985, 97%, 96% or 95% identical to the sequence shown in Table IIA or IIB, columns 5 or 7, lines 72 to 81 and 460 to 462.

**[0205.0.0.7] to [0226.0.0.7]** for the disclosure of the paragraphs [0205.0.0.7] to [0226.0.0.7] see paragraphs [0205.0.0.0] to [0226.0.0.0] above.

**[0227.0.7.7]** The abovementioned nucleic acid molecules can be cloned into the nucleic acid constructs or vectors according to the invention in combination together with further genes, or else different genes are introduced by transforming several nucleic acid constructs or vectors (including plasmids) into a host cell, advantageously into a plant cell or a microorgansims.
In addition to the sequence mentioned in Table I, columns 5 or 7, lines 72 to 81 and 460 to 462 or its derivatives, it is advantageous additionally to express and/or mutate further genes in the organisms. Especially advantageously, additionally at least one further gene of the fatty acid biosynthetic pathway such as for palmitate, palmitoleate, stearate and/or oleate is expressed in the organisms such as plants or microorganisms. It is also possible that the regulation of the natural genes has been modified advantageously so that the gene and/or its gene product is no longer subject to the regulatory mechanisms which exist in the organisms. This leads to an increased synthesis of the amino acids desired since, for example, feedback regulations no longer exist to the same extent or not at all. In addition it might be advantageously to combine the sequences shown in Table I, columns 5 or 7, lines 72 to 81 and 460 to 462 with genes which generally support or enhances to growth or yield of the target organismen, for example genes which lead to faster growth rate of microorganisms or genes which produces stress-, pathogen, or herbicide resistant plants.

**[0228.0.5.7] to [0230.0.5.7]** for the disclosure of the paragraphs [0228.0.5.7] to [0230.0.5.7] see paragraphs [0228.0.0.0] to [0230.0.0.0] above.

**[0231.0.7.7]** In a further advantageous embodiment of the process of the invention, the organisms used in the process are those in which simultaneously a stearic acid degrading protein is attenuated, in particular by reducing the rate of expression of the corresponding gene.

**[0232.0.0.7] to [0276.0.0.7]** for the disclosure of the paragraphs [0232.0.0.7] to [0276.0.0.7] see paragraphs [0232.0.0.0] to [0276.0.0.0] above.

**[0277.0.5.7]** for the disclosure of this paragraph see paragraph [0277.0.5.5] above.

**[0278.0.0.7] to [0282.0.0.7]** for the disclosure of the paragraphs [0278.0.0.7] to [0282.0.0.7] see paragraphs [0278.0.0.0] to [0282.0.0.0] above.

**[0283.0.7.7]** Moreover, native polypeptide conferring the increase of the respective fine chemical in an organism or part thereof can be isolated from cells (e.g., endothelial cells), for example using the antibody of the present invention as described below, in particular, an antibody against polypeptides as shown in Table II, columns 5 or 7, lines 72 to 81 and 460 to 462 or an antigenic part thereof, which can be produced by standard techniques utilizing polypeptides comprising or consisting of abovementioned sequences, e.g. the polypeptid of the present invention or fragment thereof. Preferred are monoclonal antibodies specifically binding to polypeptides as indicated in Table II, columns 5 or 7, lines 72 to 81 and 460 to 462.

**[0284.0.0.7]** for the disclosure of this paragraph see [0284.0.0.0] above.

**[0285.0.7.7]** In one embodiment, the present invention relates to a polypeptide having the sequence shown in Table II, columns 5 or 7, lines 72 to 81 and 460 to 462 or as coded by the nucleic acid molecule shown in Table I, columns 5 or 7, lines 72 to 81 and 460 to 462 or functional homologues thereof.

**[0286.0.7.7]** In one advantageous embodiment, in the method of the present invention the activity of a polypeptide is increased which comprises or consists of a consensus sequence as indicated in Table IV, column 7, lines 72 to 81 and 460 to 462 and in one another embodiment, the present invention relates to a polypeptide comprising or consisting of a consensus sequence as indicated in Table IV, column 7, lines 72 to 81 and 460 to 462, whereby 20 or less, preferably 15 or 10, preferably 9, 8, 7, or 6, more preferred 5 or 4, even more preferred 3, even more preferred 2, even more preferred 1, most preferred 0 of the amino acids positions indicated can be replaced by any amino acid or, in an further embodiment, can be replaced and/or absent. In one embodiment, the present invention relates to the method of the present invention comprising a poylpeptide or to a polypeptide comprising more than one consensus sequences as indicated in Table IV, column 7, lines 72 to 81 and 460 to 462.

**[0287.0.0.7] to [0290.0.0.7]** for the disclosure of the paragraphs [0287.0.0.7] to [0290.0.0.7] see paragraphs [0287.0.0.0] to [0290.0.0.0] above.

**[0291.0.7.7]** In one advantageous embodiment, the method of the present invention comprises the increasing of a polypeptide comprising or consisting of plant or microorganism specific consensus sequences. Accordingly, in one embodiment, the present invention relates to a polypeptide comprising or consisting of a plant or microorganism specific consensus sequences.
In one embodiment, said polypeptide of the invention distinguishes over the sequence as indicated in Table IIA or IIB, columns 5 or 7, lines 72 to 81 and 460 to 462 by one or more amino acids. In one embodiment, polypeptide distinguishes form the sequence shown in Table IIA or IIB, columns 5 or 7, lines 72 to 81 and 460 to 462 by more than 5, 6, 7, 8 or 9 amino acids, preferably by more than 10, 15, 20, 25 or 30 amino acids, evenmore preferred are more than 40, 50, or 60 amino acids and, preferably, the sequence of the polypeptide of the invention distinguishes from the sequence shown in Table IIA or IIB, columns 5 or 7, lines 72 to 81 and 460 to 462 by not more than 80% or 70% of the amino acids, preferably not more than 60% or 50%, more preferred not more than 40% or 30%, even more preferred not more than 20% or 10%. In another embodiment, said polypeptide of the invention does not consist of the sequence shown in Table IIA or IIB, columns 5 or 7, lines 72 to 81 and 460 to 462.

**[0292.0.0.7]** for the disclosure of this paragraph see [0292.0.0.0] above.

**[0293.0.7.7]** In one embodiment, the invention relates to polypeptide conferring an increase in the respective fine chemical in an organism or part being encoded by the nucleic acid molecule of the invention or by a nucleic acid molecule used in the process.
In one embodiment, the polypeptide of the invention has a sequence, which distinguishes from the sequence as shown in Table IIA or IIB, columns 5 or 7, lines 72 to 81 and 460 to 462 by one or more amino acids. In another embodiment, said polypeptide of the invention does not consist of the sequence shown in Table IIA or IIB, columns 5 or 7, lines 72 to 81 and 460 to 462. In a further embodiment, said polypeptide of the present invention is less than 100%, 99,999%, 99,99%, 99,9% or 99% identical. In one embodiment, said polypeptide does not consist of the sequence encoded by the nucleic acid molecules shown in Table IA or IB, columns 5 or 7, lines 72 to 81 and 460 to 462.

**[0294.0.7.7]** In one embodiment, the present invention relates to a polypeptide having an activity of a protein as indicated in Table II, column 3, lines 72 to 81 and 460 to 462, which distinguishes over the sequence as indicated in Table IIA or IIB, columns 5 or 7, lines 72 to 81 and 460 to 462 by one or more amino acids, preferably by more than 5, 6, 7, 8 or 9 amino acids, preferably by more than 10, 15, 20, 25 or 30 amino acids, evenmore preferred are more than 40, 50, or 60 amino acids but even more preferred by less than 70% of the amino acids, more preferred by less than 50%, even more preferred my less than 30% or 25%, more preferred are 20% or 15%, even more preferred are less than 10%.

**[0295.0.0.7], [0296.0.0.7] and [0297.0.5.7]** for the disclosure of the paragraphs [0295.0.0.7], [0296.0.0.7] and [0297.0.5.7] see paragraphs [0295.0.0.0] to [0297.0.0.0] above.

**[00297.1.0.7]** Non-polypeptide of the invention-chemicals are e.g. polypeptides having not the activity and/or the amino acid sequence of a polypeptide indicated in Table II, columns 3, 5 or 7, lines 72 to 81 and 460 to 462.

**[0298.0.7.7]** A polypeptide of the invention can participate in the process of the present invention. The polypeptide or a portion thereof comprises preferably an amino acid sequence which is sufficiently homologous to an amino acid sequence as indicated in Table II, columns 5 or 7, lines 72 to 81 and 460 to 462 such that the protein or portion thereof maintains the ability to confer the activity of the present invention. The portion of the protein is preferably a biologically active portion as described herein. Preferably, the polypeptide used in the process of the invention has an amino acid sequence identical to a sequence as indicated in Table II, columns 5 or 7, lines 72 to 81 and 460 to 462.

**[0299.0.7.7]** Further, the polypeptide can have an amino acid sequence which is encoded by a nucleotide sequence which hybridizes, preferably hybridizes under stringent conditions as described above, to a nucleotide sequence of the nucleic acid molecule of the present invention. Accordingly, the polypeptide has an amino acid sequence which is encoded by a nucleotide sequence that is at least about 35%, 40%, 45%, 50%, 55%, 60%, 65% or 70%, preferably at least about 75%, 80%, 85% or 90, and more preferably at least about 91%, 92%, 93%, 94% or 95%, and even more preferably at least about 96%, 97%, 98%, 99% or more homologous to one of the amino acid sequences of Table II, columns 5 or 7, lines 72 to 81 and 460 to 462. The preferred polypeptide of the present invention preferably possesses at least one of the activities according to the invention and described herein. A preferred polypeptide of the present invention includes an amino acid sequence encoded by a nucleotide sequence which hybridizes, preferably hybridizes under stringent conditions, to a nucleotide sequence of Table I, columns 5 or 7, lines 72 to 81 and 460 to 462 or which is homologous thereto, as defined above.

**[0300.0.7.7]** Accordingly the polypeptide of the present invention can vary from a sequence as indicated in Table II, columns 5 or 7, lines 72 to 81 and 460 to 462 in amino acid sequence due to natural variation or mutagenesis, as described in detail herein. Accordingly, the polypeptide comprise an amino acid sequence which is at least about 35%, 40%, 45%, 50%, 55%, 60%, 65% or 70%, preferably at least about 75%, 80%, 85% or 90, and more preferably at least about 91%, 92%, 93%, 94% or 95%, and most preferably at least about 96%, 97%, 98%, 99% or more homologous to an entire amino acid sequence of a sequence as indicated in Table IIA or IIB, columns 5 or 7, lines 72 to 81 and 460 to 462.

**[0301.0.0.7]** for the disclosure of this paragraph see [0301.0.0.0] above.

**[0302.0.7.7]** Biologically active portions of an polypeptide of the present invention include peptides comprising amino acid sequences derived from the amino acid sequence of the polypeptide of the present invention or used in the process of the present invention, e.g., an amino acid sequence shown in Table II, columns 5 or 7, lines 72 to 81 and 460 to 462 or the amino acid sequence of a protein homologous thereto, which include fewer amino acids than a full length polypeptide of the present invention or used in the process of the present invention or the full length protein which is homologous to an polypeptide of the present invention or used in the process of the present invention depicted herein, and exhibit at least one activity of polypeptide of the present invention or used in the process of the present invention.

**[0303.0.0.7]** for the disclosure of this paragraph see [0303.0.0.0] above.

**[0304.0.7.7]** Manipulation of the nucleic acid molecule of the invention or the nucleic acid molecule used in the method of the invention may result in the production of a protein having essentially the activity of the polypeptides as indicated in Table II, columns 5 or 7, lines 72 to 81 and 460 to 462 but having differences in the sequence from said wild-type protein. These proteins may be improved in efficiency or activity, may be present in greater numbers in the cell than is usual, or may be decreased in efficiency or activity in relation to the wild type protein.

**[0305.0.5.7], [0306.0.5.7] and [0306.1.0.7]** for the disclosure of the paragraphs [0305.0.5.7], [0306.0.5.7] and [0306.1.0.7] see paragraphs [0305.0.0.0], [0306.0.0.0] and [0306.1.0.0] above.

**[0307.0.0.7] and [0308.0.0.7]** for the disclosure of the paragraphs [0307.0.0.7] and [0308.0.0.7] see paragraphs [0307.0.0.0 and [0308.0.0.0] above.

**[0309.0.7.7]** In one embodiment, a reference to protein (= polypeptide)" of the invention e.g. as indicated in Table II, columns 5 or 7, lines 72 to 81 and 460 to 462 refers to a polypeptide having an amino acid sequence corresponding to the polypeptide of the invention or used in the process of the invention, whereas a "non-polypeptide" or "other polypeptide" e.g. not indicated in Table II, columns 5 or 7, lines 72 to 81 and 460 to 462 refers to a polypeptide having an amino acid sequence corresponding to a protein which is not substantially homologous to a polypeptide of the invention, preferably which is not substantially homologous to a polypeptide having a protein activity, e.g., a protein which does not confer the activity described herein and which is derived from the same or a different organism.

**[0310.0.0.7] to [0334.0.0.7]** for the disclosure of the paragraphs [0310.0.0.7] to [0334.0.0.7] see paragraphs [0310.0.0.0] to [0334.0.0.0] above.

**[0335.0.7.7]** The dsRNAi method has proved to be particularly effective and advantageous for reducing the expression of a nucleic acid sequence as indicated in Table I, columns 5 or 7, lines 72 to 81 and 460 to 462 and/or homologs thereof. As described inter alia in WO 99/32619, dsRNAi approaches are clearly superior to traditional antisense approaches. The invention therefore furthermore relates to double-stranded RNA molecules (dsRNA molecules) which, when introduced into an organism, advantageously into a plant (or a cell, tissue, organ or seed derived therefrom), bring about altered metabolic activity by the reduction in the expression of a nucleic acid sequences as indicated in Table I, columns 5 or 7, lines 72 to 81 and 460 to 462 and/or homologs thereof. In a double-stranded RNA molecule for reducing the expression of an protein encoded by a nucleic acid sequence of one of the sequences as indicated in Table I, columns 5 or 7, lines 72 to 81 and 460 to 462 and/or homologs thereof, one of the two RNA strands is essentially identical to at least part of a nucleic acid sequence, and the respective other RNA strand is essentially identical to at least part of the complementary strand of a nucleic acid sequence.

**[0336.0.0.7] to [0342.0.0.7]** for the disclosure of the paragraphs [0336.0.0.7] to [0342.0.0.7] see paragraphs [0336.0.0.0] to [0342.0.0.0] above.

**[0343.0.7.7]** As has already been described, 100 % sequence identity between the dsRNA and a gene transcript of a nucleic acid sequence as indicated in Table I, columns 5 or 7, lines 72 to 81 and 460 to 462 or it's homolog is not necessarily required in order to bring about effective reduction in the expression. The advantage is, accordingly, that the method is tolerant with regard to sequence deviations as may be present as a consequence of genetic mutations, polymorphisms or evolutionary divergences. Thus, for example, using the dsRNA, which has been generated starting from a sequence of one of the sequences as indicated in Table I, columns 5 or 7, lines 72 to 81 and 460 to 462 or homologs thereof of the one organism, may be used to suppress the corresponding expression in another organism.

**[0344.0.0.7] to [0350.0.0.7], [0351.0.5.7] and [0352.0.0.7] to [0361.0.0.7]** for the disclosure of the paragraphs [0344.0.0.7] to [0350.0.0.7], [0351.0.5.7] and [0352.0.0.7] to [0361.0.0.7] see paragraphs [0344.0.0.0] to [0361.0.0.0] above.

**[0362.0.7.7]** Accordingly the present invention relates to any cell transgenic for any nucleic acid characterized as part of the invention, e.g. conferring the increase of the respective fine chemical in a cell or an organism or a part thereof, e.g. the nucleic acid molecule of the invention or the nucleic acid molecule used in the method of the invention, the nucleic acid construct of the invention, the antisense molecule of the invention, the vector of the invention or a nucleic acid molecule encoding the polypeptide of the invention, e.g. encoding a polypeptide having an activity of a protein such as the polypeptides as indicated in Table II, column 3, lines 72 to 81 and 460 to 462. Due to the above mentioned activity the fine chemical content in a cell or an organism is increased. For example, due to modulation or manupulation, the cellular activity is increased, e.g. due to an increased expression or specific activity of the subject matters of the invention in a cell or an organism or a part thereof. Transgenic for a polypeptide having an activity of a protein such as the polypeptides as indicated in Table II, column 3, lines 72 to 81 and 460 to 462. Activity means herein that due to modulation or manipulation of the genome, the activity of polypeptide having an activity of a protein such as the polypeptides as indicated in Table II, column 3, lines 72 to 81 and 460 to 462 or a similar activity, which is increased in the cell or organism or part thereof. Examples are described above in context with the process of the invention.

**[0363.0.0.7], [0364.0.5.7] and [0365.0.0.7] to [0379.0.5.7** for the disclosure of the paragraphs [0363.0.0.7], [0364.0.5.7] and [0365.0.0.7] to [0379.0.5.7] see paragraphs [0363.0.0.0] to [0379.0.0.0] above.

**[0380.0.5.7], [0381.0.0.7] and [0382.0.0.7]** for the disclosure of the paragraphs [0380.0.5.7], [0381.0.0.7] and [0382.0.0.7] see paragraphs [0380.0.5.5], [0381.0.0.0] and [0382.0.0.0] above.

**[0383.0.5.7], [0384.0.0.7], [0385.0.5.7] and [0386.0.5.7]** for the disclosure of the paragraphs [0383.0.5.7], [0384.0.0.7], [0385.0.5.7] and [0386.0.5.7] see paragraphs [0383.0.5.5], [0384.0.0.0], [0385.0.5.5] and [0386.0.5.5] above.

**[0387.0.0.7] to [0392.0.0.7]** for the disclosure of the paragraphs [0387.0.0.7] to [0392.0.0.7] see paragraphs [0387.0.0.0] to [0392.0.0.0] above.

**[0393.0.7.7]** In one embodiment, the present invention relates to a method for the identification of a gene product conferring an increase in the fine chemical production in a cell, comprising the following steps:
(a) contacting, e.g. hybridising, the nucleic acid molecules of a sample, e.g. cells, tissues, plants or microorganisms or a nucleic acid library , which can contain a candidate gene encoding a gene product conferring an increase in the respective fine chemical after expression, with the nucleic acid molecule of the present invention;
(b) identifying the nucleic acid molecules, which hybridize under relaxed stringent conditions with the nucleic acid molecule of the present invention in particular to the nucleic acid molecule sequence shown in Table I, columns 5 or 7, lines 72 to 81 and 460 to 462, preferably in Table I B, columns 5 or 7, lines 72 to 81 and 460 to 462 and, optionally, isolating the full length cDNA clone or complete genomic clone;
(c) introducing the candidate nucleic acid molecules in host cells, preferably in a plant cell or a microorganism, appropriate for producing the respective fine chemical;
(d) expressing the identified nucleic acid molecules in the host cells;
(e) assaying the respective fine chemical level in the host cells; and
(f) identifying the nucleic acid molecule and its gene product which expression confers an increase in the respective fine chemical level in the host cell after expression compared to the wild type.

**[0394.0.0.7] to [0415.0.0.7] and [0416.0.5.7]** for the disclosure of the paragraphs [0394.0.0.7] to [0415.0.0.7] and [0416.0.5.7] see paragraphs [0394.0.0.0] to [0416.0.0.0] above.

**[0417.0.5.7] and [0418.0.0.7] to [0430.0.0.7]** for the disclosure of the paragraphs [0417.0.5.7] and [0418.0.0.7] to [0430.0.0.7] see paragraphs [0417.0.5.5] and [0418.0.0.0] to [0430.0.0.0] above.

**[0431.0.5.7], [0432.0.5.7], [0433.0.0.7] and [0434.0.0.7]** for the disclosure of the paragraphs [0431.0.5.7], [0432.0.5.7], [0433.0.0.7] and [0434.0.0.7] see paragraphs [0431.0.0.0] to [0434.0.0.0] above.

**[0435.0.5.7] to [0440.0.5.7]** for the disclosure of the paragraphs [0435.0.5.7] to [0440.0.5.7] see paragraphs [0435.0.5.5] to [0440.0.5.5] above.

**[0441.0.0.7] and [0442.0.5.7]** for the disclosure of the paragraphs [0441.0.0.7] and [0442.0.5.7] see [0441.0.0.0] and [0442.0.5.5] above.

**[0443.0.0.7]** for the disclosure of this paragraph see [0443.0.0.0] above.

**[0444.0.5.7] and [0445.0.5.7]** for the disclosure of the paragraphs [0444.0.5.7] and [0445.0.5.7] see [0444.0.5.5] and [0445.0.5.5] above.

**[0446.0.0.7] to [0453.0.0.7]** for the disclosure of the paragraphs [0446.0.0.7] to [0453.0.0.7] see paragraphs [0446.0.0.0] to [0453.0.0.0] above.

**[0454.0.5.7] and [0455.0.5.7]** for the disclosure of the paragraphs [0454.0.5.7] and [0455.0.5.7] see [0454.0.5.5] and [0455.0.5.5] above.

**[0456.0.0.7]** for the disclosure of this paragraph see [0456.0.0.0] above.

**[0457.0.5.7] to [0460.0.5.7]** for the disclosure of the paragraphs [0457.0.5.7] to [0460.0.6.7] see paragraphs [0457.0.5.5] to [0460.0.5.5] above.

**[0461.0.7.7]** Example 10: Cloning SEQ ID NO: 5818 for the expression in plants

**[0462.0.0.7]** for the disclosure of this paragraph see [0462.0.0.0] above.

**[0463.0.7.7]** SEQ ID NO: 5818 is amplified by PCR as described in the protocol of the Pfu Turbo or DNA Herculase polymerase (Stratagene).

**[0464.0.5.7], [0465.0.5.7] and [0466.0.0.7]** for the disclosure of the paragraphs [0464.0.5.7], [0465.0.5.7] and [0466.0.0.7] see paragraphs [0464.0.5.5], [0465.0.5.5] and [0466.0.0.0] above.

**[0467.0.7.7]** The following primer sequences were selected for the gene SEQ ID NO: 5818:
i) forward primer (SEQ ID NO: 6426)
   ttatttttcc tgaagaccga gttttt
ii) reverse primer (SEQ ID NO: 6427)
   atgctggata aaattgttat tgcc

**[0468.0.0.7] to [0479.0.0.7]** for the disclosure of the paragraphs [0468.0.0.7] to [0479.0.0.7] see paragraphs [0468.0.0.0] to [0479.0.0.0] above.

**[0480.0.7.7]** Example 11: Generation of transgenic plants which express SEQ ID NO: 5818

**[0481.0.0.7]** for the disclosure of this paragraph see [0481.0.0.0] above.

**[0482.0.0.7] to [0513.0.0.7]** for the disclosure of the paragraphs [0482.0.0.7] to [0513.0.0.7] see paragraphs [0482.0.0.0] to [0513.0.0.0] above.

**[0514.0.7.7]** As an alternative, the amino acids can be detected advantageously via HPLC separation in ethanolic extract as described by Geigenberger et al. (Plant Cell & Environ, 19, 1996: 43-55).
The results of the different plant analyses can be seen from the table, which follows:

**Table 1**

| **ORF** | Metabolite | Method | Min | Max |
|---|---|---|---|---|
| b3256 | Stearic Acid (C18:0) | GC | 1,17 | 1,25 |
| b2699 | Stearic Acid (C18:0) | GC | 1,22 | 1,83 |
| b2095 | Stearic Acid (C18:0) | GC | 1,17 | 1,26 |
| YOR024W | Stearic Acid (C18:0) | GC | 1,16 | 1,40 |
| YBR089C-A | Stearic Acid (C18:0) | GC | 1,36 | 2,34 |
| YFR042W | Stearic Acid (C18:0) | GC | 1,19 | 1,26 |
| YIL150C | Stearic Acid (C18:0) | CG | 1,34 | 3,20 |
| YDR513W | Stearic Acid (C18:0) | GC | 1,16 | 1,51 |
| YLR010C | Stearic Acid (C18:0) | GC | 1,16 | 1,76 |
| b1093 | Stearic Acid (C18:0) | GC | 1,16 | 1,31 |
| b0161 | Stearic acid (C18:0) | GC | 1.16 | 2.29 |
| b1896 | Stearic acid (C18:0) | GC | 1.23 | 1.30 |
| b3457 | Stearic acid (C18:0) | GC | 1.18 | 1.34 |

**[0515.0.5.7]** Column 2 shows the fatty acid analyzed. Columns 4 and 5 shows the ratio of the analyzed fatty acid between the transgenic plants and the wild type; Increase of the metabolites: Max: maximal x-fold (normalised to wild type)-Min: minimal x-fold (normalised to wild type). Decrease of the metabolites: Max: maximal x-fold (normalised to wild type) (minimal decrease), Min: minimal x-fold (normalised to wild type) (maximal decrease). Column 3 indicates the analytical method.

**[0516.0.0.7] and [0517.0.5.7]** for the disclosure of the paragraphs [0516.0.0.7] and [0517.0.5.7] see paragraphs [0516.0.0.0] and [0517.0.0.0] above.

**[0518.0.0.7] to [0529.0.0.7] and [0530.0.5.7]** for the disclosure of the paragraphs [0518.0.0.7] to [0529.0.0.7] and [0530.0.5.7] see paragraphs [0518.0.0.0] to [0530.0.0.0] above.

**[0530.1.0.5] to [0530.6.0.5]** for the disclosure of the paragraphs [0530.1.0.5] to [0530.6.0.5] see paragraphs [0530.1.0.0] to [0530.6.0.0] above.

**[0531.0.0.7] to [0533.0.0.7] and [0534.0.5.7]** for the disclosure of the paragraphs [0531.0.0.7] to [0533.0.0.7] and [0534.0.5.7] see paragraphs [0531.0.0.0] to [0534.0.0.0] above.

**[0535.0.0.7] to [0537.0.0.7] and [0538.0.5.7]** for the disclosure of the paragraphs [0535.0.0.7] to [0537.0.0.7] and [0538.0.5.7] see paragraphs [0535.0.0.0] to [0538.0.0.0] above.

**[0539.0.0.7] to [0542.0.0.7] and [0543.0.5.7]** for the disclosure of the paragraphs [0539.0.0.7] to [0542.0.0.7] and [0543.0.5.7] see paragraphs [0539.0.0.0] to [0543.0.0.0] above.

**[0544.0.0.7] to [0547.0.0.7] and [0548.0.5.7] to [0552.0.0.7]** for the disclosure of the paragraphs [0544.0.0.7] to [0547.0.0.7] and [0548.0.5.7] to [0552.0.0.7] see paragraphs [0544.0.0.0] to [0552.0.0.0] above.

**[0552.1.0.7]:** Example 15: Metabolite Profiling Info from *Zea mays*
*Zea mays* plants were engineered as described in Example 14c.
The results of the different *Zea mays* plants analysed can be seen from table 2, which follows:

**Table 2**

| **ORF_NAME** | **Metabolite** | **Min** | **Max** |
|---|---|---|---|
| YIL150C | Stearic Acid (C18:0) | 1.469 | 2.241 |

Table 2 shows the increase in stearic acid in genetically modified corn plants expressing the Saccharomyces cerevisiae nucleic acid sequence YIL150c .
In one embodiment, in case the activity of the Saccaromyces cerevisiae protein YIL150C or its homologs, e.g. a "chromatin binding protein, required for S-phase (DNA synthesis) initiation or completion" or its homologs, is increased in corn plants, preferably, an increase of the fine chemical stearic acid between 46% and 124% is conferred.

**[0552.2.0.7]** for the disclosure of this paragraph see [0552.2.0.0] above.

**[0553.0.7.7]**
1. A process for the production of stearic acid, which comprises
   (a) increasing or generating the activity of a protein as indicated in Table II, columns 5 or 7, lines 72 to 81 and 460 to 462 or a functional equivalent thereof in a non-human organism, or in one or more parts thereof; and
   (b) growing the organism under conditions which permit the production of stearic acid in said organism.
2. A process for the production of stearic acid, comprising the increasing or generating in an organism or a part thereof the expression of at least one nucleic acid molecule comprising a nucleic acid molecule selected from the group consisting of:
   a) nucleic acid molecule encoding of a polypeptide as indicated in Table II, columns 5 or 7, lines 72 to 81 and 460 to 462 or a fragment thereof, which confers an increase in the amount of stearic acid in an organism or a part thereof;
   b) nucleic acid molecule comprising of a nucleic acid molecule as indicated in Table I, columns 5 or 7, lines 72 to 81 and 460 to 462;
   c) nucleic acid molecule whose sequence can be deduced from a polypeptide sequence encoded by a nucleic acid molecule of (a) or (b) as a result of the degeneracy of the genetic code and conferring an increase in the amount of stearic acid in an organism or a part thereof;
   d) nucleic acid molecule which encodes a polypeptide which has at least 50% identity with the amino acid sequence of the polypeptide encoded by the nucleic acid molecule of (a) to (c) and conferring an increase in the amount of stearic acid in an organism or a part thereof;
   e) nucleic acid molecule which hybidizes with a nucleic acid molecule of (a) to (c) under stringent hybridisation conditions and conferring an increase in the amount of stearic acid in an organism or a part thereof;
   f) nucleic acid molecule which encompasses a nucleic acid molecule which is obtained by amplifying nucleic acid molecules from a cDNA library or a genomic library using the primers or primer pairs as indicated in Table III, column 7, lines 72 to 81 and 460 to 462 and conferring an increase in the amount of stearic acid in an organism or a part thereof;
   g) nucleic acid molecule encoding a polypeptide which is isolated with the aid of monoclonal antibodies against a polypeptide encoded by one of the nucleic acid molecules of (a) to (f) and conferring an increase in the amount of stearic acid in an organism or a part thereof;
   h) nucleic acid molecule encoding a polypeptide comprising a consensus sequence as indicated in Table IV, column 7, lines 72 to 81 and 460 to 462 and conferring an increase in the amount of stearic acid in an organism or a part thereof; and
   i) nucleic acid molecule which is obtainable by screening a suitable nucleic acid library under stringent hybridization conditions with a probe comprising one of the sequences of the nucleic acid molecule of (a) to (k) or with a fragment thereof having at least 15 nt, preferably 20 nt, 30 nt, 50 nt, 100 nt, 200 nt or 500 nt of the nucleic acid molecule characterized in (a) to (k) and conferring an increase in the amount of stearic acid in an organism or a part thereof.
   or comprising a sequence which is complementary thereto.
3. The process of claim 1 or 2, comprising recovering of the free or bound stearic acid.
4. The process of any one of claim 1 to 3, comprising the following steps:
   (a) selecting an organism or a part thereof expressing a polypeptide encoded by the nucleic acid molecule characterized in claim 2;
   (b) mutagenizing the selected organism or the part thereof;
   (c) comparing the activity or the expression level of said polypeptide in the mutagenized organism or the part thereof with the activity or the expression of said polypeptide of the selected organisms or the part thereof;
   (d) selecting the mutated organisms or parts thereof, which comprise an increased activity or expression level of said polypeptide compared to the selected organism or the part thereof;
   (e) optionally, growing and cultivating the organisms or the parts thereof; and
   (f) recovering, and optionally isolating, the free or bound stearic acid produced by the selected mutated organisms or parts thereof.
5. The process of any one of claims 1 to 4, wherein the activity of said protein or the expression of said nucleic acid molecule is increased or generated transiently or stably.
6. An isolated nucleic acid molecule comprising a nucleic acid molecule selected from the group consisting of:
   a) nucleic acid molecule encoding of a polypeptide as indicated in Table II, columns 5 or 7, lines 72 to 81 and 460 to 462 or a fragment thereof, which confers an increase in the amount of stearic acid in an organism or a part thereof;
   b) nucleic acid molecule comprising of a nucleic acid molecule as indicated in Table I, columns 5 or 7, lines 72 to 81 and 460 to 462;
   c) nucleic acid molecule whose sequence can be deduced from a polypeptide sequence encoded by a nucleic acid molecule of (a) or (b) as a result of the degeneracy of the genetic code and conferring an increase in the amount of stearic acid in an organism or a part thereof;
   d) nucleic acid molecule which encodes a polypeptide which has at least 50% identity with the amino acid sequence of the polypeptide encoded by the nucleic acid molecule of (a) to (c) and conferring an increase in the amount of stearic acid in an organism or a part thereof;
   e) nucleic acid molecule which hybidizes with a nucleic acid molecule of (a) to (c) under stringent hybridisation conditions and conferring an increase in the amount of stearic acid in an organism or a part thereof;
   f) nucleic acid molecule which encompasses a nucleic acid molecule which is obtained by amplifying nucleic acid molecules from a cDNA library or a genomic library using the primers or primer pairs as indicated in Table III, column 7, lines 72 to 81 and 460 to 462 and conferring an increase in the amount of stearic acid in an organism or a part thereof;
   g) nucleic acid molecule encoding a polypeptide which is isolated with the aid of monoclonal antibodies against a polypeptide encoded by one of the nucleic acid molecules of (a) to (f) and conferring an increase in the amount of stearic acid in an organism or a part thereof;
   h) nucleic acid molecule encoding a polypeptide comprising a consensus sequence as indicated in Table IV, column 7, lines 72 to 81 and 460 to 462 and conferring an increase in the amount of stearic acid in an organism or a part thereof; and
   i) nucleic acid molecule which is obtainable by screening a suitable nucleic acid library under stringent hybridization conditions with a probe comprising one of the sequences of the nucleic acid molecule of (a) to (k) or with a fragment thereof having at least 15 nt, preferably 20 nt, 30 nt, 50 nt, 100 nt, 200 nt or 500 nt of the nucleic acid molecule characterized in (a) to (k) and conferring an increase in the amount of stearic acid in an organism or a part thereof.
   whereby the nucleic acid molecule distinguishes over the sequence as indicated in Table I A, columns 5 or 7, lines 72 to 81 and 460 to 462 by one or more nucleotides.
7. A nucleic acid construct which confers the expression of the nucleic acid molecule of claim 6, comprising one or more regulatory elements.
8. A vector comprising the nucleic acid molecule as claimed in claim 6 or the nucleic acid construct of claim 7.
9. The vector as claimed in claim 8, wherein the nucleic acid molecule is in operable linkage with regulatory sequences for the expression in a prokaryotic or eukaryotic, or in a prokaryotic and eukaryotic, host.
10. A host cell, which has been transformed stably or transiently with the vector as claimed in claim 8 or 9 or the nucleic acid molecule as claimed in claim 6 or the nucleic acid construct of claim 7 or produced as described in claim any one of claims 2 to 5.
11. The host cell of claim 10, which is a transgenic host cell.
12. The host cell of claim 10 or 11, which is a plant cell, an animal cell, a microorganism, or a yeast cell, a fungus cell, a prokaryotic cell, an eukaryotic cell or an archaebacterium.
13. A process for producing a polypeptide, wherein the polypeptide is expressed in a host cell as claimed in any one of claims 10 to 12.
14. A polypeptide produced by the process as claimed in claim 13 or encoded by the nucleic acid molecule as claimed in claim 6 whereby the polypeptide distinguishes over a sequence as indicated in Table II A, columns 5 or 7, lines 72 to 81 and 460 to 462 by one or more amino acids
15. An antibody, which binds specifically to the polypeptide as claimed in claim 14.
16. A plant tissue, propagation material, harvested material or a plant comprising the host cell as claimed in claim 12 which is plant cell or an Agrobacterium.
17. A method for screening for agonists and antagonists of the activity of a polypeptide encoded by the nucleic acid molecule of claim 6 conferring an increase in the amount of stearic acid in an organism or a part thereof comprising:
   (a) contacting cells, tissues , plants or microorganisms which express the a polypeptide encoded by the nucleic acid molecule of claim 6 conferring an increase in the amount of stearic acid in an organism or a part thereof with a candidate compound or a sample comprising a plurality of compounds under conditions which permit the expression the polypeptide;
   (b) assaying the stearic acid level or the polypeptide expression level in the cell, tissue, plant or microorganism or the media the cell, tissue, plant or microorganisms is cultured or maintained in; and
   (c) identifying a agonist or antagonist by comparing the measured stearic acid level or polypeptide expression level with a standard of stearic acid or polypeptide expression level measured in the absence of said candidate compound or a sample comprising said plurality of compounds, whereby an increased level over the standard indicates that the compound or the sample comprising said plurality of compounds is an agonist and a decreased level over the standard indicates that the compound or the sample comprising said plurality of compounds is an antagonist.
18. A process for the identification of a compound conferring increased stearic acid production in a plant or microorganism, comprising the steps:
   (a) culturing a plant cell or tissue or microorganism or maintaining a plant expressing the polypeptide encoded by the nucleic acid molecule of claim 6 conferring an increase in the amount of stearic acid in an organism or a part thereof and a readout system capable of interacting with the polypeptide under suitable conditions which permit the interaction of the polypeptide with dais readout system in the presence of a compound or a sample comprising a plurality of compounds and capable of providing a detectable signal in response to the binding of a compound to said polypeptide under conditions which permit the expression of said readout system and of the polypeptide encoded by the nucleic acid molecule of claim 6 conferring an increase in the amount of stearic acid in an organism or a part thereof;
   (b) identifying if the compound is an effective agonist by detecting the presence or absence or increase of a signal produced by said readout system.
19. A method for the identification of a gene product conferring an increase in stearic acid production in a cell, comprising the following steps:
   (a) contacting the nucleic acid molecules of a sample, which can contain a candidate gene encoding a gene product conferring an increase in stearic acid after expression with the nucleic acid molecule of claim 6;
   (b) identifying the nucleic acid molecules, which hybridise under relaxed stringent conditions with the nucleic acid molecule of claim 6;
   (c) introducing the candidate nucleic acid molecules in host cells appropriate for producing stearic acid;
   (d) expressing the identified nucleic acid molecules in the host cells;
   (e) assaying the stearic acid level in the host cells; and
   (f) identifying nucleic acid molecule and its gene product which expression confers an increase in the stearic acid level in the host cell in the host cell after expression compared to the wild type.
20. A method for the identification of a gene product conferring an increase in stearic acid production in a cell, comprising the following steps:
   (a) identifiying in a data bank nucleic acid molecules of an organism; which can contain a candidate gene encoding a gene product conferring an increase in the stearic acid amount or level in an organism or a part thereof after expression, and which are at least 20% homolog to the nucleic acid molecule of claim 6;
   (b) introducing the candidate nucleic acid molecules in host cells appropriate for producing stearic acid;
   (c) expressing the identified nucleic acid molecules in the host cells;
   (d) assaying the stearic acid level in the host cells; and
   (e) identifying nucleic acid molecule and its gene product which expression confers an increase in the stearic acid level in the host cell after expression compared to the wild type.
21. A method for the production of an agricultural composition comprising the steps of the method of any one of claims 17 to 20 and formulating the compound identified in any one of claims 17 to 20 in a form acceptable for an application in agriculture.
22. A composition comprising the nucleic acid molecule of claim 6, the polypeptide of claim 14, the nucleic acid construct of claim 7, the vector of any one of claims 8 or 9, an antagonist or agonist identified according to claim 17, the compound of claim 18, the gene product of claim 19 or 20, the antibody of claim 15, and optionally an agricultural acceptable carrier.
23. Use of the nucleic acid molecule as claimed in claim 6 for the identification of a nucleic acid molecule conferring an increase of stearic acid after expression.
24. Use of the polypeptide of claim 14 or the nucleic acid construct claim 7 or the gene product identified according to the method of claim 19 or 20 for identifying compounds capable of conferring a modulation of stearic acid levels in an organism.
25. Food or feed composition comprising the nucleic acid molecule of claim 6, the polypeptide of claim 14, the nucleic acid construct of claim 7, the vector of claim 8 or 9, the antagonist or agonist identified according to claim 17, the antibody of claim 15, the plant or plant tissue of claim 16, the harvested material of claim 16, the host cell of claim 10 to 12 or the gene product identified according to the method of claim 19 or 20.
26. Use of the nucleic acid molecule of claim 6, the polypeptide of claim 14, the nucleic acid construct of claim 7, the vector of claim 8 or 9, the antagonist or agonist identified according to claim 17, the antibody of claim 15, the plant or plant tissue of claim 16, the host cell of claim 10 to 12 or the gene product identified according to the method of claim 19 or 20 for the protection of a plant against a stearic acid synthesis inhibiting herbicide.

**[0554.0.0.7] Abstract: see [0554.0.0.0]**
Process for the production of fine chemicals

**[0000.0.0.8]** In a further embodiment, the present invention relates to a further process for the production of fine chemicals as defined below and the corresponding embodiments as described herein as follows.

**[0001.0.0.8] and [0002.0.7.8]** for the disclosure of the paragraphs [0001.0.0.8] and [0002.0.7.8] see paragraphs [0001.0.0.0] and [0002.0.7.7] above.

**[0003.0.8.8]** Palmitic acid is a major component for manufacturing of soaps, lubricating oils and waterproofing materials. Furhermore it is used for the synthesis of metallic palmitates. Additional applications are as food additive and in the synthesis of food-grade additives; as a constituent of cosmetic formulations. Palmitic acid is a major component of many natural fats and oils in the form of a glyceryl ester, e.g. palm oil, and in most commercial- grade stearic acid products.

**[0004.0.7.8] and [0005.0.5.8]** for the disclosure of the paragraphs [0004.0.7.8] and [0005.0.5.8] see paragraphs [0004.0.7.7] and [0005.0.5.5] above.

**[0006.0.8.8]** Palmitic acid is as mentioned above the major fat in meat and dairy products.

**[0007.0.8.8]** Further uses or palmitic acid are as food ingredients raw material for emulsifiers or personal care emulsifier for facial creams and lotions. Palmitic acid is also used in shaving cream formulations, waxes or fruit wax formulations.

**[0008.0.8.8]** Palmitic acid is also used in shaving cream formulations, waxes or fruit wax formulations.

**[0009.0.7.8] to [0012.0.7.8]** for the disclosure of the paragraphs [0009.0.7.8] to [0012.0.7.8] see paragraphs [0009.0.7.7] and [0012.0.7.7] above.

**[0013.0.0.8]** for the disclosure of this paragraph see [0013.0.0.0] above.

**[0014.0.8.8]** Accordingly, in a first embodiment, the invention relates to a process for the production of a fine chemical, whereby the fine chemical is palmitic acid or tryglycerides, lipids, oils or fats containing palmitic acid. Accordingly, in the present invention, the term "the fine chemical" as used herein relates to "palmitic acid and/or tryglycerides, lipids, oils and/or fats containing palmitic acid". Further, the term "the fine chemicals" as used herein also relates to fine chemicals comprising palmitic acid and/or triglycerides, lipids, oils and/or fats containing palmitic acid.

**[0015.0.8.8]** In one embodiment, the term "the fine chemical" means palmitic acid and/or tryglycerides, lipids, oils and/or fats containing palmitic acid. Throughout the specification the term "the fine chemical" means palmitic acid and/or tryglycerides, lipids, oils and/or fats containing palmitic acid, palmitic acid and its salts, ester, thioester or palmitic acid in free form or bound to other compounds such as triglycerides, glycolipids, phospholipids etc. In a preferred embodiment, the term "the fine chemical" means palmitic acid, in free form or its salts or bound to triglycerides. Triglycerides, lipids, oils, fats or lipid mixture thereof shall mean any triglyceride, lipid, oil and/or fat containing any bound or free palmitic acid for example sphingolipids, phosphoglycerides, lipids, glycolipids such as glycosphingolipids, phospholipids such as phosphatidylethanolamine, phosphatidylcholine, phosphatidylserine, phosphatidylglycerol, phosphatidylinositol or diphosphatidylglycerol, or as monoacylglyceride, diacylglyceride or triacylglyceride or other fatty acid esters such as acetyl-Coenzym A thioester, which contain further saturated or unsaturated fatty acids in the fatty acid molecule.
In one embodiment, the term "the fine chemical" and the term "the respective fine chemical" mean at least one chemical compound with an activity of the above mentioned fine chemical.

**[0016.0.8.8]** Accordingly, the present invention relates to a process comprising
(a) increasing or generating the activity of a YDR513W, YDR447C, YBR089C-A, b3256, YGR126W, YPL099C, b0399, b0849, b3457, b3578, b3644 and/or b4129 protein(s) or of a protein having the sequence of a polypeptide encoded by a nucleic acid molecule indicated in Table I, columns 5 or 7, lines 82 to 88 and 463 to 467 in a non-human organism in one or more parts thereof and
(b) growing the organism under conditions which permit the production of the fine chemical, thus, palmitic acid or fine chemicals comprising palmitic acid, in said organism.
Accordingly, the present invention relates to a process for the production of a fine chemical comprising
(a) increasing or generating the activity of one or more proteins having the activity of a protein indicated in Table II, column 3, lines 82 to 88 and 463 to 467 or having the sequence of a polypeptide encoded by a nucleic acid molecule indicated in Table I, column 5 or 7, lines 82 to 88 and 463 to 467, in a non-human organism in one or more parts thereof and
(b) growing the organism under conditions which permit the production of the fine chemical, in particular palmitic acid.

**[0017.0.0.8] and [0018.0.0.8]** for the disclosure of the paragraphs [0017.0.0.8] and [0018.0.0.8] see paragraphs [0017.0.0.0] and [0018.0.0.0] above.

**[0019.0.8.8]** Advantageously the process for the production of the fine chemical leads to an enhanced production of the fine chemical. The terms "enhanced" or "increase" mean at least a 10%, 20%, 30%, 40% or 50%, preferably at least 60%, 70%, 80%, 90% or 100%, more preferably 150%, 200%, 300%, 400% or 500% higher production of the fine chemical in comparison to the reference as defined below, e.g. that means in comparison to an organism without the aforementioned modification of the activity of a protein having the activity of a protein indicated in Table II, column 3, lines 82 to 88 and 463 to 467 or encoded by nucleic acid molecule indicated in Table I, columns 5 or 7, lines 82 to 88 and 463 to 467.

**[0020.0.8.8]** Surprisingly it was found, that the transgenic expression of at least one of the Saccaromyces cerevisiae protein(s) indicated in Table II, Column 3, lines 82 to 84 and 86 and 87, and/or the Escherichia coli K12 protein(s) indicated in Table II, Column 3, lines 85 and 88 and 463 to 467 in Arabidopsis thaliana conferred an increase in the palmitic acid (or fine chemical) content of the transformed plants.

**[0021.0.0.8]** for the disclosure of this paragraph see [0021.0.0.0] above.

**[0022.0.8.8]** The sequence of b3256 from Escherichia coli K12 has been published in Blattner et al., Science 277(5331), 1453-1474, 1997, and its activity is being defined as acetyl CoA carboxylase. Accordingly, in one embodiment, the process of the present invention comprises the use of a acetyl CoA carboxylase from E. coli or its homolog, e.g. as shown herein, for the production of the fine chemical, meaning of palmitic acid and/or tryglycerides, lipids, oils and/or fats containing palmitic acid, in particular for increasing the amount of palmitic acid and/or tryglycerides, lipids, oils and/or fats containing palmitic acid, preferably palmitic acid in free or bound form in an organism or a part thereof, as mentioned. In one embodiment, in the process of the present invention the activity of a acetyl CoA carboxylase is increased or generated, e.g. from E. coli or a homolog thereof.
The sequence of b0399 from Escherichia coli K12 has been published in Blattner et al., Science 277(5331), 1453-1474, 1997, and its activity is being defined as a positive response regulator for the pho regulon. Accordingly, in one embodiment, the process of the present invention comprises the use of a positive response regulator for the pho regulon from E. coli or its homolog, e.g. as shown herein, for the production of the fine chemical, meaning of palmitic acid and/or tryglycerides, lipids, oils and/or fats containing palmitic acid, in particular for increasing the amount of palmitic and/or tryglycerides, lipids, oils and/or fats containing palmitic acid, preferably palmitic acid in free or bound form in an organism or a part thereof, as mentioned. In one embodiment, in the process of the present invention the activity of a positive response regulator for the pho regulon is increased or generated, e.g. from E. coli or a homolog thereof.
The sequence of YDR513W from Saccharomyces cerevisiae has been published in Jacq et al., Nature 387 (6632 Suppl), 75-78 (1997) and in Goffeau et al., Science 274 (5287), 546-547, 1996 and its cellular activity has characterized as glutaredoxin (thioltransferase, glutathione reductase). Accordingly, in one embodiment, the process of the present invention comprises the use of a glutaredoxin, from Saccharomyces cerevisiae or its homolog, e.g. as shown herein, for the production of the fine chemical, meaning of palmitic acid and/or tryglycerides, lipids, oils and/or fats containing palmitic acid, in particular for increasing the amount of palmitic acid and/or tryglycerides, lipids, oils and/or fats containing palmitic acid, preferably palmitic acid in free or bound form in an organism or a part thereof, as mentioned. In one embodiment, in the process of the present invention the activity of a glutaredoxin is increased or generated, e.g. from Saccharomyces cerevisiae or a homolog thereof.
The sequence of YDR447C from Saccharomyces cerevisiae has been published in Jacq et al., Nature 387 (6632 Suppl), 75-78 (1997) and in Goffeau et al., Science 274 (5287), 546-547, 1996 and its cellular activity has been characterized as a ribosomal protein 51 (rp51) of the small (40s) subunit; nearly identical to rps17Ap and has similarity to rat S17 ribosomal protein; rps17bp. Accordingly, in one embodiment, the process of the present invention comprises the use of a ribosomal protein 51 (rp51) of the small (40s) subunit, from Saccharomyces cerevisiae or its homolog, e.g. as shown herein, for the production of the fine chemical, meaning of palmitic acid and/or tryglycerides, lipids, oils and/or fats containing palmitic acid, in particular for increasing the amount of palmitic acid and/or tryglycerides, lipids, oils and/or fats containing palmitic acid, preferably palmitic acid in free or bound form in an organism or a part thereof, as mentioned. In one embodiment, in the process of the present invention the activity of a ribosomal protein 51 (rp51) of the small (40s) subunit is increased or generated, e.g. from Saccharomyces cerevisiae or a homolog thereof.
The sequence of YBR089C-A from Saccharomyces cerevisiae has been published in Feldmann et al., EMBO J., 13 (24), 5795-5809 (1994) and Goffeau et al., Science 274 (5287), 546-547, 1996, and its cellular activity has not been characterized yet. It shows homology to mammalian high mobility group proteins 1 and 2. Its function may be redundantly with the highly homologous gene NHP6A. Furthermore it shows homology to the high-mobility group non-histone chromatin protein Nhp6bp. Accordingly, in one embodiment, the process of the present invention comprises the use of a YBR089C-A activity from Saccharomyces cerevisiae or its homolog, e.g. as shown herein, for the production of the fine chemical, meaning of palmitic acid and/or tryglycerides, lipids, oils and/or fats containing palmitic acid, in particular for increasing the amount of palmitic acid and/or tryglycerides, lipids, oils and/or fats containing palmitic acid, preferably palmitic acid in free or bound form in an organism or a part thereof, as mentioned. In one embodiment, in the process of the present invention the activity of a YBR089C-A protein is increased or generated, e.g. from Saccharomyces cerevisiae or a homolog thereof.
The sequence of YGR126W from Saccharomyces cerevisiae has been published in Tettelin et al., Nature 387 (6632 Suppl), 81-84 (1997) and Goffeau et al., Science 274 (5287), 546-547, 1996, and its cellular activity has not been characterized yet. It seams to be a "putative open reading frame ". Accordingly, in one embodiment, the process of the present invention comprises the use of a YGR126W activity from Saccharomyces cerevisiae or its homolog, e.g. as shown herein, for the production of the fine chemical, meaning of palmitic acid and/or tryglycerides, lipids, oils and/or fats containing palmitic acid, in particular for increasing the amount of palmitic acid and/or tryglycerides, lipids, oils and/or fats containing palmitic acid, preferably palmitic acid in free or bound form in an organism or a part thereof, as mentioned. In one embodiment, in the process of the present invention the activity of a YGR126W protein is increased or generated, e.g. from Saccharomyces cerevisiae or a homolog thereof.
The sequence of YPL099C from Saccharomyces cerevisiae has been published in Bussey et al., Nature 387 (6632 Suppl), 103-105 (1997) and in Goffeau et al., Science 274 (5287), 546-547, 1996 and its cellular activity has not been characterized yet. "The authentic, non-tagged protein was localized to the mitochondria". Accordingly, in one embodiment, the process of the present invention comprises the use of YPL099C, from Saccharomyces cerevisiae or its homolog, e.g. as shown herein, for the production of the fine chemical, meaning of palmitic acid and/or tryglycerides, lipids, oils and/or fats containing palmitic acid, in particular for increasing the amount of palmitic acid and/or tryglycerides, lipids, oils and/or fats containing palmitic acid, preferably palmitic acid in free or bound form in an organism or a part thereof, as mentioned. In one embodiment, in the process of the present invention the activity of a YPL099C protein is increased or generated, e.g. from Saccharomyces cerevisiae or a homolog thereof.
The sequence of b0849 (Accession number NP_415370) from Escherichia coli K12 has been published in Blattner et al., Science 277 (5331), 1453-1474, 1997, and its activity is being defined as a glutaredoxin 1 redox coenzyme for glutathione-dependent ribonucleotide reductase. Accordingly, in one embodiment, the process of the present invention comprises the use of a glutaredoxin 1 redox coenzyme for glutathione-dependent ribonucleotide reductase protein from E. coli or its homolog, e.g. as shown herein, for the production of the fine chemical, meaning of palmitic acid and/or tryglycerides, lipids, oils and/or fats containing palmitic acid, in particular for increasing the amount of palmitic acid and/or tryglycerides, lipids, oils and/or fats containing palmitic acid, preferably palmitic acid in free or bound form in an organism or a part thereof, as mentioned. In one embodiment, in the process of the present invention the activity of a glutaredoxin 1 redox coenzyme for glutathione-dependent ribonucleotide reductase protein is increased or generated, e.g. from E. coli or a homolog thereof.
The sequence of b3457 (Accession number NP_417914) from Escherichia coli K12 has been published in Blattner et al., Science 277 (5331), 1453-1474, 1997, and its activity is being defined as a high-affinity branched-chain amino acid transport protein (ABC superfamily). Accordingly, in one embodiment, the process of the present invention comprises the use of a high-affinity branched-chain amino acid transport protein from E. coli or its homolog, e.g. as shown herein, for the production of the fine chemical, meaning of palmitic acid and/or tryglycerides, lipids, oils and/or fats containing palmitic acid, in particular for increasing the amount of palmitic acid and/or tryglycerides, lipids, oils and/or fats containing palmitic acid, preferably palmitic acid in free or bound form in an organism or a part thereof, as mentioned. In one embodiment, in the process of the present invention the activity of a high-affinity branched-chain amino acid transport protein is increased or generated, e.g. from E. coli or a homolog thereof.
The sequence of b3578 (Accession number YP_026232) from Escherichia coli K12 has been published in Blattner et al., Science 277(5331), 1453-1474, 1997, and its activity is being defined as putative component of transport system. Accordingly, in one embodiment, the process of the present invention comprises the use of a putative component of transport system from E. coli or its homolog, e.g. as shown herein, for the production of the fine chemical, meaning of palmitic acid and/or tryglycerides, lipids, oils and/or fats containing palmitic acid, in particular for increasing the amount of palmitic acid and/or tryglycerides, lipids, oils and/or fats containing palmitic acid, preferably palmitic acid in free or bound form in an organism or a part thereof, as mentioned. In one embodiment, in the process of the present invention the activity of a putative component of transport system is increased or generated, e.g. from E. coli or a homolog thereof.
The sequence of b3644 (Accession number NP_418101) from Escherichia coli K12 has been published in Blattner et al., Science 277 (5331), 1453-1474, 1997, and its activity is being defined as an uncharacterized stress-induced protein. Accordingly, in one embodiment, the process of the present invention comprises the use of an uncharacterized stress-induced protein from E. coli or its homolog, e.g. as shown herein, for the production of the fine chemical, meaning of palmitic acid and/or tryglycerides, lipids, oils and/or fats containing palmitic acid, in particular for increasing the amount of palmitic acid and/or tryglycerides, lipids, oils and/or fats containing palmitic acid, preferably palmitic acid in free or bound form in an organism or a part thereof, as mentioned. In one embodiment, in the process of the present invention the activity of an uncharacterized stress-induced protein is increased or generated, e.g. from E. coli or a homolog thereof.
The sequence of b4129 (Accession number NP_418553) from Escherichia coli K12 has been published in Blattner et al., Science 277 (5331), 1453-1474, 1997, and its activity is being defined as a lysine tRNA synthetase. Accordingly, in one embodiment, the process of the present invention comprises the use of a lysine tRNA synthetase protein from E. coli or its homolog, e.g. as shown herein, for the production of the fine chemical, meaning of palmitic acid and/or tryglycerides, lipids, oils and/or fats containing palmitic acid, in particular for increasing the amount of palmitic acid and/or tryglycerides, lipids, oils and/or fats containing palmitic acid, preferably palmitic acid in free or bound form in an organism or a part thereof, as mentioned. In one embodiment, in the process of the present invention the activity of a lysine tRNA synthetase protein is increased or generated, e.g. from E. coli or a homolog thereof.

**[0023.0.8.8]** Homologues ( = homologs) of the present gene products can be derived from any organisms as long as the homologue confers the herein mentioned activity, in particular, confers an increase in the fine chemical amount or content. Further, in the present invention, the term "homologue" relates to the sequence of an organism having the highest sequence homology to the herein mentioned or listed sequences of all expressed sequences of said organism.
However, the person skilled in the art knows, that, preferably, the homologue has said fine-chemical increasing activity and, if known, the same biological function or activity in the organism as at least one of the protein(s) indicated in Table II, Column 3, lines 82 to 88 and 463 to 467, e.g. having the sequence of a polypeptide encoded by a nucleic acid molecule comprising the sequence indicated in indicated in Table I, Column 5 or 7, lines 82 to 88 and 463 to 467.
In one embodiment, the homolog of any one of the polypeptides indicated in Table II, lines 82 to 84 and 86 and 87 is a homolog having the same or a similar acitivity, in particular an increase of activity confers an increase in the content of the fine chemical in the organsims and being derived from an Eukaryot. In one embodiment, the homolog of a polypeptide indicated in Table II, column 3, lines 85 and 88 and 463 to 467 is a homolog having the same or a similar acitivity, in particular an increase of activity confers an increase in the content of the fine chemical in the organisms or part thereof, and being derived from bacteria. In one embodiment, the homolog of a polypeptide indicated in Table II, column 3, lines 82 to 84 and 86 and 87 is a homolog having the same or a similar acitivity, in particular an increase of activity confers an increase in the content of the fine chemical in an organisms or part thereof, and being derived from Fungi. In one embodiment, the homolog of a polypeptide indicated in Table II, column 3, lines 85 and 88 and 463 to 467 is a homolog having the same or a similar acitivity, in particular an increase of activity confers an increase in the content of the fine chemical in the organsims or part thereof and being derived from Proteobacteria. In one embodiment, the homolog of a polypeptide indicated in Table II, column 3, lines 82 to 84 and 86 and 87 is a homolog having the same or a similar acitivity, in particular an increase of activity confers an increase in the content of the fine chemical in the organsims or a part thereof and being derived from Ascomycota. In one embodiment, the homolog of a polypeptide indicated in Table II, column 3, lines 85 and 88 and 463 to 467 is a homolog having the same or a similar acitivity, in particular an increase of activity confers an increase in the content of the fine chemical in the organisms or part thereof, and being derived from Gammaproteobacteria. In one embodiment, the homolog of a polypeptide polypeptide indicated in Table II, column 3, lines 82 to 84 and 86 and 87 is a homolog having the same or a similar acitivity, in particular an increase of activity confers an increase in the content of the fine chemical in the organisms or part thereof, and being derived from Saccharomycotina. In one embodiment, the homolog of a polypeptide indicated in Table II, column 3, lines 85 and 88 and 463 to 467 is a homolog having the samie or a similar acitivity, in particular an increase of activity confers an increase in the content of the fine chemical in the organisms or part thereof, and being derived from Enterobacteriales. In one embodiment, the homolog of a polypeptide indicated in Table II, column 3, lines 82 to 84 and 86 and 87 is a homolog having the samie or a similar acitivity, in particular an increase of activity confers an increase in the content of the fine chemical in the organisms or a part thereof, and being derived from Saccharomycetes. In one embodiment, the homolog of the a polypeptide indicated in Table II, column 3, lines 85 and 88 and 463 to 467 is a homolog having the samie or a similar acitivity, in particular an increase of activity confers an increase in the content of the fine chemical in the organisms or part thereof, and being derived from Enterobacteriaceae. In one embodiment, the homolog of a polypeptide indicated in Table II, column 3, lines 82 to 84 and 86 and 87 is a homolog having the same or a similar acitivity, in particular an increase of activity confers an increase in the content of the fine chemical in the organisms, and being derived from Saccharomycetales. In one embodiment, the homolog of a polypeptide indicated in Table II, column 3, lines 85 and 88 and 463 to 467 is a homolog having the same or a similar acitivity, in particular an increase of activity confers an increase in the content of the fine chemical in the organisms or a part thereof, and being derived from Escherichia. In one embodiment, the homolog of a polypeptide indicated in Table II, column 3, lines 82 to 84 and 86 and 87 is a homolog having the same or a similar acitivity, in particular an increase of activity confers an increase in the content of the fine chemical in the organisms or a part thereof, and being derived from Saccharomycetaceae. In one embodiment, the homolog of a polypeptide indicated in Table II, column 3, line 82 to 84 and 86 and 87 is a homolog having the same or a similar acitivity, in particular an increase of activity confers an increase in the content of the fine chemical in the organisms or a part thereof, and being derived from Saccharomycetes.

**[0023.1.0.8] and [0024.0.0.8]** for the disclosure of the paragraphs [0023.1.0.8] and [0024.0.0.8] see [0023.1.0.0] and [0024.0.0.0] above.

**[0025.0.8.8]** In accordance with the invention, a protein or polypeptide has the "activity of a protein as indicated in Table II, column 3, lines 82 to 88 and 463 to 467" if its *de novo* activity, or its increased expression directly or indirectly leads to an increased palmitic acid and/or tryglycerides, lipids, oils and/or fats containing palmitic acid level in the organism or a part thereof, preferably in a cell of said organism and the protein has the above mentioned activities of a protein as indicated in Table II, column 3, lines 82 to 88 and 463 to 467. Throughout the specification the activity or preferably the biological activity of such a protein or polypeptide or an nucleic acid molecule or sequence encoding such protein or polypeptide is identical or similar if it still has the biological or enzymatic activity of a protein as indicated in Table II, column 3, lines 82 to 88 and 463 to 467, or which has at least 10% of the original enzymatic activity, preferably 20%, particularly preferably 30%, most particularly preferably 40% in comparison to a protein of Saccharomyces cerevisiae as indicated in Table II, column 3, lines 82 to 84 and 86 and 87 and/or a protein of E. coli K12 as indicated in Table II, column 3, lines 85 and 88 and 463 to 467.

**[0025.1.0.8] and [0025.2.0.8]** for the disclosure of the paragraphs [0025.1.0.8] and [0025.2.0.8] see paragraphs [0025.1.0.0] and [0025.2.0.0] above.

**[0026.0.0.8] to [0033.0.0.8]** for the disclosure of the paragraphs [0026.0.0.8] to [0033.0.0.8] see paragraphs [0026.0.0.0] to [0033.0.0.0] above.

**[0034.0.8.8]** Preferably, the reference, control or wild type differs form the subject of the present invention only in the cellular activity of the polypeptide of the invention or the polypeptide used in the method of the invention, e.g. as result of an increase in the level of the nucleic acid molecule of the present invention or an increase of the specific activity of the polypeptide of the invention or the polypeptide used in the method of the invention. E.g., it differs by or in the expression level or activity of an protein having the activity of a protein as indicated in Table II, column 3, lines 82 to 88 and 463 to 467 or being encoded by a nucleic acid molecule indicated in Table I, column 5, lines 82 to 88 and 463 to 467 or its homologs, e.g. as indicated in Table I, column 7, lines 82 to 88 and 463 to 467, its biochemical or genetical causes and therefore shows the increased amount of the fine chemical.

**[0035.0.0.8] to [0038.0.0.8] and [0039.0.5.8]** for the disclosure of the paragraphs [0035.0.0.8] to [0038.0.0.8] and [0039.0.5.8] see paragraphs [0035.0.0.0] to [0039.0.0.0] above.

**[0040.0.0.8] to [0044.0.0.8]** for the disclosure of the paragraphs [0040.0.0.8] to [0044.0.0.8] see paragraphs [0035.0.0.0] and [0044.0.0.0] above.

**[0045.0.8.8]** In one embodiment, in case the activity of the Escherichia coli K12 protein b3256 or its homologs e.g. a acetyl CoA carboxylase protein, e.g. as indicated in Table II, columns 5 or 7, line 85, is increased, preferably, in one embodiment the increase of the fine chemical, preferably of palmitic acid between 15% and 17% or more is conferred.
In case the activity of the Escherichia coli K12 protein b0399 or its homologs e.g. a positive response regulator for the pho regulon, e.g. as indicated in Table II, columns 5 or 7, line 88, is increased, preferably, in one embodiment the increase of the fine chemical, preferably of palmitic acid between 20% and 27% or more is conferred.
In one embodiment, in case the activity of the Saccharomyces cerevisiae protein YDR513W or its homologs, e.g. a "glutaredoxin", e.g. as indicated in Table II, columns 5 or 7, line 82, is increased, preferably, in one embodiment an increase of the fine chemical, preferably of palmitic acid between 17% and 74% or more is conferred.
In one embodiment, in case the activity of the Saccharomyces cerevisiae protein YDR447C or its homologs, e.g. a "ribosomal protein 51 (rp51) of the small (40s) subunit", e.g. as indicated in Table II, columns 5 or 7, line 83, is increased, preferably, in one embodiment an increase of the fine chemical, preferably of palmitic acid between 19% and 181% or more is conferred.
In case the activity of the Saccharomyces cerevisiae protein YBR089C-A or its homologs, e.g. a "uncharacterized protein YBR089C-A", which seams to have "homology to mammalian high mobility group proteins 1 and 2", e.g. as indicated in Table II, columns 5 or 7, line 84, is increased, preferably, in one embodiment an increase of the fine chemical, preferably of palmitic acid between 55% and 95% or more is conferred.
In one embodiment, in case the activity of the Saccharomyces cerevisiae protein YGR126W or its homologs, e.g. a "uncharacterized protein YGR126W", e.g. as indicated in Table II, columns 5 or 7, line 86, is increased, preferably, in one embodiment an increase of the fine chemical, preferably of palmitic acid between 34% and 167% or more is conferred.
In one embodiment, in case the activity of the Saccaromyces cerevisiae protein YPL099C or its homologs e.g. a "uncharacterized protein YPL099C", e.g. as indicated in Table II, columns 5 or 7, line 87, is increased, preferably, in one embodment the increase of the fine chemical, preferably of of palmitic acid between 21 % and 55% or more is conferred.
In one embodiment, in case the activity of the Escherichia coli K12 protein b0849 or its homologs e.g. a glutaredoxin 1 redox coenzyme for glutathione-dependent ribonucleotide reductase protein, e.g. as indicated in Table II, columns 5 or 7, line 463, is increased, preferably, in one embodiment the increase of the fine chemical, preferably of palmitic acid between 17% and 30% or more is conferred.
In one embodiment, in case the activity of the Escherichia coli K12 protein b3457 or its homologs e.g. a high-affinity branched-chain amino acid transport protein (ABC superfamily), e.g. as indicated in Table II, columns 5 or 7, line 464, is increased, preferably, in one embodiment the increase of the fine chemical, preferably of palmitic acid between 16% and 36% or more is conferred.
In one embodiment, in case the activity of the Escherichia coli K12 protein b3578 or its homologs e.g. a putative component of transport system, e.g. as indicated in Table II, columns 5 or 7, line 465, is increased, preferably, in one embodiment the increase of the fine chemical, preferably of palmitic acid between 16% and 27% or more is conferred.
In one embodiment, in case the activity of the Escherichia coli K12 protein b3644 or its homologs e.g. an uncharacterized stress-induced protein, e.g. as indicated in Table II, columns 5 or 7, line 466, is increased, preferably, in one embodiment the increase of the fine chemical, preferably of palmitic acid between 16% and 42% or more is conferred.
In one embodiment, in case the activity of the Escherichia coli K12 protein b4129 or its homologs e.g. a lysine tRNA synthetase, e.g. as indicated in Table II, columns 5 or 7, line 467, is increased, preferably, in one embodiment the increase of the fine chemical, preferably of palmitic acid between 33% and 36% or more is conferred.

**[0046.0.8.8]** In case the activity of the Escherichia coli K12 protein b3256 or its homologs e.g. a acetyl CoA carboxylase protein, is increased, preferably an increase of the fine chemical and of tryglycerides, lipids, oils and/or fats containing palmitic acid is conferred.
In case the activity of the Escherichia coli K12 protein b0399 or its homologs e.g. a positive response regulator for the pho regulon protein, is increased, preferably an increase of the fine chemical and of tryglycerides, lipids, oils and/or fats containing palmitic acid is conferred.
In case the activity of the Saccharomyces cerevisiae protein YDR513W or its homologs, e.g. a "glutaredoxin protein" is increased, preferably, in one embodiment an increase of the fine chemical, preferably an increase of the fine chemical and of tryglycerides, lipids, oils and/or fats containing palmitic acid is conferred.
In case the activity of the Saccharomyces cerevisiae protein YDR447C or its homologs, e.g. a "ribosomal protein 51 (rp51) of the small (40s) subunit protein", which is required for cell viability" is increased, preferably, in one embodiment an increase of the fine chemical, preferably an increase of the fine chemical and of tryglycerides, lipids, oils and/or fats containing palmitic acid is conferred.
In case the activity of the Saccharomyces cerevisiae protein YBR089C-A or its homologs, e.g. a "uncharacterized protein YBR089C-A", which seams to have "homology to mammalian high mobility group proteins 1 and 2" is increased, preferably, an increase of the fine chemical and of tryglycerides, lipids, oils and/or fats containing palmitic acid is conferred.
In case the activity of the Saccharomyces cerevisiae protein YGR126W or its homologs, e.g. a "uncharacterized protein YGR126W" is increased, preferably, in one embodiment an increase of the fine chemical, preferably an increase of the fine chemical and of tryglycerides, lipids, oils and/or fats containing palmitic acid is conferred.
In case the activity of the Saccaromyces cerevisiae protein YPL099C or its homologs e.g. a "uncharacterized protein YPL099C protein" is increased, preferably an increase of the fine chemical and of tryglycerides, lipids, oils and/or fats containing palmitic acid is conferred.
In one embodiment, in case the activity of the Escherichia coli K12 protein b0849 or its homologs e.g. a glutaredoxin 1 redox coenzyme for glutathione-dependent ribonucleotide reductase protein is increased, preferably an increase of the fine chemical and of tryglycerides, lipids, oils and/or fats containing palmitic acid is conferred.
In one embodiment, in case the activity of the Escherichia coli K12 protein b3457 or its homologs e.g. a high-affinity branched-chain amino acid transport protein (ABC superfamily) is increased, preferably an increase of the fine chemical and of tryglycerides, lipids, oils and/or fats containing palmitic acid is conferred.
In one embodiment, in case the activity of the Escherichia coli K12 protein b3578 or its homologs e.g. a putative component of transport system is increased, preferably an increase of the fine chemical and of tryglycerides, lipids, oils and/or fats containing palmitic acid is conferred.
In one embodiment, in case the activity of the Escherichia coli K12 protein b3644 or its homologs e.g. an uncharacterized stress-induced protein is increased, preferably an increase of the fine chemical and of tryglycerides, lipids, oils and/or fats containing palmitic acid is conferred.
In one embodiment, in case the activity of the Escherichia coli K12 protein b4129 or its homologs e.g. a lysine tRNA synthetase is increased, preferably an increase of the fine chemical and of tryglycerides, lipids, oils and/or fats containing palmitic acid is conferred.

**[0047.0.0.8] to [0048.0.0.8]** for the disclosure of the paragraphs [0047.0.0.8] and [0048.0.0.8] see paragraphs [0047.0.0.0] and [0048.0.0.0] above.

**[0049.0.8.8]** A protein having an activity conferring an increase in the amount or level of the respective fine chemical preferably has the structure of the polypeptide described herein, in particular a polypeptide comprising a consensus sequence as indicated in Table IV, column 7, lines 82 to 88 and 463 to 467 or of the polypeptide as shown in the amino acid sequences as disclosed in table II, columns 5 and 7, lines 82 to 88 and 463 to 467 or the functional homologues thereof as described herein, or is encoded by the nucleic acid molecule characterized herein or the nucleic acid molecule according to the invention, for example by the nucleic acid molecule as shown in table I, columns 5 and 7, lines 82 to 88 and 463 to 467 or its herein described functional homologues and has the herein mentioned activity.

**[0050.0.8.8]** For the purposes of the present invention, the term "palmitic acid" also encompasses the corresponding salts, such as, for example, the potassium or sodium salts of palmitic acid or the salts of palmitic acid with amines such as diethylamine.

**[0051.0.5.8] and [0052.0.0.8]** for the disclosure of the paragraphs [0051.0.5.8] and [0052.0.0.8] see paragraphs [0051.0.0.0] and [0052.0.0.0] above.

**[0053.0.8.8]** In one embodiment, the process of the present invention comprises one or more of the following steps
a) stabilizing a protein conferring the increased expression of a protein encoded by the nucleic acid molecule of the invention or of the polypeptid of the invention or the nucleic acid molecule or the polypeptide used in the method of the invention, e.g. of a polypeptide having an activity of a protein as indicated in Table II, column 3, lines 82 to 88 and 463 to 467 or its homolog's activity, e.g. as indicated in Table II, column 7, lines 82 to 88 and 463 to 467, having herein-mentioned the fine chemical increasing activity;
b) stabilizing a mRNA conferring the increased expression of a protein encoded by the nucleic acid molecule of the invention or the nucleic acid molecule used in the method of the invention, e.g. of a polypeptide having an activity of a protein as indicated in Table II, column 3, lines 82 to 88 and 463 to 467 or its homologs activity, e.g. as indicated in Table II, column 7, lines 82 to 88 and 463 to 467 or of a mRNA encoding the polypeptide of the present invention having herein-mentioned palmitic acid increasing activity;
c) increasing the specific activity of a protein conferring the increased expression of a protein encoded by the nucleic acid molecule of the invention or of the polypeptide of the present invention or the nucleic acid molecule or the polypeptide used in the method of the invention, having herein-mentioned palmitic acid increasing activity, e.g. of a polypeptide having an activity of a protein as indicated in Table II, column 3, lines 82 to 88 and 463 to 467 or its homologs activity, e.g. as indicated in Table II, column 7, lines 82 to 88 and 463 to 467, or decreasing the inhibitiory regulation of the polypeptide of the invention or of the polypeptide used in the method of the invention;
d) generating or increasing the expression of an endogenous or artificial transcription factor mediating the expression of a protein conferring the increased expression of a protein encoded by the nucleic acid molecule of the invention or the nucleic acid molecule used in the method of the invention or of the polypeptide of the invention or the polypeptide used in the method of the invention having herein-mentioned palmitic acid increasing activity, e.g. of a polypeptide having an activity of a protein as indicated in Table II, column 3, lines 82 to 88 and 463 to 467 or its homolog's activity, e.g. as indicated in Table II, column 7, lines 82 to 88 and 463 to 467;
e) stimulating activity of a protein conferring the increased expression of a protein encoded by the nucleic acid molecule of the present invention or a polypeptide of the present invention having herein-mentioned palmitic acid increasing activity, e.g. of a polypeptide having an activity of a protein as indicated in Table II, column 3, lines 82 to 88 and 463 to 467 or its homolog's activity, e.g. as indicated in Table II, column 7, lines 82 to 88 and 463 to 467, by adding one or more exogenous inducing factors to the organismus or parts thereof;
f) expressing a transgenic gene encoding a protein conferring the increased expression of a polypeptide encoded by the nucleic acid molecule of the present invention or a polypeptide of the present invention, having herein-mentioned palmitic acid increasing activity, e.g. of a polypeptide having an activity of a protein as indicated in Table II, column 3, lines 82 to 88 and 463 to 467 or its homolog's activity, e.g. as indicated in Table II, column 7, lines 82 to 88 and 463 to 467;
g) increasing the copy number of a gene conferring the increased expression of a nucleic acid molecule encoding a polypeptide encoded by the nucleic acid molecule of the invention or the nucleic acid molecule used in the method of the invention or the polypeptide of the invention or the polypeptide used in the method of the invention having herein-mentioned palmitic acid increasing activity, e.g. of a polypeptide having an activity of a protein as indicated in Table II, column 3, lines 82 to 88 and 463 to 467 or its homolog's activity, e.g. as indicated in Table II, column 7, lines 82 to 88 and 463 to 467;
h) increasing the expression of the endogenous gene encoding the polypeptide of the invention or the polypeptide used in the method of the invention, e.g. a polypeptide having an activity of a protein as indicated in Table II, column 3, lines 82 to 88 and 463 to 467 or its homolog's activity, e.g. as indicated in Table II, column 7, lines 82 to 88 and 463 to 467, by adding positive expression or removing negative expression elements, e.g. homologous recombination can be used to either introduce positive regulatory elements like for plants the 35S enhancer into the promoter or to remove repressor elements form regulatory regions. Further gene conversion methods can be used to disrupt repressor elements or to enhance to acitivty of positive elements. Positive elements can be randomly introduced in plants by T-DNA or transposon mutagenesis and lines can be identified in which the positive elements have be integrated near to a gene of the invention, the expression of which is thereby enhanced;
i) Modulating growth conditions of an organism in such a manner, that the expression or activity of the gene encoding the protein of the invention or the protein itself is enhanced for example microorganisms or plants can be grown for example under a higher temperature regime leading to an enhanced expression of heat shock proteins, which can lead an enhanced fine chemical production.
j) selecting of organisms with expecially high activity of the proteins of the invention from natural or from mutagenized resources and breeding them into the target organisms, eg the elite crops.

**[0054.0.8.8]** Preferably, said mRNA is the nucleic acid molecule of the present invention and/or the protein conferring the increased expression of a protein encoded by the nucleic acid molecule of the present invention or the polypeptide having the herein mentioned activity is the polypeptide of the present invention, e.g. conferring the increase of palmitic acid after increasing the expression or activity of the encoded polypeptide or having the activity of a polypeptide having an activity of a protein as indicated in Table II, column 3, lines 82 to 88 and 463 to 467 or its homolog's activity, e.g. as indicated in Table II, columns 5 or 7, lines 82 to 88 and 463 to 467.

**[0055.0.0.8] to [0067.0.0.8]** for the disclosure of the paragraphs [0055.0.0.8] to [0067.0.0.8] see paragraphs [0055.0.0.0] to [0067.0.0.0] above.

**[0068.0.5.8] and [0069.0.5.8]** for the disclosure of the paragraphs [0068.0.5.8] and [0069.0.5.8] see paragraphs [0068.0.0.0] and [0069.0.0.0] above.

**[0070.0.6.8] and [0071.0.5.8]** for the disclosure of the paragraphs [0070.0.6.8] and [0071.0.5.8] see paragraphs [0070.0.5.5] and [0071.0.0.0] above.

**[0072.0.8.8]** By influencing the metabolism thus, it is possible to produce, in the process according to the invention, further advantageous compounds. Examples of such compounds are, in addition to palmitic acid, triglycerides, lipids, oils and/or fats containing palmitic acid compounds such as palmitate, palmitoleate, stearate, oleate, α-linolenic acid and/or linoleic acid.

**[0073.0.8.8]** Accordingly, in one embodiment, the process according to the invention relates to a process, which comprises:
a) providing a non-human organism, preferably a microorganism, a non-human animal, a plant or animal cell, a plant or animal tissue or a plant;
b) increasing the activity of a protein having the activity of a polypeptide of the invention or the polypeptide used in the method of the invention or a homolog thereof, e.g. as shown in Table II, columns 5 or 7, lines 82 to 88 and 463 to 467 or of a polypeptide being encoded by the nucleic acid molecule of the present invention and described below, i.e. conferring an increase of the respective fine chemical in the organism, preferably a microorganism, the a non-human animal, a plant or animal cell, a plant or animal tissue or the plant,
c) growing the organism, preferably a microorganism, a non-human animal, a plant or animal cell, a plant or animal tissue or the plant under conditions which permit the production of the fine chemical in the organism, preferably a microorganism, a plant cell, a plant tissue or the plant; and
d) if desired, recovering, optionally isolating, the free and/or bound the respective fine chemical and, optionally further free and/or bound fatty acids synthetized by the organism, the microorganism, the non-human animal, the plant or animal cell, the plant or animal tissue or the plant.

**[0074.0.5.8]** for the disclosure of this paragraph see [0074.0.5.5] above.

**[0075.0.0.8] to [0084.0.0.8]** for the disclosure of the paragraphs [0075.0.0.8] to [0084.0.0.8] see paragraphs [0075.0.0.0] to [0084.0.0.0] above.

**[0085.0.8.8]** With regard to the nucleic acid sequence as depicted a nucleic acid construct which contains a nucleic acid sequence mentioned herein or an organism (= transgenic organism) which is transformed with said nucleic acid sequence or said nucleic acid construct, "transgene" means all those constructs which have been brought about by genetic manipulation methods,preferably in which either
a) the nucleic acid sequence as shown in table I, lines 82 to 88 and 463 to 467, columns 5 and 7 or a derivative thereof, or
b) a genetic regulatory element, for example a promoter, which is functionally linked to the nucleic acid sequence as shown table I, lines 82 to 88 and 463 to 467, columns 5 and 7 or a derivative thereof, or
c) (a) and (b)
is/are not present in its/their natural genetic environment or has/have been modified by means of genetic manipulation methods, it being possible for the modification to be, by way of example, a substitution, addition, deletion, inversion or insertion of one or more nucleotide. "Natural genetic environment" means the natural chromosomal locus in the organism of origin or the presence in a genomic library. In the case of a genomic library, the natural, genetic environment of the nucleic acid sequence is preferably at least partially still preserved. The environment flanks the nucleic acid sequence at least on one side and has a sequence length of at least 50 bp, preferably at least 500 bp, particularly preferably at least 1000 bp, very particularly preferably at least 5000 bp.

**[0086.0.0.8] and [0087.0.0.8]** for the disclosure of the paragraphs [0086.0.0.8] and [0087.0.0.8] see paragraphs [0086.0.0.0] and [0087.0.0.0] above.

**[0088.0.8.8]** In an advantageous embodiment of the invention, the organism takes the form of a plant whose fatty acid content is modified advantageously owing to the nucleic acid molecule of the present invention expressed. This is important for plant breeders since, for example, the nutritional value of plants for poultry is dependent on the abovementioned essential fatty acids and the general amount of fatty acids as energy source in feed. After the activity of a protein as shown in Table II, columns 5 or 7, lines 82 to 88 and 463 to 467 has been increased or generated, or after the expression of nucleic acid molecule or polypeptide according to the invention has been generated or increased, the transgenic plant generated thus is grown on or in a nutrient medium or else in the soil and subsequently harvested.

**[0088.1.0.8], [0089.0.0.8], [0090.0.0.8] and [0091.0.5.8]** for the disclosure of the paragraphs [0088.1.0.8], [0089.0.0.8], [0090.0.0.8] and [0091.0.5.8] see paragraphs [0088.1.0.0], [0089.0.0.0], [0090.0.0.0] and [0091.0.0.0] above.

**[0092.0.0.8] to [0094.0.0.8]** for the disclosure of the paragraphs [0092.0.0.8] to [0094.0.0.8] see paragraphs [0092.0.0.0] to [0094.0.0.0] above.

**[0095.0.5.8], [0096.0.5.8] and [0097.0.5.8]** for the disclosure of the paragraphs [0095.0.5.8], [0096.0.5.8] and [0097.0.5.8] see paragraphs [0095.0.5.5], [0096.0.5.5] and [0097.0.0.0] above.

**[0098.0.8.8]** In a preferred embodiment, the fine chemical (palmitic acid) is produced in accordance with the invention and, if desired, is isolated. The production of further fatty acids such as stearic acid, palmitoleic acid, oleic acid, α-linolenic acid and/or linoleic acid mixtures thereof or mixtures of other fatty acids by the process according to the invention is advantageous.

**[0099.0.5.8] and [0100.0.5.8]** for the disclosure of the paragraphs [0099.0.5.8] and [0100.0.5.8] see paragraphs [0099.0.5.5] and [0100.0.5.5] above.

**[0101.0.5.8] and [0102.0.5.8]** for the disclosure of the paragraphs [0101.0.5.8] and [0102.0.5.8] see [0101.0.0.0] and [0102.0.5.5] above.

**[0103.0.8.8]** In a preferred embodiment, the present invention relates to a process for the production of the fine chemical comprising or generating in an organism or a part thereof the expression of at least one nucleic acid molecule comprising a nucleic acid molecule selected from the group consisting of:
a) nucleic acid molecule encoding, preferably at least the mature form, of a polypeptide having a sequence as shown in Table II, columns 5 or 7, lines 82 to 88 and 463 to 467 in or a fragment thereof, which confers an increase in the amount of the respective fine chemical in an organism or a part thereof;
b) nucleic acid molecule comprising, preferably at least the mature form, of the nucleic acid molecule having a sequence as shown in Table I, columns 5 or 7, lines 82 to 88 and 463 to 467,
c) nucleic acid molecule whose sequence can be deduced from a polypeptide sequence encoded by a nucleic acid molecule of (a) or (b) as result of the degeneracy of the genetic code and conferring an increase in the amount of the fine chemical in an organism or a part thereof;
d) nucleic acid molecule encoding a polypeptide which has at least 50% identity with the amino acid sequence of the polypeptide encoded by the nucleic acid molecule of (a) to (c) and conferring an increase in the amount of the fine chemical in an organism or a part thereof;
e) nucleic acid molecule which hybidizes with a nucleic acid molecule of (a) to (c) under under stringent hybridisation conditions and conferring an increase in the amount of the fine chemical in an organism or a part thereof;
f) nucleic acid molecule encoding a polypeptide, the polypeptide being derived by substituting, deleting and/or adding one or more amino acids of the amino acid sequence of the polypeptide encoded by the nucleic acid molecules (a) to (d), preferably to (a) to (c) and conferring an increase in the amount of the respective fine chemical in an organism or a part thereof;
g) nucleic acid molecule encoding a fragment or an epitope of a polypeptide which is encoded by one of the nucleic acid molecules of (a) to (e), preferably to (a) to (c) and and conferring an increase in the amount of the respective fine chemical in an organism or a part thereof;
h) nucleic acid molecule comprising a nucleic acid molecule which is obtained by amplifying nucleic acid molecules from a cDNA library or a genomic library using the primer pairs having a sequence as shown in Table III, column 7, lines 82 to 88 and 463 to 467 and conferring an increase in the amount of the respective fine chemical in an organism or a part thereof;
i) nucleic acid molecule encoding a polypeptide which is isolated, e.g. from an expression library, with the aid of monoclonal antibodies against a polypeptide encoded by one of the nucleic acid molecules of (a) to (h), preferably to (a) to (c), and and conferring an increase in the amount of the respective fine chemical in an organism or a part thereof;
j) nucleic acid molecule which encodes a polypeptide comprising the consensus sequence having a sequence as shown in Table IV, column 7, lines 82 to 88 and 463 to 467 and conferring an increase in the amount of the respective fine chemical in an organism or a part thereof ;
k) nucleic acid molecule comprising one or more of the nucleic acid molecule encoding the amino acid sequence of a polypeptide encoding a domain of a polypeptide as shown in Table II, columns 5 or 7, lines 82 to 88 and 463 to 467 and conferring an increase in the amount of the respective fine chemical in an organism or a part thereof; and
l) nucleic acid molecule which is obtainable by screening a suitable library under stringent conditions with a probe comprising one of the sequences of the nucleic acid molecule of (a) to (k), preferably to (a) to (c), or with a fragment of at least 15 nt, preferably 20 nt, 30 nt, 50 nt, 100 nt, 200 nt or 500 nt of the nucleic acid molecule characterized in (a) to (k), preferably to (a) to (c), and conferring an increase in the amount of the fine chemical in an organism or a part thereof;
or which comprises a sequence which is complementary thereto.

**[0103.1.0.8] and [0103.2.0.8]** for the disclosure of the paragraphs [0103.1.0.8] and [0103.2.0.8] see paragraphs [0103.1.0.0] and [0103.2.0.0] above.

**[0104.0.8.8]** In one embodiment, the nucleic acid molecule used in the process distinguishes over the sequence shown in Table I, columns 5 or 7, lines 82 to 88 and 463 to 467, preferably over the sequences as shown in Table IA, columns 5 or 7, lines 82 to 88 and 463 to 467 by one or more nucleotides or does not consist of the sequence shown in Table I, columns 5 or 7, lines 82 to 88 and 463 to 467, preferably not of the sequences as shown in Table IA, columns 5 or 7, lines 82 to 88 and 463 to 467. In one embodiment, the nucleic acid molecule of the present invention is less than 100%, 99,999%, 99,99%, 99,9% or 99% identical to the sequence shown in Table I, columns 5 or 7, lines 82 to 88 and 463 to 467, preferably to the sequences as shown in Table IA, columns 5 or 7, lines 82 to 88 and 463 to 467. In another embodiment, the nucleic acid molecule does not encode a polypeptide of the sequence shown in Table II, columns 5 or 7, lines 82 to 88 and 463 to 467, preferably of the sequences as shown in Table IA, columns 5 or 7, lines 82 to 88 and 463 to 467.

**[0105.0.0.8] to [0107.0.0.8]** for the disclosure of the paragraphs [0105.0.0.8] to [0107.0.0.8] see paragraphs [0105.0.0.0] and [0107.0.0.0] above.

**[0108.0.8.8]** Nucleic acid molecules with the sequence shown in Table I, columns 5 or 7, lines 82 to 88 and 463 to 467, nucleic acid molecules which are derived from the amino acid sequences shown in Table II, columns 5 or 7, lines 82 to 88 and 463 to 467 or from polypeptides comprising the consensus sequence shown in Table IV, column 7, lines 82 to 88 and 463 to 467, or their derivatives or homologues encoding polypeptides with the enzymatic or biological activity of a protein as shown in Table II, columns 5 or 7, lines 82 to 88 and 463 to 467 or e.g. conferring a linoleic acid increase after increasing its expression or activity are advantageously increased in the process according to the invention.

**[0109.0.5.8]** for the disclosure of this paragraph see [0109.0.0.0] above.

**[0110.0.8.8]** Nucleic acid molecules, which are advantageous for the process according to the invention and which encode polypeptides with an activity of a polypeptide used in the method of the invention or used in the process of the invention, e.g. of a protein as shown in Table II, columns 5 or 7, lines 82 to 88 and 463 to 467 or being encoded by a nucleic acid molecule indicated in Table I, columns 5 or 7, lines 82 to 88 and 463 to 467 or of its homologs, e.g. as indicated in Table II, columns 5 or 7, lines 82 to 88 and 463 to 467 can be determined from generally accessible databases.

**[0111.0.0.8]** for the disclosure of this paragraph see [0111.0.0.0] above.

**[0112.0.8.8]** The nucleic acid molecules used in the process according to the invention take the form of isolated nucleic acid sequences, which encode polypeptides with an activity of a polypeptide as indicated in Table II, column3, lines lines 82 to 88 and 463 to 467 or having the sequence of a polypeptide as indicated in Table II, columns 5 and 7, lines 82 to 88 and 463 to 467 and conferring an increase of the respective fine chemical.

**[0113.0.0.8] to [0120.0.0.8]** for the disclosure of the paragraphs [0113.0.0.8] to [0120.0.0.8] see paragraphs [0113.0.0.0] and [0120.0.0.0] above.

**[0121.0.8.8]** However, it is also possible to use artificial sequences, which differ in one or more bases from the nucleic acid sequences found in organisms, or in one or more amino acid molecules from polypeptide sequences found in organisms, in particular from the polypeptide sequences shown in Table II, columns 5 or 7, lines 82 to 88 and 463 to 467 or the functional homologues thereof as described herein, preferably conferring above-mentioned activity, i.e. conferring an increase of the respective fine chemical after increasing its activity.

**[0122.0.0.8] to [0127.0.0.8]** for the disclosure of the paragraphs [0122.0.0.8] to [0127.0.0.8] see paragraphs [0122.0.0.0] and [0127.0.0.0] above.

**[0128.0.8.8]** Synthetic oligonucleotide primers for the amplification, e.g. as shown in Table III, column 7, lines 82 to 88 and 463 to 467, by means of polymerase chain reaction can be generated on the basis of a sequence shown herein, for example the sequence shown in Table I, columns 5 or 7, lines 82 to 88 and 463 to 467 or the sequences as shown in Table II, columns 5 or 7, lines 82 to 88 and 463 to 467.

**[0129.0.8.8]** Moreover, it is possible to identify conserved regions from various organisms by carrying out protein sequence alignments with the polypeptide used in the process of the invention, in particular with sequences of the polypeptide of the invention, from which conserved regions, and in turn, degenerate primers can be derived. Conserved region for the polypeptide of the invention are indicated in the alignments shown in the figures. Conserved regions are those, which show a very little variation in the amino acid in one particular position of several homologs from different origin. The consenus sequences shown in Table IV, column 7, lines 82 to 88 and 463 to 467 are derived from said aligments.

**[0130.0.8.8]** for the disclosure of this paragraph see [0130.0.0.0].

**[0131.0.0.8] to [0138.0.0.8]** for the disclosure of the paragraphs [0131.0.0.8] to [0138.0.0.8] see paragraphs [0131.0.0.0] to [0138.0.0.0] above.

**[0139.0.8.8]** Polypeptides having above-mentioned activity, i.e. conferring the respective fine chemical increase, derived from other organisms, can be encoded by other DNA sequences which hybridize to the sequences shown in Table I, columns 5 or 7, lines 82 to 88 and 463 to 467, preferably of Table I B, columns 5 or 7, lines 82 to 88 and 463 to 467 under relaxed hybridization conditions and which code on expression for peptides having the palmitic acid increasing activity.

**[0140.0.0.8] to [0146.0.0.8]** for the disclosure of the paragraphs [0140.0.0.8] to [0146.0.0.8] see paragraphs [0140.0.0.0] and [0146.0.0.0] above.

**[0147.0.8.8]** Further, the nucleic acid molecule of the invention comprises a nucleic acid molecule, which is a complement of one of the nucleotide sequences of above mentioned nucleic acid molecules or a portion thereof. A nucleic acid molecule which is complementary to one of the nucleotide sequences indicated in Table I, columns 5 or 7, lines 82 to 88 and 463 to 467, preferably in Table I B, columns 5 or 7, lines 82 to 88 and 463 to 467 is one which is sufficiently complementary to one of said nucleotide sequences such that it can hybridize to one of said nucleotide sequences thereby forming a stable duplex. Preferably, the hybridisation is performed under stringent hybrization conditions. However, a complement of one of the herein disclosed sequences is preferably a sequence complement thereto according to the base pairing of nucleic acid molecules well known to the skilled person. For example, the bases A and G undergo base pairing with the bases T and U or C, resp. and visa versa. Modifications of the bases can influence the base-pairing partner.

**[0148.0.8.8]** The nucleic acid molecule of the invention comprises a nucleotide sequence which is at least about 30%, 35%, 40% or 45%, preferably at least about 50%, 55%, 60% or 65%, more preferably at least about 70%, 80%, or 90%, and even more preferably at least about 95%, 97%, 98%, 99% or more homologous to a nucleotide sequence indicated in Table I, columns 5 or 7, lines 82 to 88 and 463 to 467, preferably of Table I B, columns 5 or 7, lines 82 to 88 and 463 to 467, or a functional portion thereof and preferably has above mentioned activity, in particular has the-fine-chemical-increasing activity after increasing its acitivity or an activity of a product of a gene encoding said sequence or its homologs.

**[0149.0.8.8]** The nucleic acid molecule of the invention or the nucleic acid molecule used in the method of the invention comprises a nucleotide sequence which hybridizes, preferably hybridizes under stringent conditions as defined herein, to one of the nucleotide sequences indicated in Table I, columns 5 or 7, lines 82 to 88 and 463 to 467, preferably of Table I B, columns 5 or 7, lines 82 to 88 and 463 to 467 or a portion thereof and encodes a protein having above-mentioned activity as indicated in Table II, columns 5 or 7, lines 82 to 88 and 463 to 467, preferably of Table II B, columns 5 or 7, lines 82 to 88 and 463 to 467, e.g. conferring an increase of the respective fine chemical.

**[00149.1.0.8]** Optionally, the nucleotide sequence, which hybridises to one of the nucleotide sequences indicated in Table I, columns 5 or 7, lines 82 to 88 and 463 to 467, preferably of Table I B, columns 5 or 7, lines 82 to 88 and 463 to 467 has further one or more of the activities annotated or known for the a protein as indicated in Table II, column 3, lines 82 to 88 and 463 to 467.

**[0150.0.8.8]** Moreover, the nucleic acid molecule of the invention or used in the process of the invention can comprise only a portion of the coding region of one of the sequences indicated in Table I, columns 5 or 7, lines 82 to 88 and 463 to 467, preferably of Table I B, columns 5 or 7, lines 82 to 88 and 463 to 467, for example a fragment which can be used as a probe or primer or a fragment encoding a biologically active portion of the polypeptide of the present invention or of a polypeptide used in the process of the present invention, i.e. having above-mentioned activity, e.g.conferring an increase of methionine if its activity is increased. The nucleotide sequences determined from the cloning of the present protein-according-to-the-invention-encoding gene allows for the generation of probes and primers designed for use in identifying and/or cloning its homologues in other cell types and organisms. The probe/primer typically comprises substantially purified oligonucleotide. The oligonucleotide typically comprises a region of nucleotide sequence that hybridizes under stringent conditions to at least about 12, 15 preferably about 20 or 25, more preferably about 40, 50 or 75 consecutive nucleotides of a sense strand of one of the sequences indicated in Table I, columns 5 or 7, lines 82 to 88 and 463 to 467, an anti-sense sequence of one of the sequences indicated in Table I, columns 5 or 7, lines 82 to 88 and 463 to 467, or naturally occurring mutants thereof. Primers based on a nucleotide sequence of the invention can be used in PCR reactions to clone homologues of the polypeptide of the invention or of the polypeptide used in the process of the invention, e.g. as the primers described in the examples of the present invention, e.g. as shown in the examples. A PCR with the primer pairs indicated in Table III, column 7, lines 82 to 88 and 463 to 467 will result in a fragment of a polynucleotide sequence as indicated in Table I, columns 5 or 7, lines 82 to 88 and 463 to 467. Preferred is Table I B, column 7, lines 82 to 88 and 463 to 467.

**[0151.0.0.8]** for the disclosure of this paragraph see [0151.0.0.0] above.

**[0152.0.8.8]** The nucleic acid molecule of the invention encodes a polypeptide or portion thereof which includes an amino acid sequence which is sufficiently homologous to the amino acid sequence shown in Table II, columns 5 or 7, lines 82 to 88 and 463 to 467 such that the protein or portion thereof maintains the ability to participate in the fine chemical production, in particular a palmitic acid increasing the activity as mentioned above or as described in the examples in plants or microorganisms is comprised.

**[0153.0.8.8]** As used herein, the language "sufficiently homologous" refers to proteins or portions thereof which have amino acid sequences which include a minimum number of identical or equivalent amino acid residues (e.g., an amino acid residue which has a similar side chain as an amino acid residue in one of the sequences of the polypeptide of the present invention) to an amino acid sequence shown in Table II, columns 5 or 7, lines 82 to 88 and 463 to 467 such that the protein or portion thereof is able to participate in the increase of the respective fine chemical production. In one embodiment, a protein or portion thereof as shown in Table II, columns 5 or 7, lines 82 to 88 and 463 to 467 has for example an activity of a polypeptide as indicated in Table II, columns 5 or 7, lines 82 to 88 and 463 to 467.

**[0154.0.8.8]** In one embodiment, the nucleic acid molecule of the present invention comprises a nucleic acid that encodes a portion of the protein of the present invention. The protein is at least about 30%, 35%, 40%, 45% or 50%, preferably at least about 55%, 60%, 65% or 70%, and more preferably at least about 75%, 80%, 85%, 90%, 91%, 92%, 93% or 94% and most preferably at least about 95%, 97%, 98%, 99% or more homologous to an entire amino acid sequence as shown in Table II, columns 5 or 7, lines 82 to 88 and 463 to 467 and having above-mentioned activity, e.g.conferring preferably the increase of the respective fine chemical.

**[0155.0.0.8] and [0156.0.0.8]** for the disclosure of the paragraphs [0155.0.0.8] and [0156.0.0.8] see paragraphs [0155.0.0.0] and [0156.0.0.0] above.

**[0157.0.8.8]** The invention further relates to nucleic acid molecules that differ from one of the nucleotide sequences indicated in Table I, columns 5 or 7, lines 82 to 88 and 463 to 467 (and portions thereof) due to degeneracy of the genetic code and thus encode a polypeptide of the present invention, in particular a polypeptide having above mentioned activity, e.g. conferring an increase in the respective fine chemical in a organism, e.g. as that polypeptides comprising the consensus sequences as indicated in Table IV, column 7, lines 82 to 88 and 463 to 467 or of the polypeptide as indicated in Table II, columns 5 or 7, lines 82 to 88 and 463 to 467 or their functional homologues. Advantageously, the nucleic acid molecule of the invention or the nucleic acid molecule used in the method of the invention comprises, or in an other embodiment has, a nucleotide sequence encoding a protein comprising, or in an other embodiment having, a consensus sequences as indicated in Table IV, column 7, lines 82 to 88 and 463 to 467 or of the polypeptide as indicated in Table II, columns 5 or 7, lines 82 to 88 and 463 to 467 or the functional homologues. In a still further embodiment, the nucleic acid molecule of the invention or the nucleic acid molecule used in the method of the invention encodes a full length protein which is substantially homologous to an amino acid sequence comprising a consensus sequence as indicated in Table IV, column 7, lines 82 to 88 and 463 to 467 or of a polypeptide as indicated in Table II, columns 5 or 7, lines 82 to 88 and 463 to 467 or the functional homologues thereof. However, in a preferred embodiment, the nucleic acid molecule of the present invention does not consist of a sequence as indicated in Table I, columns 5 or 7, lines 82 to 88 and 463 to 467, preferably as indicated in Table I A, columns 5 or 7, lines 82 to 88 and 463 to 467. Preferably the nucleic acid molecule of the invention is a functional homologue or identical to a nucleic acid molecule indicated in Table I B, columns 5 or 7, lines 82 to 88 and 463 to 467.

**[0158.0.0.8] to [0160.0.0.8]** for the disclosure of the paragraphs [0158.0.0.8] to [0160.0.0.8] see paragraphs [0158.0.0.0] to [0160.0.0.0] above.

**[0161.0.8.8]** Accordingly, in another embodiment, a nucleic acid molecule of the invention or the nucleic acid molecule used in the method of the invention is at least 15, 20, 25 or 30 nucleotides in length. Preferably, it hybridizes under stringent conditions to a nucleic acid molecule comprising a nucleotide sequence of the nucleic acid molecule of the present invention or used in the process of the present invention, e.g. comprising the sequence shown in Table I, columns 5 or 7, lines 82 to 88 and 463 to 467. The nucleic acid molecule is preferably at least 20, 30, 50, 100, 250 or more nucleotides in length.

**[0162.0.0.8]** for the disclosure of this paragraph see paragraph [0162.0.0.0] above.

**[0163.0.8.8]** Preferably, nucleic acid molecule of the invention that hybridizes under stringent conditions to a sequence as indicated in Table I, columns 5 or 7, lines 82 to 88 and 463 to 467 corresponds to a naturally-occurring nucleic acid molecule of the invention. As used herein, a "naturally-occurring" nucleic acid molecule refers to an RNA or DNA molecule having a nucleotide sequence that occurs in nature (e.g., encodes a natural protein). Preferably, the nucleic acid molecule encodes a natural protein having above-mentioned activity, e.g. conferring the respective fine chemical increase after increasing the expression or activity thereof or the activity of a protein of the invention or used in the process of the invention.

**[0164.0.0.8]** for the disclosure of this paragraph see paragraph [0164.0.0.0] above.

**[0165.0.8.8]** For example, nucleotide substitutions leading to amino acid substitutions at "non-essential" amino acid residues can be made in a sequence of the nucleic acid molecule of the invention or used in the process of the invention, e.g. as shown in Table I, columns 5 or 7, lines 82 to 88 and 463 to 467.

**[0166.0.0.8] and [0167.0.0.8]** for the disclosure of the paragraphs [0166.0.0.8] and [0167.0.0.8] see paragraphs [0166.0.0.0] and [0167.0.0.0] above.

**[0168.0.8.8]** Accordingly, the invention relates to nucleic acid molecules encoding a polypeptide having above-mentioned activity, e.g. conferring an increase in the respective fine chemical in an organism or parts thereof that contain changes in amino acid residues that are not essential for said activity. Such polypeptides differ in amino acid sequence from a sequence contained in a sequence as indicated in Table II, columns 5 or 7, lines 82 to 88 and 463 to 467 yet retain said activity described herein. The nucleic acid molecule can comprise a nucleotide sequence encoding a polypeptide, wherein the polypeptide comprises an amino acid sequence at least about 50% identical to an amino acid sequence as indicated in Table II, columns 5 or 7, lines 82 to 88 and 463 to 467 and is capable of participation in the increase of production of the respective fine chemical after increasing its activity, e.g. its expression. Preferably, the protein encoded by the nucleic acid molecule is at least about 60% identical to the sequence as indicated in Table II, columns 5 or 7, lines 82 to 88 and 463 to 467, more preferably at least about 70% identical to one of the sequences as indicated in Table II, columns 5 or 7, lines 82 to 88 and 463 to 467, even more preferably at least about 80%, 90%, 95% homologous to the sequence as indicated in Table II, columns 5 or 7, lines 82 to 88 and 463 to 467, and most preferably at least about 96%, 97%, 98%, or 99% identical to the sequence as indicated in Table II, columns 5 or 7, lines 82 to 88 and 463 to 467.
Accordingly, the invention relates to nucleic acid molecules encoding a polypeptide having above-mentioned activity, e.g. conferring an increase in the the respective fine chemical in an organisms or parts thereof that contain changes in amino acid residues that are not essential for said activity. Such polypeptides differ in amino acid sequence from a sequence contained in a sequence as indicated in Table II, columns 5 or 7, lines 82 to 88 and 463 to 467, preferably of Table II B, column 7, lines 82 to 88 and 463 to 467 yet retain said activity described herein. The nucleic acid molecule can comprise a nucleotide sequence encoding a polypeptide, wherein the polypeptide comprises an amino acid sequence at least about 50% identical to an amino acid sequence as indicated in Table II, columns 5 or 7, lines 82 to 88 and 463 to 467, preferably of Table II B, column 7, lines 82 to 88 and 463 to 467 and is capable of participation in the increase of production of the respective fine chemical after increasing its activity, e.g. its expression. Preferably, the protein encoded by the nucleic acid molecule is at least about 60% identical to a sequence as indicated in Table II, columns 5 or 7, lines 82 to 88 and 463 to 467, preferably of Table II B, column 7, lines 82 to 88 and 463 to 467, more preferably at least about 70% identical to one of the sequences as indicated in Table II, columns 5 or 7, lines 82 to 88 and 463 to 467, preferably of Table II B, column 7, lines 82 to 88 and 463 to 467, even more preferably at least about 80%, 90%, or 95% homologous to a sequence as indicated in Table II, columns 5 or 7, lines 82 to 88 and 463 to 467, preferably of Table II B, column 7, lines 82 to 88 and 463 to 467, and most preferably at least about 96%, 97%, 98%, or 99% identical to the sequence as indicated in Table II, columns 5 or 7, lines 82 to 88 and 463 to 467, preferably of Table II B, column 7, lines 82 to 88 and 463 to 467.

**[0169.0.0.8] to [0175.0.5.8]** for the disclosure of the paragraphs [0169.0.0.8] to [0175.0.5.8] see paragraphs [0169.0.0.0] to [0175.0.0.0] above.

**[0176.0.8.8]** Functional equivalents derived from one of the polypeptides as shown in Table II, columns 5 or 7, lines 82 to 88 and 463 to 467 according to the invention by substitution, insertion or deletion have at least 30%, 35%, 40%, 45% or 50%, preferably at least 55%, 60%, 65% or 70% by preference at least 80%, especially preferably at least 85% or 90%, 91 %, 92%, 93% or 94%, very especially preferably at least 95%, 97%, 98% or 99% homology with one of the polypeptides as shown in Table II, columns 5 or 7, lines 82 to 88 and 463 to 467 according to the invention and are distinguished by essentially the same properties as the polypeptide as shown in Table II, columns 5 or 7, lines 82 to 88 and 463 to 467.

**[0177.0.8.8]** Functional equivalents derived from the nucleic acid sequence as shown in Table I, columns 5 or 7, lines 82 to 88 and 463 to 467 according to the invention by substitution, insertion or deletion have at least 30%, 35%, 40%, 45% or 50%, preferably at least 55%, 60%, 65% or 70% by preference at least 80%, especially preferably at least 85% or 90%, 91%, 92%, 93% or 94%, very especially preferably at least 95%, 97%, 98% or 99% homology with one of the polypeptides as shown in Table II, columns 5 or 7, lines 82 to 88 and 463 to 467 according to the invention and encode polypeptides having essentially the same properties as the polypeptide as shown in Table II, columns 5 or 7, lines 82 to 88 and 463 to 467.

**[0178.0.0.8]** for the disclosure of this paragraph see [0178.0.0.0] above.

**[0179.0.8.8]** A nucleic acid molecule encoding a homologous protein to a protein sequence of as indicated in Table II, columns 5 or 7, lines 82 to 88 and 463 to 467, preferably of Table II B, column 7, lines 82 to 88 and 463 to 467 can be created by introducing one or more nucleotide substitutions, additions or deletions into a nucleotide sequence of the nucleic acid molecule of the present invention, in particular as indicated in Table I, columns 5 or 7, lines 82 to 88 and 463 to 467 such that one or more amino acid substitutions, additions or deletions are introduced into the encoded protein. Mutations can be introduced into the encoding sequences for example into a sequences as indicated in Table I, columns 5 or 7, lines 82 to 88 and 463 to 467 by standard techniques, such as site-directed mutagenesis and PCR-mediated mutagenesis.

**[0180.0.0.8] to [0183.0.0.8]** for the disclosure of the paragraphs [0180.0.0.8] to [0183.0.0.8] see paragraphs [0180.0.0.0] to [0183.0.0.0] above.

**[0184.0.8.8]** Homologues of the nucleic acid sequences used, with a sequence as indicated in Table I, columns 5 or 7, lines 82 to 88 and 463 to 467, preferably of Table I B, column 7, lines 82 to 88 and 463 to 467, or of the nucleic acid sequences derived from a sequences as indicated in Table II, columns 5 or 7, lines 82 to 88 and 463 to 467, preferably of Table II B, column 7, lines 82 to 88 and 463 to 467, comprise also allelic variants with at least approximately 30%, 35%, 40% or 45% homology, by preference at least approximately 50%, 60% or 70%, more preferably at least approximately 90%, 91%, 92%, 93%, 94% or 95% and even more preferably at least approximately 96%, 97%, 98%, 99% or more homology with one of the nucleotide sequences shown or the abovementioned derived nucleic acid sequences or their homologues, derivatives or analogues or parts of these. Allelic variants encompass in particular functional variants which can be obtained by deletion, insertion or substitution of nucleotides from the sequences shown, preferably from a sequence as indicated in Table I, columns 5 or 7, lines 82 to 88 and 463 to 467, or from the derived nucleic acid sequences, the intention being, however, that the enzyme activity or the biological activity of the resulting proteins synthesized is advantageously retained or increased. [0185.0.8.8] In one embodiment of the present invention, the nucleic acid molecule of the invention or used in the process of the invention comprises one or more sequences as indicated in Table I, columns 5 or 7, lines 82 to 88 and 463 to 467, preferably of Table I B, column 7, lines 82 to 88 and 463 to 467. In one embodiment, it is preferred that the nucleic acid molecule comprises as little as possible other nucleotide sequences not shown in any one of sequences as indicated in Table I, columns 5 or 7, lines 82 to 88 and 463 to 467, preferably of Table I B, column 7, lines 82 to 88 and 463 to 467. In one embodiment, the nucleic acid molecule comprises less than 500, 400, 300, 200, 100, 90, 80, 70, 60, 50 or 40 further nucleotides. In a further embodiment, the nucleic acid molecule comprises less than 30, 20 or 10 further nucleotides. In one embodiment, a nucleic acid molecule used in the process of the invention is identical to a sequences as indicated in Table I, columns 5 or 7, lines 82 to 88 and 463 to 467, preferably of Table I B, column 7, lines 82 to 88 and 463 to 467.

**[0186.0.8.8]** Also preferred is that one or more nucleic acid molecule(s) used in the process of the invention encode a polypeptide comprising a sequence as indicated in Table II, columns 5 or 7, lines 82 to 88 and 463 to 467, preferably of Table II B, column 7, lines 82 to 88 and 463 to 467. In one embodiment, the nucleic acid molecule encodes less than 150, 130, 100, 80, 60, 50, 40 or 30 further amino acids. In a further embodiment, the encoded polypeptide comprises less than 20, 15, 10, 9, 8, 7, 6 or 5 further amino acids. In one embodiment, the encoded polypeptide used in the process of the invention is identical to the sequences as indicated in Table II, columns 5 or 7, lines 82 to 88 and 463 to 467, preferably of Table II B, column 7, lines 82 to 88 and 463 to 467.

**[0187.0.8.8]** In one embodiment, the nucleic acid molecule of the invention or used in the process encodes a polypeptide comprising a sequence as indicated in Table II, columns 5 or 7, lines 82 to 88 and 463 to 467, preferably of Table II B, column 7, lines 82 to 88 and 463 to 467 and comprises less than 100 further nucleotides. In a further embodiment, said nucleic acid molecule comprises less than 30 further nucleotides. In one embodiment, the nucleic acid molecule used in the process is identical to a coding sequence encoding a sequences as indicated in Table II, columns 5 or 7, lines 82 to 88 and 463 to 467, preferably of Table II B, column 7, lines 82 to 88 and 463 to 467.

**[0188.0.8.8]** Polypeptides (= proteins), which still have the essential biological activity of the polypeptide of the present invention conferring an increase of the fine chemical i.e. whose activity is essentially not reduced, are polypeptides with at least 10% or 20%, by preference 30% or 40%, especially preferably 50% or 60%, very especially preferably 80% or 90 or more of the wild type biological activity or enzyme activity, advantageously, the activity is essentially not reduced in comparison with the activity of a polypeptide shown in Table II, columns 5 or 7, lines 82 to 88 and 463 to 467 expressed under identical conditions.
In one embodiment, the polypeptide of the invention is a homolog consisting of or comprising the sequence as indicated in Table II B, columns 7, lines 82 to 88 and 463 to 467.

**[0189.0.8.8]** Homologues of sequences as indicated in Table I, columns 5 or 7, lines 82 to 88 and 463 to 467 or of the derived sequences shown in Table II, columns 5 or 7, lines 82 to 88 and 463 to 467 also mean truncated sequences, cDNA, single-stranded DNA or RNA of the coding and noncoding DNA sequence. Homologues of said sequences are also understood as meaning derivatives, which comprise noncoding regions such as, for example, UTRs, terminators, enhancers or promoter variants. The promoters upstream of the nucleotide sequences stated can be modified by one or more nucleotide substitution(s), insertion(s) and/or deletion(s) without, however, interfering with the functionality or activity either of the promoters, the open reading frame (= ORF) or with the 3'-regulatory region such as terminators or other 3'regulatory regions, which are far away from the ORF. It is furthermore possible that the activity of the promoters is increased by modification of their sequence, or that they are replaced completely by more active promoters, even promoters from heterologous organisms. Appropriate promoters are known to the person skilled in the art and are mentioned herein below.

**[0190.0.0.8], [0191.0.5.8], [00191.1.0.8] and [0192.0.0.8] to [0203.0.0.8]** for the disclosure of the paragraphs [0190.0.0.8], [0191.0.5.8], [0191.1.0.8] and [0192.0.0.8] to [0203.0.0.8] see paragraphs [0190.0.0.0], [0191.0.5.5], [0191.1.0.0] and [0192.0.0.0] to [0203.0.0.0] above.

**[0204.0.8.8]** Accordingly, in one embodiment, the invention relates to a nucleic acid molecule, which comprises a nucleic acid molecule selected from the group consisting of:
a) nucleic acid molecule encoding, preferably at least the mature form, of the polypeptide shown in Table II, columns 5 or 7, lines 82 to 88 and 463 to 467; preferably of Table II B, column 7, lines 82 to 88 and 463 to 467; or a fragment thereof conferring an increase in the amount of the fine chemical in an organism or a part thereof
b) nucleic acid molecule comprising, preferably at least the mature form, of the nucleic acid molecule shown in Table I, columns 5 or 7, lines 82 to 88 and 463 to 467 preferably of Table I B, column 7, lines 82 to 88 and 463 to 467; or a fragment thereof conferring an increase in the amount of the fine chemical in an organism or a part thereof;
c) nucleic acid molecule whose sequence can be deduced from a polypeptide sequence encoded by a nucleic acid molecule of (a) or (b) as result of the degeneracy of the genetic code and conferring an increase in the amount of the respective fine chemical in an organism or a part thereof;
d) nucleic acid molecule encoding a polypeptide whose sequence has at least 50% identity with the amino acid sequence of the polypeptide encoded by the nucleic acid molecule of (a) to (c) and conferring an increase in the amount of the respective fine chemical in an organism or a part thereof;
e) nucleic acid molecule which hybridizes with a nucleic acid molecule of (a) to (c) under under stringent hybridisation conditions and conferring an increase in the amount of the respective fine chemical in an organism or a part thereof;
f) nucleic acid molecule encoding a polypeptide, the polypeptide being derived by substituting, deleting and/or adding one or more amino acids of the amino acid sequence of the polypeptide encoded by the nucleic acid molecules (a) to (d), preferably to (a) to (c), and conferring an increase in the amount of the respective fine chemical in an organism or a part thereof;
g) nucleic acid molecule encoding a fragment or an epitope of a polypeptide which is encoded by one of the nucleic acid molecules of (a) to (e), preferably to (a) to (c) and conferring an increase in the amount of the respective fine chemical in an organism or a part thereof ;
h) nucleic acid molecule comprising a nucleic acid molecule which is obtained by amplifying a cDNA library or a genomic library using the primers or primer pairs as indicated in Table III, column 7, lines 82 to 88 and 463 to 467 and conferring an increase in the amount of the respective fine chemical in an organism or a part thereof;
i) nucleic acid molecule encoding a polypeptide which is isolated, e.g. from a expression library, with the aid of monoclonal antibodies against a polypeptide encoded by one of the nucleic acid molecules of (a) to (g), preferably to (a) to (c) and conferring an increase in the amount of the respective fine chemical in an organism or a part thereof;
j) nucleic acid molecule which encodes a polypeptide comprising the consensus sequence shown in Table IV, column 7, lines 82 to 88 and 463 to 467 and conferring an increase in the amount of the respective fine chemical in an organism or a part thereof;
k) nucleic acid molecule encoding the amino acid sequence of a polypeptide encoding a domaine of the polypeptide shown in Table II, columns 5 or 7, lines 82 to 88 and 463 to 467, preferably of Table II B, column 7, lines 82 to 88 and 463 to 467; and conferring an increase in the amount of the respective fine chemical in an organism or a part thereof; and
l) nucleic acid molecule which is obtainable by screening a suitable nucleic acid library under stringent hybridization conditions with a probe comprising one of the sequences of the nucleic acid molecule of (a) to (k) or with a fragment of at least 15 nt, preferably 20 nt, 30 nt, 50 nt, 100 nt, 200 nt or 500 nt of the nucleic acid molecule characterized in (a) to (h) or of the nucleic acid molecule shown in Table I, columns 5 or 7, lines 82 to 88 and 463 to 467 or a nucleic acid molecule encoding, preferably at least the mature form of, the polypeptide shown in Table II, columns 5 or 7, lines 82 to 88 and 463 to 467, and conferring an increase in the amount of the respective fine chemical in an organism or a part thereof; or which encompasses a sequence which is complementary thereto;
whereby, preferably, the nucleic acid molecule according to (a) to (I) distinguishes over the sequence as depicted in Table IA or IB, columns 5 or 7, lines 82 to 88 and 463 to 467 by one or more nucleotides. In one embodiment, the nucleic acid molecule of the invention does not consist of the sequence shown in Table IA or IB, columns 5 or 7, lines 82 to 88 and 463 to 467. In an other embodiment, the nucleic acid molecule of the present invention is at least 30 % identical and less than 100%, 99,999%, 99,99%, 99,9% or 99% identical to the sequence shown in Table IA or IB, columns 5 or 7, lines 82 to 88 and 463 to 467. In a further embodiment the nucleic acid molecule does not encode the polypeptide sequence shown in Table IIA or IIB, columns 5 or 7, lines 82 to 88 and 463 to 467. Accordingly, in one embodiment, the nucleic acid molecule of the present invention encodes in one embodiment a polypeptide which differs at least in one or more amino acids from the polypeptide as depicted in Table IIA or IIB, columns 5 or 7, lines 82 to 88 and 463 to 467 and therefore does not encode a protein of the sequence shown in Table IIA or IIB, columns 5 or 7, lines 82 to 88 and 463 to 467. Accordingly, in one embodiment, the protein encoded by a sequence of a nucleic acid according to (a) to (I) does not consist of the sequence shown in Table IIA or IIB, columns 5 or 7, lines 82 to 88 and 463 to 467. In a further embodiment, the protein of the present invention is at least 30 % identical to protein sequence depicted in Table IIA or IIB, columns 5 or 7, lines 82 to 88 and 463 to 467 and less than 100%, preferably less than 99,999%, 99,99% or 99,9%, more preferably less than 99%, 985, 97%, 96% or 95% identical to the sequence shown in Table IIA or IIB, columns 5 or 7, lines 82 to 88 and 463 to 467.

**[0205.0.0.8] to [0226.0.0.8]** for the disclosure of the paragraphs [0205.0.0.8] to [0226.0.0.8] see paragraphs [0205.0.0.0] to [0226.0.0.0] above.

**[0227.0.8.8]** The abovementioned nucleic acid molecules can be cloned into the nucleic acid constructs or vectors according to the invention in combination together with further genes, or else different genes are introduced by transforming several nucleic acid constructs or vectors (including plasmids) into a host cell, advantageously into a plant cell or a microorgansims.
In addition to the sequence mentioned in Table I, columns 5 or 7, lines 82 to 88 and 463 to 467 or its derivatives, it is advantageous additionally to express and/or mutate further genes in the organisms. Especially advantageously, additionally at least one further gene of the fatty acid biosynthetic pathway such as for palmitate, palmitoleate, stearate and/or oleate is expressed in the organisms such as plants or microorganisms. It is also possible that the regulation of the natural genes has been modified advantageously so that the gene and/or its gene product is no longer subject to the regulatory mechanisms which exist in the organisms. This leads to an increased synthesis of the amino acids desired since, for example, feedback regulations no longer exist to the same extent or not at all. In addition it might be advantageously to combine the sequences shown in Table I, columns 5 or 7, lines 82 to 88 and 463 to 467 with genes which generally support or enhances to growth or yield of the target organismen, for example genes which lead to faster growth rate of microorganisms or genes which produces stress-, pathogen, or herbicide resistant plants.

**[0228.0.5.8] to [0230.0.5.8]** for the disclosure of the paragraphs [0228.0.5.8] to [0230.0.5.8] see paragraphs [0228.0.0.0] to [0230.0.0.0] above.

**[0231.0.8.8]** In a further advantageous embodiment of the process of the invention, the organisms used in the process are those in which simultaneously a palmitic acid degrading protein is attenuated, in particular by reducing the rate of expression of the corresponding gene.

**[0232.0.0.8] to [0276.0.0.8]** for the disclosure of the paragraphs [0232.0.0.8] to [0276.0.0.8] see paragraphs [0232.0.0.0] to [0276.0.0.0] above.

**[0277.0.5.8]** for the disclosure of this paragraph see paragraph [0277.0.5.5] above.

**[0278.0.0.8] to [0282.0.0.8]** for the disclosure of the paragraphs [0278.0.0.8] to [0282.0.0.8] see paragraphs [0278.0.0.0] to [0282.0.0.0] above.

**[0283.0.8.8]** Moreover, native polypeptide conferring the increase of the respective fine chemical in an organism or part thereof can be isolated from cells (e.g., endothelial cells), for example using the antibody of the present invention as described below, in particular, an antibody against polypeptides as shown in Table II, columns 5 or 7, lines 82 to 88 and 463 to 467 or an antigenic part thereof, which can be produced by standard techniques utilizing polypeptides comprising or consisting of abovementioned sequences, e.g. the polypeptid of the present invention or fragment thereof. Preferred are monoclonal antibodies specifically binding to polypeptides as indicated in Table II, columns 5 or 7, lines 82 to 88 and 463 to 467.

**[0284.0.0.8]** for the disclosure of this paragraph see [0284.0.0.0] above.

**[0285.0.8.8]** In one embodiment, the present invention relates to a polypeptide having the sequence shown in Table II, columns 5 or 7, lines 82 to 88 and 463 to 467 or as coded by the nucleic acid molecule shown in Table I, columns 5 or 7, lines 82 to 88 and 463 to 467 or functional homologues thereof.

**[0286.0.8.8]** In one advantageous embodiment, in the method of the present invention the activity of a polypeptide is increased which comprises or consists of a consensus sequence as indicated in Table IV, column 7, lines 82 to 88 and 463 to 467 and in one another embodiment, the present invention relates to a polypeptide comprising or consisting of a consensus sequence as indicated in Table IV, column 7, lines 82 to 88 and 463 to 467, whereby 20 or less, preferably 15 or 10, preferably 9, 8, 7, or 6, more preferred 5 or 4, even more preferred 3, even more preferred 2, even more preferred 1, most preferred 0 of the amino acids positions indicated can be replaced by any amino acid or, in an further embodiment, can be replaced and/or absent. In one embodiment, the present invention relates to the method of the present invention comprising a poylpeptide or to a polypeptide comprising more than one consensus sequences as indicated in Table IV, column 7, lines 82 to 88 and 463 to 467.

**[0287.0.0.8] to [0290.0.0.8]** for the disclosure of the paragraphs [0287.0.0.8] to [0290.0.0.8] see paragraphs [0287.0.0.0] to [0290.0.0.0] above.

**[0291.0.8.8]** In one advantageous embodiment, the method of the present invention comprises the increasing of a polypeptide comprising or consisting of plant or microorganism specific consensus sequences. Accordingly, in one embodiment, the present invention relates to a polypeptide comprising or consisting of a plant or microorganism specific consensus sequences.
In one embodiment, said polypeptide of the invention distinguishes over the sequence as indicated in Table IIA or IIB, columns 5 or 7, lines 82 to 88 and 463 to 467 by one or more amino acids. In one embodiment, polypeptide distinguishes form the sequence shown in Table IIA or IIB, columns 5 or 7, lines 82 to 88 and 463 to 467 by more than 5, 6, 7, 8 or 9 amino acids, preferably by more than 10, 15, 20, 25 or 30 amino acids, evenmore preferred are more than 40, 50, or 60 amino acids and, preferably, the sequence of the polypeptide of the invention distinguishes from the sequence shown in Table IIA or IIB, columns 5 or 7, lines 82 to 88 and 463 to 467 by not more than 80% or 70% of the amino acids, preferably not more than 60% or 50%, more preferred not more than 40% or 30%, even more preferred not more than 20% or 10%. In another embodiment, said polypeptide of the invention does not consist of the sequence shown in Table IIA or IIB, columns 5 or 7, lines 82 to 88 and 463 to 467.

**[0292.0.0.8]** for the disclosure of this paragraph see [0292.0.0.0] above.

**[0293.0.8.8]** In one embodiment, the invention relates to polypeptide conferring an increase in the respective fine chemical in an organism or part being encoded by the nucleic acid molecule of the invention or by a nucleic acid molecule used in the process.
In one embodiment, the polypeptide of the invention has a sequence, which distinguishes from the sequence as shown in Table IIA or IIB, columns 5 or 7, lines 82 to 88 and 463 to 467 by one or more amino acids. In another embodiment, said polypeptide of the invention does not consist of the sequence shown in Table IIA or IIB, columns 5 or 7, lines 82 to 88 and 463 to 467. In a further embodiment, said polypeptide of the present invention is less than 100%, 99,999%, 99,99%, 99,9% or 99% identical. In one embodiment, said polypeptide does not consist of the sequence encoded by the nucleic acid molecules shown in Table IA or IB, columns 5 or 7, lines 82 to 88 and 463 to 467.

**[0294.0.8.8]** In one embodiment, the present invention relates to a polypeptide having an activity of a protein as indicated in Table II, column 3, lines 82 to 88 and 463 to 467, which distinguishes over the sequence as indicated in Table IIA or IIB, columns 5 or 7, lines 82 to 88 and 463 to 467 by one or more amino acids, preferably by more than 5, 6, 7, 8 or 9 amino acids, preferably by more than 10, 15, 20, 25 or 30 amino acids, evenmore preferred are more than 40, 50, or 60 amino acids but even more preferred by less than 70% of the amino acids, more preferred by less than 50%, even more preferred my less than 30% or 25%, more preferred are 20% or 15%, even more preferred are less than 10%.

**[0295.0.0.8], [0296.0.0.8] and [0297.0.5.8]** for the disclosure of the paragraphs [0295.0.0.8], [0296.0.0.8] and [0297.0.5.8] see paragraphs [0295.0.0.0] to [0297.0.0.0] above.

**[00297.1.0.8]** Non-polypeptide of the invention-chemicals are e.g. polypeptides having not the activity and/or the amino acid sequence of a polypeptide indicated in Table II, columns 3, 5 or 7, lines 82 to 88 and 463 to 467.

**[0298.0.8.8]** A polypeptide of the invention can participate in the process of the present invention. The polypeptide or a portion thereof comprises preferably an amino acid sequence which is sufficiently homologous to an amino acid sequence as indicated in Table II, columns 5 or 7, lines 82 to 88 and 463 to 467 such that the protein or portion thereof maintains the ability to confer the activity of the present invention. The portion of the protein is preferably a biologically active portion as described herein. Preferably, the polypeptide used in the process of the invention has an amino acid sequence identical to a sequence as indicated in Table II, columns 5 or 7, lines 82 to 88 and 463 to 467.

**[0299.0.8.8]** Further, the polypeptide can have an amino acid sequence which is encoded by a nucleotide sequence which hybridizes, preferably hybridizes under stringent conditions as described above, to a nucleotide sequence of the nucleic acid molecule of the present invention. Accordingly, the polypeptide has an amino acid sequence which is encoded by a nucleotide sequence that is at least about 35%, 40%, 45%, 50%, 55%, 60%, 65% or 70%, preferably at least about 75%, 80%, 85% or 90, and more preferably at least about 91%, 92%, 93%, 94% or 95%, and even more preferably at least about 96%, 97%, 98%, 99% or more homologous to one of the amino acid sequences of Table II, columns 5 or 7, lines 82 to 88 and 463 to 467. The preferred polypeptide of the present invention preferably possesses at least one of the activities according to the invention and described herein. A preferred polypeptide of the present invention includes an amino acid sequence encoded by a nucleotide sequence which hybridizes, preferably hybridizes under stringent conditions, to a nucleotide sequence of Table I, columns 5 or 7, lines 82 to 88 and 463 to 467 or which is homologous thereto, as defined above.

**[0300.0.8.8]** Accordingly the polypeptide of the present invention can vary from a sequence as indicated in Table II, columns 5 or 7, lines 82 to 88 and 463 to 467 in amino acid sequence due to natural variation or mutagenesis, as described in detail herein. Accordingly, the polypeptide comprise an amino acid sequence which is at least about 35%, 40%, 45%, 50%, 55%, 60%, 65% or 70%, preferably at least about 75%, 80%, 85% or 90, and more preferably at least about 91%, 92%, 93%, 94% or 95%, and most preferably at least about 96%, 97%, 98%, 99% or more homologous to an entire amino acid sequence of a sequence as indicated in Table IIA or IIB, columns 5 or 7, lines 82 to 88 and 463 to 467.

**[0301.0.0.8]** for the disclosure of this paragraph see [0301.0.0.0] above.

**[0302.0.8.8]** Biologically active portions of an polypeptide of the present invention include peptides comprising amino acid sequences derived from the amino acid sequence of the polypeptide of the present invention or used in the process of the present invention, e.g., an amino acid sequence shown in Table II, columns 5 or 7, lines 82 to 88 and 463 to 467 or the amino acid sequence of a protein homologous thereto, which include fewer amino acids than a full length polypeptide of the present invention or used in the process of the present invention or the full length protein which is homologous to an polypeptide of the present invention or used in the process of the present invention depicted herein, and exhibit at least one activity of polypeptide of the present invention or used in the process of the present invention.

**[0303.0.0.8]** for the disclosure of this paragraph see [0303.0.0.0] above.

**[0304.0.8.8]** Manipulation of the nucleic acid molecule of the invention or the nucleic acid molecule used in the method of the invention may result in the production of a protein having essentially the activity of the polypeptides as indicated in Table II, columns 5 or 7, lines 82 to 88 and 463 to 467 but having differences in the sequence from said wild-type protein. These proteins may be improved in efficiency or activity, may be present in greater numbers in the cell than is usual, or may be decreased in efficiency or activity in relation to the wild type protein.

**[0305.0.5.8], [0306.0.5.8] and [0306.1.0.8]** for the disclosure of the paragraphs [0305.0.5.8], [0306.0.5.8] and [0306.1.0.8] see paragraphs [0305.0.0.0], [0306.0.0.0] and [0306.1.0.0] above.

**[0307.0.0.8] and [0308.0.0.8]** for the disclosure of the paragraphs [0307.0.0.8] and [0308.0.0.8] see paragraphs [0307.0.0.0 and [0308.0.0.0] above.

**[0309.0.8.8]** In one embodiment, a reference to protein (= polypeptide)" of the invention e.g. as indicated in Table II, columns 5 or 7, lines 82 to 88 and 463 to 467 refers to a polypeptide having an amino acid sequence corresponding to the polypeptide of the invention or used in the process of the invention, whereas a "non-polypeptide" or "other polypeptide" e.g. not indicated in Table II, columns 5 or 7, lines 82 to 88 and 463 to 467 refers to a polypeptide having an amino acid sequence corresponding to a protein which is not substantially homologous to a polypeptide of the invention, preferably which is not substantially homologous to a polypeptide having a protein activity, e.g., a protein which does not confer the activity described herein and which is derived from the same or a different organism.

**[0310.0.0.8] to [0334.0.0.8]** for the disclosure of the paragraphs [0310.0.0.8] to [0334.0.0.8] see paragraphs [0310.0.0.0] to [0334.0.0.0] above.

**[0335.0.8.8]** The dsRNAi method has proved to be particularly effective and advantageous for reducing the expression of a nucleic acid sequence as indicated in Table I, columns 5 or 7, lines 82 to 88 and 463 to 467 and/or homologs thereof. As described inter alia in WO 99/32619, dsRNAi approaches are clearly superior to traditional antisense approaches. The invention therefore furthermore relates to double-stranded RNA molecules (dsRNA molecules) which, when introduced into an organism, advantageously into a plant (or a cell, tissue, organ or seed derived therefrom), bring about altered metabolic activity by the reduction in the expression of a nucleic acid sequences as indicated in Table I, columns 5 or 7, lines 82 to 88 and 463 to 467 and/or homologs thereof. In a double-stranded RNA molecule for reducing the expression of an protein encoded by a nucleic acid sequence of one of the sequences as indicated in Table I, columns 5 or 7, lines 82 to 88 and 463 to 467 and/or homologs thereof, one of the two RNA strands is essentially identical to at least part of a nucleic acid sequence, and the respective other RNA strand is essentially identical to at least part of the complementary strand of a nucleic acid sequence.

**[0336.0.0.8] to [0342.0.0.8]** for the disclosure of the paragraphs [0336.0.0.8] to [0342.0.0.8] see paragraphs [0336.0.0.0] to [0342.0.0.0] above.

**[0343.0.8.8]** As has already been described, 100 % sequence identity between the dsRNA and a gene transcript of a nucleic acid sequence as indicated in Table I, columns 5 or 7, lines 82 to 88 and 463 to 467 or it's homolog is not necessarily required in order to bring about effective reduction in the expression. The advantage is, accordingly, that the method is tolerant with regard to sequence deviations as may be present as a consequence of genetic mutations, polymorphisms or evolutionary divergences. Thus, for example, using the dsRNA, which has been generated starting from a sequence of one of the sequences as indicated in Table I, columns 5 or 7, lines 82 to 88 and 463 to 467 or homologs thereof of the one organism, may be used to suppress the corresponding expression in another organism.

**[0344.0.0.8] to [0350.0.0.8], [0351.0.5.8] and [0352.0.0.8] to [0361.0.0.8]** for the disclosure of the paragraphs [0344.0.0.8] to [0350.0.0.8], [0351.0.5.8] and [0352.0.0.8] to [0361.0.0.8] see paragraphs [0344.0.0.0] to [0361.0.0.0] above.

**[0362.0.8.8]** Accordingly the present invention relates to any cell transgenic for any nucleic acid characterized as part of the invention, e.g. conferring the increase of the respective fine chemical in a cell or an organism or a part thereof, e.g. the nucleic acid molecule of the invention or the nucleic acid molecule used in the method of the invention, the nucleic acid construct of the invention, the antisense molecule of the invention, the vector of the invention or a nucleic acid molecule encoding the polypeptide of the invention, e.g. encoding a polypeptide having an activity of a protein such as the polypeptides as indicated in Table II, column 3, lines 82 to 88 and 463 to 467. Due to the above mentioned activity the fine chemical content in a cell or an organism is increased. For example, due to modulation or manupulation, the cellular activity is increased, e.g. due to an increased expression or specific activity of the subject matters of the invention in a cell or an organism or a part thereof. Transgenic for a polypeptide having an activity of a protein such as the polypeptides as indicated in Table II, column 3, lines 82 to 88 and 463 to 467. Activity means herein that due to modulation or manipulation of the genome, the activity of polypeptide having an activity of a protein such as the polypeptides as indicated in Table II, column 3, lines 82 to 88 and 463 to 467 or a similar activity, which is increased in the cell or organism or part thereof. Examples are described above in context with the process of the invention.

**[0363.0.0.8], [0364.0.5.8] and [0365.0.0.8] to [0379.0.5.8]** for the disclosure of the paragraphs [0363.0.0.8], [0364.0.5.8] and [0365.0.0.8] to [0379.0.5.8] see paragraphs [0363.0.0.0] to [0379.0.0.0] above.

**[0380.0.5.8], [0381.0.0.8] and [0382.0.0.8]** for the disclosure of the paragraphs [0380.0.5.8], [0381.0.0.8] and [0382.0.0.8] see paragraphs [0380.0.5.5], [0381.0.0.0] and [0382.0.0.0] above.

**[0383.0.5.8], [0384.0.0.8], [0385.0.5.8] and [0386.0.5.8]** for the disclosure of the paragraphs [0383.0.5.8], [0384.0.0.8], [0385.0.5.8] and [0386.0.5.8] see paragraphs [0383.0.5.5], [0384.0.0.0], [0385.0.5.5] and [0386.0.5.5] above.

**[0387.0.0.8] to [0392.0.0.8]** for the disclosure of the paragraphs [0387.0.0.8] to [0392.0.0.8] see paragraphs [0387.0.0.0] to [0392.0.0.0] above.

[0393.0.8.8] In one embodiment, the present invention relates to a method for the identification of a gene product conferring an increase in the fine chemical production in a cell, comprising the following steps:
(a) contacting, e.g. hybridising, the nucleic acid molecules of a sample, e.g. cells, tissues, plants or microorganisms or a nucleic acid library , which can contain a candidate gene encoding a gene product conferring an increase in the respective fine chemical after expression, with the nucleic acid molecule of the present invention;
(b) identifying the nucleic acid molecules, which hybridize under relaxed stringent conditions with the nucleic acid molecule of the present invention in particular to the nucleic acid molecule sequence shown in Table I, columns 5 or 7, lines 82 to 88 and 463 to 467, preferably in Table I B, columns 5 or 7, lines 82 to 88 and 463 to 467 and, optionally, isolating the full length cDNA clone or complete genomic clone;
(c) introducing the candidate nucleic acid molecules in host cells, preferably in a plant cell or a microorganism, appropriate for producing the respective fine chemical;
(d) expressing the identified nucleic acid molecules in the host cells;
(e) assaying the respective fine chemical level in the host cells; and
(f) identifying the nucleic acid molecule and its gene product which expression confers an increase in the respective fine chemical level in the host cell after expression compared to the wild type.

**[0394.0.0.8] to [0415.0.0.8] and [0416.0.5.8]** for the disclosure of the paragraphs [0394.0.0.8] to [0415.0.0.8] and [0416.0.5.8] see paragraphs [0394.0.0.0] to [0416.0.0.0] above.

**[0417.0.5.8] and [0418.0.0.8] to [0430.0.0.8]** for the disclosure of the paragraphs [0417.0.5.8] and [0418.0.0.8] to [0430.0.0.8] see paragraphs [0417.0.5.5] and [0418.0.0.0] to [0430.0.0.0] above.

**[0431.0.5.8], [0432.0.5.8], [0433.0.0.8] and [0434.0.0.8]** for the disclosure of the paragraphs [0431.0.5.8] , [0432.0.5.8], [0433.0.0.8] and [0434.0.0.8] see paragraphs [0431.0.0.0] to [0434.0.0.0] above.

**[0435.0.5.8] to [0440.0.5.8]** for the disclosure of the paragraphs [0435.0.5.8] to [0440.0.5.8] see paragraphs [0435.0.5.5] to [0440.0.5.5] above.

**[0441.0.0.8] and [0442.0.5.8]** for the disclosure of the paragraphs [0441.0.0.8] and [0442.0.5.8] see [0441.0.0.0] and [0442.0.5.5] above.

**[0443.0.0.8]** for the disclosure of this paragraph see [0443.0.0.0] above.

**[0444.0.5.8] and [0445.0.5.8]** for the disclosure of the paragraphs [0444.0.5.8] and [0445.0.5.8] see [0444.0.5.5] and [0445.0.5.5] above.

**[0446.0.0.8] to [0453.0.0.8]** for the disclosure of the paragraphs [0446.0.0.8] to [0453.0.0.8] see paragraphs [0446.0.0.0] to [0453.0.0.0] above.

**[0454.0.5.8] and [0455.0.5.8]** for the disclosure of the paragraphs [0454.0.5.8] and [0455.0.5.8] see [0454.0.5.5] and [0455.0.5.5] above.

**[0456.0.0.8]** for the disclosure of this paragraph see [0456.0.0.0] above.

**[0457.0.5.8] to [0460.0.5.8]** for the disclosure of the paragraphs [0457.0.5.8] to [0460.0.6.8] see paragraphs [0457.0.5.5] to [0460.0.5.5] above.

**[0461.0.8.8]** Example 10: Cloning SEQ ID NO: 7435 for the expression in plants

**[0462.0.0.8]** for the disclosure of this paragraph see [0462.0.0.0] above.

**[0463.0.8.8]** SEQ ID NO: 7435 is amplified by PCR as described in the protocol of the Pfu Turbo or DNA Herculase polymerase (Stratagene).

**[0464.0.5.8], [0465.0.5.8] and [0466.0.0.8]** for the disclosure of the paragraphs [0464.0.5.8], [0465.0.5.8] and [0466.0.0.8] see paragraphs [0464.0.5.5], [0465.0.5.5] and [0466.0.0.0] above.

**[0467.0.8.8]** The following primer sequences were selected for the gene SEQ ID NO: 7435:
i) forward primer (SEQ ID NO: 7569)
   atggagacca atttttcctt cgact
ii) reverse primer (SEQ ID NO: 7570)
   ctattgaaat accggcttca atattt

**[0468.0.0.8] to [0479.0.0.8]** for the disclosure of the paragraphs [0468.0.0.8] to [0479.0.0.8] see paragraphs [0468.0.0.0] to [0479.0.0.0] above.

**[0480.0.8.8]** Example 11: Generation of transgenic plants which express SEQ ID NO: 7435

**[0481.0.0.8]** for the disclosure of this paragraph see [0481.0.0.0] above.

**[0482.0.0.8] to [0513.0.0.8]** for the disclosure of the paragraphs [0482.0.0.8] to [0513.0.0.8] see paragraphs [0482.0.0.0] to [0513.0.0.0] above.

**[0514.0.8.8]** As an alternative, the amino acids can be detected advantageously via HPLC separation in ethanolic extract as described by Geigenberger et al. (Plant Cell & Environ, 19, 1996: 43-55).
The results of the different plant analyses can be seen from the table, which follows:

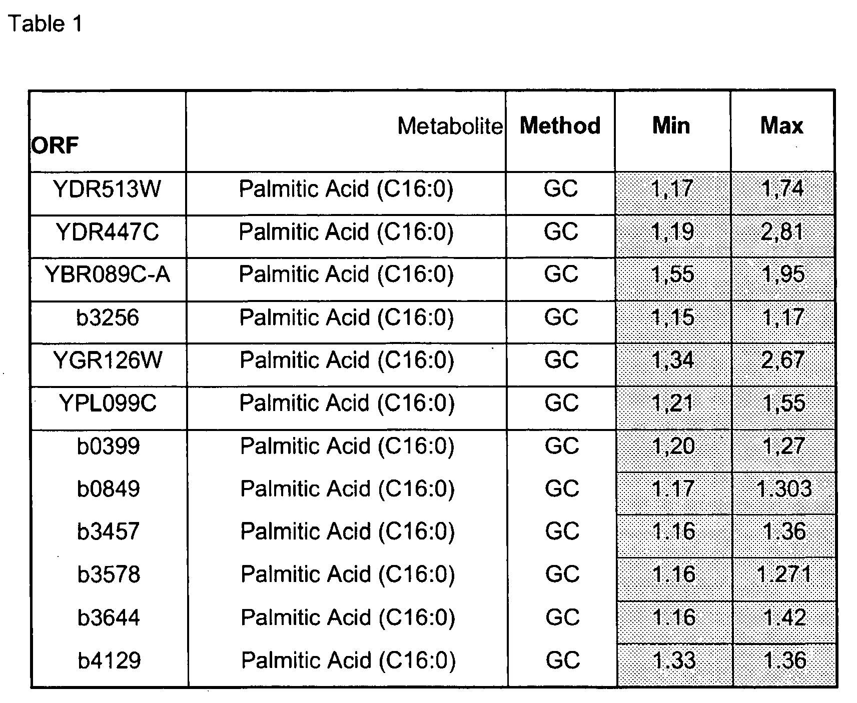

**[0515.0.5.8]** Column 2 shows the fatty acid analyzed. Columns 4 and 5 shows the ratio of the analyzed fatty acid between the transgenic plants and the wild type; Increase of the metabolites: Max: maximal x-fold (normalised to wild type)-Min: minimal x-fold (normalised to wild type). Decrease of the metabolites: Max: maximal x-fold (normalised to wild type) (minimal decrease), Min: minimal x-fold (normalised to wild type) (maximal decrease). Column 3 indicates the analytical method.

**[0516.0.0.8] and [0517.0.5.8]** for the disclosure of the paragraphs [0516.0.0.8] and [0517.0.5.8] see paragraphs [0516.0.0.0] and [0517.0.0.0] above.

**[0518.0.0.8] to [0529.0.0.8] and [0530.0.5.8]** for the disclosure of the paragraphs [0518.0.0.8] to [0529.0.0.8] and [0530.0.5.8] see paragraphs [0518.0.0.0] to [0530.0.0.0] above.

**[0530.1.0.8] to [0530.6.0.8]** for the disclosure of the paragraphs [0530.1.0.8] to [0530.6.0.8] see paragraphs [0530.1.0.0] to [0530.6.0.0] above.

**[0531.0.0.8] to [0533.0.0.8] and [0534.0.5.8]** for the disclosure of the paragraphs [0531.0.0.8] to [0533.0.0.8] and [0534.0.5.8] see paragraphs [0531.0.0.0] to [0534.0.0.0] above.

**[0535.0.0.8] to [0537.0.0.8] and [0538.0.5.8]** for the disclosure of the paragraphs [0535.0.0.8] to [0537.0.0.8] and [0538.0.5.8] see paragraphs [0535.0.0.0] to [0538.0.0.0] above.

**[0539.0.0.8] to [0542.0.0.8] and [0543.0.5.8]** for the disclosure of the paragraphs [0539.0.0.8] to [0542.0.0.8] and [0543.0.5.8] see paragraphs [0539.0.0.0] to [0543.0.0.0] above.

**[0544.0.0.8] to [0547.0.0.8] and [0548.0.5.8] to [0552.0.0.8]** for the disclosure of the paragraphs [0544.0.0.8] to [0547.0.0.8] and [0548.0.5.8] to [0552.0.0.8] see paragraphs [0544.0.0.0] to [0552.0.0.0] above.

**[0552.2.0.8]** for the disclosure of this paragraph see [0552.2.0.0] above.

**[0553.0.8.8]**
1. A process for the production of palmitic acid, which comprises
   (a) increasing or generating the activity of a protein as indicated in Table II, columns 5 or 7, lines 82 to 88 and 463 to 467 or a functional equivalent thereof in a non-human organism, or in one or more parts thereof; and
   (b) growing the organism under conditions which permit the production of palmitic acid in said organism.
2. A process for the production of palmitic acid, comprising the increasing or generating in an organism or a part thereof the expression of at least one nucleic acid molecule comprising a nucleic acid molecule selected from the group consisting of:
   a) nucleic acid molecule encoding of a polypeptide as indicated in Table II, columns 5 or 7, lines 82 to 88 and 463 to 467 or a fragment thereof, which confers an increase in the amount of palmitic acid in an organism or a part thereof;
   b) nucleic acid molecule comprising of a nucleic acid molecule as indicated in Table I, columns 5 or 7, lines 82 to 88 and 463 to 467;
   c) nucleic acid molecule whose sequence can be deduced from a polypeptide sequence encoded by a nucleic acid molecule of (a) or (b) as a result of the degeneracy of the genetic code and conferring an increase in the amount of palmitic acid in an organism or a part thereof;
   d) nucleic acid molecule which encodes a polypeptide which has at least 50% identity with the amino acid sequence of the polypeptide encoded by the nucleic acid molecule of (a) to (c) and conferring an increase in the amount of palmitic acid in an organism or a part thereof;
   e) nucleic acid molecule which hybidizes with a nucleic acid molecule of (a) to (c) under stringent hybridisation conditions and conferring an increase in the amount of palmitic acid in an organism or a part thereof;
   f) nucleic acid molecule which encompasses a nucleic acid molecule which is obtained by amplifying nucleic acid molecules from a cDNA library or a genomic library using the primers or primer pairs as indicated in Table III, column 7, lines 82 to 88 and 463 to 467 and conferring an increase in the amount of palmitic acid in an organism or a part thereof;
   g) nucleic acid molecule encoding a polypeptide which is isolated with the aid of monoclonal antibodies against a polypeptide encoded by one of the nucleic acid molecules of (a) to (f) and conferring an increase in the amount of palmitic acid in an organism or a part thereof;
   h) nucleic acid molecule encoding a polypeptide comprising a consensus sequence as indicated in Table IV, column 7, lines 82 to 88 and 463 to 467 and conferring an increase in the amount of palmitic acid in an organism or a part thereof; and
   i) nucleic acid molecule which is obtainable by screening a suitable nucleic acid library under stringent hybridization conditions with a probe comprising one of the sequences of the nucleic acid molecule of (a) to (k) or with a fragment thereof having at least 15 nt, preferably 20 nt, 30 nt, 50 nt, 100 nt, 200 nt or 500 nt of the nucleic acid molecule characterized in (a) to (k) and conferring an increase in the amount of palmitic acid in an organism or a part thereof.
   or comprising a sequence which is complementary thereto.
3. The process of claim 1 or 2, comprising recovering of the free or bound palmitic acid.
4. The process of any one of claim 1 to 3, comprising the following steps:
   (a) selecting an organism or a part thereof expressing a polypeptide encoded by the nucleic acid molecule characterized in claim 2;
   (b) mutagenizing the selected organism or the part thereof;
   (c) comparing the activity or the expression level of said polypeptide in the mutagenized organism or the part thereof with the activity or the expression of said polypeptide of the selected organisms or the part thereof;
   (d) selecting the mutated organisms or parts thereof, which comprise an increased activity or expression level of said polypeptide compared to the selected organism or the part thereof;
   (e) optionally, growing and cultivating the organisms or the parts thereof; and
   (f) recovering, and optionally isolating, the free or bound palmitic acid produced by the selected mutated organisms or parts thereof.
5. The process of any one of claims 1 to 4, wherein the activity of said protein or the expression of said nucleic acid molecule is increased or generated transiently or stably.
6. An isolated nucleic acid molecule comprising a nucleic acid molecule selected from the group consisting of:
   a) nucleic acid molecule encoding of a polypeptide as indicated in Table II, columns 5 or 7, lines 82 to 88 and 463 to 467 or a fragment thereof, which confers an increase in the amount of palmitic acid in an organism or a part thereof;
   b) nucleic acid molecule comprising of a nucleic acid molecule as indicated in Table I, columns 5 or 7, lines 82 to 88 and 463 to 467;
   c) nucleic acid molecule whose sequence can be deduced from a polypeptide sequence encoded by a nucleic acid molecule of (a) or (b) as a result of the degeneracy of the genetic code and conferring an increase in the amount of palmitic acid in an organism or a part thereof;
   d) nucleic acid molecule which encodes a polypeptide which has at least 50% identity with the amino acid sequence of the polypeptide encoded by the nucleic acid molecule of (a) to (c) and conferring an increase in the amount of palmitic acid in an organism or a part thereof;
   e) nucleic acid molecule which hybidizes with a nucleic acid molecule of (a) to (c) under stringent hybridisation conditions and conferring an increase in the amount of palmitic acid in an organism or a part thereof;
   f) nucleic acid molecule which encompasses a nucleic acid molecule which is obtained by amplifying nucleic acid molecules from a cDNA library or a genomic library using the primers or primer pairs as indicated in Table III, column 7, lines 82 to 88 and 463 to 467 and conferring an increase in the amount of palmitic acid in an organism or a part thereof;
   g) nucleic acid molecule encoding a polypeptide which is isolated with the aid of monoclonal antibodies against a polypeptide encoded by one of the nucleic acid molecules of (a) to (f) and conferring an increase in the amount of palmitic acid in an organism or a part thereof;
   h) nucleic acid molecule encoding a polypeptide comprising a consensus sequence as indicated in Table IV, column 7, lines 82 to 88 and 463 to 467 and conferring an increase in the amount of palmitic acid in an organism or a part thereof; and
   i) nucleic acid molecule which is obtainable by screening a suitable nucleic acid library under stringent hybridization conditions with a probe comprising one of the sequences of the nucleic acid molecule of (a) to (k) or with a fragment thereof having at least 15 nt, preferably 20 nt, 30 nt, 50 nt, 100 nt, 200 nt or 500 nt of the nucleic acid molecule characterized in (a) to (k) and conferring an increase in the amount of palmitic acid in an organism or a part thereof.
   whereby the nucleic acid molecule distinguishes over the sequence as indicated in Table I A, columns 5 or 7, lines 82 to 88 and 463 to 467 by one or more nucleotides.
7. A nucleic acid construct which confers the expression of the nucleic acid molecule of claim 6, comprising one or more regulatory elements.
8. A vector comprising the nucleic acid molecule as claimed in claim 6 or the nucleic acid construct of claim 7.
9. The vector as claimed in claim 8, wherein the nucleic acid molecule is in operable linkage with regulatory sequences for the expression in a prokaryotic or eukaryotic, or in a prokaryotic and eukaryotic, host.
10. A host cell, which has been transformed stably or transiently with the vector as claimed in claim 8 or 9 or the nucleic acid molecule as claimed in claim 6 or the nucleic acid construct of claim 7 or produced as described in claim any one of claims 2 to 5.
11. The host cell of claim 10, which is a transgenic host cell.
12. The host cell of claim 10 or 11, which is a plant cell, an animal cell, a microorganism, or a yeast cell, a fungus cell, a prokaryotic cell, an eukaryotic cell or an archaebacterium.
13. A process for producing a polypeptide, wherein the polypeptide is expressed in a host cell as claimed in any one of claims 10 to 12.
14. A polypeptide produced by the process as claimed in claim 13 or encoded by the nucleic acid molecule as claimed in claim 6 whereby the polypeptide distinguishes over a sequence as indicated in Table II A, columns 5 or 7, lines 82 to 88 and 463 to 467 by one or more amino acids
15. An antibody, which binds specifically to the polypeptide as claimed in claim 14.
16. A plant tissue, propagation material, harvested material or a plant comprising the host cell as claimed in claim 12 which is plant cell or an Agrobacterium.
17. A method for screening for agonists and antagonists of the activity of a polypeptide encoded by the nucleic acid molecule of claim 6 conferring an increase in the amount of palmitic acid in an organism or a part thereof comprising:
   (a) contacting cells, tissues , plants or microorganisms which express the a polypeptide encoded by the nucleic acid molecule of claim 6 conferring an increase in the amount of palmitic acid in an organism or a part thereof with a candidate compound or a sample comprising a plurality of compounds under conditions which permit the expression the polypeptide;
   (b) assaying the palmitic acid level or the polypeptide expression level in the cell, tissue, plant or microorganism or the media the cell, tissue, plant or microorganisms is cultured or maintained in; and
   (c) identifying a agonist or antagonist by comparing the measured palmitic acid level or polypeptide expression level with a standard of palmitic acid or polypeptide expression level measured in the absence of said candidate compound or a sample comprising said plurality of compounds, whereby an increased level over the standard indicates that the compound or the sample comprising said plurality of compounds is an agonist and a decreased level over the standard indicates that the compound or the sample comprising said plurality of compounds is an antagonist.
18. A process for the identification of a compound conferring increased palmitic acid production in a plant or microorganism, comprising the steps:
   (a) culturing a plant cell or tissue or microorganism or maintaining a plant expressing the polypeptide encoded by the nucleic acid molecule of claim 6 conferring an increase in the amount of palmitic acid in an organism or a part thereof and a readout system capable of interacting with the polypeptide under suitable conditions which permit the interaction of the polypeptide with dais readout system in the presence of a compound or a sample comprising a plurality of compounds and capable of providing a detectable signal in response to the binding of a compound to said polypeptide under conditions which permit the expression of said readout system and of the polypeptide encoded by the nucleic acid molecule of claim 6 conferring an increase in the amount of palmitic acid in an organism or a part thereof;
   (b) identifying if the compound is an effective agonist by detecting the presence or absence or increase of a signal produced by said readout system.
19. A method for the identification of a gene product conferring an increase in palmitic acid production in a cell, comprising the following steps:
   (a) contacting the nucleic acid molecules of a sample, which can contain a candidate gene encoding a gene product conferring an increase in palmitic acid after expression with the nucleic acid molecule of claim 6;
   (b) identifying the nucleic acid molecules, which hybridise under relaxed stringent conditions with the nucleic acid molecule of claim 6;
   (c) introducing the candidate nucleic acid molecules in host cells appropriate for producing palmitic acid;
   (d) expressing the identified nucleic acid molecules in the host cells;
   (e) assaying the palmitic acid level in the host cells; and
   (f) identifying nucleic acid molecule and its gene product which expression confers an increase in the palmitic acid level in the host cell in the host cell after expression compared to the wild type.
20. A method for the identification of a gene product conferring an increase in palmitic acid production in a cell, comprising the following steps:
   (a) identifiying in a data bank nucleic acid molecules of an organism; which can contain a candidate gene encoding a gene product conferring an increase in the palmitic acid amount or level in an organism or a part thereof after expression, and which are at least 20% homolog to the nucleic acid molecule of claim 6;
   (b) introducing the candidate nucleic acid molecules in host cells appropriate for producing palmitic acid;
   (c) expressing the identified nucleic acid molecules in the host cells;
   (d) assaying the palmitic acid level in the host cells; and
   (e) identifying nucleic acid molecule and its gene product which expression confers an increase in the palmitic acid level in the host cell after expression compared to the wild type.
21. A method for the production of an agricultural composition comprising the steps of the method of any one of claims 17 to 20 and formulating the compound identified in any one of claims 17 to 20 in a form acceptable for an application in agriculture.
22. A composition comprising the nucleic acid molecule of claim 6, the polypeptide of claim 14, the nucleic acid construct of claim 7, the vector of any one of claims 8 or 9, an antagonist or agonist identified according to claim 17, the compound of claim 18, the gene product of claim 19 or 20, the antibody of claim 15, and optionally an agricultural acceptable carrier.
23. Use of the nucleic acid molecule as claimed in claim 6 for the identification of a nucleic acid molecule conferring an increase of palmitic acid after expression.
24. Use of the polypeptide of claim 14 or the nucleic acid construct claim 7 or the gene product identified according to the method of claim 19 or 20 for identifying compounds capable of conferring a modulation of palmitic acid levels in an organism.
25. Food or feed composition comprising the nucleic acid molecule of claim 6, the polypeptide of claim 14, the nucleic acid construct of claim 7, the vector of claim 8 or 9, the antagonist or agonist identified according to claim 17, the antibody of claim 15, the plant or plant tissue of claim 16, the harvested material of claim 16, the host cell of claim 10 to 12 or the gene product identified according to the method of claim 19 or 20.
26. Use of the nucleic acid molecule of claim 6, the polypeptide of claim 14, the nucleic acid construct of claim 7, the vector of claim 8 or 9, the antagonist or agonist identified according to claim 17, the antibody of claim 15, the plant or plant tissue of claim 16, the host cell of claim 10 to 12 or the gene product identified according to the method of claim 19 or 20 for the protection of a plant against a palmitic acid synthesis inhibiting herbicide.

**[0554.0.0.8] Abstract: see [0554.0.0.0]**
Process for the production of fine chemicals

**[0000.0.0.9]** In a further embodiment, the present invention relates to a further process for the production of fine chemicals as defined below and the corresponding embodiments as described herein as follows.

### Description

**[0001.0.0.9]** see **[0001.0.0.0]**

**[0002.0.9.9]** Due to their plastids, plants possess some biosynthetic pathways, which are, besides in cyanobacteria, unique in living organisms. Some plastidic compounds are indispensable for human and animal nutrition and are therefore called vitamins. Two essential lipophilic components for nutrition are provitamin A (beta-carotene) and vitamin E.
Vitamin E is classified by its pharmacological effect and chromanol ring structure and not by biosynthesis. It comprises a class of 8 lipid-soluble components, being subdivided into tocopherols and tocotrienols. While tocopherols share an isoprenoid side chain derived from phytyl-PP, tocotrienol side chains are derivates of geranylgeranyl-PP. The α, β, γ and δ-members of these subclasses differ in their degree of methylation in the 6-chromanol-ring structure.
The tocopherol group (1a-d) has a saturated side chain, and the tocotrienol group (2a-d) has an unsaturated side chain: 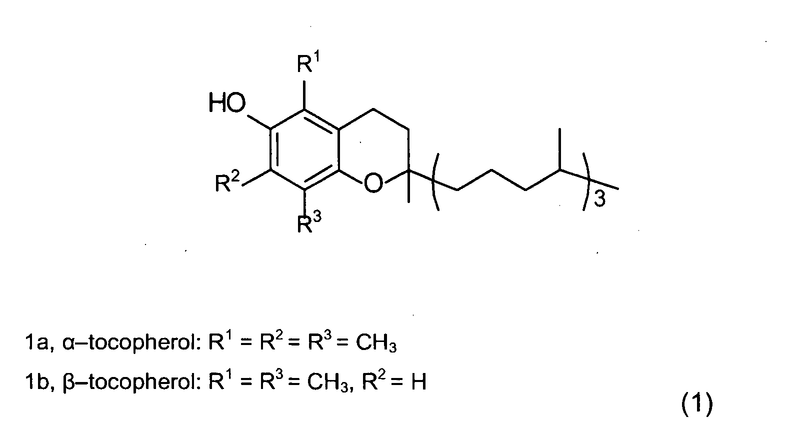 1c, γ-tocopherol: R¹ = H, R² = R³ = CH₃
1 d, -tocopherol: R¹ = R² = H, R³ = CH₃ 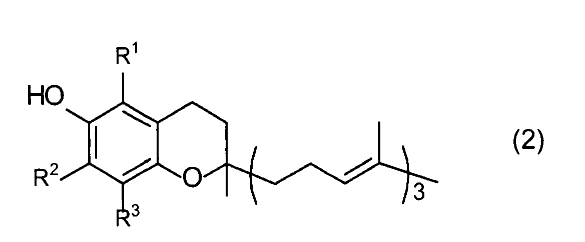 2a, α-tocotrienol: R¹ = R² = R³ = CH₃
2b, β-tocotrienol: R¹ = R³ = CH₃, R² = H
2c, γ-tocotrienol: R¹ = H, R² = R³ = CH₃
2d, -tocotrienol: R¹ = R² = H, R³ = CH₃
In the present invention, vitamin E means all of the aforementioned tocopherols and tocotrienols with vitamin E activity.

**[0003.0.9.9]** The four major forms of tocopherols, α, β, γ, and δ, differ in the position and number of methyl groups. The predominant form in the leaves of higher plants is α-tocopherol, whereas in seeds γ-tocopherol is often the major isoform. Tocopherols predominantly function as antioxidants in vivo in photosynthetic organisms and in animals, as well as in isolated compounds such as oils. The antioxidant properties of tocopherols derive from their ability to quench free radicals and different tocopherols may be optimal as antioxidants for different biological systems. For human and animal utility, α-tocopherol has the highest vitamin E activity and has been implicated in a variety of health areas, including possible benefits in preventing cardiovascular disease, certain cancers, and cataract formation. The amounts of vitamin E needed to achieve these effects are often quite high, 100 to 400 International Units (I.U.) and even up to 800 I.U. compared with the recommended daily allowance of 40 I.U. In fats and oils, tocopherols protect unsaturated fatty acids from oxidation. In these systems, γ-tocopherol appears to have the greater utility. In fact, tocopherols are often included in processed oils to help stabilize the fatty acids. For human health as well as food and feed utility, it is desirable to have plants with increased tocopherol content along with those where the tocopherol composition is customized.

**[0004.0.9.9]** Tocopherols contain an aromatic head group, which is derived from homogentisic acid (HGA) and a hydrocarbon portion, which arises from phytyldiphosphate (phytyl-DP). HGA is derived from the shikimic acid pathway and phytyl-DP is generated from the condensation of four isoprenoid units. The isoprenoid contribution to tocopherol biosynthesis is thought to come primarily from the plastidal methyl-erythritol phosphate pathway, and not the cytosolic mevalonic acid pathway. The condensation of HGA and phytyl-DP to form 2-methyl-6-phytylplastoquinol, the first committed step in tocopherol biosynthesis, is a prenyltransferase reaction that is performed by a homogentisate phytyltransferase (HPT). Subsequent cyclization and methylation reactions result in the formation of the four major tocopherols. The enzymatic reactions in tocopherol biosynthesis were identified 15 to 20 years ago, but cloning of the genes encoding these enzymes has only occurred in the last few years.

**[0005.0.9.9]** Tocopherol biosynthesis takes place in the plastid and the enzymes are associated with the chloroplast envelope. The membrane association of the enzymes has made purification difficult. With the advent of genomics and the availability of complete genome sequences of a number of organisms, including *Synechocystis* sp. PCC 6803 and Arabidopsis, it has become possible to use bioinformatics techniques to identify and clone additional genes in the tocopherol pathway.
The first enzyme cloned in the tocopherol pathway, γ-tocopherol methyl transferase (γ-TMT), was identified in *Synechocystis* sp. PCC 6803 and Arabidopsis using bioinformatics. In that study, the Arabidopsis γ-TMT was shown to alter seed tocopherol composition when overexpressed in Arabidopsis. γ- Tocopherol, normally the predominant tocopherol isomer in Arabidopsis seeds, was almost completely converted to α-tocopherol.
HPT catalyzes the first committed reaction in the tocopherol pathway, and was unidentified previously. Concomitant with this study, *slr*1736 was found to encode a HPT in *Synechocystis* sp. PCC 6803 and the Arabidopsis HTP was identified.
There are prenyltransferases that condense prenyl groups with allylic chains and those that condense prenyl chains with aromatic groups. The prenyltransferases that catalyze sequential condensations of isopentenylpyrophosphate with allylic chains share common features, including Asp-rich motifs, and lead to the formation of compounds with two isoprenoid units, such as geranylpyrophosphate, or to much longer molecules, such as rubber, which contains greater than 1,000 isoprenoid units. Prenyltransferases that catalyze condensations with nonisoprenoid groups have an Asp-rich motif distinct from that of the allylic class, and include *UbiA*, which attaches a prenyl group to 4-hydroxybenzoic acid, and chlorophyll synthase, which attaches a prenyl group to chlorophyllide.

**[0006.0.9.9]** The first committed step in tocopherol biosynthesis is catalyzed by an aromatic prenyltransferase that transfers a phytyl chain to HGA
Classification by head groups would arrange tocopherols, tocotrienols and plastoquinones in one group, being quinones with antioxidant properties and having homogentisic acid as a precursor. Plastoquinones are important components of the quinone-pool in the photosynthetic electron transport chains of plastids, also interfering in the biosynthesis of provitamin A (beta-carotene; Norris SR, (1995). Plant Cell 7, 2139-2149).

**[0007.0.9.9]** Vitamin E is predominantly delivered by the ingestion of vegetable oils. It plays an important role as a membrane-associated antioxidant scavenger. During past years several additional functions of vitamin E as anti-hypercholesterolemic and immunostimulatory agent in humans have been proposed (Beharka (1997). Methods Enzymol. 282, 247-263).
These compounds with vitamin E activity are important natural fat-soluble antioxidants. A vitamin E deficiency leads to pathophysiological situations in humans and animals. Vitamin E compounds therefore are of high economical value as additives in the food and feed sectors, in pharmaceutical formulations and in cosmetic applications.

**[0008.0.9.9]** In plastids of plants many isoprenoid pathways are localized, which are interconnected by their substrates, end products and by regulation. These are, e.g. monoterpene-, diterpene-, giberillic acid-, abscisic acid-, chlorophyll-, phylloquinone-, carotenoid-, tocopherol-, tocotrienol- and plastoquinone-biosynthesis. In all these pathways prenyltransferases are involved in the biosynthesis of these compounds. With respect to the length of their side chains diterpenes, chlorophylls, phylloquinones, tocopherols and tocotrienols can be arranged into a C₂₀-group of isoprenoids. Another classification by degree of desaturation of the side chain, would arrange e.g. chlorophylls, phylloquinones and tocopherols into a phytyl-group and e.g. diterpenes, tocotrienols, plastoquinones and carotenoids into a group of desaturated isoprenoid compounds.

**[0009.0.9.9]** An economical method for producing vitamin E or its precursor and food- and feedstuffs with increased vitamin E content are therefore very important. Particularly economical methods are biotechnological methods utilizing vitamin E-producing organisms which are either natural or optimized by genetic modification.
There is a constant need for providing novel enzyme activities or direct or indirect regulators and thus alternative methods with advantageous properties for producing vitamin E or its precursor in organisms, e.g. in transgenic organisms.
Attempts are known to achieve an increase in the flow of metabolites so as to increase the tocopherol and/or tocotrienol content by overexpressing Phytyl/prenyltransferasegenes in transgenic organisms; WO 00/63391, WO 00/68393, WO 01/62781 and WO 02/33060.

**[0010.0.9.9]** Therefore improving the quality of foodstuffs and animal feeds is an important task of the food-and-feed industry. This is necessary since, for example, Vitamin E, which occur in plants and some microorganisms are limited with regard to the supply of mammals. Especially advantageous for the quality of foodstuffs and animal feeds is as balanced as possible a vitamin profile in the diet since a great excess of some vitamins above a specific concentration in the food has only some or little or no positive effect. A further increase in quality is only possible via addition of further vitamins, which are limiting.

**[0011.0.9.9]** To ensure a high quality of foods and animal feeds, it is therefore necessary to add one or a plurality of vitamins in a balanced manner to suit the organism.

**[0012.0.9.9]** Accordingly, there is still a great demand for new and more suitable genes which encode enzymes which participate in the biosynthesis of vitamins, in particular vitamin E and make it possible to produce them specifically on an industrial scale without unwanted byproducts forming. In the selection of genes for or regulators of biosynthesis two characteristics above all are particularly important. On the one hand, there is as ever a need for improved processes for obtaining the highest possible contents of vitamins like vitamin E; on the other hand as less as possible byproducts should be produced in the production process.

**[0013.0.0.9]** see **[0013.0.0.0]**

**[0014.0.9.9]** Accordingly, in a first embodiment, the invention relates to a process for the production of a fine chemical, whereby the fine chemical is Vitamin E. Accordingly, in the present invention, the term "the fine chemical" as used herein relates to a "Vitamin E". Further, in an other embodiment the term "the fine chemicals" as used herein also relates to compositions of the fine chemicals comprising Vitamin E.

**[0015.0.9.9]** In one embodiment, the term "Vitamin E" or "the fine chemical" or "the respective fine chemical" means at least one chemical compound with vitamin E activity selected from the group "alpha-tocopherol, beta-tocopherol, gamma-tocopherol, delta-tocopherol, alpha-tocotrienol, beta-tocotrienol, gamma-tocotrienol and delta-tocotrienol". In an preferred embodiment, the term "the fine chemical" or the term "Vitamin E" or the term "the respective fine chemical" means at least one chemical compound with vitamin E activity selected from the group "alpha-tocopherol, beta-tocopherol, and gamma-tocopherol".
An increased vitamin E content normally means an increased total vitamin E content. However, an increased vitamin E content also means, in particular, a modified content of the above-described 8 compounds with vitamin E activity, without the need for an inevitable increase in the total vitamin E content. In a preferred embodiment, the term "the fine chemical" means vitamin E in free form or its salts or its ester or bound. In one embodiment, the term "the fine chemical" and the term "the respective fine chemical" mean at least one chemical compound with an activity of the above mentioned fine chemical.

**[0016.0.9.9]** Accordingly, the present invention relates to a process for the production of Vitamin E comprising
(a) increasing or generating the activity of one or more
   - YFL019C, YPL268W, YFL013C, b1829, and/or b1827 - protein(s);
   - b0161, b0970, b3160, b4063, b2699, b0112, and/or b1829 - protein(s);
   - b0785, b3938, YFL053W, b1827, b0986, b1829 and/or b0175 - protein(s); and/or
   - b0175, b0785, b0986, b3838, YFL053W and/or b1829 - protein(s);
   in a non-human organism in one or more parts thereof; and
(b) growing the organism under conditions which permit the production of the fine chemical, thus, vitamin E, in particular alpha-tocopherol, beta-tocopherol, and/or gamma-tocopherol and/or the vitamin E precursor 2,3-Dimethyl-5-pythylquinol, respectively, in said organism.
In particular, the present invention relates to a process for the production of 2,3-Dimethyl-5-phytylquinol, alpha-Tocopherol, beta-Tocopherol, and/or gamma-Tocopherol comprising
(a) increasing or generating the activity of one or more
   - YFL019C, YPL268W, YFL013C, b1829, and/or b1827 - protein(s) for 2,3-Dimethyl-5-phytylquinol;
   - b0161, b0970, b3160, b4063, b2699, b0112, and/or b1829 - protein(s) for alpha-Tocopherol,
   - b0785, b3938, YFL053W, b1827, b0986, b1829 and/or b0175 - protein(s) for beta-Tocopherol; and/or
   - b0175, b0785, b0986, b3838, YFL053W and/or b1829 - protein(s) for gamma-Tocopherol;
   in a non-human organism in one or more parts thereof; and
(b) growing the organism under conditions which permit the production of alpha-tocopherol, beta-tocopherol, and/or gamma-tocopherol or the vitamin E precursor 2,3-Dimethyl-5-pythylquinol, respectively, in said organism.
Accordingly, the present invention relates to a process for the production of vitamin E comprising
(a) increasing or generating the activity of one or more proteins having the activity of a protein indicated in Table II, column 3, lines 89 to 102 or lines 472 to 482 or having the sequence of a polypeptide encoded by a nucleic acid molecule indicated in Table I, column 5 or 7, lines 89 to 102 or lines 472 to 482 , in a non-human organism in one or more parts thereof and
(b) growing the organism under conditions which permit the production of the fine chemical, thus, vitamin E, in particular alpha-tocopherol, beta-tocopherol, and/or gamma-tocopherol and/or the vitamin E precursor 2,3-Dimethyl-5-pythylquinol, resp.
Particularly, the present invention relates to a process for the production of vitamin E comprising
(a) increasing or generating the activity of one or more proteins having the activity of a protein indicated in
   - Table II, column 3, lines 89 to 92 or 482 for 2,3-Dimethyl-5-phytylquinol;
   - Table II, column 3, lines 93 to 95 or 472 to 475 for alpha-Tocopherol;
   - Table II, column 3, lines 96 to 100 or 476 to 477 for beta-Tocopherol; and/or
   - Table II, column 3, lines 101 to 102 or 478 to 481 for gamma-Tocopherol; e.g. having the sequence of a polypeptide encoded by a nucleic acid molecule indicated in
   - Table I, column 5 or 7, lines 89 to 92 or 482 for 2,3-Dimethyl-5-phytylquinol;
   - Table I, column 5 or 7, lines 93 to 95 or 472 to 475 for alpha-Tocopherol;
   - Table I, column 5 or 7, lines 96 to 100 or 476 to 477 for beta-Tocopherol; and/or
   - Table I, column 5 or 7, lines 101 to 102 or 478 to 481 for gamma-Tocopherol; in a non-human organism in one or more parts thereof and
(b) growing the organism under conditions which permit the production of the fine chemical, in particular alpha-tocopherol, beta-tocopherol, and/or gamma-tocopherol and/or the vitamin E precursor 2,3-Dimethyl-5-pythylquinol.

**[0016.1.9.9]** Accordingly, the term "the fine chemical" means in one embodiment "2,3-dimethyl-5-pythylquinol" in relation to all sequences listed in Table I to IV, lines 89 to 92 or 482 or homologs thereof and means in one embodiment "alpha-tocopherol" in relation to all sequences listed in Tables I to IV, lines 93 to 95 or 472 to 475 or homologs thereof and means in one embodiment "beta-tocopherol" in relation to all sequences listed in Table I, lines 96 to 100 or 476 to 477, and means in one embodiment "gamma-tocopherol" in relation to all sequences listed in Table I to IV, lines 101 to 102 or 478 to 481.
In one embodiment, the method of the present invention confers the increase of the content of more than one of the respective fine chemicals, i.e. of alpha-, beta-, gamma-tocopherol or 2,3-dimethyl-5-pythylquinol. Accordingly, in one embodiment the term "the fine chemical" means "2,3-dimethyl-5-phytylquinol", "beta-tocopherol" and "gamma-tocopherol" in relation to all sequences listed in Table I to IV, lines 91, 94, 100 or 102, i.e. the method of the present invention confers the increase of the content of more than one of the respective fine chemicals, i.e. of 2,3-dimethyl-5-phytylquinol , alpha-, beta- and gamma-tocopherol. Preferably the method of the invention confers the increase the content of 2,3-dimethyl-5-phytylquinol, alpha-, beta- and gamma-tocopherol. In one embodiment the term "the fine chemical" means "2,3-dimethyl-5-phytylquinol" and "beta-tocopherol" in relation to all sequences listed in Table I to IV, lines 92 or 97, i.e. the method of the present invention confers the increase of the content of 2,3-dimethyl-5-phytylquinol and beta-tocopherol. In one embodiment the term "the fine chemical" means "beta-tocopherol" and "gamma-tococpherol in relation to all sequences listed in Table I to IV, lines 96, 98, 99, 101, 476, 477, 478, 479, 480, or 481.
Accordingly, the term "the fine chemical" can mean "2,3- dimethyl-5-pythylquinol", "alpha-tocopherol", "beta-tocopherol", and/or "gamma-tocopherol", owing to circumstances and the context. In order to illustrate that the meaning of the term "the fine chemical" means "2,3- dimethyl-5-pythylquinol", "alpha-tocopherol", "beta-tocopherol", and/or "gamma-tocopherol" the term "the respective fine chemical" is also used.

**[0017.0.0.9]** to **[0018.0.0.9]: see [0017.0.0.0]** to **[0018.0.0.0]**

**[0019.0.9.9]** Advantageously the process for the production of the respective fine chemical leads to an enhanced production of the respective fine chemical. The terms "enhanced" or "increase" mean at least a 10%, 20%, 30%, 40% or 50%, preferably at least 60%, 70%, 80%, 90% or 100%, more preferably 150%, 200%, 300%, 400% or 500% higher production of the respective fine chemical in comparison to the reference as defined below, e.g. that means in comparison to an organism without the aforementioned modification of the activity of a protein having the activity of a protein indicated in Table II, column 3, lines 89 to 102 and 472 to 482 or encoded by nucleic acid molecule indicated in Table I, columns 5 or 7, lines 89 to 102 and 472 to 482.

**[0020.0.9.9]** Surprisingly it was found, that the transgenic expression of at least one of the proteins of the Saccharomyces cerevisiae protein(s) YFL019C, YPL268W and/or YFL013C, and/or the Escherichia coli K12 protein(s) b1829, and/or b1827 in Arabidopsis thaliana conferred an increase in the 2,3-dimethyl-5-phytylquinol, which is a precursor in the biosynthesis of vitamin E, in particular of gamma-tocopherol and thus alpha-tocopherol. Thus, an increase in the level of this precursor of the tocopherol biosynthesis can be advantageous for the production of vitamin E. For example, in one embodiment the level of 2,3-dimethyl-5-phytylquinol is increased in combination with the modulation of the expression of other genes of the biosynthesis of vitamin E, in particular of genes encoding enzymes metabolising 2,3-dimethyl-5-phytylquinol to produce vitamin E or a precursor thereof, such as the 2,3-dimethyl-5-phytylquinol-Cyclase and/or gamma-tocopherol-methyltransferase II or with genes encoding the polypeptides indicated in Table II, columns 5 or 7, lines 89 to 102 or 472 to 482.
Surprisingly, it was found that the transgenic expression of the Escherichia coli K12 protein(s) b0161, b0970, b3160, b4063, b2699, b1829, and/or b0112 in Arabidopsis thaliana conferred an increase in the alpha-tocopherol content of the transformed plants.
Surprisingly it was found, that the transgenic expression of the Saccharomyces cerevisiae protein YFL053W and/or of the Escherichia coli K12 protein(s) b0785, b3938, b1827, b1829, b0986, and/or b0175 in Arabidopsis thaliana conferred an increase in the beta-tocopherol content of the transformed plants.
Surprisingly it was found, that the transgenic expression of the Saccharomyces cerevisiae protein YFL053W, and/or the Escherichia coli K12 protein(s) b0175, b0785, b03938, b0986, b1829 in Arabidopsis thaliana conferred an increase in gamma-tocopherol content of the transformed plants.
Accordingly, it was surprisingly found, that the transgenic expression of the Escherichia coli K12 protein b1829 in Arabidopsis thaliana conferred an increase in 2,3-dimethyl-5-phytylquinol, alpha-tocopherol, beta-tocopherol, and/or gamma-tocopherol (or the respective fine chemical) content of the transformed plants. Thus, in one embodiment, said protein or its homologs are used for the production of 2,3-Dimethyl-5-phytylquinol; in one embodiment, said protein or its homologs are used for the production of alpha-tocopherol; in one embodiment, said protein or its homologs are used for the production of beta-tocopherol; in one embodiment, said protein or its homologs are used for the production of gamma-tocopherol; in one embodiment, said protein or its homologs are used for the production of one or more fine chemical selected from the group consisting of: 2,3-Dimethyl-5-phytylquinol, alpha-tocopherol, beta-tocopherol, and gamma-tocopherol.
Accordingly, it was surprisingly found, that the transgenic expression of the Escherichia coli K12 protein b1827 in Arabidopsis thaliana conferred an increase in 2,3-Dimethyl-5-phytylquinol, and/or beta-tocopherol (or the respective fine chemical) content of the transformed plants. Thus, in one embodiment, said protein or its homologs are used for the production of 2,3-Dimethyl-5-phytylquinol; in one embodiment, said protein or its homologs are used for the production of beta-tocopherol; in one embodiment, said protein or its homologs are used for the production of 2,3-Dimethyl-5-phytylquinol and beta-tocopherol.
Surprisingly it was found, that the transgenic expression of the Saccharomyces cerevisiae protein YFL053W and the E. coli protein b0785, b3938, b0986 or b0175 in Arabidopsis thaliana conferred an increase in beta-tocopherol and/or gamma-tocopherol (or the respective fine chemical) content of the transformed plants. Thus, in one embodiment, said protein or its homologs are used for the production of beta-tocopherol; in one embodiment, said protein or its homologs are used for the production of gamma-tocopherol, in one embodiment, said protein or its homologs are used for the production of beta-tocopherol and gamma-tocopherol.

**[0021.0.0.9]** see **[0021.0.0.0]**

**[0022.0.9.9]** The sequence of b1829 from Escherichia coli K12 has been published in Blattner et al., Science 277(5331), 1453-1474, 1997, and its activity is being defined as a heat shock protein with protease activity. Accordingly, in one embodiment, the process of the present invention comprises the use of a protein of the stress response, the pheromone response, the mating type determination, protein modification or having a proteolytic degradation activity or being a sex specific protein, in particular the use of a protease, in particular of a heat shock protein with protease activity, preferably of the superfamily of the heat shock protein htpX, e.g. as shown herein, for the production of the respective fine chemical, meaning of vitamin E, e.g. alpha-tocopherol, beta-tocopherol and/or gamma-tocopherol, e.g. via increasing of the vitamin E biosynthesis precursor 2,3-dimethyl-5-phytylquinol, preferably in free or bound form, in an organism or a part thereof, as mentioned. In one further embodiment the b1829 protein expression is increased together with the increase of another gene of the vitamin E biosynthesis pathway, preferably with a gene encoding a protein being involved in the production of vitamin E via transformation of the intermediate 2,3-dimethyl-5-phytylquiol. In one embodiment, in the process of the present invention the activity of a protease, in particular of a heat shock protein with protease activity, preferably of the superfamily of the heat shock protein htpX, is increased or generated, e.g. from E. coli or a homolog thereof.
The sequence of b1827 from Escherichia coli K12 has been published in Blattner et al., Science 277(5331), 1453-1474, 1997, and its activity is being defined as a putative transcriptional repressor with DNA-binding Winged helix domain (IcIR family), preferably of the acetate operon repressor superfamily. Accordingly, in one embodiment, the process of the present invention comprises the use of a protein of the acetate operon repressor superfamily, preferably such protein is having a transcriptional control activity, in particular an activity of a putative transcriptional repressor with DNA-binding Winged helix domain (IcIR family), e.g. as shown herein, for the production of the respective fine chemical, meaning of vitamin E, e.g. beta-tocopherol, e.g. via increasing the biosynthesis of the vitamin E precursor 2,3-dimethyl-5-phytylquinol, preferably in free or bound form, in an organism or a part thereof, as mentioned. In one further embodiment the b1827 protein expression is increased together with the increase of another gene of the vitamin E biosynthesis pathway, preferably with a gene encoding a protein being involved in the production of vitamin E from the intermediate 2,3-dimethyl-5-phytylquiol. In one embodiment, in the process of the present invention said activity, e.g. the activity of a putative transcriptional repressor with DNA-binding Winged helix domain (IcIR family), preferably of the acetate operon repressor superfamily is increased or generated from E. coli or a homolog thereof.
The sequence of b0785 from Escherichia coli K12 has been published in Blattner et al., Science 277(5331), 1453-1474, 1997, and its activity is being defined as a Molybdenum cofactor biosynthesis protein E. Accordingly, in one embodiment, the process of the present invention comprises the use of a protein involved in the metabolism of vitamins, cofactors, and prosthetic groups; other metal binding; protein binding; molybdopterin binding, preferably of the molybdopterin biosynthesis protein E chain superfamily, preferably of a Molybdenum cofactor biosynthesis protein E, e.g. from E. coli or its homolog, e.g. as shown herein, for the production of the respective fine chemical, meaning vitamin E, in particular beta-tocopherol and/or gamma-tocopherol, preferably in free or bound form, in an organism or a part thereof, as mentioned. In one embodiment, in the process of the present invention said activity, e.g. the activity of a Molybdenum cofactor biosynthesis protein E is increased or generated, e.g. from E. coli or a homolog thereof.
The sequence of b3938 from Escherichia coli K12 has been published in Blattner et al., Science 277(5331), 1453-1474, 1997, and its activity is being defined as a transcriptional repressor for methionine biosynthesis. Accordingly, in one embodiment, the process of the present invention comprises the use of a protein of the metJ protein superfamily, preferably of a transcriptional repressor for methionine biosynthesis, e.g from E. coli or its homolog, e.g. as shown herein, for the production of the respective fine chemical, meaning vitamin E, in particular beta-tocopherol and/or gamma-tocopherol, preferably in free or bound form, in an organism or a part thereof, as mentioned. In one embodiment, in the process of the present invention said activity, e.g. the activity of a transcriptional repressor for methionine biosynthesis is increased or generated, e.g. from E. coli or a homolog thereof.
The sequence of b0175 from Escherichia coli K12 has been published in Blattner et al., Science 277(5331), 1453-1474, 1997, and its activity is being defined as a CDP-diglyceride synthase. Accordingly, in one embodiment, the process of the present invention comprises the use of a protein involved in the nucleotide-metabolism, phospholipid biosynthesis, lipid, fatty-acid or isoprenoids metabolism, cellular communication, signal transduction, or cellular sensing and response, preferably of the phophatidate cytidylyltransferase superfamily, preferably of a CDP-diglyceride synthase, e.g from E. coli or its homolog, e.g. as shown herein, for the production of the respective fine chemical, meaning vitamin E, in particular beta-tocopherol and/or gamma-tocopherol, preferably in free or bound, form in an organism or a part thereof, as mentioned. In one embodiment, in the process of the present invention said activity, e.g. the activity of a phosphatidate cytidylytransferase, in particular of a CDP-diglyceride synthase, is increased or generated, e.g. from E. coli or a homolog thereof.
The sequence of b2699 from Escherichia coli K12 has been published in Blattner et al., Science 277(5331), 1453-1474, 1997, and its activity is being defined as a DNA strand exchange and recombination protein with protease and nuclease activity of the superfamily of the recombination protein recA. Accordingly, in one embodiment, the process of the present invention comprises the use of a protein with a DNA recombination and DNA repair activity, a pheromone response activity, a mating-type determination activity, a sex-specific protein activity, a nucleotide binding activity and/or a protease and nuclease activity, in particular a DNA strand exchange and recombination protein with protease and nuclease activity, in particular of the superfamily of the recombination protein recA from E. coli or its homolog, e.g. as shown herein, for the production of the respective fine chemical, meaning of vitamin E, in particular for increasing the amount of alpha-tocopherol, preferably in free or bound form, in an organism or a part thereof, as mentioned. In one embodiment, in the process of the present invention said activity, e.g. the activity of a protease and nuclease activity, in particular a DNA strand exchange and recombination protein with protease and nuclease activity, in particular of the superfamily of the recombination protein recA is increased or generated, e.g. from E. coli or a homolog thereof.
The sequence of b3160 from Escherichia coli K12 has been published in Blattner et al., Science 277(5331), 1453-1474, 1997, and its activity is being defined as a putative monooxygenase with luciferase-like ATPase activity. Accordingly, in one embodiment, the process of the present invention comprises the use of a putative monooxygenase with luciferase-like ATPase activity from E. coli or its homolog, e.g. as shown herein, for the production of the fine chemical, meaning of alpha-tocopherol, in particular for increasing the amount of alpha-tocopherol, preferably alpha-tocopherol in free or bound form, in an organism or a part thereof, as mentioned. In one embodiment, in the process of the present invention the activity of a putative monooxygenase with luciferase-like ATPase activity is increased or generated, e.g. from E. coli or a homolog thereof.
The sequence of b0161 from Escherichia coli K12 has been published in Blattner et al., Science 277(5331), 1453-1474, 1997, and its activity is being defined as a periplasmic serine protease and/or heat shock protein. Accordingly, in one embodiment, the process of the present invention comprises the use of extracellular / secretion proteins, or proteins involved in cell growth / morphogenesis, proteolytic degradation, protein binding, intracellular signalling, preferably being a protein of the superfamily of Helicobacter serine proteinase, preferably a periplasmic serine protease (heat shock protein) from E. coli or its homolog, e.g. as shown herein, for the production of the fine chemical, meaning of alpha-tocopherol, in particular for increasing the amount of alpha-tocopherol, preferably alpha-tocopherol in free or bound form, in an organism or a part thereof, as mentioned. In one embodiment, in the process of the present invention the activity of a periplasmic serine protease (heat shock protein) is increased or generated, e.g. from E. coli or a homolog thereof.
The sequence of b0970 from Escherichia coli K12 has been published in Blattner et al., Science 277(5331), 1453-1474, 1997, and its activity is being defined as probable glutamate receptor. Accordingly, in one embodiment, the process of the present invention comprises the use of a protein being involved in apoptosis (type I programmed cell death), transmembrane signal transduction, channel / pore class transporters, unspecified signal transduction and/or protein binding, preferably from the Escherichia coli ybhL protein superfamily, in particular of a probable glutamate receptor from E. coli or its homolog, e.g. as shown herein, for the production of the respective fine chemical, meaning of vitamin E, in particular for increasing the amount of alpha-tocopherol, preferably in free or bound form, in an organism or a part thereof, as mentioned. In one embodiment, in the process of the present invention said activity, e.g. an probable glutamate receptor, in particular of the superfamily of Escherichia coli ybhL protein, is increased or generated, e.g. from E. coli or a homolog thereof.
The sequence of b4063 from Escherichia coli K12 has been published in Blattner et al., Science 277(5331), 1453-1474, 1997, and its activity is being defined as transcriptional activator for superoxide response. Accordingly, in one embodiment, the process of the present invention comprises the use of a protein being involved in transcriptional control, DNA binding, inorganic chemical agent resistance (e.g. heavy metals), regulation of amino acid metabolism and/or regulation of nitrogen and sulphur utilization, in particular of a transcriptional activator for superoxide response from E. coli or its homolog, e.g. as shown herein, for the production of the respective fine chemical, meaning of vitamin E, in particular for increasing the amount of alpha-tocopherol, preferably in free or bound form, in an organism or a part thereof, as mentioned. In one embodiment, in the process of the present invention said activity, e.g. a transcriptional activator for superoxide response, in particular of the superfamily of Escherichia coli ybhL protein, is increased or generated, e.g. from E. coli or a homolog thereof.
The sequence of b0112 from Escherichia coli K12 has been published in Blattner et al., Science 277(5331), 1453-1474, 1997, and its activity is being defined as aromatic amino acid transport protein (APC family). Accordingly, in one embodiment, the process of the present invention comprises the use of a protein with a amino acid transporter activity, a cellular transport activity, preferably from the arginine permease superfamily, in particular of a aromatic amino acid transport protein (APC family) from E. coli or its homolog, e.g. as shown herein, for the production of the respective fine chemical, meaning of vitamin E, in particular for increasing the amount of alpha-tocopherol, preferably in free or bound form, in an organism or a part thereof, as mentioned. In one embodiment, in the process of the present invention said activity, e.g. the activity of a protein of the APC family, in particular an aromatic amino acid transport protein, in particular of the superfamily of arginine permease, is increased or generated, e.g. from E. coli or a homolog thereof.
The sequence of b0986 from Escherichia coli K12 has been published in Blattner et al., Science 277(5331), 1453-1474, 1997, and its activity is being defined as a lipoprotein. Accordingly, in one embodiment, the process of the present invention comprises the use of a lipoprotein, preferably from the superfamily of the hypothetical protein b1706, from E. coli or its homolog, e.g. as shown herein, for the production of the respective fine chemical, meaning of vitamin E, in particular for increasing the amount of beta-tocopherol and/or gamma-tocopherol, preferably in free or bound form, in an organism or a part thereof, as mentioned. In one embodiment, in the process of the present invention said activity, e.g. the activity of a lipoprotein, in particular of the superfamily the hypothetical protein b1706, is increased or generated, e.g. from E. coli or a homolog thereof.
The sequence of YFL019C from Saccharomyces cerevisiae has been published in Murakami Y. et al., Nat. Genet. 10:261-268(1995) and its activity has not been characterized yet. Accordingly, in one embodiment, the process of the present invention comprises the use of a protein with a YFL019C activity or its homolog, e.g. as shown herein, for the production of the respective fine chemical, meaning of vitamin E, e.g. via increasing the biosynthesis of the vitamin E precursor 2,3-dimethyl-5-phytylquinol, preferably in free or bound form, in an organism or a part thereof, as mentioned. In one further embodiment the YFL019C protein expression is increased together with the increase of another gene of the vitamin E biosynthesis pathway, preferably with a gene encoding a protein being involved in the production of vitamin E from the intermediate 2,3-Dimethyl-5-phytylquiol. In one embodiment, in the process of the present invention said YFL019C activity is increased or generated, e.g. from Saccharomyces cerevisiae or a homolog thereof.
The sequence of YFL013C from Saccharomyces cerevisiae has been published in Murakami,Y et al., Nat. Genet. 10 (3), 261-268 (1995), and Goffeau et al., Science 274 (5287), 546-547, 1996, and its activity is being defined as subunit of the INO80 chromatin remodelling complex. Accordingly, in one embodiment, the process of the present invention comprises the use of a protein having the activity of a subunit of the INO80 chromation remodelling complex, in particular of a subunit of the INO80 chromation remodelling complex of the superfamily of the probable membrane protein YFL013c, e.g. from E. coli or its homolog, for the production of the respective fine chemical, meaning vitamin E, e.g. via increasing the biosynthesis of the vitamin E precursor 2,3-dimethyl-5-phytylquinol, preferably in free or bound form, in an organism or a part thereof, as mentioned. In one further embodiment the YFL013C protein expression is increased together with the increase of another gene of the vitamin E biosynthesis pathway, preferably with a gene encoding a protein being involved in the production of vitamin E from the intermediate 2,3-Dimethyl-5-phytylquiol. In one embodiment, in the process of the present invention said activity, e.g. the activity of a subunit of the INO80 chromation remodelling complex is increased or generated, e.g. from Saccharomyces cerevisiae or a homolog thereof.
The sequence of YPL268W from Saccharomyces cerevisiae has been published in Bussey,H. et al., Nature 387 (6632 Suppl), 103-105 (1997), and Goffeau et al., Science 274 (5287), 546-547, 1996, and its activity is being defined as phophoinositide phospholipase. Accordingly, in one embodiment, the process of the present invention comprises the use of a protein involved in the breakdown of lipids, fatty acids and isoprenoids, cell growth and morphogenesis, mitotic cell cycle, cell cycle control, intracellular signal transduction activities, or DNA processing, in particular of the superfamiliy of the yeast 1-phosphatidylinostiol-4,5-bisphophate phosphodiesterase, preferably having a phophoinositide phospholipase activity or its homolog, e.g. as shown herein, for the production of the respective fine chemical, meaning of vitamin E, e.g. via increasing the biosynthesis of the vitamin E precursor 2,3-dimethyl-5-phytylquinol, preferably in free or bound form, in an organism or a part thereof, as mentioned. In one further embodiment the YPL268W protein expression is increased together with the increase of another gene of the vitamin E biosynthesis pathway, preferably with a gene encoding a protein being involved in the production of vitamin E from the intermediate 2,3-Dimethyl-5-phytylquiol. In one embodiment, in the process of the present invention said activity, e.g. of a protein of the yeast 1-phosphatidylinostiol-4,5-bisphophate phosphodiesterase superfamily or preferably having a phophoinositide phospholipase activity is increased or generated, e.g. from Saccharomyces cerevisiae or a homolog thereof.
The sequence of YFL053W from Saccharomyces cerevisiae has been published in Murakami,Y et al., Nat. Genet. 10 (3), 261-268 (1995), and Goffeau et al., Science 274 (5287), 546-547, 1996, and its activity is being defined as dihydroxyacetone kinase. Accordingly, in one embodiment, the process of the present invention comprises the use of a protein involved in the utilization of c-compound and carbohydrate, stress response, modification by phosphorylation or dephosphorylation, in particular of the superfamiliy glyerone kinase DAK1, preferably having a dihydroxyacetone kinase activity, or its homolog, e.g. as shown herein, for the production of the respective fine chemical, meaning of vitamin E, e.g. gamma- and/or beta-tocopherol, preferably in free or bound form, in an organism or a part thereof, as mentioned. In one embodiment, in the process of the present invention said activity, e.g. of a protein of the superfamiliy glyerone kinase DAK1, preferably having a dihydroxyacetone kinase activity, is increased or generated, e.g. from Saccharomyces cerevisiae or a homolog thereof.

**[0023.0.9.9]** Homologues ( = homologs) of the present gene products can be derived from any organisms as long as the homologue confers the herein mentioned activity, in particular, confers an increase in the respective fine chemical amount or content. Further, in the present invention, the term "homologue" relates to the sequence of an organism having the highest sequence homology to the herein mentioned or listed sequences of all expressed sequences of said organism. However, the person skilled in the art knows, that, preferably, the homologue has said the-fine-chemical-increasing activity and, if known, the same biological function or activity in the organism as at least one of the protein(s) indicated in Table II, Column 3, lines 89 to 102 and/or 472 to 482, e.g. having the sequence of a polypeptide encoded by a nucleic acid molecule comprising the sequence indicated in Table I, Column 5 or 7, lines 89 to 102 and/or 472 to 482.
In one embodiment, the homolog of the any one of the polypeptides indicated in Table II, column 3, line 89 or 90 or 482, resp. is a homolog having the same or a similar activity, resp. In particular an increase of activity confers an increase in the content of the respective fine chemical in the organisms, preferably of vitamin E via its precursor 2,3-dimethyl-5-phytylquinol. In one embodiment, the homolog is a homolog with a sequence as indicated in Table I or II, column 7, line 89 or 90 or 482, resp. In one embodiment, the homolog of one of the polypeptides indicated in Table II, column 3, line 89 or 90 or 482 resp., is derived from an eukaryotic. In one embodiment, the homolog is derived from Fungi. In one embodiment, the homolog of a polypeptide indicated in Table II, column 3, line 89 or 90 or 482, resp., is derived from Ascomyceta. In one embodiment, the homolog of a polypeptide indicated in Table II, column 3, line 89 or 90 or 482, resp., is derived from Saccharomycotina. In one embodiment, the homolog of a polypeptide indicated in Table II, column 3, line 89 or 90 or 482, resp., is derived from Saccharomycetes. In one embodiment, the homolog of a polypeptide indicated in Table II, column 3, line 89 or 90 or 482, resp., is a homolog being derived from Saccharomycetales. In one embodiment, the homolog of a polypeptide indicated in Table II, column 3, line 89 or 90 or 482, resp., is a homolog having the same or a similar activity being derived from Saccharomycetaceae. In one embodiment, the homolog of a polypeptide indicated in Table II, column 3, line 89 or 90 or 482, resp., is a homolog having the same or a similar activity being derived from Saccharomycetes.
In one embodiment, the homolog of the any one of the polypeptides indicated in Table II, column 3, line 96 is a homolog having the same or a similar activity. In particular an increase of activity confers an increase in the content of the respective fine chemical in the organisms, preferably of vitamin E, preferably of beta-tocopherol. In one embodiment, the homolog is a homolog with a sequence as indicated in Table I or II, column 7, line 96. In one embodiment, the homolog of one of the polypeptides indicated in Table II, column 3, line 96., is derived from an eukaryotic. In one embodiment, the homolog is derived from Fungi. In one embodiment, the homolog of a polypeptide indicated in Table II, column 3, line 96, is derived from Ascomyceta. In one embodiment, the homolog of a polypeptide indicated in Table II, column 3, line 96, is derived from Saccharomycotina. In one embodiment, the homolog of a polypeptide indicated in Table II, column 3, line 96, is derived from Saccharomycetes. In one embodiment, the homolog of a polypeptide indicated in Table II, column 3, line 96, is a homolog being derived from Saccharomycetales. In one embodiment, the homolog of a polypeptide indicated in Table II, column 3, line 96, is a homolog having the same or a similar activity being derived from Saccharomycetaceae. In one embodiment, the homolog of a polypeptide indicated in Table II, column 3, line 96, is a homolog having the same or a similar activity being derived from Saccharomycetes.
In one embodiment, the homolog of the any one of the polypeptides indicated in Table II, column 3, line 101. is a homolog having the same or a similar activity. In particular an increase of activity confers an increase in the content of the respective fine chemical in the organisms, preferably of vitamin E, preferably of gamma-tocopherol. In one embodiment, the homolog is a homolog with a sequence as indicated in Table I or II, column 7, line 101. In one embodiment, the homolog of one of the polypeptides indicated in Table II, column 3, line 101., is derived from an eukaryotic. In one embodiment, the homolog is derived from Fungi. In one embodiment, the homolog of a polypeptide indicated in Table II, column 3, line 101, is derived from Ascomyceta. In one embodiment, the homolog of a polypeptide indicated in Table II, column 3, line 101, is derived from Saccharomycotina. In one embodiment, the homolog of a polypeptide indicated in Table II, column 3, line 101, is derived from Saccharomycetes. In one embodiment, the homolog of a polypeptide indicated in Table II, column 3, line 101, is a homolog being derived from Saccharomycetales. In one embodiment, the homolog of a polypeptide indicated in Table II, column 3, line 101, is a homolog having the same or a similar activity being derived from Saccharomycetaceae. In one embodiment, the homolog of a polypeptide indicated in Table II, column 3, line 101, is a homolog having the same or a similar activity being derived from Saccharomycetes.
In one embodiment, the homolog of the any one of the polypeptides indicated in Table II, column 3, lines 91 or 92 is a homolog having the same or a similar activity. In particular an increase of activity confers an increase in the content of the respective fine chemical in the organisms preferably of vitamin E via its precursor 2,3-dimethyl-5-phytylquinol. In one embodiment, the homolog is a homolog with a sequence as indicated in Table I or II, column 7, lines 91 or 92, resp. In one embodiment, the homolog of one of the polypeptides indicated in Table II, column 3, 91 or 92 is derived from an bacteria. In one embodiment, the homolog of a polypeptide indicated in Table II, column 3, lines 91 or 92 is derived from Proteobacteria. In one embodiment, the homolog of a polypeptide indicated in Table II, column 3, lines 91 or 92 is a homolog having the same or a similar activity being derived from Gammaproteobacteria. In one embodiment, the homolog of a polypeptide indicated in Table II, column 3, lines 91 and 92 is derived from Enterobacteriales. In one embodiment, the homolog of a polypeptide indicated in Table II, column 3, lines 91 or 92 is a homolog being derived from Enterobacteriaceae. In one embodiment, the homolog of a polypeptide indicated in Table II, column 3, lines 91 or 92 is a homolog having the same or a similar activity and being derived from Escherichia.
In one embodiment, the homolog of the any one of the polypeptides indicated in Table II, column 3, lines 93, 94, 95, 472, 473, 474, or 475 is a homolog having the same or a similar activity. In particular an increase of activity confers an increase in the content of the respective fine chemical in the organisms, preferably of alpha-tocopherol. In one embodiment, the homolog is a homolog with a sequence as indicated in Table I or II, column 7, lines 93, 94, 95, 472, 473, 474, or 475 , resp. In one embodiment, the homolog of one of the polypeptides indicated in Table II, column 3, lines 93, 94, 95, 472, 473, 474, or 475 is derived from an bacteria. In one embodiment, the homolog of a polypeptide indicated in Table II, column 3, lines 93, 94, 95, 472, 473, 474, or 475 is derived from Proteobacteria. In one embodiment, the homolog of a polypeptide indicated in Table II, column 3, lines 93, 94, 95, 472, 473, 474, or 475 is a homolog having the same or a similar activity being derived from Gammaproteobacteria. In one embodiment, the homolog of a polypeptide indicated in Table II, column 3, lines 93, 94, 95, 472, 473, 474, or 475 is derived from Enterobacteriales. In one embodiment, the homolog of a polypeptide indicated in Table II, column 3, lines 93, 94, 95, 472, 473, 474, or 475 is a homolog being derived from Enterobacteriaceae. In one embodiment, the homolog of a polypeptide indicated in Table II, column 3, lines 93, 94, 95, 472, 473, 474, or 475 is a homolog having the same or a similar activity and being derived from Escherichia.
In one embodiment, the homolog of the any one of the polypeptides indicated in Table II, column 3, lines 97 to 100 or 476 or 477 is a homolog having the same or a similar activity. In particular an increase of activity confers an increase in the content of the respective fine chemical in the organisms preferably beta-tocopherol. In one embodiment, the homolog is a homolog with a sequence as indicated in Table I or II, column 7, lines 97 to 100 or 476 or 477, resp. In one embodiment, the homolog of one of the polypeptides indicated in Table II, column 3, lines 97 to 100 or 476 or 477 is derived from an bacteria. In one embodiment, the homolog of a polypeptide indicated in Table II, column 3, lines 97 to 100 or 476 or 477 is derived from Proteobacteria. In one embodiment, the homolog of a polypeptide indicated in Table II, column 3, lines 97 to 100 or 476 or 477 is a homolog having the same or a similar activity being derived from Gammaproteobacteria. In one embodiment, the homolog of a polypeptide indicated in Table II, column 3, lines 97 to 100 or 476 or 477 is derived from Enterobacteriales. In one embodiment, the homolog of a polypeptide indicated in Table II, column 3, lines 97 to 100 or 476 or 477 is a homolog being derived from Enterobacteriaceae. In one embodiment, the homolog of a polypeptide indicated in Table II, column 3, lines 97 to 100 or 476 or 477 is a homolog having the same or a similar activity, being derived from Escherichia.
In one embodiment, the homolog of the any one of the polypeptides indicated in Table II, column 3, line 102 or lines 478 to 481 is a homolog having the same or a similar activity. In particular an increase of activity confers an increase in the content of the respective fine chemical in the organisms preferably gamma-tocopherol. In one embodiment, the homolog is a homolog with a sequence as indicated in Table I or II, column 7, line 102 or lines 478 to 481, resp. In one embodiment, the homolog of one of the polypeptides indicated in Table II, column 3, line 102 or lines 478 to 481 is derived from an bacteria. In one embodiment, the homolog of a polypeptide indicated in Table II, column 3, line 102 or lines 478 to 481 is derived from Proteobacteria. In one embodiment, the homolog of a polypeptide indicated in Table II, column 3, line 102 or lines 478 to 481 is a homolog having the same or a similar activity being derived from Gammaproteobacteria. In one embodiment, the homolog of a polypeptide indicated in Table II, column 3, line 102 or lines 478 to 481 is derived from Enterobacteriales. In one embodiment, the homolog of a polypeptide indicated in Table II, column 3, line 102 or lines 478 to 481 is a homolog being derived from Enterobacteriaceae. In one embodiment, the homolog of a polypeptide indicated in Table II, column 3, line 102 or lines 478 to 481 is a homolog having the same or a similar activity, being derived from Escherichia.

**[0023.1.9.9]** Homologs of the polypeptide indicated in Table II, column 3, lines 89 to 102 or 472 to 482 may be the polypeptides encoded by the nucleic acid molecules indicated in Table I, column 7, lines 89 to 102 or 472 to 482, resp., or may be the polypeptides indicated in Table II, column 7, lines 89 to 102 or 472 to 482, resp. Homologs of the polypeptides indicated in Table II, column 3, lines 89 to 102 or 472 to 482 may be the polypeptides encoded by the nucleic acid molecules indicated in Table I, column 7, lines 89 to 102 or 472 to 482, resp., or may be the polypeptides indicated in Table II, column 7, lines 89 to 102 or 472 to 482.
Homologs of the polypeptides indicated in Table II, column 3, lines 89 to 92 or 482 may be the polypeptides encoded by the nucleic acid molecules indicated in Table I, column 7, lines 89 to 92 or 482, respectively or may be the polypeptides indicated in Table II, column 7, lines 89 to 92 or 482, having a 2,3-Dimethyl-5-phytylquinol content- and/or amount-increasing activity. Homologs of the polypeptides indicated in Table II, column 3, lines 89 to 92 or 482 may be the polypeptides encoded by the nucleic acid molecules indicated in Table I, column 7, lines 89 to 92 or 482 or may be the polypeptides indicated in Table II, column 7, lines 89 to 92 or 482 having a 2,3-Dimethyl-5-phytylquinol content- and/or amount-increasing activity.
Homologs of the polypeptides indicated in Table II, column 3, lines 93 to 95 or 472 to 475 may be the polypeptides encoded by the nucleic acid molecules indicated in Table I, column 7, lines 93 to 95 or 472 to 475, respectively or may be the polypeptides indicated in Table II, column 7, lines 93 to 95 or 472 to 475, having a alpha-tocopherol content- and/or amount-increasing activity. Homologs of the polypeptides indicated in Table II, column 3, lines 93 to 95 or 472 to 475 may be the polypeptides encoded by the nucleic acid molecules indicated in Table I, column 7, lines 93 to 95 or 472 to 475 or may be the polypeptides indicated in Table II, column 7, lines 93 to 95 or 472 to 475 having a alpha-tocopherol content- and/or amount-increasing activity.
Homologs of the polypeptides indicated in Table II, column 3, lines 96 to 100 or 476 to 477 may be the polypeptides encoded by the nucleic acid molecules indicated in Table I, column 7, 96 to 100 or 476 to 477, respectively or may be the polypeptides indicated in Table II, column 7, 96 to 100 or 476 to 477, having a beta-tocopherol content- and/or amount-increasing activity. Homologs of the polypeptides indicated in Table II, column 3, 96 to 100 or 476 to 477 may be the polypeptides encoded by the nucleic acid molecules indicated in Table I, column 7, 96 to 100 or 476 to 477 or may be the polypeptides indicated in Table II, column 7, lines 96 to 100 or 476 to 477 having a beta-tocopherol content- and/or amount-increasing activity.
Homologs of the polypeptides indicated in Table II, column 3, lines 101 to 102 or 478 to 481 may be the polypeptides encoded by the nucleic acid molecules indicated in Table I, column 7, 101 to 102 or 478 to 481, respectively or may be the polypeptides indicated in Table II, column 7, 101 to 102 or 478 to 481, having a gamma-tocopherol content- and/or amount-increasing activity. Homologs of the polypeptides indicated in Table II, column 3, 101 to 102 or 478 to 481 may be the polypeptides encoded by the nucleic acid molecules indicated in Table I, column 7, 101 to 102 or 478 to 481 or may be the polypeptides indicated in Table II, column 7, lines 101 to 102 or 478 to 481 having a gamma-tocopherol content- and/or amount-increasing activity.

**[0024.0.0.9]** see **[0024.0.0.0]**

**[0025.0.9.9]** In accordance with the invention, a protein or polypeptide has the "activity of an protein of the invention", e.g. the activity of a protein indicated in Table II, column 3, lines 89 to 92 or 482 and/or lines 93 to 95 or 472 to 475 and/or lines 96 to 100 or 476 to 477 and/or line 101 to 102 or 478 to 481, resp., if its *de novo* activity, or its increased expression directly or indirectly leads to an increased vitamin E or its precursor 2,3-dimethyl-5-phytylquinol level, in particular to a increased alpha-, beta-, and/or gamma-tocopherol, resp., in the organism or a part thereof, preferably in a cell of said organism. In a preferred embodiment, the protein or polypeptide has the above-mentioned additional activities of a protein indicated in Table II, column 3, lines 89 to 92 or 482 and/or lines 93 to 95 or 472 to 475 and/or lines 96 to 100 or 476 to 477 and/or line 101 to 102 or 478 to 481. Throughout the specification the activity or preferably the biological activity of such a protein or polypeptide or an nucleic acid molecule or sequence encoding such protein or polypeptide is identical or similar if it still has the biological or enzymatic activity of any one of the proteins indicated in Table II, column 3, lines 89 to 92 or 482 and/or lines 93 to 95 or 472 to 475 and/or lines 96 to 100 or 476 to 477 and/or line 101 to 102 or 478 to 481, resp. or which has at least 10% of the original enzymatic activity, preferably 20%, particularly preferably 30%, most particularly preferably 40% in comparison to any one of the proteins indicated in Table II, column 3, lines 89, 90, 96, 101, or 482 of Saccharomyces cerevisiae and/or any one of the proteins indicated in Table II, column 3, lines 91 to 95, 97 to 100, or 102 or 472 to 481 of E. coli K12.
In one embodiment, the polypeptide of the invention confers said activity, e.g. the increase of the respective fine chemical in an organism or a part thereof, if it is derived from an organism, which is evolutionary close to the organism indicated in Table I, column 4 and is expressed in an organism, which is evolutionary distant to the origin organism. For example origin and expressing organism are derived from different families, orders, classes or phylums whereas origin and the organism indicated in Table I, column 4 are derived from the same families, orders, classes or phylums.

**[0025.1.0.9] see [0025.1.0.0]**

**[0025.2.0.9] see [0025.2.0.0]**

**[0025.3.18.18]** In one embodiment, the polypeptide of the invention or used in the method of the invention confers said activity, e.g. the increase of the respective fine chemical in an organism or a part thereof, if it is derived from an organism, which is evolutionary close to the organism indicated in Table I, column 4 and is expressed in an organism, which is evolutionary distant to the origin organism. For example origin and expressing organism are derived from different families, orders, classes or phylums whereas origin and the organism indicated in Table I, column 4 are derived from the same families, orders, classes or phylums.

**[0026.0.0.9] to [0033.0.0.9]: see [0026.0.0.0] to [0033.0.0.0]**

**[0034.0.9.9]** Preferably, the reference, control or wild type differs form the subject of the present invention only in the cellular activity of the polypeptide of the invention or the polypeptide used in the method of the invention, e.g. as result of an increase in the level of the nucleic acid molecule of the present invention or an increase of the specific activity of the polypeptide of the invention or the polypeptide used in the method of the invention. E.g., it differs by or in the expression level or activity of an protein having the activity of a protein as indicated in Table II, column 3, lines 89 to 92 or 482 and/or lines 93 to 95 or 472 to 475 and/or lines 96 to 100 or 476 to 477 and/or line 101 and/or 102 and/or lines 478 to 481, resp., or being encoded by a nucleic acid molecule indicated in Table I, column 5, lines 89 to 92 or 482 and/or lines 93 to 95 or 472 to 475 and/or lines 96 to 100 or 476 to 477 and/or line 101 and/or 102 and/or lines 478 to 481, resp., or its homologs, e.g. as indicated in Table I, column 7, lines 89 to 92 or 482 and/or lines 93 to 95 or 472 to 475 and/or lines 96 to 100 or 476 to 477 and/or line 101 to 102 or 478 to 481, resp., its biochemical or genetical causes and therefore shows the increased amount of the respective fine chemical.

**[0035.0.0.9]** to **[0044.0.0.9]: see [0035.0.0.0]** to **[0044.0.0.0]**

**[0045.0.9.9]** In case the activity of the Escherichia coli K12 protein b0161 or its homologs, as indicated in Table I, columns 5 or 7, line 472, e.g. protein with an activity as defined in [0022.0.9.9], is increased, preferably, in one embodiment the increase of the fine chemical, preferably of alpha-tocopherol between 50% and 138% or more is conferred.
In case the activity of the Escherichia coli K12 protein b0970 or its homologs, as indicated in Table I, columns 5 or 7, line 473, e.g. protein with an activity as defined in [0022.0.9.9], is increased, preferably, in one embodiment the increase of the fine chemical, preferably of alpha-tocopherol between 47% and 54% or more is conferred.
In case the activity of the Escherichia coli K12 protein b4063 or its homologs, as indicated in Table I, columns 5 or 7, line 475, e.g. protein with an activity as defined in [0022.0.9.9], is increased, preferably, in one embodiment the increase of the fine chemical, preferably of alpha-tocopherol between 16% and 47% or more is conferred.
In case the activity of the Escherichia coli K12 protein b0785 or its homologs, as indicated in Table I, columns 5 or 7, line 476 or 479, e.g. protein with an activity as defined in [0022.0.9.9], is increased, preferably, in one embodiment the increase of the fine chemical, preferably of beta- and/or gamma-tocopherol between 34% and 40% or more is conferred.
In case the activity of the Escherichia coli K12 protein b3938 or its homologs, as indicated in Table I, columns 5 or 7, line 477 or 481, e.g. protein with an activity as defined in [0022.0.9.9], is increased, preferably, in one embodiment the increase of the fine chemical, preferably of beta- and/or gamma-tocopherol between 50% and 120% or more is conferred.
In one embodiment, in case the activity of the Escherichia coli K12 protein b3160, as indicated in Table I, columns 5 or 7, line 474, or a protein with the activity defined as putative monooxygenase with luciferase-like ATPase activity or its homologs, e.g. transcriptional regulator, is increased, preferably, in one embodiment an increase of the fine chemical alpha-tocopherol between 47% and 91% is conferred.
In one embodiment the activity of the Escherichia coli K12 protein b1829 or its homologs, e.g. having a heat shock protein or a protease activity, e.g. as indicated in Table I, columns 5 or 7, line 91, is increased conferring an increase of the respective fine chemical, preferably of the vitamin E precursor 2,3-dimethyl-5-phytylquinol, between 48% and 283% or more.
In one embodiment, the activity of the Escherichia coli K12 protein b1829 or its homologs, e.g. with a heat shock protein with protease activity, e.g. as indicated in Table I, columns 5 or 7, line 94, is increased conferring an increase of the respective fine chemical, preferably of vitamin E, in particular of alpha-tocopherol, between 39% and 90% or more.
In one embodiment, the activity of the Escherichia coli K12 protein b1829 or its homologs, e.g. with a heat shock protein with protease activity, e.g. as indicated in Table I, columns 5 or 7, line 100 or 102, is increased conferring an increase of the respective fine chemical, preferably of the vitamin E in particular of beta- and/or gamma-tocopherol between 40% and 325% or more.
In one embodiment, the activity of the Escherichia coli K12 protein b1827 or its homologs, e.g. with a putative transcriptional repressor with DNA-binding Winged helix domain (IcIR family) activity, e.g. as indicated in Table I, columns 5 or 7, line 92, is increased conferring an increase of the respective fine chemical, preferably of the vitamin E precursor 2,3-dimethyl-5-phytylquinol, between 76% and 91% or more.
In one embodiment, the activity of the Escherichia coli K12 protein b1827 or its homologs, e.g. with a putative transcriptional repressor with DNA-binding Winged helix domain (IcIR family) activity, e.g. as indicated in Table I, columns 5 or 7, line 97, is increased conferring an increase of the respective fine chemical, preferably of the vitamin E, in particular beta-tocopherol, between 34% and 116% or more.
In one embodiment, the activity of the Escherichia coli K12 protein b2699 or its homologs, e.g. a protein with a DNA strand exchange and recombination protein with protease und nuclease-activity, e.g. as indicated in Table I, columns 5 or 7, line 93, is increased conferring an increase of the respective fine chemical, preferably of the vitamin E, in particular of alpha-tocopherol, between 51% and 196% or more.
In one embodiment, the activity of the Escherichia coli K12 protein b0112 or its homologs, e.g. a protein with an aromatic amino acid transport protein (APC family)-activity, e.g. as indicated in Table I, columns 5 or 7, line 95, is increased conferring an increase of the respective fine chemical, preferably of the vitamin E, in particular of alpha-tocopherol, between 43% and 63% or more.
In one embodiment, the activity of the Escherichia coli K12 protein b0175 or its homologs, e.g. a CDP-diglyceride synthase-activity, e.g. as indicated in Table I, columns 5 or 7, line 99 or 478, is increased conferring an increase of the respective fine chemical, preferably of the vitamin E, in particular of beta- and/or gamma-tocopherol, between 34% and 40% or more.
In one embodiment, the activity of the Escherichia coli K12 protein b0986 or its homologs, e.g. a lipoprotein, e.g. as indicated in Table I, columns 5 or 7, line 98 and 480, is increased conferring an increase of the respective fine chemical, preferably of the vitamin E, in particular of beta and/or gamma-tocopherol, between 35% and 61 % or more.
In case the activity of the Saccharomyces cerevisiae protein YFL019C or its homologs, as indicated in Table I, columns 5 or 7, line 482, e.g. protein with an activity as defined in [0022.0.9.9], is increased, preferably, in one embodiment the increase of the fine chemical, preferably of 2,3-Dimethyl-5-pythylquinol between 97% and 440% or more is conferred.
In one embodiment, the activity of the Saccharomyces cerevisiae protein YFL013C or its homologs, e.g. an activity of a subunit of the IN080 chroamtion remodeling comples, in particular, of the superfamiliy of the membrane protein YFL013c, e.g. as indicated in Table I, columns 5 or 7, 90, is increased conferring an increase of the respective fine chemical, preferably of the vitamin E precursor 2,3-dimethyl-5-phytyhlquinol between 97% and 440% or more.
In one embodiment, the activity of the Saccharomyces cerevisiae protein YPL268W or its homologs, e.g. an activity of a phophoinositide phopholipase, in particular, of the superfamiliy of the yeast 1-phosphatidylinositol-4,5-bisphosphate phophodiesterase, e.g. as indicated in Table I, columns 5 or 7, 89, is increased conferring an increase of the respective fine chemical, preferably of the vitamin E precursor 2,3-dimethyl-5-phytyhlquinol between 44% and 51 % or more.
In one embodiment, in case the activity of the Saccharomyces cerevisiae protein YFL053W or its homologs, e.g. an activity of a dihydroxaacetone kinase, in particular, of the superfamiliy of theglyerone kinase DAK1, e.g. as indicated in Table I, columns 5 or 7, 96 and 101, is increased conferring an increase of the respective fine chemical, preferably of vitamin E, particularly of gamma and/or beta-tocopherol, between 45% and 123% or more.

**[0046.0.9.9]** In one embodiment the activity of a Escherichia coli K12 protein b0161 as disclosed in [0016.0.9.9] or its homologs, as indicated in Table I, columns 5 or 7, lines 472 , e.g. a protein with an activity as defined in [0022.0.9.9], is increased conferring, preferably, an increase of the respective fine chemical, preferably of alpha-tocopherol and of further vitamin E activity-having compounds or their precursors.
In one embodiment the activity of a Escherichia coli K12 protein b0970 as disclosed in [0016.0.9.9] or its homologs, as indicated in Table I, columns 5 or 7, lines 473 , e.g. a protein with an activity as defined in [0022.0.9.9], is increased conferring preferably, an increase of the respective fine chemical, preferably of alpha-tocopherol and of further vitamin E activity-having compounds or their precursors.
In one embodiment the activity of a Escherichia coli K12 protein b3160 as disclosed in [0016.0.9.9] or its homologs, as indicated in Table I, columns 5 or 7, lines 474 , e.g. a protein with an activity as defined in [0022.0.9.9], is increased conferring preferably, an increase of the respective fine chemical, preferably of alpha-tocopherol and of further vitamin E activity-having compounds or their precursors.
In one embodiment the activity of a Escherichia coli K12 protein b4063 as disclosed in [0016.0.9.9] or its homologs, as indicated in Table I, columns 5 or 7, lines 475 , e.g. a protein with an activity as defined in [0022.0.9.9], is increased conferring, preferably, an increase of the respective fine chemical, preferably of alpha-tocopherol and of further vitamin E activity-having compounds or their precursors.
In one embodiment the activity of a Escherichia coli K12 protein b0785 as disclosed in [0016.0.9.9] or its homologs, as indicated in Table I, columns 5 or 7, lines 476 and 479, e.g. a protein with an activity as defined in [0022.0.9.9] is increased conferring, preferably, an increase of the respective fine chemical, preferably of beta-tocopherol and/or gamma-tocopherol and of further vitamin E activity-having compounds or their precursors.
In one embodiment, the activity of a Escherichia coli K12 protein b3938 as disclosed in [0016.0.9.9] or its homologs, as indicated in Table I, columns 5 or 7, lines 477 and 481, e.g. a protein with an activity as defined in [0022.0.9.9] is increased conferring, preferably, an increase of the respective fine chemical, preferably of beta-tocopherol and/or gamma-tocopherol and of further vitamin E activity-having compounds or their precursors.
In one embodiment, the activity of the Escherichia coli K12 protein b1829 or its homologs, e.g. having a heat shock protein with protease activity, e.g. as indicated in Table I, columns 5 or 7, line 91, 94, or 100, is increased conferring an increase of the respective fine chemical, preferably of alpha-, beta- and/or gamma-tocopherol and/or 2,3-Dimethyl-5-phyhtylquinol, and of further vitamin E activity-having compounds or their precursors.
In one embodiment, the activity of the Escherichia coli K12 protein b1827 or its homologs, e.g. with a putative transcriptional repressor with DNA-binding Winged helix domain (IcIR family) activity, e.g. as indicated in Table I, columns 5 or 7, line 92 or 97 is increased, conferring preferably, an increase of the respective fine chemical, preferably of beta-tocopherol and/or 2,3-Dimethyl-5-phyhtylquinol, and of further vitamin E activity-having compounds or their precursors.
In one embodiment, in case the activity of the Escherichia coli K12 protein b2699 or its homologs, e.g. a protein with a DNA strand exchange and recombination protein with protease und nuclease-activity, e.g. as indicated in Table I, columns 5 or 7, line 93, is increased conferring, preferably, an increase of the respective fine chemical, preferably of alpha-tocopherol and of further vitamin E activity-having compounds or their precursors.
In one embodiment, in case the activity of the Escherichia coli K12 protein b0112 or its homologs, e.g. a protein with an aromatic amino acid transport protein (APC family)-activity, e.g. as indicated in Table I, columns 5 or 7, line 95, is increased conferring, preferably, an increase of the respective fine chemical, preferably of alpha-tocopherol, and of further vitamin E activity-having compounds or their precursors.
In one embodiment, in case the activity of the Escherichia coli K12 protein b0175 or its homologs, e.g. a CDP-diglyceride synthase-activity, e.g. as indicated in Table I, columns 5 or 7, line 99 or 478, is increased conferring, preferably, an increase of the respective fine chemical, preferably of beta-tocopherol and/or gamma-tocopherol, and of further vitamin E activity-having compounds or their precursor.
In one embodiment, in case the activity of the Escherichia coli K12 protein b0986 or its homologs, e.g. a lipoprotein, e.g. as indicated in Table I, columns 5 or 7, line 98 and 480, is increased conferring, preferably, an increase of the respective fine chemical, preferably of beta-tocopherol and/or gamma-tocopherol, and of further vitamin E-activity having compounds or their precursors.
In one embodiment, in case the activity of the Saccharomyces cerevisiae protein YFL013C or its homologs, e.g. an activity of a subunit of the INO80 chroamtion remodeling complexes, in particular, of the superfamiliy of the membrane protein YFL013c, e.g. as indicated in Table I, columns 5 or 7, 90, is increased conferring, preferably, an increase of the respective fine chemical, preferably of 2,3-Dimethyl-5-phyhtylquinol, and of further vitamin E activity having-compounds or their precursors.
In one embodiment, in case the activity of the Saccharomyces cerevisiae protein YFL053W or its homologs, e.g. an activity of a dihydroxaacetone kinase, in particular, of the superfamiliy of theglyerone kinase DAK1, e.g. as indicated in Table I, columns 5 or 7, 96 or 101, is increased conferring, preferably, an increase of the respective fine chemical, preferably of beta- and/or gamma-tocopherol, and of further vitamin E activity-having compounds or their precursors.
In one embodiment, in case the activity of the Saccharomyces cerevisiae protein YPL268W or its homologs, e.g. an activity of a phophoinositide phopholipase, in particular, of the superfamiliy of the yeast 1-phosphatidylinositol-4,5-bisphosphate phophodiesterase, e.g. as indicated in Table I, columns 5 or 7, 89,is increased conferring, preferably, an increase of the respective fine chemical, preferably of 2,3-Dimethyl-5-phyhtylquinol, and of further vitamin E activity-having compounds or their precursors.

**[0047.0.0.9]** to **[0048.0.0.9]: see [0047.0.0.0]** to **[0048.0.0.0]**

**[0049.0.9.9]** A protein having an activity conferring an increase in the amount or level of the 2,3-dimethyl-5-phytylquinol preferably has the structure of the polypeptide described herein. In a particular embodiment, the polypeptides used in the process of the present invention or the polypeptide of the present invention comprises the sequence of a consensus sequence as indicated in Table IV, columns 7, lines 89 to 92 or 482 and/or the sequence of a polypeptide as indicated in Table II, columns 5 or 7, lines 89 to 92 or 482, or of a functional homologue thereof as described herein, or of a polypeptide which is encoded by the nucleic acid molecule characterized herein or the nucleic acid molecule according to the invention, for example by a nucleic acid molecule as indicated in Table I, columns 5 or 7, lines 89 to 92 or 482 or its herein described functional homologues and has the herein mentioned activity conferring an increase in the beta-2,3-dimethyl-5-phytylquinol level.
A protein having an activity conferring an increase in the amount or level of the alpha-tocopherol preferably has the structure of the polypeptide described herein. In a particular embodiment, the polypeptides used in the process of the present invention or the polypeptide of the present invention comprises the sequence of a consensus sequence as indicated in Table IV, columns 7, lines 93 to 95 or 472 to 475 and/or the sequence of a polypeptide as indicated in Table II, columns 5 or 7, lines 93 to 95 or 472 to 475 or of a functional homologue thereof as described herein, or of a polypeptide which is encoded by the nucleic acid molecule characterized herein or the nucleic acid molecule according to the invention, for example by a nucleic acid molecule as indicated in Table I, columns 5 or 7, lines 93 to 95 or 472 to 475 or its herein described functional homologues and has the herein mentioned activity confering an increase in the alpha-tocopherol level.
A protein having an activity conferring an increase in the amount or level of the beta-tocopherol preferably has the structure of the polypeptide described herein. In particular embodiment, the polypeptides used in the process of the present invention or the polypeptide of the present invention comprises the sequence of a consensus sequence as indicated in Table IV, columns 7, lines 96 to 100 or 476 to 477 and/or the sequence of a polypeptide as indicated in Table II, columns 5 or 7, lines 96 to 100 or 476 to 477 or of a functional homologue thereof as described herein, or of a
polypeptide encoded by the nucleic acid molecule characterized herein or the nucleic acid molecule according to the invention, for example by a nucleic acid molecule as indicated in Table I, columns 5 or 7, lines 96 to 100 or 476 to 477 or its herein described functional homologues and has the herein mentioned activity confering an increase in the beta-tocopherol level.
A protein having an activity conferring an increase in the amount or level of the gamma-tocopherol preferably has the structure of the polypeptide described herein. In a particular embodiment, the polypeptides used in the process of the present invention or the polypeptide of the present invention comprises the sequence of a consensus sequence as indicated in Table IV, columns 7, lines 101 to 102 or 478 to 481 and/or the sequence of a polypeptide as indicated in Table II, columns 5 or 7, lines 101 to 102 or 478 to 481 or of a functional homologue thereof as described herein, or of a polypeptide encoded by the nucleic acid molecule characterized herein or the nucleic acid molecule according to the invention, for example by a nucleic acid molecule as indicated in Table I, columns 5 or 7, lines 101 to 102 or 478 to 481 or its herein described functional homologues and has the herein mentioned activity confering an increase in the gamma-tocopherol level.

**[0050.0.9.9]** For the purposes of the present invention, the term "the respective fine chemical" also encompass the corresponding salts, such as, for example, the potassium or sodium salts of vitamin E or its precursor 2,3-dimethyl-5- phytylquinol, resp., or their ester.

**[0051.0.9.9]** Owing to the biological activity of the proteins which are used in the process according to the invention and which are encoded by nucleic acid molecules according to the invention, it is possible to produce compositions comprising the respective fine chemical, i.e. an increased amount of the free chemical free or bound, e.g compositions comprising vitamin E or its precursor 2,3-dimethyl-5- phytylquinol, resp.,. Depending on the choice of the organism used for the process according to the present invention, for example a microorganism or a plant, compositions or mixtures of various vitamin E compounds or their precursor 2,3-dimethyl-5- phytylquinol, resp., can be produced.

**[0052.0.0.9]** see **[0052.0.0.0]**

**[0053.0.9.9]** In one embodiment, the process of the present invention comprises one or more of the following steps
a) stabilizing a protein conferring the increased expression of a protein encoded by the nucleic acid molecule of the invention or of the polypeptide of the invention or the polypeptide used in the method of the invention, e.g. of a polypeptide having an activity of a protein as indicated in Table II, column 3, lines 89 to 92 or 482 or lines 93 to 95 or 472 to 475 or lines 96 to 100 or 476 to 477 or lines 101 to 102 or 478 to 481 or its homologs, e.g. as indicated in Table II, columns 5 or 7, lines 89 to 92 or 482 or lines 93 to 95 or 472 to 475 or lines 96 to 100 or 476 to 477 or line 101 to102 or lines 478 to 481, activity having herein-mentioned the respective fine chemical-increasing activity;
b) stabilizing a mRNA conferring the increased expression of a protein encoded by the nucleic acid molecule of the invention or the nucleic acid molecule used in the method of the invention, e.g. of a polypeptide having an activity of a protein as indicated in Table II, column 3, lines 89 to 92 or 482 or lines 93 to 95 or 472 to 475 or lines 96 to 100 or 476 to 477 or lines 101 to 102 or 478 to 481 or its homologs activity, e.g. as indicated in Table II, columns 5 or 7, lines 89 to 92 or 482 or lines 93 to 95 or 472 to 475 or lines 96 to 100 or 476 to 477 or lines 101 to 102 or 478 to 481, or of a mRNA encoding the polypeptide of the present invention having herein-mentioned the respective fine chemical-increasing activity;
c) increasing the specific activity of a protein conferring the increased expression of a protein encoded by the nucleic acid molecule of the invention or of the polypeptide of the present invention or the nucleic acid molecule or polypeptide used in the method of the invention having herein-mentioned the respective fine chemical-increasing activity, e.g. of a polypeptide having an activity of a protein as indicated in Table II, column 3, lines 89 to 92 or 482 or lines 93 to 95 or 472 to 475 or lines 96 to 100 or 476 to 477 or lines 101 to 102 or 478 to 481 or its homologs activity, e.g. as indicated in Table II, columns 5 or 7, lines 89 to 92 or 482 or lines 93 to 95 or 472 to 475 or lines 96 to 100 or 476 to 477 or lines 101 to 102 or 478 to 481, or decreasing the inhibitory regulation of the polypeptide of the invention or the polypeptide used in the method of the invention;
d) generating or increasing the expression of an endogenous or artificial transcription factor mediating the expression of a protein conferring the increased expression of a protein encoded by the nucleic acid molecule of the invention or the nucleic acid molecule used in the method of the invention or of the polypeptide of the invention or the polypeptide used in the method of the invention having herein-mentioned the respective fine chemical-increasing activity, e.g. of a polypeptide having an activity of a protein as indicated in Table II, column 3, lines 89 to 92 or 482 or lines 93 to 95 or 472 to 475 or lines 96 to 100 or 476 to 477 or lines 101 to 102 or 478 to 481 or its homologs activity, e.g. as indicated in Table II, columns 5 or 7, lines 89 to 92 or 482 or lines 93 to 95 or 472 to 475 or lines 96 to 100 or 476 to 477 or lines 101 to 102 or 478 to 481,
e) stimulating activity of a protein conferring the increased expression of a protein encoded by the nucleic acid molecule of the present invention or a polypeptide of the present invention having herein-mentioned the respective fine chemical-increasing activity, e.g. of a polypeptide having an activity of a protein as indicated in Table II, column 3, lines 89 to 92 or 482 or lines 93 to 95 or 472 to 475 or lines 96 to 100 or 476 to 477 or lines 101 to 102 or 478 to 481 or its homologs activity, e.g. as indicated in Table II, columns 5 or 7, lines 89 to 92 or 482 or lines 93 to 95 or 472 to 475 or lines 96 to 100 or 476 to 477 or lines 101 to 102 or 478 to 481, by adding one or more exogenous inducing factors to the organism or parts thereof;
f) expressing a transgenic gene encoding a protein conferring the increased expression of a polypeptide encoded by the nucleic acid molecule of the present invention or a polypeptide of the present invention, having herein-mentioned the respective fine chemical-increasing activity, e.g. of a polypeptide having an activity of a protein as indicated in Table II, column 3, lines 89 to 92 or 482 or lines 93 to 95 or 472 to 475 or lines 96 to 100 or 476 to 477 or lines 101 to 102 or 478 to 481 or its homologs activity, e.g. as indicated in Table II, columns 5 or 7, lines 89 to 92 or 482 or lines 93 to 95 or 472 to 475 or lines 96 to 100 or 476 to 477 or lines 101 to 102 or 478 to 481, and/or
g) increasing the copy number of a gene conferring the increased expression of a nucleic acid molecule encoding a polypeptide encoded by the nucleic acid molecule of the invention or the nucleic acid molecule used in the method of the invention or the polypeptide of the invention or the polypeptide used in the method of the invention having herein-mentioned the respective fine chemical-increasing activity, e.g. of a polypeptide having an activity of a protein as indicated in Table II, column 3, lines 89 to 92 or 482 or lines 93 to 95 or 472 to 475 or lines 96 to 100 or 476 to 477 or lines 101 to 102 or 478 to 481 or its homologs, e.g. as indicated in Table II, columns 5 or 7, lines 89 to 92 or 482 or lines 93 to 95 or 472 to 475 or lines 96 to 100 or 476 to 477 or lines 101 to 102 or 478 to 481, activity.
h) Increasing the expression of the endogenous gene encoding the polypeptide of the invention or the polypeptide used in the method of the invention, e.g. a polypeptide having an activity of a protein as indicated in Table II, column 3, lines 89 to 92 or 482 or lines 93 to 95 or 472 to 475 or lines 96 to 100 or 476 to 477 or lines 101 to 102 or 478 to 481 or its homologs activity, e.g. as indicated in Table II, columns 5 or 7, lines 89 to 92 or 482 or lines 93 to 95 or 472 to 475 or lines 96 to 100 or 476 to 477 or lines 101 to 102 or 478 to 481, by adding positive expression or removing negative expression elements, e.g. homologous recombination can be used to either introduce positive regulatory elements like for plants the 35S enhancer into the promoter or to remove repressor elements form regulatory regions. Further gene conversion methods can be used to disrupt repressor elements or to enhance to activity of positive elements. Positive elements can be randomly introduced in plants by T-DNA or transposon mutagenesis and lines can be identified in which the positive elements have be integrated near to a gene of the invention, the expression of which is thereby enhanced;
i) Modulating growth conditions of an organism in such a manner, that the expression or activity of the gene encoding the protein of the invention or the protein itself is enhanced for example microorganisms or plants can be grown for example under a higher temperature regime leading to an enhanced expression of heat shock proteins, which can lead an enhanced fine chemical production and/or
j) selecting of organisms with especially high activity of the proteins of the invention from natural or from mutagenized resources and breeding them into the target organisms, e.g. the elite crops.

**[0054.0.9.9]** Preferably, said mRNA is the nucleic acid molecule of the present invention and/or the protein conferring the increased expression of a protein encoded by the nucleic acid molecule of the present invention or the polypeptide having the herein mentioned activity is the polypeptide of the present invention, e.g. conferring the increase of the respective fine chemical after increasing the expression or activity of the encoded polypeptide or having the activity of a polypeptide having an activity of a protein as indicated in Table II, column 5 , lines 89 to 92 or 482 or lines 93 to 95 or 472 to 475 or lines 96 to 100 or 476 to 477 or lines 101 to 102 or 478 to 481, resp., or its homologs activity, e.g. as indicated in Table II, columnr 7, lines 89 to 92 or 482 or lines 93 to 95 or 472 to 475 or lines 96 to 100 or 476 to 477 or lines 101 to 102 or 478 to 481, resp.

**[0055.0.0.9]** to **[0067.0.0.9]: see [0055.0.0.0]** to **[0067.0.0.0]**

**[0068.0.9.9]** The mutation is introduced in such a way that the production of the vitamin E or its precursor 2,3-dimethyl-5-phytylquinol is not adversely affected.

**[0069.0.0.9] see [0069.0.0.0]**

**[0070.0.9.9]** Owing to the introduction of a gene or a plurality of genes conferring the expression of the nucleic acid molecule of the invention or the polypeptide of the invention or the nucleic acid molecule used in the method of the invention, for example the nucleic acid construct mentioned below, or encoding a protein of the invention or the polypeptide used in the method of the invention into an organism alone or in combination with other genes, it is possible not only to increase the biosynthetic flux towards the end product, but also to increase, modify or create *de novo* an advantageous, preferably novel metabolites composition in the organism, e.g. an advantageous composition of vitamins comprising a higher content of (from a viewpoint of nutrional physiology limited) of vitamin E or its precursor 2,3-dimethyl-5-phytylquinol.

**[0071.0.0.9] see [0071.0.0.0]**

**[0072.0.9.9]** By influencing the metabolism thus, it is possible to produce, in the process according to the invention, further advantageous compounds. Examples of such compounds are, in addition to of vitamin E or its precursor 2,3-dimethyl-5-phytylquinol, further vitamins or provitamins or carotenoids.

**[0073.0.9.9]** Accordingly, in one embodiment, the process according to the invention relates to a process, which comprises:
e) providing a non-human organism, preferably a microorganism, a non-human animal, a plant or animal cell, a plant or animal tissue or a plant;
f) increasing an activity of a polypeptide of the invention or the polypeptide used in the method of the invention or a homolog thereof, e.g. as indicated in Table II, columns 5 or 7, lines 89 to 92 or 482 or lines 93 to 95 or 472 to 475 or lines 96 to 100 or 476 to 477 or lines 101 to 102 or 478 to 481, or of a polypeptide being encoded by the nucleic acid molecule of the present invention and described below, e.g. conferring an increase of the respective fine chemical in an organism, preferably in a microorganism, a non-human animal, a plant or animal cell, a plant or animal tissue or a plant,
g) growing an organism, preferably a microorganism, a non-human animal, a plant or animal cell, a plant or animal tissue or a plant under conditions which permit the production of the respective fine chemical in the organism, preferably the microorganism, the plant cell, the plant tissue or the plant; and
h) if desired, recovering, optionally isolating, the free and/or bound the respective fine chemical and, optionally further free and/or bound vitamin E or its precursor 2,3-dimethyl-5-phytylquinol synthesized by the organism, the microorganism, the non-human animal, the plant or animal cell, the plant or animal tissue or the plant.

**[0074.0.9.9]** The organism, in particular the microorganism, non-human animal, the plant or animal cell, the plant or animal tissue or the plant is advantageously grown in such a way that it is not only possible to recover, if desired isolate the free or bound the respective fine chemical or the free and bound the respective fine chemical but as option it is also possible to produce, recover and, if desired isolate, other free or/and bound of vitamin E or its precursor 2,3-dimethyl-5-phytylquinol.

**[0075.0.0.9]** to **[0077.0.0.9]: see [0075.0.0.0]** to **[0077.0.0.0]**

**[0078.0.9.9]** The organism such as microorganisms or plants or the recovered, and if desired isolated, respective fine chemical can then be processed further directly into foodstuffs or animal feeds or for other applications, for example according to the disclosures made in6,399,059: Thermally stable enzyme composition and method of preparing the same, 6,361,800: Multi-vitamin and mineral supplement, 6,348,200: Cosmetic composition, 6,338,854: Photoaging skin-care preparation and method of treating wrinkled skin, 6,323,188: Treatment and prevention of cardiovascular diseases, heart attack, and stroke, primary and subsequent, with help of aspirin and certain vitamins, 6,299,896: Multi-vitamin and mineral supplement, 6,262,279: Preparation of tocopherols, 6,362,221: Compositions containing natural lycopene and natural tocopherol, 6,358,997: Tocopherol and tocotrienol compositions, 6,344,573: Process for extraction and concentration of liposoluble vitamins and provitamins, growth factors and animal and vegetable hormones from residues and by-products of industrialized animal and vegetable products, 6,242,227: Method of vitamin production, 6,207,187: Compositions based on tocopherols, 6,177,114: Refining of edible oil rich in natural carotenes and Vitamin E which are expressly incorporated herein by reference. The fermentation broth, fermentation products, plants or plant products can be purified as described in above mentioned applications or by other methods known to the person skilled in the art and described herein below.
In the method for producing vitamin E or its precursor 2,3-dimethyl-5-phytylquinol according to the invention, the cultivation step of the genetically modified organisms, also referred to as transgenic organisms herein below, is preferably followed by harvesting said organisms and isolating vitamin E from said organisms.
The organisms are harvested in a manner known per se and appropriate for the particular organism. Microorganisms such as bacteria, mosses, yeasts and fungi or plant cells which are cultured in liquid media by fermentation may be removed, for example, by centrifugation, decanting or filtration. Plants are grown on solid media in a manner known per se and harvested accordingly.
Vitamin E or its precursor 2,3-dimethyl-5-phytylquinol are isolated from the harvested biomass in a manner known per se, for example by extraction and, where appropriate, further chemical or physical purification processes such as, for example, precipitation methods, crystallography, thermal separation methods such as rectification methods or physical separation methods such as, for example, chromatography.
Vitamin E is isolated from oil-containing plants, for example, preferably by chemical conversion and distillation from vegetable oils or from the steam distillates obtained in the deodorization of vegetable oils (deodorizer condensates).
Further methods of isolating vitamin E from deodorizer condensates are described, for example, in DE 31 26 110 A1, EP 171 009 A2, GB 2 145 079, EP 333 472 A2 and WO 94/05650.
Products of these different work-up procedures are vitamin E or its precursor 2,3-dimethyl-5-phytylquinol, e.g. alpha, beta, or gamma-tocopherol or vitamin E or its precursor 2,3-dimethyl-5-phytylquinol comprising compositions, e.g. compositions comprising alpha, beta, or gamma-tocopherol which still comprise fermentation broth, plant particles and cell components in different amounts, advantageously in the range of from 0 to 99% by weight, preferably below 80% by weight, especially preferably between below 50% by weight.

**[0079.0.0.9]** to **[0084.0.0.9]: see [0079.0.0.0]** to **[0084.0.0.0]**

**[0084.1.9.9]** The invention also contemplates embodiments in which the vitamin E or its precursor 2,3-dimethyl-5-phytylquinol, or other vitamin E precursor compounds in the production of the respective fine chemical, is present in the phytosynthetically active organisms chosen as the host; for example, cyanobacteria, moses, algae or plants which, even as a wild type, are capable of producing vitamin E. The invention also contemplates embodiments in which a host lacks vitamin E or its precursor 2,3-dimethyl-5-phytylquinol or other vitamin E or its precursor 2,3-dimethyl-5-phytylquinol precursors, such as the vinca. In a plant of the latter type, the inserted DNA includes genes that code for proteins producing vitamin E precursors (compounds that can be converted biologically into a compound with vitamin E activity) and one or more modifying enzymes which were originally absent in such a plant.
Preferred monocotyledonous plants are selected in particular from the monocotyledonous crop plants such as, for example, of the family of Gramineae such as rice, corn, wheat or other cereals species such as barley, millet, rye, triticale or oats, and sugarcane, and all grass species. The invention is particularly applied to dicotyledonous plant organisms. Preferred dicotyledonous plants are in particular selected from the dicotyledonous crop plants such as, for example,
- Asteraceae such as sunflower, Tagetes or Calendula and many others,
- Compositae, especially the genus Lactuca, very especially the species sativa (lettuce) and many others,
- Cruciferae, especially the genus Brassica, very especially the species napus (oilseed rape), campestris (beet), oleracea (e.g. cabbage, cauliflower or broccoli and other cabbage types); and of the genus Arabidopsis, very especially the species thaliana, and cress or canola and many others,
- Cucurbitaceae such as melon, pumpkin or zucchini and many others,
- Leguminosae especially the genus Glycine, very especially the species max (soybean) soja and alfalfa, pea, beans or peanut and many others,
- Rubiaceae, preferably of the subclass Lamiidae such as, for example, Coffea arabica or Coffea liberica (coffee plant) and many others,
- Solanaceae especially the genus Lycopersicon, very especially the species esculentum (tomato) and the genus Solanum, very especially the species tuberosum (potato) and melongena (aubergine) and the genus Capsicum, very especially the species annum (paprika), and tobacco and many others,
- Sterculiaceae, preferably of the subclass Dilleniidae such as, for example, Theobroma cacao (cocoa plant) and many others,
- Theaceae, preferably of the subclass Dilleniidae such as, for example, Camellia sinensis or Thea sinensis (tea plant) and many others,
- Umbelliferae, especially the genus Daucus (very especially the species carota (carrot)) and Apium (very especially the species graveolens dulce (celeriac)) and many others;
and linseed, soybean, cotton, hemp, flax, cucumber, spinach, carrot, sugarbeet and the various tree, nut and grape species, especially banana and kiwi.
Also comprised are ornamental plants, productive or ornamental trees, flowers, cut flowers, shrubs or turf. Non-restrictive examples which may be mentioned are angiosperms, bryophytes such as, for example, Hepaticae (liverworts) and Musci (mosses); pteridophytes such as ferns, horsetail and club mosses; gymnosperms such as conifers, cycads, ginkgo and gnetales, the families of Rosaceae such as rose, Ericaceae such as rhododendrons and azaleas, Euphorbiaceae such as poinsettias and croton, Caryophyllaceae such as carnations, Solanaceae such as petunias, Gesneriaceae such as African violet, Balsaminaceae such as touch-me-not, Orchidaceae such as orchids, Iridaceae such as gladioli, iris, freesia and crocus, Compositae such as marigold, Geraniaceae such as geraniums, Liliaceae such as the dragon tree, Moraceae such as ficus, Araceae such as philodendron and many others.
Particularly preferred plant organisms are those selected from the group of oil plants consisting of Borago officinalis, Brassica campestris, Brassica napus, Brassica rapa, Cannabis sativa, Carthamus tinctorius, Cocos nucifera, Crambe abyssinica, Cuphea species, Elaeis guineensis, Ekeis oleiferu, Glycine max, Gossypium hirsitum, Gossypium barbadense, Gossypium herbaceum, Helianthus annus, Linum usitatissimum, Oenothera biennis, Ozea europea, Oryza sativa, Ricinus communis, Sesamum indicum, Triticum species, Zea maize, walnut and almond.
In one embodiment, preferred plants include, but are not limited to: Tagetes erecta, Brassica napus, Nicotinana tabacum, sunflower, canola, Solanum sp. (potato), and soybean.
Preferred cyanobacteria are cyanobacteria of the genus *Synechocystis, e.g. Synechocystis* sp. PCC 6803, or Physcometrella patens or Nostoc punctiforme ATCC 29133, Anabaena sp. PCC7120.
Preferred algae are green algae such as, for example, algae of the genus Chlorella, *Haematococcus, Phaedactylum tricornatum, Volvox* or *Dunaliella*, in particular e.g. Spongiococcum sp, e.g. Spongiococcum exentricum, Chlorella sp., Haematococcus, Phaedactylum tricornatum.

**[0085.0.9.9]** With regard to the nucleic acid sequence as depicted a nucleic acid construct which contains a nucleic acid sequence mentioned herein or an organism (= transgenic organism) which is transformed with said nucleic acid sequence or said nucleic acid construct, "transgene" means all those constructs which have been brought about by genetic manipulation methods,preferably in which either
a) a nucleic acid sequence as indicated in Table I, columns 5 or 7, lines 89 to 92 or 482 and/or lines 93 to 95 or 472 to 475 and/or lines 96 to 100 or 476 to 477 and/or line 101 to 102 or 478 to 481, resp., or a derivative thereof, or
b) a genetic regulatory element, for example a promoter, which is functionally linked to the nucleic acid sequence as indicated in Table I, columns 5 or 7, lines 89 to 92 or 482 and/or lines 93 to 95 or 472 to 475 and/or lines 96 to 100 or 476 to 477 and/or line 101 to 102 or 478 to 481, resp. or a derivative thereof, or
c) (a) and (b)

is/are not present in its/their natural genetic environment or has/have been modified by means of genetic manipulation methods, it being possible for the modification to be, by way of example, a substitution, addition, deletion, inversion or insertion of one or more nucleotide. "Natural genetic environment" means the natural chromosomal locus in the organism of origin or the presence in a genomic library. In the case of a genomic library, the natural, genetic environment of the nucleic acid sequence is preferably at least partially still preserved. The environment flanks the nucleic acid sequence at least on one side and has a sequence length of at least 50 bp, preferably at least 500 bp, particularly preferably at least 1000 bp, very particularly preferably at least 5000 bp.

**[0086.0.0.9]** to **[0087.0.0.9]: see [0086.0.0.0]** to **[0087.0.0.0]**

**[0088.0.9.9]** In an advantageous embodiment of the invention, the organism takes the form of a plant whose content the respective fine chemical is modified advantageously owing to the nucleic acid molecule of the present invention expressed. This is important for plant breeders since, for example, the nutritional value of plants for poultry is dependent on the abovementioned vitamin E and the general amount ofvitamin E as source in feed. Further, this is also important for the production of cosmetic compostions since, for example, the antioxidant level of plant extracts is depending on the abovementioned vitamin E and the general amount of vitamins e.g. as antioxidants.

**[0088.1.0.9]** see **[0088.1.0.0]**

**[0089.0.0.9]** to **[0090.0.0.9]: see [0089.0.0.0]** to **[0090.0.0.0]**

**[0091.0.9.9]** Thus, the content of plant components and preferably also further impurities is as low as possible, and the abovementioned vitamin E or its precursor 2,3-dimethyl-5-phytylquinol are obtained in as pure form as possible. In these applications, the content of plant components advantageously amounts to less than 10%, preferably 1%, more preferably 0.1 %, very especially preferably 0.01 % or less.

**[0092.0.0.9]** to **[0094.0.0.9]: see [0092.0.0.0]** to **[0094.0.0.0]**

**[0095.0.9.9]** It may be advantageous to increase the pool of said vitamin E or its precursor 2,3-dimethyl-5-phytylquinol in the transgenic organisms by the process according to the invention in order to isolate high amounts of the essentially pure fine chemical.

**[0096.0.9.9]** In another preferred embodiment of the invention a combination of the increased expression of the nucleic acid sequence or the protein of the invention together with the transformation of a protein or polypeptid or a compound, which functions as a sink for the desired fine chemical, for example vitamin E or its precursor 2,3-dimethyl-5-phytylquinol in the organism, is useful to increase the production of the respective fine chemical.

**[0097.0.0.9] see [0097.0.0.0]**

**[0098.0.9.9]** In a preferred embodiment, the respective fine chemical (vitamin E or its precursor 2,3-dimethyl-5-phytylquinol) is produced in accordance with the invention and, if desired, is isolated. The production of further vitamins, provitamins or carotenoids, e.g. carotenes or xanthophylls, or mixtures thereof or mixtures with other compounds by the process according to the invention is advantageous.

**[0099.0.9.9]** In the case of the fermentation of microorganisms, the abovementioned vitamin E or its precursor 2,3-dimethyl-5-phytylquinolmay accumulate in the medium and/or the cells. If microorganisms are used in the process according to the invention, the fermentation broth can be processed after the cultivation. Depending on the requirement, all or some of the biomass can be removed from the fermentation broth by separation methods such as, for example, centrifugation, filtration, decanting or a combination of these methods, or else the biomass can be left in the fermentation broth. The fermentation broth can subsequently be reduced, or concentrated, with the aid of known methods such as, for example, rotary evaporator, thin-layer evaporator, falling film evaporator, by reverse osmosis or by nanofiltration. Afterwards advantageously further compounds for formulation can be added such as corn starch or silicates. This concentrated fermentation broth advantageously together with compounds for the formulation can subsequently be processed by lyophilization, spray drying, and spray granulation or by other methods. Preferably the respective fine chemical or the vitamin E or its precursor 2,3-dimethyl-5-phytylquinol comprising compositions are isolated from the organisms, such as the microorganisms or plants or the culture medium in or on which the organisms have been grown, or from the organism and the culture medium, in the known manner, for example via extraction, distillation, crystallization, chromatography or a combination of these methods. These purification methods can be used alone or in combination with the aforementioned methods such as the separation and/or concentration methods.

**[0100.0.9.9]** Transgenic plants which comprise the vitamin E or its precursor 2,3-dimethyl-5-phytylquinolsuch as alpha, beta, or gamma-tocopherol, synthesized in the process according to the invention can advantageously be marketed directly without there being any need for the oils, lipids or fatty acids synthesized to be isolated. Plants for the process according to the invention are listed as meaning intact plants and all plant parts, plant organs or plant parts such as leaf, stem, seeds, root, tubers, anthers, fibers, root hairs, stalks, embryos, calli, cotelydons, petioles, harvested material, plant tissue, reproductive tissue and cell cultures which are derived from the actual transgenic plant and/or can be used for bringing about the transgenic plant. In this context, the seed comprises all parts of the seed such as the seed coats, epidermal cells, seed cells, endosperm or embryonic tissue.
The site of vitamin E biosynthesis in plants is, inter alia, the leaf tissue so that the isolation of leafs makes sense. However, this is not limiting, since the expression may also take place in a tissue-specific manner in all of the remaining parts of the plant, in particular in fat-containing seeds. A further preferred embodiment therefore relates to a seed-specific isolation of vitamin E or its precursor 2,3-dimethyl-5-phytylquinol.
However, the respective fine chemical produced in the process according to the invention can also be isolated from the organisms, advantageously plants, in the form of their oils, fats, lipids, as extracts, e.g. ether, alcohol, or other organic solvents or water containing extract and/or free vitamin E or its precursor 2,3-dimethyl-5-phytylquinol. The respective fine chemical produced by this process can be obtained by harvesting the organisms, either from the crop in which they grow, or from the field. This can be done via pressing or extraction of the plant parts, e.g. in the plant seeds. To increase the efficiency of extraction it is beneficial to clean, to temper and if necessary to hull and to flake the plant material especially the seeds. E.g., oils, fats, and/or lipids comprising vitamin E or its precursor 2,3-dimethyl-5-phytylquinolcan be obtained by what is known as cold beating or cold pressing without applying heat. To allow for greater ease of disruption of the plant parts, specifically the seeds, they can previously be comminuted, steamed or roasted. Seeds, which have been pre-treated in this manner can subsequently be pressed or extracted with solvents such as warm hexane. The solvent is subsequently removed. In the case of microorganisms, the latter are, after harvesting, for example extracted directly without further processing steps or else, after disruption, extracted via various methods with which the skilled worker is familiar. Thereafter, the resulting products can be processed further, i.e. degummed and/or refined. In this process, substances such as the plant mucilages and suspended matter can be first removed. What is known as desliming can be affected enzymatically or, for example, chemico-physically by addition of acid such as phosphoric acid.
Because vitamin E or its precursor 2,3-dimethyl-5-phytylquinol in microorganisms may be localized intracellular, their recovery essentials comes down to the isolation of the biomass. Well-established approaches for the harvesting of cells include filtration, centrifugation and coagulation/flocculation as described herein. Determination of tocopherols in cells has been described by Tan and Tsumura 1989, see also Biotechnology of Vitamins, Pigments and Growth Factors, Edited by Erik J. Vandamme, London, 1989, p.96 to 103. Many further methods to determine the tocopherol content are known to the person skilled in the art.

**[0101.0.0.9] see [0101.0.0.0]**

**[0102.0.9.9]** Vitamin E or its precursor 2,3-dimethyl-5-phytylquinol can for example be analyzed advantageously via HPLC or GC separation methods and detected by MS or MSMS methods. The unambiguous detection for the presence of Vitamin E or its precursor 2,3-dimethyl-5-phytylquinol containing products can be obtained by analyzing recombinant organisms using analytical standard methods: GC, GC-MS or TLC, as described on several occasions by Christie and the references therein (1997, in: Advances on Lipid Methodology, Fourth Edition: Christie, Oily Press, Dundee, 119-169; 1998, Gaschromatographie-Massenspektrometrie-Verfahren [Gas chromatography/mass spectrometric methods], Lipide 33:343-353). The material to be analyzed can be disrupted by sonication, grinding in a glass mill, liquid nitrogen and grinding, cooking, or via other applicable methods; see also Biotechnology of Vitamins, Pigments and Growth Factors, Edited by Erik J. Vandamme, London, 1989, p.96 to 103.

**[0103.0.9.9]** In a preferred embodiment, the present invention relates to a process for the production of the respective fine chemical comprising or generating in an organism or a part thereof the expression of at least one nucleic acid molecule comprising a nucleic acid molecule selected from the group consisting of:
a) nucleic acid molecule encoding, preferably at least the mature form, of the polypeptide having a sequence as indicated in Table II, columns 5 or 7, lines 89 to 92 or 482 and/or lines 93 to 95 or 472 to 475 and/or lines 96 to 100 or 476 to 477 and/or line 101 to 102 or 478 to 481, resp. or a fragment thereof, which confers an increase in the amount of the respective fine chemical in an organism or a part thereof;
b) nucleic acid molecule comprising, preferably at least the mature form, of a nucleic acid molecule having a sequence as indicated in Table I, columns 5 or 7, lines 89 to 92 or 482 and/or lines 93 to 95 or 472 to 475 and/or lines 96 to 100 or 476 to 477 and/or line 101 to 102 or 478 to 481, resp.
c) nucleic acid molecule whose sequence can be deduced from a polypeptide sequence encoded by a nucleic acid molecule of (a) or (b) as result of the degeneracy of the genetic code and conferring an increase in the amount of the respective fine chemical in an organism or a part thereof;
d) nucleic acid molecule encoding a polypeptide which has at least 50% identity with the amino acid sequence of the polypeptide encoded by the nucleic acid molecule of (a) to (c) and conferring an increase in the amount of the respective fine chemical in an organism or a part thereof;
e) nucleic acid molecule which hybridizes with a nucleic acid molecule of (a) to (c) under stringent hybridisation conditions and conferring an increase in the amount of the respective fine chemical in an organism or a part thereof;
f) nucleic acid molecule encoding a polypeptide, the polypeptide being derived by substituting, deleting and/or adding one or more amino acids of the amino acid sequence of the polypeptide encoded by the nucleic acid molecules (a) to (d), preferably to (a) to (c) and conferring an increase in the amount of the respective fine chemical in an organism or a part thereof;
g) nucleic acid molecule encoding a fragment or an epitope of a polypeptide which is encoded by one of the nucleic acid molecules of (a) to (e), preferably to (a) to (c) and conferring an increase in the amount of the respective fine chemical in an organism or a part thereof;
h) nucleic acid molecule comprising a nucleic acid molecule which is obtained by amplifying nucleic acid molecules from a cDNA library or a genomic library using the primers pairs having a sequence as indicated in Table III, column 7, lines 89 to 92 or 482 and/or lines 93 to 95 or 472 to 475 and/or lines 96 to 100 or 476 to 477 and/or line 101 to 102 or 478 to 481, resp.and conferring an increase in the amount of the respective fine chemical in an organism or a part thereof;
i) nucleic acid molecule encoding a polypeptide which is isolated, e.g. from an expression library, with the aid of monoclonal antibodies against a polypeptide encoded by one of the nucleic acid molecules of (a) to (h), preferably to (a) to (c), and conferring an increase in the amount of the respective fine chemical in an organism or a part thereof;
j) nucleic acid molecule which encodes a polypeptide comprising the consensus sequence having a sequences as indicated in Table IV, column 7, lines 89 to 92 or 482 and/or lines 93 to 95 or 472 to 475 and/or lines 96 to 100 or 476 to 477 and/or line 101 to 102 or 478 to 481, resp., and conferring an increase in the amount of the respective fine chemical in an organism or a part thereof;
k) nucleic acid molecule comprising one or more of the nucleic acid molecule encoding the amino acid sequence of a polypeptide encoding a domain of the polypeptide indicated in Table II, columns 5 or 7, lines 89 to 92 or 482 and/or lines 93 to 95 or 472 to 475 and/or lines 96 to 100 or 476 to 477 and/or line 101 to 102 or 478 to 481, resp. and conferring an increase in the amount of the respective fine chemical in an organism or a part thereof; and
l) nucleic acid molecule which is obtainable by screening a suitable library under stringent conditions with a probe comprising one of the sequences of the nucleic acid molecule of (a) to (k), preferably to (a) to (c), or with a fragment of at least 15 nt, preferably 20 nt, 30 nt, 50 nt, 100 nt, 200 nt or 500 nt of the nucleic acid molecule characterized in (a) to (k), preferably to (a) to (c), and conferring an increase in the amount of the respective fine chemical in an organism or a part thereof;
or which comprises a sequence which is complementary thereto.

**[0103.1.9.9]** In one embodiment, the nucleic acid molecule used in the process of the invention distinguishes over the sequence indicated in Table I A, columns 5 or 7, lines 89 to 92 or 482 and/or lines 93 to 95 or 472 to 475 and/or lines 96 to 100 or 476 to 477 and/or line 101 to 102 or 478 to 481, resp., by one or more nucleotides. In one embodiment, the nucleic acid molecule used in the process of the invention does not consist of the sequence indicated in Table I A, columns 5 or 7, lines 89 to 92 or 482 and/or lines 93 to 95 or 472 to 475 and/or lines 96 to 100 or 476 to 477 and/or line 101 to 102 or 478 to 481, resp.: In one embodiment, the nucleic acid molecule used in the process of the invention is less than 100%, 99,999%, 99,99%, 99,9% or 99% identical to a sequence indicated in Table I A, columns 5 or 7, lines 89 to 92 or 482 and/or lines 93 to 95 or 472 to 475 and/or lines 96 to 100 or 476 to 477 and/or line 101 to 102 or 478 to 481, resp.. In another embodiment, the nucleic acid molecule does not encode a polypeptide of a sequence indicated in Table II A, columns 5 or 7, lines 89 to 92 or 482 and/or lines 93 to 95 or 472 to 475 and/or lines 96 to 100 or 476 to 477 and/or line 101 to 102 or 478 to 481, resp..

**[0103.2.9.9.]** In one embodiment, the nucleic acid molecule used in the process of the invention distinguishes over a or more sequence(s) indicated in Table I B, column 7, lines 89 to 92 or 482 and/or lines 93 to 95 or 472 to 475 and/or lines 96 to 100 or 476 to 477 and/or line 101 to 102 or 478 to 481, resp., by one or more nucleotides. In one embodiment, the nucleic acid molecule used in the process of the invention does not consist of a or more sequence(s) indicated in Table I B, column 7, lines 89 to 92 or 482 and/or lines 93 to 95 or 472 to 475 and/or lines 96 to 100 or 476 to 477 and/or line 101 to 102 or 478 to 481, resp.. In one embodiment, the nucleic acid molecule used in the process of the invention is less than 100%, 99,999%, 99,99%, 99,9% or 99% identical to a sequence indicated in Table I B, column 7, lines 89 to 92 or 482 and/or lines 93 to 95 or 472 to 475 and/or lines 96 to 100 or 476 to 477 and/or line 101 to 102 or 478 to 481, resp.. In another embodiment, the nucleic acid molecule does not encode a polypeptide of a sequence indicated in Table II B, column 7, lines 89 to 92 or 482 and/or lines 93 to 95 or 472 to 475 and/or lines 96 to 100 or 476 to 477 and/or line 101 to 102 or 478 to 481, resp..

**[0104.0.9.9]** In one embodiment, the nucleic acid molecule of the invention or used in the process distinguishes over the sequence indicated in Table I, columns 5 or 7, lines 89 to 92 or 482 and/or lines 93 to 95 or 472 to 475 and/or lines 96 to 100 or 476 to 477 and/or line 101 to 102 or 478 to 481, resp., by one or more nucleotides. In one embodiment, the nucleic acid molecule used in the process of the invention or the nucleic acid used in the process of the invention does not consist of the sequence indicated in Table I, columns 5 or 7, lines 89 to 92 or 482 and/or lines 93 to 95 or 472 to 475 and/or lines 96 to 100 or 476 to 477 and/or line 101 to 102 or 478 to 481, resp., In one embodiment, the nucleic acid molecule of the present invention is less than 100%, 99,999%, 99,99%, 99,9% or 99% identical to the sequence indicated in Table I, columns 5 or 7, lines 89 to 92 or 482 and/or lines 93 to 95 or 472 to 475 and/or lines 96 to 100 or 476 to 477 and/or line 101 to 102 or 478 to 481, resp. In another embodiment, the nucleic acid molecule does not encode a polypeptide of a sequence indicated in Table II, columns 5 or 7, lines 89 to 92 or 482 and/or lines 93 to 95 or 472 to 475 and/or lines 96 to 100 or 476 to 477 and/or line 101 to 102 or 478 to 481, resp.

**[0105.0.0.9]** to **[0107.0.0.9] see [0105.0.0.0]** to **[0107.0.0.0]**

**[0108.0.9.9]** Nucleic acid molecules with the sequence as indicated in Table I, columns 5 or 7, lines 89 to 92 or 482 and/or lines 93 to 95 or 472 to 475 and/or lines 96 to 100 or 476 to 477 and/or line 101 to 102 or 478 to 481, resp., nucleic acid molecules which are derived from an amino acid sequences as indicated in Table II, columns 5 or 7, lines 89 to 92 or 482 and/or lines 93 to 95 or 472 to 475 and/or lines 96 to 100 or 476 to 477 and/or line 101 to 102 or 478 to 481, resp., or from polypeptides comprising the consensus sequence as indicated in Table IV, column 7, lines 89 to 92 or 482 and/or lines 93 to 95 or 472 to 475 and/or lines 96 to 100 or 476 to 477 and/or line 101 to 102 or 478 to 481, resp. or their derivatives or homologues encoding polypeptides with the enzymatic or biological activity of an activity of a polypeptide as indicated in Table II, column 3, 5 or 7, lines 89 to 92 or 482 and/or lines 93 to 95 or 472 to 475 and/or lines 96 to 100 or 476 to 477 and/or line 101 to 102 or 478 to 481, resp., or conferring an increase of the respective fine chemical, meaning Vitamin E or its precursor 2,3-dimethyl-5-phytylquinol., in particular, alpha-, beta-, and/or gamma-tocopherol after increasing its expression or activity are advantageously increased in the process according to the invention.

**[0109.0.0.9] see [0109.0.0.0]**

**[0110.0.9.9]** Nucleic acid molecules, which are advantageous for the process according to the invention and which encode polypeptides with an activity of a polypeptide of the invention or the polypeptide used in the method of the invention or used in the process of the invention, e.g. of a protein as indicated in Table II, column 5, lines 89 to 92 or 482 and/or lines 93 to 95 or 472 to 475 and/or lines 96 to 100 or 476 to 477 and/or line 101 to 102 or 478 to 481, resp or being encoded by a nucleic acid molecule indicated in Table I, column 5, lines 89 to 92 or 482 and/or lines 93 to 95 or 472 to 475 and/or lines 96 to 100 or 476 to 477 and/or line 101 to 102 or 478 to 481, resp, or of its homologs, e.g. as indicated in Table I or Table II, column 7, lines 89 to 92 or 482 and/or lines 93 to 95 or 472 to 475 and/or lines 96 to 100 or 476 to 477 and/or line 101 to 102 or 478 to 481, resp., can be determined from generally accessible databases.

**[0111.0.0.9] see [0111.0.0.0]**

**[0112.0.9.9]** The nucleic acid molecules used in the process according to the invention take the form of isolated nucleic acid sequences, which encode polypeptides with an activity of a polypeptide as indicated in Table II, column 3, lines 89 to 92 or 482 and/or lines 93 to 95 or 472 to 475 and/or lines 96 to 100 or 476 to 477 and/or line 101 to 102 or 478 to 481, resp., or having the sequence of a polypeptide as indicated in Table II, columns 5 and 7, lines 89 to 92 or 482 and/or lines 93 to 95 or 472 to 475 and/or lines 96 to 100 or 476 to 477 and/or line 101 to 102 or 478 to 481, resp., and conferring an increase in the level of vitamin E or its precursor 2,3-dimethyl-5-phytylquinol resp., in particular, of alpha-, beta-, and/or gamma-tocopherol.

**[0113.0.0.9] to [0120.0.0.9]: see [0113.0.0.0] to [0120.0.0.0]**

**[0120.1.9.9]**: Production strains which are also advantageously selected in the process according to the invention are microorganisms selected from the group of green algae, like Spongioccoccum exentricum, Chlorella sorokiniana (pyrenoidosa, 7-11-05), or algae of the genus *Haematococcus, Phaedactylum tricornatum, Volvox* or *Dunaliella* or form the group of fungi like fungi belonging to the Daccrymycetaceae family, or non-photosynthetic bacteria, like methylotrophs, flavobacteria, actinomycetes, like streptomyces chrestomyceticus, Mycobacteria like Mycobacterim phlei, or Rhodobacter capsulatus. Thus, the invention also contemplates embodiments in which a host lacks vitamin E or its precursor 2,3-dimethyl-5-phytylquinol or other vitamin E or its precursor 2,3-dimethyl-5-phytylquinol precursors, such as the vinca. In a plant of the latter type, the inserted DNA includes genes that code for proteins producing vitamin E precursors (compounds that can be converted biologically into a cmpound with vitamin E activity) and one or more modifiying enzymes which were originally absent in such a plant.
The invention also contemplates embodiments in which the vitamin E or its precursor 2,3-dimethyl-5-phytylquinol, or other vitamin E precursor compounds in the production of the respective fine chemical, is present in the phytosynthetically active organisms chosen as the host; for example, cyanobacteria, moses, algae or plants which, even as a wild type, are capable of producing vitamin E.

**[0121.0.9.9]** However, it is also possible to use artificial sequences, which differ in one or more bases from the nucleic acid sequences found in organisms, or in one or more amino acid molecules from polypeptide sequences found in organisms, in particular from the polypeptide sequences indicated in Table II, columns 5 or 7, lines 89 to 92 or 482 and/or lines 93 to 95 or 472 to 475 and/or lines 96 to 100 or 476 to 477 and/or line 101 to 102 or 478 to 481, resp., or the functional homologues thereof as described herein, preferably conferring above-mentioned activity, i.e. conferring an increase in the level of 2,3-dimethyl-5-phytyhlquinol after increasing the activity of the polypeptide sequences indicated in Table II, columns 5 or 7, lines 89 to 92 or 482; or conferring increase in the level of alpha-tocopherol after increasing the activity of the polypeptide sequences indicated in Table II, columns 5 or 7, lines 93 to 95 or 472 to 475; or conferring increase in the level of beta-tocopherol after increasing the activity of the polypeptide sequences indicated in Table II, columns 5 or 7, lines 96 to 100 or 476 to 477; or conferring increase in the level of gamma-tocopherol after increasing the activity of the polypeptide sequences indicated in Table II, columns 5 or 7, lines 101 to 102 or 478 to 481.

**[0122.0.0.9] to [0127.0.0.9]: see [0122.0.0.0] to [0127.0.0.0]**

**[0128.0.9.9]** Synthetic oligonucleotide primers for the amplification, e.g. as the pairs indicated in Table III, column 7, lines 89 to 92 or 482 and/or lines 93 to 95 or 472 to 475 and/or lines 96 to 100 or 476 to 477 and/or line 101 to 102 or 478 to 481, resp. by means of polymerase chain reaction can be generated on the basis of a sequence shown herein, for example the sequence as indicated in Table I, columns 5 or 7, lines 89 to 92 or 482 and/or lines 93 to 95 or 472 to 475 and/or lines 96 to 100 or 476 to 477 and/or line 101 to 102 or 478 to 481, resp. or the sequences derived from a sequence as indicated in Table II, columns 5 or 7, lines 89 to 92 or 482 and/or lines 93 to 95 or 472 to 475 and/or lines 96 to 100 or 476 to 477 and/or line 101 to 102 or 478 to 481, resp.

**[0129.0.9.9]** Moreover, it is possible to identify conserved regions from various organisms by carrying out protein sequence alignments with the polypeptide used in the process of the invention, in particular with sequences of the polypeptide of the invention or the polypeptide used in the method of the invention, from which conserved regions, and in turn, degenerate primers can be derived. Conserved region for the polypeptide of the invention or the polypeptide used in the method of the invention are indicated in the alignments shown in the figures. Conserved regions are those, which show a very little variation in the amino acid sequence in one particular position of several homologs from different origin. The consensus sequence indicated in Table IV, column 7, lines 89 to 92 or 482 and/or lines 93 to 95 or 472 to 475 and/or lines 96 to 100 or 476 to 477 and/or line 101 to 102 or 478 to 481, resp. is derived from said alignments.

**[0130.0.9.9]** Degenerated primers can then be utilized by PCR for the amplification of fragments of novel proteins having above-mentioned activity, e.g. conferring the increase of vitamin E or its precursor 2,3-dimethyl-5-phytylquinol resp., in particular, of alpha-, beta-, and/or gamma-tocopherol after increasing the expression or activity of the protein comprising said fragment.

**[0131.0.0.9] to [0138.0.0.9]: see [0131.0.0.0] to [0138.0.0.0]**

**[0139.0.9.9]** Polypeptides having above-mentioned activity, i.e. conferring the increase of the respective fine chemical level, derived from other organisms, can be encoded by other DNA sequences which hybridize to a sequences indicated in Table I, columns 5 or 7, lines 89 to 92 or 482, preferably Table I B, column 7, lines 89 to 92 or 482 for 2,3-dimethyl-5-phytylquinol or indicated in Table I, columns 5 or 7,lines 93 to 95 or 472 to 475, preferably Table I B, column 7, lines 93 to 95 or 472 to 475 for alpha-tocopherol or indicated in Table I, columns 5 or 7,lines 96 to 100 or 476 to 477, preferably Table I B, column 7, lines 96 to 100 or 476 to 477 for beta-tocopherol or indicated in Table I, columns 5 or 7,lines 101 to 102 or 478 to 481, preferably Table I B , column 7,lines 96 to 100 or 476 to 477 for gamma-tocopherol under relaxed hybridization conditions and which code on expression for peptides having the respective fine chemical, i.e. vitamin E or its precursor 2,3-dimethyl-5-phytylquinol resp., in particular, of alpha-, beta-, and/or gamma-tocopherol, resp., increasing activity.

**[0140.0.0.9] to [0146.0.0.9]: see [0140.0.0.0] to [0146.0.0.0]**

**[0147.0.9.9]** Further, the nucleic acid molecule of the invention or used in the method of the invention comprises a nucleic acid molecule, which is a complement of one of the nucleotide sequences of above mentioned nucleic acid molecules or a portion thereof. A nucleic acid molecule which is complementary to one of the nucleotide sequences indicated in Table I, columns 5 or 7, lines 89 to 92 or 482, and/or lines 93 to 95 or 472 to 475 and/or lines 96 to 100 or 476 to 477 and/or line 101 to 102 or 478 to 481, resp., preferably Table I B, column 7, lines 89 to 92 or 482 and/or lines 93 to 95 or 472 to 475 and/or lines 96 to 100 or 476 to 477 and/or line 101 to 102 or 478 to 481, resp., is one which is sufficiently complementary to one of said nucleotide sequences such that it can hybridize to one of said nucleotide sequences, thereby forming a stable duplex. Preferably, the hybridisation is performed under stringent hybrization conditions. However, a complement of one of the herein disclosed sequences is preferably a sequence complement thereto according to the base pairing of nucleic acid molecules well known to the skilled person. For example, the bases A and G undergo base pairing with the bases T and U or C, resp. and visa versa. Modifications of the bases can influence the base-pairing partner.

**[0148.0.9.9]** The nucleic acid molecule of the invention or used in the method of the invention comprises a nucleotide sequence which is at least about 30%, 35%, 40% or 45%, preferably at least about 50%, 55%, 60% or 65%, more preferably at least about 70%, 80%, or 90%, and even more preferably at least about 95%, 97%, 98%, 99% or more homologous to a nucleotide sequence indicated in Table I, columns 5 or 7, lines 89 to 92 or 482 and/or lines 93 to 95 or 472 to 475 and/or lines 96 to 100 or 476 to 477 and/or line 101 to 102 or 478 to 481, resp., preferably Table I B, column 7, lines 89 to 92 or 482 and/or lines 93 to 95 or 472 to 475 and/or lines 96 to 100 or 476 to 477 and/or line 101 to 102 or 478 to 481, resp. or a portion thereof and preferably has above mentioned activity, in particular having a vitamin E or its precursor 2,3-dimethyl-5-phytylquinol resp., in particular, of alpha-, beta-, and/or gamma-tocopherol increasing activity after increasing the activity or an activity of a product of a gene encoding said sequences or their homologs.

**[0149.0.9.9]** The nucleic acid molecule of the invention or used in the method of the invention comprises a nucleotide sequence which hybridizes, preferably hybridizes under stringent conditions as defined herein, to one of the nucleotide sequences indicated in Table I, columns 5 or 7, lines 89 to 92 or 482 and/or lines 93 to 95 or 472 to 475 and/or lines 96 to 100 or 476 to 477 and/or line 101 to 102 or 478 to 481, resp. or a portion thereof and encodes a protein having above-mentioned activity, e.g. conferring increase of vitamin E or its precursor 2,3-dimethyl-5-phytylquinol resp., in particular, of alpha-, beta-, and/or gamma-tocopherol, resp., and optionally, the activity of a protein indicated in Table II.

**[00149.1.9.9]** Optionally, in one embodiment, the nucleotide sequence, which hybridises to one of the nucleotide sequences indicated in Table I, columns 5 or 7, lines 89 to 92 or 482 and/or lines 93 to 95 or 472 to 475 and/or lines 96 to 100 or 476 to 477 and/or line 101 to 102 or 478 to 481, resp., preferably Table I B, columns 7, lines 89 to 92 or 482 and/or lines 93 to 95 or 472 to 475 and/or lines 96 to 100 or 476 to 477 and/or line 101 to 102 or 478 to 481, resp., has further one or more of the activities annotated or known for the a protein as indicated in Table II, column 3, lines 89 to 92 or 482 and/or lines 93 to 95 or 472 to 475 and/or lines 96 to 100 or 476 to 477 and/or line 101 to 102 or 478 to 481.

**[0150.0.9.9]** Moreover, the nucleic acid molecule of the invention or used in the process of the invention can comprise only a portion of the coding region of one of the sequences indicated in Table I, columns 5 or 7, lines 89 to 92 or 482 and/or lines 93 to 95 or 472 to 475 and/or lines 96 to 100 or 476 to 477 and/or line 101 to 102 or 478 to 481, resp., preferably Table I B, column 7, lines 89 to 92 or 482 and/or lines 93 to 95 or 472 to 475 and/or lines 96 to 100 or 476 to 477 and/or line 101 to 102 or 478 to 481, resp. for example a fragment which can be used as a probe or primer or a fragment encoding a biologically active portion of the polypeptide of the present invention or of a polypeptide used in the process of the present invention, i.e. having above-mentioned activity, e.g. conferring an increase of vitamin E or its precursor 2,3-dimethyl-5-phytylquinol resp., in particular, of alpha-, beta-, and/or gamma-tocopherol resp., if its activity is increased. The nucleotide sequences determined from the cloning of the present protein-according-to-the-invention-encoding gene allows for the generation of probes and primers designed for use in identifying and/or cloning its homologues in other cell types and organisms. The probe/primer typically comprises substantially purified oligonucleotide. The oligonucleotide typically comprises a region of nucleotide sequence that hybridizes under stringent conditions to at least about 12, 15 preferably about 20 or 25, more preferably about 40, 50 or 75 consecutive nucleotides of a sense strand of one of the sequences set forth, e.g., as indicated in Table I, columns 5 or 7, lines 89 to 92 or 482 and/or lines 93 to 95 or 472 to 475 and/or lines 96 to 100 or 476 to 477 and/or line 101 to 102 or 478 to 481, resp., preferably Table I B, column 7, lines 89 to 92 or 482 and/or lines 93 to 95 or 472 to 475 and/or lines 96 to 100 or 476 to 477 and/or line 101 to 102 or 478 to 481, resp. an anti-sense sequence of one of the sequences, e.g., as indicated in Table I, columns 5 or 7, lines 89 to 92 or 482 and/or lines 93 to 95 or 472 to 475 and/or lines 96 to 100 or 476 to 477 and/or line 101 to 102 or 478 to 481, resp., preferably Table I B, column 7, lines 89 to 92 or 482 and/or lines 93 to 95 or 472 to 475 and/or lines 96 to 100 or 476 to 477 and/or line 101 to 102 or 478 to 481, resp. or naturally occurring mutants thereof. Primers based on a nucleotide of invention can be used in PCR reactions to clone homologues of the polypeptide of the invention or of the polypeptide used in the process of the invention, e.g. as the primers described in the examples of the present invention, e.g. as shown in the examples. A PCR with the primer pairs indicated in Table III, column 7, lines 89 to 92 or 482 and/or lines 93 to 95 or 472 to 475 and/or lines 96 to 100 or 476 to 477 and/or line 101 to 102 or 478 to 481, resp., can result in a fragment of a polynucleotide sequence as indicated in Table I, columns 5 or 7, lines 89 to 92 or 482 and/or lines 93 to 95 or 472 to 475 and/or lines 96 to 100 or 476 to 477 and/or line 101 to 102 or 478 to 481, resp., preferably Table I B, column 7, lines 89 to 92 or 482 and/or lines 93 to 95 or 472 to 475 and/or lines 96 to 100 or 476 to 477 and/or line 101 to 102 or 478 to 481, resp., or its gene product.

**[0151.0.0.9]** see **[0151.0.0.0]**

**[0152.0.9.9]** The nucleic acid molecule of the invention or the nucleic acid used in the method of the invention encodes a polypeptide or portion thereof which includes an amino acid sequence which is sufficiently homologous to an amino acid sequence as indicated in Table II, columns 5 or 7, lines 89 to 92 or 482 and/or lines 93 to 95 or 472 to 475 and/or lines 96 to 100 or 476 to 477 and/or line 101 to 102 or 478 to 481, resp., preferably Table II B, column 7, lines 89 to 92 or 482 and/or lines 93 to 95 or 472 to 475 and/or lines 96 to 100 or 476 to 477 and/or line 101 to 102 or 478 to 481, resp., such that the protein or portion thereof maintains the ability to participate in the respective fine chemical production, in particular an activity increasing the level of vitamin E or its precursor 2,3-dimethyl-5-phytylquinol resp., in particular, of alpha-, beta-, and/or gamma-tocopherol, resp., as mentioned above or as described in the examples in plants or microorganisms is comprised. **[0153.0.9.9]** As used herein, the language "sufficiently homologous" refers to proteins or portions thereof which have amino acid sequences which include a minimum number of identical or equivalent amino acid residues (e.g., an amino acid residue which has a similar side chain as an amino acid residue in one of the sequences of the polypeptide of the present invention) to an amino acid sequence as indicated in Table II, columns 5 or 7, lines 89 to 92 or 482 and/or lines 93 to 95 or 472 to 475 and/or lines 96 to 100 or 476 to 477 and/or line 101 to 102 or 478 to 481, resp., such that the protein or portion thereof is able to participate in the increase of the respective fine chemical production. In one embodiment, a protein or portion thereof as indicated in Table II, columns 5 or 7, lines 89 to 92 or 482 and/or lines 93 to 95 or 472 to 475 and/or lines 96 to 100 or 476 to 477 and/or line 101 to 102 or 478 to 481, resp., has for example an activity of a polypeptide as indicated in Table II, column 3, lines 89 to 92 or 482 and/or lines 93 to 95 or 472 to 475 and/or lines 96 to 100 or 476 to 477 and/or line 101 to 102 or 478 to 481, resp.

**[0154.0.9.9]** In one embodiment, the nucleic acid molecule of the present invention comprises a nucleic acid that encodes a portion of the protein of the present invention. The protein is at least about 30%, 35%, 40%, 45% or 50%, preferably at least about 55%, 60%, 65% or 70%, and more preferably at least about 75%, 80%, 85%, 90%, 91%, 92%, 93% or 94% and most preferably at least about 95%, 97%, 98%, 99% or more homologous to an entire amino acid sequence as indicated in Table II, columns 5 or 7, lines 89 to 92 or 482 and/or lines 93 to 95 or 472 to 475 and/or lines 96 to 100 or 476 to 477 and/or line 101 to 102 or 478 to 481, resp., and has above-mentioned activity, e.g. conferring preferably the increase of the respective fine chemical.

**[0155.0.0.9]** to **[0156.0.0.9]: see [0155.0.0.0]** to **[0156.0.0.0]**

**[0157.0.9.9]** The invention further relates to nucleic acid molecules that differ from one of the nucleotide sequences as indicated in Table I, columns 5 or 7, lines 89 to 92 or 482 and/or lines 93 to 95 or 472 to 475 and/or lines 96 to 100 or 476 to 477 and/or line 101 to 102 or 478 to 481, resp., (and portions thereof) due to degeneracy of the genetic code and thus encode a polypeptide of the present invention, in particular a polypeptide having above mentioned activity, e.g. conferring an increase in the respective fine chemical in a organism, e.g. as that polypeptides comprising a consensus sequence as indicated in Table IV, column 7, lines 89 to 92 or 482 and/or lines 93 to 95 or 472 to 475 and/or lines 96 to 100 or 476 to 477 and/or line 101 to 102 or 478 to 481, resp., or of the polypeptide as indicated in Table II, columns 5 or 7, lines 89 to 92 or 482 and/or lines 93 to 95 or 472 to 475 and/or lines 96 to 100 or 476 to 477 and/or line 101 to 102 or 478 to 481, resp., or the functional homologues. Advantageously, the nucleic acid molecule of the invention or used in the method of the invention comprises, or in an other embodiment has, a nucleotide sequence encoding a protein comprising, or in an other embodiment having, an amino acid sequence of a consensus sequences as indicated in Table IV, column 7, lines 89 to 92 or 482 and/or lines 93 to 95 or 472 to 475 and/or lines 96 to 100 or 476 to 477 and/or line 101 to 102 or 478 to 481, resp., or of the polypeptide as indicated in Table II, columns 5 or 7, lines 89 to 92 or 482 and/or lines 93 to 95 or 472 to 475 and/or lines 96 to 100 or 476 to 477 and/or line 101 to 102 or 478 to 481, resp., or the functional homologues. In a still further embodiment, the nucleic acid molecule of the invention encodes a full length protein which is substantially homologous to an amino acid sequence comprising a consensus sequence as indicated in Table IV, column 7, lines 89 to 92 or 482 and/or lines 93 to 95 or 472 to 475 and/or lines 96 to 100 or 476 to 477 and/or line 101 to 102 or 478 to 481, resp., or of a polypeptide as indicated in Table II, columns 5 or 7, lines 89 to 92 or 482 and/or lines 93 to 95 or 472 to 475 and/or lines 96 to 100 or 476 to 477 and/or line 101 to 102 or 478 to 481, resp., or the functional homologues. However, in a preferred embodiment, the nucleic acid molecule of the present invention does not consist of a sequence as indicated in Table I, columns 5 or 7, lines 89 to 92 or 482 and/or lines 93 to 95 or 472 to 475 and/or lines 96 to 100 or 476 to 477 and/or line 101 to 102 or 478 to 481, resp. , preferably of Table I A, columns 5 or 7, lines 89 to 92 or 482 and/or lines 93 to 95 or 472 to 475 and/or lines 96 to 100 or 476 to 477 and/or line 101 to 102 or 478 to 481. Preferably, the nucleic acid molecule of the invention is a functional homologue or identical to a nucleic acid molecule indicated in Table I B, column 7, lines 89 to 92 or 482 and/or lines 93 to 95 or 472 to 475 and/or lines 96 to 100 or 476 to 477 and/or line 101 to 102 or 478 to 481.

**[0158.0.0.9]** to **[0160.0.0.9]: see [0158.0.0.0]** to **[0160.0.0.0]**

**[0161.0.9.9]** Accordingly, in another embodiment, a nucleic acid molecule of the invention is at least 15, 20, 25 or 30 nucleotides in length. Preferably, it hybridizes under stringent conditions to a nucleic acid molecule comprising a nucleotide sequence of the nucleic acid molecule of the present invention or used in the process of the present invention, e.g. comprising a sequence as indicated in Table I, columns 5 or 7, lines 89 to 92 or 482 and/or lines 93 to 95 or 472 to 475 and/or lines 96 to 100 or 476 to 477 and/or line 101 to 102 or 478 to 481, resp. The nucleic acid molecule is preferably at least 20, 30, 50, 100, 250 or more nucleotides in length.

**[0162.0.0.9]** see **[0162.0.0.0]**

**[0163.0.9.9]** Preferably, nucleic acid molecule of the invention that hybridizes under stringent conditions to a sequence as indicated in Table I, columns 5 or 7, lines 89 to 92 or 482 and/or lines 93 to 95 or 472 to 475 and/or lines 96 to 100 or 476 to 477 and/or line 101 to 102 or 478 to 481, resp., corresponds to a naturally-occurring nucleic acid molecule of the invention. As used herein, a "naturally-occurring" nucleic acid molecule refers to an RNA or DNA molecule having a nucleotide sequence that occurs in nature (e.g., encodes a natural protein). Preferably, the nucleic acid molecule encodes a natural protein having above-mentioned activity, e.g. conferring the respective fine chemical increase after increasing the expression or activity thereof or the activity of a protein of the invention or used in the process of the invention.

**[0164.0.0.9] see [0164.0.0.0]**

**[0165.0.9.9]** For example, nucleotide substitutions leading to amino acid substitutions at "non-essential" amino acid residues can be made in a sequence of the nucleic acid molecule of the invention or used in the process of the invention, e.g. as indicated in Table I, preferably Table I B, columns 5 or 7, lines 89 to 92 or 482 and/or lines 93 to 95 or 472 to 475 and/or lines 96 to 100 or 476 to 477 and/or line 101 to 102 or 478 to 481, resp.

**[0166.0.0.9] to [0167.0.0.9]: see [0166.0.0.0] to [0167.0.0.0]**

**[0168.0.9.9]** Accordingly, the invention relates to nucleic acid molecules encoding a polypeptide having above-mentioned activity, e.g. conferring an increase in the respective fine chemical in an organisms or parts thereof that contain changes in amino acid residues that are not essential for said activity. Such polypeptides differ in amino acid sequence from a sequence contained in a sequence as indicated in Table II, columns 5 or 7, lines 89 to 92 or 482 and/or lines 93 to 95 or 472 to 475 and/or lines 96 to 100 or 476 to 477 and/or line 101 to 102 or 478 to 481, resp., preferably Table II B, column 7, lines 89 to 92 or 482 and/or lines 93 to 95 or 472 to 475 and/or lines 96 to 100 or 476 to 477 and/or line 101 to 102 or 478 to 481,resp., yet retain said activity described herein. The nucleic acid molecule can comprise a nucleotide sequence encoding a polypeptide, wherein the polypeptide comprises an amino acid sequence at least about 50% identical to an amino acid sequence as indicated in Table II, columns 5 or 7, lines 89 to 92 or 482 and/or lines 93 to 95 or 472 to 475 and/or lines 96 to 100 or 476 to 477 and/or line 101 to 102 or 478 to 481, resp., preferably Table II B, column 7, lines 89 to 92 or 482 and/or lines 93 to 95 or 472 to 475 and/or lines 96 to 100 or 476 to 477 and/or line 101 to 102 or 478 to 481, resp., and is capable of participation in the increase of production of the respective fine chemical after increasing its activity, e.g. its expression. Preferably, the protein encoded by the nucleic acid molecule is at least about 60% identical to a sequence as indicated in Table II, columns 5 or 7, lines 89 to 92 or 482 and/or lines 93 to 95 or 472 to 475 and/or lines 96 to 100 or 476 to 477 and/or line 101 to 102 or 478 to 481, resp., preferably Table II B, column 7, lines 89 to 92 or 482 and/or lines 93 to 95 or 472 to 475 and/or lines 96 to 100 or 476 to 477 and/or line 101 to 102 or 478 to 481, resp. more preferably at least about 70% identical to one of the sequences as indicated in Table II, columns 5 or 7, lines 89 to 92 or 482 and/or lines 93 to 95 or 472 to 475 and/or lines 96 to 100 or 476 to 477 and/or line 101 to 102 or 478 to 481, resp. even more preferably at least about 80%, 90%, 95% homologous to a sequence as indicated in Table II, columns 5 or 7, lines 89 to 92 or 482 and/or lines 93 to 95 or 472 to 475 and/or lines 96 to 100 or 476 to 477 and/or line 101 to 102 or 478 to 481, resp., preferably Table II B, column 7, lines 89 to 92 or 482 and/or lines 93 to 95 or 472 to 475 and/or lines 96 to 100 or 476 to 477 and/or line 101 to 102 or 478 to 481, resp. and most preferably at least about 96%, 97%, 98%, or 99% identical to the sequence as indicated in Table II, columns 5 or 7, lines 89 to 92 or 482 and/or lines 93 to 95 or 472 to 475 and/or lines 96 to 100 or 476 to 477 and/or line 101 to 102 or 478 to 481, resp., preferably Table II B, column 7, lines 89 to 92 or 482 and/or lines 93 to 95 or 472 to 475 and/or lines 96 to 100 or 476 to 477 and/or line 101 to 102 or 478 to 481.

**[0169.0.0.9]** to **[0172.0.0.9]: see [0169.0.0.0]** to **[0172.0.0.0]**

**[0173.0.9.9]** For example a sequence, which has 80% homology with sequence SEQ ID NO: 8673 at the nucleic acid level is understood as meaning a sequence which, upon comparison with the sequence SEQ ID NO: 8673 by the above Gap program algorithm with the above parameter set, has a 80% homology.

**[0174.0.0.9] see [0174.0.0.0]**

**[0175.0.9.9]** For example a sequence which has a 80% homology with sequence SEQ ID NO: 8674 at the protein level is understood as meaning a sequence which, upon comparison with the sequence SEQ ID NO: 8674 by the above program algorithm with the above parameter set, has a 80% homology.

**[0176.0.9.9]** Functional equivalents derived from one of the polypeptides as indicated in Table II, columns 5 or 7, lines 89 to 92 or 482 and/or lines 93 to 95 or 472 to 475 and/or lines 96 to 100 or 476 to 477 and/or line 101 to 102 or 478 to 481, resp., according to the invention by substitution, insertion or deletion have at least 30%, 35%, 40%, 45% or 50%, preferably at least 55%, 60%, 65% or 70% by preference at least 80%, especially preferably at least 85% or 90%, 91%, 92%, 93% or 94%, very especially preferably at least 95%, 97%, 98% or 99% homology with one of the polypeptides as indicated in Table II, columns 5 or 7, lines 89 to 92 or 482 and/or lines 93 to 95 or 472 to 475 and/or lines 96 to 100 or 476 to 477 and/or line 101 to 102 or 478 to 481, resp., according to the invention and are distinguished by essentially the same properties as a polypeptide as indicated in Table II, columns 5 or 7, lines 89 to 92 or 482 and/or lines 93 to 95 or 472 to 475 and/or lines 96 to 100 or 476 to 477 and/or line 101 to 102 or 478 to 481, resp.

**[0177.0.9.9]** Functional equivalents derived from a nucleic acid sequence as indicated in Table I, columns 5 or 7, lines 89 to 92 or 482 and/or lines 93 to 95 or 472 to 475 and/or lines 96 to 100 or 476 to 477 and/or line 101 to 102 or 478 to 481, resp., according to the invention by substitution, insertion or deletion have at least 30%, 35%, 40%, 45% or 50%, preferably at least 55%, 60%, 65% or 70% by preference at least 80%, especially preferably at least 85% or 90%, 91 %, 92%, 93% or 94%, very especially preferably at least 95%, 97%, 98% or 99% homology with one of the polypeptides as indicated in Table II, columns 5 or 7, lines 89 to 92 or 482 and/or lines 93 to 95 or 472 to 475 and/or lines 96 to 100 or 476 to 477 and/or line 101 to 102 or 478 to 481, resp., according to the invention and encode polypeptides having essentially the same properties as a polypeptide as indicated in Table II, columns 5 or 7, lines 89 to 92 or 482 and/or lines 93 to 95 or 472 to 475 and/or lines 96 to 100 or 476 to 477 and/or line 101 to 102 or 478 to 481, resp.

**[0178.0.0.9] see [0178.0.0.0]**

**[0179.0.9.9]** A nucleic acid molecule encoding an homolog to a protein sequence as indicated in Table II, columns 5 or 7, lines 89 to 92 or 482 and/or lines 93 to 95 or 472 to 475 and/or lines 96 to 100 or 476 to 477 and/or line 101 to 102 or 478 to 481, resp., preferably Table II B, column 7, lines 89 to 92 or 482 and/or lines 93 to 95 or 472 to 475 and/or lines 96 to 100 or 476 to 477 and/or line 101 to 102 or 478 to 481, resp., can be created by introducing one or more nucleotide substitutions, additions or deletions into a nucleotide sequence of the nucleic acid molecule of the present invention, in particular as indicated in Table I, columns 5 or 7, lines 89 to 92 or 482 and/or lines 93 to 95 or 472 to 475 and/or lines 96 to 100 or 476 to 477 and/or line 101 to 102 or 478 to 481, resp., preferably Table I B, column 7, lines 89 to 92 or 482 and/or lines 93 to 95 or 472 to 475 and/or lines 96 to 100 or 476 to 477 and/or line 101 to 102 or 478 to 481, resp., such that one or more amino acid substitutions, additions or deletions are introduced into the encoded protein. Mutations can be introduced into the encoding sequences of a sequences as indicated in Table I, columns 5 or 7, lines 89 to 92 or 482 and/or lines 93 to 95 or 472 to 475 and/or lines 96 to 100 or 476 to 477 and/or line 101 to 102 or 478 to 481, resp., preferably Table I B, column 7, lines 89 to 92 or 482 and/or lines 93 to 95 or 472 to 475 and/or lines 96 to 100 or 476 to 477 and/or line 101 to 102 or 478 to 481, resp., by standard techniques, such as site-directed mutagenesis and PCR-mediated mutagenesis.

**[0180.0.0.9] to [0183.0.0.9]: see [0180.0.0.0] to [0183.0.0.0]**

**[0184.0.9.9]** Homologues of the nucleic acid sequences used, with a sequence as indicated in Table I, columns 5 or 7, lines 89 to 92 or 482 and/or lines 93 to 95 or 472 to 475 and/or lines 96 to 100 or 476 to 477 and/or line 101 to 102 or 478 to 481, resp., or of the nucleic acid sequences derived from a sequences as indicated in Table II, columns 5 or 7, lines 89 to 92 or 482 and/or lines 93 to 95 or 472 to 475 and/or lines 96 to 100 or 476 to 477 and/or line 101 to 102 or 478 to 481, resp. comprise also allelic variants with at least approximately 30%, 35%, 40% or 45% homology, by preference at least approximately 50%, 60% or 70%, more preferably at least approximately 90%, 91%, 92%, 93%, 94% or 95% and even more preferably at least approximately 96%, 97%, 98%, 99% or more homology with one of the nucleotide sequences shown or the abovementioned derived nucleic acid sequences or their homologues, derivatives or analogues or parts of these. Allelic variants encompass in particular functional variants which can be obtained by deletion, insertion or substitution of nucleotides from the sequences shown, preferably from a sequence as indicated in Table I, columns 5 or 7, lines 89 to 92 or 482 and/or lines 93 to 95 or 472 to 475 and/or lines 96 to 100 or 476 to 477 and/or line 101 to 102 or 478 to 481, resp., preferably Table I B, column 7, lines 89 to 92 or 482 and/or lines 93 to 95 or 472 to 475 and/or lines 96 to 100 or 476 to 477 and/or line 101 to 102 or 478 to 481, resp., or from the derived nucleic acid sequences, the intention being, however, that the enzyme activity or the biological activity of the resulting proteins synthesized is advantageously retained or increased.

**[0185.0.9.9]** In one embodiment of the present invention, the nucleic acid molecule of the invention or used in the process of the invention comprises one or more sequences as indicated in Table I, columns 5 or 7, lines 89 to 92 or 482 and/or lines 93 to 95 or 472 to 475 and/or lines 96 to 100 or 476 to 477 and/or line 101 to 102 or 478 to 481, resp., preferably Table I B, column 7, lines 89 to 92 or 482 and/or lines 93 to 95 or 472 to 475 and/or lines 96 to 100 or 476 to 477 and/or line 101 to 102 or 478 to 481, resp., In one embodiment, it is preferred that the nucleic acid molecule comprises as little as possible other nucleotides not shown in any one of sequences as indicated in Table I, columns 5 or 7, lines 89 to 92 or 482 and/or lines 93 to 95 or 472 to 475 and/or lines 96 to 100 or 476 to 477 and/or line 101 to 102 or 478 to 481, resp., , preferably Table I B, column 7, lines 89 to 92 or 482 and/or lines 93 to 95 or 472 to 475 and/or lines 96 to 100 or 476 to 477 and/or line 101 to 102 or 478 to 481, resp.,. In one embodiment, the nucleic acid molecule comprises less than 500, 400, 300, 200, 100, 90, 80, 70, 60, 50 or 40 further nucleotides. In a further embodiment, the nucleic acid molecule comprises less than 30, 20 or 10 further nucleotides. In one embodiment, a nucleic acid molecule used in the process of the invention is identical to a sequences as indicated in Table I, columns 5 or 7, lines 89 to 92 or 482 and/or lines 93 to 95 or 472 to 475 and/or lines 96 to 100 or 476 to 477 and/or line 101 to 102 or 478 to 481, resp. , preferably Table I B, column 7, lines 89 to 92 or 482 and/or lines 93 to 95 or 472 to 475 and/or lines 96 to 100 or 476 to 477 and/or line 101 to 102 or 478 to 481, resp.,

**[0186.0.9.9]** Also preferred is that one or more nucleic acid molecule(s) used in the process of the invention encodes a polypeptide comprising a sequence as indicated in Table II, columns 5 or 7, lines 89 to 92 or 482 and/or lines 93 to 95 or 472 to 475 and/or lines 96 to 100 or 476 to 477 and/or line 101 to 102 or 478 to 481, resp., preferably Table II B, column 7, lines 89 to 92 or 482 and/or lines 93 to 95 or 472 to 475 and/or lines 96 to 100 or 476 to 477 and/or line 101 to 102 or 478 to 481, resp. In one embodiment, the nucleic acid molecule encodes less than 150, 130, 100, 80, 60, 50, 40 or 30 further amino acids. In a further embodiment, the encoded polypeptide comprises less than 20, 15, 10, 9, 8, 7, 6 or 5 further amino acids. In one embodiment, the encoded polypeptide used in the process of the invention is identical to the sequences as indicated in Table II, columns 5 or 7, lines 89 to 92 or 482 and/or lines 93 to 95 or 472 to 475 and/or lines 96 to 100 or 476 to 477 and/or line 101 to 102 or 478 to 481, resp., preferably Table II B, column 7, lines 89 to 92 or 482 and/or lines 93 to 95 or 472 to 475 and/or lines 96 to 100 or 476 to 477 and/or line 101 to 102 or 478 to 481, resp

**[0187.0.9.9]** In one embodiment, a nucleic acid molecule of the invention or used in the process encodes a polypeptide comprising the sequence as indicated in Table II, columns 5 or 7, lines 89 to 92 or 482 and/or lines 93 to 95 or 472 to 475 and/or lines 96 to 100 or 476 to 477 and/or line 101 to 102 or 478 to 481, resp., preferably Table IIB, column 7, lines 89 to 92 or 482 and/or lines 93 to 95 or 472 to 475 and/or lines 96 to 100 or 476 to 477 and/or line 101 to 102 or 478 to 481, resp, comprises less than 100 further nucleotides. In a further embodiment, said nucleic acid molecule comprises less than 30 further nucleotides. In one embodiment, the nucleic acid molecule used in the process is identical to a coding sequence as indicated in Table II, columns 5 or 7, lines 89 to 92 or 482 and/or lines 93 to 95 or 472 to 475 and/or lines 96 to 100 or 476 to 477 and/or line 101 to 102 or 478 to 481, resp., preferably Table II B, column 7, lines 89 to 92 or 482 and/or lines 93 to 95 or 472 to 475 and/or lines 96 to 100 or 476 to 477 and/or line 101 to 102 or 478 to 481, resp.

**[0188.0.9.9]** Polypeptides (= proteins), which still have the essential biological or enzymatic activity of the polypeptide of the present invention conferring an increase of the respective fine chemical i.e. whose activity is essentially not reduced, are polypeptides with at least 10% or 20%, by preference 30% or 40%, especially preferably 50% or 60%, very especially preferably 80% or 90 or more of the wild type biological activity or enzyme activity, advantageously, the activity is essentially not reduced in comparison with the activity of a polypeptide as indicated in Table II columns 5 or 7, lines 89 to 92 or 482 and/or lines 93 to 95 or 472 to 475 and/or lines 96 to 100 or 476 to 477 and/or line 101 to 102 or 478 to 481, resp., and is expressed under identical conditions.

In one embodiment, the polypeptide of the invention is a homolog consisting of or comprising the sequence as indicated in Table II B, column 7, lines 89 to 92 or 482 and/or lines 93 to 95 or 472 to 475 and/or lines 96 to 100 or 476 to 477 and/or line 101 to 102 or 478 to 481.

**[0189.0.9.9]** Homologues of a sequences as indicated in Table I, columns 5 or 7, lines 89 to 92 or 482 and/or lines 93 to 95 or 472 to 475 and/or lines 96 to 100 or 476 to 477 and/or line 101 to 102 or 478 to 481, resp., or of a derived sequences as indicated in Table II, columns 5 or 7, lines 89 to 92 or 482 and/or lines 93 to 95 or 472 to 475 and/or lines 96 to 100 or 476 to 477 and/or line 101 to 102 or 478 to 481,resp., also mean truncated sequences, cDNA, single-stranded DNA or RNA of the coding and noncoding DNA sequence. Homologues of said sequences are also understood as meaning derivatives, which comprise noncoding regions such as, for example, UTRs, terminators, enhancers or promoter variants. The promoters upstream of the nucleotide sequences stated can be modified by one or more nucleotide substitution(s), insertion(s) and/or deletion(s) without, however, interfering with the functionality or activity either of the promoters, the open reading frame (= ORF) or with the 3'-regulatory region such as terminators or other 3'regulatory regions, which are far away from the ORF. It is furthermore possible that the activity of the promoters is increased by modification of their sequence, or that they are replaced completely by more active promoters, even promoters from heterologous organisms. Appropriate promoters are known to the person skilled in the art and are mentioned herein below.

**[0190.0.0.9] to [0191.0.0.9] see [0190.0.0.0] to [0191.0.0.0]**

**[0191.1.9.9]** -/-

**[0192.0.0.9] to [0203.0.0.9] see [0192.0.0.0] to [0203.0.0.0]**

**[0204.0.9.9]** Accordingly, in one embodiment, the invention relates to a nucleic acid molecule, which comprises a nucleic acid molecule selected from the group consisting of:
a) nucleic acid molecule encoding, preferably at least the mature form, of a polypeptide as indicated in Table II, columns 5 or 7, lines 89 to 92 or 482 and/or lines 93 to 95 or 472 to 475 and/or lines 96 to 100 or 476 to 477 and/or line 101 to 102 or 478 to 481, resp., preferably Table II B, column 7, lines 89 to 92 or 482 and/or lines 93 to 95 or 472 to 475 and/or lines 96 to 100 or 476 to 477 and/or line 101 to 102 or 478 to 481, resp.; or a fragment thereof conferring an increase in the amount of the respective fine chemical, i.e. vitamin E or its precursor 2,3-dimethyl-5-phytylquinol (lines 89 to 92 or 482), resp., in particular, of alpha- (lines 93 to 95 or 472 to 475), beta- (lines 96 to 100 or 476 to 477), and/or gamma-tocopherol (lines 101 to 102 or 478 to 481), resp., in an organism or a part thereof
b) nucleic acid molecule comprising, preferably at least the mature form, of a nucleic acid molecule as indicated in Table I, columns 5 or 7, lines 89 to 92 or 482 and/or lines 93 to 95 or 472 to 475 and/or lines 96 to 100 or 476 to 477 and/or line 101 to 102 or 478 to 481, resp., preferably Table I B, column 7, lines 89 to 92 or 482 and/or lines 93 to 95 or 472 to 475 and/or lines 96 to 100 or 476 to 477 and/or line 101 to 102 or 478 to 481, resp., or a fragment thereof conferring an increase in the amount of the respective fine chemical in an organism or a part thereof;
c) nucleic acid molecule whose sequence can be deduced from a polypeptide sequence encoded by a nucleic acid molecule of (a) or (b) as result of the degeneracy of the genetic code and conferring an increase in the amount of the respective fine chemical in an organism or a part thereof;
d) nucleic acid molecule encoding a polypeptide whose sequence has at least 50% identity with the amino acid sequence of the polypeptide encoded by the nucleic acid molecule of (a) to (c) and conferring an increase in the amount of the respective fine chemical in an organism or a part thereof;
e) nucleic acid molecule which hybridizes with a nucleic acid molecule of (a) to (c) under stringent hybridisation conditions and conferring an increase in the amount of the respective fine chemical in an organism or a part thereof;
f) nucleic acid molecule encoding a polypeptide, the polypeptide being derived by substituting, deleting and/or adding one or more amino acids of the amino acid sequence of the polypeptide encoded by the nucleic acid molecules (a) to (d), preferably to (a) to (c), and conferring an increase in the amount of the respective fine chemical in an organism or a part thereof;
g) nucleic acid molecule encoding a fragment or an epitope of a polypeptide which is encoded by one of the nucleic acid molecules of (a) to (e), preferably to (a) to (c) and conferring an increase in the amount of the respective fine chemical in an organism or a part thereof;
h) nucleic acid molecule comprising a nucleic acid molecule which is obtained by amplifying a cDNA library or a genomic library using primers or primer pairs as indicated in Table III, column 7, lines 89 to 92 or 482 and/or lines 93 to 95 or 472 to 475 and/or lines 96 to 100 or 476 to 477 and/or line 101 to 102 or 478 to 481, resp., and conferring an increase in the amount of the respective fine chemical, i.e. vitamin E or its precursor 2,3-dimethyl-5-phytylquinol (lines 89 to 92 or 482), resp., in particular, of alpha- (lines 93 to 95 or 472 to 475), beta- (lines 96 to 100 or 476 to 477), and/or gamma-tocopherol (lines 101 to 102 or 478 to 481), resp. in an organism or a part thereof;
i) nucleic acid molecule encoding a polypeptide which is isolated, e.g. from a expression library, with the aid of monoclonal antibodies against a polypeptide encoded by one of the nucleic acid molecules of (a) to (g), preferably to (a) to (c) and conferring an increase in the amount of the respective fine chemical in an organism or a part thereof;
j) nucleic acid molecule which encodes a polypeptide comprising a consensus sequence as indicated in Table IV, columns 7, lines 89 to 92 or 482 and/or lines 93 to 95 or 472 to 475 and/or lines 96 to 100 or 476 to 477 and/or line 101 to 102 or 478 to 481, resp., and conferring an increase in the amount of the respective fine chemical, i.e. vitamin E or its precursor 2,3-dimethyl-5-phytylquinol (lines 89 to 92 or 482), resp., in particular, of alpha- (lines 93 to 95 or 472 to 475), beta-(lines 96 to 100 or 476 to 477), and/or gamma-tocopherol (lines 101 to 102 or 478 to 481), resp., in an organism or a part thereof;
k) nucleic acid molecule encoding the amino acid sequence of a polypeptide encoding a domain of a polypeptide as indicated in Table II, columns 5 or 7, lines 89 to 92 or 482 and/or lines 93 to 95 or 472 to 475 and/or lines 96 to 100 or 476 to 477 and/or line 101 to 102 or 478 to 481, resp., preferably Table II B, column 7, lines 89 to 92 or 482 and/or lines 93 to 95 or 472 to 475 and/or lines 96 to 100 or 476 to 477 and/or line 101 to 102 or 478 to 481, resp., and conferring an increase in the amount of the respective fine chemical, i.e. vitamin E or its precursor 2,3-dimethyl-5-phytylquinol (lines 89 to 92 or 482), resp., in particular, of alpha- (lines 93 to 95 or 472 to 475), beta- (lines 96 to 100 or 476 to 477), and/or gamma-tocopherol (lines 101 to 102 or 478 to 481), resp., in an organism or a part thereof; and
l) nucleic acid molecule which is obtainable by screening a suitable nucleic acid library under stringent hybridization conditions with a probe comprising one of the sequences of the nucleic acid molecule of (a) to (k) or with a fragment of at least 15 nt, preferably 20 nt, 30 nt, 50 nt, 100 nt, 200 nt or 500 nt of the nucleic acid molecule characterized in (a) to (h) or of a nucleic acid molecule as indicated in Table I, columns 5 or 7, lines 89 to 92 or 482 and/or lines 93 to 95 or 472 to 475 and/or lines 96 to 100 or 476 to 477 and/or line 101 to 102 or 478 to 481, resp., preferably Table I B, column 7, lines 89 to 92 or 482 and/or lines 93 to 95 or 472 to 475 and/or lines 96 to 100 or 476 to 477 and/or line 101 to 102 or 478 to 481, resp., or a nucleic acid molecule encoding, preferably at least the mature form of, a polypeptide as indicated in Table II, columns 5 or 7, lines 89 to 92 or 482 and/or lines 93 to 95 or 472 to 475 and/or lines 96 to 100 or 476 to 477 and/or line 101 to 102 or 478 to 481, resp., preferably Table II B, column 7, lines 89 to 92 or 482 and/or lines 93 to 95 or 472 to 475 and/or lines 96 to 100 or 476 to 477 and/or line 101 to 102 or 478 to 481, resp. and conferring an increase in the amount of the respective fine chemical in an organism or a part thereof; or which encompasses a sequence which is complementary thereto;
whereby, preferably, the nucleic acid molecule according to (a) to (I) distinguishes over a sequence as indicated in Table I A, columns 5 or 7, lines 89 to 92 or 482 and/or lines 93 to 95 or 472 to 475 and/or lines 96 to 100 or 476 to 477 and/or line 101 to 102 or 478 to 481, resp., by one or more nucleotides. In one embodiment, the nucleic acid molecule of the invention does not consist of the sequence as indicated in Table I, columns 5 or 7, lines 89 to 92 or 482 and/or lines 93 to 95 or 472 to 475 and/or lines 96 to 100 or 476 to 477 and/or line 101 to 102 or 478 to 481, resp.,. In an other embodiment, the nucleic acid molecule of the present invention is at least 30 % identical and less than 100%, 99,999%, 99,99%, 99,9% or 99% identical to a sequence as indicated in Table I A, columns 5 or 7, lines 89 to 92 or 482 and/or lines 93 to 95 or 472 to 475 and/or lines 96 to 100 or 476 to 477 and/or line 101 to 102 or 478 to 481, resp. In a further embodiment the nucleic acid molecule does not encode a polypeptide sequence as indicated in Table II A, columns 5 or 7, lines 89 to 92 or 482 and/or lines 93 to 95 or 472 to 475 and/or lines 96 to 100 or 476 to 477 and/or line 101 to 102 or 478 to 481, resp. Accordingly, in one embodiment, the nucleic acid molecule of the present invention encodes in one embodiment a polypeptide which differs at least in one or more amino acids from a polypeptide indicated in Table II A, columns 5 or 7, lines 89 to 92 or 482 and/or lines 93 to 95 or 472 to 475 and/or lines 96 to 100 or 476 to 477 and/or line 101 to 102 or 478 to 481, resp., does not encode a protein of a sequence as indicated in Table II A, columns 5 or 7, lines 89 to 92 or 482 and/or lines 93 to 95 or 472 to 475 and/or lines 96 to 100 or 476 to 477 and/or line 101 to 102 or 478 to 481, resp. Accordingly, in one embodiment, the protein encoded by a sequences of a nucleic acid according to (a) to (I) does not consist of a sequence as indicated in Table II A, columns 5 or 7, lines 89 to 92 or 482 and/or lines 93 to 95 or 472 to 475 and/or lines 96 to 100 or 476 to 477 and/or line 101 to 102 or 478 to 481, resp. In a further embodiment, the protein of the present invention is at least 30 % identical to a protein sequence indicated in Table II A, columns 5 or 7, lines 89 to 92 or 482 and/or lines 93 to 95 or 472 to 475 and/or lines 96 to 100 or 476 to 477 and/or line 101 to 102 or 478 to 481, resp., and less than 100%, preferably less than 99,999%, 99,99% or 99,9%, more preferably less than 99%, 985, 97%, 96% or 95% identical to a sequence as indicated in Table II A, columns 5 or 7, lines 89 to 92 or 482 and/or lines 93 to 95 or 472 to 475 and/or lines 96 to 100 or 476 to 477 and/or line 101 to 102 or 478 to 481, resp.
whereby, in a further embodiment, the nucleic acid molecule according to (a) to (I) distinguishes over a sequence as indicated in Table I B, column 7, lines 89 to 92 or 482 and/or lines 93 to 95 or 472 to 475 and/or lines 96 to 100 or 476 to 477 and/or line 101 to 102 or 478 to 481, resp., by one or more nucleotides. In one embodiment, the nucleic acid molecule of the invention does not consist of the sequence as indicated in Table I B, column 7, lines 89 to 92 or 482 and/or lines 93 to 95 or 472 to 475 and/or lines 96 to 100 or 476 to 477 and/or line 101 to 102 or 478 to 481, resp.,. In an further embodiment, the nucleic acid molecule of the present invention is at least 30 % identical and less than 100%, 99,999%, 99,99%, 99,9% or 99% identical to a sequence as indicated in Table I B, column 7, lines 89 to 92 or 482 and/or lines 93 to 95 or 472 to 475 and/or lines 96 to 100 or 476 to 477 and/or line 101 to 102 or 478 to 481, resp. In a further embodiment the nucleic acid molecule does not encode a polypeptide sequence as indicated in Table II B, column 7, lines 89 to 92 or 482 and/or lines 93 to 95 or 472 to 475 and/or lines 96 to 100 or 476 to 477 and/or line 101 to 102 or 478 to 481, resp. Accordingly, in one embodiment, the nucleic acid molecule of the present invention encodes in one embodiment a polypeptide which differs at least in one or more amino acids from a polypeptide indicated in Table II B, column 7, lines 89 to 92 or 482 and/or lines 93 to 95 or 472 to 475 and/or lines 96 to 100 or 476 to 477 and/or line 101 to 102 or 478 to 481, resp., does not encode a protein of a sequence as indicated in Table II B, columns 5 or 7, lines 89 to 92 or 482 and/or lines 93 to 95 or 472 to 475 and/or lines 96 to 100 or 476 to 477 and/or line 101 to 102 or 478 to 481, resp. Accordingly, in one further embodiment, the protein encoded by a sequences of a nucleic acid according to (a) to (I) does not consist of a sequence as indicated in Table II B, column 7, lines 89 to 92 or 482 and/or lines 93 to 95 or 472 to 475 and/or lines 96 to 100 or 476 to 477 and/or line 101 to 102 or 478 to 481, resp. In a further embodiment, the protein of the present invention is at least 30 % identical to a protein sequence indicated in Table II B, column 7, lines 89 to 92 or 482 and/or lines 93 to 95 or 472 to 475 and/or lines 96 to 100 or 476 to 477 and/or line 101 to 102 or 478 to 481, resp., and less than 100%, preferably less than 99,999%, 99,99% or 99,9%, more preferably less than 99%, 985, 97%, 96% or 95% identical to a sequence as indicated in Table II B, columns 5 or 7, lines 89 to 92 or 482 and/or lines 93 to 95 or 472 to 475 and/or lines 96 to 100 or 476 to 477 and/or line 101 to 102 or 478 to 481, resp.

**[0205.0.0.9] to [0206.0.0.9]: see [0205.0.0.0] to [0206.0.0.0]**

**[0207.0.9.9]** As described herein, the nucleic acid construct can also comprise further genes, which are to be introduced into the organisms or cells. It is possible and advantageous to introduce into, and express in, the host organisms regulatory genes such as genes for inductors, repressors or enzymes, which, owing to their enzymatic activity, engage in the regulation of one or more genes of a biosynthetic pathway. These genes can be of heterologous or homologous origin. Moreover, further biosynthesis genes may advantageously be present, or else these genes may be located on one or more further nucleic acid constructs. Genes, which are advantageously employed as biosynthesis genes, are genes of the tocopherol metabolism, the tocotriene metabolism, the carotenoid metabolism, the amino acid metabolism, of glycolysis, of the tricarboxylic acid metabolism or their combinations. As described herein, regulator sequences or factors can have a positive effect on preferably the gene expression of the genes introduced, thus increasing it. Thus, an enhancement of the regulator elements may advantageously take place at the transcriptional level by using strong transcription signals such as promoters and/or enhancers. In addition, however, an enhancement of translation is also possible, for example by increasing mRNA stability or by inserting a translation enhancer sequence.

**[0208.0.0.9] to [0226.0.0.9]: see [0208.0.0.0] to [0226.0.0.0]**

**[0227.0.9.9]** The abovementioned nucleic acid molecules can be cloned into the nucleic acid constructs or vectors according to the invention in combination together with further genes, or else different genes are introduced by transforming several nucleic acid constructs or vectors (including plasmids) into a host cell, advantageously into a plant cell or a microorganisms.
In addition to a sequence indicated in Table I, columns 5 or 7, lines 89 to 92 or 482 and/or lines 93 to 95 or 472 to 475 and/or lines 96 to 100 or 476 to 477 and/or line 101 to 102 or 478 to 481, resp., preferably Table I B, column 7, lines 89 to 92 or 482 and/or lines 93 to 95 or 472 to 475 and/or lines 96 to 100 or 476 to 477 and/or line 101 to 102 or 478 to 481, resp., or its derivatives, it is advantageous additionally to express and/or mutate further genes in the organisms. Especially advantageously, additionally at least one further gene of the tocopherol biosynthetic pathway such as for a vitamin E precursor, is expressed in the organisms such as plants or microorganisms. It is also possible that the regulation of the natural genes has been modified advantageously so that the gene and/or its gene product is no longer subject to the regulatory mechanisms which exist in the organisms. This leads to an increased synthesis of the amino acids desired since, for example, feedback regulations no longer exist to the same extent or not at all. In addition it might be advantageously to combine one or more of the sequences indicated in Table I, columns 5 or 7, lines 89 to 92 or 482 and/or lines 93 to 95 or 472 to 475 and/or lines 96 to 100 or 476 to 477 and/or line 101 to 102 or 478 to 481, resp., with genes which generally support or enhances to growth or yield of the target organism, for example genes which lead to faster growth rate of microorganisms or genes which produces stress-, pathogen, or herbicide resistant plants.

**[0228.0.9.9]** In a further embodiment of the process of the invention, therefore, organisms are grown, in which there is simultaneous overexpression of at least one nucleic acid or one of the genes which code for proteins involved in the tocopherol metabolism, in particular in synthesis of alpha-, beta-, and/or gamma-tocopherol.

**[0229.0.9.9]** Further advantageous nucleic acid sequences which can be expressed in combination with the sequences used in the process and/or the abovementioned biosynthesis genes are the sequences encoding further genes of the tocopherol biosynthetic pathway, such as the homogentisate phytyltransferase (HPT) or the enzymes catalysing the subsequent cyclization and methylation reactions, γ-tocopherol methyl transferase (γ-TMT), prenyltransferases that condense prenyl groups with allylic chains and those that condense prenyl chains with aromatic groups and others. These genes can lead to an increased synthesis of the essential vitamin E or its precursor 2,3-dimethyl-5-phytylquinol resp., in particular, of alpha-, beta-, and/or gamma-tocopherol, resp.

**[0230.0.0.9] see [0230.0.0.0]**

**[0231.0.9.9]** In a further advantageous embodiment of the process of the invention, the organisms used in the process are those in which simultaneously a protein degrading vitamin E or its precursor 2,3-dimethyl-5-phytylquinol resp., in particular, alpha-, beta-, and/or gamma-tocopherol, resp., is attenuated, in particular by reducing the rate of expression of the corresponding gene.

**[0232.0.0.9]** to **[0276.0.0.9]: see [0232.0.0.0]** to **[0276.0.0.0]**

**[0277.0.9.9]** The respective fine chemical produced can be isolated from the organism by methods with which the skilled worker is familiar. For example, via extraction, salt precipitation, and/or different chromatography methods. The process according to the invention can be conducted batchwise, semibatchwise or continuously. The respective fine chemical produced by this process can be obtained by harvesting the organisms, either from the crop in which they grow, or from the field. This can be done via pressing or extraction of the plant parts.

**[0278.0.0.9]** to **[0282.0.0.9]: see [0278.0.0.0]** to **[0282.0.0.0]**

**[0283.0.9.9]** Moreover, a native polypeptide conferring the increase of the respective fine chemical in an organism or part thereof can be isolated from cells (e.g., endothelial cells), for example using the antibody of the present invention as described below, e.g. an antibody against a protein as indicated in Table II, column 3, lines 89 to 92 or 482 and/or lines 93 to 95 or 472 to 475 and/or lines 96 to 100 or 476 to 477 and/or line 101 to 102 or 478 to 481, resp., or an antibody against a polypeptide as indicated in Table II, columns 5 or 7, lines 89 to 92 or 482 and/or lines 93 to 95 or 472 to 475 and/or lines 96 to 100 or 476 to 477 and/or line 101 to 102 or 478 to 481, resp. or an antigenic part thereof, which can be produced by standard techniques utilizing the polypeptid of the present invention or fragment thereof, i.e., the polypeptide of this invention. Preferred are monoclonal antibodies specifically binding to polypeptide as indicated in Table II, columns 5 or 7, lines 93 to 95 or 472 to 475 and/or lines 96 to 100 or 476 to 477 and/or line 101 to 102 or 478 to 481, resp.

**[0284.0.0.9]: see [0284.0.0.0]**

**[0285.0.9.9]** In one embodiment, the present invention relates to a polypeptide having a sequence as indicated in Table II, columns 5 or 7, lines 89 to 92 or 482 and/or lines 93 to 95 or 472 to 475 and/or lines 96 to 100 or 476 to 477 and/or line 101 to 102 or 478 to 481, resp., or as encoded by a nucleic acid molecule as indicated in Table I, columns 5 or 7, lines 89 to 92 or 482 and/or lines 93 to 95 or 472 to 475 and/or lines 96 to 100 or 476 to 477 and/or line 101 to 102 or 478 to 481, resp., or functional homologues thereof.

**[0286.0.9.9]** In one advantageous embodiment, in the method of the present invention the activity of a polypeptide is increased which comprises or consists of a consensus sequence as indicated in Table IV, column 7, lines 89 to 92 or 482 and/or lines 93 to 95 or 472 to 475 and/or lines 96 to 100 or 476 to 477 and/or line 101 to 102 or 478 to 481, resp. In another embodiment, the present invention relates to a polypeptide comprising or consisting of a consensus sequence as indicated in Table IV, column 7, lines 89 to 92 or 482 and/or lines 93 to 95 or 472 to 475 and/or lines 96 to 100 or 476 to 477 and/or line 101 to 102 or 478 to 481, resp., whereby 20 or less, preferably 15 or 10, preferably 9, 8, 7, or 6, more preferred 5 or 4, even more preferred 3, even more preferred 2, even more preferred 1, most preferred 0 of the amino acids positions indicated can be replaced by any amino acid or, in an further embodiment, can be replaced and/or absent. In one embodiment, the present invention relates to the method of the present invention comprising a polypeptide or to a polypeptide comprising more than one consensus sequences (of an individual line) as indicated in Table IV, column 7, lines 89 to 92 or 482 and/or lines 93 to 95 or 472 to 475 and/or lines 96 to 100 or 476 to 477 and/or line 101 to 102 or 478 to 481, resp

**[0287.0.0.9]** to **[0290.0.0.9]: see [0287.0.0.0]** to **[0290.0.0.0]**

**[0291.0.9.9]** In one advantageous embodiment, the method of the present invention comprises the increasing of a polypeptide comprising or consisting of plant or microorganism specific consensus sequences.
In one embodiment, said polypeptide of the invention distinguishes over a sequence as indicated in Table II A or II B, columns 5 or 7, lines 89 to 92 or 482 and/or lines 93 to 95 or 472 to 475 and/or lines 96 to 100 or 476 to 477 and/or line 101 to 102 or 478 to 481, resp., by one or more amino acids. In one embodiment, polypeptide distinguishes from a sequence as indicated in Table II A or II B, columns 5 or 7, lines 89 to 92 or 482 and/or lines 93 to 95 or 472 to 475 and/or lines 96 to 100 or 476 to 477 and/or line 101 to 102 or 478 to 481, resp., by more than 5, 6, 7, 8 or 9 amino acids, preferably by more than 10, 15, 20, 25 or 30 amino acids, even more preferred are more than 40, 50, or 60 amino acids and, preferably, the sequence of the polypeptide of the invention distinguishes from a sequence as indicated in Table II A or II B, columns 5 or 7, lines 89 to 92 or 482 and/or lines 93 to 95 or 472 to 475 and/or lines 96 to 100 or 476 to 477 and/or line 101 to 102 or 478 to 481, resp., by not more than 80% or 70% of the amino acids, preferably not more than 60% or 50%, more preferred not more than 40% or 30%, even more preferred not more than 20% or 10%. In an other embodiment, said polypeptide of the invention does not consist of a sequence as indicated in Table II A orll B, columns 5 or 7, lines 89 to 92 or 482 and/or lines 93 to 95 or 472 to 475 and/or lines 96 to 100 or 476 to 477 and/or line 101 to 102 or 478 to 481, resp.

**[0292.0.0.9] see [0292.0.0.0]**

**[0293.0.9.9]** In one embodiment, the invention relates to polypeptide conferring an increase in the respective fine chemical in an organism or part thereof and being encoded by the nucleic acid molecule of the invention or a nucleic acid molecule used in the process of the invention. In one embodiment, said polypeptide is having a sequence which distinguishes from a sequence as indicated in Table II A or II B, columns 5 or 7, lines 89 to 92 or 482 and/or lines 93 to 95 or 472 to 475 and/or lines 96 to 100 or 476 to 477 and/or line 101 to 102 or 478 to 481, resp., by one or more amino acids. In an other embodiment, the polypeptide of the invention does not consist of the sequence as indicated in Table II A or II B , columns 5 or 7, lines 89 to 92 or 482 and/or lines 93 to 95 or 472 to 475 and/or lines 96 to 100 or 476 to 477 and/or line 101 to 102 or 478 to 481, resp. In a further embodiment, the polypeptide of the present invention is less than 100%, 99,999%, 99,99%, 99,9% or 99% identical.

**[0294.0.9.9]** In one embodiment, the present invention relates to a polypeptide having an activity of a protein as indicated in Table II, column 3, lines 89 to 92 or 482 and/or lines 93 to 95 or 472 to 475 and/or lines 96 to 100 or 476 to 477 and/or line 101 to 102 or 478 to 481, resp., which distinguishes over a sequence as indicated in Table II A or II B, columns 5 or 7, lines 89 to 92 or 482 and/or lines 93 to 95 or 472 to 475 and/or lines 96 to 100 or 476 to 477 and/or line 101 to 102 or 478 to 481, resp., by one or more amino acids, preferably by more than 5, 6, 7, 8 or 9 amino acids, preferably by more than 10, 15, 20, 25 or 30 amino acids, even more preferred are more than 40, 50, or 60 amino acids but even more preferred by less than 70% of the amino acids, more preferred by less than 50%, even more preferred my less than 30% or 25%, more preferred are 20% or 15%, even more preferred are less than 10%.

**[0295.0.0.9]** to **[0297.0.0.9]: see [0295.0.0.0]** to **[0297.0.0.0] [0297.1.9.9]** Non polypeptide of the invention-chemicals are e.g. polypeptides having not the activity of a polypeptide indicated in Table II, columns 3, 5 or 7, lines 89 to 92 or 482 and/or lines 93 to 95 or 472 to 475 and/or lines 96 to 100 or 476 to 477 and/or line 101 to 102 or 478 to 481, resp.

**[0298.0.9.9]** A polypeptide of the invention can participate in the process of the present invention. The polypeptide or a portion thereof comprises preferably an amino acid sequence which is sufficiently homologous to an amino acid sequence as indicated in Table II, columns 5 or 7, lines 89 to 92 or 482 and/or lines 93 to 95 or 472 to 475 and/or lines 96 to 100 or 476 to 477 and/or line 101 to 102 or 478 to 481, resp. The portion of the protein is preferably a biologically active portion as described herein. Preferably, the polypeptide used in the process of the invention has an amino acid sequence identical to a sequence as indicated in Table II, columns 5 or 7, lines 89 to 92 or 482 and/or lines 93 to 95 or 472 to 475 and/or lines 96 to 100 or 476 to 477 and/or line 101 to 102 or 478 to 481, resp.

**[0299.0.9.9]** Further, the polypeptide can have an amino acid sequence which is encoded by a nucleotide sequence which hybridizes, preferably hybridizes under stringent conditions as described above, to a nucleotide sequence of the nucleic acid molecule of the present invention. Accordingly, the polypeptide has an amino acid sequence which is encoded by a nucleotide sequence that is at least about 35%, 40%, 45%, 50%, 55%, 60%, 65% or 70%, preferably at least about 75%, 80%, 85% or 90, and more preferably at least about 91%, 92%, 93%, 94% or 95%, and even more preferably at least about 96%, 97%, 98%, 99% or more homologous to one of the nucleotide sequence as indicated in Table I, columns 5 or 7, lines 89 to 92 or 482 and/or lines 93 to 95 or 472 to 475 and/or lines 96 to 100 or 476 to 477 and/or line 101 to 102 or 478 to 481, resp. The preferred polypeptide of the present invention preferably possesses at least one of the activities according to the invention and described herein. A preferred polypeptide of the present invention includes an amino acid sequence encoded by a nucleotide sequence which hybridizes, preferably hybridizes under stringent conditions, to a nucleotide sequence as indicated in Table I, columns 5 or 7, lines 89 to 92 or 482 and/or lines 93 to 95 or 472 to 475 and/or lines 96 to 100 or 476 to 477 and/or line 101 to 102 or 478 to 481, resp., or which is homologous thereto, as defined above.

**[0300.0.9.9]** Accordingly the polypeptide of the present invention can vary from a sequence as indicated in Table II, columns 5 or 7, lines 89 to 92 or 482 and/or lines 93 to 95 or 472 to 475 and/or lines 96 to 100 or 476 to 477 and/or line 101 to 102 or 478 to 481, resp., in amino acid sequence due to natural variation or mutagenesis, as described in detail herein. Accordingly, the polypeptide comprise an amino acid sequence which is at least about 35%, 40%, 45%, 50%, 55%, 60%, 65% or 70%, preferably at least about 75%, 80%, 85% or 90, and more preferably at least about 91%, 92%, 93%, 94% or 95%, and most preferably at least about 96%, 97%, 98%, 99% or more homologous to an entire amino acid sequence of a sequence as indicated in Table II A or II B, columns 5 or 7, lines 89 to 92 or 482 and/or lines 93 to 95 or 472 to 475 and/or lines 96 to 100 or 476 to 477 and/or line 101 to 102 or 478 to 481, resp.

**[0301.0.0.9] see [0301.0.0.0]**

**[0302.0.9.9]** Biologically active portions of an polypeptide of the present invention include peptides comprising amino acid sequences derived from the amino acid sequence of the polypeptide of the present invention or used in the process of the present invention, e.g., an amino acid sequence as indicated in Table II, columns 5 or 7, lines 89 to 92 or 482 and/or lines 93 to 95 or 472 to 475 and/or lines 96 to 100 or 476 to 477 and/or line 101 to 102 or 478 to 481, resp., or the amino acid sequence of a protein homologous thereto, which include fewer amino acids than a full length polypeptide of the present invention or used in the process of the present invention or the full length protein which is homologous to an polypeptide of the present invention or used in the process of the present invention depicted herein, and exhibit at least one activity of polypeptide of the present invention or used in the process of the present invention.

**[0303.0.0.9]: see [0303.0.0.0]**

**[0304.0.9.9]** Manipulation of the nucleic acid molecule of the invention or the nucleic acid molecule used in the method of the invention may result in the production of a protein having essentially the activity of the polypeptides as indicated in Table II, column 3, lines 89 to 92 or 482 and/or lines 93 to 95 or 472 to 475 and/or lines 96 to 100 or 476 to 477 and/or line 101 to 102 or 478 to 481, resp., but having differences in the sequence from said wild-type protein. These proteins may be improved in efficiency or activity, may be present in greater numbers in the cell than is usual, or may be decreased in efficiency or activity in relation to the wild type protein.

**[0305.0.0.9]** to **[0306.0.0.9]: see [0305.0.0.0]** to **[0306.0.0.0]**

**[0306.1.9.9]** Preferably, the compound is a composition comprising the essentially pure fine chemical, i.e. Vitamin E, i.e. alpha-tocopherol, beta-tocopherol, and/or gamma-tocopherol or the vitamin E precursor 2,3-Dimethyl-5-pythylquinol, respectively or a recovered or isolated Vitamin E, i.e. alpha-tocopherol, beta-tocopherol, and/or gamma-tocopherol or the vitamin E precursor 2,3-Dimethyl-5-pythylquinol, respectively, e.g. in free or in protein- or membrane-bound form.

**[0307.0.0.9]** to **[0308.0.0.9]: see [0307.0.0.0]** to **[0308.0.0.0]**

**[0309.0.9.9]** In one embodiment, a reference to a protein (= polypeptide) of the invention or as indicated in Table II, columns 5 or 7, lines 89 to 92 or 482 and/or lines 93 to 95 or 472 to 475 and/or lines 96 to 100 or 476 to 477 and/or line 101 to 102 or 478 to 481, resp., refers to a polypeptide having an amino acid sequence corresponding to the polypeptide of the invention or used in the process of the invention , whereas a " non-polypeptide of the invention" or "other polypeptide" not being indicated in Table II, columns 5 or 7, lines 89 to 92 or 482 and/or lines 93 to 95 or 472 to 475 and/or lines 96 to 100 or 476 to 477 and/or line 101 to 102 or 478 to 481, resp., refers to a polypeptide having an amino acid sequence corresponding to a protein which is not substantially homologous a polypeptide of the invention, preferably which is not substantially homologous to a polypeptide as indicated in Table II, columns 5 or 7, lines 89 to 92 or 482 and/or lines 93 to 95 or 472 to 475 and/or lines 96 to 100 or 476 to 477 and/or line 101 to 102 or 478 to 481, resp., e.g., a protein which does not confer the activity described herein or annotated or known for as indicated in Table II, column 3, lines 89 to 92 or 482 and/or lines 93 to 95 or 472 to 475 and/or lines 96 to 100 or 476 to 477 and/or line 101 to 102 or 478 to 481, resp., and which is derived from the same or a different organism. In one embodiment, a "non-polypeptide of the invention" or "other polypeptide" not being indicated in Table II, columns 5 or 7, lines 89 to 92 or 482 and/or lines 93 to 95 or 472 to 475 and/or lines 96 to 100 or 476 to 477 and/or line 101 to 102 or 478 to 481, resp., does not confer an increase of the respective fine chemical in an organism or part thereof.

**[0310.0.0.9]** to **[0334.0.0.9]: see [0310.0.0.0]** to **[0334.0.0.0]**

**[0335.0.9.9]** The dsRNAi method has proved to be particularly effective and advantageous for reducing the expression of a nucleic acid sequences as indicated in Table I, columns 5 or 7, lines 89 to 92 or 482 and/or lines 93 to 95 or 472 to 475 and/or lines 96 to 100 or 476 to 477 and/or line 101 to 102 or 478 to 481, resp., and/or homologs thereof. As described inter alia in WO 99/32619, dsRNAi approaches are clearly superior to traditional antisense approaches. The invention therefore furthermore relates to double-stranded RNA molecules (dsRNA molecules) which, when introduced into an organism, advantageously into a plant (or a cell, tissue, organ or seed derived therefrom), bring about altered metabolic activity by the reduction in the expression of a nucleic acid sequences as indicated in Table I, columns 5 or 7, lines 89 to 92 or 482 and/or lines 93 to 95 or 472 to 475 and/or lines 96 to 100 or 476 to 477 and/or line 101 to 102 or 478 to 481, resp., and/or homologs thereof. In a double-stranded RNA molecule for reducing the expression of an protein encoded by a nucleic acid sequence sequences as indicated in Table I, columns 5 or 7, lines 89 to 92 or 482 and/or lines 93 to 95 or 472 to 475 and/or lines 96 to 100 or 476 to 477 and/or line 101 to 102 or 478 to 481, resp., and/or homologs thereof, one of the two RNA strands is essentially identical to at least part of a nucleic acid sequence, and the respective other RNA strand is essentially identical to at least part of the complementary strand of a nucleic acid sequence.

**[0336.0.0.9] to [0342.0.0.9]: see [0336.0.0.0] to [0342.0.0.0]**

**[0343.0.9.9]** As has already been described, 100 % sequence identity between the dsRNA and a gene transcript of a nucleic acid sequence as indicated in Table I, columns 5 or 7, lines 89 to 92 or 482 and/or lines 93 to 95 or 472 to 475 and/or lines 96 to 100 or 476 to 477 and/or line 101 to 102 or 478 to 481, resp., or it's homolog is not necessarily required in order to bring about effective reduction in the expression. The advantage is, accordingly, that the method is tolerant with regard to sequence deviations as may be present as a consequence of genetic mutations, polymorphisms or evolutionary divergences. Thus, for example, using the dsRNA, which has been generated starting from a sequence as indicated in Table I, columns 5 or 7, lines 89 to 92 or 482 and/or lines 93 to 95 or 472 to 475 and/or lines 96 to 100 or 476 to 477 and/or line 101 to 102 or 478 to 481, resp., or homologs thereof of the one organism, may be used to suppress the corresponding expression in another organism.

**[0344.0.0.9] to [0361.0.0.9]: see [0344.0.0.0] to [0361.0.0.0]**

**[0362.0.9.9]** Accordingly the present invention relates to any cell transgenic for any nucleic acid characterized as part of the invention, e.g. conferring the increase of the respective fine chemical in a cell or an organism or a part thereof, e.g. the nucleic acid molecule of the invention or the nucleic acid molecule used in the method of the invention, the nucleic acid construct of the invention, the antisense molecule of the invention, the vector of the invention or a nucleic acid molecule encoding the polypeptide of the invention, e.g. the polypeptide as indicated in Table II, columns 5 or 7, lines 89 to 92 or 482 and/or lines 93 to 95 or 472 to 475 and/or lines 96 to 100 or 476 to 477 and/or line 101 to 102 or 478 to 481, resp. e.g. encoding a polypeptide having protein activity, as indicated in Table II, column 3, lines 89 to 92 or 482 and/or lines 93 to 95 or 472 to 475 and/or lines 96 to 100 or 476 to 477 and/or line 101 to 102 or 478 to 481, resp. Due to the above mentioned activity the respective fine chemical content in a cell or an organism is increased. For example, due to modulation or manipulation, the cellular activity of the polypeptide of the invention or nucleic acid molecule of the invention is increased, e.g. due to an increased expression or specific activity of the subject matters of the invention in a cell or an organism or a part thereof. In one embodiment, transgenic for a polypeptide having an activity of a polypeptide as indicated in Table II, columns 5 or 7, lines 89 to 92 or 482 and/or lines 93 to 95 or 472 to 475 and/or lines 96 to 100 or 476 to 477 and/or line 101 to 102 or 478 to 481, resp., means herein that due to modulation or manipulation of the genome, an activity as annotated for a polypeptide as indicated in Table II, column 3, lines 89 to 92 or 482 and/or lines 93 to 95 or 472 to 475 and/or lines 96 to 100 or 476 to 477 and/or line 101 to 102 or 478 to 481, resp. e.g. having a sequence as indicated in Table II, columns 5 or 7, lines 89 to 92 or 482 and/or lines 93 to 95 or 472 to 475 and/or lines 96 to 100 or 476 to 477 and/or line 101 to 102 or 478 to 481, resp., is increased in a cell or an organism or a part thereof. Examples are described above in context with the process of the invention

**[0363.0.0.9]: see [0363.0.0.0]**

**[0364.0.9.9]** A naturally occurring expression cassette - for example the naturally occurring combination of a promoter of a polypeptide of the invention as indicated in Table II, column 3, lines 89 to 92 or 482 and/or lines 93 to 95 or 472 to 475 and/or lines 96 to 100 or 476 to 477 and/or line 101 to 102 or 478 to 481, resp., with the corresponding protein-encoding sequence as indicated in Table I, column 3, lines 89 to 92 or 482 and/or lines 93 to 95 or 472 to 475 and/or lines 96 to 100 or 476 to 477 and/or line 101 to 102 or 478 to 481, resp., - becomes a transgenic expression cassette when it is modified by non-natural, synthetic "artificial" methods such as, for example, mutagenization. Such methods have been described (US 5,565,350; WO 00/15815; also see above).

**[0365.0.0.9] to [0373.0.0.9]: see [0365.0.0.0] to [0373.0.0.0]**

**[0374.0.9.9]** Transgenic plants comprising the respective fine chemical synthesized in the process according to the invention can be marketed directly without isolation of the compounds synthesized. In the process according to the invention, plants are understood as meaning all plant parts, plant organs such as leaf, stalk, root, tubers or seeds or propagation material or harvested material or the intact plant. In this context, the seed encompasses all parts of the seed such as the seed coats, epidermal cells, seed cells, endosperm or embryonic tissue. Vitamin E or its precursor 2,3-dimethyl-5-phytylquinol resp., in particular, alpha-, beta-, and/or gamma-tocopherol resp., produced in the process according to the invention may, however, also be isolated from the plant in the form of their free vitamin E or bound in or to compounds or moieties. Vitamin E or its precursor 2,3-dimethyl-5-phytylquinol resp., in particular, alpha-, beta-, and/or gamma-tocopherol resp., produced by this process can be harvested by harvesting the organisms either from the culture in which they grow or from the field. This can be done via expressing, grinding and/or extraction, salt precipitation and/or ion-exchange chromatography or other chromatographic methods of the plant parts, preferably the plant seeds, plant fruits, plant tubers and the like.

**[0375.0.0.9] to [0376.0.0.9]: see [0375.0.0.0] to [0376.0.0.0]**

**[0377.0.9.9]** Accordingly, the present invention relates also to a process according to the present invention whereby the produced vitamin E comprising composition or the produced the respective fine chemical is isolated.

**[0378.0.9.9]:** In this manner, more than 50% by weight, advantageously more than 60% by weight, preferably more than 70% by weight, especially preferably more than 80% by weight, very especially preferably more than 90% by weight, of the vitamin E or its precursor 2,3-dimethyl-5-phytylquinol resp., in particular, alpha-, beta-, and/or gamma-tocopherol, resp., produced in the process can be isolated. The resulting recovered, isolated or purified vitamin E or its precursor 2,3-dimethyl-5-phytylquinol resp., in particular, alpha-, beta-, and/or gamma-tocopherol resp.,, e.g. compositions comprising the former, can, if appropriate, subsequently be further purified, if desired mixed with other active ingredients such as vitamins, amino acids, carbohydrates, antibiotics and the like, and, if appropriate, formulated.

**[0379.0.9.9]** In one embodiment, the vitamin E or its precursor 2,3-dimethyl-5-phytylquinol resp., in particular, alpha-, beta-, and/or gamma-tocopherol resp., is a mixture comprising of one or more the respective fine chemicals. In one embodiment, the respective fine chemical means here vitamin E, in particular alpha-, beta-, or gamma-tocopherol. In one embodiment, vitamin E means here a mixture of the respective fine chemicals.

**[0380.0.9.9]** The vitamin E or its precursor 2,3-dimethyl-5-phytylquino,I resp., in particular, alpha-, beta-, and/or gamma-tocopherol resp., obtained in the process are suitable as starting material for the synthesis of further products of value. For example, they can be used in combination with each other or alone for the production of pharmaceuticals, foodstuffs, animal feeds or cosmetics. Accordingly, the present invention relates a method for the production of pharmaceuticals, food stuff, animal feeds, nutrients or cosmetics comprising the steps of the process according to the invention, including the isolation of the Vitamin E- or its precursor 2,3-dimethyl-5-phytylquinol- comprising composition produced or the respective fine chemical produced if desired and formulating the product with a pharmaceutical acceptable carrier or formulating the product in a form acceptable for an application in agriculture. A further embodiment according to the invention is the use of the Vitamin E or its precursor 2,3-dimethyl-5-phytylquinol resp., produced in the process or of the transgenic organisms in animal feeds, foodstuffs, medicines, food supplements, cosmetics or pharmaceuticals.

**[0381.0.0.9]** to **[0382.0.0.9]: see [0381.0.0.0]** to **[0382.0.0.0]**

**[0383.0.9.9]:** ./.

**[0384.0.0.9]: see [0384.0.0.0]**

**[0385.0.9.9]** The fermentation broths obtained in this way, containing in particular vitamin E or its precursor 2,3-dimethyl-5-phytylquinol, resp., in particular, alpha-, beta-, and/or gamma-tocopherol, resp., in mixtures with other compounds, in particular with other vitamins or e.g. with carotenoids, e.g. with astaxanthin, or fatty acids or containing microorganisms or parts of microorganisms, like plastids, containing vitamin E or its precursor 2,3-dimethyl-5-phytylquinol resp., in particular, alpha-, beta-, and/or gamma-tocopherol resp., in mixtures with other compounds, e.g. with carotenoids, normally have a dry matter content of from 7.5 to 25% by weight. Sugar-limited fermentation is additionally advantageous, e.g. at the end, for example over at least 30% of the fermentation time. This means that the concentration of utilizable sugar in the fermentation medium is kept at, or reduced to, 0 to 3 g/I during this time. The fermentation broth is then processed further. Depending on requirements, the biomass can be removed or isolated entirely or partly by separation methods, such as, for example, centrifugation, filtration, decantation, coagulation/flocculation or a combination of these methods, from the fermentation broth or left completely in it.
The fermentation broth can then be thickened or concentrated by known methods, such as, for example, with the aid of a rotary evaporator, thin-film evaporator, falling film evaporator, by reverse osmosis or by nanofiltration. This concentrated fermentation broth can then be worked up by freeze-drying, spray drying, spray granulation or by other processes.
As vitamin E is often localized in membranes or plastids, in one embodiment it is advantageous to avoid a leaching of the cells when the biomass is isolated entirely or partly by separation methods, such as, for example, centrifugation, filtration, decantation, coagulation/flocculation or a combination of these methods, from the fermentation broth. The dry biomass can directly be added to animal feed, provided the vitamin E concentration is sufficiently high and no toxic compounds are present. In view of the instability of vitamin E, conditions for drying, e.g. spray or flash-drying, can be mild and can be avoiding oxidation and *cis*/*trans* isomerization. For example antioxidants, e.g. BHT, ethoxyquin or other, can be added. In case the vitamin E concentration in the biomass is to dilute, solvent extraction can be used for their isolation, e.g. with alcohols, ether or other organic solvents, e.g. with methanol, ethanol, acetone, alcoholic potassium hydroxide, glycerol-fenol, liquefied fenol or for example with acids or bases, like trichloroacetatic acid or potassium hydroxide. A wide range of advantageous methods and techniques for the isolation of vitamin E can be found in the state of the art.

**[0386.0.9.9]** Accordingly, it is possible to further purify the produced vitamin E or its precursor 2,3-dimethyl-5-phytylquinol resp., in particular, alpha-, beta-, and/or gamma-tocopherol resp., . For this purpose, the product-containing composition, e.g. a total or partial lipid extraction fraction using organic solvents, e.g. as described above, is subjected for example to a saponification to remove triglycerides, partition between e.g. hexane/methanol (separation of non-polar epiphase from more polar hypophasic derivates) and separation via e.g. an open column chromatography or HPLC in which case the desired product or the impurities are retained wholly or partly on the chromatography resin. These chromatography steps can be repeated if necessary, using the same or different chromatography resins. The skilled worker is familiar with the choice of suitable chromatography resins and their most effective use.

**[0387.0.0.9]** to **[0392.0.0.9]: see [0387.0.0.0]** to **[0392.0.0.0]**

**[0393.0.9.9]** In one embodiment, the present invention relates to a method for the identification of a gene product conferring an increase in the respective fine chemical production in a cell, comprising the following steps:
(b) contacting, e.g. hybridising, the nucleic acid molecules of a sample, e.g. cells, tissues, plants or microorganisms or a nucleic acid library , which can contain a candidate gene encoding a gene product conferring an increase in the respective fine chemical after expression, with the nucleic acid molecule of the present invention;
(c) identifying the nucleic acid molecules, which hybridize under relaxed stringent conditions with the nucleic acid molecule of the present invention in particular to the nucleic acid molecule sequence as indicated in Table I, preferably I B, columns 5 or 7, lines 89 to 92 or 482 and/or lines 93 to 95 or 472 to 475 and/or lines 96 to 100 or 476 to 477 and/or line 101 to 102 or 478 to 481, resp., and, optionally, isolating the full length cDNA clone or complete genomic clone;
(d) introducing the candidate nucleic acid molecules in host cells, preferably in a plant cell or a microorganism, appropriate for producing the respective fine chemical;
(e) expressing the identified nucleic acid molecules in the host cells;
(f) assaying the respective fine chemical level in the host cells; and
(g) identifying the nucleic acid molecule and its gene product which expression confers an increase in the respective the respective fine chemical level in the host cell after expression compared to the wild type.

**[0394.0.0.9]** to **[0398.0.0.9]: see [0394.0.0.0]** to **[0398.0.0.0]**

**[0399.0.9.9]** Furthermore, in one embodiment, the present invention relates to process for the identification of a compound conferring increased respective fine chemical production in a plant or microorganism, comprising the steps:
(g) culturing a cell or tissue or microorganism or maintaining a plant expressing the polypeptide according to the invention or a nucleic acid molecule encoding said polypeptide and a readout system capable of interacting with the polypeptide under suitable conditions which permit the interaction of the polypeptide with said readout system in the presence of a compound or a sample comprising a plurality of compounds and capable of providing a detectable signal in response to the binding of a compound to said polypeptide under conditions which permit the expression of said readout system and the polypeptide of the present invention or used in the process of the invention; and
(h) identifying if the compound is an effective agonist by detecting the presence or absence or increase of a signal produced by said readout system.
The screen for a gene product or an agonist conferring an increase in the respective fine chemical production can be performed by growth of an organism for example a microorganism in the presence of growth reducing amounts of an inhibitor of the synthesis of the respective fine chemical. Better growth, e.g. higher dividing rate or high dry mass in comparison to the control under such conditions would identify a gene or gene product or an agonist conferring an increase in fine chemical production.

**[00399.1.9.9]** One can think to screen for increased production of the respective fine chemical by for example searching for a resistance to a drug blocking the synthesis of the respective fine chemical and looking whether this effect is dependent on the activity or expression of a polypeptide as indicated in Table II, columns 5 or 7, lines 89 to 92 or 482 and/or lines 93 to 95 or 472 to 475 and/or lines 96 to 100 or 476 to 477 and/or line 101 to 102 or 478 to 481, resp., or a homolog thereof, e.g. comparing the phenotype of nearly identical organisms with low and high activity of a protein as indicated in Table II, columns 5 or 7, lines 89 to 92 or 482 and/or lines 93 to 95 or 472 to 475 and/or lines 96 to 100 or 476 to 477 and/or line 101 to 102 or 478 to 481, resp., after incubation with the drug.

**[0400.0.0.9]** to **[0416.0.0.9]: see [0400.0.0.0]** to **[0416.0.0.0]**

**[0417.0.9.9]** The nucleic acid molecule of the invention or used in the method of the invention, the vector of the invention or the nucleic acid construct of the invention may also be useful for the production of organisms resistant to inhibitors of the vitamin E production biosynthesis pathways. In particular, the overexpression of the polypeptide of the present invention may protect an organism such as a microorganism or a plant against inhibitors, which block the vitamin E, in particular the respective fine chemical synthesis in said organism.
As vitamin E can protect organisms against damages of oxidative stress, especially singlet oxygen, a increased level of the respective fine chemical can protect plants against herbicides which cause the toxic build-up of oxidative compounds, e.g. singlet oxygen. For example, inhibition of the protoporphorineogen oxidase (Protox), an enzyme important in the synthesis of chlorophyll and heme biosynthesis results in the loss of chlorophyll and carotenoids and in leaky membranes; the membrane destruction is due to creation of free oxygen radicals (which is also reported for other classic photosynthetic inhibitor herbicides).
Accordingly, in one embodiment, the increase of the level of the respective fine chemical is used to protect plants against herbicides destroying membranes due to the creation of free oxygen radicals.
Examples of inhibitors or herbicides building up oxidative stress are aryl triazion, e.g. sulfentrazone, carfentrazone; or diphenylethers, e.g. acifluorfen, lactofen, or oxyfluorfen; or N-Phenylphthalimide, e.g. flumiclorac or flumioxazin; substituted ureas, e.g. fluometuron, tebuthiuron, diuron, or linuron; triazines, e.g. atrazine, prometryn, ametryn, metributzin, prometon, simazine, or hexazinone: or uracils, e.g. bromacil or terbacil.

**[0418.0.0.9]** to **[0423.0.0.9]: see [0418.0.0.0]** to **[0423.0.0.0]**

**[0424.0.9.9]** Accordingly, the nucleic acid of the invention or the nucleic acid molecule used in the method of the invention, the polypeptide of the invention or the polypeptide used in the method of the invention, the nucleic acid construct of the invention, the organisms, the host cell, the microorganisms, the plant, plant tissue, plant cell, or the part thereof of the invention, the vector of the invention, the agonist identified with the method of the invention, the nucleic acid molecule identified with the method of the present invention, can be used for the production of the respective fine chemical or of the respective fine chemical and one or more other carotenoids, vitamins or fatty acids. In one embodiment, in the process of the present invention, the produced vitamin E is used to protect fatty acids against oxidization, e.g. it is in a further step added in a pure form or only partly isolated to a composition comprising fatty acids.
Accordingly, the nucleic acid of the invention or the nucleic acid molecule used in the method of the invention, or the nucleic acid molecule identified with the method of the present invention or the complement sequences thereof, the polypeptide of the invention or the polypeptide used in the method of the invention, the nucleic acid construct of the invention, the organisms, the host cell, the microorganisms, the plant, plant tissue, plant cell, or the part thereof of the invention, the vector of the invention, the antagonist identified with the method of the invention, the antibody of the present invention, the antisense molecule of the present invention, can be used for the reduction of the respective fine chemical in a organism or part thereof, e.g. in a cell.

**[0424.1.9.9]** In a further embodiment the present invention relates to the use of the antagonist of the present invention, the plant of the present invention or a part thereof, the microorganism or the host cell of the present invention or a part thereof for the production a cosmetic composition or a pharmaceutical composition. Such a composition has an antioxidative activity, photoprotective activity, can be used to protect, treat or heal the above mentioned diseases, e.g. rhypercholesterolemic or cardiovascular diseases, certain cancers, and cataract formation or as immunostimulatory agent.
The vitamin E can be also used as stabilizer of other colours or oxygen sensitive compounds, like fatty acids, in particular unsaturated fatty acids.

**[0425.0.0.9]** to **[0434.0.0.9]: see [0425.0.0.0]** to **[0434.0.0.0]**

**[0435.0.9.9]** Example 3: In-vivo and in-vitro mutagenesis

**[0436.0.9.9]** An *in vivo* mutagenesis of organisms such as algae (e.g. Spongiococcum sp, e.g. Spongiococcum exentricum, Chlorella sp., Haematococcus, Phaedactylum tricornatum, Volvox or Dunaliella), *Synechocystis* sp. PCC 6803, Physcometrella patens, Saccharomyces, Mortierella, Escherichia and others mentioned above, which are beneficial for the production of vitamin E can be carried out by passing a plasmid DNA (or another vector DNA) containing the desired nucleic acid sequence or nucleic acid sequences, e.g. the nucleic acid molecule of the invention or the vector of the invention, through E. coli and other microorganisms (for example Bacillus spp. or yeasts such as Saccharomyces cerevisiae) which are not capable of maintaining the integrity of its genetic information. Usual mutator strains have mutations in the genes for the DNA repair system [for example mutHLS, mutD, mutT and the like; for comparison, see Rupp, W.D. (1996) DNA repair mechanisms in Escherichia coli and Salmonella, pp. 2277-2294, ASM: Washington]. The skilled worker knows these strains. The use of these strains is illustrated for example in Greener, A. and Callahan, M. (1994) Strategies 7; 32-34.
In-vitro mutation methods such as increasing the spontaneous mutation rates by chemical or physical treatment are well known to the skilled person. Mutagens like 5-bromo-uracil, N-methyl-N-nitro-N-nitrosoguanidine (= NTG), ethyl methanesulfonate (= EMS), hydroxylamine and/or nitrous acid are widely used as chemical agents for random in-vitro mutagenesis. The most common physical method for mutagenesis is the treatment with UV irradiation. Another random mutagenesis technique is the error-prone PCR for introducing amino acid changes into proteins. Mutations are deliberately introduced during PCR through the use of error-prone DNA polymerases and special reaction conditions known to a person skilled in the art. For this method randomized DNA sequences are cloned into expression vectors and the resulting mutant libraries screened for altered or improved protein activity as described below.
Site-directed mutagenesis method such as the introduction of desired mutations with an M13 or phagemid vector and short oligonucleotides primers is a well-known approach for site-directed mutagenesis. The clou of this method involves cloning of the nucleic acid sequence of the invention into an M13 or phagemid vector, which permits recovery of single-stranded recombinant nucleic acid sequence. A mutagenic oligonucleotide primer is then designed whose sequence is perfectly complementary to nucleic acid sequence in the region to be mutated, but with a single difference: at the intended mutation site it bears a base that is complementary to the desired mutant nucleotide rather than the original. The mutagenic oligonucleotide is then allowed to prime new DNA synthesis to create a complementary full-length sequence containing the desired mutation. Another site-directed mutagenesis method is the PCR mismatch primer mutagenesis method also known to the skilled person. Dpnl site-directed mutagenesis is a further known method as described for example in the Stratagene QuickchangeTM site-directed mutagenesis kit protocol. A huge number of other methods are also known and used in common practice.
Positive mutation events can be selected by screening the organisms for the production of the desired fine chemical.

**[0436.1.0.9] see [0436.1.0.0]**

**[0437.0.9.9] to [0445.0.9.9]: see [0437.0.5.5] to [0445.0.5.5]**

**[0445.1.9.9]** *Synechocystis* sp. PCC 6803 is a unicellular, non-nitrogen-fixing cyanobacterium which has undergone thorough genetic investigation (Churin et al. (1995) J Bacteriol 177: 3337-3343), can easily be transformed (Williams (1988) Methods Enzymol 167:766-778) and has a very active homologous recombination potential. The strain PCC 6803 was isolated as *Aphanocapsa* N-1 from fresh water in California, USA, by R. Kunisawa in 1968 and is now obtainable through the "Pasteur Culture Collection of Axenic Cyanobacterial Strains" (PCC), Unité de Physiologie Microbienne, Paris, France. The complete genomic sequence of *Synechocystis* sp. PCC 6803 has been published from 1995 (Kaneko et al. (1995) DNA Research 2:153-166; Kaneko et al. (1995)DNA Research 2:191-198; Kaneko et al. (1996) DNA Research 3:109-136; Kaneko et al. (1996) DNA Research 3:185-209; Kaneko and Tabata (1997) Plant Cell Physiol 38:1171-1176; Kotani and Tabata (1998) Annu Rev Plant Physiol 49:151-171) and is published on the Internet (http://www.kazusa.or.jp/cyano/cyano.html) under the name "CyanoBase". Efficient expression systems for *Synechocystis* 6803 are described in the literature (Mermet-Bouvier et al. (1993) Curr Microbiol 27:323-327; Mermet-Bouvier and Chauvat (1993) Curr Microbiol 28:145-148; Murphy and Stevens (1992) Appl Environ Microbiol 58:1650-1655; Takeshima et al. (1994) Proc Natl Acad Sci USA 91:9685-9689; Xiaoqiang et al. (1997) Appl Environ Microbiol 63:4971-4975; Ren et al. (1998) FEMS Microbiol Lett 158:127-132).

**[0445.2.9.9]** Growing synechocystis
The cells of *Synechocystis* sp. PCC 6803 can be normally cultured autotrophically in BG11 medium. They have a diameter of 2.3 to 2.5 µm. For example, a cyanobacterium *Synechocystis* sp. PCC 6803 strain which is glucose-tolerant can be used, i.e. it is also able to grow heterotrophically in the dark with only a few minutes of weak blue light illumination per day. The culture conditions were developed by Anderson and McIntosh (Anderson und McIntosh (1991) J Bacteriol 173:2761-2767) and called light-activated heterotrophic growth (LAHG). This makes it possible to cultivate these cyanobacteria without continuous photosynthesis and thus without production of oxygen.

### BG 11 culture medium for Synechocystis

Stock solution 100 x BG11:
- NaNO₃: 1.76 M = 149.58 g
- MgSO₄ x 7 H₂O: 30.4 mM = 7.49 g
- CaCl₂ x 2 H₂O: 24.5 mM = 3.6 g
- Citric acid: 3.12 mM = 0.6 g
- Na EDTA pH 8: 0.279 mM = 0.104 g
The weighed substances can be dissolved in 900 ml of H2O and made up to 1000 ml with 100 ml of the trace metal mix stock 1000x. The solution thus obtained is used as stock solution.
Trace metal mix stock 1000x:
- H₃BO₃: 46.3 mM = 2.86 g/l
- MnCl₂ x 4 H₂O: 4.15 mM = 1.81 g/l
- ZnSO₄ x 7 H₂O: 0.77 mM = 0.222 g/l
- Na₂MOO₄ x 2 H₂O: 1.61 mM = 0.39 g/l
- CuSO₄ x 5 H₂O: 0.32 mM = 0.079 g/I
- Co(NO₃)₂ x 6 H₂O: 0.17 mM = 0.0494 g/l
The following solutions are required for 1 liter of BG11 culture solution:
- 1.: 10 ml of stock solution 100 x BG 11
- 2.: 1 ml Na₂CO₃ (189 mM)
- 3.: 5 ml TES (1 M, pH 8)
- 4.: 1 ml K₂PO₄ (175 mM)
Whereas solution 2. and 3. ought to be sterilized by filtration, solution 4 must be autoclaved. The complete BG11 culture solution must be autoclaved before use and then be mixed with 1 ml of iron ammonium citrate (6 mg/ml) which has previously been sterilized by filtration. The iron ammonium citrate should never be autoclaved. For agar plates, 1.5% (w/v) bacto agar are added per liter of BG11 medium.

**[0445.3.9.9]** Amplification and cloning of DNA from Synechocystis spec. PCC 6803
The DNA can be amplified by the polymerase chain reaction (PCR) from Synechocystis spec. PCC 6803 by the method of Crispin A. Howitt (Howitt CA (1996) BioTechniques 21:32-34).

**[0445.4.9.9]** Tocopherol production in Synechocystis spec. PCC 6803
The cells of each of independent Synechocystis spec. PCC 6803 strains cultured on the BG-11km agar medium, and untransformed wild-type cells (on BG11 agar medium without kanamycin) can be used to inoculate liquid cultures. For this, cells of a mutant or of the wild-type Synechocystis spec. PCC 6803 are transferred from plate into 10 ml of liquid culture in each case. These cultures are cultivated at 28°C and 30 µmol photons*(m²*s)⁻¹ (30 µE) for about 3 days. After determination of the OD₇₃₀ of the individual cultures, the OD₇₃₀ of all cultures is synchronized by appropriate dilutions with BG-11 (wild types) or e.g. BG-11km (mutants). These cell density-synchronized cultures are used to inoculate three cultures of the mutant and of the wild-type control. It is thus possible to carry out biochemical analyses using in each case three independently grown cultures of a mutant and of the corresponding wild types. The cultures are grown until the optical density was OD₇₃₀=0.3.
The cell culture medium is removed by centrifugation in an Eppendorf bench centrifuge at 14000 rpm twice. The subsequent disruption of the cells and extraction of the tocopherols or vitamin E take place by incubation in an Eppendorf shaker at 30°C, 1000 rpm in 100% methanol for 15 minutes twice, combining the supernatants obtained in each case.
In order to avoid oxidation, the resulting extracts can be analyzed immediate after the extraction with the aid of a Waters Allience 2690 HPLC system. Tocopherols and vitamin E is separated on a reverse phase column (ProntoSil 200-3-C30, Bischoff) with a mobile phase of 100% methanol, and identified by means of a standard (Merck). The fluorescence of the substances (excitation 295 nm, emission 320 nm), which is detected with the aid of a Jasco FP 920 fluorescence detector, can serve as detection system.

**[0446.0.0.9]** to **[0450.0.0.9]: see [0446.0.0.0]** to **[0450.0.0.0]**

**[0451.0.0.9]: see [0451.0.5.5]**

**[0452.0.0.9]** to **[0454.0.0.9]: see [0452.0.0.0]** to **[0454.0.0.0]**

**[0455.0.9.9]** Characterization of the transgenic plants.
In order to confirm that vitamin E biosynthesis in the transgenic plants is influenced by the expression of the polypeptides described herein, the tocopherol and vitamin E contents in leaves and seeds of the plants transformed with the described constructs (*Arabidopsis thaliana, Brassica napus* and *Nicotiana tabacum*) are analyzed. For this purpose, the transgenic plants are grown in a greenhouse, and plants which express the gene coding for polypeptide of the invention or used in the method of the invention are identified at the Northern level. The tocopherol content or the vitamin E content in leaves and seeds of these plants is measured. In all, the tocopherol concentration is raised by comparison with untransformed plants.

**[0456.0.0.9]: see [0456.0.0.0]**

**[0457.0.9.9]** Example 9: Purification of the vitamin E or its precursor 2,3-dimethyl-5-phytylquinol.

**[0458.0.9.9]** Abbreviations:; GC-MS, gas liquid chromatography/mass spectrometry; TLC, thin-layer chromatography.
The unambiguous detection for the presence of vitamin E, like alpha-, beta-, or gamma-tocopherol, or its precursor 2,3-dimethyl-5-phytylquinol can be obtained by analyzing recombinant organisms using analytical standard methods: GC, GC-MS or TLC, as described (1997, in: Advances on Lipid Methodology, Fourth Edition: Christie, Oily Press, Dundee, 119-169; 1998, Gaschromatographie-Massenspektrometrie-Verfahren [Gas chromatography/mass spectrometric methods], Lipide 33:343-353).
The total vitamin E produced in the organism for example in yeasts used in the inventive process can be analysed for example according to the following procedure: The material such as yeasts, E. coli or plants to be analyzed can be disrupted by sonication, grinding in a glass mill, liquid nitrogen and grinding or via other applicable methods.
Plant material is initially homogenized mechanically by comminuting in a pestle and mortar to make it more amenable to extraction.
A typical sample pretreatment consists of a total lipid extraction using such polar organic solvents as acetone or alcohols as methanol, or ethers, saponification , partition between phases, seperation of non-polar epiphase from more polar hypophasic derivatives and chromatography. E.g.:

**[0459.0.9.9]:** If required and desired, further chromatography steps with a suitable resin may follow. Advantageously, the vitamin E, like alpha-, beta-, or gamma-tocopherol, or its precursor 2,3-dimethyl-5-phytylquinol, can be further purified with a so-called RTHPLC. As eluent acetonitrile/water or chloroform/acetonitrile mixtures can be used. If necessary, these chromatography steps may be repeated, using identical or other chromatography resins. The skilled worker is familiar with the selection of suitable chromatography resin and the most effective use for a particular molecule to be purified.

**[0460.0.9.9] see [0460.0.0.0]**

**[0461.0.9.9]** Example 10: Cloning SEQ ID NO: 8673 for the expression in plants.

**[0462.0.0.9]: see [0462.0.0.0]**

**[0463.0.9.9]** SEQ ID NO: 8673 is amplified by PCR as described in the protocol of the Pfu Turbo or DNA Herculase polymerase (Stratagene).

**[0464.0.0.9]** to **[0466.0.0.9]: see [0464.0.0.0]** to **[0466.0.0.0]**

**[0467.0.9.9]** The following primer sequences were selected for the gene SEQ ID NO: 8673:
i) forward primer (SEQ ID NO: 8677)
   atggggaaga gagtatacga tcca
ii) reverse primer (SEQ ID NO: 8678)
   tcactccagc ttaaacatgg cgg

**[0468.0.0.9] to [0479.0.0.9]: see [0468.0.0.0] to [0479.0.0.0]**

**[0480.0.9.9]**: Example 11: Generation of transgenic plants which express SEQ ID NO: 8673

**[0481.0.0.9] to [0513.0.0.9]: see [0481.0.0.0] to [0513.0.0.0]**

**[0514.0.9.9]:** As an alternative, the vitamin E, like alpha-, beta-, or gamma-tocopherol, or its precursor 2,3-dimethyl-5-phytylquinol, can be detected advantageously as described in Deli,J. & Molnar,P., Paprika carotenoids: Analysis, isolation, structure elucidation. Curr. Org. Chem. 6, 1197-1219 (2004) or Fraser,P.D., Pinto,M.E., Holloway,D.E. & Bramley,P.M. Technical advance: application of high-performance liquid chromatography with photodiode array detection to the metabolic profiling of plant isoprenoids. Plant J. 24, 551-558 (2000).
The results of the different plant analyses can be seen from the table 1, which follows:

**Table 1**

| **ORF** | **Metabolite** | **Method** | **Min** | **Max** |
|---|---|---|---|---|
| YPL268W | 2,3-Dimethyl-5-phytylquinol | LC | 1,44 | 1,51 |
| YFL013C | 2,3-Dimethyl-5-phytylquinol | LC | 1,97 | 5,40 |
| b1829 | 2,3-Dimethyl-5-phytylquinol | LC | 1,48 | 3,83 |
| b1827 | 2,3-Dimethyl-5-phytylquinol | LC | 1,76 | 1,91 |
| YFL019C | 2,3-Dimethyl-5-phytylquinol | LC | 1,97 | 5,40 |
| b2699 | alpha-Tocopherol | GC | 1,51 | 2,96 |
| b1829 | alpha-Tocopherol | LC | 1,39 | 1,90 |
| b0112 | alpha-Tocopherol | LC | 1,43 | 1,63 |
| b0161 | alpha-Tocopherol | GC | 1,50 | 2,38 |
| b0970 | alpha-Tocopherol | LC | 1,47 | 1,54 |
| b3160 | alpha-Tocopherol | GC | 1,47 | 1,91 |
| b4063 | alpha-Tocopherol | LC | 1,16 | 1,47 |
| b1827 | beta-Tocopherol | LC | 1,34 | 2,16 |
| b0986 | gamma+beta- Tocopherol | LC | 1,35 | 1,61 |
| b0175 | gamma+beta- Tocopherol | LC | 1,34 | 1,40 |
| b0785 | gamma+beta- Tocopherol | LC | 1,34 | 1,85 |
| b3938 | gamma+beta- Tocopherol | LC | 1,50 | 2,20 |
| YFL053W | gamma+beta- Tocopherol | LC | 1,45 | 2,23 |
| b1829 | gamma+beta- Tocopherol | LC | 1,40 | 4,25 |

**[0515.0.9.9]** to **[0552.0.0.9]: see [0515.0.0.0]** to **[0552.0.0.0]** including [0530.1.0.0] to [0530.6. 0.0] as well as [0552.1.0.0] and [0552.2.0.0]

**[0553.0.9.9]**
1. A process for the production of vitamin E or its precursor 2,3-dimethyl-5-phytylquinol, which comprises
   (a) increasing or generating the activity of one or more proteins as indicated in Table II, columns 5 or 7, lines 89 to 92 or 482 and/or lines 93 to 95 or 472 to 475 and/or lines 96 to 100 or 476 to 477 and/or lines 101 to 102 or 478 to 481 resp., preferably Table II B, column 7, lines 89 to 92 or 482 and/or lines 93 to 95 or 472 to 475 and/or lines 96 to 100 or 476 to 477 and/or line 101 to 102 or 478 to 481, resp., or a functional equivalent thereof in a non-human organism, or in one or more parts thereof; and
   (b) growing the organism under conditions which permit the production of vitamin E or its precursor 2,3-dimethyl-5-phytylquinol in said organism.
2. A process for the production of vitamin E or its precursor 2,3-dimethyl-5-phytylquinol , comprising the increasing or generating in an organism or a part thereof the expression of at least one nucleic acid molecule comprising a nucleic acid molecule selected from the group consisting of:
   a) nucleic acid molecule encoding of a polypeptide as indicated in Table II, columns 5 or 7, lines 89 to 92 or 482 and/or lines 93 to 95 or 472 to 475 and/or lines 96 to 100 or 476 to 477 and/or lines 101 to 102 or 478 to 481, resp., preferably Table II B, column 7, lines 89 to 92 or 482 and/or lines 93 to 95 or 472 to 475 and/or lines 96 to 100 or 476 to 477 and/or line 101 to 102 or 478 to 481, resp., or a fragment thereof, which confers an increase in the amount of vitamin E or its precursor 2,3-dimethyl-5-phytylquinolin an organism or a part thereof;
   b) nucleic acid molecule comprising of a nucleic acid molecule as indicated in Table I, columns 5 or 7, lines 89 to 92 or 482 and/or lines 93 to 95 or 472 to 475 and/or lines 96 to 100 or 476 to 477 and/or lines 101 to 102 or 478 to 481, resp., preferably Table I B, column 7, lines 89 to 92 or 482 and/or lines 93 to 95 or 472 to 475 and/or lines 96 to 100 or 476 to 477 and/or line 101 to 102 or 478 to 481, resp.;
   c) nucleic acid molecule whose sequence can be deduced from a polypeptide sequence encoded by a nucleic acid molecule of (a) or (b) as a result of the degeneracy of the genetic code and conferring an increase in the amount of vitamin E or its precursor 2,3-dimethyl-5-phytylquinol in an organism or a part thereof;
   d) nucleic acid molecule which encodes a polypeptide which has at least 50% identity with the amino acid sequence of the polypeptide encoded by the nucleic acid molecule of (a) to (c) and conferring an increase in the amount of vitamin E or its precursor 2,3-dimethyl-5-phytylquinol in an organism or a part thereof;
   e) nucleic acid molecule which hybridizes with a nucleic acid molecule of (a) to (c) under stringent hybridisation conditions and conferring an increase in the amount of vitamin E or its precursor 2,3-dimethyl-5-phytylquinol in an organism or a part thereof;
   f) nucleic acid molecule which encompasses a nucleic acid molecule which is obtained by amplifying nucleic acid molecules from a cDNA library or a genomic library using the primers or primer pairs as indicated in Table III, column 7, lines 89 to 92 or 482 and/or lines 93 to 95 or 472 to 475 and/or lines 96 to 100 or 476 to 477 and/or lines 101 to 102 or 478 to 481 and conferring an increase in the amount of vitamin E or its precursor 2,3-dimethyl-5-phytylquinolin an organism or a part thereof;
   g) nucleic acid molecule encoding a polypeptide which is isolated with the aid of monoclonal antibodies against a polypeptide encoded by one of the nucleic acid molecules of (a) to (f) and conferring an increase in the amount of vitamin E or its precursor 2,3-dimethyl-5-phytylquinol in an organism or a part thereof;
   h) nucleic acid molecule encoding a polypeptide comprising a consensus as indicated in Table IV, column 7, lines 89 to 92 or 482 and/or lines 93 to 95 or 472 to 475 and/or lines 96 to 100 or 476 to 477 and/or lines 101 to 102 or 478 to 481 and conferring an increase in the amount of vitamin E or its precursor 2,3-dimethyl-5-phytylquinol in an organism or a part thereof; and
   i) nucleic acid molecule which is obtainable by screening a suitable nucleic acid library under stringent hybridization conditions with a probe comprising one of the sequences of the nucleic acid molecule of (a) to (k) or with a fragment thereof having at least 15 nt, preferably 20 nt, 30 nt, 50 nt, 100 nt, 200 nt or 500 nt of the nucleic acid molecule characterized in (a) to (k) and conferring an increase in the amount of vitamin E or its precursor 2,3-dimethyl-5-phytylquinol in an organism or a part thereof.
   or comprising a sequence which is complementary thereto.
3. The process of claim 1 or 2, wherein 2,3-dimethyl-5-phytylquinol is isolated.
4. The process of any one of claim 1 to 3, comprising the following steps:
   (a) selecting an organism or a part thereof expressing a polypeptide encoded by the nucleic acid molecule characterized in claim 2;
   (b) mutagenizing the selected organism or the part thereof;
   (c) comparing the activity or the expression level of said polypeptide in the mutagenized organism or the part thereof with the activity or the expression of said polypeptide of the selected organisms or the part thereof;
   (d) selecting the mutated organisms or parts thereof, which comprise an increased activity or expression level of said polypeptide compared to the selected organism or the part thereof;
   (e) optionally, growing and cultivating the organisms or the parts thereof; and
   (f) recovering, and optionally isolating, the free or bound vitamin E or its precursor 2,3-dimethyl-5-phytylquinol produced by the selected mutated organisms or parts thereof.
5. The process of any one of claims 1 to 4, wherein the activity of said protein or the expression of said nucleic acid molecule is increased or generated transiently or stably.
6. An isolated nucleic acid molecule comprising a nucleic acid molecule selected from the group consisting of:
   a) nucleic acid molecule encoding of a polypeptide as indicated in Table II, columns 5 or 7, lines 89 to 92 or 482 and/or lines 93 to 95 or 472 to 475 and/or lines 96 to 100 or 476 to 477 and/or lines 101 to 102 or 478 to 481 resp., preferably Table II B, column 7, lines 89 to 92 or 482 and/or lines 93 to 95 or 472 to 475 and/or lines 96 to 100 or 476 to 477 and/or line 101 to 102 or 478 to 481, resp., or a fragment thereof, which confers an increase in the amount of vitamin E or its precursor 2,3-dimethyl-5-phytylquinol in an organism or a part thereof;
   b) nucleic acid molecule comprising of a nucleic acid molecule as indicated in Table I, columns 5 or 7, lines 89 to 92 or 482 and/or lines 93 to 95 or 472 to 475 and/or lines 96 to 100 or 476 to 477 and/or lines 101 to 102 or 478 to 481, resp., preferably Table I B, column 7, lines 89 to 92 or 482 and/or lines 93 to 95 or 472 to 475 and/or lines 96 to 100 or 476 to 477 and/or line 101 to 102 or 478 to 481, resp.,;
   c) nucleic acid molecule whose sequence can be deduced from a polypeptide sequence encoded by a nucleic acid molecule of (a) or (b) as a result of the degeneracy of the genetic code and conferring an increase in the amount of vitamin E or its precursor 2,3-dimethyl-5-phytylquinol in an organism or a part thereof;
   d) nucleic acid molecule which encodes a polypeptide which has at least 50% identity with the amino acid sequence of the polypeptide encoded by the nucleic acid molecule of (a) to (c) and conferring an increase in the amount of vitamin E or its precursor 2,3-dimethyl-5-phytylquinol in an organism or a part thereof;
   e) nucleic acid molecule which hybridizes with a nucleic acid molecule of (a) to (c) under stringent hybridisation conditions and conferring an increase in the amount of vitamin E or its precursor 2,3-dimethyl-5-phytylquinol in an organism or a part thereof;
   f) nucleic acid molecule which encompasses a nucleic acid molecule which is obtained by amplifying nucleic acid molecules from a cDNA library or a genomic library using the primers or primer pairs as indicated in Table III, column 7, lines 89 to 92 or 482 and/or lines 93 to 95 or 472 to 475 and/or lines 96 to 100 or 476 to 477 and/or lines 101 to 102 or 478 to 481 and conferring an increase in the amount of vitamin E or its precursor 2,3-dimethyl-5-phytylquinol in an organism or a part thereof;
   g) nucleic acid molecule encoding a polypeptide which is isolated with the aid of monoclonal antibodies against a polypeptide encoded by one of the nucleic acid molecules of (a) to (f) and conferring an increase in the amount of vitamin E or its precursor 2,3-dimethyl-5-phytylquinol in an organism or a part thereof;
   h) nucleic acid molecule encoding a polypeptide comprising a consensus as indicated in Table IV, columns 7, lines 89 to 92 or 482 and/or lines 93 to 95 or 472 to 475 and/or lines 96 to 100 or 476 to 477 and/or lines 101 to 102 or 478 to 481 and conferring an increase in the amount of vitamin E or its precursor 2,3-dimethyl-5-phytylquinol in an organism or a part thereof; and
   i) nucleic acid molecule which is obtainable by screening a suitable nucleic acid library under stringent hybridization conditions with a probe comprising one of the sequences of the nucleic acid molecule of (a) to (k) or with a fragment thereof having at least 15 nt, preferably 20 nt, 30 nt, 50 nt, 100 nt, 200 nt or 500 nt of the nucleic acid molecule characterized in (a) to (k) and conferring an increase in the amount of vitamin E or its precursor 2,3-dimethyl-5-phytylquinol in an organism or a part thereof.
   whereby the nucleic acid molecule distinguishes over the sequence as indicated in Table I A, columns 5 or 7, lines 89 to 92 or 482 and/or lines 93 to 95 or 472 to 475 and/or lines 96 to 100 or 476 to 477 and/or lines 101 to 102 or 478 to 481 by one or more nucleotides.
7. A nucleic acid construct which confers the expression of the nucleic acid molecule of claim 6, comprising one or more regulatory elements.
8. A vector comprising the nucleic acid molecule as claimed in claim 6 or the nucleic acid construct of claim 7.
9. The vector as claimed in claim 8, wherein the nucleic acid molecule is in operable linkage with regulatory sequences for the expression in a prokaryotic or eukaryotic, or in a prokaryotic and eukaryotic, host.
10. A host cell, which has been transformed stably or transiently with the vector as claimed in claim 8 or 9 or the nucleic acid molecule as claimed in claim 6 or the nucleic acid construct of claim 7 or produced as described in claim any one of claims 2 to 5.
11. The host cell of claim 10, which is a transgenic host cell.
12. The host cell of claim 10 or 11, which is a plant cell, an animal cell, a microorganism, or a yeast cell, a fungus cell, a prokaryotic cell, an eukaryotic cell or an archaebacterium.
13. A process for producing a polypeptide, wherein the polypeptide is expressed in a host cell as claimed in any one of claims 10 to 12.
14. A polypeptide produced by the process as claimed in claim 13 or encoded by the nucleic acid molecule as claimed in claim 6 whereby the polypeptide distinguishes over a sequence as indicated in Table II A, columns 5 or 7, lines 89 to 92 or 482 and/or lines 93 to 95 or 472 to 475 and/or lines 96 to 100 or 476 to 477 and/or lines 101 to 102 or 478 to 481 by one or more amino acids
15. An antibody, which binds specifically to the polypeptide as claimed in claim 14.
16. A plant tissue, propagation material, harvested material or a plant comprising the host cell as claimed in claim 12 which is plant cell or an Agrobacterium.
17. A method for screening for agonists and antagonists of the activity of a polypeptide encoded by the nucleic acid molecule of claim 6 conferring an increase in the amount of vitamin E or its precursor 2,3-dimethyl-5-phytylquinol in an organism or a part thereof comprising:
   (a) contacting cells, tissues , plants or microorganisms which express the a polypeptide encoded by the nucleic acid molecule of claim 6 conferring an increase in the amount of vitamin E or its precursor 2,3-dimethyl-5-phytylquinol in an organism or a part thereof with a candidate compound or a sample comprising a plurality of compounds under conditions which permit the expression the polypeptide;
   (b) assaying the vitamin E level or its precursor 2,3-dimethyl-5-phytylquinol level or the polypeptide expression level in the cell, tissue, plant or microorganism or the media the cell, tissue, plant or microorganisms is cultured or maintained in; and
   (c) identifying a agonist or antagonist by comparing the measured vitamin E or its precursor 2,3-dimethyl-5-phytylquinol level or polypeptide expression level with a standard vitamin E or its precursor 2,3-dimethyl-5-phytylquinol acid or polypeptide expression level measured in the absence of said candidate compound or a sample comprising said plurality of compounds, whereby an increased level over the standard indicates that the compound or the sample comprising said plurality of compounds is an agonist and a decreased level over the standard indicates that the compound or the sample comprising said plurality of compounds is an antagonist.
18. A process for the identification of a compound conferring increased vitamin E or its precursor 2,3-dimethyl-5-phytylquinol production in a plant or microorganism, comprising the steps:
   (a) culturing a plant cell or tissue or microorganism or maintaining a plant expressing the polypeptide encoded by the nucleic acid molecule of claim 6 conferring an increase in the amount of vitamin E or its precursor 2,3-dimethyl-5-phytylquinol in an organism or a part thereof and a readout system capable of interacting with the polypeptide under suitable conditions which permit the interaction of the polypeptide with said readout system in the presence of a compound or a sample comprising a plurality of compounds and capable of providing a detectable signal in response to the binding of a compound to said polypeptide under conditions which permit the expression of said readout system and of the polypeptide encoded by the nucleic acid molecule of claim 6 conferring an increase in the amount of vitamin E or its precursor 2,3-dimethyl-5-phytylquinol in an organism or a part thereof;
   (b) identifying if the compound is an effective agonist by detecting the presence or absence or increase of a signal produced by said readout system.
19. A method for the identification of a gene product conferring an increase in vitamin E or its precursor 2,3-dimethyl-5-phytylquinol production in a cell, comprising the following steps:
   (a) contacting the nucleic acid molecules of a sample, which can contain a candidate gene encoding a gene product conferring an increase in vitamin E or its precursor 2,3-dimethyl-5-phytylquinol after expression with the nucleic acid molecule of claim 6;
   (b) identifying the nucleic acid molecules, which hybridise under relaxed stringent conditions with the nucleic acid molecule of claim 6;
   (c) introducing the candidate nucleic acid molecules in host cells appropriate for producing vitamin E or its precursor 2,3-dimethyl-5-phytylquinol;
   (d) expressing the identified nucleic acid molecules in the host cells;
   (e) assaying the vitamin E or its precursor 2,3-dimethyl-5-phytylquinol level in the host cells; and
   (f) identifying nucleic acid molecule and its gene product which expression confers an increase in the vitamin E or its precursor 2,3-dimethyl-5-phytylquinol level in the host cell in the host cell after expression compared to the wild type.
20. A method for the identification of a gene product conferring an increase in vitamin E or its precursor 2,3-dimethyl-5-phytylquinol production in a cell, comprising the following steps:
   (a) identifying in a data bank nucleic acid molecules of an organism; which can contain a candidate gene encoding a gene product conferring an increase in the vitamin E or its precursor 2,3-dimethyl-5-phytylquinol amount or level in an organism or a part thereof after expression, and which are at least 20% homolog to the nucleic acid molecule of claim 6;
   (b) introducing the candidate nucleic acid molecules in host cells appropriate for producing vitamin E or its precursor 2,3-dimethyl-5-phytylquinol;
   (c) expressing the identified nucleic acid molecules in the host cells;
   (d) assaying the vitamin E or its precursor 2,3-dimethyl-5-phytylquinol level in the host cells; and
   (e) identifying nucleic acid molecule and its gene product which expression confers an increase in the vitamin E or its precursor 2,3-dimethyl-5-phytylquinol level in the host cell after expression compared to the wild type.
21. A method for the production of an agricultural composition comprising the steps of the method of any one of claims 17 to 20 and formulating the compound identified in any one of claims 17 to 20 in a form acceptable for an application in agriculture.
22. A composition comprising the nucleic acid molecule of claim 6, the polypeptide of claim 14, the nucleic acid construct of claim 7, the vector of any one of claims 8 or 9, an antagonist or agonist identified according to claim 17, the compound of claim 18, the gene product of claim 19 or 20, the antibody of claim 15, and optionally an agricultural acceptable carrier.
23. Use of the nucleic acid molecule as claimed in claim 6 for the identification of a nucleic acid molecule conferring an increase of vitamin E or its precursor 2,3-dimethyl-5-phytylquinol after expression.
24. Use of the polypeptide of claim 14 or the nucleic acid construct claim 7 or the gene product identified according to the method of claim 19 or 20 for identifying compounds capable of conferring a modulation of vitamin E or its precursor 2,3-dimethyl-5-phytylquinol levels in an organism.
25. Cosmetic, pharmaceutical, food or feed composition comprising the nucleic acid molecule of claim 6, the polypeptide of claim 14, the nucleic acid construct of claim 7, the vector of claim 8 or 9, the antagonist or agonist identified according to claim 17, the antibody of claim 15, the plant or plant tissue of claim 16, the harvested material of claim 16, the host cell of claim 10 to 12 or the gene product identified according to the method of claim 19 or 20.
26. The method of any one of claims 1 to 5, the nucleic acid molecule of claim 6, the polypeptide of claim 14, the nucleic acid construct of claim 7, the vector of claim 8 or 9, the antagonist or agonist identified according to claim 17, the antibody of claim 15, the plant or plant tissue of claim 16, the harvested material of claim 16, the host cell of claim 10 to 12 or the gene product identified according to the method of claim 19 or 20, wherein the vitamin E is alpha-, beta-, or gamma-tocopherol, resp.
27. Use of the nucleic acid molecule of claim 6, the polypeptide of claim 14, the nucleic acid construct of claim 7, the vector of claim 8 or 9, the antagonist or agonist identified according to claim 17, the antibody of claim 15, the plant or plant tissue of claim 16, the harvested material of claim 16, the host cell of claim 10 to 12 or the gene product identified according to the method of claim 19 or 20 for the protection of a plant against a oxidative stress.
28. Use of the nucleic acid molecule of claim 6, the polypeptide of claim 14, the nucleic acid construct of claim 7, the vector of claim 8 or 9, the antagonist or agonist identified according to claim 17, the antibody of claim 15, the plant or plant tissue of claim 16, the harvested material of claim 16, the host cell of claim 10 to 12 or the gene product identified according to the method of claim 19 or 20 for the protection of a plant against a oxidative stress causing herbicide.
29. Use of the agonist identified according to claim 17, the plant or plant tissue of claim 16, the harvested material of claim 16, or the host cell of claim 10 to 12 for the production of a cosmetic or pharmaceutical composition.

**[0554.0.0.9] Abstract: see [0554.0.0.0]**
Process for the production of fine chemicals

**[0000.0.0.10] see [0000.0.0.1]**

**[0001.0.0.10]** see **[0001.0.0.0]**

**[0002.0.10.10]** Carotenoids are red, yellow and orange pigments that are widely distributed in nature. Although specific carotenoids have been identified in photosynthetic centers in plants, in bird feathers, in crustaceans and in marigold petals, they are especially abundant in yellow-orange fruits and vegetables and dark green, leafy vegetables. Of the more than 700 naturally occurring carotenoids identified thus far, as many as 50 may be absorbed and metabolized by the human body. To date, only 14 carotenoids have been identified in human serum.
In animals some carotenoids (particularly beta-carotene) serve as dietary precursors to Vitamin A, and many of them may function as fat-soluble antioxidants. In plants carotenes serve for example as antioxidants to protect the highly reactive photosystems and act as accessory photopigments. In vitro experiments have shown that lycopene, alpha-carotene, zeaxanthin, lutein and cryptoxanthin quench singlet oxygen and inhibit lipid peroxidation. The isolation and identification of oxidized metabolites of lutein, zeaxanthin and lycopene provide direct evidence of the antioxidant action of these carotenoids.
Carotenoids are 40-carbon (C₄₀) terpenoids generally comprising eight isoprene (C₅) units joined together. Linking of the units is reversed at the center of the molecule. "Ketocarotenoid" is a general term for carotenoid pigments that contain a keto group in the ionene ring portion of the molecule, whereas "hydroxycarotenoid" refers to carotenoid pigments that contain a hydroxyl group in the ionene ring. Trivial names and abbreviations will be used throughout this disclosure, with IUPAC-recommended semi-systematic names usually being given in parentheses after first mention of a trivial name.
Carotenoids are synthesized from a five carbon atom metabolic precursor, isopentenyl pyrophosphate (IPP). There are at least two known biosynthetic pathways in the formation of IPP, the universal isoprene unit. One pathway begins with mevalonic acid, the first specific precursor of terpenoids, formed from acetyl-CoA via HMG-CoA (3-hydroxy-3-methylglutaryl-CoA), that is itself converted to isopentenyl pyrophosphate (IPP). Later, condensation of two geranylgeranyl pyrophosphate (GGPP) molecules with each other produces colorless phytoene, which is the initial carotenoid. Studies have also shown the existence of an alternative, mevalonate-independent pathway for IPP formation that was characterized initially in several species of eubacteria, a green alga, and in the plastids of higher plants. The first reaction in this alternative pathway is the transketolase-type condensation reaction of pyruvate and D-glyceraldehylde-3-phosphate to yield 1-deoxy-D-xylulose-5-phosphate (DXP) as an intermediate.
Through a series of desaturation reactions, phytoene is converted to phytofluene, - carotene, neurosporene and finally to lycopene. Subsequently, lycopene is converted by a cyclization reaction to β-carotene that contains two β-ionene rings. A keto-group and/or a hydroxyl group are introduced into each ring of β-carotene to thereby synthesize canthaxanthin, zeaxanthin, astaxanthin. A hydroxylase enzyme has been shown to convert canthaxanthin to astaxanthin. Similarly, a ketolase enzyme has been shown to convert zeaxanthin to astaxanthin. The ketolase also converts β-carotene to canthaxanthin and the hydroxylase converts β-carotene to zeaxanthin.
Carotenoids absorb light in the 400-500 nm region of the visible spectrum. This physical property imparts the characteristic red/yellow color of the pigments. A conjugated backbone composed of isoprene units is usually inverted at the center of the molecule, imparting symmetry. Changes in geometrical configuration about the double bonds result in the existence of many *cis*- and *trans*- isomers. Hydroxylated, oxidized, hydrogenated or ring-containing derivatives also exist. Hydrocarbon carotenoids are classified as carotenes while those containing oxygen are known as xanthophylls.
In animals, carotenoids are absorbed from the intestine with the aid of dietary fat and incorporated into chylomicrons for transport in the serum. The different structural features possessed by carotenoids account for selective distribution in organ tissue, biological activity and pro-vitamin A potency, or *in vivo* conversion to vitamin A. Due to the hydrophobic character, carotenoids are associated with lipid portions of human tissues, cells, and membranes. In general, 80-85% of carotenoids are distributed in adipose tissue, with smaller amounts found in the liver, muscle, adrenal glands, and reproductive organs. Approximately 1% circulate in the serum on high and low density lipoproteins. Serum concentrations are fairly constant and slow to change during periods of low intake. The estimated half-life was estimated to be 11-14 days for lycopene, -carotene, -carotene, lutein and zeaxanthin. Evidence for the existence of more than one body pool has been published. The major serum carotenoids are - carotene, -carotene, lutein, zeaxanthin, lycopene and cryptoxanthin. Smaller amounts of polyenes such as phytoene and phytofluene are also present.
Human serum levels reflect lifestyle choices and dietary habits within and between cultures. Approximately only 15 circulate in the blood, on HDL and LDL. Variations can be attributed to different intakes, unequal abilities to absorb certain carotenoids, and different rates of metabolism and tissue uptake. Decreased serum levels occur with alcohol consumption, the use of oral contraceptives, smoking and prolonged exposure to UV light.
-Carotene, -carotene and -cryptoxanthin can be converted to retinol or vitamin A in the intestine and liver by the enzyme 15-15'-b-carotenoid dioxygenase. Such *in vivo* formation of retinol appears to be homeostatically controlled, such that conversion to retinol is limited in persons having adequate vitamin A status.

[0003.0.10.10] The established efficacy of beta-carotene in quenching singlet oxygen and intercepting deleterious free radicals and reactive oxygen species makes it part of the diverse antioxidant defense system in humans. Reactive oxygen species have been implicated in the development of many diseases, including ischemic heart disease, various cancers, cataracts and macular degeneration. Because the conjugated polyene portion of beta-carotene confers its antioxidant capability and all carotenoids possess this structural feature, research efforts have been directed at evaluating the efficacy of other carotenoids in the prevention of free radical-mediated diseases. Indeed, *in vitro* experiments have demonstrated that lycopene, alpha-carotene, zeaxanthin, lutein and cryptoxanthin quench singlet oxygen and inhibit lipid peroxidation. The isolation and identification of oxidized metabolites of lutein, zeaxanthin and lycopene may provide direct evidence of the antioxidant action of these carotenoids.
In addition to antioxidant capability, other biological actions of carotenoids include the ability to enhance immunocompetence and in vitro gap junction communication, reduce or inhibited mutagenesis and inhibit cell transformations in vitro.
Many epidemiological studies have established an inverse correlation between dietary intake of yellow-orange fruit and dark green, leafy vegetables and the incidence of various cancers, especially those of the mouth, pharynx, larynx, esophagus, lung, stomach, cervix and bladder. While a number of protective compounds may be responsible for this observation, the co-incidence of carotenoids in these foods has been noted. Because nutritionists and medical professionals currently recognize the occurrence of a large number of distinct carotenoids in food, interest in their functions and biological impact on health is burgeoning.
Lutein exists in the retina. It functions to protect photoreceptor cells from light-generated oxygen radicals, and thus plays a key role in preventing advanced macular degeneration. Lutein possesses chemopreventive activity, induces gap junction communication between cells and inhibits lipid peroxidation in vitro more effectively than beta-carotene, alpha-carotene and lycopene. High levels of lutein in serum have been inversely correlated with lung cancer.
In addition to lutein, zeaxanthin exists in the retina and confers protection against macular degeneration. Zeaxanthin is also prevalent in ovaries and adipocyte tissue. This xanthophyll does not possess provitamin A activity.
Alcohol consumption has been shown to influence lipid peroxidation. Anhydrolutein, an oxidative by-product of lutein and zeaxanthin, was higher in plasma after alcohol ingestion, while concentrations of these xanthophylls were reduced. Lutein and zeaxanthin may therefore have protective effects against LDL oxidation.
The all-trans isomer of Lycopene is typically quantified in serum, although signals for 9-, 13- and 15-cis isomers are detectable and account for as much as 50% of the total lycopene. In experiments performed *in vitro*, lycopene quenched singlet oxygen more efficiently than alpha-carotene, beta-carotene, zeaxanthin, lutein and cryptoxanthin. Lycopene induces gap junction communication, inhibits lipid peroxidation and has displays chemopreventive activity. Serum levels of lycopene have been inversely related to the risk of cancer in the pancreas and cervix. This carotenoid has been identified in tissues of the thyroid, kidneys, adrenals, spleen, liver, heart, testes and pancreas. Lycopene is not converted to retinol *in vivo.*
beta-Cryptoxanthin is capable of quenching singlet oxygen. beta-Cryptoxanthin is used to color butter. beta-Cryptoxanthin exhibits provitamin A activity.
The all-trans isomer of this carotenoid is the major source of dietary retinoids, due to its high provitamin A activity. One molecule of trans-beta-carotene can theoretically provide two molecules of trans retinaldehyde in vivo. Signals for 13- and 15-cis isomers of beta-carotene are also observed in the carotenoid profile and account for 10% or less of the total beta-carotene in serum. beta-Carotene quenches singlet oxygen, induces gap junction communication and inhibits lipid peroxidation. High serum levels of beta-carotene are correlated with low incidences of cancer in the mouth, lung, breast, cervix, skin and stomach. beta-Carotene has been identified in tissues of the thyroid, kidney, spleen, liver, heart, pancreas, fat, ovaries and adrenal glands. alpha-Carotene is similar to beta-carotene in its biological activity, but quenches singlet oxygen more effectively. alpha-Carotene improves gap junction communication, prevents lipid peroxidation and inhibits the formation and uptake of carcinogens in the body. High serum levels have been associated with lower risks of lung cancer. With one half the provitamin A potency of beta-carotene, alpha-carotene also restores normal cell growth and differentiation. Serum levels are usually between 10 and 20% of the values for total beta-carotene.
Alpha-Carotene, beta-carotene and beta-cryptoxanthin can be converted to Vitamin A in the intestine and liver. Vitamin A is essential for the immune response and is also involved in other defenses against infectious agents. Nevertheless, in many individuals, this conversion is slow and ineffectual, particularly for older. Some individuals are known as non or low-responders because they do not convert beta-carotene to Vitamin A at the rate as expected. A number of factors can inhibit this conversion of beta-carotene to Vitamin A. The major reason why so many Americans have a poor vitamin A status is the regular use of excessive alcohol. Intestinal parasites can be a factor. And, any prescription drug that requires liver metabolism will decrease the liver conversion of beta-carotene to retinol in the liver. Diabetics and individuals with hypothyroidism or even borderline hypothyroidism are likely to be low-responders.

**[0004.0.10.10]** In plants, approximately 80-90% of the carotenoids present in green, leafy vegetables such as broccoli, kale, spinach and brussel sprouts are xanthophylls, whereas 10-20% are carotenes. Conversely, yellow and orange vegetables including carrots, sweet potatoes and squash contain predominantly carotenes. Up to 60% of the xanthophylls and 15% of the carotenes in these foods are destroyed during microwave cooking. Of the xanthophylls, lutein appears to be the most stable.
Lutein occurs in mango, papaya, oranges, kiwi, peaches, squash, peas, lima beans, green beans, broccoli, brussel sprouts, cabbage, kale, lettuce, prunes, pumpkin, sweet potatoes and honeydew melon. Commercial sources are obtained from the extraction of marigold petals. Lutein does not possess provitamin A activity.
Dietary sources of Zeaxanthin include peaches, squash, apricots, oranges, papaya, prunes, pumpkin, mango, kale, kiwi, lettuce, honeydew melon and yellow corn.
The red color of fruits and vegetables such as tomatoes, pink grapefruit, the skin of red grapes, watermelon and red guavas is due to lycopene. Other dietary sources include papaya and apricots.
beta-Cryptoxanthin occurs in oranges, mango, papaya, cantaloupe, peaches, prunes, squash.
Dietary sources of beta-Carotene include mango, cantaloupe, carrots, pumpkin, papaya, peaches, prunes, squash, sweet potato, apricots, cabbage, lima beans, green beans, broccoli, brussel sprouts, kale, kiwi, lettuce, peas, spinach, tomatoes, pink grapefruit, honeydew melon and oranges.
Dietary sources of alpha-Carotene include sweet potatoes, apricots, pumpkin, cantaloupe, green beans, lima beans, broccoli, brussel sprouts, cabbage, kale, kiwi, lettuce, peas, spinach, prunes, peaches, mango, papaya, squash and carrots.

**[0005.0.10.10]**Some carotenoids occur particularly in a wide variety of marine animals including fish such as salmonids and sea bream, and crustaceans such as crab, lobster, and shrimp. Because animals generally cannot biosynthesize carotenoids, they obtain those carotenoids present in microorganisms or plants upon which they feed.
Carotenoids e.g. xanthophylls, e.g. as astaxanthin, supplied from biological sources, such as crustaceans, yeast, and green alga is limited by low yield and costly extraction methods when compared with that obtained by organic synthetic methods. Usual synthetic methods, however, produce by-products that can be considered unacceptable. It is therefore desirable to find a relatively inexpensive source of carotenoids, in particular xantophylls, to be used as a feed supplement in aquaculture and as a valuable chemical for other industrial uses and for diets. Sources of Xanthophylls include crustaceans such as a krill in the Antarctic Ocean, cultured products of the yeast *Phaffia*, cultured products of a green alga *Haematococcus pluvialis*, and products obtained by organic synthetic methods. However, when crustaceans such as a krill or the like are used, a great deal of work and expense are required for the isolation of xanthophylls from contaminants such as lipids and the like during the harvesting and extraction. Moreover, in the case of the cultured product of the yeast *Phaffia*, a great deal of expense is required for the gathering and extraction of astaxanthin because the yeast has rigid cell walls and produces xantophylls only in a low yield. One approach to increase the productivity of some xantophylls' production in a biological system is to use genetic engineering technology.

**[0006.0.10.10]**In many plants, lycopene is a branch point in carotenoid biosynthesis. Thus, some of the plant's lycopene is made into beta-carotene and zeaxanthin, and sometimes zeaxanthin diglucoside, whereas remaining portions of lycopene are formed into alpha-carotene and lutein (3,3'-dihydroxy-α-carotene), another hydroxylated compound. Carotenoids in higher plants; i.e., angiosperms, are found in plastids; i.e., chloroplasts and chromoplasts. Plastids are intracellular storage bodies that differ from vacuoles in being surrounded by a double membrane rather than a single membrane. Plastids such as chloroplasts can also contain their own DNA and ribosomes, can reproduce independently and synthesize some of their own proteins. Plastids thus share several characteristics of mitochondria. In leaves, carotenoids are usually present in the grana of chloroplasts where they provide a photoprotective function. Beta-carotene and lutein are the predominant carotenoids, with the epoxidized carotenoids violaxanthin and neoxanthin being present in smaller amounts. Carotenoids accumulate in developing chromoplasts of flower petals, usually with the disappearance of chlorophyll. As in flower petals, carotenoids appear in fruit chromoplasts as they develop from chloroplasts. Most enzymes that take part in conversion of phytoene to carotenes and xanthophylls are labile, membrane-associated proteins that lose activity upon solubilization. In maize, cartonoids were present in horny endosperm (74% to 86%), floury endosperm (9%-23%) and in the germ and bran of the kernel.

**[0007.0.10.10]**At the present time only a few plants are widely used for commercial colored carotenoid production. However, the productivity of colored carotenoid synthesis in most of these plants is relatively low and the resulting carotenoids are expensively produced.
Dried marigold petals and marigold petal concentrates obtained from so-called xanthophyll marigolds are used as feed additives in the poultry industry to intensify the yellow color of egg yolks and broiler skin. The pigmenting ability of marigold petal meal resides largely in the carotenoid fraction known as the xanthophylls, primarily lutein esters. The xanthophyll zeaxanthin, also found in marigold petals, has been shown to be effective as a broiler pigmenter, producing a highly acceptable yellow to yellow-orange color. Of the xanthophylls, the pigments lutein and zeaxanthin are the most abundant in commercially available hybrids. Structural formulas for lutein and zeaxanthin are shown below.
Carotenoids have been found in various higher plants in storage organs and in flower petals. For example, marigold flower petals accumulate large quantities of esterified lutein as their predominant xanthophyll carotenoid (about 75 to more than 90 percent), with smaller amounts of esterified zeaxanthin. Besides lutein and zeaxanthin, marigold flower petals also typically exhibit a small accumulation of β-carotene and epoxidized xanthophylls, but do not produce or accumulate canthaxanthin or astaxanthin because a 4-keto-β-ionene ring-forming enzyme is absent in naturally-occurring marigolds or their hybrids.

**[0008.0.10.10]**One way to increase the productive capacity of biosynthesis is to apply recombinant DNA technology. Thus, it would be desirable to produce colored carotenoids generally and, with the use of recent advances in determining carotenoid biosynthesis from β-carotene to xanthophylls to control the production of carotenoids. That type of production permits control over quality, quantity and selection of the most suitable and efficient producer organisms. The latter is especially important for commercial production economics and therefore availability to consumers.
Methods of recombinant DNA technology have been used for some years to improve the production of Xanthophylls in microorganisms, in particular algae or in plants by amplifying individual xanthophyll biosynthesis genes and investigating the effect on xanthophyll production. It is for example reportet, that the five ketocarotenoids, e.g. the xanthophyll astaxanthin could be produced in the nectaries of transgenic tobacco plants. Those transgenic plants were prepared by *Argobacterium tumifaciens-*mediated transformation of tobacco plants using a vector that contained a ketolase-encoding gene from *H. pluvialis* denominated *crtO* along with the Pds gene from tomato as the promoter and to encode a leader sequence. The Pds gene was said by those workers to direct transcription and expression in chloroplasts and/or chromoplast-containing tissues of plants. Those results indicated that about 75 percent of the carotenoids found in the flower of the transformed plant contained a keto group. Further, in maize the phytonene synthase (Psy), Phytone desaturase (Pds), and the - carotene desaturase were identified and it was shown, that PSY activity is an important control point for the regulation of the flux.
Genes suitable for conversion of microorganisms have also been reported (U.S. Patent No. 6,150,130 WO 99/61652). Two different genes that can convert a carotenoid β-ionene ring compound into astaxanthin have been isolated from the green alga Haematococcus *pluvialis*. Zeaxanthin or -carotene were also found in the marine bacteria *Agrobacterium aurantiacum, Alcaligenes PC-1, Erwinia uredovora*. An *A. aurantiacum crtZ* gene was introduced to an *E. coli* transformant that accumulated all-*trans*-β-carotene. The transformant so formed produced zeaxanthin. A gene cluster encoding the enzymes for a carotenoid biosynthesis pathway has been also cloned from the purple photosynthetic bacterium *Rhodobacter capsulatus*. A similar cluster for carotenoid biosynthesis from ubiquitous precursors such as farnesyl pyrophosphate and geranyl pyrophosphate has been cloned from the non-photosynthetic bacteria *Erwinia herbicola*. Yet another carotenoid biosynthesis gene cluster has been cloned from *Erwinia uredovora*. It is yet unknown and unpredictable as to whether enzymes encoded by other organisms behave similarly to that of *A. aurantiacum in vitro* or *in vivo* after transformation into the cells of a higher plant.

**[0009.0.10.10]**In addition to the above said about the biological importance of carotenoids, e.g. in vision, bone growth, reproduction, immune function, gene expression, emboryonic expression, cell division and cell differation, and respiration, it should be mentioned that in the world, the prevalence of vitamin A deficiency ranges from 100 to 250 million children and an estimated 250.000 to 500.000 children go blind each year from vitamin A deficiency.
Thus, it would be advantageous if an algae or other microorganism were available who produce large amounts of β-carotene, beta-cryptoxanthin, lutein, zeaxanthin, or other carotenoids. It might be advantageous that only small amounts or no lutein is produced so that such organisms could be transformed with e.g. one or more of an appropriate hydroxylase gene and/or an appropriate ketolase gene to produce cryptoxanthin, zeaxanthin or astaxanthin. The invention discussed hereinafter relates in some embodiments to such transformed prokaryotic or eukaryotic microorganisms.
It would also be advantageous if a marigold or other plants were available whose flowers produced large amounts of β-carotene, beta-cryptoxanthin, lutein, zeaxanthin, or other carotenoids. It might be advantageous that only small amounts or no lutein is produced so that such plants could be transformed with one or more of an appropriate hydroxylase gene and an appropriate ketolase gene to produce cryptoxanthin, zeaxanthin or astaxanthin from e.g the flowers of the resulting transformants. The invention discussed hereinafter relates in some embodiments to such transformed plants.

**[0010.0.10.10]**Therefore improving the quality of foodstuffs and animal feeds is an important task of the food-and-feed industry. This is necessary since, for example, as mentioned above xanthophylls, which occur in plants and some microorganisms are limited with regard to the supply of mammals. Especially advantageous for the quality of foodstuffs and animal feeds is as balanced as possible a carotenoids profile in the diet since a great excess of some carotenoids above a specific concentration in the food has only some positive effect. A further increase in quality is only possible via addition of further carotenoids, which are limiting..

**[0011.0.10.10]**To ensure a high quality of foods and animal feeds, it is therefore necessary to add one or a plurality of carotenoids in a balanced manner to suit the organism.

**[0012.0.10.10]**Accordingly, there is still a great demand for new and more suitable genes which encode enzymes which participate in the biosynthesis of carotenoids, e.g. xanthophylls, e.g. like beta-crypotxanthin, or zeaxanthin, or astaxanthin, and make it possible to produce them specifically on an industrial scale without unwanted byproducts forming. In the selection of genes for biosynthesis two characteristics above all are particularly important. On the one hand, there is as ever a need for improved processes for obtaining the highest possible contents of carotenoids like xanthophylls; on the other hand as less as possible byproducts should be produced in the production process.

**[0013.0.0.10]** see **[0013.0.0.0]**

**[0014.0.10.10]**Accordingly, in a first embodiment, the invention relates to a process for the production of a fine chemical, whereby the fine chemical is a xanthophyll. Accordingly, in the present invention, the term "the fine chemical" as used herein relates to a "Xanthophyll". Further, in an other embodiment the term "the fine chemicals" as used herein also relates to compositions of fine chemicals comprising xanthophylls.

**[0015.0.10.10]**In one embodiment, the term xanthophylls" or "the fine chemical" or "the respective fine chemical" means at least one chemical compound with xanthophylls activity selected from the group comprising zeaxanthin or cryptoxanthin.
In one embodiment, the term "the fine chemical" means a xanthophyll. In one embodiment, the term "the fine chemical" means beta-cryptoxanthin or zeaxanthin depending on the context in which the term is used. Throughout the specification the term "the fine chemical" means a xanthophyll, in particular beta cryptoxanthin or zeaxanthin, its salts, ester, thioester or in free form or bound to other compounds such sugars or sugarpolymers, like glucoside, e.g. diglucoside. In one embodiment, the term "the fine chemicals" means beta-cryptoxanthin and zeaxanthin, in free form or their salts or their ester or bound to a glucoside, e.g a diglucoside. In one embodiment, the term "the fine chemical" and the term "the respective fine chemical" mean at least one chemical compound with an activity of the above mentioned fine chemical.

**[0016.0.10.10]**Accordingly, the present invention relates to a process comprising
(a) increasing or generating the activity of one or more b0050, b0851, b2211, b3926, b0986, b3684, b4401, and/or YCL040W protein(s) or a b2699 and/or YHR055C protein(s) or of a protein having the sequence of a polypeptide encoded by a nucleic acid molecule indicated in Table II, column 3, lines 103 to 108 or 468 to 471 in a non-human organism in one or more parts thereof; and
(b) growing the organism under conditions which permit the production of the fine chemical, thus, beta-cryptoxanthin or zeaxanthin, resp., or fine chemicals comprising beta-cryptoxanthin or zeaxanthin, resp., in said organism.
Accordingly, the present invention relates to a process comprising
(a) increasing or generating the activity of one or more proteins having the activity of a protein indicated in Table II, column 3, lines 103 to 108 or 468 to 471, resp., or having the sequence of a polypeptide encoded by a nucleic acid molecule indicated in Table I, column 5 or 7, lines 103 to 108 or 468 to 471, resp., in a non-human organism in one or more parts thereof; and
(b) growing the organism under conditions which permit the production of the fine chemical, thus, beta-cryptoxanthin or zeaxanthin, resp., or fine chemicals comprising beta-cryptoxanthin or zeaxanthin, resp., in said organism.

**[0016.1.0.10]** Accordingly, the term "the fine chemical" means "beta-cryptoxanthin" in relation to all sequences listed in Table I to Table IV, lines 103 to 106 and/or 468 to 471 or homologs thereof and means "zeaxanthin" in relation to all sequences listed in Table I to Table IV, lines 107 and 108 or homologs thereof. Accordingly, the term "the fine chemical" can mean "zeaxanthin" or "cryptoxanthin", owing to circumstances and the context. In order to illustrate that the meaning of the term "the respective fine chemical" means "cryptoxanthin", and/or "zeaxanthin" owing to the sequences listed in the context the term "the respective fine chemical" is also used.
The terms "beta-cryptoxanthin" and "cryptoxanthin" are used as equivalent terms.

**[0017.0.0.10]** to **[0018.0.0.10]: see [0017.0.0.0]** to **[0018.0.0.0]**

**[0019.0.10.10]**Advantageously the process for the production of the respective fine chemical leads to an enhanced production of the respective fine chemical. The terms "enhanced" or "increase" mean at least a 10%, 20%, 30%, 40% or 50%, preferably at least 60%, 70%, 80%, 90% or 100%, more preferably 150%, 200%, 300%, 400% or 500% higher production of the respective fine chemical in comparison to the reference as defined below, e.g. that means in comparison to an organism without the aforementioned modification of the activity of a protein having the activity of a protein indicated in Table II, column 3, lines 103 to 108 or 468 to 471 or encoded by nucleic acid molecule indicated in Table I, columns 5 or 7, lines 103 to 108 or 468 to 471.

**[0020.0.10.10]**Surprisingly it was found, that the transgenic expression of at least one of the proteins of the Saccharomyces cerevisiae protein YCL040W, and/or the Escherichia coli K12 protein b0050, b0851, b2211, b3926, b0986, b3684 and/or b4401 in Arabidopsis thaliana conferred an increase in the beta-cryptoxanthin ("the fine chemical" or "the fine respective chemical" in respect to said proteins and their homologs as wells as the encoding nucleic acid molecules, in particular as indicated in Table I or II, column 3, lines 103 to 106 and/or 468 to 471) content of the transformed plants.
Surprisingly it was found, that the transgenic expression of the Saccharomyces cerevisiae protein YHR055C, and/or the Escherichia coli K12 protein b2699 in Arabidopsis thaliana conferred an increase in the zeaxanthin ("the fine chemical" or "the fine respective chemical" in respect to said proteins and their homologs as wells as the encoding nucleic acid molecules, in particular as indicated in Table I or II, column 3, lines 107 and 108 of ) content of the transformed plants.

**[0021.0.0.10]** see **[0021.0.0.0]**

**[0022.0.10.10]** The sequence of b0050 from Escherichia coli K12 has been published in Blattner et al., Science 277(5331), 1453-1474, 1997, and its activity is being defined as conserved protein potentially involved in protein-protein interaction of the superfamily of apaG protein. Accordingly, in one embodiment, the process of the present invention comprises the use of a protein with an activity for proteolytic degradation, protein modification, modification by ubiquitination, deubiquitination, and/or protein binding, in particular being a conserved protein potentially involved in protein-protein interaction, in particular being a conserved protein potentially involved in protein-protein interaction of the superfamily of apaG protein from E. coli or its homolog, e.g. as shown herein, for the production of the respective fine chemical, meaning of xanthophylls, in particular for increasing the amount of beta-cryptoxanthin, preferably in free or bound form in an organism or a part thereof, as mentioned. In one embodiment, in the process of the present invention the activity of a conserved protein potentially involved in protein-protein interaction of the superfamily of apaG protein is increased or generated, e.g. from E. coli or a homolog thereof.
The sequence of b0851 from Escherichia coli K12 has been published in Blattner et al., Science 277(5331), 1453-1474, 1997, and its activity is being defined as oxygen insensitive NADPH nitroreductase of the superfamily of NADPH-flavin oxidoreductase homologs. Accordingly, in one embodiment, the process of the present invention comprises the use of a protein with an activity for nitrogen metabolism, sulfur metabolism, electron transport and membrane-associated energy conservation, stress response, electron / hydrogen carrier, C-compound and carbohydrate utilization, and/or utilization of vitamins, cofactors, and prosthetic groups and/or as a resistance protein, in particular being a oxygen insensitive NADPH nitroreductase, in particular being a oxygen insensitive NADPH nitroreductase of the superfamily of NADPH-flavin oxidoreductase homologs from E. coli or its homolog, e.g. as shown herein, for the production of the respective fine chemical, meaning of xanthophylls, in particular for increasing the amount of beta-cryptoxanthin, preferably in free or bound form in an organism or a part thereof, as mentioned. In one embodiment, in the process of the present invention the activity of a oxygen insensitive NADPH nitroreductase of the superfamily of NADPH-flavin oxidoreductase homologs is increased or generated, e.g. from E. coli or a homolog thereof.
The sequence of b2211 from Escherichia coli K12 has been published in Blattner et al., Science 277(5331), 1453-1474, 1997, and its activity is being defined as ATP-binding transport protein of the ABC superfamily of the superfamily of unassigned ATP-binding cassette proteins. Accordingly, in one embodiment, the process of the present invention comprises the use of a protein with an activity for nucleotide binding, ABC transporters, and/or CELLULAR TRANSPORT AND TRANSPORT MECHANISMS, in particular being a ATP-binding transport protein of the ABC superfamily, in particular being a ATP-binding transport protein of the ABC superfamily of the superfamily of unassigned ATP-binding cassette proteins from E. coli or its homolog, e.g. as shown herein, for the production of the respective fine chemical, meaning of xanthophylls, in particular for increasing the amount of beta-cryptoxanthin, preferably in free or bound form in an organism or a part thereof, as mentioned. In one embodiment, in the process of the present invention the activity of an ATP-binding transport protein of the ABC superfamily of the superfamily of unassigned ATP-binding cassette proteins is increased or generated, e.g. from E. coli or a homolog thereof.
The sequence of b3926 from Escherichia coli K12 has been published in Blattner et al., Science 277(5331), 1453-1474, 1997, and its activity is being defined as glycerol kinase of the superfamily of xylulokinase. Accordingly, in one embodiment, the process of the present invention comprises the use of a protein with an activity in the C-compound and carbohydrate catabolism, phospholipid biosynthesis, glycolipid biosynthesis, unspecified signal transduction, enzyme mediated signal transduction, and/or phosphotransferase system, in particular being a glycerol kinase, in particular being a glycerol kinase of the superfamily of xylulokinase from E. coli or its homolog, e.g. as shown herein, for the production of the respective fine chemical, meaning of xanthophylls, in particular for increasing the amount of beta-cryptoxanthin, preferably in free or bound form in an organism or a part thereof, as mentioned. In one embodiment, in the process of the present invention the activity of a glycerol kinase of the superfamily of xylulokinase is increased or generated, e.g. from E. coli or a homolog thereof.
The sequence of b0986 from Escherichia coli K12 has been published in Blattner et al., Science 277(5331), 1453-1474, 1997, and its activity is being defined as a lipoprotein of the superfamily of the hypothetical protein b1706. Accordingly, in one embodiment, the process of the present invention comprises the use of a lipoprotein, in particular of a lipoprotein of the superfamily of the hypothetical protein b1706 from E. coli or its homolog, e.g. as shown herein, for the production of the respective fine chemical, meaning of xanthophylls, in particular for increasing the amount of beta-cryptoxanthin, preferably in free or bound form in an organism or a part thereof, as mentioned. In one embodiment, in the process of the present invention the activity of a lipoprotein, in particular of a lipoprotein of the superfamily of the hypothetical protein b1706 is increased or generated, e.g. from E. coli or a homolog thereof.
The sequence of b3684 from Escherichia coli K12 has been published in Blattner et al., Science 277(5331), 1453-1474, 1997, and its activity is being defined as a transcriptional regulator of the GntR family. Accordingly, in one embodiment, the process of the present invention comprises the use of protein having a transcriptional control activity, a regulation of C-compound and carbohydrate utilization activity, a DNA binding activity, a transcriptional repressor activity, a regulation of lipid, fatty-acid and/or isoprenoid metabolism activity, and/or a regulation of C-compound and carbohydrate utilization activity, e.g. of a transcriptional regulator, in particular of a transcriptional regulator of the GntR family from E. coli or its homolog, e.g. as shown herein, for the production of the respective fine chemical, meaning of xanthophylls, in particular for increasing the amount of beta-cryptoxanthin, preferably in free or bound form in an organism or a part thereof, as mentioned. In one embodiment, in the process of the present invention said activity, e.g. the activity of a transcriptional regulator, in particular of a transcriptional regulator of the GntR family is increased or generated, e.g. from E. coli or a homolog thereof.
The sequence of b4401 from Escherichia coli K12 has been published in Blattner et al., Science 277(5331), 1453-1474, 1997, and its activity is being defined as a response regulator of the OmpR family. Accordingly, in one embodiment, the process of the present invention comprises the use of a protein of a transcriptional control activity, a unspecified signal transduction activity, a two-component signal transduction system (e.g. response regulator component activity, a transcriptional activator activity, a regulation of respiration activity, an aerobic respiration activity, and/or an anaerobic respiration activity, e.g. of a response regulator, in particular of a response regulator of the OmpR family from E. coli or its homolog, e.g. as shown herein, for the production of the respective fine chemical, meaning of xanthophylls, in particular for increasing the amount of beta-cryptoxanthin, preferably in free or bound form in an organism or a part thereof, as mentioned. In one embodiment, in the process of the present invention said activity, e.g. the activity of a response regulator, in particular of a response regulator of the OmpR family is increased or generated, e.g. from E. coli or a homolog thereof.
The sequence of b2699 from Escherichia coli K12 has been published in Blattner et al., Science 277(5331), 1453-1474, 1997, and its activity is being defined as a DNA strand exchange and recombination protein with protease and nuclease activity of the superfamily of the recombination protein recA. Accordingly, in one embodiment, the process of the present invention comprises the use of a protein with a DNA recombination and DNA repair activity, a pheromone response activity, a mating-type determination activity, a sex-specific protein activity, a nucleotide binding activity and/or a protease and nuclease activity, in particular a DNA strand exchange and recombination protein with protease and nuclease activity, in particular of the superfamily of the recombination protein recA from E. coli or its homolog, e.g. as shown herein, for the production of the respective fine chemical, meaning of xanthophylls, in particular for increasing the amount of zeaxanthin, preferably in free or bound form in an organism or a part thereof, as mentioned. In one embodiment, in the process of the present invention said activity, e.g. the activity of a protease and nuclease activity, in particular a DNA strand exchange and recombination protein with protease and nuclease activity, in particular of the superfamily of the recombination protein recA is increased or generated, e.g. from E. coli or a homolog thereof.
The sequence of YCL040W from Saccaromyces cerevisiae has been published in Oliver, S.G., et al., Nature 357 (6373), 38-46 (1992), and Goffeau et al., Science 274 (5287), 546-547, 1996, and its activity is being defined as glucokinase of the superfamily hexokinase. Accordingly, in one embodiment, the process of the present invention comprises the use of a protein having a C-compound and carbohydrate utilization activity, a C-compound or carbohydrate transport activity, a activity in the glycolysis and/or a activity in the gluconeogenesis, and/or a cellular import activity, in particular of a hexokinase, preferably of a glucinase or its homolog, e.g. as shown herein, for the production of the respective fine chemical, meaning of xanthophylls, in particular for increasing the amount of beta-cryptoxanthin in free or bound form in an organism or a part thereof, as mentioned. In one embodiment, in the process of the present invention said activity, e.g. the activity of a glucokinase is increased or generated, e.g. from Saccharomyces cerevisiae or a homolog thereof.
The sequence of YHR055C from Saccharomyces cerevisiae has been published in Johnston, M et al., Science 265 (5181), 2077-2082 (1994), and Goffeau et al., Science 274 (5287), 546-547, 1996, and its activity is being defined as copper binding metallothionein of the superfamily metallothionein. Accordingly, in one embodiment, the process of the present invention comprises the use of a protein having a detoxification activity, particular of a metallothionein, preferably of copper binding metallothionein or its homolog, e.g. as shown herein, for the production of the respective fine chemical, meaning of xanthophylls, in particular for increasing the amount of zeaxanthin in free or bound form in an organism or a part thereof, as mentioned. In one embodiment, in the process of the present invention said activity, e.g. the activity of a metellothionein, preferably of a copper binding metellothionein is increased or generated, e.g. from Saccharomyces cerevisiae or a homolog thereof.

**[0023.0.10.10]** Homologues (= homologs) of the present gene products can be derived from any organisms as long as the homologue confers the herein mentioned activity, in particular, confers an increase in the respective fine chemical amount or content. Further, in the present invention, the term "homologue" relates to the sequence of an organism having the highest sequence homology to the herein mentioned or listed sequences of all expressed sequences of said organism. However, the person skilled in the art knows, that, preferably, the homologue has said the-fine-chemical-increasing activity and, if known, the same biological function or activity in the organism as at least one of the protein(s) indicated in Table II, Column 3, lines 103 to 106 and/or 468 to 471, e.g. having the sequence of a polypeptide encoded by a nucleic acid molecule comprising the sequence indicated in Table I, Column 5 or 7, lines 103 to 106 and/or 468 to 471.
In one embodiment, the homolog of any one of the polypeptides indicated in Table II, column 3, line 103 is a homolog having the same or a similar activity. In particular an increase of activity confers an increase in the content of the respective fine chemical in the organisms preferably beta-cryptoxanthin. In one embodiment, the homologue is a homolog with a sequence as indicated in Table I or II, column 7, lines 103, resp.. In one embodiment, the homologue of one of the polypeptides indicated in Table II, column 3, line 103 is derived from an eukaryotic. In one embodiment, the homologue is derived from Fungi. In one embodiment, the homologue of a polypeptide indicated in Table II, column 3, line 103 is derived from Ascomyceta. In one embodiment, the homologue of a polypeptide indicated in Table II, column 3, line 103 is derived from Saccharomycotina. In one embodiment, the homologue of a polypeptide indicated in Table II, column 3, line 103 is derived from Saccharomycetes. In one embodiment, the homolog of a polypeptide indicated in Table II, column 3, line 103 is a homologue being derived from Saccharomycetales. In one embodiment, the homologue of a polypeptide indicated in Table II, column 3, line 103 is a homologue having the same or a similar activity being derived from Saccharomycetaceae. In one embodiment, the homologue of a polypeptide indicated in Table II, column 3, line 103 is a homologue having the same or a similar activity being derived from Saccharomycetes.
In one embodiment, the homolog of the any one of the polypeptides indicated in Table II, column 3, lines 104 to 106 or 468 to 471 is a homolog having the same or a similar activity. In particular an increase of activity confers an increase in the content of the respective fine chemical in the organisms preferably cryptoxanthin. In one embodiment, the homolog is a homolog with a sequence as indicated in Table I or II, column 7, lines 104 to 106 or 468 to 471, resp.. In one embodiment, the homolog of one of the polypeptides indicated in Table II, column 3, lines 104 to 106 or 468 to 471 is derived from an bacteria.. In one embodiment, the homolog of a polypeptide indicated in Table II, column 3, lines 104 to 106 or 468 to 471 is derived from Proteobacteria. In one embodiment, the homolog of a polypeptide indicated in Table II, column 3, lines 104 to 106 or 468 to 471 is a homolog having the same or a similar activity being derived from Gammaproteobacteria. In one embodiment, the homolog of a polypeptide indicated in Table II, column 3, lines 104 to 106 or 468 to 471 is derived from Enterobacteriales. In one embodiment, the homolog of a polypeptide indicated in Table II, column 3, lines 104 to 106 or 468 to 471 is a homolog being derived from Enterobacteriaceae. In one embodiment, the homolog of a polypeptide indicated in Table II, column 3, lines 104 to 106 or 468 to 471 is a homolog having the same or a similar activity, in particular an increase of activity confers an increase in the content of the beta-cryptoxanthin in the organisms or part thereof being derived from Escherichia.
In one embodiment, the homolog of the any one of the polypeptides indicated in Table II, column 3, line 107 is a homolog having the same or a similar activity. In particular an increase of activity confers an increase in the content of the respective fine chemical in the organisms preferably zeaxanthin. In one embodiment, the homolog is a homolog with a sequence as indicated in Table I or II, column 7, line 107, resp.. In one embodiment, the homolog of one of the polypeptides indicated in Table II, column 3, line 107 is derived from an eukaryotic. In one embodiment, the homolog is derived from Fungi. In one embodiment, the homolog of a polypeptide indicated in Table II, column 3, line 107 is derived from Ascomyceta. In one embodiment, the homolog of a polypeptide indicated in Table II, column 3, line 107lines 65 to 67 is derived from Saccharomycotina. In one embodiment, the homolog of a polypeptide indicated in Table II, column 3, lines line 107 is derived from Saccharomycetes. In one embodiment, the homolog of a polypeptide indicated in Table II, column 3, line 107 is a homolog being derived from Saccharomycetales. In one embodiment, the homolog of a polypeptide indicated in Table II, column 3, line 107 is a homolog having the same or a similar activity being derived from Saccharomycetaceae. In one embodiment, the homolog of a polypeptide indicated in Table II, column 3, line 107 is a homolog having the same or a similar activity being derived from Saccharomycetes.
In one embodiment, the homolog of the any one of the polypeptides indicated in Table II, column 3, line 108 is a homolog having the same or a similar activity. In particular an increase of activity confers an increase in the content of the respective fine chemical in the organisms preferably zeaxanthin. In one embodiment, the homolog is a homolog with a sequence as indicated in Table I or II, column 7, line 108, resp.. In one embodiment, the homolog of one of the polypeptides indicated in Table II, column 3, line 108 is derived from an bacteria.. In one embodiment, the homolog of a polypeptide indicated in Table II, column 3, line 108 is derived from Proteobacteria. In one embodiment, the homolog of a polypeptide indicated in Table II, column 3, line 108 is a homolog having the same or a similar activity being derived from Gammaproteobacteria. In one embodiment, the homolog of a polypeptide indicated in Table II, column 3, line 108 is derived from Enterobacteriales. In one embodiment, the homolog of a polypeptide indicated in Table II, column 3, line 108 is a homolog being derived from Enterobacteriaceae. In one embodiment, the homolog of a polypeptide indicated in Table II, column 3, line 108 is a homolog having the same or a similar activity, in particular an increase of activity confers an increase in the content of the zeaxanthin in the organisms or part thereof being derived from Escherichia.

**[0023.1.0.10]** Homologs of the polypeptides indicated in Table II, column 3, lines 103 to 108 or 468 to 471 may be the polypeptides encoded by the nucleic acid molecules indicated in Table I, column 7, lines 103 to 108 or 468 to 471 or may be the polypeptides indicated in Table II, column 7, lines 103 to 108 or 468 to 471. Homologs of the polypeptides indicated in Table II, column 3, lines 103 to 108 or 468 to 471 may be the polypeptides encoded by the nucleic acid molecules indicated in Table I, column 7, lines 103 to 108 or 468 to 471 or may be the polypeptides indicated in Table II, column 7, lines 103 to 108 or 468 to 471.
Homologs of the polypeptides indicated in Table II, column 3, lines 103 to 106 and/or 468 to 471 may be the polypeptides encoded by the nucleic acid molecules indicated in Table I, column 7, lines 103 to 106 and/or 468 to 471, respectively or may be the polypeptides indicated in Table II, column 7, lines 103 to 106 and/or 468 to 471, having a cryptoxanthin content and/or amount increasing activity. Homologs of the polypeptides indicated in Table II, column 3, lines 103 to 106 and/or 468 to 471 may be the polypeptides encoded by the nucleic acid molecules indicated in Table I, column 7, lines 103 to 106 and/or 468 to 471 or may be the polypeptides indicated in Table II, column 7, lines 103 to 106 and/or 468 to 471 having a cryptoxanthin content and/or amount increasing activity.
Homologs of the polypeptides indicated in Table II, column 3, lines 107 and/or 108 may be the polypeptides encoded by the nucleic acid molecules indicated in Table I, column 7, lines 107 and/or 108, respectively or may be the polypeptides indicated in Table II, column 7, lines 107 and/or 108, having a zeaxanthin content and/or amount increasing activity. Homologs of the polypeptides indicated in Table II, column 3, lines 107 and/or 108 may be the polypeptides encoded by the nucleic acid molecules indicated in Table I, column 7, lines 107 and/or 108 or may be the polypeptides indicated in Table II, column 7, lines 107 and/or 108 having a zeaxanthin content and/or amount increasing activity.

**[0024.0.0.10]** see **[0024.0.0.0]**

**[0025.0.10.10]**In accordance with the invention, a protein or polypeptide has the "activity of an protein of the invention", e.g. the activity of a protein indicated in Table II, column 3, lines 103 to 106 and/or 468 to 471 or lines 107 and/or 108 if its *de novo* activity, or its increased expression directly or indirectly leads to an increased xanthophylls level, in particular to a increased beta-cryptoxanthin level or zeaxanthin level, resp., in the organism or a part thereof, preferably in a cell of said organism. In a preferred embodiment, the protein or polypeptide has the above-mentioned additional activities of a protein indicated in Table II, column 3, lines 103 to 106 and/or 468 to 471 or lines 107 and/or 108. Throughout the specification the activity or preferably the biological activity of such a protein or polypeptide or an nucleic acid molecule or sequence encoding such protein or polypeptide is identical or similar if it still has the biological or enzymatic activity of any one of the proteins indicated in Table II, column 3, lines 103 to 106 and/or 468 to 471 or lines 107 and/or 108, or which has at least 10% of the original enzymatic activity, preferably 20%, particularly preferably 30%, most particularly preferably 40% in comparison to any one of the proteins indicated in Table II, column 3, line 103 or line 107 of Saccharomyces cerevisiae and/or any one of the proteins indicated in Table II, column 3, lines 104 to 106 or line 108 and/or lines 468 to 471 of E. coli K12.
In one embodiment, the polypeptide of the invention confers said activity, e.g. the increase of the respective fine chemical in an organism or a part thereof, if it is derived from an organism, which is evolutionary close to the organism indicated in Table I, column 4 and is expressed in an organism, which is evolutionary distant to the origin organism. For example origin and expressing organism are derived from different families, orders, classes or phylums whereas origin and the organism indicated in Table I, column 4 are derived from the same families, orders, classes or phylums.

**[0025.1.0.10] see [0025.1.0.0]**

**[0025.2.0.10] see [0025.2.0.0]**

**[0026.0.0.10]** to **[0033.0.0.10]: see [0026.0.0.0]** to **[0033.0.0.0]**

**[0034.0.10.10]**Preferably, the reference, control or wild type differs form the subject of the present invention only in the cellular activity of the polypeptide of the invention or the polypeptide used in the method of the invention, e.g. as result of an increase in the level of the nucleic acid molecule of the present invention or an increase of the specific activity of the polypeptide of the invention or the polypeptide used in the method of the invention. E.g., it differs by or in the expression level or activity of an protein having the activity of a protein as indicated in Table II, column 3, lines 103 to 106 and/or 468 to 471 or lines 107 and/or 108 or being encoded by a nucleic acid molecule indicated in Table I, column 5, lines 103 to 106 and/or 468 to 471 or lines 107 and/or 108 or its homologs, e.g. as indicated in Table I, column 7, lines 103 to 106 and/or 468 to 471 or lines 107 and/or 108, its biochemical or genetic causes. It therefore shows the increased amount of the respective fine chemical.

**[0035.0.0.10]** to **[0044.0.0.10]: see [0035.0.0.0]** to **[0044.0.0.0]**

**[0045.0.10.10]** In one embodiment, the activity of the Escherichia coli K12 protein b0050 or its homologs e.g. a conserved protein potentially involved in protein-protein interaction, in particular of a conserved protein potentially involved in protein-protein interaction of the superfamily of apaG proteins, e.g. as indicated in Table I, columns 5 or 7, line 468, is increased conferring preferably an increase of the respective fine chemical, preferably of beta-cryptoxanthin of between 42% and 84% or more.
In one embodiment, the activity of the Escherichia coli K12 protein b0851 or its homologs, e.g. oxygen insensitive NADPH nitroreductase, in particular of a oxygen insensitive NADPH nitroreductase of the superfamily of NADPH-flavin oxidoreductase homologs, e.g. as indicated in Table I, columns 5 or 7, line 469, is increased conferring preferably an increase of the respective fine chemical, preferably of beta-cryptoxanthin of between 57% and 76% or more.
In one embodiment, the activity of the Escherichia coli K12 protein b2211 or its homologs, e.g. an ATP-binding transport protein of the ABC superfamily, in particular of an ATP-binding transport proteins of the ABC superfamily of the superfamily of unassigned ATP-binding cassette proteins, e.g. as indicated in Table I, columns 5 or 7, line 470, is increased conferring preferably an increase of the respective fine chemical, preferably of beta-cryptoxanthin of between 5% and 26% or more.
In one embodiment, the activity of the Escherichia coli K12 protein b3926 or its homologs, e.g. a glycerol kinase, in particular of a glycerol kinase of the superfamily of xylulokinases, e.g. as indicated in Table I, columns 5 or 7, line 471, is increased conferring preferably an increase of the respective fine chemical, preferably of beta-cryptoxanthin of around 33% or more.
In one embodiment, the activity of the Escherichia coli K12 protein b0986 or its homologs e.g. a lipoprotein, in particular of a lipoprotein of the superfamily of the hypothetical protein b1706, e.g. as indicated in Table I, columns 5 or 7, line 104, is increased, conferring preferably, the increase of the respective fine chemical, preferably of beta-cryptoxanthin between 33% and 40% or more.
Thus, in one embodiment, the activity of the Escherichia coli K12 protein b3684 or its homologs is increased. Accordingly, in one embodiment, the process of the present invention comprises the use of a protein having a transcriptional control activity, a regulation of C-compound and carbohydrate utilization activity, a DNA binding activity, a transcriptional repressor activity, a regulation of lipid, fatty-acid and/or isoprenoid metabolism activity, and/or a regulation of C-compound and carbohydrate utilization activity, e.g. of a transcriptional regulator, in particular of a transcriptional regulator of the GntR family, e.g. as indicated in Table I, columns 5 or 7, line 105, is increased, preferably,conferring the increase of the respective fine chemical, preferably of beta-cryptoxanthin between 35% and 67% or more .
In one embodiment, the activity of the Escherichia coli K12 protein b4401 or its homologs e.g. a protein of a transcriptional control activity, a unspecified signal transduction activity, a two-component signal transduction system (e.g. response regulator component activity, a transcriptional activator activity, a regulation of respiration activity, an aerobic respiration activity, and/or an anaerobic respiration activity, e.g. of a response regulator, in particular of a response regulator of the OmpR family, e.g. as indicated in Table I, columns 5 or 7, line 106, is increased, preferably, conferring the increase of the respective fine chemical, preferably of beta-cryptoxanthin between 48% and 60% or more .
In one embodiment, the activity of the Saccharomyces cerevisiae protein YCL040W or its homologs, e.g. a protein having a C-compound and carbohydrate utilization activity, a C-compound or carbohydrate transport activity, a activity in the glycolysis and/or a activity in the gluconeogenesis, and/or a cellular import activity, in particular of a hexokinase, preferably of a glucinase, e.g. as indicated in Table I, columns 5 or 7, line 103, is increased, preferably, conferring an increase of the respective fine chemical, preferably of beta-cryptoxanthin between 36% and 51% or more.
In one embodiment, the activity of the Escherichia coli K12 protein b2699 or its homologs e.g. a DNA recombination and DNA repair activity, a pheromone response activity, a mating-type determination activity, a sex-specific protein activity, a nucleotide binding activity and/or a protease and nuclease activity, in particular a DNA strand exchange and recombination protein with protease and nuclease activity, in particular of the superfamily of the recombination protein recA, e.g. as indicated in Table I, columns 5 or 7, line 108, is increased, preferably, conferring the increase of the respective fine chemical, preferably of zeaxanthin between 25% and 48% or more.
In one embodiment, the activity of the Saccharomyces cerevisiae protein YHR055C or its homologs, e.g. a protein having a detoxification activity, particular of a metallothionein, preferably of copper binding metallothionein or its homolog, e.g. as indicated in Table I, columns 5 or 7, line 107, is increased, preferably, conferring an increase of the respective fine chemical, preferably of zeaxanthin between 05% and 38% or more.

**[0046.0.10.10]** In one embodiment, the activity of the Escherichia coli K12 protein b0050 or its homologs e.g. a conserved protein potentially involved in protein-protein interaction, in particular of a conserved protein potentially involved in protein-protein interaction of the superfamily of apaG proteins, e.g. as indicated in Table I, columns 5 or 7, line 468, is increased conferring preferably an increase of the respective fine chemical and of further carotenoids, preferably xanthophylls.
In one embodiment, the activity of the Escherichia coli K12 protein b0851 or its homologs, e.g. oxygen insensitive NADPH nitroreductase, in particular of a oxygen insensitive NADPH nitroreductase of the superfamily of NADPH-flavin oxidoreductase homologs, e.g. as indicated in Table I, columns 5 or 7, line 469, is increased conferring preferably an increase of the respective fine chemical and of further carotenoids, preferably xanthophylls.
In one embodiment, the activity of the Escherichia coli K12 protein b2211 or its homologs, e.g. an ATP-binding transport protein of the ABC superfamily, in particular of an ATP-binding transport proteins of the ABC superfamily of the superfamily of unassigned ATP-binding cassette proteins, e.g. as indicated in Table I, columns 5 or 7, line 470, is increased conferring preferably an increase of the respective fine chemical and of further carotenoids, preferably xanthophylls.
In one embodiment, the activity of the Escherichia coli K12 protein b3926 or its homologs, e.g. a glycerol kinase, in particular of a glycerol kinase of the superfamily of xylulokinases, e.g. as indicated in Table I, columns 5 or 7, line 471, is increased conferring preferably an increase of the respective fine chemical and of further carotenoids, preferably xanthophylls.
In one embodiment, the activity of the Escherichia coli K12 protein b0986 or its homologs e.g. lipoprotein, in particular of a lipoprotein of the superfamily of the hypothetical protein b1706, e.g. as indicated in Table I, columns 5 or 7, line 104, is increased; conferring preferably an increase of the respective fine chemical and of further carotenoids, preferably xanthophylls.
In one embodiment, the activity of the Escherichia coli K12 protein b3684 or its homologs, meaning e.g. a transcriptional regulator of the GntR family , is increased; preferably an increase of the respective fine chemical and of further carotenoids, preferably xanthophylls is conferred. Accordingly, in one embodiment, the process of the present invention comprises the use of a protein having a transcriptional control activity, a regulation of C-compound and carbohydrate utilization activity, a DNA binding activity, a transcriptional repressor activity, a regulation of lipid, fatty-acid and/or isoprenoid metabolism activity, and/or a regulation of C-compound and carbohydrate utilization activity, e.g. the activity of a transcriptional regulator, in particular of a transcriptional regulator of the GntR family, e.g. as indicated in Table I, columns 5 or 7, line 105, preferably conferring an increase of the respective fine chemical and of further carotenoids, preferably xanthophylls.
In one embodiment, the activity of the Escherichia coli K12 protein b4401 or its homologs e.g. a protein of a transcriptional control activity, a unspecified signal transduction activity, a two-component signal transduction system (e.g. response regulator component activity, a transcriptional activator activity, a regulation of respiration activity, an aerobic respiration activity, and/or an anaerobic respiration activity, e.g. of a response regulator, in particular of a response regulator of the OmpR family, e.g. as indicated in Table I, columns 5 or 7, line 106, is increased, preferably conferring an increase of the respective fine chemical and of further carotenoids, preferably xanthophylls.
In one embodiment, the activity of the Saccharomyces cerevisiae protein YCL040W or its homologs, e.g. a protein having a C-compound and carbohydrate utilization activity, a C-compound or carbohydrate transport activity, a activity in the glycolysis and/or a activity in the gluconeogenesis, and/or a cellular import activity, in particular of a hexokinase, preferably of a glucinase, e.g. as indicated in Table I, columns 5 or 7, line 103, is increased, preferably conferring an increase of the respective fine chemical and of further carotenoids, preferably xanthophylls.
In one embodiment, in case the activity of the Escherichia coli K12 protein b2699 or its homologs e.g. a DNA recombination and DNA repair activity, a pheromone response activity, a mating-type determination activity, a sex-specific protein activity, a nucleotide binding activity and/or a protease and nuclease activity, in particular a DNA strand exchange and recombination protein with protease and nuclease activity, in particular of the superfamily of the recombination protein recA, e.g. as indicated in Table I, columns 5 or 7, line 108, is increased, preferably conferring an increase of the respective fine chemical and of further carotenoids, preferably xanthophylls.
In one embodiment, in case the activity of the Saccharomyces cerevisiae protein YHR055C or its homologs, e.g. a protein having a detoxification activity, particular of a metallothionein, preferably of copper binding metallothionein or its homolog, e.g. as indicated in Table I, columns 5 or 7, line 107, is increased, preferably conferring an increase of the respective fine chemical and of further carotenoids, preferably xanthophylls is conferred.

**[0047.0.0.10]** to **[0048.0.0.10]: see [0047.0.0.0]** to **[0048.0.0.0]**

**[0049.0.10.10]**A protein having an activity conferring an increase in the amount or level of the respective fine chemical cryptoxanthin preferably has the structure of the polypeptide described herein. In a particular embodiment, the polypeptides used in the process of the present invention or the polypeptide of the present invention comprises the sequence of a consensus sequence as indicated in Table IV, column 7, lines 103 to 107 or and/or 468 to 471 or of a polypeptide as indicated in Table II, columns 5 or 7, lines 103 to 106 and/or 468 to 471, or of a functional homologue thereof as described herein, or of a polypeptide encoded by the nucleic acid molecule characterized herein or the nucleic acid molecule according to the invention, for example by a nucleic acid molecule as indicated in Table I, columns 5 or 7, lines 103 to 106 and/or 468 to 471 or its herein described functional homologues and has the herein mentioned activity conferring an increase in the beta-cryptoxanthin level.
A protein having an activity conferring an increase in the amount or level of the zeaxanthin preferably has the structure of the polypeptide described herein. In a particular embodiment, the polypeptides used in the process of the present invention or the polypeptide of the present invention comprises the sequence of a consensus sequence as indicated in Table IV, column 7, lines 107 and 108 or of a polypeptide as indicated in Table II, columns 5 or 7, line 107 or 108 or of a functional homologue thereof as described herein, or of a polypeptide encoded by the nucleic acid molecule characterized herein or the nucleic acid molecule according to the invention, for example by a nucleic acid molecule as indicated in Table I, columns 5 or 7, lines 107 or 108 or its herein described functional homologues and has the herein mentioned activity confering an increase in the zeaxanthin level.

**[0050.0.10.10]**For the purposes of the present invention, the term "the respective fine chemical" also encompass the corresponding salts, such as, for example, the potassium or sodium salts of cryptoxanthin or zeaxanthin, resp., or their ester, or glucoside thereof, e.g. the diglucoside thereof.

**[0051.0.10.10]**Owing to the biological activity of the proteins which are used in the process according to the invention and which are encoded by nucleic acid molecules according to the invention, it is possible to produce compositions comprising the respective fine chemical, i.e. an increased amount of the free chemical free or bound, e.g. compositions comprising xanthophylls, in particular cryptoxanthin or zeaxanthin. Depending on the choice of the organism used for the process according to the present invention, for example a microorganism or a plant, compositions or mixtures of various xanthophylls, in particular beta-cryptoxanthin or zeaxanthin.can be produced.

**[0052.0.0.10]** see **[0052.0.0.0]**

**[0053.0.10.10]**In one embodiment, the process of the present invention comprises one or more of the following steps
a) stabilizing a protein conferring the increased expression of a protein encoded by the nucleic acid molecule of the invention or of the polypeptide of the invention or the polypeptide used in the method of the invention, e.g. of a polypeptide having an activity of a protein as indicated in Table II, column 3, lines 103 to 106 and/or 468 to 471 or line 107 and/or 108 or its homologs, e.g. as indicated in Table II, columns 5 or 7, lines 103 to 106 and/or 468 to 471 or line 107 and/or 108, activity having herein-mentioned the respective fine chemical increasing activity;
b) stabilizing a mRNA conferring the increased expression of a protein encoded by the nucleic acid molecule of the invention or the nucleic acid molecule used in the method of the invention, e.g. of a polypeptide having an activity of a protein as indicated in Table II, column 3, lines 103 to 106 and/or 468 to 471 or line 107 and/or 108 or its homologs activity, e.g. as indicated in Table II, columns 5 or 7, lines 103 to 106 and/or 468 to 471 or line 107 and/or 108, or of a mRNA encoding the polypeptide of the present invention having herein-mentioned the respective fine chemical increasing activity;
c) increasing the specific activity of a protein conferring the increased expression of a protein encoded by the nucleic acid molecule of the invention or of the polypeptide of the present invention or the nucleic acid molecule or polypeptide used in the method of the invention having herein-mentioned the respective fine chemical increasing activity, e.g. of a polypeptide having an activity of a protein as indicated in Table II, column 3, lines 103 to 106 and/or 468 to 471 or line 107 and/or 108 or its homologs activity, e.g. as indicated in Table II, columns 5 or 7, lines 103 to 106 and/or 468 to 471 or line 107 and/or 108, or decreasing the inhibitory regulation of the polypeptide of the invention or the polypeptide used in the method of the invention;
d) generating or increasing the expression of an endogenous or artificial transcription factor mediating the expression of a protein conferring the increased expression of a protein encoded by the nucleic acid molecule of the invention or the nucleic acid molecule used in the method of the invention or of the polypeptide of the invention or the polypeptide used in the method of the invention having herein-mentioned the respective fine chemical increasing activity, e.g. of a polypeptide having an activity of a protein as indicated in Table II, column 3, lines 103 to 106 and/or 468 to 471 or line 107 and/or 108 or its homologs activity, e.g. as indicated in Table II, columns 5 or 7, lines 103 to 106 and/or 468 to 471 or line 107 and/or 108;
e) stimulating activity of a protein conferring the increased expression of a protein encoded by the nucleic acid molecule of the present invention or a polypeptide of the present invention having herein-mentioned the respective fine chemical increasing activity, e.g. of a polypeptide having an activity of a protein as indicated in Table II, column 3, lines 103 to 106 and/or 468 to 471 or line 107 and/or 108 or its homologs activity, e.g. as indicated in Table II, columns 5 or 7, lines 103 to 106 and/or 468 to 471 or line 107 and/or 108, by adding one or more exogenous inducing factors to the organism or parts thereof;
f) expressing a transgenic gene encoding a protein conferring the increased expression of a polypeptide encoded by the nucleic acid molecule of the present invention or a polypeptide of the present invention, having herein-mentioned the respective fine chemical increasing activity, e.g. of a polypeptide having an activity of a protein as indicated in Table II, column 3, lines 103 to 106 and/or 468 to 471 or line 107 and/or 108 or its homologs activity, e.g. as indicated in Table II, columns 5 or 7, lines 103 to 106 and/or 468 to 471 or line 107 and/or 108, and/or
g) increasing the copy number of a gene conferring the increased expression of a nucleic acid molecule encoding a polypeptide encoded by the nucleic acid molecule of the invention or the nucleic acid molecule used in the method of the invention or the polypeptide of the invention or the polypeptide used in the method of the invention having herein-mentioned the respective fine chemical increasing activity, e.g. of a polypeptide having an activity of a protein as indicated in Table II, column 3, lines 103 to 106 and/or 468 to 471 or line 107 and/or 108 or its homologs, e.g. as indicated in Table II, columns 5 or 7, lines 103 to 106 and/or 468 to 471 or line 107 and/or 108, activity.
h) Increasing the expression of the endogenous gene encoding the polypeptide of the invention or the polypeptide used in the method of the invention, e.g. a polypeptide having an activity of a protein as indicated in Table II, column 3, lines 103 to 106 and/or 468 to 471 or line 107 and/or 108 or its homologs activity, e.g. as indicated in Table II, columns 5 or 7, lines 103 to 106 and/or 468 to 471 or line 107 and/or 108, by adding positive expression or removing negative expression elements, e.g. homologous recombination can be used to either introduce positive regulatory elements like for plants the 35S enhancer into the promoter or to remove repressor elements form regulatory regions. Further gene conversion methods can be used to disrupt repressor elements or to enhance to activity of positive elements. Positive elements can be randomly introduced in plants by T-DNA or transposon mutagenesis and lines can be identified in which the positive elements have be integrated near to a gene of the invention, the expression of which is thereby enhanced;
i) Modulating growth conditions of an organism in such a manner, that the expression or activity of the gene encoding the protein of the invention or the protein itself is enhanced for example microorganisms or plants can be grown for example under a higher temperature regime leading to an enhanced expression of heat shock proteins, which can lead an enhanced respective fine chemical production and/or
j) selecting of organisms with especially high activity of the proteins of the invention from natural or from mutagenized resources and breeding them into the target organisms, e.g. the elite crops.

**[0054.0.10.10]**Preferably, said mRNA is the nucleic acid molecule of the present invention and/or the protein conferring the increased expression of a protein encoded by the nucleic acid molecule of the present invention or the polypeptide having the herein mentioned activity is the polypeptide of the present invention, e.g. conferring the increase of the respective fine chemical after increasing the expression or activity of the encoded polypeptide or having the activity of a polypeptide having an activity of a protein as indicated in Table II, columns 5 or 7, lines 103 to 106 and/or 468 to 471 or line 107 and/or 108, resp., or its homologs activity, e.g. as indicated in Table II, columns 5 or 7, lines 103 to 106 and/or 468 to 471 or line 107 and/or 108, resp.,.

**[0055.0.0.10] to [0067.0.0.10]: see [0055.0.0.0] to [0067.0.0.0]**

**[0068.0.10.10]**The mutation is introduced in such a way that the production of the xanthophylls, in particular of beta-cryptoxanthin or zeaxanthin is not adversely affected.

**[0069.0.10.10]see [0069.0.0.0]**

**[0070.0.10.10]**Owing to the introduction of a gene or a plurality of genes conferring the expression of the nucleic acid molecule of the invention or the nucleic acid molecule used in the method of the invention or the polypeptide of the invention or the polypeptide used in the method of the invention, for example the nucleic acid construct mentioned below, or encoding a protein of the invention into an organism alone or in combination with other genes, it is possible not only to increase the biosynthetic flux towards the end product, but also to increase, modify or create de novo an advantageous, preferably novel metabolites composition in the organism, e.g. an advantageous composition of carotenoids, in particular xanthophylls, e.g. comprising a higher content of (from a viewpoint of nutrional physiology limited) carotenoids, in particular xanthophylls, like beta-cryptoxanthin or zeaxanthin.

**[0071.0.0.10]** see **[0071.0.0.0]**

**[0072.0.10.10]** By influencing the metabolism thus, it is possible to produce, in the process according to the invention, further advantageous compounds. Examples of such compounds are, in addition to beta-cryptoxanthin or zeaxanthin further carotenoids, e.g. carotenes or xanthophylls, in particular ketocarentoids, or hydrocarotenoids, e.g. Lutein, lycopene, alpha-carotene, or beta-carentene, or compounds for which beta-cryptoxanthin or zeaxanthin are precursor compounds, in particular astaxanthin.

**[0073.0.10.10]** Accordingly, in one embodiment, the process according to the invention relates to a process, which comprises:
(f) providing a non-human organism, preferably a microorganism, a non-human animal, a plant or animal cell, a plant or animal tissue or a plant;
(g) increasing an activity of a polypeptide of the invention or the polypeptide used in the method of the invention or a homolog thereof, e.g. as indicated in Table II, columns 5 or 7, lines 103 to 106 and/or 468 to 471 or line 107 and/or 108, or of a polypeptide being encoded by the nucleic acid molecule of the present invention and described below, e.g. conferring an increase of the respective fine chemical in an organism, preferably in a microorganism, a non-human animal, a plant or animal cell, a plant or animal tissue or a plant,
(h) growing an organism, preferably a microorganism, a non-human animal, a plant or animal cell, a plant or animal tissue or a plant under conditions which permit the production of the respective fine chemical in the organism, preferably the microorganism, the plant cell, the plant tissue or the plant; and
(i) if desired, recovering, optionally isolating, the free and/or bound the respective fine chemical and, optionally further free and/or bound carotenoids, in particular xanthophylls, e.g. astaxanthin, synthesized by the organism, the microorganism, the non-human animal, the plant or animal cell, the plant or animal tissue or the plant.

**[0074.0.10.10]** The organism, in particular the microorganism, non-human animal, the plant or animal cell, the plant or animal tissue or the plant is advantageously grown in such a way that it is not only possible to recover, if desired isolate the free or bound the respective fine chemical or the free and bound the respective fine chemical but as option it is also possible to produce, recover and, if desired isolate, other free or/and bound carotenoids, in particular xanthophylls, e.g. astaxanthin.

**[0075.0.0.10]** to **[0077.0.0.10]: see [0075.0.0.0]** to **[0077.0.0.0]**

**[0078.0.10.10]**The organism such as microorganisms or plants or the recovered, and if desired isolated, the respective fine chemical can then be processed further directly into foodstuffs or animal feeds or for other applications, for example according to the disclosures made in US 6,380,442; US US6,329, 557, US 6,329,432, 6,316,012, US 6,309,883; US 6,291,533, US 6,291,294, US 6,287,615, US 6,262,284, US 6,262,284, US 6,261,622, US 6,261,598, US 6,235,315, US 6,224,876, US 6,221,417, US 6,221,412, US 6,218,436, US 6,207,409, US 6,319,872, US 6,132,790, US 6,124,113 US 6,110,478, US 6,093,348, US 6,087,152, or US 6,056,962. (Pharmaceutical and other compositions comprising zeaxanthin are e.g. described in: US 6,383,523, 6,368,621, US 6,362,221, US 6,348,200, US 6,316,012: Cosmetic or pharmaceutical composition comprising, in combination, a peroxidase and an anti-singlet oxygen agent, 6,296,880: Pharmaceutical compositions and methods for managing skin conditions, US 6,296,877: Stable, aqueous dispersions and stable, water-dispersible dry xanthophyll powder, their production and use, US 6,261,598: Carotenoid formulations, comprising a mixture of B-carotens, lycopene and lutein, US 6,248,378: Enhanced food products, US 6,248,374: Stabilized food additive. Processes for the isolation are described e.g. in US 6,380,442, US 6,362,221: Compositions containing natural lycopene and natural tocopherol, US 6,291,204: Fermentative carotenoid production, US 6,262,284: Process for extraction and purification of lutein, zeaxanthin and rare carotenoids from marigold flowers and plants, or US 6,224,876: Isolation and formulations of nutrient-rich carotenoids. The cited literatures describe some preferred embodiments. Said applications describe some advantageous embodiments without meant to be limiting. The fermentation broth, fermentation products, plants or plant products can be purified as described in above mentioned applications and other methods known to the person skilled in the art, e.g. as described in Methods in Enzymology: Carotenoids, Part A: Chemistry, Separation, Quantitation and Antioxidation, by John N Abelson or Part B, Metabolism, Genetics, and Biochemistry, or described herein below. Products of these different work-up procedures are xanthophylls, in particular zeaxanthin or beta-cryptoxanthin or xanthophylls, in particular zeaxanthin or cryptoxanthin comprising compositions which still comprise fermentation broth, plant particles and cell components in different amounts, advantageously in the range of from 0 to 99% by weight, preferably below 80% by weight, especially preferably between below 50% by weight.

**[0079.0.0.10]** to **[0084.0.0.10]: see [0079.0.0.0]** to **[0084.0.0.0]**

**[0084.1.0.10]** The invention also contemplates embodiments in which the β-carotene, or other carotenoid precursor compounds in the production of the respective fine chemical, is present in the flowers of the flowering plant chosen as the host (for example, marigolds). The invention also contemplates embodiments in which a host plant's flowers lack β-carotene or other carotenoid precursors, such as the vinca. In a plant of the latter type, the inserted DNA includes genes that code for carotenoid precursors (compounds that can be converted biologically into β-carotene) and a ketolase, as well as a hydroxylase, if otherwise absent.
In one embodiment, preferred flowering plants include, but are not limited to: Amaryllidaceae (Allium, Narcissus); Apocynaceae (Catharanthus); Asteraceae, alternatively Compositae (Aster, Calendula, Callistephus, Cichorium, Coreopsis, Dahlia, Dendranthema, Gazania, Gerbera, Helianthus, Helichrysum, Lactuca, Rudbeckia, Tagetes, Zinnia); Balsaminaceae (Impatiens); Begoniaceae (Begonia); Caryophyllaceae (Dianthus); Chenopodiaceae (Beta, Spinacia); Cucurbitaceae (Citrullus, Curcurbita, Cucumis); Cruciferae (Alyssum, Brassica, Erysimum, Matthiola, Raphanus); Gentinaceae (Eustoma); Geraniaceae (Pelargonium); Graminae, alternatively Poaceae, (Avena, Horedum, Oryza, Panicum, Pennisetum, Poa, Saccharum, Secale, Sorghum, Triticum, Zea); Euphorbiaceae (Poinsettia); Labiatae (Salvia); Leguminosae (Glycine, Lathyrus, Medicago, Phaseolus, Pisum); Liliaceae (Lilium); Lobeliaceae (Lobelia); Malvaceae (Abelmoschus, Gossypium, Malva); Plumbaginaceae (Limonium); Polemoniaceae (Phlox); Primulaceae (Cyclamen); Ranunculaceae (Aconitum, Anemone, Aquilegia, Caltha, Delphinium, Ranunculus); Rosaceae (Rosa); Rubiaceae (Pentas); Scrophulariaceae (Angelonia, Antirrhinum, Torenia); Solanaceae (Capsicum, Lycopersicon, Nicotiana, Petunia, Solanum); Umbelliferae (Apium, Daucus, Pastinaca); Verbenaceae (Verbena, Lantana); Violaceae (Viola).

**[0085.0.10.10]**With regard to the nucleic acid sequence as depicted a nucleic acid construct which contains a nucleic acid sequence mentioned herein or an organism (= transgenic organism) which is transformed with said nucleic acid sequence or said nucleic acid construct, "transgene" means all those constructs which have been brought about by genetic manipulation methods, preferably in which either
a) a nucleic acid sequence as indicated in Table I, columns 5 or 7, lines 103 to 106 and/or 468 to 471 or lines 107 and/or 108, or a derivative thereof, or
b) a genetic regulatory element, for example a promoter, which is functionally linked to the nucleic acid sequence as indicated in Table I, columns 5 or 7, lines 103 to 106 and/or 468 to 471 or lines 107 and/or 108, or a derivative thereof, or
c) (a) and (b)
is/are not present in its/their natural genetic environment or has/have been modified by means of genetic manipulation methods, it being possible for the modification to be, by way of example, a substitution, addition, deletion, inversion or insertion of one or more nucleotide. "Natural genetic environment" means the natural chromosomal locus in the organism of origin or the presence in a genomic library. In the case of a genomic library, the natural, genetic environment of the nucleic acid sequence is preferably at least partially still preserved. The environment flanks the nucleic acid sequence at least on one side and has a sequence length of at least 50 bp, preferably at least 500 bp, particularly preferably at least 1000 bp, very particularly preferably at least 5000 bp.

**[0086.0.0.10]** to **[0087.0.0.10]: see [0086.0.0.0]** to **[0087.0.0.0]**

**[0088.0.10.10]**In an advantageous embodiment of the invention, the organism takes the form of a plant whose content of the respective fine chemical is modified advantageously owing to the nucleic acid molecule of the present invention expressed. This is important for plant breeders since, for example, the nutritional value of plants for poultry is dependent on the abovementioned xanthophylls and the general amount of xanthohylls as energy source in feed. Further, this is also important for the production of cosmetic compostions since, for example, the antioxidant level of plant extraction is dependent on the amount of th abovementioned of xanthophylls and/or the amount of carotenoids as antioxidants.

**[0088.1.0.10]** see **[0088.1.0.0]**

**[0089.0.0.10]** to **[0090.0.0.10]: see [0089.0.0.0]** to **[0090.0.0.0]**

**[0091.0.10.10]**Thus, the content of plant components and preferably also further impurities is as low as possible, and the abovementioned xanthophylls, in particular the fine chemical, is/are obtained in as pure form as possible. In these applications, the content of plant components advantageously amounts to less than 10%, preferably 1%, more preferably 0.1%, very especially preferably 0.01 % or less.

**[0092.0.0.10]** to **[0094.0.0.10]: see [0092.0.0.0]** to **[0094.0.0.0]**

**[0095.0.10.10]** It may be advantageous to increase the pool of said carotenoids, in particular xanthophylls, preferably cryptoxanthin or zeaxanthin in the transgenic organisms by the process according to the invention in order to isolate high amounts of the essentially pure respective fine chemical.

**[0096.0.10.10]**In another preferred embodiment of the invention a combination of the increased expression of the nucleic acid sequence or the protein of the invention together with the transformation of a protein or polypeptid or a compound, which functions as a sink for the desired fine chemical, for example zeaxanthin or cryptoxanthin in the organism, is useful to increase the production of the respective fine chemical. It has been reported, that the inhibition of Lyopene production increases the amount of other xanthophylls in the cell. Further it may be, that the inhibition of enzymes using zeaxanthin or cryptoxanthin as substrate increases the amount of said chemicals in a cell. For example, in one embodiment, it can be advantageous to inhibit the production of astaxanthin, if a high amount of cryptoxanthin or zeaxanthin is desired.

**[0097.0.10.10]**Glucosides, in particular, dicglucosides of the zeaxanthin and beta-cryptoxanthin as well as other modification of zeaxanthin and cryptoxanthin are known to a person skilled in the art. In may also be advantageous to increase the content of the bound respective fine chemical, e.g. of modification of zeaxanthin and cryptoxanthin, in particular its glucosides, e.g. diglucosides.

**[0098.0.10.10]**In a preferred embodiment, the respective fine chemical is produced in accordance with the invention and, if desired, is isolated. The production of further carotenoids, e.g. carotenes or xanthophylls, in particular ketocarentoids or hydrocarotenoids, e.g. lutein, lycopene, alpha-carotene, or beta-carentene, or compounds for which the respective fine chemical is a biosynthesis precursor compounds, e.g. astaxanthin, or mixtures thereof or mixtures of other carotenoids, in particular of xanthophylls, by the process according to the invention is advantageous.

**[0099.0.10.10]**In the case of the fermentation of microorganisms, the abovementioned desired fine chemical may accumulate in the medium and/or the cells. If microorganisms are used in the process according to the invention, the fermentation broth can be processed after the cultivation. Depending on the requirement, all or some of the biomass can be removed from the fermentation broth by separation methods such as, for example, centrifugation, filtration, decanting or a combination of these methods, or else the biomass can be left in the fermentation broth. The fermentation broth can subsequently be reduced, or concentrated, with the aid of known methods such as, for example, rotary evaporator, thin-layer evaporator, falling film evaporator, by reverse osmosis or by nanofiltration. Afterwards advantageously further compounds for formulation can be added such as corn starch or silicates. This concentrated fermentation broth advantageously together with compounds for the formulation can subsequently be processed by lyophilization, spray drying, and spray granulation or by other methods. Preferably the respective fine chemical comprising compositions are isolated from the organisms, such as the microorganisms or plants or the culture medium in or on which the organisms have been grown, or from the organism and the culture medium, in the known manner, for example via extraction, distillation, crystallization, chromatography or a combination of these methods. These purification methods can be used alone or in combination with the aforementioned methods such as the separation and/or concentration methods.

**[0100.0.10.10]**Transgenic plants which comprise the carotenoids such as said xanthophylls, e.g. cryptoxanthin or zeaxanthin (or astaxanthin as it is synthesized from cryptoxanthin or zeaxanthin) synthesized in the process according to the invention can advantageously be marketed directly without there being any need for the carotenoids synthesized to be isolated. Plants for the process according to the invention are listed as meaning intact plants and all plant parts, plant organs or plant parts such as leaf, stem, seeds, root, tubers, anthers, fibers, root hairs, stalks, embryos, calli, cotelydons, petioles, harvested material, plant tissue, reproductive tissue and cell cultures which are derived from the actual transgenic plant and/or can be used for bringing about the transgenic plant. In this context, the seed comprises all parts of the seed such as the seed coats, epidermal cells, seed cells, endosperm or embryonic tissue.
However, the respective fine chemical produced in the process according to the invention can also be isolated from the organisms, advantageously plants, (in the form of their oils, fats, lipids, as extracts, e.g. ether, alcohol, or other organic solvents or water containing extract and/or free xanthophylls. The respective fine chemical produced by this process can be obtained by harvesting the organisms, either from the crop in which they grow, or from the field. This can be done via pressing or extraction of the plant parts. To increase the efficiency of extraction it is beneficial to clean, to temper and if necessary to hull and to flake the plant material..E.g., oils, fats, and/or lipids comprising xanthophylls can be obtained by what is known as cold beating or cold pressing without applying heat. To allow for greater ease of disruption of the plant parts, specifically the seeds, they can previously be comminuted, steamed or roasted. Seeds, which have been pretreated in this manner can subsequently be pressed or extracted with solvents such as warm hexane. The solvent is subsequently removed. In the case of microorganisms, the latter are, after harvesting, for example extracted directly without further processing steps or else, after disruption, extracted via various methods with which the skilled worker is familiar. Thereafter, the resulting products can be processed further, i.e. degummed and/or refined. In this process, substances such as the plant mucilages and suspended matter canb be first removed. What is known as desliming can be affected enzymatically or, for example, chemico-physically by addition of acid such as phosphoric acid.
Because carotenoids in microorganisms are localized intracellular, their recovery essentials comes down to the isolation of the biomass. Well-established approaches for the harvesting of cells include filtration, centrifugation and coagulation/flocculation as described herein. Of the residual hydrocarbon, adsorbed on the cells, has to be removed. Solvent extraction or treatment with surfactants have been suggested for this purpose. However, it can be advantageous to avoid this treatment as it can result in cells devoid of most carotenoids.

**[0101.0.10.10]**The identity and purity of the compound(s) isolated can be determined by prior-art techniques. They encompass high-performance liquid chromatography (HPLC), gas chromatography (GC), spectroscopic methods, mass spectrometry (MS), staining methods, thin-layer chromatography, NIRS, enzyme assays or microbiological assays. These analytical methods are compiled in: Patek et al. (1994) Appl. Environ. Microbiol. 60:133-140; Malakhova et al. (1996) Biotekhnologiya 11 27-32; and Schmidt et al. (1998) Bioprocess Engineer. 19:67-70. Ulmann's Encyclopedia of Industrial Chemistry (1996) Bd. A27, VCH Weinheim, pp. 89-90, pp. 521-540, pp. 540-547, pp. 559-566, 575-581 and pp. 581-587; Michal, G (1999) Biochemical Pathways: An Atlas of Biochemistry and Molecular Biology, John Wiley and Sons; Fallon, A. et al. (1987) Applications of HPLC in Biochemistry in: Laboratory Techniques in Biochemistry and Molecular Biology, vol. 17.

**[0102.0.10.10]**Xanthophylls, in particular beta-cryptoxanthin or zeaxanthin can for example be detected advantageously via HPLC, LC or GC separation methods. The unambiguous detection for the presence of xanthophylls, in particular beta-cryptoxanthin or zeaxanthin containing products can be obtained by analyzing recombinant organisms using analytical standard methods: LC, LC-MS, MS or TLC). The material to be analyzed can be disrupted by sonication, grinding in a glass mill, liquid nitrogen and grinding, cooking, or via other applicable methods

**[0103.0.10.10]**In a preferred embodiment, the present invention relates to a process for the production of the respective fine chemical comprising or generating in an organism or a part thereof the expression of at least one nucleic acid molecule comprising a nucleic acid molecule selected from the group consisting of:
a) nucleic acid molecule encoding, preferably at least the mature form, of the polypeptide having a sequence as indicated in Table II, columns 5 or 7, lines 103 to 106 and/or 468 to 471 or lines 107 and/or 108, or a fragment thereof, which confers an increase in the amount of the respective fine chemical in an organism or a part thereof;
b) nucleic acid molecule comprising, preferably at least the mature form, of a nucleic acid molecule having a sequence as indicated in Table I, columns 5 or 7, lines 103 to 106 and/or 468 to 471 or lines 107 and/or 108,
c) nucleic acid molecule whose sequence can be deduced from a polypeptide sequence encoded by a nucleic acid molecule of (a) or (b) as result of the degeneracy of the genetic code and conferring an increase in the amount of the respective fine chemical in an organism or a part thereof;
d) nucleic acid molecule encoding a polypeptide which has at least 50% identity with the amino acid sequence of the polypeptide encoded by the nucleic acid molecule of (a) to (c) and conferring an increase in the amount of the respective fine chemical in an organism or a part thereof;
e) nucleic acid molecule which hybridizes with a nucleic acid molecule of (a) to (c) under under stringent hybridization conditions and conferring an increase in the amount of the respective fine chemical in an organism or a part thereof;
f) nucleic acid molecule encoding a polypeptide, the polypeptide being derived by substituting, deleting and/or adding one or more amino acids of the amino acid sequence of the polypeptide encoded by the nucleic acid molecules (a) to (d), preferably to (a) to (c) and conferring an increase in the amount of the respective fine chemical in an organism or a part thereof;
g) nucleic acid molecule encoding a fragment or an epitope of a polypeptide which is encoded by one of the nucleic acid molecules of (a) to (e), preferably to (a) to (c) and and conferring an increase in the amount of the respective fine chemical in an organism or a part thereof;
h) nucleic acid molecule comprising a nucleic acid molecule which is obtained by amplifying nucleic acid molecules from a cDNA library or a genomic library using the primers pairs having a sequence as indicated in Table III, column 7, lines 103 to 106 and/or 468 to 471 or lines 107 and/or 108 and conferring an increase in the amount of the respective fine chemical in an organism or a part thereof;
i) nucleic acid molecule encoding a polypeptide which is isolated, e.g. from an expression library, with the aid of monoclonal antibodies against a polypeptide encoded by one of the nucleic acid molecules of (a) to (h), preferably to (a) to (c), and and conferring an increase in the amount of the respective fine chemical in an organism or a part thereof;
j) nucleic acid molecule which encodes a polypeptide comprising the consensus sequence having a sequences as indicated in Table IV, column 7, lines 103 to 106 and/or 468 to 471 or lines 107 and/or 108 and conferring an increase in the amount of the respective fine chemical in an organism or a part thereof ;
k) nucleic acid molecule comprising one or more of the nucleic acid molecule encoding the amino acid sequence of a polypeptide encoding a domain of the polypeptide indicated in Table II, columns 5 or 7, lines 103 to 106 and/or 468 to 471 or lines 107 and/or 108, and conferring an increase in the amount of the respective fine chemical in an organism or a part thereof; and
l) nucleic acid molecule which is obtainable by screening a suitable library under stringent conditions with a probe comprising one of the sequences of the nucleic acid molecule of (a) to (k), preferably to (a) to (c), or with a fragment of at least 15 nt, preferably 20 nt, 30 nt, 50 nt, 100 nt, 200 nt or 500 nt of the nucleic acid molecule characterized in (a) to (k), preferably to (a) to (c), and conferring an increase in the amount of the respective fine chemical in an organism or a part thereof;
or which comprises a sequence which is complementary thereto.

**[0103.1.10.10]** In one embodiment, the nucleic acid molecule used in the process of the invention distinguishes over the sequence indicated in Table IA, columns 5 or 7, lines 103 to 106 and/or 468 to 471 or lines 107 and/or 108, by one or more nucleotides. In one embodiment, the nucleic acid molecule used in the process of the invention does not consist of the sequence indicated in Table I A, columns 5 or 7, lines 103 to 106 and/or 468 to 471 or lines 107 and/or 108: In one embodiment, the nucleic acid molecule used in the process of the invention is less than 100%, 99,999%, 99,99%, 99,9% or 99% identical to a sequence indicated in Table I A, columns 5 or 7, lines 103 to 106 and/or 468 to 471 or lines 107 and/or 108. In another embodiment, the nucleic acid molecule does not encode a polypeptide of a sequence indicated in Table II A, columns 5 or 7, lines 103 to 106 and/or 468 to 471 or lines 107 and/or 108.

**[0103.2.10.10.]** In one embodiment, the nucleic acid molecule used in the process of the invention distinguishes over the sequence indicated in Table I B, columns 5 or 7, lines 103 to 106 and/or 468 to 471 or lines 107 and/or 108, by one or more nucleotides. In one embodiment, the nucleic acid molecule used in the process of the invention does not consist of the sequence indicated in Table I B, columns 5 or 7, lines 103 to 106 and/or 468 to 471 or lines 107 and/or 108: In one embodiment, the nucleic acid molecule used in the process of the invention is less than 100%, 99,999%, 99,99%, 99,9% or 99% identical to a sequence indicated in Table I B, columns 5 or 7, lines 103 to 106 and/or 468 to 471 or lines 107 and/or 108. In another embodiment, the nucleic acid molecule does not encode a polypeptide of a sequence indicated in Table II B, columns 5 or 7, lines 103 to 106 and/or 468 to 471 or lines 107 and/or 108.

**[0104.0.10.10]**In one embodiment, the nucleic acid molecule of the invention or used in the process distinguishes over the sequence indicated in Table I, columns 5 or 7, lines 103 to 106 and/or 468 to 471 or lines 107 and/or 108 by one or more nucleotides. In one embodiment, the nucleic acid molecule used in the process or the nucleic acid used in the process of the invention of the invention does not consist of the sequence indicated in Table I, columns 5 or 7, lines 103 to 106 and/or 468 to 471 or lines 107 and/or 108 In one embodiment, the nucleic acid molecule of the present invention is less than 100%, 99,999%, 99,99%, 99,9% or 99% identical to the sequence indicated in Table I, columns 5 or 7, lines 103 to 106 and/or 468 to 471 or lines 107 and/or 108. In another embodiment, the nucleic acid molecule does not encode a polypeptide of a sequence indicated in Table II, columns 5 or 7, lines 103 to 106 and/or 468 to 471 or lines 107 and/or 108.

**[0105.0.0.10]** to **[0107.0.0.10]: see [0105.0.0.0]** to **[0107.0.0.0]**

**[0108.0.10.10]**Nucleic acid molecules with the sequence as indicated in Table I, columns 5 or 7, lines 103 to 106 and/or 468 to 471 or lines 107 and/or 108, nucleic acid molecules which are derived from an amino acid sequences as indicated in Table II, columns 5 or 7, lines 103 to 106 and/or 468 to 471 or lines 107 and/or 108 or from polypeptides comprising the consensus sequence as indicated in Table IV, column 7, lines 103 to 106 and/or 468 to 471 or lines 107 and/or 108, or their derivatives or homologues encoding polypeptides with the enzymatic or biological activity of a polypeptide as indicated in Table II, column 3, 5 or 7, lines 103 to 106 and/or 468 to 471 or lines 107 and/or 108, e.g. conferring the increase of the respective fine chemical, meaning beta-cryptoxanthin or zeaxanthin, resp., after increasing its expression or activity, are advantageously increased in the process according to the invention.

**[0109.0.10.10]**In one embodiment, said sequences are cloned into nucleic acid constructs, either individually or in combination. These nucleic acid constructs enable an optimal synthesis of the respective fine chemicals, in particular beta-cryptoxanthin or zeaxanthin, produced in the process according to the invention.

**[0110.0.10.10]**Nucleic acid molecules, which are advantageous for the process according to the invention and which encode polypeptides with an activity of a polypeptide of the invention or the polypeptide used in the method of the invention or used in the process of the invention, e.g. of a protein as indicated in Table II, column 5, lines103 to 106 and/or 468 to 471 or lines 107 and/or 108 or being encoded by a nucleic acid molecule indicated in Table I, column 5, lines 103 to 106 and/or 468 to 471 or lines 107 and/or 108 or of its homologs, e.g. as indicated in Table I or Table II, column 7, lines 103 to 106 and/or 468 to 471 or lines 107 and/or 1088, can be determined from generally accessible databases.

**[0111.0.0.10] see [0111.0.0.0]**

**[0112.0.10.10]**The nucleic acid molecules used in the process according to the invention take the form of isolated nucleic acid sequences, which encode polypeptides with an activity of a polypeptide as indicated in Table II, column 3, lines 103 to 106 and/or 468 to 471 or lines 107 and/or 108 or having the sequence of a polypeptide as indicated in Table II, columns 5 and 7, lines 103 to 106 and/or 468 to 471 or lines 107 and/or 108 and conferring an increase in the cryptoxanthin or zeaxanthin level.

**[0113.0.0.10] to [01200.0.10]: see [0113.0.0.0] to [0120.0.0.0]**

**[0120.1.10.10]:** Production strains which are also advantageously selected in the process according to the invention are microorganisms selected from the group green algae , like Spongioccoccum exentricum, Chlorella sorokiniana (pyrenoidosa, 7-11-05), or form the group of fungi like fungi belonging to the Daccrymycetaceae family, or non-photosynthetic bacteria, like methylotrophs, flavobacteria, actinomycetes, like streptomyces chrestomyceticus, Mycobacteria like Mycobacterim phlei, or Rhodobacter capsulatus.

**[0121.0.10.10]**However, it is also possible to use artificial sequences, which differ in one or more bases from the nucleic acid sequences found in organisms, or in one or more amino acid molecules from polypeptide sequences found in organisms, in particular from the polypeptide sequences indicated in Table II, columns 5 or 7, lines 103 to 106 and/or 468 to 471 or lines 107 and/or 108 or the functional homologues thereof as described herein, preferably conferring above-mentioned activity, i.e. conferring a cryptoxanthin level increase after increasing the activity of the polypeptide sequences indicated in Table II, columns 5 or 7, lines 103 to 106 and/or 468 to 471 or conferring a zeaxanthin level increase after increasing the activity of the polypeptide sequences indicated in Table II, columns 5 or 7, lines 107 and/or 108.

**[0122.0.0.10] to [0127.0.0.10]: see [0122.0.0.0] to [0127.0.0.0]**

**[0128.0.10.10]**Synthetic oligonucleotide primers for the amplification, e.g. as the pairs indicated in Table III, column 7, lines 103 to 106 and/or 468 to 471 or lines 107 and/or 108, by means of polymerase chain reaction can be generated on the basis of a sequence shown herein, for example the sequence as indicated in Table I, columns 5 or 7, lines 103 to 106 and/or 468 to 471, resp., or lines 107 and/or 108, resp. or the sequences derived from a sequences as indicated in Table II, columns 5 or 7, lines 103 to 106 and/or 468 to 471, resp., or lines 107 and/or 108, resp..

**[0129.0.10.10]**Moreover, it is possible to identify conserved regions from various organisms by carrying out protein sequence alignments with the polypeptide used in the process of the invention, in particular with sequences of the polypeptide of the invention or the polypeptide used in the method of the invention, from which conserved regions, and in turn, degenerate primers can be derived. Conserved region for the polypeptide of the invention or the polypeptide used in the method of the invention are indicated in the alignments shown in the figures. Conserved regions are those, which show a very little variation in the amino acid in one particular position of several homologs from different origin. The consensus sequence indicated in Table IV, column 7, lines 103 to 106 and/or 468 to 471 or lines 107 and/or 108 is derived from said alignments.

**[0130.0.10.10]**Degenerated primers can then be utilized by PCR for the amplification of fragments of novel proteins having above-mentioned activity, e.g. conferring the increase of zeaxanthin or cryptoxanthin after increasing the expression or activity the protein comprising said fragment.

**[0131.0.0.10] to [0138.0.0.10]: see [0131.0.0.0] to [0138.0.0.0] [0139.0.10.10]**Polypeptides having above-mentioned activity, i.e. conferring the respective fine chemical level increase, derived from other organisms, can be encoded by other DNA sequences which hybridise to a sequences indicated in Table I, columns 5 or 7, lines 103 to 106 and/or 468 to 471, preferably of Table I B, columns 5 or 7, lines 103 to 106 and/or 468 to 471 for cryptoxanthin or indicated in Table I, columns 5 or 7,lines 107 and/or 108, preferably of Table I B, columns 5 or 7, lines 107 and/or 108 for zeaxanthin under relaxed hybridization conditions and which code on expression for peptides having the respective fine chemical, i.e. cryptoxanthin or zeaxanthin, resp., increasing-activity.

**[0140.0.0.10] to [0146.0.0.10]: see [0140.0.0.0] to [0146.0.0.0]**

**[0147.0.10.10]**Further, the nucleic acid molecule of the invention or used in the method of the invention comprises a nucleic acid molecule, which is a complement of one of the nucleotide sequences of above mentioned nucleic acid molecules or a portion thereof. A nucleic acid molecule which is complementary to one of the nucleotide sequences indicated in Table I, columns 5 or 7, lines 103 to 106 and/or 468 to 471 or lines 107 and/or 108, preferably of Table I B, columns 5 or 7, lines 103 to 106 and/or 468 to 471 or lines 107 and/or 108, is one which is sufficiently complementary to one of said nucleotide sequences such that it can hybridise to one of said nucleotide sequences, thereby forming a stable duplex. Preferably, the hybridisation is performed under stringent hybridisation conditions. However, a complement of one of the herein disclosed sequences is preferably a sequence complement thereto according to the base pairing of nucleic acid molecules well known to the skilled person. For example, the bases A and G undergo base pairing with the bases T and U or C, resp. and visa versa. Modifications of the bases can influence the base-pairing partner.

**[0148.0.10.10]**The nucleic acid molecule of the invention or used in the method of the invention comprises a nucleotide sequence which is at least about 30%, 35%, 40% or 45%, preferably at least about 50%, 55%, 60% or 65%, more preferably at least about 70%, 80%, or 90%, and even more preferably at least about 95%, 97%, 98%, 99% or more homologous to a nucleotide sequence indicated in Table I, columns 5 or 7, lines 103 to 106 and/or 468 to 471 or lines 107 and/or 108, preferably of Table I B, columns 5 or 7, lines 103 to 106 and/or 468 to 471 or lines 107 and/or 108, or a portion thereof and preferably has above mentioned activity, in particular having a cryptoxanthin or zeaxanthin increasing activity after increasing the activity or an activity of a product of a gene encoding said sequences or their homologs.

**[0149.0.10.10]**The nucleic acid molecule of the invention or used in the process of the invention comprises a nucleotide sequence which hybridizes, preferably hybridizes under stringent conditions as defined herein, to one of the nucleotide sequences indicated in Table I, columns 5 or 7, lines 103 to 106 and/or 468 to 471 or lines 107 and/or 108, preferably of Table I B, columns 5 or 7, lines 103 to 106 and/or 468 to 471 or lines 107 and/or 108,, or a portion thereof and encodes a protein having above-mentioned activity, e.g. conferring a of cryptoxanthin or zeaxanthin increase, resp., and optionally, the activity of protein indicated in Table II, column 5, lines 103 to 106 and/or 468 to 471, or lines 107 and/or 108, preferably of Table II B, columns 5 or 7, lines 103 to 106 and/or 468 to 471 or lines 107 and/or 108,.

**[00149.1.10.10]** Optionally, in one embodiment, the nucleotide sequence, which hybridises to one of the nucleotide sequences indicated in Table I, columns 5 or 7, lines 103 to 106 and/or 468 to 471 or lines 107 and/or 108, preferably of Table I B, columns 5 or 7, lines 103 to 106 and/or 468 to 471 or lines 107 and/or 108, has further one or more of the activities annotated or known for the a protein as indicated in Table II, column 3, lines 103 to 106 and/or 468 to 471 or lines 107 and/or 108.

**[0150.0.10.10]**Moreover, the nucleic acid molecule of the invention or used in the process of the invention can comprise only a portion of the coding region of one of the sequences indicated in Table I, columns 5 or 7, lines 103 to 106 and/or 468 to 471 or lines 107 and/or 108, preferably of Table I B, columns 5 or 7, lines 103 to 106 and/or 468 to 471 or lines 107 and/or 108,, for example a fragment which can be used as a probe or primer or a fragment encoding a biologically active portion of the polypeptide of the present invention or of a polypeptide used in the process of the present invention, i.e. having above-mentioned activity, e.g. conferring an increase of cryptoxanthin or zeaxanthin, resp., if its activity is increased. The nucleotide sequences determined from the cloning of the present protein-according-to-the-invention-encoding gene allows for the generation of probes and primers designed for use in identifying and/or cloning its homologues in other cell types and organisms. The probe/primer typically comprises substantially purified oligonucleotide. The oligonucleotide typically comprises a region of nucleotide sequence that hybridizes under stringent conditions to at least about 12, 15 preferably about 20 or 25, more preferably about 40, 50 or 75 consecutive nucleotides of a sense strand of one of the sequences set forth, e.g., as indicated in Table I, columns 5 or 7, lines 103 to 106 and/or 468 to 471 or lines 107 and/or 108, an anti-sense sequence of one of the sequences, e.g., as indicated in Table I, columns 5 or 7, lines 103 to 106 and/or 468 to 471 or lines 107 and/or 108, or naturally occurring mutants thereof. Primers based on a nucleotide of invention can be used in PCR reactions to clone homologues of the polypeptide of the invention or of the polypeptide used in the process of the invention, e.g. as the primers described in the examples of the present invention, e.g. as shown in the examples. A PCR with the primer pairs indicated in Table III, column 7, lines 103 to 106 and/or 468 to 471 or lines 107 and/or 108 can result in a fragment of a polynucleotide sequence as indicated in Table I, columns 5 or 7, lines 103 to 106 and/or 468 to 471 or lines 107 and/or 108 or its gene product.

**[0151.0.0.10]:** see **[0151.0.0.0]**

**[0152.0.10.10]**The nucleic acid molecule of the invention or the nucleic acid used in the method of the invention encodes a polypeptide or portion thereof which includes an amino acid sequence which is sufficiently homologous to an amino acid sequence as indicated in Table II, columns 5 or 7, lines 103 to 106 and/or 468 to 471 or lines 107 and/or 108 such that the protein or portion thereof maintains the ability to participate in the respective fine chemical production, in particular a cryptoxanthin (lines 103 to 106 and/or 468 to 471) or zeaxanthin (lines 107 and/or 108) increasing activity as mentioned above or as described in the examples in plants or microorganisms is comprised.

**[0153.0.10.10]**As used herein, the language "sufficiently homologous" refers to proteins or portions thereof which have amino acid sequences which include a minimum number of identical or equivalent amino acid residues (e.g., an amino acid residue which has a similar side chain as an amino acid residue in one of the sequences of the polypeptide of the present invention) to an amino acid sequence as indicated in Table II, columns 5 or 7, lines 103 to 106 and/or 468 to 471 or lines 107 and/or 108 such that the protein or portion thereof is able to participate in the increase of the respective fine chemical production. In one embodiment, a protein or portion thereof as indicated in Table II, columns 5 or 7, lines 103 to 106 and/or 468 to 471 or lines 107 and/or 108 has for example an activity of a polypeptide indicated in Table II, column 3, lines 103 to 106 and/or 468 to 471 or lines 107 and/or 108.

**[0154.0.10.10]**In one embodiment, the nucleic acid molecule of the present invention comprises a nucleic acid that encodes a portion of the protein of the present invention. The protein is at least about 30%, 35%, 40%, 45% or 50%, preferably at least about 55%, 60%, 65% or 70%, and more preferably at least about 75%, 80%, 85%, 90%, 91%, 92%, 93% or 94% and most preferably at least about 95%, 97%, 98%, 99% or more homologous to an entire amino acid sequence as indicated in Table II, columns 5 or 7, lines 103 to 106 and/or 468 to 471 or lines 107 and/or 108 and has above-mentioned activity, e.g. conferring preferably the increase of the respective fine chemical.

**[0155.0.0.10]** to **[0156.0.0.10]: see [0155.0.0.0]** to **[0156.0.0.0]**

**[0157.0.10.10]**The invention further relates to nucleic acid molecules that differ from one of the nucleotide sequences as indicated in Table I, columns 5 or 7, lines 103 to 106 and/or 468 to 471 or lines 107 and/or 108 (and portions thereof) due to degeneracy of the genetic code and thus encode a polypeptide of the present invention, in particular a polypeptide having above mentioned activity, e.g. conferring an increase in the respective fine chemical in a organism, e.g. as polypeptides comprising a consensus sequence as indicated in Table IV, column 7, lines 103 to 106 and/or 468 to 471 or lines 107 and/or 108 or as polypeptides depicted in Table II, columns 5 or 7, lines 103 to 106 and/or 468 to 471 or lines 107 and/or 108 or the functional homologues. Advantageously, the nucleic acid molecule of the invention or used in the method of the invention comprises, or in an other embodiment has, a nucleotide sequence encoding a protein comprising, or in an other embodiment having, an amino acid sequence of a consensus sequences as indicated in Table IV, column 7, lines 103 to 106 and/or 468 to 471 or lines 107 and/or 108 or of the polypeptide as indicated in Table II, columns 5 or 7, lines 103 to 106 and/or 468 to 471 or lines 107 and/or 108, resp., or the functional homologues. In a still further embodiment, the nucleic acid molecule of the invention encodes a full length protein which is substantially homologous to an amino acid sequence comprising a consensus sequence as indicated in Table IV, column 7, lines 103 to 106 and/or 468 to 471 or lines 107 and/or 108 or of a polypeptide as indicated in Table II, columns 5 or 7, lines 103 to 106 and/or 468 to 471 or lines 107 and/or 108 or the functional homologues thereof. However, in a preferred embodiment, the nucleic acid molecule of the present invention does not consist of a sequence as indicated in Table I, columns 5 or 7, lines 103 to 106 and/or 468 to 471 or lines 107 and/or 108, resp., preferably of Table I A, columns 5 or 7, lines 103 to 106 and/or 468 to 471 or lines 107 and/or 108. Preferably the nucleic acid molecule of the invention is a functional homologue or identical to a nucleic acid molecule indicated in Table I B, columns 5 or 7, lines 103 to 106 and/or 468 to 471 or lines 107 and/or 108.

**[0158.0.0.10]** to **[0160.0.0.10]: see [0158.0.0.0]** to **[0160.0.0.0] [0161.0.10.10]**Accordingly, in another embodiment, a nucleic acid molecule of the invention is at least 15, 20, 25 or 30 nucleotides in length. Preferably, it hybridizes under stringent conditions to a nucleic acid molecule comprising a nucleotide sequence of the nucleic acid molecule of the present invention or used in the process of the present invention, e.g. comprising a sequence as indicated in Table I, columns 5 or 7, lines 103 to 106 and/or 468 to 471 or lines 107 and/or 108. The nucleic acid molecule is preferably at least 20, 30, 50, 100, 250 or more nucleotides in length.

**[0162.0.0.10] see [0162.0.0.0]**

**[0163.0.10.10]**Preferably, nucleic acid molecule of the invention that hybridizes under stringent conditions to a sequence as indicated in Table I, columns 5 or 7, lines 103 to 106 and/or 468 to 471 or lines 107 and/or 108 corresponds to a naturally-occurring nucleic acid molecule of the invention. As used herein, a "naturally-occurring" nucleic acid molecule refers to an RNA or DNA molecule having a nucleotide sequence that occurs in nature (e.g., encodes a natural protein). Preferably, the nucleic acid molecule encodes a natural protein having above-mentioned activity, e.g. conferring the increase of the amount of the respective fine chemical in a organism or a part thereof, e.g. a tissue, a cell, or a compartment of a cell, after increasing the expression or activity thereof or the activity of a protein of the invention or used in the process of the invention.

**[0164.0.0.10] see [0164.0.0.0]**

**[0165.0.10.10]**For example, nucleotide substitutions leading to amino acid substitutions at "non-essential" amino acid residues can be made in a sequence of the nucleic acid molecule of the invention or used in the process of the invention, e.g. as indicated in Table I, columns 5 or 7, lines 103 to 106 and/or 468 to 471 or lines 107 and/or 108, resp.,.

**[0166.0.0.10] to [0167.0.0.10]: see [0166.0.0.0] to [0167.0.0.0]**

**[0168.0.10.10]**Accordingly, the invention relates to nucleic acid molecules encoding a polypeptide having above-mentioned activity, e.g. conferring an increase in the respective fine chemical in an organisms or parts thereof that contain changes in amino acid residues that are not essential for said activity. Such polypeptides differ in amino acid sequence from a sequence contained in a sequence as indicated in Table II, columns 5 or 7, lines 103 to 106 and/or 468 to 471 or lines 107 and/or 108, preferably of Table II B, column 7, lines 103 to 106 and/or 468 to 471 or lines 107 and/or 108, resp., yet retain said activity described herein. The nucleic acid molecule can comprise a nucleotide sequence encoding a polypeptide, wherein the polypeptide comprises an amino acid sequence at least about 50% identical to an amino acid sequence as indicated in Table II, columns 5 or 7, lines 103 to 106 and/or 468 to 471 or lines 107 and/or 108, preferably of Table II B, column 7, lines 103 to 106 and/or 468 to 471 or lines 107 and/or 108, resp., and is capable of participation in the increase of production of the respective fine chemical after increasing its activity, e.g. its expression. Preferably, the protein encoded by the nucleic acid molecule is at least about 60% identical to a sequence as indicated in Table II, columns 5 or 7, lines 103 to 106 and/or 468 to 471 or lines 107 and/or 108, preferably of Table II B, column 7, lines 103 to 106 and/or 468 to 471 or lines 107 and/or 108, resp., more preferably at least about 70% identical to one of the sequences as indicated in Table II, columns 5 or 7, lines 103 to 106 and/or 468 to 471 or lines 107 and/or 108, preferably of Table II B, column 7, lines 103 to 106 and/or 468 to 471 or lines 107 and/or 108, resp., even more preferably at least about 80%, 90%, 95% homologous to a sequence as indicated in Table II, columns 5 or 7, lines 103 to 106 and/or 468 to 471 or lines 107 and/or 108, preferably of Table II B, column 7, lines 103 to 106 and/or 468 to 471 or lines 107 and/or 108, resp., and most preferably at least about 96%, 97%, 98%, or 99% identical to the sequence as indicated in Table II, columns 5 or 7, lines 103 to 106 and/or 468 to 471 or lines 107 and/or 108, preferably of Table II B, column 7, lines 103 to 106 and/or 468 to 471 or lines 107 and/or 108, resp.

**[0169.0.0.10]** to **[0172.0.0.10]: see [0169.0.0.0]** to **[0172.0.0.0]**

**[0173.0.10.10]**For example a sequence, which has 80% homology with sequence SEQ ID NO: 10237 at the nucleic acid level is understood as meaning a sequence which, upon comparison with the sequence SEQ ID NO: 10237 by the above Gap program algorithm with the above parameter set, has a 80% homology.

**[0174.0.0.10]: see [0174.0.0.0]**

**[0175.0.10.10]**For example a sequence which has a 80% homology with sequence SEQ ID NO: 10238 at the protein level is understood as meaning a sequence which, upon comparison with the sequence SEQ ID NO: 10238 by the above program algorithm with the above parameter set, has a 80% homology.

**[0176.0.10.10]**Functional equivalents derived from one of the polypeptides as indicated in Table II, columns 5 or 7, lines 103 to 106 and/or 468 to 471 or lines 107 and/or 108, resp., according to the invention by substitution, insertion or deletion have at least 30%, 35%, 40%, 45% or 50%, preferably at least 55%, 60%, 65% or 70% by preference at least 80%, especially preferably at least 85% or 90%, 91%, 92%, 93% or 94%, very especially preferably at least 95%, 97%, 98% or 99% homology with one of the polypeptides as indicated in Table II, columns 5 or 7, lines 103 to 106 and/or 468 to 471 or lines 107 and/or 108, resp., according to the invention and are distinguished by essentially the same properties as a polypeptide as indicated in Table II, columns 5 or 7, lines 103 to 106 and/or 468 to 471 or lines 107 and/or 108, resp.,.

**[0177.0.10.10]**Functional equivalents derived from a nucleic acid sequence as indicated in Table I, columns 5 or 7, lines 103 to 106 and/or 468 to 471 or lines 107 and/or 108,resp., according to the invention by substitution, insertion or deletion have at least 30%, 35%, 40%, 45% or 50%, preferably at least 55%, 60%, 65% or 70% by preference at least 80%, especially preferably at least 85% or 90%, 91%, 92%, 93% or 94%, very especially preferably at least 95%, 97%, 98% or 99% homology with one of the polypeptides as indicated in Table II, columns 5 or 7, lines 103 to 106 and/or 468 to 471 or lines 107 and/or 108, resp., according to the invention and encode polypeptides having essentially the same properties as a polypeptide as indicated in Table II, columns 5 or 7, lines 103 to 106 and/or 468 to 471 or lines 107 and/or 108, resp.

**[0178.0.0.10] see [0178.0.0.0]**

**[0179.0.10.10]**A nucleic acid molecule encoding an homologous to a protein sequence as indicated in Table II, columns 5 or 7, lines 103 to 106 and/or 468 to 471 or lines 107 and/or 108, preferably of Table II B, column 7, lines 103 to 106 and/or 468 to 471 or lines 107 and/or 108, resp., can be created by introducing one or more nucleotide substitutions, additions or deletions into a nucleotide sequence of the nucleic acid molecule of the present invention, in particular as indicated in Table I, columns 5 or 7, lines 103 to 106 and/or 468 to 471 or lines 107 and/or 108, resp., such that one or more amino acid substitutions, additions or deletions are introduced into the encoded protein. Mutations can be introduced into the encoding sequences of a sequences for example as indicated in Table I, columns 5 or 7, lines 103 to 106 and/or 468 to 471 or lines 107 and/or 108, resp., by standard techniques, such as site-directed mutagenesis and PCR-mediated mutagenesis.

**[0180.0.0.10] to [0183.0.0.10]: see [0180.0.0.0] to [0183.0.0.0]**

**[0184.0.10.10]** Homologues of the nucleic acid sequences used, with a sequence as indicated in Table I, columns 5 or 7, lines 103 to 106 and/or 468 to 471 or lines 107 and/or 108, preferably of Table I B, column 7, lines 103 to 106 and/or 468 to 471 or lines 107 and/or 108, resp., or of the nucleic acid sequences derived from a sequences as indicated in Table II, columns 5 or 7, lines 103 to 106 and/or 468 to 471 or lines 107 and/or 108, preferably of Table II B, column 7, lines 103 to 106 and/or 468 to 471 or lines 107 and/or 108, resp.,, comprise also allelic variants with at least approximately 30%, 35%, 40% or 45% homology, by preference at least approximately 50%, 60% or 70%, more preferably at least approximately 90%, 91%, 92%, 93%, 94% or 95% and even more preferably at least approximately 96%, 97%, 98%, 99% or more homology with one of the nucleotide sequences shown or the abovementioned derived nucleic acid sequences or their homologues, derivatives or analogues or parts of these. Allelic variants encompass in particular functional variants which can be obtained by deletion, insertion or substitution of nucleotides from the sequences shown, preferably from a sequence as indicated in Table I, columns 5 or 7, lines 103 to 106 and/or 468 to 471 or lines 107 and/or 108, resp., or from the derived nucleic acid sequences, the intention being, however, that the enzyme activity or the biological activity of the resulting proteins synthesized is advantageously retained or increased.

**[0185.0.10.10]**In one embodiment of the present invention, the nucleic acid molecule of the invention or used in the process of the invention comprises one or more sequences as indicated in Table I, columns 5 or 7, lines 103 to 106 and/or 468 to 471 or lines 107 and/or 108, preferably of Table I B, column 7, lines 103 to 106 and/or 468 to 471 or lines 107 and/or 108, resp.,. In one embodiment, it is preferred that the nucleic acid molecule comprises as little as possible other nucleotides or nucleotide sequences not shown in any one of sequences as indicated in Table I, columns 5 or 7, lines 103 to 106 and/or 468 to 471 or lines 107 and/or 108, preferably of Table I B, column 7, lines 103 to 106 and/or 468 to 471 or lines 107 and/or 108, resp.,. In one embodiment, the nucleic acid molecule comprises less than 500, 400, 300, 200, 100, 90, 80, 70, 60, 50 or 40 further nucleotides. In a further embodiment, the nucleic acid molecule comprises less than 30, 20 or 10 further nucleotides. In one embodiment, a nucleic acid molecule used in the process of the invention is identical to a sequence as indicated in Table I, columns 5 or 7, lines 103 to 106 and/or 468 to 471 or lines 107 and/or 108, preferably of Table I B, column 7, lines 103 to 106 and/or 468 to 471 or lines 107 and/or 108, resp.,.

**[0186.0.10.10]**Also preferred is that one or more nucleic acid molecule(s) used in the process of the invention encodes a polypeptide comprising a sequence as indicated in Table II, columns 5 or 7, lines 103 to 106 and/or 468 to 471 or lines 107 and/or 108, preferably of Table II B, column 7, lines 103 to 106 and/or 468 to 471 or lines 107 and/or 108, resp.,. In one embodiment, the nucleic acid molecule encodes less than 150, 130, 100, 80, 60, 50, 40 or 30 further amino acids. In a further embodiment, the encoded polypeptide comprises less than 20, 15, 10, 9, 8, 7, 6 or 5 further amino acids. In one embodiment, the encoded polypeptide used in the process of the invention is identical to the sequences as indicated in Table II, columns 5 or 7, lines 103 to 106 and/or 468 to 471 or lines 107 and/or 108, resp. preferably of Table II B, column 7, lines 103 to 106 and/or 468 to 471 or lines 107 and/or 108".

**[0187.0.10.10]**In one embodiment, a nucleic acid molecule of the invention or used in the process encodes a polypeptide comprising the sequence as indicated in Table II, columns 5 or 7, lines 103 to 106 and/or 468 to 471 or lines 107 and/or 108, preferably of Table II B, column 7, lines 103 to 106 and/or 468 to 471 or lines 107 and/or 108,resp., comprises less than 100 further nucleotides. In a further embodiment, said nucleic acid molecule comprises less than 30 further nucleotides. In one embodiment, the nucleic acid molecule used in the process is identical to a coding sequence encoding a sequences as indicated in Table II, columns 5 or 7, lines 103 to 106 and/or 468 to 471 or lines 107 and/or 108, resp., preferably of Table II B, column 7, lines 103 to 106 and/or 468 to 471 or lines 107 and/or 108.

**[0188.0.10.10]**Polypeptides (= proteins), which still have the essential enzymatic activity of the polypeptide of the present invention conferring an increase of the respective fine chemical i.e. whose activity is essentially not reduced, are polypeptides with at least 10% or 20%, by preference 30% or 40%, especially preferably 50% or 60%, very especially preferably 80% or 90 or more of the wild type biological activity or enzyme activity, advantageously, the activity is essentially not reduced in comparison with the activity of a polypeptide as indicated in Table II, columns 5 or 7, lines 103 to 106 and/or 468 to 471 or lines 107 and/or 108, resp., preferably compared to a sequence as indicated in Table II, column 3 and 5, lines 103 to 106 and/or 468 to 471 or lines 107 and/or 108, and expressed under identical conditions.
In one embodiment, the polypeptide of the invention is a homolog consisting of or comprising the sequence as indicated in Table II B, column 7, lines 103 to 106 and/or 468 to 471 or lines 107 and/or 108.

**[0189.0.10.10]**Homologues of a sequences as indicated in Table I, columns 5 or 7, lines 103 to 106 and/or 468 to 471 or lines 107 and/or 108, resp., or of a derived sequences as indicated in Table II, columns 5 or 7, lines 103 to 106 and/or 468 to 471 or lines 107 and/or 108, resp., also mean truncated sequences, cDNA, single-stranded DNA or RNA of the coding and noncoding DNA sequence. Homologues of said sequences are also understood as meaning derivatives, which comprise noncoding regions such as, for example, UTRs, terminators, enhancers or promoter variants. The promoters upstream of the nucleotide sequences stated can be modified by one or more nucleotide substitution(s), insertion(s) and/or deletion(s) without, however, interfering with the functionality or activity either of the promoters, the open reading frame (= ORF) or with the 3'-regulatory region such as terminators or other 3'regulatory regions, which are far away from the ORF. It is furthermore possible that the activity of the promoters is increased by modification of their sequence, or that they are replaced completely by more active promoters, even promoters from heterologous organisms. Appropriate promoters are known to the person skilled in the art and are mentioned herein below.

**[0190.0.0.10]: see [0190.0.0.0]**

**[0191.0.0.10] see [0191.1.0.0]**

**[0192.0.0.10] to [0203.0.0.10]: see [0192.0.0.0] to [0203.0.0.0]**

**[0204.0.10.10]**Accordingly, in one embodiment, the invention relates to a nucleic acid molecule, which comprises a nucleic acid molecule selected from the group consisting of:
a) nucleic acid molecule encoding, preferably at least the mature form, of a polypeptide as indicated in Table II, columns 5 or 7, lines 103 to 106 and/or 468 to 471 or lines 107 and/or 108, preferably of Table II B, column 7, lines 103 to 106 and/or 468 to 471 or lines 107 and/or 108resp.; or a fragment thereof conferring an increase in the amount of the respective fine chemical, i.e. beta-cryptoxanthin (lines 103 to 106 and/or 468 to 471) or zeaxanthin (lines 107 and/or 108), resp., in an organism or a part thereof
b) nucleic acid molecule comprising, preferably at least the mature form, of a nucleic acid molecule as indicated in Table I, columns 5 or 7, lines 103 to 106 and/or 468 to 471 or lines 107 and/or 108, preferably of Table I B, column 7, lines103 to 106 and/or 468 to 471 or lines 107 and/or 108 resp., or a fragment thereof conferring an increase in the amount of the respective fine chemical in an organism or a part thereof;
c) nucleic acid molecule whose sequence can be deduced from a polypeptide sequence encoded by a nucleic acid molecule of (a) or (b) as result of the degeneracy of the genetic code and conferring an increase in the amount of the respective fine chemical in an organism or a part thereof;
d) nucleic acid molecule encoding a polypeptide whose sequence has at least 50% identity with the amino acid sequence of the polypeptide encoded by the nucleic acid molecule of (a) to (c) and conferring an increase in the amount of the respective fine chemical in an organism or a part thereof;
e) nucleic acid molecule which hybridizes with a nucleic acid molecule of (a) to (c) under stringent hybridisation conditions and conferring an increase in the amount of the respective fine chemical in an organism or a part thereof;
f) nucleic acid molecule encoding a polypeptide, the polypeptide being derived by substituting, deleting and/or adding one or more amino acids of the amino acid sequence of the polypeptide encoded by the nucleic acid molecules (a) to (d), preferably to (a) to (c), and conferring an increase in the amount of the respective fine chemical in an organism or a part thereof;
g) nucleic acid molecule encoding a fragment or an epitope of a polypeptide which is encoded by one of the nucleic acid molecules of (a) to (e), preferably to (a) to (c) and conferring an increase in the amount of the respective fine chemical in an organism or a part thereof ;
h) nucleic acid molecule comprising a nucleic acid molecule which is obtained by amplifying a cDNA library or a genomic library using primers or primer pairs as indicated in Table III, column 7, lines 103 to 106 and/or 468 to 471 or lines 107 and/or 108 and conferring an increase in the amount of the respective fine chemical, i.e. beta-cryptoxanthin (lines 103 to 106 and/or 468 to 471) or zeaxanthin (lines 107 and/or 108), resp., in an organism or a part thereof;
i) nucleic acid molecule encoding a polypeptide which is isolated, e.g. from a expression library, with the aid of monoclonal antibodies against a polypeptide encoded by one of the nucleic acid molecules of (a) to (g), preferably to (a) to (c) and conferring an increase in the amount of the respective fine chemical in an organism or a part thereof;
j) nucleic acid molecule which encodes a polypeptide comprising a consensus sequence as indicated in Table IV, column 7, lines 103 to 106 and/or 468 to 471 or lines 107 and/or 108 and conferring an increase in the amount of the respective fine chemical, i.e. beta-cryptoxanthin (lines 103 to 106 and/or 468 to 471) or zeaxanthin (lines 107 and/or 108), resp., in an organism or a part thereof;
k) nucleic acid molecule encoding the amino acid sequence of a polypeptide encoding a domain of a polypeptide as indicated in Table II, columns 5 or 7, lines 103 to 106 and/or 468 to 471 or lines 107 and/or 108, preferably of Table II B, column 7, lines103 to 106 and/or 468 to 471 or lines 107 and/or 108, resp., and conferring an increase in the amount of the respective fine chemical, i.e. beta-cryptoxanthin (lines 103 to 106 and/or 468 to 471) or zeaxanthin (lines 107 and/or 108), resp., in an organism or a part thereof; and
l) nucleic acid molecule which is obtainable by screening a suitable nucleic acid library under stringent hybridization conditions with a probe comprising one of the sequences of the nucleic acid molecule of (a) to (k) or with a fragment of at least 15 nt, preferably 20 nt, 30 nt, 50 nt, 100 nt, 200 nt or 500 nt of the nucleic acid molecule characterized in (a) to (h) or of a nucleic acid molecule as indicated in Table I, columns 5 or 7, lines 103 to 106 and/or 468 to 471 or lines 107 and/or 108, preferably of Table II B, column 7, lines103 to 106 and/or 468 to 471 or lines 107 and/or 108, resp., or a nucleic acid molecule encoding, preferably at least the mature form of, a polypeptide as indicated in Table II, columns 5 or 7, lines 103 to 106 and/or 468 to 471 or lines 107 and/or 108, preferably of Table II B, column 7, lines 103 to 106 and/or 468 to 471 or lines 107 and/or 108 resp.,, and conferring an increase in the amount of the respective fine chemical in an organism or a part thereof;
or which encompasses a sequence which is complementary thereto;
whereby, preferably, the nucleic acid molecule according to (a) to (I)
distinguishes over the sequence indicated in Table IA, columns 5 or 7, lines 1 to 5 and/or lines 334 to 338, by one or more nucleotides. In one embodiment, the nucleic acid molecule does not consist of the sequence shown and indicated in Table I A or I B, columns 5 or 7, lines 103 to 106 and/or 468 to 471 or lines 107 and/or 108: In one embodiment, the nucleic acid molecule is less than 100%, 99,999%, 99,99%, 99,9% or 99% identical to a sequence indicated in Table I A or I B, columns 5 or 7, lines 103 to 106 and/or 468 to 471 or lines 107 and/or 108. In another embodiment, the nucleic acid molecule does not encode a polypeptide of a sequence indicated in Table II A or II B, columns 5 or 7, lines 103 to 106 and/or 468 to 471 or lines 107 and/or 108. In an other embodiment, the nucleic acid molecule of the present invention is at least 30 %, 40 %, 50%, or 60% identical and less than 100%, 99,999%, 99,99%, 99,9% or 99% identical to a sequence indicated in Table I A or I B, columns 5 or 7, lines 103 to 106 and/or 468 to 471 or lines 107 and/or 108. In a further embodiment the nucleic acid molecule does not encode a polypeptide sequence as indicated in Table II A or II B, columns 5 or 7, lines 103 to 106 and/or 468 to 471 or lines 107 and/or 108. Accordingly, in one embodiment, the nucleic acid molecule of the differs at least in one or more residues from a nucleic acid molecule indicated in Table I A or I B, columns 5 or 7, lines 103 to 106 and/or 468 to 471 or lines 107 and/or 108. Accordingly, in one embodiment, the nucleic acid molecule of the present invention encodes a polypeptide, which differs at least in one or more amino acids from a polypeptide indicated in Table II A or II B, columns 5 or 7, lines 103 to 106 and/or 468 to 471 or lines 107 and/or 108. In another embodiment, a nucleic acid molecule indicated in Table I A or I B, columns 5 or 7, lines 103 to 106 and/or 468 to 471 or lines 107 and/or 108 does not encode a protein of a sequence indicated in Table II A or II B, columns 5 or 7, lines 103 to 106 and/or 468 to 471 or lines 107 and/or 108. Accordingly, in one embodiment, the protein encoded by a sequences of a nucleic acid according to (a) to (I) does not consist of a sequence as indicated in Table II A or II B, columns 5 or 7, lines 103 to 106 and/or 468 to 471 or lines 107 and/or 108. In a further embodiment, the protein of the present invention is at least 30 %, 40 %, 50%, or 60% identical to a protein sequence indicated in Table II A or II B, columns 5 or 7, lines 103 to 106 and/or 468 to 471 or lines 107 and/or 108 and less than 100%, preferably less than 99,999%, 99,99% or 99,9%, more preferably less than 99%, 985, 97%, 96% or 95% identical to a sequence as indicated in Table II A or II B, columns 5 or 7, lines 103 to 106 and/or 468 to 471 or lines 107 and/or 108.

**[0205.0.0.10]** to **[0206.0.0.10]: see [0205.0.0.0]** to **[0206.0.0.0]**

**[0207.0.10.10]** As described herein, the nucleic acid construct can also comprise further genes, which are to be introduced into the organisms or cells. It is possible and advantageous to introduce into, and express in, the host organisms regulatory genes such as genes for inductors, repressors or enzymes, which, owing to their enzymatic activity, engage in the regulation of one or more genes of a biosynthetic pathway. These genes can be of heterologous or homologous origin. Moreover, further biosynthesis genes may advantageously be present, or else these genes may be located on one or more further nucleic acid constructs. Genes, which are advantageously employed as biosynthesis genes, are genes of the carotenoid metabolism, the xanthophyll metabolism, the astaxanthin metabolism, the amino acid metabolism, of glycolysis, of the tricarboxylic acid metabolism or their combinations. As described herein, regulator sequences or factors can have a positive effect on preferably the gene expression of the genes introduced, thus increasing it. Thus, an enhancement of the regulator elements may advantageously take place at the transcriptional level by using strong transcription signals such as promoters and/or enhancers. In addition, however, an enhancement of translation is also possible, for example by increasing mRNA stability or by inserting a translation enhancer sequence.

**[0208.0.0.10] to [0226.0.0.10]: see [0208.0.0.0] to [0226.0.0.0]**

**[0227.0.10.10]**The abovementioned nucleic acid molecules can be cloned into the nucleic acid constructs or vectors according to the invention in combination together with further genes, or else different genes are introduced by transforming several nucleic acid constructs or vectors (including plasmids) into a host cell, advantageously into a plant cell or a microorganisms.
In addition to a sequence indicated in Table I, columns 5 or 7, lines 103 to 106 and/or 468 to 471 or lines 107 and/or 108 or its derivatives, it is advantageous to express and/or mutate further genes in the organisms. Especially advantageously, additionally at least one further gene of the xanthophyll biosynthetic pathway such as for cryptoxanthin or zeaxanthin, e.g. one of the above mentioned genes of this pathway, or e.g. for the synthesis of astaxanthin or for another provitamin A or for another carotenoids is expressed in the organisms such as plants or microorganisms. It is also possible that the regulation of the natural genes has been modified advantageously so that the gene and/or its gene product is no longer subject to the regulatory mechanisms which exist in the organisms. This leads to an increased synthesis of the amino acids desired since, for example, feedback regulations no longer exist to the same extent or not at all. In addition it might be advantageously to combine one or more of the sequences indicated in Table I, columns 5 or 7, lines 103 to 106 and/or 468 to 471 or lines 107 and/or 108, resp., with genes which generally support or enhances to growth or yield of the target organism, for example genes which lead to faster growth rate of microorganisms or genes which produces stress-, pathogen, or herbicide resistant plants.

**[0228.0.10.10]**In a further embodiment of the process of the invention, therefore, organisms are grown, in which there is simultaneous overexpression of at least one nucleic acid or one of the genes which code for proteins involved in the xanthophylls metabolism, in particular in synthesis of beta-cryptoxanthin, zeaxanthin, astaxanthin or lutein.

**[0229.0.10.10]**Further advantageous nucleic acid sequences which can be expressed in combination with the sequences used in the process and/or the abovementioned biosynthesis genes are the sequences encoding further genes of the carotenoids biosynthetic pathway, such as phytoene synthase (Psy), which is an important control point for the regulation of the flux (Fraser et al., 2002), phytoene desaturase (Pds), z-carotene desaturase, above mentioned enzymes (s. introduction of the application), e.g. hydroxylases such as beta-carotene hydroxylase (US 6,214,575), ketolases, or cyclases such as the beta-cyclase (US 6,232,530) or oxygenases such as the beta-C4-oxygenase described in US 6,218,599 or homologs thereof, astaxanthin synthase (US 6,365,386), or other genes as described in US 6,150,130. These genes can lead to an increased synthesis of the essential carotenoids, in particular xanthophylls.

**[0230.0.0.10] see [230.0.0.0].**

**[0231.0.10.10]**In a further advantageous embodiment of the process of the invention, the organisms used in the process are those in which simultaneously a zeaxanthin or cryptoxanthin degrading protein is attenuated, in particular by reducing the rate of expression of the corresponding gene. A person skilled in the art knows for example, that the inhibition of the lutein synthesis from carotene increases the amount of cryptoxanthin and zeaxanthin in an organism, in particular in plants. In one embodiment, the level of astaxanthin in the organism shall be increased. Thus, astaxanthin degrading enzymes are attenuated but not enzymes catalyzing the synthesis of astaxanthin from zeaxanthin or cryptoxanthin.

**[0232.0.0.10] to [0276.0.0.10]: see [0232.0.0.0] to [0276.0.0.0]**

**[0277.0.10.10]** The respective fine chemical produced can be isolated from the organism by methods with which the skilled worker are familiar, for example via extraction, salt precipitation, and/or different chromatography methods. The process according to the invention can be conducted batchwise, semibatchwise or continuously. The fine chemical and other xanthophylls produced by this process can be obtained by harvesting the organisms, either from the crop in which they grow, or from the field. This can be done via pressing or extraction of the plant parts

**[0278.0.0.10]** to **[0282.0.0.10]: see [0278.0.0.0]** to **[0282.0.0.0]**

**[0283.0.10.10]**Moreover, native polypeptide conferring the increase of the respective fine chemical in an organism or part thereof can be isolated from cells (e.g., endothelial cells), for example using the antibody of the present invention as described below, e.g. an antibody against a protein as indicated in Table II, column 3, lines 103 to 106 and/or 468 to 471 or lines 107 and/or 108, resp., or an antibody against a polypeptide as indicated in Table II, columns 5 or 7, lines 103 to 106 and/or 468 to 471 or lines 107 and/or 108, resp., or an antigenic part thereof, which can be produced by standard techniques utilizing polypeptides comprising or consisting of above mentioned sequences, e.g. the polypeptide of the present invention or a fragment thereof, i.e., the polypeptide of this invention. Preferred are monoclonal antibodies specifically binding to polypeptide as indicated in Table II, columns 5 or 7, lines 103 to 106 and/or 468 to 471 or lines 107 and/or 108.

**[0284.0.0.10] see [0284.0.0.0]**

**[0285.0.10.10]**In one embodiment, the present invention relates to a polypeptide having a sequence as indicated in Table II, columns 5 or 7, lines 103 to 106 and/or 468 to 471 or lines 107 and/or 108, resp., or as coded by a nucleic acid molecule as indicated in Table I, columns 5 or 7, lines 103 to 106 and/or 468 to 471 or lines 107 and/or 108, resp., or functional homologues thereof.

**[0286.0.10.10]**In one advantageous embodiment, in the method of the present invention the activity of a polypeptide is increased which comprises or consists of a consensus sequence as indicated in Table IV, column 7, lines 103 to 106 and/or 468 to 471 or lines 107 and/or 108 and in one another embodiment, the present invention relates to a polypeptide comprising or consisting of a consensus sequence as indicated in Table IV, column 7, lines 103 to 106 and/or 468 to 471 or lines 107 and/or 108 whereby 20 or less, preferably 15 or 10, preferably 9, 8, 7, or 6, more preferred 5 or 4, even more preferred 3, even more preferred 2, even more preferred 1, most preferred 0 of the amino acids positions indicated can be replaced by any amino acid. or, in an further embodiment, can be replaced and/or absent. In one embodiment, the present invention relates to the method of the present invention comprising a polypeptide or to a polypeptide comprising more than one consensus sequences (of an individual line) as indicated in Table IV, column 7, lines 103 to 106 and/or 468 to 471 or lines 107 and/or 108.

**[0287.0.0.10]** to **[0289.0.0.10]: see [0287.0.0.0]** to **[0289.0.0.0]**

**[00290.0.10.10]** The alignment was performed with the Software AlignX (sept 25, 2002) a component of Vector NTI Suite 8.0 , InforMax™,Invitrogen™ life science software, U.S. Main Office, 7305 Executive Way,Frederick, MD 21704,USA with the following settings: For pairwise alignments: gap opening penalty: 10,0; gap extension penalty 0,1. For multiple alignments: Gap opening penalty: 10,0; Gap extension penalty: 0,1; Gap separation penalty range: 8; Residue substitution matrix: blosum62; Hydrophilic residues: G P S N D Q E K R; Transition weighting: 0,5; Consensus calculation options: Residue fraction for consensus: 0,9. Presettings were selected to allow also for the alignment of conserved amino acids.

**[0291.0.10.10]**In one advantageous embodiment, the method of the present invention comprises the increasing of a polypeptide comprising or consisting of plant or microorganism specific consensus sequences. Accordingly, in one embodiment, the present invention relates to a polypeptide comprising or consisting of plant or microorganism specific consensus sequences.
In one embodiment, said polypeptide of the invention distinguishes over a sequence as indicated in Table II A or IIB, columns 5 or 7, lines 103 to 106 and/or 468 to 471 or lines 107 and/or 108 by one or more amino acids. In one embodiment, polypeptide distinguishes form a sequence as indicated in Table II A or IIB, columns 5 or 7, lines 103 to 106 and/or 468 to 471 or lines 107 and/or 108 by more than 1, 2, 3, 4, 5, 6, 7, 8 or 9 amino acids, preferably by more than 10, 15, 20, 25 or 30 amino acids, even more preferred are more than 40, 50, or 60 amino acids and, preferably, the sequence of the polypeptide of the invention distinguishes from a sequence as indicated in Table II A or II B, columns 5 or 7, lines 103 to 106 and/or 468 to 471 or lines 107 and/or 108 by not more than 80% or 70% of the amino acids, preferably not more than 60% or 50%, more preferred not more than 40% or 30%, even more preferred not more than 20% or 10%. In an other embodiment, said polypeptide of the invention does not consist of a sequence as indicated in Table II A or II B, columns 5 or 7, lines 103 to 106 and/or 468 to 471 or lines 107 and/or 108.

**[0292.0.0.10] see [0292.0.0.0]**

**[0293.0.10.10]**In one embodiment, the invention relates to polypeptide conferring an increase in the fine chemical in an organism or part being encoded by the nucleic acid molecule of the invention or by a nucleic acid molecule used in the process of the invention.
In one embodiment, the polypeptide of the invention has a sequence which distinguishes from a sequence as indicated in Table II A or II B, columns 5 or 7, lines 103 to 106 and/or 468 to 471 or lines 107 and/or 108 by one or more amino acids. In an other embodiment, said polypeptide of the invention does not consist of the sequence as indicated in Table II A or II B, columns 5 or 7, lines 103 to 106 and/or 468 to 471 or lines 107 and/or 108. In a further embodiment, said polypeptide of the present invention is less than 100%, 99,999%, 99,99%, 99,9% or 99% identical. In one embodiment, said polypeptide does not consist of the sequence encoded by a nucleic acid molecules as indicated in Table I A or IB, columns 5 or 7, lines 103 to 106 and/or 468 to 471 or lines 107 and/or 108.

**[0294.0.10.10]** In one embodiment, the present invention relates to a polypeptide having an activity of a protein as indicated in Table II, column 3, lines 103 to 106 and/or 468 to 471 or lines 107 and/or 108, which distinguishes over a sequence as indicated in Table II A or II B, columns 5 or 7, lines 103 to 106 and/or 468 to 471 or lines 107 and/or 108 by one or more amino acids, preferably by more than 5, 6, 7, 8 or 9 amino acids, preferably by more than 10, 15, 20, 25 or 30 amino acids, even more preferred are more than 40, 50, or 60 amino acids but even more preferred by less than 70% of the amino acids, more preferred by less than 50%, even more preferred my less than 30% or 25%, more preferred are 20% or 15%, even more preferred are less than 10%.

**[0295.0.0.10]** to **[0297.0.0.10]: see [0295.0.0.0]** to **[0297.0.0.0]**

**[00297.1.0.10]** Non-polypeptide of the invention-chemicals are e.g. polypeptides having not the activity and/or amino acid sequence of a polypeptide indicated in Table II, columns 3, 5 or 7, lines 103 to 108 or 468 to 471, resp.,.

**[0298.0.10.10]**A polypeptide of the invention can participate in the process of the present invention. The polypeptide or a portion thereof comprises preferably an amino acid sequence which is sufficiently homologous to an amino acid sequence as indicated in Table II, columns 5 or 7, lines 103 to 106 and/or 468 to 471 or lines 107 and/or 108, resp.. The portion of the protein is preferably a biologically active portion as described herein. Preferably, the polypeptide used in the process of the invention has an amino acid sequence identical to a sequence as indicated in Table II, columns 5 or 7, lines 103 to 106 and/or 468 to 471 or lines 107 and/or 108, resp..

**[0299.0.10.10]**Further, the polypeptide can have an amino acid sequence which is encoded by a nucleotide sequence which hybridizes, preferably hybridizes under stringent conditions as described above, to a nucleotide sequence of the nucleic acid molecule of the present invention. Accordingly, the polypeptide has an amino acid sequence which is encoded by a nucleotide sequence that is at least about 35%, 40%, 45%, 50%, 55%, 60%, 65% or 70%, preferably at least about 75%, 80%, 85% or 90, and more preferably at least about 91%, 92%, 93%, 94% or 95%, and even more preferably at least about 96%, 97%, 98%, 99% or more homologous to one of the nucleotide sequence as indicated in Table I, columns 5 or 7, lines 103 to 106 and/or 468 to 471 or lines 107 and/or 108, resp.,. The preferred polypeptide of the present invention preferably possesses at least one of the activities according to the invention and described herein. A preferred polypeptide of the present invention includes an amino acid sequence encoded by a nucleotide sequence which hybridizes, preferably hybridizes under stringent conditions, to a nucleotide sequence as indicated in Table I, columns 5 or 7, lines 103 to 106 and/or 468 to 471 or lines 107 and/or 108, resp., or which is homologous thereto, as defined above.

**[0300.0.10.10]**Accordingly the polypeptide of the present invention can vary from a sequence as indicated in Table II, columns 5 or 7, lines 103 to 106 and/or 468 to 471 or lines 107 and/or 108, resp., in amino acid sequence due to natural variation or mutagenesis, as described in detail herein. Accordingly, the polypeptide comprise an amino acid sequence which is at least about 35%, 40%, 45%, 50%, 55%, 60%, 65% or 70%, preferably at least about 75%, 80%, 85% or 90, and more preferably at least about 91 %, 92%, 93%, 94% or 95%, and most preferably at least about 96%, 97%, 98%, 99% or more homologous to an entire amino acid sequence of as indicated in Table II A or II B, columns 5 or 7, lines 103 to 106 and/or 468 to 471 or lines 107 and/or 108, resp.,.

**[0301.0.0.10] see [0301.0.0.0]**

**[0302.0.10.10]**Biologically active portions of an polypeptide of the present invention include peptides comprising amino acid sequences derived from the amino acid sequence of the polypeptide of the present invention or used in the process of the present invention, e.g., an amino acid sequence as indicated in Table II, columns 5 or 7, lines 103 to 106 and/or 468 to 471 or lines 107 and/or 108, resp., or the amino acid sequence of a protein homologous thereto, which include fewer amino acids than a full length polypeptide of the present invention or used in the process of the present invention or the full length protein which is homologous to an polypeptide of the present invention or used in the process of the present invention depicted herein, and exhibit at least one activity of polypeptide of the present invention or used in the process of the present invention.

**[0303.0.0.10] see [0303.0.0.0]**

**[0304.0.10.10]**Manipulation of the nucleic acid molecule of the invention or the nucleic acid molecule used in the method of the invention may result in the production of a protein having essentially the activity of the polypeptides as indicated in Table II, column 3, lines 103 to 106 and/or 468 to 471 or lines 107 and/or 108 but having differences in the sequence from said wild-type protein. These proteins may be improved in efficiency or activity, may be present in greater numbers in the cell than is usual, or may be decreased in efficiency or activity in relation to the wild type protein.

**[0305.0.0.10]** to **[0306.0.0.10]: see [0305.0.0.0]** to **[0306.0.0.0]**

**[0306.1.0.10]** Preferably, the compound is a composition comprising the essentially pure cryptoxanthin or zeaxanthin or a recovered or isolated cryptoxanthin or zeaxanthin, in particular, the respective fine chemical, free or in protein-bound form.

**[0307.0.0.10]** to **[0308.0.0.10]: see [0307.0.0.0]** to **[0308.0.0.0]**

**[0309.0.10.10]**In one embodiment, a reference to a protein (= polypeptide) of the invention or as indicated in Table II, columns 5 or 7, lines 103 to 106 and/or 468 to 471 or lines 107 and/or 108, resp., refers to a polypeptide having an amino acid sequence corresponding to the polypeptide of the invention or used in the process of the invention , whereas a " non-polypeptide of the invention" or "other polypeptide" not being indicated in Table II, columns 5 or 7, lines 103 to 106 and/or 468 to 471 or lines 107 and/or 108, resp., refers to a polypeptide having an amino acid sequence corresponding to a protein which is not substantially homologous a polypeptide of the invention, preferably which is not substantially homologous to a polypeptide as indicated in Table II, columns 5 or 7, lines 103 to 106 and/or 468 to 471 or lines 107 and/or 108, resp." e.g., a protein which does not confer the activity described herein or annotated or known for as indicated in Table II, column 3, lines 103 to 106 and/or 468 to 471 or lines 107 and/or 108, resp." and which is derived from the same or a different organism. In one embodiment, a "non-polypeptide of the invention" or "other polypeptide" not being indicated in Table II, columns 5 or 7, lines 103 to 106 and/or 468 to 471 or lines 107 and/or 108, resp., does not confer an increase of the respective fine chemical in an organism or part thereof.

**[0310.0.0.10]** to **[0334.0.0.10]: see [0310.0.0.0]** to **[0334.0.0.0]**

**[0335.0.10.10]**The dsRNAi method has proved to be particularly effective and advantageous for reducing the expression of a nucleic acid sequences as indicated in Table I, columns 5 or 7, lines 103 to 106 and/or 468 to 471 or lines 107 and/or 108, resp., and/or homologs thereof. As described inter alia in WO 99/32619, dsRNAi approaches are clearly superior to traditional antisense approaches. The invention therefore furthermore relates to double-stranded RNA molecules (dsRNA molecules) which, when introduced into an organism, advantageously into a plant (or a cell, tissue, organ or seed derived there from), bring about altered metabolic activity by the reduction in the expression of a nucleic acid sequences as indicated in Table I, columns 5 or 7, lines 103 to 106 and/or 468 to 471 or lines 107 and/or 108, resp., and/or homologs thereof. In a double-stranded RNA molecule for reducing the expression of an protein encoded by a nucleic acid sequence sequences as indicated in Table I, columns 5 or 7, lines 103 to 106 and/or 468 to 471 or lines 107 and/or 108, resp., and/or homologs thereof, one of the two RNA strands is essentially identical to at least part of a nucleic acid sequence, and the respective other RNA strand is essentially identical to at least part of the complementary strand of a nucleic acid sequence.

**[0336.0.0.10] to [0342.0.0.10]: see [0336.0.0.0] to [0342.0.0.0]**

**[0343.0.10.10]**As has already been described, 100 % sequence identity between the dsRNA and a gene transcript of a nucleic acid sequence as indicated in Table I, columns 5 or 7, lines 103 to 106 and/or 468 to 471 or lines 107 and/or 108, resp., or it's homolog is not necessarily required in order to bring about effective reduction in the expression. The advantage is, accordingly, that the method is tolerant with regard to sequence deviations as may be present as a consequence of genetic mutations, polymorphisms or evolutionary divergences. Thus, for example, using the dsRNA, which has been generated starting from a sequence as indicated in Table I, columns 5 or 7, lines 103 to 106 and/or 468 to 471 or lines 107 and/or 108, resp., or homologs thereof of the one organism, may be used to suppress the corresponding expression in another organism.

**[0344.0.0.10] to [0361.0.0.10]: see [0344.0.0.0] to [0361.0.0.0]**

**[0362.0.10.10]**Accordingly the present invention relates to any cell transgenic for any nucleic acid characterized as part of the invention, e.g. conferring the increase of the respective fine chemical in a cell or an organism or a part thereof, e.g. the nucleic acid molecule of the invention or the nucleic acid molecule used in the method of the invention, the nucleic acid construct of the invention, the antisense molecule of the invention, the vector of the invention or a nucleic acid molecule encoding the polypeptide of the invention, e.g. the polypeptide as indicated in Table II, columns 5 or 7, lines 103 to 106 and/or 468 to 471 or lines 107 and/or 108, resp." e.g. encoding a polypeptide having protein activity, as indicated in Table II, columns 3, lines 103 to 106 and/or 468 to 471 or lines 107 and/or 108, resp.,. Due to the above mentioned activity the respective fine chemical content in a cell or an organism is increased. For example, due to modulation or manipulation, the cellular activity of the polypeptide of the invention or nucleic acid molecule of the invention is increased, e.g. due to an increased expression or specific activity of the subject matters of the invention in a cell or an organism or a part thereof. In one embodiment, transgenic for a polypeptide having an activity of a polypeptide as indicated in Table II, columns 5 or 7, lines 103 to 106 and/or 468 to 471 or lines 107 and/or 108, resp., means herein that due to modulation or manipulation of the genome, an activity as annotated for a polypeptide as indicated in Table II, column 3, lines 103 to 106 and/or 468 to 471 or lines 107 and/or 108, e.g. having a sequence as indicated in Table II, columns 5 or 7, lines 103 to 106 and/or 468 to 471 or lines 107 and/or 108, resp., is increased in a cell or an organism or a part thereof. Examples are described above in context with the process of the invention

**[0363.0.0.10] see [0363.0.0.0]**

**[0364.0.10.10]**A naturally occurring expression cassette - for example the naturally occurring combination of a promoter of a gene encoding a polypeptide of the invention as indicated in Table II, column 3, lines 103 to 106 and/or 468 to 471 or lines 107 and/or 108, resp." with the corresponding protein-encoding sequence as indicated in Table I, column 3, lines 103 to 106 and/or 468 to 471 or lines 107 and/or 108, resp.,, - becomes a transgenic expression cassette when it is modified by non-natural, synthetic "artificial" methods such as, for example, mutagenization. Such methods have been described (US 5,565,350; WO 00/15815; also see above).

**[0365.0.0.10] to [0373.0.0.10]: see [0365.0.0.0] to [0373.0.0.0]**

**[0374.0.10.10]**Transgenic plants comprising the respective fine chemical synthesized in the process according to the invention can be marketed directly without isolation of the compounds synthesized. In the process according to the invention, plants are understood as meaning all plant parts, plant organs such as leaf, stalk, root, tubers or seeds or propagation material or harvested material or the intact plant. In this context, the seed encompasses all parts of the seed such as the seed coats, epidermal cells, seed cells, endosperm or embryonic tissue. Xanthophylls, in particular the respective fine chemical, produced in the process according to the invention may, however, also be isolated from the plant in the form of their free xanthophylls, in particular the free respective fine chemical, or bound in or to compounds or moieties, like glucosides, e.g. diglucosides. The respective fine chemical produced by this process can be harvested by harvesting the organisms either from the culture in which they grow or from the field. This can be done via expressing, grinding and/or extraction, salt precipitation and/or ion-exchange chromatography or other chromatographic methods of the plant parts, preferably the plant seeds, plant fruits, plant tubers and the like.

**[0375.0.0.10] to [0376.0.0.10]: see [0375.0.0.0] to [0376.0.0.0] [0377.0.10.10]**Accordingly, the present invention relates also to a process according to the present invention whereby the produced carotenoids comprising, in particular the xanthophylls comprising composition is isolated. In one embodiment, the produced respective fine chemical is isolated.

**[0378.0.10.10]**In this manner, more than 50% by weight, advantageously more than 60% by weight, preferably more than 70% by weight, especially preferably more than 80% by weight, very especially preferably more than 90% by weight, of the xanthophylls, in particular the respective fine chemical produced in the process can be isolated. The resulting recovered, isolated or purified xanthophylls, e.g. the composition comprising xanthophylls can, if appropriate, subsequently be further purified, if desired mixed with other active ingredients such as vitamins, amino acids, carbohydrates, antibiotics and the like, and, if appropriate, formulated.

**[0379.0.10.10]**In one embodiment, the xanthophylls are a mixture of the respective fine chemicals. In one embodiment, the xanthophylls are the respective fine chemical. In one embodiment, the xanthophylls are a mixture of the respective fine chemicals with astaxanthin.

**[0380.0.10.10]**The xanthophylls, in particular the respective fine chemicals obtained in the process are suitable as starting material for the synthesis of further products of value. For example, they can be used in combination with each other or alone for the production of pharmaceuticals, foodstuffs, animal feeds or cosmetics. Accordingly, the present invention relates a method for the production of pharmaceuticals, food stuff, animal feeds, nutrients or cosmetics comprising the steps of the process according to the invention, including the isolation of the carotenoids containing, in particular xanthophylls containing composition produced or the respective fine chemical produced if desired and formulating the product with a pharmaceutical acceptable carrier or formulating the product in a form acceptable for an application in agriculture. A further embodiment according to the invention is the use of the carentoids or xanthophylls produced in the process or of the transgenic organisms in animal feeds, foodstuffs, medicines, food supplements, cosmetics or pharmaceuticals or for the production of astaxanthin, e.g. in after isolation of the respective fine chemical or without, e.g. in situ, e.g. in the organism used for the process for the production of the respective fine chemical.

**[0381.0.0.10]** to **[0382.0.0.10]: see [0381.0.0.0]** to **[0382.0.0.0]**

**[0383.0.10.10]./.**

**[0384.0.0.10] see [0384.0.0.0]**

**[0385.0.10.10]**The fermentation broths obtained in this way, containing in particular zeaxanthin or beta-cryptoxanthin in mixtures with other carotenoids, in particular with other xanthophylls, e.g. with astaxanthin, or containing microorganisms or parts of microorganisms, like plastids, containing zeaxanthin or beta-cryptoxanthin in mixtures with other carotenoids, in particular with other xanthophylls, e.g. with astaxanthin, normally have a dry matter content of from 1 to 70% by weight, preferably 7.5 to 25% by weight. Sugar-limited fermentation is additionally advantageous, e.g. at the end, for example over at least 30% of the fermentation time. This means that the concentration of utilizable sugar in the fermentation medium is kept at, or reduced to, 0 to 10 g/l, preferably to 0 to 3 g/l during this time. The fermentation broth is then processed further. Depending on requirements, the biomass can be removed or isolated entirely or partly by separation methods, such as, for example, centrifugation, filtration, decantation, coagulation/flocculation or a combination of these methods, from the fermentation broth or left completely in it.
The fermentation broth can then be thickened or concentrated by known methods, such as, for example, with the aid of a rotary evaporator, thin-film evaporator, falling film evaporator, by reverse osmosis or by nanofiltration. This concentrated fermentation broth can then be worked up by freeze-drying, spray drying, spray granulation or by other processes.
As carotenoids are often localized in membranes or plastids, in one embodiment it is advantageous to avoid a leaching of the cells when the biomass is isolated entirely or partly by separation methods, such as, for example, centrifugation, filtration, decantation, coagulation/flocculation or a combination of these methods, from the fermentation broth. The dry biomass can directly be added to animal feed, provided the carotenoids concentration is sufficiently high and no toxic compounds are present. In view of the instability of carentoids, conditions for drying, e.g. spray or flash-drying, can be mild and can be avoiding oxidation and *cis*/*trans* isomerization. For example antioxidants, e.g. BHT, ethoxyquin or other, can be added. In case the carotenoids concentration in the biomass is to dilute, solvent extraction can be used for their isolation, e.g. with alcohols, ether or other organic solvents, e.g. with methanol, ethanol, acetone, alcoholic potassium hydroxide, glycerol-phenol, liquefied phenol or for example with acids or bases, like trichloroacetatic acid or potassium hydroxide. A wide range of advantageous Methods and techniques for the isolation of carotenoids, in particular of xanthophylls, in particular of zeaxanthin or cryptoxanthin can be found in the state of the art. In case phenol is used it can for example be removed with ether and water extraction and the dry eluate comprises a mixture of the carotenoids of the biomass.

**[0386.0.10.10]**Accordingly, it is possible to purify the carotenoids, in particular the xanthophylls produced according to the invention further. For this purpose, the product-containing composition, e.g. a total or partial lipid extraction fraction using organic solvents, e.g. as described above, is subjected for example to a saponification to remove triglycerides, partition between e.g. hexane/methanol (separation of non-polar epiphase from more polar hypophasic derivates) and separation via e.g. an open column chromatography or HPLC in which case the desired product or the impurities are retained wholly or partly on the chromatography resin. These chromatography steps can be repeated if necessary, using the same or different chromatography resins. The skilled worker is familiar with the choice of suitable chromatography resins and their most effective use.

**[0387.0.0.10]** to **[0392.0.0.10]: see [0387.0.0.0]** to **[0392.0.0.0]**

**[0393.0.10.10]**In one embodiment, the present invention relates to a method for the identification of a gene product conferring an increase in the respective fine chemical production in a cell, comprising the following steps:
(a) contacting, e.g. hybridising, the nucleic acid molecules of a sample, e.g. cells, tissues, plants or microorganisms or a nucleic acid library , which can contain a candidate gene encoding a gene product conferring an increase in the respective fine chemical after expression, with the nucleic acid molecule of the present invention;
(b) identifying the nucleic acid molecules, which hybridize under relaxed stringent conditions with the nucleic acid molecule of the present invention in particular to the nucleic acid molecule sequence as indicated in Table I, columns 5 or 7, lines 103 to 106 and/or 468 to 471 or lines 107 and/or 108, preferably in Table I B, columns 5 or 7, lines 103 to 106 and/or 468 to 471 or lines 107 and/or 108, resp., and, optionally, isolating the full length cDNA clone or complete genomic clone;
(c) introducing the candidate nucleic acid molecules in host cells, preferably in a plant cell or a microorganism, appropriate for producing the respective fine chemical;
(d) expressing the identified nucleic acid molecules in the host cells;
(e) assaying the respective fine chemical level in the host cells; and
(f) identifying the nucleic acid molecule and its gene product which expression confers an increase in the respective fine chemical level in the host cell after expression compared to the wild type.

**[0394.0.0.10] to [0398.0.0.10]: see [0394.0.0.0] to [0398.0.0.0]**

**[0399.0.10.10]**Furthermore, in one embodiment, the present invention relates to process for the identification of a compound conferring increased the respective fine chemical production in a plant or microorganism, comprising the steps:
(h) culturing a cell or tissue or microorganism or maintaining a plant expressing the polypeptide according to the invention or a nucleic acid molecule encoding said polypeptide and a readout system capable of interacting with the polypeptide under suitable conditions which permit the interaction of the polypeptide with said readout system in the presence of a compound or a sample comprising a plurality of compounds and capable of providing a detectable signal in response to the binding of a compound to said polypeptide under conditions which permit the expression of said readout system and the polypeptide of the present invention or used in the process of the invention; and
(i) identifying if the compound is an effective agonist by detecting the presence or absence or increase of a signal produced by said readout system.
The screen for a gene product or an agonist conferring an increase in the respective fine chemical production can be performed by growth of an organism for example a microorganism in the presence of growth reducing amounts of an inhibitor of the synthesis of the respective fine chemical. Better growth, e.g. higher dividing rate or high dry mass in comparison to the control under such conditions would identify a gene or gene product or an agonist conferring an increase in respective fine chemical production.

**[00399.1.0.10]** One can think to screen for increased production of the respective fine chemical by for example searching for a resistance to a drug blocking the synthesis of the respective fine chemical and looking whether this effect is dependent on the activity or expression of a polypeptide as indicated in Table II, columns 5 or 7, lines 103 to 106 and/or 468 to 471 or lines 107 and/or 108 or a homolog thereof, e.g. comparing the phenotype of nearly identical organisms with low and high activity of a protein as indicated in Table II, columns 5 or 7, lines 103 to 106 and/or 468 to 471 or lines 107 and/or 108 after incubation with the drug.

**[0400.0.0.10] to [0416.0.0.10]: see [0400.0.0.0] to [0416.0.0.0]**

**[0417.0.10.10]** The nucleic acid molecule of the invention or used in the method of the invention, the vector of the invention or the nucleic acid construct of the invention may also be useful for the production of organisms resistant to inhibitors of the carotenoids production biosynthesis pathways. In particular, the overexpression of the polypeptide of the present invention may protect an organism such as a microorganism or a plant against inhibitors, which block the carotenoid synthesis, in particular the respective fine chemical synthesis in said organism. Examples of inhibitors or herbicides blocking the synthesis of carotenoids in organism such as microorganism or plants are for example classified in two groups. The first group consists of inhibitors that cause the accumulation of early intermediates in the pathway, particularly the colorless phytene, e.g. diphenylamine. Other inhibitors preferentially block late reactions in the pathway, notably the cyclization of lycopene. Inhibitors are e.g. nicotine, 2-(4-chlorophenylthio)-triethylamine and other substituted amines as well as nitrogenous heterocyclic bases, e.g. imidazole.
As xanthophylls can protect organisms against damages of oxidative stress, especially singlet oxygen, a increased level of the respective fine chemical can protect plants against herbicides which cause the toxic build-up of oxidative compounds, e.g. singlet oxygen. For example, inhibition of the protoporphorineogen oxidase (Protox), an enzyme important in the synthesis of chlorophyll and heme biosynthesis results in the loss of chlorophyll and carotenoids and in leaky membranes; the membrane destruction is due to creation of free oxygen radicals (which is also reported for other classic photosynthetic inhibitor herbicides).
Accordingly, in one embodiment, the increase of the level of the respective fine chemical is used to protect plants against herbicides destroying membranes due to the creation of free oxygen radicals.
Examples of inhibitors or herbicides building up oxidative stress are aryl triazion, e.g. sulfentrazone, carfentrazone, or diphenylethers, e.g. acifluorfen, lactofen, or oxyfluorfen, or N-Phenylphthalimide, e.g. flumiclorac or flumioxazin, substituted ureas, e.g. fluometuron, tebuthiuron, or diuron, linuron, or triazines, e.g. atrazine, prometryn, ametryn, metributzin, prometon, simazine, or hexazinone, or uracils, e.g. bromacil or terbacil.
Carotenoid inhibitors are e.g. Pyridines and Pyridazinones, e.g. norflurazon, fluridone or dithiopyr. Thus, in one embodiment, the present invention relates to the use of an increase of the respective respective fine chemical according to the present invention for the protection of plants against carotenoids inhibitors as pyridines and pyridazinones.

**[0418.0.0.10] to [0423.0.0.10]: see [0418.0.0.0] to [0423.0.0.0]**

**[0424.0.10.10]**Accordingly, the nucleic acid of the invention or the nucleic acid molecule used in the method of the invention, the polypeptide of the invention or the polypeptid used in the method of the invention, the nucleic acid construct of the invention, the organisms, the host cell, the microorganisms, the plant, plant tissue, plant cell, or the part thereof of the invention, the vector of the invention, the agonist identified with the method of the invention, the nucleic acid molecule identified with the method of the present invention, can be used for the production of the respective fine chemical or of the respective fine chemical and one or more other carotenoids, in particular other xanthophylls, e.g. astaxanthin.
Accordingly, the nucleic acid of the invention, the nucleic acid molecule used in the method of the invention, or the nucleic acid molecule identified with the method of the present invention or the complement sequences thereof, the polypeptide of the invention or the polypeptide used in the method of the invention, the nucleic acid construct of the invention, the organisms, the host cell, the microorganisms, the plant, plant tissue, plant cell, or the part thereof of the invention, the vector of the invention, the antagonist identified with the method of the invention, the antibody of the present invention, the antisense molecule of the present invention, can be used for the reduction of the respective fine chemical in a organism or part thereof, e.g. in a cell.

**[0424.1.0.10]** In a further embodiment the present invention relates to the use of the antagonist of the present invention, the plant of the present invention or a part thereof, the microorganism or the host cell of the present invention or a part thereof for the production a cosmetic composition or a pharmaceutical composition. Such a composition has antioxidative activity, photoprotective activity, tanning activity, can be used for the treating of high levels of cholesterol and/or lipids, can be used to protect, treat or heal the above mentioned diseases, e.g. retinal disorders, hyperholsterolemia, hyperlipidemia, and ahterosclerosis, or can be used for the cleaning, conditioning, and/or treating of the skin, e.g. if combined with a pharmaceutically or cosmetically acceptable carrier.
The xanthophylls can be also used as stabilizer of other colours or oxygen sensitive compounds.

**[0425.0.0.10]** to **[0434.0.0.10]: see [0425.0.0.0]** to **[0434.0.0.0]**

**[0435.0.10.10]** Example 3: In-vivo and in-vitro mutagenesis

**[0436.0.10.10]** An *in vivo* mutagenesis of organisms such as green algae (e.g. Spongiococcum sp, e.g. Spongiococcum exentricum, Chlorella sp.), Saccharomyces, Mortierella, Escherichia and others mentioned above, which are beneficial for the production of xanthophylls can be carried out by passing a plasmid DNA (or another vector DNA) containing the desired nucleic acid sequence or nucleic acid sequences, e.g. the nucleic acid molecule of the invention or the vector of the invention, through E. coli and other microorganisms (for example Bacillus spp. or yeasts such as Saccharomyces cerevisiae) which are not capable of maintaining the integrity of its genetic information. Usual mutator strains have mutations in the genes for the DNA repair system [for example mutHLS, mutD, mutT and the like; for comparison, see Rupp, W.D. (1996) DNA repair mechanisms in Escherichia coli and Salmonella, pp. 2277-2294, ASM: Washington]. The skilled worker knows these strains. The use of these strains is illustrated for example in Greener, A. and Callahan, M. (1994) Strategies 7; 32-34.
In-vitro mutation methods such as increasing the spontaneous mutation rates by chemical or physical treatment are well known to the skilled person. Mutagens like 5-bromo-uracil, N-methyl-N-nitro-N-nitrosoguanidine (= NTG), ethyl methanesulfonate (= EMS), hydroxylamine and/or nitrous acid are widly used as chemical agents for random in-vitro mutagensis. The most common physical method for mutagensis is the treatment with UV irradiation. Another random mutagenesis technique is the error-prone PCR for introducing amino acid changes into proteins. Mutations are deliberately introduced during PCR through the use of error-prone DNA polymerases and special reaction conditions known to a person skilled in the art. For this method randomized DNA sequences are cloned into expression vectors and the resulting mutant libraries screened for altered or improved protein activity as described below.
Site-directed mutagensis method such as the introduction of desired mutations with an M13 or phagemid vector and short oligonucleotides primers is a well-known approach for site-directed mutagensis. The clou of this method involves cloning of the nucleic acid sequence of the invention into an M13 or phagemid vector, which permits recovery of single-stranded recombinant nucleic acid sequence. A mutagenic oligonucleotide primer is then designed whose sequence is perfectly complementary to nucleic acid sequence in the region to be mutated, but with a single difference: at the intended mutation site it bears a base that is complementary to the desired mutant nucleotide rather than the original. The mutagenic oligonucleotide is then allowed to prime new DNA synthesis to create a complementary full-length sequence containing the desired mutation. Another site-directed mutagensis method is the PCR mismatch primer mutagensis method also known to the skilled person. Dpnl site-directed mutagensis is a further known method as described for example in the Stratagene QuickchangeTM site-directed mutagenesis kit protocol. A huge number of other methods are also known and used in common practice.
Positive mutation events can be selected by screening the organisms for the production of the desired respective fine chemical.

**[0436.1.0.10] see [0436.1.0.0]**

**[0437.0.10.10]** Example 4: DNA transfer between Escherichia coli, Saccharomyces cerevisiae and Mortierella alpina

**[0438.0.10.10]** Shuttle vectors such as pYE22m, pPAC-ResQ, pClasper, pAUR224, pAMH10, pAML10, pAMT10, pAMU10, pGMH10, pGML10, pGMT10, pGMU10, pPGAL1, pPADH1, pTADH1, pTAex3, pNGA142, pHT3101 and derivatives thereof which alow the transfer of nucleic acid sequences between Escherichia coli, Saccharomyces cerevisiae and/or Mortierella alpina are available to the skilled worker. An easy method to isolate such shuttle vectors is disclosed by Soni R. and Murray J.A.H. [Nucleic Acid Research, vol. 20 no. 21, 1992: 5852]: If necessary such shuttle vectors can be constructed easily using standard vectors for E. coli (Sambrook, J. et al., (1989), "Molecular Cloning: A Laboratory Manual", Cold Spring Harbor Laboratory Press or Ausubel, F.M. et al. (1994) "Current Protocols in Molecular Biology", John Wiley & Sons) and/or the aforementioned vectors, which have a replication origin for, and suitable marker from, Escherichia coli, Saccharomyces cerevisiae or Mortierella alpina added. Such replication origins are preferably taken from endogenous plasmids, which have been isolated from species used in the inventive process. Genes, which are used in particular as transformation markers for these species are genes for kanamycin resistance (such as those which originate from the Tn5 or Tn-903 transposon) or for chloramphenicol resistance (Winnacker, E.L. (1987) "From Genes to Clones - Introduction to Gene Technology, VCH, Weinheim) or for other antibiotic resistance genes such as for G418, gentamycin, neomycin, hygromycin or tetracycline resistance.

**[0439.0.10.10]** Using standard methods, it is possible to clone a gene of interest into one of the above-described shuttle vectors and to introduce such hybrid vectors into the microorganism strains used in the inventive process. The transformation of Saccharomyces can be achieved for example by LiCl or sheroplast transformation (Bishop et al., Mol. Cell. Biol., 6, 1986: 3401 - 3409; Sherman et al., Methods in Yeasts in Genetics, [Cold Spring Harbor Lab. Cold Spring Harbor, N. Y.] 1982, Agatep et al., Technical Tips Online 1998, 1:51: P01525 or Gietz et al., Methods Mol. Cell. Biol. 5, 1995: 255f) or electroporation (Delorme E., Appl. Environ. Microbiol., vol. 55, no. 9, 1989 : 2242 - 2246).

**[0440.0.10.10]** If the transformed sequence(s) is/are to be integrated advantageously into the genome of the microorganism used in the inventive process for example into the yeast or fungi genome, standard techniques known to the skilled worker also exist for this purpose. Solinger et al. (Proc Natl Acad Sci U S A., 2001 (15): 8447-8453) and Freedman et al. (Genetics, Vol. 162, 15-27, September 2002,) teaches a homolog recombination system dependent on rad 50, rad51, rad54 and rad59 in yeasts. Vectors using this system for homologous recombination are vectors derived from the Ylp series. Plasmid vectors derived for example from the 2µ-Vector are known by the skilled worker and used for the expression in yeasts. Other preferred vectors are for example pART1, pCHY21 or pEVP11 as they have been described by McLeod et al. (EMBO J. 1987, 6:729-736) and Hoffman et al. (Genes Dev. 5, 1991: :561-571.) or Russell et al. (J. Biol. Chem. 258, 1983: 143-149.). Other beneficial yeast vectors are plasmids of the REP, REP-X, pYZ or RIP series.

**[0441.0.0.10]** to **[0443.0.0.10]** see **[0441.0.0.0] to [0443.0.0.0]**

**[0444.0.10.10]** Example 6: Growth of genetically modified organism: media and culture conditions

**[0445.0.10.10]** Genetically modified Yeast, Mortierella or Escherichia coli are grown in synthetic or natural growth media known by the skilled worker. A number of different growth media for Yeast, Mortierella or Escherichia coli are well known and widely available. A method for clulturing Mortierella is disclosed by Jang et al. [Bot. Bull. Acad. Sin. (2000) 41: 41-48]. Mortierella can be grown at 20°C in a culture medium containing: 10 g/I glucose, 5 g/I yeast extract at pH 6.5. Futhermore Jang et al. teaches a submerged basal medium containing 20 g/I soluble starch, 5 g/l Bacto yeast extract, 10 g/l KNO₃, 1 g/l KH₂PO₄, and 0.5 g/l MgSO₄•7H₂O, pH 6.5.

**[0446.0.0.10]** to **[0454.0.0.10]: see [0446.0.0.0]** to **[0454.0.0.0]**

**[0455.0.10.10]** The effect of the genetic modification in plants, fungi, algae, ciliates or on the production of a desired compound (such as a fatty acid) can be determined by growing the modified microorganisms or the modified plant under suitable conditions (such as those described above) and analyzing the medium and/or the cellular components for the elevated production of desired product (i.e. of the lipids or a fatty acid). These analytical techniques are known to the skilled worker and comprise spectroscopy, thin-layer chromatography, various types of staining methods, enzymatic and microbiological methods and analytical chromatography such as high-performance liquid chromatography (see, for example, Ullman, Encyclopedia of Industrial Chemistry, Vol. A2, p. 89-90 and p. 443-613, VCH: Weinheim (1985); Fallon, A., et al., (1987) "Applications of HPLC in Biochemistry" in: Laboratory Techniques in Biochemistry and Molecular Biology, Vol. 17; Rehm et al. (1993) Biotechnology, Vol. 3, Chapter III: "Product recovery and purification", p. 469-714, VCH: Weinheim; Belter, P.A., et al. (1988) Bioseparations: downstream processing for Biotechnology, John Wiley and Sons; Kennedy, J.F., and Cabral, J.M.S. (1992) Recovery processes for biological Materials, John Wiley and Sons; Shaeiwitz, J.A., and Henry, J.D. (1988) Biochemical Separations, in: Ullmann's Encyclopedia of Industrial Chemistry, Vol. B3; Chapter 11, p. 1-27, VCH: Weinheim; and Dechow, F.J. (1989) Separation and purification techniques in biotechnology, Noyes Publications).
In addition to the abovementioned processes, plant lipids are extracted from plant material as described by Cahoon et al. (1999) Proc. Natl. Acad. Sci. USA 96 (22): 12935-12940 and Browse et al. (1986) Analytic Biochemistry 152:141-145. The qualitative and quantitative analysis of lipids is described by Christie, William W., Advances in Lipid Methodology, Ayr/Scotland: Oily Press (Oily Press Lipid Library; 2); Christie, William W., Gas Chromatography and Lipids. A Practical Guide - Ayr, Scotland: Oily Press, 1989, Repr. 1992, IX, 307 pp. (Oily Press Lipid Library; 1); "Progress in Lipid Research, Oxford: Pergamon Press, 1 (1952) - 16 (1977) under the title: Progress in the Chemistry of Fats and Other Lipids CODEN.

**[0456.0.0.10]: see [0456.0.0.0]**

**[0457.0.10.10]**Example 9: Purification of the Xanthophylls and determination of the carotenoids content

**[0458.0.10.10]**Abbreviations:; GC-MS, gas liquid chromatography/mass spectrometry; TLC, thin-layer chromatography.
The unambiguous detection for the presence of Xanthophylls can be obtained by analyzing recombinant organisms using analytical standard methods: LC, LC-MSMS or TLC, as described The total xanthophylls produced in the organism for example in yeasts used in the inventive process can be analysed for example according to the following procedure:
The material such as yeasts, E. coli or plants to be analyzed can be disrupted by sonication, grinding in a glass mill, liquid nitrogen and grinding or via other applicable methods.
Plant material is initially homogenized mechanically by comminuting in a pestle and mortar to make it more amenable to extraction.
A typical sample pretreatment consists of a total lipid extraction using such polar organic solvents as acetone or alcohols as methanol, or ethers, saponification , partition between phases, seperation of non-polar epiphase from more polar hypophasic derivatives and chromatography. E.g.:
For analysis, solvent delivery and aliquot removal can be accomplished with a robotic system comprising a single injector valve Gilson 232XL and a 402 2S1V diluter [Gilson, Inc. USA, 3000 W. Beltline Highway, Middleton, WI]. For saponification, 3 ml of 50% potassium hydroxide hydro-ethanolic solution (4 water: 1 ethanol) can be added to each vial, followed by the addition of 3 ml of octanol. The saponification treatment can be conducted at room temperature with vials maintained on an IKA HS 501 horizontal shaker [Labworld-online, Inc., Wilmington, NC] for fifteen hours at 250 movements/minute, followed by a stationary phase of approximately one hour. Following saponification, the supernatant can be diluted with 0.10 ml of methanol. The addition of methanol cqan be conducted under pressure to ensure sample homogeneity. Using a 0.25 ml syringe, a 0.1 ml aliquot can be removed and transferred to HPLC vials for analysis.
For HPLC analysis, a Hewlett Packard 1100 HPLC, complete with a quaternary pump, vacuum degassing system, six-way injection valve, temperature regulated autosampler, column oven and Photodiode Array detector can be used [Agilent Technologies available through Ultra Scientific Inc., 250 Smith Street, North Kingstown, RI]. The column can be a Waters YMC30, 5-micron, 4.6 x 250 mm with a guard column of the same material [Waters, 34 Maple Street, Milford, MA]. The solvents for the mobile phase can be 81 methanol: 4 water: 15 tetrahydrofuran (THF) stabilized with 0.2% BHT (2,6-di-tert-butyl-4-methylphenol). Injections were 20 l. Separation can be isocratic at 30°C with a flow rate of 1.7 ml/minute. The peak responses can be measured by absorbance at 447 nm.
Carotenoid compositions can be determined for wild-type and mutant samples selected from those identified in a screening procedure. Petal samples can be stored in a - 80°C freezer until mutants were identified. Samples can be lyophilized, and the dried tissue can be stored under argon at -80°C until ready for analysis.
Extraction procedures can be performed under red light. Dried petals can be ground to pass through a No. 40 sieve mesh size. A ground sample can be accurately weighed and transferred into a 100 ml red volumetric flask. To the sample, 500 microliters. l) of H₂O can be added, and the mixture can be swirled for 1 minute. Thirty ml of extractant solvent (10 ml hexane + 7 ml acetone + 6 ml absolute alcohol + 7 ml toluene) can be added, and the flask can be shaken at 160 rpm for 10 minutes.
For saponification, 2 ml of 40% methanolic KOH can be added into the flask, which can be then swirled for one minute. The flask can be placed in a 56°C H₂O bath for 20 minutes. An air condenser can be attached to prevent loss of solvent. The sample can be cooled in the dark for one hour with the condenser attached. After cooling, 30 ml of hexane can be added, and the flask can be shaken at 160 rpm for 10 minutes.
The shaken sample can be diluted to volume (100 ml) with 10% sodium sulfate solution and shaken vigorously for one minute. The sample can be remained in the dark for at least 30 minutes. A 35 ml aliquot can be removed from the approximately 50 ml upper phase, and transferred to a sample cup. An additional 30 ml of hexane can be added into the flask that can be then shaken at 160 rpm for 10 minutes. After approximately one hour, the upper phases can be combined. For HPLC analysis, 10 ml aliquots can be dried under nitrogen and stored under argon at -80°C.
HPLC equipment comprised an Alliance 2690 equipped with a refrigerated autosampler, column heater and a Waters Photodiode Array 996 detector (Waters Corp., 34 Maple Street Milford, MA 01757). Separation can be obtained with a YMC30 column, 3 m, 2.0 x 150 mm with a guard column of the same material. Standards can be obtained from ICC Indorespective fine chemicals Somerville, New Jersey 088876 and from DHI-Water & Environment, DK -2970 Horsholm, Denmark.
The dried mutant samples can be resuspended in tetrahydrofuran and methanol to a total volume of 200 I and filtered, whereas the control can be not additionally concentrated. Carotenoids can be separated using a gradient method. Initial gradient conditions can be 90% methanol: 5% water: 5% methyl tert-butyl ether at a flow rate of 0.4 milliliters per minute (ml/min). From zero to 15 minutes, the mobile phase can be changed from the initial conditions to 80 methanol: 5 water: 15 methyl tert-butyl ether, and from 15 to 60 minutes to 20 methanol: 5 water: 75 methyl tert-butyl ether. For the following 10 minutes, the mobile phase can be returned to the initial conditions and the column equilibrated for an additional 10 minutes. The column temperature can be maintained at 27°C and the flow rate was 0.4 ml/minute. Injections were 10 I. The majority of peak responses can be measured at 450 nm and additional areas added from 286, 348, 400 and 472 nm extracted channels.

**[0459.0.10.10]** If required and desired, further chromatography steps with a suitable resin may follow. Advantageously, the xanthophylls can be further purified with a so-called RTHPLC. As eluent acetonitrile/water or chloroform/acetonitrile mixtures can be used. If necessary, these chromatography steps may be repeated, using identical or other chromatography resins. The skilled worker is familiar with the selection of suitable chromatography resin and the most effective use for a particular molecule to be purified.

**[0460.0.10.10]see [0460.0.0.0]**

**[0461.0.10.10]**Example 10: Cloning SEQ ID NO: 10215 for the expression in plants

**[0462.0.0.10] see [0462.0.0.0]**

**[0463.0.10.10]**SEQ ID NO: 10215 is amplified by PCR as described in the protocol of the Pfu Turbo or DNA Herculase polymerase (Stratagene).

**[0464.0.0.10]** to **[0466.0.0.10]: see [0464.0.0.0]** to **[0466.0.0.0]**

**[0466.1.0.10]** In case the Herculase enzyme can be used for the amplification, the PCR amplification cycles were as follows: 1 cycle of 2-3 minutes at 94°C, followed by 25-30 cycles of in each case 30 seconds at 94°C, 30 seconds at 55-60°C and 5-10 minutes at 72°C, followed by 1 cycle of 10 minutes at 72°C, then 4°C.

**[0467.0.10.10]**The following primer sequences were selected for the gene SEQ ID NO: 10215:
i) forward primer (SEQ ID NO: 10235)
   atgcgccctc ttattttatc gatttt
ii) reverse primer (SEQ ID NO: 10236)
   ttatggtgcg ggtttaagaa acgtc

**[0468.0.0.10] to [0479.0.0.10]: see [0468.0.0.0] to [0479.0.0.0]**

**[0480.0.10.10]**Example 11: Generation of transgenic plants which express SEQ ID NO: 10215

### [0481.0.0.10] to [0513.0.0.10]: see [0481.0.0.0] to [0513.0.0.0]

**[0514.0.10.10]** As an alternative, xanthophylls can be detected as described in Deli,J. & Molnar,P. Paprika carotenoids: Analysis, isolation, structure eucidation. Curr. Org. Chem. 6, 1197-1219 (2004) or Fraser,P.D., Pinto,M.E., Holloway,D.E. & Bramley,P.M. Technical advance: application of high-performance liquid chromatography with photodiode array detection to the metabolic profiling of plant isoprenoids. Plant J. 24, 551-558 (2000).
The results of the different plant analyses can be seen from the table 1 which follows:

**Table 1**

| **ORF** | **Metabolite** | **Method** | **Min** | **Max** |
|---|---|---|---|---|
| YCL040W | Cryptoxanthin | LC | 1,36 | 1,51 |
| b0986 | Cryptoxanthin | LC | 1,33 | 1,40 |
| b3684 | Cryptoxanthin | LC | 1,35 | 1,67 |
| b4401 | Cryptoxanthin | LC | 1,48 | 1,60 |
| b3926 | Cryptoxanthin | LC | 1.33 | 1.33 |
| b0851 | Cryptoxanthin | LC | 1.57 | 1.76 |
| b2211 | Cryptoxanthin | LC | 1.05 | 1.26 |
| b0050 | Cryptoxanthin | LC | 1.42 | 1.84 |
| YHR055C | Zeaxanthin | LC | 1,05 | 1,38 |
| b2699 | Zeaxanthin | LC | 1,25 | 1,48 |

**[0515.0.10.10]**to **[0552.0.0.10]: see [0515.0.0.0]** to **[0552.0.0.0]** including [0530.1.0.0] to [0530.6. 0.0] as well as [0552.1.0.0] and [0552.2.0.0]

**[0553.0.10.10]**
1. A process for the production of xanthopyhlls, which comprises
   (a) increasing or generating the activity of one or more proteins as indicated in Table II, columns 5 or 7, lines 103 to 106 and/or 468 to 471 or lines 107 and/or 108 or a functional equivalent thereof in a non-human organism, or in one or more parts thereof; and
   (b) growing the organism under conditions which permit the production of xanthopyhlls in said organism.
2. A process for the production of xanthopyhlls, comprising the increasing or generating in an organism or a part thereof the expression of at least one nucleic acid molecule comprising a nucleic acid molecule selected from the group consisting of:
   a) nucleic acid molecule encoding of a polypeptide as indicated in Table II, columns 5 or 7, lines 103 to 106 and/or 468 to 471 or lines 107 and/or 108 or a fragment thereof, which confers an increase in the amount of xanthopyhlls in an organism or a part thereof;
   b) nucleic acid molecule comprising of a nucleic acid molecule as indicated in Table I, columns 5 or 7, lines 103 to 106 and/or 468 to 471 or lines 107 and/or 108;
   c) nucleic acid molecule whose sequence can be deduced from a polypeptide sequence encoded by a nucleic acid molecule of (a) or (b) as a result of the degeneracy of the genetic code and conferring an increase in the amount of xanthopyhlls in an organism or a part thereof;
   d) nucleic acid molecule which encodes a polypeptide which has at least 50% identity with the amino acid sequence of the polypeptide encoded by the nucleic acid molecule of (a) to (c) and conferring an increase in the amount of xanthopyhlls in an organism or a part thereof;
   e) nucleic acid molecule which hybidizes with a nucleic acid molecule of (a) to (c) under under stringent hybridisation conditions and conferring an increase in the amount of xanthopyhlls in an organism or a part thereof;
   f) nucleic acid molecule which encompasses a nucleic acid molecule which is obtained by amplifying nucleic acid molecules from a cDNA library or a genomic library using the primers or primer pairs as indicated in Table III, columns 7, lines 103 to 106 and/or 468 to 471 or lines 107 and/or 108 and conferring an increase in the amount of xanthopyhllsrespective fine chemical in an organism or a part thereof;
   g) nucleic acid molecule encoding a polypeptide which is isolated with the aid of monoclonal antibodies against a polypeptide encoded by one of the nucleic acid molecules of (a) to (f) and conferring an increase in the amount of xanthopyhlls in an organism or a part thereof;
   h) nucleic acid molecule encoding a polypeptide comprising a consensus as indicated in Table IV, columns 7, lines 103 to 106 and/or 468 to 471 or lines 107 and/or 108 and conferring an increase in the amount of xanthopyhllsrespective fine chemical in an organism or a part thereof; and
   i) nucleic acid molecule which is obtainable by screening a suitable nucleic acid library under stringent hybridization conditions with a probe comprising one of the sequences of the nucleic acid molecule of (a) to (k) or with a fragment thereof having at least 15 nt, preferably 20 nt, 30 nt, 50 nt, 100 nt, 200 nt or 500 nt of the nucleic acid molecule characterized in (a) to (k) and conferring an increase in the amount of xanthopyhllsrespective fine chemical in an organism or a part thereof.
   or comprising a sequence which is complementary thereto.
3. The process of claim 1 or 2, comprising recovering of the free or bound xanthopyhlls.
4. The process of any one of claim 1 to 3, comprising the following steps:
   (a) selecting an organism or a part thereof expressing a polypeptide encoded by the nucleic acid molecule characterized in claim 2;
   (b) mutagenizing the selected organism or the part thereof;
   (c) comparing the activity or the expression level of said polypeptide in the mutagenized organism or the part thereof with the activity or the expression of said polypeptide of the selected organisms or the part thereof;
   (d) selecting the mutated organisms or parts thereof, which comprise an increased activity or expression level of said polypeptide compared to the selected organism or the part thereof;
   (e) optionally, growing and cultivating the organisms or the parts thereof; and
   (f) recovering, and optionally isolating, the free or bound xanthopyhlls produced by the selected mutated organisms or parts thereof.
5. The process of any one of claims 1 to 4, wherein the activity of said protein or the expression of said nucleic acid molecule is increased or generated transiently or stably.
6. An isolated nucleic acid molecule comprising a nucleic acid molecule selected from the group consisting of:
   a) nucleic acid molecule encoding of a polypeptide as indicated in Table II, columns 5 or 7, lines 103 to 106 and/or 468 to 471 or lines 107 and/or 108 or a fragment thereof, which confers an increase in the amount of xanthopyhlls in an organism or a part thereof;
   b) nucleic acid molecule comprising of a nucleic acid molecule as indicated in Table I, columns 5 or 7, lines 103 to 106 and/or 468 to 471 or lines 107 and/or 108;
   c) nucleic acid molecule whose sequence can be deduced from a polypeptide sequence encoded by a nucleic acid molecule of (a) or (b) as a result of the degeneracy of the genetic code and conferring an increase in the amount of xanthopyhlls in an organism or a part thereof;
   d) nucleic acid molecule which encodes a polypeptide which has at least 50% identity with the amino acid sequence of the polypeptide encoded by the nucleic acid molecule of (a) to (c) and conferring an increase in the amount of xanthopyhlls in an organism or a part thereof;
   e) nucleic acid molecule which hybidizes with a nucleic acid molecule of (a) to (c) under under stringent hybridisation conditions and conferring an increase in the amount of xanthopyhlls in an organism or a part thereof;
   f) nucleic acid molecule which encompasses a nucleic acid molecule which is obtained by amplifying nucleic acid molecules from a cDNA library or a genomic library using the primers or primer pairs as indicated in Table III, columns 7, lines 103 to 106 and/or 468 to 471 or lines 107 and/or 108 and conferring an increase in the amount of xanthopyhllsrespective fine chemical in an organism or a part thereof;
   g) nucleic acid molecule encoding a polypeptide which is isolated with the aid of monoclonal antibodies against a polypeptide encoded by one of the nucleic acid molecules of (a) to (f) and conferring an increase in the amount of xanthopyhlls in an organism or a part thereof;
   h) nucleic acid molecule encoding a polypeptide comprising a consensus as indicated in Table IV, columns 7, lines 103 to 106 and/or 468 to 471 or lines 107 and/or 108 and conferring an increase in the amount of xanthopyhllsrespective fine chemical in an organism or a part thereof; and
   i) nucleic acid molecule which is obtainable by screening a suitable nucleic acid library under stringent hybridization conditions with a probe comprising one of the sequences of the nucleic acid molecule of (a) to (k) or with a fragment thereof having at least 15 nt, preferably 20 nt, 30 nt, 50 nt, 100 nt, 200 nt or 500 nt of the nucleic acid molecule characterized in (a) to (k) and conferring an increase in the amount of xanthopyhllsrespective fine chemical in an organism or a part thereof.
   whereby the nucleic acid molecule distinguishes over the sequence as indicated in Table I A, columns 5 or 7, lines 103 to 106 and/or 468 to 471 or lines 107 and/or 108 by one or more nucleotides.
7. A nucleic acid construct which confers the expression of the nucleic acid molecule of claim 6, comprising one or more regulatory elements.
8. A vector comprising the nucleic acid molecule as claimed in claim 6 or the nucleic acid construct of claim 7.
9. The vector as claimed in claim 8, wherein the nucleic acid molecule is in operable linkage with regulatory sequences for the expression in a prokaryotic or eukaryotic, or in a prokaryotic and eukaryotic, host.
10. A host cell, which has been transformed stably or transiently with the vector as claimed in claim 8 or 9 or the nucleic acid molecule as claimed in claim 6 or the nucleic acid construct of claim 7 or produced as described in claim any one of claims 2 to 5.
11. The host cell of claim 10, which is a transgenic host cell.
12. The host cell of claim 10 or 11, which is a plant cell, an animal cell, a microorganism, or a yeast cell, a fungus cell, a prokaryotic cell, an eukaryotic cell or an archaebacterium.
13. A process for producing a polypeptide, wherein the polypeptide is expressed in a host cell as claimed in any one of claims 10 to 12.
14. A polypeptide produced by the process as claimed in claim 13 or encoded by the nucleic acid molecule as claimed in claim 6 whereby the polypeptide distinguishes over a sequence as indicated in Table II A, columns 5 or 7, lines 103 to 106 and/or 468 to 471 or lines 107 and/or 108 by one or more amino acids
15. An antibody, which binds specifically to the polypeptide as claimed in claim 14.
16. A plant tissue, propagation material, harvested material or a plant comprising the host cell as claimed in claim 12 which is plant cell or an Agrobacterium.
17. A method for screening for agonists and antagonists of the activity of a polypeptide encoded by the nucleic acid molecule of claim 6 conferring an increase in the amount of xanthopyhlls in an organism or a part thereof comprising:
   (a) contacting cells, tissues , plants or microorganisms which express the a polypeptide encoded by the nucleic acid molecule of claim 6 conferring an increase in the amount of xanthopyhlls in an organism or a part thereof with a candidate compound or a sample comprising a plurality of compounds under conditions which permit the expression the polypeptide;
   (b) assaying the linoleic acid level or the polypeptide expression level in the cell, tissue, plant or microorganism or the media the cell, tissue, plant or microorganisms is cultured or maintained in; and
   (c) identifying a agonist or antagonist by comparing the measured xanthopyhlls level or polypeptide expression level with a standard linoleic acid or polypeptide expression level measured in the absence of said candidate compound or a sample comprising said plurality of compounds, whereby an increased level over the standard indicates that the compound or the sample comprising said plurality of compounds is an agonist and a decreased level over the standard indicates that the compound or the sample comprising said plurality of compounds is an antagonist.
18. A process for the identification of a compound conferring increased xanthopyhlls production in a plant or microorganism, comprising the steps:
   (a) culturing a plant cell or tissue or microorganism or maintaining a plant expressing the polypeptide encoded by the nucleic acid molecule of claim 6 conferring an increase in the amount of xanthopyhlls in an organism or a part thereof and a readout system capable of interacting with the polypeptide under suitable conditions which permit the interaction of the polypeptide with said readout system in the presence of a compound or a sample comprising a plurality of compounds and capable of providing a detectable signal in response to the binding of a compound to said polypeptide under conditions which permit the expression of said readout system and of the polypeptide encoded by the nucleic acid molecule of claim 6 conferring an increase in the amount of xanthopyhlls in an organism or a part thereof;
   (b) identifying if the compound is an effective agonist by detecting the presence or absence or increase of a signal produced by said readout system.
19. A method for the identification of a gene product conferring an increase in xanthopyhlls production in a cell, comprising the following steps:
   (a) contacting the nucleic acid molecules of a sample, which can contain a candidate gene encoding a gene product conferring an increase in xanthopyhlls after expression with the nucleic acid molecule of claim 6;
   (b) identifying the nucleic acid molecules, which hybridise under relaxed stringent conditions with the nucleic acid molecule of claim 6;
   (c) introducing the candidate nucleic acid molecules in host cells appropriate for producing xanthopyhlls;
   (d) expressing the identified nucleic acid molecules in the host cells;
   (e) assaying the xanthopyhlls level in the host cells; and
   (f) identifying nucleic acid molecule and its gene product which expression confers an increase in the xanthopyhlls level in the host cell in the host cell after expression compared to the wild type.
20. A method for the identification of a gene product conferring an increase in xanthopyhlls production in a cell, comprising the following steps:
   (a) identifiying in a data bank nucleic acid molecules of an organism; which can contain a candidate gene encoding a gene product conferring an increase in the xanthopyhllsamount or level in an organism or a part thereof after expression, and which are at least 20% homolog to the nucleic acid molecule of claim 6;
   (b) introducing the candidate nucleic acid molecules in host cells appropriate for producing xanthopyhlls;
   (c) expressing the identified nucleic acid molecules in the host cells;
   (d) assaying the xanthopyhllslevel in the host cells; and
   (e) identifying nucleic acid molecule and its gene product which expression confers an increase in the xanthopyhlls level in the host cell after expression compared to the wild type.
21. A method for the production of an agricultural composition comprising the steps of the method of any one of claims 17 to 20 and formulating the compound identified in any one of claims 17 to 20 in a form acceptable for an application in agriculture.
22. A composition comprising the nucleic acid molecule of claim 6, the polypeptide of claim 14, the nucleic acid construct of claim 7, the vector of any one of claims 8 or 9, an antagonist or agonist identified according to claim 17, the compound of claim 18, the gene product of claim 19 or 20, the antibody of claim 15, and optionally an agricultural acceptable carrier.
23. Use of the nucleic acid molecule as claimed in claim 6 for the identification of a nucleic acid molecule conferring an increase of xanthopyhlls after expression.
24. Use of the polypeptide of claim 14 or the nucleic acid construct claim 7 or the gene product identified according to the method of claim 19 or 20 for identifying compounds capable of conferring a modulation of xanthopyhlls levels in an organism.
25. Cosmetic, pharmaceutical, food or feed composition comprising the nucleic acid molecule of claim 6, the polypeptide of claim 14, the nucleic acid construct of claim 7, the vector of claim 8 or 9, the antagonist or agonist identified according to claim 17, the antibody of claim 15, the plant or plant tissue of claim 16, the harvested material of claim 16, the host cell of claim 10 to 12 or the gene product identified according to the method of claim 19 or 20.
26. The method of any one of claims 1 to 5, the nucleic acid molecule of claim 6, the polypeptide of claim 14, the nucleic acid construct of claim 7, the vector of claim 8 or 9, the antagonist or agonist identified according to claim 17, the antibody of claim 15, the plant or plant tissue of claim 16, the harvested material of claim 16, the host cell of claim 10 to 12 or the gene product identified according to the method of claim 19 or 20, wherein the xanthylphyll is beta-cryptoxanthin or zeaxanthin, resp.
27. Use of the nucleic acid molecule of claim 6, the polypeptide of claim 14, the nucleic acid construct of claim 7, the vector of claim 8 or 9, the antagonist or agonist identified according to claim 17, the antibody of claim 15, the plant or plant tissue of claim 16, the harvested material of claim 16, the host cell of claim 10 to 12 or the gene product identified according to the method of claim 19 or 20 for the protection of a plant against a oxidative stress.
28. Use of the nucleic acid molecule of claim 6, the polypeptide of claim 14, the nucleic acid construct of claim 7, the vector of claim 8 or 9, the antagonist or agonist identified according to claim 17, the antibody of claim 15, the plant or plant tissue of claim 16, the harvested material of claim 16, the host cell of claim 10 to 12 or the gene product identified according to the method of claim 19 or 20 for the protection of a plant against a oxidative stress causing or a carotenoid synthesis inhibiting herbicide.
29. Use of the agonist identified according to claim 17, the plant or plant tissue of claim 16, the harvested material of claim 16, or the host cell of claim 10 to 12for the production of a cosmetic composition.

**[0554.0.0.10] Abstract: see [0554.0.0.0]**
Process for the production of fine chemicals

**[0001.0.0.12]** for the disclosure of this paragraph see [0001.0.0.0].

**[0002.0.12.12]** Sterols are a class of essential, natural compounds required by all eukaryotes to complete their life cycle. In animals, cholesterol is typically the major sterol while in fungi it is ergosterol. Plants produce a class of sterols called phytosterols. Phytosterols are natural components of many vegetables and grains. The term "phytosterols" covers plant sterols and plant stanols. Plant sterols are naturally occurring substances present in the diet as minor components of vegetable oils. The structures of these plant sterols are similar to that of cholesterol with an extra methyl or ethyl group and a double bond in the side chain. Saturated plant sterols, referred to as stanols, have no double bond in the ring structure.
Phytosterols (including plant sterols and stanols) are natural components of plant foods, especially plant oils, seeds and nuts, cereals and legumes specially of edible vegetable oils such as sunflower seed oil and, as such are natural constituents of the human diet. The most common phytosterols are beta-sitosterol, campesterol, and stigmasterol. Beta-sitosterol is found in high amounts in nuts.

**[0003.0.12.12]** A high concentration of cholesterol in serum, i.e., hypercholesterolemia, is a wellknown risk factor for coronary heart disease (CHD). Blood cholesterol levels can be decreased by following diets, which are low in saturated fat, high in polyunsaturated fat and low in cholesterol. Although considerable achievements have been made in terms of knowledge and education, consumers still find it difficult to follow healthy eating advice.
Both plant sterols and plant stanols are effective in lowering plasma total and low density lipoprotein (LDL) cholesterol and this occurs by inhibiting the absorption of cholesterol from the small intestine. The plasma cholesterol-lowering properties of plant sterols have been known since the 1950s (Pollak, Circulation, 7, 702-706.1953). They have been used as cholesterol-lowering agents, first in a free form (Pollak and Kritchevsky, Sitosterol. In: Monographs on Aherosclerosis. Clarkson TB, Kritchevsky D,Pollak OJ, eds. New York, Basel, Karger 1981; 1-219) and recently mainly as esterified phytosterols (Katan et al., Mayo Clin Proc 2003; 78: 965-978).
The consumption of plant sterols and plant stanols lowers blood cholesterol levels by inhibiting the absorption of dietary and endogenously-produced cholesterol from the small intestine and the plant sterols/stanols are only very poorly absorbed themselves. This inhibition is related to the similarity in physico-chemical properties of plant sterols and stanols and cholesterol. Plant sterols and plant stanols appear to be without hazard to health, having been shown without adverse effects in a large number of human studies. They show no evidence of toxicity even at high dose levels and gastro-intestinal absorption is low.

**[0004.0.12.12]** The most abundant sterols of vascular plants are campesterol, sitosterol and stigmasterol, all of which contain a double bond between the carbon atoms at positions 5 and 6 and are classified as delta-5 sterols.
Exemplary naturally occurring delta-5 plant sterols isofucosterol, sitosterol, stigmasterol, campesterol, cholesterol, and dihydrobrassicasterol. Exemplary naturally occurring non-delta-5 plant sterols are cycloartenol, 24-methylene cycloartenol, cycloeucalenol, and obtusifoliol.
The ratio of delta-5 to non-delta-5 sterols in plants can be an important factor relating to insect pest resistance. Insect pests are unable to synthesize de novo the steroid nucleus and depend upon external sources of sterols in their food source for production of necessary steroid compounds. In particular, insect pests require an external source of delta-5 sterols. By way of example, externally provided delta-5 sterols are necessary for the production of ecdysteroids, hormones that control reproduction and development. See, e.g., Costet et al., Proc. Natl. Acad. Sci. USA, 84:643 (1987) and Corio-Costet et al., Archives of Insect Biochem. Physiol., 11:47 (1989).

**[0005.0.12.12]** US 20020148006 and WO 98/45457 describes the modulation of phytosterol compositions to confer resistance to insects, nematodes, fungi and/or environmental stresses, and/or to improve the nutritional value of plants by using a DNA sequence encoding a first enzyme; which binds a first sterol and is preferably selected from the group consisting of S-adenosyl-L-methionine- ₂₄₍₂₅₎-sterol methyl transferase, a C-4 demethylase, a cycloeucalenol to obtusifoliol-isomerase, a 14- - demethylase, a ₈ to ₇-isomerase, a ₇-C-5-desaturase and a 24,25-reductase, and produces a second sterol and a 3' non-translated region which causes polyadenylation at the 3' end of the RNA.
WO 93/16187 discloses new plants containing in its genome one or more genes involved in the early stages of phytosterol biosynthesis, preferably the genes encode mevalonate kinase.
US 5,306,862, US 5,589,619, US 5,365,017, US 5,349,126 and US 20030150008 describe a method of increasing sterol (and squalene) accumulation in a plant based on an increased HMG-CoA reductase activity to increase the pest resistance of transgenic plants.
WO 97/48793 discloses a C-14 sterol reductase polypeptide for the genetic manipulation of a plant sterol biosynthetic pathway.
US 20040172680 disclose the use of a gene expressing a SMT1 (sterol methyltransferase) to increase the level of sterols in the seeds of plants. A DNA sequence encoding sterol methyltransferase isolated from Zea mays is disclosed in WO 00/08190. Bouvier-Nav et al in Eur. J. Biochem. 256, 88-96 (1988) describes two families of sterol methyl transferases (SMTs), The first (SMT1) applying to cycloartenol and the second (SMT2) to 24-methylene lophenol. Schaller et al (Plant Physiology (1998) 118: 461-169) describes the over-expression of SMT2 from Arabidopsis in tobacco resulting in a change in the ratio of 24-methyl cholesterol to sitosterol in the tobacco leaf.
US 6,723,837 and US 20040199940 disclose nucleic acid molecules encoding proteins and fragments of proteins associated with sterol and phytosterol metabolism as well as cells, that have been manipulated to contain increased levels or overexpress at least one sterol or phytosterol compound. The protein or fragment is selected from the group consisting of a HES1, HMGCoA reductase, squalene synthase, cycloartenol synthase, SMTI, SMTII and UPC, preferably from member of the KES1/HES1/OSH1 family of oxysterol-binding (OSBP) proteins comprising an oxysterol-binding protein consensus sequence--E(K, Q) xSH (H, R) PPx (S, T, A, C, F)A. One class of proteins, oxysterol-binding proteins, have been reported in humans and yeast (Jiang et al., Yeast 10: 341-353 (1994), the entirety of which is herein incorporated by reference). These proteins have been reported to modulate ergosterol levels in yeast (Jiang et al., Yeast 10: 341-353 (1994)). In particular, Jiang et al., reported three genes KES1, HES1 and OSH1, which encode proteins containing an oxysterol-binding region.

**[0006.0.12.12]** Transgenic plants having altered sterol profiles could be instrumental in establishing a dietary approach to cholesterol management and cardiovascular disease prevention. The altered phytosterol profile further leads to pest resistance.

**[0007.0.12.12]** Although people consume plant sterols every day in their normal diet, the amount is not great enough to have a significant blood cholesterol lowering effect. The intake of phytosterols varies among different populations according to the food products being consumed, but the average daily Western diet is reported to contain 150-300 mg of these sterols (de Vries et al., J Food Comp Anal 1997; 19: 115-141; Björkhem et al. Inborn errors in bile acid biosynthesis and storage of sterols other than cholesterol. In: The Metabolic and Molecular Bases of Inherited Disease. Scriver CS, Beaudet AL, Sly WS, Valle D, eds. New York, McGraw-Hill 2001; 2961-2988). In order to achieve a cholesterol-lowering benefit, approximately 1 g/day of plant sterols need to be consumed (Hendriks et al., European Journal of Clinical Nutrition, 53, 319-327.1999).

**[0008.0.12.12]** Phytosterols are found naturally in plant foods at low levels. The enrichment of foods such as margarines with plant sterols and stanols is one of the recent developments in functional foods to enhance the cholesterol-lowering ability of traditional food products. Incorporation of additional phytosterols into the diet may be an effective way of lowering total and LDL cholesterol levels. The non-esterified phytosterols can be used as novel food ingredients in:
(a) bakery products and cereals (eg, breakfast cereals, breakfast bars);
(b) dairy products such as low and reduced fat liquid milk, low and reduced fat yoghurt and yoghurt products, and dairy based desserts;
(c) non-carbonated soft drinks like low and reduced fat soy beverages and low and reduced fat soy-based yoghurts;
(d) meat products or edible fats and oils (eg, mayonnaise, spice sauces, salad dressings);
(e) margarine; and
table spreads or dietary fats.

**[0009.0.12.12]** When edible oils undergo normal refining, plant sterols are partially extracted. It is estimated that 2500 tonnes of vegetable oil needs to be refined to produce 1 tonne of plant sterols. Plant stanols are obtained by hydrogenation of the plant sterols.

**[0010.0.12.12]** Another source of plant sterols is tall oil, derived from the process of paper production from wood and approximately 2500 tonnes of pine is required to produce 1 tonne of plant sterols. Tall oil also contains a higher proportion of plant stanols (primarily b-sitostanol) than do vegetable oils.

**[0011.0.12.12]** As described above, phytosterols are used in a lot of different applications, for example in cosmetics, pharmaceuticals and in feed and food. Therefore improving the quality of foodstuffs and animal feeds is an important task of the food-and-feed industry. Especially advantageous for the quality of foodstuffs and animal feeds is as balanced as possible a sterol profile in the diet.

**[0012.0.12.12]** Accordingly, there is still a great demand for new and more suitable genes which encode enzymes which participate in the biosynthesis of phytosterols and make it possible to produce them specifically on an industrial scale without unwanted byproducts forming. In the selection of genes for or regulators of biosynthesis two characteristics above all are particularly important. On the one hand, there is as ever a need for improved processes for obtaining the highest possible contents of phytosterols; on the other hand as less as possible by products should be produced in the production process.

**[0013.0.0.12]** for the disclosure of this paragraph see [0013.0.0.0] above.

**[0014.0.12.12]** Accordingly, in a first embodiment, the invention relates to a process for the production of a fine chemical, whereby the fine chemical is/are phytosterols. The term "phytosterols" covers plant sterols and plant stanols. Accordingly, in the present invention, the term "the fine chemical" as used herein relates to "phytosterols". Further, the term "the fine chemicals" as used herein also relates to fine chemicals comprising plant sterols and plant stanols, preferably beta-sitosterol, campesterol, and/or stigmasterol.

**[0015.0.12.12]** In one embodiment, the term "the fine chemical" means phytosterols, plant sterols and plant stanols. Throughout the specification the term "the fine chemical" means phytosterols and ester, thioester or sterols in free form or bound to other compounds. For the purpose of this description, the term sterol/stanol refers both to free sterols/stanols and conjugated sterols/stanols, for example, where the 3-hydroxy group is esterified by a fatty acid chain or phenolic acid to give a steryl/stanyl ester. As used herein, the term "phytosterol" includes all phytosterols without limitation, for example: sitosterol, campesterol, stigmasterol, brassicasterol, desmosterol, chalinosterol, poriferasterol, clionasterol, the corresponding stanols and all natural or synthesized forms and derivatives thereof, including isomers. It is to be understood that modifications to the phytosterols i.e. to include side chains also falls within the purview of this invention. All those derivates forms are summarized as "conjugates". In an preferred embodiment, the term "the fine chemical" or the term "phytosterol" or the term "the respective fine chemical" means at least one chemical compound plant sterols and plant stanols selected from the group "beta-sitosterol, sitostanol, stigmasterol, brassicasterol, campestanol, isofucosterol and campesterol", preferred "beta-sitosterol, campesterol, and/or stigmasterol", most preferred "beta-sitosterol and/or campesterol". Also preferably, are esters of sterols/stanols with C10-24 fatty acids.
Increased phytosterol content normally means an increased total phytosterol content. However, an increased phytosterol content also means, in particular, a modified content of the above-described compounds ("beta-sitosterol, sitostanol, stigmasterol, brassicasterol, campestanol, isofucosterol and campesterol") with phytosterol activity, without the need for an inevitable increase in the total phytosterol content. **[0016.0.12.12]** Accordingly, the present invention relates to a process for the production of phytosterol comprising
(a) increasing or generating the activity of one or more
   YER156C, YKR057W, YOR084W, b0019, b0421, b2699, b0050, b0161 and/or b4129 protein(s) and/or
   YER156C, YER173W, YKR057W, YOR044W, YPR172W, b2699, b3256, b0161, b0464, b1896, b2341, b2478 and/or b2822 protein(s) in a non-human organism in one or more parts thereof and
(b) growing the organism under conditions which permit the production of the fine chemical, thus, phytosterol, plant sterol and stanol, preferably beta-sitosterol, sitostanol, stigmasterol, brassicasterol, campestanol, isofucosterol and campesterol, more preferred beta-sitosterol, campesterol, and/or stigmasterol, most preferred beta-sitosterol and/or campesterol or fine chemicals comprising phytosterol, in said organism.
Accordingly, the present invention relates to a process for the production of phytosterol comprising
(a) increasing or generating the activity of one or more proteins having the activity of a protein indicated in Table II, column 3, lines 112 to 124 and/or lines 483 to 491 or having the sequence of a polypeptide encoded by a nucleic acid molecule indicated in Table I, column 5 or 7, lines 112 to 124 and/or lines 483 to 491, in a non-human organism in one or more parts thereof and
(b) growing the organism under conditions which permit the production of the fine chemical, thus, phytosterol, in particular beta-sitosterol and/or campesterol.

**[0016.1.12.12]** Accordingly, the term "the fine chemical" means in one embodiment "beta-sitosterol " in relation to all sequences listed in Table I to IV, lines 112 to 117 and/or 483 to 485 or homologs thereof and means in one embodiment "campesterol" in relation to all sequences listed in Tables I to IV, lines 118 to 124 and/or 486 to 491 or homologs thereof.
Accordingly, in one embodiment the term "the fine chemical" means "beta-sitosterol" and "campesterol" in relation to all sequences listed in Table I to IV, lines 113 or 120 and 120; in one embodiment the term "the fine chemical" means "beta-sitosterol" and "campesterol" in relation to all sequences listed in Table I to IV, lines 114 or 122; in one embodiment the term "the fine chemical" means "beta-sitosterol" and "campesterol" in relation to all sequences listed in Table I to IV, lines 115 or 124, in one embodiment the term "the fine chemical" means "beta-sitosterol" and "campesterol" in relation to all sequences listed in Table I to IV, lines 484 or 486.
Accordingly, the term "the fine chemical" can mean "beta-sitosterol" and "campesterol", owing to circumstances and the context. In order to illustrate that the meaning of the term "the fine chemical" means ""beta-sitosterol" and "campesterol" the term "the respective fine chemical" is also used.

**[0017.0.0.12] and [0018.0.0.12]** for the disclosure of the paragraphs [0017.0.0.12] and [0018.0.0.12] see paragraphs [0017.0.0.0] and [0018.0.0.0] above.

**[0019.0.12.12]** Advantageously the process for the production of the respective fine chemical leads to an enhanced production of the respective fine chemical. The terms "enhanced" or "increase" mean at least a 10%, 20%, 30%, 40% or 50%, preferably at least 60%, 70%, 80%, 90% or 100%, more preferably 150%, 200%, 300%, 400% or 500% higher production of the fine respective chemical in comparison to the reference as defined below, e.g. that means in comparison to an organism without the aforementioned modification of the activity of a protein indicated in Table II, column 3, lines 112 to 124 and/or lines 483 to 491 or encoded by nucleic acid molecule indicated in Table I, columns 5 or 7, lines 112 to 124 and/or lines 483 to 491.

**[0020.0.12.12]** Surprisingly it was found, that the transgenic expression of the Saccaromyces cerevisiae protein YER156C, YKR057W and/or YOR084W and/or the Escherichia coli K12 protein b0019, b0421, b2699, b0050, b0161 and/or b4129 in Arabidopsis thaliana conferred an increase in the beta-sitosterol content of the transformed plants.
Surprisingly it was found, that the transgenic expression of the Saccaromyces cerevisiae protein YER156C, YER173W, YKR057W, YOR044W and/or YPR172W and/or the Escherichia coli K12 protein b2699, b3256, b0161, b0464, b1896, b2341, b2478 and/or b2822 in Arabidopsis thaliana conferred an increase in campesterol content of the transformed plants.
Accordingly, it was surprisingly found, that the transgenic expression of the Saccaromyces cerevisiae protein YER156C in Arabidopsis thaliana conferred an increase in beta-sitosterol and/or campesterol (or the respective fine chemical) content of the transformed plants. Thus, in one embodiment, said protein or its homologs are used for the production of beta-sitosterol; in one embodiment, said protein or its homologs are used for the production of campesterol; in one embodiment, said protein or its homologs are used for the production of beta-sitosterol and campesterol.
Accordingly, it was surprisingly found, that the transgenic expression of the Saccaromyces cerevisiae protein YKR057W in Arabidopsis thaliana conferred an increase in beta-sitosterol and/or campesterol (or the respective fine chemical) content of the transformed plants. Thus, in one embodiment, said protein or its homologs are used for the production of beta-sitosterol; in one embodiment, said protein or its homologs are used for the production of campesterol; in one embodiment, said protein or its homologs are used for the production of beta-sitosterol and campesterol.
Accordingly, it was surprisingly found, that the transgenic expression of the Escherichia coli K12 protein b2699 in Arabidopsis thaliana conferred an increase in beta-sitosterol and/or campesterol (or the respective fine chemical) content of the transformed plants. Thus, in one embodiment, said protein or its homologs are used for the production of beta-sitosterol; in one embodiment, said protein or its homologs are used for the production of campesterol; in one embodiment, said protein or its homologs are used for the production of beta-sitosterol and campesterol.
Accordingly, it was surprisingly found, that the transgenic expression of the Escherichia coli K12 protein b0161 in Arabidopsis thaliana conferred an increase in beta-sitosterol and/or campesterol (or the respective fine chemical) content of the transformed plants. Thus, in one embodiment, said protein or its homologs are used for the production of beta-sitosterol; in one embodiment, said protein or its homologs are used for the production of campesterol; in one embodiment, said protein or its homologs are used for the production of beta-sitosterol and campesterol.

**[0021.0.0.12]** for the disclosure of this paragraph see [0021.0.0.0] above.

**[0022.0.12.12]** The sequence of YER156C from Saccharomyces cerevisiae has been published in Dietrich, et al.(Nature 387 (6632 Suppl), 78-81 (1997)), and its activity has not been characterized yet. It seems to have an activity similar to Arabidopsis thaliana hypothetical protein F2K15.180. Accordingly, in one embodiment, the process of the present invention comprises the use of a protein with the activity of a YER156C from Saccharomyces cerevisiae or of a Arabidopsis thaliana hypothetical protein F2K15.180 or its homolog, e.g. as shown herein, for the production of the respective fine chemical, meaning of phytosterol, e.g. beta-sitosterol and/or campesterol and/or conjugates, preferably in free or bound form in an organism or a part thereof, as mentioned. In one further embodiment the YER156C protein expression is increased together with the increase of another gene of the phytosterol biosynthesis pathway, preferably with a gene encoding a protein being involved in the production of phytosterol from the intermediates like squalene and squalene epoxide or cycloartenol. In one embodiment, in the process of the present invention said activity, e.g. of a YER156C protein is increased or generated, e.g. from Saccharomyces cerevisiae or a homolog thereof. The sequence of YER173W from Saccharomyces cerevisiae has been published in Dietrich et al. (Nature 387 (6632 Suppl), 78-81, 1997), and Goffeau et al. (Science 274 (5287), 546-547, 1996), and its activity is being defined as an "Checkpoint protein, involved in the activation of the DNA damage and meiotic pachytene checkpoints". Accordingly, in one embodiment, the process of the present invention comprises the use of a YER173W activity from Saccharomyces cerevisiae or its homolog, e.g. as shown herein, for the production of phytosterol, meaning of campesterol and/or conjugates, in particular for increasing the amount of campesterol and/or conjugates, preferably campesterol in free or bound form in an organism or a part thereof, as mentioned. In one further embodiment the YER173W protein expression is increased together with the increase of another gene of the phytosterol biosynthesis pathway, preferably with a gene encoding a protein being involved in the production of phytosterol from the intermediates like squalene and squalene epoxide or cycloartenol. In one embodiment, in the process of the present invention the activity of a YER173W protein is increased or generated, e.g. from Saccharomyces cerevisiae or a homolog thereof.
The sequence of YKR057W from Saccharomyces cerevisiae has been published in Dujon et al., Nature 369 (6479), 371-378, 1994 and Goffeau et al., Science 274 (5287), 546-547, 1996 and its activity is being defined as a ribosomal protein, similar to S21 ribosomal proteins, involved in ribosome biogenesis and translation. Accordingly, in one embodiment, the process of the present invention comprises the use of a protein involved in the ribosome biogenesis and translation, in particular of the superfamiliy of the ribosomal protein, preferably having a S21 ribosomal protein activity or its homolog, e.g. as shown herein, for the production of the respective fine chemical, meaning of phytosterol, e.g. beta-sitosterol and/or campesterol and/or conjugates, preferably in free or bound form in an organism or a part thereof, as mentioned. In one further embodiment the YKR057W protein expression is increased together with the increase of another gene of the phytosterol biosynthesis pathway, preferably with a gene encoding a protein being involved in the production of phytosterol from the intermediates like squalene and squalene epoxide or cycloartenol. In one embodiment, in the process of the present invention said activity, e.g. of a protein of the yeast ribosomal protein superfamily or preferably having a activity involved in ribosome biogenesis and translation is increased or generated, e.g. from Saccharomyces cerevisiae or a homolog thereof.
The sequence of YOR044W from Saccharomyces cerevisiae has been published in Dujon et al., Nature 387 (6632 Suppl), 98-102 (1997), and its activity has not been characterized yet. Accordingly, in one embodiment, the process of the present invention comprises the use of a protein with the activity of a YOR044w from Saccharomyces cerevisiae or of a Saccharomyces cerevisiae probable membrane protein YOR044w or its homolog, e.g. as shown herein, for the production of the respective fine chemical, meaning of phytosterol, e.g. campesterol and/or conjugates, preferably in free or bound form in an organism or a part thereof, as mentioned. In one further embodiment the YOR044W protein expression is increased together with the increase of another gene of the phytosterol biosynthesis pathway, preferably with a gene encoding a protein being involved in the production of phytosterol from the intermediates like squalene and squalene epoxide or cycloartenol. In one embodiment, in the process of the present invention said activity, e.g. of a YOR044W protein is increased or generated, e.g. from Saccharomyces cerevisiae or a homolog thereof. The sequence of YOR084W from Saccharomyces cerevisiae has been published in Dujon, Nature 387 (6632 Suppl), 98-102, 1997, and Goffeau, Science 274 (5287), 546-547, 1996, and its activity is being defined as a putative lipase of the peroxisomal matrix. Accordingly, in one embodiment, the process of the present invention comprises the use of a putative lipase of the peroxisomal matrix protein or its homolog, e.g. as shown herein, for the production of the respective fine chemical, meaning of phytosterol, e.g. beta-sitosterol and/or conjugates, preferably in free or bound form in an organism or a part thereof, as mentioned. In one further embodiment the YOR084W protein expression is increased together with the increase of another gene of the phytosterol biosynthesis pathway, preferably with a gene encoding a protein being involved in the production of phytosterol from the intermediates like squalene and squalene epoxide or cycloartenol. In one embodiment, in the process of the present invention said activity, e.g. of a a putative lipase of the peroxisomal matrix protein is increased or generated, e.g. from Saccharomyces cerevisiae or a homolog thereof. The sequence of YPR172W from Saccharomyces cerevisiae has been published in Bussey et al., Nature 387 (6632 Suppl), 103-105 (1997), and its activity has not been characterized yet. Accordingly, in one embodiment, the process of the present invention comprises the use of a protein with the activity of a YPR172W from Saccharomyces cerevisiae or its homolog, e.g. as shown herein, for the production of the respective fine chemical, meaning of phytosterol, e.g. campesterol and/or conjugates, preferably in free or bound form in an organism or a part thereof, as mentioned. In one further embodiment the YPR172W protein expression is increased together with the increase of another gene of the phytosterol biosynthesis pathway, preferably with a gene encoding a protein being involved in the production of phytosterol from the intermediates like squalene and squalene epoxide or cycloartenol. In one embodiment, in the process of the present invention said activity, e.g. of a YPR172W protein is increased or generated, e.g. from Saccharomyces cerevisiae or a homolog thereof.
The sequence of b0019 from Escherichia coli K12 has been published in Blattner et al., Science 277(5331), 1453-1474, 1997, and its activity is being defined as protein for the transport of small molecules, preferably cations, meaning a Na+/H+ antiporter protein. In a more preferred embodiment the protein has the activity of a Na+/H+ antiporter, responsive to stress, especially to high salinity and pH. Accordingly, in one embodiment, the process of the present invention comprises the use of a transport protein, in particular of the superfamiliy of Na+/H+-exchanging protein nhaA, preferably having a Na+/H+ antiporter activity from E. coli or its homolog, e.g. as shown herein, for the production of the respective fine chemical, meaning of phytosterol, e.g. beta-sitosterol and/or conjugates, preferably in free or bound form in an organism or a part thereof, as mentioned. In one further embodiment the b0019 protein expression is increased together with the increase of another gene of the phytosterol biosynthesis pathway, preferably with a gene encoding a protein being involved in the production of phytosterol from the intermediates like squalene and squalene epoxide or cycloartenol. In one embodiment, in the process of the present invention said activity, e.g. of a transport protein, in particular of the superfamiliy of Na+/H+-exchanging protein nhaA, preferably having a Na+/H+ antiporter activity from E. coli is increased or generated, e.g. from E. coli or a homolog thereof.
The sequence of b0421 from Escherichia coli K12 has been published in Blattner et al., Science 277(5331), 1453-1474, 1997, and its activity is being defined as geranyltranstransferase (=farnesyldiphosphate synthase). Accordingly, in one embodiment, the process of the present invention comprises the use of a geranyltranstransferase (=farnesyldiphosphate synthase), in particular of the superfamiliy of dimethylallyltrans-transferase, geranyltranstransferase from E. coli or its homolog, e.g. as shown herein, for the production of the respective fine chemical, meaning of phytosterol, e.g. beta-sitosterol and/or conjugates, preferably in free or bound form in an organism or a part thereof, as mentioned. In one further embodiment the b0421 protein expression is increased together with the increase of another gene of the phytosterol biosynthesis pathway, preferably with a gene encoding a protein being involved in the production of phytosterol from the intermediates like squalene and squalene epoxide or cycloartenol. In one embodiment, in the process of the present invention said activity, e.g. of a geranyltranstransferase (=farnesyldiphosphate synthase), in particular of the superfamiliy of dimethylallyltrans-transferase, geranyltranstransferase is increased or generated, e.g. from E. coli or a homolog thereof.
The sequence of b2699 from Escherichia coli K12 has been published in Blattner et al., Science 277(5331), 1453-1474, 1997, and its activity is being defined as a DNA strand exchange and recombination protein with protease and nuclease activity of the superfamily of the recombination protein recA. Accordingly, in one embodiment, the process of the present invention comprises the use of a protein with a DNA recombination and DNA repair activity, a pheromone response activity, a mating-type determination activity, a sex-specific protein activity, a nucleotide binding activity and/or a protease and nuclease activity, in particular a DNA strand exchange and recombination protein with protease and nuclease activity, in particular of the superfamily of the recombination protein recA from E. coli or its homolog, e.g. as shown herein, for the production of the respective fine chemical, meaning of phytosterol, in particular for increasing the amount of beta-sitosterol and/or campesterol and/or conjugates, preferably in free or bound form in an organism or a part thereof, as mentioned. In one further embodiment the b2699 protein expression is increased together with the increase of another gene of the phytosterol biosynthesis pathway, preferably with a gene encoding a protein being involved in the production of phytosterol from the intermediates like squalene and squalene epoxide or cycloartenol. In one embodiment, in the process of the present invention said activity, e.g. the activity of a protease and nuclease activity, in particular a DNA strand exchange and recombination protein with protease and nuclease activity, in particular of the superfamily of the recombination protein recA is increased or generated, e.g. from E. coli or a homolog thereof.
The sequence of b3256 from Escherichia coli K12 has been published in Blattner et al., Science 277(5331), 1453-1474, 1997, and its activity is being defined as acetyl CoA carboxylase, biotin carboxylase subunit. Accordingly, in one embodiment, the process of the present invention comprises the use of a acetyl CoA carboxylase, in particular of the superfamiliy of biotin carboxylase from E. coli or its homolog, e.g. as shown herein, for the production of the respective fine chemical, meaning of phytosterol, in particular for increasing the amount of campesterol and/or conjugates, preferably in free or bound form in an organism or a part thereof, as mentioned. In one further embodiment the b3256 protein expression is increased together with the increase of another gene of the phytosterol biosynthesis pathway, preferably with a gene encoding a protein being involved in the production of phytosterol from the intermediates like squalene and squalene epoxide or cycloartenol. In one embodiment, in the process of the present invention said activity, e.g. the activity of a acetyl CoA carboxylase, in particular of the superfamiliy of biotin carboxylase is increased or generated, e.g. from E. coli or a homolog thereof.
The sequence of b0050 (Accession number NP_414592) from Escherichia coli K12 has been published in Blattner et al., Science 277 (5331), 1453-1474, 1997, and its activity is being defined as a conserved protein potentially involved in protein protein interaction. Accordingly, in one embodiment, the process of the present invention comprises the use of said protein from E. coli or its homolog, e.g. as shown herein, for the production of the fine chemical, meaning of phytosterol, e.g. beta-sitosterol and/or conjugates, preferably in free or bound form in an organism or a part thereof, as mentioned. In one further embodiment the b0050 protein expression is increased together with the increase of another gene of the phytosterol biosynthesis pathway, preferably with a gene encoding a protein being involved in the production of phytosterol from the intermediates like squalene and squalene epoxide or cycloartenol. In one embodiment, in the process of the present invention the activity of said protein is increased or generated, e.g. from E. coli or a homolog thereof.
The sequence of b0161 (Accession number NP_414703) from Escherichia coli K12 has been published in Blattner et al., Science 277 (5331), 1453-1474, 1997, and its activity is being defined as a periplasmic serine protease (heat shock protein). Accordingly, in one embodiment, the process of the present invention comprises the use of a periplasmic serine protease from E. coli or its homolog, e.g. as shown herein, for the production of the fine chemical, meaning of phytosterol, e.g. beta-sitosterol and/or campesterol and/or conjugates, preferably in free or bound form in an organism or a part thereof, as mentioned. In one further embodiment the b0161 protein expression is increased together with the increase of another gene of the phytosterol biosynthesis pathway, preferably with a gene encoding a protein being involved in the production of phytosterol from the intermediates like squalene and squalene epoxide or cycloartenol. In one embodiment, in the process of the present invention the activity of a periplasmic serine protease is increased or generated, e.g. from E. coli or a homolog thereof.
The sequence of b4129 (Accession number NP_418553) from Escherichia coli K12 has been published in Blattner et al., Science 277 (5331), 1453-1474, 1997, and its activity is being defined as a lysine tRNA synthetase. Accordingly, in one embodiment, the process of the present invention comprises the use of a lysine tRNA synthetase protein from E. coli or its homolog, e.g. as shown herein, for the production of the fine chemical, meaning of phytosterol, e.g. beta-sitosterol and/or conjugates, preferably in free or bound form in an organism or a part thereof, as mentioned. In one further embodiment the b4129 protein expression is increased together with the increase of another gene of the phytosterol biosynthesis pathway, preferably with a gene encoding a protein being involved in the production of phytosterol from the intermediates like squalene and squalene epoxide or cycloartenol. In one embodiment, in the process of the present invention the activity of a lysine tRNA synthetase protein is increased or generated, e.g. from E. coli or a homolog thereof.
The sequence of b0464 (Accession number NP_414997) from Escherichia coli K12 has been published in Blattner, Science 277(5331), 1453-1474, 1997, and its activity is being defined as a"transcriptional repressor for multidrug efflux pump (TetR/AcrR family)". Accordingly, in one embodiment, the process of the present invention comprises the use of a "transcriptional repressor for multidrug efflux pump (TetR/AcrR family)" from E. coli or its homolog, e.g. as shown herein, for the production of the fine chemical, meaning of phytosterol, in particular for increasing the amount of campesterol and/or conjugates, preferably in free or bound form in an organism or a part thereof, as mentioned. In one further embodiment the b0464 protein expression is increased together with the increase of another gene of the phytosterol biosynthesis pathway, preferably with a gene encoding a protein being involved in the production of phytosterol from the intermediates like squalene and squalene epoxide or cycloartenol. In one embodiment, in the process of the present invention the activity of a "transcriptional repressor for multidrug efflux pump (TetR/AcrR family)" is increased or generated, e.g. from E. coli or a homolog thereof.
The sequence of b1896 (Accession number NP_416410) from Escherichia coli K12 has been published in Blattner et al., Science 277(5331), 1453-1474, 1997, and its activity is being defined as a trehalose-6-phosphate synthase. Accordingly, in one embodiment, the process of the present invention comprises the use of a trehalose-6-phosphate synthase from E. coli or its homolog, e.g. as shown herein, for the production of the fine chemical, meaning of phytosterol, in particular for increasing the amount of campesterol and/or conjugates, preferably in free or bound form in an organism or a part thereof, as mentioned. In one further embodiment the b1896 protein expression is increased together with the increase of another gene of the phytosterol biosynthesis pathway, preferably with a gene encoding a protein being involved in the production of phytosterol from the intermediates like squalene and squalene epoxide or cycloartenol. In one embodiment, in the process of the present invention the activity of a trehalose-6-phosphate synthase is increased or generated, e.g. from E. coli or a homolog thereof.
The sequence of b2341 (Accession number NP_416410) from Escherichia coli K12 has been published in Blattner et al., Science 277(5331), 1453-1474, 1997, and its activity is being defined as a "bifunctional anaerobic fatty acid oxidation complex protein: enoyl-CoA hydratase/epimerase/isomerase (N-terminal); 3-hydroxyacyl-CoA dehydrogenase (C-terminal)". Accordingly, in one embodiment, the process of the present invention comprises the use of a bifunctional anaerobic fatty acid oxidation complex protein: enoyl-CoA hydratase/epimerase/isomerase (N-terminal); 3-hydroxyacyl-CoA dehydrogenase (C-terminal) from E. coli or its homolog, e.g. as shown herein, for the production of the fine chemical, meaning of phytosterol, in particular for increasing the amount of campesterol and/or conjugates, preferably in free or bound form in an organism or a part thereof, as mentioned. In one further embodiment the b2341 protein expression is increased together with the increase of another gene of the phytosterol biosynthesis pathway, preferably with a gene encoding a protein being involved in the production of phytosterol from the intermediates like squalene and squalene epoxide or cycloartenol. In one embodiment, in the process of the present invention the activity of a bifunctional anaerobic fatty acid oxidation complex protein: enoyl-CoA hydratase/epimerase/isomerase (N-terminal); 3-hydroxyacyl-CoA dehydrogenase (C-terminal) is increased or generated, e.g. from E. coli or a homolog thereof.
The sequence of b2478 (Accession number NP_416973) from Escherichia coli K12 has been published in Blattner et al., Science 277(5331), 1453-1474, 1997, and its activity is being defined as a "dihydrodipicolinate synthase". Accordingly, in one embodiment, the process of the present invention comprises the use of a dihydrodipicolinate synthase from E. coli or its homolog, e.g. as shown herein, for the production of the fine chemical, meaning of phytosterol, in particular for increasing the amount of campesterol and/or conjugates, preferably in free or bound form in an organism or a part thereof, as mentioned. In one further embodiment the b2478 protein expression is increased together with the increase of another gene of the phytosterol biosynthesis pathway, preferably with a gene encoding a protein being involved in the production of phytosterol from the intermediates like squalene and squalene epoxide or cycloartenol. In one embodiment, in the process of the present invention the activity of a dihydrodipicolinate synthase is increased or generated, e.g. from E. coli or a homolog thereof.
The sequence of b2822 (Accession number NP_417299) from Escherichia coli K12 has been published in Blattner et al., Science 277 (5331), 1453-1474, 1997, and its activity is being defined as a DNA helicase, ATP-dependent dsDNA/ssDNA exonuclease V subunit, ssDNA endonuclease. Accordingly, in one embodiment, the process of the present invention comprises the use of a DNA helicase, ATP-dependent dsDNA/ssDNA exonuclease V subunit, ssDNA endonuclease protein from E. coli or its homolog, e.g. as shown herein, for the production of the fine chemical, meaning of phytosterol, in particular for increasing the amount of campesterol and/or conjugates, preferably in free or bound form in an organism or a part thereof, as mentioned. In one further embodiment the b2822 protein expression is increased together with the increase of another gene of the phytosterol biosynthesis pathway, preferably with a gene encoding a protein being involved in the production of phytosterol from the intermediates like squalene and squalene epoxide or cycloartenol. In one embodiment, in the process of the present invention the activity of a DNA helicase, ATP-dependent dsDNA/ssDNA exonuclease V subunit, ssDNA endonuclease protein is increased or generated, e.g. from E. coli or a homolog thereof.

**[0023.0.12.12]** Homologues ( = homologs) of the present gene products can be derived from any organisms as long as the homologue confers the herein mentioned activity, in particular, confers an increase in the respective fine chemical amount or content.
In one embodiment, the homolog of the any one of the polypeptides indicated in Table II, column 3, line 112, 113 or 114, resp. is a homolog having the same or a similar activity, resp. In particular an increase of activity confers an increase in the content of the respective fine chemical in the organisms, preferably of beta-sitosterol. In one embodiment, the homolog is a homolog with a sequence as indicated in Table I or II, column 7, line 112, 113 or 114, resp. In one embodiment, the homolog of one of the polypeptides indicated in Table II, column 3, line 112, 113 or 114 resp., is derived from an eukaryotic. In one embodiment, the homolog is derived from Fungi. In one embodiment, the homolog of a polypeptide indicated in Table II, column 3, line 112, 113 or 114, resp., is derived from Ascomycota. In one embodiment, the homolog of a polypeptide indicated in Table II, column 3, line 112, 113 or 114 resp., is derived from Saccharomycotina. In one embodiment, the homolog of a polypeptide indicated in Table II, column 3, line 112, 113 or 114, resp., is derived from Saccharomycetes. In one embodiment, the homolog of a polypeptide indicated in Table II, column 3, line 112, 113 or 114, resp., is a homolog being derived from Saccharomycetales. In one embodiment, the homolog of a polypeptide indicated in Table II, column 3, line 112, 113 or 114, resp., is a homolog having the same or a similar activity being derived from Saccharomycetaceae. In one embodiment, the homolog of a polypeptide indicated in Table II, column 3, line 112, 113 or 114 resp., is a homolog having the same or a similar activity being derived from Saccharomyces.
In one embodiment, the homolog of the any one of the polypeptides indicated in Table II, column 3, lines 118 to 122 is a homolog having the same or a similar activity. In particular an increase of activity confers an increase in the content of the respective fine chemical in the organisms, preferably of phytosterol, preferably of campesterol. In one embodiment, the homolog is a homolog with a sequence as indicated in Table I or II, column 7, lines 118 to 122. In one embodiment, the homolog of one of the polypeptides indicated in Table II, column 3, lines 118 to 122, is derived from an eukaryotic. In one embodiment, the homolog is derived from Fungi. In one embodiment, the homolog of a polypeptide indicated in Table II, column 3, lines 118 to 122 , is derived from Ascomycota. In one embodiment, the homolog of a polypeptide indicated in Table II, column 3,lines 118 to 122, is derived from Saccharomycotina. In one embodiment, the homolog of a polypeptide indicated in Table II, column 3, lines 118 to 122, is derived from Saccharomycetes. In one embodiment, the homolog of a polypeptide indicated in Table II, column 3, lines 118 to 122, is a homolog being derived from Saccharomycetales. In one embodiment, the homolog of a polypeptide indicated in Table II, column 3, lines 118 to 122, is a homolog having the same or a similar activity being derived from Saccharomycetaceae. In one embodiment, the homolog of a polypeptide indicated in Table II, column 3, lines 118 to 122, is a homolog having the same or a similar activity being derived from Saccharomyces.
In one embodiment, the homolog of the any one of the polypeptides indicated in Table II, column 3, lines 115, 116 or 117 and 483 to 485 is a homolog having the same or a similar activity. In particular an increase of activity confers an increase in the content of the respective fine chemical in the organisms, preferably of phytosterol, more preferably beta-sitosterol. In one embodiment, the homolog is a homolog with a sequence as indicated in Table I or II, column 7, lines 115, 116 or 117 and 483 to 485, resp. In one embodiment, the homolog of one of the polypeptides indicated in Table II, column 3, 115, 116 or 117 and 483 to 485 is derived from a bacteria. In one embodiment, the homolog of a polypeptide indicated in Table II, column 3, lines 115, 116 or 117 and 483 to 485 is derived from Proteobacteria. In one embodiment, the homolog of a polypeptide indicated in Table II, column 3, lines 115, 116 or 117 and 483 to 485 is a homolog having the same or a similar activity being derived from Gammaproteobacteria. In one embodiment, the homolog of a polypeptide indicated in Table II, column 3, lines 115, 116 or 117 and 483 to 485 is derived from Enterobacteriales. In one embodiment, the homolog of a polypeptide indicated in Table II, column 3, lines 115, 116 or 117 and 483 to 485 is a homolog being derived from Enterobacteriaceae. In one embodiment, the homolog of a polypeptide indicated in Table II, column 3, lines 115, 116 or 117 and 483 to 485 is a homolog having the same or a similar activity and being derived from Escherichia.
In one embodiment, the homolog of the any one of the polypeptides indicated in Table II, column 3, lines 123 or 124 and 486 to 491 is a homolog having the same or a similar activity. In particular an increase of activity confers an increase in the content of the respective fine chemical in the organisms, preferably of phytosterol, more preferably of campesterol. In one embodiment, the homolog is a homolog with a sequnence as indicated in Table I or II, column 7, lines 123 or 124 and 486 to 491, resp. In one embodiment, the homolog of one of the polypeptides indicated in Table II, column 3, lines 123 or 124 and 486 to 491 is derived from an bacteria. In one embodiment, the homolog of a polypeptide indicated in Table II, column 3, lines 123 or 124 and 486 to 491 is derived from Proteobacteria. In one embodiment, the homolog of a polypeptide indicated in Table II, column 3, lines 123 or 124 and 486 to 491 is a homolog having the same or a similar activity being derived from Gammaproteobacteria. In one embodiment, the homolog of a polypeptide indicated in Table II, column 3, lines 123 or 124 and 486 to 491 is derived from Enterobacteriales. In one embodiment, the homolog of a polypeptide indicated in Table II, column 3, lines 123 or 124 and 486 to 491 is a homolog being derived from Enterobacteriaceae. In one embodiment, the homolog of a polypeptide indicated in Table II, column 3, lines 123 or 124 and 486 to 491 is a homolog having the same or a similar activity and being derived from Escherichia.

**[0023.1.12.12]** Homologs of the polypeptide indicated in Table II, column 3, lines 112 to 124 and/or lines 483 to 491 may be the polypeptides encoded by the nucleic acid molecules indicated in Table I, column 7, lines 112 to 124 and/or lines 483 to 491, resp., or may be the polypeptides indicated in Table II, column 7, lines 112 to 124 and/or lines 483 to 491, resp. Homologs of the polypeptides indicated in Table II, column 3, lines 112 to 124 and/or lines 483 to 491 may be the polypeptides encoded by the nucleic acid molecules indicated in Table I, column 7, lines 112 to 124 and/or lines 483 to 491, resp., or may be the polypeptides indicated in Table II, column 7, lines 112 to 124 and/or lines 483 to 491.
Homologs of the polypeptides indicated in Table II, column 3, lines 112 to 117 and 483 to 485 may be the polypeptides encoded by the nucleic acid molecules indicated in Table I, column 7, lines 112 to 117 and 483 to 485, respectively or may be the polypeptides indicated in Table II, column 7, lines 112 to 117 and 483 to 485, having a beta-sitosterol content- and/or amount-increasing activity. Homologs of the polypeptides indicated in Table II, column 3, lines 112 to 117 and 483 to 485 may be the polypeptides encoded by the nucleic acid molecules indicated in Table I, column 7, lines 112 to 117 and 483 to 485 or may be the polypeptides indicated in Table II, column 7, lines 112 to 117 and 483 to 485 having a beta-sitosterol content- and/or amount-increasing activity.
Homologs of the polypeptides indicated in Table II, column 3, lines 118 to 124 and 486 to 491 may be the polypeptides encoded by the nucleic acid molecules indicated in Table I, column 7, 118 to 124 and 486 to 491, respectively or may be the polypeptides indicated in Table II, column 7, 118 to 124 and 486 to 491, having a campesterol content- and/or amount-increasing activity. Homologs of the polypeptides indicated in Table II, column 3, 118 to 124 and 486 to 491 may be the polypeptides encoded by the nucleic acid molecules indicated in Table I, column 7, 118 to 124 and 486 to 491 or may be the polypeptides indicated in Table II, column 7, lines 118 to 124 and 486 to 491 having a campesterol content- and/or amount-increasing activity.

**[0024.0.0.12]** for the disclosure of this paragraph see [0024.0.0.0] above.

**[0025.0.12.12]** In accordance with the invention, a protein or polypeptide has the "activity of an protein of the invention", e.g. the activity of a protein indicated in Table II, column 3, lines 112 to 117 and/or lines 483 to 485 and/or lines 118 to 124 and/or lines 486 to 491 resp., if its *de novo* activity, or its increased expression directly or indirectly leads to an increased phytosterol level, in particular to a increased beta-sitosterol and/or campesterol, resp., in the organism or a part thereof, preferably in a cell of said organism. In a preferred embodiment, the protein or polypeptide has the above-mentioned additional activities of a protein indicated in Table II, column 3, lines 112 to 117 and/or lines 483 to 485 and/or lines 118 to 124 and/or lines 486 to 491. Throughout the specification the activity or preferably the biological activity of such a protein or polypeptide or an nucleic acid molecule or sequence encoding such protein or polypeptide is identical or similar if it still has the biological or enzymatic activity of any one of the proteins indicated in Table II, column 3, lines 112 to 117 and/or lines 483 to 485 and/or lines 118 to 124 and/or lines 486 to 491 resp. or which has at least 10% of the original enzymatic activity, preferably 20%, particularly preferably 30%, most particularly preferably 40% in comparison to any one of the proteins indicated in Table II, column 3, lines 112, 113, 114, 118, 119, 120, 121 or 122 of Saccharomyces cerevisiae and/or any one of the proteins indicated in Table II, column 3, lines 115, 116, 117, 123 or 124 or 483 to 491 of E. coli K12.
In one embodiment, the polypeptide of the invention confers said activity, e.g. the increase of the respective fine chemical in an organism or a part thereof, if it is derived from an organism, which is evolutionary close to the organism indicated in Table I, column 4 and is expressed in an organism, which is evolutionary distant to the origin organism. For example origin and expressing organism are derived from different families, orders, classes or phylums whereas origin and the organism indicated in Table I, column 4 are derived from the same families, orders, classes or phylums.

**[0025.1.0.12] and [0025.2.0.12]** for the disclosure of the paragraphs [0025.1.0.12] and [0025.2.0.12] see [0025.1.0.0] and [0025.2.0.0] above.

**[0026.0.0.12] to [0033.0.0.12]** for the disclosure of the paragraphs [0026.0.0.12] to [0033.0.0.12] see paragraphs [0026.0.0.0] to [0033.0.0.0] above.

**[0034.0.12.12]** Preferably, the reference, control or wild type differs form the subject of the present invention only in the cellular activity of the polypeptide of the invention, e.g. as result of an increase in the level of the nucleic acid molecule of the present invention or an increase of the specific activity of the polypeptide of the invention. E.g., it differs by or in the expression level or activity of an protein having the activity of a protein as indicated in Table II, column 3, lines 112 to 117 and/or lines 483 to 485 and/or lines 118 to 124 and/or lines 486 to 491 resp., or being encoded by a nucleic acid molecule as indicated in Table I, column 5, lines 112 to 117 and/or lines 483 to 485 and/or lines 118 to 124 and/or lines 486 to 491 resp., or its homologs, e.g. as indicated in Table I, column 7, lines 112 to 117 and/or lines 483 to 485 and/or lines 118 to 124 and/or lines 486 to 491 resp. its biochemical or genetic causes and therefore shows the increased amount of the respective fine chemical.

**[0035.0.0.12] to [0038.0.0.12] and [0039.0.5.12]** for the disclosure of the paragraphs [0035.0.0.12] to [0038.0.0.12] and [0039.0.5.12] see paragraphs [0035.0.0.0] to [0039.0.0.0] above.

**[0040.0.0.12] to [0044.0.0.12]** for the disclosure of the paragraphs [0040.0.0.12] to [0044.0.0.12] see paragraphs [0035.0.0.0] and [0044.0.0.0] above.

**[0045.0.12.12.]** In one embodiment, the activity of the Saccharomyces cerevisiae protein YER156C or its homologs, e.g. an activity of a Arabidopsis thaliana hypothetical protein F2K15.180, e.g. as indicated in Table I, columns 5 or 7, lines 114 or 122, is increased conferring an increase of the respective fine chemical, preferably of beta-sitosterol between 18% and 39% or more or an increase of the respective fine chemical, preferably of campesterol between 17% and 95% or more or an increase of the respective fine chemical, preferably of beta-sitosterol and campesterol between 17% and 95%, preferably between 18% and 39% or more.
In one embodiment, the activity of the Saccharomyces cerevisiae protein YER173W or its homologs, e.g. an activity of a checkpoint protein, involved in the activation of the DNA damage and meiotic pachytene checkpoints, e.g. as indicated in Table I, columns 5 or 7, line 121, is increased conferring an increase of the respective fine chemical, preferably of the campesterol between 23% and 51% or more.
In one embodiment, in case the activity of the Saccharomyces cerevisiae protein YKR057W or its homologs, e.g. an activity of ribosomal protein, similar to S21 ribosomal proteins, involved in ribosome biogenesis and translation, e.g. as indicated in Table I, columns 5 or 7, lines 113 or 120, is increased conferring an increase of the respective fine chemical, preferably of beta-sitosterol between 14% and 32% or more or an increase of the respective fine chemical, preferably of campesterol between 20% and 39% or more or an increase of the respective fine chemical, preferably of beta-sitosterol and campesterol between 14% and 39%, preferably between 20% and 32% or more.
In one embodiment, the activity of the Saccharomyces cerevisiae protein YOR044W or its homologs, e.g. an activity of a probable membrane protein YOR044w, e.g. as indicated in Table I, columns 5 or 7, line 119, is increased conferring an increase of the respective fine chemical, preferably of the campesterol between 28% and 57% or more.
In one embodiment, the activity of the Saccharomyces cerevisiae protein YOR084W or its homologs, e.g. an activity of a putative lipase of the peroxisomal matrix, e.g. as indicated in Table I, columns 5 or 7, line 112, is increased conferring an increase of the respective fine chemical, preferably of the beta-sitosterol between 100% and 259% or more.
In one embodiment, the activity of the Saccharomyces cerevisiae protein YPR172W or its homologs, e.g. an activity of a YPR172W protein, e.g. as indicated in Table I, columns 5 or 7, line 118, is increased conferring an increase of the respective fine chemical, preferably of the campesterol between 17% and 62% or more.
In one embodiment the activity of the Escherichia coli K12 protein b0019 or its homologs, e.g. having a Na+/H+ antiporter activity, in particular, of the superfamiliy of Na+/H+-exchanging protein nhaA, e.g. as indicated in Table I, columns 5 or 7, line 117, is increased conferring an increase of the respective fine chemical, preferably of the beta-sitosterol between 20% and 47% or more.
In one embodiment, the activity of the Escherichia coli K12 protein b0421 or its homologs, e.g. with a geranyltranstransferase (=farnesyldiphosphate synthase) activity, in particular, of the superfamiliy of dimethylallyltrans-transferase, geranyltranstransferase, e.g. as indicated in Table I, columns 5 or 7, line 116, is increased conferring an increase of the respective fine chemical, preferably of beta-sitosterol between 13% and 52% or more.
In one embodiment, the activity of the Escherichia coli K12 protein b2699 or its homologs, e.g. a protein with a DNA strand exchange and recombination protein with protesase und nuclease-activity, e.g. as indicated in Table I, columns 5 or 7, lines 115 or 124, is increased conferring an increase of the respective fine chemical, preferably of preferably of beta-sitosterol between 14% and 35% or more or an increase of the respective fine chemical, preferably of campesterol between 17% and 62% or more or an increase of the respective fine chemical, preferably of beta-sitosterol and campesterol between 14% and 62%, preferably between 17% and 35% or more.
In one embodiment, the activity of the Escherichia coli K12 protein b3256 or its homologs, e.g. a protein with acetyl CoA carboxylase, biotin carboxylase subunit activity, in particular, of the superfamiliy of biotin carboxylase, e.g. as indicated in Table I, columns 5 or 7, line 123, is increased conferring an increase of the respective fine chemical, preferably of campesterol between 20% and 22% or more.
In case the activity of the Escherichia coli K12 protein b0050 or its homologs e.g. a conserved protein potentially involved in protein protein interaction e.g. as indicated in Table II, columns 5 or 7, line 483, is increased, preferably, in one embodiment the increase of the fine chemical, preferably of preferably of the beta-sitosterol between 15% and 24% or more is conferred.
In one embodiment, in case the activity of the Escherichia coli K12 protein b0161 or its homologs e.g. a periplasmic serine protease e.g. as indicated in Table II, columns 5 or 7, line 484 or 486, is increased, preferably of beta-sitosterol between 12% and 26% or more or an increase of the respective fine chemical, preferably of campesterol between 17% and 42% or more or an increase of the respective fine chemical, preferably of beta-sitosterol and campesterol between 12% and 42%, preferably between 17% and 26% or more.
In case the activity of the Escherichia coli K12 protein b4129 or its homologs e.g. a lysine tRNA synthetase e.g. as indicated in Table II, columns 5 or 7, line 485, is increased, preferably, in one embodiment the increase of the fine chemical, preferably of the beta-sitosterol between 21% and 23% or more is conferred.
In one embodiment, in case the activity of the Escherichia coli K12 protein b0464 or its homologs, e.g. the activity of a transcriptional repressor for multidrug efflux pump (TetR/AcrR family) protein as indicated in Table II, columns 5 or 7, line 487, is increased, preferably, in one embodiment the increase of the fine chemical, preferably of campesterol between 16% and 32% or more is conferred.
In one embodiment, in case the activity of the Escherichia coli K12 protein b1896 or its homologs, e.g. the activity of a trehalose-6-phosphate synthase as indicated in Table II, columns 5 or 7, line 488, is increased, preferably, in one embodiment the increase of the fine chemical, preferably of campesterol between 18% and 59% or more is conferred.
In one embodiment, in case the activity of the Escherichia coli K12 protein b2341 or its homologs, e.g. the activity of a bifunctional anaerobic fatty acid oxidation complex protein: enoyl-CoA hydratase/epimerase/isomerase (N-terminal); 3-hydroxyacyl-CoA dehydrogenase (C-terminal) as indicated in Table II, columns 5 or 7, line 489, is increased, preferably, in one embodiment the increase of the fine chemical, preferably of campesterol between 18% and 48% or more is conferred.
In one embodiment, in case the activity of the Escherichia coli K12 protein b2478 or its homologs, e.g. the activity of a dihydrodipicolinate synthase as indicated in Table II, columns 5 or 7, line 490, is increased, preferably, in one embodiment the increase of the fine chemical, preferably of campesterol between 17% and 37% or more is conferred.
In case the activity of the Escherichia coli K12 protein b2822 or its homologs e.g. a DNA helicase, ATP-dependent dsDNA/ssDNA exonuclease V subunit, ssDNA endonuclease e.g. as indicated in Table II, columns 5 or 7, line 491, is increased, preferably, in one embodiment the increase of the fine chemical, preferably of campesterol between 21 % and 32% or more is conferred.

**[0046.0.12.12]** In one embodiment, the activity of the Saccharomyces cerevisiae protein YER156C or its homologs, e.g. an activity of a Arabidopsis thaliana hypothetical protein F2K15.180, e.g. as indicated in Table I, columns 5 or 7, lines 114 or 122 confers an increase of the respective fine chemical and of further phytosterol activity-having compounds or their precursors.
In one embodiment, the activity of the Saccharomyces cerevisiae protein YER173W or its homologs, e.g. an activity of a checkpoint protein, involved in the activation of the DNA damage and meiotic pachytene checkpoints, e.g. as indicated in Table I, columns 5 or 7, line 121, confers an increase of the respective fine chemical and of further phytosterol activity-having compounds or their precursors.
In one embodiment, in case the activity of the Saccharomyces cerevisiae protein YKR057W or its homologs, e.g. an activity of ribosomal protein, similar to S21 ribosomal proteins, involved in ribosome biogenesis and translation, e.g. as indicated in Table I, columns 5 or 7, lines 113 or 120, confers an increase of the respective fine chemical and of further phytosterol activity-having compounds or their precursors.
In one embodiment, the activity of the Saccharomyces cerevisiae protein YOR044W or its homologs, e.g. an activity of a probable membrane protein YOR044w, e.g. as indicated in Table I, columns 5 or 7, line 119, confers an increase of the respective fine chemical and of further phytosterol activity-having compounds or their precursors.
In one embodiment, the activity of the Saccharomyces cerevisiae protein YOR084W or its homologs, e.g. an activity of a putative lipase of the peroxisomal matrix, e.g. as indicated in Table I, columns 5 or 7, line 112 confers an increase of the respective fine chemical and of further phytosterol activity-having compounds or their precursors.
In one embodiment, the activity of the Saccharomyces cerevisiae protein YPR172W or its homologs, e.g. an activity of a YPR172W protein, e.g. as indicated in Table I, columns 5 or 7, line 118, confers an increase of the respective fine chemical and of further phytosterol activity-having compounds or their precursors.
In one embodiment the activity of the Escherichia coli K12 protein b0019 or its homologs, e.g. having a Na+/H+ antiporter activity, in particular, of the superfamiliy of Na+/H+-exchanging protein nhaA, e.g. as indicated in Table I, columns 5 or 7, line 117 confers an increase of the respective fine chemical and of further phytosterol activity-having compounds or their precursors.
In one embodiment, the activity of the Escherichia coli K12 protein b0421 or its homologs, e.g. with a geranyltranstransferase (=farnesyldiphosphate synthase) activity, in particular, of the superfamiliy of dimethylallyltrans-transferase, geranyltranstransferase, e.g. as indicated in Table I, columns 5 or 7, line 116 confers an increase of the respective fine chemical and of further phytosterol activity-having compounds or their precursors.
In one embodiment, the activity of the Escherichia coli K12 protein b2699 or its homologs, e.g. a protein with a DNA strand exchange and recombination protein with protesase und nuclease-activity, e.g. as indicated in Table I, columns 5 or 7, lines 115 or 124 confers an increase of the respective fine chemical and of further phytosterol activity-having compounds or their precursors.
In one embodiment, the activity of the Escherichia coli K12 protein b3256 or its homologs, e.g. a protein with acetyl CoA carboxylase, biotin carboxylase subunit activity, in particular, of the superfamiliy of biotin carboxylase, e.g. as indicated in Table I, columns 5 or 7, line 123, confers an increase of the respective fine chemical and of further phytosterol activity-having compounds or their precursors.
In one embodiment, in case the activity of the Escherichia coli K12 protein b0050 or its homologs e.g. a conserved protein potentially involved in protein protein interaction is increased, preferably an increase of the fine chemical and of further phytosterol activity-having compounds or their precursor is conferred.
In case the activity of the Escherichia coli K12 protein b0161 or its homologs e.g. a periplasmic serine protease is increased, preferably, in one embodiment an increase of one or more fine chemical, preferably an increase of one or more the fine chemical and of further phytosterol activity-having compounds or their precursor is conferred.
In one embodiment, in case the activity of the Escherichia coli K12 protein b4129 or its homologs e.g. a lysine tRNA synthetase is increased, preferably, in one embodiment an increase of the fine chemical, preferably an increase of the fine chemical and of further phytosterol activity-having compounds or their precursor is conferred.
In one embodiment, in case the activity of the Escherichia coli K12 protein b0464 or its homologs e.g. a transcriptional repressor for multidrug efflux pump (TetR/AcrR family) is increased, preferably, in one embodiment an increase of the fine chemical, preferably an increase of the fine chemical and of further phytosterol activity-having compounds or their precursor is conferred.
In one embodiment, in case the activity of the Escherichia coli K12 protein b1896 or its homologs e.g. a trehalose-6-phosphate synthase is increased, preferably, in one embodiment an increase of the fine chemical, preferably an increase of the fine chemical and of further phytosterol activity-having compounds or their precursor is conferred.
In one embodiment, in case the activity of the Escherichia coli K12 protein b2341 or its homologs e.g. a bifunctional anaerobic fatty acid oxidation complex protein: enoyl-CoA hydratase/epimerase/isomerase (N-terminal); 3-hydroxyacyl-CoA dehydrogenase (C-terminal) is increased, preferably, in one embodiment an increase of the fine chemical, preferably an increase of the fine chemical and of further phytosterol activity-having compounds or their precursor is conferred.
In one embodiment, in case the activity of the Escherichia coli K12 protein b2478 or its homologs e.g. a dihydrodipicolinate synthase is increased, preferably, in one embodiment an increase of the fine chemical, preferably an increase of the fine chemical and of further phytosterol activity-having compounds or their precursor is conferred.
In one embodiment, in case the activity of the Escherichia coli K12 protein b2822 or its homologs e.g. a DNA helicase, ATP-dependent dsDNA/ssDNA exonuclease V subunit, ssDNA endonuclease is increased, preferably, in one embodiment an increase of the fine chemical, preferably an increase of the fine chemical and of further phytosterol activity-having compounds or their precursor is conferred.

**[0047.0.0.12] to [0048.0.0.12]** for the disclosure of the paragraphs [0047.0.0.12] and [0048.0.0.12] see paragraphs [0047.0.0.0] and [0048.0.0.0] above.

**[0049.0.7.7]** A protein having an activity conferring an increase in the amount or level of the beta-sitosterol preferably has the structure of the polypeptide described herein.
In a particular embodiment, the polypeptides used in the process of the present invention or the polypeptide of the present invention comprises the sequence of a consensus sequence as shown in Table IV, column 7, lines 112 to 117 and 483 to 485 and/or the sequence of a consensus sequence as indicated in Table IV, columns 5 or 7, lines 112 to 117 and 483 to 485 and/or the sequence of a polypeptide as indicated in Table II, columns 5 or 7, lines 112 to 117 and 483 to 485 or of a functional homologue thereof as described herein, or of a polypeptide encoded by the nucleic acid molecule characterized herein or the nucleic acid molecule according to the invention, for example by a nucleic acid molecule as indicated in Table I, columns 5 or 7, lines 112 to 117 and 483 to 485 or its herein described functional homologues and has the herein mentioned activity confering an increase in the beta-sitosterol level.
A protein having an activity conferring an increase in the amount or level of the campesterol preferably has the structure of the polypeptide described herein. In a particular embodiment, the polypeptides used in the process of the present invention or the polypeptide of the present invention comprises the sequence of a consensus sequence as shown in Table IV, column 7, lines 118 to 124 and 486 to 491 and/or the sequence of a consensus sequence as indicated in Table IV, columns 5 or 7, lines 118 to 124 and 486 to 491 and/or the sequence of a polypeptide as indicated in Table II, columns 5 or 7, lines 118 to 124 and 486 to 491 or of a functional homologue thereof as described herein, or of a polypeptide encoded by the nucleic acid molecule characterized herein or the nucleic acid molecule according to the invention, for example by a nucleic acid molecule as indicated in Table I, columns 5 or 7, lines 118 to 124 and 486 to 491 or its herein described functional homologues and has the herein mentioned activity confering an increase in the campesterol level.

**[0050.0.12.12]** For the purposes of the present invention, the term "the respective fine chemical" also encompass the corresponding salts, such as, for example, the potassium or sodium salts of phytosterols or their ester.

**[0051.0.5.12] and [0052.0.0.12]** for the disclosure of the paragraphs [0051.0.5.12] and [0052.0.0.12] see paragraphs [0051.0.0.0] and [0052.0.0.0] above.

**[0053.0.12.12]** In one embodiment, the process of the present invention comprises one or more of the following steps
a) stabilizing a protein conferring the increased expression of a protein encoded by the nucleic acid molecule of the invention or of the polypeptide of the invention, e.g. of a polypeptide having an activity of a protein as indicated in Table II, column 3, lines 112 to 117 or lines 483 to 485 or lines 118 to 124 or lines 486 to 491 or its homologs, e.g. as indicated in Table II, columns 5 or 7, lines 112 to 117 or lines 483 to 485 or lines 118 to 124 or lines 486 to 491, activity having herein-mentioned the respective fine chemical-increasing activity;
b) stabilizing a mRNA conferring the increased expression of a protein encoded by the nucleic acid molecule of the invention, e.g. of a polypeptide having an activity of a protein as indicated in Table II, column 3, lines 112 to 117 or lines 483 to 485 or lines 118 to 124 or lines 486 to 491 or its homologs activity, e.g. as indicated in Table II, columns 5 or 7, lines 112 to 117 or lines 483 to 485 or lines 118 to 124 or lines 486 to 491, or of a mRNA encoding the polypeptide of the present invention having herein-mentioned the respective fine chemical-increasing activity;
c) increasing the specific activity of a protein conferring the increased expression of a protein encoded by the nucleic acid molecule of the invention or of the polypeptide of the present invention having herein-mentioned the respective fine chemical-increasing activity, e.g. of a polypeptide having an activity of a protein as indicated in Table II, column 3, lines 112 to 117 or lines 483 to 485 or lines 118 to 124 or lines 486 to 491 or its homologs activity, e.g. as indicated in Table II, columns 5 or 7, lines 112 to 117 or lines 483 to 485 or lines 118 to 124 or lines 486 to 491, or decreasing the inhibitory regulation of the polypeptide of the invention;
d) generating or increasing the expression of an endogenous or artificial transcription factor mediating the expression of a protein conferring the increased expression of a protein encoded by the nucleic acid molecule of the invention or of the polypeptide of the invention having herein-mentioned the respective fine chemical-increasing activity, e.g. of a polypeptide having an activity of a protein as indicated in Table II, column 3, lines 112 to 117 or lines 483 to 485 or lines 118 to 124 or lines 486 to 491 or its homologs activity, e.g. as indicated in Table II, columns 5 or 7, lines 112 to 117 or lines 483 to 485 or lines 118 to 124 or lines 486 to 491;
e) stimulating activity of a protein conferring the increased expression of a protein encoded by the nucleic acid molecule of the present invention or a polypeptide of the present invention having herein-mentioned the respective fine chemical-increasing activity, e.g. of a polypeptide having an activity of a protein as indicated in Table II, column 3, lines 112 to 117 or lines 483 to 485 or lines 118 to 124 or lines 486 to 491 or its homologs activity, e.g. as indicated in Table II, columns 5 or 7, lines 112 to 117 or lines 483 to 485 or lines 118 to 124 or lines 486 to 491, by adding one or more exogenous inducing factors to the organism or parts thereof;
f) expressing a transgenic gene encoding a protein conferring the increased expression of a polypeptide encoded by the nucleic acid molecule of the present invention or a polypeptide of the present invention, having herein-mentioned the respective fine chemical-increasing activity, e.g. of a polypeptide having an activity of a protein as indicated in Table II, column 3, lines 112 to 117 or lines 483 to 485 or lines 118 to 124 or lines 486 to 491 or its homologs activity, e.g. as indicated in Table II, columns 5 or 7, lines 112 to 117 or lines 483 to 485 or lines 118 to 124 or lines 486 to 491, and/or
g) increasing the copy number of a gene conferring the increased expression of a nucleic acid molecule encoding a polypeptide encoded by the nucleic acid molecule of the invention or the polypeptide of the invention having herein-mentioned the respective fine chemical-increasing activity, e.g. of a polypeptide having an activity of a protein as indicated in Table II, column 3, lines 112 to 117 or lines 483 to 485 or lines 118 to 124 or lines 486 to 491 or its homologs, e.g. as indicated in Table II, columns 5 or 7, lines 112 to 117 or lines 483 to 485 or lines 118 to 124 or lines 486 to 491, activity.
h) Increasing the expression of the endogenous gene encoding the polypeptide of the invention, e.g. a polypeptide having an activity of a protein as indicated in Table II, column 3, lines 112 to 117 or lines 483 to 485 or lines 118 to 124 or lines 486 to 491 or its homologs activity, e.g. as indicated in Table II, columns 5 or 7, lines 112 to 117 or lines 483 to 485 or lines 118 to 124 or lines 486 to 491, by adding positive expression or removing negative expression elements, e.g. homologous recombination can be used to either introduce positive regulatory elements like for plants the 35S enhancer into the promoter or to remove repressor elements form regulatory regions. Further gene conversion methods can be used to disrupt repressor elements or to enhance to activity of positive elements. Positive elements can be randomly introduced in plants by T-DNA or transposon mutagenesis and lines can be identified in which the positive elements have be integrated near to a gene of the invention, the expression of which is thereby enhanced;
i) Modulating growth conditions of an organism in such a manner, that the expression or activity of the gene encoding the protein of the invention or the protein itself is enhanced for example microorganisms or plants can be grown for example under a higher temperature regime leading to an enhanced expression of heat shock proteins, which can lead an enhanced fine chemical production and/or
j) selecting of organisms with especially high activity of the proteins of the invention from natural or from mutagenized resources and breeding them into the target organisms, eg the elite crops.

**[0054.0.12.12]**Preferably, said mRNA is the nucleic acid molecule of the present invention and/or the protein conferring the increased expression of a protein encoded by the nucleic acid molecule of the present invention or the polypeptide having the herein mentioned activity is the polypeptide of the present invention, e.g. conferring the increase of the respective fine chemical after increasing the expression or activity of the encoded polypeptide or having the activity of a polypeptide having an activity of a protein as indicated in Table II, column 5, lines 112 to 117 or lines 483 to 485 or lines 118 to 124 or lines 486 to 491 resp., or its homologs activity, e.g. as indicated in Table II, column 5, lines 112 to 117 or lines 483 to 485 or lines 118 to 124 or lines 486 to 491 resp..

**[0055.0.0.12] to [0067.0.0.12]** for the disclosure of the paragraphs [0055.0.0.12] to [0067.0.0.12] see paragraphs [0055.0.0.0] to [0067.0.0.0] above.

**[0068.0.12.12]** The mutation is introduced in such a way that the production of the phytosterol(s) is not adversely affected.

**[0069.0.0.12]** for the disclosure of this paragraph see paragraphs [0069.0.0.0] above.

**[0070.0.12.12]** Owing to the introduction of a gene or a plurality of genes conferring the expression of the nucleic acid molecule of the invention or the polypeptide of the invention, for example the nucleic acid construct mentioned below, or encoding the YER156C, YKR057W, YOR084W, b0019, b0421, b2699, b0050, b0161 and/or b4129 protein or of the YER156C, YER173W, YKR057W, YOR044W, YPR172W, b2699, b3256, b0161, b0464, b1896, b2341, b2478 and/or b2822 protein into an organism alone or in combination with other genes, it is possible not only to increase the biosynthetic flux towards the end product, but also to increase, modify or create *de novo* an advantageous, preferably novel metabolites composition in the organism, e.g. an advantageous fatty acid composition comprising a higher content of (from a viewpoint of nutrional physiology limited) phytosterol(s) etc..

**[0071.0.5.12]** for the disclosure of this paragraph see [0071.0.0.0] above.

**[0072.0.12.12]** By influencing the metabolism thus, it is possible to produce, in the process according to the invention, further advantageous compounds. Examples of such compounds are, in addition to phytosterols further sterols, stanols or squalene, squalene epoxide or cycloartenol.

**[0073.0.12.12]**Accordingly, in one embodiment, the process according to the invention relates to a process, which comprises:
a) providing a non-human organism, preferably a microorganism, a non-human animal, a plant or animal cell, a plant or animal tissue or a plant;
b) increasing an activity of a polypeptide of the invention or a homolog thereof, e.g. as indicated in Table II, columns 5 or 7, lines 112 to 117 or lines 483 to 485 or lines 118 to 124 or lines 486 to 491, or of a polypeptide being encoded by the nucleic acid molecule of the present invention and described below, e.g. conferring an increase of the respective fine chemical in an organism, preferably in a microorganism, a non-human animal, a plant or animal cell, a plant or animal tissue or a plant,
c) growing an organism, preferably a microorganism, a non-human animal, a plant or animal cell, a plant or animal tissue or a plant under conditions which permit the production of the respective fine chemical in the organism, preferably the microorganism, the plant cell, the plant tissue or the plant; and
d) if desired, revovering, optionally isolating, the free and/or bound the respective fine chemical and, optionally further free and/or bound phytosterol or its conjugate synthesised by the organism, the microorganism, the non-human animal, the plant or animal cell, the plant or animal tissue or the plant.

**[0074.0.12.12]** The organism, in particular the microorganism, non-human animal, the plant or animal cell, the plant or animal tissue or the plant is advantageously grown in such a way that it is not only possible to recover, if desired isolate the free or bound the respective fine chemical or the free and bound respective fine chemical but as option it is also possible to produce, recover and, if desired isolate, other free or/and bound of phytosterol(s) or its/their conjugates.

**[0075.0.0.12] to [0077.0.0.12]** for the disclosure of the paragraphs [0075.0.0.12] to [0077.0.0.12] see paragraphs [0075.0.0.0] to [0077.0.0.0] above.

**[0078.0.12.12]**The organism such as microorganisms or plants or the recovered, and if desired isolated, respective fine chemical can then be processed further directly into foodstuffs or animal feeds or for other applications, for example according to the disclosures made in:
US 20040101829, which disclose a methods for treating hyperlipidemia and to reduce Low Density Lipoprotein ("LDL") levels in a subject,
US 20040047971, which disclose the preparation of a fat composition containing sterol esters characterised by direct interesterification of sterol with triglyceride,
US 5,965,449, which describes phytosterol-based compositions useful in preventing and treating cardiovascular disease and other disorders,
US 5,523,087, which is for a pharmaceutical composition containing beta-sitosterol for the treatment of diabetic male sexual dysfunction;
US 5,747,464, which discloses a composition for inhibiting absorption of fat and cholesterol from the gut comprising beta.-sitosterol bound irreversibly to pectin,
US 4,588,717, which describes a vitamin supplement which comprises a fatty acid ester of a phytosterol,
US 5,270,041, which teaches the use of small amounts of sterols, their fatty acid esters and glucosides for the treatment of tumours,
US 6,087,353, which comprises methods of making a composition suitable for incorporation into foods, beverages, pharmaceuticals, nutraceuticals and the like which comprises condensing a suitable aliphatic acid with a phytosterol to form a phytosterol ester and subsequently hydrogenating the phytosterol ester to form a hydrogenated phytosterol ester,
which are expressly incorporated herein by reference.
The fermentation broth, fermentation products, plants or plant products can be treated with water and a mixture of organic solvents (hexane and acetone) in order to extract the phytosterols. Crude phytosterols are obtained from the organic phase by removal of the solvents, complexation of the sterols in the extract with calcium chloride in methanol, separation of the sterol-complexes by centrifugation, dissociation of the complexes by heating in water and removal of the water. The crude phytosterols can be further purified by crystallisation from isopropanol. According to an other production process the tall oil soap is first subjected to fractional distillation which removes volatile compounds. The resulting residue (tall oil pitch) containing sterols in esterified form is treated with alkali to liberate these sterols. After neutralisation, the material is subjected to a two-stage distillation process. The distillate is then dissolved in methanol/methylethylketone solvent and the sterols crystallising from this solution are obtained by filtration, washed with solvent and dried. US 4,420,427 teaches the preparation of sterols from vegetable oil sludge using solvents such as methanol. Alternatively, phytosterols may be obtained from tall oil pitch or soap, by-products of the forestry practise as described in PCT/CA95/00555, incorporated herein by reference. The extraction and crystallization may be performed by other methods known to the person skilled in the art and described herein below.
To form a phytosterol ester in accordance with the US 6,087,353, the selected phytosterol and aliphatic acid or its ester with volatile alcohol are mixed together under reaction conditions to permit condensation of the phytosterol with the aliphatic acid to produce an ester. A most preferred method of preparing these esters which is widely used in the edible fat and oil industry is described in U.S. Pat. No. 5,502,045 (which is incorporated herein by reference). The stanol and/or sterol esters with the desired fatty acid composition can also be produced by direct, preferably catalytic esterification methods, e.g. U.S. Pat. No. 5,892,068, between free fatty acids or fatty acid blends of the composition and the stanol and/or sterol. In addition, stanol and/or sterol esters can also be produced by enzymatic esterification e.g. as outlined in EP 195 311 (which are incorporated herein by reference).
Products of these different work-up procedures are phytosterols and/or esters and/or conjugates or compositions which still comprise fermentation broth, plant particles and cell components in different amounts, advantageously in the range of from 0 to 99% by weight, preferably below 80% by weight, especially preferably between below 50% by weight.

**[0079.0.0.12] to [0084.0.0.12]** for the disclosure of the paragraphs [0079.0.0.12] to [0084.0.0.12] see paragraphs [0079.0.0.0] to [0084.0.0.0] above.

**[0085.0.12.12]** With regard to the nucleic acid sequence as depicted a nucleic acid construct which contains a nucleic acid sequence mentioned herein or an organism (= transgenic organism) which is transformed with said nucleic acid sequence or said nucleic acid construct, "transgene" means all those constructs which have been brought about by genetic manipulation methods,preferably in which either
a) a nucleic acid sequence as indicated in Table I, columns 5 or 7, lines 112 to 117 or lines 483 to 485 or lines 118 to 124 or lines 486 to 491, resp. or a derivative thereof, or
b) a genetic regulatory element, for example a promoter, which is functionally linked to the nucleic acid sequence as indicated in Table I, columns 5 or 7, lines 112 to 117 or lines 483 to 485 or lines 118 to 124 or lines 486 to 491, resp. or a derivative thereof, or
c) (a) and (b)
is/are not present in its/their natural genetic environment or has/have been modified by means of genetic manipulation methods, it being possible for the modification to be, by way of example, a substitution, addition, deletion, inversion or insertion of one or more nucleotide. "Natural genetic environment" means the natural chromosomal locus in the organism of origin or the presence in a genomic library. In the case of a genomic library, the natural, genetic environment of the nucleic acid sequence is preferably at least partially still preserved. The environment flanks the nucleic acid sequence at least on one side and has a sequence length of at least 50 bp, preferably at least 500 bp, particularly preferably at least 1000 bp, very particularly preferably at least 5000 bp.

**[0086.0.0.12] and [0087.0.0.12]** for the disclosure of the paragraphs [0086.0.0.12] and [0087.0.0.12] see paragraphs [0086.0.0.0] and [0087.0.0.0] above.

**[0088.0.12.12]** In an advantageous embodiment of the invention, the organism takes the form of a plant whose phytosterol content is modified advantageously owing to the nucleic acid molecule of the present invention expressed. This is important for plant breeders since, for example, the nutritional value of plants for poultry is dependent on the abovementioned phytosterol and the general amount of phytosterol as source in feed and/or food. Further, this is also important since, for example a balanced content of different phytosterols induces stress resistance to plants. After the YER156C, YKR057W, YOR084W, b0019, b0421, b2699, b0050, b0161 and/or b4129 and/or YER156C, YER173W, YKR057W, YOR044W, YPR172W, b2699, b3256, b0161, b0464, b1896, b2341, b2478 and/or b2822 protein activity has been increased or generated, or after the expression of nucleic acid molecule or polypeptide according to the invention has been generated or increased, the transgenic plant generated thus is grown on or in a nutrient medium or else in the soil and subsequently harvested.

**[0088.1.0.12], [0089.0.0.12] and [0090.0.0.12]** for the disclosure of the paragraphs [0088.1.0.12], [0089.0.0.12] and [0090.0.0.12] see paragraphs [0088.1.0.0], [0089.0.0.0] and [0090.0.0.0] above.

**[0091.0.12.12]** Thus, the content of plant components and preferably also further impurities is as low as possible, and the abovementioned phytosterols are obtained in as pure form as possible. In these applications, the content of plant components advantageously amounts to less than 10%, preferably 1%, more preferably 0.1 %, very especially preferably 0.01 % or less.

**[0092.0.0.12] to [0094.0.0.12]** for the disclosure of the paragraphs [0092.0.0.12] to [0094.0.0.12] see paragraphs [0092.0.0.0] to [0094.0.0.0] above.

**[0095.0.12.12]** It may be advantageous to increase the pool of said phytosterols in the transgenic organisms by the process according to the invention in order to isolate high amounts of the pure respective fine chemical.

**[0096.0.12.12]** In another preferred embodiment of the invention a combination of the increased expression of the nucleic acid sequence or the protein of the invention together with the transformation of a protein or polypeptid or a compound, which functions as a sink for the desired fine chemical, for example beta-sitosterol or campesterol in the organism, is useful to increase the production of the respective fine chemical.

**[0097.0.0.12]** for the disclosure of this paragraph see paragraph [0097.0.0.0] above.

**[0098.0.12.12]** In a preferred embodiment, the respective fine chemical (beta-sitosterol or campesterol) is produced in accordance with the invention and, if desired, is isolated. The production of further phytosterols or conjugates or mixtures thereof or mixtures with other compounds by the process according to the invention is advantageous.

**[0099.0.12.12]** In the case of the fermentation of microorganisms, the abovementioned phytosterol (preferably beta-sitosterol and/or campesterol) accumulate in the medium and/or the cells. If microorganisms are used in the process according to the invention, the fermentation broth can be processed after the cultivation. Depending on the requirement, all or some of the biomass can be removed from the fermentation broth by separation methods such as, for example, centrifugation, filtration, decanting or a combination of these methods, or else the biomass can be left in the fermentation broth. The fermentation broth can subsequently be reduced, or concentrated, with the aid of known methods such as, for example, rotary evaporator, thin-layer evaporator, falling film evaporator, by reverse osmosis or by nanofiltration. Afterwards advantageously further compounds for formulation can be added such as corn starch or silicates. This concentrated fermentation broth advantageously together with compounds for the formulation can subsequently be processed by lyophilization, spray drying, and spray granulation or by other methods. Preferably the respective fine chemical or the phytosterol (preferably beta-sitosterol and/or campesterol) comprising compositions are isolated from the organisms, such as the microorganisms or plants or the culture medium in or on which the organisms have been grown, or from the organism and the culture medium, in the known manner, for example via extraction, distillation, crystallization, chromatography or a combination of these methods. These purification methods can be used alone or in combination with the aforementioned methods such as the separation and/or concentration methods.

**[0100.0.12.12]** Transgenic plants which comprise the phytosterol (preferably beta-sitosterol and/or campesterol) synthesized in the process according to the invention can advantageously be marketed directly without there being any need for the phytosterols (preferably beta-sitosterol and/or campesterol) (oils, lipids or fatty acids synthesized to be isolated). Plants for the process according to the invention are listed as meaning intact plants and all plant parts, plant organs or plant parts such as leaf, stem, seeds, root, tubers, anthers, fibers, root hairs, stalks, embryos, calli, cotelydons, petioles, harvested material, plant tissue, reproductive tissue and cell cultures which are derived from the actual transgenic plant and/or can be used for bringing about the transgenic plant. In this context, the seed comprises all parts of the seed such as the seed coats, epidermal cells, seed cells, endosperm or embryonic tissue. However, the respective fine chemical produced in the process according to the invention can also be isolated from the organisms, advantageously plants, in the form of their oils, fats, lipids, esters and/or as extracts, e.g. ether, alcohol, or other organic solvents or water containing extract and/or free phytosterol(s) . The respective fine chemical produced by this process can be obtained by harvesting the organisms, either from the crop in which they grow, or from the field. This can be done via pressing or extraction of the plant parts, preferably the plant seeds. To increase the efficiency of extraction it is beneficial to clean, to temper and if necessary to hull and to flake the plant material especially the seeds. In this context, the oils, fats, lipids, esters and/or free phytosterols can be obtained by what is known as cold beating or cold pressing without applying heat. To allow for greater ease of disruption of the plant parts, specifically the seeds, they are previously comminuted, steamed or roasted. The seeds, which have been pretreated in this manner can subsequently be pressed or extracted with solvents such as warm hexane. The solvent is subsequently removed. In the case of microorganisms, the latter are, after harvesting, for example extracted directly without further processing steps or else, after disruption, extracted via various methods with which the skilled worker is familiar. In this manner, more than 96% of the compounds produced in the process can be isolated. Thereafter, the resulting products are processed further, i.e. degummed and/or refined. In this process, substances such as the plant mucilages and suspended matter are first removed. What is known as desliming can be affected enzymatically or, for example, chemico-physically by addition of acid such as phosphoric acid.
Plant sterols (phytosterols) are by-products of traditional vegetable oil refining. The source may be commonly a blend of crude edible oils, consisting of soy bean oil or of other edible oils, e.g. corn, rapeseed, olive and palm oil in varying proportions. Hemp may also be a source of new oilseed, oil and food ingredients as well as Sea buckthorn (hippophaë rhamnoides). The crude oil, which is obtained by pressing or solvent extraction, may undergoes a series of refining processes to remove solvents, lecithins, free fatty acids, color bodies, off-odors and off-flavors. In one of these steps, the oil may be subjected to steam distillation at reduced pressure (deodorisation) and the resulting distillate contains the phytosterol fraction. From this fraction, fatty acids, lecithins and other compounds are removed by fractional distillation, ethanolysis/transesterification, distillation and crystallisation from a heptane solution, and the phytosterols are further purified by recrystallisation using food grade materials and good manufacturing practices. The extraction and purification steps are standard methods and similar to the procedures used traditionally by the food industry for the production of plant sterols. Phytosterol esters may be produced from the sterols using food grade vegetable oil-derived fatty acids or triglycerides and applying standard methods for esterification or transesterification commonly used in the fats and oils industry.
Phytosterol in microorganisms may be localized intracellularly, therefor their recovery essentials comes down to the isolation of the biomass. Well-establisthed approaches for the harvesting of cells include filtration, centrifugation and coagulation/flocculation as described herein. Determination of tocopherols in cells has been described by Tan and Tsumura 1989, see also Biotechnology of Vitamins, Pigments and Growth Factors, Edited by Erik J. Vandamme, London, 1989, p.96 to 103. Many further methods to determine the tocopherol content are known to the person skilled in the art.

**[0101.0.12.12]** The identity and purity of the compound(s) isolated can be determined by prior-art techniques. They encompass high-performance liquid chromatography (HPLC), gas chromatography (GC), spectroscopic methods, mass spectrometry (MS), staining methods, thin-layer chromatography, NIRS, enzyme assays or microbiological assays. These analytical methods are compiled in: Patek et al. (1994) Appl. Environ. Microbiol. 60:133-140; Malakhova et al. (1996) Biotekhnologiya 11 27-32; and Schmidt et al. (1998) Bioprocess Engineer. 19:67-70. Ulmann's Encyclopedia of Industrial Chemistry (1996) Bd. A27, VCH Weinheim, pp. 89-90, pp. 521-540, pp. 540-547, pp. 559-566, 575-581 and pp. 581-587; Michal, G (1999) Biochemical Pathways: An Atlas of Biochemistry and Molecular Biology, John Wiley and Sons; Fallon, A. et al. (1987) Applications of HPLC in Biochemistry in: Laboratory Techniques in Biochemistry and Molecular Biology, vol. 17.

**[0102.0.12.12]** Phytosterols can for example be analyzed advantageously via HPLC or GC separation methods and detected by MS oder MSMS methods. The unambiguous detection for the presence of beta-sitosterol and/or campesterol containing products can be obtained by analyzing recombinant organisms using analytical standard methods: GC, GC-MS or TLC, as described on several occasions by Christie and the references therein (1997, in: Advances on Lipid Methodology, Fourth Edition: Christie, Oily Press, Dundee, 119-169; 1998, Gaschromatographie-Massenspektrometrie-Verfahren [Gas chromatography/mass spectrometric methods], Lipide 33:343-353). The material to be analyzed can be disrupted by sonication, grinding in a glass mill, liquid nitrogen and grinding, cooking, or via other applicable methods; see also Biotechnology of Vitamins, Pigments and Growth Factors, Edited by Erik J. Vandamme, London, 1989, p.96 to 103.
For the sterol isolation and analysis the German standard method F III (1) may be used. The method comprises: saponification of fat, isolation of unsaponifiable matter using an aliminium oxide column, separation of sterol fraction by preparative TLC and determination of the composition of sterols as trimetysilyl ethers by GLC.

**[0103.0.12.12]** In a preferred embodiment, the present invention relates to a process for the production of the respective fine chemical comprising or generating in an organism or a part thereof the expression of at least one nucleic acid molecule comprising a nucleic acid molecule selected from the group consisting of:
a) nucleic acid molecule encoding, preferably at least the mature form, of the polypeptide having a sequence as indicated in Table II, columns 5 or 7, lines 112 to 117 or lines 483 to 485 or lines 118 to 124 or lines 486 to 491, resp. or a fragment thereof, which confers an increase in the amount of the respective fine chemical in an organism or a part thereof;
b) nucleic acid molecule comprising, preferably at least the mature form, of a nucleic acid molecule having a sequence as indicated in Table I, columns 5 or 7, lines 112 to 117 or lines 483 to 485 or lines 118 to 124 or lines 486 to 491, resp.
c) nucleic acid molecule whose sequence can be deduced from a polypeptide sequence encoded by a nucleic acid molecule of (a) or (b) as result of the degeneracy of the genetic code and conferring an increase in the amount of the respective fine chemical in an organism or a part thereof;
d) nucleic acid molecule encoding a polypeptide which has at least 50% identity with the amino acid sequence of the polypeptide encoded by the nucleic acid molecule of (a) to (c) and conferring an increase in the amount of the respective fine chemical in an organism or a part thereof;
e) nucleic acid molecule which hybidizes with a nucleic acid molecule of (a) to (c) under under stringent hybridisation conditions and conferring an increase in the amount of the respective fine chemical in an organism or a part thereof;
f) nucleic acid molecule encoding a polypeptide, the polypeptide being derived by substituting, deleting and/or adding one or more amino acids of the amino acid sequence of the polypeptide encoded by the nucleic acid molecules (a) to (d), preferably to (a) to (c) and conferring an increase in the amount of the respective fine chemical in an organism or a part thereof;
g) nucleic acid molecule encoding a fragment or an epitope of a polypeptide which is encoded by one of the nucleic acid molecules of (a) to (e), preferably to (a) to (c) and and conferring an increase in the amount of the respective fine chemical in an organism or a part thereof;
h) nucleic acid molecule comprising a nucleic acid molecule which is obtained by amplifying nucleic acid molecules from a cDNA library or a genomic library using the primers pairs having a sequence as indicated in Table III, column 7, lines 112 to 117 or lines 483 to 485 or lines 118 to 124 or lines 486 to 491, resp.and conferring an increase in the amount of the respective fine chemical in an organism or a part thereof;
i) nucleic acid molecule encoding a polypeptide which is isolated, e.g. from an expression library, with the aid of monoclonal antibodies against a polypeptide encoded by one of the nucleic acid molecules of (a) to (h), preferably to (a) to (c), and and conferring an increase in the amount of the respective fine chemical in an organism or a part thereof;
j) nucleic acid molecule which encodes a polypeptide comprising the consensus sequence having a sequences as indicated in Table IV, column 7, lines 112 to 117 or lines 483 to 485 or lines 118 to 124 or lines 486 to 491, resp., and conferring an increase in the amount of the respective fine chemical in an organism or a part thereof ;
k) nucleic acid molecule comprising one or more of the nucleic acid molecule encoding the amino acid sequence of a polypeptide encoding a domain of the polypeptide indicated in Table II, columns 5 or 7, lines 112 to 117 or lines 483 to 485 or lines 118 to 124 or lines 486 to 491, resp. and conferring an increase in the amount of the respective fine chemical in an organism or a part thereof; and
l) nucleic acid molecule which is obtainable by screening a suitable library under stringent conditions with a probe comprising one of the sequences of the nucleic acid molecule of (a) to (k), preferably to (a) to (c), or with a fragment of at least 15 nt, preferably 20 nt, 30 nt, 50 nt, 100 nt, 200 nt or 500 nt of the nucleic acid molecule characterized in (a) to (k), preferably to (a) to (c), and conferring an increase in the amount of the respective fine chemical in an organism or a part thereof;
or which comprises a sequence which is complementary thereto.

**[00103.1.0.12.]** In one embodiment, the nucleic acid molecule used in the process of the invention distinguishes over the sequence indicated in Table IA, columns 5 or 7, lines 112 to 117 or lines 483 to 485 and/or lines 118 to 124 or lines 486 to 491, by one or more nucleotides. In one embodiment, the nucleic acid molecule used in the process of the invention does not consist of the sequence shown in indicated in Table I A, columns 5 or 7, lines 112 to 117 or lines 483 to 485 and/or lines 118 to 124 or lines 486 to 491: In one embodiment, the nucleic acid molecule used in the process of the invention is less than 100%, 99,999%, 99,99%, 99,9% or 99% identical to a sequence indicated in Table I A, columns 5 or 7, lines 112 to 117 or lines 483 to 485 and/or lines 118 to 124 or lines 486 to 491. In another embodiment, the nucleic acid molecule does not encode a polypeptide of a sequence indicated in Table II A, columns 5 or 7, lines 112 to 117 or lines 483 to 485 and/or lines 118 to 124 or lines 486 to 491.

**[00103.2.0.12.]** In one embodiment, the nucleic acid molecule used in the process of the invention distinguishes over the sequence indicated in Table I B, columns 5 or 7, lines 112 to 117 or lines 483 to 485 and/or lines 118 to 124 or lines 486 to 491, by one or more nucleotides. In one embodiment, the nucleic acid molecule used in the process of the invention does not consist of the sequence shown in indicated in Table I B, columns 5 or 7, lines 112 to 117 or lines 483 to 485 and/or lines 118 to 124 or lines 486 to 491. In one embodiment, the nucleic acid molecule used in the process of the invention is less than 100%, 99,999%, 99,99%, 99,9% or 99% identical to a sequence indicated in Table I B, columns 5 or 7, lines 112 to 117 or lines 483 to 485 and/or lines 118 to 124 or lines 486 to 491. In another embodiment, the nucleic acid molecule does not encode a polypeptide of a sequence indicated in Table II B, columns 5 or 7, lines 112 to 117 or lines 483 to 485 and/or lines 118 to 124 or lines 486 to 491.

**[0104.0.12.12]** In one embodiment, the nucleic acid molecule used in the process distinguishes over the sequence indicated in Table I, columns 5 or 7, lines 112 to 117 or lines 483 to 485 and/or lines 118 to 124 or lines 486 to 491, resp., by one or more nucleotides. In one embodiment, the nucleic acid molecule used in the process of the invention does not consist of the sequence indicated in Table I, columns 5 or 7, lines 112 to 117 or lines 483 to 485 and/or lines 118 to 124 or lines 486 to 491, resp., In one embodiment, the nucleic acid molecule of the present invention is less than 100%, 99,999%, 99,99%, 99,9% or 99% identical to the sequence indicated in Table I, columns 5 or 7, lines 112 to 117 or lines 483 to 485 and/or lines 118 to 124 or lines 486 to 491, resp. In another embodiment, the nucleic acid molecule does not encode a polypeptide of a sequence indicated in Table II, columns 5 or 7, lines 112 to 117 or lines 483 to 485 and/or lines 118 to 124 or lines 486 to 491, resp.

**[0105.0.0.12] to [0107.0.0.12]** for the disclosure of the paragraphs [0105.0.0.12] to [0107.0.0.12] see paragraphs [0105.0.0.0] and [0107.0.0.0] above.

**[0108.0.12.12]** Nucleic acid molecules with the sequence as indicated in Table I, columns 5 or 7, lines 112 to 117 or lines 483 to 485 and/or lines 118 to 124 or lines 486 to 491, resp., nucleic acid molecules which are derived from an amino acid sequences as indicated in Table II, columns 5 or 7, lines 112 to 117 or lines 483 to 485 and/or lines 118 to 124 or lines 486 to 491, resp., or from polypeptides comprising the consensus sequence as indicated in Table IV, column 7, lines 112 to 117 or lines 483 to 485 and/or lines 118 to 124 or lines 486 to 491 resp. or their derivatives or homologues encoding polypeptides with the enzymatic or biological activity of an activity of a polypeptide as indicated in Table II, column 3, 5 or 7, lines 112 to 117 or lines 483 to 485 and/or lines 118 to 124 or lines 486 to 491, resp., or conferring an increase of the respective fine chemical, meaning phytosterol, in particular, beta-sitosterol and/or campesterol after increasing its expression or activity are advantageously increased in the process according to the invention.

**[0109.0.0.12]** for the disclosure of this paragraph see [0109.0.0.0] above.

**[0110.0.12.12]** Nucleic acid molecules, which are advantageous for the process according to the invention and which encode polypeptides with an activity of a polypeptide used in the method of the invention or used in the process of the invention, e.g. of a protein as shown in Table II, columns 5 or 7, lines 112 to 117 or lines 483 to 485 and/or lines 118 to 124 or lines 486 to 491 or being encoded by a nucleic acid molecule indicated in Table I, columns 5 or 7, lines 112 to 117 or lines 483 to 485 and/or lines 118 to 124 or lines 486 to 491 or of its homologs, e.g. as indicated in Table II, columns 5 or 7, lines 112 to 117 or lines 483 to 485 and/or lines 118 to 124 or lines 486 to 491 can be determined from generally accessible databases.

**[0111.0.0.12]** for the disclosure of this paragraph see [0111.0.0.0] above.

**[0112.0.12.12]** The nucleic acid molecules used in the process according to the invention take the form of isolated nucleic acid sequences, which encode polypeptides with an activity of a polypeptide as indicated in Table II, column 3, lines 112 to 117 or lines 483 to 485 and/or lines 118 to 124 or lines 486 to 4914 resp., or having the sequence of a polypeptide as indicated in Table II, columns 5 and 7 lines 112 to 117 or lines 483 to 485 and/or lines 118 to 124 or lines 486 to 491 resp., and conferring an increase in the level of phytosterols, preferably beta-sitosterol and/or campesterol.

**[0113.0.0.12] to [0120.0.0.12]** for the disclosure of the paragraphs [0113.0.0.12] to [0120.0.0.12] see paragraphs [0113.0.0.0] and [0120.0.0.0] above.

**[0121.0.12.12]** However, it is also possible to use artificial sequences, which differ in one or more bases from the nucleic acid sequences found in organisms, or in one or more amino acid molecules from polypeptide sequences found in organisms, in particular from the polypeptide sequences indicated in Table II, columns 5 or 7, lines 112 to 117 or lines 483 to 485 and/or lines 118 to 124 or lines 486 to 491, resp., or the functional homologues thereof as described herein, preferably conferring above-mentioned activity, i.e. conferring an increase in the level of beta-sitosterol after increasing the activity of the polypeptide sequences indicated in Table II, columns 5 or 7, lines 112 to 117 and/or lines 483 to 485 , or conferring increase in the level of campesterol after increasing the activity of the polypeptide sequences indicated in Table II, columns 5 or 7, lines 118 to 124 and/or lines 486 to 491.

**[0122.0.0.12] to [0127.0.0.12]** for the disclosure of the paragraphs [0122.0.0.12] to [0127.0.0.12] see paragraphs [0122.0.0.0] and [0127.0.0.0] above.

**[0128.0.12.12]** Synthetic oligonucleotide primers for the amplification, e.g. as the pairs indicated in Table III, column 7, lines 112 to 117 or lines 483 to 485 and/or lines 118 to 124 or lines 486 to 491, resp. by means of polymerase chain reaction can be generated on the basis of a sequence shown herein, for example the sequence as indicated in Table I, columns 5 or 7, lines 112 to 117 or lines 483 to 485 and/or lines 118 to 124 or lines 486 to 491, resp. or the sequences derived from a sequence as indicated in Table II, columns 5 or 7, lines 112 to 117 or lines 483 to 485 and/or lines 118 to 124 or lines 486 to 491, resp.

**[0129.0.12.12]** Moreover, it is possible to identify conserved regions from various organisms by carrying out protein sequence alignments with the polypeptide used in the process of the invention, in particular with sequences of the polypeptide of the invention, from which conserved regions, and in turn, degenerate primers can be derived. Conserved regions are those, which show a very little variation in the amino acid in one particular position of several homologs from different origin. The consenus sequences indicated in Table IV, column 7, lines 112 to 117 or lines 483 to 485 and/or lines 118 to 124 or lines 486 to 491, resp. are derived from said aligments.

**[0130.0.12.12]** Degenerated primers can then be utilized by PCR for the amplification of fragments of novel proteins having above-mentioned activity, e.g. conferring the increase of phytosterol, preferably beta-sitosterol and/or campesterol resp after increasing the expression or activity of the protein comprising said fragment.

**[0131.0.0.12] to [0138.0.0.12]** for the disclosure of the paragraphs [0131.0.0.12] to [0138.0.0.12] see paragraphs [0131.0.0.0] to [0138.0.0.0] above.

**[0139.0.12.12]** Polypeptides having above-mentioned activity, i.e. conferring the respective fine chemical increase, derived from other organisms, can be encoded by other DNA sequences which hybridize to a sequences indicated in Table I, columns 5 or 7, lines 112 to 117 or lines 483 to 485 and/or lines 118 to 124 or lines 486 to 491, preferably of Table I B, columns 5 or 7, lines 112 to 117 or lines 483 to 485 and/or lines 118 to 124 or lines 486 to 491 under relaxed hybridization conditions and which code on expression for peptides having the phytosterol, preferably beta-sitosterol and/or campesterol increasing activity.

**[0140.0.0.12] to [0146.0.0.12]** for the disclosure of the paragraphs [0140.0.0.12] to [0146.0.0.12] see paragraphs [0140.0.0.0] to [0146.0.0.0] above.

**[0147.0.12.12]** Further, the nucleic acid molecule of the invention comprises a nucleic acid molecule, which is a complement of one of the nucleotide sequences of above mentioned nucleic acid molecules or a portion thereof. A nucleic acid molecule which is complementary to one of the nucleotide sequences indicated in Table I, columns 5 or 7, lines 112 to 117 or lines 483 to 485 and/or lines 118 to 124 or lines 486 to 491, preferably of Table I B, columns 5 or 7, lines 112 to 117 or lines 483 to 485 and/or lines 118 to 124 or lines 486 to 491 resp., is one which is sufficiently complementary to one of said nucleotide sequences such that it can hybridize to one of said nucleotide sequences, thereby forming a stable duplex. Preferably, the hybridisation is performed under stringent hybrization conditions. However, a complement of one of the herein disclosed sequences is preferably a sequence complement thereto according to the base pairing of nucleic acid molecules well known to the skilled person. For example, the bases A and G undergo base pairing with the bases T and U or C, resp. and visa versa. Modifications of the bases can influence the base-pairing partner.

**[0148.0.12.12]** The nucleic acid molecule of the invention comprises a nucleotide sequence which is at least about 30%, 35%, 40% or 45%, preferably at least about 50%, 55%, 60% or 65%, more preferably at least about 70%, 80%, or 90%, and even more preferably at least about 95%, 97%, 98%, 99% or more homologous to a nucleotide sequence indicated in Table I, columns 5 or 7, lines 112 to 117 or lines 483 to 485 and/or lines 118 to 124 or lines 486 to 491, preferably of Table I B, columns 5 or 7, lines 112 to 117 or lines 483 to 485 and/or lines 118 to 124 or lines 486 to 491 resp. or a portion thereof and preferably has above mentioned activity, in particular having a phytosterol, in particular, of beta-sitosterol and/or campesterol increasing activity after increasing the activity or an activity of a product of a gene encoding said sequences or their homologs.

**[0149.0.12.12]** The nucleic acid molecule of the invention comprises a nucleotide sequence which hybridizes, preferably hybridizes under stringent conditions as defined herein, to one of the nucleotide sequences indicated in Table I, columns 5 or 7, lines 112 to 117 or lines 483 to 485 and/or lines 118 to 124 or lines 486 to 491, preferably of Table I B, columns 5 or 7, lines 112 to 117 or lines 483 to 485 and/or lines 118 to 124 or lines 486 to 491 resp. or a portion thereof and encodes a protein having above-mentioned activity, e.g. conferring increase of phytosterol, in particular, of beta-sitosterol and/or campesterol resp., and optionally, the activity of a protein indicated in Table II, column 5, lines 112 to 117 or lines 483 to 485 and/or lines 118 to 124 or lines 486 to 491.

**[00149.1.12.12]** Optionally, in one embodiment, the nucleotide sequence, which hybridises to one of the nucleotide sequences indicated in Table I, columns 5 or 7, lines 112 to 117 or lines 483 to 485 and/or lines 118 to 124 or lines 486 to 491, preferably of Table I B, columns 5 or 7, lines 112 to 117 or lines 483 to 485 and/or lines 118 to 124 or lines 486 to 491 resp., has further one or more of the activities annotated or known for the a protein as indicated in Table II, column 3, lines 112 to 117 or lines 483 to 485 and/or lines 118 to 124 or lines 486 to 491.

**[0150.0.12.12]** Moreover, the nucleic acid molecule of the invention can comprise only a portion of the coding region of one of the sequences indicated in Table I, columns 5 or 7, lines 112 to 117 or lines 483 to 485 and/or lines 118 to 124 or lines 486 to 491, preferably of Table I B, columns 5 or 7, lines 112 to 117 or lines 483 to 485 and/or lines 118 to 124 or lines 486 to 491 resp. for example a fragment which can be used as a probe or primer or a fragment encoding a biologically active portion of the polypeptide of the present invention or of a polypeptide used in the process of the present invention, i.e. having above-mentioned activity, e.g. conferring an increase of phytosterol, in particular, of beta-sitosterol and/or campesterol resp., if its activity is increased. The nucleotide sequences determined from the cloning of the present protein-according-to-the-invention-encoding gene allows for the generation of probes and primers designed for use in identifying and/or cloning its homologues in other cell types and organisms. The probe/primer typically comprises substantially purified oligonucleotide. The oligonucleotide typically comprises a region of nucleotide sequence that hybridizes under stringent conditions to at least about 12, 15 preferably about 20 or 25, more preferably about 40, 50 or 75 consecutive nucleotides of a sense strand of one of the sequences set forth, e.g., as indicated in Table I, columns 5 or 7, lines 112 to 117 or lines 483 to 485 and/or lines 118 to 124 or lines 486 to 491, resp. an anti-sense sequence of one of the sequences, e.g., as indicated in Table I, columns 5 or 7, lines 112 to 117 or lines 483 to 485 and/or lines 118 to 124 or lines 486 to 491, resp. or naturally occurring mutants thereof. Primers based on a nucleotide of invention can be used in PCR reactions to clone homologues of the polypeptide of the invention or of the polypeptide used in the process of the invention, e.g. as the primers described in the examples of the present invention, e.g. as shown in the examples. A PCR with the primer pairs indicated in Table III, column 7, lines 112 to 117 or lines 483 to 485 and/or lines 118 to 124 or lines 486 to 491, resp., will result in a fragment of a polynucleotide sequence as indicated in Table I, columns 5 or 7, lines 112 to 117 or lines 483 to 485 and/or lines 118 to 124 or lines 486 to 491, resp., or its gene product.

**[0151.0.0.12]** for the disclosure of this paragraph see paragraph [0151.0.0.0] above.

**[0152.0.12.12]** The nucleic acid molecule of the invention encodes a polypeptide or portion thereof which includes an amino acid sequence which is sufficiently homologous to an amino acid sequence as indicated in Table II, columns 5 or 7, lines 112 to 117 or lines 483 to 485 and/or lines 118 to 124 or lines 486 to 491, resp., such that the protein or portion thereof maintains the ability to participate in the respective fine chemical production, in particular an activity increasing the level of phytosterol, in particular, of beta-sitosterol and/or campesterol, resp., as mentioned above or as described in the examples in plants or microorganisms is comprised.

**[0153.0.12.12]** As used herein, the language "sufficiently homologous" refers to proteins or portions thereof which have amino acid sequences which include a minimum number of identical or equivalent amino acid residues (e.g., an amino acid residue which has a similar side chain as an amino acid residue in one of the sequences of the polypeptide of the present invention) to an amino acid sequence as indicated in Table II, columns 5 or 7, lines 112 to 117 or lines 483 to 485 and/or lines 118 to 124 or lines 486 to 491 resp., such that the protein or portion thereof is able to participate in the increase of the respective fine chemical production. In one embodiment, a protein or portion thereof as indicated in Table II, columns 5 or 7, lines 112 to 117 or lines 483 to 485 and/or lines 118 to 124 or lines 486 to 491, resp., has for example an activity of a polypeptide as indicaded in Table II, column 3, lines 112 to 117 or lines 483 to 485 and/or lines 118 to 124 or lines 486 to 491, resp.

**[0154.0.12.12]** In one embodiment, the nucleic acid molecule of the present invention comprises a nucleic acid that encodes a portion of the protein of the present invention. The protein is at least about 30%, 35%, 40%, 45% or 50%, preferably at least about 55%, 60%, 65% or 70%, and more preferably at least about 75%, 80%, 85%, 90%, 91%, 92%, 93% or 94% and most preferably at least about 95%, 97%, 98%, 99% or more homologous to an entire amino acid sequence as indicated in Table II, columns 5 or 7, lines 112 to 117 or lines 483 to 485 and/or lines 118 to 124 or lines 486 to 491, resp., and has above-mentioned activity, e.g.conferring preferably the increase of the respective fine chemical.

**[0155.0.0.12] and [0156.0.0.12]** for the disclosure of the paragraphs [0155.0.0.12] and [0156.0.0.12] see paragraphs [0155.0.0.0] and [0156.0.0.0] above.

**[0157.0.12.12]** The invention further relates to nucleic acid molecules that differ from one of the nucleotide sequences as indicated in Table I, columns 5 or 7, lines 112 to 117 or lines 483 to 485 and/or lines 118 to 124 or lines 486 to 491, resp., (and portions thereof) due to degeneracy of the genetic code and thus encode a polypeptide of the present invention, in particular a polypeptide having above mentioned activity, e.g. conferring an increase in the respective fine chemical in a organism, e.g. as that polypeptides encoded by the sequence as indicated in Table I, columns 5 or 7, lines 112 to 117 or lines 483 to 485 and/or lines 118 to 124 or lines 486 to 491, resp., or of the polypeptide as indicated in Table II, columns 5 or 7, lines 112 to 117 or lines 483 to 485 and/or lines 118 to 124 or lines 486 to 491, resp., or the functional homologues. Advantageously, the nucleic acid molecule of the invention comprises, or in an other embodiment has, a nucleotide sequence encoding a protein comprising, or in an other embodiment having, an amino acid sequence of a consensus sequences as indicated in Table IV, column 7, lines 112 to 117 or lines 483 to 485 and/or lines 118 to 124 or lines 486 to 491, resp., or of the polypeptide as indicated in Table II, columns 5 or 7, lines 112 to 117 or lines 483 to 485 and/or lines 118 to 124 or lines 486 to 491, resp., or the functional homologues. In a still further embodiment, the nucleic acid molecule of the invention encodes a full length protein which is substantially homologous to an amino acid sequence comprising a consensus sequence as indicated in Table IV, column 7, lines 112 to 117 or lines 483 to 485 and/or lines 118 to 124 or lines 486 to 491, resp., or of a polypeptide as indicated in Table II, columns 5 or 7, lines 112 to 117 or lines 483 to 485 and/or lines 118 to 124 or lines 486 to 491, resp., or the functional homologues. However, in a preferred embodiment, the nucleic acid molecule of the present invention does not consist of a sequence as indicated in Table I, columns 5 or 7, lines 112 to 117 or lines 483 to 485 and/or lines 118 to 124 or lines 486 to 491, preferably as indicated in Table I A, columns 5 or 7, lines 112 to 117 or lines 483 to 485 and/or lines 118 to 124 or lines 486 to 491, resp. Preferably the nucleic acid molecule of the invention is a functional homologue or identical to a nucleic acid molecule indicated in Table I B, column 7, lines 112 to 117 or lines 483 to 485 and/or lines 118 to 124 or lines 486 to 491.

**[0158.0.0.12] to [0160.0.0.12]** for the disclosure of the paragraphs [0158.0.0.12] to [0160.0.0.12] see paragraphs [0158.0.0.0] to [0160.0.0.0] above.

**[0161.0.12.12]** Accordingly, in another embodiment, a nucleic acid molecule of the invention is at least 15, 20, 25 or 30 nucleotides in length. Preferably, it hybridizes under stringent conditions to a nucleic acid molecule comprising a nucleotide sequence of the nucleic acid molecule of the present invention or used in the process of the present invention, e.g. comprising a sequence as indicated in Table I, columns 5 or 7, lines 112 to 117 or lines 483 to 485 and/or lines 118 to 124 or lines 486 to 491, resp. The nucleic acid molecule is preferably at least 20, 30, 50, 100, 250 or more nucleotides in length.

**[0162.0.0.12]** for the disclosure of this paragraph see paragraph [0162.0.0.0] above.

**[0163.0.12.12]** Preferably, nucleic acid molecule of the invention that hybridizes under stringent conditions to a sequence as indicated in Table I, columns 5 or 7, lines 112 to 117 or lines 483 to 485 and/or lines 118 to 124 or lines 486 to 491, resp., corresponds to a naturally-occurring nucleic acid molecule of the invention. As used herein, a "naturally-occurring" nucleic acid molecule refers to an RNA or DNA molecule having a nucleotide sequence that occurs in nature (e.g., encodes a natural protein). Preferably, the nucleic acid molecule encodes a natural protein having above-mentioned activity, e.g. conferring the respective fine chemical increase after increasing the expression or activity thereof or the activity of a protein of the invention or used in the process of the invention.

**[0164.0.0.12]** for the disclosure of this paragraph see paragraph [0164.0.0.0] above.

**[0165.0.12.12]**For example, nucleotide substitutions leading to amino acid substitutions at "non-essential" amino acid residues can be made in a sequence of the nucleic acid molecule of the invention or used in the process of the invention, e.g. as indicated in Table I, columns 5 or 7, lines 112 to 117 or lines 483 to 485 and/or lines 118 to 124 or lines 486 to 491, resp.

**[0166.0.0.12] and [0167.0.0.12]** for the disclosure of the paragraphs [0166.0.0.12] and [0167.0.0.12] see paragraphs [0166.0.0.0] and [0167.0.0.0] above.

**[0168.0.12.12]** Accordingly, the invention relates to nucleic acid molecules encoding a polypeptide having above-mentioned activity, e.g. conferring an increase in the respective fine chemical in an organisms or parts thereof that contain changes in amino acid residues that are not essential for said activity. Such polypeptides differ in amino acid sequence from a sequence contained in a sequence as indicated in Table II, columns 5 or 7, lines 112 to 117 or lines 483 to 485 and/or lines 118 to 124 or lines 486 to 491, resp., yet retain said activity described herein. The nucleic acid molecule can comprise a nucleotide sequence encoding a polypeptide, wherein the polypeptide comprises an amino acid sequence at least about 50% identical to an amino acid sequence as indicated in Table II, columns 5 or 7, lines 112 to 117 or lines 483 to 485 and/or lines 118 to 124 or lines 486 to 491, resp., and is capable of participation in the increase of production of the respective fine chemical after increasing its activity, e.g. its expression. Preferably, the protein encoded by the nucleic acid molecule is at least about 60% identical to a sequence as indicated in Table II, columns 5 or 7, lines 112 to 117 or lines 483 to 485 and/or lines 118 to 124 or lines 486 to 491, resp. more preferably at least about 70% identical to one of the sequences as indicated in Table II, columns 5 or 7, lines 112 to 117 or lines 483 to 485 and/or lines 118 to 124 or lines 486 to 491, resp. even more preferably at least about 80%, 90%, 95% homologous to a sequence as indicated in Table II, columns 5 or 7, lines 112 to 117 or lines 483 to 485 and/or lines 118 to 124 or lines 486 to 491, resp. and most preferably at least about 96%, 97%, 98%, or 99% identical to the sequence as indicated in Table II, columns 5 or 7, lines 112 to 117 or lines 483 to 485 and/or lines 118 to 124 or lines 486 to 491. Accordingly, the invention relates to nucleic acid molecules encoding a polypeptide having above-mentioned activity, e.g. conferring an increase in the the respective fine chemical in an organisms or parts thereof that contain changes in amino acid residues that are not essential for said activity. Such polypeptides differ in amino acid sequence from a sequence contained in a sequence as indicated in Table II, columns 5 or 7, lines 112 to 117 or lines 483 to 485 and/or lines 118 to 124 or lines 486 to 491, preferably of Table II B, column 7, lines 112 to 117 or lines 483 to 485 and/or lines 118 to 124 or lines 486 to 491 yet retain said activity described herein. The nucleic acid molecule can comprise a nucleotide sequence encoding a polypeptide, wherein the polypeptide comprises an amino acid sequence at least about 50% identical to an amino acid sequence as indicated in Table II, columns 5 or 7, lines 112 to 117 or lines 483 to 485 and/or lines 118 to 124 or lines 486 to 491, preferably of Table II B, column 7, lines 112 to 117 or lines 483 to 485 and/or lines 118 to 124 or lines 486 to 491 and is capable of participation in the increase of production of the respective fine chemical after increasing its activity, e.g. its expression. Preferably, the protein encoded by the nucleic acid molecule is at least about 60% identical to a sequence as indicated in Table II, columns 5 or 7, lines 112 to 117 or lines 483 to 485 and/or lines 118 to 124 or lines 486 to 491, preferably of Table II B, column 7, lines 112 to 117 or lines 483 to 485 and/or lines 118 to 124 or lines 486 to 491, more preferably at least about 70% identical to one of the sequences as indicated in Table II, columns 5 or 7, lines 112 to 117 or lines 483 to 485 and/or lines 118 to 124 or lines 486 to 491, preferably of Table II B, column 7, lines 112 to 117 or lines 483 to 485 and/or lines 118 to 124 or lines 486 to 491, even more preferably at least about 80%, 90%, or 95% homologous to a sequence as indicated in Table II, columns 5 or 7, lines 112 to 117 or lines 483 to 485 and/or lines 118 to 124 or lines 486 to 491, preferably of Table II B, column 7, lines 112 to 117 or lines 483 to 485 and/or lines 118 to 124 or lines 486 to 491, and most preferably at least about 96%, 97%, 98%, or 99% identical to the sequence as indicated in Table II, columns 5 or 7, lines 112 to 117 or lines 483 to 485 and/or lines 118 to 124 or lines 486 to 491, preferably of Table II B, column 7, lines 112 to 117 or lines 483 to 485 and/or lines 118 to 124 or lines 486 to 491.

**[0169.0.0.12] to [0174.0.0.12]** for the disclosure of the paragraphs [0169.0.0.12] to [0174.0.0.12] see paragraphs [0169.0.0.0] to [0174.0.0.0] above.

**[0175.0.12.12]** For example a sequence which has a 80% homology with sequence SEQ ID NO: 12136 at the protein level is understood as meaning a sequence which, upon comparison with the sequence SEQ ID NO: 12136 by the above program algorithm with the above parameter set, has a 80% homology.

**[0176.0.12.12]** Functional equivalents derived from one of the polypeptides as indicated in Table II, columns 5 or 7, lines 112 to 117 or lines 483 to 485 and/or lines 118 to 124 or lines 486 to 491, resp., according to the invention by substitution, insertion or deletion have at least 30%, 35%, 40%, 45% or 50%, preferably at least 55%, 60%, 65% or 70% by preference at least 80%, especially preferably at least 85% or 90%, 91%, 92%, 93% or 94%, very especially preferably at least 95%, 97%, 98% or 99% homology with one of the polypeptides as indicated in Table II, columns 5 or 7, lines 112 to 117 or lines 483 to 485 and/or lines 118 to 124 or lines 486 to 491, resp., according to the invention and are distinguished by essentially the same properties as a polypeptide as indicated in Table II, columns 5 or 7, lines 112 to 117 or lines 483 to 485 and/or lines 118 to 124 or lines 486 to 491, resp.

**[0177.0.12.12]** Functional equivalents derived from a nucleic acid sequence as indicated in Table I, columns 5 or 7, lines 112 to 117 or lines 483 to 485 and/or lines 118 to 124 or lines 486 to 491, resp., according to the invention by substitution, insertion or deletion have at least 30%, 35%, 40%, 45% or 50%, preferably at least 55%, 60%, 65% or 70% by preference at least 80%, especially preferably at least 85% or 90%, 91%, 92%, 93% or 94%, very especially preferably at least 95%, 97%, 98% or 99% homology with one of the polypeptides as indicated in Table II, columns 5 or 7, lines 112 to 117 or lines 483 to 485 and/or lines 118 to 124 or lines 486 to 491, resp., according to the invention and encode polypeptides having essentially the same properties as a polypeptide as indicated in Table II, columns 5 or 7, lines 112 to 117 or lines 483 to 485 and/or lines 118 to 124 or lines 486 to 491, resp.

**[0178.0.0.12]** for the disclosure of this paragraph see [0178.0.0.0] above.

**[0179.0.12.12]**A nucleic acid molecule encoding an homologous to a protein sequence as indicated in Table II, columns 5 or 7, lines 112 to 117 or lines 483 to 485 and/or lines 118 to 124 or lines 486 to 491, preferably of Table II B, column 7, lines 112 to 117 or lines 483 to 485 and/or lines 118 to 124 or lines 486 to 491, resp., can be created by introducing one or more nucleotide substitutions, additions or deletions into a nucleotide sequence of the nucleic acid molecule of the present invention, in particular as indicated in Table I, columns 5 or 7, lines 112 to 117 or lines 483 to 485 and/or lines 118 to 124 or lines 486 to 491, resp., such that one or more amino acid substitutions, additions or deletions are introduced into the encoded protein. Mutations can be introduced into the encoding sequences of a sequence as indicated in Table I, columns 5 or 7, lines 112 to 117 or lines 483 to 485 and/or lines 118 to 124 or lines 486 to 491, resp., by standard techniques, such as site-directed mutagenesis and PCR-mediated mutagenesis.

**[0180.0.0.12] to [0183.0.0.12]** for the disclosure of the paragraphs [0180.0.0.12] to [0183.0.0.12] see paragraphs [0180.0.0.0] to [0183.0.0.0] above.

**[0184.0.12.12]** Homologues of the nucleic acid sequences used, with a sequence as indicated in Table I, columns 5 or 7, lines 112 to 117 or lines 483 to 485 and/or lines 118 to 124 or lines 486 to 491, preferably of Table I B, column 7, lines 112 to 117 or lines 483 to 485 and/or lines 118 to 124 or lines 486 to 491, resp., or of the nucleic acid sequences derived from a sequences as indicated in Table II, columns 5 or 7, lines 112 to 117 or lines 483 to 485 and/or lines 118 to 124 or lines 486 to 491, preferably of Table II B, column 7, lines 112 to 117 or lines 483 to 485 and/or lines 118 to 124 or lines 486 to 491, resp. comprise also allelic variants with at least approximately 30%, 35%, 40% or 45% homology, by preference at least approximately 50%, 60% or 70%, more preferably at least approximately 90%, 91%, 92%, 93%, 94% or 95% and even more preferably at least approximately 96%, 97%, 98%, 99% or more homology with one of the nucleotide sequences shown or the abovementioned derived nucleic acid sequences or their homologues, derivatives or analogues or parts of these. Allelic variants encompass in particular functional variants which can be obtained by deletion, insertion or substitution of nucleotides from the sequences shown, preferably from a sequence as indicated in Table I, columns 5 or 7, lines 112 to 117 or lines 483 to 485 and/or lines 118 to 124 or lines 486 to 491, resp., or from the derived nucleic acid sequences, the intention being, however, that the enzyme activity or the biological activity of the resulting proteins synthesized is advantageously retained or increased.

**[0185.0.12.12]** In one embodiment of the present invention, the nucleic acid molecule of the invention or used in the process of the invention comprises one or more sequences as indicated in Table I, columns 5 or 7, lines 112 to 117 or lines 483 to 485 and/or lines 118 to 124 or lines 486 to 491, preferably of Table I B, column 7, lines 112 to 117 or lines 483 to 485 and/or lines 118 to 124 or lines 486 to 491, resp. In one embodiment, it is preferred that the nucleic acid molecule comprises as little as possible other nucleotides not shown in any one of sequences as indicated in Table I, columns 5 or 7, lines 112 to 117 or lines 483 to 485 and/or lines 118 to 124 or lines 486 to 491, preferably of Table I B, column 7, lines 112 to 117 or lines 483 to 485 and/or lines 118 to 124 or lines 486 to 491, resp. In one embodiment, the nucleic acid molecule comprises less than 500, 400, 300, 200, 100, 90, 80, 70, 60, 50 or 40 further nucleotides. In a further embodiment, the nucleic acid molecule comprises less than 30, 20 or 10 further nucleotides. In one embodiment, a nucleic acid molecule used in the process of the invention is identical to a sequences as indicated in Table I, columns 5 or 7, lines 112 to 117 or lines 483 to 485 and/or lines 118 to 124 or lines 486 to 491, preferably of Table I B, column 7, lines 112 to 117 or lines 483 to 485 and/or lines 118 to 124 or lines 486 to 491, resp.

**[0186.0.12.12]** Also preferred is that one or more nucleic acid molecule(s) used in the process of the invention encodes a polypeptide comprising a sequence as indicated in Table II, columns 5 or 7, lines 112 to 117 or lines 483 to 485 and/or lines 118 to 124 or lines 486 to 491, preferably of Table II B, column 7, lines 112 to 117 or lines 483 to 485 and/or lines 118 to 124 or lines 486 to 491, resp. In one embodiment, the nucleic acid molecule encodes less than 150, 130, 100, 80, 60, 50, 40 or 30 further amino acids. In a further embodiment, the encoded polypeptide comprises less than 20, 15, 10, 9, 8, 7, 6 or 5 further amino acids. In one embodiment, the encoded polypeptide used in the process of the invention is identical to the sequences as indicated in Table II, columns 5 or 7, lines 112 to 117 or lines 483 to 485 and/or lines 118 to 124 or lines 486 to 491, preferably of Table II B, column 7, lines 112 to 117 or lines 483 to 485 and/or lines 118 to 124 or lines 486 to 491, resp.

**[0187.0.12.12]** In one embodiment, a nucleic acid molecule of the invention or used in the process encodes a polypeptide comprising the sequence as indicated in Table II, columns 5 or 7, lines 112 to 117 or lines 483 to 485 and/or lines 118 to 124 or lines 486 to 491, preferably of Table II B, column 7, lines 112 to 117 or lines 483 to 485 and/or lines 118 to 124 or lines 486 to 491, resp., comprises less than 100 further nucleotides. In a further embodiment, said nucleic acid molecule comprises less than 30 further nucleotides. In one embodiment, the nucleic acid molecule used in the process is identical to a coding sequence of the amino sequences indicated in Table II, columns 5 or 7, lines 112 to 117 or lines 483 to 485 and/or lines 118 to 124 or lines 486 to 491, preferably of Table II B, column 7, lines 112 to 117 or lines 483 to 485 and/or lines 118 to 124 or lines 486 to 491, resp..

**[0188.0.12.12]** Polypeptides (= proteins), which still have the essential biological or enzymatic activity of the polypeptide of the present invention conferring an increase of the respective fine chemical i.e. whose activity is essentially not reduced, are polypeptides with at least 10% or 20%, by preference 30% or 40%, especially preferably 50% or 60%, very especially preferably 80% or 90 or more of the wild type biological activity or enzyme activity, advantageously, the activity is essentially not reduced in comparison with the activity of a polypeptide as indicated in Table II, columns 5 or 7, lines 112 to 117 or lines 483 to 485 and/or lines 118 to 124 or lines 486 to 491, resp., and is expressed under identical conditions.
In one embodiment, the polypeptide of the invention is a homolog consisting of or comprising the sequence as indicated in Table II B, columns 7, lines 112 to 117 or lines 483 to 485 and/or lines 118 to 124 or lines 486 to 491.

**[0189.0.12.12]** Homologues of a sequences as indicated in Table I, columns 5 or 7, lines 112 to 117 or lines 483 to 485 and/or lines 118 to 124 or lines 486 to 491, resp., or of a derived sequences as indicated in Table II, columns 5 or 7, lines 112 to 117 or lines 483 to 485 and/or lines 118 to 124 or lines 486 to 491,resp., also mean truncated sequences, cDNA, single-stranded DNA or RNA of the coding and noncoding DNA sequence. Homologues of said sequences are also understood as meaning derivatives, which comprise noncoding regions such as, for example, UTRs, terminators, enhancers or promoter variants. The promoters upstream of the nucleotide sequences stated can be modified by one or more nucleotide substitution(s), insertion(s) and/or deletion(s) without, however, interfering with the functionality or activity either of the promoters, the open reading frame (= ORF) or with the 3'-regulatory region such as terminators or other 3'regulatory regions, which are far away from the ORF. It is furthermore possible that the activity of the promoters is increased by modification of their sequence, or that they are replaced completely by more active promoters, even promoters from heterologous organisms. Appropriate promoters are known to the person skilled in the art and are mentioned herein below.

**[0190.0.0.12] to [0203.0.0.12]** for the disclosure of the paragraphs [0190.0.0.12] to [0203.0.0.12] see paragraphs [0190.0.0.0] to [0203.0.0.0] above.

**[0204.0.12.12]** Accordingly, in one embodiment, the invention relates to a nucleic acid molecule, which comprises a nucleic acid molecule selected from the group consisting of:
a) nucleic acid molecule encoding, preferably at least the mature form, of a polypeptide as indicated in Table II, columns 5 or 7, lines 112 to 117 or lines 483 to 485 and/or lines 118 to 124 or lines 486 to 491, preferably of Table II B, column 7, lines 112 to 117 or lines 483 to 485 and/or lines 118 to 124 or lines 486 to 491, resp.; or a fragment thereof conferring an increase in the amount of the respective fine chemical, i.e. phytosterol, in particular, of beta-sitosterol (lines 112 to 117 and/or lines 483 to 485) and/or campesterol (lines 118 to 124 and/or lines 486 to 491) resp., in an organism or a part thereof
b) nucleic acid molecule comprising, preferably at least the mature form, of a nucleic acid molecule as indicated in Table I, columns 5 or 7, lines 112 to 117 or lines 483 to 485 and/or lines 118 to 124 or lines 486 to 491, preferably of Table I B, column 7, lines 112 to 117 or lines 483 to 485 and/or lines 118 to 124 or lines 486 to 491, resp., or a fragment thereof conferring an increase in the amount of the respective fine chemical in an organism or a part thereof;
c) nucleic acid molecule whose sequence can be deduced from a polypeptide sequence encoded by a nucleic acid molecule of (a) or (b) as result of the degeneracy of the genetic code and conferring an increase in the amount of the respective fine chemical in an organism or a part thereof;
d) nucleic acid molecule encoding a polypeptide whose sequence has at least 50% identity with the amino acid sequence of the polypeptide encoded by the nucleic acid molecule of (a) to (c) and conferring an increase in the amount of the respective fine chemical in an organism or a part thereof;
e) nucleic acid molecule which hybridizes with a nucleic acid molecule of (a) to (c) under under stringent hybridisation conditions and conferring an increase in the amount of the respective fine chemical in an organism or a part thereof;
f) nucleic acid molecule encoding a polypeptide, the polypeptide being derived by substituting, deleting and/or adding one or more amino acids of the amino acid sequence of the polypeptide encoded by the nucleic acid molecules (a) to (d), preferably to (a) to (c), and conferring an increase in the amount of the respective fine chemical in an organism or a part thereof;
g) nucleic acid molecule encoding a fragment or an epitope of a polypeptide which is encoded by one of the nucleic acid molecules of (a) to (e), preferably to (a) to (c) and conferring an increase in the amount of the respective fine chemical in an organism or a part thereof;
h) nucleic acid molecule comprising a nucleic acid molecule which is obtained by amplifying a cDNA library or a genomic library using primers or primer pairs as indicated in Table III, column 7, lines 112 to 117 or lines 483 to 485 and/or lines 118 to 124 or lines 486 to 491, resp., and conferring an increase in the amount of the respective fine chemical, i.e. phytosterol, in particular, of beta-sitosterol (lines 112 to 117) and/or campesterol (lines 118 to 124) resp., in an organism or a part thereof;
i) nucleic acid molecule encoding a polypeptide which is isolated, e.g. from a expression library, with the aid of monoclonal antibodies against a polypeptide encoded by one of the nucleic acid molecules of (a) to (g), preferably to (a) to (c) and conferring an increase in the amount of the respective fine chemical in an organism or a part thereof;
j) nucleic acid molecule which encodes a polypeptide comprising a consensus sequence as indicated in Table IV, column 7, lines 112 to 117 or lines 483 to 485 and/or lines 118 to 124 or lines 486 to 491, resp., and conferring an increase in the amount of the respective fine chemical, i.e. phytosterol, in particular, of beta-sitosterol (lines 112 to 117 and/or lines 483 to 485) and/or campesterol (lines 118 to 124 and/or lines 486 to 491) resp., in an organism or a part thereof;
k) nucleic acid molecule encoding the amino acid sequence of a polypeptide encoding a domaine of a polypeptide as indicated in Table II, columns 5 or 7, lines 112 to 117 or lines 483 to 485 and/or lines 118 to 124 or lines 486 to 491, preferably of Table II B, column 7, lines 112 to 117 or lines 483 to 485 and/or lines 118 to 124 or lines 486 to 491, resp., and conferring an increase in the amount of the respective fine chemical, i.e. phytosterol, in particular, of beta-sitosterol (lines 112 to 117 and/or lines 483 to 485) and/or campesterol (lines 118 to 124 and/or lines 486 to 491) resp., in an organism or a part thereof; and
l) nucleic acid molecule which is obtainable by screening a suitable nucleic acid library under stringent hybridization conditions with a probe comprising one of the sequences of the nucleic acid molecule of (a) to (k) or with a fragment of at least 15 nt, preferably 20 nt, 30 nt, 50 nt, 100 nt, 200 nt or 500 nt of the nucleic acid molecule characterized in (a) to (h) or of a nucleic acid molecule as indicated in Table I, columns 5 or 7, lines 112 to 117 or lines 483 to 485 and/or lines 118 to 124 or lines 486 to 491, preferably of Table I B, column 7, lines 112 to 117 or lines 483 to 485 and/or lines 118 to 124 or lines 486 to 491, resp., or a nucleic acid molecule encoding, preferably at least the mature form of, a polypeptide as indicated in Table II, columns 5 or 7, lines 112 to 117 or lines 483 to 485 and/or lines 118 to 124 or lines 486 to 491, preferably of Table II B, column 7, lines 112 to 117 or lines 483 to 485 and/or lines 118 to 124 or lines 486 to 491, resp. and conferring an increase in the amount of the respective fine chemical in an organism or a part thereof; or which encompasses a sequence which is complementary thereto;
whereby, preferably, the nucleic acid molecule according to (a) to (I) distinguishes over a sequence as indicated in Table IA, columns 5 or 7, lines 112 to 117 or lines 483 to 485 and/or lines 118 to 124 or lines 486 to 491, resp., by one or more nucleotides. In one embodiment, the nucleic acid molecule of the invention does not consist of the sequence as indicated in Table IA, columns 5 or 7, lines 112 to 117 or lines 483 to 485 and/or lines 118 to 124 or lines 486 to 491, resp.,. In an other embodiment, the nucleic acid molecule of the present invention is at least 30 % identical and less than 100%, 99,999%, 99,99%, 99,9% or 99% identical to a sequence as indicated in Table IA, columns 5 or 7, lines 112 to 117 or lines 483 to 485 and/or lines 118 to 124 or lines 486 to 491, resp. In a further embodiment the nucleic acid molecule does not encode a polypeptide sequence as indicated in Table IIA, columns 5 or 7, lines 112 to 117 or lines 483 to 485 and/or lines 118 to 124 or lines 486 to 491, resp. Accordingly, in one embodiment, the nucleic acid molecule of the present invention encodes in one embodiment a polypeptide which differs at least in one or more amino acids from a polypeptide indicated in Table IIA, columns 5 or 7, lines 112 to 117 or lines 483 to 485 and/or lines 118 to 124 or lines 486 to 491, resp., does not encode a protein of a sequence as indicated in Table IIA, columns 5 or 7, lines 112 to 117 or lines 483 to 485 and/or lines 118 to 124 or lines 486 to 491, resp. Accordingly, in one embodiment, the protein encoded by a sequences of a nucleic acid accoriding to (a) to (I) does not consist of a sequence as indicated in Table IIA, columns 5 or 7, lines 112 to 117 or lines 483 to 485 and/or lines 118 to 124 or lines 486 to 491, resp. In a further embodiment, the protein of the present invention is at least 30 % identical to a protein sequence indicated in Table IIA, columns 5 or 7, lines 112 to 117 or lines 483 to 485 and/or lines 118 to 124 or lines 486 to 491, resp., and less than 100%, preferably less than 99,999%, 99,99% or 99,9%, more preferably less than 99%, 985, 97%, 96% or 95% identical to a sequence as indicated in Table IIA, columns 5 or 7, lines 112 to 117 or lines 483 to 485 and/or lines 118 to 124 or lines 486 to 491, resp.

**[0205.0.0.12] and [0206.0.0.12]** for the disclosure of the paragraphs [0205.0.0.12] and [0206.0.0.12] see paragraphs [0205.0.0.0] and [0206.0.0.0] above.

**[0207.0.12.12]** As described herein, the nucleic acid construct can also comprise further genes, which are to be introduced into the organisms or cells. It is possible and advantageous to introduce into, and express in, the host organisms regulatory genes such as genes for inductors, repressors or enzymes, which, owing to their enzymatic activity, engage in the regulation of one or more genes of a biosynthetic pathway. These genes can be of heterologous or homologous origin. Moreover, further biosynthesis genes may advantageously be present, or else these genes may be located on one or more further nucleic acid constructs. Genes, which are advantageously employed as biosynthesis genes, are genes of the sterol metabolism, the squalen metabolism, the amino acid metabolism, of glycolysis, of the tricarboxylic acid metabolism or their combinations. As described herein, regulator sequences or factors can have a positive effect on preferably the gene expression of the genes introduced, thus increasing it. Thus, an enhancement of the regulator elements may advantageously take place at the transcriptional level by using strong transcription signals such as promoters and/or enhancers. In addition, however, an enhancement of translation is also possible, for example by increasing mRNA stability or by inserting a translation enhancer sequence.

**[0208.0.0.12] to [0226.0.0.12]** for the disclosure of the paragraphs [0208.0.0.12] to [0226.0.0.12] see paragraphs [0208.0.0.0] to [0226.0.0.0] above.

**[0227.0.12.12]** The abovementioned nucleic acid molecules can be cloned into the nucleic acid constructs or vectors according to the invention in combination together with further genes, or else different genes are introduced by transforming several nucleic acid constructs or vectors (including plasmids) into a host cell, advantageously into a plant cell or a microorganisms.
In addition to a sequence indicated in Table I, columns 5 or 7, lines 112 to 117 or lines 483 to 485 and/or lines 118 to 124 or lines 486 to 491 resp., or its derivatives, it is advantageous additionally to express and/or mutate further genes in the organisms. Especially advantageously, additionally at least one further gene of the sterol biosynthetic pathway such as for a phytosterol precursor, for example squalene epoxide is expressed in the organisms such as plants or microorganisms. It is also possible that the regulation of the natural genes has been modified advantageously so that the gene and/or its gene product is no longer subject to the regulatory mechanisms which exist in the organisms. This leads to an increased synthesis of the amino acids desired since, for example, feedback regulations no longer exist to the same extent or not at all. In addition it might be advantageously to combine one or more of the sequences indicated in Table I, columns 5 or 7, lines 112 to 117 or lines 483 to 485 and/or lines 118 to 124 or lines 486 to 491, resp., with genes which generally support or enhances to growth or yield of the target organismen, for example genes which lead to faster growth rate of microorganisms or genes which produces stress-, pathogen, or herbicide resistant plants.

**[0228.0.12.12]** In a further embodiment of the process of the invention, therefore, organisms are grown, in which there is simultaneous overexpression of at least one nucleic acid or one of the genes which code for proteins involved in the sterol/phytosterol metabolism, in particular in synthesis of of beta-sitosterol and/or campesterol.

**[0229.0.12.12]** Further advantageous nucleic acid sequences which can be expressed in combination with the sequences used in the process and/or the abovementioned biosynthesis genes are the sequences encoding further genes of the phytosterol biosynthetic pathway, such as the enzymes catalyzing the production of acetyl CoA_HMGCoA, mevalonate, mevalonate 5 phosphate, mevalonate 5-pyrophosphate, isopentyl diphosphate, 5-pyrophosphatemevalonate, isopentyl pyrophosphate (PIP), dimethylallyl pyrophosphate (DMAPP), PIP+DMAPP, geranyl pyrophosphate+IPP, farnesyl pyrophosphate, 2 farnesyl pyrophosphate, squalene (squalene synthase) and squalene epoxide, or cycloartenol synthase controling the cyclization of squalene epoxide, S-adenosyl-L-methionine:sterol C-24 methyl transferase (EC 2.1.1.41) (SMT1) catalyzing the transfer of a methyl group from a cofactor, SMT2 catalyzing the second methyl transfer reaction, sterol C-14 demethylase catalyzing the demethylation at C-14, removing the methyl group and creating a double bond. These genes can lead to an increased synthesis of the essential phytosterol, in particular, of beta-sitosterol and/or campesterol resp..

**[0230.0.0.12]** for the disclosure of this paragraph see paragraph [0230.0.0.0] above.

**[0231.0.12.12]** In a further advantageous embodiment of the process of the invention, the organisms used in the process are those in which simultaneously a protein degrading phytosterol, in particular, of beta-sitosterol and/or campesterol, resp., is attenuated, in particular by reducing the rate of expression of the corresponding gene.

**[0232.0.0.5] to [0276.0.0.5]** for the disclosure of the paragraphs [0232.0.0.5] to [0276.0.0.5] see paragraphs [0232.0.0.0] to [0276.0.0.0] above.

**[0277.0.12.12]**The respective fine chemical produced can be isolated from the organism by methods with which the skilled worker is familiar. For example, via extraction, salt precipitation, and/or different chromatography methods. The process according to the invention can be conducted batchwise, semibatchwise or continuously. The respective fine chemical produced by this process can be obtained by harvesting the organisms, either from the crop in which they grow, or from the field. This can be done via pressing or extraction of the plant parts.

**[0278.0.0.12] to [0282.0.0.12]** for the disclosure of the paragraphs [0278.0.0.12] to [0282.0.0.12] see paragraphs [0278.0.0.0] to [0282.0.0.0] above.

**[0283.0.12.12]** Moreover, a native polypeptide conferring the increase of the respective fine chemical in an organism or part thereof can be isolated from cells (e.g., endothelial cells), for example using the antibody of the present invention as described below, e.g. an antibody against a protein as indicated in Table II, column 3, lines 112 to 117 or lines 483 to 485 and/or lines 118 to 124 or lines 486 to 491, resp., or an antibody against a polypeptide as indicated in Table II, columns 5 or 7, lines 112 to 117 or lines 483 to 485 and/or lines 118 to 124 or lines 486 to 491, resp. which can be produced by standard techniques utilizing the polypeptid of the present invention or fragment thereof, i.e., the polypeptide of this invention. Preferred are monoclonal antibodies.

**[0284.0.0.12]** for the disclosure of this paragraph see [0284.0.0.0] above.

**[0285.0.12.12]** In one embodiment, the present invention relates to a polypeptide having a sequence as indicated in Table II, columns 5 or 7, lines 112 to 117 or lines 483 to 485 and/or lines 118 to 124 or lines 486 to 491, resp., or as encoded by a nucleic acid molecule as indicated in Table I, columns 5 or 7, lines 112 to 117 or lines 483 to 485 and/or lines 118 to 124 or lines 486 to 491, resp., or functional homologues thereof.

**[0286.0.12.12]**In one advantageous embodiment, in the method of the present invention the activity of a polypeptide is increased which comprises or consists of a consensus sequence as indicated in Table IV, column 7, lines 112 to 117 or lines 483 to 485 and/or lines 118 to 124 or lines 486 to 491, resp. In another embodiment, the present invention relates to a polypeptide comprising or consisting of a consensus sequence as indicated in Table IV, column 7, lines 112 to 117 or lines 483 to 485 and/or lines 118 to 124 or lines 486 to 491, resp., whereby 20 or less, preferably 15 or 10, preferably 9, 8, 7, or 6, more preferred 5 or 4, even more preferred 3, even more preferred 2, even more preferred 1, most preferred 0 of the amino acids positions indicated can be replaced by any amino acid or, in an further embodiment, can be replaced and/or absent. In one embodiment, the present invention relates to the method of the present invention comprising a polypeptide or to a polypeptide comprising more than one consensus sequences (of an individual line) as indicated in Table IV, column 7, lines 112 to 117 or lines 483 to 485 and/or lines 118 to 124 or lines 486 to 491.

**[0287.0.0.12] to [0290.0.0.12]** for the disclosure of the paragraphs [0287.0.0.12] to [0290.0.0.12] see paragraphs [0287.0.0.0] to [0290.0.0.0] above.

**[0291.0.12.12]** In one advantageous embodiment, the method of the present invention comprises the increasing of a polypeptide comprising or consisting of plant or microorganism specific consensus sequences.
In one embodiment, said polypeptide of the invention distinguishes over a sequence as indicated in Table IIA or IIB, columns 5 or 7, lines 112 to 117 or lines 483 to 485 and/or lines 118 to 124 or lines 486 to 491, resp., by one or more amino acids. In one embodiment, polypeptide distinguishes from a sequence as indicated in Table IIA or IIB, columns 5 or 7, lines 112 to 117 or lines 483 to 485 and/or lines 118 to 124 or lines 486 to 491, resp., by more than 5, 6, 7, 8 or 9 amino acids, preferably by more than 10, 15, 20, 25 or 30 amino acids, evenmore preferred are more than 40, 50, or 60 amino acids and, preferably, the sequence of the polypeptide of the invention distinguishes from a sequence as indicated in Table IIA or IIB, columns 5 or 7, lines 112 to 117 or lines 483 to 485 and/or lines 118 to 124 or lines 486 to 491, resp., by not more than 80% or 70% of the amino acids, preferably not more than 60% or 50%, more preferred not more than 40% or 30%, even more preferred not more than 20% or 10%. In an other embodiment, said polypeptide of the invention does not consist of a sequence as indicated in Table IIA or IIB, columns 5 or 7, lines 112 to 117 or lines 483 to 485 and/or lines 118 to 124 or lines 486 to 491, resp.

**[0292.0.0.12]** for the disclosure of this paragraph see [0292.0.0.0] above.

**[0293.0.12.12]** In one embodiment, the invention relates to polypeptide conferring an increase in the respective fine chemical in an organism or part thereof and being encoded by the nucleic acid molecule of the invention or a nucleic acid molecule used in the process of the invention. In one embodiment, said polypeptide is having a sequence which distinguishes from a sequence as indicated in Table IIA or IIB, columns 5 or 7, lines 112 to 117 or lines 483 to 485 and/or lines 118 to 124 or lines 486 to 491, resp., by one or more amino acids. In an other embodiment, the polypeptide of the invention does not consist of the sequence as indicated in Table IIA or IIB, columns 5 or 7, lines 112 to 117 or lines 483 to 485 and/or lines 118 to 124 or lines 486 to 491, resp. In a further embodiment, the polypeptide of the present invention is less than 100%, 99,999%, 99,99%, 99,9% or 99% identical.

**[0294.0.12.12]** In one embodiment, the present invention relates to a polypeptide having an activity of a protein as indicated in Table IIA or IIB, column 3, lines 112 to 117 or lines 483 to 485 and/or lines 118 to 124 or lines 486 to 491, resp., which distinguishes over a sequence as indicated in Table IIA or IIB, columns 5 or 7, lines 112 to 117 or lines 483 to 485 and/or lines 118 to 124 or lines 486 to 491, resp., by one or more amino acids, preferably by more than 5, 6, 7, 8 or 9 amino acids, preferably by more than 10, 15, 20, 25 or 30 amino acids, evenmore preferred are more than 40, 50, or 60 amino acids but even more preferred by less than 70% of the amino acids, more preferred by less than 50%, even more preferred my less than 30% or 25%, more preferred are 20% or 15%, even more preferred are less than 10%.

**[0295.0.0.12] to [0297.0.0.12]** for the disclosure of the paragraphs [0295.0.0.12] to [0297.0.0.12] see paragraphs [0295.0.0.0] to [0297.0.0.0] above.

**[0297.1.12.12]** Non polypeptide of the invention-chemicals are e.g. polypeptides having not the activity of a polypeptide indicated in Table II, columns 3, 5 or 7, lines 112 to 117 or lines 483 to 485 and/or lines 118 to 124 or lines 486 to 491, resp.

**[0298.0.12.12]** A polypeptide of the invention can participate in the process of the present invention. The polypeptide or a portion thereof comprises preferably an amino acid sequence, which is sufficiently homologous to an amino acid sequence as indicated in Table II, columns 5 or 7, lines 112 to 117 or lines 483 to 485 and/or lines 118 to 124 or lines 486 to 491, resp. The portion of the protein is preferably a biologically active portion as described herein. Preferably, the polypeptide used in the process of the invention has an amino acid sequence identical to a sequence as indicated in Table II, columns 5 or 7, lines 112 to 117 or lines 483 to 485 and/or lines 118 to 124 or lines 486 to 491, resp.

**[0299.0.12.12]** Further, the polypeptide can have an amino acid sequence which is encoded by a nucleotide sequence which hybridizes, preferably hybridizes under stringent conditions as described above, to a nucleotide sequence of the nucleic acid molecule of the present invention. Accordingly, the polypeptide has an amino acid sequence which is encoded by a nucleotide sequence that is at least about 35%, 40%, 45%, 50%, 55%, 60%, 65% or 70%, preferably at least about 75%, 80%, 85% or 90, and more preferably at least about 91%, 92%, 93%, 94% or 95%, and even more preferably at least about 96%, 97%, 98%, 99% or more homologous to one of the nucleotide sequence as indicated in Table I, columns 5 or 7, lines 112 to 117 or lines 483 to 485 and/or lines 118 to 124 or lines 486 to 491, resp. The preferred polypeptide of the present invention preferably possesses at least one of the activities according to the invention and described herein. A preferred polypeptide of the present invention includes an amino acid sequence encoded by a nucleotide sequence which hybridizes, preferably hybridizes under stringent conditions, to a nucleotide sequence as indicated in Table I, lines 112 to 117 or lines 483 to 485 and/or lines 118 to 124 or lines 486 to 491, resp., or which is homologous thereto, as defined above.

**[0300.0.12.12]** Accordingly the polypeptide of the present invention can vary from a sequence as indicated in Table II, columns 5 or 7, lines 112 to 117 or lines 483 to 485 and/or lines 118 to 124 or lines 486 to 491, resp., in amino acid sequence due to natural variation or mutagenesis, as described in detail herein. Accordingly, the polypeptide comprise an amino acid sequence which is at least about 35%, 40%, 45%, 50%, 55%, 60%, 65% or 70%, preferably at least about 75%, 80%, 85% or 90, and more preferably at least about 91%, 92%, 93%, 94% or 95%, and most preferably at least about 96%, 97%, 98%, 99% or more homologous to an entire amino acid sequence of a sequence as indicated in Table II, columns 5 or 7, lines 112 to 117 or lines 483 to 485 and/or lines 118 to 124 or lines 486 to 491, resp..

**[0301.0.0.12]** for the disclosure of this paragraph see [0301.0.0.0] above.

**[0302.0.12.12]** Biologically active portions of an polypeptide of the present invention include peptides comprising amino acid sequences derived from the amino acid sequence of the polypeptide of the present invention or used in the process of the present invention, e.g., the amino acid sequence as indicated in Table II, columns 5 or 7, lines 112 to 117 or lines 483 to 485 and/or lines 118 to 124 or lines 486 to 491, resp., or the amino acid sequence of a protein homologous thereto, which include fewer amino acids than a full length polypeptide of the present invention or used in the process of the present invention or the full length protein which is homologous to an polypeptide of the present invention or used in the process of the present invention depicted herein, and exhibit at least one activity of polypeptide of the present invention or used in the process of the present invention.

**[0303.0.0.12]** for the disclosure of this paragraph see [0303.0.0.0] above.

**[0304.0.12.12]** Manipulation of the nucleic acid molecule of the invention may result in the production of a protein having essentially the activity of the polypeptides as indicated in Table II, column 3, lines 112 to 117 or lines 483 to 485 and/or lines 118 to 124 or lines 486 to 491, resp., but having differences in the sequence from said wild-type protein. These proteins may be improved in efficiency or activity, may be present in greater numbers in the cell than is usual, or may be decreased in efficiency or activity in relation to the wild type protein.

**[0305.0.0.12], [0306.0.0.12] and [0306.1.0.12]** for the disclosure of the paragraphs [0305.0.0.12], [0306.0.0.12] and [0306.1.0.12] see paragraphs [0305.0.0.0], [0306.0.0.0] and [0306.1.0.0] above.

**[0307.0.0.12] and [0308.0.0.12]** for the disclosure of the paragraphs [0307.0.0.12] and [0308.0.0.12] see paragraphs [0307.0.0.0 and [0308.0.0.0] above.

**[0309.0.12.12]** In one embodiment, a reference to a protein (= polypeptide) of the invention or as indicated in Table II, columns 5 or 7, lines 112 to 117 or lines 483 to 485 and/or lines 118 to 124 or lines 486 to 491, resp., refers to a polypeptide having an amino acid sequence corresponding to the polypeptide of the invention or used in the process of the invention, whereas a " non-polypeptide of the invention" or "other polypeptide" not being indicated in Table II, columns 5 or 7, lines 112 to 117 or lines 483 to 485 and/or lines 118 to 124 or lines 486 to 491, resp., refers to a polypeptide having an amino acid sequence corresponding to a protein which is not substantially homologous a polypeptide of the invention, preferably which is not substantially homologous to a polypeptide as indicated in Table II, columns 5 or 7, lines 112 to 117 or lines 483 to 485 and/or lines 118 to 124 or lines 486 to 491, resp., e.g., a protein which does not confer the activity described herein or annotated or known for as indicated in Table II, column 3, lines 112 to 117 or lines 483 to 485 and/or lines 118 to 124 or lines 486 to 491, resp., and which is derived from the same or a different organism. In one embodmeint, a "non-polypeptide of the invention" or "other polypeptide" not being indicated in Table II, columns 5 or 7, lines 112 to 117 or lines 483 to 485 and/or lines 118 to 124 or lines 486 to 491, resp., does not confer an increase of the respective fine chemical in an organism or part therof.

**[0310.0.0.12] to [0334.0.0.12]** for the disclosure of the paragraphs [0310.0.0.12] to [0334.0.0.12] see paragraphs [0310.0.0.0] to [0334.0.0.0] above.

**[0335.0.12.12]** The dsRNAi method has proved to be particularly effective and advantageous for reducing the expression of a nucleic acid sequences as indicated in Table I, columns 5 or 7, lines 112 to 117 or lines 483 to 485 and/or lines 118 to 124 or lines 486 to 491, resp., and/or homologs thereof. As described inter alia in WO 99/32619, dsRNAi approaches are clearly superior to traditional antisense approaches. The invention therefore furthermore relates to double-stranded RNA molecules (dsRNA molecules) which, when introduced into an organism, advantageously into a plant (or a cell, tissue, organ or seed derived therefrom), bring about altered metabolic activity by the reduction in the expression of a nucleic acid sequences as indicated in Table I, columns 5 or 7, lines 112 to 117 or lines 483 to 485 and/or lines 118 to 124 or lines 486 to 491, resp., and/or homologs thereof. In a double-stranded RNA molecule for reducing the expression of an protein encoded by a nucleic acid sequence sequences as indicated in Table I, columns 5 or 7, lines 112 to 117 or lines 483 to 485 and/or lines 118 to 124 or lines 486 to 491, resp., and/or homologs thereof, one of the two RNA strands is essentially identical to at least part of a nucleic acid sequence, and the respective other RNA strand is essentially identical to at least part of the complementary strand of a nucleic acid sequence.

**[0336.0.0.12] to [0342.0.0.12]** for the disclosure of the paragraphs [0336.0.0.12] to [0342.0.0.12] see paragraphs [0336.0.0.0] to [0342.0.0.0] above.

**[0343.0.12.12]** As has already been described, 100 % sequence identity between the dsRNA and a gene transcript of a nucleic acid sequence as indicated in Table I, columns 5 or 7, lines 112 to 117 or lines 483 to 485 and/or lines 118 to 124 or lines 486 to 491, resp., or it's homolog is not necessarily required in order to bring about effective reduction in the expression. The advantage is, accordingly, that the method is tolerant with regard to sequence deviations as may be present as a consequence of genetic mutations, polymorphisms or evolutionary divergences. Thus, for example, using the dsRNA, which has been generated starting from a sequence as indicated in Table I, columns 5 or 7, lines 112 to 117 or lines 483 to 485 and/or lines 118 to 124 or lines 486 to 491, resp., or homologs thereof of the one organism, may be used to suppress the corresponding expression in another organism.

**[0344.0.0.12] to [0361.0.0.12]** for the disclosure of the paragraphs [0344.0.0.12] to [0361.0.0.12] see paragraphs [0344.0.0.0] to [0361.0.0.0] above.

**[0362.0.12.12]** Accordingly the present invention relates to any cell transgenic for any nucleic acid characterized as part of the invention, e.g. conferring the increase of the respective fine chemical in a cell or an organism or a part thereof, e.g. the nucleic acid molecule of the invention, the nucleic acid construct of the invention, the antisense molecule of the invention, the vector of the invention or a nucleic acid molecule encoding the polypeptide of the invention, e.g. the polypeptide as indicated in Table II, columns 5 or 7, lines 112 to 117 or lines 483 to 485 and/or lines 118 to 124 or lines 486 to 491, resp. e.g. encoding a polypeptide having protein activity, as indicated in Table II, column 3, lines lines 112 to 117 or lines 483 to 485 and/or lines 118 to 124 or lines 486 to 491, resp. Due to the above mentioned activity the respective fine chemical content in a cell or an organism is increased. For example, due to modulation or manipulation, the cellular activity of the polypeptide of the invention or nucleic acid molecule of the invention is increased, e.g. due to an increased expression or specific activity of the subject matters of the invention in a cell or an organism or a part thereof. Transgenic for a polypeptide having an activity of a polypeptide as indicated in Table II, columns 5 or 7, lines 112 to 117 or lines 483 to 485 and/or lines 118 to 124 or lines 486 to 491, resp., means herein that due to modulation or manipulation of the genome, an activity as annotated for a polypeptide as indicated in Table II, column 3, lines 112 to 117 or lines 483 to 485 and/or lines 118 to 124 or lines 486 to 491, resp. e.g. having a sequence as indicated in Table II, columns 5 or 7, lines 112 to 117 or lines 483 to 485 and/or lines 118 to 124 or lines 486 to 491, resp., is increased in a cell or an organism or a part thereof. Examples are described above in context with the process of the invention

**[0363.0.0.12]** for the disclosure of this paragraph see paragraph [0363.0.0.0] above.

**[0364.0.12.12]** A naturally occurring expression cassette - for example the naturally occurring combination of a promoter of a polypeptide of the invention as indicated in Table II, column 3, lines 112 to 117 or lines 483 to 485 and/or lines 118 to 124 or lines 486 to 491, resp., with the corresponding protein-encoding sequence as indicated in Table I, column 3, lines 112 to 117 or lines 483 to 485 and/or lines 118 to 124 or lines 486 to 4914, resp., - becomes a transgenic expression cassette when it is modified by non-natural, synthetic "artificial" methods such as, for example, mutagenization. Such methods have been described (US 5,565,350; WO 00/15815; also see above).

**[0365.0.0.12] to [0373.0.0.12]** for the disclosure of the paragraphs [0365.0.0.12], to [0373.0.0.12] see paragraphs [0365.0.0.0] to [0373.0.0.0] above.

**[0374.0.12.12]** Transgenic plants comprising the respective fine chemical synthesized in the process according to the invention can be marketed directly without isolation of the compounds synthesized. In the process according to the invention, plants are understood as meaning all plant parts, plant organs such as leaf, stalk, root, tubers or seeds or propagation material or harvested material or the intact plant. In this context, the seed encompasses all parts of the seed such as the seed coats, epidermal cells, seed cells, endosperm or embryonic tissue. Phytosterol, in particular, of beta-sitosterol and/or campesterol resp., produced in the process according to the invention may, however, also be isolated from the plant in the form of their free form or bound in or to compounds or moieties. Phytosterol, in particular, of beta-sitosterol and/or campesterol resp., produced by this process can be harvested by harvesting the organisms either from the culture in which they grow or from the field. This can be done via expressing, grinding and/or extraction, salt precipitation and/or ion-exchange chromatography or other chromatographic methods of the plant parts, preferably the plant seeds, plant fruits, plant tubers and the like.

**[0375.0.0.12] and [0376.0.0.12]** for the disclosure of the paragraphs [0375.0.0.12] and [0376.0.0.12] see paragraphs [0375.0.0.0] and [0376.0.0.0] above.

**[0377.0.12.12]** Accordingly, the present invention relates also to a process according to the present invention whereby the produced phytosterol, in particular, of beta-sitosterol and/or campesterol comprising composition or the produced the respective fine chemical is isolated.

**[0378.0.12.12]** In this manner, more than 50% by weight, advantageously more than 60% by weight, preferably more than 70% by weight, especially preferably more than 80% by weight, very especially preferably more than 90% by weight, of the phytosterol, in particular, of beta-sitosterol and/or campesterol, resp., produced in the process can be isolated. The resulting phytosterol, in particular, of beta-sitosterol and/or campesterol resp., can, if appropriate, subsequently be further purified, if desired mixed with other active ingredients such as vitamins, amino acids, carbohydrates, antibiotics and the like, and, if appropriate, formulated.

**[0379.0.12.12]** In one embodiment, the phytosterol, in particular, of beta-sitosterol and/or campesterol resp., is a mixture comprising of one or more the respective fine chemicals. In one embodiment, the respective fine chemical means here phytosterol, in particular, of beta-sitosterol and/or campesterol. In one embodiment, phytosterol means here a mixture of the respective fine chemicals.

**[0380.0.12.12]** The phytosterol, in particular, the beta-sitosterol and/or campesterol resp., obtained in the process are suitable as starting material for the synthesis of further products of value. For example, they can be used in combination with each other or alone for the production of pharmaceuticals, foodstuffs, animal feeds or cosmetics. Accordingly, the present invention relates a method for the production of pharmaceuticals, food stuff, animal feeds, nutrients or cosmetics comprising the steps of the process according to the invention, including the isolation of the phytosterol comprising composition produced or the respective fine chemical produced if desired and formulating the product with a pharmaceutical acceptable carrier or formulating the product in a form acceptable for an application in agriculture. A further embodiment according to the invention is the use of the phytosterol resp., produced in the process or of the transgenic organisms in animal feeds, foodstuffs, medicines, food supplements, cosmetics or pharmaceuticals.

**[0381.0.0.12] and [0382.0.0.12]** for the disclosure of the paragraphs [0381.0.0.12] and [0382.0.0.12] see paragraphs [0381.0.0.0] and [0382.0.0.0] above.

**[0383.0.12.12]** For preparing sterol compound-containing fine chemicals, in particular the respective fine chemical of the invention, it is possible to use as source organic compounds such as, for example, acetyl CoA_HMGCoA, mevalonate, mevalonate 5 phosphate, mevalonate 5-pyrophosphate, isopentyl diphosphate, 5-pyrophosphatemevalonate, isopentyl pyrophosphate (PIP), dimethylallyl pyrophosphate (DMAPP), PIP+DMAPP, geranyl pyrophosphate+IPP, farnesyl pyrophosphate, 2 farnesyl pyrophosphate, squalene and squalene epoxide, cycloartenol and for preparing esters comprising the phytosterols of the invention oils, fats and/or lipids comprising fatty acids such as fatty acids having a carbon back bone between C₁₀- to C₁₆- carbon atoms and/or small organic acids such acetic acid, propionic acid or butanoic acid as precursor compounds.

**[0384.0.0.12]** for the disclosure of this paragraph see paragraph [0384.0.0.0] above.

**[0385.0.12.12]** The fermentation broths obtained in this way, containing in particular phytosterol, in particular, of beta-sitosterol and/or campesterol resp., in mixtures with other compounds, in particular with other sterols or vitamins, e.g. with carotenoids, e.g. with astaxanthin, or fatty acids or containing microorganisms or parts of microorganisms, like plastids, containing phytosterol, in particular, of beta-sitosterol and/or campesterol resp., in mixtures with other compounds, e.g. with vitamins, normally have a dry matter content of from 7.5 to 25% by weight. Sugar-limited fermentation is additionally advantageous, e.g. at the end, for example over at least 30% of the fermentation time. This means that the concentration of utilizable sugar in the fermentation medium is kept at, or reduced to, 0 to 3 g/I during this time. The fermentation broth is then processed further. Depending on requirements, the biomass can be removed or isolated entirely or partly by separation methods, such as, for example, centrifugation, filtration, decantation, coagulation/flocculation or a combination of these methods, from the fermentation broth or left completely in it. The fermentation broth can then be thickened or concentrated by known methods, such as, for example, with the aid of a rotary evaporator, thin-film evaporator, falling film evaporator, by reverse osmosis or by nanofiltration. This concentrated fermentation broth can then be worked up by freeze-drying, spray drying, spray granulation or by other processes.
As phytosterol is often localized in membranes or plastids, in one embodiment it is advantageous to avoid a leaching of the cells when the biomass is isolated entirely or partly by separation methods, such as, for example, centrifugation, filtration, decantation, coagulation/flocculation or a combination of these methods, from the fermentation broth. The dry biomass can directly be added to animal feed; provided the vitamin E concentration is sufficiently high and no toxic compounds are present.

**[0386.0.12.12]** Accordingly, it is possible to further purify the produced phytosterol, in particular, of beta-sitosterol and/or campesterol resp.. For this purpose, the product-containing composition, e.g. a total or partial lipid extraction fraction using organic solvents, e.g. as described above, is subjected for example to a saponification to remove triglycerides, partition between e.g. hexane/methanol (seperation of non-polar epiphase from more polar hypophasic derivates) and separation via e.g. an open column chromatography, preparative thin layer chromatography or HPLC in which case the desired product or the impurities are retained wholly or partly on the chromatography resin (see e.g. Kaluzny et al., J Lipid Res 1985; 26: 135-140). These chromatography steps can be repeated if necessary, using the same or different chromatography resins. The skilled worker is familiar with the choice of suitable chromatography resins and their most effective use.

**[0387.0.0.12] to [0392.0.0.12]** for the disclosure of the paragraphs [0387.0.0.12] to [0392.0.0.12] see paragraphs [0387.0.0.0] to [0392.0.0.0] above.

**[0393.0.12.12]** In one embodiment, the present invention relates to a method for the identification of a gene product conferring an increase in the respective fine chemical production in a cell, comprising the following steps:
(g) contacting,̅ e.g. hybridising, the nucleic acid molecules of a sample, e.g. cells, tissues, plants or microorganisms or a nucleic acid library , which can contain a candidate gene encoding a gene product conferring an increase in the respective fine chemical after expression, with the nucleic acid molecule of the present invention;
(h) identifying the nucleic acid molecules, which hybridize under relaxed stringent conditions with the nucleic acid molecule of the present invention in particular to the nucleic acid molecule sequence as indicated in Table I, columns 5 or 7, lines 112 to 117 or lines 483 to 485 and/or lines 118 to 124 or lines 486 to 491, preferably in Table I B, clumns 5 or 7, lines 112 to 117 or lines 483 to 485 and/or lines 118 to 124 or lines 486 to 491 resp., and, optionally, isolating the full length cDNA clone or complete genomic clone;
(i) introducing the candidate nucleic acid molecules in host cells, preferably in a plant cell or a microorganism, appropriate for producing the respective fine chemical;
(j) expressing the identified nucleic acid molecules in the host cells;
(k) assaying the the respective fine chemical level in the host cells; and
(I) identifying the nucleic acid molecule and its gene product which expression confers an increase in the respective fine chemical level in the host cell after expression compared to the wild type.

**[0394.0.0.12] to [0399.0.0.12]** for the disclosure of the paragraphs [0394.0.0.12] to [0399.0.0.12] see paragraphs [0394.0.0.0] to [0399.0.0.0] above.

**[0399.1.12.12]**One can think to screen for increased production of the respective fine chemical by for example searching for a resistance to a drug blocking the synthesis of the respective fine chemical and looking whether this effect is dependent on the activity or expression of a polypeptide as indicated in Table II, columns 5 or 7, lines 112 to 117 or lines 483 to 485 and/or lines 118 to 124 or lines 486 to 491, resp., or a homolog thereof, e.g. comparing the phenotyp of nearly identical organisms with low and high activity of a protein as indicated in Table II, columns 5 or 7, lines 112 to 117 or lines 483 to 485 and/or lines 118 to 124 or lines 486 to 491, resp., after incubation with the drug.

**[0400.0.0.12] to [0416.0.0.12]** for the disclosure of the paragraphs [0400.0.0.12] to [0416.0.0.12] see paragraphs [0400.0.0.0] to [0416.0.0.0] above.

**[0417.0.12.12]**The nucleic acid molecule of the invention, the vector of the invention or the nucleic acid construct of the invention may also be useful for the production of organisms resistant to inhibitors of the phytosterol production biosynthesis pathways. In particular, the overexpression of the polypeptide of the present invention may protect an organism such as a microorganism or a plant against inhibitors, which block the phytosterol, in particular the respective fine chemical, synthesis in said organism. Examples of inhibitors or herbicides blocking the phytosterol synthesis in organism such as microorganism or plants are for example compounds which inhibit the cytochrom P450 such as Tetcyclasis, triazoles like Paclobutrazol or Epoxiconazol, pyridines like Obtusifoliol, demethylases inhibitors, or compounds like Mevilonin, which inhibits the HMG-CoA reductase.

**[0418.0.0.12] to [0423.0.0.12]** for the disclosure of the paragraphs [0418.0.0.12] to [0423.0.0.12] see paragraphs [0418.0.0.0] to [0423.0.0.0] above.

**[0424.0.12.12]** Accordingly, the nucleic acid of the invention, the polypeptide of the invention, the nucleic acid construct of the invention, the organisms, the host cell, the microorganisms, the plant, plant tissue, plant cell, or the part thereof of the invention, the vector of the invention, the agonist identified with the method of the invention, the nucleic acid molecule identified with the method of the present invention, can be used for the production of the respective fine chemical or of the respective fine chemical and one or more other sterols, phytosterols, carotenoids, vitamins or fatty acids. Accordingly, the nucleic acid of the invention, or the nucleic acid molecule identified with the method of the present invention or the complement sequences thereof, the polypeptide of the invention, the nucleic acid construct of the invention, the organisms, the host cell, the microorganisms, the plant, plant tissue, plant cell, or the part thereof of the invention, the vector of the invention, the antagonist identified with the method of the invention, the antibody of the present invention, the antisense molecule of the present invention, can be used for the reduction of the respective fine chemical in a organism or part thereof, e.g. in a cell.

**[0425.0.0.12] to [0430.0.0.12]** for the disclosure of the paragraphs [0425.0.0.12] to [0430.0.0.12] see paragraphs [0425.0.0.0] to [0430.0.0.0] above.

**[0431.0.12.12]** Example 1: Cloning SEQ ID NO: 12135 in Escherichia coli

**[0432.0.12.12]** SEQ ID NO: 12135 was cloned into the plasmids pBR322 (Sutcliffe, J.G. (1979) Proc. Natl Acad. Sci. USA, 75: 3737-3741); pACYC177 (Change & Cohen (1978) J. Bacteriol. 134: 1141-1156); plasmids of the pBS series (pBSSK+, pBSSK- and others; Stratagene, LaJolla, USA) or cosmids such as SuperCos1 (Stratagene, LaJolla, USA) or Lorist6 (Gibson, T.J. Rosenthal, A., and Waterson, R.H. (1987) Gene 53: 283-286) for expression in E. coli using known, well-established procedures (see, for example, Sambrook, J. et al. (1989) "Molecular Cloning: A Laboratory Manual". Cold Spring Harbor Laboratory Press or Ausubel, F.M. et al. (1994) "Current Protocols in Molecular Biology", John Wiley & Sons).

**[0433.0.0.12] and [0434.0.0.12]** for the disclosure of the paragraphs [0433.0.0.12] and [0434.0.0.12] see paragraphs [0433.0.0.0] and [0434.0.0.0] above.

**[0435.0.12.12]** Example 3: In-vivo and in-vitro mutagenesis

**[0436.0.12.12]** An *in vivo* mutagenesis of organisms such as green algae (e.g. Spongiococcum sp, e.g. Spongiococcum exentricum, Chlorella sp., Haematococcus, Phaedactylum tricornatum, Volvox or Dunaliella), Synchocytic spec. PLL 6803, Physocmetrella patens, Saccharomyces, Mortierella, Escherichia and others mentioned above, which are beneficial for the production of phytosterol can be carried out by passing a plasmid DNA (or another vector DNA) containing the desired nucleic acid sequences, e.g. the nucleic acid molecule of the invention or the vector of the invention, or nucleic acid sequences through E. coli and other microorganisms (for example Bacillus spp. or yeasts such as Saccharomyces cerevisiae) which are not capable of maintaining the integrity of its genetic information. Usual mutator strains have mutations in the genes for the DNA repair system [for example mutHLS, mutD, mutT and the like; for comparison, see Rupp, W.D. (1996) DNA repair mechanisms in Escherichia coli and Salmonella, pp. 2277-2294, ASM: Washington]. The skilled worker knows these strains. The use of these strains is illustrated for example in Greener, A. and Callahan, M. (1994) Strategies 7; 32-34.
In-vitro mutation methods such as increasing the spontaneous mutation rates by chemical or physical treatment are well known to the skilled person. Mutagens like 5-bromo-uracil, N-methyl-N-nitro-N-nitrosoguanidine (= NTG), ethyl methanesulfonate (= EMS), hydroxylamine and/or nitrous acid are widly used as chemical agents for random in-vitro mutagensis. The most common physical method for mutagensis is the treatment with UV irradiation. Another random mutagenesis technique is the error-prone PCR for introducing amino acid changes into proteins. Mutations are deliberately introduced during PCR through the use of error-prone DNA polymerases and special reaction conditions known to a person skilled in the art. For this method randomized DNA sequences are cloned into expression vectors and the resulting mutant libraries screened for altered or improved protein activity as described below.
Site-directed mutagensis method such as the introduction of desired mutations with an M13 or phagemid vector and short oligonucleotides primers is a well-known approach for site-directed mutagensis. The clou of this method involves cloning of the nucleic acid sequence of the invention into an M13 or phagemid vector, which permits recovery of single-stranded recombinant nucleic acid sequence. A mutagenic oligonucleotide primer is then designed whose sequence is perfectly complementary to nucleic acid sequence in the region to be mutated, but with a single difference: at the intended mutation site it bears a base that is complementary to the desired mutant nucleotide rather than the original. The mutagenic oligonucleotide is then allowed to prime new DNA synthesis to create a complementary full-length sequence containing the desired mutation. Another site-directed mutagensis method is the PCR mismatch primer mutagensis method also known to the skilled person. Dpnl site-directed mutagensis is a further known method as described for example in the Stratagene QuickchangeTM site-directed mutagenesis kit protocol. A huge number of other methods are also known and used in common practice.
Positive mutation events can be selected by screening the organisms for the production of the desired respective fine chemical.

**[0437.0.5.12] to [0440.0.5.12] and [0441.0.0.12]** for the disclosure of the paragraphs [0437.0.5.12] to [0440.0.5.12] and [0441.0.0.12] see paragraphs [0437.0.5.5] to [0444.0.5.5] and [0441.0.0.0]above.

**[0442.0.5.12], [0443.0.0.12], [0444.0.5.12] and [0445.0.5.12]** for the disclosure of the paragraphs [0442.0.5.12], [0443.0.0.12], [0444.0.5.12] and [0445.0.5.12] see paragraphs [0442.0.5.5], [0443.0.0.0], [0444.0.5.5] and [0445.0.5.5] above.

**[0446.0.0.12] to [0450.0.0.12] and [0451.0.5.12]** for the disclosure of the paragraphs [0446.0.0.12] to [0450.0.0.12] and [0451.0.5.12] see paragraphs [0446.0.0.0] to [0450.0.0.0] and [0451.0.5.5] above.

**[0452.0.0.12] to [0454.0.0.12], [0455.0.5.12] and [0456.0.0.12]** for the disclosure of the paragraphs [0452.0.0.12] to [0454.0.0.12], [0455.0.5.12] and [0456.0.0.12] see [0452.0.0.0] to [0454.0.0.0], [0455.0.5.5] and [0456.0.0.0] above.

**[0457.0.12.12]**Example 9: Purification of the phytosterol

**[0458.0.12.12]** One example is the analysis of phytosterol: the content of the phytosterols of the invention can be determinated by gas chromatography with flame ionisation detection (GC-FID; column SAC-5, 30 m x 0.25 mm, 0.25 µm, samples not silylated) using standards for these phytosterols. Another method is the detection by gas chromatography-mass spectrometry (GC-MS) using the same type of column as indicated above.
For the analysis of the concentrations of sterols by gas chromatography mass spectrometry a Hewlett-Packard (HP) 5890 gas chromatograph equipped with an NB-54 fused-silica capillary column (15 mx0.20mm I.D.; Nordion, Helsinki, Finland) and interfaced with an HP 5970A mass spectrometry detector operating in electron impact mode (70 eV) can be used. The column oven is programmed from 230 °C to 285 °C at 10 °C/min and injector and detector should be at 285 °C. The lipids from the samples (200 µl) are extracted with chloroform/methanol (2:1) and transesterified with sodium methoxide. The released free sterols are trimethylsilylated as described previously (Gylling et al. J. Lipid Res 40: 593-600, 1999) and quantified by single ion monitoring technique using m/z 129 (cholesterol, campesterol and β-sitosterol), m/z 215 (β-sitostanol), m/z 343 (desmosterol), m/z 255 (lathosterol) and m/z 217 (5-α-cholestane, internal standard) as selected ions (Vaskonen, Dissertation, Biomedicum Helsinki, June 19, 2002).

**[0459.0.12.12]** If required and desired, further chromatography steps with a suitable resin may follow. Advantageously, the phytosterol, in particular, of beta-sitosterol and/or campesteroll, can be further purified with a so-called RTHPLC. As eluent acetonitrile/water or chloroform/acetonitrile mixtures can be used. If necessary, these chromatography steps may be repeated, using identical or other chromatography resins. The skilled worker is familiar with the selection of suitable chromatography resin and the most effective use for a particular molecule to be purified.

**[0460.0.0.12]** for the disclosure of this paragraph see [0460.0.0.0] above.

**[0461.0.12.12]** Example 10: Cloning SEQ ID NO: 12135 for the expression in plants

**[0462.0.0.12]** for the disclosure of this paragraph see [0462.0.0.0] above.

**[0463.0.12.12]** SEQ ID NO: 12135 is amplified by PCR as described in the protocol of the Pfu Turbo or DNA Herculase polymerase (Stratagene).

**[0464.0.0.12] to [0466.0.0.12]** for the disclosure of the paragraphs [0464.0.0.12] to [0466.0.0.12] see paragraphs [0464.0.0.0] to [0466.0.0.0] above.

**[0467.0.12.12]**The following primer sequences were selected for the gene SEQ ID NO: 12135:
i) forward primer (SEQ ID NO: 12137)
   ATGGAACAGAACAGGTTCAAGAAAG
ii) reverse primer (SEQ ID NO: 12138)
   TTACAGTTTTTGTTTAGTCGTTTTAAC

**[0468.0.0.12] to [0479.0.0.12]** for the disclosure of the paragraphs [0468.0.0.12] to [0479.0.0.12] see paragraphs [0468.0.0.0] to [0479.0.0.0] above.

**[0480.0.12.12]** Example 11: Generation of transgenic plants which express SEQ ID NO: 12135

**[0481.0.0.12] to [0513.0.0.12]** for the disclosure of the paragraphs [0481.0.0.12] to [0513.0.0.12] see paragraphs [0482.0.0.0] to [0513.0.0.0] above.

**[0514.0.12.12]** As an alternative, phytosterols can be detected via HPLC, e.g. reversed-phase HPLC, as described by Heftmann, E. and Hunter, I.R. (J Chromatogr 1979; 165: 283-299). As separating principles of HPLC and GC are complementary, preparative reversed-phase HPLC followed by GC-MS analysis of the obtained sterol fractions is a preferred method to analyze sterols from natural products (Bianchini, J.-P. et al.; J Chromatogr 1985; 329: 231-246).
The results of the different plant analyses can be seen from the table, which follows:

**Table 1**

| **ORF** | **Metabolite** | **Method** | **Min** | **Max** |
|---|---|---|---|---|
| YER156C | beta-Sitosterol | GC | 1.18 | 1.39 |
| YER156C | Campesterol | GC | 1.17 | 1.95 |
| YER173W | Campesterol | GC | 1.23 | 1.51 |
| YKR057W | beta-Sitosterol | GC | 1.14 | 1.32 |
| YKR057W | Campesterol | GC | 1.20 | 1.39 |
| YOR044W | Campesterol | GC | 1.28 | 1.57 |
| YOR084W | beta-Sitosterol | GC | 3.59 | 3.59 |
| YPR172W | Campesterol | GC | 1.17 | 1.62 |
| b0019 | beta-Sitosterol | GC | 1.20 | 1.47 |
| b0421 | beta-Sitosterol | GC | 1.13 | 1.52 |
| b2699 | beta-Sitosterol | GC | 1.14 | 1.35 |
| b2699 | Campesterol | GC | 1.17 | 1.62 |
| b3256 | Campesterol | GC | 1.20 | 1.22 |
| b0050 | beta-Sitosterol | GC | 1,15 | 1,24 |
| b0161 | beta-Sitosterol | GC | 1,12 | 1,26 |
| b0161 | Campesterol | GC | 1,17 | 1,42 |
| b0464 | Campesterol | GC | 1,16 | 1,32 |
| b1896 | Campesterol | GC | 1,18 | 1,59 |
| b2341 | Campesterol | GC | 1,18 | 1,48 |
| b2478 | Campesterol | GC | 1,17 | 1,37 |
| b2822 | Campesterol | GC | 1,21 | 1,32 |
| b4129 | beta-Sitosterol | GC | 1,21 | 1,23 |

**[0515.0.12.12]** Column 2 shows the phytosterol analyzed. Columns 4 and 5 shows the ratio of the analyzed phytosterol between the transgenic plants and the wild type; Increase of the metabolites: Max: maximal x-fold (normalised to wild type)-Min: minimal x-fold (normalised to wild type). Decrease of the metabolites: Max: maximal x-fold (normalised to wild type) (minimal decrease), Min: minimal x-fold (normalised to wild type) (maximal decrease). Column 3 indicates the analytical method.

**[0516.0.0.12]** for the disclosure of this paragraph see [0516.0.0.0] above.

**[0517.0.12.12]** Example 14a: Engineering ryegrass plants by over-expressing YOR084W from Saccharomyces cerevisiae or other nucleic acids of the present invention.

**[0518.0.0.12] to [0524.0.0.12]** for the disclosure of the paragraphs [0518.0.0.12] to [0524.0.0.12] see paragraphs [0518.0.0.0] to [0524.0.0.0] above.

**[0525.0.12.12]** Example 14b: Engineering soybean plants by over-expressing YOR084W from Saccharomyces cerevisiae or other nucleic acids of the present invention.

**[0526.0.0.12] to [0529.0.0.12]** for the disclosure of the paragraphs [0526.0.0.12] to [0529.0.0.12] see paragraphs [0526.0.0.0] to [0529.0.0.0] above.

**[0530.0.12.12]** Example 14c: Engineering corn plants by over-expressing YOR084W from Saccharomyces cerevisiae or other nucleic acids of the present invention.

**[0530.1.0.12] to [0530.6.0.12]** for the disclosure of the paragraphs [0530.1.0.12] to [0530.6.0.12] see paragraphs [0530.1.0.0] to [0530.6.0.0] above.

**[0531.0.0.12] to [0533.0.0.12]** for the disclosure of the paragraphs [0531.0.0.12] to [0533.0.0.12] see paragraphs [0531.0.0.0] to [0533.0.0.0] above.

**[0534.0.12.12]** Example 14d: Engineering wheat plants by over-expressing YOR084W from Saccharomyces cerevisiae or other nucleic acids of the present invention.

**[0535.0.0.12] to [0537.0.0.12]** for the disclosure of the paragraphs [0535.0.0.12] to [0537.0.0.12] see paragraphs [0535.0.0.0] to [0537.0.0.0] above.

**[0538.0.12.12]** Example 14e: Engineering Rapeseed/Canola plants by over-expressing YOR084W from Saccharomyces cerevisiae or other nucleic acids of the present invention.

**[0539.0.0.12] to [0542.0.0.12]** for the disclosure of the paragraphs [0539.0.0.12] to [0542.0.0.12] see paragraphs [0539.0.0.0] to [0542.0.0.0] above.

**[0543.0.12.12]** Example 14f: Engineering alfalfa plants by over-expressing YOR084W from Saccharomyces cerevisiae or other nucleic acids of the present invention.

**[0544.0.0.12] to [0547.0.0.12]** for the disclosure of the paragraphs [0544.0.0.12] to [0547.0.0.12] see paragraphs [0544.0.0.0] to [0547.0.0.0] above.

**[0548.0.12.12]** Example 14g: Engineering alfalfa plants by over-expressing YOR084W from Saccharomyces cerevisiae or other nucleic acids of the present invention.

**[0549.0.0.12] to [0552.0.0.12]** for the disclosure of the paragraphs [0549.0.0.12] to [0552.0.0.12] see paragraphs [0549.0.0.0] to [0552.0.0.0] above.

**[0552.2.0.12]** for the disclosure of this paragraph see [0552.2.0.0] above.

**[0553.0.12.12]**
1. A process for the production of phytosterol, in particular, of beta-sitosterol and/or campesterol, which comprises
   (a) increasing or generating the activity of a YER156C, YKR057W, YOR084W, b0019, b0421, b2699, b0050, b0161 and/or b4129 protein and/or YER156C, YER173W, YKR057W, YOR044W, YPR172W, b2699, b3256, b0161, b0464, b1896, b2341, b2478 and/or b2822 protein in a non-human organism, or in one or more parts thereof; and
   (b) growing the organism under conditions which permit the production of phytosterol, in particular, of beta-sitosterol and/or campesterol in said organism.
2. A process for the production of phytosterol, in particular, of beta-sitosterol and/or campesterol comprising the increasing or generating in an organism or a part thereof the expression of at least one nucleic acid molecule comprising a nucleic acid molecule selected from the group consisting of:
   a) nucleic acid molecule encoding of the polypeptide as indicated in Table II, columns 5 or 7, lines 112 to 117 or lines 483 to 485 and/or lines 118 to 124 or lines 486 to 491 resp., or a fragment thereof, which confers an increase in the amount of phytosterol, in particular, of beta-sitosterol and/or campesterol in an organism or a part thereof;
   b) nucleic acid molecule comprising of the nucleic acid molecule as indicated in Table I, columns 5 or 7, lines 112 to 117 or lines 483 to 485 and/or lines 118 to 124 or lines 486 to 491, resp;
   c) nucleic acid molecule whose sequence can be deduced from a polypeptide sequence encoded by a nucleic acid molecule of (a) or (b) as a result of the degeneracy of the genetic code and conferring an increase in the amount of phytosterol, in particular, of beta-sitosterol and/or campesterol in an organism or a part thereof;
   d) nucleic acid molecule which encodes a polypeptide which has at least 50% identity with the amino acid sequence of the polypeptide encoded by the nucleic acid molecule of (a) to (c) and conferring an increase in the amount of phytosterol, in particular, of beta-sitosterol and/or campesterol in an organism or a part thereof;
   e) nucleic acid molecule which hybridizes with a nucleic acid molecule of (a) to (c) under under stringent hybridisation conditions and conferring an increase in the amount of phytosterol, in particular, of beta-sitosterol and/or campesterol in an organism or a part thereof;
   f) nucleic acid molecule which encompasses a nucleic acid molecule which is obtained by amplifying nucleic acid molecules from a cDNA library or a genomic library using the primers as shown in table III, column 7, lines 112 to 117 or lines 483 to 485 and/or lines 118 to 124 or lines 486 to 491 resp., and conferring an increase in the amount of phytosterol, in particular, of beta-sitosterol and/or campesterol in an organism or a part thereof;
   g) nucleic acid molecule encoding a polypeptide which is isolated with the aid of monoclonal antibodies against a polypeptide encoded by one of the nucleic acid molecules of (a) to (f) and conferring an increase in the amount of phytosterol, in particular, of beta-sitosterol and/or campesterol in an organism or a part thereof;
   h) nucleic acid molecule encoding a polypeptide comprising the consensus sequence shown in table IV, column 7, lines 112 to 117 or lines 483 to 485 and/or lines 118 to 124 or lines 486 to 491 resp., and conferring an increase in the amount of phytosterol, in particular, of beta-sitosterol and/or campesterol in an organism or a part thereof; and
   i) nucleic acid molecule which is obtainable by screening a suitable nucleic acid library under stringent hybridization conditions with a probe comprising one of the sequences of the nucleic acid molecule of (a) to (k) or with a fragment thereof having at least 15 nt, preferably 20 nt, 30 nt, 50 nt, 100 nt, 200 nt or 500 nt of the nucleic acid molecule characterized in (a) to (k) and conferring an increase in the amount of phytosterol, in particular, of beta-sitosterol and/or campesterol in an organism or a part thereof.
   or comprising a sequence which is complementary thereto.
3. The process of claim 1 or 2, comprising recovering of the free or bound phytosterol, in particular, of beta-sitosterol and/or campesterol.
4. The process of any one of claim 1 to 3, comprising the following steps:
   (a) selecting an organism or a part thereof expressing a polypeptide encoded by the nucleic acid molecule characterized in claim 2;
   (b) mutagenizing the selected organism or the part thereof;
   (c) comparing the activity or the expression level of said polypeptide in the mutagenized organism or the part thereof with the activity or the expression of said polypeptide of the selected organisms or the part thereof;
   (d) selecting the mutated organisms or parts thereof, which comprise an increased activity or expression level of said polypeptide compared to the selected organism or the part thereof;
   (e) optionally, growing and cultivating the organisms or the parts thereof; and
   (f) recovering, and optionally isolating, the free or bound phytosterol, in particular, of beta-sitosterol and/or campesterol produced by the selected mutated organisms or parts thereof.
5. The process of any one of claims 1 to 4, wherein the activity of said protein or the expression of said nucleic acid molecule is increased or generated transiently or stably.
6. An isolated nucleic acid molecule comprising a nucleic acid molecule selected from the group consisting of:
   a) nucleic acid molecule encoding of the polypeptide as indicated in Table II, column 5 or 7, lines 112 to 117 or lines 483 to 485 and/or lines 118 to 124 or lines 486 to 491 resp. or a fragment thereof, which confers an increase in the amount of phytosterol, in particular, of beta-sitosterol and/or campesterol in an organism or a part thereof;
   b) nucleic acid molecule comprising of the nucleic acid molecule shown in Table I, columns 5 or 7, lines 112 to 117 or lines 483 to 485 and/or lines 118 to 124 or lines 486 to 491, resp.;
   c) nucleic acid molecule whose sequence can be deduced from a polypeptide sequence encoded by a nucleic acid molecule of (a) or (b) as a result of the degeneracy of the genetic code and conferring an increase in the amount of phytosterol, in particular, of beta-sitosterol and/or campesterol in an organism or a part thereof;
   d) nucleic acid molecule which encodes a polypeptide which has at least 50% identity with the amino acid sequence of the polypeptide encoded by the nucleic acid molecule of (a) to (c) and conferring an increase in the amount of phytosterol, in particular, of beta-sitosterol and/or campesterol in an organism or a part thereof;
   e) nucleic acid molecule which hybridizes with a nucleic acid molecule of (a) to (c) under under stringent hybridisation conditions and conferring an increase in the amount of phytosterol, in particular, of beta-sitosterol and/or campesterol in an organism or a part thereof;
   f) nucleic acid molecule which encompasses a nucleic acid molecule which is obtained by amplifying nucleic acid molecules from a cDNA library or a genomic library using the primers as shown in table III, column 7, lines 112 to 117 or lines 483 to 485 and/or lines 118 to 124 or lines 486 to 491 resp., and conferring an increase in the amount of phytosterol, in particular, of beta-sitosterol and/or campesterol in an organism or a part thereof;
   g) nucleic acid molecule encoding a polypeptide which is isolated with the aid of monoclonal antibodies against a polypeptide encoded by one of the nucleic acid molecules of (a) to (f) and conferring an increase in the amount of phytosterol, in particular, of beta-sitosterol and/or campesterol in an organism or a part thereof;
   h) nucleic acid molecule encoding a polypeptide comprising the consensus sequence shown in table IV, column 7, lines 112 to 117 or lines 483 to 485 and/or lines 118 to 124 or lines 486 to 491 resp., and conferring an increase in the amount of phytosterol, in particular, of beta-sitosterol and/or campesterol in an organism or a part thereof; and
   i) nucleic acid molecule which is obtainable by screening a suitable nucleic acid library under stringent hybridization conditions with a probe comprising one of the sequences of the nucleic acid molecule of (a) to (k) or with a fragment thereof having at least 15 nt, preferably 20 nt, 30 nt, 50 nt, 100 nt, 200 nt or 500 nt of the nucleic acid molecule characterized in (a) to (k) and conferring an increase in the amount of phytosterol, in particular, of beta-sitosterol and/or campesterol in an organism or a part thereof, whereby the nucleic acid molecule distinguishes over the sequence as shown in Table IA, columns 5 or 7, lines 112 to 117 or lines 483 to 485 and/or lines 118 to 124 or lines 486 to 491, resp., by one or more nucleotides.
7. A nucleic acid construct which confers the expression of the nucleic acid molecule of claim 6, comprising one or more regulatory elements.
8. A vector comprising the nucleic acid molecule as claimed in claim 6 or the nucleic acid construct of claim 7.
9. he vector as claimed in claim 8, wherein the nucleic acid molecule is in operable linkage with regulatory sequences for the expression in a prokaryotic or eukaryotic, or in a prokaryotic and eukaryotic, host.
10. A host cell, which has been transformed stably or transiently with the vector as claimed in claim 8 or 9 or the nucleic acid molecule as claimed in claim 6 or the nucleic acid construct of claim 7 or produced as described in claim any one of claims 2 to 5.
11. The host cell of claim 10, which is a transgenic host cell.
12. The host cell of claim 10 or 11, which is a plant cell, an animal cell, a microorganism, or a yeast cell, a fungus cell, a prokaryotic cell, an eukaryotic cell or an archaebacterium.
13. A process for producing a polypeptide, wherein the polypeptide is expressed in a host cell as claimed in any one of claims 10 to 12.
14. A polypeptide produced by the process as claimed in claim 13 or encoded by the nucleic acid molecule as claimed in claim 6 whereby the polypeptide distinguishes over the sequence as indicated in Table IIA, columns 5 or 7, I A by one or more amino acids
15. An antibody, which binds specifically to the polypeptide as claimed in claim 14.
16. A plant tissue, propagation material, harvested material or a plant comprising the host cell as claimed in claim 12 which is plant cell or an Agrobacterium.
17. A method for screening for agonists and antagonists of the activity of a polypeptide encoded by the nucleic acid molecule of claim 6 conferring an increase in the amount of phytosterol, in particular, of beta-sitosterol and/or campesterol in an organism or a part thereof comprising:
   (a) contacting cells, tissues , plants or microorganisms which express the a polypeptide encoded by the nucleic acid molecule of claim 6 conferring an increase in the amount of phytosterol, in particular, of beta-sitosterol and/or campesterol in an organism or a part thereof with a candidate compound or a sample comprising a plurality of compounds under conditions which permit the expression the polypeptide;
   (b) assaying the phytosterol, in particular, of beta-sitosterol and/or campesterol level or the polypeptide expression level in the cell, tissue, plant or microorganism or the media the cell, tissue, plant or microorganisms is cultured or maintained in; and
   (c) identifying a agonist or antagonist by comparing the measured phytosterol, in particular, of beta-sitosterol and/or campesterol level or polypeptide expression level with a standard phytosterol, in particular, of beta-sitosterol and/or campesterol or polypeptide expression level measured in the absence of said candidate compound or a sample comprising said plurality of compounds, whereby an increased level over the standard indicates that the compound or the sample comprising said plurality of compounds is an agonist and a decreased level over the standard indicates that the compound or the sample comprising said plurality of compounds is an antagonist.
18. A process for the identification of a compound conferring increased phytosterol, in particular, of beta-sitosterol and/or campesterol production in a plant or microorganism, comprising the steps:
   (a) culturing a plant cell or tissue or microorganism or maintaining a plant expressing the polypeptide encoded by the nucleic acid molecule of claim 6 conferring an increase in the amount of phytosterol, in particular, of beta-sitosterol and/or campesterol in an organism or a part thereof and a readout system capable of interacting with the polypeptide under suitable conditions which permit the interaction of the polypeptide with dais readout system in the presence of a compound or a sample comprising a plurality of compounds and capable of providing a detectable signal in response to the binding of a compound to said polypeptide under conditions which permit the expression of said readout system and of the polypeptide encoded by the nucleic acid molecule of claim 6 conferring an increase in the amount of phytosterol, in particular, of beta-sitosterol and/or campesterol in an organism or a part thereof;
   (b) identifying if the compound is an effective agonist by detecting the presence or absence or increase of a signal produced by said readout system.
19. A method for the identification of a gene product conferring an increase in phytosterol, in particular, of beta-sitosterol and/or campesterol production in a cell, comprising the following steps:
   (a) contacting the nucleic acid molecules of a sample, which can contain a candidate gene encoding a gene product conferring an increase in phytosterol, in particular, of beta-sitosterol and/or campesterol after expression with the nucleic acid molecule of claim 6;
   (b) identifying the nucleic acid molecules, which hybridise under relaxed stringent conditions with the nucleic acid molecule of claim 6;
   (c) introducing the candidate nucleic acid molecules in host cells appropriate for producing phytosterol, in particular, of beta-sitosterol and/or campesterol;
   (d) expressing the identified nucleic acid molecules in the host cells;
   (e) assaying the phytosterol, in particular, of beta-sitosterol and/or campesterol level in the host cells; and
   (f) identifying nucleic acid molecule and its gene product which expression confers an increase in the phytosterol, in particular, of beta-sitosterol and/or campesterol level in the host cell in the host cell after expression compared to the wild type.
20. A method for the identification of a gene product conferring an increase in phytosterol, in particular, of beta-sitosterol and/or campesterol production in a cell, comprising the following steps:
   (a) identifiying in a data bank nucleic acid molecules of an organism; which can contain a candidate gene encoding a gene product conferring an increase in the phytosterol, in particular, of beta-sitosterol and/or campesterol amount or level in an organism or a part thereof after expression, and which are at least 20% homolog to the nucleic acid molecule of claim 6;
   (b) introducing the candidate nucleic acid molecules in host cells appropriate for producing phytosterol, in particular, of beta-sitosterol and/or campesterol;
   (c) expressing the identified nucleic acid molecules in the host cells;
   (d) assaying the phytosterol, in particular, of beta-sitosterol and/or campesterol level in the host cells; and
   (e) identifying nucleic acid molecule and its gene product which expression confers an increase in the phytosterol, in particular, of beta-sitosterol and/or campesterol level in the host cell after expression compared to the wild type.
21. A method for the production of an agricultural composition comprising the steps of the method of any one of claims 17 to 20 and formulating the compound identified in any one of claims 17 to 20 in a form acceptable for an application in agriculture.
22. A composition comprising the nucleic acid molecule of claim 6, the polypeptide of claim 14, the nucleic acid construct of claim 7, the vector of any one of claims 8 or 9, an antagonist or agonist identified according to claim 17, the compound of claim 18, the gene product of claim 19 or 20, the antibody of claim 15, and optionally an agricultural acceptable carrier.
23. Use of the nucleic acid molecule as claimed in claim 6 for the identification of a nucleic acid molecule conferring an increase of phytosterol, in particular, of beta-sitosterol and/or campesterol after expression.
24. Use of the polypeptide of claim 14 or the nucleic acid construct claim 7 or the gene product identified according to the method of claim 19 or 20 for identifying compounds capable of conferring a modulation of phytosterol, in particular, of beta-sitosterol and/or campesterol levels in an organism.
25. Food or feed composition comprising the nucleic acid molecule of claim 6, the polypeptide of claim 14, the nucleic acid construct of claim 7, the vector of claim 8 or 9, the antagonist or agonist identified according to claim 17, the antibody of claim 15, the plant or plant tissue of claim 16, the harvested material of claim 16, the host cell of claim 10 to 12 or the gene product identified according to the method of claim 19 or 20.
26. Use of the nucleic acid molecule of claim 6, the polypeptide of claim 14, the nucleic acid construct of claim 7, the vector of claim 8 or 9, the antagonist or agonist identified according to claim 17, the antibody of claim 15, the plant or plant tissue of claim 16, the host cell of claim 10 to 12 or the gene product identified according to the method of claim 19 or 20 for the protection of a plant against a beta-sitosterol and/or campesterol synthesis inhibiting herbicide.

**[0554.0.0.12] Abstract: see [0554.0.0.0]:**
Process for the production of fine chemicals

**[0000.0.0.14]** In a further embodiment, the present invention relates to a further process for the production of fine chemicals as defined below and corresponding embodiments as described herein as follows.

**[0001.0.0.14] to [0002.0.0.14]: see [0001.0.0.0] to [0002.0.0.0]**

**[0002.1.14.14]** L-alanine is used in various pharmaceutical and veterinary applications. For example, it is included, together with other amino acids, in preparations for infusion solutions or preparations for parenteral administration as clinical preoperative and postoperative foods, as well as an animal feed supplement. Furthermore, alanine is used as a food additive on account of its sweet taste. L-phenylalanine and L-aspartic acid have very important markets as key components in the manufacture of the sweetener aspartame. Aspartame (C₁₄H₁₈N₂O₅), L-aspartyl-L-phenylalanine methyl ester, is a compound of three components, which are methanol, aspartic acid and phenylalanine. L-aspartic acid is further used as a flavoring agent.
The amino acid L-citrulline is a metabolite in the urea cycle. Other amino acids in this cycle are L-arginine and L-ornithine.L-citrulline is involved in liver detoxification of ammonia, and has been shown to speed recover from fatigue. It has also been utilized in the treatment of Ornithine Transcarbamylase Deficiency and other Urea Cycle disorders. In cell metabolism, L-arginine and L-citrulline might serve as endogenous N sources (Ludwig et al., PLANT PHYSIOLOGY , Vol 101, Issue 2 429-434, 1993). Glycine is a valuable compound of wide use as food additives for processed foodstuffs and raw materials for agricultural chemicals and medicines. Glycine is the simplest amino acid, and is used in crop production as a chelating agent for micronutrients and has been used as a nitrogen fertilizer, at least on an experimental basis. As such, it is representative of amino acids used in crop production. Practically all commercial glycine is produced by synthetic processes such as the Strecker Synthesis, the reaction of formaldehyde, ammonia, and hydrogen cyanide, and hydrolysis of the resulting aminonitrile. Glycine is used as chelating / complexing agent for cation nutrients, plant growth regulators, substrate for microbiological products, fertilizer source of nitrogen. Serine is a primary intermediate in the biosynthesis of a wide variety of cellular metabolites including such economically important compounds as choline, glycine, cysteine and tryptophan. In addition, serine acts as a single carbon donor and is responsible for 60% to 75% of the total need of the cell for C1 units through the production of 5,10-methylenetetrahydrofolate from tetrahydrofolate. These C1 units are used in a wide variety of biosynthetic pathways including the synthesis of methionine, inosine monophosphate, other purines and some pyrimidines (e.g., thymidine and hydroxymethyl cytidine). The glycine-serine interconversion, catalysed by glycine decarboxylase and serine hydroxymethyltransferase, is an important reaction of primary metabolism in all organisms including plants, by providing one-carbon units for many biosynthetic reactions. In plants, in addition, it is an integral part of the photorespiratory metabolic pathway and produces large amounts of photorespiratory CO₂ within mitochondria (Bauwe et al., Journal of Experimental Botany, Vol. 54, No. 387, pp. 1523-1535, June 1, 2003.)
The enzymatic conversion of phenylalanine to tyrosine is known in eukaryotes. Human phenylalanine hydroxylase is specifically expressed in the liver to convert L-phenylalanine to L-tyrosine (Wang et al. J. Biol. Chem. 269 (12): 9137-46 (1994)). Deficiency of the PAH enzyme causes classic phenylketonurea, a common genetic disorder.
Tyrosine and homoserine and their derivatives are also used in organic synthesis. For example, tyrosine is starting material in the synthesis of chatecolamines or DOPA (dihydroxy-phenyl-alanine) as well as a precursor of adrenaline, dopamine and norepinepherine. A variety of beta-amino-gamma-keto acids can be prepared from commercially available I-homoserine. 5-Oxoproline, also named as pyroglutamic acid PCA and slats like sodium-PCA, is used as cosmetic ingredient, such as hair and skin conditioning agent. One optical isomer of PCA (the L form) is a naturally occurring component of mammalian tissue. 5-Oxoproline is further used as templates in the synthesis of homochiral glutamate antagonists.

**[0003.0.0.14] to [0008.0.0.14]: see [0003.0.0.0] to [0008.0.0.0]**

**[0008.1.14.14]**US 5,498,532 disclose the production of various L-amino acids like glutamic acid, glutamine, lysine, threonine, isoleucine, valine, leucine, tryptophan, phenylalanine, tyrosine, histidine, arginine, ornithine, citrulline and proline by direct fermentation using, coryneform bacteria belonging to the genus Corynbacterium or Brevibacterium, which are inherently unable to assimilate lactose, but due to recombinant DNA technology able to assimilate lactose,which represent the carbon source.
An other method for producing amino acids such as homoserine is disclosed in US 20010049126, which use a bacterium belonging to the genus Escherichia which harbors a PTS, phosphotransferase system, gene.
The coproduction of glutamic acid and other amino acids including lysine, aspartic acid, alanine by an auxotroph of Bacillus methanolicus is described in US 6,110,713. According to the teaching of US 5,677,156 L-aspartic acid can be efficiently produced from maleic acid or fumaric acid by adding the aspartase-containing microorganism, like Brevibacterium flavum AB-41 strain (FERM BP-1498) and Eschirichia coli ATCC 11303.
US 5,354,672 discloses a method of producing tyrosine, methionine, or phenylalanine by transiently incorporating a DNA inversion gene into the host cell, Escherichia coli cells, which induce hypersecretion of amino acids. Known is also the production of citrulline in the small intestine as a product of glutamine metabolism, or in the arginine biosynthetic pathway, where ornithine carbamoyltransferases catalyse the production of citrulline from carbamoyl-phosphate and ornithine. Benninghoff et al. disclose the production of citrulline and ornithine by interferon-gamma treated macrophages (International Immunology, Vol 3, 413-417, 1991).
There disclosed is a method for producing glycine in US 20030040085, which comprises subjecting an aqueous solution of glycinonitrile to a hydrolysis reaction in a hydrolysis reaction system under the action of a microbial enzyme, thereby converting the glycinonitrile to glycine while by-producing ammonia. US 20040157290 discloses a process for preparing a serine-rich foreign protein comprising culturing a bacterium containing the cysteine synthase (cysK) gene and a gene encoding the foreign protein. US 20030079255 disclose the production of Para-hydroxycinnamic acid by introducing genes encoding phenylalanine ammonia-lyase from C. violaceum or R. glutinis tyrosine into a host microorganism and as intermediates, tyrosine and cinnamic acid are also produced.
Production of single cell protein and selected amino acids by microbial fermentation is known, e.g., US 4,652,527. One amino acid which has been produced on an industrial scale is lysine, see Tosaka et al., Trends in Biotechnology, 1: 70-74 (1983), Tosaka and Takinami, Progress in Industrial Microbiology, Ch. 24, pp. 152-172 (Aida et al., 1986). Another example is glutamic acid which has been produced using bacteria of the genera Corynebacterium, Brevibacterium, Microbacterium, and Arothrobacter by fermentation on molasses and starch hydrozylates. Aspartic acid and alanine are produced by enzymatic means from fumaric acid and ammonia. Bacillus species have been used in fermentation processes to produce amino acids, Tosaka et al.; Tosaka and Takinami, as named above.

**[0009.0.14.14]**As described above, the amino acids are necessary for humans and many mammals, for example for livestock or other applications in the health care area. L-aspartic acid is one of the amino acids that is difficult to produce directly by fermentation. Consequently today the enzyme catalyzed addition of ammonia to fumaric acid is employed commercially in the production of aspartic acid. As chelating agents, amino acids increase the biological availability of different metals. Chelating agents in general can enhance nutrient uptake, but may also increase the uptake of toxic metals if those are also present. If cation impurities are present in micronutrient sources (e.g. Cadmium), chelation of those metals would make those contaminants more readily assimilated by plants than in less available forms.

**[0010.0.0.14] to [0011.0.0.14]: see [0010.0.0.0] to [0011.0.0.0]**

**[0012.0.14.14]**It is an object of the present invention to develop an inexpensive process for the synthesis of 5-oxoproline, alanine, aspartic acid, citrulline, glycine, homoserine, phenylalanine, serine and/or tyrosine. Amino acids are (depending on the organism) one of the most frequently limiting components of food or feed .

**[0013.0.0.14] see [0013.0.0.00]**

**[0014.0.14.14]**Accordingly, in a first embodiment, the invention relates to a process for the production of a fine chemical, whereby the fine chemical is 5-oxoproline, alanine, aspartic acid, citrulline, glycine, homoserine, phenylalanine, serine and/or tyrosine. Accordingly, in the present invention, the term "the fine chemical" as used herein relates to "5-oxoproline, alanine, aspartic acid, citrulline, glycine, homoserine, phenylalanine, serine and/or tyrosine". Further, the term "the fine chemicals" as used herein also relates to fine chemicals comprising 5-oxoproline, alanine, aspartic acid, citrulline, glycine, homoserine, phenylalanine, serine and/or tyrosine.

**[0015.0.14.14]**In one embodiment, the term "the fine chemical" or "the respective fine chemical" means 5-oxoproline, alanine, aspartic acid, citrulline, glycine, homoserine, phenylalanine, serine and/or tyrosine, preferably the amino acid of the present invention in the L configuration, meaning L-5-oxoproline, L-alanine, L-aspartic acid, L-citrulline, L-glycine, L-homoserine, L-phenylalanine, L-serine and/or L-tyrosine . Throughout the specification the term "the fine chemical" means 5-oxoproline, alanine, aspartic acid, citrulline, glycine, homoserine, phenylalanine, serine and/or tyrosine, preferably the amino acid of the present invention in the L configuration, its salts, ester or amids in free form or bound to proteins. In a preferred embodiment, the term "the fine chemical" means L-5-oxoproline, L-alanine, L-aspartic acid, L-citrulline, L-glycine, L-homoserine, L-phenylalanine, L-serine and/or L-tyrosine in free form or its salts or bound to proteins.

**[0016.0.14.14]**Accordingly, the present invention relates to a process for the production of the fine respective chemical comprising
(a) increasing or generating the activity of one or more
   YDL127W, b1896, b0161, b0376, b0970, b1343 and/or b3172 - protein(s) or of a protein having the sequence of a polypeptide encoded by a nucleic acid molecule indicated in Table II, columns 5 or 7, lines 126 to 127 and/or 492 to 496;
   in a non-human organism in one or more parts thereof and
(b) growing the organism under conditions which permit the production of the fine chemical, meaning of 5-oxoproline or fine chemicals comprising 5-oxoproline in said organism;
or
(a) increasing or generating the activity of one or more
   YIL150C, YFL050C, YER173W,YBL015W, b3008, b2095,b0236, b0486, b1343, b1863, b2489, b2576, b3231 and/or b3767 - protein(s) or of a protein having the sequence of a polypeptide encoded by a nucleic acid molecule indicated in Table II, columns 5 or 7, lines 128 to 133 and/or 497 to 504;
   in a non-human organism in one or more parts thereof and
(b) growing the organism under conditions which permit the production of the fine chemical, meaning of alanine or fine chemicals comprising alanine in said organism;
or
(a) increasing or generating the activity of one or more
   YIL150C, YGR104C, YER173W, b1896, b0730, b0161, b0577, b1343, b2023, b2507, b2576, b2753, b3116, b3169, b3172, b4129 and/or b4346 - protein(s) or of a protein having the sequence of a polypeptide encoded by a nucleic acid molecule indicated in Table II, columns 5 or 7, lines 134 to 138 and/or 505 to 516;
   in a non-human organism in one or more parts thereof and
(b) growing the organism under conditions which permit the production of the fine chemical, meaning of aspartic acid or fine chemicals comprising aspartic acid in said organism;
or
(a) increasing or generating the activity of one or more
   YOR245C, YLL013C, b0057, b0462, b1275, b1360, b2239, b2414, b2426, b2489, b3160, b3241, b3926, b4214 and/or b4269 - protein(s) or of a protein having the sequence of a polypeptide encoded by a nucleic acid molecule indicated in Table II, columns 5 or 7, lines 139 to 140 and/or 517 to 529;
   in a non-human organism in one or more parts thereof and
(b) growing the organism under conditions which permit the production of the fine chemical, meaning of citrulline or fine chemicals comprising citrulline in said organism;
or
(a) increasing or generating the activity of one or more
   YOL123W, YFL050C, b0057, b0577, b2414, b2489, b2553 and/or b2576 - protein(s) or of a protein having the sequence of a polypeptide encoded by a nucleic acid molecule indicated in Table II, columns 5 or 7, lines 141 to 142 and/or 530 to 535; in a non-human organism in one or more parts thereof and
(b) growing the organism under conditions which permit the production of the fine chemical, meaning of glycine or fine chemicals comprising glycine in said organism;
or
(a) increasing or generating the activity of one or more
   YEL046C and/or b3767 - protein(s) or of a protein having the sequence of a polypeptide encoded by a nucleic acid molecule indicated in Table II, columns 5 or 7, lines 143 and/or 536;
   in a non-human organism in one or more parts thereof and
(b) growing the organism under conditions which permit the production of the fine chemical, meaning of homoserine or fine chemicals comprising homoserine in said organism;
or
(a) increasing or generating the activity of one or more
   YPR138C, YJL072C, YHR130C, YGR101W, YER173W, YAL049C, b3462, b3256, b1886, b1827, b1708, b1697, b0695, b0161, b1228, b2078, b2414, b2576, b2796, b3919, b3938 and/or b3983 - protein(s) or of a protein having the sequence of a polypeptide encoded by a nucleic acid molecule indicated in Table II, columns 5 or 7, lines 144 to 156 and/or 537 to 545;
   in a non-human organism in one or more parts thereof and
(b) growing the organism under conditions which permit the production of the fine chemical, meaning of phenylalanine or fine chemicals comprising phenylalanine in said organism;
or
(a) increasing or generating the activity of one or more
   YOR261C, YLR082C, YLL009C, YKR057W, YIL150C, YER152C, YEL045C, b3462, b1886, b1829, b0057, b0486, b2414, b2489, b2664, b3064, b3116, b3160 and/or b3231 - protein(s) or of a protein having the sequence of a polypeptide encoded by a nucleic acid molecule indicated in Table II, columns 5 or 7, lines 157 to 166 and/or 546 to 554;
   in a non-human organism in one or more parts thereof and
(b) growing the organism under conditions which permit the production of the fine chemical, meaning of serine or fine chemicals comprising serine in said organism;
or
(a) increasing or generating the activity of one or more
   YOR350C, YIL150C, YHR130C, YFL050C, b1829, b1827, b0970, b2491 and/or b3983 - protein(s) or of a protein having the sequence of a polypeptide encoded by a nucleic acid molecule indicated in Table II, columns 5 or 7, lines 167 to 172 and/or 555 to 557;
   in a non-human organism in one or more parts thereof and
(b) growing the organism under conditions which permit the production of the fine chemical, meaning of tyrosine or fine chemicals comprising tyrosine in said organism.
Accordingly, the present invention relates to a process for the production of a fine chemical comprising
(a) increasing or generating the activity of one or more proteins having the activity of a protein indicated in Table II, column 3, lines 126 to 172 and/or lines 492 to 557 or having the sequence of a polypeptide encoded by a nucleic acid molecule indicated in Table I, column 5 or 7, lines 126 to 172 and/or lines 492 to 557, in a non-human organism in one or more parts thereof and
(b) growing the organism under conditions which permit the production of the fine chemical, in particular 5-oxoproline and/or alanine and/or aspartic acid and/or citrulline and/or glycine and/or homoserine and/or phenylalanine and/or serine and/or tyrosine.

**[0016.1.14.14]**Accordingly, the term "the fine chemical" means in one embodiment "5-oxoproline" in relation to all sequences listed in Table I to IV, lines 126 to 127 and/or 492 to 496 or homologs thereof and
means in one embodiment "alanine" in relation to all sequences listed in Tables I to IV, lines 128 to 133 and/or 497 to 504 or homologs thereof and
means in one embodiment "aspartic acid" in relation to all sequences listed in Table I to IV, lines 134 to 138 and/or 505 to 516, or homologs thereof and
means in one embodiment "citrulline" in relation to all sequences listed in Table I to IV, lines 139 to 140 and/or 517 to 529 or homologs thereof and
means in one embodiment "glycine" in relation to all sequences listed in Table I to IV, lines 141 to 142 and/or 530 to 535 or homologs thereof and
means in one embodiment "homoserine" in relation to all sequences listed in Table I to IV, lines 143 and/or 536 or homologs thereof and
means in one embodiment "phenylalanine" in relation to all sequences listed in Table I to IV, lines 144 to 156 and/or 537 to 545 or homologs thereof and
means in one embodiment "serine" in relation to all sequences listed in Table I to IV, lines 157 to 166 and/or 546 to 554 or homologs thereof and
means in one embodiment "tyrosine" in relation to all sequences listed in Table I to IV, lines 167 to 172 and/or 555 to 557.or homologs thereof.
Accordingly, in one embodiment the term "the fine chemical" means "5-oxoproline" and "aspartic acid" in relation to all sequences listed in Table I to IV, lines 127 and/or 137;
in one embodiment the term "the fine chemical" means "alanine", "aspartic acid", "serine" and "tyrosine" in relation to all sequences listed in Table I to IV, lines 128, 134, 161 and/or 168;
in one embodiment the term "the fine chemical" means "alanine", "glycine" and "tyrosine" in relation to all sequences listed in Table I to IV, lines 129, 142 and/or 170;
in one embodiment the term "the fine chemical" means "phenylalanine" and "tyrosine" in relation to all sequences listed in Table I to IV, lines 146 and/or 169;
in one embodiment the term "the fine chemical" means "phenylalanine" and "tyrosine" in relation to all sequences listed in Table I to IV, lines 153 and/or 172;
in one embodiment the term "the fine chemical" means "serine" and "tyrosine" in relation to all sequences listed in Table I to IV, lines 166 and/or 171;
in one embodiment the term "the fine chemical" means "phenylalanine" and "serine" in relation to all sequences listed in Table I to IV, lines 150 and/or 164;
in one embodiment the term "the fine chemical" means "phenylalanine" and "serine" in relation to all sequences listed in Table I to IV, lines 152 and/or 165;
in one embodiment the term "the fine chemical" means "citrulline", "glycine" and "serine" in relation to all sequences listed in Table I to IV, lines 517, 530 and/or 546;
in one embodiment the term "the fine chemical" means "5-oxoproline", "aspartic acid" and "phenylalanine" in relation to all sequences listed in Table I to IV, lines 492, 505 and/or 537;
in one embodiment the term "the fine chemical" means "alanine" and "serine" in relation to all sequences listed in Table I to IV, lines 498 and/or 547;
in one embodiment the term "the fine chemical" means "glycine" and "aspartic acid" in relation to all sequences listed in Table I to IV, lines 531 and/or 506;
in one embodiment the term "the fine chemical" means "5-oxoproline" and "tyrosine" in relation to all sequences listed in Table I to IV, lines 494 and/or 555;
in one embodiment the term "the fine chemical" means "alanine", "5-oxoproline" and "aspartic acid" in relation to all sequences listed in Table I to IV, lines 499, 495 and/or 507;
in one embodiment the term "the fine chemical" means "citrulline", "glycine", "serine" and "phenylalanine" in relation to all sequences listed in Table I to IV, lines 522, 532, 548 and/or 540;
in one embodiment the term "the fine chemical" means "citrulline", "alanine", "glycine" and "serine" in relation to all sequences listed in Table I to IV, lines 524, 501, 533 and/or 549;
in one embodiment the term "the fine chemical" means "alanine", "glycine", "aspartic acid" and "phenylalanine" in relation to all sequences listed in Table I to IV, lines 502, 535, 510 and/or 541;
in one embodiment the term "the fine chemical" means "serine" and "aspartic acid" in relation to all sequences listed in Table I to IV, lines 552 and/or 512;
in one embodiment the term "the fine chemical" means "citrulline" and "serine" in relation to all sequences listed in Table I to IV, lines 525 and/or 553;
in one embodiment the term "the fine chemical" means "5-oxoproline" and "aspartic acid" in relation to all sequences listed in Table I to IV, lines 496 and/or 514;
in one embodiment the term "the fine chemical" means "alanine" and "serine" in relation to all sequences listed in Table I to IV, lines 503 and/or 554;
in one embodiment the term "the fine chemical" means "alanine" and "homoserine" in relation to all sequences listed in Table I to IV, lines 504 and/or 536;
in one embodiment the term "the fine chemical" means "phenylalanine" and "tyrosine" in relation to all sequences listed in Table I to IV, lines 545 and/or 557;
in one embodiment the term "the fine chemical" means "alanine", "aspartic acid" and "phenylalanine" in relation to all sequences listed in Table I to IV, lines 130, 136 and/or 148;
Accordingly, the term "the fine chemical" can mean "5-oxoproline", "alanine", "aspartic acid", "citrulline", "glycine", "homoserine", "phenylalanine", "serine" and/or "tyrosine" , owing to circumstances and the context. In order to illustrate that the meaning of the term "the fine chemical" means "5-oxoproline", "alanine", "aspartic acid", "citrulline", "glycine", "homoserine", "phenylalanine", "serine" and/or "tyrosine" the term "the respective fine chemical" is also used.

**[0017.0.0.14] to [0018.0.0.14]: see [0017.0.0.0] to [0018.0.0.0]**

**[0019.0.14.14]**Advantageously the process for the production of the respective fine chemical leads to an enhanced production of the fine respective chemical. The terms "enhanced" or "increase" mean at least a 10%, 20%, 30%, 40% or 50%, preferably at least 60%, 70%, 80%, 90% or 100%, more preferably 150%, 200%, 300%, 400% or 500% higher production of the respective fine chemical in comparison to the reference as defined below, e.g. that means in comparison to an organism without the aforementioned modification of the activity of a protein having the activity of a protein indicated in Table II, column 3, lines 126 to 172 and/or lines 492 to 557or encoded by nucleic acid molecule indicated in Table I, columns 5 or 7, lines 126 to 172 and/or lines 492 to 557.

**[0020.0.14.14]**Surprisingly it was found, that the transgenic expression of at least one of the Saccaromyces cerevisiae protein(s) indicated in Table II, Column 3,
line 126 for 5-oxoproline
and/or lines 128 to 131 for alanine
and/or lines 134 to 136 for aspartic acid
and/or lines 139 to 140 for citrulline
and/or lines 141 to 142 for glycine
and/or line 143 for homoserine
and/or lines 144 to 149 for phenylalanine
and/or lines 157 to 163 for serine
and/or lines 167 to 170 for tyrosine
in Arabidopsis thaliana conferred an increase in the respective fine chemical content of the transformed plants
and/or
at least one of the Escherichia coli K12 proteins indicated in Table II, Column 3, line 127 and/or 492 to 496 for 5-oxoproline
and/or lines 132 to 133 and/or 497 to 504 for alanine
and/or lines 137 to 138 and/or 505 to 516 for aspartic acid
and/or lines 517 to 529 for citrulline
and/or lines 530 to 535 for glycine
and/or line 536 for homoserine
and/or lines 150 to 156 and/or 537 to 545 for phenylalanine
and/or lines 164 to 166 and/or 546 to 554 for serine
and/or lines 171 to 172 and/or 555 to 557 for tyrosine
in Arabidopsis thaliana conferred an increase in the respective fine chemical content of the transformed plants.
Surprisingly it was found, that the transgenic expression of the Escherichia coli K12 protein as indicated in Table II, column 5, lines 127 and 137 in Arabidopsis thaliana conferred an increase in 5-oxoproline and/or aspartic acid (or the respective fine chemical) content of the transformed plants. Thus, in one embodiment, said protein or its homologs are used for the production of 5-oxoproline, in one embodiment, said protein or its homologs are used for the production of aspartic acid; in one embodiment, said protein or its homologs are used for the production of one or more fine chemical selected from the group consisting of: 5-oxoproline and/or aspartic acid.
Surprisingly it was found, that the transgenic expression of the Saccharomyces cerevisiae protein as indicated in Table II, column 5, lines 128 and 134 and 161 and 168 in Arabidopsis thaliana conferred an increase in alanine and/or aspartic acid and/or serine and/or tyrosine (or the respective fine chemical) content of the transformed plants. Thus, in one embodiment, said protein or its homologs are used for the production of alanine; in one embodiment, said protein or its homologs are used for the production of aspartic acid, in one embodiment, said protein or its homologs are used for the production of serine, in one embodiment, said protein or its homologs are used for the production of tyrosine, in one embodiment, said protein or its homologs are used for the production of one or more fine chemical selected from the group consisting of: alanine and/or aspartic acid and/or serine and/or tyrosine.
Surprisingly it was found, that the transgenic expression of the Saccharomyces cerevisiae protein as indicated in Table II, column 5, lines 129 and 142 and 170 in Arabidopsis thaliana conferred an increase in alanine and/or glycine and/or tyrosine (or the respective fine chemical) content of the transformed plants. Thus, in one embodiment, said protein or its homologs are used for the production of alanine; in one embodiment, said protein or its homologs are used for the production of glycine, in one embodiment, said protein or its homologs are used for the production of tyrosine, in one embodiment, said protein or its homologs are used for the production of one or more fine chemical selected from the group consisting of: alanine and/or glycine and/or tyrosine.
Surprisingly it was found, that the transgenic expression of the Saccharomyces cerevisiae protein as indicated in Table II, column 5, lines 146 and 169 in Arabidopsis thaliana conferred an increase in phenylalanine and/or tyrosine (or the respective fine chemical) content of the transformed plants. Thus, in one embodiment, said protein or its homologs are used for the production of phenylalanine; in one embodiment, said protein or its homologs are used for the production of tyrosine, in one embodiment, said protein or its homologs are used for the production of one or more fine chemical selected from the group consisting of: phenylalanine and/or tyrosine.
Surprisingly it was found, that the transgenic expression of the Escherichia coli K12 protein as indicated in Table II, column 5, lines 153 and 172 in Arabidopsis thaliana conferred an increase in phenylalanine and/or tyrosine (or the respective fine chemical) content of the transformed plants. Thus, in one embodiment, said protein or its homologs are used for the production of phenylalanine, in one embodiment, said protein or its homologs are used for the production of tyrosine; in one embodiment, said protein or its homologs are used for the production of one or more fine chemical selected from the group consisting of: phenylalanine and/or tyrosine.
Surprisingly it was found, that the transgenic expression of the Escherichia coli K12 protein as indicated in Table II, column 5, lines 166 and 171 in Arabidopsis thaliana conferred an increase in serine and/or tyrosine (or the respective fine chemical) content of the transformed plants. Thus, in one embodiment, said protein or its homologs are used for the production of serine, in one embodiment, said protein or its homologs are used for the production of tyrosine; in one embodiment, said protein or its homologs are used for the production of one or more fine chemical selected from the group consisting of: serine and/or tyrosine.
Surprisingly it was found, that the transgenic expression of the Escherichia coli K12 protein as indicated in Table II, column 5, lines 150 and 164 in Arabidopsis thaliana conferred an increase in phenylalanine and/or serine (or the respective fine chemical) content of the transformed plants. Thus, in one embodiment, said protein or its homologs are used for the production of phenylalanine, in one embodiment, said protein or its homologs are used for the production of serine; in one embodiment, said protein or its homologs are used for the production of one or more fine chemical selected from the group consisting of: phenylalanine and/or serine.
Surprisingly it was found, that the transgenic expression of the Escherichia coli K12 protein as indicated in Table II, column 5, lines 152 and 165 in Arabidopsis thaliana conferred an increase in phenylalanine and/or serine (or the respective fine chemical) content of the transformed plants. Thus, in one embodiment, said protein or its homologs are used for the production of phenylalanine, in one embodiment, said protein or its homologs are used for the production of serine; in one embodiment, said protein or its homologs are used for the production of one or more fine chemical selected from the group consisting of: phenylalanine and/or serine.
Surprisingly it was found, that the transgenic expression of the Escherichia coli K12 protein as indicated in Table II, column 5, lines 517 and 530 and 546 in Arabidopsis thaliana conferred an increase in citrulline and/or glycine and/or serine (or the respective fine chemical) content of the transformed plants. Thus, in one embodiment, said protein or its homologs are used for the production of citrulline; in one embodiment, said protein or its homologs are used for the production of glycine, in one embodiment, said protein or its homologs are used for the production of serine, in one embodiment, said protein or its homologs are used for the production of one or more fine chemical selected from the group consisting of: citrulline and/or glycine and/or serine.
Surprisingly it was found, that the transgenic expression of the Escherichia coli K12 protein as indicated in Table II, column 5, lines 492 and 505 and 537 in Arabidopsis thaliana conferred an increase in 5-oxoproline and/or aspartic acid and/or phenylalanine (or the respective fine chemical) content of the transformed plants. Thus, in one embodiment, said protein or its homologs are used for the production of 5-oxoproline; in one embodiment, said protein or its homologs are used for the production of aspartic acid, in one embodiment, said protein or its homologs are used for the production of phenylalanine, in one embodiment, said protein or its homologs are used for the production of one or more fine chemical selected from the group consisting of: 5-oxoproline and/or aspartic acid and/or phenylalanine.
Surprisingly it was found, that the transgenic expression of the Escherichia coli K12 protein as indicated in Table II, column 5, lines 498 and 547 in Arabidopsis thaliana conferred an increase in alanine and/or serine (or the respective fine chemical) content of the transformed plants. Thus, in one embodiment, said protein or its homologs are used for the production of alanine; in one embodiment, said protein or its homologs are used for the production of serine, in one embodiment, said protein or its homologs are used for the production of one or more fine chemical selected from the group consisting of: alanine and/or serine.
Surprisingly it was found, that the transgenic expression of the Escherichia coli K12 protein as indicated in Table II, column 5, lines 531 and 506 in Arabidopsis thaliana conferred an increase in glycine and/or aspartic acid (or the respective fine chemical) content of the transformed plants. Thus, in one embodiment, said protein or its homologs are used for the production of glycine; in one embodiment, said protein or its homologs are used for the production of aspartic acid, in one embodiment, said protein or its homologs are used for the production of one or more fine chemical selected from the group consisting of: glycine and/or aspartic acid.
Surprisingly it was found, that the transgenic expression of the Escherichia coli K12 protein as indicated in Table II, column 5, lines 494 and 555 in Arabidopsis thaliana conferred an increase in 5-oxoproline and/or tyrosine (or the respective fine chemical) content of the transformed plants. Thus, in one embodiment, said protein or its homologs are used for the production of 5-oxoproline; in one embodiment, said protein or its homologs are used for the production of tyrosine, in one embodiment, said protein or its homologs are used for the production of one or more fine chemical selected from the group consisting of: 5-oxoproline and/or tyrosine.
Surprisingly it was found, that the transgenic expression of the Escherichia coli K12 protein as indicated in Table II, column 5, lines 499 and 495 and 507 in Arabidopsis thaliana conferred an increase in alanine and/or 5-oxoproline and/or aspartic acid (or the respective fine chemical) content of the transformed plants. Thus, in one embodiment, said protein or its homologs are used for the production of alanine; in one embodiment, said protein or its homologs are used for the production of 5-oxoproline, in one embodiment, said protein or its homologs are used for the production of aspartic acid, in one embodiment, said protein or its homologs are used for the production of one or more fine chemical selected from the group consisting of: alanine and/or 5-oxoproline and/or aspartic acid.
Surprisingly it was found, that the transgenic expression of the Escherichia coli K12 protein as indicated in Table II, column 5, lines 522 and 532 and 548 and 540 in Arabidopsis thaliana conferred an increase in citrulline and/or glycine and/or serine and/or phenylalanine (or the respective fine chemical) content of the transformed plants. Thus, in one embodiment, said protein or its homologs are used for the production of citrulline; in one embodiment, said protein or its homologs are used for the production of glycine, in one embodiment, said protein or its homologs are used for the production of serine, in one embodiment, said protein or its homologs are used for the production of phenylalanine, in one embodiment, said protein or its homologs are used for the production of one or more fine chemical selected from the group consisting of: citrulline and/or glycine and/or serine and/or phenylalanine.
Surprisingly it was found, that the transgenic expression of the Escherichia coli K12 protein as indicated in Table II, column 5, lines 524 and 501 and 533 and 549 in Arabidopsis thaliana conferred an increase in citrulline and/or alanine and/or glycine and/or serine (or the respective fine chemical) content of the transformed plants. Thus, in one embodiment, said protein or its homologs are used for the production of citrulline; in one embodiment, said protein or its homologs are used for the production of alanine, in one embodiment, said protein or its homologs are used for the production of glycine, in one embodiment, said protein or its homologs are used for the production of serine, in one embodiment, said protein or its homologs are used for the production of one or more fine chemical selected from the group consisting of: citrulline and/or alanine and/or glycine and/or serine.
Surprisingly it was found, that the transgenic expression of the Escherichia coli K12 protein as indicated in Table II, column 5, lines 502 and 535 and 510 and 541 in Arabidopsis thaliana conferred an increase in alanine and/or glycine and/or aspartic acid and/or phenylalanine (or the respective fine chemical) content of the transformed plants. Thus, in one embodiment, said protein or its homologs are used for the production of alanine; in one embodiment, said protein or its homologs are used for the production of glycine, in one embodiment, said protein or its homologs are used for the production of aspartic acid, in one embodiment, said protein or its homologs are used for the production of phenylalanine, in one embodiment, said protein or its homologs are used for the production of one or more fine chemical selected from the group consisting of: alanine and/or glycine and/or aspartic acid and/or phenylalanine.
Surprisingly it was found, that the transgenic expression of the Escherichia coli K12 protein as indicated in Table II, column 5, lines 552 and 512 in Arabidopsis thaliana conferred an increase in serine and/or aspartic acid (or the respective fine chemical) content of the transformed plants. Thus, in one embodiment, said protein or its homologs are used for the production of serine; in one embodiment, said protein or its homologs are used for the production of aspartic acid, in one embodiment, said protein or its homologs are used for the production of one or more fine chemical selected from the group consisting of: serine and/or aspartic acid.
Surprisingly it was found, that the transgenic expression of the Escherichia coli K12 protein as indicated in Table II, column 5, lines 525 and 553 in Arabidopsis thaliana conferred an increase in citrulline and/or serine (or the respective fine chemical) content of the transformed plants. Thus, in one embodiment, said protein or its homologs are used for the production of citrulline; in one embodiment, said protein or its homologs are used for the production of serine, in one embodiment, said protein or its homologs are used for the production of one or more fine chemical selected from the group consisting of: citrulline and/or serine.
Surprisingly it was found, that the transgenic expression of the Escherichia coli K12 protein as indicated in Table II, column 5, lines 496 and 514 in Arabidopsis thaliana conferred an increase in 5-oxoproline and/or aspartic acid (or the respective fine chemical) content of the transformed plants. Thus, in one embodiment, said protein or its homologs are used for the production of 5-oxoproline; in one embodiment, said protein or its homologs are used for the production of aspartic acid, in one embodiment, said protein or its homologs are used for the production of one or more fine chemical selected from the group consisting of: 5-oxoproline and/or aspartic acid.
Surprisingly it was found, that the transgenic expression of the Escherichia coli K12 protein as indicated in Table II, column 5, lines 503 and 554 in Arabidopsis thaliana conferred an increase in alanine and/or serine (or the respective fine chemical) content of the transformed plants. Thus, in one embodiment, said protein or its homologs are used for the production of alanine; in one embodiment, said protein or its homologs are used for the production of serine, in one embodiment, said protein or its homologs are used for the production of one or more fine chemical selected from the group consisting of: alanine and/or serine.
Surprisingly it was found, that the transgenic expression of the Escherichia coli K12 protein as indicated in Table II, column 5, lines 504 and 536 in Arabidopsis thaliana conferred an increase in alanine and/or homoserine (or the respective fine chemical) content of the transformed plants. Thus, in one embodiment, said protein or its homologs are used for the production of alanine; in one embodiment, said protein or its homologs are used for the production of homoserine, in one embodiment, said protein or its homologs are used for the production of one or more fine chemical selected from the group consisting of: alanine and/or homoserine.
Surprisingly it was found, that the transgenic expression of the Escherichia coli K12 protein as indicated in Table II, column 5, lines 545 and 557 in Arabidopsis thaliana conferred an increase in phenylalanine and/or tyrosine (or the respective fine chemical) content of the transformed plants. Thus, in one embodiment, said protein or its homologs are used for the production of phenylalanine; in one embodiment, said protein or its homologs are used for the production of tyrosine, in one embodiment, said protein or its homologs are used for the production of one or more fine chemical selected from the group consisting of: phenylalanine and/or tyrosine.
Surprisingly it was found, that the transgenic expression of the Saccharomyces cerevisiae protein as indicated in Table II, column 5, lines 130 and 136 and 148 in Arabidopsis thaliana conferred an increase in alanine and/or aspartic acid and/or phenylalanine (or the respective fine chemical) content of the transformed plants. Thus, in one embodiment, said protein or its homologs are used for the production of alanine; in one embodiment, said protein or its homologs are used for the production of aspartic acid, in one embodiment, said protein or its homologs are used for the production of phenylalanine, in one embodiment, said protein or its homologs are used for the production of one or more fine chemical selected from the group consisting of: alanine and/or aspartic acid and/or phenylalanine.

**[0021.0.0.14] see [0021.0.0.0]**

**[0022.0.14.14]** The sequence of YER152C from Saccharomyces cerevisiae has been published in Dietrich, Nature 387 (6632 Suppl), 78-81, 1997, and Goffeau, Science 274 (5287), 546-547, 1996. It seems to have an activity similar to tyrosine aminotransferase. Accordingly, in one embodiment, the process of the present invention comprises the use of a gene product with an activity similar to tyrosine aminotransferase from Saccaromyces cerevisiae, preferably of the family of valine-pyruvate transaminase or its homolog, e.g. as shown herein, for the production of the the respective fine chemical, meaning of serine, in particular for increasing the amount of serine in free or bound form in an organism or a part thereof, as mentioned. In one embodiment, in the process of the present invention the activity of a similar to tyrosine aminotransferase protein is increased or generated, e.g. from Saccharomyces cerevisiae or a homolog thereof. The sequence of YAL049C from Saccharomyces cerevisiae has been published in Bussey et al., Proc. Natl. Acad. Sci. U.S.A. 92 (9), 3809-3813 (1995), and Goffeau, Science 274 (5287), 546-547, 1996. It activity is not characterized yet . Accordingly, in one embodiment, the process of the present invention comprises the use of a gene product with an activity of a YAL049C protein from Saccaromyces cerevisiae, or its homolog, e.g. as shown herein, for the production of the respective fine chemical, meaning of phenylalanine, in particular for increasing the amount of phenylalanine in free or bound form in an organism or a part thereof, as mentioned. In one embodiment, in the process of the present invention the activity of a YAL049C protein is increased or generated, e.g. from Saccharomyces cerevisiae or a homolog thereof. The sequence of b3008 from Escherichia coli K12 has been published in Blattner et al., Science 277 (5331), 1453-1474, 1997, and its activity is being defined as a cystathionine beta-lyase, PLP-dependent (beta-cystathionase), preferably of the O-succinylhomoserine (thiol)-lyase superfamily. Accordingly, in one embodiment, the process of the present invention comprises the use of a protein of the O-succinylhomoserine (thiol)-lyase superfamily, preferably such protein is having a cystathionine beta-lyase, PLP-dependent (beta-cystathionase) activity, e.g. as shown herein, for the production of the respective fine chemical, meaning of alanine, preferably in free or bound form in an organism or a part thereof, as mentioned. In one embodiment, in the process of the present invention the activity of a cystathionine beta-lyase is increased or generated, e.g. from E. coli or a homolog thereof. The sequence of YOR261C from Saccharomyces cerevisiae has been published in Dujon,et al., Nature 387 (6632 Suppl), 98-102 (1997), and Goffeau, Science 274 (5287), 546-547, 1996 and its activity is being defined as a proteasome regulatory particle subunit, preferably of the mov-34 protein superfamily. Accordingly, in one embodiment, the process of the present invention comprises the use of a gene product with an activity of mov-34 protein superfamily, preferably such protein is having a proteasome regulatory particle subunit activity or its homolog, e.g. as shown herein, for the production of the the respective fine chemical, meaning of serine, in particular for increasing the amount of serine, preferably of serine in free or bound form in an organism or a part thereof, as mentioned. In one embodiment, in the process of the present invention the activity of a proteasome regulatory particle subunit is increased or generated, e.g. from Saccharomyces cerevisiae or a homolog thereof. The sequence of YOR245C from Saccharomyces cerevisiae has been published in Dujon,et al., Nature 387 (6632 Suppl), 98-102 (1997), and Goffeau, Science 274 (5287), 546-547, 1996 and its activity is being defined as a Acyl-CoA:diacylglycerol acyltransferase, preferably of the Caenorhabditis elegans hypothetical protein K07B1.4 superfamily. Accordingly, in one embodiment, the process of the present invention comprises the use of a gene product with an activity of Caenorhabditis elegans hypothetical protein K07B1.4 superfamily, preferably such protein is having a Acyl-CoA:diacylglycerol acyltransferase activity or its homolog, e.g. as shown herein, for the production of the the respective fine chemical, meaning of citrulline, in particular for increasing the amount of citrulline in free or bound form in an organism or a part thereof, as mentioned. In one embodiment, in the process of the present invention the activity of a Acyl-CoA:diacylglycerol acyltransferase is increased or generated, e.g. from Saccharomyces cerevisiae or a homolog thereof. The sequence of YOL123W from Saccharomyces cerevisiae has been published in Dujon,et al., Nature 387 (6632 Suppl), 98-102 (1997), and Goffeau, Science 274 (5287), 546-547, 1996 and its activity is being defined as a cleavage and polyadenylation factor CF I component involved in pre-mRNA 3'-end processing, preferably of the heterogeneous nuclear ribonucleoprotein HRP1 - yeast (Saccharomyces cerevisiae) superfamily. Accordingly, in one embodiment, the process of the present invention comprises the use of a gene product with an activity of heterogeneous nuclear ribonucleoprotein HRP1 - yeast (Saccharomyces cerevisiae) superfamily, preferably such protein is having a cleavage and polyadenylation factor CF I component involved in pre-mRNA 3'-end processing activity or its homolog, e.g. as shown herein, for the production of the respective fine chemical, meaning of glycine, in particular for increasing the amount of glycine in free or bound form in an organism or a part thereof, as mentioned. In one embodiment, in the process of the present invention the activity of a cleavage and polyadenylation factor CF I component involved in pre-mRNA 3'-end processing is increased or generated, e.g. from Saccharomyces cerevisiae or a homolog thereof. The sequence of YLR082C from Saccharomyces cerevisiae has been published in Johnston, Nature 387 (6632 Suppl), 87-90, (1997), and Goffeau, Science 274 (5287), 546-547, 1996, and Goffeau, Science 274 (5287), 546-547, 1996 and its activity is being defined as a suppressor of Rad53 null lethality. Accordingly, in one embodiment, the process of the present invention comprises the use of a gene product with an activity of a suppressor of Rad53 null lethality activity or its homolog, e.g. as shown herein, for the production of the respective fine chemical, meaning of serine, in particular for increasing the amount of serine in free or bound form in an organism or a part thereof, as mentioned. In one embodiment, in the process of the present invention the activity of a suppressor of Rad53 null lethality is increased or generated, e.g. from Saccharomyces cerevisiae or a homolog thereof. The sequence of b0695 from Escherichia coli K12 has been published in Blattner et al., Science 277(5331), 1453-1474, 1997, and its activity is being defined as sensory histidine kinase in two-component regulatory system, preferably of the sensor histidine kinase homology superfamily. Accordingly, in one embodiment, the process of the present invention comprises the use of a gene product with an activity of sensor histidine kinase homology superfamily, preferably such protein is having a sensory histidine kinase in two-component regulatory system activity from E. coli or its homolog, e.g. as shown herein, for the production of the respective fine chemical, meaning of phenylalanine, in particular for increasing the amount of phenylalanine in free or bound form in an organism or a part thereof, as mentioned. In one embodiment, in the process of the present invention the activity of a sensory histidine kinase in two-component regulatory system is increased or generated, e.g. from E. coli or a homolog thereof.
The sequence of b0730 from Escherichia coli K12 has been published in Blattner et al., Science 277(5331), 1453-1474, 1997, and its activity is being defined as transcriptional regulator of succinylCoA synthetase operon and/or fatty acyl response regulator, preferably of the transcription regulator GntR superfamily. Accordingly, in one embodiment, the process of the present invention comprises the use of a gene product with an activity of transcription regulator GntR superfamily, preferably such protein is having a transcriptional regulator of succinylCoA synthetase operon and/or fatty acyl response regulator activity from E. coli or its homolog, e.g. as shown herein, for the production of the respective fine chemical, meaning of aspartic acid, in particular for increasing the amount of aspartic acid in free or bound form in an organism or a part thereof, as mentioned. In one embodiment, in the process of the present invention the activity of a transcriptional regulator of succinylCoA synthetase operon and/or fatty acyl response regulator is increased or generated, e.g. from E. coli or a homolog thereof. The sequence of b1697 from Escherichia coli K12 has been published in Blattner et al., Science 277(5331), 1453-1474, 1997, and its activity is being defined as an electron transfer flavoprotein subunit with ETFP adenine nucleotide-binding like domain, preferably of the electron transfer flavoprotein beta chain superfamily. Accordingly, in one embodiment, the process of the present invention comprises the use of a gene product with an activity of electron transfer flavoprotein beta chain superfamily, preferably such protein is having a electron transfer flavoprotein subunit with ETFP adenine nucleotide-binding like domain activity from E. coli or its homolog, e.g. as shown herein, for the production of the respective fine chemical, meaning of phenylalanine, in particular for increasing the amount of phenylalanine in free or bound form in an organism or a part thereof, as mentioned. In one embodiment, in the process of the present invention the activity of an electron transfer flavoprotein subunit with ETFP adenine nucleotide-binding like domain is increased or generated, e.g. from E. coli or a homolog thereof. The sequence of b1708 from Escherichia coli K12 has been published in Blattner et al., Science 277(5331), 1453-1474, 1997, and its activity is being defined as a lipoprotein, preferably of the conserved hypothetical protein HI1314 superfamily. Accordingly, in one embodiment, the process of the present invention comprises the use of a gene product with an activity of conserved hypothetical protein HI1314 superfamily, preferably such protein is having a lipoprotein activity from E. coli or its homolog, e.g. as shown herein, for the production of the respective fine chemical, meaning of phenylalanine, in particular for increasing the amount of phenylalanine in free or bound form in an organism or a part thereof, as mentioned. In one embodiment, in the process of the present invention the activity of a lipoprotein is increased or generated, e.g. from E. coli or a homolog thereof. The sequence of b1827 from Escherichia coli K12 has been published in Blattner et al., Science 277(5331), 1453-1474, 1997, and its activity is being defined as a transcriptional repressor with DNA-binding Winged helix domain (IcIR family), preferably of the acetate operon repressor superfamily. Accordingly, in one embodiment, the process of the present invention comprises the use of a gene product with an activity of acetate operon repressor superfamily, preferably such protein is having a transcriptional repressor with DNA-binding Winged helix domain (IcIR family) activity from E. coli or its homolog, e.g. as shown herein, for the production of the respective fine chemical, meaning of phenylalanine and/or tyrosine, in particular for increasing the amount of phenylalanine and/or tyrosine, preferably of phenylalanine and/or tyrosine in free or bound form in an organism or a part thereof, as mentioned. In one embodiment, in the process of the present invention the activity of a transcriptional repressor protein with a DNA-binding Winged helix domain (IcIR family) is increased or generated, e.g. from E. coli or a homolog thereof. The sequence of b1829 from Escherichia coli K12 has been published in Blattner, Science 277(5331), 1453-1474, 1997, and its activity is being defined as a heat shock protein with protease activity, preferably of the heat-shock protein htpX superfamily. Accordingly, in one embodiment, the process of the present invention comprises the use of a gene product with an activity of the heat-shock protein htpX superfamily, preferably such protein is having a heat shock protein acitivity with protease activity from E. coli or its homolog, e.g. as shown herein, for the production of the respective fine chemical, meaning of serine and/or tyrosine, in particular for increasing the amount of serine and/or tyrosine, preferably of serine and/or tyrosine in free or bound form in an organism or a part thereof, as mentioned. In one embodiment, in the process of the present invention the activity of a heat shock protein with protease is increased or generated, e.g. from E. coli or a homolog thereof. The sequence of b1886 from Escherichia coli K12 has been published in Blattner, Science 277(5331), 1453-1474, 1997, and its activity is being defined as a methyl-accepting chemotaxis protein II and/or aspartate sensor receptor , preferably of the methyl-accepting chemotaxis protein superfamily. Accordingly, in one embodiment, the process of the present invention comprises the use of a gene product with an activity of the methyl-accepting chemotaxis protein superfamily, preferably such protein is having a methyl-accepting chemotaxis protein II and/or aspartate sensor receptor activity from E. coli or its homolog, e.g. as shown herein, for the production of the respective fine chemical, meaning of serine and/or phenylalanine, in particular for increasing the amount of serine and/or phenylalanine, preferably of serine and/or phenylalanine in free or bound form in an organism or a part thereof, as mentioned. In one embodiment, in the process of the present invention the activity of a methyl-accepting chemotaxis protein II and/or aspartate sensor receptor is increased or generated, e.g. from E. coli or a homolog thereof. The sequence of b1896 from Escherichia coli K12 has been published in Blattner, Science 277(5331), 1453-1474, 1997, and its activity is being defined as a trehalose-6-phosphate synthase, preferably of the Schizosaccharomyces pombe alpha,alpha-trehalose-phosphate synthase (UdP-forming) superfamily. Accordingly, in one embodiment, the process of the present invention comprises the use of a gene product with an activity of the Schizosaccharomyces pombe alpha,alpha-trehalose-phosphate synthase (UdP-forming) superfamily, preferably such protein is having a trehalose-6-phosphate synthase activity from E. coli or its homolog, e.g. as shown herein, for the production of the respective fine chemical, meaning of 5-oxoproline and/or aspartic acid, in particular for increasing the amount of 5-oxoproline and/or aspartic acid, preferably of 5-oxoproline and/or aspartic acid in free or bound form in an organism or a part thereof, as mentioned. In one embodiment, in the process of the present invention the activity of a trehalose-6-phosphate synthase is increased or generated, e.g. from E. coli or a homolog thereof. The sequence of b2095 from Escherichia coli K12 has been published in Blattner, Science 277(5331), 1453-1474, 1997, and its activity is being defined as a tagatose-6-phosphate kinase, preferably of the Escherichia probable tagatose 6-phosphate kinase gatZ superfamily. Accordingly, in one embodiment, the process of the present invention comprises the use of a gene product with an activity of the Escherichia probable tagatose 6-phosphate kinase gatZ superfamily, preferably such protein is having a tagatose-6-phosphate kinase activity from E. coli or its homolog, e.g. as shown herein, for the production of the respective fine chemical, meaning of phenylalanine, in particular for increasing the amount of alanine, preferably of alanine in free or bound form in an organism or a part thereof, as mentioned. In one embodiment, in the process of the present invention the activity of a tagatose-6-phosphate kinase is increased or generated, e.g. from E. coli or a homolog thereof. The sequence of b3256 from Escherichia coli K12 has been published in Blattner, Science 277(5331), 1453-1474, 1997, and its activity is being defined as a acetyl CoA carboxylase and/or biotin carboxylase subunit, preferably of the biotin carboxylase superfamily. Accordingly, in one embodiment, the process of the present invention comprises the use of a gene product with an activity of the biotin carboxylase superfamily, preferably such protein is having a acetyl CoA carboxylase and/or biotin carboxylase subunit activity from E. coli or its homolog, e.g. as shown herein, for the production of the respective fine chemical, meaning of phenylalanine, in particular for increasing the amount of phenylalanine, preferably of phenylalanine in free or bound form in an organism or a part thereof, as mentioned. In one embodiment, in the process of the present invention the activity of a acetyl CoA carboxylase and/or biotin carboxylase subunit is increased or generated, e.g. from E. coli or a homolog thereof. The sequence of b3462 from Escherichia coli K12 has been published in Blattner, Science 277(5331), 1453-1474, 1997, and its activity is being defined as a integral membrane cell division protein, preferably of the cell division protein ftsX superfamily. Accordingly, in one embodiment, the process of the present invention comprises the use of a gene product with an activity of the cell division protein ftsX superfamily, preferably such protein is having a integral membrane cell division protein activity from E. coli or its homolog, e.g. as shown herein, for the production of the respective fine chemical, meaning of serine and/or phenylalanine, in particular for increasing the amount of serine and/or phenylalanine, preferably of serine and/or phenylalanine in free or bound form in an organism or a part thereof, as mentioned. In one embodiment, in the process of the present invention the activity of a integral membrane cell division protein is increased or generated, e.g. from E. coli or a homolog thereof. The sequence of YBL015W from Saccharomyces cerevisiae has been published in Goffeau, Science 274 (5287), 546-547, 1996, and in Feldmann, EMBO J., 13, 5795-5809, 1994 and its activity is being defined as a mannose-containing glycoprotein and/or as an acetyl-CoA hydrolase, preferably of the acetyl-CoA hydrolase superfamily. Accordingly, in one embodiment, the process of the present invention comprises the use of a gene product with an activity of the acetyl-CoA hydrolase superfamily, preferably such protein is having a mannose-containing glycoprotein and/or as an acetyl-CoA hydrolase activity or its homolog, e.g. as shown herein, for the production of the the respective fine chemical, meaning of alanine, in particular for increasing the amount of alanine, preferably of alanine in free or bound form in an organism or a part thereof, as mentioned. In one embodiment, in the process of the present invention the activity of a a mannose-containing glycoprotein and/or as an acetyl-CoA hydrolase is increased or generated, e.g. from Saccharomyces cerevisiae or a homolog thereof. The sequence of YDL127W from Saccharomyces cerevisiae has been published in Jacq et al., Nature 387 (6632 Suppl), 75-78, 1997 and Goffeau, Science 274 (5287), 546-547, 1996, and its activity is being defined as a G1/S-specific cyclin PCL2 (Cyclin HCS26 homolog). Accordingly, in one embodiment, the process of the present invention comprises the use of a gene product with an activity of the G1/S-specific cyclin PCL2 (Cyclin HCS26 homolog) or its homolog, e.g. as shown herein, for the production of the the respective fine chemical, meaning of 5-oxoproline, in particular for increasing the amount of 5-oxoproline, preferably of 5-oxoproline in free or bound form in an organism or a part thereof, as mentioned. In one embodiment, in the process of the present invention the activity of a G1/S-specific cyclin PCL2 (Cyclin HCS26 homolog) is increased or generated, e.g. from Saccharomyces cerevisiae or a homolog thereof. The sequence of YEL045C from Saccharomyces cerevisiae has been published in Dietrich, Nature 387 (6632 Suppl), 78-81, 1997. It activity is not characterized yet . Accordingly, in one embodiment, the process of the present invention comprises the use of a gene product with an activity of a YEL045C protein from Saccaromyces cerevisiae, or its homolog, e.g. as shown herein, for the production of the respective fine chemical, meaning of serine, in particular for increasing the amount of serine, preferably of serine in free or bound form in an organism or a part thereof, as mentioned. In one embodiment, in the process of the present invention the activity of a YEL045C protein is increased or generated, e.g. from Saccharomyces cerevisiae or a homolog thereof. The sequence of YLL009C from Saccharomyces cerevisiae has been published in Johnston et al., Nature 387 (6632 Suppl), 87-90, 1997 and Goffeau, Science 274 (5287), 546-547, 1996, and its activity is being defined as a cytochrome c oxidase copper chaperone. Accordingly, in one embodiment, the process of the present invention comprises the use of a gene product with an activity of the cytochrome c oxidase copper chaperone or its homolog, e.g. as shown herein, for the production of the respective fine chemical, meaning of serine, in particular for increasing the amount of serine preferably of serine in free or bound form in an organism or a part thereof, as mentioned. In one embodiment, in the process of the present invention the activity of a cytochrome c oxidase copper chaperone is increased or generated, e.g. from Saccharomyces cerevisiae or a homolog thereof. The sequence of YER173W from Saccharomyces cerevisiae has been published in Dietrich et al., Nature 387 (6632 Suppl), 78-81, 1997, and Goffeau et al., Science 274 (5287), 546-547, 1996, and its activity is being defined as a checkpoint protein, involved in the activation of the DNA damage and meiotic pachytene checkpoints. Accordingly, in one embodiment, the process of the present invention comprises the use of a gene product with an activity of Checkpoint protein, involved in the activation of the DNA damage and meiotic pachytene checkpoints or its homolog, e.g. as shown herein, for the production of the respective fine chemical, meaning of alanine and/or aspartic acid, of alanine and or phenylalanine, of aspartic acid and/or phenylalanine, of alanine and/or aspartic acid and/or phenylalanine, in particular for increasing the amount of one or two or all amino acid(s) selected from the group consisting of alanine and/or aspartic acid and/or phenylalanine, preferably of one or two or all amino acid(s) selected from the group consisting of alanine and/or aspartic acid and/or phenylalanine in free or bound form in an organism or a part thereof, as mentioned. In one embodiment, in the process of the present invention the activity of a Checkpoint protein, involved in the activation of the DNA damage and meiotic pachytene checkpoints is increased or generated, e.g. from Saccharomyces cerevisiae or a homolog thereof. The sequence of YFL050C from Saccharomyces cerevisiae has been published in Murakami et al., Nat. Genet. 10 (3), 261-268, 1995, and Goffeau et al., Science 274 (5287), 546-547, 1996, and its activity is being defined as a di-and/or trivalent inorganic cation transporter, preferably of the magnesium and cobalt transport protein superfamily. Accordingly, in one embodiment, the process of the present invention comprises the use of a gene product with an activity of the magnesium and cobalt transport protein superfamily, preferably such protein is having a di-and/or trivalent inorganic cation transporter activity or its homolog, e.g. as shown herein, for the production of the the respective fine chemical, meaning of alanine and/or glycine, of alanine and or tyrosine, of glycine and/or tyrosine, of alanine and/or glycine and/or tyrosine, in particular for increasing the amount of one or two or all amino acid(s) selected from the group consisting of alanine and/or glycine and/or tyrosine, preferably of one or two or all amino acid(s) selected from the group consisting of alanine and/or glycine and/or tyrosine in free or bound form in an organism or a part thereof, as mentioned. In one embodiment, in the process of the present invention the activity of a di-and/or trivalent inorganic cation transporter is increased or generated, e.g. from Saccharomyces cerevisiae or a homolog thereof. The sequence of YGR101W from Saccharomyces cerevisiae has been published in Tettelin et al., Nature 387 (6632 Suppl), 81-84 (1997) and Goffeau et al., Science 274 (5287), 546-547, 1996, and its activity is being defined as a rhomboid protease. Accordingly, in one embodiment, the process of the present invention comprises the use of a gene product with an activity of rhomboid protease or its homolog, e.g. as shown herein, for the production of the respective fine chemical, meaning of phenylalanine, in particular for increasing the amount of phenylalanine, preferably of phenylalanine in free or bound form in an organism or a part thereof, as mentioned. In one embodiment, in the process of the present invention the activity of a rhomboid protease is increased or generated, e.g. from Saccharomyces cerevisiae or a homolog thereof.
The sequence of YGR104C from Saccharomyces cerevisiae has been published in Thompson et al., Cell 73:1361-1375(1993) and its activity is being defined as a RNA polymerase II suppressor protein SRB5 from yeast; and/or as an suppressor of RNA polymerase B SRB5, preferably of the RNA polymerase II suppressor protein SRB5 superfamily. Accordingly, in one embodiment, the process of the present invention comprises the use of a gene product with an activity of the RNA polymerase II suppressor protein SRB5 superfamily, preferably such protein is having a RNA polymerase II suppressor protein SRB5 from yeast; and/or a suppressor of RNA polymerase B SRB5 activity or its homolog, e.g. as shown herein, for the production of the the respective fine chemical, meaning of aspartic acid, in particular for increasing the amount of aspartic acid, preferably of aspartic acid in free or bound form in an organism or a part thereof, as mentioned. In one embodiment, in the process of the present invention the activity of a RNA polymerase II suppressor protein SRB5 from yeast and/or as an suppressor of RNA polymerase B SRB5 is increased or generated, e.g. from Saccharomyces cerevisiae or a homolog thereof. The sequence of YHR130C from Saccharomyces cerevisiae has been published in Johnston et al., Science 265:2077-2082(1994). Its activity is not characterized yet. Accordingly, in one embodiment, the process of the present invention comprises the use of a gene product with an activity of a YHR130C protein from Saccaromyces cerevisiae, or its homolog, e.g. as shown herein, for the production of the respective fine chemical, meaning of phenylalanine and/or tyrosine, in particular for increasing the amount of phenylalanine and/or tyrosine, preferably of phenylalanine and/or tyrosine in free or bound form in an organism or a part thereof, as mentioned. In one embodiment, in the process of the present invention the activity of a YHR130C protein is increased or generated, e.g. from Saccharomyces cerevisiae or a homolog thereof. The sequence of YIL150C from Saccharomyces cerevisiae has been published in Churcher et al., Nature 387 (6632 Suppl), 84-87 (1997) and Goffeau et al., Science 274 (5287), 546-547, 1996, and its activity is being defined as a chromatin binding protein, required for S-phase (DNA synthesis) initiation or completion. Accordingly, in one embodiment, the process of the present invention comprises the use of a gene product with an activity of chromatin binding protein, required for S-phase (DNA synthesis) initiation or completion or its homolog, e.g. as shown herein, for the production of the respective fine chemical, meaning of alanine and aspartic acid, alanine and serine, alanine and tyrosine, aspartic acid and serine, aspartic acid and tyrosine, serine and tyrosine, alanine and aspartic acid and serine, alanine and aspartic acid and tyrosine, alanine and serine and tyrosine, aspartic acid and serine and tyrosine and/or alanine and aspartic acid and serine and tyrosine, in particular for increasing the amount of alanine and aspartic acid, alanine and serine, alanine and tyrosine, aspartic acid and serine, aspartic acid and tyrosine, serine and tyrosine, alanine and aspartic acid and serine, alanine and aspartic acid and tyrosine, alanine and serine and tyrosine, aspartic acid and serine and tyrosine and/or alanine and aspartic acid and serine and tyrosine, preferably of alanine and aspartic acid, alanine and serine, alanine and tyrosine, aspartic acid and serine, aspartic acid and tyrosine, serine and tyrosine, alanine and aspartic acid and serine, alanine and aspartic acid and tyrosine, alanine and serine and tyrosine, aspartic acid and serine and tyrosine and/or alanine and aspartic acid and serine and tyrosine in free or bound form in an organism or a part thereof, as mentioned. In one embodiment, in the process of the present invention the activity of a chromatin binding protein, required for S-phase (DNA synthesis) initiation or completion is increased or generated, e.g. from Saccharomyces cerevisiae or a homolog thereof. The sequence of YJL072C from Saccharomyces cerevisiae has been published in Goffeau, Science 274 (5287), 546-547 (1996) and Galibert,EMBO J. 15 (9), 2031-2049 (1996) and its activity is being defined as a subunit of the GINS complex required for chromosomal DNA replication, preferably of the Saccharomyces cerevisiae probable membrane protein YJL072c superfamily. Accordingly, in one embodiment, the process of the present invention comprises the use of a gene product with an activity of the Saccharomyces cerevisiae probable membrane protein YJL072c superfamily, preferably such protein is having a subunit of the GINS complex required for chromosomal DNA replication activity or its homolog, e.g. as shown herein, for the production of the respective fine chemical, meaning of phenylalanine, in particular for increasing the amount of phenylalanine, preferably of phenylalanine in free or bound form in an organism or a part thereof, as mentioned. In one embodiment, in the process of the present invention the activity of a subunit of the GINS complex required for chromosomal DNA replication is increased or generated, e.g. from Saccharomyces cerevisiae or a homolog thereof. The sequence of YKR057W from Saccharomyces cerevisiae has been published in Goffeau, Science 274 (5287), 546-547 (1996) and Dujon, Nature 369 (6479), 371-378 (1994) and its activity is being defined as a ribosomal protein, similar to S21 ribosomal proteins, involved in ribosome biogenesis and translation, preferably of the rat ribosomal protein S21 superfamily. Accordingly, in one embodiment, the process of the present invention comprises the use of a gene product with an activity of the rat ribosomal protein S21 superfamily, preferably such protein is having a ribosomal protein, similar to S21 ribosomal proteins, involved in ribosome biogenesis and translation activity or its homolog, e.g. as shown herein, for the production of the respective fine chemical, meaning of serine, in particular for increasing the amount of serine, preferably of serine in free or bound form in an organism or a part thereof, as mentioned. In one embodiment, in the process of the present invention the activity of a ribosomal protein, similar to S21 ribosomal proteins, involved in ribosome biogenesis and translation is increased or generated, e.g. from Saccharomyces cerevisiae or a homolog thereof. The sequence of YLL013C from Saccharomyces cerevisiae has been published in Johnston, Nature 387 (6632 Suppl), 87-90 (1997) and Goffeau et al., Science 274 (5287), 546-547, 1996, and its activity is being defined as a member of the PUF protein family, which is named for the founding members, pumilio and Fbf. Accordingly, in one embodiment, the process of the present invention comprises the use of a gene product with an activity of member of the PUF protein family, which is named for the founding members, pumilio and Fbf or its homolog, e.g. as shown herein, for the production of the the respective fine chemical, meaning of citrulline, in particular for increasing the amount of citrulline, preferably of citrulline in free or bound form in an organism or a part thereof, as mentioned. In one embodiment, in the process of the present invention the activity of a member of the PUF protein family, which is named for the founding members, pumilio and Fbf is increased or generated, e.g. from Saccharomyces cerevisiae or a homolog thereof. The sequence of YOR350C from Saccharomyces cerevisiae has been published in Goffeau, Science 274 (5287), 546-547 (1996) and Dujon, Nature 369 (6479), 371-378 (1994) and its activity is not been characterized yet. It seems to be member of the Saccharomyces cerevisiae MNE1 protein superfamily. Accordingly, in one embodiment, the process of the present invention comprises the use of a gene product with an activity of the Saccharomyces cerevisiae MNE1 protein superfamily, preferably such protein is having a YOR350C activity or its homolog, e.g. as shown herein, for the production of the the respective fine chemical, meaning of tyrosine, in particular for increasing the amount of tyrosine, preferably of tyrosine in free or bound form in an organism or a part thereof, as mentioned. In one embodiment, in the process of the present invention the activity of a YOR350C protein is increased or generated, e.g. from Saccharomyces cerevisiae or a homolog thereof. The sequence of YPR138C from Saccharomyces cerevisiae has been published in Goffeau, Science 274 (5287), 546-547 (1996) and Bussey, Nature 387 (6632 Suppl), 103-105 (1997) and its activity is being defined as a subunit of the NH4+ transporter, preferably of the ammonium transport protein and/or ammonium transporter nrgA superfamily. Accordingly, in one embodiment, the process of the present invention comprises the use of a gene product with an activity of the ammonium transport protein and/or ammonium transporter nrgA superfamily, preferably such protein is having a sNH4+ transporter activity or its homolog, e.g. as shown herein, for the production of the the respective fine chemical, meaning of phenylalanine, in particular for increasing the amount of phenylalanine, preferably of phenylalanine in free or bound form in an organism or a part thereof, as mentioned. In one embodiment, in the process of the present invention the activity of a NH4+ transporter is increased or generated, e.g. from Saccharomyces cerevisiae or a homolog thereof. The sequence of YEL046C from Saccharomyces cerevisiae has been published in Goffeau, Science 274 (5287), 546-547 (1996) and Dietrich, Nature 387 (6632 Suppl), 78-81 (1997) and its activity is being defined as a low specificity L-threonine aldolase. Accordingly, in one embodiment, the process of the present invention comprises the use of a gene product with an activity of the low specificity L-threonine aldolase or its homolog, e.g. as shown herein, for the production of the respective fine chemical, meaning of homoserine, in particular for increasing the amount of homoserine, preferably of homoserine in free or bound form in an organism or a part thereof, as mentioned. In one embodiment, in the process of the present invention the activity of a low specificity L-threonine aldolase is increased or generated, e.g. from Saccharomyces cerevisiae or a homolog thereof.
The sequence of b0057 from Escherichia coli K12 has been published in Blattner et al., Science 277(5331), 1453-1474, 1997, and its activity has not been characterized yet. Accordingly, in one embodiment, the process of the present invention comprises the use of a gene product with an activity of the b0057 protein from E. coli or its homolog, e.g. as shown herein, for the production of the respective fine chemical, meaning of citrulline, glycine and/or serine, in particular for increasing the amount of citrulline, in particular for increasing the amount of glycine, in particular for increasing the amount of serine, in particular for increasing the amount of citrulline and glycine, in particular for increasing the amount of citrulline and serine, in particular for increasing the amount of glycine and serine, in particular for increasing the amount of citrulline and glycine and serine, preferably of citrulline, glycine and/or serine in free or bound form in an organism or a part thereof, as mentioned.
The sequence of b0161 from Escherichia coli K12 has been published in Blattner et al., Science 277(5331), 1453-1474, 1997, and its activity has been defined as a periplasmic serine protease (heat shock protein), preferably of the Helicobacter serine proteinase superfamily. Accordingly, in one embodiment, the process of the present invention comprises the use of a gene product with an activity of the periplasmic serine protease (heat shock protein) from E. coli or its homolog, e.g. as shown herein, for the production of the respective fine chemical, meaning of 5-oxoproline, aspartic acid and/or phenylalanine, in particular for increasing the amount of 5-oxoproline, in particular for increasing the amount of aspartic acid, in particular for increasing the amount of phenylalanine, in particular for increasing the amount of 5-oxoproline and aspartic acid, in particular for increasing the amount of 5-oxoproline and phenylalanine, in particular for increasing the amount of aspartic acid and phenylalanine, in particular for increasing the amount of 5-oxoproline and aspartic acid and phenylalanine, preferably of 5-oxoproline, aspartic acid and/or phenylalanine in free or bound form in an organism or a part thereof, as mentioned.
The sequence of b0236 from Escherichia coli K12 has been published in Blattner et al., Science 277 (5331), 1453-1474, 1997, and its activity is being defined as a Peptide chain release factor homolog, preferably of the translation releasing factor superfamily. Accordingly, in one embodiment, the process of the present invention comprises the use of a gene product with an activity of a Peptide chain release factor homolog from E. coli or its homolog, e.g. as shown herein, for the production of the respective fine chemical, meaning of alanine, in particular for increasing the amount of alanine, preferably alanine in free or bound form in an organism or a part thereof, as mentioned.
The sequence of 0376 from Escherichia coli K12 has been published in Blattner et al., Science 277 (5331), 1453-1474, 1997, and its activity is being defined as a beta-lactamase/D-ala carboxypeptidase, penicillin-binding protein, preferably of the Escherichia coli beta-lactamase superfamily. Accordingly, in one embodiment, the process of the present invention comprises the use of a gene product with an activity of a beta-lactamase/D-ala carboxypeptidase, penicillin-binding protein from E. coli or its homolog, e.g. as shown herein, for the production of the respective fine chemical, meaning of 5-oxoproline, in particular for increasing the amount of 5-oxoproline, preferably 5-oxoproline in free or bound form in an organism or a part thereof, as mentioned.
The sequence of b0462 from Escherichia coli K12 has been published in Blattner et al., Science 277 (5331), 1453-1474, 1997, and its activity is being defined as a acridine efflux pump protein, preferably of the acriflavin resistance protein superfamily. Accordingly, in one embodiment, the process of the present invention comprises the use of a gene product with an activity of a acridine efflux pump protein from E. coli or its homolog,, e.g. as shown herein, for the production of the respective fine chemical, meaning of citrulline, in particular for increasing the amount of citrulline, preferably citrulline in free or bound form in an organism or a part thereof, as mentioned.
The sequence of b0486 from Escherichia coli K12 has been published in Blattner et al., Science 277(5331), 1453-1474, 1997, and its activity has been defined as a putative amino-acid/amine transport protein (APC family), preferably of the probable membrane protein ybaT superfamily. Accordingly, in one embodiment, the process of the present invention comprises the use of a gene product with an activity of the putative amino-acid/amine transport protein (APC family) from E. coli or its homolog, e.g. as shown herein, for the production of the respective fine chemical, meaning of alanine and/or serine, in particular for increasing the amount of alanine, in particular for increasing the amount of serine, in particular for increasing the amount of alanine and serine, preferably of alanine and/or serine in free or bound form in an organism or a part thereof, as mentioned.
The sequence of b0577 from Escherichia coli K12 has been published in Blattner et al., Science 277(5331), 1453-1474, 1997, and its activity has been defined as a putative transport protein. Accordingly, in one embodiment, the process of the present invention comprises the use of a gene product with an activity of the putative transport protein from E. coli or its homolog, e.g. as shown herein, for the production of the respective fine chemical, meaning of glycine and/or aspartic acid, in particular for increasing the amount of glycine, in particular for increasing the amount of aspartic acid, in particular for increasing the amount of glycine and aspartic acid, preferably of glycine and/or aspartic acid in free or bound form in an organism or a part thereof, as mentioned.
The sequence of b0970 from Escherichia coli K12 has been published in Blattner et al., Science 277(5331), 1453-1474, 1997, and its activity has been defined as a probable glutamate receptor, preferably of the Escherichia coli ybhL protein superfamily. Accordingly, in one embodiment, the process of the present invention comprises the use of a gene product with an activity of the probable glutamate receptor from E. coli or its homolog, e.g. as shown herein, for the production of the respective fine chemical, meaning of 5-oxoproline and/or tyrosine, in particular for increasing the amount of 5-oxoproline, in particular for increasing the amount of tyrosine, in particular for increasing the amount of 5-oxoproline and tyrosine, preferably of 5-oxoproline and/or tyrosine in free or bound form in an organism or a part thereof, as mentioned.
The sequence of b1228 from Escherichia coli K12 has been published in Blattner et al., Science 277 (5331), 1453-1474, 1997, and its activity has not been characterized yet. Accordingly, in one embodiment, the process of the present invention comprises the use of a gene product with an activity of the b1228 protein from E. coli or its homolog, e.g. as shown herein, for the production of the respective fine chemical, meaning of phenylalanine, in particular for increasing the amount of phenylalanine, preferably phenylalanine in free or bound form in an organism or a part thereof, as mentioned.
The sequence of b1275 from Escherichia coli K12 has been published in Blattner et al., Science 277 (5331), 1453-1474, 1997, and its activity is being defined as a transcriptional regulator of biosynthesis of L-cysteine and regulator of sulfur assimilation (LysR familiy), preferably of the regulatory protein IysR superfamily. Accordingly, in one embodiment, the process of the present invention comprises the use of a gene product with an activity of a transcriptional regulator of biosynthesis of L-cysteine and regulator of sulfur assimilation (LysR familiy) from E. coli or its homolog, e.g. as shown herein, for the production of the respective fine chemical, meaning of citrulline, in particular for increasing the amount of citrulline, preferably citrulline in free or bound form in an organism or a part thereof, as mentioned.
The sequence of b1343 from Escherichia coli K12 has been published in Blattner et al., Science 277(5331), 1453-1474, 1997, and its activity has been defined as a ATP-dependent RNA helicase, stimulated by 23S rRNA. Accordingly, in one embodiment, the process of the present invention comprises the use of a gene product with an activity of the ATP-dependent RNA helicase, stimulated by 23S rRNA from E. coli or its homolog, e.g. as shown herein, for the production of the respective fine chemical, meaning of alanine, 5-oxoproline and/or aspartic acid, in particular for increasing the amount of alanine, in particular for increasing the amount of 5-oxoproline, in particular for increasing the amount of aspartic acid, in particular for increasing the amount of alanine and 5-oxoproline, in particular for increasing the amount of alanine and aspartic acid, in particular for increasing the amount of 5-oxoproline and aspartic acid, in particular for increasing the amount of alanine and 5-oxoproline and aspartic acid, preferably of alanine, 5-oxoproline and/or aspartic acid in free or bound form in an organism or a part thereof, as mentioned.
The sequence of b1360 from Escherichia coli K12 has been published in Blattner et al., Science 277 (5331), 1453-1474, 1997, and its activity is being defined as a putative DNA replication protein, preferably of the DNA replication protein dnaC superfamily. Accordingly, in one embodiment, the process of the present invention comprises the use of a gene product with an activity of a putative DNA replication protein from E. coli or its homolog, e.g. as shown herein, for the production of the respective fine chemical, meaning of citrulline, in particular for increasing the amount of citrulline, preferably citrulline in free or bound form in an organism or a part thereof, as mentioned.
The sequence of b1863 from Escherichia coli K12 has been published in Blattner et al., Science 277 (5331), 1453-1474, 1997, and its activity is being defined as a Crossover junction endodeoxyribonuclease, preferably of the DNA repair protein ruvC superfamily. Accordingly, in one embodiment, the process of the present invention comprises the use of a gene product with an activity of a Crossover junction endodeoxyribonuclease from E. coli or its homolog, e.g. as shown herein, for the production of the respective fine chemical, meaning of alanine, in particular for increasing the amount of alanine, preferably alanine in free or bound form in an organism or a part thereof, as mentioned.
The sequence of b2023 from Escherichia coli K12 has been published in Blattner et al., Science 277 (5331), 1453-1474, 1997, and its activity is being defined as a glutamine amidotransferase subunit of imidazole glycerol phosphate synthase heterodimer, preferably of the amidotransferase hisH, trpG homology superfamily. Accordingly, in one embodiment, the process of the present invention comprises the use of a gene product with an activity of a glutamine amidotransferase subunit of imidazole glycerol phosphate synthase heterodimer from E. coli or its homolog, e.g. as shown herein, for the production of the respective fine chemical, meaning of aspartic acid, in particular for increasing the amount of aspartic acid, preferably aspartic acid in free or bound form in an organism or a part thereof, as mentioned.
The sequence of b2078 from Escherichia coli K12 has been published in Blattner et al., Science 277 (5331), 1453-1474, 1997, and its activity is being defined as a sensory histidine kinase in two-component regulatory system, preferably of the sensor histidine kinase homology, envZ protein superfamily. Accordingly, in one embodiment, the process of the present invention comprises the use of a gene product with an activity of a sensory histidine kinase in two-component regulatory system from E. coli or its homolog, e.g. as shown herein, for the production of the respective fine chemical, meaning of phenylalanine, in particular for increasing the amount of phenylalanine, preferably phenylalanine in free or bound form in an organism or a part thereof, as mentioned.
The sequence of b2239 from Escherichia coli K12 has been published in Blattner et al., Science 277 (5331), 1453-1474, 1997, and its activity is being defined as a glycerophosphodiester phosphodiesterase. Accordingly, in one embodiment, the process of the present invention comprises the use of a gene product with an activity of a glycerophosphodiester phosphodiesterase from E. coli or its homolog, e.g. as shown herein, for the production of the respective fine chemical, meaning of citrulline, in particular for increasing the amount of citrulline, preferably citrulline in free or bound form in an organism or a part thereof, as mentioned.
The sequence of b2414 from Escherichia coli K12 has been published in Blattner et al., Science 277(5331), 1453-1474, 1997, and its activity has been defined as a subunit of cysteine synthase A and O-acetylserine sulfhydrolase A, PLP-dependent enzyme, preferably of the threonine dehydratase superfamily. Accordingly, in one embodiment, the process of the present invention comprises the use of a gene product with an activity of the subunit of cysteine synthase A and O-acetylserine sulfhydrolase A, PLP-dependent enzyme from E. coli or its homolog, e.g. as shown herein, for the production of the respective fine chemical, meaning of citrulline, glycine, serine and/or phenylalanine, in particular for increasing the amount of citrulline, in particular for increasing the amount of glycine, in particular for increasing the amount of serine, in particular for increasing the amount of phenylalanine, in particular for increasing the amount of citrulline and glycine, in particular for increasing the amount of citrulline and serine, in particular for increasing the amount of citrulline and phenylalanine, in particular for increasing the amount of glycine and serine, in particular for increasing the amount of glycine and phenylalanine, in particular for increasing the amount of serine and phenylalanine, in particular for increasing the amount of citrulline and glycine and serine, in particular for increasing the amount of citrulline and glycine and phenylalanine, in particular for increasing the amount of citrulline and serine and phenylalanine, in particular for increasing the amount of citrulline and glycine and phenylalanine, in particular for increasing the amount of citrulline and glycine and serine and phen, preferably of citrulline, glycine, serine and/or phenylalanine in free or bound form in an organism or a part thereof, as mentioned.
The sequence of b2426 from Escherichia coli K12 has been published in Blattner et al., Science 277 (5331), 1453-1474, 1997, and its activity is being defined as a putative oxidoreductase with NAD(P)-binding domain, preferably of the ribitol dehydrogenase, short-chain alcohol dehydrogenase homology superfamily. Accordingly, in one embodiment, the process of the present invention comprises the use of a gene product with an activity of a putative oxidoreductase with NAD(P)-binding domain from E. coli or its homolog, e.g. as shown herein, for the production of the respective fine chemical, meaning of citrulline, in particular for increasing the amount of citrulline, preferably citrulline in free or bound form in an organism or a part thereof, as mentioned.
The sequence of b2489 from Escherichia coli K12 has been published in Blattner et al., Science 277(5331), 1453-1474, 1997, and its activity has been defined as a hydrogenase Fe-S subunit, preferably of the psbG protein superfamily. Accordingly, in one embodiment, the process of the present invention comprises the use of a gene product with an activity of the hydrogenase Fe-S subunit from E. coli or its homolog, e.g. as shown herein, for the production of the respective fine chemical, meaning of citrulline, alanine, glycine and/or serine, in particular for increasing the amount of citrulline, in particular for increasing the amount of alanine, in particular for increasing the amount of glycine, in particular for increasing the amount of serine,in particular for increasing the amount of citrulline and alanine, in particular for increasing the amount of citrulline and glycine, in particular for increasing the amount of citrulline and serine, in particular for increasing the amount of alanine and glycine, in particular for increasing the amount of alanine and serine, in particular for increasing the amount of glycine and serine, in particular for increasing the amount of citrulline and alanine and glycine, in particular for increasing the amount of citrulline and alanine and serine, in particular for increasing the amount of citrulline and glycine and serine, in particular for increasing the amount of alanine and glycine and serine, in particular for increasing the amount of citrulline and alanine and glycine and serine, preferably of citrulline, alanine, glycine and/or serine in free or bound form in an organism or a part thereof, as mentioned.
The sequence of b2491 from Escherichia coli K12 has been published in Blattner et al., Science 277 (5331), 1453-1474, 1997, and its activity is being defined as a transcriptional activator for expression of hydrogenase 4 genes, interacts with sigma 54 (EBP family). Accordingly, in one embodiment, the process of the present invention comprises the use of a gene product with an activity of a transcriptional activator for expression of hydrogenase 4 genes, interacts with sigma 54 (EBP family) from E. coli or its homolog, e.g. as shown herein, for the production of the respective fine chemical, meaning of tyrosine, in particular for increasing the amount of tyrosine, preferably tyrosine in free or bound form in an organism or a part thereof, as mentioned.
The sequence of b2507 from Escherichia coli K12 has been published in Blattner et al., Science 277 (5331), 1453-1474, 1997, and its activity is being defined as a GMP synthetase (glutamine aminotransferase), preferably of the GMP synthase (glutamine-hydrolyzing), trpG homology superfamily. Accordingly, in one embodiment, the process of the present invention comprises the use of a gene product with an activity of a GMP synthetase (glutamine aminotransferase) from E. coli or its homolog, e.g. as shown herein, for the production of the respective fine chemical, meaning of aspartic acid, in particular for increasing the amount of aspartic acid, preferably aspartic acid in free or bound form in an organism or a part thereof, as mentioned.
The sequence of b2553 from Escherichia coli K12 has been published in Blattner et al., Science 277 (5331), 1453-1474, 1997, and its activity is being defined as a regulatory protein P-II for glutamine synthetase, preferably of the regulatory protein P-II superfamily. Accordingly, in one embodiment, the process of the present invention comprises the use of a gene product with an activity of a regulatory protein P-II for glutamine synthetase from E. coli or its homolog, e.g. as shown herein, for the production of the respective fine chemical, meaning of glycine, in particular for increasing the amount of glycine, preferably glycine in free or bound form in an organism or a part thereof, as mentioned.
The sequence of b2576 from Escherichia coli K12 has been published in Blattner et al., Science 277(5331), 1453-1474, 1997, and its activity has been defined as a ATP-dependent RNA helicase. Accordingly, in one embodiment, the process of the present invention comprises the use of a gene product with an activity of the ATP-dependent RNA helicase from E. coli or its homolog, e.g. as shown herein, for the production of the respective fine chemical, meaning of alanine, glycine, aspartic acid and/or phenylalanine, in particular for increasing the amount of alanine, in particular for increasing the amount of glycine, in particular for increasing the amount of aspartic acid, in particular for increasing the amount of phenylalanine,in particular for increasing the amount of alanine and glycine, in particular for increasing the amount of alanine and aspartic acid, in particular for increasing the amount of alanine and phenylalanine, in particular for increasing the amount of glycine and aspartic acid, in particular for increasing the amount of glycine and phenylalanine, in particular for increasing the amount of aspartic acid and phenylalanine, in particular for increasing the amount of alanine and glycine and aspartic acid, in particular for increasing the amount of alanine and glycine and phenylalanine, in particular for increasing the amount of alanine and aspartic acid and phenylalanine, in particular for increasing the amount of glycine and aspartic acid and phenylalanine, in particular for increasing the amount of alanine and glycine and aspartic acid and phenylalanine, preferably of alanine, glycine, aspartic acid and/or phenylalanine in free or bound form in an organism or a part thereof, as mentioned.
The sequence of b2664 from Escherichia coli K12 has been published in Blattner et al., Science 277 (5331), 1453-1474, 1997, and its activity is being defined as a putative transcriptional repressor with DNA-binding Winged helix domain (GntR familiy), preferably of the probable transcription regulator gabP superfamily. Accordingly, in one embodiment, the process of the present invention comprises the use of a gene product with an activity of a putative transcriptional repressor with DNA-binding Winged helix domain (GntR familiy) from E. coli or its homolog, e.g. as shown herein, for the production of the respective fine chemical, meaning of serine, in particular for increasing the amount of serine, preferably serine in free or bound form in an organism or a part thereof, as mentioned.
The sequence of b2753 from Escherichia coli K12 has been published in Blattner et al., Science 277 (5331), 1453-1474, 1997, and its activity is being defined as a aminopeptidase in alkaline phosphatase isozyme conversion, preferably of the alkaline phosphatase isozyme conversion protein superfamily. Accordingly, in one embodiment, the process of the present invention comprises the use of a gene product with an activity of a aminopeptidase in alkaline phosphatase isozyme conversion from E. coli or its homolog, e.g. as shown herein, for the production of the respective fine chemical, meaning of aspartic acid, in particular for increasing the amount of aspartic acid, preferably aspartic acid in free or bound form in an organism or a part thereof, as mentioned.
The sequence of b2796 from Escherichia coli K12 has been published in Blattner et al., Science 277 (5331), 1453-1474, 1997, and its activity is being defined as a putative serine transport protein (HAAAP family), preferably of the threonine-serine permease superfamily. Accordingly, in one embodiment, the process of the present invention comprises the use of a gene product with an activity of a putative serine transport protein (HAAAP family) from E. coli or its homolog, e.g. as shown herein, for the production of the respective fine chemical, meaning of phenylalanine, in particular for increasing the amount of phenylalanine, preferably phenylalanine in free or bound form in an organism or a part thereof, as mentioned.
The sequence of b3064 from Escherichia coli K12 has been published in Blattner et al., Science 277 (5331), 1453-1474, 1997, and its activity is being defined as a putative O-sialoglycoprotein endopeptidase, with actin-like ATPase domain, preferably of the O-sialoglycoprotein endopeptidase superfamily. Accordingly, in one embodiment, the process of the present invention comprises the use of a gene product with an activity of a putative O-sialoglycoprotein endopeptidase, with actin-like ATPase domain from E. coli or its homolog, e.g. as shown herein, for the production of the respective fine chemical, meaning of serine, in particular for increasing the amount of serine, preferably serine in free or bound form in an organism or a part thereof, as mentioned.
The sequence of b3116 from Escherichia coli K12 has been published in Blattner et al., Science 277(5331), 1453-1474, 1997, and its activity has been defined as a L-threonine/L-serine permease, anaerobically inducible (HAAAP family), preferably of the threonine-serine permease superfamily. Accordingly, in one embodiment, the process of the present invention comprises the use of a gene product with an activity of the L-threonine/L-serine permease, anaerobically inducible (HAAAP family) from E. coli or its homolog, e.g. as shown herein, for the production of the respective fine chemical, meaning of serine and/or aspartic acid, in particular for increasing the amount of serine, in particular for increasing the amount of aspartic acid, in particular for increasing the amount of serine and aspartic acid, preferably of serine and/or aspartic acid in free or bound form in an organism or a part thereof, as mentioned.
The sequence of b3160 from Escherichia coli K12 has been published in Blattner et al., Science 277(5331), 1453-1474, 1997, and its activity has been defined as a putative monooxygenase with luciferase-like ATPase activity, preferably of the ynbW protein superfamily. Accordingly, in one embodiment, the process of the present invention comprises the use of a gene product with an activity of the putative monooxygenase with luciferase-like ATPase activity from E. coli or its homolog, e.g. as shown herein, for the production of the respective fine chemical, meaning of citrulline and/or serine, in particular for increasing the amount of citrulline, in particular for increasing the amount of serine, in particular for increasing the amount of citrulline and serine, preferably of citrulline and/or serine in free or bound form in an organism or a part thereof, as mentioned.
The sequence of b3169 from Escherichia coli K12 has been published in Blattner et al., Science 277 (5331), 1453-1474, 1997, and its activity is being defined as a transcription termination-antitermination factor, preferably of the Escherichia coli transcription factor nusA superfamily. Accordingly, in one embodiment, the process of the present invention comprises the use of a gene product with an activity of a transcription termination-antitermination factor from E. coli or its homolog, e.g. as shown herein, for the production of the respective fine chemical, meaning of aspartic acid, in particular for increasing the amount of aspartic acid, preferably aspartic acid in free or bound form in an organism or a part thereof, as mentioned.
The sequence of b3172 from Escherichia coli K12 has been published in Blattner et al., Science 277(5331), 1453-1474, 1997, and its activity has been defined as a argininosuccinate synthetase, preferably of the argininosuccinate synthase superfamily. Accordingly, in one embodiment, the process of the present invention comprises the use of a gene product with an activity of the argininosuccinate synthetase from E. coli or its homolog, e.g. as shown herein, for the production of the respective fine chemical, meaning of 5-oxoproline and/or aspartic acid, in particular for increasing the amount of 5-oxoproline, in particular for increasing the amount of aspartic acid, in particular for increasing the amount of 5-oxoproline and aspartic acid, preferably of 5-oxoproline and/or aspartic acid in free or bound form in an organism or a part thereof, as mentioned.
The sequence of b3231 from Escherichia coli K12 has been published in Blattner et al., Science 277(5331), 1453-1474, 1997, and its activity has been defined as a 50S ribosomal subunit protein L13, preferably of the Escherichia coli ribosomal protein L13 superfamily. Accordingly, in one embodiment, the process of the present invention comprises the use of a gene product with an activity of the 50S ribosomal subunit protein L13 from E. coli or its homolog, e.g. as shown herein, for the production of the respective fine chemical, meaning of alanine and/or serine, in particular for increasing the amount of alanine, in particular for increasing the amount of serine, in particular for increasing the amount of alanine and serine, preferably of alanine and/or serine in free or bound form in an organism or a part thereof, as mentioned.
The sequence of b3241 from Escherichia coli K12 has been published in Blattner et al., Science 277 (5331), 1453-1474, 1997, and its activity is being defined as a putative membrane located multidrug resistance protein. Accordingly, in one embodiment, the process of the present invention comprises the use of a gene product with an activity of a putative membrane located multidrug resistance protein from E. coli or its homolog, e.g. as shown herein, for the production of the respective fine chemical, meaning of citrulline, in particular for increasing the amount of citrulline, preferably citrulline in free or bound form in an organism or a part thereof, as mentioned.
The sequence of b3767 from Escherichia coli K12 has been published in Blattner et al., Science 277(5331), 1453-1474, 1997, and its activity has been defined as a Acetolactate synthase isozyme II large subunit. Accordingly, in one embodiment, the process of the present invention comprises the use of a gene product with an activity of the Acetolactate synthase isozyme II large subunit from E. coli or its homolog, e.g. as shown herein, for the production of the respective fine chemical, meaning of alanine and/or homoserine, in particular for increasing the amount of alanine, in particular for increasing the amount of homoserine, in particular for increasing the amount of alanine and homoserine, preferably of alanine and/or homoserine in free or bound form in an organism or a part thereof, as mentioned.
The sequence of b3919 from Escherichia coli K12 has been published in Blattner et al., Science 277 (5331), 1453-1474, 1997, and its activity is being defined as a triosephosphate isomerase, preferably of the triose-phosphate isomerase superfamily. Accordingly, in one embodiment, the process of the present invention comprises the use of a gene product with an activity of a triosephosphate isomerase from E. coli or its homolog, e.g. as shown herein, for the production of the respective fine chemical, meaning of phenylalanine, in particular for increasing the amount of phenylalanine, preferably phenylalanine in free or bound form in an organism or a part thereof, as mentioned.
The sequence of b3926 from Escherichia coli K12 has been published in Blattner et al., Science 277 (5331), 1453-1474, 1997, and its activity is being defined as a glycerol kinase, preferably of the xylulokinase superfamily. Accordingly, in one embodiment, the process of the present invention comprises the use of a gene product with an activity of a glycerol kinase from E. coli or its homolog, e.g. as shown herein, for the production of the respective fine chemical, meaning of citrulline, in particular for increasing the amount of citrulline, preferably citrulline in free or bound form in an organism or a part thereof, as mentioned.
The sequence of b3938 from Escherichia coli K12 has been published in Blattner et al., Science 277 (5331), 1453-1474, 1997, and its activity is being defined as a transcriptional repressor for methionine biosynthesis, preferably of the metJ protein superfamily. Accordingly, in one embodiment, the process of the present invention comprises the use of a gene product with an activity of a transcriptional repressor for methionine biosynthesis from E. coli or its homolog, e.g. as shown herein, for the production of the respective fine chemical, meaning of phenylalanine, in particular for increasing the amount of phenylalanine, preferably phenylalanine in free or bound form in an organism or a part thereof, as mentioned.
The sequence of b3983 from Escherichia coli K12 has been published in Blattner et al., Science 277(5331), 1453-1474, 1997, and its activity has been defined as a 50S ribosomal subunit protein L12, preferably of the Escherichia coli ribosomal protein L11 superfamily. Accordingly, in one embodiment, the process of the present invention comprises the use of a gene product with an activity of the 50S ribosomal subunit protein L12 from E. coli or its homolog, e.g. as shown herein, for the production of the respective fine chemical, meaning of phenylalanine and/or tyrosine, in particular for increasing the amount of phenylalanine, in particular for increasing the amount of tyrosine, in particular for increasing the amount of phenylalanine and tyrosine, preferably of phenylalanine and/or tyrosine in free or bound form in an organism or a part thereof, as mentioned.
The sequence of b4129 from Escherichia coli K12 has been published in Blattner et al., Science 277 (5331), 1453-1474, 1997, and its activity is being defined as a lysine tRNA synthetase, inducible, preferably of the lysine-tRNA ligase superfamily. Accordingly, in one embodiment, the process of the present invention comprises the use of a gene product with an activity of a lysine tRNA synthetase, inducible from E. coli or its homolog, e.g. as shown herein, for the production of the respective fine chemical, meaning of aspartic acid, in particular for increasing the amount of aspartic acid, preferably aspartic acid in free or bound form in an organism or a part thereof, as mentioned.
The sequence of b4214 from Escherichia coli K12 has been published in Blattner et al., Science 277 (5331), 1453-1474, 1997, and its activity is being defined as a ammonium transport system structural protein, preferably of the Aquifex aeolicus cysQ protein superfamily. Accordingly, in one embodiment, the process of the present invention comprises the use of a gene product with an activity of a ammonium transport system structural protein from E. coli or its homolog, e.g. as shown herein, for the production of the respective fine chemical, meaning of citrulline, in particular for increasing the amount of citrulline, preferably citrulline in free or bound form in an organism or a part thereof, as mentioned.
The sequence of b4269 from Escherichia coli K12 has been published in Blattner et al., Science 277 (5331), 1453-1474, 1997, and its activity is being defined as a putative alcohol dehydrogenase with NAD(P)-binding and GroES domains. Accordingly, in one embodiment, the process of the present invention comprises the use of a gene product with an activity of a putative alcohol dehydrogenase with NAD(P)-binding and GroES domains from E. coli or its homolog, e.g. as shown herein, for the production of the respective fine chemical, meaning of citrulline, in particular for increasing the amount of citrulline, preferably citrulline in free or bound form in an organism or a part thereof, as mentioned.
The sequence of b4346 from Escherichia coli K12 has been published in Blattner et al., Science 277 (5331), 1453-1474, 1997, and its activity is being defined as a component of 5-methylcytosine-specific restriction enzyme McrBC, preferably of the 5-methylcytosine-specific restriction enzyme B superfamily. Accordingly, in one embodiment, the process of the present invention comprises the use of a gene product with an activity of a component of 5-methylcytosine-specific restriction enzyme McrBC from E. coli or its homolog, e.g. as shown herein, for the production of the respective fine chemical, meaning of aspartic acid, in particular for increasing the amount of aspartic acid, preferably aspartic acid in free or bound form in an organism or a part thereof, as mentioned.

**[0023.0.14.14]**Homologues ( = homologs) of the present gene products can be derived from any organisms as long as the homologue confers the herein mentioned activity, in particular, confers an increase in the respective fine chemical amount or content. In one embodiment, the homolog of the any one of the polypeptides indicated in Table II, column 3, line 126 is a homolog having the same or a similar acitivity. In particular an increase of activity confers an increase in the content of the respective fine chemical in the organisms, preferably of 5-oxoproline. In one embodiment, the homolog is a homolog with a sequence as indicated in Table I or II, column 7, line 126. In one embodiment, the homolog of one of the polypeptides indicated in Table II, column 3, line 126, is derived from an eukaryotic. In one embodiment, the homolog is derived from Fungi. In one embodiment, the homolog of a polypeptide indicated in Table II, column 3, line 126, is derived from Ascomyceta. In one embodiment, the homolog of a polypeptide indicated in Table II, column 3, line 126, is derived from Saccharomycotina. In one embodiment, the homolog of a polypeptide indicated in Table II, column 3, line 126, is derived from Saccharomycetes. In one embodiment, the homolog of a polypeptide indicated in Table II, column 3, line 126, is a homolog being derived from Saccharomycetales. In one embodiment, the homolog of a polypeptide indicated in Table II, column 3, line 126, is a homolog having the same or a similar acitivity being derived from Saccharomycetaceae. In one embodiment, the homolog of a polypeptide indicated in Table II, column 3, line 126, is a homolog having the same or a similar acitivity being derived from Saccharomycetes.
In one embodiment, the homolog of the any one of the polypeptides indicated in Table II, column 3, lines 128, 129, 130 or 131 resp., is a homolog having the same or a similar acitivity. In particular an increase of activity confers an increase in the content of the respective fine chemical in the organisms, preferably of alanine. In one embodiment, the homolog is a homolog with a sequence as indicated in Table I or II, column 7, lines 128, 129, 130 or 131 resp.. In one embodiment, the homolog of one of the polypeptides indicated in Table II, column 3, lines 128, 129, 130 or 131 resp., is derived from an eukaryotic. In one embodiment, the homolog is derived from Fungi. In one embodiment, the homolog of a polypeptide indicated in Table II, column 3, lines 128, 129, 130 or 131 resp., is derived from Ascomyceta. In one embodiment, the homolog of a polypeptide indicated in Table II, column 3, lines 128, 129, 130 or 131 resp., is derived from Saccharomycotina. In one embodiment, the homolog of a polypeptide indicated in Table II, column 3, lines 128, 129, 130 or 131 resp., is derived from Saccharomycetes. In one embodiment, the homolog of a polypeptide indicated in Table II, column 3, lines 128, 129, 130 or 131 resp., is a homolog being derived from Saccharomycetales. In one embodiment, the homolog of a polypeptide indicated in Table II, column 3, lines 128, 129, 130 or 131 resp., is a homolog having the same or a similar acitivity being derived from Saccharomycetaceae. In one embodiment, the homolog of a polypeptide indicated in Table II, column 3, lines 128, 129, 130 or 131 resp., is a homolog having the same or a similar acitivity being derived from Saccharomycetes.
In one embodiment, the homolog of the any one of the polypeptides indicated in Table II, column 3, lines 134, 135 or 136 resp., is a homolog having the same or a similar acitivity. In particular an increase of activity confers an increase in the content of the respective fine chemical in the organisms, preferably of aspartic acid. In one embodiment, the homolog is a homolog with a sequnence as indicated in Table I or II, column 7, lines 134, 135 or 136 resp.. In one embodiment, the homolog of one of the polypeptides indicated in Table II, column 3, lines 134, 135 or 136 resp., is derived from an eukaryotic. In one embodiment, the homolog is derived from Fungi. In one embodiment, the homolog of a polypeptide indicated in Table II, column 3, lines 134, 135 or 136 resp., is derived from Ascomyceta. In one embodiment, the homolog of a polypeptide indicated in Table II, column 3, lines 134, 135 or 136 resp., is derived from Saccharomycotina. In one embodiment, the homolog of a polypeptide indicated in Table II, column 3, lines 134, 135 or 136 resp., is derived from Saccharomycetes. In one embodiment, the homolog of a polypeptide indicated in Table II, column 3, lines 134, 135 or 136 resp., is a homolog being derived from Saccharomycetales. In one embodiment, the homolog of a polypeptide indicated in Table II, column 3, lines 134, 135 or 136 resp., is a homolog having the same or a similar acitivity being derived from Saccharomycetaceae. In one embodiment, the homolog of a polypeptide indicated in Table II, column 3, lines 134, 135 or 136 resp., is a homolog having the same or a similar acitivity being derived from Saccharomycetes.
In one embodiment, the homolog of the any one of the polypeptides indicated in Table II, column 3, lines 139 or 140 resp., is a homolog having the same or a similar acitivity. In particular an increase of activity confers an increase in the content of the respective fine chemical in the organisms, preferably of citrulline. In one embodiment, the homolog is a homolog with a sequence as indicated in Table I or II, column 7, lines 139 or 140 resp.. In one embodiment, the homolog of one of the polypeptides indicated in Table II, column 3, lines 139 or 140 resp., is derived from an eukaryotic. In one embodiment, the homolog is derived from Fungi. In one embodiment, the homolog of a polypeptide indicated in Table II, column 3, lines 139 or 140 resp., is derived from Ascomyceta. In one embodiment, the homolog of a polypeptide indicated in Table II, column 3, lines 139 or 140 resp., is derived from Saccharomycotina. In one embodiment, the homolog of a polypeptide indicated in Table II, column 3, lines 139 or 140 resp., is derived from Saccharomycetes. In one embodiment, the homolog of a polypeptide indicated in Table II, column 3, lines 139 or 140 resp., is a homolog being derived from Saccharomycetales. In one embodiment, the homolog of a polypeptide indicated in Table II, column 3, lines 139 or 140 resp., is a homolog having the same or a similar acitivity being derived from Saccharomycetaceae. In one embodiment, the homolog of a polypeptide indicated in Table II, column 3, lines 139 or 140 resp., is a homolog having the same or a similar acitivity being derived from Saccharomycetes.
In one embodiment, the homolog of the any one of the polypeptides indicated in Table II, column 3, lines 141 or 142 resp., is a homolog having the same or a similar acitivity.
In particular an increase of activity confers an increase in the content of the respective fine chemical in the organisms, preferably of glycine. In one embodiment, the homolog is a homolog with a sequence as indicated in Table I or II, column 7, lines 141 or 142 resp.. In one embodiment, the homolog of one of the polypeptides indicated in Table II, column 3, lines 141 or 142 resp., is derived from an eukaryotic. In one embodiment, the homolog is derived from Fungi. In one embodiment, the homolog of a polypeptide indicated in Table II, column 3, lines 141 or 142 resp., is derived from Ascomyceta. In one embodiment, the homolog of a polypeptide indicated in Table II, column 3, lines 141 or 142 resp., is derived from Saccharomycotina. In one embodiment, the homolog of a polypeptide indicated in Table II, column 3, lines 141 or 142 resp., is derived from Saccharomycetes. In one embodiment, the homolog of a polypeptide indicated in Table II, column 3, lines 141 or 142 resp., is a homolog being derived from Saccharomycetales. In one embodiment, the homolog of a polypeptide indicated in Table II, column 3, lines 141 or 142 resp., is a homolog having the same or a similar acitivity being derived from Saccharomycetaceae. In one embodiment, the homolog of a polypeptide indicated in Table II, column 3, lines 141 or 142 resp., is a homolog having the same or a similar acitivity being derived from Saccharomycetes.
In one embodiment, the homolog of the any one of the polypeptides indicated in Table II, column 3, line 143, is a homolog having the same or a similar acitivity. In particular an increase of activity confers an increase in the content of the respective fine chemical in the organisms, preferably of homoserine. In one embodiment, the homolog is a homolog with a sequence as indicated in Table I or II, column 7, line 143. In one embodiment, the homolog of one of the polypeptides indicated in Table II, column 3, line 143, is derived from an eukaryotic. In one embodiment, the homolog is derived from Fungi. In one embodiment, the homolog of a polypeptide indicated in Table II, column 3, line 143, is derived from Ascomyceta. In one embodiment, the homolog of a polypeptide indicated in Table II, column 3, line 143, is derived from Saccharomycotina. In one embodiment, the homolog of a polypeptide indicated in Table II, column 3, line 143, is derived from Saccharomycetes. In one embodiment, the homolog of a polypeptide indicated in Table II, column 3, line 143, is a homolog being derived from Saccharomycetales. In one embodiment, the homolog of a polypeptide indicated in Table II, column 3, line 143, is a homolog having the same or a similar acitivity being derived from Saccharomycetaceae. In one embodiment, the homolog of a polypeptide indicated in Table II, column 3, line 143, is a homolog having the same or a similar acitivity being derived from Saccharomycetes.
In one embodiment, the homolog of the any one of the polypeptides indicated in Table II, column 3, lines 144, 145, 146, 147, 148 or 149 resp., is a homolog having the same or a similar acitivity. In particular an increase of activity confers an increase in the content of the respective fine chemical in the organisms, preferably of phenylalanine. In one embodiment, the homolog is a homolog with a sequence as indicated in Table I or II, column 7, lines 144, 145, 146, 147, 148 or 149 resp.. In one embodiment, the homolog of one of the polypeptides indicated in Table II, column 3, lines 144, 145, 146, 147, 148 or 149 resp., is derived from an eukaryotic. In one embodiment, the homolog is derived from Fungi. In one embodiment, the homolog of a polypeptide indicated in Table II, column 3, lines 144, 145, 146, 147, 148 or 149 resp., is derived from Ascomyceta. In one embodiment, the homolog of a polypeptide indicated in Table II, column 3, lines 144, 145, 146, 147, 148 or 149 resp., is derived from Saccharomycotina. In one embodiment, the homolog of a polypeptide indicated in Table II, column 3, lines 144, 145, 146, 147, 148 or 149 resp., is derived from Saccharomycetes. In one embodiment, the homolog of a polypeptide indicated in Table II, column 3, lines 144, 145, 146, 147, 148 or 149 resp., is a homolog being derived from Saccharomycetales. In one embodiment, the homolog of a polypeptide indicated in Table II, column 3, lines 144, 145, 146, 147, 148 or 149 resp., is a homolog having the same or a similar acitivity being derived from Saccharomycetaceae. In one embodiment, the homolog of a polypeptide indicated in Table II, column 3, lines 144, 145, 146, 147, 148 or 149 resp., is a homolog having the same or a similar acitivity being derived from Saccharomycetes.
In one embodiment, the homolog of the any one of the polypeptides indicated in Table II, column 3, lines 157, 158, 159, 160, 161, 162 or 163 resp., is a homolog having the same or a similar acitivity. In particular an increase of activity confers an increase in the content of the respective fine chemical in the organisms, preferably of serine. In one embodiment, the homolog is a homolog with a sequence as indicated in Table I or II, column 7, lines 157, 158, 159, 160, 161, 162 or 163 resp.. In one embodiment, the homolog of one of the polypeptides indicated in Table II, column 3, lines 157, 158, 159, 160, 161, 162 or 163 resp., is derived from an eukaryotic. In one embodiment, the homolog is derived from Fungi. In one embodiment, the homolog of a polypeptide indicated in Table II, column 3, lines 157, 158, 159, 160, 161, 162 or 163 resp., is derived from Ascomyceta. In one embodiment, the homolog of a polypeptide indicated in Table II, column 3, lines 157, 158, 159, 160, 161, 162 or 163 resp., is derived from Saccharomycotina. In one embodiment, the homolog of a polypeptide indicated in
Table II, column 3, lines 157, 158, 159, 160, 161, 162 or 163 resp., is derived from Saccharomycetes. In one embodiment, the homolog of a polypeptide indicated in Table II, column 3, lines 157, 158, 159, 160, 161, 162 or 163 resp., is a homolog being derived from Saccharomycetales. In one embodiment, the homolog of a polypeptide indicated in Table II, column 3, lines 157, 158, 159, 160, 161, 162 or 163 resp., is a homolog having the same or a similar acitivity being derived from Saccharomycetaceae. In one embodiment, the homolog of a polypeptide indicated in Table II, column 3, lines 157, 158, 159, 160, 161, 162 or 163 resp., is a homolog having the same or a similar acitivity being derived from Saccharomycetes.
In one embodiment, the homolog of the any one of the polypeptides indicated in Table II, column 3, lines 167, 168, 169 or 170 resp., is a homolog having the same or a similar acitivity. In particular an increase of activity confers an increase in the content of the respective fine chemical in the organisms, preferably of tyrosine. In one embodiment, the homolog is a homolog with a sequence as indicated in Table I or II, column 7, lines 167, 168, 169 or 170 resp.. In one embodiment, the homolog of one of the polypeptides indicated in Table II, column 3, lines 167, 168, 169 or 170 resp., is derived from an eukaryotic. In one embodiment, the homolog is derived from Fungi. In one embodiment, the homolog of a polypeptide indicated in Table II, column 3, lines 167, 168, 169 or 170 resp., is derived from Ascomyceta. In one embodiment, the homolog of a polypeptide indicated in Table II, column 3, lines 167, 168, 169 or 170 resp., is derived from Saccharomycotina. In one embodiment, the homolog of a polypeptide indicated in Table II, column 3, lines 167, 168, 169 or 170 resp., is derived from Saccharomycetes. In one embodiment, the homolog of a polypeptide indicated in Table II, column 3, lines 167, 168, 169 or 170 resp., is a homolog being derived from Saccharomycetales. In one embodiment, the homolog of a polypeptide indicated in Table II, column 3, lines 167, 168, 169 or 170 resp., is a homolog having the same or a similar acitivity being derived from Saccharomycetaceae. In one embodiment, the homolog of a polypeptide indicated in Table II, column 3, lines 167, 168, 169 or 170 resp., is a homolog having the same or a similar acitivity being derived from Saccharomycetes.
In one embodiment, the homolog of the any one of the polypeptides indicated in Table II, column 3, lines 127 and/or 492 to 496 resp. is a homolog having the same or a similar acitivity. In particular an increase of activity confers an increase in the content of the respective fine chemical in the organisms preferably of 5-oxoproline. In one embodiment, the homolog is a homolog with a sequence as indicated in Table I or II, column 7, lines 127 and/or 492 to 496 resp. In one embodiment, the homolog of one of the polypeptides indicated in Table II, column 3, line lines 127 and/or 492 to 496 resp. is derived from an bacteria. In one embodiment, the homolog of a polypeptide indicated in Table II, column 3, lines 127 and/or 492 to 496 resp. is derived from Proteobacteria.. In one embodiment, the homolog of a polypeptide indicated in Table II, column 3, lines 127 and/or 492 to 496 resp. is a homolog having the same or a similar acitivity being derived from Gammaproteobacteria. In one embodiment, the homolog of a polypeptide indicated in Table II, column 3, lines 127 and/or 492 to 496 resp. is derived from Enterobacteriales. In one embodiment, the homolog of a polypeptide indicated in Table II, column 3, line lines 127 and/or 492 to 496 resp. is a homolog being derived from Enterobacteriaceae. In one embodiment, the homolog of a polypeptide indicated in Table II, column 3, line lines 127 and/or 492 to 496 resp. is a homolog having the same or a similar acitivity and being derived from Escherichia.
In one embodiment, the homolog of the any one of the polypeptides indicated in Table II, column 3, lines 132 to 133 and/or 497 to 504 resp. is a homolog having the same or a similar acitivity. In particular an increase of activity confers an increase in the content of the respective fine chemical in the organisms, preferably of alanine. In one embodiment, the homolog is a homolog with a sequence as indicated in Table I or II, column 7, lines 132 to 133 and/or 497 to 504 resp.. In one embodiment, the homolog of one of the polypeptides indicated in Table II, column 3, lines 132 to 133 and/or 497 to 504 resp. is derived from an bacteria. In one embodiment, the homolog of a polypeptide indicated in Table II, column 3, lines 132 to 133 and/or 497 to 504 resp. is derived from Proteobacteria. In one embodiment, the homolog of a polypeptide indicated in Table II, column 3, lines 132 to 133 and/or 497 to 504 resp. is a homolog having the same or a similar acitivity being derived from Gammaproteobacteria. In one embodiment, the homolog of a polypeptide indicated in Table II, column 3, lines 132 to 133 and/or 497 to 504 resp. is derived from Enterobacteriales. In one embodiment, the homolog of a polypeptide indicated in Table II, column 3, lines 132 to 133 and/or 497 to 504 resp. is a homolog being derived from Enterobacteriaceae. In one embodiment, the homolog of a polypeptide indicated in Table II, column 3, lines 132 to 133 and/or 497 to 504 resp. is a homolog having the same or a similar acitivity and being derived from Escherichia.
In one embodiment, the homolog of the any one of the polypeptides indicated in Table II, column 3, lines 137 to 138 and/or 505 to 516 resp. is a homolog having the same or a similar acitivity. In particular an increase of activity confers an increase in the content of the respective fine chemical in the organisms, preferably of aspartic acid. In one embodiment, the homolog is a homolog with a sequnence as indicated in Table I or II, column 7, lines 137 to 138 and/or 505 to 516 resp.. In one embodiment, the homolog of one of the polypeptides indicated in Table II, column 3, lines 137 to 138 and/or 505 to 516 resp. is derived from an bacteria. In one embodiment, the homolog of a polypeptide indicated in Table II, column 3, lines 137 to 138 and/or 505 to 516 resp. is derived from Proteobacteria. In one embodiment, the homolog of a polypeptide indicated in Table II, column 3, lines 137 to 138 and/or 505 to 516 resp. is a homolog having the same or a similar acitivity being derived from Gammaproteobacteria. In one embodiment, the homolog of a polypeptide indicated in Table II, column 3, lines 137 to 138 and/or 505 to 516 resp. is derived from Enterobacteriales. In one embodiment, the homolog of a polypeptide indicated in Table II, column 3, lines 137 to 138 and/or 505 to 516 resp. is a homolog being derived from Enterobacteriaceae. In one embodiment, the homolog of a polypeptide indicated in Table II, column 3, lines 137 to 138 and/or 505 to 516 resp. is a homolog having the same or a similar acitivity and being derived from Escherichia.
In one embodiment, the homolog of the any one of the polypeptides indicated in Table II, column 3, lines 517 to 529 resp. is a homolog having the same or a similar acitivity. In particular an increase of activity confers an increase in the content of the respective fine chemical in the organisms, preferably of citrulline. In one embodiment, the homolog is a homolog with a sequnence as indicated in Table I or II, column 7, lines 517 to 529 resp.. In one embodiment, the homolog of one of the polypeptides indicated in Table II, column 3, lines 517 to 529 resp. is derived from an bacteria. In one embodiment, the homolog of a polypeptide indicated in Table II, column 3, lines 517 to 529 resp. is derived from Proteobacteria. In one embodiment, the homolog of a polypeptide indicated in Table II, column 3, lines 517 to 529 resp. is a homolog having the same or a similar acitivity being derived from Gammaproteobacteria. In one embodiment, the homolog of a polypeptide indicated in Table II, column 3, lines 517 to 529 resp. is derived from Enterobacteriales. In one embodiment, the homolog of a polypeptide indicated in Table II, column 3, lines 517 to 529 resp. is a homolog being derived from Enterobacteriaceae. In one embodiment, the homolog of a polypeptide indicated in Table II, column 3, lines 517 to 529 resp. is a homolog having the same or a similar acitivity and being derived from Escherichia.
In one embodiment, the homolog of the any one of the polypeptides indicated in Table II, column 3, lines 530 to 535 resp. is a homolog having the same or a similar acitivity. In particular an increase of activity confers an increase in the content of the respective fine chemical in the organisms, preferably of glycine. In one embodiment, the homolog is a homolog with a sequnence as indicated in Table I or II, column 7, lines 530 to 535 resp.. In one embodiment, the homolog of one of the polypeptides indicated in Table II, column 3, lines 530 to 535 resp. is derived from an bacteria. In one embodiment, the homolog of a polypeptide indicated in Table II, column 3, lines 530 to 535 resp. is derived from Proteobacteria. In one embodiment, the homolog of a polypeptide indicated in Table II, column 3, lines 530 to 535 resp. is a homolog having the same or a similar acitivity being derived from Gammaproteobacteria. In one embodiment, the homolog of a polypeptide indicated in Table II, column 3, lines 530 to 535 resp. is derived from Enterobacteriales. In one embodiment, the homolog of a polypeptide indicated in Table II, column 3, lines 530 to 535 resp. is a homolog being derived from Enterobacteriaceae. In one embodiment, the homolog of a polypeptide indicated in Table II, column 3, lines 530 to 535 resp. is a homolog having the same or a similar acitivity and being derived from Escherichia.
In one embodiment, the homolog of the any one of the polypeptides indicated in Table II, column 3, line 536 resp. is a homolog having the same or a similar acitivity. In particular an increase of activity confers an increase in the content of the respective fine chemical in the organisms, preferably of homoserine. In one embodiment, the homolog is a homolog with a sequnence as indicated in Table I or II, column 7, line 536 resp.. In one embodiment, the homolog of one of the polypeptides indicated in Table II, column 3, line 536 resp. is derived from an bacteria. In one embodiment, the homolog of a polypeptide indicated in Table II, column 3, line 536 resp. is derived from Proteobacteria. In one embodiment, the homolog of a polypeptide indicated in Table II, column 3, line 536 resp. is a homolog having the same or a similar acitivity being derived from Gammaproteobacteria. In one embodiment, the homolog of a polypeptide indicated in Table II, column 3, line 536 resp. is derived from Enterobacteriales. In one embodiment, the homolog of a polypeptide indicated in Table II, column 3, line 536 resp. is a homolog being derived from Enterobacteriaceae. In one embodiment, the homolog of a polypeptide indicated in Table II, column 3, line 536 resp. is a homolog having the same or a similar acitivity and being derived from Escherichia.
In one embodiment, the homolog of the any one of the polypeptides indicated in Table II, column 3, lines 150 to 156 and/or 537 to 545 resp. is a homolog having the same or a similar acitivity. In particular an increase of activity confers an increase in the content of the respective fine chemical in the organisms, preferably of phenylalanine. In one embodiment, the homolog is a homolog with a sequence as indicated in Table I or II, column 7, lines 150 to 156 and/or 537 to 545 resp.. In one embodiment, the homolog of one of the polypeptides indicated in Table II, column 3, lines 150 to 156 and/or 537 to 545 resp. is derived from an bacteria. In one embodiment, the homolog of a polypeptide indicated in Table II, column 3, lines 150 to 156 and/or 537 to 545 resp. is derived from Proteobacteria. In one embodiment, the homolog of a polypeptide indicated in Table II, column 3, lines 150 to 156 and/or 537 to 545 resp. is a homolog having the same or a similar acitivity being derived from Gammaproteobacteria. In one embodiment, the homolog of a polypeptide indicated in Table II, column 3, lines 150 to 156 and/or 537 to 545 resp. is derived from Enterobacteriales. In one embodiment, the homolog of a polypeptide indicated in Table II, column 3, lines 150 to 156 and/or 537 to 545 resp. is a homolog being derived from Enterobacteriaceae. In one embodiment, the homolog of a polypeptide indicated in Table II, column 3, lines 150 to 156 and/or 537 to 545 resp. is a homolog having the same or a similar acitivity and being derived from Escherichia.
In one embodiment, the homolog of the any one of the polypeptides indicated in Table II, column 3, lines 164 to 166 and/or 546 to 554 resp. is a homolog having the same or a similar acitivity. In particular an increase of activity confers an increase in the content of the respective fine chemical in the organisms, preferably of serine. In one embodiment, the homolog is a homolog with a sequnence as indicated in Table I or II, column 7, lines 164 to 166 and/or 546 to 554 resp.. In one embodiment, the homolog of one of the polypeptides indicated in Table II, column 3, lines 164 to 166 and/or 546 to 554 resp. is derived from an bacteria. In one embodiment, the homolog of a polypeptide indicated in Table II, column 3, lines 164 to 166 and/or 546 to 554 resp. is derived from Proteobacteria. In one embodiment, the homolog of a polypeptide indicated in Table II, column 3, lines 164 to 166 and/or 546 to 554 resp., is a homolog having the same or a similar acitivity being derived from Gammaproteobacteria. In one embodiment, the homolog of a polypeptide indicated in Table II, column 3, lines 164 to 166 and/or 546 to 554 resp. is derived from Enterobacteriales. In one embodiment, the homolog of a polypeptide indicated in Table II, column 3, lines 164 to 166 and/or 546 to 554 resp. is a homolog being derived from Enterobacteriaceae. In one embodiment, the homolog of a polypeptide indicated in Table II, column 3, lines 164 to 166 and/or 546 to 554 resp. is a homolog having the same or a similar acitivity and being derived from Escherichia.
In one embodiment, the homolog of the any one of the polypeptides indicated in Table II, column 3, lines 171 to 172 and/or 555 to 557 resp. is a homolog having the same or a similar acitivity. In particular an increase of activity confers an increase in the content of the respective fine chemical in the organisms, preferably of tyrosine. In one embodiment, the homolog is a homolog with a sequnence as indicated in Table I or II, column 7, lines 171 to, 172 and/or 555 to 557 resp.. In one embodiment, the homolog of one of the polypeptides indicated in Table II, column 3, lines 171 to 172 and/or 555 to 557 resp. is derived from an bacteria. In one embodiment, the homolog of a polypeptide indicated in Table II, column 3, lines 171 to 172 and/or 555 to 557 resp. is derived from Proteobacteria. In one embodiment, the homolog of a polypeptide indicated in Table II, column 3, lines 171 to 172 and/or 555 to 557 resp., is a homolog having the same or a similar acitivity being derived from Gammaproteobacteria. In one embodiment, the homolog of a polypeptide indicated in Table II, column 3, lines 171 to 172 and/or 555 to 557 resp. is derived from Enterobacteriales. In one embodiment, the homolog of a polypeptide indicated in Table II, column 3, lines 171 to 172 and/or 555 to 557 resp. is a homolog being derived from Enterobacteriaceae. In one embodiment, the homolog of a polypeptide indicated in Table II, column 3, lines 171 to 172 and/or 555 to 557 resp. is a homolog having the same or a similar acitivity and being derived from Escherichia.

**[0023.1.14.14]**Homologs of the polypeptide indicated in Table II, column 3, lines 126 to 172 and/or lines 492 to 557, resp., may be the polypetides encoded by the nucleic acid molecules indicated in Table I, column 7, lines 126 to 172 and/or lines 492 to 557, resp., or may be the polypeptides indicated in Table II, column 7, lines 126 to 172 and/or lines 492 to 557, resp.. Homologs of the polypeptides polypeptide indicated in Table II, column 3, lines 126 to 172 and/or lines 492 to 557, resp., may be the polypetides encoded by the nucleic acid molecules indicated in Table I, column 7, lines 126 to 172 and/or lines 492 to 557, resp., or may be the polypeptides indicated in Table II, column 7, lines 126 to 172 and/or lines 492 to 557, resp..
Homologs of the polypeptides indicated in Table II, column 3, lines 126 to 127 and/or 492 to 496 resp. may be the polypetides encoded by the nucleic acid molecules indicated in Table I, column 7, lines 126 to 127 and/or 492 to 496 respectively or may be the polypeptides indicated in Table II, column 7, lines 126 to 127 and/or 492 to 496 resp., having a 5-oxoproline content- and/or amount-increasing activity.
Homologs of the polypeptides indicated in Table II, column 3, lines 128 to 133 and/or 497 to 504 resp. may be the polypetides encoded by the nucleic acid molecules indicated in Table I, column 7, lines 128 to 133 and/or 497 to 504 respectively or may be the polypeptides indicated in Table II, column 7, lines 128 to 133 and/or 497 to 504 resp., having a alanine content- and/or amount-increasing activity.
Homologs of the polypeptides indicated in Table II, column 3, lines 134 to 138 and/or 505 to 516 resp. may be the polypetides encoded by the nucleic acid molecules indicated in Table I, column 7, lines 134 to 138 and/or 505 to 516 respectively or may be the polypeptides indicated in Table II, column 7, lines 134 to 138 and/or 505 to 516 resp., having a aspartic acid content- and/or amount-increasing activity.
Homologs of the polypeptides indicated in Table II, column 3, lines 139 to 140 and/or 517 to 529 resp. may be the polypetides encoded by the nucleic acid molecules indicated in Table I, column 7, lines 139 to 140 and/or 517 to 529 respectively or may be the polypeptides indicated in Table II, column 7, lines 139 to 140 and/or 517 to 529 resp., having a citrulline content- and/or amount-increasing activity.
Homologs of the polypeptides indicated in Table II, column 3, lines 141 to 142 and/or 530 to 535 resp. may be the polypetides encoded by the nucleic acid molecules indicated in Table I, column 7, lines 141 to 142 and/or 530 to 535 respectively or may be the polypeptides indicated in Table II, column 7, lines 141 to 142 and/or 530 to 535 resp., having a glycine content- and/or amount-increasing activity.
Homologs of the polypeptides indicated in Table II, column 3, lines 143 and/or 536 resp. may be the polypetides encoded by the nucleic acid molecules indicated in Table I, column 7, lines 143 and/or 536 resp. or may be the polypeptides indicated in Table II, column 7, lines 143 and/or 536 resp., having a homoserine content- and/or amount-increasing activity.
Homologs of the polypeptides indicated in Table II, column 3, lines 144 to 156 and/or 537 to 545 resp. may be the polypetides encoded by the nucleic acid molecules indicated in Table I, column 7, lines 144 to 156 and/or 537 to 545 respectively or may be the polypeptides indicated in Table II, column 7, lines 144 to 156 and/or 537 to 545 resp., having a phenylalanine content- and/or amount-increasing activity.
Homologs of the polypeptides indicated in Table II, column 3, lines 157 to 166 and/or 546 to 554 resp. may be the polypetides encoded by the nucleic acid molecules indicated in Table I, column 7, lines 157 to 166 and/or 546 to 554 respectively or may be the polypeptides indicated in Table II, column 7, lines 157 to 166 and/or 546 to 554 resp. having a serine content- and/or amount-increasing activity.
Homologs of the polypeptides indicated in Table II, column 3, lines 167 to 172 and/or 555 to 557 resp. may be the polypetides encoded by the nucleic acid molecules indicated in Table I, column 7, lines 167 to 172 and/or 555 to 557 resp.respectively or may be the polypeptides indicated in Table II, column 7, lines 167 to 172 and/or 555 to 557 resp., having a tyrosine content- and/or amount-increasing activity.

**[0024.0.0.14] see [0024.0.0.0]**

**[0025.0.14.14]** In accordance with the invention, a protein or polypeptide has the "activity of an protein of the invention", e.g. the activity of a protein indicated in Table II, column 3, lines 126 to 127 and/or 492 to 496 for 5-oxoproline and/or lines 128 to 133 and/or 497 to 504 for alanine and/or lines 134 to 138 and/or 505 to 516 for aspartic acid and/or lines 139 to 140 and/or 517 to 529 for citrulline and/or lines 141 to 142 and/or 530 to 535 for glycine and/or lines 143 and/or 536 for homoserine and/or lines 144 to 156 and/or 537 to 545 for phenylalanine and/or lines 157 to 166 and/or 546 to 554 for serine and/or lines 167 to 172 and/or 555 to 557 for tyrosine, resp.,
if its *de novo* activity, or its increased expression directly or indirectly leads to an increased amino acid level, in particular to a increased 5-oxoproline, alanine, aspartic acid, citrulline, glycine, homoserine, phenylalanine, serine and/or tyrosine resp., in the organism or a part thereof, preferably in a cell of said organism. In a preferred embodiment, the protein or polypeptide has the above-mentioned additional activities of a protein indicated in Table II, column 3, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp..
Throughout the specification the activity or preferably the biological activity of such a protein or polypeptide or an nucleic acid molecule or sequence encoding such protein or polypeptide is identical or similar if it still has the biological or enzymatic activity of any one of the proteins indicated in Table II, column 3, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp., or which has at least 10% of the original enzymatic activity, preferably 20%, particularly preferably 30%, most particularly preferably 40% in comparison to any one of the proteins indicated in Table II, column 3, line 126 and/or 128 to 131 and/or 134 to 136 and/or lines 139 to 140 and/or lines 141 to 142 and/or line 143 and/or lines 144 to 149 and/or lines 157 to 163 and/or lines 167 to 170 resp. of Saccharomyces cerevisiae and/or any one of the proteins indicated in Table II, column 3, lines 127 and/or 492 to 496 and/or lines 132 to 133 and/or 497 to 504 and/or lines 137 to 138 and/or 505 to 516 and/or lines 517 to 529 and/or lines 530 to 535 and/or line 536 and/or lines 150 to 156 and/or 537 to 545 and/or lines 164 to 166 and/or 546 to 554 and/or lines 171 to 172 and/or 555 to 557 resp. of E. coli K12.

**[0025.1.0.14] to [0033.0.0.14]: see [0025.1.0.0] to [0033.0.0.0]**

**[0034.0.14.14]** Preferably, the reference, control or wild type differs form the subject of the present invention only in the cellular activity of the polypeptide of the invention, e.g. as result of an increase in the level of the nucleic acid molecule of the present invention or an increase of the specific activity of the polypeptide of the invention. E.g., it differs by or in the expression level or activity of an protein having the activity of a protein as indicated in Table II, column 3, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp., or being encoded by a nucleic acid molecule indicated in Table I, column 5, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp., or its homologs, e.g. as indicated in Table I, column 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp., its biochemical or genetical causes and therefore shows the increased amount of the fine chemical.

**[0035.0.0.14] to [0044.0.0.14]: see [0035.0.0.0] to [0044.0.0.0]**

**[0045.0.14.14]**In one embodiment, the activity of the Saccharomyces cerevisiae protein YAL049C or its homologs, e.g. an activity of a YAL049C protein, e.g. as indicated in Table I, columns 5 or 7, line 149, is increased conferring an increase of the respective fine chemical, preferably of the phenylalanine between 30% and 74% or more.
In one embodiment, the activity of the Saccharomyces cerevisiae protein YBL015W or its homologs, e.g. an activity of a mannose-containing glycoprotein and/or as an acetyl-CoA hydrolase, preferably of the acetyl-CoA hydrolase superfamily, e.g. as indicated in Table I, columns 5 or 7, line 131, is increased conferring an increase of the respective fine chemical, preferably of the alanine between 29% and 204% or more.
In one embodiment, the activity of the Saccharomyces cerevisiae protein YDL127W or its homologs, e.g. an activity of a Gl/S-specific cyclin PCL2 (Cyclin HCS26 homolog), e.g. as indicated in Table I, columns 5 or 7, line 126, is increased conferring an increase of the respective fine chemical, preferably of the 5-oxoproline between 40% and 127% or more.
In one embodiment, the activity of the Saccharomyces cerevisiae protein YEL045C or its homologs, e.g. an activity of a YEL045C protein, e.g. as indicated in Table I, columns 5 or 7, line 163, is increased conferring an increase of the respective fine chemical, preferably of the serine between 23% and 80% or more.
In one embodiment, the activity of the Saccharomyces cerevisiae protein YEL046C or its homologs, e.g. an activity of a low specificity L-threonine aldolase, e.g. as indicated in Table I, columns 5 or 7, line 143, is increased conferring an increase of the respective fine chemical, preferably of the homoserine between 44% and 177% or more.
In one embodiment, the activity of the Saccharomyces cerevisiae protein YER152C or its homologs, e.g. an activity similar to tyrosine aminotransferase, e.g. as indicated in Table I, columns 5 or 7, line 162, is increased conferring an increase of the respective fine chemical, preferably of the serine between 23% and 75% or more.
In one embodiment, the activity of the Saccharomyces cerevisiae protein YER173W or its homologs, e.g. an activity of a checkpoint protein, involved in the activation of the DNA damage and meiotic pachytene checkpoints, e.g. as indicated in Table I, columns 5 or 7, line 130, is increased conferring an increase of the respective fine chemical, preferably of the alanine between 21% and 132% or more. In one embodiment, the activity of the Saccharomyces cerevisiae protein YER173W or its homologs, e.g. an activity of a checkpoint protein, involved in the activation of the DNA damage and meiotic pachytene checkpoints, e.g. as indicated in Table I, columns 5 or 7, line 136, is increased conferring an increase of the respective fine chemical, preferably of the aspartic acid between 64% and 93% or more. In one embodiment, the activity of the Saccharomyces cerevisiae protein YER173W or its homologs, e.g. an activity of a checkpoint protein, involved in the activation of the DNA damage and meiotic pachytene checkpoints, e.g. as indicated in Table I, columns 5 or 7, lines 130 and/or 136, is increased conferring an increase of the respective fine chemical, preferably of alanine and aspartic acid between 21% and 132%, preferably between 64% and 132% or more. In one embodiment, the activity of the Saccharomyces cerevisiae protein YER173W or its homologs, e.g. an activity of a checkpoint protein, involved in the activation of the DNA damage and meiotic pachytene checkpoints, e.g. as indicated in Table I, columns 5 or 7, line 148, is increased conferring an increase of the respective fine chemical, preferably of the phenylalanine between 44% and 99% or more. In one embodiment, the activity of the Saccharomyces cerevisiae protein YER173W or its homologs, e.g. an activity of a checkpoint protein, involved in the activation of the DNA damage and meiotic pachytene checkpoints, e.g. as indicated in Table I, columns 5 or 7, lines 130 and/or 148, is increased conferring an increase of the respective fine chemical, preferably of alanine and phenylalanine between 21 % and 132%, preferably between 44% and 132% or more. In one embodiment, the activity of the Saccharomyces cerevisiae protein YER173W or its homologs, e.g. an activity of a checkpoint protein, involved in the activation of the DNA damage and meiotic pachytene checkpoints, e.g. as indicated in Table I, columns 5 or 7, lines 136 and/or 148, is increased conferring an increase of the respective fine chemical, preferably of aspartic acid and phenylalanine between 44% and 99%, preferably between 64% and 99% or more. In one embodiment, the activity of the Saccharomyces cerevisiae protein YER173W or its homologs, e.g. an activity of a checkpoint protein, involved in the activation of the DNA damage and meiotic pachytene checkpoints, e.g. as indicated in Table I, columns 5 or 7, lines 130 and/or 136 and/or 148, is increased conferring an increase of the respective fine chemical, preferably of alanine and aspartic acid and phenylalanine between 21% and 132%, preferably 44% and 132%, more preferably between 64% and 132% or more.
In one embodiment, the activity of the Saccharomyces cerevisiae protein YFL050C or its homologs, e.g. an activity of a di-and/or trivalent inorganic cation transporter, preferably of the magnesium and cobalt transport protein superfamily, e.g. as indicated in Table I, columns 5 or 7, line 129, is increased conferring an increase of the respective fine chemical, preferably of the alanine between 19% and 104% or more. In one embodiment, the activity of the Saccharomyces cerevisiae protein YFL050C or its homologs, e.g. an activity of a di-and/or trivalent inorganic cation transporter, preferably of the magnesium and cobalt transport protein superfamily, e.g. as indicated in Table I, columns 5 or 7, line 142, is increased conferring an increase of the respective fine chemical, preferably of the glycine between 36% and 74% or more. In one embodiment, the activity of the Saccharomyces cerevisiae protein YFL050C or its homologs, e.g. an activity of a di-and/or trivalent inorganic cation transporter, preferably of the magnesium and cobalt transport protein superfamily, e.g. as indicated in Table I, columns 5 or 7, lines 129 and/or 142, is increased conferring an increase of the respective fine chemical, preferably of alanine and glycine between 19% and 104%, preferably between 36% and 104% or more. In one embodiment, the activity of the Saccharomyces cerevisiae protein YFL050C or its homologs, e.g. an activity of a di-and/or trivalent inorganic cation transporter, preferably of the magnesium and cobalt transport protein superfamily, e.g. as indicated in Table I, columns 5 or 7, line 170, is increased conferring an increase of the respective fine chemical, preferably of the tyrosine between 42% and 56% or more. In one embodiment, the activity of the Saccharomyces cerevisiae protein YFL050C or its homologs, e.g. an activity of a di-and/or trivalent inorganic cation transporter, preferably of the magnesium and cobalt transport protein superfamily, e.g. as indicated in Table I, columns 5 or 7, lines 129 and/or 170, is increased conferring an increase of the respective fine chemical, preferably of alanine and tyrosine between 19% and 104%, preferably between 42% and 104% or more.
In one embodiment, the activity of the Saccharomyces cerevisiae protein YFL050C or its homologs, e.g. an activity of a di-and/or trivalent inorganic cation transporter, preferably of the magnesium and cobalt transport protein superfamily, involved in the activation of the DNA damage and meiotic pachytene checkpoints, e.g. as indicated in Table I, columns 5 or 7, lines 142 and/or 170, is increased conferring an increase of the respective fine chemical, preferably of glycine and tyrosine between 36% and 74%, preferably between 42% and 74% or more.
In one embodiment, the activity of the Saccharomyces cerevisiae protein YFL050C or its homologs, e.g. an activity of a di-and/or trivalent inorganic cation transporter, preferably of the magnesium and cobalt transport protein superfamily, e.g. as indicated in Table I, columns 5 or 7, lines 129 and/or 142 and/or 170, is increased conferring an increase of the respective fine chemical, preferably of alanine and glycine and tyrosine between 19% and 104%, preferably 36% and 104%, more preferably between 42% and 104% or more.
In one embodiment, the activity of the Saccharomyces cerevisiae protein YGR101W or its homologs, e.g. an activity of a rhomboid protease, e.g. as indicated in Table I, columns 5 or 7, line 147, is increased conferring an increase of the respective fine chemical, preferably of the phenylalanine between 40% and 92% or more.
In one embodiment, the activity of the Saccharomyces cerevisiae protein YGR104C or its homologs, e.g. an activity of a rhomboid protease, e.g. as indicated in Table I, columns 5 or 7, line 135, is increased conferring an increase of the respective fine chemical, preferably of the aspartic acid between 108% and 126% or more.
In one embodiment, the activity of the Saccharomyces cerevisiae protein YHR130C or its homologs, e.g. an activity of a YHR130C protein, e.g. as indicated in Table I, columns 5 or 7, line 146, is increased conferring an increase of the respective fine chemical, preferably of the phenylalanine between 77% and 77%, preferably of 77% or more.
In one embodiment, the activity of the Saccharomyces cerevisiae protein YHR130C or its homologs, e.g. an activity of a YHR130C protein, e.g. as indicated in Table I, columns 5 or 7, line 169, is increased conferring an increase of the respective fine chemical, preferably of the tyrosine between 51% and 126% or more. In one embodiment, the activity of the Saccharomyces cerevisiae protein YHR130C or its homologs, e.g. an activity of a YHR130C protein, e.g. as indicated in Table I, columns 5 or 7, lines 146 and/or 169, is increased conferring an increase of the respective fine chemical, preferably of phenylalanine and tyrosine between 51% and 126%, preferably between 77% and 126%, more preferably of 126% or more.
In one embodiment, the activity of the Saccharomyces cerevisiae protein YIL150C or its homologs, e.g. an activity of a chromatin binding protein, required for S-phase (DNA synthesis) initiation or completion, e.g. as indicated in Table I, columns 5 or 7, line 128, is increased conferring an increase of the respective fine chemical, preferably of the alanine between 47% and 328% or more. In one embodiment, the activity of the Saccharomyces cerevisiae protein YIL150C or its homologs, e.g. an activity of a chromatin binding protein, required for S-phase (DNA synthesis) initiation or completion, e.g. as indicated in Table I, columns 5 or 7, line 134, is increased conferring an increase of the respective fine chemical, preferably of the aspartic acid between 308% and 308%, preferably 308% or more. In one embodiment, the activity of the Saccharomyces cerevisiae protein YIL150C or its homologs, e.g. an activity of a chromatin binding protein, required for S-phase (DNA synthesis) initiation or completion, e.g. as indicated in Table I, columns 5 or 7, lines 128 and/or 134, is increased conferring an increase of the respective fine chemical, preferably of alanine and aspartic acid between 47% and 328%, preferably between 308% and 328% or more. In one embodiment, the activity of the Saccharomyces cerevisiae protein YIL150C or its homologs, e.g. an activity of a chromatin binding protein, required for S-phase (DNA synthesis) initiation or completion, e.g. as indicated in Table I, columns 5 or 7, line 161, is increased conferring an increase of the respective fine chemical, preferably of the serine between 30% and 322% or more. In one embodiment, the activity of the Saccharomyces cerevisiae protein YIL150C or its homologs, e.g. an activity of a chromatin binding protein, required for S-phase (DNA synthesis) initiation or completion, e.g. as indicated in Table I, columns 5 or 7, lines 128 and/or 161, is increased conferring an increase of the respective fine chemical, preferably of alanine and serine between 30% and 328%, preferably between 47% and 328% or more.
In one embodiment, the activity of the Saccharomyces cerevisiae protein YIL150C or its homologs, e.g. an activity of a chromatin binding protein, required for S-phase (DNA synthesis) initiation or completion, involved in the activation of the DNA damage and meiotic pachytene checkpoints, e.g. as indicated in Table I, columns 5 or 7, lines 134 and/or 161, is increased conferring an increase of the respective fine chemical, preferably of aspartic acid and serine between 30% and 322%, preferably between 308% and 328% or more.
In one embodiment, the activity of the Saccharomyces cerevisiae protein YIL150C or its homologs, e.g. an activity of a chromatin binding protein, required for S-phase (DNA synthesis) initiation or completion, e.g. as indicated in Table I, columns 5 or 7, lines 128 and/or 134 and/or 161, is increased conferring an increase of the respective fine chemical, preferably of alanine and aspartic acid and serine between 30% and 328%, preferably 47% and 328%, more preferably between 308% and 328% or more. In one embodiment, the activity of the Saccharomyces cerevisiae protein YIL150C or its homologs, e.g. an activity of a chromatin binding protein, required for S-phase (DNA synthesis) initiation or completion, e.g. as indicated in Table I, columns 5 or 7, line 168, is increased conferring an increase of the respective fine chemical, preferably of the tyrosine between 698% and 698%, preferably 698% or more. In one embodiment, the activity of the Saccharomyces cerevisiae protein YIL150C or its homologs, e.g. an activity of a chromatin binding protein, required for S-phase (DNA synthesis) initiation or completion, e.g. as indicated in Table I, columns 5 or 7, lines 128 and/or 168, is increased conferring an increase of the respective fine chemical, preferably of alanine and tyrosine between 47% and 698%, preferably between 698% and 698% or more. In one embodiment, the activity of the Saccharomyces cerevisiae protein YIL150C or its homologs, e.g. an activity of a chromatin binding protein, required for S-phase (DNA synthesis) initiation or completion, involved in the activation of the DNA damage and meiotic pachytene checkpoints, e.g. as indicated in Table I, columns 5 or 7, lines 134 and/or 168, is increased conferring an increase of the respective fine chemical, preferably of aspartic acid and tyrosine between 308% and 698%, preferably between 698% and 698% or more.
In one embodiment, the activity of the Saccharomyces cerevisiae protein YIL150C or its homologs, e.g. an activity of a chromatin binding protein, required for S-phase (DNA synthesis) initiation or completion, involved in the activation of the DNA damage and meiotic pachytene checkpoints, e.g. as indicated in Table I, columns 5 or 7, lines 161 and/or 168, is increased conferring an increase of the respective fine chemical, preferably of serine and tyrosine between 30% and 698%, preferably between 698% and 698% or more. In one embodiment, the activity of the Saccharomyces cerevisiae protein YIL150C or its homologs, e.g. an activity of a chromatin binding protein, required for S-phase (DNA synthesis) initiation or completion, e.g. as indicated in Table I, columns 5 or 7, lines 128 and/or 134 and/or 161, is increased conferring an increase of the respective fine chemical, preferably of alanine and aspartic acid and tyrosine between 47% and 698%, preferably 308% and 698%, more preferably between 698% and 698% or more.
In one embodiment, the activity of the Saccharomyces cerevisiae protein YIL150C or its homologs, e.g. an activity of a chromatin binding protein, required for S-phase (DNA synthesis) initiation or completion, e.g. as indicated in Table I, columns 5 or 7, lines 134 and/or 161 and/or 168, is increased conferring an increase of the respective fine chemical, preferably of aspartic acid serine and tyrosine between 30% and 698%, preferably 308% and 698%, more preferably between 698% and 698% or more. In one embodiment, the activity of the Saccharomyces cerevisiae protein YIL150C or its homologs, e.g. an activity of a chromatin binding protein, required for S-phase (DNA synthesis) initiation or completion, e.g. as indicated in Table I, columns 5 or 7, lines 128 and/or 161 and/or 168, is increased conferring an increase of the respective fine chemical, preferably of alanine and serine and tyrosine between 30% and 698%, preferably 47% and 698%, more preferably between 698% and 698% or more. In one embodiment, the activity of the Saccharomyces cerevisiae protein YIL150C or its homologs, e.g. an activity of a chromatin binding protein, required for S-phase (DNA synthesis) initiation or completion, e.g. as indicated in Table I, columns 5 or 7, lines 128 and/or 134 and/or 161 and/or 168, is increased conferring an increase of the respective fine chemical, preferably of alanine and aspartic acid and serine and tyrosine between 30% and 698%, preferably 47% and 698%, more preferably between 308% and 698%, or more.
In one embodiment, the activity of the Saccharomyces cerevisiae protein YJL072C or its homologs, e.g. an activity of a subunit of the GINS complex required for chromosomal DNA replication, preferably of the Saccharomyces cerevisiae probable membrane protein YJL072c superfamily, e.g. as indicated in Table I, columns 5 or 7, line 145, is increased conferring an increase of the respective fine chemical, preferably of the phenylalanine between 25% and 204% or more.
In one embodiment, the activity of the Saccharomyces cerevisiae protein YKR057W or its homologs, e.g. an activity of a ribosomal protein, similar to S21 ribosomal proteins, involved in ribosome biogenesis and translation, preferably of the rat ribosomal protein S21 superfamily, e.g. as indicated in Table I, columns 5 or 7, line 160, is increased conferring an increase of the respective fine chemical, preferably of the serine between 24% and 170% or more.
In one embodiment, the activity of the Saccharomyces cerevisiae protein YLL009C or its homologs, e.g. an activity of a cytochrome c oxidase copper chaperone, e.g. as indicated in Table I, columns 5 or 7, line 159, is increased conferring an increase of the respective fine chemical, preferably of the serine between 27% and 84% or more.
In one embodiment, the activity of the Saccharomyces cerevisiae protein YLL013C or its homologs, e.g. an activity as a PUF protein family, which is named for the founding members, pumilio and Fbf, e.g. as indicated in Table I, columns 5 or 7, line 140, is increased conferring an increase of the respective fine chemical, preferably of the citrulline between 30% and 44% or more.
In one embodiment, the activity of the Saccharomyces cerevisiae protein YLR082C or its homologs, e.g. an activity as a suppressor of Rad53 null lethality, e.g. as indicated in Table I, columns 5 or 7, line 158, is increased conferring an increase of the respective fine chemical, preferably of the serine between 22% and 61% or more.
In one embodiment, the activity of the Saccharomyces cerevisiae protein YOL123W or its homologs, e.g. an activity of a cleavage and polyadenylation factor CF I component involved in pre-mRNA 3'-end processing, preferably of the heterogeneous nuclear ribonucleoprotein HRP1 - yeast (Saccharomyces cerevisiae) superfamily, e.g. as indicated in Table I, columns 5 or 7, line 141 is increased conferring an increase of the respective fine chemical, preferably of glycine between 35% and 105% or more.
In one embodiment, the activity of the Saccharomyces cerevisiae protein YOR245C or its homologs, e.g. an activity of a Acyl-CoA:diacylglycerol acyltransferase, preferably of the Caenorhabditis elegans hypothetical protein K07B1.4 superfamily, e.g. as indicated in Table I, columns 5 or 7, line 139 is increased conferring an increase of the respective fine chemical, preferably of citrulline between 47% and 69% or more.
In one embodiment, the activity of the Saccharomyces cerevisiae protein YOR261C or its homologs, e.g. an activity of a proteasome regulatory particle subunit, preferably of the mov-34 protein superfamily, e.g. as indicated in Table I, columns 5 or 7, line 157 is increased conferring an increase of the respective fine chemical, preferably of serine between 28% and 40% or more.
In one embodiment, the activity of the Saccharomyces cerevisiae protein YOR350C or its homologs, e.g. an activity which is not been characterized yet, but seems to be member of the Saccharomyces cerevisiae MNE1 protein superfamily, e.g. as indicated in Table I, columns 5 or 7, line 167 is increased conferring an increase of the respective fine chemical, preferably of tyrosine between 67% and 177% or more.
In one embodiment, the activity of the Saccharomyces cerevisiae protein YPR138C or its homologs, e.g. an activity of a subunit of the NH4+ transporter, preferably of the ammonium transport protein and/or ammonium transporter nrgA superfamily, e.g. as indicated in Table I, columns 5 or 7, line 144 is increased conferring an increase of the respective fine chemical, preferably of phenylalanine between 49% and 93% or more.
In one embodiment, the activity of the Escherichia coli K12 protein b0695 or its homologs, e.g. an activity of a sensory histidine kinase in two-component regulatory system, preferably of the sensor histidine kinase homology superfamily , e.g. as indicated in Table I, columns 5 or 7, line 156, is increased conferring an increase of the respective fine chemical, preferably of the phenylalanine between 36% and 74% or more.
In one embodiment, the activity of the Escherichia coli K12 protein b0730 or its homologs, e.g. an activity of a transcriptional regulator of succinylCoA synthetase operon and/or fatty acyl response regulator, preferably of the transcription regulator GntR superfamily, e.g. as indicated in Table I, columns 5 or 7, line 138, is increased conferring an increase of the respective fine chemical, preferably of the aspartic acid between 47% and 268% or more.
In one embodiment, the activity of the Escherichia coli K12 protein b1708 or its homologs, e.g. an activity of a lipoprotein, preferably of the conserved hypothetical protein HI1314 superfamily, e.g. as indicated in Table I, columns 5 or 7, line 154, is increased conferring an increase of the respective fine chemical, preferably of the phenylalanine between 48% and 237% or more.
In one embodiment, the activity of the Escherichia coli K12 protein b1827 or its homologs, e.g. an activity of a transcriptional repressor with DNA-binding Winged helix domain (IcIR family), preferably of the acetate operon repressor superfamily, e.g. as indicated in Table I, columns 5 or 7, line 153, is increased conferring an increase of the respective fine chemical, preferably of the phenylalanine between 40% and 293% or more.
In one embodiment, the activity of the Escherichia coli K12 protein b1827 or its homologs, e.g. an activity of a transcriptional repressor with DNA-binding Winged helix domain (IcIR family), preferably of the acetate operon repressor superfamily, e.g. as indicated in Table I, columns 5 or 7, line 172, is increased conferring an increase of the respective fine chemical, preferably of the tyrosine between 40% and 228% or more. In one embodiment, the activity of the Escherichia coli K12 protein b1827 or its homologs, e.g. an activity of a transcriptional repressor with DNA-binding Winged helix domain (IcIR family), preferably of the acetate operon repressor superfamily, e.g. as indicated in Table I, columns 5 or 7, lines 153 and/or 172, is increased conferring an increase of the respective fine chemical, preferably of the phenylalanine and tyrosine between 40% and 293%, preferably between 40% and 293% or more.
In one embodiment, the activity of the Escherichia coli K12 protein b1829 or its homologs, e.g. an activity of a heat shock protein with protease activity, preferably of the heat-shock protein htpX superfamily, e.g. as indicated in Table I, columns 5 or 7, line 166, is increased conferring an increase of the respective fine chemical, preferably of the serine between 24% and 166% or more. In one embodiment, the activity of the Escherichia coli K12 protein b1829 or its homologs, e.g. an activity of a heat shock protein with protease activity, preferably of the heat-shock protein htpX superfamily, e.g. as indicated in Table I, columns 5 or 7, line 171, is increased conferring an increase of the respective fine chemical, preferably of the tyrosine between 56% and 741 % or more. In one embodiment, the activity of the Escherichia coli K12 protein b1829 or its homologs, e.g. an activity of a heat shock protein with protease activity, preferably of the heat-shock protein htpX superfamily, e.g. as indicated in Table I, columns 5 or 7, lines 166 and/or 171, is increased conferring an increase of the respective fine chemical, preferably of the serine and tyrosine between 24% and 741%, preferably between 56% and 741% or more.
In one embodiment, the activity of the Escherichia coli K12 protein b2095 or its homologs, e.g. an activity of a tagatose -6-phosphate kinase, preferably of the Escherichia probable tagatose 6-phosphate kinase gatZ superfamily, e.g. as indicated in Table I, columns 5 or 7, line 133, is increased conferring an increase of the respective fine chemical, preferably of the alanine between 22% and 33% or more.
In one embodiment, the activity of the Escherichia coli K12 protein b3008 or its homologs, e.g. an activity of a cystathionine beta-lyase, PLP-dependent (beta-cystathionase), preferably of the O-succinylhomoserine (thiol)-lyase superfamily, e.g. as indicated in Table I, columns 5 or 7, line 132, is increased conferring an increase of the respective fine chemical, preferably of the alanine between 27% and 71% or more.
In one embodiment, the activity of the Escherichia coli K12 protein b3256 or its homologs, e.g. an activity of an acetyl CoA carboxylase and/or biotin carboxylase subunit, preferably of the biotin carboxylase superfamily, e.g. as indicated in Table I, columns 5 or 7, line 151, is increased conferring an increase of the respective fine chemical, preferably of the phenylalanine between 44% and 50% or more.
In one embodiment, the activity of the Escherichia coli K12 protein b1697 or its homologs, e.g. an activity of an electron transfer flavoprotein subunit with ETFP adenine nucleotide-binding like domain, preferably of the electron transfer flavoprotein beta chain superfamily, e.g. as indicated in Table I, columns 5 or 7, line 155, is increased conferring an increase of the respective fine chemical, preferably of the phenylalanine between 30% and 188% or more.
In one embodiment, the activity of the Escherichia coli K12 protein b1886 or its homologs, e.g. an activity of a methyl-accepting chemotaxis protein II and/or aspartate sensor receptor , preferably of the methyl-accepting chemotaxis protein superfamily e.g. as indicated in Table I, columns 5 or 7, line 152, is increased conferring an increase of the respective fine chemical, preferably of the phenylalanine between 33% and 196% or more. In one embodiment, the activity of the Escherichia coli K12 protein b1886 or its homologs, e.g. an activity of a methyl-accepting chemotaxis protein II and/or aspartate sensor receptor , preferably of the methyl-accepting chemotaxis protein superfamily, e.g. as indicated in Table I, columns 5 or 7, line 165, is increased conferring an increase of the respective fine chemical, preferably of the serine between 25% and 111% or more.
In one embodiment, the activity of the Escherichia coli K12 protein b1886 or its homologs, e.g. an activity of a methyl-accepting chemotaxis protein II and/or aspartate sensor receptor , preferably of the methyl-accepting chemotaxis protein superfamily, e.g. as indicated in Table I, columns 5 or 7, lines 152 and/or 165, is increased conferring an increase of the respective fine chemical, preferably of the phenylalanine and serine between 25% and 196%, preferably between 33% and 196% or more. In one embodiment, the activity of the Escherichia coli K12 protein b1896 or its homologs, e.g. an activity of a trehalose-6-phosphate synthase, preferably of the Schizosaccharomyces pombe alpha,alpha-trehalose-phosphate synthase (UdP-forming) superfamily, e.g. as indicated in Table I, columns 5 or 7, line 127, is increased conferring an increase of the respective fine chemical, preferably of the 5-oxoproline between 34% and 150% or more.
In one embodiment, the activity of the Escherichia coli K12 protein b1896 or its homologs, e.g. an activity of a trehalose-6-phosphate synthase, preferably of the Schizosaccharomyces pombe alpha,alpha-trehalose-phosphate synthase (UdP-forming) superfamily, e.g. as indicated in Table I, columns 5 or 7, line 137, is increased conferring an increase of the respective fine chemical, preferably of the aspartic acid between 90% and 255% or more. In one embodiment, the activity of the Escherichia coli K12 protein b1896 or its homologs, e.g. an activity of a trehalose-6-phosphate synthase, preferably of the Schizosaccharomyces pombe alpha,alpha-trehalose-phosphate synthase (UdP-forming) superfamily, e.g. as indicated in Table I, columns 5 or 7, lines 127 and/or 137, is increased conferring an increase of the respective fine chemical, preferably of the 5-oxoproline and aspartic acid between 34% and 255%, preferably between 90% and 255% or more.
In one embodiment, the activity of the Escherichia coli K12 protein b3462 or its homologs, e.g. an activity of integral membrane cell division protein, preferably of the cell division protein ftsX superfamily, e.g. as indicated in Table I, columns 5 or 7, line 150, is increased conferring an increase of the respective fine chemical, preferably of the phenylalanine between 26% and 140% or more. In one embodiment, the activity of the Escherichia coli K12 protein b3462 or its homologs, e.g. an activity of integral membrane cell division protein, preferably of the cell division protein ftsX superfamily, e.g. as indicated in Table I, columns 5 or 7, line 164, is increased conferring an increase of the respective fine chemical, preferably of the serine between 26% and 64% or more. In one embodiment, the activity of the Escherichia coli K12 protein b3462 or its homologs, e.g. an activity of integral membrane cell division protein, preferably of the cell division protein ftsX superfamily, e.g. as indicated in Table I, columns 5 or 7, lines 127 and/or 137, is increased conferring an increase of the respective fine chemical, preferably of the phenylalanine and serine between 26% and 140%, preferably between 26% and 140% or more.
In case the activity of the Escherichia coli K12 protein b0057 or its homologs e.g. a protein with an activity as defined in [0022.0.14.14],e.g. as indicated in Table II, columns 5 or 7, line 517 and 530 and 546, is increased, preferably, in one embodiment the increase of the fine chemical, preferably of citrulline between 33% and 119%, preferably of glycine between 43% and 67%, preferably of serine between 32% and 61%, preferably of citrulline and serine between 32% and 119%, preferably of citrulline and glycine between 33% and 119%, preferably of glycine and serine between 32% and 67%, preferably of citrulline and glycine and serine between 32% and 119% or more is conferred.
In case the activity of the Escherichia coli K12 protein b0161 or its homologs e.g. a periplasmic serine protease e.g. as indicated in Table II, columns 5 or 7, lines 492 and 505 and 537, is increased, preferably, in one embodiment the increase of the fine chemical, preferably of 5-oxoproline between 45% and 123% or more, preferably of aspartic acid between 57% and 116% or more, preferably of phenylalanine between 37% and 668% or more, preferably of 5-oxoproline and aspartic acid between 45% and 123% or more, preferably of 5-oxoproline and phenylalanine between 37% and 668% or more, preferably of aspartic acid and phenylalanine between 37% and 668% or more, preferably of 5-oxoproline and aspartic acid and phenylalanine between 37% and 668% or more is conferred.
In case the activity of the Escherichia coli K12 protein b0236 or its homologs, as indicated in Table II, columns 5 or 7, line 497, e.g. protein with an activity as defined in [0022.0.14.14], is increased, preferably, in one embodiment the increase of the fine chemical, preferably of alanine between 22% and 41 % or more is conferred.
In case the activity of the Escherichia coli K12 protein b0376 or its homologs e.g. a beta-lactamase/D-ala carboxypeptidase, penicilling binding protein e.g. as indicated in Table II, columns 5 or 7, line 493, is increased, preferably, in one embodiment the increase of the fine chemical, preferably of 5-oxoproline between 41 % and 100% or more is conferred.
In case the activity of the Escherichia coli K12 protein b0462 or its homologs, as indicated in Table II, columns 5 or 7, line 518, e.g. protein with an activity as defined in [0022.0.14.14], is increased, preferably, in one embodiment the increase of the fine chemical, preferably of citrulline between 36% and 44% or more is conferred.
In case the activity of the Escherichia coli K12 protein b0486 or its homologs e.g. a amino-acid/amine transport protein (APC family) e.g. as indicated in Table II, columns 5 or 7, line 498 and 547, is increased, preferably, in one embodiment the increase of the fine respective chemical, preferably of alanine between 44% and 52%, preferably of serine between 27% and 49%, preferably of alanine and serine between 27% and 52% or more, is conferred.
In case the activity of the Escherichia coli K12 protein b0577 or its homologs e.g. a putative transport protein e.g. as indicated in Table II, columns 5 or 7, line 506 and531, is increased, preferably, in one embodiment the increase of the fine chemical, preferably of glycine between 36% and 50%, preferably of aspartic acid between 56% and 65%, preferably of glycine and aspartic acid between 36% and 65%, or more is conferred.
In case the activity of the Escherichia coli K12 protein b0970 or its homologs e.g. a glutamate receptor e.g. as indicated in Table II, columns 5 or 7, line 494 and 555, is increased, preferably, in one embodiment the increase of the fine respective chemical, preferably of 5-oxoproline between 37% and 203%, preferably of tyrosine between 35% and 498%, preferably of 5-oxoproline and tyrosine between 35% and 498%, or more, is conferred.
In case the activity of the Escherichia coli K12 protein b1228 or its homologs, as indicated in Table II, columns 5 or 7, line 538, e.g. protein with an activity as defined in [0022.0.14.14], is increased, preferably, in one embodiment the increase of the fine chemical, preferably of phenylalanine between 35% and 44% or more is conferred.
In case the activity of the Escherichia coli K12 protein b1275 or its homologs, as indicated in Table II, columns 5 or 7, line 519, e.g. protein with an activity as defined in [0022.0.14.14], is increased, preferably, in one embodiment the increase of the fine chemical, preferably of citrulline between 32% and 50% or more is conferred.
In one embodiment, in case the activity of the Escherichia coli K12 protein b1343 or its homologs, e.g. as indicated in Table II, columns 5 or 7, line 495 and 499 and 507, is increased, e.g. the activity of a protein involved in rRNA processing and/or translation is increased, preferred the activity of a ATP-dependent RNA helicase, stimulated by 23S rRNA or its homolog is increased. Preferably, an increase of the respective fine chemical, preferably of 5-oxoproline between 37% and 87%, preferably of alanine between 27% and 44%, preferably of aspartic acid between 76% and 136%, preferably of alanine and 5-oxoproline between 27% and 87%, preferably of alanine and aspartic acid between 27% and 136%, preferably of 5-oxoproline and aspartic acid between 37% and 136%, preferably of alanine and 5-oxoproline and aspartic acid between 27% and 136%, or more is conferred.
In one embodiment, in case the activity of the Escherichia coli K12 protein b1360 or a protein with the activity defined as putative DNA replication protein or its homologs, e.g. transcriptional regulator or its homologs, e.g. as indicated in Table II, columns 5 or 7, line 520, is increased, preferably, in one embodiment an increase of the fine chemical, preferably of citrulline between 34% and 72% or more is conferred.
In case the activity of the Escherichia coli K12 protein b1863 or its homologs, as indicated in Table II, columns 5 or 7, line 500, e.g. protein with an activity as defined in [0022.0.14.14], is increased, preferably, in one embodiment the increase of the fine chemical, preferably of alanine between 22% and 45%, or more is conferred.
In case the activity of the Escherichia coli K12 protein b2023 or its homologs, as indicated in Table II, columns 5 or 7, line 508, e.g. protein with an activity as defined in [0022.0.14.14], is increased, preferably, in one embodiment the increase of the fine chemical, preferably of aspartic acid between 56% and 81%, or more is conferred.
In case the activity of the Escherichia coli K12 protein b2078 or its homologs, as indicated in Table II, columns 5 or 7, line 539, e.g. protein with an activity as defined in [0022.0.14.14], is increased, preferably, in one embodiment the increase of the fine chemical, preferably of phenylalanine between 26% and 89%, or more is conferred.
In case the activity of the Escherichia coli K12 protein b2239 or its homologs, as indicated in Table II, columns 5 or 7, line 521, e.g. protein with an activity as defined in [0022.0.14.14], is increased, preferably, in one embodiment the increase of the fine chemical, preferably of citrulline between 37% and 73%, or more is conferred.
In one embodiment, in case the activity of the Escherichia coli K12 protein b2414 or its homologs, e.g. as indicated in Table II, columns 5 or 7, line 522 and 532 and 548 and 540, is increased, e.g. the activity of a protein of the threonine dehydratase-superfamily is increased preferably the activity of a protein involved in amino acid biosynthesis, biosynthesis of the cysteine-aromatic group, degradation of amino acids of the cysteine-aromatic group, nitrogen and sulfur utilizationbiosynthesis of the aspartate family, degradation of amino acids of the aspartate group, biosynthesis of sulfuric acid and L-cysteine derivatives, biosynthesis of secondary products derived from primary amino acids, biosynthesis of secondary products derived from glycine, L-serine and L-alanine, pyridoxal phosphate binding is increased, preferred the activity of a subunit of cysteine synthase A and O-acetylserine sulfhydrolase A, PLP-dependent enzyme or its homolog is increased. Preferably, an increase of the respective fine chemical, preferably of citrulline between 31% and 40%, preferably of glycine between 38% and 62%, preferably of serine between 27% and 53%, preferably of phenylalanine between 27% and 197%, preferably of citrulline and glycine between 31% and 62%, preferably of citrulline and serine between 27% and 53%, preferably of citrulline and phenylalanine between 27% and 197%, preferably of glycine and serine between 27% and 62%, preferably of glycine and phenylalanine between 27% and 197%, preferably of serine and phenylalanine between 27% and 197%, preferably of citrulline and glycine and serine between 27% and 62%, preferably of citrulline and glycine and phenylalanine between 27% and 197%, preferably of citrulline and serine and phenylalanine between 27% and 197%, preferably of glycine and serine and phenylalanine between 27% and 197%, preferably of citrulline and glycine and serine and phenylalanine between 27% and 197%, or more is conferred.
In one embodiment, in case the activity of the Escherichia coli K12 protein b2426 or its homologs, e.g. as indicated in Table II, columns 5 or 7, line 523, is increased, e.g. the activity of a oxidoreductase with NAD(P)-binding domain is increased. Preferably, an increase of the respective fine chemical, preferably of citrulline between 32% and 41 % or more is conferred.
In one embodiment, in case the activity of the Escherichia coli K12 protein b2489 or its homologs, e.g. as indicated in Table II, columns 5 or 7, line 524, 533, 549 and 501, is increased, e.g. the activity of a hydrogenase Fe-S subunit is increased. Preferably, an increase of the respective fine chemical, preferably of citrulline between 33% and 60%, preferably of glycine between 33% and 78%, preferably of serine between 23% and 47%, preferably of alanine between 21% and 27%, preferably of citrulline and glycine between 33% and 78%, preferably of citrulline and serine between 23% and 60%, preferably of citrulline and alanine between 21% and 60%, preferably of glycine and serine between 23% and 78%, preferably of glycine and alanine between 21% and 78%, preferably of serine and alanine between 21% and 47%, preferably of citrulline and glycine and serine between 23% and 78%, preferably of citrulline and glycine and alanine between 21 % and 78%, preferably of citrulline and serine and alanine between 21% and 60%, preferably of glycine and serine and alanine between 21 % and 78%, preferably of citrulline and glycine and serine and alanine between 21% and 78%, or more is conferred.
In case the activity of the Escherichia coli K12 protein b2491 or its homologs, as indicated in Table II, columns 5 or 7, line 556, e.g. protein with an activity as defined in [0022.0.14.14], is increased, preferably, in one embodiment the increase of the fine chemical, preferably of tyrosine between 40% and 89%, or more is conferred.
In case the activity of the Escherichia coli K12 protein b2507 or its homologs, as indicated in Table II, columns 5 or 7, line 509, e.g. protein with an activity as defined in [0022.0.14.14], is increased, preferably, in one embodiment the increase of the fine chemical, preferably of aspartic acid between 49% and 120%, or more is conferred.
In one embodiment, in case the activity of the Escherichia coli K12 protein b2553 or its homologs, e.g. as indicated in Table II, columns 5 or 7, line 534, is increased, e.g. the activity of a regulatory protein P-II for glutamine synthetase is increased. Preferably, an increase of the respective fine chemical, preferably of glycine between 36% and 83% or more is conferred.
In one embodiment, in case the activity of the Escherichia coli K12 protein b2576 or its homologs, e.g. as indicated in Table III, columns 5 or 7, line 502 and 535 and 510 and 541, is increased, e.g. the activity of a protein with an activity as defined in [0022.0.14.14] is increased, an increase of the respective fine chemical, preferably of alanine between 24% and 49%, preferably of glycine between 40% and 73%, preferably of aspartic acid between 52% and 103%, preferably of phenylalanine between 23% and 41%, preferably of alanine and glycine between 24% and 73%, preferably of alanine and aspartic acid between 24% and 103%, preferably of alanine and phenylalanine between 23% and 49%, preferably of glycine and aspartic acid between 40% and 103%, preferably of glycine and phenylalanine between 23% and 73%, preferably of aspartic acid and phenylalanine between 23% and 103%, preferably of alanine and glycine and aspartic acid between 24% and 103%, preferably of alanine and glycine and phenylalanine between 23% and 73%, preferably of alanine and aspartic acid and phenylalanine between 23% and 103%, preferably of glycine and aspartic acid and phenylalanine between 23% and 103%, preferably of alanine and glycine and aspartic acid and phenylalanine between 23% and 103%, or more is conferred.
In one embodiment, in case the activity of the Escherichia coli K12 protein b2664 or a protein with the activity defined as putative transcriptional repressor with DNA-binding Winged helix domain (GntR familiy) or its homologs, e.g. transcriptional regulator, e.g. as indicated in Table III, columns 5 or 7, line 550, is increased, preferably, in one embodiment an increase of the fine chemical serine between 25% and 231 % or more is conferred.
In case the activity of the Escherichia coli K12 protein b2753 or its homologs, as indicated in Table II, columns 5 or 7, line 511, e.g. protein with an activity as defined in [0022.0.14.14], is increased, preferably, in one embodiment the increase of the fine chemical, preferably of aspartic acid between 56% and 154%, or more is conferred.
In case the activity of the Escherichia coli K12 protein b2796 or its homologs, as indicated in Table II, columns 5 or 7, line 542, e.g. protein with an activity as defined in [0022.0.14.14], is increased, preferably, in one embodiment the increase of the fine chemical, preferably of phenylalanine between 33% and 105%, or more is conferred.
In case the activity of the Escherichia coli K12 protein b3064 or its homologs, as indicated in Table II, columns 5 or 7, line 551, e.g. protein with an activity as defined in [0022.0.14.14], is increased, preferably, in one embodiment the increase of the fine chemical, preferably of serine between 39% and 84%, or more is conferred.
In case the activity of the Escherichia coli K12 protein b3116 or its homologs, e.g. as indicated in Table II, columns 5 or 7, line 512 and 552, is increased, e.g. the activity of a L-threonine/L-serine permease, anaerobically inducible (HAAAP family) is increased, preferably, an increase of the respective fine chemical, preferably of serine between 35% and 70%, preferably of aspartic acid between 50% and 130%, preferably of serine and aspartic acid between 35% and 130%,or more is conferred.
In case the activity of the Escherichia coli K12 protein b3160 or its homologs, e.g. as indicated in Table II, columns 5 or 7, line 525 and 553, is increased, e.g. the activity of a monooxygenase with luciferase-like ATPase activity is increased, preferably, an increase of the respective fine chemical, preferably of citrulline between 34% and 48%, preferably of serine between 31 % and 60%, preferably of citrulline and serine between 31 % and 60% or more is conferred.
In case the activity of the Escherichia coli K12 protein b3169 or its homologs e.g. a transcription termination-antitermination factor e.g. as indicated in Table II, columns 5 or 7, line 513, is increased, preferably, in one embodiment the increase of the fine chemical, preferably of aspartic acid between 66% and 168% or more is conferred.
In case the activity of the Escherichia coli K12 protein b3172 or its homologs, as indicated in Table II, columns 5 or 7, line 496 and 514, e.g. protein with an activity as defined in [0022.0.14.14], is increased, preferably, in one embodiment the increase of the fine chemical, preferably of aspartic acid between 52% and 234%, preferably of 5-oxoproline between 44% and 94%, preferably of aspartic acid and 5-oxoproline between 44% and 234%,or more is conferred.
In case the activity of the Escherichia coli K12 protein b3231 or its homologs e.g. a 50S ribosomal subunit protein L13 e.g. as indicated in Table II, columns 5 or 7, line 503 and 554, is increased, preferably, in one embodiment the increase of the fine chemical, preferably of alanine between 21 % and 35%, preferably of serine between 23% and 49%, preferably of alanine and serine between 21 % and 49% or more is conferred.
In case the activity of the Escherichia coli K12 protein b3241 or its homologs, as indicated in Table II, columns 5 or 7, line 526, e.g. protein with an activity as defined in [0022.0.14.14], is increased, preferably, in one embodiment the increase of the fine chemical, preferably of citrulline between 38% and 70%, or more is conferred.
In case the activity of the Escherichia coli K12 protein b3767 or its homologs, as indicated in Table II, columns 5 or 7, line 504 and 536, e.g. protein with an activity as defined in [0022.0.14.14], is increased, preferably, in one embodiment the increase of the fine chemical, preferably of alanine between 24% and 70%, preferably of homoserine between 24% and 71 %, preferably of alanine and homoserine between 24% and 71%, or more is conferred.
In case the activity of the Escherichia coli K12 protein b3919 or its homologs e.g. an triosephosphate isomerase e.g. as indicated in Table II, columns 5 or 7, line 543, is increased, preferably, in one embodiment the increase of the fine chemical, preferably of phenylalanine between 30% and 188% or more is conferred.
In case the activity of the Escherichia coli K12 protein b3926 or its homologs, as indicated in Table II, columns 5 or 7, line 527, e.g. protein with an activity as defined in [0022.0.14.14], is increased, preferably, in one embodiment the increase of the fine chemical, preferably of citrulline between 70% and 94%, or more is conferred.
In case the activity of the Escherichia coli K12 protein b3938 or its homologs, as indicated in Table II, columns 5 or 7, line 544, e.g. protein with an activity as defined in [0022.0.14.14], is increased, preferably, in one embodiment the increase of the fine chemical, preferably of phenylalanine between 34% and 40%, or more is conferred.
In case the activity of the Escherichia coli K12 protein b3983 or its homologs, e.g. the activity of a Escherichia coli ribosomal protein L11 superfamily, preferably a protein with with a t50S ribosomal subunit protein L12 activity, e.g. as indicated in Table II, columns 5 or 7, line 545 and 557, is increased conferring an increase of the respective fine chemical, preferably phenylalanine between 37% and 266%, preferably tyrosine between 44% and 357%, preferably phenylalanine and tyrosine between 37% and 357% or more is conferred.
In case the activity of the Escherichia coli K12 protein b4129 or its homologs e.g. a lysine tRNA synthetase e.g. as indicated in Table II, columns 5 or 7, line 515, is increased, preferably, in one embodiment the increase of the fine chemical, preferably of aspartic acid between 53% and 141% or more is conferred.
In case the activity of the Escherichia coli K12 protein b4214 or its homologs, as indicated in Table II, columns 5 or 7, line 528, e.g. protein with an activity as defined in [0022.0.14.14], is increased, preferably, in one embodiment the increase of the fine chemical, preferably of citrulline between 32% and 102%, or more is conferred.
In case the activity of the Escherichia coli K12 protein b4269 or its homologs, as indicated in Table II, columns 5 or 7, line 529, e.g. protein with an activity as defined in [0022.0.14.14], is increased, preferably, in one embodiment the increase of the fine chemical, preferably of citrulline between 31% and 63%, or more is conferred.
In case the activity of the Escherichia coli K12 protein b4346 or its homologs e.g. a component of 5-methylcytosine-specific restriction enzyme McrBC e.g. as indicated in Table II, columns 5 or 7, line 516, is increased, preferably, in one embodiment the increase of the fine chemical, preferably of aspartic acid between 63% and 94% or more is conferred.

**[0046.0.14.14]**In one embodiment, the activity of the Saccaromyces cerevisiae protein YAL049C or its homologs, e.g. an activity of a YAL049C protein, e.g. as indicated in Table I, columns 5 or 7, line 149, confers an increase of the respective fine chemical and of further amino acids or their precursors. In one embodiment, the activity of the Saccaromyces cerevisiae protein YBL015W or its homologs, e.g. an activity of a mannose-containing glycoprotein and/or as an acetyl-CoA hydrolase, preferably of the hacetyl-CoA hydrolase superfamily, e.g. as indicated in Table I, columns 5 or 7, line 131, confers an increase of the respective fine chemical and of further amino acid activity having-compounds or their precursors. In one embodiment, the activity of the Saccaromyces cerevisiae protein YDL127W or its homologs, e.g. an activity of a Gl/S-specific cyclin PCL2 (Cyclin HCS26 homolog), e.g. as indicated in Table I, columns 5 or 7, line 126, confers an increase of the respective fine chemical and of further amino acid activity having-compounds or their precursors.
In one embodiment, the activity of the Saccaromyces cerevisiae protein YEL045C or its homologs, e.g. an activity of a YEL045C protein, e.g. as indicated in Table I, columns 5 or 7, line 163, confers an increase of the respective fine chemical and of further amino acid activity having-compounds or their precursors.
In one embodiment, the activity of the Saccaromyces cerevisiae protein YEL046C or its homologs, e.g. an activity of a low specificity L-threonine aldolase, e.g. as indicated in Table I, columns 5 or 7, line 143, confers an increase of the respective fine chemical and of further amino acid activity having-compounds or their precursors. In one embodiment, the activity of the Saccaromyces cerevisiae protein YER152C or its homologs, e.g. an activity similar to tyrosine aminotransferase, e.g. as indicated in Table I, columns 5 or 7, line 162, confers an increase of the respective fine chemical and of further amino acid activity having-compounds or their precursors. In one embodiment, the activity of the Saccaromyces cerevisiae protein YER173W or its homologs, e.g. an activity of a checkpoint protein, involved in the activation of the DNA damage and meiotic pachytene checkpoints, e.g. as indicated in Table I, columns 5 or 7, lines 130 and/or 136 and/or 148, confers an increase of the respective fine chemical and of further amino acid activity having-compounds or their precursors. In one embodiment, the activity of the Saccaromyces cerevisiae protein YFL050C or its homologs, e.g. an activity of a di-and/or trivalent inorganic cation transporter, preferably of the magnesium and cobalt transport protein superfamily, e.g. as indicated in Table I, columns 5 or 7, lines 129 and/or 142 and/or 170, confers an increase of the respective fine chemical and of further amino acid activity having-compounds or their precursors.
In one embodiment, the activity of the Saccaromyces cerevisiae protein YGR101W or its homologs, e.g. an activity of a rhomboid protease, e.g. as indicated in Table I, columns 5 or 7, line 147, confers an increase of the respective fine chemical and of further amino acid activity having-compounds or their precursors. In one embodiment, the activity of the Saccaromyces cerevisiae protein YGR104C or its homologs, e.g. an activity of a rhomboid protease, e.g. as indicated in Table I, columns 5 or 7, line 135, confers an increase of the respective fine chemical and of further amino acid activity having-compounds or their precursors. In one embodiment, the activity of the Saccaromyces cerevisiae protein YHR130C or its homologs, e.g. an activity of a YHR130C protein, e.g. as indicated in Table I, columns 5 or 7, lines 146 and/or 169, confers an increase of the respective fine chemical and of further amino acid activity having-compounds or their precursors. In one embodiment, the activity of the Saccaromyces cerevisiae protein YIL150C or its homologs, e.g. an activity of a chromatin binding protein, required for S-phase (DNA synthesis) initiation or completion, e.g. as indicated in Table I, columns 5 or 7, lines 128 and/or 134 and/or 161 and/or 168, confers an increase of the respective fine chemical and of further amino acid activity having-compounds or their precursors. In one embodiment, the activity of the Saccaromyces cerevisiae protein YJL072C or its homologs, e.g. an activity of a subu nit of the GINS complex required for chromosomal DNA replication, preferably of the Saccharomyces cerevisiae probable membrane protein YJL072c superfamily, e.g. as indicated in Table I, columns 5 or 7, line 145, confers an increase of the respective fine chemical and of further amino acid activity having-compounds or their precursors. In one embodiment, the activity of the Saccaromyces cerevisiae protein YKR057W or its homologs, e.g. an activity of a ribosomal protein, similar to S21 ribosomal proteins, involved in ribosome biogenesis and translation, preferably of the rat ribosomal protein S21 superfamily, e.g. as indicated in Table I, columns 5 or 7, line 160, confers an increase of the respective fine chemical and of further amino acid activity having-compounds or their precursors. In one embodiment, the activity of the Saccaromyces cerevisiae protein YLL009C or its homologs, e.g. an activity of a cytochrome c oxidase copper chaperone, e.g. as indicated in Table I, columns 5 or 7, line 159, confers an increase of the respective fine chemical and of further amino acid activity having-compounds or their precursors. In one embodiment, the activity of the Saccaromyces cerevisiae protein YLL013C or its homologs, e.g. an activity as a PUF protein family, which is named for the founding members, pumilio and Fbf, e.g. as indicated in Table I, columns 5 or 7, line 140, confers an increase of the respective fine chemical and of further amino acid activity having-compounds or their precursors. In one embodiment, the activity of the Saccaromyces cerevisiae protein YLR082C or its homologs, e.g. an activity as a suppressor of Rad53 null lethality, e.g. as indicated in Table I, columns 5 or 7, line 158, confers an increase of the respective fine chemical and of further amino acid activity having-compounds or their precursors. In one embodiment, the activity of the Saccaromyces cerevisiae protein YOL123W or its homologs, e.g. an activity of a cleavage and polyadenylation factor CF I component involved in pre-mRNA 3'-end processing, preferably of the heterogeneous nuclear ribonucleoprotein HRP1 - yeast (Saccharomyces cerevisiae) superfamily, e.g. as indicated in Table I, columns 5 or 7, line 141, confers an increase of the respective fine chemical and of further amino acid activity having-compounds or their precursors. In one embodiment, the activity of the Saccaromyces cerevisiae protein YOR245C or its homologs, e.g. an activity of a Acyl-CoA:diacylglycerol acyltransferase, preferably of the Caenorhabditis elegans hypothetical protein K07B1.4 superfamily, e.g. as indicated in Table I, columns 5 or 7, line 139, confers an increase of the respective fine chemical and of further amino acid activity having-compounds or their precursors. In one embodiment, the activity of the Saccaromyces cerevisiae protein YOR261C or its homologs, e.g. an activity of a proteasome regulatory particle subunit, preferably of the mov-34 protein superfamily, e.g. as indicated in Table I, columns 5 or 7, line 157, confers an increase of the respective fine chemical and of further amino acid activity having-compounds or their precursors. In one embodiment, the activity of the Saccaromyces cerevisiae protein YOR350C or its homologs, e.g. an activity which is not been characterized yet, but seems to be member of the Saccharomyces cerevisiae MNE1 protein superfamily, e.g. as indicated in Table I, columns 5 or 7, line 167, confers an increase of the respective fine chemical and of further amino acid activity having-compounds or their precursors. In one embodiment, the activity of the Saccaromyces cerevisiae protein YPR138C or its homologs, e.g. an activity of a subunit of the NH4+ transporter, preferably of the ammonium transport protein and/or ammonium transporter nrgA superfamily, e.g. as indicated in Table I, columns 5 or 7, line 144, confers an increase of the respective fine chemical and of further amino acid activity having-compounds or their precursors.
In one embodiment, the activity of the Escherichia coli K12 protein b0695 or its homologs, e.g. an activity of a sensory histidine kinase in two-component regulatory system, preferably of the sensor histidine kinase homology superfamily, e.g. as indicated in Table I, columns 5 or 7, line 156, confers an increase of the respective fine chemical and of further amino acid activity-having compounds or their precursors. In one embodiment, the activity of the Escherichia coli K12 protein b0730 or its homologs, e.g. an activity of a transcriptional regulator of succinylCoA synthetase operon and/or fatty acyl response regulator, preferably of the transcription regulator GntR superfamily, e.g. as indicated in Table I, columns 5 or 7, line 138, confers an increase of the respective fine chemical and of further amino acid activity-having compounds or their precursors. In one embodiment, the activity of the Escherichia coli K12 protein b1708 or its homologs, e.g. an activity of a lipoprotein, preferably of the conserved hypothetical protein HI1314 superfamily, e.g. as indicated in Table I, columns 5 or 7, line 154, confers an increase of the respective fine chemical and of further amino acid activity-having compounds or their precursors. In one embodiment, the activity of the Escherichia coli K12 protein b1827 or its homologs, e.g. an activity of a transcriptional repressor with DNA-binding Winged helix domain (IcIR family), preferably of the acetate operon repressor superfamily, e.g. as indicated in Table I, columns 5 or 7, lines 153 and/or 172, confers an increase of the respective fine chemical and of further amino acid activity-having compounds or their precursors.
In one embodiment, the activity of the Escherichia coli K12 protein b1829 or its homologs, e.g. an activity of a heat shock protein with protease activity, preferably of the heat-shock protein htpX superfamily, e.g. as indicated in Table I, columns 5 or 7, lines 166 and/or 171, confers an increase of the respective fine chemical and of further amino acid activity-having compounds or their precursors. In one embodiment, the activity of the Escherichia coli K12 protein b2095 or its homologs, e.g. an activity of a tagatose -6-phosphate kinase, preferably of the Escherichia probable tagatose 6-phosphate kinase gatZ superfamily, e.g. as indicated in Table I, columns 5 or 7, line 133, confers an increase of the respective fine chemical and of further amino acid activity-having compounds or their precursors. In one embodiment, the activity of the Escherichia coli K12 protein b3008 or its homologs, e.g. an activity of a cystathionine beta-lyase, PLP-dependent (beta-cystathionase), preferably of the O-succinylhomoserine (thiol)-lyase superfamily, e.g. as indicated in Table I, columns 5 or 7, line 132, confers an increase of the respective fine chemical and of further amino acid activity-having compounds or their precursors. In one embodiment, the activity of the Escherichia coli K12 protein b3256 or its homologs, e.g. an activity of an acetyl CoA carboxylase and/or biotin carboxylase subunit, preferably of the biotin carboxylase superfamily, e.g. as indicated in Table I, columns 5 or 7, line 151, confers an increase of the respective fine chemical and of further amino acid activity-having compounds or their precursors. In one embodiment, the activity of the Escherichia coli K12 protein b1697 or its homologs, e.g. an activity of an electron transfer flavoprotein subunit with ETFP adenine nucleotide-binding like domain, preferably of the electron transfer flavoprotein beta chain superfamily, e.g. as indicated in Table I, columns 5 or 7, line 155, confers an increase of the respective fine chemical and of further amino acid activity-having compounds or their precursors. In one embodiment, the activity of the Escherichia coli K12 protein b1886 or its homologs, e.g. an activity of a methyl-accepting chemotaxis protein II and/or aspartate sensor receptor , preferably of the methyl-accepting chemotaxis protein superfamily, e.g. as indicated in Table I, columns 5 or 7, lines 152 and/or 165, confers an increase of the respective fine chemical and of further amino acid activity-having compounds or their precursors. In one embodiment, the activity of the Escherichia coli K12 protein b1896 or its homologs, e.g. an activity of a trehalose-6-phosphate synthase, preferably of the Schizosaccharomyces pombe alpha,alpha-trehalose-phosphate synthase (UdP-forming) superfamily, e.g. as indicated in Table I, columns 5 or 7, lines 127 and/or 137, confers an increase of the respective fine chemical and of further amino acid activity-having compounds or their precursors. In one embodiment, the activity of the Escherichia coli K12 protein b3462 or its homologs, e.g. an activity of integral membrane cell division protein, preferably of the cell division protein ftsX superfamily, e.g. as indicated in Table I, columns 5 or 7, lines 150 and/or 164, confers an increase of the respective fine chemical and of further amino acid activity-having compounds or their precursors.
In case the activity of the Escherichia coli K12 protein b0057 or its homologs e.g. a protein with an activity as defined in [0022.0.14.14],e.g. as indicated in Table II, columns 5 or 7, line 517 and 530 and 546, is increased, preferably, in one embodiment the increase of the fine chemical, preferably of citrulline between 33% and 119%, preferably of glycine between 43% and 67%, preferably of serine between 32% and 61%, preferably of citrulline and serine between 32% and 119%, preferably of citrulline and glycine between 33% and 119%, preferably of glycine and serine between 32% and 67%, preferably of citrulline and glycine and serine between 32% and 119% and of further amino acids or their precursors or more is conferred.
In case the activity of the Escherichia coli K12 protein b0161 or its homologs e.g. a periplasmic serine protease e.g. as indicated in Table II, columns 5 or 7, lines 492 and 505 and 537, is increased, preferably, in one embodiment the increase of the fine chemical, preferably of 5-oxoproline between 45% and 123% or more, preferably of aspartic acid between 57% and 116% or more, preferably of phenylalanine between 37% and 668% or more, preferably of 5-oxoproline and aspartic acid between 45% and 123% or more, preferably of 5-oxoproline and phenylalanine between 37% and 668% or more, preferably of aspartic acid and phenylalanine between 37% and 668% or more, preferably of 5-oxoproline and aspartic acid and phenylalanine between 37% and 668% and of further amino acids or their precursors or more is conferred.
In case the activity of the Escherichia coli K12 protein b0236 or its homologs, as indicated in Table II, columns 5 or 7, line 497, e.g. protein with an activity as defined in [0022.0.14.14], is increased, preferably, in one embodiment the increase of the fine chemical, preferably of alanine between 22% and 41 % and of further amino acids or their precursors or more is conferred.
In case the activity of the Escherichia coli K12 protein b0376 or its homologs e.g. a beta-lactamase/D-ala carboxypeptidase, penicilling binding protein e.g. as indicated in Table II, columns 5 or 7, line 493, is increased, preferably, in one embodiment the increase of the fine chemical, preferably of 5-oxoproline between 41% and 100% and of further amino acids or their precursors or more is conferred.
In case the activity of the Escherichia coli K12 protein b0462 or its homologs, as indicated in Table II, columns 5 or 7, line 518, e.g. protein with an activity as defined in [0022.0.14.14], is increased, preferably, in one embodiment the increase of the fine chemical, preferably of citrulline between 36% and 44% and of further amino acids or their precursors or more is conferred.
In case the activity of the Escherichia coli K12 protein b0486 or its homologs e.g. a amino-acid/amine transport protein (APC family) e.g. as indicated in Table II, columns 5 or 7, line 498 and 547, is increased, preferably, in one embodiment the increase of the fine respective chemical, preferably of alanine between 44% and 52%, preferably of serine between 27% and 49%, preferably of alanine and serine between 27% and 52% and of further amino acids or their precursors or more, is conferred.
In case the activity of the Escherichia coli K12 protein b0577 or its homologs e.g. a putative transport protein e.g. as indicated in Table II, columns 5 or 7, line506 and531, is increased, preferably, in one embodiment the increase of the fine chemical, preferably of glycine between 36% and 50%, preferably of aspartic acid between 56% and 65%, preferably of glycine and aspartic acid between 36% and 65%, and of further amino acids or their precursors or more is conferred.
In case the activity of the Escherichia coli K12 protein b0970 or its homologs e.g. a glutamate receptor e.g. as indicated in Table II, columns 5 or 7, line 494 and 555, is increased, preferably, in one embodiment the increase of the fine respective chemical, preferably of 5-oxoproline between 37% and 203%, preferably of tyrosine between 35% and 498%, preferably of 5-oxoproline and tyrosine between 35% and 498%, and of further amino acids or their precursors or more, is conferred.
In case the activity of the Escherichia coli K12 protein b1228 or its homologs, as indicated in Table II, columns 5 or 7, line 538, e.g. protein with an activity as defined in [0022.0.14.14], is increased, preferably, in one embodiment the increase of the fine chemical, preferably of phenylalanine between 35% and 44% and of further amino acids or their precursors or more is conferred.
In case the activity of the Escherichia coli K12 protein b1275 or its homologs, as indicated in Table II, columns 5 or 7, line 519, e.g. protein with an activity as defined in [0022.0.14.14], is increased, preferably, in one embodiment the increase of the fine chemical, preferably of citrulline between 32% and 50% and of further amino acids or their precursors or more is conferred.
In one embodiment, in case the activity of the Escherichia coli K12 protein b1343 or its homologs, e.g. as indicated in Table II, columns 5 or 7, line 495 and 499 and 507, is increased, e.g. the activity of a protein involved in rRNA processing and/or translation is increased, preferred the activity of a ATP-dependent RNA helicase, stimulated by 23S rRNA or its homolog is increased. Preferably, an increase of the respective fine chemical, preferably of 5-oxoproline between 37% and 87%, preferably of alanine between 27% and 44%, preferably of aspartic acid between 76% and 136%, preferably of alanine and 5-oxoproline between 27% and 87%, preferably of alanine and aspartic acid between 27% and 136%, preferably of 5-oxoproline and aspartic acid between 37% and 136%, preferably of alanine and 5-oxoproline and aspartic acid between 27% and 136%, and of further amino acids or their precursors or more is conferred.
In one embodiment, in case the activity of the Escherichia coli K12 protein b1360 or a protein with the activity defined as putative DNA replication protein or its homologs, e.g. transcriptional regulator or its homologs, e.g. as indicated in Table II, columns 5 or 7, line 520, is increased, preferably, in one embodiment an increase of the fine chemical, preferably of citrulline between 34% and 72% and of further amino acids or their precursors or more is conferred.
In case the activity of the Escherichia coli K12 protein b1863 or its homologs, as indicated in Table II, columns 5 or 7, line 500, e.g. protein with an activity as defined in [0022.0.14.14], is increased, preferably, in one embodiment the increase of the fine chemical, preferably of alanine between 22% and 45%, and of further amino acids or their precursors or more is conferred.
In case the activity of the Escherichia coli K12 protein b2023 or its homologs, as indicated in Table II, columns 5 or 7, line 508, e.g. protein with an activity as defined in [0022.0.14.14], is increased, preferably, in one embodiment the increase of the fine chemical, preferably of aspartic acid between 56% and 81%, or more is conferred.
In case the activity of the Escherichia coli K12 protein b2078 or its homologs, as indicated in Table II, columns 5 or 7, line 539, e.g. protein with an activity as defined in [0022.0.14.14], is increased, preferably, in one embodiment the increase of the fine chemical, preferably of phenylalanine between 26% and 89%, or more is conferred.
In case the activity of the Escherichia coli K12 protein b2239 or its homologs, as indicated in Table II, columns 5 or 7, line 521, e.g. protein with an activity as defined in [0022.0.14.14], is increased, preferably, in one embodiment the increase of the fine chemical, preferably of citrulline between 37% and 73%, and of further amino acids or their precursors or more is conferred.
In one embodiment, in case the activity of the Escherichia coli K12 protein b2414 or its homologs, e.g. as indicated in Table II, columns 5 or 7, line 522 and 532 and 548 and 540, is increased, e.g. the activity of a protein of the threonine dehydratase-superfamily is increased preferably the activity of a protein involved in amino acid biosynthesis, biosynthesis of the cysteine-aromatic group, degradation of amino acids of the cysteine-aromatic group, nitrogen and sulfur utilizationbiosynthesis of the aspartate family, degradation of amino acids of the aspartate group, biosynthesis of sulfuric acid and L-cysteine derivatives, biosynthesis of secondary products derived from primary amino acids, biosynthesis of secondary products derived from glycine, L-serine and L-alanine, pyridoxal phosphate binding is increased, preferred the activity of a subunit of cysteine synthase A and O-acetylserine sulfhydrolase A, PLP-dependent enzyme or its homolog is increased. Preferably, an increase of the respective fine chemical, preferably of citrulline between 31 % and 40%, preferably of glycine between 38% and 62%, preferably of serine between 27% and 53%, preferably of phenylalanine between 27% and 197%, preferably of citrulline and glycine between 31% and 62%, preferably of citrulline and serine between 27% and 53%, preferably of citrulline and phenylalanine between 27% and 197%, preferably of glycine and serine between 27% and 62%, preferably of glycine and phenylalanine between 27% and 197%, preferably of serine and phenylalanine between 27% and 197%, preferably of citrulline and glycine and serine between 27% and 62%, preferably of citrulline and glycine and phenylalanine between 27% and 197%, preferably of citrulline and serine and phenylalanine between 27% and 197%, preferably of glycine and serine and phenylalanine between 27% and 197%, preferably of citrulline and glycine and serine and phenylalanine between 27% and 197%, or more is conferred.
In one embodiment, in case the activity of the Escherichia coli K12 protein b2426 or its homologs, e.g. as indicated in Table II, columns 5 or 7, line 523, is increased, e.g. the activity of a oxidoreductase with NAD(P)-binding domain is increased. Preferably, an increase of the respective fine chemical, preferably of citrulline between 32% and 41 % and of further amino acids or their precursors or more is conferred.
In one embodiment, in case the activity of the Escherichia coli K12 protein b2489 or its homologs, e.g. as indicated in Table II, columns 5 or 7, line 524, 533, 549 and 501, is increased, e.g. the activity of a hydrogenase Fe-S subunit is increased. Preferably, an increase of the respective fine chemical, preferably of citrulline between 33% and 60%, preferably of glycine between 33% and 78%, preferably of serine between 23% and 47%, preferably of alanine between 21 % and 27%, preferably of citrulline and glycine between 33% and 78%, preferably of citrulline and serine between 23% and 60%, preferably of citrulline and alanine between 21% and 60%, preferably of glycine and serine between 23% and 78%, preferably of glycine and alanine between 21% and 78%, preferably of serine and alanine between 21% and 47%, preferably of citrulline and glycine and serine between 23% and 78%, preferably of citrulline and glycine and alanine between 21% and 78%, preferably of citrulline and serine and alanine between 21% and 60%, preferably of glycine and serine and alanine between 21% and 78%, preferably of citrulline and glycine and serine and alanine between 21% and 78%, and of further amino acids or their precursors or more is conferred.
In case the activity of the Escherichia coli K12 protein b2491 or its homologs, as indicated in Table II, columns 5 or 7, line 556, e.g. protein with an activity as defined in [0022.0.14.14], is increased, preferably, in one embodiment the increase of the fine chemical, preferably of tyrosine between 40% and 89%, or more is conferred.
In case the activity of the Escherichia coli K12 protein b2507 or its homologs, as indicated in Table II, columns 5 or 7, line 509, e.g. protein with an activity as defined in [0022.0.14.14], is increased, preferably, in one embodiment the increase of the fine chemical, preferably of aspartic acid between 49% and 120%, or more is conferred.
In one embodiment, in case the activity of the Escherichia coli K12 protein b2553 or its homologs, e.g. as indicated in Table II, columns 5 or 7, line 534, is increased, e.g. the activity of a regulatory protein P-II for glutamine synthetase is increased. Preferably, an increase of the respective fine chemical, preferably of glycine between 36% and 83% and of further amino acids or their precursors or more is conferred.
In one embodiment, in case the activity of the Escherichia coli K12 protein b2576 or its homologs, e.g. as indicated in Table III, columns 5 or 7, line 502 and 535 and 510 and 541, is increased, e.g. the activity of a protein with an activity as defined in [0022.0.14.14] is increased, an increase of the respective fine chemical, preferably of alanine between 24% and 49%, preferably of glycine between 40% and 73%, preferably of aspartic acid between 52% and 103%, preferably of phenylalanine between 23% and 41%, preferably of alanine and glycine between 24% and 73%, preferably of alanine and aspartic acid between 24% and 103%, preferably of alanine and phenylalanine between 23% and 49%, preferably of glycine and aspartic acid between 40% and 103%, preferably of glycine and phenylalanine between 23% and 73%, preferably of aspartic acid and phenylalanine between 23% and 103%, preferably of alanine and glycine and aspartic acid between 24% and 103%, preferably of alanine and glycine and phenylalanine between 23% and 73%, preferably of alanine and aspartic acid and phenylalanine between 23% and 103%, preferably of glycine and aspartic acid and phenylalanine between 23% and 103%, preferably of alanine and glycine and aspartic acid and phenylalanine between 23% and 103%, or more is conferred.
In one embodiment, in case the activity of the Escherichia coli K12 protein b2664 or a protein with the activity defined as putative transcriptional repressor with DNA-binding Winged helix domain (GntR familiy) or its homologs, e.g. transcriptional regulator, e.g. as indicated in Table III, columns 5 or 7, line 550, is increased, preferably, in one embodiment an increase of the fine chemical serine between 25% and 231 % and of further amino acids or their precursors or more is conferred.
In case the activity of the Escherichia coli K12 protein b2753 or its homologs, as indicated in Table II, columns 5 or 7, line 511, e.g. protein with an activity as defined in [0022.0.14.14], is increased, preferably, in one embodiment the increase of the fine chemical, preferably of aspartic acid between 56% and 154%, or more is conferred.
In case the activity of the Escherichia coli K12 protein b2796 or its homologs, as indicated in Table II, columns 5 or 7, line 542, e.g. protein with an activity as defined in [0022.0.14.14], is increased, preferably, in one embodiment the increase of the fine chemical, preferably of phenylalanine between 33% and 105%, or more is conferred.
In case the activity of the Escherichia coli K12 protein b3064 or its homologs, as indicated in Table II, columns 5 or 7, line 551, e.g. protein with an activity as defined in [0022.0.14.14], is increased, preferably, in one embodiment the increase of the fine chemical, preferably of serine between 39% and 84%, and of further amino acids or their precursors or more is conferred.
In case the activity of the Escherichia coli K12 protein b3116 or its homologs, e.g. as indicated in Table II, columns 5 or 7, line 512 and 552, is increased, e.g. the activity of a L-threonine/L-serine permease, anaerobically inducible (HAAAP family) is increased, preferably, an increase of the respective fine chemical, preferably of serine between 35% and 70%, preferably of aspartic acid between 50% and 130%, preferably of serine and aspartic acid between 35% and 130%, and of further amino acids or their precursors or more is conferred.
In case the activity of the Escherichia coli K12 protein b3160 or its homologs, e.g. as indicated in Table II, columns 5 or 7, line 525 and 553, is increased, e.g. the activity of a monooxygenase with luciferase-like ATPase activity is increased, preferably, an increase of the respective fine chemical, preferably of citrulline between 34% and 48%, preferably of serine between 31 % and 60%, preferably of citrulline and serine between 31 % and 60% and of further amino acids or their precursors or more is conferred.
In case the activity of the Escherichia coli K12 protein b3169 or its homologs e.g. a transcription termination-antitermination factor e.g. as indicated in Table II, columns 5 or 7, line 513, is increased, preferably, in one embodiment the increase of the fine chemical, preferably of aspartic acid between 66% and 168% or more is conferred.
In case the activity of the Escherichia coli K12 protein b3172 or its homologs, as indicated in Table II, columns 5 or 7, line 496 and 514, e.g. protein with an activity as defined in [0022.0.14.14], is increased, preferably, in one embodiment the increase of the fine chemical, preferably of aspartic acid between 52% and 234%, preferably of 5-oxoproline between 44% and 94%, preferably of aspartic acid and 5-oxoproline between 44% and 234%, and of further amino acids or their precursors or more is conferred.
In case the activity of the Escherichia coli K12 protein b3231 or its homologs e.g. a 50S ribosomal subunit protein L13 e.g. as indicated in Table II, columns 5 or 7, line 503 and 554, is increased, preferably, in one embodiment the increase of the fine chemical, preferably of alanine between 21% and 35%, preferably of serine between 23% and 49%, preferably of alanine and serine between 21 % and 49% and of further amino acids or their precursors or more is conferred.
In case the activity of the Escherichia coli K12 protein b3241 or its homologs, as indicated in Table II, columns 5 or 7, line 526, e.g. protein with an activity as defined in [0022.0.14.14], is increased, preferably, in one embodiment the increase of the fine chemical, preferably of citrulline between 38% and 70%, and of further amino acids or their precursors or more is conferred.
In case the activity of the Escherichia coli K12 protein b3767 or its homologs, as indicated in Table II, columns 5 or 7, line 504 and 536, e.g. protein with an activity as defined in [0022.0.14.14], is increased, preferably, in one embodiment the increase of the fine chemical, preferably of alanine between 24% and 70%, preferably of homoserine between 24% and 71%, preferably of alanine and homoserine between 24% and 71 %, and of further amino acids or their precursors or more is conferred.
In case the activity of the Escherichia coli K12 protein b3919 or its homologs e.g. an triosephosphate isomerase e.g. as indicated in Table II, columns 5 or 7, line 543, is increased, preferably, in one embodiment the increase of the fine chemical, preferably of phenylalanine between 30% and 188% and of further amino acids or their precursors or more is conferred.
In case the activity of the Escherichia coli K12 protein b3926 or its homologs, as indicated in Table II, columns 5 or 7, line 527, e.g. protein with an activity as defined in [0022.0.14.14], is increased, preferably, in one embodiment the increase of the fine chemical, preferably of citrulline between 70% and 94%, and of further amino acids or their precursors or more is conferred.
In case the activity of the Escherichia coli K12 protein b3938 or its homologs, as indicated in Table II, columns 5 or 7, line 544, e.g. protein with an activity as defined in [0022.0.14.14], is increased, preferably, in one embodiment the increase of the fine chemical, preferably of phenylalanine between 34% and 40%, and of further amino acids or their precursors or more is conferred.
In case the activity of the Escherichia coli K12 protein b3983 or its homologs, e.g. the activity of a Escherichia coli ribosomal protein L11 superfamily, preferably a protein with with a t50S ribosomal subunit protein L12 activity, e.g. as indicated in Table II, columns 5 or 7, line 545 and 557, is increased conferring an increase of the respective fine chemical, preferably phenylalanine between 37% and 266%, preferably tyrosine between 44% and 357%, preferably phenylalanine and tyrosine between 37% and 357% and of further amino acids or their precursors or more is conferred.
In case the activity of the Escherichia coli K12 protein b4129 or its homologs e.g. a lysine tRNA synthetase e.g. as indicated in Table II, columns 5 or 7, line 515, is increased, preferably, in one embodiment the increase of the fine chemical, preferably of aspartic acid between 53% and 141 % and of further amino acids or their precursors or more is conferred.
In case the activity of the Escherichia coli K12 protein b4214 or its homologs, as indicated in Table II, columns 5 or 7, line 528, e.g. protein with an activity as defined in [0022.0.14.14], is increased, preferably, in one embodiment the increase of the fine chemical, preferably of citrulline between 32% and 102%, and of further amino acids or their precursors or more is conferred.
In case the activity of the Escherichia coli K12 protein b4269 or its homologs, as indicated in Table II, columns 5 or 7, line 529, e.g. protein with an activity as defined in [0022.0.14.14], is increased, preferably, in one embodiment the increase of the fine chemical, preferably of citrulline between 31 % and 63%, and of further amino acids or their precursors or more is conferred.
In case the activity of the Escherichia coli K12 protein b4346 or its homologs e.g. a component of 5-methylcytosine-specific restriction enzyme McrBC e.g. as indicated in Table II, columns 5 or 7, line 516, is increased, preferably, in one embodiment the increase of the fine chemical, preferably of aspartic acid between 63% and 94% and of further amino acids or their precursors or more is conferred.

**[0047.0.0.14] to [0048.0.0.14]: see [0047.0.0.0] to [0048.0.0.0]**

**[0049.0.14.14]**
A protein having an activity conferring an increase in the amount or level of 5-oxoproline chemical preferably has the structure of the polypeptide described herein, in particular of a polypeptides comprising a consensus sequence as indicated in Table IV, columns 7, lines 126 to 127 and/or 492 to 496 of a polypeptide as indicaded in Table II, columns 5 or 7, lines 126 to 127 and/or 492 to 496 the functional homologues thereof as described herein, or is encoded by the nucleic acid molecule characterized herein or the nucleic acid molecule according to the invention, for example by a nucleic acid molecule as indicated in Table I, columns 5 or 7, lines 126 to 127 and/or 492 to 496 or its herein described functional homologues and has the herein mentioned activity.
A protein having an activity conferring an increase in the amount or level of alanine chemical preferably has the structure of the polypeptide described herein, in particular of a polypeptides comprising a consensus sequence as indicated in Table IV, columns 7, lines 128 to 133 and/or 497 to 504 of a polypeptide as indicaded in Table II, columns 5 or 7, lines 128 to 133 and/or 497 to 504 the functional homologues thereof as described herein, or is encoded by the nucleic acid molecule characterized herein or the nucleic acid molecule according to the invention, for example by a nucleic acid molecule as indicated in Table I, columns 5 or 7, lines 128 to 133 and/or 497 to 504 or its herein described functional homologues and has the herein mentioned activity.
A protein having an activity conferring an increase in the amount or level of aspartic acid chemical preferably has the structure of the polypeptide described herein, in particular of a polypeptides comprising a consensus sequence as indicated in Table IV, columns 7, lines 134 to 138 and/or 505 to 516 of a polypeptide as indicaded in Table II, columns 5 or 7, lines 134 to 138 and/or 505 to 516 the functional homologues thereof as described herein, or is encoded by the nucleic acid molecule characterized herein or the nucleic acid molecule according to the invention, for example by a nucleic acid molecule as indicated in Table I, columns 5 or 7, lines 134 to 138 and/or 505 to 516 or its herein described functional homologues and has the herein mentioned activity.
A protein having an activity conferring an increase in the amount or level of citrulline chemical preferably has the structure of the polypeptide described herein, in particular of a polypeptides comprising a consensus sequence as indicated in Table IV, columns 7, lines 139 to 140 and/or 517 to 529 of a polypeptide as indicaded in Table II, columns 5 or 7, lines 139 to 140 and/or 517 to 529 the functional homologues thereof as described herein, or is encoded by the nucleic acid molecule characterized herein or the nucleic acid molecule according to the invention, for example by a nucleic acid molecule as indicated in Table I, columns 5 or 7, lines 139 to 140 and/or 517 to 529 or its herein described functional homologues and has the herein mentioned activity.
A protein having an activity conferring an increase in the amount or level of glycine chemical preferably has the structure of the polypeptide described herein, in particular of a polypeptides comprising a consensus sequence as indicated in Table IV, columns 7, lines 141 to 142 and/or 530 to 535 of a polypeptide as indicaded in Table II, columns 5 or 7, lines 141 to 142 and/or 530 to 535 the functional homologues thereof as described herein, or is encoded by the nucleic acid molecule characterized herein or the nucleic acid molecule according to the invention, for example by a nucleic acid molecule as indicated in Table I, columns 5 or 7, lines 141 to 142 and/or 530 to 535 or its herein described functional homologues and has the herein mentioned activity.
A protein having an activity conferring an increase in the amount or level of homoserine chemical preferably has the structure of the polypeptide described herein, in particular of a polypeptides comprising a consensus sequence as indicated in Table IV, columns 7, lines 143 and/or 536 of a polypeptide as indicaded in Table II, columns 5 or 7, lines 143 and/or 536 the functional homologues thereof as described herein, or is encoded by the nucleic acid molecule characterized herein or the nucleic acid molecule according to the invention, for example by a nucleic acid molecule as indicated in Table I, columns 5 or 7, lines 143 and/or 536 or its herein described functional homologues and has the herein mentioned activity.
A protein having an activity conferring an increase in the amount or level of phenylalanine chemical preferably has the structure of the polypeptide described herein, in particular of a polypeptides comprising a consensus sequence as indicated in Table IV, columns 7, lines 144 to 156 and/or 537 to 545 of a polypeptide as indicaded in Table II, columns 5 or 7, lines 144 to 156 and/or 537 to 545 the functional homologues thereof as described herein, or is encoded by the nucleic acid molecule characterized herein or the nucleic acid molecule according to the invention, for example by a nucleic acid molecule as indicated in Table I, columns 5 or 7, lines 144 to 156 and/or 537 to 545 or its herein described functional homologues and has the herein mentioned activity.
A protein having an activity conferring an increase in the amount or level of serine chemical preferably has the structure of the polypeptide described herein, in particular of a polypeptides comprising a consensus sequence as indicated in Table IV, columns 7, lines 157 to 166 and/or 546 to 554 of a polypeptide as indicaded in Table II, columns 5 or 7, lines 157 to 166 and/or 546 to 554 the functional homologues thereof as described herein, or is encoded by the nucleic acid molecule characterized herein or the nucleic acid molecule according to the invention, for example by a nucleic acid molecule as indicated in Table I, columns 5 or 7, lines 157 to 166 and/or 546 to 554 or its herein described functional homologues and has the herein mentioned activity.
A protein having an activity conferring an increase in the amount or level of tyrosine chemical preferably has the structure of the polypeptide described herein, in particular of a polypeptides comprising a consensus sequence as indicated in Table IV, columns 7, lines 167 to 172 and/or 555 to 557 of a polypeptide as indicaded in Table II, columns 5 or 7, lines 167 to 172 and/or 555 to 557 the functional homologues thereof as described herein, or is encoded by the nucleic acid molecule characterized herein or the nucleic acid molecule according to the invention, for example by a nucleic acid molecule as indicated in Table I, columns 5 or 7, lines 167 to 172 and/or 555 to 557 or its herein described functional homologues and has the herein mentioned activity.

**[0050.0.14.14]**For the purposes of the present invention "the respective fine chemical" also encompass the corresponding salts, such as, for example, the potassium, amonium or sodium salts of the respective fine chemical or the respective amino acid hydrochloride or sulfateof 5-oxoproline, alanine, aspartic acid, citrulline, glycine, homoserine, phenylalanine, serine and/or tyrosine .

**[0051.0.0.14] to [0052.0.0.14]: see [0051.0.0.0] to [0052.0.0.0]**

**[0053.0.14.14]**In one embodiment, the process of the present invention comprises one or more of the following steps:
a) stabilizing a protein conferring the increased expression of a protein encoded by the nucleic acid molecule of the invention or of the polypeptid of the invention, e.g. of a polypeptide having an activity of a protein as indicated in Table II, column 3, lines 126 to 127 and/or 492 to 496 for 5-oxoproline and/or lines 128 to 133 and/or 497 to 504 for alanine and/or lines 134 to 138 and/or 505 to 516 for aspartic acid and/or lines 139 to 140 and/or 517 to 529 for citrulline and/or lines 141 to 142 and/or 530 to 535 for glycine and/or lines 143 and/or 536 for homoserine and/or lines 144 to 156 and/or 537 to 545 for phenylalanine and/or lines 157 to 166 and/or 546 to 554 for serine and/or lines 167 to 172 and/or 555 to 557 for tyrosine resp., or its homologs activity, e.g. as indicated in Table II, columns 5 or 7, lines 126 to 127 and/or 492 to 496 for 5-oxoproline and/or lines 128 to 133 and/or 497 to 504 for alanine and/or lines 134 to 138 and/or 505 to 516 for aspartic acid and/or lines 139 to 140 and/or 517 to 529 for citrulline and/or lines 141 to 142 and/or 530 to 535 for glycine and/or lines 143 and/or 536 for homoserine and/or lines 144 to 156 and/or 537 to 545 for phenylalanine and/or lines 157 to 166 and/or 546 to 554 for serine and/or lines 167 to 172 and/or 555 to 557 for tyrosine resp., having herein-mentioned the respective fine chemical-increasing activity;
b) stabilizing a mRNA conferring the increased expression of a protein encoded by the nucleic acid molecule of the invention, e.g. of a polypeptide having an activity of a protein as indicated in Table II, column 3, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp.,
   or its homologs activity, e.g. as indicated in Table II, columns 5 or 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp., or of a mRNA encoding the polypeptide of the present invention having herein-mentioned the respective fine chemical increasing activity;
c) increasing the specific activity of a protein conferring the increased expression of a protein encoded by the nucleic acid molecule of the invention or of the polypeptide of the present invention having herein-mentioned the respective fine chemical increasing activity, e.g. of a polypeptide having an activity of a protein as indicated in Table II, column 3, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp., or its homologs activity, e.g. as indicated in Table II, columns 5 or 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp., or decreasing the inhibitory regulation of the polypeptide of the invention;
d) generating or increasing the expression of an endogenous or artificial transcription factor mediating the expression of a protein conferring the increased expression of a protein encoded by the nucleic acid molecule of the invention or of the polypeptide of the invention having herein-mentioned the respective fine chemical increasing activity, e.g. of a polypeptide having an activity of a protein as indicated in Table II, column 3, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp., or its homologs activity, e.g. as indicated in Table II, columns 5 or 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp.;
e) stimulating activity of a protein conferring the increased expression of a protein encoded by the nucleic acid molecule of the present invention or a polypeptide of the present invention having herein-mentioned the respective fine chemical increasing activity, e.g. of a polypeptide having an activity of a protein as indicated in Table II, column 3, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp., or its homologs activity, e.g. as indicated in Table II, columns 5 or 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp., by adding one or more exogenous inducing factors to the organismus or parts thereof;
f) expressing a transgenic gene encoding a protein conferring the increased expression of a polypeptide encoded by the nucleic acid molecule of the present invention or a polypeptide of the present invention, having herein-mentioned the respective fine chemical increasing activity, e.g. of a polypeptide having an activity of a protein as indicated in Table II, column 3, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp., or its homologs activity, e.g. as indicated in Table II, columns 5 or 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp.;
g) increasing the copy number of a gene conferring the increased expression of a nucleic acid molecule encoding a polypeptide encoded by the nucleic acid molecule of the invention or the polypeptide of the invention having herein-mentioned the respective fine chemical increasing activity, e.g. of a polypeptide havingan an activity of a protein as indicated in Table II, column 3, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp., or its homologs activity, e.g. as indicated in Table II, columns 5 or 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp.;
h) Increasing the expression of the endogenous gene encoding the polypeptide of the invention, e.g. a polypeptide having an activity of a protein as indicated in Table II, column 3, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp., or its homologs activity, e.g. as indicated in Table II, columns 5 or 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp., by adding positive expression or removing negative expression elements, e.g. homologous recombination can be used to either introduce positive regulatory elements like for plants the 35S enhancer into the promoter or to remove repressor elements form regulatory regions. Further gene conversion methods can be used to disrupt repressor elements or to enhance to acitivty of positive elements. Positive elements can be randomly introduced in plants by T-DNA or transposon mutagenesis and lines can be identified in which the positive elements have be integrated near to a gene of the invention, the expression of which is thereby enhanced;
i) Modulating growth conditions of an organism in such a manner, that the expression or activity of the gene encoding the protein of the invention or the protein itself is enhanced for example microorganisms or plants can be grown under a higher temperature regime leading to an enhanced expression of heat shock proteins, e.g. the heat shock protein of the invention, which can lead an enhanced the fine chemical production; and/or
j) selecting of organisms with expecially high activity of the proteins of the invention from natural or from mutagenized resources and breeding them into the target organisms, eg the elite crops.

**[0054.0.14.14]** Preferably, said mRNA is the nucleic acid molecule of the present invention and/or the protein conferring the increased expression of a protein encoded by the nucleic acid molecule of the present invention or the polypeptide having the herein mentioned activity is the polypeptide of the present invention, e.g. conferring the increase of the respective fine chemical after increasing the expression or activity of the encoded polypeptide or having the activity of a polypeptide having an activity of a protein according to Table II, column 3, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp., or its homologs activity, e.g. as indicated in Table II, columns 5 or 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp..

**[0055.0.0.14] to [0071.0.0.14]: see [0055.0.0.0] to [0071.0.0.0]**

**[0072.0.14.14]**By influencing the metabolism thus, it is possible to produce, in the process according to the invention, further advantageous compounds. Examples of such compounds are, in addition to 5-oxoproline, alanine, aspartic acid, citrulline, glycine, homoserine, phenylalanine, serine and/or tyrosine further amino acids or the respective precursors.

**[0073.0.14.14]**Accordingly, in one embodiment, the process according to the invention relates to a process which comprises:
a) providing a non-human organism, preferably a microorganism, a non-human animal, a plant or animal cell, a plant or animal tissue or a plant;
b) increasing an activity of a polypeptide of the invention or a homolog thereof, e.g. as indicated in Table II, columns 5 or 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp., or of a polypeptide being encoded by the nucleic acid molecule of the present invention and described below, i.e. conferring an increase of the respective fine chemical in the organism, preferably in a microorganism, a non-human animal, a plant or animal cell, a plant or animal tissue or a plant,
c) growing the organism, preferably a microorganism, a non-human animal, a plant or animal cell, a plant or animal tissue or a plant, under conditions which permit the production of the respective fine chemical in the organism, preferably the microorganism, the plant cell, the plant tissue or the plant; and
d) if desired, recovering, optionally isolating, the free and/or bound the respective fine chemical and , optionally further free and/or bound amino acids synthetized by the organism, the microorganism, the non-human animal, the plant or animal cell, the plant or animal tissue or the plant.

**[0074.0.0.14] to [0084.0.0.14]: see [0075.0.0.0] to [0084.0.0.0]**

**[0085.0.14.14]**With regard to the nucleic acid sequence as depicted a nucleic acid construct which contains a nucleic acid sequence mentioned herein or an organism (= transgenic organism) which is transformed with said nucleic acid sequence or said nucleic acid construct, "transgene" means all those constructs which have been brought about by genetic manipulation methods,preferably in which either
a) a nucleic acid sequence as indicated in Table I, columns 5 or 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp., or a derivative thereof, or
b) a genetic regulatory element, for example a promoter, which is functionally linked to the nucleic acid sequence as indicated in Table I, columns 5 or 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp., or a derivative thereof, or
c) (a) and (b)
is/are not present in its/their natural genetic environment or has/have been modified by means of genetic manipulation methods, it being possible for the modification to be, by way of example, a substitution, addition, deletion, inversion or insertion of one or more nucleotide. "Natural genetic environment" means the natural chromosomal locus in the organism of origin or the presence in a genomic library. In the case of a genomic library, the natural, genetic environment of the nucleic acid sequence is preferably at least partially still preserved. The environment flanks the nucleic acid sequence at least on one side and has a sequence length of at least 50 bp, preferably at least 500 bp, particularly preferably at least 1000 bp, very particularly preferably at least 5000 bp.

**[0086.0.0.14] to [0088.1.0.14]: see [0086.0.0.0] to [0088.1.0.0]**

**[0089.0.0.14] to [0097.0.0.14]: see [0089.0.0.0] to [0097.0.0.0]**

**[0098.0.14.14]**In a preferred embodiment, the respective fine chemical (5-oxoproline, alanine, aspartic acid, citrulline, glycine, homoserine, phenylalanine, serine and/or tyrosine ) are produced in accordance with the invention and, if desired, are isolated. The production of further amino acids and of amino acid mixtures or mixtures with other compounds by the process according to the invention is advantageous.

**[0099.0.0.14] to [0102.0.0.14]: see [0099.0.0.0] to [0102.0.0.0]**

**[0103.0.14.14]**In a preferred embodiment, the present invention relates to a process for the production of the respective fine chemical comprising or generating in an organism or a part thereof the expression of at least one nucleic acid molecule comprising a nucleic acid molecule selected from the group consisting of:
a) nucleic acid molecule encoding, preferably at least the mature form, of a polypeptide having a sequence as indicated in Table II, columns 5 or 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp., or a fragment thereof, which confers an increase in the amount of the respective fine chemical in an organism or a part thereof;
b) nucleic acid molecule comprising, preferably at least the mature form, of a nucleic acid molecule having a sequence as indicated in Table I, columns 5 or 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp.;
c) nucleic acid molecule whose sequence can be deduced from a polypeptide sequence encoded by a nucleic acid molecule of (a) or (b) as result of the degeneracy of the genetic code and conferring an increase in the amount of the respective fine chemical in an organism or a part thereof;
d) nucleic acid molecule encoding a polypeptide which has at least 50% identity with the amino acid sequence of the polypeptide encoded by the nucleic acid molecule of (a) to (c) and conferring an increase in the amount of the respective fine chemical in an organism or a part thereof;
e) nucleic acid molecule which hybidizes with a nucleic acid molecule of (a) to (c) under under stringent hybridisation conditions and conferring an increase in the amount of the respective fine chemical in an organism or a part thereof;
f) nucleic acid molecule encoding a polypeptide, the polypeptide being derived by substituting, deleting and/or adding one or more amino acids of the amino acid sequence of the polypeptide encoded by the nucleic acid molecules (a) to (d), preferably to (a) to (c) and conferring an increase in the amount of the respective fine chemical in an organism or a part thereof;
g) nucleic acid molecule encoding a fragment or an epitope of a polypeptide which is encoded by one of the nucleic acid molecules of (a) to (e), preferably to (a) to (c) and and conferring an increase in the amount of the respective fine chemical in an organism or a part thereof;
h) nucleic acid molecule comprising a nucleic acid molecule which is obtained by amplifying nucleic acid molecules from a cDNA library or a genomic library using the primers pairs having a sequence as indicated in Table III, columns 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp., and conferring an increase in the amount of the respective fine chemical in an organism or a part thereof;
i) nucleic acid molecule encoding a polypeptide which is isolated, e.g. from an expression library, with the aid of monoclonal antibodies against a polypeptide encoded by one of the nucleic acid molecules of (a) to (h), preferably to (a) to (c), and and conferring an increase in the amount of the respective fine chemical in an organism or a part thereof;
j) nucleic acid molecule which encodes a polypeptide comprising the consensus sequence having a sequences as indicated in Table IV, columns 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp., and conferring an increase in the amount of the respective fine chemical in an organism or a part thereof ;
k) nucleic acid molecule comprising one or more of the nucleic acid molecule encoding the amino acid sequence of a polypeptide encoding a domaine of a polypeptide indicated in Table II, columns 5 or 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp., and conferring an increase in the amount of the respective fine chemical in an organism or a part thereof; and
l) nucleic acid molecule which is obtainable by screening a suitable library under stringent conditions with a probe comprising one of the sequences of the nucleic acid molecule of (a) to (k), preferably to (a) to (c), or with a fragment of at least 15 nt, preferably 20 nt, 30 nt, 50 nt, 100 nt, 200 nt or 500 nt of the nucleic acid molecule characterized in (a) to (k), preferably to (a) to (c), and conferring an increase in the amount of the respective fine chemical in an organism or a part thereof;
or which comprises a sequence which is complementary thereto.

**[00103.1.0.14.]** In one embodiment, the nucleic acid molecule used in the process of the invention distinguishes over the sequence indicated in Table IA, columns 5 or 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp., by one or more nucleotides. In one embodiment, the nucleic acid molecule used in the process of the invention does not consist of the sequence shown in indicated in Table I A, columns 5 or 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp.. In one embodiment, the nucleic acid molecule used in the process of the invention is less than 100%, 99,999%, 99,99%, 99,9% or 99% identical to a sequence indicated in Table I A, columns 5 or 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp.. In another embodiment, the nucleic acid molecule does not encode a polypeptide of a sequence indicated in Table II A, columns 5 or 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp..

**[00103.2.0.14.]** In one embodiment, the nucleic acid molecule used in the process of the invention distinguishes over the sequence indicated in Table I B, columns 5 or 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp., by one or more nucleotides. In one embodiment, the nucleic acid molecule used in the process of the invention does not consist of the sequence shown in indicated in Table I B, columns 5 or 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp.. In one embodiment, the nucleic acid molecule used in the process of the invention is less than 100%, 99,999%, 99,99%, 99,9% or 99% identical to a sequence indicated in Table I B, columns 5 or 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp.. In another embodiment, the nucleic acid molecule does not encode a polypeptide of a sequence indicated in Table II B, columns 5 or 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp..

**[0104.0.14.14]** In one embodiment, the nucleic acid molecule of the invention distinguishes over the sequence indicated in Table I, columns 5 or 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp., by one or more nucleotides. In one embodiment, the nucleic acid molecule of the invention does not consist of the sequence shown in indicated in Table I, columns 5 or 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp.. In one embodiment, the nucleic acid molecule of the present invention is less than 100%, 99,999%, 99,99%, 99,9% or 99% identical to a sequence indicated in Table I, columns 5 or 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp.. In another embodiment, the nucleic acid molecule does not encode a polypeptide of a sequence indicated in Table II, columns 5 or 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp..

**[0105.0.0.14] to [0107.0.0.14]: see [0105.0.0.0] to [0107.0.0.0]**

**[0108.0.14.14]** Nucleic acid molecules with the sequence as indicated in Table I, columns 5 or 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp., nucleic acid molecules which are derived from a amino acid sequences as indicated in Table II, columns 5 or 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp., or from polypeptides comprising the consensus sequence as indicated in Table IV, columns 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp., or their derivatives or homologues encoding polypeptides with the enzymatic or biological activity of a polypeptide as indicated in Table I, column 3, 5 or 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp., or e.g. conferring a increase of the respective fine chemical after increasing its expression or activity are advantageously increased in the process according to the invention.

**[0109.0.0.14]: see [0109.0.0.0]**

**[0110.0.4.4]** Nucleic acid molecules, which are advantageous for the process according to the invention and which encode polypeptides with an activity of a polypeptide of the invention or the polypeptide used in the method of the invention or used in the process of the invention, e.g. of a protein as indicated in Table II, column 5, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp., being encoded by a nucleic acid molecule indicated in Table I, column 5, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp., or of its homologs, e.g. as indicated in Table II, column 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp., can be determined from generally accessible databases.

**[0111.0.0.14]: see [0111.0.0.0]**

**[0112.0.14.14]** The nucleic acid molecules used in the process according to the invention take the form of isolated nucleic acid sequences, which encode polypeptides with an activity of a polypeptide as indicated in Table I, column3, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp., or having the sequence of a polypeptide as indicated in Table II, columns 5 and 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp., and conferring a 5-oxoproline and/or alanine and/or aspartic acid and/or citrulline and/or glycine and/or homoserine and/or phenylalanine and/or serine and/or tyrosine increase.

**[0113.0.0.14] to [0120.0.0.14]: see [0113.0.0.0] to [0120.0.0.0]**

**[0121.0.14.14]** However, it is also possible to use artificial sequences, which differ in one or more bases from the nucleic acid sequences found in organisms, or in one or more amino acid molecules from polypeptide sequences found in organisms, in particular from the polypeptide sequences indicated in Table II, columns 5 or 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp., or the functional homologues thereof as described herein, preferably conferring above-mentioned activity, i.e. conferring a increase of the respective fine chemical after increasing its activity.

**[0122.0.0.14] to [0127.0.0.14]: see [0122.0.0.0] to [0127.0.0.0]**

**[0128.0.14.14]** Synthetic oligonucleotide primers for the amplification, e.g. as the pairs indicated in Table III, column 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp., by means of polymerase chain reaction can be generated on the basis of a sequence shown herein, for example the sequence as indicated in Table I, columns 5 or 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp., or the sequences derived from sequences as indicated in Table II, columns 5 or 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp..

**[0129.0.14.14]** Moreover, it is possible to identify conserved regions from various organisms by carrying out protein sequence alignments with the polypeptide used in the process of the invention, in particular with sequences of the polypeptide of the invention, from which conserved regions, and in turn, degenerate primers can be derived. Conserved region for the polypeptide of the invention are indicated in the alignments shown in the figures. Conserved regions are those, which show a very little variation in the amino acid in one particular position of several homologs from different origin. The consensus sequences indicated in Table IV, column 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp., are derived from said alignments.

**[0130.0.14.14]**Degenerated primers can then be utilized by PCR for the amplification of fragments of novel proteins having above-mentioned activity, e.g. conferring the increase of amino acids, e.g. of 5-oxoproline, alanine, aspartic acid, citrulline, glycine, homoserine, phenylalanine, serine and/or tyrosine after increasing its expression or activity of the protein comprising said fragment.

**[0131.0.0.14] to [0138.0.0.14]: see [0131.0.0.0] to [0138.0.0.0]**

**[0139.0.14.14]** Polypeptides having above-mentioned activity, i.e. conferring an increase of the respective fine chemical level, derived from other organisms, can be encoded by other DNA sequences which hybridize to a sequences indicated in Table I, columns 5 or 7, preferably Table I B, lines 126 to 127 and/or 492 to 496 for 5-oxoproline and/or lines 128 to 133 and/or 497 to 504 for alanine and/or lines 134 to 138 and/or 505 to 516 for aspartic acid and/or lines 139 to 140 and/or 517 to 529 for citrulline and/or lines 141 to 142 and/or 530 to 535 for glycine and/or lines 143 and/or 536 for homoserine and/or lines 144 to 156 and/or 537 to 545 for phenylalanine and/or lines 157 to 166 and/or 546 to 554 for serine and/or lines 167 to 172 and/or 555 to 557 for tyrosine resp., under relaxed hybridization conditions and which code on expression for peptides having the respective fine chemical, in particular, of 5-oxoproline and/or alanine and/or aspartic acid and/or citrulline and/or glycine and/or homoserine and/or phenylalanine and/or serine and/or tyrosine resp., increasing activity.

**[0140.0.0.14] to [0146.0.0.14]: see [0140.0.0.0] to [0146.0.0.0]**

**[0147.0.14.14]** Further, the nucleic acid molecule of the invention comprises a nucleic acid molecule, which is a complement of one of the nucleotide sequences of above mentioned nucleic acid molecules or a portion thereof. A nucleic acid molecule which is complementary to one of the nucleotide sequences indicated in Table I, columns 5 or 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp., preferably of Table I B, columns 5 or 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp., is one which is sufficiently complementary to one of said nucleotide sequences s such that it can hybridize to one of said nucleotide sequences thereby forming a stable duplex. Preferably, the hybridisation is performed under stringent hybrization conditions. However, a complement of one of the herein disclosed sequences is preferably a sequence complement thereto according to the base pairing of nucleic acid molecules well known to the skilled person. For example, the bases A and G undergo base pairing with the bases T and U or C, resp. and visa versa. Modifications of the bases can influence the base-pairing partner.

**[0148.0.14.14]** The nucleic acid molecule of the invention comprises a nucleotide sequence which is at least about 30%, 35%, 40% or 45%, preferably at least about 50%, 55%, 60% or 65%, more preferably at least about 70%, 80%, or 90%, and even more preferably at least about 95%, 97%, 98%, 99% or more homologous to a nucleotide sequence indicated in Table I, columns 5 or 7, preferably Table I B, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp., preferably Table I B or a portion thereof and preferably has above mentioned activity, in particular, of arginine and/or glutamate and/or proline and/or glutamine increasing activity after increasing the acitivity or an activity of a product of a gene encoding said sequences or their homologs.

**[0149.0.14.14]** The nucleic acid molecule of the invention or the nucleic acid molecule used in the method of the invention comprises a nucleotide sequence which hybridizes, preferably hybridizes under stringent conditions as defined herein, to one of the nucleotide sequences indicated in Table I, columns 5 or 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp., preferably of Table I B, columns 5 or 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp., or a portion thereof and encodes a protein having above-mentioned activity and as indicated in indicated in Table II.

**[00149.1.14.14]** Optionally, the nucleotide sequence, which hybridises to one of the nucleotide sequences indicated in Table I, columns 5 or 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp., preferably of Table I B, columns 5 or 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp., has further one or more of the activities annotated or known for the a protein as indicated in Table II, column 3, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp..

**[0150.0.14.14]** Moreover, the nucleic acid molecule of the invention can comprise only a portion of the coding region of one of the sequences indicated in Table I, columns 5 or 7, preferably Table I B, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp., for example a fragment which can be used as a probe or primer or a fragment encoding a biologically active portion of the polypeptide of the present invention or of a polypeptide used in the process of the present invention, i.e. having above-mentioned activity, e.g.conferring an increase of arginine and/or glutamate and/or proline and/or glutamine, resp., if its activity is increased. The nucleotide sequences determined from the cloning of the present protein-according-to-the-invention-encoding gene allows for the generation of probes and primers designed for use in identifying and/or cloning its homologues in other cell types and organisms. The probe/primer typically comprises substantially purified oligonucleotide. The oligonucleotide typically comprises a region of nucleotide sequence that hybridizes under stringent conditions to at least about 12, 15 preferably about 20 or 25, more preferably about 40, 50 or 75 consecutive nucleotides of a sense strand of one of the sequences set forth, e.g., as indicated in Table I, columns 5 or 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp., an anti-sense sequence of one of the sequences, e.g., as indicated in Table I, columns 5 or 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp., or naturally occurring mutants thereof. Primers based on a nucleotide of invention can be used in PCR reactions to clone homologues of the polypeptide of the invention or of the polypeptide used in the process of the invention, e.g. as the primers described in the examples of the present invention, e.g. as shown in the examples. A PCR with the primer pairs indicated in Table III, column 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp., will result in a fragment of a polynucleotide sequence as indicated in Table I, columns 5 or 7 lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp., or its gene product.

**[0151.0.0.14] see [0151.0.0.0]**

**[0152.0.14.14]** The nucleic acid molecule of the invention encodes a polypeptide or portion thereof which includes an amino acid sequence which is sufficiently homologous to an amino acid sequence as indicated in Table II, columns 5 or 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp., such that the protein or portion thereof maintains the ability to participate in the respective fine chemical production, in particular an activity increasing the level of 5-oxoproline and/or alanine and/or aspartic acid and/or citrulline and/or glycine and/or homoserine and/or phenylalanine and/or serine and/or tyrosine as mentioned above or as described in the examples in plants or microorganisms is comprised.

**[0153.0.14.14]** As used herein, the language "sufficiently homologous" refers to proteins or portions thereof which have amino acid sequences which include a minimum number of identical or equivalent amino acid residues (e.g., an amino acid residue which has a similar side chain as an amino acid residue in one of the sequences of the polypeptide of the present invention) to an amino acid sequence as indicated in Table I, columns 5 or 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp., such that the protein or portion thereof is able to participate in the increase of the respective fine chemical production. In one embodiment, a protein or portion thereof as indicated in Table II, columns 5 or 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp., has for example an activity of a polypeptide indicaded in Table II, column 3, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp..

**[0154.0.14.14]** In one embodiment, the nucleic acid molecule of the present invention comprises a nucleic acid that encodes a portion of the protein of the present invention. The protein is at least about 30%, 35%, 40%, 45% or 50%, preferably at least about 55%, 60%, 65% or 70%, and more preferably at least about 75%, 80%, 85%, 90%, 91%, 92%, 93% or 94% and most preferably at least about 95%, 97%, 98%, 99% or more homologous to an entire amino acid sequence as indicated in Table II, columns 5 or 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp., and has above-mentioned activity, e.g. conferring preferably the increase of the respective fine chemical.

**[0155.0.0.14] to [0156.0.0.14]: see [0155.0.0.0] to [0156.0.0.0]**

**[0157.0.14.14]** The invention further relates to nucleic acid molecules that differ from one of the nucleotide sequences indicated in Table I, columns 5 or 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp., (and portions thereof) due to degeneracy of the genetic code and thus encode a polypeptide of the present invention, in particular a polypeptide having above mentioned activity, e.g. conferring an increase in the respective fine chemical in a organism, e.g. as that polypeptides comprising the consensus sequences as indicated in Table IV, column 7 lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp., or of the polypeptide as indicated in Table II, columns 5 or 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp., or their functional homologues. Advantageously, the nucleic acid molecule of the invention or the nucleic acid molecule used in the method of the invention comprises, or in an other embodiment has, a nucleotide sequence encoding a protein comprising, or in an other embodiment having, a consensus sequences as indicated in Table IV, column 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp., or of the polypeptide as indicated in Table II, columns 5 or 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp., or the functional homologues. In a still further embodiment, the nucleic acid molecule of the invention or the nucleic acid molecule used in the method of the invention encodes a full length protein which is substantially homologous to an amino acid sequence comprising a consensus sequence as indicated in Table IV, column 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp., or of a polypeptide as indicated in Table II, columns 5 or 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp., or the functional homologues thereof. However, in a preferred embodiment, the nucleic acid molecule of the present invention does not consist of a sequence as indicated in Table I, columns 5 or 7 lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp., preferably as indicated in Table I A, columns 5 or 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp.. Preferably the nucleic acid molecule of the invention is a functional homologue or identical to a nucleic acid molecule indicated in Table I B, columns 5 or 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp..

**[0158.0.0.14] to [0160.0.0.14]: see [0158.0.0.0] to [0160.0.0.0]**

**[0161.0.14.14]** Accordingly, in another embodiment, a nucleic acid molecule of the invention is at least 15, 20, 25 or 30 nucleotides in length. Preferably, it hybridizes under stringent conditions to a nucleic acid molecule comprising a nucleotide sequence of the nucleic acid molecule of the present invention or used in the process of the present invention, e.g. comprising a sequence as indicated in Table I, columns 5 or 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp.. The nucleic acid molecule is preferably at least 20, 30, 50, 100, 250 or more nucleotides in length.

**[0162.0.0.14] see [0162.0.0.0]**

**[0163.0.14.14]** Preferably, nucleic acid molecule of the invention that hybridizes under stringent conditions to a sequence as indicated in Table I, columns 5 or 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp., corresponds to a naturally-occurring nucleic acid molecule of the invention. As used herein, a "naturally-occurring" nucleic acid molecule refers to an RNA or DNA molecule having a nucleotide sequence that occurs in nature (e.g., encodes a natural protein). Preferably, the nucleic acid molecule encodes a natural protein having above-mentioned activity, e.g. conferring the respective fine chemical increase after increasing the expression or activity thereof or the activity of an protein of the invention or used in the process of the invention.

**[0164.0.0.14] see [0164.0.0.0]**

**[0165.0.14.14]** For example, nucleotide substitutions leading to amino acid substitutions at "non-essential" amino acid residues can be made in a sequence of the nucleic acid molecule of the invention or used in the process of the invention, e.g. as indicated in Table I, columns 5 or 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp..

**[0166.0.0.14] to [0167.0.0.14]: see [0166.0.0.0] to [0167.0.0.0]**

**[0168.0.14.14]** Accordingly, the invention relates to nucleic acid molecules encoding a polypeptide having above-mentioned activity, e.g. conferring an increase in the the respective fine chemical in an organisms or parts thereof that contain changes in amino acid residues that are not essential for said activity. Such polypeptides differ in amino acid sequence from a sequence contained in a sequence as indicated in Table II, columns 5 or 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp., preferably of Table II B, column 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp., yet retain said activity described herein. The nucleic acid molecule can comprise a nucleotide sequence encoding a polypeptide, wherein the polypeptide comprises an amino acid sequence at least about 50% identical to an amino acid sequence as indicated in Table II, columns 5 or 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp., preferably of Table II B, column 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp., and is capable of participation in the increase of production of the respective fine chemical after increasing its activity, e.g. its expression. Preferably, the protein encoded by the nucleic acid molecule is at least about 60% identical to a sequence as indicated in Table II, columns 5 or 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp., preferably of Table II B, column 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp., more preferably at least about 70% identical to one of the sequences as indicated in Table II, columns 5 or 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp., preferably of Table II B, column 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp., even more preferably at least about 80%, 90%, or 95% homologous to a sequence as indicated in Table II, columns 5 or 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp., preferably of Table II B, column 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp., and most preferably at least about 96%, 97%, 98%, or 99% identical to the sequence as indicated in Table II, columns 5 or 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp., preferably of Table II B, column 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp..

**[0169.0.0.14] to [0172.0.0.14]: see [0169.0.0.0] to [0172.0.0.0]**

**[0173.0.14.14]**For example a sequence which has a 80% homology with sequence SEQ ID NO: 21744 at the nucleic acid level is understood as meaning a sequence which, upon comparison with the sequence SEQ ID NO: 21744 by the above Gap program algorithm with the above parameter set, has a 80% homology.

**[0174.0.0.14] see [0174.0.0.0]**

**[0175.0.14.14]**For example a sequence which has a 80% homology with sequence SEQ ID NO: 21745 at the protein level is understood as meaning a sequence which, upon comparison with the sequence SEQ ID NO: 21745 by the above program algorithm with the above parameter set, has a 80% homology.

**[0176.0.14.14]** Functional equivalents derived from one of the polypeptides as indicated in Table II, columns 5 or 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp., according to the invention by substitution, insertion or deletion have at least 30%, 35%, 40%, 45% or 50%, preferably at least 55%, 60%, 65% or 70% by preference at least 80%, especially preferably at least 85% or 90%, 91%, 92%, 93% or 94%, very especially preferably at least 95%, 97%, 98% or 99% homology with one of the polypeptides as indicated in Table II, columns 5 or 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp., according to the invention and are distinguished by essentially the same properties as a polypeptide as indicated in Table II, columns 5 or 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp..

**[0177.0.14.14]** Functional equivalents derived from a nucleic acid sequence as indicated in Table I, columns 5 or 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp., preferably of Table I B, column 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp., according to the invention by substitution, insertion or deletion have at least 30%, 35%, 40%, 45% or 50%, preferably at least 55%, 60%, 65% or 70% by preference at least 80%, especially preferably at least 85% or 90%, 91%, 92%, 93% or 94%, very especially preferably at least 95%, 97%, 98% or 99% homology with one of a polypeptide as indicated in Table II, columns 5 or 7 lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp., according to the invention and encode polypeptides having essentially the same properties as a polypeptide as indicated in Table II, columns 5 or 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp., preferably of Table I B, column 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp..

**[0178.0.0.14]: see [0178.0.0.0]**

**[0179.0.14.14]** A nucleic acid molecule encoding an homologous to a protein sequence as indicated in Table II, columns 5 or 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp., preferably of Table II B, column 7 lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp., can be created by introducing one or more nucleotide substitutions, additions or deletions into a nucleotide sequence of the nucleic acid molecule of the present invention, in particular as indicated in Table I, columns 5 or 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp., such that one or more amino acid substitutions, additions or deletions are introduced into the encoded protein. Mutations can be introduced into the encoding sequences of a sequences as indicated in Table I, columns 5 or 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp., by standard techniques, such as site-directed mutagenesis and PCR-mediated mutagenesis.

**[0180.0.0.14]** to **[0183.0.0.14]: see [0180.0.0.0]** to **[0183.0.0.0]**

**[0184.0.14.14]** Homologues of the nucleic acid sequences used, with a sequence as indicated in Table I, preferably Table I B, columns 5 or 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp., or of the nucleic acid sequences derived from a sequences as indicated in Table II, columns 5 or 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp., comprise also allelic variants with at least approximately 30%, 35%, 40% or 45% homology, by preference at least approximately 50%, 60% or 70%, more preferably at least approximately 90%, 91%, 92%, 93%, 94% or 95% and even more preferably at least approximately 96%, 97%, 98%, 99% or more homology with one of the nucleotide sequences shown or the abovementioned derived nucleic acid sequences or their homologues, derivatives or analogues or parts of these. Allelic variants encompass in particular functional variants which can be obtained by deletion, insertion or substitution of nucleotides from the sequences shown, preferably from a sequence as indicated in Table I, columns 5 or 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp., or from the derived nucleic acid sequences, the intention being, however, that the enzyme activity or the biological activity of the resulting proteins synthesized is advantageously retained or increased.

**[0185.0.14.14]** In one embodiment of the present invention, the nucleic acid molecule of the invention or used in the process of the invention comprises one or more sequences as indicated in Table I, preferably Table I B, columns 5 or 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp.. In one embodiment, it is preferred that the nucleic acid molecule comprises as little as possible other nucleotides not shown in any one of sequences as indicated in Table I, preferably table I B, columns 5 or 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp.. In one embodiment, the nucleic acid molecule comprises less than 500, 400, 300, 200, 100, 90, 80, 70, 60, 50 or 40 further nucleotides. In a further embodiment, the nucleic acid molecule comprises less than 30, 20 or 10 further nucleotides. In one embodiment, a nucleic acid molecule used in the process of the invention is identical to a sequences as indicated in Table I, columns 5 or 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp.,

**[0186.0.14.14]** Also preferred is that one or more nucleic acid molecule(s) used in the process of the invention encodes a polypeptide comprising a sequence as indicated in Table II, preferably table II B, columns 5 or 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp.. In one embodiment, the nucleic acid molecule encodes less than 150, 130, 100, 80, 60, 50, 40 or 30 further amino acids. In a further embodiment, the encoded polypeptide comprises less than 20, 15, 10, 9, 8, 7, 6 or 5 further amino acids. In one embodiment, the encoded polypeptide used in the process of the invention is identical to the sequences as indicated in Table II, preferably table II B, columns 5 or 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp..

**[0187.0.14.14]** In one embodiment, a nucleic acid molecule of the invention or used in the process encodes a polypeptide comprising the sequence as indicated in Table II, columns 5 or 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp., comprises less than 100 further nucleotides. In a further embodiment, said nucleic acid molecule comprises less than 30 further nucleotides. In one embodiment, the nucleic acid molecule used in the process is identical to a coding sequence as indicated in Table II, preferably Table II B, columns 5 or 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp..

**[0188.0.14.14]** Polypeptides (= proteins), which still have the essential biological or enzymatic activity of the polypeptide of the present invention conferring an increase of the respective fine chemical i.e. whose activity is essentially not reduced, are polypeptides with at least 10% or 20%, by preference 30% or 40%, especially preferably 50% or 60%, very especially preferably 80% or 90 or more of the wild type biological activity or enzyme activity, advantageously, the activity is essentially not reduced in comparison with the activity of a polypeptide as indicated in Table II, columns 5 or 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp., and is expressed under identical conditions.
In one embodiment, the polypeptide of the invention is a homolog consisting of or comprising the sequence as indicated in Table II B, columns 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp..

**[0189.0.14.14]** Homologues of a sequences as indicated in Table I, columns 5 or 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp., or of a derived sequences as indicated in Table II, columns 5 or 7 lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp., also mean truncated sequences, cDNA, single-stranded DNA or RNA of the coding and noncoding DNA sequence. Homologues of said sequences are also understood as meaning derivatives, which comprise noncoding regions such as, for example, UTRs, terminators, enhancers or promoter variants. The promoters upstream of the nucleotide sequences stated can be modified by one or more nucleotide substitution(s), insertion(s) and/or deletion(s) without, however, interfering with the functionality or activity either of the promoters, the open reading frame (= ORF) or with the 3'-regulatory region such as terminators or other 3'regulatory regions, which are far away from the ORF. It is furthermore possible that the activity of the promoters is increased by modification of their sequence, or that they are replaced completely by more active promoters, even promoters from heterologous organisms. Appropriate promoters are known to the person skilled in the art and are mentioned herein below.

**[0190.0.0.14] to [0203.0.0.14]: see [0190.0.0.0] to [0203.0.0.0]**

**[0204.0.14.14]** Accordingly, in one embodiment, the invention relates to a nucleic acid molecule which comprises a nucleic acid molecule selected from the group consisting of:
a) nucleic acid molecule encoding, preferably at least the mature form, of a polypeptide as indicated in Table II, preferably Table II B, columns 5 or 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp., or a fragment thereof conferring an increase in the amount of the respective fine chemical, in particular, of 5-oxoproline (lines 126 to 127 and/or 492 to 496) and/or alanine (lines 128 to 133 and/or 497 to 504) and/or aspartic acid (lines 134 to 138 and/or 505 to 516) and/or citrulline (lines 139 to 140 and/or 517 to 529) and/or glycine (lines 141 to 142 and/or 530 to 535) and/or homoserine (lines 143 and/or 536) and/or phenylalanine (lines 144 to 156 and/or 537 to 545) and/or serine (lines 157 to 166 and/or 546 to 554) and/or tyrosine (lines 167 to 172 and/or 555 to 557), resp., in an organism or a part thereof
b) nucleic acid molecule comprising, preferably at least the mature form, of a nucleic acid molecule as indicated in Table I, columns 5 or 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp., or a fragment thereof conferring an increase in the amount of the respective fine chemical in an organism or a part thereof;
c) nucleic acid molecule whose sequence can be deduced from a polypeptide sequence encoded by a nucleic acid molecule of (a) or (b) as result of the degeneracy of the genetic code and conferring an increase in the amount of the fine chemical in an organism or a part thereof;
d) nucleic acid molecule encoding a polypeptide whose sequence has at least 50% identity with the amino acid sequence of the polypeptide encoded by the nucleic acid molecule of (a) to (c) and conferring an increase in the amount of the fine chemical in an organism or a part thereof;
e) nucleic acid molecule which hybridizes with a nucleic acid molecule of (a) to (c) under under stringent hybridisation conditions and conferring an increase in the amount of the fine chemical in an organism or a part thereof;
f) nucleic acid molecule encoding a polypeptide, the polypeptide being derived by substituting, deleting and/or adding one or more amino acids of the amino acid sequence of the polypeptide encoded by the nucleic acid molecules (a) to (d), preferably to (a) to (c), and conferring an increase in the amount of the fine chemical in an organism or a part thereof;
g) nucleic acid molecule encoding a fragment or an epitope of a polypeptide which is encoded by one of the nucleic acid molecules of (a) to (e), preferably to (a) to (c) and conferring an increase in the amount of the fine chemical in an organism or a part thereof ;
h) nucleic acid molecule comprising a nucleic acid molecule which is obtained by amplifying a cDNA library or a genomic library using primers or primer pairs as indicated in Table III, column 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp., and conferring an increase in the amount of the respective fine chemical, in particular, of 5-oxoproline (lines 126 to 127 and/or 492 to 496) and/or alanine (lines 128 to 133 and/or 497 to 504) and/or aspartic acid (lines 134 to 138 and/or 505 to 516) and/or citrulline (lines 139 to 140 and/or 517 to 529) and/or glycine (lines 141 to 142 and/or 530 to 535) and/or homoserine (lines 143 and/or 536) and/or phenylalanine (lines 144 to 156 and/or 537 to 545) and/or serine (lines 157 to 166 and/or 546 to 554) and/or tyrosine (lines 167 to 172 and/or 555 to 557), resp., in an organism or a part thereof;
i) nucleic acid molecule encoding a polypeptide which is isolated, e.g. from a expression library, with the aid of monoclonal antibodies against a polypeptide encoded by one of the nucleic acid molecules of (a) to (g), preferably to (a) to (c) and conferring an increase in the amount of the fine chemical in an organism or a part thereof;
j) nucleic acid molecule which encodes a polypeptide comprising a consensus sequence as indicated in Table IV, columns 5 or 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp., and conferring an increase in the amount of the respective fine chemical, in particular, of 5-oxoproline (lines 126 to 127 and/or 492 to 496) and/or alanine (lines 128 to 133 and/or 497 to 504) and/or aspartic acid (lines 134 to 138 and/or 505 to 516) and/or citrulline (lines 139 to 140 and/or 517 to 529) and/or glycine (lines 141 to 142 and/or 530 to 535) and/or homoserine (lines 143 and/or 536) and/or phenylalanine (lines 144 to 156 and/or 537 to 545) and/or serine (lines 157 to 166 and/or 546 to 554) and/or tyrosine (lines 167 to 172 and/or 555 to 557), resp., in an organism or a part thereof;
k) nucleic acid molecule encoding the amino acid sequence of a polypeptide encoding a domaine of a polypeptide as indicated in Table II, columns 5 or 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp., and conferring an increase in the amount of the respective fine chemical, in particular, of 5-oxoproline (lines 126 to 127 and/or 492 to 496) and/or alanine (lines 128 to 133 and/or 497 to 504) and/or aspartic acid (lines 134 to 138 and/or 505 to 516) and/or citrulline (lines 139 to 140 and/or 517 to 529) and/or glycine (lines 141 to 142 and/or 530 to 535) and/or homoserine (lines 143 and/or 536) and/or phenylalanine (lines 144 to 156 and/or 537 to 545) and/or serine (lines 157 to 166 and/or 546 to 554) and/or tyrosine (lines 167 to 172 and/or 555 to 557), resp., in an organism or a part thereof; and
l) nucleic acid molecule which is obtainable by screening a suitable nucleic acid library under stringent hybridization conditions with a probe comprising one of the sequences of the nucleic acid molecule of (a) to (k) or with a fragment of at least 15 nt, preferably 20 nt, 30 nt, 50 nt, 100 nt, 200 nt or 500 nt of the nucleic acid molecule characterized in (a) to (h) or of a nucleic acid molecule as indicated in Table I, columns 5 or 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp., or a nucleic acid molecule encoding, preferably at least the mature form of, a polypeptide as indicated in Table II, columns 5 or 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp., and conferring an increase in the amount of the respective fine chemical in an organism or a part thereof;
   or which encompasses a sequence which is complementary thereto;
   whereby, preferably, the nucleic acid molecule according to (a) to (I) distinguishes over the sequence indicated in Table IA, columns 5 or 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp., by one or more nucleotides. In one embodiment, the nucleic acid molecule does not consist of the sequence shown and indicated in Table I A or I B, columns 5 or 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp..
   In one embodiment, the nucleic acid molecule is less than 100%, 99,999%, 99,99%, 99,9% or 99% identical to a sequence indicated in Table I A or I B, columns 5 or 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp..
   In another embodiment, the nucleic acid molecule does not encode a polypeptide of a sequence indicated in Table II A or II B, columns 5 or 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp..
   In an other embodiment, the nucleic acid molecule of the present invention is at least 30 %, 40 %, 50%, or 60% identical and less than 100%, 99,999%, 99,99%, 99,9% or 99% identical to a sequence indicated in Table I A or I B, columns 5 or 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp..
   In a further embodiment the nucleic acid molecule does not encode a polypeptide sequence as indicated in Table II A or II B, columns 5 or 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp..
   Accordingly, in one embodiment, the nucleic acid molecule of the differs at least in one or more residues from a nucleic acid molecule indicated in Table I A or I B, columns 5 or 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp..
   Accordingly, in one embodiment, the nucleic acid molecule of the present invention encodes a polypeptide, which differs at least in one or more amino acids from a polypeptide indicated in Table II A or I B, columns 5 or 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp..
   In another embodiment, a nucleic acid molecule indicated in Table I A or I B, columns 5 or 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp., does not encode a protein of a sequence indicated in Table II A or II B, columns 5 or 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp..
   Accordingly, in one embodiment, the protein encoded by a sequences of a nucleic acid accoriding to (a) to (I) does not consist of a sequence as indicated in Table II A or II B, columns 5 or 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp..
   In a further embodiment, the protein of the present invention is at least 30 %, 40 %, 50%, or 60% identical to a protein sequence indicated in Table II A or II B, columns 5 or 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp., and less than 100%, preferably less than 99,999%, 99,99% or 99,9%, more preferably less than 99%, 985, 97%, 96% or 95% identical to a sequence as indicated in Table I A or II B, columns 5 or 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp..

**[0205.0.0.14] to [0226.0.0.14]: see [0205.0.0.0] to [0226.0.0.0]**

**[0227.0.14.14]**The abovementioned nucleic acid molecules can be cloned into the nucleic acid constructs or vectors according to the invention in combination together with further genes, or else different genes are introduced by transforming several nucleic acid constructs or vectors (including plasmids) into a host cell, advantageously into a plant cell or a microorgansims.
In addition to a sequence indicated in Table I, columns 5 or 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp., or its derivatives, it is advantageous additionally to express and/or mutate further genes in the organisms. Especially advantageously, additionally at least one further gene of the amino acid biosynthetic pathway such as for amino acid precursors is expressed in the organisms such as plants or microorganisms. It is also possible that the regulation of the natural genes has been modified advantageously so that the gene and/or its gene product is no longer subject to the regulatory mechanisms which exist in the organisms. This leads to an increased synthesis of the amino acids desired since, for example, feedback regulations no longer exist to the same extent or not at all. In addition it might be advantageously to combine a sequence as indicated in Table I, columns 5 or 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp., with genes which generally support or enhances to growth or yield of the target organismen, for example genes which lead to faster growth rate of microorganisms or genes which produces stress-, pathogen, or herbicide resistant plants.

**[0228.0.0.14] to [0230.0.0.14]: see [0228.0.0.0] to [0230.0.0.0]**

**[00231.0.14.14]** In a further advantageous embodiment of the process of the invention, the organisms used in the process are those in which simultaneously a protein degrading 5-oxoproline and/or alanine and/or aspartic acid and/or citrulline and/or glycine and/or homoserine and/or phenylalanine and/or serine and/or tyrosine resp., is attenuated, in particular by reducing the rate of expression of the corresponding gene.

**[0232.0.0.14] to [0282.0.0.14]: see [0232.0.0.0] to [0282.0.0.0]**

**[0283.0.14.14]** Moreover, a native polypeptide conferring the increase of the respective fine chemical in an organism or part thereof can be isolated from cells (e.g., endothelial cells), for example using the antibody of the present invention as described below, e.g. an antibody against a protein as indicated in Table II, column 3, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp., or an antibody against a polypeptide as indicated in Table II, columns 5 or 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp., which can be produced by standard techniques utilizing polypeptides comprising or consisting of above mentioned sequences, e.g. the polypeptid of the present invention or fragment thereof,. Preferred are monoclonal antibodies.

**[0284.0.0.14] see [0284.0.0.0]**

**[0285.0.14.14]** In one embodiment, the present invention relates to a polypeptide having a sequence as indicated in Table II, columns 5 or 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp., or as coded by a nucleic acid molecule as indicated in Table I, columns 5 or 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp., or functional homologues thereof.

**[0286.0.14.14]** In one advantageous embodiment, in the method of the present invention the activity of a polypeptide is increased which comprises or consists of a consensus sequence as indicated in Table IV, column 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp..
In another embodiment, the present invention relates to a polypeptide comprising or consisting of a consensus sequence as indicated in Table IV, column 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp., whereby 20 or less, preferably 15 or 10, preferably 9, 8, 7, or 6, more preferred 5 or 4, even more preferred 3, even more preferred 2, even more preferred 1, most preferred 0 of the amino acids positions indicated can be replaced by any amino acid or, in an further embodiment, can be replaced and/or absent. In one embodiment, the present invention relates to the method of the present invention comprising a polypeptide or to a polypeptide comprising more than one consensus sequences (of an individual line) as indicated in Table IV, column 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp..

**[0287.0.0.14] to [0290.0.0.14]: see [0287.0.0.0] to [0290.0.0.0]**

**[0291.0.14.14]** In one advantageous embodiment, the method of the present invention comprises the increasing of a polypeptide comprising or consisting of plant or microorganism specific consensus sequences. In one embodiment, said polypeptide of the invention distinguishes over a sequence as indicated in Table II A or II B, columns 5 or 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp., by one or more amino acids. In one embodiment, polypeptide distinguishes form a sequence as indicated in Table II A or II B, columns 5 or 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp., by more than 1, 2, 3, 4, 5, 6, 7, 8 or 9 amino acids, preferably by more than 10, 15, 20, 25 or 30 amino acids, evenmore preferred are more than 40, 50, or 60 amino acids and, preferably, the sequence of the polypeptide of the invention distinguishes from a sequence as indicated in Table II A or II B, columns 5 or 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp., by not more than 80% or 70% of the amino acids, preferably not more than 60% or 50%, more preferred not more than 40% or 30%, even more preferred not more than 20% or 10%. In an other embodiment, said polypeptide of the invention does not consist of a sequence as indicated in Table II A or II B, columns 5 or 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp..

**[0292.0.0.14]: see [0292.0.0.0]**

**[0293.0.14.14]** In one embodiment, the invention relates to polypeptide conferring an increase in the respective fine chemical in an organism or part thereof and being encoded by the nucleic acid molecule of the invention or a nucleic acid molecule used in the process of the invention. In one embodiment, the polypeptide of the invention has a sequence which distinguishes from a sequence as indicated in Table II A or II B, columns 5 or 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp., by one or more amino acids. In an other embodiment, said polypeptide of the invention does not consist of the sequence as indicated in Table II A or II B, columns 5 or 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp..
In a further embodiment, said polypeptide of the present invention is less than 100%, 99,999%, 99,99%, 99,9% or 99% identical. In one embodiment, said polypeptide does not consist of the sequence encoded by a nucleic acid molecules as indicated in Table I A or IB, columns 5 or 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp..

**[0294.0.14.14]** In one embodiment, the present invention relates to a polypeptide having an activity of a protein as indicated in Table II, column 3, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp., which distinguishes over a sequence as indicated in Table II A or Table II B, columns 5 or 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp., by one or more amino acids, preferably by more than 5, 6, 7, 8 or 9 amino acids, preferably by more than 10, 15, 20, 25 or 30 amino acids, evenmore preferred are more than 40, 50, or 60 amino acids but even more preferred by less than 70% of the amino acids, more preferred by less than 50%, even more preferred my less than 30% or 25%, more preferred are 20% or 15%, even more preferred are less than 10%.

**[0295.0.0.14] to [0297.0.0.14]: see [0295.0.0.0] to [0297.0.0.0]**

**[0297.1.14.14]** Non-polypeptide of the invention-chemicals are e.g. polypeptides having not the activity of a polypeptide indicated in Table II, columns 3, 5 or 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp..

**[0298.0.14.14]** A polypeptide of the invention can participate in the process of the present invention. The polypeptide or a portion thereof comprises preferably an amino acid sequence which is sufficiently homologous to an amino acid sequence as indicated in Table II, columns 5 or 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp., such that the protein or portion thereof maintains the ability to confer the activity of the present invention. The portion of the protein is preferably a biologically active portion as described herein. Preferably, the polypeptide used in the process of the invention has an amino acid sequence identical to a sequence as indicated in Table II, columns 5 or 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554. and/or lines 167 to 172 and/or 555 to 557, resp..

**[0299.0.14.14]** Further, the polypeptide can have an amino acid sequence which is encoded by a nucleotide sequence which hybridizes, preferably hybridizes under stringent conditions as described above, to a nucleotide sequence of the nucleic acid molecule of the present invention. Accordingly, the polypeptide has an amino acid sequence which is encoded by a nucleotide sequence that is at least about 35%, 40%, 45%, 50%, 55%, 60%, 65% or 70%, preferably at least about 75%, 80%, 85% or 90, and more preferably at least about 91%, 92%, 93%, 94% or 95%, and even more preferably at least about 96%, 97%, 98%, 99% or more homologous to one of the nucleotide sequence as indicated in Table I, columns 5 or 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp..
The preferred polypeptide of the present invention preferably possesses at least one of the activities according to the invention and described herein. A preferred polypeptide of the present invention includes an amino acid sequence encoded by a nucleotide sequence which hybridizes, preferably hybridizes under stringent conditions, to a nucleotide sequence as indicated in Table I, columns 5 or 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp., or which is homologous thereto, as defined above.

**[0300.0.14.14]** Accordingly the polypeptide of the present invention can vary from a sequence as indicated in Table II, columns 5 or 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp., in amino acid sequence due to natural variation or mutagenesis, as described in detail herein. Accordingly, the polypeptide comprise an amino acid sequence which is at least about 35%, 40%, 45%, 50%, 55%, 60%, 65% or 70%, preferably at least about 75%, 80%, 85% or 90, and more preferably at least about 91%, 92%, 93%, 94% or 95%, and most preferably at least about 96%, 97%, 98%, 99% or more homologous to an entire amino acid sequence of a sequence as indicated in Table II A or II B, columns 5 or 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp..

**[0301.0.0.14] see [0301.0.0.0]**

**[0302.0.14.14]** Biologically active portions of an polypeptide of the present invention include peptides comprising amino acid sequences derived from the amino acid sequence of the polypeptide of the present invention or used in the process of the present invention, e.g., an amino acid sequence as indicated in Table II, columns 5 or 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp., or the amino acid sequence of a protein homologous thereto, which include fewer amino acids than a full length polypeptide of the present invention or used in the process of the present invention or the full length protein which is homologous to an polypeptide of the present invention or used in the process of the present invention depicted herein, and exhibit at least one activity of polypeptide of the present invention or used in the process of the present invention.

**[0303.0.0.14] see [0303.0.0.0]**

**[0304.0.14.14]** Manipulation of the nucleic acid molecule of the invention may result in the production of a protein having essentially the activity of the polypeptides as indicated in Table II, column 3, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp., but having differences in the sequence from said wild-type protein. These proteins may be improved in efficiency or activity, may be present in greater numbers in the cell than is usual, or may be decreased in efficiency or activity in relation to the wild type protein.

**[0305.0.0.14] to [0308.0.0.14]: see [0305.0.0.0] to [0308.0.0.0]**

**[0309.0.14.14]** In one embodiment, an reference to a " protein (= polypeptide) " of the invention or as indicated in Table II, columns 5 or 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp., refers to a polypeptide having an amino acid sequence corresponding to the polypeptide of the invention or used in the process of the invention , whereas a "non-polypeptide of the invention" or "other polypeptide" not being indicated in Table II, columns 5 or 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp., refers to a polypeptide having an amino acid sequence corresponding to a protein which is not substantially homologous a polypeptide of the invention, preferably which is not substantially homologous to a as indicated in Table II, columns 5 or 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp., e.g., a protein which does not confer the activity described herein or annotated or known for as indicated in Table II, column 3, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp., and which is derived from the same or a different organism. In one embodmeint, a "non-polypeptide of the invention" or "other polypeptide" not being indicated in Table II, columns 5 or 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp., does not confer an increase of the respective fine chemical in an organism or part thereof.

**[0310.0.0.14] to [0334.0.0.14]: see [0310.0.0.0] to [0334.0.0.0]**

**[0335.0.14.14]** The dsRNAi method has proved to be particularly effective and advantageous for reducing the expression of a nucleic acid sequences as indicated in Table I, columns 5 or 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp., and/or homologs thereof. As described inter alia in WO 99/32619, dsRNAi approaches are clearly superior to traditional antisense approaches. The invention therefore furthermore relates to double-stranded RNA molecules (dsRNA molecules) which, when introduced into an organism, advantageously into a plant (or a cell, tissue, organ or seed derived therefrom), bring about altered metabolic activity by the reduction in the expression of a nucleic acid sequences as indicated in Table I, columns 5 or 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp., and/or homologs thereof. In a double-stranded RNA molecule for reducing the expression of an protein encoded by a nucleic acid sequence of one of the sequences as indicated in Table I, columns 5 or 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp., and/or homologs thereof, one of the two RNA strands is essentially identical to at least part of a nucleic acid sequence, and the respective other RNA strand is essentially identical to at least part of the complementary strand of a nucleic acid sequence.

**[0336.0.0.14] to [0342.0.0.14]: see [0336.0.0.0] to [0342.0.0.0]**

**[0343.0.14.14]** As has already been described, 100 % sequence identity between the dsRNA and a gene transcript of a nucleic acid sequence as indicated in Table I, columns 5 or 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp., or it's homolog is not necessarily required in order to bring about effective reduction in the expression. The advantage is, accordingly, that the method is tolerant with regard to sequence deviations as may be present as a consequence of genetic mutations, polymorphisms or evolutionary divergences. Thus, for example, using the dsRNA, which has been generated starting from a sequence as indicated in Table I, columns 5 or 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp., or homologs thereof of the one organism, may be used to suppress the corresponding expression in another organism.

**[0344.0.0.14] to [0361.0.0.14]: see [0344.0.0.0] to [0361.0.0.0]**

**[0362.0.14.14]** Accordingly the present invention relates to any cell transgenic for any nucleic acid characterized as part of the invention, e.g. conferring the increase of the respective fine chemical in a cell or an organism or a part thereof, e.g. the nucleic acid molecule of the invention, the nucleic acid construct of the invention, the antisense molecule of the invention, the vector of the invention or a nucleic acid molecule encoding the polypeptide of the invention, e.g. the polypeptide as indicated in Table II, columns 5 or 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp., e.g. encoding a polypeptide having protein activity, as indicated in Table II, columns 3, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp..
Due to the above mentioned activity the respective fine chemical content in a cell or an organism is increased. For example, due to modulation or manipulation, the cellular activity of the polypeptide of the invention or nucleic acid molecule of the invention is increased, e.g. due to an increased expression or specific activity of the subject matters of the invention in a cell or an organism or a part thereof. Transgenic for a polypeptide having an activity of a polypeptide as indicated in Table II, columns 5 or 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp., means herein that due to modulation or manipulation of the genome, an activity as annotated for a polypeptide as indicated in Table II, column 3, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp., e.g. having a sequence as indicated in Table II, columns 5 or 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp., is increased in a cell or an organism or a part thereof. Examples are described above in context with the process of the invention

**[0363.0.0.14] to [0384.0.0.14]: see [0363.0.0.0] to [0384.0.0.0]**

**[0385.0.14.14]**The fermentation broths obtained in this way, containing in particular 5-oxoproline, alanine, aspartic acid, citrulline, glycine, homoserine, phenylalanine, serine and/or tyrosine, normally have a dry matter content of from 7.5 to 25% by weight. Sugar-limited fermentation is additionally advantageous, at least at the end, but especially over at least 30% of the fermentation time. This means that the concentration of utilizable sugar in the fermentation medium is kept at, or reduced to, 0 to 3 g/l during this time. The fermentation broth is then processed further. Depending on requirements, the biomass can be removed entirely or partly by separation methods, such as, for example, centrifugation, filtration, decantation or a combination of these methods, from the fermentation broth or left completely in it. The fermentation broth can then be thickened or concentrated by known methods, such as, for example, with the aid of a rotary evaporator, thin-film evaporator, falling film evaporator, by reverse osmosis or by nanofiltration. This concentrated fermentation broth can then be worked up by freeze-drying, spray drying, spray granulation or by other processes. An other method for purification the amino acids of the invention, in particular 5-oxoproline, alanine, aspartic acid, citrulline, glycine, homoserine, phenylalanine, serine and/or tyrosine is a process by means of electrodialysis as described in US 6,551,803.

**[0386.0.0.14] to [0392.0.0.14]: see [0386.0.0.0] to [0392.0.0.0]**

**[0393.0.4.4]** In one embodiment, the present invention relates to a method for the identification of a gene product conferring an increase in the fine chemical production in a cell, comprising the following steps:
i) contacting, e.g. hybridising, the nucleic acid molecules of a sample, e.g. cells, tissues, plants or microorganisms or a nucleic acid library , which can contain a candidate gene encoding a gene product conferring an increase in the fine chemical after expression, with the nucleic acid molecule of the present invention;
j) identifying the nucleic acid molecules, which hybridize under relaxed stringent conditions with the nucleic acid molecule of the present invention in particular to the nucleic acid molecule sequence as indicated in Table I, preferably Table I B, columns 5 or 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp., and, optionally, isolating the full length cDNA clone or complete genomic clone;
k) introducing the candidate nucleic acid molecules in host cells, preferably in a plant cell or a microorganism, appropriate for producing the fine chemical;
I) expressing the identified nucleic acid molecules in the host cells;
m) assaying the the fine chemical level in the host cells; and
n) identifying the nucleic acid molecule and its gene product which expression confers an increase in the the fine chemical level in the host cell after expression compared to the wild type.

**[0394.0.0.14] to [0399.0.0.14]: see [0394.0.0.0] to [0399.0.0.0]**

**[0399.1.14.14]** One can think to screen for increased production of the respective fine chemical by for example searching for a resistance to a drug blocking the synthesis of the fine chemical and looking whether this effect is dependent on the activity or expression of a polypeptide as indicated in Table II, columns 5 or 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp., or a homolog thereof, e.g. comparing the phenotyp of nearly identical organisms with low and high acitivity of a protein as indicated in Table II, columns 5 or 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp., after incubation with the drug.

**[0400.0.0.14] to [0423.0.0.14]: see [0400.0.0.0] to [0423.0.0.0]**

**[0424.0.14.14]**Accordingly, the nucleic acid of the invention, the polypeptide of the invention, the nucleic acid construct of the invention, the organisms, the host cell, the microorgansims, the plant, plant tissue, plant cell, or the part thereof of the invention, the vector of the invention, the agonist identified with the method of the invention, the nucleic acid molecule identified with the method of the present invention, can be used for the production of the fine chemical or of the fine chemical and one or more other amino acids, in particular Threonine, Glutamin, Glutamic acid, Valine, Aspargine, Methionine, Cysteine, Leucine, Proline, Tryptophan , Valine, Isoleucineand, Arginine. Accordingly, the nucleic acid of the invention, or the nucleic acid molecule identified with the method of the present invention or the complement sequences thereof, the polypeptide of the invention, the nucleic acid construct of the invention, the organisms, the host cell, the microorgansims, the plant, plant tissue, plant cell, or the part thereof of the invention, the vector of the invention, the antagonist identified with the method of the invention, the antibody of the present invention, the antisense molecule of the present invention, can be used for the reduction of the respective fine chemical in a organism or part thereof, e.g. in a cell.

**[0425.0.0.14] to [0460.0.0.14]: see [0425.0.0.0] to [0460.0.0.0]**

**[0461.0.14.14]**Example 10: Cloning SEQ ID NO: 18718 for the expression in plants

**[0462.0.0.14] see [0462.0.0.0]**

**[0463.0.14.14]**SEQ ID NO: 18718 is amplified by PCR as described in the protocol of the Pfu Turbo or DNA Herculase polymerase (Stratagene).

**[0464.0.0.14] to [0466.0.0.14]: see [0464.0.0.0] to [0466.0.0.0]**

**[0467.0.14.14]**The following primer sequences were selected for the gene SEQ ID NO: 18718:
i) forward primer (SEQ ID NO: 18874) atgaaataca aggaaatcaa tttcttc
ii) reverse primer (SEQ ID NO: 18875) ctacatagtt atgttattgg tgatcg

**[0468.0.0.14] to [0479.0.0.14]: see [0468.0.0.0] to [0479.0.0.0]**

**[0480.0.14.14]**Example 11: Generation of transgenic plants which express SEQ ID NO: 18718

**[0481.0.0.14] to [0513.0.0.14]: see [0481.0.0.0] to [0513.0.0.0]**

**[0514.0.14.14]**As an alternative, the amino acids can be detected advantageously via HPLC separation in ethanolic extract as described by Geigenberger et al. (Plant Cell & Environ, 19, 1996: 43-55).
The results of the different plant analyses can be seen from the table 1 which follows:

| **ORF** | **Metabolite** | **Method** | **Min** | **Max** |
|---|---|---|---|---|
| YAL049C | Phenylalanine | GC+LC | 1.30 | 1.74 |
| YBL015W | Alanine | GC | 1.29 | 3.04 |
| YDL127W | 5-Oxoproline | GC | 1.40 | 2.27 |
| YEL045C | Serine | GC | 1.23 | 1.80 |
| YEL046C | Homoserine | GC | 1.44 | 2.77 |
| YER152C | Serine | GC | 1.23 | 1.75 |
| YER173W | Alanine | GC | 1.21 | 2.32 |
| YER173W | Aspartic Acid | GC | 1.64 | 1.93 |
| YER173W | Phenylalanine | GC | 1.44 | 1.99 |
| YFL050C | Alanine | GC | 1.19 | 2.04 |
| YFL050C | Glycine | GC | 1.36 | 1.74 |
| YFL050C | Tyrosine | GC | 1.42 | 1.56 |
| YGR101W | Phenylalanine | GC | 1.40 | 1.92 |
| YGR104C | Aspartic Acid | GC | 2.08 | 2.26 |
| YHR130C | Phenylalanine | LC | 1.77 | 1.77 |
| YHR130C | Tyrosine | GC+LC | 1.51 | 2.26 |
| YIL150C | Alanine | GC | 1.47 | 4.28 |
| YIL150C | Serine | GC | 1.30 | 4.22 |
| YIL150C | Aspartic Acid | GC | 4.08 | 4.08 |
| YIL150C | Tyrosine | GC | 7.98 | 7.98 |
| YJL072C | Phenylalanine | GC | 1.25 | 3.04 |
| YKR057W | Serine | GC | 1.24 | 2.70 |
| YLL009C | Serine | GC | 1.27 | 1.84 |
| YLL013C | Citrulline | LC | 1.30 | 1.44 |
| YLR082C | Serine | GC | 1.22 | 1.61 |
| YOL123W | Glycine | GC | 1.35 | 2.05 |
| YOR245C | Citrulline | LC | 1.47 | 1.69 |
| YOR261C | Serine | GC | 1.28 | 1.40 |
| YOR350C | Tyrosine | GC | 1.67 | 2.77 |
| YPR138C | Phenylalanine | GC+LC | 1.49 | 1.93 |
| b0695 | Phenylalanine | GC+LC | 1.36 | 1.74 |
| b0730 | Aspartic Acid | GC | 1.47 | 3.68 |
| b1708 | Phenylalanine | GC | 1.48 | 3.37 |
| b1827 | Phenylalanine | GC | 1.40 | 3.93 |
| b1827 | Tyrosine | GC | 1.40 | 3.28 |
| b1829 | Serine | GC | 1.24 | 2.66 |
| b1829 | Tyrosine | GC+LC | 1.56 | 8.41 |
| b2095 | Alanine | GC | 1.22 | 1.33 |
| b3008 | Alanine | GC | 1.27 | 1.71 |
| b3256 | Phenylalanine | GC | 1.44 | 1.50 |
| b1697 | Phenylalanine | GC | 1.30 | 2.88 |
| b1886 | Phenylalanine | LC | 1.33 | 2.96 |
| b1886 | Serine | GC | 1.25 | 2.11 |
| b1896 | 5-Oxoproline | GC | 1.34 | 2.50 |
| b1896 | Aspartic acid | GC | 1.90 | 3.55 |
| b3462 | Serine | GC | 1.26 | 1.64 |
| b3462 | Phenylalanine | GC+LC | 1.26 | 2.40 |
| b0057 | Citrulline | LC | 1.33 | 2.19 |
| b0057 | Glycine | GC | 1.43 | 1.67 |
| b0057 | Serine 1 | GC | 1.32 | 1.61 |
| b0161 | 5-Oxoproline | GC | 1.45 | 2.23 |
| b0161 | Aspartic acid | GC | 1.57 | 2.16 |
| b0161 | Phenylalanine | LC + GC | 1.37 | 7.68 |
| b0236 | Alanine | GC | 1.22 | 1.41 |
| b0376 | 5-Oxoproline | GC | 1.41 | 2.00 |
| b0462 | Citrulline | LC | 1.36 | 1.44 |
| b0486 | Alanine | GC | 1.44 | 1.52 |
| b0486 | Serine | GC | 1.27 | 1.49 |
| b0577 | Glycine | GC | 1.36 | 1.50 |
| b0577 | Aspartic acid | GC | 1.56 | 1.65 |
| b0970 | 5-Oxoproline | GC | 1.37 | 3.03 |
| b0970 | Tyrosine | GC | 1.35 | 5.98 |
| b1228 | Phenylalanine | GC | 1.35 | 1.44 |
| b1275 | Citrulline | LC | 1.32 | 1.50 |
| b1343 | Alanine | GC | 1.27 | 1.44 |
| b1343 | 5-Oxoproline | GC | 1.37 | 1.87 |
| b1343 | Aspartic acid | GC | 1.76 | 2.36 |
| b1360 | Citrulline | LC | 1.34 | 1.72 |
| b1863 | Alanine | GC | 1.22 | 1.45 |
| b2023 | Aspartic acid | GC | 1.56 | 1.81 |
| b2078 | Phenylalanine | LC + GC | 1.26 | 1.89 |
| b2239 | Citrulline | LC | 1.37 | 1.73 |
| b2414 | Citrulline | LC | 1.31 | 1.40 |
| b2414 | Glycine | GC | 1.38 | 1.62 |
| b2414 | Serine | GC | 1.27 | 1.53 |
| b2414 | Phenylalanine | GC | 1.27 | 2.97 |
| b2426 | Citrulline | LC | 1.32 | 1.41 |
| b2489 | Citrulline | LC | 1.33 | 1.60 |
| b2489 | Alanine | GC | 1.21 | 1.27 |
| b2489 | Glycine | GC | 1.33 | 1.78 |
| b2489 | Serine | GC | 1.23 | 1.47 |
| b2491 | Tyrosine | GC | 1.40 | 1.89 |
| b2507 | Aspartic acid | GC | 1.49 | 2.20 |
| b2553 | Glycine | GC | 1.36 | 1.83 |
| b2576 | Alanine | GC | 1.24 | 1.49 |
| b2576 | Glycine | GC | 1.40 | 1.73 |
| b2576 | Aspartic acid | GC | 1.52 | 2.03 |
| b2576 | Phenylalanine | GC | 1.23 | 1.41 |
| b2664 | Serine | GC | 1.25 | 3.81 |
| b2753 | Aspartic acid | GC | 1.56 | 2.54 |
| b2796 | Phenylalanine | LC | 1.33 | 2.05 |
| b3064 | Serine | GC | 1.39 | 1.84 |
| b3116 | Serine | GC | 1.35 | 1.70 |
| b3116 | Aspartic acid | GC | 1.50 | 2.30 |
| b3160 | Citrulline | LC | 1.34 | 1.48 |
| b3160 | Serine | GC | 1.31 | 1.60 |
| b3169 | Aspartic acid | GC | 1.66 | 2.68 |
| b3172 | 5-Oxoproline | GC | 1.44 | 1.94 |
| b3172 | Aspartic acid | GC | 1.52 | 3.34 |
| b3231 | Alanine | GC | 1.21 | 1.35 |
| b3231 | Serine | GC | 1.23 | 1.49 |
| b3241 | Citrulline | LC | 1.38 | 1.70 |
| b3767 | Alanine | GC | 1.24 | 1.70 |
| b3767 | Homoserine | GC | 1.24 | 1.71 |
| b3919 | Phenylalanine | GC | 1.30 | 2.88 |
| b3926 | Citrulline | LC | 1.70 | 1.94 |
| b3938 | Phenylalanine | LC + GC | 1.34 | 1.40 |
| b3983 | Phenylalanine | LC + GC | 1.37 | 3.66 |
| b3983 | Tyrosine | LC + GC | 1.44 | 4.57 |
| b4129 | Aspartic acid | GC | 1.53 | 2.41 |
| b4214 | Citrulline | LC | 1.32 | 2.02 |
| b4269 | Citrulline | LC | 1.31 | 1.63 |
| b4346 | Aspartic acid | GC | 1.63 | 1.94 |

**[0515.0.14.14]**Column 2 shows the amino acid analyzed. Columns 4 and 5 shows the ratio of the analyzed amino acid between the transgenic plants and the wild type; Increase of the metabolites: Max: maximal x-fold (normalised to wild type)-Min: minimal x-fold (normalised to wild type). Decrease of the metabolites: Max: maximal x-fold (normalised to wild type) (minimal decrease), Min: minimal x-fold (normalised to wild type) (maximal decrease). Column 6 indicates the analytical method.

**[0516.0.0.14] to [0552.0.0.14]: see [0516.0.0.0] to [0552.0.0.0]**

**[0552.1.14.14]:** Example 15: Metabolite Profiling Info from *Zea mays*
*Zea mays* plants were engineered as described in Example 14c.
The results of the different *Zea mays* plants analysed can be seen from table 2 which follows:

**Table 2**

| ORF_NAME | Metabolite | Min | Max |
|---|---|---|---|
| YAL049C | Phenylalanine | 1.78 | 1.86 |
| YIL150C | Alanine | 1.63 | 3.17 |
| YIL150C | Serine | 1.25 | 2.02 |
| YKR057W | Serine | 2.05 | 12.64 |
| YIL150C | Aspartic acid | 2.11 | 3.56 |
| YIL150C | Tyrosine | 1.19 | 2.09 |

Table 2 describes the increase in phenylalanine and alanine and serine and aspartic acid and tyrosine in genetically modified corn plants expressing the Saccharomyces cerevisiae nucleic acid sequence YAL049C or YIL150C or YKR057W resp..
In one embodiment, in case the activity of the Saccharomyces cerevisiae protein YAL049C or its homologs, its activity has not been characterized yet, is increased in corn plants, preferably, an increase of the fine chemical phenylalanine between 78% and 86% is conferred.
In one embodiment, in case the activity of the Saccaromyces cerevisiae protein YIL150C or its homologs, e.g. "a chromatin binding protein, required for S-phase (DNA synthesis) initiation or completion" or its homologs, is increased in corn plants, preferably, an increase of the fine chemical alanine between 63% and 217% is conferred and/or an increase of the fine chemical serine between 25% and 102% is conferred and/or an increase of the fine chemical aspartic acid between 111 % and 256% is conferred and/or an increase of the fine chemical tyrosine between 19% and 109% is conferred.
In one embodiment, in case the activity of the Saccharomyces cerevisiae protein YKR057W or a ribosomal protein, similar to S21 ribosomal proteins, involved in ribosome biogenesis and translation or its homologs, is increased in corn plants, preferably, an increase of the fine chemical serine between 105% and 1164% is conferred.

**[0552.2.14.14]: see [0552.2.0.0]**

**[0553.0.14.14]**
1. A process for the production of 5-oxoproline, alanine, aspartic acid, citrulline, glycine, homoserine, phenylalanine, serine and/or tyrosine resp., which comprises
   (a) increasing or generating the activity of a protein as indicated in Table II, columns 5 or 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp., or a functional equivalent thereof in a non-human organism, or in one or more parts thereof; and
   (b) growing the organism under conditions which permit the production of 5-oxoproline, alanine, aspartic acid, citrulline, glycine, homoserine, phenylalanine, serine and/or tyrosine resp. in said organism.
2. A process for the production of 5-oxoproline, alanine, aspartic acid, citrulline, glycine, homoserine, phenylalanine, serine and/or tyrosine resp., comprising the increasing or generating in an organism or a part thereof the expression of at least one nucleic acid molecule comprising a nucleic acid molecule selected from the group consisting of:
   a) nucleic acid molecule encoding of a polypeptide as indicated in Table II, columns 5 or 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp., or a fragment thereof, which confers an increase in the amount of 5-oxoproline, alanine, aspartic acid, citrulline, glycine, homoserine, phenylalanine, serine and/or tyrosine resp., in an organism or a part thereof;
   b) nucleic acid molecule comprising of a nucleic acid molecule as indicated in Table I, columns 5 or 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp.,
   c) nucleic acid molecule whose sequence can be deduced from a polypeptide sequence encoded by a nucleic acid molecule of (a) or (b) as a result of the degeneracy of the genetic code and conferring an increase in the amount of 5-oxoproline, alanine, aspartic acid, citrulline, glycine, homoserine, phenylalanine, serine and/or tyrosine resp., in an organism or a part thereof;
   d) nucleic acid molecule which encodes a polypeptide which has at least 50% identity with the amino acid sequence of the polypeptide encoded by the nucleic acid molecule of (a) to (c) and conferring an increase in the amount of 5-oxoproline, alanine, aspartic acid, citrulline, glycine, homoserine, phenylalanine, serine and/or tyrosine resp., in an organism or a part thereof;
   e) nucleic acid molecule which hybidizes with a nucleic acid molecule of (a) to (c) under under stringent hybridisation conditions and conferring an increase in the amount of 5-oxoproline, alanine, aspartic acid, citrulline, glycine, homoserine, phenylalanine, serine and/or tyrosine resp., in an organism or a part thereof;
   f) nucleic acid molecule which encompasses a nucleic acid molecule which is obtained by amplifying nucleic acid molecules from a cDNA library or a genomic library using the primers or primer pairs as indicated in Table III, column 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp., and conferring an increase in the amount of the 5-oxoproline, alanine, aspartic acid, citrulline, glycine, homoserine, phenylalanine, serine and/or tyrosine resp., in an organism or a part thereof;
   g) nucleic acid molecule encoding a polypeptide which is isolated with the aid of monoclonal antibodies against a polypeptide encoded by one of the nucleic acid molecules of (a) to (f) and conferring an increase in the amount of 5-oxoproline, alanine, aspartic acid, citrulline, glycine, homoserine, phenylalanine, serine and/or tyrosine resp., in an organism or a part thereof;
   h) nucleic acid molecule encoding a polypeptide comprising a consensus sequence as indicated in Table IV, column 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp., and conferring an increase in the amount of 5-oxoproline, alanine, aspartic acid, citrulline, glycine, homoserine, phenylalanine, serine and/or tyrosine resp., in an organism or a part thereof; and
   i) nucleic acid molecule which is obtainable by screening a suitable nucleic acid library under stringent hybridization conditions with a probe comprising one of the sequences of the nucleic acid molecule of (a) to (k) or with a fragment thereof having at least 15 nt, preferably 20 nt, 30 nt, 50 nt, 100 nt, 200 nt or 500 nt of the nucleic acid molecule characterized in (a) to (k) and conferring an increase in the amount of 5-oxoproline, alanine, aspartic acid, citrulline, glycine, homoserine, phenylalanine, serine and/or tyrosine resp., in an organism or a part thereof.
   or comprising a sequence which is complementary thereto.
3. The process of claim 1 or 2, comprising recovering of the free or bound 5-oxoproline, alanine, aspartic acid, citrulline, glycine, homoserine, phenylalanine, serine and/or tyrosine resp..
4. The process of any one of claim 1 to 3, comprising the following steps:
   (a) selecting an organism or a part thereof expressing a polypeptide encoded by the nucleic acid molecule characterized in claim 2;
   (b) mutagenizing the selected organism or the part thereof;
   (c) comparing the activity or the expression level of said polypeptide in the mutagenized organism or the part thereof with the activity or the expression of said polypeptide of the selected organisms or the part thereof;
   (d) selecting the mutated organisms or parts thereof, which comprise an increased activity or expression level of said polypeptide compared to the selected organism or the part thereof;
   (e) optionally, growing and cultivating the organisms or the parts thereof; and
   (f) recovering, and optionally isolating, the free or bound 5-oxoproline, alanine, aspartic acid, citrulline, glycine, homoserine, phenylalanine, serine and/or tyrosine resp., produced by the selected mutated organisms or parts thereof.
5. The process of any one of claims 1 to 4, wherein the activity of said protein or the expression of said nucleic acid molecule is increased or generated transiently or stably.
6. An isolated nucleic acid molecule comprising a nucleic acid molecule selected from the group consisting of:
   a) nucleic acid molecule encoding of a polypeptide as indicated in Table II, columns 5 or 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp., or a fragment thereof, which confers an increase in the amount of 5-oxoproline, alanine, aspartic acid, citrulline, glycine, homoserine, phenylalanine, serine and/or tyrosine resp., in an organism or a part thereof;
   b) nucleic acid molecule comprising of a nucleic acid molecule as indicated in Table I, columns 5 or 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp.,
   c) nucleic acid molecule whose sequence can be deduced from a polypeptide sequence encoded by a nucleic acid molecule of (a) or (b) as a result of the degeneracy of the genetic code and conferring an increase in the amount of 5-oxoproline, alanine, aspartic acid, citrulline, glycine, homoserine, phenylalanine, serine and/or tyrosine resp., in an organism or a part thereof;
   d) nucleic acid molecule which encodes a polypeptide which has at least 50% identity with the amino acid sequence of the polypeptide encoded by the nucleic acid molecule of (a) to (c) and conferring an increase in the amount of 5-oxoproline, alanine, aspartic acid, citrulline, glycine, homoserine, phenylalanine, serine and/or tyrosine resp., in an organism or a part thereof;
   e) nucleic acid molecule which hybidizes with a nucleic acid molecule of (a) to (c) under under stringent hybridisation conditions and conferring an increase in the amount of 5-oxoproline, alanine, aspartic acid, citrulline, glycine, homoserine, phenylalanine, serine and/or tyrosine resp., in an organism or a part thereof;
   f) nucleic acid molecule which encompasses a nucleic acid molecule which is obtained by amplifying nucleic acid molecules from a cDNA library or a genomic library using the primers or primer pairs as indicated in Table III, column 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp., and conferring an increase in the amount of 5-oxoproline, alanine, aspartic acid, citrulline, glycine, homoserine, phenylalanine, serine and/or tyrosine resp., in an organism or a part thereof;
   g) nucleic acid molecule encoding a polypeptide which is isolated with the aid of monoclonal antibodies against a polypeptide encoded by one of the nucleic acid molecules of (a) to (f) and conferring an increase in the amount of 5-oxoproline, alanine, aspartic acid, citrulline, glycine, homoserine, phenylalanine, serine and/or tyrosine resp., in an organism or a part thereof;
   h) nucleic acid molecule encoding a polypeptide comprising a consensus sequence as indicated in Table IV, column 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp., and conferring an increase in the amount of 5-oxoproline, alanine, aspartic acid, citrulline, glycine, homoserine, phenylalanine, serine and/or tyrosine resp., in an organism or a part thereof; and
   i) nucleic acid molecule which is obtainable by screening a suitable nucleic acid library under stringent hybridization conditions with a probe comprising one of the sequences of the nucleic acid molecule of (a) to (k) or with a fragment thereof having at least 15 nt, preferably 20 nt, 30 nt, 50 nt, 100 nt, 200 nt or 500 nt of the nucleic acid molecule characterized in (a) to (k) and conferring an increase in the amount of 5-oxoproline, alanine, aspartic acid, citrulline, glycine, homoserine, phenylalanine, serine and/or tyrosine resp., in an organism or a part thereof.
   whereby the nucleic acid molecule distinguishes over the sequence as indicated in Table I A, columns 5 or 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp., by one or more nucleotides.
7. A nucleic acid construct which confers the expression of the nucleic acid molecule of claim 6, comprising one or more regulatory elements.
8. A vector comprising the nucleic acid molecule as claimed in claim 6 or the nucleic acid construct of claim 7.
9. The vector as claimed in claim 8, wherein the nucleic acid molecule is in operable linkage with regulatory sequences for the expression in a prokaryotic or eukaryotic, or in a prokaryotic and eukaryotic, host.
10. A host cell, which has been transformed stably or transiently with the vector as claimed in claim 8 or 9 or the nucleic acid molecule as claimed in claim 6 or the nucleic acid construct of claim 7 or produced as described in claim any one of claims 2 to 5.
11. The host cell of claim 10, which is a transgenic host cell.
12. The host cell of claim 10 or 11, which is a plant cell, an animal cell, a microorganism, or a yeast cell, a fungus cell, a prokaryotic cell, an eukaryotic cell or an archaebacterium.
13. A process for producing a polypeptide, wherein the polypeptide is expressed in a host cell as claimed in any one of claims 10 to 12.
14. A polypeptide produced by the process as claimed in claim 13 or encoded by the nucleic acid molecule as claimed in claim 6 whereby the polypeptide distinguishes over a sequence as indicated in Table II A, columns 5 or 7, lines 126 to 127 and/or 492 to 496 and/or lines 128 to 133 and/or 497 to 504 and/or lines 134 to 138 and/or 505 to 516 and/or lines 139 to 140 and/or 517 to 529 and/or lines 141 to 142 and/or 530 to 535 and/or lines 143 and/or 536 and/or lines 144 to 156 and/or 537 to 545 and/or lines 157 to 166 and/or 546 to 554 and/or lines 167 to 172 and/or 555 to 557, resp., by one or more amino acids
15. An antibody, which binds specifically to the polypeptide as claimed in claim 14.
16. A plant tissue, propagation material, harvested material or a plant comprising the host cell as claimed in claim 12 which is plant cell or an Agrobacterium.
17. A method for screening for agonists and antagonists of the activity of a polypeptide encoded by the nucleic acid molecule of claim 6 conferring an increase in the amount of 5-oxoproline, alanine, aspartic acid, citrulline, glycine, homoserine, phenylalanine, serine and/or tyrosine resp., in an organism or a part thereof comprising:
   (a) contacting cells, tissues , plants or microorganisms which express the a polypeptide encoded by the nucleic acid molecule of claim 5 conferring an increase in the amount of 5-oxoproline, alanine, aspartic acid, citrulline, glycine, homoserine, phenylalanine, serine and/or tyrosine resp., in an organism or a part thereof with a candidate compound or a sample comprising a plurality of compounds under conditions which permit the expression the polypeptide;
   (b) assaying the 5-oxoproline, alanine, aspartic acid, citrulline, glycine, homoserine, phenylalanine, serine and/or tyrosine resp., level or the polypeptide expression level in the cell, tissue, plant or microorganism or the media the cell, tissue, plant or microorganisms is cultured or maintained in; and
   (c) identifying a agonist or antagonist by comparing the measured 5-oxoproline, alanine, aspartic acid, citrulline, glycine, homoserine, phenylalanine, serine and/or tyrosine resp., level or polypeptide expression level with a standard 5-oxoproline, alanine, aspartic acid, citrulline, glycine, homoserine, phenylalanine, serine and/or tyrosine resp., or polypeptide expression level measured in the absence of said candidate compound or a sample comprising said plurality of compounds, whereby an increased level over the standard indicates that the compound or the sample comprising said plurality of compounds is an agonist and a decreased level over the standard indicates that the compound or the sample comprising said plurality of compounds is an antagonist.
18. A process for the identification of a compound conferring increased 5-oxoproline, alanine, aspartic acid, citrulline, glycine, homoserine, phenylalanine, serine and/or tyrosine resp., production in a plant or microorganism, comprising the steps:
   (a) culturing a plant cell or tissue or microorganism or maintaining a plant expressing the polypeptide encoded by the nucleic acid molecule of claim 6 conferring an increase in the amount of 5-oxoproline, alanine, aspartic acid, citrulline, glycine, homoserine, phenylalanine, serine and/or tyrosine resp., in an organism or a part thereof and a readout system capable of interacting with the polypeptide under suitable conditions which permit the interaction of the polypeptide with dais readout system in the presence of a compound or a sample comprising a plurality of compounds and capable of providing a detectable signal in response to the binding of a compound to said polypeptide under conditions which permit the expression of said readout system and of the polypeptide encoded by the nucleic acid molecule of claim 6 conferring an increase in the amount of 5-oxoproline, alanine, aspartic acid, citrulline, glycine, homoserine, phenylalanine, serine and/or tyrosine rersp., in an organism or a part thereof;
   (b) identifying if the compound is an effective agonist by detecting the presence or absence or increase of a signal produced by said readout system.
19. A method for the identification of a gene product conferring an increase in 5-oxoproline, alanine, aspartic acid, citrulline, glycine, homoserine, phenylalanine, serine and/or tyrosine resp., production in a cell, comprising the following steps:
   (a) contacting the nucleic acid molecules of a sample, which can contain a candidate gene encoding a gene product conferring an increase in 5-oxoproline, alanine, aspartic acid, citrulline, glycine, homoserine, phenylalanine, serine and/or tyrosine resp., after expression with the nucleic acid molecule of claim 6;
   (b) identifying the nucleic acid molecules, which hybridise under relaxed stringent conditions with the nucleic acid molecule of claim 6;
   (c) introducing the candidate nucleic acid molecules in host cells appropriate for producing 5-oxoproline, alanine, aspartic acid, citrulline, glycine, homoserine, phenylalanine, serine and/or tyrosine resp.;
   (d) expressing the identified nucleic acid molecules in the host cells;
   (e) assaying the 5-oxoproline, alanine, aspartic acid, citrulline, glycine, homoserine, phenylalanine, serine and/or tyrosine rersp., level in the host cells; and
   (f) identifying nucleic acid molecule and its gene product which expression confers an increase in the 5-oxoproline, alanine, aspartic acid, citrulline, glycine, homoserine, phenylalanine, serine and/or tyrosine resp., level in the host cell in the host cell after expression compared to the wild type.
20. A method for the identification of a gene product conferring an increase in 5-oxoproline, alanine, aspartic acid, citrulline, glycine, homoserine, phenylalanine, serine and/or tyrosine resp., production in a cell, comprising the following steps:
   (a) identifiying in a data bank nucleic acid molecules of an organism; which can contain a candidate gene encoding a gene product conferring an increase in the 5-oxoproline, alanine, aspartic acid, citrulline, glycine, homoserine, phenylalanine, serine and/or tyrosine resp., amount or level in an organism or a part thereof after expression, and which are at least 20% homolog to the nucleic acid molecule of claim 6;
   (b) introducing the candidate nucleic acid molecules in host cells appropriate for producing 5-oxoproline, alanine, aspartic acid, citrulline, glycine, homoserine, phenylalanine, serine and/or tyrosine resp.,;
   (c) expressing the identified nucleic acid molecules in the host cells;
   (d) assaying the 5-oxoproline, alanine, aspartic acid, citrulline, glycine, homoserine, phenylalanine, serine and/or tyrosineresp., level in the host cells; and
   (e) identifying nucleic acid molecule and its gene product which expression confers an increase in the 5-oxoproline, alanine, aspartic acid, citrulline, glycine, homoserine, phenylalanine, serine and/or tyrosine resp., level in the host cell after expression compared to the wild type.
21. A method for the production of an agricultural composition comprising the steps of the method of any one of claims 17 to 20 and formulating the compound identified in any one of claims 17 to 20 in a form acceptable for an application in agriculture.
22. A composition comprising the nucleic acid molecule of claim 6, the polypeptide of claim 14, the nucleic acid construct of claim 7, the vector of any one of claims 8 or 9, an antagonist or agonist identified according to claim 17, the compound of claim 18, the gene product of claim 19 or 20, the antibody of claim 15, and optionally an agricultural acceptable carrier.
23. Use of the nucleic acid molecule as claimed in claim 6 for the identification of a nucleic acid molecule conferring an increase of 5-oxoproline, alanine, aspartic acid, citrulline, glycine, homoserine, phenylalanine, serine and/or tyrosine resp., after expression.
24. Use of the polypeptide of claim 14 or the nucleic acid construct claim 7 or the gene product identified according to the method of claim 19 or 20 for identifying compounds capable of conferring a modulation of 5-oxoproline, alanine, aspartic acid, citrulline, glycine, homoserine, phenylalanine, serine and/or tyrosine resp., levels in an organism.
25. Food or feed composition comprising the nucleic acid molecule of claim 6, the polypeptide of claim 14, the nucleic acid construct of claim 7, the vector of claim 8 or 9, the antagonist or agonist identified according to claim 17, the antibody of claim 15, the plant or plant tissue of claim 16, the harvested material of claim 16, the host cell of claim 10 to 12 or the gene product identified according to the method of claim 19 or 20.
26. Use of the nucleic acid molecule of claim 6, the polypeptide of claim 14, the nucleic acid construct of claim 7, the vector of claim 8 or 9, the antagonist or agonist identified according to claim 17, the antibody of claim 15, the plant or plant tissue of claim 16, the harvested material of claim 16, the host cell of claim 10 to 12 or the gene product identified according to the method of claim 19 or 20 for the protection of a plant against a 5-oxoproline and/or alanine and/or aspartic acid and/or citrulline and/or glycine and/or homoserine and/or phenylalanine and/or serine and/or tyrosine synthesis inhibiting herbicide.

**[0554.0.0.14] Abstract: see [0554.0.0.0]**
Process for the production of fine chemicals

**[0000.0.0.15]** In a further embodiment, the present invention relates to a further process for the production of fine chemicals as defined below and the corresponding embodiments as described herein as follows.

**[0001.0.0.15] see [0001.0.0.0]**

**[0002.0.15.15]** Oils and fats, which chemically are glycerol esters of fatty acids (triacylglycerols (TAGs)), play a major role in nutrition but more and more in nonfood applications such as lubricants, hydraulic oil, biofuel, or oleochemicals for coatings, plasticizer, soaps, and detergents (W. Lohs and W. Friedt, in Designer Oil Crops, D. J. Murphy, Ed. (VCH, Weinheirn, Germany, 1993)). The ideal oil for industrial application would consist of a particular type of fatty acid that could be supplied constantly at a competitively low price as compared with raw materials based on mineral oil products. Furthermore, such a fatty acid may have a reactive group in addition to the carboxyl function to provide an additional target for chemical modifications (Töpfer et al., Science, Vol. 268, 681-686, 1995).

**[0003.0.15.15]** Fatty acids and triglycerides have numerous applications in the food and feed industry, in cosmetics and in the drug sector. Depending on whether they are free saturated or unsaturated fatty acids or triglycerides with an increased content of saturated or unsaturated fatty acids, they are suitable for the most varied applications; thus, for example, polyunsaturated fatty acids (= PUFAs) are added to infant formula to increase its nutritional value. The various fatty acids and triglycerides are mainly obtained from microorganisms such as fungi or from oil-producing plants including phytoplankton and algae, such as soybean, oilseed rape, sunflower and others, where they are usually obtained in the form of their triacylglycerides.

**[0004.0.15.15]** Principally microorganisms such as Mortierella or oil producing plants such as soybean, rapeseed or sunflower or algae such as Crytocodinium or Phaeodactylum are a common source for oils containing fatty acids, where they are usually obtained in the form of their triacyl glycerides. Alternatively, they are obtained advantageously from animals, such as fish. The free fatty acids are prepared advantageously by hydrolysis with a strong base such as potassium or sodium hydroxide.

**[0005.0.15.15]** Further sources of fatty acids are membrane lipids of organisms. Preferably lipids are phopholipids and/or glycolipids, more preferably glycerophospholipids, galactolipids and/or sphingolipids.

**[0006.0.15.15.]** Margaric acid was first mentioned in the early 1800s. 1813 M. E. Chevreul discovered that fats are composed of fatty acids and named one of these "margaric acid" because it glistened with lustrous pearly drops that reminded him of the Greek word for pearl, margaron or margarites. In the middle of the 1800s W. H. Heintz showed that "margaric acid" discovered by Chevreul was an indefinite mixture of palmitic and stearic acids.
Today, the term "margaric acid" is the trivial name for heptadecanoic acid (17:0), which is naturally occurring in minor amounts.
The fatty acid with odd number of carbon atoms is present in trace amounts in plants, in triglycerides from Brazil-nut oil, Dracocephalum moldavica oil, Poppy-seed, Palm, Almond, Sunflower or Soyabean. Margaric acid can be isolated from tallow (1%), specially from subcutaneous adipose tissue in subcutaneous fat from lambs.
Margaric acid can be ingredient of satiety agents or fungicide composition. It is further used as ingredient in cosmetics, pharmaceuticals and in feed and food, like baking adjuvants as disclosed in US 20030143312 or accordind to US 20040097392 as component in surfactant systems.
The heptadecanoic acid is mainly used as an internal standard in quantification of fatty acids. It can be further useful in treatment of neurological diseases which may be caused by yeast, fungi or prions based on yeast or fungal etiology (US 6,652,866) or in antikeratolytic-wound healing compositions (US 5,641,814).Heptadecanoic acid was produced up to now in higher amount primarily by organic synthesis.

**[0007.0.15.15.]** 2-Hydroxy fatty acids are synthesised in animal and plant tissues, and are often major constituents of the sphingolipids. Sphingolipids with 2-hydroxy fatty acid are found in most organisms including plants, yeast, worms, vertebrate animals, and some bacterial species.
In plants more than 95% of the fatty acid component of the ceramides and sphingolipids is alpha-hydroxylated. The acyl groups of ceramides tend to consist of long-chain (C16 up to C26 but occasionally longer) odd- and even-numbered saturated or monoenoic fatty acids and related 2-*D*-hydroxy fatty acids, both in plant and animal tissues.
Typical plant sphingolipids are made up by the long-chain sphingosine backbone which is glycosylated and amide-linked to an usually hydroxylated (very-)-long-chain fatty acid, called cerebroside. Cerebrosides are essential constituents of the plasma membrane involved in various physiological functions including signaling, exocytosis, anchoring of proteins, and vesicular protein transport (Matthes et al., Z. Naturforsch. 57C, 843-852, 2002).
In mammals, 2-hydroxysphingolipids are present abundantly in brain because the major myelin lipids galactosylceramides and sulfatides contain 2-hydroxy fatty acids. In mammals, 2-hydroxy fatty acid-containing sphingolipids are uniquely abundant in nervous and epidermal tissues. In mammalian central and peripheral nervous systems, galactosylceramides and sulfatides (3-sulfate ester of galactosylceramide) are major lipid components of myelin. These glycosphingolipids contain a high proportion ( about 50%) of 2-hydroxy fatty acid and are critical components of myelin (4, 5).
In the yeast *Saccharomyces cerevisiae* most sphingolipids contain 2-hydroxy fatty acid. COS7 cells expressing human FA2H contained 3-20-fold higher levels of 2-hydroxyceramides (C16, C18, C24, and C24:1) and 2-hydroxy fatty acids compared with control cells (Alderson et al., J. Biol. Chem. Vol. 279, No. 47, 48562-48568, 2004).
The 2-hydroxylation occurs during *de novo* ceramide synthesis and is catalyzed by fatty acid 2-hydroxylase (also known as fatty acid alpha-hydroxylase). No free hydroxy fatty acid or hydroxy fatty acid CoA has ever been reported; the hydroxylated product always appeared as a component of ceramide or cerebroside (Hoshi et al., J. Biol. Chem. 248, 4123-4130, 1973). The alpha-hydroxylation involves the direct hydroxylation of a sphingolipid-bound fatty acid. ( Kayal et al., J. Biol. Chem. Vol. 259, No. 6, 3548-3553, 1984).
Hydroxylated fatty acids initiate inflammation in the soft tissues and regulate the immune response.
The 2-hydroxyl group in sphingolipids has a profound effect in the lipid organization within membranes because of its hydrogenbonding capability.
Alpha-hydroxy-palmitic acid (hC 16:0) is mainly a building block of plant sphingolipids, for example soy glucosylceramide (GlcCer), which consists predominantly of a 4,8-sphingadiene backbone and alpha-hydroxy-palmitic acid. Soy GlcCer suppress tumorigenesis and gene expression in mice (Symolon et al., J. Nutr. 2004 May;134(5):1157-61).
A monoglucosecerebroside (pinelloside) with strong antimicrobial properties (against Gram-positive and -negative bacteria and against fungi) was described in the tuber of *Pinella ternata* (*Araceae*), one component of decoctions used in traditional Chinese medicine (Chen et al., Phytochemistry 2003, 64, 903). Its structure was shown to include a glucose moiety and the unusual 4,11-sphingadienine linked to a 2-hydroxy-palmitic acid.
Another hydroxylated fatty acid being a building block of cerrebrosides is the 2-hydroxy-nervonic acid (2-OH-C24:1). 2-hydroxy-15-tetracosenoic acid (hydroxynervonic acid) is constituent of the ceramide part of cerebrosides (glycosphingolipides found mainly in nervous tissue and in little amount in plants). The occurrence of 2-hydroxy nervonic acid is characteristic for the leaf cerebrosides of some chilling-resistant cereals (Sperling et al., BBA 1632, 1-15, 2003).
The hydroxylated fatty acid may be used in a method for producing fats or oils according to US 20030054509 or in lanolin-free cosmetic composition according to US 20040166130.

**[0008.0.15.15]** Whether oils with unsaturated or with saturated fatty acids are preferred depends on the intended purpose; thus, for example, lipids with unsaturated fatty acids, specifically polyunsaturated fatty acids, are preferred in human nutrition since they have a positive effect on the cholesterol level in the blood and thus on the possibility of heart disease. They are used in a variety of dietetic foodstuffs or medicaments. In addition PUFAs are commonly used in food, feed and in the cosmetic industry. Poly unsaturated ω-3- and/or ω-6-fatty acids are an important part of animal feed and human food. Because of the common composition of human food polyunsaturated ω-3-fatty acids, which are an essential component of fish oil, should be added to the food to increase the nutritional value of the food; thus, for example, polyunsaturated fatty acids such as DHA or EPA are added as mentioned above to infant formula to increase its nutritional value. The true essential fatty acids linoleic and linolenic fatty acid have a lot of positive effects in the human body such as a positive effect on healthy heart, arteries and skin. They bring for example relieve from eczema, diabetic neuropathy or PMS and cyclical breast pain.

**[0009.0.15.15.]** Further poly unsaturated w-3- and/or ω-6-fatty acids important part of animal feed and human food are delta 7, 10 hexadecadienic acid (16:2(n-6))'and delta 7, 10, 13 hexadecatrienic acid (16:3(n-3)). Hexadecadienic acid is a minor component of some seed and fish oils, and of plant leaves but the precursor of hexadecatrienic acid 16:3(n-3), which is a common constituent of leaf lipids. This acid is known to occur in photosynthetic leaves, such as for example Arabidopsis thaliana, rape leaves, fern lipid, ginko leaves, potato leaves, tomato leaves and spinach. It may also occur in the leaves of Brassicaceae plants, such as horse radish, cabbage, turnip, Chinese mustard, cauliflower and watercress.
In higher plants, the galactolipids contain a high proportion of polyunsaturated fatty acids, up to 95% of which can be linolenic acid (18:3(n-3)). In this instance, the most abundant molecular species of mono- and digalactosyldiacylglycerols must have 18:3 at both sn-I and sn-2 positions of the glycerol backbone. Plants such as the pea, which have 18:3 as almost the only fatty acid in the monogalactosyldiacylglycerols, have been termed "18:3 plants". Other species, and Arabidopsis thaliana is an example, contain appreciable amounts of hexadecatrienoic acid (16:3(n-3)) in the monogalactosyldiacylglycerols, and they are termed "16:3 plants".
As mentioned, polyunsaturated fatty acid are further used in the cosmetic industry. The application US 20030039672 discloses a cosmetic method for treating aged, sensitive, dry, flaky, wrinkled and/or photodamaged skin through topical application of a composition which comprises an unsaturated C16 fatty acid having at least three double bonds, which may be preferably hexadecatrienoic acid.

**[0010.0.15.15.]** On account of their positive properties there has been no shortage of attempts in the past to make available genes which participate in the synthesis of fatty acids or triglycerides for the production of oils in various organisms having a modified content of unsaturated fatty acids.

**[0011.0.15.15]** Methods of recombinant DNA technology have also been used for some years to improve the oil content in microorganisms or plants by amplifying individual fatty acid biosynthesis genes and investigating the effect on fatty acid production. For example in WO 91/13972 a Δ-9-desaturase is described, which is involved in the synthesis of polyunsaturated fatty acids. In WO 93/11245 a Δ-15-desaturase and in WO 94/11516 a Δ-12-desaturase is claimed. Other desaturases are described, for example, in EP-A-0 550 162, WO 94/18337, WO 97/30582, WO 97/21340, WO 95/18222, EP-A-0 794 250, Stukey et al., J. Biol. Chem., 265, 1990: 20144-20149, Wada et al., Nature 347, 1990: 200-203 or Huang et al., Lipids 34, 1999: 649-659. To date, however, the various desaturases have been only inadequately characterized biochemically since the enzymes in the form of membrane-bound proteins are isolable and characterizable only with very great difficulty (McKeon et al., Methods in Enzymol. 71, 1981: 12141-12147, Wang et al., Plant Physiol. Biochem., 26, 1988: 777-792). Generally, membrane-bound desaturases are characterized by introduction into a suitable organism, which is then investigated for enzyme activity by means of analysis of starting materials and products. With regard to the effectiveness of the expression of desaturases and their effect on the formation of polyunsaturated fatty acids it may be noted that through expression of a desaturases and elongases as described to date only low contents of poly-unsaturated fatty acids/lipids have been achieved. Therefore, an alternative and more effective pathway with higher product yield is desirable.

**[0012.0.15.15]** Accordingly, there is still a great demand for new and more suitable genes which encode enzymes which participate in the biosynthesis of fatty acids and make it possible to produce certain fatty acids specifically on an industrial scale without unwanted byproducts forming. In the selection of genes for biosynthesis two characteristics above all are particularly important. On the one hand, there is as ever a need for improved processes for obtaining the highest possible contents of polyunsaturated fatty acids on the other hand as less as possible byproducts should be produced in the production process.

**[0013.0.0.15] see [0013.0.0.0]**

**[0014.0.15.15]** Accordingly, in a first embodiment, the invention relates to a process for the production of a fine chemical, whereby the fine chemical is heptadecanoic acid (C17:0, margaric acid) and/or 2-hydroxy palmitic acid (2-OH-C16:0, alfa-hydroxy palmitic acid, C16:0 OH) and/or 2-hydroxy-tetracosenoic-acid (2-hydroxy-15-tetracosenoic acid, **hydroxynervonic acid, alfa-hydroxy-tetracosenoic-acid, C24:1 (n-9) OH, 2-hydroxy-cis 9-tetracosenoic-acid, delta 9 hydroxy-tetracosenoic-acid)** and/or hexadecadienoic acid, preferably delta 7, 10 hexadecadienoic acid (C16:2 (n-6), cis 7- cis 10-hexadecadienoic acid) and/or hexadecatrienoic acid, preferably delta 7, 10 ,13 hexadecatrienoic acid (C16:3 (n-3), cis 7- cis 10- cis 13-hexadecatrienoic acid, hiragonic acid) or tryglycerides, lipids, oils or fats containing heptadecanoic acid and/or 2-hydroxy palmitic acid and/or 2-hydroxy-tetracosenoic-acid and/or hexadecadienoic acid, preferably delta 7, 10 hexadecadienoic acid and/or hexadecatrienoic acid, preferably delta 7, 10 ,13 hexadecatrienoic acid . Accordingly, in the present invention, the term "the fine chemical" as used herein relates to "heptadecanoic acid and/or 2-hydroxy palmitic acid and/or 2-hydroxy-tetracosenoic-acid and/or hexadecadienoic acid, preferably delta 7, 10 hexadecadienoic acid and/or hexadecatrienoic acid, preferably delta 7, 10 ,13 hexadecatrienoic acid and/or tryglycerides, lipids, oils and/or fats containing heptadecanoic acid and/or 2-hydroxy palmitic acid and/or 2-hydroxy-tetracosenoic-acid and/or hexadecadienoic acid, preferably delta 7, 10 hexadecadienoic acid and/or hexadecatrienoic acid, preferably delta 7, 10 ,13 hexadecatrienoic acid". Further, the term "the fine chemicals" as used herein also relates to fine chemicals comprising heptadecanoic acid and/or 2-hydroxy palmitic acid and/or 2-hydroxy-tetracosenoic-acid and/or hexadecadienoic acid, preferably delta 7, 10 hexadecadienoic acid and/or hexadecatrienoic acid, preferably delta 7, 10 ,13 hexadecatrienoic acid and/or triglycerides, lipids, oils and/or fats containing heptadecanoic acid and/or 2-hydroxy palmitic acid and/or 2-hydroxy-tetracosenoic-acid and/or hexadecadienoic acid, preferably delta 7, 10 hexadecadienoic acid and/or hexadecatrienoic acid, preferably delta 7, 10 ,13 hexadecatrienoic acid.

**[0015.0.15.15]** In one embodiment, the term "the fine chemical" means heptadecanoic acid and/or 2-hydroxy palmitic acid and/or 2-hydroxy-tetracosenoic-acid and/or hexadecadienoic acid, preferably delta 7, 10 hexadecadienoic acid and/or hexadecatrienoic acid, preferably delta 7, 10 ,13 hexadecatrienoic acid and/or tryglycerides, lipids, oils and/or fats containing heptadecanoic acid and/or 2-hydroxy palmitic acid and/or 2-hydroxy-tetracosenoic-acid and/or hexadecadienoic acid, preferably delta 7, 10 hexadecadienoic acid and/or hexadecatrienoic acid, preferably delta 7, 10 ,13 hexadecatrienoic acid . Throughout the specification the term "the fine chemical" means heptadecanoic acid and/or 2-hydroxy palmitic acid and/or 2-hydroxy-tetracosenoic-acid and/or hexadecadienoic acid, preferably delta 7, 10 hexadecadienoic acid and/or hexadecatrienoic acid, preferably delta 7, 10 ,13 hexadecatrienoic acid and/or tryglycerides, lipids, oils and/or fats containing heptadecanoic acid and/or 2-hydroxy palmitic acid and/or 2-hydroxy-tetracosenoic-acid and/or hexadecadienoic acid, preferably delta 7, 10 hexadecadienoic acid and/or hexadecatrienoic acid, preferably delta 7, 10 ,13 hexadecatrienoic , heptadecanoic acid and/or 2-hydroxy palmitic acid and/or 2-hydroxy-tetracosenoic-acid and/or hexadecadienoic acid, preferably delta 7, 10 hexadecadienoic acid and/or hexadecatrienoic acid, preferably delta 7, 10 ,13 hexadecatrienoic acid and its salts, ester, thioester or heptadecanoic acid and/or 2-hydroxy palmitic acid and/or 2-hydroxy-tetracosenoic-acid and/or hexadecadienoic acid, preferably delta 7, 10 hexadecadienoic acid and/or hexadecatrienoic acid, preferably delta 7, 10 ,13 hexadecatrienoic acid in free form or bound to other compounds such as triglycerides, glycolipids, phospholipids etc. In a preferred embodiment, the term "the fine chemical" means heptadecanoic acid and/or 2-hydroxy palmitic acid and/or 2-hydroxy-tetracosenoic-acid and/or hexadecadienoic acid, preferably delta 7, 10 hexadecadienoic acid and/or hexadecatrienoic acid, preferably delta 7, 10 ,13 hexadecatrienoic acid, in free form or its salts or bound to a glycerol backbone or to glycerol-3-phosphate backbone or to a sphingosine-phosphate backbone or to sphingosine-mono- or oligosaccharide backbone or to a glycerol-3-mono- or disaccharide backbone. Triglycerides, lipids, oils, fats or lipid mixture thereof shall mean any triglyceride, lipid, oil and/or fat containing any bound or free heptadecanoic acid and/or 2-hydroxy palmitic acid and/or 2-hydroxy-tetracosenoic-acid and/or hexadecadienoic acid, preferably delta 7, 10 hexadecadienoic acid and/or hexadecatrienoic acid, preferably delta 7, 10 ,13 hexadecatrienoic acid for example sphingolipids, phosphoglycerides, lipids, glycerophospholipids, galactolipids, glycolipids such as glycosphingolipids, phospholipids such as phosphatidylethanolamine, phosphatidylcholine, phosphatidylserine, phosphatidylglycerol, phosphatidylinositol or diphosphatidylglycerol, or as monoacylglyceride, diacylglyceride or triacylglyceride or other fatty acid esters such as acetyl-Coenzym A thioester, which contain further saturated or unsaturated fatty acids in the fatty acid molecule.

**[0016.0.15.15]** Accordingly, the present invention relates to a process comprising
(a) increasing or generating the activity of of one or more
   YDR513W and/or YER156C and/or YLR255C and/or b1829 protein(s) and/or
   YER173W protein and/or
   YFR042W protein and/or
   YOR317W protein and/or
   YGL205W and/or YIL150C and/or YKL132C and/or YOR344C and/or b3430 and/or b0161 and/or b0758 and/or b0057 and/or b1097 and/or b2078 and/or b3231 protein(s) or of a protein having the sequence of a polypeptide indicated in Table II A or II B, column 3, lines 174 to 185, 558 to 563 or having the sequence of a polypeptide encoded by a nucleic acid molecule indicated in Table IA or IB, column 5 or 7, lines 174 to 185, 558 to 563,
   in a non-human organism in one or more parts thereof and
(b) growing the organism under conditions which permit the production of the fine chemical, thus, heptadecanoic acid and/or 2-hydroxy palmitic acid and/or 2-hydroxy-tetracosenoic-acid and/or hexadecadienoic acid, preferably delta 7, 10 hexadecadienoic acid and/or hexadecatrienoic acid, preferably delta 7, 10 ,13 hexadecatrienoic acid or fine chemicals comprising heptadecanoic acid and/or 2-hydroxy palmitic acid and/or 2-hydroxy-tetracosenoic-acid and/or hexadecadienoic acid, preferably delta 7, 10 hexadecadienoic acid and/or hexadecatrienoic acid, preferably delta 7, 10 ,13 hexadecatrienoic acid and/or 24:1 fatty acid, preferably C24:1 delta 15 fatty acid, in said organism.
Accordingly, the present invention relates to a process for the production of the respective fine chemical comprising
(a) increasing or generating the activity of one or more protein(s) having the activity of a protein indicated in Table IIA or IIB, Column 3, lines 174 to 185, 558 to 563 or having the sequence of a polypeptide encoded by a nucleic acid molecule indicated in Table IA or IB , columns 5 or 7, lines 174 to 185, 558 to 563 in a non-human organism in one or more parts thereof and
(b) growing the organism under conditions which permit the production of the fine chemical, thus, fatty acid, in particular heptadecanoic acid and/or 2-hydroxy palmitic acid and/or 2-hydroxy-tetracosenoic-acid and/or hexadecadienoic acid, preferably delta 7, 10 hexadecadienoic acid and/or hexadecatrienoic acid, preferably delta 7, 10 ,13 hexadecatrienoic acid and/or 24:1 fatty acid, preferably C24:1 delta 15 fatty acid.

**[0016.1.15.15]** Accordingly, the term "the fine chemical" means in one embodiment "heptadecanoic acid " in relation to all sequences listed in Table I to IV, lines 174 to 177 or homologs thereof and
means in one embodiment "2-hydroxy palmitic acid " in relation to all sequences listed in Tables I to IV, line 178, 558 and 559 or homologs thereof and
means in one embodiment "2-Hydroxy-tetracosenoic acid " in relation to all sequences listed in Table IA or IB , line 179, and
means in one embodiment "hexadecadienoic acid, preferably delta 7, 10 hexadecadienoic acid " in relation to all sequences listed in Table I to IV, line 180 and
means in one embodiment "hexadecatrienoic acid, preferably delta 7, 10 ,13 hexadecatrienoic acid " in relation to all sequences listed in Table I to IV, lines 181 to 185, and means in one embodiment "tetracosenoic acid " in relation to all sequences listed in Table I to IV, lines 560 to 563 or homologs thereof.
Accordingly, the term "the fine chemical" can mean "heptadecanoic acid" ("C17:0", "margaric acid") and/or "2-hydroxy palmitic acid" ("2-OH-C16:0", "alfa-hydroxy palmitic acid", "C16:0 OH") and/or "2-hydroxy-tetracosenoic-acid" ("2-hydroxy-15-tetracosenoic acid", **"hydroxynervonic acid", "alfa-hydroxy-tetracosenoic-acid", "C24:1 (n-9) OH", "2-hydroxy-cis 9-tetracosenoic-acid", "delta 9 hydroxy-tetracosenoic-acid")** and/or "hexadecadienoic acid", preferably "delta 7, 10 hexadecadienoic acid" ("C16:2 (n-6)", "cis 7- cis 10-hexadecadienoic acid") and/or "hexadecatrienoic acid", preferably "delta 7, 10 ,13 hexadecatrienoic acid" ("C16:3 (n-3)", "cis 7- cis 10- cis 13-hexadecatrienoic acid", "hiragonic acid") , owing to circumstances and the context. In order to illustrate that the meaning of the term "the fine chemical" means "heptadecanoic acid" ("C17:0", "margaric acid") and/or "2-hydroxy palmitic acid" ("2-OH-C16:0", "alfa-hydroxy palmitic acid", "C16:0 OH") and/or "2-hydroxy-tetracosenoic-acid" ("2-hydroxy-15-tetracosenoic acid", **"hydroxynervonic acid", "alfa-hydroxy-tetracosenoic-acid", "C24:1 (n-9) OH", "2-hydroxy-cis 9-tetracosenoic-acid", "delta 9 hydroxy-tetracosenoic-acid")** and/or "hexadecadienoic acid", preferably "delta 7, 10 hexadecadienoic acid" ("C16:2 (n-6)", "cis 7- cis 10-hexadecadienoic acid") and/or "hexadecatrienoic acid", preferably "delta 7, 10 ,13 hexadecatrienoic acid" ("C16:3 (n-3)", "cis 7- cis 10- cis 13-hexadecatrienoic acid", "hiragonic acid") and/or 24:1 fatty acid, preferably C24:1 delta 15 fatty acid the term "the respective fine chemical" is also used.

**[0017.0.0.15] see [0017.0.0.0]**

**[0018.0.0.15] see [0018.0.0.0]**

**[0019.0.15.15]** Advantageously the process for the production of the respective fine chemical leads to an enhanced production of the fine respective chemical. The terms "enhanced" or "increase" mean at least a 10%, 20%, 30%, 40% or 50%, preferably at least 60%, 70%, 80%, 90% or 100%, more preferably 150%, 200%, 300%, 400% or 500% higher production of the respective fine chemical in comparison to the reference as defined below, e.g. that means in comparison to an organism without the aforementioned modification of the activity of a protein having the activity of a protein indicated in Table IIA or IIB, column 3, lines 174 to 185, 558 to 563 or encoded by nucleic acid molecule indicated in Table IA or IB , columns 5 or 7, lines 174 to 185, 558 to 563.

**[0020.0.15.15]** Surprisingly it was found, that the transgenic expression of the Saccharomyces cerevisiae protein(s) YDR513W and/or YER156C and/or YLR255C as indicated in Table II, Column 3 or 5, lines 174 to 176 respectively and/or the Escherichia coli K12 protein(s) b1829 as indicated in Table I, Column 3 or 5, line 177 in Arabidopsis thaliana conferred an increase in the heptadecanoic acid content of the transformed plants. Thus, in one embodiment, said protein(s) or its homologs as indicated in Table II, Column 7, line174 to 177 are used for the production of heptadecanoic acid.
Surprisingly it was found, that the transgenic expression of the Saccharomyces cerevisiae protein YER173W as indicated in Table II, Column 3 or 5, line 178 in Arabidopsis thaliana conferred an increase in the 2-hydroxy palmitic acid content of the transformed plants. Thus, in one embodiment, said protein or its homologs as indicated in Table II, Column 7, line 178 are used for the production of 2-hydroxy palmitic acid .
Surprisingly it was found, that the transgenic expression of the Saccharomyces cerevisiae protein YFR042W as indicated in Table II, Column 3 or 5, line 179 in Arabidopsis thaliana conferred an increase in the 2-hydroxy-tetracosenoic-acid content of the transformed plants. Thus, in one embodiment, said protein(s) or its homologs as indicated in Table II, Column 7, line 179 are used for the production of 2-hydroxy-tetracosenoic-acid .
Surprisingly it was found, that the transgenic expression of the Saccharomyces cerevisiae protein YOR317W as indicated in Table II, Column 3 or 5, line 180 in Arabidopsis thaliana conferred an increase in the hexadecadienoic acid, preferably delta 7, 10 hexadecadienoic acid content of the transformed plants. Thus, in one embodiment, said protein(s) or its homologs as indicated in Table II, Column 7, line 180 are used for the production of hexadecadienoic acid, preferably delta 7, 10 hexadecadienoic acid .
Surprisingly it was found, that the transgenic expression of the Saccharomyces cerevisiae protein(s) YGL205W and/or YIL150C and/or YKL132C and/or YOR344C as indicated in Table II, Column 3 or 5, line 181 to 184 and/or the Escherichia coli K12 protein b3430 as indicated in Table II, Column 3 or 5, line 185 respectively in Arabidopsis thaliana conferred an increase in the hexadecatrienoic acid, preferably delta 7, 10 ,13 hexadecatrienoic content of the transformed plants. Thus, in one embodiment, said protein(s) or its homologs as indicated in Table II, Column 7, line 181 to 185 are used for the production of hexadecatrienoic acid, preferably delta 7, 10 ,13 hexadecatrienoic. Surprisingly it was found, that the transgenic expression of the Escherichia coli protein b 0161and/or b0758 as indicated in Table IIA or IIB, Column 3 or 5, line 558, 559 in Arabidopsis thaliana conferred an increase in the 2-hydroxypalmitic acid content of the transformed plants. Thus, in one embodiment, said protein(s) or its homologs as indicated in Table II, column 7, line 558, 559 are used for the production of 2-hydroxypalmitic acid.
Surprisingly it was found, that the transgenic expression of the Escherichia coli protein b0057, b1097, b2078 or b3231 as indicated in Table IIA or IIB, Column 3 or 5, line 560 to 563 in Arabidopsis thaliana conferred an increase in the C24:1 fatty acid content of the transformed plants. Thus, in one embodiment, said protein(s) or its homologs as indicated in Table II, column 7, line 560 to 563 are used for the production of C24:1 fatty acid.

**[0021.0.15.15] see [0021.0.0.0]**

[0022.0.15.15] The sequence of YDR513W from Saccharomyces cerevisiae has been published in Jacq et al., Nature 387 (6632 Suppl), 75-78 (1997) and in Goffeau et al., Science 274 (5287), 546-547, 1996 and its cellular activity has been characterized as a glutathione reductase, preferably of the glutaredoxin superfamily. Accordingly, in one embodiment, the process of the present invention comprises the use of a gene product with an activity of the glutaredoxin superfamily, preferably a protein with a glutathione reductase activity or its homolog, e.g. as shown herein, from Saccharomyces cerevisiae or its homolog, e.g. as shown herein, for the production of the fine chemical, meaning of heptadecanoic acid and/or tryglycerides, lipids, preferably glycerophospholipids, sphingolipids and/or galactolipids, oils and/or fats containing heptadecanoic acid, in particular for increasing the amount of heptadecanoic acid and/or tryglycerides, lipids, preferably glycerophospholipids, sphingolipids and/or galactolipids, oils and/or fats containing margaric acid, preferably heptadecanoic acid in free or bound form in an organism or a part thereof, as mentioned. In one further embodiment the YDR513W protein expression is increased together with the increase of another gene of the lipid biosynthesis pathway, preferably with a gene encoding a protein being involved in the production of fatty acids or encoding a fatty acid transporter protein or a compound, which functions as a sink for the respective fatty acid. In one embodiment, in the process of the present invention the activity of a protein of the glutaredoxin superfamily, preferably of a glutathione reductase is increased or generated, e.g. from Saccharomyces cerevisiae or a homolog thereof. The sequence of YER156C from Saccharomyces cerevisiae has been published in Dietrich, et al.(Nature 387 (6632 Suppl), 78-81 (1997)), and its activity has not been characterized yet. It seems to have a activity similar to Arabidopsis thaliana hypothetical protein F2K15.180. Accordingly, in one embodiment, the process of the present invention comprises the use of a protein with the activity of a YER156C from Saccharomyces cerevisiae or of a Arabidopsis thaliana hypothetical protein F2K15.180 or its homolog, e.g. as shown herein, for the production of the respective fine chemical, meaning of heptadecanoic acid and/or tryglycerides, lipids, preferably glycerophospholipids, sphingolipids and/or galactolipids,oils and/or fats containing heptadecanoic acid and/or conjugates, preferably in free or bound form in an organism or a part thereof, as mentioned. In one further embodiment the YER156C protein expression is increased together with the increase of another gene of the lipid biosynthesis pathway, preferably with a gene encoding a protein being involved in the production of fatty acids or encoding a fatty acid transporter protein or a compound, which functions as a sink for the respective fatty acid. In one embodiment, in the process of the present invention said activity, e.g. of a YER156C protein is increased or generated, e.g. from Saccharomyces cerevisiae or a homolog thereof.
The sequence of YLR255C from Saccharomyces cerevisiae has been submitted by R;Johnson to the EMBL Data Library, (February 1995) , and its activity has not been characterized yet , but having preferably an activity of Saccharomyces hypothetical protein YLR255c superfamily Accordingly, in one embodiment, the process of the present invention comprises the use of a gene product with an activity of a protein of the Saccharomyces hypothetical protein YLR255c superfamily, preferably of a a gene product with an activity of a YLR255C protein from Saccharomyces cerevisiae or its homolog, e.g. as shown herein, for the production of the respective fine chemical, meaning of heptadecanoic acid and/or tryglycerides, lipids, preferably glycerophospholipids, sphingolipids and/or galactolipids,oils and/or fats containing heptadecanoic acid in free or bound form in an organism or a part thereof, as mentioned. In one further embodiment the YLR255C protein expression is increased together with the increase of another gene of the lipid biosynthesis pathway, preferably with a gene encoding a protein being involved in the production of fatty acids or encoding a fatty acid transporter protein or a compound, which functions as a sink for the resprective fatty acid. In one embodiment, in the process of the present invention the activity of a Saccharomyces hypothetical protein YLR255c superfamily protein, preferably YLR255C protein , is increased or generated, e.g. from Saccharomyces cerevisiae or a homolog thereof.
The sequence of b1829 from Escherichia coli K12 has been published in Blattner, Science 277(5331), 1453-1474, 1997, and its activity is being defined as a heat shock protein with protease activity, preferably of the heat-shock protein htpX superfamily. Accordingly, in one embodiment, the process of the present invention comprises the use of a gene product with an activity of the heat-shock protein htpX superfamily, preferably such protein is having a heat shock protein acitivity with protease activity from E. coli or its homolog, e.g. as shown herein, for the production of the respective fine chemical, meaning of heptadecanoic acid and/or tryglycerides, lipids, preferably glycerophospholipids, sphingolipids and/or galactolipids,oils and/or fats containing margaric acid, in particular for increasing the amount of margaric acid and/or tryglycerides, lipids, preferably glycerophospholipids, sphingolipids and/or galactolipids, oils and/or fats containing heptadecanoic acid, preferably heptadecanoic acid in free or bound form in an organism or a part thereof, as mentioned. In one further embodiment the b1829 protein expression is increased together with the increase of another gene of the lipid biosynthesis pathway, preferably with a gene encoding a protein being involved in the production of fatty acids or encoding a fatty acid transporter protein or a compound, which functions as a sink for the respective fatty acid. In one embodiment, in the process of the present invention the activity of a heat shock protein with protease activity is increased or generated, e.g. from E. coli or a homolog thereof.
The sequence of YER173W from Saccharomyces cerevisiae has been published in Dietrich et al., Nature 387 (6632 Suppl), 78-81, 1997, and Goffeau et al., Science 274 (5287), 546-547, 1996, and its activity is being defined as a checkpoint protein, involved in the activation of the DNA damage and meiotic pachytene checkpoints. Accordingly, in one embodiment, the process of the present invention comprises the use of a gene product with an activity of Checkpoint protein, involved in the activation of the DNA damage and meiotic pachytene checkpoints or its homolog, e.g. as shown herein, for the production of the respective fine chemical, meaning of 2-hydroxy-palmitic acid and/or tryglycerides, lipids, preferably glycerophospholipids, sphingolipids and/or galactolipids,oils and/or fats containing 2-hydroxy-palmitic acid, in particular for increasing the amount of 2-hydroxy-palmitic acid and/or tryglycerides, lipids, preferably glycerophospholipids, sphingolipids and/or galactolipids, oils and/or fats containing 2-hydroxy-palmitic acid in free or bound form in an organism or a part thereof, as mentioned. In one further embodiment the YER173w protein expression is increased together with the increase of another gene of the lipid biosynthesis pathway, preferably with a gene encoding a protein being involved in the production of glucosylceramide, preferably of monoglucosecerebroside. In one embodiment, in the process of the present invention the activity of a Checkpoint protein, involved in the activation of the DNA damage and meiotic pachytene checkpoints is increased or generated, e.g. from Saccharomyces cerevisiae or a homolog thereof.
The sequence of YFR042W from Saccharomyces cerevisiae has been published in Murakami et al., Nat. Genet. 10 (3), 261-268 (1995) and Goffeau et al., Science 274 (5287), 546-547, 1996, and has a putative cellular function of a "protein, which is required for cell viability", preferably of the Saccharomyces cerevisiae probable membrane protein YFR042w superfamily. Accordingly, in one embodiment, the process of the present invention comprises the use of a a gene product with an activity of the Saccharomyces cerevisiae probable membrane protein YFR042w superfamily, preferably a gene product with a YFR042W protein activity from Saccharomyces cerevisiae or its homolog, e.g. as shown herein, for the production of the fine chemical, meaning of tetracosenoic acid (nervonic acid, C24:1 delta 15), preferably 2-hydroxy-tetracosenoic-acid and/or tryglycerides, lipids, preferably glycerophospholipids, sphingolipids and/or galactolipids, oils and/or fats containing tetracosenoic acid (nervonic acid, C24:1 delta 15), preferably 2-hydroxy-tetracosenoic acid , in particular for increasing the amount of tetracosenoic acid (nervonic acid), preferably 2-hydroxy-tetracosenoic acid and/or tryglycerides, lipids, preferably glycerophospholipids, sphingolipids and/or galactolipids,oils and/or fats containing tetracosenoic acid (nervonic acid), preferably 2-hydroxy-tetracosenoic acid , preferably tetracosenoic acid (nervonic acid), preferably 2-hydroxy-tetracosenoic acid in free or bound form in an organism or a part thereof, as mentioned. In one further embodiment the YFR042W protein expression is increased together with the increase of another gene of the lipid biosynthesis pathway, preferably with a gene encoding a protein being involved in the production of lipids, preferably of cerebroside. In one embodiment, in the process of the present invention the activity of a YFR042W protein is increased or generated, e.g. from Saccharomyces cerevisiae or a homolog thereof.
The sequence of YOR317W from Saccharomyces cerevisiae has been published in Dujon et al., Nature 387 (6632 Suppl), 98-102 (1997) and in Goffeau et al., Science 274 (5287), 546-547, 1996 and its cellular activity has characterized as a long chain fatty acyl:CoA synthetase, preferably of the long-chain-fatty-acid-CoA ligase superfamily. Accordingly, in one embodiment, the process of the present invention comprises the use of a gene product with an activity of the long-chain-fatty-acid-CoA ligase superfamily, preferably a protein with a long chain fatty acyl:CoA synthetase activity or its homolog, e.g. as shown herein, from Saccharomyces cerevisiae or its homolog, e.g. as shown herein, for the production of the fine chemical, meaning of hexadecadienoic acid (C16:2, preferably C16:2 delta 7, 10) and/or tryglycerides, lipids, preferably glycerophospholipids, sphingolipids and/or galactolipids, oils and/or fats containing hexadecadienoic acid, in particular for increasing the amount of hexydecadienoic acid and/or tryglycerides, lipids, oils and/or fats containing hexadecadienoic acid, preferably hexydecadienoic acid in free or bound form in an organism or a part thereof, as mentioned. In one further embodiment the YOR317W protein expression is increased together with the increase of another gene of the lipid biosynthesis pathway, preferably with a gene encoding a protein being involved in the production of galactolipids, preferably of monogalactosyldiacylglycerol. In one embodiment, in the process of the present invention the activity of a protein of the long-chain-fatty-acid-CoA ligase superfamily, preferably of a long chain fatty acyl:CoA synthetase is increased or generated, e.g. from Saccharomyces cerevisiae or a homolog thereof.
The sequence of YGL205W from Saccharomyces cerevisiae has been published in Tettelin et al., Nature 387 (6632 Suppl), 81-84 (1997) and in Goffeau et al., Science 274 (5287), 546-547, 1996 and its cellular activity has characterized as fatty-acyl coenzyme A oxidase, preferably of the acyl-CoA oxidase superfamily. Accordingly, in one embodiment, the process of the present invention comprises the use of a gene product with an activity of the acyl-CoA oxidase superfamily, preferably a protein with a fatty-acyl coenzyme A oxidase activity or its homolog, e.g. as shown herein, from Saccharomyces cerevisiae or its homolog, e.g. as shown herein, for the production of the fine chemical, meaning of hexydecatrienoic acid (C16:3, preferably C16:2 delta 7, 10, 13) and/or tryglycerides, lipids, preferably glycerophospholipids, sphingolipids and/or galactolipids, oils and/or fats containing hexydecatrienoic acid, in particular for increasing the amount of hexydecatrienoic acid and/or tryglycerides, lipids, preferably glycerophospholipids, sphingolipids and/or galactolipids, oils and/or fats containing hexydecatrienoic acid, preferably hexydecatrienoic acid in free or bound form in an organism or a part thereof, as mentioned. In one further embodiment the YGL205W protein expression is increased together with the increase of another gene of the lipid biosynthesis pathway, preferably with a gene encoding a protein being involved in the production of galactolipids, preferably of monogalactosyldiacylglycerol. In one embodiment, in the process of the present invention the activity of a protein of the acyl-CoA oxidase superfamily, preferably of a fatty-acyl coenzyme A oxidase is increased or generated, e.g. from Saccharomyces cerevisiae or a homolog thereof.
The sequence of YIL150C from Saccharomyces cerevisiae has been published in Churcher et al., Nature 387 (6632 Suppl), 84-87 (1997) and in Goffeau et al., Science 274 (5287), 546-547, 1996 and seems to have a putative cellular activity of a its "protein required for S-phase (DNA synthesis) initiation or completion and/or chromatin binding protein".
Accordingly, in one embodiment, the process of the present invention comprises the use of a gene product with the activity of a YIL150C protein, from Saccharomyces cerevisiae or its homolog, e.g. as shown herein, for the production of the fine chemical, meaning of hexadecatrienoic acid, preferably delta 7, 10 ,13 hexadecatrienoic acid and/or tryglycerides, lipids, preferably glycerophospholipids, sphingolipids and/or galactolipids, oils and/or fats containing hexadecatrienoic acid, preferably delta 7, 10 ,13 hexadecatrienoic acid, in particular for increasing the amount of hexadecatrienoic acid and/or tryglycerides, lipids, preferably glycerophospholipids, sphingolipids and/or galactolipids, oils and/or fats containing hexadecatrienoic acid, preferably hexadecatrienoic acid, preferably delta 7, 10 ,13 hexadecatrienoic acid in free or bound form in an organism or a part thereof, as mentioned. In one further embodiment the YIL150C protein expression is increased together with the increase of another gene of the lipid biosynthesis pathway, preferably with a gene encoding a protein being involved in the production of galactolipids, preferably of monogalactosyldiacylglycerol. In one embodiment, in the process of the present invention the activity of a YIL150C protein is increased or generated, e.g. from Saccharomyces cerevisiae or a homolog thereof.
The sequence of YKL132C from Saccharomyces cerevisiae has been published in Dujon et al., Nature 369 (6479), 371-378 (1994) and in Goffeau et al., Science 274 (5287), 546-547, 1996 and its cellular activity has characterized as folyl-polyglutamate synthase, preferably of the folylpolyglutamate synthase superfamily. Accordingly, in one embodiment, the process of the present invention comprises the use of a gene product with an activity of the folylpolyglutamate synthase superfamily, preferably a protein with a folyl-polyglutamate synthase activity or its homolog, e.g. as shown herein, from Saccharomyces cerevisiae or its homolog, e.g. as shown herein, for the production of the fine chemical, meaning of hexydecatrienoic acid preferably delta 7, 10 ,13 hexadecatrienoic and/or tryglycerides, lipids, preferably glycerophospholipids, sphingolipids and/or galactolipids, oils and/or fats containing hexydecatrienoic acid, in particular for increasing the amount of hexydecatrienoic acid preferably delta 7, 10 ,13 hexadecatrienoic and/or tryglycerides, lipids, preferably glycerophospholipids, sphingolipids and/or galactolipids, oils and/or fats containing hexydecatrienoic acid, preferably hexydecatrienoic acid in free or bound form in an organism or a part thereof, as mentioned. In one further embodiment the YKL132C protein expression is increased together with the increase of another gene of the lipid biosynthesis pathway, preferably with a gene encoding a protein being involved in the production of galactolipids, preferably of monogalactosyldiacylglycerol. In one embodiment, in the process of the present invention the activity of a protein of the folylpolyglutamate synthase superfamily, preferably of a folyl-polyglutamate synthase is increased or generated, e.g. from Saccharomyces cerevisiae or a homolog thereof.
The sequence of YOR344C from Saccharomyces cerevisiae has been published in Dujon et al., Nature 369 (6479), 371-378 (1994) and in Goffeau et al., Science 274 (5287), 546-547, 1996 and its cellular activity has characterized as a serine-rich protein, putatively involved in glycolytic gene expression. Accordingly, in one embodiment, the process of the present invention comprises the use of a gene product with an activity of a serine-rich protein, putatively involved in glycolytic gene expression activity or its homolog, e.g. as shown herein, from Saccharomyces cerevisiae or its homolog, e.g. as shown herein, for the production of the fine chemical, meaning of hexydecatrienoic acid preferably delta 7, 10 ,13 hexadecatrienoic acid and/or tryglycerides, lipids, preferably glycerophospholipids, sphingolipids and/or galactolipids, oils and/or fats containing hexydecatrienoic acid preferably delta 7, 10 ,13 hexadecatrienoic acid, in particular for increasing the amount of hexydecatrienoic acid preferably delta 7, 10 ,13 hexadecatrienoic acid and/or tryglycerides, lipids, preferably glycerophospholipids, sphingolipids and/or galactolipids, oils and/or fats containing hexydecatrienoic acid, preferably preferably delta 7, 10 ,13 hexadecatrienoic acid in free or bound form in an organism or a part thereof, as mentioned. In one further embodiment the YOR344C protein expression is increased together with the increase of another gene of the lipid biosynthesis pathway, preferably with a gene encoding a protein being involved in the production of galactolipids, preferably of monogalactosyldiacylglycerol. In one embodiment, in the process of the present invention the activity of a serine-rich protein, putatively involved in glycolytic gene expression is increased or generated, e.g. from Saccharomyces cerevisiae or a homolog thereof.
The sequence of b3430 from Escherichia coli K12 has been published in Blattner, Science 277(5331), 1453-1474, 1997, and its activity is being defined as a glucose-1-phosphate adenylyltransferase, preferably of the a glucose-1-phosphate adenylyltransferase superfamily. Accordingly, in one embodiment, the process of the present invention comprises the use of a gene product with an activity of the a glucose-1-phosphate adenylyltransferase superfamily, preferably such protein is having a a glucose-1-phosphate adenylyltransferase acitivity from E. coli or its homolog, e.g. as shown herein, for the production of the respective fine chemical, meaning of hexydecatrienoic acid (C16:3, preferably C16:3 delta 7, 10, 13) and/or tryglycerides, lipids, preferably glycerophospholipids, sphingolipids and/or galactolipids, oils and/or fats containing hexydecatrienoic acid, preferably delta 7, 10 ,13 hexadecatrienoic acid, in particular for increasing the amount of hexydecatrienoic acid, preferably delta 7, 10 ,13 hexadecatrienoic acid and/or tryglycerides, lipids, preferably glycerophospholipids, sphingolipids and/or galactolipids, oils and/or fats containing hexydecatrienoic acid, preferably delta 7, 10 ,13 hexadecatrienoic acid in free or bound form in an organism or a part thereof, as mentioned. In one further embodiment the b3430 protein expression is increased together with the increase of another gene of the lipid biosynthesis pathway, preferably with a gene encoding a protein being involved in the production of galactolipids, preferably of monogalactosyldiacylglycerol In one embodiment, in the process of the present invention the activity of a a glucose-1-phosphate adenylyltransferase is increased or generated, e.g. from E. coli or a homolog thereof.
The sequence of b0057 from Escherichia coli K12 has been published in Blattner, Science 277(5331), 1453-1474, 1997, and its activity has not been characterized yet. Accordingly, in one embodiment, the process of the present invention comprises the use of a b0057 protein from E. coli or its homolog, e.g. as shown herein, for the production of the respective fine chemical, meaning of tetracosenoic acid (nervonic acid, C24:1 delta 15), preferably 2-hydroxy-tetracosenoic-acid and/or tryglycerides, lipids, preferably glycerophospholipids, sphingolipids and/or galactolipids, oils and/or fats containing tetracosenoic acid (nervonic acid, C24:1 delta 15), preferably 2-hydroxy-tetracosenoic acid , in particular for increasing the amount of tetracosenoic acid (nervonic acid), preferably 2-hydroxy-tetracosenoic acid and/or tryglycerides, lipids, preferably glycerophospholipids, sphingolipids and/or galactolipids,oils and/or fats containing tetracosenoic acid (nervonic acid), preferably 2-hydroxy-tetracosenoic acid , preferably tetracosenoic acid (nervonic acid), preferably 2-hydroxy-tetracosenoic acid in free or bound form in an organism or a part thereof, as mentioned. In one further embodiment the b0057 protein expression is increased together with the increase of another gene of the lipid biosynthesis pathway, preferably with a gene encoding a protein being involved in the production of lipids, preferably of cerebroside. In one embodiment, in the process of the present invention the activity of a b0057 protein is increased or generated, e.g. from Echerischia Coli or a homolog thereof.
The sequence of b0161 from Escherichia coli K12 has been published in Blattner, Science 277(5331), 1453-1474, 1997, and its activity is being defined as a protein having periplasmic serine protease (heat shock protein) activity. Accordingly, in one embodiment, the process of the present invention comprises the use of a gene product with an activity of a b0161 protein from E. coli or its homolog, e.g. as shown herein, for the production of the respective fine chemical, meaning of 2-hydroxy-palmitic acid and/or tryglycerides, lipids, preferably glycerophospholipids, sphingolipids and/or galactolipids,oils and/or fats containing 2-hydroxy-palmitic acid, in particular for increasing the amount of 2-hydroxy-palmitic acid and/or tryglycerides, lipids, preferably glycerophospholipids, sphingolipids and/or galactolipids, oils and/or fats containing 2-hydroxy-palmitic acid in free or bound form in an organism or a part thereof, as mentioned. In one further embodiment the b0161 protein expression is increased together with the increase of another gene of the lipid biosynthesis pathway, preferably with a gene encoding a protein being involved in the production of glucosylceramide, preferably of monoglucosecerebroside. In one embodiment, in the process of the present invention the activity of a protein having periplasmic serine protease (heat shock protein) activity is increased or generated, e.g. from Escherichia Coli.
The sequence of b0758 from Escherichia coli K12 has been published in Blattner, Science 277(5331), 1453-1474, 1997, and its activity is being defined as a protein having galactose-1-phosphate uridylyltransferase activity. Accordingly, in one embodiment, the process of the present invention comprises the use of a gene product with an activity of a b0758 protein from E. coli or its homolog, e.g. as shown herein, for the production of the respective fine chemical, meaning of 2-hydroxy-palmitic acid and/or tryglycerides, lipids, preferably glycerophospholipids, sphingolipids and/or galactolipids,oils and/or fats containing 2-hydroxy-palmitic acid, in particular for increasing the amount of 2-hydroxy-palmitic acid and/or tryglycerides, lipids, preferably glycerophospholipids, sphingolipids and/or galactolipids, oils and/or fats containing 2-hydroxy-palmitic acid in free or bound form in an organism or a part thereof, as mentioned. In one further embodiment the b0758 protein expression is increased together with the increase of another gene of the lipid biosynthesis pathway, preferably with a gene encoding a protein being involved in the production of glucosylceramide, preferably of monoglucosecerebroside. In one embodiment, in the process of the present invention the activity of a protein having galactose-1-phosphate uridylyltransferase activity is increased or generated, e.g. from Escherichia Coli.
The sequence of b1097 from Escherichia coli K12 has been published in Blattner, Science 277(5331), 1453-1474, 1997, and its activity is being defined as a protein having thymidylate kinase activity. Accordingly, in one embodiment, the process of the present invention comprises the use of a gene product with an activity of a b1097 protein from E. coli or its homolog, e.g. as shown herein, for the production of the respective fine chemical, meaning of tetracosenoic acid (nervonic acid, C24:1 delta 15), preferably 2-hydroxy-tetracosenoic-acid and/or tryglycerides, lipids, preferably glycerophospholipids, sphingolipids and/or galactolipids, oils and/or fats containing tetracosenoic acid (nervonic acid, C24:1 delta 15), preferably 2-hydroxy-tetracosenoic acid , in particular for increasing the amount of tetracosenoic acid (nervonic acid), preferably 2-hydroxy-tetracosenoic acid and/or tryglycerides, lipids, preferably glycerophospholipids, sphingolipids and/or galactolipids,oils and/or fats containing tetracosenoic acid (nervonic acid), preferably 2-hydroxy-tetracosenoic acid , preferably tetracosenoic acid (nervonic acid), preferably 2-hydroxy-tetracosenoic acid in free or bound form in an organism or a part thereof, as mentioned. In one further embodiment the b1097 protein expression is increased together with the increase of another gene of the lipid biosynthesis pathway, preferably with a gene encoding a protein being involved in the production of lipids, preferably of cerebroside. In one embodiment, in the process of the present invention the activity of a b0057 protein is increased or generated, e.g. from Echerischia Coli or a homolog thereof.
The sequence of b2078 from Escherichia coli K12 has been published in Blattner, Science 277(5331), 1453-1474, 1997, and its activity is being defined as a protein having histidine kinase activity. Accordingly, in one embodiment, the process of the present invention comprises the use of a gene product with an activity of a b2078 protein from E. coli or its homolog, e.g. as shown herein, for the production of the respective fine chemical, meaning of tetracosenoic acid (nervonic acid, C24:1 delta 15), preferably 2-hydroxy-tetracosenoic-acid and/or tryglycerides, lipids, preferably glycerophospholipids, sphingolipids and/or galactolipids, oils and/or fats containing tetracosenoic acid (nervonic acid, C24:1 delta 15), preferably 2-hydroxy-tetracosenoic acid , in particular for increasing the amount of tetracosenoic acid (nervonic acid), preferably 2-hydroxy-tetracosenoic acid and/or tryglycerides, lipids, preferably glycerophospholipids, sphingolipids and/or galactolipids,oils and/or fats containing tetracosenoic acid (nervonic acid), preferably 2-hydroxy-tetracosenoic acid , preferably tetracosenoic acid (nervonic acid), preferably 2-hydroxy-tetracosenoic acid in free or bound form in an organism or a part thereof, as mentioned. In one further embodiment the b2078 protein expression is increased together with the increase of another gene of the lipid biosynthesis pathway, preferably with a gene encoding a protein being involved in the production of lipids, preferably of cerebroside. In one embodiment, in the process of the present invention the activity of a b2078 protein is increased or generated, e.g. from Echerischia Coli or a homolog thereof.
The sequence of b3231 from Escherichia coli K12 has been published in Blattner, Science 277(5331), 1453-1474, 1997, and its activity is being defined as a protein having 50S ribosomal subunit protein L13 activity. Accordingly, in one embodiment, the process of the present invention comprises the use of a gene product with an activity of a b3231 protein from E. coli or its homolog, e.g. as shown herein, for the production of the respective fine chemical, meaning of tetracosenoic acid (nervonic acid, C24:1 delta 15), preferably 2-hydroxy-tetracosenoic-acid and/or tryglycerides, lipids, preferably glycerophospholipids, sphingolipids and/or galactolipids, oils and/or fats containing tetracosenoic acid (nervonic acid, C24:1 delta 15), preferably 2-hydroxy-tetracosenoic acid , in particular for increasing the amount of tetracosenoic acid (nervonic acid), preferably 2-hydroxy-tetracosenoic acid and/or tryglycerides, lipids, preferably glycerophospholipids, sphingolipids and/or galactolipids,oils and/or fats containing tetracosenoic acid (nervonic acid), preferably 2-hydroxy-tetracosenoic acid , preferably tetracosenoic acid (nervonic acid), preferably 2-hydroxy-tetracosenoic acid in free or bound form in an organism or a part thereof, as mentioned. In one further embodiment the b3231 protein expression is increased together with the increase of another gene of the lipid biosynthesis pathway, preferably with a gene encoding a protein being involved in the production of lipids, preferably of cerebroside. In one embodiment, in the process of the present invention the activity of a b3231 protein is increased or generated, e.g. from Echerischia Coli or a homolog thereof.

**[0023.0.15.15]** Homologues ( = homologs) of the present gene products can be derived from any organisms as long as the homologue confers the herein mentioned activity, in particular, confers an increase in the respective fine chemical amount or content.
In one embodiment, the homolog of the any one of the polypeptides indicated in Table IIA or IIB, column 3, lines 174, 175 or 176 resp. is a homolog having the same or a similar acitivity. In particular an increase of activity confers an increase in the content of the respective fine chemical in the organisms, preferably of heptadecanoic acid. In one embodiment, the homolog is a homolog with a sequence as indicated in Table I or II, column 7, lines 174, 175 and/or 176. In one embodiment, the homolog of one of the polypeptides indicated in Table IIA or IIB, column 3, lines 174, 175 or 176 resp., is derived from an eukaryotic. In one embodiment, the homolog is derived from Fungi. In one embodiment, the homolog of a polypeptide indicated in Table II, column 3, lines 174, 175 or 176 resp., is derived from Ascomyceta. In one embodiment, the homolog of a polypeptide indicated in Table IIA or IIB, column 3, lines 174, 175 or 176 resp., is derived from Saccharomycotina. In one embodiment, the homolog of a polypeptide indicated in Table IIA or IIB, column 3, lines 174, 175 or 176 resp., is derived from Saccharomycetes. In one embodiment, the homolog of a polypeptide indicated in Table IIA or IIB, column 3, lines 174, 175 or 176 resp., is a homolog being derived from Saccharomycetales. In one embodiment, the homolog of a polypeptide indicated in Table IIA or IIB, column 3, lines 174, 175 or 176 resp., is a homolog having the same or a similar acitivity being derived from Saccharomycetaceae. In one embodiment, the homolog of a polypeptide indicated in Table IIA or IIB, column 3, lines 174, 175 or 176 resp., is a homolog having the same or a similar acitivity being derived from Saccharomycetes.
In one embodiment, the homolog of the any one of the polypeptides indicated in Table IIA or IIB, column 3, line 178, 558 or 559 is a homolog having the same or a similar acitivity. In particular an increase of activity confers an increase in the content of the respective fine chemical in the organisms, preferably of 2-hydroxy palmitic acid. In one embodiment, the homolog is a homolog with a sequence as indicated in Table I or II, column 7, line178, 558 or 559. In one embodiment, the homolog of one of the polypeptides indicated in Table IIA or IIB, column 3, line178, 558 or 559 is derived from an eukaryotic. In one embodiment, the homolog is derived from Fungi. In one embodiment, the homolog of a polypeptide indicated in Table IIA or IIB, column 3, line178, 558 or 559 is derived from Ascomyceta. In one embodiment, the homolog of a polypeptide indicated in Table IIA or IIB, column 3, line178, 558 or 559 is derived from Saccharomycotina. In one embodiment, the homolog of a polypeptide indicated in Table IIA or IIB, column 3, line178, 558 or 559 is derived from Saccharomycetes. In one embodiment, the homolog of a polypeptide indicated in Table IIA or IIB, column 3, line178, 558 or 559 is a homolog being derived from Saccharomycetales. In one embodiment, the homolog of a polypeptide indicated in Table IIA or IIB, column 3, line178, 558 or 559 is a homolog having the same or a similar acitivity being derived from Saccharomycetaceae. In one embodiment, the homolog of a polypeptide indicated in Table IIA or IIB, column 3, line178, 558 or 559 is a homolog having the same or a similar acitivity being derived from Saccharomycetes.
In one embodiment, the homolog of the any one of the polypeptides indicated in Table IIA or IIB, column 3, line 558 or 559 is a homolog having the same or a similar acitivity. In particular an increase of activity confers an increase in the content of the respective fine chemical in the organisms, preferably of 2-hydroxy palmitic acid. In one embodiment, the homolog is a homolog with a sequence as indicated in Table I or II, column 7, line 558 or 559. In one embodiment, the homolog of one of the polypeptides indicated in Table IIA or IIB, column 3, line 558 or 559 is derived from a bacteria. In one embodiment, the homolog is derived from Proteobacteria. In one embodiment, the homolog of a polypeptide indicated in Table IIA or IIB, column 3, line 558 or 559 is derived from Gammaproteobacteria. In one embodiment, the homolog of a polypeptide indicated in Table IIA or IIB, column 3, line 558 or 559 is derived from Enterobacteriales. In one embodiment, the homolog of a polypeptide indicated in Table IIA or IIB, column 3, line 558 or 559 is derived from Enterobacteriaceae. In one embodiment, the homolog of a polypeptide indicated in Table IIA or IIB, column 3, line 558 or 559 is a homolog being derived from Escherichia.
In one embodiment, the homolog of the any one of the polypeptides indicated in Table IIA or IIB, column 3, line179 is a homolog having the same or a similar acitivity. In particular an increase of activity confers an increase in the content of the respective fine chemical in the organisms, preferably of 2-hydroxy-tetracosenoic-acid. In one embodiment, the homolog is a homolog with a sequence as indicated in Table I or II, column 7, line179. In one embodiment, the homolog of one of the polypeptides indicated in Table IIA or IIB, column 3, line179 is derived from an eukaryotic. In one embodiment, the homolog is derived from Fungi. In one embodiment, the homolog of a polypeptide indicated in Table IIA or IIB, column 3, line179 is derived from Ascomyceta. In one embodiment, the homolog of a polypeptide indicated in Table IIA or IIB, column 3, line179 is derived from Saccharomycotina. In one embodiment, the homolog of a polypeptide indicated in Table IIA or IIB, column 3, line179 is derived from Saccharomycetes. In one embodiment, the homolog of a polypeptide indicated in Table IIA or IIB, column 3, line179 is a homolog being derived from Saccharomycetales. In one embodiment, the homolog of a polypeptide indicated in Table IIA or IIB, column 3, line179 is a homolog having the same or a similar acitivity being derived from Saccharomycetaceae. In one embodiment, the homolog of a polypeptide indicated in Table IIA or IIB, column 3, line179 is a homolog having the same or a similar acitivity being derived from Saccharomycetes.
In one embodiment, the homolog of the any one of the polypeptides indicated in Table IIA or IIB, column 3, line180 is a homolog having the same or a similar acitivity. In particular an increase of activity confers an increase in the content of the respective fine chemical in the organisms, preferably of hexadecadienoic acid, preferably delta 7, 10 hexadecadienoic acid. In one embodiment, the homolog is a homolog with a sequence as indicated in Table I or II, column 7, line180 . In one embodiment, the homolog of one of the polypeptides indicated in Table IIA or IIB, column 3, line180 is derived from an eukaryotic. In one embodiment, the homolog is derived from Fungi. In one embodiment, the homolog of a polypeptide indicated in Table IIA or IIB, column 3, line180 is derived from Ascomyceta. In one embodiment, the homolog of a polypeptide indicated in Table IIA or IIB, column 3, line180 is derived from Saccharomycotina. In one embodiment, the homolog of a polypeptide indicated in Table IIA or IIB, column 3, line180 is derived from Saccharomycetes. In one embodiment, the homolog of a polypeptide indicated in Table IIA or IIB, column 3, line180 is a homolog being derived from Saccharomycetales. In one embodiment, the homolog of a polypeptide indicated in Table IIA or IIB, column 3, line180 is a homolog having the same or a similar acitivity being derived from Saccharomycetaceae. In one embodiment, the homolog of a polypeptide indicated in Table IIA or IIB, column 3, line180 is a homolog having the same or a similar acitivity being derived from Saccharomycetes.
In one embodiment, the homolog of the any one of the polypeptides indicated in Table IIA or IIB, column 3, lines 181, 182, 183 or 184 resp., is a homolog having the same or a similar acitivity. In particular an increase of activity confers an increase in the content of the respective fine chemical in the organisms, preferably of hexadecatrienoic acid, preferably delta 7, 10 ,13 hexadecatrienoic acid. In one embodiment, the homolog is a homolog with a sequence as indicated in Table I or II, column 7, lines 181, 182, 183 or 184 resp. In one embodiment, the homolog of one of the polypeptides indicated in Table IIA or IIB, column 3, lines 181, 182, 183 or 184 resp., is derived from an eukaryotic. In one embodiment, the homolog is derived from Fungi. In one embodiment, the homolog of a polypeptide indicated in Table IIA or IIB, column 3, lines 181, 182, 183 or 184 resp., is derived from Ascomyceta. In one embodiment, the homolog of a polypeptide indicated in Table IIA or IIB, column 3, lines 181, 182, 183 or 184 resp., is derived from Saccharomycotina. In one embodiment, the homolog of a polypeptide indicated in Table IIA or IIB, column 3, lines 181, 182, 183 or 184 resp., is derived from Saccharomycetes. In one embodiment, the homolog of a polypeptide indicated in Table IIA or IIB, column 3, lines 181,182,183 or 184 resp., is a homolog being derived from Saccharomycetales. In one embodiment, the homolog of a polypeptide indicated in Table IIA or IIB, column 3, lines 181, 182, 183 or 184 resp., is a homolog having the same or a similar acitivity being derived from Saccharomycetaceae. In one embodiment, the homolog of a polypeptide indicated in Table IIA or IIB, column 3, lines 181, 182, 183 or 184 resp., is a homolog having the same or a similar acitivity being derived from Saccharomycetes.
In one embodiment, the homolog of the any one of the polypeptides indicated in Table IIA or IIB, column 3, line 177 is a homolog having the same or a similar acitivity. In particular an increase of activity confers an increase in the content of the respective fine chemical in the organisms preferably of heptadecanoic acid. In one embodiment, the homolog is a homolog with a sequence as indicated in Table I or II, column 7, line 177, resp. In one embodiment, the homolog of one of the polypeptides indicated in Table IIA or IIB, column 3, line 177 is derived from an bacteria. In one embodiment, the homolog of a polypeptide indicated in Table IIA or IIB, column 3, line 177 is derived from Proteobacteria. In one embodiment, the homolog of a polypeptide indicated in Table IIA or IIB, column 3, line 177 is a homolog having the same or a similar acitivity being derived from Gammaproteobacteria. In one embodiment, the homolog of a polypeptide indicated in Table IIA or IIB, column 3, line 177 is derived from Enterobacteriales. In one embodiment, the homolog of a polypeptide indicated in Table IIA or IIB, column 3, line 177 is a homolog being derived from Enterobacteriaceae. In one embodiment, the homolog of a polypeptide indicated in Table IIA or IIB, column 3, line 177 is a homolog having the same or a similar acitivity and being derived from Escherichia.
In one embodiment, the homolog of the any one of the polypeptides indicated in Table IIA or IIB, column 3, line 185 is a homolog having the same or a similar acitivity. In particular an increase of activity confers an increase in the content of the respective fine chemical in the organisms, preferably of hexadecatrienoic acid, preferably delta 7, 10 ,13 hexadecatrienoic acid. In one embodiment, the homolog is a homolog with a sequence as indicated in Table I or II, column 7, line185. In one embodiment, the homolog of one of the polypeptides indicated in Table IIA or IIB, column 3, line185 resp. is derived from a bacteria. In one embodiment, the homolog of a polypeptide indicated in Table IIA or IIB, column 3, line185 resp. is derived from Proteobacteria. In one embodiment, the homolog of a polypeptide indicated in Table IIA or IIB, column 3, line185 is a homolog having the same or a similar acitivity being derived from Gammaproteobacteria. In one embodiment, the homolog of a polypeptide indicated in Table IIA or IIB, column 3, line185 is derived from Enterobacteriales. In one embodiment, the homolog of a polypeptide indicated in Table IIA or IIB, column 3, line185 is a homolog being derived from Enterobacteriaceae. In one embodiment, the homolog of a polypeptide indicated in Table IIA or IIB, column 3, lines185 is a homolog having the same or a similar acitivity and being derived from Escherichia.
In one embodiment, the homolog of the any one of the polypeptides indicated in Table II, column 3, line 560 to 563 is a homolog having the same or a similar acitivity. In particular an increase of activity confers an increase in the content of the respective fine chemical in the organisms, preferably of C24:1 fatty acid, preferably C24:1 delta 15 fatty acid. In one embodiment, the homolog is a homolog with a sequence as indicated in Table I or II, column 7, line 560 to 563. In one embodiment, the homolog of one of the polypeptides indicated in Table IIA or IIB, column 3, line 560 to 563 resp. is derived from an bacteria. In one embodiment, the homolog of a polypeptide indicated in Table IIA or IIB, column 3, line 560 to 563 resp. is derived from Proteobacteria. In one embodiment, the homolog of a polypeptide indicated in Table IIA or IIB, column 3, line 560 to 563 is a homolog having the same or a similar acitivity being derived from Gammaproteobacteria. In one embodiment, the homolog of a polypeptide indicated in Table IIA or IIB, column 3, line560 to 563 is derived from Enterobacteriales. In one embodiment, the homolog of a polypeptide indicated in Table IIA or IIB, column 3, line 560 to 563 is a homolog being derived from Enterobacteriaceae. In one embodiment, the homolog of a polypeptide indicated in Table IIA or IIB, column 3, lines 560 to 563 is a homolog having the same or a similar acitivity and being derived from Escherichia.

**[0023.1.15.15]** Homologs of the polypeptide indicated in Table IIA or IIB, column 3, lines 174 to 185, 558 to 563 may be the polypetides encoded by the nucleic acid molecules indicated in Table IA or IB , column 7, lines 174 to 185, 558 to 563 , resp., or may be the polypeptides indicated in Table IIA or IIB, column 7, lines 174 to 185, 558 to 563 , resp. Homologs of the polypeptides polypeptide indicated in Table IIA or IIB, column 3, lines 174 to 185, 558 to 563 may be the polypetides encoded by the nucleic acid molecules indicated in Table IA or IB , column 7, lines 174 to 185, 558 to 563 or may be the polypeptides indicated in Table IIA or IIB, column 7, lines 174 to 185, 558 to 563 .
Homologs of the polypeptides indicated in Table IIA ot IIB, column 3, lines 174 to 177 may be the polypetides encoded by the nucleic acid molecules indicated in Table IA or IB , column 7, lines 174 to 177, respectively or may be the polypeptides indicated in Table IIA or IIB, column 7, lines 174 to 177, having a heptadecanoic acid content- and/or amount-increasing activity.
Homologs of the polypeptides indicated in Table IIA or IIB, column 3, line 179 may be the polypetides encoded by the nucleic acid molecules indicated in Table IA or IB , column 7, line 179, respectively or may be the polypeptides indicated in Table IIA or IIB, column 7, line 179, having a 2-hydroxy-tetracosenoic-acid content- and/or amount-increasing activity.
Homologs of the polypeptides indicated in Table IIA or IIB, column 3, line 180 may be the polypetides encoded by the nucleic acid molecules indicated in Table IA or IB, column 7, line 180, respectively or may be the polypeptides indicated in Table IIA or IIB, column 7, line 180, having a hexadecadienoic acid, preferably delta 7, 10 hexadecadienoic acid content- and/or amount-increasing activity.
Homologs of the polypeptides indicated in Table IIA or IIB, column 3, lines 181 to 185 may be the polypetides encoded by the nucleic acid molecules indicated in Table IA or IB , column 7, lines 181 to 185, respectively or may be the polypeptides indicated in Table IIA or IIB, column 7, lines 181 to 185, having a hexadecatrienoic acid, preferably delta 7, 10 ,13 hexadecatrienoic acid content- and/or amount-increasing activity.
Homologs of the polypeptides indicated in Table IIA or IIB, column 3, lines 558, 559 may be the polypetides encoded by the nucleic acid molecules indicated in Table IA or IB, column 7, lines 558, 559, respectively or may be the polypeptides indicated in Table IIA or IIB, column 7, lines 558, 559 having 2-hydroxy-palmitic acid content- and/or amount-increasing activity.
Homologs of the polypeptides indicated in Table IIA or IIB, column 3, lines 560 to 563 may be the polypetides encoded by the nucleic acid molecules indicated in Table IA or IB, column 7, lines 560 to 563, respectively or may be the polypeptides indicated in Table IIA or IIB, column 7, lines 560 to 563, having a C24:1 fatty acid, preferably C24:1delta 15 fatty acid content- and/or amount-increasing activity.

**[0024.0.0.15] see [0024.0.0.0]**

**[0025.0.15.15]**In accordance with the invention, a protein or polypeptide has the "activity of an protein of the invention", e.g. the activity of a protein indicated in Table IIA or IIB, column 3, lines 174 to 177 and/or line 178 and/or line 179 and/or line180 and/or lines 181 to 185 and/or 558, 559 and/or 560 to 563 resp., if its *de novo* activity, or its increased expression directly or indirectly leads to an increased fatty acid level, in particular to a increased heptadecanoic acid and/or 2-hydroxy palmitic acid and/or 2-hydroxy-tetracosenoic-acid and/or hexadecadienoic acid and/or 2-hydroxy-palmitic acid and/or C24:1 fatty acid, preferably delta 7, 10 hexadecadienoic acid and/or hexadecatrienoic acid, preferably delta 7, 10 ,13 hexadecatrienoic acid resp., in the organism or a part thereof, preferably in a cell of said organism. In a preferred embodiment, the protein or polypeptide has the above-mentioned additional activities of a protein indicated in Table IIA or IIB, column 3, lines 174 to 177 and/or line 178 and/or line 179 and/or line180 and/or lines 181 to 185 and/or 558, 559 and/or 560 to 563 resp. Throughout the specification the activity or preferably the biological activity of such a protein or polypeptide or an nucleic acid molecule or sequence encoding such protein or polypeptide is identical or similar if it still has the biological or enzymatic activity of any one of the proteins indicated in Table IIA or IIB, column 3, lines 174 to 177 and/or line 178 and/or line 179 and/or line180 and/or lines 181 to 185 resp., or which has at least 10% of the original enzymatic activity, preferably 20%, particularly preferably 30%, most particularly preferably 40% in comparison to any one of the proteins indicated in Table IIA or IIB, column 3, lines 174 to 176, or 178, or 179, or 180 or 181 to 184 of Saccharomyces cerevisiae and/or any one of the proteins indicated in Table IIA or IIB, column 3, lines 177 or 185 or 558, 559 or 560 to 563 of E. coli K12.
In one embodiment, the polypeptide of the invention confers said activity, e.g. the increase of the respective fine chemical in an organism or a part thereof, if it is derived from an organism, which is evolutionary close to the organism indicated in Table IA or IB , column 4 and is expressed in an organism, which is evolutionary distant to the origin organism. For example origin and expressing organism are derived from different families, orders, classes or phylums whereas origin and the organism indicated in Table IA or IB , column 4 are derived from the same families, orders, classes or phylums.

**[0026.0.0.15] to [0033.0.0.15] see [0026.0.0.0] to [0033.0.0.0]**

**[0034.0.15.15]**Preferably, the reference, control or wild type differs form the subject of the present invention only in the cellular activity of the polypeptide of the invention, e.g. as result of an increase in the level of the nucleic acid molecule of the present invention or an increase of the specific activity of the polypeptide of the invention. E.g., it differs by or in the expression level or activity of an protein having the activity of a protein as indicated in Table IIA or IIB, column 3, lines 174 to 177 and/or line 178 and/or line 179 and/or line180 and/or lines 181 to 185 and/or lines 558, 559 and/or lines 560 to 563 resp., or being encoded by a nucleic acid molecule indicated in Table IA or IB , column 5, lines 174 to 177 and/or line 178 and/or line 179 and/or line180 and/or lines 181 to 185 and/or lines 558, 559 and/or lines 560 to 563 resp., or its homologs, e.g. as indicated in Table I, column 7, lines 174 to 177 and/or line 178 and/or line 179 and/or lines 181 to 185 and/or lines 558, 559 and/or lines 560 to 563 resp., its biochemical or genetical causes and therefore shows the increased amount of the fine chemical.

**[0035.0.0.15] to [0036.0.0.15] see [0035.0.0.0] to [0036.0.0.0]**

**[0037.0.15.15]** A series of mechanisms exists via which a modification of the a protein, e.g. the polypeptide of the invention can directly or indirectly affect the yield, production and/or production efficiency of the fatty acid.

**[0038.0.0.15] to [0044.0.0.15] see [0038.0.0.0] to [0044.0.0.0]**

**[0045.0.15.15]** In one embodiment, the activity of the Saccharomyces cerevisiae protein YDR513W or its homologs, e.g. an activity of a glutathione reductase, preferably of the glutaredoxin superfamily, e.g. as indicated in Table II, columns 5 or 7, line 174, is increased conferring an increase of the respective fine chemical, preferably of the heptadecanoic acid between 24% and 97% or more.
In one embodiment, the activity of the Saccharomyces cerevisiae protein YER156C or its homologs, e.g. an activity of a YER156C protein, e.g. as indicated in Table II, columns 5 or 7, line 175, is increased conferring an increase of the respective fine chemical, preferably of the heptadecanoic acid between 20% and 49% or more.
In one embodiment, the activity of the Saccharomyces cerevisiae protein YER173W or its homologs, e.g. an activity of a checkpoint protein, involved in the activation of the DNA damage and meiotic pachytene checkpoints, e.g. as indicated in Table II, columns 5 or 7, line 178, is increased conferring an increase of the respective fine chemical, preferably of the 2-hydroxy palmitic acid between 26% and 394% or more.
In one embodiment, the activity of the Saccharomyces cerevisiae protein YFR042W or its homologs, e.g. an activity of a protein, which is required for cell viability, preferably of the Saccharomyces cerevisiae probable membrane protein YFR042w superfamily, e.g. as indicated in Table II, columns 5 or 7, line 179, is increased conferring an increase of the respective fine chemical, preferably of the 2-hydroxy-tetracosenoic-acid between 28% and 56% or more.
In one embodiment, the activity of the Saccharomyces cerevisiae protein YGL205W or its homologs, e.g. an activity of a fatty-acyl coenzyme A oxidase, preferably of the acyl-CoA oxidase superfamily , e.g. as indicated in Table II, columns 5 or 7, line 181, is increased conferring an increase of the respective fine chemical, preferably of the hexadecatrienoic acid, preferably delta 7, 10 ,13 hexadecatrienoic acid between 14% and 16% or more.
In one embodiment, the activity of the Saccharomyces cerevisiae protein YIL150C or its homologs, e.g. an activity of a protein required for S-phase (DNA synthesis) initiation or completion and/or chromatin binding protein, e.g. as indicated in Table II, columns 5 or 7, line 182, is increased conferring an increase of the respective fine chemical, preferably of the hexadecatrienoic acid, preferably delta 7, 10 ,13 hexadecatrienoic acid between 150% and 205%, preferably of 224% or more.
In one embodiment, the activity of the Saccharomyces cerevisiae protein YKL132C or its homologs, e.g. an activity of a folyl-polyglutamate synthase, preferably of the folylpolyglutamate synthase superfamily, e.g. as indicated in Table II, columns 5 or 7, line 183, is increased conferring an increase of the respective fine chemical, preferably of the hexadecatrienoic acid, preferably delta 7, 10 ,13 hexadecatrienoic acid between 13% and 56% or more.
In one embodiment, the activity of the Saccharomyces cerevisiae protein YLR255C or its homologs, e.g. an activity of of Saccharomyces hypothetical protein YLR255c superfamily, e.g. as indicated in Table II, columns 5 or 7, line 176, is increased conferring an increase of the respective fine chemical, preferably of heptadecanoic acid between 20% and 27% or more.
In one embodiment, the activity of the Saccharomyces cerevisiae protein YOR317W or its homologs, e.g. an activity of a long chain fatty acyl:CoA synthetase, preferably of the long-chain-fatty-acid-CoA ligase superfamily , e.g. as indicated in Table II, columns 5 or 7, line 180, is increased conferring an increase of the respective fine chemical, preferably of the hexadecadienoic acid, preferably delta 7, 10 hexadecadienoic acid between 19% and 69% or more.
In one embodiment, the activity of the Saccharomyces cerevisiae protein YOR344C or its homologs, e.g. an activity of a serine-rich protein, putatively involved in glycolytic gene expression , e.g. as indicated in Table II, columns 5 or 7, line 184, is increased conferring an increase of the respective fine chemical, preferably of the hexadecatrienoic acid, preferably delta 7, 10 ,13 hexadecatrienoic acid between 12% and 20% or more.
In one embodiment, the activity of the Escherichia coli K12 protein b1829 or its homologs, e.g. an activity of a heat shock protein with protease activity, preferably of the heat-shock protein htpX superfamily, e.g. as indicated in Table II, columns 5 or 7, line 177, is increased conferring an increase of the respective fine chemical, preferably of the heptadecanoic acid between 20% and 133% or more.
In one embodiment, the activity of the Escherichia coli K12 protein b3430 or its homologs, e.g. an activity of a transcriptional regulator of glucose-1-phosphate adenylyltransferase, preferably of the a glucose-1-phosphate adenylyltransferase superfamily , e.g. as indicated in Table II, columns 5 or 7, line 185, is increased conferring an increase of the respective fine chemical, preferably of the hexadecatrienoic acid, preferably delta 7, 10 ,13 hexadecatrienoic acid between 12% and 20% or more.
In one embodiment, the activity of the Escherichia coli K12 protein b0057 or its homologs, e.g. as indicated in Table II, columns 5 or 7, line 560, is increased conferring an increase of the respective fine chemical, preferably of the C24:1 fatty acid between 22% and 37% or more.
In one embodiment, the activity of the Escherichia coli K12 protein b0161 or its homologs, e.g. an activity of a periplasmic serine protease (heat shock protein), e.g. as indicated in Table II, columns 5 or 7, line 558, is increased conferring an increase of the respective fine chemical, preferably of the 2-hydroxy-palmitic acid between 21 % and 48% or more.
In one embodiment, the activity of the Escherichia coli K12 protein b0758 or its homologs, e.g. an activity of a galactose-1-phosphate uridylyltransferase protein, e.g. as indicated in Table II, columns 5 or 7, line 559, is increased conferring an increase of the respective fine chemical, preferably of the 2-hydroxy-palmitic acid between 19% and 38% or more.
In one embodiment, the activity of the Escherichia coli K12 protein b1097 or its homologs, e.g. an activity of a thymidylate kinase protein, e.g. as indicated in Table II, columns 5 or 7, line 561, is increased conferring an increase of the respective fine chemical, preferably of the C24:1 fatty acid between 23% and 41% or more.
In one embodiment, the activity of the Escherichia coli K12 protein b2078 or its homologs, e.g. an activity of a sensory histidine kinase in two-component regulatory system protein, e.g. as indicated in Table II, columns 5 or 7, line 562, is increased conferring an increase of the respective fine chemical, preferably of the the C24:1 fatty acid between 23% and 41% or more.
In one embodiment, the activity of the Escherichia coli K12 protein b3231 or its homologs, e.g. an activity of a 50S ribosomal subunit protein L13 protein, e.g. as indicated in Table II, columns 5 or 7, line 563, is increased conferring an increase of the respective fine chemical, preferably of the the C24:1 fatty acid between 23% and 48% or more.

**[0046.0.15.15]** In one embodiment, the activity of the Saccharomyces cerevisiae protein YDR513W or its homologs, e.g. an activity of a glutathione reductase, preferably of the glutaredoxin superfamily, e.g. as indicated in Table II, columns 5 or 7, line 174, confers an increase of the respective fine chemical and of further fatty acid activity having-compounds or lipids, preferably glycerophospholipids, sphingolipids and/or galactolipids, or their precursors.
In one embodiment, the activity of the Saccharomyces cerevisiae protein YER156C or its homologs, e.g. an activity of a YER156C protein, e.g. as indicated in Table II, columns 5 or 7, line 175" confers an increase of the respective fine chemical and of further fatty acid activity having-compounds or lipids, preferably glycerophospholipids, sphingolipids and/or galactolipids, or their precursors.
In one embodiment, the activity of the Saccharomyces cerevisiae protein YER173W or its homologs, e.g. an activity of a checkpoint protein, involved in the activation of the DNA damage and meiotic pachytene checkpoints, e.g. as indicated in Table II, columns 5 or 7, line 178, confers an increase of the respective fine chemical and of further fatty acid activity having-compounds or lipids, preferably glycerophospholipids, sphingolipids and/or galactolipids, or their precursors.
In one embodiment, the activity of the Saccharomyces cerevisiae protein YFR042W or its homologs, e.g. an activity of a protein, which is required for cell viability, preferably of the Saccharomyces cerevisiae probable membrane protein YFR042w superfamily, e.g. as indicated in Table II, columns 5 or 7, line 179, confers an increase of the respective fine chemical and of further fatty acid activity having-compounds or lipids, preferably glycerophospholipids, sphingolipids and/or galactolipids, or their precursors.
In one embodiment, the activity of the Saccharomyces cerevisiae protein YGL205W or its homologs, e.g. an activity of a fatty-acyl coenzyme A oxidase, preferably of the acyl-CoA oxidase superfamily , e.g. as indicated in Table II, columns 5 or 7, line 181 , confers an increase of the respective fine chemical and of further fatty acid activity having-compounds or lipids, preferably glycerophospholipids, sphingolipids and/or galactolipids, or their precursors.
In one embodiment, the activity of the Saccharomyces cerevisiae protein YIL150C or its homologs, e.g. an activity of a protein required for S-phase (DNA synthesis) initiation or completion and/or chromatin binding protein, e.g. as indicated in Table II, columns 5 or 7, line 182 , confers an increase of the respective fine chemical and of further fatty acid activity having-compounds or lipids, preferably glycerophospholipids, sphingolipids and/or galactolipids, or their precursors.
In one embodiment, the activity of the Saccharomyces cerevisiae protein YKL132C or its homologs, e.g. an activity of a folyl-polyglutamate synthase, preferably of the folylpolyglutamate synthase superfamily, e.g. as indicated in Table II, columns 5 or 7, line 183 , confers an increase of the respective fine chemical and of further fatty acid activity having-compounds or lipids, preferably glycerophospholipids, sphingolipids and/or galactolipids, or their precursors.
In one embodiment, the activity of the Saccharomyces cerevisiae protein YLR255C or its homologs, e.g. an activity of the Saccharomyces hypothetical protein YLR255c superfamily, e.g. as indicated in Table II, columns 5 or 7, line 176, confers an increase of the respective fine chemical and of further fatty acid activity having-compounds or lipids, preferably glycerophospholipids, sphingolipids and/or galactolipids, or their precursors.
In one embodiment, the activity of the Saccharomyces cerevisiae protein YOR317W or its homologs, e.g. an activity of a long chain fatty acyl:CoA synthetase, preferably of the long-chain-fatty-acid-CoA ligase superfamily , e.g. as indicated in Table II, columns 5 or 7, line 180, confers an increase of the respective fine chemical and of further fatty acid activity having-compounds or lipids, preferably glycerophospholipids, sphingolipids and/or galactolipids, or their precursors.
In one embodiment, the activity of the Saccharomyces cerevisiae protein YOR344C or its homologs, e.g. an activity of a serine-rich protein, putatively involved in glycolytic gene expression , e.g. as indicated in Table II, columns 5 or 7, line 184 , confers an increase of the respective fine chemical and of further fatty acid activity having-compounds or lipids, preferably glycerophospholipids, sphingolipids and/or galactolipids, or their precursors.
In one embodiment, the activity of the Escherichia coli K12 protein b1829 or its homologs, e.g. an activity of a heat shock protein with protease activity, preferably of the heat-shock protein htpX superfamily, e.g. as indicated in Table II, columns 5 or 7, line 177, confers an increase of the respective fine chemical and of further fatty acid activity having-compounds or lipids, preferably glycerophospholipids, sphingolipids and/or galactolipids, or their precursors.
In one embodiment, the activity of the Escherichia coli K12 protein b3430 or its homologs, e.g. an activity of a transcriptional regulator of glucose-1-phosphate adenylyltransferase, preferably of the a glucose-1-phosphate adenylyltransferase superfamily , e.g. as indicated in Table II, columns 5 or 7, line 185, confers an increase of the respective fine chemical and of further fatty acid activity having-compounds or lipids, preferably glycerophospholipids, sphingolipids and/or galactolipids, compounds or their precursors.
In one embodiment, the activity of the Escherichia coli K12 protein b0057 or its homologs, e.g. as indicated in Table II, columns 5 or 7, line 560, confers an increase of the respective fine chemical and of further fatty acid activity having-compounds or lipids, preferably glycerophospholipids, sphingolipids and/or galactolipids, compounds or their precursors.
In one embodiment, the activity of the Escherichia coli K12 protein b0161 or its homologs, e.g. a periplasmic serine protease (heat shock protein) activity, e.g. as indicated in Table II, columns 5 or 7, line 558, confers an increase of the respective fine chemical and of further fatty acid activity having-compounds or lipids, preferably glycerophospholipids, sphingolipids and/or galactolipids, compounds or their precursors.
In one embodiment, the activity of the Escherichia coli K12 protein b0758 or its homologs, e.g. a galactose-1-phosphate uridylyltransferase activity, e.g. as indicated in Table II, columns 5 or 7, line 559, confers an increase of the respective fine chemical and of further fatty acid activity having-compounds or lipids, preferably glycerophospholipids, sphingolipids and/or galactolipids, compounds or their precursors.
In one embodiment, the activity of the Escherichia coli K12 protein b1097 or its homologs, e.g. a thymidylate kinase activity, e.g. as indicated in Table II, columns 5 or 7, line 561, confers an increase of the respective fine chemical and of further fatty acid activity having-compounds or lipids, preferably glycerophospholipids, sphingolipids and/or galactolipids, compounds or their precursors.
In one embodiment, the activity of the Escherichia coli K12 protein b2078 or its homologs, e.g. a sensory histidine kinase in two-component regulatory system activity, e.g. as indicated in Table II, columns 5 or 7, line 562, confers an increase of the respective fine chemical and of further fatty acid activity having-compounds or lipids, preferably glycerophospholipids, sphingolipids and/or galactolipids, compounds or their precursors.
In one embodiment, the activity of the Escherichia coli K12 protein b3231 or its homologs, e.g. a 50S ribosomal subunit protein L13 activity, e.g. as indicated in Table II, columns 5 or 7, line 563, confers an increase of the respective fine chemical and of further fatty acid activity having-compounds or lipids, preferably glycerophospholipids, sphingolipids and/or galactolipids, compounds or their precursors.

**[0047.0.0.15] and [0048.0.0.15] see [0047.0.0.0] and [0048.0.0.0]**

**[0049.0.15.15]** A protein having an activity conferring an increase in the amount or level of the heptadecanoic acid preferably has the structure of the polypeptide described herein. In a particular embodiment, the polypeptides used in the process of the present invention or the polypeptide of the present invention comprises the sequence of a consensus sequence as shown in SEQ ID NO: 15686, 15687and/or 15688 or 19697, 15698, 15699, 15700, 15701 and/or 15702 or 15703, 15704 and/or 15705 and/or the sequence of a consensus sequence as indicated in Table IV, columns 5 or 7, lines 174 to 177 and/or the sequence of a polypeptide as indicated in Table II, columns 5 or 7, lines 174 to 177, or of a functional homologue thereof as described herein, or of a polypeptide encoded by the nucleic acid molecule characterized herein or the nucleic acid molecule according to the invention, for example by a nucleic acid molecule as indicated in Table I, columns 5 or 7, lines 174 to 177 or its herein described functional homologues and has the herein mentioned activity conferring an increase in the heptadecanoic acid level.
A protein having an activity conferring an increase in the amount or level of the 2-hydroxy-tetracosenoic-acid preferably has the structure of the polypeptide described herein. In a particular embodiment, the polypeptides used in the process of the present invention or the polypeptide of the present invention comprises the sequence of a consensus sequence as shown in SEQ ID NO: 15691 and/or 15692 and/or the sequence of a consensus sequence as indicated in Table IV, columns 5 or 7, line 179 and/or the sequence of a polypeptide as indicated in Table II, columns 5 or 7, line 179 or of a functional homologue thereof as described herein, or of a polypeptide encoded by the nucleic acid molecule characterized herein or the nucleic acid molecule according to the invention, for example by a nucleic acid molecule as indicated in Table I, columns 5 or 7, line 179 or its herein described functional homologues and has the herein mentioned activity confering an increase in the 2-hydroxy-tetracosenoic-acid level.
A protein having an activity conferring an increase in the amount or level of the hexadecadienoic acid, preferably delta 7, 10 hexadecadienoic acid preferably has the structure of the polypeptide described herein. In a particular embodiment, the polypeptides used in the process of the present invention or the polypeptide of the present invention comprises the sequence of a consensus sequence as shown in SEQ ID NO: 15661, 16662, 15663, 15664, 15665 and/or 15666 and/or the sequence of a consensus sequence as indicated in Table IV, columns 5 or 7, line 180 and/or the sequence of a polypeptide as indicated in Table II, columns 5 or 7, line 180 or of a functional homologue thereof as described herein, or of a polypeptide encoded by the nucleic acid molecule characterized herein or the nucleic acid molecule according to the invention, for example by a nucleic acid molecule as indicated in Table I, columns 5 or 7, line 180 or its herein described functional homologues and has the herein mentioned activity confering an increase in the hexadecadienoic acid, preferably delta 7, 10 hexadecadienoic acid level.
A protein having an activity conferring an increase in the amount or level of the hexadecatrienoic acid, preferably delta 7, 10 ,13 hexadecatrienoic acid preferably has the structure of the polypeptide described herein. In a particular embodiment, the polypeptides used in the process of the present invention or the polypeptide of the present invention comprises the sequence of a consensus sequence as shown in SEQ ID NO: 15706 and/or 15707 or 15689 and/or, 15690 or 15682, 15683, 15684 and/or, 15685 or 15658, 15659 and/or 15660 or 15677, 15678, 15679, 15680 and/or 15681 and/or the sequence of a consensus sequence as indicated in Table IV, columns 5 or 7, lines 181 to 185 and/or the sequence of a polypeptide as indicated in Table II, columns 5 or 7, lines 181 to 185 or of a functional homologue thereof as described herein, or of a polypeptide encoded by the nucleic acid molecule characterized herein or the nucleic acid molecule according to the invention, for example by a nucleic acid molecule as indicated in Table I, columns 5 or 7, lines 181 to 185 or its herein described functional homologues and has the herein mentioned activity confering an increase in the hexadecatrienoic acid, preferably delta 7, 10 ,13 hexadecatrienoic acid level.
A protein having an activity conferring an increase in the amount or level of the 2-hydroxy-palmitic acid, preferably has the structure of the polypeptide described herein. In a particular embodiment, the polypeptides used in the process of the present invention or the polypeptide of the present invention comprises the sequence of a consensus sequence as shown in SEQ ID NO: 85367, 85368, 85369, 85370, 85371, 85372 or 85515, 85516, 85517, 85518 and/or the sequence of a consensus sequence as indicated in Table IV, columns 5 or 7, lines 558 or 559 and/or the sequence of a polypeptide as indicated in Table II, columns 5 or 7, lines 558 or 559 or of a functional homologue thereof as described herein, or of a polypeptide encoded by the nucleic acid molecule characterized herein or the nucleic acid molecule according to the invention, for example by a nucleic acid molecule as indicated in Table I, columns 5 or 7, lines 558 or 559 or its herein described functional homologues and has the herein mentioned activity confering an increase in the 2-hydroxy-palmitic acid level.
A protein having an activity conferring an increase in the amount or level of the C24:1 fatty acid, preferably C24:1 delta 15 fatty acid preferably has the structure of the polypeptide described herein. In a particular embodiment, the polypeptides used in the process of the present invention or the polypeptide of the present invention comprises the sequence of a consensus sequence as shown in SEQ ID NO: 85801, 85802, 85803 or 86104, 86105, 86106 or 86427, 86428, 86429 and/or the sequence of a consensus sequence as indicated in Table IV, columns 5 or 7, lines 560 to 563 and/or the sequence of a polypeptide as indicated in Table II, columns 5 or 7, lines 560 to 563 or of a functional homologue thereof as described herein, or of a polypeptide encoded by the nucleic acid molecule characterized herein or the nucleic acid molecule according to the invention, for example by a nucleic acid molecule as indicated in Table I, columns 5 or 7, lines 560 to 563 or its herein described functional homologues and has the herein mentioned activity confering an increase in the C24:1 fatty acid, preferably C24:1 fatty acid delta15 level.

**[0050.0.15.15]**For the purposes of the present invention, the term "heptadecanoic acid", "2-hydroxy palmitic acid", "2-hydroxy-tetracosenoic-acid", "hexadecadienoic acid", preferably "delta 7, 10 hexadecadienoic acid" and/or "hexadecatrienoic acid", preferably "delta 7, 10 ,13 hexadecatrienoic acid" and/or C24:1 fatty acid also encompasses the corresponding salts, such as, for example, the potassium or sodium salts of the above named fatty acids or the salts of the above named fatty acids with amines such as diethylamine or the esters the above named fatty acids.

**[0051.0.15.15]** Owing to the biological activity of the proteins which are used in the process according to the invention and which are encoded by nucleic acid molecules according to the invention, it is possible to produce compositions comprising the fine chemical, i.e. an increased amount of the free chemical free or bound, e.g fatty acid compositions. Depending on the choice of the organism used for the process according to the present invention, for example a microorganism or a plant, compositions or mixtures of various fatty acids can be produced.

**[0052.0.0.15] see [0052.0.0.0]**

**[0053.0.15.15]**In one embodiment, the process of the present invention comprises one or more of the following steps
a) stabilizing a protein conferring the increased expression of a protein encoded by the nucleic acid molecule of the invention or of the polypeptide of the invention, e.g. of a polypeptide having an activity of a protein as indicated in Table II, column 3, lines 174 to 177 and/or line 178 and/or line 179 and/or Iine180 and/or lines 181 to 185 or its homologs activity, e.g. as indicated in Table II, columns 5 or 7, lines 174 to 177 and/or line 178 and/or line 179 and/or Iine180 and/or lines 181 to 185 and/or line 558, 559 and/or line 560 to 563 having herein-mentioned the respective fine chemical-increasing activity;
b) stabilizing a mRNA conferring the increased expression of a protein encoded by the nucleic acid molecule of the invention, e.g. of a polypeptide having an activity of a protein as indicated in Table II, column 3, lines 174 to 177 and/or line 178 and/or line 179 and/or line180 and/or lines 181 to 185, or its homologs activity, e.g. as indicated in Table II, columns 5 or 7, lines 174 to 177 and/or line 178 and/or line 179 and/or line180 and/or lines 181 to 185 and/or line 558, 559 and/or line 560 to 563, or of a mRNA encoding the polypeptide of the present invention having herein-mentioned the respective fine chemical increasing activity;
c) increasing the specific activity of a protein conferring the increased expression of a protein encoded by the nucleic acid molecule of the invention or of the polypeptide of the present invention having herein-mentioned the respective fine chemical increasing activity, e.g. of a polypeptide having an activity of a protein as indicated in Table II, column 3, lines 174 to 177 and/or line 178 and/or line 179 and/or line180 and/or lines 181 to 185 or its homologs activity, e.g. as indicated in Table II, columns 5 or 7, lines 174 to 177 and/or line 178 and/or line 179 and/or line180 and/or lines 181 to 185 and/or line 558, 559 and/or line 560 to 563 or decreasing the inhibitory regulation of the polypeptide of the invention;
d) generating or increasing the expression of an endogenous or artificial transcription factor mediating the expression of a protein conferring the increased expression of a protein encoded by the nucleic acid molecule of the invention or of the polypeptide of the invention having herein-mentioned the respective fine chemical increasing activity, e.g. of a polypeptide having an activity of a protein as indicated in Table II, column 3, lines 174 to 177 and/or line 178 and/or line 179 and/or line180 and/or lines 181 to 185, and/or line 558, 559 and/or line 560 to 563 or its homologs activity, e.g. as indicated in Table II, columns 5 or 7, lines 174 to 177 and/or line 178 and/or line 179 and/or line180 and/or lines 181 to 185 and/or line 558, 559 and/or line 560 to 563;
e) stimulating activity of a protein conferring the increased expression of a protein encoded by the nucleic acid molecule of the present invention or a polypeptide of the present invention having herein-mentioned the respective fine chemical increasing activity, e.g. of a polypeptide having an activity of a protein as indicated in Table II, column 3, lines 174 to 177 and/or line 178 and/or line 179 and/or line180 and/or lines 181 to 185, or its homologs activity, e.g. as indicated in Table II, columns 5 or 7, lines 174 to 177 and/or line 178 and/or line 179 and/or line180 and/or lines 181 to 185 and/or line 558, 559 and/or line 560 to 563, by adding one or more exogenous inducing factors to the organismus or parts thereof;
f) expressing a transgenic gene encoding a protein conferring the increased expression of a polypeptide encoded by the nucleic acid molecule of the present invention or a polypeptide of the present invention, having herein-mentioned the respective fine chemical increasing activity, e.g. of a polypeptide having an activity of a protein as indicated in Table II, column 3, lines 174 to 177 and/or line 178 and/or line 179 and/or line180 and/or lines 181 to 185, or its homologs activity, e.g. as indicated in Table II, columns 5 or 7, lines 174 to 177 and/or line 178 and/or line 179 and/or line180 and/or lines 181 to 185 and/or line 558, 559 and/or line 560 to 563;
g) increasing the copy number of a gene conferring the increased expression of a nucleic acid molecule encoding a polypeptide encoded by the nucleic acid molecule of the invention or the polypeptide of the invention having herein-mentioned the respective fine chemical increasing activity, e.g. of a polypeptide havingan an activity of a protein as indicated in Table II, column 3, lines 174 to 177 and/or line 178 and/or line 179 and/or line180 and/or lines 181 to 185, or its homologs activity, e.g. as indicated in Table II, columns 5 or 7, lines 174 to 177 and/or line 178 and/or line 179 and/or line180 and/or lines 181 to 185 and/or line 558, 559 and/or line 560 to 563;
h) Increasing the expression of the endogenous gene encoding the polypeptide of the invention, e.g. a polypeptide having an activity of a protein as indicated in Table II, column 3, lines 174 to 177 and/or line 178 and/or line 179 and/or line180 and/or lines 181 to 185, or its homologs activity, e.g. as indicated in Table II, columns 5 or 7, lines 174 to 177 and/or line 178 and/or line 179 and/or line180 and/or lines 181 to 185 and/or line 558, 559 and/or line 560 to 563 by adding positive expression or removing negative expression elements, e.g. homologous recombination can be used to either introduce positive regulatory elements like for plants the 35S enhancer into the promoter or to remove repressor elements form regulatory regions. Further gene conversion methods can be used to disrupt repressor elements or to enhance to activity of positive elements. Positive elements can be randomly introduced in plants by T-DNA or transposon mutagenesis and lines can be identified in which the positive elements have be integrated near to a gene of the invention, the expression of which is thereby enhanced;
i) Modulating growth conditions of an organism in such a manner, that the expression or activity of the gene encoding the protein of the invention or the protein itself is enhanced for example microorganisms or plants can be grown under a higher temperature regime leading to an enhanced expression of heat shock proteins, e.g. the heat shock protein of the invention, which can lead an enhanced the fine chemical production; and/or
j) selecting of organisms with expecially high activity of the proteins of the invention from natural or from mutagenized resources and breeding them into the target organisms, eg the elite crops.

**[0054.0.15.15]** Preferably, said mRNA is the nucleic acid molecule of the present invention and/or the protein conferring the increased expression of a protein encoded by the nucleic acid molecule of the present invention or the polypeptide having the herein mentioned activity is the polypeptide of the present invention, e.g. conferring the increase of the respective fine chemical after increasing the expression or activity of the encoded polypeptide or having the activity of a polypeptide having an activity of a protein according to Table II, column 3, lines 174 to 177 and/or line 178 and/or line 179 and/or line180 and/or lines 181 to 185 or its homologs activity, e.g. as indicated in Table II, columns 5 or 7, lines 174 to 177 and/or line 178 and/or line 179 and/or line180 and/or lines 181 to 185 and/or line 558, 559 and/or line 560 to 563.

**[0055.0.0.15] to [0067.0.0.15] see [0055.0.0.0] to [0067.0.0.0]**

**[0068.0.15.15]** The mutation is introduced in such a way that the production of the fatty acids is not adversely affected.

**[0069.0.15.15]** Less influence on the regulation of a gene or its gene product is understood as meaning a reduced regulation of the enzymatic activity leading to an increased specific or cellular activity of the gene or its product. An increase of the enzymatic activity is understood as meaning an enzymatic activity, which is increased by at least 10%, advantageously at least 20, 30 or 40%, especially advantageously by at least 50, 60 or 70% in comparison with the starting organism. This leads to an increased productivity of the desired fatty acid(s).

**[0070.0.15.15]** Owing to the introduction of a gene or a plurality of genes conferring the expression of the nucleic acid molecule of the invention or the polypeptide of the invention, for example the nucleic acid construct mentioned below into an organism alone or in combination with other genes, it is possible not only to increase the biosynthetic flux towards the end product, but also to increase, modify or create *de novo* an advantageous, preferably novel metabolites composition in the organism, e.g. an advantageous fatty acid composition comprising a higher content of (from a viewpoint of nutritonal physiology limited) fatty acids, like heptadecanoic acid and/or 2-hydroxy palmitic acid and/or 2-hydroxy-tetracosenoic-acid and/or hexadecadienoic acid, preferably delta 7, 10 hexadecadienoic acid and/or hexadecatrienoic acid, preferably delta 7, 10 ,13 hexadecatrienoic acid.

**[0071.0.15.15]** Preferably the composition comprises further higher amounts of metabolites positively affecting or lower amounts of metabolites negatively affecting the nutrition or health of animals or humans provided with said compositions or organisms of the invention or parts thereof. Likewise, the number or activity of further genes which are required for the import or export of nutrients or metabolites, including amino acids, fatty acids, vitamins etc. or its precursors, required for the cell's biosynthesis of the fine chemical may be increased so that the concentration of necessary or relevant precursors, cofactors or intermediates within the cell(s) or within the corresponding storage compartments is increased. Owing to the increased or novel generated activity of the polypeptide of the invention or owing to the increased number of nucleic acid sequences of the invention and/or to the modulation of further genes which are involved in the biosynthesis of the fine chemical, e.g. by increasing the activity of enzymes synthesizing precursors or by destroying the activity of one or more genes which are involved in the breakdown of the fine chemical, it is possible to increase the yield, production and/or production efficiency of the fine chemical in the host organism, such as plants or the microorganims.

**[0072.0.15.15]** By influencing the metabolism thus, it is possible to produce, in the process according to the invention, further advantageous compounds. Examples of such compounds are, in addition to heptadecanoic acid and/or 2-hydroxy palmitic acid and/or 2-hydroxy-tetracosenoic-acid and/or hexadecadienoic acid, preferably delta 7, 10 hexadecadienoic acid and/or hexadecatrienoic acid, preferably delta 7, 10 ,13 hexadecatrienoic acid, triglycerides, lipids, preferably glycerophospholipids, sphingolipids and/or galactolipids, oils and/or fats containing heptadecanoic acid and/or 2-hydroxy palmitic acid and/or 2-hydroxy-tetracosenoic-acid and/or hexadecadienoic acid, preferably delta 7, 10 hexadecadienoic acid and/or hexadecatrienoic acid, preferably delta 7, 10 ,13 hexadecatrienoic acid compounds like other fatty acids such as palmitate, stearate, palmitoleate, oleate, linoleate and/or linoleate or erucic acid and/or, arachidonic acid.

**[0073.0.15.15]**Accordingly, in one embodiment, the process according to the invention relates to a process, which comprises:
(k) providing a non-human organism, preferably a microorganism, a non-human animal, a plant or animal cell, a plant or animal tissue or a plant;
(I) increasing an activity of a polypeptide of the invention or a homolog thereof, e.g. as indicated in Table II, columns 5 or 7, lines 174 to 177 and/or line 178 and/or line 179 and/or line180 and/or lines 181 to 185 and/or line 558, 559 and/or line 560 to 563 resp., or of a polypeptide being encoded by the nucleic acid molecule of the present invention and described below, i.e. conferring an increase of the respective fine chemical in the organism, preferably in a microorganism, a non-human animal, a plant or animal cell, a plant or animal tissue or a plant,
(m) growing the organism, preferably the microorganism, the non-human animal, the plant or animal cell, the plant or animal tissue or the plant under conditions which permit the production of the fine chemical in the organism, preferably the microorganism, the plant cell, the plant tissue or the plant; and
(n) if desired, recovering, optionally isolating, the free and/or bound the fine chemical and, optionally further free and/or bound fatty acids synthesized by the organism, the microorganism, the non-human animal, the plant or animal cell, the plant or animal tissue or the plant.

**[0074.0.15.15]** The organism, in particular the microorganism, non-human animal, the plant or animal cell, the plant or animal tissue or the plant is advantageously grown in such a way that it is not only possible to recover, if desired isolate the free or bound the fine chemical or the free and bound the fine chemical but as option it is also possible to produce, recover and, if desired isolate, other free or/and bound fatty acids.

**[0075.0.0.15] to [0077.0.0.15] see [0075.0.0.0] to [0077.0.0.0]**

**[0078.0.15.15]** The organism such as microorganisms or plants or the recovered, and if desired isolated, fine chemical can then be processed further directly into foodstuffs or animal feeds or for other applications, for example according to the disclosures made in EP-A-0 568 608, EP-A-568 606, WO 2004/007732, WO 02/057465, WO 01/02591, WO 2004/071467 or US 20020156254, which are expressly incorporated herein by reference. The fermentation broth, fermentation products, plants or plant products can be purified in the customary manner by hydrolysis with strong bases, extraction and crystallization or via thin layer chromatography and other methods known to the person skilled in the art and described herein below. Products of these different work-up procedures are fatty acids or fatty acid compositions which still comprise fermentation broth, plant particles and cell components in different amounts, advantageously in the range of from 0 to 99% by weight, preferably below 80% by weight, especially preferably between below 50% by weight.

**[0079.0.0.15] to [0084.0.0.15]: see [0079.0.0.0] to [0084.0.0.0]**

**[0085.0.15.15]**With regard to the nucleic acid sequence as depicted a nucleic acid construct which contains a nucleic acid sequence mentioned herein or an organism (= transgenic organism) which is transformed with said nucleic acid sequence or said nucleic acid construct, "transgene" means all those constructs which have been brought about by genetic manipulation methods,preferably in which either
a) the nucleic acid sequence as shown in table I, columns 5 or 7, lines 174 to 177 and/or line 178 and/or line 179 and/or line180 and/or lines 181 to 185 and/or line 558, 559 and/or line 560 to 563 or a derivative thereof, or
b) a genetic regulatory element, for example a promoter, which is functionally linked to the nucleic acid sequence as shown table I, columns 5 or 7, lines 174 to 177 and/or line 178 and/or line 179 and/or line180 and/or lines 181 to 185 and/or line 558, 559 and/or line 560 to 563 or a derivative thereof, or
c) (a) and (b)
is/are not present in its/their natural genetic environment or has/have been modified by means of genetic manipulation methods, it being possible for the modification to be, by way of example, a substitution, addition, deletion, inversion or insertion of one or more nucleotide. "Natural genetic environment" means the natural chromosomal locus in the organism of origin or the presence in a genomic library. In the case of a genomic library, the natural, genetic environment of the nucleic acid sequence is preferably at least partially still preserved. The environment flanks the nucleic acid sequence at least on one side and has a sequence length of at least 50 bp, preferably at least 500 bp, particularly preferably at least 1000 bp, very particularly preferably at least 5000 bp.

**[0086.0.0.15] to [0087.0.0.15] see [0086.0.0.0] to [0087.0.0.0]**

**[0088.0.15.15]** In an advantageous embodiment of the invention, the organism takes the form of a plant whose fatty acid content is modified advantageously owing to the nucleic acid molecule of the present invention expressed. This is important for plant breeders since, for example, the nutritional value of plants for animals is dependent on the abovementioned fatty acids and the general amount of fatty acids as energy source in feed. After the above mentioned protein activity has been increased or generated, or after the expression of nucleic acid molecule or polypeptide according to the invention has been generated or increased, the transgenic plant generated thus is grown on or in a nutrient medium or else in the soil and subsequently harvested.

**[0089.0.0.15] to [0095.0.0.15] see [0089.0.0.0] to [0095.0.0.0]**

**[0096.0.15.15]** In another preferred embodiment of the invention a combination of the increased expression of the nucleic acid sequence or the protein of the invention together with the transformation of a protein or polypeptide for example a fatty acid transporter protein or a compound, which functions as a sink for the desired fatty acids in the organism is useful to increase the production of the fine chemical (see Bao and Ohlrogge, Plant Physiol. 1999 August; 120 (4): 1057-1062). Such fatty acid transporter protein may serve as a link between the location of fatty acid synthesis and the so called sink tissue, in which fatty acids, triglycerides, oils and fats are stored.

**[0097.0.0.15] see [0097.0.0.0]** In may also be advantageous to increase the content of the bound fine chemical.

**[0098.0.15.15]** In a preferred embodiment, the fine chemical (heptadecanoic acid and/or 2-hydroxy palmitic acid and/or 2-hydroxy-tetracosenoic-acid and/or hexadecadienoic acid, preferably delta 7, 10 hexadecadienoic acid and/or hexadecatrienoic acid, preferably delta 7, 10 ,13 hexadecatrienoic acid) is produced in accordance with the invention and, if desired, is isolated. The production of further fatty acids such as palmitoleic acid, palmitic acid, stearic acid, oleic acid, linoleic acid, nervonic acid and/or linolenic acid mixtures thereof or mixtures of other fatty acids by the process according to the invention is advantageous.

**[0099.0.15.15]** In the case of the fermentation of microorganisms, the above mentioned fatty acids may accumulate in the medium and/or the cells. If microorganisms are used in the process according to the invention, the fermentation broth can be processed after the cultivation. Depending on the requirement, all or some of the biomass can be removed from the fermentation broth by separation methods such as, for example, centrifugation, filtration, decanting or a combination of these methods, or else the biomass can be left in the fermentation broth. The fermentation broth can subsequently be reduced, or concentrated, with the aid of known methods such as, for example, rotary evaporator, thin-layer evaporator, falling film evaporator, by reverse osmosis or by nanofiltration. Afterwards advantageously further compounds for formulation can be added such as corn starch or silicates. This concentrated fermentation broth advantageously together with compounds for the formulation can subsequently be processed by lyophilization, spray drying, spray granulation or by other methods. Preferably the fatty acids or the fatty acid compositions are isolated from the organisms, such as the microorganisms or plants or the culture medium in or on which the organisms have been grown, or from the organism and the culture medium, in the known manner, for example via extraction, distillation, crystallization, chromatography or a combination of these methods. These purification methods can be used alone or in combination with the aforementioned methods such as the separation and/or concentration methods.

**[0100.0.15.15]** Transgenic plants which comprise the fatty acids such as saturated or polyunsaturated fatty acids synthesized in the process according to the invention can advantageously be marketed directly without there being any need for the oils, lipids or fatty acids synthesized to be isolated. Plants for the process according to the invention are listed as meaning intact plants and all plant parts, plant organs or plant parts such as leaf, stem, seeds, root, tubers, anthers, fibers, root hairs, stalks, embryos, calli, cotelydons, petioles, harvested material, plant tissue, reproductive tissue and cell cultures which are derived from the actual transgenic plant and/or can be used for bringing about the transgenic plant. In this context, the seed comprises all parts of the seed such as the seed coats, epidermal cells, seed cells, endosperm or embryonic tissue. However, the fine chemical produced in the process according to the invention can also be isolated from the organisms, advantageously plants, in the form of their oils, fats, lipids and/or free fatty acids. Fatty acids produced by this process can be obtained by harvesting the organisms, either from the crop in which they grow, or from the field. This can be done via pressing or extraction of the plant parts, preferably the plant seeds. To increase the efficiency of oil extraction it is beneficial to clean, to temper and if necessary to hull and to flake the plant material expecially the seeds. In this context, the oils, fats, lipids and/or free fatty acids can be obtained by what is known as cold beating or cold pressing without applying heat. To allow for greater ease of disruption of the plant parts, specifically the seeds, they are previously comminuted, steamed or roasted. The seeds, which have been pretreated in this manner can subsequently be pressed or extracted with solvents such as warm hexane. The solvent is subsequently removed. In the case of microorganisms, the latter are, after harvesting, for example extracted directly without further processing steps or else, after disruption, extracted via various methods with which the skilled worker is familiar. In this manner, more than 96% of the compounds produced in the process can be isolated. Thereafter, the resulting products are processed further, i.e. degummed and/or refined. In this process, substances such as the plant mucilages and suspended matter are first removed. What is known as desliming can be affected enzymatically or, for example, chemico-physically by addition of acid such as phosphoric acid. Thereafter optionally, the free fatty acids are removed by treatment with a base like alkali, for example aqueous KOH or NaOH, or acid hydrolysis, advantageously in the presence of an alcohol such as methanol or ethanol, or via enzymatic cleavage, and isolated via, for example, phase separation and subsequent acidification via, for example, H₂SO₄. The fatty acids can also be liberated directly without the above-described processing step. If desired the resulting product can be washed thoroughly with water to remove traces of soap and the alkali remaining in the product and then dried. To remove the pigment remaining in the product, the products can be subjected to bleaching, for example using filler's earth or active charcoal. At the end, the product can be deodorized, for example using steam distillation under vacuum. These chemically pure fatty acids or fatty acid compositions are advantageous for applications in the food industry sector, the cosmetic sector and especially the pharmacological industry sector.

**[0101.0.15.15]** The identity and purity of the compound(s) isolated can be determined by prior-art techniques. They encompass high-performance liquid chromatography (HPLC), gas chromatography (GC), spectroscopic methods, mass spectrometry (MS), staining methods, thin-layer chromatography, NIRS, enzyme assays or microbiological assays. These analytical methods are compiled in: Patek et al. (1994) Appl. Environ. Microbiol. 60:133-140; Malakhova et al. (1996) Biotekhnologiya 11 27-32; and Schmidt et al. (1998) Bioprocess Engineer. 19:67-70. Ulmann's Encyclopedia of Industrial Chemistry (1996) Bd. A27, VCH Weinheim, pp. 89-90, pp. 521-540, pp. 540-547, pp. 559-566, 575-581 and pp. 581-587; Michal, G (1999) Biochemical Pathways: An Atlas of Biochemistry and Molecular Biology, John Wiley and Sons; Fallon, A. et al. (1987) Applications of HPLC in Biochemistry in: Laboratory Techniques in Biochemistry and Molecular Biology, vol. 17.

**[0102.0.15.15]** Fatty acids can for example be detected advantageously via GC separation methods. The unambiguous detection for the presence of fatty acid products can be obtained by analyzing recombinant organisms using analytical standard methods: GC, GC-MS or TLC, as described on several occasions by Christie and the references therein (1997, in: Advances on Lipid Methodology, Fourth Edition: Christie, Oily Press, Dundee, 119-169; 1998, Gaschromatographie-Massenspektrometrie-Verfahren [Gas chromatography/mass spectrometric methods], Lipide 33:343-353). One example is the analysis of fatty acids via FAME and GC-MS or TLC (abbreviations: FAME, fatty acid methyl ester; GC-MS, gas liquid chromatography/mass spectrometry; TLC, thin-layer chromatography. The material to be analyzed can be disrupted by sonication, grinding in a glass mill, liquid nitrogen and grinding or via other applicable methods. After disruption, the material must be centrifuged. The sediment is resuspended in distilled water, heated for 10 minutes at 100°C, cooled on ice and recentrifuged, followed by extraction for one hour at 90°C in 0.5 M sulfuric acid in methanol with 2% dimethoxypropane, which leads to hydrolyzed oil and lipid compounds, which give transmethylated lipids. These fatty acid methyl esters are extracted in petroleum ether and finally subjected to a GC analysis using a capillary column (Chrompack, WCOT Fused Silica, CP-Wax-52 CB, 25 µm, 0.32 mm) at a temperature gradient of between 170°C and 240°C for 20 minutes and 5 minutes at 240°C. The identity of the resulting fatty acid methyl esters must be defined using standards which are available from commercial sources (i.e. Sigma).

**[0102.1.15.15]** An other analytical method is described by Summit et al, (Proceedings of the Ocean Drilling Program, Scientific Results Volume 169, 2000). The fatty acid methyl esters are analyzed by capillary gas chromatography with flame ionization detection. Various mono- and polyunsaturated fatty acids can further be individually distinguished and quantified in one sample without prior separation by semi-selective HSQC (heteronuclear single quantum coherence)-NMR (Willker et al., Magn. Reson. Chem. 36, S79-S84 (1998)).
Fatty acid compositions, preferably of monogalactosyl diacylglycerol (MGDG) and digalactosyl diacylglycerol (DGDG) can be investigated using HPLC/ESI-MS combined with in-source (or cone voltage) fragmentation, negative-ion electrospray ionization (ESI) mass spectrometry interfaced with high performance liquid chromatography (HPLC) (Kim et al., Bull. Korean Chem. Soc. 2003, Vol. 24, No. 8).

**[0103.0.15.15]**In a preferred embodiment, the present invention relates to a process for the production of the fine chemical comprising or generating in an organism or a part thereof the expression of at least one nucleic acid molecule comprising a nucleic acid molecule selected from the group consisting of:
a) nucleic acid molecule encoding, preferably at least the mature form, of the polypeptide shown in Table II, columns 5 or 7, lines 174 to 177 and/or line 178 and/or line 179 and/or line180 and/or lines 181 to 185 and/or line 558, 559 and/or line 560 to 563 or a fragment thereof, which confers an increase in the amount of the fine chemical in an organism or a part thereof;
b) nucleic acid molecule comprising, preferably at least the mature form, of the nucleic acid molecule shown in Table IA or IB , columns 5 or 7, lines 174 to 177 and/or line 178 and/or line 179 and/or line180 and/or lines 181 to 185 and/or line 558, 559 and/or line 560 to 563;
c) nucleic acid molecule whose sequence can be deduced from a polypeptide sequence encoded by a nucleic acid molecule of (a) or (b) as result of the degeneracy of the genetic code and conferring an increase in the amount of the fine chemical in an organism or a part thereof;
d) nucleic acid molecule encoding a polypeptide which has at least 50% identity with the amino acid sequence of the polypeptide encoded by the nucleic acid molecule of (a) to (c) and conferring an increase in the amount of the fine chemical in an organism or a part thereof;
e) nucleic acid molecule which hybidizes with a nucleic acid molecule of (a) to (c) under under stringent hybridisation conditions and conferring an increase in the amount of the fine chemical in an organism or a part thereof;
f) nucleic acid molecule encoding a polypeptide, the polypeptide being derived by substituting, deleting and/or adding one or more amino acids of the amino acid sequence of the polypeptide encoded by the nucleic acid molecules (a) to (d), preferably to (a) to (c) and conferring an increase in the amount of the fine chemical in an organism or a part thereof;
g) nucleic acid molecule encoding a fragment or an epitope of a polypeptide which is encoded by one of the nucleic acid molecules of (a) to (e), preferably to (a) to (c) and and conferring an increase in the amount of the fine chemical in an organism or a part thereof;
h) nucleic acid molecule comprising a nucleic acid molecule which is obtained by amplifying nucleic acid molecules from a cDNA library or a genomic library using the primers shown in Table III, column 7, lines 174 to 177 and/or line 178 and/or line 179 and/or line180 and/or lines 181 to 185 and/or line 558, 559 and/or line 560 to 563 and conferring an increase in the amount of the fine chemical in an organism or a part thereof;
i) nucleic acid molecule encoding a polypeptide which is isolated, e.g. from an expression library, with the aid of monoclonal antibodies against a polypeptide encoded by one of the nucleic acid molecules of (a) to (h), preferably to (a) to (c), and and conferring an increase in the amount of the fine chemical in an organism or a part thereof;
j) nucleic acid molecule which encodes a polypeptide comprising the consensus sequence shown in Table IV, columns 7, lines 174 to 177 and/or line 178 and/or line 179 and/or line180 and/or lines 181 to 185 and/or line 558, 559 and/or line 560 to 563 and conferring an increase in the amount of the fine chemical in an organism or a part thereof;
k) nucleic acid molecule comprising one or more of the nucleic acid molecule encoding the amino acid sequence of a polypeptide encoding a domain of the polypeptide shown in Table II, columns 5 or 7, lines 174 to 177 and/or line 178 and/or line 179 and/or line180 and/or lines 181 to 185 and/or line 558, 559 and/or line 560 to 563 and conferring an increase in the amount of the fine chemical in an organism or a part thereof; and
l) nucleic acid molecule which is obtainable by screening a suitable library under stringent conditions with a probe comprising one of the sequences of the nucleic acid molecule of (a) to (k), preferably to (a) to (c), or with a fragment of at least 15 nt, preferably 20 nt, 30 nt, 50 nt, 100 nt, 200 nt or 500 nt of the nucleic acid molecule characterized in (a) to (k), preferably to (a) to (c), and conferring an increase in the amount of the fine chemical in an organism or a part thereof;
or which comprises a sequence which is complementary thereto.

**[0104.0.15.15]** In one embodiment, the nucleic acid molecule used in the process distinguishes over the sequence shown in Table IA or IB , columns 5 or 7, lines 174 to 177 and/or line 178 and/or line 179 and/or line180 and/or lines 181 to 185 and/or line 558, 559 and/or line 560 to 563 by one or more nucleotides or does not consist of the sequence shown in Table IA or IB , columns 5 or 7, lines 174 to 177 and/or line 178 and/or line 179 and/or line180 and/or lines 181 to 185 and/or line 558, 559 and/or line 560 to 563. In one embodiment, the nucleic acid molecule of the present invention is less than 100%, 99,999%, 99,99%, 99,9% or 99% identical to the sequence shown in Table IA or IB , columns 5 or 7, lines 174 to 177 and/or line 178 and/or line 179 and/or line180 and/or lines 181 to 185 and/or line 558, 559 and/or line 560 to 563. In another embodiment, the nucleic acid molecule does not encode a polypeptide of the sequence shown in Table II, columns 5 or 7, lines 174 to 177 and/or line 178 and/or line 179 and/or line180 and/or lines 181 to 185 and/or line 558, 559 and/or line 560 to 563.

**[0105.0.0.15] to [0107.0.0.15] see [0105.0.0.0] to [0107.0.0.0]**

**[0108.0.15.15]** Nucleic acid molecules with the sequence shown in Table IA or IB , columns 5 or 7, lines 174 to 177 and/or line 178 and/or line 179 and/or line 180 and/or lines 181 to 185 , nucleic acid molecules which are derived from the amino acid sequences shown in Table II, columns 5 or 7, lines 174 to 177 and/or line 178 and/or line 179 and/or line180 and/or lines 181 to 185 and/or line 558, 559 and/or line 560 to 563 or from polypeptides comprising the consensus sequence shown in Table IV, columns 5 or 7, lines 174 to 177 and/or line 178 and/or line 179 and/or line180 and/or lines 181 to 185 , and/or line 558, 559 and/or line 560 to 563 or their derivatives or homologues encoding polypeptides with the enzymatic or biological activity of a polypeptide as indicated in Table IA or IB , column 3, 5 or 7 , lines 174 to 177 and/or line 178 and/or line 179 and/or line180 and/or lines 181 to 185 and/or line 558, 559 and/or line 560 to 563 resp., or e.g. conferring a increase in the respective fine chemical after increasing its expression or activity are advantageously increased in the process according to the invention.

**[0109.0.15.15]** In one embodiment, said sequences are cloned into nucleic acid constructs, either individually or in combination. These nucleic acid constructs enable an optimal synthesis of the fatty acids produced in the process according to the invention.

**[0110.0.0.15] see [0110.0.0.0]**

**[0111.0.0.15] see [0111.0.0.0]**

**[0112.0.15.15]**The nucleic acid molecules used in the process according to the invention take the form of isolated nucleic acid sequences, which encode polypeptides with an activity of a polypeptide as indicated in Table IA or IB , column 3, lines 174 to 177 and/or line 178 and/or line 179 and/or line180 and/or lines 181 to 185 and/or line 558, 559 and/or line 560 to 563 resp., or having the sequence of a polypeptide as indicated in Table II, columns 5 and 7, lines 174 to 177 and/or line 178 and/or line 179 and/or line180 and/or lines 181 to 185 and/or line 558, 559 and/or line 560 to 563 resp., and conferring an increase of the respective fine chemical.

**[0113.0.0.15] to [0120.0.0.15] see [0113.0.0.0] to [0120.0.0.0]**

**[0121.0.15.15]**However, it is also possible to use artificial sequences, which differ in one or more bases from the nucleic acid sequences found in organisms, or in one or more amino acid molecules from polypeptide sequences found in organisms, in particular from the polypeptide sequences indicated in Table II, columns 5 or 7, lines 174 to 177 and/or line 178 and/or line 179 and/or line180 and/or lines 181 to 185 and/or line 558, 559 and/or line 560 to 563 resp., or the functional homologues thereof as described herein, preferably conferring above-mentioned activity, i.e.
conferring an increase in the level of heptadecanoic acid after increasing the activity of the polypeptide sequences indicated in Table II, columns 5 or 7, lines 174 to 177;
or conferring increase in the level of 2-hydroxy palmitic acid after increasing the activity of the polypeptide sequences indicated in Table II, columns 5 or 7, line 178 or 558, 559;
or conferring increase in the level of 2-hydroxy-tetracosenoic-acid after increasing the activity of the polypeptide sequences indicated in Table II, columns 5 or 7, line 179; or conferring increase in the level of hexadecadienoic acid, preferably delta 7, 10 hexadecadienoic acid after increasing the activity of the polypeptide sequences indicated in Table II, columns 5 or 7, line180;
or conferring increase in the level of hexadecatrienoic acid, preferably delta 7, 10 ,13 hexadecatrienoic acid after increasing the activity of the polypeptide sequences indicated in Table II, columns 5 or 7, lines 181 to 185.
or conferring increase in the level of C24:1 fatty acid, preferably C24:1 delta 15 fatty acid after increasing the activity of the polypeptide sequences indicated in Table II, columns 5 or 7, lines 560 to 563.

**[0122.0.0.15] to [0127.0.0.15] see [0122.0.0.0] to [0127.0.0.0]**

**[0128.0.15.15]** Synthetic oligonucleotide primers for the amplification, e.g. as the pairs indicated in Table III, columns 7, lines 174 to 177 and/or line 178 and/or line 179 and/or line180 and/or lines 181 to 185 and/or lines 558, 559 and/or lines 560 to 563 resp., by means of polymerase chain reaction can be generated on the basis of a sequence shown herein, for example the sequence as indicated in Table IA or IB , columns 5 or 7, lines 174 to 177 and/or line 178 and/or line 179 and/or line180 and/or lines 181 to 185 and/or lines 558, 559 and/or lines 560 to 563 resp., or the sequences derived from sequences as indicated in Table II, columns 5 or 7, lines 174 to 177 and/or line 178 and/or line 179 and/or line180 and/or lines 181 to 185 and/or lines 558, 559 and/or lines 560 to 563 resp..

**[0129.0.15.15]**Moreover, it is possible to identify conserved regions from various organisms by carrying out protein sequence alignments with the polypeptide used in the process of the invention, in particular with sequences of the polypeptide of the invention, from which conserved regions, and in turn, degenerate primers can be derived. Conserved region for the polypeptide of the invention are indicated in the alignments shown in the figures. Conserved regions are those, which show a very little variation in the amino acid in one particular position of several homologs from different origin. The consensus sequences indicated in Table IV, columns 7, lines 174 to 177 and/or and/or line 179 and/or line180 and/or lines 181 to 185 and/or lines 558, 559 and/or lines 560 to 563 resp., are derived from said alignments.

**[0130.0.15.15]**Degenerated primers can then be utilized by PCR for the amplification of fragments of novel proteins having above-mentioned activity, e.g. conferring the increase of fatty acids, e.g. of heptadecanoic acid and/or 2-hydroxy palmitic acid and/or 2-hydroxy-tetracosenoic-acid and/or hexadecadienoic acid, preferably delta 7, 10 hexadecadienoic acid and/or hexadecatrienoic acid, preferably delta 7, 10 ,13 hexadecatrienoic acid and/or 24:1 fatty acid, preferably C24:1 delta 15 fatty acid after increasing its expression or activity of the protein comprising said fragment.

**[0131.0.0.15] to [0138.0.0.15] see [0131.0.0.0] to [0138.0.0.0]**

**[0139.0.15.15]** Polypeptides having above-mentioned activity, i.e. conferring an increase of the respective fine chemical level, derived from other organisms, can be encoded by other DNA sequences which hybridize to a sequences indicated in Table IA or IB , columns 5 or 7, lines 174 to 177 and/or line 178 and/or line 179 and/or line180 and/or lines 181 to 185 and/or lines 558, 559 and/or lines 560 to 563 resp., preferably Table I B, columns 5 or 7, lines 174 to 177 and/or line 178 and/or line 179 and/or line180 and/or lines 181 to 185 and/or lines 558, 559 and/or lines 560 to 563 resp., under relaxed hybridization conditions and which code on expression for peptides having the heptadecanoic acid and/or 2-hydroxy palmitic acid and/or 2-hydroxy-tetracosenoic-acid and/or hexadecadienoic acid, preferably delta 7, 10 hexadecadienoic acid and/or hexadecatrienoic acid, preferably delta 7, 10 ,13 hexadecatrienoic acid and/or 24:1 fatty acid, preferably C24:1 delta 15 fatty acid resp., increasing activity.

**[0140.0.0.15] to [0146.0.0.15]: see [0140.0.0.0] to [0146.0.0.0]**

**[0147.0.15.15]** Further, the nucleic acid molecule of the invention comprises a nucleic acid molecule, which is a complement of one of the nucleotide sequences of above mentioned nucleic acid molecules or a portion thereof. A nucleic acid molecule which is complementary to one of the nucleotide sequences indicated in Table IA or IB , columns 5 or 7, lines 174 to 177 and/or line 178 and/or line 179 and/or line180 and/or lines 181 to 185 and/or lines 558, 559 and/or lines 560 to 563 resp., preferably Table B, columns 5 or 7, lines 174 to 177 and/or line 178 and/or line 179 and/or line180 and/or lines 181 to 185 and/or lines 558, 559 and/or lines 560 to 563 resp., is one which is sufficiently complementary to one of said nucleotide sequences s such that it can hybridize to one of said nucleotide sequences thereby forming a stable duplex. Preferably, the hybridisation is performed under stringent hybrization conditions. However, a complement of one of the herein disclosed sequences is preferably a sequence complement thereto according to the base pairing of nucleic acid molecules well known to the skilled person. For example, the bases A and G undergo base pairing with the bases T and U or C, resp. and visa versa. Modifications of the bases can influence the base-pairing partner.

**[0148.0.15.15]** The nucleic acid molecule of the invention comprises a nucleotide sequence which is at least about 30%, 35%, 40% or 45%, preferably at least about 50%, 55%, 60% or 65%, more preferably at least about 70%, 80%, or 90%, and even more preferably at least about 95%, 97%, 98%, 99% or more homologous to a nucleotide sequence shown in Table IA or IB , columns 5 or 7, lines 174 to 177 and/or line 178 and/or line 179 and/or line180 and/or lines 181 to 185 and/or lines 558, 559 and/or lines 560 to 563 resp., or a portion thereof and preferably has above mentioned activity, in particular having a heptadecanoic acid and/or 2-hydroxy palmitic acid and/or 2-hydroxy-tetracosenoic-acid and/or hexadecadienoic acid, preferably delta 7, 10 hexadecadienoic acid and/or hexadecatrienoic acid, preferably delta 7, 10 ,13 hexadecatrienoic acid and/or 24:1 fatty acid, preferably C24:1 delta 15 fatty acid - increasing activity after increasing its activity or an activity of a product of a gene encoding said sequence or its homologs.

**[0149.0.15.15]** The nucleic acid molecule of the invention comprises a nucleotide sequence which hybridizes, preferably hybridizes under stringent conditions as defined herein, to one of the nucleotide sequences indicated in Table IA or IB , columns 5 or 7, lines 174 to 177 and/or line 178 and/or line 179 and/or line180 and/or lines 181 to 185 and/or lines 558, 559 and/or lines 560 to 563 resp., preferably Table I B, columns 5 or 7, lines 174 to 177 and/or line 178 and/or line 179 and/or line180 and/or lines 181 to 185 and/or lines 558, 559 and/or lines 560 to 563 resp., or a portion thereof and encodes a protein having above-mentioned activity, e.g. conferring an increase of the respective fine chemical, e.g. of heptadecanoic acid and/or 2-hydroxy palmitic acid and/or 2-hydroxy-tetracosenoic-acid and/or hexadecadienoic acid, preferably delta 7, 10 hexadecadienoic acid and/or hexadecatrienoic acid, preferably delta 7, 10 ,13 hexadecatrienoic acid and and/or 24:1 fatty acid, preferably C24:1 delta 15 fatty acid optionally the activity of a protein indicated in Table IIa or IIB, column 5, lines 174 to 177 and/or line 178 and/or line 179 and/or line180 and/or lines 181 to 185 and/or lines 558, 559 and/or lines 560 to 563 resp.

**[00149.1.15.15]** Optionally, the nucleotide sequence, which hybridises to one of the nucleotide sequences indicated in Table IA or IB , columns 5 or 7, lines 174 to 177 and/or line 178 and/or line 179 and/or line180 and/or lines 181 to 185 and/or lines 558, 559 and/or lines 560 to 563 resp., preferably Table I B, columns 5 or 7, lines 174 to 177 and/or line 178 and/or line 179 and/or line180 and/or lines 181 to 185 and/or lines 558, 559 and/or lines 560 to 563 resp., has further one or more of the activities annotated or known for the a protein as indicated in Table IIA or IIB, column 3, lines 174 to 177 and/or line 178 and/or line 179 and/or line180 and/or lines 181 to 185 and/or lines 558, 559 and/or lines 560 to 563 resp..

**[0150.0.15.15]** Moreover, the nucleic acid molecule of the invention can comprise only a portion of the coding region of one of the sequences shown in Table IA or IB , columns 5 or 7, lines 174 to 177 and/or line 178 and/or line 179 and/or line180 and/or lines 181 to 185 and/or lines 558, 559 and/or lines 560 to 563 resp., preferably Table I B, columns 5 or 7, lines 174 to 177 and/or line 178 and/or line 179 and/or linel80 and/or lines 181 to 185 and/or lines 558, 559 and/or lines 560 to 563 resp., for example a fragment which can be used as a probe or primer or a fragment encoding a biologically active portion of the polypeptide of the present invention or of a polypeptide used in the process of the present invention, i.e. having above-mentioned activity, e.g.conferring an increase of heptadecanoic acid and/or 2-hydroxy palmitic acid and/or 2-hydroxy-tetracosenoic-acid and/or hexadecadienoic acid, preferably delta 7, 10 hexadecadienoic acid and/or hexadecatrienoic acid, preferably delta 7, 10 ,13 hexadecatrienoic acid and/or 24:1 fatty acid, preferably C24:1 delta 15 fatty acid if its activity is increased. The nucleotide sequences determined from the cloning of the present protein-according-to-the-invention-encoding gene allows for the generation of probes and primers designed for use in identifying and/or cloning its homologues in other cell types and organisms. The probe/primer typically comprises substantially purified oligonucleotide. The oligonucleotide typically comprises a region of nucleotide sequence that hybridizes under stringent conditions to at least about 12, 15 preferably about 20 or 25, more preferably about 40, 50 or 75 consecutive nucleotides of a sense strand of one of the sequences set forth, e.g., in Table IA or IB, columns 5 or 7, lines 174 to 177 and/or line 178 and/or line 179 and/or line180 and/or lines 181 to 185
and/or lines 558, 559 and/or lines 560 to 563 resp., preferably Table I B, columns 5 or 7, lines 174 to 177 and/or line 178 and/or line 179 and/or line180 and/or lines 181 to 185 and/or lines 558, 559 and/or lines 560 to 563 resp., an anti-sense sequence of one of the sequences, e.g., set forth in Table IA or IB , columns 5 or 7, lines 174 to 177 and/or line 178 and/or line 179 and/or line180 and/or lines 181 to 185 and/or lines 558, 559 and/or lines 560 to 563 resp., preferably Table I B, columns 5 or 7, lines 174 to 177 and/or line 178 and/or line 179 and/or line180 and/or lines 181 to 185 and/or lines 558, 559 and/or lines 560 to 563 resp., or naturally occurring mutants thereof. Primers based on a nucleotide of invention can be used in PCR reactions to clone homologues of the polypeptide of the invention or of the polypeptide used in the process of the invention, e.g. as the primers described in the examples of the present invention, e.g. as shown in the examples. A PCR with the primers shown in table III, column 7, lines 174 to 177 and/or line 178 and/or line 179 and/or line180 and/or lines 181 to 185 and/or lines 558, 559 and/or lines 560 to 563 resp., will result in a fragment of a polynucleotide sequence as indicated in Table IA or IB , columns 5 or 7, lines 174 to 177 and/or line 178 and/or line 179 and/or line180 and/or lines 181 to 185 and/or lines 558, 559 and/or lines 560 to 563 resp..

**[0151.0.0.15] see [0151.0.0.0]**

**[0152.0.15.15]** The nucleic acid molecule of the invention encodes a polypeptide or portion thereof which includes an amino acid sequence which is sufficiently homologous to an amino acid sequence as indicated in Table IIA or IB, columns 5 or 7, lines 174 to 177 and/or line 178 and/or line 179 and/or line180 and/or lines 181 to 185 and/or lines 558, 559 and/or lines 560 to 563 resp.,such that the protein or portion thereof maintains the ability to participate in the respective fine chemical production, in particular an activity increasing the level of amino acids, in particular of heptadecanoic acid and/or 2-hydroxy palmitic acid and/or 2-hydroxy-tetracosenoic-acid and/or hexadecadienoic acid, preferably delta 7, 10 hexadecadienoic acid and/or hexadecatrienoic acid, preferably delta 7, 10 ,13 hexadecatrienoic acid and/or 24:1 fatty acid, preferably C24:1 delta 15 fatty acid as mentioned above or as described in the examples in plants or microorganisms is comprised.

**[0153.0.15.15]** As used herein, the language "sufficiently homologous" refers to proteins or portions thereof which have amino acid sequences which include a minimum number of identical or equivalent amino acid residues (e.g., an amino acid residue which has a similar side chain as an amino acid residue in one of the sequences of the polypeptide of the present invention) to an amino acid sequence as indicated in Table IA or IB , columns 5 or 7,lines 174 to 177 and/or line 178 and/or line 179 and/or line180 and/or lines 181 to 185 and/or lines 558, 559 and/or lines 560 to 563 resp., such that the protein or portion thereof is able to participate in the increase of the respective fine chemical production. In one embodiment, a protein or portion thereof as indicated in Table IIA or IIB, columns 5 or 7, lines 174 to 177 and/or line 178 and/or line 179 and/or line180 and/or lines 181 to 185 and/or lines 558, 559 and/or lines 560 to 563 resp., has for example an activity of a polypeptide indicated in Table IIA or IIB, column 3, lines 174 to 177 and/or line 178 and/or line 179 and/or line180 and/or lines 181 to 185 and/or lines 558, 559 and/or lines 560 to 563 resp..

**[0154.0.15.15]** In one embodiment, the nucleic acid molecule of the present invention comprises a nucleic acid that encodes a portion of the protein of the present invention. The protein is at least about 30%, 35%, 40%, 45% or 50%, preferably at least about 55%, 60%, 65% or 70%, and more preferably at least about 75%, 80%, 85%, 90%, 91%, 92%, 93% or 94% and most preferably at least about 95%, 97%, 98%, 99% or more homologous to an entire amino acid sequence as indicated in Table IIA or IIB, columns 5 or 7, lines 174 to 177 and/or line 178 and/or line 179 and/or line180 and/or lines 181 to 185 and/or lines 558, 559 and/or lines 560 to 563 resp., and has above-mentioned activity, e.g. conferring preferably the increase of the respective fine chemical.

**[0155.0.0.15] to [0156.0.0.15]: see [0155.0.0.0] to [0156.0.0.0]**

**[0157.0.15.15]** The invention further relates to nucleic acid molecules that differ from one of a nucleotide sequences as indicated in Table IA or IB , columns 5 or 7, lines 174 to 177 and/or line 178 and/or line 179 and/or line180 and/or lines 181 to 185 and/or lines 558, 559 and/or lines 560 to 563 resp.,(and portions thereof) due to degeneracy of the genetic code and thus encode a polypeptide of the present invention, in particular a polypeptide having above mentioned activity, e.g. conferring an increase in the respective fine chemical in a organism, e.g. as that polypeptides encoded by the sequences as indicated in Table IV, columns 5 or 7, lines 174 to 177 and/or line 178 and/or line 179 and/or line180 and/or lines 181 to 185 and/or lines 558, 559 and/or lines 560 to 563 resp., or of the polypeptide as indicated in Table IIA or IIB, columns 5 or 7, lines 174 to 177 and/or line 178 and/or line 179 and/or line180 and/or lines 181 to 185 and/or lines 558, 559 and/or lines 560 to 563 resp.,or their functional homologues. Advantageously, the nucleic acid molecule of the invention comprises, or in an other embodiment has, a nucleotide sequence encoding a protein comprising, or in an other embodiment having, a consensus sequences as indicated in Table IV, columns 5 or 7, lines 174 to 177 and/or line 179 and/or line180 and/or lines 181 to 185 and/or lines 558, 559 and/or lines 560 to 563 resp.,or of the polypeptide as as indicated in Table IIA or IIB, columns 5 or 7, lines 174 to 177 and/or line 178 and/or line 179 and/or line180 and/or lines 181 to 185 and/or lines 558, 559 and/or lines 560 to 563 resp., or the functional homologues. In a still further embodiment, the nucleic acid molecule of the invention encodes a full length protein which is substantially homologous to an amino acid sequence comprising a consensus sequence as indicated in Table IV, columns 5 or 7, lines 174 to 177 and/or line 179 and/or line180 and/or lines 181 to 185 resp., or of a polypeptide as indicated in Table IIA or IIB, columns 5 or 7, lines 174 to 177 and/or line 178 and/or line 179 and/or line180 and/or lines 181 to 185 and/or lines 558, 559 and/or lines 560 to 563 resp., or the functional homologues thereof. However, in a preferred embodiment, the nucleic acid molecule of the present invention does not consist of a sequence as indicated in Table IA or IB , columns 5 or 7, lines 174 to 177 and/or line 178 and/or line 179 and/or line180 and/or lines 181 to 185 and/or lines 558, 559 and/or lines 560 to 563 resp., preferably of Table I A, columns 5 or 7, lines 181 to 185 and/or lines 558, 559 and/or lines 560 to 563. Preferably the nucleic acid molecule of the invention is a functional homologue or identical to a nucleic acid molecule indicated in Table I B, columns 5 or 7, lines 181 to 185 and/or lines 558, 559 and/or lines 560 to 563.

**[0158.0.0.15] to [0160.0.0.15]: see [0158.0.0.0] to [0160.0.0.0]**

**[0161.0.15.15]** Accordingly, in another embodiment, a nucleic acid molecule of the invention is at least 15, 20, 25 or 30 nucleotides in length. Preferably, it hybridizes under stringent conditions to a nucleic acid molecule comprising a nucleotide sequence of the nucleic acid molecule of the present invention or used in the process of the present invention, e.g. comprising a sequence as indicated in Table IA or IB , columns 5 or 7, lines 174 to 177 and/or line 178 and/or line 179 and/or line180 and/or lines 181 to 185 and/or lines 558, 559 and/or lines 560 to 563 resp.. The nucleic acid molecule is preferably at least 20, 30, 50, 100, 250 or more nucleotides in length.

**[0162.0.0.15] see [0162.0.0.0]**

**[0163.0.15.15]** Preferably, nucleic acid molecule of the invention that hybridizes under stringent conditions to a sequence as indicated in Table IA or IB , columns 5 or 7, lines 174 to 177 and/or line 178 and/or line 179 and/or line180 and/or lines 181 to 185 and/or lines 558, 559 and/or lines 560 to 563 resp., corresponds to a naturally-occurring nucleic acid molecule of the invention. As used herein, a "naturally-occurring" nucleic acid molecule refers to an RNA or DNA molecule having a nucleotide sequence that occurs in nature (e.g., encodes a natural protein). Preferably, the nucleic acid molecule encodes a natural protein having above-mentioned activity, e.g. conferring the respective fine chemical increase after increasing the expression or activity thereof or the activity of a protein of the invention or used in the process of the invention.

**[0164.0.0.15] see [0164.0.0.0]**

**[0165.0.15.15]** For example, nucleotide substitutions leading to amino acid substitutions at "non-essential" amino acid residues can be made in a sequence of the nucleic acid molecule of the invention or used in the process of the invention, e.g. as indicated in Table IA or IB , columns 5 or 7, lines 174 to 177 and/or line 178 and/or line 179 and/or line180 and/or lines 181 to 185 and/or lines 558, 559 and/or lines 560 to 563 resp., preferably Table I B columns, 5 or 7, lines 174 to 177 and/or line 178 and/or line 179 and/or line180 and/or lines 181 to 185 and/or lines 558, 559 and/or lines 560 to 563 resp..

**[0166.0.0.15] to [0167.0.0.15] see [0166.0.0.0] to [0167.0.0.0]**

**[0168.0.15.15]** Accordingly, the invention relates to nucleic acid molecules encoding a polypeptide having above-mentioned activity, e.g. conferring an increase in the respective fine chemical in an organisms or parts thereof that contain changes in amino acid residues that are not essential for said activity. Such polypeptides differ in amino acid sequence from a sequence contained in a sequence as indicated in Table IIA or IIB, columns 5 or 7, lines 174 to 177 and/or line 178 and/or line 179 and/or line180 and/or lines 181 to 185 and/or lines 558, 559 and/or lines 560 to 563 resp., preferably Table II B, columns 5 or 7, lines 174 to 177 and/or line 178 and/or line 179 and/or line180 and/or lines 181 to 185 and/or lines 558, 559 and/or lines 560 to 563 resp., yet retain said activity described herein. The nucleic acid molecule can comprise a nucleotide sequence encoding a polypeptide, wherein the polypeptide comprises
an amino acid sequence at least about 50% identical to an amino acid sequence as indicated in Table IIA or IIB, columns 5 or 7, lines 174 to 177 and/or line 178 and/or line 179 and/or line180 and/or lines 181 to 185 and/or lines 558, 559 and/or lines 560 to 563 resp., preferably Table II B, columns 5 or 7, lines 174 to 177 and/or line 178 and/or line 179 and/or line180 and/or lines 181 to 185 and/or lines 558, 559 and/or lines 560 to 563 resp., and is capable of participation in the increase of production of the respective fine chemical after increasing its activity, e.g. its expression. Preferably, the protein encoded by the nucleic acid molecule is at least about 60% identical to a sequence as indicated in Table IIA or IIB, columns 5 or 7, lines 174 to 177 and/or line 178 and/or line 179 and/or line180 and/or lines 181 to 185 and/or lines 558, 559 and/or lines 560 to 563 resp., preferably Table II B, columns 5 or 7, lines 174 to 177 and/or line 178 and/or line 179 and/or line180 and/or lines 181 to 185 and/or lines 558, 559 and/or lines 560 to 563 resp., more preferably at least about 70% identical to one of the sequences as indicated in Table IIA or IIB, columns 5 or 7, lines 174 to 177 and/or line 178 and/or line 179 and/or line180 and/or lines 181 to 185 and/or lines 558, 559 and/or lines 560 to 563 resp., preferably Table II B, columns 5 or 7, lines 174 to 177 and/or line 178 and/or line 179 and/or line180 and/or lines 181 to 185 and/or lines 558, 559 and/or lines 560 to 563 resp., even more preferably at least about 80%, 90%, or 95% homologous to a sequence as indicated in Table IIA or IIB, columns 5 or 7, lines 174 to 177 and/or line 178 and/or line 179 and/or line180 and/or lines 181 to 185 and/or lines 558, 559 and/or lines 560 to 563 resp., preferably Table II B, columns 5 or 7, lines 174 to 177 and/or line 178 and/or line 179 and/or line180 and/or lines 181 to 185 and/or lines 558, 559 and/or lines 560 to 563 resp.. and most preferably at least about 96%, 97%, 98%, or 99% identical to the sequence as indicated in Table IIA or IIB, columns 5 or 7, lines 174 to 177 and/or line 178 and/or line 179 and/or line180 and/or lines 181 to 185 and/or lines 558, 559 and/or lines 560 to 563 resp., preferably Table II B, columns 5 or 7, lines 174 to 177 and/or line 178 and/or line 179 and/or line180 and/or lines 181 to 185 and/or lines 558, 559 and/or lines 560 to 563 resp..

**[0169.0.0.15] to [0172.0.0.15] see [0169.0.0.0] to [0172.0.0.0]**

**[0173.0.15.15]** For example a sequence, which has 80% homology with sequence SEQ ID NO: 13930 at the nucleic acid level is understood as meaning a sequence which, upon comparison with the sequence SEQ ID NO: 13930 by the above Gap program algorithm with the above parameter set, has a 80% homology.

**[0174.0.0.15] see [0174.0.0.0]**

**[0175.0.15.15]** For example a sequence which has a 80% homology with sequence SEQ ID NO: 13931 at the protein level is understood as meaning a sequence which, upon comparison with the sequence SEQ ID NO: 13931 by the above program algorithm with the above parameter set, has a 80% homology.

**[0176.0.15.15]** Functional equivalents derived from one of the polypeptides as indicated in Table IIA or IIB, columns 5 or 7, lines 174 to 177 and/or line 178 and/or line 179 and/or line180 and/or lines 181 to 185 and/or lines 558, 559 and/or lines 560 to 563 resp., according to the invention by substitution, insertion or deletion have at least 30%, 35%, 40%, 45% or 50%, preferably at least 55%, 60%, 65% or 70% by preference at least 80%, especially preferably at least 85% or 90%, 91%, 92%, 93% or 94%, very especially preferably at least 95%, 97%, 98% or 99% homology with one of the polypeptides as indicated in Table IIA or IIB, columns 5 or 7, lines 174 to 177 and/or line 178 and/or line 179 and/or line180 and/or lines 181 to 185 and/or lines 558, 559 and/or lines 560 to 563 resp., according to the invention and are distinguished by essentially the same properties as a polypeptide as indicated in Table IIA or IIB, columns 5 or 7, lines 174 to 177 and/or line 178 and/or line 179 and/or line180 and/or lines 181 to 185 and/or lines 558, 559 and/or lines 560 to 563 resp..

**[0177.0.15.15]** Functional equivalents derived from a nucleic acid sequence as indicated in Table IA or IB , columns 5 or 7,lines 174 to 177 and/or line 178 and/or line 179 and/or line180 and/or lines 181 to 185 and/or lines 558, 559 and/or lines 560 to 563 resp., according to the invention by substitution, insertion or deletion have at least 30%, 35%, 40%, 45% or 50%, preferably at least 55%, 60%, 65% or 70% by preference at least 80%, especially preferably at least 85% or 90%, 91%, 92%, 93% or 94%, very especially preferably at least 95%, 97%, 98% or 99% homology with one of a polypeptides as indicated in Table IIA or IIB, columns 5 or 7, lines 174 to 177 and/or line 178 and/or line 179 and/or line180 and/or lines 181 to 185 and/or lines 558, 559 and/or lines 560 to 563 resp., according to the invention and encode polypeptides having essentially the same properties as a polypeptide as indicated in Table IIA or IIB, columns 5 or 7, lines 174 to 177 and/or line 178 and/or line 179 and/or line180 and/or lines 181 to 185 and/or lines 558, 559 and/or lines 560 to 563 resp..

**[0178.0.0.15] see [0178.0.0.0]**

**[0179.0.15.15]**A nucleic acid molecule encoding a homologous to a protein sequence of as indicated in Table IIA or IIB, columns 5 or 7, lines 174 to 177 and/or line 178 and/or line 179 and/or line180 and/or lines 181 to 185 and/or lines 558, 559 and/or lines 560 to 563 resp., preferably Table II B, columns 5 or 7, lines 174 to 177 and/or line 178 and/or line 179 and/or line180 and/or lines 181 to 185 and/or lines 558, 559 and/or lines 560 to 563 resp., can be created by introducing one or more nucleotide substitutions, additions or deletions into a nucleotide sequence of the nucleic acid molecule of the present invention, in particular as indicated in Table IA or IB , columns 5 or 7, llines 174 to 177 and/or line 178 and/or line 179 and/or line180 and/or lines 181 to 185 and/or lines 558, 559 and/or lines 560 to 563 resp., such that one or more amino acid substitutions, additions or deletions are introduced into the encoded protein. Mutations can be introduced into the encoding sequences of a sequences as indicated in Table IA or IB , columns 5 or 7, lines 174 to 177 and/or line 178 and/or line 179 and/or line180 and/or lines 181 to 185 and/or lines 558, 559 and/or lines 560 to 563 resp., by standard techniques, such as site-directed mutagenesis and PCR-mediated mutagenesis.

**[0180.0.0.15] to [0183.0.0.15] see [0180.0.0.0] to [0183.0.0.0]**

**[0184.0.15.15]** Homologues of the nucleic acid sequences used, with a sequence as indicated in Table IA or IB , columns 5 or 7, lines 174 to 177 and/or line 178 and/or line 179 and/or line180 and/or lines 181 to 185 and/or lines 558, 559 and/or lines 560 to 563 resp., preferably Table I B, columns 5 or 7, lines 174 to 177 and/or line 178 and/or line 179 and/or line180 and/or lines 181 to 185 and/or lines 558, 559 and/or lines 560 to 563 resp., or of the nucleic acid sequences derived from a sequences as indicated in Table IIA or IIB, columns 5 or 7, lines 174 to 177 and/or line 178 and/or line 179 and/or line180 and/or lines 181 to 185 and/or lines 558, 559 and/or lines 560 to 563 resp., comprise also allelic variants with at least approximately 30%, 35%, 40% or 45% homology, by preference at least approximately 50%, 60% or 70%, more preferably at least approximately 90%, 91 %, 92%, 93%, 94% or 95% and even more preferably at least approximately 96%, 97%, 98%, 99% or more homology with one of the nucleotide sequences shown or the abovementioned derived nucleic acid sequences or their homologues, derivatives or analogues or parts of these. Allelic variants encompass in particular functional variants which can be obtained by deletion, insertion or substitution of nucleotides from the sequences shown, preferably from a sequence as indicated in Table IA or IB , columns 5 or 7, lines 174 to 177 and/or line 178 and/or line 179 and/or line180 and/or lines 181 to 185 and/or lines 558, 559 and/or lines 560 to 563 resp., preferably Table I B, columns 5 or 7, lines 174 to 177 and/or line 178 and/or line 179 and/or line180 and/or lines 181 to 185 and/or lines 558, 559 and/or lines 560 to 563 resp.. or from the derived nucleic acid sequences, the intention being, however, that the enzyme activity or the biological activity of the resulting proteins synthesized is advantageously retained or increased.

**[0185.0.15.15]** In one embodiment of the present invention, the nucleic acid molecule of the invention or used in the process of the invention comprises one or more sequences as indicated in Table IA or IB , columns 5 or 7, lines 174 to 177 and/or line 178 and/or line 179 and/or line180 and/or lines 181 to 185 and/or lines 558, 559 and/or lines 560 to 563 resp., preferably Table I B, columns 5 or 7, lines 174 to 177 and/or line 178 and/or line 179 and/or line180 and/or lines 181 to 185 and/or lines 558, 559 and/or lines 560 to 563 resp.. In one embodiment, it is preferred that the nucleic acid molecule comprises as little as possible other nucleotide sequences not shown in any one of sequences as indicated in Table IA or IB , columns 5 or 7, lines 174 to 177 and/or line 178 and/or line 179 and/or line180 and/or lines 181 to 185 and/or lines 558, 559 and/or lines 560 to 563 resp., preferably Table I B, columns 5 or 7, lines 174 to 177 and/or line 178 and/or line 179 and/or line180 and/or lines 181 to 185 and/or lines 558, 559 and/or lines 560 to 563 resp.. In one embodiment, the nucleic acid molecule comprises less than 500, 400, 300, 200, 100, 90, 80, 70, 60, 50 or 40 further nucleotides. In a further embodiment, the nucleic acid molecule comprises less than 30, 20 or 10 further nucleotides. In one embodiment, a nucleic acid molecule used in the process of the invention is identical to a sequences as indicated in Table IA or IB , columns 5 or 7, lines 174 to 177 and/or line 178 and/or line 179 and/or line180 and/or lines 181 to 185 and/or lines 558, 559 and/or lines 560 to 563 resp., preferably Table I B, columns 5 or 7, lines 174 to 177 and/or line 178 and/or line 179 and/or line180 and/or lines 181 to 185 and/or lines 558, 559 and/or lines 560 to 563 resp..

**[0186.0.15.15]**Also preferred is that one or more nucleic acid molecule(s) used in the process of the invention encode a polypeptide comprising a sequence as indicated in Table IIA or IIB, columns 5 or 7, lines 174 to 177 and/or line 178 and/or line 179 and/or line180 and/or lines 181 to 185 and/or lines 558, 559 and/or lines 560 to 563 resp., preferably Table II B, columns 5 or 7, lines 174 to 177 and/or line 178 and/or line 179 and/or line180 and/or lines 181 to 185 and/or lines 558, 559 and/or lines 560 to 563 resp.. In one embodiment, the nucleic acid molecule encodes less than 150, 130, 100, 80, 60, 50, 40 or 30 further amino acids. In a further embodiment, the encoded polypeptide comprises less than 20, 15, 10, 9, 8, 7, 6 or 5 further amino acids. In one embodiment, the encoded polypeptide used in the process of the invention is identical to the sequences as indicated in Table IIA or IIB, columns 5 or 7, lines 174 to 177 and/or line 178 and/or line 179 and/or line180 and/or lines 181 to 185 and/or lines 558, 559 and/or lines 560 to 563 resp., preferably Table II B columns, 5 or 7, lines 174 to 177 and/or line 178 and/or line 179 and/or line180 and/or lines 181 to 185 and/or lines 558, 559 and/or lines 560 to 563 resp..

**[0187.0.15.15]**In one embodiment, the nucleic acid molecule of the invention or used in the process encodes a polypeptide comprising a sequence as indicated in Table IIA or IIB, columns 5 or 7, lines 174 to 177 and/or line 178 and/or line 179 and/or line180 and/or lines 181 to 185 and/or lines 558, 559 and/or lines 560 to 563 resp., preferably Table II B, columns 5 or 7, lines 174 to 177 and/or line 178 and/or line 179 and/or line180 and/or lines 181 to 185 and/or lines 558, 559 and/or lines 560 to 563 resp., comprises less than 100 further nucleotides. In a further embodiment, said nucleic acid molecule comprises less than 30 further nucleotides. In one embodiment, the nucleic acid molecule used in the process is identical to a coding sequence encoding a sequences as indicated in Table IIA or IIB, columns 5 or 7, lines 174 to 177 and/or line 178 and/or line 179 and/or line180 and/or lines 181 to 185 and/or lines 558, 559 and/or lines 560 to 563 resp., preferably Table II B, columns 5 or 7, lines 174 to 177 and/or line 178 and/or line 179 and/or line180 and/or lines 181 to 185 and/or lines 558, 559 and/or lines 560 to 563 resp..

**[0188.0.15.15]**Polypeptides (= proteins), which still have the essential biological or enzymatic activity of the polypeptide of the present invention conferring an increase of the respective fine chemical i.e. whose activity is essentially not reduced, are polypeptides with at least 10% or 20%, by preference 30% or 40%, especially preferably 50% or 60%, very especially preferably 80% or 90 or more of the wild type biological activity or enzyme activity, advantageously, the activity is essentially not reduced in comparison with the activity of a polypeptide as indicated in Table IIA or IIB, columns 5 or 7, lines 174 to 177 and/or line 178 and/or line 179 and/or line180 and/or lines 181 to 185 and/or lines 558, 559 and/or lines 560 to 563 resp., preferably compared to a sequence as indicated in Table IIA or IIB, column 3 and 5, lines 174 to 177 and/or line 178 and/or line 179 and/or line180 and/or lines 181 to 185 and/or lines 558, 559 and/or lines 560 to 563 resp., and expressed under identical conditions.
In one embodiment, the polypeptide of the invention is a homolog consisting of or comprising the sequence as indicated in Table II B, column 7, lines 174 to 177 and/or line 178 and/or line 179 and/or line180 and/or lines 181 to 185 and/or lines 558, 559 and/or lines 560 to 563 resp.

**[0189.0.15.15]**Homologues of a sequences as indicated in Table IA or IB , columns 5 or 7, lines 174 to 177 and/or line 178 and/or line 179 and/or line180 and/or lines 181 to 185 and/or lines 558, 559 and/or lines 560 to 563 resp., or of a derived sequences as indicated in Table IIA or IIB, columns 5 or 7, lines 174 to 177 and/or line 178 and/or line 179 and/or line180 and/or lines 181 to 185 and/or lines 558, 559 and/or lines 560 to 563 resp., also mean truncated sequences, cDNA, single-stranded DNA or RNA of the coding and noncoding DNA sequence. Homologues of said sequences are also understood as meaning derivatives which comprise noncoding regions such as, for example, UTRs, terminators, enhancers or promoter variants. The promoters upstream of the nucleotide sequences stated can be modified by one or more nucleotide substitution(s), insertion(s) and/or deletion(s) without, however, interfering with the functionality or activity either of the promoters, the open reading frame (= ORF) or with the 3'-regulatory region such as terminators or other 3'regulatory regions, which are far away from the ORF. It is furthermore possible that the activity of the promoters is increased by modification of their sequence, or that they are replaced completely by more active promoters, even promoters from heterologous organisms. Appropriate promoters are known to the person skilled in the art and are mentioned herein below.

**[0190.0.0.15] to [0203.0.0.15] see [0192.0.0.0] to [0203.0.0.0]**

**[0204.0.15.15]** Accordingly, in one embodiment, the invention relates to a nucleic acid molecule, which comprises a nucleic acid molecule selected from the group consisting of:
a) nucleic acid molecule encoding, preferably at least the mature form, of a polypeptide as indicated in Table IIA or IIB, columns 5 or 7, lines 174 to 177 and/or line 178 and/or line 179 and/or line180 and/or lines 181 to 185 and/or lines 558, 559 and/or lines 560 to 563 resp.; or a fragment thereof conferring an increase in the amount of the respective fine chemical, i.e. heptadecanoic acid and/or 2-hydroxy palmitic acid and/or 2-hydroxy-tetracosenoic-acid and/or hexadecadienoic acid, preferably delta 7, 10 hexadecadienoic acid and/or hexadecatrienoic acid, preferably delta 7, 10 ,13 hexadecatrienoic acid and/or 24:1 fatty acid, preferably C24:1 delta 15 fatty acid in an organism or a part thereof;
b) nucleic acid molecule comprising, preferably at least the mature form, of a nucleic acid molecule as indicated in Table IA or IB , columns 5 or 7, lines 174 to 177 and/or line 178 and/or line 179 and/or line180 and/or lines 181 to 185 and/or lines 558, 559 and/or lines 560 to 563 resp., or a fragment thereof conferring an increase in the amount of the respective fine chemical in an organism or a part thereof;
c) nucleic acid molecule whose sequence can be deduced from a polypeptide sequence encoded by a nucleic acid molecule of (a) or (b) as result of the degeneracy of the genetic code and conferring an increase in the amount of the fine chemical in an organism or a part thereof;
d) nucleic acid molecule encoding a polypeptide whose sequence has at least 50% identity with the amino acid sequence of the polypeptide encoded by the nucleic acid molecule of (a) to (c) and conferring an increase in the amount of the fine chemical in an organism or a part thereof;
e) nucleic acid molecule which hybridizes with a nucleic acid molecule of (a) to (c) under under stringent hybridisation conditions and conferring an increase in the amount of the fine chemical in an organism or a part thereof;
f) nucleic acid molecule encoding a polypeptide, the polypeptide being derived by substituting, deleting and/or adding one or more amino acids of the amino acid sequence of the polypeptide encoded by the nucleic acid molecules (a) to (d), preferably to (a) to (c), and conferring an increase in the amount of the fine chemical in an organism or a part thereof;
g) nucleic acid molecule encoding a fragment or an epitope of a polypeptide which is encoded by one of the nucleic acid molecules of (a) to (e), preferably to (a) to (c) and conferring an increase in the amount of the fine chemical in an organism or a part thereof;
h) nucleic acid molecule comprising a nucleic acid molecule which is obtained by amplifying a cDNA library or a genomic library using primers or primer pairs as indicated in Table III, column 7, lines 174 to 177 and/or line 178 and/or line 179 and/or line180 and/or lines 181 to 185 and/or lines 558, 559 and/or lines 560 to 563 resp., and conferring an increase in the amount of the respective fine chemical, i.e. heptadecanoic acid and/or 2-hydroxy palmitic acid and/or 2-hydroxy-tetracosenoic-acid and/or hexadecadienoic acid, preferably delta 7, 10 hexadecadienoic acid and/or hexadecatrienoic acid, preferably delta 7, 10 ,13 hexadecatrienoic acid and/or 24:1 fatty acid, preferably C24:1 delta 15 fatty acid in an organism or a part thereof;
i) nucleic acid molecule encoding a polypeptide which is isolated, e.g. from a expression library, with the aid of monoclonal antibodies against a polypeptide encoded by one of the nucleic acid molecules of (a) to (g), preferably to (a) to (c) and conferring an increase in the amount of the fine chemical in an organism or a part thereof;
j) nucleic acid molecule which encodes a polypeptide comprising a consensus sequence as indicated in Table IV, column 7, lines 174 to 177 and/or line 178 and/or line 179 and/or line180 and/or lines 181 to 185 and/or lines 558, 559 and/or lines 560 to 563 resp., and conferring an increase in the amount of the respective fine chemical i.e. heptadecanoic acid and/or 2-hydroxy palmitic acid and/or 2-hydroxy-tetracosenoic-acid and/or hexadecadienoic acid, preferably delta 7, 10 hexadecadienoic acid and/or hexadecatrienoic acid, preferably delta 7, 10 ,13 hexadecatrienoic acid and/or 24:1 fatty acid, preferably C24:1 delta 15 fatty acid in an organism or a part thereof;
k) nucleic acid molecule encoding the amino acid sequence of a polypeptide encoding a domaine of a polypeptide as indicated in Table IIA or IIB, columns 5 or 7, lines 174 to 177 and/or line 178 and/or line 179 and/or line180 and/or lines 181 to 185 and/or lines 558, 559 and/or lines 560 to 563 resp., and conferring an increase in the amount of the respective fine chemical i.e. heptadecanoic acid and/or 2-hydroxy palmitic acid and/or 2-hydroxy-tetracosenoic-acid and/or hexadecadienoic acid, preferably delta 7, 10 hexadecadienoic acid and/or hexadecatrienoic acid, preferably delta 7, 10 ,13 hexadecatrienoic acid and/or 24:1 fatty acid, preferably C24:1 delta 15 fatty acid in an organism or a part thereof; and
l) nucleic acid molecule which is obtainable by screening a suitable nucleic acid library under stringent hybridization conditions with a probe comprising one of the sequences of the nucleic acid molecule of (a) to (k) or with a fragment of at least 15 nt, preferably 20 nt, 30 nt, 50 nt, 100 nt, 200 nt or 500 nt of the nucleic acid molecule characterized in (a) to (h) or of a nucleic acid molecule as indicated in Table IA or IB , columns 5 or 7, lines 174 to 177 and/or line 178 and/or line 179 and/or line180 and/or lines 181 to 185 and/or lines 558, 559 and/or lines 560 to 563 resp., or a nucleic acid molecule encoding, preferably at least the mature form of, the polypeptide as indicated in Table IIA or IIB, columns 5 or 7, lines 174 to 177 and/or line 178 and/or line 179 and/or line180 and/or lines 181 to 185 and/or lines 558, 559 and/or lines 560 to 563 resp., and conferring an increase in the amount of the respective fine chemical in an organism or a part thereof;
whereby, preferably, the nucleic acid molecule according to (a) to (I) distinguishes over a sequence depicted in as indicated in Table I A, columns 5 or 7, lines 174 to 177 and/or line 178 and/or line 179 and/or line180 and/or lines 181 to 185 and/or lines 558, 559 and/or lines 560 to 563 resp., by one or more nucleotides. In one embodiment, the nucleic acid molecule of the invention does not consist of a sequence as indicated in Table I A or IB, columns 5 or 7, lines 174 to 177 and/or line 178 and/or line 179 and/or line180 and/or lines 181 to 185 and/or lines 558, 559 and/or lines 560 to 563 resp. In an other embodiment, the nucleic acid molecule of the present invention is at least 30 % identical and less than 100%, 99,999%, 99,99%, 99,9% or 99% identical to a sequence indicated in Table I A or IB, columns 5 or 7, lines 174 to 177 and/or line 178 and/or line 179 and/or line180 and/or lines 181 to 185 and/or lines 558, 559 and/or lines 560 to 563 resp.. In a further embodiment the nucleic acid molecule does not encode a polypeptide sequence as indicated in Table II A or II B, columns 5 or 7, lines 174 to 177 and/or line 178 and/or line 179 and/or line180 and/or lines 181 to 185 and/or lines 558, 559 and/or lines 560 to 563 resp. Accordingly, in one embodiment, the nucleic acid molecule of the present invention encodes in one embodiment a polypeptide which differs at least in one or more amino acids from a polypeptide indicated in Table II A or II B, columns 5 or 7, lines 174 to 177 and/or line 178 and/or line 179 and/or line180 and/or lines 181 to 185 and/or lines 558, 559 and/or lines 560 to 563 resp. In another embodiment, a nucleic acid molecule indicated in Table I A or I B, columns 5 or 7, lines 174 to 177 and/or line 178 and/or line 179 and/or line180 and/or lines 181 to 185 and/or lines 558, 559 and/or lines 560 to 563 resp., does not encode a protein of a sequence indicated in Table II A or II B, columns 5 or 7, lines 174 to 177 and/or line 178 and/or line 179 and/or line180 and/or lines 181 to 185 and/or lines 558, 559 and/or lines 560 to 563 resp. Accordingly, in one embodiment, the protein encoded by a sequences of a nucleic acid according to (a) to (I) does not consist of a sequence as indicated in Table II A or II B, columns 5 or 7, lines 174 to 177 and/or line 178 and/or line 179 and/or line180 and/or lines 181 to 185 and/or lines 558, 559 and/or lines 560 to 563 resp. In a further embodiment, the protein of the present invention is at least 30 % identical to a protein sequence indicated in Table II A or II B, columns 5 or 7, lines 174 to 177 and/or line 178 and/or line 179 and/or line180 and/or lines 181 to 185 and/or lines 558, 559 and/or lines 560 to 563 resp., and less than 100%, preferably less than 99,999%, 99,99% or 99,9%, more preferably less than 99%, 985, 97%, 96% or 95% identical to a sequence as indicated in Table I A or I B, columns 5 or 7,lines 174 to 177 and/or line 178 and/or line 179 and/or line180 and/or lines 181 to 185 and/or lines 558, 559 and/or lines 560 to 563 resp..

**[0205.0.0.15] to [0206.0.0.15]: see [0205.0.0.0] to [0206.0.0.0]**

**[0207.0.15.15]** As described herein, the nucleic acid construct can also comprise further genes, which are to be introduced into the organisms or cells. It is possible and advantageous to introduce into, and express in, the host organisms regulatory genes such as genes for inductors, repressors or enzymes, which, owing to their enzymatic activity, engage in the regulation of one or more genes of a biosynthetic pathway. These genes can be of heterologous or homologous origin. Moreover, further biosynthesis genes may advantageously be present, or else these genes may be located on one or more further nucleic acid constructs. Genes, which are advantageously employed as biosynthesis genes are genes of the fatty acid metabolism, amino acid metabolism, of glycolysis, of the tricarboxylic acid metabolism, of triacylglycerol or lipid, preferably glycerophospholipids, sphingolipids and/or galactolipids biosynthesis or their combinations. As described herein, regulator sequences or factors can have a positive effect on preferably the gene expression of the genes introduced, thus increasing it. Thus, an enhancement of the regulator elements may advantageously take place at the transcriptional level by using strong transcription signals such as promoters and/or enhancers. In addition, however, an enhancement of translation is also possible, for example by increasing mRNA stability or by inserting a translation enhancer sequence.

**[0208.0.0.15] to [0226.0.0.15] see [0208.0.0.0] to [0226.0.0.0]**

**[0227.0.15.15]** The abovementioned nucleic acid molecules can be cloned into the nucleic acid constructs or vectors according to the invention in combination together with further genes, or else different genes are introduced by transforming several nucleic acid constructs or vectors (including plasmids) into a host cell, advantageously into a plant cell or a microorgansims.
In addition to the sequence mentioned in Table IA or IB, columns 5 or 7, lines 174 to 177 and/or line 178 and/or line 179 and/or line180 and/or lines 181 to 185 and/or lines 558, 559 and/or lines 560 to 563 resp., or its derivatives, it is advantageous additionally to express and/or mutate further genes in the organisms. Especially advantageously, additionally at least one further gene of the fatty acid biosynthetic pathway such as for palmitate, stearate, palmitoleate, oleate, linoleate and/or linolenate or of the of triacylglycerol or lipid, preferably glycerophospholipids, sphingolipids and/or galactolipids biosynthetic pathway is expressed in the organisms such as plants or microorganisms. It is also possible that the regulation of the natural genes has been modified advantageously so that the gene and/or its gene product is no longer subject to the regulatory mechanisms which exist in the organisms. This leads to an increased synthesis of the amino acids desired since, for example, feedback regulations no longer exist to the same extent or not at all. In addition it might be advantageously to combine the sequences shown in Table IA or IB, columns 5 or 7, lines 174 to 177 and/or line 178 and/or line 179 and/or line180 and/or lines 181 to 185 and/or lines 558, 559 and/or lines 560 to 563 resp., with genes which generally support or enhances to growth or yield of the target organism, for example genes which lead to faster growth rate of microorganisms or genes which produces stress-, pathogen, or herbicide resistant plants.

**[0228.0.15.15]** In a further embodiment of the process of the invention, therefore, organisms are grown, in which there is simultaneous overexpression of at least one nucleic acid or one of the genes which code for proteins involved in the fatty acid metabolism, in particular in fatty acid synthesis.

**[0229.0.15.15]**Further advantageous nucleic acid sequences which can be expressed in combination with the sequences used in the process and/or the abovementioned biosynthesis genes are the sequences encoding further genes of the saturated, poly unsaturated fatty acid biosynthesis such as desaturases like Δ-4-desaturases, Δ-5-desaturases, Δ-6-desaturases, Δ-8-desaturases, Δ-9-desaturases, Δ-12-desaturases, Δ-17-desaturases, ω-3-desaturases, elongases like Δ-5-elongases, Δ-6-elongases, Δ-9-elongases, acyl-CoA-dehydrogenases, acyl-ACP-desaturases, acyl-ACP-thioesterases, fatty acid acyl-transferases, acyl-CoA lysophospholipid-acyltransferases, acyl-CoA carboxylases, fatty acid synthases, fatty acid hydroxylases, acyl-CoA oxydases, acetylenases, lipoxygenases, triacyl-lipases etc. as described in WO 98/46765, WO 98/46763, WO 98/46764, WO 99/64616, WO 00/20603, WO 00/20602, WO 00/40705, US 20040172682, US 20020156254, US 6,677,145 US 20040053379 or US 20030101486. These genes lead to an increased synthesis of the essential fatty acids.

**[0229.1.15.15]** Further nucleic acid sequences which can be expressed in combination with the sequences used in the process and/or the abovementioned biosynthesis genes are the sequences encoding further genes of the saturated, unsaturated, polyunsaturated and/or hydroxylated fatty acid biosynthesis such as the FA2H gene encoding a fatty acid 2-hydroxylase (Alderson et al., J. Biol. Chem. Vol. 279, No. 47, Issue of November 19, pp. 48562-48568, 2004), ω-3-desaturases encoded by FAD2 and FAD3 (US 20030221217), delta-12 fatty acid desaturase, delta-15 fatty acid desaturase as disclosed by Okuley, et al., Plant Cell 6:147-158 (1994), Lightner et al., WO94/11516, Yadav, N., et al., Plant Physiol., 103:467-476 (1993), WO 93/11245 and Arondel, V. et al., Science, 258:1353-1355 (1992), US 20040083503.

**[0230.0.0.15] see [0230.0.0.0]**

**[0231.0.15.15]**In a further advantageous embodiment of the process of the invention, the organisms used in the process are those in which simultaneously a heptadecanoic acid and/or 2-hydroxy palmitic acid and/or 2-hydroxy-tetracosenoic-acid and/or hexadecadienoic acid, preferably delta 7, 10 hexadecadienoic acid and/or hexadecatrienoic acid, preferably delta 7, 10 ,13 hexadecatrienoic acid and/or 24:1 fatty acid, preferably C24:1 delta 15 fatty acid degrading protein is attenuated, in particular by reducing the rate of expression of the corresponding gene.

**[0232.0.0.15] to [0276.0.0.15] see [0232.0.0.0] to [0276.0.0.0]**

**[0277.0.15.15]** The fatty acids produced can be isolated from the organism by methods with which the skilled worker is familiar. For example via extraction, salt precipitation and/or different chromatography methods. The process according to the invention can be conducted batchwise, semibatchwise or continuously. The fine chemical produced in the process according to the invention can be isolated as mentioned above from the organisms, advantageously plants, in the form of their oils, fats, lipids and/or free fatty acids. Fatty acids produced by this process can be obtained by harvesting the organisms, either from the crop in which they grow, or from the field. This can be done via pressing or extraction of the plant parts, preferably the plant seeds. Hexane is preferably used as solvent in the process, in which more than 96% of the compounds produced in the process can be isolated. Thereafter, the resulting products are processed further, i.e. degummed, refined, bleached and/or deodorized.

**[0278.0.0.15] to [0282.0.0.15]see [0278.0.0.0] to [0282.0.0.0]**

**[0283.0.15.15]** Moreover, a native polypeptide conferring the increase of the fine chemical in an organism or part thereof can be isolated from cells (e.g., endothelial cells), for example using the antibody of the present invention as described below, in particular, an antibody against a protein as indicated in Table IIA or IIB, column 3, lines 174 to 177 and/or line 178 and/or line 179 and/or line180 and/or lines 181 to 185 and/or lines 558, 559 and/or lines 560 to 563 resp.. e.g. an antibody against a polypeptide as indicated in Table IIA or IIB, columns 5 or 7, lines 174 to 177 and/or line 178 and/or line 179 and/or line180 and/or lines 181 to 185 and/or lines 558, 559 and/or lines 560 to 563 resp., which can be produced by standard techniques utilizing polypeptides comprising or consisting of above mentioned sequences, e.g. the polypeptide of the present invention or fragment thereof. Preferred are monoclonal antibodies.

**[0284.0.0.15] see [0284.0.0.0]**

**[0285.0.15.15]**In one embodiment, the present invention relates to a polypeptide having a sequence as indicated in Table IIA or IIB, columns 5 or 7, lines 174 to 177 and/or line 178 and/or line 179 and/or line180 and/or lines 181 to 185 and/or lines 558, 559 and/or lines 560 to 563 resp., or as coded by a nucleic acid molecule as indicated in Table IA or IB, columns 5 or 7, lines 174 to 177 and/or line 178 and/or line 179 and/or line180 and/or lines 181 to 185 and/or lines 558, 559 and/or lines 560 to 563 resp., or functional homologues thereof.

**[0286.0.15.15]**In one advantageous embodiment, in the method of the present invention the activity of a polypeptide is increased which comprises or consists of a consensus sequence as indicated in Table IV, column 7, lines 174 to 177 and/or line 178 and/or line 179 and/or line180 and/or lines 181 to 185 and/or lines 558, 559 and/or lines 560 to 563 resp., and in one another embodiment, the present invention relates to a polypeptide comprising or consisting of a consensus sequence as indicated in Table IV, column 7, lines 174 to 177 and/or line 178 and/or line 179 and/or line180 and/or lines 181 to 185 and/or lines 558, 559 and/or lines 560 to 563 resp., whereby 20 or less, preferably 15 or 10, preferably 9, 8, 7, or 6, more preferred 5 or 4, even more preferred 3, even more preferred 2, even more preferred 1, most preferred 0 of the amino acids positions indicated can be replaced by any amino acid, or, in an further embodiment, can be replaced and/or absent. In one embodiment, the present invention relates to the method of the present invention comprising a polypeptide or to a polypeptide comprising more than one consensus sequences (of an individual line) as indicated in Table IV, column 7, lines 174 to 177 and/or line 178 and/or line 179 and/or line180 and/or lines 181 to 185 and/or lines 558, 559 and/or lines 560 to 563 resp.

**[0287.0.0.15] to [0290.0.0.15] see [0287.0.0.0] to [0290.0.0.0]**

**[0291.0.15.15]** In one advantageous embodiment, the method of the present invention comprises the increasing of a polypeptide comprising or consisting of plant or microorganism specific consensus sequences.
In one embodiment, said polypeptide of the invention distinguishes over a sequence as indicated in Table II A or II B, columns 5 or 7, lines 174 to 177 and/or line 178 and/or line 179 and/or line180 and/or lines 181 to 185 and/or lines 558, 559 and/or lines 560 to 563 resp., by one or more amino acids. In one embodiment, polypeptide distinguishes form a sequence as indicated in Table II A or II B, columns 5 or 7, lines 174 to 177 and/or line 178 and/or line 179 and/or line180 and/or lines 181 to 185 and/or lines 558, 559 and/or lines 560 to 563 resp., by more than 5, 6, 7, 8 or 9 amino acids, preferably by more than 10, 15, 20, 25 or 30 amino acids, even more preferred are more than 40, 50, or 60 amino acids and, preferably, the sequence of the polypeptide of the invention distinguishes from a sequence as indicated in Table II A or II B, columns 5 or 7, lines 174 to 177 and/or line 178 and/or line 179 and/or line180 and/or lines 181 to 185 and/or lines 558, 559 and/or lines 560 to 563 resp., by not more than 80% or 70% of the amino acids, preferably not more than 60% or 50%, more preferred not more than 40% or 30%, even more preferred not more than 20% or 10%. In an other embodiment, said polypeptide of the invention does not consist of a sequence as indicated in Table II A or II B, columns 5 or 7, lines 174 to 177 and/or line 178 and/or line 179 and/or line180 and/or lines 181 to 185 and/or lines 558, 559 and/or lines 560 to 563 resp.

**[0292.0.0.15] see [0292.0.0.0]**

**[0293.0.15.15]** In one embodiment, the invention relates to polypeptide conferring an increase in the respective fine chemical in an organism or part being encoded by the nucleic acid molecule of the invention or by a nucleic acid molecule used in the process of the invention. In one embodiment, the polypeptide of the invention has a sequence which distinguishes from a sequence as indicated in Table II A or II B, lines 174 to 177 and/or line 178 and/or line 179 and/or line180 and/or lines 181 to 185 and/or lines 558, 559 and/or lines 560 to 563 resp., by one or more amino acids. In an other embodiment, said polypeptide of the invention does not consist of the sequence as indicated in Table II A or II B, columns 5 or 7, lines 174 to 177 and/or line 178 and/or line 179 and/or line180 and/or lines 181 to 185 and/or lines 558, 559 and/or lines 560 to 563 resp. In a further embodiment, said polypeptide of the present invention is less than 100%, 99,999%, 99,99%, 99,9% or 99% identical. In one embodiment, said polypeptide does not consist of the sequence encoded by a nucleic acid molecules as indicated in Table I A or I B, columns 5 or 7, lines 174 to 177 and/or line 178 and/or line 179 and/or line180 and/or lines 181 to 185 and/or lines 558, 559 and/or lines 560 to 563 resp.

**[0294.0.15.15]** In one embodiment, the present invention relates to a polypeptide having an activity of a protein as indicated in Table II, column 3, lines 174 to 177 and/or line 178 and/or line 179 and/or line180 and/or lines 181 to 185 and/or lines 558, 559 and/or lines 560 to 563 resp., which distinguishes over a sequence as indicated in Table II A or II B, columns 5 or 7, lines 174 to 177 and/or line 178 and/or line 179 and/or line180 and/or lines 181 to 185 and/or lines 558, 559 and/or lines 560 to 563 resp., by one or more amino acids, preferably by more than 5, 6, 7, 8 or 9 amino acids, preferably by more than 10, 15, 20, 25 or 30 amino acids, even more preferred are more than 40, 50, or 60 amino acids but even more preferred by less than 70% of the amino acids, more preferred by less than 50%, even more preferred my less than 30% or 25%, more preferred are 20% or 15%, even more preferred are less than 10%.

**[0295.0.0.15] to [0297.0.0.15] see [0295.0.0.0] to [0297.0.0.0]**

**[00297.1.15.15]** Non-polypeptide of the invention-chemicalsare e.g. polypeptides having not the activity of a polypeptide indicated in Table IIA or IIB, columns 5 or 7, lines 174 to 177 and/or line 178 and/or line 179 and/or line180 and/or lines 181 to 185 and/or lines 558, 559 and/or lines 560 to 563 resp..

**[0298.0.15.15]** A polypeptide of the invention can participate in the process of the present invention. The polypeptide or a portion thereof comprises preferably an amino acid sequence which is sufficiently homologous to an amino acid sequence as indicated in Table IIA or IIB, columns 5 or 7, lines 174 to 177 and/or line 178 and/or line 179 and/or line180 and/or lines 181 to 185 and/or lines 558, 559 and/or lines 560 to 563 resp., such that the protein or portion thereof maintains the ability to confer the activity of the present invention. The portion of the protein is preferably a biologically active portion as described herein. Preferably, the polypeptide used in the process of the invention has an amino acid sequence identical to a sequence as indicated in Table IIA or IIB, columns 5 or 7, lines 174 to 177 and/or line 178 and/or line 179 and/or line180 and/or lines 181 to 185 and/or lines 558, 559 and/or lines 560 to 563 resp.

**[0299.0.15.15]**Further, the polypeptide can have an amino acid sequence which is encoded by a nucleotide sequence which hybridizes, preferably hybridizes under stringent conditions as described above, to a nucleotide sequence of the nucleic acid molecule of the present invention. Accordingly, the polypeptide has an amino acid sequence which is encoded by a nucleotide sequence that is at least about 35%, 40%, 45%, 50%, 55%, 60%, 65% or 70%, preferably at least about 75%, 80%, 85% or 90, and more preferably at least about 91%, 92%, 93%, 94% or 95%, and even more preferably at least about 96%, 97%, 98%, 99% or more homologous to one of the amino acid sequences as shown in Table IIA or IIB, columns 5 or 7, lines 174 to 177 and/or line 178 and/or line 179 and/or line180 and/or lines 181 to 185 and/or lines 558, 559 and/or lines 560 to 563 resp. The preferred polypeptide of the present invention preferably possesses at least one of the activities according to the invention and described herein. A preferred polypeptide of the present invention includes an amino acid sequence encoded by a nucleotide sequence which hybridizes, preferably hybridizes under stringent conditions, to a nucleotide sequence of Table IA or IB, columns 5 or 7, lines 174 to 177 and/or line 178 and/or line 179 and/or line180 and/or lines 181 to 185 and/or lines 558, 559 and/or lines 560 to 563 resp., or which is homologous thereto, as defined above.

**[0300.0.15.15]** Accordingly the polypeptide of the present invention can vary from the sequences shown in Table IIA or IIB, columns 5 or 7, lines 174 to 177 and/or line 178 and/or line 179 and/or line180 and/or lines 181 to 185 and/or lines 558, 559 and/or lines 560 to 563 resp., in amino acid sequence due to natural variation or mutagenesis, as described in detail herein. Accordingly, the polypeptide comprise an amino acid sequence which is at least about 35%, 40%, 45%, 50%, 55%, 60%, 65% or 70%, preferably at least about 75%, 80%, 85% or 90, and more preferably at least about 91%, 92%, 93%, 94% or 95%, and most preferably at least about 96%, 97%, 98%, 99% or more homologous to an entire amino acid sequence shown in table II A or II B, columns 5 or 7, lines 174 to 177 and/or line 178 and/or line 179 and/or line180 and/or lines 181 to 185 and/or lines 558, 559 and/or lines 560 to 563 resp.

**[0301.0.0.15] see [0301.0.0.0]**

**[0302.0.15.15]** Biologically active portions of an polypeptide of the present invention include peptides comprising amino acid sequences derived from the amino acid sequence of the polypeptide of the present invention or used in the process of the present invention, e.g., the amino acid sequence shown in Table IIA or IIB, columns 5 or 7, lines 174 to 177 and/or line 178 and/or line 179 and/or line180 and/or lines 181 to 185 and/or lines 558, 559 and/or lines 560 to 563 resp., or the amino acid sequence of a protein homologous thereto, which include fewer amino acids than a full length polypeptide of the present invention or used in the process of the present invention or the full length protein which is homologous to an polypeptide of the present invention or used in the process of the present invention depicted herein, and exhibit at least one activity of polypeptide of the present invention or used in the process of the present invention.

**[0303.0.0.15] see [0303.0.0.0]**

**[0304.0.15.15]** Manipulation of the nucleic acid molecule of the invention or the nucleic acid molecule used in the method of the invention, may result in the production of a protein having essentially the activity of the polypeptides as indicated in Table IIA or IIB, column 3, lines 174 to 177 and/or line 178 and/or line 179 and/or line180 and/or lines 181 to 185 and/or lines 558, 559 and/or lines 560 to 563 resp., but having differences in the sequence from said wild-type protein. These proteins may be improved in efficiency or activity, may be present in greater numbers in the cell than is usual, or may be decreased in efficiency or activity in relation to the wild type protein.

**[0305.0.0.15] to [0308.0.0.15] see [0305.0.0.0] to [0308.0.0.0]**

**[0309.0.15.15]** In one embodiment, an reference to a " protein (= polypeptide) " of the invention or as indicated in Table IIA or IIB, columns 5 or 7, lines 174 to 177 and/or line 178 and/or line 179 and/or line180 and/or lines 181 to 185 and/or lines 558, 559 and/or lines 560 to 563 resp., refers to a polypeptide having an amino acid sequence corresponding to the polypeptide of the invention or used in the process of the invention , whereas a "non-polypeptide of the invention" or "other polypeptide" not being indicated in Table IIA or IIB, columns 5 or 7, lines 174 to 177 and/or line 178 and/or line 179 and/or line180 and/or lines 181 to 185 and/or lines 558, 559 and/or lines 560 to 563 resp., refers to a polypeptide having an amino acid sequence corresponding to a protein which is not substantially homologous a polypeptide of the invention, preferably which is not substantially homologous to a as indicated in Table IIA or IIB, columns 5 or 7, lines 174 to 177 and/or line 178 and/or line 179 and/or line180 and/or lines 181 to 185 and/or lines 558, 559 and/or lines 560 to 563 resp., e.g., a protein which does not confer the activity described herein or annotated or known for as indicated in Table IIA or IIB, column 3, lines 174 to 177 and/or line 178 and/or line 179 and/or line180 and/or lines 181 to 185 and/or lines 558, 559 and/or lines 560 to 563 resp., and which is derived from the same or a different organism. In one embodmeint, a "non-polypeptide of the invention" or "other polypeptide" not being indicated in Table IIA or IIB, columns 5 or 7, lines 174 to 177 and/or line 178 and/or line 179 and/or line180 and/or lines 181 to 185 and/or lines 558, 559 and/or lines 560 to 563 resp., does not confer an increase of the respective fine chemical in an organism or part thereof.

**[0310.0.0.15] to [0334.0.0.15] see [0310.0.0.0] to [0334.0.0.0]**

**[0335.0.15.15]** The dsRNAi method has proved to be particularly effective and advantageous for reducing the expression of the nucleic acid sequences of the table IA or IB, columns 5 or 7, lines 174 to 177 and/or line 178 and/or line 179 and/or line180 and/or lines 181 to 185 and/or lines 558, 559 and/or lines 560 to 563 resp., and/or homologs thereof. As described inter alia in WO 99/32619, dsRNAi approaches are clearly superior to traditional antisense approaches. The invention therefore furthermore relates to double-stranded RNA molecules (dsRNA molecules) which, when introduced into an organism, advantageously into a plant (or a cell, tissue, organ or seed derived therefrom), bring about altered metabolic activity by the reduction in the expression of the nucleic acid sequences of the Table IA or IB, columns 5 or 7, lines 174 to 177 and/or line 178 and/or line 179 and/or line180 and/or lines 181 to 185 and/or lines 558, 559 and/or lines 560 to 563 resp., and/or homologs thereof. In a double-stranded RNA molecule for reducing the expression of an protein encoded by a nucleic acid sequence of one of the Table IA orr IB, columns 5 or 7, lines 174 to 177 and/or line 178 and/or line 179 and/or line180 and/or lines 181 to 185 and/or lines 558, 559 and/or lines 560 to 563 resp., and/or homologs thereof, one of the two RNA strands is essentially identical to at least part of a nucleic acid sequence, and the respective other RNA strand is essentially identical to at least part of the complementary strand of a nucleic acid sequence.

**[0336.0.0.15] to [0342.0.0.15] see [0336.0.0.0] to [0342.0.0.0]**

**[0343.0.15.15]** As has already been described, 100 % sequence identity between the dsRNA and a gene transcript of a nucleic acid sequence of one of the sequences shown in Table IA or IB, columns 5 or 7, lines 174 to 177 and/or line 178 and/or line 179 and/or line180 and/or lines 181 to 185 and/or lines 558, 559 and/or lines 560 to 563 resp., or it's homolog is not necessarily required in order to bring about effective reduction in the expression. The advantage is, accordingly, that the method is tolerant with regard to sequence deviations as may be present as a consequence of genetic mutations, polymorphisms or evolutionary divergences. Thus, for example, using the dsRNA, which has been generated starting from a sequence of one of sequences shown in Table IA or IB, columns 5 or 7, lines 174 to 177 and/or line 178 and/or line 179 and/or line180 and/or lines 181 to 185 and/or lines 558, 559 and/or lines 560 to 563 resp., or homologs thereof of the one organism, may be used to suppress the corresponding expression in another organism.

**[0344.0.0.15] to [0361.0.0.15] see [0344.0.0.0] to [0361.0.0.0]**

**[0362.0.15.15]** Accordingly the present invention relates to any cell transgenic for any nucleic acid characterized as part of the invention, e.g. conferring the increase of the respective fine chemical in a cell or an organism or a part thereof, e.g. the nucleic acid molecule of the invention, the nucleic acid construct of the invention, the antisense molecule of the invention, the vector of the invention or a nucleic acid molecule encoding the polypeptide of the invention, e.g. the polypeptide as indicated in Table IIA or IIB, columns 5 or 7, lines 174 to 177 and/or line 178 and/or line 179 and/or line180 and/or lines 181 to 185 and/or lines 558, 559 and/or lines 560 to 563 resp. Due to the above mentioned activity the fine chemical content in a cell or an organism is increased. For example, due to modulation or manipulation, the cellular activity of the polypeptide of the invention or nucleic acid molecule of the invention is increased, e.g. due to an increased expression or specific activity of the subject matters of the invention in a cell or an organism or a part thereof. In one embodiment, transgenic for a polypeptide having an activity of a polypeptide as indicated in Table IIA or IIB, columns 5 or 7, lines 174 to 177 and/or line 178 and/or line 179 and/or line180 and/or lines 181 to 185 and/or lines 558, 559 and/or lines 560 to 563 resp., means herein that due to modulation or manipulation of the genome, an activity as annotated for a polypeptide as indicated in Table IIA or IIB, column 3, lines 174 to 177 and/or line 178 and/or line 179 and/or line180 and/or lines 181 to 185 and/or lines 558, 559 and/or lines 560 to 563 resp., e.g. having a sequence as indicated in Table IIA or IIB, columns 5 or 7, lines 174 to 177 and/or line 178 and/or line 179 and/or line180 and/or lines 181 to 185 and/or lines 558, 559 and/or lines 560 to 563 resp.,is increased in a cell or an organism or a part thereof. Examples are described above in context with the process of the invention.

**[0363.0.0.15] see [0363.0.0.0]**

**[0364.0.15.15]**A naturally occurring expression cassette - for example the naturally occurring combination of a promoter of a polypeptide of the invention as indicated in Table IIA or IIB, column 3 and/or 5, lines 174 to 177 and/or line 178 and/or line 179 and/or line180 and/or lines 181 to 185 and/or lines 558, 559 and/or lines 560 to 563 resp., with the corresponding protein-encoding sequence as indicated in Table IA or IB, column 3 and/or 5, lines 174 to 177 and/or line 178 and/or line 179 and/or line180 and/or lines 181 to 185 and/or lines 558, 559 and/or lines 560 to 563 resp.,

**[0365.0.0.15] to [0373.0.0.15]see [0365.0.0.0] to [0373.0.0.0]**

**[0374.0.15.15]** Transgenic plants comprising the fatty acids synthesized in the process according to the invention can be marketed directly without isolation of the compounds synthesized. In the process according to the invention, plants are understood as meaning all plant parts, plant organs such as leaf, stalk, root, tubers or seeds or propagation material or harvested material or the intact plant. In this context, the seed encompasses all parts of the seed such as the seed coats, epidermal cells, seed cells, endosperm or embryonic tissue. The fatty acids produced in the process according to the invention may, however, also be isolated from the plant in the form of their free fatty acids or bound in or to compounds. Fatty acids produced by this process can be harvested by harvesting the organisms either from the culture in which they grow or from the field. This can be done via expressing, grinding and/or extraction, salt precipitation and/or ion-exchange chromatography or other chromatographic methods of the plant parts, preferably the plant seeds, plant fruits, plant tubers and the like.

**[0375.0.0.15] to [0376.0.0.15] see [0375.0.0.0] to [0376.0.0.0]**

**[0377.0.15.15]** Accordingly, the present invention relates also to a process according to the present invention whereby the produced fatty acid and/or fatty acid composition or the produced the fine chemical is isolated.

**[0378.0.15.15]** In this manner, more than 50% by weight, advantageously more than 60% by weight, preferably more than 70% by weight, especially preferably more than 80% by weight, very especially preferably more than 90% by weight, of the fatty acids produced in the process can be isolated. The resulting fatty acids can, if appropriate, subsequently be further purified, if desired mixed with other active ingredients such as vitamins, amino acids, carbohydrates, antibiotics and the like, and, if appropriate, formulated.

**[0379.0.15.15]** In one embodiment, the amino acid, in particular, heptadecanoic acid and/or 2-hydroxy palmitic acid and/or 2-hydroxy-tetracosenoic-acid and/or hexadecadienoic acid, preferably delta 7, 10 hexadecadienoic acid and/or hexadecatrienoic acid, preferably delta 7, 10 ,13 hexadecatrienoic acid resp., is a mixture comprising of one or more the respective fine chemicals. In one embodiment, the respective fine chemical means here amino acid, in particular heptadecanoic acid and/or 2-hydroxy palmitic acid and/or 2-hydroxy-tetracosenoic-acid and/or hexadecadienoic acid, preferably delta 7, 10 hexadecadienoic acid and/or hexadecatrienoic acid, preferably delta 7, 10 ,13 hexadecatrienoic acid and/or 24:1 fatty acid, preferably C24:1 delta 15 fatty acid. In one embodiment, amino acid means here a mixture of the respective fine chemicals.

**[0380.0.15.15]** The fatty acids obtained in the process are suitable as starting material for the synthesis of further products of value. For example, they can be used in combination with each other or alone for the production of pharmaceuticals, foodstuffs, animal feeds or cosmetics. Accordingly, the present invention relates a method for the production of a pharmaceuticals, food stuff, animal feeds, nutrients or cosmetics comprising the steps of the process according to the invention, including the isolation of the fatty acid composition produced or the fine chemical produced if desired and formulating the product with a pharmaceutical acceptable carrier or formulating the product in a form acceptable for an application in agriculture. A further embodiment according to the invention is the use of the fatty acids produced in the process or of the transgenic organisms in animal feeds, foodstuffs, medicines, food supplements, cosmetics or pharmaceuticals.

**[0380.1.15.15]** The fatty acids obtained in the process are further suitable in purified form as internal standard in quantification of fatty acids. They can be also used as starting compound in the synthesis of further fatty acids, by elongation, saturation or desaturation resp., hydroxylation or synthesis of glycerides or lipids, preferably glycerophospholipids, sphingolipids and/or galactolipids.

**[0381.0.0.15] to [0382.0.0.15] see [0381.0.0.0] to [0382.0.0.0]**

**[0383.0.15.15]** For preparing fatty acid compound-containing fine chemicals, in particular the fine chemical, it is possible to use as fatty acid source organic compounds such as, for example, oils, fats and/or lipids comprising fatty acids such as fatty acids having a carbon back bone between C₁₀- to C₁₆- carbon atoms and/or small organic acids such acetic acid, propionic acid or butanoic acid as precursor compounds.

**[0384.0.0.15] see [0384.0.0.0]**

**[0385.0.15.15]** The fermentation broths obtained in this way, containing in particular heptadecanoic acid and/or 2-hydroxy palmitic acid and/or 2-hydroxy-tetracosenoic-acid and/or hexadecadienoic acid, preferably delta 7, 10 hexadecadienoic acid and/or hexadecatrienoic acid, preferably delta 7, 10 ,13 hexadecatrienoic acid and/or 24:1 fatty acid, preferably C24:1 delta 15 fatty acid in mixtures with other lipids, fats and/or oils, normally have a dry matter content of from 7.5 to 25% by weight. Sugar-limited fermentation is additionally advantageous, at least at the end, but especially over at least 30% of the fermentation time. This means that the concentration of utilizable sugar in the fermentation medium is kept at, or reduced to, 0 to 3 g/l during this time. The fermentation broth is then processed further. Depending on requirements, the biomass can be removed entirely or partly by separation methods, such as, for example, centrifugation, filtration, decantation or a combination of these methods, from the fermentation broth or left completely in it. The fermentation broth can then be thickened or concentrated by known methods, such as, for example, with the aid of a rotary evaporator, thin-film evaporator, falling film evaporator, by reverse osmosis or by nanofiltration. This concentrated fermentation broth can then be worked up by freeze-drying, spray drying, spray granulation or by other processes.

**[0386.0.15.15]** However, it is also possible to purify the fatty acid produced further. For this purpose, the product-containing composition is subjected for example to.a thin layer chromatography on silica gel plates or to a chromatography such as a Florisil column (Bouhours J.F., J. Chromatrogr. 1979, 169, 462), in which case the desired product or the impurities are retained wholly or partly on the chromatography resin. These chromatography steps can be repeated if necessary, using the same or different chromatography resins. The skilled worker is familiar with the choice of suitable chromatography resins and their most effective use. An alternative method to purify the fatty acids is for example crystallization in the presence of urea. These methods can be combined with each other.

**[0387.0.0.15] to [0392.0.0.15] see [0387.0.0.0] to [0392.0.0.0]**

**[0393.0.15.15]** In one embodiment, the present invention relates to a method for the identification of a gene product conferring an increase in the fine chemical production in a cell, comprising the following steps:
(m) contacting, e.g. hybridising, the nucleic acid molecules of a sample, e.g. cells, tissues, plants or microorganisms or a nucleic acid library , which can contain a candidate gene encoding a gene product conferring an increase in the fine chemical after expression, with the nucleic acid molecule of the present invention;
(n) identifying the nucleic acid molecules, which hybridize under relaxed stringent conditions with the nucleic acid molecule of the present invention in particular to the nucleic acid molecule sequence shown in table IA or IB, preferably table I B, columns 5 or 7, lines 174 to 177 and/or line 178 and/or line 179 and/or line180 and/or lines 181 to 185 and/or lines 558, 559 and/or lines 560 to 563 resp., and, optionally, isolating the full length cDNA clone or complete genomic clone;
(o) introducing the candidate nucleic acid molecules in host cells, preferably in a plant cell or a microorganism, appropriate for producing the fine chemical;
(p) expressing the identified nucleic acid molecules in the host cells;
(q) assaying the fine chemical level in the host cells; and
(r) identifying the nucleic acid molecule and its gene product which expression confers an increase in the fine chemical level in the host cell after expression compared to the wild type.

**[0394.0.0.15] to [0398.0.0.15] see [0394.0.0.0] to [0398.0.0.0]**

**[0399.0.15.15]** Furthermore, in one embodiment, the present invention relates to process for the identification of a compound conferring increased the fine chemical production in a plant or microorganism, comprising the steps:
(g) culturing a cell or tissue or microorganism or maintaining a plant expressing the polypeptide according to the invention or a nucleic acid molecule encoding said polypeptide and a readout system capable of interacting with the polypeptide under suitable conditions which permit the interaction of the polypeptide with said readout system in the presence of a compound or a sample comprising a plurality of compounds and capable of providing a detectable signal in response to the binding of a compound to said polypeptide under conditions which permit the expression of said readout system and the polypeptide of the present invention or used in the process of the invention; and
(h) identifying if the compound is an effective agonist by detecting the presence or absence or increase of a signal produced by said readout system.
The screen for a gene product or an agonist conferring an increase in the fine chemical production can be performed by growth of an organism for example a microorganism in the presence of growth reducing amounts of an inhibitor of the synthesis of the fine chemical. Better growth, eg higher dividing rate or high dry mass in comparison to the control under such conditions would identify a gene or gene product or an agonist conferring an increase in fine chemical production.
One can think to screen for increased fine chemical production by for example resistance to drugs blocking fatty synthesis and looking whether this effect is YDR513W dependent eg comparing near identical organisms with low and high YDR513W activity.

**[00399.1.15.15]** One can think to screen for increased production of the respective fine chemical by for example searching for a resistance to a drug blocking the synthesis of the fine chemical and looking whether this effect is dependent on the activity or expression of a polypeptide as indicated in Table IIA or IIB, columns 5 or 7, lines 174 to 177 and/or line 178 and/or line 179 and/or line180 and/or lines 181 to 185 and/or lines 558, 559 and/or lines 560 to 563 resp., or a homolog thereof, e.g. comparing the phenotype of nearly identical organisms with low and high activity of a protein as indicated in Table IIA or IIB, columns 5 or 7, lines 174 to 177 and/or line 178 and/or line 179 and/or line180 and/or lines 181 to 185 and/or lines 558, 559 and/or lines 560 to 563 resp., after incubation with the drug.

**[0400.0.0.15] to [0416.0.0.15] see [0400.0.0.0] to [0416.0.0.0]**

**[0417.0.15.15]** The nucleic acid molecule of the invention, the vector of the invention or the nucleic acid construct of the invention may also be useful for the production of organisms resistant to inhibitors of the fatty acid production biosynthesis pathways. In particular, the overexpression of the polypeptide of the present invention may protect an organism such as a microorganism or a plant against inhibitors, which block the fatty acid, in particular the fine chemical, synthesis in said organism. Examples of inhibitors or herbicides blocking the fatty acid synthesis in organism such as microorganism or plants are for example cerulenin, Thiolactomycin, Diazoborines or Triclosan, which inhibit the fatty acids (beta-ketoacyl thioester synthetase inhibitors) and sterol biosynthesis inhibitors, aryloxyphenoxypropionates such as diclofop, fenoxaprop, haloxyfop, fluazifop or quizalofop or cyclohexanediones such as clethodim or sethoxydim [(2-[1-{ethoxyimino}butyl]-5-[2-{ethylthio}propyl]-3-hydroxy-2-cyclohexen-1-one], which inhibit the plant acetyl-coenzyme A carboxylase or thiocarbamates such as butylate, EPTC [= S-ethyl dipropylcarbamothioat] or vernolate.

**[0418.0.0.15] to [0423.0.0.15] see [0418.0.0.0] to [0423.0.0.0]**

**[0424.0.15.15]** Accordingly, the nucleic acid of the invention, the polypeptide of the invention, the nucleic acid construct of the invention, the organisms, the host cell, the microorgansims, the plant, plant tissue, plant cell, or the part thereof of the invention, the vector of the invention, the agonist identified with the method of the invention, the nucleic acid molecule identified with the method of the present invention, can be used for the production of the fine chemical or of the fine chemical and one or more other fatty acids, in particular heptadecanoic acid and/or 2-hydroxy palmitic acid and/or 2-hydroxy-tetracosenoic-acid and/or hexadecadienoic acid, preferably delta 7, 10 hexadecadienoic acid and/or hexadecatrienoic acid, preferably delta 7, 10 ,13 hexadecatrienoic acid and/or 24:1 fatty acid, preferably C24:1 delta 15 fatty acid mixtures thereof or mixtures of other fatty acids. Accordingly, the nucleic acid of the invention, or the nucleic acid molecule identified with the method of the present invention or the complement sequences thereof, the polypeptide of the invention, the nucleic acid construct of the invention, the organisms, the host cell, the microorganisms, the plant, plant tissue, plant cell, or the part thereof of the invention, the vector of the invention, the antagonist identified with the method of the invention, the antibody of the present invention, the antisense molecule of the present invention, can be used for the reduction of the fine chemical in a organism or part thereof, e.g. in a cell.

**[0425.0.0.15] to [0430.0.0.15] see [0425.0.0.0] to [0430.0.0.0]**

**[0431.0.15.15]** Example 1: Cloning SEQ ID NO: 13930 in Escherichia coli

**[0432.0.15.15]** SEQ ID NO: 13930 was cloned into the plasmids pBR322 (Sutcliffe, J.G. (1979) Proc. Natl Acad. Sci. USA, 75: 3737-3741); pACYC177 (Change & Cohen (1978) J. Bacteriol. 134: 1141-1156); plasmids of the pBS series (pBSSK+, pBSSK- and others; Stratagene, LaJolla, USA) or cosmids such as SuperCos1 (Stratagene, LaJolla, USA) or Lorist6 (Gibson, T.J. Rosenthal, A., and Waterson, R.H. (1987) Gene 53: 283-286) for expression in E. coli using known, well-established procedures (see, for example, Sambrook, J. et al. (1989) "Molecular Cloning: A Laboratory Manual". Cold Spring Harbor Laboratory Press or Ausubel, F.M. et al. (1994) "Current Protocols in Molecular Biology", John Wiley & Sons).

**[0433.0.0.15] to [0434.0.0.15] see [0433.0.0.0] to [0434.0.0.0]**

**[0435.0.15.15]** Example 3: In-vivo and in-vitro mutagenesis

**[0436.0.15.15]** An *in vivo* mutagenesis of organisms such as Saccharomyces, Mortierella, Escherichia and others mentioned above, which are beneficial for the production of fatty acids can be carried out by passing a plasmid DNA (or another vector DNA) containing the desired nucleic acid sequence or nucleic acid sequences through E. coli and other microorganisms (for example Bacillus spp. or yeasts such as Saccharomyces cerevisiae) which are not capable of maintaining the integrity of its genetic information. Usual mutator strains have mutations in the genes for the DNA repair system [for example mutHLS, mutD, mutT and the like; for comparison, see Rupp, W.D. (1996) DNA repair mechanisms in Escherichia coli and Salmonella, pp. 2277-2294, ASM: Washington]. The skilled worker knows these strains. The use of these strains is illustrated for example in Greener, A. and Callahan, M. (1994) Strategies 7; 32-34.
In-vitro mutation methods such as increasing the spontaneous mutation rates by chemical or physical treatment are well known to the skilled person. Mutagens like 5-bromo-uracil, N-methyl-N-nitro-N-nitrosoguanidine (= NTG), ethyl methanesulfonate (= EMS), hydroxylamine and/or nitrous acid are widly used as chemical agents for random in-vitro mutagensis. The most common physical method for mutagensis is the treatment with UV irradiation. Another random mutagenesis technique is the error-prone PCR for introducing amino acid changes into proteins. Mutations are deliberately introduced during PCR through the use of error-prone DNA polymerases and special reaction conditions known to a person skilled in the art. For this method randomized DNA sequences are cloned into expression vectors and the resulting mutant libraries screened for altered or improved protein activity as described below.
Site-directed mutagensis method such as the introduction of desired mutations with an M13 or phagemid vector and short oligonucleotides primers is a well-known approach for site-directed mutagensis. The clou of this method involves cloning of the nucleic acid sequence of the invention into an M13 or phagemid vector, which permits recovery of single-stranded recombinant nucleic acid sequence. A mutagenic oligonucleotide primer is then designed whose sequence is perfectly complementary to nucleic acid sequence in the region to be mutated, but with a single difference: at the intended mutation site it bears a base that is complementary to the desired mutant nucleotide rather than the original. The mutagenic oligonucleotide is then allowed to prime new DNA synthesis to create a complementary full-length sequence containing the desired mutation. Another site-directed mutagensis method is the PCR mismatch primer mutagensis method also known to the skilled person. DpnI site-directed mutagensis is a further known method as described for example in the Stratagene QuickchangeTM site-directed mutagenesis kit protocol. A huge number of other methods are also known and used in common practice.
Positive mutation events can be selected by screening the organisms for the production of the desired fine chemical.

**[0437.0.15.15]** Example 4: DNA transfer between Escherichia coli, Saccharomyces cerevisiae and Mortierella alpine

**[0438.0.15.15]** Shuttle vectors such as pYE22m, pPAC-ResQ, pClasper, pAUR224, pAMH10, pAML10, pAMT10, pAMU10, pGMH10, pGML10, pGMT10, pGMU10, pPGAL1, pPADH1, pTADH1, pTAex3, pNGA142, pHT3101 and derivatives thereof which alow the transfer of nucleic acid sequences between Escherichia coli, Saccharomyces cerevisiae and/or Mortierella alpina are available to the skilled worker. An easy method to isolate such shuttle vectors is disclosed by Soni R. and Murray J.A.H. [Nucleic Acid Research, vol. 20 no. 21, 1992: 5852]: If necessary such shuttle vectors can be constructed easily using standard vectors for E. coli (Sambrook, J. et al., (1989), "Molecular Cloning: A Laboratory Manual", Cold Spring Harbor Laboratory Press or Ausubel, F.M. et al. (1994) "Current Protocols in Molecular Biology", John Wiley & Sons) and/or the aforementioned vectors, which have a replication origin for, and suitable marker from, Escherichia coli, Saccharomyces cerevisiae or Mortierella alpina added. Such replication origins are preferably taken from endogenous plasmids, which have been isolated from species used in the inventive process. Genes, which are used in particular as transformation markers for these species are genes for kanamycin resistance (such as those which originate from the Tn5 or Tn-903 transposon) or for chloramphenicol resistance (Winnacker, E.L. (1987) "From Genes to Clones - Introduction to Gene Technology, VCH, Weinheim) or for other antibiotic resistance genes such as for G418, gentamycin, neomycin, hygromycin or tetracycline resistance.

**[0439.0.15.15]** Using standard methods, it is possible to clone a gene of interest into one of the above-described shuttle vectors and to introduce such hybrid vectors into the microorganism strains used in the inventive process. The transformation of Saccharomyces can be achieved for example by LiCl or sheroplast transformation (Bishop et al., Mol. Cell. Biol., 6, 1986: 3401 - 3409; Sherman et al., Methods in Yeasts in Genetics, [Cold Spring Harbor Lab. Cold Spring Harbor, N. Y.] 1982, Agatep et al., Technical Tips Online 1998, 1:51: P01525 or Gietz et al., Methods Mol. Cell. Biol. 5, 1995: 255269) or electroporation (Delorme E., Appl. Environ. Microbiol., vol. 55, no. 9, 1989 : 2242 - 2246).

**[0440.0.15.15]** If the transformed sequence(s) is/are to be integrated advantageously into the genome of the microorganism used in the inventive process for example into the yeast or fungi genome, standard techniques known to the skilled worker also exist for this purpose. Solinger et al. (Proc Natl Acad Sci U S A., 2001 (15): 8447-8453) and Freedman et al. (Genetics, Vol. 162, 15-27, September 2002,) teaches a homolog recombination system dependent on rad 50, rad51, rad54 and rad59 in yeasts. Vectors using this system for homologous recombination are vectors derived from the Ylp series. Plasmid vectors derived for example from the 2µ-Vector are known by the skilled worker and used for the expression in yeasts. Other preferred vectors are for example pART1, pCHY21 or pEVP11 as they have been described by McLeod et al. (EMBO J. 1987, 6:729-736) and Hoffman et al. (Genes Dev. 5, 1991: 561-571.) or Russell et al. (J. Biol. Chem. 258, 1983: 143-149.). Other beneficial yeast vectors are plasmids of the REP, REP-X, pYZ or RIP series.

**[0441.0.0.15] see [0441.0.0.0]**

**[0442.0.15.15]** The observations of the acivity of a mutated, or transgenic, protein in a transformed host cell are based on the fact that the protein is expressed in a similar manner and in a similar quantity as the wild-type protein. A suitable method for determining the transcription quantity of the mutant, or transgenic, gene (a sign for the amount of mRNA which is available for the translation of the gene product) is to carry out a Northern blot (see, for example, Ausubel et al., (1988) Current Protocols in Molecular Biology, Wiley: New York), where a primer which is designed in such a way that it binds to the gene of interest is provided with a detectable marker (usually a radioactive or chemiluminescent marker) so that, when the total RNA of a culture of the organism is extracted, separated on a gel, applied to a stable matrix and incubated with this probe, the binding and quantity of the binding of the probe indicates the presence and also the amount of mRNA for this gene. Another method is a quantitative PCR. This information detects the extent to which the gene has been transcribed. Total cell RNA can be isolated for example from yeasts or E. coli by a variety of methods, which are known in the art, for example with the Ambion kit according to the instructions of the manufacturer or as described in Edgington et al., Promega Notes Magazine Number 41, 1993, p. 14.

**[0443.0.0.15] see [0443.0.0.0]**

**[0444.0.15.15]** Example 6: Growth of genetically modified organism: media and culture conditions

**[0445.0.15.15]** Genetically modified Yeast, Mortierella or Escherichia coli are grown in synthetic or natural growth media known by the skilled worker. A number of different growth media for Yeast, Mortierella or Escherichia coli are well known and widely available. A method for clulturing Mortierella is disclosed by Jang et al. [Bot. Bull. Acad. Sin. (2000) 41: 41-48]. Mortierella can be grown at 20°C in a culture medium containing: 10 g/l glucose, 5 g/I yeast extract at pH 6.5. Futhermore Jang et al. teaches a submerged basal medium containing 20 g/l soluble starch, 5 g/l Bacto yeast extract, 10 g/l KNO₃, 1 g/l KH₂PO₄, and 0.5 g/l MgSO₄•7H₂O, pH 6.5.

**[0446.0.0.15] to [0450.0.0.15] see [0446.0.0.0] to [0450.0.0.0]**

**[0451.0.15.15]** If genetically modified clones are examined, an unmodified control clone, or a control clone, which contains the basic plasmid without insertion, should also be included in the tests. If a transgenic sequence is expressed, a control clone should advantageously again be included in these tests. The medium is advantageously inoculated to an OD600 of 0.5 to 1.5 using cells which have been grown on agar plates, such as CM plates (10 g/I glucose, 2.5 g/l NaCl, 2 g/l urea, 10 g/l polypeptone, 5 g/l yeast extract, 5 g/I meat extract, 22 g/l agar, pH value 6.8 established with 2M NaOH), which have been incubated at 30°C. The media are inoculated for example by addition of a liquid preculture of seed organism such as E. coli or S. cerevisiae.

**[0452.0.0.15] to [0453.0.0.15] see [0452.0.0.0] to [0453.0.0.0]**

**[0454.0.15.15]** Example 8: Analysis of the effect of the nucleic acid molecule on the production of the fatty acids

**[0455.0.15.15]** The effect of the genetic modification in plants, fungi, algae, ciliates or on the production of a desired compound (such as a fatty acid) can be determined by growing the modified microorganisms or the modified plant under suitable conditions (such as those described above) and analyzing the medium and/or the cellular components for the elevated production of desired product (i.e. of the lipids or a fatty acid). These analytical techniques are known to the skilled worker and comprise spectroscopy, thin-layer chromatography, various types of staining methods, enzymatic and microbiological methods and analytical chromatography such as high-performance liquid chromatography (see, for example, Ullman, Encyclopedia of Industrial Chemistry, Vol. A2, p. 89-90 and p. 443-613, VCH: Weinheim (1985); Fallon, A., et al., (1987) "Applications of HPLC in Biochemistry" in: Laboratory Techniques in Biochemistry and Molecular Biology, Vol. 17; Rehm et al. (1993) Biotechnology, Vol. 3, Chapter III: "Product recovery and purification", p. 469-714, VCH: Weinheim; Belter, P.A., et al. (1988) Bioseparations: downstream processing for Biotechnology, John Wiley and Sons; Kennedy, J.F., and Cabral, J.M.S. (1992) Recovery processes for biological Materials, John Wiley and Sons; Shaeiwitz, J.A., and Henry, J.D. (1988) Biochemical Separations, in: Ullmann's Encyclopedia of Industrial Chemistry, Vol. B3; Chapter 11, p. 1-27, VCH: Weinheim; and Dechow, F.J. (1989) Separation and purification techniques in biotechnology, Noyes Publications).
In addition to the abovementioned processes, plant lipids are extracted from plant material as described by Cahoon et al. (1999) Proc. Natl. Acad. Sci. USA 96 (22): 12935-12940 and Browse et al. (1986) Analytic Biochemistry 152:141-145. The qualitative and quantitative analysis of lipids or fatty acids is described by Christie, William W., Advances in Lipid Methodology, Ayr/Scotland: Oily Press (Oily Press Lipid Library; 2); Christie, William W., Gas Chromatography and Lipids. A Practical Guide - Ayr, Scotland: Oily Press, 1989, Repr. 1992, IX, 307 pp. (Oily Press Lipid Library; 1); "Progress in Lipid Research, Oxford: Pergamon Press, 1 (1952) - 16 (1977) under the title: Progress in the Chemistry of Fats and Other Lipids CODEN.

**[0456.0.0.15] see [0456.0.0.0]** In addition to the determination of the fermentation end product, other components of the metabolic pathways which are used for the production of the desired compound, such as intermediates and by-products, may also be analyzed in order to determine the total productivity of the organism, the yield and/or production efficiency of the compound. The analytical methods encompass determining the amounts of nutrients in the medium (for example sugars, hydrocarbons, nitrogen sources, phosphate and other ions), determining biomass composition and growth, analyzing the production of ordinary metabolites from biosynthetic pathways and measuring gases generated during the fermentation. Standard methods for these are described in Applied Microbial Physiology; A Practical Approach, P.M. Rhodes and P.F. Stanbury, Ed. IRL Press, pp. 103-129; 131-163 and 165-192 (ISBN: 0199635773) and the references cited therein.

**[0457.0.15.15]** Example 9: Purification of the fatty acid

**[0458.0.15.15]** One example is the analysis of fatty acids (abbreviations: FAME, fatty acid methyl ester; GC-MS, gas liquid chromatography/mass spectrometry; TAG, triacylglycerol; TLC, thin-layer chromatography).
The unambiguous detection for the presence of fatty acid products can be obtained by analyzing recombinant organisms using analytical standard methods: GC, GC-MS or TLC, as described on several occasions by Christie and the references therein (1997, in: Advances on Lipid Methodology, Fourth Edition: Christie, Oily Press, Dundee, 119-169; 1998, Gaschromatographie-Massenspektrometrie-Verfahren [Gas chromatography/mass spectrometric methods], Lipide 33:343-353).
The total fatty acids produced in the organism for example in yeasts used in the inventive process can be analysed for example according to the following procedure:
The material such as yeasts, E. coli or plants to be analyzed can be disrupted by sonication, grinding in a glass mill, liquid nitrogen and grinding or via other applicable methods. After disruption, the material must be centrifuged (1000 x g, 10 min., 4°C) and washed once with 100 mM NaHCO₃, pH 8.0 to remove residual medium and fatty acids. For preparation of the fatty acid methyl esters (FAMES) the sediment is resuspended in distilled water, heated for 10 minutes at 100°C, cooled on ice and recentrifuged, followed by extraction for one hour at 90°C in 0.5 M sulfuric acid in methanol with 2% dimethoxypropane, which leads to hydrolyzed oil and lipid compounds, which give transmethylated lipids.
The FAMES are then extracted twice with 2 ml petrolether, washed once with 100 mM NaHCO₃, pH 8.0 and once with distilled water and dried with Na₂SO₄. The organic solvent can be evaporated under a stream of Argon and the FAMES were dissolved in 50 µl of petrolether. The samples can be separated on a ZEBRON ZB-Wax capillary column (30 m, 0,32 mm, 0,25 µm; Phenomenex) in a Hewlett Packard 6850 gas chromatograph with a flame ionisation detector. The oven temperature is programmed from 70°C (1 min. hold) to 200°C at a rate of 20°C/min., then to 250°C (5 min. hold) at a rate of 5°C/min and finally to 260°C at a rate of 5°C/min. Nitrogen is used as carrier gas (4,5 ml/min. at 70°C). The identity of the resulting fatty acid methyl esters can be identified by comparison with retention times of FAME standards, which are available from commercial sources (i.e. Sigma).
Plant material is initially homogenized mechanically by comminuting in a pestle and mortar to make it more amenable to extraction.
This is followed by heating at 100°C for 10 minutes and, after cooling on ice, by resedimentation. The cell sediment is hydrolyzed for one hour at 90°C with 1 M methanolic sulfuric acid and 2% dimethoxypropane, and the lipids are transmethylated. The resulting fatty acid methyl esters (FAMEs) are extracted in petroleum ether. The extracted FAMEs are analyzed by gas liquid chromatography using a capillary column (Chrompack, WCOT Fused Silica, CP-Wax-52 CB, 25 m, 0.32 mm) and a temperature gradient of from 170°C to 240°C in 20 minutes and 5 minutes at 240°C. The identity of the fatty acid methyl esters is confirmed by comparison with corresponding FAME standards (Sigma). The identity and position of the double bond can be analyzed further by suitable chemical derivatization of the FAME mixtures, for example to give 4,4-dimethoxyoxazoline derivatives (Christie, 1998) by means of GC-MS.
The methodology is described for example in Napier and Michaelson, 2001, Lipids. 36(8):761-766; Sayanova et al., 2001, Journal of Experimental Botany. 52(360):1581-1585, Sperling et al., 2001, Arch. Biochem. Biophys. 388(2):293-298 and Michaelson et al., 1998, FEBS Letters. 439(3):215-218.

**[0459.0.15.15]** If required and desired, further chromatography steps with a suitable resin may follow. Advantageously the fatty acids can be further purified with a so-called RTHPLC. As eluent different an acetonitrile/water or chloroform/acetonitrile mixtures are advantageously is used. For example canola oil can be separated said HPLC method using an RP-18-column (ET 250/3 Nucleosil 120-5 C₁₈ Macherey und Nagel, Düren, Germany). A chloroform/acetonitrile mixture (v/v 30:70) is used as eluent. The flow rate is beneficial 0.8 ml/min. For the analysis of the fatty acids an ELSD detector (evaporative light-scattering detector) is used. MPLC, dry-flash chromatography or thin layer chromatography are other beneficial chromatography methods for the purification of fatty acids. If necessary, these chromatography steps may be repeated, using identical or other chromatography resins. The skilled worker is familiar with the selection of suitable chromatography resin and the most effective use for a particular molecule to be purified.

**[0460.0.15.15]** In addition depending on the produced fine chemical purification is also possible with cristalisation or destilation. Both methods are well known to a person skilled in the art.

**[0461.0.15.15]**Example 10: Cloning SEQ ID NO: 13930 for the expression in plants

**[0462.0.0.15] see [0462.0.0.0]**

**[0463.0.15.15]**SEQ ID NO: 13930 is amplified by PCR as described in the protocol of the Pfu Turbo or DNA Herculase polymerase (Stratagene).

**[0464.0.15.15]** The composition for the protocol of the *Pfu* Turbo DNA polymerase was as follows: 1 x PCR buffer (Stratagene), 0.2 mM of each dNTP, 100 ng genomic DNA of *Saccharomyces cerevisiae* (strain S288C; Research Genetics, Inc., now Invitrogen) or *Escherichia coli* (strain MG1655; *E.coli* Genetic Stock Center), 50 pmol forward primer, 50 pmol reverse primer, 2.5 u Pfu Turbo DNA polymerase. The amplification cycles were as follows:

**[0465.0.15.15]** 1 cycle of 3 minutes at 94-95°C, followed by 25-36 cycles of in each case 1 minute at 95°C or 30 seconds at 94°C, 45 seconds at 50°C, 30 seconds at 50°C or 30 seconds at 55°C and 210-480 seconds at 72°C, followed by 1 cycle of 8 minutes at 72°C, then 4°C.

**[0466.0.0.15] see [0466.0.0.0]**

**[0461.0.15.15]** The following primer sequences were selected for the gene SEQ ID NO: 13930:
i) forward primer (SEQ ID NO: 13950)
   atgaactcta ttttagacag aaatgtt
ii) reverse primer (SEQ ID NO: 13951)
   ttatttttgg tcttgtttca aagtgtc
or were selected for the genes described in Table III, column 5, lines 174 to 177 and/or line 178 and/or line 179 and/or line180 and/or lines 181 to 185 and/or lines 558, 559 and/or lines 560 to 563 as described in Table III, column 7, lines 174 to 177 and/or line 178 and/or line 179 and/or line180 and/or lines 181 to 185 and/or lines 558, 559 and/or lines 560 to 563.

**[0468.0.0.15] to [0479.0.0.15] see [0468.0.0.0] to [0479.0.0.0]**

**[0480.0.15.15]** Example 11: Generation of transgenic plants which express SEQ ID NO: 13930

**[0481.0.0.15] to [0513.0.0.15] see [0481.0.0.0] to [0513.0.0.0]**

**[0514.0.15.15]** As an alternative, the fatty acids can be detected advantageously via HPLC separation in ethanolic extract as described by Geigenberger et al. (Plant Cell & Environ, 19, 1996: 43-55).
The results of the different plant analyses can be seen from the table, which follows:

**Table 1**

| ORF | Metabolite | Method | Min | Max |
|---|---|---|---|---|
| YDR513W | Margaric Acid (C17:0) | GC | 1,24 | 1,97 |
| YER156C | Margaric Acid (C17:0) | GC | 1,20 | 1,49 |
| YLR255C | Margaric Acid (C17:0) | GC | 1,20 | 1,27 |
| b1829 | Margaric Acid (C17:0) | GC | 1,20 | 2,33 |
| YER173W | 2-Hydroxy-Palmitic Acid | GC | 1,26 | 4,94 |
| YFR042W | 2-Hydroxy-tetracosenoic acid | GC | 1,28 | 1,56 |
| YOR317W | Hexadeca-dienoic acid | GC | 1,19 | 1,69 |
| YGL205W | Hexadecatrienoic Acid (C16:3) | GC | 1,14 | 1,16 |
| YIL150C | Hexadecatrienoic Acid (C16:3) | GC | 3,24 | 3,24 |
| YKL132C | Hexadecatrienoic Acid (C16:3) | GC | 1,13 | 1,56 |
| YOR344C | Hexadecatrienoic Acid (C16:3) | GC | 1,12 | 1,20 |
| b3430 | Hexadecatrienoic Acid (C16:3) | GC | 1,12 | 1,20 |
| b0057 | C24:1 fatty acid | GC | 1.22 | 1.37 |
| b0161 | 2-Hydroxy-palmitic acid | GC | 1.21 | 1.48 |
| b0758 | 2-Hydroxy-palmitic acid | GC | 1.19 | 1.38 |
| b1097 | C24:1 fatty acid | GC | 1.23 | 1.41 |
| b2078 | C24:1 fatty acid | GC | 1.23 | 1.41 |
| b3231 | C24:1 fatty acid | GC | 1.23 | 1.48 |

**[0515.0.15.15]** Column 2 shows the fatty acid analyzed. Columns 4 and 5 shows the ratio of the analyzed fatty acid between the transgenic plants and the wild type; Increase of the metabolites: Max: maximal x-fold (normalised to wild type)-Min: minimal x-fold (normalised to wild type). Decrease of the metabolites: Max: maximal x-fold (normalised to wild type) (minimal decrease), Min: minimal x-fold (normalised to wild type) (maximal decrease). Column 3 indicates the analytical method.

**[0516.0.0.15] see [0516.0.0.0]**

**[0517.0.15.15]**Example 14a: Engineering ryegrass plants by over-expressing YOR344C from Saccharomyces cerevisiae or homologs of YOR344C from other organisms.

**[0518.0.0.15] to [0524.0.0.15] see [0518.0.0.0] to [0524.0.0.0]**

**[0525.0.15.15]**Example 14b: Engineering soybean plants by over-expressing YOR344C from Saccharomyces cerevisiae or homologs of YOR344C from other organisms.

**[0526.0.0.15] to [0529.0.0.15] see [0526.0.0.0] to [0529.0.0.0]**

**[0530.0.15.15]** Example 14c: Engineering corn plants by over-expressing YOR344C from Saccharomyces cerevisiae or homologs of YOR344C from other organisms.

**[0531.0.0.15] to [0533.0.0.15] see [0531.0.0.0] to [0533.0.0.0]**

**[0534.0.15.15]**Example 14d: Engineering wheat plants by over-expressing YOR344C from Saccharomyces cerevisiae or homologs of YOR344C from other organisms.

**[0535.0.0.15] to [0537.0.0.15] see [0535.0.0.0] to [0537.0.0.0]**

**[0538.0.15.15]**Example 14e: Engineering Rapeseed/Canola plants by over-expressing YOR344C from Saccharomyces cerevisiae or homologs of YOR344C from other organisms.

**[0539.0.0.15] to [0542.0.0.15] see [0539.0.0.0] to [0542.0.0.0]**

**[0543.0.15.15]**Example 14f: Engineering alfalfa plants by over-expressing YOR344C from Saccharomyces cerevisiae or homologs of YOR344C from other organisms.

**[0544.0.0.15] to [0547.0.0.15] see [0544.0.0.0] to [0547.0.0.0]**

**[0548.0.15.15]** Example 14g: Engineering alfalfa plants by over-expressing YOR344C from Saccharomyces cerevisiae or homologs of YOR344C from other organisms.

**[0549.0.0.15] to [0552.0.0.15] see [0549.0.0.0] to [0552.0.0.0]**

**[0552.1.0.15]:** Example 15: Metabolite Profiling Info from *Zea mays*
*Zea mays* plants were engineered as described in Example 14c.
The results of the different *Zea mays* plants analysed can be seen from table 2 which follows:

**Table 2**

| ORF | Metabolite | Min | Max |
|---|---|---|---|
| b1829 | Heptadecanoic acid (C17:0) | 1.65 | 1.66 |

Table 2 describes the increase in heptadecanoic acid (C17:0) in genetically modified corn plants expressing the Escherichia coli K12 nucleic acid sequence b1829.
In case the activity of the Escherichia coli K12 protein b1829 or a protein with an activity being defined as a heat shock protein or its homolog, is increased in corn plants, preferably, an increase of the fine chemical heptadecanoic acid (C17:0) between 65% and 66% is conferred.

**[00552.2.0.15] see [00552.2.0.0]**

**[0553.0.15.15]**
1. A process for the production of heptadecanoic acid and/or 2-hydroxy palmitic acid and/or 2-hydroxy-tetracosenoic-acid and/or hexadecadienoic acid, preferably delta 7, 10 hexadecadienoic acid and/or hexadecatrienoic acid, preferably delta 7, 10 ,13 hexadecatrienoic acid and/or 24:1 fatty acid, preferably C24:1 delta 15 fatty acid, which comprises
   (a) increasing or generating the activity of a protein as indicated in Table IIA or IIB, columns 5 or 7, lines 174 to 177 and/or line 178 and/or line 179 and/or line180 and/or lines 181 to 185 and/or lines 558 to 559 and/or 560 to 563 resp., or a functional equivalent thereof in a non-human organism, or in one or more parts thereof; and
   (b) growing the organism under conditions which permit the production of heptadecanoic acid and/or 2-hydroxy palmitic acid and/or 2-hydroxy-tetracosenoic-acid and/or hexadecadienoic acid, preferably delta 7, 10 hexadecadienoic acid and/or hexadecatrienoic acid, preferably delta 7, 10, 13 hexadecatrienoic acid and/or 24:1 fatty acid, preferably C24:1 delta 15 fatty acid in said organism.
2. A process for the production of heptadecanoic acid and/or 2-hydroxy palmitic acid and/or 2-hydroxy-tetracosenoic-acid and/or hexadecadienoic acid, preferably delta 7, 10 hexadecadienoic acid and/or hexadecatrienoic acid, preferably delta 7, 10 ,13 hexadecatrienoic acid and/or 24:1 fatty acid, preferably C24:1 delta 15 fatty acid, comprising the increasing or generating in an organism or a part thereof the expression of at least one nucleic acid molecule comprising a nucleic acid molecule selected from the group consisting of:
   a) nucleic acid molecule encoding of the polypeptide shown in table IIA or IIB, columns 5 or 7, lines 174 to 177 and/or line 178 and/or line 179 and/or line180 and/or lines 181 to 185 and/or lines 558 to 559 and/or 560 to 563 resp., or a fragment thereof, which confers an increase in the amount of heptadecanoic acid and/or 2-hydroxy palmitic acid and/or 2-hydroxy-tetracosenoic-acid and/or hexadecadienoic acid, preferably delta 7, 10 hexadecadienoic acid and/or hexadecatrienoic acid, preferably delta 7, 10 ,13 hexadecatrienoic acid and/or 24:1 fatty acid, preferably C24:1 delta 15 fatty acid in an organism or a part thereof;
   b) nucleic acid molecule comprising of the nucleic acid molecule shown in table IA or IB, columns 5 or 7, lines 174 to 177 and/or line 178 and/or line 179 and/or line180 and/or lines 181 to 185 and/or lines 558 to 559 and/or 560 to 563 resp;
   c) nucleic acid molecule whose sequence can be deduced from a polypeptide sequence encoded by a nucleic acid molecule of (a) or (b) as a result of the degeneracy of the genetic code and conferring an increase in the amount of heptadecanoic acid and/or 2-hydroxy palmitic acid and/or 2-hydroxy-tetracosenoic-acid and/or hexadecadienoic acid, preferably delta 7, 10 hexadecadienoic acid and/or hexadecatrienoic acid, preferably delta 7, 10 ,13 hexadecatrienoic acid and/or 24:1 fatty acid, preferably C24:1 delta 15 fatty acid in an organism or a part thereof;
   d) nucleic acid molecule which encodes a polypeptide which has at least 50% identity with the amino acid sequence of the polypeptide encoded by the nucleic acid molecule of (a) to (c) and conferring an increase in the amount of heptadecanoic acid and/or 2-hydroxy palmitic acid and/or 2-hydroxy-tetracosenoic-acid and/or hexadecadienoic acid, preferably delta 7, 10 hexadecadienoic acid and/or hexadecatrienoic acid, preferably delta 7, 10 ,13 hexadecatrienoic acid and/or 24:1 fatty acid, preferably C24:1 delta 15 fatty acid in an organism or a part thereof;
   e) nucleic acid molecule which hybidizes with a nucleic acid molecule of (a) to (c) under under stringent hybridisation conditions and conferring an increase in the amount of heptadecanoic acid and/or 2-hydroxy palmitic acid and/or 2-hydroxy-tetracosenoic-acid and/or hexadecadienoic acid, preferably delta 7, 10 hexadecadienoic acid and/or hexadecatrienoic acid, preferably delta 7, 10 ,13 hexadecatrienoic acid and/or 24:1 fatty acid, preferably C24:1 delta 15 fatty acid in an organism or a part thereof;
   f) nucleic acid molecule which encompasses a nucleic acid molecule which is obtained by amplifying nucleic acid molecules from a cDNA library or a genomic library using the primers as shown in table III, column 7, lines 174 to 177 and/or line 178 and/or line 179 and/or line180 and/or lines 181 to 185 and/or lines 558 to 559 and/or 560 to 563 resp., and conferring an increase in the amount of the fine chemical in an organism or a part thereof;
   g) nucleic acid molecule encoding a polypeptide which is isolated with the aid of monoclonal antibodies against a polypeptide encoded by one of the nucleic acid molecules of (a) to (f) and conferring an increase in the amount of heptadecanoic acid and/or 2-hydroxy palmitic acid and/or 2-hydroxy-tetracosenoic-acid and/or hexadecadienoic acid, preferably delta 7, 10 hexadecadienoic acid and/or hexadecatrienoic acid, preferably delta 7, 10 ,13 hexadecatrienoic acid and/or 24:1 fatty acid, preferably C24:1 delta 15 fatty acid in an organism or a part thereof;
   h) nucleic acid molecule encoding a polypeptide comprising the consensus sequence shown in table IV, column 7, lines 174 to 177 and/or line 179 and/or line180 and/or lines 181 to 185 and/or lines 558 to 559 and/or 560 to 563 resp., and conferring an increase in the amount of the fine chemical in an organism or a part thereof; and
   i) nucleic acid molecule which is obtainable by screening a suitable nucleic acid library under stringent hybridization conditions with a probe comprising one of the sequences of the nucleic acid molecule of (a) to (k) or with a fragment thereof having at least 15 nt, preferably 20 nt, 30 nt, 50 nt, 100 nt, 200 nt or 500 nt of the nucleic acid molecule characterized in (a) to (k) and conferring an increase in the amount of the fine chemical in an organism or a part thereof.
   or comprising a sequence which is complementary thereto.
3. The process of claim 1 or 2, comprising recovering of the free or bound heptadecanoic acid and/or 2-hydroxy palmitic acid and/or 2-hydroxy-tetracosenoic-acid and/or hexadecadienoic acid, preferably delta 7, 10 hexadecadienoic acid and/or hexadecatrienoic acid, preferably delta 7, 10 ,13 hexadecatrienoic acid and/or 24:1 fatty acid, preferably C24:1 delta 15 fatty acid.
4. The process of any one of claim 1 to 3, comprising the following steps:
   (a) selecting an organism or a part thereof expressing a polypeptide encoded by the nucleic acid molecule characterized in claim 2;
   (b) mutagenizing the selected organism or the part thereof;
   (c) comparing the activity or the expression level of said polypeptide in the mutagenized organism or the part thereof with the activity or the expression of said polypeptide of the selected organisms or the part thereof;
   (d) selecting the mutated organisms or parts thereof, which comprise an increased activity or expression level of said polypeptide compared to the selected organism or the part thereof;
   (e) optionally, growing and cultivating the organisms or the parts thereof; and
   (f) recovering, and optionally isolating, the free or bound heptadecanoic acid and/or 2-hydroxy palmitic acid and/or 2-hydroxy-tetracosenoic-acid and/or hexadecadienoic acid, preferably delta 7, 10 hexadecadienoic acid and/or hexadecatrienoic acid, preferably delta 7, 10 ,13 hexadecatrienoic acid and/or 24:1 fatty acid, preferably C24:1 delta 15 fatty acid produced by the selected mutated organisms or parts thereof.
5. The process of any one of claims 1 to 4, wherein the activity of said protein or the expression of said nucleic acid molecule is increased or generated transiently or stably.
6. An isolated nucleic acid molecule comprising a nucleic acid molecule selected from the group consisting of:
   a) nucleic acid molecule encoding of the polypeptide shown in table IIA or IIB, columns 5 or 7, lines 174 to 177 and/or line 178 and/or line 179 and/or line180 and/or lines 181 to 185 and/or lines 558 to 559 and/or 560 to 563 resp., or a fragment thereof, which confers an increase in the amount of heptadecanoic acid and/or 2-hydroxy palmitic acid and/or 2-hydroxy-tetracosenoic-acid and/or hexadecadienoic acid, preferably delta 7, 10 hexadecadienoic acid and/or hexadecatrienoic acid, preferably delta 7, 10 ,13 hexadecatrienoic acid and/or 24:1 fatty acid, preferably C24:1 delta 15 fatty acid in an organism or a part thereof;
   b) nucleic acid molecule comprising of the nucleic acid molecule shown in table IA or IB, columns 5 or 7, lines 174 to 177 and/or line 178 and/or line 179 and/or line180 and/or lines 181 to 185 and/or lines 558 to 559 and/or 560 to 563 resp.;
   c) nucleic acid molecule whose sequence can be deduced from a polypeptide sequence encoded by a nucleic acid molecule of (a) or (b) as a result of the degeneracy of the genetic code and conferring an increase in the amount of heptadecanoic acid and/or 2-hydroxy palmitic acid and/or 2-hydroxy-tetracosenoic-acid and/or hexadecadienoic acid, preferably delta 7, 10 hexadecadienoic acid and/or hexadecatrienoic acid, preferably delta 7, 10 ,13 hexadecatrienoic acid and/or 24:1 fatty acid, preferably C24:1 delta 15 fatty acid in an organism or a part thereof;
   d) nucleic acid molecule which encodes a polypeptide which has at least 50% identity with the amino acid sequence of the polypeptide encoded by the nucleic acid molecule of (a) to (c) and conferring an increase in the amount of heptadecanoic acid and/or 2-hydroxy palmitic acid and/or 2-hydroxy-tetracosenoic-acid and/or hexadecadienoic acid, preferably delta 7, 10 hexadecadienoic acid and/or hexadecatrienoic acid, preferably delta 7, 10, 13 hexadecatrienoic acid in an organism or a part thereof;
   e) nucleic acid molecule which hybidizes with a nucleic acid molecule of (a) to (c) under stringent hybridisation conditions and conferring an increase in the amount of heptadecanoic acid and/or 2-hydroxy palmitic acid and/or 2-hydroxy-tetracosenoic-acid and/or hexadecadienoic acid, preferably delta 7, 10 hexadecadienoic acid and/or hexadecatrienoic acid, preferably delta 7, 10 ,13 hexadecatrienoic acid and/or 24:1 fatty acid, preferably C24:1 delta 15 fatty acid in an organism or a part thereof;
   f) nucleic acid molecule which encompasses a nucleic acid molecule which is obtained by amplifying nucleic acid molecules from a cDNA library or a genomic library using the primers as shown in table III, column 7, lines 174 to 177 and/or line 178 and/or line 179 and/or line180 and/or lines 181 to 185 and/or lines 558 to 559 and/or 560 to 563 resp.,and conferring an increase in the amount of the fine chemical in an organism or a part thereof;
   g) nucleic acid molecule encoding a polypeptide which is isolated with the aid of monoclonal antibodies against a polypeptide encoded by one of the nucleic acid molecules of (a) to (f) and conferring an increase in the amount of heptadecanoic acid and/or 2-hydroxy palmitic acid and/or 2-hydroxy-tetracosenoic-acid and/or hexadecadienoic acid, preferably delta 7, 10 hexadecadienoic acid and/or hexadecatrienoic acid, preferably delta 7, 10 ,13 hexadecatrienoic acid and/or 24:1 fatty acid, preferably C24:1 delta 15 fatty acid in an organism or a part thereof;
   h) nucleic acid molecule encoding a polypeptide comprising the consensus sequence shown in table IV, column 7, lines 174 to 177 and/or line 179 and/or line180 and/or lines 181 to 185 and/or lines 558 to 559 and/or 560 to 563 resp., and conferring an increase in the amount of the fine chemical in an organism or a part thereof; and
   i) nucleic acid molecule which is obtainable by screening a suitable nucleic acid library under stringent hybridization conditions with a probe comprising one of the sequences of the nucleic acid molecule of (a) to (k) or with a fragment thereof having at least 15 nt, preferably 20 nt, 30 nt, 50 nt, 100 nt, 200 nt or 500 nt of the nucleic acid molecule characterized in (a) to (k) and conferring an increase in the amount of the fine chemical in an organism or a part thereof.
   whereby the nucleic acid molecule distinguishes over the sequence as shown in table I A, columns 5 or 7, lines 174 to 177 and/or line 178 and/or line 179 and/or line180 and/or lines 181 to 185 and/or lines 558 to 563 resp., by one or more nucleotides.
7. A nucleic acid construct which confers the expression of the nucleic acid molecule of claim 6, comprising one or more regulatory elements.
8. A vector comprising the nucleic acid molecule as claimed in claim 6 or the nucleic acid construct of claim 7.
9. The vector as claimed in claim 8, wherein the nucleic acid molecule is in operable linkage with regulatory sequences for the expression in a prokaryotic or eukaryotic, or in a prokaryotic and eukaryotic, host.
10. A host cell, which has been transformed stably or transiently with the vector as claimed in claim 8 or 9 or the nucleic acid molecule as claimed in claim 6 or the nucleic acid construct of claim 7 or produced as described in claim any one of claims 2 to 5.
11. The host cell of claim 10, which is a transgenic host cell.
12. The host cell of claim 10 or 11, which is a plant cell, an animal cell, a microorganism, or a yeast cell, a fungus cell, a prokaryotic cell, an eukaryotic cell or an archaebacterium.
13. A process for producing a polypeptide, wherein the polypeptide is expressed in a host cell as claimed in any one of claims 10 to 12.
14. A polypeptide produced by the process as claimed in claim 13 or encoded by the nucleic acid molecule as claimed in claim 6 whereby the polypeptide distinguishes over the sequence as shown in table II A, columns 5 or 7, lines 174 to 177 and/or line 178 and/or line 179 and/or line180 and/or lines 181 to 185 and/or lines 558 to 563 resp., by one or more amino acids
15. An antibody, which binds specifically to the polypeptide as claimed in claim 14.
16. A plant tissue, propagation material, harvested material or a plant comprising the host cell as claimed in claim 12 which is plant cell or an Agrobacterium.
17. A method for screening for agonists and antagonists of the activity of a polypeptide encoded by the nucleic acid molecule of claim 6 conferring an increase in the amount of heptadecanoic acid and/or 2-hydroxy palmitic acid and/or 2-hydroxy-tetracosenoic-acid and/or hexadecadienoic acid, preferably delta 7, 10 hexadecadienoic acid and/or hexadecatrienoic acid, preferably delta 7, 10 ,13 hexadecatrienoic acid and/or 24:1 fatty acid, preferably C24:1 delta 15 fatty acid in an organism or a part thereof comprising:
   (a) contacting cells, tissues , plants or microorganisms which express the a polypeptide encoded by the nucleic acid molecule of claim 6 conferring an increase in the amount of heptadecanoic acid and/or 2-hydroxy palmitic acid and/or 2-hydroxy-tetracosenoic-acid and/or hexadecadienoic acid, preferably delta 7, 10 hexadecadienoic acid and/or hexadecatrienoic acid, preferably delta 7, 10 ,13 hexadecatrienoic acid and/or 24:1 fatty acid, preferably C24:1 delta 15 fatty acid in an organism or a part thereof with a candidate compound or a sample comprising a plurality of compounds under conditions which permit the expression the polypeptide;
   (b) assaying the palmitic acid level or the polypeptide expression level in the cell, tissue, plant or microorganism or the media the cell, tissue, plant or microorganisms is cultured or maintained in; and
   (c) identifying a agonist or antagonist by comparing the measured palmitic acid level or polypeptide expression level with a standard palmitic acid or polypeptide expression level measured in the absence of said candidate compound or a sample comprising said plurality of compounds, whereby an increased level over the standard indicates that the compound or the sample comprising said plurality of compounds is an agonist and a decreased level over the standard indicates that the compound or the sample comprising said plurality of compounds is an antagonist.
18. A process for the identification of a compound conferring increased heptadecanoic acid and/or 2-hydroxy palmitic acid and/or 2-hydroxy-tetracosenoic-acid and/or hexadecadienoic acid, preferably delta 7, 10 hexadecadienoic acid and/or hexadecatrienoic acid, preferably delta 7, 10 ,13 hexadecatrienoic acid and/or 24:1 fatty acid, preferably C24:1 delta 15 fatty acid production in a plant or microorganism, comprising the steps:
   (a) culturing a plant cell or tissue or microorganism or maintaining a plant expressing the polypeptide encoded by the nucleic acid molecule of claim 6 conferring an increase in the amount of heptadecanoic acid and/or 2-hydroxy palmitic acid and/or 2-hydroxy-tetracosenoic-acid and/or hexadecadienoic acid, preferably delta 7, 10 hexadecadienoic acid and/or hexadecatrienoic acid, preferably delta 7, 10 ,13 hexadecatrienoic acid and/or 24:1 fatty acid, preferably C24:1 delta 15 fatty acid in an organism or a part thereof and a readout system capable of interacting with the polypeptide under suitable conditions which permit the interaction of the polypeptide with dais readout system in the presence of a compound or a sample comprising a plurality of compounds and capable of providing a detectable signal in response to the binding of a compound to said polypeptide under conditions which permit the expression of said readout system and of the polypeptide encoded by the nucleic acid molecule of claim 6 conferring an increase in the amount of heptadecanoic acid and/or 2-hydroxy palmitic acid and/or 2-hydroxy-tetracosenoic-acid and/or hexadecadienoic acid, preferably delta 7, 10 hexadecadienoic acid and/or hexadecatrienoic acid, preferably delta 7, 10 ,13 hexadecatrienoic acid and/or 24:1 fatty acid, preferably C24:1 delta 15 fatty acid in an organism or a part thereof;
   (b) identifying if the compound is an effective agonist by detecting the presence or absence or increase of a signal produced by said readout system.
19. A method for the identification of a gene product conferring an increase in heptadecanoic acid and/or 2-hydroxy palmitic acid and/or 2-hydroxy-tetracosenoic-acid and/or hexadecadienoic acid, preferably delta 7, 10 hexadecadienoic acid and/or hexadecatrienoic acid, preferably delta 7, 10 ,13 hexadecatrienoic acid and/or 24:1 fatty acid, preferably C24:1 delta 15 fatty acid production in a cell, comprising the following steps:
   (a) contacting the nucleic acid molecules of a sample, which can contain a candidate gene encoding a gene product conferring an increase in heptadecanoic acid and/or 2-hydroxy palmitic acid and/or 2-hydroxy-tetracosenoic-acid and/or hexadecadienoic acid, preferably delta 7, 10 hexadecadienoic acid and/or hexadecatrienoic acid, preferably delta 7, 10 ,13 hexadecatrienoic acid and/or 24:1 fatty acid, preferably C24:1 delta 15 fatty acid after expression with the nucleic acid molecule of claim 6;
   (b) identifying the nucleic acid molecules, which hybridise under relaxed stringent conditions with the nucleic acid molecule of claim 6;
   (c) introducing the candidate nucleic acid molecules in host cells appropriate for producing heptadecanoic acid and/or 2-hydroxy palmitic acid and/or 2-hydroxy-tetracosenoic-acid and/or hexadecadienoic acid, preferably delta 7, 10 hexadecadienoic acid and/or hexadecatrienoic acid, preferably delta 7, 10 ,13 hexadecatrienoic acid and/or 24:1 fatty acid, preferably C24:1 delta 15 fatty acid;
   (d) expressing the identified nucleic acid molecules in the host cells;
   (e) assaying the heptadecanoic acid and/or 2-hydroxy palmitic acid and/or 2-hydroxy-tetracosenoic-acid and/or hexadecadienoic acid, preferably delta 7, 10 hexadecadienoic acid and/or hexadecatrienoic acid, preferably delta 7, 10 ,13 hexadecatrienoic acid level and/or 24:1 fatty acid, preferably C24:1 delta 15 fatty acid in the host cells; and
   (f) identifying nucleic acid molecule and its gene product which expression confers an increase in the heptadecanoic acid and/or 2-hydroxy palmitic acid and/or 2-hydroxy-tetracosenoic-acid and/or hexadecadienoic acid, preferably delta 7, 10 hexadecadienoic acid and/or hexadecatrienoic acid, preferably delta 7, 10 ,13 hexadecatrienoic acid and/or 24:1 fatty acid, preferably C24:1 delta 15 fatty acid level in the host cell in the host cell after expression compared to the wild type.
20. A method for the identification of a gene product conferring an increase in heptadecanoic acid and/or 2-hydroxy palmitic acid and/or 2-hydroxy-tetracosenoic-acid and/or hexadecadienoic acid, preferably delta 7, 10 hexadecadienoic acid and/or hexadecatrienoic acid, preferably delta 7, 10 ,13 hexadecatrienoic acid and/or 24:1 fatty acid, preferably C24:1 delta 15 fatty acid production in a cell, comprising the following steps:
   (a) identifiying in a data bank nucleic acid molecules of an organism; which can contain a candidate gene encoding a gene product conferring an increase in the heptadecanoic acid and/or 2-hydroxy palmitic acid and/or 2-hydroxy-tetracosenoic-acid and/or hexadecadienoic acid, preferably delta 7, 10 hexadecadienoic acid and/or hexadecatrienoic acid, preferably delta 7, 10, 13 hexadecatrienoic acid and/or 24:1 fatty acid, preferably C24:1 delta 15 fatty acid amount or level in an organism or a part thereof after expression, and which are at least 20% homolog to the nucleic acid molecule of claim 6;
   (b) introducing the candidate nucleic acid molecules in host cells appropriate for producing heptadecanoic acid and/or 2-hydroxy palmitic acid and/or 2-hydroxy-tetracosenoic-acid and/or hexadecadienoic acid, preferably delta 7, 10 hexadecadienoic acid and/or hexadecatrienoic acid, preferably delta 7, 10 ,13 hexadecatrienoic acid and/or 24:1 fatty acid, preferably C24:1 delta 15 fatty acid;
   (c) expressing the identified nucleic acid molecules in the host cells;
   (d) assaying the heptadecanoic acid and/or 2-hydroxy palmitic acid and/or 2-hydroxy-tetracosenoic-acid and/or hexadecadienoic acid, preferably delta 7, 10 hexadecadienoic acid and/or hexadecatrienoic acid, preferably delta 7, 10 ,13 hexadecatrienoic acid and/or 24:1 fatty acid, preferably C24:1 delta 15 fatty acid level in the host cells; and
   (e) identifying nucleic acid molecule and its gene product which expression confers an increase in the heptadecanoic acid and/or 2-hydroxy palmitic acid and/or 2-hydroxy-tetracosenoic-acid and/or hexadecadienoic acid, preferably delta 7, 10 hexadecadienoic acid and/or hexadecatrienoic acid, preferably delta 7, 10 ,13 hexadecatrienoic acid and/or 24:1 fatty acid, preferably C24:1 delta 15 fatty acid level in the host cell after expression compared to the wild type.
21. A method for the production of an agricultural composition comprising the steps of the method of any one of claims 17 to 20 and formulating the compound identified in any one of claims 17 to 20 in a form acceptable for an application in agriculture.
22. A composition comprising the nucleic acid molecule of claim 6, the polypeptide of claim 14, the nucleic acid construct of claim 7, the vector of any one of claims 8 or 9, an antagonist or agonist identified according to claim 17, the compound of claim 18, the gene product of claim 19 or 20, the antibody of claim 15, and optionally an agricultural acceptable carrier.
23. Use of the nucleic acid molecule as claimed in claim 6 for the identification of a nucleic acid molecule conferring an increase of heptadecanoic acid and/or 2-hydroxy palmitic acid and/or 2-hydroxy-tetracosenoic-acid and/or hexadecadienoic acid, preferably delta 7, 10 hexadecadienoic acid and/or hexadecatrienoic acid, preferably delta 7, 10 ,13 hexadecatrienoic acid and/or 24:1 fatty acid, preferably C24:1 delta 15 fatty acid after expression.
24. Use of the polypeptide of claim 14 or the nucleic acid construct claim 7 or the gene product identified according to the method of claim 19 or 20 for identifying compounds capable of conferring a modulation of heptadecanoic acid and/or 2-hydroxy palmitic acid and/or 2-hydroxy-tetracosenoic-acid and/or hexadecadienoic acid, preferably delta 7, 10 hexadecadienoic acid and/or hexadecatrienoic acid, preferably delta 7, 10 ,13 hexadecatrienoic acid and/or 24:1 fatty acid, preferably C24:1 delta 15 fatty acid levels in an organism.
25. Food or feed composition comprising the nucleic acid molecule of claim 6, the polypeptide of claim 14, the nucleic acid construct of claim 7, the vector of claim 8 or 9, the antagonist or agonist identified according to claim 17, the antibody of claim 15, the plant or plant tissue of claim 16, the harvested material of claim 16, the host cell of claim 10 to 12 or the gene product identified according to the method of claim 19 or 20.
26. Use of the nucleic acid molecule of claim 6, the polypeptide of claim 14, the nucleic acid construct of claim 7, the vector of claim 8 or 9, the antagonist or agonist identified according to claim 17, the antibody of claim 15, the plant or plant tissue of claim 16, the harvested material of claim 16, the host cell of claim 10 to 12 or the gene product identified according to the method of claim 19 or 20 for the protection of a plant against a heptadecanoic acid and/or 2-hydroxy palmitic acid and/or 2-hydroxy-tetracosenoic-acid and/or hexadecadienoic acid, preferably delta 7, 10 hexadecadienoic acid and/or hexadecatrienoic acid, preferably delta 7, 10 ,13 hexadecatrienoic acid and/or 24:1 fatty acid, preferably C24:1 delta 15 fatty acid synthesis inhibiting herbicide.

**[0554.0.0.15] Abstract: see [0554.0.0.0]**

**[0000.0.0.16]** In a further embodiment, the present invention relates to a further process for the production of fine chemicals as defined below and the corresponding embodiments as described herein as follows.

### Process for the production of fine chemicals

### Description

**[0001.0.0.16] see [0001.0.0.0]**

**[0002.0.16.16]** The discovery in *Arabidopsis* of citramalic acid (Fiehn et al. 2000 Nature Biotechnology 18, 1157-1161) a potential precursor of pyruvic acid and acetate suggests a novel aspect of carbon metabolism and furthermore suggests the existence of a tricarboxylic acid cycle bypass previously found only in bacteria.
The malic acid oxaloacetate shuttle is characteristic for plant cells. It transports redox equivalents intracellularly.
Malic acid is not only a central metabolite in intermediary flow of carbon in organisms. In higher plants, vacuolar malic acid accumulation, and hence, transtonoplast malic acid transport, also plays a paramount role in many physiological functions. These include adjustment of osmotic and turgor potentials in extension growth and movements of stomata and pulvini, pH-regulation, e.g. during nitrate reduction, and others (for review, see Luttge et al, Plant Physiol, 124(2000), 1335-1348).
Osawa and Matsumoto, Plant Physiol, 126(2001), 411-420 discuss the involvement malic acid in aluminium resistance in plants.
Malic acid is a common constituent of all plants, and its formation is controlled by an enzyme (protein catalyst) called malic acid dehydrogenase (MDH).
Malic acid occupies a central role in plant metabolism. Its importance in plant mineral nutrition is reflected by the role it plays in symbiotic nitrogen fixation, phosphorus acquisition, and aluminum tolerance.
During phosphorus deficiency, malic acid is frequently secreted from roots to release unavailable forms of phosphorus.
In nitrogen-fixing root nodules, malic acid is the primary substrate for bacteroid respiration, thus fueling nitrogenase.
Pyruvic acid is a naturally occurring component in plants and vegetables and in the body, where it is inherently involved in metabolism, the process whereby energy is produced. Pyruvic acid represents the final step in the metabolism of glucose or starch.
Increased pyruvic acid production in yeast strains is known (WO 04/099425).
Glyceric acid is an improtan precursor in the anabolism of amino acids, in particular for Serin and Glycin. Further, the energy level of a cell may be depend on the level of glyceric acid found. Glycerate and Glycerate-3-phophate form a shuttle for the transportation of energy equivalents, e.g. during photorespiration between glycosomes and peroxisomes.
Succinic acid is an intermediate of the citric acid cycle (and the glyoxylate cycle) produced by action of the enzyme succinvl-CoA synthetase on succinyl-CoA. Succinic acid is converted to fumaric acid by action of the enzyme succinic acid dehydrogenase (with formation of FADH2).
Trihydroxibutanoic acid, trihydroxybutyric acid lacton can be regarded as hypothetic precusors of pheromone like metabolites in plants.
Due to these interesting physiological roles and agrobiotechnological potential of citramalic acid, glyceric acid, fumaric acid, malic acid, pyruvic acid, succinic acid, trihydroxybutyric acid or trihydroxybutanoic acid there is a need to identify the genes of enzymes and other proteins involved in citramalic acid, glyceric acid, fumaric acid, malic acid, pyruvic acid, succinic acid, trihydroxybutyric acid or trihydroxybutanoic acid metabolism, and to generate mutants or transgenic plant lines with which to modify the citramalic acid, glyceric acid, fumaric acid, malic acid, pyruvic acid, succinic acid, trihydroxybutyric acid or trihydroxybutanoic acid content.

**[0003.0.0.16] to [0007.0.0.16]** -/-

**[0008.0.0.16]** One way to increase the productive capacity of biosynthesis is to apply recombinant DNA technology. Thus, it would be desirable to produce glyceric acid, citramalic acid, fumaric acid, malic acid, pyruvic acid, succinic acid, trihydroxybutyric acid or trihydroxybutanoic acid in plants. That type of production permits control over quality, quantity and selection of the most suitable and efficient producer organisms. The latter is especially important for commercial production economics and therefore availability to consumers. In addition it is desirable to produce glyceric acid, citramalic acid, fumaric acid, malic acid, pyruvic acid, succinic acid, trihydroxybutyric acid or trihydroxybutanoic acid in plants in order to increase plant productivity and resistance against biotic and abiotic stress as discussed before.
Methods of recombinant DNA technology have been used for some years to improve the production of fine chemicals in microorganisms and plants by amplifying individual biosynthesis genes and investigating the effect on production of fine chemicals. It is for example reported, that the xanthophyll astaxanthin could be produced in the nectaries of transgenic tobacco plants. Those transgenic plants were prepared by *Agrobacterium tumefaciens*-mediated transformation of tobacco plants using a vector that contained a ketolase-encoding gene from *H. pluvialis* denominated *crtO* along with the *Pds* gene from tomato as the promoter and to encode a leader sequence. Those results indicated that about 75 percent of the carotenoids found in the flower of the transformed plant contained a keto group.

**[0009.0.16.16]** Thus, it would be advantageous if an algae, plant or other microorganism were available which produce large amounts glyceric acid, citramalic acid, fumaric acid, malic acid, pyruvic acid, succinic acid, trihydroxybutyric acid or trihydroxybutanoic acid. The invention discussed hereinafter relates in some embodiments to such transformed prokaryotic or eukaryotic microorganisms.
It would also be advantageous if plants were available whose roots, leaves, stem, fruits or flowers produced large amounts of glyceric acid, citramalic acid, fumaric acid, malic acid, pyruvic acid, succinic acid, trihydroxybutyric acid or trihydroxybutanoic acid. The invention discussed hereinafter relates in some embodiments to such transformed plants.

**[0010.0.16.16]** Therefore improving the quality of foodstuffs and animal feeds is an important task of the food-and-feed industry. This is necessary since, for example glyceric acid, citramalic acid, fumaric acid, malic acid, pyruvic acid, succinic acid, trihydroxybutyric acid or trihydroxybutanoic acid, as mentioned above, which occur in plants and some microorganisms are limited with regard to the supply of mammals. Especially advantageous for the quality of foodstuffs and animal feeds is as balanced as possible a specific glyceric acid, citramalic acid, fumaric acid, malic acid, pyruvic acid, succinic acid, trihydroxybutyric acid or trihydroxybutanoic acid profile in the diet since an excess of glyceric acid, citramalic acid, fumaric acid, malic acid, pyruvic acid, succinic acid, trihydroxybutyric acid or trihydroxybutanoic acid above a specific concentration in the food has a positive effect. A further increase in quality is only possible via addition of further glyceric acid, citramalic acid, fumaric acid, malic acid, pyruvic acid, succinic acid, trihydroxybutyric acid and/or trihydroxybutanoic acid, which are limiting.

**[0011.0.16.16]** To ensure a high quality of foods and animal feeds, it is therefore necessary to add glyceric acid, citramalic acid, fumaric acid, malic acid, pyruvic acid, succinic acid, trihydroxybutyric acid and/or trihydroxybutanoic acid in a balanced manner to suit the organism.

**[0012.0.16.16]** Accordingly, there is still a great demand for new and more suitable genes which encode enzymes or other proteins which participate in the biosynthesis of glyceric acid, citramalic acid, fumaric acid, malic acid, pyruvic acid, succinic acid, trihydroxybutyric acid and/or trihydroxybutanoic acid and make it possible to produce them specifically on an industrial scale without unwanted by-products forming. In the selection of genes for biosynthesis two characteristics above all are particularly important. On the one hand, there is as ever a need for improved processes for obtaining the highest possible contents of glyceric acid, citramalic acid, fumaric acid, malic acid, pyruvic acid, succinic acid, trihydroxybutyric acid and/or trihydroxybutanoic acid; on the other hand as less as possible by-products should be produced in the production process.

**[0013.0.0.16] see [0013.0.0.0]**

**[0014.0.16.16]** Accordingly, in a first embodiment, the invention relates to a process for the production of a fine chemical, whereby the fine chemical is a glyceric acid, citramalic acid, fumaric acid, malic acid, pyruvic acid, succinic acid, trihydroxybutyric acid and/or trihydroxybutanoic acid. Accordingly, in the present invention, the term "the fine chemical" as used herein relates to a glyceric acid, citramalic acid, fumaric acid, malic acid, pyruvic acid, succinic acid, trihydroxybutyric acid and/or trihydroxybutanoic acid. Further, the term "the fine chemicals" as used herein also relates to fine chemicals comprising glyceric acid, citramalic acid, fumaric acid, malic acid, pyruvic acid, succinic acid, trihydroxybutyric acid and/or trihydroxybutanoic acid.

**[0015.0.16.16]** In one embodiment, the term "the fine chemical" or "the respective fine chemical" means at least one chemical compound with glyceric acid, citramalic acid, fumaric acid, malic acid, pyruvic acid, succinic acid, trihydroxybutyric acid and/or trihydroxybutanoic acid activity.
In one embodiment, the term "the fine chemical" or "the respective fine chemical" means a glyceric acid.
In one embodiment, the term "the fine chemical" or "the respective fine chemical" means a citramalic acid. In one embodiment, the term "the fine chemical" or "the respective fine chemical" means fumaric acid. In one embodiment, the term "the fine chemical" or "the respective fine chemical" means malic acid. In one embodiment, the term "the fine chemical" or "the respective fine chemical" means pyruvic acid. In one embodiment, the term "the fine chemical" or "the respective fine chemical" means succinic acid. In one embodiment, the term "the fine chemical" or "the respective fine chemical" means trihydroxybutyric acid. In one embodiment, the term "the fine chemical" or "the respective fine chemical" means trihydroxybutanoic acid depending on the context in which the term is used. Throughout the specification the term "the fine chemical" or "the respective fine chemical" means glyceric acid, citramalic acid, fumaric acid, malic acid, pyruvic acid, succinic acid, trihydroxybutyraic acid and/or trihydroxybutanoic acid, its salts, ester, thioester or in free form or bound to other compounds such sugars or sugar polymers, like glucoside, e.g. diglucoside.
In particular it is known to the skilled that anionic compounds as acids are present in an equilibrium of the acid and its salts according to the pH present in the respective compartment of the cell or organism and the pK of the acid. Thus, the term "the fine chemical", the term "the respective fine chemical", the term "acid" or the use of a demonination referring to a neutralized anionic compound respectivley relates the anionic form as well as the neutralised status of that compound.
Thus, citramalic acid relates also to citramalate, fumaric acid also relates to fumarate, malic acid also relates to malate, pyruvic acid also relates to pyruvate, succinic acid relates to succinate, trihydroxybuyraic acid relates to trihydroxybutyrate or trihydroxybutanoic acid relates also to trihydroxybutanoic.
In one embodiment, the term "the fine chemical" and the term "the respective fine chemical" mean at least one chemical compound with an activity of the above mentioned fine chemical

**[0016.0.16.16]** Accordingly, the present invention relates to a process comprising
(a) increasing or generating the activity of one or more YMR241W, YJL099W, YJL055W, YGR007W, YCR059C, YCL032W, YBR184W, YBR084W, b4139, b1676, b1611, b0695, b0730, b1896, b2699, b4063, YBL015W, YFR007W, YJL072C, YKL132C, YOR044W, YPR024W or YPR138C protein(s) or of a protein having the sequence of a polypeptide encoded by a nucleic acid molecule indicated in Table I, columns 5 or 7, lines 190 to 226 or lines 564 to 594 in a non-human organism in one or more parts thereof; and
(b) growing the organism under conditions which permit the production of the fine chemical, thus glyceric acid, citramalic acid, fumaric acid, malic acid, pyruvic acid, succinic acid, trihydroxybutyric acid and/or trihydroxybutanoic acid in said organism.
Accordingly, the present invention relates to a process comprising.
(a) increasing or generating the activity of one or more proteins having the activity of a protein indicated in Table II, column 3, lines 190 to 226 or lines 564 to 594, resp. or having the sequence of a polypeptide encoded by a nucleic acid molecule indicated in Table I, column 5 or 7, lines 190 to 226 or lines 564 to 594, resp. in a non-human organism in one or more parts thereof; and
growing the organism under conditions which permit the production of the fine chemical, thus, glyceric acid referring to line 572 to 576, citramalic acid referring to line 190 and/or 564, fumaric acid referring to line 191 to 205, and/or 565 to 571, malic acid referring to line 206 to 217 and/or 577 to 583, pyruvic acid referring to line 220 or 584, succinic acid referring to line 221 to 224 and/or 585 to 591, trihydroxybutyric acid referring to line 218 or 219 and/or trihydroxybutanoic acid referring to 225 and 226 and/or 592 to 594, in said organism.

**[0016.1.16.16]** Accordingly, the term "the fine chemical" means "citramalic acid " in relation to all sequences listed in Table I, line 190 and/or 564 or homologs thereof. Accordingly, the term "the fine chemical" means "fumaric acid" in relation to the sequences listed in Table I, line 191 to 205, and/or 565 to 571 ,or homologs thereof. Accordingly, the term "the fine chemical" means "malic acid" in relation to the sequences listed in table I, line 206 to 217 and/or 577 to 583, or homologs thereof. Accordingly, the term "the fine chemical" means "trihydroxybutyric acid" in relation to the sequences listed in Table I, line 218 or 219 or homologs thereof. Accordingly, the term "the fine chemical" means "pyruvic acid" in relation to the sequences listed in Table I, line 220 or 584, or homologs thereof. Accordingly, the term "the fine chemical" means "succinic acid" in relation to the sequences listed in Table I, line 221 to 224 and/or 585 to 591, or homologs thereof and means "trihydroxybutanoic acid" in relation to the sequences listed in Table I, line 225 and 226 and/or 592 to 594, or homologs thereof. Accordingly, the term "the fine chemical" means "glyceric acid" in relation to the sequences listed in Table I, line 572 to 576, or homologs thereof. Accordingly, the term "the fine chemical" can mean "glyceric acid", "citramalic acid ", " fumaric acid", "malic acid", "trihydroxybutyric acid", "pyruvic acid", "succinic acid" or "trihydroxybutanoic acid" owing to circumstances and the context. In order to illustrate that the meaning of the term "the respective fine chemical" means "glyceric acid", "citramalic acid "or "fumaric acid" or "malic acid" or "trihydroxybutyric acid" or "pyruvic acid" or "succinic acid" or "trihydroxybutanoic acid" owing to the sequences listed in the context the term "the respective fine chemical" is also used.
In one embodiment, the method of the present invention confers the increase of the content of more than one of the respective fine chemicals, i.e. of "one or more of the organic aicds: "glyceric acid", "citramalic acid ", " fumaric acid", "malic acid", "trihydroxybutyric acid", "pyruvic acid", "succinic acid" and/or "trihydroxybutanoic acid.

**[0017.0.0.16] to [0018.0.0.16]: see [0017.0.0.0] to [0018.0.0.0]**

**[0019.0.16.16]** Advantageously the process for the production of the respective fine chemical leads to an enhanced production of the respective fine chemical. The terms "enhanced" or "increase" mean at least a 10%, 20%, 30%, 40% or 50%, preferably at least 60%, 70%, 80%, 90% or 100%, more preferably 150%, 200%, 300%, 400% or 500% higher production of the respective fine chemical in comparison to the reference as defined below, e.g. that means in comparison to an organism without the aforementioned modification of the activity of a protein having the activity of a protein indicated in Table II, column 3, lines 190 to 226 or lines 564 to 594 or encoded by nucleic acid molecule indicated in Table I, columns 5 or 7, lines 190 to 226 or lines 564 to 594.

**[0020.0.16.16]** Surprisingly it was found, that the transgenic expression of the Saccharomyces cerevisiae protein as indicated in Table I or II, column 4, e.g. YBL015W, YCR059C, YFR007W, YJL055W, YJL099W, YMR241 W, YPR024W, YPR138C, YBR084W, YBR184W, YCL032W, YGR007W, YJL072C, YKL132C or YOR044W or line 571, 582 to 583, 591 or as indicated in Table I or II, column or the Escherichia coli K12 protein as indicated in Table I or II, column 4, e.g b1896, b0730, b1611, b2699, b4139, b1676, b0695, or b4063 or lines 564 to 570, 572 to 581, 584 to 590, 592 to 594 in Arabidopsis thaliana conferred an increase in the glyceric acid, citramalic acid, fumaric acid, malic acid, pyruvic acid, succinic acid, trihydroxybutyric acid and/or trihydroxybutanoic acid ("the fine chemical" or "the fine respective chemical") content in respect to said proteins and their homologs as wells as the encoding nucleic acid molecules, in particular as indicated in Table II, column 3, lines 190 to 226 or lines 564 to 594 of the transformed plants.

**[0021.0.0.16] see [0021.0.0.0]**

**[0022.0.16.16]** The sequence of b1343 from Escherichia coli K12 has been published in Blattner, Science 277(5331), 1453-1474, 1997, and its activity is being defined as a ATP-dependent RNA helicase, stimulated by 23S rRNA. Accordingly, in one embodiment, the process of the present invention comprises the use of a "ATP-dependent RNA helicase, stimulated by 23S rRNA" from E. coli or its homolog, e.g. as shown herein, for the production of the fine chemical, meaning of trihydroxybutanoic acid and/or compositions containing trihydroxybutanoic acid, in particular for increasing the amount of trihydroxybutanoic acid in free or bound form in an organism or a part thereof, as mentioned. In one embodiment, in the process of the present invention the activity of a protein having an activity in rRNA processing or translation is increased or generated, e.g. from E. coli or a homolog thereof. Accordingly, in one embodiment, in the process of the present invention the activity of a ATP-dependent RNA helicase, stimulated by 23S rRNA or its homolog is increased for the production of the fine chemical, meaning of trihydroxybutanoic acid, in particular for increasing the amount of trihydroxybutanoic acid in free or bound form in an organism or a part thereof, as mentioned.
The sequence of b3160 from Escherichia coli K12 has been published in Blattner et al., Science 277(5331), 1453-1474, 1997, and its activity is being defined as a putative monooxygenase with luciferase-like ATPase activity. Accordingly, in one embodiment, the process of the present invention comprises the use of a putative monooxygenase with luciferase-like ATPase activity from E. coli or its homolog, e.g. as shown herein, for the production of the fine chemical, meaning of succinic acid, in particular for increasing the amount of succinic acid and/or compositions containing succinic acid, preferably succinic acid in free or bound form in an organism or a part thereof, as mentioned. In one embodiment, in the process of the present invention the activity of a putative monooxygenase with luciferase-like ATPase activity is increased or generated, e.g. from E. coli or a homolog thereof.
The sequence of b3231 from Escherichia coli K12 has been published in Blattner et al., Science 277(5331), 1453-1474, 1997, and its activity is being defined as a 50S ribosomal subunit protein L13. Accordingly, in one embodiment, the process of the present invention comprises the use of a 50S ribosomal subunit protein L13 from E. coli or its homolog, e.g. as shown herein, for the production of the fine chemical, meaning of pyruvic acid and/or compositions containing pyruvic acid, in particular for increasing the amount of pyruvic acid, preferably pyruvic acid in free or bound form in an organism or a part thereof, as mentioned. In one embodiment, in the process of the present invention the activity of a 50S ribosomal subunit protein L13 is increased or generated, e.g. from E. coli or a homolog thereof.
The sequence of b3231 from Escherichia coli K12 has been published in Blattner et al., Science 277(5331), 1453-1474, 1997, and its activity is being defined as a 50S ribosomal subunit protein L13. Accordingly, in one embodiment, the process of the present invention comprises the use of a 50S ribosomal subunit protein L13 from E. coli or its homolog, e.g. as shown herein, for the production of the fine chemical, meaning of glyceric acid and/or compositions containing glyceric acid, in particular for increasing the amount of glyceric acid, preferably glyceric acid in free or bound form in an organism or a part thereof, as mentioned. In one embodiment, in the process of the present invention the activity of a 50S ribosomal subunit protein L13 is increased or generated, e.g. from E. coli or a homolog thereof.
The sequence of b3231 from Escherichia coli K12 has been published in Blattner et al., Science 277(5331), 1453-1474, 1997, and its activity is being defined as a 50S ribosomal subunit protein L13. Accordingly, in one embodiment, the process of the present invention comprises the use of a 50S ribosomal subunit protein L13 from E. coli or its homolog, e.g. as shown herein, for the production of pyruvic acid and glyceric acid and/or compositions containing pyruvic acid and glyceric acid, in particular for increasing the amount of pyruvic acid and glyceric acid, preferably pyruvic acid and glyceric acid in free or bound form in an organism or a part thereof, as mentioned. In one embodiment, in the process of the present invention the activity of a 50S ribosomal subunit protein L13 is increased or generated, e.g. from E. coli or a homolog thereof.
The sequence of b1738 from Escherichia coli K12 has been published in Blattner et al., Science 277(5331), 1453-1474, 1997, and its activity is being defined as a PEP-dependent phosphotransferase. Accordingly, in one embodiment, the process of the present invention comprises the use of a PEP-dependent phosphotransferase from E. coli or its homolog, e.g. as shown herein, for the production of the fine chemical, meaning of succinic acid and/or compositions comprising succinic acid, in particular for increasing the amount of succinic acid, preferably succinic acid in free or bound form in an organism or a part thereof, as mentioned. In one embodiment, in the process of the present invention the activity of a PEP-dependent phosphotransferase is increased or generated, e.g. from E. coli or a homolog thereof.
The sequence of b1738 from Escherichia coli K12 has been published in Blattner et al., Science 277(5331), 1453-1474, 1997, and its activity is being defined as a PEP-dependent phosphotransferase. Accordingly, in one embodiment, the process of the present invention comprises the use of a PEP-dependent phosphotransferase from E. coli or its homolog, e.g. as shown herein, for the production of the fine chemical, meaning of fumaric acid and/or compositions containing fumaric acid, in particular for increasing the amount of fumaric acid , preferably fumaric acid in free or bound form in an organism or a part thereof, as mentioned. In one embodiment, in the process of the present invention the activity of a PEP-dependent phosphotransferase is increased or generated, e.g. from E. coli or a homolog thereof.
The sequence of b1738 from Escherichia coli K12 has been published in Blattner et al., Science 277(5331), 1453-1474, 1997, and its activity is being defined as a PEP-dependent phosphotransferase. Accordingly, in one embodiment, the process of the present invention comprises the use of a PEP-dependent phosphotransferase from E. coli or its homolog, e.g. as shown herein, for the production of fumaric acid and succinic acid and/or compositions containing fumaric acid and succinic acid, in particular for increasing the amount of fumaric acid and succinic acid, preferably fumaric acid and succinic acid in free or bound form in an organism or a part thereof, as mentioned. In one embodiment, in the process of the present invention the activity of a PEP-dependent phosphotransferase is increased or generated, e.g. from E. coli or a homolog thereof.
The sequence of b0161 (Accession number NP_414703) from Escherichia coli K12 has been published in Blattner et al., Science 277 (5331), 1453-1474, 1997, and its activity is being defined as a periplasmic serine protease (heat shock protein). Accordingly, in one embodiment, the process of the present invention comprises the use of a periplasmic serine protease from E. coli or its homolog, e.g. as shown herein, for the production of the fine chemical, meaning of fumaric acid and/or compositions containing fumaric acid, in particular for increasing the amount of fumaric acid, preferably fumaric acid in free or bound form in an organism or a part thereof, as mentioned. In one embodiment, in the process of the present invention the activity of a periplasmic serine protease is increased or generated, e.g. from E. coli or a homolog thereof.
The sequence of b0161 (Accession number NP_414703) from Escherichia coli K12 has been published in Blattner et al., Science 277 (5331), 1453-1474, 1997, and its activity is being defined as a periplasmic serine protease (heat shock protein). Accordingly, in one embodiment, the process of the present invention comprises the use of a periplasmic serine protease from E. coli or its homolog, e.g. as shown herein, for the production of the fine chemical, meaning of malic acid and/or compositions containing malic acid, in particular for increasing the amount of malic acid, preferably malic acid in free or bound form in an organism or a part thereof, as mentioned. In one embodiment, in the process of the present invention the activity of a periplasmic serine protease is increased or generated, e.g. from E. coli or a homolog thereof.
The sequence of b0161 (Accession number NP_414703) from Escherichia coli K12 has been published in Blattner et al., Science 277 (5331), 1453-1474, 1997, and its activity is being defined as a periplasmic serine protease (heat shock protein). Accordingly, in one embodiment, the process of the present invention comprises the use of a periplasmic serine protease from E. coli or its homolog, e.g. as shown herein, for the production of malic acid and fumaric acid and/or compositions containing malic acid and fumaric acid, in particular for increasing the amount of malic acid and fumaric acid, preferably malic acid and fumaric acid in free or bound form in an organism or a part thereof, as mentioned. In one embodiment, in the process of the present invention the activity of a periplasmic serine protease is increased or generated, e.g. from E. coli or a homolog thereof.
The sequence of b1693 (Accession number NP_416208) from Escherichia coli K12 has been published in Blattner et al., Science 277 (5331), 1453-1474, 1997, and its activity is being defined as a 3-dehydroquinate dehydratase. Accordingly, in one embodiment, the process of the present invention comprises the use of a 3-dehydroquinate dehydratase protein from E. coli or its homolog, e.g. as shown herein, for the production of the fine chemical, meaning of glyceric acid and/or compositions containing glyceric acid, in particular for increasing the amount of glyceric acid, preferably glyceric acid in free or bound form in an organism or a part thereof, as mentioned. In one embodiment, in the process of the present invention the activity of a 3-dehydroquinate dehydratase protein is increased or generated, e.g. from E. coli or a homolog thereof.
The sequence of b1693 (Accession number NP_416208) from Escherichia coli K12 has been published in Blattner et al., Science 277 (5331), 1453-1474, 1997, and its activity is being defined as a 3-dehydroquinate dehydratase. Accordingly, in one embodiment, the process of the present invention comprises the use of a 3-dehydroquinate dehydratase protein from E. coli or its homolog, e.g. as shown herein, for the production of the fine chemical, meaning of citramalic acid and/or compositions containing citramalic acid, in particular for increasing the amount of citramalic acid, preferably citramalic acid in free or bound form in an organism or a part thereof, as mentioned. In one embodiment, in the process of the present invention the activity of a 3-dehydroquinate dehydratase protein is increased or generated, e.g. from E. coli or a homolog thereof.
The sequence of b1693 (Accession number NP_416208) from Escherichia coli K12 has been published in Blattner et al., Science 277 (5331), 1453-1474, 1997, and its activity is being defined as a 3-dehydroquinate dehydratase. Accordingly, in one embodiment, the process of the present invention comprises the use of a 3-dehydroquinate dehydratase protein from E. coli or its homolog, e.g. as shown herein, for the production of citramalic acid and glyceric acid and/or compositions containing citramalic acid and glyceric acid, in particular for increasing the amount of citramalic acid and glyceric acid, preferably citramalic acid and glyceric acid in free or bound form in an organism or a part thereof, as mentioned. In one embodiment, in the process of the present invention the activity of a 3-dehydroquinate dehydratase protein is increased or generated, e.g. from E. coli or a homolog thereof.
The sequence of b0970 (Accession number NP_415490) from Escherichia coli K12 has been published in Blattner et al., Science 277 (5331), 1453-1474, 1997, and its activity is being defined as a glutamate receptor. Accordingly, in one embodiment, the process of the present invention comprises the use of a gene product with an activity of Escherichia coli ybhL protein superfamily, preferably a protein with the activity of a glutamate receptor protein from E. coli or its homolog, e.g. as shown herein, for the production of the fine chemical, meaning of trihydroxybutanoic acid and/or compositions comprising trihydroxybutanoic acid, in particular for increasing the amount of trihydroxybutanoic acid, preferably trihydroxybutanoic acid in free or bound form in an organism or a part thereof, as mentioned. In one embodiment, in the process of the present invention the activity of a glutamate receptor is increased or generated, e.g. from E. coli or a homolog thereof.
The sequence of b3169 (Accession number NP_417638) from Escherichia coli K12 has been published in Blattner et al., Science 277 (5331), 1453-1474, 1997, and its activity is being defined as a transcription termination-antitermination factor. Accordingly, in one embodiment, the process of the present invention comprises the use of a gene product with an activity of Escherichia coli transcription factor nusA superfamily, preferably a protein with the activity of a transcription termination-antitermination factor from E. coli or its homolog, e.g. as shown herein, for the production of the fine chemical, meaning of malic acid or compositions comprising malic acid, in particular for increasing the amount of malic acid, preferably malic acid in free or bound form in an organism or a part thereof, as mentioned. In one embodiment, in the process of the present invention the activity of a transcription termination-antitermination factor is increased or generated, e.g. from E. coli or a homolog thereof.
The sequence of b3169 (Accession number NP_417638) from Escherichia coli K12 has been published in Blattner et al., Science 277 (5331), 1453-1474, 1997, and its activity is being defined as a transcription termination-antitermination factor. Accordingly, in one embodiment, the process of the present invention comprises the use of a gene product with an activity of Escherichia coli transcription factor nusA superfamily, preferably a protein with the activity of a transcription termination-antitermination factor from E. coli or its homolog, e.g. as shown herein, for the production of the fine chemical, meaning of trihydroxybutanoic acid or compositions comprising trihydroxybutanoic acid, in particular for increasing the amount of trihydroxybutanoic acid, preferably trihydroxybutanoic acid in free or bound form in an organism or a part thereof, as mentioned. In one embodiment, in the process of the present invention the activity of a transcription termination-antitermination factor is increased or generated, e.g. from E. coli or a homolog thereof.
The sequence of b3169 (Accession number NP_417638) from Escherichia coli K12 has been published in Blattner et al., Science 277 (5331), 1453-1474, 1997, and its activity is being defined as a transcription termination-antitermination factor. Accordingly, in one embodiment, the process of the present invention comprises the use of a gene product with an activity of Escherichia coli transcription factor nusA superfamily, preferably a protein with the activity of a transcription termination-antitermination factor from E. coli or its homolog, e.g. as shown herein, for the production of malic acid and trihydroxyutanoic acid or compositions comprising malic acid and trihydroxybutanoic acid , in particular for increasing the amount of malic acid and trihydroxybutanoic acid, preferably malic acid and trihydroxybutanoic acid in free or bound form in an organism or a part thereof, as mentioned. In one embodiment, in the process of the present invention the activity of a transcription termination-antitermination factor is increased or generated, e.g. from E. coli or a homolog thereof.
The sequence of b3116 (Accession number NP_417586) from Escherichia coli K12 has been published in Blattner et al., Science 277 (5331), 1453-1474, 1997, and its activity is being defined as a L-threonine/L-serine permease, anaerobically inducible (HAAAP family). Accordingly, in one embodiment, the process of the present invention comprises the use of a gene product with an activity of threonine-serine permease superfamily, preferably a protein with the activity of a L-threonine/L-serine permease, anaerobically inducible (HAAAP family) from E. coli or its homolog, e.g. as shown herein, for the production of the fine chemical, meaning of succinic acid and/or compositions comprising succinic acid, in particular for increasing the amount of succinic acid, preferably succinic acid in free or bound form in an organism or a part thereof, as mentioned. In one embodiment, in the process of the present invention the activity of a L-threonine/L-serine permease, anaerobically inducible (HAAAP family) is increased or generated, e.g. from E. coli or a homolog thereof.
The sequence of b3116 (Accession number NP_417586) from Escherichia coli K12 has been published in Blattner et al., Science 277 (5331), 1453-1474, 1997, and its activity is being defined as a L-threonine/L-serine permease, anaerobically inducible (HAAAP family). Accordingly, in one embodiment, the process of the present invention comprises the use of a gene product with an activity of threonine-serine permease superfamily, preferably a protein with the activity of a L-threonine/L-serine permease, anaerobically inducible (HAAAP family) from E. coli or its homolog, e.g. as shown herein, for the production of the fine chemical, meaning of fumaric acid and/or compositions comprising fumaric acid, in particular for increasing the amount of fumaric acid , preferably fumaric acid in free or bound form in an organism or a part thereof, as mentioned. In one embodiment, in the process of the present invention the activity of a L-threonine/L-serine permease, anaerobically inducible (HAAAP family) is increased or generated, e.g. from E. coli or a homolog thereof.
succinic acid, in particular for increasing the amount of maleicmalic acid, fumaric acid and/or succinic acid preferably maleicmalic acid, fumaric acid and/or succinic acid in free or bound form in an organism or a part thereof, as mentioned. In one embodiment, in the process of the present invention the activity of a L-threonine/L-serine permease, anaerobically inducible (HAAAP family) is increased or generated, e.g. from E. coli or a homolog thereof.
The sequence of b3116 (Accession number NP_417586) from Escherichia coli K12 has been published in Blattner et al., Science 277 (5331), 1453-1474, 1997, and its activity is being defined as a L-threonine/L-serine permease, anaerobically inducible (HAAAP family). Accordingly, in one embodiment, the process of the present invention comprises the use of a gene product with an activity of threonine-serine permease superfamily, preferably a protein with the activity of a L-threonine/L-serine permease, anaerobically inducible (HAAAP family) from E. coli or its homolog, e.g. as shown herein, for the production of the fine chemical, meaning of malic acid and/or compositions comprising malic acid, in particular for increasing the amount of malic acid, preferably malic acid in free or bound form in an organism or a part thereof, as mentioned. In one embodiment, in the process of the present invention the activity of a L-threonine/L-serine permease, anaerobically inducible (HAAAP family) is increased or generated, e.g. from E. coli or a homolog thereof.
The sequence of b3116 (Accession number NP_417586) from Escherichia coli K12 has been published in Blattner et al., Science 277 (5331), 1453-1474, 1997, and its activity is being defined as a L-threonine/L-serine permease, anaerobically inducible (HAAAP family). Accordingly, in one embodiment, the process of the present invention comprises the use of a gene product with an activity of threonine-serine permease superfamily, preferably a protein with the activity of a L-threonine/L-serine permease, anaerobically inducible (HAAAP family) from E. coli or its homolog, e.g. as shown herein, for the production of the fine chemical, meaning of malic acid, fumaric acid and/or succinic acid and/or compositions comprising malic acid, fumaric acid and/or succinic acid, in particular for increasing the amount of malic acid, fumaric acid and/or succinic acid preferably malic acid, fumaric acid and/or succinic acid in free or bound form in an organism or a part thereof, as mentioned. In one embodiment, in the process of the present invention the activity of a L-threonine/L-serine permease, anaerobically inducible (HAAAP family) is increased or generated, e.g. from E. coli or a homolog thereof.
The sequence of b0057 (Accession number NP_414599) from Escherichia coli K12 has been published in Blattner et al., Science 277 (5331), 1453-1474, 1997, and its activity is not been characterized yet. Accordingly, in one embodiment, the process of the present invention comprises the use of a gene product with an activity of b0057 protein from E. coli or its homolog, e.g. as shown herein, for the production of the fine chemical, meaning of glyceric acid, in particular for increasing the amount of glyceric acid or compositions comprising glyceric acid, preferably glyceric acid in free or bound form in an organism or a part thereof, as mentioned.
The sequence of b4129 (Accession number NP_418553) from Escherichia coli K12 has been published in Blattner et al., Science 277 (5331), 1453-1474, 1997, and its activity is being defined as a lysine tRNA synthetase. Accordingly, in one embodiment, the process of the present invention comprises the use of a lysine tRNA synthetase protein from E. coli or its homolog, e.g. as shown herein, for the production of the fine chemical, meaning of succinic acid and/or compositions containing succinic acid, preferably succinic acid in free or bound form in an organism or a part thereof, as mentioned. In one embodiment, in the process of the present invention the activity of a lysine tRNA synthetase protein is increased or generated, e.g. from E. coli or a homolog thereof. In one embodiment, in the process of the present invention the activity of a lysine tRNA synthetase is increased or generated, e.g. from E. coli or a homolog thereof.
The sequence of b3129 (Accession number NP_417598) from Escherichia coli K12 has been published in Blattner et al., Science 277 (5331), 1453-1474, 1997, and its activity is being defined as a putative protease; htrA suppressor protein. Accordingly, in one embodiment, the process of the present invention comprises the use of a putative protease; htrA suppressor protein from E. coli or its homolog, e.g. as shown herein, for the production of the fine chemical, meaning of glyceric acid and/or compositions containing glyceric acid, in particular for increasing the amount of glyceric acid, preferably glyceric acid in free or bound form in an organism or a part thereof, as mentioned. In one embodiment, in the process of the present invention the activity of a putative protease; htrA suppressor protein is increased or generated, e.g. from E. coli or a homolog thereof.
The sequence of b3938 from Escherichia coli K12 has been published in Blattner et al., Science 277 (5331), 1453-1474, 1997, and its activity is being defined as a transcriptional repressor for methionine biosynthesis. Accordingly, in one embodiment, the process of the present invention comprises the use of a protein of the metJ protein superfamily, in particular of a transcriptional repressor for methionine biosynthesis from E. coli or its homolog, e.g. as shown herein, for the production of the fine chemical, meaning of glyceric acid and/or compositions containing glyceric acid, in particular for increasing the amount of glyceric acid, preferably glyceric acid in free or bound form in an organism or a part thereof, as mentioned. In one embodiment, in the process of the present invention the activity of a transcriptional repressor for methionine biosynthesis is increased or generated, e.g. from E. coli or a homolog thereof.
The sequence of b2478 from Escherichia coli K12 has been published in Blattner, Science 277(5331), 1453-1474, 1997, and its activity is being defined as a dihydrodipicolinate synthase. Accordingly, in one embodiment, the process of the present invention comprises the use of a dihydrodipicolinate synthase from E. coli or its homolog, e.g. as shown herein, for the production of the fine chemical, meaning of malic acid and/or compositions containing malic acid, in particular for increasing the amount of malic acid in free or bound form in an organism or a part thereof, as mentioned. In one embodiment, in the process of the present invention the activity of the superfamily of dihydrodipicolinate synthase, e.g. a protein involved in diaminopimelin acid pathway, biosynthesis of lysine, C-compound and carbohydrate utilization, and/or Entner-Doudoroff pathway, preferably of a dihydrodipicolinate synthase is increased or generated, e.g. from E. coli or a homolog thereof.. Accordingly, in one embodiment, in the process of the present invention the activity of a dihydrodipicolinate synthase or its homolog is increased for the production of the fine chemical, meaning of malic acid, in particular for increasing the amount of malic acid in free or bound form in an organism or a part thereof, as mentioned.
The sequence of b3172 from Escherichia coli K12 has been published in Blattner et al., Science 277 (5331), 1453-1474, 1997, and its activity is being defined as a argininosuccinic acid synthetase. Accordingly, in one embodiment, the process of the present invention comprises the use of a gene product with an activity of a protein of the argininosuccinic acid synthase superfamily, preferably a protein being involved in amino acid biosynthesis, nitrogen and sulfur metabolism, biosynthesis of the glutamate group (proline, hydroxyprolin, arginine, glutamine, glutamate), degradation of amino acids of the glutamate group, nitrogen and sulfur utilization, urea cycle, biosynthesis of polyamines and creatine, biosynthesis of the aspartate family, assimilation of ammonia, and/or biosynthesis of the glutamate group, more preferred with the activity of a argininosuccinic acid synthetase from E. coli or its homolog, e.g. as shown herein, for the production of the fine chemical, meaning of fumaric acid and/or compositions comprising fumaric acid, in particular for increasing the amount of fumaric acid, preferably fumaric acid in free or bound form in an organism or a part thereof, as mentioned. In one embodiment, in the process of the present invention the activity of a argininosuccinic acid synthetase is increased or generated, e.g. from E. coli or a homolog thereof.
The sequence of b3172 from Escherichia coli K12 has been published in Blattner et al., Science 277 (5331), 1453-1474, 1997, and its activity is being defined as a argininosuccinic acid synthetase. Accordingly, in one embodiment, the process of the present invention comprises the use of a gene product with an activity of argininosuccinic acid synthase superfamily, preferably a protein being involved in amino acid biosynthesis, nitrogen and sulfur metabolism, biosynthesis of the glutamate group (proline, hydroxyprolin, arginine, glutamine, glutamate), degradation of amino acids of the glutamate group, nitrogen and sulfur utilization, urea cycle, biosynthesis of polyamines and creatine, biosynthesis of the aspartate family, assimilation of ammonia, and/or biosynthesis of the glutamate group, more preferred with the activity of a argininosuccinic acid synthetase from E. coli or its homolog, e.g. as shown herein, for the production of the fine chemical, meaning of malic acid and/or compositions comprising malic acid, in particular for increasing the amount of malic acid, preferably malic acid in free or bound form in an organism or a part thereof, as mentioned. In one embodiment, in the process of the present invention the activity of a argininosuccinic acid synthetase is increased or generated, e.g. from E. coli or a homolog thereof.
The sequence of b3172 from Escherichia coli K12 has been published in Blattner et al., Science 277 (5331), 1453-1474, 1997, and its activity is being defined as a argininosuccinic acid synthetase. Accordingly, in one embodiment, the process of the present invention comprises the use of a gene product with an activity of argininosuccinic acid synthase superfamily, preferably a protein being involved in amino acid biosynthesis, nitrogen and sulfur metabolism, biosynthesis of the glutamate group (proline, hydroxyprolin, arginine, glutamine, glutamate), degradation of amino acids of the glutamate group, nitrogen and sulfur utilization, urea cycle, biosynthesis of polyamines and creatine, biosynthesis of the aspartate family, assimilation of ammonia, and/or biosynthesis of the glutamate group, more preferred with the activity of a argininosuccinic acid synthetase from E. coli or its homolog, e.g. as shown herein, for the production of the fine chemical, meaning of malic acid and fumaric acid and/or compositions comprising malic acid and fumaric acid, in particular for increasing the amount of malic acid and fumaric acid, preferably malic acid and fumaric acid in free or bound form in an organism or a part thereof, as mentioned. In one embodiment, in the process of the present invention the activity of a argininosuccinic acid synthetase is increased or generated, e.g. from E. coli or a homolog thereof.
The sequence of b1961 from Escherichia coli K12 has been published in Blattner et al., Science 277 (5331), 1453-1474, 1997, and its activity is being defined as a DNA cytosine methylase. Accordingly, in one embodiment, the process of the present invention comprises the use of a gene product with an activity of a protein of "site-specific methyltransferase (cytosine-specific) EcoRII" superfamily, preferably a protein being involved in nucleotide metabolism, metabolism of cyclic and unusual nucleotides, TRANSCRIPTION, DNA repair, cell differentiation, DEVELOPMENT (Systemic), and/or nucleic acid binding, more preferred with the activity of a cytosine methylase from E. coli or its homolog, e.g. as shown herein, for the production of the fine chemical, meaning of succinic acid and/or compositions comprising succinic acid, in particular for increasing the amount of succinic acid, preferably succinic acid in free or bound form, in an organism or a part thereof, as mentioned. In one embodiment, in the process of the present invention the activity of a cytosine methylase is increased or generated, e.g. from E. coli or a homolog thereof.
The sequence of b1961 from Escherichia coli K12 has been published in Blattner et al., Science 277 (5331), 1453-1474, 1997, and its activity is being defined as a DNA cytosine methylase. Accordingly, in one embodiment, the process of the present invention comprises the use of a gene product with an activity of a protein of "site-specific methyltransferase (cytosine-specific) EcoRII" superfamily, preferably a protein being involved in nucleotide metabolism, metabolism of cyclic and unusual nucleotides, TRANSCRIPTION, DNA repair, cell differentiation, DEVELOPMENT (Systemic), and/or nucleic acid binding, more preferred with the activity of a cytosine methylase from E. coli or its homolog, e.g. as shown herein, for the production of the fine chemical, meaning of fumaric acid and/or compositions comprising fumaric acid, in particular for increasing the amount of fumaric acid, preferably fumaric acid in free or bound form, in an organism or a part thereof, as mentioned. In one embodiment, in the process of the present invention the activity of a cytosine methylase is increased or generated, e.g. from E. coli or a homolog thereof.
The sequence of b1961 from Escherichia coli K12 has been published in Blattner et al., Science 277 (5331), 1453-1474, 1997, and its activity is being defined as a DNA cytosine methylase. Accordingly, in one embodiment, the process of the present invention comprises the use of a gene product with an activity of a protein of "site-specific methyltransferase (cytosine-specific) EcoRII" superfamily, preferably a protein being involved in nucleotide metabolism, metabolism of cyclic and unusual nucleotides, TRANSCRIPTION, DNA repair, cell differentiation, DEVELOPMENT (Systemic), and/or nucleic acid binding, more preferred with the activity of a cytosine methylase from E. coli or its homolog, e.g. as shown herein, for the production of the fine chemical, meaning of fumaric acid and succinic acid and/or compositions comprising fumaric acid and succinic acid, in particular for increasing the amount of fumaric acid and succinic acid, preferably fumaric acid and succinic acid in free or bound form, in an organism or a part thereof, as mentioned. In one embodiment, in the process of the present invention the activity of a cytosine methylase is increased or generated, e.g. from E. coli or a homolog thereof.

The sequence of b2599 from Escherichia coli K12 has been published in Blattner et al., Science 277 (5331), 1453-1474, 1997, and its activity is being defined as a bifunctional enzyme: chorismate mutase P (N-terminal) and prephenate dehydratase (C-terminal). Accordingly, in one embodiment, the process of the present invention comprises the use of a gene product with an activity of "pheA bifunctional enzyme, prephenate dehydratase homology"-superfamily, preferably a protein being involved in biosynthesis of the cysteine-aromatic group, amino acid biosynthesis, degradation of amino acids of the cysteine-aromatic group, and/or biosynthesis of vitamins, cofactors, and prosthetic groups, more preferred with the activity of a bifunctional enzyme: chorismate mutase P (N-terminal) and prephenate dehydratase (C-terminal) from E. coli or its homolog, e.g. as shown herein, for the production of the fine chemical, meaning of succinic acid and/or compositions comprising succinic acid, in particular for increasing the amount of succinic acid, preferably succinic acid in free or bound form in an organism or a part thereof, as mentioned. In one embodiment, in the process of the present invention the activity of a bifunctional enzyme: chorismate mutase P (N-terminal) and prephenate dehydratase (C-terminal) is increased or generated, e.g. from E. coli or a homolog thereof.
The sequence of b4122 from Escherichia coli K12 has been published in Blattner et al., Science 277 (5331), 1453-1474, 1997, and its activity is being defined as a fumarase B ( hydratase Class I). Accordingly, in one embodiment, the process of the present invention comprises the use of a gene product with an activity of "iron-dependent hydratase, iron-dependent tartrate dehydratase alpha chain homology "-superfamily, preferably with the activity of a fumarase B ( hydratase Class I) from E. coli or its homolog, e.g. as shown herein, for the production of the fine chemical, meaning of fumaric acid and/or compositions comprising fumaric acid, in particular for increasing the amount of fumaric acid, preferably fumaric acid in free or bound form in an organism or a part thereof, as mentioned. In one embodiment, in the process of the present invention the activity of a fumarase B ( hydratase Class I) is increased or generated, e.g. from E. coli or a homolog thereof.
The sequence of YCL038C from Saccharomyces cerevisiae has been published in Dujon, B. et al., Nature 387 (6632 Suppl), 98-102 (1997), and its activity is being defined as a Autophagy-related protein. Accordingly, in one embodiment, the process of the present invention comprises the use of a gene product being involved in other transport facilitators, preferably with the activity of a Autophagy-related protein from Saccharomyces cerevisiae or its homolog, e.g. as shown herein, for the production of the fine chemical, meaning of succinic acid and/or compositions comprising succinic acid, in particular for increasing the amount of succinic acid, preferably succinic acid in free or bound form in an organism or a part thereof, as mentioned. In one embodiment, in the process of the present invention the activity of a Autophagy-related protein is increased or generated, e.g. from Saccharomyces cerevisiae or a homolog thereof.
The sequence of YCL038C from Saccharomyces cerevisiae has been published in Goffeau,A, Science 274 (5287), 546-547 (1996), Oliver,S.G et al., Nature 357 (6373), 38-46 (1992), and its activity is being defined as a Autophagy-related protein. Accordingly, in one embodiment, the process of the present invention comprises the use of a gene product being involved in other transport facilitators, preferably with the activity of a Autophagy-related protein from Saccharomyces cerevisiae or its homolog, e.g. as shown herein, for the production of the fine chemical, meaning of malic acid and/or compositions comprising malic acid, in particular for increasing the amount of malic acid, preferably malic acid in free or bound form in an organism or a part thereof, as mentioned. In one embodiment, in the process of the present invention the activity of a Autophagy-related protein is increased or generated, e.g. from Saccharomyces cerevisiae or a homolog thereof.
The sequence of YCL038C from Saccharomyces cerevisiae has been published in Dujon, B. et al., Nature 387 (6632 Suppl), 98-102 (1997), and its activity is being defined as a Autophagy-related protein. Accordingly, in one embodiment, the process of the present invention comprises the use of a gene product being involved in other transport facilitators, preferably with the activity of a Autophagy-related protein from Saccharomyces cerevisiae or its homolog, e.g. as shown herein, for the production of the fine chemical, meaning of succinic acid and malic acid and/or compositions comprising succinic acid and malic acid, in particular for increasing the amount of succinic acid and malic acid, preferably succinic acid and malic acid in free or bound form in an organism or a part thereof, as mentioned. In one embodiment, in the process of the present invention the activity of a Autophagy-related protein is increased or generated, e.g. from Saccharomyces cerevisiae or a homolog thereof.
The sequence of YCR012W from Saccharomyces cerevisiae has been published in Goffeau,A., Science 274 (5287), 546-547 (1996), and Dujon, B. et al., Nature 387 (6632 Suppl), 98-102 (1997), and its activity is being defined as a 3-phosphoglycerate kinase. Accordingly, in one embodiment, the process of the present invention comprises the use of a gene product of the phosphoglycerate kinase-superfamily, preferably being involved in C-compound and carbohydrate utilization, glycolysis and gluconeogenesis, sugar, glucoside, polyol and carboxylate catabolism, and/or ENERGY, more preferred with the activity of a 3-phosphoglycerate kinase from Saccharomyces cerevisiae or its homolog, e.g. as shown herein, for the production of the fine chemical, meaning of fumaric acid and/or compositions comprising fumaric acid, in particular for increasing the amount of fumaric acid, preferably fumaric acid in free or bound form in an organism or a part thereof, as mentioned. In one embodiment, in the process of the present invention the activity of a 3-phosphoglycerate kinase is increased or generated, e.g. from Saccharomyces cerevisiae or a homolog thereof.
The sequence of YOR168W from Saccharomyces cerevisiae has been published in Dujon, B. et al., Nature 387 (6632 Suppl), 98-102 (1997), and Goffeau,A, Science 274 (5287), 546-547 (1996), and its activity is being defined as a glutamine-tRNA ligase. Accordingly, in one embodiment, the process of the present invention comprises the use of a gene product of the "human glutamine-tRNA ligase, glutamine-tRNA ligase homology"-superfamily, preferably being involved in aminoacyl-tRNA-synthetases, nucleotide binding, translation, and/or PROTEIN SYNTHESIS, more preferred with the activity of a glutamine-tRNA ligase from Saccharomyces cerevisiae or its homolog, e.g. as shown herein, for the production of the fine chemical, meaning of malic acid and/or compositions comprising malic acid, in particular for increasing the amount of malic acid, preferably malic acid in free or bound form in an organism or a part thereof, as mentioned. In one embodiment, in the process of the present invention the activity of a glutamine-tRNA ligase is increased or generated, e.g. from Saccharomyces cerevisiae or a homolog thereof.
The sequence of b1896 from Escherichia coli K12 has been published in Blattner,F.R. et al., Science 277 (5331), 1453-1474 (1997) and it is being defined as a protein with trehalose-6-phosphate synthase activity. Accordingly, in one embodiment, the process of the present invention comprises the use of a protein b1896 from Escherichia coli K12 or its homolog, e.g. as shown herein, for the production of the respective fine chemical, in particular for increasing the amount of citramalic acid, fumaric acid, malic acid, succinic acid, trihydroxybutyric acid and/or trihydroxybutanoic acid , preferably in free or bound form in an organism or a part thereof, as mentioned. In one embodiment, in the process of the present invention the activity of the trehalose-6-phosphate synthase b1896 is increased.
The sequence of YBL015W from Saccharomyces cerevisiae has been published in Goffeau, A. et al., Science 274 (5287), 546-547 (1996) and it is being defined as a protein having acetyl-coA hydrolase activity. Accordingly, in one embodiment, the process of the present invention comprises the use of a protein having said activity, for the production of the respective fine chemical, in particular for increasing the amount of fumaric acid and/or malic acid , preferably in free or bound form in an organism or a part thereof, as mentioned. In one embodiment, in the process of the present invention said acetyl-coA hydrolase activity YBL015W is increased.
The sequence of YCR059C from Saccharomyces cerevisiae has been published in Goffeau, A. et al. Science 274 (5287), 546-547 (1996) and its activity is being defined as an unclassified protein. Accordingly, in one embodiment, the process of the present invention comprises the use of said unclassified protein from Saccharomyces cerevisiae or its homolog, e.g. as shown herein, for the production of the respective fine chemical, in particular for increasing the amount of fumaric acid , preferably in free or bound form in an organism or a part thereof, as mentioned. In one embodiment, in the process of the present invention the activity of the unclassified protein YCR059Cis increased.
The sequence of YFR007W from Saccharomyces cerevisiae has been published in Goffeau, A. et al. Science 274 (5287), 546-547 (1996) and its activity is being defined as an unclassified protein. Accordingly, in one embodiment, the process of the present invention comprises the use of said unclassified protein from Saccharomyces cerevisiae or its homolog, e.g. as shown herein, for the production of the respective fine chemical, in particular for increasing the amount of fumaric acid preferably in free or bound form in an organism or a part thereof, as mentioned. In one embodiment, in the process of the present invention the activity of the unclassified protein YFR007W is increased.
The sequence of YJL055W from Saccharomyces cerevisiae has been published in Goffeau, A. et al. Science 274 (5287), 546-547 (1996) and its activity is being defined as a lysine-decarboxylase-like protein. Accordingly, in one embodiment, the process of the present invention comprises the use of said protein YJL055W from Saccharomyces cerevisiae or its homolog, e.g. as shown herein, for the production of the respective fine chemical, in particular for increasing the amount of fumaric acid, preferably in free or bound form in an organism or a part thereof, as mentioned. In one embodiment, in the process of the present invention the activity of the protein YJL055W is increased.
The sequence of YJL099W from Saccharomyces cerevisiae has been published in Goffeau, A. et al. Science 274 (5287), 546-547 (1996) and its activity is being defined as a protein involved in chitin biosynthesis. Accordingly, in one embodiment, the process of the present invention comprises the use of said protein involved in chitin biosynthesis from Saccharomyces cerevisiae or its homolog, e.g. as shown herein, for the production of the respective fine chemical, in particular for increasing the amount of fumaric acid and/or malic acid, preferably in free or bound form in an organism or a part thereof, as mentioned. In one embodiment, in the process of the present invention the activity of the protein YJL099W is increased.
The sequence of YMR241W from Saccharomyces cerevisiae has been published in Bowman, S. Nature 387 (6632 Suppl), 90-93 (1997) and its activity is being defined as a mitochondrial DNA replication protein having yeast suppressor gene activity (abf2). Accordingly, in one embodiment, the process of the present invention comprises the use of a protein having suppressor gene activity from Saccharomyces cerevisiae or its homolog, e.g. as shown herein, for the production of the respective fine chemical, in particular for increasing the amount of fumaric acid, preferably in free or bound form in an organism or a part thereof, as mentioned. In one embodiment, in the process of the present invention the activity of said protein YMR241 W is increased.
The sequence of YPR024W from Saccharomyces cerevisiae has been published in Bussey, H. et al. Nature 387 (6632 Suppl), 103-105 (1997) and its activity is being defined as a protein having mitochondrial ATPase activity. Accordingly, in one embodiment, the process of the present invention comprises the use of an ATPase from Saccharomyces cerevisiae or its homolog, e.g. as shown herein, for the production of the respective fine chemical, in particular for increasing the amount of fumaric acid, preferably in free or bound form in an organism or a part thereof, as mentioned. In one embodiment, in the process of the present invention the activity of the protein YPR024W is increased.
The sequence of YPR138C from Saccharomyces cerevisiae has been published in Bussey, H. et al. Nature 387 (6632 Suppl), 103-105 (1997) and its activity is being defined as a protein having ammonia transporter activity. Accordingly, in one embodiment, the process of the present invention comprises the use of a protein having ammonia transporter activity from Saccharomyces cerevisiae or its homolog, e.g. as shown herein, for the production of the respective fine chemical, in particular for increasing the amount of fumaric acid and/or malic acid, preferably in free or bound form in an organism or a part thereof, as mentioned. In one embodiment, in the process of the present invention the activity of the protein YPR138C is increased.
The sequence of b0730 from Escherichia coli K12 has been published in Buck D. et al. Biochem. J. 260:737-747(1989) and its activity is being defined as a protein having fatty acyl responsive regulator and/or "transcriptional regulator of the succinyl-CoA synthase operon" function. Accordingly, in one embodiment, the process of the present invention comprises the use of a protein b0730 having fatty acyl responsive regulator function from Escherichia coli K12 or its homolog, e.g. as shown herein, for the production of the respective fine chemical, in particular for increasing the amount of fumaric acid, malic acid and/or succinic acid, preferably in free or bound form in an organism or a part thereof, as mentioned. In one embodiment, in the process of the present invention the activity of the protein b0730 is increased.
The sequence of b1611 from Escherichia coli K12 has been published in Blattner, F.R. et al. Science 277 (5331), 1453-1474 (1997) and its activity is being defined as a protein having fumarase C or fumaric acid hydratase class II activity. Accordingly, in one embodiment, the process of the present invention comprises the use of a protein having fumarase C or fumaric acid hydratase class II activity from Escherichia coli K12 or its homolog, e.g. as shown herein, for the production of the respective fine chemical, in particular for increasing the amount of fumaric acid, preferably in free or bound form in an organism or a part thereof, as mentioned. In one embodiment, in the process of the present invention the activity of the protein b1611 is increased.
The sequence of b2699 from Escherichia coli K12 has been published in Blattner, F.R. et al. Science 277 (5331), 1453-1474 (1997) and its activity is being defined as a protein having DNA strand exchange and recombination activity (RecA). Accordingly, in one embodiment, the process of the present invention comprises the use of a protein b2699 having RecA activity from Escherichia coli K12 or its homolog, e.g. as shown herein, for the production of the respective fine chemical, in particular for increasing the amount of fumaric acid, trihydroxybutyric acid and/or trihydroxybutanoic acid, preferably in free or bound form in an organism or a part thereof, as mentioned. In one embodiment, in the process of the present invention the activity of the protein b2699 is increased.
The sequence of b4139 from Escherichia coli K12 has been published in Blattner, F.R. et al. Science 277 (5331), 1453-1474 (1997) and its activity is being defined as a protein having aspartate ammonia-lyase (aspartase) activity. Accordingly, in one embodiment, the process of the present invention comprises the use of a protein having aspartate ammonia-lyase (aspartase) activity from Escherichia coli K12 or its homolog, e.g. as shown herein, for the production of the respective fine chemical, in particular for increasing the amount of fumaric acid, preferably in free or bound form in an organism or a part thereof, as mentioned. In one embodiment, in the process of the present invention the activity of the protein b4139 is increased.
The sequence of b1676 from Escherichia coli K12 has been published in Blattner, F.R. et al. Science 277 (5331), 1453-1474 (1997) and its activity is being defined as a protein having pyruvic acid kinase I (formerly F) activity and is stimulated by fructose. Accordingly, in one embodiment, the process of the present invention comprises the use of a protein having pyruvic acid kinase I (formerly F) activity from Escherichia coli K12 or its homolog, e.g. as shown herein, for the production of the respective fine chemical, in particular for increasing the amount of fumaric acid, preferably in free or bound form in an organism or a part thereof, as mentioned. In one embodiment, in the process of the present invention the activity of the protein b1676 is increased.
The sequence of b4063 from Escherichia coli K12 has been published in Blattner, F.R. et al. Science 277 (5331), 1453-1474 (1997) and its activity is being defined as a protein having redox-sensing activator function of soxS and transcriptional activator function for superoxide response. Accordingly, in one embodiment, the process of the present invention comprises the use of a protein having redox-sensing activator function of soxS and transcriptional activator function for superoxide response from Escherichia coli K12 or its homolog, e.g. as shown herein, for the production of the respective fine chemical, in particular for increasing the amount of fumaric acid and/or malic acid, preferably in free or bound form in an organism or a part thereof, as mentioned. In one embodiment, in the process of the present invention the activity of the protein b4063 is increased.
The sequence of YBR084W from Saccharomyces cerevisiae has been published in Goffeau, A. et al., Science 274 (5287), 546-547 (1996) and its activity is being defined as a protein having mitochondrial C1-tetrahydrofolate synthase activity. Accordingly, in one embodiment, the process of the present invention comprises the use of a protein having mitochondrial C1-tetrahydrofolate synthase activity from Saccharomyces cerevisiae or its homolog, e.g. as shown herein, for the production of the respective fine chemical, in particular for increasing the amount of malic acid, preferably in free or bound form in an organism or a part thereof, as mentioned. In one embodiment, in the process of the present invention the activity of the protein YBR084W is increased.
The sequence of YBR184W from Saccharomyces cerevisiae has been published in Goffeau, A. et al., Science 274 (5287), 546-547 (1996) and its activity is being defined as an unclassified protein. Accordingly, in one embodiment, the process of the present invention comprises the use of an unclassified protein YBR184W from Saccharomyces cerevisiae or its homolog, e.g. as shown herein, for the production of the respective fine chemical, in particular for increasing the amount of malic acid, preferably in free or bound form in an organism or a part thereof, as mentioned. In one embodiment, in the process of the present invention the activity of the unclassified protein YBR184W is increased.
The sequence of YCL032W from Saccharomyces cerevisiae has been published in Goffeau, A. et al., Science 274 (5287), 546-547 (1996) and its activity is being defined as a protein involved in the pheromone signal transduction pathway. Accordingly, in one embodiment, the process of the present invention comprises the use of the protein YCL032W involved in the pheromone signal transduction pathway from Saccharomyces cerevisiae or its homolog, e.g. as shown herein, for the production of the respective fine chemical, in particular for increasing the amount of malic acid and/or succinic acid, preferably in free or bound form in an organism or a part thereof, as mentioned. In one embodiment, in the process of the present invention the activity of the protein YCL032W is increased.
The sequence of YGR007W from Saccharomyces cerevisiae has been published in Tettelin, H. et al., Nature 387 (6632 Suppl), 81-84 (1997) and its activity is being defined as a protein having choline phosphate cytidylyltransferase ( also called phosphoethanolamine cytidylyltransferase or phosphocholine cytidylyltransferase) activity. Accordingly, in one embodiment, the process of the present invention comprises the use of a protein having choline phosphate cytidylyltransferase activity from Saccharomyces cerevisiae or its homolog, e.g. as shown herein, for the production of the respective fine chemical, in particular for increasing the amount of malic acid, preferably in free or bound form in an organism or a part thereof, as mentioned. In one embodiment, in the process of the present invention the activity of the protein YGR007W is increased.
The sequence of YJL072C from Saccharomyces cerevisiae has been published in Goffeau, A. Science 274 (5287), 546-547 (1996) and its function is being defined as a protein being the subunit of the GINS complex required for chromosomal DNA replication. Accordingly, in one embodiment, the process of the present invention comprises the use of a protein YJL072C from Saccharomyces cerevisiae or its homolog, e.g. as shown herein, for the production of the respective fine chemical, in particular for increasing the amount of malic acid, preferably in free or bound form in an organism or a part thereof, as mentioned. In one embodiment, in the process of the present invention the activity of the protein YJL072C is increased.
The sequence of YKL132C from Saccharomyces cerevisiae has been published in Goffeau, A. et al., Science 274 (5287), 546-547 (1996) and its activity is being defined as a protein having folyl-polyglutamate synthetase activity. Accordingly, in one embodiment, the process of the present invention comprises the use of a protein YKL132C from Saccharomyces cerevisiae or its homolog, e.g. as shown herein, for the production of the respective fine chemical, in particular for increasing the amount of malic acid, preferably in free or bound form in an organism or a part thereof, as mentioned. In one embodiment, in the process of the present invention the activity of the YKL132C protein is increased.
The sequence of b0695 from Escherichia coli K12 has been published in Blattner, F.R. et al., Science 277 (5331), 1453-1474 (1997) and its activity is being defined as a protein having histidine kinase activity. Accordingly, in one embodiment, the process of the present invention comprises the use of a protein b0695 from Escherichia coli K12 or its homolog, e.g. as shown herein, for the production of the respective fine chemical, in particular for increasing the amount of pyruvic acid, preferably in free or bound form in an organism or a part thereof, as mentioned. In one embodiment, in the process of the present invention the activity of the protein b0695 is increased.
The sequence of YOR044W from Saccharomyces cerevisiae has been published in Dujon, B. et al., Nature 387 (6632 Suppl), 98-102 (1997) and its activity is being defined as an unclassified protein. Accordingly, in one embodiment, the process of the present invention comprises the use of a protein YOR044W from Saccharomyces cerevisiae or its homolog, e.g. as shown herein, for the production of the respective fine chemical, in particular for increasing the amount of succinic acid, preferably in free or bound form in an organism or a part thereof, as mentioned. In one embodiment, in the process of the present invention the activity of the protein YOR044W is increased.

**[0023.0.16.16]** Homologues ( = homologs) of the present gene products can be derived from any organisms as long as the homologue confers the herein mentioned activity, in particular, confers an increase in the respective fine chemical amount or content.
Further, in the present invention, the term "homologue" relates to the sequence of an organism having the highest sequence homology to the herein mentioned or listed sequences of all expressed sequences of said organism.
However, the person skilled in the art knows, that, preferably, the homologue has said the-fine-chemical-increasing activity and, if known, the same biological function or activity in the organism as at least one of the protein(s) indicated in Table I, Column 3, lines 190 to 226 or lines 564 to 594, e.g. having the sequence of a polypeptide encoded by a nucleic acid molecule comprising the sequence indicated in indicated in Table I, Column 5 or 7, lines 190 to 226 or lines 564 to 594.
In one embodiment, the homolog of any one of the polypeptides indicated in Table II, column 3, line 190 or 564 is a homolog having the same or a similar activity. In particular an increase of activity confers an increase in the content of the respective fine chemical in the organisms preferably citramalic acid.
In one embodiment, the homolog of the polypeptides indicated in Table II, column 3, lines 191 to 205 or lines 565 to 571 is a homolog having the same or a similar activity. In particular an increase of activity confers an increase in the content of the respective fine chemical in the organisms preferably fumaric acid.
In one embodiment, the homolog of the any one of the polypeptides indicated in Table II, column 3, lines 206 to 217 or lines 577 to 583 is a homolog having the same or a similar activity. In particular an increase of activity confers an increase in the content of malic acid in the organisms.
In one embodiment, the homolog of the any one of the polypeptides indicated in Table II, column 3, lines 218 to 219 is a homolog having the same or a similar activity. In particular an increase of activity confers an increase in the content of the lacton of trihydroxybutyric acid in the organisms.
In one embodiment, the homolog of the any one of the polypeptides indicated in Table II, column 3, line 220 or line 584 is a homolog having the same or a similar activity. In particular an increase of activity confers an increase in the content of pyruvic acid in the organisms.
In one embodiment, the homolog of the any one of the polypeptides indicated in Table II, column 3, lines 221 to 224 or lines 585 to 591 is a homolog having the same or a similar activity. In particular an increase of activity confers an increase in the content of succinic acid in the organisms.
In one embodiment, the homolog of the any one of the polypeptides indicated in Table II, column 3, lines 225 to 226 or lines 592 to 594 is a homolog having the same or a similar activity. In particular an increase of activity confers an increase in the content of trihydroxybutanoic acid in the organisms.
In one embodiment, the homolog of the any one of the polypeptides indicated in Table II, column 3, lines 572 to 576 is a homolog having the same or a similar activity. In particular an increase of activity confers an increase in the content of glyceric acid in the organisms.

**[0023.1.0.16]** Homologs of the polypeptides indicated in Table II, column 3, lines 190 to 226 or lines 564 to 594 may be the polypeptides encoded by the nucleic acid molecules indicated in Table I, column 7, lines 190 to 226 or lines 564 to 594 or may be the polypeptides indicated in Table II, column 7, lines 190 to 226 or lines 564 to 594.
Homologs of the polypeptides indicated in Table II, column 3, lines 190 or 564 may be the polypeptides encoded by the nucleic acid molecules indicated in Table I, column 7, line 190 or 564, respectively or may be the polypeptides indicated in Table II, column 7, lines 190 or 564, having citramalic acid content- and/or amount-increasing activity.
Homologs of the polypeptide indicated in Table II, column 3, lines 191 to 205 or lines 565 to 571 may be the polypeptides encoded by the nucleic acid molecules indicated in Table I, column 7, lines 191 to 205 or lines 565 to 571, respectively or may be the polypeptides indicated in Table II, column 7, lines 191 to 205 or lines 565 to 571, having a fumaric acid content- and/or amount-increasing activity.
Homologs of the polypeptide indicated in Table II, column 3, lines 206 to 217 or lines 577 to 583 may be the polypeptides encoded by the nucleic acid molecules indicated in Table I, column 7, lines 206 to 217 or lines 577 to 583, respectively or may be the polypeptides indicated in Table II, column 7, lines 206 to 217 or lines 577 to 583, having a malic acid content- and/or amount-increasing activity.
Homologs of the polypeptide indicated in Table II, column 3, lines 218 and 219 may be the polypeptides encoded by the nucleic acid molecules indicated in Table I, column 7, lines 218 and 219, respectively or may be the polypeptides indicated in Table II, column 7, lines 218 and 219 , having a trihydroxybuyrate content- and/or amount-increasing activity.
Homologs of the polypeptide indicated in Table II, column 3, line 220 or line 584 may be the polypeptides encoded by the nucleic acid molecules indicated in Table I, column 7, line 220 or line 584, respectively or may be the polypeptides indicated in Table II, column 7, line 220 or line 584, having a pyruvic acid content- and/or amount-increasing activity.
Homologs of the polypeptide indicated in Table II, column 3, lines 221 to 224 or lines 585 to 591 may be the polypeptides encoded by the nucleic acid molecules indicated in Table I, column 7, lines 221 to 224 or lines 585 to 591, respectively or may be the polypeptides indicated in Table II, column 7, lines 221 to 224 or lines 585 to 591, having a succinic acid content- and/or amount-increasing activity.
Homologs of the polypeptide indicated in Table II, column 3, lines 225 and 226 or lines 592 to 594 may be the polypeptides encoded by the nucleic acid molecules indicated in Table I, column 7, lines 225 and 226 or lines 592 to 594 , respectively or may be the polypeptides indicated in Table II, column 7, lines 225 and 226 or lines 592 to 594, having a trihydroxybutanoic acid content- and/or amount-increasing activity.

**[0024.0.0.16]** see **[0024.0.0.0]**

**[0025.0.16.16]** In accordance with the invention, a protein or polypeptide has the "activity of a protein of the invention", e.g. the activity of a protein indicated in Table II, column 3, lines 190 to 226 or lines 564 to 594 if its *de novo* activity, or its increased expression directly or indirectly leads to an increased glyceric acid, citramalic acid, fumaric acid, malic acid, pyruvic acid, succinic acid, trihydroxybutyric acid and/or trihydroxybutanoic acid level, resp., in the organism or a part thereof, preferably in a cell of said organism. In a preferred embodiment, the protein or polypeptide has the above-mentioned additional activities of a protein indicated in Table II, column 3, lines 190 to 226 or lines 564 to 594. Throughout the specification the activity or preferably the biological activity of such a protein or polypeptide or an nucleic acid molecule or sequence encoding such protein or polypeptide is identical or similar if it still has the biological or enzymatic activity of any one of the proteins indicated in Table II, column 3, lines 190 to 226 or lines 564 to 594, or which has at least 10% of the original enzymatic activity, preferably 20%, particularly preferably 30%, most particularly preferably 40% in comparison to any one of the proteins indicated in Table II, column 3, line 190 to 226 or lines 564 to 594.
In one embodiment, the polypeptide of the invention confers said activity, e.g. the increase of the respective fine chemical in an organism or a part thereof, if it is derived from an organism, which is evolutionary close to the organism indicated in Table I, column 4 and is expressed in an organism, which is evolutionary distant to the origin organism. For example origin and expressing organism are derived from different families, orders, classes or phylums whereas origin and the organism indicated in Table I, column 4 are derived from the same families, orders, classes or phylums.

**[0025.1.0.16] and [0025.2.0.16] see [0025.1.0.0] and [0025.2.0.0]**

**[0025.3.16.16]** In one embodiment, the polypeptide of the invention or used in the method of the invention confers said activity, e.g. the increase of the respective fine chemical in an organism or a part thereof, if it is derived from an organism, which is evolutionary close to the organism indicated in Table I, column 4 and is expressed in an organism, which is evolutionary distant to the origin organism. For example origin and expressing organism are derived from different families, orders, classes or phylums whereas origin and the organism indicated in Table I, column 4 are derived from the same families, orders, classes or phylums.

**[0026.0.0.16] to [0033.0.0.16]: see [0026.0.0.0] to [0033.0.0.0]**

**[0034.0.16.16]** Preferably, the reference, control or wild type differs form the subject of the present invention only in the cellular activity of the polypeptide of the invention or used in the process of the invention, e.g. as result of an increase in the level of the nucleic acid molecule of the present invention or an increase of the specific activity of the polypeptide of the invention or used in the process of the invention. E.g., it differs by or in the expression level or activity of an protein having the activity of a protein as indicated in Table II, column 3, lines 190 to 226 or lines 564 to 594 or being encoded by a nucleic acid molecule indicated in Table I, column 5, lines 190 to 226 or lines 564 to 594 or its homologs, e.g. as indicated in Table I, column 7, lines 190 to 226 or lines 564 to 594, its biochemical or genetic causes. It therefore shows the increased amount of the respective fine chemical.

**[0035.0.0.16] to [0044.0.0.16]: see [0035.0.0.0] to [0044.0.0.0]**

**[0045.0.16.16]** In one embodiment the activity of the Escherichia coli K12 protein b0057 or its homologs, as indicated in Table II, columns 5 or 7, line 572, e.g. a protein with an activity as defined in [0022.0.16.16], is increased, conferring preferably, the increase of the fine chemical, preferably of glyceric acid between 35% and 57%, or more.
In one embodiment the activity of the Escherichia coli K12 protein b0161 or its homologs, as indicated in Table II, columns 5 or 7, line 565 for fumaric acid or line 577 for malic acid, e.g. a protein with an activity as defined in [0022.0.16.16], is increased, conferring preferably, the increase of the fine chemical, preferably of fumaric acid and/or of malic acid, preferably of fumaric acid between 64% and 151% and/or of malic acid between 64% and 229%, or more.
In one embodiment the activity of the Escherichia coli K12 protein b0970 or its homologs, as indicated in Table II, columns 5 or 7, line 592, e.g. a protein with an activity as defined in [0022.0.16.16], is increased, conferring preferably, the increase of the fine chemical, preferably of trihydroxybutanoic acid between 34% and 80%, or more.
In one embodiment the activity of the Escherichia coli K12 protein b1343 or its homologs, as indicated in Table II, columns 5 or 7, line 593, e.g. a protein with an activity as defined in [0022.0.16.16], is increased, conferring preferably, the increase of the fine chemical, preferably of trihydroxybutanoic acid between 33% and 108%, or more.
In one embodiment the activity of the Escherichia coli K12 protein b1693 or its homologs, as indicated in Table II, columns 5 or 7, line 564 for citramalic acid or line 573 for glyceric acid, e.g. a protein with an activity as defined in [0022.0.16.16], is increased, conferring preferably, the increase of the fine chemical, preferably of glyceric acid and/or of citramalic acid, preferably of glyceric acid between 33% and 52% and/or of citramalic acid between 48% and 105%, or more.
In one embodiment the activity of the Escherichia coli K12 protein b1738 or its homologs, as indicated in Table II, columns 5 or 7, line 566 for fumaric acid or line 585 for succinic acid, e.g. a protein with an activity as defined in [0022.0.16.16], is increased, conferring preferably, the increase of the fine chemical, preferably of succinic acid and/or of fumaric acid, preferably of succinic acid between 49% and 95% and/or of fumaric acid between 53% and 149%, or more.
In one embodiment the activity of the Escherichia coli K12 protein b1961 or its homologs, as indicated in Table II, columns 5 or 7, line 567 for fumaric acid or line 586 for succinic acid, e.g. a protein with an activity as defined in [0022.0.16.16], is increased, conferring preferably, the increase of the fine chemical, preferably of succinic acid and/or of fumaric acid, preferably of succinic acid between 29% and 54% and/or of fumaric acid between 76% and 105%, or more.
In one embodiment the activity of the Escherichia coli K12 protein b2478 or its homologs, as indicated in Table II, columns 5 or 7, line 578, e.g. a protein with an activity as defined in [0022.0.16.16], is increased, conferring preferably, the increase of the fine chemical, preferably of malic acid between 51 % and 124%, or more.
In one embodiment the activity of the Escherichia coli K12 protein b2599 or its homologs, as indicated in Table II, columns 5 or 7, line 587, e.g. a protein with an activity as defined in [0022.0.16.16], is increased, conferring preferably, the increase of the fine chemical, preferably of succinic acid between 33% and 156%, or more.
In one embodiment the activity of the Escherichia coli K12 protein b3116 or its homologs, as indicated in Table II, columns 5 or 7, line 588 for succinic acid or line 568 for fumaric acid or line 579 for malic acid, e.g. a protein with an activity as defined in [0022.0.16.16], is increased, conferring preferably, the increase of the fine chemical, preferably of succinic acid, and/or of fumaric acid, and/or of malic acid between, preferably of succinic acid between 31 % and 42%, and/or of fumaric acid between 63% and 99%, and/or of malic acid between 60% and 212%, or more.
In one embodiment the activity of the Escherichia coli K12 protein b3129 or its homologs, as indicated in Table II, columns 5 or 7, line 574, e.g. a protein with an activity as defined in [0022.0.16.16], is increased, conferring preferably, the increase of the fine chemical, preferably of glyceric acid between 30% and 35%, or more.
In one embodiment the activity of the Escherichia coli K12 protein b3160 or its homologs, as indicated in Table II, columns 5 or 7, line 589, e.g. a protein with an activity as defined in [0022.0.16.16], is increased, conferring preferably, the increase of the fine chemical, preferably of succinic acid between 33% and 90%, or more.
In one embodiment the activity of the Escherichia coli K12 protein b3169 or its homologs, as indicated in Table II, columns 5 or 7, line 580 for malic acid or 594 for trihydroxybutanoic acid, e.g. a protein with an activity as defined in [0022.0.16.16], is increased, conferring preferably, the increase of the fine chemical, preferably of malic acid and/or of trihydroxybutanoic acid, preferably of malic acid between 132% and 143% and/or of trihydroxybutanoic acid between 60% and 88%, or more.
In one embodiment the activity of the Escherichia coli K12 protein b3172 or its homologs, as indicated in Table II, columns 5 or 7, line 581 for malic acid or 569 for fumaric acid, e.g. a protein with an activity as defined in [0022.0.16.16], is increased, conferring preferably, the increase of the fine chemical, preferably of fumaric acid and/or of malic acid, preferably of fumaric acid between 63% and 325% and/or of malic acid between 64% and 408%, or more.
In one embodiment the activity of the Escherichia coli K12 protein b3231 or its homologs, as indicated in Table II, columns 5 or 7, line 575 for glyceric acid or line 584 for pyruvic acid, e.g. a protein with an activity as defined in [0022.0.16.16], is increased, conferring preferably, the increase of the fine chemical, preferably of pyruvic acid and/or of glyceric acid, preferably of pyruvic acid between 43% and 46% and/or of glyceric acid between 21 % and 34%, or more.
In one embodiment the activity of the Escherichia coli K12 protein b3938 or its homologs, as indicated in Table II, columns 5 or 7, line 576, e.g. a protein with an activity as defined in [0022.0.16.16], is increased, conferring preferably, the increase of the fine chemical, preferably of glyceric acid between 37% and 40%, or more.
In one embodiment the activity of the Escherichia coli K12 protein b4122 or its homologs, as indicated in Table II, columns 5 or 7, line 570, e.g. a protein with an activity as defined in [0022.0.16.16], is increased, conferring preferably, the increase of the fine chemical, preferably of fumaric acid between 108% and 430%, or more.
In one embodiment the activity of the Escherichia coli K12 protein b4129 or its homologs, as indicated in Table II, columns 5 or 7, line 590, e.g. a protein with an activity as defined in [0022.0.16.16], is increased, conferring preferably, the increase of the fine chemical, preferably of succinic acid between 40% and 84%, or more.
In one embodiment the activity of the Saccharomyces cerevisiae protein YCL038C or its homologs, as indicated in Table II, columns 5 or 7, line 591 for succinic acid or line 582 for malic acid, e.g. a protein with an activity as defined in [0022.0.16.16], is increased, conferring preferably, the increase of the fine chemical, preferably of succinic acid and/or of malic acid, preferably of succinic acid between 47% and 91 % and/or of malic acid between 87% and 123%, or more.
In one embodiment the activity of the Saccharomyces cerevisiae protein YCR012W or its homologs, as indicated in Table II, columns 5 or 7, line 571, e.g. a protein with an activity as defined in [0022.0.16.16], is increased, conferring preferably, the increase of the fine chemical, preferably of fumaric acid between 62% and 93%, or more.
In one embodiment the activity of the Saccharomyces cerevisiae protein YOR168W or its homologs, as indicated in Table II, columns 5 or 7, line 583, e.g. a protein with an activity as defined in [0022.0.16.16], is increased, conferring preferably, the increase of the fine chemical, preferably of malic acid between 56% and 562%, or more.
In case the activity of the Escherichia coli K12 protein b1896 or its homologs, e.g. as indicated in Table II, columns 5 or 7, line 190 is increased, in one embodiment the increase of the respective fine chemical, preferably of citramalic acid between 63 % and 99% or more is conferred.
In one embodiment, in case the activity of the Saccharomyces cerevisae protein YBL015W or its homologs, e.g. as indicated in Table II, columns 5 or 7, line 191 is increased, preferably, in one embodiment the increase of the respective fine chemical, preferably of fumaric acid between 50% and 126% or more is conferred.
In one embodiment, in case the activity of the Saccharomyces cerevisae protein YCR059C or its homologs e.g. a protein as indicated in Table 11, columns 5 or 7, line 192, is increased, preferably, in one embodiment the increase of the respective fine chemical, preferably of fumaric acid between 71% and 183% or more is conferred.
In one embodiment, in case the activity of the Saccharomyces cerevisiae protein YFR007W or its homologs, e.g. a protein as indicated in Table II, columns 5 or 7, line 193, is increased, preferably, in one embodiment an increase of the respective fine chemical, preferably of fumaric acid between 69% and 156% or more is conferred.
In one embodiment, in case the activity of the Saccharomyces cerevisiae protein YJL055W or its homologs, e.g. a protein as indicated in Table 11, columns 5 or 7, line 194, is increased, preferably, in one embodiment an increase of the respective fine chemical, preferably of fumaric acid between 72 % and 561% or more is conferred.
In one embodiment, in case the activity of the Saccharomyces cerevisiae protein YJL099W or its homologs, e.g. a protein as indicated in Table II, columns 5 or 7, line 195, is increased, preferably, in one embodiment an increase of the respective fine chemical, preferably of fumaric acid between 98% and 734% or more is conferred.
In one embodiment, in case the activity of the Saccharomyces cerevisiae protein YMR241W or its homologs, e.g. a protein as indicated in Table II, columns 5 or 7, line 196, is increased, preferably, in one embodiment an increase of the respective fine chemical, preferably of fumaric acid between 75% and 368% or more is conferred.
In one embodiment, in case the activity of the Saccharomyces cerevisiae protein YPR024W or its homologs, e.g. a protein as indicated in Table II, columns 5 or 7, line 197, is increased, preferably, in one embodiment an increase of the respective fine chemical, preferably of fumaric acid between 97% and 374% or more is conferred.
In one embodiment, in case the activity of the Saccharomyces cerevisiae protein YPR138C or its homologs, e.g. a protein as indicated in Table II, columns 5 or 7, line 198, is increased, preferably, in one embodiment an increase of the respective fine chemical, preferably of fumaric acid between 61 % and 327% or more is conferred.
In one embodiment, in case the activity of the Escherichia coli K12 protein b0730 or its homologs, e.g. a protein as indicated in Table II, columns 5 or 7, line 199, is increased, preferably, in one embodiment an increase of the respective fine chemical, preferably of fumaric acid between 75% and 838% or more is conferred.
In one embodiment, in case the activity of the Escherichia coli K12 protein b1611 or its homologs, e.g. a protein as indicated in Table II, columns 5 or 7, line 200, is increased, preferably, in one embodiment an increase of the respective fine chemical, preferably of fumaric acid between 106% and 328% or more is conferred.
In one embodiment, in case the activity of the Escherichia coli K12 protein b2699 or its homologs, e.g. a protein as indicated in Table II, columns 5 or 7, line 201, is increased, preferably, in one embodiment an increase of the respective fine chemical, preferably of fumaric acid between 65% and 802% or more is conferred.
In one embodiment, in case the activity of the Escherichia coli K12 protein b4139 or its homologs, e.g. a protein as indicated in Table II, columns 5 or 7; line 202, is increased, preferably, in one embodiment an increase of the respective fine chemical, preferably of fumaric acid between 118% and 525% or more is conferred.
In one embodiment, in case the activity of the Escherichia coli K12 protein b1676 or its homologs, e.g. a protein as indicated in Table II, columns 5 or 7, line 203, is increased, preferably, in one embodiment an increase of the respective fine chemical, preferably of fumaric acid between 108% and 430% or more is conferred.
In one embodiment, in case the activity of the Escherichia coli K12 protein b1896 or its homologs, e.g. a protein as indicated in Table II, columns 5 or 7, line 204, is increased, preferably, in one embodiment an increase of the respective fine chemical, preferably of fumaric acid between 232% and 320% or more is conferred.
In one embodiment, in case the activity of the Escherichia coli K12 protein b4063 or its homologs, e.g. a protein as indicated in Table II, columns 5 or 7, line 205, is increased, preferably, in one embodiment an increase of the respective fine chemical, preferably of fumaric acid between 64% and 308% or more is conferred.
In one embodiment, in case the activity of the Saccharomyces cerevisiae protein YBL015W or its homologs, e.g. a protein as indicated in Table II, columns 5 or 7, line 206, is increased, preferably, in one embodiment an increase of the respective fine chemical, preferably of malic acid between 45% and 86% or more is conferred.
In one embodiment, in case the activity of the Saccharomyces cerevisiae protein YBR084W or its homologs, e.g. a protein as indicated in Table II, columns 5 or 7, line 207, is increased, preferably, in one embodiment an increase of the respective fine chemical, preferably of malic acid between 107% and 448% or more is conferred.
In one embodiment, in case the activity of the Saccharomyces cerevisiae protein YBR184W or its homologs, e.g. a protein as indicated in Table II, columns 5 or 7, line 208, is increased, preferably, in one embodiment an increase of the respective fine chemical, preferably of malic acid between 67% and 213% or more is conferred.
In one embodment, in case the activity of the Saccharomyces cerevisiae protein YCL032W or its homologs, e.g. a protein as indicated in Table II, columns 5 or 7, line 209, is increased, preferably, in one embodiment an increase of the respective fine chemical, preferably of malic acid between 87% and 123% or more is conferred.
In one embodiment, in case the activity of the Saccharomyces cerevisiae protein YGR007W or its homologs, e.g. a protein as indicated in Table II, columns 5 or 7, line 210, is increased, preferably, in one embodiment an increase of the respective fine chemical, preferably of malic acid between 55% and 90% or more is conferred.
In one embodiment, in case the activity of the Saccharomyces cerevisiae protein YJL072C or its homologs, e.g. a protein as indicated in Table II, columns 5 or 7, line 211, is increased, preferably, in one embodiment an increase of the respective fine chemical, preferably of malic acid between 49% and 249% or more is conferred.
In one embodiment, in case the activity of the Saccharomyces cerevisiae protein YJL099W or its homologs, e.g. a protein as indicated in Table II, columns 5 or 7, line 212, is increased, preferably, in one embodiment an increase of the respective fine chemical, preferably of malic acid between 94% and 797% or more is conferred.
In one embodiment, in case the activity of the Saccharomyces cerevisiae protein YKL132C or its homologs, e.g. a protein as indicated in Table II, columns 5 or 7, line 213, is increased, preferably, in one embodiment an increase of the respective fine chemical, preferably of malic acid between 109% and 213% or more is conferred.
In one embodiment, in case the activity of the Saccharomyces cerevisiae protein YPR138C or its homologs, e.g. a protein as indicated in Table II, columns 5 or 7, line 214, is increased, preferably, in one embodiment an increase of the respective fine chemical, preferably of malic acid between 72% and 359% or more is conferred.
In one embodiment, in case the activity of the Escherichia coli K12 protein b0730 or its homologs, e.g. a protein as indicated in Table II, columns 5 or 7, line 215, is increased, preferably, in one embodiment an increase of the respective fine chemical, preferably of malic acid between 96% and 672% or more is conferred.
In one embodiment, in case the activity of the Escherichia coli K12 protein b1896 or its homologs, e.g. a protein as indicated in Table II, columns 5 or 7, line 216, is increased, preferably, in one embodiment an increase of the respective fine chemical, preferably of malic acid between 149% and 319% or more is conferred.
In one embodiment, in case the activity of the Escherichia coli K12 protein b4063 or its homologs, e.g. a protein as indicated in Table II, columns 5 or 7, line 217, is increased, preferably, in one embodiment an increase of the respective fine chemical, preferably of malic acid between 78% and 93% or more is conferred.
In one embodiment, in case the activity of the Escherichia coli K12 protein b2699 or its homologs, e.g. a protein as indicated in Table II, columns 5 or 7, line 218, is increased, preferably, in one embodiment an increase of the respective fine chemical, preferably of trihydroxybutyric acid between 48% and 215% or more is conferred.
In one embodiment, in case the activity of the Escherichia coli K12 protein b1896 or its homologs, e.g. a protein as indicated in Table II, columns 5 or 7, line 219, is increased, preferably, in one embodiment an increase of the respective fine chemical, preferably of trihydroxybutyric acid between 34% and 96% or more is conferred.
In one embodiment, in case the activity of the Escherichia coli K12 protein b0695 or its homologs, e.g. a protein as indicated in Table II, columns 5 or 7, line 220, is increased, preferably, in one embodiment an increase of the respective fine chemical, preferably of pyruvic acid between 61% and 155% or more is conferred.
In one embodiment, in case the activity of the Saccharomyces cerevisiae protein YCL032W or its homologs, e.g. a protein as indicated in Table II, columns 5 or 7, line 221, is increased, preferably, in one embodiment an increase of the respective fine chemical, preferably of succinic acid between 47% and 91% or more is conferred.
In one embodiment, in case the activity of the Saccharomyces cerevisiae protein YOR044W or its homologs, e.g. a protein as indicated in Table II, columns 5 or 7, line 222, is increased, preferably, in one embodiment an increase of the respective fine chemical, preferably of succinic acid between 45% and 265% or more is conferred.
In one embodiment, in case the activity of the Escherichia coli K12 protein b0730 or its homologs, e.g. a protein as indicated in Table II, columns 5 or 7, line 223, is increased, preferably, in one embodiment an increase of the respective fine chemical, preferably of succinic acid between 51% and 332% or more is conferred.
In one embodiment, in case the activity of the Escherichia coli K12 protein b1896 or its homologs, e.g. a protein as indicated in Table II, columns 5 or 7, line 224, is increased, preferably, in one embodiment an increase of the respective fine chemical, preferably of succinic acid between 55% and 110% or more is conferred.
In one embodiment, in case the activity of the Escherichia coli K12 protein b2699 or its homologs, e.g. a protein as indicated in Table II, columns 5 or 7, line 225, is increased, preferably, in one embodiment an increase of the respective fine chemical, preferably of trihydroxybutanoic acid between 82% and 281% or more is conferred.
In one embodiment, in case the activity of the Escherichia coli K12 protein b1896 or its homologs, e.g. a protein as indicated in Table II, columns 5 or 7, line 226, is increased, preferably, in one embodiment an increase of the respective fine chemical, preferably of trihydroxybutanoic acid between 60% and 161% or more is conferred.

**[0046.0.16.16]** In case the activity of a protein as disclosed in [0016.0.16.16] or its homologs, as indicated in Table I, columns 5 or 7, line 190, e.g. a protein with an activity as defined in [0022.0.16.16], is increased, preferably, an increase of the respective fine chemical, preferably of citramalic acid and of an other organic acid is conferred.
In case the activity of a protein as disclosed in [0016.0.16.16] or its homologs, as indicated in Table I, columns 5 or 7, lines 191 to 205, e.g. a protein with an activity as defined in [0022.0.16.16], is increased, preferably, an increase of the respective fine chemical, preferably of fumaric acid and of an other organic acid is conferred.
In case the activity of a protein as disclosed in [0016.0.16.16] or its homologs, as indicated in Table I, columns 5 or 7, lines 206 to 217, e.g. a protein with an activity as defined in [0022.0.16.16], is increased, preferably, an increase of the respective fine chemical, preferably of malic acid and of an other organic acid is conferred.
In case the activity of a protein as disclosed in [0016.0.16.16] or its homologs, as indicated in Table I, columns 5 or 7, lines 218 or 219, e.g. a protein with an activity as defined in [0022.0.16.16], is increased, preferably, an increase of the respective fine chemical, preferably of lacton of trihydroxybutyric acid and of an other organic acid is conferred.
In case the activity of a protein as disclosed in [0016.0.16.16] or its homologs, as indicated in Table I, columns 5 or 7, line 220, e.g. a protein with an activity as defined in [0022.0.16.16], is increased, preferably, an increase of the respective fine chemical, preferably of pyruvic acid and of an other organic acid is conferred.
In case the activity of a protein as disclosed in [0016.0.16.16] or its homologs, as indicated in Table I, columns 5 or 7, lines 221 to 224, e.g. a protein with an activity as defined in [0022.0.16.16], is increased, preferably, an increase of the respective fine chemical, preferably of succinic acid and of an other organic acid is conferred.
In case the activity of a protein as disclosed in [0016.0.16.16] or its homologs, as indicated in Table I, columns 5 or 7, lines 225 to 226, e.g. a protein with an activity as defined in [0022.0.16.16], is increased, preferably, an increase of the respective fine chemical, preferably of trihydroxybutanoic acid and of an other organic acid is conferred.
In case the activity of a Escherichia coli K12 protein b0057 as disclosed in [0016.0.16.16] or its homologs, as indicated in Table I, columns 5 or 7, line 572 , e.g. a protein with an activity as defined in [0022.0.16.16], is increased, preferably, an increase of the respective fine chemical, preferably of glyceric acid and of an other organic acid is conferred.
In case the activity of a Escherichia coli K12 protein b0161 as disclosed in [0016.0.16.16] or its homologs, as indicated in Table I, columns 5 or 7, line 565 for fumaric acid and/or line 577 for malic acid, e.g. a protein with an activity as defined in [0022.0.16.16], is increased, preferably, an increase of the respective fine chemical, preferably of fumaric acid and/or malic acid, and of an other organic acid is conferred.
In case the activity of a Escherichia coli K12 protein b0970 as disclosed in [0016.0.16.16] or its homologs, as indicated in Table I, columns 5 or 7, line 592 , e.g. a protein with an activity as defined in [0022.0.16.16], is increased, preferably, an increase of the respective fine chemical, preferably of trihydroxybutanoic acid, and of an other organic acid is conferred.
In case the activity of a Escherichia coli K12 protein b1343 as disclosed in [0016.0.16.16] or its homologs, as indicated in Table I, columns 5 or 7, line 593, e.g. a protein with an activity as defined in [0022.0.16.16], is increased, preferably, an increase of the respective fine chemical, preferably of trihydroxybutanoic acid, and of an other organic acid is conferred.
In case the activity of a Escherichia coli K12 protein b1693 as disclosed in [0016.0.16.16] or its homologs, as indicated in Table I, columns 5 or 7, line 573 for glyceric acid and/or line 564 for citramalic acid , e.g. a protein with an activity as defined in [0022.0.16.16], is increased, preferably, an increase of the respective fine chemical, preferably of glyceric acid and/or citramalic acid, and of an other organic acid is conferred.
In case the activity of a Escherichia coli K12 protein b1738 as disclosed in [0016.0.16.16] or its homologs, as indicated in Table I, columns 5 or 7, line 566 for fumaric acid and/or line 585 for succinic acid, e.g. a protein with an activity as defined in [0022.0.16.16], is increased, preferably, an increase of the respective fine chemical, preferably of fumaric acid and/or succinic acid, and of an other organic acid is conferred.
In case the activity of a Escherichia coli K12 protein b1961 as disclosed in [0016.0.16.16] or its homologs, as indicated in Table I, columns 5 or 7, line 567 for fumaric acid and/or line 586 for succinic acid, e.g. a protein with an activity as defined in [0022.0.16.16], is increased, preferably, an increase of the respective fine chemical, preferably of fumaric acid and/or succinic acid, and of an other organic acid is conferred.
In case the activity of a Escherichia coli K12 protein b2478 as disclosed in [0016.0.16.16] or its homologs, as indicated in Table I, columns 5 or 7, line 578, e.g. a protein with an activity as defined in [0022.0.16.16], is increased, preferably, an increase of the respective fine chemical, preferably of malic acid and of an other organic acid is conferred.
In case the activity of a Escherichia coli K12 protein b2599 as disclosed in [0016.0.16.16] or its homologs, as indicated in Table I, columns 5 or 7, line 587, e.g. a protein with an activity as defined in [0022.0.16.16], is increased, preferably, an increase of the respective fine chemical, preferably of succinic acid, and of an other organic acid is conferred.
In case the activity of a Escherichia coli K12 protein b3116 as disclosed in [0016.0.16.16] or its homologs, as indicated in Table I, columns 5 or 7, line 588 for succinic acid, and/or line 568 for fumaric acid and/or line 579 for malic acid, e.g. a protein with an activity as defined in [0022.0.16.16], is increased, preferably, an increase of the respective fine chemical, preferably of succinic acid, fumaric acid and/or malic acid, and of an other organic acid is conferred.
In case the activity of a Escherichia coli K12 protein b3129 as disclosed in [0016.0.16.16] or its homologs, as indicated in Table I, columns 5 or 7, line 574, e.g. a protein with an activity as defined in [0022.0.16.16], is increased, preferably, an increase of the respective fine chemical, preferably of glyceric acid, and of an other organic acid is conferred.
In case the activity of a Escherichia coli K12 protein b3160 as disclosed in [0016.0.16.16] or its homologs, as indicated in Table I, columns 5 or 7, line 589, e.g. a protein with an activity as defined in [0022.0.16.16], is increased, preferably, an increase of the respective fine chemical, preferably of succinic acid, and of an other organic acid is conferred.
In case the activity of a Escherichia coli K12 protein b3169 as disclosed in [0016.0.16.16] or its homologs, as indicated in Table I, columns 5 or 7, line 594 for trihydroxabutanoic acid and/or line 580 for malic acid, e.g. a protein with an activity as defined in [0022.0.16.16], is increased, preferably, an increase of the respective fine chemical, preferably of trihydroxybutanoic acid and/or malic acid, and of an other organic acid is conferred.
In case the activity of a Escherichia coli K12 protein b3172 as disclosed in [0016.0.16.16] or its homologs, as indicated in Table I, columns 5 or 7, line 569 for fumaric acid and/or line 581 for malic acid, e.g. a protein with an activity as defined in [0022.0.16.16], is increased, preferably, an increase of the respective fine chemical, preferably of fumaric acid and/or malic acid, and of an other organic acid is conferred.
In case the activity of a Escherichia coli K12 protein b3231 as disclosed in [0016.0.16.16] or its homologs, as indicated in Table I, columns 5 or 7, line 575 for glyceric acid and/or line 584 for pyruvic acid, e.g. a protein with an activity as defined in [0022.0.16.16], is increased, preferably, an increase of the respective fine chemical, preferably of glyceric acid and/or pyruvic acid, and of an other organic acid is conferred.
In case the activity of a Escherichia coli K12 protein b3938 as disclosed in [0016.0.16.16] or its homologs, as indicated in Table I, columns 5 or 7, line 576, e.g. a protein with an activity as defined in [0022.0.16.16], is increased, preferably, an increase of the respective fine chemical, preferably of glyceric acid, and of an other organic acid is conferred.
In case the activity of a Escherichia coli K12 protein b4122 as disclosed in [0016.0.16.16] or its homologs, as indicated in Table I, columns 5 or 7, line 570, e.g. a protein with an activity as defined in [0022.0.16.16], is increased, preferably, an increase of the respective fine chemical, preferably of fumaric acid, and of an other organic acid is conferred.
In case the activity of a Escherichia coli K12 protein b4129 as disclosed in [0016.0.16.16] or its homologs, as indicated in Table I, columns 5 or 7, line 590, e.g. a protein with an activity as defined in [0022.0.16.16], is increased, preferably, an increase of the respective fine chemical, preferably of succinic acid, and of an other organic acid is conferred.
In case the activity of a Saccharomyces cerevisiae protein YCL038C as disclosed in [0016.0.16.16] or its homologs, as indicated in Table I, columns 5 or 7, line 591 for succinic acid and/or line 582 for malic acid, e.g. a protein with an activity as defined in [0022.0.16.16], is increased, preferably, an increase of the respective fine chemical, preferably of succinic acid and/or malic acid, and of an other organic acid is conferred.
In case the activity of a Saccharomyces cerevisiae protein YCR012W as disclosed in [0016.0.16.16] or its homologs, as indicated in Table I, columns 5 or 7, line 571, e.g. a protein with an activity as defined in [0022.0.16.16], is increased, preferably, an increase of the respective fine chemical, preferably of fumaric acid, and of an other organic acid is conferred.
In case the activity of a Saccharomyces cerevisiae protein YOR168W as disclosed in [0016.0.16.16] or its homologs, as indicated in Table I, columns 5 or 7, line 583, e.g. a protein with an activity as defined in [0022.0.16.16], is increased, preferably, an increase of the respective fine chemical, preferably of malic acid, and of an other organic acid is conferred.

**[0047.0.0.16] to [0048.0.0.16]: see [0047.0.0.0] to [0048.0.0.0]**

**[0049.0.16.16]** A protein having an activity conferring an increase in the amount or level of the respective fine chemical citramalic acid preferably has the structure of the polypeptide described herein. In a particular embodiment, the polypeptides used in the process of the present invention or the polypeptide of the present invention comprises the sequence of a consensus sequence as indicated in Table IV, column 7, lines 190 or line 564 or of a polypeptide as indicated in Table II, columns 5 or 7, line 190 or line 564 or of a functional homologue thereof as described herein, or of a polypeptide encoded by the nucleic acid molecule characterized herein or the nucleic acid molecule according to the invention, for example by a nucleic acid molecule as indicated in Table I, columns 5 or 7, line 190 or line 564 or its herein described functional homologues and has the herein mentioned activity conferring an increase in the citramalic acid level.
A protein having an activity conferring an increase in the amount or level of the fumaric acid preferably has the structure of the polypeptide described herein. In a particular embodiment, the polypeptides used in the process of the present invention or the polypeptide of the present invention comprises the sequence of a consensus sequence as indicated in Table IV, column 7, lines 191 to 205 or lines 565 to 571 or of a polypeptide as indicated in Table II, columns 5 or 7, lines 191 to 205 or lines 565 to 571 or of a functional homologue thereof as described herein, or of a polypeptide encoded by the nucleic acid molecule characterized herein or the nucleic acid molecule according to the invention, for example by a nucleic acid molecule as indicated in Table I, columns 5 or 7, lines 191 to 205 or lines 565 to 571 or its herein described functional homologues and has the herein mentioned activity confering an increase in the fumaric acid level.
A protein having an activity conferring an increase in the amount or level of the glyceric acid preferably has the structure of the polypeptide described herein. In a particular embodiment, the polypeptides used in the process of the present invention or the polypeptide of the present invention comprises the sequence of a consensus sequence as indicated in Table IV, column 7, lines 572 to 576 or of a polypeptide as indicated in Table II, columns 5 or 7, lines 572 to 576 or of a functional homologue thereof as described herein, or of a polypeptide encoded by the nucleic acid molecule characterized herein or the nucleic acid molecule according to the invention, for example by a nucleic acid molecule as indicated in Table I, columns 5 or 7, lines 572 to 576 or its herein described functional homologues and has the herein mentioned activity confering an increase in the glyceric acid level.
A protein having an activity conferring an increase in the amount or level of the respective fine chemical malic acid preferably has the structure of the polypeptide described herein. In a particular embodiment, the polypeptides used in the process of the present invention or the polypeptide of the present invention comprises the sequence of a consensus sequence as indicated in Table IV, column 7, lines 206 to 217 or lines 577 to 583 or of a polypeptide as indicated in Table II, columns 5 or 7, lines 577 to 583or of a functional homologue thereof as described herein, or of a polypeptide encoded by the nucleic acid molecule characterized herein or the nucleic acid molecule according to the invention, for example by a nucleic acid molecule as indicated in Table I, columns 5 or 7, line 206 to 217 or lines 577 to 583 or its herein described functional homologues and has the herein mentioned activity conferring an increase in the malic acidlevel.
A protein having an activity conferring an increase in the amount or level of the respective fine chemical trihydroxybutyric acid preferably has the structure of the polypeptide described herein. In a particular embodiment, the polypeptides used in the process of the present invention or the polypeptide of the present invention comprises the sequence of a consensus sequence as indicated in Table IV, column 7, line 218 or 219, or of a polypeptide as indicated in Table II, columns 5 or 7, line218 or 219 or of a functional homologue thereof as described herein, or of a polypeptide encoded by the nucleic acid molecule characterized herein or the nucleic acid molecule according to the invention, for example by a nucleic acid molecule as indicated in Table I, columns 5 or 7, line 218 or 219 or its herein described functional homologues and has the herein mentioned activity conferring an increase in the trihydroxybutyric acid level.
A protein having an activity conferring an increase in the amount or level of the respective fine chemical pyruvic acid preferably has the structure of the polypeptide described herein. In a particular embodiment, the polypeptides used in the process of the present invention or the polypeptide of the present invention comprises the sequence of a consensus sequence as indicated in Table IV, column 7, line 220 or line 584 or of a polypeptide as indicated in Table II, columns 5 or 7, line 220 or line 584 or of a functional homologue thereof as described herein, or of a polypeptide encoded by the nucleic acid molecule characterized herein or the nucleic acid molecule according to the invention, for example by a nucleic acid molecule as indicated in Table I, columns 5 or 7, line 220 or line 584 or its herein described functional homologues and has the herein mentioned activity conferring an increase in the pyruvic acid level.
A protein having an activity conferring an increase in the amount or level of the respective fine chemical succinic acid preferably has the structure of the polypeptide described herein. In a particular embodiment, the polypeptides used in the process of the present invention or the polypeptide of the present invention comprises the sequence of a consensus sequence as indicated in Table IV, column 7, lines 221 to 224 or lines 585 to 591 or of a polypeptide as indicated in Table II, columns 5 or 7, lines 221 to 224 or lines 585 to 591 or of a functional homologue thereof as described herein, or of a polypeptide encoded by the nucleic acid molecule characterized herein or the nucleic acid molecule according to the invention, for example by a nucleic acid molecule as indicated in Table I, columns 5 or 7, lines 221 to 224 or lines 585 to 591 or its herein described functional homologues and has the herein mentioned activity conferring an increase in the succinic acid level.
A protein having an activity conferring an increase in the amount or level of the respective fine chemical trihydroxybutanoic acid preferably has the structure of the polypeptide described herein. In a particular embodiment, the polypeptides used in the process of the present invention or the polypeptide of the present invention comprises the sequence of a consensus sequence as indicated in Table IV, column 7, lines 225 to 226 or lines 592 to 594 or of a polypeptide as indicated in Table II, columns 5 or 7, lines 225 to 226 or lines 592 to 594 or of a functional homologue thereof as described herein, or of a polypeptide encoded by the nucleic acid molecule characterized herein or the nucleic acid molecule according to the invention, for example by a nucleic acid molecule as indicated in Table I, columns 5 or 7, line 225 to 226 or lines 592 to 594 or its herein described functional homologues and has the herein mentioned activity conferring an increase in the trihydroxybutanoic acid level.

**[0050.0.16.16]** For the purposes of the present invention, the term "the respective fine chemical" also encompass the corresponding salts, such as, for example, the potassium or sodium salts of glyceric acid, citramalic acid, fumaric acid, malic acid, pyruvic acid, succinic acid, trihydroxybutyric acid and/or trihydroxybutanoic acid, resp., or their ester, or glucoside thereof, e.g the diglucoside thereof.

**[0051.0.16.16]** Owing to the biological activity of the proteins which are used in the process according to the invention and which are encoded by nucleic acid molecules according to the invention, it is possible to produce compositions comprising the respective fine chemical, i.e. an increased amount of the free chemical free or bound, e.g. compositions comprising glyceric acid, citramalic acid, fumaric acid, malic acid, pyruvic acid, succinic acid, trihydroxybutyric acid and/or trihydroxybutanoic acid. Depending on the choice of the organism used for the process according to the present invention, for example a microorganism or a plant, compositions or mixtures of organic acids or there salts, e.g. glyceric acid, citramalic acid, fumaric acid, malic acid, pyruvic acid, succinic acid, trihydroxybutyric acid and/or trihydroxybutanoic acid can be produced.

**[0052.0.0.16] see [0052.0.0.0]**

**[0053.0.16.16]** In one embodiment, the process of the present invention comprises one or more of the following steps
a) stabilizing a protein conferring the increased expression of a protein encoded by the nucleic acid molecule of the invention or used in the process of the invention or of the polypeptid of the invention or used in the process of the invention, e.g. of a polypeptide having an activity of a protein as indicated in Table II, column 3, lines 190 to 226 or lines 564 to 594 or its homologs, e.g. as indicated in Table II, columns 5 or 7, lines 190 to 226 or lines 564 to 594 , activity having herein-mentioned the respective fine chemical increasing activity;
b) stabilizing a mRNA conferring the increased expression of a protein encoded by the nucleic acid molecule of the invention or used in the process of the invention, e.g. of a polypeptide having an activity of a protein as indicated in Table II, column 3, lines 190 to 226 or lines 564 to 594 or its homologs activity, e.g. as indicated in Table II, columns 5 or 7, lines 190 to 226 or lines 564 to 594, or of a mRNA encoding the polypeptide of the present invention having herein-mentioned the respective fine chemical increasing activity;
c) increasing the specific activity of a protein conferring the increased expression of a protein encoded by the nucleic acid molecule of the invention or used in the process of the invention or of the polypeptide of the present invention having herein-mentioned the respective fine chemical increasing activity, e.g. of a polypeptide having an activity of a protein as indicated in Table II, column 3, lines 190 to 226 or lines 564 to 594 or its homologs activity, e.g. as indicated in Table II, columns 5 or 7, lines 190 to 226 or lines 564 to 594, or decreasing the inhibitory regulation of the polypeptide of the invention or used in the process of the invention;
d) generating or increasing the expression of an endogenous or artificial transcription factor mediating the expression of a protein conferring the increased expression of a protein encoded by the nucleic acid molecule of the invention or used in the process of the invention or of the polypeptide of the invention or used in the process of the invention having herein-mentioned the respective fine chemical increasing activity, e.g. of a polypeptide having an activity of a protein as indicated in Table II, column 3, lines 190 to 226 or lines 564 to 594 or its homologs activity, e.g. as indicated in Table II, columns 5 or 7, lines 190 to 226 or lines 564 to 594;
e) stimulating activity of a protein conferring the increased expression of a protein encoded by the nucleic acid molecule of the present invention or a polypeptide of the present invention having herein-mentioned the respective fine chemical increasing activity, e.g. of a polypeptide having an activity of a protein as indicated in Table II, column 3, lines 190 to 226 or lines 564 to 594 or its homologs activity, e.g. as indicated in Table II, columns 5 or 7, lines 190 to 226 or lines 564 to 594, by adding one or more exogenous inducing factors to the organism or parts thereof;
f) expressing a transgenic gene encoding a protein conferring the increased expression of a polypeptide encoded by the nucleic acid molecule of the present invention or a polypeptide of the present invention, having herein-mentioned the respective fine chemical increasing activity, e.g. of a polypeptide having an activity of a protein as indicated in Table II, column 3, lines 190 to 226 or lines 564 to 594 or its homologs activity, e.g. as indicated in Table II, columns 5 or 7, lines 190 to 226 or lines 564 to 594,
g) increasing the copy number of a gene conferring the increased expression of a nucleic acid molecule encoding a polypeptide encoded by the nucleic acid molecule of the invention or used in the process of the invention or the polypeptide of the invention or used in the process of the invention having herein-mentioned the respective fine chemical increasing activity, e.g. of a polypeptide having an activity of a protein as indicated in Table II, column 3, lines 190 to 226 or lines 564 to 594 or its homologs, e.g. as indicated in Table II, columns 5 or 7, lines 190 to 226 or lines 564 to 594, activity.
h) Increasing the expression of the endogenous gene encoding the polypeptide of the invention or used in the process of the invention, e.g. a polypeptide having an activity of a protein as indicated in Table II, column 3, lines 190 to 226 or lines 564 to 594 or its homologs activity, e.g. as indicated in Table II, columns 5 or 7, lines 190 to 226 or lines 564 to 594, by adding positive expression or removing negative expression elements, e.g. homologous recombination can be used to either introduce positive regulatory elements like for plants the 35S enhancer into the promoter or to remove repressor elements form regulatory regions. Further gene conversion methods can be used to disrupt repressor elements or to enhance to activity of positive elements. Positive elements can be randomly introduced in plants by T-DNA or transposon mutagenesis and lines can be identified in which the positive elements have be integrated near to a gene of the invention or used in the process of the invention, the expression of which is thereby enhanced;
i) Modulating growth conditions of an organism in such a manner, that the expression or activity of the gene encoding the protein of the invention or used in the process of the invention or the protein itself is enhanced for example microorganisms or plants can be grown for example under a higher temperature regime leading to an enhanced expression of heat shock proteins, which can lead to an enhanced respective fine chemical production, and/or
j) selecting of organisms with especially high activity of the proteins of the invention or used in the process of the invention from natural or from mutagenized resources and breeding them into the target organisms, e.g. the elite crops.

**[0054.0.16.16]** Preferably, said mRNA is the nucleic acid molecule of the present invention and/or the protein conferring the increased expression of a protein encoded by the nucleic acid molecule of the present invention or the polypeptide having the herein mentioned activity is the polypeptide of the present invention, e.g. conferring the increase of the respective fine chemical after increasing the expression or activity of the encoded polypeptide or having the activity of a polypeptide having an activity of a protein as indicated in Table II, columns 3 or 5, lines 190 to 226 or lines 564 to 594, resp., or its homologs activity, e.g. as indicated in Table II, columns 5 or 7, lines 190 to 226 or lines 564 to 594, resp.

**[0055.0.0.16] to [0067.0.0.16]: see [0055.0.0.0] to [0067.0.0.0]**

**[0068.0.16.16]** The mutation is introduced in such a way that the production of glyceric acid, citramalic acid, fumaric acid, malic acid, pyruvic acid, succinic acid, trihydroxybutyric acid and/or trihydroxybutanoic acid is not adversely affected.

**[0069.0.0.16] see [0069.0.0.0]**

**[0070.0.16.16]** Owing to the introduction of a gene or a plurality of genes conferring the expression of the nucleic acid molecule of the invention or used in the process of the invention or the polypeptide of the invention or used in the process of the invention, for example the nucleic acid construct mentioned below, or encoding a protein of the invention or used in the process of the invention into an organism alone or in combination with other genes, it is possible not only to increase the biosynthetic flux towards the end product, but also to increase, modify or create *de novo* an advantageous, preferably novel metabolite composition in the organism, e.g. an advantageous composition of citramalic acid, glyceric acid, fumaric acid, malic acid, pyruvic acid, succinic acid, trihydroxybutyric acid and/or trihydroxybutanoic acid or their biochemical derivatives , e.g. comprising a higher content of (from a viewpoint of nutrional physiology limited) glyceric acid, citramalic acid, fumaric acid, malic acid, pyruvic acid, succinic acid, trihydroxybutyric acid and/or trihydroxybutanoic acid or their derivatives.

**[0071.0.0.16] see [0071.0.0.0]**

**[0072.0.16.16]**

**[0073.0.16.16]** Accordingly, in one embodiment, the process according to the invention relates to a process, which comprises:
providing a non-human organism, preferably a microorganism, a non-human animal, a plant or animal cell, a plant or animal tissue or a plant; and
increasing an activity of a polypeptide of the invention or used in the process of the invention or a homolog thereof, e.g. as indicated in Table II, columns 5 or 7, lines 190 to 226 or lines 564 to 594, or of a polypeptide being encoded by the nucleic acid molecule of the present invention and described below, e.g. conferring an increase of the respective fine chemical in an organism, preferably in a microorganism, a non-human animal, a plant or animal cell, a plant or animal tissue or a plant, and
growing an organism, preferably a microorganism, a non-human animal, a plant or animal cell, a plant or animal tissue or a plant under conditions which permit the production of the respective fine chemical in the organism, preferably the microorganism, the plant cell, the plant tissue or the plant; and
if desired, recovering, optionally isolating, the free and/or bound the respective fine chemical synthesized by the organism, the microorganism, the non-human animal, the plant or animal cell, the plant or animal tissue or the plant.

**[0074.0.16.16]** The organism, in particular the microorganism, non-human animal, the plant or animal cell, the plant or animal tissue or the plant is advantageously grown in such a way that it is not only possible to recover, if desired isolate the free or bound respective fine chemical.

**[0075.0.0.16] to [0077.0.0.16]: see [0075.0.0.0] to [0077.0.0.0]**

**[0078.0.16.16]** The organism such as microorganisms or plants or the recovered, and if desired isolated, the respective fine chemical can then be processed further directly into foodstuffs or animal feeds or for other applications. The fermentation broth, fermentation products, plants or plant products can be purified with methods known to the person skilled in the art. Products of these different work-up procedures are glyceric acid, citramalic acid, fumaric acid, malic acid, pyruvic acid, succinic acid, trihydroxybutyric acid and/or trihydroxybutanoic acid or comprising compositions of glyceric acid, citramalic acid, fumaric acid, malic acid, pyruvic acid, succinic acid, trihydroxybutyric acid and/or trihydroxybutanoic acid still comprising fermentation broth, plant particles and cell components in different amounts, advantageously in the range of from 0 to 99% by weight, preferably below 80% by weight, especially preferably below 50% by weight.

**[0079.0.0.16] to [0084.0.0.16]: see [0079.0.0.0] to [0084.0.0.0]**

**[0084.0.16.16]** -/-

**[0085.0.16.16]** With regard to the nucleic acid sequence as depicted a nucleic acid construct which contains a nucleic acid sequence mentioned herein or an organism (= transgenic organism) which is transformed with said nucleic acid sequence or said nucleic acid construct, "transgene" means all those constructs which have been brought about by genetic manipulation methods, preferably in which either
a) a nucleic acid sequence as indicated in Table I, columns 5 or 7, lines 190 to 226 or lines 564 to 594, or a derivative thereof, or
b) a genetic regulatory element, for example a promoter, which is functionally linked to the nucleic acid sequence as indicated in Table I, columns 5 or 7, lines 190 to 226 or lines 564 to 594, or a derivative thereof, or
c) (a) and (b)
is/are not present in its/their natural genetic environment or has/have been modified by means of genetic manipulation methods, it being possible for the modification to be, by way of example, a substitution, addition, deletion, inversion or insertion of one or more nucleotide. "Natural genetic environment" means the natural chromosomal locus in the organism of origin or the presence in a genomic library. In the case of a genomic library, the natural, genetic environment of the nucleic acid sequence is preferably at least partially still preserved. The environment flanks the nucleic acid sequence at least on one side and has a sequence length of at least 50 bp, preferably at least 500 bp, particularly preferably at least 1000 bp, very particularly preferably at least 5000 bp.

**[0086.0.0.16] to [0087.0.0.16]: see [0086.0.0.0] to [0087.0.0.0]**

**[0088.0.16.16]** In an advantageous embodiment of the invention, the organism takes the form of a plant whose content of the respective fine chemical is modified advantageously owing to the nucleic acid molecule of the present invention expressed. This is important for plant breeders since, for example, the nutritional value of plants for poultry is dependent on the abovementioned fine chemicals or the plants are more resistant to biotic and abiotic stress and the yield is increased.

**[0088.1.0.16] see [0088.1.0.0]**

**[0089.0.0.16] to [0094.0.0.16]: see [0089.0.0.0] to [0094.0.0.0]**

**[0095.0.16.16]** It may be advantageous to increase the pool of glyceric acid, citramalic acid, fumaric acid, malic acid, pyruvic acid, succinic acid, trihydroxybutyric acid and/or trihydroxybutanoic acid in the transgenic organisms by the process according to the invention in order to isolate high amounts of the pure respective fine chemical and/or to obtain increased resistance against biotic and abiotic stresses and to obtain higher yield.

**[0096.0.16.16]** In another preferred embodiment of the invention a combination of the increased expression of the nucleic acid sequence or the protein of the invention or used in the process of the invention together with the transformation of a protein or polypeptid or a compound, which functions as a sink for the desired fine chemical, for example in the organism, is useful to increase the production of the respective fine chemical.

**[0097.0.16.16] -/-**

**[0098.0.16.16]** In a preferred embodiment, the respective fine chemical is produced in accordance with the invention and, if desired, is isolated.

**[0099.0.10.16]** In the case of the fermentation of microorganisms, the abovementioned desired fine chemical may accumulate in the medium and/or the cells. If microorganisms are used in the process according to the invention, the fermentation broth can be processed after the cultivation. Depending on the requirement, all or some of the biomass can be removed from the fermentation broth by separation methods such as, for example, centrifugation, filtration, decanting or a combination of these methods, or else the biomass can be left in the fermentation broth. The fermentation broth can subsequently be reduced, or concentrated, with the aid of known methods such as, for example, rotary evaporator, thin-layer evaporator, falling film evaporator, by reverse osmosis or by nanofiltration. Afterwards advantageously further compounds for formulation can be added such as corn starch or silicates. This concentrated fermentation broth advantageously together with compounds for the formulation can subsequently be processed by lyophilization, spray drying, and spray granulation or by other methods. Preferably the respective fine chemical comprising compositions are isolated from the organisms, such as the microorganisms or plants or the culture medium in or on which the organisms have been grown, or from the organism and the culture medium, in the known manner, for example via extraction, distillation, crystallization, chromatography or a combination of these methods. These purification methods can be used alone or in combination with the aforementioned methods such as the separation and/or concentration methods.

**[0100.0.16.16]** Transgenic plants which comprise the fine chemicals such as glyceric acid, citramalic acid, fumaric acid, malic acid, pyruvic acid, succinic acid, trihydroxybutyric acid and/or trihydroxybutanoic acid synthesized in the process according to the invention can advantageously be marketed directly without there being any need for the fine chemicals synthesized to be isolated. Plants for the process according to the invention are listed as meaning intact plants and all plant parts, plant organs or plant parts such as leaf, stem, seeds, root, tubers, anthers, fibers, root hairs, stalks, embryos, calli, cotelydons, petioles, harvested material, plant tissue, reproductive tissue and cell cultures which are derived from the actual transgenic plant and/or can be used for bringing about the transgenic plant. In this context, the seed comprises all parts of the seed such as the seed coats, epidermal cells, seed cells, endosperm or embryonic tissue.
However, the respective fine chemical produced in the process according to the invention can also be isolated from the organisms, advantageously plants, as extracts, e.g. ether, alcohol, or other organic solvents or water containing extract and/or free fine chemicals. The respective fine chemical produced by this process can be obtained by harvesting the organisms, either from the crop in which they grow, or from the field. This can be done via pressing or extraction of the plant parts. To increase the efficiency of extraction it is beneficial to clean, to temper and if necessary to hull and to flake the plant material. To allow for greater ease of disruption of the plant parts, specifically the seeds, they can previously be comminuted, steamed or roasted. Seeds, which have been pre-treated in this manner can subsequently be pressed or extracted with solvents such as warm hexane. The solvent is subsequently removed. In the case of microorganisms, the latter are, after harvesting, for example extracted directly without further processing steps or else, after disruption, extracted via various methods with which the skilled worker is familiar. Thereafter, the resulting products can be processed further, i.e. degummed and/or refined. In this process, substances such as the plant mucilages and suspended matter can be first removed. What is known as desliming can be affected enzymatically or, for example, chemico-physically by addition of acid such as phosphoric acid.
Because glyceric acid, citramalic acid, fumaric acid, malic acid, pyruvic acid, succinic acid, trihydroxybutyric acid and/or trihydroxybutanoic acid in microorganisms are localized intracellular, their recovery essentially comes down to the isolation of the biomass. Well-established approaches for the harvesting of cells include filtration, centrifugation and coagulation/flocculation as described herein. Of the residual hydrocarbon, adsorbed on the cells, has to be removed. Solvent extraction or treatment with surfactants have been suggested for this purpose.

**[0101.0.10.16]** The identity and purity of the compound(s) isolated can be determined by prior-art techniques. They encompass high-performance liquid chromatography (HPLC), gas chromatography (GC), spectroscopic methods, mass spectrometry (MS), staining methods, thin-layer chromatography, NIRS, enzyme assays or microbiological assays. These analytical methods are compiled in: Patek et al. (1994) Appl. Environ. Microbiol. 60:133-140; Malakhova et al. (1996) Biotekhnologiya 11 27-32; and Schmidt et al. (1998) Bioprocess Engineer. 19:67-70. Ulmann's Encyclopedia of Industrial Chemistry (1996) Bd. A27, VCH Weinheim, pp. 89-90, pp. 521-540, pp. 540-547, pp. 559-566, 575-581 and pp. 581-587; Michal, G (1999) Biochemical Pathways: An Atlas of Biochemistry and Molecular Biology, John Wiley and Sons; Fallon, A. et al. (1987) Applications of HPLC in Biochemistry in: Laboratory Techniques in Biochemistry and Molecular Biology, vol. 17.

**[0102.0.16.16]** glyceric acid, citramalic acid, fumaric acid, malic acid, pyruvic acid, succinic acid, trihydroxybutyric acid and/or trihydroxybutanoic acid can for example be detected advantageously via HPLC, LC or GC separation methods. The unambiguous detection for the presence of glyceric acid, citramalic acid, fumaric acid, malic acid, pyruvic acid, succinic acid, trihydroxybutyric acid and/or trihydroxybutanoic acid containing products can be obtained by analyzing recombinant organisms using analytical standard methods: LC, LC-MS, MS or TLC. The material to be analyzed can be disrupted by sonication, grinding in a glass mill, liquid nitrogen and grinding, cooking, or via other applicable methods.

**[0103.0.16.16]** In a preferred embodiment, the present invention relates to a process for the production of the respective fine chemical comprising or generating in an organism or a part thereof the expression of at least one nucleic acid molecule comprising a nucleic acid molecule selected from the group consisting of:
a) nucleic acid molecule encoding, preferably at least the mature form, of the polypeptide having a sequence as indicated in Table II, columns 5 or 7, lines 190 to 226 or lines 564 to 594,or a fragment thereof, which confers an increase in the amount of the respective fine chemical in an organism or a part thereof;
b) nucleic acid molecule comprising, preferably at least the mature form, of a nucleic acid molecule having a sequence as indicated in Table I, columns 5 or 7, lines 190 to 226 or lines 564 to 594,
c) nucleic acid molecule whose sequence can be deduced from a polypeptide sequence encoded by a nucleic acid molecule of (a) or (b) as result of the degeneracy of the genetic code and conferring an increase in the amount of the respective fine chemical in an organism or a part thereof;
d) nucleic acid molecule encoding a polypeptide which has at least 50% identity with the amino acid sequence of the polypeptide encoded by the nucleic acid molecule of (a) to (c) and conferring an increase in the amount of the respective fine chemical in an organism or a part thereof;
e) nucleic acid molecule which hybridizes with a nucleic acid molecule of (a) to (c) under stringent hybridization conditions and conferring an increase in the amount of the respective fine chemical in an organism or a part thereof;
f) nucleic acid molecule encoding a polypeptide, the polypeptide being derived by substituting, deleting and/or adding one or more amino acids of the amino acid sequence of the polypeptide encoded by the nucleic acid molecules (a) to (d), preferably to (a) to (c) and conferring an increase in the amount of the respective fine chemical in an organism or a part thereof;
g) nucleic acid molecule encoding a fragment or an epitope of a polypeptide which is encoded by one of the nucleic acid molecules of (a) to (e), preferably to (a) to (c) and conferring an increase in the amount of the respective fine chemical in an organism or a part thereof;
h) nucleic acid molecule comprising a nucleic acid molecule which is obtained by amplifying nucleic acid molecules from a cDNA library or a genomic library using the primers pairs having a sequence as indicated in Table III, columns 7, lines 190 to 226 or lines 564 to 594, and conferring an increase in the amount of the respective fine chemical in an organism or a part thereof;
i) nucleic acid molecule encoding a polypeptide which is isolated, e.g. from an expression library, with the aid of monoclonal antibodies against a polypeptide encoded by one of the nucleic acid molecules of (a) to (h), preferably to (a) to (c), and conferring an increase in the amount of the respective fine chemical in an organism or a part thereof;
j) nucleic acid molecule which encodes a polypeptide comprising the consensus sequence having a sequences as indicated in Table IV, columns 7, lines 190 to 226 or lines 564 to 594 and conferring an increase in the amount of the respective fine chemical in an organism or a part thereof;
k) nucleic acid molecule comprising one or more of the nucleic acid molecule encoding the amino acid sequence of a polypeptide encoding a domain of the polypeptide indicated in Table II, columns 5 or 7, lines 190 to 226 or lines 564 to 594, and conferring an increase in the amount of the respective fine chemical in an organism or a part thereof; and
l) nucleic acid molecule which is obtainable by screening a suitable library under stringent conditions with a probe comprising one of the sequences of the nucleic acid molecule of (a) to (k), preferably to (a) to (c), or with a fragment of at least 15 nt, preferably 20 nt, 30 nt, 50 nt, 100 nt, 200 nt or 500 nt of the nucleic acid molecule characterized in (a) to (k), preferably to (a) to (c), and conferring an increase in the amount of the respective fine chemical in an organism or a part thereof;
or which comprises a sequence which is complementary thereto.

**[00103.1.18.16]** In one embodiment, the nucleic acid molecule used in the process of the invention distinguishes over the sequence indicated in Table IA, columns 5 or 7, lines 190 to 226 or lines 564 to 594, by one or more nucleotides. In one embodiment, the nucleic acid molecule used in the process of the invention does not consist of the sequence shown in indicated in Table I A, columns 5 or 7, lines 190 to 226 or lines 564 to 594: In one embodiment, the nucleic acid molecule used in the process of the invention is less than 100%, 99,999%, 99,99%, 99,9% or 99% identical to a sequence indicated in Table I A, columns 5 or 7, lines 190 to 226 or lines 564 to 594. In another embodiment, the nucleic acid molecule does not encode a polypeptide of a sequence indicated in Table II A, columns 5 or 7, lines 190 to 226 or lines 564 to 594.

**[00103.2.0.16]** In one embodiment, the nucleic acid molecule used in the process of the invention distinguishes over the sequence indicated in Table I B, columns 5 or 7, lines 190 to 226 or lines 564 to 594, by one or more nucleotides. In one embodiment, the nucleic acid molecule used in the process of the invention does not consist of the sequence shown in indicated in Table I B, columns 5 or 7, lines 190 to 226 or lines 564 to 594: In one embodiment, the nucleic acid molecule used in the process of the invention is less than 100%, 99,999%, 99,99%, 99,9% or 99% identical to a sequence indicated in Table I B, columns 5 or 7, lines 190 to 226 or lines 564 to 594. In another embodiment, the nucleic acid molecule does not encode a polypeptide of a sequence indicated in Table II B, columns 5 or 7, lines 190 to 226 or lines 564 to 594.

**[0104.0.16.16]** In one embodiment, the nucleic acid molecule used in the process of the present invention or used in the the process of the invention distinguishes over the sequence indicated in Table I, columns 5 or 7, lines 190 to 226 or lines 564 to 594 by one or more nucleotides. In one embodiment, the nucleic acid molecule of the invention or used in the process of the invention does not consist of the sequence indicated in Table I, columns 5 or 7, lines 190 to 226 or lines 564 to 594. In one embodiment, the nucleic acid molecule of the present invention is less than 100%, 99,999%, 99,99%, 99,9% or 99% identical to the sequence indicated in Table I, columns 5 or 7, lines 190 to 226 or lines 564 to 594. In another embodiment, the nucleic acid molecule does not encode a polypeptide of a sequence indicated in Table II, columns 5 or 7, lines 190 to 226 or lines 564 to 594.

**[0105.0.0.16] to [0107.0.0.16]: see [0105.0.0.0] to [0107.0.0.0]**

**[0108.0.16.16]** Nucleic acid molecules with the sequence as indicated in Table I, columns 5 or 7, lines 190 to 226 or lines 564 to 594, nucleic acid molecules which are derived from an amino acid sequences as indicated in Table II, columns 5 or 7, lines 190 to 226 or lines 564 to 594 or from polypeptides comprising the consensus sequence as indicated in Table IV, column 7, lines 190 to 226 or lines 564 to 594, or their derivatives or homologues encoding polypeptides with the enzymatic or biological activity of an activity of a polypeptide as indicated in Table II, column 3, 5 or 7, lines 190 to 226 or lines 564 to 594, e.g. conferring the increase of the respective fine chemical, meaning glyceric acid, citramalic acid, fumaric acid, malic acid, pyruvic acid, succinic acid, trihydroxybutyric acid and/or trihydroxybutanoic acid, resp., after increasing its expression or activity, are advantageously increased in the process according to the invention.

**[0109.0.16.16]** In one embodiment, said sequences are cloned into nucleic acid constructs, either individually or in combination. These nucleic acid constructs enable an optimal synthesis of the respective fine chemicals, in particular glyceric acid, citramalic acid, fumaric acid, malic acid, pyruvic acid, succinic acid, trihydroxybutyric acid and/or trihydroxybutanoic acid, produced in the process according to the invention.

**[0110.0.0.16] see [0110.0.0.0]**

**[0111.0.0.16] see [0111.0.0.0]**

**[0112.0.16.16]** The nucleic acid molecules used in the process according to the invention take the form of isolated nucleic acid sequences, which encode polypeptides with an activity of a polypeptide as indicated in Table II, column 3, lines 190 to 226 or lines 564 to 594 or having the sequence of a polypeptide as indicated in Table II, columns 5 and 7, lines 190 to 226 or lines 564 to 594 and conferring an increase in the glyceric acid, citramalic acid, fumaric acid, malic acid, pyruvic acid, succinic acid, trihydroxybutyric acid and/or trihydroxybutanoic acid level.

**[0113.0.0.16] to [0114.0.0.16]: see [0113.0.0.0] to [0114.0.0.0]**

**[0115.0.0.16] see [0115.0.0.0]**

**[0116.0.0.16] to [0120.0.0.16] see [0116.0.0.0] to [0120.0.0.0]**

**[0120.1.0.16]:** -/-

**[0121.0.16.16]** However, it is also possible to use artificial sequences, which differ in one or more bases from the nucleic acid sequences found in organisms, or in one or more amino acid molecules from polypeptide sequences found in organisms, in particular from the polypeptide sequences indicated in Table II, columns 5 or 7, lines 190 to 226 or the functional homologues thereof as described herein, preferably conferring above-mentioned activity, i.e. conferring a citramalic acid level increase after increasing the activity of the polypeptide sequences indicated in Table II, columns 5 or 7, line 190 or line 564 or conferring a fumaric acid level increase after increasing the activity of the polypeptide sequences indicated in Table II, columns 5 or 7, lines 191 to 205 or lines 565 to 571 or conferring a glyceric acid level increase after increasing the activity of the polypeptide sequence indicated in Table II, columns 5 or 7, lines 572 to 576, or conferring a malic acid level increase after increasing the activity of the polypeptide sequences indicated in Table II, columns 5 or 7, lines 206 to 217 or lines 577 to 583 or conferring a trihydroxybutyric acid level increase after increasing the activity of the polypeptide sequences indicated in Table II, columns 5 or 7, line 218 or 219 or conferring a pyruvic acid level increase after increasing the activity of the polypeptide sequences indicated in Table II, columns 5 or 7, line 220 or 584 or conferring a succinic acid level increase after increasing the activity of the polypeptide sequences indicated in Table II, columns 5 or 7, lines 221 to 224 or lines 585 to 591 or conferring a trihydroxybutanoic acid level increase after increasing the activity of the polypeptide sequences indicated in Table II, columns 5 or 7, lines 225 and 226 or lines 592 to 594.

**[0122.0.0.16] to [0127.0.0.16]: see [0122.0.0.0] to [0127.0.0.0]**

**[0128.0.16.16]** Synthetic oligonucleotide primers for the amplification, e.g. as the pairs indicated in Table III, columns 7, lines 190 to 226 or lines 564 to 594, by means of polymerase chain reaction can be generated on the basis of a sequence shown herein, for example the sequence as indicated in Table I, columns 5 or 7, lines 190 to 226 or lines 564 to 594 or the sequences derived from a sequence as indicated in Table II, columns 5 or 7, lines 190 to 226 or lines 564 to 594.

**[0129.0.16.16]** Moreover, it is possible to identify conserved regions from various organisms by carrying out protein sequence alignments with the polypeptide used in the process of the invention, in particular with sequences of the polypeptide of the invention or used in the process of the invention, from which conserved regions, and in turn, degenerate primers can be derived. Conserved region for the polypeptide of the invention or used in the process of the invention are indicated in the alignments shown in the figures. Conserved regions are those, which show a very little variation in the amino acid in one particular position of several homologs from different origin. The consensus sequence indicated in Table IV, columns 7, lines 190 to 226 or lines 564 to 594 is derived from said alignments.

**[0130.0.16.16]** Degenerated primers can then be utilized by PCR for the amplification of fragments of novel proteins having above-mentioned activity, e.g. conferring the increase of glyceric acid, citramalic acid, fumaric acid, malic acid, pyruvic acid, succinic acid, trihydroxybutyric acid and/or trihydroxybutanoic acid after increasing the expression or activity the protein comprising said fragment.

**[0131.0.0.16] to [0138.0.0.16]: see [0131.0.0.0] to [0138.0.0.0]**

**[0139.0.16.16]** Polypeptides having above-mentioned activity, i.e. conferring the respective fine chemical level increase, derived from other organisms, can be encoded by other DNA sequences which hybridise to a sequence indicated in Table I, columns 5 or 7, line 190 or line 564 for citramalic acid or indicated in Table I, columns 5 or 7, lines 191 to 205 or 565 to 571 for fumaric acid or indicated in Table I, columns 5 or 7, lines 206 to 217 or 577 to 583 for malic acid or indicated in Table I, columns 5 or 7, line 218 or 219 for trihydroxybutyric acid or indicated in Table I, columns 5 or 7, line 220 or line 584 for pyruvic acid or indicated in Table I, columns 5 or 7, lines 221 to 224 or lines 585 to 591 for succinic acid or indicated in Table I, columns 5 or 7, lines 225 or 226 or 592 to 594 for trihydroxybutanoic acid under relaxed hybridization conditions and which code on expression for peptides having the respective fine chemical, i.e. glyceric acid, citramalic acid, fumaric acid, malic acid, pyruvic acid, succinic acid, trihydroxybutyric acid and/or trihydroxybutanoic acid, resp., increasing-activity.

**[0140.0.0.16] to [0146.0.0.16]: see [0140.0.0.0] to [0146.0.0.0]**

**[0147.0.16.16]** Further, the nucleic acid molecule of the invention or used in the process of the invention comprises a nucleic acid molecule, which is a complement of one of the nucleotide sequences of above mentioned nucleic acid molecules or a portion thereof. A nucleic acid molecule which is complementary to one of the nucleotide sequences indicated in Table I, columns 5 or 7, lines 190 to 226 or lines 564 to 594, preferably in Table I B, columns 5 or 7, lines 190 to 226 or lines 564 to 594, is one which is sufficiently complementary to one of said nucleotide sequences such that it can hybridise to one of said nucleotide sequences, thereby forming a stable duplex. Preferably, the hybridisation is performed under stringent hybridization conditions. However, a complement of one of the herein disclosed sequences is preferably a sequence complement thereto according to the base pairing of nucleic acid molecules well known to the skilled person. For example, the bases A and G undergo base pairing with the bases T and U or C, resp. and visa versa. Modifications of the bases can influence the base-pairing partner.

**[0148.0.16.16]** The nucleic acid molecule of the invention comprises a nucleotide sequence which is at least about 30%, 35%, 40% or 45%, preferably at least about 50%, 55%, 60% or 65%, more preferably at least about 70%, 80%, or 90%, and even more preferably at least about 95%, 97%, 98%, 99% or more homologous to a nucleotide sequence indicated in Table I, columns 5 or 7, lines 190 to 226 or lines 564 to 594, preferably in Table I B, columns 5 or 7, lines 190 to 226 or lines 564 to 594, or a portion thereof and preferably has above mentioned activity, in particular having a glyceric acid, citramalic acid, fumaric acid, malic acid, pyruvic acid, succinic acid, trihydroxybutyric acid and/or trihydroxybutanoic acid increasing activity after increasing the activity or an activity of a product of a gene encoding said sequences or their homologs.

**[0149.0.16.16]** The nucleic acid molecule of the invention comprises a nucleotide sequence which hybridizes, preferably hybridizes under stringent conditions as defined herein, to one of the nucleotide sequences indicated in Table I, columns 5 or 7, lines 190 to 226 or lines 564 to 594, preferably in Table IB, columns 7, lines 190 to 226 or lines 564 to 594, or a portion thereof and encodes a protein having above-mentioned activity, e.g. conferring a of glyceric acid, citramalic acid, fumaric acid, malic acid, pyruvic acid, succinic acid, trihydroxybutyric acid and/or trihydroxybutanoic acid increase, resp., and optionally, the activity of protein indicated in Table II, column 5, lines 190 to 226 or lines 564 to 594, preferably in Table II B, columns 7, lines 190 to 226 or lines 564 to 594.

**[00149.1.0.16]** Optionally, in one embodiment, the nucleotide sequence, which hybridises to one of the nucleotide sequences indicated in Table I, columns 5 or 7, lines 190 to 226 or lines 564 to 594, preferably in Table I B, columns 7, lines 190 to 226 or lines 564 to 594, has further one or more of the activities annotated or known for a protein as indicated in Table II, column 3, lines 190 to 226 or lines 564 to 594, preferably in Table I B, columns 7, lines 190 to 226 or lines 564 to 594.

**[0150.0.16.16]** Moreover, the nucleic acid molecule of the invention or used in the process of the invention can comprise only a portion of the coding region of one of the sequences indicated in Table I, columns 5 or 7, lines 190 to 226 or lines 564 to 594, preferably in Table I B, columns 7, lines 190 to 226 or lines 564 to 594 for example a fragment which can be used as a probe or primer or a fragment encoding a biologically active portion of the polypeptide of the present invention or of a polypeptide used in the process of the present invention, i.e. having above-mentioned activity, e.g. conferring an increase of glyceric acid, citramalic acid, fumaric acid, malic acid, pyruvic acid, succinic acid, trihydroxybutyric acid and/or trihydroxybutanoic acid , resp., if its activity is increased. The nucleotide sequences determined from the cloning of the present protein-according-to-the-invention-encoding gene allows for the generation of probes and primers designed for use in identifying and/or cloning its homologues in other cell types and organisms. The probe/primer typically comprises substantially purified oligonucleotide. The oligonucleotide typically comprises a region of nucleotide sequence that hybridizes under stringent conditions to at least about 12, 15 preferably about 20 or 25, more preferably about 40, 50 or 75 consecutive nucleotides of a sense strand of one of the sequences set forth, e.g., as indicated in Table I, columns 5 or 7, lines 190 to 226 or lines 564 to 594, an anti-sense sequence of one of the sequences, e.g., as indicated in Table I, columns 5 or 7, lines 190 to 226 or lines 564 to 594, or naturally occurring mutants thereof. Primers based on a nucleotide of invention can be used in PCR reactions to clone homologues of the polypeptide of the invention or of the polypeptide used in the process of the invention, e.g. as the primers described in the examples of the present invention, e.g. as shown in the examples. A PCR with the primer pairs indicated in Table III, column 7, lines 190 to 226 or lines 564 to 594 will result in a fragment of a polynucleotide sequence as indicated in Table I, columns 5 or 7, lines 190 to 226 or lines 564 to 594 or its gene product. , Preferreid is Table I B, columns 7, lines 190 to 226 or lines 564 to 594.

**[0151.0.0.16]: see [0151.0.0.0]**

**[0152.0.16.16]** The nucleic acid molecule of the invention or used in the process of the invention encodes a polypeptide or portion thereof which includes an amino acid sequence which is sufficiently homologous to an amino acid sequence as indicated in Table II, columns 5 or 7, lines 190 to 226 or lines 564 to 594 such that the protein or portion thereof maintains the ability to participate in the respective fine chemical production, in particular a citramalic acid (line 190) or fumaric acid (lines191 to 205 or 565 to 571) or malic acid (lines 206 to 217 or 577 to 583) or trihydroxybutyric acid (line 218 or 219) or pyruvic acid (line 220 or line 584) or succinic acid (lines 221 to 224 or lines 585 to 591) or trihydroxybutanoic acid (line 225 or 226) increasing activity as mentioned above or as described in the examples in plants or microorganisms is comprised.

**[0153.0.16.16]** As used herein, the language "sufficiently homologous" refers to proteins or portions thereof which have amino acid sequences which include a minimum number of identical or equivalent amino acid residues (e.g., an amino acid residue which has a similar side chain as an amino acid residue in one of the sequences of the polypeptide of the present invention) to an amino acid sequence as indicated in Table II, columns 5 or 7, lines 190 to 226 or lines 564 to 594 such that the protein or portion thereof is able to participate in the increase of the respective fine chemical production. In one embodiment, a protein or portion thereof as indicated in Table II, columns 5 or 7, lines 190 to 226 or lines 564 to 594 has for example an activity of a polypeptide indicated in Table II, column 3, lines 190 to 226 or lines 564 to 594.

**[0154.0.16.16]** In one embodiment, the nucleic acid molecule of the present invention comprises a nucleic acid that encodes a portion of the protein of the present invention. The protein is at least about 30%, 35%, 40%, 45% or 50%, preferably at least about 55%, 60%, 65% or 70%, and more preferably at least about 75%, 80%, 85%, 90%, 91%, 92%, 93% or 94% and most preferably at least about 95%, 97%, 98%, 99% or more homologous to an entire amino acid sequence as indicated in Table II, columns 5 or 7, lines 190 to 226 or lines 564 to 594 and has above-mentioned activity, e.g. conferring preferably the increase of the respective fine chemical.

**[0155.0.0.16] to [0156.0.0.16]: see [0155.0.0.0] to [0156.0.0.0]**

**[0157.0.16.16]** The invention further relates to nucleic acid molecules that differ from one of the nucleotide sequences as indicated in Table I, columns 5 or 7, lines 190 to 226 or lines 564 to 594 (and portions thereof) due to degeneracy of the genetic code and thus encode a polypeptide of the present invention, in particular a polypeptide having above mentioned activity, e.g. conferring an increase in the respective fine chemical in a organism, e.g. as polypeptides comprising the consensus sequence as indicated in Table IV, columns 5 or 7, lines 190 to 226 or lines 564 to 594 or as polypeptides depicted in Table II, columns 5 or 7, lines 190 to 226 or lines 564 to 594 or the functional homologues. Advantageously, the nucleic acid molecule of the invention or used in the process of the invention comprises, or in an other embodiment has, a nucleotide sequence encoding a protein comprising, or in an other embodiment having, an amino acid sequence of a consensus sequences as indicated in Table IV, columns 5 or 7, lines 190 to 226 or lines 564 to 594 or of the polypeptide as indicated in Table II, columns 5 or 7, lines 190 to 226 or lines 564 to 594, resp., or the functional homologues. In a still further embodiment, the nucleic acid molecule of the invention encodes a full length protein which is substantially homologous to an amino acid sequence comprising a consensus sequence as indicated in Table IV, columns 5 or 7, lines 190 to 226 or lines 564 to 594 or of a polypeptide as indicated in Table II, columns 5 or 7, lines 190 to 226 or lines 564 to 594, or the functional homologues thereof. However, in a preferred embodiment, the nucleic acid molecule of the present invention does not consist of a sequence as indicated in Table I, columns 5 or 7, lines 190 to 226 or lines 564 to 594, preferably in Table I A, columns 7, lines 190 to 226 or lines 564 to 594, resp. Preferably, the nucleic acid molecule of the invention is a functional homologue or identical to a nucleic acid molecule indicated in Table I B, columns 5 or 7, ines 190 to 226 or lines 564 to 594.

**[0158.0.0.16]** to **[0160.0.0.16]: see [0158.0.0.0]** to **[0160.0.0.0]**

**[0161.0.16.16]** Accordingly, in another embodiment, a nucleic acid molecule of the invention is at least 15, 20, 25 or 30 nucleotides in length. Preferably, it hybridizes under stringent conditions to a nucleic acid molecule comprising a nucleotide sequence of the nucleic acid molecule of the present invention or used in the process of the present invention, e.g. comprising a sequence as indicated in Table I, columns 5 or 7, lines 190 to 226 or lines 564 to 594. The nucleic acid molecule is preferably at least 20, 30, 50, 100, 250 or more nucleotides in length.

**[0162.0.0.16] see [0162.0.0.0]**

**[0163.0.16.16]** Preferably, a nucleic acid molecule of the invention or used in the process of the invention that hybridizes under stringent conditions to a sequence as indicated in Table I, columns 5 or 7, lines 190 to 226 or lines 564 to 594 corresponds to a naturally-occurring nucleic acid molecule of the invention. As used herein, a "naturally-occurring" nucleic acid molecule refers to an RNA or DNA molecule having a nucleotide sequence that occurs in nature (e.g., encodes a natural protein). Preferably, the nucleic acid molecule encodes a natural protein having above-mentioned activity, e.g. conferring the increase of the amount of the respective fine chemical in a organism or a part thereof, e.g. a tissue, a cell, or a compartment of a cell, after increasing the expression or activity thereof or the activity of a protein of the invention or used in the process of the invention.

**[0164.0.0.16] see [0164.0.0.0]**

**[0165.0.16.16]** For example, nucleotide substitutions leading to amino acid substitutions at "non-essential" amino acid residues can be made in a sequence of the nucleic acid molecule of the invention or used in the process of the invention, e.g. as indicated in Table I, columns 5 or 7, lines 190 to 226 or lines 564 to 594, resp.

**[0166.0.0.16] to [0167.0.0.16]: see [0166.0.0.0] to [0167.0.0.0]**

**[0168.0.16.16]** Accordingly, the invention relates to nucleic acid molecules encoding a polypeptide having above-mentioned activity, e.g. conferring an increase in the respective fine chemical in an organisms or parts thereof that contain changes in amino acid residues that are not essential for said activity. Such polypeptides differ in amino acid sequence from a sequence contained in a sequence as indicated in Table II, columns 5 or 7, lines 190 to 226 or lines 564 to 594, preferably in Table II B, columns 7, lines 190 to 226 or lines 564 to 594, resp., yet retain said activity described herein. The nucleic acid molecule can comprise a nucleotide sequence encoding a polypeptide, wherein the polypeptide comprises an amino acid sequence at least about 50% identical to an amino acid sequence as indicated in Table II, columns 5 or 7, lines 190 to 226 or lines 564 to 594, preferably in Table II B, columns 7, lines 190 to 226 or lines 564 to 594, resp., and is capable of participation in the increase of production of the respective fine chemical after increasing its activity, e.g. its expression. Preferably, the protein encoded by the nucleic acid molecule is at least about 60% identical to a sequence as indicated in Table II, columns 5 or 7, lines 190 to 226 or lines 564 to 594, preferably in Table II B, columns 7, lines 190 to 226 or lines 564 to 594, resp., more preferably at least about 70% identical to one of the sequences as indicated in Table II, columns 5 or 7, lines 190 to 226 or lines 564 to 594, preferably in Table II B, columns 7, lines 190 to 226 or lines 564 to 594, resp., even more preferably at least about 80%, 90%, 95% homologous to a sequence as indicated in Table II, columns 5 or 7, lines 190 to 226 or lines 564 to 594, preferably in Table II B, columns 7, lines 190 to 226 or lines 564 to 594, resp., and most preferably at least about 96%, 97%, 98%, or 99% identical to the sequence as indicated in Table II, columns 5 or 7, lines 190 to 226 or lines 564 to 594 preferably in Table II B, columns 7, lines 190 to 226 or lines 564 to 594,.

**[0169.0.0.16] to [0172.0.0.16]: see [0169.0.0.0] to [0172.0.0.0]**

**[0173.0.16.16]** For example a sequence, which has 80% homology with sequence SEQ ID NO: 17198 at the nucleic acid level is understood as meaning a sequence which, upon comparison with the sequence SEQ ID NO: 17198 by the above Gap program algorithm with the above parameter set, has a 80% homology.

**[0174.0.0.16]: see [0174.0.0.0]**

**[0175.0.16.16]** For example a sequence which has a 80% homology with sequence SEQ ID NO: 17199 at the protein level is understood as meaning a sequence which, upon comparison with the sequence SEQ ID NO: 17199 by the above program algorithm with the above parameter set, has a 80% homology.

**[0176.0.16.16]** Functional equivalents derived from one of the polypeptides as indicated in Table II, columns 5 or 7, lines 190 to 226 or lines 564 to 594, resp., according to the invention by substitution, insertion or deletion have at least 30%, 35%, 40%, 45% or 50%, preferably at least 55%, 60%, 65% or 70% by preference at least 80%, especially preferably at least 85% or 90%, 91%, 92%, 93% or 94%, very especially preferably at least 95%, 97%, 98% or 99% homology with one of the polypeptides as indicated in Table II, columns 5 or 7, lines 190 to 226 or lines 564 to 594, resp., according to the invention and are distinguished by essentially the same properties as a polypeptide as indicated in Table II, columns 5 or 7, lines 190 to 226 or lines 564 to 594, resp.

**[0177.0.16.16]** Functional equivalents derived from a nucleic acid sequence as indicated in Table I, columns 5 or 7, lines 190 to 226 or lines 564 to 594, preferably in Table I B, columns 7, lines 190 to 226 or lines 564 to 594, resp., according to the invention by substitution, insertion or deletion have at least 30%, 35%, 40%, 45% or 50%, preferably at least 55%, 60%, 65% or 70% by preference at least 80%, especially preferably at least 85% or 90%, 91%, 92%, 93% or 94%, very especially preferably at least 95%, 97%, 98% or 99% homology with one of the polypeptides as indicated in Table II, columns 5 or 7, lines 190 to 226 or lines 564 to 594, preferably in Table II B, columns 7, lines 190 to 226 or lines 564 to 594, resp., according to the invention and encode polypeptides having essentially the same properties as a polypeptide as indicated in Table II, columns 5 or 7, lines 190 to 226 or lines 564 to 594, preferably in Table II B, columns 7, lines 190 to 226 or lines 564 to 594, resp.

**[0178.0.0.16] see [0178.0.0.0]**

**[0179.0.16.16]** A nucleic acid molecule encoding a homologous to a protein sequence as indicated in Table II, columns 5 or 7, lines 190 to 226 or lines 564 to 594, preferably in Table I B, columns 7, lines 190 to 226 or lines 564 to 594, resp., can be created by introducing one or more nucleotide substitutions, additions or deletions into a nucleotide sequence of the nucleic acid molecule of the present invention, in particular as indicated in Table I, columns 5 or 7, lines 190 to 226 or lines 564 to 594, resp., such that one or more amino acid substitutions, additions or deletions are introduced into the encoded protein. Mutations can be introduced into the encoding sequences for example into sequences of nucleic acid molecules as indicated in Table I, columns 5 or 7, lines 190 to 226 or lines 564 to 594, resp., by standard techniques, such as site-directed mutagenesis and PCR-mediated mutagenesis.

**[0180.0.0.16] to [0183.0.0.16]: see [0180.0.0.0] to [0183.0.0.0]**

**[0184.0.16.16]** Homologues of the nucleic acid sequences used, with a sequence as indicated in Table I, columns 5 or 7, lines 190 to 226 or lines 564 to 594, preferably in Table I B, columns 7, lines 190 to 226 or lines 564 to 594, resp., or of the nucleic acid sequences derived from a sequences as indicated in Table II, columns 5 or 7, lines 190 to 226 or lines 564 to 594, preferably in Table II B, columns 7, lines 190 to 226 or lines 564 to 594, resp.,, comprise also allelic variants with at least approximately 30%, 35%, 40% or 45% homology, by preference at least approximately 50%, 60% or 70%, more preferably at least approximately 90%, 91%, 92%, 93%, 94% or 95% and even more preferably at least approximately 96%, 97%, 98%, 99% or more homology with one of the nucleotide sequences shown or the abovementioned derived nucleic acid sequences or their homologues, derivatives or analogues or parts of these. Allelic variants encompass in particular functional variants which can be obtained by deletion, insertion or substitution of nucleotides from the sequences shown, preferably from a sequence as indicated in Table I, columns 5 or 7, lines 190 to 226 or lines 564 to 594, resp., or from the derived nucleic acid sequences, the intention being, however, that the enzyme activity or the biological activity of the resulting proteins synthesized is advantageously retained or increased.

**[0185.0.16.16]** In one embodiment of the present invention, the nucleic acid molecule of the invention or used in the process of the invention comprises one or more sequences as indicated in Table I, columns 5 or 7, lines 190 to 226 or lines 564 to 594, preferably in Table I B, columns 7, lines 190 to 226 or lines 564 to 594, resp. In one embodiment, it is preferred that the nucleic acid molecule comprises as little as possible other nucleotides not shown in any one of sequences as indicated in Table I, columns 5 or 7, lines 190 to 226 or lines 564 to 594, preferably in Table I B, columns 7, lines 190 to 226 or lines 564 to 594, resp. In one embodiment, the nucleic acid molecule comprises less than 500, 400, 300, 200, 100, 90, 80, 70, 60, 50 or 40 further nucleotides. In a further embodiment, the nucleic acid molecule comprises less than 30, 20 or 10 further nucleotides. In one embodiment, a nucleic acid molecule used in the process of the invention is identical to a sequence as indicated in Table I, columns 5 or 7, lines 190 to 226 or lines 564 to 594, preferably in Table I B, columns 7, lines 190 to 226 or lines 564 to 594,resp.

**[0186.0.16.16]** Also preferred is that one or more nucleic acid molecule(s) used in the process of the invention encodes a polypeptide comprising a sequence as indicated in Table II, columns 5 or 7, lines 190 to 226 or lines 564 to 594, preferably in Table II B, columns 7, lines 190 to 226 or lines 564 to 594,resp. In one embodiment, the nucleic acid molecule encodes less than 150, 130, 100, 80, 60, 50, 40 or 30 further amino acids. In a further embodiment, the encoded polypeptide comprises less than 20, 15, 10, 9, 8, 7, 6 or 5 further amino acids. In one embodiment, the encoded polypeptide used in the process of the invention is identical to the sequences as indicated in Table II, columns 5 or 7, lines 190 to 226 or lines 564 to 594, preferably in Table II B, columns 7, lines 190 to 226 or lines 564 to 594,resp.

**[0187.0.16.16]** In one embodiment, a nucleic acid molecule of the invention or used in the process encodes a polypeptide comprising the sequence as indicated in Table II, columns 5 or 7, lines 190 to 226 or lines 564 to 594, preferably in Table II B, columns 7, lines 190 to 226 or lines 564 to 594, resp., comprises less than 100 further nucleotides. In a further embodiment, said nucleic acid molecule comprises less than 30 further nucleotides. In one embodiment, the nucleic acid molecule used in the process is identical to a coding sequence encoding a sequences as indicated in Table II, columns 5 or 7, lines 190 to 226 or lines 564 to 594, preferably in Table II B, columns 7, lines 190 to 226 or lines 564 to 594, resp.

**[0188.0.16.16]** Polypeptides (= proteins), which still have the essential enzymatic activity of the polypeptide of the present invention conferring an increase of the respective fine chemical i.e. whose activity is essentially not reduced, are polypeptides with at least 10% or 20%, by preference 30% or 40%, especially preferably 50% or 60%, very especially preferably 80% or 90 or more of the wild type biological activity or enzyme activity, advantageously, the activity is essentially not reduced in comparison with the activity of a polypeptide as indicated in Table II, columns 5 or 7, lines 190 to 226 or lines 564 to 594, resp., preferably compared to a sequence as indicated in preferably in Table II, columns 5 or 7, lines 190 to 226 or lines 564 to 594,, and is expressed under identical conditions. In one embodiment, the polypeptide of the invention is a homolog consisting of or comprising the sequence as indicated in Table I B, columns 7, lines 190 to 226 or lines 564 to 594,.

**[0189.0.16.16]** Homologues of a sequence as indicated in Table I, columns 5 or 7, lines 190 to 226 or lines 564 to 594, resp., or of a derived sequences as indicated in Table II, columns 5 or 7, lines 190 to 226 or lines 564 to 594, resp., also mean truncated sequences, cDNA, single-stranded DNA or RNA of the coding and noncoding DNA sequence. Homologues of said sequences are also understood as meaning derivatives, which comprise noncoding regions such as, for example, UTRs, terminators, enhancers or promoter variants. The promoters upstream of the nucleotide sequences stated can be modified by one or more nucleotide substitution(s), insertion(s) and/or deletion(s) without, however, interfering with the functionality or activity either of the promoters, the open reading frame (=ORF) or with the 3'-regulatory region such as terminators or other 3'regulatory regions, which are far away from the ORF. It is furthermore possible that the activity of the promoters is increased by modification of their sequence, or that they are replaced completely by more active promoters, even promoters from heterologous organisms. Appropriate promoters are known to the person skilled in the art and are mentioned herein below.

**[0190.0.0.16]: see [0190.0.0.0]**

**[0191.0.0.16] see [0191.0.0.0]**

**[0191.0.16.16]** The organisms or part thereof produce according to the herein mentioned process of the invention an increased level of free and/or bound the respective fine chemical compared to said control or selected organisms or parts thereof.

**[0192.0.0.16] to [0203.0.0.16]: see [0192.0.0.0] to [0203.0.0.0]**

**[0204.0.16.16]** Accordingly, in one embodiment, the invention relates to a nucleic acid molecule, which comprises a nucleic acid molecule selected from the group consisting of:
a) nucleic acid molecule encoding, preferably at least the mature form, of a polypeptide as indicated in Table II, columns 5 or 7, lines 190 to 226 or lines 564 to 594, preferably in Table II B, columns 7, lines 190 to 226 or lines 564 to 594, resp.; or a fragment thereof conferring an increase in the amount of the respective fine chemical, i.e. glyceric acid (lines 572 to 576), citramalic acid (line 190 or 564) or fumaric acid (lines 191 to 205 or 565 to 571) or malic acid (lines 206 to 217 or 577 to 583) or trihydroxybutyric acid (line 218 or 219) or pyruvic acid (line 220 or line 584) or succinic acid (lines 221 to 224 or lines 585 to 591) or trihydroxybutanoic acid (lines 225 or 226 or 592 to 594), resp., in an organism or a part thereof
b) nucleic acid molecule comprising, preferably at least the mature form, of a nucleic acid molecule as indicated in Table I, columns 5 or 7, lines 190 to 226 or lines 564 to 594, preferably in Table I B, columns 7, lines 190 to 226 or lines 564 to 594, resp., or a fragment thereof conferring an increase in the amount of the respective fine chemical in an organism or a part thereof;
c) nucleic acid molecule whose sequence can be deduced from a polypeptide sequence encoded by a nucleic acid molecule of (a) or (b) as result of the degeneracy of the genetic code and conferring an increase in the amount of the respective fine chemical in an organism or a part thereof;
d) nucleic acid molecule encoding a polypeptide whose sequence has at least 50% identity with the amino acid sequence of the polypeptide encoded by the nucleic acid molecule of (a) to (c) and conferring an increase in the amount of the respective fine chemical in an organism or a part thereof;
e) nucleic acid molecule which hybridizes with a nucleic acid molecule of (a) to (c) under stringent hybridisation conditions and conferring an increase in the amount of the respective fine chemical in an organism or a part thereof;
f) nucleic acid molecule encoding a polypeptide, the polypeptide being derived by substituting, deleting and/or adding one or more amino acids of the amino acid sequence of the polypeptide encoded by the nucleic acid molecules (a) to (d), preferably to (a) to (c), and conferring an increase in the amount of the respective fine chemical in an organism or a part thereof;
g) nucleic acid molecule encoding a fragment or an epitope of a polypeptide which is encoded by one of the nucleic acid molecules of (a) to (e), preferably to (a) to (c) and conferring an increase in the amount of the respective fine chemical in an organism or a part thereof;
h) nucleic acid molecule comprising a nucleic acid molecule which is obtained by amplifying a cDNA library or a genomic library using primers or primer pairs as indicated in Table III, columns 5 or 7, lines 190 to 226 or lines 564 to 594 and conferring an increase in the amount of the respective fine chemical, i.e. glyceric acid (lines 572 to 576), citramalic acid (line 190 or 564) or fumaric acid (lines 191 to 205 or 565 to 571) or malic acid (lines 206 to 217 or 577 to 583) or trihydroxybutyric acid (line 218 or 219) or pyruvic acid (line 220 or line 584) or succinic acid (lines 221 to 224 or lines 585 to 591) or trihydroxybutanoic acid (lines 225 or 226 or 592 to 594), resp., in an organism or a part thereof;
i) nucleic acid molecule encoding a polypeptide which is isolated, e.g. from a expression library, with the aid of monoclonal antibodies against a polypeptide encoded by one of the nucleic acid molecules of (a) to (g), preferably to (a) to (c) and conferring an increase in the amount of the respective fine chemical in an organism or a part thereof;
j) nucleic acid molecule which encodes a polypeptide comprising a consensus sequence as indicated in Table IV, columns 7, lines 190 to 226 or lines 564 to 594 and conferring an increase in the amount of the respective fine chemical, i.e. glyceric acid (lines 572 to 576), citramalic acid (line 190 or 564) or fumaric acid (lines 191 to 205 or 565 to 571) or malic acid (lines 206 to 217 or 577 to 583) or trihydroxybutyric acid (line 218 or 219) or pyruvic acid (line 220 or line 584) or succinic acid (lines 221 to 224 or lines 585 to 591) or trihydroxybutanoic acid (lines 225 or 226 or 592 to 594), resp., in an organism or a part thereof;
k) nucleic acid molecule encoding the amino acid sequence of a polypeptide encoding a domain of a polypeptide as indicated in Table II, columns 5 or 7, lines 190 to 226 or lines 564 to 594, preferably in Table II B, columns 7, lines 190 to 226 or lines 564 to 594, resp., and conferring an increase in the amount of the respective fine chemical, i.e. glyceric acid (lines 572 to 576), citramalic acid (line 190 or 564) or fumaric acid (lines 191 to 205 or 565 to 571) or malic acid (lines 206 to 217 or 577 to 583) or trihydroxybutyric acid (line 218 or 219) or pyruvic acid (line 220 or line 584) or succinic acid (lines 221 to 224 or lines 585 to 591) or trihydroxybutanoic acid (lines 225 or 226 or 592 to 594), resp., in an organism or a part thereof; and
l) nucleic acid molecule which is obtainable by screening a suitable nucleic acid library under stringent hybridization conditions with a probe comprising one of the sequences of the nucleic acid molecule of (a) to (k) or with a fragment of at least 15 nt, preferably 20 nt, 30 nt, 50 nt, 100 nt, 200 nt or 500 nt of the nucleic acid molecule characterized in (a) to (h) or of a nucleic acid molecule as indicated in Table I, columns 5 or 7, lines 190 to 226 or lines 564 to 594, preferably in Table I B, columns 7, lines 190 to 226 or lines 564 to 594, resp., or a nucleic acid molecule encoding, preferably at least the mature form of, a polypeptide as indicated in Table II, columns 5 or 7, lines 190 to 226 or lines 564 to 594, resp., and conferring an increase in the amount of the respective fine chemical in an organism or a part thereof; or which encompasses a sequence which is complementary thereto;
whereby, preferably, the nucleic acid molecule according to (a) to (I) distinguishes over a sequence as indicated in Table IA, columns 5 or 7, lines 190 to 226 or lines 564 to 594, resp., by one or more nucleotides. In one embodiment, the nucleic acid molecule of the invention does not consist of the sequence as indicated in Table I A, columns 5 or 7, lines 190 to 226 or lines 564 to 594, resp. In an other embodiment, the nucleic acid molecule of the present invention is at least 30 % identical and less than 100%, 99,999%, 99,99%, 99,9% or 99% identical to a sequence as indicated in Table I A, columns 5 or 7, lines 190 to 226 or lines 564 to 594, resp. In a further embodiment the nucleic acid molecule does not encode a polypeptide sequence as indicated in Table II A, columns 5 or 7, lines 190 to 226 or lines 564 to 594, resp. Accordingly, in one embodiment, the nucleic acid molecule of the present invention encodes in one embodiment a polypeptide which differs at least in one or more amino acids from a polypeptide indicated in Table II A, columns 5 or 7, lines 190 to 226 or lines 564 to 594 does not encode a protein of a sequence as indicated in Table II A, columns 5 or 7, lines 190 to 226 or lines 564 to 594. Accordingly, in one embodiment, the protein encoded by a sequences of a nucleic acid according to (a) to (I) does not consist of a sequence as indicated in Table II A, columns 5 or 7, lines 190 to 226 or lines 564 to 594. In a further embodiment, the protein of the present invention is at least 30 % identical to a protein sequence indicated in Table II A, columns 5 or 7, lines 190 to 226 or lines 564 to 594 and less than 100%, preferably less than 99,999%, 99,99% or 99,9%, more preferably less than 99%, 985, 97%, 96% or 95% identical to a sequence as indicated in Table II A, columns 5 or 7, lines 190 to 226 or lines 564 to 594.
In an other embodiment, however, the nucleic acid molecule according to (a) to (I) distinguishes over a sequence as indicated in Table I B, columns 5 or 7, lines 190 to 226 or lines 564 to 594, resp., by one or more nucleotides. In one embodiment, the nucleic acid molecule of the invention does not consist of the sequence as indicated in Table I B, columns 5 or 7, lines 190 to 226 or lines 564 to 594, resp. In an other embodiment, the nucleic acid molecule of the present invention is at least 30 % identical and less than 100%, 99,999%, 99,99%, 99,9% or 99% identical to a sequence as indicated in Table I B, columns 5 or 7, lines 190 to 226 or lines 564 to 594, resp. In a further embodiment the nucleic acid molecule does not encode a polypeptide sequence as indicated in Table II B, columns 5 or 7, lines 190 to 226 or lines 564 to 594, resp. Accordingly, in one embodiment, the nucleic acid molecule of the present invention encodes in one embodiment a polypeptide which differs at least in one or more amino acids from a polypeptide indicated in Table II B, columns 5 or 7, lines 190 to 226 or lines 564 to 594 does not encode a protein of a sequence as indicated in Table II B, columns 5 or 7, lines 190 to 226 or lines 564 to 594. Accordingly, in one embodiment, the protein encoded by a sequences of a nucleic acid according to (a) to (I) does not consist of a sequence as indicated in Table II B, columns 5 or 7, lines 190 to 226 or lines 564 to 594. In a further embodiment, the protein of the present invention is at least 30 % identical to a protein sequence indicated in Table II B, columns 5 or 7, lines 190 to 226 or lines 564 to 594 and less than 100%, preferably less than 99,999%, 99,99% or 99,9%, more preferably less than 99%, 98%, 97%, 96% or 95% identical to a sequence as indicated in Table II B, columns 5 or 7, lines 190 to 226 or lines 564 to 594.

**[0205.0.0.16] to [0206.0.0.16]: see [0205.0.0.0] to [0206.0.0.0]**

**[0207.0.16.16]** As described herein, the nucleic acid construct can also comprise further genes, which are to be introduced into the organisms or cells. It is possible and advantageous to introduce into, and express in, the host organisms regulatory genes such as genes for inductors, repressors or enzymes, which, owing to their enzymatic activity, engage in the regulation of one or more genes of a biosynthetic pathway. These genes can be of heterologous or homologous origin. Moreover, further biosynthesis genes may advantageously be present, or else these genes may be located on one or more further nucleic acid constructs. Genes, which are advantageously employed as biosynthesis genes, are genes of the glutamic acid metabolism, the phosphoenolpyruvate metabolism, the amino acid metabolism, of glycolysis, of the tricarboxylic acid metabolism or their combinations. As described herein, regulator sequences or factors can have a positive effect on preferably the gene expression of the genes introduced, thus increasing it. Thus, an enhancement of the regulator elements may advantageously take place at the transcriptional level by using strong transcription signals such as promoters and/or enhancers. In addition, however, an enhancement of translation is also possible, for example by increasing mRNA stability or by inserting a translation enhancer sequence.

**[0208.0.0.16] to [0226.0.0.16]: see [0208.0.0.0] to [0226.0.0.0]**

**[0227.0.16.16]** The abovementioned nucleic acid molecules can be cloned into the nucleic acid constructs or vectors according to the invention in combination together with further genes, or else different genes are introduced by transforming several nucleic acid constructs or vectors (including plasmids) into a host cell, advantageously into a plant cell or a microorganisms.
In addition to a sequence indicated in Table I, columns 5 or 7, lines 190 to 226 or lines 564 to 594 or its derivatives, it is advantageous to express and/or mutate further genes in the organisms. Especially advantageously, additionally at least one further gene of the glutamic acid or phosphoenolpyruvate metabolic pathway, is expressed in the organisms such as plants or microorganisms. It is also possible that the regulation of the natural genes has been modified advantageously so that the gene and/or its gene product is no longer subject to the regulatory mechanisms which exist in the organisms. This leads to an increased synthesis of the fine chemicals desired since, for example, feedback regulations no longer exist to the same extent or not at all. In addition it might be advantageously to combine one or more of the sequences indicated in Table I, columns 5 or 7, lines 190 to 226 or lines 564 to 594, resp., with genes which generally support or enhances to growth or yield of the target organism, for example genes which lead to faster growth rate of microorganisms or genes which produces stress-, pathogen, or herbicide resistant plants.

**[0228.0.16.16]** In a further embodiment of the process of the invention, therefore, organisms are grown, in which there is simultaneous overexpression of at least one nucleic acid or one of the genes which code for proteins directly or indirectly involved in the glutamic acid or phosphoenolpyruvate metabolism.

**[0229.0.0.0] %**

**[0230.0.0.16] see [230.0.0.0]**

**[0231.0.16.16]** In a further advantageous embodiment of the process of the invention, the organisms used in the process are those in which simultaneously a glyceric acid, citramalic acid, fumaric acid, malic acid, pyruvic acid, succinic acid, trihydroxybutyric acid and/or trihydroxybutanoic acid degrading protein is attenuated, in particular by reducing the rate of expression of the corresponding gene.

**[0232.0.0.16] to [0276.0.0.16]: see [0232.0.0.0] to [0276.0.0.0]**

**[0277.0.16.16]** The respective fine chemical produced can be isolated from the organism by methods with which the skilled worker are familiar, for example via extraction, salt precipitation, and/or different chromatography methods. The process according to the invention can be conducted batchwise, semibatchwise or continuously.

**[0278.0.0.16] to [0282.0.0.16]: see [0278.0.0.0] to [0282.0.0.0]**

**[0283.0.16.16]** Moreover, native polypeptide conferring the increase of the respective fine chemical in an organism or part thereof can be isolated from cells, for example using the antibody of the present invention as described below, e.g. an antibody against a protein as indicated in Table II, column 3, lines 190 to 226 or lines 564 to 594, resp., or an antibody against a polypeptide as indicated in Table II, columns 5 or 7, lines 190 to 226 or lines 564 to 594, resp., or an antigenic part thereof which can be produced by standard techniques utilizing polypeptides comprising or consisting of above mentioned sequences, e.g. the polypeptid of the present invention or fragment thereof. Preferred are monoclonal antibodies specifically binding to polypeptide as indicated in Table II, columns 5 or 7, lines 190 to 226 or lines 564 to 594.

**[0284.0.0.16] see [0284.0.0.0]**

**[0285.0.16.16]** In one embodiment, the present invention relates to a polypeptide having a sequence as indicated in Table II, columns 5 or 7, lines 190 to 226 or lines 564 to 594, resp., or as coded by a nucleic acid molecule as indicated in Table I, columns 5 or 7, lines 190 to 226 or lines 564 to 594, resp., or functional homologues thereof.

**[0286.0.16.16]** In one advantageous embodiment, in the method of the present invention the activity of a polypeptide is increased which comprises or consists of a consensus sequence as indicated in Table IV, columns 7, lines 190 to 226 or lines 564 to 594 and in one another embodiment, the present invention relates to a polypeptide comprising or consisting of a consensus sequence as indicated in Table IV, columns 7, lines 190 to 226 or lines 564 to 594 whereby 20 or less, preferably 15 or 10, preferably 9, 8, 7, or 6, more preferred 5 or 4, even more preferred 3, even more preferred 2, even more preferred 1, most preferred 0 of the amino acids positions indicated can be replaced by any amino acid or, in an further embodiment, can be replaced and/or absent. In one embodiment, the present invention relates to the method of the present invention comprising a poylpeptide or to a polypeptide comprising more than one consensus sequences as indicated in Table IV, column 7, ines 190 to 226 or lines 564 to 594..

**[0287.0.0.16] to [0289.0.0.16]: see [0287.0.0.0] to [0289.0.0.0]**

**[00290.0.16.16]** The alignment was performed with the Software AlignX (sept 25, 2002) a component of Vector NTI Suite 8.0, InforMax™,Invitrogen™ life science software, U.S. Main Office, 7305 Executive Way,Frederick, MD 21704,USA with the following settings: For pair wise alignments: gap opening penalty: 10,0; gap extension penalty 0,1. For multiple alignments: Gap opening penalty: 10,0; Gap extension penalty: 0,1; Gap seperation penalty range: 8; Residue substitution matrix: blosum62; Hydrophilic residues: G P S N D Q E K R; Transition weighting: 0,5; Consensus calculation options: Residue fraction for consensus: 0,9. Presettings were selected to allow also for the alignment of conserved amino acids

**[0291.0.16.16]** In one advantageous embodiment, the method of the present invention comprises the increasing of a polypeptide comprising or consisting of plant or microorganism specific consensus sequences. Accordingly, in one embodiment, the present invention relates to a polypeptide comprising or consisting of plant or microorganism specific consensus sequences.
In one embodiment, said polypeptide of the invention distinguishes over a sequence as indicated in Table II A or II B, columns 5 or 7, lines 190 to 226 or lines 564 to 594, resp., by one or more amino acids. In one embodiment, polypeptide distinguishes from a sequence as indicated in Table II A or II B, columns 5 or 7, lines 190 to 226 or lines 564 to 594, resp., by more than 5, 6, 7, 8 or 9 amino acids, preferably by more than 10, 15, 20, 25 or 30 amino acids, even more preferred are more than 40, 50, or 60 amino acids and, preferably, the sequence of the polypeptide of the invention distinguishes from a sequence as indicated in Table II A or II B, columns 5 or 7, lines 190 to 226 or lines 564 to 594, resp., by not more than 80% or 70% of the amino acids, preferably not more than 60% or 50%, more preferred not more than 40% or 30%, even more preferred not more than 20% or 10%. In an other embodiment, said polypeptide of the invention does not consist of a sequence as indicated in Table II A or II B, columns 5 or 7, lines 190 to 226 or lines 564 to 594.

**[0292.0.0.16] see [0292.0.0.0]**

**[0293.0.16.16]** In one embodiment, the invention relates to a polypeptide conferring an increase in the fine chemical in an organism or part being encoded by the nucleic acid molecule of the invention or by a nucleic acid molecule used in the process of the invention. In one embodiment, the polypeptide of the invention has a sequence which distinguishes from a sequence as indicated in Table II A or II B, columns 5 or 7, lines 190 to 226 or lines 564 to 594, resp., by one or more amino acids. In an other embodiment, said polypeptide of the invention does not consist of the sequence as indicated in Table II A or II B, columns 5 or 7, lines 190 to 226 or lines 564 to 594, resp. In a further embodiment, said polypeptide of the present invention is less than 100%, 99,999%, 99,99%, 99,9% or 99% identical. In one embodiment, said polypeptide does not consist of the sequence encoded by a nucleic acid molecules as indicated in Table I A or I B, columns 5 or 7, lines 190 to 226 or lines 564 to 594, resp.

**[0294.0.16.16]** In one embodiment, the present invention relates to a polypeptide having an activity of a protein as indicated in Table II, column 3, lines 190 to 226 or lines 564 to 594, resp., which distinguishes over a sequence as indicated in Table II A or II B, columns 5 or 7, lines 190 to 226 or lines 564 to 594, resp., by one or more amino acids, preferably by more than 5, 6, 7, 8 or 9 amino acids, preferably by more than 10, 15, 20, 25 or 30 amino acids, even more preferred are more than 40, 50, or 60 amino acids but even more preferred by less than 70% of the amino acids, more preferred by less than 50%, even more preferred my less than 30% or 25%, more preferred are 20% or 15%, even more preferred are less than 10%.

**[0295.0.0.16] to [0296.0.0.16]: see [0295.0.0.0] to [0296.0.0.0]**

**[0297.0.0.16] see [0297.0.0.0]**

**[00297.1.0.16]** Non-polypeptide of the invention-chemicals are e.g. polypeptides having not the activity and/or amino acid sequence of a polypeptide indicated in Table II, columns 3, 5 or 7, lines 190 to 226 or lines 564 to 594

**[0298.0.16.16]** A polypeptide of the invention can participate in the process of the present invention. The polypeptide or a portion thereof comprises preferably an amino acid sequence which is sufficiently homologous to an amino acid sequence as indicated in Table II, columns 5 or 7, lines 190 to 226 or lines 564 to 594, resp. The portion of the protein is preferably a biologically active portion as described herein. Preferably, the polypeptide used in the process of the invention has an amino acid sequence identical to a sequence as indicated in Table II, columns 5 or 7, lines 190 to 226 or lines 564 to 594, resp.

**[0299.0.16.16]** Further, the polypeptide can have an amino acid sequence which is encoded by a nucleotide sequence which hybridizes, preferably hybridizes under stringent conditions as described above, to a nucleotide sequence of the nucleic acid molecule of the present invention. Accordingly, the polypeptide has an amino acid sequence which is encoded by a nucleotide sequence that is at least about 35%, 40%, 45%, 50%, 55%, 60%, 65% or 70%, preferably at least about 75%, 80%, 85% or 90, and more preferably at least about 91%, 92%, 93%, 94% or 95%, and even more preferably at least about 96%, 97%, 98%, 99% or more homologous to one of the nucleotide sequence as indicated in Table I, columns 5 or 7, lines 190 to 226 or lines 564 to 594, resp. The preferred polypeptide of the present invention preferably possesses at least one of the activities according to the invention and described herein. A preferred polypeptide of the present invention includes an amino acid sequence encoded by a nucleotide sequence which hybridizes, preferably hybridizes under stringent conditions, to a nucleotide sequence as indicated in Table I, columns 5 or 7, lines 190 to 226 or lines 564 to 594, resp., or which is homologous thereto, as defined above.

**[0300.0.16.16]** Accordingly the polypeptide of the present invention can vary from a sequence as indicated in Table II, columns 5 or 7, lines 190 to 226 or lines 564 to 594, resp., in amino acid sequence due to natural variation or mutagenesis, as described in detail herein. Accordingly, the polypeptide comprise an amino acid sequence which is at least about 35%, 40%, 45%, 50%, 55%, 60%, 65% or 70%, preferably at least about 75%, 80%, 85% or 90, and more preferably at least about 91%, 92%, 93%, 94% or 95%, and most preferably at least about 96%, 97%, 98%, 99% or more homologous to an entire amino acid sequence of as indicated in Table II A or B, columns 5 or 7, lines 190 to 226 or lines 564 to 594, resp.

**[0301.0.0.16] see [0301.0.0.0]**

**[0302.0.16.16]** Biologically active portions of an polypeptide of the present invention include peptides comprising amino acid sequences derived from the amino acid sequence of the polypeptide of the present invention or used in the process of the present invention, e.g., an amino acid sequence as indicated in Table II, columns 5 or 7, lines 190 to 226 or lines 564 to 594, resp., or the amino acid sequence of a protein homologous thereto, which include fewer amino acids than a full length polypeptide of the present invention or used in the process of the present invention or the full length protein which is homologous to an polypeptide of the present invention or used in the process of the present invention depicted herein, and exhibit at least one activity of polypeptide of the present invention or used in the process of the present invention.

**[0303.0.0.16] see [0303.0.0.0]**

**[0304.0.16.16]** Manipulation of the nucleic acid molecule of the invention or the nucleic acid molecule used in the method of the invention may result in the production of a protein having essentially the activity of the polypeptides as indicated in Table II, column 3, lines 190 to 226 or lines 564 to 594 but having differences in the sequence from said wild-type protein. These proteins may be improved in efficiency or activity, may be present in greater numbers in the cell than is usual, or may be decreased in efficiency or activity in relation to the wild type protein.

**[0305.0.0.16] to [0308.0.0.16]: see [0305.0.0.0] to [0308.0.0.0]**

**[00306.1.0.0]** In one embodiment, the compound is a composition comprising the repective fine chemical, i.e. said organic acids, or a recovered fine chemical, i.e. said organic acid free or in protein-bound form.

**[0309.0.16.16]** In one embodiment, a reference to a protein (= polypeptide) of the invention or as indicated in Table II, columns 5 or 7, lines 190 to 226 or lines 564 to 594, resp., refers to a polypeptide having an amino acid sequence corresponding to the polypeptide of the invention or used in the process of the invention, whereas an "other polypeptide" not being indicated in Table II, columns 5 or 7, lines 190 to 226 or lines 564 to 594, resp., refers to a polypeptide having an amino acid sequence corresponding to a protein which is not substantially homologous to a polypeptide of the invention, preferably which is not substantially homologous to a polypeptide as indicated in Table II, columns 5 or 7, lines 190 to 226 or lines 564 to 594, resp., e.g., a protein which does not confer the activity described herein or annotated or known for as indicated in Table II, column 3, lines 190 to 226 or lines 564 to 594, resp., and which is derived from the same or a different organism. In one embodiment, an "other polypeptide" not being indicated in Table II, columns 5 or 7, lines 190 to 226 or lines 564 to 594, resp., does not confer an increase of the respective fine chemical in an organism or part thereof. In one embodiment, a "non-polypeptide of the invention" or "other polypeptide" not being indicated in Table II, columns 5 or 7, lines 190 to 226 or lines 564 to 594, resp., does not confer an increase of the respective fine chemical in an organism or part thereof.

**[0310.0.0.16] to [0334.0.0.16] but [0318.0.18.18] : see [0310.0.0.0] to [0334.0.0.0]**

**[0318.0.18.18]** In an especially preferred embodiment, the polypeptide according to the invention furthermore also does not have the sequences of those proteins which are encoded by a sequences shown in table II, lines 190 to 226 or lines 564 to 594.

**[0335.0.16.16]** The dsRNAi method has proved to be particularly effective and advantageous for reducing the expression of a nucleic acid sequences as indicated in Table I, columns 5 or 7, lines 190 to 226 or lines 564 to 594 , resp., and/or homologs thereof. As described inter alia in WO 99/32619, dsRNAi approaches are clearly superior to traditional antisense approaches. The invention therefore furthermore relates to double-stranded RNA molecules (dsRNA molecules) which, when introduced into an organism, advantageously into a plant (or a cell, tissue, organ or seed derived there from), bring about altered metabolic activity by the reduction in the expression of a nucleic acid sequences as indicated in Table I, columns 5 or 7, lines 190 to 226 or lines 564 to 594, resp., and/or homologs thereof. In a double-stranded RNA molecule for reducing the expression of a protein encoded by a nucleic acid sequence as indicated in Table I, columns 5 or 7, lines 190 to 226 or lines 564 to 594, resp., and/or homologs thereof, one of the two RNA strands is essentially identical to at least part of a nucleic acid sequence, and the respective other RNA strand is essentially identical to at least part of the complementary strand of a nucleic acid sequence.

**[0336.0.0.16] to [0342.0.0.16]: see [0336.0.0.0] to [0342.0.0.0]**

**[0343.0.16.16]** As has already been described, 100 % sequence identity between the dsRNA and a gene transcript of a nucleic acid sequence as indicated in Table I, columns 5 or 7, lines 190 to 226 or lines 564 to 594, resp., or it's homolog is not necessarily required in order to bring about effective reduction in the expression. The advantage is, accordingly, that the method is tolerant with regard to sequence deviations as may be present as a consequence of genetic mutations, polymorphisms or evolutionary divergences. Thus, for example, using the dsRNA, which has been generated starting from a sequence as indicated in Table I, columns 5 or 7, lines 190 to 226 or lines 564 to 594, resp., or homologs thereof of the one organism, may be used to suppress the corresponding expression in another organism.

**[0344.0.0.16] to [0361.0.0.16]: see [0344.0.0.0] to [0361.0.0.0]**

**[0362.0.16.16]** Accordingly the present invention relates to any cell transgenic for any nucleic acid characterized as part of the invention, e.g. conferring the increase of the respective fine chemical in a cell or an organism or a part thereof, e.g. the nucleic acid molecule of the invention, the nucleic acid construct of the invention, the antisense molecule of the invention, the vector of the invention or a nucleic acid molecule encoding the polypeptide of the invention, e.g. the polypeptide as indicated in Table II, columns 5 or 7, lines 190 to 226 or lines 564 to 594, resp., e.g. encoding a polypeptide having protein activity, as indicated in Table II, columns 3, lines 190 to 226 or lines 564 to 594, resp., Due to the abovementioned activity the respective fine chemical content in a cell or an organism is increased. For example, due to modulation or manipulation, the cellular activity of the polypeptide of the invention or nucleic acid molecule of the invention or the nucleic acid molecule or polypeptide used in the method of the invention is increased, e.g. due to an increased expression or specific activity of the subject matters of the invention in a cell or an organism or a part thereof. Transgenic for a polypeptide having an activity of a polypeptide as indicated in Table II, columns 5 or 7, lines 190 to 226 or lines 564 to 594, resp., means herein that due to modulation or manipulation of the genome, an activity as annotated for a polypeptide as indicated in Table II, column 3, lines 190 to 226 or lines 564 to 594, e.g. having a sequence as indicated in Table II, columns 5 or 7, lines 190 to 226 or lines 564 to 594, resp., is increased in a cell or an organism or a part thereof. Examples are described above in context with the process of the invention.

**[0363.0.0.16] see [0363.0.0.0]**

**[0364.0.16.16]** A naturally occurring expression cassette - for example the naturally occurring combination of a promoter of a gene encoding a polypeptide of the invention as indicated in Table II, column 3, lines 190 to 226 or lines 564 to 594, resp. with the corresponding protein-encoding sequence as indicated in Table I, column 5, lines 190 to 226 or lines 564 to 594, resp., becomes a transgenic expression cassette when it is modified by non-natural, synthetic "artificial" methods such as, for example, mutagenization. Such methods have been described (US 5,565,350; WO 00/15815; also see above).

**[0365.0.0.16] to [0373.0.0.16]: see [0365.0.0.0] to [0373.0.0.0]**

**[0374.0.16.16]** Transgenic plants comprising the respective fine chemical synthesized in the process according to the invention can be marketed directly without isolation of the compounds synthesized. In the process according to the invention, plants are understood as meaning all plant parts, plant organs such as leaf, stalk, root, tubers or seeds or propagation material or harvested material or the intact plant. In this context, the seed encompasses all parts of the seed such as the seed coats, epidermal cells, seed cells, endosperm or embryonic tissue, glyceric acid, citramalic acid, fumaric acid, malic acid, pyruvic acid, succinic acid, trihydroxybutyric acid and/or trihydroxybutanoic acid, in particular the respective fine chemical, produced in the process according to the invention may, however, also be isolated from the plant in the form of their free glyceric acid, citramalic acid, fumaric acid, malic acid, pyruvic acid, succinic acid, trihydroxybutyric acid and/or trihydroxybutanoic acid, in particular the free respective fine chemical, or bound in or to compounds or moieties, like glucosides, e.g. diglucosides. The respective fine chemical produced by this process can be harvested by harvesting the organisms either from the culture in which they grow or from the field. This can be done via expressing, grinding and/or extraction, salt precipitation and/or ion-exchange chromatography or other chromatographic methods of the plant parts, preferably the plant seeds, plant fruits, plant tubers and the like.

**[0375.0.0.16] to [0376.0.0.16]: see [0375.0.0.0] to [0376.0.0.0]**

**[0377.0.16.16]** Accordingly, the present invention relates also to a process whereby the produced glyceric acid, citramalic acid, fumaric acid, malic acid, pyruvic acid, succinic acid, trihydroxybutyric acid and/or trihydroxybutanoic acid is isolated.

**[0378.0.16.16]** In this manner, more than 50% by weight, advantageously more than 60% by weight, preferably more than 70% by weight, especially preferably more than 80% by weight, very especially preferably more than 90% by weight, of the glyceric acid, citramalic acid, fumaric acid, malic acid, pyruvic acid, succinic acid, trihydroxybutyric acid and/or trihydroxybutanoic acid produced in the process can be isolated. The resulting glyceric acid, citramalic acid, fumaric acid, malic acid, pyruvic acid, succinic acid, trihydroxybutyric acid and/or trihydroxybutanoic acid can, if appropriate, subsequently be further purified, if desired mixed with other active ingredients such as vitamins, amino acids, carbohydrates, antibiotics and the like, and, if appropriate, formulated.

**[0379.0.16.16]** In one embodiment, gamma-aminobutyric acid and shikimate are a mixture of the respective fine chemicals.

**[0380.0.16.16]** The glyceric acid, citramalic acid, fumaric acid, malic acid, pyruvic acid, succinic acid, trihydroxybutyric acid and/or trihydroxybutanoic acid obtained in the process are suitable as starting material for the synthesis of further products of value. For example, they can be used in combination with each other or alone for the production of pharmaceuticals, foodstuffs, animal feeds or cosmetics. Accordingly, the present invention relates a method for the production of pharmaceuticals, food stuff, animal feeds, nutrients or cosmetics comprising the steps of the process according to the invention, including the isolation of the glyceric acid, citramalic acid, fumaric acid, malic acid, pyruvic acid, succinic acid, trihydroxybutyric acid and/or trihydroxybutanoic acid composition produced or the respective fine chemical produced if desired and formulating the product with a pharmaceutical acceptable carrier or formulating the product in a form acceptable for an application in agriculture. A further embodiment according to the invention is the use of the glyceric acid, citramalic acid, fumaric acid, malic acid, pyruvic acid, succinic acid, trihydroxybutyric acid and/or trihydroxybutanoic acid produced in the process or of the transgenic organisms in animal feeds, foodstuffs, medicines, food supplements, cosmetics or pharmaceuticals or for the production of glyceric acid, citramalic acid, fumaric acid, malic acid, pyruvic acid, succinic acid, trihydroxybutyric acid and/or trihydroxybutanoic acid e.g. after isolation of the respective fine chemical or without, e.g. in situ, e.g. in the organism used for the process for the production of the respective fine chemical.

**[0381.0.0.16]** to **[0382.0.0.16]: see [0381.0.0.0]** to **[0382.0.0.0]**

**[0383.0.16.16] -/-**

**[0384.0.0.16] see [0384.0.0.0]**

**[0385.0.16.16]** The fermentation broths obtained in this way, containing in particular glyceric acid, citramalic acid, fumaric acid, malic acid, pyruvic acid, succinic acid, trihydroxybutyric acid and/or trihydroxybutanoic acid in mixtures with other organic acids, amino acids, polypeptides or polysaccarides, normally have a dry matter content of from 1 to 70% by weight, preferably 7.5 to 25% by weight. Sugar-limited fermentation is additionally advantageous, e.g. at the end, for example over at least 30% of the fermentation time. This means that the concentration of utilizable sugar in the fermentation medium is kept at, or reduced to, 0 to 10 g/l, preferably to 0 to 3g/l during this time. The fermentation broth is then processed further. Depending on requirements, the biomass can be removed or isolated entirely or partly by separation methods, such as, for example, centrifugation, filtration, decantation, coagulation/flocculation or a combination of these methods, from the fermentation broth or left completely in it.
The fermentation broth can then be thickened or concentrated by known methods, such as, for example, with the aid of a rotary evaporator, thin-film evaporator, falling film evaporator, by reverse osmosis or by nanofiltration. This concentrated fermentation broth can then be worked up by freeze-drying, spray drying, spray granulation or by other processes.

**[0386.0.16.16]** Accordingly, it is possible to purify the glyceric acid, citramalic acid, fumaric acid, malic acid, pyruvic acid, succinic acid, trihydroxybutyric acid and/or trihydroxybutanoic acid produced according to the invention further. For this purpose, the product-containing composition is subjected for example to separation via e.g. an open column chromatography or HPLC in which case the desired product or the impurities are retained wholly or partly on the chromatography resin. These chromatography steps can be repeated if necessary, using the same or different chromatography resins. The skilled worker is familiar with the choice of suitable chromatography resins and their most effective use.

**[0387.0.0.16] to [0392.0.0.16]: see [0387.0.0.0] to [0392.0.0.0]**

**[0393.0.16.16]** In one embodiment, the present invention relates to a method for the identification of a gene product conferring an increase in the respective fine chemical production in a cell, comprising the following steps:
a) contacting, e.g. hybridising, the nucleic acid molecules of a sample, e.g. cells, tissues, plants or microorganisms or a nucleic acid library , which can contain a candidate gene encoding a gene product conferring an increase in the respective fine chemical after expression, with the nucleic acid molecule of the present invention;
b) identifying the nucleic acid molecules, which hybridize under relaxed stringent conditions with the nucleic acid molecule of the present invention in particular to the nucleic acid molecule sequence as indicated in Table I, columns 5 or 7, lines 190 to 226 or lines 564 to 594, preferably in Table I B, columns 5 or 7, ines 190 to 226 or lines 564 to 594, resp., and, optionally, isolating the full length cDNA clone or complete genomic clone;
c) introducing the candidate nucleic acid molecules in host cells, preferably in a plant cell or a microorganism, appropriate for producing the respective fine chemical;
d) expressing the identified nucleic acid molecules in the host cells;
e) assaying the respective fine chemical level in the host cells; and
f) identifying the nucleic acid molecule and its gene product which expression confers an increase in the respective fine chemical level in the host cell after expression compared to the wild type.

**[0394.0.0.16] to [0398.0.0.16]: see [0394.0.0.0] to [0398.0.0.0]**

**[0399.0.16.16]** Furthermore, in one embodiment, the present invention relates to process for the identification of a compound conferring increase of the respective fine chemical production in a plant or microorganism, comprising the steps:
culturing a cell or tissue or microorganism or maintaining a plant expressing the polypeptide according to the invention or a nucleic acid molecule encoding said polypeptide and a readout system capable of interacting with the polypeptide under suitable conditions which permit the interaction of the polypeptide with said readout system in the presence of a compound or a sample comprising a plurality of compounds and capable of providing a detectable signal in response to the binding of a compound to said polypeptide under conditions which permit the expression of said readout system and the polypeptide of the present invention or used in the process of the invention; and
identifying if the compound is an effective agonist by detecting the presence or absence or increase of a signal produced by said readout system.
The screen for a gene product or an agonist conferring an increase in the respective fine chemical production can be performed by growth of an organism for example a microorganism in the presence of growth reducing amounts of an inhibitor of the synthesis of the respective fine chemical. Better growth, e.g. higher dividing rate or high dry mass in comparison to the control under such conditions would identify a gene or gene product or an agonist conferring an increase in respective fine chemical production.

**[00399.1.0.16]** One can think to screen for increased production of the respective fine chemical by for example searching for a resistance to a drug blocking the synthesis of the respective fine chemical and looking whether this effect is dependent on the activity or expression of a polypeptide as indicated in Table II, columns 5 or 7, lines 190 to 226 or lines 564 to 594 or a homolog thereof, e.g. comparing the phenotype of nearly identical organisms with low and high activity of a protein as indicated in Table II, columns 5 or 7, lines 190 to 226 or lines 564 to 594 after incubation with the drug.

**[0400.0.0.16] to [0415.0.0.16]: see [0400.0.0.0] to [0415.0.0.0]**

**[0416.0.0.16] see [0416.0.0.0]**

**[0417.0.16.16]** The nucleic acid molecule of the invention, the vector of the invention or the nucleic acid construct of the invention may also be useful for the production of organisms resistant to inhibitors of the glyceric acid, citramalic acid, fumaric acid, malic acid, pyruvic acid, succinic acid, trihydroxybutyric acid and/or trihydroxybutanoic acid biosynthesis pathways. In particular, the overexpression of the polypeptide of the present invention may protect an organism such as a microorganism or a plant against inhibitors, which block the glyceric acid, citramalic acid, fumaric acid, malic acid, pyruvic acid, succinic acid, trihydroxybutyric acid and/or trihydroxybutanoic acid synthesis.

**[0418.0.0.16] to [0423.0.0.16]: see [0418.0.0.0] to [0423.0.0.0]**

**[0424.0.16.16]** Accordingly, the nucleic acid of the invention or used in the method of the invention, the polypeptide of the invention or used in the method of the invention, the nucleic acid construct of the invention, the organisms, the host cell, the microorganisms, the plant, plant tissue, plant cell, or the part thereof of the invention, the vector of the invention, the agonist identified with the method of the invention, the nucleic acid molecule identified with the method of the present invention, can be used for the production of the respective fine chemical or of the respective fine chemical and one or more other organic acids. Accordingly, the nucleic acid of the invention, or the nucleic acid molecule identified with the method of the present invention or the complement sequences thereof, the polypeptide of the invention, the nucleic acid construct of the invention, the organisms, the host cell, the microorganisms, the plant, plant tissue, plant cell, or the part thereof of the invention, the vector of the invention, the antagonist identified with the method of the invention, the antibody of the present invention, the antisense molecule of the present invention, can be used for the reduction of the respective fine chemical in a organism or part thereof, e.g. in a cell.

**[0425.0.0.16] to [0434.0.0.0]: see [0425.0.0.0] to [0434.0.0.0]**

**[0435.0.16.16]** Example 3: In-vivo and in-vitro mutagenesis

**[0436.0.16.16]** An *in vivo* mutagenesis of organisms such as Saccharomyces, Mortierella, Escherichia and others mentioned above, which are beneficial for the production of glyceric acid, citramalic acid, fumaric acid, malic acid, pyruvic acid, succinic acid, trihydroxybutyric acid and/or trihydroxybutanoic acid can be carried out by passing a plasmid DNA (or another vector DNA) containing the desired nucleic acid sequence or nucleic acid sequences, e.g. the nucleic acid molecule of the invention or the vector of the invention, through E. coli and other microorganisms (for example Bacillus spp. or yeasts such as Saccharomyces cerevisiae) which are not capable of maintaining the integrity of its genetic information. Usual mutator strains have mutations in the genes for the DNA repair system [for example mutHLS, mutD, mutT and the like; for comparison, see Rupp, W.D. (1996) DNA repair mechanisms in Escherichia coli and Salmonella, pp. 2277-2294, ASM: Washington]. The skilled worker knows these strains. The use of these strains is illustrated for example in Greener, A. and Callahan, M. (1994) Strategies 7; 32-34.
In-vitro mutation methods such as increasing the spontaneous mutation rates by chemical or physical treatment are well known to the skilled person. Mutagens like 5-bromo-uracil, N-methyl-N-nitro-N-nitrosoguanidine (= NTG), ethyl methanesulfonate (=EMS), hydroxylamine and/or nitrous acid are widely used as chemical agents for random in-vitro mutagenesis. The most common physical method for mutagenesis is the treatment with UV irradiation. Another random mutagenesis technique is the error-prone PCR for introducing amino acid changes into proteins. Mutations are deliberately introduced during PCR through the use of error-prone DNA polymerases and special reaction conditions known to a person skilled in the art. For this method randomized DNA sequences are cloned into expression vectors and the resulting mutant libraries screened for altered or improved protein activity as described below.
Site-directed mutagensis method such as the introduction of desired mutations with an M13 or phagemid vector and short oligonucleotides primers is a well-known approach for site-directed mutagensis. The clou of this method involves cloning of the nucleic acid sequence of the invention into an M13 or phagemid vector, which permits recovery of single-stranded recombinant nucleic acid sequence. A mutagenic oligonucleotide primer is then designed whose sequence is perfectly complementary to nucleic acid sequence in the region to be mutated, but with a single difference: at the intended mutation site it bears a base that is complementary to the desired mutant nucleotide rather than the original. The mutagenic oligonucleotide is then allowed to prime new DNA synthesis to create a complementary full-length sequence containing the desired mutation. Another site-directed mutagensis method is the PCR mismatch primer mutagensis method also known to the skilled person. Dpnl site-directed mutagensis is a further known method as described for example in the Stratagene QuickchangeTM site-directed mutagenesis kit protocol. A huge number of other methods are also known and used in common practice.
Positive mutation events can be selected by screening the organisms for the production of the desired respective fine chemical.

**[0437.0.16.16]** Example 4: DNA transfer between Escherichia coli, Saccharomyces cerevisiae and Mortierella alpina

**[0438.0.16.16]** Shuttle vectors such as pYE22m, pPAC-ResQ, pClasper, pAUR224, pAMH10, pAML10, pAMT10, pAMU10, pGMH10, pGML10, pGMT10, pGMU10, pPGAL1, pPADH1, pTADH1, pTAex3, pNGA142, pHT3101 and derivatives thereof which allow the transfer of nucleic acid sequences between Escherichia coli, Saccharomyces cerevisiae and/or Mortierella alpina are available to the skilled worker. An easy method to isolate such shuttle vectors is disclosed by Soni R. and Murray J.A.H. [Nucleic Acid Research, vol. 20 no. 21, 1992: 5852]: If necessary such shuttle vectors can be constructed easily using standard vectors for E. coli (Sambrook, J. et al., (1989), "Molecular Cloning: A Laboratory Manual", Cold Spring Harbor Laboratory Press or Ausubel, F.M. et al. (1994) "Current Protocols in Molecular Biology", John Wiley & Sons) and/or the aforementioned vectors, which have a replication origin for, and suitable marker from, Escherichia coli, Saccharomyces cerevisiae or Mortierella alpina added. Such replication origins are preferably taken from endogenous plasmids, which have been isolated from species used in the inventive process. Genes, which are used in particular as transformation markers for these species are genes for kanamycin resistance (such as those which originate from the Tn5 or Tn-903 transposon) or for chloramphenicol resistance (Winnacker, E.L. (1987) "From Genes to Clones - Introduction to Gene Technology", VCH, Weinheim) or for other antibiotic resistance genes such as for G418, gentamycin, neomycin, hygromycin or tetracycline resistance.

**[0439.0.16.16]** Using standard methods, it is possible to clone a gene of interest into one of the above-described shuttle vectors and to introduce such hybrid vectors into the microorganism strains used in the inventive process. The transformation of Saccharomyces can be achieved for example by LiCl or sheroplast transformation (Bishop et al., Mol. Cell. Biol., 6, 1986: 3401 - 3409; Sherman et al., Methods in Yeasts in Genetics, [Cold Spring Harbor Lab. Cold Spring Harbor, N. Y.] 1982, Agatep et al., Technical Tips Online 1998, 1:51: P01525 or Gietz et al., Methods Mol. Cell. Biol. 5, 1995: 255f) or electroporation (Delorme E., Appl. Environ. Microbiol., vol. 55, no. 9, 1989 : 2242 - 2246).

**[0440.0.16.16]** If the transformed sequence(s) is/are to be integrated advantageously into the genome of the microorganism used in the inventive process for example into the yeast or fungi genome, standard techniques known to the skilled worker also exist for this purpose. Solinger et al. (Proc Natl Acad Sci U S A., 2001 (15): 8447-8453) and Freedman et al. (Genetics, Vol. 162, 15-27, September 2002,) teaches a homolog recombination system dependent on rad 50, rad51, rad54 and rad59 in yeasts. Vectors using this system for homologous recombination are vectors derived from the Ylp series. Plasmid vectors derived for example from the 2µ-Vector are known by the skilled worker and used for the expression in yeasts. Other preferred vectors are for example pART1, pCHY21 or pEVP11 as they have been described by McLeod et al. (EMBO J. 1987, 6:729-736) and Hoffman et al. (Genes Dev. 5, 1991: :561-571.) or Russell et al. (J. Biol. Chem. 258, 1983: 143-149.). Other beneficial yeast vectors are plasmids of the REP, REP-X, pYZ or RIP series.

**[0441.0.0.16] to [0443.0.0.16] see [0441.0.0.0] to [0443.0.0.0]**

**[0444.0.16.16]** Example 6: Growth of genetically modified organism: media and culture conditions

**[0445.0.16.16]** Genetically modified Yeast, Mortierella or Escherichia coli are grown in synthetic or natural growth media known by the skilled worker. A number of different growth media for Yeast, Mortierella or Escherichia coli are well known and widely available. A method for culturing Mortierella is disclosed by Jang et al. [Bot. Bull. Acad. Sin. (2000) 41: 41-48]. Mortierella can be grown at 20°C in a culture medium containing: 10 g/l glucose, 5 g/I yeast extract at pH 6.5. Futhermore Jang et al. teaches a submerged basal medium containing 20 g/I soluble starch, 5 g/l Bacto yeast extract, 10 g/l KNO₃, 1 g/l KH₂PO₄, and 0.5 g/l MgSO₄•7H₂O, pH 6.5.

**[0446.0.0.16] to [0450.0.0.16]: see [0446.0.0.0] to [0450.0.0.0]**

**[0451.0.0.16] see [0451.0.5.5]**

**[0452.0.0.16] to [0453.0.0.16]: see [0452.0.0.0] to [0453.0.0.0]**

**[0454.0.16.16]** Analysis of the effect of the nucleic acid molecule on the production of glyceric acid, citramalic acid, fumaric acid, malic acid, pyruvic acid, succinic acid, trihydroxybutyric acid and/or trihydroxybutanoic acid

**[0455.0.16.16]** The effect of the genetic modification in plants, fungi, algae, ciliates or on the production of a desired compound (such as a glyceric acid, citramalic acid, fumaric acid, malic acid, pyruvic acid, succinic acid, trihydroxybutyric acid and/or trihydroxybutanoic acid) can be determined by growing the modified microorganisms or the modified plant under suitable conditions (such as those described above) and analyzing the medium and/or the cellular components for the elevated production of desired product (i.e. of glyceric acid, citramalic acid, fumaric acid, malic acid, pyruvic acid, succinic acid, trihydroxybutyric acid and/or trihydroxybutanoic acid). These analytical techniques are known to the skilled worker and comprise spectroscopy, thin-layer chromatography, various types of staining methods, enzymatic and microbiological methods and analytical chromatography such as high-performance liquid chromatography (see, for example, Ullman, Encyclopedia of Industrial Chemistry, Vol. A2, p. 89-90 and p. 443-613, VCH: Weinheim (1985); Fallon, A., et al., (1987) "Applications of HPLC in Biochemistry" in: Laboratory Techniques in Biochemistry and Molecular Biology, Vol. 17; Rehm et al. (1993) Biotechnology, Vol. 3, Chapter III: "Product recovery and purification", p. 469-714, VCH: Weinheim; Belter, P.A., et al. (1988) Bioseparations: downstream processing for Biotechnology, John Wiley and Sons; Kennedy, J.F., and Cabral, J.M.S. (1992) Recovery processes for biological Materials, John Wiley and Sons; Shaeiwitz, J.A., and Henry, J.D. (1988) Biochemical Separations, in: Ullmann's Encyclopedia of Industrial Chemistry, Vol. B3; Chapter 11, p. 1-27, VCH: Weinheim; and Dechow, F.J. (1989) Separation and purification techniques in biotechnology, Noyes Publications).

**[0456.0.0.16]: see [0456.0.0.0]**

**[0457.0.16.16]** Example 9: Purification of glyceric acid, citramalic acid, fumaric acid, malic acid, pyruvic acid, succinic acid, trihydroxybutyric acid or trihydroxybutanoic acid

**[0458.0.16.16]** Abbreviations; GC-MS, gas liquid chromatography/mass spectrometry; TLC, thin-layer chromatography.
The unambiguous detection for the presence of glyceric acid, citramalic acid, fumaric acid, malic acid, pyruvic acid, succinic acid, trihydroxybutyric acid or trihydroxybutanoic acid can be obtained by analyzing recombinant organisms using analytical standard methods: LC, LC-MSMS or TLC, as described. The total amount produced in the organism for example in yeasts used in the inventive process can be analysed for example according to the following procedure:
The material such as yeasts, E. coli or plants to be analyzed can be disrupted by sonication, grinding in a glass mill, liquid nitrogen and grinding or via other applicable methods.
Plant material is initially homogenized mechanically by comminuting in a pestle and mortar to make it more amenable to extraction.
A typical sample pre-treatment consists of a total lipid extraction using such polar organic solvents as acetone or alcohols as methanol, or ethers, saponification, partition between phases, seperation of non-polar epiphase from more polar hypophasic derivatives and chromatography.
For analysis, solvent delivery and aliquot removal can be accomplished with a robotic system comprising a single injector valve Gilson 232XL and a 402 2S1V diluter [Gilson, Inc. USA, 3000 W. Beltline Highway, Middleton, WI]. For saponification, 3 ml of 50% potassium hydroxide hydro-ethanolic solution (4 water - 1 ethanol) can be added to each vial, followed by the addition of 3 ml of octanol. The saponification treatment can be conducted at room temperature with vials maintained on an IKA HS 501 horizontal shaker [Labworld-online, Inc., Wilmington, NC] for fifteen hours at 250 movements/minute, followed by a stationary phase of approximately one hour.
Following saponification, the supernatant can be diluted with 0 0.17 ml of methanol. The addition of methanol can be conducted under pressure to ensure sample homogeneity. Using a 0.25 ml syringe, a 0.1 ml aliquot can be removed and transferred to HPLC vials for analysis.
For HPLC analysis, a Hewlett Packard 1100 HPLC, complete with a quaternary pump, vacuum degassing system, six-way injection valve, temperature regulated autosampler, column oven and Photodiode Array detector can be used [Agilent Technologies available through Ultra Scientific Inc., 250 Smith Street, North Kingstown, RI]. The column can be a Waters YMC30, 5-micron, 4.6 x 250 mm with a guard column of the same material [Waters, 34 Maple Street, Milford, MA]. The solvents for the mobile phase can be 81 methanol: 4 water: 15 tetrahydrofuran (THF) stabilized with 0.2% BHT (2,6-di-tert-butyl-4-methylphenol). Injections were 20 I. Separation can be isocratic at 30°C with a flow rate of 1.7 ml/minute. The peak responses can be measured by absorbance at 447 nm.

**[0459.0.16.16]** If required and desired, further chromatography steps with a suitable resin may follow. Advantageously, the glyceric acid, citramalic acid, fumaric acid, malic acid, pyruvic acid, succinic acid, trihydroxybutyric acid and/or trihydroxybutanoic acid can be further purified with a so-called RTHPLC. As eluent acetonitrile/water or chloroform/acetonitrile mixtures can be used. If necessary, these chromatography steps may be repeated, using identical or other chromatography resins. The skilled worker is familiar with the selection of suitable chromatography resin and the most effective use for a particular molecule to be purified.

**[0460.0.0.16] see [0460.0.0.0]**

**[0461.0.16.16]** Example 10: Cloning SEQ ID NO: 17198, 17818, 16100, 17898, 15872, 15848, 15780, 18092, 18380, 17104, 16824, 17374, 16284, 16410, 17712, 16132, 16128, 16118, 16014, 17920, 17948, 17198, 17038 or 18088 or others as idicated, for the expression in plants.

**[0462.0.0.16] see [0462.0.0.0]**

**[0463.0.16.16]** SEQ ID NO: 17198, 17818, 16100, 17898, 15872, 15848, 15780, 18092, 18380, 17104, 16824, 17374, 16284, 16410, 17712, 16132, 16128, 16118, 16014, 17920, 17948, 17198, 17038 or 18088 or others as idicated, is amplified by PCR as described in the protocol of the Pfu Turbo or DNA Herculase polymerase (Stratagene).

**[0464.0.0.0.16] to [0466.0.0.16]: see [0464.0.0.0] to [0466.0.0.0]**

**[0466.1.0.16]** In case the Herculase enzyme can be used for the amplification, the PCR amplification cycles were as follows: 1 cycle of 2-3 minutes at 94°C, followed by 25-30 cycles of in each case 30 seconds at 94°C, 30 seconds at 55-60°C and 5-10 minutes at 72°C, followed by 1 cycle of 10 minutes at 72°C, then 4°C.

**[0467.0.16.16]** The following primer sequences were selected for the gene SEQ ID NO: 17198:
i) forward primer (SEQ ID NO: 17372 )
   atgagtcgtt tagtcgtagt atcta
ii) reverse primer (SEQ ID NO: 17373)
   ttacgcaagc tttggaaagg tagc
The following primer sequences were selected for the gene SEQ ID NO: 17818:
i) forward primer (SEQ ID NO: 17896 )
   atgacaattt ctaatttgtt aaagcag
ii) reverse primer (SEQ ID NO: 17897)
   ctagtcaact ggttcccagc tgt
The following primer sequences were selected for the gene SEQ ID NO: 16100
i) forward primer (SEQ ID NO: 16116)
   atggatgacg atcacgaaca gttg
ii) reverse primer (SEQ ID NO: 16117)
   ttacgagtcg aagccggccc t
The following primer sequences were selected for the gene SEQ ID NO: 17898:
i) forward primer (SEQ ID NO: 17918 )
   atggtcgata cgcataaact agca
ii) reverse primer (SEQ ID NO: 17919)
   ctagtcgttg cggatatgga cga
The following primer sequences were selected for the gene SEQ ID NO: 15872:
i) forward primer (SEQ ID NO: 16012)
   atgacaatgg aaaaaaatgg aggtaa
ii) reverse primer (SEQ ID NO: 16013)
   ttatttagca caatcctcgt gagc
The following primer sequences were selected for the gene SEQ ID NO: 15848:
i) forward primer (SEQ ID NO: 15870)
   atgaatttgt tttggccatc ggaaa
ii) reverse primer (SEQ ID NO: 15871)
   tcaaggttgt actgtgtgat ctag
The following primer sequences were selected for the gene SEQ ID NO: 15780:
i) forward primer (SEQ ID NO: 15846)
   atgccatcta ccactaatac tgct
ii) reverse primer (SEQ ID NO: 15847)
   ctaatgtttg gcaactgggg tttc
The following primer sequences were selected for the gene SEQ ID NO: 18092:
i) forward primer (SEQ ID NO: 18378)
   atgaacgttt caaaaatact tgtgtc
ii) reverse primer (SEQ ID NO: 18379)
   tcatgcattt aacattgtag gaatttt
The following primer sequences were selected for the gene SEQ ID NO: 18380:
i) forward primer (SEQ ID NO: 18640)
   atggctcggg gtgacggaca t
ii) reverse primer (SEQ ID NO: 18641)
   tcatgcttct tttgcgtgat gcaat
The following primer sequences were selected for the gene SEQ ID NO: 17104:
i) forward primer (SEQ ID NO: 17196)
   atgggacaca agcccttata ccg
ii) reverse primer (SEQ ID NO: 17197)
   ttatcgcgat gattttcgct gcg
The following primer sequences were selected for the gene SEQ ID NO: 16824:
i) forward primer (SEQ ID NO: 17036)
   atgaatacag tacgcagcga aaa
ii) reverse primer (SEQ ID NO: 17037)
   ttaacgcccg gctttcatac tgc
The following primer sequences were selected for the gene SEQ ID NO: 17374:
i) forward primer (SEQ ID NO: 17710)
   atggctatcg acgaaaacaa acag
ii) reverse primer (SEQ ID NO: 17711)
   ttaaaaatct tcgttagttt ctgctac
The following primer sequences were selected for the gene SEQ ID NO: 16284:
i) forward primer (SEQ ID NO: 16408)
   atgtgtttaa agcaaatcat tggcag
ii) reverse primer (SEQ ID NO: 16409)
   ttactgttcg ctttcatcag tatag
The following primer sequences were selected for the gene SEQ ID NO: 16410:
i) forward primer (SEQ ID NO: 16822)
   atgaaaaaga ccaaaattgt ttgca
ii) reverse primer (SEQ ID NO: 16823)
   ttacaggacg tgaacagatg cgg
The following primer sequences were selected for the gene SEQ ID NO: 17712:
i) forward primer (SEQ ID NO: 17816)
   atggaaaaga aattaccccg catta
ii) reverse primer (SEQ ID NO: 17817)
   ttagttttgt tcatcttcca gcaag
The following primer sequences were selected for the gene SEQ ID NO: 17818:
i) forward primer (SEQ ID NO: 17896)
   atgacaattt ctaatttgtt aaagcag
ii) reverse primer (SEQ ID NO: 17897)
   ctagtcaact ggttcccagc tgt
The following primer sequences were selected for the gene SEQ ID NO: 16132:
i) forward primer (SEQ ID NO: 16282)
   atgttgtcga gactatcttt attgag
ii) reverse primer (SEQ ID NO: 16283)
   ttaaaataga ccttcaattt caccgt
The following primer sequences were selected for the gene SEQ ID NO: 16128:
i) forward primer (SEQ ID NO: 16130)
   atgtaccaaa ataatgtatt gaatgct
ii) reverse primer (SEQ ID NO: 16131)
   tcaatagtgc attaactctc ccatt
The following primer sequences were selected for the gene SEQ ID NO: 16118:
i) forward primer (SEQ ID NO: 16126)
   atggaggacg gtaaacaggc cat
ii) reverse primer (SEQ ID NO: 16127)
   ttagagtctt ccaccggggg tg
The following primer sequences were selected for the gene SEQ ID NO: 16014:
i) forward primer (SEQ ID NO: 16098)
   atgacggtaa acttagatcc ggata
ii) reverse primer (SEQ ID NO: 16099)
   ttatatggac attccctttt tttggt
The following primer sequences were selected for the gene SEQ ID NO: 17920:
i) forward primer (SEQ ID NO: 17946)
   atgtcactac cggcacattt gca
ii) reverse primer (SEQ ID NO: 17947)
   ttaaatattg aattcttctt catcatttt
The following primer sequences were selected for the gene SEQ ID NO: 17948:
i) forward primer (SEQ ID NO: 18086)
   atggatgata taagcggaag gcaaa
ii) reverse primer (SEQ ID NO: 18087)
   ctatactggc aagtgacagt tgtg
The following primer sequences were selected for the gene SEQ ID NO: 17038:
i) forward primer (SEQ ID NO: 17102)
   atgaataacg aacccttacg tccc
ii) reverse primer (SEQ ID NO: 17103)
   ttacatatcc tcatgaaatt cttcaagt
The following primer sequences were selected for the gene SEQ ID NO: 16118:
i) forward primer (SEQ ID NO: 16126 )
   atggaggacg gtaaacaggc cat
ii) reverse primer (SEQ ID NO: 16127)
   ttagagtctt ccaccggggg tg
The following primer sequences were selected for the gene SEQ ID NO: 18088:
i) forward primer (SEQ ID NO: 18090)
   atgatagagg cgttggaaat agttc
ii) reverse primer (SEQ ID NO: 18091)
   ttatatcgat cctgcgatat aataac
Further primers for the cloning of the nucleic acids indicated in table II, column 5 see table III, column 7, resp.,

[0468.0.16.16] to [0470.0.16.16]: see [0468.0.0.0] to [0470.0.0.0]

[0470.1.16.16] The PCR-products, produced by *Pfu* Turbo DNA polymerase, were phosphorylated using a T4 DNA polymerase using a standard protocol (e.g. MBI Fermentas) and cloned into the processed binary vector.

**[0471.0.16.16] see [0471.0.0.0]**

**[0471.1.16.16]** The DNA termini of the PCR-products, produced by Herculase DNA polymerase, were blunted in a second synthesis reaction using Pfu Turbo DNA polymerase. The composition for the protocol of the blunting the DNA-termini was as follows: 0.2 mM blunting dTTP and 1.25 u *Pfu* Turbo DNA polymerase. The reaction was incubated at 72°C for 30 minutes. Then the PCR-products were phosphorylated using a T4 DNA polymerase using a standard protocol (e.g. MBI Fermentas) and cloned into the processed vector as well.

**[0472.0.16.16] to [0479.0.16.16]: see [0472.0.0.0] to [0479.0.0.0]**

**[0480.0.16.16]** Example 11: Generation of transgenic plants which express SEQ ID NO:17198, 17818, 16100, 17898, 15872, 15848, 15780, 18092, 18380, 17104, 16824, 17374, 16284, 16410, 17712, 16132, 16128, 16118, 16014, 17920, 17948, 17198, 17038 or 18088 or as indicated in table I column 5.

**[0481.0.0.16] to [0513.0.0.16]: see [0481.0.0.0] to [0513.0.0.0]**

**[0514.0.16.16]** As an alternative, the organic acids can be detected as described in Farré, E. et al. , Plant Physiol, 2001, Vol. 127, pp. 685-700.
The results of the different plant analyses can be seen from the table 1 which follows:

**Table 1**

| **ORF** | **Metabolite** | **Method** | **Min** | **Max** |
|---|---|---|---|---|
| b1896 | citramalic acid | GC | 1.63 | 1.99 |
| YBL015W | fumaric acid | LC | 1.50 | 2.26 |
| YCR059C | fumaric acid | GC | 1.71 | 2.83 |
| YFR007W | fumaric acid | GC | 1.69 | 2.56 |
| YJL055W | fumaric acid | GC | 1.72 | 6.61 |
| YJL099W | fumaric acid | GC | 1.98 | 8.34 |
| YMR241W | fumaric acid | GC + LC | 1.75 | 4.68 |
| YPR024W | fumaric acid | GC | 1.97 | 4.74 |
| YPR138C | fumaric acid | LC | 1.61 | 4.27 |
| b0730 | fumaric acid | GC + LC | 1.75 | 9.38 |
| b1611 | fumaric acid | GC + LC | 2.06 | 4.28 |
| b2699 | fumaric acid | GC | 1.65 | 9.02 |
| b4139 | fumaric acid | GC + LC | 2.18 | 6.25 |
| b1676 | fumaric acid | GC | 2.08 | 5.30 |
| b1896 | fumaric acid | GC + LC | 3.32 | 4.20 |
| b4063 | fumaric acid | GC | 1.64 | 4.08 |
| YBL015W | malic acid | LC | 1.45 | 1.86 |
| YBR084W | malic acid | GC | 2.07 | 5.48 |
| YBR184W | malic acid | LC | 1.67 | 3.13 |
| YCL032W | malic acid | LC | 1.87 | 2.23 |
| YGR007W | malic acid | LC | 1.55 | 1.90 |
| YJL072C | malic acid | GC | 1.49 | 3.49 |
| YJL099W | malic acid | GC | 1.94 | 8.97 |
| YKL132C | malic acid | GC | 2.09 | 3.13 |
| YPR138C | malic acid | GC | 1.72 | 4.59 |
| b0730 | malic acid | GC + LC | 1.96 | 7.72 |
| b1896 | malic acid | GC | 2.49 | 4.19 |
| b4063 | malic acid | GC | 1.78 | 1.93 |
| b2699 | trihydroxybutyric acid | GC | 1.48 | 3.15 |
| b1896 | trihydroxybutyric acid | GC | 1.34 | 1.96 |
| b0695 | pyruvic acid | GC | 1.61 | 2.55 |
| YCL032W | succinic acid | LC | 1.47 | 1.91 |
| YOR044W | succinic acid | LC | 1.45 | 3.65 |
| b0730 | succinic acid | LC | 1.51 | 4.32 |
| b1896 | succinic acid | GC + LC | 1.55 | 2.10 |
| b2699 | Trihydroxybutanoic acid | GC | 1.82 | 3.81 |
| b1896 | Trihydroxybutanoic acid | GC | 1.60 | 2.61 |
| b1693 | Citramalic acid | GC | 1.48 | 2.05 |
| b0161 | Fumaric acid | LC | 1.64 | 2.51 |
| b1738 | Fumaric acid | LC | 1.53 | 2.49 |
| b1961 | Fumaric acid | GC + LC | 1.76 | 2.05 |
| b3116 | Fumaric acid | GC | 1.63 | 1.99 |
| b3172 | Fumaric acid | GC | 1.63 | 4.25 |
| b4122 | Fumaric acid | GC | 2.08 | 5.30 |
| YCR012W | Fumaric acid | LC | 1.62 | 1.93 |
| b0057 | Glyceric acid | GC | 1.35 | 1.57 |
| b1693 | Glyceric acid | GC | 1.33 | 1.52 |
| b3129 | Glyceric acid | GC | 1.30 | 1.35 |
| b3231 | Glyceric acid | GC | 1.21 | 1.34 |
| b3938 | Glyceric acid | GC | 1.37 | 1.40 |
| b0161 | Malic acid | GC | 1.64 | 3.29 |
| b2478 | Malic acid | GC + LC | 1.51 | 2.24 |
| b3116 | Malic acid | GC | 1.60 | 3.12 |
| b3169 | Malic acid | GC | 2.32 | 2.43 |
| b3172 | Malic acid | GC | 1.64 | 5.08 |
| YCL038C | Malic acid | LC | 1.87 | 2.23 |
| YOR168W | Malic acid | GC + LC | 1.56 | 6.62 |
| b3231 | Pyruvic acid | GC | 1.43 | 1.46 |
| b1738 | Succinic acid | LC | 1.49 | 1.95 |
| b1961 | Succinic acid | LC | 1.29 | 1.54 |
| b2599 | Succinic acid | GC + LC | 1.33 | 2.56 |
| b3116 | Succinic acid | LC | 1.31 | 1.42 |
| b3160 | Succinic acid | LC | 1.33 | 1.90 |
| b4129 | Succinic acid | LC | 1.40 | 1.84 |
| YCL038C | Succinic acid | LC | 1.47 | 1.91 |
| b0970 | Trihydroxybutanoic acid | GC | 1.34 | 1.80 |
| b1343 | Trihydroxybutanoic acid | GC | 1.33 | 2.08 |
| b3169 | Trihydroxybutanoic acid | GC | 1.60 | 1.88 |

Column 2 shows the organic acids citramalic acid, fumaric acid, malic acid, trihydroxybutyric acid, trihydroxybutanoic acid, succinic acid, pyruvic acid as analyzed. Columns 4 and 5 show the ratio of the analyzed organic acid between the transgenic plants and the wild type; Increase of the metabolites: Max: maximal x-fold (normalised to wild type)-Min: minimal x-fold (normalised to wild type). Decrease of the metabolites: Max: maximal x-fold (normalised to wild type) minimal decrease), Min: minimal x-fold (normalised to wild type) (minimal decrease), Min: minimal x-fold (normalised to wild type) maximal decrease). Column 3 indicates the analytical method.

**[0515.0.0.16] to [0552.0.0.16]: see [0515.0.0.0] to [0552.0.0.0]** including [0530.1.0.0] to [0530.6. 0.0] as well as [0552.2.0.0]

**[0552.1.18.18]** Example 15: Metabolite Profiling Info from *Zea mays*
*Zea mays* plants were engineered as described in Example 14c.
The results of the different *Zea mays* plants analysed can be seen from table 2 which follows:

**Table 2**

| **ORF** | **Metabolite** | **Min** | **Max** |
|---|---|---|---|
| b2699 | Fumaric acid | 1.38 | 1.54 |
| YJL055W | Fumaric acid | 1.42 | 2.01 |
| YMR241W | Fumaric acid | 1.75 | 4.89 |
| b1611 | Fumaric acid | 1.42 | 1.69 |
| YBR084W | Malic acid | 1.73 | 11.39 |
| b1896 | Lacton of Trihydroxybutyraic acid | 1.59 | 2.01 |

Table 2 describes the increase in organic acids in genetically modified corn plants expressing the E.coli K12 nucleic acid sequence b2699, b1611, b1896 or the Saccaromyces cerevisiae nucleic acids YJL055W YMR241 W or YBR084W.
In one embodiment, the activity of the Escherichia coli K12 protein b2699 or its homologs, e.g. a protein having the activity of a recombination protein recA, preferably being involved in DNA recombination and DNA repair, pheromone response, mating-type determination, sex-specific proteins, nucleotide binding, is increased, preferably, conferring the increase of fumeric acid between 38% and 54% or more.
In one embodiment, in case the activity of the Escherichia coli K12 protein b1896 or its homologs, e.g. a trehalose-6-phosphate synthase, is increased, conferring an increase of the fine chemical preferably of Trihydroxybutyraic acid between 59% and 101 % or more.
In one embodiment, the activity of the Escherichia coli K12 protein b1611 or its homologs, e.g. a protein of the superfamily of fumarate hydratase, preferably being involved in C-compound, carbohydrate catabolism, tricarboxylic-acid pathway (citrate cycle, Krebs cycle, TCA cycle), ENERGY, biosynthesis of aspartate, degradation of aspartate, nitrogen and sulfur utilization, degradation of amino acids of the aspartate group, biosynthesis of the pyruvate family (alanine, isoleucine, leucine, valine) and d-alanine, or regulation of nitrogen and sulphur utilization, preferably being a fumarase C (fumarate hydratase Class II), is increased, preferably, conferring the increase of fumeric acid between 42% and 69% or more.
In one embodiment, the activity of the Saccharomyces cerevisiae protein YJL055W or its homologs, e.g. a protein of the superfamily of yeast conserved hypothetical protein YJL055w, preferably being involved in biosynthesis of lysine, preferably being a Lysine decarboxylase-like protein, is increased, preferably, conferring the increase of fumeric acid between 42% and 101 % or more.
In one embodiment, the activity of the Saccharomyces cerevisiae protein YMR241 W or its homologs, e.g. a protein of the superfamily of ADP,ATP carrier protein, preferably being involved in C-compound, carbohydrate transport, and/or mitochondrial transport, preferably being a Mitochondrial DNA replication protein, is increased, preferably, conferring the increase of fumeric acid between 74% and 389% or more.
In one embodiment, the activity of the Saccharomyces cerevisiae protein YBR084W or its homologs, e.g. a protein of the superfamily "C1-tetrahydrofolate synthase, formate-tetrahydrofolate ligase homology, methylenetetrahydrofolate dehydrogenase (NAD+) homology", preferably being involved in amino acid biosynthesis, purine nucleotide metabolism, biosynthesis of vitamins, cofactors, and prosthetic groups, C-compound and carbohydrate utilization, tRNA modification, biosynthesis of histidine, degradation of histidine, biosynthesis of the aspartate family, degradation of amino acids of the aspartate group, nucleotide binding, and/or nucleotide metabolism, preferably being a C1-tetrahydrofolate synthase, is increased, preferably, conferring the increase of malic acid between 73% and 1039% or more.

**[0553.0.16.16]**
1. A process for the production of glyceric acid, citramalic acid, fumaric acid, malic acid, pyruvic acid, succinic acid, trihydroxybutyric acid or trihydroxybutanoic acid, which comprises
   a) increasing or generating the activity of a protein as indicated in Table II, columns 5 or 7, lines 190 to 226 or lines 564 to 594 or a functional equivalent thereof in a non-human organism or in one or more parts thereof; and
   b) growing the organism under conditions which permit the production of glyceric acid, citramalic acid, fumaric acid, malic acid, pyruvic acid, succinic acid, trihydroxybutyric acid or trihydroxybutanoic acid in said organism.
2. A process for the production of glyceric acid, citramalic acid, fumaric acid, malic acid, pyruvic acid, succinic acid, trihydroxybutyric acid or trihydroxybutanoic acid, comprising the increasing or generating in an organism or a part thereof the expression of at least one nucleic acid molecule comprising a nucleic acid molecule selected from the group consisting of:
   a) nucleic acid molecule encoding of a polypeptide as indicated in Table II, columns 5 or 7, lines 190 to 226 or lines 564 to 594 or a fragment thereof, which confers an increase in the amount of glyceric acid, citramalic acid, fumaric acid, malic acid, pyruvic acid, succinic acid, trihydroxybutyric acid or trihydroxybutanoic acid in an organism or a part thereof;
   b) nucleic acid molecule comprising of a nucleic acid molecule as indicated in Table I, columns 5 or 7, lines 190 to 226 or lines 564 to 594;
   c) nucleic acid molecule whose sequence can be deduced from a polypeptide sequence encoded by a nucleic acid molecule of (a) or (b) as a result of the degeneracy of the genetic code and conferring an increase in the amount of glyceric acid, citramalic acid, fumaric acid, malic acid, pyruvic acid, succinic acid, trihydroxybutyric acid or trihydroxybutanoic acid in an organism or a part thereof;
   d) nucleic acid molecule which encodes a polypeptide which has at least 50% identity with the amino acid sequence of the polypeptide encoded by the nucleic acid molecule of (a) to (c) and conferring an increase in the amount of glyceric acid, citramalic acid, fumaric acid, malic acid, pyruvic acid, succinic acid, trihydroxybutyric acid or trihydroxybutanoic acid in an organism or a part thereof;
   e) nucleic acid molecule which hybridizes with a nucleic acid molecule of (a) to (c) under stringent hybridisation conditions and conferring an increase in the amount of glyceric acid, citramalic acid, fumaric acid, malic acid, pyruvic acid, succinic acid, trihydroxybutyric acid or trihydroxybutanoic acid in an organism or a part thereof;
   f) nucleic acid molecule which encompasses a nucleic acid molecule which is obtained by amplifying nucleic acid molecules from a cDNA library or a genomic library using the primers or primer pairs as indicated in Table III, columns 5 or 7, lines 190 to 226 or lines 564 to 594 and conferring an increase in the amount of glyceric acid, citramalic acid, fumaric acid, malic acid, pyruvic acid, succinic acid, trihydroxybutyric acid or trihydroxybutanoic acid in an organism or a part thereof;
   g) nucleic acid molecule encoding a polypeptide which is isolated with the aid of monoclonal antibodies against a polypeptide encoded by one of the nucleic acid molecules of (a) to (f) and conferring an increase in the amount of glyceric acid, citramalic acid, fumaric acid, malic acid, pyruvic acid, succinic acid, trihydroxybutyric acid or trihydroxybutanoic acid in an organism or a part thereof;
   h) nucleic acid molecule encoding a polypeptide comprising a consensus as indicated in Table IV, columns 5 or 7, lines 190 to 226 or lines 564 to 594 and conferring an increase in the amount of glyceric acid, citramalic acid, fumaric acid, malic acid, pyruvic acid, succinic acid, trihydroxybutyric acid or trihydroxybutanoic acid in an organism or a part thereof; and
   i) nucleic acid molecule which is obtainable by screening a suitable nucleic acid library under stringent hybridization conditions with a probe comprising one of the sequences of the nucleic acid molecule of (a) to (k) or with a fragment thereof having at least 15 nt, preferably 20 nt, 30 nt, 50 nt, 100 nt, 200 nt or 500 nt of the nucleic acid molecule characterized in (a) to (k) and conferring an increase in the amount of glyceric acid, citramalic acid, fumaric acid, malic acid, pyruvic acid, succinic acid, trihydroxybutyric acid or trihydroxybutanoic acid in an organism or a part thereof.
      or comprising a sequence which is complementary thereto.
3. The process of claim 1 or 2, comprising recovering of the free or bound glyceric acid, citramalic acid, fumaric acid, malic acid, pyruvic acid, succinic acid, trihydroxybutyric acid or trihydroxybutanoic acid.
4. The process of any one of claim 1 to 3, comprising the following steps:
   a) selecting an organism or a part thereof expressing a polypeptide encoded by the nucleic acid molecule characterized in claim 2;
   b) mutagenizing the selected organism or the part thereof;
   c) comparing the activity or the expression level of said polypeptide in the mutagenized organism or the part thereof with the activity or the expression of said polypeptide of the selected organisms or the part thereof;
   d) selecting the mutated organisms or parts thereof, which comprise an increased activity or expression level of said polypeptide compared to the selected organism or the part thereof;
   e) optionally, growing and cultivating the organisms or the parts thereof; and
   f) recovering, and optionally isolating, the free or bound glyceric acid, citramalic acid, fumaric acid, malic acid, pyruvic acid, succinic acid, trihydroxybutyric acid or trihydroxybutanoic acid produced by the selected mutated organisms or parts thereof.
5. The process of any one of claims 1 to 4, wherein the activity of said protein or the expression of said nucleic acid molecule is increased or generated transiently or stably.
6. An isolated nucleic acid molecule comprising a nucleic acid molecule selected from the group consisting of:
   a) nucleic acid molecule encoding of a polypeptide as indicated in Table II, columns 5 or 7, lines 190 to 226 or lines 564 to 594 or a fragment thereof, which confers an increase in the amount of glyceric acid, citramalic acid, fumaric acid, malic acid, pyruvic acid, succinic acid, trihydroxybutyric acid or trihydroxybutanoic acid in an organism or a part thereof;
   b) nucleic acid molecule comprising of a nucleic acid molecule as indicated in Table I, columns 5 or 7, lines 90 to 226 or lines 564 to 594;
   c) nucleic acid molecule whose sequence can be deduced from a polypeptide sequence encoded by a nucleic acid molecule of (a) or (b) as a result of the degeneracy of the genetic code and conferring an increase in the amount of glyceric acid, citramalic acid, fumaric acid, malic acid, pyruvic acid, succinic acid, trihydroxybutyric acid or trihydroxybutanoic acid in an organism or a part thereof;
   d) nucleic acid molecule which encodes a polypeptide which has at least 50% identity with the amino acid sequence of the polypeptide encoded by the nucleic acid molecule of (a) to (c) and conferring an increase in the amount of glyceric acid, citramalic acid, fumaric acid, malic acid, pyruvic acid, succinic acid, trihydroxybutyric acid or trihydroxybutanoic acid in an organism or a part thereof;
   e) nucleic acid molecule which hybridizes with a nucleic acid molecule of (a) to (c) under stringent hybridisation conditions and conferring an increase in the amount of glyceric acid, citramalic acid, fumaric acid, malic acid, pyruvic acid, succinic acid, trihydroxybutyric acid or trihydroxybutanoic acid in an organism or a part thereof;
   f) nucleic acid molecule which encompasses a nucleic acid molecule which is obtained by amplifying nucleic acid molecules from a cDNA library or a genomic library using the primers or primer pairs as indicated in Table III, column 7, lines 190 to 226 or lines 564 to 594 and conferring an increase in the amount of glyceric acid, citramalic acid, fumaric acid, malic acid, pyruvic acid, succinic acid, trihydroxybutyric acid or trihydroxybutanoic acid in an organism or a part thereof;
   g) nucleic acid molecule encoding a polypeptide which is isolated with the aid of monoclonal antibodies against a polypeptide encoded by one of the nucleic acid molecules of (a) to (f) and conferring an increase in the amount of glyceric acid, citramalic acid, fumaric acid, malic acid, pyruvic acid, succinic acid, trihydroxybutyric acid or trihydroxybutanoic acid in an organism or a part thereof;
   h) nucleic acid molecule encoding a polypeptide comprising a consensus as indicated in Table IV, column 7, lines 190 to 226 or lines 564 to 594 and conferring an increase in the amount of in an organism or a part thereof; and
   i) nucleic acid molecule which is obtainable by screening a suitable nucleic acid library under stringent hybridization conditions with a probe comprising one of the sequences of the nucleic acid molecule of (a) to (k) or with a fragment thereof having at least 15 nt, preferably 20 nt, 30 nt, 50 nt, 100 nt, 200 nt or 500 nt of the nucleic acid molecule characterized in (a) to (k) and conferring an increase in the amount of glyceric acid, citramalic acid, fumaric acid, malic acid, pyruvic acid, succinic acid, trihydroxybutyric acid or trihydroxybutanoic acid in an organism or a part thereof.
   whereby the nucleic acid molecule distinguishes over the sequence as indicated in Table I A, columns 5 or 7, lines 190 to 226 or lines 564 to 594 by one or more nucleotides.
7. A nucleic acid construct which confers the expression of the nucleic acid molecule of claim 6, comprising one or more regulatory elements.
8. A vector comprising the nucleic acid molecule as claimed in claim 6 or the nucleic acid construct of claim 7.
9. The vector as claimed in claim 8, wherein the nucleic acid molecule is in operable linkage with regulatory sequences for the expression in a prokaryotic or eukaryotic, or in a prokaryotic and eukaryotic, host.
10. A host cell, which has been transformed stably or transiently with the vector as claimed in claim 8 or 9 or the nucleic acid molecule as claimed in claim 6 or the nucleic acid construct of claim 7 or produced as described in claim any one of claims 2 to 5.
11. The host cell of claim 10, which is a transgenic host cell.
12. The host cell of claim 10 or 11, which is a plant cell, an animal cell, a microorganism, or a yeast cell, a fungus cell, a prokaryotic cell, an eukaryotic cell or an archaebacterium.
13. A process for producing a polypeptide, wherein the polypeptide is expressed in a host cell as claimed in any one of claims 10 to 12.
14. A polypeptide produced by the process as claimed in claim 13 or encoded by the nucleic acid molecule as claimed in claim 6 whereby the polypeptide distinguishes over a sequence as indicated in Table II A, columns 5 or 7, lines 190 to 226 or lines 564 to 594 by one or more amino acids.
15. An antibody, which binds specifically to the polypeptide as claimed in claim 14.
16. A plant tissue, propagation material, harvested material or a plant comprising the host cell as claimed in claim 12 which is plant cell or an Agrobacterium.
17. A method for screening for agonists and antagonists of the activity of a polypeptide encoded by the nucleic acid molecule of claim 6 conferring an increase in the amount of glyceric acid, citramalic acid, fumaric acid, malic acid, pyruvic acid, succinic acid, trihydroxybutyric acid or trihydroxybutanoic acid in an organism or a part thereof comprising:
   (a) contacting cells, tissues, plants or microorganisms which express the a polypeptide encoded by the nucleic acid molecule of claim 6 conferring an increase in the amount of glyceric acid, citramalic acid, fumaric acid, malic acid, pyruvic acid, succinic acid, trihydroxybutyric acid or trihydroxybutanoic acid in an organism or a part thereof with a candidate compound or a sample comprising a plurality of compounds under conditions which permit the expression the polypeptide;
   (b) assaying the glyceric acid, citramalic acid, fumaric acid, malic acid, pyruvic acid, succinic acid, trihydroxybutyric acid or trihydroxybutanoic acid level or the polypeptide expression level in the cell, tissue, plant or microorganism or the media the cell, tissue, plant or microorganisms is cultured or maintained in; and
   (c) identifying a agonist or antagonist by comparing the measured glyceric acid, citramalic acid, fumaric acid, malic acid, pyruvic acid, succinic acid, trihydroxybutyric acid or trihydroxybutanoic acid level or polypeptide expression level with a standard glyceric acid, citramalic acid, fumaric acid, malic acid, pyruvic acid, succinic acid, trihydroxybutyric acid or trihydroxybutanoic acid or polypeptide expression level measured in the absence of said candidate compound or a sample comprising said plurality of compounds, whereby an increased level over the standard indicates that the compound or the sample comprising said plurality of compounds is an agonist and a decreased level over the standard indicates that the compound or the sample comprising said plurality of compounds is an antagonist.
18. A process for the identification of a compound conferring increased glyceric acid, citramalic acid, fumaric acid, malic acid, pyruvic acid, succinic acid, trihydroxybutyric acid or trihydroxybutanoic acid production in a plant or microorganism, comprising the steps:
   (a) culturing a plant cell or tissue or microorganism or maintaining a plant expressing the polypeptide encoded by the nucleic acid molecule of claim 6 conferring an increase in the amount of glyceric acid, citramalic acid, fumaric acid, malic acid, pyruvic acid, succinic acid, trihydroxybutyric acid or trihydroxybutanoic acid in an organism or a part thereof and a readout system capable of interacting with the polypeptide under suitable conditions which permit the interaction of the polypeptide with said readout system in the presence of a compound or a sample comprising a plurality of compounds and capable of providing a detectable signal in response to the binding of a compound to said polypeptide under conditions which permit the expression of said readout system and of the polypeptide encoded by the nucleic acid molecule of claim 6 conferring an increase in the amount of glyceric acid, citramalic acid, fumaric acid, malic acid, pyruvic acid, succinic acid, trihydroxybutyric acid or trihydroxybutanoic acid in an organism or a part thereof;
   (b) identifying if the compound is an effective agonist by detecting the presence or absence or increase of a signal produced by said readout system.
19. A method for the identification of a gene product conferring an increase in glyceric acid, citramalic acid, fumaric acid, malic acid, pyruvic acid, succinic acid, trihydroxybutyric acid or trihydroxybutanoic acid production in a cell, comprising the following steps:
   (a) contacting the nucleic acid molecules of a sample, which can contain a candidate gene encoding a gene product conferring an increase in glyceric acid, citramalic acid, fumaric acid, malic acid, pyruvic acid, succinic acid, trihydroxybutyric acid or trihydroxybutanoic acid after expression with the nucleic acid molecule of claim 6;
   (b) identifying the nucleic acid molecules, which hybridise under relaxed stringent conditions with the nucleic acid molecule of claim 6;
   (c) introducing the candidate nucleic acid molecules in host cells appropriate for producing glyceric acid, citramalic acid, fumaric acid, malic acid, pyruvic acid, succinic acid, trihydroxybutyric acid or trihydroxybutanoic acid;
   (d) expressing the identified nucleic acid molecules in the host cells;
   (e) assaying the glyceric acid, citramalic acid, fumaric acid, malic acid, pyruvic acid, succinic acid, trihydroxybutyric acid or trihydroxybutanoic acid level in the host cells; and
   (f) identifying nucleic acid molecule and its gene product which expression confers an increase in the glyceric acid, citramalic acid, fumaric acid, malic acid, pyruvic acid, succinic acid, trihydroxybutyric acid or trihydroxybutanoic acid level in the host cell in the host cell after expression compared to the wild type.
20. A method for the identification of a gene product conferring an increase in glyceric acid, citramalic acid, fumaric acid, malic acid, pyruvic acid, succinic acid, trihydroxybutyric acid or trihydroxybutanoic acid production in a cell, comprising the following steps:
   (a) identifying in a data bank nucleic acid molecules of an organism; which can contain a candidate gene encoding a gene product conferring an increase in the glyceric acid, citramalic acid, fumaric acid, malic acid, pyruvic acid, succinic acid, trihydroxybutyric acid or trihydroxybutanoic acid amount or level in an organism or a part thereof after expression, and which are at least 20% homolog to the nucleic acid molecule of claim 6;
   (b) introducing the candidate nucleic acid molecules in host cells appropriate for producing glyceric acid, citramalic acid, fumaric acid, malic acid, pyruvic acid, succinic acid, trihydroxybutyric acid or trihydroxybutanoic acid;
   (c) expressing the identified nucleic acid molecules in the host cells;
   (d) assaying the glyceric acid, citramalic acid, fumaric acid, malic acid, pyruvic acid, succinic acid, trihydroxybutyric acid or trihydroxybutanoic acid level in the host cells; and
   (e) identifying nucleic acid molecule and its gene product which expression confers an increase in the glyceric acid, citramalic acid, fumaric acid, malic acid, pyruvic acid, succinic acid, trihydroxybutyric acid or trihydroxybutanoic acid level in the host cell after expression compared to the wild type.
21. A method for the production of an agricultural composition comprising the steps of the method of any one of claims 17 to 20 and formulating the compound identified in any one of claims 17 to 20 in a form acceptable for an application in agriculture.
22. A composition comprising the nucleic acid molecule of claim 6, the polypeptide of claim 14, the nucleic acid construct of claim 7, the vector of any one of claims 8 or 9, an antagonist or agonist identified according to claim 17, the compound of claim 18, the gene product of claim 19 or 20, the antibody of claim 15, and optionally an agricultural acceptable carrier.
23. Use of the nucleic acid molecule as claimed in claim 6 for the identification of a nucleic acid molecule conferring an increase of glyceric acid, citramalic acid, fumaric acid, malic acid, pyruvic acid, succinic acid, trihydroxybutyric acid or trihydroxybutanoic acid after expression.
24. Use of the polypeptide of claim 14 or the nucleic acid construct claim 7 or the gene product identified according to the method of claim 19 or 20 for identifying compounds capable of conferring a modulation of glyceric acid, citramalic acid, fumaric acid, malic acid, pyruvic acid, succinic acid, trihydroxybutyric acid or trihydroxybutanoic acid levels in an organism.
25. Agrochemical, pharmaceutical, food or feed composition comprising the nucleic acid molecule of claim 6, the polypeptide of claim 14, the nucleic acid construct of claim 7, the vector of claim 8 or 9, the antagonist or agonist identified according to claim 17, the antibody of claim 15, the plant or plant tissue of claim 16, the harvested material of claim 16, the host cell of claim 10 to 12 or the gene product identified according to the method of claim 19 or 20.
26. The method of any one of claims 1 to 5, the nucleic acid molecule of claim 6, the polypeptide of claim 14, the nucleic acid construct of claim 7, the vector of claim 8 or 9, the antagonist or agonist identified according to claim 17, the antibody of claim 15, the plant or plant tissue of claim 16, the harvested material of claim 16, the host cell of claim 10 to 12 or the gene product identified according to the method of claim 19 or 20, wherein the fine chemical is glyceric acid, citramalic acid, fumaric acid, malic acid, pyruvic acid, succinic acid, trihydroxybutyric acid or trihydroxybutanoic acid.

**[0554.0.0.16] Abstract: see [0554.0.0.0]**
Process for the production of fine chemicals

### Description

**[0000.0.0.17]** In a further embodiment, the present invention relates to a further process for the production of fine chemicals as defined below and the corresponding embodiments as described herein as follows.

**[0001.0.0.17] see [0001.0.0.0]**

**[0002.0.17.17]** Gamma-aminobutyric acid is used to enhance growth of specified plants, prevent development of powdery mildew on grapes, and suppress certain other plant diseases. Humans and animals normally ingest and metabolize gamma-aminobutyric acid in variable amounts. Gamma-aminobutyric acid was registered (licensed for sale) as growth enhancing pesticidal active ingredient in 1998. Gamma-aminobutyric acid is an important signal which helps to regulate mineral availability in plants. Minerals support the biochemical pathways governing growth and reproduction as well as the pathways that direct plant's response to a variety of biotic and abiotic stresses. Mineral needs are especially high during times of stress and at certain stages of plant growth. Gamma-aminobutyric acid levels in plants naturally increase at these times.
Gamma-Aminobutyric acid (GABA), a nonprotein amino acid, is often accumulated in plants following environmental stimuli that can also cause ethylene production. Exogenous GABA causes up to a 14-fold increase in the ethylene production rate after about 12 h. GABA causes increases in ACC synthase mRNA accumulation, ACC levels, ACC oxidase mRNA levels and in vitro ACC oxidase activity. Possible roles of GABA as a signal transducer are suggested, see Plant Physiol.115(1):129-35(1997)
Gamma-aminobutyric acid (GABA), a four-carbon non-protein amino acid, is a significant component of the free amino acid pool in most prokaryotic and eukaryotic organisms. In plants, stress initiates a signal-transduction pathway, in which increased cytosolic Ca²⁺ activates Ca²⁺/calmodulin-dependent glutamate decarboxylase activity and GABA synthesis. Elevated H⁺ and substrate levels can also stimulate glutamate decarboxylase activity. GABA accumulation probably is mediated primarily by glutamate decarboxylase. Experimental evidence supports the involvement of GABA synthesis in pH regulation, nitrogen storage, plant development and defence, as well as a compatible osmolyte and an alternative pathway for glutamate utilization, see Trends Plant Sci. 4(11):446-452(1999).
Gamma-aminobutyric acid enhances nutrient uptake by roots and leaves so that plant nutrient levels are higher than those achieved by using nutrients alone. When plants are stressed and nutrient uptake is limited, it is believed that gamma-aminobutyric acid facilitates nutrient utilization, thereby enhancing growth during stress.
Rapid GABA accumulation in response to wounding may play a role in plant defense against insects (Ramputh and Brown, Plant Physiol. 111(1996): 1349-1352). The development of gamma aminobutyrate (GABA) as a potential control agent in plant - invertebrate pest systems has been reviewed in Shelp et al., Canadien Journal of Botany (2003) 81, 11, 1045-1048. The authors describe that available evidence indicates that GABA accumulation in plants in response to biotic and abiotic stresses is mediated via the activation of glutamate decarboxylase. More applied research, based on the fact that GABA acts as an inhibitory neurotransmitter in invertebrate pests, indicates that ingested GABA disrupts nerve functioning and causes damage to oblique-banded leafroller larvae, and that walking or herbivory by tobacco budworm and oblique-banded leafroller larvae stimulate GABA accumulation in soybean and tobacco, respectively. In addition, elevated levels of endogenous GABA in genetically engineered tobacco deter feeding by tobacco budworm larvae and infestation by the northern root-knot nematode. Therefore the author concluded that genetically engineered crop species having high GABA-producing potential may be an alternative strategy to chemical pesticides for the management of invertebrate pests.
During angiosperm reproduction, pollen grains form a tube that navigates through female tissues to the micropyle, delivering sperm to the egg. In vitro, GABA stimulates pollen tube growth. The Arabidopsis POP2 gene encodes a transaminase that degrades GABA and contributes to the formation of a gradient leading up to the micropyle, see Cell. 114(1):47-59(2003).
Due to these interesting physiological roles and agrobiotechnological potential of GABA there is a need to identify the genes of enzymes and other proteins involved in GABA metabolism, and to generate mutants or transgenic plant lines with which to modify the GABA content in plants.
Shikimic acid is found in various plants. It has two functional groups in the same molecule, hydroxyl groups and a carboxylic acid group which are optically active. They can yield various kinds of esters and salts. It belongs to the class of cyclitols, which means it is a hydroxylated cycloalkane containing at least three hydroxy groups, each attached to a different ring carbon atom. The term "shikimic acid" or "shikimate" relates to the anionic form as well as the neutralised status of that compound.
A key intermediate in synthesis of virtually all aromatic compounds in the cells is shikimic acid. These include phenylalanine, tyrosine, tryptophan, p-aminobenzoic acid, and p-hydroxybenzoic acid.
Glyphosate (N-phosphonomethylglycine) is a non-selective, broad spectrum herbicide that is symplastically translocated to the meristems of growing plants. It causes shikimate accumulation through inhibition of the chloroplast localized EPSP synthase (5-*eno*/pyruvylshikimate-3-phosphate synthase; EPSPs) [EC 2.5.1.19] (Amrhein et al, 1980, Plant Physiol. 66: 830-834).
The starting product of the biosynthesis of most phenolic compounds is shikimate. Phenols are acidic due to the dissociability of their -OH group. They are rather reactive compounds and as long as no steric inhibition due to additional side chains occurs, they form hydrogen bonds. Consequently, many flavonoids have intramolecular bonds. Another important feature is their ability to form chelate complexes with metals. Also, they are easily oxidized and, if so, form polymers (dark aggregates). The darkening of cut or dying plant parts is caused by this reaction. They have usually an inhibiting effect on plant growth. Among the phenylpropanol derivatives of lower molecular weight are a number of scents like the coumarins, cinnamic acid, sinapinic acid, the coniferyl alcohols and others. These substances and their derivatives are at the same time intermediates of the biosynthesis of lignin.
The shikimate pathway links metabolism of carbohydrates to biosynthesis of aromatic compounds. In a sequence of seven metabolic steps, phosphoenolpyruvate and erythrose 4-phosphate are converted to chorismate, the precursor of the aromatic amino acids and many aromatic secondary metabolites. All pathway intermediates can also be considered branch point compounds that may serve as substrates for other metabolic pathways. The shikimate pathway is found only in microorganisms and plants, never in animals. All enzymes of this pathway have been obtained in pure form from prokaryotic and eukaryotic sources and their respective DNAs have been characterized from several organisms. The cDNAs of higher plants encode proteins with amino terminal signal sequences for plastid import, suggesting that plastids are the exclusive locale for chorismate biosynthesis. In microorganisms, the shikimate pathway is regulated by feedback inhibition and by repression of the first enzyme. In higher plants, no physiological feedback inhibitor has been identified, suggesting that pathway regulation may occur exclusively at the genetic level. This difference between microorganisms and plants is reflected in the unusually large variation in the primary structures of the respective first enzymes. Several of the pathway enzymes occur in isoenzymic forms whose expression varies with changing environmental conditions and, within the plant, from organ to organ. The penultimate enzyme of the pathway is the sole target for the herbicide glyphosate. Glyphosate-tolerant transgenic plants are at the core of novel weed control systems for several crop plants (Annual Review of Plant Physiology and Plant Molecular Biology 50(1999): 473-503). The term "shikimic acid" or "shikimate" relates to the anionic form as well as the neutralised status of that compound.
Natural products derived from shikimic acid range in complexity from the very simple, such as vanillin (used primarily as a flavoring agent), salicylic acid (the precursor of aspirin), lawsone (a naphthoquinone used in some sunscreens), and scopletin (a coumarin once used as a uterine sedative), to the more complex, such as the lignan lactone podophyllotoxin. Podophyllotoxin is basically a dimer incorporating two phenylpropanoid (a nine-carbon unit derived from shikimic acid) units. Podophyllotoxin was first isolated from Podophyllum peltatum, also known as mayapple or American mandrake, a plant which has a long history of use as a cathartic and purgative. Podophyllotoxin has been used to treat warts, and is a mitotic inhibitor which shows antineoplastic activity. Etoposide, in particular, is used to treat forms of lung cancer, testicular cancer, and acute lymphocytic leukemia.
Putrescine is synthesized by healthy living cells by the action of ornithin decarboxylase, is one of the simplest polyamines and appears to be a growth factor necessary for cell division.
Experimental evidence indicate that polyamines may be involved in growth, differentiation or morphogenesis, stress and senescence in plants ( Evans and Malmberg, 1989).

**[0003.0.0.17] %**

**[0004.0.0.17] %**

**[0005.0.0.17] %**

**[0006.0.0.17] %**

**[0007.0.0.17] %**

**[0008.0.17.17]** One way to increase the productive capacity of biosynthesis is to apply recombinant DNA technology. Thus, it would be desirable to produce gamma-aminobutyric acid or shikimate in plants. That type of production permits control over quality, quantity and selection of the most suitable and efficient producer organisms. The latter is especially important for commercial production economics and therefore availability to consumers. In addition it is desireable to produce gamma-aminobutyric acid or shikimate in plants in order to increase plant productivity and resistance against biotic and abiotic stress as discussed before. The term "shikimic acid" or "shikimate" relates to the anionic form as well as the neutralised status of that compound.
Methods of recombinant DNA technology have been used for some years to improve the production of fine chemicals in microorganisms and plants by amplifying individual biosynthesis genes and investigating the effect on production of fine chemicals. It is for example reported, that the xanthophyll astaxanthin could be produced in the nectaries of transgenic tobacco plants. Those transgenic plants were prepared by *Argobacterium tumifaciens*-mediated transformation of tobacco plants using a vector that contained a ketolase-encoding gene from *H. pluvialis* denominated *crtO* along with the Pds gene from tomato as the promoter and to encode a leader sequence. Those results indicated that about 75 percent of the carotenoids found in the flower of the transformed plant contained a keto group.

**[0009.0.17.17]** Thus, it would be advantageous if an algae, plant or other microorganism were available which produce large amounts gamma-aminobutyric acid or shikimate or putrescine. The invention discussed hereinafter relates in some embodiments to such transformed prokaryotic or eukaryotic microorganisms.
It would also be advantageous if plants were available whose roots, leaves, stem, fruits or flowers produced large amounts of gamma-aminobutyric acid or shikimate or putrescine. The invention discussed hereinafter relates in some embodiments to such transformed plants.

**[0010.0.17.17]** Therefore improving the quality of foodstuffs and animal feeds is an important task of the food-and-feed industry. This is necessary since, for example gamma-aminobutyric acid or shikimate, as mentioned above, which occur in plants and some microorganisms are limited with regard to the supply of mammals. Especially advantageous for the quality of foodstuffs and animal feeds is as balanced as possible a specific gamma-aminobutyric acid or shikimate profile in the diet since an excess of gamma-aminobutyric acid or shikimate above a specific concentration in the food has a positive effect. A further increase in quality is only possible via addition of further gamma-aminobutyric acid or shikimate, which are limiting.

**[0011.0.17.17]** To ensure a high quality of foods and animal feeds, it is therefore necessary to add gamma-aminobutyric acid or shikimate in a balanced manner to suit the organism.

**[0012.0.17.17]** Accordingly, there is still a great demand for new and more suitable genes which encode enzymes or other proteins which participate in the biosynthesis of gamma-aminobutyric acid or shikimate or putrescine and make it possible to produce them specifically on an industrial scale without unwanted byproducts forming. In the selection of genes for biosynthesis two characteristics above all are particularly important. On the one hand, there is as ever a need for improved processes for obtaining the highest possible contents of gamma-aminobutyric acid, putrescine and shikimate; on the other hand as less as possible byproducts should be produced in the production process.

**[0013.0.0.17] see [0013.0.0.0]**

**[0014.0.17.17]** Accordingly, in a first embodiment, the invention relates to a process for the production of a fine chemical, whereby the fine chemical is a gamma-aminobutyric acid or shikimate or putrescine. Accordingly, in the present invention, the term "the fine chemical" as used herein relates to a gamma-aminobutyric acid or shikimate or putrescine. Further, the term "the fine chemicals" as used herein also relates to fine chemicals comprising gamma-aminobutyric acid or shikimate or putrescine.

**[0015.0.17.17]** In one embodiment, the term "the fine chemical" or "the respective fine chemical" means at least one chemical compound with gamma-aminobutyric acid or shikimate or putrescine activity.
In one embodiment, the term "the fine chemical" means a gamma-aminobutyric acid. In one embodiment, the term "the fine chemical" means shikimate depending on the context in which the term is used. In one embodiment, the term "the fine chemical" means putrescine depending on the context in which the term is used. Throughout the specification the term "the fine chemical" means gamma-aminobutyric acid or shikimate or putrescine, its salts, ester, thioester or in free form or bound to other compounds such sugars or sugarpolymers, like glucoside, e.g. diglucoside.

**[0016.0.17.17]** Accordingly, the present invention relates to a process comprising
(a) increasing or generating the activity of one or more YER156C, YFR042W, YOR084W,YLR375W, b1693, b0651, b0847 or b 2965 protein(s) in a non-human organism in one or more parts thereof; and
(b) growing the organism under conditions which permit the production of the fine chemical, thus gamma-aminobutyric acid, putrescine or shikimate in said organism.
Accordingly, the present invention relates to a process comprising.
(a) increasing or generating the activity of one or more proteins having the activity of a protein indicated in Table IIA or IIB, column 3, lines 227 to 230, 595 to 598, resp. or having the sequence of a polypeptide encoded by a nucleic acid molecule indicated in Table IA or IB, column 5 or 7, lines 227 to 230, 595 to 598, resp. in a non-human organism in one or more parts thereof; and
growing the organism under conditions which permit the production of the fine chemical, thus, gamma-aminobutyric acid, putrescine or shikimate, in said organism.

**[0016.1.17.17]** Accordingly, the term "the fine chemical" means "gamma-aminobutyric acid" in relation to all sequences listed in Table IA or IB, lines 227 to 229, 595 and 596 or homologs thereof and means "shikimate" in relation to the sequence listed in Table IA or IB, line 230 and 597 or homologs thereof and means "putrescine" in relation to the sequence listed in Table IA or IB, line 598. Accordingly, the term "the fine chemical" can mean "gamma-aminobutyric acid" or "shikimate" or "putrescine", owing to circumstances and the context. In order to illustrate that the meaning of the term "the respective fine chemical" means "gamma-aminobutyric acid "or " shikimate " or "putrescine" owing to the sequences listed in the context the term "the respective fine chemical" is also used.

**[0017.0.0.17]** to **[0018.0.0.17]: see [0017.0.0.0]** to **[0018.0.0.0]**

**[0019.0.17.17]** Advantageously the process for the production of the respective fine chemical leads to an enhanced production of the respective fine chemical. The terms "enhanced" or "increase" mean at least a 10%, 20%, 30%, 40% or 50%, preferably at least 60%, 70%, 80%, 90% or 100%, more preferably 150%, 200%, 300%, 400% or 500% higher production of the respective fine chemical in comparison to the reference as defined below, e.g. that means in comparison to an organism without the aforementioned modification of the activity of a protein having the activity of a protein indicated in Table IIA or.IIB, column 3, lines 227 to 230, 595 to 598 or encoded by nucleic acid molecule indicated in Table IA or IB, columns 5 or 7, lines 227 to 230, 595 to 598.

**[0020.0.17.17]** Surprisingly it was found, that the transgenic expression of the Saccharomyces cerevisiae proteins YER156C, YFR042W, YOR084W, YLR375W, and/or the Escherichia coli proteins b1693, b0651, b0847 or b 2965 in Arabidopsis thaliana conferred an increase in the gamma-aminobutyric acid or shikimate or putrescine ("the fine chemical" or "the fine respective chemical") content in respect to said proteins and their homologs as well as the encoding nucleic acid molecules, in particular as indicated in Table IA or IB and Table IIA or IIB, column 3, lines 227 to 230, 595 to 598 of the transformed plants.

**[0021.0.0.17]** see **[0021.0.0.0]**

**[0022.0.17.17]** The sequence of YER156C from Saccharomyces cerevisiae has been published in Dietrich, F.S et al, Nature 387 (6632 Suppl), 78-81 (1997), and its activity is being defined as an unclassified protein. Accordingly, in one embodiment, the process of the present invention comprises the use of a protein YER156C from Saccharomyces cerevisiae or its homolog, e.g. as shown herein, for the production of the respective fine chemical, in particular for increasing the amount of gamma-aminobutyric acid, preferably in free or bound form in an organism or a part thereof, as mentioned. In one embodiment, in the process of the present invention the activity of the unspecified protein YER156C is increased.
The sequence of YFR042W from Saccharomyces cerevisiae has been published in Goffeau, A. et al., Science 274 (5287), 546-547 (1996) and its activity is being defined as a protein required for cell viability. Accordingly, in one embodiment, the process of the present invention comprises the use of a protein having said activity, for the production of the respective fine chemical, in particular for increasing the amount of gamma-aminobutyric acid, preferably in free or bound form in an organism or a part thereof, as mentioned. In one embodiment, in the process of the present invention said activity required for cell viability is increased.
The sequence of YOR084W from Saccharomyces cerevisiae has been published in Dujon, B. et al, Nature 387 (6632 Suppl), 98-102 (1997) and its activity is being defined as a putative lipase of the peroxisomal martrix. Accordingly, in one embodiment, the process of the present invention comprises the use of a lipase protein of the peroxisomal martrix from Saccharomyces cerevisiae or its homolog, e.g. as shown herein, for the production of the respective fine chemical, in particular for increasing the amount of gamma-aminobutyric acid , preferably in free or bound form in an organism or a part thereof, as mentioned. In one embodiment, in the process of the present invention said activity, e.g. the activity of the lipase protein of the peroxisomal matrix is increased.
The sequence of YLR375W from Saccharomyces cerevisiae has been published in Johnston, M., Nature 387 (6632 Suppl), 87-90 (1997) and its activity is being defined as protein involved in pre-tRNA splicing and in uptake of branched-chain amino acids. Accordingly, in one embodiment, the process of the present invention comprises the use of a protein with pre-tRNA splicing and in uptake of branched-chain amino acids activity from Saccharomyces cerevisiae or its homolog, e.g. as shown herein, for the production of the respective fine chemical, in particular for increasing the amount of shikimate preferably in free or bound form in an organism pr a part thereof, as mentioned. In one embodiment, in the process of the present invention said activity, e.g. the activity of a protein involved in pre-tRNA splicing and in uptake of branched-chain amino acids is increased.
The sequence of b0651 from Escherichia coli K12 has been published in Blattner et al., Science 277 (5331), 1453-1474, 1997, and its activity is being defined as a pyrimidine specific nucleoside hydrolase. Accordingly, in one embodiment, the process of the present invention comprises the use of a protein with pyrimidine specific nucleoside hydrolase activity from Escherichia coli K12 or its homolog, e.g. as shown herein, for the production of the respective fine chemical, in particular for increasing the amount of gamma-aminobutyric acid preferably in free or bound form in an organism or a part thereof, as mentioned. In one embodiment, in the process of the present invention said activity, e.g. the activity of a protein with pyrimidine specific nucleoside hydrolase activity, is increased.
The sequence of b0847 from Escherichia coli K12 has been published in Blattner et al., Science 277 (5331), 1453-1474, 1997, and its activity is being defined as a putative transport protein. Accordingly, in one embodiment, the process of the present invention comprises the use of a protein with putative transport protein activity from Escherichia coli K12 or its homolog, e.g. as shown herein, for the production of the respective fine chemical, in particular for increasing the amount of gamma-aminobutyric acid preferably in free or bound form in an organism or a part thereof, as mentioned. In one embodiment, in the process of the present invention said activity, e.g. the activity of a transport protein b0847 is increased.
The sequence of b1693 from Escherichia coli K12 has been published in Blattner et al., Science 277 (5331), 1453-1474, 1997, and its activity is being defined as a 3-dehydroquinate dehydratase. Accordingly, in one embodiment, the process of the present invention comprises the use of a 3-dehydroquinate dehydratase protein from E. coli or its homolog, e.g. as shown herein, for the production of the fine chemical, meaning of shikimate or linoleic acid and/or tryglycerides, lipids, oils and/or fats containing shikimate, linoleic acid, in particular for increasing the amount of shikimate, linoleic acid and/or tryglycerides, lipids, oils and/or fats containing shikimate, linoleic acid, preferably linoleic acid in free or bound form in an organism or a part thereof, as mentioned. In one embodiment, in the process of the present invention the activity of a 3-dehydroquinate dehydratase protein is increased or generated, e.g. from E. coli or a homolog thereof.
The sequence of b 2965 from Escherichia coli K12 has been published in Blattner et al., Science 277 (5331), 1453-1474, 1997, and its activity is being defined as an ornithine decarboxylase isozyme. Accordingly, in one embodiment, the process of the present invention comprises the use of a protein with ornithine decarboxylase isozyme activity from Escherichia coli K12 or its homolog, e.g. as shown herein, for the production of the respective fine chemical, in particular for increasing the amount of putrescine preferably in free or bound form in an organism or a part thereof, as mentioned. In one embodiment, in the process of the present invention said activity, e.g. the activity of a protein with ornithine decarboxylase activity is increased.

**[0023.0.17.17]** Homologues ( = homologs) of the present gene products can be derived from any organisms as long as the homologue confers the herein mentioned activity, in particular, confers an increase in the respective fine chemical amount or content.
In one embodiment, the homolog of any one of the polypeptides indicated in Table IIA or IIB, column 3, line 227 is a homolog having the same or a similar activity. In particular an increase of activity confers an increase in the content of the respective fine chemical in the organisms preferably gamma-aminobutyric acid.
In one embodiment, the homolog of the polypeptides indicated in Table IIA or IIB, column 3, line 228 is a homolog having the same or a similar acitivity. In particular an increase of activity confers an increase in the content of the respective fine chemical in the organisms preferably gamma-aminobutyric acid.
In one embodiment, the homolog of the polypeptides indicated in Table IIA or IIB, column 3, line 229 is a homolog having the same or a similar acitivity. In particular an increase of activity confers an increase in the content of the respective fine chemical in the organisms preferably gamma-aminobutyric acid.
In one embodiment, the homolog of the any one of the polypeptides indicated in Table IIA or IIB, column 3, line 230 is a homolog having the same or a similar activity. In particular an increase of activity confers an increase in the content of shikimate in the organisms.
In one embodiment, the homolog of the any one of the polypeptides indicated in Table IIA or IIB, column 3, line 595 is a homolog having the same or a similar activity. In particular an increase of activity confers an increase in the content of gamma-aminobutyric acid in the organisms.
In one embodiment, the homolog of the any one of the polypeptides indicated in Table IIA or IIB, column 3, line 596 is a homolog having the same or a similar activity. In particular an increase of activity confers an increase in the content of gamma-aminobutyric acid in the organisms.
In one embodiment, the homolog of the any one of the polypeptides indicated in Table IIA or IIB, column 3, line 597 is a homolog having the same or a similar activity. In particular an increase of activity confers an increase in the content of shikimate in the organisms.
In one embodiment, the homolog of the any one of the polypeptides indicated in Table IIA or IIB, column 3, line 598 is a homolog having the same or a similar activity. In particular an increase of activity confers an increase in the content of putrescine in the organisms.

**[0023.1.0.17]** Homologs of the polypeptides indicated in Table IIA or IIB, column 3, lines 227 to 230, 595 to 598 may be the polypeptides encoded by the nucleic acid molecules indicated in Table IA or IB, column 7, lines 227 to 230, 595 to 598 or may be the polypeptides indicated in Table IIA or IIB, column 7, lines 227 to 230, 595 to 598.
Homologs of the polypeptides indicated in Table IIA or IIB, column 3, lines 227 to 229, 595, 596 may be the polypeptides encoded by the nucleic acid molecules indicated in Table IA or IB, column 7, lines 227 to 229, 595, 596, respectively or may be the polypeptides indicated in Table IIA or IIB, column 7, lines 227 to 229, 595, 596, having a gamma-aminobutyric acid content and/or amount increasing activity.
Homologs of the polypeptide indicated in Table IIA or IIB, column 3, line 230 and 597 may be the polypeptides encoded by the nucleic acid molecules indicated in Table IA or IB, column 7, line 230 and 597, respectively or may be the polypeptides indicated in Table IIA or IIB, column 7, line 230 and 597, having a shikimate content and/or amount increasing activity.
Homologs of the polypeptide indicated in Table IIA or IIB, column 3, line 598 may be the polypeptides encoded by the nucleic acid molecules indicated in Table IA or IB, column 7, line 598, respectively or may be the polypeptides indicated in Table IIA or IIB, column 7, line 598 having a putrescine content and/or amount increasing activity.

**[0024.0.0.17]** see **[0024.0.0.0]**

**[0025.0.17.17]** In accordance with the invention, a protein or polypeptide has the "activity of a protein of the invention", e.g. the activity of a protein indicated in Table IIA or IIB, column 3, lines 227 to 229 or line 230 or lines 595 to 598 if its *de novo* activity, or its increased expression directly or indirectly leads to an increased gamma-aminobutyric acid or shikimate or putrescine level, resp., in the organism or a part thereof, preferably in a cell of said organism. In a preferred embodiment, the protein or polypeptide has the above-mentioned additional activities of a protein indicated in Table IIA or IIB, column 3, lines 227 to 229 or line 230 or lines 595 to 598. Throughout the specification the activity or preferably the biological activity of such a protein or polypeptide or a nucleic acid molecule or sequence encoding such protein or polypeptide is identical or similar if it still has the biological or enzymatic activity of any one of the proteins indicated in Table IIA or IIB, column 3, lines 227 to 229 or line 230 or lines 595 to 598, or which has at least 10% of the original enzymatic activity, preferably 20%, particularly preferably 30%, most particularly preferably 40% in comparison to any one of the proteins indicated in Table IIA or IIB, column 3, line 227 to 230, 595 to 598 of Saccharomyces cerevisiae.
In one embodiment, the polypeptide of the invention confers said activity, e.g. the increase of the respective fine chemical in an organism or a part thereof, if it is derived from an organism, which is evolutionary close to the organism indicated in Table IA or IB, column 4 and is expressed in an organism, which is evolutionary distant to the origin organism. For example origin and expressing organism are derived from different families, orders, classes or phylums whereas origin and the organism indicated in Table IA or IB, column 4 are derived from the same families, orders, classes or phylums.

**[0025.1.0.17] see [0025.1.0.0]**

**[0026.0.0.17] to [0033.0.0.17]: see [0026.0.0.0] to [0033.0.0.0]**

**[0034.0.17.17]** Preferably, the reference, control or wild type differs form the subject of the present invention only in the cellular activity of the polypeptide of the invention, e.g. as result of an increase in the level of the nucleic acid molecule of the present invention or an increase of the specific activity of the polypeptide of the invention. E.g., it differs by or in the expression level or activity of an protein having the activity of a protein as indicated in Table IIA or IIB, column 3, lines 227 to 229 or line 230 or lines 595 to 598 or being encoded by a nucleic acid molecule indicated in Table IA or IB, column 5, lines 227 to 229 or line 230 or 595 to 598 or its homologs, e.g. as indicated in Table IA or IB, column 7, lines 227 to 229 or line 230 or lines 595 to 598, its biochemical or genetic causes. It therefore shows the increased amount of the respective fine chemical.

**[0035.0.0.17] to [0038.0.0.17]: see [0035.0.0.0] to [0038.0.0.0]**

**[0039.0.0.17]: see [0039.0.0.0]**

**[0040.0.0.17] to [0044.0.0.17]: see [0040.0.0.0] to [0044.0.0.0]**

**[0045.0.17.17]** In case the activity of the Saccharomyces cerevisae protein YER156C or its homologs, e.g. as indicated in Table IIA or IIB, columns 5 or 7, line 227, is increased, in one embodiment the increase of the respective fine chemical, preferably of gamma-aminobutyric acid between 68 % and 1029% or more is conferred.
In one embodiment, in case the activity of the Saccharomyces cerevisae protein YFR042W or its homologs, e.g. as indicated in Table IIA or IIB, columns 5 or 7, line 228 is increased, preferably, in one embodiment the increase of the respective fine chemical, preferably of gamma-aminobutyric acid between 91 % and 110% or more is conferred.
In one embodiment, in case the activity of the Saccharomyces cerevisae protein YOR084W or its homologs e.g. a protein of a putative lipase of the peroxisomal matrix as indicated in Table IIA or IIB, columns 5 or 7, line 229, is increased, preferably, in one embodiment the increase of the respective fine chemical, preferably of gamma-aminobutyric acid between 71 % and 459% or more is conferred.
In one embodiment, in case the activity of the Saccharomyces cerevisiae protein YLR375W or its homologs, e.g. a protein involved in pre-tRNA splicing and in uptake of branched-chain amino acids as indicated in Table IIA or IIB, columns 5 or 7, line 230, is increased, preferably, in one embodiment an increase of the respective fine chemical, preferably of shikimate between 14% and 26% or more is conferred.
In one embodiment, in case the activity of the Escherichia coli protein b 0651 or its homologs, e.g. a protein with pyrimidine specific nucleoside hydrolase activity as indicated in Table IIA or IIB, columns 5 or 7, line 595, is increased, preferably, in one embodiment an increase of the respective fine chemical, preferably of gamma-aminobutyric acid between 53% and 206% or more is conferred.
In one embodiment, in case the activity of the Escherichia coli protein b 0847 or its homologs, e.g. a protein with putative transport protein activity as indicated in Table IIA or IIB, columns 5 or 7, line 596, is increased, preferably, in one embodiment an increase of the respective fine chemical, preferably of gamma-aminobutyric acid between 85% and 187% or more is conferred.
In one embodiment, in case the activity of the Escherichia coli protein b 1693 or its homologs, e.g. a protein with 3-dehydroquinate dehydratase activity as indicated in Table IIA or IIB, columns 5 or 7, line 597, is increased, preferably, in one embodiment an increase of the respective fine chemical, preferably of shikimate between 15% and 31% or more is conferred.
In one embodiment, in case the activity of the Escherichia coli protein b 2965 or its homologs, e.g. a protein with ornithine decarboxylase activity as indicated in Table IIA or IIB, columns 5 or 7, line 598, is increased, preferably, in one embodiment an increase of the respective fine chemical, preferably of putrescine between 111 % and 1693% or more is conferred.

**[0046.0.17.17] %**

**[0047.0.0.17] to [0048.0.0.17]: see [0047.0.0.0] to [0048.0.0.0]**

**[0049.0.17.17]** A protein having an activity conferring an increase in the amount or level of the respective fine chemical gamma-aminobutyric acid preferably has the structure of the polypeptide described herein. In a particular embodiment, the polypeptides used in the process of the present invention or the polypeptide of the present invention comprises the sequence of a consensus sequence as shown in SEQ ID NO: 15722, 15723, 15724, 15725, 15726, 15727 or 15778, 15779, 91412, 91413, 91414, 91525, 91526, 91527, 91528, 91529 or as indicated in Table IV, column 7, lines 227, 228, 229, 595 and 596 or of a functional homologue thereof as described herein, or of a polypeptide encoded by the nucleic acid molecule characterized herein or the nucleic acid molecule according to the invention, for example by a nucleic acid molecule as indicated in Table IA or IB, columns 5 or 7, lines 227, 228, 229, 595 and 596 or its herein described functional homologues and has the herein mentioned activity conferring an increase in the gamma-aminobutyric acid level.
A protein having an activity conferring an increase in the amount or level of the shikimate preferably has the structure of the polypeptide described herein. In a particular embodiment, the polypeptides used in the process of the present invention or the polypeptide of the present invention comprises the sequence of a consensus sequence as shown in SEQ ID NO: 15768, 15769, 15770, 15771, 91630, 91631, 91632, 91633, 91634 e.g. as indicated in Table IV, column 7, line 230, 597 or of a polypeptide as indicated in Table IIA or IIB, columns 5 or 7, line 230, 597 or of a functional homologue thereof as described herein, or of a polypeptide encoded by the nucleic acid molecule characterized herein or the nucleic acid molecule according to the invention, for example by a nucleic acid molecule as indicated in Table IA or IB, columns 5 or 7, line 230, 597 or its herein described functional homologues and has the herein mentioned activity confering an increase in the shikimate level.
A protein having an activity conferring an increase in the amount or level of the putrescine preferably has the structure of the polypeptide described herein. In a particular embodiment, the polypeptides used in the process of the present invention or the polypeptide of the present invention comprises the sequence of a consensus sequence as shown in SEQ ID NO: 91831, 91832, 91833, 91834, 91835, 91836 e.g. as indicated in Table IV, column 7, line 598 or of a polypeptide as indicated in Table IIA or IIB, columns 5 or 7, line 598 or of a functional homologue thereof as described herein, or of a polypeptide encoded by the nucleic acid molecule characterized herein or the nucleic acid molecule according to the invention, for example by a nucleic acid molecule as indicated in Table IA or IB, columns 5 or 7, line 598 or its herein described functional homologues and has the herein mentioned activity confering an increase in the putrescine level.

**[0050.0.17.17]** For the purposes of the present invention, the term "the respective fine chemical" also encompass the corresponding salts, such as, for example, the potassium or sodium salts of gamma-aminobutyric acid or shikimate, resp., or their ester, or glucoside thereof, e.g the diglucoside thereof.

**[0051.0.17.17]** Owing to the biological activity of the proteins which are used in the process according to the invention and which are encoded by nucleic acid molecules according to the invention, it is possible to produce compositions comprising the respective fine chemical, i.e. an increased amount of the free chemical free or bound, e.g compositions comprising gamma-aminobutyric acid or shikimate or putrescine. Depending on the choice of the organism used for the process according to the present invention, for example a microorganism or a plant, compositions or mixtures of gamma-aminobutyric acid or shikimate or putrescine can be produced.

**[0052.0.0.17] see [0052.0.0.0]**

**[0053.0.17.17]** In one embodiment, the process of the present invention comprises one or more of the following steps
a) stabilizing a protein conferring the increased expression of a protein encoded by the nucleic acid molecule of the invention or of the polypeptid of the invention, e.g. of a polypeptide having an activity of a protein as indicated in Table IIA or IIB, column 3, lines 227 to 229 or line 230 or lines 595 to 598 or its homologs, e.g. as indicated in Table IIA or IIB, columns 5 or 7, lines 227 to 229 or line 230 or lines 595 to 598, activity having herein-mentioned the respective fine chemical increasing activity;
b) stabilizing a mRNA conferring the increased expression of a protein encoded by the nucleic acid molecule of the invention, e.g. of a polypeptide having an activity of a protein as indicated in Table IIA or IIB, column 3, lines 227 to 229 or line 230 or lines 595 to 598 or its homologs activity, e.g. as indicated in Table IIA or IIB, columns 5 or 7, lines 227 to 229 or line 230 or lines 595 to 598, or of a mRNA encoding the polypeptide of the present invention having herein-mentioned the respective fine chemical increasing activity;
c) increasing the specific activity of a protein conferring the increased expression of a protein encoded by the nucleic acid molecule of the invention or of the polypeptide of the present invention having herein-mentioned the respective fine chemical increasing activity, e.g. of a polypeptide having an activity of a protein as indicated in Table IIA or IIB, column 3, lines 227 to 229 or line 230 or lines 595 to 598 or its homologs activity, e.g. as indicated in Table IIA or IIB, columns 5 or 7, lines 227 to 229 or line 230 or lines 595 to 598, or decreasing the inhibitory regulation of the polypeptide of the invention;
d) generating or increasing the expression of an endogenous or artificial transcription factor mediating the expression of a protein conferring the increased expression of a protein encoded by the nucleic acid molecule of the invention or of the polypeptide of the invention having herein-mentioned the respective fine chemical increasing activity, e.g. of a polypeptide having an activity of a protein as indicated in Table IIA or IIB, column 3, lines 227 to 229 or line 230 or lines 595 to 598 or its homologs activity, e.g. as indicated in Table IIA or IIB, columns 5 or 7, lines 227 to 229 or line 230 or lines 595 to 598;
e) stimulating activity of a protein conferring the increased expression of a protein encoded by the nucleic acid molecule of the present invention or a polypeptide of the present invention having herein-mentioned the respective fine chemical increasing activity, e.g. of a polypeptide having an activity of a protein as indicated in Table IIA or IIB, column 3, lines 227 to 229 or line 230 or lines 595 to 598 or its homologs activity, e.g. as indicated in Table IIA or IIB, columns 5 or 7, lines 227 to 229 or line 230 or lines 595 to 598, by adding one or more exogenous inducing factors to the organism or parts thereof;
f) expressing a transgenic gene encoding a protein conferring the increased expression of a polypeptide encoded by the nucleic acid molecule of the present invention or a polypeptide of the present invention, having herein-mentioned the respective fine chemical increasing activity, e.g. of a polypeptide having an activity of a protein as indicated in Table IIA or IIB, column 3, lines 227 to 229 or line 230 or lines 595 to 598 or its homologs activity, e.g. as indicated in Table IIA or IIB, columns 5 or 7, lines 227 to 229 or line 230 or lines 595 to 598, and/or
g) increasing the copy number of a gene conferring the increased expression of a nucleic acid molecule encoding a polypeptide encoded by the nucleic acid molecule of the invention or the polypeptide of the invention having herein-mentioned the respective fine chemical increasing activity, e.g. of a polypeptide having an activity of a protein as indicated in Table IIA or IIB, column 3, lines 227 to 229 or line 230 or lines 595 to 598 or its homologs, e.g. as indicated in Table IIA or IIB, columns 5 or 7, lines 227 to 229 or line 230 or lines 595 to 598, activity.
h) Increasing the expression of the endogenous gene encoding the polypeptide of the invention, e.g. a polypeptide having an activity of a protein as indicated in Table IIA or IIB, column 3, lines 227 to 229 or line 230 or lines 595 to 598 or its homologs activity, e.g. as indicated in Table IIA or IIB, columns 5 or 7, lines 227 to 229 or line 230 or lines 595 to 598, by adding positive expression or removing negative expression elements, e.g. homologous recombination can be used to either introduce positive regulatory elements like for plants the 35S enhancer into the promoter or to remove repressor elements form regulatory regions. Further gene conversion methods can be used to disrupt repressor elements or to enhance to activity of positive elements. Positive elements can be randomly introduced in plants by T-DNA or transposon mutagenesis and lines can be identified in which the positive elements have be integrated near to a gene of the invention, the expression of which is thereby enhanced; and/or
i) Modulating growth conditions of an organism in such a manner, that the expression or activity of the gene encoding the protein of the invention or the protein itself is enhanced for example microorganisms or plants can be grown for example under a higher temperature regime leading to an enhanced expression of heat shock proteins, which can lead to an enhanced respective fine chemical production.
j) selecting of organisms with especially high activity of the proteins of the invention from natural or from mutagenized resources and breeding them into the target organisms, e.g. the elite crops.

**[0054.0.17.17]** Preferably, said mRNA is the nucleic acid molecule of the present invention and/or the protein conferring the increased expression of a protein encoded by the nucleic acid molecule of the present invention or the polypeptide having the herein mentioned activity is the polypeptide of the present invention, e.g. conferring the increase of the respective fine chemical after increasing the expression or activity of the encoded polypeptide or having the activity of a polypeptide having an activity of a protein as indicated in Table IIA or IIB, columns 3 or 5, lines 227 to 229 or line 230 or lines 595 to 598, resp., or its homologs activity, e.g. as indicated in Table IIA or IIB, columns 5 or 7, lines 227 to 229 or line 230 or lines 595 to 598, resp.

**[0055.0.0.17] to [0067.0.0.17]: see [0055.0.0.0] to [0067.0.0.0]**

**[0068.0.17.17]** The mutation is introduced in such a way that the production of gamma-aminobutyric acid or shikimate or putrescine is not adversely affected.

**[0069.0.0.17] see [0069.0.0.0]**

**[0070.0.17.17]** Owing to the introduction of a gene or a plurality of genes conferring the expression of the nucleic acid molecule of the invention or the polypeptide of the invention, for example the nucleic acid construct mentioned below, or encoding a protein of the invention into an organism alone or in combination with other genes, it is possible not only to increase the biosynthetic flux towards the end product, but also to increase, modify or create *de novo* an advantageous, preferably novel metabolite composition in the organism, e.g. an advantageous composition ofgamma-aminobutyric acid and shikimate or their biochemical derivatives , e.g. comprising a higher content of (from a viewpoint of nutrional physiology limited) gamma-aminobutyric acid and shikimate or putrescine or their derivatives.

**[0071.0.0.17] see [0071.0.0.0]**

**[0072.0.17.17]** By influencing the metabolism thus, it is possible to produce, in the process according to the invention, further advantageous compounds. Examples of such compounds are, in addition to shikimate their biochemical derivatives like chorismate, prephenate, anthranilate, phenylpyruvate, phenylalanine, 4-hydroxyphenylbutyrate, tyrosine, tryptophan, vanillin, salicylic acid, lawsone or scopletin.

**[0073.0.17.17]** Accordingly, in one embodiment, the process according to the invention relates to a process, which comprises:
providing a non-human organism, preferably a microorganism, a non-human animal, a plant or animal cell, a plant or animal tissue or a plant;
increasing an activity of a polypeptide of the invention or a homolog thereof, e.g. as indicated in Table IIA or IIB, columns 5 or 7, lines 227 to 229 or line 230 or lines 595 to 598, or of a polypeptide being encoded by the nucleic acid molecule of the present invention and described below, e.g. conferring an increase of the respective fine chemical in an organism, preferably in a microorganism, a non-human animal, a plant or animal cell, a plant or animal tissue or a plant,
growing an organism, preferably a microorganism, a non-human animal, a plant or animal cell, a plant or animal tissue or a plant under conditions which permit the production of the respective fine chemical in the organism, preferably the microorganism, the plant cell, the plant tissue or the plant; and
if desired, recovering, optionally isolating, the free and/or bound the respective fine chemical synthesized by the organism, the microorganism, the non-human animal, the plant or animal cell, the plant or animal tissue or the plant.

**[0074.0.17.17]** The organism, in particular the microorganism, non-human animal, the plant or animal cell, the plant or animal tissue or the plant is advantageously grown in such a way that it is not only possible to recover, if desired isolate the free or bound respective fine chemical.

**[0075.0.0.17] to [0077.0.0.17]: see [0075.0.0.0] to [0077.0.0.0]**

**[0078.0.17.17]** The organism such as microorganisms or plants or the recovered, and if desired isolated, the respective fine chemical can then be processed further directly into foodstuffs or animal feeds or for other applications. The fermentation broth, fermentation products, plants or plant products can be purified with methods known to the person skilled in the art. Products of these different work-up procedures are gamma-aminobutyric acid or shikimate or putrescine or comprising compositions of gamma-butyric acid or shikimate or putrescine still comprising fermentation broth, plant particles and cell components in different amounts, advantageously in the range of from 0 to 99% by weight, preferably below 80% by weight, especially preferably below 50% by weight.

**[0079.0.0.17] to [0084.0.0.17]: see [0079.0.0.0] to [0084.0.0.0]**

**[0084.0.17.17] %**

**[0085.0.17.17]** With regard to the nucleic acid sequence as depicted a nucleic acid construct which contains a nucleic acid sequence mentioned herein or an organism (= transgenic organism) which is transformed with said nucleic acid sequence or said nucleic acid construct, "transgene" means all those constructs which have been brought about by genetic manipulation methods, preferably in which either
a) a nucleic acid sequence as indicated in Table IA or IB, columns 5 or 7, lines 227 to 229 or line 230 or lines 595 to 598, or a derivative thereof, or
b) a genetic regulatory element, for example a promoter, which is functionally linked to the nucleic acid sequence as indicated in Table IA or IB, columns 5 or 7, lines 227 to 229 or line 230 or lines 595 to 598, or a derivative thereof, or
c) (a) and (b)
is/are not present in its/their natural genetic environment or has/have been modified by means of genetic manipulation methods, it being possible for the modification to be, by way of example, a substitution, addition, deletion, inversion or insertion of one or more nucleotide. "Natural genetic environment" means the natural chromosomal locus in the organism of origin or the presence in a genomic library. In the case of a genomic library, the natural, genetic environment of the nucleic acid sequence is preferably at least partially still preserved. The environment flanks the nucleic acid sequence at least on one side and has a sequence length of at least 50 bp, preferably at least 500 bp, particularly preferably at least 1000 bp, very particularly preferably at least 5000 bp.

**[0086.0.0.17] to [0087.0.0.17]: see [0086.0.0.0] to [0087.0.0.0]**

**[0088.0.17.17]** In an advantageous embodiment of the invention, the organism takes the form of a plant whose content of the respective fine chemical is modified advantageously owing to the nucleic acid molecule of the present invention expressed. This is important for plant breeders since, for example, the nutritional value of plants for poultry is dependent on the abovementioned fine chemicals or the plants are more resistant to biotic and abiotic stress and the yield is increased.

**[0088.1.0.17] see [0088.1.0.0]**

**[0089.0.0.17] to [0090.0.0.17]: see [0089.0.0.0] to [0090.0.0.0]**

**[0091.0.0.17] see [0091.0.0.0]**

**[0092.0.0.17] to [0094.0.0.17]: see [0092.0.0.0] to [0094.0.0.0]**

**[0095.0.17.17]** It may be advantageous to increase the pool of gamma-aminobutyric acid or shikimate or putrescine in the transgenic organisms by the process according to the invention in order to isolate high amounts of the pure respective fine chemical and/or to obtain increased resistance against biotic and abiotic stresses and to obtain higher yield.

**[0096.0.17.17]** In another preferred embodiment of the invention a combination of the increased expression of the nucleic acid sequence or the protein of the invention together with the transformation of a protein or polypeptid or a compound, which functions as a sink for the desired fine chemical, for example in the organism, is useful to increase the production of the respective fine chemical.

**[0097.0.17.17] %**

**[0098.0.17.17]** In a preferred embodiment, the respective fine chemical is produced in accordance with the invention and, if desired, is isolated.

**[0099.0.10.17]** In the case of the fermentation of microorganisms, the abovementioned desired fine chemical may accumulate in the medium and/or the cells. If microorganisms are used in the process according to the invention, the fermentation broth can be processed after the cultivation. Depending on the requirement, all or some of the biomass can be removed from the fermentation broth by separation methods such as, for example, centrifugation, filtration, decanting or a combination of these methods, or else the biomass can be left in the fermentation broth. The fermentation broth can subsequently be reduced, or concentrated, with the aid of known methods such as, for example, rotary evaporator, thin-layer evaporator, falling film evaporator, by reverse osmosis or by nanofiltration. Afterwards advantageously further compounds for formulation can be added such as corn starch or silicates. This concentrated fermentation broth advantageously together with compounds for the formulation can subsequently be processed by lyophilization, spray drying, and spray granulation or by other methods. Preferably the respective fine chemical comprising compositions are isolated from the organisms, such as the microorganisms or plants or the culture medium in or on which the organisms have been grown, or from the organism and the culture medium, in the known manner, for example via extraction, distillation, crystallization, chromatography or a combination of these methods. These purification methods can be used alone or in combination with the aforementioned methods such as the separation and/or concentration methods.

**[0100.0.17.17]** Transgenic plants which comprise the fine chemicals such as gamma-aminobutyric acid or shikimate or putrescine synthesized in the process according to the invention can advantageously be marketed directly without there being any need for the fine chemicals synthesized to be isolated. Plants for the process according to the invention are listed as meaning intact plants and all plant parts, plant organs or plant parts such as leaf, stem, seeds, root, tubers, anthers, fibers, root hairs, stalks, embryos, calli, cotelydons, petioles, harvested material, plant tissue, reproductive tissue and cell cultures which are derived from the actual transgenic plant and/or can be used for bringing about the transgenic plant. In this context, the seed comprises all parts of the seed such as the seed coats, epidermal cells, seed cells, endosperm or embryonic tissue.
However, the respective fine chemical produced in the process according to the invention can also be isolated from the organisms, advantageously plants, as extracts, e.g. ether, alcohol, or other organic solvents or water containing extract and/or free fine chemicals. The respective fine chemical produced by this process can be obtained by harvesting the organisms, either from the crop in which they grow, or from the field. This can be done via pressing or extraction of the plant parts. To increase the efficiency of extraction it is beneficial to clean, to temper and if necessary to hull and to flake the plant material. To allow for greater ease of disruption of the plant parts, specifically the seeds, they can previously be comminuted, steamed or roasted. Seeds, which have been pretreated in this manner can subsequently be pressed or extracted with solvents such as warm hexane. The solvent is subsequently removed. In the case of microorganisms, the latter are, after harvesting, for example extracted directly without further processing steps or else, after disruption, extracted via various methods with which the skilled worker is familiar. Thereafter, the resulting products can be processed further, i.e. degummed and/or refined. In this process, substances such as the plant mucilages and suspended matter can be first removed. What is known as desliming can be affected enzymatically or, for example, chemico-physically by addition of acid such as phosphoric acid.
Because gamma-aminobutyric acid or shikimate or putrescine in microorganisms are localized intracellular, their recovery essentially comes down to the isolation of the biomass. Well-established approaches for the harvesting of cells include filtration, centrifugation and coagulation/flocculation as described herein. Of the residual hydrocarbon, adsorbed on the cells, has to be removed. Solvent extraction or treatment with surfactants have been suggested for this purpose.

**[0101.0.10.17]** The identity and purity of the compound(s) isolated can be determined by prior-art techniques. They encompass high-performance liquid chromatography (HPLC), gas chromatography (GC), spectroscopic methods, mass spectrometry (MS), staining methods, thin-layer chromatography, NIRS, enzyme assays or microbiological assays. These analytical methods are compiled in: Patek et al. (1994) Appl. Environ. Microbiol. 60:133-140; Malakhova et al. (1996) Biotekhnologiya 11 27-32; and Schmidt et al. (1998) Bioprocess Engineer. 19:67-70. Ulmann's Encyclopedia of Industrial Chemistry (1996) Bd. A27, VCH Weinheim, pp. 89-90, pp. 521-540, pp. 540-547, pp. 559-566, 575-581 and pp. 581-587; Michal,'G (1999) Biochemical Pathways: An Atlas of Biochemistry and Molecular Biology, John Wiley and Sons; Fallon, A. et al. (1987) Applications of HPLC in Biochemistry in: Laboratory Techniques in Biochemistry and Molecular Biology, vol. 17.

**[0102.0.17.17]** Gamma-aminobutyric acid or shikimate or putrescine can for example be detected advantageously via HPLC, LC or GC separation methods. The unambiguous detection for the presence of gamma-aminobutyric acid or shikimate containing or putrescine products can be obtained by analyzing recombinant organisms using analytical standard methods: LC, LC-MS, MS or TLC). The material to be analyzed can be disrupted by sonication, grinding in a glass mill, liquid nitrogen and grinding, cooking, or via other applicable methods.

**[0103.0.17.17]** In a preferred embodiment, the present invention relates to a process for the production of the respective fine chemical comprising or generating in an organism or a part thereof the expression of at least one nucleic acid molecule comprising a nucleic acid molecule selected from the group consisting of:
a) nucleic acid molecule encoding, preferably at least the mature form, of the polypeptide having a sequence as indicated in Table IIA or IIB, columns 5 or 7, lines 227 to 229 or line 230 or lines 595 to 598 or a fragment thereof, which confers an increase in the amount of the respective fine chemical in an organism or a part thereof;
b) nucleic acid molecule comprising, preferably at least the mature form, of a nucleic acid molecule having a sequence as indicated in Table IA or IB, columns 5 or 7, lines 227 to 229 or line 230 or lines 595 to 598,
c) nucleic acid molecule whose sequence can be deduced from a polypeptide sequence encoded by a nucleic acid molecule of (a) or (b) as result of the degeneracy of the genetic code and conferring an increase in the amount of the respective fine chemical in an organism or a part thereof;
d) nucleic acid molecule encoding a polypeptide which has at least 50% identity with the amino acid sequence of the polypeptide encoded by the nucleic acid molecule of (a) to (c) and conferring an increase in the amount of the respective fine chemical in an organism or a part thereof;
e) nucleic acid molecule which hybridizes with a nucleic acid molecule of (a) to (c) under under stringent hybridization conditions and conferring an increase in the amount of the respective fine chemical in an organism or a part thereof;
f) nucleic acid molecule encoding a polypeptide, the polypeptide being derived by substituting, deleting and/or adding one or more amino acids of the amino acid sequence of the polypeptide encoded by the nucleic acid molecules (a) to (d), preferably to (a) to (c) and conferring an increase in the amount of the respective fine chemical in an organism or a part thereof;
g) nucleic acid molecule encoding a fragment or an epitope of a polypeptide which is encoded by one of the nucleic acid molecules of (a) to (e), preferably to (a) to (c) and and conferring an increase in the amount of the respective fine chemical in an organism or a part thereof;
h) nucleic acid molecule comprising a nucleic acid molecule which is obtained by amplifying nucleic acid molecules from a cDNA library or a genomic library using the primers pairs having a sequence as indicated in Table III, columns 7, lines 227 to 229 or line 230 or lines 595 to 598, and conferring an increase in the amount of the respective fine chemical in an organism or a part thereof;
i) nucleic acid molecule encoding a polypeptide which is isolated, e.g. from an expression library, with the aid of monoclonal antibodies against a polypeptide encoded by one of the nucleic acid molecules of (a) to (h), preferably to (a) to (c), and and conferring an increase in the amount of the respective fine chemical in an organism or a part thereof;
j) nucleic acid molecule which encodes a polypeptide comprising the consensus sequence having a sequences as indicated in Table IV, columns 7, lines 227 to 229 or line 230 or lines 595 to 598 and conferring an increase in the amount of the respective fine chemical in an organism or a part thereof;
k) nucleic acid molecule comprising one or more of the nucleic acid molecule encoding the amino acid sequence of a polypeptide encoding a domain of the polypeptide indicated in Table IIA or IIB, columns 5 or 7, lines 227 to 229 or line 230 or lines 595 to 598, and conferring an increase in the amount of the respective fine chemical in an organism or a part thereof; and
l) nucleic acid molecule which is obtainable by screening a suitable library under stringent conditions with a probe comprising one of the sequences of the nucleic acid molecule of (a) to (k), preferably to (a) to (c), or with a fragment of at least 15 nt, preferably 20 nt, 30 nt, 50 nt, 100 nt, 200 nt or 500 nt of the nucleic acid molecule characterized in (a) to (k), preferably to (a) to (c), and conferring an increase in the amount of the respective fine chemical in an organism or a part thereof;
or which comprises a sequence which is complementary thereto.
**[0104.0.17.17]** In one embodiment, the nucleic acid molecule used in the process of the present ivention distinguishes over the sequence indicated in Table IA or IB, columns 5 or 7, lines 227 to 229 or line 230 or lines 595 to 598 by one or more nucleotides. In one embodiment, the nucleic acid molecule used in the process of the invention does not consist of the sequence indicated in Table IA or IB, columns 5 or 7, lines 227 to 229 or line 230 or lines 595 to 598. In one embodiment, the nucleic acid molecule of the present invention is less than 100%, 99,999%, 99,99%, 99,9% or 99% identical to the sequence indicated in Table IA or IB, columns 5 or 7, lines 227 to 229 or line 230 or lines 595 to 598. In another embodiment, the nucleic acid molecule does not encode a polypeptide of a sequence indicated in Table IIA or IIB, columns 5 or 7, lines 227 to 229 or line 230 or lines 595 to 598.

**[0105.0.0.17] to [0107.0.0.17]: see [0105.0.0.0] to [0107.0.0.0]**

**[0108.0.17.17]** Nucleic acid molecules with the sequence as indicated in Table IA or IB, columns 5 or 7, lines 227 to 229 or line 230 or lines 595 to 598, nucleic acid molecules which are derived from an amino acid sequences as indicated in Table IIA or IIB, columns 5 or 7, lines 227 to 229 or line 230 or lines 595 to 598 or from polypeptides comprising the consensus sequence as indicated in Table IV, column 7, lines 227 to 229 or lines 230 or lines 595 to 598, or their derivatives or homologues encoding polypeptides with the enzymatic or biological activity of an activity of a polypeptide as indicated in Table IIA or IIB, column 3, 5 or 7, lines 227 to 229 or line 230 or lines 595 to 598, e.g. conferring the increase of the respective fine chemical, meaning gamma-aminobutyric acid or shikimate or putrescine, resp., after increasing its expression or activity, are advantageously increased in the process according to the invention.

**[0109.0.17.17]** In one embodiment, said sequences are cloned into nucleic acid constructs, either individually or in combination. These nucleic acid constructs enable an optimal synthesis of the respective fine chemicals, in particular gamma-aminobutyric acid or shikimate or putrescine, produced in the process according to the invention.

**[0110.0.0.17] see [0110.0.0.0]**

**[0111.0.0.17] see [0111.0.0.0]**

**[0112.0.17.17]** The nucleic acid molecules used in the process according to the invention take the form of isolated nucleic acid sequences, which encode polypeptides with an activity of a polypeptide as indicated in Table IIA or IIB, column 3, lines 227 to 229 or line 230 or lines 595 to 598 or having the sequence of a polypeptide as indicated in Table IIA or IIB, columns 5 and 7, lines 227 to 229 or line 230 or lines 595 to 598 and conferring an increase in the gamma-aminobutyric acid or shikimate or putrescine level.

**[0113.0.0.17] to [0114.0.0.17]: see [0113.0.0.0] to [0114.0.0.0]**

**[0115.0.0.17] see [0115.0.0.0]**

**[0116.0.0.17] to [0120.0.0.17] see [0116.0.0.0] to [0120.0.0.0]**

**[0120.1.0.17]: %**

**[0121.0.17.17]** However, it is also possible to use artificial sequences, which differ in one or more bases from the nucleic acid sequences found in organisms, or in one or more amino acid molecules from polypeptide sequences found in organisms, in particular from the polypeptide sequences indicated in Table IIA or IIB, columns 5 or 7, lines 227 to 229 or line 230 or lines 595 to 598 or the functional homologues thereof as described herein, preferably conferring above-mentioned activity, i.e. conferring a gamma-aminobutyric acid level increase after increasing the activity of the polypeptide sequences indicated in Table IIA or IIB, columns 5 or 7, lines 227 to 229, 595 or 596 or conferring a shikimate level increase after increasing the activity of the polypeptide sequences indicated in Table IIA or IIB, columns 5 or 7, lines 230 or 597 or conferring a putrescin level increase after increasing the activity of the polypeptide sequences indicated in Table IIA or IIB, columns 5 or 7, lines 598.

**[0122.0.0.17] to [0127.0.0.17]: see [0122.0.0.0] to [0127.0.0.0]**

**[0128.0.17.17]** Synthetic oligonucleotide primers for the amplification, e.g. as the pairs indicated in Table III, columns 7, lines 227 to 229 or line 230 or lines 595 to 598, by means of polymerase chain reaction can be generated on the basis of a sequence shown herein, for example the sequence as indicated in Table IA or IB, columns 5 or 7, lines 227 to 229, resp. or line 230 or lines 595 to 598, resp. or the sequences derived from a sequences as indicated in Table IIA or IIB, columns 5 or 7, lines 227 to 229, resp. or line 230 or lines 595 to 598, resp.

**[0129.0.17.17]** Moreover, it is possible to identify conserved regions from various organisms by carrying out protein sequence alignments with the polypeptide used in the process of the invention, in particular with sequences of the polypeptide of the invention, from which conserved regions, and in turn, degenerate primers can be derived. Conserved region for the polypeptide of the invention are indicated in the alignments shown in the figures. Conserved regions are those, which show a very little variation in the amino acid in one particular position of several homologs from different origin. The consensus sequence indicated in Table IV, columns 7, lines 227 and 228 or line 230 or lines 595 to 598 is derived from said alignments.

**[0130.0.17.17]** Degenerated primers can then be utilized by PCR for the amplification of fragments of novel proteins having above-mentioned activity, e.g. conferring the increase of gamma-aminobutyric acid or shikimate after increasing the expression or activity the protein comprising said fragment.

**[0131.0.0.17] to [0138.0.0.17]: see [0131.0.0.0] to [0138.0.0.0]**

**[0139.0.17.17]** Polypeptides having above-mentioned activity, i.e. conferring the respective fine chemical level increase, derived from other organisms, can be encoded by other DNA sequences which hybridise to a sequence indicated in Table IA or IB, columns 5 or 7, lines 227 to 229, 595, 596 for gamma-aminobutyric acid or indicated in Table IA or IB, columns 5 or 7 lines 230, 597 for shikimate or indicated in Table IA or IB, columns 5 or 7, line 598 for putrescine under relaxed hybridization conditions and which code on expression for peptides having the respective fine chemical, i.e. gamma-aminobutyric acid or shikimate or putrescine, resp., increasing-activity.

**[0140.0.0.17] to [0146.0.0.17]: see [0140.0.0.0] to [0146.0.0.0]**

**[0147.0.17.17]** Further, the nucleic acid molecule of the invention comprises a nucleic acid molecule, which is a complement of one of the nucleotide sequences of above mentioned nucleic acid molecules or a portion thereof. A nucleic acid molecule which is complementary to one of the nucleotide sequences indicated in Table IA or IB, columns 5 or 7, lines 227 to 229 or line 230 or lines 595 to 598 is one which is sufficiently complementary to one of said nucleotide sequences such that it can hybridise to one of said nucleotide sequences, thereby forming a stable duplex. Preferably, the hybridisation is performed under stringent hybrization conditions. However, a complement of one of the herein disclosed sequences is preferably a sequence complement thereto according to the base pairing of nucleic acid molecules well known to the skilled person. For example, the bases A and G undergo base pairing with the bases T and U or C, resp. and visa versa. Modifications of the bases can influence the base-pairing partner.

**[0148.0.17.17]** The nucleic acid molecule of the invention comprises a nucleotide sequence which is at least about 30%, 35%, 40% or 45%, preferably at least about 50%, 55%, 60% or 65%, more preferably at least about 70%, 80%, or 90%, and even more preferably at least about 95%, 97%, 98%, 99% or more homologous to a nucleotide sequence indicated in Table IA or IB, columns 5 or 7, lines 227 to 229 or line 230 or lines 595 to 598, or a portion thereof and preferably has above mentioned activity, in particular having a gamma-aminobutyric acid or shikimate or putrescine increasing activity after increasing the acitivity or an activity of a product of a gene encoding said sequences or their homologs.

**[0149.0.17.17]** The nucleic acid molecule of the invention comprises a nucleotide sequence which hybridizes, preferably hybridizes under stringent conditions as defined herein, to one of the nucleotide sequences indicated in Table IA or IB, columns 5 or 7, lines 227 to 229 or line 230 or lines 595 to 598, or a portion thereof and encodes a protein having above-mentioned activity, e.g. conferring a of gamma-aminobutyric acid or shikimate or putrescine increase, resp., and optionally, the activity of protein indicated in Table IIA or IIB, column 5, lines 227 to 229 or line 230 or lines 595 to 598.

**[00149.1.0.17]** Optionally, in one embodiment, the nucleotide sequence, which hybridises to one of the nucleotide sequences indicated in Table IA or IB, columns 5 or 7, lines 227 to 229 or line 230 or lines 595 to 598 has further one or more of the activities annotated or known for a protein as indicated in Table IIA or IIB, column 3, lines 227 to 229 or line 230 or lines 595 to 598.

**[0150.0.17.17]** Moreover, the nucleic acid molecule of the invention can comprise only a portion of the coding region of one of the sequences indicated in Table IA or IB, columns 5 or 7, lines 227 to 229 or line 230 or lines 595 to 598, for example a fragment which can be used as a probe or primer or a fragment encoding a biologically active portion of the polypeptide of the present invention or of a polypeptide used in the process of the present invention, i.e. having above-mentioned activity, e.g.conferring an increase of gamma-aminobutyric acid or shikimate or putrescine, resp., if its activity is increased. The nucleotide sequences determined from the cloning of the present protein-according-to-the-invention-encoding gene allows for the generation of probes and primers designed for use in identifying and/or cloning its homologues in other cell types and organisms. The probe/primer typically comprises substantially purified oligonucleotide. The oligonucleotide typically comprises a region of nucleotide sequence that hybridizes under stringent conditions to at least about 12, 15 preferably about 20 or 25, more preferably about 40, 50 or 75 consecutive nucleotides of a sense strand of one of the sequences set forth, e.g., as indicated in Table IA or IB, columns 5 or 7, lines 227 to 229 or line 230 or lines 595 to 598, an anti-sense sequence of one of the sequences, e.g., as indicated in Table IA or IB, columns 5 or 7, lines 227 to 229 or line 230 or lines 595 to 598, or naturally occurring mutants thereof. Primers based on a nucleotide of invention can be used in PCR reactions to clone homologues of the polypeptide of the invention or of the polypeptide used in the process of the invention, e.g. as the primers described in the examples of the present invention, e.g. as shown in the examples. A PCR with the primer pairs indicated in Table III, column 7, lines 227 to 229 or line 230 or lines 595 to 598 will result in a fragment of a polynucleotide sequence as indicated in Table IA or IB, columns 5 or 7, lines 227 to 229 or line 230 or lines 595 to 598 or its gene product.

**[0151.0.0.17]: see [0151.0.0.0]**

**[0152.0.17.17]** The nucleic acid molecule of the invention encodes a polypeptide or portion thereof which includes an amino acid sequence which is sufficiently homologous to an amino acid sequence as indicated in Table IIA or IIB, columns 5 or 7, lines 227 to 229 or line 230 or lines 595 to 598 such that the protein or portion thereof maintains the ability to participate in the respective fine chemical production, in particular a gamma-aminobutyric acid (lines 227 to 229, 595, 596) or shikimate (lines 230, 597) or putrescine (line 598) increasing activity as mentioned above or as described in the examples in plants or microorganisms is comprised.

**[0153.0.17.17]** As used herein, the language "sufficiently homologous" refers to proteins or portions thereof which have amino acid sequences which include a minimum number of identical or equivalent amino acid residues (e.g., an amino acid residue which has a similar side chain as an amino acid residue in one of the sequences of the polypeptide of the present invention) to an amino acid sequence as indicated in Table II A or IIB, columns 5 or 7, lines 227 to 229 or line 230 or lines 595 to 598 such that the protein or portion thereof is able to participate in the increase of the respective fine chemical production. In one embodiment, a protein or portion thereof as indicated in Table IIA or IIB, columns 5 or 7, lines 227 to 229 or line 230 or lines 595 to 598 has for example an activity of a polypeptide indicated in Table IIA or IIB, column 3, lines 227 to 229 or line 230 or lines 595 to 598.

**[0154.0.17.17]** In one embodiment, the nucleic acid molecule of the present invention comprises a nucleic acid that encodes a portion of the protein of the present invention. The protein is at least about 30%, 35%, 40%, 45% or 50%, preferably at least about 55%, 60%, 65% or 70%, and more preferably at least about 75%, 80%, 85%, 90%, 91 %, 92%, 93% or 94% and most preferably at least about 95%, 97%, 98%, 99% or more homologous to an entire amino acid sequence as indicated in Table IIA or IIB, columns 5 or 7, lines 227 to 229 or line 230 or lines 595 to 598 and has above-mentioned activity, e.g.conferring preferably the increase of the respective fine chemical.

**[0155.0.0.17] to [0156.0.0.17]: see [0155.0.0.0] to [0156.0.0.0]**

**[0157.0.17.17]** The invention further relates to nucleic acid molecules that differ from one of the nucleotide sequences as indicated in Table IA or IB, columns 5 or 7, lines 227 to 229 or line 230 or lines 595 to 598 (and portions thereof) due to degeneracy of the genetic code and thus encode a polypeptide of the present invention, in particular a polypeptide having above mentioned activity, e.g. conferring an increase in the respective fine chemical in a organism, e.g. as polypeptides comprising the sequence as indicated in Table IV, columns 5 or 7, lines 227 to 229 or line 230 or lines 595 to 598 or as polypeptides depicted in Table IIA or IIB, columns 5 or 7, lines 227 to 229 or line 230 or lines 595 to 598 or the functional homologues. Advantageously, the nucleic acid molecule of the invention comprises, or in an other embodiment has, a nucleotide sequence encoding a protein comprising, or in an other embodiment having, an amino acid sequence of a consensus sequences as indicated in Table IV, columns 5 or 7, lines 227 to 229 or line 230 or lines 595 to 598 or of the polypeptide as indicated in Table IIA or IIB, columns 5 or 7, lines 227 to 229 or line 230 or lines 595 to 598, resp., or the functional homologues. In a still further embodiment, the nucleic acid molecule of the invention encodes a full length protein which is substantially homologous to an amino acid sequence comprising a consensus sequence as indicated in Table IV, columns 5 or 7, lines 227 to 229 or line 230 or lines 595 to 598 or of a polypeptide as indicated in Table IIA or IIB, columns 5 or 7, lines 227 to 229 or line 230 or lines 595 to 598 or the functional homologues. However, in a preferred embodiment, the nucleic acid molecule of the present invention does not consist of a sequence as indicated in Table IA or IB, columns 5 or 7, lines 227 to 229 or line 230 or lines 595 to 598, resp.

**[0158.0.0.17] to [0160.0.0.17]: see [0158.0.0.0] to [0160.0.0.0]**

**[0161.0.17.17]** Accordingly, in another embodiment, a nucleic acid molecule of the invention is at least 15, 20, 25 or 30 nucleotides in length. Preferably, it hybridizes under stringent conditions to a nucleic acid molecule comprising a nucleotide sequence of the nucleic acid molecule of the present invention or used in the process of the present invention, e.g. comprising a sequence as indicated in Table IA or IB, columns 5 or 7, lines 227 to 229 or line 230 or lines 595 to 598. The nucleic acid molecule is preferably at least 20, 30, 50, 100, 250 or more nucleotides in length.

**[0162.0.0.17] see [0162.0.0.0]**

**[0163.0.17.17]** Preferably, a nucleic acid molecule of the invention that hybridizes under stringent conditions to a sequence as indicated in Table IA or IB, columns 5 or 7, lines 227 to 229 or line 230 or lines 595 to 598 corresponds to a naturally-occurring nucleic acid molecule of the invention. As used herein, a "naturally-occurring" nucleic acid molecule refers to an RNA or DNA molecule having a nucleotide sequence that occurs in nature (e.g., encodes a natural protein). Preferably, the nucleic acid molecule encodes a natural protein having above-mentioned activity, e.g. conferring the increase of the amount of the respective fine chemical in a organism or a part thereof, e.g. a tissue, a cell, or a compartment of a cell, after increasing the expression or activity thereof or the activity of a protein of the invention or used in the process of the invention.

**[0164.0.0.17] see [0164.0.0.0]**

**[0165.0.17.17]** For example, nucleotide substitutions leading to amino acid substitutions at "non-essential" amino acid residues can be made in a sequence of the nucleic acid molecule of the invention or used in the process of the invention, e.g. as indicated in Table IA or IB, columns 5 or 7, lines 227 to 229 or line 230 or lines 595 to 598, resp.

**[0166.0.0.17] to [0167.0.0.17]: see [0166.0.0.0] to [0167.0.0.0]**

**[0168.0.17.17]** Accordingly, the invention relates to nucleic acid molecules encoding a polypeptide having above-mentioned activity, e.g. conferring an increase in the respective fine chemical in an organisms or parts thereof that contain changes in amino acid residues that are not essential for said activity. Such polypeptides differ in amino acid sequence from a sequence contained in a sequence as indicated in Table IIA or IIB, columns 5 or 7, lines 227 to 229 or line 230 or lines 595 to 598, resp., yet retain said activity described herein. The nucleic acid molecule can comprise a nucleotide sequence encoding a polypeptide, wherein the polypeptide comprises an amino acid sequence at least about 50% identical to an amino acid sequence as indicated in Table IIA or IIB, columns 5 or 7, lines 227 to 229 or line 230 or lines 595 to 598, resp., and is capable of participation in the increase of production of the respective fine chemical after increasing its activity, e.g. its expression. Preferably, the protein encoded by the nucleic acid molecule is at least about 60% identical to a sequence as indicated in Table IIA or IIB, columns 5 or 7, lines 227 to 229 or line 230 or lines 595 to 598, resp., more preferably at least about 70% identical to one of the sequences as indicated in Table IIA or IIB, columns 5 or 7, lines 227 to 229 or line 230 or lines 595 to 598, resp., even more preferably at least about 80%, 90%, 95% homologous to a sequence as indicated in Table IIA or IIB, columns 5 or 7, lines 227 to 229 or line 230 or lines 595 to 598, resp., and most preferably at least about 96%, 97%, 98%, or 99% identical to the sequence as indicated in Table IIA or IIB, columns 5 or 7, lines 227 to 229 or line 230 or lines 595 to 598.

**[0169.0.0.17] to [0172.0.0.17]: see [0169.0.0.0] to [0172.0.0.0]**

**[0173.0.17.17]** For example a sequence, which has 80% homology with sequence SEQ ID NO: 15720 at the nucleic acid level is understood as meaning a sequence which, upon comparison with the sequence SEQ ID NO: 15720 by the above Gap program algorithm with the above parameter set, has a 80% homology.

**[0174.0.0.17]: see [0174.0.0.0]**

**[0175.0.17.17]** For example a sequence which has a 80% homology with sequence SEQ ID NO: 15721 at the protein level is understood as meaning a sequence which, upon comparison with the sequence SEQ ID NO: 15721 by the above program algorithm with the above parameter set, has a 80% homology.

**[0176.0.17.17]** Functional equivalents derived from one of the polypeptides as indicated in Table IIA or IIB, columns 5 or 7, lines 227 to 229 or line 230 or lines 595 to 598, resp., according to the invention by substitution, insertion or deletion have at least 30%, 35%, 40%, 45% or 50%, preferably at least 55%, 60%, 65% or 70% by preference at least 80%, especially preferably at least 85% or 90%, 91 %, 92%, 93% or 94%, very especially preferably at least 95%, 97%, 98% or 99% homology with one of the polypeptides as indicated in Table IIA or IIB, columns 5 or 7, lines 227 to 229 or line 230 or lines 595 to 598, resp., according to the invention and are distinguished by essentially the same properties as a polypeptide as indicated in Table IIA or IIB, columns 5 or 7, lines 227 to 229 or line 230 or lines 595 to 598, resp.

**[0177.0.17.17]** Functional equivalents derived from a nucleic acid sequence as indicated in Table IA or IB, columns 5 or 7, lines 227 to 229 or line 230 or lines 595 to 598, resp., according to the invention by substitution, insertion or deletion have at least 30%, 35%, 40%, 45% or 50%, preferably at least 55%, 60%, 65% or 70% by preference at least 80%, especially preferably at least 85% or 90%, 91%, 92%, 93% or 94%, very especially preferably at least 95%, 97%, 98% or 99% homology with one of the polypeptides as indicated in Table IIA or IIB, columns 5 or 7, lines 227 to 229 or line 230 or lines 595 to 598, resp., according to the invention and encode polypeptides having essentially the same properties as a polypeptide as indicated in Table IIA or IIB, columns 5 or 7, lines 227 to 229 or line 230 or lines 595 to 598, resp.

**[0178.0.0.17] see [0178.0.0.0]**

**[0179.0.17.17]** A nucleic acid molecule encoding a homologous to a protein sequence as indicated in Table IIA or IIB, columns 5 or 7, lines 227 to 229 or line 230 or lines 595 to 598, resp., can be created by introducing one or more nucleotide substitutions, additions or deletions into a nucleotide sequence of the nucleic acid molecule of the present invention, in particular as indicated in Table IA or IB, columns 5 or 7, lines 227 to 229 or line 230 or lines 595 to 598, resp., such that one or more amino acid substitutions, additions or deletions are introduced into the encoded protein. Mutations can be introduced into the encoding sequences as indicated in Table IA or IB, columns 5 or 7, lines 227 to 229 or line 230 or lines 595 to 598, resp., by standard techniques, such as site-directed mutagenesis and PCR-mediated mutagenesis.

**[0180.0.0.17] to [0183.0.0.17]: see [0180.0.0.0] to [0183.0.0.0]**

**[0184.0.17.17]** Homologues of the nucleic acid sequences used, with a sequence as indicated in Table IA or IB, columns 5 or 7, lines 227 to 229 or line 230 or lines 595 to 598, resp., or of the nucleic acid sequences derived from a sequences as indicated in Table IIA or IIB, columns 5 or 7, lines 227 to 229 or line 230 or lines 595 to 598, resp.,, comprise also allelic variants with at least approximately 30%, 35%, 40% or 45% homology, by preference at least approximately 50%, 60% or 70%, more preferably at least approximately 90%, 91%, 92%, 93%, 94% or 95% and even more preferably at least approximately 96%, 97%, 98%, 99% or more homology with one of the nucleotide sequences shown or the abovementioned derived nucleic acid sequences or their homologues, derivatives or analogues or parts of these. Allelic variants encompass in particular functional variants which can be obtained by deletion, insertion or substitution of nucleotides from the sequences shown, preferably from a sequence as indicated in Table IA or IB, columns 5 or 7, lines 227 to 229 or line 230 or lines 595 to 598, resp., or from the derived nucleic acid sequences, the intention being, however, that the enzyme activity or the biological activity of the resulting proteins synthesized is advantageously retained or increased.

**[0185.0.17.17]** In one embodiment of the present invention, the nucleic acid molecule of the invention or used in the process of the invention comprises one or more sequences as indicated in Table IA or IB, columns 5 or 7, lines 227 to 229 or line 230 or lines 595 to 598, resp. In one embodiment, it is preferred that the nucleic acid molecule comprises as little as possible other nucleotides not shown in any one of sequences as indicated in Table IA or IB, columns 5 or 7, lines 227 to 229 or line 230 or lines 595 to 598, resp. In one embodiment, the nucleic acid molecule comprises less than 500, 400, 300, 200, 100, 90, 80, 70, 60, 50 or 40 further nucleotides. In a further embodiment, the nucleic acid molecule comprises less than 30, 20 or 10 further nucleotides. In one embodiment, a nucleic acid molecule used in the process of the invention is identical to a sequence as indicated in Table IA or IB, columns 5 or 7, lines 227 to 229 or line 230 or lines 595 to 598, resp.

**[0186.0.17.17]** Also preferred is that one or more nucleic acid molecule(s) used in the process of the invention encodes a polypeptide comprising a sequence as indicated in Table IIA or IIB, columns 5 or 7, lines 227 to 229 or line 230 or lines 595 to 598, resp. In one embodiment, the nucleic acid molecule encodes less than 150, 130, 100, 80, 60, 50, 40 or 30 further amino acids. In a further embodiment, the encoded polypeptide comprises less than 20, 15, 10, 9, 8, 7, 6 or 5 further amino acids. In one embodiment, the encoded polypeptide used in the process of the invention is identical to the sequences as indicated in Table IIA or IIB, columns 5 or 7, lines 227 to 229 or line 230 or lines 595 to 598, resp.

**[0187.0.17.17]** In one embodiment, a nucleic acid molecule of the invention or used in the process encodes a polypeptide comprising the sequence as indicated in Table II A or IIB, columns 5 or 7, lines 227 to 229 or line 230 or lines 595 to 598, resp., comprises less than 100 further nucleotides. In a further embodiment, said nucleic acid molecule comprises less than 30 further nucleotides. In one embodiment, the nucleic acid molecule used in the process is identical to a coding sequence encoding a sequences as indicated in Table IIA or IIB, columns 5 or 7, lines 227 to 229 or line 230 or lines 595 to 598, resp.

**[0188.0.17.17]** Polypeptides (= proteins), which still have the essential enzymatic activity of the polypeptide of the present invention conferring an increase of the respective fine chemical i.e. whose activity is essentially not reduced, are polypeptides with at least 10% or 20%, by preference 30% or 40%, especially preferably 50% or 60%, very especially preferably 80% or 90 or more of the wild type biological activity or enzyme activity, advantageously, the activity is essentially not reduced in comparison with the activity of a polypeptide as indicated in Table IIA or IIB, columns 5 or 7, lines 227 to 229 or line 230 or lines 595 to 598, resp., and is expressed under identical conditions.

**[0189.0.17.17]** Homologues of a sequences as indicated in Table IA or IB, columns 5 or 7, lines 227 to 229 or line 230 or lines 595 to 598, resp., or of a derived sequences as indicated in Table IIA or IIB, columns 5 or 7, lines 227 to 229 or line 230 or lines 595 to 598, resp., also mean truncated sequences, cDNA, single-stranded DNA or RNA of the coding and noncoding DNA sequence. Homologues of said sequences are also understood as meaning derivatives, which comprise noncoding regions such as, for example, UTRs, terminators, enhancers or promoter variants. The promoters upstream of the nucleotide sequences stated can be modified by one or more nucleotide substitution(s), insertion(s) and/or deletion(s) without, however, interfering with the functionality or activity either of the promoters, the open reading frame (=ORF) or with the 3'-regulatory region such as terminators or other 3'regulatory regions, which are far away from the ORF. It is furthermore possible that the activity of the promoters is increased by modification of their sequence, or that they are replaced completely by more active promoters, even promoters from heterologous organisms. Appropriate promoters are known to the person skilled in the art and are mentioned herein below.

**[0190.0.0.17]: see [0190.0.0.0]**

**[0191.0.17.17]** The organisms or part thereof produce according to the herein mentioned process of the invention an increased level of free and/or bound the respective fine chemical compared to said control or selected organisms or parts thereof.

**[0192.0.0.17] to [0203.0.0.17]: see [0192.0.0.0] to [0203.0.0.0]**

**[0204.0.17.17]** Accordingly, in one embodiment, the invention relates to a nucleic acid molecule, which comprises a nucleic acid molecule selected from the group consisting of:
a) nucleic acid molecule encoding, preferably at least the mature form, of a polypeptide as indicated in Table IIA or IIB, columns 5 or 7, lines 227 to 229 or line 230 or lines 595 to 598, resp.; or a fragment thereof conferring an increase in the amount of the respective fine chemical, i.e. gamma-aminobutyric acid (lines 227 to 229, 595, 596) or shikimate (lines 230, 597) or putrescine (line 598), resp., in an organism or a part thereof
b) nucleic acid molecule comprising, preferably at least the mature form, of a nucleic acid molecule as indicated in Table IA or IB, columns 5 or 7, lines 227 to 229 or line 230 or lines 595, 596 or line 597 or line 598, resp., or a fragment thereof conferring an increase in the amount of the respective fine chemical in an organism or a part thereof;
c) nucleic acid molecule whose sequence can be deduced from a polypeptide sequence encoded by a nucleic acid molecule of (a) or (b) as result of the degeneracy of the genetic code and conferring an increase in the amount of the respective fine chemical in an organism or a part thereof;
d) nucleic acid molecule encoding a polypeptide whose sequence has at least 50% identity with the amino acid sequence of the polypeptide encoded by the nucleic acid molecule of (a) to (c) and conferring an increase in the amount of the respective fine chemical in an organism or a part thereof;
e) nucleic acid molecule which hybridizes with a nucleic acid molecule of (a) to (c) under under stringent hybridisation conditions and conferring an increase in the amount of the respective fine chemical in an organism or a part thereof;
f) nucleic acid molecule encoding a polypeptide, the polypeptide being derived by substituting, deleting and/or adding one or more amino acids of the amino acid sequence of the polypeptide encoded by the nucleic acid molecules (a) to (d), preferably to (a) to (c), and conferring an increase in the amount of the respective fine chemical in an organism or a part thereof;
g) nucleic acid molecule encoding a fragment or an epitope of a polypeptide which is encoded by one of the nucleic acid molecules of (a) to (e), preferably to (a) to (c) and conferring an increase in the amount of the respective fine chemical in an organism or a part thereof;
h) nucleic acid molecule comprising a nucleic acid molecule which is obtained by amplifying a cDNA library or a genomic library using primers or primer pairs as indicated in Table III, columns 5 or 7, lines 227 to 229 or line 230 or line 595, 596 or line 597 or line 598 and conferring an increase in the amount of the respective fine chemical, i.e. gamma-aminobutyric acid (lines 227 to 229, 595, 596) or shikimate (line 230, 597) or putrescine ( line 598), resp., in an organism or a part thereof;
i) nucleic acid molecule encoding a polypeptide which is isolated, e.g. from a expression library, with the aid of monoclonal antibodies against a polypeptide encoded by one of the nucleic acid molecules of (a) to (g), preferably to (a) to (c) and conferring an increase in the amount of the respective fine chemical in an organism or a part thereof;
j) nucleic acid molecule which encodes a polypeptide comprising a consensus sequence as indicated in Table IV, columns 5 or 7, lines 227 to 229 or line 230 and conferring an increase in the amount of the respective fine chemical, i.e. gamma-aminobutyric acid (lines 227 to 229, 595, 596) or shikimate(line 230, 597) or putrescine (line 598), resp., in an organism or a part thereof;
k) nucleic acid molecule encoding the amino acid sequence of a polypeptide encoding a domaine of a polypeptide as indicated in Table IIA or IIB, columns 5 or 7, lines 227 to 229 or line 230, resp., and conferring an increase in the amount of the respective fine chemical, i.e. gamma-aminobutyric acid (lines 227 to 229, 595, 596) or shikimate (line 230, 597) or putrescine (line 598), resp., in an organism or a part thereof; and
l) nucleic acid molecule which is obtainable by screening a suitable nucleic acid library under stringent hybridization conditions with a probe comprising one of the sequences of the nucleic acid molecule of (a) to (k) or with a fragment of at least 15 nt, preferably 20 nt, 30 nt, 50 nt, 100 nt, 200 nt or 500 nt of the nucleic acid molecule characterized in (a) to (h) or of a nucleic acid molecule as indicated in Table IA or IB, columns 5 or 7, lines 227 to 229 or line 230 or lines 595 to 598, resp., or a nucleic acid molecule encoding, preferably at least the mature form of, a polypeptide as indicated in Table IIA or IIB, columns 5 or 7, lines 227 to 229 or line 230 or lines 595 to 598, resp., and conferring an increase in the amount of the respective fine chemical in an organism or a part thereof; or which encompasses a sequence which is complementary thereto;
whereby, preferably, the nucleic acid molecule according to (a) to (I) distinguishes over a sequence as indicated in Table IA or IB, columns 5 or 7, lines 227 to 229 or line 230 or lines 595 to 598, resp., by one or more nucleotides. In one embodiment, the nucleic acid molecule of the invention does not consist of the sequence as indicated in Table I A or IB, columns 5 or 7, lines 227 to 229 or line 230 or lines 595 to 598, resp. In an other embodiment, the nucleic acid molecule of the present invention is at least 30 % identical and less than 100%, 99,999%, 99,99%, 99,9% or 99% identical to a sequence as indicated in Table IA or IB, columns 5 or 7, lines 227 to 229 or line 230 or lines 595 to 598, resp. In a further embodiment the nucleic acid molecule does not encode a polypeptide sequence as indicated in Table IIA or IIB, columns 5 or 7, lines 227 to 229 or line 230 or lines 595 to 598, resp. Accordingly, in one embodiment, the nucleic acid molecule of the present invention encodes in one embodiment a polypeptide which differs at least in one or more amino acids from a polypeptide indicated in Table IIA or IIB, columns 5 or 7, lines 227 to 229 or line 230 or lines 595 to 598 does not encode a protein of a sequence as indicated in Table IIA or IIB, columns 5 or 7, lines 227 to 229 or line 230 or lines 595 to 598. Accordingly, in one embodiment, the protein encoded by a sequences of a nucleic acid accoriding to (a) to (I) does not consist of a sequence as indicated in Table IIA or IIB, columns 5 or 7, lines 227 to 229 or line 230 or lines 595 to 598. In a further embodiment, the protein of the present invention is at least 30 % identical to a protein sequence indicated in Table IIA or IIB, columns 5 or 7, lines 227 to 229 or line 230 or lines 595 to 598 and less than 100%, preferably less than 99,999%, 99,99% or 99,9%, more preferably less than 99%, 98%, 97%, 96% or 95% identical to a sequence as indicated in Table IIA or IIB, columns 5 or 7, lines 227 to 229 or line 230 or lines 595 to 598.

**[0205.0.0.17] to [0206.0.0.17]: see [0205.0.0.0] to [0206.0.0.0]**

**[0207.0.17.17]** As described herein, the nucleic acid construct can also comprise further genes, which are to be introduced into the organisms or cells. It is possible and advantageous to introduce into, and express in, the host organisms regulatory genes such as genes for inductors, repressors or enzymes, which, owing to their enzymatic activity, engage in the regulation of one or more genes of a biosynthetic pathway. These genes can be of heterologous or homologous origin. Moreover, further biosynthesis genes may advantageously be present, or else these genes may be located on one or more further nucleic acid constructs. Genes, which are advantageously employed as biosynthesis genes, are genes of the glutamic acid metabolism, the phosphoenolpyruvate metabolism, the amino acid metabolism, of glycolysis, of the tricarboxylic acid metabolism or their combinations. As described herein, regulator sequences or factors can have a positive effect on preferably the gene expression of the genes introduced, thus increasing it. Thus, an enhancement of the regulator elements may advantageously take place at the transcriptional level by using strong transcription signals such as promoters and/or enhancers. In addition, however, an enhancement of translation is also possible, for example by increasing mRNA stability or by inserting a translation enhancer sequence.

**[0208.0.0.17] to [0226.0.0.17]: see [0208.0.0.0] to [0226.0.0.0]**

**[0227.0.17.17]** The abovementioned nucleic acid molecules can be cloned into the nucleic acid constructs or vectors according to the invention in combination together with further genes, or else different genes are introduced by transforming several nucleic acid constructs or vectors (including plasmids) into a host cell, advantageously into a plant cell or a microorgansims.
In addition to a sequence indicated in Table IA or IB, columns 5 or 7, lines 227 to 229 or line 230 or lines 595 to 598 or its derivatives, it is advantageous to express and/or mutate further genes in the organisms. Especially advantageously, additionally at least one further gene of the glutamic acid or phosphoenolpyruvate metabolic pathway, is expressed in the organisms such as plants or microorganisms. It is also possible that the regulation of the natural genes has been modified advantageously so that the gene and/or its gene product is no longer subject to the regulatory mechanisms which exist in the organisms. This leads to an increased synthesis of the fine chemicals desired since, for example, feedback regulations no longer exist to the same extent or not at all. In addition it might be advantageously to combine one or more of the sequences indicated in Table IA or IB, columns 5 or 7, lines 227 to 229 or line 230 or lines 595 to 598, resp., with genes which generally support or enhances to growth or yield of the target organismen, for example genes which lead to faster growth rate of microorganisms or genes which produces stress-, pathogen, or herbicide resistant plants.

**[0228.0.17.17]** In a further embodiment of the process of the invention, therefore, organisms are grown, in which there is simultaneous overexpression of at least one nucleic acid or one of the genes which code for proteins directly or indirectly involved in the glutamic acid or phosphoenolpyruvate metabolism.

**[0229.0.17.17]** Further advantageous nucleic acid sequences which can be expressed in combination with the sequences indicated in Table IA or IB, columns 5 or 7, lines 227 to 229 or line 230 or lines 595 to 598 used in the process and/or the abovementioned biosynthesis genes are the sequences encoding further genes of the aromatic amino acid pathway, such as tryptophan, phenylalanine or tyrosine.These genes can lead to an increased synthesis of the essential amino acids tryptophan, phenylalanine or tyrosine.

**[0230.0.0.17] see [230.0.0.0]**

**[0231.0.17.17]** In a further advantageous embodiment of the process of the invention, the organisms used in the process are those in which simultaneously a gamma-aminobutyric acid or shikimate degrading protein is attenuated, in particular by reducing the rate of expression of the corresponding gene. A person skilled in the art knows for example, that the inhibition of an enzyme or a gene of the biosynthesis of the essential aromatic amino acids tryptophan, tyrosine or phenylalanine may increase the amount of shikimate accumulating in an organism, in particular in plants. Furthermore the inhibition of tryptophan, phenylalanine or tyrosine degrading enzymes also may result in an increased shikimate accumulation in the organism.

**[0232.0.0.17] to [0276.0.0.17]: see [0232.0.0.0] to [0276.0.0.0]**

**[0277.0.17.17]** The respective fine chemical produced can be isolated from the organism by methods with which the skilled worker are familiar, for example via extraction, salt precipitation, and/or different chromatography methods. The process according to the invention can be conducted batchwise, semibatchwise or continuously.

**[0278.0.0.17] to [0282.0.0.17]: see [0278.0.0.0] to [0282.0.0.0]**

**[0283.0.17.17]** Moreover, native polypeptide conferring the increase of the respective fine chemical in an organism or part thereof can be isolated from cells, for example using the antibody of the present invention as described below, e.g. an antibody against a protein as indicated in Table IIA or IIB, column 3, lines 227 to 229 or line 230 or lines 595 to 598, resp., or an antibody against a polypeptide as indicated in Table II A or IIB, columns 5 or 7, lines 227 to 229 or line 230 or lines 595 to 598, resp., which can be produced by standard techniques utilizing the polypeptid of the present invention or fragment thereof. Preferred are monoclonal antibodies.

**[0284.0.0.17] see [0284.0.0.0]**

**[0285.0.17.17]** In one embodiment, the present invention relates to a polypeptide having a sequence as indicated in Table IIA or IIB, columns 5 or 7, lines 227 to 229 or line 230 or lines 595 to 598, resp., or as coded by a nucleic acid molecule as indicated in Table IA or IB, columns 5 or 7, lines 227 to 229 or line 230 or lines 595 to 598, resp., or functional homologues thereof.

**[0286.0.17.17]** In one advantageous embodiment, in the method of the present invention the activity of a polypeptide is increased which comprises or consists of a consensus sequence as indicated in Table IV, column 7, lines 227 to 229 or line 230 or lines 595 to 598 and in one another embodiment, the present invention relates to a polypeptide comprising or consisting of a consensus sequence as indicated in Table IV, column 7, lines 227 to 229 or line 230 or lines 595 to 598 whereby 20 or less, preferably 15 or 10, preferably 9, 8, 7, or 6, more preferred 5 or 4, even more preferred 3, even more preferred 2, even more preferred 1, most preferred 0 of the amino acids positions indicated can be replaced by any amino acid or, in an further embodiment, can be replaced and/or absent. In one embodiment, the present invention relates to the method of the present invention comprising a polypeptide or to a polypeptide comprising more than one consensus sequences (of an individual line) as indicated in Table IV, column 7, lines 227 to 230 and/or 595 to 598, resp..

**[0287.0.0.17] to [0289.0.0.17]: see [0287.0.0.0] to [0289.0.0.0]**

**[00290.0.17.17]** The alignment was performed with the Software AlignX (sept 25, 2002) a component of Vector NTI Suite 8.0, InforMax™,Invitrogen™ life science software, U.S. Main Office, 7305 Executive Way,Frederick, MD 21704,USA with the following settings: For pairwise alignments: gap opening penality: 10,0; gap extension penality 0,1. For multiple alignments: Gap opening penalty: 10,0; Gap extension penalty: 0,1; Gap separation penalty range: 8; Residue substitution matrix: blosum62; Hydrophilic residues: G P S N D Q E K R; Transition weighting: 0,5; Consensus calculation options: Residue fraction for consensus: 0,9. Presettings were selected to allow also for the alignment of conserved amino acids

**[0291.0.17.17]** In one advantageous embodiment, the method of the present invention comprises the increasing of a polypeptide comprising or consisting of plant or microorganism specific consensus sequences.
In one embodiment, said polypeptide of the invention distinguishes over a sequence as indicated in Table IIA or IIB, columns 5 or 7, lines 227 to 229 or line 230 or lines 595 to 598, resp., by one or more amino acids. In one embodiment, polypeptide distinguishes from a sequence as indicated in Table IIA or IIB, columns 5 or 7, lines 227 to 229 or line 230 or lines 595 to 598, resp., by more than 5, 6, 7, 8 or 9 amino acids, preferably by more than 10, 15, 20, 25 or 30 amino acids, even more preferred are more than 40, 50, or 60 amino acids and, preferably, the sequence of the polypeptide of the invention distinguishes from a sequence as indicated in Table IIA or IIB, columns 5 or 7, lines 227 to 229 or line 230 or lines 595 to 598, resp., by not more than 80% or 70% of the amino acids, preferably not more than 60% or 50%, more preferred not more than 40% or 30%, even more preferred not more than 20% or 10%. In an other embodiment, said polypeptide of the invention does not consist of a sequence as indicated in Table IIA or IIB, columns 5 or 7, lines 227 to 229 or line 230 or lines 595 to 598.

**[0292.0.0.17] see [0292.0.0.0]**

**[0293.0.17.17]** In one embodiment, the invention relates to a polypeptide conferring an increase in the fine chemical in an organism or part being encoded by the nucleic acid molecule of the invention or by a nucleic acid molecule used in the process of the invention. In one embodiment, the polypeptide of the invention has a sequence which distinguishes from a sequence as indicated in Table IIA or IIB, columns 5 or 7, lines 227 to 229 or line 230 or lines 595 to 598, resp., by one or more amino acids. In an other embodiment, said polypeptide of the invention does not consist of the sequence as indicated in Table IIA or IIB, columns 5 or 7, lines 227 to 229 or line 230 or lines 595 to 598, resp. In a further embodiment, said polypeptide of the present invention is less than 100%, 99,999%, 99,99%, 99,9% or 99% identical. In one embodiment, said polypeptide does not consist of the sequence encoded by a nucleic acid molecules as indicated in Table IA or IB, columns 5 or 7, lines 227 to 229 or line 230 or lines 595 to 598, resp.

**[0294.0.17.17]** In one embodiment, the present invention relates to a polypeptide having an activity of a protein as indicated in Table IIA or IIB, column 3, lines 227 to 229 or line 230 or lines 595 to 598, resp., which distinguishes over a sequence as indicated in Table IIA or IIB, columns 5 or 7, lines 227 to 229 or line 230 or lines 595 to 598, resp., by one or more amino acids, preferably by more than 5, 6, 7, 8 or 9 amino acids, preferably by more than 10, 15, 20, 25 or 30 amino acids, even more preferred are more than 40, 50, or 60 amino acids but even more preferred by less than 70% of the amino acids, more preferred by less than 50%, even more preferred my less than 30% or 25%, more preferred are 20% or 15%, even more preferred are less than 10%.

**[0295.0.0.17] to [0296.0.0.17]: see [0295.0.0.0] to [0296.0.0.0]**

**[0297.0.0.17] see [0297.0.0.0]**

**[00297.1.0.17] %**

**[0298.0.17.17]** A polypeptide of the invention can participate in the process of the present invention. The polypeptide or a portion thereof comprises preferably an amino acid sequence which is sufficiently homologous to an amino acid sequence as indicated in Table IIA or IIB, columns 5 or 7, lines 227 to 229 or line 230 or lines 595 to 598, resp. The portion of the protein is preferably a biologically active portion as described herein. Preferably, the polypeptide used in the process of the invention has an amino acid sequence identical to a sequence as indicated in Table IIA or IIB, columns 5 or 7, lines 227 to 229 or line 230 or lines 595 to 598, resp.

**[0299.0.17.17]** Further, the polypeptide can have an amino acid sequence which is encoded by a nucleotide sequence which hybridizes, preferably hybridizes under stringent conditions as described above, to a nucleotide sequence of the nucleic acid molecule of the present invention. Accordingly, the polypeptide has an amino acid sequence which is encoded by a nucleotide sequence that is at least about 35%, 40%, 45%, 50%, 55%, 60%, 65% or 70%, preferably at least about 75%, 80%, 85% or 90, and more preferably at least about 91%, 92%, 93%, 94% or 95%, and even more preferably at least about 96%, 97%, 98%, 99% or more homologous to one of the nucleotide sequence as indicated in Table IA or IB, columns 5 or 7, lines 227 to 229 or line 230 or lines 595 to 598, resp. The preferred polypeptide of the present invention preferably possesses at least one of the activities according to the invention and described herein. A preferred polypeptide of the present invention includes an amino acid sequence encoded by a nucleotide sequence which hybridizes, preferably hybridizes under stringent conditions, to a nucleotide sequence as indicated in Table IA or IB, columns 5 or 7, lines 227 to 229 or line 230 or lines 595 to 598, resp., or which is homologous thereto, as defined above.

**[0300.0.17.17]** Accordingly the polypeptide of the present invention can vary from a sequence as indicated in Table IIA or IIB, columns 5 or 7, lines 227 to 229 or line 230 or lines 595 to 598, resp., in amino acid sequence due to natural variation or mutagenesis, as described in detail herein. Accordingly, the polypeptide comprise an amino acid sequence which is at least about 35%, 40%, 45%, 50%, 55%, 60%, 65% or 70%, preferably at least about 75%, 80%, 85% or 90, and more preferably at least about 91%, 92%, 93%, 94% or 95%, and most preferably at least about 96%, 97%, 98%, 99% or more homologous to an entire amino acid sequence of as indicated in Table IIA or IIB, columns 5 or 7, lines 227 to 229 or line 230 or lines 595 to 598, resp.

**[0301.0.0.17] see [0301.0.0.0]**

**[0302.0.17.17]** Biologically active portions of an polypeptide of the present invention include peptides comprising amino acid sequences derived from the amino acid sequence of the polypeptide of the present invention or used in the process of the present invention, e.g., an amino acid sequence as indicated in Table IIA or IIB, columns 5 or 7, lines 227 to 229 or line 230 or lines 595 to 598, resp., or the amino acid sequence of a protein homologous thereto, which include fewer amino acids than a full length polypeptide of the present invention or used in the process of the present invention or the full length protein which is homologous to an polypeptide of the present invention or used in the process of the present invention depicted herein, and exhibit at least one activity of polypeptide of the present invention or used in the process of the present invention.

**[0303.0.0.17] see [0303.0.0.0]**

**[0304.0.17.17]** Manipulation of the nucleic acid molecule of the invention may result in the production of a protein having essentially the activity of the polypeptides as indicated in Table IIA or IIB, column 3, lines 227 to 229 or line 230 or lines 595 to 598 but having differences in the sequence from said wild-type protein. These proteins may be improved in efficiency or activity, may be present in greater numbers in the cell than is usual, or may be decreased in efficiency or activity in relation to the wild type protein.

**[0305.0.0.17] to [0308.0.0.17]: see [0305.0.0.0] to [0308.0.0.0]**

**[0309.0.17.17]** In one embodiment, a reference to a protein (= polypeptide) of the invention or as indicated in Table IIA or IIB, columns 5 or 7, lines 227 to 229 or line 230 or lines 595 to 598, resp., refers to a polypeptide having an amino acid sequence corresponding to the polypeptide of the invention or used in the process of the invention, whereas an "other polypeptide" not being indicated in Table IIA or IIB, columns 5 or 7, lines 227 to 229 or line 230 or lines 595 to 598, resp., refers to a polypeptide having an amino acid sequence corresponding to a protein which is not substantially homologous to a polypeptide of the invention, preferably which is not substantially homologous to a polypeptide as indicated in Table IIA or IIB, columns 5 or 7, lines 227 to 229 or line 230 or lines 595 to 598, resp.,, e.g., a protein which does not confer the activity described herein or annotated or known for as indicated in Table IIA or IIB, column 3, lines 227 to 229 or line 230 or lines 595 to 598, resp., and which is derived from the same or a different organism. In one embodiment, an "other polypeptide" not being indicated in Table IIA or IIB, columns 5 or 7, lines 227 to 229 or line 230 or lines 595 to 598, resp., does not confer an increase of the respective fine chemical in an organism or part thereof.

**[0310.0.0.17] to [0334.0.0.17]: see [0310.0.0.0] to [0334.0.0.0] [0335.0.17.17]** The dsRNAi method has proved to be particularly effective and advantageous for reducing the expression of a nucleic acid sequences as indicated in Table IA or IB, columns 5 or 7, lines 227 to 229 or line 230 or lines 595 to 598, resp., and/or homologs thereof. As described inter alia in WO 99/32619, dsRNAi approaches are clearly superior to traditional antisense approaches. The invention therefore furthermore relates to double-stranded RNA molecules (dsRNA molecules) which, when introduced into an organism, advantageously into a plant (or a cell, tissue, organ or seed derived there from), bring about altered metabolic activity by the reduction in the expression of a nucleic acid sequences as indicated in Table IA or IB, columns 5 or 7, lines 227 to 229 or line 230 or lines 595 to 598, resp., and/or homologs thereof. In a double-stranded RNA molecule for reducing the expression of aprotein encoded by a nucleic acid sequence as indicated in Table IA or IB, columns 5 or 7, lines 227 to 229 or line 230 or lines 595 to 598, resp., and/or homologs thereof, one of the two RNA strands is essentially identical to at least part of a nucleic acid sequence, and the respective other RNA strand is essentially identical to at least part of the complementary strand of a nucleic acid sequence.

**[0336.0.0.17] to [0342.0.0.17]: see [0336.0.0.0] to [0342.0.0.0]**

**[0343.0.17.17]** As has already been described, 100 % sequence identity between the dsRNA and a gene transcript of a nucleic acid sequence as indicated in Table IA or IB, columns 5 or 7, lines 227 to 229 or line 230 or lines 595 to 598, resp., or it's homolog is not necessarily required in order to bring about effective reduction in the expression. The advantage is, accordingly, that the method is tolerant with regard to sequence deviations as may be present as a consequence of genetic mutations, polymorphisms or evolutionary divergences. Thus, for example, using the dsRNA, which has been generated starting from a sequence as indicated in Table IA or IB, columns 5 or 7, lines 227 to 229 or line 230 or lines 595 to 598, resp., or homologs thereof of the one organism, may be used to suppress the corresponding expression in another organism.

**[0344.0.0.17] to [0350.0.0.17]: see [0344.0.0.0] to [0350.0.0.0]**

**[0351.0.0.17] to [0361.0.0.17]: see [0351.0.0.0] to [0361.0.0.0]**

**[0362.0.17.17]** Accordingly the present invention relates to any cell transgenic for any nucleic acid characterized as part of the invention, e.g. conferring the increase of the respective fine chemical in a cell or an organism or a part thereof, e.g. the nucleic acid molecule of the invention, the nucleic acid construct of the invention, the antisense molecule of the invention, the vector of the invention or a nucleic acid molecule encoding the polypeptide of the invention, e.g. the polypeptide as indicated in Table IIA or IIB, columns 5 or 7, lines 227 to 229 or line 230 or lines 595 to 598, resp., e.g. encoding a polypeptide having protein activity, as indicated in Table IIA or IIB, columns 3, lines 227 to 229 or line 230 or lines 595 to 598, resp.. Due to the abovementioned activity the respective fine chemical content in a cell or an organism is increased. For example, due to modulation or manipulation, the cellular activity of the polypeptide of the invention or nucleic acid molecule of the invention is increased, e.g. due to an increased expression or specific activity of the subject matters of the invention in a cell or an organism or a part thereof. Transgenic for a polypeptide having an activity of a polypeptide as indicated in Table IIA or IIB, columns 5 or 7, lines 227 to 229 or line 230 or lines 595 to 598, resp., means herein that due to modulation or manipulation of the genome, an activity as annotated for a polypeptide as indicated in Table IIA or IIB, column 3, lines 227 to 229 or line 230 or lines 595 to 598, e.g. having a sequence as indicated in Table IIA or IIB, columns 5 or 7, lines 227 to 229 or line 230 or lines 595 to 598, resp., is increased in a cell or an organism or a part thereof. Examples are described above in context with the process of the invention.

**[0363.0.0.17] see [0363.0.0.0]**

**[0364.0.17.17]** A naturally occurring expression cassette - for example the naturally occurring combination of a promoter of a gene encoding a polypeptide of the invention as indicated in Table IIA or IIB, column 3, lines 227 to 229 or line 230 or lines 595 to 598, resp. with the corresponding protein-encoding sequence as indicated in Table IA or IB, column 5, lines 227 to 229 or line 230 or lines 595 to 598, resp., becomes a transgenic expression cassette when it is modified by non-natural, synthetic "artificial" methods such as, for example, mutagenization. Such methods have been described (US 5,565,350; WO 00/15815; also see above).

**[0365.0.0.17] to [0373.0.0.17]: see [0365.0.0.0] to [0373.0.0.0]**

**[0374.0.17.17]** Transgenic plants comprising the respective fine chemical synthesized in the process according to the invention can be marketed directly without isolation of the compounds synthesized. In the process according to the invention, plants are understood as meaning all plant parts, plant organs such as leaf, stalk, root, tubers or seeds or propagation material or harvested material or the intact plant. In this context, the seed encompasses all parts of the seed such as the seed coats, epidermal cells, seed cells, endosperm or embryonic tissue, gamma-aminobutyric acid or shikimate, in particular the respective fine chemical, produced in the process according to the invention may, however, also be isolated from the plant in the form of their free gamma-aminobutyric acid or shikimate, in particular the free respective fine chemical, or bound in or to compounds or moieties, like glucosides, e.g. diglucosides. The respective fine chemical produced by this process can be harvested by harvesting the organisms either from the culture in which they grow or from the field. This can be done via expressing, grinding and/or extraction, salt precipitation and/or ion-exchange chromatography or other chromatographic methods of the plant parts, preferably the plant seeds, plant fruits, plant tubers and the like.

**[0375.0.0.17] to [0376.0.0.17]: see [0375.0.0.0] to [0376.0.0.0]**

**[0377.0.17.17]** Accordingly, the present invention relates also to a process whereby the produced gamma-aminobutyric acid or shikimate is isolated.

**[0378.0.17.17]** In this manner, more than 50% by weight, advantageously more than 60% by weight, preferably more than 70% by weight, especially preferably more than 80% by weight, very especially preferably more than 90% by weight, of the gamma-aminobutyric acid or shikimate produced in the process can be isolated. The resulting gamma-aminobutyric acid or shikimate can, if appropriate, subsequently be further purified, if desired mixed with other active ingredients such as vitamins, amino acids, carbohydrates, antibiotics and the like, and, if appropriate, formulated.

**[0379.0.17.17]** In one embodiment, gamma-aminobutyric acid and shikimate are a mixture of the respective fine chemicals.

**[0380.0.17.17]** The gamma-aminobutyric acid or shikimate obtained in the process are suitable as starting material for the synthesis of further products of value. For example, they can be used in combination with each other or alone for the production of pharmaceuticals, foodstuffs, animal feeds or cosmetics. Accordingly, the present invention relates a method for the production of pharmaceuticals, food stuff, animal feeds, nutrients or cosmetics comprising the steps of the process according to the invention, including the isolation of the gamma-aminobutyric acid or shikimate composition produced or the respective fine chemical produced if desired and formulating the product with a pharmaceutical acceptable carrier or formulating the product in a form acceptable for an application in agriculture. A further embodiment according to the invention is the use of the gamma-aminobutyric acid or shikimate produced in the process or of the transgenic organisms in animal feeds, foodstuffs, medicines, food supplements, cosmetics or pharmaceuticals or for the production of gamma-aminobutyric acid or shikimate e.g. after isolation of the respective fine chemical or without, e.g. in situ, e.g in the organism used for the process for the production of the respective fine chemical.

**[0381.0.0.17] to [0382.0.0.17]: see [0381.0.0.0] to [0382.0.0.0]**

**[0383.0.17.17]**

**[0384.0.0.17] see [0384.0.0.0]**

**[0385.0.17.17]** The fermentation broths obtained in this way, containing in particular gamma-aminobutyric acid or shikimate in mixtures with other organic acids, aminoacids, polypeptides or polysaccarides, normally have a dry matter content of from 1 to 70% by weight, preferably 7.5 to 25% by weight. Sugar-limited fermentation is additionally advantageous, e.g. at the end, for example over at least 30% of the fermentation time. This means that the concentration of utilizable sugar in the fermentation medium is kept at, or reduced to, 0 to 10 g/l, preferably to 0 to 3g/l during this time. The fermentation broth is then processed further. Depending on requirements, the biomass can be removed or isolated entirely or partly by separation methods, such as, for example, centrifugation, filtration, decantation, coagulation/flocculation or a combination of these methods, from the fermentation broth or left completely in it.
The fermentation broth can then be thickened or concentrated by known methods, such as, for example, with the aid of a rotary evaporator, thin-film evaporator, falling film evaporator, by reverse osmosis or by nanofiltration. This concentrated fermentation broth can then be worked up by freeze-drying, spray drying, spray granulation or by other processes.

**[0386.0.17.17]** Accordingly, it is possible to purify the gamma-aminobutyric acid or shikimate produced according to the invention further. For this purpose, the product-containing compositions subjected for example to separation via e.g. an open column chromatography or HPLC in which case the desired product or the impurities are retained wholly or partly on the chromatography resin. These chromatography steps can be repeated if necessary, using the same or different chromatography resins. The skilled worker is familiar with the choice of suitable chromatography resins and their most effective use.

**[0387.0.0.17] to [0392.0.0.17]: see [0387.0.0.0] to [0392.0.0.0]**

**[0393.0.17.17]** In one embodiment, the present invention relates to a method for the identification of a gene product conferring an increase in the respective fine chemical production in a cell, comprising the following steps:
a. contacting, e.g. hybridising, the nucleic acid molecules of a sample, e.g. cells, tissues, plants or microorganisms or a nucleic acid library , which can contain a candidate gene encoding a gene product conferring an increase in the respective fine chemical after expression, with the nucleic acid molecule of the present invention;
b. identifying the nucleic acid molecules, which hybridize under relaxed stringent conditions with the nucleic acid molecule of the present invention in particular to the nucleic acid molecule sequence as indicated in Table IA or IB, columns 5 or 7, lines 227 to 229 or line 230 or lines 595 to 598, resp., and, optionally, isolating the full length cDNA clone or complete genomic clone;
c. introducing the candidate nucleic acid molecules in host cells, preferably in a plant cell or a microorganism, appropriate for producing the respective fine chemical;
d. expressing the identified nucleic acid molecules in the host cells;
e. assaying the respective fine chemical level in the host cells; and
f. identifying the nucleic acid molecule and its gene product which expression confers an increase in the respective fine chemical level in the host cell after expression compared to the wild type.

**[0394.0.0.17] to [0398.0.0.17]: see [0394.0.0.0] to [0398.0.0.0]**

**[0399.0.17.17]** Furthermore, in one embodiment, the present invention relates to process for the identification of a compound conferring increase of the respective fine chemical production in a plant or microorganism, comprising the steps:
culturing a cell or tissue or microorganism or maintaining a plant expressing the polypeptide according to the invention or a nucleic acid molecule encoding said polypeptide and a readout system capable of interacting with the polypeptide under suitable conditions which permit the interaction of the polypeptide with said readout system in the presence of a compound or a sample comprising a plurality of compounds and capable of providing a detectable signal in response to the binding of a compound to said polypeptide under conditions which permit the expression of said readout system and the polypeptide of the present invention or used in the process of the invention; and
identifying if the compound is an effective agonist by detecting the presence or absence or increase of a signal produced by said readout system.
The screen for a gene product or an agonist conferring an increase in the respective fine chemical production can be performed by growth of an organism for example a microorganism in the presence of growth reducing amounts of an inhibitor of the synthesis of the respective fine chemical. Better growth, e.g. higher dividing rate or high dry mass in comparison to the control under such conditions would identify a gene or gene product or an agonist conferring an increase in respective fine chemical production.

**[00399.1.0.17]** One can think to screen for increased production of the respective fine chemical by for example searching for a resistance to a drug blocking the synthesis of the respective fine chemical and looking whether this effect is dependent on the activity or expression of a polypeptide as indicated in Table IIA or IIB, columns 5 or 7, lines 227 to 229 or line 230 or lines 595 to 598 or a homolog thereof, e.g. comparing the phenotype of nearly identical organisms with low and high activity of a protein as indicated in Table IIA or IIB, columns 5 or 7, lines 227 to 229 or line 230 or lines 595 to 598 after incubation with the drug.

**[0400.0.0.17] to [0415.0.0.17]: see [0400.0.0.0] to [0415.0.0.0]**

**[0416.0.0.17] see [0416.0.0.0]**

**[0417.0.17.17]** The nucleic acid molecule of the invention, the vector of the invention or the nucleic acid construct of the invention may also be useful for the production of organisms resistant to inhibitors of the gamma-aminobutyric acid or shikimate or putrescine biosynthesis pathways. In particular, the overexpression of the polypeptide of the present invention may protect an organism such as a microorganism or a plant against inhibitors, which block the gamma-aminobutyric acid or shikimate or putrescine synthesis.

**[0418.0.0.17] to [0423.0.0.17]: see [0418.0.0.0] to [0423.0.0.0]**

**[0424.0.17.17]** Accordingly, the nucleic acid of the invention, the polypeptide of the invention, the nucleic acid construct of the invention, the organisms, the host cell, the microorgansims, the plant, plant tissue, plant cell, or the part thereof of the invention, the vector of the invention, the agonist identified with the method of the invention, the nucleic acid molecule identified with the method of the present invention, can be used for the production of the respective fine chemical or of the respective fine chemical and one or more other organic acids. Accordingly, the nucleic acid of the invention, or the nucleic acid molecule identified with the method of the present invention or the complement sequences thereof, the polypeptide of the invention, the nucleic acid construct of the invention, the organisms, the host cell, the microorganisms, the plant, plant tissue, plant cell, or the part thereof of the invention, the vector of the invention, the antagonist identified with the method of the invention, the antibody of the present invention, the antisense molecule of the present invention, can be used for the reduction of the respective fine chemical in a organism or part thereof, e.g. in a cell.

**[0425.0.0.17] to [0434.0.0.0]: see [0425.0.0.0] to [0434.0.0.0]**

**[0435.0.17.17]** Example 3: In-vivo and in-vitro mutagenesis

**[0436.0.17.17]** An *in vivo* mutagenesis of organisms such as Saccharomyces, Mortierella, Escherichia and others mentioned above, which are beneficial for the production of gamma-aminobutyric acid or shikimate or putrescine can be carried out by passing a plasmid DNA (or another vector DNA) containing the desired nucleic acid sequence or nucleic acid sequences, e.g. the nucleic acid molecule of the invention or the vector of the invention, through E.coli and other microorganisms (for example Bacillus spp. or yeasts such as Saccharomyces cerevisiae) which are not capable of maintaining the integrity of its genetic information. Usual mutator strains have mutations in the genes for the DNA repair system [for example mutHLS, mutD, mutT and the like; for comparison, see Rupp, W.D. (1996) DNA repair mechanisms in Escherichia coli and Salmonella, pp. 2277-2294, ASM: Washington]. The skilled worker knows these strains. The use of these strains is illustrated for example in Greener, A. and Callahan, M. (1994) Strategies 7; 32-34.
In-vitro mutation methods such as increasing the spontaneous mutation rates by chemical or physical treatment are well known to the skilled person. Mutagens like 5-bromo-uracil, N-methyl-N-nitro-N-nitrosoguanidine (= NTG), ethyl methanesulfonate (=EMS), hydroxylamine and/or nitrous acid are widly used as chemical agents for random in-vitro mutagensis. The most common physical method for mutagensis is the treatment with UV irradiation. Another random mutagenesis technique is the error-prone PCR for introducing amino acid changes into proteins. Mutations are deliberately introduced during PCR through the use of error-prone DNA polymerases and special reaction conditions known to a person skilled in the art. For this method randomized DNA sequences are cloned into expression vectors and the resulting mutant libraries screened for altered or improved protein activity as described below.
Site-directed mutagensis method such as the introduction of desired mutations with an M13 or phagemid vector and short oligonucleotides primers is a well-known approach for site-directed mutagensis. The clou of this method involves cloning of the nucleic acid sequence of the invention into an M13 or phagemid vector, which permits recovery of single-stranded recombinant nucleic acid sequence. A mutagenic oligonucleotide primer is then designed whose sequence is perfectly complementary to nucleic acid sequence in the region to be mutated, but with a single difference: at the intended mutation site it bears a base that is complementary to the desired mutant nucleotide rather than the original. The mutagenic oligonucleotide is then allowed to prime new DNA synthesis to create a complementary full-length sequence containing the desired mutation. Another site-directed mutagensis method is the PCR mismatch primer mutagensis method also known to the skilled person. Dpnl site-directed mutagensis is a further known method as described for example in the Stratagene QuickchangeTM site-directed mutagenesis kit protocol. A huge number of other methods are also known and used in common practice.
Positive mutation events can be selected by screening the organisms for the production of the desired respective fine chemical.

**[0437.0.17.17]** Example 4: DNA transfer between Escherichia coli, Saccharomyces cerevisiae and Mortierella alpina

**[0438.0.17.17]** Shuttle vectors such as pYE22m, pPAC-ResQ, pClasper, pAUR224, pAMH10, pAML10, pAMT10, pAMU10, pGMH10, pGML10, pGMT10, pGMU10, pPGAL1, pPADH1, pTADH1, pTAex3, pNGA142, pHT3101 and derivatives thereof which allow the transfer of nucleic acid sequences between Escherichia coli, Saccharomyces cerevisiae and/or Mortierella alpina are available to the skilled worker. An easy method to isolate such shuttle vectors is disclosed by Soni R. and Murray J.A.H. [Nucleic Acid Research, vol. 20 no. 21, 1992: 5852]: If necessary such shuttle vectors can be constructed easily using standard vectors for E. coli (Sambrook, J. et al., (1989), "Molecular Cloning: A Laboratory Manual", Cold Spring Harbor Laboratory Press or Ausubel, F.M. et al. (1994) "Current Protocols in Molecular Biology", John Wiley & Sons) and/or the aforementioned vectors, which have a replication origin for, and suitable marker from, Escherichia coli, Saccharomyces cerevisiae or Mortierella alpina added. Such replication origins are preferably taken from endogenous plasmids, which have been isolated from species used in the inventive process. Genes, which are used in particular as transformation markers for these species are genes for kanamycin resistance (such as those which originate from the Tn5 or Tn-903 transposon) or for chloramphenicol resistance (Winnacker, E.L. (1987) "From Genes to Clones - Introduction to Gene Technology", VCH, Weinheim) or for other antibiotic resistance genes such as for G418, gentamycin, neomycin, hygromycin or tetracycline resistance.

**[0439.0.17.17]** Using standard methods, it is possible to clone a gene of interest into one of the above-described shuttle vectors and to introduce such hybrid vectors into the microorganism strains used in the inventive process. The transformation of Saccharomyces can be achieved for example by LiCl or sheroplast transformation (Bishop et al., Mol. Cell. Biol., 6, 1986: 3401 - 3409; Sherman et al., Methods in Yeasts in Genetics, [Cold Spring Harbor Lab. Cold Spring Harbor, N. Y.] 1982, Agatep et al., Technical Tips Online 1998, 1:51: P01525 or Gietz et al., Methods Mol. Cell. Biol. 5, 1995: 255f) or electroporation (Delorme E., Appl. Environ. Microbiol., vol. 55, no. 9, 1989 : 2242 - 2246).

**[0440.0.17.17]** If the transformed sequence(s) is/are to be integrated advantageously into the genome of the microorganism used in the inventive process for example into the yeast or fungi genome, standard techniques known to the skilled worker also exist for this purpose. Solinger et al. (Proc Natl Acad Sci U S A., 2001 (15): 8447-8453) and Freedman et al. (Genetics, Vol. 162, 15-27, September 2002,) teaches a homolog recombination system dependent on rad 50, rad51, rad54 and rad59 in yeasts. Vectors using this system for homologous recombination are vectors derived from the Ylp series. Plasmid vectors derived for example from the 2µ-Vector are known by the skilled worker and used for the expression in yeasts. Other preferred vectors are for example pART1, pCHY21 or pEVP11 as they have been described by McLeod et al. (EMBO J. 1987, 6:729-736) and Hoffman et al. (Genes Dev. 5, 1991: :561-571.) or Russell et al. (J. Biol. Chem. 258, 1983: 143-149.). Other beneficial yeast vectors are plasmids of the REP, REP-X, pYZ or RIP series.

**[0441.0.0.17] see [0441.0.0.0]**

**[0442.0.0.17] see [0442.0.0.0]**

**[0443.0.0.17] see [0443.0.0.0]**

**[0444.0.17.17]** Example 6: Growth of genetically modified organism: media and culture conditions

**[0445.0.17.17]** Genetically modified Yeast, Mortierella or Escherichia coli are grown in synthetic or natural growth media known by the skilled worker. A number of different growth media for Yeast, Mortierella or Escherichia coli are well known and widely available. A method for culturing Mortierella is disclosed by Jang et al. [Bot. Bull. Acad. Sin. (2000) 41: 41-48]. Mortierella can be grown at 20°C in a culture medium containing: 10 g/l glucose, 5 g/I yeast extract at pH 6.5. Futhermore Jang et al. teaches a submerged basal medium containing 20 g/l soluble starch, 5 g/l Bacto yeast extract, 10 g/l KNO₃, 1 g/l KH₂PO₄, and 0.5 g/l MgSO₄•7H₂O, pH 6.5.

**[0446.0.0.17] to [0450.0.0.17]: see [0446.0.0.0] to [0450.0.0.0]**

**[0451.0.0.17] see [0451.0.5.5]**

**[0452.0.0.17] to [0453.0.0.17]: see [0452.0.0.0] to [0453.0.0.0]**

**[0454.0.17.17]** Analysis of the effect of the nucleic acid molecule on the production of gamma-aminobutyric acid or shikimate or putrescine

**[0455.0.17.17]** The effect of the genetic modification in plants, fungi, algae, ciliates or on the production of a desired compound (such as a gamma-aminobutyric acid or shikimate or putrescine) can be determined by growing the modified microorganisms or the modified plant under suitable conditions (such as those described above) and analyzing the medium and/or the cellular components for the elevated production of desired product (i.e. of gamma-aminobutyric acid or shikimate or putrescine). These analytical techniques are known to the skilled worker and comprise spectroscopy, thin-layer chromatography, various types of staining methods, enzymatic and microbiological methods and analytical chromatography such as high-performance liquid chromatography (see, for example, Ullman, Encyclopedia of Industrial Chemistry, Vol. A2, p. 89-90 and p. 443-613, VCH: Weinheim (1985); Fallon, A., et al., (1987) "Applications of HPLC in Biochemistry" in: Laboratory Techniques in Biochemistry and Molecular Biology, Vol. 17; Rehm et al. (1993) Biotechnology, Vol. 3, Chapter III: "Product recovery and purification", p. 469-714, VCH: Weinheim; Belter, P.A., et al. (1988) Bioseparations: downstream processing for Biotechnology, John Wiley and Sons; Kennedy, J.F., and Cabral, J.M.S. (1992) Recovery processes for biological Materials, John Wiley and Sons; Shaeiwitz, J.A., and Henry, J.D. (1988) Biochemical Separations, in: Ullmann's Encyclopedia of Industrial Chemistry, Vol. B3; Chapter 11, p. 1-27, VCH: Weinheim; and Dechow, F.J. (1989) Separation and purification techniques in biotechnology, Noyes Publications).

**[0456.0.0.17]: see [0456.0.0.0]**

**[0457.0.17.17]** Example 9: Purification of gamma-aminobutyric acid or shikimate or putrescine

**[0458.0.17.17]** Abbreviations; GC-MS, gas liquid chromatography/mass spectrometry; TLC, thin-layer chromatography.
The unambiguous detection for the presence of gamma-aminobutyric acid or shikimate or putrescine can be obtained by analyzing recombinant organisms using analytical standard methods: LC, LC-MSMS or TLC, as described. The total amount produced in the organism for example in yeasts used in the inventive process can be analysed for example according to the following procedure:
The material such as yeasts, E. coli or plants to be analyzed can be disrupted by sonication, grinding in a glass mill, liquid nitrogen and grinding or via other applicable methods.
Plant material is initially homogenized mechanically by comminuting in a pestle and mortar to make it more amenable to extraction.
A typical sample pretreatment consists of a total lipid extraction using such polar organic solvents as acetone or alcohols as methanol, or ethers, saponification, partition between phases, seperation of non-polar epiphase from more polar hypophasic derivatives and chromatography.
For analysis, solvent delivery and aliquot removal can be accomplished with a robotic system comprising a single injector valve Gilson 232XL and a 402 2S1V diluter [Gilson, Inc. USA, 3000 W. Beltline Highway, Middleton, WI]. For saponification, 3 ml of 50% potassium hydroxide hydro-ethanolic solution (4 water - 1 ethanol) can be added to each vial, followed by the addition of 3 ml of octanol. The saponification treatment can be conducted at room temperature with vials maintained on an IKA HS 501 horizontal shaker [Labworld-online, Inc., Wilmington, NC] for fifteen hours at 250 movements/minute, followed by a stationary phase of approximately one hour.
Following saponification, the supernatant can be diluted with 0.17 ml of methanol. The addition of methanol can be conducted under pressure to ensure sample homogeneity. Using a 0.25 ml syringe, a 0.1 ml aliquot can be removed and transferred to HPLC vials for analysis.
For HPLC analysis, a Hewlett Packard 1100 HPLC, complete with a quaternary pump, vacuum degassing system, six-way injection valve, temperature regulated autosampler, column oven and Photodiode Array detector can be used [Agilent Technologies available through Ultra Scientific Inc., 250 Smith Street, North Kingstown, RI]. The column can be a Waters YMC30, 5-micron, 4.6 x 250 mm with a guard column of the same material [Waters, 34 Maple Street, Milford, MA]. The solvents for the mobile phase can be 81 methanol: 4 water: 15 tetrahydrofuran (THF) stabilized with 0.2% BHT (2,6-di-tert-butyl-4-methylphenol). Injections were 20 I. Separation can be isocratic at 30°C with a flow rate of 1.7 ml/minute. The peak responses can be measured by absorbance at 447 nm.

**[0459.0.17.17]** If required and desired, further chromatography steps with a suitable resin may follow. Advantageously, the gamma-aminobutyric acid or shikimate or putrescine can be further purified with a so-called RTHPLC. As eluent acetonitrile/water or chloroform/acetonitrile mixtures can be used. If necessary, these chromatography steps may be repeated, using identical or other chromatography resins. The skilled worker is familiar with the selection of suitable chromatography resin and the most effective use for a particular molecule to be purified.

**[0460.0.0.17] see [0460.0.0.0]**

**[0461.0.17.17]** Example 10: Cloning SEQ ID NO: 15720, 15762, 15708, 15712, 91094, 91415, 91530 or 91635 for the expression in plants

**[0462.0.0.17] see [0462.0.0.0]**

**[0463.0.17.17]** SEQ ID NO: 15720, 15762, 15708 ,15712, 91094, 91415, 91530 or 91635 is amplified by PCR as described in the protocol of the Pfu Turbo or DNA Herculase polymerase (Stratagene).

**[0464.0.0.0.17] to [0466.0.0.17]: see [0464.0.0.0] to [0466.0.0.0]**

**[0466.1.0.17]** In case the Herculase enzyme can be used for the amplification, the PCR amplification cycles were as follows: 1 cycle of 2-3 minutes at 94°C, followed by 25-30 cycles of in each case 30 seconds at 94°C, 30 seconds at 55-60°C and 5-10 minutes at 72°C, followed by 1 cycle of 10 minutes at 72°C, then 4°C.

**[0467.0.17.17]** The following primer sequences were selected for the gene SEQ ID NO: 15720:
i) forward primer (SEQ ID NO:15760)
   atgaatagcg taaaaagagt aaagct
ii) reverse primer (SEQ ID NO: 15761)
   ctaggccaaa gacatcttag cca
The following primer sequences were selected for the gene SEQ ID NO: 15762:
i) forward primer (SEQ ID NO: 15766)
   atggcaggta tcaagttgac gcat
ii) reverse primer (SEQ ID NO: 15767)
   tcattttgtt aatagttttt tgtatgct
The following primer sequences were selected for the gene SEQ ID NO: 15708
i) forward primer (SEQ ID NO: 15710)
   atggaacaga acaggttcaa gaaag
ii) reverse primer (SEQ ID NO: 15711)
   ttacagtttt tgtttagtcg ttttaac
The following primer sequences were selected for the gene SEQ ID NO: 15712:
i) forward primer (SEQ ID NO: 15718)
   atgagtaatg caaacaatag tgctat
ii) reverse primer (SEQ ID NO: 15719)
   tcaatggtat ttatagccgc attgt
The primer sequences were selected for the genes described in Table III, column 5, lines 227 to 230 or lines 595 to 598 and as described in Table III, column 7, lines 227 to 230 or lines 595 to 598.

**[0468.0.17.17] to [0470.0.17.17]: see [0468.0.0.0] to [0470.0.0.0]**

**[0470.1.17.17]** The PCR-products, produced by *Pfu* Turbo DNA polymerase, were phosphorylated using a T4 DNA polymerase using a standard protocol (e.g. MBI Fermentas) and cloned into the processed binary vector.

**[0471.0.17.17] see [0471.0.0.0]**

**[0471.1.17.17]** The DNA termini of the PCR-products, produced by Herculase DNA polymerase, were blunted in a second synthesis reaction using *Pfu* Turbo DNA polymerase. The composition for the protocol of the blunting the DNA-termini was as follows: 0.2 mM blunting dTTP and 1.25 u *Pfu* Turbo DNA polymerase. The reaction was incubated at 72°C for 30 minutes. Then the PCR-products were phosphorylated using a T4 DNA polymerase using a standard protocol (e.g. MBI Fermentas) and cloned into the processed vector as well.

**[0472.0.17.17] to [0479.0.17.17]: see [0472.0.0.0] to [0479.0.0.0]**

**[0480.0.17.17]** Example 11: Generation of transgenic plants which express SEQ ID NO: 15720,15762,15708,15712, 91094, 91415, 91530 or 91635.

**[0481.0.0.17] to [0513.0.0.17]: see [0481.0.0.0] to [0513.0.0.0]**

**[0514.0.17.17]** As an alternative, gamma-aminobutyric acid can be detected as described in Haak and Reineke, Antimicrob. Agents Chemother. 19(3): 493(1981)
As an alternative, shikimate can be detected as described in Gould and Erickson, J Antibiot 41(5), 688-9 (1988).
As an alternative, putrescine can be detected as described in Endo Y., Anal Biochem. 89(1):235-46(1978).
The results of the different plant analyses can be seen from the table 1 which follows:

**Table 1**

| ORF | metabolite | method | min | max |
|---|---|---|---|---|
| YER156C | gamma-Aminobutyric Acid | GC | 1,68 | 11,29 |
| YFR042W | gamma-Aminobutyric Acid | GC | 1,91 | 2,10 |
| YOR084W | gamma-Aminobutyric Acid | GC | 1,71 | 5,59 |
| YLR375W | Shikimate | GC | 1,14 | 1,26 |
| b0651 | gamma-Aminobutyric acid (GABA) | GC | 1.53 | 3.06 |
| b1693 | Shikimic Acid | GC | 1.15 | 1.31 |
| b2965 | Putrescine | GC | 2.11 | 17.93 |
| b0847 | gamma-Aminobutyric acid (GABA) | GC | 1.85 | 2.87 |

**[0515.0.0.17] to [0552.0.0.17]: see [0515.0.0.0] to [0552.0.0.0]**

**[0552.1.0.17]:** Example 15: Metabolite Profiling Info from *Zea mays*
*Zea mays* plants were engineered as described in Example 14c.
The results of the different *Zea mays* plants analysed can be seen from table 2 which follows:

**Table 2**

| ORF | Metabolite | Min | Max |
|---|---|---|---|
| YLR375W | Shikimate | 2,12 | 5,11 |

Table 2 describes the increase in shikimate in genetically modified corn plants expressing the Saccharomyces cerevisiae nucleic acid sequence YLR375W.
In case the activity of the Saccharomyces cerevisiae protein YLR375W or a protein with an activity being involved in pre-tRNA splicing and in uptake of branched-chain amino acids or its homolog, is increased in corn plants, preferably, an increase of the fine chemical shikimate between 112% and 411% is conferred.

**[00552.2.0.17] : see [00552.2.0.0]**

**[0553.0.17.17]**
1. A process for the production of gamma-aminobutyric acid or shikimate or putrescine, which comprises
   a) increasing or generating the activity of a protein as indicated in Table IIA or IIB, columns 5 or 7, lines 227 to 229 or line 230 or lines 595 to 598 or a functional equivalent thereof in a non-human organism or in one or more parts thereof; and
   b) growing the organism under conditions which permit the production of gamma-aminobutyric acid or shikimate or putrescine in said organism.
2. A process for the production of gamma-aminobutyric acid or shikimate or putrescine, comprising the increasing or generating in an organism or a part thereof the expression of at least one nucleic acid molecule comprising a nucleic acid molecule selected from the group consisting of:
   a) nucleic acid molecule encoding of a polypeptide as indicated in Table IIA or IIB, columns 5 or 7, lines 227 to 229 or line 230 or lines 595 to 598 or a fragment thereof, which confers an increase in the amount of gamma-aminobutyric acid or shikimate or putrescine in an organism or a part thereof;
   b) nucleic acid molecule comprising of a nucleic acid molecule as indicated in Table IA or IB, columns 5 or 7, lines 227 to 229 or line 230 or lines 595 to 598;
   c) nucleic acid molecule whose sequence can be deduced from a polypeptide sequence encoded by a nucleic acid molecule of (a) or (b) as a result of the degeneracy of the genetic code and conferring an increase in the amount of gamma-aminobutyric acid or shikimate or putrescine in an organism or a part thereof;
   d) nucleic acid molecule which encodes a polypeptide which has at least 50% identity with the amino acid sequence of the polypeptide encoded by the nucleic acid molecule of (a) to (c) and conferring an increase in the amount of gamma-aminobutyric acid or shikimate or putrescine in an organism or a part thereof;
   e) nucleic acid molecule which hybidizes with a nucleic acid molecule of (a) to (c) under under stringent hybridisation conditions and conferring an increase in the amount of gamma-aminobutyric acid or shikimate or putrescine in an organism or a part thereof;
   f) nucleic acid molecule which encompasses a nucleic acid molecule which is obtained by amplifying nucleic acid molecules from a cDNA library or a genomic library using the primers or primer pairs as indicated in Table III, columns 5 or 7, lines 227 to 229 or line 230 or lines 595 to 598 and conferring an increase in the amount of gamma-aminobutyric acid or shikimate or putrescine in an organism or a part thereof;
   g) nucleic acid molecule encoding a polypeptide which is isolated with the aid of monoclonal antibodies against a polypeptide encoded by one of the nucleic acid molecules of (a) to (f) and conferring an increase in the amount of gamma-aminobutyric acid or shikimate or putrescine in an organism or a part thereof;
   h) nucleic acid molecule encoding a polypeptide comprising a consensus as indicated in Table IV, column 7, lines 227 to 229 or line 230 or lines 595 to 598 and conferring an increase in the amount of gamma-aminobutyric acid or shikimate or putrescine in an organism or a part thereof; and
   i) nucleic acid molecule which is obtainable by screening a suitable nucleic acid library under stringent hybridization conditions with a probe comprising one of the sequences of the nucleic acid molecule of (a) to (k) or with a fragment thereof having at least 15 nt, preferably 20 nt, 30 nt, 50 nt, 100 nt, 200 nt or 500 nt of the nucleic acid molecule characterized in (a) to (k) and conferring an increase in the amount of gamma-aminobutyric acid or shikimate or putrescine in an organism or a part thereof.
   or comprising a sequence which is complementary thereto.
3. The process of claim 1 or 2, comprising recovering of the free or bound gamma-aminobutyric acid or shikimate or putrescine.
4. The process of any one of claim 1 to 3, comprising the following steps:
   a) selecting an organism or a part thereof expressing a polypeptide encoded by the nucleic acid molecule characterized in claim 2;
   b) mutagenizing the selected organism or the part thereof;
   c) comparing the activity or the expression level of said polypeptide in the mutagenized organism or the part thereof with the activity or the expression of said polypeptide of the selected organisms or the part thereof;
   d) selecting the mutated organisms or parts thereof, which comprise an increased activity or expression level of said polypeptide compared to the selected organism or the part thereof;
   e) optionally, growing and cultivating the organisms or the parts thereof; and
   f) recovering, and optionally isolating, the free or bound gamma-aminobutyric acid or shikimate or putrescine produced by the selected mutated organisms or parts thereof.
5. The process of any one of claims 1 to 4, wherein the activity of said protein or the expression of said nucleic acid molecule is increased or generated transiently or stably.
6. An isolated nucleic acid molecule comprising a nucleic acid molecule selected from the group consisting of:
   a) nucleic acid molecule encoding of a polypeptide as indicated in Table IIA or IIB, columns 5 or 7, lines 227 to 229 or line 230 or lines 595 to 598 or a fragment thereof, which confers an increase in the amount of gamma-aminobutyric acid or shikimate or putrescine in an organism or a part thereof;
   b) nucleic acid molecule comprising of a nucleic acid molecule as indicated in Table IA or IB, columns 5 or 7, lines 227 to 229 or line 230 or lines 595 to 598;
   c) nucleic acid molecule whose sequence can be deduced from a polypeptide sequence encoded by a nucleic acid molecule of (a) or (b) as a result of the degeneracy of the genetic code and conferring an increase in the amount of gamma-aminobutyric acid or shikimate or putrescine in an organism or a part thereof;
   d) nucleic acid molecule which encodes a polypeptide which has at least 50% identity with the amino acid sequence of the polypeptide encoded by the nucleic acid molecule of (a) to (c) and conferring an increase in the amount of gamma-aminobutyric acid or shikimate or putrescine in an organism or a part thereof;
   e) nucleic acid molecule which hybidizes with a nucleic acid molecule of (a) to (c) under stringent hybridisation conditions and conferring an increase in the amount of gamma-aminobutyric acid or shikimate or putrescine in an organism or a part thereof;
   f) nucleic acid molecule which encompasses a nucleic acid molecule which is obtained by amplifying nucleic acid molecules from a cDNA library or a genomic library using the primers or primer pairs as indicated in Table III, column 7, lines 227 to 229 or line 230 or lines 595 to 598 and conferring an increase in the amount of gamma-aminobutyric acid or shikimate or putrescine in an organism or a part thereof;
   g) nucleic acid molecule encoding a polypeptide which is isolated with the aid of monoclonal antibodies against a polypeptide encoded by one of the nucleic acid molecules of (a) to (f) and conferring an increase in the amount of gamma-aminobutyric acid or shikimate or putrescine in an organism or a part thereof;
   h) nucleic acid molecule encoding a polypeptide comprising a consensus as indicated in Table IV, column 7, lines 227 to 229 or line 230 or lines 595 to 598 and conferring an increase in the amount of gamma-aminobutyric acid or shikimate or putrescine in an organism or a part thereof; and
   i) nucleic acid molecule which is obtainable by screening a suitable nucleic acid library under stringent hybridization conditions with a probe comprising one of the sequences of the nucleic acid molecule of (a) to (k) or with a fragment thereof having at least 15 nt, preferably 20 nt, 30 nt, 50 nt, 100 nt, 200 nt or 500 nt of the nucleic acid molecule characterized in (a) to (k) and conferring an increase in the amount of gamma-aminobutyric acid or shikimate or putrescine in an organism or a part thereof.
   whereby the nucleic acid molecule distinguishes over the sequence as indicated in Table IA or IB, columns 5 or 7, lines 227 to 229 or line 230 or lines 595 to 598 by one or more nucleotides.
7. A nucleic acid construct which confers the expression of the nucleic acid molecule of claim 6, comprising one or more regulatory elements.
8. A vector comprising the nucleic acid molecule as claimed in claim 6 or the nucleic acid construct of claim 7.
9. The vector as claimed in claim 8, wherein the nucleic acid molecule is in operable linkage with regulatory sequences for the expression in a prokaryotic or eukaryotic, or in a prokaryotic and eukaryotic, host.
10. A host cell, which has been transformed stably or transiently with the vector as claimed in claim 8 or 9 or the nucleic acid molecule as claimed in claim 6 or the nucleic acid construct of claim 7 or produced as described in claim any one of claims 2 to 5.
11. The host cell of claim 10, which is a transgenic host cell.
12. The host cell of claim 10 or 11, which is a plant cell, an animal cell, a microorganism, or a yeast cell, a fungus cell, a prokaryotic cell, an eukaryotic cell or an archaebacterium.
13. A process for producing a polypeptide, wherein the polypeptide is expressed in a host cell as claimed in any one of claims 10 to 12.
14. A polypeptide produced by the process as claimed in claim 13 or encoded by the nucleic acid molecule as claimed in claim 6 whereby the polypeptide distinguishes over a sequence as indicated in Table IIA or IIB, columns 5 or 7, lines 227 to 229 or line 230 or lines 595 to 598 by one or more amino acids.
15. An antibody, which binds specifically to the polypeptide as claimed in claim 14.
16. A plant tissue, propagation material, harvested material or a plant comprising the host cell as claimed in claim 12 which is plant cell or an Agrobacterium.
17. A method for screening for agonists and antagonists of the activity of a polypeptide encoded by the nucleic acid molecule of claim 6 conferring an increase in the amount of gamma-aminobutyric acid or shikimate or putrescine in an organism or a part thereof comprising:
   (a) contacting cells, tissues, plants or microorganisms which express the a polypeptide encoded by the nucleic acid molecule of claim 6 conferring an increase in the amount of gamma-aminobutyric acid or shikimate or putrescine in an organism or a part thereof with a candidate compound or a sample comprising a plurality of compounds under conditions which permit the expression the polypeptide;
   (b) assaying the gamma-aminobutyric acid or shikimate or putrescine level or the polypeptide expression level in the cell, tissue, plant or microorganism or the media the cell, tissue, plant or microorganisms is cultured or maintained in; and
   (c) identifying a agonist or antagonist by comparing the measured gamma-aminobutyric acid or shikimate or putrescine level or polypeptide expression level with a standard gamma-aminobutyric acid or shikimate or putrescine or polypeptide expression level measured in the absence of said candidate compound or a sample comprising said plurality of compounds, whereby an increased level over the standard indicates that the compound or the sample comprising said plurality of compounds is an agonist and a decreased level over the standard indicates that the compound or the sample comprising said plurality of compounds is an antagonist.
18. A process for the identification of a compound conferring increased gamma - aminobutyric acid or shikimate or putrescine production in a plant or microorganism, comprising the steps:
   (a) culturing a plant cell or tissue or microorganism or maintaining a plant expressing the polypeptide encoded by the nucleic acid molecule of claim 6 conferring an increase in the amount of gamma-aminobutyric acid or shikimate or putrescine in an organism or a part thereof and a readout system capable of interacting with the polypeptide under suitable conditions which permit the interaction of the polypeptide with said readout system in the presence of a compound or a sample comprising a plurality of compounds and capable of providing a detectable signal in response to the binding of a compound to said polypeptide under conditions which permit the expression of said readout system and of the polypeptide encoded by the nucleic acid molecule of claim 6 conferring an increase in the amount of gamma-aminobutyric acid or shikimate or putrescine in an organism or a part thereof;
   (b) identifying if the compound is an effective agonist by detecting the presence or absence or increase of a signal produced by said readout system.
19. A method for the identification of a gene product conferring an increase in gamma-aminobutyric acid or shikimate or putrescine production in a cell, comprising the following steps:
   (a) contacting the nucleic acid molecules of a sample, which can contain a candidate gene encoding a gene product conferring an increase in gamma-aminobutyric acid or shikimate or putrescine after expression with the nucleic acid molecule of claim 6;
   (b) identifying the nucleic acid molecules, which hybridise under relaxed stringent conditions with the nucleic acid molecule of claim 6;
   (c) introducing the candidate nucleic acid molecules in host cells appropriate for producing gamma-aminobutyric acid or shikimate or putrescine ;
   (d) expressing the identified nucleic acid molecules in the host cells;
   (e) assaying the gamma-aminobutyric acid or shikimate or putrescine level in the host cells; and
   (f) identifying nucleic acid molecule and its gene product which expression confers an increase in the gamma-aminobutyric acid or shikimate or putrescine level in the host cell in the host cell after expression compared to the wild type.
20. A method for the identification of a gene product conferring an increase in gamma-aminobutyric acid or shikimate or putrescine production in a cell, comprising the following steps:
   (a) identifiying in a data bank nucleic acid molecules of an organism; which can contain a candidate gene encoding a gene product conferring an increase in the gamma-aminobutyric acid or shikimate or putrescine amount or level in an organism or a part thereof after expression, and which are at least 20% homolog to the nucleic acid molecule of claim 6;
   (b) introducing the candidate nucleic acid molecules in host cells appropriate for producing gamma-aminobutyric acid or shikimate or putrescine;
   (c) expressing the identified nucleic acid molecules in the host cells;
   (d) assaying the gamma-aminobutyric acid or shikimate or putrescine level in the host cells; and
   (e) identifying nucleic acid molecule and its gene product which expression confers an increase in the gamma-aminobutyric acid or shikimate or putrescine level in the host cell after expression compared to the wild type.
21. A method for the production of an agricultural composition comprising the steps of the method of any one of claims 17 to 20 and formulating the compound identified in any one of claims 17 to 20 in a form acceptable for an application in agriculture.
22. A composition comprising the nucleic acid molecule of claim 6, the polypeptide of claim 14, the nucleic acid construct of claim 7, the vector of any one of claims 8 or 9, an antagonist or agonist identified according to claim 17, the compound of claim 18, the gene product of claim 19 or 20, the antibody of claim 15, and optionally an agricultural acceptable carrier.
23. Use of the nucleic acid molecule as claimed in claim 6 for the identification of a nucleic acid molecule conferring an increase of gamma-aminobutyric acid or shikimate or putrescine after expression.
24. Use of the polypeptide of claim 14 or the nucleic acid construct claim 7 or the gene product identified according to the method of claim 19 or 20 for identifying compounds capable of conferring a modulation of gamma-aminobutyric acid or shikimate or putrescine levels in an organism.
25. Agrochemical, pharmaceutical, food or feed composition comprising the nucleic acid molecule of claim 6, the polypeptide of claim 14, the nucleic acid construct of claim 7, the vector of claim 8 or 9, the antagonist or agonist identified according to claim 17, the antibody of claim 15, the plant or plant tissue of claim 16, the harvested material of claim 16, the host cell of claim 10 to 12 or the gene product identified according to the method of claim 19 or 20.
26. The method of any one of claims 1 to 5, the nucleic acid molecule of claim 6, the polypeptide of claim 14, the nucleic acid construct of claim 7, the vector of claim 8 or 9, the antagonist or agonist identified according to claim 17, the antibody of claim 15, the plant or plant tissue of claim 16, the harvested material of claim 16, the host cell of claim 10 to 12 or the gene product identified according to the method of claim 19 or 20, wherein the fine chemical is gamma-aminobutyric acid or shikimate or putrescine.

**[0554.0.0.17] Abstract: see [0554.0.0.0]**

**[0000.0.0.18]** In a further embodiment, the present invention relates to a further process for the production of fine chemicals as defined below and the corresponding embodiments as described herein as follows.
Process for the production of fine chemicals

### Description

**[0001.0.18.18]** The present invention relates to a process for the production of the fine chemical in a microorganism, a plant cell, a plant, a plant tissue or in one or more parts thereof. The invention furthermore relates to nucleic acid molecules, polypeptides, nucleic acid constructs, vectors, antisense molecules, antibodies, host cells, plant tissue, propagation material, harvested material, plants, microorganisms as well as agricultural compositions and to their use.

**[0002.0.18.18]** Coenzymes are molecules that cooperate in the catalytic action of an enzyme. Like enzymes, coenzymes are not irreversibly changed during catalysis; they are either unmodified or regenerated. Each kind of coenzyme has a particular chemical function. Coenzymes may either be attached by covalent bonds to a particular enzyme or exist freely in solution, but in either case they participate intimately in the chemical reactions catalyzed by the enzyme.

**[0003.0.18.18]** Coenzyme Q10 (CoQ 10) or ubiquinone is essentially a vitamin or vitamin-like substance. Disagreements on nomenclature notwithstanding, vitamins are defined as organic compounds essential in minute amounts for normal body function acting as coenzymes or precursors to coenzymes. Coenzyme Q10 or CoQ10 belongs to a family of substances called ubiquinones. Ubiquinones, also known as coenzymes Q and mitoquinones, are lipophilic, water-insoluble substances involved in electron transport and energy production in mitochondria. The basic structure of ubiquinones consists of a benzoquinone "head" and a terpinoid "tail." The "head" structure participates in the redox activity of the electron transport chain. The major difference among the various coenzymes Q is in the number of isoprenoid units (5-carbon structures) in the "tail." Coenzymes Q contain one to 12 isoprenoid units in the "tail"; 10 isoprenoid units are common in animals. Coenzymes Q occur in the majority of aerobic organisms, from bacteria to plants and animals. Two numbering systems exist for designation of the number of isoprenoid units in the terpinoid "tail": coenzyme Qn and coenzyme Q(x). N refers to the number of isoprenoid side chains, and x refers to the number of carbons in the terpinoid "tail" and can be any multiple of five. Thus, coenzyme Q10 refers to a coenzyme Q having 10 isoprenoid units in the "tail." Since each isoprenoid unit has five carbons, coenzyme Q10 can also be designated coenzyme Q(50). The structures of coenzymes Q are analogous to those of vitamin K2. Coenzyme Q10 is also known as Coenzyme Q(5O), CoQ10, CoQ(50), ubiquinone (50), ubiquinol- 10 and ubidecarerone.
They are present naturally in foods and sometimes are also synthesized in the body. CoQ10 likewise is found in small amounts in a wide variety of foods and is synthesized in all tissues. The biosynthesis of CoQ10 from the amino acid tyrosine is a multistage process requiring at least eight vitamins and several trace elements. Coenzymes are cofactors upon which the comparatively large and complex enzymes absolutely depend for their function. Coenzyme Q10 is the coenzyme for at least three mitochondrial enzymes (complexes I, II and III) as well as enzymes in other parts of the cell. Mitochondrial enzymes of the oxidative phosphorylation pathway are essential for the production of the high-energy phosphate, adenosine triphosphate (ATP), upon which all cellular functions depend. The electron and proton transfer functions of the quinone ring are of fundamental importance to all life forms; ubiquinone in the mitochondria of animals, plastoquinone in the chloroplast of plants, and menaquinone in bacteria. The term "bioenergetics" has been used to describe the field of biochemistry looking specifically at cellular energy production. In the related field of free radical chemistry, CoQ10 has been studied in its reduced form as a potent antioxidant. The bioenergetics and free radical chemistry of CoQ10 are reviewed in Gian Paolo Littarru's book, Energy and Defense, published in 1994. The precise chemical structure of CoQ10 is 2,3 dimethoxy-5 methyl-6 decaprenyl benzoquinone

**[0004.0.18.18]** Discovered in 1957, CoQ-10 is also called ubiquinone because it belongs to a class of compounds called quinones, and because it's ubiquitous in living organisms, especially in the heart, liver, and kidneys. It plays a crucial role in producing energy in cells. And it acts as a powerful antioxidant, meaning that it helps neutralize cell-damaging molecules called free radicals. Manufactured by all cells in the body, CoQ-10 is also found in small amounts in foods, notably meat and fish. By the mid-1970's, the industrial technology to produce pure CoQ10 in quantities sufficient for larger clinical trials was established. Principally CoQ10 can be isolated from microorganisms or plants or algae; in particular mitochondria are a common source for CoQ10. Alternatively, they are obtained advantageously from animals or fish. **[0005.0.18.18]** Since the actions of supplemental CoQ10 have yet to be clarified, the mechanism of these actions is a matter of speculation. However, much is known about the biochemistry of CoQ10. CoQ10 is an essential cofactor in the mitochondrial electron transport chain, where it accepts electrons from complex I and II, an activity that is vital for the production of ATP. CoQ10 has antioxidant activity in mitochondria and cellular membranes, protecting against peroxidation of lipid membranes. It also inhibits the oxidation of LDL-cholesterol. LDL-cholesterol oxidation is believed to play a significant role in the pathogenesis of atherosclerosis. CoQ10 is biosynthesized in the body and shares a common synthetic pathway with cholesterol.
CoQ10 levels decrease with aging in humans. Why this occurs is not known but may be due to decreased synthesis and/or increased lipid peroxidation which occurs with aging. Significantly decreased levels of CoQ10 have been noted in a wide variety of diseases in both animal and human studies. CoQ10 deficiency may be caused by insufficient dietary CoQ10, impairment in CoQ10 biosynthesis, excessive utilization of CoQ10 by the body, or any combination of the three. Decreased dietary intake is presumed in chronic malnutrition and cachexia.

**[0006.0.18.18]** The relative contribution of CoQ10 biosynthesis versus dietary CoQ10 is under investigation. Karl Folkers takes the position that the dominant source of CoQ10 in man is biosynthesis. This complex, 17 step process, requiring at least seven vitamins (vitamin B2 - riboflavin, vitamin B3 - niacinamide, vitamin B6, folic acid, vitamin B12, vitamin C, and pantothenic acid) and several trace elements, is, by its nature, highly vulnerable. Karl Folkers argues that suboptimal nutrient intake in man is almost universal and that there is subsequent secondary impairment in CoQ10 biosynthesis. This would mean that average or "normal" levels of CoQ10 are really suboptimal and the very low levels observed in advanced disease states represent only the tip of a deficiency "ice berg".
Supplemental CoQ10 may have cardioprotective, cytoprotective and neuroprotective activities. There are claims that it has positive effects in cancer, muscular dystrophy and immune dysfunction. Similarly, it may inhibit obesity or enhance athletic performance.

**[0007.0.18.18]** HMG-CoA reductase inhibitors used to treat elevated blood cholesterol levels by blocking cholesterol biosynthesis also block CoQ10 biosynthesis. The resulting lowering of blood CoQ10 level is due to the partially shared biosynthetic pathway of CoQ10 and cholesterol. In patients with heart failure this is more than a laboratory observation. It has a significant harmful effect which can be negated by oral CoQ10 supplementation.
Increased body consumption of CoQ10 is the presumed cause of low blood CoQ10 levels seen in excessive exertion, hypermetabolism, and acute shock states. It is likely that all three mechanisms (insufficient dietary CoQ10, impaired CoQ10 biosynthesis, and excessive utilization of CoQ10) are operable to varying degrees in most cases of observed CoQ10 deficiency.

**[0008.0.18.18]** In nature, Coenzymes Q0 to Q9 are found as well. E.g. Coenzyme Q9 is a derivative of CoQ10 found e.g. in the chloroplast of plants. Coenzyme Q9 has a shorter aliphatic group bound to the ring structure. Due to the high structural homology of Coenzymes Q0 to Q9 are expected to provide the same or very similar activities as CoQ10 in cells or organisms. However, Matsura et al., Biochim Biophys Acta, 1992, 1123(3) pp. 309-15 concluded from their study that CoQ9 constantly acts as a potential antioxidant in hepatocytes whereas CoQ10 manly exhibit its antioxidant activity in cells containing CoQ10 as the predominate CoQ homolog. Coenzyme Q10 is actual a very common ingredient in different types of cosmetics, due to its protective role against radicals and its predicted function in skin tautening.

**[0009.0.18.18]** Thus, Coenzymes, in particular CoQ10 or CoQ9 can be used in a lot of different applications, for example in cosmetics, pharmaceuticals and in feed and food.

**[0010.0.18.18]** Therefore improving the productivity of said Coenzymes and improving the quality of cosmetics, pharmaceuticals, foodstuffs and animal feeds, in particular of nutrition supplements, is an important task of the different industries.

**[0011.0.18.18]** To ensure a high productivity of said Coenzymes in plants or microorganism, it is necessary to manipulate the natural biosynthesis of said Coenzymes in said organisms.

**[0012.0.18.18]** Accordingly, there is still a great demand for new and more suitable genes which encode enzymes or other regulators which participate in the biosynthesis of said Coenzymes and make it possible to produce certain said Coenzymes specifically on an industrial scale without unwanted byproducts forming. In the selection of genes for biosynthesis two characteristics above all are particularly important. On the one hand, there is as ever a need for improved processes for obtaining the highest possible contents of said Coenzymes on the other hand as less as possible byproducts should be produced in the production process.

**[0013.0.0.18]** It was now found that this object is achieved by providing the process according to the invention described herein and the embodiments characterized in the claims.

**[0014.0.18.18]** It was found that the overexpression of the nucleic acid molecules characterized herein confers an increase in the content of Coenzyme Q10 or Coenzyme Q9 in plants. Accordingly, in a first embodiment, the invention relates to a process for the production of Coenyzme Q10 and/or Coenyzme Q9. Accordingly, in a further embodiment, the invention relates to a process for the production of a fine chemical, whereby the fine chemical is Coenzyme Q10 and/or Coenzyme Q9. Accordingly, in the present invention, the term "the fine chemical" as used herein relates to "Coenzyme Q10 and/or Coenzyme Q9". Further, in another embodiment the term "the fine chemicals" as used herein also relates to compositions of the fine chemicals comprising Coenzyme Q10 and/or Coenzyme Q9.

**[0015.0.18.18]** In one embodiment, the term "the respective fine chemicals" means Coenzyme Q10 and/or Coenzyme Q9, depending on the context. For example, the increase of the gene product of a gene with the Gene/ORF Locus name YPR138C, YBR184W, b2699, or b1829 or its homologs as mentioned confer the increase of the level of coenzyme CoQ10 in plants or parts thereof. For example, the increase of the gene product of a gene with the Gene/ORF Locus name YPR172W, YER174C, YER156C, YDR513W, b2426, b2703, b2729, b3644, b3605, b2699, b0730, or b0175 or its homologs as mentioned confers the increase of the level of coenzymes CoQ9 in plants or parts thereof. For example, the increase of the gene product of a gene with the ORF name b2699 from E. coli or its homologs confer the increase of the level of coenzymes CoQ9 and CoQ10 in plants or parts thereof. For example, the increase of a sequence as indicated in the Tables, lines 231 to 234 confers the increase of the level of coenzyme CoQ10 in plants or parts thereof. For example, the increase of a sequence as indicated in the Tables, lines 235 to 242 and/or 599 to 602 confers the increase of the level of coenzyme Q9 in plants or parts thereof.
Accordingly, in one embodiment, the term "the respective fine chemicals" means Coenzyme Q10 and Coenzyme Q9. In one embodiment, the term "the respective fine chemical" means Coenzyme Q10 or Coenzyme Q9. In a further embodiment, the term "the respective fine chemical" means Coenzyme Q10 and/or Coenzyme Q9 and their salts, ester, or thioester or Coenzyme Q10 and/or Coenzyme Q9 in free form or bound to protein(s), e.g. enzyme(s), or peptide(s), e.g. polypeptide(s) or to membranes or parts thereof, e.g. in the form of oils or waxes or in compositions with lipids, oils, fats or lipid mixture, as well as Coenzyme Q10 and/or Coenzyme Q9 in its reduced or oxidized form. In a preferred embodiment, the term "the respective fine chemical" means Coenzyme Q10 or CoQ9, in free form or its salts or bound to peptide(s) or protein(s). Lipids, oils, waxes, fats or lipid mixture shall mean any, lipid, oil, wax and/or fat containing any bound or free Coenzyme Q10 and/or Coenzyme Q9.
In one embodiment, the term "the fine chemical" and the term "the respective fine chemical" mean at least one chemical compound with an activity of the above mentioned fine chemical.

**[0016.0.18.18]** Accordingly, the present invention relates to a process comprising
(a) increasing or generating the activity of one or more of YPR138C, YBR184W, b2699 and/or b1829 protein(s) in a non-human organism in one or more parts thereof and
(b) growing the organism under conditions which permit the production of the respective fine chemical, thus, CoQ10 or the respective fine chemical-comprising compositions, in said organism.
Accordingly, the present invention relates to a process comprising
(a) increasing or generating the activity of one or more of YPR172W, YER174C, YER156C, YDR513W, b2426, b2703, b2729, b3644, b3605, b2699, b0730 and/or b0175 protein(s) in a non-human organism in one or more parts thereof and
(b) growing the organism under conditions which permit the production of the respective fine chemical, thus, CoQ9 or the respective fine chemical-comprising compositions, in said organism.
Accordingly, the present invention relates to a process comprising
(a) increasing or generating the activity of one or more proteins having the activity of a protein indicated in Table II, column 3, lines 231 to 234 or having the sequence of a polypeptide encoded by a nucleic acid molecule indicated in Table I, column 5 or 7, lines 231 to 234, in a non-human organism in one or more parts thereof; and
(b) growing the organism under conditions which permit the production of the respective fine chemical, thus, CoQ10 in said organism.
Accordingly, the present invention relates to a process comprising
(a) increasing or generating the activity of one or more proteins having the activity of a protein indicated in Table II, column 3, lines 235 to 242 and/or 599 to 602 or having the sequence of a polypeptide encoded by a nucleic acid molecule indicated in Table I, column 5 or 7, lines 235 to 242 and/or 599 to 602, in a non-human organism in one or more parts thereof; and
(b) growing the organism under conditions which permit the production of the respective fine chemical, thus, CoQ9 in said organism.

**[0016.1.0.18]** In one embodiment, the method of the present invention confers the increase of the content of more than one of the respective fine chemicals, i.e. of Coenzyme Q9 and/or Coenzyme Q10.
Accordingly, the term "the fine chemical" can mean "Coenzyme Q9", and/or "Coenzyme Q10", owing to circumstances and the context. In order to illustrate that the meaning of the term "the fine chemical" means "Coenyzme Q9" and/or "Coenzyme Q10" the term "the respective fine chemical" is also used.

**[0017.0.0.18]** to **[0018.0.0.18]** see **[0017.0.0.0] to [0018.0.0.0]**

**[0019.0.18.18]** Advantageously the process of the invention leads to an enhanced production of the respective fine chemical. The terms "enhanced" or "increase" mean at least a 10%, 20%, 30%, 40% or 50%, preferably at least 60%, 70%, 80%, 90% or 100%, more preferably 150%, 200%, 300%, 400% or 500% higher production of the respective fine chemical in comparison to the reference as defined below, e.g. that means in comparison to an organism without the aforementioned modification of the activity of an YPR138C, YBR184W, b2699, b1829, and/or YPR172W, YER174C, YER156C, YDR513W, b2426, b2703, b2729, b3644, b3605, b2699, b0730, b0175 protein, resp..

**[0020.0.18.18]** Surprisingly it was found, that the transgenic expression of at least one of the Saccharomyces cerevisiae protein(s) YPR138C or YBR184W, or the E. coli K12 protein(s) b2699 or b1829 in Arabidopsis thaliana conferred an increase in the CoQ10 content of the transformed plants.
Surprisingly it was found, that the transgenic expression of at least one of the Saccharomyces cerevisiae protein(s) YPR172W, YER174C, YER156C or YDR513W or the E. coli K12 protein(s) b2426, b2703, b2729, b3644, b3605, b2699, b0730 or b0175 in Arabidopsis thaliana conferred an increase in the CoQ9 content of the transformed plants. Surprisingly it was found, that the transgenic expression of the E. coli K12 protein b2699 in Arabidopsis thaliana conferred an increase in the CoQ9 and CoQ10 content of the transformed plants.

**[0021.0.0.18]** see **[0021.0.0.0]**

**[0022.0.18.18]**The sequence of b2426 (Accession number NP 416921) from Escherichia coli K12 has been published in Blattner et al., Science 277 (5331), 1453-1474, 1997, and its activity is being defined as a putative oxidoreductase with NAD(P)-binding domain. Accordingly, in one embodiment, the process of the present invention comprises the use of a gene product with an activity of the "ribitol dehydrogenase, short-chain alcohol dehydrogenase homology" superfamily, preferably being involved in C-compound and carbohydrate utilization, metabolism of vitamins, cofactors and prosthetic groups, biosynthesis of secondary products derived from primary amino acids, aerobic aromate catabolism, anaerobic aromate catabolism, and/or catabolism of secondary metabolites, biosynthesis of the cysteine-aromatic group and/or degradation of amino acids of the cysteine-aromatic group, aromate anabolism, more preferred a protein with the activity of a putative oxidoreductase with NAD(P)-binding domain from E. coli or its homolog, e.g. as shown herein in Table II, column 5 or 7, line 599 or as encoded by the nucleic acid molecule shown in Table I, column 5 or 7, line 599, for the production of the respective fine chemical, meaning of Coenzyme Q9, in particular for increasing the amount of Coenzyme Q9, preferably Coenzyme Q9 in free or bound form in an organism or a part thereof, as mentioned.
The sequence of b2729 (Accession number YP 026181) from Escherichia coli K12 has been published in Blattner et al., Science 277 (5331), 1453-1474, 1997, and its activity is being defined as hydrogenase expression/formation protein. Accordingly, in one embodiment, the process of the present invention comprises the use of a gene product with an activity of the "hydrogenase expression/formation protein hypD"-superfamily, preferably a protein with the activity of a hydrogenase expression/formation protein from E. coli or its homolog, e.g. as shown herein in Table II, column 5 or 7, line 601 or as encoded by the nucleic acid molecule shown in Table I, column 5 or 7, line 601, for the production of the respective fine chemical, meaning of Coenzyme Q9, in particular for increasing the amount of Coenzyme Q9, preferably Coenzyme Q9 in free or bound form in an organism or a part thereof, as mentioned.
The sequence of b2703 (Accession number NP 417209) from Escherichia coli K12 has been published in Blattner et al., Science 277 (5331), 1453-1474, 1997, and its activity is being defined as phosphotransferase system enzyme II; glucitol/sorbitol-specific. Accordingly, in one embodiment, the process of the present invention comprises the use of a gene product with an activity of the "phosphotransferase system sorbitol-specific enzyme II, factor II"-superfamily, preferably being involved in plasma membrane, cellular import, modification by phosphorylation, dephosphorylation, C-compound, carbohydrate transport, sugar binding, and/or phosphotransferase system, more preferred a protein with the activity of a phosphotransferase system enzyme II; glucitol/sorbitol-specific from E. coli or its homolog, e.g. as shown herein in Table II, column 5 or 7, line 600 or as encoded by the nucleic acid molecule shown in Table I, column 5 or 7, line 600, for the production of the respective fine chemical, meaning of Coenzyme Q9, in particular for increasing the amount of Coenzyme Q9, preferably Coenzyme Q9 in free or bound form in an organism or a part thereof, as mentioned. The sequence of b3644 (Accession number NP 418101) from Escherichia coli K12 has been published in Blattner et al., Science 277 (5331), 1453-1474, 1997, and its activity is being defined as an uncharacterized stress-induced protein. Accordingly, in one embodiment, the process of the present invention comprises the use of a protein of the superfamily of hypothetical protein HI0467, preferably of a stress-induced protein from E. coli or its homolog, e.g. as shown herein in Table II, column 5 or 7, line 602 or as encoded by the nucleic acid molecule shown in Table I, column 5 or 7, line 602, for the production of the respective fine chemical, meaning of Coenzyme Q9, preferably in free or bound form, in particular for increasing the amount of Coenzyme Q9, preferably in free or bound form in an organism or a part thereof, as mentioned. In one embodiment, in the process of the present invention the activity of an uncharacterized stress-induced protein is increased or generated, e.g. from E. coli or a homolog thereof.
The sequence of b2699 from Escherichia coli K12 has been published in Blattner et al., Science 277(5331), 1453-1474, 1997, and its activity is being defined as a DNA strand exchange and recombination protein with protease and nuclease activity. Accordingly, in one embodiment, the process of the present invention comprises the use of a protein of the superfamily of the recombination protein recA, in particular of a protein with DNA recombination and DNA repair, pheromone response, mating-type determination, sex-specific protein, nucleotide binding and/or a protease and nuclease activity, in particular a DNA strand exchange and recombination protein with protease and nuclease activity or its homologs, e.g. as shown herein in Table II, column 5 or 7, lines 233, or as encoded by the nucleic acid molecule shown in Table I, column 5 or 7, lines 233, for the production of the respective fine chemical, meaning of reduced or oxidized Coenzyme Q10, preferably in free or bound or derivative form in an organism or a part thereof, as mentioned. In one embodiment, in the process of the present invention the activity of a protein with protease and nuclease activity, in particular of a DNA strand exchange and recombination protein with protease and nuclease activity of the superfamily of recombination protein rec A or its homolog is increased or generated, e.g. from E. coli or a homolog thereof.
The sequence of b1829 (ACCESSION NP_416343) from Escherichia coli K12 has been published in Blattner et al., Science 277(5331), 1453-1474, 1997, and its activity is being defined as a heat shock protein with protease activity. Accordingly, in one embodiment, the process of the present invention comprises the use of a protein of the stress response, the pheromone response, the mating type determination, protein modification or having a proteolytic degradation activity or being a sex specific protein, in particular the use of a protease, in particular of a heat shock protein with protease activity, preferably of the superfamily of the heat shock protein htpX, or its homologs, e.g. as shown herein in Table II, column 5 or 7, lines 234, or as encoded by the nucleic acid molecule shown in Table I, column 5 or 7, lines 234, for the production of the respective fine chemical, meaning of reduced or oxidized Coenzyme Q10, e.g. in free or bound form in an organism or a part thereof, as mentioned. In one embodiment, in the process of the present invention the activity of a protease, in particular of a heat shock protein with protease activity, preferably of the superfamily of the heat shock protein htpX, is increased or generated, e.g. from E. coli or a homolog thereof.
The sequence of b3605 (ACCESSION NP_418062) from Escherichia coli K12 has been published in Blattner et al., Science 277(5331), 1453-1474, 1997, and its activity is being defined as a L-lactate dehydrogenase. Accordingly, in one embodiment, the process of the present invention comprises the use of a protein of the superfamily (S)-2-hydroxy-acid oxidase homology, in particular of a protein with an activity in the respiration, lipid, fatty-acid and isoprenoid metabolism, as electron / hydrogen carrier, and/or in the breakdown of lipids, fatty acids and isoprenoids activity, e.g. a L-lactate dehydrogenase, or its homologs, e.g. as shown herein in Table II, column 5 or 7, lines 239, or as encoded by the nucleic acid molecule shown in Table I, column 5 or 7, lines 239, for the production of the respective fine chemical, meaning of reduced or oxidized Coenzyme Q9 preferably in free or bound or derivative form in an organism or a part thereof, as mentioned. In one embodiment, in the process of the present invention the activity of a protein of the superfamily (S)-2-hydroxy-acid oxidase homology, in particular of a protein with L-lactate dehydrogenase or its homolog is increased or generated, e.g. from E. coli or a homolog thereof.
The sequence of b2699 (ACCESSION NP_417179) from Escherichia coli K12 has been published in Blattner et al., Science 277(5331), 1453-1474, 1997, and its activity is being defined as a DNA strand exchange and recombination protein with protease and nuclease activity. Accordingly, in one embodiment, the process of the present invention comprises the use of a protein of the superfamily of the recombination protein recA, in particular of a protein with DNA recombination and DNA repair, pheromone response, mating-type determination, sex-specific protein, nucleotide binding and/or a protease and nuclease activity, in particular a DNA strand exchange and recombination protein with protease and nuclease activity or its homologs, e.g. as shown herein in Table II, column 5 or 7, lines 240, or as encoded by the nucleic acid molecule shown in Table I, column 5 or 7, lines 240, for the production of the respective fine chemical, meaning of reduced or oxidized Coenzyme Q9, preferably in free or bound or derivative form in an organism or a part thereof, as mentioned. In one embodiment, in the process of the present invention the activity of a protein with protease and nuclease activity, in particular of a DNA strand exchange and recombination protein with protease and nuclease activity of the superfamily of recombination protein rec A or its homolog is increased or generated, e.g. from E. coli or a homolog thereof.
The sequence of b0730 (ACCESSION NP_415258) from Escherichia coli K12 has been published in Blattner et al., Science 277(5331), 1453-1474, 1997, and its activity is being defined as transcriptional regulator of succinylCoA synthetase operon and fatty acyl response regulator. Accordingly, in one embodiment, the process of the present invention comprises the use of a protein of the transcription regulator GntR superfamily, in particular of a protein having a regulation of C-compound and carbohydrate utilization, transcriptional control, prokaryotic nucleotide, transcriptional repressor, DNA binding transcriptional regulator of succinylCoA synthetase operon or a fatty acid response regulator activity, e.g. a transcriptional regulator of succinylCoA synthetase operon and fatty acyl response regulator or its homologs, e.g. as shown herein in Table II, column 5 or 7, lines 241, or as encoded by the nucleic acid molecule shown in Table I, column 5 or 7, lines 241, for the production of the respective fine chemical, meaning of reduced or oxidized Coenzyme Q9, preferably in free or bound or derivative form in an organism or a part thereof, as mentioned. In one embodiment, in the process of the present invention the activity of a protein transcriptional regulator of succinylCoA synthetase operon and fatty acyl response regulator or its homolog is increased or generated, e.g. from E. coli or a homolog thereof.
The sequence of b0175 from Escherichia coli K12 has been published in Blattner et al., Science 277(5331), 1453-1474, 1997, and its activity is being defined as a CDP-diglyceride synthase. Accordingly, in one embodiment, the process of the present invention comprises the use of a protein having a activity in the nucleotide-metabolism, phospholipid biosynthesis, lipid, fatty-acid or isoprenoids metabolism, cellular communication, signal transduction, or cellular sensing and response, preferably of a protein of the phophatidate cytidylyltransferase superfamily, preferably of a CDP-diglyceride synthase, e.g. from E. coli or its homolog, e.g. as shown herein, in Table II, column 5 or 7, lines 242, or as encoded by the nucleic acid molecule shown in Table I, column 5 or 7, lines 242, for the production of the respective fine chemical, meaning reduced or oxidized Coenzyme Q9, preferably in free or bound form in an organism or a part thereof, as mentioned. In one embodiment, in the process of the present invention said activity, e.g. the activity of a phosphatidate cytidylytransferase, in particular of a CDP-diglyceride synthase, is increased or generated, e.g. from E. coli or a homolog thereof.
The sequence of YPR138C (ACCESSION NP_015464) from Saccharomyces cerevisiae has been published in Bussey,H et al., Nature 387 (6632 Suppl), 103-105 (1997) and in Goffeau et al., Science 274 (5287), 546-547, 1996 and its cellular activity has characterized as NH4+ transporter. Accordingly, in one embodiment, the process of the present invention comprises the use of a NH4+ transporter or an ammonium transport protein; or a protein of the ammonium transporter nrgA superfamily, in particular the use of a protein having a anion transporters (Cl, SO4, PO4, etc.), other cation transporters (Na, K, Ca , NH4, etc.), nitrogen and sulfur transport, cellular import, and/or plasma membrane activity for the production of Coenzyme Q10. Accordingly, in one embodiment, the process of the present invention comprises the use of YPR138C from Saccharomyces cerevisiae, e.g. as indicated herein in Table II, line 231, columns 3 or 5, or its homologue, e.g. as shown herein in Table II, line 231, column 7, for the production of the respective fine chemical, meaning of Coenzyme Q10, in particular for increasing the amount of reduced and/or oxidized Coenzyme Q10, preferably in free or bound form in an organism or a part thereof, as mentioned. In one embodiment, in the process of the present invention the activity of a NH4+ transporter is increased or generated, e.g. from Saccharomyces cerevisiae or a homolog thereof.
The sequence of YBR184W (Accession NP_009743) from Saccharomyces cerevisiae has been published in Feldmann,H. et al., EMBO J. 13 (24), 5795-5809 (1994) and in Goffeau et al., Science 274 (5287), 546-547, 1996 and its cellular activity has not been identified characterized, yet. Accordingly, in one embodiment, the process of the present invention comprises the use of YBR184W from Saccharomyces cerevisiae, e.g. as indicated herein in Table II, line 232, columns 3 or 5, or its homologue, e.g. as shown herein in Table II, line 232, column 7, for the production of the respective fine chemical, meaning of Coenzyme Q10, in particular for increasing the amount of reduced and/or oxidized Coenzyme Q10, preferably of Coenzyme Q10 in free or bound form in an organism or a part thereof, as mentioned. In one embodiment, in the process of the present invention the activity of YBR 184W from Saccharomyces cerevisiae or a homolog thereof is increased or generated.
The sequence of YPR172W (ACCESSION NP 015498) from Saccharomyces cerevisiae has been published in Bussey,H.et al., Nature 387 (6632 Suppl), 103-105 (1997) and in Goffeau et al., Science 274 (5287), 546-547, 1996 and its cellular activity has not been identified characterized, yet. Accordingly, in one embodiment, the process of the present invention comprises the use of YPR172W from Saccharomyces cerevisiae, e.g. as indicated herein in Table II, line 235, columns 3 or 5, or its homologue, e.g. as shown herein in Table II, line 235, column 7, for the production of the respective fine chemical, meaning of Coenzyme Q9, in particular for increasing the amount of reduced and/or oxidized Coenzyme Q9, preferably of Coenzyme Q9 in free or bound form in an organism or a part thereof, as mentioned. In one embodiment, in the process of the present invention the activity of YPR172W from Saccharomyces cerevisiae or a homolog thereof is increased or generated.
The sequence of YER174C (ACCESSION NP_011101) from Saccharomyces cerevisiae has been published in Dietrich,F.S et al., Nature 387 (6632 Suppl), 78-81 (1997) and in Goffeau et al., Science 274 (5287), 546-547, 1996 and its cellular activity has characterized as hydroperoxide and superoxide-radical responsive glutathione-dependent oxidoreductase. Accordingly, in one embodiment, the process of the present invention comprises the use of hydroperoxide and superoxide-radical responsive glutathione-dependent oxidoreductase or a protein of the thioredoxin homology superfamily, e.g. of a protein having a protein modification, stress response activity for the production of Coenzyme Q9. Accordingly, in one embodiment, the process of the present invention comprises the use of YER174C from Saccharomyces cerevisiae, e.g. as indicated herein in Table II, line 236, columns 3 or 5, or its homologue, e.g. as shown herein in Table II, line 236, column 7, for the production of the respective fine chemical, meaning of Coenzyme Q9, in particular for increasing the amount of reduced and/or oxidized Coenzyme Q9 and/or Coenzyme Q9 in free or bound form in an organism or a part thereof, as mentioned. In one embodiment, in the process of the present invention the activity of a hydroperoxide and superoxide-radical responsive glutathione-dependent oxidoreductase is increased or generated, e.g. from Saccharomyces cerevisiae or a homolog thereof.
The sequence of YER156C (ACCESSION NP_011083) from Saccharomyces cerevisiae has been published in Dietrich et al., Nature 387 (6632 Suppl), 78-81 (1997) and in Goffeau et al., Science 274 (5287), 546-547, 1996 and its cellular activity has not been identified characterized, yet. Accordingly, in one embodiment, the process of the present invention comprises the use of YER156C or a protein of the Arabidopsis thaliana hypothetical protein F2K15.180 superfamily for the production of Coenzyme Q9. Accordingly, in one embodiment, the process of the present invention comprises the use of YER156C from Saccharomyces cerevisiae, e.g. as indicated herein in Table II, line 237, columns 3 or 5, or its homologue, e.g. as shown herein in Table II, line 237, column 7, for the production of the respective fine chemical, meaning of Coenzyme Q9, in particular for increasing the amount of reduced and/or oxidized Coenzyme Q9, preferably of Coenzyme Q9 in free or bound form in an organism or a part thereof, as mentioned. In one embodiment, in the process of the present invention the activity of YER156C from Saccharomyces cerevisiae or a homolog thereof is increased or generated.
The sequence of YDR513W (ACCESSION NP_010801 )from Saccharomyces cerevisiae has been published in Jacq et al., Nature 387 (6632 Suppl), 75-78 (1997) and in Goffeau et al., Science 274 (5287), 546-547, 1996 and its cellular activity has characterized as glutathione reductase. Accordingly, in one embodiment, the process of the present invention comprises the use of glutathione reductase or a protein of the glutaredoxin superfamily, in particular of a protein having a deoxyribonucleotide metabolism, cytoplasm, stress response, detoxification, and/or electron transport and membrane-associated energy conservation activity for the production of Coenzyme Q9. Accordingly, in one embodiment, the process of the present invention comprises the use of YDR513W from Saccharomyces cerevisiae, e.g. as indicated herein in Table II, line 238, columns 3 or 5, or its homologue, e.g. as shown herein in Table II, line 238, column 7, for the production of the respective fine chemical, meaning of Coenzyme Q9, in particular for increasing the amount of reduced and/or oxidized Coenzyme Q9 and/or Coenzyme Q9 in free or bound form in an organism or a part thereof, as mentioned. In one embodiment, in the process of the present invention the activity of a glutathione reductase is increased or generated, e.g. from Saccharomyces cerevisiae or a homolog thereof.

**[0023.0.18.18]** Homologues ( = homologs) of the present gene products can be derived from any organisms as long as the homologue confers the herein mentioned activity, in particular, confers an increase in the respective fine chemical amount or content. Further, in the present invention, the term "homologue" relates to the sequence of an organism having the highest sequence homology to the herein mentioned or listed sequences of all expressed sequences of said organism.
However, the person skilled in the art knows, that, preferably, the homologue has said the-fine-chemical-increasing activity and, if known, the same biological function or activity in the organism as at least one of the protein(s) indicated in Table II, Column 3, e.g. having the sequence of a polypeptide encoded by a nucleic acid molecule comprising the sequence indicated in Table I, Column 5 or 7.
In one embodiment, a homolog of any one of the polypeptides indicated in any one of lines 231 or 232 or 235 to 238 of Table II, column 3 is a homolog having the same or a similar activity as described herein or annotated. In particular an increase of activity confers an increase in the content of the respective fine chemical in the organisms. In one embodiment, the homologue is a homolog with a sequence as indicated in any one of lines 231 or 232 or 235 to 238 of Table I or II, column 7, resp. In one embodiment, the homologue of one of the polypeptides indicated in any one of lines 231 or 232 or 235 to 238 of Table II, column 3, is derived from an eukaryotic organism. In one embodiment, the homologue is derived from Fungi. In one embodiment, the homologue of a polypeptide indicated in any one of lines 231 or 232 or 235 to 238 of Table II, column 3, is derived from Ascomyceta. In one embodiment, the homologue of a polypeptide indicated in any one of lines 231 or 232 or 235 to 238 of Table II, column 3, is derived from Saccharomycotina. In one embodiment, the homologue of a polypeptide indicated in any one of lines 231 or 232 or 235 to 238 of Table II, column 3, is derived from Saccharomycetes. In one embodiment, the homolog of a polypeptide indicated in any one of lines 231 or 232 or 235 to 238 of Table II, column 3, is a homologue being derived from Saccharomycetales. In one embodiment, the homologue of a polypeptide indicated in any one of lines 231 or 232 or 235 to 238 of Table II, column 3, is a homologue having the same or a similar activity being derived from Saccharomycetaceae. In one embodiment, the homologue of a polypeptide indicated in any one of lines 231 or 232 or 235 to 238 of Table II, column 3 is a homologue having the same or a similar activity, in particular an increase of activity confers an increase in the content of Coenzyme Q10 and/or Coenzyme Q9 in a organisms or part thereof, being derived from Saccharomycetes.
In one embodiment, the homolog of the any one of the polypeptides indicated in any one of lines 233 or 234 or 239 to 242 and/or 599 to 602 of Table II, column 3, is a homolog having the same or a similar activity as described herein or annotated. In particular an increase of activity confers an increase in the content of the respective fine chemical. In one embodiment, the homolog is a homolog with a sequence as indicated in any one of lines 233 or 234 or 239 to 242 and/or 599 to 602 of Table I or II, column 7, resp. In one embodiment, the homolog of one of the polypeptides indicated in any one of lines 233 or 234 or 239 to 242 and/or 599 to 602 of Table II, column 3, is derived from an bacteria. In one embodiment, the homolog of a polypeptide indicated in any one of lines 233 or 234 or 239 to 242 and/or 599 to 602 of Table II, column 3, is derived from Proteobacteria. In one embodiment, the homolog of a polypeptide indicated in any one of lines 233 or 234 or 239 to 242 and/or 599 to 602 of Table II, column 3, is a homolog having the same or a similar activity being derived from Gammaproteobacteria. In one embodiment, the homolog of a polypeptide indicated in any one of lines 233 or 234 or 239 to 242 and/or 599 to 602 of Table II, column 3, is derived from Enterobacteriales. In one embodiment, the homolog of a polypeptide indicated in any one of lines 233 or 234 or 239 to 242 and/or 599 to 602 of Table II, column 3, is a homolog being derived from Enterobacteriaceae. In one embodiment, the homolog of a polypeptide indicated in any one of lines 233 or 234 or 239 to 242 and/or 599 to 602 of Table II, column 3, is a homolog having the same or a similar activity, in particular an increase of activity confers an increase in the content of Coenzyme Q10 and/or Coenzyme Q9 in a organisms or part thereof being derived from Escherichia.

**[0023.1.18.18]** Homologs of the polypeptides indicated in Table II, column 3, lines 231 to 234 may be the polypeptides encoded by the nucleic acid molecules indicated in Table I, column 7, lines 231 to 234, respectively or may be the polypeptides indicated in Table II, column 7, lines 231 to 234 having a Coenzyme Q10 content and/or amount increasing activity.
Homologs of the polypeptides indicated in Table II, column 3, lines 235 to 242 and/or 599 to 602 may be the polypeptides encoded by the nucleic acid molecules indicated in Table I, column 7, lines 235 to 242 and/or 599 to 602, respectively, or may be the polypeptides indicated in Table II, column 7, lines 235 to 242 and/or 599 to 602 having a Coenzyme Q9 content and/or amount increasing activity.

**[0024.0.0.18]** see **[0024.0.0.0]**

**[0025.0.18.18]** In accordance with the invention, a protein or polypeptide has the "activity of an YPR138C, YBR184W, b2699, and/or b1829, and/or YPR172W, YER174C, YER156C, YDR513W, b2426, b2703, b2729, b3644, b3605, b2699, b0730, and/or b0175 protein" if its *de novo* activity or its increased expression directly or indirectly leads to an increased level of the respective fine chemical, in particular of Coenzyme Q10 and/or Coenzyme Q9, resp., and/or polypeptides, proteins, peptides, enzymes, triglycerides, lipids, oils, waxes, membranes, membrane fractions and/or fats containing Coenzyme Q10 and/or Coenzyme Q9 in the organism or a part thereof, preferably in a cell of said organism. In one embodiment, the protein has the activities of a protein as indicated in Table II, lines 231 to 234 and/or 235 to 242 and/or 599 to 602 , columns 3 or 5. Throughout the specification, in a preferred embodiment, the activity or preferably the biological activity of such a protein or polypeptide or an nucleic acid molecule or sequence encoding such protein or polypeptide is identical or similar if it still has the biological or enzymatic activity protein as indicated in Table II, lines 231 to 234 and/or 235 to 242 and/or 599 to 602 , columns 3 or 5, or which has at least 10% of the original enzymatic activity, preferably at least 20%, particularly preferably at least 30%, most particularly preferably at least 40% in comparison to an, YPR138C, YBR184W, b2699, and/or b1829, and/or YPR172W, YER174C, YER156C, YDR513W, b2426, b2703, b2729, b3644, b3605, b2699, b0730, and/or b0175 protein of Saccharomyces cerevisiae or E. Coli , resp. or a combination thereof.

**[0025.3.18.18]** In one embodiment, the polypeptide of the invention or used in the method of the invention confers said activity, e.g. the increase of the respective fine chemical in an organism or a part thereof, if it is derived from an organism, which is evolutionary close to the organism indicated in Table I, column 4 and is expressed in an organism, which is evolutionary distant to the origin organism. For example origin and expressing organism are derived from different families, orders, classes or phylums whereas origin and the organism indicated in Table I, column 4 are derived from the same families, orders, classes or phylums.

**[0025.2.0.18] see [0025.2.0.0]**

**[0025.1.0.18] see [0025.1.0.0]**

**[0026.0.0.18] to [0033.0.0.18] see[0026.0.0.0] to [0033.0.0.0]**

**[0034.0.18.18]** Preferably, the reference, control or wild type differs from the subject of the present invention only in the cellular activity of the polypeptide of the invention or the polypeptide used in the method of the invention, e.g. as result of an increase in the level of the nucleic acid molecule of the present invention or used in the method of the invention or an increase of the specific activity of the polypeptide of the invention or the polypeptide used in the method of the invention, e.g. by or in the expression level or activity of an protein having the activity of a protein as indicated in Table II, column 3, lines 231 to 234 and/or 235 to 242 and/or 599 to 602 or being encoded by a nucleic acid molecule indicated in Table I, column 5, lines 231 to 234 and/or 235 to 242 and/or 599 to 602 or its homologs, e.g. as indicated in Table I, column 7, lines 231 to 234 and/or 235 to 242 and/or 599 to 602 its biochemical or genetical causes and the increased amount of the respective fine chemical.

**[0035.0.0.18]** to **[0044.0.0.18] see [0035.0.0.0]** to **[0044.0.0.0]**

**[0045.0.18.18]** In one embodiment, in case the activity of the Escherichia coli K12 protein b2426 or its homologs, e.g. as indicated in Table II, columns 5 or 7, line 599, is increased, e.g. the activity of a oxidoreductase with NAD(P)-binding domain is increased. Preferably, an increase of the respective fine chemical, preferably of Coenzyme Q9 between 38% and 77% or more is conferred.
In one embodiment, the activity of the Escherichia coli K12 protein b3644 or its homologs e.g. an uncharacterized stress-induced protein e.g. as indicated in Table II, columns 5 or 7, line 602, is increased, preferably, conferring the increase of the fine chemical, preferably of Coenzyme Q9 between 37% and 130% or more.
In one embodiment, the activity of the Escherichia coli K12 protein b2703 or its homologs e.g. phosphotransferase system enzyme II; glucitol/sorbitol-specific, e.g. as indicated in Table II, columns 5 or 7, line 600, is increased, preferably, conferring the increase of the fine chemical, preferably of Coenzyme Q9 between 37% and 118% or more.
In one embodiment, the activity of the Escherichia coli K12 protein b2729 or its homologs, e.g. an hydrogenase expression/formation protein, e.g. as indicated in Table II, columns 5 or 7, line 601, is increased, preferably, conferring the increase of the fine chemical, preferably of Coenzyme Q9 between 35% and 41 % or more.
In one embodiment, the activity of the Escherichia coli K12 protein b1829 or its homologs, e.g. of a protein of the stress response, the pheromone response, the mating type determination, protein modification or having a proteolytic degradation activity or being a sex specific protein, in particular the use of a protease, in particular of a heat shock protein with protease activity, preferably of the superfamily of the heat shock protein htpX or its homologs, e.g. as indicated in Table II, columns 5 or 7, line 234, is increased, conferring an increase of the respective fine chemical, preferably of Coenzyme Q10 between 48% and 433% or more.
In one embodiment, the activity of the Escherichia coli K12 protein b2699 or its homologs, e.g. a DNA recombination and DNA repair activity, a pheromone response activity, a mating-type determination activity, a sex-specific protein activity, a nucleotide binding activity and/or a protease and nuclease activity, in particular a DNA strand exchange and recombination protein with protease and nuclease activity, in particular of the superfamily of the recombination protein recA, e.g. as indicated in Table II, columns 5 or 7, line 233, is increased, conferring an increase of the respective fine chemical, preferably of Coenzyme Q10 between 62% and 220% or more.
In one embodiment, the activity of the Escherichia coli K12 protein b2699 or its homologs, e.g. a DNA recombination and DNA repair activity, a pheromone response activity, a mating-type determination activity, a sex-specific protein activity, a nucleotide binding activity and/or a protease and nuclease activity, in particular a DNA strand exchange and recombination protein with protease and nuclease activity, in particular of the superfamily of the recombination protein recA, e.g. as indicated in Table II, columns 5 or 7, line 240, is increased, conferring an increase of the respective fine chemical, preferably of Coenzyme Q9 between 34% and 253% or more.
In one embodiment, the activity of the Escherichia coli K12 protein b2699 or its homologs, e.g. a DNA recombination and DNA repair activity, a pheromone response activity, a mating-type determination activity, a sex-specific protein activity, a nucleotide binding activity and/or a protease and nuclease activity, in particular a DNA strand exchange and recombination protein with protease and nuclease activity, in particular of the superfamily of the recombination protein recA, e.g. as indicated in Table II, columns 5 or 7, line 233 and 240, is increased, preferably conferring an increase of the respective fine chemicals, preferably of Coenzyme Q10 between 62% and 220% and Coenzyme Q9 between 34% and 253% or more.
In one embodiment, the activity of the Escherichia coli K12 protein b3605 or its homologs, e.g. of a protein of the superfamily (S)-2-hydroxy-acid oxidase homology, in particular of a protein with an activity in the respiration, lipid, fatty-acid and isoprenoid metabolism, as electron / hydrogen carrier, and/or in the breakdown of lipids, fatty acids and isoprenoids activity, e.g. a L-lactate dehydrogenase, e.g. as indicated in Table II, columns 5 or 7, line 239, is increased, preferably conferring an increase of the respective fine chemical, preferably of Coenzyme Q9 between 35% and 41% or more. In one embodiment, the activity of the Escherichia coli K12 protein b0730 or its homologs, e.g. of a protein transcriptional regulator of succinylCoA synthetase operon and fatty acyl response regulator, e.g. as indicated in Table II, columns 5 or 7, line 241, is increased, preferably conferring an increase of the respective fine chemical, preferably of Coenzyme Q9 between 30% and 203% or more .
In one embodiment, the activity of the Escherichia coli K12 protein b0175 or its homologs, e.g. of a protein having a activity in the nucleotide-metabolism, phospholipid biosynthesis, lipid, fatty-acid or isoprenoids metabolism, cellular communication, signal transduction, or cellular sensing and response, preferably of a protein of the phophatidate cytidylyltransferase superfamily, preferably of a CDP-diglyceride synthase, e.g. as indicated in Table II, columns 5 or 7, line 242, is increased, preferably conferring an increase of the respective fine chemical, preferably of Coenzyme Q9 between 45% and 70% or more .
In one embodiment, the activity of the Saccharomyces cerevisiae protein YDR513W or its homologs, e.g. of glutathione reductase or a protein of the glutaredoxin superfamily, in particular of a protein having a deoxyribonucleotide metabolism, cytoplasm, stress response, detoxification, and/or electron transport and membrane-associated energy conservation activity, e.g. as indicated in Table II, columns 5 or 7, line 238, is increased, preferably, conferring an increase of the respective fine chemical, preferably of Coenzyme Q9 between 31% and 73% or more.
In one embodiment, the activity of the Saccharomyces cerevisiae protein YER156C or its homologs, e.g. of a protein of the Arabidopsis thaliana hypothetical protein F2K15.180 superfamily, e.g. as indicated in Table II, columns 5 or 7, line 237, is increased, preferably, conferring an increase of the respective fine chemical, preferably of free Coenzyme Q9 between 32% and 58% or more.
In one embodiment, the activity of the Saccharomyces cerevisiae protein YER174C or its homologs, e.g. of a Hydroperoxide and superoxide-radical responsive glutathione-dependent oxidoreductase or a protein of the thioredoxin homology superfamily, e.g. of a protein having a protein modification, stress response activity, e.g. as indicated in Table II, columns 5 or 7, line 236, is increased, preferably, conferring an increase of the respective fine chemical, preferably of Coenzyme Q9 between 31% and 72% or more.
In one embodiment, the activity of the Saccharomyces cerevisiae protein YPR172W or its homologs, e.g. as indicated in Table II, columns 5 or 7, line 235, is increased, preferably, conferring an increase of the respective fine chemical, preferably of free Coenzyme Q9 between 34% and 66% or more.
In one embodiment, the activity of the Saccharomyces cerevisiae protein YBR184W or its homologs, e.g. as indicated in Table II, columns 5 or 7, line 232, is increased, preferably, conferring an increase of the respective fine chemical, preferably of free Coenzyme Q10 between 70% and 95% or more.
In one embodiment, the activity of the Saccharomyces cerevisiae protein YPR138C or its homologs, e.g. of a NH4+ transporter or an ammonium transport protein; or a protein of the ammonium transporter nrgA superfamily, in particular the use of a protein having a anion transporters (Cl, SO4, PO4, etc.), other cation transporters (Na, K, Ca , NH4, etc.), nitrogen and sulfur transport, cellular import, and/or plasma membrane activity, e.g. as indicated in Table II, columns 5 or 7, line 231, is increased, preferably, conferring an increase of the respective fine chemical, preferably of free Coenzyme Q10 between 65% and 257% or more.

**[0046.0.18.18]** In one embodiment, the activity of a protein as disclosed in [0016.0.18.18] or its homologs, as indicated in Table I, columns 5 or 7, lines 231 to 234 , e.g. a protein with an activity as defined in [0022.0.18.18], is increased conferring, preferably, an increase of the respective fine chemical, preferably of Coenzyme Q10 and of further vitamin- or coenzyme-activity-having compounds or their precursors.
In one embodiment, the activity of a protein as disclosed in [0016.0.18.18] or its homologs, as indicated in Table I, columns 5 or 7, lines 235 to 242 and/or 599 to 602 , e.g. a protein with an activity as defined in [0022.0.18.18], is increased conferring preferably, an increase of the respective fine chemical, preferably of Coenzyme Q10 and of further vitamin- or coenzyme-activity-having compounds or their precursors.
In one embodiment the activity of one or more of the Saccharomyces cerevisiae or Escherichia coli protein YPR138C, YBR184W, b2699, and/or b1829, and/or YPR172W, YER174C, YER156C, YDR513W, b2426, b2703, b2729, b3644, b3605, b2699, b0730, and/or b0175 or of its homologs with an activity as described above in any one of the paragraphs of section [0045.0.18.18], e.g. a homolog as indicated in Table II, columns 5 or 7, lines 231 to 234 and/or 235 to 242 and/or 599 to 602 , resp., is increased and confers an increased level of the respective fine chemical; preferably of the respective fine chemical and/or of waxes, membranes, membrane fractions, organelles, triglycerides, lipids, oils and/or fats containing an increased level of Coenzyme Q9 and/or Coenzyme Q10.

**[0047.0.0.18] see [0047.0.0.0]**

**[0048.0.18.18]** The respective fine chemical can be contained in the organism either in its free form and/or bound to lipids, waxes, membranes, membrane fractions, proteins, fatty acids or other parts of membranes, in particular to compounds contained in membranes of mitochondria or plastids, resp., or mixtures thereof. Accordingly, in one embodiment, the amount of the free form in a membrane, an organelle, a cell, a tissue, more preferred in an organism e.g. as a plant or a microorganism, or a part thereof, is increased by 3% or more, especially preferably are 10% or more, very especially preferably are more than 30% and most preferably are 70% or more, such as 100%, 300% or 500%. Accordingly, in an other embodiment, the amount of the bound the respective fine chemical in a membrane, an organelle, a cell, a tissue, more preferred in an organism, e.g. as a plant or a microorganism or part thereof, is increased by 3% or more, especially preferably are 10% or more, very especially preferably are more than 30% and most preferably are 70% or more, such as 100%, 300% or 500%.

**[0049.0.18.18]** A protein having an activity conferring an increase in the amount or level of the respective fine chemical has the structure of the polypeptide described herein. In a particular embodiment, the polypeptides used in the process of the present invention or the polypeptide of the present invention comprises the sequence of a consensus sequence as indicated in Table IV, column 7, lines 231 to 234 and/or 235 to 242 and/or 599 to 602 , respectively, or of a polypeptide as indicated in Table II, columns 5, lines 231 to 234 and/or 235 to 242 and/or 599 to 602 , or of a functional homologue thereof as described herein, e.g. as indicated in Table II, column 7, lines 231 to 234 and/or 235 to 242 and/or 599 to 602 , or of a polypeptide encoded by the nucleic acid molecule characterized herein or the nucleic acid molecule according to the invention, for example by a nucleic acid molecule as indicated in Table I, columns 5 or 7, lines 231 to 234 and/or 235 to 242 and/or 599 to 602 , or its herein described functional homologues and has the herein mentioned activity, in particular conferring an increase in the Coenzyme Q10 and/or Coenzyme Q9 level of an organism.

**[0050.0.18.18]** ./.

**[0051.0.18.18]** Owing to the biological activity of the proteins which are used in the process according to the invention and which are encoded by nucleic acid molecules according to the invention, it is possible to produce compositions comprising the respective fine chemical, i.e. an increased amount of the free chemical free or bound, e.g. hydrophobic or lipophilic compositions. Depending on the choice of the organism used for the process according to the present invention, for example a microorganism or a plant, compositions or mixtures of the respective fine chemical and various other coenzymes, vitamins and/or antioxidants, e.g. vitamin B6 or vitamin E, can be produced.

**[0052.0.0.18] see [0052.0.0.0]**

**[0053.0.18.18]** In one embodiment, the process of the present invention comprises one or more of the following steps
a) stabilizing a protein conferring the increased expression of a protein encoded by the nucleic acid molecule of the invention or of the polypeptide of the invention, or the polypeptide used in the method of the invention e.g. of a polypeptide having an YPR138C, YBR184W, b2699 and/or b1829, and/or YPR172W, YER174C, YER156C, YDR513W, b2426, b2703, b2729, b3644, b3605, b2699, b0730, and/or b0175 protein activity e.g. of a protein as indicated in Table II, column 5, lines 231 to 234 and/or 235 to 242 and/or 599 to 602 or being encoded by a nucleic acid molecule indicated in Table I, column 5, lines 231 to 234 and/or 235 to 242 and/or 599 to 602 or its homologs activity having herein-mentioned Coenzyme Q10 and/or Coenzyme Q9 increasing activity, e.g. as indicated in Table II, column 7, lines 231 to 234 and/or 235 to 242 and/or 599 to 602 ;
b) stabilizing a mRNA conferring the increased expression of a protein encoded by the nucleic acid molecule of the invention or used in the method of the invention, e.g. of a polypeptide having a YPR138C, YBR184W, b2699 and/or b1829, and/or YPR172W, YER174C, YER156C, YDR513W, b2426, b2703, b2729, b3644, b3605, b2699, b0730, and/or b0175 protein activity, egg a protein as indicated in Table II, column 5, lines 231 to 234 and/or 235 to 242 and/or 599 to 602 or being encoded by a nucleic acid molecule indicated in Table I, column 5, lines 231 to 234 and/or 235 to 242 and/or 599 to 602 or its homologs activity or of a mRNA encoding the polypeptide of the present invention having herein-mentioned Coenzyme Q10 and/or Coenzyme Q9 increasing activity, e.g. as indicated in Table II, column 7, lines 231 to 234 and/or 235 to 242 and/or 599 to 602 ;
c) increasing the specific activity of a protein conferring the increased expression of a protein encoded by the nucleic acid molecule of the invention or used in the method of the invention or of the polypeptide of the present invention or used in the method of the invention having herein-mentioned Coenzyme Q10 and/or Coenzyme Q9 increasing activity, e.g. of a polypeptide having a YPR138C, YBR184W, b2699 and/or b1829, and/or YPR172W, YER174C, YER156C, YDR513W, b2426, b2703, b2729, b3644, b3605, b2699, b0730, and/or b0175 protein activity, e.g. of a protein as indicated in Table II, column 5, lines 231 to 234 and/or 235 to 242 and/or 599 to 602 or being encoded by a nucleic acid molecule indicated in Table I, column 5, lines 231 to 234 and/or 235 to 242 and/or 599 to 602 or its homologs activity, e.g. as indicated in Table II, column 7, lines 231 to 234 and/or 235 to 242 and/or 599 to 602 , or decreasing the inhibitory regulation of the polypeptide of the invention or the polypeptide used in the method of the invention;
d) generating or increasing the expression of an endogenous or artificial transcription factor mediating the expression of a protein conferring the increased expression of a protein encoded by the nucleic acid molecule of the invention or used in the method of the invention or of the polypeptide of the invention or the polypeptide used in the method of the invention having herein-mentioned Coenzyme Q10 and/or Coenzyme Q9 increasing activity, e.g. of a polypeptide having the YPR138C, YBR184W, b2699, and/or b1829, and/or YPR172W, YER174C, YER156C, YDR513W, b2426, b2703, b2729, b3644, b3605, b2699, b0730, and/or b0175 protein activity, e.g. of a protein as indicated in Table II, column 5, lines 231 to 234 and/or 235 to 242 and/or 599 to 602 or being encoded by a nucleic acid molecule indicated in Table I, column 5, lines 231 to 234 and/or 235 to 242 and/or 599 to 602 , or of their homologs, e.g. as indicated in Table I or II, column 7, lines 231 to 234 and/or 235 to 242 and/or 599 to 602 ;
e) stimulating activity of a protein conferring the increased expression of a protein encoded by the nucleic acid molecule of the present invention or a polypeptide of the present invention having herein-mentioned Coenzyme Q10 and/or Coenzyme Q9 increasing activity, e.g. of a polypeptide having the YPR138C, YBR184W, b2699, and/or b1829, and/or YPR172W, YER174C, YER156C, YDR513W, b2426, b2703, b2729, b3644, b3605, b2699, b0730, and/or b0175 protein activity, e.g. of a protein as indicated in Table II, column 5, lines 231 to 234 and/or 235 to 242 and/or 599 to 602 or being encoded by a nucleic acid molecule indicated in Table I, column 5, lines 231 to 234 and/or 235 to 242 and/or 599 to 602 , or of their homologs, e.g. as indicated in Table I or II, column 7, lines 231 to 234 and/or 235 to 242 and/or 599 to 602 , by adding one or more exogenous inducing factors to the organism or parts thereof;
f) expressing a transgenic gene encoding a protein conferring the increased expression of a polypeptide encoded by the nucleic acid molecule of the present invention or a polypeptide of the present invention, having herein-mentioned Coenzyme Q10 and/or Coenzyme Q9 increasing activity, e.g. of a polypeptide having the YPR138C, YBR184W, b2699, and/or b1829, and/or YPR172W, YER174C, YER156C, YDR513W, b2426, b2703, b2729, b3644, b3605, b2699, b0730, and/or b0175 protein activity, e.g. of a protein as indicated in Table II, column 5, lines 231 to 234 and/or 235 to 242 and/or 599 to 602 or being encoded by a nucleic acid molecule indicated in Table I, column 5, lines 231 to 234 and/or 235 to 242 and/or 599 to 602 , or of their homologs, e.g. as indicated in Table I or II, column 7, lines 231 to 234 and/or 235 to 242 and/or 599 to 602 ,
g) increasing the copy number of a gene conferring the increased expression of a nucleic acid molecule encoding a polypeptide encoded by the nucleic acid molecule of the invention or used in the method of the invention or the polypeptide of the invention or used in the method of the invention having herein-mentioned Coenzyme Q10 and/or Coenzyme Q9 increasing activity, e.g. of a polypeptide having the YPR138C, YBR184W, b2699, b1829, and/or YPR172W, YER174C, YER156C, YDR513W, b2426, b2703, b2729, b3644, b3605, b2699, b0730, b0175 protein activity, e.g. of a protein as indicated in Table II, column 5, lines 231 to 234 and/or 235 to 242 and/or 599 to 602 or being encoded by a nucleic acid molecule indicated in Table I, column 5, lines 231 to 234 and/or 235 to 242 and/or 599 to 602 , or of their homologs, e.g. as indicated in Table I or II, column 7, lines 231 to 234 and/or 235 to 242 and/or 599 to 602 ;
h) Increasing the expression of the endogenous gene encoding the polypeptide of the invention or used in the method of the invention, e.g. a polypeptide having the YPR138C, YBR184W, b2699 and/or b1829, and/or YPR172W, YER174C, YER156C, YDR513W, b2426, b2703, b2729, b3644, b3605, b2699, b0730, and/or b0175 protein activity, e.g. of a protein as indicated in Table II, column 5, lines 231 to 234 and/or 235 to 242 and/or 599 to 602 or being encoded by a nucleic acid molecule indicated in Table I, column 5, lines 231 to 234 and/or 235 to 242 and/or 599 to 602 , or of their homologs, e.g. as indicated in Table I or II, column 7, lines 231 to 234 and/or 235 to 242 and/or 599 to 602 , by adding positive expression or removing negative expression elements, e.g. homologous recombination can be used to either introduce positive regulatory elements like for plants the 35S enhancer into the promoter or to remove repressor elements form regulatory regions. Further gene conversion methods can be used to disrupt repressor elements or to enhance to activity of positive elements. Positive elements can be randomly introduced in plants by T-DNA or transposon mutagenesis and lines can be identified in which the positive elements have be integrated near to a gene of the invention, the expression of which is thereby enhanced;
i) Modulating growth conditions of an organism in such a manner, that the expression or activity of the gene encoding the protein of the invention or the protein itself is enhanced for example microorganisms or plants can be grown for example under a higher temperature regime leading to an enhanced expression of heat shock proteins, which can lead an enhanced fine chemical production; and/or
j) selecting of organisms with especially high activity of the proteins of the invention from natural or from mutagenized resources and breeding them into the target organisms, e.g. the elite crops.

**[0054.0.18.18]** Preferably, said mRNA is the nucleic acid molecule of the present invention and/or the protein conferring the increased expression of a protein encoded by the nucleic acid molecule of the present invention or the polypeptide having the herein mentioned activity is the polypeptide of the present invention, e.g. conferring the increase of Coenzyme Q10 and/or Coenzyme Q9 after increasing the expression or activity of the encoded polypeptide or having the activity of a polypeptide having an YPR138C, YBR184W, b2699 and/or b1829, and/or YPR172W, YER174C, YER156C, YDR513W, b2426, b2703, b2729, b3644, b3605, b2699, b0730, and/or b0175 protein, e.g. of a protein as indicated in Table II, column 5, lines 231 to 234 and/or 235 to 242 and/or 599 to 602 or being encoded by a nucleic acid molecule indicated in Table I, column 5, lines 231 to 234 and/or 235 to 242 and/or 599 to 602 , or of their homologs, e.g. as indicated in Table I or II, column 7, lines 231 to 234 and/or 235 to 242 and/or 599 to 602 .

**[0055.0.0.18] to [0067.0.0.18] see [0055.0.0.0] to [0067.0.0.0]**

**[0068.0.9.9]** The mutation is introduced in such a way that the production of the Coenzyme Q9 and/or Coenzyme Q10 is not adversely affected.

**[0069.0.0.18] see [0069.0.0.0]**

**[0070.0.18.18]** Owing to the introduction of a gene or a plurality of genes conferring the expression of the nucleic acid molecule of the invention or used in the method of the invention or the polypeptide of the invention or used in the method of the invention, for example the nucleic acid construct mentioned below, or encoding the YPR138C, YBR184W, b2699 and/or b1829, and/or YPR172W, YER174C, YER156C, YDR513W, b2426, b2703, b2729, b3644, b3605, b2699, b0730, and/or b0175 protein, e.g. of a protein as indicated in Table II, column 5, lines 231 to 234 and/or 235 to 242 and/or 599 to 602 or being encoded by a nucleic acid molecule indicated in Table I, column 5, lines 231 to 234 and/or 235 to 242 and/or 599 to 602 or of its homologs, e.g. as indicated in Table II, column 7, lines 231 to 234 and/or 235 to 242 and/or 599 to 602 , into an organism alone or in combination with other genes, it is possible not only to increase the biosynthetic flux towards the end product, but also to increase, modify or create de *novo* an advantageous, preferably novel metabolites composition in the organism, e.g. an advantageous hydrophopic composition comprising a higher content of (from a viewpoint of nutrional physiology limited) coenzymes, vitamins and/or antioxidants, e.g. vitamin B6 and/or vitamin E. In one embodiment, the expression of a protein as indicated in Table II, column 5, lines 231 to 234 and/or 235 to 242 and/or 599 to 602 or being encoded by a nucleic acid molecule indicated in Table I, column 5, lines 231 to 234 and/or 235 to 242 and/or 599 to 602 or of its homologs, e.g. as indicated in Table II, column 7, lines 231 to 234 and/or 235 to 242 and/or 599 to 602 conferring an increase of coenzymes, in particular of Coenzyme Q9 and/or Coenzyme Q10, and preferably of further coenzymes, vitamins, and/or antioxidants.

**[0071.0.18.18]** Preferably the composition further comprises higher amounts of metabolites positively affecting or lower amounts of metabolites negatively affecting the nutrition or health of animals or humans provided with said compositions or organisms of the invention or parts thereof. Likewise, the number or activity of further genes which are required for the import or export of nutrients or metabolites, including amino acids, fatty acids, vitamins, coenzymes, antioxidants etc. or any one of their precursors, required for the cell's biosynthesis of the respective fine chemical may be increased so that the concentration of necessary or relevant precursors, e.g. of isoprenoids, acetyl CoA, HMG-CoA, mevalonate, Isopentenyl pyrophosphate, Geranyl pyrophosphate, Farnesyl Pyrophosphate, or other cofactors or intermediates within the organelle, e.g. in mitochondria or plastids, resp., within (a) cell(s) or within the corresponding storage compartments is increased. Owing to the increased or novel generated activity of the polypeptide of the invention or used in the method of the invention or owing to the increased number of nucleic acid sequences of the invention or used in the method of the invention and/or to the modulation of further genes which are involved in the biosynthesis of the respective fine chemical, e.g. by increasing the activity of enzymes synthesizing precursors, e.g. Lovastatin, HMG-CoA Reductase, Mevalonate Kinase, or by destroying the activity of one or more genes which are involved in the breakdown of the respective fine chemical, it is possible to increase the yield, production and/or production efficiency of the respective fine chemical in the host organism, such as plants or the microorganisms.

**[0072.0.18.18]** By influencing the metabolism thus, it is possible to produce, in the process according to the invention, further advantageous compounds. Examples of such compounds are antioxidants, further coenzymes, vitamins, e.g. vitamin B6 or vitamin E, or triglycerides, fatty acids, lipids, oils and/or fats containing Coenzyme Q10 and/or Coenzyme Q9.

**[0073.0.18.18]** Accordingly, in one embodiment, the process according to the invention relates to a process, which comprises:
o) providing a non-human organism, preferably a microorganism, a non-human animal, a plant or animal cell, an organelle, a plant or animal tissue or a plant;
p) increasing the YPR138C, YBR184W, b2699 and/or b1829, and/or YPR172W, YER174C, YER156C, YDR513W, b2426, b2703, b2729, b3644, b3605, b2699, b0730, and/or b0175 protein activity or of a polypeptide being encoded by the nucleic acid molecule of the present invention and described below, e.g. of a protein as indicated in Table II, column 5, lines 231 to 234 and/or 235 to 242 and/or 599 to 602 or being encoded by a nucleic acid molecule indicated in Table I, column 5, lines 231 to 234 and/or 235 to 242 and/or 599 to 602 or of its homologs, e.g. as indicated in Table II, column 7, lines 231 to 234 and/or 235 to 242 and/or 599 to 602 , and conferring an increase of the respective fine chemical in the organism, preferably in the microorganism, the non-human animal, the plant or animal cell, the plant or animal tissue, the organelle or the plant,
q) growing the organism, preferably a microorganism, a non-human animal, a plant or animal cell, a plant or animal tissue or a plant under conditions which permit the production of the respective fine chemical in the organism, preferably the microorganism, the plant cell, the plant tissue or the plant; and
r) if desired, recovering, optionally isolating, the free and/or bound the respective fine chemical and, optionally further free and/or bound vitamins, coenzymes or antioxidants synthesized by the organism, the microorganism, the non-human animal, the plant or animal cell, the plant or animal tissue or the plant.

**[0074.0.18.18]** The organism, in particular the microorganism, the non-human animal, the plant or animal cell, the plant or animal tissue, the organelle or the plant is advantageously grown in such a way that it is not only possible to recover, if desired isolate the free or bound the respective fine chemical or the free and bound the respective fine chemical but as option it is also possible to produce, recover and, if desired isolate, other free or/and bound antioxidants, vitamins or coenzymes and mixtures thereof.

**[0075.0.0.18] to [0077.0.0.18] see [0075.0.0.0] to [0077.0.0.0]**

**[0078.0.18.18]** The organism such as microorganisms or plants or the recovered, and if desired isolated, fine chemical can then be processed further directly into foodstuffs or animal feeds or for other applications, for example according to the disclosures made in the following US patent publications: 6,380,252: Use of L-acetylcarnitine, L-isovalerylcarnitine, L-propionylcarnitine for increasing the levels of IGF-1, 6,372,198: Dentifrice for the mineralization and remineralization of teeth, 6,368,617: Dietary supplement, 6,350,473: Method for treating hypercholesterolemia, hyperlipidemia, and atherosclerosis , 6,335,361: Method of treating benign forgetfulness, 6,329,432: Mesozeaxanthin formulations for treatment of retinal disorders, 6,328,987: Cosmetic skin care compositions containing alpha interferon, 6,312,703: Compressed lecithin preparations, 6,306,392: Composition comprising a carnitine and glutathione, useful to increase the absorption of glutathione and synergize its effects, 6,303,586: Supportive therapy for diabetes, hyperglycemia and hypoglycemia, 6,297,281: Association of no syntase inhibitors with trappers of oxygen reactive forms, 6,294,697: Discrete-length polyethylene glycols, 6,277,842: Dietary supplemental method for fat and weight reduction, 6,261,250: Method and apparatus for enhancing cardiovascular activity and health through rhythmic limb elevation, 6,258,855: Method of retarding and ameliorating carpal tunnel syndrome, 6,258,848: Methods and compositions for increasing insulin sensitivity, 6,258,847: Use of 2-mercaptoethanolamine (2-MEA) and related aminothiol compounds and copper(II)-3,5 di-isopropyl salicylates and related compounds in the prevention and treatment of various diseases, 6,255,354: Preparation of a pulmonary surfactant for instillation and oral application, 6,254,547: Breath methylated alkane contour: a new marker of oxidative stress and disease, 6,248,552: Enzyme-based assay for determining effects of exogenous and endogenous factors on cellular energy production, 6,248,363: Solid carriers for improved delivery of active ingredients in pharmaceutical compositions, 6,245,800: Method of preventing or treating statin-induced toxic effects using L-carnitine or an alkanoyl L-carnitine, 6,245,378: Nutritional supplement for facilitating skeletal muscle adaptation to strenuous exercise and counteracting defatigation in asthenic individuals, 6,242,491: Use of creatine or creatine compounds for skin preservation, 6,232,346: Composition for improvement of cellular nutrition and mitochondrial energetics, 6,231,836: Folic acid dentifrice, 6,228,891: Use of 2,3-dimethoxy-5-methyl-6-decaprenyl-1,4-benzoquinone, 6,228,402: Xylitol-containing non-human foodstuff and method, 6,228,347: Antioxidant gel for gingival conditions, 6,218,436: Pharmaceutically active carotenoids, 6,203,818: Nutritional supplement for cardiovascular health, 6,200,550: Oral care compositions comprising coenzyme Q10, 6,191,172: Water-soluble compositions of bioactive lipophilic compounds , 6,184,255: Pharmaceutical composition comprising coenzyme Q10, 6,166,077: Use of L-acetylcarnitine, L-isovalerylcarnitine, L-propionylcarnitine for increasing the levels of IGF-1, 6,162,419: Stabilized ascorbyl compositions, 6,159,508: Xylitol-containing non-human foodstuff and method, 6,159,476: Herbal supplement for increased muscle strength and endurance for athletes, 6,153,582: Defined serumfree medical solution for ophthalmology, 6,136,859: Pharmaceutical formulation for treating liver disorders, 6,107,281: Compounds and their combinations for the treatment of influenza infection, 6,106,286: Method and device for administering medicine to the periodontium, 6,099,854: Dry composition containing flavonol useful as a food supplement, 6,086,910: Food supplements, 6,080,788: Composition for improvement of cellular nutrition and mitochondrial energetics, 6,069,167: Use of antioxidant agents to treat cholestatic liver disease, 6,063,820: Medical food for diabetics, 6,054,261: Coenzyme Q.sub.10 compositions for organ protection during perfusion, 6,051,250: Process for the stabilization of vesicles of amphiphilic lipid(s) and composition for topical application containing the said stabilized vesicles,
The fermentation broth, fermentation products, plants or plant products can be purified in the customary manner by hydrolysis with strong bases, extraction and crystallization or via thin layer chromatography and other methods known to the person skilled in the art and described herein below. Products of these different work-up procedures are fatty acids or fatty acid compositions which still comprise fermentation broth, plant particles and cell components in different amounts, advantageously in the range of from 0 to 99% by weight, preferably below 80% by weight, especially preferably between below 50% by weight.

**[0079.0.0.18] to [0084.0.0.18] see [0079.0.0.0] to [0084.0.0.0]**

**[0084.2.18.18]** Coenzyme Q10 production was reported in Agrobacterium sp., Protaminobacter rubber and Paracoccus denitrificans. Coenzyme Q9 production was reported in Candida tropicalis. Production of ubiquiones with side chain length of 6-10 units, e.g. including Coenzyme Q10 and Coenzyme Q9 was reported for controlled continuous culture of phototrophic bacteria (wild-type strains of Rhodobacter capsulatus, Rhodobacter sphaeroides, Thiocapsa roseopersicina and Ectothiorhodospira shaposhnikovii. Cells mostly contained one main ubiquinone, whereby the content and composition dependent on growth conditions, substrates and other factors. Preferred is a production of more than 0,1, preferably more than 1 to 6mg/g dry cells in one of said organisms or in any other microorganism, even more preferred are more than 10 mg/g dry cells, 20mg/g dry cells, 50mg/g dry cells, 100mg/g dry cells, 200mg/g dry cells, 300mg/g dry cells, 500mg/g dry cells or more.

**[0084.0.0.18] see [0084.0.0.0]**

**[0085.0.18.18]** With regard to the nucleic acid sequence as depicted a nucleic acid construct which contains a nucleic acid sequence mentioned herein or an organism (= transgenic organism) which is transformed with said nucleic acid sequence or said nucleic acid construct, "transgene" means all those constructs which have been brought about by genetic manipulation methods,preferably in which either
a) a nucleic acid sequence as shown in table I, lines 231 to 234 and/or 235 to 242 and/or 599 to 602 , columns 5 or 7 or a derivative thereof, or
b) a genetic regulatory element, for example a promoter, which is functionally linked to the nucleic acid sequence as shown table I, lines 231 to 234 and/or 235 to 242 and/or 599 to 602 , columns 5 or 7 or a derivative thereof, or
c) (a) and (b)
is/are not present in its/their natural genetic environment or has/have been modified by means of genetic manipulation methods, it being possible for the modification to be, by way of example, a substitution, addition, deletion, inversion or insertion of one or more nucleotide. "Natural genetic environment" means the natural chromosomal locus in the organism of origin or the presence in a genomic library. In the case of a genomic library, the natural, genetic environment of the nucleic acid sequence is preferably at least partially still preserved. The environment flanks the nucleic acid sequence at least on one side and has a sequence length of at least 50 bp, preferably at least 500 bp, particularly preferably at least 1000 bp, very particularly preferably at least 5000 bp.

**[0086.0.0.18] to [0087.0.0.18] see [0086.0.0.0] to [0087.0.0.0]**

**[0088.0.18.18]** In an advantageous embodiment of the invention, the organism takes the form of a plant whose Coenzyme content, in particular its Coenzyme Q9 and/or Coenzyme Q10 content is modified advantageously owing to the nucleic acid molecule of the present invention expressed. This is important for plant breeders since, for example, the nutritional value of plants for feed or nutrition is dependent on the abovementioned coenzymes, in particular on the essential coenzyme Q10, and the general amount of coenzymes as source in feed or food. After the YPR138C, YBR184W, b2699 and/or b1829, and/or YPR172W, YER174C, YER156C, YDR513W, b2426, b2703, b2729, b3644, b3605, b2699, b0730, and/or b0175 protein activity, e.g. of a protein as indicated in Table II, column 5, lines 231 to 234 and/or 235 to 242 and/or 599 to 602 or being encoded by a nucleic acid molecule indicated in Table I, column 5, lines 231 to 234 and/or 235 to 242 and/or 599 to 602 or of its homologs, e.g. as indicated in Table II, column 7, lines 231 to 234 and/or 235 to 242 and/or 599 to 602 , has been increased or generated, or after the expression of nucleic acid molecule or polypeptide according to the invention has been generated or increased, the transgenic plant generated thus is grown on or in a nutrient medium or else in the soil and subsequently harvested.

**[0088.1.0.18] see [0088.1.0.0]**

**[0089.0.0.18] to [0094.0.0.18] see [0089.0.0.0] to [0094.0.0.0]**

**[0095.0.18.18]** It may be advantageous to increase the pool of the respective fine chemical in the transgenic organisms by the process according to the invention in order to isolate high amounts of the essentially pure fine chemical.

**[0096.0.18.18]** In another preferred embodiment of the invention a combination of the increased expression of the nucleic acid sequence or the protein of the invention together with the transformation of a protein or polypeptid for example a coenzyme precursor (e.g. isoprenoids, acetyl CoA, HMG-CoA, mevalonate, isopentyl pyrophosphate, geranyl pyrophosphate, farnesyl pyrophosphate, etc.) transporter protein or a compound, which increases the production of Coenzyme precursors is useful to increase the production of the respective fine chemical (see Bao and Ohlrogge, Plant Physiol. 1999 August; 120 (4): 1057-1062).

**[0097.0.18.18]** In may also be advantageous to increase the content of the lipid bound respective fine chemical.

**[0098.0.18.18]** In a preferred embodiment, the respective fine chemical is produced in accordance with the invention and, if desired, is isolated. The production of further antioxidants, vitamins or coenzymes such as coenzymes Q0, Q1, Q2, Q3, Q4, Q5, Q6, Q7, and/or Q8 or retinal, vitamin E, vitamin B6, or other fat soluble vitamins or mixtures thereof by the process according to the invention is advantageous, e.g. for the production of compositions used in food and/or feed or cosmetic production.

**[0099.0.18.18]** In the case of the fermentation of microorganisms, the abovementioned coenzymes may accumulate in membrane fragments and/or in the lipophilic or hydrophobic fraction. If microorganisms are used in the process according to the invention, the fermentation broth can be processed after the cultivation. Depending on the requirement, all or some of the biomass can be removed from the fermentation broth by separation methods such as, for example, centrifugation, filtration, decanting or a combination of these methods, or else the biomass can be left in the fermentation broth. The fermentation broth can subsequently be reduced, or concentrated, with the aid of known methods such as, for example, rotary evaporator, thin-layer evaporator, falling film evaporator, by reverse osmosis or by nanofiltration.. This concentrated fermentation broth advantageously together with compounds for the formulation can subsequently be processed by lyophilization, spray drying, spray granulation or by other methods. Preferably the Coenzymes or the lipophilic or hydrophobic compositions are isolated from the organisms, such as the microorganisms or plants or the culture medium in or on which the organisms have been grown, or from the organism and the culture medium, in the known manner, for example via extraction, distillation, crystallization, chromatography or a combination of these methods. These purification methods can be used alone or in combination with the aforementioned methods such as the separation and/or concentration methods.

**[0100.0.18.18]** Transgenic plants which comprise Coenzyme Q9 and Coenzyme Q10 synthesized in the process according to the invention can advantageously be marketed directly without there being any need for the membrane fragments, cells, cell fragments, organelles, plastids, waxes, oils, lipids or fats comprising the respective fine chemical to be isolated. For example, as coenzymes may be isolated from membranes, e.g. mitochondrial or plastid membranes, it can be sufficient to isolate only cell fractions comprising said membrane or fragments of said membranes. Plants for the process according to the invention are listed as meaning intact plants and all plant parts, plant organs or plant parts such as leaf, stem, seeds, root, tubers, anthers, fibers, root hairs, stalks, embryos, calli, cotelydons, petioles, harvested material, plant tissue, reproductive tissue and cell cultures which are derived from the actual transgenic plant and/or can be used for bringing about the transgenic plant. In this context, the seed comprises all parts of the seed such as the seed coats, epidermal cells, seed cells, endosperm or embryonic tissue. However, the respective fine chemical produced in the process according to the invention can also be isolated from the organisms, advantageously plants, in the form of their cells, cell fragments, plastids, organelles, membranes, membrane fragments, waxes oils, fats, lipids and/or fatty acids. Coenzyme Q9 or Coenzyme Q10 produced by this process can be obtained by harvesting the organisms, either from the crop in which they grow, or from the field. This can be done via pressing or extraction of the plant parts, preferably the plant seeds. To increase the efficiency of oil extraction it is beneficial to clean, to temper and if necessary to hull and to flake the plant material especially the seeds. In this context, oils, fats, lipids, waxes and/or fatty acids fractions can be obtained by what is known as cold beating or cold pressing without applying heat. In the case of microorganisms, the latter are, after harvesting, for example extracted directly without further processing steps or else, after disruption, extracted via various methods with which the skilled worker is familiar. Chemically pure the fine chemical comprising compositions are advantageous for applications in the food or feed industry sector, the cosmetic sector and especially the pharmacological industry sector.

**[0101.0.18.18]see [0101.0.0.0]**

**[0102.0.18.18]**Coenzymes can for example be detected advantageously via LC separation methods. The unambiguous detection for the presence of Coenzymes products can be obtained by analyzing recombinant organisms using analytical standard methods like LC-MS, LC-MSMS, or TLC. The material to be analyzed can be disrupted by sonication, grinding in a glass mill, liquid nitrogen and grinding, cooking, or via other applicable methods; see also Biotechnology of Vitamins, Pigments and Growth Factors, Edited by Erik J. Vandamme, London, 1989, p.96 to 103.

**[0103.0.18.18]** In a preferred embodiment, the present invention relates to a process for the production of the respective fine chemical comprising or generating in an organism or a part thereof the expression of at least one nucleic acid molecule comprising a nucleic acid molecule selected from the group consisting of:
a) nucleic acid molecule encoding, preferably at least the mature form, of the polypeptide shown in table II, lines 231 to 234 and/or 235 to 242 and/or 599 to 602 , columns 5 or 7 or a fragment thereof, which confers an increase in the amount of the respective fine chemical in an organism or a part thereof;
b) nucleic acid molecule comprising, preferably at least the mature form, of the nucleic acid molecule shown in table I, lines 231 to 234 and/or 235 to 242 and/or 599 to 602 , columns 5 or 7;
c) nucleic acid molecule whose sequence can be deduced from a polypeptide sequence encoded by a nucleic acid molecule of (a) or (b) as result of the degeneracy of the genetic code and conferring an increase in the amount of the respective fine chemical in an organism or a part thereof;
d) nucleic acid molecule encoding a polypeptide which has at least 50% identity with the amino acid sequence of the polypeptide encoded by the nucleic acid molecule of (a) to (c) and conferring an increase in the amount of the respective fine chemical in an organism or a part thereof;
e) nucleic acid molecule which hybridizes with a nucleic acid molecule of (a) to (c) under stringent hybridisation conditions and conferring an increase in the amount of the respective fine chemical in an organism or a part thereof;
f) nucleic acid molecule encoding a polypeptide, the polypeptide being derived by substituting, deleting and/or adding one or more amino acids of the amino acid sequence of the polypeptide encoded by the nucleic acid molecules (a) to (d), preferably to (a) to (c) and conferring an increase in the amount of the respective fine chemical in an organism or a part thereof;
g) nucleic acid molecule encoding a fragment or an epitope of a polypeptide which is encoded by one of the nucleic acid molecules of (a) to (e), preferably to (a) to (c) and conferring an increase in the amount of the respective fine chemical in an organism or a part thereof;
h) nucleic acid molecule comprising a nucleic acid molecule which is obtained by amplifying nucleic acid molecules from a cDNA library or a genomic library using the primers shown in table III, lines 231 to 234 and/or 235 to 242 and/or 599 to 602 , column 7 and conferring an increase in the amount of the respective fine chemical in an organism or a part thereof;
i) nucleic acid molecule encoding a polypeptide which is isolated, e.g. from an expression library, with the aid of monoclonal antibodies against a polypeptide encoded by one of the nucleic acid molecules of (a) to (h), preferably to (a) to (c), and conferring an increase in the amount of the respective fine chemical in an organism or a part thereof;
j) nucleic acid molecule which encodes a polypeptide comprising the consensus sequence shown in table IV, lines 231 to 234 and/or 235 to 242 and/or 599 to 602 , column 7 and conferring an increase in the amount of the respective fine chemical in an organism or a part thereof ;
k) nucleic acid molecule comprising one or more of the nucleic acid molecule encoding the amino acid sequence of a polypeptide encoding a domain of the polypeptide shown in table II, lines 231 to 234 and/or 235 to 242 and/or 599 to 602 , columns 5 or 7 and conferring an increase in the amount of the respective fine chemical in an organism or a part thereof; and
l) nucleic acid molecule which is obtainable by screening a suitable library under stringent conditions with a probe comprising one of the sequences of the nucleic acid molecule of (a) to (k), preferably to (a) to (c), or with a fragment of at least 15 nt, preferably 20 nt, 30 nt, 50 nt, 100 nt, 200 nt or 500 nt of the nucleic acid molecule characterized in (a) to (k), preferably to (a) to (c), and conferring an increase in the amount of the respective fine chemical in an organism or a part thereof;
or which comprises a sequence which is complementary thereto.
**[0103.1.18.18]**In one embodiment, the nucleic acid molecule used in the process of the invention distinguishes over the sequence indicated in Table I A, columns 5 or 7, lines 231 to 234 and/or 235 to 242 and/or 599 to 602, resp., by one or more nucleotides. In one embodiment, the nucleic acid molecule used in the process of the invention does not consist of the sequence indicated in Table I A, columns 5 or 7, lines 231 to 234 and/or 235 to 242 and/or 599 to 602, resp.: In one embodiment, the nucleic acid molecule used in the process of the invention is less than 100%, 99,999%, 99,99%, 99,9% or 99% identical to a sequence indicated in Table I A, columns 5 or 7, lines 231 to 234 and/or 235 to 242 and/or 599 to 602, resp.. In another embodiment, the nucleic acid molecule does not encode a polypeptide of a sequence indicated in Table II A, columns 5 or 7, lines 231 to 234 and/or 235 to 242 and/or 599 to 602, resp..

**[0103.2.18.18]** In one embodiment, the nucleic acid molecule used in the process of the invention distinguishes over a or more sequence(s) indicated in Table I B, column 7, lines 231 to 234 and/or 235 to 242 and/or 599 to 602, resp., by one or more nucleotides. In one embodiment, the nucleic acid molecule used in the process of the invention does not consist of a or more sequence(s) indicated in Table I B, column 7, lines 231 to 234 and/or 235 to 242 and/or 599 to 602, resp.. In one embodiment, the nucleic acid molecule used in the process of the invention is less than 100%, 99,999%, 99,99%, 99,9% or 99% identical to a sequence indicated in Table I B, column 7, lines 231 to 234 and/or 235 to 242 and/or 599 to 602, resp.. In another embodiment, the nucleic acid molecule does not encode a polypeptide of a sequence indicated in Table II B, column 7, lines 231 to 234 and/or 235 to 242 and/or 599 to 602, resp..

[0104.0.18.18] In one embodiment, the nucleic acid molecule or the invention or used in the process of the invention distinguishes over the sequence shown in table I, lines 231 to 234 and/or 235 to 242 and/or 599 to 602 , columns 5 or 7 by one or more nucleotides or does not consist of the sequence shown in table I, lines 231 to 234 and/or 235 to 242 and/or 599 to 602 , columns 5 or 7. In one embodiment, the nucleic acid molecule of the present invention is less than 100%, 99,999%, 99,99%, 99,9% or 99% identical to the sequence shown in table I, lines 231 to 234 and/or 235 to 242 and/or 599 to 602 , columns 5 or 7. In another embodiment, the nucleic acid molecule does not encode a polypeptide of the sequence shown in table II, lines 231 to 234 and/or 235 to 242 and/or 599 to 602 , columns 5 or 7.

**[0105.0.0.18] to [0107.0.0.18] see [0105.0.0.0] to [0107.0.0.0]**

**[0108.0.18.18]** Nucleic acid molecules with the sequence shown in table I, lines 231 to 234 and/or 235 to 242 and/or 599 to 602 , columns 5 or 7, nucleic acid molecules which are derived from the amino acid sequences shown in table II, lines 231 to 234 and/or 235 to 242 and/or 599 to 602 , columns 5 or 7 or from polypeptides comprising the consensus sequence shown in table IV, lines 231 to 234 and/or 235 to 242 and/or 599 to 602 , column 7, or their derivatives or homologues encoding polypeptides with the enzymatic or biological activity of an YPR138C, YBR184W, b2699 and/or b1829, and/or YPR172W, YER174C, YER156C, YDR513W, b2426, b2703, b2729, b3644, b3605, b2699, b0730, and/or b0175 protein or conferring a Coenzyme Q10 and/or Coenzyme Q9 increase dependent on its expression or its activity, are advantageously increased in the process according to the invention.

**[0109.0.0.18] see [0109.0.0.0]**

**[0110.0.18.18]** Nucleic acid molecules, which are advantageous for the process according to the invention and which encode polypeptides with an activity of a polypeptide of the invention or the polypeptide used in the method of the invention or used in the process of the invention, e.g. with YPR138C, YBR184W, b2699 and/or b1829, and/or YPR172W, YER174C, YER156C, YDR513W, b2426, b2703, b2729, b3644, b3605, b2699, b0730, and/or b0175 protein activity, e.g. of a protein as indicated in Table II, column 5, lines 231 to 234 and/or 235 to 242 and/or 599 to 602 or being encoded by a nucleic acid molecule indicated in Table I, column 5, lines 231 to 234 and/or 235 to 242 and/or 599 to 602 or of its homologs, e.g. as indicated in Table II, column 7, lines 231 to 234 and/or 235 to 242 and/or 599 to 602 , can be determined from generally accessible databases.

**[0111.0.0.18] see [0111.0.0.0]**

**[0112.0.18.18]** The nucleic acid molecules used in the process according to the invention take the form of isolated nucleic acid sequences, which encode polypeptides with YPR138C, YBR184W, b2699 and/or b1829, and/or YPR172W, YER174C, YER156C, YDR513W, b2426, b2703, b2729, b3644, b3605, b2699, b0730, and/or b0175 protein activity and conferring Coenzyme Q10 and/or Coenzyme Q9 increase, e.g. a protein as indicated in Table II, column 5, lines 231 to 234 and/or 235 to 242 and/or 599 to 602 or being encoded by a nucleic acid molecule indicated in Table I, column 5, lines 231 to 234 and/or 235 to 242 and/or 599 to 602 , or of their homologs, e.g. as indicated in Table I or II, column 7, lines 231 to 234 and/or 235 to 242 and/or 599 to 602 .

**[0113.0.0.18] to [0117.0.0.18] see [0113.0.0.0] to [0117.0.0.0]**

**[0118.1.0.18]** In one embodiment, the nucleic acid molecule according to the invention or used in the process of the invention originates from a plant with a high Coenzyme Q10 and/or Coenzyme Q9 content. In one embodiment, the nucleic acid molecule according to the invention or used in the process of the invention originates from and/or is transformed into a plant with a high Coenzyme Q10 and/or Coenzyme Q9 content.

**[0118.0.0.18] to [0120.0.0.18] see [0118.0.0.0] to [0120.0.0.0]**

**[0121.0.18.18]** However, it is also possible to use artificial sequences, which differ in one or more bases from the nucleic acid sequences found in organisms, or in one or more amino acid molecules from polypeptide sequences found in organisms, in particular from the polypeptide sequences shown in table II, lines 231 to 234 and/or 235 to 242 and/or 599 to 602 , columns 5 or 7 or the functional homologues thereof as described herein, preferably conferring above-mentioned activity, i.e. conferring a respective fine chemical increase after increasing its activity, e.g. having the activity of an YPR138C, YBR184W, b2699 and/or b1829, and/or YPR172W, YER174C, YER156C, YDR513W, b2426, b2703, b2729, b3644, b3605, b2699, b0730, and/or b0175 protein, e.g. of a protein as indicated in Table II, column 5, lines 231 to 234 and/or 235 to 242 and/or 599 to 602 or being encoded by a nucleic acid molecule indicated in Table I, column 5, lines 231 to 234 and/or 235 to 242 and/or 599 to 602 or of its homologs, e.g. as indicated in Table II, column 7, lines 231 to 234 and/or 235 to 242 and/or 599 to 602 .

**[0122.0.0.18] to [0127.0.0.18] see [0122.0.0.0] to [0127.0.0.0]**

**[0128.0.18.18]** Synthetic oligonucleotide primers for the amplification, e.g. as the pairs shown in table III, column 7, lines 231 to 234 and/or 235 to 242 and/or 599 to 602 , by means of polymerase chain reaction can be generated on the basis of a sequence shown herein, for example the sequence shown in table I, lines 231 to 234 and/or 235 to 242 and/or 599 to 602 , columns 5 or 7 or the sequences derived from table II, lines 231 to 234 and/or 235 to 242 and/or 599 to 602 , columns 5 or 7.

**[0129.0.18.18]** Moreover, it is possible to identify conserved regions from various organisms by carrying out protein sequence alignments with the polypeptide used in the process of the invention, in particular with sequences of the polypeptide of the invention, from which conserved regions, and in turn, degenerate primers can be derived. Conserved region for the polypeptide of the invention or used in the method of the invention are indicated in the alignments shown in the figures. Conserved regions are those, which show a very little variation in the amino acid in one particular position of several homologs from different origin. The consensus sequence shown in table IV, lines 231 to 234 and/or 235 to 242 and/or 599 to 602 , column 7 is derived from said alignments.

**[0130.0.18.18]** Degenerated primers can then be utilized by PCR for the amplification of fragments of novel proteins having above-mentioned activity, e.g. conferring the increase of the respective fine chemical, in particular of Coenzyme Q10 and/or Coenzyme Q9 after increasing the expression or activity or having an YPR138C, YBR184W, b2699 and/or b1829, and/or YPR172W, YER174C, YER156C, YDR513W, b2426, b2703, b2729, b3644, b3605, b2699, b0730, and/or b0175 activity or further functional homologs of the polypeptide of the invention, e.g. homologs from a protein as indicated in Table II, column 5, lines 231 to 234 and/or 235 to 242 and/or 599 to 602 or being encoded by a nucleic acid molecule indicated in Table I, column 5, lines 231 to 234 and/or 235 to 242 and/or 599 to 602 or of its homologs, e.g. as indicated in Table II, column 7, lines 231 to 234 and/or 235 to 242 and/or 599 to 602 , from other organisms.

**[0131.0.0.18] to [0138.0.0.18] see [0131.0.0.0] to [0138.0.0.0]**

**[0139.0.18.18]** Polypeptides having above-mentioned activity, i.e. conferring the respective fine chemical increase, derived from other organisms, can be encoded by other DNA sequences which hybridize to the sequences shown in table I, lines 231 to 234 and/or 235 to 242 and/or 599 to 602 , columns 5 or 7 under relaxed hybridization conditions and which code on expression for peptides having the respective fine chemical-increasing activity.

**[0140.0.0.18] to [0146.0.0.18] see [0140.0.0.0] to [0146.0.0.0]**

**[0147.0.18.18]** Further, the nucleic acid molecule of the invention or used in the method of the invention comprises a nucleic acid molecule, which is a complement of one of the nucleotide sequences of above mentioned nucleic acid molecules or a portion thereof. A nucleic acid molecule which is complementary to one of the nucleotide sequences shown in table I, lines 231 to 234 and/or 235 to 242 and/or 599 to 602 , columns 5 or 7 is one which is sufficiently complementary to one of the nucleotide sequences shown in table I, lines 231 to 234 and/or 235 to 242 and/or 599 to 602 , columns 5 or 7 such that it can hybridize to one of the nucleotide sequences shown in table I, lines 231 to 234 and/or 235 to 242 and/or 599 to 602 , columns 5 or 7, thereby forming a stable duplex. Preferably, the hybridisation is performed under stringent hybridization conditions. However, a complement of one of the herein disclosed sequences is preferably a sequence complement thereto according to the base pairing of nucleic acid molecules well known to the skilled person. For example, the bases A and G undergo base pairing with the bases T and U or C, resp. and visa versa. Modifications of the bases can influence the base-pairing partner.

**[0148.0.18.18]** The nucleic acid molecule of the invention or used in the method of the invention comprises a nucleotide sequence which is at least about 30%, 35%, 40% or 45%, preferably at least about 50%, 55%, 60% or 65%, more preferably at least about 70%, 80%, or 90%, and even more preferably at least about 95%, 97%, 98%, 99% or more homologous to a nucleotide sequence shown in table I, lines 231 to 234 and/or 235 to 242 and/or 599 to 602 , columns 5 or 7, or a functional portion thereof and preferably has above mentioned activity, in particular having a the respective fine chemical, in particular Coenzyme Q10 and/or Coenzyme Q9-increasing activity after increasing the activity or an activity of an YPR138C, YBR184W, b2699 and/or b1829, and/or YPR172W, YER174C, YER156C, YDR513W, b2426, b2703, b2729, b3644, b3605, b2699, b0730, and/or b0175 gene product, e.g. a gene encoding a protein as indicated in Table II, column 5, lines 231 to 234 and/or 235 to 242 and/or 599 to 602 or comprising or expressing a nucleic acid molecule indicated in Table I, column 5, lines 231 to 234 and/or 235 to 242 and/or 599 to 602 , or of their homologs, e.g. as indicated in Table I or II, column 7, lines 231 to 234 and/or 235 to 242 and/or 599 to 602 .

**[0149.0.18.18]** The nucleic acid molecule of the invention or used in the method of the invention comprises a nucleotide sequence which hybridizes, preferably hybridizes under stringent conditions as defined herein, to one of the nucleotide sequences shown in table I, lines 231 to 234 and/or 235 to 242 and/or 599 to 602 , columns 5 or 7, or a portion thereof and encodes a protein having above-mentioned activity, e.g. conferring an increase of the fine chemical, and optionally, the activity of YPR138C, YBR184W, b2699 and/or b1829, and/or YPR172W, YER174C, YER156C, YDR513W, b2426, b2703, b2729, b3644, b3605, b2699, b0730, and/or b0175, e.g. of a protein as indicated in Table II, column 5, lines 231 to 234 and/or 235 to 242 and/or 599 to 602 , or being encoded by a nucleic acid molecule indicated in Table I, column 5, lines 231 to 234 and/or 235 to 242 and/or 599 to 602 , or of their homologs, e.g. as indicated in Table I or II, column 7, lines 231 to 234 and/or 235 to 242 and/or 599 to 602 .

**[00149.1.18.18]** Optionally, the nucleotide sequence, which hybridises to one of the nucleotide sequences indicated in Table I, columns 5 or 7, lines 231 to 234 and/or 235 to 242 and/or 599 to 602, resp., preferably Table I B, column 7, lines 231 to 234 and/or 235 to 242 and/or 599 to 602, resp., has further one or more of the activities annotated or known for the a protein as indicated in Table II, column 3, lines 231 to 234 and/or 235 to 242 and/or 599 to 602 .

**[0150.0.18.18]** Moreover, the nucleic acid molecule of the invention or used in the method of the invention can comprise only a portion of the coding region of one of the sequences shown in table I, lines 231 to 234 and/or 235 to 242 and/or 599 to 602 , columns 5 or 7, , resp., preferably Table I B, column 7, lines 231 to 234 and/or 235 to 242 and/or 599 to 602, resp., for example a fragment which can be used as a probe or primer or a fragment encoding a biologically active portion of the polypeptide of the present invention or of a polypeptide used in the process of the present invention, i.e. having above-mentioned activity, e.g. conferring an increase of Coenzyme Q10 and/or Coenzyme Q9 if its activity is increased. The nucleotide sequences determined from the cloning of the present protein-according-to-the-invention-encoding gene allows for the generation of probes and primers designed for use in identifying and/or cloning its homologues in other cell types and organisms. The probe/primer typically comprises substantially purified oligonucleotide. The oligonucleotide typically comprises a region of nucleotide sequence that hybridizes under stringent conditions to at least about 12, 15 preferably about 20 or 25, more preferably about 40, 50 or 75 consecutive nucleotides of a sense strand of one of the sequences set forth, e.g., in table I, lines 231 to 234 and/or 235 to 242 and/or 599 to 602 , columns 5 or 7, resp., preferably Table I B, column 7, lines 231 to 234 and/or 235 to 242 and/or 599 to 602, resp., an anti-sense sequence of one of the sequences, e.g., set forth in table I, lines 231 to 234 and/or 235 to 242 and/or 599 to 602 , columns 5 or 7, or naturally occurring mutants thereof. Primers based on a nucleotide of invention can be used in PCR reactions to clone homologues of the polypeptide of the invention or of the polypeptide used in the process of the invention, e.g. as the primers described in the examples of the present invention, e.g. as shown in the examples. A PCR with the primers pairs shown in table III, lines 231 to 234 and/or 235 to 242 and/or 599 to 602 , column 7 will result in a fragment of YPR138C, YBR184W, b2699 and/or b1829, and/or YPR172W, YER174C, YER156C, YDR513W, b2426, b2703, b2729, b3644, b3605, b2699, b0730, and/or b0175 gene product, e.g. of a gene encoding of a protein as indicated in Table II, column 5, lines 231 to 234 and/or 235 to 242 and/or 599 to 602 , resp., preferably Table II B, column 7, lines 231 to 234 and/or 235 to 242 and/or 599 to 602, resp., or expressing a nucleic acid molecule indicated in Table I, column 5, lines 231 to 234 and/or 235 to 242 and/or 599 to 602 or of its homologs, e.g. as indicated in Table II, column 7, lines 231 to 234 and/or 235 to 242 and/or 599 to 602 .

**[0151.0.0.18] see [0151.0.0.0]**

**[0152.0.18.18]** The nucleic acid molecule of the invention or used in the method of the invention encodes a polypeptide or portion thereof which includes an amino acid sequence which is sufficiently homologous to the amino acid sequence shown in table II, lines 231 to 234 and/or 235 to 242 and/or 599 to 602 , columns 5 or 7 , resp., preferably Table I B, column 7, lines 231 to 234 and/or 235 to 242 and/or 599 to 602, resp., such that the protein or portion thereof maintains the ability to participate in the respective fine chemical production, in particular a Coenzyme Q10 and/or Coenzyme Q9 increasing the activity as mentioned above or as described in the examples in plants or microorganisms is comprised.

**[0153.0.18.18]** As used herein, the language "sufficiently homologous" refers to proteins or portions thereof which have amino acid sequences which include a minimum number of identical or equivalent amino acid residues (e.g., an amino acid residue which has a similar side chain as an amino acid residue in one of the sequences of the polypeptide of the present invention) to an amino acid sequence shown in table II, lines 231 to 234 and/or 235 to 242 and/or 599 to 602 , columns 5 or 7 such that the protein or portion thereof is able to participate in the increase of the respective fine chemical production. In one embodiment, a protein or portion thereof as indicated in Table II, columns 5 or 7, lines 231 to 234 and/or 235 to 242 and/or 599 to 602 has for example an activity of a polypeptide indicated in Table II, column 3, lines 231 to 234 and/or 235 to 242 and/or 599 to 602.

**[0154.0.18.18]** In one embodiment, the nucleic acid molecule of the present invention comprises a nucleic acid that encodes a portion of the protein of the present invention. The protein is at least about 30%, 35%, 40%, 45% or 50%, preferably at least about 55%, 60%, 65% or 70%, and more preferably at least about 75%, 80%, 85%, 90%, 91%, 92%, 93% or 94% and most preferably at least about 95%, 97%, 98%, 99% or more homologous to an entire amino acid sequence of table II, lines 231 to 234 and/or 235 to 242 and/or 599 to 602 , columns 5 or 7 and having above-mentioned activity, e.g. conferring preferably the increase of the respective fine chemical.

**[0155.0.0.18]** to **[0156.0.0.18]see [0155.0.0.0]** to **[0156.0.0.0]**

**[0157.0.18.18]**The invention further relates to nucleic acid molecules that differ from one of the nucleotide sequences shown in table I, lines 231 to 234 and/or 235 to 242 and/or 599 to 602 , columns 5 or 7 (and portions thereof) due to degeneracy of the genetic code and thus encode a polypeptide of the present invention, in particular a polypeptide having above mentioned activity, e.g. conferring an increase in the respective fine chemical in a organism, e.g. as that polypeptide comprising a consensus sequence as indicated in Table IV, column 7, lines 231 to 234 and/or 235 to 242 and/or 599 to 602, resp., or of the polypeptides encoded by the sequence shown in table II, lines 231 to 234 and/or 235 to 242 and/or 599 to 602 , columns 5 or 7 or the functional homologues. Advantageously, the nucleic acid molecule of the invention or used in the method of the invention comprises, or in an other embodiment has, a nucleotide sequence encoding a protein comprising, or in an other embodiment having, an amino acid sequence as indicated in Table IV, column 7, lines 231 to 234 and/or 235 to 242 and/or 599 to 602, resp.,or as shown in table II, lines 231 to 234 and/or 235 to 242 and/or 599 to 602 , columns 5 or 7 or the functional homologues. In a still further embodiment, the nucleic acid molecule of the invention or used in the method of the invention encodes a full length protein which is substantially homologous to an amino acid sequence shown in table II, lines 231 to 234 and/or 235 to 242 and/or 599 to 602 , columns 5 or 7 or the functional homologues. However, in a preferred embodiment, the nucleic acid molecule of the present invention does not consist of the sequence shown in table I, lines 231 to 234 and/or 235 to 242 and/or 599 to 602 , columns 5 or 7, preferably of Table I A, columns 5 or 7, lines231 to 234 and/or 235 to 242 and/or 599 to 602. Preferably, the nucleic acid molecule of the invention is a functional homologue or identical to a nucleic acid molecule indicated in Table I B, column 7, lines 231 to 234 and/or 235 to 242 and/or 599 to 602.

**[0158.0.0.18] to [0160.0.0.18] see [0158.0.0.0] to [0160.0.0.0]**

**[0161.0.18.18]** Accordingly, in another embodiment, a nucleic acid molecule of the invention or used in the method of the invention is at least 15, 20, 25 or 30 nucleotides in length. Preferably, it hybridizes under stringent conditions to a nucleic acid molecule comprising a nucleotide sequence of the nucleic acid molecule of the present invention or used in the process of the present invention, e.g. comprising a sequence shown in table I, lines 231 to 234 and/or 235 to 242 and/or 599 to 602 , columns 5 or 7. The nucleic acid molecule is preferably at least 20, 30, 50, 100, 250 or more nucleotides in length.

**[0162.0.0.18] see [0162.0.0.0]**

**[0163.0.18.18]** Preferably, a nucleic acid molecule of the invention or used in the method of the invention that hybridizes under stringent conditions to a sequence shown in table I, lines 231 to 234 and/or 235 to 242 and/or 599 to 602 , columns 5 or 7 corresponds to a naturally-occurring nucleic acid molecule of the invention or used in the method of the invention. As used herein, a "naturally-occurring" nucleic acid molecule refers to an RNA or DNA molecule having a nucleotide sequence that occurs in nature (e.g., encodes a natural protein). Preferably, the nucleic acid molecule encodes a natural protein having above-mentioned activity, e.g. conferring the respective fine chemical increase after increasing the expression or activity thereof or the activity of a protein of the invention or used in the process of the invention.

**[0164.0.0.18] see [0164.0.0.0]**

**[0165.0.18.18]** For example, nucleotide substitutions leading to amino acid substitutions at "non-essential" amino acid residues can be made in a sequence of the nucleic acid molecule of the invention or used in the process of the invention, e.g. shown in table I, lines 231 to 234 and/or 235 to 242 and/or 599 to 602 , columns 5 or 7, resp., preferably Table I B, column 7, lines 231 to 234 and/or 235 to 242 and/or 599 to 602, resp.,.

**[0166.0.0.18] to [0167.0.0.18] see [0166.0.0.0] to [0167.0.0.0]**

**[0168.0.18.18]** Accordingly, the invention relates to nucleic acid molecules encoding a polypeptide having above-mentioned activity, e.g. conferring an increase in the respective fine chemical in an organisms or parts thereof that contain changes in amino acid residues that are not essential for said activity. Such polypeptides differ in amino acid sequence from a sequence contained in the sequences shown in table II, lines 231 to 234 and/or 235 to 242 and/or 599 to 602 , columns 5 or 7, resp., preferably Table II B, column 7, lines 231 to 234 and/or 235 to 242 and/or 599 to 602, resp., yet retain said activity described herein. The nucleic acid molecule can comprise a nucleotide sequence encoding a polypeptide, wherein the polypeptide comprises an amino acid sequence at least about 50% identical to an amino acid sequence shown in table II, lines 231 to 234 and/or 235 to 242 and/or 599 to 602 , columns 5 or 7, resp., preferably Table II B, column 7, lines 231 to 234 and/or 235 to 242 and/or 599 to 602, resp.,. and is capable of participation in the increase of production of the respective fine chemical after increasing its activity, e.g. its expression. Preferably, the protein encoded by the nucleic acid molecule is at least about 60% identical to the sequence shown in table II, lines 231 to 234 and/or 235 to 242 and/or 599 to 602 , columns 5 or 7, resp., preferably Table II B, column 7, lines 231 to 234 and/or 235 to 242 and/or 599 to 602, resp.,., more preferably at least about 70% identical to one of the sequences shown in table II, lines 231 to 234 and/or 235 to 242 and/or 599 to 602 , columns 5 or 7, resp., preferably Table II B, column 7, lines 231 to 234 and/or 235 to 242 and/or 599 to 602, resp.,., even more preferably at least about 80%, 90%, 95% homologous to the sequence shown in table II, lines 231 to 234 and/or 235 to 242 and/or 599 to 602 , columns 5 or 7, resp., preferably Table II B, column 7, lines 231 to 234 and/or 235 to 242 and/or 599 to 602, resp.,., and most preferably at least about 96%, 97%, 98%, or 99% identical to the sequence shown in table II, lines 231 to 234 and/or 235 to 242 and/or 599 to 602 , columns 5 or 7, resp., preferably Table II B, column 7, lines 231 to 234 and/or 235 to 242 and/or 599 to 602, resp.,..

**[0169.0.0.18] to [0172.0.0.18] see [0169.0.0.0] to [0172.0.0.0]**

**[0173.0.18.18]** For example a sequence, which has 80% homology with sequence SEQ ID NO: 22609 at the nucleic acid level is understood as meaning a sequence which, upon comparison with the sequence SEQ ID NO: 22609 by the above Gap program algorithm with the above parameter set, has a 80% homology.

**[0174.0.0.18] see [0174.0.0.0]**

**[0175.0.18.18]** For example a sequence which has a 80% homology with sequence SEQ ID NO: 22610 at the protein level is understood as meaning a sequence which, upon comparison with the sequence SEQ ID NO: 22610 by the above program algorithm with the above parameter set, has a 80% homology.

**[0176.0.18.18]** Functional equivalents derived from one of the polypeptides as shown in table II, lines 231 to 234 and/or 235 to 242 and/or 599 to 602 , columns 5 or 7 according to the invention by substitution, insertion or deletion have at least 30%, 35%, 40%, 45% or 50%, preferably at least 55%, 60%, 65% or 70% by preference at least 80%, especially preferably at least 85% or 90%, 91%, 92%, 93% or 94%, very especially preferably at least 95%, 97%, 98% or 99% homology with one of the polypeptides as shown in table II, lines 231 to 234 and/or 235 to 242 and/or 599 to 602 , columns 5 or 7 according to the invention and are distinguished by essentially the same properties as a polypeptide as shown in table II, lines 231 to 234 and/or 235 to 242 and/or 599 to 602 , columns 5 or 7.

**[0177.0.18.18]** Functional equivalents derived from a nucleic acid sequence as shown in table I, lines 231 to 234 and/or 235 to 242 and/or 599 to 602 , columns 5 or 7 according to the invention by substitution, insertion or deletion have at least 30%, 35%, 40%, 45% or 50%, preferably at least 55%, 60%, 65% or 70% by preference at least 80%, especially preferably at least 85% or 90%, 91%, 92%, 93% or 94%, very especially preferably at least 95%, 97%, 98% or 99% homology with one of a polypeptides as shown in table II, lines 231 to 234 and/or 235 to 242 and/or 599 to 602 , columns 5 or 7 according to the invention and encode polypeptides having essentially the same properties as a polypeptide as shown in table II, lines 231 to 234 and/or 235 to 242 and/or 599 to 602 , columns 5 or 7.

**[0178.0.0.18] see [0178.0.0.0]**

**[0179.0.18.18]** A nucleic acid molecule encoding an homologous to a protein sequence of table II, lines 231 to 234 and/or 235 to 242 and/or 599 to 602 , columns 5 or 7, resp., preferably Table II B, column 7, lines 231 to 234 and/or 235 to 242 and/or 599 to 602, resp., can be created by introducing one or more nucleotide substitutions, additions or deletions into a nucleotide sequence of the nucleic acid molecule of the present invention, in particular of table I, lines 231 to 234 and/or 235 to 242 and/or 599 to 602 , columns 5 or 7, resp., preferably Table I B, column 7, lines 231 to 234 and/or 235 to 242 and/or 599 to 602, resp., such that one or more amino acid substitutions, additions or deletions are introduced into the encoded protein. Mutations can be introduced into the encoding sequences of table I, lines 231 to 234 and/or 235 to 242 and/or 599 to 602 , columns 5 or 7, resp., preferably Table I B, column 7, lines 231 to 234 and/or 235 to 242 and/or 599 to 602, resp., by standard techniques, such as site-directed mutagenesis and PCR-mediated mutagenesis.

**[0180.0.0.18] to [0183.0.0.18] see [0180.0.0.0] to [0183.0.0.0]**

**[0184.0.18.18]** Homologues of the nucleic acid sequences used, with a sequence shown in table I, lines 231 to 234 and/or 235 to 242 and/or 599 to 602 , columns 5 or 7, comprise also allelic variants with at least approximately 30%, 35%, 40% or 45% homology, by preference at least approximately 50%, 60% or 70%, more preferably at least approximately 90%, 91%, 92%, 93%, 94% or 95% and even more preferably at least approximately 96%, 97%, 98%, 99% or more homology with one of the nucleotide sequences shown or the abovementioned derived nucleic acid sequences or their homologues, derivatives or analogues or parts of these. Allelic variants encompass in particular functional variants which can be obtained by deletion, insertion or substitution of nucleotides from a sequences shown, preferably from table I, lines 231 to 234 and/or 235 to 242 and/or 599 to 602 , columns 5 or 7, resp., preferably Table I B, column 7, lines 231 to 234 and/or 235 to 242 and/or 599 to 602, resp., or from the derived nucleic acid sequences, the intention being, however, that the enzyme activity or the biological activity of the resulting proteins synthesized is advantageously retained or increased.

**[0185.0.18.18]** In one embodiment of the present invention, the nucleic acid molecule of the invention or used in the process of the invention comprises one or more of the sequences shown in any of the table I, lines 231 to 234 and/or 235 to 242 and/or 599 to 602 , columns 5 or 7, resp., preferably Table I B, column 7, lines 231 to 234 and/or 235 to 242 and/or 599 to 602, resp.,. In one embodiment, it is preferred that the nucleic acid molecule comprises as little as possible other nucleotides not shown in any one of table I, lines 231 to 234 and/or 235 to 242 and/or 599 to 602 , columns 5 or 7, resp., preferably Table I B, column 7, lines 231 to 234 and/or 235 to 242 and/or 599 to 602, resp.,. In one embodiment, the nucleic acid molecule comprises less than 500, 400, 300, 200, 100, 90, 80, 70, 60, 50 or 40 further nucleotides. In a further embodiment, the nucleic acid molecule comprises less than 30, 20 or 10 further nucleotides. In one embodiment, the nucleic acid molecule use in the process of the invention is identical to a sequences shown in table I, lines 231 to 234 and/or 235 to 242 and/or 599 to 602 , columns 5 or 7, resp., preferably Table I B, column 7, lines 231 to 234 and/or 235 to 242 and/or 599 to 602, resp.,.

**[0186.0.18.18]** Also preferred is that one or more nucleic acid molecule used in the process of the invention encodes a polypeptide comprising a sequence shown in table II, lines 231 to 234 and/or 235 to 242 and/or 599 to 602 , columns 5 or 7, resp., preferably Table II B, column 7, lines 231 to 234 and/or 235 to 242 and/or 599 to 602, resp.,. In one embodiment, the nucleic acid molecule encodes less than 150, 130, 100, 80, 60, 50, 40 or 30 further amino acids. In a further embodiment, the encoded polypeptide comprises less than 20, 15, 10, 9, 8, 7, 6 or 5 further amino acids. In one embodiment used in the inventive process, the encoded polypeptide is identical to a sequence shown in table II, lines 231 to 234 and/or 235 to 242 and/or 599 to 602 , columns 5 or 7, resp., preferably Table II B, column 7, lines 231 to 234 and/or 235 to 242 and/or 599 to 602, resp.,.

**[0187.0.18.18]** In one embodiment, the nucleic acid molecule of the invention or used in the process encodes a polypeptide comprising a sequence shown in table II, lines 231 to 234 and/or 235 to 242 and/or 599 to 602 , columns 5 or 7, resp., preferably Table II B, column 7, lines 231 to 234 and/or 235 to 242 and/or 599 to 602, resp., and comprises less than 100 further nucleotides. In a further embodiment, said nucleic acid molecule comprises less than 30 further nucleotides. In one embodiment, the nucleic acid molecule used in the process is identical to a coding sequence of a sequence shown in table I, lines 231 to 234 and/or 235 to 242 and/or 599 to 602 , columns 5 or 7, resp., preferably Table I B, column 7, lines 231 to 234 and/or 235 to 242 and/or 599 to 602, resp.,.

**[0188.0.18.18]** Polypeptides (= proteins), which still have the essential enzymatic activity of the polypeptide of the present invention conferring an increase of the respective fine chemical i.e. whose activity is essentially not reduced, are polypeptides with at least 10% or 20%, by preference 30% or 40%, especially preferably 50% or 60%, very especially preferably 80% or 90 or more of the wild type biological activity or enzyme activity, advantageously, the activity is essentially not reduced in comparison with the activity of a polypeptide shown in table II, lines 231 to 234 and/or 235 to 242 and/or 599 to 602 , columns 5 or 7, expressed under identical conditions.
In one embodiment, the polypeptide of the invention is a homolog consisting of or comprising the sequence as indicated in, preferably Table II B, column 7, lines 231 to 234 and/or 235 to 242 and/or 599 to 602, resp.,.

**[0189.0.18.18]** Homologues of nucleic acid sequences shown in table I, lines 231 to 234 and/or 235 to 242 and/or 599 to 602 , columns 5 or 7 or of the derived sequences shown in table II, lines 231 to 234 and/or 235 to 242 and/or 599 to 602 , columns 5 or 7 also mean truncated sequences, cDNA, single-stranded DNA or RNA of the coding and noncoding DNA sequence. Homologues of said sequences are also understood as meaning derivatives, which comprise noncoding regions such as, for example, UTRs, terminators, enhancers or promoter variants. The promoters upstream of the nucleotide sequences stated can be modified by one or more nucleotide substitution(s), insertion(s) and/or deletion(s) without, however, interfering with the functionality or activity either of the promoters, the open reading frame (= ORF) or with the 3'-regulatory region such as terminators or other 3'regulatory regions, which are far away from the ORF. It is furthermore possible that the activity of the promoters is increased by modification of their sequence, or that they are replaced completely by more active promoters, even promoters from heterologous organisms. Appropriate promoters are known to the person skilled in the art and are mentioned herein below.

**[0190.0.0.18] to [0191.0.0.18] see [0190.0.0.0] to [0191.0.0.0]**

**[0191.1.18.18]** In one embodiment, the organisms or a part thereof provides according to the herein mentioned process of the invention an increased level of the fine chemical bound to any wax, triglycerides, lipid, oil and/or fat or any composition thereof containing any bound or free Coenzyme Q10 and/or Coenzyme Q9, for example bound to lipids, lipoproteins, membrane fractions, sphingolipids, phosphoglycerides, lipids, glycolipids such as glycosphingolipids, phospholipids such as phosphatidylethanolamine, phosphatidylcholine, phosphatidylserine, phosphatidylglycerol, phosphatidylinositol or diphosphatidylglycerol, or as monoacylglyceride, diacylglyceride or triacylglyceride, compared to said control or selected organisms or parts thereof.

**[0192.0.0.18] to [0203.0.0.18] see [0192.0.0.0] to [0203.0.0.0]**

**[0204.0.18.18]** Accordingly, in one embodiment, the invention relates to a nucleic acid molecule, which comprises a nucleic acid molecule selected from the group consisting of:
a) nucleic acid molecule encoding, preferably at least the mature form, of a polypeptide shown in table II, lines 231 to 234 and/or 235 to 242 and/or 599 to 602 , columns 5 or 7; resp., preferably Table II B, column 7, lines 231 to 234 and/or 235 to 242 and/or 599 to 602, resp., or a fragment thereof conferring an increase in the amount of the respective fine chemical in an organism or a part thereof
b) nucleic acid molecule comprising, preferably at least the mature form, of a nucleic acid molecule shown in table I, lines 231 to 234 and/or 235 to 242 and/or 599 to 602 , columns 5 or 7, resp., preferably Table I B, column 7, lines 231 to 234 and/or 235 to 242 and/or 599 to 602, resp., or a fragment thereof conferring an increase in the amount of the respective fine chemical in an organism or a part thereof;
c) nucleic acid molecule whose sequence can be deduced from a polypeptide sequence encoded by a nucleic acid molecule of (a) or (b) as result of the degeneracy of the genetic code and conferring an increase in the amount of the respective fine chemical in an organism or a part thereof;
d) nucleic acid molecule encoding a polypeptide whose sequence has at least 50% identity with the amino acid sequence of the polypeptide encoded by the nucleic acid molecule of (a) to (c) and conferring an increase in the amount of the respective fine chemical in an organism or a part thereof;
e) nucleic acid molecule which hybridizes with a nucleic acid molecule of (a) to (c) under stringent hybridisation conditions and conferring an increase in the amount of the respective fine chemical in an organism or a part thereof;
f) nucleic acid molecule encoding a polypeptide, the polypeptide being derived by substituting, deleting and/or adding one or more amino acids of the amino acid sequence of the polypeptide encoded by the nucleic acid molecules (a) to (d), preferably to (a) to (c), and conferring an increase in the amount of the respective fine chemical in an organism or a part thereof;
g) nucleic acid molecule encoding a fragment or an epitope of a polypeptide which is encoded by one of the nucleic acid molecules of (a) to (e), preferably to (a) to (c) and conferring an increase in the amount of the respective fine chemical in an organism or a part thereof ;
h) nucleic acid molecule comprising a nucleic acid molecule which is obtained by amplifying a cDNA library or a genomic library using the primers or primer pairs indicated in table III, lines 231 to 234 and/or 235 to 242 and/or 599 to 602 , column 7 and conferring an increase in the amount of the respective fine chemical in an organism or a part thereof;
i) nucleic acid molecule encoding a polypeptide which is isolated, e.g. from a expression library, with the aid of monoclonal antibodies against a polypeptide encoded by one of the nucleic acid molecules of (a) to (g), preferably to (a) to (c) and conferring an increase in the amount of the respective fine chemical in an organism or a part thereof;
j) nucleic acid molecule which encodes a polypeptide comprising a consensus sequence shown in table IV, lines 231 to 234 and/or 235 to 242 and/or 599 to 602 , column 7 and conferring an increase in the amount of the respective fine chemical in an organism or a part thereof;
k) nucleic acid molecule encoding the amino acid sequence of a polypeptide encoding a domain of the polypeptide shown in table II, lines 231 to 234 and/or 235 to 242 and/or 599 to 602 , columns 5 or 7, resp., preferably Table II B, column 7, lines 231 to 234 and/or 235 to 242 and/or 599 to 602, resp., and conferring an increase in the amount of the respective fine chemical in an organism or a part thereof; and
l) nucleic acid molecule which is obtainable by screening a suitable nucleic acid library under stringent hybridization conditions with a probe comprising one of the sequences of the nucleic acid molecule of (a) to (k) or with a fragment of at least 15 nt, preferably 20 nt, 30 nt, 50 nt, 100 nt, 200 nt or 500 nt of the nucleic acid molecule characterized in (a) to (h) or of the nucleic acid molecule shown in table I, lines 231 to 234 and/or 235 to 242 and/or 599 to 602 , columns 5 or 7 or a nucleic acid molecule encoding, preferably at least the mature form of, a polypeptide shown in table II, lines 231 to 234 and/or 235 to 242 and/or 599 to 602 , columns 5 or 7, and conferring an increase in the amount of the respective fine chemical in an organism or a part thereof; or which encompasses a sequence which is complementary thereto;
whereby, preferably, the nucleic acid molecule according to (a) to (I) distinguishes over a sequence depicted in table I A, lines 231 to 234 and/or 235 to 242 and/or 599 to 602 , columns 5 or 7 by one or more nucleotides. In one embodiment, the nucleic acid molecule of the invention or used in the method of the invention does not consist of a sequence shown in table I A, lines 231 to 234 and/or 235 to 242 and/or 599 to 602 , columns 5 or 7. In an other embodiment, the nucleic acid molecule of the present invention is at least 30 % identical and less than 100%, 99,999%, 99,99%, 99,9% or 99% identical to a sequence shown in table I A, lines 231 to 234 and/or 235 to 242 and/or 599 to 602 , columns 5 or 7. In a further embodiment the nucleic acid molecule does not encode a polypeptide sequence shown in table II A, lines 231 to 234 and/or 235 to 242 and/or 599 to 602 , columns 5 or 7. Accordingly, in one embodiment, the nucleic acid molecule of the present invention encodes in one embodiment a polypeptide which differs at least in one or more amino acids from a polypeptide shown in table II A, lines 231 to 234 and/or 235 to 242 and/or 599 to 602 , columns 5 or 7 but does not encode a protein of a sequence shown in table II A, lines 231 to 234 and/or 235 to 242 and/or 599 to 602 , columns 5 or 7. Accordingly, in one embodiment, the protein encoded by a sequence of a nucleic acid according to (a) to (I) does not consist of a sequence shown in table II A, lines 231 to 234 and/or 235 to 242 and/or 599 to 602 , columns 5 or 7. In a further embodiment, the protein of the present invention is at least 30 % identical to protein sequence depicted in table II A, lines 231 to 234 and/or 235 to 242 and/or 599 to 602 , columns 5 or 7 and less than 100%, preferably less than 99,999%, 99,99% or 99,9%, more preferably less than 99%, 985, 97%, 96% or 95% identical to a sequence shown in table II A, lines 231 to 234 and/or 235 to 242 and/or 599 to 602 , columns 5 or 7.
whereby, in a further embodimen, the nucleic acid molecule according to (a) to (I) distinguishes over a sequence depicted in table I B, lines 231 to 234 and/or 235 to 242 and/or 599 to 602 , columns 5 or 7 by one or more nucleotides. In one embodiment, the nucleic acid molecule of the invention or used in the method of the invention does not consist of a sequence shown in table I B, lines 231 to 234 and/or 235 to 242 and/or 599 to 602 , columns 5 or 7. In an other embodiment, the nucleic acid molecule of the present invention is at least 30 % identical and less than 100%, 99,999%, 99,99%, 99,9% or 99% identical to a sequence shown in table I b, lines 231 to 234 and/or 235 to 242 and/or 599 to 602 , columns 5 or 7. In a further embodiment the nucleic acid molecule does not encode a polypeptide sequence shown in table II B, lines 231 to 234 and/or 235 to 242 and/or 599 to 602 , columns 5 or 7. Accordingly, in one embodiment, the nucleic acid molecule of the present invention encodes in one embodiment a polypeptide which differs at least in one or more amino acids from a polypeptide shown in table II A, lines 231 to 234 and/or 235 to 242 and/or 599 to 602 , columns 5 or 7 but does not encode a protein of a sequence shown in table II B, lines 231 to 234 and/or 235 to 242 and/or 599 to 602 , columns 5 or 7. Accordingly, in one embodiment, the protein encoded by a sequence of a nucleic acid according to (a) to (I) does not consist of a sequence shown in table II B, lines 231 to 234 and/or 235 to 242 and/or 599 to 602 , columns 5 or 7. In a further embodiment, the protein of the present invention is at least 30 % identical to protein sequence depicted in table II B, lines 231 to 234 and/or 235 to 242 and/or 599 to 602 , columns 5 or 7 and less than 100%, preferably less than 99,999%, 99,99% or 99,9%, more preferably less than 99%, 985, 97%, 96% or 95% identical to a sequence shown in table II B, lines 231 to 234 and/or 235 to 242 and/or 599 to 602 , columns 5 or 7.

**[0205.0.0.18] to [0226.0.0.18] see [0205.0.0.0] to [0226.0.0.0]**

**[0227.0.18.18]** The abovementioned nucleic acid molecules can be cloned into the nucleic acid constructs or vectors according to the invention in combination together with further genes, or else different genes are introduced by transforming several nucleic acid constructs or vectors (including plasmids) into a host cell, advantageously into a plant cell or a microorganisms.
In addition to a sequence mentioned in table I, lines 231 to 234 and/or 235 to 242 and/or 599 to 602, resp., preferably Table I B, column 7, lines 231 to 234 and/or 235 to 242 and/or 599 to 602, resp.,, columns 5 or 7 or its derivatives, it is advantageous additionally to express and/or mutate further genes in the organisms. Especially advantageously, additionally at least one further gene of the isoprenoid biosynthetic pathway such as for acetyl CoA, HMG-CoA, Mevalonate, Isopentyl pyrophosphate, Geranyl pyrophosphate, Farnesyl pyrophosphate e.g. HMG-CoA Reductase, Mevalonate, Kinase, etc., is expressed in the organisms such as plants or microorganisms. It is also possible that the regulation of the natural genes has been modified advantageously so that the gene and/or its gene product is no longer subject to the regulatory mechanisms which exist in the organisms. This leads to an increased synthesis of the isoprenoids, coenzyme precursor or coenzymes, preferably Q9 and/or Q10, as desired since, for example, feedback regulations no longer exist to the same extent or not at all. In addition it might be advantageously to combine one or more sequences shown in table I, lines 231 to 234 and/or 235 to 242 and/or 599 to 602 , columns 5 or 7 with genes which generally support or enhances to growth or yield of the target organism, for example genes which lead to faster growth rate of microorganisms or genes which produces stress-, pathogen, or herbicide resistant plants.

**[0227.1.18.18]** In addition to the sequence mentioned in table I, lines 231 to 234 and/or 235 to 242 and/or 599 to 602 , columns 5 or 7 or its derivatives, it is advantageous additionally to knock out and/or mutate further genes in the organisms. E.g. it may be advantageous, if one or more genes of the catabolic pathway for isoprenoids or quinones are reduced, deleted or in another way knocked out in the organisms such as plants or microorganisms.

**[0228.0.18.18]** In a further embodiment of the process of the invention, therefore, organisms are grown, in which there is simultaneous overexpression of at least one nucleic acid or one of the genes which code for proteins involved in the isoprenoid metabolism, in particular in Coenzyme Q9 or Coenzyme Q10 synthesis.

**[0229.0.18.18]** ./.

**[0230.1.0.18]** ./.

**[0230.2.0.18]** ./.

**[0230.0.0.18] see [0230.0.0.0]**

**[0231.0.18.18]** In a further advantageous embodiment of the process of the invention, the organisms used in the process are those in which simultaneously a Coenzyme Q10 and/or Coenzyme Q9 degrading protein is attenuated, in particular by reducing the rate of expression of the corresponding gene.

**[0232.0.0.18] to [0276.0.0.18] see [0232.0.0.0] to [0276.0.0.0]**

**[0277.0.18.18]** The coenzymes produced can be isolated from the organism by methods with which the skilled worker is familiar, for example, via extraction, salt precipitation and/or different chromatography methods, e.g. as mentioned above. The process according to the invention can be conducted batchwise, semibatchwise or continuously. The respective fine chemical produced in the process according to the invention can be isolated as mentioned above from the organisms, advantageously plants, in the form of their waxes , membrane fractions, oils, fats, lipids and/or fatty acids. Fractions comprising the coenzymes produced by this process can be obtained by harvesting the organisms, either from the crop in which they grow, or from the field. This can be done via pressing or extraction of the plant parts, preferably the plant seeds.

**[0278.0.0.18] to [0283.0.0.18] see [0278.0.0.0] to [0283.0.0.0]**

**[0283.0.18.18]** Moreover, a native polypeptide conferring the increase of the respective fine chemical in an organism or part thereof can be isolated from cells (e.g., endothelial cells), for example using the antibody of the present invention as described below, in particular, an anti-YPR138C, YBR184W, b2699 and/or b1829, and/or YPR172W, YER174C, YER156C, YDR513W, b2426, b2703, b2729, b3644, b3605, b2699, b0730, and/or b0175 protein antibody or an antibody against a polypeptide as shown in table II, lines 231 to 234 and/or 235 to 242 and/or 599 to 602 , columns 5 or 7 or a antigenic part thereof, which can be produced by standard techniques utilizing the polypeptid of the present invention or characterized in the process of the present invention or fragment thereof, i.e., the polypeptide of this invention. Preferred are monoclonal antibodies specifically binding to a polypeptide as indicated in Table II, columns 5 or 7, lines 231 to 234 and/or 235 to 242 and/or 599 to 602 .

**[0284.0.0.18] see [0284.0.0.0]**

**[0285.0.18.18]** In one embodiment, the present invention relates to a polypeptide having a sequence shown in table II, lines 231 to 234 and/or 235 to 242 and/or 599 to 602 , columns 5 or 7 or as coded by a nucleic acid molecule shown in table I, lines 231 to 234 and/or 235 to 242 and/or 599 to 602 , columns 5 or 7 or functional homologues thereof.

**[0286.0.18.18]** In one advantageous embodiment, in the method of the present invention the activity of a polypeptide is increased comprising or consisting of a consensus sequence shown in table IV, lines 231 to 234 and/or 235 to 242 and/or 599 to 602 , column 7 and in one another embodiment, the present invention relates to a polypeptide comprising or consisting of a consensus sequence shown in table IV, lines 231 to 234 and/or 235 to 242 and/or 599 to 602 , column 7 whereby 20 or less, preferably 15 or 10, preferably 9, 8, 7, or 6, more preferred 5 or 4, even more preferred 3, even more preferred 2, even more preferred 1, most preferred 0 of the amino acids positions indicated can be replaced by any amino acid or, in an further embodiment, can be replaced and/or absent. In one embodiment, the present invention relates to the method of the present invention comprising a polypeptide or to a polypeptide comprising more than one consensus sequences (of an individual line) as indicated in Table IV, column 7, lines 231 to 234 and/or 235 to 242 and/or 599 to 602, resp.

**[0287.0.0.18] to [0289.0.0.18] see [0287.0.0.0] to [0289.0.0.0]**

**[0290.0.0.18] see [0290.0.0.0]**

**[0291.0.18.18]** In one advantageous embodiment, the method of the present invention comprises the increasing of a polypeptide comprising or consisting of plant or microorganism specific consensus sequences.
In one embodiment, said polypeptide of the invention or used in the method of the invention distinguishes over a sequence shown in table II A or II B, lines 231 to 234 and/or 235 to 242 and/or 599 to 602 , columns 5 or 7 by one or more amino acids. In one embodiment, polypeptide distinguishes form a sequence shown in table II A or II B, lines 231 to 234 and/or 235 to 242 and/or 599 to 602 , columns 5 or 7 by more than 5, 6, 7, 8 or 9 amino acids, preferably by more than 10, 15, 20, 25 or 30 amino acids, even more preferred are more than 40, 50, or 60 amino acids and, preferably, the sequence of the polypeptide of the invention distinguishes from a sequence shown in table II A or II B, lines 231 to 234 and/or 235 to 242 and/or 599 to 602 , columns 5 or 7 by not more than 80% or 70% of the amino acids, preferably not more than 60% or 50%, more preferred not more than 40% or 30%, even more preferred not more than 20% or 10%. In an other embodiment, said polypeptide of the invention does not consist of a sequence shown in table II A or II B, lines 231 to 234 and/or 235 to 242 and/or 599 to 602 , columns 5 or 7.

**[0292.0.0.18] see [0292.0.0.0]**

**[0293.0.18.18]** In one embodiment, the invention relates to polypeptide conferring an increase in the respective fine chemical in an organism or part being encoded by the nucleic acid molecule of the invention or used in the process of the invention and having a sequence which distinguishes from the sequence as shown in table II A or II B, lines 231 to 234 and/or 235 to 242 and/or 599 to 602 , columns 5 or 7 by one or more amino acids. In another embodiment, said polypeptide of the invention does not consist of the sequence shown in table II A or II B, lines 231 to 234 and/or 235 to 242 and/or 599 to 602 , columns 5 or 7. In a further embodiment, said polypeptide of the present invention is less than 100%, 99,999%, 99,99%, 99,9% or 99% identical. In one embodiment, said polypeptide does not consist of the sequence encoded by the nucleic acid molecules shown in table I, lines 231 to 234 and/or 235 to 242 and/or 599 to 602 , columns 5 or 7.

**[0294.0.18.18]** In one embodiment, the present invention relates to a polypeptide having YPR138C, YBR184W, b2699 and/or b1829, and/or YPR172W, YER174C, YER156C, YDR513W, b2426, b2703, b2729, b3644, b3605, b2699, b0730, and/or b0175 protein activity, which distinguishes over the sequence depicted in table II A or II B, lines 231 to 234 and/or 235 to 242 and/or 599 to 602 , columns 5 or 7 by one or more amino acids, preferably by more than 5, 6, 7, 8 or 9 amino acids, preferably by more than 10, 15, 20, 25 or 30 amino acids, even more preferred are more than 40, 50, or 60 amino acids but even more preferred by less than 70% of the amino acids, more preferred by less than 50%, even more preferred my less than 30% or 25%, more preferred are 20% or 15%, even more preferred are less than 10%.

**[0295.0.0.18] to [0296.0.0.18] see [0295.0.0.0] to [0296.0.0.0]**

**[0297.0.18.18]** The language "substantially free of cellular material" includes preparations of the polypeptide of the invention or used in the method of the invention in which the protein is separated from cellular components of the cells in which it is naturally or recombinantly produced. In one embodiment, the language "substantially free of cellular material" includes preparations having less than about 30% (by dry weight) of "contaminating protein", more preferably less than about 20% of "contaminating protein", still more preferably less than about 10% of "contaminating protein", and most preferably less than about 5% "contaminating protein". The term "Contaminating protein" relates to polypeptides, which are not polypeptides of the present invention. When the polypeptide of the present invention or biologically active portion thereof is recombinantly produced, it is also preferably substantially free of culture medium, i.e., culture medium represents less than about 20%, more preferably less than about 10%, and most preferably less than about 5% of the volume of the protein preparation. The language "substantially free of chemical precursors or other chemicals" includes preparations in which the polypeptide of the present invention is separated from chemical precursors or other chemicals, which are involved in the synthesis of the protein. The language "substantially free of chemical precursors or other chemicals" includes preparations having less than about 30% (by dry weight) of chemical precursors of a protein as indicated in Table II, column 5, lines 231 to 234 and/or 235 to 242 and/or 599 to 602 or being encoded by a nucleic acid molecule indicated in Table I, column 5, lines 231 to 234 and/or 235 to 242 and/or 599 to 602 or of its homologs, e.g. as indicated in Table II, column 7, lines 231 to 234 and/or 235 to 242 and/or 599 to 602 , or of non-YPR138C, YBR184W, b2699 and/or b1829, and/or YPR172W, YER174C, YER156C, YDR513W, b2426, b2703, b2729, b3644, b3605, b2699, b0730, and/or b0175 chemicals, more preferably less than about 20% chemical precursors of a protein as indicated in Table II, column 5, lines 231 to 234 and/or 235 to 242 and/or 599 to 602 or being encoded by a nucleic acid molecule indicated in Table I, column 5, lines 231 to 234 and/or 235 to 242 and/or 599 to 602 or of its homologs, e.g. as indicated in Table II, column 7, lines 231 to 234 and/or 235 to 242 and/or 599 to 602 , or of non-YPR138C, YBR184W, b2699 and/or b1829, and/or YPR172W, YER174C, YER156C, YDR513W, b3605, b2699 b0730 and/or b0175 chemicals, still more preferably less than about 10% chemical precursors of a protein as indicated in Table II, column 5, lines 231 to 234 and/or 235 to 242 and/or 599 to 602 or being encoded by a nucleic acid molecule indicated in Table I, column 5, lines 231 to 234 and/or 235 to 242 and/or 599 to 602 or of its homologs, e.g. as indicated in Table II, column 7, lines 231 to 234 and/or 235 to 242 and/or 599 to 602 , or of non-YPR138C, YBR184W, b2699 and/or b1829, and/or YPR172W, YER174C, YER156C, YDR513W, b2426, b2703, b2729, b3644, b3605, b2699, b0730, and/or b0175 chemicals, and most preferably less than about 5% chemical precursors of a protein as indicated in Table II, column 5, lines 231 to 234 and/or 235 to 242 and/or 599 to 602 or being encoded by a nucleic acid molecule indicated in Table I, column 5, lines 231 to 234 and/or 235 to 242 and/or 599 to 602 or of its homologs, e.g. as indicated in Table II, column 7, lines 231 to 234 and/or 235 to 242 and/or 599 to 602 , or of non-YPR138C, YBR184W, b2699, b1829, and/or YPR172W, YER174C, YER156C, YDR513W,b2426, b2703, b2729, b3644, b3605, b2699, b0730, b0175 chemicals. In preferred embodiments, isolated proteins or biologically active portions thereof lack contaminating proteins from the same organism from which the polypeptide of the present invention is derived. Typically, such proteins are produced by recombinant techniques.

**[0298.0.18.18]** A polypeptide of the invention or used in the method of the invention can participate in the process of the present invention. The polypeptide or a portion thereof comprises preferably an amino acid sequence which is sufficiently homologous to an amino acid sequence shown in table II, lines 231 to 234 and/or 235 to 242 and/or 599 to 602 , columns 5 or 7.

**[0299.0.18.18]** Further, the polypeptide can have an amino acid sequence which is encoded by a nucleotide sequence which hybridizes, preferably hybridizes under stringent conditions as described above, to a nucleotide sequence of the nucleic acid molecule of the present invention. Accordingly, the polypeptide has an amino acid sequence which is encoded by a nucleotide sequence that is at least about 35%, 40%, 45%, 50%, 55%, 60%, 65% or 70%, preferably at least about 75%, 80%, 85% or 90, and more preferably at least about 91%, 92%, 93%, 94% or 95%, and even more preferably at least about 96%, 97%, 98%, 99% or more homologous to one of the amino acid sequences as shown in table II A or II B, lines 231 to 234 and/or 235 to 242 and/or 599 to 602 , columns 5 or 7. The preferred polypeptide of the present invention preferably possesses at least one of the activities according to the invention and described herein. A preferred polypeptide of the present invention includes an amino acid sequence encoded by a nucleotide sequence which hybridizes, preferably hybridizes under stringent conditions, to a nucleotide sequence of table I, lines 231 to 234 and/or 235 to 242 and/or 599 to 602 , columns 5 or 7 or which is homologous thereto, as defined above.

**[0300.0.18.18]** Accordingly the polypeptide of the present invention can vary from the sequences shown in table II, lines 231 to 234 and/or 235 to 242 and/or 599 to 602 , columns 5 or 7 in amino acid sequence due to natural variation or mutagenesis, as described in detail herein. Accordingly, the polypeptide comprise an amino acid sequence which is at least about 35%, 40%, 45%, 50%, 55%, 60%, 65% or 70%, preferably at least about 75%, 80%, 85% or 90, and more preferably at least about 91%, 92%, 93%, 94% or 95%, and most preferably at least about 96%, 97%, 98%, 99% or more homologous to an entire amino acid sequence shown in table II, lines 231 to 234 and/or 235 to 242 and/or 599 to 602 , columns 5 or 7.

**[0301.0.0.18] see [0301.0.0.0]**

**[0302.0.18.18]** Biologically active portions of an polypeptide of the present invention include peptides comprising amino acid sequences derived from the amino acid sequence of the polypeptide of the present invention or used in the process of the present invention, e.g., the amino acid sequence shown in table II, lines 231 to 234 and/or 235 to 242 and/or 599 to 602 , columns 5 or 7 or the amino acid sequence of a protein homologous thereto, which include fewer amino acids than a full length polypeptide of the present invention or used in the process of the present invention or the full length protein which is homologous to an polypeptide of the present invention or used in the process of the present invention depicted herein, and exhibit at least one activity of polypeptide of the present invention or used in the process of the present invention.

**[0303.0.0.18] see [0303.0.0.0]**

**[0304.0.18.18]** Manipulation of the nucleic acid molecule of the invention or used in the method of the invention may result in the production of protein indicated in Table II, column 5, lines 231 to 234 and/or 235 to 242 and/or 599 to 602 or being encoded by a nucleic acid molecule indicated in Table I, column 5, lines 231 to 234 and/or 235 to 242 and/or 599 to 602 or of its homologs, e.g. as indicated in Table II, column 7, lines 231 to 234 and/or 235 to 242 and/or 599 to 602, having differences from the wild-type protein. These proteins may be improved in efficiency or activity, may be present in greater numbers in the cell than is usual, or may be decreased in efficiency or activity in relation to the wild type protein.

**[0305.0.18.18]** Any mutagenesis strategies for the polypeptide of the present invention or the polypeptide used in the process of the present invention to result in increasing said activity are not meant to be limiting; variations on these strategies will be readily apparent to one skilled in the art. Using such strategies, and incorporating the mechanisms disclosed herein, the nucleic acid molecule and polypeptide of the invention or used in the method of the invention may be utilized to generate plants or parts thereof, expressing wildtype YPR138C, YBR184W, b2699 and/or b1829, and/or YPR172W, YER174C, YER156C, YDR513W, b2426, b2703, b2729, b3644, b3605, b2699, b0730, and/or b0175 proteins, e.g. of a protein as indicated in Table II, column 5, lines 231 to 234 and/or 235 to 242 and/or 599 to 602 or being encoded by a nucleic acid molecule indicated in Table I, column 5, lines 231 to 234 and/or 235 to 242 and/or 599 to 602 or of its homologs, e.g. as indicated in Table II, column 7, lines 231 to 234 and/or 235 to 242 and/or 599 to 602 , or a mutant of a protein as indicated in Table II, column 5or 7, lines 231 to 234 and/or 235 to 242 and/or 599 to 602 or being encoded by a nucleic acid molecule indicated in Table I, column 5, lines 231 to 234 and/or 235 to 242 and/or 599 to 602 or of its homologs, e.g. as indicated in Table II, column 7, lines 231 to 234 and/or 235 to 242 and/or 599 to 602 , e.g. a mutated YPR138C, YBR184W, b2699 and/or b1829, and/or YPR172W, YER174C, YER156C, YDR513W, b2426, b2703, b2729, b3644, b3605, b2699, b0730, and/or b0175 protein, and encoding nucleic acid molecules and polypeptide molecules of the invention or used in the method of the invention such that the yield, production, and/or efficiency of production of a desired compound is improved.

**[0306.0.0.18] to [0308.0.0.18] see [0306.0.0.0] to [0308.0.0.0]**

**[0309.0.18.18]** In one embodiment, an "YPR138C, YBR184W, b2699 and/or b1829, and/or YPR172W, YER174C, YER156C, YDR513W, b2426, b2703, b2729, b3644, b3605, b2699, b0730, and/or b0175 protein (= polypeptide)" refers to a polypeptide having an amino acid sequence corresponding to the polypeptide of the invention or used in the process of the invention , whereas a "non-YPR138C, YBR184W, b2699 and/or b1829, and/or YPR172W, YER174C, YER156C, YDR513W, b2426, b2703, b2729, b3644, b3605, b2699, b0730, and/or b0175 polypeptide" or "other polypeptide" refers to a polypeptide having an amino acid sequence corresponding to a protein which is not substantially homologous a polypeptide of the invention, preferably which is not substantially homologous to a polypeptide having an YPR138C, YBR184W, b2699 and/or b1829, and/or YPR172W, YER174C, YER156C, YDR513W, b2426, b2703, b2729, b3644, b3605, b2699, b0730, and/or b0175 protein activity, e.g., a protein which does not confer the activity described herein and which is derived from the same or a different organism. In one embodiment, a "non-polypeptide of the invention" or "other polypeptide" not being indicated in Table II, columns 5 or 7, lines 231 to 234 and/or 235 to 242 and/or 599 to 602, resp., does not confer an increase of the respective fine chemical in an organism or part thereof.

**[0310.0.0.18] to [0317.0.0.18] see [0310.0.0.0] to [0317.0.0.0]**

**[0318.0.18.18]** In an especially preferred embodiment, the polypeptide according to the invention furthermore also does not have the sequences of those proteins which are encoded by a sequences shown in table II, lines 231 to 234 and/or 235 to 242 and/or 599 to 602 , columns 5 or 7.

**[0319.0.0.18] to [0334.0.0.18] see [0319.0.0.0] to [0334.0.0.0]**

**[0335.0.18.18]** The dsRNAi method has proved to be particularly effective and advantageous for reducing the expression of the nucleic acid sequences of the table I, lines 231 to 234 and/or 235 to 242 and/or 599 to 602 , columns 5 or 7 and/or homologs thereof. As described inter alia in WO 99/32619, dsRNAi approaches are clearly superior to traditional antisense approaches. The invention therefore furthermore relates to double-stranded RNA molecules (dsRNA molecules) which, when introduced into an organism, advantageously into a plant (or a cell, tissue, organ or seed derived there from), bring about altered metabolic activity by the reduction in the expression of a nucleic acid sequences of the table I, lines 231 to 234 and/or 235 to 242 and/or 599 to 602 , columns 5 or 7 and/or homologs thereof. In a double-stranded RNA molecule for reducing the expression of an protein encoded by a nucleic acid sequence of one of the table I, lines 231 to 234 and/or 235 to 242 and/or 599 to 602 , columns 5 or 7 and/or homologs thereof, one of the two RNA strands is essentially identical to at least part of a nucleic acid sequence, and the respective other RNA strand is essentially identical to at least part of the complementary strand of a nucleic acid sequence.

**[0336.0.0.18] to [0342.0.0.18] see [0336.0.0.0] to [0342.0.0.0]**

**[0343.0.18.18]** As has already been described, 100 % sequence identity between the dsRNA and a gene transcript of a nucleic acid sequence of one of the sequences shown in table I, lines 231 to 234 and/or 235 to 242 and/or 599 to 602 , columns 5 or 7 or it's homolog is not necessarily required in order to bring about effective reduction in the expression. The advantage is, accordingly, that the method is tolerant with regard to sequence deviations as may be present as a consequence of genetic mutations, polymorphisms or evolutionary divergences. Thus, for example, using the dsRNA, which has been generated starting from a sequence of one of sequences shown in table I, lines 231 to 234 and/or 235 to 242 and/or 599 to 602 , columns 5 or 7 or homologs thereof of the one organism, may be used to suppress the corresponding expression in another organism.

**[0344.0.0.18] to [0361.0.0.18] see [0344.0.0.0] to [0361.0.0.0]**

**[0362.0.18.18]** Accordingly the present invention relates to any cell transgenic for any nucleic acid characterized as part of the invention, e.g. conferring the increase of the respective fine chemical in a cell or an organism or a part thereof, e.g. the nucleic acid molecule of the invention or used in the method of the invention, the nucleic acid construct of the invention, the antisense molecule of the invention, the vector of the invention or a nucleic acid molecule encoding the polypeptide of the invention, e.g. encoding a polypeptide having an YPR138C, YBR184W, b2699 and/or b1829, and/or YPR172W, YER174C, YER156C, YDR513W, b2426, b2703, b2729, b3644, b3605, b2699, b0730, and/or b0175 protein activity or of a protein as indicated in Table II, column 5, lines 231 to 234 and/or 235 to 242 and/or 599 to 602 or being encoded by a nucleic acid molecule indicated in Table I, column 5, lines 231 to 234 and/or 235 to 242 and/or 599 to 602 or of its homologs, e.g. as indicated in Table II, column 7, lines 231 to 234 and/or 235 to 242 and/or 599 to 602 . Due to the above mentioned activity the respective fine chemical content in a cell or an organism is increased. For example, due to modulation or manipulation, the cellular activity is increased, e.g. due to an increased expression or specific activity of the subject matters of the invention in a cell or an organism or a part thereof. Transgenic for a polypeptide having an YPR138C, YBR184W, b2699 and/or b1829, and/or YPR172W, YER174C, YER156C, YDR513W, b2426, b2703, b2729, b3644, b3605, b2699, b0730, and/or b0175 protein activity e.g. for a protein as indicated in Table II, column 5, lines 231 to 234 and/or 235 to 242 and/or 599 to 602 or being encoded by a nucleic acid molecule indicated in Table I, column 5, lines 231 to 234 and/or 235 to 242 and/or 599 to 602 or of its homologs, e.g. as indicated in Table II, column 7, lines 231 to 234 and/or 235 to 242 and/or 599 to 602 , means herein that due to modulation or manipulation of the genome, the activity of YPR138C, YBR184W, b2699 and/or b1829, and/or YPR172W, YER174C, YER156C, YDR513W, b2426, b2703, b2729, b3644, b3605, b2699, b0730, and/or b0175 or a YPR138C, YBR184W, b2699 and/or b1829, and/or YPR172W, YER174C, YER156C, YDR513W, b2426, b2703, b2729, b3644, b3605, b2699, b0730, and/or b0175-like activity is increased in the cell or organism or part thereof, e.g. of a protein as indicated in Table II, column 5, lines 231 to 234 and/or 235 to 242 and/or 599 to 602 or being encoded by a nucleic acid molecule indicated in Table I, column 5, lines 231 to 234 and/or 235 to 242 and/or 599 to 602 or of its homologs, e.g. as indicated in Table II, column 7, lines 231 to 234 and/or 235 to 242 and/or 599 to 602 . Examples are described above in context with the process of the invention.

**[0363.0.0.18] see [0363.0.0.0]**

**[0364.0.18.18]** A naturally occurring expression cassette - for example the naturally occurring combination of the YPR138C, YBR184W, b2699 and/or B1829, and/or YPR172W, YER174C, YER156C, YDR513W, b2426, b2703, b2729, b3644, b3605, b2699, b0730, and/or b0175 protein promoter with the corresponding YPR138C, YBR184W, b2699 and or b1829, and/or YPR172W, YER174C, YER156C, YDR513W, b2426, b2703, b2729, b3644, b3605, b2699, b0730, and/or b0175 protein gene. e.g. the combination of the promoter of a protein as indicated in Table II, column 5, lines 231 to 234 and/or 235 to 242 and/or 599 to 602 or being encoded by a nucleic acid molecule indicated in Table I, column 5, lines 231 to 234 and/or 235 to 242 and/or 599 to 602 or of its homologs, e.g. as indicated in Table II, column 7, lines 231 to 234 and/or 235 to 242 and/or 599 to 602 , with the corresponding gene - becomes a transgenic expression cassette when it is modified by non-natural, synthetic "artificial" methods such as, for example, mutagenization. Such methods have been described (US 5,565,350; WO 00/15815; also see above).

**[0365.0.0.18] to [0376.0.0.18] see [0365.0.0.0] to [0376.0.0.0]**

**[0377.0.18.18]** Accordingly, the present invention relates also to a process according to the present invention whereby the produced coenzymes are isolated to high purity, preferably, to purity of more than 70% w/w or more, more preferred are 80% or more, in particular preferred are 90% w/w or more, even more preferred are 95% w/w or more, e.g. 97% w/w or more, e.g. 98% w/w, 99% w/w, or 99,9% w/w.

**[0378.0.18.18]** In this manner, more than 50% by weight, advantageously more than 60% by weight, preferably more than 70% by weight, especially preferably more than 80% by weight, very especially preferably more than 90% by weight, of the Coenzyme Q9 or Coenzyme Q10 produced in the process can be recovered, purified or isolated. The resulting coenzymes, e.g. compositions comprising the former, can, if appropriate, subsequently be further purified, if desired mixed with other active ingredients such as vitamins, amino acids, carbohydrates, antibiotics and the like, and, if appropriate, formulated.

**[0379.0.18.18]** In one embodiment, said coenzyme is the respective fine chemical, preferably reduced Coenzyme Q9 and/or Coenzyme Q10.

**[0380.0.18.18]** The Coenzyme Q9 or Coenzyme Q10 comprising fraction or the respective fine chemical obtained in the process are suitable as starting material for the synthesis of further products of value. For example, they can be used in combination with each other or alone for the production of pharmaceuticals, foodstuffs, animal feeds or cosmetics. Accordingly, the present invention relates a method for the production of a pharmaceuticals, food stuff, animal feeds, nutrients or cosmetics comprising the steps of the process according to the invention, including the isolation of Coenzyme Q9 or Coenzyme Q10 composition produced or the respective fine chemical produced if desired and formulating the product with a pharmaceutical acceptable carrier or formulating the product in a form acceptable for an application in agriculture. A further embodiment according to the invention is the use of the respective fine chemical produced in the process or of the transgenic organisms in animal feeds, foodstuffs, medicines, food supplements, cosmetics or pharmaceuticals.

**[0381.0.0.18] to [0382.0.0.18] see [0381.0.0.0] to [0382.0.0.0]**

**[0383.0.5.18]** For preparing antioxidant, coenzyme and/or vitamin-containing fine chemicals, in particular the respective fine chemical-comprising fine chemicals, it is possible to use as source organic compounds such as, for example, waxes, oils, fats and/or lipids or a membrane comprising fraction, in particular compositions comprising membranes of mitochondria or of plastids.

**[0384.0.0.18] see [0384.0.0.0]**

**[0385.0.18.18]** The fermentation broths obtained in this way, containing in particular Coenzyme Q10 and/or Coenzyme Q9 in mixtures with other lipids, fats and/or oils and/or with other vitamins or coenzymes or antioxidants normally have a dry matter content of from, 1% to 50% by weight. Depending on requirements, the biomass can be removed entirely or partly by separation methods, such as, for example, centrifugation, filtration, decantation or a combination of these methods, from the fermentation broth or left completely in it. The fermentation broth can then be thickened or concentrated by known methods, such as, for example, with the aid of a rotary evaporator, thin-film evaporator, falling film evaporator, by reverse osmosis or by nanofiltration. This concentrated fermentation broth can then be worked up by freeze-drying, spray drying, spray granulation or by other processes.

**[0386.0.18.18]** However, it is also possible to further purify the fraction or composition comprising the respective fine chemical, e.g. Coenzyme Q9 and/or Coenzyme Q10, as produced. For this purpose, the product-containing composition is subjected for example to a thin layer chromatography on silica gel plates or to a chromatography such as a Florisil column (Bouhours J.F., J. Chromatrogr. 1979, 169, 462), in which case the desired product or the impurities are retained wholly or partly on the chromatography resin. These chromatography steps can be repeated if necessary, using the same or different chromatography resins. The skilled worker is familiar with the choice of suitable chromatography resins and their most effective use. An alternative method to purify the fatty acids is for example crystallization in the presence of urea. These methods can be combined with each other.

**[0387.0.0.18] to [0392.0.0.18] see [0387.0.0.0] to [0392.0.0.0]**

**[0393.0.18.18]** In one embodiment, the present invention relates to a method for the identification of a gene product conferring an increase in the respective fine chemical production in a cell, comprising the following steps:
(i) contacting, e.g. hybridising, the nucleic acid molecules of a sample, e.g. cells, tissues, plants or microorganisms or a nucleic acid library , which can contain a candidate gene encoding a gene product conferring an increase in the respective fine chemical after expression, with the nucleic acid molecule of the present invention;
(j) identifying the nucleic acid molecules, which hybridize under relaxed stringent conditions with the nucleic acid molecule of the present invention in particular to the nucleic acid molecule sequence shown in table I, lines 231 to 234 and/or 235 to 242 and/or 599 to 602 , columns 5 or 7 and, optionally, isolating the full length cDNA clone or complete genomic clone;
(k) introducing the candidate nucleic acid molecules in host cells, preferably in a plant cell or a microorganism, appropriate for producing the respective fine chemical;
(I) expressing the identified nucleic acid molecules in the host cells;
(m) assaying the respective fine chemical level in the host cells; and
(n) identifying the nucleic acid molecule and its gene product which expression confers an increase in the respective fine chemical level in the host cell after expression compared to the wild type.

**[0394.0.0.18] to [0399.0.0.18] see [0394.0.0.0] to [0399.0.0.0]**

**[0399.1.18.18]** One can think to screen for increased fine chemical production by for example resistance to drugs blocking Coenzyme Q10 and/or Coenzyme Q9 synthesis and looking whether this effect is dependent on a protein as indicated in Table II, column 5, lines 231 to 234 and/or 235 to 242 and/or 599 to 602 or being encoded by a nucleic acid molecule indicated in Table I, column 5, lines 231 to 234 and/or 235 to 242 and/or 599 to 602 or of its homologs, e.g. as indicated in Table II, column 7, lines 231 to 234 and/or 235 to 242 and/or 599 to 602 , e.g. by comparing the phenotype of nearly identical organisms with low and high activity of a protein as indicated in Table II, column 5, lines 231 to 234 and/or 235 to 242 and/or 599 to 602 or being encoded by a nucleic acid molecule indicated in Table I, column 5, lines 231 to 234 and/or 235 to 242 and/or 599 to 602 or of its homologs, e.g. as indicated in Table II, column 7, lines 231 to 234 and/or 235 to 242 and/or 599 to 602 .

**[0400.0.0.18] to [0416.0.0.18] see [0400.0.0.0] to [0416.0.0.0]**

**[0417.0.18.18]** The nucleic acid molecule of the invention or used in the method of the invention, the vector of the invention or the nucleic acid construct of the invention may also be useful for the production of organisms resistant to inhibitors of the isoprenoid, in particular the Coenzyme Q9 and/or Coenzyme Q10 production biosynthesis pathways. In particular, the overexpression of the polypeptide of the present invention may protect an organism such as a microorganism or a plant against inhibitors, which block the respective fine chemical synthesis in said organism.
As reduced Coenzyme Q9 and/or Coenzyme Q10 can protect organisms against damages of oxidative stress, a increased level of the respective fine chemical may protect plants against herbicides which cause the toxic build-up of oxidative compounds, e.g. singlet oxygen. Further it is known, that Coenzyme Q9 and/or Coenzyme Q10 stabilize membranes.
Accordingly, in one embodiment, the increase of the level of the respective fine chemical is used to protect plants against herbicides destroying membranes or cells due to the creation of free oxygen radicals.
Examples of inhibitors or herbicides building up oxidative stress are aryl triazion, e.g. sulfentrazone, carfentrazone, or diphenylethers, e.g. acifluorfen, lactofen, or oxyfluorfen, or N-Phenylphthalimide, e.g. flumiclorac or flumioxazin, substituted ureas, e.g. fluometuron, tebuthiuron, or diuron, linuron, or triazines, e.g. atrazine, prometryn, ametryn, metributzin, prometon, simazine, or hexazinone, or uracils, e.g. bromacil or terbacil.
Inhibitors of the isoprenoids pathway may also lead to an toxic decrease of Coenzyme Q9 and/or Coenzyme Q10 in cells or membranes, e.g. as Clomazone. Thus, in one embodiment, the present invention relates to the use of an increase of the respective fine chemical according to the present invention for the protection of plants against carotenoids inhibitors as pyridines and pyridazinones.

**[0418.0.0.18] to [0423.0.0.18] see [0418.0.0.0] to [0423.0.0.0]**

**[0424.0.18.18]** Accordingly, the nucleic acid of the invention or used in the method of the invention, the polypeptide of the invention or used in the method of the invention, the nucleic acid construct of the invention, the organisms, the host cell, the microorganisms, the plant, plant tissue, plant cell, or the part thereof of the invention, the vector of the invention, the agonist identified with the method of the invention, the nucleic acid molecule identified with the method of the present invention, can be used for the production of the respective fine chemical or of the respective fine chemical and one or more other antioxidants, coenzymes or vitamins, in particular vitamin E, or vitamin B6. Accordingly, the nucleic acid of the invention or used in the method of the invention, or the nucleic acid molecule identified with the method of the present invention or the complement sequences thereof, the polypeptide of the invention, the nucleic acid construct of the invention, the organisms, the host cell, the microorganisms, the plant, plant tissue, plant cell, or the part thereof of the invention, the vector of the invention, the antagonist identified with the method of the invention, the antibody of the present invention, the antisense molecule of the present invention, can be used for the reduction of the respective fine chemical in a organism or part thereof, e.g. in a cell.

**[0425.0.0.18] see [0425.0.0.0]**

**[0425.1.18.18]** There are many studies, spanning more than two decades, reporting positive results from the use of Coenzyme Q10 as adjunctive therapy in the treatment of congestive heart failure, e.g. supporting ATP in the mitochondria having Antioxidant action, stabilizing cell membranes or reducing platelet size and limitation of platelet activity. Coenzyme Q10 has been an approved drug in Japan for use in congestive heart failure since 1974. Early studies of congestive heart failure focused on idiopathic dilated cardiomyopathy, testing Coenzyme Q10 against placebo using echocardiography to assess heart function. Echocardiographic improvement seen in these studies was generally slow but sustained and was accompanied by diminished fatigue, chest pain, dyspnea and palpitations. Normal heart size and function were restored in some patients using only Coenzyme Q10; this occurred primarily in patients with recent onset of congestive heart failure.
It is now known that the HMG-CoA reductase inhibitors, while very effective in lowering cholesterol levels, also significantly lower levels of Coenzyme Q10. This may be particularly hazardous for patients with heart failure, suggesting a possible indication for Coenzyme Q10 in many, if not all, individuals using these cholesterol-lowering drugs. Significant Coenzyme Q10 deficiencies have been noted in diseased gingiva. It is not unreasonable to hypothesize that Coenzyme Q10 might be helpful in muscular dystrophy, cancer (e.g. reduce tumor size, remission in metastatic breast cancer), AIDS, neurodegenerative diseases (e.g. Huntington's disease, Familial Alzheimer's disease, Parkinson's disease), Male infertility (Coenzyme Q10 improves sperm motility and protects seminal fluid from free radical injury), chronic stable angina, Significant reduction of plasma levels of lipid peroxidation, skin photoaging, other immune dysfunctions. Muscular dystrophy is usually associated with cardiac disease. There is also some evidence that Coenzyme Q10 might boost energy and speed recovery of exercise-related muscle exhaustion and damage.
Accordingly, the nucleic acid of the invention or used in the method of the invention, the polypeptide of the invention or used in the method of the invention, the nucleic acid construct of the invention, the organisms, the host cell, the microorganisms, the plant, plant tissue, plant cell, or the part thereof of the invention, the vector of the invention, the antagonist or the agonist identified with the method of the invention, the antibody of the present invention, the antisense molecule of the present invention or the nucleic acid molecule identified with the method of the present invention, can be used for the preparation of pharmaceutical, e.g. comprising isolated or recovered the fine chemical, vegetable, lipids, fats or oils, in particular e.g. in combination with other antioxidants, vitamins or coenzymes for the treatment of congestive heart failure, heart muscle dysfunction, (e.g. for stabilizing cell membranes), reduced platelet size, limited of platelet activity, idiopathic dilated cardiomyopathy, atigue, chest pain, dyspnea and palpitations, hypertension and other manifestations of cardiovascular disease, lowering levels of Coenzyme Q10 due to treatments with HMG-CoA reductase inhibitors, diseased gingival, gingival inflammation, cancer, AIDS, other immune dysfunctions, Muscular dystrophy associated with cardiac disease, exercise-related muscle exhaustion and damage, obesity, neurodegenerative diseases (e.g. Huntington's disease, Familial Alzheimer's disease (e.g. in combination with vitamin B6), Parkinson's disease), male infertility, chronic stable angina, significant reduction of plasma levels of lipid peroxidation, skin photoaging, lipid peroxidation or for reducing the size of tumors, for remission in metastatic breast cancer, for improving sperm motility, for protecting against oxidative stress or protecting seminal fluid. The pharmaceutical composition can comprise other coenzymes, vitamins, antioxidants or nutrients. As antioxidant the respective fine chemical is used in one advantageous embodiment in the reduced form.

**[0425.2.18.18]** Further, the nucleic acid of the invention or used in the method of the invention, the polypeptide of the invention or used in the method of the invention, the nucleic acid construct of the invention, the organisms, the host cell, the microorganisms, the plant, plant tissue, plant cell, or the part thereof of the invention, the vector of the invention, the antagonist or the agonist identified with the method of the invention, the antibody of the present invention, the antisense molecule of the present invention or the nucleic acid molecule identified with the method of the present invention, can be used for the preparation of an pharmaceutical composition, e.g. in combination with a pharmaceutical acceptable carrier, a cosmetical composition, e.g. in combination with a emulsifier, detergent, dye additive, softener, formulation agent, wetting agent, lubricating agent etc, or as nutrition supplement, e.g. as oil-based capsules, powder-filled capsules, or tablets or solubilized softgels (microemulsions and others).
Daily doses of Coenzyme Q10 range from 5 to 300 milligrams. Those who use Coenzyme Q10 for periodontal health take 50 to 200 milligrams daily, e.g. 100 to 150 milligrams daily. The same dose range applies to those who take statin drugs for treatment of hypercholesterolemia. Coenzyme Q10 is best taken with food, in particular together with fats or oils. It is reported, that about three weeks of daily dosing are necessary to reach maximal serum concentrations of Coenzyme Q10. Coenzyme Q10 is also available topically in some toothpastes and skin creams.
In one further embodiment, the nucleic acid of the invention or used in the method of the invention, the polypeptide of the invention or used in the method of the invention, the nucleic acid construct of the invention, the organisms, the host cell, the microorganisms, the plant, plant tissue, plant cell, or the part thereof, of the invention, the vector of the invention, the antagonist or the agonist identified with the method of the invention, the antibody of the present invention, the antisense molecule of the present invention or the nucleic acid molecule identified with the method of the present invention used for the preparation of an pharmaceutical composition comprises the isolated or recovered the respective fine chemical, or are comprised in lipids, fats, oils, or membrane fractions of said organism, host cell, microorganism, plant, plant tissue, plant cell, or the part thereof, of the invention, e.g. comprising further other antioxidants, vitamins, nutritional supplements or coenzymes, e.g. selen, vitamin E, vitamin B6, retinol.
It is assumed that Coenzyme Q9 can be used for the above mentioned usages as well. Coenzyme Q9 distincts from Coenzyme Q10 only by "tail" which is one isoprenoid unit shorter. Thus, it is assumed that Coenzyme Q9 is less lipophilic and more hydrophilic than Coenzyme Q10. Therefore, in one embodiment, it is advantageous to use products of the process of the present invention or the products of the present invention for production of a particular hydrophilic cosmetic composition, e.g. in combination with a emulsifier, detergent, dye additive, softener, formulation agent, wetting agent, lubricating agent etc, or a particular hydrophilic nutrition supplement, e.g. as oil-based capsules, powder-filled capsules, or tablets or solubilized softgels (microemulsions and others).

**[0425.3.18.18]** In a further embodiment the present invention relates to the use of the agonist of the present invention, the plant of the present invention or a part thereof, the microorganism or the host cell of the present invention or the polypeptide of the present invention, or a part thereof, for the production a cosmetic composition or a pharmaceutical composition. Such a composition has antioxidative activity, photoprotective activity, and/or tanning activity, can be used for the treating of high levels of cholesterol and/or lipids, to protect, treat or heal the above mentioned diseases, or can be used for the cleaning, conditioning, and/or treating of the skin, e.g. if combined with a pharmaceutically or cosmetically acceptable carrier.
The Coenzyme Q9 and/or Coenzyme Q10 can be also used as stabilizer of other colours or oxygen sensitive compounds.

**[0426.0.0.18] see [0426.0.0.0]**

**[0426.1.0.18]** ./.

**[0427.0.0.18] to [0430.0.0.0] see [0427.0.0.0] to [0430.0.0.0]**

**[0431.0.18.18]** Example 1: Cloning SEQ ID NO: 22609 into in Escherichia coli

**[0432.0.18.18]** A DNA polynucleotide with a sequence as indicated in Table I, column 5 and encoding a polypeptide as listed in Table 1 below, e.g. SEQ ID NO: 22609 was cloned into the plasmids pBR322 (Sutcliffe, J.G. (1979) Proc. Natl Acad. Sci. USA, 75: 3737-3741); pACYC177 (Change & Cohen (1978) J. Bacteriol. 134: 1141-1156); plasmids of the pBS series (pBSSK+, pBSSK- and others; Stratagene, LaJolla, USA) or cosmids such as SuperCos1 (Stratagene, LaJolla, USA) or Lorist6 (Gibson, T.J. Rosenthal, A., and Waterson, R.H. (1987) Gene 53: 283-286) for expression in E. coli using known, well-established procedures (see, for example, Sambrook, J. et al. (1989) "Molecular Cloning: A Laboratory Manual". Cold Spring Harbor Laboratory Press or Ausubel, F.M. et al. (1994) "Current Protocols in Molecular Biology", John Wiley & Sons).

**[0433.0.0.18]** to **[0435.0.0.18] see [0433.0.0.0]** to **[0435.0.0.0]**

**[0436.0.0.18] see [0436.0.0.0]**

**[0436.1.0.18] see [0436.1.0.0]**

**[0437.0.5.18]** Example 4: DNA transfer between Escherichia coli, Saccharomyces cerevisiae and Mortierella alpina (see [0437.0.5.5])

**[0438.0.5.18]** see **[0438.0.5.5]**

**[0438.1.5.18]** see **[0438.0.5.5])**

**[0439.0.0.18] see [0439.0.0.18]**

**[0439.1.5.18]** see **[0439.0.5.5]**

**[0440.0.0.18] see [0440.0.0.0]**

**[0440.1.5.18]** see **[0440.0.5.5]**

**[0441.0.0.18]** see **[0441.0.0.0]**

**[0442.0.0.18]** see **[0442.0.0.0]**

**[0442.1.5.18]** see **[0442.0.5.5]**

**[0443.0.0.18] to [0445.0.0.18] see [0443.0.0.0] to [0445.0.0.0]**

**[0445.1.5.18]** see **[0445.0.5.5]**

**[0445.1.9.18] to [0445.3.9.18] see [0445.1.9.9] to [0445.3.9.9]**

**[0446.0.0.18]** to **[0451.0.0.18] see [0446.0.0.0]** to **[0451.0.0.0]**

**[0451.1.5.18]** see **[0451.0.5.5])**

**[00451.2.0.18]** see **[00451.1.0.0]**

**[0452.0.0.18] to [0455.0.0.18]** see **[0452.0.0.0] to [0455.0.0.0]**

**[0455.0.5.18]** The effect of the genetic modification in plants, fungi, algae, ciliates or on the production of a desired compound (such as a fatty acid) can be determined by growing the modified microorganisms or the modified plant under suitable conditions (such as those described above) and analyzing the medium and/or the cellular components for the elevated production of desired product (i.e. of the lipids or a fatty acid). These analytical techniques are known to the skilled worker and comprise spectroscopy, thin-layer chromatography, various types of staining methods, enzymatic and microbiological methods and analytical chromatography such as high-performance liquid chromatography (see, for example, Ullman, Encyclopedia of Industrial Chemistry, Vol. A2, p. 89-90 and p. 443-613, VCH: Weinheim (1985); Fallon, A., et al., (1987) "Applications of HPLC in Biochemistry" in: Laboratory Techniques in Biochemistry and Molecular Biology, Vol. 17; Rehm et al. (1993) Biotechnology, Vol. 3, Chapter III: "Product recovery and purification", p. 469-714, VCH: Weinheim; Belter, P.A., et al. (1988) Bioseparations: downstream processing for Biotechnology, John Wiley and Sons; Kennedy, J.F., and Cabral, J.M.S. (1992) Recovery processes for biological Materials, John Wiley and Sons; Shaeiwitz, J.A., and Henry, J.D. (1988) Biochemical Separations, in: Ullmann's Encyclopedia of Industrial Chemistry, Vol. B3; Chapter 11, p. 1-27, VCH: Weinheim; and Dechow, F.J. (1989) Separation and purification techniques in biotechnology, Noyes Publications).
In addition to the abovementioned processes, plant lipids are extracted from plant material as described by Cahoon et al. (1999) Proc. Natl. Acad. Sci. USA 96 (22): 12935-12940 and Browse et al. (1986) Analytic Biochemistry 152:141-145. The qualitative and quantitative analysis of lipids or fatty acids is described by Christie, William W., Advances in Lipid Methodology, Ayr/Scotland: Oily Press (Oily Press Lipid Library; 2); Christie, William W., Gas Chromatography and Lipids. A Practical Guide - Ayr, Scotland: Oily Press, 1989, Repr. 1992, IX, 307 pp. (Oily Press Lipid Library; 1); "Progress in Lipid Research, Oxford: Pergamon Press, 1 (1952) - 16 (1977) under the title: Progress in the Chemistry of Fats and Other Lipids CODEN. (see [0455.0.5.5])

**[0456.0.0.18]** see **[0456.0.0.0]**

**[0457.0.18.18]** Example 9: Purification of Coenzyme Q9 or Coenzyme Q10

**[0458.0.18.18]** One example is the analysis of the coenzymes. The unambiguous detection for the presence of the coenzymes products can be obtained by analyzing recombinant organisms using analytical standard methods, especially HPLC with UV or electrochemical detection as for example described in The Journal of Lipid Research, Vol. 39, 2099-2105, 1998.
Possible methods for the production and preparation of coenzymes like Coenzyme Q10 has also been described for example in WO2003056024, J57129695, J57202294, DE3416853 and DD-229152. Further methods for the isolation of the respective fine chemical can also been found in WO 9500634, Fat-Sci.Technol.; (1992) 94, 4, 153-57, DD-294280, DD-293048, JP-145413, DD-273002, DD-271128, SU1406163, JP-166837, JP-176705, Acta-Biotechnol.; (1986) 6, 3, 277-79, DD-229152, DE3416854, DE3416853, JP-202840, JP-048433, JP-125306, JP-087137, JP-014026, WO2003056024 and WO200240682
Plant material is initially homogenized mechanically by comminuting in a pestle and mortar to make it more amenable to extraction.

**[0459.0.18.18]** Usually acetone or hexane is used for the extraction of the coenzymes and further purification is achieved either by column chromatography with a suitable resin.
If necessary, these chromatography steps may be repeated, using identical or other chromatography resins. The skilled worker is familiar with the selection of suitable chromatography resin and the most effective use for a particular molecule to be purified.

**[0460.0.18.18]** In addition depending on the produced fine chemical purification is also possible with crystallization or distillation. Both methods are well known to a person skilled in the art.

**[0461.0.18.18]** Example 10: Cloning SEQ ID NO: 22609 for the expression in plants

**[0462.0.0.18]** see **[0462.0.0.0]**

**[0463.0.18.18]** SEQ ID NO: 22609 is amplified by PCR as described in the protocol of the Pfu Turbo or DNA Herculase polymerase (Stratagene).

**[0464.0.0.18]** to **[0466.0.0.18] see [0464.0.0.0] to [0466.0.0.0]**

**[0467.0.18.18]** The following primer sequences were selected for the gene SEQ ID NO: 22609:
i) forward primer (SEQ ID NO: 22701)
   atgggacaca agcccttata ccg
ii) reverse primer (SEQ ID NO: 22702)
   ttatcgcgat gattttcgct gcg

**[0468.0.0.18]** to **[0479.0.0.18] see [0468.0.0.0]** to **[0479.0.0.0]**

**[0480.0.18.18]** Example 11: Generation of transgenic plants which express SEQ ID NO: 22609

**[0481.0.0.18]** to **[0513.0.0.18] see [0481.0.0.0]** to **[0513.0.0.0]**

**[0514.0.18.18]** The results of the different plant analyses can be seen from the table which follows:

**Table 1:**

| Metabolite Profiling Info: | | | | |
|---|---|---|---|---|
| ORF | **Metabolite** | **Method** | **Min** | **Max** |
| YBR184W | Coenzyme Q10 | LC | 1.70 | 1.95 |
| YDR513W | Coenzyme Q9 | LC | 1.31 | 1.73 |
| YER156C | Coenzyme Q9 | LC | 1.32 | 1.58 |
| YER174C | Coenzyme Q9 | LC | 1.31 | 1.72 |
| YPR138C | Coenzyme Q10 | LC | 1.65 | 3.57 |
| YPR172W | Coenzyme Q9 | LC | 1.34 | 1.66 |
| b3644 | Coenzyme Q9 | LC | 1,37 | 2,30 |
| b2426 | Coenzyme Q9 | LC | 1,38 | 1,77 |
| b2703 | Coenzyme Q9 LC | LC | 1,37 | 2,18 |
| b2729 | Coenzyme Q9 | LC | 1,35 | 1,41 |
| b0175 | Coenzyme Q9 | LC | 1.45 | 1.70 |
| b0730 | Coenzyme Q9 | LC | 1.30 | 3.03 |
| b1829 | Coenzyme Q10 | LC | 1.48 | 5.33 |
| b2699 | Coenzyme Q10 | LC | 1.62 | 3.20 |
| b2699 | Coenzyme Q9 | LC | 1.34 | 3.53 |
| b3605 | Coenzyme Q9 | LC | 1.35 | 1.41 |

**[0515.0.0.18]** to **[0552.0.0.18] see [0515.0.0.0]** to **[0552.0.0.0]** including [0530.1.0.0] to [0530.6. 0.0] as well as [0552.2.0.0]

**[0552.1.18.18]** Example 15: Metabolite Profiling Info from *Zea mays*
*Zea mays* plants were engineered as described in Example 14c.
The results of the different *Zea mays* plants analysed can be seen from table 2 which follows:

**Table 2**

| **ORF_NAME** | **Metabolite** | **Min** | **Max** |
|---|---|---|---|
| b1829 | Coenzyme Q10 | 1,28 | 1,65 |

Table 2 describes the increase in Coenzyme Q10 in genetically modified corn plants expressing the E.coli K12 nucleic acid sequence b1829.
In one embodiment, in case the activity of the E. coli protein b1829 or its homologs, e.g. "a chromatin binding protein, required for S-phase (DNA synthesis) initiation or completion" or its homologs, is increased in corn plants, preferably, an increase of the fine chemical threonine between 28% and 65% is conferred.

**[0553.0.18.18]**
1. A process for the production of Coenzyme Q10 and/or Coenzyme Q9, which comprises
   (a) increasing or generating the activity of a protein as indicated in Table II, columns 5 or 7, lines 231 to 234 and/or 235 to 242 and/or 599 to 602 or a functional equivalent thereof in a non-human organism, or in one or more parts thereof; and
   (b) growing the organism under conditions which permit the production of Coenzyme Q10 and/or Coenzyme Q9 in said organism.
2. A process for the production of Coenzyme Q10 and/or Coenzyme Q9, comprising the increasing or generating in an organism or a part thereof the expression of at least one nucleic acid molecule comprising a nucleic acid molecule selected from the group consisting of:
   a) nucleic acid molecule encoding of a polypeptide as indicated in Table II, columns 5 or 7, lines 231 to 234 and/or 235 to 242 and/or 599 to 602 or a fragment thereof, which confers an increase in the amount of Coenzyme Q10 and/or Coenzyme Q9 in an organism or a part thereof;
   b) nucleic acid molecule comprising of a nucleic acid molecule as indicated in Table I, columns 5 or 7, lines 231 to 234 and/or 235 to 242 and/or 599 to 602 ;
   c) nucleic acid molecule whose sequence can be deduced from a polypeptide sequence encoded by a nucleic acid molecule of (a) or (b) as a result of the degeneracy of the genetic code and conferring an increase in the amount of Coenzyme Q10 and/or Coenzyme Q9 in an organism or a part thereof;
   d) nucleic acid molecule which encodes a polypeptide which has at least 50% identity with the amino acid sequence of the polypeptide encoded by the nucleic acid molecule of (a) to (c) and conferring an increase in the amount of Coenzyme Q10 and/or Coenzyme Q9in an organism or a part thereof;
   e) nucleic acid molecule which hybridizes with a nucleic acid molecule of (a) to (c) under stringent hybridisation conditions and conferring an increase in the amount of Coenzyme Q10 and/or Coenzyme Q9 in an organism or a part thereof;
   f) nucleic acid molecule which encompasses a nucleic acid molecule which is obtained by amplifying nucleic acid molecules from a cDNA library or a genomic library using the primers or primer pairs as indicated in Table III, column 7, lines 231 to 234 and/or 235 to 242 and/or 599 to 602 and conferring an increase in the amount of Coenzyme Q10 and/or Coenzyme Q9 in an organism or a part thereof;
   g) nucleic acid molecule encoding a polypeptide which is isolated with the aid of monoclonal antibodies against a polypeptide encoded by one of the nucleic acid molecules of (a) to (f) and conferring an increase in the amount of Coenzyme Q10 and/or Coenzyme Q9 in an organism or a part thereof;
   h) nucleic acid molecule encoding a polypeptide comprising a consensus as indicated in Table IV, column 7, lines 231 to 234 and/or 235 to 242 and/or 599 to 602 and conferring an increase in the amount of Coenzyme Q10 and/or Coenzyme Q9 in an organism or a part thereof; and
   i) nucleic acid molecule which is obtainable by screening a suitable nucleic acid library under stringent hybridization conditions with a probe comprising one of the sequences of the nucleic acid molecule of (a) to (k) or with a fragment thereof having at least 15 nt, preferably 20 nt, 30 nt, 50 nt, 100 nt, 200 nt or 500 nt of the nucleic acid molecule characterized in (a) to (k) and conferring an increase in the amount of Coenzyme Q10 and/or Coenzyme Q9 in an organism or a part thereof
   or comprising a sequence which is complementary thereto.
3. The process of claim 1 or 2, comprising recovering of the free or bound Coenzyme Q10 and/or Coenzyme Q9.
4. The process of any one of claim 1 to 3, comprising the following steps:
   (a) selecting an organism or a part thereof expressing a polypeptide encoded by the nucleic acid molecule characterized in claim 2;
   (b) mutagenizing the selected organism or the part thereof;
   (c) comparing the activity or the expression level of said polypeptide in the mutagenized organism or the part thereof with the activity or the expression of said polypeptide of the selected organisms or the part thereof;
   (d) selecting the mutated organisms or parts thereof, which comprise an increased activity or expression level of said polypeptide compared to the selected organism or the part thereof;
   (e) optionally, growing and cultivating the organisms or the parts thereof; and
   (f) recovering, and optionally isolating, the free or bound Coenzyme Q10 and/or Coenzyme Q9 produced by the selected mutated organisms or parts thereof.
5. The process of any one of claims 1 to 4, wherein the activity of said protein or the expression of said nucleic acid molecule is increased or generated transiently or stably.
6. An isolated nucleic acid molecule comprising a nucleic acid molecule selected from the group consisting of:
   a) nucleic acid molecule encoding of a polypeptide as indicated in Table II, columns 5 or 7, lines 231 to 234 and/or 235 to 242 and/or 599 to 602, preferably Table II B, columns 5 or 7, lines 231 to 234 and/or 235 to 242 and/or 599 to 602, or a fragment thereof, which confers an increase in the amount of Coenzyme Q10 and/or Coenzyme Q9 in an organism or a part thereof;
   b) nucleic acid molecule comprising of a nucleic acid molecule as indicated in Table I, columns 5 or 7, lines 231 to 234 and/or 235 to 242 and/or 599 to 602, preferably Table I B, columns 5 or 7, lines 231 to 234 and/or 235 to 242 and/or 599 to 602, ;
   c) nucleic acid molecule whose sequence can be deduced from a polypeptide sequence encoded by a nucleic acid molecule of (a) or (b) as a result of the degeneracy of the genetic code and conferring an increase in the amount of Coenzyme Q10 and/or Coenzyme Q9 in an organism or a part thereof;
   d) nucleic acid molecule which encodes a polypeptide which has at least 50% identity with the amino acid sequence of the polypeptide encoded by the nucleic acid molecule of (a) to (c) and conferring an increase in the amount of Coenzyme Q10 and/or Coenzyme Q9 in an organism or a part thereof;
   e) nucleic acid molecule which hybridizes with a nucleic acid molecule of (a) to (c) under stringent hybridisation conditions and conferring an increase in the amount of Coenzyme Q10 and/or Coenzyme Q9 in an organism or a part thereof;
   f) nucleic acid molecule which encompasses a nucleic acid molecule which is obtained by amplifying nucleic acid molecules from a cDNA library or a genomic library using the primers or primer pairs as indicated in Table III, column 7, lines 231 to 234 and/or 235 to 242 and/or 599 to 602 and conferring an increase in the amount of Coenzyme Q10 and/or Coenzyme Q9 in an organism or a part thereof;
   g) nucleic acid molecule encoding a polypeptide which is isolated with the aid of monoclonal antibodies against a polypeptide encoded by one of the nucleic acid molecules of (a) to (f) and conferring an increase in the amount of Coenzyme Q10 and/or Coenzyme Q9 in an organism or a part thereof;
   h) nucleic acid molecule encoding a polypeptide comprising a consensus as indicated in Table IV, column 7, lines 231 to 234 and/or 235 to 242 and/or 599 to 602 and conferring an increase in the amount of Coenzyme Q10 and/or Coenzyme Q9 in an organism or a part thereof; and
   i) nucleic acid molecule which is obtainable by screening a suitable nucleic acid library under stringent hybridization conditions with a probe comprising one of the sequences of the nucleic acid molecule of (a) to (k) or with a fragment thereof having at least 15 nt, preferably 20 nt, 30 nt, 50 nt, 100 nt, 200 nt or 500 nt of the nucleic acid molecule characterized in (a) to (k) and conferring an increase in the amount of Coenzyme Q10 and/or Coenzyme Q9 in an organism or a part thereof
   whereby the nucleic acid molecule distinguishes over the sequence as indicated in Table I A, columns 5 or 7, lines 231 to 234 and/or 235 to 242 and/or 599 to 602 by one or more nucleotides.
7. A nucleic acid construct which confers the expression of the nucleic acid molecule of claim 6, comprising one or more regulatory elements.
8. A vector comprising the nucleic acid molecule as claimed in claim 6 or the nucleic acid construct of claim 7.
9. The vector as claimed in claim 8, wherein the nucleic acid molecule is in operable linkage with regulatory sequences for the expression in a prokaryotic or eukaryotic, or in a prokaryotic and eukaryotic, host.
10. A host cell, which has been transformed stably or transiently with the vector as claimed in claim 8 or 9 or the nucleic acid molecule as claimed in claim 6 or the nucleic acid construct of claim 7 or produced as described in claim any one of claims 2 to 5.
11. The host cell of claim 10, which is a transgenic host cell.
12. The host cell of claim 10 or 11, which is a plant cell, an animal cell, a microorganism, or a yeast cell, a fungus cell, a prokaryotic cell, an eukaryotic cell or an archaebacterium.
13. A process for producing a polypeptide, wherein the polypeptide is expressed in a host cell as claimed in any one of claims 10 to 12.
14. A polypeptide produced by the process as claimed in claim 13 or encoded by the nucleic acid molecule as claimed in claim 6 whereby the polypeptide distinguishes over a sequence as indicated in Table II A, columns 5 or 7, lines 231 to 234 and/or 235 to 242 and/or 599 to 602 by one or more amino acids
15. An antibody, which binds specifically to the polypeptide as claimed in claim 14.
16. A plant tissue, propagation material, harvested material or a plant comprising the host cell as claimed in claim 12 which is plant cell or an Agrobacterium.
17. A method for screening for agonists and antagonists of the activity of a polypeptide encoded by the nucleic acid molecule of claim 6 conferring an increase in the amount of Coenzyme Q10 and/or Coenzyme Q9 in an organism or a part thereof comprising:
   (a) contacting cells, tissues , plants or microorganisms which express the a polypeptide encoded by the nucleic acid molecule of claim 6 conferring an increase in the amount of Coenzyme Q10 and/or Coenzyme Q9 in an organism or a part thereof with a candidate compound or a sample comprising a plurality of compounds under conditions which permit the expression the polypeptide;
   (b) assaying the Coenzyme Q10 and/or Coenzyme Q9 level or the polypeptide expression level in the cell, tissue, plant or microorganism or the media the cell, tissue, plant or microorganisms is cultured or maintained in; and
   (c) identifying a agonist or antagonist by comparing the measured Coenzyme Q10 and/or Coenzyme Q9 level or polypeptide expression level with a standard Coenzyme Q10 and/or Coenzyme Q9 or polypeptide expression level measured in the absence of said candidate compound or a sample comprising said plurality of compounds, whereby an increased level over the standard indicates that the compound or the sample comprising said plurality of compounds is an agonist and a decreased level over the standard indicates that the compound or the sample comprising said plurality of compounds is an antagonist.
18. A process for the identification of a compound conferring increased Coenzyme Q10 and/or Coenzyme Q9 production in a plant or microorganism, comprising the steps:
   (a) culturing a plant cell or tissue or microorganism or maintaining a plant expressing the polypeptide encoded by the nucleic acid molecule of claim 6 conferring an increase in the amount of Coenzyme Q10 and/or Coenzyme Q9 CoQ 9in an organism or a part thereof and a readout system capable of interacting with the polypeptide under suitable conditions which permit the interaction of the polypeptide with said readout system in the presence of a compound or a sample comprising a plurality of compounds and capable of providing a detectable signal in response to the binding of a compound to said polypeptide under conditions which permit the expression of said readout system and of the polypeptide encoded by the nucleic acid molecule of claim 6 conferring an increase in the amount of Coenzyme Q10 and/or Coenzyme Q9 in an organism or a part thereof;
   (b) identifying if the compound is an effective agonist by detecting the presence or absence or increase of a signal produced by said readout system.
19. A method for the identification of a gene product conferring an increase in Coenzyme Q10 and/or Coenzyme Q9 production in a cell, comprising the following steps:
   (a) contacting the nucleic acid molecules of a sample, which can contain a candidate gene encoding a gene product conferring an increase in Coenzyme Q10 and/or Coenzyme Q9 after expression with the nucleic acid molecule of claim 6;
   (b) identifying the nucleic acid molecules, which hybridise under relaxed stringent conditions with the nucleic acid molecule of claim 6;
   (c) introducing the candidate nucleic acid molecules in host cells appropriate for producing Coenzyme Q10 and/or Coenzyme Q9;
   (d) expressing the identified nucleic acid molecules in the host cells;
   (e) assaying the Coenzyme Q10 and/or Coenzyme Q9 level in the host cells; and
   (f) identifying nucleic acid molecule and its gene product which expression confers an increase in the Coenzyme Q10 and/or Coenzyme Q9 level in the host cell in the host cell after expression compared to the wild type.
20. A method for the identification of a gene product conferring an increase in Coenzyme Q10 and/or Coenzyme Q9 production in a cell, comprising the following steps:
   (a) identifying in a data bank nucleic acid molecules of an organism; which can contain a candidate gene encoding a gene product conferring an increase in the Coenzyme Q10 and/or Coenzyme Q9amount or level in an organism or a part thereof after expression, and which are at least 20% homolog to the nucleic acid molecule of claim 6;
   (b) introducing the candidate nucleic acid molecules in host cells appropriate for producing Coenzyme Q10 and/or Coenzyme Q9;
   (c) expressing the identified nucleic acid molecules in the host cells;
   (d) assaying the Coenzyme Q10 and/or Coenzyme Q9 level in the host cells; and
   (e) identifying nucleic acid molecule and its gene product which expression confers an increase in the Coenzyme Q10 and/or Coenzyme Q9 level in the host cell after expression compared to the wild type.
21. A method for the production of an agricultural composition comprising the steps of the method of any one of claims 17 to 20 and formulating the compound identified in any one of claims 17 to 20 in a form acceptable for an application in agriculture.
22. A composition comprising the nucleic acid molecule of claim 6, the polypeptide of claim 14, the nucleic acid construct of claim 7, the vector of any one of claims 8 or 9, an antagonist or agonist identified according to claim 17, the compound of claim 18, the gene product of claim 19 or 20, the antibody of claim 15, and optionally an agricultural acceptable carrier.
23. Use of the nucleic acid molecule as claimed in claim 6 for the identification of a nucleic acid molecule conferring an increase of Coenzyme Q10 and/or Coenzyme Q9 after expression.
24. Use of the polypeptide of claim 14 or the nucleic acid construct claim 7 or the gene product identified according to the method of claim 19 or 20 for identifying compounds capable of conferring a modulation of Coenzyme Q10 and/or Coenzyme Q9 levels in an organism.
25. Cosmetic, pharmaceutical, nutrition composition, food or feed composition comprising the nucleic acid molecule of claim 6, the polypeptide of claim 14, the nucleic acid construct of claim 7, the vector of claim 8 or 9, the antagonist or agonist identified according to claim 17, the antibody of claim 15, the plant or plant tissue of claim 16, the harvested material of claim 16, the host cell of claim 10 to 12 or the gene product identified according to the method of claim 19 or 20.
26. Use of the nucleic acid molecule of claim 6, the polypeptide of claim 14, the nucleic acid construct of claim 7, the vector of claim 8 or 9, the antagonist or agonist identified according to claim 17, the antibody of claim 15, the plant or plant tissue of claim 16, the harvested material of claim 16, the host cell of claim 10 to 12 or the gene product identified according to the method of claim 19 or 20 for the protection of vegetable fats, oils, lipids or waxes comprising the respective fine chemical.
27. Use of the nucleic acid molecule of claim 6, the polypeptide of claim 14, the nucleic acid construct of claim 7, the vector of claim 8 or 9, the antagonist or agonist identified according to claim 17, the antibody of claim 15, the plant or plant tissue of claim 16, the harvested material of claim 16, the host cell of claim 10 to 12 or the gene product identified according to the method of claim 19 or 20 for the production of oils, lipids, fats or waxes comprising the respective fine chemical derived from microorganisms.
28. Use of the nucleic acid molecule of claim 6, the polypeptide of claim 14, the nucleic acid construct of claim 7, the vector of claim 8 or 9, the antagonist or agonist identified according to claim 17, the antibody of claim 15, the plant or plant tissue of claim 16, the harvested material of claim 16, the host cell of claim 10 to 12 or the gene product identified according to the method of claim 19 or 20 for the production of of a pharmaceutical agent for the treatment of congestive heart failure, heart muscle dysfunction, reduced platelet size, limited of platelet activity, idiopathic dilated cardiomyopathy, atigue, chest pain, dyspnea and palpitations, hypertension and other manifestations of cardiovascular disease, lowering levels of Coenzyme Q10 due to treatments with HMG-CoA reductase inhibitors, diseased gingival, gingival inflammation, cancer, AIDS, other immune dysfunctions, Muscular dystrophy associated with cardiac disease, exercise-related muscle exhaustion and damage, obesity, neurodegenerative diseases, male infertility, chronic stable angina, significant reduction of plasma levels of lipid peroxidation, skin photoaging, lipid peroxidation or for reducing the size of tumors, for remission in metastatic breast cancer, for improving sperm motility, for protecting against oxidative stress, for stabilizing cell membranes or protecting seminal fluid or a cosmetic agent or a nutrition supplement for food or feed.

**[0554.0.0.18] Abstract: see [0554.0.0.0]**
Process for the production of fine chemicals

### Description

**[0000.0.0.19]** In a further embodiment, the present invention relates to a further process for the production of fine chemicals as defined below and the corresponding embodiments as described herein as follows.

**[0001.0.0.19]** for the disclosure of this paragraph see [0001.0.0.0].

**[0002.0.19.19]** Plants produce several important secondary metabolites from phenylalanine through the phenylpropanoid pathway. Such substances include flavonoids, lignins, tannins, salicylic acid and hydroxycinnamic acid esters. Recent work on the phenylpropanoid pathway has shown that the traditional view of lignin biosynthesis is incorrect. Although the hydroxylation and methylation reactions of the pathway were long thought to occur at the level of the free hydroxycinnamic aicds, it turns now out, that the enzymes catalyzing phenylpropanoid 3-hydroxylation and 3-O-methylation reactions uses shikimate and CoA conjugates as substrates. The recent cloning of a aldehyde dehydrogenase involved in ferulic acid and sinapic acid biosynthesis suggest that both substances are derived at least in part through oxidation of coniferaldehyde and sinapaldehyde (see Nair et al., 2004, Plant Cell, 16, 544-554 and citations therein).

**[0003.0.0.19]** Ferulic acid is a substance found in the seeds and leaves of most plants, especially in the brans of grasses such as wheat, rice, and oats. Its chemical structure strongly resembles that of curcumin, the substance responsible for the yellow color of the spice turmeric.

**[0004.0.0.19]** The amount of ferulic acid in plant materials varies widely depending on the species and growing conditions; supplements are therefore a more reliable source of this substance than food or unprocessed herbal materials.

**[0005.0.0.19]** Ferulic acid has antioxidant properties that make it an important anti-aging supplement, and they also contribute to ferulic acid's other potential uses. These include applications in diabetes, cardiovascular disease, cancer, neuroprotection, bone degeneration, menopause, immunity, and (perhaps) athletic performance. **[0006.0.0.19]** In male rats fed a high cholesterol diet, ferulic acid supplementation significantly lowered total cholesterol and triglyceride concentrations in the blood, as compared to a control group. Moreover, HDL ('good cholesterol') is increased with ferulic acid supplementation.
Like many other dietary substances, ferulic acid is an antioxidant - but it is an unusually good one. It is especially good at neutralizing the free radicals known as 'superoxide', 'hydroxyl radical', and 'nitric oxide'. It acts synergistically with other antioxidants, giving them extra potency. In addition, ferulic acid can be activated to even higher antioxidant activity by exposure to UV light, suggesting that it might help to protect skin from sun damage.
In microbiological applications ferulic acid is useful as a substrate for vanillin production, as for example described in WO 9735999 or DE19960106 or for melanin production (WO 9720944).

**[0007.0.0.19]** Cinnamic acids, which include caffeic and ferulic acids, are also powerful antioxidants. Experiments have found that these compounds can stop the growth of cancer cells.
In addition sinapic acid is an intermediate in syringyl lignin biosynthesis in angiosperms, and in some taxa serves as a precursor for soluble secondary metabolites. The biosynthesis and accumulation of the sinapate esters sinapoylglucose, sinapoylmalate, and sinapoylcholine are developmentally regulated in at least Arabidopsis and other members of the Brassicaceae (Ruegger et al., 1999, 119(1): 101-10,1999).
Due to these interesting physiological roles and agrobiotechnological potential of ferulic acid or sinapic acid there is a need to identify the genes of enzymes and other proteins involved in ferulic acid or sinapic acid metabolism, and to generate mutants or transgenic plant lines with which to modify the ferulic acid or sinapic acid content in plants.

**[0008.0.19.19]** One way to increase the productive capacity of biosynthesis is to apply recombinant DNA technology. Thus, it would be desirable to produce ferulic acid or sinapic acid in plants. That type of production permits control over quality, quantity and selection of the most suitable and efficient producer organisms. The latter is especially important for commercial production economics and therefore availability to consumers. In addition it is desirable to produce ferulic acid or sinapic acid in plants in order to increase plant productivity and resistance against biotic and abiotic stress as discussed before.
Methods of recombinant DNA technology have been used for some years to improve the production of fine chemicals in microorganisms and plants by amplifying individual biosynthesis genes and investigating the effect on production of fine chemicals. It is for example reported, that the xanthophyll astaxanthin could be produced in the nectaries of transgenic tobacco plants. Those transgenic plants were prepared by *Argobacterium tumifaciens*-mediated transformation of tobacco plants using a vector that contained a ketolase-encoding gene from *H. pluvialis* denominated *crtO* along with the Pds gene from tomato as the promoter and to encode a leader sequence. Those results indicated that about 75 percent of the carotenoids found in the flower of the transformed plant contained a keto group.

**[0009.0.19.19]** Thus, it would be advantageous if an algae, plant or other microorganism were available which produce large amounts ferulic acid or sinapic acid .The invention discussed hereinafter relates in some embodiments to such transformed prokaryotic or eukaryotic microorganisms.
It would also be advantageous if plants were available whose roots, leaves, stem, fruits or flowers produced large amounts of ferulic acid or sinapic acid. The invention discussed hereinafter relates in some embodiments to such transformed plants.

**[0010.0.19.19]** Therefore improving the quality of foodstuffs and animal feeds is an important task of the food-and-feed industry. This is necessary since, for example ferulic acid or sinapic acid, as mentioned above, which occur in plants and some microorganisms are limited with regard to the supply of mammals. Especially advantageous for the quality of foodstuffs and animal feeds is as balanced as possible a specific ferulic acid or sinapic acid profile in the diet since an excess of ferulic acid or sinapic acid above a specific concentration in the food has a positive effect. A further increase in quality is only possible via addition of further ferulic acid or sinapic acid, which are limiting.

**[0011.0.19.19]** To ensure a high quality of foods and animal feeds, it is therefore necessary to add ferulic acid or sinapic acid in a balanced manner to suit the organism.

**[0012.0.19.19]** Accordingly, there is still a great demand for new and more suitable genes which encode enzymes or other proteins which participate in the biosynthesis of ferulic acid or sinapic acid and make it possible to produce them specifically on an industrial scale without unwanted byproducts forming. In the selection of genes for biosynthesis two characteristics above all are particularly important. On the one hand, there is as ever a need for improved processes for obtaining the highest possible contents of ferulic acid or sinapic acid; on the other hand as less as possible byproducts should be produced in the production process.

**[0013.0.0.19]** for the disclosure of this paragraph see [0013.0.0.0].

**[0014.0.19.19]** Accordingly, in a first embodiment, the invention relates to a process for the production of a fine chemical, whereby the fine chemical is a ferulic acid or sinapic acid. Accordingly, in the present invention, the term "the fine chemical" as used herein relates to a ferulic acid or sinapic acid. Further, the term "the fine chemicals" as used herein also relates to fine chemicals comprising ferulic acid or sinapic acid.

**[0015.0.19.19]** In one embodiment, the term "the fine chemical" or "the respective fine chemical" means at least one chemical compound with ferulic acid or sinapic acid activity.
In one embodiment, the term "the fine chemical" means ferulic acid. In one embodiment, the term "the fine chemical" means sinapic acid depending on the context in which the term is used. Throughout the specification the term "the fine chemical" means ferulic acid or sinapic acid, its salts, ester, thioester or in free form or bound to other compounds such sugars or sugarpolymers, like glucoside, e.g. diglucoside.

**[0016.0.19.19]** Accordingly, the present invention relates to a process comprising
(a) increasing or generating the activity of one or more b0196, b0730, b1896, b2414, b3074, b3172, YBR184W, YDR513W or b2818 protein(s) in a non-human organism in one or more parts thereof; and
(b) growing the organism under conditions which permit the production of the fine chemical, thus ferulic acid or sinapic acid in said organism.
Accordingly, the present invention relates to a process comprising.
(a) increasing or generating the activity of one or more proteins having the activity of a protein indicated in Table II, column 3, lines 243 to 250 and 603, resp. or having the sequence of a polypeptide encoded by a nucleic acid molecule indicated in Table I, column 5 or 7, lines 243 to 250 and 603, resp. in a non-human organism in one or more parts thereof; and
growing the organism under conditions which permit the production of the fine chemical, thus, ferulic acid or sinapic acid, in said organism.

**[0016.1.19.19]** Accordingly, the term "the fine chemical" means "ferulic acid " in relation to all sequences listed in Table I, lines 243, 244, 246, 247, 249 or homologs thereof and means "sinapic acid" in relation to the sequence listed in Table I, lines 245, 248, 250, 603 or homologs thereof. Accordingly, the term "the fine chemical" can mean "ferulic acid" or "sinapic acid", owing to circumstances and the context. In order to illustrate that the meaning of the term "the respective fine chemical" means "ferulic acid" or "sinapic acid" owing to the sequences listed in the context the term "the respective fine chemical" is also used.

**[0017.0.0.19] and [0018.0.0.19]** for the disclosure of the paragraphs [0017.0.0.19] and [0018.0.0.19] see paragraphs [0017.0.0.0] and [0018.0.0.0] above.

**[0019.0.19.19]** Advantageously the process for the production of the respective fine chemical leads to an enhanced production of the respective fine chemical. The terms "enhanced" or "increase" mean at least a 10%, 20%, 30%, 40% or 50%, preferably at least 60%, 70%, 80%, 90% or 100%, more preferably 150%, 200%, 300%, 400% or 500% higher production of the respective fine chemical in comparison to the reference as defined below, e.g. that means in comparison to an organism without the aforementioned modification of the activity of a protein having the activity of a protein indicated in Table II, column 3, lines 243 to 250 and 603 or encoded by nucleic acid molecule indicated in Table I, columns 5 or 7, lines 243 to 250 and 603.

**[0020.0.19.19]** Surprisingly it was found, that the transgenic expression of the Escherichia coli K12 protein b0196, b0730, b1896, b2414, b3074, b3172, b2818 or Saccharomyces cerevisiae protein YBR184W or YDR513W in Arabidopsis thaliana conferred an increase in ferulic acid or sinapic acid ("the fine chemical" or "the fine respective chemical") in respect to said proteins and their homologs as wells as the encoding nucleic acid molecules, in particular as indicated in Table II, column 3, lines 243 to 250 and 603 content of the transformed plants.

**[0021.0.0.19]** for the disclosure of this paragraph see [0021.0.0.0] above.

**[0022.0.19.19]** The sequence of b0196 from Escherichia coli K12 has been published in Blattner, F.R. et al., Science 277 (5331), 1453-1474 (1997) and its activity is being defined as regulator in colanic acid synthesis. Accordingly, in one embodiment, the process of the present invention comprises the use of a protein b0196 from Escherichia coli K12 or its homolog, e.g. as shown herein, for the production of the respective fine chemical, in particular for increasing the amount offerulic acid, preferably in free or bound form in an organism or a part thereof, as mentioned. In one embodiment, in the process of the present invention the activity of the protein b0196 is increased.
The sequence of b0730 from Escherichia coli K12 has been published in Blattner F.R. et al., Science 277:1453-1474(1997) and its activity is being defined as a transcriptional regulator of succinyl Co Asynthase operon and fatty acyl responsive regulator. Accordingly, in one embodiment, the process of the present invention comprises the use of a protein b0730 from Escherichia coli K12 or its homolog, e.g. as shown herein, for the production of the respective fine chemical, in particular for increasing the amount of ferulic acid, preferably in free or bound form in an organism or a part thereof, as mentioned. In one embodiment, in the process of the present invention the activity of the protein b0730 is increased.
The sequence of b1896 from Escherichia coli K12 has been published in Blattner F.R. et al., Science 277:1453-1474(1997) and its activity is being defined as a protein having trehalose-6-phosphate synthase activity. Accordingly, in one embodiment, the process of the present invention comprises the use of a protein b1896 from Escherichia coli K12 or its homolog, e.g. as shown herein, for the production of the respective fine chemical, in particular for increasing the amount of sinapic acid, preferably in free or bound form in an organism or a part thereof, as mentioned. In one embodiment, in the process of the present invention the activity of the protein b1896 is increased.
The sequence of b2414 from Escherichia coli K12 has been published in Blattner F.R. et al., Science 277:1453-1474(1997) and its function is being defined as a subunit of cysteine synthase A and O-acetylserine sulfhydrolase A, a PLP-dependent enzyme. Accordingly, in one embodiment, the process of the present invention comprises the use of a protein b2414 from Escherichia coli K12 or its homolog, e.g. as shown herein, for the production of the respective fine chemical, in particular for increasing the amount of ferulic acid, preferably in free or bound form in an organism or a part thereof, as mentioned. In one embodiment, in the process of the present invention the activity of the protein b2414 is increased.
The sequence of b3074 from Escherichia coli K12 has been published in Blattner F.R. et al., Science 277:1453-1474(1997) and its activity is being defined as a putative tRNA synthetase protein. Accordingly, in one embodiment, the process of the present invention comprises the use of a protein b3074 from Escherichia coli K12 or its homolog, e.g. as shown herein, for the production of the respective fine chemical, in particular for increasing the amount of ferulic acid, preferably in free or bound form in an organism or a part thereof, as mentioned. In one embodiment, in the process of the present invention the activity of the protein b3074 is increased.
The sequence of b3172 from Escherichia coli K12 has been published in Blattner F.R. et al., Science 277:1453-1474(1997) and its activity is being defined as a protein having argininosuccinate synthetase activity. Accordingly, in one embodiment, the process of the present invention comprises the use of a protein b3172 from Escherichia coli K12 or its homolog, e.g. as shown herein, for the production of the respective fine chemical, in particular for increasing the amount of sinapic acid, preferably in free or bound form in an organism or a part thereof, as mentioned. In one embodiment, in the process of the present invention the activity of the protein b3172 is increased.
The sequence of YBR184W from Saccharomyces cerevisiae has been published in Goffeau, A. et al., Science 274 (5287), 546-547 (1996) and its activity is being defined as an unclassified protein. Accordingly, in one embodiment, the process of the present invention comprises the use of a protein YBR184W from Saccharomyces cerevisiae or its homolog, e.g. as shown herein, for the production of the respective fine chemical, in particular for increasing the amount of ferulic acid, preferably in free or bound form in an organism or a part thereof, as mentioned. In one embodiment, in the process of the present invention the activity of the unspecified protein YBR184W is increased.
The sequence of YDR513W from Saccharomyces cerevisiae has been published in Jacq, C. et al., Nature 387 (6632 Suppl), 75-78 (1997) and its activity is being defined as a protein having glutaredoxin (thioltransferase) (glutathione reductase) activity. Accordingly, in one embodiment, the process of the present invention comprises the use of a protein YDR513W having said activity, for the production of the respective fine chemical, in particular for increasing the amount of sinapic acid, preferably in free or bound form in an organism or a part thereof, as mentioned. In one embodiment, in the process of the present invention the activity of the protein YDR513W is increased.
The sequence of b2818 (Accession number NP_417295) from Escherichia coli K12 has been published in Blattner et al., Science 277 (5331), 1453-1474, 1997, and its activity is being defined as a N-acetylglutamate synthase (amino acid N-acetyltransferase). Accordingly, in one embodiment, the process of the present invention comprises the use of a gene product with an activity of amino-acid acetyltransferase, acetylglutamate kinase superfamily, preferably a protein with the activity of a a N-acetylglutamate synthase (amino acid N-acetyltransferase) from E. coli or its homolog, e.g. as shown herein, for the production of the fine chemical, meaning of sinapic acid, in particular for increasing the amount of sinapic acid, preferably sinapic acid in free or bound form in an organism or a part thereof, as mentioned.

**[0023.0.19.19]** Homologues (= homologs) of the present gene products can be derived from any organisms as long as the homologue confers the herein mentioned activity, in particular, confers an increase in the respective fine chemical amount or content.
In one embodiment, the homolog of any one of the polypeptides indicated in Table II, column 3, lines 243, 244, 246, 247 or 249 is a homolog having the same or a similar activity. In particular an increase of activity confers an increase in the content of the respective fine chemical in the organisms preferably ferulic acid.
In one embodiment, the homolog of the polypeptides indicated in Table II, column 3, line 245, 248, 250 or 603 is a homolog having the same or a similar activity. In particular an increase of activity confers an increase in the content of the respective fine chemical in the organisms preferably sinapic acid.

**[0023.0.19.19]** Homologs of the polypeptides indicated in Table II, column 3, lines 243 to 248, 250 and 603 may be the polypeptides encoded by the nucleic acid molecules indicated in Table I, column 7, lines 243 to 248, 250 and 603 or may be the polypeptides indicated in Table II, column 7, lines 243 to 248, 250 and 603.
Homologs of the polypeptides indicated in Table II, column 3, lines 243, 244, 246, 247 may be the polypeptides encoded by the nucleic acid molecules indicated in Table I, column 7, lines 243, 244, 246, 247, respectively or may be the polypeptides indicated in Table II, column 7, lines 243, 244, 246, 247, having a ferulic acid content and/or amount increasing activity.
Homologs of the polypeptide indicated in Table 11, column 3, lines 245, 248, 250, 603 may be the polypeptides encoded by the nucleic acid molecules indicated in Table I, column 7, lines 245, 248, 250, 603 respectively or may be the polypeptides indicated in Table II, column 7, lines 245, 248, 250, 603 having a sinapic acid content and/or amount increasing activity.

**[0023.1.0.19]** Homologs of the polypeptides polypeptide indicated in Table II, column 3, lines 243 to 250 and 603 may be the polypetides encoded by the nucleic acid molecules polypeptide indicated in Table I, column 7, lines 243 to 250 and 603 or may be the polypeptides indicated in Table II, column 7, lines 243 to 250 and 603.

**[0024.0.0.19]** for the disclosure of this paragraph see [0024.0.0.0] above.

**[0025.0.19.19]** In accordance with the invention, a protein or polypeptide has the "activity of a protein of the invention", e.g. the activity of a protein indicated in Table II, column 3, lines 243 to 250 and 603 if its *de novo* activity, or its increased expression directly or indirectly leads to an increased ferulic acid or sinapic acid level, resp., in the organism or a part thereof, preferably in a cell of said organism. In a preferred embodiment, the protein or polypeptide has the above-mentioned additional activities of a protein indicated in Table II, column 3, lines 243 to 250 and 603. Throughout the specification the activity or preferably the biological activity of such a protein or polypeptide or an nucleic acid molecule or sequence encoding such protein or polypeptide is identical or similar if it still has the biological or enzymatic activity of any one of the proteins indicated in Table II, column 3, lines 243 to 250 and 603, or which has at least 10% of the original enzymatic activity, preferably 20%, particularly preferably 30%, most particularly preferably 40% in comparison to any one of the proteins indicated in Table II, column 3, lines 243 to 250 and 603 of Escherichia coli K12 or Saccharomyces cerevisiae respectively.
In one embodiment, the polypeptide of the invention confers said activity, e.g. the increase of the respective fine chemical in an organism or a part thereof, if it is derived from an organism, which is evolutionary close to the organism indicated in Table I, column 4 and is expressed in an organism, which is evolutionary distant to the origin organism. For example origin and expressing organism are derived from different families, orders, classes or phylums whereas origin and the organism indicated in Table I, column 4 are derived from the same families, orders, classes or phylums.

**[0025.1.0.19] and [0025.2.0.19]** for the disclosure of the paragraphs [0025.1.0.19] and [0025.2.0.19] see [0025.1.0.0] and [0025.2.0.0] above.

**[0026.0.0.19] to [0033.0.0.19]** for the disclosure of the paragraphs [0026.0.0.19] to [0033.0.0.19] see [0026.0.0.0] to [0033.0.0.0] above.

**[0034.0.19.19]** Preferably, the reference, control or wild type differs form the subject of the present invention only in the cellular activity of the polypeptide of the invention, e.g. as result of an increase in the level of the nucleic acid molecule of the present invention or an increase of the specific activity of the polypeptide of the invention. E.g., it differs by or in the expression level or activity of an protein having the activity of a protein as indicated in Table II, column 3, lines 243 to 250 and 603 or being encoded by a nucleic acid molecule indicated in Table I, column 5, lines 243 to 250 and 603 or its homologs, e.g. as indicated in Table I, column 7, lines 243 to 250 and 603, its biochemical or genetic causes. It therefore shows the increased amount of the respective fine chemical.

**[0035.0.0.19] to [0044.0.0.19]** for the disclosure of the paragraphs [0035.0.0.19] to [0044.0.0.19] see paragraphs [0035.0.0.0] to [0044.0.0.0] above.

**[0045.0.19.19]** In case the activity of the Escherichia coli K12 protein b0196 or its homologs, e.g. as indicated in Table II, columns 5 or 7, line 243 is increased, in one embodiment the increase of the respective fine chemical, preferably of ferulic acid acid between 10% and 25% or more is conferred.
In one embodiment, in case the activity of the Escherichia coli K12 protein b0730 or its homologs, e.g. as indicated in Table II, columns 5 or 7, line 244 is increased, preferably, in one embodiment the increase of the respective fine chemical, preferably of ferulic acid between 38% and 97% or more is conferred.
In one embodiment, in case the activity of the Escherichia coli K12 protein b1896 or its homologs, e.g. as indicated in Table II, columns 5 or 7, line 245 is increased, preferably, in one embodiment the increase of the respective fine chemical, preferably of sinapic acid between 38% and 98% or more is conferred.
In one embodiment, in case the activity of the Escherichia coli K12 protein b2414 or its homologs, e.g. as indicated in Table II, columns 5 or 7, line 246 is increased, preferably, in one embodiment the increase of the respective fine chemical, preferably of ferulic acid between 34% and 86% or more is conferred.
In one embodiment, in case the activity of the Escherichia coli K12 protein b3074 or its homologs, e.g. as indicated in Table II, columns 5 or 7, line 247 is increased, preferably, in one embodiment the increase of the respective fine chemical, preferably of ferulic acid between 35% and 73% or more is conferred.
In one embodiment, in case the activity of the Escherichia coli K12 protein b3172 or its homologs, e.g. as indicated in Table II, columns 5 or 7, line 248 is increased, preferably, in one embodiment the increase of the respective fine chemical, preferably of sinapic acid between 31 % and 89% or more is conferred.
In one embodiment, in case the activity of the Saccharomyces cerevisae protein YBR184W or its homologs as indicated in Table II, columns 5 or 7, line 249, is increased, preferably, in one embodiment the increase of the respective fine chemical, preferably of ferulic acid between 30% and 37% or more is conferred.
In one embodiment, in case the activity of the Saccharomyces cerevisiae protein YDR513W or its homologsas indicated in Table II, columns 5 or 7, line 250, is increased, preferably, in one embodiment an increase of the respective fine chemical, preferably of sinapic acid between 30% and 39% or more is conferred.
In one embodiment, in case the activity of the Escherichia coli K12 protein b2818 or its homologs, e.g. as indicated in Table II, columns 5 or 7, line 603 is increased, preferably, in one embodiment the increase of the respective fine chemical, preferably of sinapic acid between 27% and 54% or more is conferred.

**[0046.0.0.19]** In one embodiment, in case the activity of the Escherichia coli K12 protein b0196 or its homologs, e.g. a regulator in colanic acid synthesis is increased, preferably an increase of the fine chemical ferulic acid is conferred. In one embodiment, in case the activity of the Escherichia coli K12 protein b0730 or its homologs, e.g. a transcriptional regulator of succinylCoA synthetase operon and fatty acyl response regulator increased, preferably an increase of the fine chemical ferulic acid is conferred.
In one embodiment, in case the activity of the Escherichia coli K12 protein b1896 or its homologs, e.g. a trehalose-6-phosphate synthase 5 is increased, preferably an increase of the fine chemical sinapic acid is conferred.
In one embodiment, in case the activity of the Escherichia coli K12 protein b2414 or its homologs, e.g. a subunit of cysteine synthase A and O-acetylserine sulfhydrolase A, PLP-dependent enzyme is increased, preferably an increase of the fine chemical ferulic acid is conferred.
In one embodiment, in case the activity of the Escherichia coli K12 protein b3074 or its homologs, e.g. a putative tRNA synthetase is increased, preferably an increase of the fine chemical ferulic acid is conferred.
In one embodiment, in case the activity of the Escherichia coli K12 protein b3172 or its homologs, e.g. a protein having argininosuccinate synthetase activity is increased, preferably an increase of the fine chemical sinapic acid is conferred.
In one embodiment, in case the activity of the Saccharomyces cerevisae protein YBR184W or its homologs is increased, preferably an increase of the fine chemical ferulic acid is conferred.
In one embodiment, in case the activity of the Saccharomyces cerevisiae protein YDR513W or its homologs is increased, preferably an increase of the fine chemical sinapic acid is conferred.
In one embodiment, in case the activity of the Escherichia coli K12 protein b2818 or its homologs, e.g. a N-acetylglutamate synthase (amino acid N-acetyltransferase) is increased, preferably, in one embodiment the increase of the respective fine chemical, preferably an increase of the fine chemical sinapic acid is conferred.

**[[0047.0.0.19] and [0048.0.0.19]** for the disclosure of the paragraphs [0047.0.0.19] and [0048.0.0.19] see paragraphs [0047.0.0.0] and [0048.0.0.0] above.

**[0049.0.19.19]** A protein having an activity conferring an increase in the amount or level of the respective fine chemical ferulic acid preferably has the structure of the polypeptide described herein. In a particular embodiment, the polypeptides used in the process of the present invention or the polypeptide of the present invention comprises the sequence of a consensus sequence as indicated in Table IV, columns 7, lines 243, 244, 246, 247 or of a polypeptide as indicated in Table II, columns 5 or 7, lines 243, 244, 246, 247 and/or 249 or of a functional homologue thereof as described herein, or of a polypeptide encoded by the nucleic acid molecule characterized herein or the nucleic acid molecule according to the invention, for example by a nucleic acid molecule as indicated in Table I, columns 5 or 7, lines 243, 244, 246, 247 and/or 249 or its herein described functional homologues and has the herein mentioned activity conferring an increase in the ferulic acid level.
A protein having an activity conferring an increase in the amount or level of the sinapic preferably has the structure of the polypeptide described herein. In a particular embodiment, the polypeptides used in the process of the present invention or the polypeptide of the present invention comprises the sequence of a consensus sequence as indicated in Table IV, column 7, line 245, 248, 250 and/or 603 or of a polypeptide as indicated in Table II, columns 5 or 7, line 245, 248, 250 and/or 603 or of a functional homologue thereof as described herein, or of a polypeptide encoded by the nucleic acid molecule characterized herein or the nucleic acid molecule according to the invention, for example by a nucleic acid molecule as indicated in Table I, columns 5 or 7, line 245, 248, 250 and/or 603 or its herein described functional homologues and has the herein mentioned activity confering an increase in the sinapic level.

**[0050.0.19.19]** For the purposes of the present invention, the term "the respective fine chemical" also encompass the corresponding salts, such as, for example, the potassium or sodium salts of ferulic acid or sinapic acid, resp., or their ester, or glucoside thereof, e.g the diglucoside thereof.

**[0051.0.19.19]** Owing to the biological activity of the proteins which are used in the process according to the invention and which are encoded by nucleic acid molecules according to the invention, it is possible to produce compositions comprising the respective fine chemical, i.e. an increased amount of the free chemical free or bound, e.g compositions comprising ferulic acid or sinapic acid. Depending on the choice of the organism used for the process according to the present invention, for example a microorganism or a plant, compositions or mixtures of ferulic acid or sinapic acid can be produced.

**[0052.0.0.19]** for the disclosure of this paragraph see paragraph [0052.0.0.0] above.

**[0053.0.19.19]** In one embodiment, the process of the present invention comprises one or more of the following steps
a) stabilizing a protein conferring the increased expression of a protein encoded by the nucleic acid molecule of the invention or of the polypeptid of the invention, e.g. of a polypeptide having an activity of a protein as indicated in Table II, column 3, lines 243 to 250 and 603 or its homologs, e.g. as indicated in Table II, columns 5 or 7, lines 243 to 250 and 603, activity having herein-mentioned the respective fine chemical increasing activity;
b) stabilizing a mRNA conferring the increased expression of a protein encoded by the nucleic acid molecule of the invention, e.g. of a polypeptide having an activity of a protein as indicated in Table II, column 3, lines 243 to 250 and 603 or its homologs activity, e.g. as indicated in Table II, columns 5 or 7, lines 243 to 250 and 603, or of a mRNA encoding the polypeptide of the present invention having herein-mentioned the respective fine chemical increasing activity;
c) increasing the specific activity of a protein conferring the increased expression of a protein encoded by the nucleic acid molecule of the invention or of the polypeptide of the present invention having herein-mentioned the respective fine chemical increasing activity, e.g. of a polypeptide having an activity of a protein as indicated in Table II, column 3, lines 243 to 250 and 603 or its homologs activity, e.g. as indicated in Table II, columns 5 or 7, lines 243 to 250 and 603, or decreasing the inhibitory regulation of the polypeptide of the invention;
d) generating or increasing the expression of an endogenous or artificial transcription factor mediating the expression of a protein conferring the increased expression of a protein encoded by the nucleic acid molecule of the invention or of the polypeptide of the invention having herein-mentioned the respective fine chemical increasing activity, e.g. of a polypeptide having an activity of a protein as indicated in Table Il, column 3, lines 243 to 250 and 603 or its homologs activity, e.g. as indicated in Table II, columns 5 or 7, lines 243 to 250 and 603;
e) stimulating activity of a protein conferring the increased expression of a protein encoded by the nucleic acid molecule of the present invention or a polypeptide of the present invention having herein-mentioned the respective fine chemical increasing activity, e.g. of a polypeptide having an activity of a protein as indicated in Table II, column 3, lines 243 to 250 and 603 or its homologs activity, e.g. as indicated in Table II, columns 5 or 7, lines 243 to 250 and 603, by adding one or more exogenous inducing factors to the organism or parts thereof;
f) expressing a transgenic gene encoding a protein conferring the increased expression of a polypeptide encoded by the nucleic acid molecule of the present invention or a polypeptide of the present invention, having herein-mentioned the respective fine chemical increasing activity, e.g. of a polypeptide having an activity of a protein as indicated in Table II, column 3, lines 243 to 250 and 603 or its homologs activity, e.g. as indicated in Table II, columns 5 or 7, lines 243 to 250 and 603, and/or
g) increasing the copy number of a gene conferring the increased expression of a nucleic acid molecule encoding a polypeptide encoded by the nucleic acid molecule of the invention or the polypeptide of the invention having herein-mentioned the respective fine chemical increasing activity, e.g. of a polypeptide having an activity of a protein as indicated in Table II, column 3, lines 243 to 250 and 603 or its homologs, e.g. as indicated in Table II, columns 5 or 7, lines 243 to 250 and 603, activity.
h) Increasing the expression of the endogenous gene encoding the polypeptide of the invention, e.g. a polypeptide having an activity of a protein as indicated in Table II, column 3, lines 243 to 250 and 603 or its homologs activity, e.g. as indicated in Table II, columns 5 or 7, lines 243 to 250 and 603, by adding positive expression or removing negative expression elements, e.g. homologous recombination can be used to either introduce positive regulatory elements like for plants the 35S enhancer into the promoter or to remove repressor elements form regulatory regions. Further gene conversion methods can be used to disrupt repressor elements or to enhance to activity of positive elements. Positive elements can be randomly introduced in plants by T-DNA or transposon mutagenesis and lines can be identified in which the positive elements have be integrated near to a gene of the invention, the expression of which is thereby enhanced;
   and/or
i) Modulating growth conditions of an organism in such a manner, that the expression or activity of the gene encoding the protein of the invention or the protein itself is enhanced for example microorganisms or plants can be grown for example under a higher temperature regime leading to an enhanced expression of heat shock proteins, which can lead to an enhanced respective fine chemical production.
j) selecting of organisms with especially high activity of the proteins of the invention from natural or from mutagenized resources and breeding them into the target organisms, e.g. the elite crops.

**[0054.0.19.19]** Preferably, said mRNA is the nucleic acid molecule of the present invention and/or the protein conferring the increased expression of a protein encoded by the nucleic acid molecule of the present invention or the polypeptide having the herein mentioned activity is the polypeptide of the present invention, e.g. conferring the increase of the respective fine chemical after increasing the expression or activity of the encoded polypeptide or having the activity of a polypeptide having an activity of a protein as indicated in Table II, columns 3 or 5, lines 243 to 250 and 603, resp., or its homologs activity, e.g. as indicated in Table II, columns 5 or 7, lines 243 to 250 and 603, resp.

**[0055.0.0.19] to [0067.0.0.19]** for the disclosure of the paragraphs [0055.0.0.19] to [0067.0.0.19] see paragraphs [0055.0.0.0] to [0067.0.0.0] above.

**[0068.0.19.19]** The mutation is introduced in such a way that the production of ferulic acid or sinapic acid is not adversely affected.

**[0069.0.0.19]** for the disclosure of this paragraph see paragraph [0069.0.0.0] above.

**[0070.0.19.19]** Owing to the introduction of a gene or a plurality of genes conferring the expression of the nucleic acid molecule of the invention or the polypeptide of the invention, for example the nucleic acid construct mentioned below, or encoding a protein of the invention into an organism alone or in combination with other genes, it is possible not only to increase the biosynthetic flux towards the end product, but also to increase, modify or create *de novo* an advantageous, preferably novel metabolite composition in the organism, e.g. an advantageous composition of ferulic acid and sinapic acid or their biochemical derivatives , e.g. comprising a higher content of (from a viewpoint of nutrional physiology limited) ferulic acid and/or sinapic acid or their derivatives.

**[0071.0.0.19]** for the disclosure of this paragraph see paragraph [0071.0.0.0] above.

**[0072.0.0.19]** %

**[0073.0.19.19]** Accordingly, in one embodiment, the process according to the invention relates to a process, which comprises:
(j) providing a non-human organism, preferably a microorganism, a non-human animal, a plant or animal cell, a plant or animal tissue or a plant;
(k) increasing an activity of a polypeptide of the invention or a homolog thereof, e.g. as indicated in Table II, columns 5 or 7, lines 243 to 250 and 603 , or of a polypeptide being encoded by the nucleic acid molecule of the present invention and described below, e.g. conferring an increase of the respective fine chemical in an organism, preferably in a microorganism, a non-human animal, a plant or animal cell, a plant or animal tissue or a plant,
(I) growing an organism, preferably a microorganism, a non-human animal, a plant or animal cell, a plant or animal tissue or a plant under conditions which permit the production of the respective fine chemical in the organism, preferably the microorganism, the plant cell, the plant tissue or the plant; and
(m) if desired, recovering, optionally isolating, the free and/or bound the respective fine chemical synthesized by the organism, the microorganism, the non-human animal, the plant or animal cell, the plant or animal tissue or the plant.

**[0074.0.19.19]** The organism, in particular the microorganism, non-human animal, the plant or animal cell, the plant or animal tissue or the plant is advantageously grown in such a way that it is not only possible to recover, if desired isolate the free or bound respective fine chemical.

**[0075.0.0.19] to [0077.0.0.19]** for the disclosure of the paragraphs [0075.0.0.19] to [0077.0.0.19] see paragraphs [0075.0.0.0] to [0077.0.0.0] above.

**[0078.0.19.19]** The organism such as microorganisms or plants or the recovered, and if desired isolated, the respective fine chemical can then be processed further directly into foodstuffs or animal feeds or for other applications. The fermentation broth, fermentation products, plants or plant products can be purified with methods known to the person skilled in the art. Products of these different work-up procedures are ferulic acid or sinapic acid or comprising compositions of ferulic acid and sinapic acid still comprising fermentation broth, plant particles and cell components in different amounts, advantageously in the range of from 0 to 99% by weight, preferably below 80% by weight, especially preferably below 50% by weight.

**[0079.0.0.19] to [0084.0.0.19]** for the disclosure of the paragraphs [0079.0.0.19] to [0084.0.0.19] see paragraphs [0075.0.0.0] to [0084.0.0.0] above.

**[0085.0.19.19]** With regard to the nucleic acid sequence as depicted a nucleic acid construct which contains a nucleic acid sequence mentioned herein or an organism (= transgenic organism) which is transformed with said nucleic acid sequence or said nucleic acid construct, "transgene" means all those constructs which have been brought about by genetic manipulation methods, preferably in which either
a) a nucleic acid sequence as indicated in Table I, columns 5 or 7, lines 243 to 250 and 603, or a derivative thereof, or
b) a genetic regulatory element, for example a promoter, which is functionally linked to the nucleic acid sequence as indicated in Table I, columns 5 or 7, lines 243 to 250 and 603, or a derivative thereof, or
c) (a) and (b)
is/are not present in its/their natural genetic environment or has/have been modified by means of genetic manipulation methods, it being possible for the modification to be, by way of example, a substitution, addition, deletion, inversion or insertion of one or more nucleotide. "Natural genetic environment" means the natural chromosomal locus in the organism of origin or the presence in a genomic library. In the case of a genomic library, the natural, genetic environment of the nucleic acid sequence is preferably at least partially still preserved. The environment flanks the nucleic acid sequence at least on one side and has a sequence length of at least 50 bp, preferably at least 500 bp, particularly preferably at least 1000 bp, very particularly preferably at least 5000 bp.

**[0086.0.0.19] and [0087.0.0.19]** for the disclosure of the paragraphs [0086.0.0.19] and [0087.0.0.19] see paragraphs [0086.0.0.0] and [0087.0.0.0] above.

**[0088.0.19.19]** In an advantageous embodiment of the invention, the organism takes the form of a plant whose content of the respective fine chemical is modified advantageously owing to the nucleic acid molecule of the present invention expressed.
This is important for plant breeders since, for example, the nutritional value of plants for animals such as poultry is dependent on the abovementioned fine chemicals or the plants are more resistant to biotic and abiotic stress and the yield is increased.

**[0088.1.0.19]** for the disclosure of this paragraph see paragraph [0088.1.0.0] above.

**[0089.0.0.19] to [0094.0.0.19]** for the disclosure of the paragraphs [0089.0.0.19] to [0094.0.0.19] see paragraphs [0089.0.0.0] to [0094.0.0.0] above.

**[0095.0.19.19]** It may be advantageous to increase the pool of ferulic acid or sinapic acid in the transgenic organisms by the process according to the invention in order to isolate high amounts of the pure respective fine chemical and/or to obtain increased resistance against biotic and abiotic stresses and to obtain higher yield.

**[0096.0.19.19]** In another preferred embodiment of the invention a combination of the increased expression of the nucleic acid sequence or the protein of the invention together with the transformation of a protein or polypeptid or a compound, which functions as a sink for the desired fine chemical, for example in the organism, is useful to increase the production of the respective fine chemical.

**[0097.0.0.19]** for the disclosure of this paragraph see paragraph [0097.0.0.0] above.

**[0098.0.19.19]** In a preferred embodiment, the respective fine chemical is produced in accordance with the invention and, if desired, is isolated.

**[0099.0.19.19]** In the case of the fermentation of microorganisms, the abovementioned desired fine chemical may accumulate in the medium and/or the cells. If microorganisms are used in the process according to the invention, the fermentation broth can be processed after the cultivation. Depending on the requirement, all or some of the biomass can be removed from the fermentation broth by separation methods such as, for example, centrifugation, filtration, decanting or a combination of these methods, or else the biomass can be left in the fermentation broth. The fermentation broth can subsequently be reduced, or concentrated, with the aid of known methods such as, for example, rotary evaporator, thin-layer evaporator, falling film evaporator, by reverse osmosis or by nanofiltration. Afterwards advantageously further compounds for formulation can be added such as corn starch or silicates. This concentrated fermentation broth advantageously together with compounds for the formulation can subsequently be processed by lyophilization, spray drying, and spray granulation or by other methods. Preferably the respective fine chemical comprising compositions are isolated from the organisms, such as the microorganisms or plants or the culture medium in or on which the organisms have been grown, or from the organism and the culture medium, in the known manner, for example via extraction, distillation, crystallization, chromatography or a combination of these methods. These purification methods can be used alone or in combination with the aforementioned methods such as the separation and/or concentration methods.

**[0100.0.19.19]** Transgenic plants which comprise the fine chemicals such as ferulic acid or sinapic acid synthesized in the process according to the invention can advantageously be marketed directly without there being any need for the fine chemicals synthesized to be isolated. Plants for the process according to the invention are listed as meaning intact plants and all plant parts, plant organs or plant parts such as leaf, stem, seeds, root, tubers, anthers, fibers, root hairs, stalks, embryos, calli, cotelydons, petioles, harvested material, plant tissue, reproductive tissue and cell cultures which are derived from the actual transgenic plant and/or can be used for bringing about the transgenic plant. In this context, the seed comprises all parts of the seed such as the seed coats, epidermal cells, seed cells, endosperm or embryonic tissue.
However, the respective fine chemical produced in the process according to the invention can also be isolated from the organisms, advantageously plants, as extracts, e.g. ether, alcohol, or other organic solvents or water containing extract and/or free fine chemicals. The respective fine chemical produced by this process can be obtained by harvesting the organisms, either from the crop in which they grow, or from the field. This can be done via pressing or extraction of the plant parts. To increase the efficiency of extraction it is beneficial to clean, to temper and if necessary to hull and to flake the plant material. To allow for greater ease of disruption of the plant parts, specifically the seeds, they can previously be comminuted, steamed or roasted. Seeds, which have been pretreated in this manner can subsequently be pressed or extracted with solvents such as warm hexane. The solvent is subsequently removed. In the case of microorganisms, the latter are, after harvesting, for example extracted directly without further processing steps or else, after disruption, extracted via various methods with which the skilled worker is familiar. Thereafter, the resulting products can be processed further, i.e. degummed and/or refined. In this process, substances such as the plant mucilages and suspended matter can be first removed. What is known as desliming can be affected enzymatically or, for example, chemico-physically by addition of acid such as phosphoric acid.
Because ferulic acid or sinapic acid in microorganisms are localized intracellular, their recovery essentially comes down to the isolation of the biomass. Well-established approaches for the harvesting of cells include filtration, centrifugation and coagulation/flocculation as described herein. Of the residual hydrocarbon, adsorbed on the cells, has to be removed. Solvent extraction or treatment with surfactants have been suggested for this purpose.

**[0101.0.19.19]** The identity and purity of the compound(s) isolated can be determined by prior-art techniques. They encompass high-performance liquid chromatography (HPLC), gas chromatography (GC), spectroscopic methods, mass spectrometry (MS), staining methods, thin-layer chromatography, NIRS, enzyme assays or microbiological assays. These analytical methods are compiled in: Patek et al. (1994) Appl. Environ. Microbiol. 60:133-140; Malakhova et al. (1996) Biotekhnologiya 11 27-32; and Schmidt et al. (1998) Bioprocess Engineer. 19:67-70. Ulmann's Encyclopedia of Industrial Chemistry (1996) Bd. A27, VCH Weinheim, pp. 89-90, pp. 521-540, pp. 540-547, pp. 559-566, 575-581 and pp. 581-587; Michal, G (1999) Biochemical Pathways: An Atlas of Biochemistry and Molecular Biology, John Wiley and Sons; Fallon, A. et al. (1987) Applications of HPLC in Biochemistry in: Laboratory Techniques in Biochemistry and Molecular Biology, vol. 17.

**[0102.0.19.19]** Ferulic acid or sinapic acid can for example be detected advantageously via HPLC, LC or GC separation methods. The unambiguous detection for the presence of ferulic acid or sinapic acid containing products can be obtained by analyzing recombinant organisms using analytical standard methods: LC, LC-MS, MS or TLC). The material to be analyzed can be disrupted by sonication, grinding in a glass mill, liquid nitrogen and grinding, cooking, or via other applicable methods.

**[0103.0.19.19]** In a preferred embodiment, the present invention relates to a process for the production of the respective fine chemical comprising or generating in an organism or a part thereof the expression of at least one nucleic acid molecule comprising a nucleic acid molecule selected from the group consisting of:
a) nucleic acid molecule encoding, preferably at least the mature form, of the polypeptide having a sequence as indicated in Table II, columns 5 or 7, lines 243 to 250 and 603,or a fragment thereof, which confers an increase in the amount of the respective fine chemical in an organism or a part thereof;
b) nucleic acid molecule comprising, preferably at least the mature form, of a nucleic acid molecule having a sequence as indicated in Table I, columns 5 or 7, lines 243 to 250 and 603,
c) nucleic acid molecule whose sequence can be deduced from a polypeptide sequence encoded by a nucleic acid molecule of (a) or (b) as result of the degeneracy of the genetic code and conferring an increase in the amount of the respective fine chemical in an organism or a part thereof;
d) nucleic acid molecule encoding a polypeptide which has at least 50% identity with the amino acid sequence of the polypeptide encoded by the nucleic acid molecule of (a) to (c) and conferring an increase in the amount of the respective fine chemical in an organism or a part thereof;
e) nucleic acid molecule which hybridizes with a nucleic acid molecule of (a) to (c) under under stringent hybridization conditions and conferring an increase in the amount of the respective fine chemical in an organism or a part thereof;
f) nucleic acid molecule encoding a polypeptide, the polypeptide being derived by substituting, deleting and/or adding one or more amino acids of the amino acid sequence of the polypeptide encoded by the nucleic acid molecules (a) to (d), preferably to (a) to (c) and conferring an increase in the amount of the respective fine chemical in an organism or a part thereof;
g) nucleic acid molecule encoding a fragment or an epitope of a polypeptide which is encoded by one of the nucleic acid molecules of (a) to (e), preferably to (a) to (c) and and conferring an increase in the amount of the respective fine chemical in an organism or a part thereof;
h) nucleic acid molecule comprising a nucleic acid molecule which is obtained by amplifying nucleic acid molecules from a cDNA library or a genomic library using the primers pairs having a sequence as indicated in Table III, columns 7, lines 243 to 250 and 603, and conferring an increase in the amount of the respective fine chemical in an organism or a part thereof;
i) nucleic acid molecule encoding a polypeptide which is isolated, e.g. from an expression library, with the aid of monoclonal antibodies against a polypeptide encoded by one of the nucleic acid molecules of (a) to (h), preferably to (a) to (c), and and conferring an increase in the amount of the respective fine chemical in an organism or a part thereof;
j) nucleic acid molecule which encodes a polypeptide comprising the consensus sequence having a sequences as indicated in Table IV, column 7, lines 243 to 250 and 603 and conferring an increase in the amount of the respective fine chemical in an organism or a part thereof;
k) nucleic acid molecule comprising one or more of the nucleic acid molecule encoding the amino acid sequence of a polypeptide encoding a domain of the polypeptide indicated in Table II, columns 5 or 7, lines 243 to 250 and 603, and conferring an increase in the amount of the respective fine chemical in an organism or a part thereof; and
l) nucleic acid molecule which is obtainable by screening a suitable library under stringent conditions with a probe comprising one of the sequences of the nucleic acid molecule of (a) to (k), preferably to (a) to (c), or with a fragment of at least 15 nt, preferably 20 nt, 30 nt, 50 nt, 100 nt, 200 nt or 500 nt of the nucleic acid molecule characterized in (a) to (k), preferably to (a) to (c), and conferring an increase in the amount of the respective fine chemical in an organism or a part thereof;
or which comprises a sequence which is complementary thereto.

**[00103.1.19.19]** In one embodiment, the nucleic acid molecule used in the process of the invention distinguishes over the sequence indicated in Table IA, columns 5 or 7, lines 243 to 250 and 603, by one or more nucleotides. In one embodiment, the nucleic acid molecule used in the process of the invention does not consist of the sequence shown in indicated in Table I A, columns 5 or 7, lines 243 to 250 and 603. In one embodiment, the nucleic acid molecule used in the process of the invention is less than 100%, 99,999%, 99,99%, 99,9% or 99% identical to a sequence indicated in Table I A, columns 5 or 7, lines 243 to 250 and 603. In another embodiment, the nucleic acid molecule does not encode a polypeptide of a sequence indicated in Table II A, columns 5 or 7, lines 243 to 250 and 603.

**[00103.2.19.19]** In one embodiment, the nucleic acid molecule used in the process of the invention distinguishes over the sequence indicated in Table I B, columns 5 or 7, lines 243 to 250 and 603, by one or more nucleotides. In one embodiment, the nucleic acid molecule used in the process of the invention does not consist of the sequence shown in indicated in Table I B, columns 5 or 7, lines 243 to 250 and 603. In one embodiment, the nucleic acid molecule used in the process of the invention is less than 100%, 99,999%, 99,99%, 99,9% or 99% identical to a sequence indicated in Table I B, columns 5 or 7, lines 243 to 250 and 603. In another embodiment, the nucleic acid molecule does not encode a polypeptide of a sequence indicated in Table II B, columns 5 or 7, lines 243 to 250 and 603.

**[0104.0.19.19]** In one embodiment, the nucleic acid molecule used in the process of the present invention distinguishes over the sequence indicated in Table I, columns 5 or 7, lines 243 to 250 and 603 by one or more nucleotides. In one embodiment, the nucleic acid molecule used in the process of the invention does not consist of the sequence indicated in Table I, columns 5 or 7, lines 243 to 250 and 603 In one embodiment, the nucleic acid molecule of the present invention is less than 100%, 99,999%, 99,99%, 99,9% or 99% identical to the sequence indicated in Table I, columns 5 or 7, lines 243 to 250 and 603. In another embodiment, the nucleic acid molecule does not encode a polypeptide of a sequence indicated in Table II, columns 5 or 7, lines 243 to 250 and 603.

**[0105.0.0.19] to [0107.0.0.19]** for the disclosure of the paragraphs [0105.0.0.19] to [0107.0.0.19] see paragraphs [0105.0.0.0] and [0107.0.0.0] above.

**[0108.0.19.19]** Nucleic acid molecules with the sequence as indicated in Table I, columns 5 or 7, lines 243 to 250 and 603, nucleic acid molecules which are derived from an amino acid sequences as indicated in Table II, columns 5 or 7, lines 243 to 250 and 603 or from polypeptides comprising the consensus sequence as indicated in Table IV, column 7, lines 243 to 250 and 603, or their derivatives or homologues encoding polypeptides with the enzymatic or biological activity of an activity of a polypeptide as indicated in Table II, column 3, 5 or 7, lines 243 to 250 and 603, e.g. conferring the increase of the respective fine chemical, meaning ferulic acid or sinapic acid, resp., after increasing its expression or activity, are advantageously increased in the process according to the invention.

**[0109.0.19.19]** In one embodiment, said sequences are cloned into nucleic acid constructs, either individually or in combination. These nucleic acid constructs enable an optimal synthesis of the respective fine chemicals, in particular ferulic acid or sinapic acid, produced in the process according to the invention.

**[0110.0.0.19]** Nucleic acid molecules, which are advantageous for the process according to the invention and which encode polypeptides with an activity of a polypeptide used in the method of the invention or used in the process of the invention, e.g. of a protein as shown in Table II, columns 5 or 7, lines 243 to 250 and 603 or being encoded by a nucleic acid molecule indicated in Table I, columns 5 or 7, lines 243 to 250 and 603 or of its homologs, e.g. as indicated in Table II, columns 5 or 7, lines 243 to 250 and 603 can be determined from generally accessible databases.

**[0111.0.0.19]** for the disclosure of this paragraph see [0111.0.0.0] above.

**[0112.0.19.19]** The nucleic acid molecules used in the process according to the invention take the form of isolated nucleic acid sequences, which encode polypeptides with an activity of a polypeptide as indicated in Table II, column 3, lines 243 to 250 and 603 or having the sequence of a polypeptide as indicated in Table II, columns 5 and 7, lines 243 to 250 and 603 and conferring an increase in the ferulic acid or sinapic acid level.

**[0113.0.0.19] to [0120.0.0.19]** for the disclosure of the paragraphs [0113.0.0.19] to [0120.0.0.19] see paragraphs [0113.0.0.0] and [0120.0.0.0] above.

**[0121.0.19.19]** However, it is also possible to use artificial sequences, which differ in one or more bases from the nucleic acid sequences found in organisms, or in one or more amino acid molecules from polypeptide sequences found in organisms, in particular from the polypeptide sequences indicated in Table II, columns 5 or 7, lines 243 to 250 and 603 or the functional homologues thereof as described herein, preferably conferring above-mentioned activity, i.e. conferring a ferulic acid level increase after increasing the activity of the polypeptide sequences indicated in Table II, columns 5 or 7, lines 243, 244, 246, 247, 249 or conferring a sinapic acid level increase after increasing the activity of the polypeptide sequences indicated in Table II, columns 5 or 7, lines 245, 248 250 and 603.

**[0122.0.0.19] to [0127.0.0.19]** for the disclosure of the paragraphs [0122.0.0.19] to [0127.0.0.19] see paragraphs [0122.0.0.0] and [0127.0.0.0] above.

**[0128.0.19.19]** Synthetic oligonucleotide primers for the amplification, e.g. as the pairs indicated in Table III, column 7, lines 243 to 250 and 603, by means of polymerase chain reaction can be generated on the basis of a sequence shown herein, for example the sequence as indicated in Table I, columns 5 or 7, lines 243 to 250 and 603, resp. or the sequences derived from a sequences as indicated in Table II, columns 5 or 7, lines 243 to 250 and 603, resp.

**[0129.0.19.19]** Moreover, it is possible to identify conserved regions from various organisms by carrying out protein sequence alignments with the polypeptide used in the process of the invention, in particular with sequences of the polypeptide of the invention, from which conserved regions, and in turn, degenerate primers can be derived. Conserved region for the polypeptide of the invention are indicated in the alignments shown in the figures. Conserved regions are those, which show a very little variation in the amino acid in one particular position of several homologs from different origin. The consenus sequence shown in Table IV, column 7, lines 243 to 248, 250 and 603 is derived from said aligments.

**[0130.0.19.19]** for the disclosure of this paragraph see [0130.0.0.0].

**[0131.0.0.19] to [0138.0.0.19]** for the disclosure of the paragraphs [0131.0.0.19] to [0138.0.0.19] see paragraphs [0131.0.0.0] to [0138.0.0.0] above.

**[0139.0.19.19]** Polypeptides having above-mentioned activity, i.e. conferring the respective fine chemical level increase, derived from other organisms, can be encoded by other DNA sequences which hybridise to a sequence indicated in Table I, columns 5 or 7, lines 243, 244, 246, 247, 249, preferably of Table I B, columns 5 or 7, lines 243, 244, 246, 247, 249 for ferulic acid or indicated in Table I, columns 5 or 7, lines 245, 248, 250, 603, preferably of Table I B, columns 5 or 7, lines 245, 248, 250, 603 for sinapic under relaxed hybridization conditions and which code on expression for peptides having the respective fine chemical, i.e. ferulic acid or sinapic acid, resp., increasing-activity.

**[0140.0.0.19] to [0146.0.0.19]** for the disclosure of the paragraphs [0140.0.0.19] to [0146.0.0.19] see paragraphs [0140.0.0.0] and [0146.0.0.0] above.

**[0147.0.19.19]** Further, the nucleic acid molecule of the invention comprises a nucleic acid molecule, which is a complement of one of the nucleotide sequences of above mentioned nucleic acid molecules or a portion thereof. A nucleic acid molecule which is complementary to one of the nucleotide sequences indicated in Table I, columns 5 or 7, lines 243 to 250 and 603, preferably of Table I B, columns 5 or 7, lines 243 to 250 and 603 is one which is sufficiently complementary to one of said nucleotide sequences such that it can hybridise to one of said nucleotide sequences, thereby forming a stable duplex. Preferably, the hybridisation is performed under stringent hybrization conditions. However, a complement of one of the herein disclosed sequences is preferably a sequence complement thereto according to the base pairing of nucleic acid molecules well known to the skilled person. For example, the bases A and G undergo base pairing with the bases T and U or C, resp. and visa versa. Modifications of the bases can influence the base-pairing partner.

**[0148.0.19.19]** The nucleic acid molecule of the invention comprises a nucleotide sequence which is at least about 30%, 35%, 40% or 45%, preferably at least about 50%, 55%, 60% or 65%, more preferably at least about 70%, 80%, or 90%, and even more preferably at least about 95%, 97%, 98%, 99% or more homologous to a nucleotide sequence indicated in Table I, columns 5 or 7, lines 243 to 250 and 603, preferably of Table I B, columns 5 or 7, lines 243 to 250 and 603 or a portion thereof and preferably has above mentioned activity, in particular having a ferulic acid or sinapic acid increasing activity after increasing the activity or an activity of a product of a gene encoding said sequences or their homologs.

**[0149.0.19.19]** The nucleic acid molecule of the invention comprises a nucleotide sequence which hybridizes, preferably hybridizes under stringent conditions as defined herein, to one of the nucleotide sequences indicated in Table I, columns 5 or 7, lines 243 to 250 and 603, preferably of Table I B, columns 5 or 7, lines 243 to 250 and 603 or a portion thereof and encodes a protein having above-mentioned activity, e.g. conferring a of ferulic acid or sinapic acid increase, resp., and optionally, the activity of protein indicated in Table II, column 5, lines 243 to 250 and 603, preferably of Table II B, columns 5 or 7, lines 243 to 250 and 603.

**[00149.1.19.19]** Optionally, in one embodiment, the nucleotide sequence, which hybridises to one of the nucleotide sequences indicated in Table I, columns 5 or 7, lines 243 to 250 and 603, preferably of Table I B, columns 5 or 7, lines 243 to 250 and 603 has further one or more of the activities annotated or known for a protein as indicated in Table II, column 3, lines 243 to 250 and 603, preferably of Table II B, columns 5 or 7, lines 243 to 250 and 603.

**[0150.0.19.19]** Moreover, the nucleic acid molecule of the invention can comprise only a portion of the coding region of one of the sequences indicated in Table I, columns 5 or 7, lines 243 to 250 and 603, preferably of Table I B, columns 5 or 7, lines 243 to 250 and 603 for example a fragment which can be used as a probe or primer or a fragment encoding a biologically active portion of the polypeptide of the present invention or of a polypeptide used in the process of the present invention, i.e. having above-mentioned activity, e.g.conferring an increase of ferulic acid or sinapic acid , resp., if its activity is increased. The nucleotide sequences determined from the cloning of the present protein-according-to-the-invention-encoding gene allows for the generation of probes and primers designed for use in identifying and/or cloning its homologues in other cell types and organisms. The probe/primer typically comprises substantially purified oligonucleotide. The oligonucleotide typically comprises a region of nucleotide sequence that hybridizes under stringent conditions to at least about 12, 15 preferably about 20 or 25, more preferably about 40, 50 or 75 consecutive nucleotides of a sense strand of one of the sequences set forth, e.g., as indicated in Table I, columns 5 or 7, lines 243 to 250 and 603, an anti-sense sequence of one of the sequences, e.g., as indicated in Table I, columns 5 or 7, lines 243 to 250 and 603, or naturally occurring mutants thereof. Primers based on a nucleotide of invention can be used in PCR reactions to clone homologues of the polypeptide of the invention or of the polypeptide used in the process of the invention, e.g. as the primers described in the examples of the present invention, e.g. as shown in the examples. A PCR with the primer pairs indicated in Table III, column 7, lines 243 to 250 and 603 will result in a fragment of a polynucleotide sequence as indicated in Table I, columns 5 or 7, lines 243 to 250 and 603 or its gene product. Preferred is Table II B, column 7, lines 243 to 250 and 603.

**[0151.0.0.19]:** for the disclosure of this paragraph see [0151.0.0.0] above.

**[0152.0.19.19]** The nucleic acid molecule of the invention encodes a polypeptide or portion thereof which includes an amino acid sequence which is sufficiently homologous to an amino acid sequence as indicated in Table II, columns 5 or 7, lines 243 to 250 and 603 such that the protein or portion thereof maintains the ability to participate in the respective fine chemical production, in particular a ferulic acid (lines 243, 244, 246, 247, 249) or sinapic acid (lines 245, 248, 250, 603) increasing activity as mentioned above or as described in the examples in plants or microorganisms is comprised.

**[0153.0.19.19]** As used herein, the language "sufficiently homologous" refers to proteins or portions thereof which have amino acid sequences which include a minimum number of identical or equivalent amino acid residues (e.g., an amino acid residue which has a similar side chain as an amino acid residue in one of the sequences of the polypeptide of the present invention) to an amino acid sequence as indicated in Table II, columns 5 or 7, lines 243 to 250 and 603 such that the protein or portion thereof is able to participate in the increase of the respective fine chemical production. In one embodiment, a protein or portion thereof as indicated in Table II, columns 5 or 7, lines 243 to 250 and 603 has for example an activity of a polypeptide indicated in Table II, column 3, lines 243 to 250 and 603.

**[0154.0.19.19]** In one embodiment, the nucleic acid molecule of the present invention comprises a nucleic acid that encodes a portion of the protein of the present invention. The protein is at least about 30%, 35%, 40%, 45% or 50%, preferably at least about 55%, 60%, 65% or 70%, and more preferably at least about 75%, 80%, 85%, 90%, 91 %, 92%, 93% or 94% and most preferably at least about 95%, 97%, 98%, 99% or more homologous to an entire amino acid sequence as indicated in Table II, columns 5 or 7, lines 243 to 250 and 603 and has above-mentioned activity, e.g.conferring preferably the increase of the respective fine chemical.

**[0155.0.0.19] and [0156.0.0.19]** for the disclosure of the paragraphs [0155.0.0.19] and [0156.0.0.19] see paragraphs [0155.0.0.0] and [0156.0.0.0] above.

**[0157.0.19.19]** The invention further relates to nucleic acid molecules that differ from one of the nucleotide sequences as indicated in Table I, columns 5 or 7, lines 243 to 250 and 603 (and portions thereof) due to degeneracy of the genetic code and thus encode a polypeptide of the present invention, in particular a polypeptide having above mentioned activity, e.g. conferring an increase in the respective fine chemical in a organism, e.g. as polypeptides comprising the sequence as indicated in Table IV, column 7, lines 243 to 248, 250 and 603 or as polypeptides depicted in Table II, columns 5 or 7, lines 243 to 250 and 603 or the functional homologues. Advantageously, the nucleic acid molecule of the invention comprises, or in an other embodiment has, a nucleotide sequence encoding a protein comprising, or in an other embodiment having, an amino acid sequence of a consensus sequences as indicated in Table IV, column 7, lines 243 to 248, 250 and 603 or of the polypeptide as indicated in Table II, columns 5 or 7, lines 243 to 250 and 603, resp., or the functional homologues. In a still further embodiment, the nucleic acid molecule of the invention encodes a full length protein which is substantially homologous to an amino acid sequence comprising a consensus sequence as indicated in Table IV, column 7, lines 243 to 248, 250 and 603 or of a polypeptide as indicated in Table II, columns 5 or 7, lines 243 to 250 and 603 or the functional homologues. However, in a preferred embodiment, the nucleic acid molecule of the present invention does not consist of a sequence as indicated in Table I, columns 5 or 7, lines 243 to 250 and 603, resp., preferably as indicated in Table I A, columns 5 or 7, lines 243 to 250 and/or 603. Preferably the nucleic acid molecule of the invention is a functional homologue or identical to a nucleic acid molecule indicated in Table I B, columns 5 or 7, lines 243 to 250 and/or 603.

**[0158.0.0.19] to [0160.0.0.19]** for the disclosure of the paragraphs [0158.0.0.19] to [0160.0.0.19] see paragraphs [0158.0.0.0] to [0160.0.0.0] above.

**[0161.0.19.19]** Accordingly, in another embodiment, a nucleic acid molecule of the invention is at least 15, 20, 25 or 30 nucleotides in length. Preferably, it hybridizes under stringent conditions to a nucleic acid molecule comprising a nucleotide sequence of the nucleic acid molecule of the present invention or used in the process of the present invention, e.g. comprising a sequence as indicated in Table I, columns 5 or 7, lines 243 to 250 and 603. The nucleic acid molecule is preferably at least 20, 30, 50, 100, 250 or more nucleotides in length.

**[0162.0.0.19]** for the disclosure of this paragraph see paragraph [0162.0.0.0] above.

**[0163.0.19.19]** Preferably, a nucleic acid molecule of the invention that hybridizes under stringent conditions to a sequence as indicated in Table I, columns 5 or 7, lines 243 to 250 and 603 corresponds to a naturally-occurring nucleic acid molecule of the invention. As used herein, a "naturally-occurring" nucleic acid molecule refers to an RNA or DNA molecule having a nucleotide sequence that occurs in nature (e.g., encodes a natural protein). Preferably, the nucleic acid molecule encodes a natural protein having above-mentioned activity, e.g. conferring the increase of the amount of the respective fine chemical in a organism or a part thereof, e.g. a tissue, a cell, or a compartment of a cell, after increasing the expression or activity thereof or the activity of a protein of the invention or used in the process of the invention.

**[0164.0.0.19]** for the disclosure of this paragraph see paragraph [0164.0.0.0] above.

**[0165.0.19.19]** For example, nucleotide substitutions leading to amino acid substitutions at "non-essential" amino acid residues can be made in a sequence of the nucleic acid molecule of the invention or used in the process of the invention, e.g. as indicated in Table I, columns 5 or 7, lines 243 to 250 and 603, resp.

**[0166.0.0.19] and [0167.0.0.19]** for the disclosure of the paragraphs [0166.0.0.19] and [0167.0.0.19] see paragraphs [0166.0.0.0] and [0167.0.0.0] above.

**[0168.0.19.19]** Accordingly, the invention relates to nucleic acid molecules encoding a polypeptide having above-mentioned activity, e.g. conferring an increase in the respective fine chemical in organisms or parts thereof that contain changes in amino acid residues that are not essential for said activity. Such polypeptides differ in amino acid sequence from a sequence contained in a sequence as indicated in Table II, columns 5 or 7, lines 243 to 250 and 603, resp., yet retain said activity described herein. The nucleic acid molecule can comprise a nucleotide sequence encoding a polypeptide, wherein the polypeptide comprises an amino acid sequence at least about 50% identical to an amino acid sequence as indicated in Table II, columns 5 or 7, lines 243 to 250 and 603, resp., and is capable of participation in the increase of production of the respective fine chemical after increasing its activity, e.g. its expression. Preferably, the protein encoded by the nucleic acid molecule is at least about 60% identical to a sequence as indicated in Table II, columns 5 or 7, lines 243 to 250 and 603, resp., more preferably at least about 70% identical to one of the sequences as indicated in Table II, columns 5 or 7, lines 243 to 250 and 603, resp., even more preferably at least about 80%, 90%, 95% homologous to a sequence as indicated in Table II, columns 5 or 7, lines 243 to 250 and 603, resp., and most preferably at least about 96%, 97%, 98%, or 99% identical to the sequence as indicated in Table II, columns 5 or 7, lines 243 to 250 and 603.
Accordingly, the invention relates to nucleic acid molecules encoding a polypeptide having above-mentioned activity, e.g. conferring an increase in the the respective fine chemical in an organisms or parts thereof that contain changes in amino acid residues that are not essential for said activity. Such polypeptides differ in amino acid sequence from a sequence contained in a sequence as indicated in Table II, columns 5 or 7, lines 243 to 250 and 603, preferably of Table II B, column 7, lines 243 to 250 and 603 yet retain said activity described herein. The nucleic acid molecule can comprise a nucleotide sequence encoding a polypeptide, wherein the polypeptide comprises an amino acid sequence at least about 50% identical to an amino acid sequence as indicated in Table II, columns 5 or 7, lines 243 to 250 and 603, preferably of Table II B, column 7, lines 243 to 250 and 603 and is capable of participation in the increase of production of the respective fine chemical after increasing its activity, e.g. its expression. Preferably, the protein encoded by the nucleic acid molecule is at least about 60% identical to a sequence as indicated in Table II, columns 5 or 7, lines 243 to 250 and 603, preferably of Table II B, column 7, lines 243 to 250 and 603, more preferably at least about 70% identical to one of the sequences as indicated in Table II, columns 5 or 7, lines 243 to 250 and 603, preferably of Table II B, column 7, lines 243 to 250 and 603, even more preferably at least about 80%, 90%, or 95% homologous to a sequence as indicated in Table II, columns 5 or 7, lines 243 to 250 and 603, preferably of Table II B, column 7, lines 243 to 250 and 603, and most preferably at least about 96%, 97%, 98%, or 99% identical to the sequence as indicated in Table II, columns 5 or 7, lines 243 to 250 and 603, preferably of Table II B, column 7, lines 243 to 250 and 603.

**[0169.0.0.19] to [0172.0.0.19]** for the disclosure of the paragraphs [0169.0.0.19] to [0172.0.0.19] see paragraphs [0169.0.0.0] to [0172.0.0.0] above.

**[0173.0.19.19]** For example a sequence, which has 80% homology with sequence SEQ ID NO: 24071 at the nucleic acid level is understood as meaning a sequence which, upon comparison with the sequence SEQ ID NO: 24071 by the above Gap program algorithm with the above parameter set, has a 80% homology.

**[0174.0.0.19]:** for the disclosure of this paragraph see paragraph [0174.0.0.0] above.

**[0175.0.19.19]** For example a sequence which has a 80% homology with sequence SEQ ID NO: 24072 at the protein level is understood as meaning a sequence which, upon comparison with the sequence SEQ ID NO: 24072 by the above program algorithm with the above parameter set, has a 80% homology.

**[0176.0.19.19]** Functional equivalents derived from one of the polypeptides as indicated in Table II, columns 5 or 7, lines 243 to 250 and 603, resp., according to the invention by substitution, insertion or deletion have at least 30%, 35%, 40%, 45% or 50%, preferably at least 55%, 60%, 65% or 70% by preference at least 80%, especially preferably at least 85% or 90%, 91%, 92%, 93% or 94%, very especially preferably at least 95%, 97%, 98% or 99% homology with one of the polypeptides as indicated in Table II, columns 5 or 7, lines 243 to 250 and 603, resp., according to the invention and are distinguished by essentially the same properties as a polypeptide as indicated in Table II, columns 5 or 7, lines 243 to 250 and 603, resp.

**[0177.0.19.19]** Functional equivalents derived from a nucleic acid sequence as indicated in Table I, columns 5 or 7, lines 243 to 250 and 603, resp., according to the invention by substitution, insertion or deletion have at least 30%, 35%, 40%, 45% or 50%, preferably at least 55%, 60%, 65% or 70% by preference at least 80%, especially preferably at least 85% or 90%, 91%, 92%, 93% or 94%, very especially preferably at least 95%, 97%, 98% or 99% homology with one of the polypeptides as indicated in Table II, columns 5 or 7, lines 243 to 250 and 603, resp., according to the invention and encode polypeptides having essentially the same properties as a polypeptide as indicated in Table II, columns 5 or 7, lines 243 to 250 and 603, resp.

**[0178.0.0.19]** for the disclosure of this paragraph see [0178.0.0.0] above.

**[0179.0.19.19]** A nucleic acid molecule encoding a homologous to a protein sequence as indicated in Table II, columns 5 or 7, lines 243 to 250 and 603, resp., can be created by introducing one or more nucleotide substitutions, additions or deletions into a nucleotide sequence of the nucleic acid molecule of the present invention, in particular as indicated in Table I, columns 5 or 7, lines 243 to 250 and 603, resp., such that one or more amino acid substitutions, additions or deletions are introduced into the encoded protein. Mutations can be introduced into the encoding sequences as indicated in Table I, columns 5 or 7, lines 243 to 250 and 603, resp., by standard techniques, such as site-directed mutagenesis and PCR-mediated mutagenesis.

**[0180.0.0.19] to [0183.0.0.19]** for the disclosure of the paragraphs [0180.0.0.19] to [0183.0.0.19] see paragraphs [0180.0.0.0] to [0183.0.0.0] above.

**[0184.0.19.19]** Homologues of the nucleic acid sequences used, with a sequence as indicated in Table I, columns 5 or 7, lines 243 to 250 and 603, resp., or of the nucleic acid sequences derived from a sequences as indicated in Table II, columns 5 or 7, lines 243 to 250 and 603, preferably of Table II B, column 7, lines 243 to 250 and 603, resp., comprise also allelic variants with at least approximately 30%, 35%, 40% or 45% homology, by preference at least approximately 50%, 60% or 70%, more preferably at least approximately 90%, 91%, 92%, 93%, 94% or 95% and even more preferably at least approximately 96%, 97%, 98%, 99% or more homology with one of the nucleotide sequences shown or the abovementioned derived nucleic acid sequences or their homologues, derivatives or analogues or parts of these. Allelic variants encompass in particular functional variants which can be obtained by deletion, insertion or substitution of nucleotides from the sequences shown, preferably from a sequence as indicated in Table I, columns 5 or 7, lines 243 to 250 and 603, resp., or from the derived nucleic acid sequences, the intention being, however, that the enzyme activity or the biological activity of the resulting proteins synthesized is advantageously retained or increased.

**[0185.0.19.19]** In one embodiment of the present invention, the nucleic acid molecule of the invention or used in the process of the invention comprises one or more sequences as indicated in Table I, columns 5 or 7, lines 243 to 250 and 603, preferably of Table I B, column 7, lines 243 to 250 and 603, resp. In one embodiment, it is preferred that the nucleic acid molecule comprises as little as possible other nucleotides not shown in any one of sequences as indicated in Table I, columns 5 or 7, lines 243 to 250 and 603, preferably of Table I B, column 7, lines 243 to 250 and 603, resp. In one embodiment, the nucleic acid molecule comprises less than 500, 400, 300, 200, 100, 90, 80, 70, 60, 50 or 40 further nucleotides. In a further embodiment, the nucleic acid molecule comprises less than 30, 20 or 10 further nucleotides. In one embodiment, a nucleic acid molecule used in the process of the invention is identical to a sequence as indicated in Table I, columns 5 or 7, lines 243 to 250 and 603, preferably of Table I B, column 7, lines 243 to 250 and 603, resp.

**[0186.0.19.19]** Also preferred is that one or more nucleic acid molecule(s) used in the process of the invention encodes a polypeptide comprising a sequence as indicated in Table II, columns 5 or 7, lines 243 to 250 and 603, preferably of Table II B, column 7, lines 243 to 250 and 603, resp. In one embodiment, the nucleic acid molecule encodes less than 150, 130, 100, 80, 60, 50, 40 or 30 further amino acids. In a further embodiment, the encoded polypeptide comprises less than 20, 15, 10, 9, 8, 7, 6 or 5 further amino acids. In one embodiment, the encoded polypeptide used in the process of the invention is identical to the sequences as indicated in Table II, columns 5 or 7, lines 243 to 250 and 603, preferably of Table II B, column 7, lines 243 to 250 and 603, resp.

**[0187.0.19.19]** In one embodiment, a nucleic acid molecule of the invention or used in the process encodes a polypeptide comprising the sequence as indicated in Table II, columns 5 or 7, lines 243 to 250 and 603, preferably of Table II B, column 7, lines 243 to 250 and 603, resp., comprises less than 100 further nucleotides. In a further embodiment, said nucleic acid molecule comprises less than 30 further nucleotides. In one embodiment, the nucleic acid molecule used in the process is identical to a coding sequence encoding a sequences as indicated in Table II, columns 5 or 7, lines 243 to 250 and 603, preferably of Table II B, column 7, lines 243 to 250 and 603, resp.

**[0188.0.19.19]** Polypeptides (= proteins), which still have the essential enzymatic activity of the polypeptide of the present invention conferring an increase of the respective fine chemical i.e. whose activity is essentially not reduced, are polypeptides with at least 10% or 20%, by preference 30% or 40%, especially preferably 50% or 60%, very especially preferably 80% or 90 or more of the wild type biological activity or enzyme activity, advantageously, the activity is essentially not reduced in comparison with the activity of a polypeptide as indicated in Table II, columns 5 or 7, lines 243 to 250 and 603, resp., and is expressed under identical conditions.
In one embodiment, the polypeptide of the invention is a homolog consisting or or comprising the sequence as indicated in Table II B, column 7, lines 243 to 250 and 603,

**[0189.0.19.19]** Homologues of a sequences as indicated in Table I, columns 5 or 7, lines 243 to 250 and 603, resp., or of a derived sequences as indicated in Table II, columns 5 or 7, lines 243 to 250 and 603, resp., also mean truncated sequences, cDNA, single-stranded DNA or RNA of the coding and noncoding DNA sequence. Homologues of said sequences are also understood as meaning derivatives, which comprise noncoding regions such as, for example, UTRs, terminators, enhancers or promoter variants. The promoters upstream of the nucleotide sequences stated can be modified by one or more nucleotide substitution(s), insertion(s) and/or deletion(s) without, however, interfering with the functionality or activity either of the promoters, the open reading frame (=ORF) or with the 3'-regulatory region such as terminators or other 3'regulatory regions, which are far away from the ORF. It is furthermore possible that the activity of the promoters is increased by modification of their sequence, or that they are replaced completely by more active promoters, even promoters from heterologous organisms. Appropriate promoters are known to the person skilled in the art and are mentioned herein below.

**[0190.0.0.19], [0191.0.0.19], [00191.1.0.19] and [0192.0.0.19] to [0203.0.0.19]** for the disclosure of the paragraphs [0190.0.0.19], [0191.0.0.19], [0191.1.0.19] and [0192.0.0.19] to [0203.0.0.19] see paragraphs [0190.0.0.0], [0191.0.0.0], [0191.1.0.0] and [0192.0.0.0] to [0203.0.0.0] above.

**[0204.0.19.19]** Accordingly, in one embodiment, the invention relates to a nucleic acid molecule, which comprises a nucleic acid molecule selected from the group consisting of:
a) nucleic acid molecule encoding, preferably at least the mature form, of a polypeptide as indicated in Table II, columns 5 or 7, lines 243 to 250 and 603, preferably of Table II B, column 7, lines 243 to 250 and 603, resp.; or a fragment thereof conferring an increase in the amount of the respective fine chemical, i.e. ferulic acid (lines 243, 244, 246, 247, 249) or sinapic acid (lines 245, 248, 250, 603), resp., in an organism or a part thereof
b) nucleic acid molecule comprising, preferably at least the mature form, of a nucleic acid molecule as indicated in Table I, columns 5 or 7, lines 243 to 250 and 603, preferably of Table I B, column 7, lines 243 to 250 and 603, resp., or a fragment thereof conferring an increase in the amount of the respective fine chemical in an organism or a part thereof;
c) nucleic acid molecule whose sequence can be deduced from a polypeptide sequence encoded by a nucleic acid molecule of (a) or (b) as result of the degeneracy of the genetic code and conferring an increase in the amount of the respective fine chemical in an organism or a part thereof;
d) nucleic acid molecule encoding a polypeptide whose sequence has at least 50% identity with the amino acid sequence of the polypeptide encoded by the nucleic acid molecule of (a) to (c) and conferring an increase in the amount of the respective fine chemical in an organism or a part thereof;
e) nucleic acid molecule which hybridizes with a nucleic acid molecule of (a) to (c) under under stringent hybridisation conditions and conferring an increase in the amount of the respective fine chemical in an organism or a part thereof;
f) nucleic acid molecule encoding a polypeptide, the polypeptide being derived by substituting, deleting and/or adding one or more amino acids of the amino acid sequence of the polypeptide encoded by the nucleic acid molecules (a) to (d), preferably to (a) to (c), and conferring an increase in the amount of the respective fine chemical in an organism or a part thereof;
g) nucleic acid molecule encoding a fragment or an epitope of a polypeptide which is encoded by one of the nucleic acid molecules of (a) to (e), preferably to (a) to (c) and conferring an increase in the amount of the respective fine chemical in an organism or a part thereof;
h) nucleic acid molecule comprising a nucleic acid molecule which is obtained by amplifying a cDNA library or a genomic library using primers or primer pairs as indicated in Table III, column 7, lines 243 to 250 and 603 and conferring an increase in the amount of the respective fine chemical, i.e. ferulic acid (lines 243, 244, 246, 247, 249) or sinapic acid (lines 245, 248, 250, 603), resp., in an organism or a part thereof;
i) nucleic acid molecule encoding a polypeptide which is isolated, e.g. from a expression library, with the aid of monoclonal antibodies against a polypeptide encoded by one of the nucleic acid molecules of (a) to (g), preferably to (a) to (c) and conferring an increase in the amount of the respective fine chemical in an organism or a part thereof;
j) nucleic acid molecule which encodes a polypeptide comprising a consensus sequence as indicated in Table IV, column 7, lines 243 to 248, 250 and 603 and conferring an increase in the amount of the respective fine chemical, i.e. ferulic acid (lines 243, 244, 246, 247, 249) or sinapic acid (lines 245, 248, 250, 603), resp., in an organism or a part thereof;
k) nucleic acid molecule encoding the amino acid sequence of a polypeptide encoding a domaine of a polypeptide as indicated in Table II, columns 5 or 7, lines 243 to 250 and 603, preferably of Table II B, column 7, lines 243 to 250 and 603, resp., and conferring an increase in the amount of the respective fine chemical, i.e. ferulic acid (lines 243, 244, 246, 247, 249) or sinapic acid (lines 245, 248, 250, 603), resp., in an organism or a part thereof; and
l) nucleic acid molecule which is obtainable by screening a suitable nucleic acid library under stringent hybridization conditions with a probe comprising one of the sequences of the nucleic acid molecule of (a) to (k) or with a fragment of at least 15 nt, preferably 20 nt, 30 nt, 50 nt, 100 nt, 200 nt or 500 nt of the nucleic acid molecule characterized in (a) to (h) or of a nucleic acid molecule as indicated in Table I, columns 5 or 7, lines 243 to 250 and 603, resp., or a nucleic acid molecule encoding, preferably at least the mature form of, a polypeptide as indicated in Table II, columns 5 or 7, lines 243 to 250 and 603, resp., and conferring an increase in the amount of the respective fine chemical in an organism or a part thereof; or which encompasses a sequence which is complementary thereto;
whereby, preferably, the nucleic acid molecule according to (a) to (I) distinguishes over a sequence as indicated in Table IA or IB, columns 5 or 7, lines 243 to 250 and 603, resp., by one or more nucleotides. In one embodiment, the nucleic acid molecule of the invention does not consist of the sequence as indicated in Table IA or IB, columns 5 or 7, lines 243 to 250 and 603, resp. In an other embodiment, the nucleic acid molecule of the present invention is at least 30 % identical and less than 100%, 99,999%, 99,99%, 99,9% or 99% identical to a sequence as indicated in Table IA or IB, columns 5 or 7, lines 243 to 250 and 603, resp. In a further embodiment the nucleic acid molecule does not encode a polypeptide sequence as indicated in Table IIA or IIB, columns 5 or 7, lines 243 to 250 and 603, resp. Accordingly, in one embodiment, the nucleic acid molecule of the present invention encodes in one embodiment a polypeptide which differs at least in one or more amino acids from a polypeptide indicated in Table IIA or IIB, columns 5 or 7, lines 243 to 250 and 603does not encode a protein of a sequence as indicated in Table IIA or IIB, columns 5 or 7, lines 243 to 250 and 603. Accordingly, in one embodiment, the protein encoded by a sequences of a nucleic acid accoriding to (a) to (I) does not consist of a sequence as indicated in Table IIA or IIB, columns 5 or 7, lines 243 to 250 and 603. In a further embodiment, the protein of the present invention is at least 30 % identical to a protein sequence indicated in Table IIA or IIB, columns 5 or 7, lines 243 to 250 and 603 and less than 100%, preferably less than 99,999%, 99,99% or 99,9%, more preferably less than 99%, 98%, 97%, 96% or 95% identical to a sequence as indicated in Table IIA or IIB, columns 5 or 7, lines 243 to 250 and 603.

**[0205.0.0.19] and [0206.0.0.19]** for the disclosure of the paragraphs [0205.0.0.19] and [0206.0.0.19] see paragraphs [0205.0.0.0] and [0206.0.0.0] above.

**[0207.0.19.19]** As described herein, the nucleic acid construct can also comprise further genes, which are to be introduced into the organisms or cells. It is possible and advantageous to introduce into, and express in, the host organisms regulatory genes such as genes for inductors, repressors or enzymes, which, owing to their enzymatic activity, engage in the regulation of one or more genes of a biosynthetic pathway. These genes can be of heterologous or homologous origin. Moreover, further biosynthesis genes may advantageously be present, or else these genes may be located on one or more further nucleic acid constructs. Genes, which are advantageously employed as biosynthesis genes, are genes of the glutamic acid metabolism, the phosphoenolpyruvate metabolism, the amino acid metabolism, of glycolysis, of the tricarboxylic acid metabolism or their combinations. As described herein, regulator sequences or factors can have a positive effect on preferably the gene expression of the genes introduced, thus increasing it. Thus, an enhancement of the regulator elements may advantageously take place at the transcriptional level by using strong transcription signals such as promoters and/or enhancers. In addition, however, an enhancement of translation is also possible, for example by increasing mRNA stability or by inserting a translation enhancer sequence.

**[0208.0.0.19] to [0226.0.0.19]** for the disclosure of the paragraphs [0208.0.0.19] to [0226.0.0.19] see paragraphs [0208.0.0.0] to [0226.0.0.0] above.

**[0227.0.19.19]** The abovementioned nucleic acid molecules can be cloned into the nucleic acid constructs or vectors according to the invention in combination together with further genes, or else different genes are introduced by transforming several nucleic acid constructs or vectors (including plasmids) into a host cell, advantageously into a plant cell or a microorganisms.
In addition to a sequence indicated in Table I, columns 5 or 7, lines 243 to 250 and 603 or its derivatives, it is advantageous to express and/or mutate further genes in the organisms. Especially advantageously, additionally at least one further gene of the glutamic acid or phosphoenolpyruvate metabolic pathway, is expressed in the organisms such as plants or microorganisms. It is also possible that the regulation of the natural genes has been modified advantageously so that the gene and/or its gene product is no longer subject to the regulatory mechanisms which exist in the organisms. This leads to an increased synthesis of the fine chemicals desired since, for example, feedback regulations no longer exist to the same extent or not at all. In addition it might be advantageously to combine one or more of the sequences indicated in Table I, columns 5 or 7, lines 243 to 250 and 603, resp., with genes which generally support or enhances to growth or yield of the target organismen, for example genes which lead to faster growth rate of microorganisms or genes which produces stress-, pathogen, or herbicide resistant plants.

**[0228.0.19.19]** %

**[0229.0.19.19]** Further advantageous nucleic acid sequences which can be expressed in combination with the sequences indicated in Table I, columns 5 or 7, lines 243 to 250 and 603 used in the process and/or the abovementioned biosynthesis genes are the sequences encoding further genes of the aromatic amino acid pathway, such as tryptophan, phenylalanine or tyrosine. These genes can lead to an increased synthesis of the essential amino acids tryptophan, phenylalanine or tyrosine.

**[0230.0.0.19]** for the disclosure of this paragraph see paragraph [0230.0.0.0] above.

**[0231.0.19.19]** In a further advantageous embodiment of the process of the invention, the organisms used in the process are those in which simultaneously a ferulic acid or sinapic acid degrading protein is attenuated, in particular by reducing the rate of expression of the corresponding gene. A person skilled in the art knows for example, that the inhibition or repression of a ferulic acid or sinapic acid degrading enzyme will result in an increased ferulic acid and/or sinapic acid accumulation in the plant.

**[0232.0.0.19] to [0276.0.0.19]** for the disclosure of the paragraphs [0232.0.0.19] to [0276.0.0.19] see paragraphs [0232.0.0.0] to [0276.0.0.0] above.

**[0277.0.19.19]** The respective fine chemical produced can be isolated from the organism by methods with which the skilled worker are familiar, for example via extraction, salt precipitation, and/or different chromatography methods. The process according to the invention can be conducted batchwise, semibatchwise or continuously.

**[0278.0.0.19] to [0282.0.0.19]** for the disclosure of the paragraphs [0278.0.0.19] to [0282.0.0.19] see paragraphs [0278.0.0.0] to [0282.0.0.0] above.

**[0283.0.19.19]** Moreover, native polypeptide conferring the increase of the respective fine chemical in an organism or part thereof can be isolated from cells, for example using the antibody of the present invention as described below, e.g. an antibody against a protein as indicated in Table II, column 3, lines 243 to 250 and 603 , resp., or an antibody against a polypeptide as indicated in Table II, columns 5 or 7, lines 243 to 250 and 603, resp., which can be produced by standard techniques utilizing the polypeptid of the present invention or fragment thereof. Preferred are monoclonal antibodies.

**[0284.0.0.19]** for the disclosure of this paragraph see [0284.0.0.0] above.

**[0285.0.19.19]** In one embodiment, the present invention relates to a polypeptide having a sequence as indicated in Table II, columns 5 or 7, lines 243 to 250 and 603, resp., or as coded by a nucleic acid molecule as indicated in Table I, columns 5 or 7, lines 243 to 250 and 603, resp., or functional homologues thereof.

**[0286.0.19.19]** In one advantageous embodiment, in the method of the present invention the activity of a polypeptide is increased which comprises or consists of a consensus sequence as indicated in Table IV, column 7, lines 243 to 248, 250 and 603 and in one another embodiment, the present invention relates to a polypeptide comprising or consisting of a consensus sequence as indicated in Table IV, column 7, lines 243 to 248, 250 and 603 whereby 20 or less, preferably 15 or 10, preferably 9, 8, 7, or 6, more preferred 5 or 4, even more preferred 3, even more preferred 2, even more preferred 1, most preferred 0 of the amino acids positions indicated can be replaced by any amino acid.

**[0287.0.0.19] to [0290.0.0.19]** for the disclosure of the paragraphs [0287.0.0.19] to [0290.0.0.19] see paragraphs [0287.0.0.0] to [0290.0.0.0] above.

**[0291.0.19.19]** In one advantageous embodiment, the method of the present invention comprises the increasing of a polypeptide comprising or consisting of plant or microorganism specific consensus sequences.
In one embodiment, said polypeptide of the invention distinguishes over a sequence as indicated in Table IIA or IIB, columns 5 or 7, lines 243 to 250 and 603, resp., by one or more amino acids. In one embodiment, polypeptide distinguishes from a sequence as indicated in Table IIA or IIB, columns 5 or 7, lines 243 to 250 and 603, resp., by more than 5, 6, 7, 8 or 9 amino acids, preferably by more than 10, 15, 20, 25 or 30 amino acids, even more preferred are more than 40, 50, or 60 amino acids and, preferably, the sequence of the polypeptide of the invention distinguishes from a sequence as indicated in Table IIA or IIB, columns 5 or 7, lines 243 to 250 and 603, resp., by not more than 80% or 70% of the amino acids, preferably not more than 60% or 50%, more preferred not more than 40% or 30%, even more preferred not more than 20% or 10%. In an other embodiment, said polypeptide of the invention does not consist of a sequence as indicated in Table IIA or IIB, columns 5 or 7, lines 243 to 250 and 603.

**[0292.0.0.19]** for the disclosure of this paragraph see [0292.0.0.0] above.

**[0293.0.19.19]** In one embodiment, the invention relates to a polypeptide conferring an increase in the fine chemical in an organism or part being encoded by the nucleic acid molecule of the invention or by a nucleic acid molecule used in the process of the invention. In one embodiment, the polypeptide of the invention has a sequence which distinguishes from a sequence as indicated in Table IIA or IIB, columns 5 or 7, lines 243 to 250 and 603, resp., by one or more amino acids. In an other embodiment, said polypeptide of the invention does not consist of the sequence as indicated in Table IIA or IIB, columns 5 or 7, lines 243 to 250 and 603, resp. In a further embodiment, said polypeptide of the present invention is less than 100%, 99,999%, 99,99%, 99,9% or 99% identical. In one embodiment, said polypeptide does not consist of the sequence encoded by a nucleic acid molecules as indicated in Table IA or IB, columns 5 or 7, lines 243 to 250 and 603, resp.

**[0294.0.19.19]** In one embodiment, the present invention relates to a polypeptide having an activity of a protein as indicated in Table IIA or IIB, column 3, lines 243 to 250 and 603, resp., which distinguishes over a sequence as indicated in Table IIA or IIB, columns 5 or 7, lines 243 to 250 and 603, resp., by one or more amino acids, preferably by more than 5, 6, 7, 8 or 9 amino acids, preferably by more than 10, 15, 20, 25 or 30 amino acids, even more preferred are more than 40, 50, or 60 amino acids but even more preferred by less than 70% of the amino acids, more preferred by less than 50%, even more preferred my less than 30% or 25%, more preferred are 20% or 15%, even more preferred are less than 10%.

**[0295.0.0.19] to [0297.0.0.19]** for the disclosure of the paragraphs [0295.0.0.19] to [0297.0.0.19] see paragraphs [0295.0.0.0] to [0297.0.0.0] above.

**[00297.1.0.19]** Non-polypeptide of the invention-chemicals are e.g. polypeptides having not the activity and/or the amino acid sequence of a polypeptide indicated in Table II, columns 3, 5 or 7, lines 243 to 250 and 603.

**[0298.0.19.19]** A polypeptide of the invention can participate in the process of the present invention. The polypeptide or a portion thereof comprises preferably an amino acid sequence which is sufficiently homologous to an amino acid sequence as indicated in Table II, columns 5 or 7, lines 243 to 250 and 603, resp. The portion of the protein is preferably a biologically active portion as described herein. Preferably, the polypeptide used in the process of the invention has an amino acid sequence identical to a sequence as indicated in Table II, columns 5 or 7, lines 243 to 250 and 603, resp.

**[0299.0.19.19]** Further, the polypeptide can have an amino acid sequence which is encoded by a nucleotide sequence which hybridizes, preferably hybridizes under stringent conditions as described above, to a nucleotide sequence of the nucleic acid molecule of the present invention. Accordingly, the polypeptide has an amino acid sequence which is encoded by a nucleotide sequence that is at least about 35%, 40%, 45%, 50%, 55%, 60%, 65% or 70%, preferably at least about 75%, 80%, 85% or 90, and more preferably at least about 91%, 92%, 93%, 94% or 95%, and even more preferably at least about 96%, 97%, 98%, 99% or more homologous to one of the nucleotide sequence as indicated in Table I, columns 5 or 7, lines 243 to 250 and 603, resp. The preferred polypeptide of the present invention preferably possesses at least one of the activities according to the invention and described herein. A preferred polypeptide of the present invention includes an amino acid sequence encoded by a nucleotide sequence which hybridizes, preferably hybridizes under stringent conditions, to a nucleotide sequence as indicated in Table I, columns 5 or 7, lines 243 to 250 and 603, resp., or which is homologous thereto, as defined above.

**[0300.0.19.19]** Accordingly the polypeptide of the present invention can vary from a sequence as indicated in Table II, columns 5 or 7, lines 243 to 250 and 603, resp., in amino acid sequence due to natural variation or mutagenesis, as described in detail herein. Accordingly, the polypeptide comprise an amino acid sequence which is at least about 35%, 40%, 45%, 50%, 55%, 60%, 65% or 70%, preferably at least about 75%, 80%, 85% or 90, and more preferably at least about 91%, 92%, 93%, 94% or 95%, and most preferably at least about 96%, 97%, 98%, 99% or more homologous to an entire amino acid sequence of as indicated in Table IIA or IIB, columns 5 or 7, lines 243 to 250 and 603, resp.

**[0301.0.0.19]** for the disclosure of this paragraph see [0301.0.0.0] above.

**[0302.0.19.19]** Biologically active portions of an polypeptide of the present invention include peptides comprising amino acid sequences derived from the amino acid sequence of the polypeptide of the present invention or used in the process of the present invention, e.g., an amino acid sequence as indicated in Table II, columns 5 or 7, lines 243 to 250 and 603, resp., or the amino acid sequence of a protein homologous thereto, which include fewer amino acids than a full length polypeptide of the present invention or used in the process of the present invention or the full length protein which is homologous to an polypeptide of the present invention or used in the process of the present invention depicted herein, and exhibit at least one activity of polypeptide of the present invention or used in the process of the present invention.

**[0303.0.0.19]** for the disclosure of this paragraph see [0303.0.0.0] above.

**[0304.0.19.19]** Manipulation of the nucleic acid molecule of the invention may result in the production of a protein having essentially the activity of the polypeptides as indicated in Table II, column 3, lines 243 to 250 and 603but having differences in the sequence from said wild-type protein. These proteins may be improved in efficiency or activity, may be present in greater numbers in the cell than is usual, or may be decreased in efficiency or activity in relation to the wild type protein.

**[0305.0.0.19] to [0308.0.0.19]** for the disclosure of the paragraphs [0305.0.0.19] to [0308.0.0.19] see paragraphs [0305.0.0.0 to [0308.0.0.0] above.

**[0309.0.19.19]** In one embodiment, a reference to a protein (= polypeptide) of the invention or as indicated in Table II, columns 5 or 7, lines 243 to 250 and 603, resp., refers to a polypeptide having an amino acid sequence corresponding to the polypeptide of the invention or used in the process of the invention, whereas an "other polypeptide" not being indicated in Table II, columns 5 or 7, lines 243 to 250 and 603, resp., refers to a polypeptide having an amino acid sequence corresponding to a protein which is not substantially homologous to a polypeptide of the invention, preferably which is not substantially homologous to a polypeptide as indicated in Table II, columns 5 or 7, lines 243 to 250 and 603, resp.,, e.g., a protein which does not confer the activity described herein or annotated or known for as indicated in Table II, column 3, lines 243 to 250 and 603, resp., and which is derived from the same or a different organism. In one embodiment, an "other polypeptide" not being indicated in Table II, columns 5 or 7, lines 243 to 250 and 603, resp., does not confer an increase of the respective fine chemical in an organism or part thereof.

**[0310.0.0.19] to [0334.0.0.19]** for the disclosure of the paragraphs [0310.0.0.19] to [0334.0.0.19] see paragraphs [0310.0.0.0] to [0334.0.0.0] above.

**[0335.0.19.19]** The dsRNAi method has proved to be particularly effective and advantageous for reducing the expression of a nucleic acid sequences as indicated in Table I, columns 5 or 7, lines 243 to 250 and 603, resp., and/or homologs thereof. As described inter alia in WO 99/32619, dsRNAi approaches are clearly superior to traditional antisense approaches. The invention therefore furthermore relates to double-stranded RNA molecules (dsRNA molecules) which, when introduced into an organism, advantageously into a plant (or a cell, tissue, organ or seed derived there from), bring about altered metabolic activity by the reduction in the expression of a nucleic acid sequences as indicated in Table I, columns 5 or 7, lines 243 to 250 and 603, resp., and/or homologs thereof. In a double-stranded RNA molecule for reducing the expression of aprotein encoded by a nucleic acid sequence as indicated in Table I, columns 5 or 7, lines 243 to 250 and 603, resp., and/or homologs thereof, one of the two RNA strands is essentially identical to at least part of a nucleic acid sequence, and the respective other RNA strand is essentially identical to at least part of the complementary strand of a nucleic acid sequence.

**[0336.0.0.19] to [0342.0.0.19]** for the disclosure of the paragraphs [0336.0.0.19] to [0342.0.0.19] see paragraphs [0336.0.0.0] to [0342.0.0.0] above.

**[0343.0.19.19]** As has already been described, 100 % sequence identity between the dsRNA and a gene transcript of a nucleic acid sequence as indicated in Table I, columns 5 or 7, lines 243 to 250 and 603, resp., or it's homolog is not necessarily required in order to bring about effective reduction in the expression. The advantage is, accordingly, that the method is tolerant with regard to sequence deviations as may be present as a consequence of genetic mutations, polymorphisms or evolutionary divergences. Thus, for example, using the dsRNA, which has been generated starting from a sequence as indicated in Table I, columns 5 or 7, lines 243 to 250 and 603, resp., or homologs thereof of the one organism, may be used to suppress the corresponding expression in another organism.

**[0344.0.0.19] to [0361.0.0.19]** for the disclosure of the paragraphs [0344.0.0.19] to [0361.0.0.19] see paragraphs [0344.0.0.0] to [0361.0.0.0] above.

**[0362.0.19.19]** Accordingly the present invention relates to any cell transgenic for any nucleic acid characterized as part of the invention, e.g. conferring the increase of the respective fine chemical in a cell or an organism or a part thereof, e.g. the nucleic acid molecule of the invention, the nucleic acid construct of the invention, the antisense molecule of the invention, the vector of the invention or a nucleic acid molecule encoding the polypeptide of the invention, e.g. the polypeptide as indicated in Table II, columns 5 or 7, lines 243 to 250 and 603, resp., e.g. encoding a polypeptide having protein activity, as indicated in Table II, columns 3, lines 243 to 250 and 603, resp.,. Due to the abovementioned activity the respective fine chemical content in a cell or an organism is increased. For example, due to modulation or manipulation, the cellular activity of the polypeptide of the invention or nucleic acid molecule of the invention is increased, e.g. due to an increased expression or specific activity of the subject matters of the invention in a cell or an organism or a part thereof. Transgenic for a polypeptide having an activity of a polypeptide as indicated in Table II, columns 5 or 7, lines 243 to 250 and 603, resp., means herein that due to modulation or manipulation of the genome, an activity as annotated for a polypeptide as indicated in Table II, column 3, lines 243 to 250 and 603, e.g. having a sequence as indicated in Table II, columns 5 or 7, lines 243 to 250 and 603, resp., is increased in a cell or an organism or a part thereof. Examples are described above in context with the process of the invention.

**[0363.0.0.19]** for the disclosure of this paragraph see [0363.0.0.0] above.

**[0364.0.19.19]** A naturally occurring expression cassette - for example the naturally occurring combination of a promoter of a gene encoding a polypeptide of the invention as indicated in Table II, column 3, lines 243 to 250 and 603, resp. with the corresponding protein-encoding sequence as indicated in Table I, column 5, lines 243 to 250 and 603, resp., becomes a transgenic expression cassette when it is modified by non-natural, synthetic "artificial" methods such as, for example, mutagenization. Such methods have been described (US 5,565,350; WO 00/15815; also see above).

**[0365.0.0.19] to [0373.0.0.19]** for the disclosure of the paragraphs [0365.0.0.19], to [0373.0.0.19] see paragraphs [0365.0.0.0] to [0373.0.0.0] above.

**[0374.0.19.19]** Transgenic plants comprising the respective fine chemical synthesized in the process according to the invention can be marketed directly without isolation of the compounds synthesized. In the process according to the invention, plants are understood as meaning all plant parts, plant organs such as leaf, stalk, root, tubers or seeds or propagation material or harvested material or the intact plant. In this context, the seed encompasses all parts of the seed such as the seed coats, epidermal cells, seed cells, endosperm or embryonic tissue, ferulic acid or sinapic acid, in particular the respective fine chemical, produced in the process according to the invention may, however, also be isolated from the plant in the form of their free ferulic acid or sinapic acid, in particular the free respective fine chemical, or bound in or to compounds or moieties, like glucosides, e.g. diglucosides. The respective fine chemical produced by this process can be harvested by harvesting the organisms either from the culture in which they grow or from the field. This can be done via expressing, grinding and/or extraction, salt precipitation and/or ion-exchange chromatography or other chromatographic methods of the plant parts, preferably the plant seeds, plant fruits, plant tubers and the like.

**[0375.0.0.19] and [0376.0.0.19]** for the disclosure of the paragraphs [0375.0.0.19] and [0376.0.0.19] see paragraphs [0375.0.0.0] and [0376.0.0.0] above.

**[0377.0.19.19]** Accordingly, the present invention relates also to a process whereby the produced ferulic acid or sinapic acid is isolated.

**[0378.0.19.19]** In this manner, more than 50% by weight, advantageously more than 60% by weight, preferably more than 70% by weight, especially preferably more than 80% by weight, very especially preferably more than 90% by weight, of the ferulic acid or sinapic acid produced in the process can be isolated. The resulting ferulic acid or sinapic acid can, if appropriate, subsequently be further purified, if desired mixed with other active ingredients such as vitamins, amino acids, carbohydrates, antibiotics and the like, and, if appropriate, formulated.

**[0379.0.19.19]** In one embodiment, ferulic acid and sinapic are a mixture of the respective fine chemicals.

**[0380.0.19.19]** The ferulic acid or sinapic acid obtained in the process are suitable as starting material for the synthesis of further products of value. For example, they can be used in combination with each other or alone for the production of pharmaceuticals, foodstuffs, animal feeds or cosmetics. Accordingly, the present invention relates a method for the production of pharmaceuticals, food stuff, animal feeds, nutrients or cosmetics comprising the steps of the process according to the invention, including the isolation of the ferulic acid or sinapic acid composition produced or the respective fine chemical produced if desired and formulating the product with a pharmaceutical acceptable carrier or formulating the product in a form acceptable for an application in agriculture. A further embodiment according to the invention is the use of the ferulic acid or sinapic acid produced in the process or of the transgenic organisms in animal feeds, foodstuffs, medicines, food supplements, cosmetics or pharmaceuticals or for the production of ferulic acid or sinapic acid e.g. after isolation of the respective fine chemical or without, e.g. in situ, e.g in the organism used for the process for the production of the respective fine chemical.

**[0381.0.0.19] to [0384.0.0.19]** for the disclosure of the paragraphs [0381.0.0.19], to [0384.0.0.19] see paragraphs [0381.0.0.0] to [0384.0.0.0] above.

**[0385.0.19.19]** The fermentation broths obtained in this way, containing in particular ferulic acid or sinapic acid in mixtures with other organic acids, aminoacids, polypeptides or polysaccarides, normally have a dry matter content of from 1 to 70% by weight, preferably 7.5 to 25% by weight. Sugar-limited fermentation is additionally advantageous, e.g. at the end, for example over at least 30% of the fermentation time. This means that the concentration of utilizable sugar in the fermentation medium is kept at, or reduced to, 0 to 10 g/I, preferably to 0 to 3g/l during this time. The fermentation broth is then processed further. Depending on requirements, the biomass can be removed or isolated entirely or partly by separation methods, such as, for example, centrifugation, filtration, decantation, coagulation/flocculation or a combination of these methods, from the fermentation broth or left completely in it.

The fermentation broth can then be thickened or concentrated by known methods, such as, for example, with the aid of a rotary evaporator, thin-film evaporator, falling film evaporator, by reverse osmosis or by nanofiltration. This concentrated fermentation broth can then be worked up by freeze-drying, spray drying, spray granulation or by other processes.

**[0386.0.19.19]** Accordingly, it is possible to purify the ferulic acid or sinapic acid produced according to the invention further. For this purpose, the product-containing composition is subjected for example to separation via e.g. an open column chromatography or HPLC in which case the desired product or the impurities are retained wholly or partly on the chromatography resin. These chromatography steps can be repeated if necessary, using the same or different chromatography resins. The skilled worker is familiar with the choice of suitable chromatography resins and their most effective use.

**[0387.0.0.19] to [0392.0.0.19]** for the disclosure of the paragraphs [0387.0.0.19] to [0392.0.0.19] see paragraphs [0387.0.0.0] to [0392.0.0.0] above.

**[0393.0.19.19]** In one embodiment, the present invention relates to a method for the identification of a gene product conferring an increase in the respective fine chemical production in a cell, comprising the following steps:
(c) contacting, e.g. hybridising, the nucleic acid molecules of a sample, e.g. cells, tissues, plants or microorganisms or a nucleic acid library , which can contain a candidate gene encoding a gene product conferring an increase in the respective fine chemical after expression, with the nucleic acid molecule of the present invention;
b. identifying the nucleic acid molecules, which hybridize under relaxed stringent conditions with the nucleic acid molecule of the present invention in particular to the nucleic acid molecule sequence as indicated in Table I, columns 5 or 7, lines 243 to 250 and 603, preferably in Table IB, columns 5 or 7, lines 243 to 250 and 603 resp., and, optionally, isolating the full length cDNA clone or complete genomic clone;
c. introducing the candidate nucleic acid molecules in host cells, preferably in a plant cell or a microorganism, appropriate for producing the respective fine chemical;
d. expressing the identified nucleic acid molecules in the host cells;
e. assaying the respective fine chemical level in the host cells; and
f. identifying the nucleic acid molecule and its gene product which expression confers an increase in the respective fine chemical level in the host cell after expression compared to the wild type.

**[0394.0.0.19] to [0398.0.0.19]** for the disclosure of the paragraphs [0394.0.0.19] to [0398.0.0.19] see paragraphs [0394.0.0.0] to [0398.0.0.0] above.

**[0399.0.19.19]** Furthermore, in one embodiment, the present invention relates to process for the identification of a compound conferring increase of the respective fine chemical production in a plant or microorganism, comprising the steps:
(o) culturing a cell or tissue or microorganism or maintaining a plant expressing the polypeptide according to the invention or a nucleic acid molecule encoding said polypeptide and a readout system capable of interacting with the polypeptide under suitable conditions which permit the interaction of the polypeptide with said readout system in the presence of a compound or a sample comprising a plurality of compounds and capable of providing a detectable signal in response to the binding of a compound to said polypeptide under conditions which permit the expression of said readout system and the polypeptide of the present invention or used in the process of the invention; and
(p) identifying if the compound is an effective agonist by detecting the presence or absence or increase of a signal produced by said readout system.
The screen for a gene product or an agonist conferring an increase in the respective fine chemical production can be performed by growth of an organism for example a microorganism in the presence of growth reducing amounts of an inhibitor of the synthesis of the respective fine chemical. Better growth, e.g. higher dividing rate or high dry mass in comparison to the control under such conditions would identify a gene or gene product or an agonist conferring an increase in respective fine chemical production.

**[00399.1.19.19]** One can think to screen for increased production of the respective fine chemical by for example searching for a resistance to a drug blocking the synthesis of the respective fine chemical and looking whether this effect is dependent on the activity or expression of a polypeptide as indicated in Table II, columns 5 or 7, lines 243 to 250 and 603 or a homolog thereof, e.g. comparing the phenotype of nearly identical organisms with low and high activity of a protein as indicated in Table II, columns 5 or 7, lines 243 to 250 and 603 after incubation with the drug.

**[0400.0.0.19] to [0416.0.0.19]** for the disclosure of the paragraphs [0400.0.0.19] to [0416.0.0.19] see paragraphs [0400.0.0.0] to [0416.0.0.0] above.

**[0417.0.19.19]** The nucleic acid molecule of the invention, the vector of the invention or the nucleic acid construct of the invention may also be useful for the production of organisms resistant to inhibitors of the ferulic acid or sinapic acid biosynthesis pathways. In particular, the overexpression of the polypeptide of the present invention may protect an organism such as a microorganism or a plant against inhibitors, which block the ferulic acid or sinapic acid synthesis.

**[0418.0.0.19] to [0423.0.0.19]** for the disclosure of the paragraphs [0418.0.0.19] to [0423.0.0:19] see paragraphs [0418.0.0.0] to [0423.0.0.0] above.

**[0424.0.19.19]** Accordingly, the nucleic acid of the invention, the polypeptide of the invention, the nucleic acid construct of the invention, the organisms, the host cell, the microorganisms, the plant, plant tissue, plant cell, or the part thereof of the invention, the vector of the invention, the agonist identified with the method of the invention, the nucleic acid molecule identified with the method of the present invention, can be used for the production of the respective fine chemical or of the respective fine chemical and one or more other organic acids. Accordingly, the nucleic acid of the invention, or the nucleic acid molecule identified with the method of the present invention or the complement sequences thereof, the polypeptide of the invention, the nucleic acid construct of the invention, the organisms, the host cell, the microorganisms, the plant, plant tissue, plant cell, or the part thereof of the invention, the vector of the invention, the antagonist identified with the method of the invention, the antibody of the present invention, the antisense molecule of the present invention, can be used for the reduction of the respective fine chemical in a organism or part thereof, e.g. in a cell.

**[0425.0.0.19] to [0434.0.0.19]** for the disclosure of the paragraphs [0425.0.0.19] to [0434.0.0.19] see paragraphs [0425.0.0.0] to [0434.0.0.0] above.

**[0435.0.19.19]** Example 3: In-vivo and in-vitro mutagenesis

**[0436.0.19.19]** An *in vivo* mutagenesis of organisms such as Saccharomyces, Mortierella, Escherichia and others mentioned above, which are beneficial for the production of ferulic acid or sinapic acid can be carried out by passing a plasmid DNA (or another vector DNA) containing the desired nucleic acid sequence or nucleic acid sequences, e.g. the nucleic acid molecule of the invention or the vector of the invention, through E.coli and other microorganisms (for example Bacillus spp. or yeasts such as Saccharomyces cerevisiae) which are not capable of maintaining the integrity of its genetic information. Usual mutator strains have mutations in the genes for the DNA repair system [for example mutHLS, mutD, mutT and the like; for comparison, see Rupp, W.D. (1996) DNA repair mechanisms in Escherichia coli and Salmonella, pp. 2277-2294, ASM: Washington]. The skilled worker knows these strains. The use of these strains is illustrated for example in Greener, A. and Callahan, M. (1994) Strategies 7; 32-34.
In-vitro mutation methods such as increasing the spontaneous mutation rates by chemical or physical treatment are well known to the skilled person. Mutagens like 5-bromo-uracil, N-methyl-N-nitro-N-nitrosoguanidine (= NTG), ethyl methanesulfonate (=EMS), hydroxylamine and/or nitrous acid are widly used as chemical agents for random in-vitro mutagensis. The most common physical method for mutagensis is the treatment with UV irradiation. Another random mutagenesis technique is the error-prone PCR for introducing amino acid changes into proteins. Mutations are deliberately introduced during PCR through the use of error-prone DNA polymerases and special reaction conditions known to a person skilled in the art. For this method randomized DNA sequences are cloned into expression vectors and the resulting mutant libraries screened for altered or improved protein activity as described below.
Site-directed mutagensis method such as the introduction of desired mutations with an M13 or phagemid vector and short oligonucleotides primers is a well-known approach for site-directed mutagensis. The clou of this method involves cloning of the nucleic acid sequence of the invention into an M13 or phagemid vector, which permits recovery of single-stranded recombinant nucleic acid sequence. A mutagenic oligonucleotide primer is then designed whose sequence is perfectly complementary to nucleic acid sequence in the region to be mutated, but with a single difference: at the intended mutation site it bears a base that is complementary to the desired mutant nucleotide rather than the original. The mutagenic oligonucleotide is then allowed to prime new DNA synthesis to create a complementary full-length sequence containing the desired mutation. Another site-directed mutagensis method is the PCR mismatch primer mutagensis method also known to the skilled person. Dpnl site-directed mutagensis is a further known method as described for example in the Stratagene QuickchangeTM site-directed mutagenesis kit protocol. A huge number of other methods are also known and used in common practice.
Positive mutation events can be selected by screening the organisms for the production of the desired respective fine chemical.

**[0437.0.19.19]** Example 4: DNA transfer between Escherichia coli, Saccharomyces cerevisiae and Mortierella alpina

**[0438.0.19.19]** Shuttle vectors such as pYE22m, pPAC-ResQ, pClasper, pAUR224, pAMH10, pAML10, pAMT10, pAMU10, pGMH10, pGML10, pGMT10, pGMU10, pPGAL1, pPADH1, pTADH1, pTAex3, pNGA142, pHT3101 and derivatives thereof which allow the transfer of nucleic acid sequences between Escherichia coli, Saccharomyces cerevisiae and/or Mortierella alpina are available to the skilled worker. An easy method to isolate such shuttle vectors is disclosed by Soni R. and Murray J.A.H. [Nucleic Acid Research, vol. 20 no. 21, 1992: 5852]: If necessary such shuttle vectors can be constructed easily using standard vectors for E. coli (Sambrook, J. et al., (1989), "Molecular Cloning: A Laboratory Manual", Cold Spring Harbor Laboratory Press or Ausubel, F.M. et al. (1994) "Current Protocols in Molecular Biology", John Wiley & Sons) and/or the aforementioned vectors, which have a replication origin for, and suitable marker from, Escherichia coli, Saccharomyces cerevisiae or Mortierella alpina added. Such replication origins are preferably taken from endogenous plasmids, which have been isolated from species used in the inventive process. Genes, which are used in particular as transformation markers for these species are genes for kanamycin resistance (such as those which originate from the Tn5 or Tn-903 transposon) or for chloramphenicol resistance (Winnacker, E.L. (1987) "From Genes to Clones - Introduction to Gene Technology", VCH, Weinheim) or for other antibiotic resistance genes such as for G418, gentamycin, neomycin, hygromycin or tetracycline resistance.

**[0439.0.19.19]** Using standard methods, it is possible to clone a gene of interest into one of the above-described shuttle vectors and to introduce such hybrid vectors into the microorganism strains used in the inventive process. The transformation of Saccharomyces can be achieved for example by LiCl or sheroplast transformation (Bishop et al., Mol. Cell. Biol., 6, 1986: 3401 - 3409; Sherman et al., Methods in Yeasts in Genetics, [Cold Spring Harbor Lab. Cold Spring Harbor, N. Y.] 1982, Agatep et al., Technical Tips Online 1998, 1:51: P01525 or Gietz et al., Methods Mol. Cell. Biol. 5, 1995: 255f) or electroporation (Delorme E., Appl. Environ. Microbiol., vol. 55, no. 9, 1989 : 2242 - 2246).

**[0440.0.19.19]** If the transformed sequence(s) is/are to be integrated advantageously into the genome of the microorganism used in the inventive process for example into the yeast or fungi genome, standard techniques known to the skilled worker also exist for this purpose. Solinger et al. (Proc Natl Acad Sci U S A., 2001 (15): 8447-8453) and Freedman et al. (Genetics, Vol. 162, 15-27, September 2002,) teaches a homolog recombination system dependent on rad 50, rad51, rad54 and rad59 in yeasts. Vectors using this system for homologous recombination are vectors derived from the Ylp series. Plasmid vectors derived for example from the 2µ-Vector are known by the skilled worker and used for the expression in yeasts. Other preferred vectors are for example pART1, pCHY21 or pEVP11 as they have been described by McLeod et al. (EMBO J. 1987, 6:729-736) and Hoffman et al. (Genes Dev. 5, 1991: :561-571.) or Russell et al. (J. Biol. Chem. 258, 1983: 143-149.). Other beneficial yeast vectors are plasmids of the REP, REP-X, pYZ or RIP series.

**[0441.0.0.19] to [0443.0.0.19]** for the disclosure of the paragraphs [0441.0.0.19] to [0443.0.0.19] see paragraphs [0441.0.0.0] to [0443.0.0.0] above.

**[0444.0.19.19]** Example 6: Growth of genetically modified organism: media and culture conditions

**[0445.0.19.19]** Genetically modified Yeast, Mortierella or Escherichia coli are grown in synthetic or natural growth media known by the skilled worker. A number of different growth media for Yeast, Mortierella or Escherichia coli are well known and widely available. A method for culturing Mortierella is disclosed by Jang et al. [Bot. Bull. Acad. Sin. (2000) 41: 41-48]. Mortierella can be grown at 20°C in a culture medium containing: 10 g/l glucose, 5 g/l yeast extract at pH 6.5. Futhermore Jang et al. teaches a submerged basal medium containing 20 g/l soluble starch, 5 g/l Bacto yeast extract, 10 g/l KNO₃, 1 g/l KH₂PO₄, and 0.5 g/l MgSO₄•7H₂O, pH 6.5.

**[0446.0.0.19] to [0450.0.0.19]** for the disclosure of the paragraphs [0446.0.0.19] to [0450.0.0.19] see paragraphs [0446.0.0.0] to [0450.0.0.0] above.

**[0451.0.5.19], [0452.0.0.19] and [0453.0.0.19]** for the disclosure of the paragraphs [0451.0.5.19], [0452.0.0.19] and [0453.0.0.19] see [0451.0.5.5], [0452.0.0.0] and [0453.0.0.0] above.

**[0454.0.19.19]** Analysis of the effect of the nucleic acid molecule on the production of ferulic acid or sinapic acid

**[0455.0.19.19]** The effect of the genetic modification in plants, fungi, algae, ciliates or on the production of a desired compound (such as a ferulic acid or sinapic acid) can be determined by growing the modified microorganisms or the modified plant under suitable conditions (such as those described above) and analyzing the medium and/or the cellular components for the elevated production of desired product (i.e. of ferulic acid or sinapic acid). These analytical techniques are known to the skilled worker and comprise spectroscopy, thin-layer chromatography, various types of staining methods, enzymatic and microbiological methods and analytical chromatography such as high-performance liquid chromatography (see, for example, Ullman, Encyclopedia of Industrial Chemistry, Vol. A2, p. 89-90 and p. 443-613, VCH: Weinheim (1985); Fallon, A., et al., (1987) "Applications of HPLC in Biochemistry" in: Laboratory Techniques in Biochemistry and Molecular Biology, Vol. 17; Rehm et al. (1993) Biotechnology, Vol. 3, Chapter III: "Product recovery and purification", p. 469-714, VCH: Weinheim; Belter, P.A., et al. (1988) Bioseparations: downstream processing for Biotechnology, John Wiley and Sons; Kennedy, J.F., and Cabral, J.M.S. (1992) Recovery processes for biological Materials, John Wiley and Sons; Shaeiwitz, J.A., and Henry, J.D. (1988) Biochemical Separations, in: Ullmann's Encyclopedia of Industrial Chemistry, Vol. B3; Chapter 11, p. 1-27, VCH: Weinheim; and Dechow, F.J. (1989) Separation and purification techniques in biotechnology, Noyes Publications).

**[0456.0.0.19]** for the disclosure of this paragraph see [0456.0.0.0] above.

**[0457.0.19.19]** Example 9: Purification of ferulic acid or sinapic acid

**[0458.0.19.19]** Abbreviations; GC-MS, gas liquid chromatography/mass spectrometry; TLC, thin-layer chromatography.
The unambiguous detection for the presence of ferulic acid or sinapic acid can be obtained by analyzing recombinant organisms using analytical standard methods: LC, LC-MSMS or TLC, as described. The total amount produced in the organism for example in yeasts used in the inventive process can be analysed for example according to the following procedure:
The material such as yeasts, E. coli or plants to be analyzed can be disrupted by sonication, grinding in a glass mill, liquid nitrogen and grinding or via other applicable methods.
Plant material is initially homogenized mechanically by comminuting in a pestle and mortar to make it more amenable to extraction.
A typical sample pretreatment consists of a total lipid extraction using such polar organic solvents as acetone or alcohols as methanol, or ethers, saponification, partition between phases, seperation of non-polar epiphase from more polar hypophasic derivatives and chromatography.
For analysis, solvent delivery and aliquot removal can be accomplished with a robotic system comprising a single injector valve Gilson 232XL and a 402 2S1V diluter [Gilson, Inc. USA, 3000 W. Beltline Highway, Middleton, WI]. For saponification, 3 ml of 50% potassium hydroxide hydro-ethanolic solution (4 water - 1 ethanol) can be added to each vial, followed by the addition of 3 ml of octanol. The saponification treatment can be conducted at room temperature with vials maintained on an IKA HS 501 horizontal shaker [Labworld-online, Inc., Wilmington, NC] for fifteen hours at 250 movements/minute, followed by a stationary phase of approximately one hour.
Following saponification, the supernatant can be diluted with 0.17 ml of methanol. The addition of methanol can be conducted under pressure to ensure sample homogeneity. Using a 0.25 ml syringe, a 0.1 ml aliquot can be removed and transferred to HPLC vials for analysis.
For HPLC analysis, a Hewlett Packard 1100 HPLC, complete with a quaternary pump, vacuum degassing system, six-way injection valve, temperature regulated autosampler, column oven and Photodiode Array detector can be used [Agilent Technologies available through Ultra Scientific Inc., 250 Smith Street, North Kingstown, RI]. The column can be a Waters YMC30, 5-micron, 4.6 x 250 mm with a guard column of the same material [Waters, 34 Maple Street, Milford, MA]. The solvents for the mobile phase can be 81 methanol: 4 water: 15 tetrahydrofuran (THF) stabilized with 0.2% BHT (2,6-di-tert-butyl-4-methylphenol). Injections were 20 I. Separation can be isocratic at 30°C with a flow rate of 1.7 ml/minute. The peak responses can be measured by absorbance at 447 nm.

**[0459.0.19.19]** If required and desired, further chromatography steps with a suitable resin may follow. Advantageously, the ferulic acid or sinapic acid can be further purified with a so-called RTHPLC. As eluent acetonitrile/water or chloroform/acetonitrile mixtures can be used. If necessary, these chromatography steps may be repeated, using identical or other chromatography resins. The skilled worker is familiar with the selection of suitable chromatography resin and the most effective use for a particular molecule to be purified.

**[0460.0.0.19]** for the disclosure of this paragraph see [0460.0.0.0] above.

**[0461.0.19.19]** Example 10: Cloning SEQ ID NO: 24071, 24083, 24177, 24353, 24865, 25117, 25357, 25361 or 92604 for the expression in plants

**[0462.0.0.19]** for the disclosure of this paragraph see [0462.0.0.0] above.

**[0463.0.19.19]** SEQ ID NO: 24071, 24083, 24177, 24353, 24865, 25117, 25357, 25361 or 92604 is amplified by PCR as described in the protocol of the Pfu Turbo or DNA Herculase polymerase (Stratagene).

**[0464.0.0.19] to [0466.0.0.19]** for the disclosure of the paragraphs [0464.0.0.19] to [0466.0.0.19] see paragraphs [0464.0.0.0] to [0466.0.0.0] above.

**[0466.1.0.19]** In case the Herculase enzyme can be used for the amplification, the PCR amplification cycles were as follows: 1 cycle of 2-3 minutes at 94°C, followed by 25-30 cycles of in each case 30 seconds at 94°C, 30 seconds at 55-60°C and 5-10 minutes at 72°C, followed by 1 cycle of 10 minutes at 72°C, then 4°C.

**[0467.0.19.19]** The following primer sequences were selected for the gene SEQ ID NO: 24071:
i) forward primer (SEQ ID NO: 24081)
   atgcgtgctt taccgatctg ttta
ii) reverse primer (SEQ ID NO: 24082)
   ttatttcgcc gtaatgttaa gcgcag
The following primer sequences were selected for the gene SEQ ID NO: 24083:
i) forward primer (SEQ ID NO: 24175)
   atgggacaca agcccttata ccg
ii) reverse primer (SEQ ID NO: 24176)
   ttatcgcgat gattttcgct gcg
The following primer sequences were selected for the gene SEQ ID NO: 24177:
i) forward primer (SEQ ID NO: 24351)
   atgagtcgtt tagtcgtagt atcta
ii) reverse primer (SEQ ID NO: 24152)
   ttacgcaagc tttggaaagg tagc
The following primer sequences were selected for the gene SEQ ID NO: 24353:
i) forward primer (SEQ ID NO: 24863)
   atgagtaaga tttttgaaga taac
ii) reverse primer (SEQ ID NO: 24864)
   ttactgttgc aattctttct cagtg
The following primer sequences were selected for the gene SEQ ID NO: 24865:
i) forward primer (SEQ ID NO: 25115)
   atggaaaccg tggcttacgc tg
ii) reverse primer (SEQ ID NO: 24116)
   ttatacgacg cgtacgcccg c
The following primer sequences were selected for the gene SEQ ID NO: 25117:
i) forward primer (SEQ ID NO: 25355)
   atgacgacga ttctcaagca tctc
ii) reverse primer (SEQ ID NO: 25356)
   ttactggcct ttgttttcca gattc
The following primer sequences were selected for the gene SEQ ID NO: 25357:
i) forward primer (SEQ ID NO: 25359)
   atgtaccaaa ataatgtatt gaatgct
ii) reverse primer (SEQ ID NO: 25360)
   tcaatagtgc attaactctc ccatt
The following primer sequences were selected for the gene SEQ ID NO: 25361:
i) forward primer (SEQ ID NO: 25495)
   atggagacca atttttcctt cgact
ii) reverse primer (SEQ ID NO: 25496)
   ctattgaaat accggcttca atattt
The following primer sequences were selected for the gene SEQ ID NO: 92604:
i) forward primer (SEQ ID NO: 92658)
   atggtaaagg aacgtaaaac cgagt
ii) reverse primer (SEQ ID NO: 92659)
   ttaccctaaa tccgccatca acac

**[0468.0.0.19] to [0470.0.0.19]** for the disclosure of the paragraphs [0468.0.0.19] to [0470.0.0.19] see paragraphs [0468.0.0.0] to [0470.0.0.0] above.

**[0470.1.19.19]** The PCR-products, produced by *Pfu* Turbo DNA polymerase, were phosphorylated using a T4 DNA polymerase using a standard protocol (e.g. MBI Fermentas) and cloned into the processed binary vector.

**[0471.0.0.19]** for the disclosure of this paragraph see [0471.0.0.0] above.

**[0471.1.19.19]** The DNA termini of the PCR-products, produced by Herculase DNA polymerase, were blunted in a second synthesis reaction using Pfu Turbo DNA polymerase. The composition for the protocol of the blunting the DNA-termini was as follows: 0.2 mM blunting dTTP and 1.25 u *Pfu* Turbo DNA polymerase. The reaction was incubated at 72°C for 30 minutes. Then the PCR-products were phosphorylated using a T4 DNA polymerase using a standard protocol (e.g. MBI Fermentas) and cloned into the processed vector as well.

**[0472.0.0.19] to [0479.0.0.19]** for the disclosure of the paragraphs [0472.0.0.19] to [0479.0.0.19] see paragraphs [0472.0.0.0] to [0479.0.0.0] above.

**[0480.0.19.19]** Example 11: Generation of transgenic plants which express SEQ ID NO:24071, 24083, 24177, 24353, 24865, 25117, 25357, 25361 or 92604.

**[0481.0.0.19] to [0513.0.0.19]** for the disclosure of the paragraphs [0481.0.0.19] to [0513.0.0.19] see paragraphs [0481.0.0.0] to [0513.0.0.0] above.

**[0514.0.19.19]** As an alternative, ferulic acid acid can be detected as described in Mattila, P. and Kumpulainen J., J. Agric Food Chem. 2002 Jun 19;50(13):3660-7.
As an alternative, sinapic acid can be detected as described in Noda, M. and Matsumoto, M., Biochim Biophys Acta. 1971 Feb 2;231(1):131-3.
The results of the different plant analyses can be seen from the table 1 which follows:

**Table 1**

| **ORF** | **Metabolite** | **Method** | **Min** | **Max** |
|---|---|---|---|---|
| b0196 | Ferulic acid | **LC** | 1,10 | 1,25 |
| b0730 | Ferulic acid | LC | 1,38 | 1,97 |
| b1896 | Sinapic Acid | GC | 1,38 | 1,98 |
| b2414 | Ferulic acid | LC | 1,34 | 1,86 |
| b3074 | **Ferulic acid** | LC | 1,35 | 1,73 |
| b3172 | Sinapic Acid | GC | 1,31 | 1,89 |
| YBR184W | **Ferulic acid** | LC | 1,30 | 1,37 |
| YDR513W | Sinapic Acid | GC | 1,30 | 1,39 |
| b2818 | Sinapic Acid | GC | 1,27 | 1,54 |

Column 2 shows the metabolite ferulic acid or sinapic acid analyzed. Columns 4 and 5 shows the ratio of the analyzed metabolite between the transgenic plants and the wild type; Increase of the metabolite: Max: maximal x-fold (normalised to wild type)-Min: minimal x-fold (normalised to wild type). Decrease of the metabolite: Max: maximal x-fold (normalised to wild type) (minimal decrease), Min: minimal x-fold (normalised to wild type) (maximal decrease). Column 3 indicates the analytical method.

**[0515.0.0.19] to [0530.0.0.19]** for the disclosure of the paragraphs [0515.0.0.19] to [0530.0.0.19] see paragraphs [0515.0.0.0] to [0530.0.0.0] above.

**[0530.1.0.19] to [0530.6.0.19]** for the disclosure of the paragraphs [0530.1.0.19] to [0530.6.0.19] see paragraphs [0530.1.0.0] to [0530.6.0.0] above.

**[0531.0.0.19] to [0552.0.0.19]** for the disclosure of the paragraphs [0531.0.0.19] to [0552.0.0.19] see paragraphs [0531.0.0.0] to [0552.0.0.0] above.

**[0552.1.0.19] %**

**[0552.2.0.19]** for the disclosure of this paragraph see [0552.2.0.0] above.

**[0553.0.19.19]**
1. A process for the production of ferulic acid or sinapic acid, which comprises
   (a) increasing or generating the activity of a protein as indicated in Table II, columns 5 or 7, lines 243 to 250 and 603 or a functional equivalent thereof in a non-human organism or in one or more parts thereof; and
   (b) growing the organism under conditions which permit the production of ferulic acid or sinapic acid in said organism.
2. A process for the production of ferulic acid or sinapic acid, comprising the increasing or generating in an organism or a part thereof the expression of at least one nucleic acid molecule comprising a nucleic acid molecule selected from the group consisting of:
   a) nucleic acid molecule encoding of a polypeptide as indicated in Table II, columns 5 or 7, lines 243 to 250 and 603 or a fragment thereof, which confers an increase in the amount of ferulic acid or sinapic acid in an organism or a part thereof;
   b) nucleic acid molecule comprising of a nucleic acid molecule as indicated in Table I, columns 5 or 7, lines 243 to 250 and 603;
   c) nucleic acid molecule whose sequence can be deduced from a polypeptide sequence encoded by a nucleic acid molecule of (a) or (b) as a result of the degeneracy of the genetic code and conferring an increase in the amount of ferulic acid or sinapic acid in an organism or a part thereof;
   d) nucleic acid molecule which encodes a polypeptide which has at least 50% identity with the amino acid sequence of the polypeptide encoded by the nucleic acid molecule of (a) to (c) and conferring an increase in the amount of ferulic acid or sinapic acid in an organism or a part thereof;
   e) nucleic acid molecule which hybidizes with a nucleic acid molecule of (a) to (c) under stringent hybridisation conditions and conferring an increase in the amount of ferulic acid or sinapic acid in an organism or a part thereof;
   f) nucleic acid molecule which encompasses a nucleic acid molecule which is obtained by amplifying nucleic acid molecules from a cDNA library or a genomic library using the primers or primer pairs as indicated in Table III, column 7, lines 243 to 250 and 603 and conferring an increase in the amount of ferulic acid or sinapic acid in an organism or a part thereof;
   g) nucleic acid molecule encoding a polypeptide which is isolated with the aid of monoclonal antibodies against a polypeptide encoded by one of the nucleic acid molecules of (a) to (f) and conferring an increase in the amount of ferulic acid or sinapic acid in an organism or a part thereof;
   h) nucleic acid molecule encoding a polypeptide comprising a consensus as indicated in Table IV, column 7, lines 243 to 250 and 603 and conferring an increase in the amount of ferulic acid or sinapic acid in an organism or a part thereof; and
   i) nucleic acid molecule which is obtainable by screening a suitable nucleic acid library under stringent hybridization conditions with a probe comprising one of the sequences of the nucleic acid molecule of (a) to (k) or with a fragment thereof having at least 15 nt, preferably 20 nt, 30 nt, 50 nt, 100 nt, 200 nt or 500 nt of the nucleic acid molecule characterized in (a) to (k) and conferring an increase in the amount of ferulic acid or sinapic acid in an organism or a part thereof.
   or comprising a sequence which is complementary thereto.
3. The process of claim 1 or 2, comprising recovering of the free or bound ferulic acid or sinapic acid.
4. The process of any one of claim 1 to 3, comprising the following steps:
   a) selecting an organism or a part thereof expressing a polypeptide encoded by the nucleic acid molecule characterized in claim 2;
   b) mutagenizing the selected organism or the part thereof;
   c) comparing the activity or the expression level of said polypeptide in the mutagenized organism or the part thereof with the activity or the expression of said polypeptide of the selected organisms or the part thereof;
   d) selecting the mutated organisms or parts thereof, which comprise an increased activity or expression level of said polypeptide compared to the selected organism or the part thereof;
   e) optionally, growing and cultivating the organisms or the parts thereof; and
   f) recovering, and optionally isolating, the free or bound ferulic acid or sinapic acid produced by the selected mutated organisms or parts thereof.
5. The process of any one of claims 1 to 4, wherein the activity of said protein or the expression of said nucleic acid molecule is increased or generated transiently or stably.
6. An isolated nucleic acid molecule comprising a nucleic acid molecule selected from the group consisting of:
   a) nucleic acid molecule encoding of a polypeptide as indicated in Table II, columns 5 or 7, lines 243 to 250 and 603 or a fragment thereof, which confers an increase in the amount of ferulic acid or sinapic acid in an organism or a part thereof;
   b) nucleic acid molecule comprising of a nucleic acid molecule as indicated in Table I, columns 5 or 7, lines 243 to 250 and 603;
   c) nucleic acid molecule whose sequence can be deduced from a polypeptide sequence encoded by a nucleic acid molecule of (a) or (b) as a result of the degeneracy of the genetic code and conferring an increase in the amount of ferulic acid or sinapic acid in an organism or a part thereof;
   d) nucleic acid molecule which encodes a polypeptide which has at least 50% identity with the amino acid sequence of the polypeptide encoded by the nucleic acid molecule of (a) to (c) and conferring an increase in the amount of ferulic acid or sinapic acid in an organism or a part thereof;
   e) nucleic acid molecule which hybridizes with a nucleic acid molecule of (a) to (c) under r stringent hybridization conditions and conferring an increase in the amount of ferulic acid or sinapic acid in an organism or a part thereof;
   f) nucleic acid molecule which encompasses a nucleic acid molecule which is obtained by amplifying nucleic acid molecules from a cDNA library or a genomic library using the primers or primer pairs as indicated in Table III, column 7, lines 243 to 250 and 603 and conferring an increase in the amount of ferulic acid or sinapic acid in an organism or a part thereof;
   g) nucleic acid molecule encoding a polypeptide which is isolated with the aid of monoclonal antibodies against a polypeptide encoded by one of the nucleic acid molecules of (a) to (f) and conferring an increase in the amount of ferulic acid or sinapic acid in an organism or a part thereof;
   h) nucleic acid molecule encoding a polypeptide comprising a consensus as indicated in Table IV, column 7, lines 243 to 250 and 603 and conferring an increase in the amount of in an organism or a part thereof; and
   i) nucleic acid molecule which is obtainable by screening a suitable nucleic acid library under stringent hybridization conditions with a probe comprising one of the sequences of the nucleic acid molecule of (a) to (k) or with a fragment thereof having at least 15 nt, preferably 20 nt, 30 nt, 50 nt, 100 nt, 200 nt or 500 nt of the nucleic acid molecule characterized in (a) to (k) and conferring an increase in the amount of ferulic acid or sinapic acid in an organism or a part thereof.
   whereby the nucleic acid molecule distinguishes over the sequence as indicated in Table IA, columns 5 or 7, lines 243 to 250 and 603 by one or more nucleotides.
7. A nucleic acid construct which confers the expression of the nucleic acid molecule of claim 6, comprising one or more regulatory elements.
8. A vector comprising the nucleic acid molecule as claimed in claim 6 or the nucleic acid construct of claim 7.
9. The vector as claimed in claim 8, wherein the nucleic acid molecule is in operable linkage with regulatory sequences for the expression in a prokaryotic or eukaryotic, or in a prokaryotic and eukaryotic, host.
10. A host cell, which has been transformed stably or transiently with the vector as claimed in claim 8 or 9 or the nucleic acid molecule as claimed in claim 6 or the nucleic acid construct of claim 7 or produced as described in claim any one of claims 2 to 5.
11. The host cell of claim 10, which is a transgenic host cell.
12. The host cell of claim 10 or 11, which is a plant cell, an animal cell, a microorganism, or a yeast cell, a fungus cell, a prokaryotic cell, an eukaryotic cell or an archaebacterium.
13. A process for producing a polypeptide, wherein the polypeptide is expressed in a host cell as claimed in any one of claims 10 to 12.
14. A polypeptide produced by the process as claimed in claim 13 or encoded by the nucleic acid molecule as claimed in claim 6 whereby the polypeptide distinguishes over a sequence as indicated in Table IIA, columns 5 or 7, lines 243 to 250 and 603 by one or more amino acids.
15. An antibody, which binds specifically to the polypeptide as claimed in claim 14.
16. A plant tissue, propagation material, harvested material or a plant comprising the host cell as claimed in claim 12 which is plant cell or an Agrobacterium.
17. A method for screening for agonists and antagonists of the activity of a polypeptide encoded by the nucleic acid molecule of claim 6 conferring an increase in the amount of ferulic acid or sinapic acid in an organism or a part thereof comprising:
   (a) contacting cells, tissues, plants or microorganisms which express the a polypeptide encoded by the nucleic acid molecule of claim 6 conferring an increase in the amount of ferulic acid or sinapic acid in an organism or a part thereof with a candidate compound or a sample comprising a plurality of compounds under conditions which permit the expression the polypeptide;
   (b) assaying the ferulic acid or sinapic acid level or the polypeptide expression level in the cell, tissue, plant or microorganism or the media the cell, tissue, plant or microorganisms is cultured or maintained in; and
   (c) identifying a agonist or antagonist by comparing the measured ferulic acid or sinapic acid level or polypeptide expression level with a standard ferulic acid or sinapic acid or polypeptide expression level measured in the absence of said candidate compound or a sample comprising said plurality of compounds, whereby an increased level over the standard indicates that the compound or the sample comprising said plurality of compounds is an agonist and a decreased level over the standard indicates that the compound or the sample comprising said plurality of compounds is an antagonist.
18. A process for the identification of a compound conferring increased ferulic acid or sinapic acid production in a plant or microorganism, comprising the steps:
   a) culturing a plant cell or tissue or microorganism or maintaining a plant expressing the polypeptide encoded by the nucleic acid molecule of claim 6 conferring an increase in the amount of ferulic acid or sinapic acid in an organism or a part thereof and a readout system capable of interacting with the polypeptide under suitable conditions which permit the interaction of the polypeptide with said readout system in the presence of a compound or a sample comprising a plurality of compounds and capable of providing a detectable signal in response to the binding of a compound to said polypeptide under conditions which permit the expression of said readout system and of the polypeptide encoded by the nucleic acid molecule of claim 6 conferring an increase in the amount of ferulic acid or sinapic acid in an organism or a part thereof;
   b) identifying if the compound is an effective agonist by detecting the presence or absence or increase of a signal produced by said readout system.
19. A method for the identification of a gene product conferring an increase in ferulic acid or sinapic acid production in a cell, comprising the following steps:
   a) contacting the nucleic acid molecules of a sample, which can contain a candidate gene encoding a gene product conferring an increase in ferulic acid or sinapic acid after expression with the nucleic acid molecule of claim 6;
   b) identifying the nucleic acid molecules, which hybridise under relaxed stringent conditions with the nucleic acid molecule of claim 6;
   c) introducing the candidate nucleic acid molecules in host cells appropriate for producing ferulic acid or sinapic acid;
   d) expressing the identified nucleic acid molecules in the host cells;
   e) assaying the ferulic acid or sinapic acid level in the host cells; and
   f) identifying nucleic acid molecule and its gene product which expression confers an increase in the ferulic acid or sinapic acid level in the host cell in the host cell after expression compared to the wild type.
20. A method for the identification of a gene product conferring an increase in ferulic acid or sinapic acid production in a cell, comprising the following steps:
   a) identifiying in a data bank nucleic acid molecules of an organism; which can contain a candidate gene encoding a gene product conferring an increase in the ferulic acid or sinapic acid amount or level in an organism or a part thereof after expression, and which are at least 20% homolog to the nucleic acid molecule of claim 6;
   b) introducing the candidate nucleic acid molecules in host cells appropriate for producing ferulic acid or sinapic acid;
   c) expressing the identified nucleic acid molecules in the host cells;
   d) assaying the ferulic acid or sinapic acid level in the host cells; and
   e) identifying nucleic acid molecule and its gene product which expression confers an increase in the ferulic acid or sinapic acid level in the host cell after expression compared to the wild type.
21. A method for the production of an agricultural composition comprising the steps of the method of any one of claims 17 to 20 and formulating the compound identified in any one of claims 17 to 20 in a form acceptable for an application in agriculture.
22. A composition comprising the nucleic acid molecule of claim 6, the polypeptide of claim 14, the nucleic acid construct of claim 7, the vector of any one of claims 8 or 9, an antagonist or agonist identified according to claim 17, the compound of claim 18, the gene product of claim 19 or 20, the antibody of claim 15, and optionally an agricultural acceptable carrier.
23. Use of the nucleic acid molecule as claimed in claim 6 for the identification of a nucleic acid molecule conferring an increase of ferulic acid or sinapic acid after expression.
24. Use of the polypeptide of claim 14 or the nucleic acid construct claim 7 or the gene product identified according to the method of claim 19 or 20 for identifying compounds capable of conferring a modulation of ferulic acid or sinapic acid levels in an organism.
25. Agrochemical, pharmaceutical, food or feed composition comprising the nucleic acid molecule of claim 6, the polypeptide of claim 14, the nucleic acid construct of claim 7, the vector of claim 8 or 9, the antagonist or agonist identified according to claim 17, the antibody of claim 15, the plant or plant tissue of claim 16, the harvested material of claim 16, the host cell of claim 10 to 12 or the gene product identified according to the method of claim 19 or 20.
26. The method of any one of claims 1 to 5, the nucleic acid molecule of claim 6, the polypeptide of claim 14, the nucleic acid construct of claim 7, the vector of claim 8 or 9, the antagonist or agonist identified according to claim 17, the antibody of claim 15, the plant or plant tissue of claim 16, the harvested material of claim 16, the host cell of claim 10 to 12 or the gene product identified according to the method of claim 19 or 20, wherein the fine chemical is ferulic acid or sinapic acid.

**[0554.0.0.19] Abstract: see [0554.0.0.0]**

### Process for the production of fine chemicals

### Description

**[0000.0.0.20]** In a further embodiment, the present invention relates to a further process for the production of fine chemicals as defined below and the corresponding embodiments as described herein as follows.

**[0001.0.0.20] see [0001.0.0.0]**

**[0002.0.20.20]**
Plants produce very long chain fatty acids such as behenic acid (C22:0), lignoceric acid (C24:0), cerotic acid (C26:0) and/or melissic acid (C30:0).
Very long-chain fatty acids (VLCFAs) are synthesized by a membrane-bound fatty acid elongation complex (elongase, FAE) using acyl-CoA substrates. The first reaction of elongation involves condensation of malonyl-CoA with a long chain substrate producing a β-ketoacyl-CoA. Subsequent reactions are reduction of β-hydroxyacyl-CoA, dehydration to an enoyl-CoA, followed by a second reduction to form the elongated acyl-CoA. The β-ketoacyl-CoA synthase (KCS) catalyzing the condensation reaction plays a key role in determining the chain length of fatty acid products found in seed oils and is the rate-limiting enzyme for seed VLCFA production (Lassner et al., Plant Cell, 8(1996), 281-292).
The elongation process can be repeated to yield members that are 20, 22, and 24 carbons long. Although such very long chain fatty acids are minor components of the lipid membranes of the body, they undoubtedly perform valuable functions, apparently helping to stabilize membranes, especially those in peripheral nerve cells.
Behenic acid (22:0) (docosanoic acid) is a component of rapeseed oil (up to 2%) and peanut oil (1-5%).
Behenic acid is used to give hair conditioners and moisturizers their smoothing properties.
Lignoceric acid (24:0) (tetracosanoic acid) is a component of rapeseed oil (up to 1 %) and peanut oil (1-3%).
Cerotic acid (26:0) (hexacosanoic acid) is a component of beeswax.
Echinacea angustifolia extracts are sold as natural health products comprising the very long chain fatty acid cerotic acid.
Cerotic acid is used in cosmetics as a constituent in hairstyling products.
Melissic acid (C30:0) (triacontanoic acid) is a component of beeswax.
Beeswax (cera alba) is obtained from the product excreted by certain glands of the honeybee from which the honeycomb is made. It is freed of solid impurities by
melting and centrifugation (cera flava). Finally, it is bleached completely white (cera alba). Beeswax consists of 10-15 percent paraffin carbohydrates, 35-37 percent esters of C16 to C36 fatty acids and about 15 percent cerotic acid, melissic acid and their homologues. Beeswax is used as a thickener and a humectant in the manufacture of ointments, creams, lipsticks and other cosmetics and skincare products as an emulsifier, emollient, moisturizer and film former.
Beeswax is also used for the production of candles.
Wax is a general term used to refer to the mixture of long-chain apolar lipids forming a protective coating (cutin in the cuticle) on plant leaves and fruits but also in animals (wax of honeybee, cuticular lipids of insects, spermaceti of the sperm whale, skin lipids, uropygial glands of birds, depot fat of planktonic crustacea), algae, fungi and bacteria.
Many of the waxes found in nature have commercial uses in the lubricant, food and cosmetic industry. Jojoba oil has long been suggested as a putative resource of wax, since this desert shrub is unusual in its capacity to produce waxes rather than triacylglycerols (TAG) as seed storage lipids. These waxes are esters of very-long-chain-fatty acids and fatty alcohols (Miwa , 1971, J Am Oil Chem Soc 48, 259-264). As the production cost for jojoba wax, which is primarily used for cosmetic applications, is high, there is a need to engineer crop plants to produce high level of wax esters in its seed oil.
Plant aerial surfaces are covered by epicuticular waxes, complex mixtures of very long (C₂₀-C₃₄) fatty acids, alkanes, aldehydes, ketones and esters. In addition to repelling atmospheric water they prevent dessication and are therefore an important determinant of drought resistance (Riederer and Schreiber, 2001, J. Exp. Bot 52, 2023-2032). Beside abiotic stress resistance the wax layer is part of the plant defense against biotic stressor, especially insects as for example described by Marcell and Beattie, 2002, Mol Plant Microbe Interact. 15(12), 1236-44. Furthermore they provide stability to pollen grains, thus influencing fertility and productivity.
Very-long-chain fatty acids (VLCFAs), consisting of more than 18 carbon atoms like behenic acid, lignoceric acid, cerotic acid and melissic acid, are essential components for the vitality of higher plants. The key enzyme of VLCFA biosynthesis, the extraplastidary fatty acid elongase, is shown for to be the primary target site of chloroacetamide herbicides. With an analysis of the fatty acid composition and the metabolism of 14C-labelled precursors (sterate, malonate, acetate), the reduction of VLCFAs was determined in vivo. The inhibition of the recombinant protein substantiates the first and rate-limiting step of VLCFA biosynthesis, the condensation of acyl-CoA with malonyl-CoA to β-ketoacyl-CoA, to be the primary target site of chloroacetamides (150 = 10-100 nM). The concentration of VLCFAs within the untreated cell is low, the very-long-chain compounds are found mainly in plasma membrane lipids and epicuticular waxes. A shift of fatty acids towards shorter chain length or even the complete depletion of very-long-chain components is the consequence of the inhibition of VLCFA biosynthesis. Especially the loss of plasma membrane VLCFAs is involved in phytotoxic effects of chloroacetamides such as the inhibition of membrane biogenesis and mitosis (Matthes, B., htte://www.ub.uni-konstanz.de/kops/volltexte/2001/661/).
Increased wax production in transgenic plants has for example been reported by Broun et al., 2004, Proc Natl. Acad. Sci, 101, 4706-4711. The authors overexpressed the transcriptional activator WIN1 in Arabidopsis, leading to increased wax load on arial organs. As this resulted in a complex change in the wax profile and the transgenic overexpressors had characteristic alterations in growth and development (Broun et al., 2004, Proc Natl. Acad. Sci, 101, 4706-4711) there is still a need for a more controlled increased production of defined VLCFAs.
Very long chain fatty alcohols obtained from plant waxes and beeswax have also been reported to lower plasma cholesterol in humans and existing data support the hypothesis that VLCFA exert regulatory roles in cholesterol metabolism in the peroxisome and also alter LDL uptake and metabolism (discussed in Hargrove et al., 2004, Exp Biol Med (Maywood), 229(3): 215-26).
Due to these interesting physiological roles and the nutritional, cosmetic and agrobiotechnological potential of behenic acid (C22:0), lignoceric acid (C24:0), cerotic acid (C26:0) and melissic acid (C30:0) there is a need to identify the genes of enzymes and other proteins involved in behenic acid, lignoceric acid, cerotic acid or melissic acid metabolism, and to generate mutants or transgenic plant lines with which to modify the behenic acid, lignoceric acid, cerotic acid or melissic acid content in plants.

**[0003.0.0.20]** %

**[0004.0.0.20]** %

**[0005.0.0.20]** %

**[0006.0.0.20]** %

**[0007.0.0.20]** %

**[0008.0.20.20]** One way to increase the productive capacity of biosynthesis is to apply recombinant DNA technology. Thus, it would be desirable to produce behenic acid, lignoceric acid, cerotic acid or melissic acid in plants. That type of production permits control over quality, quantity and selection of the most suitable and efficient producer organisms. The latter is especially important for commercial production economics and therefore availability to consumers. In addition it is desirable to produce behenic acid, lignoceric acid, cerotic acid or melissic acid in plants in order to increase plant productivity and resistance against biotic and abiotic stress as discussed before.
Methods of recombinant DNA technology have been used for some years to improve the production of fine chemicals in microorganisms and plants by amplifying individual biosynthesis genes and investigating the effect on production of fine chemicals. It is for example reported, that the xanthophyll astaxanthin could be produced in the nectaries of transgenic tobacco plants. Those transgenic plants were prepared by *Argobacterium tumifaciens*-mediated transformation of tobacco plants using a vector that contained a ketolase-encoding gene from *H. pluvialis* denominated *crtO* along with the *Pds* gene from tomato as the promoter and to encode a leader sequence. Those results indicated that about 75 percent of the carotenoids found in the flower of the transformed plant contained a keto group.

**[0009.0.20.20]** Thus, it would be advantageous if an algae, plant or other microorganism were available which produce large amounts behenic acid, lignoceric acid, cerotic acid or melissic acid. The invention discussed hereinafter relates in some embodiments to such transformed prokaryotic or eukaryotic microorganisms.
It would also be advantageous if plants were available whose roots, leaves, stem, fruits or flowers produced large amounts of behenic acid, lignoceric acid, cerotic acid or melissic acid. The invention discussed hereinafter relates in some embodiments to such transformed plants.

**[0010.0.20.20]** Therefore improving the quality of foodstuffs and animal feeds is an important task of the food-and-feed industry. This is necessary since, for example behenic acid, lignoceric acid, cerotic acid or melissic acid, as mentioned above, which occur in plants and some microorganisms are limited with regard to the supply of mammals. Especially advantageous for the quality of foodstuffs and animal feeds is as balanced as possible a specific behenic acid, lignoceric acid, cerotic acid or melissic acid profile in the diet since an excess of behenic acid, lignoceric acid, cerotic acid or melissic acid above a specific concentration in the food has a positive effect. A further increase in quality is only possible via addition of further behenic acid, lignoceric acid, cerotic acid or melissic acid, which are limiting.

**[0011.0.20.20]** To ensure a high quality of foods and animal feeds, it is therefore necessary to add behenic acid, lignoceric acid, cerotic acid or melissic acid in a balanced manner to suit the organism.

**[0012.0.20.20]** Accordingly, there is still a great demand for new and more suitable genes which encode enzymes or other proteins which participate in the biosynthesis of behenic acid, lignoceric acid, cerotic acid or melissic acid and make it possible to produce them specifically on an industrial scale without unwanted byproducts forming. In the selection of genes for biosynthesis two characteristics above all are particularly important. On the one hand, there is as ever a need for improved processes for obtaining the highest possible contents of behenic acid, lignoceric acid, cerotic acid or melissic acid; on the other hand as less as possible byproducts should be produced in the production process.

**[0013.0.0.20] see [0013.0.0.0]**

**[0014.0.20.20]** Accordingly, in a first embodiment, the invention relates to a process for the production of a fine chemical, whereby the fine chemical is a behenic acid, lignoceric acid, cerotic acid or melissic acid. Accordingly, in the present invention, the term "the fine chemical" as used herein relates to a behenic acid, lignoceric acid, cerotic acid or melissic acid. Further, the term "the fine chemicals" as used herein also relates to fine chemicals comprising behenic acid, lignoceric acid, cerotic acid or melissic acid.
In one embodiment, the term "the fine chemical" means behenic acid. In one embodiment, the term "the fine chemical" means lignoceric acid depending on the context in which the term is used. In another embodiment, the term "the fine chemical" means cerotic acid. In a further another embodiment, the term "the fine chemical" means melissic acid. Throughout the specification the term "the fine chemical" means behenic acid, lignoceric acid, cerotic acid or melissic acid, its salts, ester, thioester or in free form or bound to other compounds such sugars or sugarpolymers, like glucoside, e.g. diglucoside.

**[0016.0.20.20]** Accordingly, the present invention relates to a process comprising
(a) increasing or generating the activity of one or more b0019, b0880, b1886, b1896, b3938, YDR513W, YER156C, YGL205W, YHR201C, YLR255C, YPR138C, b0255 protein(s) in a non-human organism in one or more parts thereof; and
(b) growing the organism under conditions which permit the production of the fine chemical, thus behenic acid, lignoceric acid, cerotic acid or melissic acid in said organism.
Accordingly, the present invention relates to a process comprising
(a) increasing or generating the activity of one or more proteins having the activity of a protein indicated in Table IIA or IIB, column 3, lines 251 to 261, 627, resp. or having the sequence of a polypeptide encoded by a nucleic acid molecule indicated in Table IA or IB, column 5 or 7, lines 251 to 261, 627, resp. in a non-human organism in one or more parts thereof; and
growing the organism under conditions which permit the production of the fine chemical, thus, behenic acid, lignoceric acid, cerotic acid or melissic acid, in said organism.

**[0016.1.20.20]** Accordingly, the term "the fine chemical" means "behenic acid" in relation to all sequences listed in Table IA or IB, line 258 or homologs thereof, means "lignoceric acid" in relation to the sequence listed in Table IA or IB, lines 251, 255, 259 ,261 or 627 homologs thereof, means "melissic acid" in relation to the sequences listed in Table IA or IB, lines 256, 257 or 260. Accordingly, the term "the fine chemical" can mean "behenic acid", "lignoceric acid", "cerotic acid" or "melissic acid", owing to circumstances and the context. In order to illustrate that the meaning of the term "the respective fine chemical" means "behenic acid", "lignoceric acid", "cerotic acid" or "melissic acid" owing to the sequences listed in the context the term "the respective fine chemical" is also used.

**[0017.0.0.20] to [0018.0.0.20]: see [0017.0.0.0] to [0018.0.0.0]**

**[0019.0.20.20]** Advantageously the process for the production of the respective fine chemical leads to an enhanced production of the respective fine chemical. The terms "enhanced" or "increase" mean at least a 10%, 20%, 30%, 40% or 50%, preferably at least 60%, 70%, 80%, 90% or 100%, more preferably 150%, 200%, 300%, 400% or 500% higher production of the respective fine chemical in comparison to the reference as defined below, e.g. that means in comparison to an organism without the aforementioned modification of the activity of a protein having the activity of a protein indicated in Table IIA or IIB, column 3, lines 251 to 261, 627 or encoded by nucleic acid molecule indicated in Table IA or IB, columns 5 or 7, lines 251 to 261, 627.

**[0020.0.20.20]** Surprisingly it was found, that the transgenic expression of the Escherichia coli K12 protein b0019, b0880, b1886, b1896, b3938, b0255 or Saccharomyces cerevisiae protein YDR513W, YER156C, YGL205W, YHR201C, YLR255C, YPR138C in Arabidopsis thaliana conferred an increase in behenic acid, lignoceric acid, cerotic acid or melissic acid ("the fine chemical" or "the fine respective chemical") in respect to said proteins and their homologs as wells as the encoding nucleic acid molecules, in particular as indicated in Table IIA or IIB, column 3, lines 251 to 261, 627 content of the transformed plants.

**[0021.0.0.20]** see **[0021.0.0.0]**

**[0022.0.20.20]** The sequence of b0019 from Escherichia coli K12 has been published in Blattner,F.R. et al., Science 277 (5331), 1453-1474 (1997) and its activity is being defined as a protein having Na+/H+ antiporter activity. Accordingly, in one embodiment, the process of the present invention comprises the use of a protein b0019 from Escherichia coli K12 or its homolog, e.g. as shown herein, for the production of the respective fine chemical, in particular for increasing the amount of lignoceric acid and/or cerotic acid, preferably in free or bound form in an organism or a part thereof, as mentioned. In one embodiment, in the process of the present invention the activity of the protein b0019 is increased.
The sequence of b0880 from Escherichia coli K12 has been published in Blattner, F.R. et al, Science 277 (5331). 1453-1474 (1997) and its activity is being defined as a protein having stress induced DNA replication inhibitor activity. Accordingly, in one embodiment, the process of the present invention comprises the use of a protein b0880 from Escherichia coli K12 or its homolog, e.g. as shown herein, for the production of the respective fine chemical, in particular for increasing the amount of cerotic acid, preferably in free or bound form in an organism or a part thereof, as mentioned. In one embodiment, in the process of the present invention the activity of the protein b0880 is increased.
The sequence of b1886 from Escherichia coli K12 has been published in Blattner,F.R. et al., Science 277 (5331), 1453-1474 (1997) and its activity is being defined as a methyl-accepting chemotaxis protein II, aspartate sensor receptor. Accordingly, in one embodiment, the process of the present invention comprises the use of a protein b1886 from Escherichia coli K12 or its homolog, e.g. as shown herein, for the production of the respective fine chemical, in particular for increasing the amount of cerotic acid, preferably in free or bound form in an organism or a part thereof, as mentioned. In one embodiment, in the process of the present invention the activity of the protein b1886 is increased.
The sequence of b1896 from Escherichia coli K12 has been published in Blattner, F.R. et al., Science 277 (5331), 1453-1474 (1997) and its activity is being defined as a protein having trehalose-6-phosphate synthase activity. Accordingly, in one embodiment, the process of the present invention comprises the use of a protein b1896 from Escherichia coli K12 or its homolog, e.g. as shown herein, for the production of the respective fine chemical, in particular for increasing the amount of cerotic acid, preferably in free or bound form in an organism or a part thereof, as mentioned. In one embodiment, in the process of the present invention the activity of the protein b1896 is increased.
The sequence of b3938 from Escherichia coli K12 has been published in Blattner, F.R. et al., Science 277 (5331), 1453-1474 (1997) and its activity is being defined as transcriptional repressor for methionine biosynthesis. Accordingly, in one embodiment, the process of the present invention comprises the use of a protein b3938 from Escherichia coli K12 or its homolog, e.g. as shown herein, for the production of the respective fine chemical, in particular for increasing the amount of lignoceric acid, preferably in free or bound form in an organism or a part thereof, as mentioned. In one embodiment, in the process of the present invention the activity of the protein b3938 is increased.
The sequence of YDR513W from Saccharomyces cerevisiae has been published in Jacq,C. et al., Nature 387 (6632 Suppl), 75-78 (1997) and its activity is being defined as a protein having glutathione reductase activity. Accordingly, in one embodiment, the process of the present invention comprises the use of a protein YDR513W or its homolog, e.g. as shown herein, for the production of the respective fine chemical, in particular for increasing the amount of melissic acid, preferably in free or bound form in an organism or a part thereof, as mentioned. In one embodiment, in the process of the present invention the activity of the protein YDR513W is increased.
The sequence of YER156C from Saccharomyces cerevisiae has been published in Dietrich,F.S. et al., Nature 387 (6632 Suppl), 78-81 (1997) and its activity has not been characterized yet. Accordingly, in one embodiment, the process of the present invention comprises the use of a protein YER156C or its homolog, e.g. as shown herein, for the production of the respective fine chemical, in particular for increasing the amount of melissic acid, preferably in free or bound form in an organism or a part thereof, as mentioned. In one embodiment, in the process of the present invention the activity of the protein YER156C is increased.
The sequence of YGL205W from Saccharomyces cerevisiae has been published in Tettelin,H. et al., Nature 387 (6632 Suppl), 81-84 (1997) and its activity is being defined as a protein having fatty-acyl coenzyme A oxidase activity. Accordingly, in one embodiment, the process of the present invention comprises the use of a protein YGL205W or its homolog, e.g. as shown herein, for the production of the respective fine chemical, in particular for increasing the amount of behenic acid, preferably in free or bound form in an organism or a part thereof, as mentioned. In one embodiment, in the process of the present invention the activity of the protein YGL205W is increased.
The sequence of YHR201C from Saccharomyces cerevisiae has been published in Goffeau,A. et al., Science 274 (5287), 546-547 (1996) and its activity is being defined as a protein having exopolyphosphatase activity. Accordingly, in one embodiment, the process of the present invention comprises the use of a protein YHR201C or its homolog, e.g. as shown herein, for the production of the respective fine chemical, in particular for increasing the amount of lignoceric acid and/or cerotic acid, preferably in free or bound form in an organism or a part thereof, as mentioned. In one embodiment, in the process of the present invention the activity of the protein YHR201C is increased.
The sequence of YLR255C from Saccharomyces cerevisiae has been published in the EMBL Data Library, February 1995 and its activity has not been characterized yet. Accordingly, in one embodiment, the process of the present invention comprises the use of a protein YLR255C or its homolog, e.g. as shown herein, for the production of the respective fine chemical, in particular for increasing the amount of melissic acid, preferably in free or bound form in an organism or a part thereof, as mentioned. In one embodiment, in the process of the present invention the activity of the protein YLR255C is increased.
The sequence of YPR138C from Saccharomyces cerevisiae has been published in Bussey,H. et al., Nature 387 (6632 Suppl), 103-105 (1997) and its activity is being defined as a protein having NH4+ transporter activity. Accordingly, in one embodiment, the process of the present invention comprises the use of a protein YPR138C or its homolog, e.g. as shown herein, for the production of the respective fine chemical, in particular for increasing the amount of lignoceric acid, preferably in free or bound form in an organism or a part thereof, as mentioned. In one embodiment, in the process of the present invention the activity of the protein YPR138C is increased.
The sequence of b0255 from Escherichia coli K12 has been published in Blattner et al., Science 277 (5331), 1453-1474, 1997, and its activity is being defined as a CP4-6 prophage; IS911 homolog. Accordingly, in one embodiment, the process of the present invention comprises the use of said CP4-6 prophage; IS911 homolog protein from E. coli or its homolog, e.g. as shown herein, for the production of the fine chemical, meaning of linoleic acid (ist linoleic acid korrekt?)or lignoceric acid and/or tryglycerides, lipids, oils and/or fats containing linoleic acid or lignoceric acid, in particular for increasing the amount of linoleic acid or lignoceric acid and/or tryglycerides, lipids, oils and/or fats containing linoleic acid and /or lignoceric acid, preferably linoleic acid or lignoceric acid in free or bound form in an organism or a part thereof, as mentioned. In one embodiment, in the process of the present invention the activity of said CP4-6 prophage; IS911 homolog protein is increased or generated, e.g. from E. coli or a homolog thereof.

**[0023.0.20.20]** Homologues (= homologs) of the present gene products can be derived from any organisms as long as the homologue confers the herein mentioned activity, in particular, confers an increase in the respective fine chemical amount or content.
In one embodiment, the homolog of any one of the polypeptides indicated in Table IIA or IIB, column 3, lines 251, 255, 259, 261 and 627 is a homolog having the same or a similar activity. In particular an increase of activity confers an increase in the content of the respective fine chemical in the organisms preferably lignoceric acid.
In one embodiment, the homolog of the polypeptides indicated in Table IIA or IIB, column 3, lines 251, 252, 253, 254 and 259 is a homolog having the same or a similar acitivity. In particular an increase of activity confers an increase in the content of the respective fine chemical in the organisms preferably cerotic acid.
In one embodiment, the homolog of any one of the polypeptides indicated in Table IIA or IIB, column 3, lines 256, 257 and 260 is a homolog having the same or a similar activity. In particular an increase of activity confers an increase in the content of the respective fine chemical in the organisms preferably melissic acid.
In one embodiment, the homolog of any one of the polypeptides indicated in Table IIA or IIB, column 3, line 258 is a homolog having the same or a similar activity. In particular an increase of activity confers an increase in the content of the respective fine chemical in the organisms preferably behenic acid.
Homologs of the polypeptides indicated in Table IIA or IIB, column 3, lines 251, 255, 259, 261 and 627 may be the polypeptides encoded by the nucleic acid molecules indicated in Table IA or IB, column 7, lines 251, 255, 259, 261 and 627 or may be the polypeptides indicated in Table IIA or IIB, column 7, lines 251, 255, 259; 261 and 627 having a lignoceric acid content and/or amount increasing activity.
Homologs of the polypeptides indicated in Table IIA or IIB, column 3, lines 251, 252, 253, 254 and 259 may be the polypeptides encoded by the nucleic acid molecules indicated in Table IA or IB, column 7, lines 251, 252, 253, 254 and 259, respectively or may be the polypeptides indicated in Table IIA or IIB, column 7, lines 251, 252, 253, 254 and 259 having a cerotic acid content and/or amount increasing activity.
Homologs of the polypeptide indicated in Table IIA or IIB, column 3, lines 256, 257 and 260 may be the polypeptides encoded by the nucleic acid molecules indicated in Table IA or IB, column 7, lines 256, 257 and 260, respectively or may be the polypeptides indicated in Table IIA or IIB, column 7, lines 256, 257 and 260, having a melissic acid content and/or amount increasing activity.
Homologs of the polypeptide indicated in Table IIA or IIB, column 3, line 258 may be the polypeptides encoded by the nucleic acid molecules indicated in Table IA or IB, column 7, line 258, respectively or may be the polypeptides indicated in Table IIA or IIB, column 7, line 258, having a behenic content and/or amount increasing activity.

**[0024.0.0.20] see [0024.0.0.0]**

**[0025.0.20.20]** In accordance with the invention, a protein or polypeptide has the "activity of a protein of the invention", e.g. the activity of a protein indicated in Table IIA or IIB, column 3, lines 251 to 261, 627 if its *de novo* activity, or its increased expression directly or indirectly leads to an increased behenic acid, lignoceric acid, cerotic acid or melissic acid level, resp., in the organism or a part thereof, preferably in a cell of said organism. In a preferred embodiment, the protein or polypeptide has the above-mentioned additional activities of a protein indicated in Table IIA or IIB, column 3, lines 251 to 261, 627. Throughout the specification the activity or preferably the biological activity of such a protein or polypeptide or an nucleic acid molecule or sequence encoding such protein or polypeptide is identical or similar if it still has the biological or enzymatic activity of any one of the proteins indicated in Table IIA or IIB, column 3, lines 251 to 261, 627, or which has at least 10% of the original enzymatic activity, preferably 20%, particularly preferably 30%, most particularly preferably 40% in comparison to any one of the proteins indicated in Table IIA or IIB, column 3, line 251 to 255 of Escherichia coli K12 or line 256 to 261 of Saccharomyces cerevisiae respectively.
In one embodiment, the polypeptide of the invention confers said activity, e.g. the increase of the respective fine chemical in an organism or a part thereof, if it is derived from an organism, which is evolutionary close to the organism indicated in Table IA or IB, column 4 and is expressed in an organism, which is evolutionary distant to the origin organism. For example origin and expressing organism are derived from different families, orders, classes or phylums whereas origin and the organism indicated in Table IA or IB, column 4 are derived from the same families, orders, classes or phylums.

**[0025.1.0.20] see [0025.1.0.0]**

**[0026.0.0.20] to [0033.0.0.20]: see [0026.0.0.0] to [0033.0.0.0]**

**[0034.0.20.20]** Preferably, the reference, control or wild type differs from the subject of the present invention only in the cellular activity of the polypeptide of the invention, e.g. as result of an increase in the level of the nucleic acid molecule of the present invention or an increase of the specific activity of the polypeptide of the invention. E.g., it differs by or in the expression level or activity of a protein having the activity of a protein as indicated in Table IIA or IIB, column 3, lines 251 to 261, 627 or being encoded by a nucleic acid molecule indicated in Table IA or IB, column 5, lines 251 to 261, 627 or its homologs, e.g. as indicated in Table IA or IB, column 7, lines 251 to 261, 627, its biochemical or genetic causes. It therefore shows the increased amount of the respective fine chemical.

**[0035.0.0.20] to [0038.0.0.20]: see [0035.0.0.0] to [0038.0.0.0]**

**[0039.0.0.20]: see [0039.0.0.0]**

**[0040.0.0.20] to [0044.0.0.20]: see [0040.0.0.0] to [0044.0.0.0]**

**[0045.0.20.20]** In one embodiment, in case the activity of the Escherichia coli K12 protein b0019 or its homologs, e.g. as indicated in Table IIA or IIB, columns 5 or 7, line 251 is increased, preferably, in one embodiment the increase of the respective fine chemical, preferably of cerotic acid between 40% and 150% or more is conferred.
In one embodiment, in case the activity of the Escherichia coli K12 protein b0019 or its homologs, e.g. as indicated in Table IIA or IIB, columns 5 or 7, line 251 is increased, preferably, in one embodiment the increase of the respective fine chemical, preferably of lignoceric acid between 46% and 224% or more is conferred.
In one embodiment, in case the activity of the Escherichia coli K12 protein b0880 or its homologs, e.g. as indicated in Table IIA or IIB, columns 5 or 7, line 252 is increased, preferably, in one embodiment the increase of the respective fine chemical, preferably of cerotic acid between 172% and 294% or more is conferred.
In one embodiment, in case the activity of the Escherichia coli K12 protein b1886 or its homologs, e.g. as indicated in Table IIA or IIB, columns 5 or 7, line 253 is increased, preferably, in one embodiment the increase of the respective fine chemical, preferably of cerotic acid between 39% and 335% or more is conferred.
In one embodiment, in case the activity of the Escherichia coli K12 protein b1896 or its homologs, e.g. as indicated in Table IIA or IIB, columns 5 or 7, line 254 is increased, preferably, in one embodiment the increase of the respective fine chemical, preferably of cerotic acid between 39% and 75% or more is conferred.
In one embodiment, in case the activity of the Escherichia coli K12 protein b3938 or its homologs, e.g. as indicated in Table IIA or IIB, columns 5 or 7, line 255 is increased, preferably, in one embodiment the increase of the respective fine chemical, preferably of lignoceric acid between 37% and 89% or more is conferred.
In one embodiment, in case the activity of the Saccharomyces cerevisae protein YDR513W or its homologs as indicated in Table IIA or IIB, columns 5 or 7, line 256, is increased, preferably, in one embodiment the increase of the respective fine chemical, preferably of melissic acid between 31 % and 108% or more is conferred.
In one embodiment, in case the activity of the Saccharomyces cerevisiae protein YER156C or its homologs as indicated in Table IIA or IIB, columns 5 or 7, line 257, is increased, preferably, in one embodiment an increase of the respective fine chemical, preferably of melissic acid between 33% and 73% or more is conferred.
In one embodiment, in case the activity of the Saccharomyces cerevisiae protein YGL205W or its homologs as indicated in Table IIA or IIB, columns 5 or 7, line 258, is increased, preferably, in one embodiment an increase of the respective fine chemical, preferably of behenic acid between 74% and 132% or more is conferred.
In one embodiment, in case the activity of the Saccharomyces cerevisiae protein YHR201C or its homologs as indicated in Table IIA or IIB, columns 5 or 7, line 259, is increased, preferably, in one embodiment an increase of the respective fine chemical, preferably of cerotic acid between 46% and 227% or more is conferred.
In one embodiment, in case the activity of the Saccharomyces cerevisiae protein YHR201C or its homologs as indicated in Table IIA or IIB, columns 5 or 7, line 259, is increased, preferably, in one embodiment an increase of the respective fine chemical, preferably of lignoceric acid between 29% and 271% or more is conferred.
In one embodiment, in case the activity of the Saccharomyces cerevisiae protein YLR255C or its homologs as indicated in Table IIA or IIB, columns 5 or 7, line 260, is increased, preferably, in one embodiment an increase of the respective fine chemical, preferably of melissic acid between 34% and 88% or more is conferred.
In one embodiment, in case the activity of the Saccharomyces cerevisiae protein YPR138C or its homologs as indicated in Table IIA or IIB, columns 5 or 7, line 261, is increased, preferably, in one embodiment an increase of the respective fine chemical, preferably of lignoceric acid between 92% and 160% or more is conferred.
In one embodiment, in case the activity of the Escherichia coli K12 protein b0255 or its homologs, e.g. as indicated in Table IIA or IIB, columns 5 or 7, line 627 is increased, preferably, in one embodiment the increase of the respective fine chemical, preferably of lignoceric acid between 35% and 115% or more is conferred.

**[0046.0.0.20]** %

**[0047.0.0.20] to [0048.0.0.20]: see [0047.0.0.0] to [0048.0.0.0]**

**[0049.0.20.20]** A protein having an activity conferring an increase in the amount or level of the respective fine chemical lignoceric acid preferably has the structure of the polypeptide described herein. In a particular embodiment, the polypeptides used in the process of the present invention or the polypeptide of the present invention comprises the sequence of a consensus sequence as shown in SEQ ID NO: 27297, 27298 or 27309, 27310 or 27322, 27323, 27324 or 27325, 27326, 27327, 27328, 97769, 97770 or as indicated in Table IV, columns 7, lines 251, 255, 259,261 and 627 or of a polypeptide as indicated in Table IIA or IIB, columns 5 or 7, lines 251, 255, 259 261 and 627 or of a functional homologue thereof as described herein, or of a polypeptide encoded by the nucleic acid molecule characterized herein or the nucleic acid molecule according to the invention, for example by a nucleic acid molecule as indicated in Table IA or IB, columns 5 or 7, lines 251, 255, 259 ,261 or 627 or its herein described functional homologues and has the herein mentioned activity conferring an increase in the lignoceric acid level.
A protein having an activity conferring an increase in the amount or level of the respective fine chemical cerotic acid preferably has the structure of the polypeptide described herein. In a particular embodiment, the polypeptides used in the process of the present invention or the polypeptide of the present invention comprises the sequence of a consensus sequence as shown in SEQ ID NO: 27297, 27298 or 27299, 27300, 27301, 27302 or 27303, 27304 or 27305, 27306, 27307, 27308 or 27322, 27323, 27324 or as indicated in Table IV, columns 7, lines 251, 252, 253, 254 and 259 or of a polypeptide as indicated in Table IIA or IIB, columns 5 or 7, lines 251, 252, 253, 254 and 259 or of a functional homologue thereof as described herein, or of a polypeptide encoded by the nucleic acid molecule characterized herein or the nucleic acid molecule according to the invention, for example by a nucleic acid molecule as indicated in Table IA or IB, columns 5 or 7, lines 251, 252, 253, 254 and 259 or its herein described functional homologues and has the herein mentioned activity conferring an increase in the cerotic acid level.
A protein having an activity conferring an increase in the amount or level of the respective fine chemical melissic acid preferably has the structure of the polypeptide described herein. In a particular embodiment, the polypeptides used in the process of the present invention or the polypeptide of the present invention comprises the sequence of a consensus sequence as shown in SEQ ID NO: 27311, 27312, 27313 or 27314, 27315, 27316, 27317, 27318, 27319 or as indicated in Table IV, columns 7, lines 256 and 257 or of a polypeptide as indicated in Table IIA or IIB, columns 5 or 7, lines 256, 257 and 260 or of a functional homologue thereof as described herein, or of a polypeptide encoded by the nucleic acid molecule characterized herein or the nucleic acid molecule according to the invention, for example by a nucleic acid molecule as indicated in Table IA or IB, columns 5 or 7, lines 256, 257 and 260 or its herein described functional homologues and has the herein mentioned activity conferring an increase in the melissic acid level.
A protein having an activity conferring an increase in the amount or level of the respective fine chemical behenic acid preferably has the structure of the polypeptide described herein. In a particular embodiment, the polypeptides used in the process of the present invention or the polypeptide of the present invention comprises the sequence of a consensus sequence as shown in SEQ ID NO: 27320, 27321 or as indicated in Table IV, columns 7, line 258 or of a polypeptide as indicated in Table IIA or IIB, columns 5 or 7, line 258 or of a functional homologue thereof as described herein, or of a polypeptide encoded by the nucleic acid molecule characterized herein or the nucleic acid molecule according to the invention, for example by a nucleic acid molecule as indicated in Table IA or IB, columns 5 or 7, line 258 or its herein described functional homologues and has the herein mentioned activity conferring an increase in the behenic acid level.

**[0050.0.20.20]** For the purposes of the present invention, the term "the respective fine chemical" also encompass the corresponding salts, such as, for example, the potassium or sodium salts of behenic acid, lignoceric acid, cerotic acid or melissic acid, resp., or their ester, or glucoside thereof, e.g the diglucoside thereof.

**[0051.0.20.20]** Owing to the biological activity of the proteins which are used in the process according to the invention and which are encoded by nucleic acid molecules according to the invention, it is possible to produce compositions comprising the respective fine chemical, i.e. an increased amount of the free chemical free or bound, e.g compositions comprising behenic acid, lignoceric acid, cerotic acid or melissic acid. Depending on the choice of the organism used for the process according to the present invention, for example a microorganism or a plant, compositions or mixtures of behenic acid, lignoceric acid, cerotic acid or melissic acid can be produced.

**[0052.0.0.20] see [0052.0.0.0]**

**[0053.0.20.20]** In one embodiment, the process of the present invention comprises one or more of the following steps .
a) stabilizing a protein conferring the increased expression of a protein encoded by the nucleic acid molecule of the invention or of the polypeptid of the invention, e.g. of a polypeptide having an activity of a protein as indicated in Table IIA or IIB, column 3, lines 251 to 261, 627 or its homologs, e.g. as indicated in Table IIA or IIB, columns 5 or 7, lines 251 to 161, activity having herein-mentioned the respective fine chemical increasing activity;
b) stabilizing a mRNA conferring the increased expression of a protein encoded by the nucleic acid molecule of the invention, e.g. of a polypeptide having an activity of a protein as indicated in Table IIA or IIB, column 3, lines 251 to 261, 627 or its homologs activity, e.g. as indicated in Table IIA or IIB, columns 5 or 7, lines 251 to 261, 627, or of a mRNA encoding the polypeptide of the present invention having herein-mentioned the respective fine chemical increasing activity;
c) increasing the specific activity of a protein conferring the increased expression of a protein encoded by the nucleic acid molecule of the invention or of the polypeptide of the present invention having herein-mentioned the respective fine chemical increasing activity, e.g. of a polypeptide having an activity of a protein as indicated in Table IIA or IIB, column 3, lines 251 to 261, 627 or its homologs activity, e.g. as indicated in Table IIA or IIB, columns 5 or 7, lines 251 to 261, 627, or decreasing the inhibitory regulation of the polypeptide of the invention;
d) generating or increasing the expression of an endogenous or artificial transcription factor mediating the expression of a protein conferring the increased expression of a protein encoded by the nucleic acid molecule of the invention or of the polypeptide of the invention having herein-mentioned the respective fine chemical increasing activity, e.g. of a polypeptide having an activity of a protein as indicated in Table IIA or IIB, column 3, lines 251 to 261, 627 or its homologs activity, e.g. as indicated in Table IIA or IIB, columns 5 or 7, lines 251 to 261, 627;
e) stimulating activity of a protein conferring the increased expression of a protein encoded by the nucleic acid molecule of the present invention or a polypeptide of the present invention having herein-mentioned the respective fine chemical increasing activity, e.g. of a polypeptide having an activity of a protein as indicated in Table IIA or IIB, column 3, lines 251 to 261, 627 or its homologs activity, e.g. as indicated in Table IIA or IIB, columns 5 or 7, lines 251 to 261, 627, by adding one or more exogenous inducing factors to the organism or parts thereof;
f) expressing a transgenic gene encoding a protein conferring the increased expression of a polypeptide encoded by the nucleic acid molecule of the present invention or a polypeptide of the present invention, having herein-mentioned the respective fine chemical increasing activity, e.g. of a polypeptide having an activity of a protein as indicated in Table IIA or IIB, column 3, lines 251 to 261, 627 or its homologs activity, e.g. as indicated in Table IIA or IIB, columns 5 or 7, lines 251 to 261, 627, and/or
g) increasing the copy number of a gene conferring the increased expression of a nucleic acid molecule encoding a polypeptide encoded by the nucleic acid molecule of the invention or the polypeptide of the invention having herein-mentioned the respective fine chemical increasing activity, e.g. of a polypeptide having an activity of a protein as indicated in Table IIA or IIB, column 3, lines 251 to 261, 627 or its homologs, e.g. as indicated in Table IIA or IIB, columns 5 or 7, lines 251 to 261, 627.
h) Increasing the expression of the endogenous gene encoding the polypeptide of the invention, e.g. a polypeptide having an activity of a protein as indicated in Table IIA or IIB, column 3, lines 251 to 261, 627 or its homologs activity, e.g. as indicated in Table IIA or IIB, columns 5 or 7, 251 to 261, 627, by adding positive expression or removing negative expression elements, e.g. homologous recombination can be used to either introduce positive regulatory elements like for plants the 35S enhancer into the promoter or to remove repressor elements form regulatory regions. Further gene conversion methods can be used to disrupt repressor elements or to enhance to activity of positive elements. Positive elements can be randomly introduced in plants by T-DNA or transposon mutagenesis and lines can be identified in which the positive elements have be integrated near to a gene of the invention, the expression of which is thereby enhanced;
   and/or
i) Modulating growth conditions of an organism in such a manner, that the expression or activity of the gene encoding the protein of the invention or the protein itself is enhanced for example microorganisms or plants can be grown for example under a higher temperature regime leading to an enhanced expression of heat shock proteins, which can lead to an enhanced respective fine chemical production.
j) selecting of organisms with especially high activity of the proteins of the invention from natural or from mutagenized resources and breeding them into the target organisms, e.g. the elite crops.

**[0054.0.20.20]** Preferably, said mRNA is the nucleic acid molecule of the present invention and/or the protein conferring the increased expression of a protein encoded by the nucleic acid molecule of the present invention or the polypeptide having the herein mentioned activity is the polypeptide of the present invention, e.g. conferring the increase of the respective fine chemical after increasing the expression or activity of the encoded polypeptide or having the activity of a polypeptide having an activity of a protein as indicated in Table IIA or IIB, columns 3 or 5, lines 251 to 261, 627, resp., or its homologs activity, e.g. as indicated in Table IIA or IIB, columns 5 or 7, lines 251 to 261, 627, resp.

**[0055.0.0.20] to [0067.0.0.20]: see [0055.0.0.0] to [0067.0.0.0]**

**[0068.0.20.20]** The mutation is introduced in such a way that the production of behenic acid, lignoceric acid, cerotic acid or melissic acid is not adversely affected.

**[0069.0.0.20] see [0069.0.0.0]**

**[0070.0.20.20]** Owing to the introduction of a gene or a plurality of genes conferring the expression of the nucleic acid molecule of the invention or the polypeptide of the invention, for example the nucleic acid construct mentioned below, or encoding a protein of the invention into an organism alone or in combination with other genes, it is possible not only to increase the biosynthetic flux towards the end product, but also to increase, modify or create *de novo* an advantageous, preferably novel metabolite composition in the organism, e.g. an advantageous composition of behenic acid, lignoceric acid, cerotic acid and/or melissic acid or their biochemical derivatives , e.g. comprising a higher content of (from a viewpoint of nutrional physiology limited) behenic acid, lignoceric acid, cerotic acid and/or melissic acid or their derivatives.

**[0071.0.0.20] see [0071.0.0.0]**

**[0072.0.0.20]** %

**[0073.0.20.20]** Accordingly, in one embodiment, the process according to the invention relates to a process, which comprises:
providing a non-human organism, preferably a microorganism, a non-human animal, a plant or animal cell, a plant or animal tissue or a plant;
increasing an activity of a polypeptide of the invention or a homolog thereof, e.g. as indicated in Table IIA or IIB, columns 5 or 7, lines 251 to 261, 627, or of a polypeptide being encoded by the nucleic acid molecule of the present invention and described below, e.g. conferring an increase of the respective fine chemical in an organism, preferably in a microorganism, a non-human animal, a plant or animal cell, a plant or animal tissue or a plant,
growing an organism, preferably a microorganism, a non-human animal, a plant or animal cell, a plant or animal tissue or a plant under conditions which permit the production of the respective fine chemical in the organism, preferably the microorganism, the plant cell, the plant tissue or the plant; and
if desired, recovering, optionally isolating, the free and/or bound respective fine chemical synthesized by the organism, the microorganism, the non-human animal, the plant or animal cell, the plant or animal tissue or the plant.

**[0074.0.20.20]** The organism, in particular the microorganism, non-human animal, the plant or animal cell, the plant or animal tissue or the plant is advantageously grown in such a way that it is not only possible to recover, if desired isolate the free or bound respective fine chemical.

**[0075.0.0.20] to [0077.0.0.20]: see [0075.0.0.0] to [0077.0.0.0]**

**[0078.0.20.20]** The organism such as microorganisms or plants or the recovered, and if desired isolated, respective fine chemical can then be processed further directly into foodstuffs or animal feeds or for other applications. The fermentation broth, fermentation products, plants or plant products can be purified with methods known to the person skilled in the art. Products of these different work-up procedures are behenic acid, lignoceric acid, cerotic acid or melissic acid or comprising compositions of behenic acid, lignoceric acid, cerotic acid or melissic acid still comprising fermentation broth, plant particles and cell components in different amounts, advantageously in the range of from 0 to 99% by weight, preferably below 80% by weight, especially preferably below 50% by weight.

**[0079.0.0.20] to [0084.0.0.20]: see [0079.0.0.0] to [0084.0.0.0]**

**[0084.0.0.20]** %

**[0085.0.20.20]** With regard to the nucleic acid sequence as depicted a nucleic acid construct which contains a nucleic acid sequence mentioned herein or an organism (= transgenic organism) which is transformed with said nucleic acid sequence or said nucleic acid construct, "transgene" means all those constructs which have been brought about by genetic manipulation methods, preferably in which either
a) a nucleic acid sequence as indicated in Table IA or IB, columns 5 or 7, lines 251 to 261, 627, or a derivative thereof, or
b) a genetic regulatory element, for example a promoter, which is functionally linked to the nucleic acid sequence as indicated in Table IA or IB, columns 5 or 7, lines 251 to 261, 627, or a derivative thereof, or
c) (a) and (b)
is/are not present in its/their natural genetic environment or has/have been modified by means of genetic manipulation methods, it being possible for the modification to be, by way of example, a substitution, addition, deletion, inversion or insertion of one or more nucleotide. "Natural genetic environment" means the natural chromosomal locus in the organism of origin or the presence in a genomic library. In the case of a genomic library, the natural, genetic environment of the nucleic acid sequence is preferably at least partially still preserved. The environment flanks the nucleic acid sequence at least on one side and has a sequence length of at least 50 bp, preferably at least 500 bp, particularly preferably at least 1000 bp, very particularly preferably at least 5000 bp.

**[0086.0.0.20] to [0087.0.0.20]: see [0086.0.0.0] to [0087.0.0.0]**

**[0088.0.20.20]** In an advantageous embodiment of the invention, the organism takes the form of a plant whose content of the respective fine chemical is modified advantageously owing to the nucleic acid molecule of the present invention expressed. This is important for plant breeders since, for example, the nutritional value of plants for poultry is dependent on the abovementioned fine chemicals or the plants are more resistant to biotic and abiotic stress and the yield is increased.

**[0088.1.0.20] see [0088.1.0.0]**

**[0089.0.0.20] to [0090.0.0.20]: see [0089.0.0.0] to [0090.0.0.0]**

**[0091.0.0.20] see [0091.0.0.0]**

**[0092.0.0.20] to [0094.0.0.20]: see [0092.0.0.0] to [0094.0.0.0]**

**[0095.0.20.20]** It may be advantageous to increase the pool of behenic acid, lignoceric acid, cerotic acid or melissic acid in the transgenic organisms by the process according to the invention in order to isolate high amounts of the pure respective fine chemical and/or to obtain increased resistance against biotic and abiotic stresses and to obtain higher yield.

**[0096.0.20.20]** In another preferred embodiment of the invention a combination of the increased expression of the nucleic acid sequence or the protein of the invention together with the transformation of a protein or polypeptid or a compound, which functions as a sink for the desired fine chemical, for example in the organism, is useful to increase the production of the respective fine chemical.

**[0097.0.0.20]** %

**[0098.0.20.20]** In a preferred embodiment, the respective fine chemical is produced in accordance with the invention and, if desired, is isolated.

**[0099.0.20.20]** In the case of the fermentation of microorganisms, the abovementioned desired fine chemical may accumulate in the medium and/or the cells. If microorganisms are used in the process according to the invention, the fermentation broth can be processed after the cultivation. Depending on the requirement, all or some of the biomass can be removed from the fermentation broth by separation methods such as, for example, centrifugation, filtration, decanting or a combination of these methods, or else the biomass can be left in the fermentation broth. The fermentation broth can subsequently be reduced, or concentrated, with the aid of known methods such as, for example, rotary evaporator, thin-layer evaporator, falling film evaporator, by reverse osmosis or by nanofiltration. Afterwards advantageously further compounds for formulation can be added such as corn starch or silicates. This concentrated fermentation broth advantageously together with compounds for the formulation can subsequently be processed by lyophilization, spray drying, and spray granulation or by other methods. Preferably the respective fine chemical comprising compositions are isolated from the organisms, such as the microorganisms or plants or the culture medium in or on which the organisms have been grown, or from the organism and the culture medium, in the known manner, for example via extraction, distillation, crystallization, chromatography or a combination of these methods. These purification methods can be used alone or in combination with the aforementioned methods such as the separation and/or concentration methods.

**[0100.0.20.20]** Transgenic plants which comprise the fine chemicals such as behenic acid, lignoceric acid, cerotic acid or melissic acid synthesized in the process according to the invention can advantageously be marketed directly without there being any need for the fine chemicals synthesized to be isolated. Plants for the process according to the invention are listed as meaning intact plants and all plant parts, plant organs or plant parts such as leaf, stem, seeds, root, tubers, anthers, fibers, root hairs, stalks, embryos, calli, cotelydons, petioles, harvested material, plant tissue, reproductive tissue and cell cultures which are derived from the actual transgenic plant and/or can be used for bringing about the transgenic plant. In this context, the seed comprises all parts of the seed such as the seed coats, epidermal cells, seed cells, endosperm or embryonic tissue.
However, the respective fine chemical produced in the process according to the invention can also be isolated from the organisms, advantageously plants, as extracts, e.g. ether, alcohol, or other organic solvents or water containing extract and/or free fine chemicals. The respective fine chemical produced by this process can be obtained by harvesting the organisms, either from the crop in which they grow, or from the field. This can be done via pressing or extraction of the plant parts. To increase the efficiency of extraction it is beneficial to clean, to temper and if necessary to hull and to flake the plant material. To allow for greater ease of disruption of the plant parts, specifically the seeds, they can previously be comminuted, steamed or roasted. Seeds, which have been pretreated in this manner can subsequently be pressed or extracted with solvents such as warm hexane. The solvent is subsequently removed. In the case of microorganisms, the latter are, after harvesting, for example extracted directly without further processing steps or else, after disruption, extracted via various methods with which the skilled worker is familiar. Thereafter, the resulting products can be processed further, i.e. degummed and/or refined. In this process, substances such as the plant mucilages and suspended matter can be first removed. What is known as desliming can be affected enzymatically or, for example, chemico-physically by addition of acid such as phosphoric acid.
Because behenic acid, lignoceric acid, cerotic acid or melissic acid in microorganisms are localized intracellular, their recovery essentially comes down to the isolation of the biomass. Well-established approaches for the harvesting of cells include filtration, centrifugation and coagulation/flocculation as described herein. Of the residual hydrocarbon, adsorbed on the cells, has to be removed. Solvent extraction or treatment with surfactants have been suggested for this purpose.

**[0101.0.20.20]** The identity and purity of the compound(s) isolated can be determined by prior-art techniques. They encompass high-performance liquid chromatography (HPLC), gas chromatography (GC), spectroscopic methods, mass spectrometry (MS), staining methods, thin-layer chromatography, NIRS, enzyme assays or microbiological assays. These analytical methods are compiled in: Patek et al. (1994) Appl. Environ. Microbiol. 60:133-140; Malakhova et al. (1996) Biotekhnologiya 11 27-32; and Schmidt et al. (1998) Bioprocess Engineer. 19:67-70. Ulmann's Encyclopedia of Industrial Chemistry (1996) Bd. A27, VCH Weinheim, pp. 89-90, pp. 521-540, pp. 540-547, pp. 559-566, 575-581 and pp. 581-587; Michal, G (1999) Biochemical Pathways: An Atlas of Biochemistry and Molecular Biology, John Wiley and Sons; Fallon, A. et al. (1987) Applications of HPLC in Biochemistry in: Laboratory Techniques in Biochemistry and Molecular Biology, vol. 17.

**[0102.0.20.20]** Behenic acid, lignoceric acid, cerotic acid or melissic acid can for example be analyzed advantageously via HPLC, LC or GC separation and MS (masspectrometry)detection methods. The unambiguous detection for the presence of behenic acid, lignoceric acid, cerotic acid or melissic acid containing products can be obtained by analyzing recombinant organisms using analytical standard methods: LC, LC-MS, MS or TLC). The material to be analyzed can be disrupted by sonication, grinding in a glass mill, liquid nitrogen and grinding, cooking, or via other applicable methods.

**[0103.0.20.20]** In a preferred embodiment, the present invention relates to a process for the production of the respective fine chemical comprising or generating in an organism or a part thereof the expression of at least one nucleic acid molecule comprising a nucleic acid molecule selected from the group consisting of:
a) nucleic acid molecule encoding, preferably at least the mature form, of the polypeptide having a sequence as indicated in Table IIA or IIB, columns 5 or 7, lines 251 to 261, 627, or a fragment thereof, which confers an increase in the amount of the respective fine chemical in an organism or a part thereof;
b) nucleic acid molecule comprising, preferably at least the mature form, of a nucleic acid molecule having a sequence as indicated in Table IA or IB, columns 5 or 7, lines 251 to 261, 627,
c) nucleic acid molecule whose sequence can be deduced from a polypeptide sequence encoded by a nucleic acid molecule of (a) or (b) as result of the degeneracy of the genetic code and conferring an increase in the amount of the respective fine chemical in an organism or a part thereof;
d) nucleic acid molecule encoding a polypeptide which has at least 50% identity with the amino acid sequence of the polypeptide encoded by the nucleic acid molecule of (a) to (c) and conferring an increase in the amount of the respective fine chemical in an organism or a part thereof;
e) nucleic acid molecule which hybridizes with a nucleic acid molecule of (a) to (c) under stringent hybridization conditions and conferring an increase in the amount of the respective fine chemical in an organism or a part thereof;
f) nucleic acid molecule encoding a polypeptide, the polypeptide being derived by substituting, deleting and/or adding one or more amino acids of the amino acid sequence of the polypeptide encoded by the nucleic acid molecules (a) to (d), preferably to (a) to (c) and conferring an increase in the amount of the respective fine chemical in an organism or a part thereof;
g) nucleic acid molecule encoding a fragment or an epitope of a polypeptide which is encoded by one of the nucleic acid molecules of (a) to (e), preferably to (a) to (c) and and conferring an increase in the amount of the respective fine chemical in an organism or a part thereof;
h) nucleic acid molecule comprising a nucleic acid molecule which is obtained by amplifying nucleic acid molecules from a cDNA library or a genomic library using the primer pairs having a sequence as indicated in Table III, columns 7, lines 251 to 261, 627, and conferring an increase in the amount of the respective fine chemical in an organism or a part thereof;
i) nucleic acid molecule encoding a polypeptide which is isolated, e.g. from an expression library, with the aid of monoclonal antibodies against a polypeptide encoded by one of the nucleic acid molecules of (a) to (h), preferably to (a) to (c), and conferring an increase in the amount of the respective fine chemical in an organism or a part thereof;
j) nucleic acid molecule which encodes a polypeptide comprising the consensus sequence having sequences as indicated in Table IV, column 7, lines 251 to 261, 627 and conferring an increase in the amount of the respective fine chemical in an organism or a part thereof;
k) nucleic acid molecule comprising one or more of the nucleic acid molecule encoding the amino acid sequence of a polypeptide encoding a domain of the polypeptide indicated in Table IIA or IIB, columns 5 or 7, lines 251 to 261, 627, and conferring an increase in the amount of the respective fine chemical in an organism or a part thereof; and
l) nucleic acid molecule which is obtainable by screening a suitable library under stringent conditions with a probe comprising one of the sequences of the nucleic acid molecule of (a) to (k), preferably to (a) to (c), or with a fragment of at least 15 nt, preferably 20 nt, 30 nt, 50 nt, 100 nt, 200 nt or 500 nt of the nucleic acid molecule characterized in (a) to (k), preferably to (a) to (c), and conferring an increase in the amount of the respective fine chemical in an organism or a part thereof;
or which comprises a sequence which is complementary thereto.

**[0104.0.20.20]** In one embodiment, the nucleic acid molecule used in the process of the present invention distinguishes over the sequence indicated in Table IA or IB, columns 5 or 7, lines 251 to 261, 627 by one or more nucleotides. In one embodiment, the nucleic acid molecule used in the process of the invention does not consist of the sequence indicated in Table IA or IB, columns 5 or 7, lines 251 to 261, 627. In one embodiment, the nucleic acid molecule of the present invention is less than 100%, 99,999%, 99,99%, 99,9% or 99% identical to the sequence indicated in Table IA or IB, columns 5 or 7, lines 251 to 261, 627. In another embodiment, the nucleic acid molecule does not encode a polypeptide of a sequence indicated in Table IIA or IIB, columns 5 or 7, lines 251 to 261, 627.

**[0105.0.0.20] to [0107.0.0.20]: see [0105.0.0.0] to [0107.0.0.0]**

**[0108.0.20.20]** Nucleic acid molecules with the sequence as indicated in Table IA or IB, columns 5 or 7, lines 251 to 261, 627, nucleic acid molecules which are derived from an amino acid sequences as indicated in Table IIA or IIB, columns 5 or 7, lines 251 to 261, 627 or from polypeptides comprising the consensus sequence as indicated in Table IV, column 7, lines 251 to 261, 627, or their derivatives or homologues encoding polypeptides with the enzymatic or biological activity of an activity of a polypeptide as indicated in Table IIA or IIB, column 3, 5 or 7, lines 251 to 261, 627, e.g. conferring the increase of the respective fine chemical, meaning behenic acid, lignoceric acid, cerotic acid or melissic acid, resp., after increasing its expression or activity, are advantageously increased in the process according to the invention.

**[0109.0.20.20]** In one embodiment, said sequences are cloned into nucleic acid constructs, either individually or in combination. These nucleic acid constructs enable an optimal synthesis of the respective fine chemicals, in particular behenic acid, lignoceric acid, cerotic acid or melissic acid, produced in the process according to the invention.

**[0110.0.0.20] see [0110.0.0.0]**

**[0111.0.0.20] see [0111.0.0.0]**

**[0112.0.20.20]** The nucleic acid molecules used in the process according to the invention take the form of isolated nucleic acid sequences, which encode polypeptides with an activity of a polypeptide as indicated in Table IIA or IIB, column 3, lines 251 to 261, 627 or having the sequence of a polypeptide as indicated in Table IIA or IIB, columns 5 and 7, lines 251 to 261, 627 and conferring an increase in the behenic acid, lignoceric acid, cerotic acid or melissic acid level.

**[0113.0.0.20] to [0114.0.0.20]: see [0113.0.0.0] to [0114.0.0.0]**

**[0115.0.0.20] see [0115.0.0.0]**

**[0116.0.0.20] to [0120.0.0.20] see [0116.0.0.0] to [0120.0.0.0]**

**[0120.1.0.20]: %**

**[0121.0.20.20]** However, it is also possible to use artificial sequences, which differ in one or more bases from the nucleic acid sequences found in organisms, or in one or more amino acid molecules from polypeptide sequences found in organisms, in particular from the polypeptide sequences indicated in Table IIA or IIB, columns 5 or 7, lines 251 to 261, 627 or the functional homologues thereof as described herein, preferably conferring above-mentioned activity, i.e. conferring a lignoceric acid level increase after increasing the activity of the polypeptide sequences indicated in Table IIA or IIB, columns 5 or 7, lines 251, 255, 259 or 261, conferring a cerotic acid level increase after increasing the activity of the polypeptide sequences indicated in Table IIA or IIB, columns 5 or 7, lines 251, 252, 253, 254 or 259, conferring a melissic acid level increase after increasing the activity of the polypeptide sequences indicated in Table IIA or IIB, columns 5 or 7, lines 256, 257 or 260 or conferring a behenic acid level increase after increasing the activity of the polypeptide sequences indicated in Table IIA or IIB, columns 5 or 7, line 258.

**[0122.0.0.20] to [0127.0.0.20]: see [0122.0.0.0] to [0127.0.0.0]**

**[0128.0.20.20]** Synthetic oligonucleotide primers for the amplification, e.g. as the pairs indicated in Table III, columns 7, lines 251 to 261, 627, by means of polymerase chain reaction can be generated on the basis of a sequence shown herein, for example the sequence as indicated in Table IA or IB, columns 5 or 7, lines 251 to 261, 627, resp. or the sequences derived from a sequences as indicated in Table IIA or IIB, columns 5 or 7, lines 251 to 261, 627, resp.

**[0129.0.20.20]** Moreover, it is possible to identify conserved regions from various organisms by carrying out protein sequence alignments with the polypeptide used in the process of the invention, in particular with sequences of the polypeptide of the invention, from which conserved regions, and in turn, degenerate primers can be derived. Conserved regions are those, which show a very little variation in the amino acid in one particular position of several homologs from different origin. The consensus sequence indicated in Table IV, columns 7, lines 251 to 259, 261 and 627 is derived from such alignments.

**[0130.0.20.20]** Degenerated primers can then be utilized by PCR for the amplification of fragments of novel proteins having above-mentioned activity, e.g. conferring the increase of behenic acid, lignoceric acid, cerotic acid or melissic acid after increasing the expression or activity the protein comprising said fragment.

**[0131.0.0.20] to [0138.0.0.20]: see [0131.0.0.0] to [0138.0.0.0]**

**[0139.0.20.20]** Polypeptides having above-mentioned activity, i.e. conferring the respective fine chemical level increase, derived from other organisms, can be encoded by other DNA sequences which hybridise to a sequence indicated in Table IA or IB, columns 5 our 7, lines 251, 255, 259, 261 or 627 for lignoceric acid or indicated in Table IA or IB, columns 5 or 7, lines 251, 252, 253, 254 or 259 for cerotic acid or indicated in Table IA or IB, columns 5 or 7, lines 256, 257 or 260 for melissic acid or indicated in Table IA or IB, columns 5 or 7, line 258 for benic acid under relaxed hybridization conditions and which code on expression for peptides having the respective fine chemical, i.e. behenic acid, lignoceric acid, cerotic acid or melissic acid, resp., increasing-activity.

**[0140.0.0.20] to [0146.0.0.20]: see [0140.0.0.0] to [0146.0.0.0]**

**[0147.0.20.20]** Further, the nucleic acid molecule of the invention comprises a nucleic acid molecule, which is a complement of one of the nucleotide sequences of above mentioned nucleic acid molecules or a portion thereof. A nucleic acid molecule which is complementary to one of the nucleotide sequences indicated in Table IA or IB, columns 5 or 7, lines 251 to 261, 627, preferably the nucleic acid molecule of the invention is a functional homologue or identical to a nucleic acid molecule indicated in Table IB, columns 5 or 7, lines 251 to 261, 627, is one which is sufficiently complementary to one of said nucleotide sequences such that it can hybridise to one of said nucleotide sequences, thereby forming a stable duplex. Preferably, the hybridisation is performed under stringent hybrization conditions. However, a complement of one of the herein disclosed sequences is preferably a sequence complement thereto according to the base pairing of nucleic acid molecules well known to the skilled person. For example, the bases A and G undergo base pairing with the bases T and U or C, resp. and visa versa. Modifications of the bases can influence the base-pairing partner.

**[0148.0.20.20]** The nucleic acid molecule of the invention comprises a nucleotide sequence which is at least about 30%, 35%, 40% or 45%, preferably at least about 50%, 55%, 60% or 65%, more preferably at least about 70%, 80%, or 90%, and even more preferably at least about 95%, 97%, 98%, 99% or more homologous to a nucleotide sequence indicated in Table IA or IB, columns 5 or 7, lines 251 to 261, 627 or a portion thereof and preferably has above mentioned activity, in particular having a behenic acid, lignoceric acid, cerotic acid or melissic acid increasing activity after increasing the acitivity or an activity of a product of a gene encoding said sequences or their homologs.

**[0149.0.20.20]** The nucleic acid molecule of the invention comprises a nucleotide sequence which hybridizes, preferably hybridizes under stringent conditions as defined herein, to one of the nucleotide sequences indicated in Table IA or IB, columns 5 or 7, lines 251 to 261, 627, preferably the nucleic acid molecule of the invention is a functional homologue or identical to a nucleic acid molecule indicated in Table IB, columns 5 or 7, lines 251 to 261, 627, or a portion thereof and encodes a protein having above-mentioned activity, e.g. conferring an increase of behenic acid, lignoceric acid, cerotic acid or melissic acid, resp., and optionally, the activity of protein indicated in Table IIA or IIB, column 5, lines 251 to 261, 627.

**[00149.1.20.20]** Optionally, in one embodiment, the nucleotide sequence, which hybridises to one of the nucleotide sequences indicated in Table IA or IB, columns 5 or 7, lines 251 to 261, 627, preferably the nucleic acid molecule of the invention is a functional homologue or identical to a nucleic acid molecule indicated in Table IB, columns 5 or 7, lines 251 to 261, 627, has further one or more of the activities annotated or known for a protein as indicated in Table IIA or IIB, column 3, lines 251 to 261,627.

**[0150.0.20.20]** Moreover, the nucleic acid molecule of the invention can comprise only a portion of the coding region of one of the sequences indicated in Table IA or IB, columns 5 or 7, lines 251 to 261, 627 preferably the nucleic acid molecule of the invention is a functional homologue or identical to a nucleic acid molecule indicated in Table IB, columns 5 or 7, lines 251 to 261, 627 for example a fragment which can be used as a probe or primer or a fragment encoding a biologically active portion of the polypeptide of the present invention or of a polypeptide used in the process of the present invention, i.e. having above-mentioned activity, e.g.conferring an increase of behenic acid, lignoceric acid, cerotic acid or melissic acid , resp., if its activity is increased. The nucleotide sequences determined from the cloning of the present protein-according-to-the-invention-encoding gene allows for the generation of probes and primers designed for use in identifying and/or cloning its homologues in other cell types and organisms. The probe/primer typically comprises substantially purified oligonucleotide. The oligonucleotide typically comprises a region of nucleotide sequence that hybridizes under stringent conditions to at least about 12, 15 preferably about 20 or 25, more preferably about 40, 50 or 75 consecutive nucleotides of a sense strand of one of the sequences set forth, e.g., as indicated in Table IA or IB, columns 5 or 7, lines 251 to 261, 627, an anti-sense sequence of one of the sequences, e.g., as indicated in Table IA or IB, columns 5 or 7, lines 251 to 261, 627, or naturally occurring mutants thereof. Primers based on a nucleotide of invention can be used in PCR reactions to clone homologues of the polypeptide of the invention or of the polypeptide used in the process of the invention, e.g. as the primers described in the examples of the present invention, e.g. as shown in the examples. A PCR with the primer pairs indicated in I Table III, column 7, lines 251 to 261, 627 will result in a fragment of a polynucleotide sequence as indicated in Table IA or IB, columns 5 or 7, lines 251 to 261, 627 or its gene product.

**[0151.0.0.20]: see [0151.0.0.0]**

**[0152.0.20.20]** The nucleic acid molecule of the invention encodes a polypeptide or portion thereof which includes an amino acid sequence which is sufficiently homologous to an amino acid sequence as indicated in Table IIA or IIB, columns 5 or 7, lines 251 to 261, 627 such that the protein or portion thereof maintains the ability to participate in the respective fine chemical production, in particular a lignoceric acid (lines 251, 255, 259, 261, 627) or cerotic acid (lines 251, 252, 253, 254, 259) or melissic acid (lines 256, 257, 260) or behenic acid (line 258) increasing activity as mentioned above or as described in the examples in plants or microorganisms is comprised.

**[0153.0.20.20]** As used herein, the language "sufficiently homologous" refers to proteins or portions thereof which have amino acid sequences which include a minimum number of identical or equivalent amino acid residues (e.g., an amino acid residue which has a similar side chain as an amino acid residue in one of the sequences of the polypeptide of the present invention) to an amino acid sequence as indicated in Table IIA or IIB, columns 5 or 7, lines 251 to 261, 627 such that the protein or portion thereof is able to participate in the increase of the respective fine chemical production. In one embodiment, a protein or portion thereof as indicated in Table IIA or IIB, columns 5 or 7, lines 251 to 261, 627 has for example an activity of a polypeptide indicated in Table IIA or IIB, column 3, lines 251 to 261, 627.

**[0154.0.20.20]** In one embodiment, the nucleic acid molecule of the present invention comprises a nucleic acid that encodes a portion of the protein of the present invention. The protein is at least about 30%, 35%, 40%, 45% or 50%, preferably at least about 55%, 60%, 65% or 70%, and more preferably at least about 75%, 80%, 85%, 90%, 91%, 92%, 93% or 94% and most preferably at least about 95%, 97%, 98%, 99% or more homologous to an entire amino acid sequence as indicated in Table IIA or IIB, columns 5 or 7, lines 251 to 261, 627 and has above-mentioned activity, e.g.conferring preferably the increase of the respective fine chemical.

**[0155.0.0.20] to [0156.0.0.20]: see [0155.0.0.0] to [0156.0.0.0]**

**[0157.0.20.20]** The invention further relates to nucleic acid molecules that differ from one of the nucleotide sequences as indicated in Table IA or IB, columns 5 or 7, lines 251 to 261, 627 (and portions thereof) due to degeneracy of the genetic code and thus encode a polypeptide of the present invention, in particular a polypeptide having above mentioned activity, e.g. conferring an increase in the respective fine chemical in a organism, e.g. as polypeptides comprising the sequence as indicated in Table IV, column 7, lines 251 to 261, 627 or as polypeptides depicted in Table IIA or IIB, columns 5 or 7, lines 251 to 261, 627 or the functional homologues. Advantageously, the nucleic acid molecule of the invention comprises, or in an other embodiment has, a nucleotide sequence encoding a protein comprising, or in an other embodiment having, an amino acid sequence of a consensus sequences as indicated in Table IV, column 7, lines 251 to 259 and 261 or of the polypeptide as indicated in Table IIA or IIB, columns 5 or 7, lines 251 to 261, 627, resp., or the functional homologues. In a still further embodiment, the nucleic acid molecule of the invention encodes a full length protein which is substantially homologous to an amino acid sequence comprising a consensus sequence as indicated in Table IV, column 7, lines 251 to 259 and 261, 627 or of a polypeptide as indicated in Table IIA or IIB, columns 5 or 7, lines 251 to 261, 627 or the functional homologues. However, in a preferred embodiment, the nucleic acid molecule of the present invention does not consist of a sequence as indicated in Table IA or IB, columns 5 or 7, lines 251 to 261, 627, resp.

**[0158.0.0.20] to [0160.0.0.20]: see [0158.0.0.0] to [0160.0.0.0]**

**[0161.0.20.20]** Accordingly, in another embodiment, a nucleic acid molecule of the invention is at least 15, 20, 25 or 30 nucleotides in length. Preferably, it hybridizes under stringent conditions to a nucleic acid molecule comprising a nucleotide sequence of the nucleic acid molecule of the present invention or used in the process of the present invention, e.g. comprising a sequence as indicated in Table IA or IB, columns 5 or 7, lines 251 to 261, 627. The nucleic acid molecule is preferably at least 20, 30, 50, 100, 250 or more nucleotides in length.

**[0162.0.0.20] see [0162.0.0.0]**

**[0163.0.20.20]** Preferably, a nucleic acid molecule of the invention that hybridizes under stringent conditions to a sequence as indicated in Table IA or IB, columns 5 or 7, lines 251 to 261, 627 corresponds to a naturally-occurring nucleic acid molecule of the invention. As used herein, a "naturally-occurring" nucleic acid molecule refers to an RNA or DNA molecule having a nucleotide sequence that occurs in nature (e.g., encodes a natural protein). Preferably, the nucleic acid molecule encodes a natural protein having above-mentioned activity, e.g. conferring the increase of the amount of the respective fine chemical in an organism or a part thereof, e.g. a tissue, a cell, or a compartment of a cell, after increasing the expression or activity thereof or the activity of a protein of the invention or used in the process of the invention.

**[0164.0.0.20] see [0164.0.0.0]**

**[0165.0.20.20]** For example, nucleotide substitutions leading to amino acid substitutions at "non-essential" amino acid residues can be made in a sequence of the nucleic acid molecule of the invention or used in the process of the invention, e.g. as indicated in Table IA or IB, columns 5 or 7, lines 251 to 261, 627, resp.

**[0166.0.0.20] to [0167.0.0.20]: see [0166.0.0.0] to [0167.0.0.0]**

**[0168.0.20.20]** Accordingly, the invention relates to nucleic acid molecules encoding a polypeptide having above-mentioned activity, e.g. conferring an increase in the respective fine chemical in an organisms or parts thereof that contain changes in amino acid residues that are not essential for said activity. Such polypeptides differ in amino acid sequence from a sequence contained in a sequence as indicated in Table IIA or IIB, columns 5 or 7, lines 251 to 261, 627, resp., yet retain said activity described herein. The nucleic acid molecule can comprise a nucleotide sequence encoding a polypeptide, wherein the polypeptide comprises an amino acid sequence at least about 50% identical to an amino acid sequence as indicated in Table IIA or IIB, columns 5 or 7, lines 251 to 261, 627, resp., and is capable of participation in the increase of production of the respective fine chemical after increasing its activity, e.g. its expression. Preferably, the protein encoded by the nucleic acid molecule is at least about 60% identical to a sequence as indicated in Table IIA or IIB, columns 5 or 7, lines 251 to 261, 627, resp., more preferably at least about 70% identical to one of the sequences as indicated in Table IIA or IIB, columns 5 or 7, lines 251 to 261, 627, resp., even more preferably at least about 80%, 90%, 95% homologous to a sequence as indicated in Table IIA or IIB, columns 5 or 7, lines 251 to 261, 627, resp., and most preferably at least about 96%, 97%, 98%, or 99% identical to the sequence as indicated in Table IIA or IIB, columns 5 or 7, lines 251 to 261, 627.

**[0169.0.0.20] to [0172.0.0.20]: see [0169.0.0.0] to [0172.0.0.0]**

**[0173.0.20.20]** For example a sequence, which has 80% homology with sequence SEQ ID NO: 25525 at the nucleic acid level is understood as meaning a sequence which, upon comparison with the sequence SEQ ID NO: 25525 by the above Gap program algorithm with the above parameter set, has a 80% homology.

**[0174.0.0.20]: see [0174.0.0.0]**

**[0175.0.20.20]** For example a sequence which has a 80% homology with sequence SEQ ID NO: 25526 at the protein level is understood as meaning a sequence which, upon comparison with the sequence SEQ ID NO: 25526 by the above program algorithm with the above parameter set, has a 80% homology.

**[0176.0.20.20]** Functional equivalents derived from one of the polypeptides as indicated in Table IIA or IIB, columns 5 or 7, lines 251 to 261, 627, resp., according to the invention by substitution, insertion or deletion have at least 30%, 35%, 40%, 45% or 50%, preferably at least 55%, 60%, 65% or 70% by preference at least 80%, especially preferably at least 85% or 90%, 91%, 92%, 93% or 94%, very especially preferably at least 95%, 97%, 98% or 99% homology with one of the polypeptides as indicated in Table IIA or IIB, columns 5 or 7, lines 251 to 261, 627, resp., according to the invention and are distinguished by essentially the same properties as a polypeptide as indicated in Table IIA or IIB, columns 5 or 7, lines 251 to 261, 627, resp.

**[0177.0.20.20]** Functional equivalents derived from a nucleic acid sequence as indicated in Table IA or IB, columns 5 or 7, lines 251 to 261, 627, resp., according to the invention by substitution, insertion or deletion have at least 30%, 35%, 40%, 45% or 50%, preferably at least 55%, 60%, 65% or 70% by preference at least 80%, especially preferably at least 85% or 90%, 91 %, 92%, 93% or 94%, very especially preferably at least 95%, 97%, 98% or 99% homology with one of the polypeptides as indicated in Table IIA or IIB, columns 5 or 7, lines 251 to 261, 627, resp., according to the invention and encode polypeptides having essentially the same properties as a polypeptide as indicated in Table IIA or IIB, columns 5 or 7, lines 251 to 261, 627, resp.

**[0178.0.0.20] see [0178.0.0.0]**

**[0179.0.20.20]** A nucleic acid molecule encoding a homologue to a protein sequence as indicated in Table IIA or IIB, columns 5 or 7, lines 251 to 261, 627, resp., can be created by introducing one or more nucleotide substitutions, additions or deletions into a nucleotide sequence of the nucleic acid molecule of the present invention, in particular as indicated in Table IA or IB, columns 5 or 7, lines 251 to 261, 627, resp., such that one or more amino acid substitutions, additions or deletions are introduced into the encoded protein. Mutations can be introduced into the encoding sequences as indicated in Table IA or IB, columns 5 or 7, lines 251 to 261, 627, resp., by standard techniques, such as site-directed mutagenesis and PCR-mediated mutagenesis.

**[0180.0.0.20] to [0183.0.0.20]: see [0180.0.0.0] to [0183.0.0.0]**

**[0184.0.20.20]** Homologues of the nucleic acid sequences used, with a sequence as indicated in Table IA or IB, columns 5 or 7, lines 251 to 261, 627, resp., or of the nucleic acid sequences derived from a sequences as indicated in Table IIA or IIB, columns 5 or 7, lines 251 to 261, 627, resp., comprise also allelic variants with at least approximately 30%, 35%, 40% or 45% homology, by preference at least approximately 50%, 60% or 70%, more preferably at least approximately 90%, 91%, 92%, 93%, 94% or 95% and even more preferably at least approximately 96%, 97%, 98%, 99% or more homology with one of the nucleotide sequences shown or the abovementioned derived nucleic acid sequences or their homologues, derivatives or analogues or parts of these. Allelic variants encompass in particular functional variants which can be obtained by deletion, insertion or substitution of nucleotides from the sequences shown, preferably from a sequence as indicated in Table IA or IB, columns 5 or 7, lines 251 to 261, 627, resp., or from the derived nucleic acid sequences, the intention being, however, that the enzyme activity or the biological activity of the resulting proteins synthesized is advantageously retained or increased.

**[0185.0.20.20]** In one embodiment of the present invention, the nucleic acid molecule of the invention or used in the process of the invention comprises one or more sequences as indicated in Table IA or IB, columns 5 or 7, lines 251 to 261, 627, resp. In one embodiment, it is preferred that the nucleic acid molecule comprises as little as possible other nucleotides not shown in any one of sequences as indicated in Table IA or IB, columns 5 or 7, lines 251 to 261, 627, resp. In one embodiment, the nucleic acid molecule comprises less than 500, 400, 300, 200, 100, 90, 80, 70, 60, 50 or 40 further nucleotides. In a further embodiment, the nucleic acid molecule comprises less than 30, 20 or 10 further nucleotides. In one embodiment, a nucleic acid molecule used in the process of the invention is identical to a sequence as indicated in Table IA or IB, columns 5 or 7, lines 251 to 261, 627, resp.

**[0186.0.20.20]** Also preferred is that one or more nucleic acid molecule(s) used in the process of the invention encodes a polypeptide comprising a sequence as indicated in Table IIA or IIB, columns 5 or 7, lines 251 to 261, 627, resp. In one embodiment, the nucleic acid molecule encodes less than 150, 130, 100, 80, 60, 50, 40 or 30 further amino acids. In a further embodiment, the encoded polypeptide comprises less than 20, 15, 10, 9, 8, 7, 6 or 5 further amino acids. In one embodiment, the encoded polypeptide used in the process of the invention is identical to the sequences as indicated in Table IIA or IIB, columns 5 or 7, lines 251 to 261, 627, resp.

**[0187.0.20.20]** In one embodiment, a nucleic acid molecule of the invention or used in the process encodes a polypeptide comprising the sequence as indicated in Table IIA or IIB, columns 5 or 7, lines 251 to 261, 627, resp., comprises less than 100 further nucleotides. In a further embodiment, said nucleic acid molecule comprises less than 30 further nucleotides. In one embodiment, the nucleic acid molecule used in the process is identical to a coding sequence encoding a sequences as indicated in Table IIA or IIB, columns 5 or 7, lines 251 to 261, 627, resp.

**[0188.0.20.20]** Polypeptides (= proteins), which still have the essential enzymatic activity of the polypeptide of the present invention conferring an increase of the respective fine chemical i.e. whose activity is essentially not reduced, are polypeptides with at least 10% or 20%, by preference 30% or 40%, especially preferably 50% or 60%, very especially preferably 80% or 90 or more of the wild type biological activity or enzyme activity. Advantageously, the activity is essentially not reduced in comparison with the activity of a polypeptide as indicated in Table IIA or IIB, columns 5 or 7, lines 251 to 261, 627, resp., and is expressed under identical conditions.

**[0189.0.20.20]** Homologues of a sequences as indicated in Table IA or IB, columns 5 or 7, lines 251 to 261, 627, resp., or of derived sequences as indicated in Table IIA or IIB, columns 5 or 7, lines 251 to 261, 627, resp., also mean truncated sequences, cDNA, single-stranded DNA or RNA of the coding and noncoding DNA sequence. Homologues of said sequences are also understood as meaning derivatives, which comprise noncoding regions such as, for example, UTRs, terminators, enhancers or promoter variants. The promoters upstream of the nucleotide sequences stated can be modified by one or more nucleotide substitution(s), insertion(s) and/or deletion(s) without, however, interfering with the functionality or activity either of the promoters, the open reading frame (=ORF) or with the 3'-regulatory region such as terminators or other 3'regulatory regions, which are far away from the ORF. It is furthermore possible that the activity of the promoters is increased by modification of their sequence, or that they are replaced completely by more active promoters, even promoters from heterologous organisms. Appropriate promoters are known to the person skilled in the art and are mentioned herein below.

**[0190.0.0.20]: see [0190.0.0.0]**

**[0191.0.20.20]** The organisms or part thereof produce according to the herein mentioned process of the invention an increased level of free and/or bound the respective fine chemical compared to said control or selected organisms or parts thereof.

**[0192.0.0.20] to [0203.0.0.20]: see [0192.0.0.0] to [0203.0.0.0]**

**[0204.0.20.20]** Accordingly, in one embodiment, the invention relates to a nucleic acid molecule, which comprises a nucleic acid molecule selected from the group consisting of:
a) nucleic acid molecule encoding, preferably at least the mature form, of a polypeptide as indicated in Table IIA or IIB, columns 5 or 7, lines 251 to 261, 627, resp.; or a fragment thereof conferring an increase in the amount of the respective fine chemical, i.e. lignoceric acid (lines 251, 255, 259, 261, 627) or cerotic acid (lines 251, 252, 253, 254, 259) or melissic acid (lines 256, 257, 260) or behenic acid (line 258), resp., in an organism or a part thereof,
b) nucleic acid molecule comprising, preferably at least the mature form, of a nucleic acid molecule as indicated in Table IA or IB, columns 5 or 7, lines 251 to 261, 627, resp., or a fragment thereof conferring an increase in the amount of the respective fine chemical in an organism or a part thereof;
c) nucleic acid molecule whose sequence can be deduced from a polypeptide sequence encoded by a nucleic acid molecule of (a) or (b) as result of the degeneracy of the genetic code and conferring an increase in the amount of the respective fine chemical in an organism or a part thereof;
d) nucleic acid molecule encoding a polypeptide whose sequence has at least 50% identity with the amino acid sequence of the polypeptide encoded by the nucleic acid molecule of (a) to (c) and conferring an increase in the amount of the respective fine chemical in an organism or a part thereof;
e) nucleic acid molecule which hybridizes with a nucleic acid molecule of (a) to (c) under stringent hybridisation conditions and conferring an increase in the amount of the respective fine chemical in an organism or a part thereof;
f) nucleic acid molecule encoding a polypeptide, the polypeptide being derived by substituting, deleting and/or adding one or more amino acids of the amino acid sequence of the polypeptide encoded by the nucleic acid molecules (a) to (d), preferably to (a) to (c), and conferring an increase in the amount of the respective fine chemical in an organism or a part thereof;
g) nucleic acid molecule encoding a fragment or an epitope of a polypeptide which is encoded by one of the nucleic acid molecules of (a) to (e), preferably to (a) to (c) and conferring an increase in the amount of the respective fine chemical in an organism or a part thereof;
h) nucleic acid molecule comprising a nucleic acid molecule which is obtained by amplifying a cDNA library or a genomic library using primers or primer pairs as indicated in I Table III, columns 5 or 7, lines 251 to 261, 627 and conferring an increase in the amount of the respective fine chemical, i.e. lignoceric acid (lines 251, 255, 259, 261, 627) or cerotic acid (lines 251, 252, 253, 254, 259) or melissic acid (lines 256, 257, 260) or behenic acid (line 258), resp., in an organism or a part thereof;
i) nucleic acid molecule encoding a polypeptide which is isolated, e.g. from a expression library, with the aid of monoclonal antibodies against a polypeptide encoded by one of the nucleic acid molecules of (a) to (g), preferably to (a) to (c) and conferring an increase in the amount of the respective fine chemical in an organism or a part thereof;
j) nucleic acid molecule which encodes a polypeptide comprising a consensus sequence as indicated in Table IV, column 7, lines 252 to 259,261 and 627 and conferring an increase in the amount of the respective fine chemical, i.e. lignoceric acid (lines 251, 255, 259, 261, 627) or cerotic acid (lines 251, 252, 253, 254, 259) or melissic acid (lines 256, 257, 260) or behenic acid (line 258), resp., in an organism or a part thereof;
k) nucleic acid molecule encoding the amino acid sequence of a polypeptide encoding a domaine of a polypeptide as indicated in Table IIA or IIB, columns 5 or 7, lines 251 to 261, 627 , resp., and conferring an increase in the amount of the respective fine chemical, i.e. lignoceric acid (lines 251, 255, 259, 261, 627) or cerotic acid (lines 251, 252, 253, 254, 259) or melissic acid (lines 256, 257, 260) or behenic acid (line 258) resp., in an organism or a part thereof; and
l) nucleic acid molecule which is obtainable by screening a suitable nucleic acid library under stringent hybridization conditions with a probe comprising one of the sequences of the nucleic acid molecule of (a) to (k) or with a fragment of at least 15 nt, preferably 20 nt, 30 nt, 50 nt, 100 nt, 200 nt or 500 nt of the nucleic acid molecule characterized in (a) to (h) or of a nucleic acid molecule as indicated in Table IA or IB, columns 5 or 7, lines 251 to 261, 627, resp., or a nucleic acid molecule encoding, preferably at least the mature form of, a polypeptide as indicated in Table IIA or IIB, columns 5 or 7, lines 251 to 261, 627, resp., and conferring an increase in the amount of the respective fine chemical in an organism or a part thereof; or which encompasses a sequence which is complementary thereto;
whereby, preferably, the nucleic acid molecule according to (a) to (I) distinguishes over a sequence as indicated in Table IA or IB, columns 5 or 7, lines 251 to 261, 627, resp., by one or more nucleotides. In one embodiment, the nucleic acid molecule of the invention does not consist of the sequence as indicated in Table IA or IB, columns 5 or 7, lines 251 to 261, 627, resp. In another embodiment, the nucleic acid molecule of the present invention is at least 30 % identical and less than 100%, 99,999%, 99,99%, 99,9% or 99% identical to a sequence as indicated in Table IA or IB, columns 5 or 7, lines 251 to 261, 627, resp. In a further embodiment the nucleic acid molecule does not encode a polypeptide sequence as indicated in Table IIA or IIB, columns 5 or 7, lines 251 to 261, 627, resp. Accordingly, in one embodiment, the nucleic acid molecule of the present invention encodes in one embodiment a polypeptide which differs at least in one or more amino acids from a polypeptide indicated in Table IIA or IIB, columns 5 or 7, lines 251 to 261, 627 does not encode a protein of a sequence as indicated in Table IIA or IIB, columns 5 or 7, lines 251 to 261, 627. Accordingly, in one embodiment, the protein encoded by a sequences of a nucleic acid accoriding to (a) to (I) does not consist of a sequence as indicated in Table IIA or IIB, columns 5 or 7, lines 251 to 261, 627. In a further embodiment, the protein of the present invention is at least 30 % identical to a protein sequence indicated in Table IIA or IIB, columns 5 or 7, lines 251 to 261, 627 and less than 100%, preferably less than 99,999%, 99,99% or 99,9%, more preferably less than 99%, 985, 97%, 96% or 95% identical to a sequence as indicated in Table IIA or IIB, columns 5 or 7, lines 251 to 261, 627.

**[0205.0.0.20] to [0206.0.0.20]: see [0205.0.0.0] to [0206.0.0.0]**

**[0207.0.20.20]** As described herein, the nucleic acid construct can also comprise further genes, which are to be introduced into the organisms or cells. It is possible and advantageous to introduce into, and express in, the host organisms regulatory genes such as genes for inductors, repressors or enzymes, which, owing to their enzymatic activity, engage in the regulation of one or more genes of a biosynthetic pathway. These genes can be of heterologous or homologous origin. Moreover, further biosynthesis genes may advantageously be present, or else these genes may be located on one or more further nucleic acid constructs. Genes, which are advantageously employed as biosynthesis genes, are genes of the glutamic acid metabolism, the phosphoenolpyruvate metabolism, the amino acid metabolism, of glycolysis, of the tricarboxylic acid metabolism or their combinations. As described herein, regulator sequences or factors can have a positive effect on preferably the gene expression of the genes introduced, thus increasing it. Thus, an enhancement of the regulator elements may advantageously take place at the transcriptional level by using strong transcription signals such as promoters and/or enhancers. In addition, however, an enhancement of translation is also possible, for example by increasing mRNA stability or by inserting a translation enhancer sequence.

**[0208.0.0.20] to [0226.0.0.20]: see [0208.0.0.0] to [0226.0.0.0]**

**[0227.0.20.20]** The abovementioned nucleic acid molecules can be cloned into the nucleic acid constructs or vectors according to the invention in combination together with further genes, or else different genes are introduced by transforming several nucleic acid constructs or vectors (including plasmids) into a host cell, advantageously into a plant cell or a microorgansims.
In addition to a sequence indicated in Table IA or IB, columns 5 or 7, lines 251 to 261, 627 or its derivatives, it is advantageous to express and/or mutate further genes in the organisms. Especially advantageously, additionally at least one further gene of the acetyl-CoA or malonyl-CoA metabolic pathway or a polypeptide having a very long chain fatty acid acyl (VLCFA) CoA synthase activity, is expressed in the organisms such as plants or microorganisms. It is also possible that the regulation of the natural genes has been modified advantageously so that the gene and/or its gene product is no longer subject to the regulatory mechanisms which exist in the organisms. This leads to an increased synthesis of the fine chemicals desired since, for example, feedback regulations no longer exist to the same extent or not at all. In addition it might be advantageously to combine one or more of the sequences indicated in Table IA or IB, columns 5 or 7, lines 251 to 261, 627, resp., with genes which generally support or enhance to growth or yield of the target organismen, for example genes which lead to faster growth rate of microorganisms or genes which produces stress-, pathogen, or herbicide resistant plants.

**[0228.0.20.20] %**

**[0229.0.20.20]** Further advantageous nucleic acid sequences which can be expressed in combination with the sequences indicated in Table IA or IB, columns 5 or 7, lines 251 to 261, 627 used in the process and/or the abovementioned biosynthesis genes are the sequences encoding further genes of the fatty acid pathway, such as acetyl-CoA or malonyl-CoA or a polypeptide having a very long chain fatty acid acyl (VLCFA) CoA synthase activity. These genes can lead to an increased synthesis of the VLCFAs.

**[0230.0.0.20] see [230.0.0.0]**

**[0231.0.20.20]** In a further advantageous embodiment of the process of the invention, the organisms used in the process are those in which simultaneously a behenic acid, lignoceric acid, cerotic acid or melissic acid degrading protein is attenuated, in particular by reducing the rate of expression of the corresponding gene. A person skilled in the art knows for example, that the inhibition or repression of a behenic acid, lignoceric acid, cerotic acid or melissic acid degrading enzyme will result in an increased accumulation of behenic acid, lignoceric acid, cerotic acid or melissic acid in plants.

**[0232.0.0.20] to [0276.0.0.20]: see [0232.0.0.0] to [0276.0.0.0]**

**[0277.0.20.20]** The respective fine chemical produced can be isolated from the organism by methods with which the skilled worker are familiar, for example via extraction, salt precipitation, and/or different chromatography methods. The process according to the invention can be conducted batchwise, semibatchwise or continuously.

**[0278.0.0.20] to [0282.0.0.20]: see [0278.0.0.0] to [0282.0.0.0]**

**[0283.0.20.20]** Moreover, native polypeptide conferring the increase of the respective fine chemical in an organism or part thereof can be isolated from cells, for example using the antibody of the present invention as described below, e.g. an antibody against a protein as indicated in Table IIA or IIB, column 3, lines 251 to 261, 627, resp., or an antibody against a polypeptide as indicated in Table IIA or IIB, columns 5 or 7, lines 251 to 261, 627, resp., which can be produced by standard techniques utilizing the polypeptid of the present invention or fragment thereof. Preferred are monoclonal antibodies.

**[0284.0.0.20] see [0284.0.0.0]**

**[0285.0.20.20]** In one embodiment, the present invention relates to a polypeptide having a sequence as indicated in Table IIA or IIB, columns 5 or 7, lines 251 to 261, 627, resp., or as coded by a nucleic acid molecule as indicated in Table IA or IB, columns 5 or 7, lines 251 to 261, 627, resp., or functional homologues thereof.

**[0286.0.20.20]** In one advantageous embodiment, in the method of the present invention the activity of a polypeptide is increased which comprises or consists of a consensus sequence as indicated in Table IV, column 7, lines 251 to 259 and 261 and 627 and in one another embodiment, the present invention relates to a polypeptide comprising or consisting of a consensus sequence as indicated in Table IV, column 7, lines 251 to 259 and 261 and 627 whereby 20 or less, preferably 15 or 10, preferably 9, 8, 7, or 6, more preferred 5 or 4, even more preferred 3, even more preferred 2, even more preferred 1, most preferred 0 of the amino acids positions indicated can be replaced by any amino acid or, in an further embodiment, can be replaced and/or absent. In one embodiment, the present invention relates to the method of the present invention comprising a polypeptide or to a polypeptide comprising more than one consensus sequences (of an individual line) as indicated in Table IV, column 7, lines 251 to 259, 261 and 627,

**[0287.0.0.20] to [0289.0.0.20]: see [0287.0.0.0] to [0289.0.0.0]**

**[00290.0.20.20]** The alignment was performed with the Software AlignX (sept 25, 2002) a component of Vector NTI Suite 8.0, InforMas™, Invitrogen™ life science software, U.S. Main Office, 7305 Executive Way,Frederick, MD 21704,USA with the following settings: For pairwise alignments: gap opening penality: 10,0; gap extension penality 0,1. For multiple alignments: Gap opening penalty: 10,0; Gap extension penalty: 0,1; Gap separation penalty range: 8; Residue substitution matrix: blosum62; Hydrophilic residues: G P S N D Q E K R; Transition weighting: 0,5; Consensus calculation options: Residue fraction for consensus: 0,9. Presettings were selected to allow also for the alignment of conserved amino acids

**[0291.0.20.20]** In one advantageous embodiment, the method of the present invention comprises the increasing of a polypeptide comprising or consisting of plant or microorganism specific consensus sequences.
In one embodiment, said polypeptide of the invention distinguishes over a sequence as indicated in Table IIA or IIB, columns 5 or 7, lines 251 to 261, 627, resp., by one or more amino acids. In one embodiment, polypeptide distinguishes from a sequence as indicated in Table IIA or IIB, columns 5 or 7, lines 251 to 261, 627, resp., by more than 5, 6, 7, 8 or 9 amino acids, preferably by more than 10, 15, 20, 25 or 30 amino acids, even more preferred are more than 40, 50, or 60 amino acids and, preferably, the sequence of the polypeptide of the invention distinguishes from a sequence as indicated in Table IIA or IIB, columns 5 or 7, lines 251 to 261, 627, resp., by not more than 80% or 70% of the amino acids, preferably not more than 60% or 50%, more preferred not more than 40% or 30%, even more preferred not more than 20% or 10%. In another embodiment, said polypeptide of the invention does not consist of a sequence as indicated in Table IIA or IIB, columns 5 or 7, lines 251 to 261, 627.

**[0292.0.0.20] see [0292.0.0.0]**

**[0293.0.20.20]** In one embodiment, the invention relates to a polypeptide conferring an increase in the fine chemical in an organism or part being encoded by the nucleic acid molecule of the invention or by a nucleic acid molecule used in the process of the invention. In one embodiment, the polypeptide of the invention has a sequence which distinguishes from a sequence as indicated in Table IIA or IIB, columns 5 or 7, lines 251 to 261, 627, resp., by one or more amino acids. In another embodiment, said polypeptide of the invention does not consist of the sequence as indicated in Table IIA or IIB, columns 5 or 7, lines 251 to 261, 627, resp. In a further embodiment, said polypeptide of the present invention is less than 100%, 99,999%, 99,99%, 99,9% or 99% identical. In one embodiment, said polypeptide does not consist of the sequence encoded by a nucleic acid molecules as indicated in Table IA or IB, columns 5 or 7, lines 251 to 261, 627, resp.

**[0294.0.20.20]** In one embodiment, the present invention relates to a polypeptide having an activity of a protein as indicated in Table IIA or IIB, column 3, lines 251 to 261, 627, resp., which distinguishes over a sequence as indicated in Table IIA or IIB, columns 5 or 7, lines 251 to 261, 627, resp., by one or more amino acids, preferably by more than 5, 6, 7, 8 or 9 amino acids, preferably by more than 10, 15, 20, 25 or 30 amino acids, even more preferred are more than 40, 50, or 60 amino acids but even more preferred by less than 70% of the amino acids, more preferred by less than 50%, even more preferred my less than 30% or 25%, more preferred are 20% or 15%, even more preferred are less than 10%.

**[0295.0.0.20] to [0296.0.0.20]: see [0295.0.0.0] to [0296.0.0.0]**

**[0297.0.0.20] see [0297.0.0.0]**

**[00297.1.0.20] %**

**[0298.0.20.20]** A polypeptide of the invention can participate in the process of the present invention. The polypeptide or a portion thereof comprises preferably an amino acid sequence which is sufficiently homologous to an amino acid sequence as indicated in Table IIA or IIB, columns 5 or 7, lines 251 to 261, 627, resp. The portion of the protein is preferably a biologically active portion as described herein. Preferably, the polypeptide used in the process of the invention has an amino acid sequence identical to a sequence as indicated in Table IIA or IIB, columns 5 or 7, lines 251 to 261, 627, resp.

**[0299.0.20.20]** Further, the polypeptide can have an amino acid sequence which is encoded by a nucleotide sequence which hybridizes, preferably hybridizes under stringent conditions as described above, to a nucleotide sequence of the nucleic acid molecule of the present invention. Accordingly, the polypeptide has an amino acid sequence which is encoded by a nucleotide sequence that is at least about 35%, 40%, 45%, 50%, 55%, 60%, 65% or 70%, preferably at least about 75%, 80%, 85% or 90, and more preferably at least about 91%, 92%, 93%, 94% or 95%, and even more preferably at least about 96%, 97%, 98%, 99% or more homologous to one of the nucleotide sequence as indicated in Table IA or IB, columns 5 or 7, lines 251 to 261, 627, resp. The preferred polypeptide of the present invention preferably possesses at least one of the activities according to the invention and described herein. A preferred polypeptide of the present invention includes an amino acid sequence encoded by a nucleotide sequence which hybridizes, preferably hybridizes under stringent conditions, to a nucleotide sequence as indicated in Table IA or IB, columns 5 or 7, lines 251 to 261, 627, resp., or which is homologous thereto, as defined above.

**[0300.0.20.20]** Accordingly the polypeptide of the present invention can vary from a sequence as indicated in Table IIA or IIB, columns 5 or 7, lines 251 to 261, 627, resp., in amino acid sequence due to natural variation or mutagenesis, as described in detail herein. Accordingly, the polypeptide comprise an amino acid sequence which is at least about 35%, 40%, 45%, 50%, 55%, 60%, 65% or 70%, preferably at least about 75%, 80%, 85% or 90, and more preferably at least about 91%, 92%, 93%, 94% or 95%, and most preferably at least about 96%, 97%, 98%, 99% or more homologous to an entire amino acid sequence of as indicated in Table IIA or IIB, columns 5 or 7, lines 251 to 261, 627, resp.

**[0301.0.0.20] see [0301.0.0.0]**

**[0302.0.20.20]** Biologically active portions of an polypeptide of the present invention include peptides comprising amino acid sequences derived from the amino acid sequence of the polypeptide of the present invention or used in the process of the present invention, e.g., an amino acid sequence as indicated in Table IIA or IIB, columns 5 or 7, lines 251 to 261, 627, resp., or the amino acid sequence of a protein homologous thereto, which include fewer amino acids than a full length polypeptide of the present invention or used in the process of the present invention or the full length protein which is homologous to an polypeptide of the present invention or used in the process of the present invention depicted herein, and exhibit at least one activity of polypeptide of the present invention or used in the process of the present invention.

**[0303.0.0.20] see [0303.0.0.0]**

**[0304.0.20.20]** Manipulation of the nucleic acid molecule of the invention may result in the production of a protein having essentially the activity of the polypeptides as indicated in Table IIA or IIB, column 3, lines 251 to 261, 627 but having differences in the sequence from said wild-type protein. These proteins may be improved in efficiency or activity, may be present in greater numbers in the cell than is usual, or may be decreased in efficiency or activity in relation to the wild type protein.

**[0305.0.0.20] to [0308.0.0.20]: see [0305.0.0.0] to [0308.0.0.0]**

**[0309.0.20.20]** In one embodiment, a reference to a protein (= polypeptide) of the invention or as indicated in Table IIA or IIB, columns 5 or 7, lines 251 to 261, 627, resp., refers to a polypeptide having an amino acid sequence corresponding to the polypeptide of the invention or used in the process of the invention, whereas an "other polypeptide" not being indicated in Table IIA or IIB, columns 5 or 7, lines 251 to 261, 627, resp., refers to a polypeptide having an amino acid sequence corresponding to a protein which is not substantially homologous to a polypeptide of the invention, preferably which is not substantially homologous to a polypeptide as indicated in Table IIA or IIB, columns 5 or 7, lines 251 to 261, 627, resp., e.g., a protein which does not confer the activity described herein or annotated or known for as indicated in Table IIA or IIB, column 3, lines 251 to 261, 627, resp., and which is derived from the same or a different organism. In one embodiment, an "other polypeptide" not being indicated in Table IIA or IIB, columns 5 or 7, lines 251 to 261, 627, resp., does not confer an increase of the respective fine chemical in an organism or part thereof.

**[0310.0.0.20] to [0334.0.0.20]: see [0310.0.0.0] to [0334.0.0.0]**

**[0335.0.20.20]** The dsRNAi method has proved to be particularly effective and advantageous for reducing the expression of a nucleic acid sequences as indicated in Table IA or IB, columns 5 or 7, lines 251 to 261, 627, resp., and/or homologs thereof. As described inter alia in WO 99/32619, dsRNAi approaches are clearly superior to traditional antisense approaches. The invention therefore furthermore relates to double-stranded RNA molecules (dsRNA molecules) which, when introduced into an organism, advantageously into a plant (or a cell, tissue, organ or seed derived there from), bring about altered metabolic activity by the reduction in the expression of a nucleic acid sequences as indicated in Table IA or IB, columns 5 or 7, lines 251 to 261, 627, resp., and/or homologs thereof. In a double-stranded RNA molecule for reducing the expression of a protein encoded by a nucleic acid sequence as indicated in Table IA or IB, columns 5 or 7, lines 251 to 261, 627, resp., and/or homologs thereof, one of the two RNA strands is essentially identical to at least part of a nucleic acid sequence, and the respective other RNA strand is essentially identical to at least part of the complementary strand of a nucleic acid sequence.

**[0336.0.0.20] to [0342.0.0.20]: see [0336.0.0.0] to [0342.0.0.0]**

**[0343.0.20.20]** As has already been described, 100 % sequence identity between the dsRNA and a gene transcript of a nucleic acid sequence as indicated in Table IA or IB, columns 5 or 7, lines 251 to 261, 627, resp., or it's homolog is not necessarily required in order to bring about effective reduction in the expression. The advantage is, accordingly, that the method is tolerant with regard to sequence deviations as may be present as a consequence of genetic mutations, polymorphisms or evolutionary divergences. Thus, for example, using the dsRNA, which has been generated starting from a sequence as indicated in Table IA or IB, columns 5 or 7, lines 251 to 261, 627, resp., or homologs thereof of the one organism, may be used to suppress the corresponding expression in another organism.

**[0344.0.0.20] to [0350.0.0.20]: see [0344.0.0.0] to [0350.0.0.0]**

**[0351.0.0.20] to [0361.0.0.20]: see [0351.0.0.0] to [0361.0.0.0]**

**[0362.0.20.20]** Accordingly the present invention relates to any cell transgenic for any nucleic acid characterized as part of the invention, e.g. conferring the increase of the respective fine chemical in a cell or an organism or a part thereof, e.g. the nucleic acid molecule of the invention, the nucleic acid construct of the invention, the antisense molecule of the invention, the vector of the invention or a nucleic acid molecule encoding the polypeptide of the invention, e.g. the polypeptide as indicated in Table IIA or IIB, columns 5 or 7, lines 251 to 261, 627, resp., e.g. encoding a polypeptide having protein activity, as indicated in Table IIA or IIB, columns 3, lines 251 to 261, 627, resp. Due to the above-mentioned activity the respective fine chemical content in a cell or an organism is increased. For example, due to modulation or manipulation, the cellular activity of the polypeptide of the invention or nucleic acid molecule of the invention is increased, e.g. due to an increased expression or specific activity of the subject matters of the invention in a cell or an organism or a part thereof. Transgenic for a polypeptide having an activity of a polypeptide as indicated in Table IIA or IIB, columns 5 or 7, lines 251 to 261, 627, resp., means herein that due to modulation or manipulation of the genome, an activity as annotated for a polypeptide as indicated in Table IIA or IIB, column 3, lines 251 to 261, 627, e.g. having a sequence as indicated in Table IIA or IIB, columns 5 or 7, lines 251 to 261, 627, resp., is increased in a cell or an organism or a part thereof. Examples are described above in context with the process of the invention.

**[0363.0.0.20] see [0363.0.0.0]**

**[0364.0.20.20]** A naturally occurring expression cassette - for example the naturally occurring combination of a promoter of a gene encoding a polypeptide of the invention as indicated in Table IIA or IIB, column 3, lines 251 to 261, 627, resp. with the corresponding protein-encoding sequence as indicated in Table IA or IB, column 5, lines 251 to 261, 627, resp., becomes a transgenic expression cassette when it is modified by non-natural, synthetic "artificial" methods such as, for example, mutagenization. Such methods have been described (US 5,565,350; WO 00/15815; also see above).

**[0365.0.0.20] to [0373.0.0.20]: see [0365.0.0.0] to [0373.0.0.0]**

**[0374.0.20.20]** Transgenic plants comprising the respective fine chemical synthesized in the process according to the invention can be marketed directly without isolation of the compounds synthesized. In the process according to the invention, plants are understood as meaning all plant parts, plant organs such as leaf, stalk, root, tubers or seeds or propagation material or harvested material or the intact plant. In this context, the seed encompasses all parts of the seed such as the seed coats, epidermal cells, seed cells, endosperm or embryonic tissue, behenic acid, lignoceric acid, cerotic acid or melissic acid, in particular the respective fine chemical, produced in the process according to the invention may, however, also be isolated from the plant in the form of their free behenic acid, lignoceric acid, cerotic acid or melissic acid, in particular the free respective fine chemical, or bound in or to compounds or moieties, like glucosides, e.g. diglucosides. The respective fine chemical produced by this process can be harvested by harvesting the organisms either from the culture in which they grow or from the field. This can be done via expressing, grinding and/or extraction, salt precipitation and/or ion-exchange chromatography or other chromatographic methods of the plant parts, preferably the plant seeds, plant fruits, plant tubers and the like.

**[0375.0.0.20] to [0376.0.0.20]: see [0375.0.0.0] to [0376.0.0.0]**

**[0377.0.20.20]** Accordingly, the present invention relates also to a process whereby the produced behenic acid, lignoceric acid, cerotic acid or melissic acid is isolated.

**[0378.0.20.20]** In this manner, more than 50% by weight, advantageously more than 60% by weight, preferably more than 70% by weight, especially preferably more than 80% by weight, very especially preferably more than 90% by weight, of the behenic acid, lignoceric acid, cerotic acid or melissic acid produced in the process can be isolated. The resulting behenic acid, lignoceric acid, cerotic acid or melissic acid can, if appropriate, subsequently be further purified, if desired mixed with other active ingredients such as vitamins, amino acids, carbohydrates, antibiotics and the like, and, if appropriate, formulated.

**[0379.0.20.20]** In one embodiment the product produced by the present invention is a mixture of the respective fine chemicals behenic acid, lignoceric acid, cerotic acid and/or melissic acid.

**[0380.0.20.20]** The behenic acid, lignoceric acid, cerotic acid or melissic acid obtained in the process by carrying out the invention is suitable as starting material for the synthesis of further products of value. For example, they can be used in combination with each other or alone for the production of pharmaceuticals, foodstuffs, animal feeds or cosmetics. Accordingly, the present invention relates to a method for the production of pharmaceuticals, food stuff, animal feeds, nutrients or cosmetics comprising the steps of the process according to the invention, including the isolation of the behenic acid, lignoceric acid, cerotic acid or melissic acid composition produced or the respective fine chemical produced if desired and formulating the product with a pharmaceutical acceptable carrier or formulating the product in a form acceptable for an application in agriculture. A further embodiment according to the invention is the use of the behenic acid, lignoceric acid, cerotic acid or melissic acid produced in the process or of the transgenic organism in animal feeds, foodstuffs, medicines, food supplements, cosmetics or pharmaceuticals or for the production of behenic acid, lignoceric acid, cerotic acid or melissic acid e.g. after isolation of the respective fine chemical or without, e.g. in situ, e.g in the organism used for the process for the production of the respective fine chemical.

**[0381.0.0.20] to [0382.0.0.20]: see [0381.0.0.0] to [0382.0.0.0]**

**[0383.0.20.20]**

**[0384.0.0.20] see [0384.0.0.0]**

**[0385.0.20.20]** The fermentation broths obtained in this way, containing in particular behenic acid, lignoceric acid, cerotic acid or melissic acid in mixtures with other organic acids, amino acids, polypeptides or polysaccarides, normally have a dry matter content of from 1 to 70% by weight, preferably 7.5 to 25% by weight. Sugar-limited fermentation is additionally advantageous, e.g. at the end, for example over at least 30% of the fermentation time. This means that the concentration of utilizable sugar in the fermentation medium is kept at, or reduced to, 0 to 10 g/I, preferably to 0 to 3 g/I during this time. The fermentation broth is then processed further. Depending on requirements, the biomass can be removed or isolated entirely or partly by separation methods, such as, for example, centrifugation, filtration, decantation, coagulation/flocculation or a combination of these methods, from the fermentation broth or left completely in it.
The fermentation broth can then be thickened or concentrated by known methods, such as, for example, with the aid of a rotary evaporator, thin-film evaporator, falling film evaporator, by reverse osmosis or by nanofiltration. This concentrated fermentation broth can then be worked up by freeze-drying, spray drying, spray granulation or by other processes.

**[0386.0.20.20]** Accordingly, it is possible to purify the behenic acid, lignoceric acid, cerotic acid or melissic acid produced according to the invention further. For this purpose, the product-containing composition is subjected for example to separation via e.g. an open column chromatography or HPLC in which case the desired product or the impurities are retained wholly or partly on the chromatography resin. These chromatography steps can be repeated if necessary, using the same or different chromatography resins. The skilled worker is familiar with the choice of suitable chromatography resins and their most effective use.

**[0387.0.0.20] to [0392.0.0.20]: see [0387.0.0.0] to [0392.0.0.0]**

**[0393.0.20.20]** In one embodiment, the present invention relates to a method for the identification of a gene product conferring an increase in the respective fine chemical production in a cell, comprising the following steps:
a. contacting,̅ e.g. hybridising, the nucleic acid molecules of a sample, e.g. cells, tissues, plants or microorganisms or a nucleic acid library , which can contain a candidate gene encoding a gene product conferring an increase in the respective fine chemical after expression, with the nucleic acid molecule of the present invention;
b. identifying the nucleic acid molecules, which hybridize under relaxed stringent conditions with the nucleic acid molecule of the present invention in particular to the nucleic acid molecule sequence as indicated in Table IA or IB, columns 5 or 7, lines 251 to 261, 627, resp., and, optionally, isolating the full length cDNA clone or complete genomic clone;
c. introducing the candidate nucleic acid molecules in host cells, preferably in a plant cell or a microorganism, appropriate for producing the respective fine chemical;
d. expressing the identified nucleic acid molecules in the host cells;
e. assaying the respective fine chemical level in the host cells; and
f. identifying the nucleic acid molecule and its gene product which expression confers an increase in the respective fine chemical level in the host cell after expression compared to the wild type.

**[0394.0.0.20] to [0398.0.0.20]: see [0394.0.0.0] to [0398.0.0.0]**

**[0399.0.20.20]** Furthermore, in one embodiment, the present invention relates to process for the identification of a compound conferring increase of the respective fine chemical production in a plant or microorganism, comprising the steps:
culturing a cell or tissue or microorganism or maintaining a plant expressing the polypeptide according to the invention or a nucleic acid molecule encoding said polypeptide and a readout system capable of interacting with the polypeptide under suitable conditions which permit the interaction of the polypeptide with said readout system in the presence of a compound or a sample comprising a plurality of compounds and capable of providing a detectable signal in response to the binding of a compound to said polypeptide under conditions which permit the expression of said readout system and the polypeptide of the present invention or used in the process of the invention; and
identifying if the compound is an effective agonist by detecting the presence or absence or increase of a signal produced by said readout system.
The screen for a gene product or an agonist conferring an increase in the respective fine chemical production can be performed by growth of an organism for example a microorganism in the presence of growth reducing amounts of an inhibitor of the synthesis of the respective fine chemical. Better growth, e.g. higher dividing rate or high dry mass in comparison to the control under such conditions would identify a gene or gene product or an agonist conferring an increase in respective fine chemical production.

[00399.1.20.20] One can think to screen for increased production of the respective fine chemical by for example searching for a resistance to a drug blocking the synthesis of the respective fine chemical and looking whether this effect is dependent on the activity or expression of a polypeptide as indicated in Table IIA or IIB, columns 5 or 7, lines 251 to 261, 627 or a homolog thereof, e.g. comparing the phenotype of nearly identical organisms with low and high activity of a protein as indicated in Table IIA or IIB, columns 5 or 7, lines 251 to 261, 627 after incubation with the drug.

**[0400.0.0.20] to [0415.0.0.20]: see [0400.0.0.0] to [0415.0.0.0]**

**[0416.0.0.20] see [0416.0.0.0]**

**[0417.0.20.20]** The nucleic acid molecule of the invention, the vector of the invention or the nucleic acid construct of the invention may also be useful for the production of organisms resistant to inhibitors of the behenic acid, lignoceric acid, cerotic acid or melissic acid biosynthesis pathways. In particular, the overexpression of the polypeptide of the present invention may protect an organism such as a microorganism or a plant against inhibitors, which block the behenic acid, lignoceric acid, cerotic acid or melissic acid synthesis.
Examples for such inhibitors are oxyacetamides, oxyacetamides or chloroacetamides which inhibit the first step in VLCFAs biosynthesis, the condensation of acyl-CoA with malonyl-CoA to β-ketoacyl-CoA (Matthes, 2001; Schmalfuss, J., Matthes, B, and P. Böger: Chloroacetamide mode of action. Abstr. Meeting Weed Science Society of America, Toronto, 40, 117 - 118, 2000). Therefore a plant overproducing behenic acid, lignoceric acid, cerotic acid and/or melissic acid could result in the resistance to chloroacetamide-type herbicides.

**[0418.0.0.20] to [0423.0.0.20]: see [0418.0.0.0] to [0423.0.0.0]**

**[0424.0.20.20]** Accordingly, the nucleic acid of the invention, the polypeptide of the invention, the nucleic acid construct of the invention, the organisms, the host cell, the microorgansims, the plant, plant tissue, plant cell, or the part thereof of the invention, the vector of the invention, the agonist identified with the method of the invention, the nucleic acid molecule identified with the method of the present invention, can be used for the production of the respective fine chemical or of the respective fine chemical and one or more other organic acids. Accordingly, the nucleic acid of the invention, or the nucleic acid molecule identified with the method of the present invention or the complement sequences thereof, the polypeptide of the invention, the nucleic acid construct of the invention, the organisms, the host cell, the microorganisms, the plant, plant tissue, plant cell, or the part thereof of the invention, the vector of the invention, the antagonist identified with the method of the invention, the antibody of the present invention, the antisense molecule of the present invention, can be used for the reduction of the respective fine chemical in a organism or part thereof, e.g. in a cell.

**[0425.0.0.20] to [0434.0.0.0]: see [0425.0.0.0] to [0434.0.0.0]**

**[0435.0.20.20]** Example 3: In-vivo and in-vitro mutagenesis

**[0436.0.20.20]** An *in vivo* mutagenesis of organisms such as Saccharomyces, Mortierella, Escherichia and others mentioned above, which are beneficial for the production of behenic acid, lignoceric acid, cerotic acid or melissic acid can be carried out by passing a plasmid DNA (or another vector DNA) containing the desired nucleic acid sequence or nucleic acid sequences, e.g. the nucleic acid molecule of the invention or the vector of the invention, through E.coli and other microorganisms (for example Bacillus spp. or yeasts such as Saccharomyces cerevisiae) which are not capable of maintaining the integrity of its genetic information. Usual mutator strains have mutations in the genes for the DNA repair system [for example mutHLS, mutD, mutT and the like; for comparison, see Rupp, W.D. (1996) DNA repair mechanisms in Escherichia coli and Salmonella, pp. 2277-2294, ASM: Washington]. The skilled worker knows these strains. The use of these strains is illustrated for example in Greener, A. and Callahan, M. (1994) Strategies 7; 32-34.
In-vitro mutation methods such as increasing the spontaneous mutation rates by chemical or physical treatment are well known to the skilled person. Mutagens like 5-bromo-uracil, N-methyl-N-nitro-N-nitrosoguanidine (= NTG), ethyl methanesulfonate (=EMS), hydroxylamine and/or nitrous acid are widly used as chemical agents for random in-vitro mutagenesis. The most common physical method for mutagensis is the treatment with UV irradiation. Another random mutagenesis technique is the error-prone PCR for introducing amino acid changes into proteins. Mutations are deliberately introduced during PCR through the use of error-prone DNA polymerases and special reaction conditions known to a person skilled in the art. For this method randomized DNA sequences are cloned into expression vectors and the resulting mutant libraries screened for altered or improved protein activity as described below.
Site-directed mutagensis method such as the introduction of desired mutations with an M13 or phagemid vector and short oligonucleotides primers is a well-known approach for site-directed mutagensis. The clou of this method involves cloning of the nucleic acid sequence of the invention into an M13 or phagemid vector, which permits recovery of single-stranded recombinant nucleic acid sequence. A mutagenic oligonucleotide primer is then designed whose sequence is perfectly complementary to nucleic acid sequence in the region to be mutated, but with a single difference: at the intended mutation site it bears a base that is complementary to the desired mutant nucleotide rather than the original. The mutagenic oligonucleotide is then allowed to prime new DNA synthesis to create a complementary full-length sequence containing the desired mutation. Another site-directed mutagensis method is the PCR mismatch primer mutagensis method also known to the skilled person. DpnI site-directed mutagensis is a further known method as described for example in the Stratagene QuickchangeTM site-directed mutagenesis kit protocol. A huge number of other methods are also known and used in common practice.
Positive mutation events can be selected by screening the organisms for the production of the desired respective fine chemical.

**[0437.0.20.20]** Example 4: DNA transfer between Escherichia coli, Saccharomyces cerevisiae and Mortierella alpina

**[0438.0.20.20]** Shuttle vectors such as pYE22m, pPAC-ResQ, pClasper, pAUR224, pAMH10, pAML10, pAMT10, pAMU10, pGMH10, pGML10, pGMT10, pGMU10, pPGAL1, pPADH1, pTADH1, pTAex3, pNGA142, pHT3101 and derivatives thereof which allow the transfer of nucleic acid sequences between Escherichia coli, Saccharomyces cerevisiae and/or Mortierella alpina are available to the skilled worker. An easy method to isolate such shuttle vectors is disclosed by Soni R. and Murray J.A.H. [Nucleic Acid Research, vol. 20 no. 21, 1992: 5852]: If necessary such shuttle vectors can be constructed easily using standard vectors for E. coli (Sambrook, J. et al., (1989), "Molecular Cloning: A Laboratory Manual", Cold Spring Harbor Laboratory Press or Ausubel, F.M. et al. (1994) "Current Protocols in Molecular Biology", John Wiley & Sons) and/or the aforementioned vectors, which have a replication origin for, and suitable marker from, Escherichia coli, Saccharomyces cerevisiae or Mortierella alpina added. Such replication origins are preferably taken from endogenous plasmids, which have been isolated from species used in the inventive process. Genes, which are used in particular as transformation markers for these species are genes for kanamycin resistance (such as those which originate from the Tn5 or Tn-903 transposon) or for chloramphenicol resistance (Winnacker, E.L. (1987) "From Genes to Clones - Introduction to Gene Technology", VCH, Weinheim) or for other antibiotic resistance genes such as for G418, gentamycin, neomycin, hygromycin or tetracycline resistance.

**[0439.0.20.20]** Using standard methods, it is possible to clone a gene of interest into one of the above-described shuttle vectors and to introduce such hybrid vectors into the microorganism strains used in the inventive process. The transformation of Saccharomyces can be achieved for example by LiCl or sheroplast transformation (Bishop et al., Mol. Cell. Biol., 6, 1986: 3401 - 3409; Sherman et al., Methods in Yeasts in Genetics, [Cold Spring Harbor Lab. Cold Spring Harbor, N. Y.] 1982, Agatep et al., Technical Tips Online 1998, 1:51: P01525 or Gietz et al., Methods Mol. Cell. Biol. 5, 1995: 255f) or electroporation (Delorme E., Appl. Environ. Microbiol., vol. 55, no. 9, 1989 : 2242 - 2246).

**[0440.0.20.20]** If the transformed sequence(s) is/are to be integrated advantageously into the genome of the microorganism used in the inventive process for example into the yeast or fungi genome, standard techniques known to the skilled worker also exist for this purpose. Solinger et al. (Proc Natl Acad Sci U S A., 2001 (15): 8447-8453) and Freedman et al. (Genetics, Vol. 162, 15-27, September 2002,) teaches a homolog recombination system dependent on rad 50, rad51, rad54 and rad59 in yeasts. Vectors using this system for homologous recombination are vectors derived from the Ylp series. Plasmid vectors derived for example from the 2µ-Vector are known by the skilled worker and used for the expression in yeasts. Other preferred vectors are for example pART1, pCHY21 or pEVP11 as they have been described by McLeod et al. (EMBO J. 1987, 6:729-736) and Hoffman et al. (Genes Dev. 5, 1991: :561-571.) or Russell et al. (J. Biol. Chem. 258, 1983: 143-149.). Other beneficial yeast vectors are plasmids of the REP, REP-X, pYZ or RIP series.

**[0441.0.0.20] see [0441.0.0.0]**

**[0442.0.0.20] see [0442.0.0.0]**

**[0443.0.0.20] see [0443.0.0.0]**

**[0444.0.20.20]** Example 6: Growth of genetically modified organism: media and culture conditions

**[0445.0.20.20]** Example 8: Genetically modified Yeast, Mortierella or Escherichia coli are grown in synthetic or natural growth media known by the skilled worker. A number of different growth media for Yeast, Mortierella or Escherichia coli are well known and widely available. A method for culturing Mortierella is disclosed by Jang et al. [Bot. Bull. Acad. Sin. (2000) 41: 41-48]. Mortierella can be grown at 20°C in a culture medium containing: 10 g/l glucose, 5 g/l yeast extract at pH 6.5. Futhermore Jang et al. teaches a submerged basal medium containing 20 g/I soluble starch, 5 g/l Bacto yeast extract, 10 g/l KNO₃, 1 g/l KH₂PO₄, and 0.5 g/l MgSO₄•7H₂O, pH 6.5.

**[0446.0.0.20] to [0450.0.0.20]: see [0446.0.0.0] to [0450.0.0.0]**

**[0451.0.0.20] see [0451.0.5.5]**

**[0452.0.0.20] to [0453.0.0.20]: see [0452.0.0.0] to [0453.0.0.0]**

**[0454.0.20.20]** Example 8: Analysis of the effect of the nucleic acid molecule on the production of behenic acid, lignoceric acid, cerotic acid or melissic acid

**[0455.0.20.20]** The effect of the genetic modification in plants, fungi, algae, ciliates or on the production of a desired compound (such as a behenic acid, lignoceric acid, cerotic acid or melissic acid) can be determined by growing the modified microorganisms or the modified plant under suitable conditions (such as those described above) and analyzing the medium and/or the cellular components for the elevated production of desired product (i.e. of behenic acid, lignoceric acid, cerotic acid or melissic acid). These analytical techniques are known to the skilled worker and comprise spectroscopy, thin-layer chromatography, various types of staining methods, enzymatic and microbiological methods and analytical chromatography such as high-performance liquid chromatography (see, for example, Ullman, Encyclopedia of Industrial Chemistry, Vol. A2, p. 89-90 and p. 443-613, VCH: Weinheim (1985); Fallon, A., et al., (1987) "Applications of HPLC in Biochemistry" in: Laboratory Techniques in Biochemistry and Molecular Biology, Vol. 17; Rehm et al. (1993) Biotechnology, Vol. 3, Chapter III: "Product recovery and purification", p. 469-714, VCH: Weinheim; Belter, P.A., et al. (1988) Bioseparations: downstream processing for Biotechnology, John Wiley and Sons; Kennedy, J.F., and Cabral, J.M.S. (1992) Recovery processes for biological Materials, John Wiley and Sons; Shaeiwitz, J.A., and Henry, J.D. (1988) Biochemical Separations, in: Ullmann's Encyclopedia of Industrial Chemistry, Vol. B3; Chapter 11, p. 1-27, VCH: Weinheim; and Dechow, F.J. (1989) Separation and purification techniques in biotechnology, Noyes Publications).

**[0456.0.0.20]: see [0456.0.0.0]**

**[0457.0.20.20]** Example 9: Purification of behenic acid, lignoceric acid, cerotic acid or melissic acid

**[0458.0.20.20]** Abbreviations; GC-MS, gas liquid chromatography/mass spectrometry; TLC, thin-layer chromatography.
The unambiguous detection for the presence of behenic acid, lignoceric acid, cerotic acid or melissic acid can be obtained by analyzing recombinant organisms using analytical standard methods: LC, LC-MSMS, GC-MS or TLC, as described. The total amount produced in the organism for example in yeasts used in the inventive process can be analysed for example according to the following procedure:
The material such as yeasts, E. coli or plants to be analyzed can be disrupted by sonication, grinding in a glass mill, liquid nitrogen and grinding or via other applicable methods.
Plant material is initially homogenized mechanically by comminuting in a pestle and mortar to make it more amenable to extraction.
A typical sample pretreatment consists of a total lipid extraction using such polar organic solvents as acetone or alcohols as methanol, or ethers, saponification, partition between phases, seperation of non-polar epiphase from more polar hypophasic derivatives and chromatography.
For analysis, solvent delivery and aliquot removal can be accomplished with a robotic system comprising a single injector valve Gilson 232XL and a 402 2S1V diluter [Gilson, Inc. USA, 3000 W. Beltline Highway, Middleton, WI]. For saponification, 3 ml of 50% potassium hydroxide hydro-ethanolic solution (4 water - 1 ethanol) can be added to each vial, followed by the addition of 3 ml of octanol. The saponification treatment can be conducted at room temperature with vials maintained on an IKA HS 501 horizontal shaker [Labworld-online, Inc., Wilmington, NC] for fifteen hours at 250 movements/minute, followed by a stationary phase of approximately one hour.
Following saponification, the supernatant can be diluted with 0.20 ml of methanol. The addition of methanol can be conducted under pressure to ensure sample homogeneity. Using a 0.25 ml syringe, a 0.1 ml aliquot can be removed and transferred to HPLC vials for analysis.
For HPLC analysis, a Hewlett Packard 1100 HPLC, complete with a quaternary pump, vacuum degassing system, six-way injection valve, temperature regulated autosampler, column oven and Photodiode Array detector can be used [Agilent Technologies available through Ultra Scientific Inc., 250 Smith Street, North Kingstown, RI]. The column can be a Waters YMC30, 5-micron, 4.6 x 250 mm with a guard column of the same material [Waters, 34 Maple Street, Milford, MA]. The solvents for the mobile phase can be 81 methanol: 4 water: 15 tetrahydrofuran (THF) stabilized with 0.2% BHT (2,6-di-tert-butyl-4-methylphenol). Injections were 20 µl. Separation can be isocratic at 30°C with a flow rate of 1.7 ml/minute. The peak responses can be measured by absorbance at 447 nm.

**[0459.0.20.20]** If required and desired, further chromatography steps with a suitable resin may follow. Advantageously, the behenic acid, lignoceric acid, cerotic acid or melissic acid can be further purified with a so-called RTHPLC. As eluent acetonitrile/water or chloroform/acetonitrile mixtures can be used. If necessary, these chromatography steps may be repeated, using identical or other chromatography resins. The skilled worker is familiar with the selection of suitable chromatography resin and the most effective use for a particular molecule to be purified.

**[0460.0.0.20] see [0460.0.0.0]**

**[0461.0.20.20]** Example 10: Cloning SEQ ID NO: 25525, 25617, 26065, 26455, 26631, 26659, 26795, 26837, 26983, 27031, 27035 and 97565 for the expression in plants

**[0462.0.0.20] see [0462.0.0.0]**

**[0463.0.20.20]** SEQ ID NO: 25525, 25617, 26065, 26455, 26631, 26659, 26795, 26837, 26983, 27031, 27035 and 97565 is amplified by PCR as described in the protocol of the Pfu Turbo or DNA Herculase polymerase (Stratagene).

**[0464.0.0.0.20] to [0466.0.0.20]: see [0464.0.0.0] to [0466.0.0.0]**

**[0466.1.0.20]** In case the Herculase enzyme can be used for the amplification, the PCR amplification cycles were as follows: 1 cycle of 2-3 minutes at 94°C, followed by 25-30 cycles of in each case 30 seconds at 94°C, 30 seconds at 55-60°C and 5-10 minutes at 72°C, followed by 1 cycle of 10 minutes at 72°C, then 4°C.

**[0467.0.20.20]** The following primer sequences were selected for the gene SEQ ID : 25525
i) forward primer (SEQ ID NO: 25615)
   atgaaacatc tgcatcgatt ctttag
ii) reverse primer (SEQ ID NO: 25616)
   ttaaactgat ggacgcaaac gaacg
The following primer sequences were selected for the gene SEQ ID NO: 25617:
i) forward primer (SEQ ID NO: 26063)
   atggaaaagg gtactgttaa gtg
ii) reverse primer (SEQ ID NO: 26064)
   ttatgcgact gccgcttcta cttc
The following primer sequences were selected for the gene SEQ ID NO: 26065:
i) forward primer (SEQ ID NO: 26453)
   atgattaacc gtatccgcgt agtc
ii) reverse primer (SEQ ID NO: 26454 )
   ttaaaatgtt tcccagtttg gatcttg
The following primer sequences were selected for the gene SEQ ID NO: 26455 :
i) forward primer (SEQ ID NO: 26629)
   atgagtcgtt tagtcgtagt atcta
ii) reverse primer (SEQ ID NO: 26630)
   ttacgcaagc tttggaaagg tagc
The following primer sequences were selected for the gene SEQ ID NO: 26631:
i) forward primer (SEQ ID NO: 26657)
   atggctgaat ggagcggcga
ii) reverse primer (SEQ ID NO: 26658)
   ttagtattcc cacgtctccg gg
The following primer sequences were selected for the gene SEQ ID NO: 26659:
i) forward primer (SEQ ID NO: 26793)
   atggagacca atttttcctt cgact
ii) reverse primer (SEQ ID NO: 26794)
   ctattgaaat accggcttca atattt
The following primer sequences were selected for the gene SEQ ID NO: 26795:
i) forward primer (SEQ ID NO: 26835)
   atgaatagcg taaaaagagt aaagct
ii) reverse primer (SEQ ID NO: 26836)
   ctaggccaaa gacatcttag cca
The following primer sequences were selected for the gene SEQ ID NO: 26837:
i) forward primer (SEQ ID NO: 26981)
   atgacgagac gtactactat taatc
ii) reverse primer (SEQ ID NO: 26982)
   tcacttgttt attttcgata aaatttcc
The following primer sequences were selected for the gene SEQ ID NO: 26983:
i) forward primer (SEQ ID NO: 27029)
   atgtcgcctt tgagaaagac ggtt
ii) reverse primer (SEQ ID NO: 27030)
   tcactcttcc aggtttgagt acg
The following primer sequences were selected for the gene SEQ ID NO: 27031:
i) forward primer (SEQ ID NO: 27033)
   atggcggttg cgatcaaaaa gga
ii) reverse primer (SEQ ID NO: 27034)
   tcaattgata aatgtacttt caatgatg
The following primer sequences were selected for the gene SEQ ID NO: 27035:
i) forward primer (SEQ ID NO: 27295)
   atggctcggg gtgacggaca t
(q) reverse primer (SEQ ID NO: 27296)
   tcatgcttct tttgcgtgat gcaat
The following primer sequences were selected for the gene SEQ ID NO: 97565:
i) forward primer (SEQ ID NO: 97767)
   atgatatgct cacctcagaa caaca
ii) reverse primer (SEQ ID NO: 97768)
   ttaaaaacag aggctttttc ctctgc

**[0468.0.20.20] to [0470.0.20.20]: see [0468.0.0.0] to [0470.0.0.0]**

**[0470.1.20.20]** The PCR-products, produced by *Pfu* Turbo DNA polymerase, were phosphorylated using a T4 DNA polymerase using a standard protocol (e.g. MBI Fermentas) and cloned into the processed binary vector.

**[0471.0.20.20] see [0471.0.0.0]**

**[0471.1.20.20]** The DNA termini of the PCR-products, produced by Herculase DNA polymerase, were blunted in a second synthesis reaction using *Pfu* Turbo DNA polymerase. The composition for the protocol of the blunting the DNA-termini was as follows: 0.2 mM blunting dTTP and 1.25 u *Pfu* Turbo DNA polymerase. The reaction was incubated at 72°C for 30 minutes. Then the PCR-products were phosphorylated using a T4 DNA polymerase using a standard protocol (e.g. MBI Fermentas) and cloned into the processed vector as well.

**[0472.0.20.20] to [0479.0.20.20]: see [0472.0.0.0] to [0479.0.0.0]**

**[0480.0.20.20]** Example 11: Generation of transgenic plants which express SEQ ID NO: 25525, 25617, 26065, 26455, 26631, 26659, 26795, 26837, 26983, 27031, 27035 or 97565

**[0481.0.0.20] to [0513.0.0.20]: see [0481.0.0.0] to [0513.0.0.0]**

**[0514.0.20.20]** As an alternative, lignoceric acid, cerotic acid, melissic acid or behenic acid can be detected as described in Beerthuis, R.K., Keppler, J.G., Nature. 1957 Apr 6;179(4562):731-2.
The results of the different plant analyses can be seen from the table 1 which follows:

**Table 1**

| ORF | Metabolite | Method | Min | Max |
|---|---|---|---|---|
| b0019 | Cerotic Acid | GC | 1,40 | 2,50 |
| | (C26:0) | | | |
| b0019 | Lignoceric acid | GC | 1,46 | 3,24 |
| | (C24:0) | | | |
| b0880 | Cerotic Acid | GC | 2,72 | 3,94 |
| | (C26:0) | | | |
| b1886 | Cerotic Acid | GC | 1,39 | 4,35 |
| | (C26:0) | | | |
| b1896 | Cerotic Acid | GC | 1,39 | 1,75 |
| | (C26:0) | | | |
| b3938 | Lignoceric acid | GC | 1,37 | 1,89 |
| | (C24:0) | | | |
| YDR513W | Melissic Acid | GC | 1,31 | 2,08 |
| | (C30:0) | | | |
| YER156C | Melissic Acid | GC | 1,33 | 1,73 |
| | (C30:0) | | | |
| YGL205W | Behenic acid | GC | 1,74 | 2,32 |
| | (C22:0) | | | |
| YHR201C | Cerotic Acid | GC | 1,46 | 3,27 |
| | (C26:0) | | | |
| YHR201C | Lignoceric acid | GC | 1,29 | 3,71 |
| | (C24:0) | | | |
| YLR255C | Melissic Acid | GC | 1,34 | 1,88 |
| | (C30:0) | | | |
| YPR138C | Lignoceric acid | GC | 1,92 | 2,60 |
| | (C24:0) | | | |
| b0255 | Lignoceric acid GC | 1,35 | 1,35 | 2,15 |
| | (C24:0) | | | |

**[0515.0.20.20]** Column 2 shows the metabolite behenic acid, lignoceric acid, cerotic acid or melissic acid analyzed. Columns 4 and 5 shows the ratio of the analyzed metabolite between the transgenic plants and the wild type; Increase of the metabolite: Max: maximal x-fold (normalised to wild type)-Min: minimal x-fold (normalised to wild type). Decrease of the metabolite: Max: maximal x-fold (normalised to wild type) (minimal decrease), Min: minimal x-fold (normalised to wild type) (maximal decrease). Column 3 indicates the analytical method.

**[0516.0.0.20] to [0552.0.0.20]: see [0516.0.0.0] to [0552.0.0.0]**

**[0552.1.0.20] /**

**[0552.2.0.20]** Example 16: Preparation of homologous sequences from plants
Different plants can be grown under standard or varying conditions in the greenhouse. RNA can be extracted following the protocol of Jones, Dunsmuir and Bedbrook (1985) EMBO J. 4: 2411-2418. Approx. 1 gram of tissue material from various organs is ground in liquid nitrogen. The powder is transferred to a 13 ml Falcon tube containing 4.5 ml NTES buffer (100 mM NaCl, 10 mM Tris/HCl pH 7.5, 1 mM EDTA, 1 % SDS; in RNase-free water) and 3 ml phenol/chloroform/isoamylalcohol (25/24/1), immediately mixed and stored on ice. The mixture is spun for 10 minutes at 7000 rpm using a centrifuge (Sorval; SM24 or SS34 rotor). The supernatant is transferred to a new tube, 1/10th volume of 3 M NaAcetate (pH 5.2; in RNase-free water) and 1 volume of isopropanol is added, mixed at stored for 1 hour or overnight at -20 °C. The mixture is spun for 10 minutes at 7000 rpm. The supernatant is discarded and the pellet washed with 70 % ethanol (v/v). The mixture is spun for 5 minutes at 7000 rpm, the supernatant is discarded and the pellet is air-dried. 1 ml RNase-free water is added and allow the DNA/RNA pellet to dissolve on ice at 4 C. The nucleic acid solution is transferred to a 2 ml Eppendorf tube and 1 ml of 4 M LiAcetate is added. After mixing the solution is kept for at least 3 hours, or overnight, at 4 C. The mixture is spun for 10 minutes at 14000 rpm, the supernatant discarded, the pellet washed with 70 % Ethanol, air-dried and dissolved in 200 µl of RNase-free water.
Total RNA can be used to construct a cDNA-library according to the manufacturer's protocol (for example using the ZAP-cDNA synthesis and cloning kit of Stratagene, La Jolla, USA). Basically, messenger RNA (mRNA) is primed in the first strand synthesis with a oligo(dT) linker-primer and is reverse-transcribed using reverse transcriptase.
After second strand cDNA synthesis, the double-stranded cDNA is ligated into the Uni-ZAP XR vector. The Uni-ZAP XR vector allows in vivo excision of the pBluescript phagemid. The polylinker of the pBluescript phagemid has 21 unique cloning sites flanked by T3 and T7 promoters and a choice of 6 different primer sites for DNA sequencing. Systematic single run sequencing of the expected 5 prime end of the clones can allow preliminary annotation of the sequences for example with the help of the pedant pro Software package (Biomax, München). Clones for the nucleic acids of the invention or used in the process according to the invention can be identified based on homology search with standard algorithms like blastp or gap. Identified putative full length clones with identity or high homology can be subjected to further sequencing in order to obtain the complete sequence.
Additional new homologous sequences can be identified in a similar manner by preparing respective cDNA libraries from various plant sources as described above. Libraries can then be screened with available sequences of the invention under low stringency conditions for example as described in Sambrook et al., Molecular Cloning: A laboratory manual, Cold Spring Harbor 1989, Cold Spring Harbor Laboratory Press. Purified positive clones can be subjected to the in vivo excision and complete sequencing. A pairwise sequence alignment of the original and the new sequence using the blastp or gap program allows the identification of orthologs, meaning homologous sequences from different organisms, which should have a sequence identity of at least 30%. Furthermore the conservation of functionally important amino acid residues or domains, which can be identified by the alignment of several already available paralogs, can identify a new sequence as a new orthologs.
Alternatively libraries can be subjected to mass sequencing and obtained sequences can be stored in a sequence database, which then can be screened for putative orthologs by different search algorithms, for example the tbastn algorithm to search the obtained nucleic acid sequences with a amino acid sequence of the invention. Clones with the highest sequence identity are used for a complete sequence determination and orthologs can be identified as described above.

**[0553.0.20.20]**
1. A process for the production of behenic acid, lignoceric acid, cerotic acid or melissic acid, which comprises
   (a) increasing or generating the activity of a protein as indicated in Table IIA or IIB, columns 5 or 7, lines 251 to 261, 627, 627 or a functional equivalent thereof in a non-human organism or in one or more parts thereof; and
   (b) growing the organism under conditions which permit the production of behenic acid, lignoceric acid, cerotic acid or melissic acid in said organism.
2. A process for the production of behenic acid, lignoceric acid, cerotic acid or melissic acid, comprising the increasing or generating in an organism or a part thereof the expression of at least one nucleic acid molecule comprising a nucleic acid molecule selected from the group consisting of:
   a) nucleic acid molecule encoding a polypeptide as indicated in Table IIA or IIB, columns 5 or 7, lines 251 to 261, 627, 627 or a fragment thereof, which confers an increase in the amount of behenic acid, lignoceric acid, cerotic acid or melissic acid in an organism or a part thereof;
   b) nucleic acid molecule comprising of a nucleic acid molecule as indicated in Table IA or IB, columns 5 or 7, lines 251 to 261, 627, 627;
   c) nucleic acid molecule whose sequence can be deduced from a polypeptide sequence encoded by a nucleic acid molecule of (a) or (b) as a result of the degeneracy of the genetic code and conferring an increase in the amount of behenic acid, lignoceric acid, cerotic acid or melissic acid in an organism or a part thereof;
   d) nucleic acid molecule which encodes a polypeptide which has at least 50% identity with the amino acid sequence of the polypeptide encoded by the nucleic acid molecule of (a) to (c) and conferring an increase in the amount of behenic acid, lignoceric acid, cerotic acid or melissic acid in an organism or a part thereof;
   e) nucleic acid molecule which hybidizes with a nucleic acid molecule of (a) to (c) under stringent hybridisation conditions and conferring an increase in the amount of behenic acid, lignoceric acid, cerotic acid or melissic acid in an organism or a part thereof;
   f) nucleic acid molecule which encompasses a nucleic acid molecule which is obtained by amplifying nucleic acid molecules from a cDNA library or a genomic library using the primers or primer pairs as indicated in Table III, column 7, lines 251 to 261, 627, 627 and conferring an increase in the amount of behenic acid, lignoceric acid, cerotic acid or melissic acid in an organism or a part thereof;
   g) nucleic acid molecule encoding a polypeptide which is isolated with the aid of monoclonal antibodies against a polypeptide encoded by one of the nucleic acid molecules of (a) to (f) and conferring an increase in the amount of behenic acid, lignoceric acid, cerotic acid or melissic acid in an organism or a part thereof;
   h) nucleic acid molecule encoding a polypeptide comprising a consensus as indicated in Table IV, column 7, lines 251 to 259 and 261, 627 and conferring an increase in the amount of behenic acid, lignoceric acid, cerotic acid or melissic acid in an organism or a part thereof; and
   i) nucleic acid molecule which is obtainable by screening a suitable nucleic acid library under stringent hybridization conditions with a probe comprising one of the sequences of the nucleic acid molecule of (a) to (k) or with a fragment thereof having at least 15 nt, preferably 20 nt, 30 nt, 50 nt, 100 nt, 200 nt or 500 nt of the nucleic acid molecule characterized in (a) to (k) and conferring an increase in the amount of behenic acid, lignoceric acid, cerotic acid or melissic acid in an organism or a part thereof.
   or comprising a sequence which is complementary thereto.
3. The process of claim 1 or 2, comprising recovering of the free or bound behenic acid, lignoceric acid, cerotic acid or melissic acid.
4. The process of any one of claim 1 to 3, comprising the following steps:
   a) selecting an organism or a part thereof expressing a polypeptide encoded by the nucleic acid molecule characterized in claim 2;
   b) mutagenizing the selected organism or the part thereof;
   c) comparing the activity or the expression level of said polypeptide in the mutagenized organism or the part thereof with the activity or the expression of said polypeptide of the selected organisms or the part thereof;
   d) selecting the mutated organisms or parts thereof, which comprise an increased activity or expression level of said polypeptide compared to the selected organism or the part thereof;
   e) optionally, growing and cultivating the organisms or the parts thereof; and
   f) recovering, and optionally isolating, the free or bound behenic acid, lignoceric acid, cerotic acid or melissic acid produced by the selected mutated organisms or parts thereof.
5. The process of any one of claims 1 to 4, wherein the activity of said protein or the expression of said nucleic acid molecule is increased or generated transiently or stably.
6. An isolated nucleic acid molecule comprising a nucleic acid molecule selected from the group consisting of:
   a) nucleic acid molecule encoding a polypeptide as indicated in Table IIA or IIB, columns 5 or 7, lines 251 to 261, 627, 627 or a fragment thereof, which confers an increase in the amount of behenic acid, lignoceric acid, cerotic acid or melissic acid in an organism or a part thereof;
   b) nucleic acid molecule comprising of a nucleic acid molecule as indicated in Table IA or IB, columns 5 or 7, lines 251 to 261, 627, 627 ;
   c) nucleic acid molecule whose sequence can be deduced from a polypeptide sequence encoded by a nucleic acid molecule of (a) or (b) as a result of the degeneracy of the genetic code and conferring an increase in the amount of behenic acid, lignoceric acid, cerotic acid or melissic acid in an organism or a part thereof;
   d) nucleic acid molecule which encodes a polypeptide which has at least 50% identity with the amino acid sequence of the polypeptide encoded by the nucleic acid molecule of (a) to (c) and conferring an increase in the amount of behenic acid, lignoceric acid, cerotic acid or melissic acid in an organism or a part thereof;
   e) nucleic acid molecule which hybidizes with a nucleic acid molecule of (a) to (c) under stringent hybridisation conditions and conferring an increase in the amount of behenic acid, lignoceric acid, cerotic acid or melissic acid in an organism or a part thereof;
   f) nucleic acid molecule which encompasses a nucleic acid molecule which is obtained by amplifying nucleic acid molecules from a cDNA library or a genomic library using the primers or primer pairs as indicated in Table III, column 7, lines 251 to 261, 627, 627 and conferring an increase in the amount of behenic acid, lignoceric acid, cerotic acid or melissic acid in an organism or a part thereof;
   g) nucleic acid molecule encoding a polypeptide which is isolated with the aid of monoclonal antibodies against a polypeptide encoded by one of the nucleic acid molecules of (a) to (f) and conferring an increase in the amount of behenic acid, lignoceric acid, cerotic acid or melissic acid in an organism or a part thereof;
   h) nucleic acid molecule encoding a polypeptide comprising a consensus as indicated in Table IV, column 7, lines 251 to 259 and 261, 627 and conferring an increase in the amount of behenic acid, lignoceric acid, cerotic acid or melissic acid in an organism or a part thereof; and
   i) nucleic acid molecule which is obtainable by screening a suitable nucleic acid library under stringent hybridization conditions with a probe comprising one of the sequences of the nucleic acid molecule of (a) to (k) or with a fragment thereof having at least 15 nt, preferably 20 nt, 30 nt, 50 nt, 100 nt, 200 nt or 500 nt of the nucleic acid molecule characterized in (a) to (k) and conferring an increase in the amount of behenic acid, lignoceric acid, cerotic acid or melissic acid in an organism or a part thereof.
   whereby the nucleic acid molecule distinguishes over the sequence as indicated in Table IA or IB, columns 5 or 7, lines 251 to 261, 627, 627 by one or more nucleotides.
7. A nucleic acid construct which confers the expression of the nucleic acid molecule of claim 6, comprising one or more regulatory elements.
8. A vector comprising the nucleic acid molecule as claimed in claim 6 or the nucleic acid construct of claim 7.
9. The vector as claimed in claim 8, wherein the nucleic acid molecule is in operable linkage with regulatory sequences for the expression in a prokaryotic or eukaryotic, or in a prokaryotic and eukaryotic, host.
10. A host cell, which has been transformed stably or transiently with the vector as claimed in claim 8 or 9 or the nucleic acid molecule as claimed in claim 6 or the nucleic acid construct of claim 7 or produced as described in any one of claims 2 to 5.
11. The host cell of claim 10, which is a transgenic host cell.
12. The host cell of claim 10 or 11, which is a plant cell, an animal cell, a microorganism, or a yeast cell, a fungus cell, a prokaryotic cell, an eukaryotic cell or an archaebacterium.
13. A process for producing a polypeptide, wherein the polypeptide is expressed in a host cell as claimed in any one of claims 10 to 12.
14. A polypeptide produced by the process as claimed in claim 13 or encoded by the nucleic acid molecule as claimed in claim 6 whereby the polypeptide distinguishes over a sequence as indicated in Table IIA or IIB, columns 5 or 7, lines 251 to 261, 627, 627 by one or more amino acids.
15. An antibody, which binds specifically to the polypeptide as claimed in claim 14.
16. A plant tissue, propagation material, harvested material or a plant comprising the host cell as claimed in claim 12 which is plant cell or an Agrobacterium.
17. A method for screening for agonists and antagonists of the activity of a polypeptide encoded by the nucleic acid molecule of claim 6 conferring an increase in the amount of behenic acid, lignoceric acid, cerotic acid or melissic acid in an organism or a part thereof comprising:
   (a) contacting cells, tissues, plants or microorganisms which express the a polypeptide encoded by the nucleic acid molecule of claim 6 conferring an increase in the amount of behenic acid, lignoceric acid, cerotic acid or melissic acid in an organism or a part thereof with a candidate compound or a sample comprising a plurality of compounds under conditions which permit the expression the polypeptide;
   (b) assaying the behenic acid, lignoceric acid, cerotic acid or melissic acid level or the polypeptide expression level in the cell, tissue, plant or microorganism or the media the cell, tissue, plant or microorganisms is cultured or maintained in; and
   (c) identifying a agonist or antagonist by comparing the measured behenic acid, lignoceric acid, cerotic acid or melissic acid level or polypeptide expression level with a standard behenic acid, lignoceric acid, cerotic acid or melissic acid or polypeptide expression level measured in the absence of said candidate compound or a sample comprising said plurality of compounds, whereby an increased level over the standard indicates that the compound or the sample comprising said plurality of compounds is an agonist and a decreased level over the standard indicates that the compound or the sample comprising said plurality of compounds is an antagonist.
18. A process for the identification of a compound conferring increased behenic acid, lignoceric acid, cerotic acid or melissic acid production in a plant or microorganism, comprising the steps:
   (a) culturing a plant cell or tissue or microorganism or maintaining a plant expressing the polypeptide encoded by the nucleic acid molecule of claim 6 conferring an increase in the amount of behenic acid, lignoceric acid, cerotic acid or melissic acid in an organism or a part thereof and a readout system capable of interacting with the polypeptide under suitable conditions which permit the interaction of the polypeptide with said readout system in the presence of a compound or a sample comprising a plurality of compounds and capable of providing a detectable signal in response to the binding of a compound to said polypeptide under conditions which permit the expression of said readout system and of the polypeptide encoded by the nucleic acid molecule of claim 6 conferring an increase in the amount of behenic acid, lignoceric acid, cerotic acid or melissic acid in an organism or a part thereof;
   (b) identifying if the compound is an effective agonist by detecting the presence or absence or increase of a signal produced by said readout system.
19. A method for the identification of a gene product conferring an increase in behenic acid, lignoceric acid, cerotic acid or melissic acid production in a cell, comprising the following steps:
   (a) contacting the nucleic acid molecules of a sample, which can contain a candidate gene encoding a gene product conferring an increase in behenic acid, lignoceric acid, cerotic acid or melissic acid after expression with the nucleic acid molecule of claim 6;
   (b) identifying the nucleic acid molecules, which hybridise under relaxed stringent conditions with the nucleic acid molecule of claim 6;
   (c) introducing the candidate nucleic acid molecules in host cells appropriate for producing behenic acid, lignoceric acid, cerotic acid or melissic acid;
   (d) expressing the identified nucleic acid molecules in the host cells;
   (e) assaying the behenic acid, lignoceric acid, cerotic acid or melissic acid level in the host cells; and
   (f) identifying nucleic acid molecule and its gene product which expression confers an increase in the behenic acid, lignoceric acid, cerotic acid or melissic acid level in the host cell in the host cell after expression compared to the wild type.
20. A method for the identification of a gene product conferring an increase in behenic acid, lignoceric acid, cerotic acid or melissic acid production in a cell, comprising the following steps:
   a) identifiying in a data bank nucleic acid molecules of an organism; which can contain a candidate gene encoding a gene product conferring an increase in the behenic acid, lignoceric acid, cerotic acid or melissic acid amount or level in an organism or a part thereof after expression, and which are at least 20% homolog to the nucleic acid molecule of claim 6;
   b) introducing the candidate nucleic acid molecules in host cells appropriate for producing behenic acid, lignoceric acid, cerotic acid or melissic acid;
   c) expressing the identified nucleic acid molecules in the host cells;
   d) assaying the behenic acid, lignoceric acid, cerotic acid or melissic acid level in the host cells; and
   e) identifying nucleic acid molecule and its gene product which expression confers an increase in the behenic acid, lignoceric acid, cerotic acid or melissic acid level in the host cell after expression compared to the wild type.
21. A method for the production of an agricultural composition comprising the steps of the method of any one of claims 17 to 20 and formulating the compound identified in any one of claims 17 to 20 in a form acceptable for an application in agriculture.
22. A composition comprising the nucleic acid molecule of claim 6, the polypeptide of claim 14, the nucleic acid construct of claim 7, the vector of any one of claims 8 or 9, an antagonist or agonist identified according to claim 17, the compound of claim 18, the gene product of claim 19 or 20, the antibody of claim 15, and optionally an agricultural acceptable carrier.
23. Use of the nucleic acid molecule as claimed in claim 6 for the identification of a nucleic acid molecule conferring an increase of behenic acid, lignoceric acid, cerotic acid or melissic acid after expression.
24. Use of the polypeptide of claim 14 or the nucleic acid construct claim 7 or the gene product identified according to the method of claim 19 or 20 for identifying compounds capable of conferring a modulation of behenic acid, lignoceric acid, cerotic acid or melissic acid levels in an organism.
25. Agrochemical, pharmaceutical, food or feed composition comprising the nucleic acid molecule of claim 6, the polypeptide of claim 14, the nucleic acid construct of claim 7, the vector of claim 8 or 9, the antagonist or agonist identified according to claim 17, the antibody of claim 15, the plant or plant tissue of claim 16, the harvested material of claim 16, the host cell of claim 10 to 12 or the gene product identified according to the method of claim 19 or 20.
26. The method of any one of claims 1 to 5, the nucleic acid molecule of claim 6, the polypeptide of claim 14, the nucleic acid construct of claim 7, the vector of claim 8 or 9, the antagonist or agonist identified according to claim 17, the antibody of claim 15, the plant or plant tissue of claim 16, the harvested material of claim 16, the host cell of claim 10 to 12 or the gene product identified according to the method of claim 19 or 20, wherein the fine chemical is behenic acid, lignoceric acid, cerotic acid or melissic acid.
27. A host cell or plant according to any of the claims 10 to 12 which is resistant to a herbicide inhibiting the biosynthesis of behenic acid, lignoceric acid, cerotic acid or melissic acid.

**[0554.0.0.20] Abstract: see [0554.0.0.0]**

### Process for the production of fine chemicals

### Description

**[0000.0.0.21]** In a further embodiment, the present invention relates to a further process for the production of fine chemicals as defined below and the corresponding embodiments as described herein as follows.

**[0001.0.0.21]** for the disclosure of this paragraph see [0001.0.0.0].

**[0002.0.21.21]** Plants produce glycerol and glycerol-3-phosphate. Importantly lipids derived from glycerol are the major components of eukaryotic cells. In terms of dry weight they account for anywhere between 10% and 90% of the total mass of the cell. Triglycerol are the major source of store energy in eucaryotic organisms.
Glycerol-3-phosphate can be synthesized via two different routes in plants. In one route, it is formed from dihydroxyacetone phosphate (DHAP), an intermediate of glycolysis, by the sequential action of triosephosphate isomerase, glyceraldehyd-phosphate phosphatase, glyeraldehyde reductase and glycerol kinase. The last enzyme of this pathway has been suggested as the rate-limiting step of this route for glycerol-3-phosphate synthesis. In an second pathway glycerol-3-phosphate dehydrogenase (NAD(+)-G-3 -P oxidoreductase, EC 1.1.1.8) (GPDH) catalyses the reduction of dihydroxyacetone phosphate (DHAP) to form glycerol-3-phosphate (G-3-P). Based on enzymatic studies is has been suggested that this enzyme activity is probably the primary source of glycerol-3-phosphate at least in Brassica campestris seeds (Sharma et al., 2001, Plant Sci 160, 603-610).

**[0003.0.0.21]** In plants, at least two types of GPDH, a cytoplasmatic and a plastidial exist, which also differ in their reducing cosubstrate. The cytsolic GPDH uses NADH as the cosubstrate. The mitochondrial FAD-dependent glycerol-3-phosphate dehydrogenase (FAD-GPDH) of Arabidopsis forms a G-3-P shuttle, as previously established in other eukaryotic organisms, and links cytosolic G-3-P metabolism to carbon source utilization and energy metabolism in plants - also see Shen, W. et al., FEBS Lett. 2003 Feb 11; 536(1-3): 92-6.
Glycerol-insensitive *Arabidopsis* mutants: *gli1* seedlings lack glycerol kinase, accumulate glycerol and are more resistant to abiotic stress, see Eastmond P.J. , HYPERLINK "http://www.ingentaconnect.com/content/bsc/tpj" \o "The Plant Journal" The Plant Journal , 2004, 37(4), 617-625. These data show that glycerol kinase is required for glycerol catabolism in *Arabidopsis* and that the accumulation of glycerol can enhance resistance to a variety of abiotic stresses associated with dehydration.

**[0004.0.0.21]** The major storage lipids (or oils) of seeds occur in the form of triacylglycerols (TAG), or three fatty acids linked to glycerol by ester bonds. Triacylglycerol synthesis involves diverse cellular compartments, including the cytoplasm, the mitochondria, the plastids, and the endoplasmic reticulum (ER). Glycerol-3-phosphate enters the ER for the final step in triacylglycerol synthesis. The newly formed triacylglycerols accumulate between the two layers of the double membrane of the ER, forming an oil body surrounded by a single (or half) unit membrane.

**[0005.0.0.21]** Glycerol-3-phosphate acyltransferase (GPAT) is one of the most important enzymes in TAG biosynthesis, since it initiates TAG synthesis by catalyzing the acylation of the Sn-1 position of Sn-glycerol-3-phosphate, producing Sn-1-acylglycerol-3-phosphate. Lyso-phosphatidic acid (LPA) is then acetylated by LPA acyltransferases to produce phosphatidic acid (PA). Then diacylglycerol (DAG) is released through the dephosphorylation of PA by PA phosphohydrolase. Finally DAG becomes acylated by the activity of the DAG acyltransferase. In a second pathway phosphatidylcholine (PC) is formed and its acyl residues are desaturated further. The choline phosphate residue is then liberated by hydrolysis and the correspondinbg DAG acylated. This second pathway operates frequently in the synthesis of highly unsaturated TAG (Heldt 1997, Plant biochemistry and molecular biology. Oxford University Press, New York).
Additionally at present, many researches have proved that the GPAT is related to plant chilling-resistance, see Liu, Ji-Mei et al., Plant Physiol. 120(1999): 934.
Glycerol-3-phosphate is a primary substrate for triacylglycerol synthesis. Vigeolas and Geigenberger (Planta 219(2004): 827-835) have shown that injection of developing seeds with glycerol leads to increased glycerol-3-phosphate levels. These increased levels of glycerol-3-phosphate were accompanied by an increase in the flux of sucrose into total lipids and triacylglycerol providing evidence that the prevailing levels of glycerol-3-phosphate co-limit triacylglycerol production in developing seeds.
The direct acylation of glycerol by a glycerol: acyl-CoA acyltransferase to form monoacyl-glycerol and, subsequently, diacylglycerol and triacylglycerol has been shown in myoblast and hepatocytes (Lee, D.P. et al. J. Lipid res. 42 (2001): 1979-1986). This direct acylation became more prominent when the glycerol-3-phosphate pathway was attenuated or when glycerol levels become elevated.

**[0006.0.0.21]** Glycerol is used together with water and alcohol (ethyl alcohol) in glycerinated water/alcohol plant extracts and phytoaromatic compounds. These products are used as food supplements, providing concentrates of the minerals, trace elements, active ingredients (alkaloids, polyphenols, pigments, etc.) and aromatic substances to be found in plants. Glycerin acts as a carrier for plant extracts. It is found in the end product (the fresh plant extract) in concentrations of up to 24% or 25%.
Raw glycerol is a by-product of the transesterification process of rape oil to rape methyl ester (RME) and used edible oil to used edible methyl ester (AME), both better known as Biodiesel.
Glycerol world production is estimated to be around 750.000 t/year. Around 90% is manufactured on the basis of natural oils and fats.

**[0007.0.0.21]** The green alga *Dunaliella*, for example, recently has been established in mass culture as a commercial source for glycerol. *Dunaliella* withstands extreme salinities while maintaing a low intracellular salt concentration. Osmotic adjustment is achieved by intracellular accumulation of glycerol to a level counterbalancing the external osmoticum.
The osmoregulatory isoform of dihydroxyacetone phosphate (DHAP) reductase (Osm-DHAPR) is an enzyme unique to Dunaliella tertiolecta and is the osmoregulatory isoform involved in the synthesis of free glycerol for osmoregulation in extreme environments, such as high salinity, see Ghoshal, D., et al., HYPERLINK "http://www.ingentaconnect.com/content/ap/pt;jsessionid=708pg3rt74t81.victoria" \o "Protein Expression and Purification" Protein Expression and Purification , 2002, 24, (3), 404-411.
A unsolved problem in plant biochemistry is the understanding of metabolic regulation of glycerol-3-phosphate synthesis and its use in modifying glyceride metabolism or glycerol production. Practically it will have significance for rationally genetically engineering of plants for increased synthesis of triacylglycerols or for other value added products, and for introducing the glycerol synthesis capability into plants of economic importance for an elevated environmental stress tolerance - see: Durba, G. et al., J. Plant Biochemistry & Biotechnology 10(2001),113-120.

**[0008.0.21.21]** One way to increase the productive capacity of biosynthesis is to apply recombinant DNA technology. Thus, it would be desirable to produce glycerol and/or glycerol-3-phosphate in plants. That type of production permits control over quality, quantity and selection of the most suitable and efficient producer organisms. The latter is especially important for commercial production economics and therefore availability to consumers. In addition it is desirable to produce glycerol and/or glycerol-3-phosphate in plants in order to increase plant productivity and resistance against biotic and abiotic stress as discussed before.
Methods of recombinant DNA technology have been used for some years to improve the production of fine chemicals in microorganisms and plants by amplifying individual biosynthesis genes and investigating the effect on production of fine chemicals. It is for example reported, that the xanthophyll astaxanthin could be produced in the nectaries of transgenic tobacco plants. Those transgenic plants were prepared by *Argobacterium tumifaciens*-mediated transformation of tobacco plants using a vector that contained a ketolase-encoding gene from *H. pluvialis* denominated c*rtO* along with the *Pds* gene from tomato as the promoter and to encode a leader sequence. Those results indicated that about 75 percent of the carotenoids found in the flower of the transformed plant contained a keto group.

**[0009.0.21.21]** Thus, it would be advantageous if an algae, plant or other microorganism were available which produce large amounts of glycerol and/or glycerol-3-phosphate. The invention discussed hereinafter relates in some embodiments to such transformed prokaryotic or eukaryotic microorganisms.
It would also be advantageous if plants were available whose roots, leaves, stem, fruits or flowers produced large amounts of glycerol and/or glycerol-3-phosphate. The invention discussed hereinafter relates in some embodiments to such transformed plants.
Furthermore it would be advantageous if plants were available whose seed produced larger amounts of total lipids. The invention discussed hereinafter relates in some embodiments to such transformed plants.

**[0010.0.21.21]** Therefore improving the quality of foodstuffs and animal feeds is an important task of the food-and-feed industry. This is necessary since, for example glycerol and/or glycerol-3-phosphate, as mentioned above, which occur in plants and some microorganisms are limited with regard to the supply of mammals. Especially advantageous for the quality of foodstuffs and animal feeds is as balanced as possible a specific glycerol and/or glycerol-3-phosphate profile in the diet since an excess of glycerol and/or glycerol-3-phosphate above a specific concentration in the food has a positive effect. A further increase in quality is only possible via addition of further glycerol and/or glycerol-3-phosphate, which are limiting.

**[0011.0.21.21]** To ensure a high quality of foods and animal feeds, it is therefore necessary to add glycerol and/or glycerol-3-phosphate in a balanced manner to suit the organism.

**[0012.0.21.21]** Accordingly, there is still a great demand for new and more suitable genes which encode enzymes or other proteins which participate in the biosynthesis of glycerol and/or glycerol-3-phosphate and make it possible to produce them specifically on an industrial scale without unwanted byproducts forming. In the selection of genes for biosynthesis two characteristics above all are particularly important. On the one hand, there is as ever a need for improved processes for obtaining the highest possible contents of glycerol and/or glycerol-3-phosphate; on the other hand as less as possible byproducts should be produced in the production process.
Furthermore there is still a great demand for new and more suitable genes, which encode enzymes or other proteins, which participate in the biosynthesis of total lipids and make it possible to produce them specifically on an industrial scale without unwanted byproducts forming. In the selection of genes for biosynthesis two characteristics above all are particularly important. On the one hand, there is as ever a need for improved processes for obtaining the highest possible contents of total lipids; on the other hand as less as possible byproducts should be produced in the production process.
Glycerol or glycerol-3-phosphate is biosynthetic precusor for the biosynthesis of monoacylglycerols, diacylglycerols, triacylglycerols, phosphatidylglycerols and other glycerolipids (e.g. glycosylglycerides, diphosphatidylglycerols, phosphonolipids, phosphatidylcholines, phosphatidylethanolamines, phosphatidylinositols, phytoglycolipids). Therefore the analysis of the glycerol content in cells, tissues or plant parts like seeds and leaves after total lipid extraction and lipid hydrolysis directly correlates with the analysis of the total lipid content. For example if the overexpression of a gene participating in the biosynthesis of triacylglycerols in the seed results in an increase in total lipid content in the seed or leaf this seed will also show an increased glycerol content after total lipid extraction and hydrolysis of the lipids.
Therefore the method as described below which leads to an increase in glycerol in the lipid fraction after cleavage of the ester functions for example with a mixture of methanol and hydrochloric acid clearly represents a method for an increased production of triacylglycerol or total lipids.

**[0013.0.0.21]** for the disclosure of this paragraph see [0013.0.0.0].

**[0014.0.21.21]** Accordingly, in a first embodiment, the invention relates to a process for the production of a fine chemical, whereby the fine chemical is glycerol and/or glycerol-3-phosphate. Accordingly, in the present invention, the term "the fine chemical" as used herein relates to glycerol and/or glycerol-3-phosphate. Further, the term "the fine chemicals" as used herein also relates to fine chemicals comprising glycerol and/or glycerol-3-phosphate.

**[0015.0.21.21]** In one embodiment, the term "the fine chemical" means glycerol. In one embodiment, the term "the fine chemical" means glycerol-3-phosphate depending on the context in which the term is used. Throughout the specification the term "the fine chemical" means glycerol and/or glycerol-3-phosphate, its salts, ester, thioester or in free form or bound to other compounds such as sugars or sugarpolymers, like glucoside or polyols like myo-inositol.
In one embodiment, the term "the fine chemical" means monoacylglycerols, diacylglycerols, triacylglycerols, phosphatidylglycerols and/or other glycerolipids (e.g. but not limited to glycosylglycerides, diphosphatidylglycerols, phosphonolipids, phosphatidylcholines, phosphatidylethanolamines, phosphatidylinositols or phytoglycolipids) and is hereinafter referred to as "total lipids".

**[0016.0.21.21]** Accordingly, the present invention relates to a process comprising
(a) increasing or generating the activity of one or more YDR065W, b2441, b3457, YBR084W, YDR513W, YGL237C, YIL150C, YLR082C, YLR224W, YLR255C, YMR015C, YOR344C, YPL099C, YPL268W, b3644, YHR072W, b2710, b3498 or b4073 protein(s) in a non-human organism in one or more parts thereof; and
(b) growing the organism under conditions which permit the production of the fine chemical, thus glycerol and/or glycerol-3-phosphate in said organism.
Accordingly, the present invention relates to a process comprising
(a) increasing or generating the activity of one or more proteins having the activity of a protein indicated in Table II, column 3, lines 173, 262 to 274 and 628 to 632, resp. or having the sequence of a polypeptide encoded by a nucleic acid molecule indicated in Table I, column 5 or 7, lines 173, 262 to 274 and 628 to 632, resp. in a non-human organism in one or more parts thereof; and
growing the organism under conditions which permit the production of the fine chemical, thus, glycerol and/or glycerol-3-phosphate, in said organism.

**[0016.1.21.21]** Accordingly, the term "the fine chemical" means" "glycerol" in relation to all sequences listed in Table I, lines 262 to 274 and lines 628 and 629 or homologs thereof, means "glycerol-3-phosphate" in relation to the sequence listed in Table I, line 173 and lines 630 to 632 or homologs thereof. The term "the fine chemical" could in addition mean "total lipids" comprising but not limited to "monoacylglycerols, diacylglycerols, triacylglycerols, phosphatidylglycerols and/or other glycerolipids (e.g. glycosylglycerides, diphosphatidylglycerols, phosphonolipids, phosphatidylcholines, phosphatidylethanolamines, phosphatidylinositols or phytoglycolipids)" in relation to all sequences listed in Table I, lines 265, 266, 267, 269, 271, 272, 263 or 628 to 632 or homologs thereof. Accordingly, the term "the fine chemical" can mean "glycerol","glycerol-3-phosphate"or "total lipids", owing to circumstances and the context.

**[0017.0.0.21] and [0018.0.0.21]** for the disclosure of the paragraphs [0017.0.0.21] and [0018.0.0.21] see paragraphs [0017.0.0.0] and [0018.0.0.0] above.

**[0019.0.21.21]** Advantageously the process for the production of the respective fine chemical leads to an enhanced production of the respective fine chemical. The terms "enhanced" or "increase" mean at least a 10%, 20%, 30%, 40% or 50%, preferably at least 60%, 70%, 80%, 90% or 100%, more preferably 150%, 200%, 300%, 400% or 500% higher production of the respective fine chemical in comparison to the reference as defined below, e.g. that means in comparison to an organism without the aforementioned modification of the activity of a protein having the activity of a protein indicated in Table II, column 3, lines 173, 262 to 274 and 628 to 632 or encoded by nucleic acid molecule indicated in Table I, columns 5 or 7, lines 173, 262 to 274 and 628 to 632.

**[0020.0.21.21]** Surprisingly it was found, that the transgenic expression of the Escherichia coli K12 protein b2441, b3457, b3644, b2710, b3498 or b4073 or Saccharomyces cerevisiae protein YDR065W, YBR084W, YDR513W, YGL237C, YIL150C, YLR082C, YLR224W, YLR255C, YMR015C, YOR344C, YPL099C, YPL268W or YHR072W in Arabidopsis thaliana conferred an increase in glycerol and/or glycerol-3-phosphate and/or total lipid ("the fine chemical" or "the fine respective chemical") in respect to said proteins and their homologs as wells as the encoding nucleic acid molecules, in particular as indicated in Table II, column 3, lines 173, 262 to 274 and 628 to 632 content of the transformed plants.

**[0021.0.0.21]** for the disclosure of this paragraph see [0021.0.0.0] above.

**[0022.0.21.21]** The sequence of YDR065W from Saccharomyces cerevisiae has been published in Jacq, C. et al., Nature 387 (6632 Suppl), 75-78 (1997) and its activity has not been characterized yet. Accordingly, in one embodiment, the process of the present invention comprises the use of a protein YDR065W from Saccharomyces cerevisiae or its homolog, e.g. as shown herein, for the production of the respective fine chemical, in particular for increasing the amount of glycerol-3-phosphate, preferably in free or bound form in an organism or a part thereof, as mentioned. In one embodiment, in the process of the present invention the activity of the protein YDR065W is increased.
The sequence of b2441 from Escherichia coli K12 has been published in Blattner, F.R. et al., Science 277 (5331), 1453-1474 (1997) and its activity is being defined as a protein having ethanolamine ammonia-lyase activity. Accordingly, in one embodiment, the process of the present invention comprises the use of a protein b2441from Escherichia coli K12 or its homolog, e.g. as shown herein, for the production of the respective fine chemical, in particular for increasing the amount of glycerol, preferably in free or bound form in an organism or a part thereof, as mentioned. In one embodiment, in the process of the present invention the activity of the protein b2441 is increased.
The sequence of b3457 from Escherichia coli K12 has been published in Blattner, F.R. et al, Science 277 (5331), 1453-1474 (1997) and its activity is being defined as a protein having branched -chain amino acid transport activity. Accordingly, in one embodiment, the process of the present invention comprises the use of a protein b3457from Escherichia coli K12 or its homolog, e.g. as shown herein, for the production of the respective fine chemical, in particular for increasing the amount of glycerol and/or total lipids, preferably in free or bound form in an organism or a part thereof, as mentioned. In one embodiment, in the process of the present invention the activity of the protein b3457is increased.
The sequence of YBR084W from Saccharomyces cerevisiae has been published in Goffeau, A. et al., Science 274 (5287), 546-547 (1996) and its activity is being defined as a protein having C1-tetrahydrofolate synthase activity. Accordingly, in one embodiment, the process of the present invention comprises the use of a protein YBR084Wfrom Saccharomyces cerevisiae or its homolog, e.g. as shown herein, for the production of the respective fine chemical, in particular for increasing the amount of glycerol, preferably in free or bound form in an organism or a part thereof, as mentioned. In one embodiment, in the process of the present invention the activity of the protein YBR084W is increased.
The sequence of YDR513W from Saccharomyces cerevisiae has been published in Jacq, C. et al., Nature 387 (6632 Suppl), 75-78 (1997) and its activity is being defined as a protein having gluthatione reductase activity. Accordingly, in one embodiment, the process of the present invention comprises the use of a protein YDR513W from Saccharomyces cerevisiae or its homolog, e.g. as shown herein, for the production of the respective fine chemical, in particular for increasing the amount of glycerol and/or total lipid, preferably in free or bound form in an organism or a part thereof, as mentioned. In one embodiment, in the process of the present invention the activity of the protein YDR513W is increased.
The sequence of YGL237C from Saccharomyces cerevisiae has been published in Tettelin, H. et al. Nature 387 (6632 Suppl), 81-84 (1997) and its activity is being defined as transcriptional activator and global regulator of respiratory gene expression. Accordingly, in one embodiment, the process of the present invention comprises the use of a protein YGL237C from Saccharomyces cerevisiae or its homolog, e.g. as shown herein, for the production of the respective fine chemical, in particular for increasing the amount of glycerol and/or total lipid, preferably in free or bound form in an organism or a part thereof, as mentioned. In one embodiment, in the process of the present invention the activity of the protein YGL237C is increased.
The sequence of YIL150C from Saccharomyces cerevisiae has been published in Churcher, C. et al., Nature 387 (6632 Suppl), 84-87 (1997) and its activity is being defined as a protein having chromatin binding activity. Accordingly, in one embodiment, the process of the present invention comprises the use of a protein YIL150C, e.g. a protein required for S-phase iniation or completion from Saccharomyces cerevisiae or its homolog, e.g. as shown herein, for the production of the respective fine chemical, in particular for increasing the amount of glycerol and/or total lipid, preferably in free or bound form in an organism or a part thereof, as mentioned. In one embodiment, in the process of the present invention the activity of the protein YIL150C is increased.
The sequence of YLR082C from Saccharomyces cerevisiae has been published in Johnston, M. et al., Nature 387 (6632 Suppl), 87-90 (1997) and its activity has been characterized as a suppressor of Rad53 null lethality. Accordingly, in one embodiment, the process of the present invention comprises the use of a protein YLR082C from Saccharomyces cerevisiae or its homolog, e.g. as shown herein, for the production of the respective fine chemical, in particular for increasing the amount of glycerol, preferably in free or bound form in an organism or a part thereof, as mentioned. In one embodiment, in the process of the present invention the activity of the protein YLR082C is increased.
The sequence of YLR224W from Saccharomyces cerevisiae has been published in Johnston, M. et al., Nature 387 (6632 Suppl), 87-90 (1997) and its activity has not been characterized yet. Accordingly, in one embodiment, the process of the present invention comprises the use of a protein YLR224W from Saccharomyces cerevisiae or its homolog, e.g. as shown herein, for the production of the respective fine chemical, in particular for increasing the amount of glycerol and/or total lipid, preferably in free or bound form in an organism or a part thereof, as mentioned. In one embodiment, in the process of the present invention the activity of the protein YLR224W is increased.
The sequence of YLR255C from Saccharomyces cerevisiae has been published in the EMBL Data Library, February 1995.
MIPS_Reference: HYPERLINK "http://gcont01.basf-
ag.de:8000/biors/searchtool/searchtool.cgi?request=lookup_entry&file=entry&kind=fore ign&db_name=PIR&ref_id=S59386&ref_word=MIPS_Reference&use_sql=0&current_q uery_jd=%401092226732&total=3&_db=PIR&_db=SPTREMBL&_db=TREMBL_NEW& _ S59386 , Accession: S69301 and its activity has not been characterized yet. Accordingly, in one embodiment, the process of the present invention comprises the use of a protein YLR255C from Saccharomyces cerevisiae or its homolog, e.g. as shown herein, for the production of the respective fine chemical, in particular for increasing the amount of glycerol, preferably in free or bound form in an organism or a part thereof, as mentioned. In one embodiment, in the process of the present invention the activity of the protein YLR255C is increased.
The sequence of YMR015C from Saccharomyces cerevisiae has been published Bowman, S. et al., Nature 387 (6632 Suppl), 90-93 (1997) and its activity .is being defined as a protein having C-22 sterol desaturase activity. Accordingly, in one embodiment, the process of the present invention comprises the use of a protein YMR015C from Saccharomyces cerevisiae or its homolog, e.g. as shown herein, for the production of the respective fine chemical, in particular for increasing the amount of glycerol and/or total lipid, preferably in free or bound form in an organism or a part thereof, as mentioned. In one embodiment, in the process of the present invention the activity of the protein YMR015C is increased.
The sequence of YOR344C from Saccharomyces cerevisiae has been published in Dujon, B. et al., Nature 387 (6632 Suppl), 98-102 (1997) and its activity is being defined as a serine-rich protein being involved in glycolytic gene expression. Accordingly, in one embodiment, the process of the present invention comprises the use of a protein YOR344C from Saccharomyces cerevisiae or its homolog, e.g. as shown herein, for the production of the respective fine chemical, in particular for increasing the amount of glycerol and/or total lipid, preferably in free or bound form in an organism or a part thereof, as mentioned. In one embodiment, in the process of the present invention the activity of the protein YOR344C is increased.
The sequence of YPL099C from Saccharomyces cerevisiae has been published in Bussey, H. et al., Nature 387 (6632 Suppl), 103-105 (1997) and its activity is being defined as a putative membrane protein. Accordingly, in one embodiment, the process of the present invention comprises the use of a protein YPL099C from Saccharomyces cerevisiae or its homolog, e.g. as shown herein, for the production of the respective fine chemical, in particular for increasing the amount of glycerol, preferably in free or bound form in an organism or a part thereof, as mentioned. In one embodiment, in the process of the present invention the activity of the YPL099C protein is increased.
The sequence of YPL268W from Saccharomyces cerevisiae has been published in Bussey, H. et al., Nature 387 (6632 Suppl), 103-105 (1997) and its activity is being defined as a protein having phosphoinositide phospholipase activity. Accordingly, in one embodiment, the process of the present invention comprises the use of a protein YPL268W from Saccharomyces cerevisiae or its homolog, e.g. as shown herein, for the production of the respective fine chemical, in particular for increasing the amount of glycerol, preferably in free or bound form in an organism or a part thereof, as mentioned. In one embodiment, in the process of the present invention the activity of the YPL268W protein is increased.
The sequence of b271 0 (Accession number NP_417190) from Escherichia coli K12 has been published in Blattner et al., Science 277 (5331), 1453-1474, 1997, and its activity is being defined as a flavorubredoxin (FIRd) bifunctional NO and O₂ reductase. Accordingly, in one embodiment, the process of the present invention comprises the use of a flavorubredoxin (FIRd) bifunctional NO and O₂ reductase protein from E. coli or its homolog, e.g. as shown herein, for the production of the fine chemical, meaning of glycerol-3-phosphate and/or glycerides, lipids, oils and/or fats containing glycerol-3-phosphate, in particular for increasing the amount of glycerol-3-phosphate, preferably in free or bound form in an organism or a part thereof, as mentioned. In one embodiment, in the process of the present invention the activity of the protein b2710 is increased. In one embodiment, in the process of the present invention the activity of a flavorubredoxin (FIRd) bifunctional NO and O₂ reductase protein is increased or generated, e.g. from E. coli or a homolog thereof.
The sequence of b3498 (Accession number NP_417955) from Escherichia coli K12 has been published in Blattner et al., Science 277 (5331), 1453-1474, 1997, and its activity is being defined as a oligopeptidase A. Accordingly, in one embodiment, the process of the present invention comprises the use of a oligopeptidase A protein from E. coli or its homolog, e.g. as shown herein, for the production of the fine chemical, meaning of glycerol-3-phosphate and/or glycerides, lipids, oils and/or fats containing glycerol-3-phosphate, in particular for increasing the amount of glycerol-3-phosphate, preferably in free or bound form in an organism or a part thereof, as mentioned. In one embodiment, in the process of the present invention the activity of the protein b3498 is increased. In one embodiment, in the process of the present invention the activity of an oligopeptidase A protein is increased or generated, e.g. from E. coli or a homolog thereof.
The sequence of b3644 (Accession number NP_418101) from Escherichia coli K12 has been published in Blattner et al., Science 277 (5331), 1453-1474, 1997, and its activity is being defined as an uncharacterized stress-induced protein. Accordingly, in one embodiment, the process of the present invention comprises the use of an uncharacterized stress-induced protein from E. coli or its homolog, e.g. as shown herein, for the production of the fine chemical, meaning of glycerol and/or tryglycerides, lipids, oils and/or fats containing glycerol, in particular for increasing the amount of glycerol, preferably in free or bound form in an organism or a part thereof, as mentioned. In one embodiment, in the process of the present invention the activity of an uncharacterized stress-induced protein is increased or generated, e.g. from E. coli or a homolog thereof.
The sequence of b4073 (Accession number NP_418497) from Escherichia coli K12 has been published in Blattner et al., Science 277 (5331), 1453-1474, 1997, and its activity is being defined as a formate-dependent nitrate reductase. Accordingly, in one embodiment, the process of the present invention comprises the use of a formate-dependent nitrate reductase protein from E. coli or its homolog, e.g. as shown herein, for the production of the fine chemical, meaning of glycerol-3-phosphate and/or glycerides, lipids, oils and/or fats containing glycerol-3-phosphate, in particular for increasing the amount of glycerol-3-phosphate, preferably in free or bound form in an organism or a part thereof, as mentioned. In one embodiment, in the process of the present invention the activity of the protein b4073 is increased. In one embodiment, in the process of the present invention the activity of a formate-dependent nitrate reductase is increased or generated, e.g. from E. coli or a homolog thereof.
The sequence of YHR072W (Accession number NP_011939) from Escherichia coli K12 has been published in Goffeau et al., Science 274 (5287), 546-547 (1996) and Johnston et al. Science 265 (5181), 2077-2082 (1994), and its activity is being defined as a 2,3-oxidosqualene-lanosterol cyclase protein. Accordingly, in one embodiment, the process of the present invention comprises the use of a 2,3-oxidosqualene-lanosterol cyclase protein from E. coli or its homolog, e.g. as shown herein, for the production of the fine chemical, meaning of glycerol and/or tryglycerides, lipids, oils and/or fats containing glycerol, in particular for increasing the amount of glycerol, preferably in free or bound form in an organism or a part thereof, as mentioned. In one embodiment, in the process of the present invention the activity of a 2,3-oxidosqualene-lanosterol cyclase protein is increased or generated, e.g. from E. coli or a homolog thereof.

**[0023.0.21.21]** Homologues (= homologs) of the present gene products can be derived from any organisms as long as the homologue confers the herein mentioned activity, in particular, confers an increase in the respective fine chemical amount or content.
In one embodiment, the homolog of any one of the polypeptides indicated in Table II, column 3, line173 and lines 630 to 632 is a homolog having the same or a similar activity. In particular an increase of activity confers an increase in the content of the respective fine chemical in the organisms preferablyglycerol-3-phosphate.
In one embodiment, the homolog of the polypeptides indicated in Table II, column 3, lines 262 to 274, 628 and 629 is a homolog having the same or a similar activity. In particular an increase of activity confers an increase in the content of the respective fine chemical in the organisms preferablyglycerol and/or total lipid.
Homologs of the polypeptides indicated in Table II, column 3, lines 173 and lines 630 to 632 may be the polypeptides encoded by the nucleic acid molecules indicated in Table I, column 7, line 173 and lines 630 to 632 or may be the polypeptides indicated in Table II, column 7, line 173 and lines 630 to 632 having a glycerol-3-phosphate content and/or amount increasing activity.
Homologs of the polypeptides indicated in Table II, column 3, lines 262 to 267, 269, 271 to 274 may be the polypeptides encoded by the nucleic acid molecules indicated in Table I, column 7, lines 262 to 267, 269, 271 to 274 and 628 to 632, respectively or may be the polypeptides indicated in Table II, column 7, lines 262 to 267, 269, 271 to 274 and 628 to 632 having a glycerol and/or total lipid content and/or amount increasing activity.

**[0023.1.0.21]** Homologs of the polypeptides polypeptide indicated in Table II, column 3, lines 173, 262 to 274 and 628 to 632 may be the polypetides encoded by the nucleic acid molecules polypeptide indicated in Table I, column 7, lines 173, 262 to 274 and 628 to 632 or may be the polypeptides indicated in Table II, column 7, lines 173, 262 to 274 and 628 to 632.
Homologs of the polypeptides polypeptide indicated in Table II, column 3, lines 173, 262 to 274 and 628 to 632 may be the polypetides encoded by the nucleic acid molecules polypeptide indicated in Table I, column 7, lines 173, 262 to 274 and 628 to 632 or may be the polypeptides indicated in Table II, column 7, lines 173, 262 to 274 and 628 to 632.

**[0024.0.0.21]** for the disclosure of this paragraph see [0024.0.0.0] above.

**[0025.0.21.21]** In accordance with the invention, a protein or polypeptide has the "activity of a protein of the invention", e.g. the activity of a protein indicated in Table II, column 3, lines 173, 262 to 274 and 628 to 632 if its *de novo* activity, or its increased expression directly or indirectly leads to an increased glycerol-3-phosphate and/or glycerol and/or total lipid level, resp., in the organism or a part thereof, preferably in a cell of said organism. In a preferred embodiment, the protein or polypeptide has the above-mentioned additional activities of a protein indicated in Table II, column 3, lines 173, 262 to 274 and 628 to 632. Throughout the specification the activity or preferably the biological activity of such a protein or polypeptide or an nucleic acid molecule or sequence encoding such protein or polypeptide is identical or similar if it still has the biological or enzymatic activity of any one of the proteins indicated in Table II, column 3, lines 173, 262 to 274 and 628 to 632 or which has at least 10% of the original enzymatic activity, preferably 20%, particularly preferably 30%, most particularly preferably 40% in comparison to any one of the proteins indicated in Table II, column 3, line 262 and 263 and 628 and 630 to 632 of Escherichia coli K12 or line 173, 264 to 274 and 629 of Saccharomyces cerevisiae respectively.
In one embodiment, the polypeptide of the invention confers said activity, e.g. the increase of the respective fine chemical in an organism or a part thereof, if it is derived from an organism, which is evolutionary close to the organism indicated in Table I, column 4 and is expressed in an organism, which is evolutionary distant to the origin organism. For example origin and expressing organism are derived from different families, orders, classes or phylums whereas origin and the organism indicated in Table I, column 4 are derived from the same families, orders, classes or phylums.

**[0025.1.0.21] and [0025.2.0.21]** for the disclosure of the paragraphs [0025.1.0.21] and [0025.2.0.21] see [0025.1.0.0] and [0025.2.0.0] above.

**[0026.0.0.17] to [0033.0.0.17]** for the disclosure of the paragraphs [0026.0.0.17] to [0033.0.0.17] see [0026.0.0.0] to [0033.0.0.0] above.

**[0034.0.21.21]** Preferably, the reference, control or wild type differs from the subject of the present invention only in the cellular activity of the polypeptide of the invention, e.g. as result of an increase in the level of the nucleic acid molecule of the present invention or an increase of the specific activity of the polypeptide of the invention. E.g., it differs by or in the expression level or activity of a protein having the activity of a protein as indicated in Table II, column 3, lines 173, 262 to 274 and 628 to 632 or being encoded by a nucleic acid molecule indicated in Table I, column 5, lines 173, 262 to 274 and 628 to 632 or its homologs, e.g. as indicated in Table I, column 7, lines 173, 262 to 274 and 628 to 632, its biochemical or genetic causes. It therefore shows the increased amount of the respective fine chemical.

**[0035.0.0.21] to [0044.0.0.21]** for the disclosure of the paragraphs [0035.0.0.21] to [0044.0.0.21] see paragraphs [0035.0.0.0] to [0044.0.0.0] above.

**[0045.0.21.21]** In one embodiment, in case the activity of the Saccharomyces cerevisae protein YDR065W or its homologs, e.g. as indicated in Table II, columns 5 or 7, line 173 is increased, preferably, in one embodiment the increase of the respective fine chemical, preferably of glycerol-3-phosphate between 18% and 114% or more is conferred.
In one embodiment, in case the activity of the Escherichia coli K12 protein b2441 or its homologs, e.g. as indicated in Table II, columns 5 or 7, line 262 is increased, preferably, in one embodiment the increase of the respective fine chemical, preferably of glycerol in the polar fraction between 41 % and 81% or more is conferred.
In one embodiment, in case the activity of the Escherichia coli K12 proteinb3457 or its homologs, e.g. as indicated in Table II, columns 5 or 7, line 263 is increased, preferably, in one embodiment the increase of the respective fine chemical, preferably of glycerol in the lipid fraction between 18% and 44% or more is conferred.
In one embodiment, in case the activity of the Saccharomyces cerevisae protein YBR084W or its homologs, e.g. as indicated in Table II, columns 5 or 7, line 264 is increased, preferably, in one embodiment the increase of the respective fine chemical, preferably of glycerol in the polar fraction between 102% and 253% or more is conferred.
In one embodiment, in case the activity of the Saccharomyces cerevisae protein YDR513W or its homologs, e.g. as indicated in Table II, columns 5 or 7, line 265 is increased, preferably, in one embodiment the increase of the respective fine chemical, preferably of glycerol in the lipid fraction between 17% and 48% or more is conferred.
In one embodiment, in case the activity of the Saccharomyces cerevisae protein YDR513W or its homologs, e.g. as indicated in Table 11, columns 5 or 7, line 265 is increased, preferably, in one embodiment the increase of the respective fine chemical, preferably of glycerol in the polar fraction between 40% and 200% or more is conferred.
In one embodiment, in case the activity of the Saccharomyces cerevisae protein YGL237C or its homologs, e.g. as indicated in Table II, columns 5 or 7, line 266 is increased, preferably, in one embodiment the increase of the respective fine chemical, preferably of glycerol in the lipid fraction between 23% and 73% or more is conferred.
In one embodiment, in case the activity of the Saccharomyces cerevisae protein YIL150C or its homologs as indicated in Table II, columns 5 or 7, line 267, is increased, preferably, in one embodiment the increase of the respective fine chemical, preferably of glycerol lipid fraction between 94% and 219% or more is conferred.
In one embodiment, in case the activity of the Saccharomyces cerevisiae protein YLR082C or its homologs as indicated in Table II, columns 5 or 7, line 268, is increased, preferably, in one embodiment an increase of the respective fine chemical, preferably of glycerol in the polar fraction between 41 % and 173% or more is conferred.
In one embodiment, in case the activity of the Saccharomyces cerevisiae protein YRL224W or its homologs as indicated in Table II, columns 5 or 7, line 269, is increased, preferably, in one embodiment an increase of the respective fine chemical, preferably of glycerol in the lipid fraction between 17% and 43% or more is conferred.
In one embodiment, in case the activity of the Saccharomyces cerevisiae protein YLR255C or its homologs as indicated in Table II, columns 5 or 7, line 270, is increased, preferably, in one embodiment an increase of the respective fine chemical, preferably of glycerol in the polar fraction between 44% and 73% or more is conferred.
In one embodiment, in case the activity of the Saccharomyces cerevisiae protein YMR015C or its homologs as indicated in Table II, columns 5 or 7, line 271, is increased, preferably, in one embodiment an increase of the respective fine chemical, preferably of glycerol in the lipid fraction between 18% and 23% or more is conferred.
In one embodiment, in case the activity of the Saccharomyces cerevisiae protein YOR344C or its homologs as indicated in Table II, columns 5 or 7, line 272, is increased, preferably, in one embodiment an increase of the respective fine chemical, preferably of glycerol in the lipid fraction between 18% and 104% or more is conferred.
In one embodiment, in case the activity of the Saccharomyces cerevisiae protein YPL099C or its homologs as indicated in Table II, columns 5 or 7, line 273, is increased, preferably, in one embodiment an increase of the respective fine chemical, preferably of glycerol in the polar fraction between 55% and 104% or more is conferred.
In one embodiment, in case the activity of the Saccharomyces cerevisiae protein YPL268W or its homologs as indicated in Table II, columns 5 or 7, line 274, is increased, preferably, in one embodiment an increase of the respective fine chemical, preferably of glycerol in the polar fraction between 38% and 115% or more is conferred.
In case the activity of the Escherichia coli K12 protein b3644 or its homologs e.g. an uncharacterized stress-induced protein e.g. as indicated in Table II, columns 5 or 7, line 628, is increased, preferably of glycerol in the polar fraction between 17% and 44% or more is conferred.
In case the activity of the Saccharomyces cerevisiae protein YHR072W or its homologs e.g. a 2,3-oxidosqualene-lanosterol cyclase protein e.g. as indicated in Table II, columns 5 or 7, line 629, is increased, preferably of glycerol in the polar fraction between 19% and 109% or more is conferred.
In case the activity of the Escherichia coli K12 protein b2710 or its homologs e.g. a flavorubredoxin (FIRd) bifunctional NO and O₂ reductase e.g. as indicated in Table II, columns 5 or 7, line 630, is increased, preferably, in one embodiment the increase of the fine chemical, preferably of glycerol-3-phosphate between 20% and 95% or more is conferred.
In case the activity of the Escherichia coli K12 protein b3498 or its homologs e.g. a oligopeptidase A e.g. as indicated in Table II, columns 5 or 7, line 631, is increased, preferably, in one embodiment the increase of the fine chemical, preferably of glycerol-3-phosphate between 20% and 56% or more is conferred.
In case the activity of the Escherichia coli K12 protein b4073 or its homologs e.g. a formate-dependent nitrate reductase e.g. as indicated in Table II, columns 5 or 7, line 632, is increased, preferably, in one embodiment the increase of the fine chemical, preferably of glycerol-3-phosphate between 49% and 145% or more is conferred.
In addition in case the activity of the Saccharomyces cerevisiae protein YDR513W, YGL237C, YIL150C, YLR224W, YMR015C, YOR344C or YHR072W or the Escherichia coli K12 protein b3457, b3644, b2710, b3498 or b4073 or its homologs as indicated in Table II, columns 5 or 7, lines 265, 266, 267, 269, 271, 272 or 263 or 628 to 632 is increased in plant cells or especially in the seed of plants an increase in the total lipid content is conferred.

**[0046.0.0.21]** In one embodiment, in case the activity of the Saccharomyces cerevisiae protein YDR065W or its homologs is increased, preferably an increase of the fine chemical glycerol-3-phosphate is conferred.
In one embodiment, in case the activity of the Escherichia coli K12 protein b2441 or its homologs, e.g. a protein having ethanolamine ammonia-lyase activity increased, preferably an increase of the fine chemical glycerol is conferred.
In one embodiment, in case the activity of the Escherichia coli K12 protein b3457 or its homologs, e.g. a protein having branched -chain amino acid transport activity is increased, preferably an increase of the fine chemical glycerol is conferred.
In one embodiment, in case the activity of the Saccharomyces cerevisiae protein YBR084W or its homologs, e.g. a protein having C1-tetrahydrofolate synthase activity is increased, preferably an increase of the fine chemical glycerol is conferred.
In one embodiment, in case the activity of the Saccharomyces cerevisiae protein YDR513W or its homologs, e.g. a gluthatione reductase is increased, preferably an increase of the fine chemical glycerol is conferred.
In one embodiment, in case the activity of the Saccharomyces cerevisiae protein YGL237C or its homologs, e.g. a transcriptional activator protein and global regulator of respiratory gene expression activity is increased, preferably an increase of the fine chemical glycerol is conferred.
In one embodiment, in case the activity of the Saccharomyces cerevisae protein YIL150C or its homologs, e.g. a chromatin binding protein, required for S-phase (DNA synthesis) initiation or completion is increased, preferably an increase of the fine chemical glycerol is conferred.
In one embodiment, in case the activity of the Saccharomyces cerevisiae protein YLR082C or its homologs e.g. a suppressor of Rad53 null lethality is increased, preferably an increase of the fine chemical glycerol is conferred.
In one embodiment, in case the activity of the Saccharomyces cerevisiae protein YLR224W or its homologs is increased, preferably an increase of the fine chemical glycerol is conferred.
In one embodiment, in case the activity of the Saccharomyces cerevisiae protein YLR255C or its homologs is increased, preferably an increase of the fine chemical glycerol is conferred.
In one embodiment, in case the activity of the Saccharomyces cerevisae protein YMR015C or its homologs, e.g. a protein having C-22 sterol desaturase activity is increased, preferably an increase of the fine chemical glycerol is conferred.
In one embodiment, in case the activity of the Saccharomyces cerevisae protein YOR344C or its homologs, e.g. a serine-rich protein being involved in glycolytic gene expression is increased, preferably an increase of the fine chemical glycerol is conferred.
In one embodiment, in case the activity of the Saccharomyces cerevisae protein YPL099C or its homologs, e.g. a putative membrane protein is increased; preferably an increase of the fine chemical glycerol is conferred.
In one embodiment, in case the activity of the Saccharomyces cerevisae protein YPL268W or its homologs, e.g. a protein having phosphoinositide phospholipase activity is increased, preferably an increase of the fine chemical glycerol is conferred.
In one embodiment, in case the activity of the Escherichia coli K12 protein b3644 or its homologs, e.g. an uncharacterized stress-induced protein increased, preferably an increase of the fine chemical glycerol is conferred.
In one embodiment, in case the activity of the Saccharomyces cerevisae protein YHR072W or its homologs, e.g. an 2,3-oxidosqualene-lanosterol cyclase protein is increased, preferably an increase of the fine chemical glycerol is conferred.
In one embodiment, in case the activity of the Escherichia coli K12 protein b2710 or its homologs, e.g. a flavorubredoxin (FIRd) bifunctional NO and O₂ reductase is increased, preferably an increase of the fine chemical glycerol-3-phosphate is conferred.
In one embodiment, in case the activity of the Escherichia coli K12 protein b3498 or its homologs, e.g. a oligopeptidase A is increased, preferably an increase of the fine chemical glycerol-3-phosphate is conferred.
In one embodiment, in case the activity of the Escherichia coli K12 protein b4073 or its homologs, e.g. a formate-dependent nitrate reductase is increased, preferably an increase of the fine chemical glycerol-3-phosphate is conferred.

**[0047.0.0.21] and [0048.0.0.21]** for the disclosure of the paragraphs [0047.0.0.21] and [0048.0.0.21] see paragraphs [0047.0.0.0] and [0048.0.0.0] above.

**[0049.0.21.21]** A protein having an activity conferring an increase in the amount or level of the respective fine chemical glycerol-3-phosphate preferably has the structure of the polypeptide described herein. In a particular embodiment, the polypeptides used in the process of the present invention or the polypeptide of the present invention comprises the sequence of a consensus sequence as shown in SEQ ID NO: 15671 to 15676, or 97963 to 97965, or 98284 to 98287 or 98529 to 98532 or as indicated in Table IV, columns 7, lines 173 or 630 to 632 or of a polypeptide as indicated in Table II, columns 5 or 7, lines 173 or 630 to 632 or of a functional homologue thereof as described herein, or of a polypeptide encoded by the nucleic acid molecule characterized herein or the nucleic acid molecule according to the invention, for example by a nucleic acid molecule as indicated in Table I, columns 5 or 7, lines 173 or 630 to 632 or its herein described functional homologues and has the herein mentioned activity conferring an increase in the glycerol-3-phosphate level.
A protein having an activity conferring an increase in the amount or level of the respective fine chemical glycerol and/or total lipid preferably has the structure of the polypeptide described herein. In a particular embodiment, the polypeptides used in the process of the present invention or the polypeptide of the present invention comprises the sequence of a consensus sequence as shown in SEQ ID NO: 28565 to 28575 or 28576, 28577 or 28578 or 28579 to 28581 or 28582 to 28584 or 28585, 28586 or 28587 to 28590 or 28591 to 28594 or 28595 to 28597 or 28598 to 28600 or 28601 to 28605 or 98450 to 98452 or 98703 to 98713 or as indicated in Table IV, columns 7, lines 262 to 274 or 628 and 629 or of a polypeptide as indicated in Table II, columns 5 or 7, lines 262 to 274 or 628 and 629 or of a functional homologue thereof as described herein, or of a polypeptide encoded by the nucleic acid molecule characterized herein or the nucleic acid molecule according to the invention, for example by a nucleic acid molecule as indicated in Table I, columns 5 or 7, lines 262 to 274 or 628 and 629 or its herein described functional homologues and has the herein mentioned activity conferring an increase in the glycerol level.

**[0050.0.21.21]** For the purposes of the present invention, the term "the respective fine chemical" also encompass the corresponding salts, such as, for example, the potassium or sodium salts of glycerol-3-phosphate, resp., or their esters, e.g. monoacyl or diacyl fatty acids thereof.

**[0051.0.21.21]** Owing to the biological activity of the proteins which are used in the process according to the invention and which are encoded by nucleic acid molecules according to the invention, it is possible to produce compositions comprising the respective fine chemical, i.e. an increased amount of the free or bound, e.g compositions comprising glycerol and/or glycerol-3-phosphate and or total lipid. Depending on the choice of the organism used for the process according to the present invention, for example a microorganism or a plant, compositions or mixtures of glycerol and/or glycerol-3-phosphate and/or total lipid can be produced.

**[0052.0.0.21]** for the disclosure of this paragraph see paragraph [0052.0.0.0] above.

**[0053.0.21.21]** In one embodiment, the process of the present invention comprises one or more of the following steps
a) stabilizing a protein conferring the increased expression of a protein encoded by the nucleic acid molecule of the invention or of the polypeptid of the invention, e.g. of a polypeptide having an activity of a protein as indicated in Table II, column 3, lines 173, 262 to 274 and 628 to 632 or its homologs, e.g. as indicated in Table II, columns 5 or 7, lines 173, 262 to 274 and 628 to 632, activity having herein-mentioned the respective fine chemical increasing activity;
b) stabilizing a mRNA conferring the increased expression of a protein encoded by the nucleic acid molecule of the invention, e.g. of a polypeptide having an activity of a protein as indicated in Table II, column 3, lines 173, 262 to 274 and 628 to 632 or its homologs activity, e.g. as indicated in Table II, columns 5 or 7, lines 173, 262 to 274 and 628 to 632, or of a mRNA encoding the polypeptide of the present invention having herein-mentioned the respective fine chemical increasing activity;
c) increasing the specific activity of a protein conferring the increased expression of a protein encoded by the nucleic acid molecule of the invention or of the polypeptide of the present invention having herein-mentioned the respective fine chemical increasing activity, e.g. of a polypeptide having an activity of a protein as indicated in Table II, column 3, lines 173, 262 to 274 and 628 to 632 or its homologs activity, e.g. as indicated in Table II, columns 5 or 7, lines 173, 262 to 274 and 628 to 632, or decreasing the inhibitory regulation of the polypeptide of the invention;
d) generating or increasing the expression of an endogenous or artificial transcription factor mediating the expression of a protein conferring the increased expression of a protein encoded by the nucleic acid molecule of the invention or of the polypeptide of the invention having herein-mentioned the respective fine chemical increasing activity, e.g. of a polypeptide having an activity of a protein as indicated in Table II, column 3, lines 173, 262 to 274 and 628 to 632 or its homologs activity, e.g. as indicated in Table II, columns 5 or 7, lines 173, 262 to 274 and 628 to 632;
e) stimulating activity of a protein conferring the increased expression of a protein encoded by the nucleic acid molecule of the present invention or a polypeptide of the present invention having herein-mentioned the respective fine chemical increasing activity, e.g. of a polypeptide having an activity of a protein as indicated in Table II, column 3, lines 173, 262 to 274 and 628 to 632 or its homologs activity, e.g. as indicated in Table II, columns 5 or 7, lines 173, 262 to 274 and 628 to 632, by adding one or more exogenous inducing factors to the organism or parts thereof;
f) expressing a transgenic gene encoding a protein conferring the increased expression of a polypeptide encoded by the nucleic acid molecule of the present invention or a polypeptide of the present invention, having herein-mentioned the respective fine chemical increasing activity, e.g. of a polypeptide having an activity of a protein as indicated in Table II, column 3, lines 173, 262 to 274 and 628 to 632 or its homologs activity, e.g. as indicated in Table II, columns 5 or 7, lines 173, 262 to 274 and 628 to 632, and/or
g) increasing the copy number of a gene conferring the increased expression of a nucleic acid molecule encoding a polypeptide encoded by the nucleic acid molecule of the invention or the polypeptide of the invention having herein-mentioned the respective fine chemical increasing activity, e.g. of a polypeptide having an activity of a protein as indicated in Table II, column 3, lines 173, 262 to 274 and 628 to 632 or its homologs, e.g. as indicated in Table II, columns 5 or 7, lines 173, 262 to 274 and 628 to 632.
h) Increasing the expression of the endogenous gene encoding the polypeptide of the invention, e.g. a polypeptide having an activity of a protein as indicated in Table II, column 3, lines 173, 262 to 274 and 628 to 632 or its homologs activity, e.g. as indicated in Table II, columns 5 or 7, lines 173, 262 to 274 and 628 to 632, by adding positive expression or removing negative expression elements, e.g. homologous recombination can be used to either introduce positive regulatory elements like for plants the 35S enhancer into the promoter or to remove repressor elements form regulatory regions. Further gene conversion methods can be used to disrupt repressor elements or to enhance to activity of positive elements. Positive elements can be randomly introduced in plants by T-DNA or transposon mutagenesis and lines can be identified in which the positive elements have be integrated near to a gene of the invention, the expression of which is thereby enhanced; and/or
i) Modulating growth conditions of an organism in such a manner, that the expression or activity of the gene encoding the protein of the invention or the protein itself is enhanced for example microorganisms or plants can be grown for example under a higher temperature regime leading to an enhanced expression of heat shock proteins, which can lead to an enhanced respective fine chemical production; and/or
j) selecting of organisms with especially high activity of the proteins of the invention from natural or from mutagenized resources and breeding them into the target organisms, e.g. the elite crops.

**[0054.0.21.21]** Preferably, said mRNA is the nucleic acid molecule of the present invention and/or the protein conferring the increased expression of a protein encoded by the nucleic acid molecule of the present invention or the polypeptide having the herein mentioned activity is the polypeptide of the present invention, e.g. conferring the increase of the respective fine chemical after increasing the expression or activity of the encoded polypeptide or having the activity of a polypeptide having an activity of a protein as indicated in Table II, columns 3 or 5, lines 173, 262 to 274 and 628 to 632, resp., or its homologs activity, e.g. as indicated in Table II, columns 5 or 7, lines 173, 262 to 274 and 628 to 632, resp.

**[0055.0.0.21] to [0067.0.0.21]** for the disclosure of the paragraphs [0055.0.0.21] to [0067.0.0.21] see paragraphs [0055.0.0.0] to [0067.0.0.0] above.

**[0068.0.21.21]** The mutation is introduced in such a way that the production of the finde chemical, meaning glycerol and/or glycerol-3-phosphate and or total lipid is not adversely affected.

**[0069.0.0.21]** for the disclosure of this paragraph see paragraph [0069.0.0.0] above.

**[0070.0.21.21]** Owing to the introduction of a gene or a plurality of genes conferring the expression of the nucleic acid molecule of the invention or the polypeptide of the invention, for example the nucleic acid construct mentioned below, or encoding a protein of the invention into an organism alone or in combination with other genes, it is possible not only to increase the biosynthetic flux towards the end product, but also to increase, modify or create *de novo* an advantageous, preferably novel metabolite composition in the organism, e.g. an advantageous composition of glycerol-3-phosphate and glycerol or their biochemical derivatives , e.g. comprising a higher content of (from a viewpoint of nutrional physiology limited) glycerol-3-phosphate and glycerol or their derivatives including total lipids.

**[0071.0.0.21]** for the disclosure of this paragraph see paragraph [0071.0.0.0] above.

**[0072.0.0.21]** %

**[0073.0.21.21]** Accordingly, in one embodiment, the process according to the invention relates to a process, which comprises:
(a) providing a non-human organism, preferably a microorganism, a non-human animal, a plant or animal cell, a plant or animal tissue or a plant;
(b) increasing an activity of a polypeptide of the invention or a homolog thereof, e.g. as indicated in Table II, columns 5 or 7, lines 173, 262 to 274 and 628 to 632, or of a polypeptide being encoded by the nucleic acid molecule of the present invention and described below, e.g. conferring an increase of the respective fine chemical in an organism, preferably in a microorganism, a non-human animal, a plant or animal cell, a plant or animal tissue or a plant,
(c) growing an organism, preferably a microorganism, a non-human animal, a plant or animal cell, a plant or animal tissue or a plant under conditions which permit the production of the respective fine chemical in the organism, preferably the microorganism, the plant cell, the plant tissue or the plant; and
(d) if desired, recovering, optionally isolating, the free and/or bound respective fine chemical synthesized by the organism, the microorganism, the non-human animal, the plant or animal cell, the plant or animal tissue or the plant.

**[0074.0.21.21]** for the disclosure of this paragraph see paragraph [0071.0.0.17] above.

**[0075.0.0.21] to [0077.0.0.21]** for the disclosure of the paragraphs [0075.0.0.21] to [0077.0.0.21] see paragraphs [0075.0.0.0] to [0077.0.0.0] above.

**[0078.0.21.21]** The organism such as microorganisms or plants or the recovered, and if desired isolated, respective fine chemical can then be processed further directly into foodstuffs or animal feeds or for other applications. The fermentation broth, fermentation products, plants or plant products can be purified with methods known to the person skilled in the art. Products of these different work-up procedures are glycerol and/or glycerol-3-phosphate and/or glycerol as a component of lipids or comprising compositions of glycerol and/or glycerol-3-phosphate and/or glycerol as a component of lipids still comprising fermentation broth, plant particles and cell components in different amounts, advantageously in the range of from 0 to 99% by weight, preferably below 80% by weight, especially preferably below 50% by weight.

**[0079.0.0.21] to [0084.0.0.21]** for the disclosure of the paragraphs [0079.0.0.21] to [0084.0.0.21] see paragraphs [0079.0.0.0] to [0084.0.0.0] above.

**[0085.0.21.21]** With regard to the nucleic acid sequence as depicted a nucleic acid construct which contains a nucleic acid sequence mentioned herein or an organism (= transgenic organism) which is transformed with said nucleic acid sequence or said nucleic acid construct, "transgene" means all those constructs which have been brought about by genetic manipulation methods, preferably in which either
a) a nucleic acid sequence as indicated in Table I, columns 5 or 7, lines 173, 262 to 274 and 628 to 632, or a derivative thereof, or
b) a genetic regulatory element, for example a promoter, which is functionally linked to the nucleic acid sequence as indicated in Table I, columns 5 or 7, lines 173, 262 to 274 and 628 to 632, or a derivative thereof, or
c) (a) and (b)
is/are not present in its/their natural genetic environment or has/have been modified by means of genetic manipulation methods, it being possible for the modification to be, by way of example, a substitution, addition, deletion, inversion or insertion of one or more nucleotide. "Natural genetic environment" means the natural chromosomal locus in the organism of origin or the presence in a genomic library. In the case of a genomic library, the natural, genetic environment of the nucleic acid sequence is preferably at least partially still preserved. The environment flanks the nucleic acid sequence at least on one side and has a sequence length of at least 50 bp, preferably at least 500 bp, particularly preferably at least 1000 bp, very particularly preferably at least 5000 bp.

**[0086.0.0.21] and [0087.0.0.21]** for the disclosure of the paragraphs [0086.0.0.21] and [0087.0.0.21] see paragraphs [0086.0.0.0] and [0087.0.0.0] above.

**[0088.0.21.21]** In an advantageous embodiment of the invention, the organism takes the form of a plant whose content of the respective fine chemical is modified advantageously owing to the nucleic acid molecule of the present invention expressed.
This is important for plant breeders since, for example, the nutritional value of plants for poultry is dependent on the abovementioned fine chemicals or the plants are more resistant to biotic and abiotic stress and the yield is increased.

**[0088.1.0.21]** for the disclosure of this paragraph see paragraph [0088.1.0.0] above.

**[0089.0.0.21] to [0094.0.0.21]** for the disclosure of the paragraphs [0089.0.0.21] to [0094.0.0.21] see paragraphs [0088.0.0.0] to [0094.0.0.0] above.

**[0095.0.21.21]** It may be advantageous to increase the pool of glycerol and/or glycerol-3-phosphate and/or total lipid in the transgenic organisms by the process according to the invention in order to isolate high amounts of the pure respective fine chemical and/or to obtain increased resistance against biotic and abiotic stresses and to obtain higher yield.

**[0096.0.21.21]** In another preferred embodiment of the invention a combination of the increased expression of the nucleic acid sequence or the protein of the invention together with the transformation of a protein or polypeptid or a compound, which functions as a sink for the desired fine chemical, for example in the organism, is useful to increase the production of the respective fine chemical.

**[0097.0.0.21]** for the disclosure of this paragraph see paragraph [0097.0.0.0] above.

**[0098.0.21.21]** In a preferred embodiment, the respective fine chemical is produced in accordance with the invention and, if desired, is isolated.

**[0099.0.21.21]** In the case of the fermentation of microorganisms, the abovementioned desired fine chemical may accumulate in the medium and/or the cells. If microorganisms are used in the process according to the invention, the fermentation broth can be processed after the cultivation. Depending on the requirement, all or some of the biomass can be removed from the fermentation broth by separation methods such as, for example, centrifugation, filtration, decanting or a combination of these methods, or else the biomass can be left in the fermentation broth. The fermentation broth can subsequently be reduced, or concentrated, with the aid of known methods such as, for example, rotary evaporator, thin-layer evaporator, falling film evaporator, by reverse osmosis or by nanofiltration. Afterwards advantageously further compounds for formulation can be added such as corn starch or silicates. This concentrated fermentation broth advantageously together with compounds for the formulation can subsequently be processed by lyophilization, spray drying, and spray granulation or by other methods. Preferably the respective fine chemical comprising compositions are isolated from the organisms, such as the microorganisms or plants or the culture medium in or on which the organisms have been grown, or from the organism and the culture medium, in the known manner, for example via extraction, distillation, crystallization, chromatography or a combination of these methods. These purification methods can be used alone or in combination with the aforementioned methods such as the separation and/or concentration methods.

**[0100.0.21.21]** Transgenic plants which comprise the fine chemicals such as glycerol and/or glycerol-3-phosphate and/or total lipids synthesized in the process according to the invention can advantageously be marketed directly without there being any need for the fine chemicals synthesized to be isolated. Plants for the process according to the invention are listed as meaning intact plants and all plant parts, plant organs or plant parts such as leaf, stem, seeds, root, tubers, anthers, fibers, root hairs, stalks, embryos, calli, cotelydons, petioles, harvested material, plant tissue, reproductive tissue and cell cultures which are derived from the actual transgenic plant and/or can be used for bringing about the transgenic plant. In this context, the seed comprises all parts of the seed such as the seed coats, epidermal cells, seed cells, endosperm or embryonic tissue.
However, the respective fine chemical produced in the process according to the invention can also be isolated from the organisms, advantageously plants, as extracts, e.g. ether, alcohol, or other organic solvents or water containing extract and/or free fine chemicals. The respective fine chemical produced by this process can be obtained by harvesting the organisms, either from the crop in which they grow, or from the field. This can be done via pressing or extraction of the plant parts. To increase the efficiency of extraction it is beneficial to clean, to temper and if necessary to hull and to flake the plant material. To allow for greater ease of disruption of the plant parts, specifically the seeds, they can previously be comminuted, steamed or roasted. Seeds, which have been pretreated in this manner can subsequently be pressed or extracted with solvents such as warm hexane. The solvent is subsequently removed. In the case of microorganisms, the latter are, after harvesting, for example extracted directly without further processing steps or else, after disruption, extracted via various methods with which the skilled worker is familiar. Thereafter, the resulting products can be processed further, i.e. degummed and/or refined. In this process, substances such as the plant mucilages and suspended matter can be first removed. What is known as desliming can be affected enzymatically or, for example, chemico-physically by addition of acid such as phosphoric acid.
Because glycerol and/or glycerol-3-phosphate and /or total lipids in microorganisms are localized intracellular, their recovery essentially comes down to the isolation of the biomass. Well-established approaches for the harvesting of cells include filtration, centrifugation and coagulation/flocculation as described herein. Of the residual hydrocarbon, adsorbed on the cells, has to be removed. Solvent extraction or treatment with surfactants have been suggested for this purpose.

**[0101.0.21.21]** The identity and purity of the compound(s) isolated can be determined by prior-art techniques. They encompass high-performance liquid chromatography (HPLC), gas chromatography (GC), spectroscopic methods, mass spectrometry (MS), staining methods, thin-layer chromatography, NIRS, enzyme assays or microbiological assays. These analytical methods are compiled in: Patek et al. (1994) Appl. Environ. Microbiol. 60:133-140; Malakhova et al. (1996) Biotekhnologiya 11 27-32; and Schmidt et al. (1998) Bioprocess Engineer. 19:67-70. Ulmann's Encyclopedia of Industrial Chemistry (1996) Bd. A27, VCH Weinheim, pp. 89-90, pp. 521-540, pp. 540-547, pp. 559-566, 575-581 and pp. 581-587; Michal, G (1999) Biochemical Pathways: An Atlas of Biochemistry and Molecular Biology, John Wiley and Sons; Fallon, A. et al. (1987) Applications of HPLC in Biochemistry in: Laboratory Techniques in Biochemistry and Molecular Biology, vol. 17.

**[0102.0.21.21]** Glycerol and/or glycerol-3-phosphate can for example be analyzed advantageously via HPLC, LC or GC separation and MS (masspectrometry) detection methods. The unambiguous detection for the presence of glycerol and/or glycerol-3-phosphate containing products can be obtained by analyzing recombinant organisms using analytical standard methods: LC, LC-MS, MS or TLC). The material to be analyzed can be disrupted by sonication, grinding in a glass mill, liquid nitrogen and grinding, cooking, or via other applicable methods.
Total lipids in the seed of plants can for example be analyzed advantageously by lipid extraction as described by Bligh and Dyer (Can J Biochem Phys 1959, 37, 911-917) followed by the determination of the lipid content by gaschromatography as described by Benning and Sommerville (J Bacteriol 1992, 174, 6479-6487).

**[0103.0.21.21]** In a preferred embodiment, the present invention relates to a process for the production of the respective fine chemical comprising or generating in an organism or a part thereof the expression of at least one nucleic acid molecule comprising a nucleic acid molecule selected from the group consisting of:
a) nucleic acid molecule encoding, preferably at least the mature form, of the polypeptide having a sequence as indicated in Table II, columns 5 or 7, lines 173, 262 to 274 and 628 to 632, or a fragment thereof, which confers an increase in the amount of the respective fine chemical in an organism or a part thereof;
b) nucleic acid molecule comprising, preferably at least the mature form, of a nucleic acid molecule having a sequence as indicated in Table I, columns 5 or 7, lines 173, 262 to 274 and 628 to 632;
c) nucleic acid molecule whose sequence can be deduced from a polypeptide sequence encoded by a nucleic acid molecule of (a) or (b) as result of the degeneracy of the genetic code and conferring an increase in the amount of the respective fine chemical in an organism or a part thereof;
d) nucleic acid molecule encoding a polypeptide which has at least 50% identity with the amino acid sequence of the polypeptide encoded by the nucleic acid molecule of (a) to (c) and conferring an increase in the amount of the respective fine chemical in an organism or a part thereof;
e) nucleic acid molecule which hybridizes with a nucleic acid molecule of (a) to (c) under stringent hybridization conditions and conferring an increase in the amount of the respective fine chemical in an organism or a part thereof;
f) nucleic acid molecule encoding a polypeptide, the polypeptide being derived by substituting, deleting and/or adding one or more amino acids of the amino acid sequence of the polypeptide encoded by the nucleic acid molecules (a) to (d), preferably to (a) to (c) and conferring an increase in the amount of the respective fine chemical in an organism or a part thereof;
g) nucleic acid molecule encoding a fragment or an epitope of a polypeptide which is encoded by one of the nucleic acid molecules of (a) to (e), preferably to (a) to (c) and and conferring an increase in the amount of the respective fine chemical in an organism or a part thereof;
h) nucleic acid molecule comprising a nucleic acid molecule which is obtained by amplifying nucleic acid molecules from a cDNA library or a genomic library using the primer pairs having a sequence as indicated in Table III, column 7, lines 173, 262 to 274 and 628 to 632, and conferring an increase in the amount of the respective fine chemical in an organism or a part thereof;
i) nucleic acid molecule encoding a polypeptide which is isolated, e.g. from an expression library, with the aid of monoclonal antibodies against a polypeptide encoded by one of the nucleic acid molecules of (a) to (h), preferably to (a) to (c), and conferring an increase in the amount of the respective fine chemical in an organism or a part thereof;
j) nucleic acid molecule which encodes a polypeptide comprising the consensus sequence having sequences as indicated in Table IV, column 7, lines 173, 262 to 267, 269 and 271 to 274 and 628 to 632 and conferring an increase in the amount of the respective fine chemical in an organism or a part thereof;
k) nucleic acid molecule comprising one or more of the nucleic acid molecule encoding the amino acid sequence of a polypeptide encoding a domain of the polypeptide indicated in Table II, columns 5 or 7, lines 173, 262 to 274 and 628 to 632, and conferring an increase in the amount of the respective fine chemical in an organism or a part thereof; and
l) nucleic acid molecule which is obtainable by screening a suitable library under stringent conditions with a probe comprising one of the sequences of the nucleic acid molecule of (a) to (k), preferably to (a) to (c), or with a fragment of at least 15 nt, preferably 20 nt, 30 nt, 50 nt, 100 nt, 200 nt or 500 nt of the nucleic acid molecule characterized in (a) to (k), preferably to (a) to (c), and conferring an increase in the amount of the respective fine chemical in an organism or a part thereof;
or which comprises a sequence which is complementary thereto.

**[00103.1.21.21]** In one embodiment, the nucleic acid molecule used in the process of the invention distinguishes over the sequence indicated in Table IA, columns 5 or 7, lines 173, 262 to 274 and 628 to 632, by one or more nucleotides. In one embodiment, the nucleic acid molecule used in the process of the invention does not consist of the sequence shown in indicated in Table I A, columns 5 or 7, lines 173, 262 to 274 and 628 to 632. In one embodiment, the nucleic acid molecule used in the process of the invention is less than 100%, 99,999%, 99,99%, 99,9% or 99% identical to a sequence indicated in Table I A, columns 5 or 7, lines 173, 262 to 274 and 628 to 632. In another embodiment, the nucleic acid molecule does not encode a polypeptide of a sequence indicated in Table II A, columns 5 or 7, lines 173, 262 to 274 and 628 to 632.

**[00103.2.21.21]** In one embodiment, the nucleic acid molecule used in the process of the invention distinguishes over the sequence indicated in Table I B, columns 5 or 7, lines 173, 262 to 274 and 628 to 632, by one or more nucleotides. In one embodiment, the nucleic acid molecule used in the process of the invention does not consist of the sequence shown in indicated in Table I B, columns 5 or 7, lines 173, 262 to 274 and 628 to 632. In one embodiment, the nucleic acid molecule used in the process of the invention is less than 100%, 99,999%, 99,99%, 99,9% or 99% identical to a sequence indicated in Table I B, columns 5 or 7, lines 173, 262 to 274 and 628 to 632. In another embodiment, the nucleic acid molecule does not encode a polypeptide of a sequence indicated in Table II B, columns 5 or 7, lines 173, 262 to 274 and 628 to 632.

**[0104.0.21.21]** In one embodiment, the nucleic acid molecule used in the process of the present invention distinguishes over the sequence indicated in Table I, columns 5 or 7, lines 173, 262 to 274 and 628 to 632, preferably over the sequences as shown in Table I A, columns 5 or 7, lines 173, 262 to 274 and 628 to 632 by one or more nucleotides. In one embodiment, the nucleic acid molecule used in the process of the invention does not consist of the sequence indicated in Table I, columns 5 or 7, lines 173, 262 to 274 and 628 to 632. In one embodiment, the nucleic acid molecule of the present invention is less than 100%, 99,999%, 99,99%, 99,9% or 99% identical to the sequence indicated in Table I, columns 5 or 7, lines 173, 262 to 274 and 628 to 632, preferably the nucleic acid molecule of the present invention is less than 100%, 99,999%, 99,99%, 99,9% or 99% identical to the sequence as shown in Table I A, columns 5 or 7, lines 173, 262 to 274 and 628 to 632. In another embodiment, the nucleic acid molecule does not encode a polypeptide of a sequence indicated in Table II, columns 5 or 7, lines 173, 262 to 274 and 628 to 632, preferably as indicated in Table IIA, columns 5 or 7, lines 173, 262 to 274 and 628 to 632.

**[0105.0.0.21] to [0107.0.0.21]** for the disclosure of the paragraphs [0105.0.0.21] to [0107.0.0.21] see paragraphs [0105.0.0.0] to [0107.0.0.0] above.

**[0108.0.21.21]** Nucleic acid molecules with the sequence as indicated in Table I, columns 5 or 7, lines 173, 262 to 274 and 628 to 632, nucleic acid molecules which are derived from an amino acid sequences as indicated in Table II, columns 5 or 7, lines 173, 262 to 274 and 628 to 632 or from polypeptides comprising the consensus sequence as indicated in Table IV, column 7, lines 173, 262 to 267, 269 and 271 to 274 and 628 to 632, or their derivatives or homologues encoding polypeptides with the enzymatic or biological activity of an activity of a polypeptide as indicated in Table II, column 3, 5 or 7, lines 173, 262 to 274 and 628 to 632, e.g. conferring the increase of the respective fine chemical, meaning glycerol and/or glycerol-3-phosphate and/or total lipid, resp., after increasing its expression or activity, are advantageously increased in the process according to the invention.

**[0109.0.21.21]** In one embodiment, said sequences are cloned into nucleic acid constructs, either individually or in combination. These nucleic acid constructs enable an optimal synthesis of the respective fine chemicals, in particular glycerol and/or glycerol-3-phosphate and/or total lipids, produced in the process according to the invention.

**[0110.0.0.21]** Nucleic acid molecules, which are advantageous for the process according to the invention and which encode polypeptides with an activity of a polypeptide used in the method of the invention or used in the process of the invention, e.g. of a protein as shown in Table II, columns 5 or 7, lines 173, 262 to 274 and 628 to 632 or being encoded by a nucleic acid molecule indicated in Table I, columns 5 or 7, lines 173, 262 to 274 and 628 to 632 or of its homologs, e.g. as indicated in Table II, columns 5 or 7, lines 173, 262 to 274 and 628 to 632 can be determined from generally accessible databases.

**[0111.0.0.21]** for the disclosure of this paragraph see [0111.0.0.0] above.

**[0112.0.21.21]** The nucleic acid molecules used in the process according to the invention take the form of isolated nucleic acid sequences, which encode polypeptides with an activity of a polypeptide as indicated in Table II, column 3, lines 173, 262 to 274 and 628 to 632 or having the sequence of a polypeptide as indicated in Table II, columns 5 and 7, lines 173, 262 to 274 and 628 to 632 and conferring an increase in the glycerol and/or glycerol-3-phosphate and/or total lipid level.

**[0113.0.0.21] to [0120.0.0.21]** for the disclosure of the paragraphs [0113.0.0.21] to [0120.0.0.21] see paragraphs [0113.0.0.0] and [0120.0.0.0] above.

**[0121.0.21.21]** However, it is also possible to use artificial sequences, which differ in one or more bases from the nucleic acid sequences found in organisms, or in one or more amino acid molecules from polypeptide sequences found in organisms, in particular from the polypeptide sequences indicated in Table II, columns 5 or 7, line 173 and lines 630 to 632 or the functional homologues thereof as described herein, preferably conferring above-mentioned activity, i.e. conferring a glycerol-3-phosphate increase after increasing the activity of the polypeptide sequences indicated in Table II, columns 5 or 7, line 173 and lines 630 to 632 and conferring a glycerol and/or total lipid level increase after increasing the activity of the polypeptide sequences indicated in Table II, columns 5 or 7, lines 262 to 274 and lines 628 and 629.

**[0122.0.0.21] to [0127.0.0.21]** for the disclosure of the paragraphs [0122.0.0.21] to [0127.0.0.21] see paragraphs [0122.0.0.0] and [0127.0.0.0] above.

**[0128.0.21.21]** Synthetic oligonucleotide primers for the amplification, e.g. as the pairs indicated in Table III, column 7, lines 173, 262 to 274 and 628 to 632, by means of polymerase chain reaction can be generated on the basis of a sequence shown herein, for example the sequence as indicated in Table I, columns 5 or 7, lines 173, 262 to 274 and 628 to 632, resp. or the sequences derived from a sequences as indicated in Table II, columns 5 or 7, lines 173, 262 to 274 and 628 to 632, resp.

**[0129.0.21.21]** Moreover, it is possible to identify conserved regions from various organisms by carrying out protein sequence alignments with the polypeptide used in the process of the invention, in particular with sequences of the polypeptide of the invention, from which conserved regions, and in turn, degenerate primers can be derived. Conserved regions are those, which show a very little variation in the amino acid in one particular position of several homologs from different origin. The consensus sequence indicated in Table IV, columns 7, lines 173, 262 to 267, 269 and 271 to 274 and 628 to 632 is derived from such alignments.

**[0130.0.21.21]** Degenerated primers can then be utilized by PCR for the amplification of fragments of novel proteins having above-mentioned activity, e.g. conferring the increase of glycerol and/or glycerol-3-phosphate and/or total lipid after increasing the expression or activity the protein comprising said fragment.

**[0131.0.0.21] to [0138.0.0.21]** for the disclosure of the paragraphs [0131.0.0.21] to [0138.0.0.21] see paragraphs [0131.0.0.0] to [0138.0.0.0] above.

**[0139.0.21.21]** Polypeptides having above-mentioned activity, i.e. conferring the respective fine chemical level increase, derived from other organisms, can be encoded by other DNA sequences which hybridise to a sequence indicated in Table I, columns 5 or 7, line 173 and lines 630 to 632 for glycerol-3-phosphate or indicated in Table I, columns 5 or 7, lines 262 to 274 and lines 628 and 629 for glycerol, preferably of Table I B, columns 5 or 7, line 173 and lines 630 to 632 or lines 262 to 274 and lines 628 and 629 respectively and/or total lipid under relaxed hybridization conditions and which code on expression for peptides having the respective fine chemical, i.e. glycerol and/or glycerol-3-phosphate and/or total lipids, resp., increasing-activity.

**[0140.0.0.21] to [0146.0.0.21]** for the disclosure of the paragraphs [0140.0.0.21] to [0146.0.0.21] see paragraphs [0140.0.0.0] to [0146.0.0.0] above.

**[0147.0.21.21]** Further, the nucleic acid molecule of the invention comprises a nucleic acid molecule, which is a complement of one of the nucleotide sequences of above mentioned nucleic acid molecules or a portion thereof. A nucleic acid molecule which is complementary to one of the nucleotide sequences indicated in Table I, columns 5 or 7, lines 173, 262 to 274 and 628 to 632, preferably of Table I B, columns 5 or 7, lines 173, 262 to 274 and 628 to 632 is one which is sufficiently complementary to one of said nucleotide sequences such that it can hybridise to one of said nucleotide sequences, thereby forming a stable duplex. Preferably, the hybridisation is performed under stringent hybrization conditions. However, a complement of one of the herein disclosed sequences is preferably a sequence complement thereto according to the base pairing of nucleic acid molecules well known to the skilled person. For example, the bases A and G undergo base pairing with the bases T and U or C, resp. and visa versa. Modifications of the bases can influence the base-pairing partner.

**[0148.0.21.21]** The nucleic acid molecule of the invention comprises a nucleotide sequence which is at least about 30%, 35%, 40% or 45%, preferably at least about 50%, 55%, 60% or 65%, more preferably at least about 70%, 80%, or 90%, and even more preferably at least about 95%, 97%, 98%, 99% or more homologous to a nucleotide sequence indicated in Table I, columns 5 or 7, lines 173, 262 to 274 and 628 to 632, preferably of Table I B, columns 5 or 7, lines 173, 262 to 274 and 628 to 632 or a portion thereof and preferably has above mentioned activity, in particular having a glycerol and/or glycerol-3-phosphate and/or total lipid increasing activity after increasing the activity or an activity of a product of a gene encoding said sequences or their homologs.

**[0149.0.21.21]** The nucleic acid molecule of the invention comprises a nucleotide sequence which hybridizes, preferably hybridizes under stringent conditions as defined herein, to one of the nucleotide sequences indicated in Table I, columns 5 or 7, lines 173, 262 to 274 and 628 to 632, preferably of Table I B, columns 5 or 7, lines 173, 262 to 274 and 628 to 632 or a portion thereof and encodes a protein having above-mentioned activity, e.g. conferring an increase of glycerol-3-phosphate and/or glycerol, resp., and optionally, the activity of protein indicated in Table II, column 5, lines 173, 262 to 274 and 628 to 632, preferably of Table II B, columns 5 or 7, lines 173, 262 to 274 and 628 to 632.

**[00149.1.21.21]** Optionally, in one embodiment, the nucleotide sequence, which hybridises to one of the nucleotide sequences indicated in Table I, columns 5 or 7, lines 173, 262 to 274 and 628 to 632, preferably of Table I B, columns 5 or 7, lines 173, 262 to 274 and 628 to 632 has further one or more of the activities annotated or known for a protein as indicated in Table II, column 3, lines 173, 262 to 274 and 628 to 632, preferably of Table II B, columns 5 or 7, lines 173, 262 to 274 and 628 to 632.

**[0150.0.21.21]** Moreover, the nucleic acid molecule of the invention can comprise only a portion of the coding region of one of the sequences indicated in Table I, columns 5 or 7, lines 173, 262 to 274 and 628 to 632, preferably of Table I B, columns 5 or 7, lines 173, 262 to 274 and 628 to 632 for example a fragment which can be used as a probe or primer or a fragment encoding a biologically active portion of the polypeptide of the present invention or of a polypeptide used in the process of the present invention, i.e. having above-mentioned activity, e.g.conferring an increase of gglycerol-3-phosphate and/or glycerol , resp., if its activity is increased. The nucleotide sequences determined from the cloning of the present protein-according-to-the-invention-encoding gene allows for the generation of probes and primers designed for use in identifying and/or cloning its homologues in other cell types and organisms. The probe/primer typically comprises substantially purified oligonucleotide. The oligonucleotide typically comprises a region of nucleotide sequence that hybridizes under stringent conditions to at least about 12, 15 preferably about 20 or 25, more preferably about 40, 50 or 75 consecutive nucleotides of a sense strand of one of the sequences set forth, e.g., as indicated in Table I, columns 5 or 7, lines 173, 262 to 274 and 628 to 632, an anti-sense sequence of one of the sequences, e.g., as indicated in Table I, columns 5 or 7, lines 173, 262 to 274 and 628 to 632, or naturally occurring mutants thereof. Primers based on a nucleotide of invention can be used in PCR reactions to clone homologues of the polypeptide of the invention or of the polypeptide used in the process of the invention, e.g. as the primers described in the examples of the present invention, e.g. as shown in the examples. A PCR with the primer pairs indicated in Table III, column 7, lines 173, 262 to 274 and 628 to 632 will result in a fragment of a polynucleotide sequence as indicated in Table I, columns 5 or 7, lines 173, 262 to 274 and 628 to 632 or its gene product. Preferred is Table I B, column 7, lines 173, 262 to 274 and 628 to 632

**[0151.0.0.21]:** for the disclosure of this paragraph see paragraph [0151.0.0.0] above.

**[0152.0.21.21]** The nucleic acid molecule of the invention encodes a polypeptide or portion thereof which includes an amino acid sequence which is sufficiently homologous to an amino acid sequence as indicated in Table II, columns 5 or 7, lines 173, 262 to 274 and 628 to 632 such that the protein or portion thereof maintains the ability to participate in the respective fine chemical production, in particular a glycerol-3-phosphate (line173 and lines 630 to 632) or glycerol (lines 262 to 274 and lines 628 and 629) and total lipid (lines 265, 266, 267, 269, 271, 272 and 263) increasing activity as mentioned above or as described in the examples in plants or microorganisms is comprised.

**[0153.0.21.21]** As used herein, the language "sufficiently homologous" refers to proteins or portions thereof which have amino acid sequences which include a minimum number of identical or equivalent amino acid residues (e.g., an amino acid residue which has a similar side chain as an amino acid residue in one of the sequences of the polypeptide of the present invention) to an amino acid sequence as indicated in Table II, columns 5 or 7, lines 173, 262 to 274 and 628 to 632 such that the protein or portion thereof is able to participate in the increase of the respective fine chemical production. In one embodiment, a protein or portion thereof as indicated in Table II, columns 5 or 7, lines 173, 262 to 274 and 628 to 632 has for example an activity of a polypeptide indicated in Table II, column 3, lines 173, 262 to 274 and 628 to 632.

**[0154.0.21.21]** In one embodiment, the nucleic acid molecule of the present invention comprises a nucleic acid that encodes a portion of the protein of the present invention. The protein is at least about 30%, 35%, 40%, 45% or 50%, preferably at least about 55%, 60%, 65% or 70%, and more preferably at least about 75%, 80%, 85%, 90%, 91 %, 92%, 93% or 94% and most preferably at least about 95%, 97%, 98%, 99% or more homologous to an entire amino acid sequence as indicated in Table II, columns 5 or 7, lines 173, 262 to 274 and 628 to 632 and has above-mentioned activity, e.g.conferring preferably the increase of the respective fine chemical.

**[0155.0.0.21] and [0156.0.0.21]** for the disclosure of the paragraphs [0155.0.0.21] and [0156.0.0.21] see paragraphs [0155.0.0.0] and [0156.0.0.0] above.

**[0157.0.21.21]** The invention further relates to nucleic acid molecules that differ from one of the nucleotide sequences as indicated in Table I, columns 5 or 7, lines 173, 262 to 274 and 628 to 632 (and portions thereof) due to degeneracy of the genetic code and thus encode a polypeptide of the present invention, in particular a polypeptide having above mentioned activity, e.g. conferring an increase in the respective fine chemical in a organism, e.g. as polypeptides comprising the sequence as indicated in Table IV, column 7, lines 173, 262 to 274 and 628 to 632 or as polypeptides depicted in Table II, columns 5 or 7, lines 173, 262 to 274 and 628 to 632 or the functional homologues. Advantageously, the nucleic acid molecule of the invention comprises, or in an other embodiment has, a nucleotide sequence encoding a protein comprising, or in an other embodiment having, an amino acid sequence of a consensus sequences as indicated in Table IV, column 7, lines 173, 262 to 267, 269 and 271 to 274 or of the polypeptide as indicated in Table II, columns 5 or 7, lines 173, 262 to 274 and 628 to 632, resp., or the functional homologues. In a still further embodiment, the nucleic acid molecule of the invention encodes a full length protein which is substantially homologous to an amino acid sequence comprising a consensus sequence as indicated in Table IV, column 7, lines 173, 262 to 267, 269 and 271 to 274 or of a polypeptide as indicated in Table II, columns 5 or 7, lines 173, 262 to 274 and 628 to 632 or the functional homologues. However, in a preferred embodiment, the nucleic acid molecule of the present invention does not consist of a sequence as indicated in Table I, columns 5 or 7, lines 173, 262 to 274 and 628 to 632, preferably as indicated in Table I A, columns 5 or 7, lines 173, 262 to 274 and 628 to 632, resp. Preferably the nucleic acid molecule of the invention is a functional homologue or identical to a nucleic acid molecule indicated in Table I B, column 7, lines 173, 262 to 274 and 628 to 632.

**[0158.0.0.21] to [0160.0.0.21]** for the disclosure of the paragraphs [0158.0.0.21] to [0160.0.0.21] see paragraphs [0158.0.0.0] to [0160.0.0.0] above.

**[0161.0.21.21]** Accordingly, in another embodiment, a nucleic acid molecule of the invention is at least 15, 20, 25 or 30 nucleotides in length. Preferably, it hybridizes under stringent conditions to a nucleic acid molecule comprising a nucleotide sequence of the nucleic acid molecule of the present invention or used in the process of the present invention, e.g. comprising a sequence as indicated in Table I, columns 5 or 7, lines 173, 262 to 274 and 628 to 632. The nucleic acid molecule is preferably at least 20, 30, 50, 100, 250 or more nucleotides in length.

**[0162.0.0.21]** for the disclosure of this paragraph see paragraph [0162.0.0.0] above.

**[0163.0.21.21]** Preferably, a nucleic acid molecule of the invention that hybridizes under stringent conditions to a sequence as indicated in Table I, columns 5 or 7, lines 173, 262 to 274 and 628 to 632 corresponds to a naturally-occurring nucleic acid molecule of the invention. As used herein, a "naturally-occurring" nucleic acid molecule refers to an RNA or DNA molecule having a nucleotide sequence that occurs in nature (e.g., encodes a natural protein). Preferably, the nucleic acid molecule encodes a natural protein having above-mentioned activity, e.g. conferring the increase of the amount of the respective fine chemical in an organism or a part thereof, e.g. a tissue, a cell, or a compartment of a cell, after increasing the expression or activity thereof or the activity of a protein of the invention or used in the process of the invention.

**[0164.0.0.21]** for the disclosure of this paragraph see paragraph [0164.0.0.0] above.

**[0165.0.21.21]** For example, nucleotide substitutions leading to amino acid substitutions at "non-essential" amino acid residues can be made in a sequence of the nucleic acid molecule of the invention or used in the process of the invention, e.g. as indicated in Table I, columns 5 or 7, lines 173, 262 to 274 and 628 to 632, resp.

**[0166.0.0.21] and [0167.0.0.21]** for the disclosure of the paragraphs [0166.0.0.21] and [0167.0.0.21] see paragraphs [0166.0.0.0] and [0167.0.0.0] above.

**[0168.0.21.21]** Accordingly, the invention relates to nucleic acid molecules encoding a polypeptide having above-mentioned activity, e.g. conferring an increase in the respective fine chemical in organisms or parts thereof that contain changes in amino acid residues that are not essential for said activity. Such polypeptides differ in amino acid sequence from a sequence contained in a sequence as indicated in Table II, columns 5 or 7, lines 173, 262 to 274 and 628 to 632, resp., yet retain said activity described herein. The nucleic acid molecule can comprise a nucleotide sequence encoding a polypeptide, wherein the polypeptide comprises an amino acid sequence at least about 50% identical to an amino acid sequence as indicated in Table II, columns 5 or 7, lines 173, 262 to 274 and 628 to 632, resp., and is capable of participation in the increase of production of the respective fine chemical after increasing its activity, e.g. its expression. Preferably, the protein encoded by the nucleic acid molecule is at least about 60% identical to a sequence as indicated in Table II, columns 5 or 7, lines 173, 262 to 274 and 628 to 632, resp., more preferably at least about 70% identical to one of the sequences as indicated in Table II, columns 5 or 7, lines 173, 262 to 274 and 628 to 632, resp., even more preferably at least about 80%, 90%, 95% homologous to a sequence as indicated in Table II, columns 5 or 7, lines 173, 262 to 274 and 628 to 632, resp., and most preferably at least about 96%, 97%, 98%, or 99% identical to the sequence as indicated in Table II, columns 5 or 7, lines 173, 262 to 274 and 628 to 632.
Accordingly, the invention relates to nucleic acid molecules encoding a polypeptide having above-mentioned activity, e.g. conferring an increase in the the respective fine chemical in an organisms or parts thereof that contain changes in amino acid residues that are not essential for said activity. Such polypeptides differ in amino acid sequence from a sequence contained in a sequence as indicated in Table II, columns 5 or 7, lines 173, 262 to 274 and 628 to 632, preferably of Table II B, column 7, lines 173, 262 to 274 and 628 to 632 yet retain said activity described herein. The nucleic acid molecule can comprise a nucleotide sequence encoding a polypeptide, wherein the polypeptide comprises an amino acid sequence at least about 50% identical to an amino acid sequence as indicated in Table II, columns 5 or 7, lines 173, 262 to 274 and 628 to 632, preferably of Table II B, column 7, lines 173, 262 to 274 and 628 to 632 and is capable of participation in the increase of production of the respective fine chemical after increasing its activity, e.g. its expression. Preferably, the protein encoded by the nucleic acid molecule is at least about 60% identical to a sequence as indicated in Table II, columns 5 or 7, lines 173, 262 to 274 and 628 to 632, preferably of Table II B, column 7, lines 173, 262 to 274 and 628 to 632, more preferably at least about 70% identical to one of the sequences as indicated in Table II, columns 5 or 7, lines 173, 262 to 274 and 628 to 632, preferably of Table II B, column 7, lines 173, 262 to 274 and 628 to 632, even more preferably at least about 80%, 90%, or 95% homologous to a sequence as indicated in Table II, columns 5 or 7, lines 173, 262 to 274 and 628 to 632, preferably of Table II B, column 7, lines 173, 262 to 274 and 628 to 632, and most preferably at least about 96%, 97%, 98%, or 99% identical to the sequence as indicated in Table II, columns 5 or 7, lines 173, 262 to 274 and 628 to 632, preferably of Table II B, column 7, lines 173, 262 to 274 and 628 to 632.

**[0169.0.0.21] to [0172.0.0.21]** for the disclosure of the paragraphs [0169.0.0.21] to [0172.0.0.21] see paragraphs [0169.0.0.0] to [0172.0.0.0] above.

**[0173.0.21.21]** For example a sequence, which has 80% homology with sequence SEQ ID NO: 27329 at the nucleic acid level is understood as meaning a sequence which, upon comparison with the sequence SEQ ID NO: 27329 by the above Gap program algorithm with the above parameter set, has a 80% homology.

**[0174.0.0.21]:** for the disclosure of this paragraph see paragraph [0174.0.0.0] above.

**[0175.0.21.21]** For example a sequence which has a 80% homology with sequence SEQ ID NO: 27330 at the protein level is understood as meaning a sequence which, upon comparison with the sequence SEQ ID NO: 27330 by the above program algorithm with the above parameter set, has a 80% homology.

**[0176.0.21.21]** Functional equivalents derived from one of the polypeptides as indicated in Table II, columns 5 or 7, lines 173, 262 to 274 and 628 to 632, resp., according to the invention by substitution, insertion or deletion have at least 30%, 35%, 40%, 45% or 50%, preferably at least 55%, 60%, 65% or 70% by preference at least 80%, especially preferably at least 85% or 90%, 91%, 92%, 93% or 94%, very especially preferably at least 95%, 97%, 98% or 99% homology with one of the polypeptides as indicated in Table II, columns 5 or 7, lines 173, 262 to 274 and 628 to 632, resp., according to the invention and are distinguished by essentially the same properties as a polypeptide as indicated in Table II, columns 5 or 7, lines 173, 262 to 274 and 628 to 632, resp.

**[0177.0.21.21]** Functional equivalents derived from a nucleic acid sequence as indicated in Table I, columns 5 or 7, lines 173, 262 to 274 and 628 to 632, preferably of Table I B, lines 173, 262 to 274 and 628 to 632 resp., according to the invention by substitution, insertion or deletion have at least 30%, 35%, 40%, 45% or 50%, preferably at least 55%, 60%, 65% or 70% by preference at least 80%, especially preferably at least 85% or 90%, 91%, 92%, 93% or 94%, very especially preferably at least 95%, 97%, 98% or 99% homology with one of the polypeptides as indicated in Table II, columns 5 or 7, lines 173, 262 to 274 and 628 to 632, resp., according to the invention and encode polypeptides having essentially the same properties as a polypeptide as indicated in Table II, columns 5 or 7, lines 173, 262 to 274 and 628 to 632, preferably of Table II B, lines 173, 262 to 274 and 628 to 632 resp.

**[0178.0.0.21]** for the disclosure of this paragraph see [0178.0.0.0] above.

**[0179.0.21.21]** A nucleic acid molecule encoding a homologue to a protein sequence as indicated in Table II, columns 5 or 7, lines 173, 262 to 274 and 628 to 632, preferably as indicated in Table II B, lines 173, 262 to 274 and 628 to 632, resp., can be created by introducing one or more nucleotide substitutions, additions or deletions into a nucleotide sequence of the nucleic acid molecule of the present invention, in particular as indicated in Table I, columns 5 or 7, lines 173, 262 to 274 and 628 to 632, preferably as indicated in Table I B, lines 173, 262 to 274 and 628 to 632 resp., such that one or more amino acid substitutions, additions or deletions are introduced into the encoded protein. Mutations can be introduced into the encoding sequences as indicated in Table I, columns 5 or 7, lines 173, 262 to 274 and 628 to 632, preferably as indicated in Table I B, lines 173, 262 to 274 and 628 to 632, resp., by standard techniques, such as site-directed mutagenesis and PCR-mediated mutagenesis.

**[0180.0.0.21] to [0183.0.0.21]** for the disclosure of the paragraphs [0180.0.0.21] to [0183.0.0.21] see paragraphs [0180.0.0.0] to [0183.0.0.0] above.

**[0184.0.21.21]** Homologues of the nucleic acid sequences used, with a sequence as indicated in Table I, columns 5 or 7, lines 173, 262 to 274 and 628 to 632, preferably as indicated in Table I B, lines 173, 262 to 274 and 628 to 632, resp., or of the nucleic acid sequences derived from a sequences as indicated in Table II, columns 5 or 7, lines 173, 262 to 274 and 628 to 632, preferably as indicated in Table II B, lines 173, 262 to 274 and 628 to 632, resp., comprise also allelic variants with at least approximately 30%, 35%, 40% or 45% homology, by preference at least approximately 50%, 60% or 70%, more preferably at least approximately 90%, 91%, 92%, 93%, 94% or 95% and even more preferably at least approximately 96%, 97%, 98%, 99% or more homology with one of the nucleotide sequences shown or the abovementioned derived nucleic acid sequences or their homologues, derivatives or analogues or parts of these. Allelic variants encompass in particular functional variants which can be obtained by deletion, insertion or substitution of nucleotides from the sequences shown, preferably from a sequence as indicated in Table I, columns 5 or 7, lines 173, 262 to 274 and 628 to 632, preferably as indicated in Table I B, lines 173, 262 to 274 and 628 to 632, resp., or from the derived nucleic acid sequences, the intention being, however, that the enzyme activity or the biological activity of the resulting proteins synthesized is advantageously retained or increased.

**[0185.0.21.21]** In one embodiment of the present invention, the nucleic acid molecule of the invention or used in the process of the invention comprises one or more sequences as indicated in Table I, columns 5 or 7, lines 173, 262 to 274 and 628 to 632, preferably as indicated in Table I B, lines 173, 262 to 274 and 628 to 632, resp. In one embodiment, it is preferred that the nucleic acid molecule comprises as little as possible other nucleotides not shown in any one of sequences as indicated in Table I, columns 5 or 7, lines 173, 262 to 274 and 628 to 632, preferably as indicated in Table I B, lines 173, 262 to 274 and 628 to 632, resp. In one embodiment, the nucleic acid molecule comprises less than 500, 400, 300, 200, 100, 90, 80, 70, 60, 50 or 40 further nucleotides. In a further embodiment, the nucleic acid molecule comprises less than 30, 20 or 10 further nucleotides. In one embodiment, a nucleic acid molecule used in the process of the invention is identical to a sequence as indicated in Table I, columns 5 or 7, lines 173, 262 to 274 and 628 to 632, preferably as indicated in Table I B, lines 173, 262 to 274 and 628 to 632, resp.

**[0186.0.21.21]** Also preferred is that one or more nucleic acid molecule(s) used in the process of the invention encodes a polypeptide comprising a sequence as indicated in Table II, columns 5 or 7, lines 173, 262 to 274 and 628 to 632, preferably as indicated in Table II B, lines 173, 262 to 274 and 628 to 632, resp. In one embodiment, the nucleic acid molecule encodes less than 150, 130, 100, 80, 60, 50, 40 or 30 further amino acids. In a further embodiment, the encoded polypeptide comprises less than 20, 15, 10, 9, 8, 7, 6 or 5 further amino acids. In one embodiment, the encoded polypeptide used in the process of the invention is identical to the sequences as indicated in Table II, columns 5 or 7, lines 173, 262 to 274 and 628 to 632, preferably as indicated in Table II B, lines 173, 262 to 274 and 628 to 632, resp.

**[0187.0.21.21]** In one embodiment, a nucleic acid molecule of the invention or used in the process encodes a polypeptide comprising the sequence as indicated in Table II, columns 5 or 7, lines 173, 262 to 274 and 628 to 632, preferably as indicated in Table II B, lines 173, 262 to 274 and 628 to 632, resp., comprises less than 100 further nucleotides. In a further embodiment, said nucleic acid molecule comprises less than 30 further nucleotides. In one embodiment, the nucleic acid molecule used in the process is identical to a coding sequence encoding a sequences as indicated in Table II, columns 5 or 7, lines 173, 262 to 274 and 628 to 632, preferably as indicated in Table II B, lines 173, 262 to 274 and 628 to 632, resp.

**[0188.0.21.21]** Polypeptides (= proteins), which still have the essential enzymatic activity of the polypeptide of the present invention conferring an increase of the respective fine chemical i.e. whose activity is essentially not reduced, are polypeptides with at least 10% or 20%, by preference 30% or 40%, especially preferably 50% or 60%, very especially preferably 80% or 90 or more of the wild type biological activity or enzyme activity. Advantageously, the activity is essentially not reduced in comparison with the activity of a polypeptide as indicated in Table II, columns 5 or 7, lines 173, 262 to 274 and 628 to 632, resp., and is expressed under identical conditions.
In one embodiment, the polypeptide of the invention is a homolog consisting of or comprising the sequence as indicated in Table II B, columns 7, lines 173, 262 to 274 and 628 to 632.

**[0189.0.21.21]** Homologues of a sequences as indicated in Table I, columns 5 or 7, lines 173, 262 to 274 and 628 to 632, resp., or of derived sequences as indicated in Table II, columns 5 or 7, lines 173, 262 to 274 and 628 to 632, resp., also mean truncated sequences, cDNA, single-stranded DNA or RNA of the coding and noncoding DNA sequence. Homologues of said sequences are also understood as meaning derivatives, which comprise noncoding regions such as, for example, UTRs, terminators, enhancers or promoter variants. The promoters upstream of the nucleotide sequences stated can be modified by one or more nucleotide substitution(s), insertion(s) and/or deletion(s) without, however, interfering with the functionality or activity either of the promoters, the open reading frame (=ORF) or with the 3'-regulatory region such as terminators or other 3'regulatory regions, which are far away from the ORF. It is furthermore possible that the activity of the promoters is increased by modification of their sequence, or that they are replaced completely by more active promoters, even promoters from heterologous organisms. Appropriate promoters are known to the person skilled in the art and are mentioned herein below.

**[0190.0.0.21]:** for the disclosure of this paragraph see [0190.0.0.0] above.

**[0191.0.21.21]** In one embodiment, the organisms or part thereof produce according to the herein mentioned process of the invention an increased level of free and/or bound the respective fine chemical compared to said control or selected organisms or parts thereof.

**[0191.1.0.21]:** for the disclosure of this paragraph see [0191.1.0.0] above.

**[0192.0.0.21] to [0203.0.0.21]** for the disclosure of the paragraphs [0192.0.0.21] to [0203.0.0.21] see paragraphs [0192.0.0.0] to [0203.0.0.0] above.

**[0204.0.21.21]** Accordingly, in one embodiment, the invention relates to a nucleic acid molecule, which comprises a nucleic acid molecule selected from the group consisting of:
a) nucleic acid molecule encoding, preferably at least the mature form, of a polypeptide as indicated in Table II, columns 5 or 7, lines 173, 262 to 274 and 628 to 632, preferably as indicated in Table II B, lines 173, 262 to 274 and 628 to 632, resp.; or a fragment thereof conferring an increase in the amount of the respective fine chemical, i.e. glycerol-3-phosphate (line 173 and lines 630 to 632) or glycerol (lines 262 to 274 and lines 628 and 629), and accordingly glycerol as a component of total lipid (lines 265, 266, 267, 269, 271, 272 and 263), resp., in an organism or a part thereof;
b) nucleic acid molecule comprising, preferably at least the mature form, of a nucleic acid molecule as indicated in Table I, columns 5 or 7, lines 173, 262 to 274 and 628 to 632, preferably as indicated in Table I B, lines 173, 262 to 274 and 628 to 632, resp., or a fragment thereof conferring an increase in the amount of the respective fine chemical in an organism or a part thereof;
c) nucleic acid molecule whose sequence can be deduced from a polypeptide sequence encoded by a nucleic acid molecule of (a) or (b) as result of the degeneracy of the genetic code and conferring an increase in the amount of the respective fine chemical in an organism or a part thereof;
d) nucleic acid molecule encoding a polypeptide whose sequence has at least 50% identity with the amino acid sequence of the polypeptide encoded by the nucleic acid molecule of (a) to (c) and conferring an increase in the amount of the respective fine chemical in an organism or a part thereof;
e) nucleic acid molecule which hybridizes with a nucleic acid molecule of (a) to (c) under stringent hybridisation conditions and conferring an increase in the amount of the respective fine chemical in an organism or a part thereof;
f) nucleic acid molecule encoding a polypeptide, the polypeptide being derived by substituting, deleting and/or adding one or more amino acids of the amino acid sequence of the polypeptide encoded by the nucleic acid molecules (a) to (d), preferably to (a) to (c), and conferring an increase in the amount of the respective fine chemical in an organism or a part thereof;
g) nucleic acid molecule encoding a fragment or an epitope of a polypeptide which is encoded by one of the nucleic acid molecules of (a) to (e), preferably to (a) to (c) and conferring an increase in the amount of the respective fine chemical in an organism or a part thereof;
h) nucleic acid molecule comprising a nucleic acid molecule which is obtained by amplifying a cDNA library or a genomic library using primers or primer pairs as indicated in Table III, columns 5 or 7, lines 173, 262 to 274 and 628 to 632 and conferring an increase in the amount of the respective fine chemical, i.e. glycerol-3-phosphate (line173 and lines 630 to 632) or glycerol (lines 262 to 274 and lines 628 and 629) and accordingly glycerol as a component of total lipid (lines 265, 266, 267, 269, 271, 272 and 263) resp., in an organism or a part thereof;
i) nucleic acid molecule encoding a polypeptide which is isolated, e.g. from a expression library, with the aid of monoclonal antibodies against a polypeptide encoded by one of the nucleic acid molecules of (a) to (g), preferably to (a) to (c) and conferring an increase in the amount of the respective fine chemical in an organism or a part thereof;
j) nucleic acid molecule which encodes a polypeptide comprising a consensus sequence as indicated in Table IV, column 7, lines 173, 262 to 267, 269 and 271 to 274 and lines 628 to 632 and conferring an increase in the amount of the respective fine chemical, i.e. glycerol-3-phosphate (line 173 and lines 630 to 632) or glycerol (lines 262 to 274 and lines 628 and 629) and accordingly glycerol as a component of total lipid (lines 265, 266, 267, 269, 271, 272 and 263) resp., in an organism or a part thereof;
k) nucleic acid molecule encoding the amino acid sequence of a polypeptide encoding a domaine of a polypeptide as indicated in Table II, columns 5 or 7, lines 173, 262 to 274 and 628 to 632, preferably as indicated in Table II B, lines 173, 262 to 274 and 628 to 632, resp., and conferring an increase in the amount of the respective fine chemical, i.e. glycerol-3-phosphate (line 173 and lines 630 to 632) or glycerol (lines 262 to 274 and lines 628 and 629) and accordingly glycerol as a component of total lipid (lines 265, 266, 267, 269, 271, 272 and 263) resp., in an organism or a part thereof; and
l) nucleic acid molecule which is obtainable by screening a suitable nucleic acid library under stringent hybridization conditions with a probe comprising one of the sequences of the nucleic acid molecule of (a) to (k) or with a fragment of at least 15 nt, preferably 20 nt, 30 nt, 50 nt, 100 nt, 200 nt or 500 nt of the nucleic acid molecule characterized in (a) to (h) or of a nucleic acid molecule as indicated in Table I, columns 5 or 7, lines 173, 262 to 274 and 628 to 632, preferably as indicated in Table I B, lines 173, 262 to 274 and 628 to 632, resp., or a nucleic acid molecule encoding, preferably at least the mature form of, a polypeptide as indicated in Table II, columns 5 or 7, lines 173, 262 to 274 and 628 to 632, preferably as indicated in Table II B, lines 173, 262 to 274 and 628 to 632, resp., and conferring an increase in the amount of the respective fine chemical in an organism or a part thereof; or which encompasses a sequence which is complementary thereto;
whereby, preferably, the nucleic acid molecule according to (a) to (I) distinguishes over a sequence as indicated in Table IA or IB, columns 5 or 7, lines 173, 262 to 274 and 628 to 632, resp., by one or more nucleotides. In one embodiment, the nucleic acid molecule of the invention does not consist of the sequence as indicated in Table IA or IB, columns 5 or 7, lines 173, 262 to 274 and 628 to 632, resp. In another embodiment, the nucleic acid molecule of the present invention is at least 30 % identical and less than 100%, 99,999%, 99,99%, 99,9% or 99% identical to a sequence as indicated in Table IA or IB, columns 5 or 7, lines 173, 262 to 274 and 628 to 632, resp. In a further embodiment the nucleic acid molecule does not encode a polypeptide sequence as indicated in Table IIA or IIB, columns 5 or 7, lines 173, 262 to 274 and 628 to 632, resp. Accordingly, in one embodiment, the nucleic acid molecule of the present invention encodes in one embodiment a polypeptide which differs at least in one or more amino acids from a polypeptide indicated in Table IIA or IIB, columns 5 or 7, lines 173, 262 to 274 and 628 to 632 does not encode a protein of a sequence as indicated in Table IIA or IIB, columns 5 or 7, lines 173, 262 to 274 and 628 to 632. Accordingly, in one embodiment, the protein encoded by a sequences of a nucleic acid accoriding to (a) to (I) does not consist of a sequence as indicated in Table IIA or IIB, columns 5 or 7, lines 173, 262 to 274 and 628 to 632. In a further embodiment, the protein of the present invention is at least 30 % identical to a protein sequence indicated in Table IIA or IIB, columns 5 or 7, lines 173, 262 to 274 and 628 to 632 and less than 100%, preferably less than 99,999%, 99,99% or 99,9%, more preferably less than 99%, 985, 97%, 96% or 95% identical to a sequence as indicated in Table IIA or IIB, columns 5 or 7, lines 173, 262 to 274 and 628 to 632.

**[0205.0.0.21] to [0206.0.0.21]: see [0205.0.0.0] to [0206.0.0.0]**

**[0207.0.21.21]** As described herein, the nucleic acid construct can also comprise further genes, which are to be introduced into the organisms or cells. It is possible and advantageous to introduce into, and express in, the host organisms regulatory genes such as genes for inductors, repressors or enzymes, which, owing to their enzymatic activity, engage in the regulation of one or more genes of a biosynthetic pathway. These genes can be of heterologous or homologous origin. Moreover, further biosynthesis genes may advantageously be present, or else these genes may be located on one or more further nucleic acid constructs. Genes, which are advantageously employed as biosynthesis genes, are genes of the glutamic acid metabolism, the phosphoenolpyruvate metabolism, the amino acid metabolism, of glycolysis, of the tricarboxylic acid metabolism or their combinations. As described herein, regulator sequences or factors can have a positive effect on preferably the gene expression of the genes introduced, thus increasing it. Thus, an enhancement of the regulator elements may advantageously take place at the transcriptional level by using strong transcription signals such as promoters and/or enhancers. In addition, however, an enhancement of translation is also possible, for example by increasing mRNA stability or by inserting a translation enhancer sequence.

**[0208.0.0.21] to [0226.0.0.21]** for the disclosure of the paragraphs [0208.0.0.21] to [0226.0.0.21] see paragraphs [0208.0.0.0] to [0226.0.0.0] above.

**[0227.0.21.21]** The abovementioned nucleic acid molecules can be cloned into the nucleic acid constructs or vectors according to the invention in combination together with further genes, or else different genes are introduced by transforming several nucleic acid constructs or vectors (including plasmids) into a host cell, advantageously into a plant cell or a microorganisms.
In addition to a sequence indicated in Table I, columns 5 or 7, lines 173, 262 to 274 and 628 to 632 or its derivatives, it is advantageous to express and/or mutate further genes in the organisms. Especially advantageously, additionally at least one further gene of the acetyl-CoA or malonyl-CoA metabolic pathway or a polypeptide having a very long chain fatty acid acyl (VLCFA) CoA synthase activity, is expressed in the organisms such as plants or microorganisms. It is also possible that the regulation of the natural genes has been modified advantageously so that the gene and/or its gene product is no longer subject to the regulatory mechanisms which exist in the organisms. This leads to an increased synthesis of the fine chemicals desired since, for example, feedback regulations no longer exist to the same extent or not at all. In addition it might be advantageously to combine one or more of the sequences indicated in Table I, columns 5 or 7, lines 173, 262 to 274 and 628 to 632, resp., with genes which generally support or enhance to growth or yield of the target organismen, for example genes which lead to faster growth rate of microorganisms or genes which produces stress-, pathogen, or herbicide resistant plants.

**[0228.0.21.21]** In a further embodiment of the process of the invention, therefore, organisms are grown, in which there is simultaneous overexpression of at least one nucleic acid or one of the genes which code for proteins involved in the glycerol-3-phosphate and/or glycerol metabolism, in particular in synthesis of glycerol-3-phosphate or glycerol.

**[0229.0.21.21]** Further advantageous nucleic acid sequences which can be expressed in combination with the sequences indicated in Table I, columns 5 or 7, lines 173, 262 to 274 and 628 to 632 used in the process and/or the abovementioned biosynthesis genes are the sequences encoding further genes of the fatty acid pathway, such as acetyl-CoA or malonyl-CoA or a polypeptide having a very long chain fatty acid acyl (VLCFA) CoA synthase activity. These genes can lead to an increased synthesis of the VLCFAs.

**[0230.0.0.21]** for the disclosure of this paragraph see paragraph [0230.0.0.0] above.

**[0231.0.21.21]** In a further advantageous embodiment of the process of the invention, the organisms used in the process are those in which simultaneously a glycerol and/or glycerol-3-phosphate and/or a lipid degrading protein is attenuated, in particular by reducing the rate of expression of the corresponding gene. A person skilled in the art knows for example, that the inhibition or repression of a glycerol and/or glycerol-3-phosphate or lipid degrading enzyme will result in an increased accumulation of glycerol and/or glycerol-3-phosphate and/or total lipids in plants.

**[0232.0.0.21] to [0276.0.0.21]** for the disclosure of the paragraphs [0232.0.0.21] to [0276.0.0.21] see paragraphs [0232.0.0.0] to [0276.0.0.0] above.

**[0277.0.21.21]** The respective fine chemical produced can be isolated from the organism by methods with which the skilled worker are familiar, for example via extraction, salt precipitation, and/or different chromatography methods. The process according to the invention can be conducted batchwise, semibatchwise or continuously.

**[0278.0.0.21] to [0282.0.0.21]** for the disclosure of the paragraphs [0278.0.0.21] to [0282.0.0.21] see paragraphs [0278.0.0.0] to [0282.0.0.0] above.

**[0283.0.21.21]** Moreover, native polypeptide conferring the increase of the respective fine chemical in an organism or part thereof can be isolated from cells, for example using the antibody of the present invention as described below, e.g. an antibody against a protein as indicated in Table II, column 3, lines 173, 262 to 274 and 628 to 632, resp., or an antibody against a polypeptide as indicated in Table II, columns 5 or 7, lines 173, 262 to 274 and 628 to 632, resp., which can be produced by standard techniques utilizing the polypeptid of the present invention or fragment thereof. Preferred are monoclonal antibodies specifically binding to polypeptides as indicated in Table II, columns 5 or 7, lines 173, 262 to 274 and 628 to 632, more preferred specifically binding to polypeptides as indicated in Table II, column 5, lines 173, 262 to 274 and 628 to 632.

**[0284.0.0.21]** for the disclosure of this paragraph see [0284.0.0.0] above.

**[0285.0.21.21]** In one embodiment, the present invention relates to a polypeptide having a sequence as indicated in Table II, columns 5 or 7, lines 173, 262 to 274 and 628 to 632, resp., or as coded by a nucleic acid molecule as indicated in Table I, columns 5 or 7, lines 173, 262 to 274 and 628 to 632, resp., or functional homologues thereof.

**[0286.0.21.21]** In one advantageous embodiment, in the method of the present invention the activity of a polypeptide is increased which comprises or consists of a consensus sequence as indicated in Table IV, column 7, lines 173, 262 to 267, 269 and 271 to 274 and lines 628 to 632 and in one another embodiment, the present invention relates to a polypeptide comprising or consisting of a consensus sequence as indicated in Table IV, column 7, lines 173, 262 to 267, 269 and 271 to 274 and lines 628 to 632 whereby 20 or less, preferably 15 or 10, preferably 9, 8, 7, or 6, more preferred 5 or 4, even more preferred 3, even more preferred 2, even more preferred 1, most preferred 0 of the amino acids positions indicated can be replaced by any amino acid or, in an further embodiment, can be replaced and/or absent. In one embodiment, the present invention relates to the method of the present invention comprising a polypeptide or to a polypeptide comprising more than one consensus sequences (of an individual line) as indicated in Table IV, column 7, lines 173, 262 to 267, 269 and 271 to 274 and lines 628 to 632.

**[0287.0.0.21] to [0290.0.0.21]** for the disclosure of the paragraphs [0287.0.0.21] to [0290.0.0.21] see paragraphs [0287.0.0.0] to [0290.0.0.0] above.

**[0291.0.21.21]** In one advantageous embodiment, the method of the present invention comprises the increasing of a polypeptide comprising or consisting of plant or microorganism specific consensus sequences.
In one embodiment, said polypeptide of the invention distinguishes over a sequence as indicated in Table IIA or IIB, columns 5 or 7, lines 173, 262 to 274 and 628 to 632, resp., by one or more amino acids. In one embodiment, polypeptide distinguishes from a sequence as indicated in Table IIA or IIB, columns 5 or 7, lines 173, 262 to 274 and 628 to 632, resp., by more than 5, 6, 7, 8 or 9 amino acids, preferably by more than 10, 15, 20, 25 or 30 amino acids, even more preferred are more than 40, 50, or 60 amino acids and, preferably, the sequence of the polypeptide of the invention distinguishes from a sequence as indicated in Table IIA or IIB, columns 5 or 7, lines 173, 262 to 274 and 628 to 632, resp., by not more than 80% or 70% of the amino acids, preferably not more than 60% or 50%, more preferred not more than 40% or 30%, even more preferred not more than 20% or 10%. In an other embodiment, said polypeptide of the invention does not consist of a sequence as indicated in Table IIA or IIB, columns 5 or 7, lines 173, 262 to 274 and 628 to 632.

**[0292.0.0.21]** for the disclosure of this paragraph see [0292.0.0.0] above.

**[0293.0.21.21]** In one embodiment, the invention relates to a polypeptide conferring an increase in the fine chemical in an organism or part being encoded by the nucleic acid molecule of the invention or by a nucleic acid molecule used in the process of the invention. In one embodiment, the polypeptide of the invention has a sequence which distinguishes from a sequence as indicated in Table IIA or IIB, columns 5 or 7, lines 173, 262 to 274 and 628 to 632, resp., by one or more amino acids. In an other embodiment, said polypeptide of the invention does not consist of the sequence as indicated in Table IIA or IIB, columns 5 or 7, lines 173, 262 to 274 and 628 to 632, resp. In a further embodiment, said polypeptide of the present invention is less than 100%, 99,999%, 99,99%, 99,9% or 99% identical. In one embodiment, said polypeptide does not consist of the sequence encoded by a nucleic acid molecules as indicated in Table IA or IB, columns 5 or 7, lines 173, 262 to 274 and 628 to 632, resp.

**[0294.0.21.21]** In one embodiment, the present invention relates to a polypeptide having an activity of a protein as indicated in Table II, column 3, lines 173, 262 to 274 and 628 to 632, resp., which distinguishes over a sequence as indicated in Table IIA or IIB, columns 5 or 7, lines 173, 262 to 274 and 628 to 632, resp., by one or more amino acids, preferably by more than 5, 6, 7, 8 or 9 amino acids, preferably by more than 10, 15, 20, 25 or 30 amino acids, even more preferred are more than 40, 50, or 60 amino acids but even more preferred by less than 70% of the amino acids, more preferred by less than 50%, even more preferred my less than 30% or 25%, more preferred are 20% or 15%, even more preferred are less than 10%.

**[0295.0.0.21] to [0297.0.0.21]** for the disclosure of the paragraphs [0295.0.0.21] to [0297.0.0.21] see paragraphs [0295.0.0.0] to [0297.0.0.0] above.

**[00297.1.21.21]** Non polypeptide of the invention-chemicals are e.g. polypeptides having not the activity of a polypeptide indicated in Table II, columns 3, 5 or 7, lines 173, 262 to 274 and 628 to 632, resp.

**[0298.0.21.21]** A polypeptide of the invention can participate in the process of the present invention. The polypeptide or a portion thereof comprises preferably an amino acid sequence which is sufficiently homologous to an amino acid sequence as indicated in Table II, columns 5 or 7, lines 173, 262 to 274 and 628 to 632, resp. The portion of the protein is preferably a biologically active portion as described herein. Preferably, the polypeptide used in the process of the invention has an amino acid sequence identical to a sequence as indicated in Table II, columns 5 or 7, lines 173, 262 to 274 and 628 to 632, resp.

**[0299.0.21.21]** Further, the polypeptide can have an amino acid sequence which is encoded by a nucleotide sequence which hybridizes, preferably hybridizes under stringent conditions as described above, to a nucleotide sequence of the nucleic acid molecule of the present invention. Accordingly, the polypeptide has an amino acid sequence which is encoded by a nucleotide sequence that is at least about 35%, 40%, 45%, 50%, 55%, 60%, 65% or 70%, preferably at least about 75%, 80%, 85% or 90, and more preferably at least about 91%, 92%, 93%, 94% or 95%, and even more preferably at least about 96%, 97%, 98%, 99% or more homologous to one of the nucleotide sequence as indicated in Table I, columns 5 or 7, lines 173, 262 to 274 and 628 to 632, resp. The preferred polypeptide of the present invention preferably possesses at least one of the activities according to the invention and described herein. A preferred polypeptide of the present invention includes an amino acid sequence encoded by a nucleotide sequence which hybridizes, preferably hybridizes under stringent conditions, to a nucleotide sequence as indicated in Table I, columns 5 or 7, lines 173, 262 to 274 and 628 to 632, resp., or which is homologous thereto, as defined above.

**[0300.0.21.21]** Accordingly the polypeptide of the present invention can vary from a sequence as indicated in Table II, columns 5 or 7, lines 173, 262 to 274 and 628 to 632, resp., in amino acid sequence due to natural variation or mutagenesis, as described in detail herein. Accordingly, the polypeptide comprise an amino acid sequence which is at least about 35%, 40%, 45%, 50%, 55%, 60%, 65% or 70%, preferably at least about 75%, 80%, 85% or 90, and more preferably at least about 91 %, 92%, 93%, 94% or 95%, and most preferably at least about 96%, 97%, 98%, 99% or more homologous to an entire amino acid sequence of as indicated in Table IIA or IIB, columns 5 or 7, lines 173, 262 to 274 and 628 to 632, resp.

**[0301.0.0.21]** for the disclosure of this paragraph see [0301.0.0.0] above.

**[0302.0.21.21]** Biologically active portions of an polypeptide of the present invention include peptides comprising amino acid sequences derived from the amino acid sequence of the polypeptide of the present invention or used in the process of the present invention, e.g., an amino acid sequence as indicated in Table II, columns 5 or 7, lines 173, 262 to 274 and 628 to 632, resp., or the amino acid sequence of a protein homologous thereto, which include fewer amino acids than a full length polypeptide of the present invention or used in the process of the present invention or the full length protein which is homologous to an polypeptide of the present invention or used in the process of the present invention depicted herein, and exhibit at least one activity of polypeptide of the present invention or used in the process of the present invention.

**[0303.0.0.21]** for the disclosure of this paragraph see [0303.0.0.0] above.

**[0304.0.21.21]** Manipulation of the nucleic acid molecule of the invention may result in the production of a protein having essentially the activity of the polypeptides as indicated in Table II, column 3, lines 173, 262 to 274 and 628 to 632 but having differences in the sequence from said wild-type protein. These proteins may be improved in efficiency or activity, may be present in greater numbers in the cell than is usual, or may be decreased in efficiency or activity in relation to the wild type protein.

**[0305.0.0.21] to [0308.0.0.21]** for the disclosure of the paragraphs [0305.0.0.21] to [0308.0.0.21] see paragraphs [0305.0.0.0] to [0308.0.0.0] above.

**[0306.1.0.21] %**

**[0309.0.21.21]** In one embodiment, a reference to a protein (= polypeptide) of the invention or as indicated in Table II, columns 5 or 7, lines 173, 262 to 274 and 628 to 632, resp., refers to a polypeptide having an amino acid sequence corresponding to the polypeptide of the invention or used in the process of the invention, whereas an "other polypeptide" not being indicated in Table II, columns 5 or 7, lines 173, 262 to 274 and 628 to 632, resp., refers to a polypeptide having an amino acid sequence corresponding to a protein which is not substantially homologous to a polypeptide of the invention, preferably which is not substantially homologous to a polypeptide as indicated in Table II, columns 5 or 7, lines 173, 262 to 274 and 628 to 632, resp., e.g., a protein which does not confer the activity described herein or annotated or known for as indicated in Table II, column 3, lines 173, 262 to 274 and 628 to 632, resp., and which is derived from the same or a different organism. In one embodiment, an "other polypeptide" not being indicated in Table II, columns 5 or 7, lines 173, 262 to 274 and 628 to 632, resp., does not confer an increase of the respective fine chemical in an organism or part thereof.

**[0310.0.0.21] to [0334.0.0.21]** for the disclosure of the paragraphs [0310.0.0.21] to [0334.0.0.21] see paragraphs [0310.0.0.0] to [0334.0.0.0] above.

**[0335.0.21.21]** The dsRNAi method has proved to be particularly effective and advantageous for reducing the expression of a nucleic acid sequences as indicated in Table I, columns 5 or 7, lines 173, 262 to 274 and 628 to 632, resp., and/or homologs thereof. As described inter alia in WO 99/32619, dsRNAi approaches are clearly superior to traditional antisense approaches. The invention therefore furthermore relates to double-stranded RNA molecules (dsRNA molecules) which, when introduced into an organism, advantageously into a plant (or a cell, tissue, organ or seed derived there from), bring about altered metabolic activity by the reduction in the expression of a nucleic acid sequences as indicated in Table I, columns 5 or 7, lines 173, 262 to 274 and 628 to 632, resp., and/or homologs thereof. In a double-stranded RNA molecule for reducing the expression of a protein encoded by a nucleic acid sequence as indicated in Table I, columns 5 or 7, lines 173, 262 to 274 and 628 to 632, resp., and/or homologs thereof, one of the two RNA strands is essentially identical to at least part of a nucleic acid sequence, and the respective other RNA strand is essentially identical to at least part of the complementary strand of a nucleic acid sequence.

**[0336.0.0.21] to [0342.0.0.21]** for the disclosure of the paragraphs [0336.0.0.21] to [0342.0.0.21] see paragraphs [0336.0.0.0] to [0342.0.0.0] above.

**[0343.0.21.21]** As has already been described, 100 % sequence identity between the dsRNA and a gene transcript of a nucleic acid sequence as indicated in Table I, columns 5 or 7, lines 173, 262 to 274 and 628 to 632, resp., or it's homolog is not necessarily required in order to bring about effective reduction in the expression. The advantage is, accordingly, that the method is tolerant with regard to sequence deviations as may be present as a consequence of genetic mutations, polymorphisms or evolutionary divergences. Thus, for example, using the dsRNA, which has been generated starting from a sequence as indicated in Table I, columns 5 or 7, lines 173, 262 to 274 and 628 to 632, resp., or homologs thereof of the one organism, may be used to suppress the corresponding expression in another organism.

**[0344.0.0.21] to [0361.0.0.21]** for the disclosure of the paragraphs [0344.0.0.21] to [0361.0.0.21] see paragraphs [0344.0.0.0] to [0361.0.0.0] above.

**[0362.0.21.21]** Accordingly the present invention relates to any cell transgenic for any nucleic acid characterized as part of the invention, e.g. conferring the increase of the respective fine chemical in a cell or an organism or a part thereof, e.g. the nucleic acid molecule of the invention, the nucleic acid construct of the invention, the antisense molecule of the invention, the vector of the invention or a nucleic acid molecule encoding the polypeptide of the invention, e.g. the polypeptide as indicated in Table II, columns 5 or 7, lines 173, 262 to 274 and 628 to 632, resp., e.g. encoding a polypeptide having protein activity, as indicated in Table II, columns 3, lines 173, 262 to 274 and 628 to 632, resp.. Due to the above-mentioned activity the respective fine chemical content in a cell or an organism is increased. For example, due to modulation or manipulation, the cellular activity of the polypeptide of the invention or nucleic acid molecule of the invention is increased, e.g. due to an increased expression or specific activity of the subject matters of the invention in a cell or an organism or a part thereof. Transgenic for a polypeptide having an activity of a polypeptide as indicated in Table II, columns 5 or 7, lines 173, 262 to 274 and 628 to 632, resp., means herein that due to modulation or manipulation of the genome, an activity as annotated for a polypeptide as indicated in Table II, column 3, lines 173, 262 to 274 and 628 to 632, e.g. having a sequence as indicated in Table II, columns 5 or 7, lines 173, 262 to 274 and 628 to 632, resp., is increased in a cell or an organism or a part thereof. Examples are described above in context with the process of the invention.

**[0363.0.0.21]** for the disclosure of this paragraph see paragraph [0363.0.0.0] above.

**[0364.0.21.21]** A naturally occurring expression cassette - for example the naturally occurring combination of a promoter of a gene encoding a polypeptide of the invention as indicated in Table II, column 3, lines 173, 262 to 274 and 628 to 632, resp. with the corresponding protein-encoding sequence as indicated in Table I, column 5, lines 173, 262 to 274 and 628 to 632, resp., becomes a transgenic expression cassette when it is modified by non-natural, synthetic "artificial" methods such as, for example, mutagenization. Such methods have been described (US 5,565,350; WO 00/15815; also see above).

**[0365.0.0.21] to [0373.0.0.21]** for the disclosure of the paragraphs [0365.0.0.21], to [0373.0.0.21] see paragraphs [0365.0.0.0] to [0373.0.0.0] above.

**[0374.0.21.21]** Transgenic plants comprising the respective fine chemical synthesized in the process according to the invention can be marketed directly without isolation of the compounds synthesized. In the process according to the invention, plants are understood as meaning all plant parts, plant organs such as leaf, stalk, root, tubers or seeds or propagation material or harvested material or the intact plant. In this context, the seed encompasses all parts of the seed such as the seed coats, epidermal cells, seed cells, endosperm or embryonic tissue. Glycerol and/or glycerol-3-phosphate, in particular the respective fine chemical, produced in the process according to the invention may, however, also be isolated from the plant in the form of their free glycerol and/or glycerol-3-phosphate, in particular the free respective fine chemical, or bound in or to compounds or moieties, as for example but not limited to in mono-, di- or triacylglyceroles, phosphoglycerides, monoacylglycerol phosphate or diacylglycerol phosphate. The respective fine chemical produced by this process can be harvested by harvesting the organisms either from the culture in which they grow or from the field. This can be done via expressing, grinding and/or extraction, salt precipitation and/or ion-exchange chromatography or other chromatographic methods of the plant parts, preferably the plant seeds, plant fruits, plant tubers and the like.

**[0375.0.0.21] and [0376.0.0.21]** for the disclosure of the paragraphs [0375.0.0.21] and [0376.0.0.21] see paragraphs [0375.0.0.0] and [0376.0.0.0] above.

**[0377.0.21.21]** Accordingly, the present invention relates also to a process whereby the produced glycerol and/or glycerol-3-phosphate and/or total lipid is isolated.

**[0378.0.21.21]** In this manner, more than 50% by weight, advantageously more than 60% by weight, preferably more than 70% by weight, especially preferably more than 80% by weight, very especially preferably more than 90% by weight, of the glycerol and/or glycerol-3-phosphate and/or total lipid produced in the process can be isolated. The resulting glycerol and/or glycerol-3-phosphate and/or total lipid can, if appropriate, subsequently be further purified, if desired mixed with other active ingredients such as vitamins, amino acids, carbohydrates, antibiotics and the like, and, if appropriate, formulated.

**[0379.0.21.21]** In one embodiment the product produced by the present invention is a mixture of the respective fine chemicals glycerol, glycerol-3-phosphate and total lipids.

**[0380.0.21.21]** The glycerol and/or glycerol-3-phosphate or total lipids obtained in the process by carrying out the invention is suitable as starting material for the synthesis of further products of value. For example, they can be used in combination with each other or alone for the production of pharmaceuticals, foodstuffs, animal feeds or cosmetics. Accordingly, the present invention relates to a method for the production of pharmaceuticals, food stuff, animal feeds, nutrients or cosmetics comprising the steps of the process according to the invention, including the isolation of the glycerol and/or glycerol-3-phosphate composition produced or the respective fine chemical produced if desired and formulating the product with a pharmaceutical acceptable carrier or formulating the product in a form acceptable for an application in agriculture. A further embodiment according to the invention is the use of the glycerol and/or glycerol-3-phosphate and/or total lipid produced in the process or of the transgenic organism in animal feeds, foodstuffs, medicines, food supplements, cosmetics or pharmaceuticals or for the production of glycerol and/or glycerol-3-phosphate e.g. after isolation of the respective fine chemical or without, e.g. in situ, e.g in the organism used for the process for the production of the respective fine chemical.

**[0381.0.0.21] and [0382.0.0.21]** for the disclosure of the paragraphs [0381.0.0.21] and [0382.0.0.21] see paragraphs [0381.0.0.0] and [0382.0.0.0] above.

**[0383.0.21.21] %**

**[0384.0.0.21]** for the disclosure of this paragraph see [0384.0.0.0] above.

**[0385.0.21.21]** The fermentation broths obtained in this way, containing in particular glycerol and/or glycerol-3-phosphate and/or total lipids in mixtures with other organic acids, amino acids, polypeptides or polysaccarides, normally have a dry matter content of from 1 to 70% by weight, preferably 7.5 to 25% by weight. Sugar-limited fermentation is additionally advantageous, e.g. at the end, for example over at least 30% of the fermentation time. This means that the concentration of utilizable sugar in the fermentation medium is kept at, or reduced to, 0 to 10 g/l, preferably to 0 to 3 g/l during this time. The fermentation broth is then processed further. Depending on requirements, the biomass can be removed or isolated entirely or partly by separation methods, such as, for example, centrifugation, filtration, decantation, coagulation/flocculation or a combination of these methods, from the fermentation broth or left completely in it.
The fermentation broth can then be thickened or concentrated by known methods, such as, for example, with the aid of a rotary evaporator, thin-film evaporator, falling film evaporator, by reverse osmosis or by nanofiltration. This concentrated fermentation broth can then be worked up by freeze-drying, spray drying, spray granulation or by other processes.

**[0386.0.21.21]** Accordingly, it is possible to purify the glycerol and/or glycerol-3-phosphate produced according to the invention further. For this purpose, the product-containing composition is subjected for example to separation via e.g. an open column chromatography or HPLC in which case the desired product or the impurities are retained wholly or partly on the chromatography resin. These chromatography steps can be repeated if necessary, using the same or different chromatography resins. The skilled worker is familiar with the choice of suitable chromatography resins and their most effective use.

**[0387.0.0.21] to [0392.0.0.21]** for the disclosure of the paragraphs [0387.0.0.21] to [0392.0.0.21] see paragraphs [0387.0.0.0] to [0392.0.0.0] above.

**[0393.0.21.21]** In one embodiment, the present invention relates to a method for the identification of a gene product conferring an increase in the respective fine chemical production in a cell, comprising the following steps:
a. contacting,̅ e.g. hybridising, the nucleic acid molecules of a sample, e.g. cells, tissues, plants or microorganisms or a nucleic acid library , which can contain a candidate gene encoding a gene product conferring an increase in the respective fine chemical after expression, with the nucleic acid molecule of the present invention;
b. identifying the nucleic acid molecules, which hybridize under relaxed stringent conditions with the nucleic acid molecule of the present invention in particular to the nucleic acid molecule sequence as indicated in Table I, columns 5 or 7, lines 173, 262 to 274 and 628 to 632, preferably as indicated in Table IB, columns 5 or 7, lines 173, 262 to 274 and 628 to 632 resp., and, optionally, isolating the full length cDNA clone or complete genomic clone;
c. introducing the candidate nucleic acid molecules in host cells, preferably in a plant cell or a microorganism, appropriate for producing the respective fine chemical;
d. expressing the identified nucleic acid molecules in the host cells;
e. assaying the respective fine chemical level in the host cells; and
f. identifying the nucleic acid molecule and its gene product which expression confers an increase in the respective fine chemical level in the host cell after expression compared to the wild type.

**[0394.0.0.21] to [0398.0.0.21]** for the disclosure of the paragraphs [0394.0.0.21] to [0398.0.0.21] see paragraphs [0394.0.0.0] to [0398.0.0.0] above.

**[0399.0.21.21]** Furthermore, in one embodiment, the present invention relates to process for the identification of a compound conferring increase of the respective fine chemical production in a plant or microorganism, comprising the steps:
(a) culturing a cell or tissue or microorganism or maintaining a plant expressing the polypeptide according to the invention or a nucleic acid molecule encoding said polypeptide and a readout system capable of interacting with the polypeptide under suitable conditions which permit the interaction of the polypeptide with said readout system in the presence of a compound or a sample comprising a plurality of compounds and capable of providing a detectable signal in response to the binding of a compound to said polypeptide under conditions which permit the expression of said readout system and the polypeptide of the present invention or used in the process of the invention; and
(b) identifying if the compound is an effective agonist by detecting the presence or absence or increase of a signal produced by said readout system.
The screen for a gene product or an agonist conferring an increase in the respective fine chemical production can be performed by growth of an organism for example a microorganism in the presence of growth reducing amounts of an inhibitor of the synthesis of the respective fine chemical. Better growth, e.g. higher dividing rate or high dry mass in comparison to the control under such conditions would identify a gene or gene product or an agonist conferring an increase in respective fine chemical production.

**[00399.1.21.21]** One can think to screen for increased production of the respective fine chemical by for example searching for a resistance to a drug blocking the synthesis of the respective fine chemical and looking whether this effect is dependent on the activity or expression of a polypeptide as indicated in Table II, columns 5 or 7, lines 173, 262 to 274 and 628 to 632 or a homolog thereof, e.g. comparing the phenotype of nearly identical organisms with low and high activity of a protein as indicated in Table II, columns 5 or 7, lines 173, 262 to 274 and 628 to 632 after incubation with the drug.

**[0400.0.0.21] to [0416.0.0.21]** for the disclosure of the paragraphs [0400.0.0.21] to [0416.0.0.21] see paragraphs [0400.0.0.0] to [0416.0.0.0] above.

**[0417.0.21.21]** The nucleic acid molecule of the invention, the vector of the invention or the nucleic acid construct of the invention may also be useful for the production of organisms resistant to inhibitors of the glycerol and/or glycerol-3-phosphate biosynthesis pathways. In particular, the overexpression of the polypeptide of the present invention may protect an organism such as a microorganism or a plant against inhibitors, which block the glycerol and/or glycerol-3-phosphate synthesis.

**[0418.0.0.21] to [0423.0.0.21]** for the disclosure of the paragraphs [0418.0.0.21] to [0423.0.0.21] see paragraphs [0418.0.0.0] to [0423.0.0.0] above.

**[0424.0.21.21]** Accordingly, the nucleic acid of the invention, the polypeptide of the invention, the nucleic acid construct of the invention, the organisms, the host cell, the microorganisms, the plant, plant tissue, plant cell, or the part thereof of the invention, the vector of the invention, the agonist identified with the method of the invention, the nucleic acid molecule identified with the method of the present invention, can be used for the production of the respective fine chemical or of the respective fine chemical and one or more other organic acids. Accordingly, the nucleic acid of the invention, or the nucleic acid molecule identified with the method of the present invention or the complement sequences thereof, the polypeptide of the invention, the nucleic acid construct of the invention, the organisms, the host cell, the microorganisms, the plant, plant tissue, plant cell, or the part thereof of the invention, the vector of the invention, the antagonist identified with the method of the invention, the antibody of the present invention, the antisense molecule of the present invention, can be used for the reduction of the respective fine chemical in a organism or part thereof, e.g. in a cell.

**[0425.0.0.21] to [0434.0.0.21]** for the disclosure of the paragraphs [0425.0.0.21] to [0434.0.0.21] see paragraphs [0425.0.0.0] to [0434.0.0.0] above.

**[0435.0.21.21]** Example 3: In-vivo and in-vitro mutagenesis

**[0436.0.21.21]** An *in vivo* mutagenesis of organisms such as Saccharomyces, Mortierella, Escherichia and others mentioned above, which are beneficial for the production of glycerol and/or glycerol-3-phosphate can be carried out by passing a plasmid DNA (or another vector DNA) containing the desired nucleic acid sequence or nucleic acid sequences, e.g. the nucleic acid molecule of the invention or the vector of the invention, through E.coli and other microorganisms (for example Bacillus spp. or yeasts such as Saccharomyces cerevisiae) which are not capable of maintaining the integrity of its genetic information. Usual mutator strains have mutations in the genes for the DNA repair system [for example mutHLS, mutD, mutT and the like; for comparison, see Rupp, W.D. (1996) DNA repair mechanisms in Escherichia coli and Salmonella, pp. 2277-2294, ASM: Washington]. The skilled worker knows these strains. The use of these strains is illustrated for example in Greener, A. and Callahan, M. (1994) Strategies 7; 32-34.

**[0436.1.21.21]** In-vitro mutation methods such as increasing the spontaneous mutation rates by chemical or physical treatment are well known to the skilled person. Mutagens like 5-bromo-uracil, N-methyl-N-nitro-N-nitrosoguanidine (= NTG), ethyl methanesulfonate (=EMS), hydroxylamine and/or nitrous acid are widly used as chemical agents for random in-vitro mutagensis. The most common physical method for mutagensis is the treatment with UV irradiation. Another random mutagenesis technique is the error-prone PCR for introducing amino acid changes into proteins. Mutations are deliberately introduced during PCR through the use of error-prone DNA polymerases and special reaction conditions known to a person skilled in the art. For this method randomized DNA sequences are cloned into expression vectors and the resulting mutant libraries screened for altered or improved protein activity as described below.
Site-directed mutagensis method such as the introduction of desired mutations with an M13 or phagemid vector and short oligonucleotides primers is a well-known approach for site-directed mutagensis. The clou of this method involves cloning of the nucleic acid sequence of the invention into an M13 or phagemid vector, which permits recovery of single-stranded recombinant nucleic acid sequence. A mutagenic oligonucleotide primer is then designed whose sequence is perfectly complementary to nucleic acid sequence in the region to be mutated, but with a single difference: at the intended mutation site it bears a base that is complementary to the desired mutant nucleotide rather than the original. The mutagenic oligonucleotide is then allowed to prime new DNA synthesis to create a complementary full-length sequence containing the desired mutation. Another site-directed mutagensis method is the PCR mismatch primer mutagensis method also known to the skilled person. Dpnl site-directed mutagensis is a further known method as described for example in the Stratagene QuickchangeTM site-directed mutagenesis kit protocol. A huge number of other methods are also known and used in common practice.
Positive mutation events can be selected by screening the organisms for the production of the desired respective fine chemical.

**[0437.0.21.21]** Example 4: DNA transfer between Escherichia coli, Saccharomyces cerevisiae and Mortierella alpina

**[0438.0.21.21]** Shuttle vectors such as pYE22m, pPAC-ResQ, pClasper, pAUR224, pAMH10, pAML10, pAMT10, pAMU10, pGMH10, pGML10, pGMT10, pGMU10, pPGAL1, pPADH1, pTADH1, pTAex3, pNGA142, pHT3101 and derivatives thereof which allow the transfer of nucleic acid sequences between Escherichia coli, Saccharomyces cerevisiae and/or Mortierella alpina are available to the skilled worker. An easy method to isolate such shuttle vectors is disclosed by Soni R. and Murray J.A.H. [Nucleic Acid Research, vol. 20 no. 21, 1992: 5852]: If necessary such shuttle vectors can be constructed easily using standard vectors for E. coli (Sambrook, J. et al., (1989), "Molecular Cloning: A Laboratory Manual", Cold Spring Harbor Laboratory Press or Ausubel, F.M. et al. (1994) "Current Protocols in Molecular Biology", John Wiley & Sons) and/or the aforementioned vectors, which have a replication origin for, and suitable marker from, Escherichia coli, Saccharomyces cerevisiae or Mortierella alpina added. Such replication origins are preferably taken from endogenous plasmids, which have been isolated from species used in the inventive process. Genes, which are used in particular as transformation markers for these species are genes for kanamycin resistance (such as those which originate from the Tn5 or Tn-903 transposon) or for chloramphenicol resistance (Winnacker, E.L. (1987) "From Genes to Clones - Introduction to Gene Technology", VCH, Weinheim) or for other antibiotic resistance genes such as for G418, gentamycin, neomycin, hygromycin or tetracycline resistance.

**[0439.0.21.21]** Using standard methods, it is possible to clone a gene of interest into one of the above-described shuttle vectors and to introduce such hybrid vectors into the microorganism strains used in the inventive process. The transformation of Saccharomyces can be achieved for example by LiCl or sheroplast transformation (Bishop et al., Mol. Cell. Biol., 6, 1986: 3401 - 3409; Sherman et al., Methods in Yeasts in Genetics, [Cold Spring Harbor Lab. Cold Spring Harbor, N. Y.] 1982, Agatep et al., Technical Tips Online 1998, 1:51: P01525 or Gietz et al., Methods Mol. Cell. Biol. 5, 1995: 255f) or electroporation (Delorme E., Appl. Environ. Microbiol., vol. 55, no. 9, 1989 : 2242 - 2246).

**[0440.0.21.21]** If the transformed sequence(s) is/are to be integrated advantageously into the genome of the microorganism used in the inventive process for example into the yeast or fungi genome, standard techniques known to the skilled worker also exist for this purpose. Solinger et al. (Proc Natl Acad Sci U S A., 2001 (15): 8447-8453) and Freedman et al. (Genetics, Vol. 162, 15-27, September 2002,) teaches a homolog recombination system dependent on rad 50, rad51, rad54 and rad59 in yeasts. Vectors using this system for homologous recombination are vectors derived from the Ylp series. Plasmid vectors derived for example from the 2µ-Vector are known by the skilled worker and used for the expression in yeasts. Other preferred vectors are for example pART1, pCHY21 or pEVP11 as they have been described by McLeod et al. (EMBO J. 1987, 6:729-736) and Hoffman et al. (Genes Dev. 5, 1991: :561-571.) or Russell et al. (J. Biol. Chem. 258, 1983: 143-149.). Other beneficial yeast vectors are plasmids of the REP, REP-X, pYZ or RIP series.

**[0441.0.0.21] to [0443.0.0.21]** for the disclosure of the paragraphs [0441.0.0.21] to [0443.0.0.21] see [0441.0.0.0] to [0443.0.0.0] above.

**[0444.0.21.21]** Example 6: Growth of genetically modified organism: media and culture conditions.

**[0445.0.21.21]** Genetically modified Yeast, Mortierella or Escherichia coli are grown in synthetic or natural growth media known by the skilled worker. A number of different growth media for Yeast, Mortierella or Escherichia coli are well known and widely available. A method for culturing Mortierella is disclosed by Jang et al. [Bot. Bull. Acad. Sin. (2000) 41: 41-48]. Mortierella can be grown at 20°C in a culture medium containing: 10 g/I glucose, 5 g/l yeast extract at pH 6.5. Futhermore Jang et al. teaches a submerged basal medium containing 20 g/I soluble starch, 5 g/l Bacto yeast extract, 10 g/l KNO₃, 1 g/l KH₂PO₄, and 0.5 g/l MgSO₄•7H₂O, pH 6.5.

**[0446.0.0.21] to [0450.0.0.21]** for the disclosure of the paragraphs [0446.0.0.21] to [0450.0.0.21] see [0446.0.0.0] to [0450.0.0.0] above.

**[0451.0.5.21]** for the disclosure of this paragraph see paragraph [0451.0.5.5] above.

**[0452.0.0.21] and [0453.0.0.21]** for the disclosure of the paragraphs [0452.0.0.21] and [0453.0.0.21] see [0452.0.0.0] and [0453.0.0.0] above.

**[0454.0.21.21]** Example 8: Analysis of the effect of the nucleic acid molecule on the production of glycerol and/or glycerol-3-phosphate

**[0455.0.21.21]** The effect of the genetic modification in plants, fungi, algae, ciliates or on the production of a desired compound (such as a glycerol and/or glycerol-3-phosphate) can be determined by growing the modified microorganisms or the modified plant under suitable conditions (such as those described above) and analyzing the medium and/or the cellular components for the elevated production of desired product (i.e. of glycerol and/or glycerol-3-phosphate). These analytical techniques are known to the skilled worker and comprise spectroscopy, thin-layer chromatography, various types of staining methods, enzymatic and microbiological methods and analytical chromatography such as high-performance liquid chromatography (see, for example, Ullman, Encyclopedia of Industrial Chemistry, Vol. A2, p. 89-90 and p. 443-613, VCH: Weinheim (1985); Fallon, A., et al., (1987) "Applications of HPLC in Biochemistry" in: Laboratory Techniques in Biochemistry and Molecular Biology, Vol. 17; Rehm et al. (1993) Biotechnology, Vol. 3, Chapter III: "Product recovery and purification", p. 469-714, VCH: Weinheim; Belter, P.A., et al. (1988) Bioseparations: downstream processing for Biotechnology, John Wiley and Sons; Kennedy, J.F., and Cabral, J.M.S. (1992) Recovery processes for biological Materials, John Wiley and Sons; Shaeiwitz, J.A., and Henry, J.D. (1988) Biochemical Separations, in: Ullmann's Encyclopedia of Industrial Chemistry, Vol. B3; Chapter 11, p. 1-27, VCH: Weinheim; and Dechow, F.J. (1989) Separation and purification techniques in biotechnology, Noyes Publications).

**[0456.0.0.21]:** for the disclosure of this paragraph see paragraph [0456.0.0.0] above.

**[0457.0.21.21]** Example 9: Purification of glycerol and/or glycerol-3-phosphate

**[0458.0.21.21]** Abbreviations; GC-MS, gas liquid chromatography/mass spectrometry; TLC, thin-layer chromatography.
The unambiguous detection for the presence of glycerol and/or glycerol-3-phosphate can be obtained by analyzing recombinant organisms using analytical standard methods: LC, LC-MSMS, GC-MS or TLC, as described. The total amount produced in the organism for example in yeasts used in the inventive process can be analysed for example according to the following procedure:
The material such as yeasts, E. coli or plants to be analyzed can be disrupted by sonication, grinding in a glass mill, liquid nitrogen and grinding or via other applicable methods.
Plant material is initially homogenized mechanically by comminuting in a pestle and mortar to make it more amenable to extraction.
A typical sample pretreatment consists of a total lipid extraction using such polar organic solvents as acetone or alcohols as methanol, or ethers, saponification, partition between phases, seperation of non-polar epiphase from more polar hypophasic derivatives and chromatography.
For analysis, solvent delivery and aliquot removal can be accomplished with a robotic system comprising a single injector valve Gilson 232XL and a 402 2S1V diluter [Gilson, Inc. USA, 3000 W. Beltline Highway, Middleton, WI]. For saponification, 3 ml of 50% potassium hydroxide hydro-ethanolic solution (4 water - 1 ethanol) can be added to each vial, followed by the addition of 3 ml of octanol. The saponification treatment can be conducted at room temperature with vials maintained on an IKA HS 501 horizontal shaker [Labworld-online, Inc., Wilmington, NC] for fifteen hours at 250 movements/minute, followed by a stationary phase of approximately one hour.
Following saponification, the supernatant can be diluted with 0 -20 ml of methanol. The addition of methanol can be conducted under pressure to ensure sample homogeneity. Using a 0.25 ml syringe, a 0.1 ml aliquot can be removed and transferred to HPLC vials for analysis.
For HPLC analysis, a Hewlett Packard 1100 HPLC, complete with a quaternary pump, vacuum degassing system, six-way injection valve, temperature regulated autosampler, column oven and Photodiode Array detector can be used [Agilent Technologies available through Ultra Scientific Inc., 250 Smith Street, North Kingstown, RI]. The column can be a Waters YMC30, 5-micron, 4.6 x 250 mm with a guard column of the same material [Waters, 34 Maple Street, Milford, MA]. The solvents for the mobile phase can be 81 methanol: 4 water: 15 tetrahydrofuran (THF) stabilized with 0.2% BHT (2,6-di-tert-butyl-4-methylphenol). Injections were 20 µl. Separation can be isocratic at 30°C with a flow rate of 1.7 ml/minute. The peak responses can be measured by absorbance at 447 nm.

**[0459.0.21.21]** If required and desired, further chromatography steps with a suitable resin may follow. Advantageously, the glycerol and/or glycerol-3-phosphate can be further purified with a so-called RTHPLC. As eluent acetonitrile/water or chloroform/acetonitrile mixtures can be used. If necessary, these chromatography steps may be repeated, using identical or other chromatography resins. The skilled worker is familiar with the selection of suitable chromatography resin and the most effective use for a particular molecule to be purified.

**[0460.0.0.21]** for the disclosure of this paragraph see paragraph [0460.0.0.0] above.

**[0461.0.21.21]** Example 10: Cloning SEQ ID NO: 14364, 27329, 27377, 27857, 28009,28145,28179,28197,28201,28207,28211, 28341, 28363, 28369, 98288, 98533, 97771, 97966 or 98453 for the expression in plants

**[0462.0.0.21]** for the disclosure of this paragraph see paragraph [0462.0.0.0] above.

**[0463.0.21.21]** SEQ ID NO: 14364, 27329, 27377, 27857, 28009, 28145, 28179, 28197, 28201, 28207, 28211, 28341, 28363, 28369, 98288, 98533, 97771, 97966 or 98453 is amplified by PCR as described in the protocol of the Pfu Turbo or DNA Herculase polymerase (Stratagene).

**[0464.0.0.21] to [0466.0.0.21]** for the disclosure of the paragraphs [0464.0.0.21] to [0466.0.0.21] see paragraphs [0464.0.0.0] to [0466.0.0.0] above.

**[0466.1.0.21]** In case the Herculase enzyme can be used for the amplification, the PCR amplification cycles were as follows: 1 cycle of 2-3 minutes at 94°C, followed by 25-30 cycles of in each case 30 seconds at 94°C, 30 seconds at 55-60°C and 5-10 minutes at 72°C, followed by 1 cycle of 10 minutes at 72°C, then 4°C.

**[0467.0.21.21]** The following primer sequences were selected for the gene SEQ ID NO: 14364
i) forward primer (SEQ ID NO:14370)
   atggctcaaa attttggaaa gattcc
ii) reverse primer (SEQ ID NO:14371)
   ttaaaaaccg tatttcgcca aaacac
The following primer sequences were selected for the gene SEQ ID NO:27329:
i) forward primer (SEQ ID NO:27375)
   atgaaaaatg ccaatcatcg attcttc
ii) reverse primer (SEQ ID NO:27376)
   ttagaagaac agtgacggat cgcc
The following primer sequences were selected for the gene SEQ ID NO:27377:
i) forward primer (SEQ ID NO:27855)
   atgtctgagc agtttttgta tttctt
ii) reverse primer (SEQ ID NO: 27856)
   ttatactttc tctacctccg ggcg
The following primer sequences were selected for the gene SEQ ID NO: 27857:
i) forward primer (SEQ ID NO:28007)
   atgttgtcga gactatcttt attgag
ii) reverse primer (SEQ ID NO:28008)
   ttaaaataga ccttcaattt caccgt
The following primer sequences were selected for the gene SEQ ID NO:28009:
i) forward primer (SEQ ID NO:28143)
   atggagacca atttttcctt cgact
ii) reverse primer (SEQ ID NO:28144)
   ctattgaaat accggcttca atattt
The following primer sequences were selected for the gene SEQ ID NO:28145:
i) forward primer (SEQ ID NO:28177)
   atgtcagcag acgaaacgga tgc
ii) reverse primer (SEQ ID NO:28178)
   ttatgttttt ttgtctgctg cagct
The following primer sequences were selected for the gene SEQ ID NO:28179:
i) forward primer (SEQ ID NO:28195)
   atgaatgatc ctcgtgaaat tttagc
ii) reverse primer (SEQ ID NO:28196)
   ttatattatc tcaagatcgc tggca
The following primer sequences were selected for the gene SEQ ID NO:28197:
i) forward primer (SEQ ID NO:28199)
   atgtccaact ttaagaattt tactttaa
ii) reverse primer (SEQ ID NO:28200)
   tcatttgttt atcagtgtaa caagca
The following primer sequences were selected for the gene SEQ ID NO:28201:
i) forward primer (SEQ ID NO:28205)
   atgaatcaga gcgatagcag cttg
ii) reverse primer (SEQ ID NO:28206)
   tcatcttcga agataagggg tattc
The following primer sequences were selected for the gene SEQ ID NO:28207:
i) forward primer (SEQ ID NO:28209)
   atggcggttg cgatcaaaaa gga
ii) reverse primer (SEQ ID NO:28210)
   tcaattgata aatgtacttt caatgatg
The following primer sequences were selected for the gene SEQ ID NO:28211:
i) forward primer (SEQ ID NO:28339)
   atgagttctg tcgcagaaaa tataat
ii) reverse primer (SEQ ID NO:28340)
   ttattcgaag acttctccag taattg
The following primer sequences were selected for the gene SEQ ID NO: 28341:
i) forward primer (SEQ ID NO: 28361)
   atgaactcta ttttagacag aaatgtt
ii) reverse primer (SEQ ID NO: 28362)
   ttatttttgg tcttgtttca aagtgtc
The following primer sequences were selected for the gene SEQ ID NO: 28363:
i) forward primer (SEQ ID NO: 28367)
   atgttaaaaa gacgctctaa tgctc
ii) reverse primer (SEQ ID NO: 28368)
   tcaccaaaac catctcctat accaa
The following primer sequences were selected for the gene SEQ ID NO: 28369:
i) forward primer (SEQ ID NO: 28563)
   atgactgaaa gtgctataga tgacc
ii) reverse primer (SEQ ID NO: 28564)
   ctataaaatt tgtgtatgaa taaataaag
The following primer sequences were selected for the gene SEQ ID NO: 98288:
i) forward primer (SEQ ID NO: 98448)
   atgatccgca gtatgaccgc ct
ii) reverse primer (SEQ ID NO: 98449)
   ttattcgatg ttctgaatct gctc
The following primer sequences were selected for the gene SEQ ID NO: 98533:
i) forward primer (SEQ ID NO: 98701)
   ttaaagcgta tgtgtttcat atgcc
ii) reverse primer (SEQ ID NO: 98702)
   atgacagaat tttattctga cacaatc
The following primer sequences were selected for the gene SEQ ID NO: 97771:
i) forward primer (SEQ ID NO: 97961)
   atgtctattg tggtgaaaaa taacatt
ii) reverse primer (SEQ ID NO: 97962)
   ttattttgcc tccgatgcca gttct
The following primer sequences were selected for the gene SEQ ID NO: 97966:
i) forward primer (SEQ ID NO: 98282)
   atgacgaatc cgttactgac tcc
ii) reverse primer (SEQ ID NO: 98283)
   ttagccctta atgccgtaat gctcc
The following primer sequences were selected for the gene SEQ ID NO: 98453:
i) forward primer (SEQ ID NO: 98527)
   atgacgcaga cttccgcatt tca
ii) reverse primer (SEQ ID NO: 98528)
   ttacgccacc gtcaactgtc cg

**[0468.0.0.21] to [0470.0.0.21]** for the disclosure of the paragraphs [0468.0.0.21] to [0470.0.0.21] see paragraphs [0468.0.0.0] to [0470.0.0.0] above.

**[0470.1.21.21]** The PCR-products, produced by *Pfu* Turbo DNA polymerase, were phosphorylated using a T4 DNA polymerase using a standard protocol (e.g. MBI Fermentas) and cloned into the processed binary vector.

**[0471.0.21.21]** for the disclosure of this paragraph see [0471.0.0.0] above.

**[0471.1.21.21]** The DNA termini of the PCR-products, produced by Herculase DNA polymerase, were blunted in a second synthesis reaction using *Pfu* Turbo DNA polymerase. The composition for the protocol of the blunting the DNA-termini was as follows: 0.2 mM blunting dTTP and 1.25 u *Pfu* Turbo DNA polymerase. The reaction was incubated at 72°C for 30 minutes. Then the PCR-products were phosphorylated using a T4 DNA polymerase using a standard protocol (e.g. MBI Fermentas) and cloned into the processed vector as well.

**[0472.0.0.21] to [0479.0.0.21]** for the disclosure of the paragraphs [0472.0.0.21] to [0479.0.0.21] see paragraphs [0472.0.0.0] to [0479.0.0.0] above.

**[0480.0.21.21]** Example 11: Generation of transgenic plants which express SEQ ID NO: 14364, 27329, 27377, 27857, 28009, 28145, 28179, 28197, 28201, 28207, 28211, 28341, 28363, 28369, 98288, 98533, 97771, 97966 or 98453

**[0481.0.0.21] to [0513.0.0.21]** for the disclosure of the paragraphs [0481.0.0.21] to [0513.0.0.21] see paragraphs [0482.0.0.0] to [0513.0.0.0] above.

**[0514.0.21.21]** As an alternative, glycerol-3-phosphate can be detected as described in Yaqoob M et al., J Biolumin Chemilumin. 1997 Jan-Feb; 12 (1): 1-5. Glycerol can be detected as described in Foglia, T.A. et al., 2004. Chromatographia. 60:305-311.
The results of the different plant analyses can be seen from the table 1 which follows:

**Table 1**

| **ORF** | **Metabolite** | **Method** | **Min** | **Max** |
|---|---|---|---|---|
| YDR065 | glycerol-3-phosphate | GC | 1,18 | 2,14 |
| YBR084W | glycerol, polar fraction | GC | 2,02 | 3,53 |
| YDR513W | glycerol, lipid fraction | GC | 1,17 | 1,48 |
| YDR513W | glycerol, polar fraction | GC | 1,40 | 3,00 |
| YGL237C | glycerol, lipid fraction | GC | 1,23 | 1,73 |
| YIL150C | glycerol, lipid fraction | GC | 1,94 | 3.19 |
| YLR082C | glycerol, polar fraction | GC | 1,41 | 2,73 |
| YLR224W | glycerol, lipid fraction | GC | 1,17 | 1,43 |
| YLR255C | glycerol, polar fraction | GC | 1,44 | 1,73 |
| YMR015C | glycerol, lipid fraction | GC | 1,18 | 1,23 |
| YOR344C | glycerol, lipid fraction | GC | 1,18 | 2,04 |
| YPL099C | glycerol, polar fraction | GC | 1,55 | 2,04 |
| YPL268W | glycerol, polar fraction | GC | 1,38 | 2,15 |
| b2441 | glycerol, polar fraction | GC | 1,41 | 1,81 |
| b3457 | glycerol, lipid fraction | GC | 1,18 | 1,44 |
| YHR072W | Glycerol, lipid fraction | GC | 1.19 | 2.09 |
| b3644 | Glycerol, lipid fraction | GC | 1.17 | 1.44 |
| b3498 | Glycerol-3-phosphate, lipid fraction | GC | 1.20 | 1.56 |
| b2710 | Glycerol-3-phosphate, lipid fraction | GC | 1.20 | 1.95 |
| b4073 | Glycerol-3-phosphate, lipid fraction | GC | 1.49 | 2.45 |

**[0515.0.21.21]** Column 2 shows the metabolite glycerol or glycerol-3-phosphate analyzed. According to its solubility the free glycerol of the cells is present and measured in the polar fraction (glycerol, polar fraction). The non polar extract (= lipid fraction) is evaporated and the residue is treated with a mixture of methanol and hydrochloric acid in order to cleave ester functions of the analytes as desribed above in example 13. The glycerol, bonded with up to three ester functions, i.e. in lipids, is converted to free glycerol by this reaction, measured as the glycerol in the lipid fraction. Columns 4 and 5 shows the ratio of the analyzed metabolite between the transgenic plants and the wild type; Increase of the metabolite: Max: maximal x-fold (normalised to wild type)-Min: minimal x-fold (normalised to wild type). Decrease of the metabolite: Max: maximal x-fold (normalised to wild type) (minimal decrease), Min: minimal x-fold (normalised to wild type) (maximal decrease). Column 3 indicates the analytical method.

**[0516.0.0.21] to [0530.0.0.21]** for the disclosure of the paragraphs [0516.0.0.21] to [0530.0.0.21] see paragraphs [0516.0.0.0] to [0530.0.0.0] above.

**[0530.1.0.21] to [0530.6.0.21]** for the disclosure of the paragraphs [0530.1.0.21] to [0530.6.0.21] see paragraphs [0530.1.0.0] to [0530.6.0.0] above.

**[0531.0.0.21] to [0552.0.0.21]** for the disclosure of the paragraphs [0531.0.0.21] to [0552.0.0.21] see paragraphs [0531.0.0.0] to [0552.0.0.0] above.

**[0552.1.0.21]:** Example 15: Metabolite Profiling Info from *Zea mays*
*Zea mays* plants were engineered as described in Example 14c.
The results of the different *Zea mays* plants analysed can be seen from table 2, which follows:

**Table 2**

| **ORF_NAME** | **Metabolite** | **Min** | **Max** |
|---|---|---|---|
| YIL150C | Glycerol, lipid fraction | 2.12 | 2.66 |
| YBR084W | Glycerol, polar fraction | 1.36 | 1.44 |
| YLR082C | Glycerol, polar fraction | 1.12 | 1.35 |

Table 2 shows the increase in glycerol in genetically modified corn plants expressing the Saccharomyces cerevisiae nucleic acid sequence YIL150c, YBR084W or YLR082C.
In one embodiment, in case the activity of the Saccaromyces cerevisiae protein YIL150C or its homologs, e.g. a "chromatin binding protein, required for S-phase (DNA synthesis) initiation or completion" or its homologs, is increased in corn plants, preferably, an increase of the fine chemical glycerol between 112% and 166% is conferred.
In one embodiment, in case the activity of the Saccaromyces cerevisiae protein YBR084W or its homologs, e.g. a protein with C1-tetrahydrofolate synthase activity or its homologs, is increased in corn plants, preferably, an increase of the fine chemical glycerol between 36% and 44% is conferred.
In one embodiment, in case the activity of the Saccaromyces cerevisiae protein YLR082C or its homologs, e.g. a suppressor of Rad53 null lethality protein or its homologs, is increased in corn plants, preferably, an increase of the fine chemical glycerol between 12% and 35% is conferred.

**[0552.2.0.21]** for the disclosure of this paragraph see [0552.2.0.0] above.

**[0553.0.21.21]**
1. A process for the production of glycerol-3-phosphate or glycerol, which comprises
   (a) increasing or generating the activity of a protein as indicated in Table II, columns 5 or 7, lines 173, 262 to 274 and 628 to 632 or a functional equivalent thereof in a non-human organism or in one or more parts thereof; and
   (b) growing the organism under conditions which permit the production of glycerol-3-phosphate or glycerol in said organism.
2. A process for the production of glycerol-3-phosphate or glycerol, comprising the increasing or generating in an organism or a part thereof the expression of at least one nucleic acid molecule comprising a nucleic acid molecule selected from the group consisting of:
   a) nucleic acid molecule encoding a polypeptide as indicated in Table II, columns 5 or 7, lines 173, 262 to 274 and 628 to 632 or a fragment thereof, which confers an increase in the amount of glycerol-3-phosphate or glycerol in an organism or a part thereof;
   b) nucleic acid molecule comprising of a nucleic acid molecule as indicated in Table I, columns 5 or 7, lines 173, 262 to 274 and 628 to 632;
   c) nucleic acid molecule whose sequence can be deduced from a polypeptide sequence encoded by a nucleic acid molecule of (a) or (b) as a result of the degeneracy of the genetic code and conferring an increase in the amount of glycerol-3-phosphate or glycerol in an organism or a part thereof;
   d) nucleic acid molecule which encodes a polypeptide which has at least 50% identity with the amino acid sequence of the polypeptide encoded by the nucleic acid molecule of (a) to (c) and conferring an increase in the amount of glycerol-3-phosphate or glycerol in an organism or a part thereof;
   e) nucleic acid molecule which hybidizes with a nucleic acid molecule of (a) to (c) under stringent hybridisation conditions and conferring an increase in the amount of glycerol-3-phosphate or glycerol in an organism or a part thereof;
   f) nucleic acid molecule which encompasses a nucleic acid molecule which is obtained by amplifying nucleic acid molecules from a cDNA library or a genomic library using the primers or primer pairs as indicated in Table III, column 7, lines 173, 262 to 274 and 628 to 632 and conferring an increase in the amount of glycerol-3-phosphate or glycerol in an organism or a part thereof;
   g) nucleic acid molecule encoding a polypeptide which is isolated with the aid of monoclonal antibodies against a polypeptide encoded by one of the nucleic acid molecules of (a) to (f) and conferring an increase in the amount of glycerol-3-phosphate or glycerol in an organism or a part thereof;
   h) nucleic acid molecule encoding a polypeptide comprising a consensus as indicated in Table IV, column 7, lines 173, 262 to 274 and 628 to 632 and conferring an increase in the amount of glycerol-3-phosphate or glycerol in an organism or a part thereof; and
   i) nucleic acid molecule which is obtainable by screening a suitable nucleic acid library under stringent hybridization conditions with a probe comprising one of the sequences of the nucleic acid molecule of (a) to (k) or with a fragment thereof having at least 15 nt, preferably 20 nt, 30 nt, 50 nt, 100 nt, 200 nt or 500 nt of the nucleic acid molecule characterized in (a) to (k) and conferring an increase in the amount of glycerol-3-phosphate or glycerol in an organism or a part thereof.
   or comprising a sequence which is complementary thereto.
3. The process of claim 1 or 2, comprising recovering of the free or bound glycerol-3-phosphate or glycerol.
4. The process of any one of claim 1 to 3, comprising the following steps:
   a) selecting an organism or a part thereof expressing a polypeptide encoded by the nucleic acid molecule characterized in claim 2;
   b) mutagenizing the selected organism or the part thereof;
   c) comparing the activity or the expression level of said polypeptide in the mutagenized organism or the part thereof with the activity or the expression of said polypeptide of the selected organisms or the part thereof;
   d) selecting the mutated organisms or parts thereof, which comprise an increased activity or expression level of said polypeptide compared to the selected organism or the part thereof;
   e) optionally, growing and cultivating the organisms or the parts thereof; and
   f) recovering, and optionally isolating, the free or bound glycerol-3-phosphate or glycerol produced by the selected mutated organisms or parts thereof.
5. The process of any one of claims 1 to 4, wherein the activity of said protein or the expression of said nucleic acid molecule is increased or generated transiently or stably.
6. An isolated nucleic acid molecule comprising a nucleic acid molecule selected from the group consisting of:
   a) nucleic acid molecule encoding a polypeptide as indicated in Table II, columns 5 or 7, lines 173, 262 to 274 and 628 to 632 or a fragment thereof, which confers an increase in the amount of glycerol-3-phosphate or glycerol in an organism or a part thereof;
   b) nucleic acid molecule comprising of a nucleic acid molecule as indicated in Table I, columns 5 or 7, lines 173, 262 to 274 and 628 to 632;
   c) nucleic acid molecule whose sequence can be deduced from a polypeptide sequence encoded by a nucleic acid molecule of (a) or (b) as a result of the degeneracy of the genetic code and conferring an increase in the amount of glycerol-3-phosphate or glycerol in an organism or a part thereof;
   d) nucleic acid molecule which encodes a polypeptide which has at least 50% identity with the amino acid sequence of the polypeptide encoded by the nucleic acid molecule of (a) to (c) and conferring an increase in the amount of glycerol-3-phosphate or glycerol in an organism or a part thereof;
   e) nucleic acid molecule which hybidizes with a nucleic acid molecule of (a) to (c) under stringent hybridisation conditions and conferring an increase in the amount of glycerol-3-phosphate or glycerol in an organism or a part thereof;
   f) nucleic acid molecule which encompasses a nucleic acid molecule which is obtained by amplifying nucleic acid molecules from a cDNA library or a genomic library using the primers or primer pairs as indicated in Table III, column 7, lines 173, 262 to 274 and 628 to 632 and conferring an increase in the amount of glycerol-3-phosphate or glycerol in an organism or a part thereof;
   g) nucleic acid molecule encoding a polypeptide which is isolated with the aid of monoclonal antibodies against a polypeptide encoded by one of the nucleic acid molecules of (a) to (f) and conferring an increase in the amount of glycerol-3-phosphate or glycerol in an organism or a part thereof;
   h) nucleic acid molecule encoding a polypeptide comprising a consensus as indicated in Table IV, column 7, lines 173, 262 to 274 and 628 to 632 and conferring an increase in the amount of glycerol-3-phosphate or glycerol in an organism or a part thereof; and
   i) nucleic acid molecule which is obtainable by screening a suitable nucleic acid library under stringent hybridization conditions with a probe comprising one of the sequences of the nucleic acid molecule of (a) to (k) or with a fragment thereof having at least 15 nt, preferably 20 nt, 30 nt, 50 nt, 100 nt, 200 nt or 500 nt of the nucleic acid molecule characterized in (a) to (k) and conferring an increase in the amount of glycerol-3-phosphate or glycerol in an organism or a part thereof.
   whereby the nucleic acid molecule distinguishes over the sequence as indicated in Table I, columns 5 or 7, lines 173, 262 to 274 and 628 to 632 by one or more nucleotides.
7. A nucleic acid construct which confers the expression of the nucleic acid molecule of claim 6, comprising one or more regulatory elements.
8. A vector comprising the nucleic acid molecule as claimed in claim 6 or the nucleic acid construct of claim 7.
9. The vector as claimed in claim 8, wherein the nucleic acid molecule is in operable linkage with regulatory sequences for the expression in a prokaryotic or eukaryotic, or in a prokaryotic and eukaryotic, host.
10. A host cell, which has been transformed stably or transiently with the vector as claimed in claim 8 or 9 or the nucleic acid molecule as claimed in claim 6 or the nucleic acid construct of claim 7 or produced as described in any one of claims 2 to 5.
11. The host cell of claim 10, which is a transgenic host cell.
12. The host cell of claim 10 or 11, which is a plant cell, an animal cell, a microorganism, or a yeast cell, a fungus cell, a prokaryotic cell, an eukaryotic cell or an archaebacterium.
13. A process for producing a polypeptide, wherein the polypeptide is expressed in a host cell as claimed in any one of claims 10 to 12.
14. A polypeptide produced by the process as claimed in claim 13 or encoded by the nucleic acid molecule as claimed in claim 6 whereby the polypeptide distinguishes over a sequence as indicated in Table II, columns 5 or 7, lines 173, 262 to 274 and 628 to 632 by one or more amino acids.
15. An antibody, which binds specifically to the polypeptide as claimed in claim 14.
16. A plant tissue, propagation material, harvested material or a plant comprising the host cell as claimed in claim 12 which is plant cell or an Agrobacterium.
17. A method for screening for agonists and antagonists of the activity of a polypeptide encoded by the nucleic acid molecule of claim 6 conferring an increase in the amount of glycerol-3-phosphate or glycerol in an organism or a part thereof comprising:
   (a) contacting cells, tissues, plants or microorganisms which express the a polypeptide encoded by the nucleic acid molecule of claim 6 conferring an increase in the amount of glycerol-3-phosphate or glycerol in an organism or a part thereof with a candidate compound or a sample comprising a plurality of compounds under conditions which permit the expression the polypeptide;
   (b) assaying the glycerol-3-phosphate or glycerol level or the polypeptide expression level in the cell, tissue, plant or microorganism or the media the cell, tissue, plant or microorganisms is cultured or maintained in; and
   (c) identifying a agonist or antagonist by comparing the measured glycerol-3-phosphate or glycerol level or polypeptide expression level with a standard glycerol-3-phosphate or glycerol or polypeptide expression level measured in the absence of said candidate compound or a sample comprising said plurality of compounds, whereby an increased level over the standard indicates that the compound or the sample comprising said plurality of compounds is an agonist and a decreased level over the standard indicates that the compound or the sample comprising said plurality of compounds is an antagonist.
18. A process for the identification of a compound conferring increased glycerol-3-phosphate or glycerol production in a plant or microorganism, comprising the steps:
   a) culturing a plant cell or tissue or microorganism or maintaining a plant expressing the polypeptide encoded by the nucleic acid molecule of claim 6 conferring an increase in the amount of glycerol-3-phosphate or glycerol in an organism or a part thereof and a readout system capable of interacting with the polypeptide under suitable conditions which permit the interaction of the polypeptide with said readout system in the presence of a compound or a sample comprising a plurality of compounds and capable of providing a detectable signal in response to the binding of a compound to said polypeptide under conditions which permit the expression of said readout system and of the polypeptide encoded by the nucleic acid molecule of claim 6 conferring an increase in the amount of glycerol-3-phosphate or glycerol in an organism or a part thereof;
   b) identifying if the compound is an effective agonist by detecting the presence or absence or increase of a signal produced by said readout system.
19. A method for the identification of a gene product conferring an increase in glycerol-3-phosphate or glycerol production in a cell, comprising the following steps:
   a) contacting the nucleic acid molecules of a sample, which can contain a candidate gene encoding a gene product conferring an increase in glycerol-3-phosphate or glycerol after expression with the nucleic acid molecule of claim 6;
   b) identifying the nucleic acid molecules, which hybridise under relaxed stringent conditions with the nucleic acid molecule of claim 6;
   c) introducing the candidate nucleic acid molecules in host cells appropriate for producing glycerol-3-phosphate or glycerol;
   d) expressing the identified nucleic acid molecules in the host cells;
   e) assaying the glycerol-3-phosphate or glycerol level in the host cells; and
   f) identifying nucleic acid molecule and its gene product which expression confers an increase in the glycerol-3-phosphate or glycerol in the host cell in the host cell after expression compared to the wild type.
20. A method for the identification of a gene product conferring an increase in glycerol-3-phosphate or glycerol production in a cell, comprising the following steps:
   a) identifiying in a data bank nucleic acid molecules of an organism; which can contain a candidate gene encoding a gene product conferring an increase in the glycerol-3-phosphate or glycerol amount or level in an organism or a part thereof after expression, and which are at least 20% homolog to the nucleic acid molecule of claim 6;
   b) introducing the candidate nucleic acid molecules in host cells appropriate for producing glycerol-3-phosphate or glycerol;
   c) expressing the identified nucleic acid molecules in the host cells;
   d) assaying the glycerol-3-phosphate or glycerol level in the host cells; and
   e) identifying nucleic acid molecule and its gene product which expression confers an increase in the glycerol-3-phosphate or glycerol level in the host cell after expression compared to the wild type.
21. A method for the production of an agricultural composition comprising the steps of the method of any one of claims 17 to 20 and formulating the compound identified in any one of claims 17 to 20 in a form acceptable for an application in agriculture.
22. A composition comprising the nucleic acid molecule of claim 6, the polypeptide of claim 14, the nucleic acid construct of claim 7, the vector of any one of claims 8 or 9, an antagonist or agonist identified according to claim 17, the compound of claim 18, the gene product of claim 19 or 20, the antibody of claim 15, and optionally an agricultural acceptable carrier.
23. Use of the nucleic acid molecule as claimed in claim 6 for the identification of a nucleic acid molecule conferring an increase of glycerol-3-phosphate or glycerol after expression.
24. Use of the polypeptide of claim 14 or the nucleic acid construct claim 7 or the gene product identified according to the method of claim 19 or 20 for identifying compounds capable of conferring a modulation of glycerol-3-phosphate or glycerol levels in an organism.
25. Agrochemical, pharmaceutical, food or feed composition comprising the nucleic acid molecule of claim 6, the polypeptide of claim 14, the nucleic acid construct of claim 7, the vector of claim 8 or 9, the antagonist or agonist identified according to claim 17, the antibody of claim 15, the plant or plant tissue of claim 16, the harvested material of claim 16, the host cell of claim 10 to 12 or the gene product identified according to the method of claim 19 or 20.
26. The method of any one of claims 1 to 5, the nucleic acid molecule of claim 6, the polypeptide of claim 14, the nucleic acid construct of claim 7, the vector of claim 8 or 9, the antagonist or agonist identified according to claim 17, the antibody of claim 15, the plant or plant tissue of claim 16, the harvested material of claim 16, the host cell of claim 10 to 12 or the gene product identified according to the method of claim 19 or 20, wherein the fine chemical is glycerol-3-phosphate or glycerol.
27. A host cell or plant according to any of the claims 10 to 12 which is resistant to a herbicide inhibiting the biosynthesis of glycerol-3-phosphate or glycerol.
28. A process for the increased production of total lipids, comprising the increasing or generating in an organism or a part thereof the expression of at least one nucleic acid molecule comprising a nucleic acid molecule selected from the group consisting of:
   a) nucleic acid molecule encoding a polypeptide as indicated in Table II, columns 5 or 7, lines 173, 262 to 274 and 628 to 632 or a fragment thereof, which confers an increase in the amount of total lipids in an organism or a part thereof;
   b) nucleic acid molecule comprising of a nucleic acid molecule as indicated in Table I, columns 5 or 7, lines 173, 262 to 274 and 628 to 632;
   c) nucleic acid molecule whose sequence can be deduced from a polypeptide sequence encoded by a nucleic acid molecule of (a) or (b) as a result of the degeneracy of the genetic code and conferring an increase in the amount of total lipids in an organism or a part thereof;
   d) nucleic acid molecule which encodes a polypeptide which has at least 50% identity with the amino acid sequence of the polypeptide encoded by the nucleic acid molecule of (a) to (c) and conferring an increase in the amount of total lipids in an organism or a part thereof;
   e) nucleic acid molecule which hybidizes with a nucleic acid molecule of (a) to (c) under stringent hybridisation conditions and conferring an increase in the amount of total lipids in an organism or a part thereof;
   f) nucleic acid molecule which encompasses a nucleic acid molecule which is obtained by amplifying nucleic acid molecules from a cDNA library or a genomic library using the primers or primer pairs as indicated in Table III, column 7, lines 173, 262 to 274 and 628 to 632 and conferring an increase in the amount of total lipids in an organism or a part thereof;
   g) nucleic acid molecule encoding a polypeptide which is isolated with the aid of monoclonal antibodies against a polypeptide encoded by one of the nucleic acid molecules of (a) to (f) and conferring an increase in the amount of total lipids in an organism or a part thereof;
   h) nucleic acid molecule encoding a polypeptide comprising a consensus as indicated in Table IV, column 7, lines 173, 262 to 274 and 628 to 632 and conferring an increase in the amount of total lipids in an organism or a part thereof; and
   i) nucleic acid molecule which is obtainable by screening a suitable nucleic acid library under stringent hybridization conditions with a probe comprising one of the sequences of the nucleic acid molecule of (a) to (k) or with a fragment thereof having at least 15 nt, preferably 20 nt, 30 nt, 50 nt, 100 nt, 200 nt or 500 nt of the nucleic acid molecule characterized in (a) to (k) and conferring an increase in the amount of total lipids in an organism or a part thereof.
   or comprising a sequence which is complementary thereto.
29. The use of the nucleic acid molecule encoding a polypeptide as indicated in Table II, columns 5 or 7, lines 173, 262 to 274 and 628 to 632 or a fragment thereof, which confers an increase in the amount of total lipids in an organism or a part thereof for the identification of a nucleic acid molecule conferring an increase in the amount of total lipids after expression.

**[0554.0.0.21] Abstract: see [0554.0.0.0]**

### Process for the production of fine chemicals

**[0000.0.0.22]** In a further embodiment, the present invention relates to a further process for the production of fine chemicals as defined below and the corresponding embodiments as described herein as follows.

**[0001.0.0.22]** for the disclosure of this paragraph see [0001.0.0.0].

**[0002.0.22.22]** Lipids differ markedly from other groups of biomolecules and metabolites. By definition, lipids are water-insoluble biomolecules that are highly soluble in organic solvents such as chloroform. Lipids have a variety of biological roles: they serve as fuel molecules, highly concentrated energy stores, signal molecules, and components of membranes.
The major kinds of membrane lipids are phospholipids, glycolipids, and cholesterol. Glycolipids are sugar-containing lipids. The term glycolipid designates any compound containing one or more monosaccharide residues bound by a glycosidic linkage to a hydrophobic moiety such as an acylglycerol, a sphingoid, a ceramide (N-acylsphingoid) or a prenyl phosphate.

**[0003.0.22.22]** Galactose-containing lipids are the predominant nonproteinaceous components of photosynthetic membranes in plants, algae, and a variety of bacteria. In higher plants, the galactolipids contain a high proportion of polyunsaturated fatty acids, up to 95% of which can be linolenic acid (18:3(n-3)). In non-photosynthetic tissues, such as tubers or roots, the C₁₈ fatty acids are usually more saturated.
In plants, especially photosynthetic tissues, a substantial proportion of the lipids consists of 1,2-diacyl-sn-glycerols joined by a glycosidic linkage at position sn-3 to a carbohydrate moiety. The two most common galactolipids are monogalactosyl diacylglycerol and digalactosyl diacylglycerol. Up to 80% of all lipids in plants are associated with photosynthetic membranes, and monogalactosyl diacylglycerol is widely considered to be the most abundant membrane lipid on earth.
Monogalactosyldiacylglycerols are not solely plant lipids as they have been found in small amounts in brain and nervous tissue in some animal species.
Related compounds of those main components of plant glycolipids, e.g. mono- and digalactosyldiacylglycerols, have been found with up to four galactose units, or in which one or more of these is replaced by glucose moieties. In addition, a 6-*O*-acyl-monogalactosyldiacylglycerol is occasionally a component of plant tissues.

**[0004.0.22.4]** The final step in monogalactosyl diacylglycerol biosynthesis occurs in the plastid envelope and is catalyzed by monogalactosyl diacylglycerol synthase (EC 2.4.1.46). This enzyme transfers D-galactose from UDP-galactose to *sn*-1,2-diacylglycerol (DAG) (Joyard, J. & Douce, R. Stumpf, P. K., ed. (1987) in Biochemistry of Plants (Academic, New York)).
Digalactosyl diacylglycerol synthase catalyzes the transfer of galactose from one molecule of monogalactosyl diacylglycerol to another, producing digalactosyl diacylglycerol and DAG in equimolar amounts.

**[0005.0.22.22]** Even if some details are known, galactolipid biosynthesis in plants is highly complex. It involves multiple pathways giving rise to different molecular species.
Recent studies indicate that the amounts of the lipids sulfolipid sulfoquinovosyl-diacylglycerol (SQDG) and digalactosyldiacylglycerol and DGDG increase strongly during phosphate deprivation (Härtel et al., Proc. Natl. Acad. Sci., 97, 10649-10654, 2000). When phosphate is limiting, phospholipids in plant membranes are reduced and at least in part replaced by glycolipids (i.e., SQDG and DGDG).
In addition to serving as a surrogate lipid for phospholipids, galactolipids were found to be critical for the stabilization of photosynthetic complexes in the thylakoids (Dörmann and Benning, Trends Plant Sci. 7, 112-118, 2002).

**[0006.0.22.22]** In contrast to plants, which contain high amounts of glycolipids, which carry a sugar moiety in the head group, in animals and yeast phospholipids are very abundant. Nevertheless, one type of glycolipids to be found in mammalians are galactosylceramide (cerebroside), which is prevalent in brain and the central nervous system. The cerebrosides have been localized to the outer leaflet of the plasma membrane, exposed on the cell surface. They seem to be responsible for the different blood types. Blood group antigens include cerebrosides with multiple sugars attached.

**[0007.0.22.22.]** It has long been recognized that many complex glycolipid antigens are involved in the binding of lectins and antibodies at the cell surface glycolipids, containing only a single sugar headgroup, may play a combination of immunological, regulatory and structural roles in the membrane (Varki et al., Essentials of Glycobiology. Cold Spring Harbor Laboratory Press, New York, 1999).
The glycosphingolipid, galactosylceramide, has been shown to be a key activator of triggered cell death (Zhao et al., Cancer Res. 59, 482-486,1999) and may play a role in the inhibition of virus replication (Kakimi et al., J. Exp. Med. 192, 921-930, 2000).
It has recently been demonstrated that galactolipids are also responsible for preventing cell damage and the high resistance to oxidation and heat in the membranes of some microorganisms (Nakata, J. Biochem. 127, 731-737, 2000).

**[0008.0.22.22.]** Other glycoglycerolipids, such as the 1,2-di-*O*acyl- 3-*O*-(D-galactopyranosyl)-*sn*-glycerols, are found widely in nature as structural components of the photosynthetic membranes of higher plants in the cell membranes of prokaryotic blue-green algae and several other microorganisms and in the seeds of cereals, such as wheat and oats.

**[0009.0.22.22.]** Galactolipids are one of the more abundant lipid classes in nature. Sources for the galactolipids are foodstuffs, such as certain grains (oat, wheat, barley, and maize), which have been a significant part of the human diet since the beginning of time.
In addition galactolipids contain important fatty acids like linoleic acid, linolenic acids and others which have numerous applications in the food and feed industry, in cosmetics and in the drug sector. For example for cyanobacterium and marine green algae fermentation it has been described that the gamma-linolenic acid (GLA) was restricted the the galactolipid fraction (Cohen et al., J.Appl.Phycol.; (1993) 5, 1, 109-15; FEMS-Microbiol.Lett.; (1993) 107, 2-3, 163-67), meaning that increasing the concentration of galactolipids can be another method for increasing the concentration of interesting fatty acids like linoleic acid, linolenic acid, stearic acid and palmitic acid in some production systems.

**[0010.0.22.22]** Most vegetables and fruits in human and animal diets contain galactolipids, and their breakdown products represent an important dietary source of galactose and polyunsaturated fatty acids.

**[0011.0.22.22]** On account of the positive properties and interesting physiological roles potential of galactose and galactose comprising lipids there is a need to produce those compounds in large amounts and well defined quality and composition.

**[0012.0.22.22.]** Thus, it would be desirable to produce galactolipids, in a defined proportion in microorganisms or plants. This should be in way, which is not dependent on the availability of phosphate, in particular on phosphate deprivation. One way to increase the productive capacity of biosynthesis is to apply recombinant DNA technology. That type of production permits control over quality, quantity and selection of the most suitable and efficient producer organisms. The latter is especially important for commercial production economics and therefore availability to consumers. **[0013.0.22.22]** Methods of recombinant DNA technology have been used for some years to improve the production of fine chemicals in microorganisms and plants by amplifying individual biosynthesis genes and investigating the effect on production of fine chemicals.
There is a constant need for providing novel enzyme activities or direct or indirect regulators and thus alternative methods with advantageous properties for producing compounds containing galactose and fatty acids, preferably galactolipids or cerebrosid, in a defined proportion or its precursor in organisms, e.g. in transgenic organisms.

**[0014.0.22.22]** Accordingly, in a first embodiment, the invention relates to a process for the production of a fine chemical, whereby the fine chemical is a lipid, preferably a glycolipide,a glycolipid containing galactose, more preferably a galactolipid or cerebrosid.
Accordingly, in the present invention, the term "the fine chemical" as used herein relates to "lipid, preferably a glycolipid, a glycolipide containing galactose, more preferably a galactolipide and/or cerebroside".
Further, the term "the fine chemicals" as used herein also relates to fine chemicals comprising lipid, preferably a glycolipid, a glycolipide containing galactose, more preferably a galactolipide and/or cerebroside.

**[0015.0.22.22]** In one embodiment, the term "the fine chemical" means lipid, preferably a glycolipid, a glycolipide containing galactose, more preferably a galactolipide and/or cerebroside.
Throughout the specification the term "the fine chemical" means lipid, preferably a glycolipid, a glycolipide containing galactose, more preferably a galactolipide and/or cerebroside in free form or bound to other compounds, such as sulfonated salts. In a preferred embodiment, the term "the fine chemical" means galactolipid, in free form or its salts or bound to sulfate.

**[0015.1.22.22]** A measure for the content of the lipid, preferably a glycolipid, a glycolipide containing galactose, more preferably a galactolipide and/or cerebroside of the invention can be the content of galactopyranoside, preferably methyl-galactopyranosid. This compound is the analyte which indicates the presence of the lipid, preferably a glycolipid, a glycolipide containing galactose, more preferably a galactolipide and/or cerebroside of the invention if the samples are prepared and measured as described in the examples

**[0016.0.22.22]** Accordingly, the present invention relates to a process comprising
(a) increasing or generating the activity of of one or more YLR224W, YLR255C, YER173W, b2699, b3129 and/or YHR072W protein(s), in a non-human organism in one or more parts thereof and
(b) growing the organism under conditions which permit the production of the fine chemical, thus, lipid, preferably a glycolipid, a glycolipide containing galactose, more preferably a galactolipide and/or cerebroside in said organism.
Accordingly, the present invention relates to a process for the production of the respective fine chemical comprising
(a) increasing or generating the activity of one or more protein(s) having the activity of a protein indicated in Table II, column 3, lines 186 to 189 and/or lines 633 and 634 or having the sequence of a polypeptide encoded by a nucleic acid molecule indicated in Table I, columns 5 or 7, and/or lines 633 and 634, in a non-human organism in one or more parts thereof and
(b) growing the organism under conditions which permit the production of the fine chemical, thus, lipid, preferably a glycolipid, a glycolipide containing galactose, more preferably a galactolipide and/or cerebroside.

**[0017.0.0.22] and [0018.0.0.22]** for the disclosure of the paragraphs [0017.0.0.22] and [0018.0.0.22] see paragraphs [0017.0.0.0] and [0018.0.0.0] above.

**[0019.0.22.22]** Advantageously the process for the production of the respective fine chemical leads to an enhanced production of the fine respective chemical. The terms "enhanced" or "increase" mean at least a 10%, 20%, 30%, 40% or 50%, preferably at least 60%, 70%, 80%, 90% or 100%, more preferably 150%, 200%, 300%, 400% or 500% higher production of the respective fine chemical in comparison to the reference as defined below, e.g. that means in comparison to an organism without the aforementioned modification of the activity of a protein having the activity of a protein indicated in Table II, column 3, lines 186 to 189 and/or lines 633 and 634 or encoded by nucleic acid molecule indicated in Table I, columns 5 or 7, lines 186 to 189 and/or lines 633 and 634.

**[0020.0.22.22]** Surprisingly it was found, that the transgenic expression of the Saccaromyces cerevisiae protein(s) YLR224W, YLR255C YER173W and/or YHR072W as indicated in Table II, columns 3 or 5, lines 186 to 188 and 634 respectively and/or the Escherichia coli K12 protein(s) b2699 and/or b3129 as indicated in Table I, columns 3 or 5, lines 189 and 633 in Arabidopsis thaliana conferred an increase in the content of lipid, preferably a glycolipid, a glycolipide containing galactose, more preferably a galactolipide and/or cerebroside of the transformed plants. Thus, in one embodiment, said protein(s) or its homologs as indicated in Table II, column 7, lines 186 to 189 and/or lines 633 and 634 are used for the production of lipid, preferably a glycolipid, a glycolipide containing galactose, more preferably a galactolipide and/or cerebroside.

**[0021.0.0.22]** for the disclosure of this paragraph see [0021.0.0.0] above.

**[0022.0.22.22]** The sequence of YLR224W from Saccharomyces cerevisiae has been published in Johnston et al., Nature 387 (6632 Suppl), 87-90 (1997) and in Goffeau et al., Science 274 (5287), 546-547, 1996 and its cellular activity has not been characterized yet. It seems to have a activity of the Saccharomyces cerevisiae hypothetical protein YLR224w superfamily. Accordingly, in one embodiment, the process of the present invention comprises the use of a gene product with an activity of the Saccharomyces cerevisiae hypothetical protein YLR224w superfamily, preferably a protein with a YLR224w protein activity or its homolog, e.g. as shown herein, from Saccharomyces cerevisiae or its homolog, e.g. as shown herein, for the production of the fine chemical, meaning of lipid, preferably a glycolipid, a glycolipide containing galactose, more preferably a galactolipide and/or cerebroside in free or bound form in an organism or a part thereof, as mentioned. In one further embodiment the YLR224w protein expression is increased together with the increase of another gene of the galactose pathway, preferably with a gene encoding a protein being involved in the production of glycolipide containing galactose, or encoding a galactose transporter protein or a compound, which functions as a sink for the respective lipid, preferably a glycolipid, a glycolipide containing galactose, more preferably a galactolipide and/or cerebroside. In one embodiment, in the process of the present invention the activity of a protein of the Saccharomyces cerevisiae hypothetical protein YLR224w superfamily, preferably of a YLR224w protein is increased or generated, e.g. from Saccharomyces cerevisiae or a homolog thereof.
The sequence of YLR255C from Saccharomyces cerevisiae has been submitted by Johnson et al. to the EMBL Data Library, (February 1995), and its activity has not been characterized yet , but having preferably an activity of Saccharomyces hypothetical protein YLR255c superfamily. Accordingly, in one embodiment, the process of the present invention comprises the use of a gene product with an activity of a protein of the Saccharomyces hypothetical protein YLR255c superfamily, preferably of a gene product with an activity of a YLR255C protein from Saccharomyces cerevisiae or its homolog, e.g. as shown herein, for the production of the respective fine chemical, meaning of lipid, preferably a glycolipid, a glycolipide containing galactose, more preferably a galactolipide and/or cerebroside in free or bound form in an organism or a part thereof, as mentioned. In one further embodiment the YLR255C protein expression is increased together with the increase of another gene of the galactose pathway, preferably with a gene encoding a protein being involved in the production of glycolipid or encoding a galactose transporter protein or a compound, which functions as a sink for the resprective lipid, preferably a glycolipid, a glycolipide containing galactose, more preferably a galactolipide and/or cerebroside. In one embodiment, in the process of the present invention the activity of a Saccharomyces hypothetical protein YLR255c superfamily protein, preferably YLR255C protein, is increased or generated, e.g. from Saccharomyces cerevisiae or a homolog thereof.
The sequence of YER173W from Saccharomyces cerevisiae has been published in Dietrich et al., Nature 387 (6632 Suppl), 78-81, 1997, and Goffeau et al., Science 274 (5287), 546-547, 1996, and its activity is being defined as a checkpoint protein, involved in the activation of the DNA damage and meiotic pachytene checkpoints. Accordingly, in one embodiment, the process of the present invention comprises the use of a gene product with an activity of Checkpoint protein, involved in the activation of the DNA damage and meiotic pachytene checkpoints or its homolog, e.g. as shown herein, for the production of the respective fine chemical, meaning oflipid, preferably a glycolipid, a glycolipide containing galactose, more preferably a galactolipide and/or cerebroside in free or bound form in an organism or a part thereof, as mentioned. In one further embodiment the YER173w protein expression is increased together with the increase of another gene of the galactose pathway, preferably with a gene encoding a protein being involved in the production of lipid, preferably a glycolipid, a glycolipide containing galactose, more preferably a galactolipide and/or cerebroside. In one embodiment, in the process of the present invention the activity of a Checkpoint protein, involved in the activation of the DNA damage and meiotic pachytene checkpoints is increased or generated, e.g. from Saccharomyces cerevisiae or a homolog thereof.
The sequence of b2699 from Escherichia coli K12 has been published in Blattner et al., Science 277(5331), 1453-1474, 1997, and its activity is being defined as a DNA strand exchange and recombination protein with protease and nuclease activity of the superfamily of the recombination protein recA. Accordingly, in one embodiment, the process of the present invention comprises the use of a protein with a DNA recombination and DNA repair activity, a pheromone response activity, a mating-type determination activity, a sex-specific protein activity, a nucleotide binding activity and/or a protease and nuclease activity, in particular a DNA strand exchange and recombination protein with protease and nuclease activity, in particular of the superfamily of the recombination protein recA from E. coli or its homolog, e.g. as shown herein, for the production of the respective fine chemical, meaning lipid, preferably a glycolipid, a glycolipide containing galactose, more preferably a galactolipide and/or cerebroside, preferably in free or bound form in an organism or a part thereof, as mentioned. In one further embodiment the b2699 protein expression is increased together with the increase of another gene of the galactose pathway, preferably with a gene encoding a protein being involved in the production of lipid, preferably a glycolipid, a glycolipide containing galactose, more preferably a galactolipide and/or cerebroside. In one embodiment, in the process of the present invention said activity, e.g. the activity of a protease and nuclease activity, in particular a DNA strand exchange and recombination protein with protease and nuclease activity, in particular of the superfamily of the recombination protein recA is increased or generated, e.g. from E. coli or a homolog thereof.
The sequence of YHR072W (Accession number NP_011939) from Saccharomyces cerevisiae has been published in Goffeau et al., Science 274 (5287), 546-547 (1996) and Johnston et al. Science 265 (5181), 2077-2082 (1994), and its activity is being defined as a 2,3-oxidosqualene-lanosterol cyclase protein. Accordingly, in one embodiment, the process of the present invention comprises the use of an uncharacterized stress-induced protein from Saccharomyces cerevisiae or its homolog, e.g. as shown herein, for the production of the fine chemical, meaning lipid, preferably a glycolipid, a glycolipide containing galactose, more preferably a galactolipide and/or cerebroside, preferably in free or bound form in an organism or a part thereof, as mentioned. In one embodiment, in the process of the present invention the activity of a 2,3-oxidosqualene-lanosterol cyclase protein is increased or generated, e.g. from Saccharomyces cerevisiae or a homolog thereof.
The sequence of b3129 (Accession number NP_417598) from Escherichia coli K12 has been published in Blattner et al., Science 277 (5331), 1453-1474, 1997, and its activity is being defined as a putative protease; htrA suppressor protein. Accordingly, in one embodiment, the process of the present invention comprises the use of a putative protease; htrA suppressor protein from E. coli or its homolog, e.g. as shown herein, for the production of the fine chemical, meaning lipid, preferably a glycolipid, a glycolipide containing galactose, more preferably a galactolipide and/or cerebroside, preferably in free or bound form in an organism or a part thereof, as mentioned. In one embodiment, in the process of the present invention the activity of a putative protease; htrA suppressor protein is increased or generated, e.g. from E. coli or a homolog thereof.

**[0023.0.22.22]** Homologues (= homologs) of the present gene products can be derived from any organisms as long as the homologue confers the herein mentioned activity, in particular, confers an increase in the respective fine chemical amount or content.
Further, in the present invention, the term "homologue" relates to the sequence of an organism having the highest sequence homology to the herein mentioned or listed sequences of all expressed sequences of said organism. However, the person skilled in the art knows, that, preferably, the homologue has said the-fine-chemical-increasing activity and, if known, the same biological function or activity in the organism as at least one of the protein(s) indicated in Table I, Column 3, lines 186 to 188 and 634, e.g. having the sequence of a polypeptide encoded by a nucleic acid molecule comprising the sequence indicated in indicated in Table I, Column 5 or 7, lines 186 to 188 and 634. In one embodiment, the homolog of any one of the polypeptides indicated in Table II, lines 186 to 188 and 634 is a homolog having the same or a similar activity, in particular an increase of activity confers an increase in the content of the fine chemical in the organisms and being derived from eukaryot.
In one embodiment, the homolog of a polypeptide indicated in Table II, column 3, line 189 and 633 is a homolog having the same or a similar activity, in particular an increase of activity confers an increase in the content of the fine chemical in the organisms or part thereof, and being derived from bacteria.
In one embodiment, the homolog of a polypeptide indicated in Table II, column 3, lines 186 to 188 and 634 is a homolog having the same or a similar activity, in particular an increase of activity confers an increase in the content of the fine chemical in an organisms or part thereof, and being derived from Fungi.
In one embodiment, the homolog of a polypeptide indicated in Table II, column 3, line 189 and 633 is a homolog having the same or a similar activity, in particular an increase of activity confers an increase in the content of the fine chemical in the organisms or part thereof and being derived from Proteobacteria.
In one embodiment, the homolog of a polypeptide indicated in Table II, column 3, lines 186 to 188 and 634 is a homolog having the same or a similar activity, in particular an increase of activity confers an increase in the content of the fine chemical in the organisms or a part thereof and being derived from Ascomycota.
In one embodiment, the homolog of a polypeptide indicated in Table II, column 3, line 189 and 633 is a homolog having the same or a similar activity, in particular an increase of activity confers an increase in the content of the fine chemical in the organisms or part thereof, and being derived from Gammaproteobacteria.
In one embodiment, the homolog of a polypeptide polypeptide indicated in Table II, column 3, lines 186 to 188 and 634 is a homolog having the same or a similar activity, in particular an increase of activity confers an increase in the content of the fine chemical in the organisms or part thereof, and being derived from Saccharomycotina.
In one embodiment, the homolog of a polypeptide indicated in Table II, column 3, line 189 and 633 is a homolog having the same or a similar activity, in particular an increase of activity confers an increase in the content of the fine chemical in the organisms or part thereof, and being derived from Enterobacteriales.
In one embodiment, the homolog of a polypeptide indicated in Table II, column 3, lines 186 to 188 and 634 is a homolog having the same or a similar activity, in particular an increase of activity confers an increase in the content of the fine chemical in the organisms or a part thereof, and being derived from Saccharomycetes.
In one embodiment, the homolog of the a polypeptide indicated in Table II, column 3, line 189 and 633 is a homolog having the same or a similar activity, in particular an increase of activity confers an increase in the content of the fine chemical in the organisms or part thereof, and being derived from Enterobacteriaceae.
In one embodiment, the homolog of a polypeptide indicated in Table II, column 3, lines 186 to 188 and 634 is a homolog having the same or a similar activity, in particular an increase of activity confers an increase in the content of the fine chemical in the organisms, and being derived from Saccharomycetales.
In one embodiment, the homolog of a polypeptide indicated in Table II, column 3, lines 189 and 633 is a homolog having the same or a similar activity, in particular an increase of activity confers an increase in the content of the fine chemical in the organisms or a part thereof, and being derived from Escherichia.
In one embodiment, the homolog of a polypeptide indicated in Table II, column 3, lines 186 to 188 and 634 is a homolog having the same or a similar activity, in particular an increase of activity confers an increase in the content of the fine chemical in the organisms or a part thereof, and being derived from Saccharomycetaceae.
In one embodiment, the homolog of a polypeptide indicated in Table II, column 3, line 186 to 188 and 634 is a homolog having the same or a similar acivity, in particular an increase of activity confers an increase in the content of the fine chemical in the organisms or a part thereof, and being derived from Saccharomycetes.

**[0023.1.22.22]** Homologs of the polypeptides polypeptide indicated in Table II, column 3, lines 186 to 189 and/or lines 633 and 634 may be the polypetides encoded by the nucleic acid molecules polypeptide indicated in Table I, column 7, lines 186 to 189 and/or lines 633 and/or 634 or may be the polypeptides indicated in Table II, column 7, lines 186 to 189. Homologs of the polypeptides indicated in Table II, column 3, lines 186 to 189 and/or lines 633 and/or 634 may be the polypetides encoded by the nucleic acid molecules polypeptide indicated in Table I, column 7, lines 186 to 189 and/or lines 633 and/or 634 or may be the polypeptides indicated in Table II, column 7, lines 186 to 189 and/or lines 633 and/or 634.

**[0024.0.0.22]** for the disclosure of this paragraph see [0024.0.0.0] above.

**[0025.0.22.22]** In accordance with the invention, a protein or polypeptide has the "activity of an protein of the invention", e.g. the activity of a protein indicated in Table II, column 3, lines 186 to 189 and/or lines 633 and 634 if its *de novo* activity, or its increased expression directly or indirectly leads to an increased level of lipid, preferably a glycolipid, a glycolipide containing galactose, more preferably a galactolipide and/or cerebroside in the organism or a part thereof, preferably in a cell of said organism. In a preferred embodiment, the protein or polypeptide has the above-mentioned additional activities of a protein indicated in Table II, column 3, lines 186 to 189 and/or lines 633 and 634. During the specification the activity or preferably the biological activity of such a protein or polypeptide or an nucleic acid molecule or sequence encoding such protein or polypeptide is identical or similar if it still has the biological or enzymatic activity of any one of the proteins indicated in Table II, column 3, lines 186 to 189 and/or lines 633 and 634, i.e. if it has at least 10% of the original enzymatic activity, preferably 20%, particularly preferably 30%, most particularly preferably 40% in comparison to an any one of the proteins indicated in Table II, column 3, lines 186 to 188 and/or 634 of Saccharomyces cerevisiae and/or any one of the proteins indicated in Table II, column 3, line 189 and/or 633 of E. coli K12.

**[0025.1.0.22] and [0025.2.0.22]** for the disclosure of the paragraphs [0025.1.0.22] and [0025.2.0.22] see [0025.1.0.0] and [0025.2.0.0] above.

**[0026.0.0.22] to [0033.0.0.22]** for the disclosure of the paragraphs [0026.0.0.22] to [0033.0.0.22] see [0026.0.0.0] to [0033.0.0.0] above.

**[0034.0.22.22]** Preferably, the reference, control or wild type differs form the subject of the present invention only in the cellular activity of the polypeptide of the invention, e.g. as result of an increase in the level of the nucleic acid molecule of the present invention or an increase of the specific activity of the polypeptide of the invention. E.g., it differs by or in the expression level or activity of an protein having the activity of a protein as indicated in Table II, column 3, lines 186 to 189 and/or lines 633 and/or 634 or being encoded by a nucleic acid molecule indicated in Table I, column 5, lines 186 to 189 and/or lines 633 and/or 634 or its homologs, e.g. as indicated in Table I, column 7, lines 186 to 189 and/or lines 633 and/or 634, its biochemical or genetical causes and therefore shows the increased amount of the fine chemical.

**[0035.0.0.22] and [0036.0.0.22]** for the disclosure of the paragraphs [0035.0.0.22] and [0036.0.0.22] see paragraphs [0035.0.0.0] to [0036.0.0.0] above.

**[0037.0.22.22]** A series of mechanisms exists via which a modification of the a protein, e.g. the polypeptide of the invention can directly or indirectly affect the yield, production and/or production efficiency of the lipid, preferably a glycolipid, a glycolipide containing galactose, more preferably a galactolipide and/or cerebroside.

**[0038.0.0.22] to [0044.0.0.22]** for the disclosure of the paragraphs [0038.0.0.22] to [0044.0.0.22] see paragraphs [0038.0.0.0] to [0044.0.0.0] above.

**[0045.0.22.22]** In one embodiment, in case the activity of the Saccaromyces cerevisiae protein YLR224W or its homologs, e.g. as indicated in Table II, columns 5 or 7, line 186, is increased, preferably, an increase of the fine chemical between 15% and 46% or more is conferred.
In one embodiment, in case the activity of the Saccaromyces cerevisiae protein YLR255C or its homologs, e.g. as indicated in Table II, columns 5 or 7, line 187, is increased, preferably, in one embodiment the increase of the fine chemical between 16% and 30% or more is conferred.
In one embodiment, in case the activity of the Saccaromyces cerevisiae protein YER173w or its homologs, e.g. as indicated in Table II, columns 5 or 7, line 188, e.g. a checkpoint protein, involved in the activation of the DNA damage and meiotic pachytene checkpoints; is increased, preferably, the increase of the fine chemical between 20% and 70% or more is conferred.
In one embodiment, in case the activity of the Escherichia coli K12 protein b2699 or its homologs, e.g. as indicated in Table II, columns 5 or 7, line 189, is increased, e.g. the activity of a DNA strand exchange and recombination protein with protease and nuclease activity of the superfamily of the recombination protein recA or its homolog is increased preferably, the increase of the fine chemical between 15% and 64% or more is conferred.
In one embodiment, in case the activity of the Escherichia coli K12 protein b3129 or its homologs, e.g. as indicated in Table II, columns 5 or 7, line 633, is increased, e.g. the activity of a putative protease; htrA suppressor protein or its homolog is increased preferably, the increase of the fine chemical between 16% and 32% or more is conferred.
In one embodiment, in case the activity of the Saccaromyces cerevisiae protein YHR072W or its homologs, e.g. as indicated in Table II, columns 5 or 7, line 634, is increased, e.g. the activity of a 2,3-oxidosqualene-lanosterol cyclase protein or its homolog is increased, preferably, the increase of the fine chemical between 17% and 35% or more is conferred.

**[0046.0.22.22]** In one embodiment, the activity of the Saccaromyces cerevisiae protein YLR224W or its homologs, e.g. an activity of a YLR224W protein, e.g. as indicated in Table II, columns 5 or 7, line 186, confers an increase of the respective fine chemical and of further lipids, preferably glycolipids, sphingolipids and/or galactolipids, or their precursors.
In one embodiment, the activity of the Saccaromyces cerevisiae protein YLR255C or its homologs, e.g. an activity of a YLR255C protein, e.g. as indicated in Table II, columns 5 or 7, line 187, confers an increase of the respective fine chemical and of further lipids, preferably glycolipids, sphingolipids and/or galactolipids, or their precursors.
In one embodiment, the activity of the Saccaromyces cerevisiae protein YER173W or its homologs, e.g. an activity of a checkpoint protein, involved in the activation of the DNA damage and meiotic pachytene checkpoints, e.g. as indicated in Table II, columns 5 or 7, line 188, confers an increase of the respective fine chemical and of further lipids, preferably glycolipids, sphingolipids and/or galactolipids, or their precursors.
In one embodiment, the activity of the Escherichia coli K12 protein b2699 or its homologs, e.g. an activity of a DNA strand exchange and recombination protein with protease and nuclease activity of the superfamily of the recombination protein recA, e.g. as indicated in Table II, columns 5 or 7, line 189, confers an increase of the respective fine chemical and of further lipids, preferably glycolipids, sphingolipids and/or galactolipids, compounds or their precursors.
In one embodiment, the activity of the Escherichia coli K12 protein b3129 or its homologs, e.g. a protein having the activity of a putative protease; htrA suppressor protein, e.g. as indicated in Table II, columns 5 or 7, line 633, confers an increase of the respective fine chemical and of further lipids, preferably glycolipids, sphingolipids and/or galactolipids, compounds or their precursors.
In one embodiment, the activity of the Saccaromyces cerevisiae protein YHR072W or its homologs, e.g. a 2,3-oxidosqualene-lanosterol cyclase protein, e.g. as indicated in Table II, columns 5 or 7, line 634, confers an increase of the respective fine chemical and of further lipids, preferably glycolipids, sphingolipids and/or galactolipids, or their precursors.

**[0047.0.0.22] and [0048.0.0.22]** for the disclosure of the paragraphs [0047.0.0.22] and [0048.0.0.22] see paragraphs [0047.0.0.0] and [0048.0.0.0] above.

**[0049.0.22.22]** A protein having an activity conferring an increase in the amount or level of the fine chemical preferably has the structure of the polypeptide described herein, in particular of a polypeptides comprising a consensus sequence as indicated in Table IV, column 7, lines 186 to 189 and/or lines 633 and/or 634 or of a polypeptide as indicated in Table II, columns 5 or 7, lines 186 to 189 and/or lines 633 and/or 634 or the functional homologues thereof as described herein, or is encoded by the nucleic acid molecule characterized herein or the nucleic acid molecule according to the invention, for example by a nucleic acid molecule as indicated in Table I, columns 5 or 7, lines 186 to 189 and/or lines 633 and/or 634 or its herein described functional homologues and has the herein mentioned activity.

**[0050.0.22.22]** For the purposes of the present invention, the term "lipid, preferably a glycolipid, a glycolipide containing galactose, more preferably a galactolipide and/or cerebroside" also encompasses monogalactosyl diacylglycerol and/or digalactosyl diacylglycerol and/or galactolipids comprising 3, 4 or more galactosyl units.

**[0051.0.22.22]** Owing to the biological activity of the proteins which are used in the process according to the invention and which are encoded by nucleic acid molecules according to the invention, it is possible to produce compositions comprising the fine chemical, i.e. an increased amount of the fine chemical free or bound, e.g compositions comprising lipid, preferably a glycolipid, a glycolipide containing galactose, more preferably a galactolipide and/or cerebroside. Depending on the choice of the organism used for the process according to the present invention, for example a microorganism or a plant, compositions or mixtures of various fatty acids can be produced. **[0052.0.0.22]** for the disclosure of this paragraph see paragraph [0052.0.0.0] above.

**[0053.0.22.22]** In one embodiment, the process of the present invention comprises one or more of the following steps
a) stabilizing a protein conferring the increased expression of a protein encoded by the nucleic acid molecule of the invention or of the polypeptide of the invention, e.g. of a polypeptide having an activity of a protein as indicated in Table II, column 3, lines 186 to 189 and/or lines 633 and 634 or its homologs activity, e.g. as indicated in Table II, columns 5 or 7, lines 186 to 189 and/or lines 633 and 634, having herein-mentioned the respective fine chemical-increasing activity;
b) stabilizing a mRNA conferring the increased expression of a protein encoded by the nucleic acid molecule of the invention, e.g. of a polypeptide having an activity of a protein as indicated in Table II, column 3, lines 186 to 189 and/or lines 633 and 634, or its homologs activity, e.g. as indicated in Table II, columns 5 or 7, lines 186 to 189 and/or lines 633 and 634, or of a mRNA encoding the polypeptide of the present invention having herein-mentioned the respective fine chemical increasing activity;
c) increasing the specific activity of a protein conferring the increased expression of a protein encoded by the nucleic acid molecule of the invention or of the polypeptide of the present invention having herein-mentioned the respective fine chemical increasing activity, e.g. of a polypeptide having an activity of a protein as indicated in Table II, column 3, lines 186 to 189 and/or lines 633 and 634 or its homologs activity, e.g. as indicated in Table II, columns 5 or 7, lines 186 to 189 and/or lines 633 and 634, or decreasing the inhibitory regulation of the polypeptide of the invention;
d) generating or increasing the expression of an endogenous or artificial transcription factor mediating the expression of a protein conferring the increased expression of a protein encoded by the nucleic acid molecule of the invention or of the polypeptide of the invention having herein-mentioned the respective fine chemical increasing activity, e.g. of a polypeptide having an activity of a protein as indicated in Table II, column 3, lines 186 to 189 and/or lines 633 and 6349, or its homologs activity, e.g. as indicated in Table II, columns 5 or 7, lines 186 to 189 and/or lines 633 and 634;
e) stimulating activity of a protein conferring the increased expression of a protein encoded by the nucleic acid molecule of the present invention or a polypeptide of the present invention having herein-mentioned the respective fine chemical increasing activity, e.g. of a polypeptide having an activity of a protein as indicated in Table II, column 3, lines 186 to 189 and/or lines 633 and 634, or its homologs activity, e.g. as indicated in Table II, columns 5 or 7, lines 186 to 189 and/or lines 633 and 634, by adding one or more exogenous inducing factors to the organism or parts thereof;
f) expressing a transgenic gene encoding a protein conferring the increased expression of a polypeptide encoded by the nucleic acid molecule of the present invention or a polypeptide of the present invention, having herein-mentioned the respective fine chemical increasing activity, e.g. of a polypeptide having an activity of a protein as indicated in Table II, column 3, lines 186 to 189 and/or lines 633 and 634, or its homologs activity, e.g. as indicated in Table II, columns 5 or 7, lines 186 to 189 and/or lines 633 and 634;
g) increasing the copy number of a gene conferring the increased expression of a nucleic acid molecule encoding a polypeptide encoded by the nucleic acid molecule of the invention or the polypeptide of the invention having herein-mentioned the respective fine chemical increasing activity, e.g. of a polypeptide having an an activity of a protein as indicated in Table II, column 3, lines 186 to 189 and/or lines 633 and 634, or its homologs activity, e.g. as indicated in Table II, columns 5 or 7, lines 186 to 189 and/or lines 633 and 634;
h) Increasing the expression of the endogenous gene encoding the polypeptide of the invention, e.g. a polypeptide having an activity of a protein as indicated in Table II, column 3, lines 186 to 189 and/or lines 633 and 634, or its homologs activity, e.g. as indicated in Table II, columns 5 or 7, lines 186 to 189 and/or lines 633 and 634 by adding positive expression or removing negative expression elements, e.g. homologous recombination can be used to either introduce positive regulatory elements like for plants the 35S enhancer into the promoter or to remove repressor elements form regulatory regions. Further gene conversion methods can be used to disrupt repressor elements or to enhance to activity of positive elements. Positive elements can be randomly introduced in plants by T-DNA or transposon mutagenesis and lines can be identified in which the positive elements have be integrated near to a gene of the invention, the expression of which is thereby enhanced;
i) Modulating growth conditions of an organism in such a manner, that the expression or activity of the gene encoding the protein of the invention or the protein itself is enhanced for example microorganisms or plants can be grown under a higher temperature regime leading to an enhanced expression of heat shock proteins, e.g. the heat shock protein of the invention, which can lead an enhanced the fine chemical production; and/or
j) selecting of organisms with especially high activity of the proteins of the invention from natural or from mutagenized resources and breeding them into the target organisms, eg the elite crops.

**[0054.0.22.22]** Preferably, said mRNA is the nucleic acid molecule of the present invention and/or the protein conferring the increased expression of a protein encoded by the nucleic acid molecule of the present invention or the polypeptide having the herein mentioned activity is the polypeptide of the present invention, e.g. conferring the increase of the respective fine chemical after increasing the expression or activity of the encoded polypeptide or having the activity of a polypeptide having an activity of a protein according to Table II, column 3, lines 186 to 189 and/or lines 633 and 634 or its homologs activity, e.g. as indicated in Table II, columns 5 or 7, lines 186 to 189 and/or lines 633 and 634.

**[0055.0.0.22] to [0067.0.0.22]** for the disclosure of the paragraphs [0055.0.0.22] to [0067.0.0.22] see paragraphs [0055.0.0.0] to [0067.0.0.0] above.

**[0068.0.22.22]** The mutation is introduced in such a way that the production of lipid, preferably a glycolipid, a glycolipide containing galactose, more preferably a galactolipide and/or cerebroside is not adversely affected.

**[0069.0.22.22]** Less influence on the regulation of a gene or its gene product is understood as meaning a reduced regulation of the enzymatic activity leading to an increased specific or cellular activity of the gene or its product. An increase of the enzymatic activity is understood as meaning an enzymatic activity, which is increased by at least 10%, advantageously at least 20, 30 or 40%, especially advantageously by at least 50, 60 or 70% in comparison with the starting organism. This leads to an increased productivity of the desired lipid, preferably a glycolipid, a glycolipide containing galactose, more preferably a galactolipide and/or cerebroside containing compound(s).

**[0070.0.22.22]** Owing to the introduction of a gene or a plurality of genes conferring the expression of the nucleic acid molecule of the invention or the polypeptide of the invention, for example the nucleic acid construct mentioned below into an organism alone or in combination with other genes, it is possible not only to increase the biosynthetic flux towards the end product, but also to increase, modify or create de novo an advantageous, preferably novel metabolites composition in the organism, e.g. an advantageous lipid, preferably a glycolipid, a glycolipide containing galactose, more preferably a galactolipide and/or cerebroside containing composition comprising a higher content of (from a viewpoint of nutritonal physiology limited) lipid, preferably a glycolipid, a glycolipide containing galactose, more preferably a galactolipide and/or cerebroside.

**[0071.0.0.22]** for the disclosure of this paragraph see paragraph [0071.0.0.0] above.

**[0072.0.22.22]** By influencing the metabolism thus, it is possible to produce, in the process according to the invention, further advantageous compounds. Examples of such compounds are, in addition to lipid, preferably a glycolipid, a glycolipide containing galactose, more preferably a galactolipide and/or cerebroside compounds like galacturonic acid, oligo-and/or polysaccharides containing galactose or galacturonic acid, galactosamines, UDP-galactose, psychosine, UDP-N-Acyl-galactosamines, UDP-galacturonates, further glycoproteins, gangliosides, mucins, blood group substances and/or ceramides.

**[0073.0.22.22]** Accordingly, in one embodiment, the process according to the invention relates to a process, which comprises:
a. providing a non-human organism, preferably a microorganism, a non-human animal, a plant or animal cell, a plant or animal tissue or a plant;
b. increasing an activity of a polypeptide of the invention or a homolog thereof, e.g. as indicated in Table II, columns 5 or 7, lines 186 to 189 and/or lines 633 and 634 resp., or of a polypeptide being encoded by the nucleic acid molecule of the present invention and described below, i.e. conferring an increase of the respective fine chemical in the organism, preferably in a microorganism, a non-human animal, a plant or animal cell, a plant or animal tissue or a plant,
c. growing the organism, preferably the microorganism, the non-human animal, the plant or animal cell, the plant or animal tissue or the plant under conditions which permit the production of the fine chemical in the organism, preferably the microorganism, the plant cell, the plant tissue or the plant; and
if desired, recovering, optionally isolating, the free and/or bound the fine chemical and, optionally further free and/or bound lipid, preferably a glycolipid, a glycolipide containing galactose, more preferably a galactolipide and/or cerebroside synthesized by the organism, the microorganism, the non-human animal, the plant or animal cell, the plant or animal tissue or the plant.

**[0074.0.22.22]** The organism, in particular the microorganism, non-human animal, the plant or animal cell, the plant or animal tissue or the plant is advantageously grown in such a way that it is not only possible to recover, if desired isolate the free or bound the fine chemical or the free and bound the fine chemical but as option it is also possible to produce, recover and, if desired isolate, other free or/and bound lipid, preferably a glycolipid, a glycolipide containing galactose, more preferably a galactolipide and/or cerebroside galactose containing compound.

**[0075.0.0.22] to [0077.0.0.22]** for the disclosure of the paragraphs [0075.0.0.22] to [0077.0.0.22] see paragraphs [0075.0.0.0] to [0077.0.0.0] above.

**[0078.0.22.22]** The organism such as microorganisms or plants or the recovered, and if desired isolated, fine chemical can then be processed further directly into foodstuffs or food supplements or animal feeds or for other applications, for example pharmaceutical composition.

**[0079.0.0.22] to [0084.0.0.22]** for the disclosure of the paragraphs [0079.0.0.22] to [0084.0.0.22] see paragraphs [0079.0.0.0] to [0084.0.0.0] above.

**[0085.0.22.22]** With regard to the nucleic acid sequence as depicted a nucleic acid construct which contains a nucleic acid sequence mentioned herein or an organism (= transgenic organism) which is transformed with said nucleic acid sequence or said nucleic acid construct, "transgene" means all those constructs which have been brought about by genetic manipulation methods,preferably in which either
a) the nucleic acid sequence as shown in table I, columns 5 or 7, lines 186 to 189 and/or lines 633 and 634 or a derivative thereof, or
b) a genetic regulatory element, for example a promoter, which is functionally linked to the nucleic acid sequence as shown table I, columns 5 or 7, lines 186 to 189 and/or lines 633 and 634 or a derivative thereof, or
c) (a) and (b)
is/are not present in its/their natural genetic environment or has/have been modified by means of genetic manipulation methods, it being possible for the modification to be, by way of example, a substitution, addition, deletion, inversion or insertion of one or more nucleotide. "Natural genetic environment" means the natural chromosomal locus in the organism of origin or the presence in a genomic library. In the case of a genomic library, the natural, genetic environment of the nucleic acid sequence is preferably at least partially still preserved. The environment flanks the nucleic acid sequence at least on one side and has a sequence length of at least 50 bp, preferably at least 500 bp, particularly preferably at least 1000 bp, very particularly preferably at least 5000 bp.

**[0086.0.0.22] and [0087.0.0.22]** for the disclosure of the paragraphs [0086.0.0.22] and [0087.0.0.22] see paragraphs [0086.0.0.0] and [0087.0.0.0] above.

**[0088.0.22.22]** In an advantageous embodiment of the invention, the organism takes the form of a plant whose whose lipid, preferably a glycolipid, a glycolipide containing galactose, more preferably a galactolipide and/or cerebrosid content is modified advantageously owing to the nucleic acid molecule of the present invention expressed. This is important for plant breeders since, for example, the nutritional value of plants for animals is dependent on the abovementioned lipids and the general amount of glycolipids containing galactose as energy source in feed. After the above mentioned protein activity has been increased or generated, or after the expression of nucleic acid molecule or polypeptide according to the invention has been generated or increased, the transgenic plant generated thus is grown on or in a nutrient medium or else in the soil and subsequently harvested.

**[0088.1.0.22]** for the disclosure of this paragraph see paragraph [0088.1.0.0] above.

**[0089.0.0.22] to [0094.0.0.22]** for the disclosure of the paragraphs [0089.0.0.22] to [0094.0.0.22] see paragraphs [0089.0.0.0] to [0094.0.0.0] above.

**[0095.0.22.22]** It may be advantageous to increase the pool of lipids, preferably glycolipids, glycolipids containing galactose, more preferably galactolipids and/or cerebrosids in the transgenic organisms by the process according to the invention in order to isolate high amounts of the pure respective fine chemical and/or to obtain increased resistance against biotic and abiotic stresses and to obtain higher yield.

**[0096.0.22.22]** In another preferred embodiment of the invention a combination of the increased expression of the nucleic acid sequence or the protein of the invention together with the transformation of a protein or polypeptide for example a fatty acid transporter protein or a compound, which functions as a sink for the desired lipid or compound containing galactose in the organism is useful to increase the production of the fine chemical.

**[0097.0.0.22]** for the disclosure of this paragraph see paragraph [0097.0.0.0] above.

**[0098.0.22.22]** In a preferred embodiment, the fine chemical (lipid, preferably a glycolipid, a glycolipide containing galactose, more preferably a galactolipide and/or cerebrosid) is produced in accordance with the invention and, if desired, is isolated. The production of further lipids, fatty acids and/or oligosaccharides and/or mixtures thereof or mixtures of other fatty acids by the process according to the invention is advantageous.

**[0099.0.22.22]** In the case of the fermentation of microorganisms, the above mentioned lipid, preferably a glycolipid, a glycolipide containing galactose, more preferably a galactolipide and/or cerebrosid may accumulate in the medium and/or the cells. If microorganisms are used in the process according to the invention, the fermentation broth can be processed after the cultivation. Depending on the requirement, all or some of the biomass can be removed from the fermentation broth by separation methods such as, for example, centrifugation, filtration, decanting or a combination of these methods, or else the biomass can be left in the fermentation broth. The fermentation broth can subsequently be reduced, or concentrated, with the aid of known methods such as, for example, rotary evaporator, thin-layer evaporator, falling film evaporator, by reverse osmosis or by nanofiltration. Afterwards advantageously further compounds for formulation can be added such as corn starch or silicates. This concentrated fermentation broth advantageously together with compounds for the formulation can subsequently be processed by lyophilization, spray drying, spray granulation or by other methods. Preferably the fine chemical of the invention compositions are isolated from the organisms, such as the microorganisms or plants or the culture medium in or on which the organisms have been grown, or from the organism and the culture medium, in the known manner, for example via extraction, distillation, crystallization, chromatography or a combination of these methods. These purification methods can be used alone or in combination with the aforementioned methods such as the separation and/or concentration methods.

**[0100.0.22.22]** Transgenic plants which comprise the lipid synthesized in the process according to the invention can advantageously be marketed directly without there being any need for the oils, lipids or fatty acids synthesized to be isolated. Plants for the process according to the invention are listed as meaning intact plants and all plant parts, plant organs or plant parts such as leaf, stem, seeds, root, tubers, anthers, fibers, root hairs, stalks, embryos, calli, cotelydons, petioles, harvested material, plant tissue, reproductive tissue and cell cultures which are derived from the actual transgenic plant and/or can be used for bringing about the transgenic plant. In this context, the seed comprises all parts of the seed such as the seed coats, epidermal cells, seed cells, endosperm or embryonic tissue. However, the fine chemical produced in the process according to the invention can also be isolated from the organisms, advantageously plants, in the form of their lipids, oligosaccharides and/or free galactolipid. Galactolipids produced by this process can be obtained by harvesting the organisms, either from the crop in which they grow, or from the field. This can be done via pressing or extraction of the plant parts, preferably the plant seeds. To allow for greater ease of disruption of the plant parts, specifically the seeds, they are previously comminuted, steamed or roasted. The seeds or plants, which have been pretreated in this manner can subsequently be pressed or extracted with solvents or hydrolysed under acidic or basic conditions or via enzymatic cleavage. The solvent is subsequently removed. In the case of microorganisms, the latter are, after harvesting, for example extracted directly without further processing steps or else, after disruption, extracted via various methods with which the skilled worker is familiar. In this manner, more than 96% of the compounds produced in the process can be isolated. Thereafter, the resulting products are processed further, i.e. refined. If desired the resulting product can be washed thoroughly with water to remove traces of acid or alkali remaining in the product and then dried. To remove the pigment remaining in the product, the products can be subjected to bleaching, for example using filler's earth or active charcoal. At the end, the product can be deodorized, for example using steam distillation under vacuum. These chemically pure lipids, preferably glycolipids, or glycolipids containing galactose, more preferably a galactolipids and/or cerebrosids compositions are advantageous for applications in the food industry sector, the cosmetic sector and especially the pharmacological industry sector.

**[0101.0.22.22]** for the disclosure of this paragraph see paragraph [0101.0.0.0] above.

**[0102.0.22.22]** Lipids, preferably glycolipids, or glycolipids containing galactose, more preferably galactolipids and/or cerebrosides can for example be detected advantageously by the means of GC separation methods following a suitable sample preparation. The unambiguous detection for the presence of lipids, preferably glycolipids, or glycolipids containing galactose, more preferably galactolipids and/or cerebrosides can be obtained by analyzing recombinant organisms using analytical standard methods: GC, GC-MS or TLC, as described on several occasions by Christie and the references therein (1997, in: Advances on Lipid Methodology, Fourth Edition: Christie, Oily Press, Dundee, 119-169; 1998, Gaschromatographie-Massenspektrometrie-Verfahren [Gas chromatography/mass spectrometric methods], Lipide 33:343-353). The material to be analyzed can be disrupted by sonication, grinding in a glass mill, liquid nitrogen and grinding or via other applicable methods. After disruption, the material must be centrifuged. The sediment is resuspended in distilled water, heated for 10 minutes at 100°C, cooled on ice and recentrifuged, followed by extraction for one hour at 90°C in 0.5 M sulfuric acid in methanol with 2% dimethoxypropane, which leads to hydrolyzed oil and lipid compounds, which give transmethylated lipids (fatty acid methyl esters and the respective complements as glycerol, carbohydrates, phosphates). These fatty acid methyl esters are extracted in petroleum ether and finally subjected to a GC analysis using a capillary column (Chrompack, WCOT Fused Silica, CP-Wax-52 CB, 25 µm, 0.32 mm) at a temperature gradient of between 170°C and 240°C for 20 minutes and 5 minutes at 240°C. The identity of the resulting fatty acid methyl esters must be defined using standards, which are available from commercial sources (i.e. Sigma).

**[0103.0.22.22]** In a preferred embodiment, the present invention relates to a process for the production of the fine chemical comprising or generating in an organism or a part thereof the expression of at least one nucleic acid molecule comprising a nucleic acid molecule selected from the group consisting of:
a) nucleic acid molecule encoding, preferably at least the mature form, of the polypeptide shown in Table II, columns 5 or 7, lines 186 to 189 and/or lines 633 and 634 or a fragment thereof, which confers an increase in the amount of the fine chemical in an organism or a part thereof;
b) nucleic acid molecule comprising, preferably at least the mature form, of the nucleic acid molecule shown in Table I, columns 5 or 7, lines 186 to 189 and/or lines 633 and 634;
c) nucleic acid molecule whose sequence can be deduced from a polypeptide sequence encoded by a nucleic acid molecule of (a) or (b) as result of the degeneracy of the genetic code and conferring an increase in the amount of the fine chemical in an organism or a part thereof;
d) nucleic acid molecule encoding a polypeptide which has at least 50% identity with the amino acid sequence of the polypeptide encoded by the nucleic acid molecule of (a) to (c) and conferring an increase in the amount of the fine chemical in an organism or a part thereof;
e) nucleic acid molecule which hybridizes with a nucleic acid molecule of (a) to (c) under stringent hybridization conditions and conferring an increase in the amount of the fine chemical in an organism or a part thereof;
f) nucleic acid molecule encoding a polypeptide, the polypeptide being derived by substituting, deleting and/or adding one or more amino acids of the amino acid sequence of the polypeptide encoded by the nucleic acid molecules (a) to (d), preferably to (a) to (c) and conferring an increase in the amount of the fine chemical in an organism or a part thereof;
g) nucleic acid molecule encoding a fragment or an epitope of a polypeptide which is encoded by one of the nucleic acid molecules of (a) to (e), preferably to (a) to (c) and and conferring an increase in the amount of the fine chemical in an organism or a part thereof;
h) nucleic acid molecule comprising a nucleic acid molecule which is obtained by amplifying nucleic acid molecules from a cDNA library or a genomic library using the primers shown in Table III, column 7, lines 186 to 189 and/or lines 633 and 634 and conferring an increase in the amount of the fine chemical in an organism or a part thereof;
i) nucleic acid molecule encoding a polypeptide which is isolated, e.g. from an expression library, with the aid of monoclonal antibodies against a polypeptide encoded by one of the nucleic acid molecules of (a) to (h), preferably to (a) to (c), and conferring an increase in the amount of the fine chemical in an organism or a part thereof;
j) nucleic acid molecule which encodes a polypeptide comprising the consensus sequence shown in Table IV, column 7, lines 186 to 189 and/or lines 633 and 634 and conferring an increase in the amount of the fine chemical in an organism or a part thereof ;
k) nucleic acid molecule comprising one or more of the nucleic acid molecule encoding the amino acid sequence of a polypeptide encoding a domain of the polypeptide shown in Table II, columns 5 or 7, lines 186 to 189 and/or lines 633 and 634 and conferring an increase in the amount of the fine chemical in an organism or a part thereof; and
l) nucleic acid molecule which is obtainable by screening a suitable library under stringent conditions with a probe comprising one of the sequences of the nucleic acid molecule of (a) to (k), preferably to (a) to (c), or with a fragment of at least 15 nt, preferably 20 nt, 30 nt, 50 nt, 100 nt, 200 nt or 500 nt of the nucleic acid molecule characterized in (a) to (k), preferably to (a) to (c), and conferring an increase in the amount of the fine chemical in an organism or a part thereof; or which comprises a sequence which is complementary thereto.

**[00103.1.22.22]** In one embodiment, the nucleic acid molecule used in the process of the invention distinguishes over the sequence indicated in Table IA, columns 5 or 7, lines 186 to 189 and/or lines 633 and 634, by one or more nucleotides. In one embodiment, the nucleic acid molecule used in the process of the invention does not consist of the sequence shown in indicated in Table I A, columns 5 or 7, lines 186 to 189 and/or lines 633 and 634. In one embodiment, the nucleic acid molecule used in the process of the invention is less than 100%, 99,999%, 99,99%, 99,9% or 99% identical to a sequence indicated in Table I A, columns 5 or 7, lines 186 to 189 and/or lines 633 and 634. In another embodiment, the nucleic acid molecule does not encode a polypeptide of a sequence indicated in Table II A, columns 5 or 7, lines 186 to 189 and/or lines 633 and 634.

**[00103.2.22.22]** In one embodiment, the nucleic acid molecule used in the process of the invention distinguishes over the sequence indicated in Table I B, columns 5 or 7 lines 186 to 189 and/or lines 633 and 634, by one or more nucleotides. In one embodiment, the nucleic acid molecule used in the process of the invention does not consist of the sequence shown in indicated in Table I B, columns 5 or 7, lines 186 to 189 and/or lines 633 and 634. In one embodiment, the nucleic acid molecule used in the process of the invention is less than 100%, 99,999%, 99,99%, 99,9% or 99% identical to a sequence indicated in Table I B, columns 5 or 7, lines 186 to 189 and/or lines 633 and 634. In another embodiment, the nucleic acid molecule does not encode a polypeptide of a sequence indicated in Table II B, columns 5 or 7, lines 186 to 189 and/or lines 633 and 634.

**[0104.0.22.22]** In one embodiment, the nucleic acid molecule used in the process distinguishes over the sequence shown in Table I, columns 5 or 7, lines 186 to 189 and/or lines 633 and 634, preferably over the sequences as shown in Table IA, columns 5 or 7, lines 186 to 189 and/or lines 633 and 634 by one or more nucleotides or does not consist of the sequence shown in Table I, columns 5 or 7, lines 186 to 189 and/or lines 633 and 634, preferably of the sequences as shown in Table IA, columns 5 or 7, lines 186 to 189 and/or lines 633 and 634. In one embodiment, the nucleic acid molecule of the present invention is less than 100%, 99,999%, 99,99%, 99,9% or 99% identical to the sequence shown in Table I, columns 5 or 7, lines 1186 to 189, preferably to the sequences as shown in Table IA, columns 5 or 7, lines 186 to 189 and/or lines 633 and 634. In another embodiment, the nucleic acid molecule does not encode a polypeptide of the sequence shown in Table II, columns 5 or 7, lines 186 to 189, preferably of the sequences as shown in Table IIA, columns 5 or 7, lines 186 to 189 and/or lines 633 and 634.

**[0105.0.0.22] to [0107.0.0.22]** for the disclosure of the paragraphs [0105.0.0.22] to [0107.0.0.22] see paragraphs [0105.0.0.0] to [0107.0.0.0] above.

**[0108.0.22.22]** Nucleic acid molecules with the sequence shown in Table I, columns 5 or 7, lines 186 to 189 and/or lines 633 and 634, nucleic acid molecules which are derived from the amino acid sequences shown in Table II, columns 5 or 7, lines 186 to 189 and/or lines 633 and 634 or from polypeptides comprising the consensus sequence shown in Table IV, column 7, lines 186 to 189 and/or lines 633 and 634, or their derivatives or homologues encoding polypeptides with the enzymatic or biological activity of a polypeptide as indicated in Table I, column 3, 5 or 7, lines 186 to 189 and/or lines 633 and 634, or e.g. conferring an increase in the respective fine chemical after increasing its expression or activity are advantageously increased in the process according to the invention.

**[0109.0.0.22]** for the disclosure of this paragraph see [0109.0.0.0] above.

**[0110.0.0.22]** Nucleic acid molecules, which are advantageous for the process according to the invention and which encode polypeptides with an activity of a polypeptide used in the method of the invention or used in the process of the invention, e.g. of a protein as shown in Table II, columns 5 or 7, lines 186 to 189 and/or lines 633 and 634 or being encoded by a nucleic acid molecule indicated in Table I, columns 5 or 7, lines 186 to 189 and/or lines 633 and 634 or of its homologs, e.g. as indicated in Table II, columns 5 or 7, lines 186 to 189 and/or lines 633 and 634 can be determined from generally accessible databases.

**[0111.0.0.22]** for the disclosure of this paragraph see [0111.0.0.0] above. **[0112.0.22.22]** The nucleic acid molecules used in the process according to the invention take the form of isolated nucleic acid sequences, which encode polypeptides with an activity of a polypeptide as indicated in Table I, column 3, lines 186 to 189 and/or lines 633 and 634, or having the sequence of a polypeptide as indicated in Table II, columns 5 and 7, lines 186 to 189 and/or lines 633 and 634, and conferring an increase of the respective fine chemical.

**[0113.0.0.22] to [0120.0.0.22]** for the disclosure of the paragraphs [0113.0.0.22] to [0120.0.0.22] see paragraphs [0113.0.0.0] and [0120.0.0.0] above.

**[0121.0.22.22]** However, it is also possible to use artificial sequences, which differ in one or more bases from the nucleic acid sequences found in organisms, or in one or more amino acid molecules from polypeptide sequences found in organisms, in particular from the polypeptide sequences indicated in Table II, columns 5 or 7, lines 186 to 189 and/or lines 633 and 634 or the functional homologues thereof as described herein, preferably conferring above-mentioned activity, i.e. conferring a increase of the fine chemical after increasing its activity.

**[0122.0.0.22] to [0127.0.0.22]** for the disclosure of the paragraphs [0122.0.0.22] to [0127.0.0.22] see paragraphs [0122.0.0.0] and [0127.0.0.0] above.

**[0128.0.22.22]** Synthetic oligonucleotide primers for the amplification, e.g. as the pairs indicated in Table III, columns 7, lines 186 to 189 and/or lines 633 and 634, by means of polymerase chain reaction can be generated on the basis of a sequence shown herein, for example the sequence as indicated in Table I, columns 5 or 7, lines 186 to 189 and/or lines 633 and 634, or the sequences derived from sequences as indicated in Table II, columns 5 or 7, lines 186 to 189 and/or lines 633 and 634.

**[0129.0.22.22]** Moreover, it is possible to identify conserved regions from various organisms by carrying out protein sequence alignments with the polypeptide used in the process of the invention, in particular with sequences of the polypeptide of the invention, from which conserved regions, and in turn, degenerate primers can be derived. Conserved region for the polypeptide of the invention are indicated in the alignments shown in the figures. Conserved regions are those, which show a very little variation in the amino acid in one particular position of several homologs from different origin. The consensus sequences indicated in Table IV, column 7, lines 186 to 189 and/or lines 633 and 634, are derived from said alignments.

**[0130.0.0.22] to [0138.0.0.22]** for the disclosure of the paragraphs [0130.0.0.22] to [0138.0.0.22] see paragraphs [0130.0.0.0] to [0138.0.0.0] above.

**[0139.0.22.22]** Polypeptides having above-mentioned activity, i.e. conferring an increase of the respective fine chemical level, derived from other organisms, can be encoded by other DNA sequences which hybridize to a sequences indicated in Table I, columns 5 or 7, lines 186 to 189 and/or lines 633 and 634, preferably of Table IB, columns 5 or 7, lines 186 to 189 and/or lines 633 and 634 under relaxed hybridization conditions and which code on expression for peptides having the lipid, preferably a glycolipid, glycolipid containing galactose, more preferably a galactolipide and/or cerebroside resp., increasing activity.

**[0140.0.0.22] to [0146.0.0.22]** for the disclosure of the paragraphs [0140.0.0.22] to [0146.0.0.22] see paragraphs [0140.0.0.0] to [0146.0.0.0] above.

**[0147.0.22.22]** Further, the nucleic acid molecule of the invention comprises a nucleic acid molecule, which is a complement of one of the nucleotide sequences of above mentioned nucleic acid molecules or a portion thereof. A nucleic acid molecule which is complementary to one of the nucleotide sequences indicated in Table I, columns 5 or 7, lines 186 to 189 and/or lines 633 and 634, preferably of Table IB, columns 5 or 7, lines 186 to 189 and/or lines 633 and 634, is one which is sufficiently complementary to one of said nucleotide sequences s such that it can hybridize to one of said nucleotide sequences thereby forming a stable duplex. Preferably, the hybridisation is performed under stringent hybrization conditions. However, a complement of one of the herein disclosed sequences is preferably a sequence complement thereto according to the base pairing of nucleic acid molecules well known to the skilled person. For example, the bases A and G undergo base pairing with the bases T and U or C, resp. and visa versa. Modifications of the bases can influence the base-pairing partner.

**[0148.0.22.22]** The nucleic acid molecule of the invention comprises a nucleotide sequence which is at least about 30%, 35%, 40% or 45%, preferably at least about 50%, 55%, 60% or 65%, more preferably at least about 70%, 80%, or 90%, and even more preferably at least about 95%, 97%, 98%, 99% or more homologous to a nucleotide sequence shown in Table I, columns 5 or 7, lines 186 to 189 and/or lines 633 and 634, preferably of Table IB, columns 5 or 7, lines 186 to 189 and/or lines 633 and 634, or a portion thereof and preferably has above mentioned activity, in particular having a lipid, preferably a glycolipid, glycolipid containing galactose, more preferably a galactolipide and/or cerebroside -increasing activity after increasing its activity or an activity of a product of a gene encoding said sequence or its homologs .

**[0149.0.22.22]** The nucleic acid molecule of the invention comprises a nucleotide sequence which hybridizes, preferably hybridizes under stringent conditions as defined herein, to one of the nucleotide sequences indicated in Table I, columns 5 or 7, lines 186 to 189 and/or lines 633 and 634, preferably of Table IB, columns 5 or 7, lines 186 to 189 and/or lines 633 and 634, or a portion thereof and encodes a protein having above-mentioned activity, e.g. conferring an increase of the respective fine chemical, e.g. of lipid, preferably a glycolipid, glycolipid containing galactose more preferably a galactolipide and/or cerebroside and optionally the activity of a protein indicated in Table II, column 5, lines 186 to 189 and/or lines 633 and 634, preferably of Table IB, columns 5 or 7, lines 186 to 189 and/or lines 633 and 634.

**[00149.1.22.22]** Optionally, the nucleotide sequence, which hybridises to one of the nucleotide sequences indicated in Table I, columns 5 or 7, lines 186 to 189 and/or lines 633 and 634, preferably of Table IB, columns 5 or 7, lines 186 to 189 and/or lines 633 and 634, has further one or more of the activities annotated or known for the a protein as indicated in Table II, column 3, lines 186 to 189 and/or lines 633 and 634, preferably of Table IIB, columns 5 or 7, lines 186 to 189 and/or lines 633 and 634.

**[0150.0.22.22]** Moreover, the nucleic acid molecule of the invention can comprise only a portion of the coding region of one of the sequences shown in Table I, columns 5 or 7, lines 186 to 189 and/or lines 633 and 634, preferably of Table IB, columns 5 or 7, lines 186 to 189 and/or lines 633 and 634, for example a fragment which can be used as a probe or primer or a fragment encoding a biologically active portion of the polypeptide of the present invention or of a polypeptide used in the process of the present invention, i.e. having above-mentioned activity, e.g. conferring an increase of lipid, preferably a glycolipid, glycolipid containing galactose, more preferably a galactolipide and/or cerebroside if its activity is increased. The nucleotide sequences determined from the cloning of the present protein-according-to-the-invention-encoding gene allows for the generation of probes and primers designed for use in identifying and/or cloning its homologues in other cell types and organisms. The probe/primer typically comprises substantially purified oligonucleotide. The oligonucleotide typically comprises a region of nucleotide sequence that hybridizes under stringent conditions to at least about 12, 15 preferably about 20 or 25, more preferably about 40, 50 or 75 consecutive nucleotides of a sense strand of one of the sequences set forth, e.g., in Table I, columns 5 or 7, lines 186 to 189 and/or lines 633 and 634, an anti-sense sequence of one of the sequences, e.g., set forth in Table I, columns 5 or 7, lines 186 to 189 and/or lines 633 and 634, or naturally occurring mutants thereof. Primers based on a nucleotide of invention can be used in PCR reactions to clone homologues of the polypeptide of the invention or of the polypeptide used in the process of the invention, e.g. as the primers described in the examples of the present invention, e.g. as shown in the examples. A PCR with the primers shown in table III, column 7, lines 186 to 189., will result in a fragment of a polynucleotide sequence as indicated in Table I, columns 5 or 7, lines 186 to 189. Preferably is Table IB, columns 5 or 7, lines 186 to 189 and/or lines 633 and 634.

**[0151.0.0.22]** for the disclosure of this paragraph see paragraph [0151.0.0.0] above.

**[0152.0.22.22]** The nucleic acid molecule of the invention encodes a polypeptide or portion thereof which includes an amino acid sequence which is sufficiently homologous to an amino acid sequence as indicated in Table II, columns 5 or 7, lines 186 to 189 and/or lines 633 and 634, such that the protein or portion thereof maintains the ability to participate in the respective fine chemical production, in particular an activity increasing the level of lipids, preferably a glycolipid, glycolipid containing galactose, more preferably a galactolipide and/or cerebrosideas mentioned above or as described in the examples in plants or microorganisms is comprised.

**[0153.0.22.22]** As used herein, the language "sufficiently homologous" refers to proteins or portions thereof which have amino acid sequences which include a minimum number of identical or equivalent amino acid residues (e.g., an amino acid residue which has a similar side chain as an amino acid residue in one of the sequences of the polypeptide of the present invention) to an amino acid sequence as indicated in Table II, columns 5 or 7, lines 186 to 189 and/or lines 633 and 634, such that the protein or portion thereof is able to participate in the increase of the respective fine chemical production. In one embodiment, a protein or portion thereof as indicated in Table II, columns 5 or 7, lines 186 to 189 and/or lines 633 and 634, has for example an activity of a polypeptide indicated in Table II, column 3, lines 186 to 189 and/or lines 633 and 634.

**[0154.0.22.22]** In one embodiment, the nucleic acid molecule of the present invention comprises a nucleic acid that encodes a portion of the protein of the present invention. The protein is at least about 30%, 35%, 40%, 45% or 50%, preferably at least about 55%, 60%, 65% or 70%, and more preferably at least about 75%, 80%, 85%, 90%, 91%, 92%, 93% or 94% and most preferably at least about 95%, 97%, 98%, 99% or more homologous to an entire amino acid sequence as indicated in Table II, columns 5 or 7, lines 186 to 189 and/or lines 633 and 634, and has above-mentioned activity, e.g. conferring preferably the increase of the respective fine chemical.

**[0155.0.0.22] and [0156.0.0.22]** for the disclosure of the paragraphs [0155.0.0.22] and [0156.0.0.22] see paragraphs [0155.0.0.0] and [0156.0.0.0] above.

**[0157.0.22.22]** The invention further relates to nucleic acid molecules that differ from one of a nucleotide sequences as indicated in Table I, columns 5 or 7, lines 186 to 189 and/or lines 633 and 634, (and portions thereof) due to degeneracy of the genetic code and thus encode a polypeptide of the present invention, in particular a polypeptide having above mentioned activity, e.g. conferring an increase in the respective fine chemical in a organism, e.g. as polypeptides comprising the sequence as indicated in Table IV, columns 5 or 7, lines 186 to 189 and/or lines 633 and 634 or of the polypeptide as indicated in Table II, columns 5 or 7, lines 186 to 189 and/or lines 633 and 634 or their functional homologues. Advantageously, the nucleic acid molecule of the invention comprises, or in an other embodiment has, a nucleotide sequence encoding a protein comprising, or in an other embodiment having, a consensus sequences as indicated in Table IV, column 7, lines 186 to 189 and/or lines 633 and . 634, or of the polypeptide as as indicated in Table II, columns 5 or 7, lines 186 to 189 and/or lines 633 and 634, or the functional homologues. In a still further embodiment, the nucleic acid molecule of the invention encodes a full length protein which is substantially homologous to an amino acid sequence comprising a consensus sequence as indicated in Table IV, column 7, lines 186 to 189 and/or lines 633 and 634, or of a polypeptide as indicated in Table II, columns 5 or 7, lines 186 to 189 and/or lines 633 and 634, or the functional homologues thereof. However, in a preferred embodiment, the nucleic acid molecule of the present invention does not consist of a sequence as indicated in Table I, columns 5 or 7, lines 186 to 189 and/or lines 633 and 634, preferably as indicated in Table I A, columns 5 or 7, lines 186 to 189 and/or lines 633 and 634, resp. Preferably the nucleic acid molecule of the invention is a functional homologue or identical to a nucleic acid molecule indicated in Table I B, column 7, lines 186 to 189 and/or lines 633 and 634.

**[0158.0.0.22] to [0160.0.0.22]** for the disclosure of the paragraphs [0158.0.0.22] to [0160.0.0.22] see paragraphs [0158.0.0.0] to [0160.0.0.0] above.

**[0161.0.22.22]** Accordingly, in another embodiment, a nucleic acid molecule of the invention is at least 15, 20, 25 or 30 nucleotides in length. Preferably, it hybridizes under stringent conditions to a nucleic acid molecule comprising a nucleotide sequence of the nucleic acid molecule of the present invention or used in the process of the present invention, e.g. comprising a sequence as indicated in Table I, columns 5 or 7, lines 186 to 189 and/or lines 633 and 634. The nucleic acid molecule is preferably at least 20, 30, 50, 100, 250 or more nucleotides in length.

**[0162.0.0.22]** for the disclosure of this paragraph see paragraph [0162.0.0.0] above.

**[0163.0.22.22]** Preferably, nucleic acid molecule of the invention that hybridizes under stringent conditions to a sequence as indicated in Table I, columns 5 or 7, lines 186 to 189 and/or lines 633 and 634, corresponds to a naturally-occurring nucleic acid molecule of the invention. As used herein, a "naturally-occurring" nucleic acid molecule refers to an RNA or DNA molecule having a nucleotide sequence that occurs in nature (e.g., encodes a natural protein). Preferably, the nucleic acid molecule encodes a natural protein having above-mentioned activity, e.g. conferring the respective fine chemical increase after increasing the expression or activity thereof or the activity of a protein of the invention or used in the process of the invention.

**[0164.0.0.22]** for the disclosure of this paragraph see paragraph [0164.0.0.0] above.

**[0165.0.22.22]** For example, nucleotide substitutions leading to amino acid substitutions at "non-essential" amino acid residues can be made in a sequence of the nucleic acid molecule of the invention or used in the process of the invention, e.g. as indicated in Table I, columns 5 or 7, lines 186 to 189 and/or lines 633 and 634.

**[0166.0.0.22] and [0167.0.0.22]** for the disclosure of the paragraphs [0166.0.0.22] and [0167.0.0.22] see paragraphs [0166.0.0.0] and [0167.0.0.0] above.

**[0168.0.22.22]** Accordingly, the invention relates to nucleic acid molecules encoding a polypeptide having above-mentioned activity, e.g. conferring an increase in the respective fine chemical in an organisms or parts thereof that contain changes in amino acid residues that are not essential for said activity. Such polypeptides differ in amino acid sequence from a sequence contained in a sequence as indicated in Table II, columns 5 or 7, lines 186 to 189 and/or lines 633 and 634, yet retain said activity described herein. The nucleic acid molecule can comprise a nucleotide sequence encoding a polypeptide, wherein the polypeptide comprises an amino acid sequence at least about 50% identical to an amino acid sequence as indicated in Table II, columns 5 or 7, lines 186 to 189 and/or lines 633 and 634, and is capable of participation in the increase of production of the respective fine chemical after increasing its activity, e.g. its expression. Preferably, the protein encoded by the nucleic acid molecule is at least about 60% identical to a sequence as indicated in Table II, columns 5 or 7, lines 186 to 189 and/or lines 633 and 634 more preferably at least about 70% identical to one of the sequences as indicated in Table II, columns 5 or 7, lines 186 to 189, even more preferably at least about 80%, 90%, or 95% homologous to a sequence as indicated in Table II, columns 5 or 7, lines 186 to 189 and/or lines 633 and 634 and most preferably at least about 96%, 97%, 98%, or 99% identical to the sequence as indicated in Table II, columns 5 or 7, lines 186 to 189 and/or lines 633 and 634.
Accordingly, the invention relates to nucleic acid molecules encoding a polypeptide having above-mentioned activity, e.g. conferring an increase in the the respective fine chemical in an organisms or parts thereof that contain changes in amino acid residues that are not essential for said activity. Such polypeptides differ in amino acid sequence from a sequence contained in a sequence as indicated in Table II, columns 5 or 7, lines 186 to 189 and/or lines 633 and 634, preferably of Table II B, column 7, lines 186 to 189 and/or lines 633 and 634 yet retain said activity described herein. The nucleic acid molecule can comprise a nucleotide sequence encoding a polypeptide, wherein the polypeptide comprises an amino acid sequence at least about 50% identical to an amino acid sequence as indicated in Table II, columns 5 or 7, lines 186 to 189 and/or lines 633 and 634, preferably of Table II B, column 7, lines 186 to 189 and/or lines 633 and 634 and is capable of participation in the increase of production of the respective fine chemical after increasing its activity, e.g. its expression. Preferably, the protein encoded by the nucleic acid molecule is at least about 60% identical to a sequence as indicated in Table II, columns 5 or 7, lines 186 to 189 and/or lines 633 and 634, preferably of Table II B, column 7, lines 186 to 189 and/or lines 633 and 634, more preferably at least about 70% identical to one of the sequences as indicated in Table II, columns 5 or 7, lines 186 to 189 and/or lines 633 and 634, preferably of Table II B, column 7 lines 186 to 189 and/or lines 633 and 634, even more preferably at least about 80%, 90%, or 95% homologous to a sequence as indicated in Table II, columns 5 or 7, lines 186 to 189 and/or lines 633 and 634, preferably of Table II B, column 7, lines 186 to 189 and/or lines 633 and 634, and most preferably at least about 96%, 97%, 98%, or 99% identical to the sequence as indicated in Table II, columns 5 or 7, lines 186 to 189 and/or lines 633 and 634, preferably of Table II B, column 7, lines 186 to 189 and/or lines 633 and 634.

**[0169.0.0.22] to [0172.0.0.22]** for the disclosure of the paragraphs [0169.0.0.22] to [0172.0.0.22] see paragraphs [0169.0.0.0] to [0172.0.0.0] above.

**[0173.0.22.22]** For example a sequence, which has 80% homology with sequence SEQ ID NO: 14358 at the nucleic acid level is understood as meaning a sequence which, upon comparison with the sequence SEQ ID NO: 14358 by the above Gap program algorithm with the above parameter set, has a 80% homology.

**[0174.0.0.22]** for the disclosure of this paragraph see paragraph [0174.0.0.0] above.

**[0175.0.22.22]** For example a sequence which has a 80% homology with sequence SEQ ID NO: 14355 at the protein level is understood as meaning a sequence which, upon comparison with the sequence SEQ ID NO: 14355 by the above program algorithm with the above parameter set, has a 80% homology.

**[0176.0.22.22]** Functional equivalents derived from one of the polypeptides as indicated in Table II, columns 5 or 7, lines 186 to 189 and/or lines 633 and 634, according to the invention by substitution, insertion or deletion have at least 30%, 35%, 40%, 45% or 50%, preferably at least 55%, 60%, 65% or 70% by preference at least 80%, especially preferably at least 85% or 90%, 91%, 92%, 93% or 94%, very especially preferably at least 95%, 97%, 98% or 99% homology with one of the polypeptides as indicated in Table II, columns 5 or 7, lines 186 to 189 and/or lines 633 and 634, according to the invention and are distinguished by essentially the same properties as a polypeptide as indicated in Table II, columns 5 or 7, lines 186 to 189 and/or lines 633 and 634.

**[0177.0.22.22]** Functional equivalents derived from a nucleic acid sequence as indicated in Table I, columns 5 or 7, lines 186 to 189 and/or lines 633 and 634, lines 186 to 189 and/or lines 633 and 634, preferably of Table I B, lines 186 to 189 and/or lines 633 and 634 resp. according to the invention by substitution, insertion or deletion have at least 30%, 35%, 40%, 45% or 50%, preferably at least 55%, 60%, 65% or 70% by preference at least 80%, especially preferably at least 85% or 90%, 91%, 92%, 93% or 94%, very especially preferably at least 95%, 97%, 98% or 99% homology with one of a polypeptides as indicated in Table II, columns 5 or 7, lines 186 to 189 and/or lines 633 and 634, according to the invention and encode polypeptides having essentially the same properties as a polypeptide as indicated in Table II, columns 5 or 7, lines 186 to 189 and/or lines 633 and 634, preferably of Table II B, lines 186 to 189 and/or lines 633 and 634 resp.

**[0178.0.0.22]** for the disclosure of this paragraph see [0178.0.0.0] above.

**[0179.0.22.22]** A nucleic acid molecule encoding a homologous to a protein sequence of as indicated in Table II, columns 5 or 7, lines 186 to 189 and/or lines 633 and 634, preferably of Table II B, lines 186 to 189 and/or lines 633 and 634 resp., can be created by introducing one or more nucleotide substitutions, additions or deletions into a nucleotide sequence of the nucleic acid molecule of the present invention, in particular as indicated in Table I, columns 5 or 7, lines 186 to 189 and/or lines 633 and 634, preferably of Table I B, lines 186 to 189 and/or lines 633 and 634 resp., such that one or more amino acid substitutions, additions or deletions are introduced into the encoded protein. Mutations can be introduced into the encoding sequence of a sequence as indicated in Table I, columns 5 or 7, lines 186 to 189 and/or lines 633 and 634, preferably of Table I B, lines 186 to 189 and/or lines 633 and 634 resp., by standard techniques, such as site-directed mutagenesis and PCR-mediated mutagenesis.

**[0180.0.0.22] to [0183.0.0.22]** for the disclosure of the paragraphs [0180.0.0.22] to [0183.0.0.22] see paragraphs [0180.0.0.0] to [0183.0.0.0] above.

**[0184.0.22.22]** Homologues of the nucleic acid sequences used, with a sequence as indicated in Table I, columns 5 or 7, lines 186 to 189 and/or lines 633 and 634, preferably of Table I B, lines 186 to 189 and/or lines 633 and 634 resp., or of the nucleic acid sequences derived from a sequences as indicated in Table II, columns 5 or 7, lines 186 to 189 and/or lines 633 and 634, preferably of Table II B, lines 186 to 189 and/or lines 633 and 634 resp., comprise also allelic variants with at least approximately 30%, 35%, 40% or 45% homology, by preference at least approximately 50%, 60% or 70%, more preferably at least approximately 90%, 91%, 92%, 93%, 94% or 95% and even more preferably at least approximately 96%, 97%, 98%, 99% or more homology with one of the nucleotide sequences shown or the abovementioned derived nucleic acid sequences or their homologues, derivatives or analogues or parts of these. Allelic variants encompass in particular functional variants which can be obtained by deletion, insertion or substitution of nucleotides from the sequences shown, preferably from a sequence as indicated in Table I, columns 5 or 7, lines 186 to 189 and/or lines 633 and 634, preferably of Table I B, lines 186 to 189 and/or lines 633 and 634 resp., or from the derived nucleic acid sequences, the intention being, however, that the enzyme activity or the biological activity of the resulting proteins synthesized is advantageously retained or increased.

**[0185.0.22.22]** In one embodiment of the present invention, the nucleic acid molecule of the invention or used in the process of the invention comprises one or more sequences as indicated in Table I, columns 5 or 7, lines 186 to 189 and/or lines 633 and 634, preferably of Table I B, lines 186 to 189 and/or lines 633 and 634 resp. In one embodiment, it is preferred that the nucleic acid molecule comprises as little as possible other nucleotide sequences not shown in any one of sequences as indicated in Table I, columns 5 or 7, lines 186 to 189 and/or lines 633 and 634, preferably of Table I B, lines 186 to 189 and/or lines 633 and 634 resp. In one embodiment, the nucleic acid molecule comprises less than 500, 400, 300, 200, 100, 90, 80, 70, 60, 50 or 40 further nucleotides. In a further embodiment, the nucleic acid molecule comprises less than 30, 20 or 10 further nucleotides. In one embodiment, a nucleic acid molecule used in the process of the invention is identical to a sequence as indicated in Table I, columns 5 or 7, lines 186 to 189 and/or lines 633 and 634, preferably of Table I B, lines 186 to 189 and/or lines 633 and 634 resp.

**[0186.0.22.22]** Also preferred is that one or more nucleic acid molecule(s) used in the process of the invention encode a polypeptide comprising a sequence as indicated in Table II, columns 5 or 7, lines 186 to 189 and/or lines 633 and 634, preferably of Table II B, lines 186 to 189 and/or lines 633 and 634 resp. In one embodiment, the nucleic acid molecule encodes less than 150, 130, 100, 80, 60, 50, 40 or 30 further amino acids. In a further embodiment, the encoded polypeptide comprises less than 20, 15, 10, 9, 8, 7, 6 or 5 further amino acids. In one embodiment, the encoded polypeptide used in the process of the invention is identical to the sequences as indicated in Table II, columns 5 or 7, lines 186 to 189 and/or lines 633 and 634, preferably of Table II B, lines 186 to 189 and/or lines 633 and 634 resp.

**[0187.0.22.22]** In one embodiment, the nucleic acid molecule of the invention or used in the process encodes a polypeptide comprising a sequence as indicated in Table II, columns 5 or 7, lines 186 to 189 and/or lines 633 and 634, preferably of Table II B, lines 186 to 189 and/or lines 633 and 634 resp., comprises less than 100 further nucleotides. In a further embodiment, said nucleic acid molecule comprises less than 30 further nucleotides. In one embodiment, the nucleic acid molecule used in the process is identical to a coding sequence encoding a sequences as indicated in Table II, columns 5 or 7, lines 186 to 189 and/or lines 633 and 634, preferably of Table II B, lines 186 to 189 and/or lines 633 and 634 resp.

**[0188.0.22.22]** Polypeptides (= proteins), which still have the essential biological or enzymatic activity of the polypeptide of the present invention conferring an increase of the respective fine chemical i.e. whose activity is essentially not reduced, are polypeptides with at least 10% or 20%, by preference 30% or 40%, especially preferably 50% or 60%, very especially preferably 80% or 90 or more of the wild type biological activity or enzyme activity, advantageously, the activity is essentially not reduced in comparison with the activity of a polypeptide as indicated in Table II, columns 5 or 7, lines 186 to 189 and/or lines 633 and 634, preferably compared to a sequence as indicated in Table II, column 3 and 5, lines 186 to 189 and/or lines 633 and 634, and expressed under identical conditions.
In one embodiment, the polypeptide of the invention is a homolog consisting of or comprising the sequence as indicated in Table II B, columns 7, lines 186 to 189 and/or lines 633 and 634.

**[0189.0.22.22]** Homologues of a sequences as indicated in Table I, columns 5 or 7, lines 186 to 189 and/or lines 633 and 634, or of a derived sequences as indicated in Table II, columns 5 or 7, lines 186 to 189 and/or lines 633 and 634, also mean truncated sequences, cDNA, single-stranded DNA or RNA of the coding and noncoding DNA sequence. Homologues of said sequences are also understood as meaning derivatives, which comprise noncoding regions such as, for example, UTRs, terminators, enhancers or promoter variants. The promoters upstream of the nucleotide sequences stated can be modified by one or more nucleotide substitution(s), insertion(s) and/or deletion(s) without, however, interfering with the functionality or activity either of the promoters, the open reading frame (= ORF) or with the 3'-regulatory region such as terminators or other 3'regulatory regions, which are far away from the ORF. It is furthermore possible that the activity of the promoters is increased by modification of their sequence, or that they are replaced completely by more active promoters, even promoters from heterologous organisms. Appropriate promoters are known to the person skilled in the art and are mentioned herein below.

**[0190.0.0.22] and [0191.0.0.22]** for the disclosure of the paragraphs [0190.0.0.22] and [0191.0.0.22] see paragraphs [0190.0.0.0] and [0191.0.0.0] above.

**[0191.1.0.22]:** for the disclosure of this paragraph see [0191.1.0.0] above.

**[0192.0.0.22] to [0203.0.0.22]** for the disclosure of the paragraphs [0192.0.0.22] to [0203.0.0.22] see paragraphs [0192.0.0.0] to [0203.0.0.0] above.

**[0204.0.22.22]** Accordingly, in one embodiment, the invention relates to a nucleic acid molecule, which comprises a nucleic acid molecule selected from the group consisting of:
a) nucleic acid molecule encoding, preferably at least the mature form, of a polypeptide as indicated in Table II, columns 5 or 7, lines 186 to 189 and/or lines 633 and 634, preferably of Table II B, lines 186 to 189 and/or lines 633 and 634 resp.; or a fragment thereof conferring an increase in the amount of the respective fine chemical, i.e. lipid, preferably a glycolipid, glycolipid containing galactose, more preferably a galactolipide and/or cerebroside in an organism or a part thereof
b) nucleic acid molecule comprising, preferably at least the mature form, of a nucleic acid molecule as indicated in Table I, columns 5 or 7, lines 186 to 189 and/or lines 633 and 634, preferably of Table I B, lines 186 to 189 and/or lines 633 and 634 resp., or a fragment thereof conferring an increase in the amount of the respective fine chemical in an organism or a part thereof;
c) nucleic acid molecule whose sequence can be deduced from a polypeptide sequence encoded by a nucleic acid molecule of (a) or (b) as result of the degeneracy of the genetic code and conferring an increase in the amount of the fine chemical in an organism or a part thereof;
d) nucleic acid molecule encoding a polypeptide whose sequence has at least 50% identity with the amino acid sequence of the polypeptide encoded by the nucleic acid molecule of (a) to (c) and conferring an increase in the amount of the fine chemical in an organism or a part thereof;
e) nucleic acid molecule which hybridizes with a nucleic acid molecule of (a) to (c) under stringent hybridisation conditions and conferring an increase in the amount of the fine chemical in an organism or a part thereof;
f) nucleic acid molecule encoding a polypeptide, the polypeptide being derived by substituting, deleting and/or adding one or more amino acids of the amino acid sequence of the polypeptide encoded by the nucleic acid molecules (a) to,(d), preferably to (a) to (c), and conferring an increase in the amount of the fine chemical in an organism or a part thereof;
g) nucleic acid molecule encoding a fragment or an epitope of a polypeptide which is encoded by one of the nucleic acid molecules of (a) to (e), preferably to (a) to (c) and conferring an increase in the amount of the fine chemical in an organism or a part thereof ;
h) nucleic acid molecule comprising a nucleic acid molecule which is obtained by amplifying a cDNA library or a genomic library using primers or primer pairs as indicated in Table III, column 7, lines 186 to 189 and/or lines 633 and 634, and conferring an increase in the amount of the respective fine chemical, i.e. lipid, preferably a glycolipid, glycolipid containing galactose, more preferably a galactolipide and/or cerebrosidein an organism or a part thereof;
i) nucleic acid molecule encoding a polypeptide which is isolated, e.g. from a expression library, with the aid of monoclonal antibodies against a polypeptide encoded by one of the nucleic acid molecules of (a) to (g), preferably to (a) to (c) and conferring an increase in the amount of the fine chemical in an organism or a part thereof;
j) nucleic acid molecule which encodes a polypeptide comprising a consensus sequence as indicated in Table IV, column 7, lines 186 to 189, and conferring an increase in the amount of the respective fine chemical i.e. lipid, preferably a glycolipid, glycolipid containing galactose, more preferably a galactolipide and/or cerebroside in an organism or a part thereof;
k) nucleic acid molecule encoding the amino acid sequence of a polypeptide encoding a domain of a polypeptide as indicated in Table II, columns 5 or 7, lines 186 to 189 and/or lines 633 and 634, preferably of Table II B, lines 186 to 189 and/or lines 633 and 634 resp., and conferring an increase in the amount of the respective fine chemical i.e. lipid, preferably a glycolipid, glycolipid containing galactose, more preferably a galactolipide and/or cerebroside in an organism or a part thereof; and
l) nucleic acid molecule which is obtainable by screening a suitable nucleic acid library under stringent hybridization conditions with a probe comprising one of the sequences of the nucleic acid molecule of (a) to (k) or with a fragment of at least 15 nt, preferably 20 nt, 30 nt, 50 nt, 100 nt, 200 nt or 500 nt of the nucleic acid molecule characterized in (a) to (h) or of a nucleic acid molecule as indicated in Table I, columns 5 or 7, lines 186 to 189 and/or lines 633 and 634 resp., or a nucleic acid molecule encoding, preferably at least the mature form of, the polypeptide as indicated in Table II, columns 5 or 7, lines 186 to 189 and/or lines 633 and 634, and conferring an increase in the amount of the respective fine chemical in an organism or a part thereof;
whereby, preferably, the nucleic acid molecule according to (a) to (I) distinguishes over a sequence depicted in as indicated in Table IA or IB, columns 5 or 7, lines 186 to 189 and/or lines 633 and 634, by one or more nucleotides. In one embodiment, the nucleic acid molecule of the invention does not consist of a sequence as indicated in Table IA or IB, columns 5 or 7, lines 186 to 189 and/or lines 633 and 634. In an other embodiment, the nucleic acid molecule of the present invention is at least 30 % identical and less than 100%, 99,999%, 99,99%, 99,9% or 99% identical to a sequence indicated in Table IA or IB, columns 5 or 7, lines 186 to 189 and/or lines 633 and 634.
In a further embodiment the nucleic acid molecule does not encode a polypeptide sequence as indicated in Table IIA or IIB, columns 5 or 7, lines 186 to 189 and/or lines 633 and 634. Accordingly, in one embodiment, the nucleic acid molecule of the present invention encodes in one embodiment a polypeptide which differs at least in one or more amino acids from a polypeptide indicated in Table IIA or IIB, columns 5 or 7, lines 186 to 189 and/or lines 633 and 634. In another embodiment, a nucleic acid molecule indicated in Table IA or IB, columns 5 or 7, lines 186 to 189 and/or lines 633 and 634, does not encode a protein of a sequence indicated in Table IIA or IIB, columns 5 or 7, lines 186 to 189 and/or lines 633 and 634. Accordingly, in one embodiment, the protein encoded by a sequences of a nucleic acid according to (a) to (I) does not consist of a sequence as indicated in Table IIA or IIB, columns 5 or 7, lines 186 to 189 and/or lines 633 and 634. In a further embodiment, the protein of the present invention is at least 30 % identical to a protein sequence indicated in Table IIA or IIB, columns 5 or 7, lines 186 to 189 and/or lines 633 and 634, and less than 100%, preferably less than 99,999%, 99,99% or 99,9%, more preferably less than 99%, 98%, 97%, 96% or 95% identical to a sequence as indicated in Table IA or IB, columns 5 or 7, lines 186 to 189 and/or lines 633 and 634.

**[0205.0.0.22] and [0206.0.0.22]** for the disclosure of the paragraphs [0205.0.0.22] and [0206.0.0.22] see paragraphs [0205.0.0.0] and [0206.0.0.0] above.

**[0207.0.22.22]** As described herein, the nucleic acid construct can also comprise further genes, which are to be introduced into the organisms or cells. It is possible and advantageous to introduce into, and express in, the host organisms regulatory genes such as genes for inductors, repressors or enzymes, which, owing to their enzymatic activity, engage in the regulation of one or more genes of a biosynthetic pathway. These genes can be of heterologous or homologous origin. Moreover, further biosynthesis genes may advantageously be present, or else these genes may be located on one or more further nucleic acid constructs. Genes, which are advantageously employed as biosynthesis genes are genes of the glycolipid, preferably galactolipid metabolism, galactose metabolism, fatty acid metabolism, of glycolysis, of the tricarboxylic acid metabolism, of triacylglycerol or lipid, preferably glycerophospholipids, sphingolipids and/or galactolipids biosynthesis or their combinations. As described herein, regulator sequences or factors can have a positive effect on preferably the gene expression of the genes introduced, thus increasing it. Thus, an enhancement of the regulator elements may advantageously take place at the transcriptional level by using strong transcription signals such as promoters and/or enhancers. In addition, however, an enhancement of translation is also possible, for example by increasing mRNA stability or by inserting a translation enhancer sequence.

**[0208.0.0.22] to [0226.0.0.22]** for the disclosure of the paragraphs [0208.0.0.22] to [0226.0.0.22] see paragraphs [0208.0.0.0] to [0226.0.0.0] above.

**[0227.0.22.22]** The abovementioned nucleic acid molecules can be cloned into the nucleic acid constructs or vectors according to the invention in combination together with further genes, or else different genes are introduced by transforming several nucleic acid constructs or vectors (including plasmids) into a host cell, advantageously into a plant cell or a microorganisms.
In addition to the sequence mentioned in Table I, columns 5 or 7, lines 186 to 189 and/or lines 633 and 634, or its derivatives, it is advantageous additionally to express and/or mutate further genes in the organisms. Especially advantageously, additionally at least one further gene of the galyctolipid synthesis, of the galactose pathway or of the of triacylglycerol or lipid, preferably glycerolipids, sphingolipids, ceramid and/or cerebroside biosynthetic pathway is expressed in the organisms such as plants or microorganisms. It is also possible that the regulation of the natural genes has been modified advantageously so that the gene and/or its gene product is no longer subject to the regulatory mechanisms which exist in the organisms. This leads to an increased synthesis of the amino acids desired since, for example, feedback regulations no longer exist to the same extent or not at all. In addition it might be advantageously to combine the sequences shown in Table I, columns 5 or 7, lines 186 to 189 and/or lines 633 and 634, with genes which generally support or enhances to growth or yield of the target organism, for example genes which lead to faster growth rate of microorganisms or genes which produces stress-, pathogen, or herbicide resistant plants.

**[0228.0.22.22]** In a further embodiment of the process of the invention, therefore, organisms are grown, in which there is simultaneous overexpression of at least one nucleic acid or one of the genes which code for proteins involved in the glycoliopid, preferably galactolipid synthesis or galactose pathway.

**[0229.0.22.22]** for the disclosure of this paragraph see [0229.0.5.5] above.

**[0230.0.0.22]** for the disclosure of this paragraph see [0230.0.0.0] above.

**[0231.0.22.22]** In a further advantageous embodiment of the process of the invention, the organisms used in the process are those in which simultaneously a lipid, preferably a glycolipid, glycolipid containing galactose, more preferably a galactolipide and/or cerebroside degrading protein is attenuated, in particular by reducing the rate of expression of the corresponding gene.

**[0232.0.0.22] to [0276.0.0.22]** for the disclosure of the paragraphs [0232.0.0.22] to [0276.0.0.22] see paragraphs [0232.0.0.0] to [0276.0.0.0] above.

**[0277.0.22.22]** Glycolipids, preferably galactolipids or galactose containing compounds produced according to the process of the invention can be isolated from the organism by methods with which the skilled worker is familiar, for example via extraction, salt precipitation and/or different chromatography methods. The process according to the invention can be conducted batchwise, semibatchwise or continuously. The fine chemical produced in the process according to the invention can be isolated as mentioned above from the organisms, advantageously plants, in the form of their glycolipids, preferably galactolipids and/or free fatty acids, oils, fats. Glycolipids, preferably galactolipids or galactose containing compounds produced by this process can be obtained by harvesting the organisms, either from the crop in which they grow, or from the field. This can be done via pressing or extraction of the plant parts, preferably the plant seeds. Hexane is preferably used as solvent in the process, in which more than 96% of the compounds produced in the process can be isolated. The galactolipids are easily separated from phospholipids by adsorption chromatography, usually by making use of the fact that they, unlike phospholipids, are soluble in acetone. Useful are also other organic solvents such as hydrocarbons, chloroform, benzene, ethers and alcohols. Thereafter, the resulting products are processed further, i.e. degummed, refined, bleached and/or deodorized.

**[0278.0.0.22] to [0282.0.0.22]** for the disclosure of the paragraphs [0278.0.0.22] to [0282.0.0.22] see paragraphs [0278.0.0.0] to [0282.0.0.0] above.

**[0283.0.22.22]** Moreover, a native polypeptide conferring the increase of the fine chemical in an organism or part thereof can be isolated from cells (e.g., endothelial cells), for example using the antibody of the present invention as described below, in particular, an antibody against a protein as indicated in Table II, column 3, lines 186 to 189 and/or lines 633 and 634, e.g. an antibody against a polypeptide as indicated in Table II, columns 5 or 7, lines 186 to 189 and/or lines 633 and 634, which can be produced by standard techniques utilizing polypeptides comprising or consisting of above mentioned sequences, e.g. the polypeptide of the present invention or fragment thereof. Preferred are monoclonal antibodies specifically binding to polypeptides as indicated in Table II, columns 5 or 7, lines 186 to 189 and/or lines 633 and 634, more preferred specifically binding to polypeptides as indicated in Table II, column 5, lines 186 to 189 and/or lines 633 and 634.

**[0284.0.0.22]** for the disclosure of this paragraph see [0284.0.0.0] above.

**[0285.0.22.22]** In one embodiment, the present invention relates to a polypeptide having a sequence as indicated in Table II, columns 5 or 7, lines 186 to 189 and/or lines 633 and 634, or as coded by a nucleic acid molecule as indicated in Table I, columns 5 or 7, lines 186 to 189 and/or lines 633 and 634, or functional homologues thereof.

**[0286.0.22.22]** In one advantageous embodiment, in the method of the present invention the activity of a polypeptide is increased which comprises or consists of a consensus sequence as indicated in Table IV, column 7, lines 186 to 189 and/or lines 633 and 634, and in one another embodiment, the present invention relates to a polypeptide comprising or consisting of a consensus sequence as indicated in Table IV, column 7, lines 186 to 189 and/or lines 633 and 634, whereby 20 or less, preferably 15 or 10, preferably 9, 8, 7, or 6, more preferred 5 or 4, even more preferred 3, even more preferred 2, even more preferred 1, most preferred 0 of the amino acids positions indicated can be replaced by any amino acid or, in an further embodiment, can be replaced and/or absent. In one embodiment, the present invention relates to the method of the present invention comprising a polypeptide or to a polypeptide comprising more than one consensus sequences (of an individual line) as indicated in Table IV, column 7, lines 186 to 189 and/or lines 633 and 634.

**[0287.0.0.22] to [0290.0.0.22]** for the disclosure of the paragraphs [0287.0.0.22] to [0290.0.0.22] see paragraphs [0287.0.0.0] to [0290.0.0.0] above.

**[0291.0.22.22]** In one advantageous embodiment, the method of the present invention comprises the increasing of a polypeptide comprising or consisting of plant or microorganism specific consensus sequences. In one embodiment, said polypeptide of the invention distinguishes over a sequence as indicated in Table IIA or IIB, columns 5 or 7, lines 186 to 189 and/or lines 633 and 634, by one or more amino acids. In one embodiment, polypeptide distinguishes form a sequence as indicated in Table IIA or IIB, columns 5 or 7, lines 186 to 189 and/or lines 633 and 634, by more than 5, 6, 7, 8 or 9 amino acids, preferably by more than 10, 15, 20, 25 or 30 amino acids, even more preferred are more than 40, 50, or 60 amino acids and, preferably, the sequence of the polypeptide of the invention distinguishes from a sequence as indicated in Table IIA or IIB, columns 5 or 7, lines 186 to 189 and/or lines 633 and 634, by not more than 80% or 70% of the amino acids, preferably not more than 60% or 50%, more preferred not more than 40% or 30%, even more preferred not more than 20% or 10%. In an other embod iment, said polypeptide of the invention does not consist of a sequence as indicated in Table IIA or IIB, columns 5 or 7, lines 186 to 189 and/or lines 633 and 634.

**[0292.0.0.22]** for the disclosure of this paragraph see [0292.0.0.0] above.

**[0293.0.22.22]** In one embodiment, the invention relates to polypeptide conferring an increase in the respective fine chemical in an organism or part being encoded by the nucleic acid molecule of the invention or by a nucleic acid molecule used in the process of the invention. In one embodiment, the polypeptide of the invention has a sequence which distinguishes from a sequence as indicated in Table IIA or IIB, lines 186 to 189 and/or lines 633 and 634, by one or more amino acids. In an other embodiment, said polypeptide of the invention does not consist of the sequence as indicated in Table IIA or IIB, columns 5 or 7, lines 186 to 189 and/or lines 633 and 634. In a further embodiment, said polypeptide of the present invention is less than 100%, 99,999%, 99,99%, 99,9% or 99% identical. In one embodiment, said polypeptide does not consist of the sequence encoded by a nucleic acid molecules as indicated in Table IA or IB, columns 5 or 7, lines 186 to 189 and/or lines 633 and 634.

**[0294.0.22.22]** In one embodiment, the present invention relates to a polypeptide having an activity of a protein as indicated in Table IIA or IIB, column 3, lines 186 to 189 and/or lines 633 and 634, which distinguishes over a sequence as indicated in Table IIA or IIB, columns 5 or 7, lines 186 to 189 and/or lines 633 and 634 by one or more amino acids, preferably by more than 5, 6, 7, 8 or 9 amino acids, preferably by more than 10, 15, 20, 25 or 30 amino acids, even more preferred are more than 40, 50, or 60 amino acids but even more preferred by less than 70% of the amino acids, more preferred by less than 50%, even more preferred my less than 30% or 25%, more preferred are 20% or 15%, even more preferred are less than 10%.

**[0295.0.0.22] to [0297.0.0.22]** for the disclosure of the paragraphs [0295.0.0.22] to [0297.0.0.22] see paragraphs [0295.0.0.0] to [0297.0.0.0] above.

**[00297.1.22.22]** Non-polypeptide of the invention-chemicals are e.g. polypeptides having not the activity of a polypeptide indicated in Table II, columns 5 or 7, lines 186 to 189 and/or lines 633 and 634.

**[0298.0.22.22]** A polypeptide of the invention can participate in the process of the present invention. The polypeptide or a portion thereof comprises preferably an amino acid sequence, which is sufficiently homologous to an amino acid sequence as indicated in Table II, columns 5 or 7, lines 186 to 189 and/or lines 633 and 634, such that the protein or portion thereof maintains the ability to confer the activity of the present invention. The portion of the protein is preferably a biologically active portion as described herein. Preferably, the polypeptide used in the process of the invention has an amino acid sequence identical to a sequence as indicated in Table II, columns 5 or 7, lines 186 to 189 and/or lines 633 and 634.

**[0299.0.22.22]** Further, the polypeptide can have an amino acid sequence which is encoded by a nucleotide sequence which hybridizes, preferably hybridizes under stringent conditions as described above, to a nucleotide sequence of the nucleic acid molecule of the present invention. Accordingly, the polypeptide has an amino acid sequence which is encoded by a nucleotide sequence that is at least about 35%, 40%, 45%, 50%, 55%, 60%, 65% or 70%, preferably at least about 75%, 80%, 85% or 90, and more preferably at least about 91%, 92%, 93%, 94% or 95%, and even more preferably at least about 96%, 97%, 98%, 99% or more homologous to one of the amino acid sequences as shown in Table II, columns 5 or 7, lines 186 to 189 and/or lines 633 and 634. The preferred polypeptide of the present invention preferably possesses at least one of the activities according to the invention and described herein. A preferred polypeptide of the present invention includes an amino acid sequence encoded by a nucleotide sequence which hybridizes, preferably hybridizes under stringent conditions, to a nucleotide sequence of Table I, columns 5 or 7, lines 186 to 189 and/or lines 633 and 634 or which is homologous thereto, as defined above.

**[0300.0.22.22]** Accordingly the polypeptide of the present invention can vary from the sequences shown in table II, columns 5 or 7, lines 186 to 189 and/or lines 633 and 634, in amino acid sequence due to natural variation or mutagenesis, as described in detail herein. Accordingly, the polypeptide comprise an amino acid sequence which is at least about 35%, 40%, 45%, 50%, 55%, 60%, 65% or 70%, preferably at least about 75%, 80%, 85% or 90, and more preferably at least about 91%, 92%, 93%, 94% or 95%, and most preferably at least about 96%, 97%, 98%, 99% or more homologous to an entire amino acid sequence shown in table IIA or IIB, columns 5 or 7, lines 186 to 189 and/or lines 633 and 634.

**[0301.0.0.22]** for the disclosure of this paragraph see [0301.0.0.0] above.

**[0302.0.22.22]** Biologically active portions of an polypeptide of the present invention include peptides comprising amino acid sequences derived from the amino acid sequence of the polypeptide of the present invention or used in the process of the present invention, e.g., the amino acid sequence shown in table II, columns 5 or 7, lines 186 to 189 and/or lines 633 and 634, or the amino acid sequence of a protein homologous thereto, which include fewer amino acids than a full length polypeptide of the present invention or used in the process of the present invention or the full length protein which is homologous to an polypeptide of the present invention or used in the process of the present invention depicted herein, and exhibit at least one activity of polypeptide of the present invention or used in the process of the present invention.

**[0303.0.0.22]** for the disclosure of this paragraph see [0303.0.0.0] above.

**[0304.0.22.22]** Manipulation of the nucleic acid molecule of the invention may result in the production of a protein having essentially the activity of the polypeptides as indicated in Table II, column 3, lines lines 186 to 189 and/or lines 633 and 634, but having differences in the sequence from said wild-type protein. These proteins may be improved in efficiency or activity, may be present in greater numbers in the cell than is usual, or may be decreased in efficiency or activity in relation to the wild type protein.

**[0305.0.0.22] to [0308.0.0.22]** for the disclosure of the paragraphs [0305.0.0.22] to [0308.0.0.22] see paragraphs [0305.0.0.0] to [0308.0.0.0] above.

**[0306.1.0.22]** %

**[0309.0.22.22]** In one embodiment, an reference to a " protein (= polypeptide) of the invention or as indicated in Table II, columns 5 or 7, lines 186 to 189 and/or lines 633 and 634, refers to a polypeptide having an amino acid sequence corresponding to the polypeptide of the invention or used in the process of the invention, whereas a "non-polypeptide of the invention" or "other polypeptide" not being indicated in Table II, columns 5 or 7, lines 186 to 189 and/or lines 633 and 634, refers to a polypeptide having an amino acid sequence corresponding to a protein which is not substantially homologous a polypeptide of the invention, preferably which is not substantially homologous to a as indicated in Table II, columns 5 or 7, lines 186 to 189 and/or lines 633 and 634, e.g., a protein which does not confer the activity described herein or annotated or known for as indicated in Table II, column 3, lines 186 to 189 and/or lines 633 and 634 and which is derived from the same or a different organism. In one embodiment, a "non-polypeptide of the invention" or "other polypeptide" not being indicated in Table II, columns 5 or 7, lines 186 to 189 and/or lines 633 and 634, does not confer an increase of the respective fine chemical in an organism or part thereof.

**[0310.0.0.22] to [0334.0.0.22]** for the disclosure of the paragraphs [0310.0.0.22] to [0334.0.0.22] see paragraphs [0310.0.0.0] to [0334.0.0.0] above.

**[0335.0.22.22]** The dsRNAi method has proved to be particularly effective and advantageous for reducing the expression of the nucleic acid sequences of the table I, columns 5 or 7, lines 186 to 189 and/or lines 633 and 634, and/or homologs thereof. As described inter alia in WO 99/32619, dsRNAi approaches are clearly superior to traditional antisense approaches. The invention therefore furthermore relates to double-stranded RNA molecules (dsRNA molecules) which, when introduced into an organism, advantageously into a plant (or a cell, tissue, organ or seed derived therefrom), bring about altered metabolic activity by the reduction in the expression of the nucleic acid sequences of the table I, columns 5 or 7, lines 186 to 189 and/or lines 633 and 634, and/or homologs thereof. In a double-stranded RNA molecule for reducing the expression of an protein encoded by a nucleic acid sequence of one of the table I, columns 5 or 7, lines 186 to 189 and/or lines 633 and 634, and/or homologs thereof, one of the two RNA strands is essentially identical to at least part of a nucleic acid sequence, and the respective other RNA strand is essentially identical to at least part of the complementary strand of a nucleic acid sequence.

**[0336.0.0.22] to [0342.0.0.22]** for the disclosure of the paragraphs [0336.0.0.22] to [0342.0.0.22] see paragraphs [0336.0.0.0] to [0342.0.0.0] above.

**[0343.0.22.22]** As has already been described, 100 % sequence identity between the dsRNA and a gene transcript of a nucleic acid sequence of one of the sequences shown in table I, columns 5 or 7, lines 186 to 189 and/or lines 633 and 634, or it's homolog is not necessarily required in order to bring about effective reduction in the expression. The advantage is, accordingly, that the method is tolerant with regard to sequence deviations as may be present as a consequence of genetic mutations, polymorphisms or evolutionary divergences. Thus, for example, using the dsRNA, which has been generated starting from a sequence of one of sequences shown in table I, columns 5 or 7, lines 186 to 189 and/or lines 633 and 634, or homologs thereof of the one organism, may be used to suppress the corresponding expression in another organism.

**[0344.0.0.22] to [0361.0.0.22]** for the disclosure of the paragraphs [0344.0.0.22] to [0361.0.0.22] see paragraphs [0344.0.0.0] to [0361.0.0.0] above.

**[0362.0.22.22]** Accordingly the present invention relates to any cell transgenic for any nucleic acid characterized as part of the invention, e.g. conferring the increase of the respective fine chemical in a cell or an organism or a part thereof, e.g. the nucleic acid molecule of the invention, the nucleic acid construct of the invention, the antisense molecule of the invention, the vector of the invention or a nucleic acid molecule encoding the polypeptide of the invention, e.g. the polypeptide as indicated in Table II, columns 5 or 7, lines 186 to 189 and/or lines 633 and 634. Due to the above mentioned activity the fine chemical content in a cell or an organism is increased. For example, due to modulation or manipulation, the cellular activity of the polypeptide of the invention or nucleic acid molecule of the invention is increased, e.g. due to an increased expression or specific activity of the subject matters of the invention in a cell or an organism or a part thereof. In one embodiment, transgenic for a polypeptide having an activity of a polypeptide as indicated in Table II, columns 5 or 7, lines 186 to 189 and/or lines 633 and 634, means herein that due to modulation or manipulation of the genome, an activity as annotated for a polypeptide as indicated in Table II, column 3, lines 186 to 189 and/or lines 633 and 634, e.g. having a sequence as indicated in Table II, columns 5 or 7, lines 186 to 189 and/or lines 633 and 634, is increased in a cell or an organism or a part thereof. Examples are described above in context with the process of the invention.

**[0363.0.0.22]** for the disclosure of this paragraph see paragraph [0363.0.0.0] above.

**[0364.0.22.22]** A naturally occurring expression cassette - for example the naturally occurring combination of a promoter of a polypeptide of the invention as indicated in Table II, column 3 and/or 5, lines 186 to 189 and/or lines 633 and 634, with the corresponding protein-encoding sequence as indicated in Table I, column 3 and/or 5, lines 186 to 189 and/or lines 633 and 634.

**[0365.0.0.22] to [0373.0.0.22]** for the disclosure of the paragraphs [0365.0.0.22] to [0373.0.0.22] see paragraphs [0365.0.0.0] to [0373.0.0.0] above.

**[0374.0.22.22]** Transgenic plants comprising the lipid, preferably a glycolipid, glycolipid containing galactose, more preferably a galactolipide and/or cerebroside synthesized in the process according to the invention can be marketed directly without isolation of the compounds synthesized. In the process according to the invention, plants are understood as meaning all plant parts, plant organs such as leaf, stalk, root, tubers or seeds or propagation material or harvested material or the intact plant. In this context, the seed encompasses all parts of the seed such as the seed coats, epidermal cells, seed cells, endosperm or embryonic tissue. Lipid, preferably a glycolipid, glycolipid containing galactose, more preferably a galactolipide and/or cerebroside produced by this process can be harvested by harvesting the organisms either from the culture in which they grow or from the field. This can be done via expressing, grinding and/or extraction, salt precipitation and/or ion-exchange chromatography or other chromatographic methods of the plant parts, preferably the plant seeds, plant fruits, plant tubers and the like.

**[0375.0.0.22] and [0376.0.0.22]** for the disclosure of the paragraphs [0375.0.0.22] and [0376.0.0.22] see paragraphs [0375.0.0.0] and [0376.0.0.0] above.

**[0377.0.22.22]** Accordingly, the present invention relates also to a process according to the present invention whereby the produced lipid, preferably a glycolipid, glycolipid containing galactose, more preferably a galactolipide and/or cerebroside or the produced fine chemical is isolated.

**[0378.0.22.22]** In this manner, more than 50% by weight, advantageously more than 60% by weight, preferably more than 70% by weight, especially preferably more than 80% by weight, very especially preferably more than 90% by weight, of the lipid, preferably a glycolipid, glycolipid containing galactose, more preferably a galactolipide and/or cerebrosideproduced in the process can be isolated. The resulting lipid, preferably a glycolipid, glycolipid containing galactose, more preferably a galactolipide and/or cerebroside can, if appropriate, subsequently be further purified, if desired mixed with other active ingredients such as vitamins, amino acids, carbohydrates, antibiotics and the like, and, if appropriate, formulated.

**[0379.0.22.22]** In one embodiment, the fine chemical, in particular, lipid, preferably a glycolipid, glycolipid containing galactose, more preferably a galactolipide and/or cerebroside, is a mixture comprising of one or more the respective fine chemicals. In one embodiment, the respective fine chemical means here lipid, preferably a glycolipid, glycolipid containing galactose, more preferably a galactolipide and/or cerebroside. In one embodiment, lipid, preferably a glycolipid, glycolipid containing galactose, more preferably a galactolipide and/or cerebroside means here a mixture of the respective fine chemicals.

**[0380.0.22.22]** The lipid, preferably a glycolipid, glycolipid containing galactose, more preferably a galactolipide and/or cerebroside obtained in the process are suitable as starting material for the synthesis of further products of value. For example, they can be used in combination with each other or alone for the production of pharmaceuticals, foodstuffs, animal feeds or cosmetics. The fine chemicals of the invention can be used as liposomes and drug carrier in pharmaceutical preparations, called Galactosomes^{®} (US patent numbers 5688528; 5716639, 6022561 and 6,068,860).
Galactopyranosyl is used for production of oligosaccharides, which themselves have no antigenic action, but have antigenic properties if they are bonded to a suitable high-molecular carrier. For example as "haptens" which show a sialyl-galacto pyranosyl linkageas disclosed in US 5,296,594 and 5,854,218 or 4,686,193.
Galactose or galactose derivatives are supplements in animal feed composition for reducing colonization of animal intestines by Salmonella and other bacterial pathogens (US 6,126,961). Oligosaccharides containing galactose for preventing the invasion and infection of mammal cells by pathogens and for fighting diseases caused by such pathogens are disclosed in US 20050004070.
Accordingly, the present invention relates to a method for the production of a pharmaceuticals, food stuff, animal feeds, nutrients or cosmetics comprising the steps of the process according to the invention, including the isolation of the lipid, preferably a glycolipid, glycolipid containing galactose, more preferably a galactolipide and/or cerebroside composition produced or the fine chemical produced if desired and formulating the product with a pharmaceutical acceptable carrier or formulating the product in a form acceptable for an application in agriculture. A further embodiment according to the invention is the use of the lipid, preferably a glycolipid, glycolipid containing galactose, more preferably a galactolipide and/or cerebroside produced in the process or of the transgenic organisms in animal feeds, foodstuffs, medicines, food supplements, cosmetics or pharmaceuticals.

**[0381.0.0.22] and [0382.0.0.22]** for the disclosure of the paragraphs [0381.0.0.22] and [0382.0.0.22] see paragraphs [0381.0.0.0] and [0382.0.0.0] above.

**[0383.0.22.22]** For preparing lipid, preferably a glycolipid, glycolipid containing galactose, more preferably a galactolipide and/or cerebrosidecompound-containing fine chemicals, in particular the fine chemical, it is possible to use as lipid, preferably a glycolipid, glycolipid containing galactose, more preferably a galactolipide and/or cerebrosidesource organic compounds such as, for example, and/or lipids comprising fatty acids such as fatty acids having a carbon back bone between C₁₀- to C₁₆- carbon atoms and/or small organic acids such acetic acid, propionic acid or butanoic acid as precursor compounds.

**[0384.0.0.22]** for the disclosure of this paragraph see [0384.0.0.0] above.

**[0385.0.22.22]** The fermentation broths obtained in this way, containing in particular lipid, preferably a glycolipid, glycolipid containing galactose, more preferably a galactolipide and/or cerebroside in mixtures with other lipids, fats and/or oils, normally have a dry matter content of from 7.5 to 25% by weight. Depending on requirements, the biomass can be removed entirely or partly by separation methods, such as, for example, centrifugation, filtration, decantation or a combination of these methods, from the fermentation broth or left completely in it. The fermentation broth can then be thickened or concentrated by known methods, such as, for example, with the aid of a rotary evaporator, thin-film evaporator, falling film evaporator, by reverse osmosis or by nanofiltration. This concentrated fermentation broth can then be worked up by freeze-drying, spray drying, spray granulation or by other processes.

**[0386.0.22.22]** However, it is also possible to purify the lipid, preferably a glycolipid, glycolipid containing galactose, more preferably a galactolipide and/or cerebroside produced further. For this purpose, the product-containing composition is subjected for example to a thin layer chromatography on silica gel plates or to a chromatography such as a Florisil column (Bouhours J.F., J. Chromatrogr. 1979, 169, 462), in which case the desired product or the impurities are retained wholly or partly on the chromatography resin. These chromatography steps can be repeated if necessary, using the same or different chromatography resins. The skilled worker is familiar with the choice of suitable chromatography resins and their most effective use. Alternatively the lipid purfication can be performed as described by Dörmann et al., 1999, Science 284, 2181-2184.

**[0387.0.0.22] to [0392.0.0.22]** for the disclosure of the paragraphs [0387.0.0.22] to [0392.0.0.22] see paragraphs [0387.0.0.0] to [0392.0.0.0] above.

**[0393.0.22.22]** In one embodiment, the present invention relates to a method for the identification of a gene product conferring an increase in the fine chemical production in a cell, comprising the following steps:
a. contacting,̅ e.g. hybridising, the nucleic acid molecules of a sample, e.g. cells, tissues, plants or microorganisms or a nucleic acid library , which can contain a candidate gene encoding a gene product conferring an increase in the fine chemical after expression, with the nucleic acid molecule of the present invention;
b. identifying the nucleic acid molecules, which hybridize under relaxed stringent conditions with the nucleic acid molecule of the present invention in particular to the nucleic acid molecule sequence shown in table I, columns 5 or 7, lines 186 to 189 and/or lines 633 and 634, preferably in Table I B, columns 5 or 7, lines 186 to 189 and/or lines 633 and 634, and, optionally, isolating the full length cDNA clone or complete genomic clone;
c. introducing the candidate nucleic acid molecules in host cells, preferably in a plant cell or a microorganism, appropriate for producing the fine chemical;
d. expressing the identified nucleic acid molecules in the host cells;
e. assaying the fine chemical level in the host cells; and
f. identifying the nucleic acid molecule and its gene product which expression confers an increase in the fine chemical level in the host cell after expression compared to the wild type.

**[0394.0.0.22] to [0399.0.0.22]** for the disclosure of the paragraphs [0394.0.0.22] to [0399.0.0.22] see paragraphs [0394.0.0.0] to [0399.0.0.0] above.

**[00399.1.22.22]** One can think to screen for increased production of the respective fine chemical by for example searching for a resistance to a drug blocking the synthesis of the fine chemical and looking whether this effect is dependent on the activity or expression of a polypeptide as indicated in Table II, columns 5 or 7, lines 186 to 189 and/or lines 633 and 634, or a homolog thereof, e.g. comparing the phenotype of nearly identical organisms with low and high activity of a protein as indicated in Table II, columns 5 or 7, lines 186 to 189 and/or lines 633 and 634, after incubation with the drug.

**[0400.0.0.22] to [0416.0.0.22]** for the disclosure of the paragraphs [0400.0.0.22] to [0416.0.0.22] see paragraphs [0400.0.0.0] to [0416.0.0.0] above.

**[0417.0.22.22]** The nucleic acid molecule of the invention, the vector of the invention or the nucleic acid construct of the invention may also be useful for the production of organisms resistant to inhibitors of the fatty acid production biosynthesis pathways. In particular, the overexpression of the polypeptide of the present invention may protect an organism such as a microorganism or a plant against inhibitors, which block the fine chemical synthesis in said organism. Examples of inhibitors or herbicides blocking the fine chemical synthesis in organism such as microorganism or plants might for example be phenylpyridazinones, such as Norflurazon

**[0418.0.0.22] to [0423.0.0.22]** for the disclosure of the paragraphs [0418.0.0.22] to [0423.0.0.22] see paragraphs [0418.0.0.0] to [0423.0.0.0] above.

**[0424.0.22.22]** Accordingly, the nucleic acid of the invention, the polypeptide of the invention, the nucleic acid construct of the invention, the organisms, the host cell, the microorganisms, the plant, plant tissue, plant cell, or the part thereof of the invention, the vector of the invention, the agonist identified with the method of the invention, the nucleic acid molecule identified with the method of the present invention, can be used for the production of the fine chemical or of the fine chemical and one or more other lipid, preferably a glycolipid, glycolipid containing galactose, more preferably a galactolipide and/or cerebroside and mixtures thereof or mixtures of other glycolipides. Accordingly, the nucleic acid of the invention, or the nucleic acid molecule identified with the method of the present invention or the complement sequences thereof, the polypeptide of the invention, the nucleic acid construct of the invention, the organisms, the host cell, the microorganisms, the plant, plant tissue, plant cell, or the part thereof of the invention, the vector of the invention, the antagonist identified with the method of the invention, the antibody of the present invention, the antisense molecule of the present invention, can be used for the reduction of the fine chemical in a organism or part thereof, e.g. in a cell.

**[0425.0.0.22] to [0435.0.0.22]** for the disclosure of the paragraphs [0425.0.0.22] to [0435.0.0.22] see paragraphs [0425.0.0.0] to [0435.0.0.0] above.

**[0436.0.22.22]** An *in vivo* mutagenesis of organisms such as Saccharomyces, Mortierella, Escherichia and others mentioned above, which are beneficial for the production of lipid, preferably a glycolipid, glycolipid containing galactose, more preferably a galactolipide and/or cerebroside can be carried out by passing a plasmid DNA (or another vector DNA) containing the desired nucleic acid sequence or nucleic acid sequences through E. coli and other microorganisms (for example Bacillus spp. or yeasts such as Saccharomyces cerevisiae) which are not capable of maintaining the integrity of its genetic information. Usual mutator strains have mutations in the genes for the DNA repair system [for example mutHLS, mutD, mutT and the like; for comparison, see Rupp, W.D. (1996) DNA repair mechanisms in Escherichia coli and Salmonella, pp. 2277-2294, ASM: Washington]. The skilled worker knows these strains. The use of these strains is illustrated for example in Greener, A. and Callahan, M. (1994) Strategies 7; 32-34.

**[0436.1.22.22]** In-vitro mutation methods such as increasing the spontaneous mutation rates by chemical or physical treatment are well known to the skilled person. Mutagens like 5-bromo-uracil, N-methyl-N-nitro-N-nitrosoguanidine (= NTG), ethyl methanesulfonate (= EMS), hydroxylamine and/or nitrous acid are widly used as chemical agents for random in-vitro mutagensis. The most common physical method for mutagensis is the treatment with UV irradiation. Another random mutagenesis technique is the error-prone PCR for introducing amino acid changes into proteins. Mutations are deliberately introduced during PCR through the use of error-prone DNA polymerases and special reaction conditions known to a person skilled in the art. For this method randomized DNA sequences are cloned into expression vectors and the resulting mutant libraries screened for altered or improved protein activity as described below.
Site-directed mutagensis method such as the introduction of desired mutations with an M13 or phagemid vector and short oligonucleotides primers is a well-known approach for site-directed mutagensis. The clou of this method involves cloning of the nucleic acid sequence of the invention into an M13 or phagemid vector, which permits recovery of single-stranded recombinant nucleic acid sequence. A mutagenic oligonucleotide primer is then designed whose sequence is perfectly complementary to nucleic acid sequence in the region to be mutated, but with a single difference: at the intended mutation site it bears a base that is complementary to the desired mutant nucleotide rather than the original. The mutagenic oligonucleotide is then allowed to prime new DNA synthesis to create a complementary full-length sequence containing the desired mutation. Another site-directed mutagensis method is the PCR mismatch primer mutagensis method also known to the skilled person. Dpnl site-directed mutagensis is a further known method as described for example in the Stratagene QuickchangeTM site-directed mutagenesis kit protocol. A huge number of other methods are also known and used in common practice.
Positive mutation events can be selected by screening the organisms for the production of the desired fine chemical.

**[0437.0.0.22] to [0453.0.0.22] and [0451.1.0.22]** for the disclosure of the paragraphs [0437.0.0.22] to [0453.0.0.22] and [0451.1.0.22] see paragraphs [0437.0.0.0] to [0453.0.0.0] and [0451.1.0.0] above.

**[0454.0.22.22]** Example 8: Analysis of the effect of the nucleic acid molecule on the production of the lipid, preferably a glycolipid, glycolipid containing galactose, more preferably a galactolipide and/or cerebroside

**[0455.0.0.22] and [0456.0.0.22]** for the disclosure of the paragraphs [0455.0.0.22] and [0456.0.0.22] see [0455.0.5.5] and [0456.0.0.0]above.

**[0457.0.22.22]** Example 9: Purification of lipid, preferably a glycolipid, glycolipid containing galactose, more preferably a galactolipide and/or cerebroside can be carried out by thin layer chromatographie as for example described by Dörmann et al., 1999, Science 284, 2181-2184.

**[0458.0.22.22]** One example is the analysis of lipid, preferably a glycolipid, glycolipid containing galactose, more preferably a galactolipide and/or cerebroside (abbreviations: FAME, fatty acid methyl ester; GC-MS, gas liquid chromatography/mass spectrometry; TAG, triacylglycerol; TLC, thin-layer chromatography).
The unambiguous detection for the presence of fatty acid products can be obtained by analyzing recombinant organisms using analytical standard methods: GC, GC-MS or
TLC, as described on several occasions by Christie and the references therein (1997, in: Advances on Lipid Methodology, Fourth Edition: Christie, Oily Press, Dundee, 119-169; 1998, Gaschromatographie-Massenspektrometrie-Verfahren [Gas chromatography/mass spectrometric methods], Lipide 33:343-353).
The total fatty acids produced in the organism for example in yeasts used in the inventive process can be analysed for example according to the following procedure:
The material such as yeasts, E. coli or plants to be analyzed can be disrupted by sonication, grinding in a glass mill, liquid nitrogen and grinding or via other applicable methods. After disruption, the material must be centrifuged (1000 x g, 10 min., 4°C) and washed once with 100 mM NaHCO₃, pH 8.0 to remove residual medium and fatty acids. For preparation of the fatty acid methyl esters (FAMES) the sediment is resuspended in distilled water, heated for 10 minutes at 100°C, cooled on ice and recentrifuged, followed by extraction for one hour at 90°C in 0.5 M sulfuric acid in methanol with 2% dimethoxypropane, which leads to hydrolyzed oil and lipid compounds, which give transmethylated lipids.
The FAMES are then extracted twice with 2 ml petrolether, washed once with 100 mM NaHCO₃, pH 8.0 and once with distilled water and dried with Na₂SO₄. The organic solvent can be evaporated under a stream of Argon and the FAMES were dissolved in 50 µl of petrolether. The samples can be separated on a ZEBRON ZB-Wax capillary column (30 m, 0,32 mm, 0,25 µm; Phenomenex) in a Hewlett Packard 6850 gas chromatograph with a flame ionisation detector. The oven temperature is programmed from 70°C (1 min. hold) to 200°C at a rate of 20°C/min., then to 250°C (5 min. hold) at a rate of 5°C/min and finally to 260°C at a rate of 5°C/min. Nitrogen is used as carrier gas (4,5 ml/min. at 70°C). The identity of the resulting fatty acid methyl esters can be identified by comparison with retention times of FAME standards, which are available from commercial sources (i.e. Sigma).
Plant material is initially homogenized mechanically by comminuting in a pestle and mortar to make it more amenable to extraction.
This is followed by heating at 100°C for 10 minutes and, after cooling on ice, by resedimentation. The cell sediment is hydrolyzed for one hour at 90°C with 1 M methanolic sulfuric acid and 2% dimethoxypropane, and the lipids are transmethylated. The resulting fatty acid methyl esters (FAMEs) are extracted in petroleum ether. The extracted FAMEs are analyzed by gas liquid chromatography using a capillary column (Chrompack, WCOT Fused Silica, CP-Wax-52 CB, 25 m, 0.32 mm) and a temperature gradient of from 170°C to 240°C in 20 minutes and 5 minutes at 240°C. The identity of the fatty acid methyl esters is confirmed by comparison with corresponding FAME standards (Sigma). The identity and position of the double bond can be analyzed further by suitable chemical derivatization of the FAME mixtures, for example to give 4,4-dimethoxyoxazoline derivatives (Christie, 1998) by means of GC-MS.
The methodology is described for example in Napier and Michaelson, 2001, Lipids. 36(8):761-766; Sayanova et al., 2001, Journal of Experimental Botany. 52(360):1581-1585, Sperling et al., 2001, Arch. Biochem. Biophys. 388(2): 293-298 and Michaelson et al., 1998, FEBS Letters. 439(3): 215-218.

**[0459.0.22.22]** If required and desired, further chromatography steps with a suitable resin may follow. Advantageously the lipid, preferably a glycolipid, glycolipid containing galactose, more preferably a galactolipide and/or cerebroside can be further purified with a so-called RTHPLC. As eluent different an acetonitrile/water or chloroform/acetonitrile mixtures are advantageously is used. For the analysis of the fatty acids an ELSD detector (evaporative light-scattering detector) is used. MPLC, dry-flash chromatography or thin layer chromatography are other beneficial chromatography methods for the purification of glycolipids. If necessary, these chromatography steps may be repeated, using identical or other chromatography resins. The skilled worker is familiar with the selection of suitable chromatography resin and the most effective use for a particular molecule to be purified.

**[0460.0.22.22]** In addition depending on the produced fine chemical purification is also possible with cristalisation or destilation. Both methods are well known to a person skilled in the art.

**[0461.0.22.22]** Example 10: Cloning SEQ ID NO: 14354 for the expression in plants

**[0462.0.0.22] to [0466.0.0.22]** for the disclosure of the paragraphs [0462.0.0.22] to [0466.0.0.22] see [0462.0.0.0] to [0466.0.0.0] above.

**[0467.0.22.22]** The following primer sequences were selected for the gene SEQ ID NO: 14354:
i) forward primer (SEQ ID NO: 14356)
   atggcggttg cgatcaaaaa gga
ii) reverse primer (SEQ **ID** NO: 14357)
   tcaattgata aatgtacttt caatgatg

**[0468.0.0.22] to [0479.0.0.22]** for the disclosure of the paragraphs [0468.0.0.22] to [0479.0.0.22] see [0468.0.0.0] to [0479.0.0.0] above.

**[0480.0.22.22]** Example 11: Generation of transgenic plants which express SEQ ID NO: 14354

**[0481.0.0.22] to [0513.0.0.22]** for the disclosure of the paragraphs [0481.0.0.22] to [0513.0.0.22] see paragraphs [0482.0.0.0] to [0513.0.0.0] above.

**[0514.0.22.22]** In order to analyze glycolipids being present in the transgenic organism by the means of gas chromatography-mass spectrometry, material from the transgenic organisms have to be extracted and the extracts subsequently being hydrolyzed in the presence of methanol and an inorganic acid, yielding the corresponding fatty acid methyl esters and the respective monosaccharid moietye as its methylhexopyranoside.
Primary and secondary amino functions, hydroxy groups and free carboxylic functions eventually will be trimethylsilylated by reaction with N-Methyl-N-trimethylsilyltrifluoroacetamide, yielding the trimethylsilyl (TMS) derivatives of the methylhexopyranosides formed in the previous hydrolysis step (eg methylgalactopyranoside 4TMS in the case of a galactolipid). These compounds are accessible to gas chromatographic-mass spectrometric analysis.
Therefore, an increased content of the trimethylsilylated methylhexopyranosides directly correlates to an increased content of glycolipids in the transgenic organism.
The results of the different plant analyses can be seen from the table, which follows:

**Table 1**

| **ORF** | **Metabolites** | **Analyte** | **Method** | **Min** | **Max** |
|---|---|---|---|---|---|
| YER173W | Galactolipids | Methylgalacto-pyranosid 4 TMS | GC | 1,20 | 1,70 |
| YLR224W | Galactolipids | Methylgalacto-pyranosid 4 TMS | GC | 1,15 | 1,46 |
| YLR255C | Galactolipids | Methylgalacto-pyranosid 4 TMS | GC | 1,16 | 1,30 |
| b2699 | Galactolipids | Methylgalacto-pyranosid 4 TMS | GC | 1,15 | 1,64 |
| YHR072W | Galactolipids | Methylgalacto-pyranosid 4 TMS | GC | 1.17 | 1.35 |
| b3129 | Galactolipids | Methylgalacto-pyranosid 4 TMS | GC | 1.16 | 1.32 |

**[0515.0.22.22]** Column 2 shows the metabolite Galactolipids and column 3 the analyte analyzed. Columns 5 and 6 shows the ratio of the analyzed metabolite/alnalyte between the transgenic plants and the wild type; Increase of the metabolite: Max: maximal x-fold (normalised to wild type)-Min: minimal x-fold (normalised to wild type). Decrease of the metabolite: Max: maximal x-fold (normalised to wild type) (minimal decrease), Min: minimal x-fold (normalised to wild type) (maximal decrease). Column 4 indicates the analytical method.

**[0516.0.0.22] to [0530.0.0.22]** for the disclosure of the paragraphs [0516.0.0.22] to [0530.0.0.22] see paragraphs [0516.0.0.0] to [0530.0.0.0] above.

**[0530.1.0.22] to [0530.6.0.22]** for the disclosure of the paragraphs [0530.1.0.22] to [0530.6.0.22] see paragraphs [0530.1.0.0] to [0530.6.0.0] above.

**[0531.0.0.22] to [0552.0.0.22]** for the disclosure of the paragraphs [0531.0.0.22] to [0552.0.0.22] see paragraphs [0531.0.0.0] to [0552.0.0.0] above.

**[0552.1.0.22]:** %

**[0552.2.0.22]** for the disclosure of this paragraph see [0552.2.0.0] above.

**[0553.0.22.22]**
1. A process for the production of a lipid, preferably a glycolipid, a glycolipide containing galactose, more preferably a galactolipide and/or a cerebroside, which comprises
   (a) increasing or generating the activity of a protein as indicated in Table II, columns 5 or 7, lines 186 to 189 or 633 and 634, or a functional equivalent thereof in a non-human organism, or in one or more parts thereof; and
   (b) growing the organism under conditions which permit the production of a lipid, preferably a glycolipid, a glycolipide containing galactose, more preferably a galactolipide and/or a cerebrosidein said organism.
2. A process for the production of a lipid, preferably a glycolipid, a glycolipide containing galactose, more preferably a galactolipide and/or a cerebroside, comprising the increasing or generating in an organism or a part thereof the expression of at least one nucleic acid molecule comprising a nucleic acid molecule selected from the group consisting of:
   a) nucleic acid molecule encoding of the polypeptide shown in table II, columns 5 or 7, lines 186 to 189 or 633 and 634, or a fragment thereof, which confers an increase in the amount of lipids, preferably of glycolipids, of glycolipides containing galactose, more preferably of galactolipide and/or cerebrosides in an organism or a part thereof;
   b) nucleic acid molecule comprising of the nucleic acid molecule shown in table I, columns 5 or 7, lines 186 to 189 or 633 and 634;
   c) nucleic acid molecule whose sequence can be deduced from a polypeptide sequence encoded by a nucleic acid molecule of (a) or (b) as a result of the degeneracy of the genetic code and conferring an increase in the amount of lipids, preferably of glycolipids, of glycolipides containing galactose, more preferably of galactolipides and/or cerebrosides in an organism or a part thereof;
   d) nucleic acid molecule which encodes a polypeptide which has at least 50% identity with the amino acid sequence of the polypeptide encoded by the nucleic acid molecule of (a) to (c) and conferring an increase in the amount of lipids, preferably of glycolipids, of glycolipides containing galactose, more preferably of galactolipides and/or cerebrosides in an organism or a part thereof;
   e) nucleic acid molecule which hybidizes with a nucleic acid molecule of (a) to (c) under under stringent hybridisation conditions and conferring an increase in the amount of lipids, preferably of glycolipids, ofa glycolipides containing galactose, more preferably of galactolipides and/or cerebrosides in an organism or a part thereof;
   f) nucleic acid molecule which encompasses a nucleic acid molecule which is obtained by amplifying nucleic acid molecules from a cDNA library or a genomic library using the primers as shown in table III, column 7, lines 186 to 189 or 633 and 634, and conferring an increase in the amount of the fine chemical in an organism or a part thereof;
   g) nucleic acid molecule encoding a polypeptide which is isolated with the aid of monoclonal antibodies against a polypeptide encoded by one of the nucleic acid molecules of (a) to (f) and conferring an increase in the amount of lipids, preferably of glycolipids, of glycolipides containing galactose, more preferably of galactolipides and/or cerebrosides in an organism or a part thereof;
   h) nucleic acid molecule encoding a polypeptide comprising the consensus sequence shown in table IV, column 7, lines 186 to 189 or 633 and 634, and conferring an increase in the amount of the fine chemical in an organism or a part thereof; and
   i) nucleic acid molecule which is obtainable by screening a suitable nucleic acid library under stringent hybridization conditions with a probe comprising one of the sequences of the nucleic acid molecule of (a) to (k) or with a fragment thereof having at least 15 nt, preferably 20 nt, 30 nt, 50 nt, 100 nt, 200 nt or 500 nt of the nucleic acid molecule characterized in (a) to (k) and conferring an increase in the amount of the fine chemical in an organism or a part thereof.
   or comprising a sequence which is complementary thereto.
3. The process of claim 1 or 2, comprising recovering of the free or bound lipid, preferably of a glycolipid, of a glycolipide containing galactose, more preferably of a galactolipide and/or cerebroside.
4. The process of any one of claim 1 to 3, comprising the following steps:
   (a) selecting an organism or a part thereof expressing a polypeptide encoded by the nucleic acid molecule characterized in claim 2;
   (b) mutagenizing the selected organism or the part thereof;
   (c) comparing the activity or the expression level of said polypeptide in the mutagenized organism or the part thereof with the activity or the expression of said polypeptide of the selected organisms or the part thereof;
   (d) selecting the mutated organisms or parts thereof, which comprise an increased activity or expression level of said polypeptide compared to the selected organism or the part thereof;
   (e) optionally, growing and cultivating the organisms or the parts thereof; and
   (f) recovering, and optionally isolating, the free or bound palmitic acid produced by the selected mutated organisms or parts thereof.
5. The process of any one of claims 1 to 4, wherein the activity of said protein or the expression of said nucleic acid molecule is increased or generated transiently or stably.
6. An isolated nucleic acid molecule comprising a nucleic acid molecule selected from the group consisting of:
   a) nucleic acid molecule encoding of the polypeptide shown in table II, columns 5 or 7, lines 186 to 189 or 633 and 634, or a fragment thereof, which confers an increase in the amount of lipids, preferably of glycolipids, of glycolipides containing galactose, more preferably of galactolipides and/or cerebrosides in an organism or a part thereof;
   b) nucleic acid molecule comprising of the nucleic acid molecule shown in table I, columns 5 or 7, lines 186 to 189 or 633 and 634;
   c) nucleic acid molecule whose sequence can be deduced from a polypeptide sequence encoded by a nucleic acid molecule of (a) or (b) as a result of the degeneracy of the genetic code and conferring an increase in the amount of lipids, preferably of glycolipids, of glycolipides containing galactose, more preferably of galactolipides and/or cerebrosides in an organism or a part thereof;
   d) nucleic acid molecule which encodes a polypeptide which has at least 50% identity with the amino acid sequence of the polypeptide encoded by the nucleic acid molecule of (a) to (c) and conferring an increase in the amount of lipids, preferably of glycolipids, of glycolipides containing galactose, more preferably of galactolipides and/or cerebrosides in an organism or a part thereof;
   e) nucleic acid molecule which hybidizes with a nucleic acid molecule of (a) to (c) under stringent hybridisation conditions and conferring an increase in the amount of lipids, preferably of glycolipids, of glycolipides containing galactose, more preferably of galactolipides and/or cerebrosides in an organism or a part thereof;
   f) nucleic acid molecule which encompasses a nucleic acid molecule which is obtained by amplifying nucleic acid molecules from a cDNA library or a genomic library using the primers as shown in table III, column 7, lines 186 to 189 or 633 and 634, and conferring an increase in the amount of the fine chemical in an organism or a part thereof;
   g) nucleic acid molecule encoding a polypeptide which is isolated with the aid of monoclonal antibodies against a polypeptide encoded by one of the nucleic acid molecules of (a) to (f) and conferring an increase in the amount of lipids, preferably of glycolipids, of glycolipides containing galactose, more preferably of galactolipides and/or cerebrosides in an organism or a part thereof;
   h) nucleic acid molecule encoding a polypeptide comprising the consensus sequence shown in table IV, column 7, lines 186 to 189 or 633 and 634, and conferring an increase in the amount of the fine chemical in an organism or a part thereof; and
   i) nucleic acid molecule which is obtainable by screening a suitable nucleic acid library under stringent hybridization conditions with a probe comprising one of the sequences of the nucleic acid molecule of (a) to (k) or with a fragment thereof having at least 15 nt, preferably 20 nt, 30 nt, 50 nt, 100 nt, 200 nt or 500 nt of the nucleic acid molecule characterized in (a) to (k) and conferring an increase in the amount of the fine chemical in an organism or a part thereof.
   whereby the nucleic acid molecule distinguishes over the sequence as shown in table IA, columns 5 or 7, lines 186 to 189 or 633 and 634, by one or more nucleotides.
7. A nucleic acid construct which confers the expression of the nucleic acid molecule of claim 6, comprising one or more regulatory elements.
8. A vector comprising the nucleic acid molecule as claimed in claim 6 or the nucleic acid construct of claim 7.
9. The vector as claimed in claim 8, wherein the nucleic acid molecule is in operable linkage with regulatory sequences for the expression in a prokaryotic or eukaryotic, or in a prokaryotic and eukaryotic, host.
10. A host cell, which has been transformed stably or transiently with the vector as claimed in claim 8 or 9 or the nucleic acid molecule as claimed in claim 6 or the nucleic acid construct of claim 7 or produced as described in claim any one of claims 2 to 5.
11. The host cell of claim 10, which is a transgenic host cell.
12. The host cell of claim 10 or 11, which is a plant cell, an animal cell, a microorganism, or a yeast cell, a fungus cell, a prokaryotic cell, an eukaryotic cell or an archaebacterium.
13. A process for producing a polypeptide, wherein the polypeptide is expressed in a host cell as claimed in any one of claims 10 to 12.
14. A polypeptide produced by the process as claimed in claim 13 or encoded by the nucleic acid molecule as claimed in claim 6 whereby the polypeptide distinguishes over the sequence as shown in table IIA, columns 5 or 7, lines 186 to 189 or 633 and 634, by one or more amino acids
15. An antibody, which binds specifically to the polypeptide as claimed in claim 14.
16. A plant tissue, propagation material, harvested material or a plant comprising the host cell as claimed in claim 12 which is plant cell or an Agrobacterium.
17. A method for screening for agonists and antagonists of the activity of a polypeptide encoded by the nucleic acid molecule of claim 6 conferring an increase in the amount of lipids, preferably of glycolipids, of glycolipides containing galactose, more preferably of galactolipides and/or cerebrosides in an organism or a part thereof comprising:
   (a) contacting cells, tissues , plants or microorganisms which express the a polypeptide encoded by the nucleic acid molecule of claim 6 conferring an increase in the amount of lipids, preferably of glycolipids, of glycolipides containing galactose, more preferably of galactolipides and/or cerebrosides in an organism or a part thereof with a candidate compound or a sample comprising a plurality of compounds under conditions which permit the expression the polypeptide;
   (b) assaying the lipids, preferably of glycolipids, of glycolipides containing galactose, more preferably of galactolipides and/or cerebrosides level or the polypeptide expression level in the cell, tissue, plant or microorganism or the media the cell, tissue, plant or microorganisms is cultured or maintained in; and
   (c) identifying a agonist or antagonist by comparing the measured lipid, preferably the glycolipid, the glycolipide containing galactose, more preferably the galactolipide and/or cerebrosidelevel or polypeptide expression level with a standard lipid, preferably a glycolipid, a glycolipide containing galactose, more preferably a galactolipide and/or cerebroside or polypeptide expression level measured in the absence of said candidate compound or a sample comprising said plurality of compounds, whereby an increased level over the standard indicates that the compound or the sample comprising said plurality of compounds is an agonist and a decreased level over the standard indicates that the compound or the sample comprising said plurality of compounds is an antagonist.
18. A process for the identification of a compound conferring increased lipid, preferably a glycolipid, a glycolipide containing galactose, more preferably a galactolipide and/or cerebrosideproduction in a plant or microorganism, comprising the steps:
   a) culturing a plant cell or tissue or microorganism or maintaining a plant expressing the polypeptide encoded by the nucleic acid molecule of claim 6 conferring an increase in the amount of lipids, preferably of glycolipids, of glycolipides containing galactose, more preferably of galactolipides and/or cerebrosides in an organism or a part thereof and a readout system capable of interacting with the polypeptide under suitable conditions which permit the interaction of the polypeptide with dais readout system in the presence of a compound or a sample comprising a plurality of compounds and capable of providing a detectable signal in response to the binding of a compound to said polypeptide under conditions which permit the expression of said readout system and of the polypeptide encoded by the nucleic acid molecule of claim 6 conferring an increase in the amount of lipids, preferably of glycolipids, of glycolipides containing galactose, more preferably of galactolipides and/or cerebrosides in an organism or a part thereof;
   b) identifying if the compound is an effective agonist by detecting the presence or absence or increase of a signal produced by said readout system.
19. A method for the identification of a gene product conferring an increase in lipids, preferably in glycolipid, in glycolipide containing galactose, more preferably in galactolipide and/or cerebroside production in a cell, comprising the following steps:
   (a) contacting the nucleic acid molecules of a sample, which can contain a candidate gene encoding a gene product conferring an increase in lipids, preferably in glycolipid, in glycolipide containing galactose, more preferably in galactolipide and/or cerebroside after expression with the nucleic acid molecule of claim 6;
   (b) identifying the nucleic acid molecules, which hybridise under relaxed stringent conditions with the nucleic acid molecule of claim 6;
   (c) introducing the candidate nucleic acid molecules in host cells appropriate for producing lipid, preferably a glycolipid, a glycolipide containing galactose, more preferably a galactolipide and/or cerebroside;
   (d) expressing the identified nucleic acid molecules in the host cells;
   (e) assaying the lipid, preferably a glycolipid, a glycolipide containing galactose, more preferably a galactolipide and/or cerebrosidelevel in the host cells; and
   (f) identifying nucleic acid molecule and its gene product which expression confers an increase in the lipid, preferably in glycolipid, in glycolipide containing galactose, more preferably in galactolipide and/or cerebroside level in the host cell in the host cell after expression compared to the wild type.
20. A method for the identification of a gene product conferring an increase in lipid, preferably in glycolipid, in glycolipide containing galactose, more preferably in galactolipide and/or cerebroside production in a cell, comprising the following steps:
   (a) identifiying in a data bank nucleic acid molecules of an organism; which can contain a candidate gene encoding a gene product conferring an increase in the lipid, preferably a glycolipid, a glycolipide containing galactose, more preferably a galactolipide and/or cerebroside amount or level in an organism or a part thereof after expression, and which are at least 20% homolog to the nucleic acid molecule of claim 6;
   (b) introducing the candidate nucleic acid molecules in host cells appropriate for producing lipid, preferably a glycolipid, a glycolipide containing galactose, more preferably a galactolipide and/or cerebroside;
   (c) expressing the identified nucleic acid molecules in the host cells;
   (d) assaying the lipid, preferably a glycolipid, a glycolipide containing galactose, more preferably a galactolipide and/or cerebroside level in the host cells; and
   (e) identifying nucleic acid molecule and its gene product which expression confers an increase in the lipid, preferably in the glycolipid, in the glycolipide containing galactose, more preferably in the galactolipide and/or cerebroside level in the host cell after expression compared to the wild type.
21. A method for the production of an agricultural composition comprising the steps of the method of any one of claims 17 to 20 and formulating the compound identified in any one of claims 17 to 20 in a form acceptable for an application in agriculture.
22. A composition comprising the nucleic acid molecule of claim 6, the polypeptide of claim 14, the nucleic acid construct of claim 7, the vector of any one of claims 8 or 9, an antagonist or agonist identified according to claim 17, the compound of claim 18, the gene product of claim 19 or 20, the antibody of claim 15, and optionally an agricultural acceptable carrier.
23. Use of the nucleic acid molecule as claimed in claim 6 for the identification of a nucleic acid molecule conferring an increase of lipid, preferably of glycolipid, of glycolipide containing galactose, more preferably of galactolipide and/or cerebroside after expression.
24. Use of the polypeptide of claim 14 or the nucleic acid construct claim 7 or the gene product identified according to the method of claim 19 or 20 for identifying compounds capable of conferring a modulation of lipid, preferably of glycolipid, of glycolipide containing galactose, more preferably of galactolipide and/or cerebroside levels in an organism.
25. Food or feed composition comprising the nucleic acid molecule of claim 6, the polypeptide of claim 14, the nucleic acid construct of claim 7, the vector of claim 8 or 9, the antagonist or agonist identified according to claim 17, the antibody of claim 15, the plant or plant tissue of claim 16, the harvested material of claim 16, the host cell of claim 10 to 12 or the gene product identified according to the method of claim 19 or 20.
26. Use of the nucleic acid molecule of claim 6, the polypeptide of claim 14, the nucleic acid construct of claim 7, the vector of claim 8 or 9, the antagonist or agonist identified according to claim 17, the antibody of claim 15, the plant or plant tissue of claim 16, the host cell of claim 10 to 12 or the gene product identified according to the method of claim 19 or 20 for the protection of a plant against a glycolipid, of glycolipide containing galactose, more preferably of galactolipide and/or cerebroside synthesis inhibiting herbicide.

**[0554.0.0.22] Abstract: see [0554.0.0.0]**

### Process for the production of fine chemicals

**[0000.0.0.23]** In a further embodiment, the present invention relates to a further process for the production of fine chemicals as defined below and the corresponding embodiments as described herein as follows.

**[0001.0.0.23]** for the disclosure of this paragraph see [0001.0.0.0] above.

**[0002.0.23.23]** Salicylic acid is common throughout the plant kingdom and is also found in bacteria. It is an important regulator of induced plant resistance to pathogens.
Small amounts of salicylic acid are known to be present in plants. Originally salicylic acid was extracted from the willow bark to make the well-known pain relief medication Aspirin. Salicylic acid is thought to promote disease resistance, increase flower life, inhibit seed germination, and promote ethylene synthesis.
Salicylic acid can be synthesized from cinnamate. Previous isotope feeding experiments in tobacco and other higher plants, including rice, demonstrated that the direct precursor of salicylic acid is free benzoic acid. Benzoic acid is synthesized by cinnamate chain shortening reactions via the so-called beta-oxidation, analogous to fatty acid beta-oxidation. Benzoic acid is then converted to salicylic acid by benzoic acid 2-hydroxylase. Recent studies in tobacco indicated that conjugated benzoic acid, CoA thioesters or glucose esters, are more likely to be the precursors of salicylic acid. More recent genetic studies in Arabidopsis have shown that salicylic acid can also be synthesized from chorismate and that the bulk of salicylic acid is produced from chorismate.
Plants react to pathogen attack by activating elaborate defense mechanisms. The defense response is activated not only at the sites of infection, but also in neighboring and even distal uninfected parts of the plant, leading to systemic acquired resistance. Plant resistance is associated with activated expression of a large number of defense-related genes, whose products may play important roles in the restriction of pathogen growth and spread. During the past several years, evidence has accumulated which indicates that salicylic acid (SA) acts as an endogenous signal for plant defense responses.
In most plants, exposure to powdery mildew and other pathogens triggers the plant defense pathway, a series of biochemical events that occur in succession and help the plant resist infection. Salicylic acid governs this pathway.
Where resistance to a pathogen is associated with a localised necrotic lesion, the plant will subsequently be systemically "immunized" so that further infection will either exhibit increased resistance or reduced disease symptoms (reviewed by Ryals et al., 1996). This "systemic acquired resistance" (SAR) is associated with the systemic expression of a subset of defence genes, e.g. the acidic forms of pathogenesis-related PR1-5 proteins (Ward *et al*., 1992). Search for a signal that may be mobilised from the lesion to elicit systemic resistance has led to the identification of salicylic acid (SA) as the most likely candidate. SA is synthesised to high levels around the necrotic lesion, before being (possibly) mobilised through the phloem to accumulate, at much lower levels, systemically.

**[0003.0.23.23]** When faced with a fungus or bacteria, most plants turn up their production of salicylic acid, which then interacts with other molecules in the plant, eventually turning on the genes that produce the proteins involved in fighting infection. These infection-fighting proteins also turn off salicylic acid production, a phenomenon known as negative feedback. In this way, plants can turn the pathogen defense pathway on and off as needed.
The basic idea to enhance plant disease resistance by overproduction of salicylic acid has already been published years ago for example by Verberne et al., Pharm.World-Sci.; (1995) 17, 6. Later on in 2000 is was published that the expression of the Escherichia coli isochorismate-synthase and Pseudomonas fluorescence isochorismate-pyruvate-lyase in transgenic tobacco can lead to improved disease-resistance (Verberne, M et al., Nat.Biotechnol.; (2000) 18, 7, 779-83. The two enzymes converted chorismate into SA by a 2-step process. When the enzymes were targeted to the chloroplasts, the transgenic plants showed a 500- to 1,000-fold increased accumulation of SA and SA glucoside compared to control plants. These plants showed a resistance to viral (tobacco-mosaic virus) and fungal (Oidium lycopersicon) infection resembling SAR in nontransgenic plants. As the effect was the result of the plastidal expression of two heterologous genes, there is the obvious need for alternative and more simple methods for enhanced salicylic acid production in plants by the cytosolic expression of individual genes. For individual cases or specific plant species a more moderate salicylic acid increase may also be useful and desired.
Additionally salicylic acid binding proteins have been described as useful for the production of transgenic plants with increased resistance to disease (WO2003016551).
Most plants maintain very low levels of salicylic acid in their tissues unless they are fighting an infection. Metal hyperaccumulators, however, have significantly elevated salicylic acid in their tissues all the time - see:
www.newswise.com/articles/view/510423/
Recent results also suggest that in some plant species high level of endogenous salicylic acid protects the plants from oxidative stress caused for example by aging or biotic or abiotic stress (Yang et al., Plant J. 2004 Dec; 40 (6): 909-19).

**[0004.0.23.23]** Aspirin was introduced into clinical practice more than 100 years ago. This unique drug belongs to a family of compounds called the salicylates, the simplest of which is salicylic acid, the principal metabolite of aspirin. Salicylic acid is responsible for the anti-inflammatory action of aspirin, and may cause the reduced risk of colorectal cancer observed in those who take aspirin. Yet salicylic acid and other salicylates occur naturally in fruits and plants, while diets rich in these are believed to reduce the risk of colorectal cancer. Serum salicylic acid concentrations are greater in vegetarians than non-vegetarians, and there is overlap between concentrations in vegetarians and those taking low-dose aspirin. It is proposed that the cancer-preventive action of aspirin is due to its principal metabolite, salicylic acid, and that dietary salicylates can have the same effect. It is also possible that natural salicylates contribute to the other recognized benefits of a healthy diet.

**[0005.0.23.23]** The hydroxyl group of salicylic acid reacts with acetic acid to form the acetate ester, acetylsalicylic acid (see aspirin). Several useful esters are formed by reaction of the carboxyl group with alcohols. The methyl ester, methyl salicylate (also called oil of wintergreen since it produces the fragrance of wintergreen), is formed with methanol; it is used in food flavorings and in liniments. The phenyl ester, phenyl salicylate, or salol, is formed with phenol; it is used in medicine as an antiseptic and antipyretic. This ester hydrolyzes, not in the acidic stomach, but in the alkaline intestines, releasing free salicylic acid. The menthyl ester, menthyl salicylate, which is used in suntan lotions, is formed with menthol.
Salicylic acid possesses bacteriostatic, fungicidal, and keratolytic actions.

**[0006.0.23.23]** Salicylic acid is used as a food preservative and as an antiseptic in toothpaste. It is a peeling agent in ointments, creams, gels, and shampoos applied to reduce the scaling of the skin or scalp in psoriasis. It is the active ingredient in many skin products for the treatment of acne since it causes skin cells to slough off more readily, preventing them from clogging up the pores.

**[0007.0.23.23]** Salicylic acid belongs to the group of medicines known as keratolytics. Salicylic acid works by breaking down keratin, a protein, which forms part of the skin structure. This results in the shedding of skin cells from the affected area. In the treatment of warts, calluses and verrucae the effect of salicylic acid is to remove the affected skin over a period of time. If successful, the new skin, which grows underneath will be healthy.

**[0008.0.23.23]** One way to increase the productive capacity of biosynthesis is to apply recombinant DNA technology. Thus, it would be desirable to produce salicylic acid and/or salicylic acid esters in plants. That type of production permits control over quality, quantity and selection of the most suitable and efficient producer organisms. The latter is especially important for commercial production economics and therefore availability to consumers. In addition it is desirable to produce salicylic acidin plants in order to increase plant productivity and resistance against biotic and abiotic stress as discussed before.
Methods of recombinant DNA technology have been used for some years to improve the production of fine chemicals in microorganisms and plants by amplifying individual biosynthesis genes and investigating the effect on production of fine chemicals. It is for example reported, that the xanthophyll astaxanthin could be produced in the nectaries of transgenic tobacco plants. Those transgenic plants were prepared by *Argobacterium tumifaciens*-mediated transformation of tobacco plants using a vector that contained a ketolase-encoding gene from *H. pluvialis* denominated *crtO* along with the Pds gene from tomato as the promoter and to encode a leader sequence. Those results indicated that about 75 percent of the carotenoids found in the flower of the transformed plant contained a keto group.

**[0009.0.23.23]** Thus, it would be advantageous if algae, plant or other microorganism were available which produce large amounts of salicylic acid. The invention discussed hereinafter relates in some embodiments to such transformed prokaryotic or eukaryotic microorganisms.
It would also be advantageous if plants were available whose roots, leaves, stems, fruits or flowers produced large amounts of salicylic acid. The invention discussed hereinafter relates in some embodiments to such transformed plants.
Furthermore it would be advantageous if plants were available whose seed produced larger amounts of total lipids. The invention discussed hereinafter relates in some embodiments to such transformed plants.

**[0010.0.23.23]** Therefore improving the quality of foodstuffs and animal feeds is an important task of the food-and-feed industry. This is necessary since, for example salicylic acid, as mentioned above, which occur in plants and some microorganisms are limited with regard to the supply of mammals. Especially advantageous for the quality of foodstuffs and animal feeds is as balanced as possible a specific salicylic acid profile in the diet since an excess of salicylic acid above a specific concentration in the food has a positive effect. A further increase in quality is only possible via addition of further salicylic acid, which are limiting.

**[0011.0.23.23]** To ensure a high quality of foods and animal feeds, it is therefore necessary to add salicylic acidand/or salicylic acid esters in a balanced manner to suit the organism.

**[0012.0.23.23]** Accordingly, there is still a great demand for new and more suitable genes which encode enzymes or other proteins which participate in the biosynthesis of salicylic acidand make it possible to produce them specifically on an industrial scale without unwanted byproducts forming. In the selection of genes for biosynthesis two characteristics above all are particularly important. On the one hand, there is as ever a need for improved processes for obtaining the highest possible contents of salicylic acid and/or salicylic acid esters; on the other hand as less as possible byproducts should be produced in the production process.
Furthermore there is still a great demand for new and more suitable genes which encode enzymes or other proteins which participate in the biosynthesis of total lipids and make it possible to produce them specifically on an industrial scale without unwanted byproducts forming. In the selection of genes for biosynthesis two characteristics above all are particularly important. On the one hand, there is as ever a need for improved processes for obtaining the highest possible contents of total lipids; on the other hand as less as possible byproducts should be produced in the production process.

**[0013.0.0.23]** for the disclosure of this paragraph see [0013.0.0.0] above.

**[0014.0.23.23]** Accordingly, in a first embodiment, the invention relates to a process for the production of a fine chemical, whereby the fine chemical is salicylic acid or salicylic acid esters. Further, the term "the fine chemicals" as used herein also relates to fine chemicals comprising salicylic acid and/or salicylic acid esters.
In one embodiment, the term "the fine chemical" means salicylic acid. Throughout the specification the term "the fine chemical" means salicylic acid, its salts, ester, thioester or in free form or bound to other compounds such as sugars.

**[0015.0.23.23]** In particular it is known to the skilled that anionic compounds such as acids are present in the aqueous solution in an equilibrium between the acid and its salts according to the pH present in the respective compartment of the cell or organism and the pK of the acid. Depending on the strength of the acid (pK) and the pH the salt or the free acid are predominant. Thus, the term "the fine chemical", the term "the respective fine chemical", the term "acid" or the use of a denomination referring to a neutralized anionic compound respectively, relates to the anionic form as well as the neutralised status of that compound in relation to the conditions of the aqueous solution.

**[0016.0.23.23]** Accordingly, the present invention relates to a process comprising
(a) increasing or generating the activity of one or more b0161, b2664, b2796, b3116 or YLR089C protein(s) in a non-human organism in one or more parts thereof; and
(b) growing the organism under conditions which permit the production of the fine chemical, thus salicylic acid in said organism.
Accordingly, the present invention relates to a process comprising
(a) increasing or generating the activity of one or more proteins having the activity of a protein indicated in Table II, column 3, lines 275 to 277 and/or 635 or 636, resp. or having the sequence of a polypeptide encoded by a nucleic acid molecule indicated in Table I, columns 5 or 7, lines 275 to 277 and/or 635 or 636, resp. in a non-human organism in one or more parts thereof; and
growing the organism under conditions which permit the production of the fine chemical, thus, salicylic acid and/or salicylic acid esters, in said organism.

**[0016.1.23.23]** Accordingly, the term "the fine chemical" means " salicylic acid" in relation to all sequences listed in Table I, columns 5 or 7, lines 275 to 277 and/or 635 or 636 or homologs thereof.

**[0017.0.0.23] and [0018.0.0.23]** for the disclosure of the paragraphs [0017.0.0.23] and [0018.0.0.23] see paragraphs [0017.0.0.0] and [0018.0.0.0] above.

**[0019.0.23.23]** Advantageously the process for the production of the respective fine chemical leads to an enhanced production of the respective fine chemical. The terms "enhanced" or "increase" mean at least a 10%, 20%, 30%, 40% or 50%, preferably at least 60%, 70%, 80%, 90% or 100%, more preferably 150%, 200%, 300%, 400% or 500% higher production of the respective fine chemical in comparison to the reference as defined below, e.g. that means in comparison to an organism without the aforementioned modification of the activity of a protein having the activity of a protein indicated in Table II, column 3, lines 275 to 277 and/or 635 or 636 or encoded by nucleic acid molecule indicated in Table I, columns 5 or 7, lines 275 to 277 and/or 635 or 636.

**[0020.0.23.23]** Surprisingly it was found, that the transgenic expression of the Escherichia coli K12 protein b0161, b2796, b3116 or b2664 or Saccharomyces cerevisiae protein YLR089C in Arabidopsis thaliana conferred an increase in salicylic acid ("the fine chemical" or "the fine respective chemical") in respect to said proteins and their homologs as wells as the encoding nucleic acid molecules, in particular as indicated in Table II, column 3, lines 275 to 277 and/or 635 or 636 content of the transformed plants.
Surprisingly it was found, that the transgenic expression of the Saccaromyces cerevisiae protein YLR089C as indicated in Table II, columns 3 or 5, line 277 and/or the Escherichia coli K12 protein(s) b0161, b2796, b3116 and/or b2664 as indicated in Table II, columns 3 or 5, lines 275, 276, 635 and/or 636 in Arabidopsis thaliana conferred an increase in the content of salicylic acid and/or salicylic acid esters in the transformed plants. Thus, in one embodiment, said protein(s) or its homologs as indicated in Table II, column 7, lines 275 to 277 and/or 635 or 636 are used for the production of salicylic acid and/or salicylic acid esters.

**[0021.0.0.23]** for the disclosure of this paragraph see [0021.0.0.0] above.

**[0022.0.23.23]** The sequence of b0161 from Escherichia coli K12 has been published in Blattner, F.R. et al., Science 277 (5331), 1453-1474 (1997) and its activity is being defined as a protein having serine protease activity. Accordingly, in one embodiment, the process of the present invention comprises the use of a protein b0161 from Escherichia coli K12 or its homolog, e.g. as shown herein, for the production of the respective fine chemical, in particular for increasing the amount of salicylic acid, preferably in free or bound form in an organism or a part thereof, as mentioned. In one embodiment, in the process of the present invention the activity of the protein b0161 is increased.
The sequence of b2664 from Escherichia coli K12 has been published in Blattner, F.R. et al, Science 277 (5331), 1453-1474 (1997) and its activity is being defined as a protein having transcriptional repressor (GntR familiy) activity. Accordingly, in one embodiment, the process of the present invention comprises the use of a protein b2664 from Escherichia coli K12 or its homolog, e.g. as shown herein, for the production of the respective fine chemical, in particular for increasing the amount of salicylic acid, preferably in free or bound form in an organism or a part thereof, as mentioned. In one embodiment, in the process of the present invention the activity of the protein b2664 is increased.
The sequence of b2796 (Accession number NP_417276) from Escherichia coli K12 has been published in Blattner et al., Science 277 (5331), 1453-1474, 1997, and its activity is being defined as a putative serine transport protein (HAAAP family). Accordingly, in one embodiment, the process of the present invention comprises the use of a gene product with an activity of threonine-serine permease superfamily, preferably a protein with the activity of a putative serine transport protein (HAAAP family) from E. coli or its homolog, e.g. as shown herein, for the production of the fine chemical, meaning of salicylic acid, preferably in free or bound form in an organism or a part thereof, as mentioned. In one embodiment, in the process of the present invention the activity of the protein b2796 is increased.
The sequence of b3116 (Accession number NP_417586) from Escherichia coli K12 has been published in Blattner et al., Science 277 (5331), 1453-1474, 1997, and its activity is being defined as a L-threonine/L-serine permease, anaerobically inducible (HAAAP family). Accordingly, in one embodiment, the process of the present invention comprises the use of a gene product with an activity of threonine-serine permease superfamily, preferably a protein with the activity of a L-threonine/L-serine permease, anaerobically inducible (HAAAP family) from E. coli or its homolog, e.g. as shown herein, for the production of the fine chemical, meaning of salicylic acid, preferably in free or bound form in an organism or a part thereof, as mentioned. In one embodiment, in the process of the present invention the activity of the protein b3116 is increased.
The sequence of YLR089C from Saccharomyces cerevisiae has been published in Johnston, M. et al., Nature 387 (6632 Suppl), 87- 90 (1997) and its activity is being defined as a protein having alanine transaminase (glutamic pyruvic transaminase) activity. Accordingly, in one embodiment, the process of the present invention comprises the use of a protein YLR089C from Saccharomyces cerevisiae or its homolog, e.g. as shown herein, for the production of the respective fine chemical, in particular for increasing the amount of salicylic acid preferably in free or bound form in an organism or a part thereof, as mentioned. In one embodiment, in the process of the present invention the activity of the protein YLR089C is increased.

**[0023.0.23.23]** Homologues (= homologs) of the present gene products can be derived from any organisms as long as the homologue confers the herein mentioned activity, in particular, confers an increase in the respective fine chemical amount or content.
In one embodiment, the homolog of the polypeptides indicated in Table II, column 3, lines 275 to 277 and/or 635 or 636 is a homolog having the same or a similar activity.
In particular an increase of activity confers an increase in the content of the respective fine chemical in the organisms preferably salicylic acid.
Homologs of the polypeptides indicated in Table II, column 3, lines 275 to 277 and/or 635 or 636 may be the polypeptides encoded by the nucleic acid molecules indicated in Table I, column 7, lines 275 to 277 and/or 635 or 636 having a salicylic acid content and/or amount increasing activity.

**[0023.1.23.23]** Homologs of the polypeptides polypeptide indicated in Table II, column 3, lines 275 to 277 and/or 635 or 636 may be the polypetides encoded by the nucleic acid molecules polypeptide indicated in Table I, column 7, lines 275 to 277 and/or 635 or 636 or may be the polypeptides indicated in Table II, column 7, lines 275 to 277 and/or 635 or 636.

**[0024.0.0.23]** for the disclosure of this paragraph see [0024.0.0.0] above.

**[0025.0.23.23]** In accordance with the invention, a protein or polypeptide has the "activity of a protein of the invention", e.g. the activity of a protein indicated in Table II, column 3, lines 275 to 277 and/or 635 or 636 if its *de novo* activity, or its increased expression directly or indirectly leads to an increased salicylic acid level, resp., in the organism or a part thereof, preferably in a cell of said organism. In a preferred embodiment, the protein or polypeptide has the above-mentioned additional activities of a protein indicated in Table II, column 3, lines 275 to 277 and/or 635 or 636. Throughout the specification the activity or preferably the biological activity of such a protein or polypeptide or an nucleic acid molecule or sequence encoding such protein or polypeptide is identical or similar if it still has the biological or enzymatic activity of any one of the proteins indicated in Table II, column 3, lines 275 to 277 and/or 635 or 636 or which has at least 10% of the original enzymatic activity, preferably 20%, particularly preferably 30%, most particularly preferably 40% in comparison to any one of the proteins indicated in Table II, column 3, lines 275, 276, 635 and/or 636 of Escherichia coli K12 or line 277 of Saccharomyces cerevisiae respectively.
In one embodiment, the polypeptide of the invention confers said activity, e.g. the increase of the respective fine chemical in an organism or a part thereof, if it is derived from an organism, which is evolutionary close to the organism indicated in Table I, column 4 and is expressed in an organism, which is evolutionary distant to the origin organism. For example origin and expressing organism are derived from different families, orders, classes or phylums whereas origin and the organism indicated in Table I, column 4 are derived from the same families, orders, classes or phylums.

**[0025.1.0.23] and [0025.2.0.23]** for the disclosure of the paragraphs [0025.1.0.23] and [0025.2.0.23] see [0025.1.0.0] and [0025.2.0.0] above.

**[0026.0.0.23] to [0033.0.0.23]** for the disclosure of the paragraphs [0026.0.0.23] to [0033.0.0.23] see [0026.0.0.0] to [0033.0.0.0] above.

**[0034.0.23.23]** Preferably, the reference, control or wild type differs from the subject of the present invention only in the cellular activity of the polypeptide of the invention, e.g. as result of an increase in the level of the nucleic acid molecule of the present invention or an increase of the specific activity of the polypeptide of the invention. E.g., it differs by or in the expression level or activity of a protein having the activity of a protein as indicated in Table II, column 3, lines 275 to 277 and/or 635 or 636 or being encoded by a nucleic acid molecule indicated in Table I, column 5, lines 275 to 277 and/or 635 or 636 or its homologs, e.g. as indicated in Table I, column 7, lines 275 to 277 and/or 635 or 636, its biochemical or genetic causes. It therefore shows the increased amount of the respective fine chemical.

**[0035.0.0.23] to [0044.0.0.23]** for the disclosure of the paragraphs [0035.0.0.23] to [0044.0.0.23] see paragraphs [0035.0.0.0] to [0044.0.0.0] above.

**[0045.0.23.23]** In one embodiment, in case the activity of the Escherichia coli K12 protein b0161 or its homologs, e.g. as indicated in Table II, columns 5 or 7, line 275 is increased, preferably, in one embodiment an increase of the respective fine chemical, preferably of salicylic acid between 67% and 475% or more is conferred.
In one embodiment, in case the activity of the Escherichia coli K12 protein b2664 or its homologs, e.g. as indicated in Table II, columns 5 or 7, line 276 is increased, preferably, in one embodiment an increase of the respective fine chemical, preferably of salicylic acid between 56% and 425% or more is conferred.
In one embodiment, in case the activity of the Saccharomyces cerevisae protein YLR089C or its homologs, e.g. as indicated in Table II, columns 5 or 7, line 277 is increased, preferably, in one embodiment the increase of the respective fine chemical, preferably of salicylic acid between 45% and 153% or more is conferred.
In one embodiment, in case the activity of the Escherichia coli K12 protein b2796 or its homologs, e.g. as indicated in Table II, columns 5 or 7, line 635 is increased, preferably, in one embodiment an increase of the respective fine chemical, preferably of salicylic acid between 42% and 248% or more is conferred.
In one embodiment, in case the activity of the Escherichia coli K12 protein b3116 or its homologs, e.g. as indicated in Table II, columns 5 or 7, line 636 is increased, preferably, in one embodiment an increase of the respective fine chemical, preferably of salicylic acid between 41 % and 130% or more is conferred.

**[0046.0.0.23]** %

**[0047.0.0.23] and [0048.0.0.23]** for the disclosure of the paragraphs [0047.0.0.23] and [0048.0.0.23] see paragraphs [0047.0.0.0] and [0048.0.0.0] above.

**[0049.0.23.23]** A protein having an activity conferring an increase in the amount or level of the respective fine chemical salicylic acid preferably has the structure of the polypeptide described herein. In a particular embodiment, the polypeptides used in the process of the present invention or the polypeptide of the present invention comprises the sequence of a consensus sequence as shown in SEQ ID NO: 33804, 33805, 33806, 33807, 33808, 33809 or 33896, 33897, 33898 or 34219, 34220, 34221, 34222, 34223, 34224, 34225, 34226, 34227 or 99065, 99066, 99067, 99170, 99171, 99172, 99173, 99174, 99175, 99176, 99177 or as indicated in Table IV, column 7, lines 275 to 277 and/or 635 or 636 or of a polypeptide as indicated in Table II, columns 5 or 7, lines 275 to 277 and/or 635 or 636 or of a functional homologue thereof as described herein, or of a polypeptide encoded by the nucleic acid molecule characterized herein or the nucleic acid molecule according to the invention, for example by a nucleic acid molecule as indicated in Table I, columns 5 or 7, lines 275 to 277 and/or 635 or 636 or its herein described functional homologues and has the herein mentioned activity conferring an increase in the salicylic acid level.

**[0050.0.23.23]** For the purposes of the present invention, the term "the respective fine chemical" also encompass the corresponding salts, such as, for example, the potassium or sodium salts of salicylic acid, resp., or their esters, e.g. but not limited to the methyl ester, the phenyl ester or the menthol ester.

**[0051.0.23.23]** Owing to the biological activity of the proteins which are used in the process according to the invention and which are encoded by nucleic acid molecules according to the invention, it is possible to produce compositions comprising the respective fine chemical, i.e. an increased amount of the free or bound salicylic acid, e.g compositions comprising salicylic acid. Depending on the choice of the organism used for the process according to the present invention, for example a microorganism or a plant, compositions or mixtures of salicylic acidand salicylic acid esters can be produced.

**[0052.0.0.23]** for the disclosure of this paragraph see paragraph [0052.0.0.0] above.

**[0053.0.23.23]** In one embodiment, the process of the present invention comprises one or more of the following steps
a) stabilizing a protein conferring the increased expression of a protein encoded by the nucleic acid molecule of the invention or of the polypeptid of the invention, e.g. of a polypeptide having an activity of a protein as indicated in Table II, column 3, lines 275 to 277 and/or 635 or 636 or its homologs, e.g. as indicated in Table II, columns 5 or 7, lines 275 to 277 and/or 635 or 636 , activity having herein-mentioned the respective fine chemical increasing activity;
b) stabilizing a mRNA conferring the increased expression of a protein encoded by the nucleic acid molecule of the invention, e.g. of a polypeptide having an activity of a protein as indicated in Table II, column 3, lines 275 to 277 and/or 635 or 636 or its homologs activity, e.g. as indicated in Table II, columns 5 or 7, lines 275 to 277 and/or 635 or 636, or of a mRNA encoding the polypeptide of the present invention having herein-mentioned the respective fine chemical increasing activity;
c) increasing the specific activity of a protein conferring the increased expression of a protein encoded by the nucleic acid molecule of the invention or of the polypeptide of the present invention having herein-mentioned the respective fine chemical increasing activity, e.g. of a polypeptide having an activity of a protein as indicated in Table II, column 3, lines 275 to 277 and/or 635 or 636 or its homologs activity, e.g. as indicated in Table II, columns 5 or 7, lines 275 to 277 and/or 635 or 636, or decreasing the inhibitory regulation of the polypeptide of the invention;
d) generating or increasing the expression of an endogenous or artificial transcription factor mediating the expression of a protein conferring the increased expression of a protein encoded by the nucleic acid molecule of the invention or of the polypeptide of the invention having herein-mentioned the respective fine chemical increasing activity, e.g. of a polypeptide having an activity of a protein as indicated in Table II, column 3, lines 275 to 277 and/or 635 or 636 or its homologs activity, e.g. as indicated in Table II, columns 5 or 7, lines 275 to 277 and/or 635 or 636;
e) stimulating activity of a protein conferring the increased expression of a protein encoded by the nucleic acid molecule of the present invention or a polypeptide of the present invention having herein-mentioned the respective fine chemical increasing activity, e.g. of a polypeptide having an activity of a protein as indicated in Table II, column 3, lines 275 to 277 and/or 635 or 636 or its homologs activity, e.g. as indicated in Table II, columns 5 or 7, lines 275 to 277 and/or 635 or 636, by adding one or more exogenous inducing factors to the organism or parts thereof;
f) expressing a transgenic gene encoding a protein conferring the increased expression of a polypeptide encoded by the nucleic acid molecule of the present invention or a polypeptide of the present invention, having herein-mentioned the respective fine chemical increasing activity, e.g. of a polypeptide having an activity of a protein as indicated in Table II, column 3, lines 275 to 277 and/or 635 or 636 or its homologs activity, e.g. as indicated in Table II, columns 5 or 7, lines 275 to 277 and/or 635 or 636, and/or
g) increasing the copy number of a gene conferring the increased expression of a nucleic acid molecule encoding a polypeptide encoded by the nucleic acid molecule of the invention or the polypeptide of the invention having herein-mentioned the respective fine chemical increasing activity, e.g. of a polypeptide having an activity of a protein as indicated in Table II, column 3, lines 275 to 277 and/or 635 or 636 or its homologs, e.g. as indicated in Table II, columns 5 or 7, lines 275 to 277 and/or 635 or 636.
h) Increasing the expression of the endogenous gene encoding the polypeptide of the invention, e.g. a polypeptide having an activity of a protein as indicated in Table II, column 3, lines 275 to 277 and/or 635 or 636 or its homologs activity, e.g. as indicated in Table II, columns 5 or 7, lines 275 to 277 and/or 635 or 636, by adding positive expression or removing negative expression elements, e.g. homologous recombination can be used to either introduce positive regulatory elements like for plants the 35S enhancer into the promoter or to remove repressor elements form regulatory regions. Further gene conversion methods can be used to disrupt repressor elements or to enhance to activity of positive elements. Positive elements can be randomly introduced in plants by T-DNA or transposon mutagenesis and lines can be identified in which the positive elements have be integrated near to a gene of the invention, the expression of which is thereby enhanced;and/or
i) Modulating growth conditions of an organism in such a manner, that the expression or activity of the gene encoding the protein of the invention or the protein itself is enhanced for example microorganisms or plants can be grown for example under a higher temperature regime leading to an enhanced expression of heat shock proteins, which can lead to an enhanced respective fine chemical production.
j) selecting of organisms with especially high activity of the proteins of the invention from natural or from mutagenized resources and breeding them into the target organisms, e.g. the elite crops.

**[0054.0.23.23]** Preferably, said mRNA is the nucleic acid molecule of the present invention and/or the protein conferring the increased expression of a protein encoded by the nucleic acid molecule of the present invention or the polypeptide having the herein mentioned activity is the polypeptide of the present invention, e.g. conferring the increase of the respective fine chemical after increasing the expression or activity of the encoded polypeptide or having the activity of a polypeptide having an activity of a protein as indicated in Table II, columns 3 or 5, lines 275 to 277 and/or 635 or 636, resp., or its homologs activity, e.g. as indicated in Table II, columns 5 or 7, lines 275 to 277 and/or 635 or 636, resp.

**[0055.0.0.23] to [0067.0.0.23]** for the disclosure of the paragraphs [0055.0.0.23] to [0067.0.0.23] see paragraphs [0055.0.0.0] to [0067.0.0.0] above.

**[0068.0.23.23]** The mutation is introduced in such a way that the production of salicylic acidis not adversely affected.

**[0069.0.0.23]** for the disclosure of this paragraph see paragraph [0069.0.0.0] above.

[0070.0.23.23] Owing to the introduction of a gene or a plurality of genes conferring the expression of the nucleic acid molecule of the invention or the polypeptide of the invention, for example the nucleic acid construct mentioned below, or encoding a protein of the invention into an organism alone or in combination with other genes, it is possible not only to increase the biosynthetic flux towards the end product, but also to increase, modify or create de novo an advantageous, preferably novel metabolite composition in the organism, e.g. an advantageous composition of salicylic acid or their biochemical derivatives , e.g. comprising a higher content of (from a viewpoint of nutrional physiology limited) salicylic acid or their derivatives including but not limited to salicylic acid esters.

**[0071.0.0.23]** for the disclosure of this paragraph see paragraph [0071.0.0.0] above.

**[0072.0.0.23]** %

**[0073.0.23.23]** Accordingly, in one embodiment, the process according to the invention relates to a process, which comprises:
(n) providing a non-human organism, preferably a microorganism, a non-human animal, a plant or animal cell, a plant or animal tissue or a plant;
(o) increasing an activity of a polypeptide of the invention or a homolog thereof, e.g. as indicated in Table II, columns 5 or 7, lines 275 to 277 and/or 635 or 636, or of a polypeptide being encoded by the nucleic acid molecule of the present invention and described below, e.g. conferring an increase of the respective fine chemical in an organism, preferably in a microorganism, a non-human animal, a plant or animal cell, a plant or animal tissue or a plant,
(p) growing an organism, preferably a microorganism, a non-human animal, a plant or animal cell, a plant or animal tissue or a plant under conditions which permit the production of the respective fine chemical in the organism, preferably the microorganism, the plant cell, the plant tissue or the plant; and
(q) if desired, recovering, optionally isolating, the free and/or bound respective fine chemical synthesized by the organism, the microorganism, the non-human animal, the plant or animal cell, the plant or animal tissue or the plant. **[0074.0.23.23]** The organism, in particular the microorganism, non-human animal, the plant or animal cell, the plant or animal tissue or the plant is advantageously grown in such a way that it is not only possible to recover, if desired isolate the free or bound respective fine chemical.

**[0075.0.0.23] to [0077.0.0.23]** for the disclosure of the paragraphs [0075.0.0.23] to [0077.0.0.23] see paragraphs [0075.0.0.0] to [0077.0.0.0] above.

**[0078.0.23.23]** The organism such as microorganisms or plants or the recovered, and if desired isolated, respective fine chemical can then be processed further directly into foodstuffs or animal feeds or for other applications. The fermentation broth, fermentation products, plants or plant products can be purified with methods known to the person skilled in the art. Products of these different work-up procedures are salicylic acidor salicylic acid esters or comprising compositions of salicylic acid and salicylic acid esters still comprising fermentation broth, plant particles and cell components in different amounts, advantageously in the range of from 0 to 99% by weight, preferably below 80% by weight, especially preferably below 50% by weight.

**[0079.0.0.23] to [0084.0.0.23]** for the disclosure of the paragraphs [0079.0.0.23] to [0084.0.0.23] see paragraphs [0079.0.0.0] to [0084.0.0.0] above.

**[0085.0.23.23]** With regard to the nucleic acid sequence as depicted a nucleic acid construct which contains a nucleic acid sequence mentioned herein or an organism (= transgenic organism) which is transformed with said nucleic acid sequence or said nucleic acid construct, "transgene" means all those constructs which have been brought about by genetic manipulation methods, preferably in which either
a) a nucleic acid sequence as indicated in Table I, columns 5 or 7, lines 275 to 277 and/or 635 or 636, or a derivative thereof, or
b) a genetic regulatory element, for example a promoter, which is functionally linked to the nucleic acid sequence as indicated in Table I, columns 5 or 7, lines 275 to 277 and/or 635 or 636, or a derivative thereof, or
c) (a) and (b)
is/are not present in its/their natural genetic environment or has/have been modified by means of genetic manipulation methods, it being possible for the modification to be, by way of example, a substitution, addition, deletion, inversion or insertion of one or more nucleotide. "Natural genetic environment" means the natural chromosomal locus in the organism of origin or the presence in a genomic library. In the case of a genomic library, the natural, genetic environment of the nucleic acid sequence is preferably at least partially still preserved. The environment flanks the nucleic acid sequence at least on one side and has a sequence length of at least 50 bp, preferably at least 500 bp, particularly preferably at least 1000 bp, very particularly preferably at least 5000 bp.

**[0086.0.0.23] and [0087.0.0.23]** for the disclosure of the paragraphs [0086.0.0.23] and [0087.0.0.23] see paragraphs [0086.0.0.0] and [0087.0.0.0] above

**[0088.0.23.23]** In an advantageous embodiment of the invention, the organism takes the form of a plant whose content of the respective fine chemical is modified advantageously owing to the nucleic acid molecule of the present invention expressed. This is important for plant breeders since, for example, the nutritional value of plants for poultry is dependent on the abovementioned fine chemicals or the plants are more resistant to biotic and abiotic stress and the yield is increased.

**[0088.1.0.23]** for the disclosure of this paragraph see paragraph [0088.1.0.0] above.

**[0089.0.0.23] to [0094.0.0.23]** for the disclosure of the paragraphs [0089.0.0.23] to [0094.0.0.23] see paragraphs [0089.0.0.0] to [0094.0.0.0] above.

**[0095.0.23.23]** It may be advantageous to increase the pool of salicylic acid and/or salicylic acid esters in the transgenic organisms by the process according to the invention in order to isolate high amounts of the pure respective fine chemical and/or to obtain increased resistance against biotic and abiotic stresses and to obtain higher yield.

**[0096.0.23.23]** In another preferred embodiment of the invention a combination of the increased expression of the nucleic acid sequence or the protein of the invention together with the transformation of a protein or polypeptid or a compound, which functions as a sink for the desired fine chemical, for example in the organism, is useful to increase the production of the respective fine chemical.

**[0097.0.0.23]** for the disclosure of this paragraph see paragraph [0097.0.0.0] above.

**[0098.0.23.23]** In a preferred embodiment, the respective fine chemical is produced in accordance with the invention and, if desired, is isolated.

**[0099.0.23.23]** In the case of the fermentation of microorganisms, the abovementioned desired fine chemical might accumulate in the medium and/or the cells. If microorganisms are used in the process according to the invention, the fermentation broth can be processed after the cultivation. Depending on the requirement, all or some of the biomass can be removed from the fermentation broth by separation methods such as, for example, centrifugation, filtration, decanting or a combination of these methods, or else the biomass can be left in the fermentation broth. The fermentation broth can subsequently be reduced, or concentrated, with the aid of known methods such as, for example, rotary evaporator, thin-layer evaporator, falling film evaporator, by reverse osmosis or by nanofiltration. Afterwards advantageously further compounds for formulation can be added such as corn starch or silicates. This concentrated fermentation broth advantageously together with compounds for the formulation can subsequently be processed by lyophilization, spray drying, and spray granulation or by other methods. Preferably the respective fine chemical comprising compositions are isolated from the organisms, such as the microorganisms or plants or the culture medium in or on which the organisms have been grown, or from the organism and the culture medium, in the known manner, for example via extraction, distillation, crystallization, chromatography or a combination of these methods. These purification methods can be used alone or in combination with the aforementioned methods such as the separation and/or concentration methods.

**[0100.0.23.23]** Transgenic plants which comprise the fine chemicals such as salicylic acid and/or salicylic acid esters synthesized in the process according to the invention can advantageously be marketed directly without there being any need for the fine chemicals synthesized to be isolated. Plants for the process according to the invention are listed as meaning intact plants and all plant parts, plant organs or plant parts such as leaf, stem, seeds, root, tubers, anthers, fibers, root hairs, stalks, embryos, calli, cotelydons, petioles, harvested material, plant tissue, reproductive tissue and cell cultures which are derived from the actual transgenic plant and/or can be used for bringing about the transgenic plant. In this context, the seed comprises all parts of the seed such as the seed coats, epidermal cells, seed cells, endosperm or embryonic tissue.
However, the respective fine chemical produced in the process according to the invention can also be isolated from the organisms, advantageously plants, as extracts, e.g. ether, alcohol, or other organic solvents or water containing extract and/or free fine chemicals. The respective fine chemical produced by this process can be obtained by harvesting the organisms, either from the crop in which they grow, or from the field. This can be done via pressing or extraction of the plant parts. To increase the efficiency of extraction it is beneficial to clean, to temper and if necessary to hull and to flake the plant material. To allow for greater ease of disruption of the plant parts, specifically the seeds, they can previously be comminuted, steamed or roasted. Seeds, which have been pretreated in this manner can subsequently be pressed or extracted with solvents such as warm hexane. The solvent is subsequently removed. In the case of microorganisms, the latter are, after harvesting, for example extracted directly without further processing steps or else, after disruption, extracted via various methods with which the skilled worker is familiar. Thereafter, the resulting products can be processed further, i.e. degummed and/or refined. In this process, substances such as the plant mucilages and suspended matter can be first removed. What is known as desliming can be affected enzymatically or, for example, chemico-physically by addition of acid such as phosphoric acid.
Because salicylic acid and/or salicylic acid esters in microorganisms are localized intracellular or extracellular, their recovery essentially comes down to the isolation of the biomass or the supernatant. Well-established approaches for the harvesting of cells include filtration, centrifugation and coagulation/flocculation as described herein. Of the residual hydrocarbon, adsorbed on the cells, has to be removed. Solvent extraction or treatment with surfactants have been suggested for this purpose.

**[0101.0.23.23]** The identity and purity of the compound(s) isolated can be determined by prior-art techniques. They encompass high-performance liquid chromatography (HPLC), gas chromatography (GC), spectroscopic methods, mass spectrometry (MS), staining methods, thin-layer chromatography, NIRS, enzyme assays or microbiological assays. These analytical methods are compiled in: Patek et al. (1994) Appl. Environ. Microbiol. 60:133-140; Malakhova et al. (1996) Biotekhnologiya 11 27-32; and Schmidt et al. (1998) Bioprocess Engineer. 19:67-70. Ulmann's Encyclopedia of Industrial Chemistry (1996) Bd. A27, VCH Weinheim, pp. 89-90, pp. 521-540, pp. 540-547, pp. 559-566, 575-581 and pp. 581-587; Michal, G (1999) Biochemical Pathways: An Atlas of Biochemistry and Molecular Biology, John Wiley and Sons; Fallon, A. et al. (1987) Applications of HPLC in Biochemistry in: Laboratory Techniques in Biochemistry and Molecular Biology, vol. 17.

**[0102.0.23.23]** Salicylic acid and/or salicylic acid esters can for example be analyzed advantageously via HPLC, LC or GC separation and MS (masspectrometry) detection methods. The unambiguous detection for the presence of salicylic acid and/or salicylic acid containing products can be obtained by analyzing recombinant organisms using analytical standard methods: LC, LC-MS, MS or TLC). The material to be analyzed can be disrupted by sonication, grinding in a glass mill, liquid nitrogen and grinding, cooking, or via other applicable methods.

**[0103.0.23.23]** In a preferred embodiment, the present invention relates to a process for the production of the respective fine chemical comprising or generating in an organism or a part thereof the expression of at least one nucleic acid molecule comprising a nucleic acid molecule selected from the group consisting of:
a) nucleic acid molecule encoding, preferably at least the mature form, of the polypeptide having a sequence as indicated in Table II, columns 5 or 7, lines 275 to 277 and/or 635 or 636, or a fragment thereof, which confers an increase in the amount of the respective fine chemical in an organism or a part thereof;
b) nucleic acid molecule comprising, preferably at least the mature form, of a nucleic acid molecule having a sequence as indicated in Table I, columns 5 or 7, lines 275 to 277 and/or 635 or 636,
c) nucleic acid molecule whose sequence can be deduced from a polypeptide sequence encoded by a nucleic acid molecule of (a) or (b) as result of the degeneracy of the genetic code and conferring an increase in the amount of the respective fine chemical in an organism or a part thereof;
d) nucleic acid molecule encoding a polypeptide which has at least 50% identity with the amino acid sequence of the polypeptide encoded by the nucleic acid molecule of (a) to (c) and conferring an increase in the amount of the respective fine chemical in an organism or a part thereof;
e) nucleic acid molecule which hybridizes with a nucleic acid molecule of (a) to (c) under stringent hybridization conditions and conferring an increase in the amount of the respective fine chemical in an organism or a part thereof;
f) nucleic acid molecule encoding a polypeptide, the polypeptide being derived by substituting, deleting and/or adding one or more amino acids of the amino acid sequence of the polypeptide encoded by the nucleic acid molecules (a) to (d), preferably to (a) to (c) and conferring an increase in the amount of the respective fine chemical in an organism or a part thereof;
g) nucleic acid molecule encoding a fragment or an epitope of a polypeptide which is encoded by one of the nucleic acid molecules of (a) to (e), preferably to (a) to (c) and and conferring an increase in the amount of the respective fine chemical in an organism or a part thereof;
h) nucleic acid molecule comprising a nucleic acid molecule which is obtained by amplifying nucleic acid molecules from a cDNA library or a genomic library using the primer pairs having a sequence as indicated in Table III, column 7, lines 275 to 277 and/or 635 or 636, and conferring an increase in the amount of the respective fine chemical in an organism or a part thereof;
i) nucleic acid molecule encoding a polypeptide which is isolated, e.g. from an expression library, with the aid of monoclonal antibodies against a polypeptide encoded by one of the nucleic acid molecules of (a) to (h), preferably to (a) to (c), and conferring an increase in the amount of the respective fine chemical in an organism or a part thereof;
j) nucleic acid molecule which encodes a polypeptide comprising the consensus sequence having sequences as indicated in Table IV, column 7, lines 275 to 277 and/or 635 or 636 and conferring an increase in the amount of the respective fine chemical in an organism or a part thereof;
k) nucleic acid molecule comprising one or more of the nucleic acid molecule encoding the amino acid sequence of a polypeptide encoding a domain of the polypeptide indicated in Table II, columns 5 or 7, lines 275 to 277 and/or 635 or 636, and conferring an increase in the amount of the respective fine chemical in an organism or a part thereof; and
l) nucleic acid molecule which is obtainable by screening a suitable library under stringent conditions with a probe comprising one of the sequences of the nucleic acid molecule of (a) to (k), preferably to (a) to (c), or with a fragment of at least 15 nt, preferably 20 nt, 30 nt, 50 nt, 100 nt, 200 nt or 500 nt of the nucleic acid molecule characterized in (a) to (k), preferably to (a) to (c), and conferring an increase in the amount of the respective fine chemical in an organism or a part thereof;
or which comprises a sequence which is complementary thereto.

**[00103.1.23.23]** In one embodiment, the nucleic acid molecule used in the process of the invention distinguishes over the sequence indicated in Table IA, columns 5 or 7, lines 275 to 277 and/or 635 or 636, by one or more nucleotides. In one embodiment, the nucleic acid molecule used in the process of the invention does not consist of the sequence shown in indicated in Table I A, columns 5 or 7, lines 275 to 277 and/or 635 or 636. In one embodiment, the nucleic acid molecule used in the process of the invention is less than 100%, 99,999%, 99,99%, 99,9% or 99% identical to a sequence indicated in Table I A, columns 5 or 7, lines 275 to 277 and/or 635 or 636. In another embodiment, the nucleic acid molecule does not encode a polypeptide of a sequence indicated in Table II A, columns 5 or 7, lines 275 to 277 and/or 635 or 636.

**[00103.2.23.23]** In one embodiment, the nucleic acid molecule used in the process of the invention distinguishes over the sequence indicated in Table I B, columns 5 or 7, lines 275 to 277 and/or 635 or 636, by one or more nucleotides. In one embodiment, the nucleic acid molecule used in the process of the invention does not consist of the sequence shown in indicated in Table I B, columns 5 or 7, lines 275 to 277 and/or 635 or 636. In one embodiment, the nucleic acid molecule used in the process of the invention is less than 100%, 99,999%, 99,99%, 99,9% or 99% identical to a sequence indicated in Table I B, columns 5 or 7, lines 275 to 277 and/or 635 or 636. In another embodiment, the nucleic acid molecule does not encode a polypeptide of a sequence indicated in Table II B, columns 5 or 7, lines 275 to 277 and/or 635 or 636.

**[0104.0.23.23]** In one embodiment, the nucleic acid molecule used in the process of the present invention distinguishes over the sequence indicated in Table I, columns 5 or 7, lines 275 to 277 and/or 635 or 636, preverably over the sequences as shown in Table IA, columns 5 or 7, lines 275 to 277 and/or 635 or 636 by one or more nucleotides. In one embodiment, the nucleic acid molecule used in the process of the invention does not consist of the sequence indicated in Table I, columns 5 or 7, lines 275 to 277 and/or 635 or 636, preverably of the sequences as shown in Table IA, columns 5 or 7, lines 275 to 277 and/or 635 or 636. In one embodiment, the nucleic acid molecule of the present invention is less than 100%, 99,999%, 99,99%, 99,9% or 99% identical to the sequence indicated in Table I, columns 5 or 7, lines 275 to 277 and/or 635 or 636. In another embodiment, the nucleic acid molecule does not encode a polypeptide of a sequence indicated in Table II, columns 5 or 7, lines 275 to 277 and/or 635 or 636, preverably the nucleic acid molecule does not encode a polypeptide of a sequence as indicated in Table IIA, columns 5 or 7, lines 275 to 277 and/or 635 or 636.

**[0105.0.0.23] to [0107.0.0.23]** for the disclosure of the paragraphs [0105.0.0.23] to [0107.0.0.23] see paragraphs [0105.0.0.0] to [0107.0.0.0] above.

**[0108.0.23.23]** Nucleic acid molecules with the sequence as indicated in Table I, columns 5 or 7, lines 275 to 277 and/or 635 or 636, nucleic acid molecules which are derived from an amino acid sequences as indicated in Table II, columns 5 or 7, lines 275 to 277 and/or 635 or 636 or from polypeptides comprising the consensus sequence as indicated in Table IV, column 7, lines 275 to 277 and/or 635 or 636, or their derivatives or homologues encoding polypeptides with the enzymatic or biological activity of an activity of a polypeptide as indicated in Table II, columns 3, 5 or 7, lines 275 to 277 and/or 635 or 636, e.g. conferring the increase of the respective fine chemical, meaning salicylic acid and/or salicylic acid esters, resp., after increasing its expression or activity, are advantageously increased in the process according to the invention.

**[0109.0.23.23]** In one embodiment, said sequences are cloned into nucleic acid constructs, either individually or in combination. These nucleic acid constructs enable an optimal synthesis of the respective fine chemicals, in particular salicylic acid and/or salicylic acid esters, produced in the process according to the invention.

**[0110.0.0.23]** Nucleic acid molecules, which are advantageous for the process according to the invention and which encode polypeptides with an activity of a polypeptide used in the method of the invention or used in the process of the invention, e.g. of a protein as shown in Table II, columns 5 or 7, lines 275 to 277 and/or 635 or 636 or being encoded by a nucleic acid molecule indicated in Table I, columns 5 or 7, lines 275 to 277 and/or 635 or 636 or of its homologs, e.g. as indicated in Table II, columns 5 or 7, lines 275 to 277 and/or 635 or 636 can be determined from generally accessible databases.

**[0111.0.0.23]** for the disclosure of this paragraph see [0111.0.0.0] above.

**[0112.0.23.23]** The nucleic acid molecules used in the process according to the invention take the form of isolated nucleic acid sequences, which encode polypeptides with an activity of a polypeptide as indicated in Table II, column 3, lines 275 to 277 and/or 635 or 636 or having the sequence of a polypeptide as indicated in Table II, columns 5 and 7, lines 275 to 277 and/or 635 or 636 and conferring an increase in the salicylic acid and/or salicylic acid ester level.

**[0113.0.0.23] to [0120.0.0.23]** for the disclosure of the paragraphs [0113.0.0.23] to [0120.0.0.23] see paragraphs [0113.0.0.0] and [0120.0.0.0] above.

**[0120.1.0.23]: %**

**[0121.0.23.23]** However, it is also possible to use artificial sequences, which differ in one or more bases from the nucleic acid sequences found in organisms, or in one or more amino acid molecules from polypeptide sequences found in organisms, in particular from the polypeptide sequences indicated in Table II, columns 5 or 7, lines 275 to 277 and/or 635 or 636 or the functional homologues thereof as described herein, preferably conferring above-mentioned activity, i.e. conferring a salicylic acid increase after increasing the activity of the polypeptide sequences indicated in Table II, columns 5 or 7, lines 275 to 277 and/or 635 or 636 and conferring a salicylic acid level increase after increasing the activity of the polypeptide sequences indicated in Table II, columns 5 or 7, lines 275 to 277 and/or 635 or 636.

[0122.0.0.23] to [0127.0.0.23] for the disclosure of the paragraphs [0122.0.0.23] to [0127.0.0.23] see paragraphs [0122.0.0.0] and [0127.0.0.0] above.

**[0128.0.23.23]** Synthetic oligonucleotide primers for the amplification, e.g. as the pairs indicated in Table III, columns 7, lines 275 to 277 and/or 635 or 636, by means of polymerase chain reaction can be generated on the basis of a sequence shown herein, for example the sequence as indicated in Table I, columns 5 or 7, lines 275 to 277 and/or 635 or 636, resp. or the sequences derived from a sequence as indicated in Table II, columns 5 or 7, lines 275 to 277 and/or 635 or 636, resp.

**[0129.0.23.23]** Moreover, it is possible to identify conserved regions from various organisms by carrying out protein sequence alignments with the polypeptide used in the process of the invention, in particular with sequences of the polypeptide of the invention, from which conserved regions, and in turn, degenerate primers can be derived. Conserved regions are those, which show a very little variation in the amino acid in one particular position of several homologs from different origin. The consensus sequence indicated in Table IV, columns 7, lines 275 to 277 and/or 635 or 636 is derived from such alignments.

**[0130.0.23.23]** Degenerated primers can then be utilized by PCR for the amplification of fragments of novel proteins having above-mentioned activity, e.g. conferring the increase of salicylic acid and/or salicylic acid esters after increasing the expression or activity the protein comprising said fragment.

**[0131.0.0.23] to [0138.0.0.23]** for the disclosure of the paragraphs [0131.0.0.23] to [0138.0.0.23] see paragraphs [0131.0.0.0] to [0138.0.0.0] above.

**[0139.0.23.23]** Polypeptides having above-mentioned activity, i.e. conferring the respective fine chemical level increase, derived from other organisms, can be encoded by other DNA sequences which hybridise to a sequence indicated in Table I, columns 5 or 7, lines 275 to 277 and/or 635 or 636, preverably to a sequence as shown in Table IB, columns 5 or 7, lines 275 to 277 and/or 635 or 636 under relaxed hybridization conditions and which code on expression for peptides having the respective fine chemical, i.e. salicylic acid and/or salicylic acid ester, increasing-activity.

**[0140.0.0.23] to [0146.0.0.23]** for the disclosure of the paragraphs [0140.0.0.23] to [0146.0.0.23] see paragraphs [0140.0.0.0] to [0146.0.0.0] above.

**[0147.0.23.23]** Further, the nucleic acid molecule of the invention comprises a nucleic acid molecule, which is a complement of one of the nucleotide sequences of above mentioned nucleic acid molecules or a portion thereof. A nucleic acid molecule which is complementary to one of the nucleotide sequences indicated in Table I, columns 5 or 7, lines 275 to 277 and/or 635 or 636, preverably to a sequence as shown in Table IB, columns 5 or 7, lines 275 to 277 and/or 635 or 636 is one which is sufficiently complementary to one of said nucleotide sequences such that it can hybridise to one of said nucleotide sequences, thereby forming a stable duplex. Preferably, the hybridisation is performed under stringent hybrization conditions. However, a complement of one of the herein disclosed sequences is preferably a sequence complement thereto according to the base pairing of nucleic acid molecules well known to the skilled person. For example, the bases A and G undergo base pairing with the bases T and U or C, resp. and visa versa. Modifications of the bases can influence the base-pairing partner.

**[0148.0.23.23]** The nucleic acid molecule of the invention comprises a nucleotide sequence which is at least about 30%, 35%, 40% or 45%, preferably at least about 50%, 55%, 60% or 65%, more preferably at least about 70%, 80%, or 90%, and even more preferably at least about 95%, 97%, 98%, 99% or more homologous to a nucleotide sequence indicated in Table I, columns 5 or 7, lines 275 to 277 and/or 635 or 636, preverably to a sequence as shown in Table IB, columns 5 or 7, lines 275 to 277 and/or 635 or 636 or a portion thereof and preferably has above mentioned activity, in particular having a salicylic acid and/or salicylic acid increasing activity after increasing the activity or an activity of a product of a gene encoding said sequences or their homologs.

**[0149.0.23.23]** The nucleic acid molecule of the invention comprises a nucleotide sequence which hybridizes, preferably hybridizes under stringent conditions as defined herein, to one of the nucleotide sequences indicated in Table I, columns 5 or 7, lines 275 to 277 and/or 635 or 636, or a portion thereof and encodes a protein having above-mentioned activity, e.g. conferring an increase of salicylic acid and/or salicylic acid, resp., and optionally, the activity of protein indicated in Table II, column 5, lines 275 to 277 and/or 635 or 636.

**[00149.1.23.23]** Optionally, in one embodiment, the nucleotide sequence, which hybridises to one of the nucleotide sequences indicated in Table I, columns 5 or 7, lines 275 to 277 and/or 635 or 636, preverably to a sequence as shown in Table IB, columns 5 or 7, lines 275 to 277 and/or 635 or 636 has further one or more of the activities annotated or known for a protein as indicated in Table II, column 3, lines 275 to 277 and/or 635 or 636, preverably one or more activities annotated or known for a protein as indicated in Table IIB, columns 5 or 7, lines 275 to 277 and/or 635 or 636.

**[00149.1.22.22]** Optionally, the nucleotide sequence, which hybridises to one of the nucleotide sequences indicated in Table I, columns 5 or 7, lines 275 to 277 and/or 635 or 636, preferably of Table IB, columns 5 or 7, lines 275 to 277 and/or 635 or 636, has further one or more of the activities annotated or known for the a protein as indicated in Table II, column 3, lines 275 to 277 and/or lines 635 or 636, preferably of Table IIB, columns 5 or 7, lines 275 to 277 and/or 635 or 636.

**[0150.0.23.23]** Moreover, the nucleic acid molecule of the invention can comprise only a portion of the coding region of one of the sequences indicated in Table I, columns 5 or 7, lines 275 to 277 and/or 635 or 636, preferably indicated in Table IB, columns 5 or 7, lines 275 to 277 and/or 635 or 636 for example a fragment which can be used as a probe or primer or a fragment encoding a biologically active portion of the polypeptide of the present invention or of a polypeptide used in the process of the present invention, i.e. having above-mentioned activity, e.g.conferring an increase of salicylic acid and/or salicylic acid esters, resp., if its activity is increased. The nucleotide sequences determined from the cloning of the present protein-according-to-the-invention-encoding gene allows for the generation of probes and primers designed for use in identifying and/or cloning its homologues in other cell types and organisms. The probe/primer typically comprises substantially purified oligonucleotide. The oligonucleotide typically comprises a region of nucleotide sequence that hybridizes under stringent conditions to at least about 12, 15 preferably about 20 or 25, more preferably about 40, 50 or 75 consecutive nucleotides of a sense strand of one of the sequences set forth, e.g., as indicated in Table I, columns 5 or 7, lines 275 to 277 and/or 635 or 636, an anti-sense sequence of one of the sequences, e.g., as indicated in Table I, columns 5 or 7, lines 275 to 277 and/or 635 or 636, or naturally occurring mutants thereof. Primers based on a nucleotide of invention can be used in PCR reactions to clone homologues of the polypeptide of the invention or of the polypeptide used in the process of the invention, e.g. as the primers described in the examples of the present invention, e.g. as shown in the examples. A PCR with the primer pairs indicated in Table III, column 7, lines 275 to 277 and/or 635 or 636 will result in a fragment of a polynucleotide sequence as indicated in Table I, columns 5 or 7, lines 275 to 277 and/or 635 or 636 or its gene product. Preferably is Table IB, columns 5 or 7, lines 275 to 277 and/or 635 or 636.

**[0151.0.0.23]:** for the disclosure of this paragraph see paragraph [0151.0.0.0] above.

**[0152.0.23.23]** The nucleic acid molecule of the invention encodes a polypeptide or portion thereof which includes an amino acid sequence which is sufficiently homologous to an amino acid sequence as indicated in Table II, columns 5 or 7, lines 275 to 277 and/or 635 or 636 such that the protein or portion thereof maintains the ability to participate in the respective fine chemical production, in particular a salicylic acid increasing activity as mentioned above or as described in the examples in plants or microorganisms is comprised.

**[0153.0.23.23]** As used herein, the language "sufficiently homologous" refers to proteins or portions thereof which have amino acid sequences which include a minimum number of identical or equivalent amino acid residues (e.g., an amino acid residue which has a similar side chain as an amino acid residue in one of the sequences of the polypeptide of the present invention) to an amino acid sequence as indicated in Table II, columns 5 or 7, lines 275 to 277 and/or 635 or 636 such that the protein or portion thereof is able to participate in the increase of the respective fine chemical production. In one embodiment, a protein or portion thereof as indicated in Table II, columns 5 or 7, lines 275 to 277 and/or 635 or 636 has for example an activity of a polypeptide indicated in Table II, column 3, lines 275 to 277 and/or 635 or 636.

**[0154.0.23.23]** In one embodiment, the nucleic acid molecule of the present invention comprises a nucleic acid that encodes a portion of the protein of the present invention. The protein is at least about 30%, 35%, 40%, 45% or 50%, preferably at least about 55%, 60%, 65% or 70%, and more preferably at least about 75%, 80%, 85%, 90%, 91%, 92%, 93% or 94% and most preferably at least about 95%, 96%, 97%, 98%, 99% or more homologous to an entire amino acid sequence as indicated in Table II, columns 5 or 7, lines 275 to 277 and/or 635 or 636 and has above-mentioned activity, e.g.conferring preferably the increase of the respective fine chemical.

**[0155.0.0.23] and [0156.0.0.23]** for the disclosure of the paragraphs [0155.0.0.23] and [0156.0.0.23] see paragraphs [0155.0.0.0] and [0156.0.0.0] above.

**[0157.0.23.23]** The invention further relates to nucleic acid molecules that differ from one of the nucleotide sequences as indicated in Table I, columns 5 or 7, lines 275 to 277 and/or 635 or 636 (and portions thereof) due to degeneracy of the genetic code and thus encode a polypeptide of the present invention, in particular a polypeptide having above mentioned activity, e.g. conferring an increase in the respective fine chemical in a organism, e.g. as polypeptides comprising the sequence as indicated in Table IV, column 7, lines 275 to 277 and/or 635 or 636 or as polypeptides depicted in Table II, columns 5 or 7, lines 275 to 277 and/or 635 or 636 or the functional homologues. Advantageously, the nucleic acid molecule of the invention comprises, or in an other embodiment has, a nucleotide sequence encoding a protein comprising, or in an other embodiment having, an amino acid sequence of a consensus sequences as indicated in Table IV, column 7, lines 275 to 277 and/or 635 or 636or of the polypeptide as indicated in Table II, columns 5 or 7, lines 275 to 277 and/or 635 or 636, resp., or the functional homologues. In a still further embodiment, the nucleic acid molecule of the invention encodes a full length protein which is substantially homologous to an amino acid sequence comprising a consensus sequence as indicated in Table IV, column 7, lines 275 to 277 and/or 635 or 636 or of a polypeptide as indicated in Table II, columns 5 or 7, lines 275 to 277 and/or 635 or 636 or the functional homologues. However, in a preferred embodiment, the nucleic acid molecule of the present invention does not consist of a sequence as indicated in Table I, columns 5 or 7, lines 275 to 277 and/or 635 or 636, resp., preferably as indicated in Table I A, columns 5 or 7, lines 275 to 277 and/or 635 or 636, resp. Preferably the nucleic acid molecule of the invention is a functional homologue or identical to a nucleic acid molecule indicated in Table I B, column 7, lines 275 to 277 and/or 635 or 636.

**[0158.0.0.23] to [0160.0.0.23]** for the disclosure of the paragraphs [0158.0.0.23] to [0160.0.0.23] see paragraphs [0158.0.0.0] to [0160.0.0.0] above.

**[0161.0.23.23]** Accordingly, in another embodiment, a nucleic acid molecule of the invention is at least 15, 20, 25 or 30 nucleotides in length. Preferably, it hybridizes under stringent conditions to a nucleic acid molecule comprising a nucleotide sequence of the nucleic acid molecule of the present invention or used in the process of the present invention, e.g. comprising a sequence as indicated in Table I, columns 5 or 7, lines 275 to 277 and/or 635 or 636. The nucleic acid molecule is preferably at least 20, 30, 50, 100, 250 or more nucleotides in length.

**[0162.0.0.23]** for the disclosure of this paragraph see paragraph [0162.0.0.0] above.

**[0163.0.23.23]** Preferably, a nucleic acid molecule of the invention that hybridizes under stringent conditions to a sequence as indicated in Table I, columns 5 or 7, lines 275 to 277 and/or 635 or 636 corresponds to a naturally-occurring nucleic acid molecule of the invention. As used herein, a "naturally-occurring" nucleic acid molecule refers to an RNA or DNA molecule having a nucleotide sequence that occurs in nature (e.g., encodes a natural protein). Preferably, the nucleic acid molecule encodes a natural protein having above-mentioned activity, e.g. conferring the increase of the amount of the respective fine chemical in an organism or a part thereof, e.g. a tissue, a cell, or a compartment of a cell, after increasing the expression or activity thereof or the activity of a protein of the invention or used in the process of the invention.

**[0164.0.0.23]** for the disclosure of this paragraph see paragraph [0164.0.0.0] above.

**[0165.0.23.23]** For example, nucleotide substitutions leading to amino acid substitutions at "non-essential" amino acid residues can be made in a sequence of the nucleic acid molecule of the invention or used in the process of the invention, e.g. as indicated in Table I, columns 5 or 7, lines 275 to 277 and/or 635 or 636, resp.

**[0166.0.0.23] and [0167.0.0.23]** for the disclosure of the paragraphs [0166.0.0.23] and [0167.0.0.23] see paragraphs [0166.0.0.0] and [0167.0.0.0] above.

**[0168.0.23.23]** Accordingly, the invention relates to nucleic acid molecules encoding a polypeptide having above-mentioned activity, e.g. conferring an increase in the respective fine chemical in an organism or parts thereof that contain changes in amino acid residues that are not essential for said activity. Such polypeptides differ in amino acid sequence from a sequence contained in a sequence as indicated in Table II, columns 5 or 7, lines 275 to 277 and/or 635 or 636, resp., yet retain said activity described herein. The nucleic acid molecule can comprise a nucleotide sequence encoding a polypeptide, wherein the polypeptide comprises an amino acid sequence at least about 50% identical to an amino acid sequence as indicated in Table II, columns 5 or 7, lines 275 to 277 and/or 635 or 636, resp., and is capable of participation in the increase of production of the respective fine chemical after increasing its activity, e.g. its expression. Preferably, the protein encoded by the nucleic acid molecule is at least about 60% identical to a sequence as indicated in Table II, columns 5 or 7, lines 275 to 277 and/or 635 or 636, resp., more preferably at least about 70% identical to one of the sequences as indicated in Table II, columns 5 or 7, lines 275 to 277 and/or 635 or 636, resp., even more preferably at least about 80%, 90%, 95% homologous to a sequence as indicated in Table II, columns 5 or 7, lines 275 to 277 and/or 635 or 636, resp., and most preferably at least about 96%, 97%, 98%, or 99% identical to the sequence as indicated in Table II, columns 5 or 7, lines 275 to 277 and/or 635 or 636.
Accordingly, the invention relates to nucleic acid molecules encoding a polypeptide having above-mentioned activity, e.g. conferring an increase in the the respective fine chemical in an organisms or parts thereof that contain changes in amino acid residues that are not essential for said activity. Such polypeptides differ in amino acid sequence from a sequence contained in a sequence as indicated in Table II, columns 5 or 7, lines 275 to 277 and/or 635 or 636, preferably of Table II B, column 7, lines 275 to 277 and/or 635 or 636 yet retain said activity described herein. The nucleic acid molecule can comprise a nucleotide sequence encoding a polypeptide, wherein the polypeptide comprises an amino acid sequence at least about 50% identical to an amino acid sequence as indicated in Table II, columns 5 or 7, lines 275 to 277 and/or 635 or 636, preferably of Table II B, column 7, lines 275 to 277 and/or 635 or 636 and is capable of participation in the increase of production of the respective fine chemical after increasing its activity, e.g. its expression. Preferably, the protein encoded by the nucleic acid molecule is at least about 60% identical to a sequence as indicated in Table II, columns 5 or 7, lines 275 to 277 and/or 635 or 636, preferably of Table II B, column 7, lines 275 to 277 and/or 635 or 636, more preferably at least about 70% identical to one of the sequences as indicated in Table II, columns 5 or 7, lines 275 to 277 and/or 635 or 636, preferably of Table II B, column 7, lines 275 to 277 and/or 635 or 636, even more preferably at least about 80%, 90%, or 95% homologous to a sequence as indicated in Table II, columns 5 or 7, lines 275 to 277 and/or 635 or 636, preferably of Table II B, column 7, lines 275 to 277 and/or 635 or 636, and most preferably at least about 96%, 97%, 98%, or 99% identical to the sequence as indicated in Table II, columns 5 or 7, lines 275 to 277 and/or 635 or 636, preferably of Table II B, column 7, lines 275 to 277 and/or 635 or 636.

**[0169.0.0.23] to [0172.0.0.23]** for the disclosure of the paragraphs [0169.0.0.23] to [0172.0.0.23] see paragraphs [0169.0.0.0] to [0172.0.0.0] above.

**[0173.0.23.23]** For example a sequence, which has 80% homology with sequence SEQ ID NO: 33502 at the nucleic acid level is understood as meaning a sequence which, upon comparison with the sequence SEQ ID NO: 33502 by the above Gap program algorithm with the above parameter set, has a 80% homology.

**[0174.0.0.23]:** for the disclosure of this paragraph see paragraph [0174.0.0.0] above.

**[0175.0.23.23]** For example a sequence which has a 80% homology with sequence SEQ ID NO: 33503 at the protein level is understood as meaning a sequence which, upon comparison with the sequence SEQ ID NO: 33503 by the above program algorithm with the above parameter set, has a 80% homology.

**[0176.0.23.23]** Functional equivalents derived from one of the polypeptides as indicated in Table II, columns 5 or 7, lines 275 to 277 and/or 635 or 636, resp., according to the invention by substitution, insertion or deletion have at least 30%, 35%, 40%, 45% or 50%, preferably at least 55%, 60%, 65% or 70% by preference at least 80%, especially preferably at least 85% or 90%, 91 %, 92%, 93% or 94%, very especially preferably at least 95%, 97%, 98% or 99% homology with one of the polypeptides as indicated in Table II, columns 5 or 7, lines 275 to 277 and/or 635 or 636, resp., according to the invention and are distinguished by essentially the same properties as a polypeptide as indicated in Table II, columns 5 or 7, lines 275 to 277 and/or 635 or 636, resp.

**[0177.0.23.23]** Functional equivalents derived from a nucleic acid sequence as indicated in Table I, columns 5 or 7, lines 275 to 277 and/or 635 or 636, preferably of Table I B, columns 5 or 7, lines 275 to 277 and/or 635 or 636 resp., according to the invention by substitution, insertion or deletion have at least 30%, 35%, 40%, 45% or 50%, preferably at least 55%, 60%, 65% or 70% by preference at least 80%, especially preferably at least 85% or 90%, 91%, 92%, 93% or 94%, very especially preferably at least 95%, 97%, 98% or 99% homology with one of the polypeptides as indicated in Table II, columns 5 or 7, lines 275 to 277 and/or 635 or 636, resp., according to the invention and encode polypeptides having essentially the same properties as a polypeptide as indicated in Table II, columns 5 or 7, lines 275 to 277 and/or 635 or 636, preferably of Table II B, columns 5 or 7, lines 275 to 277 and/or 635 or 636, resp.

**[0178.0.0.23]** for the disclosure of this paragraph see [0178.0.0.0] above.

**[0179.0.23.23]** A nucleic acid molecule encoding a homologue to a protein sequence as indicated in Table II, columns 5 or 7, lines 275 to 277 and/or 635 or 636, , preferably in Table II B, columns 5 or 7, lines 275 to 277 and/or 635 or 636 resp., can be created by introducing one or more nucleotide substitutions, additions or deletions into a nucleotide sequence of the nucleic acid molecule of the present invention, in particular as indicated in Table I, columns 5 or 7, lines 275 to 277 and/or 635 or 636, preferably in Table I B, columns 5 or 7, lines 275 to 277 and/or 635 or 636, resp., such that one or more amino acid substitutions, additions or deletions are introduced into the encoded protein. Mutations can be introduced into the encoding sequences as indicated in Table I, columns 5 or 7, lines 275 to 277 and/or 635 or 636, preferably in Table I B, columns 5 or 7, lines 275 to 277 and/or 635 or 636, resp., by standard techniques, such as site-directed mutagenesis and PCR-mediated mutagenesis.

**[0180.0.0.23] to [0183.0.0.23]** for the disclosure of the paragraphs [0180.0.0.23] to [0183.0.0.23] see paragraphs [0180.0.0.0] to [0183.0.0.0] above.

**[0184.0.23.23]** Homologues of the nucleic acid sequences used, with a sequence as indicated in Table I, columns 5 or 7, lines 275 to 277 and/or 635 or 636, preferably in Table I B, columns 5 or 7, lines 275 to 277 and/or 635 or 636, resp., or of the nucleic acid sequences derived from a sequences as indicated in Table II, columns 5 or 7, lines 275 to 277 and/or 635 or 636, preferably in Table II B, columns 5 or 7, lines 275 to 277 and/or 635 or 636, resp., comprise also allelic variants with at least approximately 30%, 35%, 40% or 45% homology, by preference at least approximately 50%, 60% or 70%, more preferably at least approximately 90%, 91%, 92%, 93%, 94% or 95% and even more preferably at least approximately 96%, 97%, 98%, 99% or more homology with one of the nucleotide sequences shown or the abovementioned derived nucleic acid sequences or their homologues, derivatives or analogues or parts of these. Allelic variants encompass in particular functional variants which can be obtained by deletion, insertion or substitution of nucleotides from the sequences shown, preferably from a sequence as indicated in Table I, columns 5 or 7, lines 275 to 277 and/or 635 or 636, preferably in Table I B, columns 5 or 7, lines 275 to 277 and/or 635 or 636, resp., or from the derived nucleic acid sequences, the intention being, however, that the enzyme activity or the biological activity of the resulting proteins synthesized is advantageously retained or increased.

**[0185.0.23.23]** In one embodiment of the present invention, the nucleic acid molecule of the invention or used in the process of the invention comprises one or more sequences as indicated in Table I, columns 5 or 7, lines 275 to 277 and/or 635 or 636, preferably in Table I B, columns 5 or 7, lines 275 to 277 and/or 635 or 636, resp. In one embodiment, it is preferred that the nucleic acid molecule comprises as little as possible other nucleotides not shown in any one of sequences as indicated in Table I, columns 5 or 7, lines 275 to 277 and/or 635 or 636, preferably in Table I B, columns 5 or 7, lines 275 to 277 and/or 635 or 636, resp. In one embodiment, the nucleic acid molecule comprises less than 500, 400, 300, 200, 100, 90, 80, 70, 60, 50 or 40 further nucleotides. In a further embodiment, the nucleic acid molecule comprises less than 30, 20 or 10 further nucleotides. In one embodiment, a nucleic acid molecule used in the process of the invention is identical to a sequence as indicated in Table I, columns 5 or 7, lines 275 to 277 and/or 635 or 636, preferably in Table I B, columns 5 or 7, lines 275 to 277 and/or 635 or 636, resp.

**[0186.0.23.23]** Also preferred is that one or more nucleic acid molecule(s) used in the process of the invention encodes a polypeptide comprising a sequence as indicated in Table II, columns 5 or 7, lines 275 to 277 and/or 635 or 636, preferably in Table II B, columns 5 or 7, lines 275 to 277 and/or 635 or 636, resp. In one embodiment, the nucleic acid molecule encodes less than 150, 130, 100, 80, 60, 50, 40 or 30 further amino acids. In a further embodiment, the encoded polypeptide comprises less than 20, 15, 10, 9, 8, 7, 6 or 5 further amino acids. In one embodiment, the encoded polypeptide used in the process of the invention is identical to the sequences as indicated in Table II, columns 5 or 7, lines 275 to 277 and/or 635 or 636, preferably in Table II B, columns 5 or 7, lines 275 to 277 and/or 635 or 636, resp.

**[0187.0.23.23]** In one embodiment, a nucleic acid molecule of the invention or used in the process encodes a polypeptide comprising the sequence as indicated in Table II, columns 5 or 7, lines 275 to 277 and/or 635 or 636, preferably in Table II B, columns 5 or 7, lines 275 to 277 and/or 635 or 636, resp., comprises less than 100 further nucleotides. In a further embodiment, said nucleic acid molecule comprises less than 30 further nucleotides. In one embodiment, the nucleic acid molecule used in the process is identical to a coding sequence encoding a sequences as indicated in Table II, columns 5 or 7, lines 275 to 277 and/or 635 or 636, preferably in Table II B, columns 5 or 7, lines 275 to 277 and/or 635 or 636, resp.

**[0188.0.23.23]** Polypeptides (= proteins), which still have the essential enzymatic activity of the polypeptide of the present invention conferring an increase of the respective fine chemical i.e. whose activity is essentially not reduced, are polypeptides with at least 10% or 20%, by preference 30% or 40%, especially preferably 50% or 60%, very especially preferably 80% or 90 or more of the wild type biological activity or enzyme activity. Advantageously, the activity is essentially not reduced in comparison with the activity of a polypeptide as indicated in Table II, columns 5 or 7, lines 275 to 277 and/or 635 or 636, resp., and is expressed under identical conditions. **[0189.0.23.23]** Homologues of a sequences as indicated in Table I, columns 5 or 7, lines 275 to 277 and/or 635 or 636, resp., or of derived sequences as indicated in Table II, columns 5 or 7, lines 275 to 277 and/or 635 or 636, resp., also mean truncated sequences, cDNA, single-stranded DNA or RNA of the coding and noncoding DNA sequence. Homologues of said sequences are also understood as meaning derivatives, which comprise noncoding regions such as, for example, UTRs, terminators, enhancers or promoter variants. The promoters upstream of the nucleotide sequences stated can be modified by one or more nucleotide substitution(s), insertion(s) and/or deletion(s) without, however, interfering with the functionality or activity either of the promoters, the open reading frame (=ORF) or with the 3'-regulatory region such as terminators or other 3'regulatory regions, which are far away from the ORF. It is furthermore possible that the activity of the promoters is increased by modification of their sequence, or that they are replaced completely by more active promoters, even promoters from heterologous organisms. Appropriate promoters are known to the person skilled in the art and are mentioned herein below.

**[0190.0.0.23] and [0191.0.0.23]** for the disclosure of the paragraphs [0190.0.0.23] and [0191.0.0.23] see paragraphs [0190.0.0.0] and [0191.0.0.0] above.

**[0191.1.0.23]:** for the disclosure of this paragraph see [0191.1.0.0] above.

**[0192.0.0.23] to [0203.0.0.23]** for the disclosure of the paragraphs [0192.0.0.23] to [0203.0.0.23] see paragraphs [0192.0.0.0] to [0203.0.0.0] above.

**[0204.0.23.23]** Accordingly, in one embodiment, the invention relates to a nucleic acid molecule, which comprises a nucleic acid molecule selected from the group consisting of:
a) nucleic acid molecule encoding, preferably at least the mature form, of a polypeptide as indicated in Table II, columns 5 or 7, lines 275 to 277 and/or 635 or 636, preferably in Table II B, columns 5 or 7, lines 275 to 277 and/or 635 or 636, resp.; or a fragment thereof conferring an increase in the amount of the respective fine chemical, i.e. salicylic acid in an organism or a part thereof,
b) nucleic acid molecule comprising, preferably at least the mature form, of a nucleic acid molecule as indicated in Table I, columns 5 or 7, lines 275 to 277 and/or 635 or 636, preferably in Table I B, columns 5 or 7, lines 275 to 277 and/or 635 or 636 resp., or a fragment thereof conferring an increase in the amount of the respective fine chemical in an organism or a part thereof;
c) nucleic acid molecule whose sequence can be deduced from a polypeptide sequence encoded by a nucleic acid molecule of (a) or (b) as result of the degeneracy of the genetic code and conferring an increase in the amount of the respective fine chemical in an organism or a part thereof;
d) nucleic acid molecule encoding a polypeptide whose sequence has at least 50% identity with the amino acid sequence of the polypeptide encoded by the nucleic acid molecule of (a) to (c) and conferring an increase in the amount of the respective fine chemical in an organism or a part thereof;
e) nucleic acid molecule which hybridizes with a nucleic acid molecule of (a) to (c) under stringent hybridisation conditions and conferring an increase in the amount of the respective fine chemical in an organism or a part thereof;
f) nucleic acid molecule encoding a polypeptide, the polypeptide being derived by substituting, deleting and/or adding one or more amino acids of the amino acid sequence of the polypeptide encoded by the nucleic acid molecules (a) to (d), preferably to (a) to (c), and conferring an increase in the amount of the respective fine chemical in an organism or a part thereof;
g) nucleic acid molecule encoding a fragment or an epitope of a polypeptide which is encoded by one of the nucleic acid molecules of (a) to (e), preferably to (a) to (c) and conferring an increase in the amount of the respective fine chemical in an organism or a part thereof;
h) nucleic acid molecule comprising a nucleic acid molecule which is obtained by amplifying a cDNA library or a genomic library using primers or primer pairs as indicated in Table III, column 7, lines 275 to 277 and/or 635 or 636 and conferring an increase in the amount of the respective fine chemical, i.e. salicylic acid in an organism or a part thereof;
i) nucleic acid molecule encoding a polypeptide which is isolated, e.g. from a expression library, with the aid of monoclonal antibodies against a polypeptide encoded by one of the nucleic acid molecules of (a) to (g), preferably to (a) to (c) and conferring an increase in the amount of the respective fine chemical in an organism or a part thereof;
j) nucleic acid molecule which encodes a polypeptide comprising a consensus sequence as indicated in Table IV, column 7, line 275 to 277 and/or 635 or 636 and conferring an increase in the amount of the respective fine chemical, i.e. salicylic acid, in an organism or a part thereof;
k) nucleic acid molecule encoding the amino acid sequence of a polypeptide encoding a domaine of a polypeptide as indicated in Table II, columns 5 or 7, lines 275 to 277 and/or 635 or 636, resp., and conferring an increase in the amount of the respective fine chemical, i.e. salicylic acidin an organism or a part thereof; and
l) nucleic acid molecule which is obtainable by screening a suitable nucleic acid library under stringent hybridization conditions with a probe comprising one of the sequences of the nucleic acid molecule of (a) to (k) or with a fragment of at least 15 nt, preferably 20 nt, 30 nt, 50 nt, 100 nt, 200 nt or 500 nt of the nucleic acid molecule characterized in (a) to (h) or of a nucleic acid molecule as indicated in Table I, columns 5 or 7, lines 275 to 277 and/or 635 or 636, resp., or a nucleic acid molecule encoding, preferably at least the mature form of, a polypeptide as indicated in Table II, columns 5 or 7, lines 275 to 277 and/or 635 or 636, resp., and conferring an increase in the amount of the respective fine chemical in an organism or a part thereof; or which encompasses a sequence which is complementary thereto;
whereby, preferably, the nucleic acid molecule according to (a) to (I) distinguishes over a sequence as indicated in Table IA or IB, columns 5 or 7, lines 275 to 277 and/or 635 or 636, resp., by one or more nucleotides. In one embodiment, the nucleic acid molecule of the invention does not consist of the sequence as indicated in Table IA or IB, columns 5 or 7, lines 275 to 277 and/or 635 or 636, resp. In another embodiment, the nucleic acid molecule of the present invention is at least 30 % identical and less than 100%, 99,999%, 99,99%, 99,9% or 99% identical to a sequence as indicated in Table IA or IB, columns 5 or 7, lines 275 to 277 and/or 635 or 636, resp. In a further embodiment the nucleic acid molecule does not encode a polypeptide sequence as indicated in Table IIA or IIB, columns 5 or 7, lines 275 to 277 and/or 635 or 636, resp. Accordingly, in one embodiment, the nucleic acid molecule of the present invention encodes in one embodiment a polypeptide which differs at least in one or more amino acids from a polypeptide indicated in Table IIA or IIB, columns 5 or 7, lines 275 to 277 and/or 635 or 636 does not encode a protein of a sequence as indicated in Table IIA or IIB, columns 5 or 7, lines 275 to 277 and/or 635 or 636. Accordingly, in one embodiment, the protein encoded by a sequence of a nucleic acid according to (a) to (I) does not consist of a sequence as indicated in Table IIA or IIB, columns 5 or 7, lines 275 to 277 and/or 635 or 636. In a further embodiment, the protein of the present invention is at least 30 % identical to a protein sequence indicated in Table IIA or IIB, columns 5 or 7, lines 275 to 277 and/or 635 or 636 and less than 100%, preferably less than 99,999%, 99,99% or 99,9%, more preferably less than 99%, 98%, 97%, 96% or 95% identical to a sequence as indicated in Table IIA or IIB, columns 5 or 7, lines 275 to 277 and/or 635 or 636.

**[0205.0.0.23] and [0206.0.0.23]** for the disclosure of the paragraphs [0205.0.0.23] and [0206.0.0.23] see paragraphs [0205.0.0.0] and [0206.0.0.0] above.

**[0207.0.23.23]** As described herein, the nucleic acid construct can also comprise further genes, which are to be introduced into the organisms or cells. It is possible and advantageous to introduce into, and express in, the host organisms regulatory genes such as genes for inductors, repressors or enzymes, which, owing to their enzymatic activity, engage in the regulation of one or more genes of a biosynthetic pathway. These genes can be of heterologous or homologous origin. Moreover, further biosynthesis genes may advantageously be present, or else these genes may be located on one or more further nucleic acid constructs. Genes, which are advantageously employed as biosynthesis genes, are genes of the glutamic acid metabolism, the phosphoenolpyruvate metabolism, the amino acid metabolism, of glycolysis, of the tricarboxylic acid metabolism or their combinations. As described herein, regulator sequences or factors can have a positive effect on preferably the gene expression of the genes introduced, thus increasing it. Thus, an enhancement of the regulator elements may advantageously take place at the transcriptional level by using strong transcription signals such as promoters and/or enhancers. In addition, however, an enhancement of translation is also possible, for example by increasing mRNA stability or by inserting a translation enhancer sequence.

**[0208.0.0.23] to [0226.0.0.23]** for the disclosure of the paragraphs [0208.0.0.23] to [0226.0.0.23] see paragraphs [0208.0.0.0] to [0226.0.0.0] above.

**[0227.0.23.23]** The abovementioned nucleic acid molecules can be cloned into the nucleic acid constructs or vectors according to the invention in combination together with further genes, or else different genes are introduced by transforming several nucleic acid constructs or vectors (including plasmids) into a host cell, advantageously into a plant cell or a microorganisms.
In addition to a sequence indicated in Table I, columns 5 or 7, lines 275 to 277 and/or 635 or 636 or its derivatives, it is advantageous to express and/or mutate further genes in the organisms. Especially advantageously, additionally at least one further gene of the cinnamate and/or chorismate biosynthetic pathway is expressed in the organisms such as plants or microorganisms. It is also possible that the regulation of the natural genes has been modified advantageously so that the gene and/or its gene product is no longer subject to the regulatory mechanisms which exist in the organisms. This leads to an increased synthesis of the fine chemicals desired since, for example, feedback regulations no longer exist to the same extent or not at all. In addition it might be advantageously to combine one or more of the sequences indicated in Table I, columns 5 or 7, lines 275 to 277 and/or 635 or 636, resp., with genes which generally support or enhance to growth or yield of the target organismen, for example genes which lead to faster growth rate of microorganisms or genes which produces stress-, pathogen, or herbicide resistant plants.

**[0228.0.23.23]** %

**[0229.0.23.23]** Further advantageous nucleic acid sequences which can be expressed in combination with the sequences indicated in Table I, columns 5 or 7, lines 275 to 277 and/or 635 or 636 used in the process and/or the abovementioned biosynthesis genes are the sequences encoding further genes relating to the cinnamate or chorismate biosynthetic anabolic or catabolic pathway.

**[0230.0.0.23]** for the disclosure of this paragraph see [0230.0.0.0] above.

**[0231.0.23.23]** In a further advantageous embodiment of the process of the invention, the organisms used in the process are those in which simultaneously a salicylic aciddegrading protein is attenuated, in particular by reducing the rate of expression of the corresponding gene. A person skilled in the art knows for example, that the inhibition or repression of a salicylic aciddegrading enzyme will result in an increased accumulation of salicylic acid in plants.

**[0232.0.0.23] to [0276.0.0.23]** for the disclosure of the paragraphs [0232.0.0.23] to [0276.0.0.23] see paragraphs [0232.0.0.0] to [0276.0.0.0] above.

**[0277.0.23.23]** The respective fine chemical produced can be isolated from the organism by methods with which the skilled worker are familiar, for example via extraction, salt precipitation, and/or different chromatography methods. The process according to the invention can be conducted batchwise, semibatchwise or continuously.

**[0278.0.0.23] to [0282.0.0.23]** for the disclosure of the paragraphs [0278.0.0.23] to [0282.0.0.23] see paragraphs [0278.0.0.0] to [0282.0.0.0] above.

**[0283.0.23.23]** Moreover, native polypeptide conferring the increase of the respective fine chemical in an organism or part thereof can be isolated from cells, for example using the antibody of the present invention as described below, e.g. an antibody against a protein as indicated in Table II, column 3, lines 275 to 277 and/or 635 or 636, resp., or an antibody against a polypeptide as indicated in Table II, columns 5 or 7, lines 275 to 277 and/or 635 or 636, resp., which can be produced by standard techniques utilizing the polypeptid of the present invention or fragment thereof. Preferred are monoclonal antibodies specifically binding to polypeptides as indicated in Table II, columns 5 or 7, lines 275 to 277 and/or 635 or 636, more preferred specifically binding to polypeptides as indicated in Table II, column 5, lines 275 to 277 and/or 635 or 636.

**[0284.0.0.23]** for the disclosure of this paragraph see [0284.0.0.0] above.

**[0285.0.23.23]** In one embodiment, the present invention relates to a polypeptide having a sequence as indicated in Table II, columns 5 or 7, lines 275 to 277 and/or 635 or 636, resp., or as coded by a nucleic acid molecule as indicated in Table I, columns 5 or 7, lines 275 to 277 and/or 635 or 636, resp., or functional homologues thereof.

**[0286.0.23.23]** In one advantageous embodiment, in the method of the present invention the activity of a polypeptide is increased which comprises or consists of a consensus sequence as indicated in Table IV, column 7, lines 275 to 277 and/or 635 or 636 and in one another embodiment, the present invention relates to a polypeptide comprising or consisting of a consensus sequence as indicated in Table IV, column 7, lines 275 to 277 and/or 635 or 636 whereby 20 or less, preferably 15 or 10, preferably 9, 8, 7, or 6, more preferred 5 or 4, even more preferred 3, even more preferred 2, even more preferred 1, most preferred 0 of the amino acids positions indicated can be replaced by any amino acid or, in an further embodiment, can be replaced and/or absent. In one embodiment, the present invention relates to the method of the present invention comprising a polypeptide or to a polypeptide comprising more than one consensus sequences (of an individual line) as indicated in Table IV, column 7, lines 275 to 277 and/or 635 or 636.

**[0287.0.0.23] to [0290.0.0.23]** for the disclosure of the paragraphs [0287.0.0.23] to [0290.0.0.23] see paragraphs [0287.0.0.0] to [0290.0.0.0] above.

**[0291.0.23.23]** In one advantageous embodiment, the method of the present invention comprises the increasing of a polypeptide comprising or consisting of plant or microorganism specific consensus sequences.
In one embodiment, said polypeptide of the invention distinguishes over a sequence as indicated in Table IIA or IIB, columns 5 or 7, lines 275 to 277 and/or 635 or 636, resp., by one or more amino acids. In one embodiment, polypeptide distinguishes from a sequence as indicated in Table IIA or IIB, columns 5 or 7, lines 275 to 277 and/or 635 or 636, resp., by more than 5, 6, 7, 8 or 9 amino acids, preferably by more than 10, 15, 20, 25 or 30 amino acids, even more preferred are more than 40, 50, or 60 amino acids and, preferably, the sequence of the polypeptide of the invention distinguishes from a sequence as indicated in Table IIA or IIB, columns 5 or 7, lines 275 to 277 and/or 635 or 636, resp., by not more than 80% or 70% of the amino acids, preferably not more than 60% or 50%, more preferred not more than 40% or 30%, even more preferred not more than 20% or 10%. In an other embodiment, said polypeptide of the invention does not consist of a sequence as indicated in Table IIA or IIB, columns 5 or 7, lines 275 to 277 and/or 635 or 636.

**[0292.0.0.23]** for the disclosure of this paragraph see [0292.0.0.0] above.

**[0293.0.23.23]** In one embodiment, the invention relates to a polypeptide conferring an increase in the fine chemical in an organism or part being encoded by the nucleic acid molecule of the invention or by a nucleic acid molecule used in the process of the invention. In one embodiment, the polypeptide of the invention has a sequence which distinguishes from a sequence as indicated in Table IIA or IIB, columns 5 or 7, lines 275 to 277 and/or 635 or 636, resp., by one or more amino acids. In an other embodiment, said polypeptide of the invention does not consist of the sequence as indicated in Table IIA or IIB, columns 5 or 7, lines 275 to 277 and/or 635 or 636, resp. In a further embodiment, said polypeptide of the present invention is less than 100%, 99,999%, 99,99%, 99,9% or 99% identical. In one embodiment, said polypeptide does not consist of the sequence encoded by a nucleic acid molecules as indicated in Table IA or IB, columns 5 or 7, lines 275 to 277 and/or 635 or 636, resp.

**[0294.0.23.23]** In one embodiment, the present invention relates to a polypeptide having an activity of a protein as indicated in Table IIA or IIB, column 3, lines 275 to 277 and/or 635 or 636, resp., which distinguishes over a sequence as indicated in Table IIA or IIB, columns 5 or 7, lines 275 to 277 and/or 635 or 636, resp., by one or more amino acids, preferably by more than 5, 6, 7, 8 or 9 amino acids, preferably by more than 10, 15, 20, 25 or 30 amino acids, even more preferred are more than 40, 50, or 60 amino acids but even more preferred by less than 70% of the amino acids, more preferred by less than 50%, even more preferred my less than 30% or 25%, more preferred are 20% or 15%, even more preferred are less than 10%.

**[0295.0.0.23] to [0297.0.0.23]** for the disclosure of the paragraphs [0295.0.0.23] to [0297.0.0.23] see paragraphs [0295.0.0.0] to [0297.0.0.0] above.

**[00297.1.0.23]** %

**[0298.0.23.23]** A polypeptide of the invention can participate in the process of the present invention. The polypeptide or a portion thereof comprises preferably an amino acid sequence which is sufficiently homologous to an amino acid sequence as indicated in Table II, columns 5 or 7, lines 275 to 277 and/or 635 or 636, resp. The portion of the protein is preferably a biologically active portion as described herein. Preferably, the polypeptide used in the process of the invention has an amino acid sequence identical to a sequence as indicated in Table II, columns 5 or 7, lines 275 to 277 and/or 635 or 636, resp.

**[0299.0.23.23]** Further, the polypeptide can have an amino acid sequence which is encoded by a nucleotide sequence which hybridizes, preferably hybridizes under stringent conditions as described above, to a nucleotide sequence of the nucleic acid molecule of the present invention. Accordingly, the polypeptide has an amino acid sequence which is encoded by a nucleotide sequence that is at least about 35%, 40%, 45%, 50%, 55%, 60%, 65% or 70%, preferably at least about 75%, 80%, 85% or 90, and more preferably at least about 91%, 92%, 93%, 94% or 95%, and even more preferably at least about 96%, 97%, 98%, 99% or more homologous to one of the nucleotide sequence as indicated in Table I, columns 5 or 7, lines 275 to 277 and/or 635 or 636, resp. The preferred polypeptide of the present invention preferably possesses at least one of the activities according to the invention and described herein. A preferred polypeptide of the present invention includes an amino acid sequence encoded by a nucleotide sequence which hybridizes, preferably hybridizes under stringent conditions, to a nucleotide sequence as indicated in Table I, columns 5 or 7, lines 275 to 277 and/or 635 or 636, resp., or which is homologous thereto, as defined above.

**[0300.0.23.23]** Accordingly the polypeptide of the present invention can vary from a sequence as indicated in Table II, columns 5 or 7, lines 275 to 277 and/or 635 or 636, resp., in amino acid sequence due to natural variation or mutagenesis, as described in detail herein. Accordingly, the polypeptide comprise an amino acid sequence which is at least about 35%, 40%, 45%, 50%, 55%, 60%, 65% or 70%, preferably at least about 75%, 80%, 85% or 90, and more preferably at least about 91%, 92%, 93%, 94% or 95%, and most preferably at least about 96%, 97%, 98%, 99% or more homologous to an entire amino acid sequence of as indicated in Table IIA or IIB, columns 5 or 7, lines 275 to 277 and/or 635 or 636, resp.

**[0301.0.0.23]** for the disclosure of this paragraph see [0301.0.0.0] above.

**[0302.0.23.23]** Biologically active portions of an polypeptide of the present invention include peptides comprising amino acid sequences derived from the amino acid sequence of the polypeptide of the present invention or used in the process of the present invention, e.g., an amino acid sequence as indicated in Table II, columns 5 or 7, lines 275 to 277 and/or 635 or 636, resp., or the amino acid sequence of a protein homologous thereto, which include fewer amino acids than a full length polypeptide of the present invention or used in the process of the present invention or the full length protein which is homologous to an polypeptide of the present invention or used in the process of the present invention depicted herein, and exhibit at least one activity of polypeptide of the present invention or used in the process of the present invention.

**[0303.0.0.23]** for the disclosure of this paragraph see [0303.0.0.0] above. **[0304.0.23.23]** Manipulation of the nucleic acid molecule of the invention may result in the production of a protein having essentially the activity of the polypeptides as indicated in Table II, column 3, lines 275 to 277 and/or 635 or 636 but having differences in the sequence from said wild-type protein. These proteins may be improved in efficiency or activity, may be present in greater numbers in the cell than is usual, or may be decreased in efficiency or activity in relation to the wild type protein.

**[0305.0.0.23] to [0308.0.0.23]** for the disclosure of the paragraphs [0305.0.0.23] to [0308.0.0.23] see paragraphs [0305.0.0.0] to [0308.0.0.0] above.

**[0306.1.0.22]** %

**[0309.0.23.23]** In one embodiment, a reference to a protein (= polypeptide) of the invention or as indicated in Table II, columns 5 or 7, lines 275 to 277 and/or 635 or 636, resp., refers to a polypeptide having an amino acid sequence corresponding to the polypeptide of the invention or used in the process of the invention, whereas an "other polypeptide" not being indicated in Table II, columns 5 or 7, lines 275 to 277 and/or 635 or 636, resp., refers to a polypeptide having an amino acid sequence corresponding to a protein which is not substantially homologous to a polypeptide of the invention, preferably which is not substantially homologous to a polypeptide as indicated in Table II, columns 5 or 7, lines 275 to 277 and/or 635 or 636, resp., e.g., a protein which does not confer the activity described herein or annotated or known for as indicated in Table II, column 3, lines 275 to 277 and/or 635 or 636, resp., and which is derived from the same or a different organism. In one embodiment, an "other polypeptide" not being indicated in Table II, columns 5 or 7, lines 275 to 277 and/or 635 or 636, resp., does not confer an increase of the respective fine chemical in an organism or part thereof.

**[0310.0.0.23] to [0334.0.0.23]** for the disclosure of the paragraphs [0310.0.0.23] to [0334.0.0.23] see paragraphs [0310.0.0.0] to [0334.0.0.0] above.

**[0335.0.23.23]** The dsRNAi method has proved to be particularly effective and advantageous for reducing the expression of a nucleic acid sequences as indicated in Table I, columns 5 or 7, lines 275 to 277 and/or 635 or 636, resp., and/or homologs thereof. As described inter alia in WO 99/32619, dsRNAi approaches are clearly superior to traditional antisense approaches. The invention therefore furthermore relates to double-stranded RNA molecules (dsRNA molecules) which, when introduced into an organism, advantageously into a plant (or a cell, tissue, organ or seed derived there from), bring about altered metabolic activity by the reduction in the expression of a nucleic acid sequences as indicated in Table I, columns 5 or 7, lines 275 to 277 and/or 635 or 636, resp., and/or homologs thereof. In a double-stranded RNA molecule for reducing the expression of a protein encoded by a nucleic acid sequence as indicated in Table I, columns 5 or 7, lines 275 to 277 and/or 635 or 636, resp., and/or homologs thereof, one of the two RNA strands is essentially identical to at least part of a nucleic acid sequence, and the respective other RNA strand is essentially identical to at least part of the complementary strand of a nucleic acid sequence.

**[0336.0.0.23] to [0342.0.0.23]** for the disclosure of the paragraphs [0336.0.0.23] to [0342.0.0.23] see paragraphs [0336.0.0.0] to [0342.0.0.0] above.

**[0343.0.23.23]** As has already been described, 100 % sequence identity between the dsRNA and a gene transcript of a nucleic acid sequence as indicated in Table I, columns 5 or 7, lines 275 to 277 and/or 635 or 636, resp., or it's homolog is not necessarily required in order to bring about effective reduction in the expression. The advantage is, accordingly, that the method is tolerant with regard to sequence deviations as may be present as a consequence of genetic mutations, polymorphisms or evolutionary divergences. Thus, for example, using the dsRNA, which has been generated starting from a sequence as indicated in Table I, columns 5 or 7, lines 275 to 277 and/or 635 or 636, resp., or homologs thereof of the one organism, may be used to suppress the corresponding expression in another organism.

**[0344.0.0.23] to [0361.0.0.23]** for the disclosure of the paragraphs [0344.0.0.23] to [0361.0.0.23] see paragraphs [0344.0.0.0] to [0361.0.0.0] above.

**[0362.0.23.23]** Accordingly the present invention relates to any cell transgenic for any nucleic acid characterized as part of the invention, e.g. conferring the increase of the respective fine chemical in a cell or an organism or a part thereof, e.g. the nucleic acid molecule of the invention, the nucleic acid construct of the invention, the antisense molecule of the invention, the vector of the invention or a nucleic acid molecule encoding the polypeptide of the invention, e.g. the polypeptide as indicated in Table II, columns 5 or 7, lines 275 to 277 and/or 635 or 636, resp., e.g. encoding a polypeptide having protein activity, as indicated in Table II, columns 3, lines 275 to 277 and/or 635 or 636, resp.. Due to the above-mentioned activity the respective fine chemical content in a cell or an organism is increased. For example, due to modulation or manipulation, the cellular activity of the polypeptide of the invention or nucleic acid molecule of the invention is increased, e.g. due to an increased expression or specific activity of the subject matters of the invention in a cell or an organism or a part thereof. Transgenic for a polypeptide having an activity of a polypeptide as indicated in Table II, columns 5 or 7, lines 275 to 277 and/or 635 or 636, resp., means herein that due to modulation or manipulation of the genome, an activity as annotated for a polypeptide as indicated in Table II, column 3, lines 275 to 277 and/or 635 or 636, e.g. having a sequence as indicated in Table II, columns 5 or 7, lines 275 to 277 and/or 635 or 636, resp., is increased in a cell or an organism or a part thereof. Examples are described above in context with the process of the invention.

**[0363.0.0.23]** for the disclosure of this paragraph see paragraph [0363.0.0.0] above.

**[0364.0.23.23]** A naturally occurring expression cassette - for example the naturally occurring combination of a promoter of a gene encoding a polypeptide of the invention as indicated in Table II, column 3, lines 275 to 277 and/or 635 or 636, resp. with the corresponding protein-encoding sequence as indicated in Table I, column 5, lines 275 to 277 and/or 635 or 636, resp., becomes a transgenic expression cassette when it is modified by non-natural, synthetic "artificial" methods such as, for example, mutagenization. Such methods have been described (US 5,565,350; WO 00/15815; also see above).

**[0365.0.0.23] to [0373.0.0.23]** for the disclosure of the paragraphs [0365.0.0.23] to [0373.0.0.23] see paragraphs [0365.0.0.0] to [0373.0.0.0] above.

**[0374.0.23.23]** Transgenic plants comprising the respective fine chemical synthesized in the process according to the invention can be marketed directly without isolation of the compounds synthesized. In the process according to the invention, plants are understood as meaning all plant parts, plant organs such as leaf, stalk, root, tubers or seeds or propagation material or harvested material or the intact plant. In this context, the seed encompasses all parts of the seed such as the seed coats, epidermal cells, seed cells, endosperm or embryonic tissue. Salicylic acid, in particular the respective fine chemical, produced in the process according to the invention may, however, also be isolated from the plant in the form of their free salicylic acid in particular the free respective fine chemical, or bound in or to compounds or moieties as for example but not limited to esters. The respective fine chemical produced by this process can be harvested by harvesting the organisms either from the culture in which they grow or from the field. This can be done via expressing, grinding and/or extraction, salt precipitation and/or ion-exchange chromatography or other chromatographic methods of the plant parts, preferably the plant seeds, plant fruits, plant tubers and the like.

**[0375.0.0.23] and [0376.0.0.23]** for the disclosure of the paragraphs [0375.0.0.23] and [0376.0.0.23] see paragraphs [0375.0.0.0] and [0376.0.0.0] above.

**[0377.0.23.23]** Accordingly, the present invention relates also to a process whereby the produced salicylic acid and/or salicylic acid ester is isolated.

**[0378.0.23.23]** In this manner, more than 50% by weight, advantageously more than 60% by weight, preferably more than 70% by weight, especially preferably more than 80% by weight, very especially preferably more than 90% by weight, of the salicylic acid and/or salicylic acid ester produced in the process can be isolated. The resulting salicylic acid and/or salicylic acid ester can, if appropriate, subsequently be further purified, if desired mixed with other active ingredients such as vitamins, amino acids, carbohydrates, antibiotics and the like, and, if appropriate, formulated.

**[0379.0.23.23]** In one embodiment the product produced by the present invention is a mixture of the respective fine chemicals salicylic acid and/or salicylic acid esters.

**[0380.0.23.23]** The salicylic acid obtained in the process by carrying out the invention is suitable as starting material for the synthesis of further products of value. For example, they can be used in combination with each other or alone for the production of pharmaceuticals, foodstuffs, animal feeds or cosmetics. Accordingly, the present invention relates to a method for the production of pharmaceuticals, food stuff, animal feeds, nutrients or cosmetics comprising the steps of the process according to the invention, including the isolation of the salicylic acid and salicylic acid ester composition produced or the respective fine chemical produced if desired and formulating the product with a pharmaceutical acceptable carrier or formulating the product in a form acceptable for an application in agriculture. A further embodiment according to the invention is the use of the salicylic acid and/or salicylic acid esters produced in the process or of the transgenic organism in animal feeds, foodstuffs, medicines, food supplements, cosmetics or pharmaceuticals or for the production of salicylic acid and /or salicylic acid esters e.g. after isolation of the respective fine chemical or without, e.g. in situ, e.g in the organism used for the process for the production of the respective fine chemical.

**[0381.0.0.23] and [0382.0.0.23]** for the disclosure of the paragraphs [0381.0.0.23] and [0382.0.0.23] see paragraphs [0381.0.0.0] and [0382.0.0.0] above.

**[0383.0.23.23]** %

**[0384.0.0.23]** for the disclosure of this paragraph see [0384.0.0.0] above.

**[0385.0.23.23]** The fermentation broths obtained in this way, containing in particular salicylic acid and/or salicylic acid esters in mixtures with other organic acids, amino acids, polypeptides or polysaccarides, normally have a dry matter content of from 1 to 70% by weight, preferably 7.5 to 25% by weight. Sugar-limited fermentation is additionally advantageous, e.g. at the end, for example over at least 30% of the fermentation time. This means that the concentration of utilizable sugar in the fermentation medium is kept at, or reduced to, 0 to 10 g/l, preferably to 0 to 3 g/I during this time. The fermentation broth is then processed further. Depending on requirements, the biomass can be removed or isolated entirely or partly by separation methods, such as, for example, centrifugation, filtration, decantation, coagulation/flocculation or a combination of these methods, from the fermentation broth or left completely in it.
The fermentation broth can then be thickened or concentrated by known methods, such as, for example, with the aid of a rotary evaporator, thin-film evaporator, falling film evaporator, by reverse osmosis or by nanofiltration. This concentrated fermentation broth can then be worked up by freeze-drying, spray drying, spray granulation or by other processes.

**[0386.0.23.23]** Accordingly, it is possible to purify the salicylic acid and/or salicylic acid esters produced according to the invention further. For this purpose, the product-containing composition is subjected for example to separation via e.g. an open column chromatography or HPLC in which case the desired product or the impurities are retained wholly or partly on the chromatography resin. These chromatography steps can be repeated if necessary, using the same or different chromatography resins. The skilled worker is familiar with the choice of suitable chromatography resins and their most effective use.

**[0387.0.0.23] to [0392.0.0.23]** for the disclosure of the paragraphs [0387.0.0.23] to [0392.0.0.23] see paragraphs [0387.0.0.0] to [0392.0.0.0] above.

**[0393.0.23.23]** In one embodiment, the present invention relates to a method for the identification of a gene product conferring an increase in the respective fine chemical production in a cell, comprising the following steps:
a. contacting,̅ e.g. hybridising, the nucleic acid molecules of a sample, e.g. cells, tissues, plants or microorganisms or a nucleic acid library , which can contain a candidate gene encoding a gene product conferring an increase in the respective fine chemical after expression, with the nucleic acid molecule of the present invention;
b. identifying the nucleic acid molecules, which hybridize under relaxed stringent conditions with the nucleic acid molecule of the present invention in particular to the nucleic acid molecule sequence as indicated in Table I, columns 5 or 7, lines 275 to 277 and/or 635 or 636, preferably in Table I B, columns 5 or 7, lines 275 to 277 and/or 635 or 636, resp., and, optionally, isolating the full length cDNA clone or complete genomic clone;
c. introducing the candidate nucleic acid molecules in host cells, preferably in a plant cell or a microorganism, appropriate for producing the respective fine chemical;
d. expressing the identified nucleic acid molecules in the host cells;
e. assaying the respective fine chemical level in the host cells; and
f. identifying the nucleic acid molecule and its gene product which expression confers an increase in the respective fine chemical level in the host cell after expression compared to the wild type.

**[0394.0.0.23] to [0399.0.0.23]** for the disclosure of the paragraphs [0394.0.0.23] to [0399.0.0.23] see paragraphs [0394.0.0.0] to [0399.0.0.0] above.

**[00399.1.23.23]** One can think to screen for increased production of the respective fine chemical by for example searching for a resistance to a drug blocking the synthesis of the respective fine chemical and looking whether this effect is dependent on the activity or expression of a polypeptide as indicated in Table II, columns 5 or 7, lines 275 to 277 and/or 635 or 636 or a homolog thereof, e.g. comparing the phenotype of nearly identical organisms with low and high activity of a protein as indicated in Table II, columns 5 or 7, lines 275 to 277 and/or 635 or 636 after incubation with the drug.

**[0400.0.0.23] to [0416.0.0.23]** for the disclosure of the paragraphs [0400.0.0.23] to [0416.0.0.23] see paragraphs [0400.0.0.0] to [0416.0.0.0] above.

**[0417.0.23.23]** The nucleic acid molecule of the invention, the vector of the invention or the nucleic acid construct of the invention may also be useful for the production of organisms resistant to inhibitors of the salicylic acidbiosynthesis pathways. In particular, the overexpression of the polypeptide of the present invention may protect an organism such as a microorganism or a plant against inhibitors, which block the salicylic acidsynthesis.
Furthermore the overexpression of nucleic acids sequences as characterized in Table I, columns 5 and 7, lines 275 to 277 and/or 635 or 636, by increasing the salicylic acid content in plant cells and may also be useful for increasing the nickel (Ni)/zinc (Zn) tolerance in plants.

**[0418.0.0.23] to [0423.0.0.23]** for the disclosure of the paragraphs [0418.0.0.23] to [0423.0.0.23] see paragraphs [0418.0.0.0] to [0423.0.0.0] above.

**[0424.0.23.23]** Accordingly, the nucleic acid of the invention, the polypeptide of the invention, the nucleic acid construct of the invention, the organisms, the host cell, the microorganisms, the plant, plant tissue, plant cell, or the part thereof of the invention, the vector of the invention, the agonist identified with the method of the invention, the nucleic acid molecule identified with the method of the present invention, can be used for the production of the respective fine chemical or of the respective fine chemical and one or more other organic acids. Accordingly, the nucleic acid of the invention, or the nucleic acid molecule identified with the method of the present invention or the complement sequences thereof, the polypeptide of the invention, the nucleic acid construct of the invention, the organisms, the host cell, the microorganisms, the plant, plant tissue, plant cell, or the part thereof of the invention, the vector of the invention, the antagonist identified with the method of the invention, the antibody of the present invention, the antisense molecule of the present invention, can be used for the reduction of the respective fine chemical in a organism or part thereof, e.g. in a cell.

**[0425.0.0.23] to [0435.0.0.23]** for the disclosure of the paragraphs [0425.0.0.23] to [0435.0.0.23] see paragraphs [0425.0.0.0] to [0435.0.0.0] above.

**[0436.0.23.23]** An *in vivo* mutagenesis of organisms such as Saccharomyces, Mortierella, Escherichia and others mentioned above, which are beneficial for the production of salicylic acid can be carried out by passing a plasmid DNA (or another vector DNA) containing the desired nucleic acid sequence or nucleic acid sequences, e.g. the nucleic acid molecule of the invention or the vector of the invention, through E.coli and other microorganisms (for example Bacillus spp. or yeasts such as Saccharomyces cerevisiae) which are not capable of maintaining the integrity of its genetic information. Usual mutator strains have mutations in the genes for the DNA repair system [for example mutHLS, mutD, mutT and the like; for comparison, see Rupp, W.D. (1996) DNA repair mechanisms in Escherichia coli and Salmonella, pp. 2277-2294, ASM: Washington]. The skilled worker knows these strains. The use of these strains is illustrated for example in Greener, A. and Callahan, M. (1994) Strategies 7; 32-34.
In-vitro mutation methods such as increasing the spontaneous mutation rates by chemical or physical treatment are well known to the skilled person. Mutagens like 5-bromo-uracil, N-methyl-N-nitro-N-nitrosoguanidine (= NTG), ethyl methanesulfonate (=EMS), hydroxylamine and/or nitrous acid are widly used as chemical agents for random in-vitro mutagensis. The most common physical method for mutagensis is the treatment with UV irradiation. Another random mutagenesis technique is the error-prone PCR for introducing amino acid changes into proteins. Mutations are deliberately introduced during PCR through the use of error-prone DNA polymerases and special reaction conditions known to a person skilled in the art. For this method randomized DNA sequences are cloned into expression vectors and the resulting mutant libraries screened for altered or improved protein activity as described below.
Site-directed mutagensis method such as the introduction of desired mutations with an M13 or phagemid vector and short oligonucleotides primers is a well-known approach for site-directed mutagensis. The clou of this method involves cloning of the nucleic acid sequence of the invention into an M13 or phagemid vector, which permits recovery of single-stranded recombinant nucleic acid sequence. A mutagenic oligonucleotide primer is then designed whose sequence is perfectly complementary to nucleic acid sequence in the region to be mutated, but with a single difference: at the intended mutation site it bears a base that is complementary to the desired mutant nucleotide rather than the original. The mutagenic oligonucleotide is then allowed to prime new DNA synthesis to create a complementary full-length sequence containing the desired mutation. Another site-directed mutagensis method is the PCR mismatch primer mutagensis method also known to the skilled person. Dpnl site-directed mutagensis is a further known method as described for example in the Stratagene QuickchangeTM site-directed mutagenesis kit protocol. A huge number of other methods are also known and used in common practice.
Positive mutation events can be selected by screening the organisms for the production of the desired respective fine chemical.

**[0437.0.23.23]** Example 4: DNA transfer between Escherichia coli, Saccharomyces cerevisiae and Mortierella alpina

**[0438.0.23.23]** Shuttle vectors such as pYE22m, pPAC-ResQ, pCIasper, pAUR224, pAMH10, pAML10, pAMT10, pAMU10, pGMH10, pGML10, pGMT10, pGMU10, pPGAL1, pPADH1, pTADH1, pTAex3, pNGA142, pHT3101 and derivatives thereof which allow the transfer of nucleic acid sequences between Escherichia coli, Saccharomyces cerevisiae and/or Mortierella alpina are available to the skilled worker. An easy method to isolate such shuttle vectors is disclosed by Soni R. and Murray J.A.H. [Nucleic Acid Research, vol. 20 no. 21, 1992: 5852]: If necessary such shuttle vectors can be constructed easily using standard vectors for E. coli (Sambrook, J. et al., (1989), "Molecular Cloning: A Laboratory Manual", Cold Spring Harbor Laboratory Press or Ausubel, F.M. et al. (1994) "Current Protocols in Molecular Biology", John Wiley & Sons) and/or the aforementioned vectors, which have a replication origin for, and suitable marker from, Escherichia coli, Saccharomyces cerevisiae or Mortierella alpina added. Such replication origins are preferably taken from endogenous plasmids, which have been isolated from species used in the inventive process. Genes, which are used in particular as transformation markers for these species are genes for kanamycin resistance (such as those which originate from the Tn5 or Tn-903 transposon) or for chloramphenicol resistance (Winnacker, E.L. (1987) "From Genes to Clones - Introduction to Gene Technology", VCH, Weinheim) or for other antibiotic resistance genes such as for G418, gentamycin, neomycin, hygromycin or tetracycline resistance.

**[0439.0.23.23]** Using standard methods, it is possible to clone a gene of interest into one of the above-described shuttle vectors and to introduce such hybrid vectors into the microorganism strains used in the inventive process. The transformation of Saccharomyces can be achieved for example by LiCl or sheroplast transformation (Bishop et al., Mol. Cell. Biol., 6, 1986: 3401 - 3409; Sherman et al., Methods in Yeasts in Genetics, [Cold Spring Harbor Lab. Cold Spring Harbor, N. Y.] 1982, Agatep et al., Technical Tips Online 1998, 1:51: P01525 or Gietz et al., Methods Mol. Cell. Biol. 5, 1995: 255f) or electroporation (Delorme E., Appl. Environ. Microbiol., vol. 55, no. 9, 1989 : 2242 - 2246).

**[0440.0.23.23]** If the transformed sequence(s) is/are to be integrated advantageously into the genome of the microorganism used in the inventive process for example into the yeast or fungi genome, standard techniques known to the skilled worker also exist for this purpose. Solinger et al. (Proc Natl Acad Sci U S A., 2001 (15): 8447-8453) and Freedman et al. (Genetics, Vol. 162, 15-27, September 2002,) teaches a homolog recombination system dependent on rad 50, rad51, rad54 and rad59 in yeasts. Vectors using this system for homologous recombination are vectors derived from the Ylp series. Plasmid vectors derived for example from the 2µ-Vector are known by the skilled worker and used for the expression in yeasts. Other preferred vectors are for example pART1, pCHY21 or pEVP11 as they have been described by McLeod et al. (EMBO J. 1987, 6:729-736) and Hoffman et al. (Genes Dev. 5, 1991: :561-571.) or Russell et al. (J. Biol. Chem. 258, 1983: 143-149.). Other beneficial yeast vectors are plasmids of the REP, REP-X, pYZ or RIP series.

**[0441.0.0.23] to [0443.0.0.23]** for the disclosure of the paragraphs [0441.0.0.23] to [0443.0.0.23] see paragraphs [0441.0.0.0] to [0443.0.0.0] above.

**[0444.0.23.23]** Example 6: Growth of genetically modified organism: media and culture conditions

**[0445.0.23.23]** Genetically modified Yeast, Mortierella or Escherichia coli are grown in synthetic or natural growth media known by the skilled worker. A number of different growth media for Yeast, Mortierella or Escherichia coli are well known and widely available. A method for culturing Mortierella is disclosed by Jang et al. [Bot. Bull. Acad. Sin. (2000) 41: 41-48]. Mortierella can be grown at 20°C in a culture medium containing: 10 g/l glucose, 5 g/I yeast extract at pH 6.5. Futhermore Jang et al. teaches a submerged basal medium containing 20 g/l soluble starch, 5 g/I Bacto yeast extract, 10 g/l KNO₃, 1 g/l KH₂PO₄, and 0.5 g/l MgSO₄•7H₂O, pH 6.5.

**[0446.0.0.23] to [0453.0.0.23]** for the disclosure of the paragraphs [0446.0.0.23] to [0453.0.0.23] see paragraphs [0446.0.0.0] to [0453.0.0.0] above.

**[0454.0.23.23]** Example 8: Analysis of the effect of the nucleic acid molecule on the production of salicylic acid

**[0455.0.23.23]** The effect of the genetic modification in plants, fungi, algae or ciliates on the production of a desired compound (such as a salicylic acidand/or salicylic acid esters) can be determined by growing the modified microorganisms or the modified plant under suitable conditions (such as those described above) and analyzing the medium and/or the cellular components for the elevated production of desired product (i.e. of salicylic acid and/or salicylic acid esters). These analytical techniques are known to the skilled worker and comprise spectroscopy, thin-layer chromatography, various types of staining methods, enzymatic and microbiological methods and analytical chromatography such as high-performance liquid chromatography (see, for example, Ullman, Encyclopedia of Industrial Chemistry, Vol. A2, p. 89-90 and p. 443-613, VCH: Weinheim (1985); Fallon, A., et al., (1987) "Applications of HPLC in Biochemistry" in: Laboratory Techniques in Biochemistry and Molecular Biology, Vol. 17; Rehm et al. (1993) Biotechnology, Vol. 3, Chapter III: "Product recovery and purification", p. 469-714, VCH: Weinheim; Belter, P.A., et al. (1988) Bioseparations: downstream processing for Biotechnology, John Wiley and Sons; Kennedy, J.F., and Cabral, J.M.S. (1992) Recovery processes for biological Materials, John Wiley and Sons; Shaeiwitz, J.A., and Henry, J.D. (1988) Biochemical Separations, in: Ullmann's Encyclopedia of Industrial Chemistry, Vol. B3; Chapter 11, p. 1-27, VCH: Weinheim; and Dechow, F.J. (1989) Separation and purification techniques in biotechnology, Noyes Publications).

**[0456.0.0.23]:** for the disclosure of this paragraph see [0456.0.0.0] above.

**[0457.0.23.23]** Example 9: Purification of salicylic acid

**[0458.0.23.23]** Abbreviations; GC-MS, gas liquid chromatography/mass spectrometry; TLC, thin-layer chromatography.
The unambiguous detection for the presence of salicylic acidcan be obtained by analyzing recombinant organisms using analytical standard methods: LC, LC-MSMS, GC-MS or TLC, as described. The total amount produced in the organism for example in yeasts used in the inventive process can be analysed for example according to the following procedure:
The material such as yeasts, E. coli or plants to be analyzed can be disrupted by sonication, grinding in a glass mill, liquid nitrogen and grinding or via other applicable methods.
Plant material is initially homogenized mechanically by comminuting in a pestle and mortar to make it more amenable to extraction.
A typical sample pretreatment consists of a total lipid extraction using such polar organic solvents as acetone or alcohols as methanol, or ethers, saponification, partition between phases, seperation of non-polar epiphase from more polar hypophasic derivatives and chromatography.
For analysis, solvent delivery and aliquot removal can be accomplished with a robotic system comprising a single injector valve Gilson 232XL and a 402 2S1V diluter [Gilson, Inc. USA, 3000 W. Beltline Highway, Middleton, WI]. For saponification, 3 ml of 50% potassium hydroxide hydro-ethanolic solution (4 water - 1 ethanol) can be added to each vial, followed by the addition of 3 ml of octanol. The saponification treatment can be conducted at room temperature with vials maintained on an IKA HS 501 horizontal shaker [Labworld-online, Inc., Wilmington, NC] for fifteen hours at 250 movements/minute, followed by a stationary phase of approximately one hour.
Following saponification, the supernatant can be diluted with 0 -20 ml of methanol. The addition of methanol can be conducted under pressure to ensure sample homogeneity. Using a 0.25 ml syringe, a 0.1 ml aliquot can be removed and transferred to HPLC vials for analysis.
For HPLC analysis, a Hewlett Packard 1100 HPLC, complete with a quaternary pump, vacuum degassing system, six-way injection valve, temperature regulated autosampler, column oven and Photodiode Array detector can be used [Agilent Technologies available through Ultra Scientific Inc., 250 Smith Street, North Kingstown, RI]. The column can be a Waters YMC30, 5-micron, 4.6 x 250 mm with a guard column of the same material [Waters, 34 Maple Street, Milford, MA]. The solvents for the mobile phase can be 81 methanol: 4 water: 15 tetrahydrofuran (THF) stabilized with 0.2% BHT (2,6-di-tert-butyl-4-methylphenol). Injections were 20 µl. Separation can be isocratic at 30°C with a flow rate of 1.7 ml/minute. The peak responses can be measured by absorbance at 447 nm.

**[0459.0.23.23]** If required and desired, further chromatography steps with a suitable resin may follow. Advantageously, the salicylic acidcan be further purified with a so-called RTHPLC. As eluent acetonitrile/water or chloroform/acetonitrile mixtures can be used. If necessary, these chromatography steps may be repeated, using identical or other chromatography resins. The skilled worker is familiar with the selection of suitable chromatography resin and the most effective use for a particular molecule to be purified.

**[0460.0.0.23]** for the disclosure of this paragraph see [0460.0.0.0] above.

**[0461.0.23.23]** Example 10: Cloning SEQ ID NO: 33502, 33810, 33899, 98917 or 99068 for the expression in plants

**[0462.0.0.23]** for the disclosure of this paragraph see [0462.0.0.0] above.

**[0463.0.23.23]** SEQ ID NO: 33502, 33810, 33899, 98917 or 99068 are amplified by PCR as described in the protocol of the Pfu Turbo or DNA Herculase polymerase (Stratagene).

**[0464.0.0.23] to [0466.0.0.23]** for the disclosure of the paragraphs [0464.0.0.23] to [0466.0.0.23] see [0464.0.0.0] to [0466.0.0.0] above.

**[0466.1.0.23]** In case the Herculase enzyme can be used for the amplification, the PCR amplification cycles were as follows: 1 cycle of 2-3 minutes at 94°C, followed by 25-30 cycles of in each case 30 seconds at 94°C, 30 seconds at 55-60°C and 5-10 minutes at 72°C, followed by 1 cycle of 10 minutes at 72°C, then 4°C.

**[0467.0.23.23]** The following primer sequences were selected for the gene SEQ ID : 33502
i) forward primer (SEQ ID NO:33802)
   atgaaaaaaa ccacattagc actg
ii) reverse primer (SEQ ID NO:33803)
   ttactgcatt aacaggtaga tggtg
The following primer sequences were selected for the gene SEQ ID NO: 33810:
i) forward primer (SEQ ID NO:33894)
   atgatcagga gtcacaccat ga
ii) reverse primer (SEQ ID NO:33895)
   ttaattgcca gccatcgcct g
The following primer sequences were selected for the gene SEQ ID NO: 33899:
i) forward primer (SEQ ID NO:34217)
   atgttatcac tgtctgccaa aaatc
ii) reverse primer (SEQ ID NO: 34218)
   tcagtcacgg tattggtcaa aaaat
The following primer sequences were selected for the gene SEQ ID NO: 98917:
i) forward primer (SEQ ID NO: 99063)
   atggaaacga ctcaaaccag cac
ii) reverse primer (SEQ ID NO: 99064)
   ttagctgaac agagagtaga agatt
The following primer sequences were selected for the gene SEQ ID NO: 99068:
i) forward primer (SEQ ID NO: 99168)
   atgagtactt cagatagcat tgtatc
ii) reverse primer (SEQ ID NO: 99169)
   ttaaaacagt ttgtatacga tgttcag

**[0468.0.0.23] to [0470.0.0.23]** for the disclosure of the paragraphs [0468.0.0.23] to [0470.0.0.23] see [0468.0.0.0] to [0470.0.0.0] above.

**[0470.1.23.23]** The PCR-products, produced by *Pfu* Turbo DNA polymerase, were phosphorylated using a T4 DNA polymerase using a standard protocol (e.g. MBI Fermentas) and cloned into the processed binary vector.

**[0471.0.23.23]** for the disclosure of this paragraph see [0471.0.0.0] above.

**[0471.1.23.23]** The DNA termini of the PCR-products, produced by Herculase DNA polymerase, were blunted in a second synthesis reaction using *Pfu* Turbo DNA polymerase. The composition for the protocol of the blunting the DNA-termini was as follows: 0.2 mM blunting dTTP and 1.25 u *Pfu* Turbo DNA polymerase. The reaction was incubated at 72°C for 30 minutes. Then the PCR-products were phosphorylated using a T4 DNA polymerase using a standard protocol (e.g. MBI Fermentas) and cloned into the processed vector as well.

**[0472.0.0.23] to [0479.0.0.23]** for the disclosure of the paragraphs [0472.0.0.23] to [0479.0.0.23] see [0472.0.0.0] to [0479.0.0.0] above.

**[0480.0.23.23]** Example 11: Generation of transgenic plants which express SEQ ID NO: 33502, 33810, 33899, 98917 or 99068.

**[0481.0.0.23] to [0513.0.0.23]** for the disclosure of the paragraphs [0481.0.0.23] to [0513.0.0.23] see paragraphs [0482.0.0.0] to [0513.0.0.0] above.

**[0514.0.23.23]** As an alternative, salicylic acid can be detected as described in Kmetec, V., J. Pharm. Biomed. Anal. 1992 Oct-Dec;10 (10-12):1073-6.
The results of the different plant analyses can be seen from the table 1, which follows:

**Table 1**

| ORF | Metabolite | Method | Min | Max |
|---|---|---|---|---|
| b0161 | Salicylic acid | LC | 1.67 | 5.75 |
| b2664 | Salicylic acid | LC | 1.56 | 5.25 |
| YLR089C | Salicylic acid | LC | 1.45 | 2.53 |
| b2796 | **Salicylic acid** | LC | 1.42 | 3.48 |
| b3116 | Salicylic acid | LC | 1.41 | 2.30 |

**[0515.0.23.23]** Column 2 shows the metabolite salicylic acid analyzed. Columns 4 and 5 shows the ratio of the analyzed metabolite between the transgenic plants and the wild type; Increase of the metabolite: Max: maximal x-fold (normalised to wild type)-Min: minimal x-fold (normalised to wild type). Decrease of the metabolite: Max: maximal x-fold (normalised to wild type) (minimal decrease), Min: minimal x-fold (normalised to wild type) (maximal decrease). Column 3 indicates the analytical method.

**[0516.0.0.23] to [0530.0.0.23]** for the disclosure of the paragraphs [0516.0.0.23] to [0530.0.0.23] see paragraphs [0516.0.0.0] to [0530.0.0.0] above.

**[0530.1.0.23] to [0530.6.0.23]** for the disclosure of the paragraphs [0530.1.0.23] to [0530.6.0.23] see paragraphs [0530.1.0.0] to [0530.6.0.0] above.

**[0531.0.0.23] to [0552.0.0.23]** for the disclosure of the paragraphs [0531.0.0.23] to [0552.0.0.23] see paragraphs [0531.0.0.0] to [0552.0.0.0] above.

**[0552.1.0.23]:** %

**[0552.2.0.23]** for the disclosure of this paragraph see [0552.2.0.0] above.

**[0553.0.23.23]**
1. A process for the production of salicylic acid, which comprises
   (a) increasing or generating the activity of a protein as indicated in Table II, columns 5 or 7, lines 275 to 277 or 635 and 636 or a functional equivalent thereof in a non-human organism or in one or more parts thereof; and
   (b) growing the organism under conditions which permit the production of salicylic acid in said organism.
2. A process for the production of salicylic acid, comprising the increasing or generating in an organism or a part thereof the expression of at least one nucleic acid molecule comprising a nucleic acid molecule selected from the group consisting of:
   a) nucleic acid molecule encoding a polypeptide as indicated in Table II, columns 5 or 7, lines 275 to 277 or 635 and 636 or a fragment thereof, which confers an increase in the amount of salicylic acid in an organism or a part thereof;
   b) nucleic acid molecule comprising of a nucleic acid molecule as indicated in Table I, columns 5 or 7, lines 275 to 277 or 635 and 636;
   c) nucleic acid molecule whose sequence can be deduced from a polypeptide sequence encoded by a nucleic acid molecule of (a) or (b) as a result of the degeneracy of the genetic code and conferring an increase in the amount of salicylic acid in an organism or a part thereof;
   d) nucleic acid molecule which encodes a polypeptide which has at least 50% identity with the amino acid sequence of the polypeptide encoded by the nucleic acid molecule of (a) to (c) and conferring an increase in the amount of salicylic acid in an organism or a part thereof;
   e) nucleic acid molecule which hybidizes with a nucleic acid molecule of (a) to (c) under stringent hybridisation conditions and conferring an increase in the amount of salicylic acid in an organism or a part thereof;
   f) nucleic acid molecule which encompasses a nucleic acid molecule which is obtained by amplifying nucleic acid molecules from a cDNA library or a genomic library using the primers or primer pairs as indicated in Table III, column 7, lines 275 to 277 or 635 and 636 and conferring an increase in the amount of salicylic acid in an organism or a part thereof;
   g) nucleic acid molecule encoding a polypeptide which is isolated with the aid of monoclonal antibodies against a polypeptide encoded by one of the nucleic acid molecules of (a) to (f) and conferring an increase in the amount of salicylic acid in an organism or a part thereof;
   h) nucleic acid molecule encoding a polypeptide comprising a consensus as indicated in Table IV, column 7, lines 275 to 277 or 635 and 636 and conferring an increase in the amount of salicylic acid in an organism or a part thereof; and
   i) nucleic acid molecule which is obtainable by screening a suitable nucleic acid library under stringent hybridization conditions with a probe comprising one of the sequences of the nucleic acid molecule of (a) to (k) or with a fragment thereof having at least 15 nt, preferably 20 nt, 30 nt, 50 nt, 100 nt, 200 nt or 500 nt of the nucleic acid molecule characterized in (a) to (k) and conferring an increase in the amount of salicylic acid in an organism or a part thereof.
   or comprising a sequence which is complementary thereto.
3. The process of claim 1 or 2, comprising recovering of the free or bound salicylic acid.
4. The process of any one of claim 1 to 3, comprising the following steps:
   a) selecting an organism or a part thereof expressing a polypeptide encoded by the nucleic acid molecule characterized in claim 2;
   b) mutagenizing the selected organism or the part thereof;
   c) comparing the activity or the expression level of said polypeptide in the mutagenized organism or the part thereof with the activity or the expression of said polypeptide of the selected organisms or the part thereof;
   d) selecting the mutated organisms or parts thereof, which comprise an increased activity or expression level of said polypeptide compared to the selected organism or the part thereof;
   e) optionally, growing and cultivating the organisms or the parts thereof; and
   f) recovering, and optionally isolating, the free or bound salicylic acid produced by the selected mutated organisms or parts thereof.
5. The process of any one of claims 1 to 4, wherein the activity of said protein or the expression of said nucleic acid molecule is increased or generated transiently or stably.
6. An isolated nucleic acid molecule comprising a nucleic acid molecule selected from the group consisting of:
   a) nucleic acid molecule encoding a polypeptide as indicated in Table II, columns 5 or 7, lines 275 to 277 or 635 and 636 or a fragment thereof, which confers an increase in the amount of salicylic acid in an organism or a part thereof;
   b) nucleic acid molecule comprising of a nucleic acid molecule as indicated in Table I, columns 5 or 7, lines 275 to 277 or 635 and 636 ;
   c) nucleic acid molecule whose sequence can be deduced from a polypeptide sequence encoded by a nucleic acid molecule of (a) or (b) as a result of the degeneracy of the genetic code and conferring an increase in the amount of salicylic acid in an organism or a part thereof;
   d) nucleic acid molecule which encodes a polypeptide which has at least 50% identity with the amino acid sequence of the polypeptide encoded by the nucleic acid molecule of (a) to (c) and conferring an increase in the amount of salicylic acid in an organism or a part thereof;
   e) nucleic acid molecule which hybidizes with a nucleic acid molecule of (a) to (c) under stringent hybridisation conditions and conferring an increase in the amount of salicylic acid in an organism or a part thereof;
   f) nucleic acid molecule which encompasses a nucleic acid molecule which is obtained by amplifying nucleic acid molecules from a cDNA library or a genomic library using the primers or primer pairs as indicated in Table III, column 7, lines 275 to 277 or 635 and 636 and conferring an increase in the amount of salicylic acid in an organism or a part thereof;
   g) nucleic acid molecule encoding a polypeptide which is isolated with the aid of monoclonal antibodies against a polypeptide encoded by one of the nucleic acid molecules of (a) to (f) and conferring an increase in the amount of salicylic acid in an organism or a part thereof;
   h) nucleic acid molecule encoding a polypeptide comprising a consensus as indicated in Table IV, column 7, lines 275 to 277 or 635 and 636 and conferring an increase in the amount of salicylic acid in an organism or a part thereof; and
   i) nucleic acid molecule which is obtainable by screening a suitable nucleic acid library under stringent hybridization conditions with a probe comprising one of the sequences of the nucleic acid molecule of (a) to (k) or with a fragment thereof having at least 15 nt, preferably 20 nt, 30 nt, 50 nt, 100 nt, 200 nt or 500 nt of the nucleic acid molecule characterized in (a) to (k) and conferring an increase in the amount of salicylic acid in an organism or a part thereof.
   whereby the nucleic acid molecule distinguishes over the sequence as indicated in Table IA, columns 5 or 7, lines 275 to 277 or 635 and 636 by one or more nucleotides.
7. A nucleic acid construct which confers the expression of the nucleic acid molecule of claim 6, comprising one or more regulatory elements.
8. A vector comprising the nucleic acid molecule as claimed in claim 6 or the nucleic acid construct of claim 7.
9. The vector as claimed in claim 8, wherein the nucleic acid molecule is in operable linkage with regulatory sequences for the expression in a prokaryotic or eukaryotic, or in a prokaryotic and eukaryotic, host.
10. A host cell, which has been transformed stably or transiently with the vector as claimed in claim 8 or 9 or the nucleic acid molecule as claimed in claim 6 or the nucleic acid construct of claim 7 or produced as described in any one of claims 2 to 5.
11. The host cell of claim 10, which is a transgenic host cell.
12. The host cell of claim 10 or 11, which is a plant cell, an animal cell, a microorganism, or a yeast cell, a fungus cell, a prokaryotic cell, an eukaryotic cell or an archaebacterium.
13. A process for producing a polypeptide, wherein the polypeptide is expressed in a host cell as claimed in any one of claims 10 to 12.
14. A polypeptide produced by the process as claimed in claim 13 or encoded by the nucleic acid molecule as claimed in claim 6 whereby the polypeptide distinguishes over a sequence as indicated in Table IIA, columns 5 or 7, lines 275 to 277 or 635 and 636 by one or more amino acids.
15. An antibody, which binds specifically to the polypeptide as claimed in claim 14.
16. A plant tissue, propagation material, harvested material or a plant comprising the host cell as claimed in claim 12 which is plant cell or an Agrobacterium.
17. A method for screening for agonists and antagonists of the activity of a polypeptide encoded by the nucleic acid molecule of claim 6 conferring an increase in the amount of salicylic acid in an organism or a part thereof comprising:
   (a) contacting cells, tissues, plants or microorganisms which express the a polypeptide encoded by the nucleic acid molecule of claim 6 conferring an increase in the amount of salicylic acid in an organism or a part thereof with a candidate compound or a sample comprising a plurality of compounds under conditions which permit the expression the polypeptide;
   (b) assaying the salicylic acid level or the polypeptide expression level in the cell, tissue, plant or microorganism or the media the cell, tissue, plant or microorganisms is cultured or maintained in; and
   (c) identifying a agonist or antagonist by comparing the measured salicylic acid level or polypeptide expression level with a standard salicylic acid or polypeptide expression level measured in the absence of said candidate compound or a sample comprising said plurality of compounds, whereby an increased level over the standard indicates that the compound or the sample comprising said plurality of compounds is an agonist and a decreased level over the standard indicates that the compound or the sample comprising said plurality of compounds is an antagonist.
18. A process for the identification of a compound conferring increased salicylic acid production in a plant or microorganism, comprising the steps:
   a) culturing a plant cell or tissue or microorganism or maintaining a plant expressing the polypeptide encoded by the nucleic acid molecule of claim 6 conferring an increase in the amount of salicylic acid in an organism or a part thereof and a readout system capable of interacting with the polypeptide under suitable conditions which permit the interaction of the polypeptide with said readout system in the presence of a compound or a sample comprising a plurality of compounds and capable of providing a detectable signal in response to the binding of a compound to said polypeptide under conditions which permit the expression of said readout system and of the polypeptide encoded by the nucleic acid molecule of claim 6 conferring an increase in the amount of salicylic acid in an organism or a part thereof;
   b) identifying if the compound is an effective agonist by detecting the presence or absence or increase of a signal produced by said readout system.
19. A method for the identification of a gene product conferring an increase in salicylic acid production in a cell, comprising the following steps:
   a) contacting the nucleic acid molecules of a sample, which can contain a candidate gene encoding a gene product conferring an increase in salicylic acid after expression with the nucleic acid molecule of claim 6;
   b) identifying the nucleic acid molecules, which hybridise under relaxed stringent conditions with the nucleic acid molecule of claim 6;
   c) introducing the candidate nucleic acid molecules in host cells appropriate for producing salicylic acid;
   d) expressing the identified nucleic acid molecules in the host cells;
   e) assaying the salicylic acid level in the host cells; and
   f) identifying nucleic acid molecule and its gene product which expression confers an increase in the salicylic acid in the host cell in the host cell after expression compared to the wild type.
20. A method for the identification of a gene product conferring an increase in salicylic acid production in a cell, comprising the following steps:
   a) identifying in a data bank nucleic acid molecules of an organism; which can contain a candidate gene encoding a gene product conferring an increase in the salicylic acid amount or level in an organism or a part thereof after expression, and which are at least 20% homolog to the nucleic acid molecule of claim 6;
   b) introducing the candidate nucleic acid molecules in host cells appropriate for producing salicylic acid;
   c) expressing the identified nucleic acid molecules in the host cells;
   d) assaying the salicylic acid level in the host cells; and
   e) identifying nucleic acid molecule and its gene product which expression confers an increase in the salicylic acid level in the host cell after expression compared to the wild type.
21. A method for the production of an agricultural composition comprising the steps of the method of any one of claims 17 to 20 and formulating the compound identified in any one of claims 17 to 20 in a form acceptable for an application in agriculture.
22. A composition comprising the nucleic acid molecule of claim 6, the polypeptide of claim 14, the nucleic acid construct of claim 7, the vector of any one of claims 8 or 9, an antagonist or agonist identified according to claim 17, the compound of claim 18, the gene product of claim 19 or 20, the antibody of claim 15, and optionally an agricultural acceptable carrier.
23. Use of the nucleic acid molecule as claimed in claim 6 for the identification of a nucleic acid molecule conferring an increase of salicylic acid after expression.
24. Use of the polypeptide of claim 14 or the nucleic acid construct claim 7 or the gene product identified according to the method of claim 19 or 20 for identifying compounds capable of conferring a modulation of salicylic acid levels in an organism.
25. Agrochemical, pharmaceutical, food or feed composition comprising the nucleic acid molecule of claim 6, the polypeptide of claim 14, the nucleic acid construct of claim 7, the vector of claim 8 or 9, the antagonist or agonist identified according to claim 17, the antibody of claim 15, the plant or plant tissue of claim 16, the harvested material of claim 16, the host cell of claim 10 to 12 or the gene product identified according to the method of claim 19 or 20.
26. The method of any one of claims 1 to 5, the nucleic acid molecule of claim 6, the polypeptide of claim 14, the nucleic acid construct of claim 7, the vector of claim 8 or 9, the antagonist or agonist identified according to claim 17, the antibody of claim 15, the plant or plant tissue of claim 16, the harvested material of claim 16, the host cell of claim 10 to 12 or the gene product identified according to the method of claim 19 or 20, wherein the fine chemical is salicylic acid.
27. A host cell or plant according to any of the claims 10 to 12 which is resistant to a herbicide inhibiting the biosynthesis of salicylic acid.
28. A host cell or plant according to any of the claims 10 to 12 showing increased resistance to pathogens in comparison to control plants.
29. A host cell or plant according to any of the claims 10 to 12 showing increased resistance to biotic or abiotic stress.
30. A host cell or plant according to any of the claims 10 to 12 showing reduced senescence.
31. Use of the nucleic acid molecule of claim 6, the polypeptide of claim 14, the nucleic acid construct of claim 7, the vector of claim 8 or 9, the antagonist or agonist identified according to claim 17, the antibody of claim 15, the plant or plant tissue of claim 16, the host cell of claim 10 to 12 or the gene product identified according to the method of claim 19 or 20 for the protection of a plant against a salicylic acid synthesis inhibiting herbicide.

**[0554.0.0.23] Abstract: see [0554.0.0.0]**

### Process for the production of fine chemicals

**[0000.0.0.24]** In a further embodiment, the present invention relates to a further process for the production of fine chemicals as defined below and the corresponding embodiments as described herein as follows.

**[0001.0.0.24]** see **[0001.0.0.0]**

**[0002.0.24.24]** to **[0009.0.24.24]** see **[0002.0.24.24] to [0009.0.10.10]**

**[0010.0.24.24]** Therefore improving the quality of foodstuffs and animal feeds is an important task of the food-and-feed industry. This is necessary since, for example, as mentioned above beta-carotene, which occur in plants and some microorganisms are limited with regard to the supply of mammals. Especially advantageous for the quality of foodstuffs and animal feeds is as balanced as possible a carotenoids profile in the diet since a great excess of some carotenoids above a specific concentration in the food has only some positive effect. A further increase in quality is only possible via addition of further carotenoids, which are limiting.

**[0011.0.24.24]**see **[0011.0.10.10]**

**[0012.0.24.24]** Accordingly, there is still a great demand for new and more suitable genes which encode enzymes which participate in the biosynthesis of carotenoids, e.g. beta-carotene or its/their precursor, e.g. isopentyl pyrophosphate (IPP), and make it possible to produce them specifically on an industrial scale without unwanted byproducts forming.
In the selection of genes for biosynthesis two characteristics above all are particularly important. On the one hand, there is as ever a need for improved processes for obtaining the highest possible contents of carotenoids like beta-carotene; on the other hand as less as possible byproducts should be produced in the production process.

**[0013.0.0.24]** see **[0013.0.0.0]**

**[0014.0.24.24]**Accordingly, in a first embodiment, the invention relates to a process for the production of a fine chemical, whereby the fine chemical is a carotene or a precursor thereof. In a preferred embodiment, the fine chemical is beta-carotene or IPP, resp. Accordingly, in the present invention, the term "the fine chemical" as used herein relates to a "carotene, in particular beta-carotene, or its precursor, in particular IPP,. Further, the term "the fine chemicals" as used herein also relates to fine chemicals comprising carotene, in particular beta-carotene, or its precursor, in particular IPP.

**[0015.0.24.24]**In one embodiment, the term "carotene, in particular beta-carotene," or "the fine chemical" or "the respective fine chemical" means at least one chemical compound with a carotene-, in particular beta-carotene-like activity.
In a preferred embodiment, the term "carotene precursor, in particular IPP" means a chemical compound, which expression increases preferably the activity or amount of carotenes, preferably of beta-carotenes, in an organism, alone or in combination with other chemical compounds, e.g. other expression tools, like genes conferring reactions for the conversion of IPP or the production of IPP, e.g. genes being involved in the production or conversion of mevalonic acid, the first specific precursor of terpenoids, or acetyl-CoA via HMG-CoA (3-hydroxy-3-methylglutaryl-CoA), that is itself converted to isopentenyl pyrophosphate (IPP), or geranylgeranyl pyrophosphate (GGPP) molecules which produce colorless phytoene, which is the initial carotenoid, or of the existent alternative, mevalonate-independent pathway for IPP formation that was characterized initially in several species of eubacteria, i.e. in a green alga, and in the plastids of higher plants, and comprising the transketolase-type condensation reaction of pyruvate and D-glyceraldehylde-3-phosphate to yield 1-deoxy-D-xylulose-5-phosphate (DXP), or one of the desaturases, which confer the series of desaturation reactions to convert the conversion product of IPP phytoene to phytofluene, -carotene, neurosporene and finally to lycopene, or the cyclization reaction to β-carotene that contains two β-ionene rings.
In one embodiment, the term "the fine chemical" means a "carotene, in particular beta-carotene, and its precursor, in particular IPP",. In one embodiment, the term "the fine chemical" means beta-carotene or IPP depending on the context in which the term is used. Throughout the specification the term "the fine chemical" includes the free fine chemicals, its salts, ester, thioester or bound to other compounds such sugars or sugarpolymers, like glucoside, e.g. diglucoside. In one embodiment, the term "the fine chemical" means beta-carotene or IPP, resp." in free form or their salts or their ester or bound to a glucoside, e.g a diglucoside, resp.

**[0016.0.24.24]**Accordingly, the present invention relates to a process comprising
(a) increasing or generating the activity of one or more b1829, b2699, YBR089C-A, YDR316W, YDR513W, and/or YLL013C, and/or b0730, b1926, b2211, b3172, b4129, and/or YDR407C and/or b0481, b0970, b1736, b1738 and/or b3160 protein(s) in a non-human organism in one or more parts thereof; and
(b) growing the organism under conditions which permit the production of the fine chemical, thus, carotene, in particular beta-carotene, or its precursor, in particular IPP, resp., in said organism.
Accordingly, the present invention relates to a process comprising
(a) increasing or generating the activity of one or more proteins having the activity of a protein indicated in Table IIA or IIB, column 3, lines 279, 282, 285, 286, 288, and/or 289, or lines 278, 280, 281, 283, 284 and/or 287 and/or lines 637 to 641, resp., or having the sequence of a polypeptide encoded by a nucleic acid molecule indicated in Table IA or IB, column 5 or 7, lines 278 to 289 and/or lines 637 to 641, resp., in a non-human organism in one or more parts thereof; and
(b) growing the organism under conditions which permit the production of the fine chemical, thus, a carotene, in particular beta-carotene, or its precursor, in particular IPP, resp., or fine chemicals comprising carotene, in particular beta-carotene, or its precursor, in particular IPP, resp., in said organism.

**[0016.1.24.24]** Accordingly, in one embodiment the term "the fine chemical" means "beta-carotene" in relation to all sequences listed in the Tables I to IV, lines 279, 282, 285, 286, 288, and/or 289 and/or lines 637 to 641 or homologs thereof and means "IPP" in relation to all sequences listed in Table I to IV, lines 278, 280, 281, 283, 284,and/or 287 and/or lines 637 to 641, or homologs thereof. Accordingly, the term "the fine chemical" can mean "beta-carotene" or "IPP", owing to circumstances and the context. In order to illustrate that the meaning of the term "the respective fine chemical" means "beta-carotene", and/or "IPP" owing to the sequences listed in the context the term "the respective fine chemical" is also used.

**[0017.0.0.24] to [0018.0.0.24]: see [0017.0.0.0] to [0018.0.0.0]**

**[0019.0.24.24]** Advantageously the process for the production of the respective fine chemical leads to an enhanced production of the a carotene, in particular beta-carotene, a precursor thereof, e.g. IPP. The terms "enhanced" or "increase" mean at least a 10%, 20%, 30%, 40% or 50%, preferably at least 60%, 70%, 80%, 90% or 100%, more preferably 150%, 200%, 300%, 400% or 500% higher production of the respective fine chemical in comparison to the reference as defined below, e.g. that means in comparison to an organism without the aforementioned modification of the activity of a protein having the activity of a protein indicated in Table IA or IB , column 3, lines 279, 282, 285, 286, 288, and/or 289, and/or lines 278, 280, 281, 283, 284, and/or 287 and/or lines 637 to 641 or encoded by nucleic acid molecule indicated in Table IA or IB , columns 5 or 7, lines 279, 282, 285, 286, 288, and/or 289, and/or lines 278, 280, 281, 283, 284, and/or 287 and/or lines 637 to 641.

**[0020.0.24.24]** Surprisingly it was found, that the transgenic expression of the Escherichia coli K12 or Saccharomyces cerevisiae proteins b1829, b2699, YBR089C-A, YDR316W, YDR513W, and YLL013C in Arabidopsis thaliana conferred an increase in the beta-carotene ("the fine chemical" or "the fine respective chemical" in respect to said proteins and their homologs as wells as the encoding nucleic acid molecules, in particular as indicated in Table I to IV, column 3, lines 279, 282, 285, 286, 288, and/or 289) content of the transformed plants.
Surprisingly it was found, that the transgenic expression of the Escherichia coli K12 or Saccharomyces cerevisiae proteins b0730, b1926, b2211, b3172, b4129, or YDR407C and/or b0481, b0970, b1736, b1738 and/or b3160 in Arabidopsis thaliana conferred an increase in the IPP ("the fine chemical" or "the fine respective chemical" in respect to said proteins and their homologs as wells as the encoding nucleic acid molecules, in particular as indicated in Table I to IV, column 3, lines 278, 280, 281, 283, 284, or 287 or lines 637 to 641) content of the transformed plants.

**[0021.0.0.24]** see **[0021.0.0.0]**

**[0022.0.24.24]** The sequence of b0730 from Escherichia coli K12 has been published in Blattner et al., Science 277(5331), 1453-1474, 1997, and its activity is being defined as a transcriptional regulator of succinylCoA synthetase operon and a fatty acyl response regulator. Accordingly, in one embodiment, the process of the present invention comprises the use of a protein of the superfamily of transcription regulator GntR, in particular involved in regulation of C-compound and carbohydrate utilization, transcriptional control, prokaryotic nucleoid, transcriptional repressor, DNA binding, preferably being a transcriptional regulator of succinylCoA synthetase operon and fatty acyl response regulator, or its homolog, e.g. as shown herein, for the production of the respective fine chemical, meaning of the IPP, or of carotenes, preferably in free or bound form in an organism or a part thereof, as mentioned. In one embodiment, in the process of the present invention the activity of a transcriptional regulator of succinylCoA synthetase operon and a fatty acyl response regulator, in particular of a lipoprotein of the superfamily of transcription regulator GntR is increased or generated, e.g. from E. coli or a homolog thereof.
The sequence of b1829 from Escherichia coli K12 has been published in Blattner et al., Science 277(5331), 1453-1474, 1997, and its activity is being defined as a heat shock protein with protease acitivity. Accordingly, in one embodiment, the process of the present invention comprises the use of protein of the superfamily of heat-shock protein htpX, in particular involved in stress response, UNCLASSIFIED PROTEINS, pheromone response, mating-type determination, sex-specific proteins, protein modification, proteolytic degradation, preferably a heat shock protein with protease acitivity or its homolog, e.g. as shown herein, for the production of the respective fine chemical, meaning of carotene, in particular beta-carotene, preferably in free or bound form in an organism or a part thereof, as mentioned. In one embodiment, in the process of the present invention said activity, e.g the activity of heat shock protein with protease acitivity is increased or generated, e.g. from E. coli or a homolog thereof.
The sequence of b1926 from Escherichia coli K12 has been published in Blattner et al., Science 277(5331), 1453-1474, 1997, and its activity is being defined as flagellar protein fliT. Accordingly, in one embodiment, the process of the present invention comprises the use of a protein of a flagellar protein fliT from E. coli or its homolog, e.g. as shown herein, for the production of the respective fine chemical, meaning of IPP, or of carotenes, preferably in free or bound form in an organism or a part thereof, as mentioned. In one embodiment, in the process of the present invention said activity, e.g. the activity of a flagellar protein fliT is increased or generated, e.g. from E. coli or a homolog thereof.
The sequence of b2699 from Escherichia coli K12 has been published in Blattner et al., Science 277(5331), 1453-1474, 1997, and its activity is being defined as a DNA strand exchange and recombination protein with protease and nuclease activity of the superfamily of the recombination protein recA. Accordingly, in one embodiment, the process of the present invention comprises the use of a protein of the superfamily of the recombination protein recA, preferably with a DNA recombination and DNA repair activity, a pheromone response activity, a mating-type determination activity, a sex-specific protein activity, a nucleotide binding activity and/or a protease and nuclease activity, in particular a DNA strand exchange and recombination protein with protease and nuclease activity from E. coli or its homolog, e.g. as shown herein, for the production of the respective fine chemical, meaning of carotene, in particular of beta-carotene, preferably in free or bound form in an organism or a part thereof, as mentioned. In one embodiment, in the process of the present invention said activity, e.g. the activity of a protease and nuclease activity, in particular a DNA strand exchange and recombination protein with protease and nuclease activity, in particular of the superfamily of the recombination protein recA is increased or generated, e.g. from E. coli or a homolog thereof.
The sequence of b2211 from Escherichia coli K12 has been published in Blattner et al., Science 277(5331), 1453-1474, 1997, and its activity is being defined as a ATP-binding transport proteins of the ABC superfamily. Accordingly, in one embodiment, the process of the present invention comprises the use of a protein of the superfamily of unassigned ATP-binding cassette proteins, preferably with a nucleotide binding, ABC transporters, CELLULAR TRANSPORT AND TRANSPORT MECHANISMS activity, in particular a ATP-binding transport proteins of the ABC superfamily protein activity from E. coli or its homolog, e.g. as shown herein, for the production of the respective fine chemical, meaning of IPP, or of carotenes, preferably in free or bound form in an organism or a part thereof, as mentioned. In one embodiment, in the process of the present invention said activity, e.g. the activity of a ATP-binding transport proteins of the ABC superfamily is increased or generated, e.g. from E. coli or a homolog thereof.
The sequence of b3172 from Escherichia coli K12 has been published in Blattner et al., Science 277(5331), 1453-1474, 1997, and its activity is being defined as argininosuccinate synthetase. Accordingly, in one embodiment, the process of the present invention comprises the use of a protein of the superfamily of argininosuccinate synthase proteins, preferably involved in amino acid biosynthesis, nitrogen and sulfur metabolism, biosynthesis of the glutamate group (proline, hydroxyprolin, arginine, glutamine, glutamate), degradation of amino acids of the glutamate group, nitrogen and sulfur utilization, urea cycle, biosynthesis of polyamines and creatine, biosynthesis of the aspartate family, assimilation of ammonia, biosynthesis of the glutamate group, preferably having a argininosuccinate synthetase activity from E. coli or its homolog, e.g. as shown herein, for the production of the respective fine chemical, meaning of IPP, or of carotenes, preferably in free or bound form in an organism or a part thereof, as mentioned. In one embodiment, in the process of the present invention said activity, e.g. the activity of a argininosuccinate synthetase is increased or generated, e.g. from E. coli or a homolog thereof.
The sequence of b4129 from Escherichia coli K12 has been published in Blattner et al., Science 277(5331), 1453-1474, 1997, and its activity is being defined as inducible lysine tRNA synthetase. Accordingly, in one embodiment, the process of the present invention comprises the use of a protein of the superfamily of lysine-tRNA ligase proteins, preferably involved in aminoacyl-tRNA-synthetases, translation, preferably having a inducible lysine tRNA synthetase activity from E. coli or its homolog, e.g. as shown herein, for the production of the respective fine chemical, meaning of I PP, or of carotenes, preferably in free or bound form in an organism or a part thereof, as mentioned. In one embodiment, in the process of the present invention said activity, e.g. the activity of a inducible lysine tRNA synthetase is increased or generated, e.g. from E. coli or a homolog thereof.
The sequence of YBR089C-A from Saccharomyces cerevisiae has been published in Jacq et al., Nature 387 (6632 Suppl), 75-78, 1997, and Goffeau et al., Science 274 (5287), 546-547, 1996, and its activity is being defined as nonhistone chromosomal protein. Accordingly, in one embodiment, the process of the present invention comprises the use of a protein of the superfamily of nonhistone chromosomal protein HMG-2, HMG box homology, unassigned HMG box proteins, preferably being involved in transcriptional control, nucleic acid binding, TRANSCRIPTION, preferably a nonhistone chromosomal protein or its homolog, e.g. as shown herein, for the production of the respective fine chemical, meaning of carotene, in particular beta-carotene, in particular for increasing the amount in free or bound form in an organism or a part thereof, as mentioned. In one embodiment, in the process of the present invention said activity, e.g. the activity of a nonhistone chromosomal protein.is increased or generated, e.g. from Saccharomyces cerevisiae or a homolog thereof. The sequence of YDR316W from Saccharomyces cerevisiae has been published in Jacq et al., Nature 387 (6632 Suppl), 75-78, 1997, and Goffeau et al., Science 274 (5287), 546-547, 1996, and its activity is being defined as putative S-adenosylmethionine-dependent methyltransferase of the seven beta-strand family. Accordingly, in one embodiment, the process of the present invention comprises the use of a protein of the superfamily of bioC homology, preferably ofa putative S-adenosylmethionine-dependent methyltransferase of the seven beta-strand family or its homolog, e.g. as shown herein, for the production of the respective fine chemical, meaning of carotene, in particular beta-carotene, in particular for increasing the amount in free or bound form in an organism or a part thereof, as mentioned. In one embodiment, in the process of the present invention said activity, e.g. the activity of a putative S-adenosylmethionine-dependent methyltransferase of the seven beta-strand family is increased or generated, e.g. from Saccharomyces cerevisiae or a homolog thereof.
The sequence of YDR407C from Saccharomyces cerevisiae has been published in Jacq et al., Nature 387 (6632 Suppl), 75-78, 1997, and Goffeau et al., Science 274 (5287), 546-547, 1996, and its activity is being defined as targeting complex (TRAPP) component involved in ER to Golgi membrane traffic. Accordingly, in one embodiment, the process of the present invention comprises the use of a protein involved in the vesicular transport (Golgi network, etc.), preferably being involved in targeting complex (TRAPP) component involved in ER to Golgi membrane traffic, e.g. as shown herein, for the production of the respective fine chemical, meaning of IPP, or of carotenes, in particular for increasing the amount in free or bound form in an organism or a part thereof, as mentioned. In one embodiment, in the process of the present invention said activity, e.g. the activity of a protein.of the vesicular transport (Golgi network, etc.) is increased or generated, e.g. from Saccharomyces cerevisiae or a homolog thereof. The sequence of YDR513W from Saccharomyces cerevisiae has been published in Jacq et al., Nature 387 (6632 Suppl), 75-78, 1997, and Goffeau et al., Science 274 (5287), 546-547, 1996, and its activity is being defined as glutathione reductase. Accordingly, in one embodiment, the process of the present invention comprises the use of a protein of the superfamily of glutaredoxin, in particular being involved in deoxyribonucleotide metabolism, cytoplasm, stress response, detoxification, electron transport and membrane-associated energy conservation, preferably a glutathione reductase, or its homolog, e.g. as shown herein, for the production of the respective fine chemical, meaning of carotene, in particular beta-carotene, in particular for increasing the amount in free or bound form in an organism or a part thereof, as mentioned. In one embodiment, in the process of the present invention said activity, e.g. the activity of a glutathione reductase is increased or generated, e.g. from Saccharomyces cerevisiae or a homolog thereof.
The sequence of YLL013C from Saccharomyces cerevisiae has been published in Jacq et al., Nature 387 (6632 Suppl), 75-78, 1997, and Goffeau et al., Science 274 (5287), 546-547, 1996, and its activity is being defined as a member of the PUF protein family, which is named for the founding members, pumilio and Fbf. Accordingly, in one embodiment, the process of the present invention comprises the use of a protein being involved in cell differentiation, development (Systemic), nucleic acid binding, transcriptional control, other transcription activities, preferably a a member of the PUF protein family, which is named for the founding members, pumilio and Fbf, or its homolog, e.g. as shown herein, for the production of the respective fine chemical, meaning of carotene, in particular beta-carotene, in particular for increasing the amount in free or bound form in an organism or a part thereof, as mentioned. In one embodiment, in the process of the present invention said activity, e.g. the activity of a a member of the PUF protein family, which is named for the founding members, pumilio and Fbf is increased or generated, e.g. from Saccharomyces cerevisiae or a homolog thereof.
The sequence of b0481 from Escherichia coli K12 has been published in Blattner et al., Science 277(5331), 1453-1474, 1997, and its protein is being defined as a protein with a YbaK-like domain. Accordingly, in one embodiment, the process of the present invention comprises the use of a protein with a YbaK-like domain from E. coli or its homolog, e.g. as shown herein, for the production of the respective fine chemical, meaning of IPP, preferably in free or bound form in an organism or a part thereof, as mentioned. In one embodiment, in the process of the present invention said protein with a YbaK-like domain is increased or generated, e.g. from E. coli or a homolog thereof.The sequence of b0970 from Escherichia coli K12 has been published in Blattner et al., Science 277 (5331), 1453-1474, 1997, and its activity is being defined as a glutamate receptor. Accordingly, in one embodiment, the process of the present invention comprises the use of a gene product with an activity of Escherichia coli ybhL protein superfamily, preferably a protein with the activity of a glutamate receptor protein from E. coli or its homolog, e.g. as shown herein, for the production of the fine chemical, meaning of glutamine and/or isopentenylpyrophosphate, in particular for increasing the amount of glutamine and/or isopentenylpyrophosphate, preferably glutamine and/or isopentenylpyrophosphate in free or bound form in an organism or a part thereof, as mentioned.
The sequence of b1736 from Escherichia coli K12 has been published in Blattner et al., Science 277(5331), 1453-1474, 1997, and its protein is being defined as a protein with PEP-dependent phosphotransferase activity. Accordingly, in one embodiment, the process of the present invention comprises the use of a protein with PEP-dependent phosphotransferase activity from E. coli or its homolog, e.g. as shown herein, for the production of the respective fine chemical, meaning of IPP, preferably in free or bound form in an organism or a part thereof, as mentioned. In one embodiment, in the process of the present invention the activity of the PEP-dependent phosphotransferase is increased or generated, e.g. from E. coli or a homolog thereof.
The sequence of b1738 from Escherichia coli K12 has been published in Blattner et al., Science 277(5331), 1453-1474, 1997, and its protein is being defined as a protein with PEP-dependent phosphotransferase activity. Accordingly, in one embodiment, the process of the present invention comprises the use of a protein with PEP-dependent phosphotransferase activity from E. coli or its homolog, e.g. as shown herein, for the production of the respective fine chemical, meaning of IPP, preferably in free or bound form in an organism or a part thereof, as mentioned. In one embodiment, in the process of the present invention the activity of a PEP-dependent phosphotransferase is increased or generated, e.g. from E. coli or a homolog thereof.
The sequence of b3160 from Escherichia coli K12 has been published in Blattner et al., Science 277(5331), 1453-1474, 1997, and its protein is being defined as a monooxygenase protein with luciferase-like ATPase activity. Accordingly, in one embodiment, the process of the present invention comprises the use of a monooxygenase protein with luciferase-like ATPase activity from E. coli or its homolog, e.g. as shown herein, for the production of the respective fine chemical, meaning of IPP, preferably in free or bound form in an organism or a part thereof, as mentioned. In one embodiment, in the process of the present invention the activity of a monooxygenase protein with luciferase-like ATPase activity is increased or generated, e.g. from E. coli or a homolog thereof.

**[0023.0.24.24]** Homologues ( = homologs) of the present gene products can be derived from any organisms as long as the homologue confers the herein mentioned activity, in particular, confers an increase in the respective fine chemical amount or content.
In one embodiment, the homolog of any one of the polypeptides indicated in Table IIA or IIB, column 5 or 7, line 287 is a homolog of a polypeptide indicated in Table IIA or IIB, column 3, line 287 having the same or a similar activity. In particular an increase of activity confers an increase in the content of the respective fine chemical in the organisms preferably IPP, and in a other preferred embodiment, also carotenes, in particular beta-carotene. In one embodiment, the homologue is a homolog with a sequence as indicated in Table I or II, column 7, line 287, resp.. In one embodiment, the homologue of one of the polypeptides indicated in Table IIA or IIB, column 3, line 287 is derived from an eukaryotic. In one embodiment, the homologue is derived from Fungi. In one embodiment, the homologue of a polypeptide indicated in Table IIA or IIB, column 3, line 287 is derived from Ascomyceta. In one embodiment, the homologue of a polypeptide indicated in Table IIA or IIB, column 3, line 287 is derived from Saccharomycotina. In one embodiment, the homologue of a polypeptide indicated in Table IIA or IIB, column 3, line 287 is derived from Saccharomycetes. In one embodiment, the homolog of a polypeptide indicated in Table IIA or IIB, column 3, line 287 is a homologue being derived from Saccharomycetales. In one embodiment, the homologue of a polypeptide indicated in Table IIA or IIB, column 3, line 287 is a homologue having the same or a similar acitivity being derived from Saccharomycetaceae. In one embodiment, the homologue of a polypeptide indicated in Table IIA or IIB, column 3, line 287 is a homologue having the same or a similar activity being derived from Saccharomycetes.
In one embodiment, the homolog of the any one of the polypeptides indicated in Table IIA or IIB, column 5 or 7, line 278, 280, 281, 283, 284 or lines 637 to 641 is a homolog of the polypeptide indicated in Table IIA or IIB, column 3, line 278, 280, 281, 283, 284 or lines 637 to 641, resp., having the same or a similar acitivity. In particular an increase of activity confers an increase in the content of the respective fine chemical in the organisms preferably IPP, more preferably also carotenes, in particular beta-carotene. In one embodiment, the homolog is a homolog with a sequence as indicated in Table I or II, column 7, line 278, 280, 281, 283, 284 or lines 637 to 641, resp.. In one embodiment, the homolog of one of the polypeptides indicated in Table IIA or IIB, column 3, line 278, 280, 281, 283, 284 or lines 637 to 641 is derived from an bacteria.. In one embodiment, the homolog of a polypeptide indicated in Table IIA or IIB, column 3, line 278, 280, 281, 283, 284 or lines 637 to 641 is derived from Proteobacteria. In one embodiment, the homolog of a polypeptide indicated in Table IIA or IIB, column 3, line 278, 280, 281, 283, 284 or lines 637 to 641 is a homolog having the same or a similar acitivity being derived from Gammaproteobacteria. In one embodiment, the homolog of a polypeptide indicated in Table IIA or IIB, column 3, line 278, 280, 281, 283, 284 or lines 637 to 641 is derived from Enterobacteriales. In one embodiment, the homolog of a polypeptide indicated in Table IIA or IIB, column 3, line 278, 280, 281, 283, 284 or lines 637 to 641 is a homolog being derived from Enterobacteriaceae. In one embodiment, the homolog of a polypeptide indicated in Table IIA or IIB, column 3, line 278, 280, 281, 283, 284 or lines 637 to 641 is a homolog having the same or a similar activity, in particular an increase of activity confers an increase in the content of the IPP in the organisms or part thereof being derived from Escherichia.
In one embodiment, the homolog of the any one of the polypeptides indicated in Table IIA or IIB, column 5 or 7, line 285, 286, 288 or 289 is a homolog of the polypeptide indicated in Table IIA or IIB, column 3, line 285, 286, 288, or 289, resp., having the same or a similar activity. In particular an increase of activity confers an increase in the content of the respective fine chemical in the organisms, preferably beta-carotene. In one embodiment, the homolog is a homolog with a sequence as indicated in Table I or II, column 7, line 285, 286, 288 or 289, resp.. In one embodiment, the homolog of one of the polypeptides indicated in Table IIA or IIB, column 3, line 285, 286, 288 or 289 is derived from an eukaryotic. In one embodiment, the homolog is derived from Fungi. In one embodiment, the homolog of a polypeptide indicated in Table IIA or IIB, column 3, line 285, 286, 288 or 289 is derived from Ascomyceta. In one embodiment, the homolog of a polypeptide indicated in Table IIA or IIB, column 3, line 285, 286, 288 or 289 is derived from Saccharomycotina. In one embodiment, the homolog of a polypeptide indicated in Table IIA or IIB, column 3, lines line 285, 286, 288 or 289is derived from Saccharomycetes. In one embodiment, the homolog of a polypeptide indicated in Table IIA or IIB, column 3, line 285, 286, 288 or 289is a homolog being derived from Saccharomycetales. In one embodiment, the homolog of a polypeptide indicated in Table IIA or IIB, column 3, line 285, 286, 288 or 289 is a homolog having the same or a similar activity being derived from Saccharomycetaceae. In one embodiment, the homolog of a polypeptide indicated in Table IIA or IIB, column 3, line 285, 286, 288 or 289 is a homolog having the same or a similar acitivity being derived from Saccharomycetes.
In one embodiment, the homolog of the any one of the polypeptides indicated in Table IIA or IIB, column 5 or 7, line 279 or 282 is a homolog of the polypeptide indicated in Table IIA or IIB, column 3, line 279 or 282, resp having the same or a similar activity. In particular an increase of activity confers an increase in the content of the respective fine chemical in the organisms, preferably beta-carotene. In one embodiment, the homolog is a homolog with a sequence as indicated in Table I or II, column 7, line 279 or 282, resp.. In one embodiment, the homolog of one of the polypeptides indicated in Table IIA or IIB, column 3, line 279 or 282is derived from an bacteria.. In one embodiment, the homolog of a polypeptide indicated in Table IIA or IIB, column 3, line 279 or 282 is derived from Proteobacteria. In one embodiment, the homolog of a polypeptide indicated in Table IIA or IIB, column 3, line 279 or 282 is a homolog having the same or a similar activity being derived from Gammaproteobacteria. In one embodiment, the homolog of a polypeptide indicated in Table IIA or IIB, column 3, line 279 or 282 is derived from Enterobacteriales. In one embodiment, the homolog of a polypeptide indicated in Table IIA or IIB, column 3, line 279 or 282 is a homolog being derived from Enterobacteriaceae. In one embodiment, the homolog of a polypeptide indicated in Table IIA or IIB, column 3, line 279 or 282 is a homolog having the same or a similar activity, in particular an increase of activity confers an increase in the content of the zeaxanthin in the organsims or part thereof being derived from Escherichia.

**[0023.1.24.24]**Homologs of the polypeptides indicated in Table IIA or IIB, column 3, lines 279, 282, 285, 286, 288, and/or 289, and/or lines 278, 280, 281, 283, 284, and/or 287 and/or lines 637 to 641, may be the polypeptides encoded by the nucleic acid molecules indicated in Table IA or IB , column 7, lines 279, 282, 285, 286, 288, and/or 289, and/or lines 278, 280, 281, 283, 284, and/or 287 and/or lines 637 to 641, or may be the polypeptides indicated in Table IIA or IIB, column 7, lines 279, 282, 285, 286, 288 and/or 289, and/or lines 278, 280, 281, 283, 284 and/or 287 and/or lines 637 to 641.
Homologs of the polypeptides indicated in Table IIA or IIB, column 3, lines 279, 282, 285, 286, 288, and/or 289, may be the polypeptides encoded by the nucleic acid molecules indicated in Table IA or IB , column 7, lines 279, 282, 285, 286, 288, and/or 289, respectively or may be the polypeptides indicated in Table IIA or IIB, column 7, lines 279, 282, 285, 286, 288 and/or 289, having a beta-carotene-content and/or - amount-increasing activity. Homologs of the polypeptides indicated in Table IIA or IIB, column 3, lines 279, 282, 285, 286, 288 and/or 289 may be the polypeptides encoded by the nucleic acid molecules indicated in Table IA or IB , column 7, lines 279, 282, 285, 286, 288 and/or 289 or may be the polypeptides indicated in Table IIA or IIB, column 7, lines 279, 282, 285, 286, 288 and/or 289 having a beta-carotene-content and/or -amount-increasing activity.
Homologs of the polypeptides indicated in Table IIA or IIB, column 3, lines 278, 280, 281, 283, 284 and/or 287 and/or lines 637 to 641 may be the polypeptides encoded by the nucleic acid molecules indicated in Table IA or IB , column 7, lines 278, 280, 281, 283, 284, and/or 287 and/or lines 637 to 641, respectively or may be the polypeptides indicated in Table IIA or IIB, column 7, lines 278, 280, 281, 283, 284 and/or 287 and/or lines 637 to 641, having a IPP-content and/or -amount-increasing activity. Homologs of the polypeptides indicated in Table IIA or IIB, column 3, lines 278, 280, 281, 283, 284 and/or 287 and/or lines 637 to 641 may be the polypeptides encoded by the nucleic acid molecules indicated in Table IA or IB , column 7, lines 278, 280, 281, 283, 284 and/or 287 and/or lines 637 to 641 or may be the polypeptides indicated in Table IIA or IIB, column 7, lines 278, 280, 281, 283, 284, and/or 287 and/or lines 637 to 641 having a IPP content- and/or amount-increasing activity.

**[0024.0.0.24]** see **[0024.0.0.0]**

**[0025.0.24.24]** In accordance with the invention, a protein or polypeptide has the "activity of an protein of the invention", e.g. the activity of a protein indicated in Table IIA or IIB, column 3, lines 279, 282, 285, 286, 288 and/or 289 if its *de novo* activity, or its increased expression directly or indirectly leads to an increased beta-carotene level in the organism or a part thereof, preferably in a cell of said organism. In a preferred embodiment, the protein or polypeptide has the above-mentioned additional activities of a protein indicated in Table IIA or IIB, column 3, lines 279, 282, 285, 286, 288, and/or 289,.. Throughout the specification the activity or preferably the biological activity of such a protein or polypeptide or an nucleic acid molecule or sequence encoding such protein or polypeptide is identical or similar if it still has the biological or enzymatic activity of any one of the proteins indicated in Table IIA or IIB, column 3, lines 279, 282, 285, 286, 288, and/or 289, or which has at least 10% of the original enzymatic activity, preferably 20%, particularly preferably 30%, most particularly preferably 40% in comparison to any one of the proteins indicated in Table IIA or IIB, column 3, lines 285, 286, 288 or 289 of Saccharomyces cerevisiae and/or any one of the proteins indicated in Table IIA or IIB, column 3, lines 279 or 282 of E. coli K12.
In accordance with the invention, a protein or polypeptide has the "activity of an protein of the invention", e.g. the activity of a protein indicated in Table IIA or IIB, column 3, lines lines 278, 280, 281, 283, 284 and/or 287 and/or lines 637 to 641 if its *de novo* activity, or its increased expression directly or indirectly leads to an increased IPP level in the organism or a part thereof, preferably in a cell of said organism. In a preferred embodiment, the protein or polypeptide has the above-mentioned additional activities of a protein indicated in Table IIA or IIB, column 3, lines 278, 280, 281, 283, 284 and/or 287 and/or lines 637 to 641. Throughout the specification the activity or preferably the biological activity of such a protein or polypeptide or an nucleic acid molecule or sequence encoding such protein or polypeptide is identical or similar if it still has the biological or enzymatic activity of any one of the proteins indicated in Table IIA or IIB, column 3, lines 278, 280, 281, 283, 284 and/or 287 and/or lines 637 to 641, or which has at least 10% of the original enzymatic activity, preferably 20%, particularly preferably 30%, most particularly preferably 40% in comparison to any one of the proteins indicated in Table IIA or IIB, column 3, line 287 of Saccharomyces cerevisiae and/or any one of the proteins indicated in Table IIA or IIB, column 3, lines lines 278, 280, 281, 283 and/or 284 and/or lines 637 to 641 of E. coli K12.
In one embodiment, the polypeptide of the invention confers said activity, e.g. the increase of the respective fine chemical in an organism or a part thereof, if it is derived from an organism, which is evolutionary close to the organism indicated in Table I, column 4 and is expressed in an organism, which is evolutionary distant to the origin organism. For example origin and expressing organism are derived from different families, orders, classes or phylums whereas origin and the organism indicated in Table I, column 4 are derived from the same families, orders, classes or phylums.

**[0025.1.0.24] see [0025.1.0.0]**

**[0026.0.0.24]** to **[0033.0.0.24]: see [0026.0.0.0]** to **[0033.0.0.0]**

**[0034.0.24.24]** Preferably, the reference, control or wild type differs form the subject of the present invention only in the cellular activity of the polypeptide of the invention, e.g. as result of an increase in the level of the nucleic acid molecule of the present invention or an increase of the specific activity of the polypeptide of the invention. E.g., it differs by or in the expression level or activity of an protein having the activity of a protein as indicated in Table IIA or IIB, column 3, lines 279, 282, 285, 286, 288, and/or 289, and/or lines 278, 280, 281, 283, 284, and/or 287 and/or lines 637 to 641, or being encoded by a nucleic acid molecule indicated in Table IA or IB , column 5, lines 279, 282, 285, 286, 288, and/or 289, and/or lines 278, 280, 281, 283, 284 and/or 287 and/or lines 637 to 641, or its homologs, e.g. as indicated in Table IA or IB , column 7, lines279, 282, 285, 286, 288, and/or 289, and/or lines 278, 280, 281, 283, 284, and/or 287 and/or lines 637 to 641, its biochemical or genetic causes. It therefore shows the increased amount of the respective fine chemical.

**[0035.0.0.24]** to **[0038.0.0.24]: see [0035.0.0.0]** to **[0038.0.0.0]**

**[0039.0.0.24]** see **[0039.0.0.0]**

**[0040.0.0.24] to [0044.0.0.24]: see [0040.0.0.0] to [0044.0.0.0]**

**[0045.0.24.24]** In case the activity of the Escherichia coli K12 protein b0730 or its homologs e.g. a transcriptional regulator of succinylCoA synthetase operon and fatty acyl response regulator, e.g. as indicated in Table IIA or IIB, columns 5 or 7, 278, is increased, preferably, in one embodiment the increase of the respective fine chemical, preferably of IPP between 41 % and 115% or more is conferred.
In one embodiment, in case the activity of the Escherichia coli K12 protein b1829 or its homologs e.g. a heat shock protein with protease acitivity, e.g. as indicated in Table IIA or IIB, columns 5 or 7, line 279, is increased, preferably, in one embodiment the increase of the respective fine chemical, preferably of beta-carotene, between 33% and 74% or more is conferred.
In one embodiment, in case the activity of the Escherichia coli K12 protein b1926 or its homologs e.g. flagellar protein fliT, e.g. as indicated in Table IIA or IIB, columns 5 or 7, line 280, is increased, preferably, in one embodiment the increase of the respective fine chemical, preferably of IPP, between 37% and 55% or more is conferred.
In one embodiment, in case the activity of the Escherichia coli K12 protein b2211 or its homologs e.g. ATP-binding transport proteins of the ABC superfamily, e.g. as indicated in Table IIA or IIB, columns 5 or 7, line 281, is increased, preferably, in one embodiment the increase of the respective fine chemical, preferably of IPP, between 46% and 48% or more is conferred.
In one embodiment, in case the activity of the Escherichia coli K12 protein b2699 or its homologs e.g. a DNA recombination and DNA repair activity, a pheromone response activity, a mating-type determination activity, a sex-specific protein activity, a nucleotide binding activity and/or a protease and nuclease activity, in particular a DNA strand exchange and recombination protein with protease and nuclease activity, in particular of the superfamily of the recombination protein recA, e.g. as indicated in Table IIA or IIB, columns 5 or 7, line 282, is increased, preferably, in one embodiment the increase of the respective fine chemical, preferably of beta-carotene, between 43% and 76% or more is conferred.
In one embodiment, in case the activity of the Escherichia coli K12 protein b3172 or its homologs e.g. argininosuccinate synthetase, e.g. as indicated in Table IIA or IIB, columns 5 or 7, line 283, is increased, preferably, in one embodiment the increase of the respective fine chemical, preferably of IPP, between 35% and 74% or more is conferred.
In one embodiment, in case the activity of the Escherichia coli K12 protein b4129 or its homologs e.g. an inducible lysine tRNA synthetase, e.g. as indicated in Table IIA or IIB, columns 5 or 7, line 284, is increased, preferably, in one embodiment the increase of the respective fine chemical, preferably of IPP, between 34% and 63% or more is conferred.
In one embodiment, in case the activity of the Saccharomyces cerevisiae protein YBR089C-A or its homologs, e.g. a nonhistone chromosomal protein, e.g. as indicated in Table IIA or IIB, columns 5 or 7, line 285, is increased, preferably, in one embodiment an increase of the respective fine chemical, preferably of beta-carotene between 52% and 108% or more is conferred.
In one embodiment, in case the activity of the Saccharomyces cerevisiae protein YDR316W or its homologs, e.g. a putative S-adenosylmethionine-dependent methyltransferase of the seven beta-strand family, e.g. as indicated in Table IIA or IIB, columns 5 or 7, line 286, is increased, preferably, in one embodiment an increase of the respective fine chemical, preferably of beta-carotene between 28% and 52% or more is conferred.
In one embodiment, in case the activity of the Saccharomyces cerevisiae protein YDR407C or its homologs, e.g. a targeting complex (TRAPP) component involved in ER to Golgi membrane traffi or its homolog, e.g. as indicated in Table IIA or IIB, columns 5 or 7, line 287, is increased, preferably, in one embodiment an increase of the respective fine chemical, preferably of IPP between 54% and 214% or more is conferred.
In one embodiment, in case the activity of the Saccharomyces cerevisiae protein YDR513W or its homologs, e.g. a glutathione reductase, e.g. as indicated in Table IIA or IIB, columns 5 or 7, line 288, is increased, preferably, in one embodiment an increase of the respective fine chemical, preferably of beta-carotene between 39% and 43% or more is conferred.
In one embodiment, in case the activity of the Saccharomyces cerevisiae protein YLL013C or its homologs, e.g. a member of the PUF protein family, which is named for the founding members, pumilio and Fbf, e.g. as indicated in Table IIA or IIB, columns 5 or 7, line 289, is increased, preferably, in one embodiment an increase of the respective fine chemical, preferably of beta-carotene between 43% and 50% or more is conferred.
In one embodiment, in case the activity of the Escherichia coli K12 protein b0481 or its homologs e.g. a protein with a YbaK-like domain, e.g. as indicated in Table IIA or IIB, columns 5 or 7, line 637, is increased, preferably, in one embodiment the increase of the respective fine chemical, preferably of IPP, between 35% and 39% or more is conferred.
In one embodiment, in case the activity of the Escherichia coli K12 protein b0970 or its homologs e.g. a glutamate receptor, e.g. as indicated in Table IIA or IIB, columns 5 or 7, line 638, is increased, preferably, in one embodiment the increase of the respective fine chemical, preferably of IPP, between 36% and 117% or more is conferred.
In one embodiment, in case the activity of the Escherichia coli K12 protein b1736 or its homologs e.g. a PEP-dependent phosphotransferase, e.g. as indicated in Table IIA or IIB, columns 5 or 7, line 639, is increased, preferably, in one embodiment the increase of the respective fine chemical, preferably of IPP, between 51 % and 137% or more is conferred.
In one embodiment, in case the activity of the Escherichia coli K12 protein b1738 or its homologs e.g a PEP-dependent phosphotransferase, e.g. as indicated in Table IIA or IIB, columns 5 or 7, line 640, is increased, preferably, in one embodiment the increase of the respective fine chemical, preferably of IPP, between 63% and 160% or more is conferred..
In one embodiment, in case the activity of the Escherichia coli K12 protein b3160 or its homologs e.g.a monooxygenase with luciferase-like ATPase activity, e.g. as indicated in Table IIA or IIB, columns 5 or 7, line 641, is increased, preferably, in one embodiment the increase of the respective fine chemical, preferably of IPP, between 35% and 47% or more is conferred.

**[0046.0.24.24]** In one embodiment, if the activity of the Escherichia coli K12 protein b0730 or its homologs e.g. transcriptional regulator of succinylCoA synthetase operon and fatty acyl response regulator, e.g. as indicated in Table IIA or IIB, columns 5 or 7, line 278, or of b1829 or its homologs, e.g. heat shock protein with protease activity, e.g. as indicated in Table IIA or IIB, columns 5 or 7, line 279, or of b1926 or its homologs, e.g. flagellar protein fliT, e.g. as indicated in Table IIA or IIB, columns 5 or 7, line 280, or of b2211 or its homologs, e.g. ATP-binding transport proteins of the ABC superfamily, e.g. as indicated in Table IIA or IIB, columns 5 or 7, line 281, or of b2699 or its homologs, e.g. DNA strand exchange and recombination protein with protease and nuclease activity, e.g. as indicated in Table IIA or IIB, columns 5 or 7, line 282, or of b3172 or its homologs, e.g. argininosuccinate synthetase, e.g. as indicated in Table IIA or IIB, columns 5 or 7, line 283, or of b4129 or its homologs, e.g. an inducible lysine tRNA synthetase, e.g. as indicated in Table IIA or IIB, columns 5 or 7, line 284, is increased; preferably an increase of the respective fine chemical and of further carotenoids, preferably carotenes, is conferred.
In one embodiment, in case the activity of the Saccharomyces cerevisiae protein YBR089C-A or its homologs, e.g. a nonhistone chromosomal protein, e.g. as indicated in Table IIA or IIB, columns 5 or 7, line 285 or YDR316W or its homologs, e.g. a Putative S-adenosylmethionine-dependent methyltransferase of the seven beta-strand family, e.g. as indicated in Table IIA or IIB, columns 5 or 7, line 286 or YDR407C or its homologs, e.g. a targeting complex (TRAPP) component involved in ER to Golgi membrane traffic, e.g. as indicated in Table IIA or IIB, columns 5 or 7, line 287 or YDR513W or its homologs, e.g. a glutathione reductase, e.g. as indicated in Table IIA or IIB, columns 5 or 7, line 288 or YLL013C or its homologs, e.g. a member of the PUF protein family, which is named for the founding members, pumilio and Fbf, e.g. as indicated in Table IIA or IIB, columns 5 or 7, line 289, e.g. as indicated in Table IIA or IIB, columns 5 or 7, line 637 or b0481 or its homologs, e.g. a protein with a YbaK-like domain, e.g. as indicated in Table IIA or IIB, columns 5 or 7, line 638 or b0970 or its homologs, e.g. a glutamate receptor, e.g. as indicated in Table IIA or IIB, columns 5 or 7, line 639 or b1736 or its homologs, e.g. a PEP-dependent phosphotransferase, e.g. as indicated in Table IIA or IIB, columns 5 or 7, line 640 or b1738 or its homologs, e.g. a PEP-dependent phosphotransferase, e.g. as indicated in Table IIA or IIB, columns 5 or 7, line 641 or b3160 or its homologs, e.g. a monooxygenase with luciferase-like ATPase activity is increased, preferably an increase of the respective fine chemical and of further carotenoids, preferably carotenes, is conferred.

**[0047.0.0.24]** to **[0048.0.0.24]: see [0047.0.0.0]** to **[0048.0.0.0]**

**[0049.0.24.24]** A protein having an activity conferring an increase in the amount or level of the respective fine chemical preferably has the structure of the polypeptide described herein. In a particular embodiment, the polypeptides used in the process of the present invention or the polypeptide of the present invention comprises the sequence of a consensus sequence as indicated in Table IV, columns 5 or 7, lines lines 279, 282, 285, 286, 288 and/or 289, or of a polypeptide as indicated in Table IA or IB , columns 5 or 7, lines 279, 282, 285, 286, 288, and/or 289 or of a functional homologue thereof as described herein, or of a polypeptide encoded by the nucleic acid molecule characterized herein or the nucleic acid molecule according to the invention, for example by a nucleic acid molecule as indicated in Table IA or IB , columns 5 or 7, lines 279, 282, 285, 286, 288, and/or 289 or its herein described functional homologues and has the herein mentioned activity conferring an increase in the beta-carotene level.
A protein having an activity conferring an increase in the amount or level of the respective fine chemical preferably has the structure of the polypeptide described herein. In a particular embodiment, the polypeptides used in the process of the present invention or the polypeptide of the present invention comprises the sequence of a consensus sequence as indicated in Table IV, columns 5 or 7, lines 278, 280, 281, 283, 284 and/or 287 and/or 637 to 641, or of a polypeptide as indicated in Table IIA or IIB, columns 5 or 7 lines 278, 280, 281, 283, 284 and/or 287 and/or 637 to 641 or of a functional homologue thereof as described herein, or of a polypeptide encoded by the nucleic acid molecule characterized herein or the nucleic acid molecule according to the invention, for example by a nucleic acid molecule as indicated in Table IA or IB , columns 5 or 7, lines 278, 280, 281, 283, 284 and/or 287 and/or 637 to 641 or its herein described functional homologues and has the herein mentioned activity confering an increase in the IPP level.

**[0050.0.24.24]** For the purposes of the present invention, the term "the respective fine chemical" also encompass the corresponding salts, such as, for example, the potassium or sodium salts of beta-carotene or IPP, resp., or their ester, or glucoside thereof, e.g the diglucoside thereof.

**[0051.0.24.24]** Owing to the biological activity of the proteins which are used in the process according to the invention and which are encoded by nucleic acid molecules according to the invention, it is possible to produce compositions comprising the respective fine chemical, i.e. an increased amount of the free chemical free or bound, e.g compositions comprising IPP or beta-carotene. Depending on the choice of the organism used for the process according to the present invention, for example a microorganism or a plant, compositions or mixtures of various IPP or beta-carotenecan be produced.

**[0052.0.0.24]** see **[0052.0.0.0]**

**[0053.0.24.24]**In one embodiment, the process of the present invention comprises one or more of the following steps
a) stabilizing a protein conferring the increased expression of a protein encoded by the nucleic acid molecule of the invention or of the polypeptide of the invention, e.g. of a polypeptide having an activity of a protein as indicated in Table IIA or IIB, column 3, lines 279, 282, 285, 286, 288, and/or 289, and/or lines 278, 280, 281, 283, 284, and/or 287 and/or 637 to 641, or its homologs, e.g. as indicated in Table IIA or IIB, columns 5 or 7, lines 279, 282, 285, 286, 288, and/or 289, and/or lines 278, 280, 281, 283, 284, and/or 287 and/or 637 to 641, activity having herein-mentioned the respective fine chemical increasing activity;
b) stabilizing a mRNA conferring the increased expression of a protein encoded by the nucleic acid molecule of the invention, e.g. of a polypeptide having an activity of a protein as indicated in Table IIA or IIB, column 3, lines 279, 282, 285, 286, 288, and/or 289 and/or 637 to 641, and/or lines 278, 280, 281, 283, 284, and/or 287, or its homologs activity, e.g. as indicated in Table IIA or IIB, columns 5 or 7, lines 279, 282, 285, 286, 288, and/or 289 and/or 637 to 641, and/or lines 278, 280, 281, 283, 284, and/or 287, or of a mRNA encoding the polypeptide of the present invention having herein-mentioned the respective fine chemical increasing activity;
c) increasing the specific activity of a protein conferring the increased expression of a protein encoded by the nucleic acid molecule of the invention or of the polypeptide of the present invention having herein-mentioned the respective fine chemical increasing activity, e.g. of a polypeptide having an activity of a protein as indicated in Table IIA or IIB, column 3, lines 279, 282, 285, 286, 288, and/or 289 and/or 637 to 641, and/or lines 278, 280, 281, 283, 284, and/or 287, or its homologs activity, e.g. as indicated in Table IIA or IIB, columns 5 or 7, lines 279, 282, 285, 286, 288, and/or 289 and/or 637 to 641, and/or lines 278, 280, 281, 283, 284, and/or 287, or decreasing the inhibitory regulation of the polypeptide of the invention;
d) generating or increasing the expression of an endogenous or artificial transcription factor mediating the expression of a protein conferring the increased expression of a protein encoded by the nucleic acid molecule of the invention or of the polypeptide of the invention having herein-mentioned the respective fine chemical increasing activity, e.g. of a polypeptide having an activity of a protein as indicated in Table IIA or IIB, column 3, lines 279, 282, 285, 286, 288, and/or 289 and/or 637 to 641, and/or lines 278, 280, 281, 283, 284, and/or 287, or its homologs activity, e.g. as indicated in Table IIA or IIB, columns 5 or 7, lines 279, 282, 285, 286, 288, and/or 289 and/or 637 to 641, and/or lines 278, 280, 281, 283, 284, and/or 287;
e) stimulating activity of a protein conferring the increased expression of a protein encoded by the nucleic acid molecule of the present invention or a polypeptide of the present invention having herein-mentioned the respective fine chemical increasing activity, e.g. of a polypeptide having an activity of a protein as indicated in Table IIA or IIB, column 3, lines 279, 282, 285, 286, 288, and/or 289 and/or 637 to 641, and/or lines 278, 280, 281, 283, 284, and/or 287 or its homologs activity, e.g. as indicated in Table IIA or IIB, columns 5 or 7, lines 279, 282, 285, 286, 288, and/or 289 and/or 637 to 641, and/or lines 278, 280, 281, 283, 284, and/or 287, by adding one or more exogenous inducing factors to the organism or parts thereof;
f) expressing a transgenic gene encoding a protein conferring the increased expression of a polypeptide encoded by the nucleic acid molecule of the present invention or a polypeptide of the present invention, having herein-mentioned the respective fine chemical increasing activity, e.g. of a polypeptide having an activity of a protein as indicated in Table IIA or IIB, column 3, lines 279, 282, 285, 286, 288, and/or 289 and/or 637 to 641, and/or lines 278, 280, 281, 283, 284, and/or 287 or its homologs activity, e.g. as indicated in Table IIA or IIB, columns 5 or 7, lines 279, 282, 285, 286, 288, and/or 289 and/or 637 to 641, and/or lines 278, 280, 281, 283, 284, and/or 287, and/or
g) increasing the copy number of a gene conferring the increased expression of a nucleic acid molecule encoding a polypeptide encoded by the nucleic acid molecule of the invention or the polypeptide of the invention having herein-mentioned the respective fine chemical increasing activity, e.g. of a polypeptide having an activity of a protein as indicated in Table IIA or IIB, column 3, lines 279, 282, 285, 286, 288, and/or 289 and/or 637 to 641, and/or lines 278, 280, 281, 283, 284, and/or 287 or its homologs, e.g. as indicated in Table IIA or IIB, columns 5 or 7, lines 279, 282, 285, 286, 288, and/or 289 and/or 637 to 641, and/or lines 278, 280, 281, 283, 284, and/or 287, activity.
h) Increasing the expression of the endogenous gene encoding the polypeptide of the invention, e.g. a polypeptide having an activity of a protein as indicated in Table IIA or IIB, column 3, lines 279, 282, 285, 286, 288, and/or 289 and/or 637 to 641, and/or lines 278, 280, 281, 283, 284, and/or 287 or its homologs activity, e.g. as indicated in Table IIA or IIB, columns 5 or 7, lines 279, 282, 285, 286, 288, and/or 289 and/or 637 to 641 and/or lines 278, 280, 281, 283, 284, and/or 287, by adding positive expression or removing negative expression elements, e.g. homologous recombination can be used to either introduce positive regulatory elements like for plants the 35S enhancer into the promoter or to remove repressor elements form regulatory regions. Further gene conversion methods can be used to disrupt repressor elements or to enhance to activity of positive elements. Positive elements can be randomly introduced in plants by T-DNA or transposon mutagenesis and lines can be identified in which the positive elements have be integrated near to a gene of the invention, the expression of which is thereby enhanced;
   and/or
i) Modulating growth conditions of an organism in such a manner, that the expression or activity of the gene encoding the protein of the invention or the protein itself is enhanced for example microorganisms or plants can be grown for example under a higher temperature regime leading to an enhanced expression of heat shock proteins, which can lead an enhanced respective fine chemical production.
j) selecting of organisms with especially high activity of the proteins of the invention from natural or from mutagenized resources and breeding them into the target organisms, e.g. the elite crops.

**[0054.0.24.24]** Preferably, said mRNA is the nucleic acid molecule of the present invention and/or the protein conferring the increased expression of a protein encoded by the nucleic acid molecule of the present invention or the polypeptide having the herein mentioned activity is the polypeptide of the present invention, e.g. conferring the increase of the respective fine chemical after increasing the expression or activity of the encoded polypeptide or having the activity of a polypeptide having an activity of a protein as indicated in Table IIA or IIB, columns 5 or 7, lines 279, 282, 285, 286, 288, and/or 289, and/or lines 278, 280, 281, 283, 284, and/or 287 and/or lines 637 to 641, resp., or its homologs activity, e.g. as indicated in Table IIA or IIB, columns 5 or 7, lines 279, 282, 285, 286, 288, and/or 289, and/or lines 278, 280, 281, 283, 284, and/or 287 and/or lines 637 to 641 resp..

**[0055.0.0.24] to [0064.0.0.24]: see [0055.0.0.0] to [0064.0.0.0]**

**[0065.0.0.24]:** see **[0065.0.0.0]**

**[0066.0.0.24] to [0067.0.0.24]: see [0066.0.0.0] to [0067.0.0.0]**

**[0068.0.24.24]** The mutation is introduced in such a way that the production of the respective fine chemical, in particular beta-carotene or IPP is not adversely affected.

**[0069.0.0.24] see [0069.0.0.0]**

**[0070.0.24.24]** Owing to the introduction of a gene or a plurality of genes conferring the expression of the nucleic acid molecule of the invention or the polypeptide of the invention, for example the nucleic acid construct mentioned below, or encoding a protein of the invention into an organism alone or in combination with other genes, it is possible not only to increase the biosynthetic flux towards the end product, but also to increase, modify or create *de novo* an advantageous, preferably novel metabolites composition in the organism, e.g. an advantageous composition of carotenoids, in particular carotenes, in particular beta-carotene, e.g. comprising a higher content of (from a viewpoint of nutrional physiology limited) carotenoids, in particular carotene, in particular beta-carotene or precursors thereof, e.g. IPP.

**[0071.0.0.24]** see **[0071.0.0.0]**

**[0072.0.24.24]** By influencing the metabolism thus, it is possible to produce, in the process according to the invention, further advantageous compounds. Examples of such compounds are further carotenoids, e.g. carotenes or carotene, e.g. ketocarentoids, or hydrocarotenoids, e.g. lutein, lycopene, alpha-carotene, or beta-carentene, or other compounds for which IPP is a precursor well known for the skilled.

**[0073.0.24.24]** Accordingly, in one embodiment, the process according to the invention relates to a process, which comprises:
(c) providing a non-human organism, preferably a microorganism, a non-human animal, a plant or animal cell, a plant or animal tissue or a plant;
(d) increasing an activity of a polypeptide of the invention or a homolog thereof, e.g. as indicated in Table IIA or IIB, columns 5 or 7, lines 279, 282, 285, 286, 288, and/or 289, and/or lines 278, 280, 281, 283, 284, and/or 287 and/or lines 637 to 641 or of a polypeptide being encoded by the nucleic acid molecule of the present invention and described below, e.g. conferring an increase of the respective fine chemical in an organism, preferably in a microorganism, a non-human animal, a plant or animal cell, a plant or animal tissue or a plant,
(e) growing an organism, preferably a microorganism, a non-human animal, a plant or animal cell, a plant or animal tissue or a plant under conditions which permit the production of the respective fine chemical in the organism, preferably the microorganism, the plant cell, the plant tissue or the plant; and
(f) if desired, recovering, optionally isolating, the free and/or bound the respective fine chemical and, optionally further free and/or bound carotenoids, in particular carotene, in particular beta-carotene or its precursor IPP, synthesized by the organism, the microorganism, the non-human animal, the plant or animal cell, the plant or animal tissue or the plant.

**[0074.0.24.24]** The organism, in particular the microorganism, non-human animal, the plant or animal cell, the plant or animal tissue or the plant is advantageously grown in such a way that it is not only possible to recover, if desired isolate the free or bound the respective fine chemical or the free and bound the respective fine chemical but as option it is also possible to produce, recover and, if desired isolate, other free or/and bound carotenoids, in particular carotene, in particular beta-carotene or the carotene precursor IPP.

**[0075.0.0.24]** to **[0077.0.0.24]: see [0075.0.0.0]** to **[0077.0.0.0]**

**[0078.0.24.24]** The organism such as microorganisms or plants or the recovered, and if desired isolated, the respective fine chemical can then be processed further directly into foodstuffs or animal feeds or for other applications, for example according to the disclosures made in 6,399,115: Method and composition for the treatment of benign prostate hypertrophy (BPH) and prevention of prostate cancer
6,399,114: Nutritional system for nervous system disorders
6,399,060: Composition having nematicidal activity
6,399,046: Use of a content of catechins or a content of green tea extract in cosmetic preparations for tanning the skin
6,395,782: Method of increasing longevity and preventing body weight wasting in autoimmune disease by using conjugated linoleic acid
6,395,508: Peptide mixture and products thereof
6,395,315: Fermentation composition, process for preparing the same, and use thereof 6,395,311: Multicomponent biological vehicle
6,394,230: Sterol esters as food additives
6,391,640: Methods and compositions for cellular and metabolic engineering 6,391,332: Therapeutic micronutrient composition for severe trauma, burns and critical illness
6,391,321: Emulsifier-free finely disperse systems of the oil-in-water and water-in-oil type 6,391,319: Cosmetic and dermatological emulsions comprising alkyl glucosides and increased electrolyte concentrations
6,391,289: Use of sunscreen combinations comprising, as essential constituent, 4,4'-diarylbutadienes as photostable UV filters in cosmetic and pharmaceutical preparations 6,387,961: Alkyl 2-acetamido-2-deoxyglucopyranoside and methods of emulsifying 6,387,927: Epothilone derivatives and their use as antitumor agents
6,387,883: Method for the prevention and treatment of cachexia and anorexia 6,387,878: Methods of treating intestinal ischemia using heparin-binding epidermal growth factor
6,387,862: Bleach compositions
6,387,418: Pomegranate extracts and methods of using thereof 6,387,370: Compositions containing extracts of Morinda citrifolia, red wine, prune, blueberry, pomegranate, apple and enzyme mixture
6,387,355: Use of sunscreen combinations comprising, as essential constituent, amino-substituted hydroxybenzophenones as photostable UV filters in cosmetic and pharmaceutical preparations
6,384,090: Preparation of active ingredient dispersions and apparatus therefor
6,384,085: Material separated from Ecklonia cava, method for extracting and purifying the same, and use thereof as antioxidants
6,383,751: Assessing lipid metabolism
6,383,543: Process for the extraction of an organic salt from plants, the salt, and other similar salts
6,383,524: Compositions and methods for enhancing therapeutic effects 6,383,523: Pharmaceutical compositions and methods for managing skin conditions
6,383,503: PREPARATIONS OF THE W/O EMULSION TYPE WITH AN INCREASED WATER CONTENT, ADDITIONALLY COMPRISING ONE OR MORE ALKYLMETHICONE COPOLYOLS AND/OR ALKYLDIMETHICONE COPOLYOLS, AND, IF DESIRED, CATIONIC POLYMERS
6,383,474: Carotenoid preparation
6,383,473: Solid composition for reducing tooth erosion
6,380,442: Process for the isolation of mixed carotenoids from plants
6,380,232: Benzimidazole urea derivatives, and pharmaceutical compositions and unit dosages thereof
6,380,227: Fermentative preparation process for and crystal forms of cytostatics 6,379,697: Stabilization of photosensitive materials
6,379,683: Nanocapsules based on dendritic polymers
6,376,722: Lutein to zeaxanthin isomerization process and product
6,376,717: Preparation of astaxanthin
6,376,544: Nutritional product for a person having renal failure
6,376,498: Pharmaceutical, cosmetic and/or food composition with antioxidant properties
6,376,455: Quaternary ammonium compounds, compositions containing them, and uses thereof
6,376,005: Antimicrobial composition for food and beverage products
6,375,993: Pomegranate extracts and methods of using thereof
6,375,992: Methods of hydrating mammalian skin comprising oral administration of a defined composition
6,375,963: Bioadhesive hot-melt extruded film for topical and mucosal adhesion applications and drug delivery and process for preparation thereof
6,375,956: Strip pack
6,375,873: Process and apparatus for producing stably fine-particle powders 6,372,964: For higher basidiomycetes mushrooms grown as biomass in submerged culture
6,372,946: Preparation of 4,4'-diketo-.beta.-carotene derivatives
The cited literature describe some preferred embodiments. Said applications describe some advantageous embodiments without meant to be limiting.
The fermentation broth, fermentation products, plants or plant products can be purified as described in above mentioned applications and other methods known to the person skilled in the art, e.g. as described in Methods in Enzymology: Carotenoids, Part A: Chemistry, Separation, Quantitation and Antioxidation, by John N Abelson or Part B, Metabolism, Genetics, and Biochemistry, or described herein below. Products of these different work-up procedures are IPP and/or beta-carotenes and optional other carotenoids, comprising compositions which still comprise fermentation broth, plant particles and cell components in different amounts, advantageously in the range of from 0 to 99% by weight, preferably below 80% by weight, especially preferably between below 50% by weight.

**[0079.0.0.24]** to **[0084.0.0.24]: see [0079.0.0.0]** to **[0084.0.0.0]**

**[0084.2.24.24]** The invention also contemplates embodiments in which the β-carotene, or other carotenoid precursor compounds in the production of the respective fine chemical like IPP, is present in the flowers of the flowering plant chosen as the host (for example, marigolds). The invention also contemplates embodiments in which a host plant's flowers lack β-carotene or other carotenoid precursors, such as IPP. In a plant of the latter type, the inserted DNA includes genes that code for carotenoid precursors (compounds that can be converted biologically into β-carotene) and a ketolase, as well as a hydroxylase, if otherwise absent.
In one embodiment, preferred flowering plants include, but are not limited to: Amaryllidaceae (Allium, Narcissus); Apocynaceae (Catharanthus); Asteraceae, alternatively Compositae (Aster, Calendula, Callistephus, Cichorium, Coreopsis, Dahlia, Dendranthema, Gazania, Gerbera, Helianthus, Helichrysum, Lactuca, Rudbeckia, Tagetes, Zinnia); Balsaminaceae (Impatiens); Begoniaceae (Begonia); Caryophyllaceae (Dianthus); Chenopodiaceae (Beta, Spinacia); Cucurbitaceae (Citrullus, Curcurbita, Cucumis); Cruciferae (Alyssum, Brassica, Erysimum, Matthiola, Raphanus); Gentinaceae (Eustoma); Geraniaceae (Pelargonium); Graminae, alternatively Poaceae, (Avena, Horedum, Oryza, Panicum, Pennisetum, Poa, Saccharum, Secale, Sorghum, Triticum, Zea); Euphorbiaceae (Poinsettia); Labiatae (Salvia); Leguminosae (Glycine, Lathyrus, Medicago, Phaseolus, Pisum); Liliaceae (Lilium); Lobeliaceae (Lobelia); Malvaceae (Abelmoschus, Gossypium, Malva); Plumbaginaceae (Limonium); Polemoniaceae (Phlox); Primulaceae (Cyclamen); Ranunculaceae (Aconitum, Anemone, Aquilegia, Caltha, Delphinium, Ranunculus); Rosaceae (Rosa); Rubiaceae (Pentas); Scrophulariaceae (Angelonia, Antirrhinum, Torenia); Solanaceae (Capsicum, Lycopersicon, Nicotiana, Petunia, Solanum); Umbelliferae (Apium, Daucus, Pastinaca); Verbenaceae (Verbena, Lantana); Violaceae (Viola).

**[0085.0.24.24]** With regard to the nucleic acid sequence as depicted a nucleic acid construct which contains a nucleic acid sequence mentioned herein or an organism (= transgenic organism) which is transformed with said nucleic acid sequence or said nucleic acid construct, "transgene" means all those constructs which have been brought about by genetic manipulation methods, preferably in which either
a) a nucleic acid sequence as indicated in Table IA or IB, columns 5 or 7, lines 279, 282, 285, 286, 288, and/or 289, and/or lines 278, 280, 281, 283, 284, and/or 287 and/or lines 637 to 641, or a derivative thereof, or
b) a genetic regulatory element, for example a promoter, which is functionally linked to the nucleic acid sequence as indicated in Table IA or IB , columns 5 or 7, lines 279, 282, 285, 286, 288, and/or 289, and/or lines 278, 280, 281, 283, 284, and/or 287 and/or lines 637 to 641, or a derivative thereof, or
c) (a) and (b)
is/are not present in its/their natural genetic environment or has/have been modified by means of genetic manipulation methods, it being possible for the modification to be, by way of example, a substitution, addition, deletion, inversion or insertion of one or more nucleotide. "Natural genetic environment" means the natural chromosomal locus in the organism of origin or the presence in a genomic library. In the case of a genomic library, the natural, genetic environment of the nucleic acid sequence is preferably at least partially still preserved. The environment flanks the nucleic acid sequence at least on one side and has a sequence length of at least 50 bp, preferably at least 500 bp, particularly preferably at least 1000 bp, very particularly preferably at least 5000 bp.

**[0086.0.0.24]** to **[0087.0.0.24]: see [0086.0.0.0]** to **[0087.0.0.0]**

**[0088.0.24.24]** In an advantageous embodiment of the invention, the organism takes the form of a plant whose content of the respective fine chemical is modified advantageously owing to the nucleic acid molecule of the present invention expressed. This is important for plant breeders since, for example, the nutritional value of plants for poultry is dependent on the abovementioned carotene, in particular beta-carotene and the general amount of xanthohylls as energy source in feed. Further, this is also important for the production of cosmetic compostions since, for example, the antioxidant level of plant extracts is dependent on the amount of th abovementioned carotene, in particular beta-carotene and/or the amount of carotenoids as antioxidants.

**[0088.1.0.24]** see **[0088.1.0.0]**

**[0089.0.0.24]** to **[0090.0.0.24]: see [0089.0.0.0]** to **[0090.0.0.0]**

**[0091.0.24.24]**Thus, the content of plant components and preferably also further impurities is as low as possible, and the abovementioned carotene, in particular beta-carotene, in particular the fine chemical, is/are obtained in as pure form as possible. In these applications, the content of plant components advantageously amounts to less than 10%, preferably 1%, more preferably 0.1 %, very especially preferably 0.01 % or less.

**[0092.0.0.24]** to **[0094.0.0.24]: see [0092.0.0.0]** to **[0094.0.0.0]**

**[0095.0.24.24]** It may be advantageous to increase the pool of said carotenoids, in particular carotene, in particular beta-carotene in the transgenic organisms by the process according to the invention in order to isolate high amounts of the pure respective fine chemical.

**[0096.0.24.24]** In another preferred embodiment of the invention a combination of the increased expression of the nucleic acid sequence or the protein of the invention together with the transformation of a protein or polypeptide or a compound, which functions as a sink for the desired fine chemical, for example zeaxanthin or cryptoxanthin in the organism, is useful to increase the production of the respective fine chemical. It has been reported, that the inhibition of Lyopene production increases the amount of other carotene, in particular beta-carotene in the cell. Further it may be, that the inhibition of enzymes using zeaxanthin or cryptoxanthin as substrate increases the amount of said chemicals in a cell. For example, in one embodiment, it can be advantageous to inhibit the production of astaxanthin, if a high amount of cryptoxanthin or zeaxanthin is desired.

**[0097.0.24.24]** Glucosides, in particular, dicglucosides of the zeaxanthin and beta-cryptoxanthin as well as other modification of zeaxanthin and cryptoxanthin are known to a person skilled in the art. In may also be advantageous to increase the content of the bound respective fine chemical, e.g. of modification of zeaxanthin and cryptoxanthin, in particular its glucosides, e.g. diglucosides.

**[0098.0.24.24]**In a preferred embodiment, the respective fine chemical is produced in accordance with the invention and, if desired, is isolated. The production of further carotenoids, e.g. carotenes or carotene, in particular beta-carotene, in particular ketocarentoids or hydrocarotenoids, e.g. lutein, lycopene, alpha-carotene, or beta-carentene, or compounds for which the respective fine chemical is a biosynthesis precursor compounds, e.g. astaxanthin, or mixtures thereof or mixtures of other carotenoids, in particular of carotene, in particular beta-carotene, by the process according to the invention is advantageous.

**[0099.0.24.24]** In the case of the fermentation of microorganisms, the abovementioned desired fine chemical may accumulate in the medium and/or the cells. If microorganisms are used in the process according to the invention, the fermentation broth can be processed after the cultivation. Depending on the requirement, all or some of the biomass can be removed from the fermentation broth by separation methods such as, for example, centrifugation, filtration, decanting or a combination of these methods, or else the biomass can be left in the fermentation broth. The fermentation broth can subsequently be reduced, or concentrated, with the aid of known methods such as, for example, rotary evaporator, thin-layer evaporator, falling film evaporator, by reverse osmosis or by nanofiltration. Afterwards advantageously further compounds for formulation can be added such as corn starch or silicates. This concentrated fermentation broth advantageously together with compounds for the formulation can subsequently be processed by lyophilization, spray drying, and spray granulation or by other methods. Preferably the respective fine chemical comprising compositions are isolated from the organisms, such as the microorganisms or plants or the culture medium in or on which the organisms have been grown, or from the organism and the culture medium, in the known manner, for example via extraction, distillation, crystallization, chromatography or a combination of these methods. These purification methods can be used alone or in combination with the aforementioned methods such as the separation and/or concentration methods.

**[0100.0.24.24]** Transgenic plants which comprise the carotenoids such as said carotene, in particular beta-carotene, e.g. cryptoxanthin or zeaxanthin (or astaxanthin as it is synthesized from cryptoxanthin or zeaxanthin) synthesized in the process according to the invention can advantageously be marketed directly without there being any need for the carotenoids synthesized to be isolated. Plants for the process according to the invention are listed as meaning intact plants and all plant parts, plant organs or plant parts such as leaf, stem, seeds, root, tubers, anthers, fibers, root hairs, stalks, embryos, calli, cotelydons, petioles, harvested material, plant tissue, reproductive tissue and cell cultures which are derived from the actual transgenic plant and/or can be used for bringing about the transgenic plant. In this context, the seed comprises all parts of the seed such as the seed coats, epidermal cells, seed cells, endosperm or embryonic tissue.
However, the respective fine chemical produced in the process according to the invention can also be isolated from the organisms, advantageously plants, (in the form of their oils, fats, lipids, as extracts, e.g. ether, alcohol, or other organic solvents or water containing extract and/or free carotene, in particular beta-carotene. The respective fine chemical produced by this process can be obtained by harvesting the organisms, either from the crop in which they grow, or from the field. This can be done via pressing or extraction of the plant parts. To increase the efficiency of extraction it is beneficial to clean, to temper and if necessary to hull and to flake the plant material..E.g., oils, fats, and/or lipids comprising carotene, in particular beta-carotene can be obtained by what is known as cold beating or cold pressing without applying heat. To allow for greater ease of disruption of the plant parts, specifically the seeds, they can previously be comminuted, steamed or roasted. Seeds, which have been pretreated in this manner can subsequently be pressed or extracted with solvents such as warm hexane. The solvent is subsequently removed. In the case of microorganisms, the latter are, after harvesting, for example extracted directly without further processing steps or else, after disruption, extracted via various methods with which the skilled worker is familiar. Thereafter, the resulting products can be processed further, i.e. degummed and/or refined. In this process, substances such as the plant mucilages and suspended matter can be first removed. What is known as desliming can be affected enzymatically or, for example, chemico-physically by addition of acid such as phosphoric acid.
Because carotenoids in microorganisms are localized intracellular, their recovery essentials comes down to the isolation of the biomass. Well-established approaches for the harvesting of cells include filtration, centrifugation and coagulation/flocculation as described herein. Of the residual hydrocarbon, adsorbed on the cells, has to be removed. Solvent extraction or treatment with surfactants have been suggested for this purpose. However, it can be advantageous to avoid this treatment as it can result in cells devoid of most carotenoids.

**[0101.0.24.24]** The identity and purity of the compound(s) isolated can be determined by prior-art techniques. They encompass high-performance liquid chromatography (HPLC), gas chromatography (GC), spectroscopic methods, mass spectrometry (MS), staining methods, thin-layer chromatography, NIRS, enzyme assays or microbiological assays. These analytical methods are compiled in: Patek et al. (1994) Appl. Environ. Microbiol. 60:133-140; Malakhova et al. (1996) Biotekhnologiya 11 27-32; and Schmidt et al. (1998) Bioprocess Engineer. 19:67-70. Ulmann's Encyclopedia of Industrial Chemistry (1996) Bd. A27, VCH Weinheim, pp. 89-90, pp. 521-540, pp. 540-547, pp. 559-566, 575-581 and pp. 581-587; Michal, G (1999) Biochemical Pathways: An Atlas of Biochemistry and Molecular Biology, John Wiley and Sons; Fallon, A. et al. (1987) Applications of HPLC in Biochemistry in: Laboratory Techniques in Biochemistry and Molecular Biology, vol. 17.

**[0102.0.24.24]** Carotene, in particular beta-carotene, in particular beta-cryptoxanthin or zeaxanthin can for example be detected advantageously via HPLC, LC or GC separation methods. The unambiguous detection for the presence of carotene, in particular beta-carotene, in particular beta-cryptoxanthin or zeaxanthin containing products can be obtained by analyzing recombinant organisms using analytical standard methods: LC, LC-MS, MS or TLC). The material to be analyzed can be disrupted by sonication, grinding in a glass mill, liquid nitrogen and grinding, cooking, or via other applicable methods.

**[0103.0.24.24]** In a preferred embodiment, the present invention relates to a process for the production of the respective fine chemical comprising or generating in an organism or a part thereof the expression of at least one nucleic acid molecule comprising a nucleic acid molecule selected from the group consisting of:
a) nucleic acid molecule encoding, preferably at least the mature form, of the polypeptide having a sequence as indicated in Table IIA or IIB, columns 5 or 7, lines 279, 282, 285, 286, 288, and/or 289, and/or lines 278, 280, 281, 283, 284, and/or 287 and/or lines 637 to 641 or a fragment thereof, which confers an increase in the amount of the respective fine chemical in an organism or a part thereof;
b) nucleic acid molecule comprising, preferably at least the mature form, of a nucleic acid molecule having a sequence as indicated in Table IA or IB , columns 5 or 7, lines 279, 282, 285, 286, 288, and/or 289, and/or lines 278, 280, 281, 283, 284,and/or 287 and/or lines 637 to 641,
c) nucleic acid molecule whose sequence can be deduced from a polypeptide sequence encoded by a nucleic acid molecule of (a) or (b) as result of the degeneracy of the genetic code and conferring an increase in the amount of the respective fine chemical in an organism or a part thereof;
d) nucleic acid molecule encoding a polypeptide which has at least 50% identity with the amino acid sequence of the polypeptide encoded by the nucleic acid molecule of (a) to (c) and conferring an increase in the amount of the respective fine chemical in an organism or a part thereof;
e) nucleic acid molecule which hybridizes with a nucleic acid molecule of (a) to (c) under under stringent hybridization conditions and conferring an increase in the amount of the respective fine chemical in an organism or a part thereof;
f) nucleic acid molecule encoding a polypeptide, the polypeptide being derived by substituting, deleting and/or adding one or more amino acids of the amino acid sequence of the polypeptide encoded by the nucleic acid molecules (a) to (d), preferably to (a) to (c) and conferring an increase in the amount of the respective fine chemical in an organism or a part thereof;
g) nucleic acid molecule encoding a fragment or an epitope of a polypeptide which is encoded by one of the nucleic acid molecules of (a) to (e), preferably to (a) to (c) and and conferring an increase in the amount of the respective fine chemical in an organism or a part thereof;
h) nucleic acid molecule comprising a nucleic acid molecule which is obtained by amplifying nucleic acid molecules from a cDNA library or a genomic library using the primers pairs having a sequence as indicated in Table III, columns 7, lines 279, 282, 285, 286, 288, and/or 289, and/or lines 278, 280, 281, 283, 284, and/or 287 and/or lines 637 to 641,and conferring an increase in the amount of the respective fine chemical in an organism or a part thereof;
i) nucleic acid molecule encoding a polypeptide which is isolated, e.g. from an expression library, with the aid of monoclonal antibodies against a polypeptide encoded by one of the nucleic acid molecules of (a) to (h), preferably to (a) to (c), and and conferring an increase in the amount of the respective fine chemical in an organism or a part thereof;
j) nucleic acid molecule which encodes a polypeptide comprising the consensus sequence having a sequences as indicated in Table IV, columns 7, lines 279, 282, 285, 286, 288, and/or 289, and/or lines 278, 280, 281, 283, 284, and/or 287 and/or lines 637 to 641 and conferring an increase in the amount of the respective fine chemical in an organism or a part thereof;
k) nucleic acid molecule comprising one or more of the nucleic acid molecule encoding the amino acid sequence of a polypeptide encoding a domain of the polypeptide indicated in Table IIA or IIB, columns 5 or 7, lines 279, 282, 285, 286, 288, and/or 289, and/or lines 278, 280, 281, 283, 284, and/or 287 and/or lines 637 to 641, and conferring an increase in the amount of the respective fine chemical in an organism or a part thereof; and
l) nucleic acid molecule which is obtainable by screening a suitable library under stringent conditions with a probe comprising one of the sequences of the nucleic acid molecule of (a) to (k), preferably to (a) to (c), or with a fragment of at least 15 nt, preferably 20 nt, 30 nt, 50 nt, 100 nt, 200 nt or 500 nt of the nucleic acid molecule characterized in (a) to (k), preferably to (a) to (c), and conferring an increase in the amount of the respective fine chemical in an organism or a part thereof;
or which comprises a sequence which is complementary thereto.

**[0104.0.24.24]** In one embodiment, the nucleic acid molecule used in the process distinguishes over the sequence indicated in Table IA or IB , columns 5 or 7, lines 279, 282, 285, 286, 288, and/or 289, and/or lines 278, 280, 281, 283, 284 and/or 287 and/or lines 637 to 641 by one or more nucleotides. In one embodiment, the nucleic acid molecule used in the process of the invention does not consist of the sequence indicated in Table IA or IB , columns 5 or 7, lines 279, 282, 285, 286, 288, and/or 289, and/or lines 278, 280, 281, 283, 284 and/or 287 and/or lines 637 to 641. In one embodiment, the nucleic acid molecule of the present invention is less than 100%, 99,999%, 99,99%, 99,9% or 99% identical to the sequence indicated in Table IA or IB , columns 5 or 7, lines 279, 282, 285, 286, 288, and/or 289, and/or lines 278, 280, 281, 283, 284 and/or 287 and/or lines 637 to 641. In another embodiment, the nucleic acid molecule does not encode a polypeptide of a sequence indicated in Table IIA or IIB, columns 5 or 7, lines 279, 282, 285, 286, 288, and/or 289, and/or lines 278, 280, 281, 283, 284 and/or 287 and/or lines 637 to 641.

**[0105.0.0.24] to [0107.0.0.24]: see [0105.0.0.0] to [0107.0.0.0]**

**[0108.0.24.24]** Nucleic acid molecules with the sequence as indicated in Table IA or IB , columns 5 or 7, lines 279, 282, 285, 286, 288, and/or 289, and/or lines 278, 280, 281, 283, 284 and/or 287 and/or lines 637 to 641, nucleic acid molecules which are derived from an amino acid sequences as indicated in Table IIA or IIB, columns 5 or 7, lines 279, 282, 285, 286, 288, and/or 289, and/or lines 278, 280, 281, 283, 284, and/or 287 and/or lines 637 to 641 or from polypeptides comprising the consensus sequence as indicated in Table IV, columns 7, lines 279, 282, 285, 286, 288, and/or 289, and/or lines 278, 280, 281, 283, 284 and/or 287 and/or lines 637 to 641, or their derivatives or homologues encoding polypeptides with the enzymatic or biological activity of an activity of a polypeptide as indicated in Table IIA or IIB, column 3, 5 or 7, lines 279, 282, 285, 286, 288, and/or 289, and/or lines 278, 280, 281, 283, 284 and/or 287 and/or lines 637 to 641, e.g. conferring the increase of the respective fine chemical, meaning carotenoids, in particular carotene, in particular beta-carotene or the carotene precursor IPP, resp., after increasing its expression or activity, are advantageously increased in the process according to the invention.

**[0109.0.24.24]** In one embodiment, said sequences are cloned into nucleic acid constructs, either individually or in combination. These nucleic acid constructs enable an optimal synthesis of the respective fine chemicals, in particular IPP or beta-carotene, produced in the process according to the invention.

**[0110.0.0.24] see . [0110.0.0.0]**

**[0111.0.0.24] see [0111.0.0.0]**

**[0112.0.24.24]** The nucleic acid molecules used in the process according to the invention take the form of isolated nucleic acid sequences, which encode polypeptides with an activity of a polypeptide as indicated in Table IIA or IIB, column 3, lines 279, 282, 285, 286, 28 and/or 289, and/or lines 278, 280, 281, 283, 284 and/or 287 and/or lines 637 to 641 or having the sequence of a polypeptide as indicated in Table IIA or IIB, columns 5 and 7, lines 279, 282, 285, 286, 288 and/or 289 and/or lines 278, 280, 281, 283, 284 and/or 287 and/or lines 637 to 641 and conferring an increase in the IPP and/or beta-carotene level, respectively.

**[0113.0.0.24]** to **[0114.0.0.24]:** see **[0113.0.0.0]** to **[0114.0.0.0]**

**[0115.0.0.24]** see **[0115.0.0.0]**

**[0116.0.0.24]** to **[0120.0.0.24]** see **[0116.0.0.0]** to **[0120.0.0.0]**

**[0120.1.0.24]** Production strains which are also advantageously selected in the process according to the invention are microorganisms selected from the group green algae , like Spongioccoccum exentricum, Chlorella sorokiniana (pyrenoidosa, 7-11-05), or form the group of fungi like fungi belonging to the Daccrymycetaceae family, or non-photosynthetic bacteria, like methylotrophs, flavobacteria, actinomycetes, like streptomyces chrestomyceticus, Mycobacteria like Mycobacterim phlei, or Rhodobacter capsulatus.

**[0121.0.24.24]** However, it is also possible to use artificial sequences, which differ in one or more bases from the nucleic acid sequences found in organisms, or in one or more amino acid molecules from polypeptide sequences found in organisms, in particular from the polypeptide sequences indicated in Table IIA or IIB, columns 5 or 7, lines 279, 282, 285, 286, 288 and/or 289 and/or lines 278, 280, 281, 283, 284 and/or 287 and/or lines 637 to 641 or the functional homologues thereof as described herein, preferably conferring above-mentioned activity I.e. they confer a beta-carotene increase after increasing the activity of the polypeptide sequences indicated in Table IIA or IIB, columns 5 or 7, lines 279, 282, 285, 286, 288,and/or 289 or conferring a IPP level increase after increasing the activity of the polypeptide sequences indicated in Table IIA or IIB, columns 5 or 7, lines 278, 280, 281, 283, 284 and/or 287 and/or lines 637 to 641.

**[0122.0.0.24]** to **[0127.0.0.24]:** see **[0122.0.0.0]** to **[0127.0.0.0]**

**[0128.0.24.24]** Synthetic oligonucleotide primers for the amplification, e.g. as the pairs indicated in Table III, columns 7, lines 279, 282, 285, 286, 288 and/or 289, and/or lines 278, 280, 281, 283, 284 and/or 287 and/or lines 637 to 641, by means of polymerase chain reaction can be generated on the basis of a sequence shown herein, for example the sequence as indicated in Table IA or IB, columns 5 or 7, respective lines 279, 282, 285, 286, 288 and/or 289 and/or lines 278, 280, 281, 283, 284 and/or 287 and/or lines 637 to 641 or the sequences derived from a sequences as indicated in Table IIA or IIB, columns 5 or 7, respective lines 279, 282, 285, 286, 288 and/or 289,and/or lines 278, 280, 281, 283, 284 and/or 287 and/or lines 637 to 641, resp.

**[0129.0.24.24]** Moreover, it is possible to identify conserved regions from various organisms by carrying out protein sequence alignments with the polypeptide used in the process of the invention, in particular with sequences of the polypeptide of the invention, from which conserved regions, and in turn, degenerate primers can be derived. Conserved region for the polypeptide of the invention are indicated in the alignments shown in the figures. Conserved regions are those, which show a very little variation in the amino acid in one particular position of several homologs from different origin. The consensus sequence indicated in Table IV, columns 7, lines 279, 282, 285, 286, 288 and/or 289, and/or lines 278, 280, 281, 283, 284 and/or 287 and/or lines 637 to 641 is derived from said alignments.

**[0130.0.24.24]** Degenerated primers can then be utilized by PCR for the amplification of fragments of novel proteins having above-mentioned activity, e.g. conferring the increase of the respective fine chemical after increasing the expression or activity the protein comprising said fragment.

**[0131.0.0.24] to [0138.0.0.24]: see [0131.0.0.0] to [0138.0.0.0]**

**[0139.0.24.24]** Polypeptides having above-mentioned activity, i.e. conferring the respective fine chemical level increase, derived from other organisms, can be encoded by other DNA sequences which hybridise to a sequence indicated in Table IA or IB , columns 5 or 7, lines 279, 282, 285, 286, 288 and/or 289 for beta-carotene or indicated in Table IA or IB , columns 5 or 7, lines 278, 280, 281, 283, 284 and/or 287 and/or lines 637 to 641 for IPP under relaxed hybridization conditions and which code on expression for peptides having the respective fine chemical, i.e. beta-carotene or IPP, resp.,-increasing activity.

**[0140.0.0.24] to [0146.0.0.24]: see [0140.0.0.0] to [0146.0.0.0]**

**[0147.0.24.24]** Further, the nucleic acid molecule of the invention comprises a nucleic acid molecule, which is a complement of one of the nucleotide sequences of above mentioned nucleic acid molecules or a portion thereof. A nucleic acid molecule which is complementary to one of the nucleotide sequences indicated in Table IA or IB , columns 5 or 7, lines 279, 282, 285, 286, 288 and/or 289 and/or lines 278, 280, 281, 283, 284 and/or 287 and/or lines 637 to 641 is one which is sufficiently complementary to one of said nucleotide sequences such that it can hybridise to one of said nucleotide sequences, thereby forming a stable duplex. Preferably, the hybridisation is performed under stringent hybrization conditions. However, a complement of one of the herein disclosed sequences is preferably a sequence complement thereto according to the base pairing of nucleic acid molecules well known to the skilled person. For example, the bases A and G undergo base pairing with the bases T and U or C, resp. and visa versa. Modifications of the bases can influence the base-pairing partner.

**[0148.0.24.24]** The nucleic acid molecule of the invention comprises a nucleotide sequence which is at least about 30%, 35%, 40% or 45%, preferably at least about 50%, 55%, 60% or 65%, more preferably at least about 70%, 80%, or 90%, and even more preferably at least about 95%, 97%, 98%, 99% or more homologous to a nucleotide sequence indicated in Table IA or IB , columns 5 or 7, lines 279, 282, 285, 286, 288 and/or 289 and/or lines 278, 280, 281, 283, 284 and/or 287 and/or lines 637 to 641 ,or a portion thereof and preferably has above mentioned activity, in particular having a IPP or beta-carotene-increasing activity after increasing the activity or an activity of a product of a gene encoding said sequences or their homologs.

**[0149.0.24.24]** The nucleic acid molecule of the invention comprises a nucleotide sequence which hybridizes, preferably hybridizes under stringent conditions as defined herein, to one of the nucleotide sequences indicated in Table IA or IB , columns 5 or 7, lines 279, 282, 285, 286, 288 and/or 289 and/or lines 278, 280, 281, 283, 284 and/or 287 and/or lines 637 to 641, or a portion thereof and encodes a protein having above-mentioned activity, e.g. conferring a IPP or beta-carotene increase, resp., and optionally, the activity of protein indicated in Table IIA or IIB, column 5, lines 279, 282, 285, 286, 288 and/or 289 and/or lines 278, 280, 281, 283, 284 and/or 287 and/or lines 637 to 641.

**[00149.1.24.24]** Optionally, in one embodiment, the nucleotide sequence, which hybridises to one of the nucleotide sequences indicated in Table IA or IB , columns 5 or 7, lines 279, 282, 285, 286, 288 and/or 289 and/or lines 278, 280, 281, 283, 284, and/or 287 and/or lines 637 to 641 has further one or more of the activities annotated or known for the a protein as indicated in Table IIA or IIB, column 3, lines 279, 282, 285, 286, 288 and/or 289 and/or lines 278, 280, 281, 283, 284 and/or 287 and/or lines 637 to 641.

**[0150.0.24.24]** Moreover, the nucleic acid molecule of the invention can comprise only a portion of the coding region of one of the sequences indicated in Table IA or IB , columns 5 or 7, lines 279, 282, 285, 286, 288 and/or 289 and/or lines 278, 280, 281, 283, 284 and/or 287 and/or lines 637 to 641, for example a fragment which can be used as a probe or primer or a fragment encoding a biologically active portion of the polypeptide of the present invention or of a polypeptide used in the process of the present invention, i.e. having above-mentioned activity, e.g. conferring an increase of beta-carotene or IPP, resp., if its activity is increased. The nucleotide sequences determined from the cloning of the present protein-according-to-the-invention-encoding gene allows for the generation of probes and primers designed for use in identifying and/or cloning its homologues in other cell types and organisms. The probe/primer typically comprises substantially purified oligonucleotide. The oligonucleotide typically comprises a region of nucleotide sequence that hybridizes under stringent conditions to at least about 12, 15 preferably about 20 or 25, more preferably about 40, 50 or 75 consecutive nucleotides of a sense strand of one of the sequences set forth, e.g., as indicated in Table IA or IB , columns 5 or 7, lines 279, 282, 285, 286, 288 and/or 289 and/or lines 278, 280, 281, 283, 284 and/or 287 and/or lines 637 to 641, an anti-sense sequence of one of the sequences, e.g., as indicated in Table IA or IB , columns 5 or 7, lines 279, 282, 285, 286, 288 and/or 289 and/or lines 278, 280, 281, 283, 284, and/or 287 and/or lines 637 to 641, or naturally occurring mutants thereof. Primers based on a nucleotide of invention can be used in PCR reactions to clone homologues of the polypeptide of the invention or of the polypeptide used in the process of the invention, e.g. as the primers described in the examples of the present invention, e.g. as shown in the examples. A PCR with the primer pairs indicated in Table III, column 7, lines 279, 282, 285, 286, 288 and/or 289 and/or lines 278, 280, 281, 283, 284 and/or 287 and/or lines 637 to 641 will result in a fragment of a polynucleotide sequence as indicated in Table IA or IB , columns 5 or 7, lines 279, 282, 285, 286, 288 and/or 289,and/or lines 278, 280, 281, 283, 284 and/or 287 and/or lines 637 to 641 or its gene product.

**[0151.0.0.24]:** see **[0151.0.0.0]**

**[0152.0.24.24]** The nucleic acid molecule of the invention encodes a polypeptide or portion thereof which includes an amino acid sequence which is sufficiently homologous to an amino acid sequence as indicated in Table IIA or IIB, columns 5 or 7, lines 279, 282, 285, 286, 288 and/or 289 and/or lines 278, 280, 281, 283, 284 and/or 287 such that the protein or portion thereof maintains the ability to participate in the respective fine chemical production, in particular a beta-carotene (lines 279, 282, 285, 286, 288, and/or 289) or IPP (lines 278, 280, 281, 283, 284 and/or 287 and/or lines 637 to 641) increasing activity as mentioned above or as described in the examples in plants or microorganisms is comprised.

**[0153.0.24.24]** As used herein, the language "sufficiently homologous" refers to proteins or portions thereof which have amino acid sequences which include a minimum number of identical or equivalent amino acid residues (e.g., an amino acid residue which has a similar side chain as an amino acid residue in one of the sequences of the polypeptide of the present invention) to an amino acid sequence as indicated in Table IIA or IIB, columns 5 or 7, lines 279, 282, 285, 286, 288 and/or 289 and/or lines 278, 280, 281, 283, 284 and/or 287 and/or lines 637 to 641 such that the protein or portion thereof is able to participate in the increase of the respective fine chemical production. In one embodiment, a protein or portion thereof as indicated in Table IIA or IIB, columns 5 or 7, lines 279, 282, 285, 286, 288 and/or 289 and/or lines 278, 280, 281, 283, 284 and/or 287 and/or lines 637 to 641 has for example an activity of a polypeptide indicated in Table IIA or IIB, column 3, lines 279, 282, 285, 286, 288 and/or 289 and/or lines 278, 280, 281, 283, 284 and/or 287 and/or lines 637 to 641.

**[0154.0.24.24]** In one embodiment, the nucleic acid molecule of the present invention comprises a nucleic acid that encodes a portion of the protein of the present invention. The protein is at least about 30%, 35%, 40%, 45% or 50%, preferably at least about 55%, 60%, 65% or 70%, and more preferably at least about 75%, 80%, 85%, 90%, 91%, 92%, 93% or 94% and most preferably at least about 95%, 97%, 98%, 99% or more homologous to an entire amino acid sequence as indicated in Table IIA or IIB, columns 5 or 7, lines 279, 282, 285, 286, 288,and/or 289 and/or lines 278, 280, 281, 283, 284 and/or 287 and/or lines 637 to 641 and has above-mentioned activity, e.g. conferring preferably the increase of the respective fine chemical.

**[0155.0.0.24]** to **[0156.0.0.24]: see [0155.0.0.0]** to **[0156.0.0.0]**

**[0157.0.24.24]** The invention further relates to nucleic acid molecules that differ from one of the nucleotide sequences as indicated in Table IA or IB , columns 5 or 7, lines 279, 282, 285, 286, 288 and/or 289 and/or lines 278, 280, 281, 283, 284 and/or 287 and/or lines 637 to 641 (and portions thereof) due to degeneracy of the genetic code and thus encode a polypeptide of the present invention, in particular a polypeptide having above mentioned activity, e.g. conferring an increase in the respective fine chemical in a organism, e.g. as polypeptides comprising the sequence as indicated in Table IV, columns 5 or 7, lines 279, 282, 285, 286, 288 and/or 289 and/or lines 278, 280, 281, 283, 284 and/or 287 and/or lines 637 to 641 or as polypeptides depicted in Table IIA or IIB, columns 5 or 7, lines 279, 282, 285, 286, 288 and/or 289 and/or lines 278, 280, 281, 283, 284 and/or 287 and/or lines 637 to 641 or the functional homologues. Advantageously, the nucleic acid molecule of the invention comprises, or in an other embodiment has, a nucleotide sequence encoding a protein comprising, or in an other embodiment having, an amino acid sequence of a consensus sequences as indicated in Table IV, columns 5 or 7, lines 279, 282, 285, 286, 288 and/or 289 and/or lines 278, 280, 281, 283, 284 and/or 287 and/or lines 637 to 641 or of the polypeptide as indicated in Table IIA or IIB, columns 5 or 7, lines 279, 282, 285, 286, 288 and/or 289 and/or lines 278, 280, 281, 283, 284 and/or 287 and/or lines 637 to 641, resp., or the functional homologues. In a still further embodiment, the nucleic acid molecule of the invention encodes a full length protein which is substantially homologous to an amino acid sequence comprising a consensus sequence as indicated in Table IV, columns 5 or 7, lines 279, 282, 285, 286, 288, and/or 289 and/or lines 278, 280, 281, 283, 284 and/or 287 and/or lines 637 to 641 or of a polypeptide as indicated in Table IIA or IIB, columns 5 or 7, lines 279, 282, 285, 286, 288 and/or 289,and/or lines 278, 280, 281, 283, 284 and/or 287 and/or lines 637 to 641 or the functional homologues. However, in a preferred embodiment, the nucleic acid molecule of the present invention does not consist of a sequence as indicated in Table IA or IB , columns 5 or 7, lines 279, 282, 285, 286, 288 and/or 289 and/or lines 278, 280, 281, 283, 284 and/or 287 and/or lines 637 to 641, resp.

**[0158.0.0.24]** to **[0160.0.0.24]: see [0158.0.0.0]** to **[0160.0.0.0]**

**[0161.0.24.24]** Accordingly, in another embodiment, a nucleic acid molecule of the invention is at least 15, 20, 25 or 30 nucleotides in length. Preferably, it hybridizes under stringent conditions to a nucleic acid molecule comprising a nucleotide sequence of the nucleic acid molecule of the present invention or used in the process of the present invention, e.g. comprising a sequence as indicated in Table IA or IB , columns 5 or 7, lines 279, 282, 285, 286, 288 and/or 289 and/or lines 278, 280, 281, 283, 284 and/or 287 and/or lines 637 to 641. The nucleic acid molecule is preferably at least 20, 30, 50, 100, 250 or more nucleotides in length.

**[0162.0.0.24] see [0162.0.0.0]**

**[0163.0.24.24]** Preferably, nucleic acid molecule of the invention that hybridizes under stringent conditions to a sequence as indicated in Table IA or IB , columns 5 or 7, lines 279, 282, 285, 286, 288 and/or 289 and/or lines 278, 280, 281, 283, 284 and/or 287 and/or lines 637 to 641 corresponds to a naturally-occurring nucleic acid molecule of the invention. As used herein, a "naturally-occurring" nucleic acid molecule refers to an RNA or DNA molecule having a nucleotide sequence that occurs in nature (e.g., encodes a natural protein). Preferably, the nucleic acid molecule encodes a natural protein having above-mentioned activity, e.g. conferring the increase of the amount of the respective fine chemical in a organism or a part thereof, e.g. a tissue, a cell, or a compartment of a cell, after increasing the expression or activity thereof or the activity of a protein of the invention or used in the process of the invention.

**[0164.0.0.24] see [0164.0.0.0]**

**[0165.0.24.24]** For example, nucleotide substitutions leading to amino acid substitutions at "non-essential" amino acid residues can be made in a sequence of the nucleic acid molecule of the invention or used in the process of the invention, e.g. as indicated in Table IA or IB , columns 5 or 7, lines 279, 282, 285, 286, 288 and/or 289 and/or lines 278, 280, 281, 283, 284 and/or 287 and/or lines 637 to 641 resp..

**[0166.0.0.24] to [0167.0.0.24]: see [0166.0.0.0] to [0167.0.0.0]**

**[0168.0.24.24]** Accordingly, the invention relates to nucleic acid molecules encoding a polypeptide having above-mentioned activity, e.g. conferring an increase in the respective fine chemical in an organisms or parts thereof that contain changes in amino acid residues that are not essential for said activity. Such polypeptides differ in amino acid sequence from a sequence contained in a sequence as indicated in Table IIA or IIB, columns 5 or 7, lines 279, 282, 285, 286, 288 and/or 289 and/or lines 278, 280, 281, 283, 284 and/or 287 and/or lines 637 to 641, resp., yet retain said activity described herein. The nucleic acid molecule can comprise a nucleotide sequence encoding a polypeptide, wherein the polypeptide comprises an amino acid sequence at least about 50% identical to an amino acid sequence as indicated in Table IIA or IIB, columns 5 or 7, lines 279, 282, 285, 286, 288 and/or 289 and/or lines 278, 280, 281, 283, 284 and/or 287 and/or lines 637 to 641 resp., and is capable of participation in the increase of production of the respective fine chemical after increasing its activity, e.g. its expression. Preferably, the protein encoded by the nucleic acid molecule is at least about 60% identical to a sequence as indicated in Table IIA or IIB, columns 5 or 7, lines 279, 282, 285, 286, 288 and/or 289 and/or lines 278, 280, 281, 283, 284 and/or 287 and/or lines 637 to 641 resp., more preferably at least about 70% identical to one of the sequences as indicated in Table IIA or IIB, columns 5 or 7, lines 279, 282, 285, 286, 288 and/or 289 and/or lines 278, 280, 281, 283, 284 and/or 287 and/or lines 637 to 641, resp., even more preferably at least about 80%, 90%, 95% homologous to a sequence as indicated in Table IIA or IIB, columns 5 or 7, lines 279, 282, 285, 286, 288 and/or 289 and/or lines 278, 280, 281, 283, 284 and/or 287 and/or lines 637 to 641, resp., and most preferably at least about 96%, 97%, 98%, or 99% identical to the sequence as indicated in Table IIA or IIB, columns 5 or 7, lines 279, 282, 285, 286, 288 and/or 289 and/or lines 278, 280, 281, 283, 284 and/or 287 and/or lines 637 to 641.

**[0169.0.0.24]** to **[0172.0.0.24]: see [0169.0.0.0]** to **[0172.0.0.0]**

**[0173.0.24.24]** For example a sequence, which has 80% homology with sequence SEQ ID NO: 34228 at the nucleic acid level is understood as meaning a sequence which, upon comparison with the sequence SEQ ID NO: 34228 by the above Gap program algorithm with the above parameter set, has a 80% homology.

**[0174.0.0.24]: see [0174.0.0.0]**

**[0175.0.24.24]** For example a sequence which has a 80% homology with sequence SEQ ID NO: 34229 at the protein level is understood as meaning a sequence which, upon comparison with the sequence SEQ ID NO: 34229 by the above program algorithm with the above parameter set, has a 80% homology.

**[0176.0.24.24]** Functional equivalents derived from one of the polypeptides as indicated in Table IIA or IIB, columns 5 or 7, lines 279, 282, 285, 286, 288 and/or 289 and/or lines 278, 280, 281, 283, 284 and/or 287 and/or lines 637 to 641, resp., according to the invention by substitution, insertion or deletion have at least 30%, 35%, 40%, 45% or 50%, preferably at least 55%, 60%, 65% or 70% by preference at least 80%, especially preferably at least 85% or 90%, 91 %, 92%, 93% or 94%, very especially preferably at least 95%, 97%, 98% or 99% homology with one of the polypeptides as indicated in Table IIA or IIB, columns 5 or 7, lines 279, 282, 285, 286, 288 and/or 289 and/or lines 278, 280, 281, 283, 284 and/or 287 and/or lines 637 to 641, resp., according to the invention and are distinguished by essentially the same properties as a polypeptide as indicated in Table IIA or IIB, columns 5 or 7, lines 279, 282, 285, 286, 288 and/or 289, and/or lines 278, 280, 281, 283, 284 and/or 287 and/or lines 637 to 641, resp..

**[0177.0.24.24]** Functional equivalents derived from a nucleic acid sequence as indicated in Table IA or IB , columns 5 or 7, lines 279, 282, 285, 286, 288 and/or 289 and/or lines 278, 280, 281, 283, 284 and/or 287 and/or lines 637 to 641, resp., according to the invention by substitution, insertion or deletion have at least 30%, 35%, 40%, 45% or 50%, preferably at least 55%, 60%, 65% or 70% by preference at least 80%, especially preferably at least 85% or 90%, 91%, 92%, 93% or 94%, very especially preferably at least 95%, 97%, 98% or 99% homology with one of the polypeptides as indicated in Table IIA or IIB, columns 5 or 7, lines 279, 282, 285, 286, 288 and/or 289 and/or lines 278, 280, 281, 283, 284 and/or 287 and/or lines 637 to 641, resp., according to the invention and encode polypeptides having essentially the same properties as a polypeptide as indicated in Table IIA or IIB, columns 5 or 7, lines 279, 282, 285, 286, 288 and/or 289 and/or lines 278, 280, 281, 283, 284 and/or 287 and/or lines 637 to 641, resp..

**[0178.0.0.24] see [0178.0.0.0]**

**[0179.0.24.24]** A nucleic acid molecule encoding an homologous to a protein sequence as indicated in Table IIA or IIB, columns 5 or 7, lines 279, 282, 285, 286, 288 and/or 289, and/or lines 278, 280, 281, 283, 284 and/or 287 and/or lines 637 to 641, resp., can be created by introducing one or more nucleotide substitutions, additions or deletions into a nucleotide sequence of the nucleic acid molecule of the present invention, in particular as indicated in Table IA or IB , columns 5 or 7, lines 279, 282, 285, 286, 288 and/or 289 and/or lines 278, 280, 281, 283, 284 and/or 287 and/or lines 637 to 641, resp., such that one or more amino acid substitutions, additions or deletions are introduced into the encoded protein. Mutations can be introduced into the encoding sequences of a sequences as indicated in Table IA or IB , columns 5 or 7, lines 279, 282, 285, 286, 288 and/or 289 and/or lines 278, 280, 281, 283, 284 and/or 287 and/or lines 637 to 641, resp., by standard techniques, such as site-directed mutagenesis and PCR-mediated mutagenesis.

**[0180.0.0.24] to [0183.0.0.24]: see [0180.0.0.0] to [0183.0.0.0]**

**[0184.0.24.24]** Homologues of the nucleic acid sequences used, with a sequence as indicated in Table IA or IB , columns 5 or 7, lines 279, 282, 285, 286, 288 and/or 289 and/or lines 278, 280, 281, 283, 284 and/or 287 and/or lines 637 to 641, resp., or of the nucleic acid sequences derived from a sequences as indicated in Table IIA or IIB, columns 5 or 7, lines 279, 282, 285, 286, 288 and/or 289 and/or lines 278, 280, 281, 283, 284 and/or 287 and/or lines 637 to 641, resp., comprise also allelic variants with at least approximately 30%, 35%, 40% or 45% homology, by preference at least approximately 50%, 60% or 70%, more preferably at least approximately 90%, 91 %, 92%, 93%, 94% or 95% and even more preferably at least approximately 96%, 97%, 98%, 99% or more homology with one of the nucleotide sequences shown or the abovementioned derived nucleic acid sequences or their homologues, derivatives or analogues or parts of these. Allelic variants encompass in particular functional variants which can be obtained by deletion, insertion or substitution of nucleotides from the sequences shown, preferably from a sequence as indicated in Table IA or IB , columns 5 or 7, lines 279, 282, 285, 286, 288 and/or 289 and/or lines 278, 280, 281, 283, 284 and/or 287 and/or lines 637 to 641, resp., or from the derived nucleic acid sequences, the intention being, however, that the enzyme activity or the biological activity of the resulting proteins synthesized is advantageously retained or increased.

**[0185.0.24.24]** In one embodiment of the present invention, the nucleic acid molecule of the invention or used in the process of the invention comprises one or more sequences as indicated in Table IA or IB , columns 5 or 7, lines 279, 282, 285, 286, 288 and/or 289 and/or lines 278, 280, 281, 283, 284 and/or 287 and/or lines 637 to 641, resp., In one embodiment, it is preferred that the nucleic acid molecule comprises as little as possible other nucleotides not shown in any one of sequences as indicated in Table IA or IB , columns 5 or 7, lines 279, 282, 285, 286, 288 and/or 289 and/or lines 278, 280, 281, 283, 284, and/or 287 and/or lines 637 to 641, resp.,. In one embodiment, the nucleic acid molecule comprises less than 500, 400, 300, 200, 100, 90, 80, 70, 60, 50 or 40 further nucleotides. In a further embodiment, the nucleic acid molecule comprises less than 30, 20 or 10 further nucleotides. In one embodiment, a nucleic acid molecule used in the process of the invention is identical to a sequences as indicated in Table IA or IB , columns 5 or 7, lines 279, 282, 285, 286, 288 and/or 289 and/or lines 278, 280, 281, 283, 284 and/or 287 and/or lines 637 to 641, resp..

**[0186.0.24.24]** Also preferred is that one or more nucleic acid molecule(s) used in the process of the invention encodes a polypeptide comprising a sequence as indicated in Table IIA or IIB, columns 5 or 7, lines 279, 282, 285, 286, 288 and/or 289 and/or lines 278, 280, 281, 283, 284 and/or 287 and/or lines 637 to 641, resp., In one embodiment, the nucleic acid molecule encodes less than 150, 130, 100, 80, 60, 50, 40 or 30 further amino acids. In a further embodiment, the encoded polypeptide comprises less than 20, 15, 10, 9, 8, 7, 6 or 5 further amino acids. In one embodiment, the encoded polypeptide used in the process of the invention is identical to the sequences as indicated in Table IIA or IIB, columns 5 or 7, lines 279, 282, 285, 286, 288 and/or 289 and/or lines 278, 280, 281, 283, 284 and/or 287 and/or lines 637 to 641, resp.,

**[0187.0.24.24]** In one embodiment, a nucleic acid molecule of the invention or used in the process encodes a polypeptide comprising the sequence as indicated in Table IIA or IIB, columns 5 or 7, lines 279, 282, 285, 286, 288 and/or 289 and/or lines 278, 280, 281, 283, 284 and/or 287 and/or lines 637 to 641, resp., comprises less than 100 further nucleotides. In a further embodiment, said nucleic acid molecule comprises less than 30 further nucleotides. In one embodiment, the nucleic acid molecule used in the process is identical to a coding sequence encoding a sequences as indicated in Table IIA or IIB, columns 5 or 7, lines 279, 282, 285, 286, 288, and/or 289,and/or lines 278, 280, 281, 283, 284 and/or 287 and/or lines 637 to 641, resp..

**[0188.0.24.24]** Polypeptides (= proteins), which still have the essential enzymatic activity of the polypeptide of the present invention conferring an increase of the respective fine chemical i.e. whose activity is essentially not reduced, are polypeptides with at least 10% or 20%, by preference 30% or 40%, especially preferably 50% or 60%, very especially preferably 80% or 90 or more of the wild type biological activity or enzyme activity, advantageously, the activity is essentially not reduced in comparison with the activity of a polypeptide as indicated in Table IIA or IIB, columns 5 or 7, lines 279, 282, 285, 286, 288 and/or 289 and/or lines 278, 280, 281, 283, 284, and/or 287 and/or lines 637 to 641, resp., and is expressed under identical conditions. **[0189.0.24.24]** Homologues of a sequences as indicated in Table IA or IB , columns 5 or 7, lines 279, 282, 285, 286, 288 and/or 289 and/or lines 278, 280, 281, 283, 284 and/or 287 and/or lines 637 to 641, resp., or of a derived sequences as indicated in Table IIA or IIB, columns 5 or 7, lines 279, 282, 285, 286, 288 and/or 289 and/or lines 278, 280, 281, 283, 284 and/or 287 and/or lines 637 to 641, resp., also mean truncated sequences, cDNA, single-stranded DNA or RNA of the coding and noncoding DNA sequence. Homologues of said sequences are also understood as meaning derivatives, which comprise noncoding regions such as, for example, UTRs, terminators, enhancers or promoter variants. The promoters upstream of the nucleotide sequences stated can be modified by one or more nucleotide substitution(s), insertion(s) and/or deletion(s) without, however, interfering with the functionality or activity either of the promoters, the open reading frame (= ORF) or with the 3'-regulatory region such as terminators or other 3'regulatory regions, which are far away from the ORF. It is furthermore possible that the activity of the promoters is increased by modification of their sequence, or that they are replaced completely by more active promoters, even promoters from heterologous organisms. Appropriate promoters are known to the person skilled in the art and are mentioned herein below.

**[0190.0.0.24]: see [0190.0.0.0]**

**[0191.0.0.24]** The organisms or part thereof produce according to the herein mentioned process of the invention an increased level of free and/or bound the respective fine chemical compared to said control or selected organisms or parts thereof.

**[0192.0.0.24] to [0203.0.0.24]: see [0192.0.0.0] to [0203.0.0.0]**

**[0204.0.24.24]** Accordingly, in one embodiment, the invention relates to a nucleic acid molecule, which comprises a nucleic acid molecule selected from the group consisting of:
a) nucleic acid molecule encoding, preferably at least the mature form, of a polypeptide as indicated in Table IIA or IIB, columns 5 or 7, lines 279, 282, 285, 286, 288 and/or 289and/or lines 278, 280, 281, 283, 284 and/or 287 and/or lines 637 to 641, resp.; or a fragment thereof conferring an increase in the amount of the respective fine chemical, i.e. beta-carotene (lines lines 279, 282, 285, 286, 288, and/or 289) or IPP (lines 278, 280, 281, 283, 284 and/or 287 and/or lines 637 to 641), resp., in an organism or a part thereof
b) nucleic acid molecule comprising, preferably at least the mature form, of a nucleic acid molecule as indicated in Table IA or IB , columns 5 or 7, lines 279, 282, 285, 286, 288 and/or 289 and/or lines 278, 280, 281, 283, 284 and/or 287 and/or lines 637 to 641, resp., or a fragment thereof conferring an increase in the amount of the respective fine chemical in an organism or a part thereof;
c) nucleic acid molecule whose sequence can be deduced from a polypeptide sequence encoded by a nucleic acid molecule of (a) or (b) as result of the degeneracy of the genetic code and conferring an increase in the amount of the respective fine chemical in an organism or a part thereof;
d) nucleic acid molecule encoding a polypeptide whose sequence has at least 50% identity with the amino acid sequence of the polypeptide encoded by the nucleic acid molecule of (a) to (c) and conferring an increase in the amount of the respective fine chemical in an organism or a part thereof;
e) nucleic acid molecule which hybridizes with a nucleic acid molecule of (a) to (c) under under stringent hybridisation conditions and conferring an increase in the amount of the respective fine chemical in an organism or a part thereof;
f) nucleic acid molecule encoding a polypeptide, the polypeptide being derived by substituting, deleting and/or adding one or more amino acids of the amino acid sequence of the polypeptide encoded by the nucleic acid molecules (a) to (d), preferably to (a) to (c), and conferring an increase in the amount of the respective fine chemical in an organism or a part thereof;
g) nucleic acid molecule encoding a fragment or an epitope of a polypeptide which is encoded by one of the nucleic acid molecules of (a) to (e), preferably to (a) to (c) and conferring an increase in the amount of the respective fine chemical in an organism or a part thereof;
h) nucleic acid molecule comprising a nucleic acid molecule which is obtained by amplifying a cDNA library or a genomic library using primers or primer pairs as indicated in Table III, columns 5 or 7, lines 279, 282, 285, 286, 288 and/or 289 and/or lines 278, 280, 281, 283, 284 and/or 287 and/or lines 637 to 641 and conferring an increase in the amount of the respective fine chemical, i.e. beta-carotene (lines lines 279, 282, 285, 286, 288 and/or 289) or IPP (lines 278, 280, 281, 283, 284 and/or 287 and/or lines 637 to 641), resp., in an organism or a part thereof;
i) nucleic acid molecule encoding a polypeptide which is isolated, e.g. from a expression library, with the aid of monoclonal antibodies against a polypeptide encoded by one of the nucleic acid molecules of (a) to (g), preferably to (a) to (c) and conferring an increase in the amount of the respective fine chemical in an organism or a part thereof;
j) nucleic acid molecule which encodes a polypeptide comprising a consensus sequence as indicated in Table IV, columns 5 or 7, lines 279, 282, 285, 286, 288 and/or 289,and/or lines 278, 280, 281, 283, 284 and/or 287 and/or lines 637 to 641 and conferring an increase in the amount of the respective fine chemical, i.e. beta-carotene (lines lines 279, 282, 285, 286, 288 and/or 289) or IPP (lines 278, 280, 281, 283, 284 and/or 287 and/or lines 637 to 641), resp., in an organism or a part thereof;
k) nucleic acid molecule encoding the amino acid sequence of a polypeptide encoding a domain of a polypeptide as indicated in Table IIA or IIB, columns 5 or 7, lines 279, 282, 285, 286, 288 and/or 289 and/or lines 278, 280, 281, 283, 284 and/or 287, resp., and conferring an increase in the amount of the respective fine chemical, i.e. beta-carotene (lines lines 279, 282, 285, 286, 288 and/or 289) or IPP (lines 278, 280, 281, 283, 284, and/or 287 and/or lines 637 to 641), resp., in an organism or a part thereof; and
l) nucleic acid molecule which is obtainable by screening a suitable nucleic acid library under stringent hybridization conditions with a probe comprising one of the sequences of the nucleic acid molecule of (a) to (k) or with a fragment of at least 15 nt, preferably 20 nt, 30 nt, 50 nt, 100 nt, 200 nt or 500 nt of the nucleic acid molecule characterized in (a) to (h) or of a nucleic acid molecule as indicated in Table IA or IB , columns 5 or 7, lines 279, 282, 285, 286, 288 and/or 289 and/or lines 278, 280, 281, 283, 284 and/or 287 and/or lines 637 to 641, resp., or a nucleic acid molecule encoding, preferably at least the mature form of, a polypeptide as indicated in Table IIA or IIB, columns 5 or 7, lines 279, 282, 285, 286, 288 and/or 289 and/or lines 278, 280, 281, 283, 284 and/or 287 and/or lines 637 to 641, resp., and conferring an increase in the amount of the respective fine chemical in an organism or a part thereof; or which encompasses a sequence which is complementary thereto;
whereby, preferably, the nucleic acid molecule according to (a) to (I) distinguishes over a sequence as indicated in Table IA or IB , columns 5 or 7, lines 279, 282, 285, 286, 288 and/or 289,and/or lines 278, 280, 281, 283, 284 and/or 287 and/or lines 637 to 641, resp., by one or more nucleotides. In one embodiment, the nucleic acid molecule of the invention does not consist of the sequence as indicated in Table IA or IB , columns 5 or 7, lines 279, 282, 285, 286, 288 and/or 289 and/or lines 278, 280, 281, 283, 284 and/or 287 and/or lines 637 to 641, resp. In an other embodiment, the nucleic acid molecule of the present invention is at least 30 % identical and less than 100%, 99,999%, 99,99%, 99,9% or 99% identical to a sequence as indicated in Table IA or IB , columns 5 or 7, lines 279, 282, 285, 286, 288 and/or 289 and/or lines 278, 280, 281, 283, 284 and/or 287 and/or lines 637 to 641, resp. In a further embodiment the nucleic acid molecule does not encode a polypeptide sequence as indicated in Table IIA or IIB, columns 5 or 7, lines 279, 282, 285, 286, 288 and/or 289 and/or lines 278, 280, 281, 283, 284 and/or 287 and/or lines 637 to 641, resp. Accordingly, in one embodiment, the nucleic acid molecule of the present invention encodes in one embodiment a polypeptide which differs at least in one or more amino acids from a polypeptide indicated in Table IIA or IIB, columns 5 or 7, lines 279, 282, 285, 286, 288 and/or 289 and/or lines 278, 280, 281, 283, 284 and/or 287 and/or lines 637 to 641 does not encode a protein of a sequence as indicated in Table IIA or IIB, columns 5 or 7, lines 279, 282, 285, 286, 288 and/or 289 and/or lines 278, 280, 281, 283, 284 and/or 287 and/or lines 637 to 641. Accordingly, in one embodiment, the protein encoded by a sequences of a nucleic acid according to (a) to (I) does not consist of a sequence as indicated in Table IIA or IIB, columns 5 or 7, lines 279, 282, 285, 286, 288 and/or 289 and/or lines 278, 280, 281, 283, 284 and/or 287 and/or lines 637 to 641. In a further embodiment, the protein of the present invention is at least 30 % identical to a protein sequence indicated in Table IIA or IIB, columns 5 or 7, lines 279, 282, 285, 286, 288 and/or 289 and/or lines 278, 280, 281, 283, 284 and/or 287 and/or lines 637 to 641 and less than 100%, preferably less than 99,999%, 99,99% or 99,9%, more preferably less than 99%, 98%, 97%, 96% or 95% identical to a sequence as indicated in Table IIA or IIB, columns 5 or 7, lines 279, 282, 285, 286, 288 and/or 289 and/or lines 278, 280, 281, 283, 284 and/or 287 and/or lines 637 to 641.

**[0205.0.0.24]** to **[0206.0.0.24]: see [0205.0.0.0]** to **[0206.0.0.0]**

**[0207.0.24.24]** As described herein, the nucleic acid construct can also comprise further genes, which are to be introduced into the organisms or cells. It is possible and advantageous to introduce into, and express in, the host organisms regulatory genes such as genes for inductors, repressors or enzymes, which, owing to their enzymatic activity, engage in the regulation of one or more genes of a biosynthetic pathway. These genes can be of heterologous or homologous origin. Moreover, further biosynthesis genes may advantageously be present, or else these genes may be located on one or more further nucleic acid constructs. Genes, which are advantageously employed as biosynthesis genes, are genes of the carotenoid metabolism, the carotene, in particular beta-carotene metabolism, the astaxanthin metabolism, the amino acid metabolism, of glycolysis, of the tricarboxylic acid metabolism or their combinations. As described herein, regulator sequences or factors can have a positive effect on preferably the gene expression of the genes introduced, thus increasing it. Thus, an enhancement of the regulator elements may advantageously take place at the transcriptional level by using strong transcription signals such as promoters and/or enhancers. In addition, however, an enhancement of translation is also possible, for example by increasing mRNA stability or by inserting a translation enhancer sequence.

**[0208.0.0.24] to [0226.0.0.24]: see [0208.0.0.0] to [0226.0.0.0]**

**[0227.0.24.24]** The abovementioned nucleic acid molecules can be cloned into the nucleic acid constructs or vectors according to the invention in combination together with further genes, or else different genes are introduced by transforming several nucleic acid constructs or vectors (including plasmids) into a host cell, advantageously into a plant cell or a microorgansims.
In addition to a sequence indicated in Table IA or IB , columns 5 or 7, lines 279, 282, 285, 286, 288 and/or 289 and/or lines 278, 280, 281, 283, 284 and/or 287 and/or lines 637 to 641 or its derivatives, it is advantageous to express and/or mutate further genes in the organisms. Especially advantageously, additionally at least one further gene of the carotenoid, in particular carotene biosynthetic pathway, e.g. one of the above mentioned genes of this pathway, or e.g. for the synthesis of astaxanthin or for another provitamin A or for another carotenoids or carotene, is expressed in the organisms such as plants or microorganisms. It is also possible that the regulation of the natural genes has been modified advantageously so that the gene and/or its gene product is no longer subject to the regulatory mechanisms which exist in the organisms. This leads to an increased synthesis of the amino acids desired since, for example, feedback regulations no longer exist to the same extent or not at all. In addition it might be advantageously to combine one or more of the sequences indicated in Table IA or IB , columns 5 or 7, lines 279, 282, 285, 286, 288 and/or 289 and/or lines 278, 280, 281, 283, 284 and/or 287 and/or lines 637 to 641, resp., with genes which generally support or enhances to growth or yield of the target organismen, for example genes which lead to faster growth rate of microorganisms or genes which produces stress-, pathogen, or herbicide resistant plants.

**[0228.0.24.24]** In a further embodiment of the process of the invention, therefore, organisms are grown, in which there is simultaneous overexpression of at least one nucleic acid or one of the genes which code for proteins involved in the carotenoid, in particular carotenes metabolism, in particular in synthesis of beta-cryptoxanthin, zeaxanthin, astaxanthin or lutein.

**[0229.0.24.24]** Further advantageous nucleic acid sequences which can be expressed in combination with the sequences used in the process and/or the abovementioned biosynthesis genes are the sequences encoding further genes of the carotenoids biosynthetic pathway, such as phytoene synthase (Psy), which is an important control point for the regulation of the flux (Fraser et al., 2002), phytoene desaturase (Pds), z-carotene desaturase, above mentioned enzymes (s. introduction of the application), e.g. hydroxylases such as beta-carotene hydroxylase (US 6,214,575), ketolases, or cyclases such as the beta-cyclase (US 6,232,530) or oxygenases such as the beta-C4-oxygenase described in US 6,218,599 or homologs thereof, astaxanthin synthase (US 6,365,386), or other genes as described in US 6,150,130. These genes can lead to an increased synthesis of the essential carotenoids, in particular carotene, in particular beta-carotenes.

**[0230.0.0.24] see [230.0.0.0].**

**[0231.0.24.24]** In a further advantageous embodiment of the process of the invention, the organisms used in the process are those in which simultaneously a beta-carotene or IPP degrading protein is attenuated, in particular by reducing the rate of expression of the corresponding gene.

**[0232.0.0.24] to [0276.0.0.24]: see [0232.0.0.0] to [0276.0.0.0]**

**[0277.0.24.24]** The respective fine chemical produced can be isolated from the organism by methods with which the skilled worker are familiar, for example via extraction, salt precipitation, and/or different chromatography methods. The process according to the invention can be conducted batchwise, semibatchwise or continuously. The fine respective chemical, i.e. e.g. beta-carotene or IPP, and other carotenoids, in particular carotenes produced by this process can be obtained by harvesting the organisms, either from the crop in which they grow, or from the field. This can be done via pressing or extraction of the plant parts

**[0278.0.0.24]** to **[0282.0.0.24]: see [0278.0.0.0]** to **[0282.0.0.0]**

**[0283.0.24.24]** Moreover, native polypeptide conferring the increase of the respective fine chemical in an organism or part thereof can be isolated from cells (e.g., endothelial cells), for example using the antibody of the present invention as described below, e.g. an antibody against a protein as indicated in Table IIA or IIB, column 3, lines 279, 282, 285, 286, 288 and/or 289 and/or lines 278, 280, 281, 283, 284 and/or 287 and/or lines 637 to 641, resp., or an antibody against a polypeptide as indicated in Table IIA or IIB, columns 5 or 7, lines 279, 282, 285, 286, 288 and/or 289 and/or lines 278, 280, 281, 283, 284 and/or 287 and/or lines 637 to 641, resp., which can be produced by standard techniques utilizing the polypeptid of the present invention or fragment thereof, i.e., the polypeptide of this invention. Preferred are monoclonal antibodies.

**[0284.0.0.24] see [0284.0.0.0]**

**[0285.0.24.24]** In one embodiment, the present invention relates to a polypeptide having a sequence as indicated in Table IIA or IIB, columns 5 or 7, lines 279, 282, 285, 286, 288 and/or 289 and/or lines 278, 280, 281, 283, 284 and/or 287 and/or lines 637 to 641, resp., or as coded by a nucleic acid molecule as indicated in Table IA or IB , columns 5 or 7, lines 279, 282, 285, 286, 288 and/or 289 and/or lines 278, 280, 281, 283, 284 and/or 287 and/or lines 637 to 641, resp., or functional homologues thereof.

**[0286.0.24.24]** In one advantageous embodiment, in the method of the present invention the activity of a polypeptide is increased which comprises or consists of a consensus sequence as indicated in Table IV, columns 5 or 7, lines 279, 282, 285, 286, 288 and/or 289 and/or lines 278, 280, 281, 283, 284 and/or 287 and/or lines 637 to 641 and in one another embodiment, the present invention relates to a polypeptide comprising or consisting of a consensus sequence as indicated in Table IV, columns 5 or 7, lines 279, 282, 285, 286, 288 and/or 289 and/or lines 278, 280, 281, 283, 284 and/or 287 and/or lines 637 to 641 whereby 20 or less, preferably 15 or 10, preferably 9, 8, 7, or 6, more preferred 5 or 4, even more preferred 3, even more preferred 2, even more preferred 1, most preferred 0 of the amino acids positions indicated can be replaced by any amino acid or, in an further embodiment, can be replaced and/or absent. In one embodiment, the present invention relates to the method of the present invention comprising a polypeptide or to a polypeptide comprising more than one consensus sequences (of an individual line) as indicated in Table IV, column 7, lines 287 to 289 and/or 637 to 641, resp.

**[0287.0.0.24]** to **[0289.0.0.24]: see [0287.0.0.0]** to **[0289.0.0.0]**

**[00290.0.24.24]** The alignment was performed with the Software AlignX (sept 25, 2002) a component of Vector NTI Suite 8.0 , InforMax™,Invitrogen™ life science software, U.S. Main Office, 7305 Executive Way,Frederick, MD 21704,USA with the following settings: For pairwise alignments: gap opening penality: 10,0; gap extension penality 0,1. For multiple alignments: Gap opening penalty: 10,0; Gap extension penalty: 0,1; Gap separation penalty range: 8; Residue substitution matrix: blosum62; Hydrophilic residues: G P S N D Q E K R; Transition weighting: 0,5; Consensus calculation options: Residue fraction for consensus: 0,9. Presettings were selected to allow also for the alignment of conserved amino acids

**[0291.0.24.24]** In one advantageous embodiment, the method of the present invention comprises the increasing of a polypeptide comprising or consisting of plant or microorganism specific consensus sequences.
In one embodiment, said polypeptide of the invention distinguishes over a sequence as indicated in Table IIA or IIB, columns 5 or 7, lines 279, 282, 285, 286, 288, and/or 289 and/or lines 278, 280, 281, 283, 284 and/or 287 and/or lines 637 to 641, resp., by one or more amino acids. In one embodiment, polypeptide distinguishes from a sequence as indicated in Table IIA or IIB, columns 5 or 7, lines 279, 282, 285, 286, 288 and/or 289 and/or lines 278, 280, 281, 283, 284 and/or 287 and/or lines 637 to 641, resp., by more than 5, 6, 7, 8 or 9 amino acids, preferably by more than 10, 15, 20, 25 or 30 amino acids, even more preferred are more than 40, 50, or 60 amino acids and, preferably, the sequence of the polypeptide of the invention distinguishes from a sequence as indicated in Table IIA or IIB, columns 5 or 7, lines 279, 282, 285, 286, 288 and/or 289 and/or lines 278, 280, 281, 283, 284 and/or 287 and/or lines 637 to 641, resp., by not more than 80% or 70% of the amino acids, preferably not more than 60% or 50%, more preferred not more than 40% or 30%, even more preferred not more than 20% or 10%. In an other embodiment, said polypeptide of the invention does not consist of a sequence as indicated in Table IIA or IIB, columns 5 or 7, lines 279, 282, 285, 286, 288 and/or 289 and/or lines 278, 280, 281, 283, 284 and/or 287 and/or lines 637 to 641.

**[0292.0.0.24] see [0292.0.0.0]**

**[0293.0.24.24]** In one embodiment, the invention relates to polypeptide conferring an increase in the fine chemical in an organism or part being encoded by the nucleic acid molecule of the invention or by a nucleic acid molecule used in the process of the invention. In one embodiment, the polypeptide of the invention has a sequence which distinguishes from a sequence as indicated in Table IIA or IIB, columns 5 or 7, lines 279, 282, 285, 286, 288 and/or 289 and/or lines 278, 280, 281, 283, 284 and/or 287 and/or lines 637 to 641, resp., by one or more amino acids. In an other embodiment, said polypeptide of the invention does not consist of the sequence as indicated in Table IIA or IIB, columns 5 or 7, lines 279, 282, 285, 286, 288 and/or 289 and/or lines 278, 280, 281, 283, 284 and/or 287 and/or lines 637 to 641, resp,. In a further embodiment, said polypeptide of the present invention is less than 100%, 99,999%, 99,99%, 99,9% or 99% identical. In one embodiment, said polypeptide does not consist of the sequence encoded by a nucleic acid molecules as indicated in Table IA or IB , columns 5 or 7, lines 279, 282, 285, 286, 288 and/or 289 and/or lines 278, 280, 281, 283, 284 and/or 287 and/or lines 637 to 641, resp..

**[0294.0.24.24]** In one embodiment, the present invention relates to a polypeptide having an activity of a protein as indicated in Table IIA or IIB, column 3, lines 279, 282, 285, 286, 288 and/or 289 and/or lines 278, 280, 281, 283, 284 and/or 287 and/or lines 637 to 641 resp., which distinguishes over a sequence as indicated in Table IIA or IIB, columns 5 or 7, lines 279, 282, 285, 286, 288 and/or 289 and/or lines 278, 280, 281, 283, 284 and/or 287 and/or lines 637 to 641, resp., by one or more amino acids, preferably by more than 5, 6, 7, 8 or 9 amino acids, preferably by more than 10, 15, 20, 25 or 30 amino acids, even more preferred are more than 40, 50, or 60 amino acids but even more preferred by less than 70% of the amino acids, more preferred by less than 50%, even more preferred my less than 30% or 25%, more preferred are 20% or 15%, even more preferred are less than 10%.

**[0295.0.0.24]** to **[0296.0.0.24]: see [0295.0.0.0] to [0296.0.0.0]**

**[0297.0.0.24] see [0297.0.0.0]**

**[00297.1.24.24]** Non-polypeptide of the invention-chemicals are e.g. polypeptides having not the activity of a polypeptide indicated in Table IIA or IIB, columns 3, 5 or 7, lines 278 to 289 and/or lines 637 to 641, resp.

**[0298.0.24.24]** A polypeptide of the invention can participate in the process of the present invention. The polypeptide or a portion thereof comprises preferably an amino acid sequence which is sufficiently homologous to an amino acid sequence as indicated in Table IIA or IIB, columns 5 or 7, lines 279, 282, 285, 286, 288 and/or 289and/or lines 278, 280, 281, 283, 284 and/or 287 and/or lines 637 to 641, resp. The portion of the protein is preferably a biologically active portion as described herein. Preferably, the polypeptide used in the process of the invention has an amino acid sequence identical to a sequence as indicated in Table IIA or IIB, columns 5 or 7, lines 279, 282, 285, 286, 288 and/or 289 and/or lines 278, 280, 281, 283, 284, and/or 287 and/or lines 637 to 641 resp.

**[0299.0.24.24]** Further, the polypeptide can have an amino acid sequence which is encoded by a nucleotide sequence which hybridizes, preferably hybridizes under stringent conditions as described above, to a nucleotide sequence of the nucleic acid molecule of the present invention. Accordingly, the polypeptide has an amino acid sequence which is encoded by a nucleotide sequence that is at least about 35%, 40%, 45%, 50%, 55%, 60%, 65% or 70%, preferably at least about 75%, 80%, 85% or 90, and more preferably at least about 91%, 92%, 93%, 94% or 95%, and even more preferably at least about 96%, 97%, 98%, 99% or more homologous to one of the nucleotide sequence as indicated in Table IA or IB , columns 5 or 7, lines 279, 282, 285, 286, 288 and/or 289 and/or lines 278, 280, 281, 283, 284 and/or 287 and/or lines 637 to 641, resp. The preferred polypeptide of the present invention preferably possesses at least one of the activities according to the invention and described herein. A preferred polypeptide of the present invention includes an amino acid sequence encoded by a nucleotide sequence which hybridizes, preferably hybridizes under stringent conditions, to a nucleotide sequence as indicated in Table IA or IB , columns 5 or 7, lines 279, 282, 285, 286, 288 and/or 289 and/or lines 278, 280, 281, 283, 284 and/or 287 and/or lines 637 to 641, resp., or which is homologous thereto, as defined above.

**[0300.0.24.24]** Accordingly the polypeptide of the present invention can vary from a sequence as indicated in Table IIA or IIB, columns 5 or 7, lines 279, 282, 285, 286, 288 and/or 289 and/or lines 278, 280, 281, 283, 284and/or 287 and/or lines 637 to 641, resp., in amino acid sequence due to natural variation or mutagenesis, as described in detail herein. Accordingly, the polypeptide comprise an amino acid sequence which is at least about 35%, 40%, 45%, 50%, 55%, 60%, 65% or 70%, preferably at least about 75%, 80%, 85% or 90, and more preferably at least about 91%, 92%, 93%, 94% or 95%, and most preferably at least about 96%, 97%, 98%, 99% or more homologous to an entire amino acid sequence of as indicated in Table IIA or IIB, columns 5 or 7, lines 279, 282, 285, 286, 288 and/or 289 and/or lines 278, 280, 281, 283, 284 and/or 287 and/or lines 637 to 641, resp..

**[0301.0.0.24] see [0301.0.0.0]**

**[0302.0.24.24]** Biologically active portions of an polypeptide of the present invention include peptides comprising amino acid sequences derived from the amino acid sequence of the polypeptide of the present invention or used in the process of the present invention, e.g., an amino acid sequence as indicated in Table IIA or IIB, columns 5 or 7, lines 279, 282, 285, 286, 288 and/or 289 and/or lines 278, 280, 281, 283, 284 and/or 287 and/or lines 637 to 641 resp., or the amino acid sequence of a protein homologous thereto, which include fewer amino acids than a full length polypeptide of the present invention or used in the process of the present invention or the full length protein which is homologous to an polypeptide of the present invention or used in the process of the present invention depicted herein, and exhibit at least one activity of polypeptide of the present invention or used in the process of the present invention.

**[0303.0.0.24] see [0303.0.0.0]**

**[0304.0.24.24]** Manipulation of the nucleic acid molecule of the invention may result in the production of a protein having essentially the activity of the polypeptides as indicated in Table IIA or IIB, column 3, lines 279, 282, 285, 286, 288 and/or 289 and/or lines 278, 280, 281, 283, 284 and/or 287 and/or lines 637 to 641 but having differences in the sequence from said wild-type protein. These proteins may be improved in efficiency or activity, may be present in greater numbers in the cell than is usual, or may be decreased in efficiency or activity in relation to the wild type protein.

**[0305.0.0.24]** to **[0308.0.0.24]: see [0305.0.0.0]** to **[0308.0.0.0]**

**[0309.0.0.24]** In one embodiment, a reference to a protein (= polypeptide) of the invention or as indicated in Table IIA or IIB, columns 5 or 7, lines 279, 282, 285, 286, 288 and/or 289 and/or lines 278, 280, 281, 283, 284 and/or 287 and/or lines 637 to 641, resp., refers to a polypeptide having an amino acid sequence corresponding to the polypeptide of the invention or used in the process of the invention , whereas a " non-polypeptide of the invention" or "other polypeptide" not being indicated in Table IIA or IIB, columns 5 or 7, lines 279, 282, 285, 286, 288 and/or 289 and/or lines 278, 280, 281, 283, 284 and/or 287 and/or lines 637 to 641, resp., refers to a polypeptide having an amino acid sequence corresponding to a protein which is not substantially homologous a polypeptide of the invention, preferably which is not substantially homologous to a polypeptide as indicated in Table IIA or IIB, columns 5 or 7, lines 279, 282, 285, 286, 288 and/or 289 and/or lines 278, 280, 281, 283, 284 and/or 287 and/or lines 637 to 641, resp., e.g., a protein which does not confer the activity described herein or annotated or known for as indicated in Table IIA or IIB, column 3, lines 279, 282, 285, 286, 288 and/or 289 and/or lines 278, 280, 281, 283, 284 and/or 287 and/or lines 637 to 641, resp., and which is derived from the same or a different organism. In one embodiment, a "non-polypeptide of the invention" or "other polypeptide" not being indicated in Table IIA or IIB, columns 5 or 7, lines 279, 282, 285, 286, 288 and/or 289 and/or lines 278, 280, 281, 283, 284 and/or 287 and/or lines 637 to 641, resp., does not confer an increase of the respective fine chemical in an organism or part thereof.

**[0310.0.0.24] to [0334.0.0.24]: see [0310.0.0.0] to [0334.0.0.0]**

**[0335.0.24.24]** The dsRNAi method has proved to be particularly effective and advantageous for reducing the expression of a nucleic acid sequences as indicated in Table IA or IB , columns 5 or 7, lines 279, 282, 285, 286, 288 and/or 289 and/or lines 278, 280, 281, 283, 284 and/or 287 and/or lines 637 to 641, resp., and/or homologs thereof. As described inter alia in WO 99/32619, dsRNAi approaches are clearly superior to traditional antisense approaches. The invention therefore furthermore relates to double-stranded RNA molecules (dsRNA molecules) which, when introduced into an organism, advantageously into a plant (or a cell, tissue, organ or seed derived there from), bring about altered metabolic activity by the reduction in the expression of a nucleic acid sequences as indicated in Table IA or IB , columns 5 or 7, lines 279, 282, 285, 286, 288 and/or 289 and/or lines 278, 280, 281, 283, 284 and/or 287 and/or lines 637 to 641, resp., and/or homologs thereof. In a double-stranded RNA molecule for reducing the expression of an protein encoded by a nucleic acid sequence sequences as indicated in Table IA or IB , columns 5 or 7, lines 279, 282, 285, 286, 288 and/or 289 and/or lines 278, 280, 281, 283, 284 and/or 287 and/or lines 637 to 641, resp., and/or homologs thereof, one of the two RNA strands is essentially identical to at least part of a nucleic acid sequence, and the respective other RNA strand is essentially identical to at least part of the complementary strand of a nucleic acid sequence.

**[0336.0.0.24] to [0342.0.0.24]: see [0336.0.0.0] to [0342.0.0.0]**

**[0343.0.24.24]** As has already been described, 100 % sequence identity between the dsRNA and a gene transcript of a nucleic acid sequence as indicated in Table IA or IB , columns 5 or 7, lines 279, 282, 285, 286, 288, and/or 289 and/or lines 278, 280, 281, 283, 284 and/or 287 and/or lines 637 to 641, resp., or it's homolog is not necessarily required in order to bring about effective reduction in the expression. The advantage is, accordingly, that the method is tolerant with regard to sequence deviations as may be present as a consequence of genetic mutations, polymorphisms or evolutionary divergences. Thus, for example, using the dsRNA, which has been generated starting from a sequence as indicated in Table IA or IB , columns 5 or 7, lines 279, 282, 285, 286, 288 and/or 289and/or lines 278, 280, 281, 283, 284 and/or 287 and/or lines 637 to 641, resp., or homologs thereof of the one organism, may be used to suppress the corresponding expression in another organism.

**[0344.0.0.24] to [0350.0.0.24]: see [0344.0.0.0] to [0350.0.0.0]**

**[0351.0.0.24]** to **[0361.0.0.24]: see [0351.0.0.0]** to **[0361.0.0.0]**

**[0362.0.24.24]** Accordingly the present invention relates to any cell transgenic for any nucleic acid characterized as part of the invention, e.g. conferring the increase of the respective fine chemical in a cell or an organism or a part thereof, e.g. the nucleic acid molecule of the invention, the nucleic acid construct of the invention, the antisense molecule of the invention, the vector of the invention or a nucleic acid molecule encoding the polypeptide of the invention, e.g. the polypeptide as indicated in Table IIA or IIB, columns 5 or 7, lines 279, 282, 285, 286, 288 and/or 289 and/or lines 278, 280, 281, 283, 284 and/or 287 and/or lines 637 to 641, resp., e.g. encoding a polypeptide having protein activity, as indicated in Table IIA or IIB, columns 3, lines 279, 282, 285, 286, 288 and/or 289 and/or lines 278, 280, 281, 283, 284 and/or 287 and/or lines 637 to 641, resp. Due to the above mentioned activity the respective fine chemical content in a cell or an organism is increased. For example, due to modulation or manipulation, the cellular activity of the polypeptide of the invention or nucleic acid molecule of the invention is increased, e.g. due to an increased expression or specific activity of the subject matters of the invention in a cell or an organism or a part thereof. Transgenic for a polypeptide having an activity of a polypeptide as indicated in Table IIA or IIB, columns 5 or 7, lines 279, 282, 285, 286, 288 and/or 289 and/or lines 278, 280, 281, 283, 284 and/or 287 and/or lines 637 to 641, resp., means herein that due to modulation or manipulation of the genome, an activity as annotated for a polypeptide as indicated in Table IIA or IIB, column 3, lines 279, 282, 285, 286, 288 and/or 289 and/or lines 278, 280, 281, 283, 284 and/or 287 and/or lines 637 to 641, e.g. having a sequence as indicated in Table IIA or IIB, columns 5 or 7, lines 279, 282, 285, 286, 288 and/or 289 and/or lines 278, 280, 281, 283, 284 and/or 287 and/or lines 637 to 641, resp., is increased in a cell or an organism or a part thereof. Examples are described above in context with the process of the invention.

**[0363.0.0.24] see [0363.0.0.0]**

**[0364.0.24.24]** A naturally occurring expression cassette - for example the naturally occurring combination of a promoter of a gene encoding a polypeptide of the invention as indicated in Table IIA or IIB, column 3, 5 or 7, lines 279, 282, 285, 286, 288 and/or 289 and/or lines 278, 280, 281, 283, 284 and/or 287 and/or lines 637 to 641, resp., with the corresponding protein-encoding sequence as indicated in Table IA or IB , column 5 or 7 , lines 279, 282, 285, 286, 288 and/or 289 and/or lines 278, 280, 281, 283, 284 and/or 287 and/or lines 637 to 641, resp., - becomes a transgenic expression cassette when it is modified by non-natural, synthetic "artificial" methods such as, for example, mutagenization. Such methods have been described (US 5,565,350; WO 00/15815; also see above).

**[0365.0.0.24] to [0373.0.0.24]: see [0365.0.0.0] to [0373.0.0.0]**

**[0374.0.24.24]** Transgenic plants comprising the respective fine chemical synthesized in the process according to the invention can be marketed directly without isolation of the compounds synthesized. In the process according to the invention, plants are understood as meaning all plant parts, plant organs such as leaf, stalk, root, tubers or seeds or propagation material or harvested material or the intact plant. In this context, the seed encompasses all parts of the seed such as the seed coats, epidermal cells, seed cells, endosperm or embryonic tissue. The respective fine chemical produced in the process according to the invention may, however, also be isolated from the plant in the form of their free beta-carotene or free IPP or bound in or to compounds or moieties, like glucosides, e.g. diglucosides. The respective fine chemical produced by this process can be harvested by harvesting the organisms either from the culture in which they grow or from the field. This can be done via expressing, grinding and/or extraction, salt precipitation and/or ion-exchange chromatography or other chromatographic methods of the plant parts, preferably the plant seeds, plant fruits, plant tubers and the like.

**[0375.0.0.24] to [0376.0.0.24]: see [0375.0.0.0] to [0376.0.0.0]**

**[0377.0.24.24]** Accordingly, the present invention relates also to a process according to the present invention whereby the produced carotenoids, e.g the fine chemical(s) comprising composition is isolated. In one embodiment, the produced respective fine chemical is isolated.

**[0378.0.24.24]** In this manner, more than 50% by weight, advantageously more than 60% by weight, preferably more than 70% by weight, especially preferably more than 80% by weight, very especially preferably more than 90% by weight, of the respective fine chemical produced in the process can be isolated. The resulting respective fine chemical can, if appropriate, subsequently be further purified, if desired mixed with other active ingredients such as vitamins, amino acids, carbohydrates, antibiotics and the like, and, if appropriate, formulated.

**[0379.0.24.24]** In one embodiment, the product of the process is a mixture of the fine chemicals. In one embodiment, the product is a mixture of the respective fine chemicals with other carotenoids.

**[0380.0.24.24]** The respective fine chemicals obtained in the process are suitable as starting material for the synthesis of further products of value. For example, they can be used in combination with each other or alone for the production of pharmaceuticals, foodstuffs, animal feeds or cosmetics. Accordingly, the present invention relates to a method for the production of pharmaceuticals, food stuff, animal feeds, nutrients or cosmetics comprising the steps of the process according to the invention, including the isolation of the respective fine chemical containing composition produced by the process of the invention or the respective fine chemical produced by the process of the invention - if desired - and formulating the product with a pharmaceutical or cosmetic acceptable carrier or formulating the product in a form acceptable for an application in agriculture. A further embodiment according to the invention is the use of the the respective fine chemical produced in the process or of the transgenic organisms in animal feeds, foodstuffs, medicines, food supplements, cosmetics or pharmaceuticals or for the production of other carotenoids, e.g. in after isolation of the respective fine chemical or without, e.g. in situ, e.g within the organism used for the process for the production of the respective fine chemical.

**[0381.0.0.24]** to **[0382.0.0.24]: see [0381.0.0.0]** to **[0382.0.0.0]**

**[0383.0.24.24]**./.

**[0384.0.0.24] see [0384.0.0.0]**

**[0385.0.24.24]** The fermentation broths obtained in this way, containing in particular IPP or beta-carotene in mixtures with other carotenoids, in particular with other carotenoids, in particular carotenes, or containing microorganisms or parts of microorganisms, like plastids, containing the respective fine chemical or the carotenoids produced in mixtures with other carotenoids, in particular with other carotene, normally have a dry matter content of from 1 to 70% by weight, preferably 7.5 to 25% by weight. Sugar-limited fermentation is additionally advantageous, e.g. at the end, for example over at least 30% of the fermentation time. This means that the concentration of utilizable sugar in the fermentation medium is kept at, or reduced to, 0 to 10 g/l, preferably to 0 to 3 g/I during this time. The fermentation broth is then processed further. Depending on requirements, the biomass can be removed or isolated entirely or partly by separation methods, such as, for example, centrifugation, filtration, decantation, coagulation/flocculation or a combination of these methods, from the fermentation broth or left completely in it.
The fermentation broth can then be thickened or concentrated by known methods, such as, for example, with the aid of a rotary evaporator, thin-film evaporator, falling film evaporator, by reverse osmosis or by nanofiltration. This concentrated fermentation broth can then be worked up by freeze-drying, spray drying, spray granulation or by other processes.
As carotenoids are often localized in membranes or plastids, in one embodiment it is advantageous to avoid a leaching of the cells when the biomass is isolated entirely or partly by separation methods, such as, for example, centrifugation, filtration, decantation, coagulation/flocculation or a combination of these methods, from the fermentation broth. The dry biomass can directly be added to animal feed, provided the carotenoids concentration is sufficiently high and no toxic compounds are present. In view of the instability of carentoids, conditions for drying, e.g. spray or flash-drying, can be mild and can be avoiding oxidation and *cis*/*trans* isomerization. For example antioxidants, e.g. BHT, ethoxyquin or other, can be added. In case the carotenoids concentration in the biomass is to dilute, solvent extraction can be used for their isolation, e.g. with alcohols, ether or other organic solvents, e.g. with methanol, ethanol, aceton, alcoholic potassium hydroxide, glycerol-phenol, liquefied phenol or for example with acids or bases, like trichloroacetatic acid or potassium hydroxide. A wide range of advantageous Methods and techniques for the isolation of carotenoids, in particular of the respective fine chemical, in particular of IPP or beta-carotine can be found in the state of the art. In case phenol is used it can for example be removed with ether and water extraction and the dry eluate comprises a mixture of the carotenoids of the biomass.

**[0386.0.24.24]** Accordingly, it is possible to further purify the respective fine chemicals or other carotenoids, in particular the carotenes produced according to the invention. For this purpose, the product-containing composition, e.g. a total or partial lipid extraction fraction using organic solvents, e.g. as described above, is subjected for example (without meaning to be limited:) to a saponification to remove triglycerides, partition between e.g. hexane/methanol (separation of non-polar epiphase from more polar hypophasic derivates) and separation via e.g. an open column chromatography or HPLC in which case the desired product or the impurities are retained wholly or partly on the chromatography resin. These chromatography steps can be repeated if necessary, using the same or different chromatography resins. The skilled worker is familiar with the choice of suitable chromatography resins and their most effective use.

**[0387.0.0.24]** to **[0392.0.0.24]: see [0387.0.0.0]** to **[0392.0.0.0]**

**[0393.0.24.24]** In one embodiment, the present invention relates to a method for the identification of a gene product conferring an increase in the respective fine chemical production in a cell, comprising the following steps:
(f) contacting,̅ e.g. hybridising, the nucleic acid molecules of a sample, e.g. cells, tissues, plants or microorganisms or a nucleic acid library , which can contain a candidate gene encoding a gene product conferring an increase in the respective fine chemical after expression, with the nucleic acid molecule of the present invention;
(g) identifying the nucleic acid molecules, which hybridize under relaxed stringent conditions with the nucleic acid molecule of the present invention in particular to the nucleic acid molecule sequence as indicated in Table IA or IB , columns 5 or 7, lines 279, 282, 285, 286, 288 and/or 289 and/or lines 278, 280, 281, 283, 284 and/or 287 and/or lines 637 to 641, resp., and, optionally, isolating the full length cDNA clone or complete genomic clone;
(h) introducing the candidate nucleic acid molecules in host cells, preferably in a plant cell or a microorganism, appropriate for producing the respective fine chemical;
(i) expressing the identified nucleic acid molecules in the host cells;
(j) assaying the respective fine chemical level in the host cells; and
(k) identifying the nucleic acid molecule and its gene product which expression confers an increase in the respective fine chemical level in the host cell after expression compared to the wild type.

**[0394.0.0.24] to [0398.0.0.24]: see [0394.0.0.0] to [0398.0.0.0]**

**[0399.0.24.24]** Furthermore, in one embodiment, the present invention relates to process for the identification of a compound conferring increased the respective fine chemical production in a plant or microorganism, comprising the steps:
(d) culturing a cell or tissue or microorganism or maintaining a plant expressing the polypeptide according to the invention or a nucleic acid molecule encoding said polypeptide and a readout system capable of interacting with the polypeptide under suitable conditions which permit the interaction of the polypeptide with said readout system in the presence of a compound or a sample comprising a plurality of compounds and capable of providing a detectable signal in response to the binding of a compound to said polypeptide under conditions which permit the expression of said readout system and the polypeptide of the present invention or used in the process of the invention; and
(e) identifying if the compound is an effective agonist by detecting the presence or absence or increase of a signal produced by said readout system.
The screen for a gene product or an agonist conferring an increase in the respective fine chemical production can be performed by growth of an organism for example a microorganism in the presence of growth reducing amounts of an inhibitor of the synthesis of the respective fine chemical. Better growth, e.g. higher dividing rate or high dry mass in comparison to the control under such conditions would identify a gene or gene product or an agonist conferring an increase in respective fine chemical production.

**[00399.1.24.24]** One can think to screen for increased production of the respective fine chemical by for example searching for a resistance to a drug blocking the synthesis of the respective fine chemical and looking whether this effect is dependent on the activity or expression of a polypeptide as indicated in Table IIA or IIB, columns 5 or 7, lines 279, 282, 285, 286, 288 and/or 289 and/or lines 278, 280, 281, 283, 284 and/or 287 and/or lines 637 to 641 or a homolog thereof, e.g. comparing the phenotype of nearly identical organisms with low and high activity of a protein as indicated in Table IIA or IIB, columns 5 or 7, lines 279, 282, 285, 286, 288 and/or 289 and/or lines 278, 280, 281, 283, 284 and/or 287 and/or lines 637 to 641 after incubation with the drug.

**[0400.0.0.24]** to **[0415.0.0.24]: see [0400.0.0.0]** to **[0415.0.0.0]**

**[0416.0.0.24] see [0416.0.0.0]**

**[0417.0.24.24]** The nucleic acid molecule of the invention, the vector of the invention or the nucleic acid construct of the invention may also be useful for the production of organisms resistant to inhibitors of the carotenoids, in particular carotene production biosynthesis pathways. In particular, the overexpression of the polypeptide of the present invention or the polypeptide described for the process of the invention may protect an organism such as a microorganism or a plant against inhibitors, which block the carotenoid or carotene synthesis, in particular the respective fine chemical synthesis in said organism. Examples of inhibitors or herbicides blocking the synthesis of carotenoids in organism such as microorganism or plants are for example classified in two groups: The first group consists of inhibitors that cause the accumulation of early intermediates in the pathway, particularly the colorless phytene, e.g. diphenylamine. Other inhibitors preferentially block late reactions in the pathway, notably the cyclization of lycopene. Inhibitors are e.g. nicotine, 2-(4-chlorophenylthio)-triethylamine and other substituted amines as well as nitrogenous heterocyclic bases, e.g. imidazole.
As carotene may protect organisms against damages of oxidative stress, especially singlet oxygens, a increased level of the respective respective fine chemical can protect plants against herbicides which cause the toxic build-up of oxidative compounds, e.g. singlet oxygen. For example, inhibition of the protoporphorineogen oxidase (Protox), an enzyme important in the synthesis of chlorophyll and heme biosynthesis results in the loss of chlorophyll and carotenoids and in leaky membranes; the membrane destruction is due to creation of free oxygen radicals (which is also reported for other classic photosynthetic inhibitor herbicides).
Accordingly, in one embodiment, the increase of the level of the respective fine chemical is used to protect plants against herbicides destroying membranes due to the creation of free oxygen radicals.
Examples of inhibitors or herbicides building up oxidative stress are aryl triazion, e.g. sulfentrazone, carfentrazone, or diphenylethers, e.g. acifluorfen, lactofen, or oxyfluorfen, or N-Phenylphthalimide, e.g. flumiclorac or flumioxazin, substituted ureas, e.g. fluometuron, tebuthiuron, or diuron, linuron, or triazines, e.g. atrazine, prometryn, ametryn, metributzin, prometon, simazine, or hexazinone, or uracils, e.g. bromacil or terbacil.
Carotenoid inhibitors are e.g. Pyridines and Pyridazinones, e.g. norflurazon, fluridone or dithiopyr. Thus, in one embodiment, the present invention relates to the use of an increase of the respective fine chemical according to the present invention for the protection of plants against carotenoids inhibitors as pyridines and pyridazinones.

**[0418.0.0.24]** to **[0423.0.0.24]: see [0418.0.0.0]** to **[0423.0.0.0]**

**[0424.0.24.24]** Accordingly, the nucleic acid of the invention, the polypeptide of the invention, the nucleic acid construct of the invention, the organisms, the host cell, the microorgansims, the plant, plant tissue, plant cell, or the part thereof of the invention, the vector of the invention, the agonist identified with the method of the invention, the nucleic acid molecule identified with the method of the present invention, can be used for the production of the respective fine chemical or of the respective fine chemical and one or more other carotenoids, in particular other carotene, e.g. astaxanthin, alpha or gamma-carotene etc..
Accordingly, the nucleic acid of the invention, or the nucleic acid molecule identified with the method of the present invention or the complement sequences thereof, the polypeptide of the invention, the nucleic acid construct of the invention, the organisms, the host cell, the microorgansims, the plant, plant tissue, plant cell, or the part thereof of the invention, the vector of the invention, the antagonist identified with the method of the invention, the antibody of the present invention, the antisense molecule of the present invention, can be used for the reduction of the respective fine chemical in a organism or part thereof, e.g. in a cell.

**[0424.1.0.24]** In a further embodiment the present invention relates to the use of the antagonist of the present invention, the plant of the present invention or a part thereof, the microorganism or the host cell of the present invention or a part thereof for the production a cosmetic composition or a pharmaceutical composition. Such a composition has antioxidative activity, photoprotective activity, tanning activity, can be used for the treating of high levels of cholesterol and/or lipids, can be used to protect, treat or heal the above mentioned diseases, e.g. retinal disorders, hyperholsterolemia, hyperlipidemia, and ahterosclerosis, or can be used for the cleaning, conditioning, and/or treating of the skin, e.g. if combined with a pharmaceutically or cosmetically acceptable carrier.
The respective fine chemical, in particular beta-carotene can be also used as stabilizer of other colours or oxygen sensitive compounds.

**[0425.0.0.24]** to **[0434.0.0.24]: see [0425.0.0.0]** to **[0434.0.0.0]**

**[0435.0.24.24]** Example 3: In-vivo and in-vitro mutagenesis

**[0436.0.24.24]** An *in vivo* mutagenesis of organisms such as green algae (e.g. Spongiococcum sp, e.g. Spongiococcum exentricum, Chlorella sp.), Saccharomyces, Mortierella, Escherichia and others mentioned above, which are beneficial for the production of carotenoid, e.g. carotene, in particular beta-carotene, or its precursour IPP, can be carried out by passing a plasmid DNA (or another vector DNA) containing the desired nucleic acid sequence or nucleic acid sequences, e.g. the nucleic acid molecule of the invention or the vector of the invention, through E. coli and other microorganisms (for example Bacillus spp. or yeasts such as Saccharomyces cerevisiae) which are not capable of maintaining the integrity of its genetic information. Usual mutator strains have mutations in the genes for the DNA repair system [for example mutHLS, mutD, mutT and the like; for comparison, see Rupp, W.D. (1996) DNA repair mechanisms in Escherichia coli and Salmonella, pp. 2277-2294, ASM: Washington]. The skilled worker knows these strains. The use of these strains is illustrated for example in Greener, A. and Callahan, M. (1994) Strategies 7; 32-34.
In-vitro mutation methods such as increasing the spontaneous mutation rates by chemical or physical treatment are well known to the skilled person. Mutagens like 5-bromo-uracil, N-methyl-N-nitro-N-nitrosoguanidine (= NTG), ethyl methanesulfonate (= EMS), hydroxylamine and/or nitrous acid are widly used as chemical agents for random in-vitro mutagensis. The most common physical method for mutagensis is the treatment with UV irradiation. Another random mutagenesis technique is the error-prone PCR for introducing amino acid changes into proteins. Mutations are deliberately introduced during PCR through the use of error-prone DNA polymerases and special reaction conditions known to a person skilled in the art. For this method randomized DNA sequences are cloned into expression vectors and the resulting mutant libraries screened for altered or improved protein activity as described below.
Site-directed mutagensis method such as the introduction of desired mutations with an M13 or phagemid vector and short oligonucleotides primers is a well-known approach for site-directed mutagensis. The clou of this method involves cloning of the nucleic acid sequence of the invention into an M13 or phagemid vector, which permits recovery of single-stranded recombinant nucleic acid sequence. A mutagenic oligonucleotide primer is then designed whose sequence is perfectly complementary to nucleic acid sequence in the region to be mutated, but with a single difference: at the intended mutation site it bears a base that is complementary to the desired mutant nucleotide rather than the original. The mutagenic oligonucleotide is then allowed to prime new DNA synthesis to create a complementary full-length sequence containing the desired mutation. Another site-directed mutagensis method is the PCR mismatch primer mutagensis method also known to the skilled person. Dpnl site-directed mutagensis is a further known method as described for example in the Stratagene QuickchangeTM site-directed mutagenesis kit protocol. A huge number of other methods are also known and used in common practice.
Positive mutation events can be selected by screening the organisms for the production of the desired respective fine chemical.

**[0437.0.24.24]** Example 4: DNA transfer between Escherichia coli, Saccharomyces cerevisiae and Mortierella alpine

**[0438.0.24.24]** Shuttle vectors such as pYE22m, pPAC-ResQ, pClasper, pAUR224, pAMH10, pAML10, pAMT10, pAMU10, pGMH10, pGML10, pGMT10, pGMU10, pPGAL1, pPADH1, pTADH1, pTAex3, pNGA142, pHT3101 and derivatives thereof which allow the transfer of nucleic acid sequences between Escherichia coli, Saccharomyces cerevisiae and/or Mortierella alpina are available to the skilled worker. An easy method to isolate such shuttle vectors is disclosed by Soni R. and Murray J.A.H. [Nucleic Acid Research, vol. 20 no. 21, 1992: 5852]: If necessary such shuttle vectors can be constructed easily using standard vectors for E. coli (Sambrook, J. et al., (1989), "Molecular Cloning: A Laboratory Manual", Cold Spring Harbor Laboratory Press or Ausubel, F.M. et al. (1994) "Current Protocols in Molecular Biology", John Wiley & Sons) and/or the aforementioned vectors, which have a replication origin for, and suitable marker from, Escherichia coli, Saccharomyces cerevisiae or Mortierella alpina added. Such replication origins are preferably taken from endogenous plasmids, which have been isolated from species used in the inventive process. Genes, which are used in particular as transformation markers for these species are genes for kanamycin resistance (such as those which originate from the Tn5 or Tn-903 transposon) or for chloramphenicol resistance (Winnacker, E.L. (1987) "From Genes to Clones - Introduction to Gene Technology, VCH, Weinheim) or for other antibiotic resistance genes such as for G418, gentamycin, neomycin, hygromycin or tetracycline resistance.

**[0439.0.24.24]** Using standard methods, it is possible to clone a gene of interest into one of the above-described shuttle vectors and to introduce such hybrid vectors into the microorganism strains used in the inventive process. The transformation of Saccharomyces can be achieved for example by LiCl or sheroplast transformation (Bishop et al., Mol. Cell. Biol., 6, 1986: 3401 - 3409; Sherman et al., Methods in Yeasts in Genetics, [Cold Spring Harbor Lab. Cold Spring Harbor, N. Y.] 1982, Agatep et al., Technical Tips Online 1998, 1:51: P01525 or Gietz et al., Methods Mol. Cell. Biol. 5, 1995: 255f) or electroporation (Delorme E., Appl. Environ. Microbiol., vol. 55, no. 9, 1989 : 2242 - 2246).

**[0440.0.24.24]** If the transformed sequence(s) is/are to be integrated advantageously into the genome of the microorganism used in the inventive process for example into the yeast or fungi genome, standard techniques known to the skilled worker also exist for this purpose. Solinger et al. (Proc Natl Acad Sci U S A., 2001 (15): 8447-8453) and Freedman et al. (Genetics, Vol. 162, 15-27, September 2002,) teaches a homolog recombination system dependent on rad 50, rad51, rad54 and rad59 in yeasts. Vectors using this system for homologous recombination are vectors derived from the Ylp series. Plasmid vectors derived for example from the 2µ-Vector are known by the skilled worker and used for the expression in yeasts. Other preferred vectors are for example pART1, pCHY21 or pEVP11 as they have been described by McLeod et al. (EMBO J. 1987, 6:729-736) and Hoffman et al. (Genes Dev. 5, 1991: :561-571.) or Russell et al. (J. Biol. Chem. 258, 1983: 143-149.). Other beneficial yeast vectors are plasmids of the REP, REP-X, pYZ or RIP series.

**[0441.0.0.24]** see **[0441.0.0.0]**

**[0442.0.0.24] see [0442.0.0.0]**

**[0443.0.0.24] see [0443.0.0.0]**

**[0444.0.24.24]** Example 6: Growth of genetically modified organism: media and culture conditions

**[0445.0.24.24]** Genetically modified Yeast, Mortierella or Escherichia coli are grown in synthetic or natural growth media known by the skilled worker. A number of different growth media for Yeast, Mortierella or Escherichia coli are well known and widely available. A method for clulturing Mortierella is disclosed by Jang et al. [Bot. Bull. Acad. Sin. (2000) 41: 41-48]. Mortierella can be grown at 20°C in a culture medium containing: 10 g/I glucose, 5 g/l yeast extract at pH 6.5. Futhermore Jang et al. teaches a submerged basal medium containing 20 g/l soluble starch, 5 g/l Bacto yeast extract, 10 g/l KNO₃, 1 g/l KH₂PO₄, and 0.5 g/l MgSO₄•7H₂O, pH 6.5.

**[0446.0.0.24]** to **[0450.0.0.24]: see [0446.0.0.0]** to **[0450.0.0.0]**

**[0451.0.0.24] see [0451.0.5.5]**

**[0452.0.0.24]** to **[0454.0.0.24]: see [0452.0.0.0]** to **[0454.0.0.0]**

**[0455.0.24.24]** The effect of the genetic modification in plants, fungi, algae, ciliates or on the production of a desired compound (such as a fatty acid) can be determined by growing the modified microorganisms or the modified plant under suitable conditions (such as those described above) and analyzing the medium and/or the cellular components for the elevated production of desired product (i.e. of the lipids or a fatty acid). These analytical techniques are known to the skilled worker and comprise spectroscopy, thin-layer chromatography, various types of staining methods, enzymatic and microbiological methods and analytical chromatography such as high-performance liquid chromatography (see, for example, Ullman, Encyclopedia of Industrial Chemistry, Vol. A2, p. 89-90 and p. 443-613, VCH: Weinheim (1985); Fallon, A., et al., (1987) "Applications of HPLC in Biochemistry" in: Laboratory Techniques in Biochemistry and Molecular Biology, Vol. 17; Rehm et al. (1993) Biotechnology, Vol. 3, Chapter III: "Product recovery and purification", p. 469-714, VCH: Weinheim; Belter, P.A., et al. (1988) Bioseparations: downstream processing for Biotechnology, John Wiley and Sons; Kennedy, J.F., and Cabral, J.M.S. (1992) Recovery processes for biological Materials, John Wiley and Sons; Shaeiwitz, J.A., and Henry, J.D. (1988) Biochemical Separations, in: Ullmann's Encyclopedia of Industrial Chemistry, Vol. B3; Chapter 11, p. 1-27, VCH: Weinheim; and Dechow, F.J. (1989) Separation and purification techniques in biotechnology, Noyes Publications).

In addition to the abovementioned processes, plant lipids are extracted from plant material as described by Cahoon et al. (1999) Proc. Natl. Acad. Sci. USA 96 (22): 12935-12940 and Browse et al. (1986) Analytic Biochemistry 152:141-145. The qualitative and quantitative analysis of lipids is described by Christie, William W., Advances in Lipid Methodology, Ayr/Scotland: Oily Press (Oily Press Lipid Library; 2); Christie, William W., Gas Chromatography and Lipids. A Practical Guide - Ayr, Scotland: Oily Press, 1989, Repr. 1992, IX, 307 pp. (Oily Press Lipid Library; 1); "Progress in Lipid Research, Oxford: Pergamon Press, 1 (1952) - 16 (1977) under the title: Progress in the Chemistry of Fats and Other Lipids CODEN.

**[0456.0.0.24]: see [0456.0.0.0]**

**[0457.0.24.24]** Example 9: Purification of the Carotene and determination of the content

**[0458.0.24.24]** Abbreviations:; GC-MS, gas liquid chromatography/mass spectrometry; TLC, thin-layer chromatography.

The unambiguous detection for the presence of beta-carotene or IPP can be obtained by analyzing recombinant organisms using analytical standard methods: LC, LC-MSMS or TLC, as described The total carotene produced in the organism for example in yeasts used in the inventive process can be analysed for example according to the following procedure:
The material such as yeasts, E. coli or plants to be analyzed can be disrupted by sonication, grinding in a glass mill, liquid nitrogen and grinding or via other applicable methods.
Plant material is initially homogenized mechanically by comminuting in a pestle and mortar to make it more amenable to extraction.
A typical sample pretreatment consists of a total lipid extraction using such polar organic solvents as acetone or alcohols as methanol, or ethers, saponification , partition between phases, seperation of non-polar epiphase from more polar hypophasic derivatives and chromatography. E.g.:
For analysis, solvent delivery and aliquot removal can be accomplished with a robotic system comprising a single injector valve Gilson 232XL and a 402 2S1V diluter [Gilson, Inc. USA, 3000 W. Beltline Highway, Middleton, WI]. For saponification, 3 ml of 50% potassium hydroxide hydro-ethanolic solution (4 water:1 ethanol) can be added to each vial, followed by the addition of 3 ml of octanol. The saponification treatment can be conducted at room temperature with vials maintained on an IKA HS 501 horizontal shaker [Labworld-online, Inc., Wilmington, NC] for fifteen hours at 250 movements/minute, followed by a stationary phase of approximately one hour.
Following saponification, the supernatant can be diluted with 0.24 ml of methanol. The addition of methanol cqan be conducted under pressure to ensure sample homogeneity. Using a 0.25 ml syringe, a 0.1 ml aliquot can be removed and transferred to H PLC vials for analysis.
For HPLC analysis, a Hewlett Packard 1100 HPLC, complete with a quaternary pump, vacuum degassing system, six-way injection valve, temperature regulated autosampler, column oven and Photodiode Array detector can be used [Agilent Technologies available through Ultra Scientific Inc., 250 Smith Street, North Kingstown, RI]. The column can be a Waters YMC30, 5-micron, 4.6 x 250 mm with a guard column of the same material [Waters, 34 Maple Street, Milford, MA]. The solvents for the mobile phase can be 81 methanol: 4 water: 15 tetrahydrofuran (THF) stabilized with 0.2% BHT (2,6-di-tert-butyl-4-methylphenol). Injections were 20 I. Separation can be isocratic at 30°C with a flow rate of 1.7 ml/minute. The peak responses can be measured by absorbance at 447 nm.
Carotenoid compositions can be determined for wild-type and mutant samples selected from those identified in a screening procedure. Petal samples can be stored in a - 80°C freezer until mutants were identified. Samples can be lyophilized, and the dried tissue can be stored under argon at -80°C until ready for analysis.
Extraction procedures can be performed under red light. Dried petals can be ground to pass through a No. 40 sieve mesh size. A ground sample can be accurately weighed and transferred into a 100 ml red volumetric flask. To the sample, 500 microliters l) of H₂O can be added, and the mixture can be swirled for 1 minute. Thirty ml of extractant solvent (10 ml hexane + 7 ml acetone + 6 ml absolute alcohol + 7 ml toluene) can be added, and the flask can be shaken at 160 rpm for 10 minutes.
For saponification, 2 ml of 40% methanolic KOH can be added into the flask, which can be then swirled for one minute. The flask can be placed in a 56°C H₂O bath for 20 minutes. An air condenser can be attached to prevent loss of solvent. The sample can be cooled in the dark for one hour with the condenser attached. After cooling, 30 ml of hexane can be added, and the flask can be shaken at 160 rpm for 10 minutes.
The shaken sample can be diluted to volume (100 ml) with 10% sodium sulfate solution and shaken vigorously for one minute. The sample can be remained in the dark for at least 30 minutes. A 35 ml aliquot can be removed from the approximately 50 ml upper phase, and transferred to a sample cup. An additional 30 ml of hexane can be added into the flask that can be then shaken at 160 rpm for 10 minutes. After approximately one hour, the upper phases can be combined. For HPLC analysis, 10 ml aliquots can be dried under nitrogen and stored under argon at -80°C.
HPLC equipment comprised an Alliance 2690 equipped with a refrigerated autosampler, column heater and a Waters Photodiode Array 996 detector (Waters Corp., 34 Maple Street Milford, MA 01757). Separation can be obtained with a YMC30 column, 3 m, 2.0 x 150 mm with a guard column of the same material. Standards can be obtained from ICC Indorespective fine chemicals Somerville, New Jersey 088876 and from DHI-Water & Environment, DK -2970 Horsholm, Denmark.
The dried mutant samples can be resuspended in tetrahydrofuran and methanol to a total volume of 200 I and filtered, whereas the control can be not additionally concentrated. Carotenoids can be separated using a gradient method. Initial gradient conditions can be 90% methanol: 5% water: 5% methyl tert-butyl ether at a flow rate of 0.4 milliliters per minute (ml/min). From zero to 15 minutes, the mobile phase can be changed from the initial conditions to 80 methanol: 5 water: 15 methyl tert-butyl ether, and from 15 to 60 minutes to 20 methanol: 5 water: 75 methyl tert-butyl ether. For the following 10 minutes, the mobile phase can be returned to the initial conditions and the column equilibrated for an additional 10 minutes. The column temperature can be maintained at 27°C and the flow rate was 0.4 ml/minute. Injections were 10 I. The majority of peak responses can be measured at 450 nm and additional areas added from 286, 348, 400 and 472 nm extracted channels.

**[0459.0.24.24]** If required and desired, further chromatography steps with a suitable resin may follow. Advantageously, the carotene can be further purified with a so-called RPHPLC. As eluent acetonitrile/water or chloroform/acetonitrile mixtures can be used. If necessary, these chromatography steps may be repeated, using identical or other chromatography resins. The skilled worker is familiar with the selection of suitable chromatography resin and the most effective use for a particular molecule to be purified.

**[0460.0.0.24] see [0460.0.0.0]**

**[0461.0.24.24]** Example 10: Cloning SEQ ID NO: 34228 for the expression in plants

**[0462.0.0.24] see [0462.0.0.0]**

**[0463.0.24.24]** SEQ ID NO: 34228 is amplified by PCR as described in the protocol of the Pfu Turbo or DNA Herculase polymerase (Stratagene).

**[0464.0.0.24]** to **[0466.0.0.24]: see [0464.0.0.0]** to **[0466.0.0.0]**

**[0466.1.0.24]** In case the Herculase enzyme can be used for the amplification, the PCR amplification cycles were as follows: 1 cycle of 2-3 minutes at 94°C, followed by 25-30 cycles of in each case 30 seconds at 94°C, 30 seconds at 55-60°C and 5-10 minutes at 72°C, followed by 1 cycle of 10 minutes at 72°C, then 4°C.

**[0467.0.24.24]** The following primer sequences were selected for the gene SEQ ID NO: 34228:
i) forward primer (SEQ ID NO: 34320)
ii) reverse primer (SEQ ID NO: 34321)
or were selected for the genes described in Table III, column 5, lines 278 to 289 or lines 637 to 641 as described in Table III, column 7, lines 278 to 289 or lines 637 to 641.

**[0468.0.0.24] to [0479.0.0.24]: see [0468.0.0.0] to [0479.0.0.0]**

**[0480.0.24.24]** Example 11: Generation of transgenic plants which express SEQ ID NO: 34228

**[0481.0.0.24] to [0513.0.0.24]: see [0481.0.0.0] to [0513.0.0.0]**

**[0514.0.24.24]** As an alternative, carotenes can be detected as described in Deli,J. & Molnar,P. Paprika carotenoids: Analysis, isolation, structure eucidation. Curr. Org. Chem. 6, 1197-1219 (2004) or Fraser,P.D., Pinto,M.E., Holloway,D.E. & Bramley,P.M. Technical advance: application of high-performance liquid chromatography with photodiode array detection to the metabolic profiling of plant isoprenoids. Plant J. 24, 551-558 (2000).
The results of the different plant analyses can be seen from the table 1 which follows:

**Table 1**

| ORF | Metabollite | Method | Min | Max |
|---|---|---|---|---|
| b0730 | Isopentenyl Pyrophosphate | LC | 1.41 | 2.15 |
| b1829 | beta-Carotene | LC | 1.33 | 1.74 |
| b1926 | Isopentenyl Pyrophosphate | LC | 1.37 | 1.55 |
| b2211 | Isopentenyl Pyrophosphate | LC | 1.46 | 1.48 |
| b2699 | beta-Carotene | LC | 1.43 | 1.76 |
| b3172 | Isopentenyl Pyrophosphate | LC | 1.35 | 1.74 |
| b4129 | Isopentenyl Pyrophosphate | LC | 1.34 | 1.63 |
| YBR089C-A | beta-Carotene | LC | 1.52 | 2.08 |
| YDR316W | beta-Carotene | LC | 1.28 | 1.52 |
| YDR407C | Isopentenyl Pyrophosphate | LC | 1.54 | 3.14 |
| YDR513W | beta-Carotene | LC | 1.39 | 1.43 |
| YLL013C | beta-Carotene | LC | 1.43 | 1.50 |
| b0970 | Isopentenyl Pyrophosphate | LC | 1.36 | 2.17 |
| b0481 | Isopentenyl Pyrophosphate | LC | 1.35 | 1.39 |
| b1736 | Isopentenyl Pyrophosphate | LC | 1.51 | 2.37 |
| b1738 | Isopentenyl Pyrophosphate | LC | 1.53 | 2.60 |
| b3160 | Isopentenyl Pyrophosphate | LC | 1.35 | 1.47 |

**[0515.0.24.24] to [0552.0.0.24]: see [0515.0.0.0] to [0552.0.0.0]**

**[0552.1.0.24]:** Example 15: Metabolite Profiling Info from *Zea mays*
*Zea mays* plants were engineered as described in Example 14c.
The results of the different *Zea mays* plants analysed can be seen from table 2 which follows:

**Table 2**

| ORF | Metabolite | Min | Max |
|---|---|---|---|
| b1829 | beta-Carotene | 1.40 | 1.76 |

Table 2 describes the beta-Carotene increase in genetically modified corn plants expressing the Escherichia coli nucleic acid sequence b1829.
In case the activity of the Escherichia coli protein b1829 or a heat shock protein with protease acitivity or its homolog, is increased in corn plants, preferably, an increase of the fine chemical beta-Carotene between 40% and 76% is conferred.

**[00552.2.0.24] : see [00552.2.0.0]**

**[0553.0.24.24]**
1. A process for the production of carotene and/or IPP, which comprises
   (a) increasing or generating the activity of a protein as indicated in Table IIA or IIB, columns 5 or 7, lines 279, 282, 285, 286, 288 and/or 289 and/or lines 278, 280, 281, 283, 284 and/or 287 and/or lines 637 to 641 or a functional equivalent thereof in a non-human organism, or in one or more parts thereof; and
   (b) growing the organism under conditions which permit the production of carotene and/or IPP in said organism.
2. A process for the production of carotene and/or IPP, comprising the increasing or generating in an organism or a part thereof the expression of at least one nucleic acid molecule comprising a nucleic acid molecule selected from the group consisting of:
   a) nucleic acid molecule encoding of a polypeptide as indicated in Table IIA or IIB, columns 5 or 7, lines 279, 282, 285, 286, 288, and/or 289, and/or lines 278, 280, 281, 283, 284 and/or 287 and/or lines 637 to 641 or a fragment thereof, which confers an increase in the amount of carotene and/or IPP in an organism or a part thereof;
   b) nucleic acid molecule comprising of a nucleic acid molecule as indicated in Table IA or IB , columns 5 or 7, lines 279, 282, 285, 286, 288 and/or 289 and/or lines 278, 280, 281, 283, 284 and/or 287 and/or lines 637 to 641;
   c) nucleic acid molecule whose sequence can be deduced from a polypeptide sequence encoded by a nucleic acid molecule of (a) or (b) as a result of the degeneracy of the genetic code and conferring an increase in the amount of carotene and/or IPP in an organism or a part thereof;
   d) nucleic acid molecule which encodes a polypeptide which has at least 50% identity with the amino acid sequence of the polypeptide encoded by the nucleic acid molecule of (a) to (c) and conferring an increase in the amount of carotene and/or IPP in an organism or a part thereof;
   e) nucleic acid molecule which hybidizes with a nucleic acid molecule of (a) to (c) under under stringent hybridisation conditions and conferring an increase in the amount of carotene and/or IPP in an organism or a part thereof;
   f) nucleic acid molecule which encompasses a nucleic acid molecule which is obtained by amplifying nucleic acid molecules from a cDNA library or a genomic library using the primers or primer pairs as indicated in Table III, columns 5 or 7, lines lines 279, 282, 285, 286, 288 and/or 289 and/or lines 278, 280, 281, 283, 284 and/or 287 and/or lines 637 to 641 and conferring an increase in the amount of carotene and/or IPP in an organism or a part thereof;
   g) nucleic acid molecule encoding a polypeptide which is isolated with the aid of monoclonal antibodies against a polypeptide encoded by one of the nucleic acid molecules of (a) to (f) and conferring an increase in the amount of carotene and/or IPP in an organism or a part thereof;
   h) nucleic acid molecule encoding a polypeptide comprising a consensus as indicated in Table IV, columns 5 or 7, lines 279, 282, 285, 286, 288 and/or 289 and/or lines 278, 280, 281, 283, 284 and/or 287 and/or lines 637 to 641 and conferring an increase in the amount of carotene and/or IPP in an organism or a part thereof; and
   i) nucleic acid molecule which is obtainable by screening a suitable nucleic acid library under stringent hybridization conditions with a probe comprising one of the sequences of the nucleic acid molecule of (a) to (k) or with a fragment thereof having at least 15 nt, preferably 20 nt, 30 nt, 50 nt, 100 nt, 200 nt or 500 nt of the nucleic acid molecule characterized in (a) to (k) and conferring an increase in the amount of carotene and/or IPP in an organism or a part thereof.
   or comprising a sequence which is complementary thereto.
3. The process of claim 1 or 2, comprising recovering of the free or bound caroteneand/or IPP.
4. The process of any one of claim 1 to 3, comprising the following steps:
   (a) selecting an organism or a part thereof expressing a polypeptide encoded by the nucleic acid molecule characterized in claim 2;
   (b) mutagenizing the selected organism or the part thereof;
   (c) comparing the activity or the expression level of said polypeptide in the mutagenized organism or the part thereof with the activity or the expression of said polypeptide of the selected organisms or the part thereof;
   (d) selecting the mutated organisms or parts thereof, which comprise an increased activity or expression level of said polypeptide compared to the selected organism or the part thereof;
   (e) optionally, growing and cultivating the organisms or the parts thereof; and
   (f) recovering, and optionally isolating, the free or bound carotene and/or IPP produced by the selected mutated organisms or parts thereof.
5. The process of any one of claims 1 to 4, wherein the activity of said protein or the expression of said nucleic acid molecule is increased or generated transiently or stably.
6. An isolated nucleic acid molecule comprising a nucleic acid molecule selected from the group consisting of:
   a) nucleic acid molecule encoding of a polypeptide as indicated in Table IIA or IIB, columns 5 or 7, lines lines 279, 282, 285, 286, 288 and/or 289 and/or lines 278, 280, 281, 283, 284 and/or 287 and/or lines 637 to 641 or a fragment thereof, which confers an increase in the amount of carotene in an organism or a part thereof;
   b) nucleic acid molecule comprising of a nucleic acid molecule as indicated in Table IA or IB , columns 5 or 7, lines 279, 282, 285, 286, 288 and/or 289 and/or lines 278, 280, 281, 283, 284 and/or 287 and/or lines 637 to 641;
   c) nucleic acid molecule whose sequence can be deduced from a polypeptide sequence encoded by a nucleic acid molecule of (a) or (b) as a result of the degeneracy of the genetic code and conferring an increase in the amount of carotene and/or IPP in an organism or a part thereof;
   d) nucleic acid molecule which encodes a polypeptide which has at least 50% identity with the amino acid sequence of the polypeptide encoded by the nucleic acid molecule of (a) to (c) and conferring an increase in the amount of carotene and/or IPP in an organism or a part thereof;
   e) nucleic acid molecule which hybidizes with a nucleic acid molecule of (a) to (c) under stringent hybridisation conditions and conferring an increase in the amount of carotene and/or IPP in an organism or a part thereof;
   f) nucleic acid molecule which encompasses a nucleic acid molecule which is obtained by amplifying nucleic acid molecules from a cDNA library or a genomic library using the primers or primer pairs as indicated in Table III, columns 5 or 7, lines 279, 282, 285, 286, 288 and/or 289 and/or lines 278, 280, 281, 283, 284 and/or 287 and/or lines 637 to 641 and conferring an increase in the amount of carotene and/or IPP in an organism or a part thereof;
   g) nucleic acid molecule encoding a polypeptide which is isolated with the aid of monoclonal antibodies against a polypeptide encoded by one of the nucleic acid molecules of (a) to (f) and conferring an increase in the amount of carotene and/or IPP in an organism or a part thereof;
   h) nucleic acid molecule encoding a polypeptide comprising a consensus as indicated in Table IV, columns 5 or 7, lines 279, 282, 285, 286, 288 and/or 289 and/or lines 278, 280, 281, 283, 284 and/or 287 and/or lines 637 to 641 and conferring an increase in the amount of carotene and/or IPP in an organism or a part thereof; and
   i) nucleic acid molecule which is obtainable by screening a suitable nucleic acid library under stringent hybridization conditions with a probe comprising one of the sequences of the nucleic acid molecule of (a) to (k) or with a fragment thereof having at least 15 nt, preferably 20 nt, 30 nt, 50 nt, 100 nt, 200 nt or 500 nt of the nucleic acid molecule characterized in (a) to (k) and conferring an increase in the amount of carotene and/or IPP in an organism or a part thereof.
   whereby the nucleic acid molecule distinguishes over the sequence as indicated in Table IA or IB , columns 5 or 7, lines 279, 282, 285, 286, 288 and/or 289 and/or lines 278, 280, 281, 283, 284 and/or 287 and/or lines 637 to 641 by one or more nucleotides.
7. A nucleic acid construct which confers the expression of the nucleic acid molecule of claim 6, comprising one or more regulatory elements.
8. A vector comprising the nucleic acid molecule as claimed in claim 6 or the nucleic acid construct of claim 7.
9. The vector as claimed in claim 8, wherein the nucleic acid molecule is in operable linkage with regulatory sequences for the expression in a prokaryotic or eukaryotic, or in a prokaryotic and eukaryotic, host.
10. A host cell, which has been transformed stably or transiently with the vector as claimed in claim 8 or 9 or the nucleic acid molecule as claimed in claim 6 or the nucleic acid construct of claim 7 or produced as described in claim any one of claims 2 to 5.
11. The host cell of claim 10, which is a transgenic host cell.
12. The host cell of claim 10 or 11, which is a plant cell, an animal cell, a microorganism, or a yeast cell, a fungus cell, a prokaryotic cell, an eukaryotic cell or an archaebacterium.
13. A process for producing a polypeptide, wherein the polypeptide is expressed in a host cell as claimed in any one of claims 10 to 12.
14. A polypeptide produced by the process as claimed in claim 13 or encoded by the nucleic acid molecule as claimed in claim 6 whereby the polypeptide distinguishes over a sequence as indicated in Table IIA or IIB, columns 5 or 7, lines 279, 282, 285, 286, 288 and/or 289 and/or lines 278, 280, 281, 283, 284 and/or 287 and/or lines 637 to 641 by one or more amino acids
15. An antibody, which binds specifically to the polypeptide as claimed in claim 14.
16. A plant tissue, propagation material, harvested material or a plant comprising the host cell as claimed in claim 12 which is plant cell or an Agrobacterium.
17. A method for screening for agonists and antagonists of the activity of a polypeptide encoded by the nucleic acid molecule of claim 6 conferring an increase in the amount of carotene and/or IPP in an organism or a part thereof comprising:
   (a) contacting cells, tissues , plants or microorganisms which express the a polypeptide encoded by the nucleic acid molecule of claim 6 conferring an increase in the amount of carotene and/or IPP in an organism or a part thereof with a candidate compound or a sample comprising a plurality of compounds under conditions which permit the expression the polypeptide;
   (b) assaying the linoleic acid level or the polypeptide expression level in the cell, tissue, plant or microorganism or the media the cell, tissue, plant or microorganisms is cultured or maintained in; and
   (c) identifying a agonist or antagonist by comparing the measured carotene and/or IPP level or polypeptide expression level with a standard linoleic acid or polypeptide expression level measured in the absence of said candidate compound or a sample comprising said plurality of compounds, whereby an increased level over the standard indicates that the compound or the sample comprising said plurality of compounds is an agonist and a decreased level over the standard indicates that the compound or the sample comprising said plurality of compounds is an antagonist.
18. A process for the identification of a compound conferring increased carotene and/or IPP production in a plant or microorganism, comprising the steps:
   (a) culturing a plant cell or tissue or microorganism or maintaining a plant expressing the polypeptide encoded by the nucleic acid molecule of claim 6 conferring an increase in the amount of carotene and/or IPP in an organism or a part thereof and a readout system capable of interacting with the polypeptide under suitable conditions which permit the interaction of the polypeptide with said readout system in the presence of a compound or a sample comprising a plurality of compounds and capable of providing a detectable signal in response to the binding of a compound to said polypeptide under conditions which permit the expression of said readout system and of the polypeptide encoded by the nucleic acid molecule of claim 6 conferring an increase in the amount of carotene and/or IPP in an organism or a part thereof;
   (b) identifying if the compound is an effective agonist by detecting the presence or absence or increase of a signal produced by said readout system.
19. A method for the identification of a gene product conferring an increase in carotene and/or IPP production in a cell, comprising the following steps:
   (a) contacting the nucleic acid molecules of a sample, which can contain a candidate gene encoding a gene product conferring an increase in carotene and/or IPP after expression with the nucleic acid molecule of claim 6;
   (b) identifying the nucleic acid molecules, which hybridise under relaxed stringent conditions with the nucleic acid molecule of claim 6;
   (c) introducing the candidate nucleic acid molecules in host cells appropriate for producing carotene and/or IPP;
   (d) expressing the identified nucleic acid molecules in the host cells;
   (e) assaying the carotene and/or IPP level in the host cells; and
   (f) identifying nucleic acid molecule and its gene product which expression confers an increase in the carotene and/or IPP level in the host cell in the host cell after expression compared to the wild type.
20. A method for the identification of a gene product conferring an increase in carotene and/or IPP production in a cell, comprising the following steps:
   a) identifiying in a data bank nucleic acid molecules of an organism; which can contain a candidate gene encoding a gene product conferring an increase in the carotene and/or IPP amount or level in an organism or a part thereof after expression, and which are at least 20% homolog to the nucleic acid molecule of claim 6;
   b) introducing the candidate nucleic acid molecules in host cells appropriate for producing carotene and/or IPP;
   c) expressing the identified nucleic acid molecules in the host cells;
   d) assaying the carotene and/or IPP level in the host cells; and
   e) identifying nucleic acid molecule and its gene product which expression confers an increase in the carotene and/or IPP level in the host cell after expression compared to the wild type.
21. A method for the production of an agricultural composition comprising the steps of the method of any one of claims 17 to 20 and formulating the compound identified in any one of claims 17 to 20 in a form acceptable for an application in agriculture.
22. A composition comprising the nucleic acid molecule of claim 6, the polypeptide of claim 14, the nucleic acid construct of claim 7, the vector of any one of claims 8 or 9, an antagonist or agonist identified according to claim 17, the compound of claim 18, the gene product of claim 19 or 20, the antibody of claim 15, and optionally an agricultural acceptable carrier.
23. Use of the nucleic acid molecule as claimed in claim 6 for the identification of a nucleic acid molecule conferring an increase of carotene and/or IPP after expression.
24. Use of the polypeptide of claim 14 or the nucleic acid construct claim 7 or the gene product identified according to the method of claim 19 or 20 for identifying compounds capable of conferring a modulation of carotene and/or IPP levels in an organism.
25. Cosmetic, pharmaceutical, food or feed composition comprising the nucleic acid molecule of claim 6, the polypeptide of claim 14, the nucleic acid construct of claim 7, the vector of claim 8 or 9, the antagonist or agonist identified according to claim 17, the antibody of claim 15, the plant or plant tissue of claim 16, the harvested material of claim 16, the host cell of claim 10 to 12 or the gene product identified according to the method of claim 19 or 20.
26. The method of any one of claims 1 to 5, the nucleic acid molecule of claim 6, the polypeptide of claim 14, the nucleic acid construct of claim 7, the vector of claim 8 or 9, the antagonist or agonist identified according to claim 17, the antibody of claim 15, the plant or plant tissue of claim 16, the harvested material of claim 16, the host cell of claim 10 to 12 or the gene product identified according to the method of claim 19 or 20, wherein the carotene is beta-carotene
27. Use of the nucleic acid molecule of claim 6, the polypeptide of claim 14, the nucleic acid construct of claim 7, the vector of claim 8 or 9, the antagonist or agonist identified according to claim 17, the antibody of claim 15, the plant or plant tissue of claim 16, the harvested material of claim 16, the host cell of claim 10 to 12 or the gene product identified according to the method of claim 19 or 20 for the protection of a plant against a oxidative stress.
28. Use of the nucleic acid molecule of claim 6, the polypeptide of claim 14, the nucleic acid construct of claim 7, the vector of claim 8 or 9, the antagonist or agonist identified according to claim 17, the antibody of claim 15, the plant or plant tissue of claim 16, the harvested material of claim 16, the host cell of claim 10 to 12 or the gene product identified according to the method of claim 19 or 20 for the protection of a plant against a oxidative stress causing or a carotenoid synthesis inhibiting herbicide.
29. Use of the agonist identified according to claim 17, the plant or plant tissue of claim 16, the harvested material of claim 16, or the host cell of claim 10 to 12 for the production of a cosmetic composition.

**[0554.0.0.24] Abstract: see [0554.0.0.0]**

### Process for the production of fine chemicals

**[0000.0.0.25]** In a further embodiment, the present invention relates to a further process for the production of fine chemicals as defined below and the corresponding embodiments as described herein as follows.

**[0001.0.0.25]** see **[0001.0.0.0]**

**[0002.0.25.25]** Carbohydrates are aldehyde or ketone compounds with multiple hydroxyl groups. Many carbohydrates have the empirical formula (CH2O)n; some also contain nitrogen, phosphorus, or sulfur.
Carbohydrates are classsfied in monosaccharides, oligosaccharides, and polysaccharides.
Monosaccharides, or simple sugars, consist of a single polyhydroxy aldehyde or ketone unit. Monosaccharides of more than four carbons tend to have cyclic structures.
Oligosaccharides consist of short chains of monosaccharide units, or residues, usually 2 to 19 units, joined by glycosidic bonds.
The polysaccharides are sugar polymers containing more than 20 or so monosaccharide units, and some have hundreds or thousands of units. Some polysaccharides are linear chains; others are branched.
Carbohydrates are called saccharides or, if they are relatively small, sugars.
In the present invention, saccharides means all of the aforementioned carbohydrate, e.g. monosaccharides, preferably fructose, glucose, inositol, galactose, arabinose xylose or other pentoses or hexoses; oligosaccharides, preferably disaccharides like sucrose, lactose or trisaccharides like raffinose; or polysaccharides like starch or cellulose.

**[0003.0.25.25]** Carbohydrates are the most abundant class of organic compounds found in living organisms.
They are a major source of metabolic energy, both for plants and for animals. Aside from the sugars and starches that meet this vital nutritional role, carbohydrates function in energy storage (for example starch or glycogen), in signaling (by glycoproteins and glycolipids, e.g. blood group determinants), fuel the nervous system, muscle and virtually all cells, are parts of nucleic acids ( in genes, mRNA, tRNA, ribosomes), and as cell surface markers as recognition sites on cell surfaces and signaling in glycolipids and glycoproteins and also serve as a structural material for example as cell wall components (cellulose).

**[0004.0.25.25]** Glucose, also called dextrose, is the most widely distributed sugar in the plant and animal kingdoms and it is the sugar present in blood as "blood sugar". It occupies a central position in the metabolism of plants, animals, and many microorganisms. Glucose is rich in potential energy, and thus a good fuel; in the body is catabolised to produce ATP. It is stored as a high molecular weight polymer such as starch or glycogen or is converted to fatty acids. It is also a remarkably versatile precursor, capable of supplying a huge array of metabolic intermediates for biosynthetic reactions.
Based on its manifold features, glucose is used in nutrition and medicine.
Fructose, also called levulose or "fruit sugar", is the most important ketose sugar. Fructose is a hexose and is a reducing sugar. Fructose is used a a sweetener by diabetics because it does not rise the blood sugar level, even in large amounts. Fructose and glucose are the main carbohydrate constituents of honey. Those hexoses are further the main components of many oligo- and polysaccharides, like sucrose, raffinose, stachyose, trehalose, starch, cellulose or dextran.
The most frequent disaccharide is sucrose (saccharose, b-D-fructofuranosyl-a-D-glucopyranosid, cane sugar, beet sugar, sugar in a narrow sense of a name for commercially available sucrose meaning sucrose is the sugar that is commonly called "sugar") which consists of the six-carbon sugars D-glucose and D-fructose. It is formed by plants but not by animals. Sucrose is a major intermediate product of photosynthesis; in many plants it is the principal form in which sugar is transported from the leaves to other parts of the plant body. In mammalians sucrose is an obligatory component of blood and its content in blood is kept at the stable level. It is strongly necessary for brain cells as well as for normal functioning of the central nervous system. Sugar is widely-known as a source of glycogen - a substance, feeding liver, heart and muscles. It is one of the most widely-used food products and is the major disaccharide in most diets. It is present in honey, maple sugar, fruits, berries, and vegetables. It may be added to food products as liquid or crystalline sucrose or as invert sugar. It is commercially prepared from sugar cane or sugar beets. Sucrose can provide a number of desirable functional qualities to food products including sweetness, mouth-feel, and the ability to transform between amorphous and crystalline states. High-concentrated sucrose is a natural preserving agent, it determines gel-formation processes, gives necessary viscosity to the products. Sucrose is a raw material for caramel, colour etc.
Sucrose is further an excellent fermentation feedstock, which is of specific interest for fermentation industry (including a number of non-food industries -pharmaceutical industries). The presence of eight hydroxyl groups in the sucrose molecule provides a theoretical possibility of a very large number of sucrose derivatives. Sucrose derivatives are used by industries in production of detergents, emulsifiers (sucrose + fatty acids) and adhesives (sucrose octa acetate).
Sucrose is a precursor to a group of carbohydrates in plants known as the raffinose family of oligosaccharides found in many plant seeds especially legumes. This family contains the trisaccharide raffinose, the tetrasaccharide stachyose and the pentasaccharide verbascose. Oligosaccharides of the raffinose-series are major components in many food legumes (Shallenberger et al., J.Agric. Food Chem., 9,1372; 1961). Raffinose (beta-D-fructofuranosyl-6-O-alpha-D-galactopyranosyl- alpha- D-glucopyranosid, melitriose, gossypose, melitose), which consists of sucrose with a-galactose attached through its C-4 atom to the 1 position on the fructose residue and is thought to be second only to sucrose among the nonstructural carbohydrates with respect to abundance in the plant kingdom. It may be ubiquitous, at least among higher plants. Raffinose accumulate in significant quantities in the edible portion of many economically significant crop species. Examples include soybean, sugar beet, cotton, canola and all of the major edible leguminous crops including beans, peas, lentil and lupine.
An important key intermediates in the formation of raffinose and stachyose is myo-inositol (cyclohexan-1,2,3,4,5,6-hexaole), the most common cyclitol. Myo-inositol is fundamental to many different aspects of plant growth and development. In addition to its role as the precursor for phytic acid biosynthesis, myo-inositol is also used for uronide and pentose biosynthesis, it is also present in phosphoinositides of plant cell membranes, as well as other complex plant lipids including glycophosphoceramides. Furthermore, it is also a precursor of other naturally occurring inositol isomers, and many of these as well as myo-inositol are distributed as methyl ethers in a species specific pattern throughout the plant kingdom. Myo-inositol is an important growth factor.
The most carbohydrates found in nature occur as polysaccharides which are polymers of medium to high molecular weight. Polysaccharides, also called glycans, differ from each other in the identity of their recurring monosaccharide units, in the length of their chains, in the types of bonds linking the units, and in the degree of branching.
Starch is the most valuable polysaccharide. Normal native starches consist of a mixture of 15-30 % amylose and 70-85 % amylopectin. Amylose structurally is a linear polymer of anhydroglucose units, of molecular weight approximately between 40 000 and 340 000, the chains containing 250 to 2000 anhydroglucose units. Amylopectin is considered to be composed of anhydroglucose chains with many branch points; the molecular weight may reach as high as 80 000 000.
Starch is the most important, abundant, digestible food polysaccharide. It occurs as the reserve polysaccharide in the leaf, stem, root, seed, fruit and pollen of many higher plants. It occurs as discrete, partially-crystalline granules whose size, shape, and gelatinization temperature depend on the botanical source of the starch. Common food starches are derived from seed (wheat, maize, rice, barley) and root (potato, cassava/tapioca) sources. Starches have been modified to improve desired functional characteristics and are added in relatively small amounts to foods as food additives. Another important polysaccharide is cellulose. Cellulose is the most commonly seen polysaccharide and scientist estimate that over one trillion tons of cellulose are synthesized by plants each year. Cellulose forms the cell wall of plants. It is yet a third polymer of the monosaccharide glucose. Cellulose differs from starch and glycogen because the glucose units form a two-dimensional structure, with hydrogen bonds holding together nearby polymers, thus giving the molecule added stability. A single "cellulose fiber" can consist of up to 10000 individual anhydroglucose units. In
cellulose, the individual fiber molecules are arranged in bundles and thus form so called micro fibrils which ultimately result in a "densely woven" net like structure of cellulose molecules. The strong cohesion between the individual cellulose fibers is due to the huge number of strong hydrogen bonds.
Cellulose is the major polysaccharide of grass, leaves and trees and is said to include around 50% of all biological carbon found on our planet. It is the basic material of natural substances such as wood, flax or cotton and consists of long, unbranched fiber molecules. Cellulose, as plant fiber, cannot be digested by human beings therefore cellulose passes through the digestive tract without being absorbed into the body. Some animals, such as cows and termites, contain bacteria in their digestive tract that help them to digest cellulose. Nevertheless, cellulose is of importance in human nutrition in that fiber is an essential part of the diet, giving bulk to food and promoting intestinal motility.

**[0005.0.25.25]** The polysaccharides starch and cellulose are the most important raw material in the industrial and comercial production of glucose. In the common procedure starch or cellulose are acidly or enzymatically hydrolysed to glucose. Fructose is usually also produced from starch by enzymatically transforming it into glucose syrup and subsequently treating with isomerase, leading to a conversion of glucose to fructose.
Succrose is obtained comercially from the expressed juice of sugar cane or of sugar beet.
Myo-inositol exists in nature either in its free form (found, for example, in sugarcane, beet molasses, and almond hulls) or as a hexaphosphate called phytin (found, for example, in corn steep liquor). Industrial purification of phytin from corn steep liquor involves precipitation with calcium, followed by hydrolysis with a strong acid. Separation of free form inositols from plant extracts involves treatment with acid and separation of myo-inositol by column (US 5,482,631) or the use of ion-exchange (US 4,482,761).
Cellulose is a very important industrial product. As disclosed above, it serves as row material for monosaccharides. It is further used in the manufacture of paper, textiles, plastics, explosives, packaging material (cellophane®), feed, food and fermentation products. Cellulose is obtained primarily by acid or alkaline hydrolize.
Starch occurs intracellularly as large clusters or granules. These granular starch consists of microscopic granules, which differ in size and shape, depending on the plant source. The granules are insoluble in water at room temperature. There is a quite number of methods known for the extraction of starch. For example a slurry of grinded starch containing plant material is heated, whereby the granules swell and eventually burst, dispersing the starch molecules into the solution. During the liquefaction step, the long-chained starch is further degraded into smaller branched and linear units (maltodextrins) by an alpha-amylase. A large number of processes have been described for converting starch to starch hydrolysates, such as maltose, glucose or specialty syrups, either for use as sweeteners or as precursors for other saccharides such as fructose. A process for enzymatic hydrolysis of granular starch into a soluble starch hydrolysate is disclosed in US 20050042737.

**[0006.0.25.25]** Carbohydrates play a major role in human and animal diets, comprising some 40-75% of energy intake. Their most important nutritional property is digestibility. Some of them are hydrolyzed by enzymes of the human gastrointestinal system to monosaccharides that are absorbed in the small intestine and enter the pathways of carbohydrate metabolism. Others can be digested by certain animals. Carbohydrates, fat and protein are the energy yielding nutrients in animal feed. In the average diet for farm animals, carbohydrates are included at levels of 70-80%. For example pig diets are mainly based on cereals which contain the main part of the energy providing nutrients that are essential for pigs.
With view to the increasing global demand for food because of the growing world population and at the same time the shrinking availability of arable land, it is important to increase the food and feed quality, particulary the availability of certain essential nutrients, preferably carbohydrates, preferably polysaccharides like starch or cellulose and/or monosaccharides like fructose, glucose and/or myo-inositol and/or trisaccharides like raffinose and/or disaccharides like sucrose. Nutritional improvements in foods and feeds could help to meet these demands for improved quality. Modern agricultural biotechnology, which involves the application of cellular and molecular techniques to transfer DNA that encodes a desired trait to food and feed crops, is a powerful complement to traditional methods to meet global food and feed requirements.

**[0007.0.25.25]** Furthermore the physicochemical properties such as viscosity and the capacity to bind water and ions, vary between different cereals. Consequently, different cereal properties affect digestion and fermentation as well as microbial populations in the gastro-intestinal tract in various ways. Gastro-intestinal disturbances comprise a major problem for health of humans and animals.
There is a need for suitable dietary composition and food or feed ingredients, preferably cereals, legumes or fruits which promotes a beneficial gut environment and thereby preventing gastro-intestinal disorders.
Therefore improving the quality of foodstuffs and animal feeds is an important task of the food-and-feed industry. This is necessary since, for example, carbohydrates, which occur in plants and some microorganisms are limited with regard to the supply of mammals. Especially advantageous for the quality of foodstuffs and animal feeds is as balanced as possible a carbohydrate profile in the diet since a great excess of some sugars above a specific concentration in the food has only some or little or no positive effect.

**[0008.0.25.25]** Genetically modified plants having improved nutritional profiles are known in the state of art. US 20030070192 discloses a DNA expression cassette which alters the sugar alcohol of tranformed plants.
US 5,908,975 concerns methods for synthesis and accumulation of fructose polymers in transgenic plants by selective expression of bacterial fructosyltransferase genes using tissue specific promoters and a vacuole targeting sequence.
WO89/12386 describes a method for the production of glucose and fructose polymers in transgenic tomato plants.
A stress tolerance sequences including proteins like galactinol synthase (GOLS) and raffinose synthase (RAFS), which are up regulated in response to stress and lead to the production of raffinose is disclosed in US 20050055748.
US 6,887,708 provides nucleotide sequences encoding polypeptides having the function of GIGANTEA gene of Arabidopsis thaliana which allows the manipulation of the starch accumulation process in plants.
Grain having an embryo with a genotype heterozygous for two or more wild type genes (for example, Aa/Bb) and an endosperm having a genotype heterozygous for such genes and leading to plants with altered the normal starch synthesis pathway is disclosed in US 20050091716.

**[0009.0.25.25]** Nevertheles, there is a constant need for providing novel enzyme activities or direct or indirect regulators and thus alternative methods with advantageous properties for producing carbohydrates, preferably polysaccharides like starch or cellulose and/or monosaccharides like fructose, glucose and/or myo-inositol and/or trisaccharides like raffinose and/or disaccharides like sucrose or its precursor in organisms, e.g. in transgenic organisms.

**[0010.0.25.25]** Another problem is the seasonal change in carbohydrate composition of plants and optimum harvest periods for are complicated by issues of timing.

**[0011.0.25.25]** To ensure constantly a high quality of foods and animal feeds, it is necessary to add one or a plurality of carbohydrates, preferably polysaccharides like starch or cellulose and/or monosaccharides like fructose, glucose and/or myo-inositol and/or trisaccharides like raffinose and/or disaccharides like sucrose in a balanced manner to suit the organism.

**[0012.0.25.25]** Accordingly, there is still a great demand for new and more suitable genes which encode enzymes which participate in the biosynthesis of carbohydrates, preferably polysaccharides like starch or cellulose and/or monosaccharides like fructose, glucose and/or myo-inositol and/or trisaccharides like raffinose and/or disaccharides like sucrose and make it possible to produce them specifically on an industrial scale without unwanted byproducts forming. In the selection of genes for or regulators of biosynthesis two characteristics above all are particularly important. On the one hand, there is as ever a need for improved processes for obtaining the highest possible contents of carbohydrates, preferably polysaccharides like starch or cellulose and/or monosaccharides like fructose, glucose and/or myo-inositol and/or trisaccharides like raffinose and/or disaccharides like sucrose; on the other hand as less as possible byproducts should be produced in the production process.
The added carbohydrates further beneficially affects the microflora by selectively stimulating the growth and/or activity of beneficial bacteria.
Another aspect is the significant reduction of cost of production and manufacturing not only to the nutrition, in particular sweetener industry, but also agriculture and cosmetic and health industry.

**[0013.0.0.25]** see **[0013.0.0.0]**

**[0014.0.25.25]** Accordingly, in a first embodiment, the invention relates to a process for the production of a fine chemical, whereby the fine chemical is carbohydrates, preferably polysaccharides, more preferably starch and/or cellulose and/or monosaccharides, more preferably fructose, glucose and/or myo-inositol and/or trisaccharides, more preferably raffinose and/or disaccharides, more preferably sucrose. Accordingly, in the present invention, the term "the fine chemical" as used herein relates to (a) "carbohydrates, preferably polysaccharides, more preferably starch and/or cellulose and/or monosaccharides, more preferably fructose, glucose and/or myo-inositol and/or trisaccharides, more preferably raffinose and/or disaccharides, more preferably sucrose". Further, the term "the fine chemicals" as used herein also relates to compositions of fine chemicals comprising carbohydrates, preferably polysaccharides, more preferably starch and/or cellulose and/or monosaccharides, more preferably fructose, glucose and/or myo-inositol and/or trisaccharides, more preferably raffinose and/or disaccharides, more preferably sucrose.

**[0015.0.25.25]** In one embodiment, the term " carbohydrate" or "the fine chemical" or "the respective fine chemical" means at least one chemical compound with carbohydrate activity selected from the group of preferably polysaccharides, more preferably starch and/or cellulose and/or monosaccharides, more preferably fructose, glucose and/or myo-inositol and/or trisaccharides, more preferably raffinose and/or disaccharides, more preferably sucrose. In an preferred embodiment, the term "the fine chemical" or the term " carbohydrate" or the term "the respective fine chemical" means at least one chemical compound with carbohydrate activity selected from the group "carbohydrates, preferably polysaccharides, more preferably starch and/or cellulose and/or monosaccharides, more preferably fructose, glucose and/or myo-inositol and/or trisaccharides, more preferably raffinose and/or disaccharides, more preferably sucrose".
An increased carbohydrate content normally means an increased total carbohydrate content. However, an increased carbohydrate content also means, in particular, a modified content of the above-described compounds with carbohydrate activity, without the need for an inevitable increase in the total carbohydrate content.. In a preferred embodiment, the term "the fine chemical" means carbohydrates, preferably polysaccharides, more preferably starch and/or cellulose and/or monosaccharides, more preferably fructose, glucose and/or myo-inositol and/or trisaccharides, more preferably raffinose and/or disaccharides, more preferably sucrose in free form or its salts or its ester or its ether or bound to acylglycerol.

**[0015.1.22.22]** A measure for the content of the polysaccharides, preferably starch and cellulose, of the invention can be the content of anhydroglucose. This compound is the analyte which indicates the presence of the polysaccharides, preferably starch and cellulose, of the invention if the samples are prepared and measured as described in the examples.

**[0016.0.25.25]** Accordingly, the present invention relates to a process comprising
(a) increasing or generating the activity of one or more
   b0252, b1430, b1693, b3231, YER174C, b0050, ,b1539, b3919 and/or b4232 - protein(s) or of a protein having the sequence of a polypeptide encoded by a nucleic acid molecule indicated in Table II, columns 5 or 7, lines 290 to 294 and/or 604 to 607;
   in a non-human organism in one or more parts thereof and
(b) growing the organism under conditions which permit the production of the fine chemical, meaning of starch and/or cellulose or fine chemicals comprising starch and/or cellulose in said organism;
or
(a) increasing or generating the activity of one or more b0161, b0695, b1708, b1926, b2597, b2664, b4239, b4327, b0124, b0149, b1318, b1463, b2491, b3260, b3578, b3619 and/or b4122 -protein(s) or of a protein having the sequence of a polypeptide encoded by a nucleic acid molecule indicated in Table II, columns 5 or 7, lines 295 to 302 and/or 608 to 616;
   in a non-human organism in one or more parts thereof and
(b) growing the organism under conditions which permit the production of the fine chemical, meaning of fructose or fine chemicals comprising fructose in said organism;
or
(a) increasing or generating the activity of one or more b0161, b1708, b1926, b2599, b4239, YJL072C, b1463, b1736, b2491, b3578 and/or b3619-protein(s) or of a protein having the sequence of a polypeptide encoded by a nucleic acid molecule indicated in Table II, columns 5 or 7, lines 303 to 308 and/or 617 to 621;
   in a non-human organism in one or more parts thereof and
(b) growing the organism under conditions which permit the production of the fine chemical, meaning of glucose or fine chemicals comprising glucose in said organism;
or
(a) increasing or generating the activity of one or more b0138, b0290, b2023, b2699, b3172, b3430, b4129, YBR204C, YDR112W, YGR261C, YIL150C, YJL099W, YOR044W , YOR350C, b1463, b1961, b4074 and/or YHR072W-A -protein(s) or of a protein having the sequence of a polypeptide encoded by a nucleic acid molecule indicated in Table II, columns 5 or 7, lines 309 to 322 and/or 622 to 625;
   in a non-human organism in one or more parts thereof and
(b) growing the organism under conditions which permit the production of the fine chemical, meaning of myo-inositol or fine chemicals comprising myo-inositol in said organism;
or
(a) increasing or generating the activity of one or more b0161, b0730, b1701, b1886, b2664, b2699, b3601, YBR184W, YGR261C and/or b3578-protein(s) or of a protein having the sequence of a polypeptide encoded by a nucleic acid molecule indicated in Table II, columns 5 or 7, lines 323 to 331 and/or 626;
   in a non-human organism in one or more parts thereof and
(b) growing the organism under conditions which permit the production of the fine chemical, meaning of raffinose or fine chemicals comprising raffinose in said organism;
or
(a) increasing or generating the activity of one or more b0019 and/or b4239-protein(s) or of a protein having the sequence of a polypeptide encoded by a nucleic acid molecule indicated in Table II, columns 5 or 7, lines 332 to 333;
   in a non-human organism in one or more parts thereof and
(b) growing the organism under conditions which permit the production of the fine chemical, meaning of sucrose or fine chemicals comprising sucrose in said organism.
Accordingly, the present invention relates to a process for the production of carbohydrates comprising
(a) increasing or generating the activity of one or more proteins having the activity of a protein indicated in Table II, column 3, lines 290 to 333 and/or lines 604 to 626 or having the sequence of a polypeptide encoded by a nucleic acid molecule indicated in Table I, column 5 or 7, lines 290 to 333 and/or lines 604 to 626, in a non-human organism in one or more parts thereof and
(b) growing the organism under conditions which permit the production of the fine chemical, thus, carbohydrates, preferably polysaccharides, more preferably starch and/or cellulose and/or monosaccharides, more preferably fructose, glucose and/or myo-inositol and/or trisaccharides, more preferably raffinose and/or disaccharides, more preferably sucrose resp..

**[0016.1.25.25]** Accordingly, the term "the fine chemical" means in one embodiment "polysaccharides, preferably starch and/or cellulose" in relation to all sequences listed in Table I to IV, lines 290 to 294 and/or and/or 604 to 607 or homologs thereof and
means in one embodiment "monosaccharides, preferably fructose" in relation to all sequences listed in Table I to IV, lines 295 to 302 and/or 608 to 616 or homologs thereof, and
means in one embodiment "monosaccharides, preferably glucose" in relation to all sequences listed in Tables I to IV, lines 303 to 308 and/or 617 to 621 or homologs thereof and
means in one embodiment "monosaccharides, preferably myo-inositol" in relation to all sequences listed in Table I to IV, lines 309 to 322 and/or 622 to 625, and
means in one embodiment "trisaccharides, preferably raffinose" in relation to all sequences listed in Table I to IV, lines 323 to 331 and/or 626, and
means in one embodiment "disaccharides, preferably sucrose" in relation to all sequences listed in Table I to IV, lines 332 to 333.
Accordingly, in one embodiment the term "the fine chemical" means "fructose", "glucose" and "raffinose" in relation to all sequences listed in Table I to IV, lines 295, 303 and 323 and/or lines 614 and 620 and 626;
in one embodiment the term "the fine chemical" means "fructose", "glucose" and "myo-inositol" in relation to all sequences listed in Table I to IV, lines 611, 617 and 622 and/or lines 611 and 617 and 622;
in one embodiment the term "the fine chemical" means "fructose" and "glucose" in relation to all sequences listed in Table I to IV, lines 297 and 304 and/or lines 612 and 619 and/or lines 614 and 620 and/or lines 615 and 621 and or lines 611 and 617;
in one embodiment the term "the fine chemical" means "fructose" and "glucose" in relation to all sequences listed in Table I to IV, lines 298 and 305,
in one embodiment the term "the fine chemical" means "fructose" and "raffinose" in relation to all sequences listed in Table I to IV, lines 300 and 327;
in one embodiment the term "the fine chemical" means "fructose", "glucose" and "sucrose" in relation to all sequences listed in Table I to IV, lines 301, 307 and 333;
in one embodiment the term "the fine chemical" means "myo-inositol" and "raffinose" in relation to all sequences listed in Table I to IV, lines 312 and 328;
in one embodiment the term "the fine chemical" means "myo-inositol" and "raffinose" in relation to all sequences listed in Table I to IV, lines 318 and 331;
in one embodiment the term "the fine chemical" means "myo-inositol" and "fructose" in relation to all sequences listed in Table I to IV, lines 611 and 622;
in one embodiment the term "the fine chemical" means "myo-inositol" and "glucose" in relation to all sequences listed in Table I to IV, lines 617 and 622;
in one embodiment the term "the fine chemical" means "fructose" and "raffinose" in relation to all sequences listed in Table I to IV, lines 614 and 626;
in one embodiment the term "the fine chemical" means "glucose" and "raffinose" in relation to all sequences listed in Table I to IV, lines 620 and 626;
Accordingly, the term "the fine chemical" can mean "starch and/or cellulose", "fructose", "glucose", "myo-inositol", "raffinose"and/or "sucrose", owing to circumstances and the context. In order to illustrate that the meaning of the term "the fine chemical" means "starch and/or cellulose", "fructose", "glucose", "myo-inositol", "raffinose"and/or "sucrosel" the term "the respective fine chemical" is also used.

**[0017.0.25.25]** to **[0018.0.25.25]: see [0017.0.0.0]** to **[0018.0.0.0]**

**[0019.0.25.25]** Advantageously the process for the production of the respective fine chemical leads to an enhanced production of the respective fine chemical. The terms "enhanced" or "increase" mean at least a 10%, 20%, 30%, 40% or 50%, preferably at least 60%, 70%, 80%, 90% or 100%, more preferably 150%, 200%, 300%, 400% or 500% higher production of the respective fine chemical in comparison to the reference as defined below, e.g. that means in comparison to an organism without the aforementioned modification of the activity of a protein having the activity of a protein indicated in Table II, column 3, lines 290 to 333 and/or lines 604 to 626or encoded by nucleic acid molecule indicated in Table I, columns 5 or 7, lines 290 to 333 and/or lines 604 to 626.

**[0020.0.25.25]** Surprisingly it was found, that the transgenic expression of at least one of the Saccaromyces cerevisiae protein(s) indicated in Table II, Column 3, line 294 for starch and/or cellulose;
line 308 for glucose;
lines 316 to 322 and/or 625 for myo-inositol;
lines 330 to 331 for raffinose;
in Arabidopsis thaliana conferred an increase in the respective fine chemical content of the transformed plants
and/or
at least one of the Escherichia coli K12 proteins indicated in Table II, Column 3,
lines 290 to 293 and/or 604 to 607 for starch and/or cellulose;
lines 295 to 302 and/or 608 to 616 for fructose;
lines 303 to 307 and/or 617 to 621 for glucose;
lines 309 to 315 and/or 622 to 624 for myo-inositol;
lines 323 to 329 and/or 626 for raffinose;
lines 332 to 333 for sucrose;
in Arabidopsis thaliana conferred an increase in the respective fine chemical content of the transformed plants.
Accordingly, it was surprisingly found, that the transgenic expression of the Escherichia coli K12 protein as indicated in Table II, column 5, lines 295, 303 and 323 and/or lines 614 and 620 and 626 in Arabidopsis thaliana conferred an increase in fructose, glucose or raffinose (or the respective fine chemical) content of the transformed plants. Thus, in one embodiment, said protein or its homologs are used for the production of fructose; in one embodiment, said protein or its homologs are used for the production of glucose; in one embodiment, said protein or its homologs are used for the production of raffinose; in one embodiment, said protein or its homologs are used for the production of one or more fine chemical selected from the group consisting of: fructose, glucose and raffinose.
Accordingly, it was surprisingly found, that the transgenic expression of the Escherichia coli K12 protein as indicated in Table II, column 5, lines 611, 617 and 622 in Arabidopsis thaliana conferred an increase in fructose, glucose or myo-inositol (or the respective fine chemical) content of the transformed plants. Thus, in one embodiment, said protein or its homologs are used for the production of fructose; in one embodiment, said protein or its homologs are used for the production of glucose; in one embodiment, said protein or its homologs are used for the production of myo-inositol; in one embodiment, said protein or its homologs are used for the production of one or more fine chemical selected from the group consisting of: fructose, glucose and myo-inositol.
Accordingly, it was surprisingly found, that the transgenic expression of the Escherichia coli K12 protein as indicated in Table II, column 5, lines 297 and 304 and/of lines 298 and 305 and/or and/or lines 612 and 619 and/or lines 614 and 620 in Arabidopsis thaliana conferred an increase in fructose or glucose (or the respective fine chemical) content of the transformed plants. Thus, in one embodiment, said protein or its homologs are used for the production of fructose; in one embodiment, said protein or its homologs are used for the production of glucose; in one embodiment, said protein or its homologs are used for the production of one or more fine chemical selected from the group consisting of: fructose and glucose.
Accordingly, it was surprisingly found, that the transgenic expression of the Escherichia coli K12 protein as indicated in table II, line 300 and 327 in Arabidopsis thaliana conferred an increase in fructose or raffinose (or the respective fine chemical) content of the transformed plants. Thus, in one embodiment, said protein or its homologs are used for the production of frutose; in one embodiment, said protein or its homologs are used for the production of raffinose; in one embodiment, said protein or its homologs are used for the production of fructose and raffinose.
Accordingly, it was surprisingly found, that the transgenic expression of the Escherichia coli K12 protein as indicated in table II, lines 301, 307 and 333 in Arabidopsis thaliana conferred an increase in fructose, glucose or sucrose (or the respective fine chemical) content of the transformed plants. Thus, in one embodiment, said protein or its homologs are used for the production of fructose; in one embodiment, said protein or its homologs are used for the production of glucose; in one embodiment, said protein or its homologs are used for the production of sucrose; in one embodiment, said protein or its homologs are used for the production of fructose and glucose; in one embodiment, said protein or its homologs are used for the production of fructose and sucrose; in one embodiment, said protein or its homologs are used for the production of glucose and sucrose; in one embodiment, said protein or its homologs are used for the production of fructose, glucose and sucrose.
Accordingly, it was surprisingly found, that the transgenic expression of the Escherichia coli K12 protein as indicated in table II, line 312 and 328, or the Saccaromyces cerevisiae proteins as indicated in Table II, column 3, lines 318 and 331 in Arabidopsis thaliana conferred an increase in myo-inositol or raffinose (or the respective fine chemical) content of the transformed plants. Thus, in one embodiment, one of said proteins or its homologs are used for the production of myo-inositol; in one embodiment, said protein or its homologs are used for the production of raffinose; in one embodiment, said protein or its homologs are used for the production of myo-inositol and raffinose.

**[0021.0.25.25]** see **[0021.0.0.0]**

**[0022.0.25.25]** The sequence of b0019 from Escherichia coli K12 has been published in Blattner et al., Science 277(5331), 1453-1474, 1997, and its activity is being defined as a Na+/H+ antiporter. Accordingly, in one embodiment, the process of the present invention comprises the use of a gene product with an activity of the Na+/H+-exchanging protein nhaA superfamily , preferably a protein with a Na+/H+ antiporter protein activity or its homolog, e.g. as shown herein, from Escherichia coli K12 or its homolog, e.g. as shown herein, for the production of the fine chemical, meaning of carbohydrate, preferably sucrose in free or bound form in an organism or a part thereof, as mentioned.
The sequence of b0138 from Escherichia coli K12 has been published in Blattner et al., Science 277(5331), 1453-1474, 1997, and its activity is being defined as a putative fimbrial-like adhesin protein. Accordingly, in one embodiment, the process of the present invention comprises the use of a gene product with an activity of the Escherichia coli K12 fimbrial-like adhesin protein or its homolog, e.g. as shown herein, from Escherichia coli K12 or its homolog, e.g. as shown herein, for the production of the fine chemical, meaning of carbohydrate, preferably myo-inositol in free or bound form in an organism or a part thereof, as mentioned.
The sequence of b0161 from Escherichia coli K12 has been published in Blattner et al., Science 277(5331), 1453-1474, 1997, and its activity is being defined as a periplasmic serine protease (heat shock protein). Accordingly, in one embodiment, the process of the present invention comprises the use of a gene product with an activity of the helicobacter serine proteinase superfamily , preferably a protein with a periplasmic serine protease (heat shock protein) activity or its homolog, e.g. as shown herein, from Escherichia coli K12 or its homolog, e.g. as shown herein, for the production of the fine chemical, meaning of carbohydrate, preferably fructose, glucose and/or raffinose in free or bound form in an organism or a part thereof, as mentioned.
The sequence of b0252 from Escherichia coli K12 has been published in Blattner et al., Science 277(5331), 1453-1474, 1997, and its activity is not yet defined but it is a conserved protein of unknown function, similarity with helicase and ligase proteins. Accordingly, in one embodiment, the process of the present invention comprises the use of a gene product with an activity of the helicase or ligase protein, preferably a protein with activity of b0252 protein from Escherichia coli K12 or its homolog, e.g. as shown herein, from Escherichia coli K12 or its homolog, e.g. as shown herein, for the production of the fine chemical, meaning of carbohydrate, preferably starch and/or cellulose in free or bound form in an organism or a part thereof, as mentioned.
The sequence of b0290 from Escherichia coli K12 has been published in Blattner et al., Science 277(5331), 1453-1474, 1997, and its activity is being defined as a receptor protein. Accordingly, in one embodiment, the process of the present invention comprises the use of a gene product with an activity of the Escherichia coli yagW protein superfamily, preferably a protein with a receptor protein activity or its homolog, e.g. as shown herein, from Escherichia coli K12 or its homolog, e.g. as shown herein, for the production of the fine chemical, meaning of carbohydrate, preferably myo-inositol in free or bound form in an organism or a part thereof, as mentioned.
The sequence of b0695 from Escherichia coli K12 has been published in Blattner et al., Science 277(5331), 1453-1474, 1997, and its activity is being defined as a sensory histidine kinase in two-component regulatory system. Accordingly, in one embodiment, the process of the present invention comprises the use of a gene product with an activity of the sensor histidine kinase superfamily, preferably a protein with a sensory histidine kinase in two-component regulatory system activity or its homolog, e.g. as shown herein, from Escherichia coli K12 or its homolog, e.g. as shown herein, for the production of the fine chemical, meaning of carbohydrate, preferably fructose in free or bound form in an organism or a part thereof, as mentioned.
The sequence of b0730 from Escherichia coli K12 has been published in Blattner et al., Science 277(5331), 1453-1474, 1997, and its activity is being defined as a transcriptional regulator of succinylCoA synthetase operon and fatty acyl response regulator. Accordingly, in one embodiment, the process of the present invention comprises the use of a gene product with an activity of the transcription regulator GntR superfamily, preferably a protein with a transcriptional regulator of succinylCoA synthetase operon and fatty acyl response regulator activity or its homolog, e.g. as shown herein, from Escherichia coli K12 or its homolog, e.g. as shown herein, for the production of the fine chemical, meaning of carbohydrate, preferably raffinose in free or bound form in an organism or a part thereof, as mentioned.
The sequence of b1430 from Escherichia coli K12 has been published in Blattner et al., Science 277(5331), 1453-1474, 1997, and its activity is being defined as a S-adenosyl-L-methionine-dependent methyltransferase conferring tellurite resistance. Accordingly, in one embodiment, the process of the present invention comprises the use of a gene product with an activity of the hemagglutinin hag1 superfamily, preferably a protein with a S-adenosyl-L-methionine-dependent methyltransferase activity conferring tellurite resistance or its homolog, e.g. as shown herein, from Escherichia coli K12 or its homolog, e.g. as shown herein, for the production of the fine chemical, meaning of carbohydrate, preferably starch and/or cellulose in free or bound form in an organism or a part thereof, as mentioned.
The sequence of b1693 from Escherichia coli K12 has been published in Blattner et al., Science 277(5331), 1453-1474, 1997, and its activity is being defined as a 3-dehydroquinate dehydratase. Accordingly, in one embodiment, the process of the present invention comprises the use of a gene product with an activity of the 3-dehydroquinate dehydratase superfamily, preferably a protein with a 3-dehydroquinate dehydratase activity or its homolog, e.g. as shown herein, from Escherichia coli K12 or its homolog, e.g. as shown herein, for the production of the fine chemical, meaning of carbohydrate, preferably starch and/or cellulose in free or bound form in an organism or a part thereof, as mentioned.
The sequence of b1701 from Escherichia coli K12 has been published in Blattner et al., Science 277(5331), 1453-1474, 1997, and its activity is being defined as a CoenzymeA-dependent ligase with firefly luciferase-like ATPase Domain. Accordingly, in one embodiment, the process of the present invention comprises the use of a gene product with an activity of the 4-coumarate-CoA ligase or acetate-CoA ligase superfamily, preferably a protein with a CoenzymeA-dependent ligase activity or its homolog, e.g. as shown herein, from Escherichia coli K12 or its homolog, e.g. as shown herein, for the production of the fine chemical, meaning of carbohydrate, preferably raffinose in free or bound form in an organism or a part thereof, as mentioned.
The sequence of b1708 from Escherichia coli K12 has been published in Blattner et al., Science 277(5331), 1453-1474, 1997, and its activity is being defined as a lipoprotein. Accordingly, in one embodiment, the process of the present invention comprises the use of a gene product with an activity of the protein HI1314 superfamily, preferably a protein with a lipoprotein activity or its homolog, e.g. as shown herein, from Escherichia coli K12 or its homolog, e.g. as shown herein, for the production of the fine chemical, meaning of carbohydrate, preferably fructose and/or glucose in free or bound form in an organism or a part thereof, as mentioned.
The sequence of b1886 from Escherichia coli K12 has been published in Blattner et al., Science 277(5331), 1453-1474, 1997, and its activity is being defined as a methyl-accepting chemotaxis protein II or aspartate sensor receptor . Accordingly, in one embodiment, the process of the present invention comprises the use of a gene product with an activity of the methyl-accepting chemotaxis protein superfamily, preferably a protein with a methyl-accepting chemotaxis protein II or aspartate sensor receptor activity or its homolog, e.g. as shown herein, from Escherichia coli K12 or its homolog, e.g. as shown herein, for the production of the fine chemical, meaning of carbohydrate, preferably raffinose in free or bound form in an organism or a part thereof, as mentioned.
The sequence of b1926 from Escherichia coli K12 has been published in Blattner et al., Science 277(5331), 1453-1474, 1997, and its activity is being defined as a flagellar protein fliT. Accordingly, in one embodiment, the process of the present invention comprises the use of a gene product with an activity of a flagellar protein fliT or its homolog, e.g. as shown herein, from Escherichia coli K12 or its homolog, e.g. as shown herein, for the production of the fine chemical, meaning of carbohydrate, preferably fructose and/or glucose in free or bound form in an organism or a part thereof, as mentioned.
The sequence of b2023 from Escherichia coli K12 has been published in Blattner et al., Science 277(5331), 1453-1474, 1997, and its activity is being defined as a glutamine amidotransferase subunit of imidazole glycerol phosphate synthase heterodimer. Accordingly, in one embodiment, the process of the present invention comprises the use of a gene product with an activity of the amidotransferase hisH superfamily with trpG homology, preferably a protein with a glutamine amidotransferase subunit of imidazole glycerol phosphate synthase heterodimer activity or its homolog, e.g. as shown herein, from Escherichia coli K12 or its homolog, e.g. as shown herein, for the production of the fine chemical, meaning of carbohydrate, preferably myo-inositol in free or bound form in an organism or a part thereof, as mentioned.
The sequence of b2597 from Escherichia coli K12 has been published in Blattner et al., Science 277(5331), 1453-1474, 1997, and its activity is being defined as a ribosome associated factor. Accordingly, in one embodiment, the process of the present invention comprises the use of a gene product with an activity of the Pseudomonas putida protein rpoX superfamily, preferably a protein with a ribosome associated factor activity or its homolog, e.g. as shown herein, from Escherichia coli K12 or its homolog, e.g. as shown herein, for the production of the fine chemical, meaning of carbohydrate, preferably fructose in free or bound form in an organism or a part thereof, as mentioned.
The sequence of b2599 from Escherichia coli K12 has been published in Blattner et al., Science 277(5331), 1453-1474, 1997, and its activity is being defined as a bifunctional enzyme: chorismate mutase P (N-terminal) and prephenate dehydratase (C-terminal). Accordingly, in one embodiment, the process of the present invention comprises the use of a gene product with an activity of the pheA bifunctional enzyme superfamily with prephenate dehydratase homology, preferably a protein with a bifunctional enzyme: chorismate mutase P (N-terminal) and prephenate dehydratase (C-terminal) activity or its homolog, e.g. as shown herein, from Escherichia coli K12 or its homolog, e.g. as shown herein, for the production of the fine chemical, meaning of carbohydrate, preferably glucose in free or bound form in an organism or a part thereof, as mentioned.
The sequence of b2664 from Escherichia coli K12 has been published in Blattner et al., Science 277(5331), 1453-1474, 1997, and its activity is being defined as a transcriptional repressor with DNA-binding Winged helix domain (GntR familiy). Accordingly, in one embodiment, the process of the present invention comprises the use of a gene product with an activity of the transcription regulator gabP superfamily, preferably a protein with a putative transcriptional repressor with DNA-binding Winged helix domain (GntR familiy) activity or its homolog, e.g. as shown herein, from Escherichia coli K12 or its homolog, e.g. as shown herein, for the production of the fine chemical, meaning of carbohydrate, preferably fructose and/or raffinose in free or bound form in an organism or a part thereof, as mentioned.
The sequence of b2699 from Escherichia coli K12 has been published in Blattner et al., Science 277(5331), 1453-1474, 1997, and its activity is being defined as a DNA strand exchange and recombination protein with protease and nuclease activity. Accordingly, in one embodiment, the process of the present invention comprises the use of a gene product with an activity of the recombination protein recA superfamily, preferably a protein with a DNA strand exchange and recombination protein with protease and nuclease activity or its homolog, e.g. as shown herein, from Escherichia coli K12 or its homolog, e.g. as shown herein, for the production of the fine chemical, meaning of carbohydrate, preferably myo-inositol and/or raffinose in free or bound form in an organism or a part thereof, as mentioned.
The sequence of b3172 from Escherichia coli K12 has been published in Blattner et al., Science 277(5331), 1453-1474, 1997, and its activity is being defined as a argininosuccinate synthetase. Accordingly, in one embodiment, the process of the present invention comprises the use of a gene product with an activity of the argininosuccinate synthetase superfamily, preferably a protein with a argininosuccinate synthetase activity or its homolog, e.g. as shown herein, from Escherichia coli K12 or its homolog, e.g. as shown herein, for the production of the fine chemical, meaning of carbohydrate, preferably myo-inositol in free or bound form in an organism or a part thereof, as mentioned.
The sequence of b3231 from Escherichia coli K12 has been published in Blattner et al., Science 277(5331), 1453-1474, 1997, and its activity is being defined as a 50S ribosomal subunit protein L13. Accordingly, in one embodiment, the process of the present invention comprises the use of a gene product with an activity of the Escherichia coli ribosomal protein L13 superfamily, preferably a protein with a 50S ribosomal subunit protein L13 activity or its homolog, e.g. as shown herein, from Escherichia coli K12 or its homolog, e.g. as shown herein, for the production of the fine chemical, meaning of carbohydrate, preferably starch and/or cellulose in free or bound form in an organism or a part thereof, as mentioned.
The sequence of b3430 from Escherichia coli K12 has been published in Blattner et al., Science 277(5331), 1453-1474, 1997, and its activity is being defined as a glucose-1-phosphate adenylyltransferase. Accordingly, in one embodiment, the process of the present invention comprises the use of a gene product with an activity of the glucose-1-phosphate adenylyltransferase superfamily, preferably a protein with a glucose-1-phosphate adenylyltransferase activity or its homolog, e.g. as shown herein, from Escherichia coli K12 or its homolog, e.g. as shown herein, for the production of the fine chemical, meaning of carbohydrate, preferably myo-inositol in free or bound form in an organism or a part thereof, as mentioned.
The sequence of b3601 from Escherichia coli K12 has been published in Blattner et al., Science 277(5331), 1453-1474, 1997, and its activity is being defined as a transcriptional repressor for mannitol utilization. Accordingly, in one embodiment, the process of the present invention comprises the use of a protein with a transcriptional repressor activity or its homolog, e.g. as shown herein, from Escherichia coli K12 or its homolog, e.g. as shown herein, for the production of the fine chemical, meaning of carbohydrate, preferably raffinose in free or bound form in an organism or a part thereof, as mentioned.
The sequence of b4129 from Escherichia coli K12 has been published in Blattner et al., Science 277(5331), 1453-1474, 1997, and its activity is being defined as a (inducible) lysine tRNA synthetase. Accordingly, in one embodiment, the process of the present invention comprises the use of a gene product with an activity of the lysine-tRNA ligase superfamily, preferably a protein with a lysine tRNA synthetase activity or its homolog, e.g. as shown herein, from Escherichia coli K12 or its homolog, e.g. as shown herein, for the production of the fine chemical, meaning of carbohydrate, preferably myo-inositol in free or bound form in an organism or a part thereof, as mentioned.
The sequence of b4239 from Escherichia coli K12 has been published in Blattner et al., Science 277(5331), 1453-1474, 1997, and its activity is being defined as a trehalose-6-P hydrolase. Accordingly, in one embodiment, the process of the present invention comprises the use of a gene product with an activity of the alpha-glucosidase superfamily with alpha-amylase core homology, preferably a protein with a trehalose-6-P hydrolase activity or its homolog, e.g. as shown herein, from Escherichia coli K12 or its homolog, e.g. as shown herein, for the production of the fine chemical, meaning of carbohydrate, preferably fructose, glucose and/or sucrose in free or bound form in an organism or a part thereof, as mentioned.
The sequence of b4327 from Escherichia coli K12 has been published in Blattner et al., Science 277(5331), 1453-1474, 1997, and its activity is being defined as a HTH-type transcriptional regulator with periplasmic binding protein domain (LysR family). Accordingly, in one embodiment, the process of the present invention comprises the use of a gene product with an activity of the protein b2409 superfamily with alpha-amylase core homology, preferably a protein with a HTH-type transcriptional regulator with periplasmic binding protein domain (LysR family) activity or its homolog, e.g. as shown herein, from Escherichia coli K12 or its homolog, e.g. as shown herein, for the production of the fine chemical, meaning of carbohydrate, preferably fructose in free or bound form in an organism or a part thereof, as mentioned.
The sequence of b4327 from Escherichia coli K12 has been published in Blattner et al., Science 277(5331), 1453-1474, 1997, and its activity is being defined as a HTH-type transcriptional regulator with periplasmic binding protein domain (LysR family). Accordingly, in one embodiment, the process of the present invention comprises the use of a gene product with an activity of the protein b2409 superfamily with alpha-amylase core homology, preferably a protein with a HTH-type transcriptional regulator with periplasmic binding protein domain (LysR family) activity or its homolog, e.g. as shown herein, from Escherichia coli K12 or its homolog, e.g. as shown herein, for the production of the fine chemical, meaning of carbohydrate, preferably fructose in free or bound form in an organism or a part thereof, as mentioned.
The sequence of YBR184w from Saccharomyces cerevisiae has been published in Goffeau, Science 274 (5287), 546-547, 1996, and in Feldmann, EMBO J., 13, 5795-5809, 1994, and its activity is is not yet characterized. Accordingly, in one embodiment, the process of the present invention comprises the use of a gene product with an activity of the protein YBR184w superfamily, preferably a protein with a protein YBR184w activity or its homolog, e.g. as shown herein, from Saccaromyces cerevisiae or its homolog, e.g. as shown herein, for the production of the fine chemical, meaning of carbohydrate, preferably raffinose in free or bound form in an organism or a part thereof, as mentioned.
The sequence of YBR204C from Saccharomyces cerevisiae has been published in Goffeau, Science 274 (5287), 546-547, 1996, and in Feldmann, EMBO J., 13, 5795-5809, 1994, and its activity is being defined as a peroxisomal lipase. Accordingly, in one embodiment, the process of the present invention comprises the use of a protein with a peroxisomal lipase activity or its homolog, e.g. as shown herein, from Saccaromyces cerevisiae or its homolog, e.g. as shown herein, for the production of the fine chemical, meaning of carbohydrate, preferably myo-inositol in free or bound form in an organism or a part thereof, as mentioned.
The sequence of YDR112w from Saccharomyces cerevisiae (accession number S69752), and its activity is not yet defined. Accordingly, in one embodiment, the process of the present invention comprises the use of a protein with a YDR112w activity or its homolog, e.g. as shown herein, from Saccaromyces cerevisiae or its homolog, e.g. as shown herein, for the production of the fine chemical, meaning of carbohydrate, preferably myo-inositol in free or bound form in an organism or a part thereof, as mentioned.
The sequence of YER174C from Saccharomyces cerevisiae has been published in Dietrich, Nature 387 (6632 Suppl), 78-81, 1997, and Goffeau, Science 274 (5287), 546-547, 1996, and its activity is being defined as a hydroperoxide and superoxide-radical responsive glutathione-dependent oxidoreductase. Accordingly, in one embodiment, the process of the present invention comprises the use of a gene product with an activity of the thioredoxin superfamily, preferably a protein with a hydroperoxide and superoxide-radical responsive glutathione-dependent oxidoreductase activity or its homolog, e.g. as shown herein, from Saccaromyces cerevisiae or its homolog, e.g. as shown herein, for the production of the fine chemical, meaning of carbohydrate, preferably starch and/or cellulose in free or bound form in an organism or a part thereof, as mentioned.
The sequence of YGR261C from Saccharomyces cerevisiae has been published in Tettelin, Nature 387 (6632 Suppl), 81-84, 1997, and Goffeau, Science 274 (5287), 546-547, 1996, and its activity is being defined as a clathrin assembly complex beta adaptin component. Accordingly, in one embodiment, the process of the present invention comprises the use of a gene product with an activity of the beta-adaptin superfamily, preferably a protein with a clathrin assembly complex beta adaptin component activity or its homolog, e.g. as shown herein, from Saccaromyces cerevisiae or its homolog, e.g. as shown herein, for the production of the fine chemical, meaning of carbohydrate, preferably myo-inositol and/or raffinose in free or bound form in an organism or a part thereof, as mentioned.
The sequence of YIL150C from Saccharomyces cerevisiae has been published in Goffeau st al., Science 274 (5287), 546-547, 1996 and Churcher et al., Nature 387 (6632 Suppl), 84-87, 1997, and its activity is being defined as a chromatin binding protein, required for S-phase (DNA synthesis) initiation or completion. Accordingly, in one embodiment, the process of the present invention comprises the use of a protein with a chromatin binding protein, required for S-phase (DNA synthesis) initiation or completion activity or its homolog, e.g. as shown herein, from Saccaromyces cerevisiae or its homolog, e.g. as shown herein, for the production of the fine chemical, meaning of carbohydrate, preferably myo-inositol in free or bound form in an organism or a part thereof, as mentioned.
The sequence of YJL072C from Saccharomyces cerevisiae has been published in Goffeau,A., Science 274 (5287), 546-547, 1996 and Galibert,F., EMBO J. 15 (9), 2031-2049, 1996, and its activity is being defined as a subunit of the GINS complex required for chromosomal DNA replication. Accordingly, in one embodiment, the process of the present invention comprises the use of a gene product with an activity of the Saccharomyces cerevisiae membrane protein YJL072c superfamily, preferably a protein with a subunit of the GINS complex required for chromosomal DNA replication activity or its homolog, e.g. as shown herein, from Saccaromyces cerevisiae or its homolog, e.g. as shown herein, for the production of the fine chemical, meaning of carbohydrate, preferably glucose in free or bound form in an organism or a part thereof, as mentioned.
The sequence of YJL099W from Saccharomyces cerevisiae has been published in Goffeau,A., Science 274 (5287), 546-547, 1996 and Galibert,F., EMBO J. 15 (9), 2031-2049, 1996, and its activity is being defined as a protein involved in chitin biosynthesis and/or its regulation. Accordingly, in one embodiment, the process of the present invention comprises the use of a gene product with an activity of a protein involved in chitin biosynthesis and/or its regulation or its homolog, e.g. as shown herein, from Saccaromyces cerevisiae or its homolog, e.g. as shown herein, for the production of the fine chemical, meaning of carbohydrate, preferably myo-inositol in free or bound form in an organism or a part thereof, as mentioned.
The sequence of YOR044W from Saccharomyces cerevisiae has been published in Dujon, Nature 387 (6632 Suppl), 98-102, 1997, and Goffeau, Science 274 (5287), 546-547, 1996, and its activity is not yet characterized. Accordingly, in one embodiment, the process of the present invention comprises the use of a gene product with an activity of the Saccharomyces cerevisiae membrane protein YOR044w superfamily, preferably a protein with a YOR044W activity or its homolog, e.g. as shown herein, from Saccaromyces cerevisiae or its homolog, e.g. as shown herein, for the production of the fine chemical, meaning of carbohydrate, preferably myo-inositol in free or bound form in an organism or a part thereof, as mentioned.
The sequence of YOR350C from Saccharomyces cerevisiae has been published in Dujon, Nature 387 (6632 Suppl), 98-102, 1997, and Goffeau, Science 274 (5287), 546-547, 1996, and its activity is not yet characterized. Accordingly, in one embodiment, the process of the present invention comprises the use of a gene product with an activity of the Saccharomyces cerevisiae MNE1 protein superfamily, preferably a protein with a YOR350C activity or its homolog, e.g. as shown herein, from Saccaromyces cerevisiae or its homolog, e.g. as shown herein, for the production of the fine chemical, meaning of carbohydrate, preferably myo-inositol in free or bound form in an organism or a part thereof, as mentioned.
The sequence of b0050 (Accession number NP_414592) from Escherichia coli K12 has been published in Blattner et al., Science 277 (5331), 1453-1474, 1997, and its activity is being defined as a conserved protein potentially involved in protein protein interaction. Accordingly, in one embodiment, the process of the present invention comprises the use of a gene product with an activity of apaG protein superfamily, preferably a protein with the activity of a conserved protein potentially involved in protein-protein interaction from E. coli or its homolog, e.g. as shown herein, for the production of the fine chemical, meaning of starch and/or cellulose, particular for increasing the amount of starch and/or cellulose, preferably starch and/or cellulose in free or bound form in an organism or a part thereof, as mentioned.The sequence of b0124 (Accession number NP_414666) from Escherichia coli K12 has been published in Blattner et al., Science 277 (5331), 1453-1474, 1997, and its activity is being defined as a glucose dehydrogenase. Accordingly, in one embodiment, the process of the present invention comprises the use of a gene product with an activity of glucose dehydrogenase (pyrroloquinoline-quinone) superfamily, preferably a protein with the activity of a glucose dehydrogenase from E. coli or its homolog, e.g. as shown herein, for the production of the fine chemical, meaning of fructose, particular for increasing the amount of fructose, preferably fructose in free or bound form in an organism or a part thereof, as mentioned.
The sequence of b0149 (Accession number NP_414691) from Escherichia coli K12 has been published in Blattner et al., Science 277 (5331), 1453-1474, 1997, and its activity is being defined as a bifunctional penicillin-binding protein 1 b: glycosyl transferase (N-terminal); transpeptidase (C-terminal). Accordingly, in one embodiment, the process of the present invention comprises the use of a gene product with an activity of penicillin-binding protein 1 B superfamily, preferably a protein with the activity of a bifunctional penicillin-binding protein 1b: glycosyl transferase (N-terminal); transpeptidase (C-terminal) from E. coli or its homolog, e.g. as shown herein, for the production of the fine chemical, meaning of fructose, in particular for increasing the amount of fructose, preferably fructose in free or bound form in an organism or a part thereof, as mentioned.
The sequence of b1318 from Escherichia coli K12 (Accession number NP_415834) has been published in Blattner, Science 277(5331), 1453-1474, 1997, and its activity is beeing defined as a sugar transport protein (of the ABC superfamily). Accordingly, in one embodiment, the process of the present invention comprises the use of a gene product with the activity of the inner membrane protein malK (with ATP-binding cassette homology) superfamily, preferably a protein with a sugar transport protein (of the ABC superfamily) activity from E. coli or its homolog, e.g. as shown herein, for the production of the fine chemical, meaning of fructose, in particular for increasing the amount of fructose in free or bound form in an organism or a part thereof, as mentioned.
The sequence of b1463 from Escherichia coli K12 (Accession number NP_415980) has been published in Blattner, Science 277(5331), 1453-1474, 1997, and its activity is beeing defined as a N-hydroxyarylamine O-acetyltransferase. Accordingly, in one embodiment, the process of the present invention comprises the use of a gene product with the activity of the arylamine acetyltransferase superfamily, preferably a protein with a N-hydroxyarylamine O-acetyltransferase activity from E. coli or its homolog, e.g. as shown herein, for the production of the fine chemical, meaning of fructose and/of glucose and/or myo-inositol, in particular for increasing the amount of fructose, in particular for increasing the amount of glucose, in particular for increasing the amount of myo-inositol, in particular for increasing the amount of fructose and glucose, in particular for increasing the amount of fructose and myo-inositol, in particular for increasing the amount of glucose and myo-inositol, in particular for increasing the amount of fructose and glucose and myo-inositol, preferably of fructose and/or glucose and/or myo-inositol in free or bound form in an organism or a part thereof, as mentioned.
The sequence of b1539 (Accession number NP_416057) from Escherichia coli K12 has been published in Blattner et al., Science 277 (5331), 1453-1474, 1997, and its activity is being defined as a NADP-dependent L-serine/L-allo-threonine dehydrogenase. Accordingly, in one embodiment, the process of the present invention comprises the use of a gene product with an activity of ribitol dehydrogenase, short-chain alcohol dehydrogenase homology superfamily, preferably a protein with the activity of a NADP-dependent L-serine/L-allo-threonine dehydrogenase from E. coli or its homolog, e.g. as shown herein, for the production of the fine chemical, meaning of starch and/or cellulose, particular for increasing the amount of starch and/or cellulose, preferably starch and/or cellulose in free or bound form in an organism or a part thereof, as mentioned.
The sequence of b1736 (Accession number NP_416250) from Escherichia coli K12 has been published in Blattner et al., Science 277 (5331), 1453-1474, 1997, and its activity is being defined as a PEP-dependent phosphotransferase enzyme, cellobiose/arbutin/salicin sugar-specific protein. Accordingly, in one embodiment, the process of the present invention comprises the use of a gene product with an activity of phosphotransferase system lactose-specific enzyme II, factor III superfamily, preferably a protein with the activity of a PEP-dependent phosphotransferase enzyme, cellobiose/arbutin/salicin sugar-specific protein from E. coli or its homolog, e.g. as shown herein, for the production of the fine chemical, meaning of glucose, in particular for increasing the amount of glucose, preferably glucose in free or bound form in an organism or a part thereof, as mentioned.
The sequence of b1961 (Accession number NP_416470) from Escherichia coli K12 has been published in Blattner et al., Science 277 (5331), 1453-1474, 1997, and its activity is being defined as a DNA cytosine methylase. Accordingly, in one embodiment, the process of the present invention comprises the use of a gene product with an activity of site-specific methyltransferase (cytosine-specific) EcoRII superfamily, preferably a protein with the activity of a DNA cytosine methylase from E. coli or its homolog, e.g. as shown herein, for the production of the fine chemical, meaning of myo-inositol, in particular for increasing the amount of myo-inositol, preferably myo-inositol in free or bound form in an organism or a part thereof, as mentioned.
The sequence of b2491 (Accession number NP_416986) from Escherichia coli K12 has been published in Blattner et al., Science 277 (5331), 1453-1474, 1997, and its activity is being defined as a transcriptional activator for expression of hydrogenase 4 genes, interacts with sigma 54 (EBP family). Accordingly, in one embodiment, the process of the present invention comprises the use of a gene product with an activity of RNA polymerase sigma factor interaction domain homology, transcription activator fhlA, nif-specific regulatory protein, response regulator homology superfamily, preferably a protein with the activity of a transcriptional activator for expression of hydrogenase 4 genes, interacts with sigma 54 (EBP family) from E. coli or its homolog, e.g. as shown herein, for the production of the fine chemical, meaning of fructose and/or glucose, in particular for increasing the amount of fructose, in particular for increasing the amount of glucose, in particular for increasing the amount of fructose and glucose, preferably fructose and/or glucose in free or bound form in an organism or a part thereof, as mentioned.
The sequence of b3260 (Accession number NP_417726) from Escherichia coli K12 has been published in Blattner et al., Science 277 (5331), 1453-1474, 1997, and its activity is being defined as a tRNA-dihydrouridine synthase B. Accordingly, in one embodiment, the process of the present invention comprises the use of a gene product with an activity of transcription regulator yacF superfamily, preferably a protein with the activity of a tRNA-dihydrouridine synthase B from E. coli or its homolog, e.g. as shown herein, for the production of the fine chemical, meaning of fructose, in particular for increasing the amount of fructose, preferably fructose in free or bound form in an organism or a part thereof, as mentioned.
The sequence of b3578 (Accession number YP_026232) from Escherichia coli K12 has been published in Blattner et al., Science 277(5331), 1453-1474, 1997, and its activity is being defined as putative component of transport system. Accordingly, in one embodiment, the process of the present invention comprises the use of a putative component of transport system from E. coli or its homolog, e.g. as shown herein, for the production of the fine chemical, meaning of fructose and/or glucose and/or raffinose, in particular for increasing the amount of fructose, in particular for increasing the amount of glucose, in particular for increasing the amount of raffinose, in particular for increasing the amount of fructose and glucose, in particular for increasing the amount of fructose and raffinose, in particular for increasing the amount of glucose and raffinose, in particular for increasing the amount of fructose, glucose and raffinose, preferably fructose and/or glucose and/of raffinose in free or bound form in an organism or a part thereof, as mentioned.
The sequence of b3619 (Accession number NP_418076) from Escherichia coli K12 has been published in Blattner et al., Science 277 (5331), 1453-1474, 1997, and its activity is being defined as a ADP-L-glycero-D-mannoheptose-6-epimerase, NAD(P)-binding. Accordingly, in one embodiment, the process of the present invention comprises the use of a gene product with an activity of ADPglyceromanno-heptose 6-epimerase, UDPglucose 4-epimerase homology superfamily, preferably a protein with the activity of a ADP-L-glycero-D-mannoheptose-6-epimerase, NAD(P)-binding from E. coli or its homolog, e.g. as shown herein, for the production of the fine chemical, meaning of fructose and/or glucose, in particular for increasing the amount of fructose, in particular for increasing the amount of glucose, in particular for increasing the amount of fructose and glucose, preferably fructose and/or glucose in free or bound form in an organism or a part thereof, as mentioned.
The sequence of b3919 (Accession number NP_418354) from Escherichia coli K12 has been published in Blattner et al., Science 277 (5331), 1453-1474, 1997, and its activity is being defined as a triosephosphate isomerase. Accordingly, in one embodiment, the process of the present invention comprises the use of a gene product with an activity of triose-phosphate isomerase superfamily, preferably a protein with the activity of a triosephosphate isomerase from E. coli or its homolog, e.g. as shown herein, for the production of the fine chemical, meaning of starch and/or cellulose, in particular for increasing the amount of starch and/or cellulose, preferably starch and/or cellulose in free or bound form in an organism or a part thereof, as mentioned.
The sequence of b4074 (Accession number NP_418498) from Escherichia coli K12 has been published in Blattner et al., Science 277 (5331), 1453-1474, 1997, and its activity is being defined as a cytochrome c-type biogenesis protein. Accordingly, in one embodiment, the process of the present invention comprises the use of a gene product with an activity of nrfE protein superfamily, preferably a protein with the activity of a Cytochrome c-type biogenesis protein from E. coli or its homolog, e.g. as shown herein, for the production of the fine chemical, meaning of myo-inositol, in particular for increasing the amount of myo-inositol, preferably myo-inositol in free or bound form in an organism or a part thereof, as mentioned.
The sequence of b4122 (Accession number NP_418546) from Escherichia coli K12 has been published in Blattner et al., Science 277 (5331), 1453-1474, 1997, and its activity is being defined as a fumarase B (fumarate hydratase Class I). Accordingly, in one embodiment, the process of the present invention comprises the use of a gene product with an activity of iron-dependent fumarate hydratase and/or iron-dependent tartrate dehydratase alpha chain homology superfamily, preferably a protein with the activity of a fumarase B (fumarate hydratase Class I) from E. coli or its homolog, e.g. as shown herein, for the production of the fine chemical, meaning of fructose, in particular for increasing the amount of fructose, preferably fructose in free or bound form in an organism or a part thereof, as mentioned.
The sequence of b4232 from Escherichia coli K12 (ACCESSION No. NP_416986) has been published in Blattner, Science 277(5331), 1453-1474, 1997, and its activity is beeing defined as a fructose-1,6-bisphosphatase. Accordingly, in one embodiment, the process of the present invention comprises the use of a "fructose-1,6-bisphosphatase" from E. coli or its homolog, e.g. as shown herein, for the production of the fine chemical, meaning of methionin, in particular for increasing the amount of methionine in free or bound form in an organism or a part thereof, as mentioned. In one embodiment, in the process of the present invention the activity of a protein of the superfamily "fructose-bisphosphatase", preferably having an activity in C-compound and carbohydrate metabolism, C-compound and carbohydrate utilization, energy, glycolysis and gluconeogenesis, plastid, photosynthesis, more preferred having an "fructose-1,6-bisphosphatase"-activity, is increased or generated, e.g. from E. coli or a homolog thereof. Accordingly, in one embodiment, in the process of the present invention the activity of a "fructose-1,6-bisphosphatase" or its homolog is increased for the production of the fine chemical, meaning of starch and/or cellulose, in particular for increasing the amount of starch and/or cellulose in free or bound form in an organism or a part thereof, as mentioned.
The sequence of YHR072W-A from Saccharomyces cerevisiae (ACCESSION NP_058135) has been published in Goffeau et al., Science 274 (5287), 546-547, 1996 and its activity is beeing defined as that of a constituent of small nucleolar ribonucleoprotein particles containing H/ACA-type snoRNAs, which are required for pseudouridylation and processing of pre-18S rRNA. Accordingly, in one embodiment, the process of the present invention comprises the use of a constituent of small nucleolar ribonucleoprotein particles containing H/ACA-type snoRNAs, which are required for pseudouridylation and processing of pre-18S rRNA from Saccaromyces cerevisiae or its homolog, e.g. as shown herein, for the production of the fine chemical, meaning of myo-inositol, in particular for increasing the amount of myo-inositol, preferably myo-inositol in free or bound form in an organism or a part thereof, as mentioned.

**[0023.0.25.25]** Homologues ( = homologs) of the present gene products can be derived from any organisms as long as the homologue confers the herein mentioned activity, in particular, confers an increase in the respective fine chemical amount or content.
In one embodiment, the homolog of the any one of the polypeptides indicated in Table II, column 3, line 294 for starch and/or cellulose; line 308 for glucose; lines 316 to 322 and/or 625 for myo-inositol; lines 330 to 331 and/or 626 for raffinose resp. , is a homolog having the same or a similar acitivity, resp. In particular an increase of activity confers an increase in the content of the respective fine chemical in the organisms. In one embodiment, the homolog is a homolog with a sequence as indicated in Table I or II, column 7, line 294 for starch and/or cellulose; line 308 for glucose; lines 316 to 322 and/or 625 for myo-inositol; lines 330 to 331 and/or 626 for raffinose resp.. In one embodiment, the homolog of one of the polypeptides indicated in Table II, column 3, line 294 for starch and/or cellulose; line 308 for glucose; lines 316 to 322 and/or 625 for myo-inositol; lines 330 to 331 and/or 626 for raffinose resp., is derived from an eukaryotic. In one embodiment, the homolog is derived from Fungi. In one embodiment, the homolog of a polypeptide indicated in Table II, column 3, line 294 , is derived from Ascomyceta. In one embodiment, the homolog of a polypeptide indicated in Table II, column 3, line 294, is derived from Saccharomycotina. In one embodiment, the homolog of a polypeptide indicated in Table II, column 3, line 294 for starch and/or cellulose; line 308 for glucose; lines 316 to 322 and/or 625 for myo-inositol; lines 330 to 331 and/or 626 for raffinose resp., is derived from Saccharomycetes. In one embodiment, the homolog of a polypeptide indicated in Table II, column 3, line 294 for starch and/or cellulose; line 308 for glucose; lines 316 to 322 and/or 625 for myo-inositol; lines 330 to 331 and/or 626 for raffinose resp., is a homolog being derived from Saccharomycetales. In one embodiment, the homolog of a polypeptide indicated in Table II, column 3, line 294 for starch and/or cellulose; line 308 for glucose; lines 316 to 322 and/or 625 for myo-inositol; lines 330 to 331 and/or 626 for raffinose resp. is a homolog having the same or a similar acitivity being derived from Saccharomycetaceae. In one embodiment, the homolog of a polypeptide indicated in Table II, column 3, line 294 for starch and/or cellulose; line 308 for glucose; lines 316 to 322 and/or 625 for myo-inositol; lines 330 to 331 and/or 626 for raffinose resp., is a homolog having the same or a similar acitivity being derived from Saccharomycetes.
In one embodiment, the homolog of the any one of the polypeptides indicated in Table II, column 3, lines 290 to 293 is a homolog having the same or a similar acitivity. In particular an increase of activity confers an increase in the content of the respective fine chemical in the organisms preferably of carbohydrate, preferably starch and/or cellulose. In one embodiment, the homolog is a homolog with a sequnence as indicated in Table I or II, column 7, lines 290 to 293, resp. In one embodiment, the homolog of one of the polypeptides indicated in Table II, column 3, 290 to 293 is derived from an bacteria. In one embodiment, the homolog of a polypeptide indicated in Table II, column 3, lines 290 to 293 is derived from Proteobacteria.. In one embodiment, the homolog of a polypeptide indicated in Table II, column 3, lines 290 to 293 is a homolog having the same or a similar acitivity being derived from Gammaproteobacteria. In one embodiment, the homolog of a polypeptide indicated in
Table II, column 3, lines 290 to 293 is derived from Enterobacteriales. In one embodiment, the homolog of a polypeptide indicated in Table II, column 3, lines 290 to 293 is a homolog being derived from Enterobacteriaceae. In one embodiment, the homolog of a polypeptide indicated in Table II, column 3, lines 290 to 293 is a homolog having the same or a similar acitivity and being derived from Escherichia.
In one embodiment, the homolog of the any one of the polypeptides indicated in Table II, column 3, lines 290 to 293 and/or 604 to 607 for starch and/or cellulose; lines 295 to 302 and/or 608 to 616 for fructose; lines 303 to 307 and/or 617 to 621 for glucose; lines 309 to 315 and/or 622 to 624 for myo-inositol; lines 323 to 329 for raffinose; lines 332 to 333 for sucrose, resp.is a homolog having the same or a similar acitivity. In particular an increase of activity confers an increase in the content of the respective fine chemical in the organisms preferably of carbohydrate, preferably fructose. In one embodiment, the homolog is a homolog with a sequnence as indicated in Table I or II, column 7, lines 290 to 293 and/or 604 to 607 for starch and/or cellulose; lines 295 to 302 and/or 608 to 616 for fructose; lines 303 to 307 and/or 617 to 621 for glucose; lines 309 to 315 and/or 622 to 624 for myo-inositol; lines 323 to 329 for raffinose; lines 332 to 333 for sucrose, resp.. In one embodiment, the homolog of one of the polypeptides indicated in Table II, column 3, lines 290 to 293 and/or 604 to 607 for starch and/or cellulose; lines 295 to 302 and/or 608 to 616 for fructose; lines 303 to 307 and/or 617 to 621 for glucose; lines 309 to 315 and/or 622 to 624 for myo-inositol; lines 323 to 329 for raffinose; lines 332 to 333 for sucrose, resp.is derived from an bacteria. In one embodiment, the homolog of a polypeptide indicated in Table II, column 3, lines 290 to 293 and/or 604 to 607 for starch and/or cellulose; lines 295 to 302 and/or 608 to 616 for fructose; lines 303 to 307 and/or 617 to 621 for glucose; lines 309 to 315 and/or 622 to 624 for myo-inositol; lines 323 to 329 for raffinose; lines 332 to 333 for sucrose, resp. is derived from Proteobacteria.. In one embodiment, the homolog of a polypeptide indicated in Table II, column 3, lines 290 to 293 and/or 604 to 607 for starch and/or cellulose; lines 295 to 302 and/or 608 to 616 for fructose; lines 303 to 307 and/or 617 to 621for glucose; lines 309 to 315 and/or 622 to 624 for myo-inositol; lines 323 to 329 for raffinose; lines 332 to 333 for sucrose, resp.is a homolog having the same or a similar acitivity being derived from Gammaproteobacteria. In one embodiment, the homolog of a polypeptide indicated in Table II, column 3, lines 290 to 293 and/or 604 to 607 for starch and/or cellulose; lines 295 to 302 and/or 608 to 616 for fructose; lines 303 to 307 and/or 617 to 621for glucose; lines 309 to 315 and/or 622 to 624 for myo-inositol; lines 323 to 329 for raffinose; lines 332 to 333 for sucrose, resp. is derived from Enterobacteriales. In one embodiment, the homolog of a polypeptide indicated in Table II, column 3, lines 290 to 293 and/or 604 to 607 for starch and/or cellulose; lines 295 to 302 and/or 608 to 616 for fructose; lines 303 to 307 and/or 617 to 621for glucose; lines 309 to 315 and/or 622 to 624 for myo-inositol; lines 323 to 329 for raffinose; lines 332 to 333 for sucrose, resp.is a homolog being derived from Enterobacteriaceae. In one embodiment, the homolog of a polypeptide indicated in Table II, column 3, lines 290 to 293 and/or 604 to 607 for starch and/or cellulose; lines 295 to 302 and/or 608 to 616 for fructose; lines 303 to 307 and/or 617 to 621 for glucose; lines 309 to 315 and/or 622 to 624 for myo-inositol; lines 323 to 329 for raffinose; lines 332 to 333 for sucrose, resp.is a homolog having the same or a similar acitivity and being derived from Escherichia.

**[0023.1.25.25]** Homologs of the polypeptide indicated in Table II, column 3, lines 290 to 333 and/or lines 604 to 626 may be the polypetides encoded by the nucleic acid molecules indicated in Table I, column 7, lines 290 to 333 and/or lines 604 to 626, resp., or may be the polypeptides indicated in Table II, column 7, lines 290 to 333 and/or lines 604 to 626, resp.

**[0024.0.0.9]** see **[0024.0.0.0]**

**[0025.0.25.25]** In accordance with the invention, a protein or polypeptide has the "activity of an protein of the invention", e.g. the activity of a protein indicated in Table II, column 3, lines 290 to 294 and/or 604 to 607 for starch and/or cellulose;
lines 295 to 302 and/or 608 to 616 for fructose;
lines 303 to 308 and/or 617 to 621 for glucose;
lines 309 to 322 and/or 622 to 625 for myo-inositol;
lines 323 to 331 and/or 626 for raffinose;
lines 332 to 333 for sucrose;
, resp., if its *de novo* activity, or its increased expression directly or indirectly leads to increased carbohydrates, preferably polysaccharides, more preferably starch and/or cellulose and/or monosaccharides, more preferably fructose, glucose and/or myo-inositol and/or trisaccharides, more preferably raffinose and/or disaccharides, more preferably sucrose, resp., in the organism or a part thereof, preferably in a cell of said organism. In a preferred embodiment, the protein or polypeptide has the above-mentioned additional activities of a protein indicated in Table II, column 3, lines 290 to 294 and/or 604 to 607 for starch and/or cellulose; lines 295 to 302 and/or 608 to 616 for fructose; lines 303 to 308 and/or 617 to 621 for glucose; lines 309 to 322 and/or 622 to 625 for myo-inositol; lines 323 to 331 and/or 626 for raffinose; lines 332 to 333 for sucrose, resp.. Throughout the specification the activity or preferably the biological activity of such a protein or polypeptide or an nucleic acid molecule or sequence encoding such protein or polypeptide is identical or similar if it still has the biological or enzymatic activity of any one of the proteins indicated in Table II, column 3, lines 290 to 294 and/or 604 to 607 for starch and/or cellulose; lines 295 to 302 and/or 608 to 616 for fructose; lines 303 to 308 and/or 617 to 621 for glucose; lines 309 to 322 and/or 622 to 625 for myo-inositol; lines 323 to 331 and/or 626 for raffinose; lines 332 to 333 for sucrose, resp., or which has at least 10% of the original enzymatic activity, preferably 20%, particularly preferably 30%, most particularly preferably 40% in comparison to any one of the proteins indicated in Table II, column 3, line 294 for starch and/or cellulose; line 308 for glucose; lines 316 to 322 and/or 625 for myo-inositol; lines 330 to 331 and/or 626 for raffinose resp.of Saccharomyces cerevisiae and/or any one of the proteins indicated in Table II, column 3, lines 290 to 293 and/or 604 to 607 for starch and/or cellulose; lines 295 to 302 and/or 608 to 616 for fructose; lines 303 to 307 for glucose; lines 309 to 315 and/or 622 to 624 for myo-inositol; lines 323 to 329 for raffinose; lines 332 to 333 for sucrose, resp. of E. coli K12.

**[0025.1.0.25]: see [0025.1.0.0]**

**[0026.0.0.25]** to **[0033.0.0.25]: see [0026.0.0.0]** to **[0033.0.0.0]**

**[0034.0.25.25]** Preferably, the reference, control or wild type differs form the subject of the present invention only in the cellular activity of the polypeptide of the invention, e.g. as result of an increase in the level of the nucleic acid molecule of the present invention or an increase of the specific activity of the polypeptide of the invention. E.g., it differs by or in the expression level or activity of an protein having the activity of a protein as indicated in Table II, column 3, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp.;, or being encoded by a nucleic acid molecule indicated in Table I, column 5, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp.;, or its homologs, e.g. as indicated in Table I, column 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp.; its biochemical or genetical causes and therefore shows the increased amount of the respective fine chemical.

**[0035.0.0.25]** to **[0044.0.0.25]: see [0035.0.0.0]** to **[0044.0.0.0]**

**[0045.0.25.25]** In one embodiment the activity of the Escherichia coli K12 protein b0019 or its homologs, e.g. the activity of a protein of the Na+/H+-exchanging protein nhaA superfamily is increased, preferably, of a protein having a Na+/H+ antiporter activity, e.g. as indicated in Table I, columns 5 or 7, line 332, is increased conferring an increase of the respective fine chemical, preferably of sucrose, between 22% and 55% or more.
In one embodiment the activity of the Escherichia coli K12 protein b0138 or its homologs, e.g. having a fimbrial-like adhesin protein activity, e.g. as indicated in Table I, columns 5 or 7, line 309, is increased conferring an increase of the respective fine chemical, preferably of myo-inositol, between 32% and 46% or more.
In one embodiment the activity of the Escherichia coli K12 protein b0161 or its homologs, e.g. the activity of a protein of the Helicobacter serine proteinase superfamily is increased, preferably, of a protein having a periplasmic serine protease (heat shock protein) activity, e.g. as indicated in Table I, columns 5 or 7, lines 295, 303 and 323, is increased conferring an increase of the respective fine chemical, preferably of fructose (82% to 1155%), glucose (86% to 338%) and/or raffinose (128% to 197%) resp., between 82% and 1155% or more.
In one embodiment the activity of the Escherichia coli K12 protein b0252 or its homologs, e.g. having a b0252 protein activity and similarity with helicase and ligase proteins, e.g. as indicated in Table I, columns 5 or 7, line 290 is increased conferring an increase of the respective fine chemical, preferably of starch and/or cellulose, between 37% and 53% or more.
In one embodiment the activity of the Escherichia coli K12 protein b0290 or its homologs, e.g. the activity of a protein of the Escherichia coli yagW protein superfamily is increased, preferably, of a protein having a receptor protein activity, e.g. as indicated in Table I, columns 5 or 7, line 310, is increased conferring an increase of the respective fine chemical, preferably of myo-inositol, between 23% and 50% or more.
In one embodiment the activity of the Escherichia coli K12 protein b0695 or its homologs, e.g. the activity of a protein having homology with the sensor histidine kinase superfamily is increased, preferably, of a protein having a activity of sensory histidine kinase in two-component regulatory system, e.g. as indicated in Table I, columns 5 or 7, line 296, is increased conferring an increase of the respective fine chemical, preferably of fructose, between 69% and 1046% or more.
In one embodiment the activity of the Escherichia coli K12 protein b0730 or its homologs, e.g. the activity of a protein of the transcription regulator GntR superfamily is increased, preferably, of a protein having a activity of a transcriptional regulator of succinyICoA synthetase operon and fatty acyl response regulator, e.g. as indicated in Table I, columns 5 or 7, line 324, is increased conferring an increase of the respective fine chemical, preferably of raffinose, between 93% and 616% or more.
In one embodiment the activity of the Escherichia coli K12 protein b1430 or its homologs, e.g. the activity of a protein of the hemagglutinin hag1 superfamily is increased, preferably, of a protein having a activity of putative S-adenosyl-L-methionine-dependent methyltransferase conferring tellurite resistance, e.g. as indicated in Table I, columns 5 or 7, line 291, is increased conferring an increase of the respective fine chemical, preferably of starch and/or cellulose, between 31% and 58% or more.
In one embodiment the activity of the Escherichia coli K12 protein b1693 or its homologs, e.g. the activity of a protein of the 3-dehydroquinate dehydratase superfamily is increased, preferably, of a protein having a 3-dehydroquinate dehydratase activity, e.g. as indicated in Table I, columns 5 or 7, line 292, is increased conferring an increase of the respective fine chemical, preferably of starch and/or cellulose, between 34% and 116% or more.
In one embodiment the activity of the Escherichia coli K12 protein b1701 or its homologs, e.g. the activity of a protein with homology to the 4-coumarate-CoA ligase and/or acetate-CoA ligase superfamily is increased, preferably, of a protein having a activity of a CoenzymeA-dependent ligase with firefly luciferase-like ATPase Domain, e.g. as indicated in Table I, columns 5 or 7, line 325, is increased conferring an increase of the respective fine chemical, preferably of raffinose, between 57% and 958% or more.
In one embodiment the activity of the Escherichia coli K12 protein b1708 or its homologs, e.g. the activity of a protein of the protein HI1314 superfamily is increased, preferably, of a protein having a lipoprotein activity, e.g. as indicated in Table I, columns 5 or 7, lines 297 and 304, is increased conferring an increase of the respective fine chemical, preferably of fructose (77% to 2664%) and/or glucose (62% to 942%), between 62% and 2664% or more.
In one embodiment the activity of the Escherichia coli K12 protein b1886 or its homologs, e.g. the activity of a protein of the methyl-accepting chemotaxis protein superfamily is increased, preferably, of a protein having a methyl-accepting chemotaxis protein II and/or aspartate sensor receptor activity, e.g. as indicated in Table I, columns 5 or 7, line 326, is increased conferring an increase of the respective fine chemical, preferably of raffinose, between 64% and 391% or more.
In one embodiment the activity of the Escherichia coli K12 protein b1926 or its homologs, e.g. having a flagellar protein fliT activity, e.g. as indicated in Table I, columns 5 or 7, lines 298 and 305, is increased conferring an increase of the respective fine chemical, preferably of fructose (86% to 193%) and/or glucose (63% to 88%), between 63% and 193% or more.
In one embodiment the activity of the Escherichia coli K12 protein b2023 or its homologs, e.g. the activity of a protein of the amidotransferase hisH superfamily with trpG homology is increased, preferably, of a protein having a activity of a glutamine amidotransferase subunit of imidazole glycerol phosphate synthase heterodimer, e.g. as indicated in Table I, columns 5 or 7, line 311, is increased conferring an increase of the respective fine chemical, preferably of myo-inositol, between 31 % and 110% or more.
In one embodiment the activity of the Escherichia coli K12 protein b2597 or its homologs, e.g. the activity of a protein of the Pseudomonas putida hypothetical protein rpoX superfamily is increased, preferably, of a protein having a activity of a ribosome associated factor, e.g. as indicated in Table I, columns 5 or 7, line 299, is increased conferring an increase of the respective fine chemical, preferably of fructose, between 90% and 105% or more.
In one embodiment the activity of the Escherichia coli K12 protein b2599 or its homologs, e.g. the activity of a protein of the pheA bifunctional enzyme superfamily with prephenate dehydratase homology is increased, preferably, of a proteinhaving a activity of a bifunctional enzyme: chorismate mutase P (N-terminal) and prephenate dehydratase (C-terminal), e.g. as indicated in Table I, columns 5 or 7, line 306, is increased conferring an increase of the respective fine chemical, preferably of glucose, between 65% and 171% or more.
In one embodiment the activity of the Escherichia coli K12 protein b2664 or its homologs, e.g. the activity of a protein of the transcription regulator gabP superfamily is increased, preferably, of a protein having a transcriptional repressor with DNA-binding Winged helix domain (GntR familiy) activity, e.g. as indicated in Table I, columns 5 or 7, lines 300 and 327, is increased conferring an increase of the respective fine chemical, preferably of fructose (77% to 4086%) and/or raffinose (72% to 1267%), between 72% and 4086% or more.
In one embodiment the activity of the Escherichia coli K12 protein b2699 or its homologs, e.g. the activity of a protein of the recombination protein recA superfamily is increased, preferably, of a protein having a activity of a DNA strand exchange and recombination protein with protease and nuclease activity, e.g. as indicated in Table I, columns 5 or 7, lines 312 and 328, is increased conferring an increase of the respective fine chemical, preferably of myo-inositol (86% to 690%) and/or raffinose (61% to 2408%), between 61% and 2408% or more.
In one embodiment the activity of the Escherichia coli K12 protein b3172 or its homologs, e.g. the activity of a protein of the argininosuccinate synthase superfamily is increased, preferably, of a protein having a argininosuccinate synthetase activity, e.g. as indicated in Table I, columns 5 or 7, line 313, is increased conferring an increase of the respective fine chemical, preferably of myo-inositol, between 26% and 144% or more.
In one embodiment the activity of the Escherichia coli K12 protein b3231 or its homologs, e.g. the activity of a protein of the Escherichia coli ribosomal protein L13 superfamily is increased, preferably, of a protein having a 50S ribosomal subunit protein L13 activity, e.g. as indicated in Table I, columns 5 or 7, line 293, is increased conferring an increase of the respective fine chemical, preferably of starch and/or cellulose, between 31% and 74% or more.
In one embodiment the activity of the Escherichia coli K12 protein b3430 or its homologs, e.g. the activity of a protein of the glucose-1-phosphate adenylyltransferase superfamily is increased, preferably, of a protein having a glucose-1-phosphate adenylyltransferase activity, e.g. as indicated in Table I, columns 5 or 7, line 314, is increased conferring an increase of the respective fine chemical, preferably of myo-inositol, between 34% and 116% or more.
In one embodiment the activity of the Escherichia coli K12 protein b3601 or its homologs, e.g. having a transcriptional repressor for mannitol utilization activity, e.g. as indicated in Table I, columns 5 or 7, line 329, is increased conferring an increase of the respective fine chemical, preferably of raffinose, between 84% and 135% or more.
In one embodiment the activity of the Escherichia coli K12 protein b4129 or its homologs, e.g. the activity of a protein of the lysine-tRNA ligase superfamily is increased, preferably, of a protein having a (inducible) lysine tRNA synthetase activity, e.g. as indicated in Table I, columns 5 or 7, line 315, is increased conferring an increase of the respective fine chemical, preferably of myo-inositol, between 29% and 48% or more.
In one embodiment the activity of the Escherichia coli K12 protein b4239 or its homologs, e.g. the activity of a protein of the alpha-glucosidase superfamily with alpha-amylase core homology is increased, preferably, of a protein having a trehalose-6-P hydrolase activity, e.g. as indicated in Table I, columns 5 or 7, lines 301, 307 and 333, is increased conferring an increase of the respective fine chemical, preferably of fructose (321% to 768%), glucose (100% to 385%) and/or sucrose (64% to 169%) resp., between 64% and 768% or more.
In one embodiment the activity of the Escherichia coli K12 protein b4327 or its homologs, e.g. the activity of a protein of the protein b2409 superfamily is increased, preferably, of a protein having a HTH-type transcriptional regulator with periplasmic binding protein domain (LysR family) activity, e.g. as indicated in Table I, columns 5 or 7, line 302, is increased conferring an increase of the respective fine chemical, preferably of fructose, between 97% and 275% or more.
In one embodiment, the activity of the Saccharomyces cerevisiae protein YBR184W or its homologs, e.g. the activity of a YBR184W protein superfamily-protein is increased, preferably, an activity of YBR184W protein, e.g. as indicated in Table I, columns 5 or 7, line 330 is increased conferring an increase of the respective fine chemical, preferably of raffinose between 87% and 478% or more.
In one embodiment, the activity of the Saccharomyces cerevisiae protein YBR204C or its homologs, e.g. an activity of peroxisomal lipase protein, e.g. as indicated in Table I, columns 5 or 7, line 316, is increased conferring an increase of the respective fine chemical, preferably of myo-inositol between 27% and 192% or more.
In one embodiment, the activity of the Saccharomyces cerevisiae protein YDR112W or its homologs, e.g. an activity of YDR112W protein, e.g. as indicated in Table I, columns 5 or 7, line 317, is increased conferring an increase of the respective fine chemical, preferably of myo-inositol between 41% and 90% or more.
In one embodiment, the activity of the Saccharomyces cerevisiae protein YER174C or its homologs, e.g. the activity of a protein of the thioredoxin homology superfamily is increased, preferably, an activity of hydroperoxide and superoxide-radical responsive glutathione-dependent oxidoreductase protein, e.g. as indicated in Table I, columns 5 or 7, line 294 is increased conferring an increase of the respective fine chemical, preferably of starch and/or cellulose between 34% and 84% or more.
In one embodiment, the activity of the Saccharomyces cerevisiae protein YGR261C or its homologs, e.g. the activity of a protein of the beta-adaptin superfamily is increased, preferably, an activity of clathrin assembly complex beta adaptin component protein, e.g. as indicated in Table I, columns 5 or 7, lines 318 and 331, is increased conferring an increase of the respective fine chemical, preferably of myo-inositol (124% to 698%) and/or raffinose (95% to 2956%) resp., between 95% and 2956% or more.
In one embodiment, the activity of the Saccharomyces cerevisiae protein YIL150C or its homologs, e.g. the activity of a chromatin binding protein, required for S-phase (DNA synthesis) initiation or completion, e.g. as indicated in Table I, columns 5 or 7, line 319 is increased conferring an increase of the respective fine chemical, preferably of myo-inositol between 400% and 480% or more.
In one embodiment, the activity of the Saccharomyces cerevisiae protein YJL072C or its homologs, e.g. the activity of a protein of the Saccharomyces cerevisiae membrane protein YJL072c superfamily is increased, preferably, an activity of subunit of the GINS complex required for chromosomal DNA replication protein, e.g. as indicated in Table I, columns 5 or 7, line 308 is increased conferring an increase of the respective fine chemical, preferably of glucose between 58% and 293% or more.
In one embodiment, the activity of the Saccharomyces cerevisiae protein YJL099W or its homologs, e.g. the activity of a protein involved in chitin biosynthesis and/or its regulation e.g. as indicated in Table I, columns 5 or 7, line 320 is increased conferring an increase of the respective fine chemical, preferably of myo-inositol between 26% and 472% or more.
In one embodiment, the activity of the Saccharomyces cerevisiae protein YOR044W or its homologs, e.g. the activity of a protein of the Saccharomyces cerevisiae membrane protein YOR044w superfamily is increased, e.g. as indicated in Table I, columns 5 or 7, line 321 is increased conferring an increase of the respective fine chemical, preferably of myo-inositol between 44% and 160% or more.
In one embodiment, the activity of the Saccharomyces cerevisiae protein YOR350C or its homologs, e.g. the activity of a protein of the Saccharomyces cerevisiae MNE1 protein superfamily is increased, e.g. as indicated in Table I, columns 5 or 7, line 322 is increased conferring an increase of the respective fine chemical, preferably of myo-inositol between 52% and 182% or more.
In case the activity of the Escherichia coli K12 protein b0050 or its homologs, as indicated in Table I, columns 5 or 7, line 604, e.g. protein with an activity as defined in [0022.0.25.25], is increased, preferably, in one embodiment the increase of the fine chemical, preferably of starch and/or cellulose between 38% and 89% or more is conferred.
In case the activity of the Escherichia coli K12 protein b0124 or its homologs, as indicated in Table I, columns 5 or 7, line 608, e.g. protein with an activity as defined in [0022.0.25.25], is increased, preferably, in one embodiment the increase of the fine chemical, preferably of fructose between 100% and 2926% or more is conferred.
In case the activity of the Escherichia coli K12 protein b0149 or its homologs, as indicated in Table I, columns 5 or 7, line 609, e.g. protein with an activity as defined in [0022.0.25.25], is increased, preferably, in one embodiment the increase of the fine chemical, preferably of fructose between 68% and 95% or more is conferred.
In case the activity of the Escherichia coli K12 protein b1318 or its homologs, as indicated in Table I, columns 5 or 7, line 610, e.g. protein with an activity as defined in [0022.0.25.25], is increased, preferably, in one embodiment the increase of the fine chemical, preferably of fructose between 103% and 199% or more is conferred.
In case the activity of the Escherichia coli K12 protein b1463 or its homologs, as indicated in Table I, columns 5 or 7, lines 611 and 617 and 622, e.g. protein with an activity as defined in [0022.0.25.25], is increased, preferably, in one embodiment the increase of the fine chemical, preferably of fructose between 92% and 453%, preferably of glucose between 57% and 618%, preferably of myo-inositol between 36% and 96%, preferably of fructose and glucose between 57% and 618%, preferably of fructose and myo-inositol between 36% and 453%, preferably of glucose and myo-inositol between 36% and 618%, preferably of fructose and glucose and myo-inositol between 36% and 618%, or more is conferred.
In case the activity of the Escherichia coli K12 protein b1539 or its homologs, as indicated in Table I, columns 5 or 7, line 605, e.g. protein with an activity as defined in [0022.0.25.25], is increased, preferably, in one embodiment the increase of the fine chemical, preferably of starch and/or cellulose between 39% and 92% or more is conferred.
In case the activity of the Escherichia coli K12 protein b1736 or its homologs, as indicated in Table I, columns 5 or 7, line 618, e.g. protein with an activity as defined in [0022.0.25.25], is increased, preferably, in one embodiment the increase of the fine chemical, preferably of glucose between 61% and 110% or more is conferred.
In case the activity of the Escherichia coli K12 protein b1961 or its homologs, as indicated in Table I, columns 5 or 7, line 623, e.g. protein with an activity as defined in [0022.0.25.25], is increased, preferably, in one embodiment the increase of the fine chemical, preferably of myo-inositol between 25% and 58% or more is conferred.
In case the activity of the Escherichia coli K12 protein b2491 or its homologs, as indicated in Table I, columns 5 or 7, lines 612 and 619, e.g. protein with an activity as defined in [0022.0.25.25], is increased, preferably, in one embodiment the increase of the fine chemical, preferably of fructose between 123% and 278%, preferably of glucose between 81% and 289%, in particular for increasing the amount of fructose and glucose between 81% and 289%, or more is conferred.
In case the activity of the Escherichia coli K12 protein b3260 or its homologs, as indicated in Table I, columns 5 or 7, line 613, e.g. protein with an activity as defined in [0022.0.25.25], is increased, preferably, in one embodiment the increase of the fine chemical, preferably of fructose between 87% and 472% or more is conferred.
In case the activity of the Escherichia coli K12 protein b3578 or its homologs, as indicated in Table I, columns 5 or 7, lines 614 and 620 and 626, e.g. protein with an activity as defined in [0022.0.25.25], is increased, preferably, in one embodiment the increase of the fine chemical, preferably of fructose between 89% and 106%, preferably of glucose between 65% and 110%, preferably of raffinose between 55% and 84%, preferably of fructose and glucose between 65% and 110%, preferably of glucose and raffinose between 55% and 110%, preferably of fructose and raffinose between, 55% and 106%, preferably of fructose and glucose and raffinose between 55% and 110%, or more is conferred.
In case the activity of the Escherichia coli K12 protein b3619 or its homologs, as indicated in Table I, columns 5 or 7, line 615 and 621, e.g. protein with an activity as defined in [0022.0.25.25], is increased, preferably, in one embodiment the increase of the fine chemical, preferably of fructose between 61 % and 111%, preferably of glucose between 60% and 119%, preferably of fructose and glucose between 60% and 119% or more is conferred.
In case the activity of the Escherichia coli K12 protein b3919 or its homologs, as indicated in Table I, columns 5 or 7, line 606, e.g. protein with an activity as defined in [0022.0.25.25], is increased, preferably, in one embodiment the increase of the fine chemical, preferably of starch and/or cellulose between 33% and 52% or more is conferred.
In case the activity of the Escherichia coli K12 protein b4074 or its homologs, as indicated in Table I, columns 5 or 7, line 624, e.g. protein with an activity as defined in [0022.0.25.25], is increased, preferably, in one embodiment the increase of the fine chemical, preferably of myo-inositolbetween 25% and 50% or more is conferred.
In case the activity of the Escherichia coli K12 protein b4122 or its homologs, as indicated in Table I, columns 5 or 7, line 616, e.g. protein with an activity as defined in [0022.0.25.25], is increased, preferably, in one embodiment the increase of the fine chemical, preferably of fructose between 53% and 502% or more is conferred.
In case the activity of the Escherichia coli K12 protein b4232 or its homologs, as indicated in Table I, columns 5 or 7, line 607, e.g. protein with an activity as defined in [0022.0.25.25], is increased, preferably, in one embodiment the increase of the fine chemical, preferably of starch and/or cellulose between 35% and 61 % or more is conferred.
In case the activity of the Saccharomyces cerevisiae protein YHR072W-A or its homologs, as indicated in Table I, columns 5 or 7, line 625, e.g. protein with an activity as defined in [0022.0.25.25], is increased, preferably, in one embodiment the increase of the fine chemical, preferably of myo-inositol between 27% and 53% or more is conferred.

**[0046.0.25.25]** In one embodiment the activity of the Escherichia coli K12 protein b0019 or its homologs, e.g. the activity of a protein of the Na+/H+-exchanging protein nhaA superfamily is increased, preferably, of a protein having a Na+/H+ antiporter activity, e.g. as indicated in Table I, columns 5 or 7, line 332, is increased conferring an increase of the respective fine chemical, preferably of sucrose and of further carbohydrate(s).
In one embodiment the activity of the Escherichia coli K12 protein b0138 or its homologs, e.g. having a fimbrial-like adhesin protein activity, e.g. as indicated in Table I, columns 5 or 7, line 309, is increased conferring an increase of the respective fine chemical, preferably of myo-inositol and of further carbohydrate(s).
In one embodiment the activity of the Escherichia coli K12 protein b0161 or its homologs, e.g. the activity of a protein of the Helicobacter serine proteinase superfamily is increased, preferably, of a protein having a periplasmic serine protease (heat shock protein) activity, e.g. as indicated in Table I, columns 5 or 7, lines 295, 303 and 323, is increased conferring an increase of the respective fine chemical, preferably of fructose, glucose and/or raffinose resp. and of further carbohydrate(s).
In one embodiment the activity of the Escherichia coli K12 protein b0252 or its homologs, e.g. having a b0252 protein activity and similarity with helicase and ligase proteins, e.g. as indicated in Table I, columns 5 or 7, line 290 is increased conferring an increase of the respective fine chemical, preferably of starch and/or cellulose and of further carbohydrate(s).
In one embodiment the activity of the Escherichia coli K12 protein b0290 or its homologs, e.g. the activity of a protein of the Escherichia coli yagW protein superfamily is increased, preferably, of a protein having a receptor protein activity, e.g. as indicated in Table I, columns 5 or 7, line 310, is increased conferring an increase of the respective fine chemical, preferably of myo-inositol and of further carbohydrate(s).
In one embodiment the activity of the Escherichia coli K12 protein b0695 or its homologs, e.g. the activity of a protein having homology with the sensor histidine kinase superfamily is increased, preferably, of a protein having a activity of sensory histidine kinase in two-component regulatory system, e.g. as indicated in Table I, columns 5 or 7, line 296, is increased conferring an increase of the respective fine chemical, preferably of fructose and of further carbohydrate(s).
In one embodiment the activity of the Escherichia coli K12 protein b0730 or its homologs, e.g. the activity of a protein of the transcription regulator GntR superfamily is increased, preferably, of a protein having a activity of a transcriptional regulator of succinylCoA synthetase operon and fatty acyl response regulator, e.g. as indicated in Table I, columns 5 or 7, line 324, is increased conferring an increase of the respective fine chemical, preferably of raffinose and of further carbohydrate(s).
In one embodiment the activity of the Escherichia coli K12 protein b1430 or its homologs, e.g. the activity of a protein of the hemagglutinin hag1 superfamily is increased, preferably, of a protein having a activity of putative S-adenosyl-L-methionine-dependent methyltransferase conferring tellurite resistance, e.g. as indicated in Table I, columns 5 or 7, line 291, is increased conferring an increase of the respective fine chemical, preferably of starch and/or cellulose and of further carbohydrate(s).
In one embodiment the activity of the Escherichia coli K12 protein b1693 or its homologs, e.g. the activity of a protein of the 3-dehydroquinate dehydratase superfamily is increased, preferably, of a protein having a 3-dehydroquinate dehydratase activity, e.g. as indicated in Table I, columns 5 or 7, line 292, is increased conferring an increase of the respective fine chemical, preferably of starch and/or cellulose and of further carbohydrate(s).
In one embodiment the activity of the Escherichia coli K12 protein b1701 or its homologs, e.g. the activity of a protein with homology to the 4-coumarate-CoA ligase and/or acetate-CoA ligase superfamily is increased, preferably, of a protein having a activity of a CoenzymeA-dependent ligase with firefly luciferase-like ATPase Domain, e.g. as indicated in Table I, columns 5 or 7, line 325, is increased conferring an increase of the respective fine chemical, preferably of raffinose and of further carbohydrate(s).
In one embodiment the activity of the Escherichia coli K12 protein b1708 or its homologs, e.g. the activity of a protein of the protein HI1314 superfamily is increased, preferably, of a protein having a lipoprotein activity, e.g. as indicated in Table I, columns 5 or 7, lines 297 and 304, is increased conferring an increase of the respective fine chemical, preferably of fructose and/or glucose and of further carbohydrate(s).
In one embodiment the activity of the Escherichia coli K12 protein b1886 or its homologs, e.g. the activity of a protein of the methyl-accepting chemotaxis protein superfamily is increased, preferably, of a protein having a methyl-accepting chemotaxis protein II and/or aspartate sensor receptor activity, e.g. as indicated in Table I, columns 5 or 7, line 326, is increased conferring an increase of the respective fine chemical, preferably of raffinose and of further carbohydrate(s).
In one embodiment the activity of the Escherichia coli K12 protein b1926 or its homologs, e.g. having a flagellar protein fliT activity, e.g. as indicated in Table I, columns 5 or 7, lines 298 and 305, is increased conferring an increase of the respective fine chemical, preferably of fructose and/or glucose and of further carbohydrate(s).
In one embodiment the activity of the Escherichia coli K12 protein b2023 or its homologs, e.g. the activity of a protein of the amidotransferase hisH superfamily with trpG homology is increased, preferably, of a protein having a activity of a glutamine amidotransferase subunit of imidazole glycerol phosphate synthase heterodimer, e.g. as indicated in Table I, columns 5 or 7, line 311, is increased conferring an increase of the respective fine chemical, preferably of myo-inositol and of further carbohydrate(s).
In one embodiment the activity of the Escherichia coli K12 protein b2597 or its homologs, e.g. the activity of a protein of the Pseudomonas putida hypothetical protein rpoX superfamily is increased, preferably, of a protein having a activity of a ribosome associated factor, e.g. as indicated in Table I, columns 5 or 7, line 299, is increased conferring an increase of the respective fine chemical, preferably of fructose and of further carbohydrate(s).
In one embodiment the activity of the Escherichia coli K12 protein b2599 or its homologs, e.g. the activity of a protein of the pheA bifunctional enzyme superfamily with prephenate dehydratase homology is increased, preferably, of a proteinhaving a activity of a bifunctional enzyme: chorismate mutase P (N-terminal) and prephenate dehydratase (C-terminal), e.g. as indicated in Table I, columns 5 or 7, line 306, is increased conferring an increase of the respective fine chemical, preferably of glucose and of further carbohydrate(s).
In one embodiment the activity of the Escherichia coli K12 protein b2664 or its homologs, e.g. the activity of a protein of the transcription regulator gabP superfamily is increased, preferably, of a protein having a transcriptional repressor with DNA-binding Winged helix domain (GntR familiy) activity, e.g. as indicated in Table I, columns 5 or 7, lines 300 and 327, is increased conferring an increase of the respective fine chemical, preferably of fructose and/or raffinose and of further carbohydrate(s).
In one embodiment the activity of the Escherichia coli K12 protein b2699 or its homologs, e.g. the activity of a protein of the recombination protein recA superfamily is increased, preferably, of a protein having a activity of a DNA strand exchange and recombination protein with protease and nuclease activity, e.g. as indicated in Table I, columns 5 or 7, lines 312 and 328, is increased conferring an increase of the respective fine chemical, preferably of myo-inositol and/or raffinose and of further carbohydrate(s).
In one embodiment the activity of the Escherichia coli K12 protein b3172 or its homologs, e.g. the activity of a protein of the argininosuccinate synthase superfamily is increased, preferably, of a protein having a argininosuccinate synthetase activity, e.g. as indicated in Table I, columns 5 or 7, line 313, is increased conferring an increase of the respective fine chemical, preferably of myo-inositol and of further carbohydrate(s).
In one embodiment the activity of the Escherichia coli K12 protein b3231 or its homologs, e.g. the activity of a protein of the Escherichia coli ribosomal protein L13 superfamily is increased, preferably, of a protein having a 50S ribosomal subunit protein L13 activity, e.g. as indicated in Table I, columns 5 or 7, line 293, is increased conferring an increase of the respective fine chemical, preferably of starch and/or cellulose and of further carbohydrate(s).
In one embodiment the activity of the Escherichia coli K12 protein b3430 or its homologs, e.g. the activity of a protein of the glucose-1-phosphate adenylyltransferase superfamily is increased, preferably, of a protein having a glucose-1-phosphate adenylyltransferase activity, e.g. as indicated in Table I, columns 5 or 7, line 314, is increased conferring an increase of the respective fine chemical, preferably of myo-inositol and of further carbohydrate(s).
In one embodiment the activity of the Escherichia coli K12 protein b3601 or its homologs, e.g. having a transcriptional repressor for mannitol utilization activity, e.g. as indicated in Table I, columns 5 or 7, line 329, is increased conferring an increase of the respective fine chemical, preferably of raffinose and of further carbohydrate(s).
In one embodiment the activity of the Escherichia coli K12 protein b4129 or its homologs, e.g. the activity of a protein of the lysine-tRNA ligase superfamily is increased, preferably, of a protein having a (inducible) lysine tRNA synthetase activity, e.g. as indicated in Table I, columns 5 or 7, line 315, is increased conferring an increase of the respective fine chemical, preferably of myo-inositol and of further carbohydrate(s).
In one embodiment the activity of the Escherichia coli K12 protein b4239 or its homologs, e.g. the activity of a protein of the alpha-glucosidase superfamily with alpha-amylase core homology is increased, preferably, of a protein having a trehalose-6-P hydrolase activity, e.g. as indicated in Table I, columns 5 or 7, lines 301, 307 and 333, is increased conferring an increase of the respective fine chemical, preferably of fructose, glucose and/or sucrose resp., and of further carbohydrate(s).
In one embodiment the activity of the Escherichia coli K12 protein b4327 or its homologs, e.g. the activity of a protein of the protein b2409 superfamily is increased, preferably, of a protein having a HTH-type transcriptional regulator with periplasmic binding protein domain (LysR family) activity, e.g. as indicated in Table I, columns 5 or 7, line 302, is increased conferring an increase of the respective fine chemical, preferably of fructose and of further carbohydrate(s).
In one embodiment, the activity of the Saccharomyces cerevisiae protein YBR184W or its homologs, e.g. the activity of a YBR184W protein superfamily-protein is increased, preferably, an activity of YBR184W protein, e.g. as indicated in Table I, columns 5 or 7, line 330 is increased conferring an increase of the respective fine chemical, preferably of raffinose and of further carbohydrate(s).
In one embodiment, the activity of the Saccharomyces cerevisiae protein YBR204C or its homologs, e.g. an activity of peroxisomal lipase protein, e.g. as indicated in Table I, columns 5 or 7, line 316, is increased conferring an increase of the respective fine chemical, preferably of myo-inositol and of further carbohydrate(s).
In one embodiment, the activity of the Saccharomyces cerevisiae protein YDR112W or its homologs, e.g. an activity of YDR112W protein, e.g. as indicated in Table I, columns 5 or 7, line 317, is increased conferring an increase of the respective fine chemical, preferably of myo-inositol and of further carbohydrate(s).
In one embodiment, the activity of the Saccharomyces cerevisiae protein YER174C or its homologs, e.g. the activity of a protein of the thioredoxin homology superfamily is increased, preferably, an activity of hydroperoxide and superoxide-radical responsive glutathione-dependent oxidoreductase protein, e.g. as indicated in Table I, columns 5 or 7, line 294 is increased conferring an increase of the respective fine chemical, preferably of starch and/or cellulose and of further carbohydrate(s).
In one embodiment, the activity of the Saccharomyces cerevisiae protein YGR261C or its homologs, e.g. the activity of a protein of the beta-adaptin superfamily is increased, preferably, an activity of clathrin assembly complex beta adaptin component protein, e.g. as indicated in Table I, columns 5 or 7, lines 318 and 331, is increased conferring an increase of the respective fine chemical, preferably of myo-inositol and/or raffinose resp., and of further carbohydrate(s).
In one embodiment, the activity of the Saccharomyces cerevisiae protein YIL150C or its homologs, e.g. the activity of a chromatin binding protein, required for S-phase (DNA synthesis) initiation or completion, e.g. as indicated in Table I, columns 5 or 7, line 319 is increased conferring an increase of the respective fine chemical, preferably of myo-inositol and of further carbohydrate(s).
In one embodiment, the activity of the Saccharomyces cerevisiae protein YJL072C or its homologs, e.g. the activity of a protein of the Saccharomyces cerevisiae membrane protein YJL072c superfamily is increased, preferably, an activity of subunit of the GINS complex required for chromosomal DNA replication protein, e.g. as indicated in Table I, columns 5 or 7, line 308 is increased conferring an increase of the respective fine chemical, preferably of glucose and of further carbohydrate(s).
In one embodiment, the activity of the Saccharomyces cerevisiae protein YJL099W or its homologs, e.g. the activity of a protein involved in chitin biosynthesis and/or its regulation e.g. as indicated in Table I, columns 5 or 7, line 320 is increased conferring an increase of the respective fine chemical, preferably of myo-inositol and of further carbohydrate(s).
In one embodiment, the activity of the Saccharomyces cerevisiae protein YOR044W or its homologs, e.g. the activity of a protein of the Saccharomyces cerevisiae membrane protein YOR044w superfamily is increased, e.g. as indicated in Table I, columns 5 or 7, line 321 is increased conferring an increase of the respective fine chemical, preferably of myo-inositol and of further carbohydrate(s).
In one embodiment, the activity of the Saccharomyces cerevisiae protein YOR350C or its homologs, e.g. the activity of a protein of the Saccharomyces cerevisiae MNE1 protein superfamily is increased, e.g. as indicated in Table I, columns 5 or 7, line 322 is increased conferring an increase of the respective fine chemical, preferably of myo-inositol and of further carbohydrate(s).
In case the activity of the Escherichia coli K12 protein b0050 or its homologs, as indicated in Table I, columns 5 or 7, line 604, e.g. protein with an activity as defined in [0022.0.25.25], is increased, preferably, in one embodiment the increase of the fine chemical, preferably of starch and/or cellulose and of one or more other carbohydrate(s) is conferred.
In case the activity of the Escherichia coli K12 protein b0124 or its homologs, as indicated in Table I, columns 5 or 7, line 608, e.g. protein with an activity as defined in [0022.0.25.25], is increased, preferably, in one embodiment the increase of the fine chemical, preferably of fructose and of one or more other carbohydrate(s) is conferred.
In case the activity of the Escherichia coli K12 protein b0149 or its homologs, as indicated in Table I, columns 5 or 7, line 609, e.g. protein with an activity as defined in [0022.0.25.25], is increased, preferably, in one embodiment the increase of the fine chemical, preferably of fructose and of one or more other carbohydrate(s) is conferred.
In case the activity of the Escherichia coli K12 protein b1318 or its homologs, as indicated in Table I, columns 5 or 7, line 610, e.g. protein with an activity as defined in [0022.0.25.25], is increased, preferably, in one embodiment the increase of the fine chemical, preferably of fructose and of one or more other carbohydrate(s) is conferred.
In case the activity of the Escherichia coli K12 protein b1463 or its homologs, as indicated in Table I, columns 5 or 7, lines 611 and 617 and 622, e.g. protein with an activity as defined in [0022.0.25.25], is increased, preferably, in one embodiment the increase of the fine chemical, preferably of fructose and/or glucose and/or myo-inositol and of one or more other carbohydrate(s) conferred.
In case the activity of the Escherichia coli K12 protein b1539 or its homologs, as indicated in Table I, columns 5 or 7, line 605, e.g. protein with an activity as defined in [0022.0.25.25], is increased, preferably, in one embodiment the increase of the fine chemical, preferably of starch and/or cellulose and of one or more other carbohydrate(s) is conferred.
In case the activity of the Escherichia coli K12 protein b1736 or its homologs, as indicated in Table I, columns 5 or 7, line 618, e.g. protein with an activity as defined in [0022.0.25.25], is increased, preferably, in one embodiment the increase of the fine chemical, preferably of glucose and of one or more other carbohydrate(s) conferred.
In case the activity of the Escherichia coli K12 protein b1961 or its homologs, as indicated in Table I, columns 5 or 7, line 623, e.g. protein with an activity as defined in [0022.0.25.25], is increased, preferably, in one embodiment the increase of the fine chemical, preferably of myo-inositol and of one or more other carbohydrate(s) is conferred.
In case the activity of the Escherichia coli K12 protein b2491 or its homologs, as indicated in Table I, columns 5 or 7, lines 612 and 619, e.g. protein with an activity as defined in [0022.0.25.25], is increased, preferably, in one embodiment the increase of the fine chemical, preferably of fructose and/orglucose and of one or more other carbohydrate(s) is conferred.
In case the activity of the Escherichia coli K12 protein b3260 or its homologs, as indicated in Table I, columns 5 or 7, line 613, e.g. protein with an activity as defined in [0022.0.25.25], is increased, preferably, in one embodiment the increase of the fine chemical, preferably of fructose and of one or more other carbohydrate(s) is conferred.
In case the activity of the Escherichia coli K12 protein b3578 or its homologs, as indicated in Table I, columns 5 or 7, lines 614 and 620 and 626, e.g. protein with an activity as defined in [0022.0.25.25], is increased, preferably, in one embodiment the increase of the fine chemical, preferably of fructose and/or of glucose and/or raffinose and of one or more other carbohydrate(s) is conferred.
In case the activity of the Escherichia coli K12 protein b3619 or its homologs, as indicated in Table I, columns 5 or 7, line 615 and 621, e.g. protein with an activity as defined in [0022.0.25.25], is increased, preferably, in one embodiment the increase of the fine chemical, preferably of fructose and/or of glucose and of one or more other carbohydrate(s) is conferred.
In case the activity of the Escherichia coli K12 protein b3919 or its homologs, as indicated in Table I, columns 5 or 7, line 606, e.g. protein with an activity as defined in [0022.0.25.25], is increased, preferably, in one embodiment the increase of the fine chemical, preferably of starch and/or cellulose and of one or more other carbohydrate(s) is conferred.
In case the activity of the Escherichia coli K12 protein b4074 or its homologs, as indicated in Table I, columns 5 or 7, line 624, e.g. protein with an activity as defined in [0022.0.25.25], is increased, preferably, in one embodiment the increase of the fine chemical, preferably of myo-inositol and of one or more other carbohydrate(s) is conferred.
In case the activity of the Escherichia coli K12 protein b4122 or its homologs, as indicated in Table I, columns 5 or 7, line 616, e.g. protein with an activity as defined in [0022.0.25.25], is increased, preferably, in one embodiment the increase of the fine chemical, preferably of fructose and of one or more other carbohydrate(s) is conferred.
In case the activity of the Escherichia coli K12 protein b4232 or its homologs, as indicated in Table I, columns 5 or 7, line 607, e.g. protein with an activity as defined in [0022.0.25.25], is increased, preferably, in one embodiment the increase of the fine chemical, preferably of starch and/or cellulose and of one or more other carbohydrate(s) is conferred.
In case the activity of the Saccharomyces cerevisiae protein YHR072W-A or its homologs, as indicated in Table I, columns 5 or 7, line 625, e.g. protein with an activity as defined in [0022.0.25.25], is increased, preferably, in one embodiment the increase of the fine chemical, preferably of myo-inositol and of one or more other carbohydrate(s) is conferred.

**[0047.0.0.25]** to **[0048.0.0.25]: see [0047.0.0.0]** to **[0048.0.0.0]**

**[0049.0.25.25]** A protein having an activity conferring an increase in the amount or level of the starch and/or cellulose preferably has the structure of the polypeptide described herein. In a particular embodiment, the polypeptides used in the process of the present invention or the polypeptide of the present invention comprises the sequence of a consensus sequence a as indicated in Table IV, column 7, lines 290 to 294 and/or 604 to 607 and/or the sequence of a polypeptide as indicated in Table II, columns 5 or 7, lines 290 to 294 and/or 604 to 607, or of a functional homologue thereof as described herein, or of a polypeptide encoded by the nucleic acid molecule characterized herein or the nucleic acid molecule according to the invention, for example by a nucleic acid molecule as indicated in Table I, columns 5 or 7, lines 290 to 294 and/or 604 to 607 or its herein described functional homologues and has the herein mentioned activity conferring an increase in the starch and/or cellulose level.
A protein having an activity conferring an increase in the amount or level of the fructose preferably has the structure of the polypeptide described herein. In a particular embodiment, the polypeptides used in the process of the present invention or the polypeptide of the present invention comprises the sequence of a consensus sequence a as indicated in Table IV, column 7, lines 295 to 302 and/or 608 to 616 and/or the sequence of a polypeptide as indicated in Table II, columns 5 or 7, lines 295 to 302 and/or 608 to 616, or of a functional homologue thereof as described herein, or of a polypeptide encoded by the nucleic acid molecule characterized herein or the nucleic acid molecule according to the invention, for example by a nucleic acid molecule as indicated in Table I, columns 5 or 7, lines 295 to 302 and/or 608 to 616 or its herein described functional homologues and has the herein mentioned activity conferring an increase in the fructose level.
A protein having an activity conferring an increase in the amount or level of the glucose preferably has the structure of the polypeptide described herein. In a particular embodiment, the polypeptides used in the process of the present invention or the polypeptide of the present invention comprises the sequence of a consensus sequence a as indicated in Table IV, column 7, lines 303 to 308 and/or 617 to 621 and/or the sequence of a polypeptide as indicated in Table II, columns 5 or 7, lines 303 to 308 and/or 617 to 621, or of a functional homologue thereof as described herein, or of a polypeptide encoded by the nucleic acid molecule characterized herein or the nucleic acid molecule according to the invention, for example by a nucleic acid molecule as indicated in Table I, columns 5 or 7, lines 303 to 308 and/or 617 to 621 or its herein described functional homologues and has the herein mentioned activity conferring an increase in the glucose level.
A protein having an activity conferring an increase in the amount or level of the myo-inositol preferably has the structure of the polypeptide described herein. In a particular embodiment, the polypeptides used in the process of the present invention or the polypeptide of the present invention comprises the sequence of a consensus sequence a as indicated in Table IV, column 7, lines 309 to 322 and/or 622 to 625 and/or the sequence of a polypeptide as indicated in Table II, columns 5 or 7, lines 309 to 322 and/or 622 to 625, or of a functional homologue thereof as described herein, or of a polypeptide encoded by the nucleic acid molecule characterized herein or the nucleic acid molecule according to the invention, for example by a nucleic acid molecule as indicated in Table I, columns 5 or 7, lines 309 to 322 and/or 622 to 625 or its herein described functional homologues and has the herein mentioned activity conferring an increase in the myo-inositol level.
A protein having an activity conferring an increase in the amount or level of the raffinose preferably has the structure of the polypeptide described herein. In a particular embodiment, the polypeptides used in the process of the present invention or the polypeptide of the present invention comprises the sequence of a consensus sequence a as indicated in Table IV, column 7, lines 323 to 331 and/or 626 and/or the sequence of a polypeptide as indicated in Table II, columns 5 or 7, lines 323 to 331 and/or 626, or of a functional homologue thereof as described herein, or of a polypeptide encoded by the nucleic acid molecule characterized herein or the nucleic acid molecule according to the invention, for example by a nucleic acid molecule as indicated in Table I, columns 5 or 7, lines 323 to 331 and/or 626 or its herein described functional homologues and has the herein mentioned activity conferring an increase in the raffinose level.
A protein having an activity conferring an increase in the amount or level of the sucrose preferably has the structure of the polypeptide described herein. In a particular embodiment, the polypeptides used in the process of the present invention or the polypeptide of the present invention comprises the sequence of a consensus sequence a as indicated in Table IV, column 7, lines 332 to 333 and/or the sequence of a polypeptide as indicated in Table II, columns 5 or 7, lines 332 to 333, or of a functional homologue thereof as described herein, or of a polypeptide encoded by the nucleic acid molecule characterized herein or the nucleic acid molecule according to the invention, for example by a nucleic acid molecule as indicated in Table I, columns 5 or 7, lines 332 to 333 or its herein described functional homologues and has the herein mentioned activity conferring an increase in the sucrose level.

**[0050.0.25.25]** For the purposes of the present invention, the term "the respective fine chemical" also encompass the corresponding salts, ester and ethers, preferably etherificated with other mono-, di- oligo- or polysaccharides, with alkyl, alkenyl or alkinyl alcohols.

**[0051.0.25.25]** Owing to the biological activity of the proteins which are used in the process according to the invention and which are encoded by nucleic acid molecules according to the invention, it is possible to produce compositions comprising the respective fine chemical, i.e. an increased amount of the free chemical free or bound, e.g compositions comprising carbohydrates, preferably polysaccharides, more preferably starch and/or cellulose and/or monosaccharides, more preferably fructose, glucose and/or myo-inositol and/or trisaccharides, more preferably raffinose and/or disaccharides, more preferably sucrose resp.,. Depending on the choice of the organism used for the process according to the present invention, for example a microorganism or a plant, compositions or mixtures of various respective fine chemicals can be produced.

**[0052.0.0.25]** see **[0052.0.0.0]**

**[0053.0.25.25]** In one embodiment, the process of the present invention comprises one or more of the following steps
a) stabilizing a protein conferring the increased expression of a protein encoded by the nucleic acid molecule of the invention or of the polypeptid of the invention, e.g. of a polypeptide having an activity of a protein as indicated in Table II, column 3, lines 290 to 294 and/or 604 to 607 for starch and/or cellulose; lines 295 to 302 and/or 608 to 616 for fructose; lines 303 to 308 and/or 617 to 621 for glucose; lines 309 to 322 and/or 622 to 625 for myo-inositol; lines 323 to 331 and/or 626 for raffinose; lines 332 to 333 for sucrose;resp., or its homologs, e.g. as indicated in Table II, columns 5 or 7, lines 290 to 294 and/or 604 to 607 for starch and/or cellulose; lines 295 to 302 and/or 608 to 616 for fructose; lines 303 to 308 and/or 617 to 621 for glucose; lines 309 to 322 and/or 622 to 625 for myo-inositol; lines 323 to 331 and/or 626 for raffinose; lines 332 to 333 for sucrose;, resp., activity having herein-mentioned the respective fine chemical-increasing activity;
b) stabilizing a mRNA conferring the increased expression of a protein encoded by the nucleic acid molecule of the invention, e.g. of a polypeptide having an activity of a protein as indicated in Table II, column 3, lines 290 to 294 and/or 604 to 607 for starch and/or cellulose; lines 295 to 302 and/or 608 to 616 for fructose; lines 303 to 308 and/or 617 to 621 for glucose; lines 309 to 322 and/or 622 to 625 for myo-inositol; lines 323 to 331 and/or 626 for raffinose; lines 332 to 333 for sucrose;, resp., or its homologs activity, e.g. as indicated in Table II, columns 5 or 7, lines 290 to 294 and/or 604 to 607 for starch and/or cellulose; lines 295 to 302 and/or 608 to 616 for fructose; lines 303 to 308 and/or 617 to 621 for glucose; lines 309 to 322 and/or 622 to 625 for myo-inositol; lines 323 to 331 and/or 626 for raffinose; lines 332 to 333 for sucrose;, resp.,, or of a mRNA encoding the polypeptide of the present invention having herein-mentioned the respective fine chemical-increasing activity;
c) increasing the specific activity of a protein conferring the increased expression of a protein encoded by the nucleic acid molecule of the invention or of the polypeptide of the present invention having herein-mentioned the respective fine chemical-increasing activity, e.g. of a polypeptide having an activity of a protein as indicated in Table II, column 3, lines 290 to 294 and/or 604 to 607 for starch and/or cellulose; lines 295 to 302 and/or 608 to 616 for fructose; lines 303 to 308 and/or 617 to 621 for glucose; lines 309 to 322 and/or 622 to 625 for myo-inositol; lines 323 to 331 and/or 626 for raffinose; lines 332 to 333 for sucrose;, resp., or its homologs activity, e.g. as indicated in Table II, columns 5 or 7, lines 290 to 294 and/or 604 to 607 for starch and/or cellulose; lines 295 to 302 and/or 608 to 616 for fructose; lines 303 to 308 and/or 617 to 621 for glucose; lines 309 to 322 and/or 622 to 625 for myo-inositol; lines 323 to 331 and/or 626 for raffinose; lines 332 to 333 for sucrose;, resp., or decreasing the inhibitiory regulation of the polypeptide of the invention;
d) generating or increasing the expression of an endogenous or artificial transcription factor mediating the expression of a protein conferring the increased expression of a protein encoded by the nucleic acid molecule of the invention or of the polypeptide of the invention having herein-mentioned the respective fine chemical-increasing activity, e.g. of a polypeptide having an activity of a protein as indicated in Table II, column 3, lines 290 to 294 and/or 604 to 607 for starch and/or cellulose; lines 295 to 302 and/or 608 to 616 for fructose; lines 303 to 308 and/or 617 to 621 for glucose; lines 309 to 322 and/or 622 to 625 for myo-inositol; lines 323 to 331 and/or 626 for raffinose; lines 332 to 333 for sucrose;, resp., or its homologs activity, e.g. as indicated in Table II, columns 5 or 7, lines 290 to 294 and/or 604 to 607 for starch and/or cellulose; lines 295 to 302 and/or 608 to 616 for fructose; lines 303 to 308 and/or 617 to 621 for glucose; lines 309 to 322 and/or 622 to 625 for myo-inositol; lines 323 to 331 and/or 626 for raffinose; lines 332 to 333 for sucrose, resp.;
e) stimulating activity of a protein conferring the increased expression of a protein encoded by the nucleic acid molecule of the present invention or a polypeptide of the present invention having herein-mentioned the respective fine chemical-increasing activity, e.g. of a polypeptide having an activity of a protein as indicated in Table II, column 3, lines 290 to 294 and/or 604 to 607 for starch and/or cellulose; lines 295 to 302 and/or 608 to 616 for fructose; lines 303 to 308 and/or 617 to 621 for glucose; lines 309 to 322 and/or 622 to 625 for myo-inositol; lines 323 to 331 and/or 626 for raffinose; lines 332 to 333 for sucrose, resp., or its homologs activity, e.g. as indicated in Table II, columns 5 or 7, lines 290 to 294 and/or 604 to 607 for starch and/or cellulose; lines 295 to 302 and/or 608 to 616 for fructose; lines 303 to 308 and/or 617 to 621 for glucose; lines 309 to 322 and/or 622 to 625 for myo-inositol; lines 323 to 331 and/or 626 for raffinose; lines 332 to 333 for sucrose, resp., by adding one or more exogenous inducing factors to the organismus or parts thereof;
f) expressing a transgenic gene encoding a protein conferring the increased expression of a polypeptide encoded by the nucleic acid molecule of the present invention or a polypeptide of the present invention, having herein-mentioned the respective fine chemical-increasing activity, e.g. of a polypeptide having an activity of a protein as indicated in Table II, column 3, lines 290 to 294 and/or 604 to 607 for starch and/or cellulose; lines 295 to 302 and/or 608 to 616 for fructose; lines 303 to 308 and/or 617 to 621 for glucose; lines 309 to 322 and/or 622 to 625 for myo-inositol; lines 323 to 331 and/or 626 for raffinose; lines 332 to 333 for sucrose, resp.,or its homologs activity, e.g. as indicated in Table II, columns 5 or 7, lines 290 to 294 and/or 604 to 607 for starch and/or cellulose; lines 295 to 302 and/or 608 to 616 for fructose; lines 303 to 308 and/or 617 to 621 for glucose; lines 309 to 322 and/or 622 to 625 for myo-inositol; lines 323 to 331 and/or 626 for raffinose; lines 332 to 333 for sucrose, resp., and/or
g) increasing the copy number of a gene conferring the increased expression of a nucleic acid molecule encoding a polypeptide encoded by the nucleic acid molecule of the invention or the polypeptide of the invention having herein-mentioned the respective fine chemical-increasing activity, e.g. of a polypeptide having an activity of a protein as indicated in Table II, column 3, lines 290 to 294 and/or 604 to 607 for starch and/or cellulose; lines 295 to 302 and/or 608 to 616 for fructose; lines 303 to 308 and/or 617 to 621 for glucose; lines 309 to 322 and/or 622 to 625 for myo-inositol; lines 323 to 331 and/or 626 for raffinose; lines 332 to 333 for sucrose, resp.,or its homologs, e.g. as indicated in Table II, columns 5 or 7, lines 290 to 294 and/or 604 to 607 for starch and/or cellulose; lines 295 to 302 and/or 608 to 616 for fructose; lines 303 to 308 and/or 617 to 621 for glucose; lines 309 to 322 and/or 622 to 625 for myo-inositol; lines 323 to 331 and/or 626 for raffinose; lines 332 to 333 for sucrose;, resp., activity.
h) Increasing the expression of the endogenous gene encoding the polypeptide of the invention, e.g. a polypeptide having an activity of a protein as indicated in Table II, column 3, lines 290 to 294 and/or 604 to 607 for starch and/or cellulose; lines 295 to 302 and/or 608 to 616 for fructose; lines 303 to 308 and/or 617 to 621 for glucose; lines 309 to 322 and/or 622 to 625 for myo-inositol; lines 323 to 331 and/or 626 for raffinose; lines 332 to 333 for sucrose, resp., or its homologs activity, e.g. as indicated in Table II, columns 5 or 7, lines 290 to 294 and/or 604 to 607 for starch and/or cellulose; lines 295 to 302 and/or 608 to 616 for fructose; lines 303 to 308 and/or 617 to 621 for glucose; lines 309 to 322 and/or 622 to 625 for myo-inositol; lines 323 to 331 and/or 626 for raffinose; lines 332 to 333 for sucrose;, resp., by adding positive expression or removing negative expression elements, e.g. homologous recombination can be used to either introduce positive regulatory elements like for plants the 35S enhancer into the promoter or to remove repressor elements form regulatory regions. Further gene conversion methods can be used to disrupt repressor elements or to enhance to acitivty of positive elements. Positive elements can be randomly introduced in plants by T-DNA or transposon mutagenesis and lines can be identified in which the positive elements have be integrated near to a gene of the invention, the expression of which is thereby enhanced;
i) Modulating growth conditions of an organism in such a manner, that the expression or activity of the gene encoding the protein of the invention or the protein itself is enhanced for example microorganisms or plants can be grown for example under a higher temperature regime leading to an enhanced expression of heat shock proteins, which can lead an enhanced fine chemical production and/or
j) selecting of organisms with expecially high activity of the proteins of the invention from natural or from mutagenized resources and breeding them into the target organisms, eg the elite crops.

**[0054.0.25.25]** Preferably, said mRNA is the nucleic acid molecule of the present invention and/or the protein conferring the increased expression of a protein encoded by the nucleic acid molecule of the present invention or the polypeptide having the herein mentioned activity is the polypeptide of the present invention, e.g. conferring the increase of the respective fine chemical after increasing the expression or activity of the encoded polypeptide or having the activity of a polypeptide having an activity of a protein as indicated in Table II, columns 5 or 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp., or its homologs activity, e.g. as indicated in Table II, columns 5 or 7 lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp..

**[0055.0.0.25] to [0064.0.0.25]: see [0055.0.0.0] to [0064.0.0.0]**

**[0065.0.0.25]: see [0065.0.0.0]**

**[0066.0.0.25] to [0067.0.0.25]: see [0066.0.0.0] to [0067.0.0.0]**

**[0068.0.25.25]** The mutation is introduced in such a way that the production of the carbohydrates, preferably polysaccharides, more preferably starch and/or cellulose and/or monosaccharides, more preferably fructose, glucose and/or myo-inositol and/or trisaccharides, more preferably raffinose and/or disaccharides, more preferably sucroseis not adversely affected.

**[0069.0.0.25] see [0069.0.0.0]**

**[0070.0.25.25]** Owing to the introduction of a gene or a plurality of genes conferring the expression of the nucleic acid molecule of the invention or the polypeptide of the invention, for example the nucleic acid construct mentioned below, or encoding a protein of the invention into an organism alone or in combination with other genes, it is possible not only to increase the biosynthetic flux towards the end product, but also to increase, modify or create de novo an advantageous, preferably novel metabolites composition in the organism, e.g. an advantageous composition carbohydrates comprising a higher content of (from a viewpoint of nutrional physiology limited) preferably polysaccharides, more preferably starch and/or cellulose and/or monosaccharides, more preferably fructose, glucose and/or myo-inositol and/or trisaccharides, more preferably raffinose and/or disaccharides, more preferably sucrose.

**[0071.0.25.25]**Preferably the composition further comprises higher amounts of metabolites positively affecting or lower amounts of metabolites negatively affecting the nutrition or health of animals or humans provided with said compositions or organisms of the invention or parts thereof. Likewise, the number or activity of further genes which are required for the import or export of nutrients or metabolites, including carbohydrates or its precursors, required for the cell's biosynthesis of carbohydratesmay be increased so that the concentration of necessary or relevant precursors, cofactors or intermediates within the cell(s) or within the corresponding storage compartments is increased. Owing to the increased or novel generated activity of the polypeptide of the invention or the polypeptide used in the method of the invention or owing to the increased number of nucleic acid sequences of the invention and/or to the modulation of further genes which are involved in the biosynthesis of the carbohydrates, e.g. by increasing the acitivty of enzymes synthesizing precursors or by destroying the activity of one or more genes which are involved in the breakdown of the carbohydrates, it is possible to increase the yield, production and/or production efficiency of carbohydratesin the host organism, such as the plants or the microorganims.

**[0072.0.25.25]** By influencing the metabolism thus, it is possible to produce, in the process according to the invention, further advantageous compounds. Examples of such compounds are, in addition to polysaccharides, more preferably starch and/or cellulose and/or monosaccharides, more preferably fructose, glucose and/or myo-inositol and/or trisaccharides, more preferably raffinose and/or disaccharides, more preferably sucrose, further carbohydrates, preferably saccharides.

**[0073.0.25.25]** Accordingly, in one embodiment, the process according to the invention relates to a process, which comprises:
a) providing a non-human organism, preferably a microorganism, a non-human animal, a plant or animal cell, a plant or animal tissue or a plant;
b) increasing an activity of a polypeptide of the invention or a homolog thereof, e.g. as indicated in Table II, columns 5 or 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp., or of a polypeptide being encoded by the nucleic acid molecule of the present invention and described below, e.g. conferring an increase of the respective fine chemical in an organism, preferably in a microorganism, a non-human animal, a plant or animal cell, a plant or animal tissue or a plant,
c) growing an organism, preferably a microorganism, a non-human animal, a plant or animal cell, a plant or animal tissue or a plant under conditions which permit the production of the respective fine chemical in the organism, preferably the microorganism, the plant cell, the plant tissue or the plant; and
d) if desired, revovering, optionally isolating, the free and/or bound the respective fine chemical and, optionally further free and/or bound carbohydrate(s) synthetized by the organism, the microorganism, the non-human animal, the plant or animal cell, the plant or animal tissue or the plant.

**[0074.0.25.25]** The organism, in particular the microorganism, non-human animal, the plant or animal cell, the plant or animal tissue or the plant is advantageously grown in such a way that it is not only possible to recover, if desired isolate the free or bound the respective fine chemical or the free and bound the respective fine chemical but as option it is also possible to produce, recover and, if desired isolate, other free or/and bound carbohydrate(s).

**[0075.0.0.25]** to **[0077.0.0.25]: see [0075.0.0.0]** to **[0077.0.0.0]**

**[0078.0.25.25]** The organism such as microorganisms or plants or the recovered, and if desired isolated, respective fine chemical can then be processed further directly into foodstuffs or animal feeds or for other applications in nutrition or medicine or cosmetics, for example according to the disclosures made in US 6,669,962: Starch microcapsules for delivery of active agents; US 20050042737 : Starch process; US 20050054071: Enzymes for starch processing; US 20050091716 : Novel plants and processes for obtaining them; U.S. Pat. No. 5,096,594 and 5,482,631 discloses a method of purifying cyclitols; U.S. Pat. No. 4,997,489 discloses soaking almond hulls in water to obtain a syrup containing fructose, glucose, inositol, and sorbitol; U.S. Pat. No. 5,296,364 discloses a microbial method for producing inositol; US. 4,734,402; US. 4,788,065; US 6,465,037 and US 6,355,295 : relates to soy food ingredient based on carbohydrates, US 6,653,451; US 20040128713: pertains to soybean plants having in their seeds significantly lower contents of raffinose, stachyose and phytic acid and significantly higher contents of sucrose and inorganic phosphate; US 20050008713 discloses compositions of plant carbohydrates for dietary supplements and nutritional support; which are expressly incorporated herein by reference. The fermentation broth, fermentation products, plants or plant products can be treated and processed as described in above mentioned applications or by other methods known to the person skilled in the art and described herein below.
In the method for producing carbohydrates, preferably polysaccharides, more preferably starch and/or cellulose and/or monosaccharides, more preferably fructose, glucose and/or myo-inositol and/or trisaccharides, more preferably raffinose and/or disaccharides, more preferably sucrose according to the invention, the cultivation step of the genetically modified organisms, also referred to as transgenic organisms hereinbelow, is preferably followed by harvesting said organisms and isolating the respective carbohydrate(s) from said organisms.
The organisms are harvested in a manner known per se and appropriate for the particular organism. Microorganisms such as bacteria, mosses, yeasts and fungi or plant cells which are cultured in liquid media by fermentation may be removed, for example, by centrifugation, decanting or filtration. Plants are grown on solid media in a manner known per se and harvested accordingly.
Carbohydrates, preferably polysaccharides, more preferably starch and/or cellulose and/or monosaccharides, more preferably fructose, glucose and/or myo-inositol and/or trisaccharides, more preferably raffinose and/or disaccharides, more preferably sucrose are isolated from the harvested biomass in a manner known per se, for example by extraction and, where appropriate, further chemical or physical purification processes such as, for example, chemical and/or enzymatical degradation, precipitation methods, crystallography, thermal separation methods such as rectification methods or physical separation methods such as, for example, chromatography.
Products of these different work-up procedures are carbohydrates, preferably polysaccharides, more preferably starch and/or cellulose and/or monosaccharides, more preferably fructose, glucose and/or myo-inositol and/or trisaccharides, more preferably raffinose and/or disaccharides, more preferably sucrose comprising compositions, e.g. compostions comprising carbohydrates, preferably polysaccharides, more preferably starch and/or cellulose and/or monosaccharides, more preferably fructose, glucose and/or myo-inositol and/or trisaccharides, more preferably raffinose and/or disaccharides, more preferably sucrose which still comprise fermentation broth, plant particles and/or cell components in different amounts, advantageously in the range of from 0 to 99% by weight, preferably below 80% by weight, especially preferably between 50%, 40%, 30%, 20%, 20%, 10%, 5%, 3%, 2%, 1%, 05%, 0,1%, 0,01 % and 0% by weight resp..
In one embodiment, preferred plants include, but are not limited to: sugar beet, sugar cane, soybeans and/or potato ( Solanum tuberosum).

**[0079.0.0.25]** to **[0084.0.0.25]: see [0079.0.0.0]** to **[0084.0.0.0]**

**[0085.0.25.25]** With regard to the nucleic acid sequence as depicted a nucleic acid construct which contains a nucleic acid sequence mentioned herein or an organism (= transgenic organism) which is transformed with said nucleic acid sequence or said nucleic acid construct, "transgene" means all those constructs which have been brought about by genetic manipulation methods,preferably in which either
a) a nucleic acid sequence as indicated in Table I, columns 5 or 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp., or a derivative thereof, or
b) a genetic regulatory element, for example a promoter, which is functionally linked to the nucleic acid sequence as indicated in Table I, columns 5 or 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp., or a derivative thereof, or
c) (a) and (b)
is/are not present in its/their natural genetic environment or has/have been modified by means of genetic manipulation methods, it being possible for the modification to be, by way of example, a substitution, addition, deletion, inversion or insertion of one or more nucleotide. "Natural genetic environment" means the natural chromosomal locus in the organism of origin or the presence in a genomic library. In the case of a genomic library, the natural, genetic environment of the nucleic acid sequence is preferably at least partially still preserved. The environment flanks the nucleic acid sequence at least on one side and has a sequence length of at least 50 bp, preferably at least 500 bp, particularly preferably at least 1000 bp, very particularly preferably at least 5000 bp.

**[0086.0.0.25]** to **[0087.0.0.25]: see [0086.0.0.0]** to **[0087.0.0.0]**

**[0088.0.25.25]** In an advantageous embodiment of the invention, the organism takes the form of a plant whose content the respective fine chemical is modified advantageously owing to the nucleic acid molecule of the present invention expressed. This is important for humans and animals since, for example, the nutritional value of plants for nutrition is dependent on the abovementioned carbohydrates and the general amount ofsaccharides as energy source in feed.

**[0088.1.0.25]** to **[0095.0.0.25]: see [0088.1.0.0]** to **[0095.0.0.0]**

**[0096.0..25.25]** In another preferred embodiment of the invention a combination of the increased expression of the nucleic acid sequence or the protein of the invention together with the transformation of a protein or polypeptide or a compound, which functions as a sink for the desired fine chemical, for example carbohydrates, preferably polysaccharides, more preferably starch and/or cellulose and/or monosaccharides, more preferably fructose, glucose and/or myo-inositol and/or trisaccharides, more preferably raffinose and/or disaccharides, more preferably sucrose in the organism, is useful to increase the production of the respective fine chemical.

**[0097.0.0.25] see [0097.0.0.0]**

**[0098.0.25.25]** In a preferred embodiment, the respective fine chemical (carbohydrates, preferably polysaccharides, more preferably starch and/or cellulose and/or monosaccharides, more preferably fructose, glucose and/or myo-inositol and/or trisaccharides, more preferably raffinose and/or disaccharides, more preferably sucrose) is produced in accordance with the invention and, if desired, is isolated. The production of carbohydrates, or mixtures thereof or mixtures with other compounds by the process according to the invention is advantageous.

**[0099.0.25.25]** In the case of the fermentation of microorganisms, the abovementioned carbohydrates, preferably polysaccharides, more preferably starch and/or cellulose and/or monosaccharides, more preferably fructose, glucose and/or myo-inositol and/or trisaccharides, more preferably raffinose and/or disaccharides, more preferably sucrose may accumulate in the medium and/or the cells. If microorganisms are used in the process according to the invention, the fermentation broth can be processed after the cultivation. Depending on the requirement, all or some of the biomass can be removed from the fermentation broth by separation methods such as, for example, centrifugation, filtration, decanting or a combination of these methods, or else the biomass can be left in the fermentation broth. The fermentation broth can subsequently be reduced, or concentrated, with the aid of known methods such as, for example, rotary evaporator, thin-layer evaporator, falling film evaporator, by reverse osmosis or by nanofiltration. Afterwards advantageously further compounds for formulation can be added such as corn starch or silicates. This concentrated fermentation broth advantageously together with compounds for the formulation can subsequently be processed by lyophilization, spray drying, and spray granulation or by other methods. Preferably the respective fine chemical or the carbohydrates, preferably polysaccharides, more preferably starch and/or cellulose and/or monosaccharides, more preferably fructose, glucose and/or myo-inositol and/or trisaccharides, more preferably raffinose and/or disaccharides, more preferably sucrose compositions are isolated from the organisms, such as the microorganisms or plants or the culture medium in or on which the organisms have been grown, or from the organism and the culture medium, in the known manner, for example via extraction, distillation, crystallization, chromatography or a combination of these methods. These purification methods can be used alone or in combination with the aforementioned methods such as the separation and/or concentration methods.

**[0100.0.25.25]** Transgenic plants which comprise the carbohydrates, preferably polysaccharides, more preferably starch and/or cellulose and/or monosaccharides, more preferably fructose, glucose and/or myo-inositol and/or trisaccharides, more preferably raffinose and/or disaccharides, more preferably sucrose synthesized in the process according to the invention can advantageously be marketed directly without there being any need for the oils, lipids or fatty acids synthesized to be isolated. Plants for the process according to the invention are listed as meaning intact plants and all plant parts, plant organs or plant parts such as leaf, stem, seeds, root, tubers, anthers, fibers, fruits, root hairs, stalks, embryos, calli, cotelydons, petioles, harvested material, plant tissue, reproductive tissue and cell cultures which are derived from the actual transgenic plant and/or can be used for bringing about the transgenic plant. In this context, the seed comprises all parts of the seed such as the seed coats, epidermal cells, seed cells, endosperm or embryonic tissue.
However, the respective fine chemical produced in the process according to the invention can also be isolated from the organisms, advantageously plants, in the form of their extracts, e.g. water containing extract, or as fibre in case of starch and/or cellulose, as degradation products (chemical ar enzymatical degradation) or crystallisation product or as their ethers and/or esters and/or free carbohydrates, preferably polysaccharides, more preferably starch and/or cellulose and/or monosaccharides, more preferably fructose, glucose and/or myo-inositol and/or trisaccharides, more preferably raffinose and/or disaccharides, more preferably sucrose. The respective fine chemical produced by this process can be obtained by harvesting the organisms, either from the crop in which they grow, or from the field. This can be done via pressing or extraction of the plant parts, e.g. in the plant stem, seeds, root, tubers, anthers, fibers, fruits. To increase the efficiency of extraction it is beneficial to clean, to temper and if necessary to hull and to flake the plant material expecially the stem, seeds, root, tubers, anthers, fibers, fruits, extracts and/carbohydrates, preferably polysaccharides, more preferably starch and/or cellulose and/or monosaccharides, more preferably fructose, glucose and/or myo-inositol and/or trisaccharides, more preferably raffinose and/or disaccharides, more preferably sucrose can be obtained by what is known as cold beating or cold pressing without applying heat. To allow for greater ease of disruption of the plant parts, specifically the stem, seeds, root, tubers, anthers, fibers, fruits, they can previously be comminuted, steamed or roasted. Plant parts, which have been pretreated in this manner can subsequently be pressed or extracted with solvents or water. The solvent is subsequently removed. In the case of microorganisms, the latter are, after harvesting, for example extracted directly without further processing steps or else, after disruption, extracted via various methods with which the skilled worker is familiar. Thereafter, the resulting products can be processed further, i.e. degradiated, crystallized and/or refined.

**[0101.0.25.25] see [0101.0.0.0]**

**[0102.0..25.25]** Carbohydrates, preferably polysaccharides, more preferably starch and/or cellulose and/or monosaccharides, more preferably fructose, glucose and/or myo-inositol and/or trisaccharides, more preferably raffinose and/or disaccharides, more preferably sucrose can for example be analyzed advantageously via HPLC or GC separation methods and detected by MS oder MSMS methods. The unambiguous detection for the presence of carbohydrates, preferably polysaccharides, more preferably starch and/or cellulose and/or monosaccharides, more preferably fructose, glucose and/or myo-inositol and/or trisaccharides, more preferably raffinose and/or disaccharides, more preferably sucrosecontaining products can be obtained by analyzing recombinant organisms using analytical standard methods: GC, GC-MS, LC, LC-MSMS or TLC, as described on several occasions.. The carbohydrates can be analized further in plant extracts by anion-exchange chromatography with pulsed amperometric detection (Cataldi et al., Anal Chem.;72(16):3902-7, 2000), by enzymatic "BioAnalysis" usind test kits from R-Biopharm and Roche or from Megazyme, Ireland.

**[0103.0.25.25]** In a preferred embodiment, the present invention relates to a process for the production of the respective fine chemical comprising or generating in an organism or a part thereof the expression of at least one nucleic acid molecule comprising a nucleic acid molecule selected from the group consisting of:
a) nucleic acid molecule encoding, preferably at least the mature form, of the polypeptide having a sequence as indicated in Table II, columns 5 or 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp., or a fragment thereof, which confers an increase in the amount of the respective fine chemical in an organism or a part thereof;
b) nucleic acid molecule comprising, preferably at least the mature form, of a nucleic acid molecule having a sequence as indicated in Table I, columns 5 or 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp.,
c) nucleic acid molecule whose sequence can be deduced from a polypeptide sequence encoded by a nucleic acid molecule of (a) or (b) as result of the degeneracy of the genetic code and conferring an increase in the amount of the respective fine chemical in an organism or a part thereof;
d) nucleic acid molecule encoding a polypeptide which has at least 50% identity with the amino acid sequence of the polypeptide encoded by the nucleic acid molecule of (a) to (c) and conferring an increase in the amount of the respective fine chemical in an organism or a part thereof;
e) nucleic acid molecule which hybidizes with a nucleic acid molecule of (a) to (c) under under stringent hybridisation conditions and conferring an increase in the amount of the respective fine chemical in an organism or a part thereof;
f) nucleic acid molecule encoding a polypeptide, the polypeptide being derived by substituting, deleting and/or adding one or more amino acids of the amino acid sequence of the polypeptide encoded by the nucleic acid molecules (a) to (d), preferably to (a) to (c) and conferring an increase in the amount of the respective fine chemical in an organism or a part thereof;
g) nucleic acid molecule encoding a fragment or an epitope of a polypeptide which is encoded by one of the nucleic acid molecules of (a) to (e), preferably to (a) to (c) and and conferring an increase in the amount of the respective fine chemical in an organism or a part thereof;
h) nucleic acid molecule comprising a nucleic acid molecule which is obtained by amplifying nucleic acid molecules from a cDNA library or a genomic library using the primers pairs having a sequence as indicated in Table III, column 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp., and conferring an increase in the amount of the respective fine chemical in an organism or a part thereof;
i) nucleic acid molecule encoding a polypeptide which is isolated, e.g. from an expression library, with the aid of monoclonal antibodies against a polypeptide encoded by one of the nucleic acid molecules of (a) to (h), preferably to (a) to (c), and and conferring an increase in the amount of the respective fine chemical in an organism or a part thereof;
j) nucleic acid molecule which encodes a polypeptide comprising the consensus sequence having a sequences as indicated in Table IV, column 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp., and conferring an increase in the amount of the respective fine chemical in an organism or a part thereof ;
k) nucleic acid molecule comprising one or more of the nucleic acid molecule encoding the amino acid sequence of a polypeptide encoding a domain of the polypeptide indicated in Table II, columns 5 or 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp., and conferring an increase in the amount of the respective fine chemical in an organism or a part thereof; and
l) nucleic acid molecule which is obtainable by screening a suitable library under stringent conditions with a probe comprising one of the sequences of the nucleic acid molecule of (a) to (k), preferably to (a) to (c), or with a fragment of at least 15 nt, preferably 20 nt, 30 nt, 50 nt, 100 nt, 200 nt or 500 nt of the nucleic acid molecule characterized in (a) to (k), preferably to (a) to (c), and conferring an increase in the amount of the respective fine chemical in an organism or a part thereof;
or which comprises a sequence which is complementary thereto.

**[00103.1.0.4.]** In one embodiment, the nucleic acid molecule used in the process of the invention distinguishes over the sequence indicated in Table IA, columns 5 or 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp., by one or more nucleotides. In one embodiment, the nucleic acid molecule used in the process of the invention does not consist of the sequence shown in indicated in Table I A, columns 5 or 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp.. In one embodiment, the nucleic acid molecule used in the process of the invention is less than 100%, 99,999%, 99,99%, 99,9% or 99% identical to a sequence indicated in Table I A, columns 5 or 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp.. In another embodiment, the nucleic acid molecule does not encode a polypeptide of a sequence indicated in Table II A, columns 5 or 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp..

**[00103.2.0.4.]** In one embodiment, the nucleic acid molecule used in the process of the invention distinguishes over the sequence indicated in Table I B, columns 5 or 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp., by one or more nucleotides. In one embodiment, the nucleic acid molecule used in the process of the invention does not consist of the sequence shown in indicated in Table I B, columns 5 or 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp.: In one embodiment, the nucleic acid molecule used in the process of the invention is less than 100%, 99,999%, 99,99%, 99,9% or 99% identical to a sequence indicated in Table I B, columns 5 or 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp.. In another embodiment, the nucleic acid molecule does not encode a polypeptide of a sequence indicated in Table II B, columns 5 or 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp..

**[0104.0.25.25]** In one embodiment, the nucleic acid molecule used in the process distinguishes over the sequence indicated in Table I, columns 5 or 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp., by one or more nucleotides. In one embodiment, the nucleic acid molecule used in the process of the invention does not consist of the sequence indicated in Table I, columns 5 or 7 lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp.. In one embodiment, the nucleic acid molecule of the present invention is less than 100%, 99,999%, 99,99%, 99,9% or 99% identical to the sequence indicated in Table I, columns 5 or 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp.. In another embodiment, the nucleic acid molecule does not encode a polypeptide of a sequence indicated in Table II, columns 5 or 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp..

**[0105.0.0.25]** to **[0107.0.0.25]: see [0105.0.0.0]** to **[0107.0.0.0]**

**[0108.0.25.25]** Nucleic acid molecules with the sequence as indicated in Table I, columns 5 or 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp., nucleic acid molecules which are derived from an amino acid sequences as indicated in Table II, columns 5 or 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp., or from polypeptides comprising the consensus sequence as indicated in Table IV, column 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp. or their derivatives or homologues encoding polypeptides with the enzymatic or biological activity of an activity of a polypeptide as indicated in Table II, column 3, 5 or 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp., or conferring an increase of the respective fine chemical, meaning carbohydrates, preferably polysaccharides, more preferably starch and/or cellulose and/or monosaccharides, more preferably fructose, glucose and/or myo-inositol and/or trisaccharides, more preferably raffinose and/or disaccharides, more preferably sucrose after increasing its expression or activity are advantageously increased in the process according to the invention.

**[0109.0.0.25] see [0109.0.0.0]**

**[0110.0.0.9]** Nucleic acid molecules, which are advantageous for the process according to the invention and which encode polypeptides with an activity of a polypeptide of the invention or the polypeptide used in the method of the invention or used in the process of the invention , e.g. of a protein as indicated in Table II, column 5, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp., or being encoded by a nucleic acid molecule indicated in Table I, column 5, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp., or of its homologs, e.g. as indicated in Table II, column 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp., can be determined from generally accessible databases.

**[0111.0.0.25] see [0111.0.0.0]**

**[0112.0.25.25]** The nucleic acid molecules used in the process according to the invention take the form of isolated nucleic acid sequences, which encode polypeptides with an activity of a polypeptide as indicated in Table II, column 3, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp., or having the sequence of a polypeptide as indicated in Table II, columns 5 and 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp., and conferring an increase in the level of carbohydrates, preferably polysaccharides, more preferably starch and/or cellulose and/or monosaccharides, more preferably fructose, glucose and/or myo-inositol and/or trisaccharides, more preferably raffinose and/or disaccharides, more preferably sucrose.

**[0113.0.0.25] to [0120.0.0.25]: see [0113.0.0.0] to [0120.0.0.0]**

**[0121.0.25.25]** However, it is also possible to use artificial sequences, which differ in one or more bases from the nucleic acid sequences found in organisms, or in one or more amino acid molecules from polypeptide sequences found in organisms, in particular from the polypeptide sequences indicated in Table II, columns 5 or 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp., or the functional homologues thereof as described herein, preferably conferring above-mentioned activity, i.e. conferring an increase in the level of the fine chemical of the invention after increasing the activity of the polypeptide sequences indicated in Table II, columns 5 or 7, lines 290 to 294 and/or 604 to 607 for starch and/or cellulose; lines 295 to 302 and/or 608 to 616 for fructose; lines 303 to 308 and/or 617 to 621 for glucose; lines 309 to 322 and/or 622 to 625 for myo-inositol; lines 323 to 331 and/or 626 for raffinose; lines 332 to 333 for sucrose resp..

**[0122.0.0.25] to [0127.0.0.259]: see [0122.0.0.0] to [0127.0.0.0]**

**[0128.0.9.9]** Synthetic oligonucleotide primers for the amplification, e.g. as the pairs indicated in Table III, columns 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp., by means of polymerase chain reaction can be generated on the basis of a sequence shown herein, for example the sequence as indicated in Table I, columns 5 or 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp., or the sequences derived from a sequence as indicated in Table II, columns 5 or 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp..

**[0129.0.25.25]** Moreover, it is possible to identify conserved regions from various organisms by carrying out protein sequence alignments with the polypeptide used in the process of the invention, in particular with sequences of the polypeptide of the invention, from which conserved regions, and in turn, degenerate primers can be derived.. Conserved regions are those, which show a very little variation in the amino acid in one particular position of several homologs from different origin. The consenus sequences indicated in Table IV, column 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp., are derived from said aligments.

**[0130.0.25.25]** Degenerated primers can then be utilized by PCR for the amplification of fragments of novel proteins having above-mentioned activity, e.g. conferring the increase of carbohydrates, preferably polysaccharides, more preferably starch and/or cellulose and/or monosaccharides, more preferably fructose, glucose and/or myo-inositol and/or trisaccharides, more preferably raffinose and/or disaccharides, more preferably sucrose after increasing the expression or activity of the protein comprising said fragment.

**[0131.0.0.25] to [0138.0.0.25]: see [0131.0.0.0] to [0138.0.0.0]**

**[0139.0.25.25]** Polypeptides having above-mentioned activity, i.e. conferring the increase of the respective fine chemical level, derived from other organisms, can be encoded by other DNA sequences which hybridize to a sequences indicated in Table I, columns 5 or 7, preferably table I B, lines 290 to 294 and/or 604 to 607 for starch and/or cellulose; lines 295 to 302 and/or 608 to 616 for fructose; lines 303 to 308 and/or 617 to 621 for glucose; lines 309 to 322 and/or 622 to 625 for myo-inositol; lines 323 to 331 and/or 626 for raffinose; lines 332 to 333 for sucrose resp., under relaxed hybridization conditions and which code on expression for peptides having the respective fine chemical, in particular, of starch and/or cellulose and/ fructose and/or glucose and/or myo-inositol and/or raffinose and/or sucrose, resp., increasing activity.

**[0140.0.0.25] to [0146.0.0.25]: see [0140.0.0.0] to [0146.0.0.0]**

**[0147.0.25.25]:** Further, the nucleic acid molecule of the invention comprises a nucleic acid molecule, which is a complement of one of the nucleotide sequences of above mentioned nucleic acid molecules or a portion thereof. A nucleic acid molecule which is complementary to one of the nucleotide sequences indicated in Table I, columns 5 or 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp., is one which is sufficiently complementary to one of said nucleotide sequences such that it can hybridize to one of said nucleotide sequences, thereby forming a stable duplex. Preferably, the hybridisation is performed under stringent hybrization conditions. However, a complement of one of the herein disclosed sequences is preferably a sequence complement thereto according to the base pairing of nucleic acid molecules well known to the skilled person. For example, the bases A and G undergo base pairing with the bases T and U or C, resp. and visa versa. Modifications of the bases can influence the base-pairing partner.

**[0148.0.25.25]** The nucleic acid molecule of the invention comprises a nucleotide sequence which is at least about 30%, 35%, 40% or 45%, preferably at least about 50%, 55%, 60% or 65%, more preferably at least about 70%, 80%, or 90%, and even more preferably at least about 95%, 97%, 98%, 99% or more homologous to a nucleotide sequence indicated in Table I, preferably table I B, columns 5 or 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp., or a portion thereof and preferably has above mentioned activity, in particular having a carbohydrates, preferably polysaccharides, more preferably starch and/or cellulose and/or monosaccharides, more preferably fructose, glucose and/or myo-inositol and/or trisaccharides, more preferably raffinose and/or disaccharides, more preferably sucrose increasing activity after increasing the acitivity or an activity of a product of a gene encoding said sequences or their homologs.

**[0149.0.25.25]** The nucleic acid molecule of the invention comprises a nucleotide sequence which hybridizes, preferably hybridizes under stringent conditions as defined herein, to one of the nucleotide sequences indicated in Table I, preferably table I B, columns 5 or 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp., or a portion thereof and encodes a protein having above-mentioned activity, e.g. conferring increase of carbohydrates, preferably polysaccharides, more preferably starch and/or cellulose and/or monosaccharides, more preferably fructose, glucose and/or myo-inositol and/or trisaccharides, more preferably raffinose and/or disaccharides, more preferably sucrose resp., and optionally, the activity of a protein indicated in Table II, column 5, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp..

**[00149.1.25.25]** Optionally, the nucleotide sequence, which hybridises to one of the nucleotide sequences indicated in Table I, columns 5 or 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp., preferably of Table I B, columns 5 or 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp. has further one or more of the activities annotated or known for the a protein as indicated in Table II, column 3 lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp..

**[0150.0.25.25]** Moreover, the nucleic acid molecule of the invention can comprise only a portion of the coding region of one of the sequences indicated in Table I, preferably Table I B, columns 5 or 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp., for example a fragment which can be used as a probe or primer or a fragment encoding a biologically active portion of the polypeptide of the present invention or of a polypeptide used in the process of the present invention, i.e. having above-mentioned activity, e.g.conferring an increase of carbohydrates, preferably polysaccharides, more preferably starch and/or cellulose and/or monosaccharides, more preferably fructose, glucose and/or myo-inositol and/or trisaccharides, more preferably raffinose and/or disaccharides, more preferably sucrose resp., if its activity is increased. The nucleotide sequences determined from the cloning of the present protein-according-to-the-invention-encoding gene allows for the generation of probes and primers designed for use in identifying and/or cloning its homologues in other cell types and organisms. The probe/primer typically comprises substantially purified oligonucleotide. The oligonucleotide typically comprises a region of nucleotide sequence that hybridizes under stringent conditions to at least about 12, 15 preferably about 20 or 25, more preferably about 40, 50 or 75 consecutive nucleotides of a sense strand of one of the sequences set forth, e.g., as indicated in Table I, columns 5 or 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp., an anti-sense sequence of one of the sequences, e.g., as indicated in Table I, columns 5 or 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp., or naturally occurring mutants thereof. Primers based on a nucleotide of invention can be used in PCR reactions to clone homologues of the polypeptide of the invention or of the polypeptide used in the process of the invention, e.g. as the primers described in the examples of the present invention, e.g. as shown in the examples. A PCR with the primer pairs indicated in Table III, column 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp., will result in a fragment of a polynucleotide sequence as indicated in Table I, columns 5 or 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp., or its gene product.

**[0151.0.0.25]:** see **[0151.0.0.0]**

**[0152.0.25.25]** The nucleic acid molecule of the invention encodes a polypeptide or portion thereof which includes an amino acid sequence which is sufficiently homologous to an amino acid sequence as indicated in Table II, columns 5 or 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp., such that the protein or portion thereof maintains the ability to participate in the respective fine chemical production, in particular an activity increasing the level of carbohydrates, preferably polysaccharides, more preferably starch and/or cellulose and/or monosaccharides, more preferably fructose, glucose and/or myo-inositol and/or trisaccharides, more preferably raffinose and/or disaccharides, more preferably sucrose resp., as mentioned above or as described in the examples in plants or microorganisms is comprised.

**[0153.0.25.25]** As used herein, the language "sufficiently homologous" refers to proteins or portions thereof which have amino acid sequences which include a minimum number of identical or equivalent amino acid residues (e.g., an amino acid residue which has a similar side chain as an amino acid residue in one of the sequences of the polypeptide of the present invention) to an amino acid sequence as indicated in Table II, columns 5 or 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp., such that the protein or portion thereof is able to participate in the increase of the respective fine chemical production. In one embodiment, a protein or portion thereof as indicated in Table II, columns 5 or 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp., has for example an activity of a polypeptide as indicaded in Table II, column 3, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp..

**[0154.0.25.25]** In one embodiment, the nucleic acid molecule of the present invention comprises a nucleic acid that encodes a portion of the protein of the present invention. The protein is at least about 30%, 35%, 40%, 45% or 50%, preferably at least about 55%, 60%, 65% or 70%, and more preferably at least about 75%, 80%, 85%, 90%, 91%, 92%, 93% or 94% and most preferably at least about 95%, 97%, 98%, 99% or more homologous to an entire amino acid sequence as indicated in Table II, columns 5 or 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp., and has above-mentioned activity, e.g.conferring preferably the increase of the respective fine chemical.

**[0155.0.0.25]** to **[0156.0.0.25]: see [0155.0.0.0]** to **[0156.0.0.0]**

**[0157.0.25]The** invention further relates to nucleic acid molecules that differ from one of the nucleotide sequences indicated in Table I, columns 5 or 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp. (and portions thereof) due to degeneracy of the genetic code and thus encode a polypeptide of the present invention, in particular a polypeptide having above mentioned activity, e.g. conferring an increase in the respective fine chemical in a organism, e.g. as that polypeptides comprising a consensus sequence as indicated in Table IV, column 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp. or of the polypeptide as indicated in Table II, columns 5 or 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp. or their functional homologues. Advantageously, the nucleic acid molecule of the invention or the nucleic acid molecule used in the method of the invention comprises, or in an other embodiment has, a nucleotide sequence encoding a protein comprising, or in an other embodiment having, a consensus sequences as indicated in Table IV, column 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp.or of the polypeptide as indicated in Table II, column 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp. or the functional homologues. In a still further embodiment, the nucleic acid molecule of the invention or the nucleic acid molecule used in the method of the invention encodes a full length protein which is substantially homologous to an amino acid sequence comprising a consensus sequence as indicated in Table IV, column 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp.or of a polypeptide as indicated in Table II, columns 5 or 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp. or the functional homologues thereof. However, in a preferred embodiment, the nucleic acid molecule of the present invention does not consist of a sequence as indicated in Table I, columns 5 or 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp., preferably as indicated in Table I A, columns 5 or 7 lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp.. Preferably the nucleic acid molecule of the invention is a functional homologue or identical to a nucleic acid molecule indicated in Table I B, columns 5 or 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp..

**[0158.0.0.25]** to **[0160.0.0.25]: see [0158.0.0.0]** to **[0160.0.0.0]**

**[0161.0.25.25]:** Accordingly, in another embodiment, a nucleic acid molecule of the invention is at least 15, 20, 25 or 30 nucleotides in length. Preferably, it hybridizes under stringent conditions to a nucleic acid molecule comprising a nucleotide sequence of the nucleic acid molecule of the present invention or used in the process of the present invention, e.g. comprising a sequence as indicated in Table I, columns 5 or 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp.. The nucleic acid molecule is preferably at least 20, 30, 50, 100, 250 or more nucleotides in length.

**[0162.0.0.25]:** see **[0162.0.0.0]**

**[0163.0.25.25]:** Preferably, nucleic acid molecule of the invention that hybridizes under stringent conditions to a sequence as indicated in Table I, columns 5 or 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp., corresponds to a naturally-occurring nucleic acid molecule of the invention. As used herein, a "naturally-occurring" nucleic acid molecule refers to an RNA or DNA molecule having a nucleotide sequence that occurs in nature (e.g., encodes a natural protein). Preferably, the nucleic acid molecule encodes a natural protein having above-mentioned activity, e.g. conferring the respective fine chemical increase after increasing the expression or activity thereof or the activity of a protein of the invention or used in the process of the invention.

**[0164.0.0.25]: see [0164.0.0.0]**

**[0165.0.25.25]** For example, nucleotide substitutions leading to amino acid substitutions at "non-essential" amino acid residues can be made in a sequence of the nucleic acid molecule of the invention or used in the process of the invention, e.g. as indicated in Table I, columns 5 or 7 lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp..

**[0166.0.0.25] to [0167.0.0.25]: see [0166.0.0.0] to [0167.0.0.0]**

**[0168.0.25.25]:** Accordingly, the invention relates to nucleic acid molecules encoding a polypeptide having above-mentioned activity, e.g. conferring an increase in the the respective fine chemical in an organisms or parts thereof that contain changes in amino acid residues that are not essential for said activity. Such polypeptides differ in amino acid sequence from a sequence contained in a sequence as indicated in Table II, columns 5 or 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp., preferably of Table II B, column 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp.yet retain said activity described herein. The nucleic acid molecule can comprise a nucleotide sequence encoding a polypeptide, wherein the polypeptide comprises an amino acid sequence at least about 50% identical to an amino acid sequence as indicated in Table II, columns 5 or 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp., preferably of Table II B, column 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp.and is capable of participation in the increase of production of the respective fine chemical after increasing its activity, e.g. its expression. Preferably, the protein encoded by the nucleic acid molecule is at least about 60% identical to a sequence as indicated in Table II, columns 5 or 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp., preferably of Table II B, column 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp., more preferably at least about 70% identical to one of the sequences as indicated in Table II, columns 5 or 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp., preferably of Table II B, column 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp., even more preferably at least about 80%, 90%, or 95% homologous to a sequence as indicated in Table II, columns 5 or 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp., preferably of Table II B, column 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp., and most preferably at least about 96%, 97%, 98%, or 99% identical to the sequence as indicated in Table II, columns 5 or 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp., preferably of Table II B, column 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp..

**[0169.0.0.25]** to **[0172.0.0.25]: see [0169.0.0.0]** to **[0172.0.0.0]**

**[0173.0.25.25]:** For example a sequence, which has 80% homology with sequence SEQ ID NO: 28606 at the nucleic acid level is understood as meaning a sequence which, upon comparison with the sequence SEQ ID NO: 28606 by the above Gap program algorithm with the above parameter set, has a 80% homology.

**[0174.0.0.25]: see [0174.0.0.0]**

**[0175.0.25.25]:** For example a sequence which has a 80% homology with sequence SEQ ID NO: 28607 at the protein level is understood as meaning a sequence which, upon comparison with the sequence SEQ ID NO: 28607 by the above program algorithm with the above parameter set, has a 80% homology.

**[0176.0.25.25]:** Functional equivalents derived from one of the polypeptides as indicated in Table II, columns 5 or 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp., according to the invention by substitution, insertion or deletion have at least 30%, 35%, 40%, 45% or 50%, preferably at least 55%, 60%, 65% or 70% by preference at least 80%, especially preferably at least 85% or 90%, 91%, 92%, 93% or 94%, very especially preferably at least 95%, 97%, 98% or 99% homology with one of the polypeptides as indicated in Table II, columns 5 or 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp., according to the invention and are distinguished by essentially the same properties as a polypeptide as indicated in Table II, columns 5 or 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp..

**[0177.0.25.25]:** Functional equivalents derived from a nucleic acid sequence as indicated in Table I, preferably in Table I B, columns 5 or 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp., according to the invention by substitution, insertion or deletion have at least 30%, 35%, 40%, 45% or 50%, preferably at least 55%, 60%, 65% or 70% by preference at least 80%, especially preferably at least 85% or 90%, 91 %, 92%, 93% or 94%, very especially preferably at least 95%, 97%, 98% or 99% homology with one of the polypeptides as indicated in Table II, columns 5 or 7 lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp., according to the invention and encode polypeptides having essentially the same properties as a polypeptide as indicated in Table II, preferably in Table I B, columns 5 or 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp..

**[0178.0.0.25]: see [0178.0.0.0]**

**[0179.0.25.25]:** A nucleic acid molecule encoding an homologous to a protein sequence as indicated in Table II, columns 5 or 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp., preferably in Table I B, column 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp., can be created by introducing one or more nucleotide substitutions, additions or deletions into a nucleotide sequence of the nucleic acid molecule of the present invention, in particular as indicated in Table I, columns 5 or 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp., such that one or more amino acid substitutions, additions or deletions are introduced into the encoded protein. Mutations can be introduced into the encoding sequences of a sequences as indicated in Table I, columns 5 or 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp., by standard techniques, such as site-directed mutagenesis and PCR-mediated mutagenesis.

**[0180.0.0.25] to [0183.0.0.25]: see [0180.0.0.0] to [0183.0.0.0]**

**[0184.0.25.25]** Homologues of the nucleic acid sequences used, with a sequence as indicated in Table I, columns 5 or 7, preferably in Table I B, column 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp., or of the nucleic acid sequences derived from a sequences as indicated in Table II, columns 5 or 7, preferably in Table II B, column 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp., comprise also allelic variants with at least approximately 30%, 35%, 40% or 45% homology, by preference at least approximately 50%, 60% or 70%, more preferably at least approximately 90%, 91%, 92%, 93%, 94% or 95% and even more preferably at least approximately 96%, 97%, 98%, 99% or more homology with one of the nucleotide sequences shown or the abovementioned derived nucleic acid sequences or their homologues, derivatives or analogues or parts of these. Allelic variants encompass in particular functional variants which can be obtained by deletion, insertion or substitution of nucleotides from the sequences shown, preferably from a sequence as indicated in Table I, columns 5 or 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp., or from the derived nucleic acid sequences, the intention being, however, that the enzyme activity or the biological activity of the resulting proteins synthesized is advantageously retained or increased.

**[0185.0.25.25]** In one embodiment of the present invention, the nucleic acid molecule of the invention or used in the process of the invention comprises one or more sequences as indicated in Table I, columns 5 or 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp., preferably in Table I B, column 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp.. In one embodiment, it is preferred that the nucleic acid molecule comprises as little as possible other nucleotides not shown in any one of sequences as indicated in Table I, columns 5 or 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp., preferably in Table I B, column 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp.. In one embodiment, the nucleic acid molecule comprises less than 500, 400, 300, 200, 100, 90, 80, 70, 60, 50 or 40 further nucleotides. In a further embodiment, the nucleic acid molecule comprises less than 30, 20 or 10 further nucleotides. In one embodiment, a nucleic acid molecule used in the process of the invention is identical to a sequences as indicated in Table I, columns 5 or 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp., preferably in Table I B, column 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp.

**[0186.0.25.25]** Also preferred is that one or more nucleic acid molecule(s) used in the process of the invention encodes a polypeptide comprising a sequence as indicated in Table II, columns 5 or 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp., preferably in Table II B, column 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp.. In one embodiment, the nucleic acid molecule encodes less than 150, 130, 100, 80, 60, 50, 40 or 30 further amino acids. In a further embodiment, the encoded polypeptide comprises less than 20, 15, 10, 9, 8, 7, 6 or 5 further amino acids. In one embodiment, the encoded polypeptide used in the process of the invention is identical to the sequences as indicated in Table II, columns 5 or 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp., preferably in Table II B, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp..

**[0187.0.25.25]** In one embodiment, a nucleic acid molecule of the invention or used in the process encodes a polypeptide comprising the sequence as indicated in Table II, columns 5 or 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp., preferably in Table II B, column 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp., and comprises less than 100 further nucleotides. In a further embodiment, said nucleic acid molecule comprises less than 30 further nucleotides. In one embodiment, the nucleic acid molecule used in the process is identical to a coding sequence encoding a polypeptide as indicated in Table II, columns 5 or 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp., preferably in Table II B, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp..

**[0188.0.25.25]** Polypeptides (= proteins), which still have the essential biological or enzymatic activity of the polypeptide of the present invention conferring an increase of the respective fine chemical i.e. whose activity is essentially not reduced, are polypeptides with at least 10% or 20%, by preference 30% or 40%, especially preferably 50% or 60%, very especially preferably 80% or 90 or more of the wild type biological activity or enzyme activity, advantageously, the activity is essentially not reduced in comparison with the activity of a polypeptide as indicated in Table II, columns 5 or 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp., preferably compared to a sequence as indicated in Table II, column 3 and 5, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp., and expressed under identical conditions.
In one embodiment, the polypeptide of the invention is a homolog consisting of or comprising the sequence as indicated in Table II B, columns 7, lines 290 to 294 and/or .604 to 607; lines 295 to 302 and/or 608 to 616; lines 303 to 308 and/or 617 to 621; lines 309 to 322 and/or 622 to 625; lines 323 to 331 and/or 626; lines 332 to 333, resp..

**[0189.0.25.25]** Homologues of a sequences as indicated in Table I, columns 5 or 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp., or of a sequence derived from a sequence as indicated in Table II, columns 5 or 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp., also mean truncated sequences, cDNA, single-stranded DNA or RNA of the coding and noncoding DNA sequence. Homologues of said sequences are also understood as meaning derivatives, which comprise noncoding regions such as, for example, UTRs, terminators, enhancers or promoter variants. The promoters upstream of the nucleotide sequences stated can be modified by one or more nucleotide substitution(s), insertion(s) and/or deletion(s) without, however, interfering with the functionality or activity either of the promoters, the open reading frame (= ORF) or with the 3'-regulatory region such as terminators or other 3'regulatory regions, which are far away from the ORF. It is furthermore possible that the activity of the promoters is increased by modification of their sequence, or that they are replaced completely by more active promoters, even promoters from heterologous organisms. Appropriate promoters are known to the person skilled in the art and are mentioned herein below.

**[0190.0.0.25] see [0190.0.0.0]**

**[0191.0.0.25] see [0191.0.0.0]:**

**[0192.0.0.25] to [0203.0.0.25] see [0192.0.0.0] to [0203.0.0.0]**

**[0204.0.25.25]** Accordingly, in one embodiment, the invention relates to a nucleic acid molecule, which comprises a nucleic acid molecule selected from the group consisting of:
a) nucleic acid molecule encoding, preferably at least the mature form, of a polypeptide as indicated in Table II, columns 5 or 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp., preferably in Table II B, column 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp., or a fragment thereof conferring an increase in the amount of the respective fine chemical, i.e. carbohydrates, preferably polysaccharides, more preferably starch and/or cellulose and/or monosaccharides, more preferably fructose, glucose and/or myo-inositol and/or trisaccharides, more preferably raffinose and/or disaccharides, more preferably sucrose resp., in an organism or a part thereof
b) nucleic acid molecule comprising, preferably at least the mature form, of a nucleic acid molecule as indicated in Table I, columns 5 or 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp., preferably in Table I B, column 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp., , or a fragment thereof conferring an increase in the amount of the respective fine chemical in an organism or a part thereof;
c) nucleic acid molecule whose sequence can be deduced from a polypeptide sequence encoded by a nucleic acid molecule of (a) or (b) as result of the degeneracy of the genetic code and conferring an increase in the amount of the respective fine chemical in an organism or a part thereof;
d) nucleic acid molecule encoding a polypeptide whose sequence has at least 50% identity with the amino acid sequence of the polypeptide encoded by the nucleic acid molecule of (a) to (c) and conferring an increase in the amount of the respective fine chemical in an organism or a part thereof;
e) nucleic acid molecule which hybridizes with a nucleic acid molecule of (a) to (c) under under stringent hybridisation conditions and conferring an increase in the amount of the respective fine chemical in an organism or a part thereof;
f) nucleic acid molecule encoding a polypeptide, the polypeptide being derived by substituting, deleting and/or adding one or more amino acids of the amino acid sequence of the polypeptide encoded by the nucleic acid molecules (a) to (d), preferably to (a) to (c), and conferring an increase in the amount of the respective fine chemical in an organism or a part thereof;
g) nucleic acid molecule encoding a fragment or an epitope of a polypeptide which is encoded by one of the nucleic acid molecules of (a) to (e), preferably to (a) to (c) and conferring an increase in the amount of the respective fine chemical in an organism or a part thereof ;
h) nucleic acid molecule comprising a nucleic acid molecule which is obtained by amplifying a cDNA library or a genomic library using primers or primer pairs as indicated in Table III, column or 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp., and conferring an increase in the amount of the respective fine chemical, i.e. carbohydrates, preferably polysaccharides, more preferably starch and/or cellulose and/or monosaccharides, more preferably fructose, glucose and/or myo-inositol and/or trisaccharides, more preferably raffinose and/or disaccharides, more preferably sucrose resp. in an organism or a part thereof;
i) nucleic acid molecule encoding a polypeptide which is isolated, e.g. from a expression library, with the aid of monoclonal antibodies against a polypeptide encoded by one of the nucleic acid molecules of (a) to (g), preferably to (a) to (c) and conferring an increase in the amount of the respective fine chemical in an organism or a part thereof;
j) nucleic acid molecule which encodes a polypeptide comprising a consensus sequence as indicated in Table IV, column 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp., and conferring an increase in the amount of the respective fine chemical, i.e. carbohydrates, preferably polysaccharides, more preferably starch and/or cellulose and/or monosaccharides, more preferably fructose, glucose and/or myo-inositol and/or trisaccharides, more preferably raffinose and/or disaccharides, more preferably sucrose resp., in an organism or a part thereof;
k) nucleic acid molecule encoding the amino acid sequence of a polypeptide encoding a domaine of a polypeptide as indicated in Table II, columns 5 or 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp., preferably in Table II B, column 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp., and conferring an increase in the amount of the respective fine chemical, i.e. carbohydrates, preferably polysaccharides, more preferably starch and/or cellulose and/or monosaccharides, more preferably fructose, glucose and/or myo-inositol and/or trisaccharides, more preferably raffinose and/or disaccharides, more preferably sucrose resp., in an organism or a part thereof; and
l) nucleic acid molecule which is obtainable by screening a suitable nucleic acid library under stringent hybridization conditions with a probe comprising one of the sequences of the nucleic acid molecule of (a) to (k) or with a fragment of at least 15 nt, preferably 20 nt, 30 nt, 50 nt, 100 nt, 200 nt or 500 nt of the nucleic acid molecule characterized in (a) to (h) or of a nucleic acid molecule as indicated in Table I, columns 5 or 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp., preferably in Table I B, column 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp.,, or a nucleic acid molecule encoding, preferably at least the mature form of, a polypeptide as indicated in Table II, columns 5 or 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp., preferably in Table II B, column 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp., and conferring an increase in the amount of the respective fine chemical in an organism or a part thereof; or which encompasses a sequence which is complementary thereto;
whereby, preferably, the nucleic acid molecule according to (a) to (I) distinguishes over the sequence indicated in Table IA or, columns 5 or 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp., by one or more nucleotides. In one embodiment, the nucleic acid molecule does not consist of the sequence shown and indicated in Table I A or I B, columns 5 or 7 lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp..: In one embodiment, the nucleic acid molecule is less than 100%, 99,999%, 99,99%, 99,9% or 99% identical to a sequence indicated in Table I A or I B, columns 5 or 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp.. In another embodiment, the nucleic acid molecule does not encode a polypeptide of a sequence indicated in Table II A or II B, columns 5 or 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp.. In an other embodiment, the nucleic acid molecule of the present invention is at least 30 %, 40 %, 50%, or 60% identical and less than 100%, 99,999%, 99,99%, 99,9% or 99% identical to a sequence indicated in Table I A or I B, columns 5 or 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp.. In a further embodiment the nucleic acid molecule does not encode a polypeptide sequence as indicated in Table II A or II B, columns 5 or 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp.. Accordingly, in one embodiment, the nucleic acid molecule of the differs at least in one or more residues from a nucleic acid molecule indicated in Table I A or I B, columns 5 or 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp.. Accordingly, in one embodiment, the nucleic acid molecule of the present invention encodes a polypeptide, which differs at least in one or more amino acids from a polypeptide indicated in Table II A or II B, columns 5 or 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp.. In another embodiment, a nucleic acid molecule indicated in Table I A or I B, columns 5 or 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp.does not encode a protein of a sequence indicated in Table II A or II B, columns 5 or 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp.. Accordingly, in one embodiment, the protein encoded by a sequences of a nucleic acid accoriding to (a) to (I) does not consist of a sequence as indicated in Table II A or II B, columns 5 or 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp.. In a further embodiment, the protein of the present invention is at least 30 %, 40 %, 50%, or 60% identical to a protein sequence indicated in Table II A or II B, columns 5 or 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp.and less than 100%, preferably less than 99,999%, 99,99% or 99,9%, more preferably less than 99%, 985, 97%, 96% or 95% identical to a sequence as indicated in Table I A or II B, columns 5 or 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp..

**[0205.0.0.25]** to **[0206.0.0.25] see [0205.0.0.0]** to **[0206.0.0.0]**

**[0207.0.25.25]** As described herein, the nucleic acid construct can also comprise further genes, which are to be introduced into the organisms or cells. It is possible and advantageous to introduce into, and express in, the host organisms regulatory genes such as genes for inductors, repressors or enzymes, which, owing to their enzymatic activity, engage in the regulation of one or more genes of a biosynthetic pathway. These genes can be of heterologous or homologous origin. Moreover, further biosynthesis genes may advantageously be present, or else these genes may be located on one or more further nucleic acid constructs. Genes, which are advantageously employed as biosynthesis genes, are genes of the carbohydrate or starch metabolism, the glucose metabolism, the saccharide metabolism, the metabolism of glycolysis, or their combinations. As described herein, regulator sequences or factors can have a positive effect on preferably the gene expression of the genes introduced, thus increasing it. Thus, an enhancement of the regulator elements may advantageously take place at the transcriptional level by using strong transcription signals such as promoters and/or enhancers. In addition, however, an enhancement of translation is also possible, for example by increasing mRNA stability or by inserting a translation enhancer sequence.

**[0208.0.0.25] to [0226.0.0.25]: see [0208.0.0.0] to [0226.0.0.0]**

**[0227.0.25.25]:** The abovementioned nucleic acid molecules can be cloned into the nucleic acid constructs or vectors according to the invention in combination together with further genes, or else different genes are introduced by transforming several nucleic acid constructs or vectors (including plasmids) into a host cell, advantageously into a plant cell or a microorgansims.
In addition to a sequence indicated in Table I, columns 5 or 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp., or its derivatives, it is advantageous additionally to express and/or mutate further genes in the organisms. Especially advantageously, additionally at least one further gene of the carbohydrate biosynthetic pathway is expressed in the organisms such as plants or microorganisms. It is also possible that the regulation of the natural genes has been modified advantageously so that the gene and/or its gene product is no longer subject to the regulatory mechanisms which exist in the organisms. This leads to an increased synthesis of the carbohydrate desired since, for example, feedback regulations no longer exist to the same extent or not at all. In addition it might be advantageously to combine one or more of the sequences indicated in Table I, columns 5 or 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp., with genes which generally support or enhances to growth or yield of the target organismen, for example genes which lead to faster growth rate of microorganisms or genes which produces stress-, pathogen, or herbicide resistant plants.

**[0228.0.25.25:** In a further embodiment of the process of the invention, therefore, organisms are grown, in which there is simultaneous overexpression of at least one nucleic acid or one of the genes which code for proteins involved in the carbohydrate metabolism, in particular in synthesis of polysaccharides, more preferably starch and/or cellulose and/or monosaccharides, more preferably fructose, glucose and/or myo-inositol and/or trisaccharides, more preferably raffinose and/or disaccharides, more preferably sucrose.

**[0229.0.25.25]:** Further advantageous nucleic acid sequences which can be expressed in combination with the sequences used in the process and/or the abovementioned biosynthesis genes are the sequences encoding further genes of the carbohydrate biosynthetic pathway. These genes can lead to an increased synthesis of the carbohydrates, preferably polysaccharides, more preferably starch and/or cellulose and/or monosaccharides, more preferably fructose, glucose and/or myo-inositol and/or trisaccharides, more preferably raffinose and/or disaccharides, more preferably sucrose resp.

**[0230.0.0.25] see [0230.0.0.0]**

**[0231.0.25.25]** In a further advantageous embodiment of the process of the invention, the organisms used in the process are those in which simultaneously a carbohydrate degrading protein is attenuated, in particular by reducing the rate of expression of the corresponding gene.

**[0232.0.0.25] to [0276.0.0.25]: see [0232.0.0.0] to [0276.0.0.0]**

**[0277.0..25.25]** The respective fine chemical produced can be isolated from the organism by methods with which the skilled worker is familiar. For example, via extraction, chemical or enzymatical degradiation, crystallization, salt precipitation, and/or different chromatography methods. The process according to the invention can be conducted batchwise, semibatchwise or continuously. The respective fine chemcical produced by this process can be obtained by harvesting the organisms, either from the crop in which they grow, or from the field. This can be done via pressing or extraction of the plant parts

**[0278.0.0.25]** to **[0282.0.0.25]: see [0278.0.0.0]** to **[0282.0.0.0]**

**[0283.0.25.25]:** Moreover, a native polypeptide conferring the increase of the respective fine chemical in an organism or part thereof can be isolated from cells (e.g., endothelial cells), for example using the antibody of the present invention as described below, e.g. an antibody against a protein as indicated in Table II, column 3, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp., or an antibody against a polypeptide as indicated in Table II, columns 5 or 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp., which can be produced by standard techniques utilizing the polypeptid of the present invention or fragment thereof, i.e., the polypeptide of this invention. Preferred are monoclonal antibodies.

**[0284.0.0.25]: see [0284.0.0.0]**

**[0285.0.25.25]:** In one embodiment, the present invention relates to a polypeptide having a sequence as indicated in Table II, columns 5 or 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp., or as encoded by a nucleic acid molecule as indicated in Table I, columns 5 or 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp., or functional homologues thereof.

**[0286.0.25.25]:** In one advantageous embodiment, in the method of the present invention the activity of a polypeptide is increased which comprises or consists of a consensus sequence as indicated in Table IV, column 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp.. In another embodiment, the present invention relates to a polypeptide comprising or consisting of a consensus sequence as indicated in Table IV, column 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp., whereby 20 or less, preferably 15 or 10, preferably 9, 8, 7, or 6, more preferred 5 or 4, even more preferred 3, even more preferred 2, even more preferred 1, most preferred 0 of the amino acids positions indicated can be replaced by any amino acid and/or be absent.
In one embodiment, the present invention relates to the method of the present invention comprising a polypeptide or to a polypeptide comprising more than one consensus sequences (of an individual line) as indicated in Table IV, column 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp..

**[0287.0.0.25]** to **[0290.0.0.25]: see [0287.0.0.0]** to **[0290.0.0.0]**

**[0291.0.25.25]:** In one advantageous embodiment, the method of the present invention comprises the increasing of a polypeptide comprising or consisting of plant or microorganism specific consensus sequences. Accordingly, in one embodiment, the present invention relates to a polypeptide comprising or consisting of plant or microorganism specific consensus sequences.
In one embodiment, said polypeptide of the invention distinguishes over a sequence as indicated in Table II A or II B, columns 5 or 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp. by one or more amino acids. In one embodiment, polypeptide distinguishes form a sequence as indicated in Table II A or IIB, columns 5 or 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp. by more than 1, 2, 3, 4, 5, 6, 7, 8 or 9 amino acids, preferably by more than 10, 15, 20, 25 or 30 amino acids, evenmore preferred are more than 40, 50, or 60 amino acids and, preferably, the sequence of the polypeptide of the invention distinguishes from a sequence as indicated in Table II A or II B, columns 5 or 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp. by not more than 80% or 70% of the amino acids, preferably not more than 60% or 50%, more preferred not more than 40% or 30%, even more preferred not more than 20% or 10%. In an other embodiment, said polypeptide of the invention does not consist of a sequence as indicated in Table II A or II B, columns 5 or 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp..

**[0292.0.0.25]: see [0292.0.0.0]**

**[0293.0.25.25]:** In one embodiment, the invention relates to polypeptide conferring an increase in the respective fine chemical in an organism or part being encoded by the nucleic acid molecule of the invention or by a nucleic acid molecule used in the process of the invention.
In one embodiment, the polypeptide of the invention has a sequence which distinguishes from a sequence as indicated in Table II A or II B, columns 5 or 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp. by one or more amino acids. In an other embodiment, said polypeptide of the invention does not consist of the sequence as indicated in Table II A or II B, columns 5 or 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp.. In a further embodiment, said polypeptide of the present invention is less than 100%, 99,999%, 99,99%, 99,9% or 99% identical. In one embodiment, said polypeptide does not consist of the sequence encoded by a nucleic acid molecules as indicated in Table I A or I B, columns 5 or 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp..

**[0294.0.25.25]** In one embodiment, the present invention relates to a polypeptide having an activity of a protein as indicated in Table II, column 3, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp., which distinguishes over a sequence as indicated in Table II A or II B, columns 5 or 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp. by one or more amino acids, preferably by more than 5, 6, 7, 8 or 9 amino acids, preferably by more than 10, 15, 20, 25 or 30 amino acids, evenmore preferred are more than 40, 50, or 60 amino acids but even more preferred by less than 70% of the amino acids, more preferred by less than 50%, even more preferred my less than 30% or 25%, more preferred are 20% or 15%, even more preferred are less than 10%.

**[0295.0.0.25]** to **[0297.0.0.25]: see [0295.0.0.0]** to **[0297.0.0.0]**

**[0297.1.25.25]** Non polypeptide of the invention-chemicals are e.g. polypeptides having not the activity of a polypeptide indicated in Table II, columns 3, 5 or 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp..

**[0298.0.25.25]:** A polypeptide of the invention can participate in the process of the present invention. The polypeptide or a portion thereof comprises preferably an amino acid sequence which is sufficiently homologous to an amino acid sequence as indicated in Table II, columns 5 or 7 lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp.. The portion of the protein is preferably a biologically active portion as described herein. Preferably, the polypeptide used in the process of the invention has an amino acid sequence identical to a sequence as indicated in Table II, columns 5 or 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp..

**[0299.0.25.25]:** Further, the polypeptide can have an amino acid sequence which is encoded by a nucleotide sequence which hybridizes, preferably hybridizes under stringent conditions as described above, to a nucleotide sequence of the nucleic acid molecule of the present invention. Accordingly, the polypeptide has an amino acid sequence which is encoded by a nucleotide sequence that is at least about 35%, 40%, 45%, 50%, 55%, 60%, 65% or 70%, preferably at least about 75%, 80%, 85% or 90, and more preferably at least about 91%, 92%, 93%, 94% or 95%, and even more preferably at least about 96%, 97%, 98%, 99% or more homologous to one of the nucleotide sequence as indicated in Table I, columns 5 or 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp.. The preferred polypeptide of the present invention preferably possesses at least one of the activities according to the invention and described herein. A preferred polypeptide of the present invention includes an amino acid sequence encoded by a nucleotide sequence which hybridizes, preferably hybridizes under stringent conditions, to a nucleotide sequence as indicated in Table I, columns 5 or 7 lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp., or which is homologous thereto, as defined above.

**[0300.0.25.25]:** Accordingly the polypeptide of the present invention can vary from a sequence as indicated in Table II, columns 5 or 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp., in amino acid sequence due to natural variation or mutagenesis, as described in detail herein.
Accordingly, the polypeptide comprise an amino acid sequence which is at least about 35%, 40%, 45%, 50%, 55%, 60%, 65% or 70%, preferably at least about 75%, 80%, 85% or 90, and more preferably at least about 91%, 92%, 93%, 94% or 95%, and most preferably at least about 96%, 97%, 98%, 99% or more homologous to an entire amino acid sequence of a sequence as indicated in Table II A or II B, columns 5 or 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp..

**[0301.0.0.25] see [0301.0.0.0]**

**[0302.0.25.25]** Biologically active portions of an polypeptide of the present invention include peptides comprising amino acid sequences derived from the amino acid sequence of the polypeptide of the present invention or used in the process of the present invention, e.g., an amino acid sequence as indicated in Table II, columns 5 or 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp., or the amino acid sequence of a protein homologous thereto, which include fewer amino acids than a full length polypeptide of the present invention or used in the process of the present invention or the full length protein which is homologous to an polypeptide of the present invention or used in the process of the present invention depicted herein, and exhibit at least one activity of polypeptide of the present invention or used in the process of the present invention.

**[0303.0.0.25]: see [0303.0.0.0]**

**[0304.0.25.25]:** Manipulation of the nucleic acid molecule of the invention may result in the production of a protein having essentially the activity of the polypeptides as indicated in Table II, column 3, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp., but having differences in the sequence from said wild-type protein. These proteins may be improved in efficiency or activity, may be present in greater numbers in the cell than is usual, or may be decreased in efficiency or activity in relation to the wild type protein.

**[0305.0.0.25]** to **[0308.0.0.25]: see [0305.0.0.0]** to **[0308.0.0.0]**

**[0309.0.25.25]:** In one embodiment, a reference to a protein (= polypeptide) of the invention or as indicated in Table II, columns 5 or 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp., refers to a polypeptide having an amino acid sequence corresponding to the polypeptide of the invention or used in the process of the invention , whereas a " non-polypeptide of the invention" or "other polypeptide" not being indicated in Table II, columns 5 or 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp., refers to a polypeptide having an amino acid sequence corresponding to a protein which is not substantially homologous a polypeptide of the invention, preferably which is not substantially homologous to a polypeptide as indicated in Table II, columns 5 or 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp., e.g., a protein which does not confer the activity described herein or annotated or known for as indicated in Table II, column 3, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp., and which is derived from the same or a different organism. In one embodmeint, a "non-polypeptide of the invention" or "other polypeptide" not being indicated in Table II, columns 5 or 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp., does not confer an increase of the respective fine chemical in an organism or part therof.

**[0310.0.0.25] to [0334.0.0.25]: see [0310.0.0.0] to [0334.0.0.0]**

**[0335.0.25.25]:** The dsRNAi method has proved to be particularly effective and advantageous for reducing the expression of a nucleic acid sequences as indicated in Table I, columns 5 or 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp., and/or homologs thereof. As described inter alia in WO 99/32619, dsRNAi approaches are clearly superior to traditional antisense approaches. The invention therefore furthermore relates to double-stranded RNA molecules (dsRNA molecules) which, when introduced into an organism, advantageously into a plant (or a cell, tissue, organ or seed derived therefrom), bring about altered metabolic activity by the reduction in the expression of a nucleic acid sequences as indicated in Table I, columns 5 or 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp., and/or homologs thereof. In a double-stranded RNA molecule for reducing the expression of an protein encoded by a nucleic acid sequence sequences as indicated in Table I, columns 5 or 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp., and/or homologs thereof, one of the two RNA strands is essentially identical to at least part of a nucleic acid sequence, and the respective other RNA strand is essentially identical to at least part of the complementary strand of a nucleic acid sequence.

**[0336.0.0.25] to [0342.0.0.25]: see [0336.0.0.0] to [0342.0.0.0]**

**[0343.0.25.25]:** As has already been described, 100 % sequence identity between the dsRNA and a gene transcript of a nucleic acid sequence as indicated in Table I, columns 5 or 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp., or it's homolog is not necessarily required in order to bring about effective reduction in the expression. The advantage is, accordingly, that the method is tolerant with regard to sequence deviations as may be present as a consequence of genetic mutations, polymorphisms or evolutionary divergences. Thus, for example, using the dsRNA, which has been generated starting from a sequence as indicated in Table I, columns 5 or 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp., or homologs thereof of the one organism, may be used to suppress the corresponding expression in another organism.

**[0344.0.0.25] to [0361.0.0.25]: see [0344.0.0.0]** to **[0361.0.0.0]**

**[0362.0.25.25]:** Accordingly the present invention relates to any cell transgenic for any nucleic acid characterized as part of the invention, e.g. conferring the increase of the respective fine chemical in a cell or an organism or a part thereof, e.g. the nucleic acid molecule of the invention, the nucleic acid construct of the invention, the antisense molecule of the invention, the vector of the invention or a nucleic acid molecule encoding the polypeptide of the invention, e.g. the polypeptide as indicated in Table II, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp., e.g. encoding a polypeptide having protein activity, as indicated in Table II, columns 3, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp.. Due to the above mentioned activity the respective fine chemical content in a cell or an organism is increased. For example, due to modulation or manipulation, the cellular activity of the polypeptide of the invention or nucleic acid molecule of the invention is increased, e.g. due to an increased expression or specific activity of the subject matters of the invention in a cell or an organism or a part thereof. Transgenic for a polypeptide having an activity of a polypeptide as indicated in Table II, columns 5 or 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp., means herein that due to modulation or manipulation of the genome, an activity as annotated for a polypeptide as indicated in Table II, column 3, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp., e.g. having a sequence as indicated in Table II, columns 5 or 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp., is increased in a cell or an organism or a part thereof. Examples are described above in context with the process of the invention

**[0363.0.0.25]: see [0363.0.0.0]**

**[0364.0.0.25]: see [0364.0.0.0]**

**[0365.0.0.25] to [0373.0.0.25]: see [0365.0.0.0] to [0373.0.0.0]**

**[0374.0.25.25]:** Transgenic plants comprising the respective fine chemical synthesized in the process according to the invention can be marketed directly without isolation of the compounds synthesized. In the process according to the invention, plants are understood as meaning all plant parts, plant organs such as leaf, stalk, root, tubers or seeds or propagation material or harvested material or the intact plant. In this context, the seed encompasses all parts of the seed such as the seed coats, epidermal cells, seed cells, endosperm or embryonic tissue. Carbohydrates, preferably polysaccharides, more preferably starch and/or cellulose and/or monosaccharides, more preferably fructose, glucose and/or myo-inositol and/or trisaccharides, more preferably raffinose and/or disaccharides, more preferably sucrose resp., produced in the process according to the invention may, however, also be isolated from the plant in the form of their free form or bound in or to compounds or moieties. Carbohydrates, preferably polysaccharides, more preferably starch and/or cellulose and/or monosaccharides, more preferably fructose, glucose and/or myo-inositol and/or trisaccharides, more preferably raffinose and/or disaccharides, more preferably sucrose resp.,produced by this process can be harvested by harvesting the organisms either from the culture in which they grow or from the field. This can be done via expressing, grinding and/or extraction, salt precipitation and/or ion-exchange chromatography or other chromatographic methods of the plant parts, preferably the plant seeds, plant fruits, plant tubers and the like.

**[0375.0.0.25] to [0376.0.0.25]: see [0375.0.0.0] to [0376.0.0.0]**

**[0377.0.25.25]:** Accordingly, the present invention relates also to a process according to the present invention whereby the produced carbohydrates, preferably polysaccharides, more preferably starch and/or cellulose and/or monosaccharides, more preferably fructose, glucose and/or myo-inositol and/or trisaccharides, more preferably raffinose and/or disaccharides, more preferably sucrose comprising composition or the produced the respective fine chemical is isolated.

**[0378.0.25.25]:** In this manner, more than 50% by weight, advantageously more than 60% by weight, preferably more than 70% by weight, especially preferably more than 80% by weight, very especially preferably more than 90% by weight, of the carbohydrates, preferably polysaccharides, more preferably starch and/or cellulose and/or monosaccharides, more preferably fructose, glucose and/or myo-inositol and/or trisaccharides, more preferably raffinose and/or disaccharides, more preferably sucrose resp., produced in the process can be isolated. The resulting carbohydrates, preferably polysaccharides, more preferably starch and/or cellulose and/or monosaccharides, more preferably fructose, glucose and/or myo-inositol and/or trisaccharides, more preferably raffinose and/or disaccharides, more preferably sucrose resp.,can, if appropriate, subsequently be further purified, if desired mixed with other active ingredients such as vitamins, amino acids, carbohydrates, antibiotics and the like, and, if appropriate, formulated.

**[0379.0.25.25]:** In one embodiment, the carbohydrates, preferably polysaccharides, more preferably starch and/or cellulose and/or monosaccharides, more preferably fructose, glucose and/or myo-inositol and/or trisaccharides, more preferably raffinose and/or disaccharides, more preferably sucrose resp., is a mixture comprising of one or more the respective fine chemicals. In one embodiment, the respective fine chemical means here carbohydrates, preferably polysaccharides, more preferably starch and/or cellulose and/or monosaccharides, more preferably fructose, glucose and/or myo-inositol and/or trisaccharides, more preferably raffinose and/or disaccharides, more preferably sucrose. In one embodiment, carbohydrate means here a mixture of the respective fine chemicals.

**[0380.0.25.25]:** The carbohydrates, preferably polysaccharides, more preferably starch and/or cellulose and/or monosaccharides, more preferably fructose, glucose and/or myo-inositol and/or trisaccharides, more preferably raffinose and/or disaccharides, more preferably sucrose resp., obtained in the process are suitable as starting material for the synthesis of further products of value. For example, they can be used in combination with each other or alone for the production of pharmaceuticals, foodstuffs, animal feeds or cosmetics. Accordingly, the present invention relates a method for the production of pharmaceuticals, food stuff, animal feeds, nutrients or cosmetics comprising the steps of the process according to the invention, including the isolation of the carbohydrates, preferably polysaccharides, more preferably starch and/or cellulose and/or monosaccharides, more preferably fructose, glucose and/or myo-inositol and/or trisaccharides, more preferably raffinose and/or disaccharides, more preferably sucrose comprising composition produced or the respective fine chemical produced if desired and formulating the product with a pharmaceutical acceptable carrier or formulating the product in a form acceptable for an application in agriculture. A further embodiment according to the invention is the use of the carbohydrates, preferably polysaccharides, more preferably starch and/or cellulose and/or monosaccharides, more preferably fructose, glucose and/or myo-inositol and/or trisaccharides, more preferably raffinose and/or disaccharides, more preferably sucrose resp., produced in the process or of the transgenic organisms in animal feeds, foodstuffs, medicines, food supplements, cosmetics or pharmaceuticals.

**[0381.0.0.25]** to **[0382.0.0.25]: see [0381.0.0.0]** to **[0382.0.0.0]**

**[0383.0.9.25]:** ./.

**[0384.0.0.25]: see [0384.0.0.0]**

**[0385.0.25.25]:** The fermentation broths obtained in this way, containing in particular carbohydrates, preferably polysaccharides, more preferably starch and/or cellulose and/or monosaccharides, more preferably fructose, glucose and/or myo-inositol and/or trisaccharides, more preferably raffinose and/or disaccharides, more preferably sucrose, resp., in mixtures with other compounds, in particular with other carbohydrates , or fatty acids building lipids or containing microorganisms or parts of microorganisms, like plastids, containing carbohydrates, preferably polysaccharides, more preferably starch and/or cellulose and/or monosaccharides, more preferably fructose, glucose and/or myo-inositol and/or trisaccharides, more preferably raffinose and/or disaccharides, more preferably sucrose resp., in mixtures with other compounds, e.g. carbohydrates, normally have a dry matter content of from 7.5 to 25% by weight. The fermentation broth can then be thickened or concentrated by known methods, such as, for example, with the aid of a rotary evaporator, thin-film evaporator, falling film evaporator, by reverse osmosis or by nanofiltration. This concentrated fermentation broth can then be worked up by freeze-drying, spray drying, spray granulation or by other processes.

**[0386.0..0.25] -/-**

**[0387.0.0.25]** to **[0392.0.0.25]: see [0387.0.0.0]** to **[0392.0.0.0]**

**[0393.0.25.25]:** In one embodiment, the present invention relates to a method for the identification of a gene product conferring an increase in the respective fine chemical production in a cell, comprising the following steps:
(a) contacting, e.g. hybridising, the nucleic acid molecules of a sample, e.g. cells, tissues, plants or microorganisms or a nucleic acid library , which can contain a candidate gene encoding a gene product conferring an increase in the respective fine chemical after expression, with the nucleic acid molecule of the present invention;
(b) identifying the nucleic acid molecules, which hybridize under relaxed stringent conditions with the nucleic acid molecule of the present invention in particular to the nucleic acid molecule sequence as indicated in Table I, columns 5 or 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp., preferably in Table I B, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp.and, optionally, isolating the full length cDNA clone or complete genomic clone;
(c) introducing the candidate nucleic acid molecules in host cells, preferably in a plant cell or a microorganism, appropriate for producing the respective fine chemical;
(d) expressing the identified nucleic acid molecules in the host cells;
(e) assaying the the respective fine chemical level in the host cells; and
(f) identifying the nucleic acid molecule and its gene product which expression confers an increase in the respective the respective fine chemical level in the host cell after expression compared to the wild type.

**[0394.0.0.25] to [0399.0.0.25]: see [0394.0.0.0] to [0399.0.0.0]**

**[00399.1.9.9]:** One can think to screen for increased production of the respective fine chemical by for example searching for a resistance to a drug blocking the synthesis of the respective fine chemical and looking whether this effect is dependent on the activity or expression of a polypeptide as indicated in Table II, columns 5 or 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp., or a homolog thereof, e.g. comparing the phenotyp of nearly identical organisms with low and high acitivity of a protein as indicated in Table II, columns 5 or 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp., after incubation with the drug.

**[0400.0.0.25] to [0416.0.0.25]: see [0400.0.0.0] to [0416.0.0.0]**

**[0417.0.9.9]:** The nucleic acid molecule of the invention, the vector of the invention or the nucleic acid construct of the invention may also be useful for the production of organisms resistant to inhibitors of the carbohydrates, preferably polysaccharides, more preferably starch and/or cellulose and/or monosaccharides, more preferably fructose, glucose and/or myo-inositol and/or trisaccharides, more preferably raffinose and/or disaccharides, more preferably sucrose, resp.,production biosynthesis pathways. In particular, the overexpression of the polypeptide of the present invention may protect an organism such as a microorganism or a plant against inhibitors, which block the carbohydrate, in particular the respective fine chemical synthesis in said organism. Examples of inhibitors or herbicides blocking the synthesis in organism such as microorganism or plants are for example the cellulose synthesis inhibitors 2,6-dichlorobenzonitrile (DCB), N2-(1-ethyl-3-phenylpropyl)-6-(1-fluoro-1-methylethyl)-1,3,5-triazine-2,4-diamine, called AE F150944 or isoxaben.

**[0418.0.0.25] to [0423.0.0.25]: see [0418.0.0.0] to [0423.0.0.0]**

**[0424.0.25.25]:** Accordingly, the nucleic acid of the invention, the polypeptide of the invention, the nucleic acid construct of the invention, the organisms, the host cell, the microorgansims, the plant, plant tissue, plant cell, or the part thereof of the invention, the vector of the invention, the agonist identified with the method of the invention, the nucleic acid molecule identified with the method of the present invention, can be used for the production of the respective fine chemical or of the respective fine chemical and one or more other carbohydrate.
Accordingly, the nucleic acid of the invention, or the nucleic acid molecule identified with the method of the present invention or the complement sequences thereof, the polypeptide of the invention, the nucleic acid construct of the invention, the organisms, the host cell, the microorgansims, the plant, plant tissue, plant cell, or the part thereof of the invention, the vector of the invention, the antagonist identified with the method of the invention, the antibody of the present invention, the antisense molecule of the present invention, can be used for the reduction of the respective fine chemical in a organism or part thereof, e.g. in a cell.

**[0425.0.0.25] to [0454.0.0.25]: see [0425.0.0.0] to [0453.0.0.0]**

**[0454.0.25.25]** Example 8: Analysis of the effect of the nucleic acid molecule on the production of the fine chemical resp.

**[0455.0.25.25]** The effect of the genetic modification in C. glutamicum or other microbial especially bacterial strains for carbohydrate production on the production of an carbohydrate can be determined by growing the modified microorganisms under suitable conditions (such as those described above) and analyzing the medium and/or the cellular components for the increased production of the amino acid. Such analytical techniques are well known to the skilled worker and encompass spectroscopy, thin-layer chromatography, various types of staining methods, enzymatic and microbiological methods and analytical chromatography such as high-performance liquid chromatography (see, for example, Ullman, Encyclopedia of Industrial Chemistry, Vol. A2, pp. 89-90 and pp. 443-613, VCH: Weinheim (1985); Fallon, A., et al., (1987) "Applications of HPLC in Biochemistry" in: Laboratory Techniques in Biochemistry and Molecular Biology, Vol. 17; Rehm et al. (1993) Biotechnology, Vol. 3, Chapter III: "Product recovery and purification", pp. 469-714, VCH: Weinheim; Belter, P.A. et al. (1988) Bioseparations: downstream processing for Biotechnology, John Wiley and Sons; Kennedy, J.F. and Cabral, J.M.S. (1992) Recovery processes for biological Materials, John Wiley and Sons; Shaeiwitz, J.A. and Henry, J.D. (1988) Biochemical Separations, in Ullmann's Encyclopedia of Industrial Chemistry, Vol. B3; chapter 11, pp. 1-27, VCH: Weinheim; and Dechow, F.J. (1989) Separation and purification techniques in biotechnology, Noyes Publications).

**[0456.0.0.25]: see [0456.0.0.0]**

**[0457.0.25.25]**: Example 9: Purification of the carbohydrates, preferably polysaccharides, more preferably starch and/or cellulose and/or monosaccharides, more preferably fructose, glucose and/or myo-inositol and/or trisaccharides, more preferably raffinose and/or disaccharides, more preferably sucrose

**[0458.0.25.25]:** Abbreviations:; GC-MS, gas liquid chromatography/mass spectrometry; TLC, thin-layer chromatography.
The unambiguous detection for the presence of carbohydrates, preferably polysaccharides, more preferably starch and/or cellulose and/or monosaccharides, more preferably fructose, glucose and/or myo-inositol and/or trisaccharides, more preferably raffinose and/or disaccharides, more preferably sucrosecan be obtained by analyzing recombinant organisms using analytical standard methods: GC, GC-MS or TLC, as described (1997, in: Advances on Lipid Methodology, Fourth Edition: Christie, Oily Press, Dundee, 119-169; 1998, Gaschromatographie-Massenspektrometrie-Verfahren [Gas chromatography/mass spectrometric methods], Lipide 33:343-353). The total carbohydrate produced in the organism for example in yeasts used in the inventive process can be analysed for example according to the following procedure:
The material such as yeasts, E. coli or plants to be analyzed can be disrupted by sonication, grinding in a glass mill, liquid nitrogen and grinding or via other applicable methods.
Plant material is initially homogenized mechanically by comminuting in a pestle and mortar to make it more amenable to extraction.

**-[0459.0.25.25]: -/-**

**[0460.0.0.25] see [0460.0.0.0]**

**[0461.0.25.25]** Example 10: Cloning SEQ ID NO: 28606 for the expression in plants

**[0462.0.0.25]: see [0462.0.0.0]**

**[0463.025.25]** SEQ ID NO: 28606 is amplified by PCR as described in the protocol of the Pfu Turbo or DNA Herculase polymerase (Stratagene).

**[0464.0.0.25]** to **[0466.0.0.25]: see [0464.0.0.0]** to **[0466.0.0.0]**

**[0467.025.25]** The following primer sequences were selected for the gene SEQ ID NO: 28606:
i) forward primer (SEQ ID NO: 28696,)
   atgaaacatc tgcatcgatt ctttag
ii) reverse primer (SEQ ID NO: 28697)
   ttaaactgat ggacgcaaac gaacg

**[0468.0.0.25] to [0479.0.0.25]: see [0468.0.0.0] to [0479.0.0.0]**

**[0480.025.25]:** Example 11: Generation of transgenic plants which express SEQ ID NO: 28606

### [0481.0.0.25] to [0513.0.0.25]: see [0481.0.0.0] to [0513.0.0.0]

**[0514.0.9.9]**: As an alternative, the carbohydrates, preferably polysaccharides, more preferably starch and/or cellulose and/or monosaccharides, more preferably fructose, glucose and/or myo-inositol and/or trisaccharides, more preferably raffinose and/or disaccharides, more preferably sucrose can be detected advantageously as for example described by Sonnebald et al., (Nat Biotechnol. 1997 Aug;15(8):794-7) ,or Panikulangara et al., Plant Physiol. 2004 Oct;136(2):3148-58.
The results of the different plant analyses can be seen from the table 1, which follows:

**Table 1**

| **ORF** | **Metabolite** | **analyte** | **Method** | **Min** | **Max** |
|---|---|---|---|---|---|
| b0019 | Sucrose | Sucrose | GC | 1.22 | 1.55 |
| b0138 | myo-Inositol | myo-Inositol | GC | 1.32 | 1.46 |
| b0161 | Fructose | Fructose | GC | 1.82 | 12.55 |
| b0161 | Glucose | Glucose | GC | 1.86 | 4.38 |
| b0161 | Raffinose | Raffinose | LC | 2.28 | 2.97 |
| b0252 | starch/cellulose | Anhydroglucose | GC | 1.37 | 1.53 |
| b0290 | myo-Inositol | myo-Inositol | GC | 1.23 | 1.50 |
| b0695 | Fructose | Fructose | GC | 1.69 | 11.46 |
| b0730 | Raffinose | Raffinose | LC | 1.93 | 7.16 |
| b1430 | starch/cellulose | Anhydroglucose | GC | 1.31 | 1.58 |
| b1693 | starch/cellulose | Anhydroglucose | GC | 1.34 | 2.16 |
| b1701 | Raffinose | Raffinose | LC | 1.57 | 10.58 |
| b1708 | Fructose | Fructose | GC | 1.77 | 27.64 |
| b1708 | Glucose | Glucose | GC | 1.62 | 10.42 |
| b1886 | Raffinose | Raffinose | LC | 1.64 | 4.91 |
| b1926 | Fructose | Fructose | GC | 1.86 | 2.93 |
| b1926 | Glucose | Glucose | GC | 1.63 | 1.88 |
| b2023 | myo-Inositol | myo-Inositol | GC | 1.31 | 2.10 |
| b2597 | Fructose | Fructose | GC | 1.90 | 2.05 |
| b2599 | Glucose | Glucose | GC | 1.65 | 2.71 |
| b2664 | Fructose | Fructose | GC | 1.77 | 41.86 |
| b2664 | Raffinose | Raffinose | LC | 1.72 | 13.67 |
| b2699 | myo-Inositol | myo-Inositol | GC | 1.86 | 7.90 |
| b2699 | Raffinose | Raffinose | LC | 1.61 | 25.08 |
| b3172 | myo-Inositol | myo-Inositol | GC | 1.26 | 2.44 |
| b3231 | starch/cellulose | Anhydroglucose | GC | 1.31 | 1.74 |
| b3430 | myo-Inositol | myo-Inositol | GC | 1.34 | 2.16 |
| b3601 | Raffinose | Raffinose | LC | 1.84 | 2.35 |
| b4129 | myo-Inositol | myo-Inositol | GC | 1.29 | 1.48 |
| b4239 | Fructose | Fructose | GC | 4.21 | 8.68 |
| b4239 | Glucose | Glucose | GC | 2.00 | 4.85 |
| b4239 | Sucrose | Sucrose | GC | 1.64 | 2.69 |
| b4327 | Fructose | Fructose | GC | 1.97 | 3.75 |
| YBR184W | Raffinose | Raffinose | LC | 1.87 | 5.78 |
| YBR204C | myo-Inositol | myo-Inositol | GC | 1.27 | 2.92 |
| YDR112W | myo-Inositol | myo-Inositol | GC | 1.41 | 1.90 |
| YER174C | starch/cellulose | Anhydroglucose | GC | 1.34 | 1.84 |
| YGR261C | myo-Inositol | myo-Inositol | GC | 2.24 | 7.98 |
| YGR261C | Raffinose | Raffinose | LC | 1.95 | 30.56 |
| YIL150C | myo-Inositol | myo-Inositol | GC | 4.80 | 4.80 |
| YJL072C | Glucose | Glucose | GC | 1.58 | 3.93 |
| YJL099W | myo-Inositol | myo-Inositol | GC | 1.26 | 5.72 |
| YOR044W | myo-Inositol | myo-Inositol | GC | 1.44 | 2.60 |
| YOR350C | myo-Inositol | myo-Inositol | GC | 1.52 | 2.82 |
| b0050 | starch and/or cellulose | Anhydroglucose | GC | 1.38 | 1.89 |
| b0124 | Fructose | Fructose | GC | 2.03 | 2.99 |
| b0149 | Fructose | Fructose | GC | 1.68 | 1.95 |
| b1318 | Fructose | Fructose | GC | 2.03 | 2.99 |
| b1463 | Fructose | Fructose | GC | 1.92 | 5.53 |
| b1463 | Glucose | Glucose | GC | 1.57 | 7.18 |
| b1463 | myo-Inositol | myo-Inositol | GC | 1.36 | 1.96 |
| b1539 | starch and/or cellulose | Anhydroglucose | GC | 1.39 | 1.92 |
| b1736 | Glucose | Glucose | GC | 1.61 | 2.10 |
| b1961 | myo-Inositol | myo-Inositol | GC | 1.25 | 1.58 |
| b2491 | Fructose | Fructose | GC | 2.23 | 3.78 |
| b2491 | Glucose | Glucose | GC | 1.81 | 3.89 |
| b3260 | Fructose | Fructose | GC | 1.87 | 5.72 |
| b3578 | Fructose | Fructose | GC | 1.89 | 2.06 |
| b3578 | Glucose | Glucose | GC | 1.65 | 2.10 |
| b3578 | Raffinose | Raffinose | LC | 1.55 | 1.84 |
| b3619 | Fructose | Fructose | GC | 1.61 | 2.11 |
| b3619 | Glucose | Glucose | GC | 1.60 | 2.19 |
| b3919 | starch and/or cellulose | Anhydroglucose | GC | 1.33 | 1.52 |
| b4074 | myo-Inositol | myo-Inositol | GC | 1.25 | 1.50 |
| b4122 | Fructose | Fructose | GC | 1.53 | 6.02 |
| b4232 | starch and/or cellulose | Anhydroglucose | GC | 1.35 | 1.61 |
| YHR072W-A | myo-Inositol | myo-Inositol | GC | 1.27 | 1.53 |

**[0515.0.0.25] to [0552.0.0.25]: see [0515.0.0.0] to [0552.0.0.0]**

**[0552.1.0.1]:** Example 15: Metabolite Profiling Info from *Zea mays*
*Zea mays* plants were engineered as described in Example 14c.
The results of the different *Zea mays* plants analysed can be seen from table 2 which follows:

**Table 2**

| ORF_NAME | Metabolite | Min | Max |
|---|---|---|---|
| b2699 | Raffinose | 2.06 | 5.58 |
| YIL150C | myo-Inositol | 2.03 | 2.91 |

Table 2 describes the increase in proline and glutamine in genetically modified corn plants expressing the Saccharomyces cerevisiae nucleic acid sequence YIL150c and/or E. coli protein b2699 resp..
In one embodiment, in case the activity of the Saccaromyces cerevisiae protein YIL150C or its homologs, e.g. "a chromatin binding protein, required for S-phase (DNA synthesis) initiation or completion" or its homologs, is increased in corn plants, preferably, an increase of the fine chemical myo-inositol between 103% and 191% is conferred.
In case the activity of the E. coli protein b2699 or a DNA strand exchange and recombination protein with protease and nuclease activity, preferably a gene product with an activity of recombination protein recA superfamily or its homolog, is increased in corn plants, preferably, an increase of the fine chemical raffinose between 106% and 458% is conferred.

**[0552.2.4.4]: see [0552.2.0.0]**

**[0553.0.25.25]**
1. A process for the production of carbohydrates, preferably polysaccharides, more preferably starch and/or cellulose and/or monosaccharides, more preferably fructose, glucose and/or myo-inositol and/or trisaccharides, more preferably raffinose and/or disaccharides, more preferably sucrose, which comprises
   (a) increasing or generating the activity of a protein as indicated in Table II, columns 5 or 7, lines 290 to 294 and/or 604 to 607 for starch and/or cellulose;
      lines 295 to 302 and/or 608 to 616 for fructose;
      lines 303 to 308 and/or 617 to 621 for glucose;
      lines 309 to 322 and/or 622 to 625 for myo-inositol;
      lines 323 to 331 and/or 626 for raffinose;
      lines 332 to 333 for sucrose;
      resp., or a functional equivalent thereof in a non-human organism, or in one or more parts thereof; and
   (b) growing the organism under conditions which permit the production of carbohydrates, preferably polysaccharides, more preferably starch and/or cellulose and/or monosaccharides, more preferably fructose, glucose and/or myo-inositol and/or trisaccharides, more preferably raffinose and/or disaccharides, more preferably sucrose resp. in said organism.
2. A process for the production of carbohydrates, preferably polysaccharides, more preferably starch and/or cellulose and/or monosaccharides, more preferably fructose, glucose and/or myo-inositol and/or trisaccharides, more preferably raffinose and/or disaccharides, more preferably sucrose , comprising the increasing or generating in an organism or a part thereof the expression of at least one nucleic acid molecule comprising a nucleic acid molecule selected from the group consisting of:
   a) nucleic acid molecule encoding of a polypeptide as indicated in Table II, columns ' 5 or 7, lines 290 to 294 and/or 604 to 607 for starch and/or cellulose;
      lines 295 to 302 and/or 608 to 616 for fructose;
      lines 303 to 308 and/or 617 to 621 for glucose;
      lines 309 to 322 and/or 622 to 625 for myo-inositol;
      lines 323 to 331 and/or 626 for raffinose;
      lines 332 to 333 for sucrose;
      resp., or a fragment thereof, which confers an increase in the amount of carbohydrates, preferably polysaccharides, more preferably starch and/or cellulose and/or monosaccharides, more preferably fructose, glucose and/or myo-inositol and/or trisaccharides, more preferably raffinose and/or disaccharides, more preferably sucrosein an organism or a part thereof;
   b) nucleic acid molecule comprising of a nucleic acid molecule as indicated in Table I, columns 5 or 7, lines 290 to 294 and/or 604 to 607 for starch and/or cellulose;
      lines 295 to 302 and/or 608 to 616 for fructose;
      lines 303 to 308 and/or 617 to 621 for glucose;
      lines 309 to 322 and/or 622 to 625 for myo-inositol;
      lines 323 to 331 and/or 626 for raffinose;
      lines 332 to 333 for sucrose resp.;
   c) nucleic acid molecule whose sequence can be deduced from a polypeptide sequence encoded by a nucleic acid molecule of (a) or (b) as a result of the degeneracy of the genetic code and conferring an increase in the amount of carbohydrates, preferably polysaccharides, more preferably starch and/or cellulose and/or monosaccharides, more preferably fructose, glucose and/or myo-inositol and/or trisaccharides, more preferably raffinose and/or disaccharides, more preferably sucrose in an organism or a part thereof;
   d) nucleic acid molecule which encodes a polypeptide which has at least 50% identity with the amino acid sequence of the polypeptide encoded by the nucleic acid molecule of (a) to (c) and conferring an increase in the amount of carbohydrates, preferably polysaccharides, more preferably starch and/or cellulose and/or monosaccharides, more preferably fructose, glucose and/or myo-inositol and/or trisaccharides, more preferably raffinose and/or disaccharides, more preferably sucrose in an organism or a part thereof;
   e) nucleic acid molecule which hybidizes with a nucleic acid molecule of (a) to (c) under under stringent hybridisation conditions and conferring an increase in the amount of carbohydrates, preferably polysaccharides, more preferably starch and/or cellulose and/or monosaccharides, more preferably fructose, glucose and/or myo-inositol and/or trisaccharides, more preferably raffinose and/or disaccharides, more preferably sucrose in an organism or a part thereof;
   f) nucleic acid molecule which encompasses a nucleic acid molecule which is obtained by amplifying nucleic acid molecules from a cDNA library or a genomic library using the primers or primer pairs as indicated in Table III, column 7, lines 290 to 294 and/or 604 to 607 for starch and/or cellulose;
      lines 295 to 302 and/or 608 to 616 for fructose;
      lines 303 to 308 and/or 617 to 621 for glucose;
      lines 309 to 322 and/or 622 to 625 for myo-inositol;
      lines 323 to 331 and/or 626 for raffinose;
      lines 332 to 333 for sucrose resp., and conferring an increase in the amount of carbohydrates, preferably polysaccharides, more preferably starch and/or cellulose and/or monosaccharides, more preferably fructose, glucose and/or myo-inositol and/or trisaccharides, more preferably raffinose and/or disaccharides, more preferably sucrosein an organism or a part thereof;
   g) nucleic acid molecule encoding a polypeptide which is isolated with the aid of monoclonal antibodies against a polypeptide encoded by one of the nucleic acid molecules of (a) to (f) and conferring an increase in the amount of vin an organism or a part thereof;
   h) nucleic acid molecule encoding a polypeptide comprising a consensus as indicated in Table IV, column 7, lines 290 to 294 and/or 604 to 607 for starch and/or cellulose;
      lines 295 to 302 and/or 608 to 616 for fructose;
      lines 303 to 308 and/or 617 to 621 for glucose;
      lines 309 to 322 and/or 622 to 625 for myo-inositol;
      lines 323 to 331 and/or 626 for raffinose;
      lines 332 to 333 for sucroseresp., and conferring an increase in the amount of carbohydrates, preferably polysaccharides, more preferably starch and/or cellulose and/or monosaccharides, more preferably fructose, glucose and/or myo-inositol and/or trisaccharides, more preferably raffinose and/or disaccharides, more preferably sucrose in an organism or a part thereof; and
   i) nucleic acid molecule which is obtainable by screening a suitable nucleic acid library under stringent hybridization conditions with a probe comprising one of the sequences of the nucleic acid molecule of (a) to (k) or with a fragment thereof having at least 15 nt, preferably 20 nt, 30 nt, 50 nt, 100 nt, 200 nt or 500 nt of the nucleic acid molecule characterized in (a) to (k) and conferring an increase in the amount of the respective fine chemical in an organism or a part thereof.
   or comprising a sequence which is complementary thereto.
3. The process of claim 1 or 2, wherein the carbohydrates, preferably polysaccharides, more preferably starch and/or cellulose and/or monosaccharides, more preferably fructose, glucose and/or myo-inositol and/or trisaccharides, more preferably raffinose and/or disaccharides, more preferably sucrose is isolated.
4. The process of any one of claim 1 to 3, comprising the following steps:
   (a) selecting an organism or a part thereof expressing a polypeptide encoded by the nucleic acid molecule characterized in claim 2;
   (b) mutagenizing the selected organism or the part thereof;
   (c) comparing the activity or the expression level of said polypeptide in the mutagenized organism or the part thereof with the activity or the expression of said polypeptide of the selected organisms or the part thereof;
   (d) selecting the mutated organisms or parts thereof, which comprise an increased activity or expression level of said polypeptide compared to the selected organism or the part thereof;
   (e) optionally, growing and cultivating the organisms or the parts thereof; and
   (f) recovering, and optionally isolating, the free or bound carbohydrates, preferably polysaccharides, more preferably starch and/or cellulose and/or monosaccharides, more preferably fructose, glucose and/or myo-inositol and/or trisaccharides, more preferably raffinose and/or disaccharides, more preferably sucrose produced by the selected mutated organisms or parts thereof.
5. The process of any one of claims 1 to 4, wherein the activity of said protein or the expression of said nucleic acid molecule is increased or generated transiently or stably.
6. An isolated nucleic acid molecule comprising a nucleic acid molecule selected from the group consisting of:
   a) nucleic acid molecule encoding of a polypeptide as indicated in Table II, columns 5 or 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp., or a fragment thereof, which confers an increase in the amount of carbohydrates, preferably polysaccharides, more preferably starch and/or cellulose and/or monosaccharides, more preferably fructose, glucose and/or myo-inositol and/or trisaccharides, more preferably raffinose and/or disaccharides, more preferably sucrosein an organism or a part thereof;
   b) nucleic acid molecule comprising of a nucleic acid molecule as indicated in Table I, columns 5 or 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp.;
   c) nucleic acid molecule whose sequence can be deduced from a polypeptide sequence encoded by a nucleic acid molecule of (a) or (b) as a result of the degeneracy of the genetic code and conferring an increase in the amount of carbohydrates, preferably polysaccharides, more preferably starch and/or cellulose and/or monosaccharides, more preferably fructose, glucose and/or myo-inositol and/or trisaccharides, more preferably raffinose and/or disaccharides, more preferably sucrose in an organism or a part thereof;
   d) nucleic acid molecule which encodes a polypeptide which has at least 50% identity with the amino acid sequence of the polypeptide encoded by the nucleic acid molecule of (a) to (c) and conferring an increase in the amount of carbohydrates, preferably polysaccharides, more preferably starch and/or cellulose and/or monosaccharides, more preferably fructose, glucose and/or myo-inositol and/or trisaccharides, more preferably raffinose and/or disaccharides, more preferably sucrose in an organism or a part thereof;
   e) nucleic acid molecule which hybidizes with a nucleic acid molecule of (a) to (c) under stringent hybridisation conditions and conferring an increase in the amount of carbohydrates, preferably polysaccharides, more preferably starch and/or cellulose and/or monosaccharides, more preferably fructose, glucose and/or myo-inositol and/or trisaccharides, more preferably raffinose and/or disaccharides, more preferably sucrose in an organism or a part thereof;
   f) nucleic acid molecule which encompasses a nucleic acid molecule which is obtained by amplifying nucleic acid molecules from a cDNA library or a genomic library using the primers or primer pairs as indicated in Table III, column 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp., and conferring an increase in the amount of carbohydrates, preferably polysaccharides, more preferably starch and/or cellulose and/or monosaccharides, more preferably fructose, glucose and/or myo-inositol and/or trisaccharides, more preferably raffinose and/or disaccharides, more preferably sucrose in an organism or a part thereof;
   g) nucleic acid molecule encoding a polypeptide which is isolated with the aid of monoclonal antibodies against a polypeptide encoded by one of the nucleic acid molecules of (a) to (f) and conferring an increase in the amount of carbohydrates, preferably polysaccharides, more preferably starch and/or cellulose and/or monosaccharides, more preferably fructose, glucose and/or myo-inositol and/or trisaccharides, more preferably raffinose and/or disaccharides, more preferably sucrose in an organism or a part thereof;
   h) nucleic acid molecule encoding a polypeptide comprising a consensus as indicated in Table IV, column 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp., and conferring an increase in the amount of carbohydrates, preferably polysaccharides, more preferably starch and/or cellulose and/or monosaccharides, more preferably fructose, glucose and/or myo-inositol and/or trisaccharides, more preferably raffinose and/or disaccharides, more preferably sucrose in an organism or a part thereof; and
   i) nucleic acid molecule which is obtainable by screening a suitable nucleic acid library under stringent hybridization conditions with a probe comprising one of the sequences of the nucleic acid molecule of (a) to (k) or with a fragment thereof having at least 15 nt, preferably 20 nt, 30 nt, 50 nt, 100 nt, 200 nt or 500 nt of the nucleic acid molecule characterized in (a) to (k) and conferring an increase in the amount of carbohydrates, preferably polysaccharides, more preferably starch and/or cellulose and/or monosaccharides, more preferably fructose, glucose and/or myo-inositol and/or trisaccharides, more preferably raffinose and/or disaccharides, more preferably sucrosein an organism or a part thereof.
   whereby the nucleic acid molecule distinguishes over the sequence as indicated in Table I A, columns 5 or 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp., by one or more nucleotides.
7. A nucleic acid construct which confers the expression of the nucleic acid molecule of claim 6, comprising one or more regulatory elements.
8. A vector comprising the nucleic acid molecule as claimed in claim 6 or the nucleic acid construct of claim 7.
9. The vector as claimed in claim 8, wherein the nucleic acid molecule is in operable linkage with regulatory sequences for the expression in a prokaryotic or eukaryotic, or in a prokaryotic and eukaryotic, host.
10. A host cell, which has been transformed stably or transiently with the vector as claimed in claim 8 or 9 or the nucleic acid molecule as claimed in claim 6 or the nucleic acid construct of claim 7 or produced as described in claim any one of claims 2 to 5. ,
11. The host cell of claim 10, which is a transgenic host cell.
12. The host cell of claim 10 or 11, which is a plant cell, an animal cell, a microorganism, or a yeast cell, a fungus cell, a prokaryotic cell, an eukaryotic cell or an archaebacterium.
13. A process for producing a polypeptide, wherein the polypeptide is expressed in a host cell as claimed in any one of claims 10 to 12.
14. A polypeptide produced by the process as claimed in claim 13 or encoded by the nucleic acid molecule as claimed in claim 6 whereby the polypeptide distinguishes over a sequence as indicated in Table II A, columns 5 or 7, lines 290 to 294 and/or 604 to 607; lines 295 to 302 and/or 608 to 616; lines 303 to 308 and/or 617 to 621; lines 309 to 322 and/or 622 to 625; lines 323 to 331 and/or 626; lines 332 to 333, resp., by one or more amino acids
15. An antibody, which binds specifically to the polypeptide as claimed in claim 14.
16. A plant tissue, propagation material, harvested material or a plant comprising the host cell as claimed in claim 12 which is plant cell or an Agrobacterium.
17. A method for screening for agonists and antagonists of the activity of a polypeptide encoded by the nucleic acid molecule of claim 6 conferring an increase in the amount of carbohydrates, preferably polysaccharides, more preferably starch and/or cellulose and/or monosaccharides, more preferably fructose, glucose and/or myo-inositol and/or trisaccharides, more preferably raffinose and/or disaccharides, more preferably sucrose in an organism or a part thereof comprising:
   (a) contacting cells, tissues , plants or microorganisms which express the a polypeptide encoded by the nucleic acid molecule of claim 6 conferring an increase in the amount of carbohydrates, preferably polysaccharides, more preferably starch and/or cellulose and/or monosaccharides, more preferably fructose, glucose and/or myo-inositol and/or trisaccharides, more preferably raffinose and/or disaccharides, more preferably sucrose in an organism or a part thereof with a candidate compound or a sample comprising a plurality of compounds under conditions which permit the expression the polypeptide;
   (b) assaying the carbohydrates, preferably polysaccharides, more preferably starch and/or cellulose and/or monosaccharides, more preferably fructose, glucose and/or myo-inositol and/or trisaccharides, more preferably raffinose and/or disaccharides, more preferably sucroselevel or the polypeptide expression level in the cell, tissue, plant or microorganism or the media the cell, tissue, plant or microorganisms is cultured or maintained in; and
   (c) identifying a agonist or antagonist by comparing the measured carbohydrates, preferably polysaccharides, more preferably starch and/or cellulose and/or monosaccharides, more preferably fructose, glucose and/or myo-inositol and/or trisaccharides, more preferably raffinose and/or disaccharides, more preferably sucrose level or polypeptide expression level with a standard carbohydrates, preferably polysaccharides, more preferably starch and/or cellulose and/or monosaccharides, more preferably fructose, glucose and/or myo-inositol and/or trisaccharides, more preferably raffinose and/or disaccharides, more preferably sucroseor polypeptide expression level measured in the absence of said candidate compound or a sample comprising said plurality of compounds, whereby an increased level over the standard indicates that the compound or the sample comprising said plurality of compounds is an agonist and a decreased level over the standard indicates that the compound or the sample comprising said plurality of compounds is an antagonist.
18. A process for the identification of a compound conferring increased carbohydrates, preferably polysaccharides, more preferably starch and/or cellulose and/or monosaccharides, more preferably fructose, glucose and/or myo-inositol and/or trisaccharides, more preferably raffinose and/or disaccharides, more preferably sucrose production in a plant or microorganism, comprising the steps:
   (a) culturing a plant cell or tissue or microorganism or maintaining a plant expressing the polypeptide encoded by the nucleic acid molecule of claim 6 conferring an increase in the amount of carbohydrates, preferably polysaccharides, more preferably starch and/or cellulose and/or monosaccharides, more preferably fructose, glucose and/or myo-inositol and/or trisaccharides, more preferably raffinose and/or disaccharides, more preferably sucrose in an organism or a part thereof and a readout system capable of interacting with the polypeptide under suitable conditions which permit the interaction of the polypeptide with said readout system in the presence of a compound or a sample comprising a plurality of compounds and capable of providing a detectable signal in response to the binding of a compound to said polypeptide under conditions which permit the expression of said readout system and of the polypeptide encoded by the nucleic acid molecule of claim 6 conferring an increase in the amount of carbohydrates, preferably polysaccharides, more preferably starch and/or cellulose and/or monosaccharides, more preferably fructose, glucose and/or myo-inositol and/or trisaccharides, more preferably raffinose and/or disaccharides, more preferably sucrose in an organism or a part thereof;
   (b) identifying if the compound is an effective agonist by detecting the presence or absence or increase of a signal produced by said readout system.
19. A method for the identification of a gene product conferring an increase in carbohydrates, preferably polysaccharides, more preferably starch and/or cellulose and/or monosaccharides, more preferably fructose, glucose and/or myo-inositol and/or trisaccharides, more preferably raffinose and/or disaccharides, more preferably sucroseproduction in a cell, comprising the following steps:
   (a) contacting the nucleic acid molecules of a sample, which can contain a candidate gene encoding a gene product conferring an increase in carbohydrates, preferably polysaccharides, more preferably starch and/or cellulose and/or monosaccharides, more preferably fructose, glucose and/or myo-inositol and/or trisaccharides, more preferably raffinose and/or disaccharides, more preferably sucrose after expression with the nucleic acid molecule of claim 6;
   (b) identifying the nucleic acid molecules, which hybridise under relaxed stringent conditions with the nucleic acid molecule of claim 6;
   (c) introducing the candidate nucleic acid molecules in host cells appropriate for producing carbohydrates, preferably polysaccharides, more preferably starch and/or cellulose and/or monosaccharides, more preferably fructose, glucose and/or myo-inositol and/or trisaccharides, more preferably raffinose and/or disaccharides, more preferably sucrose;
   (d) expressing the identified nucleic acid molecules in the host cells;
   (e) assaying the carbohydrates, preferably polysaccharides, more preferably starch and/or cellulose and/or monosaccharides, more preferably fructose, glucose and/or myo-inositol and/or trisaccharides, more preferably raffinose and/or disaccharides, more preferably sucroselevel in the host cells; and
   (f) identifying nucleic acid molecule and its gene product which expression confers an increase in the carbohydrates, preferably polysaccharides, more preferably starch and/or cellulose and/or monosaccharides, more preferably fructose, glucose and/or myo-inositol and/or trisaccharides, more preferably raffinose and/or disaccharides, more preferably sucroselevel in the host cell in the host cell after expression compared to the wild type.
20. A method for the identification of a gene product conferring an increase in carbohydrates, preferably polysaccharides, more preferably starch and/or cellulose and/or monosaccharides, more preferably fructose, glucose and/or myo-inositol and/or trisaccharides, more preferably raffinose and/or disaccharides, more preferably sucrose production in a cell, comprising the following steps:
   (a) identifiying in a data bank nucleic acid molecules of an organism; which can contain a candidate gene encoding a gene product conferring an increase in the carbohydrates, preferably polysaccharides, more preferably starch and/or cellulose and/or monosaccharides, more preferably fructose, glucose and/or myo-inositol and/or trisaccharides, more preferably raffinose and/or disaccharides, more preferably sucrose amount or level in an organism or a part thereof after expression, and which are at least 20% homologous to the nucleic acid molecule of claim 6;
   (b) introducing the candidate nucleic acid molecules in host cells appropriate for producing carbohydrates, preferably polysaccharides, more preferably starch and/or cellulose and/or monosaccharides, more preferably fructose, glucose and/or myo-inositol and/or trisaccharides, more preferably raffinose and/or disaccharides, more preferably sucrose;
   (c) expressing the identified nucleic acid molecules in the host cells;
   (d) assaying the carbohydrates, preferably polysaccharides, more preferably starch and/or cellulose and/or monosaccharides, more preferably fructose, glucose and/or myo-inositol and/or trisaccharides, more preferably raffinose and/or disaccharides, more preferably sucrose level in the host cells; and
   (e) identifying nucleic acid molecule and its gene product which expression confers an increase in the carbohydrates, preferably polysaccharides, more preferably starch and/or cellulose and/or monosaccharides, more preferably fructose, glucose and/or myo-inositol and/or trisaccharides, more preferably raffinose and/or disaccharides, more preferably sucroselevel in the host cell after expression compared to the wild type.
21. A method for the production of an agricultural composition comprising the steps of the method of any one of claims 17 to 20 and formulating the compound identified in any one of claims 17 to 20 in a form acceptable for an application in agriculture.
22. A composition comprising the nucleic acid molecule of claim 6, the polypeptide of claim 14, the nucleic acid construct of claim 7, the vector of any one of claims 8 or 9, an antagonist or agonist identified according to claim 17, the compound of claim 18, the gene product of claim 19 or 20, the antibody of claim 15, and optionally an agricultural acceptable carrier.
23. Use of the nucleic acid molecule as claimed in claim 6 for the identification of a nucleic acid molecule conferring an increase of carbohydrates, preferably polysaccharides, more preferably starch and/or cellulose and/or monosaccharides, more preferably fructose, glucose and/or myo-inositol and/or trisaccharides, more preferably raffinose and/or disaccharides, more preferably sucrose after expression.
24. Use of the polypeptide of claim 14 or the nucleic acid construct claim 7 or the gene product identified according to the method of claim 19 or 20 for identifying compounds capable of conferring a modulation of carbohydrates, preferably polysaccharides, more preferably starch and/or cellulose and/or monosaccharides, more preferably fructose, glucose and/or myo-inositol and/or trisaccharides, more preferably raffinose and/or disaccharides, more preferably sucrose levels in an organism.
25. Cosmetic, pharmaceutical, food or feed composition comprising the nucleic acid molecule of claim 6, the polypeptide of claim 14, the nucleic acid construct of claim 7, the vector of claim 8 or 9, the antagonist or agonist identified according to claim 17, the antibody of claim 15, the plant or plant tissue of claim 16, the harvested material of claim 16, the host cell of claim 10 to 12 or the gene product identified according to the method of claim 19 or 20.
26. Use of the nucleic acid molecule of claim 6, the polypeptide of claim 14, the nucleic acid construct of claim 7, the vector of claim 8 or 9, the antagonist or agonist identified according to claim 17, the antibody of claim 15, the plant or plant tissue of claim 16, the host cell of claim 10 to 12 or the gene product identified according to the method of claim 19 or 20 for the protection of a plant against a carbohydrates, preferably polysaccharides, more preferably starch and/or cellulose and/or monosaccharides, more preferably fructose, glucose and/or myo-inositol and/or trisaccharides, more preferably raffinose and/or disaccharides, more preferably sucrose synthesis inhibiting herbicide.

**[0554.0.0.25] Abstract: see [0554.0.0.0]**

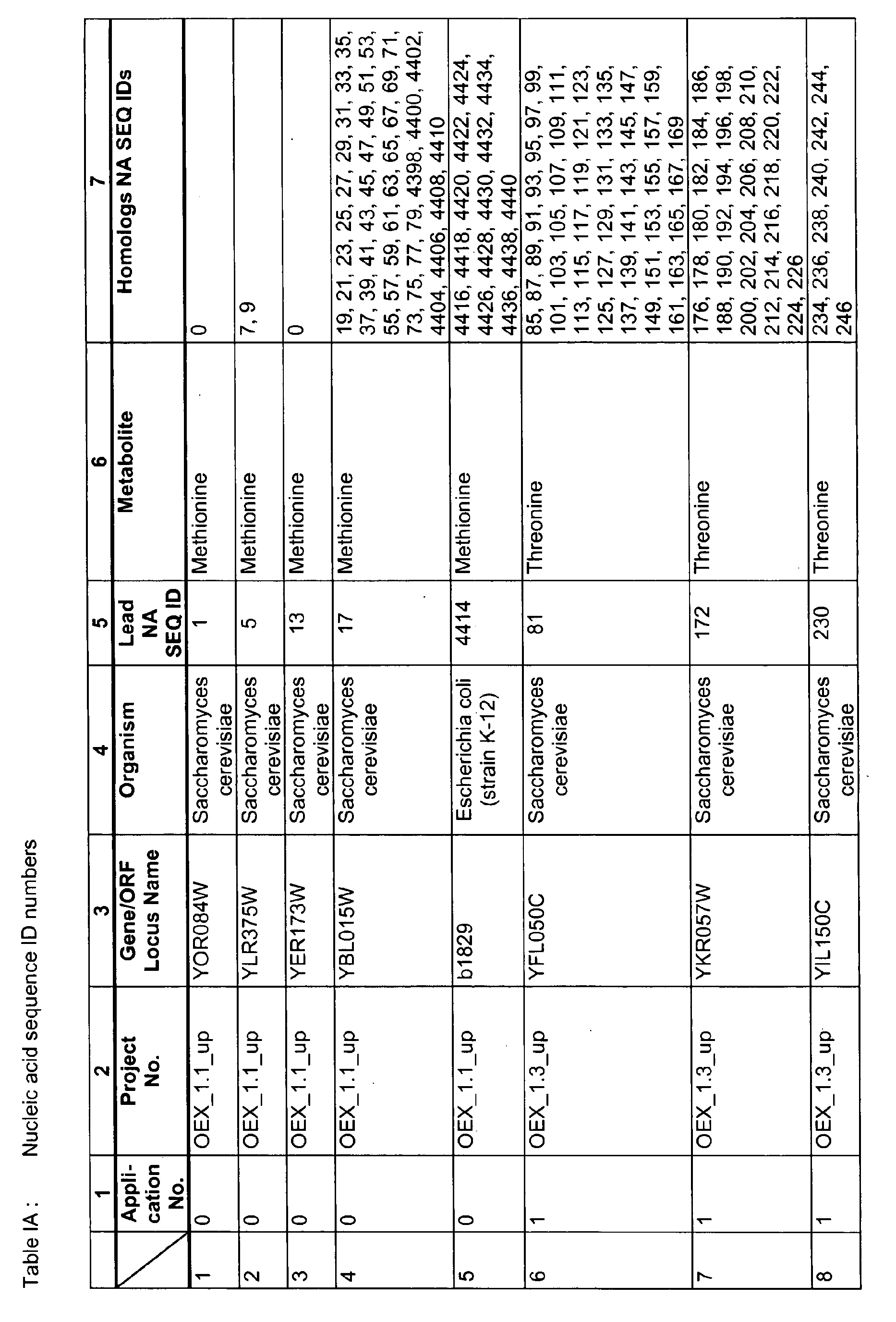

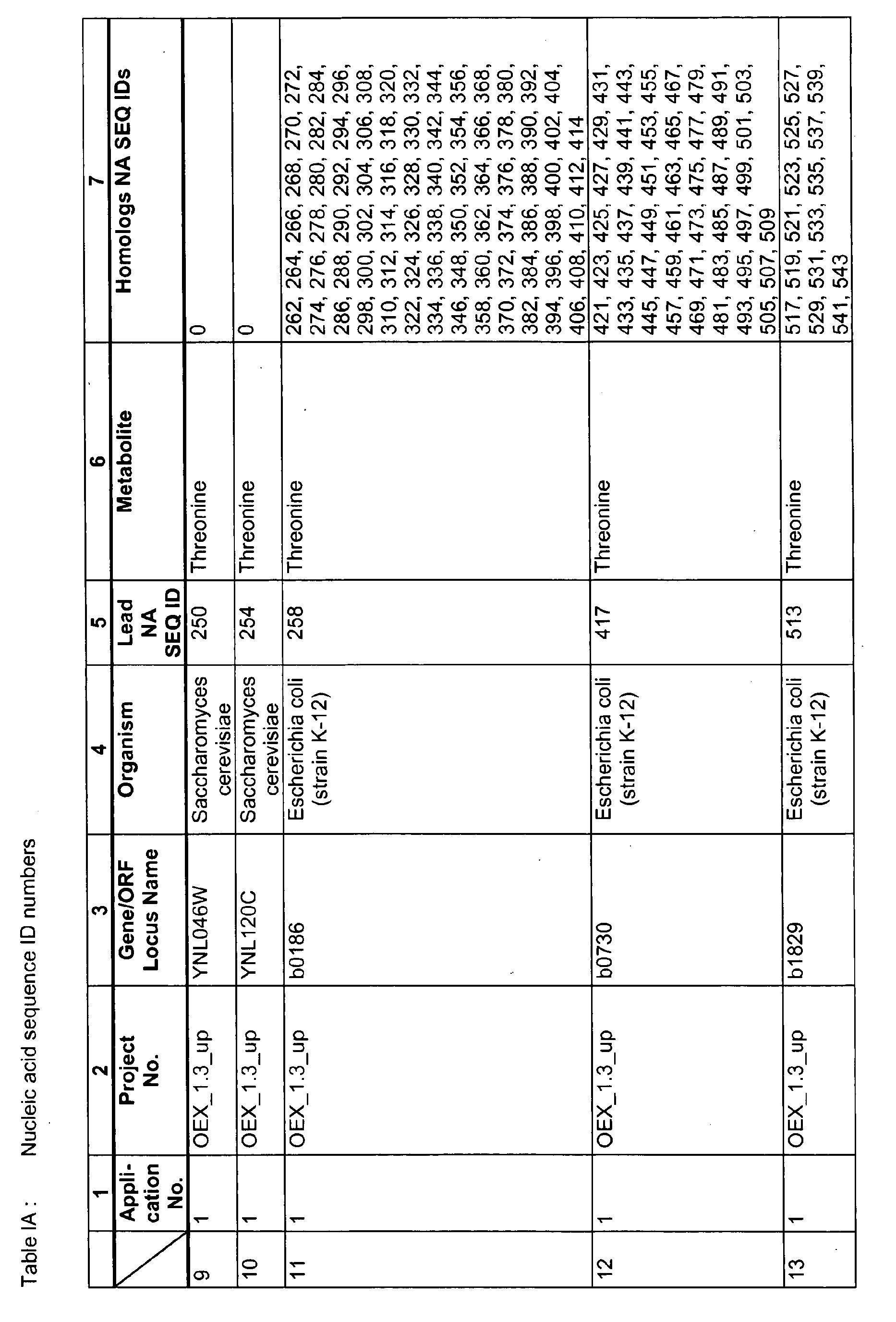

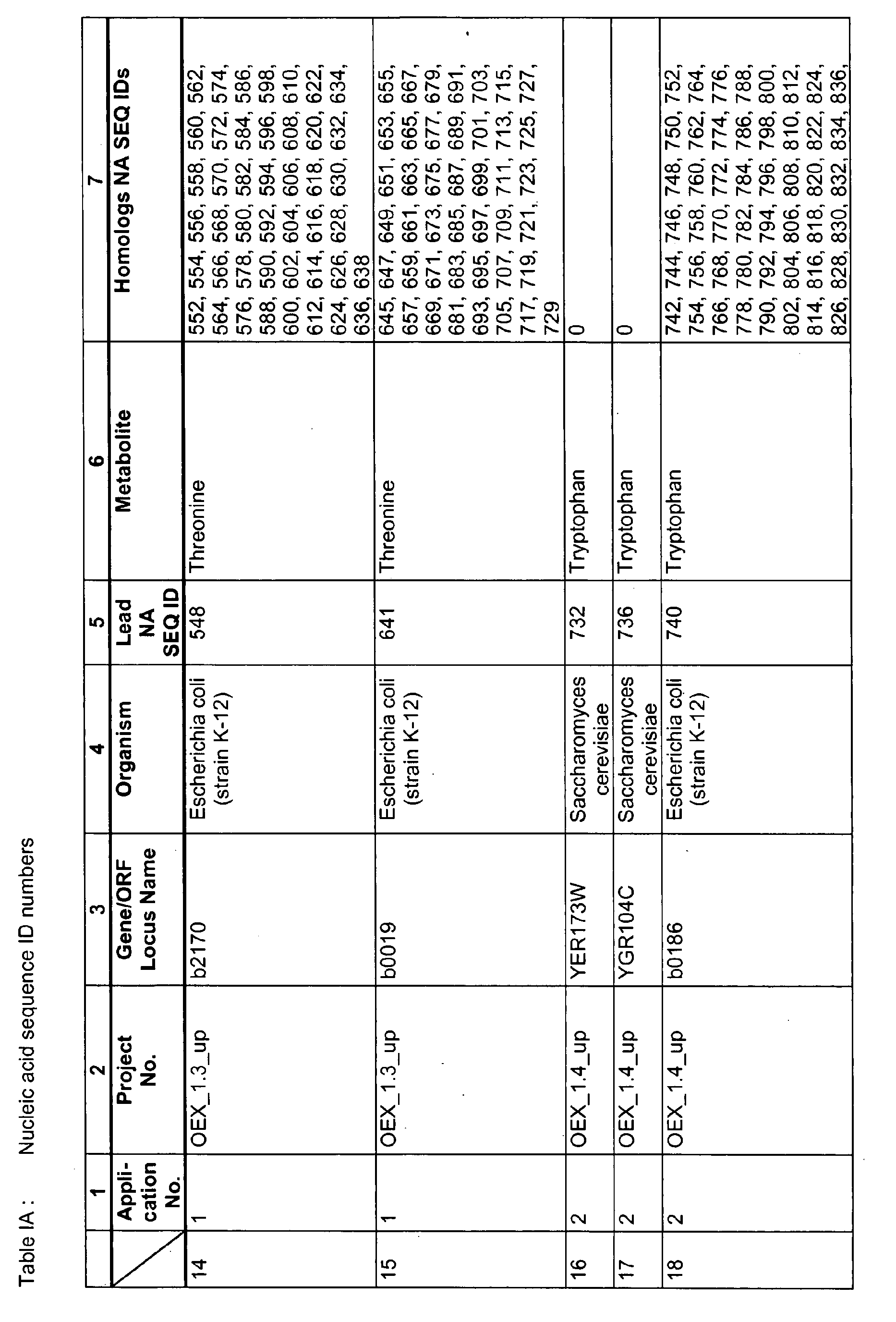

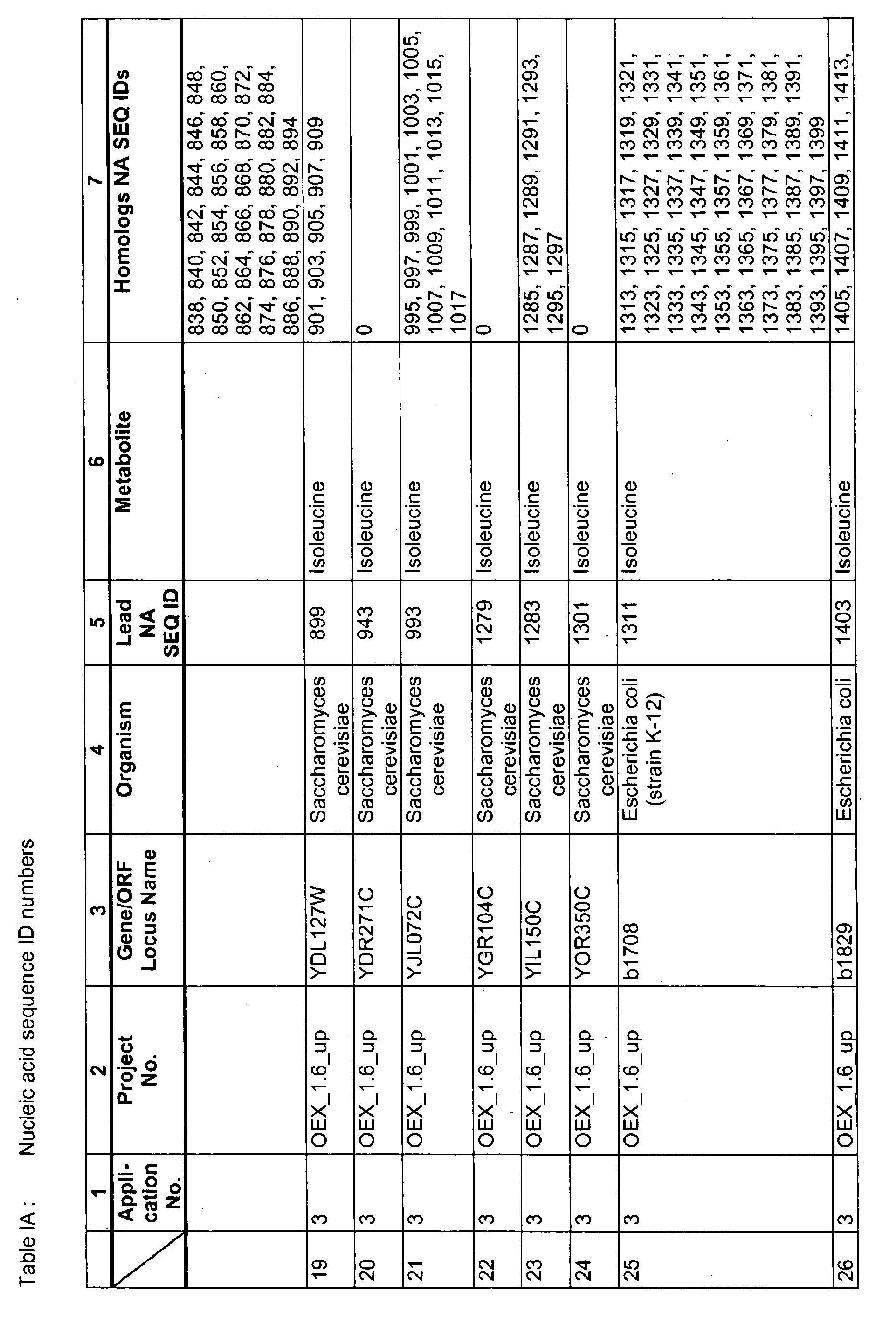

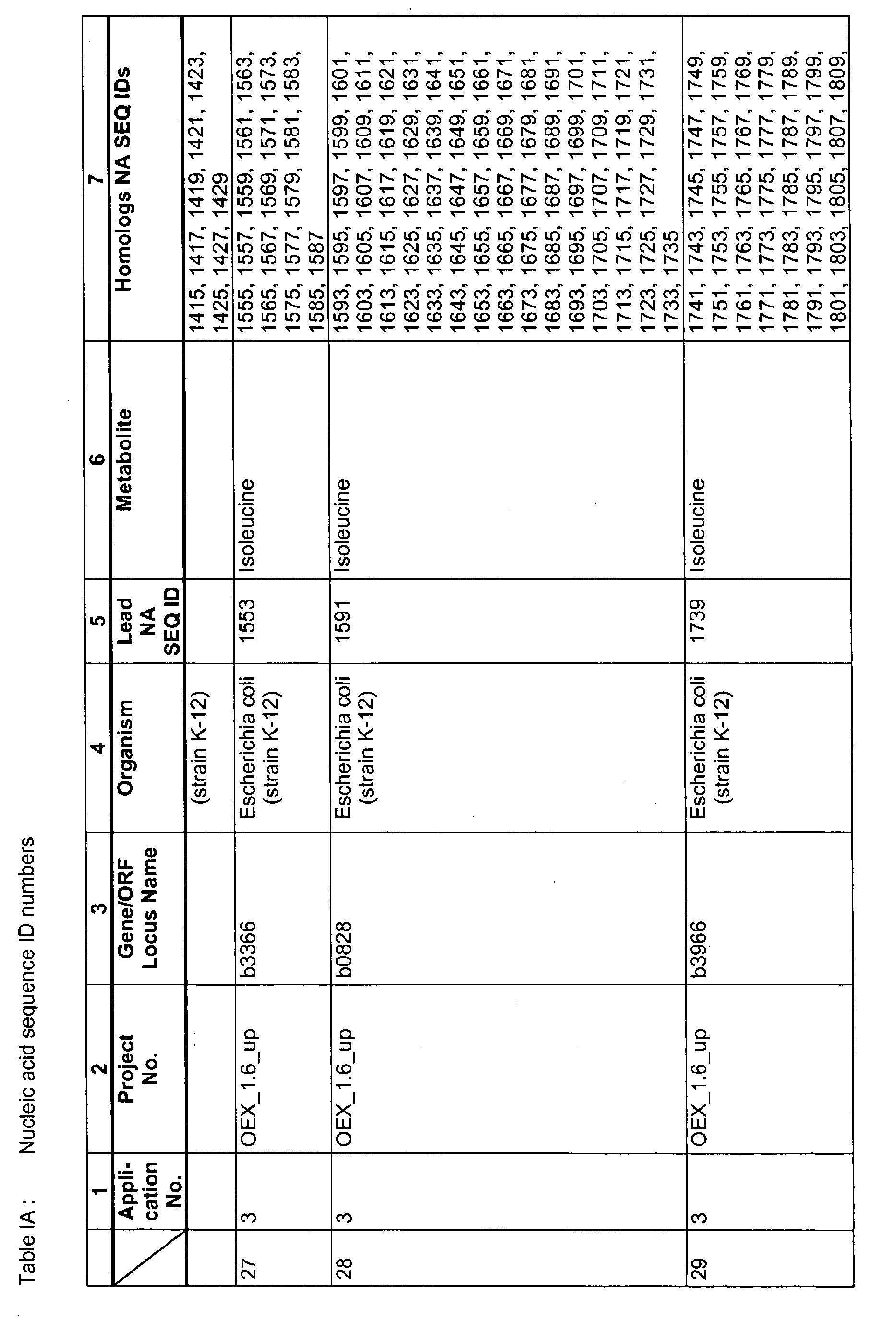

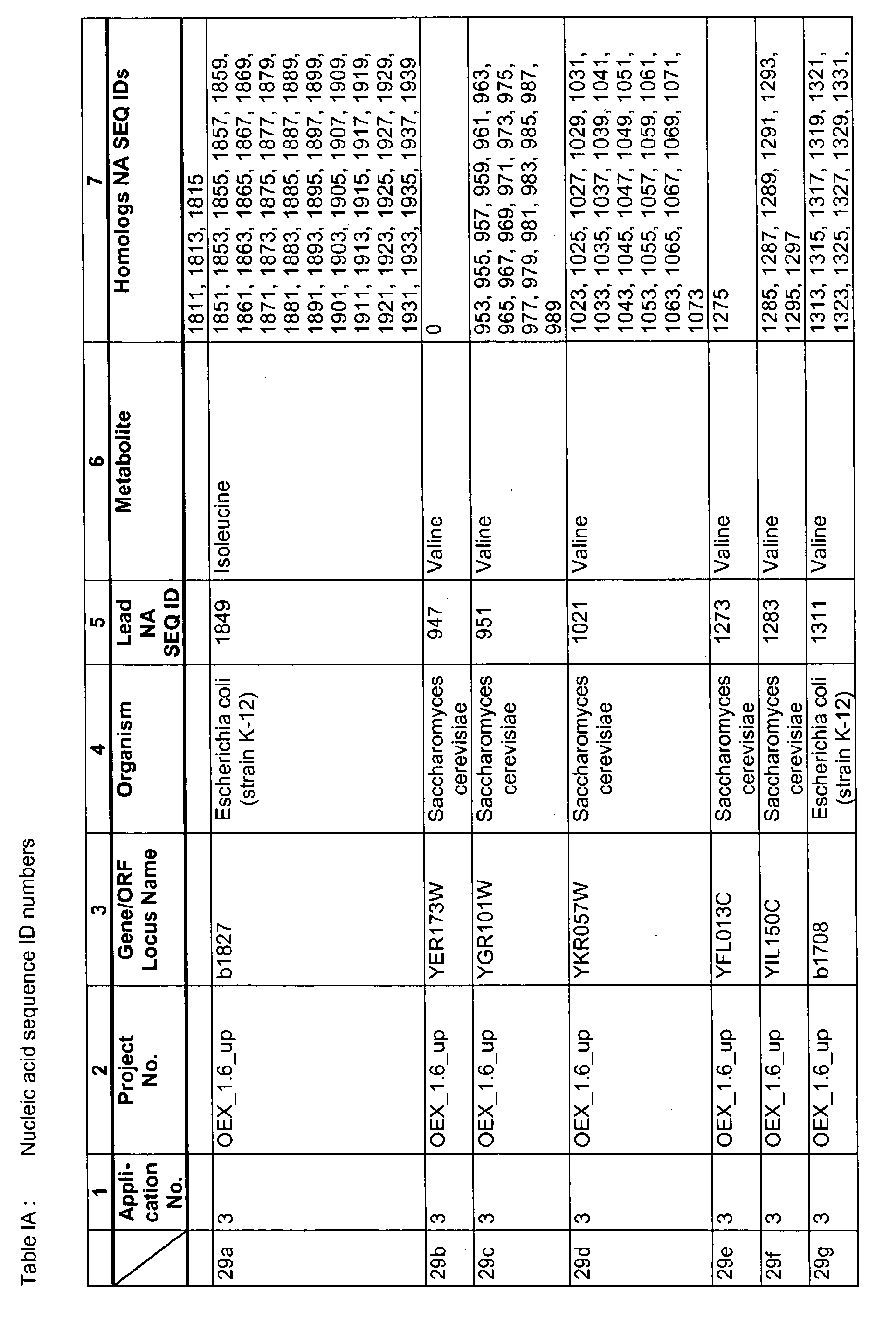

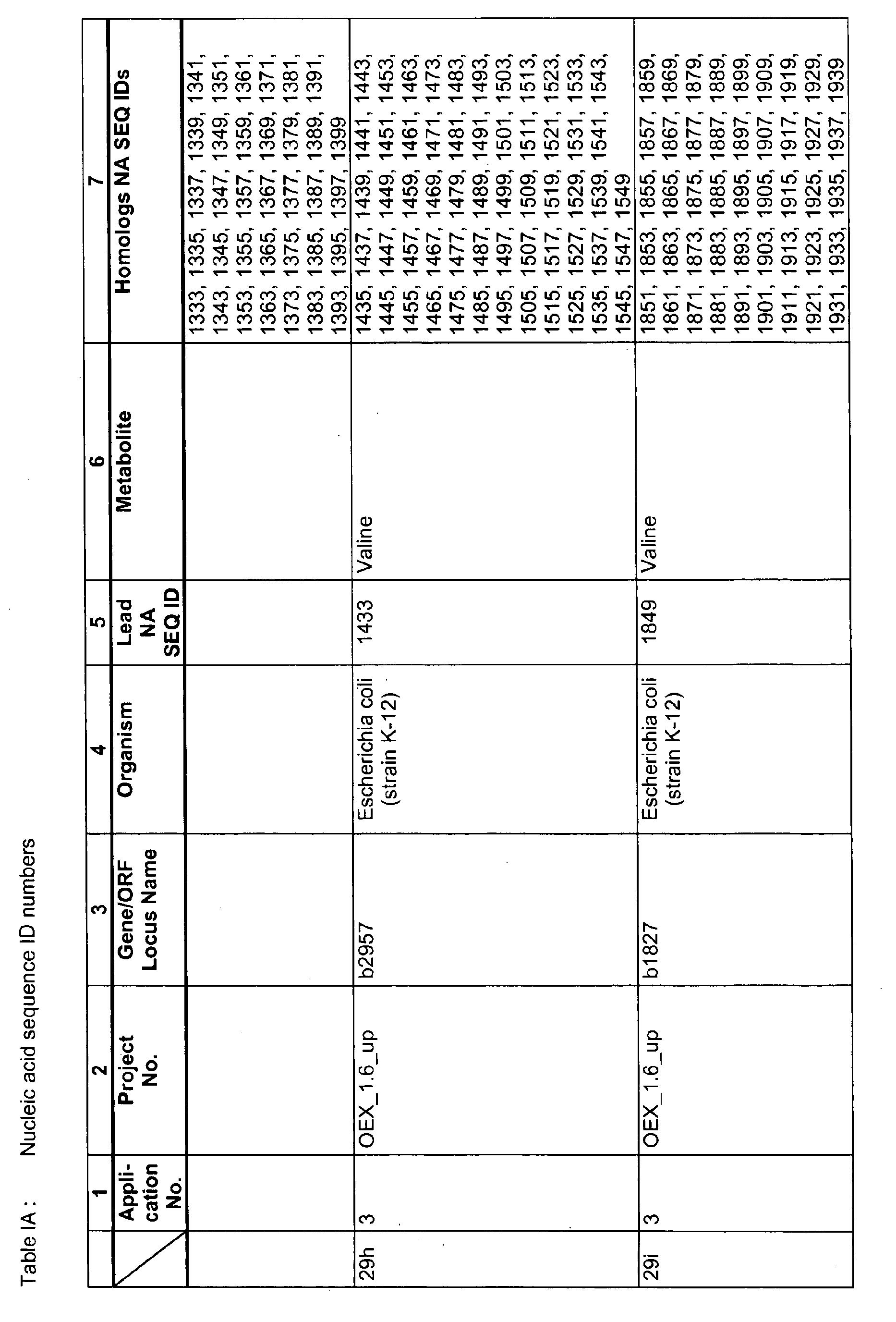

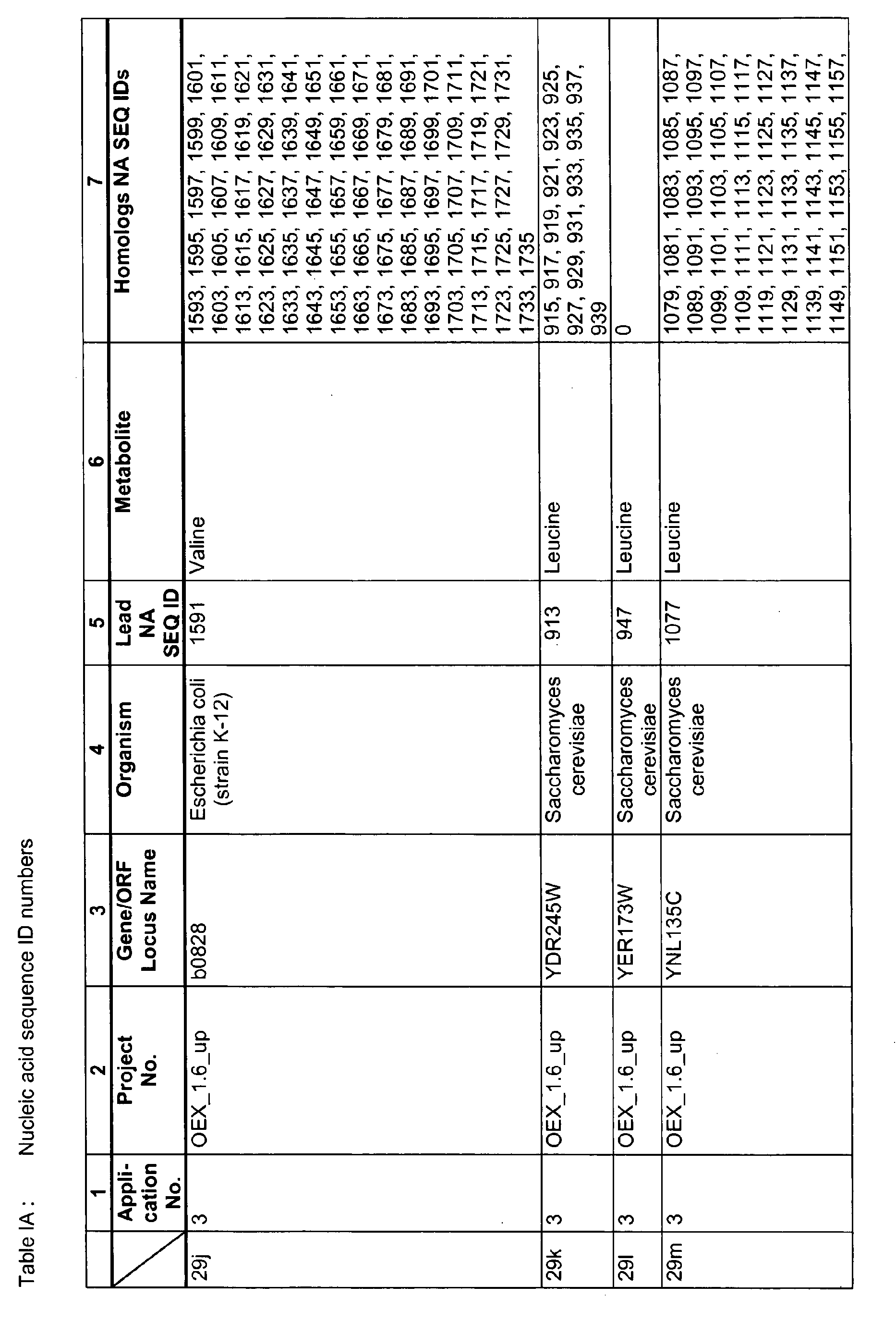

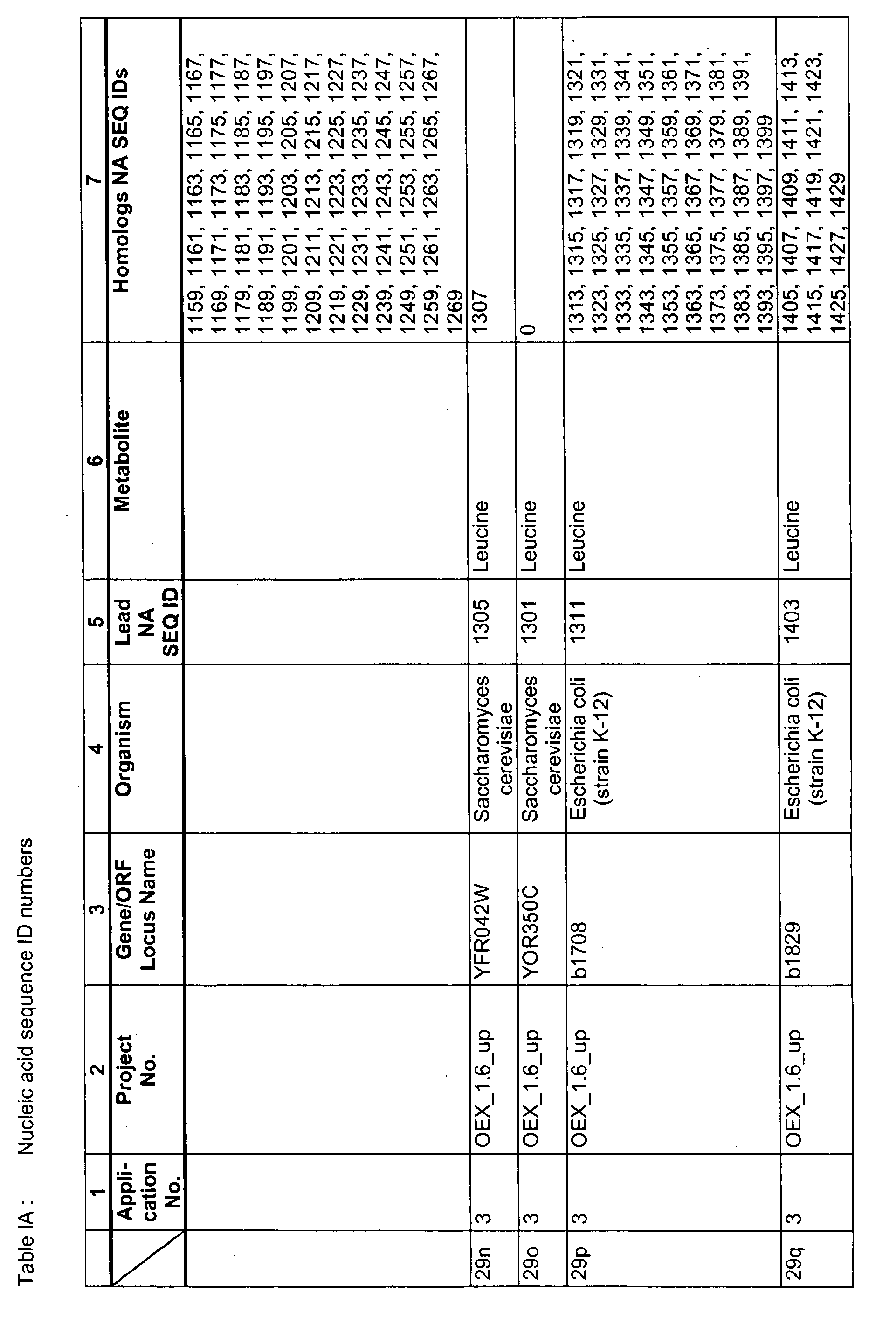

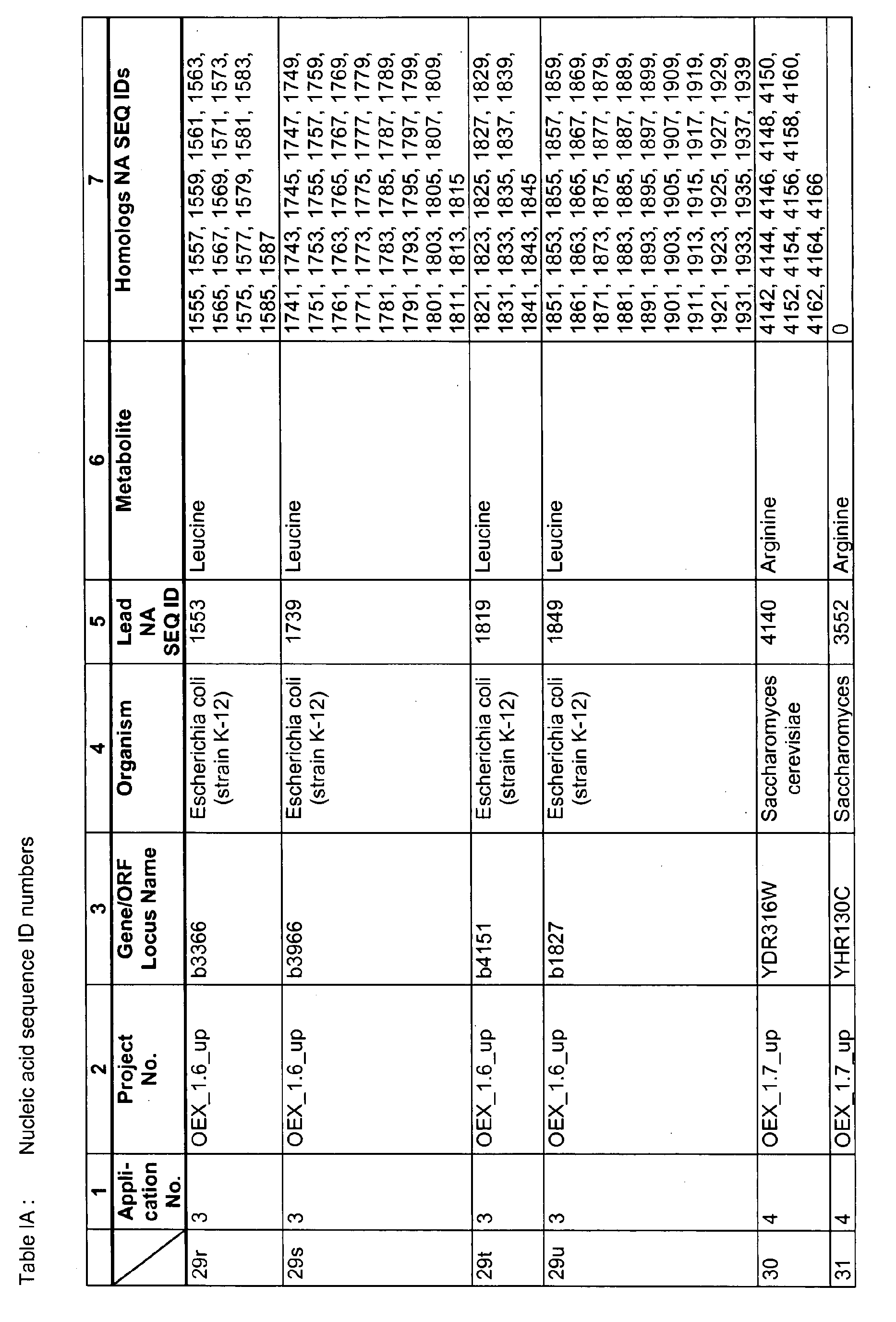

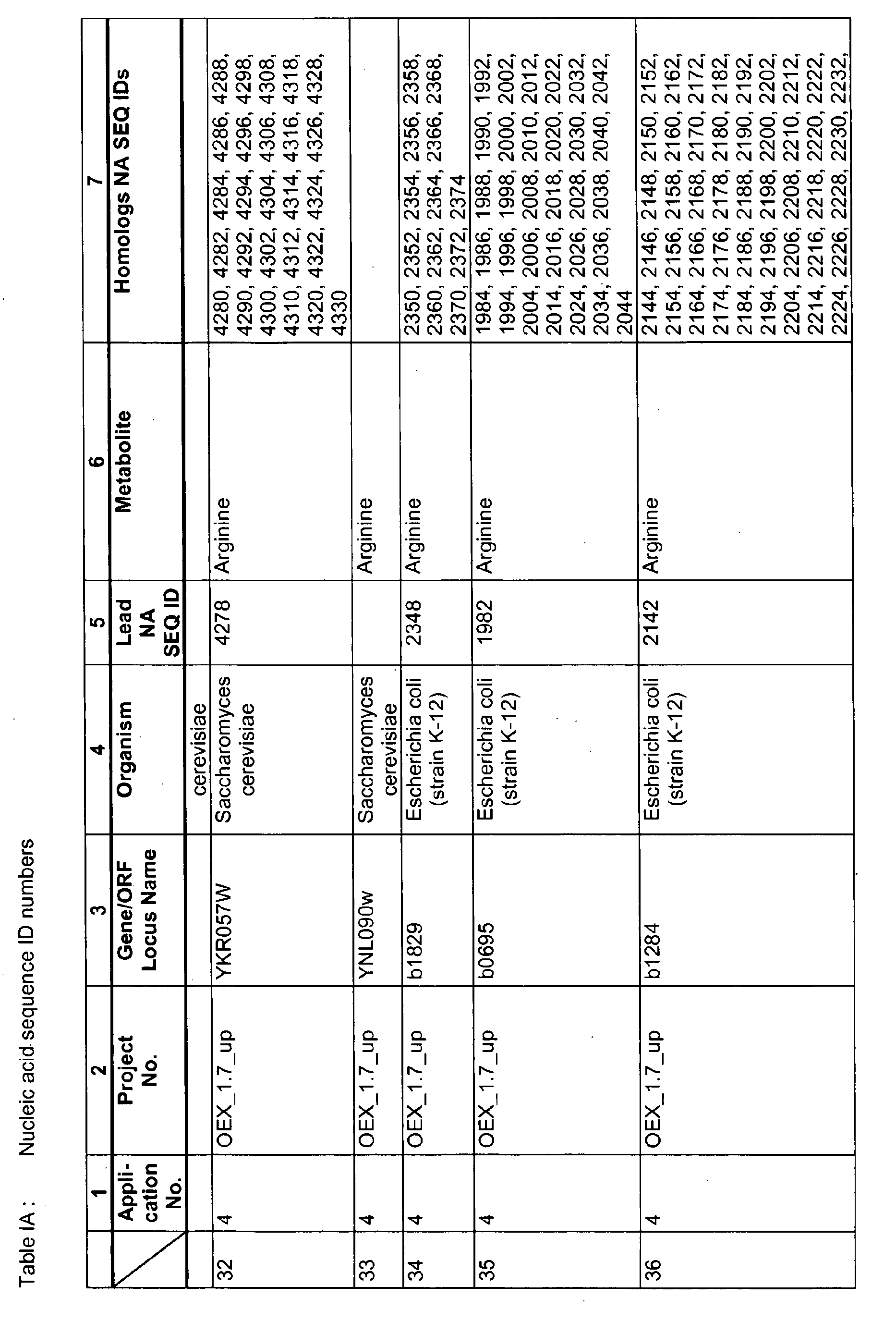

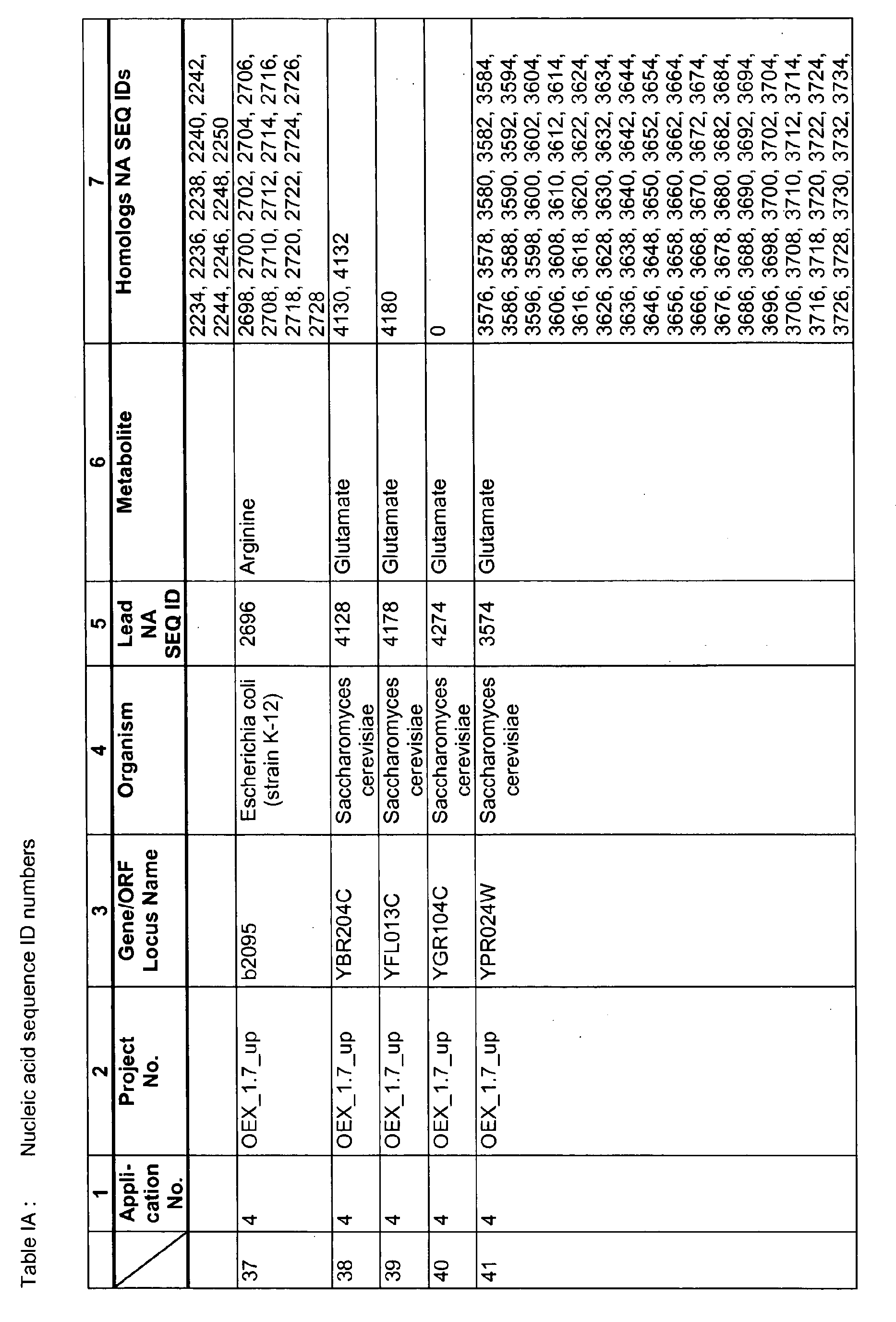

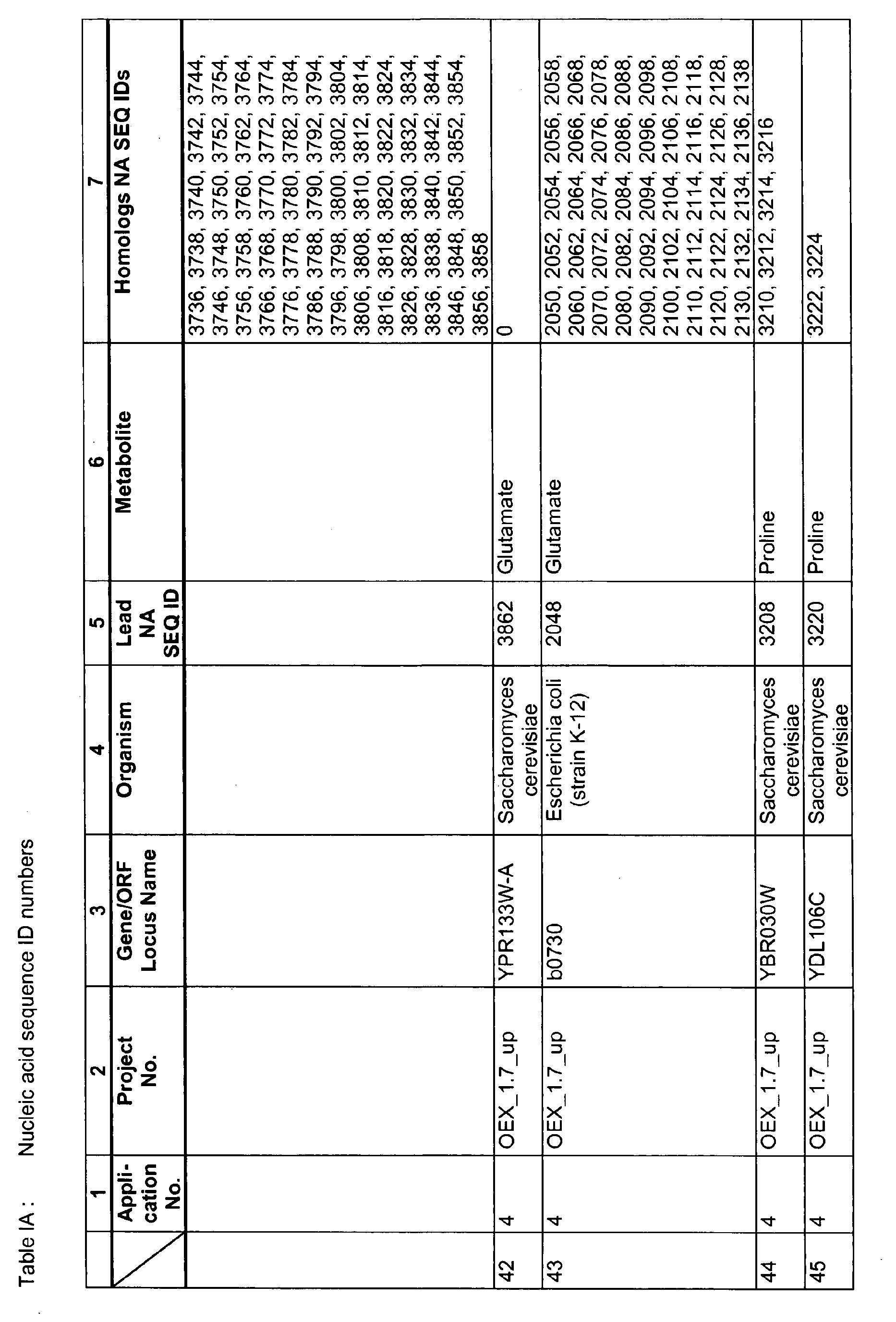

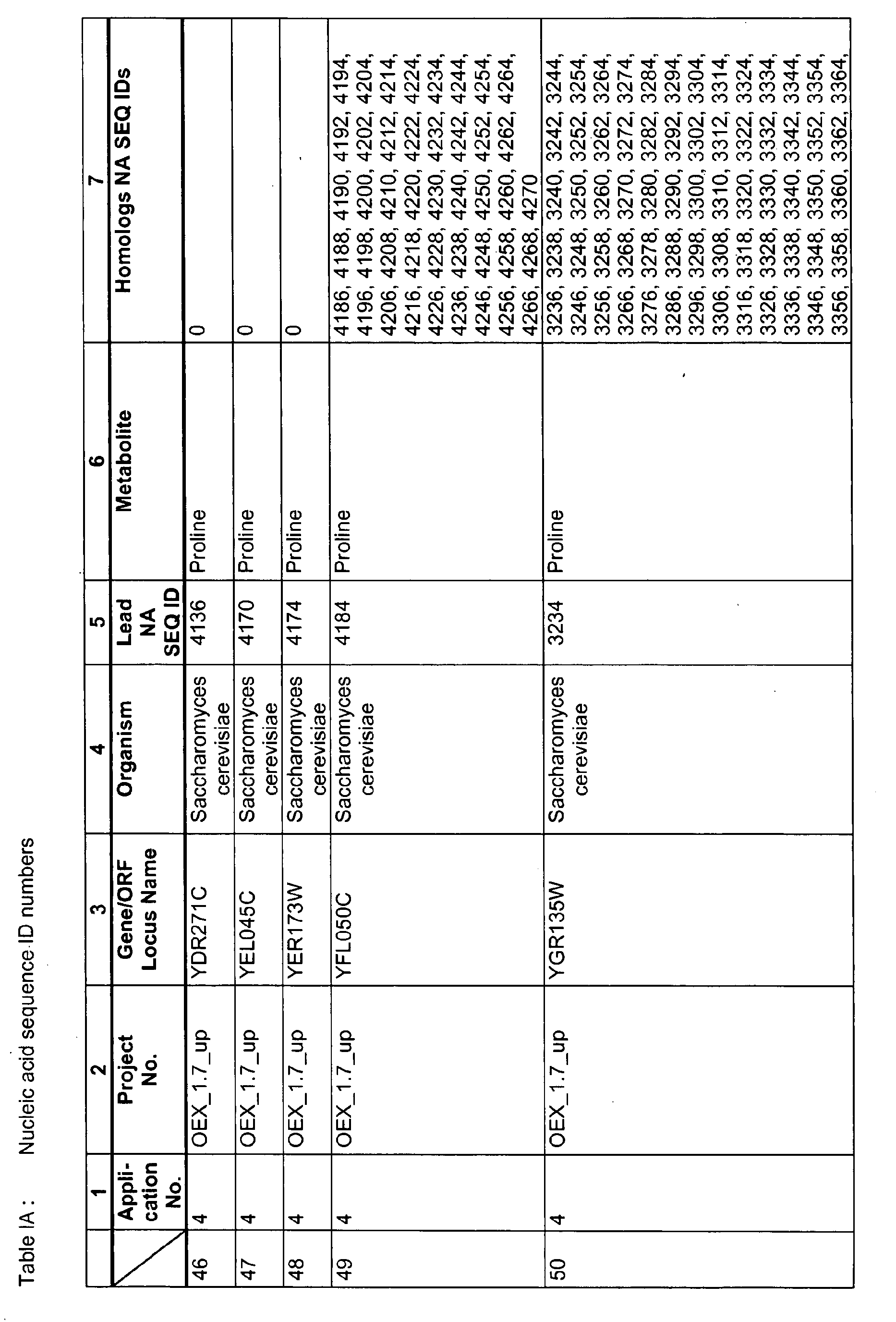

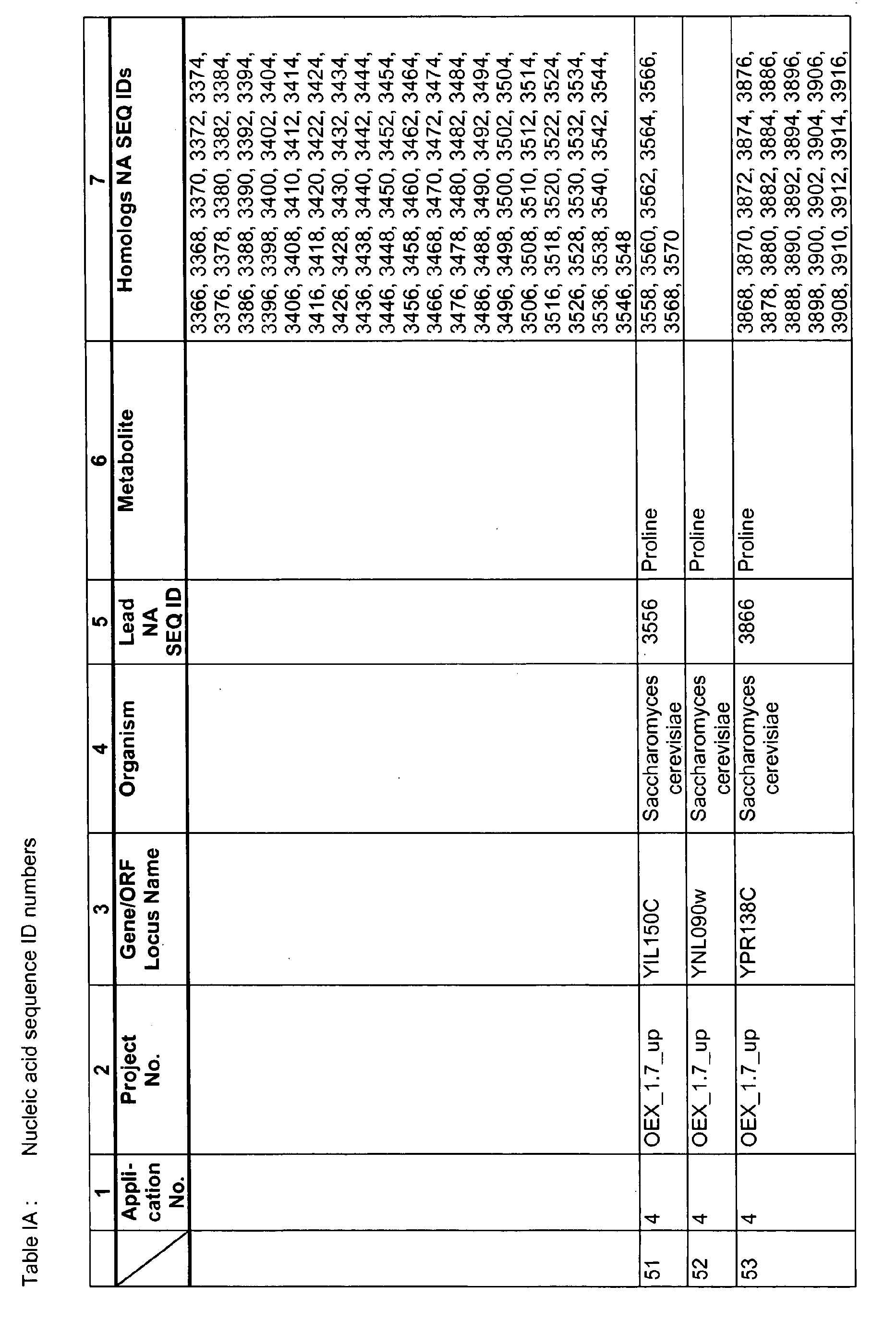

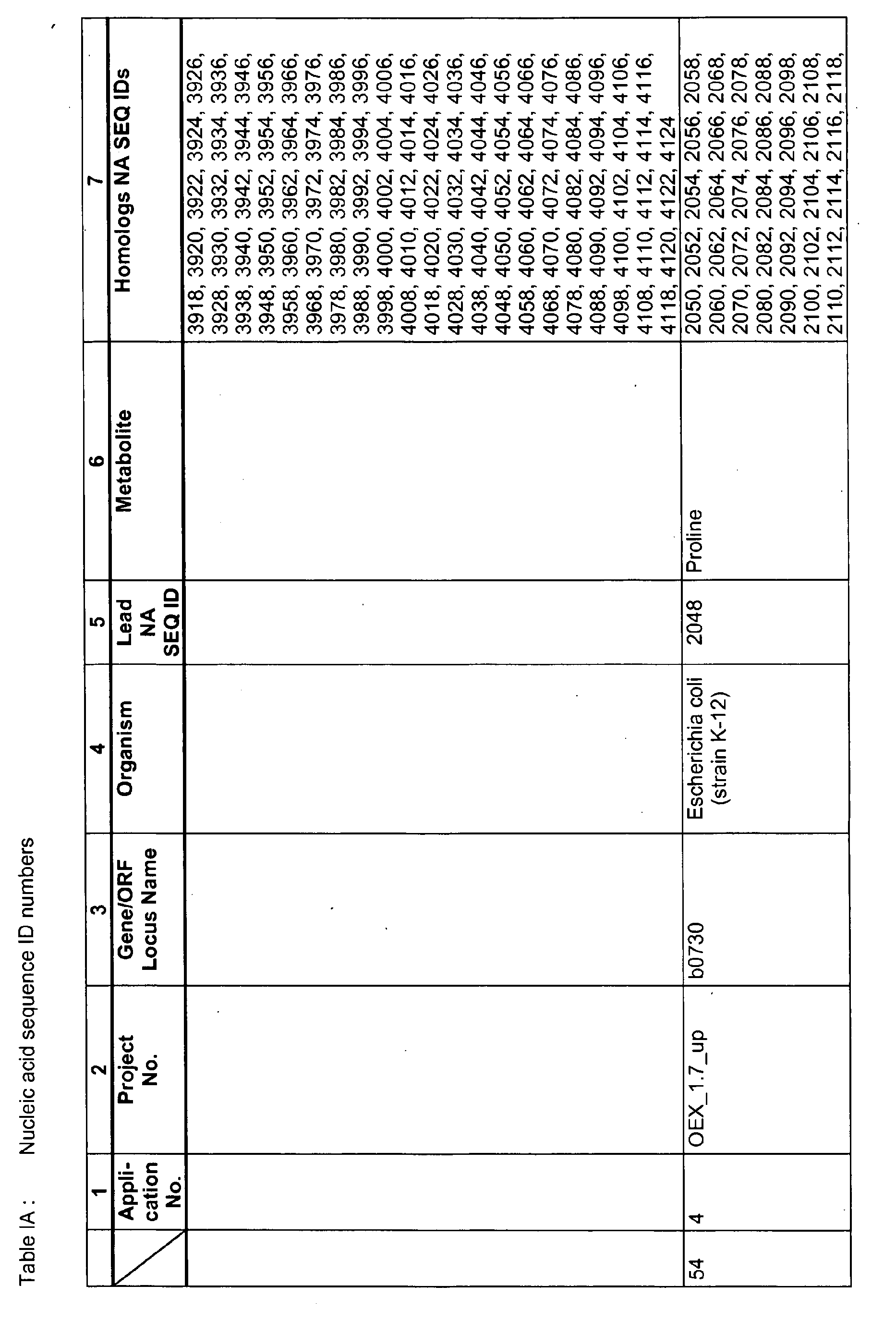

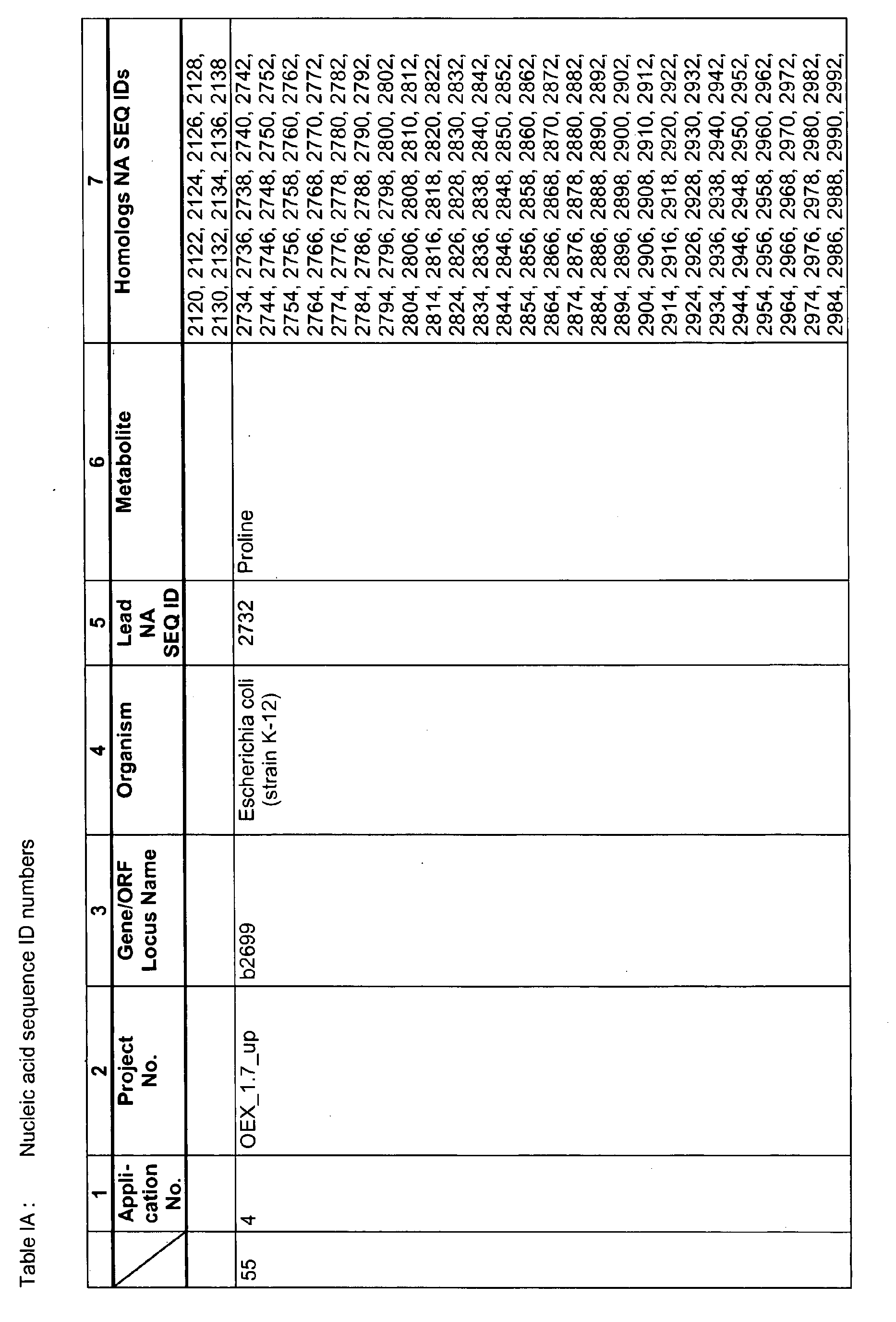

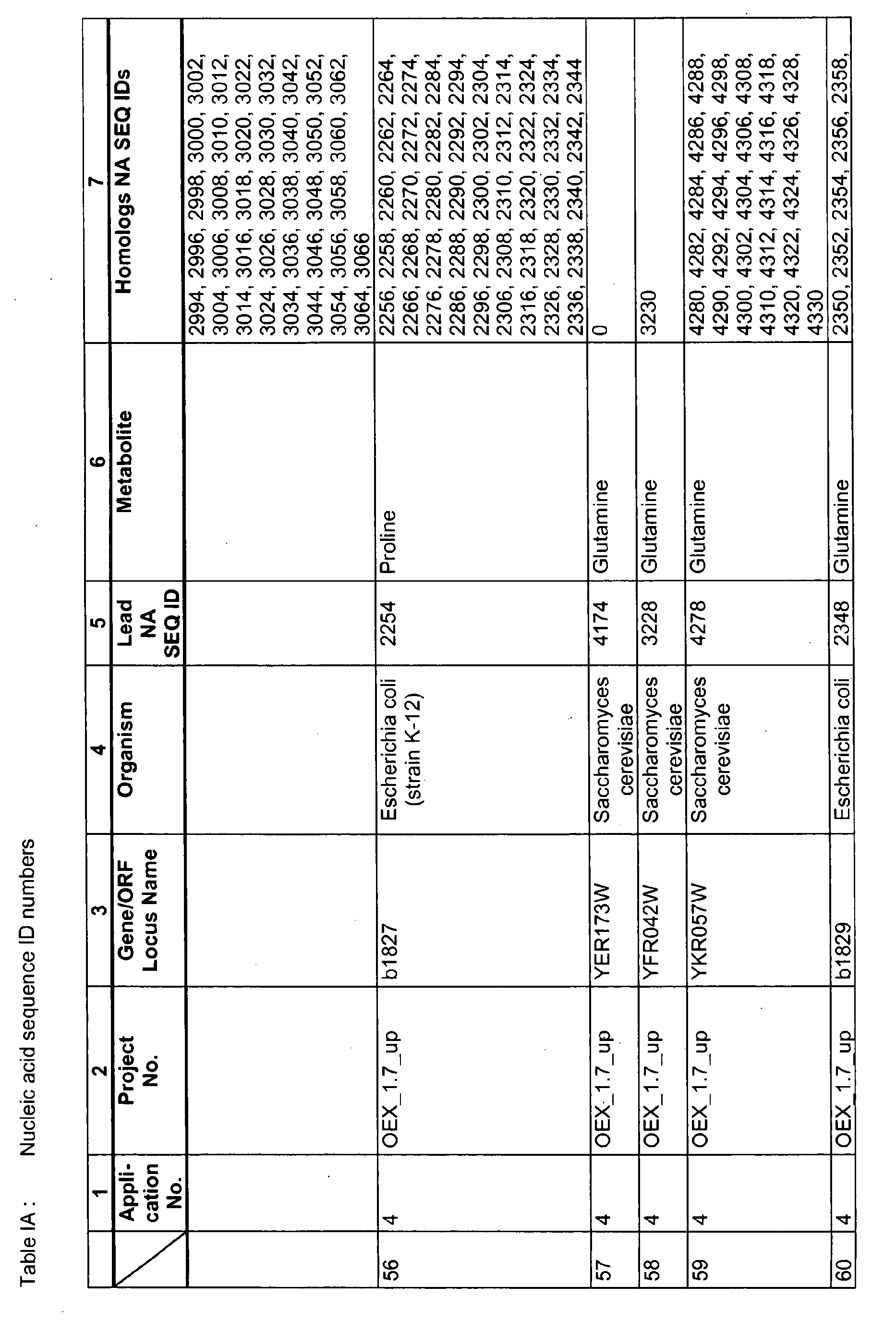

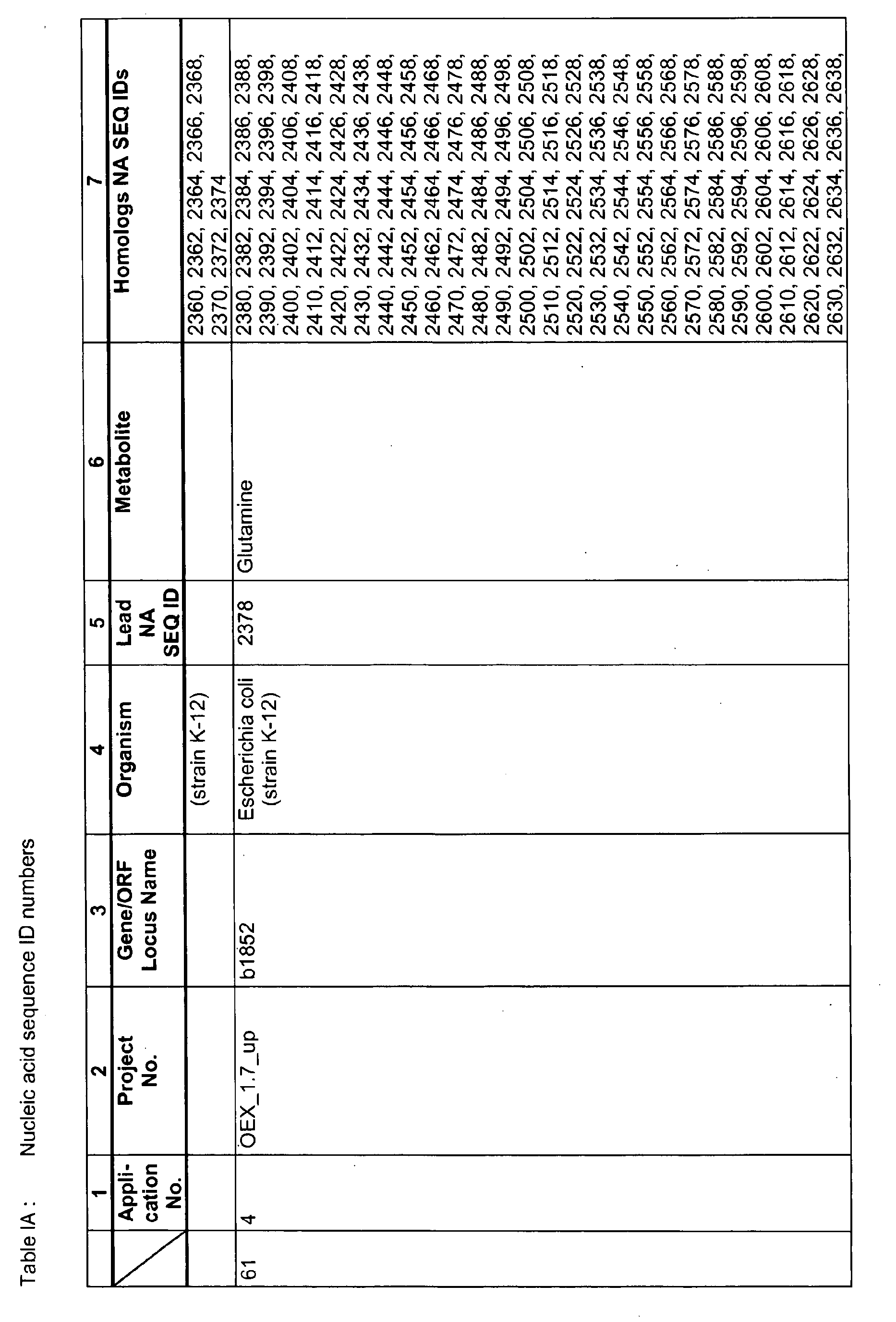

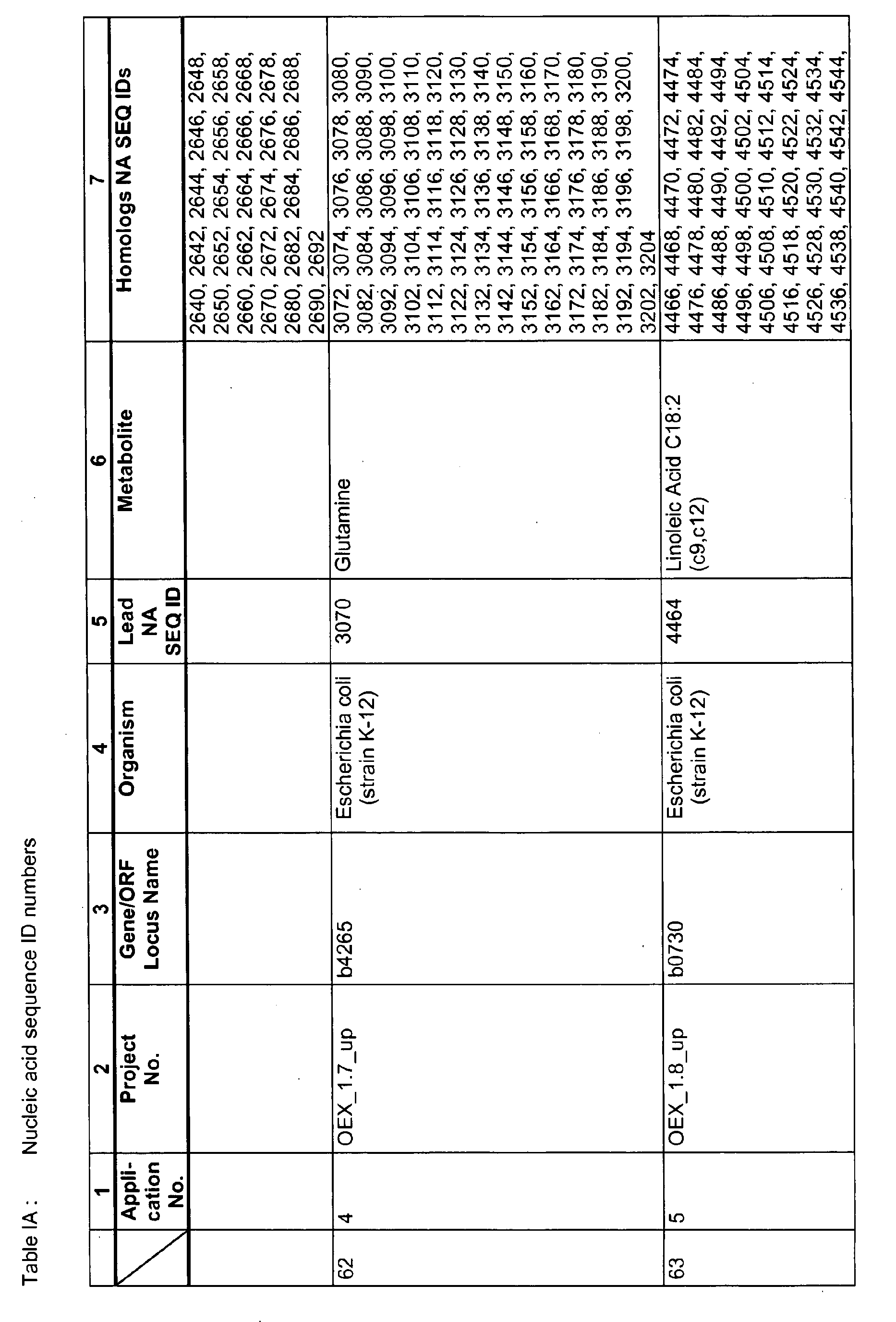

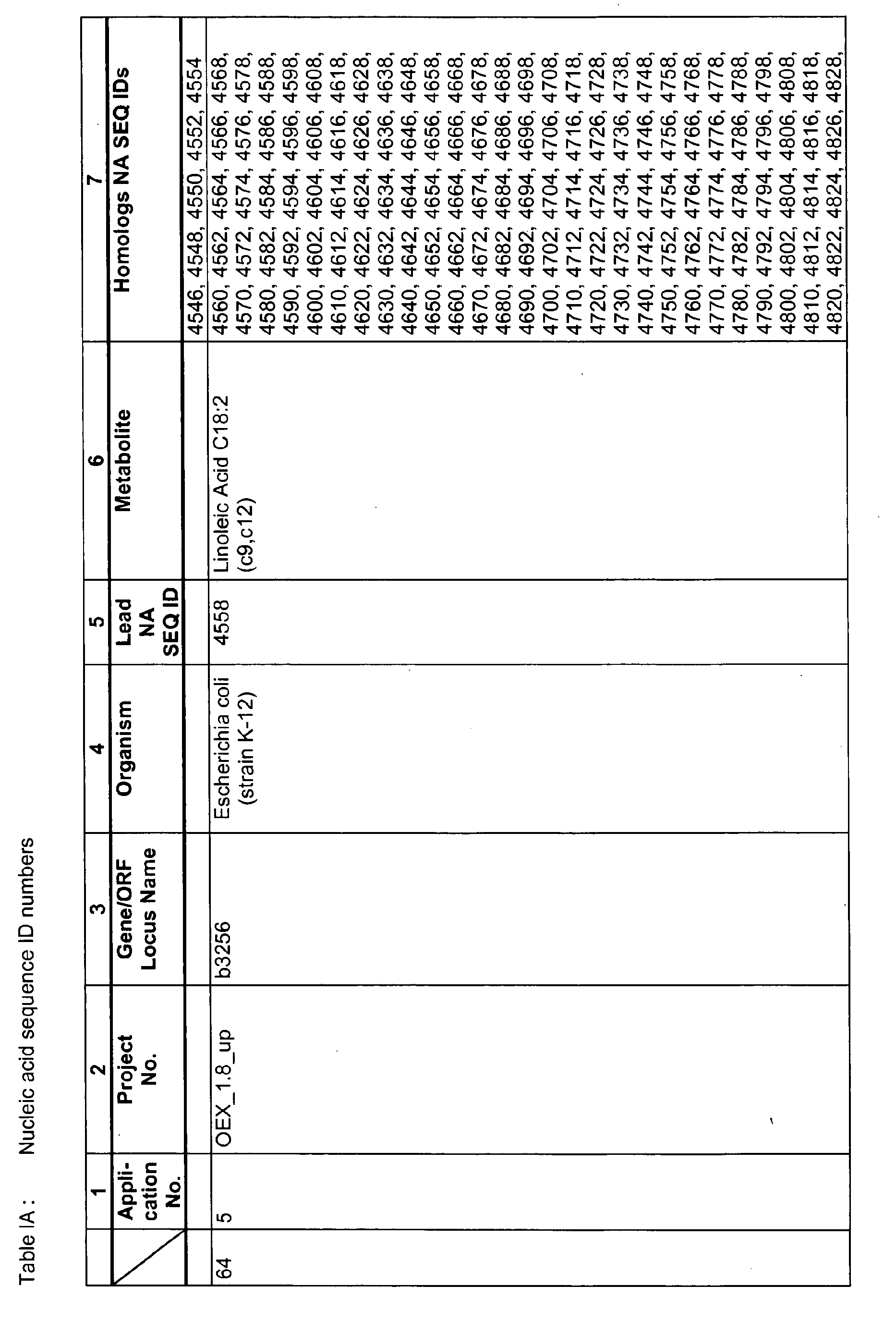

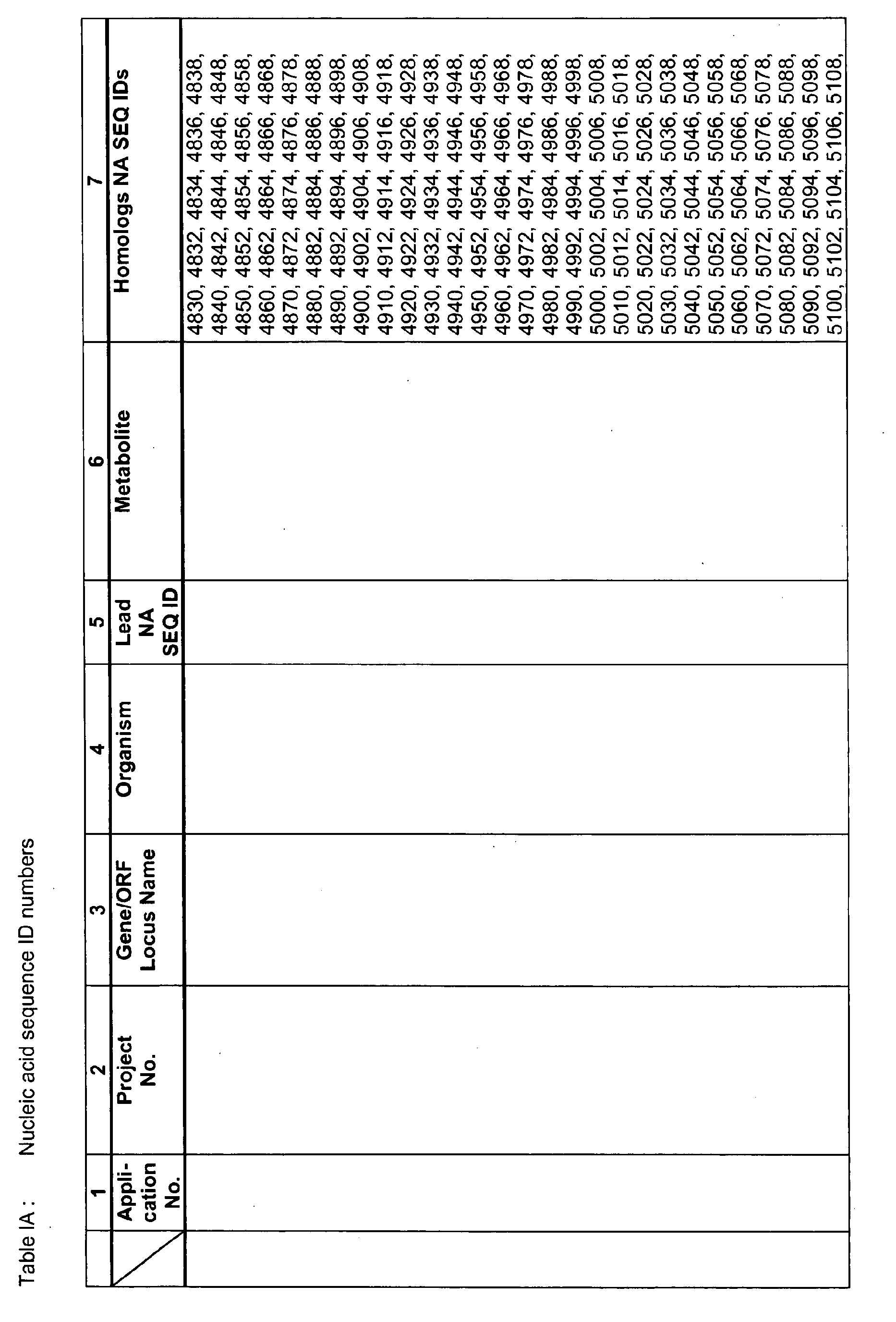

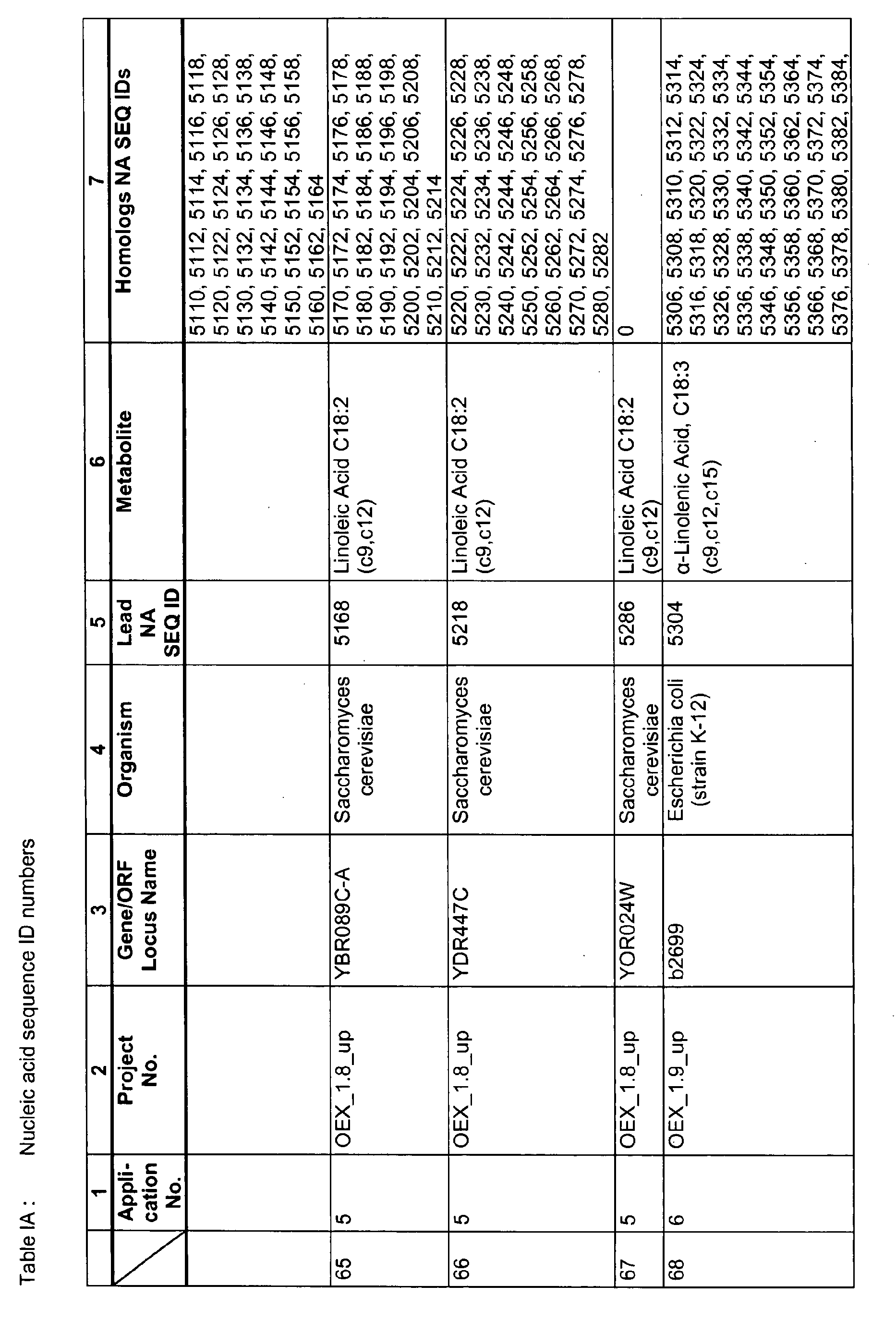

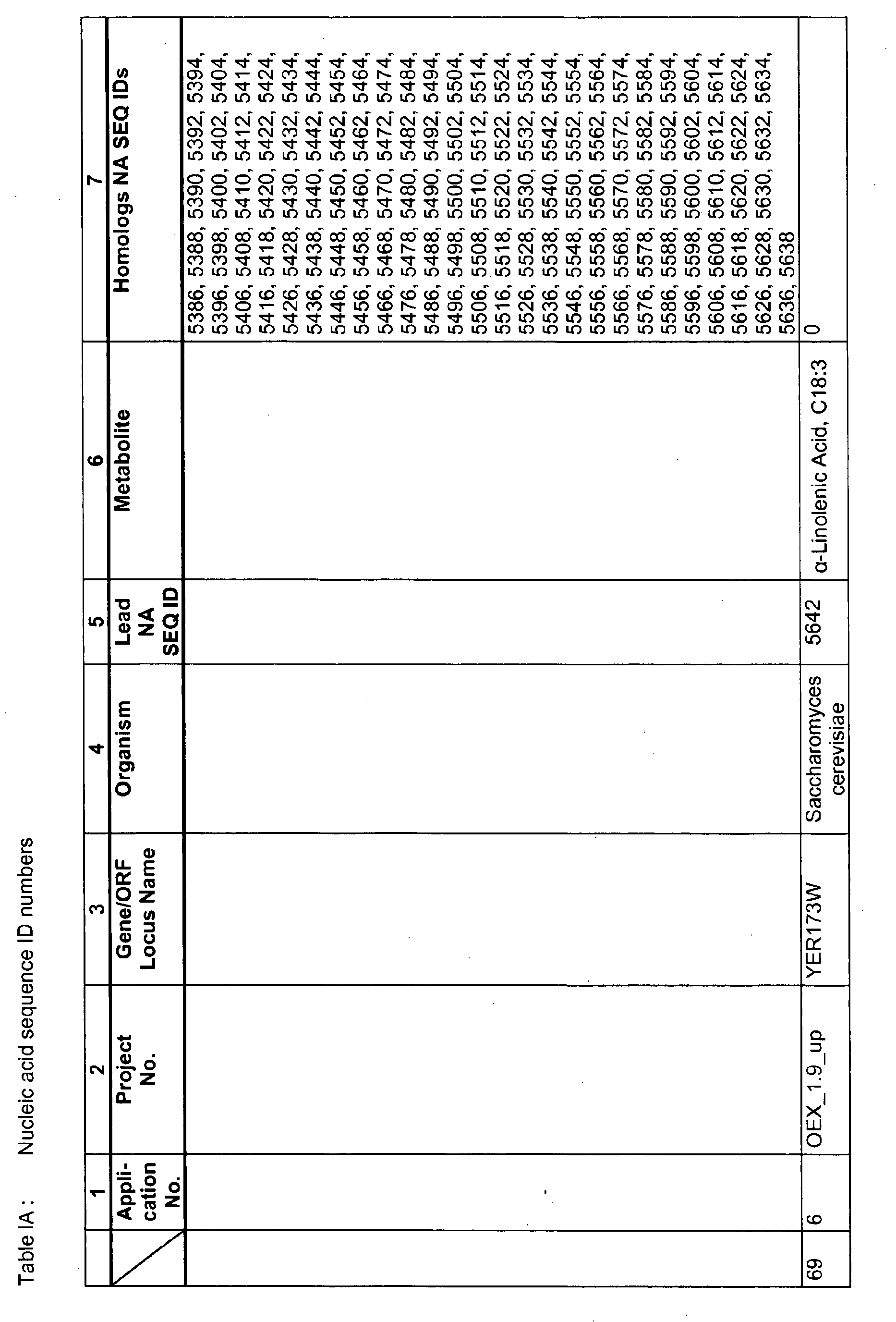

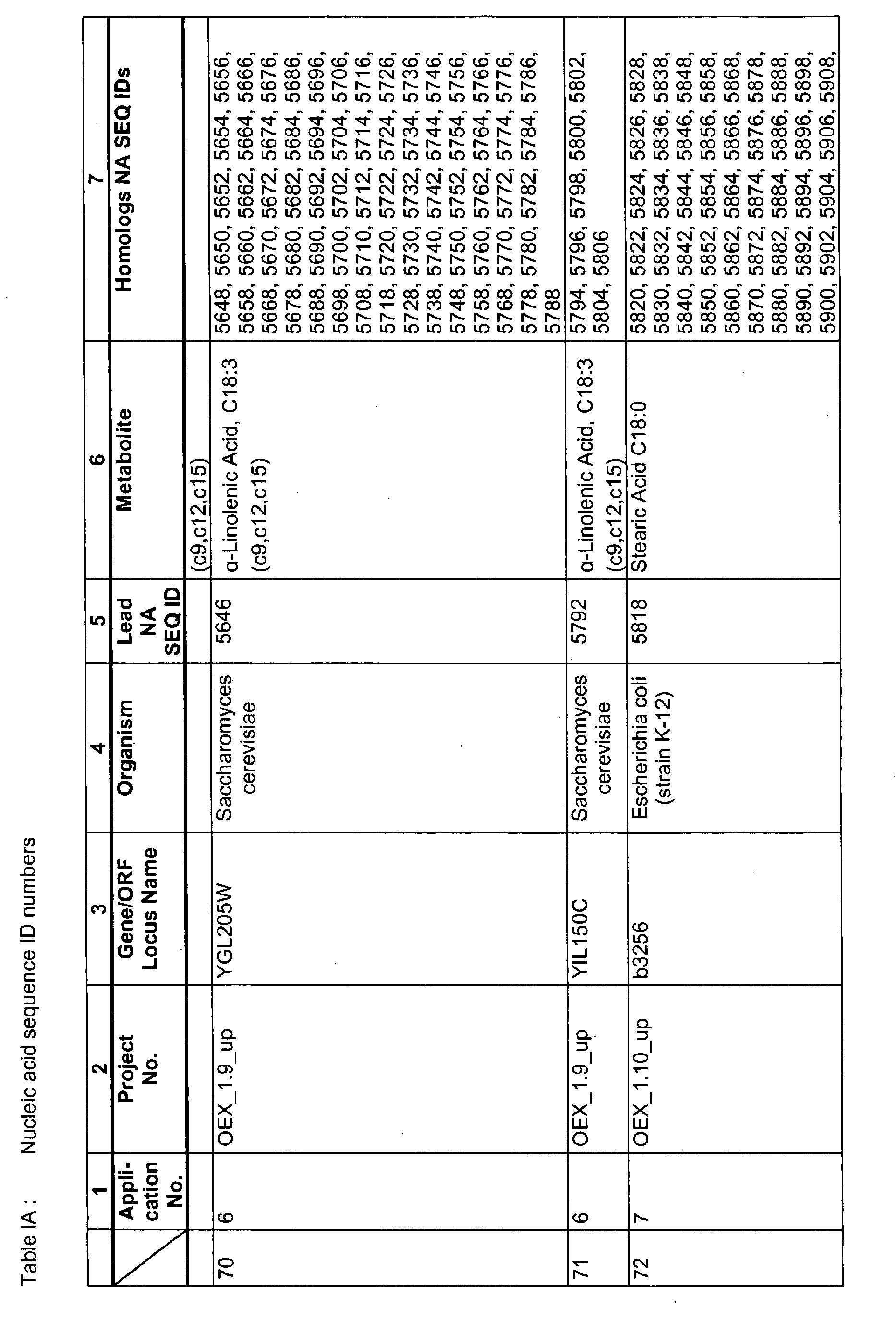

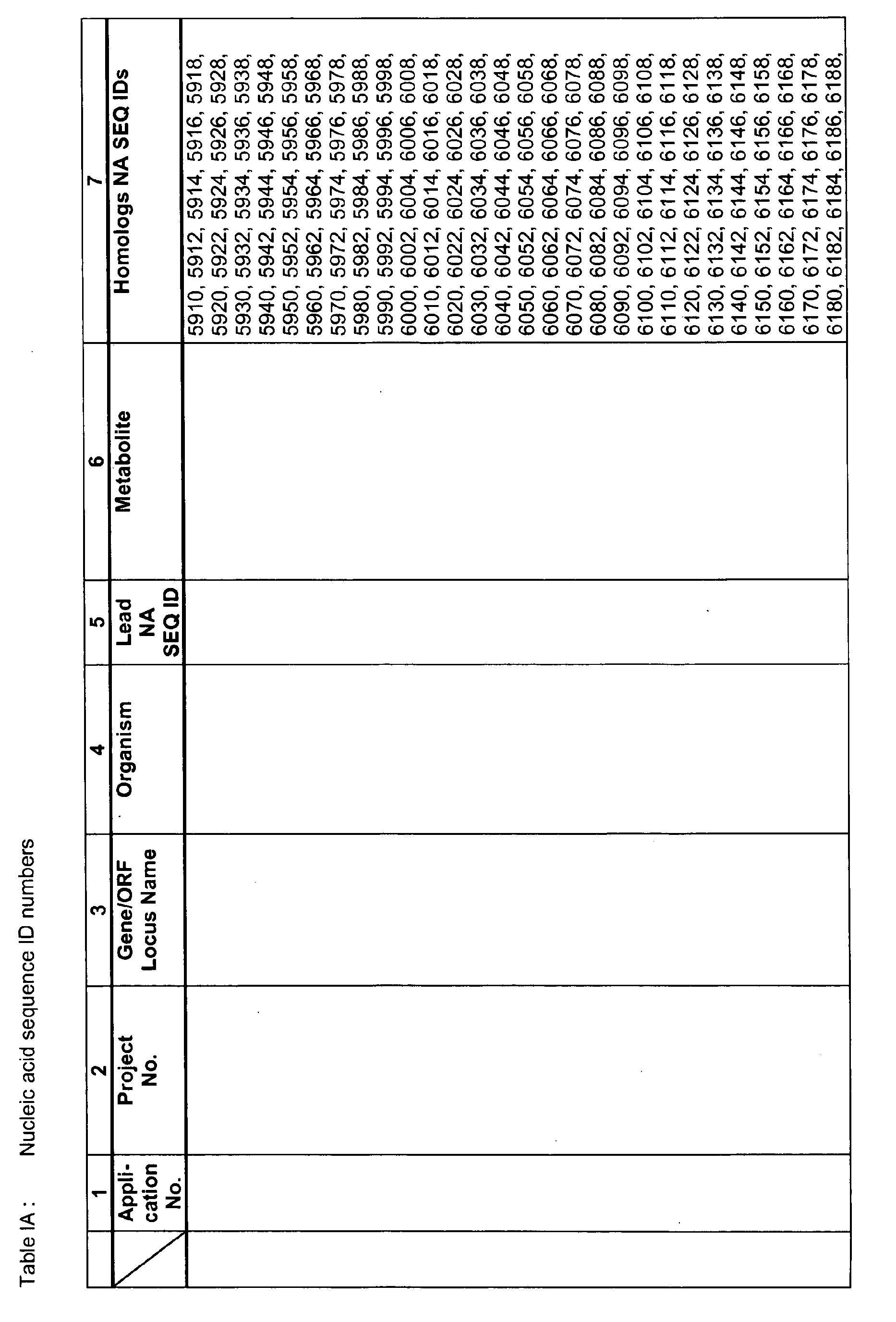

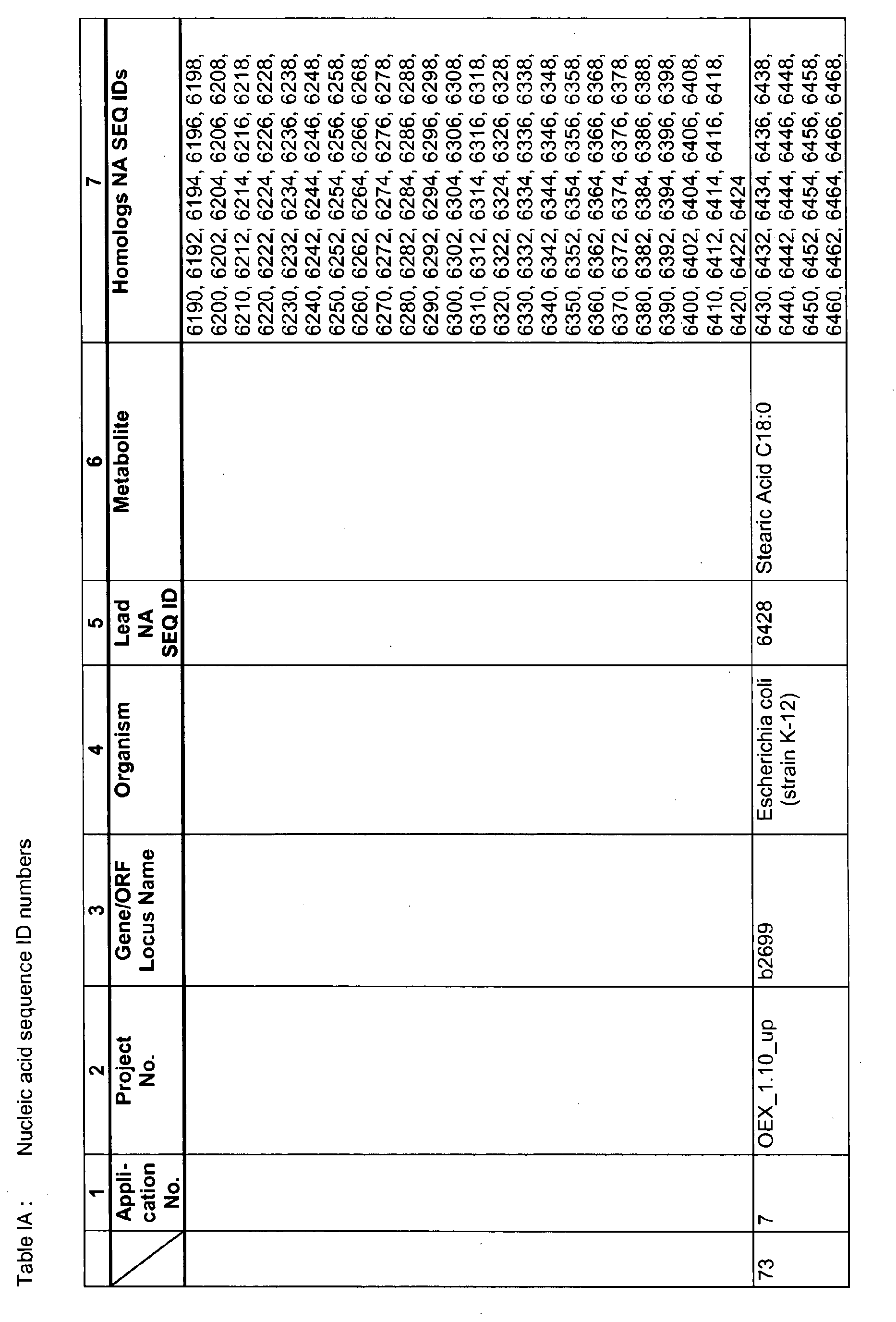

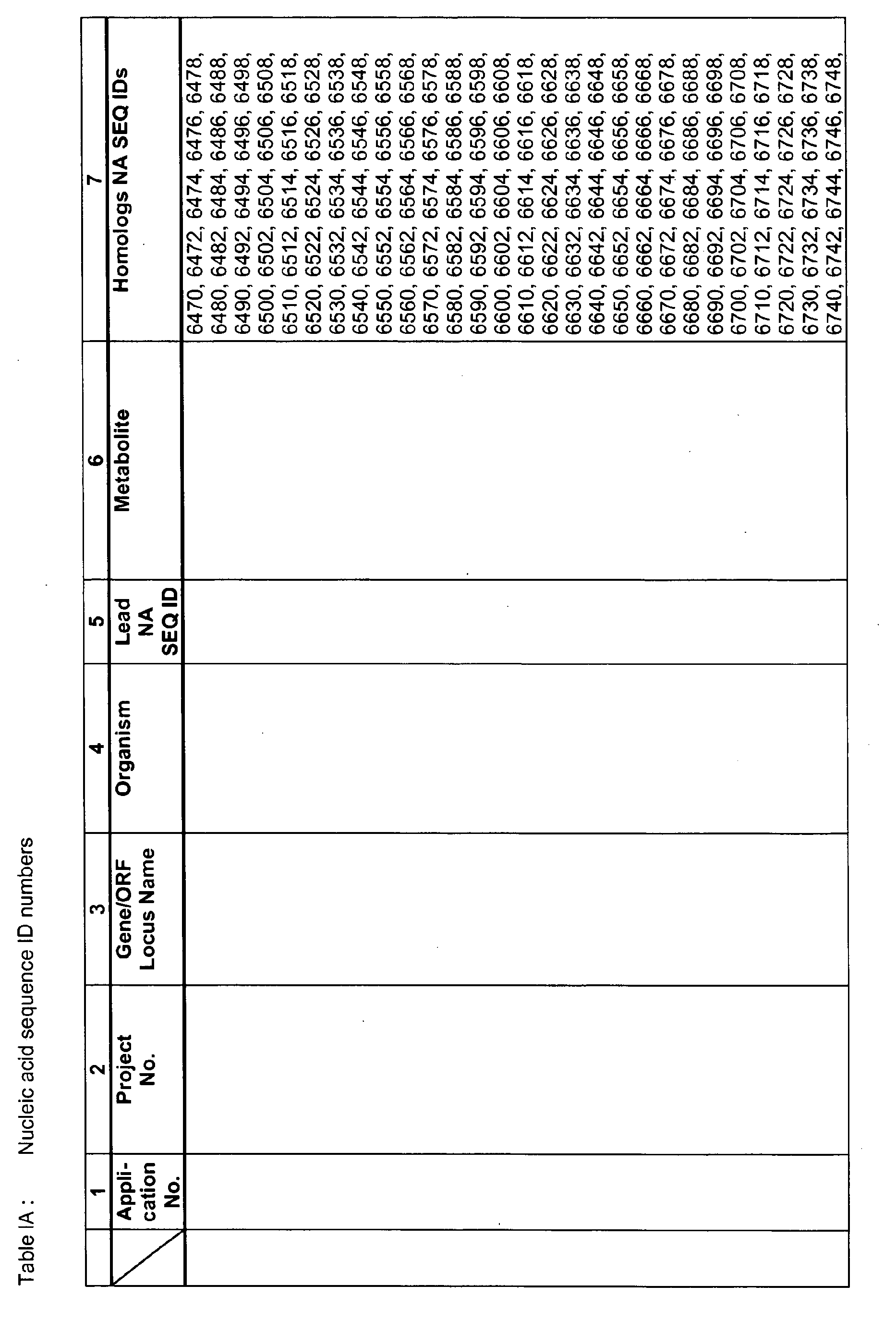

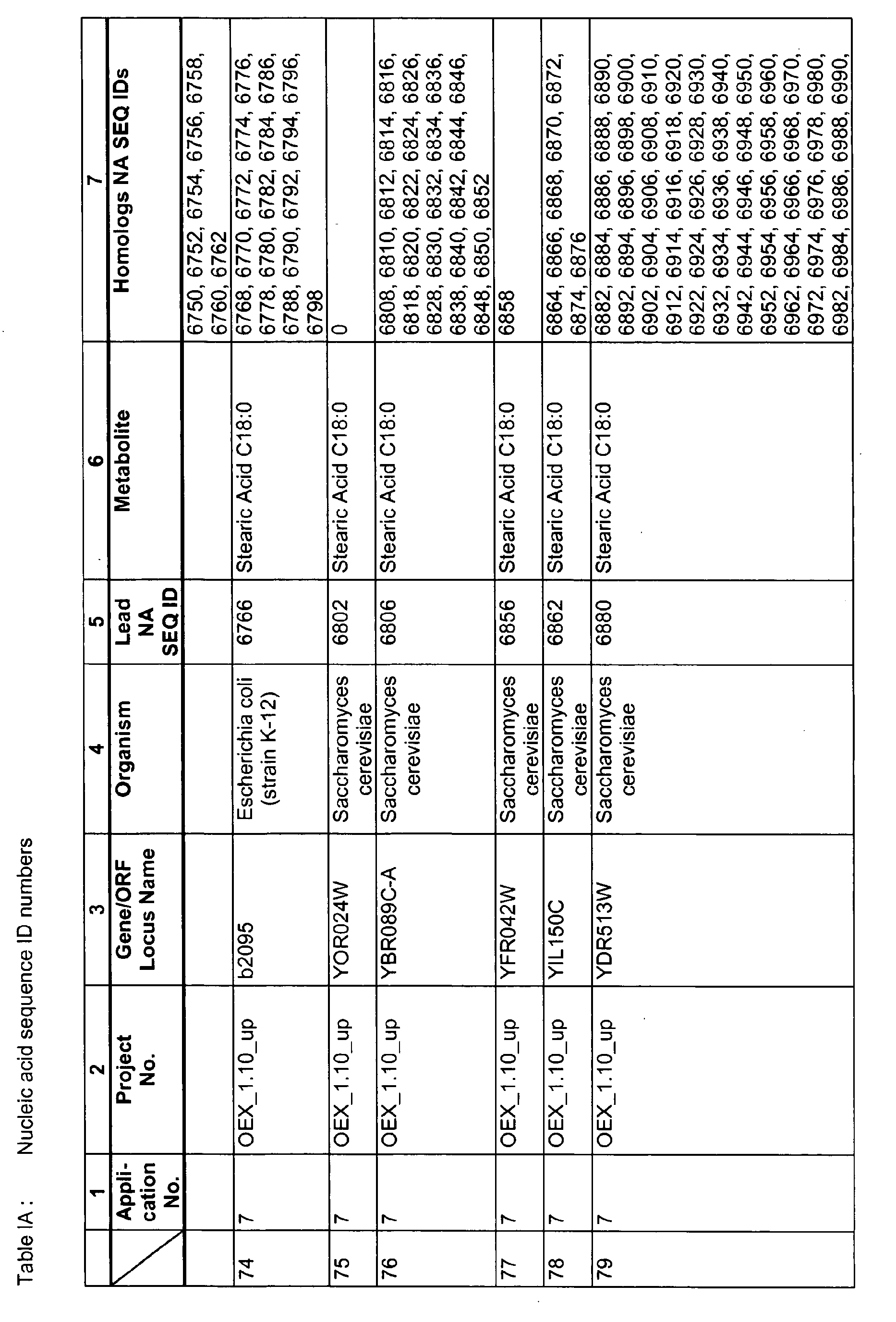

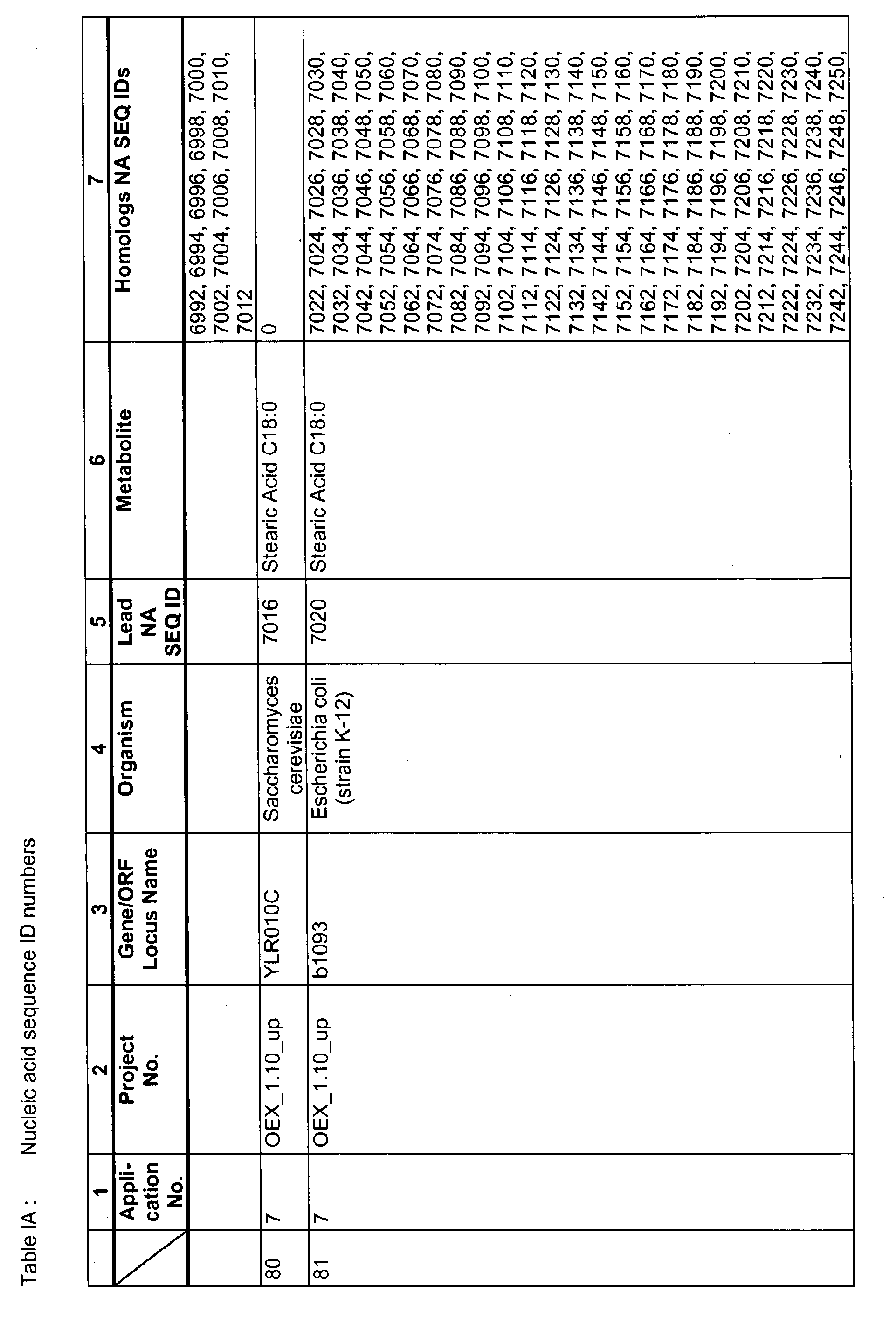

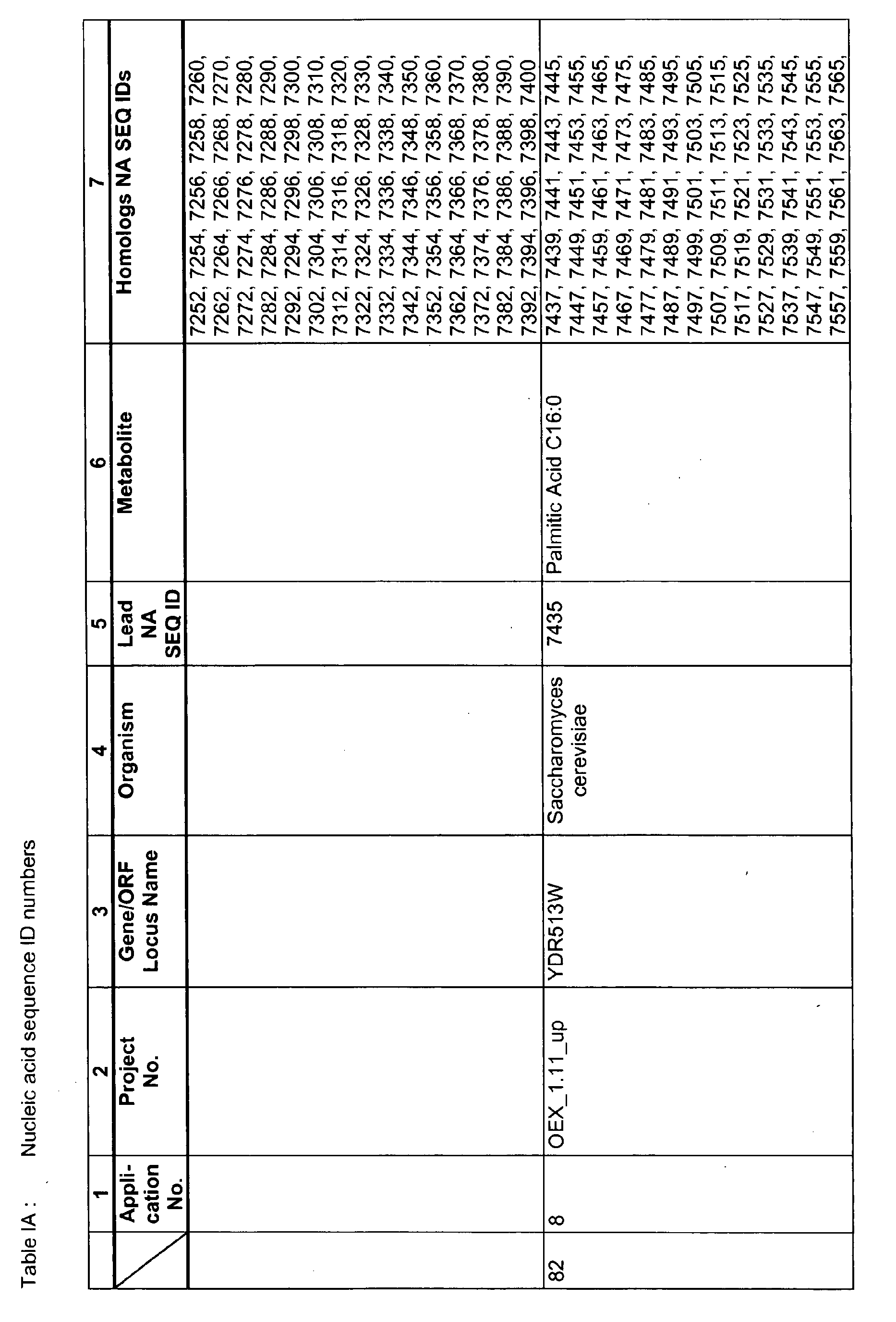

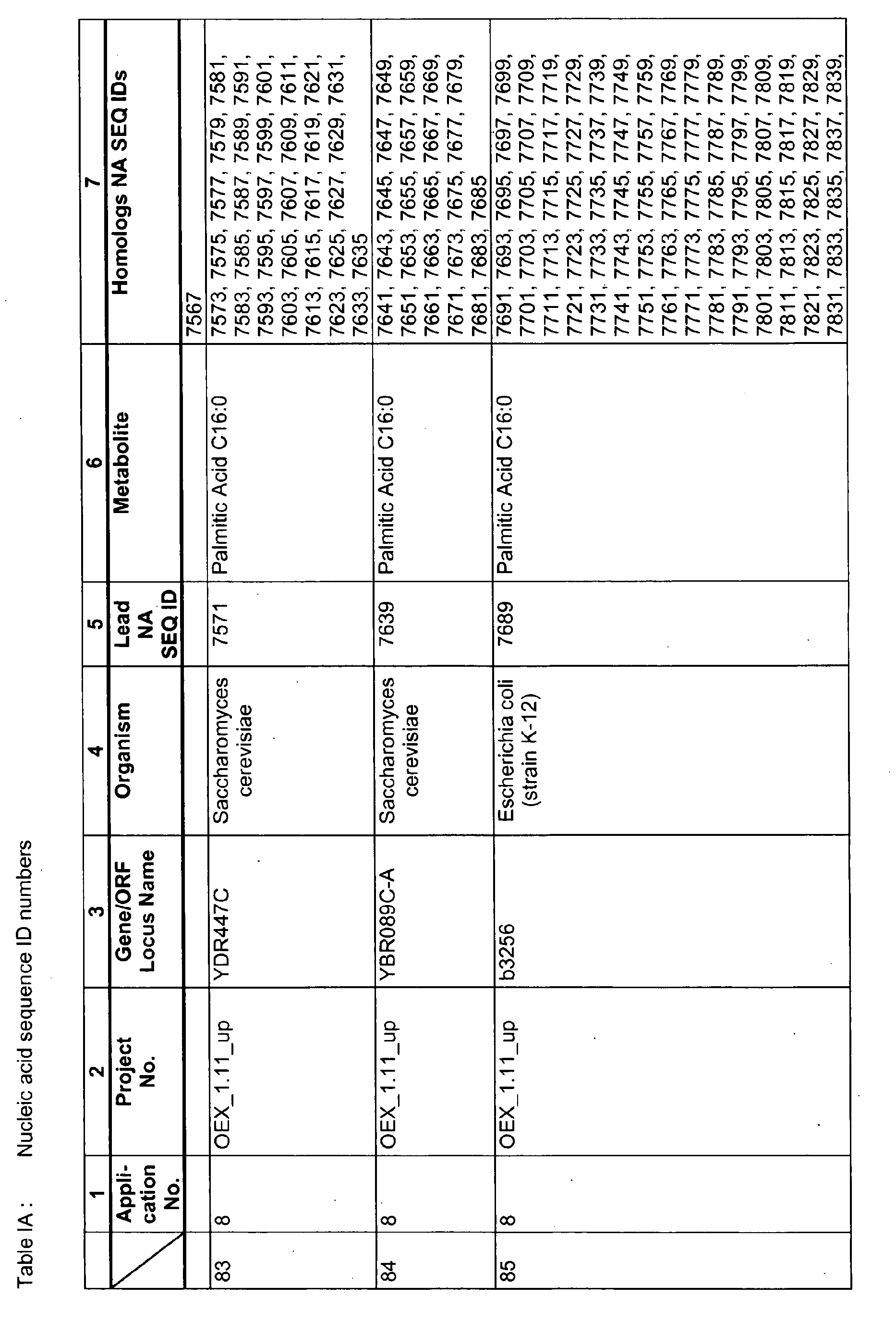

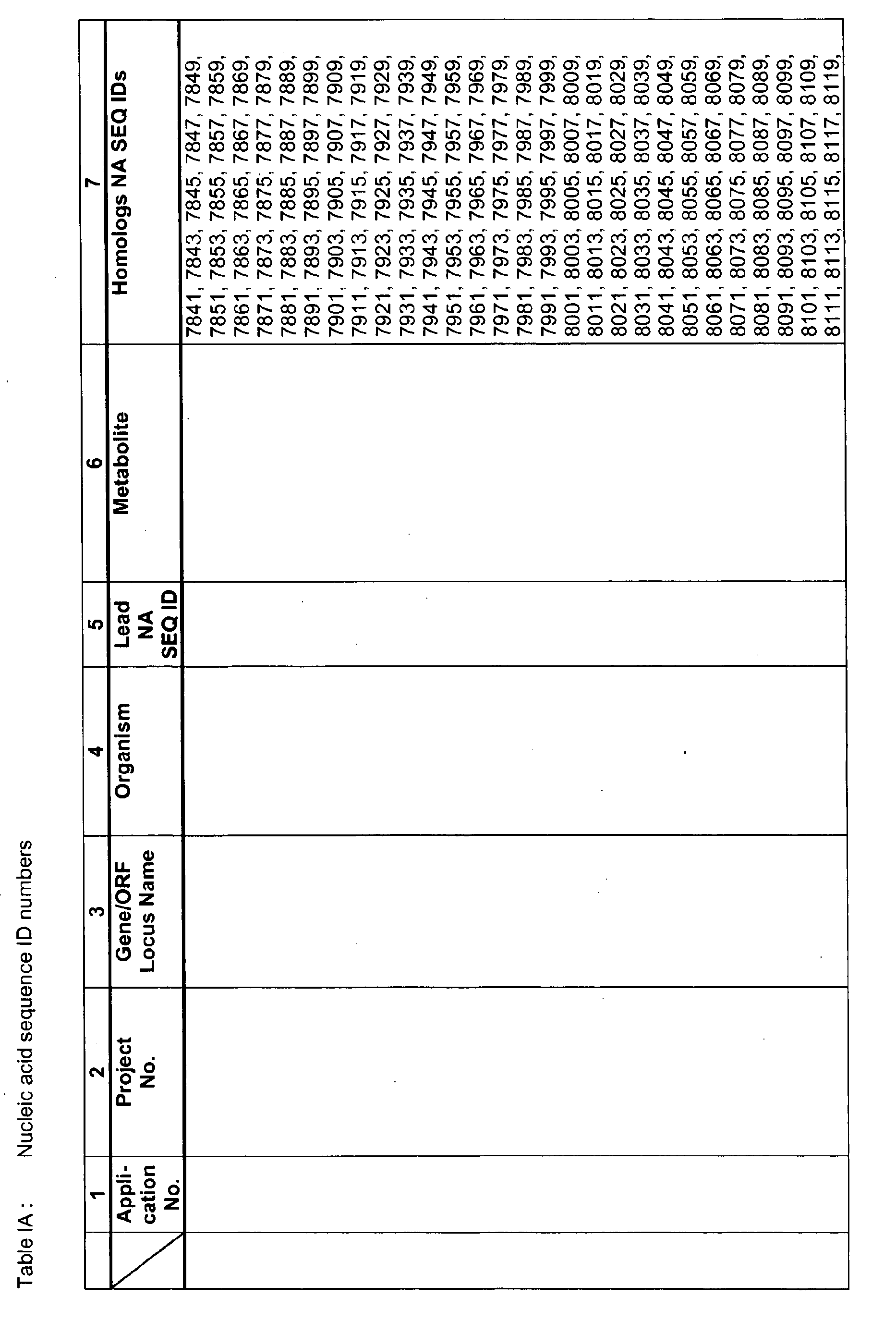

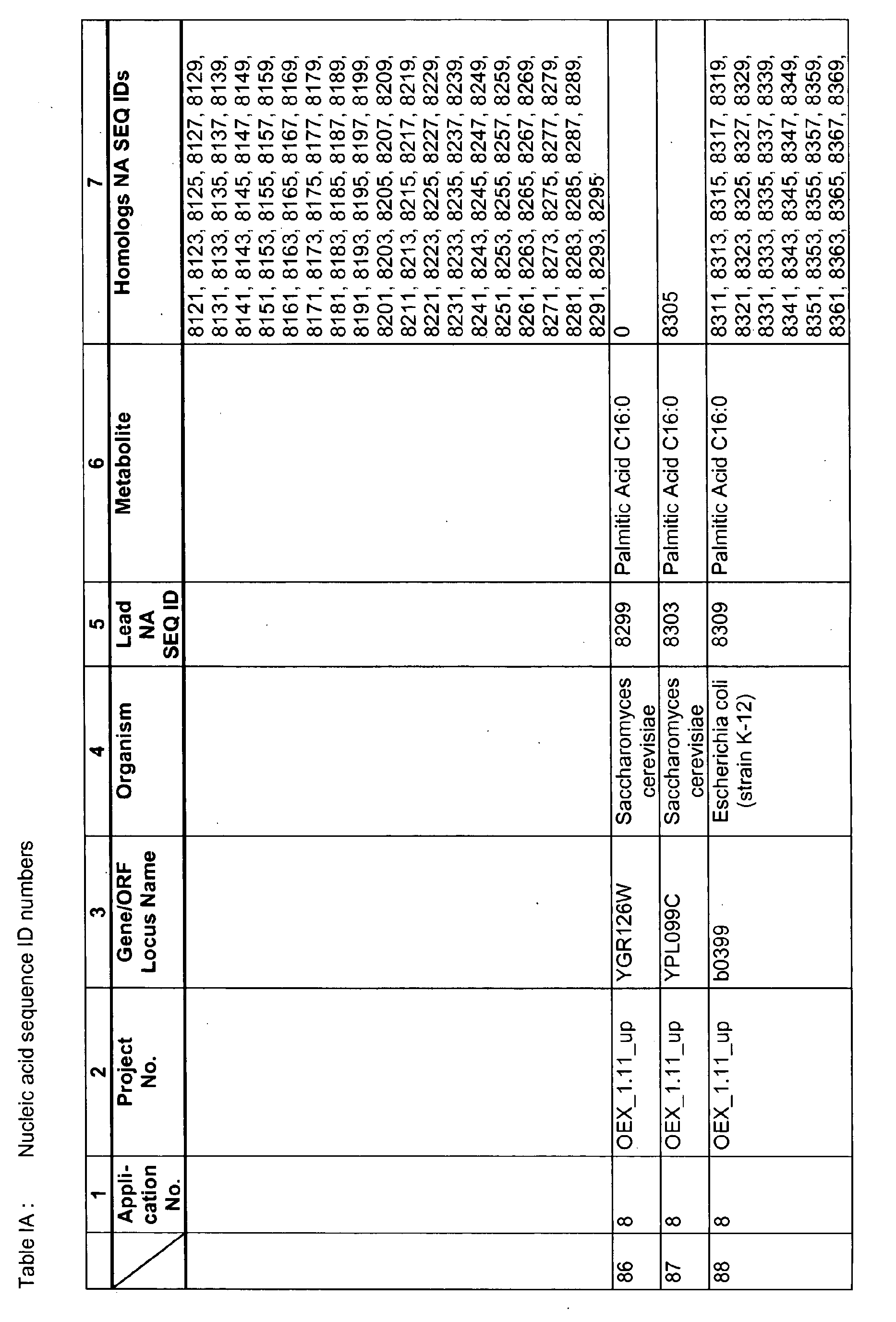

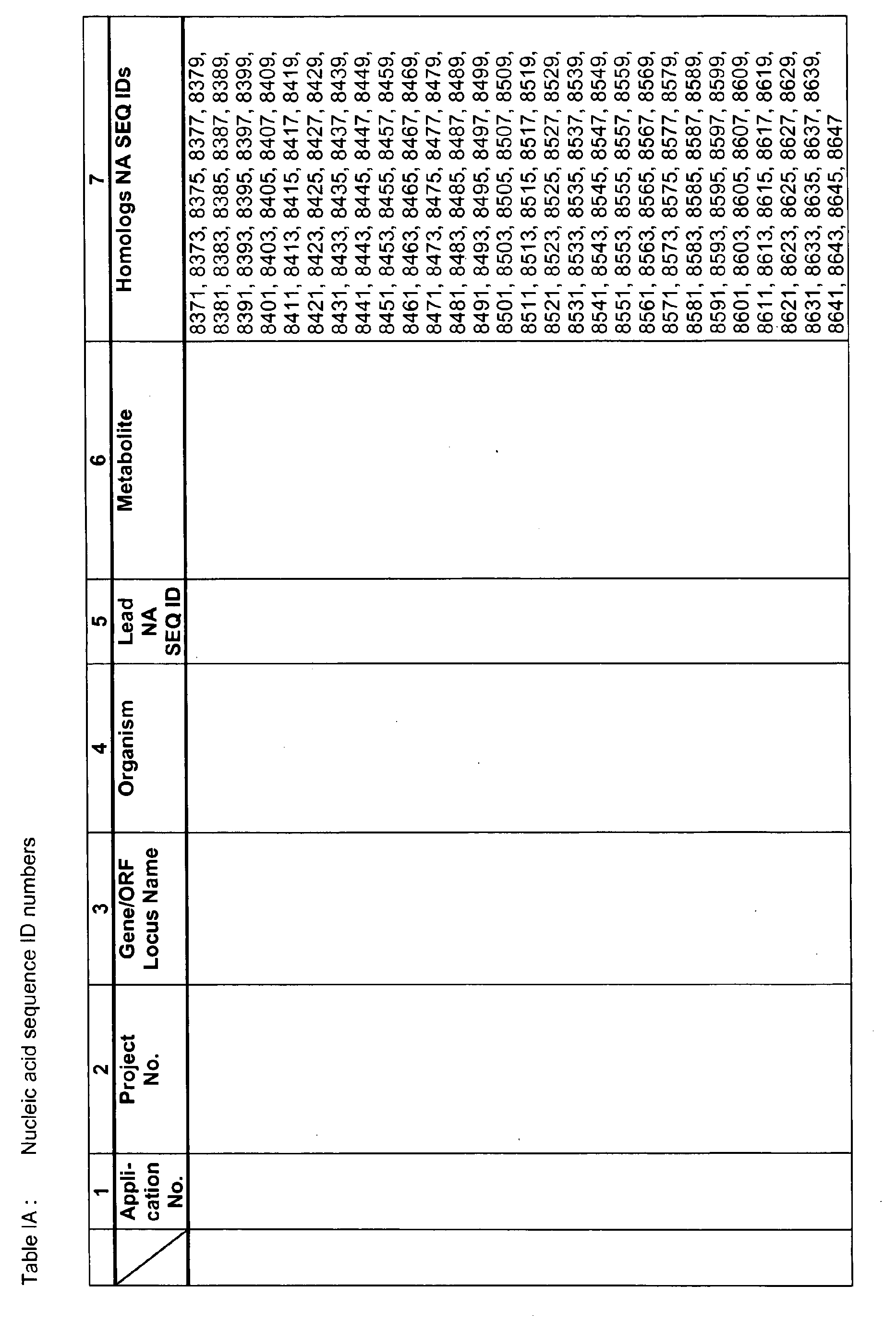

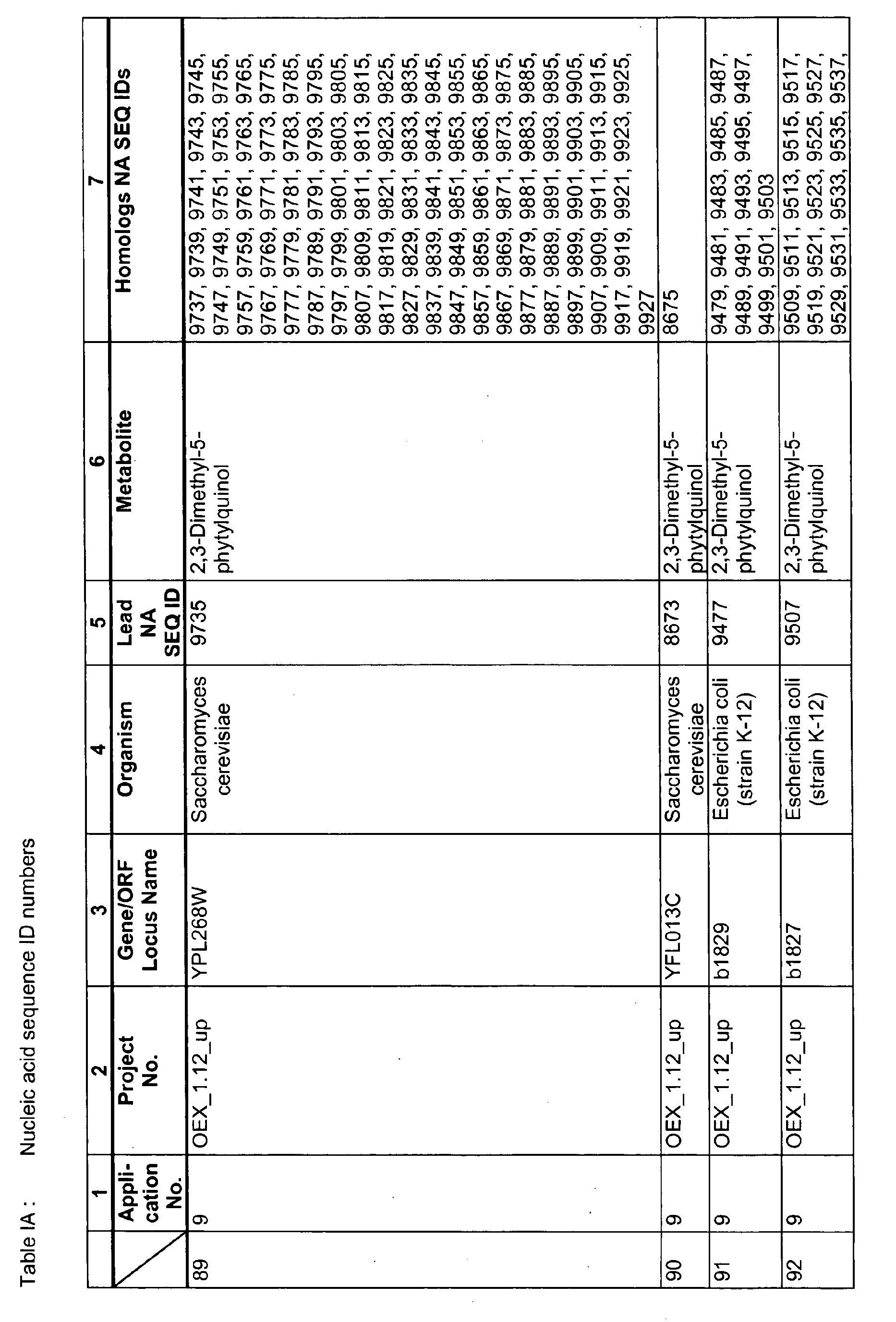

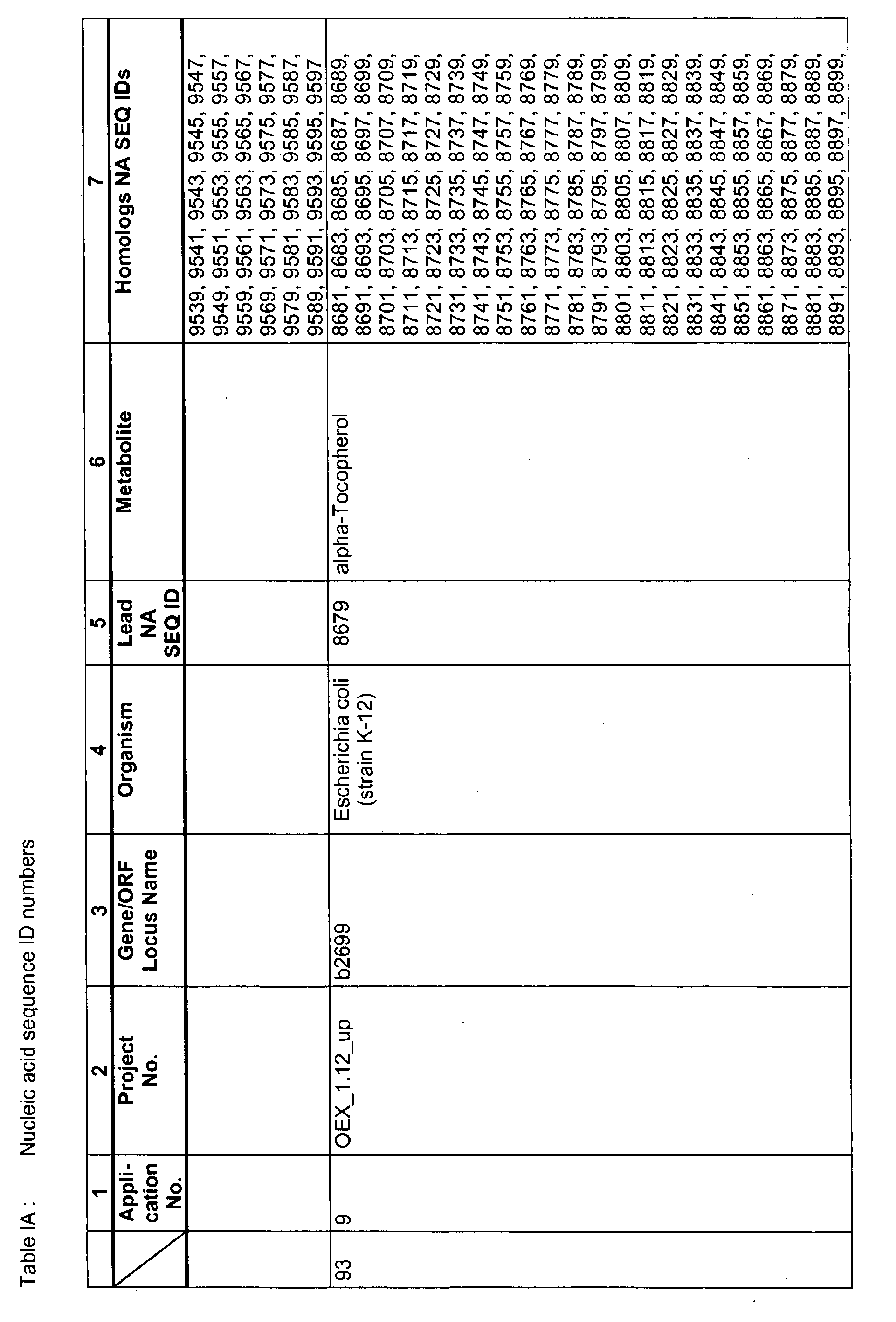

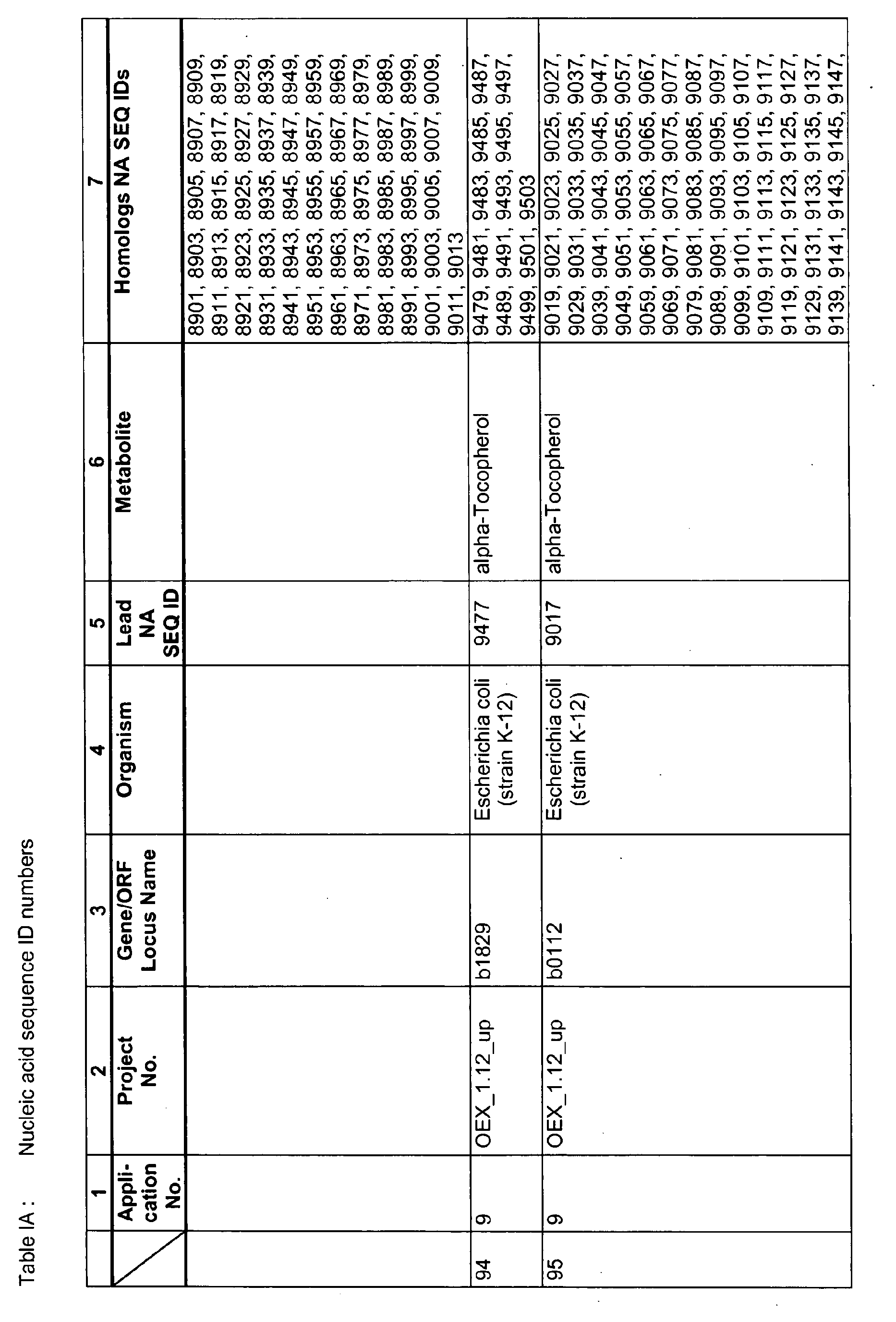

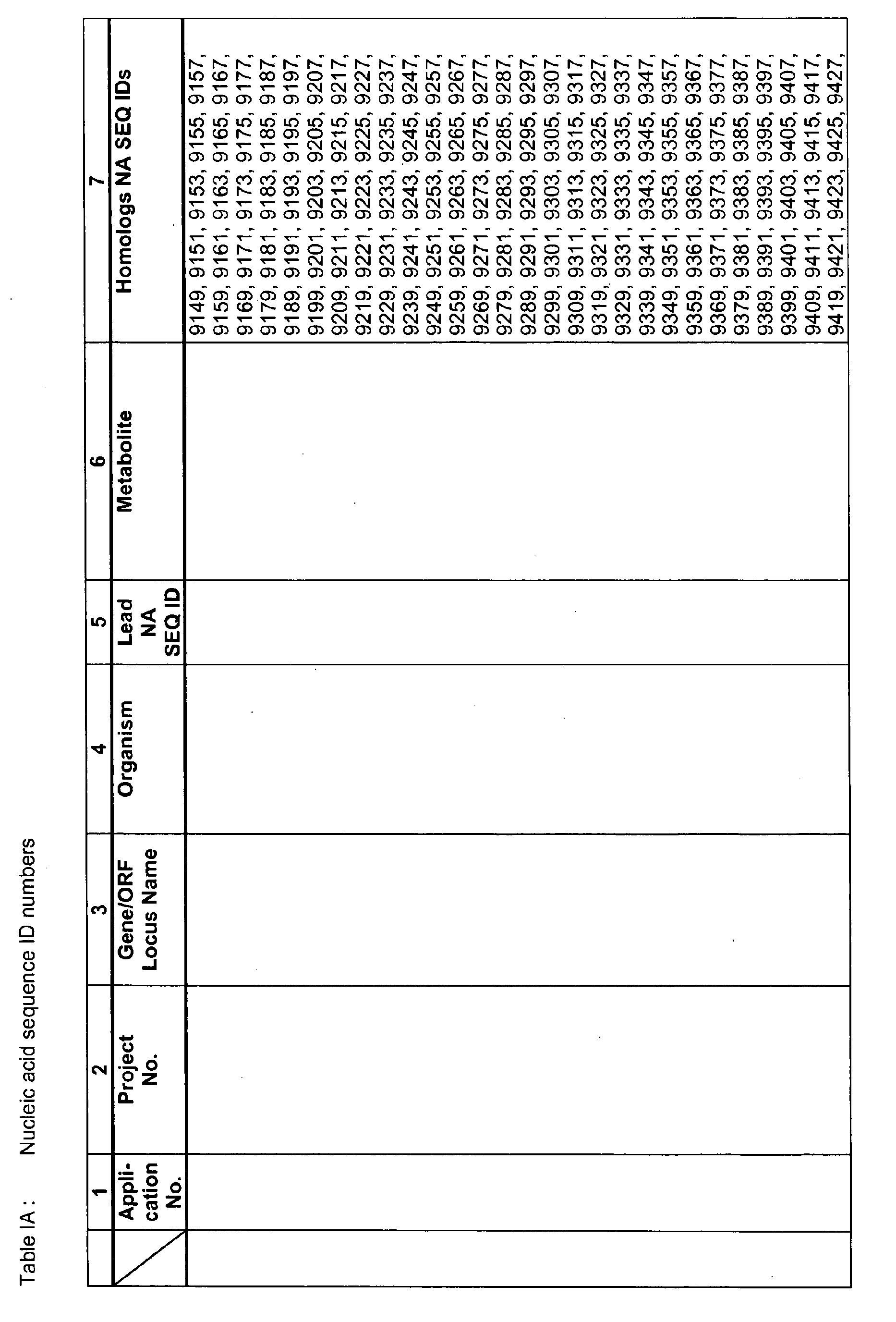

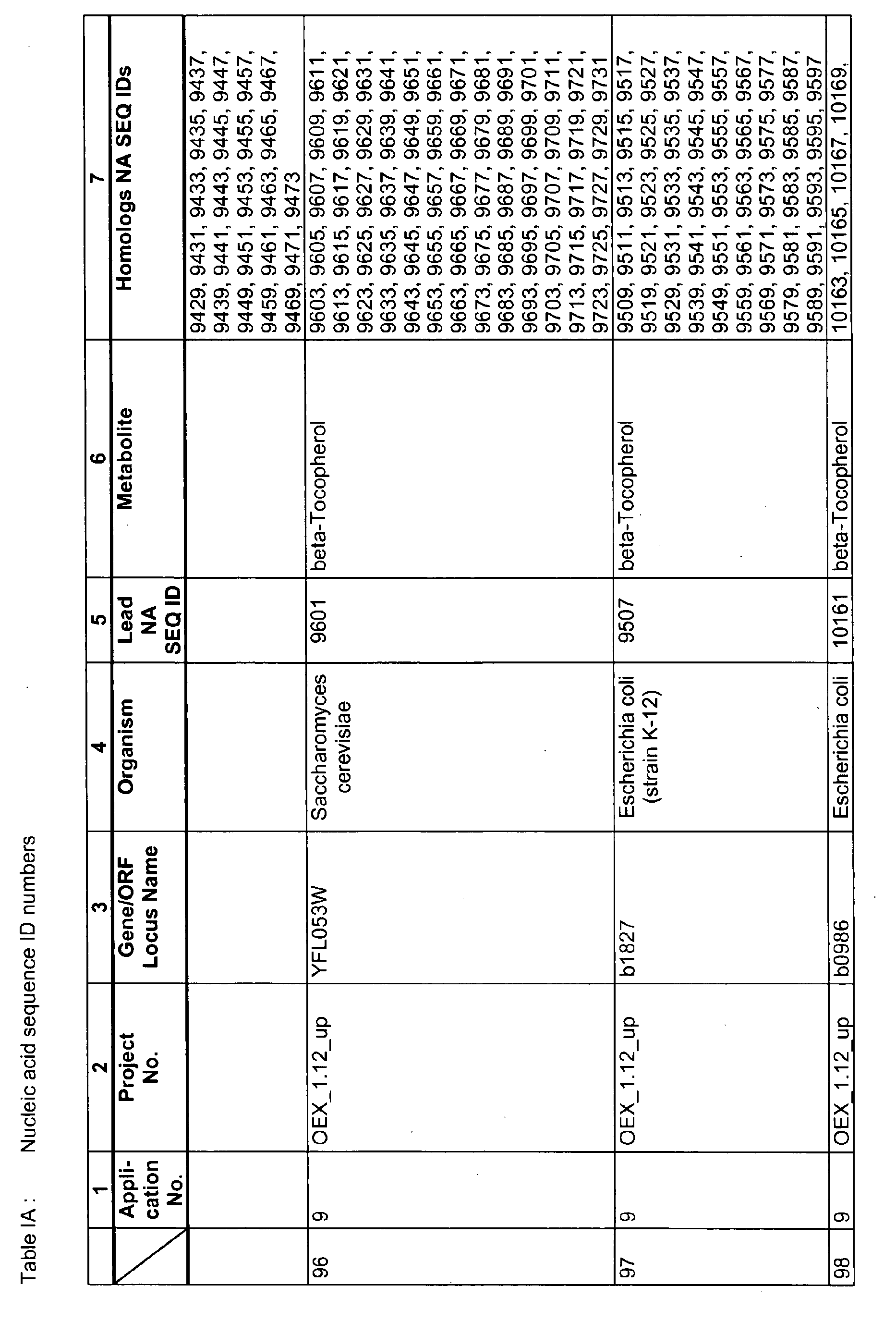

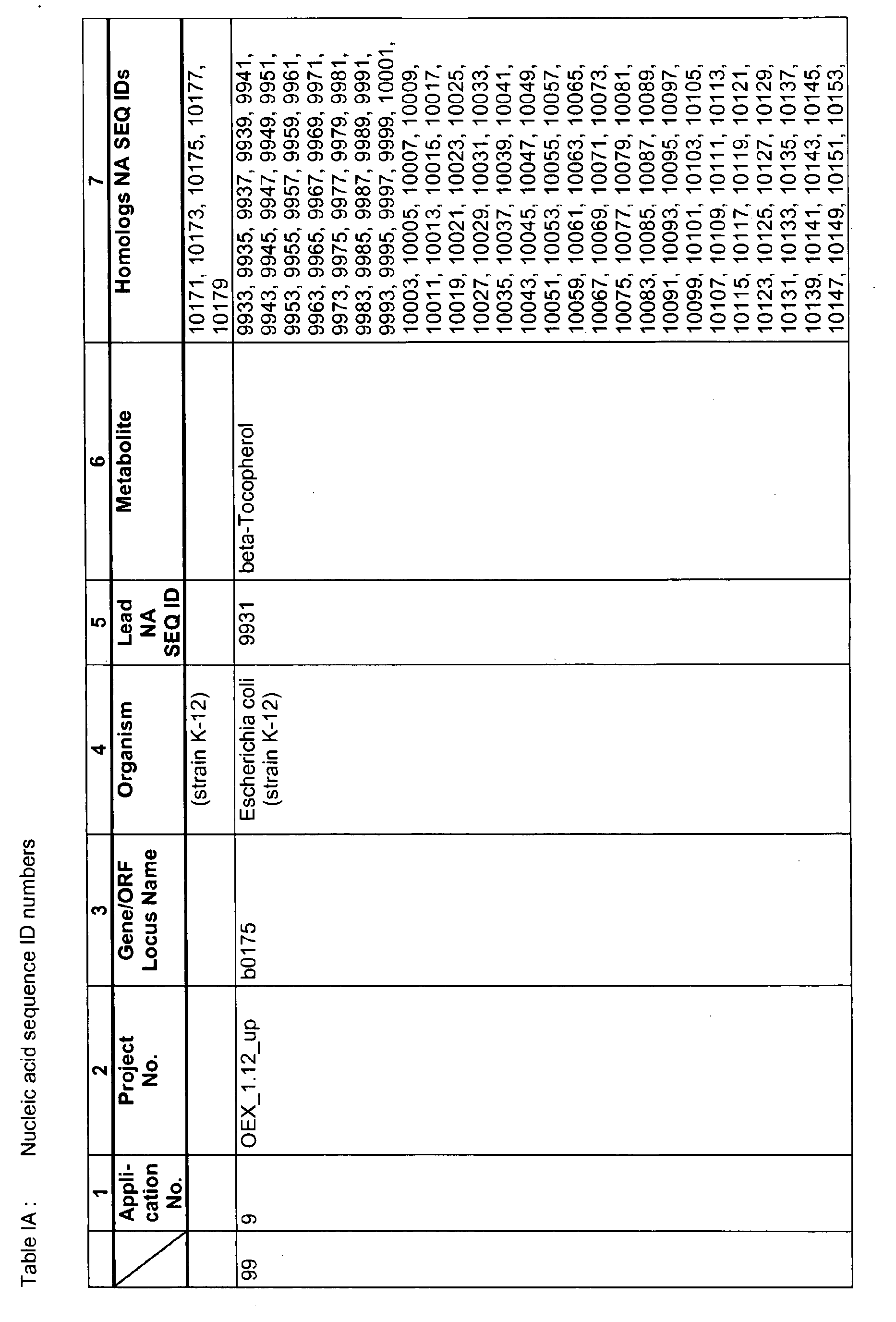

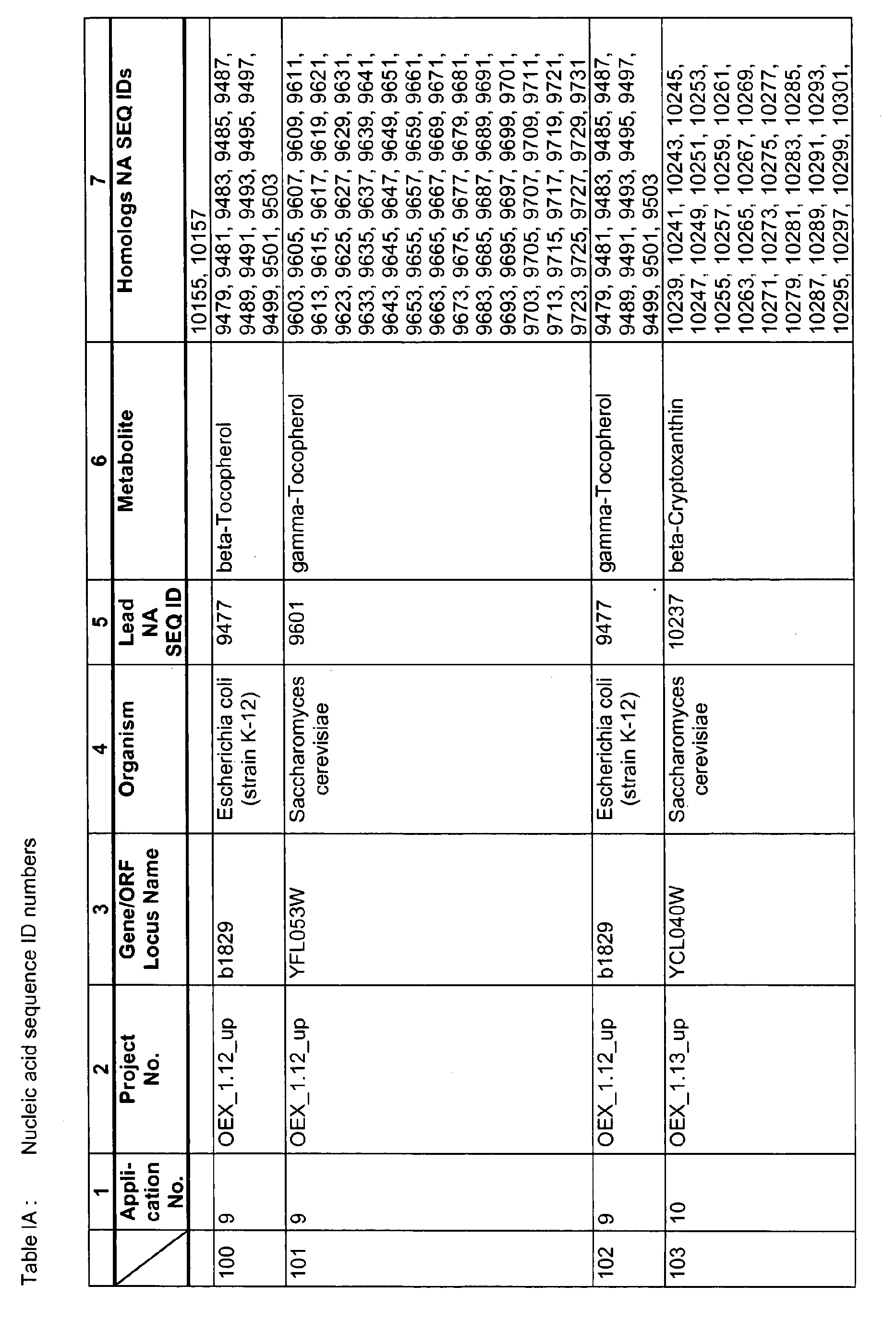

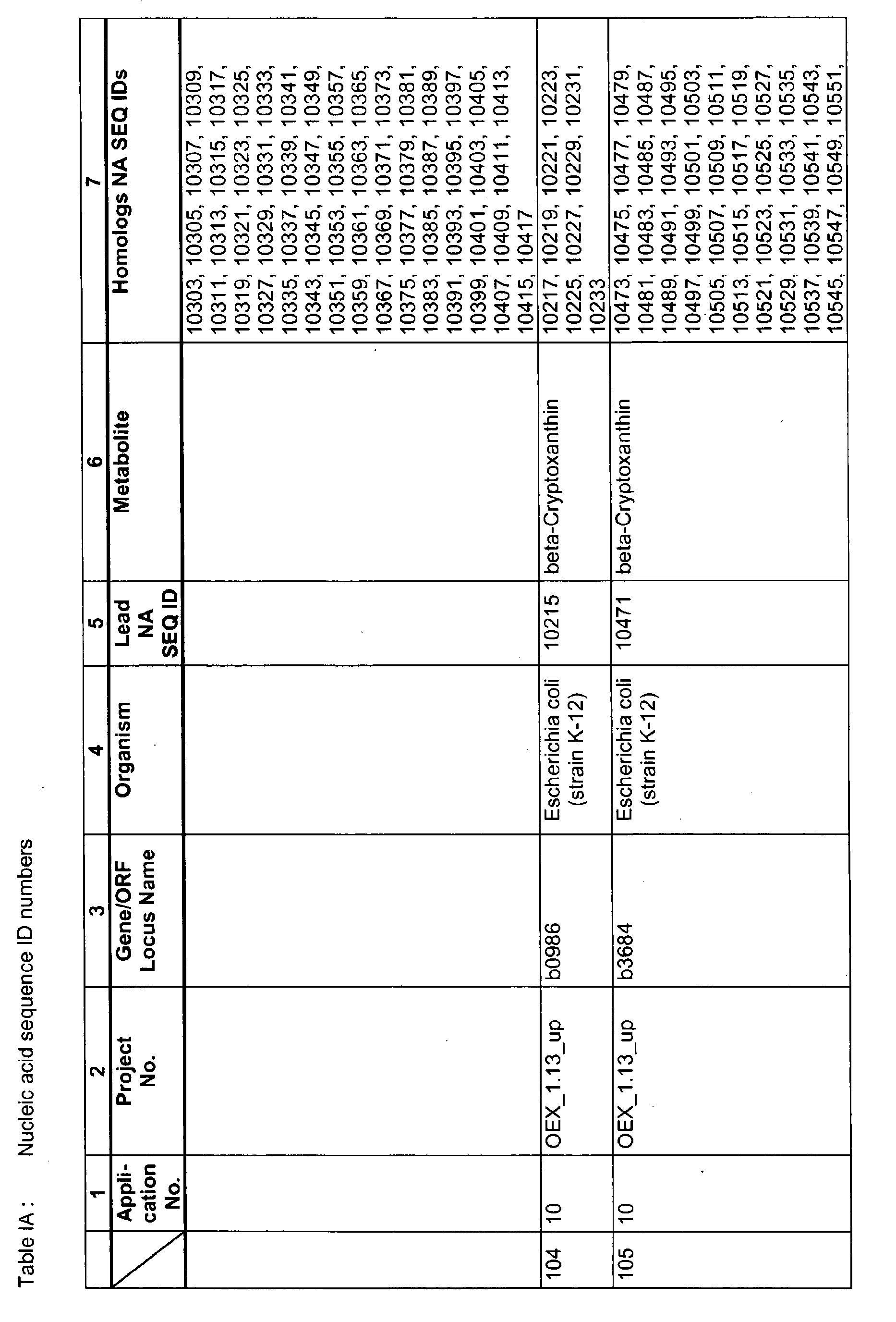

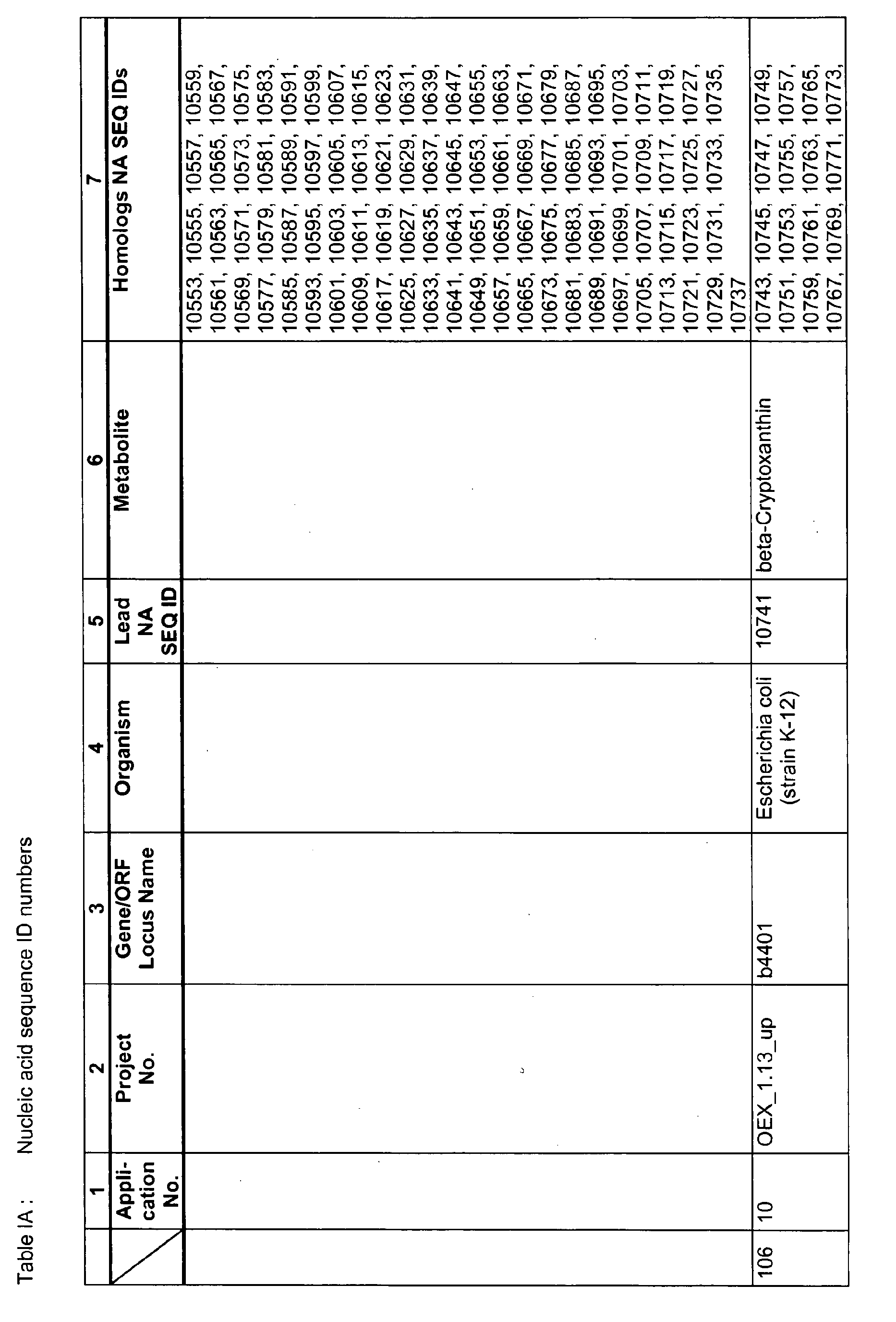

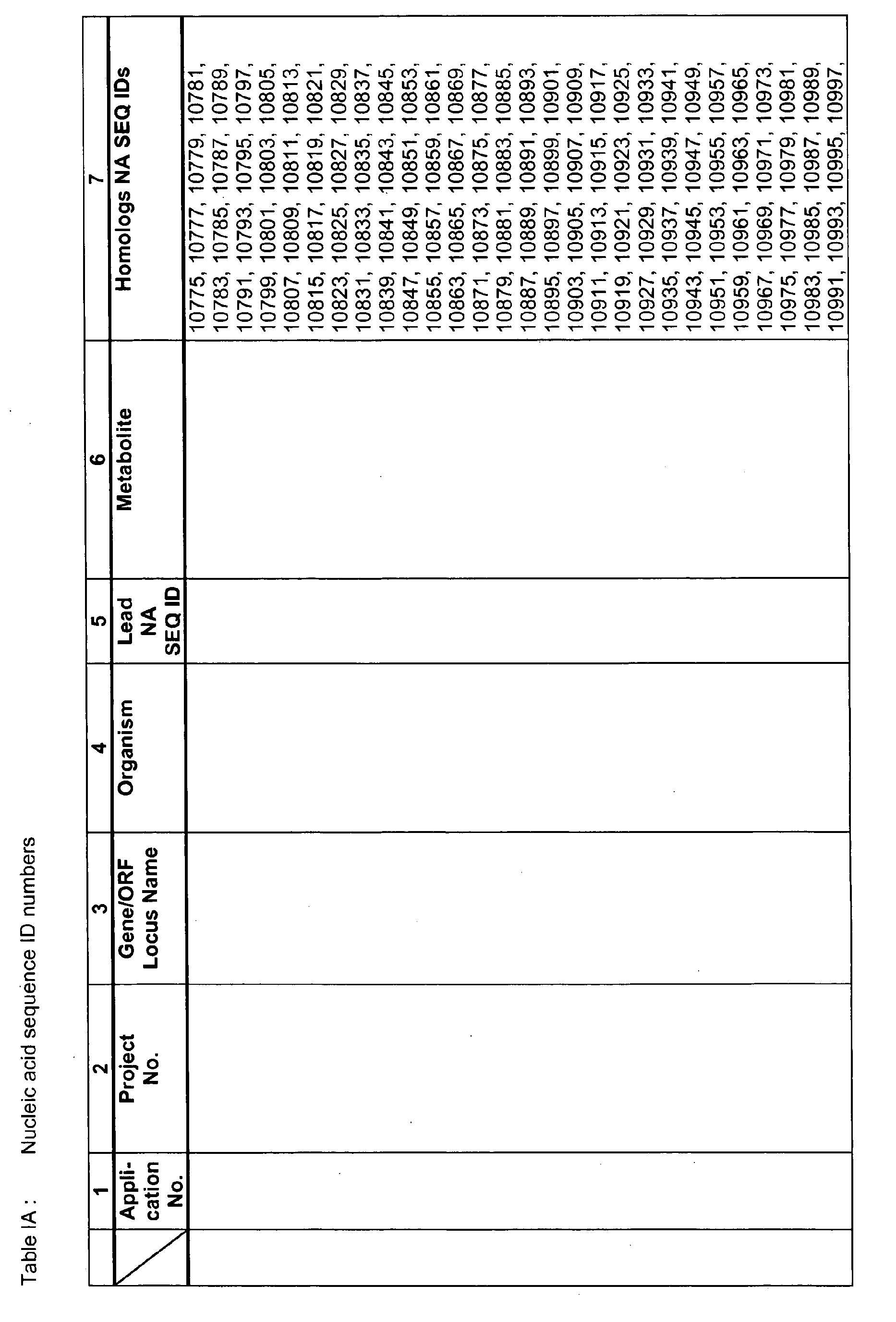

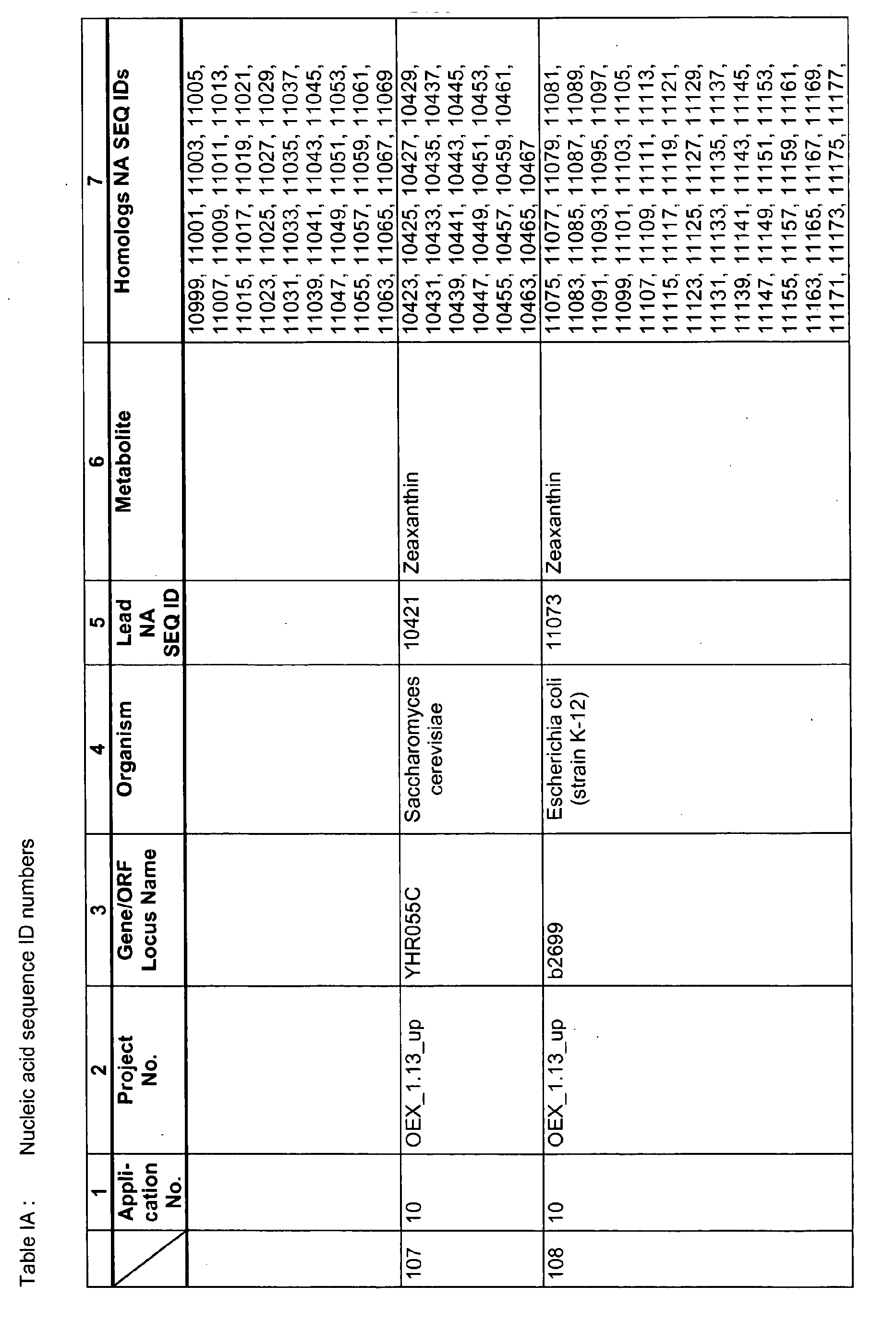

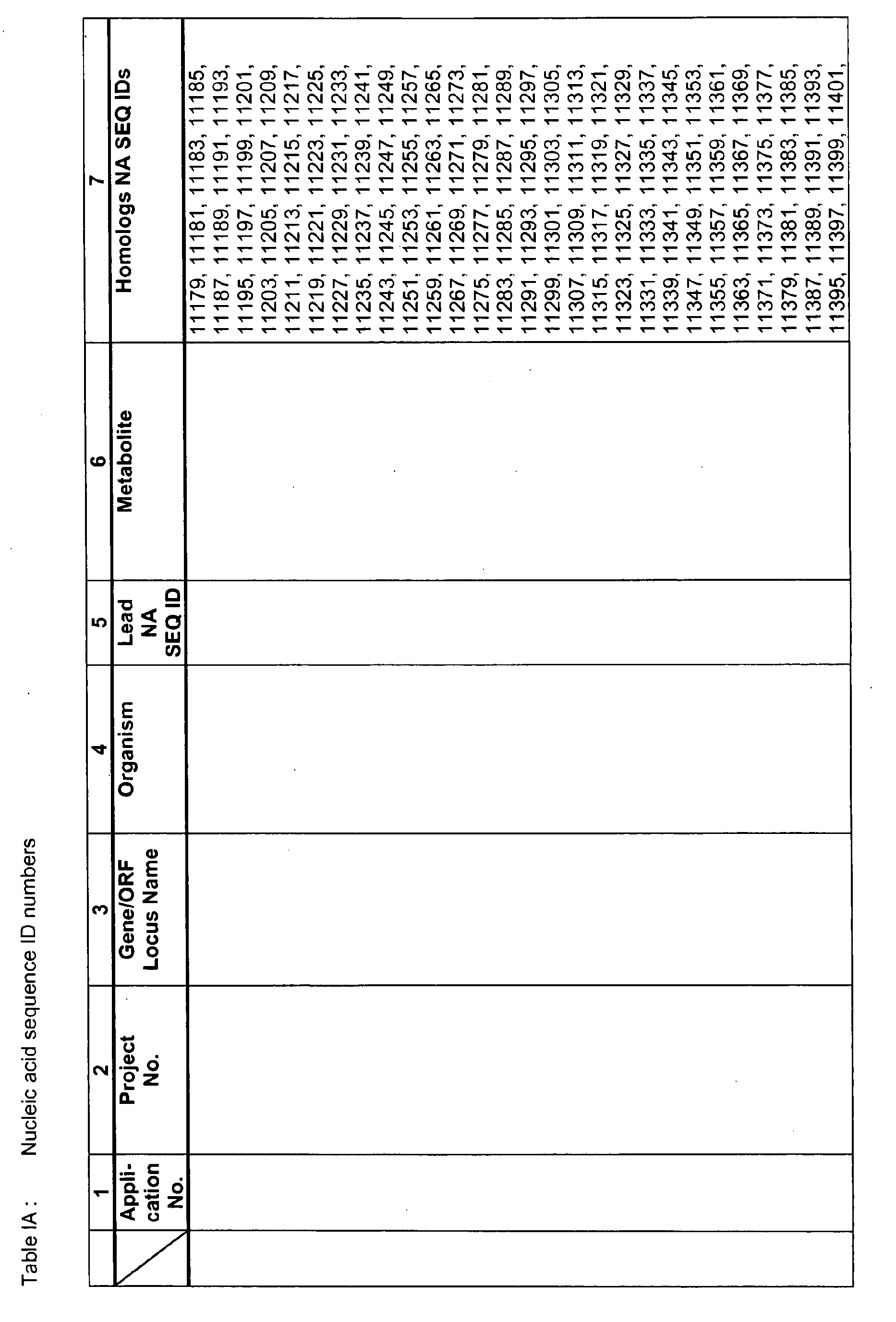

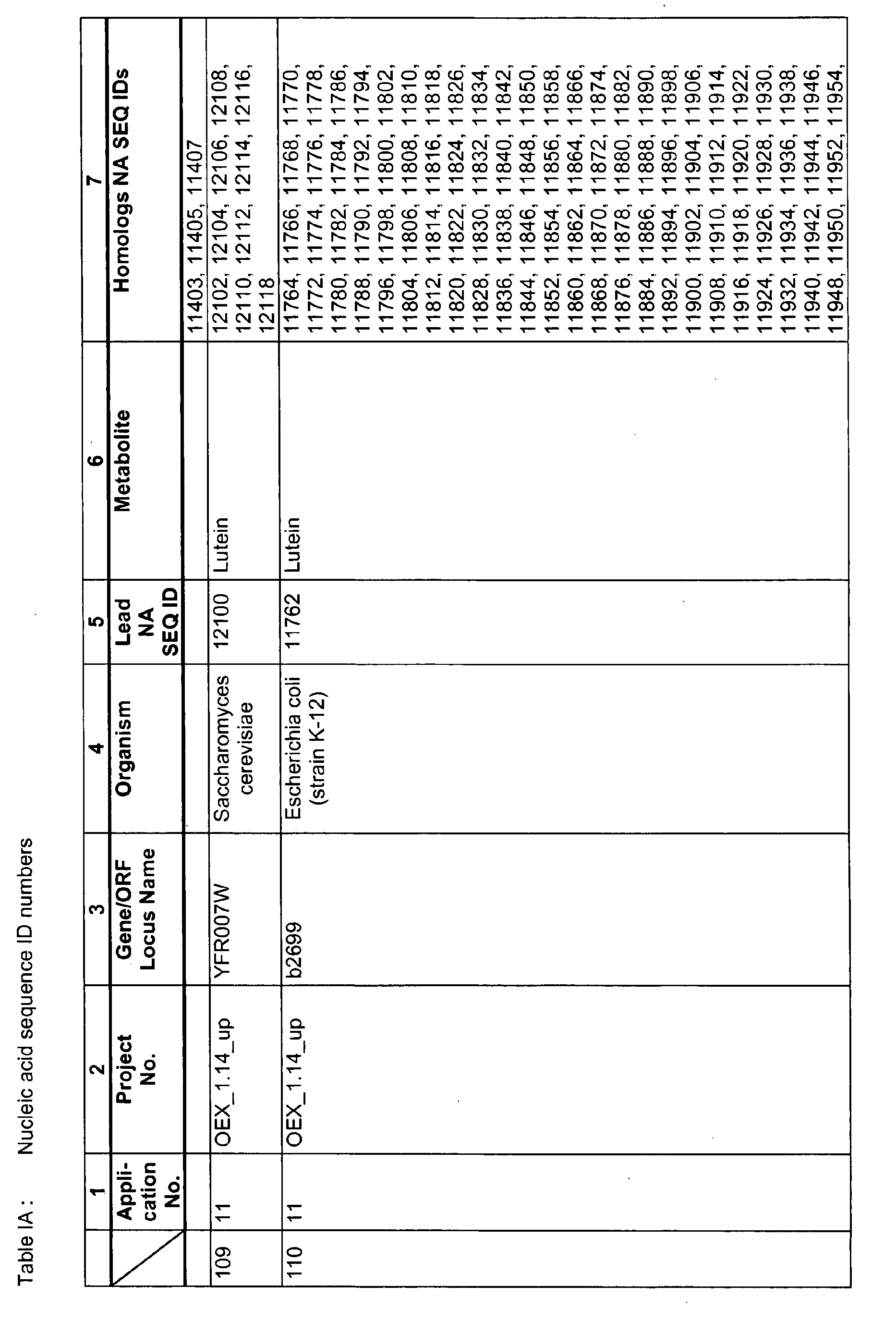

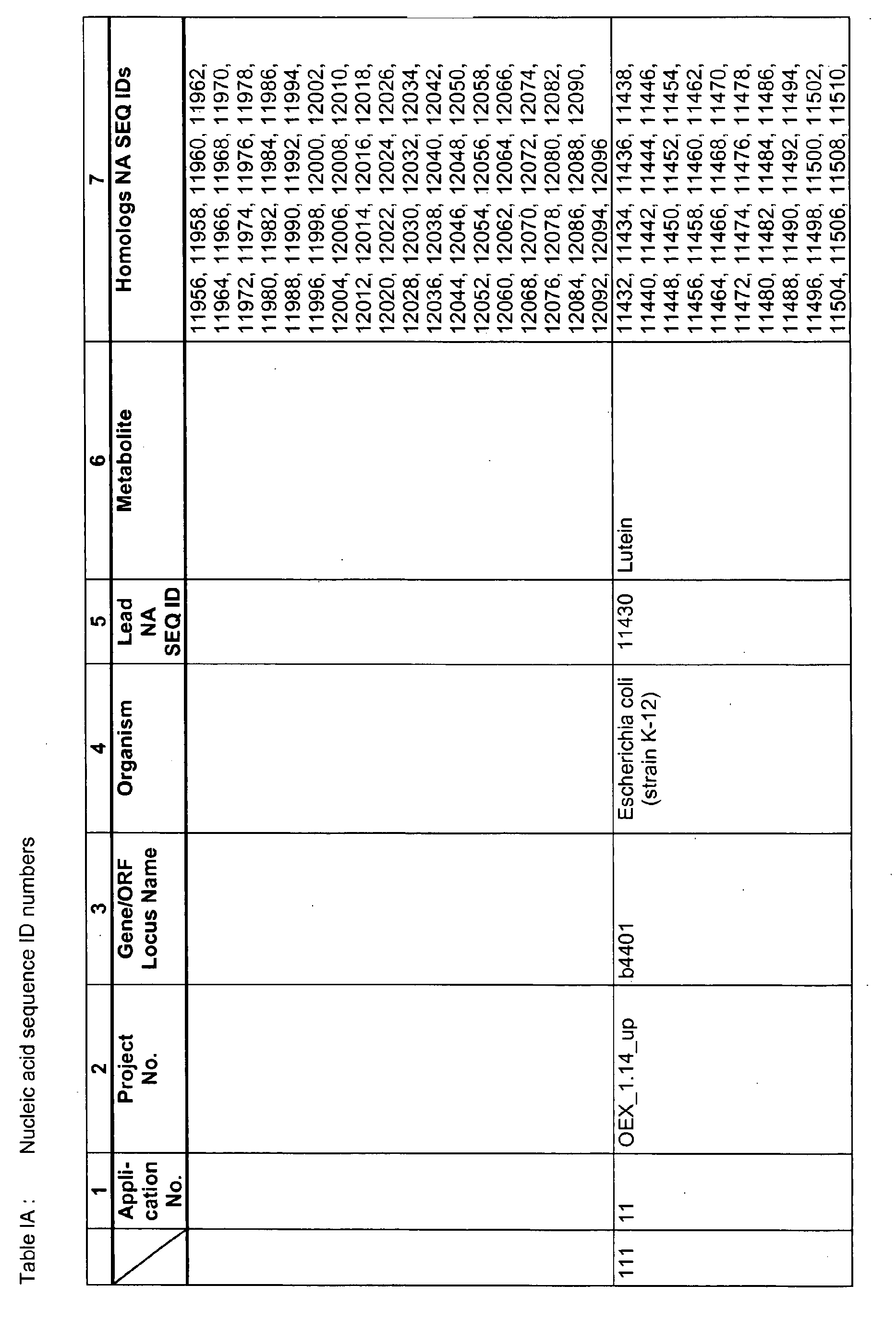

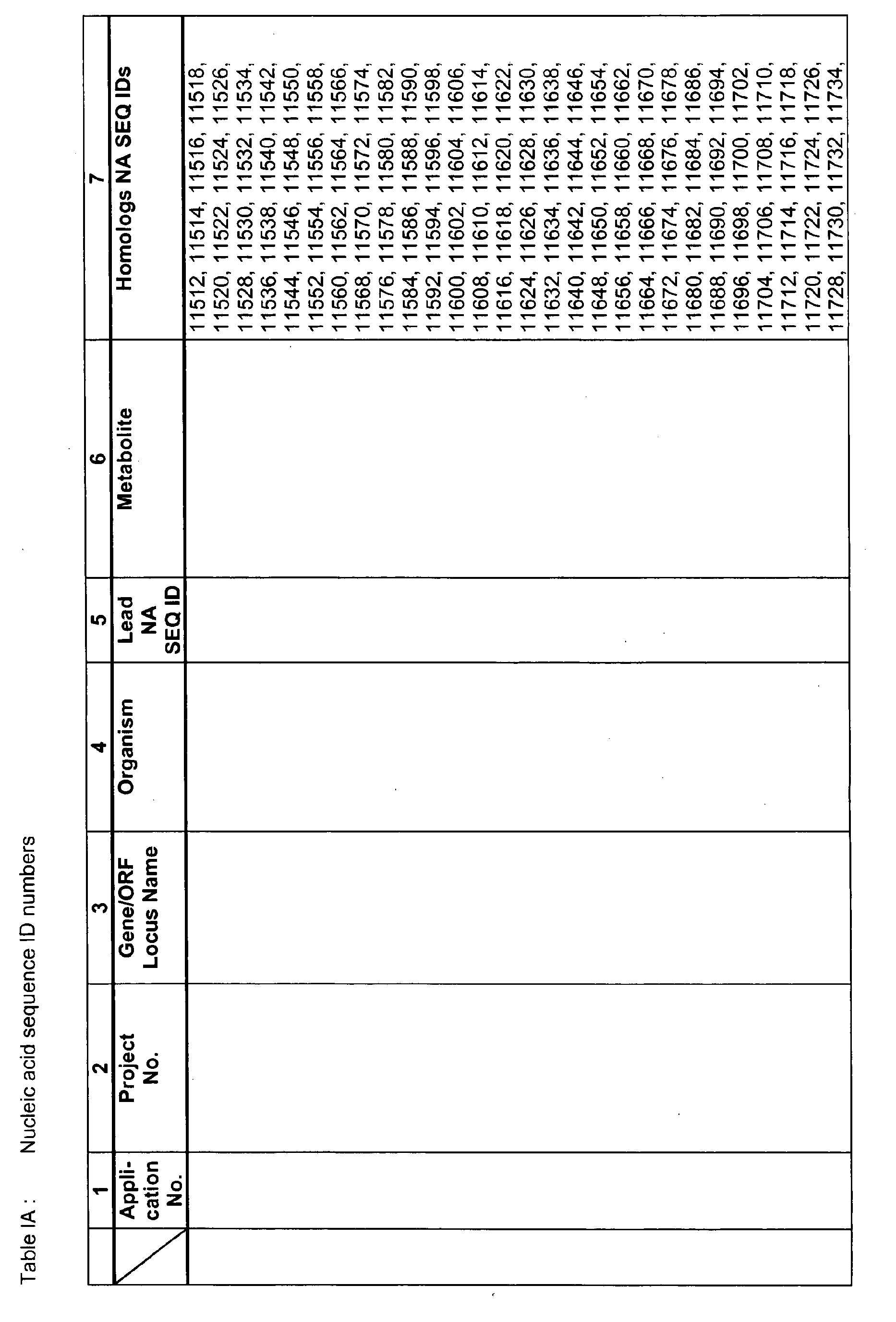

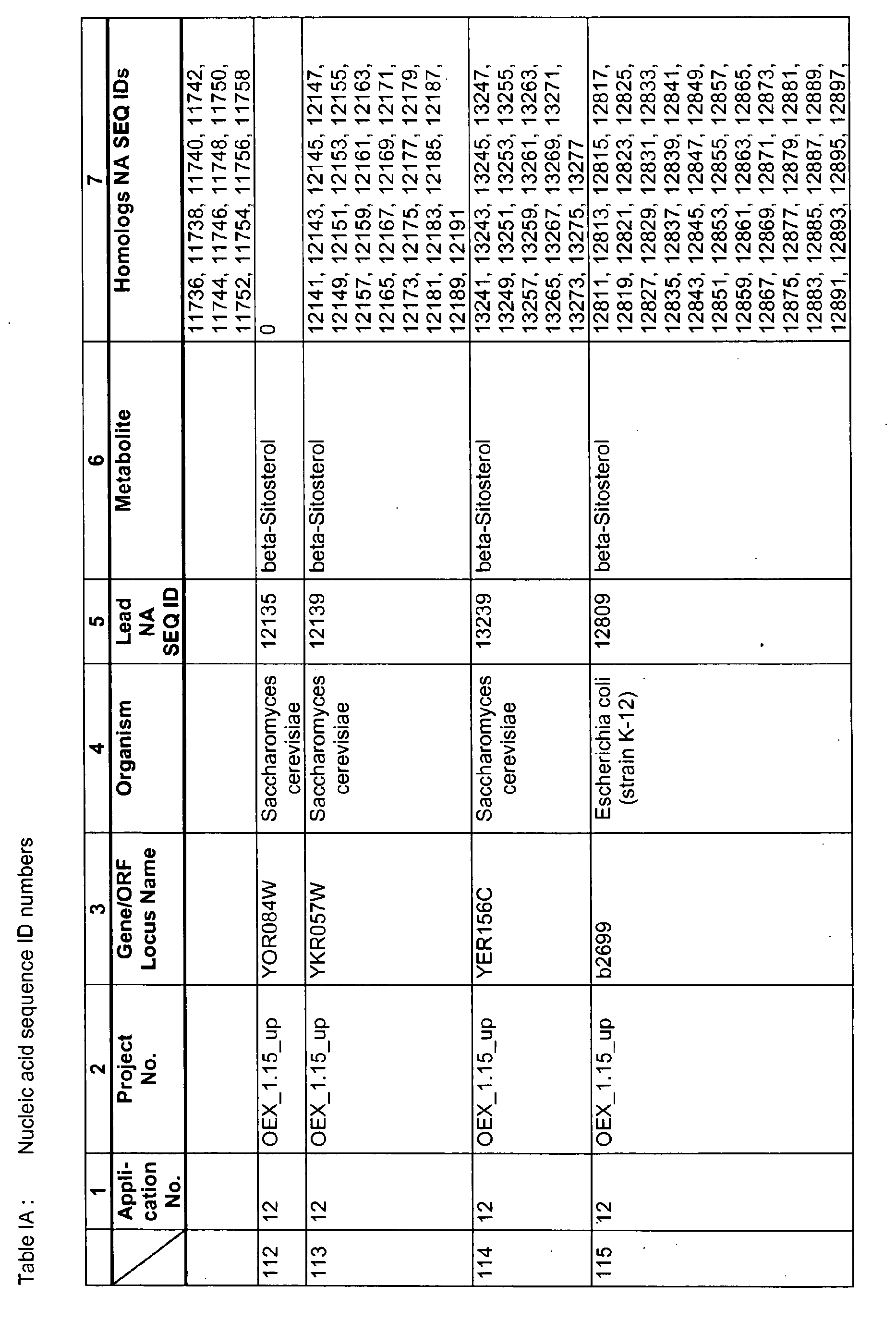

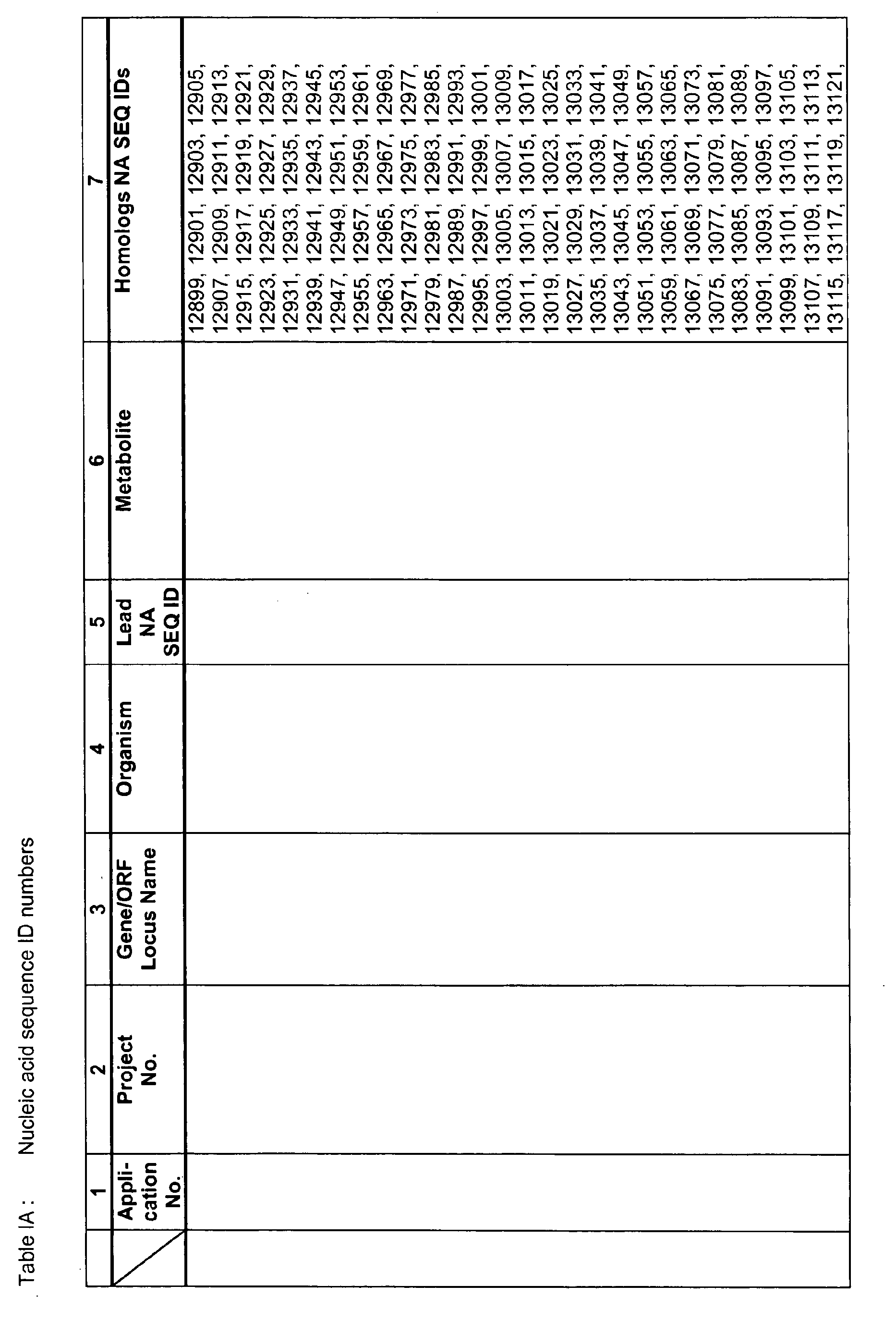

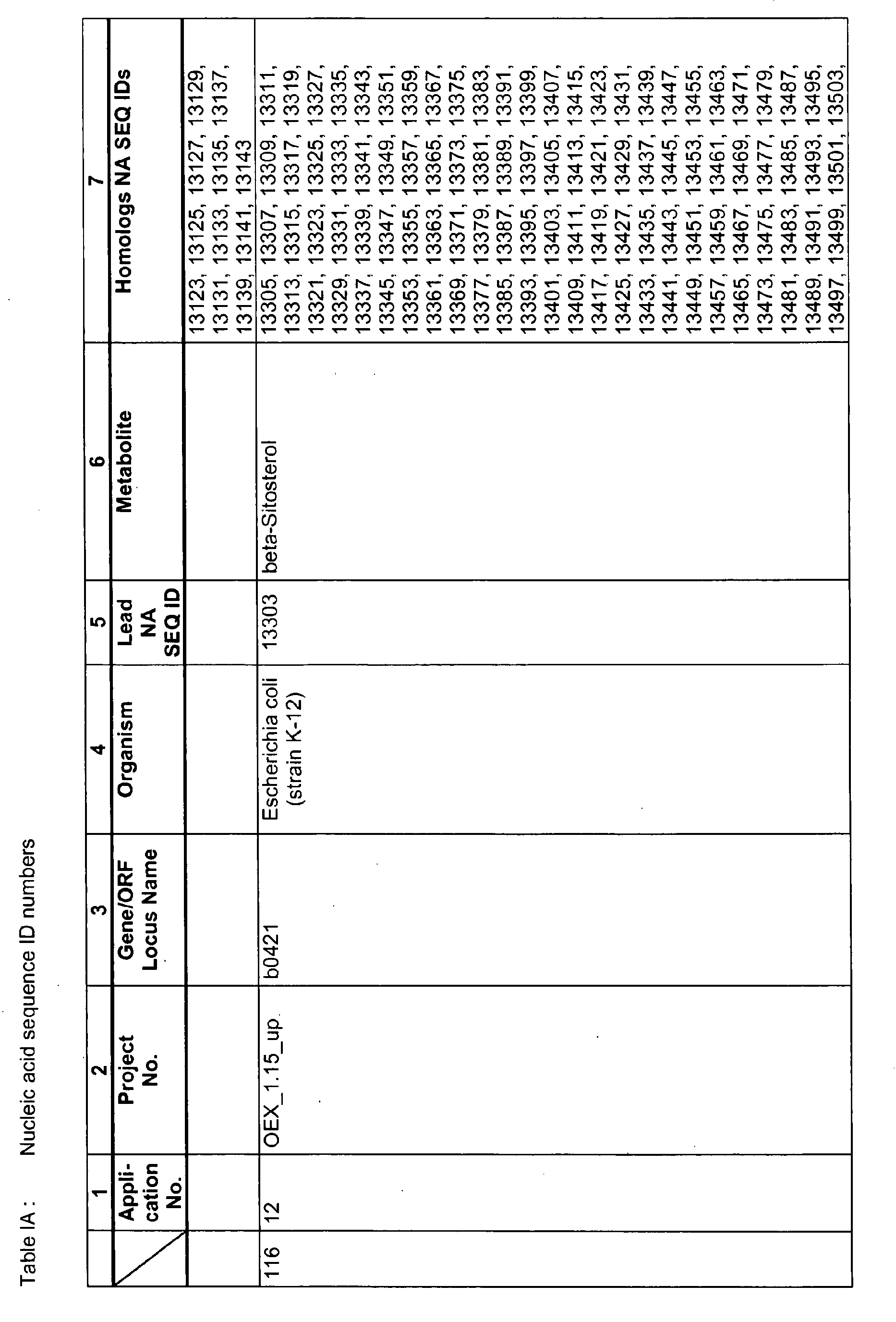

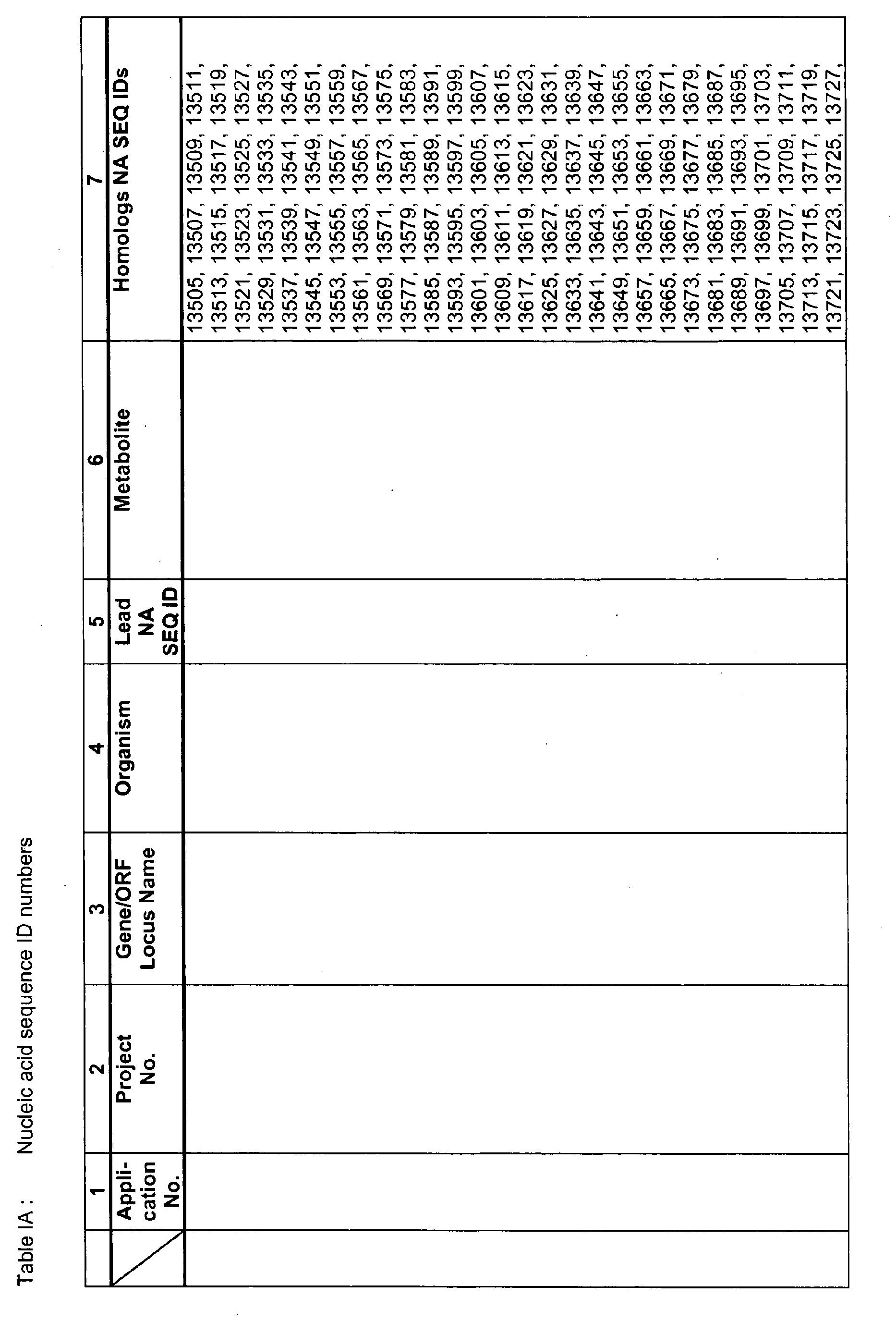

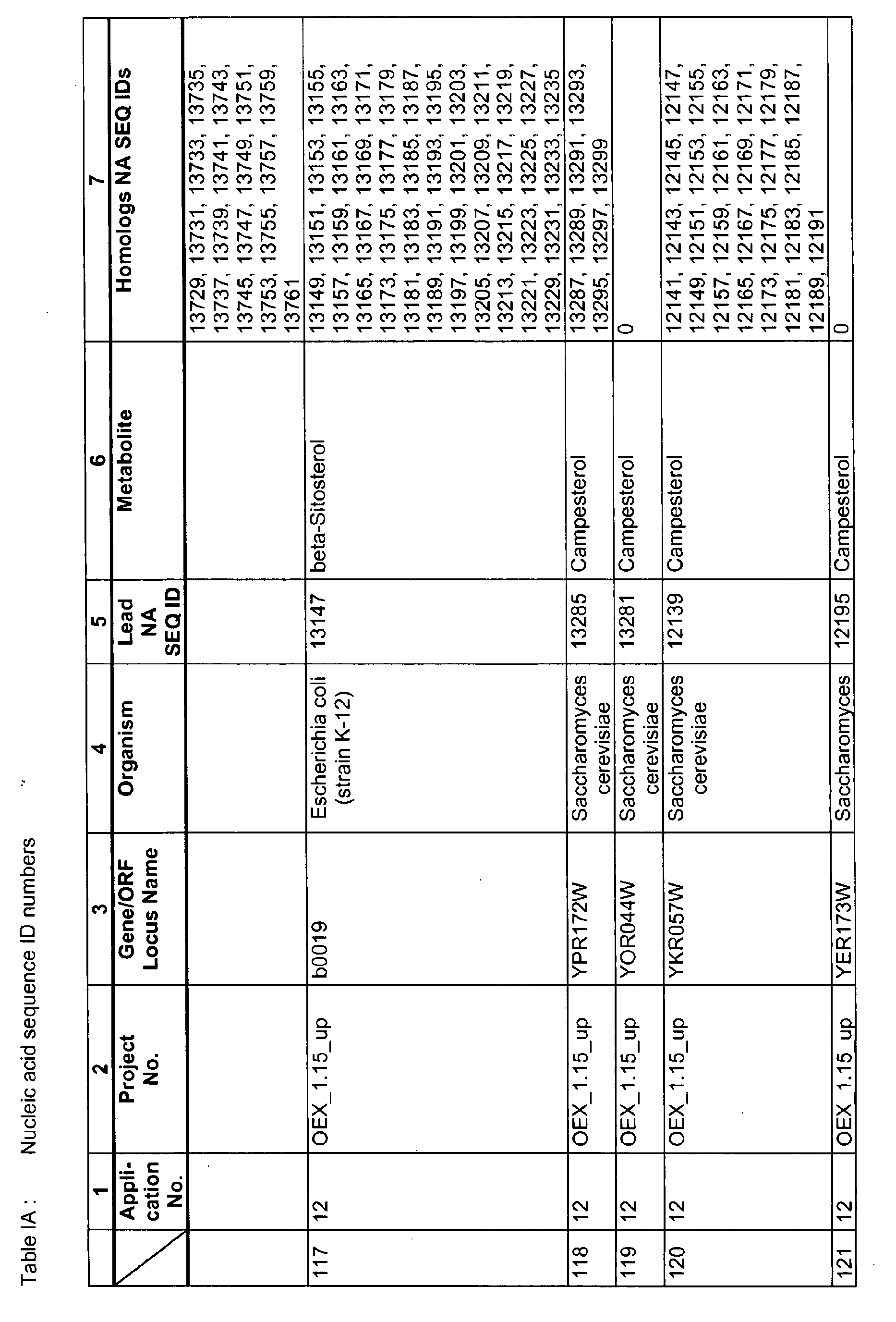

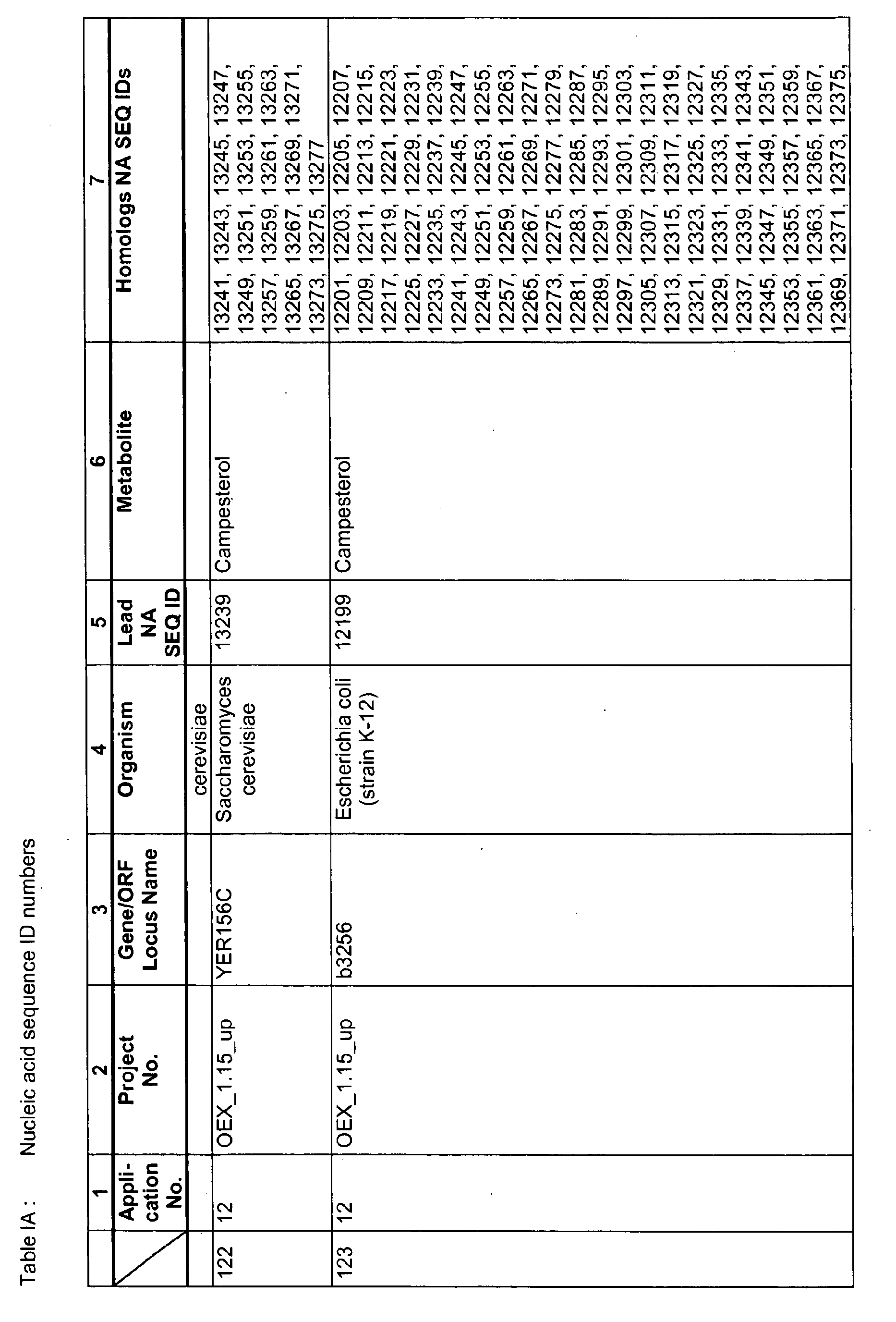

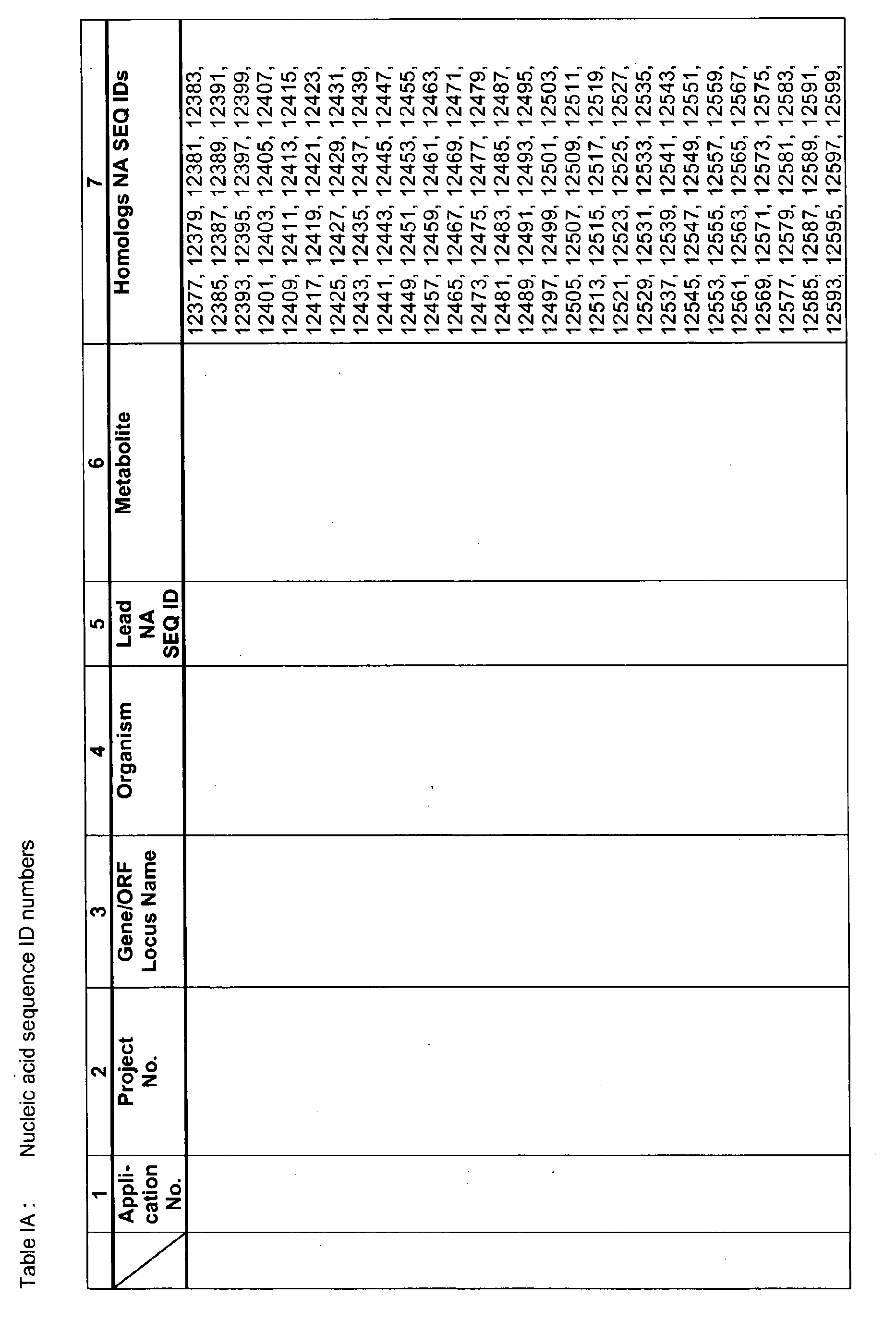

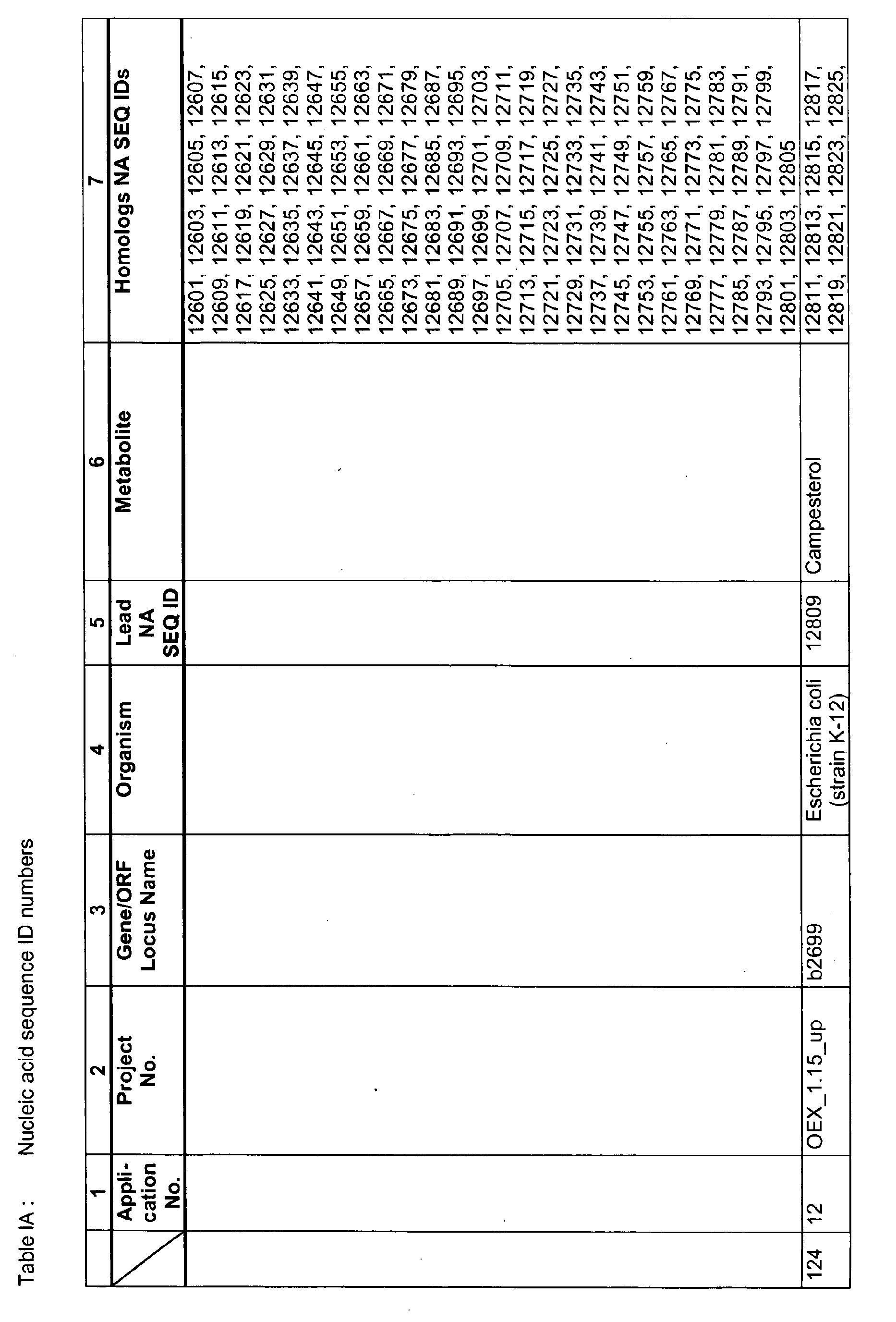

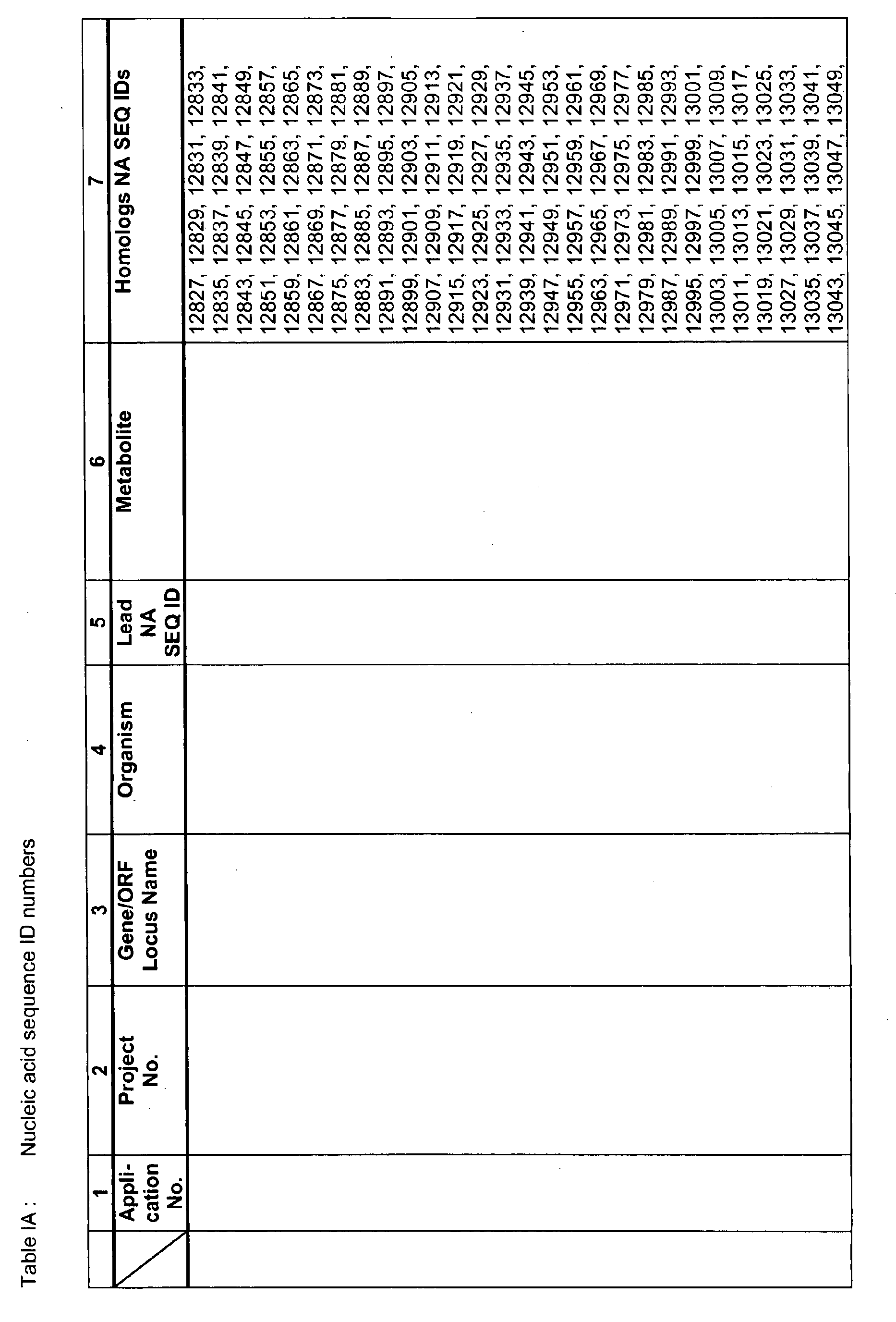

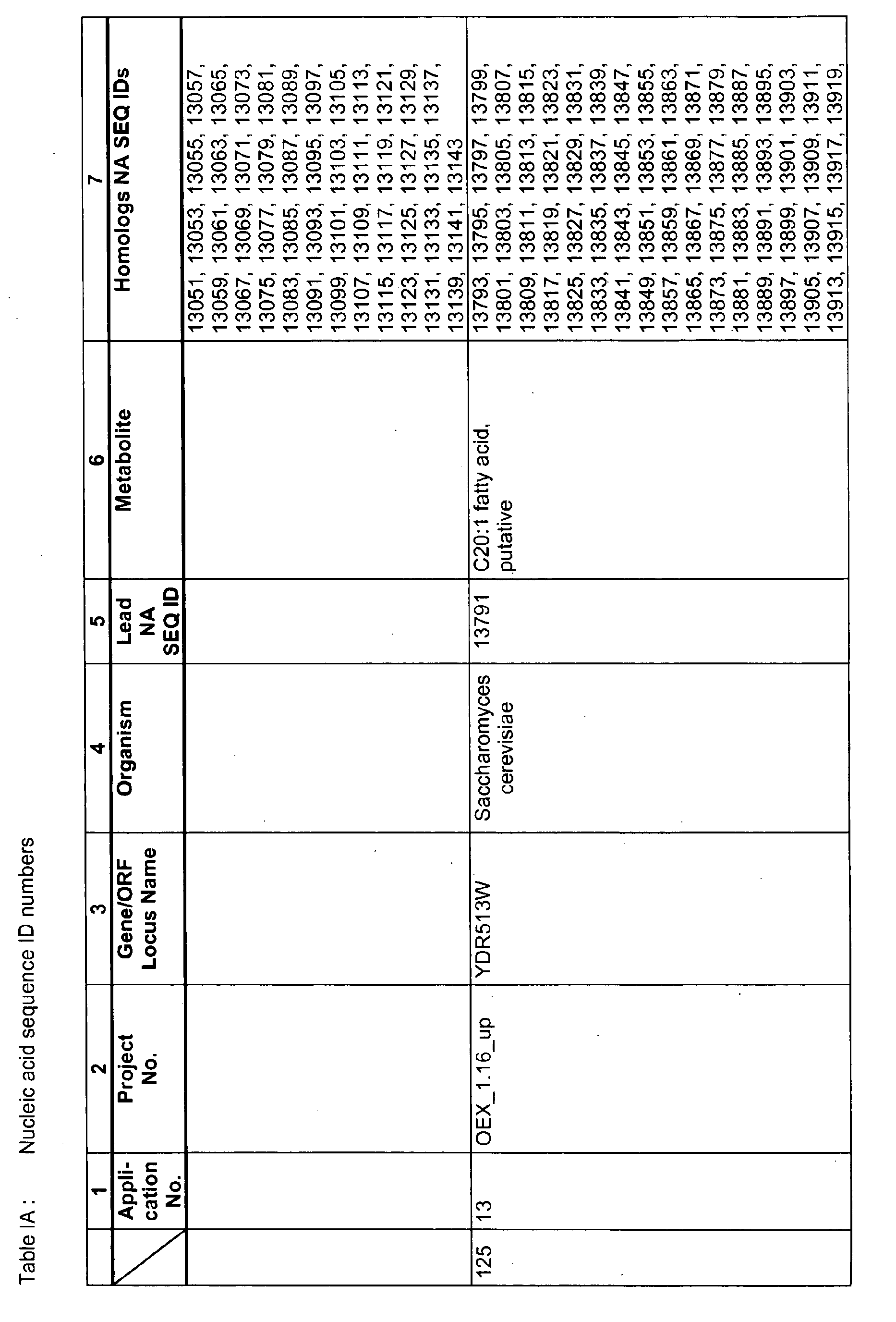

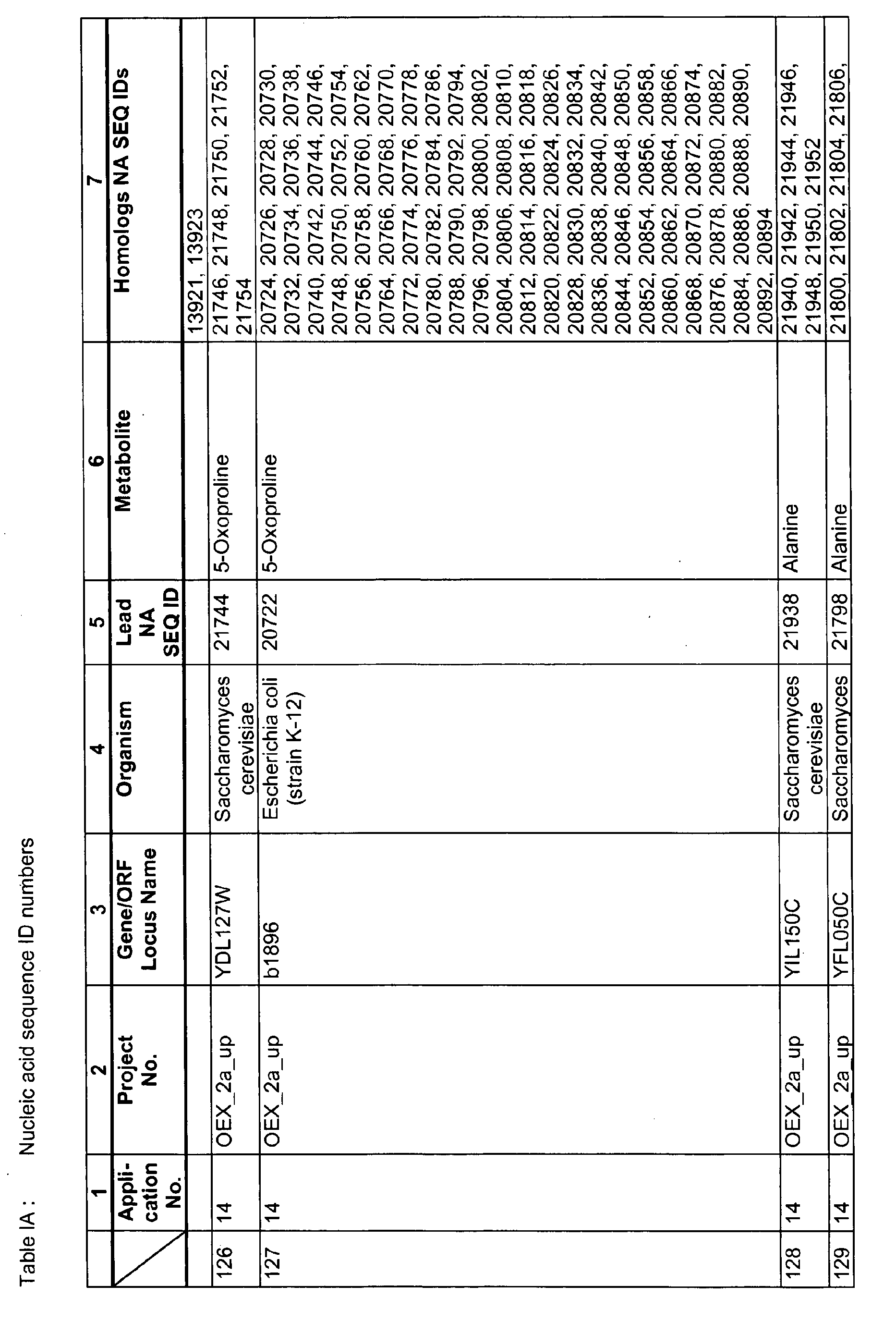

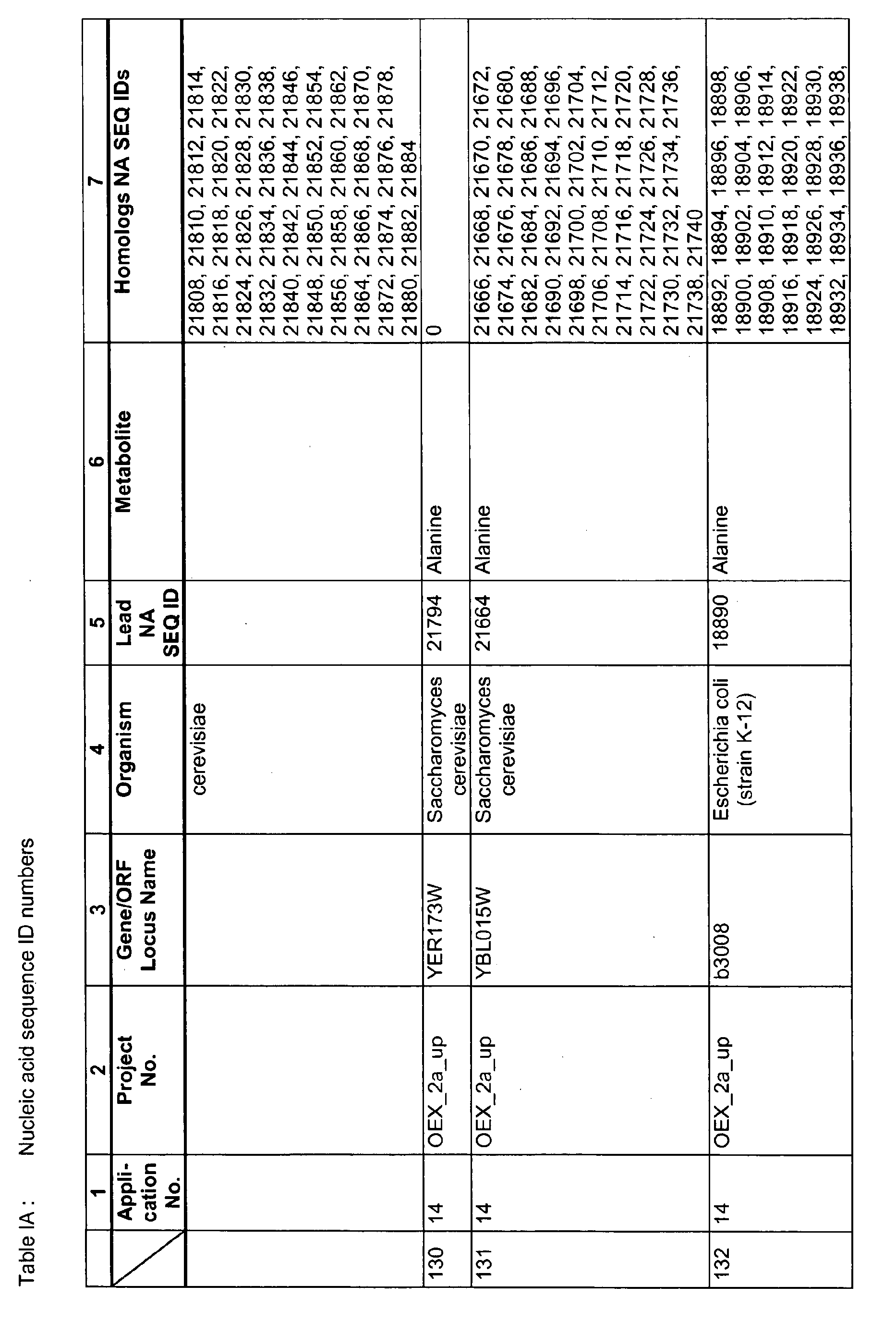

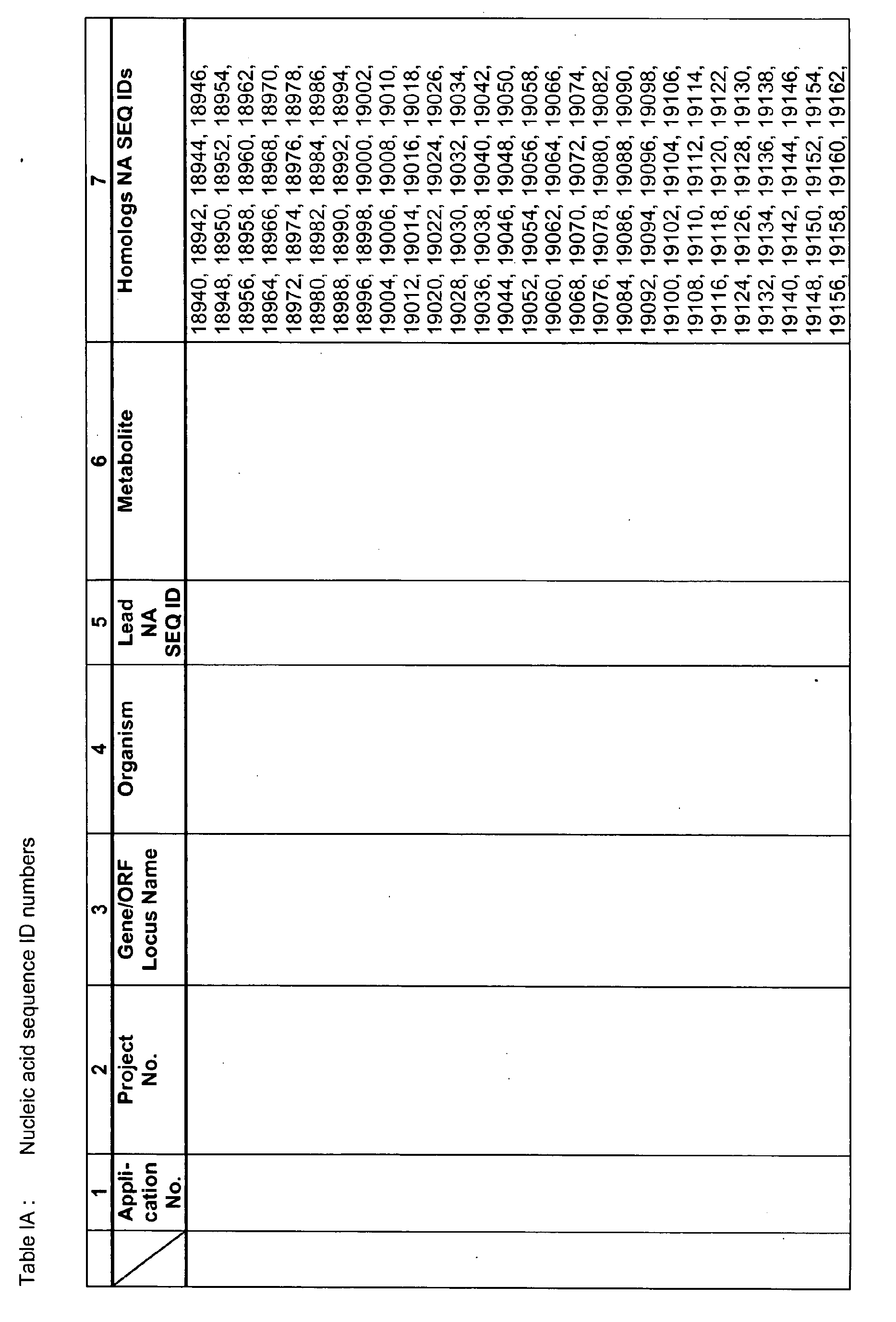

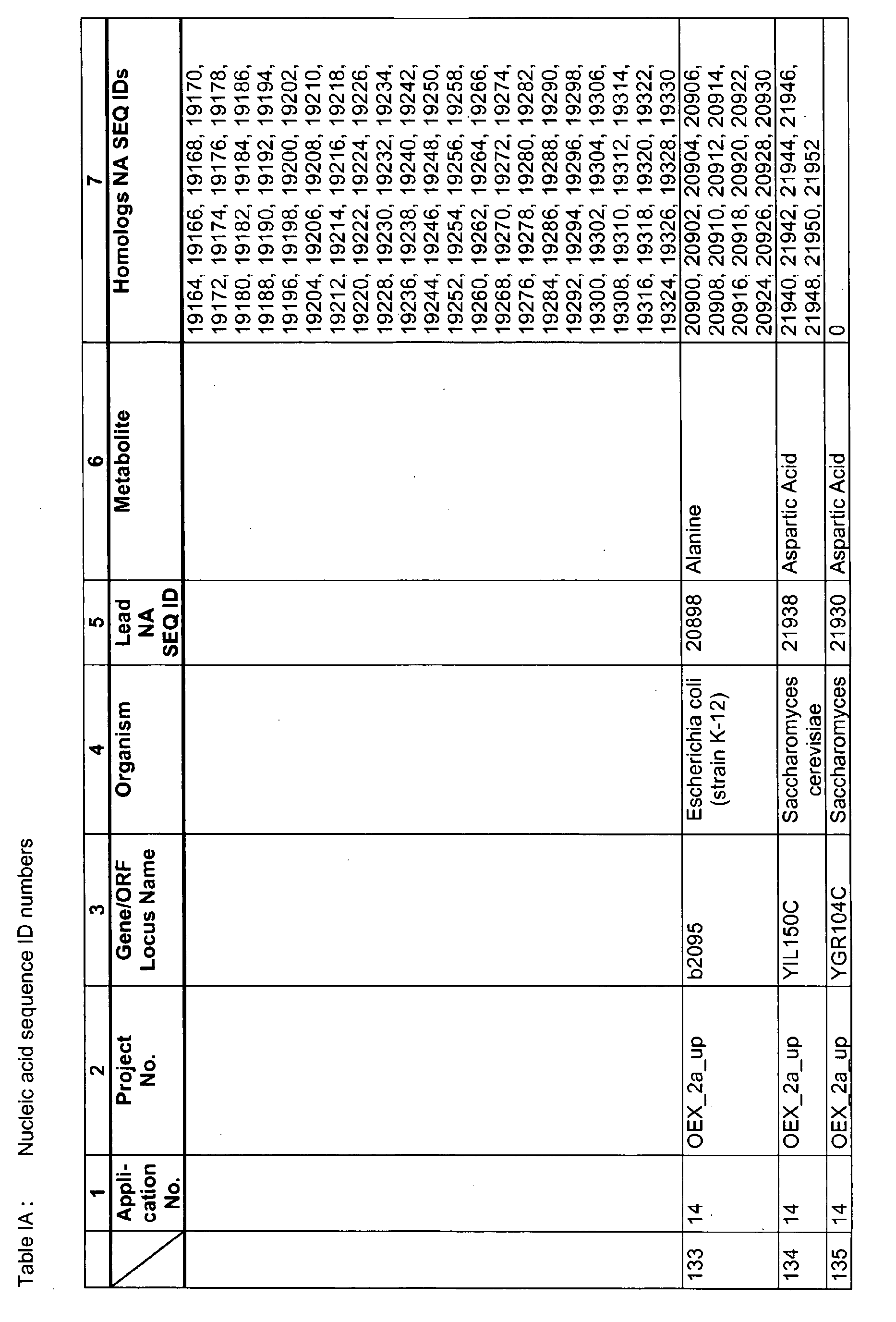

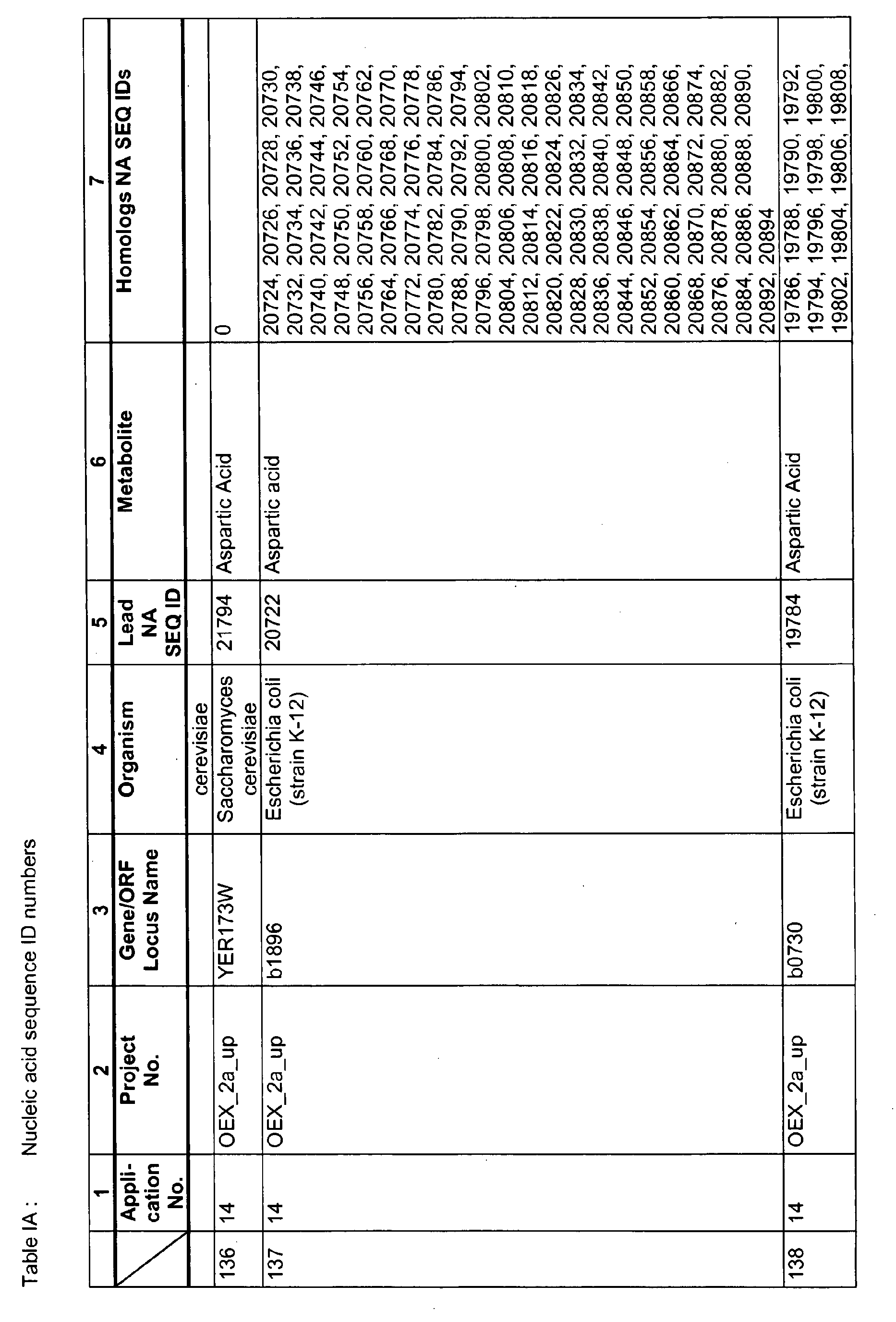

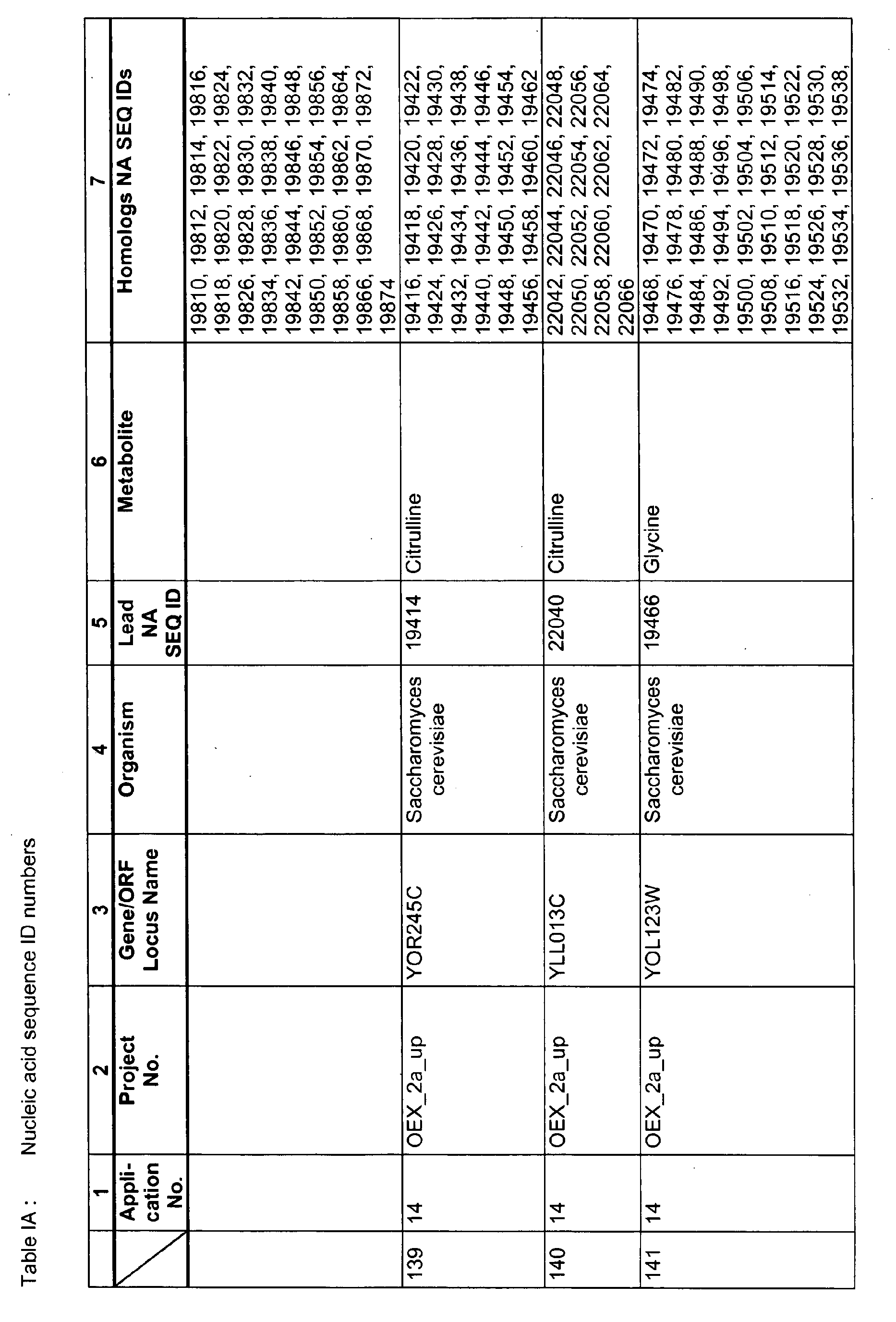

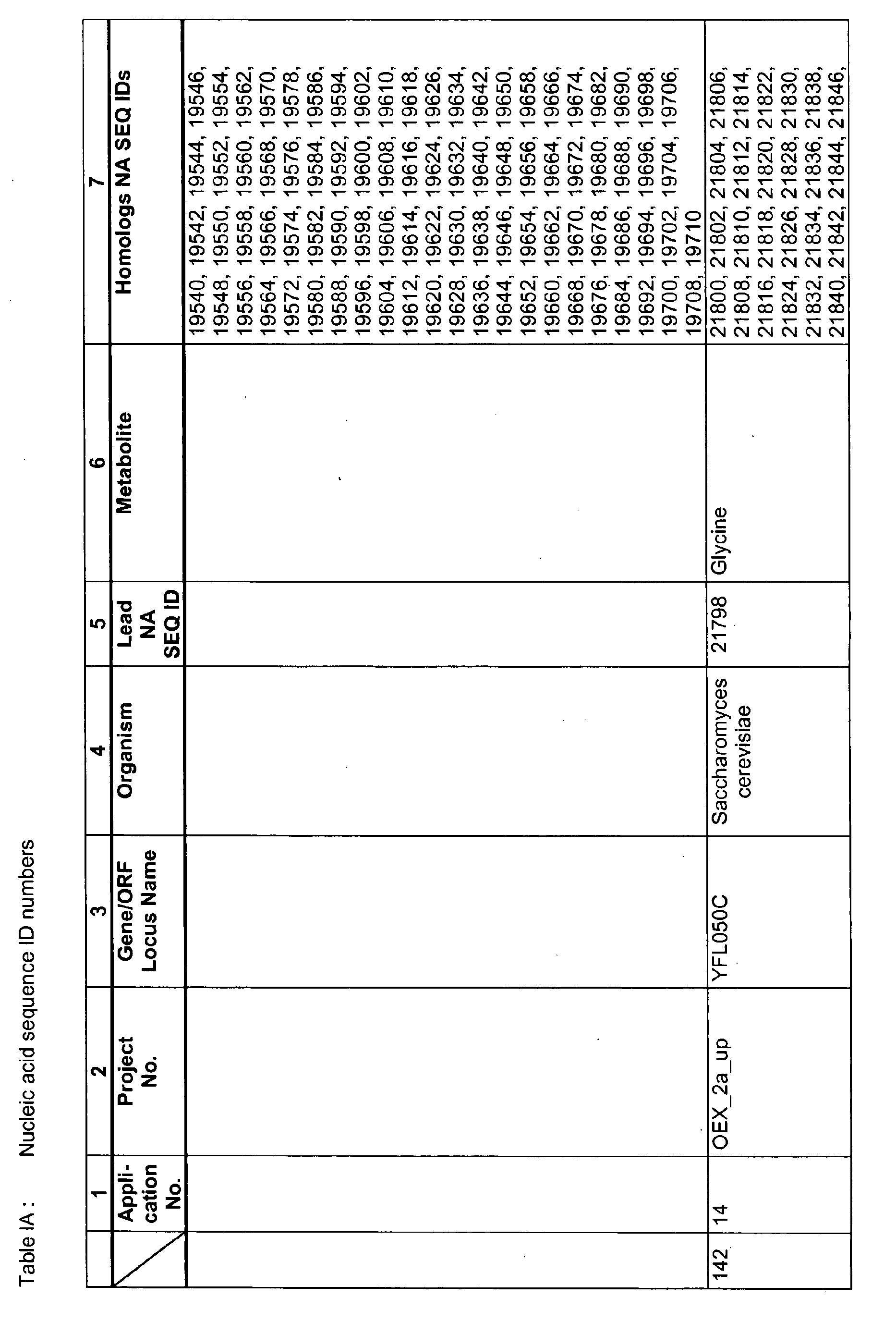

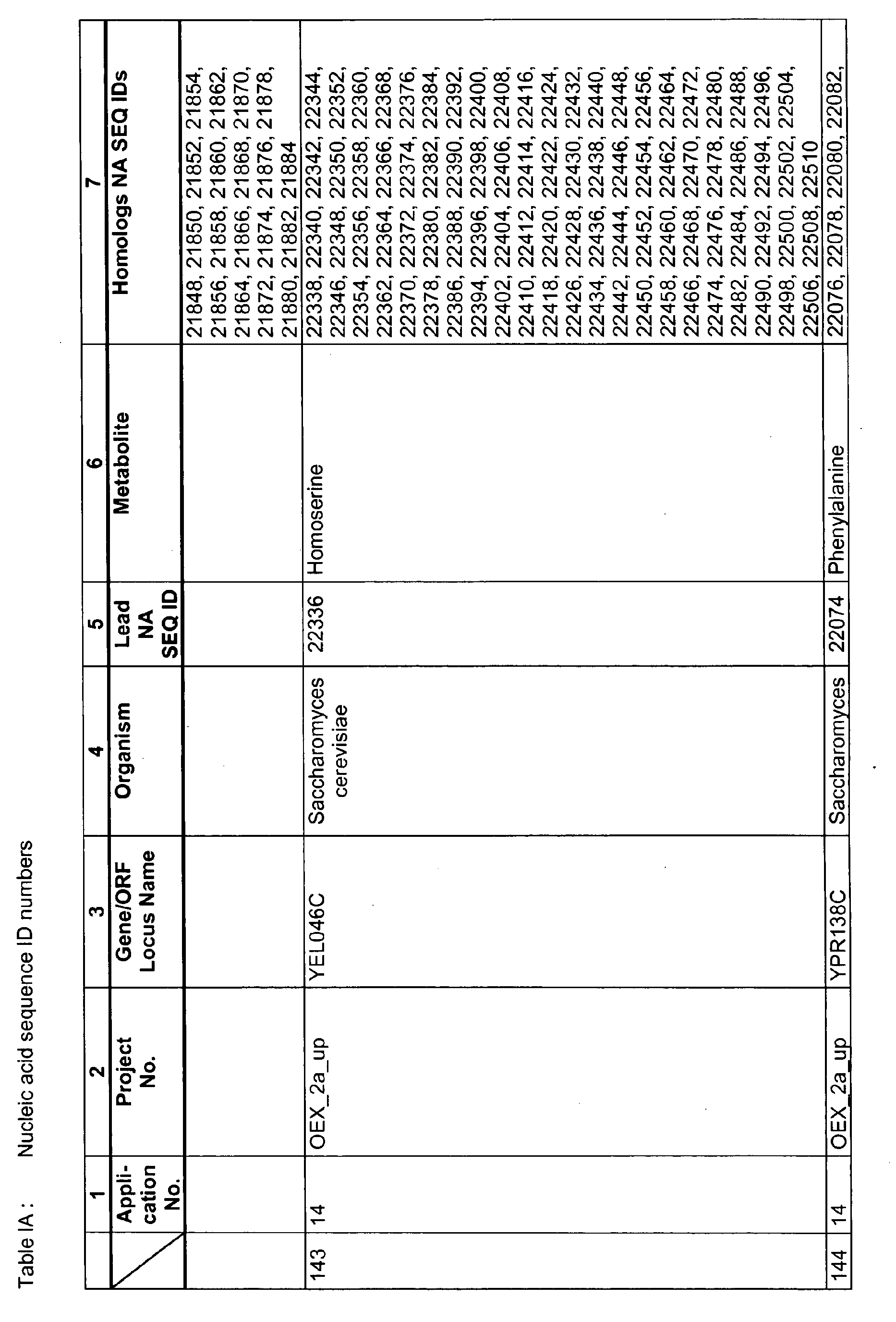

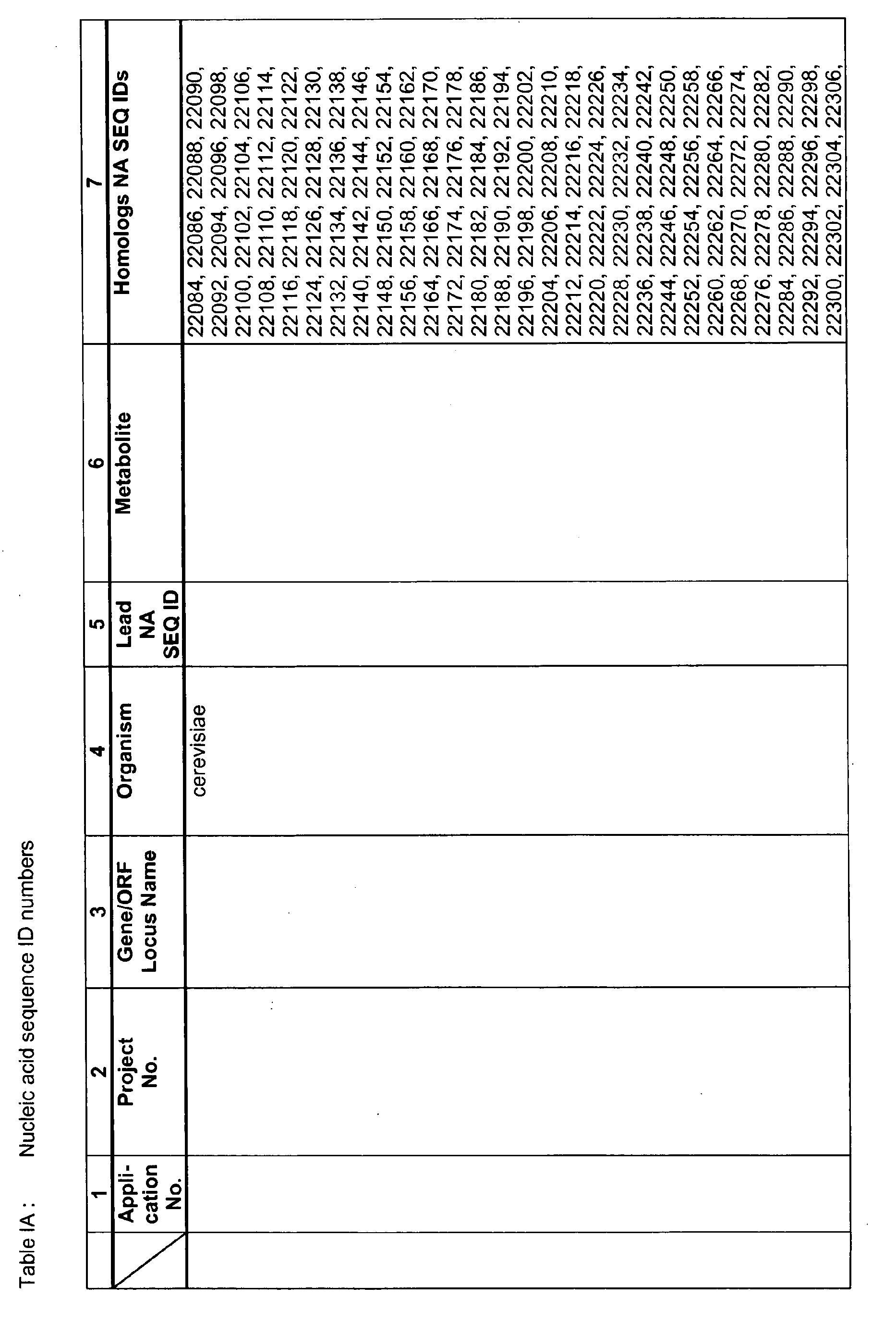

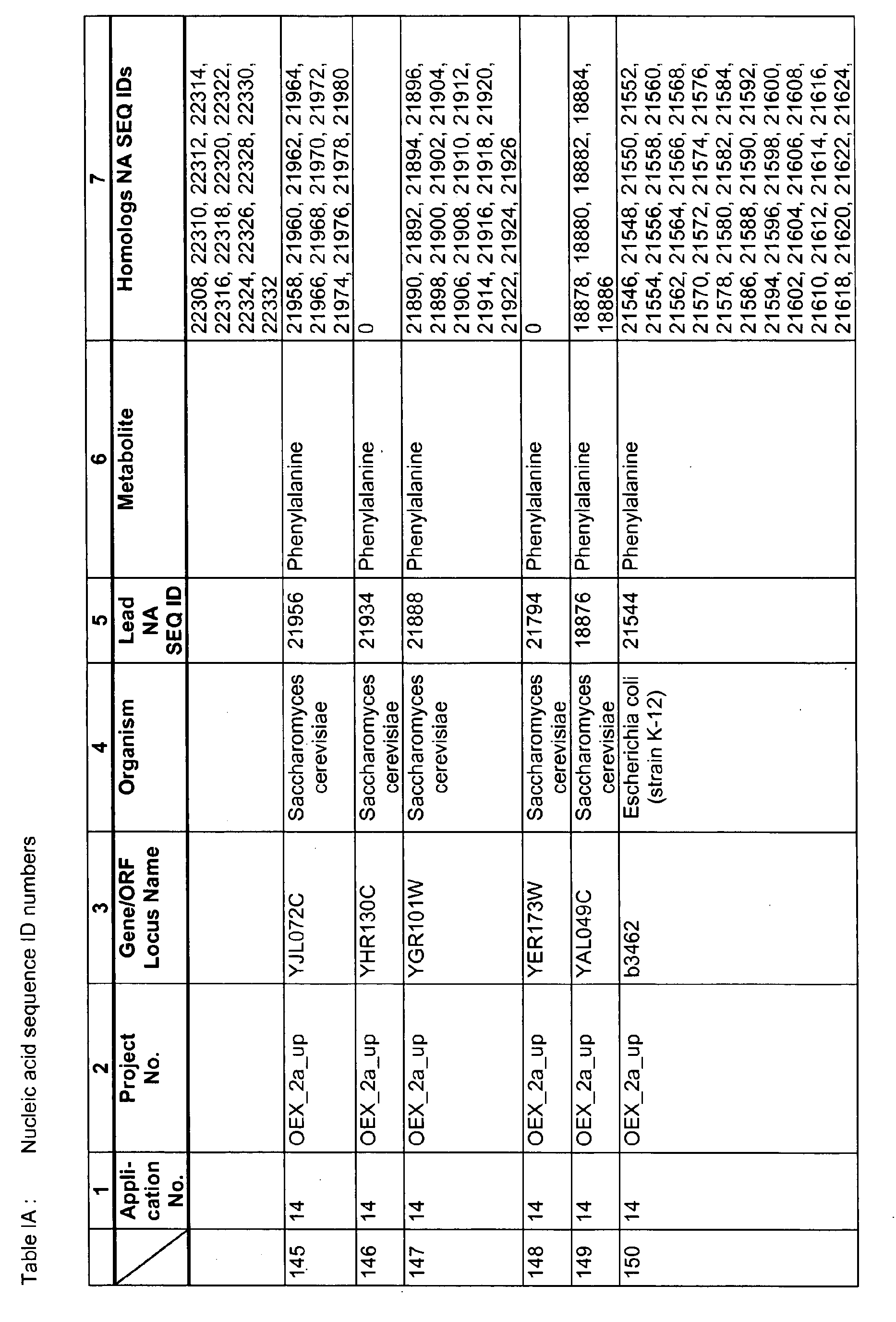

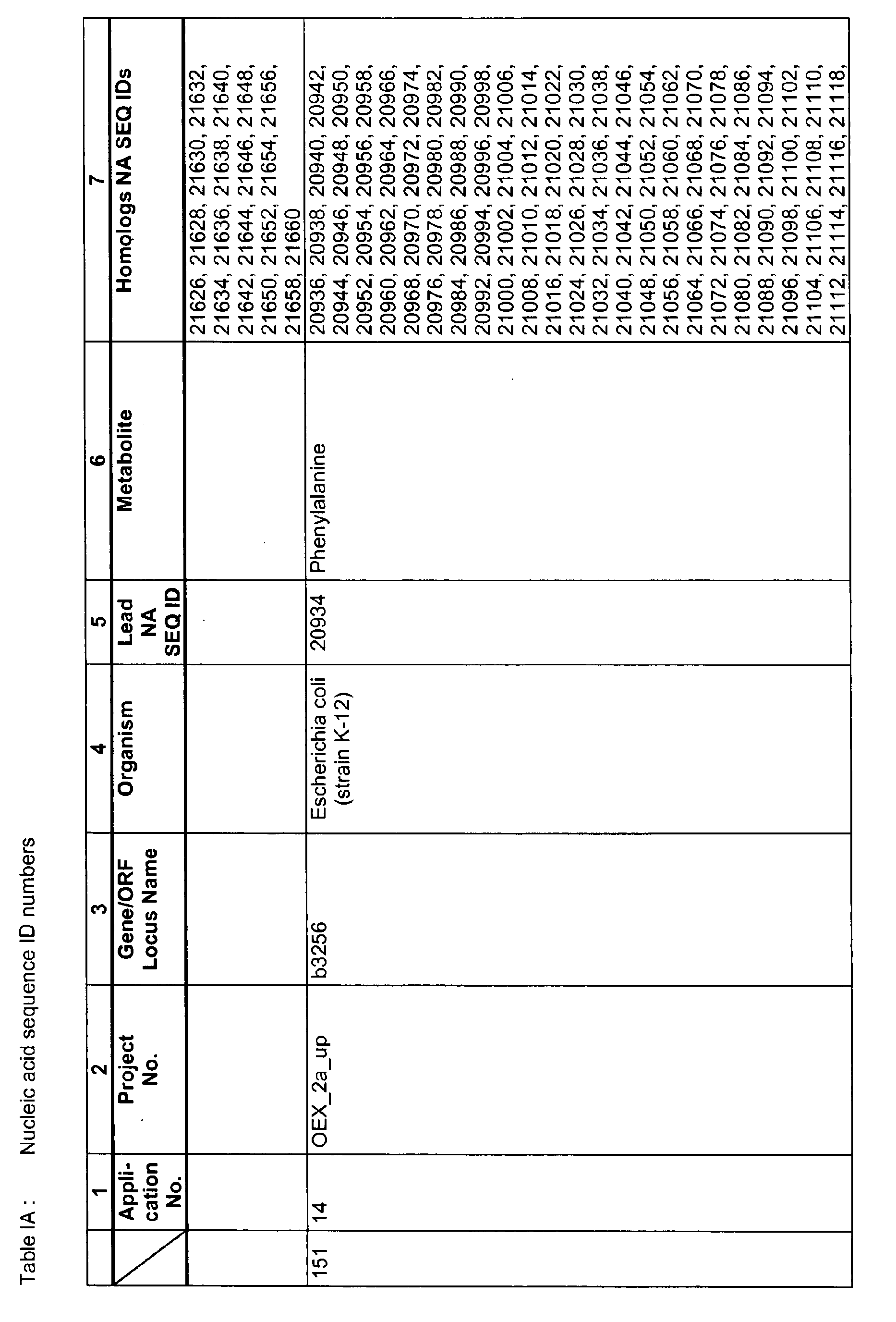

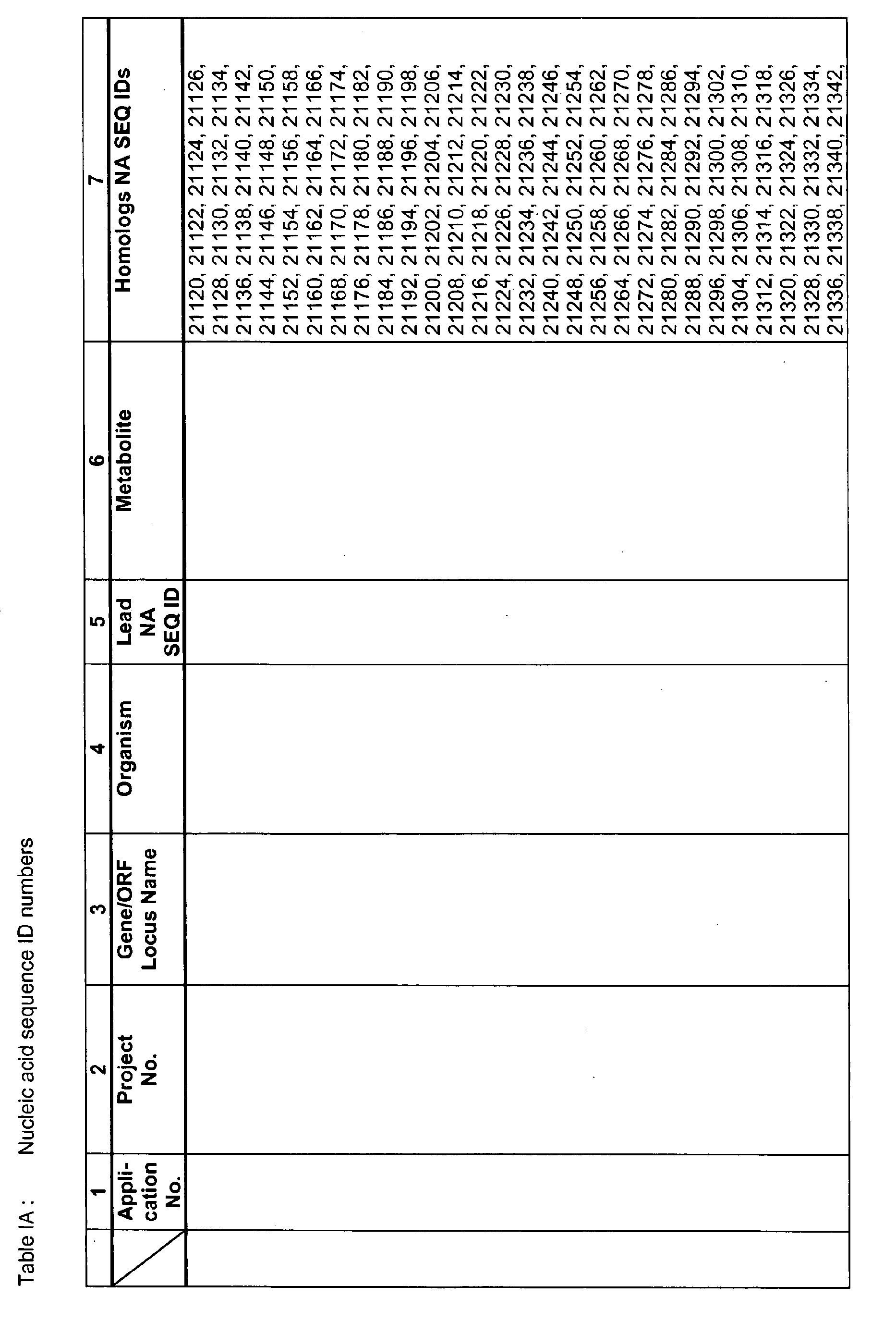

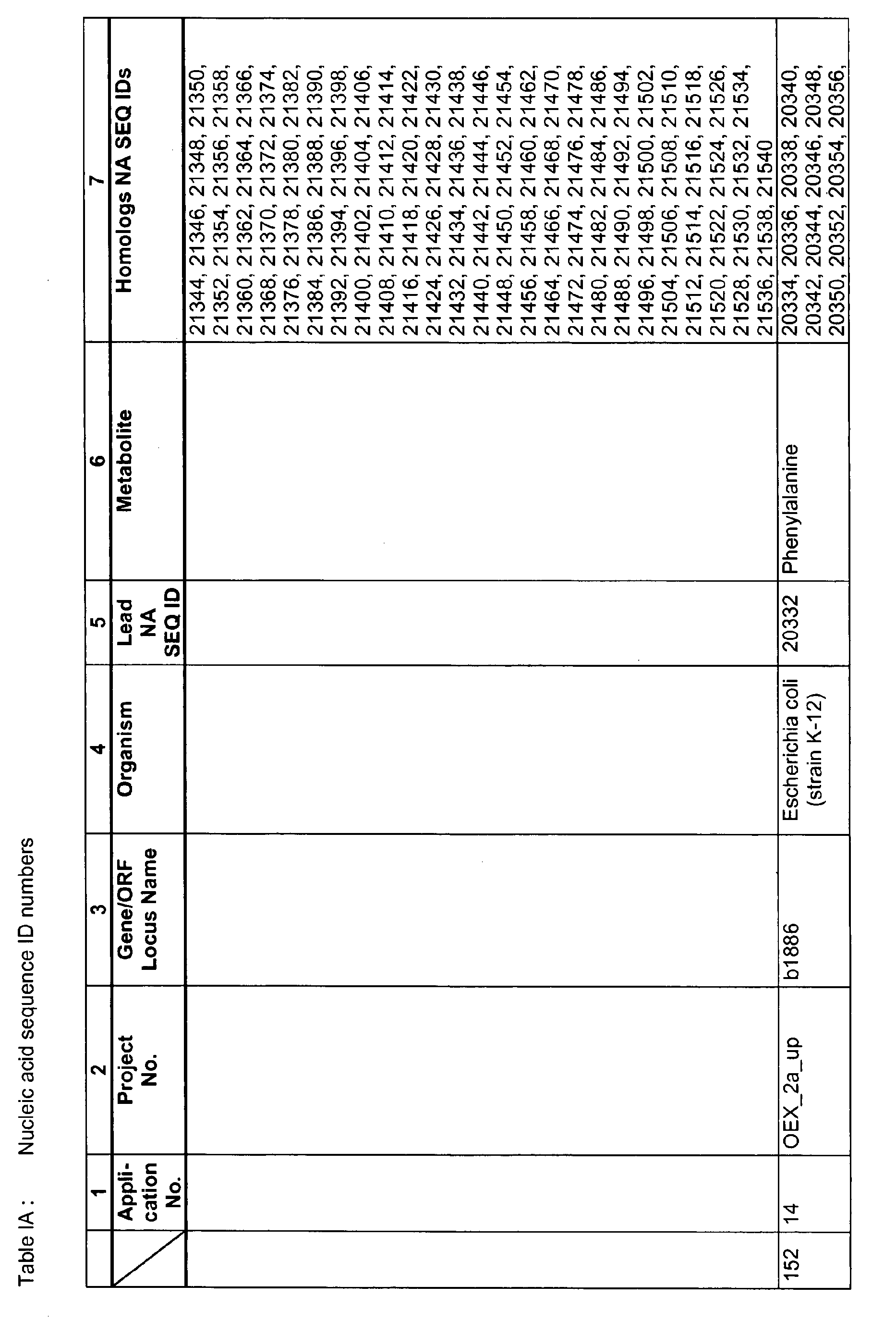

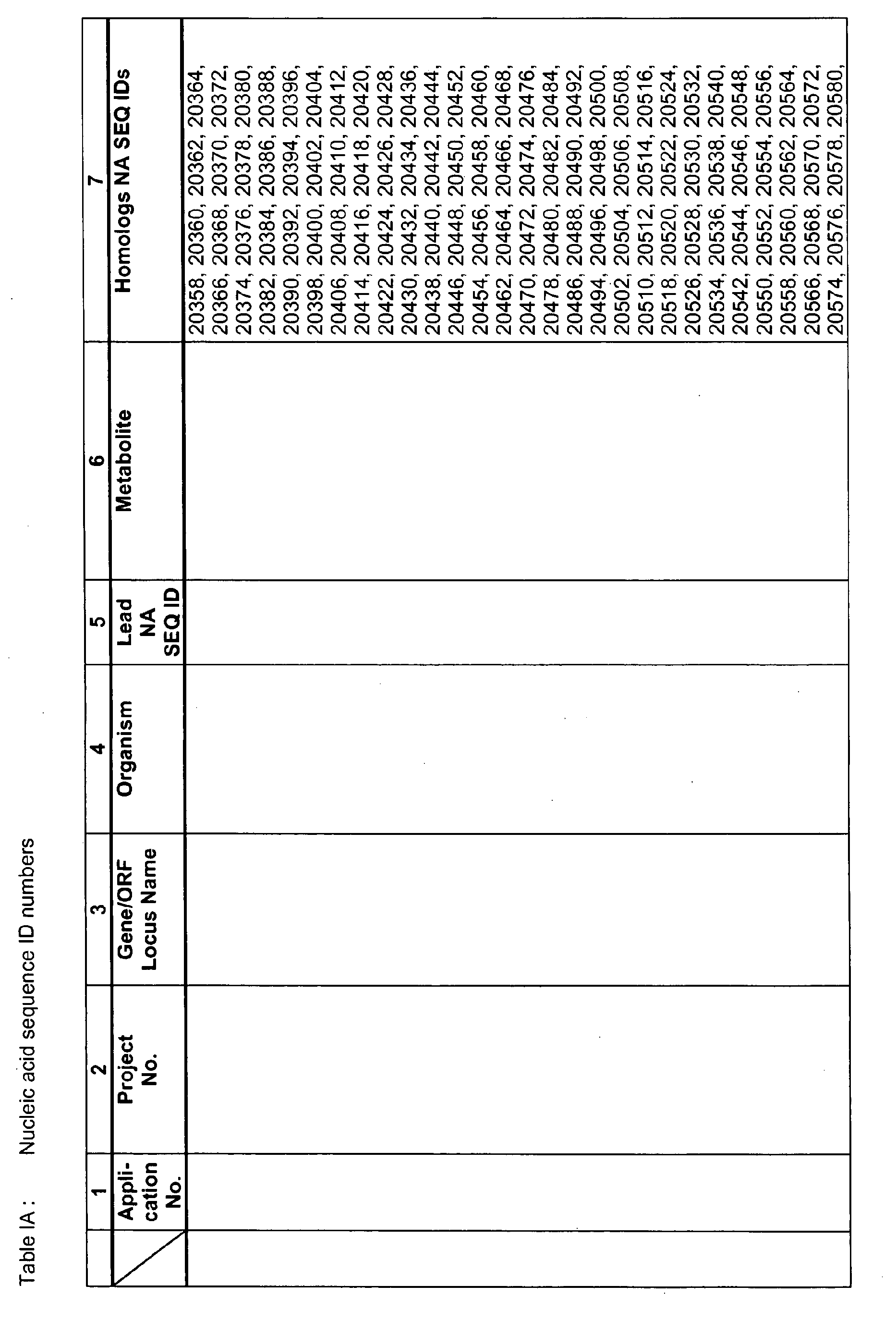

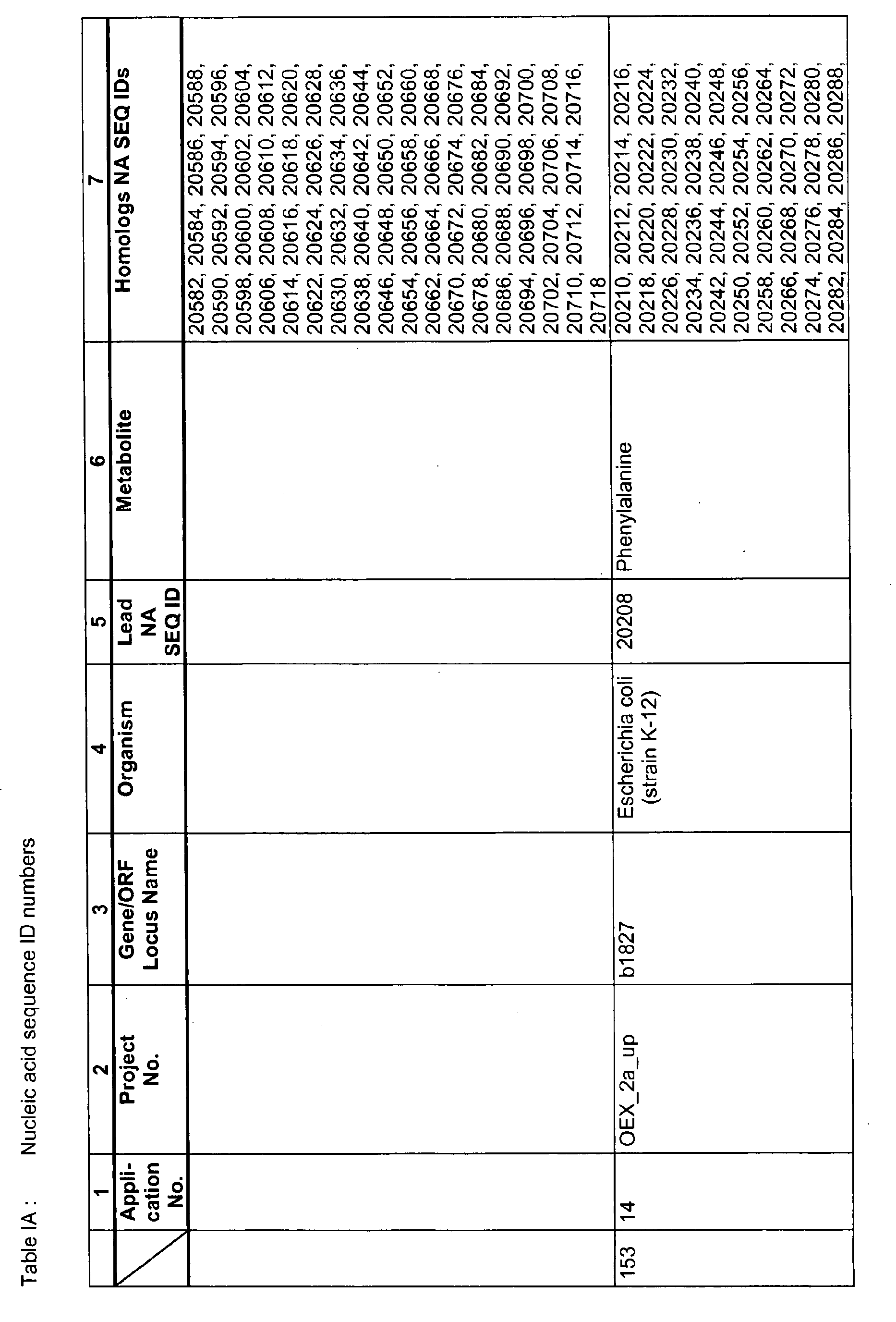

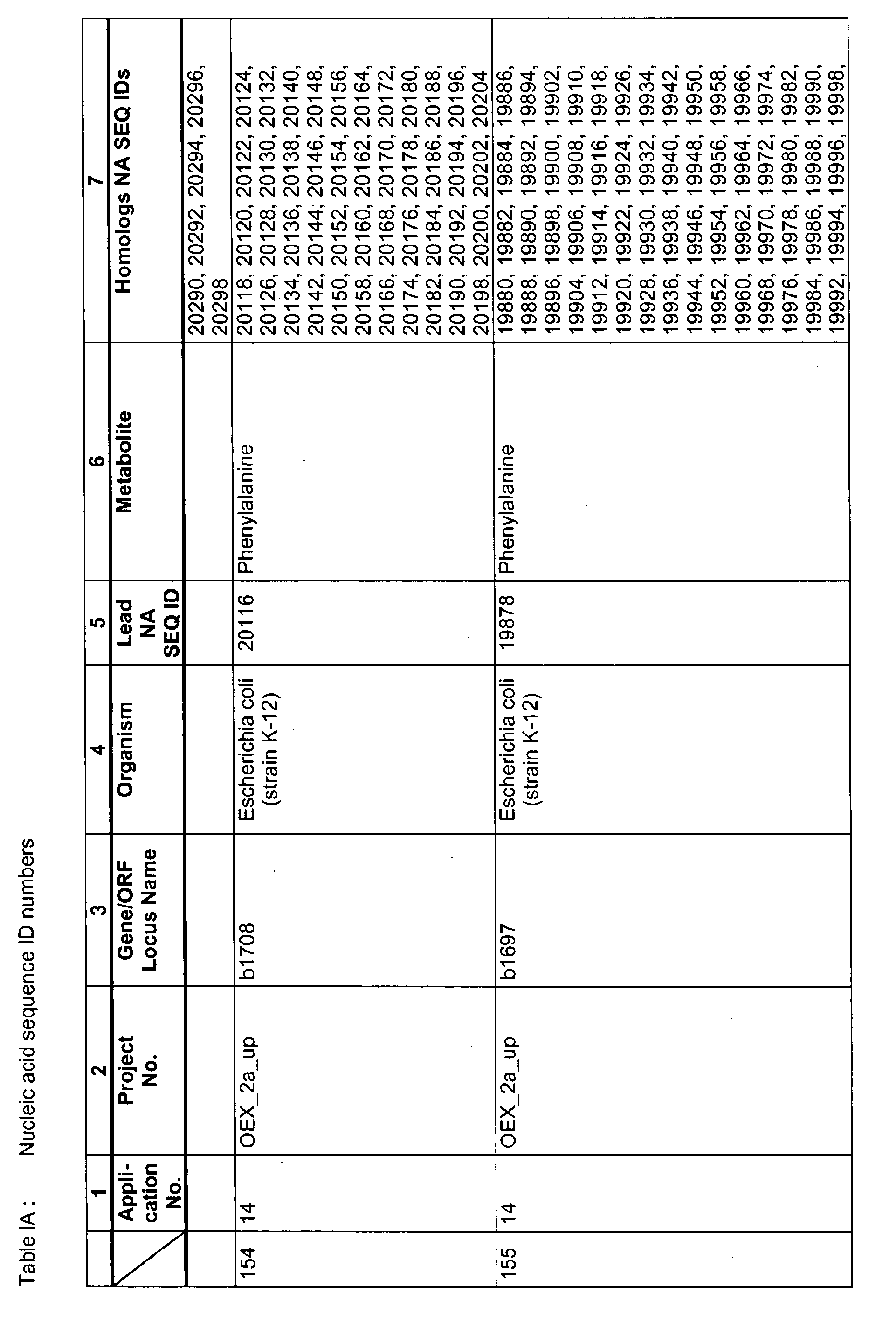

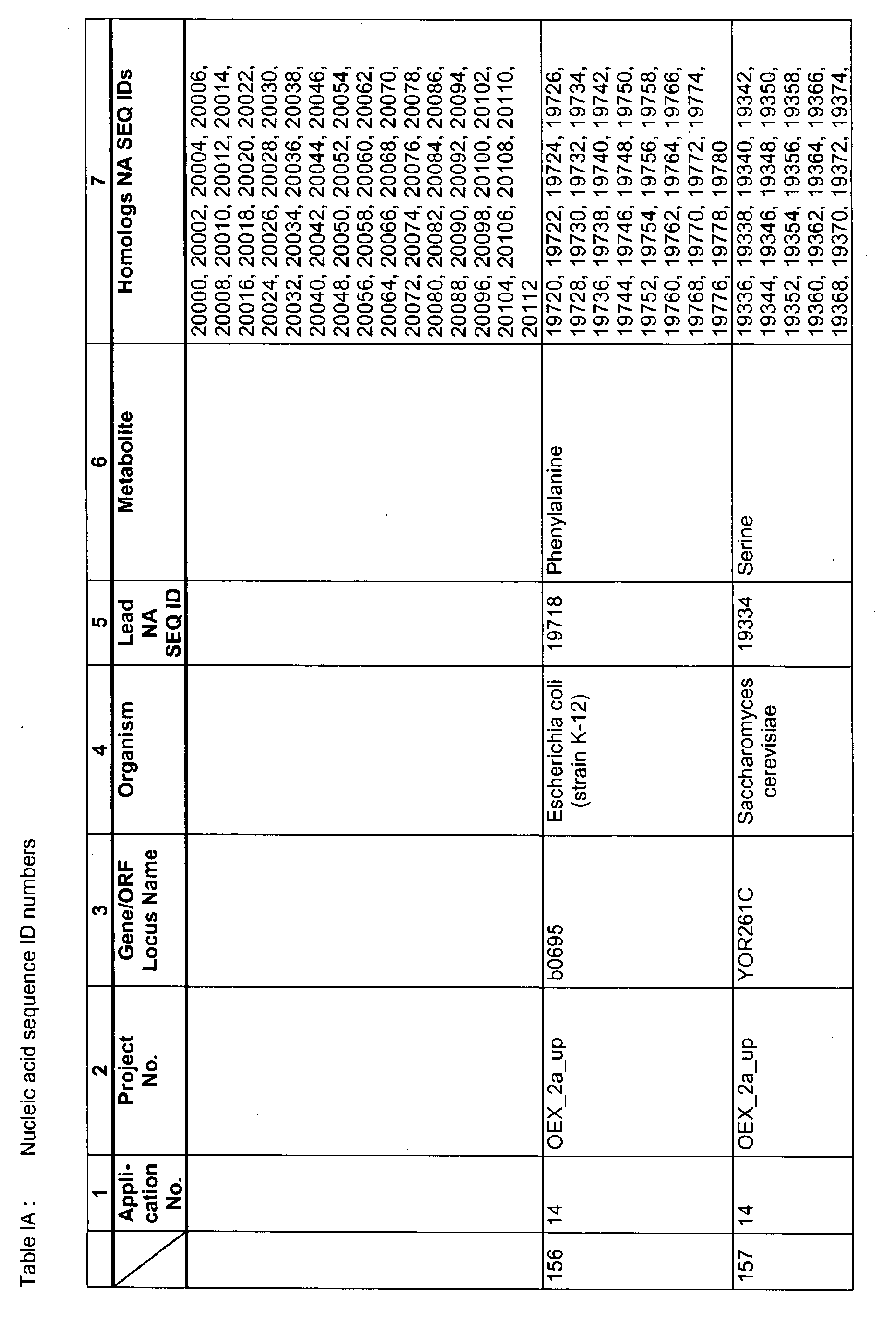

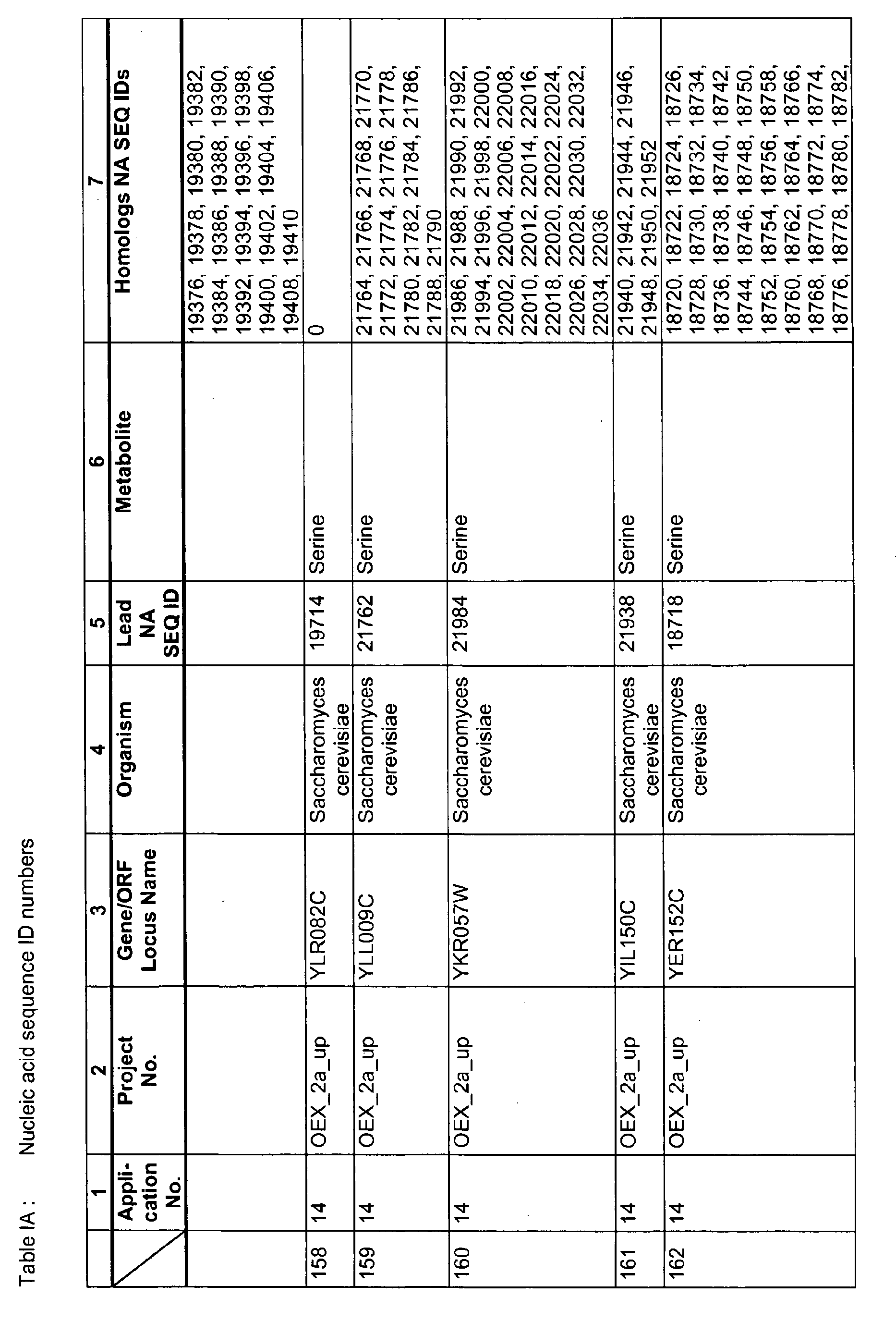

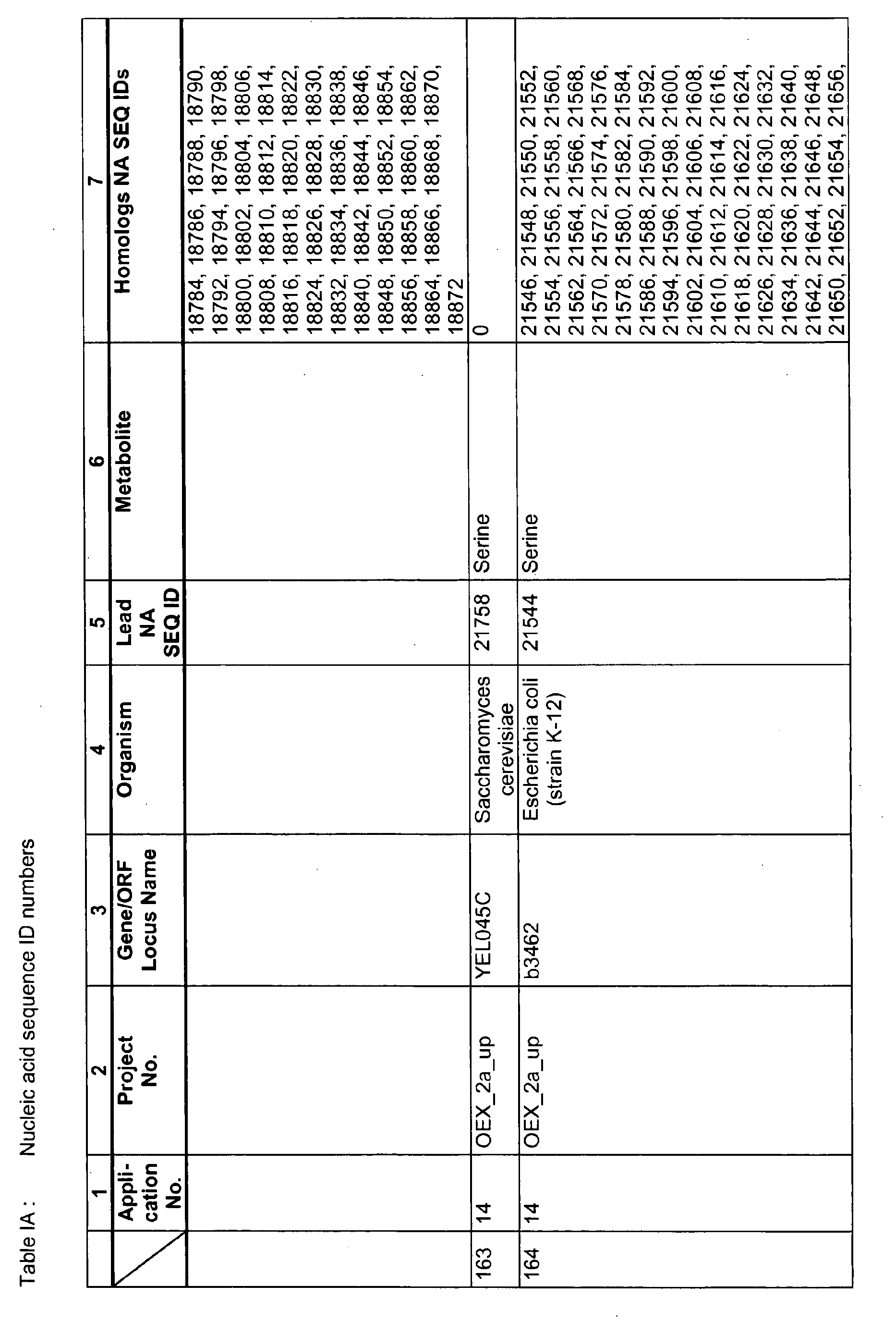

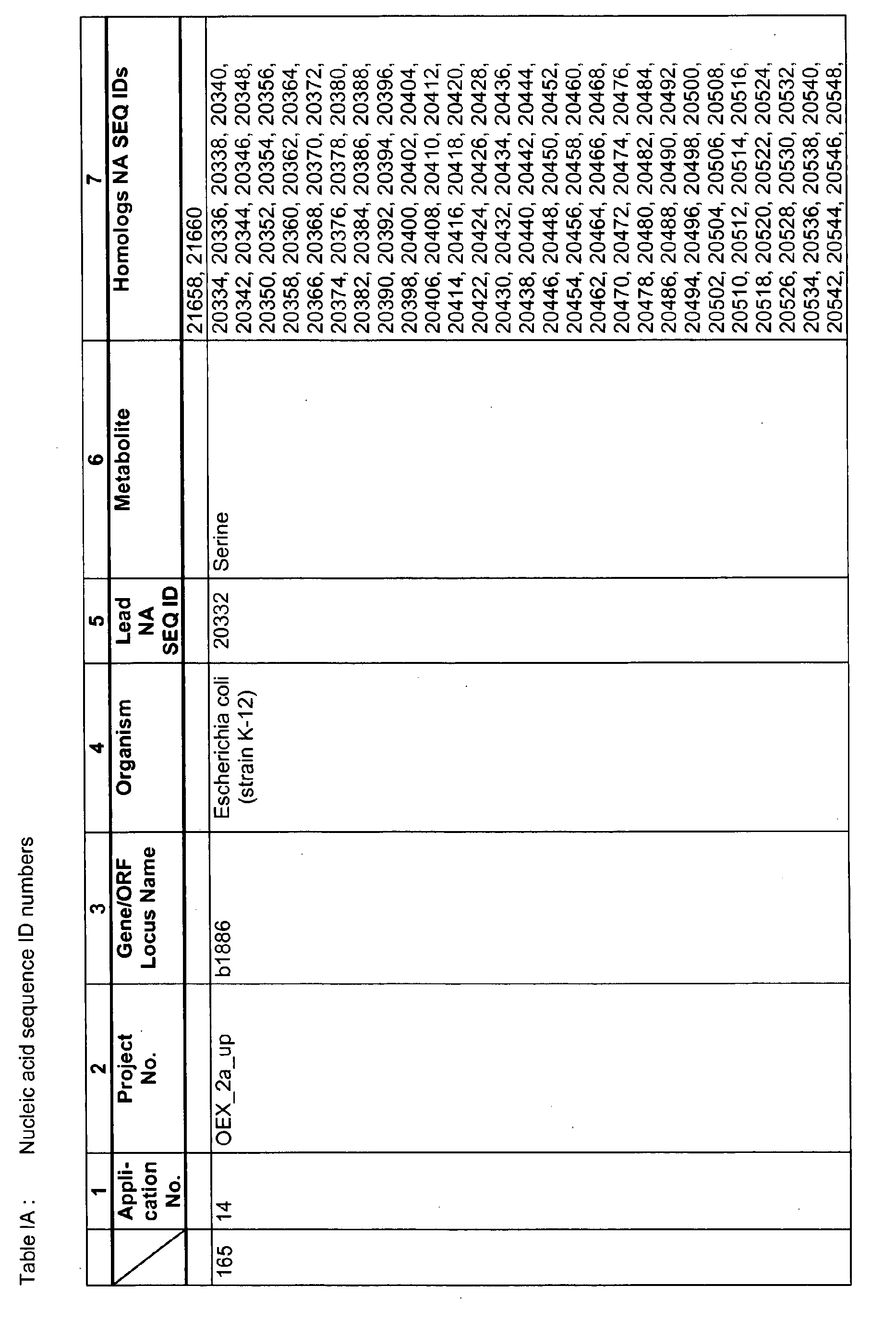

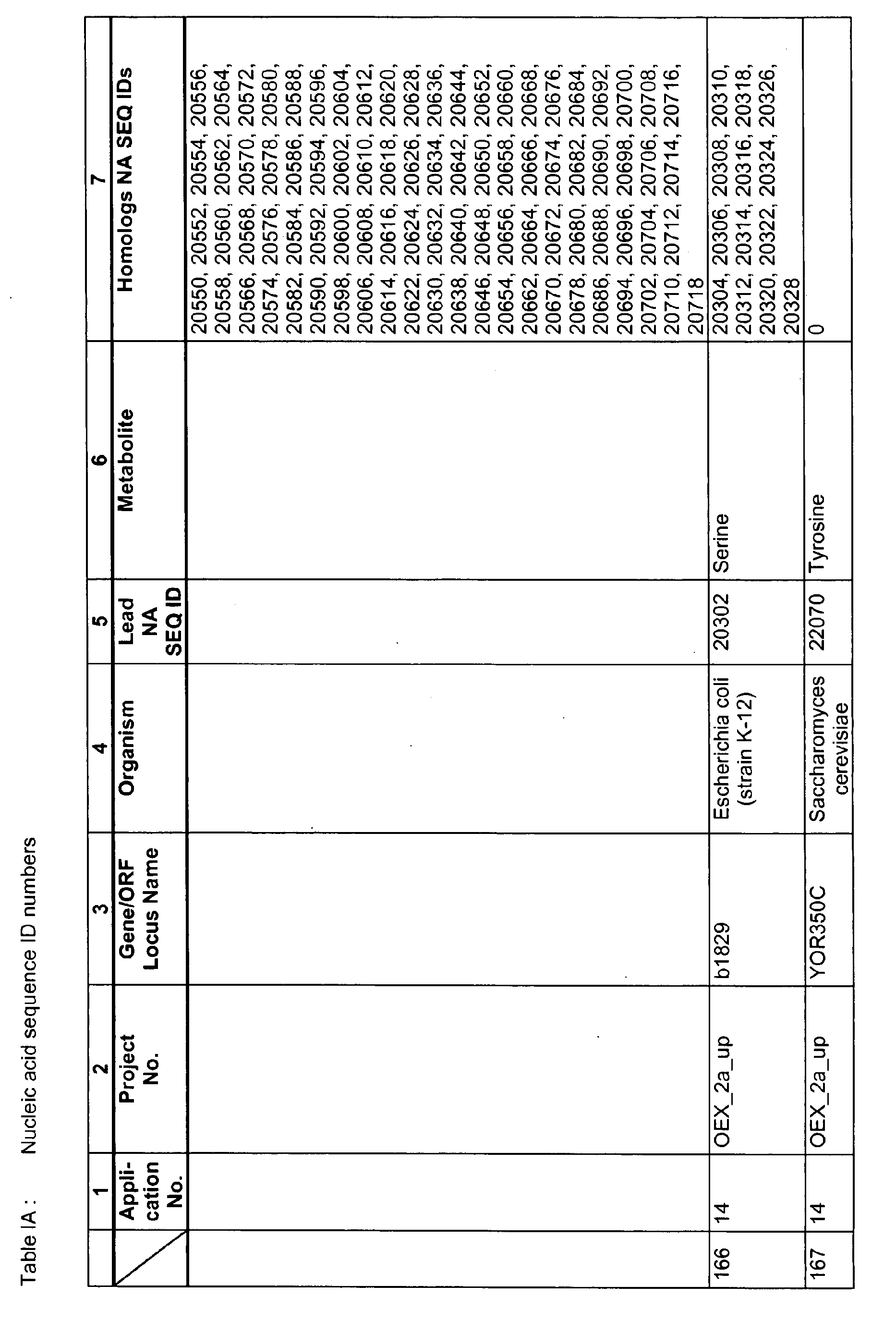

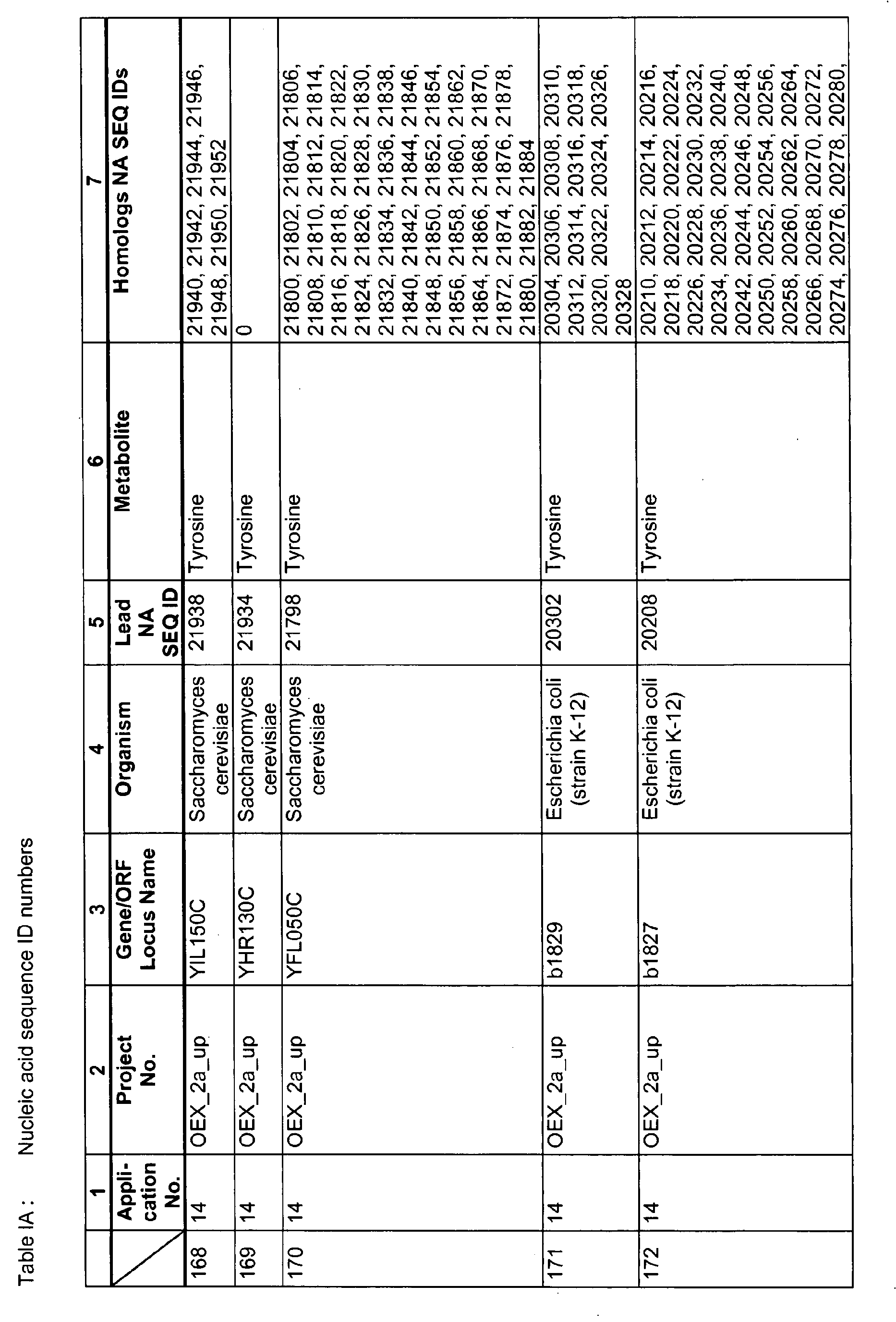

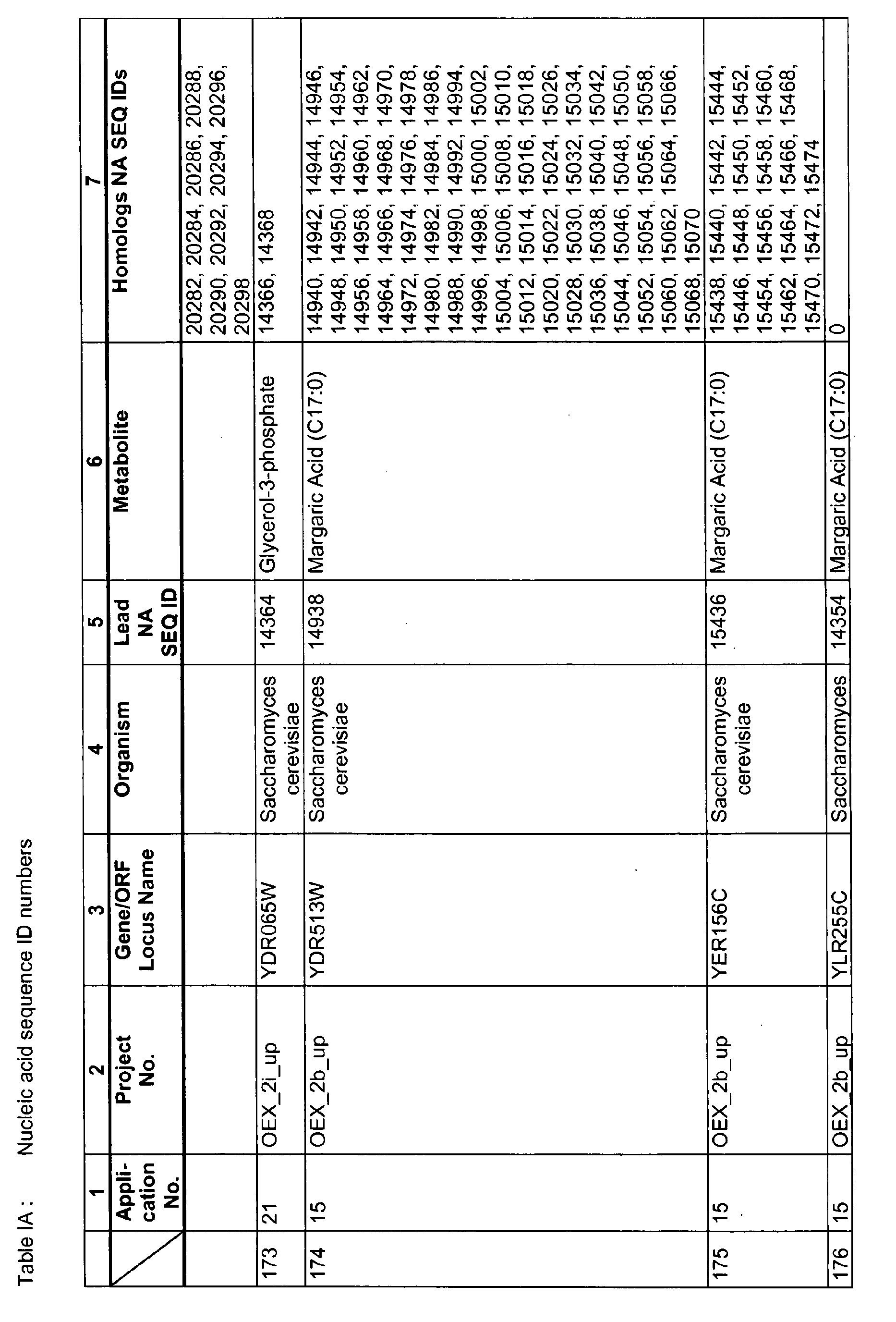

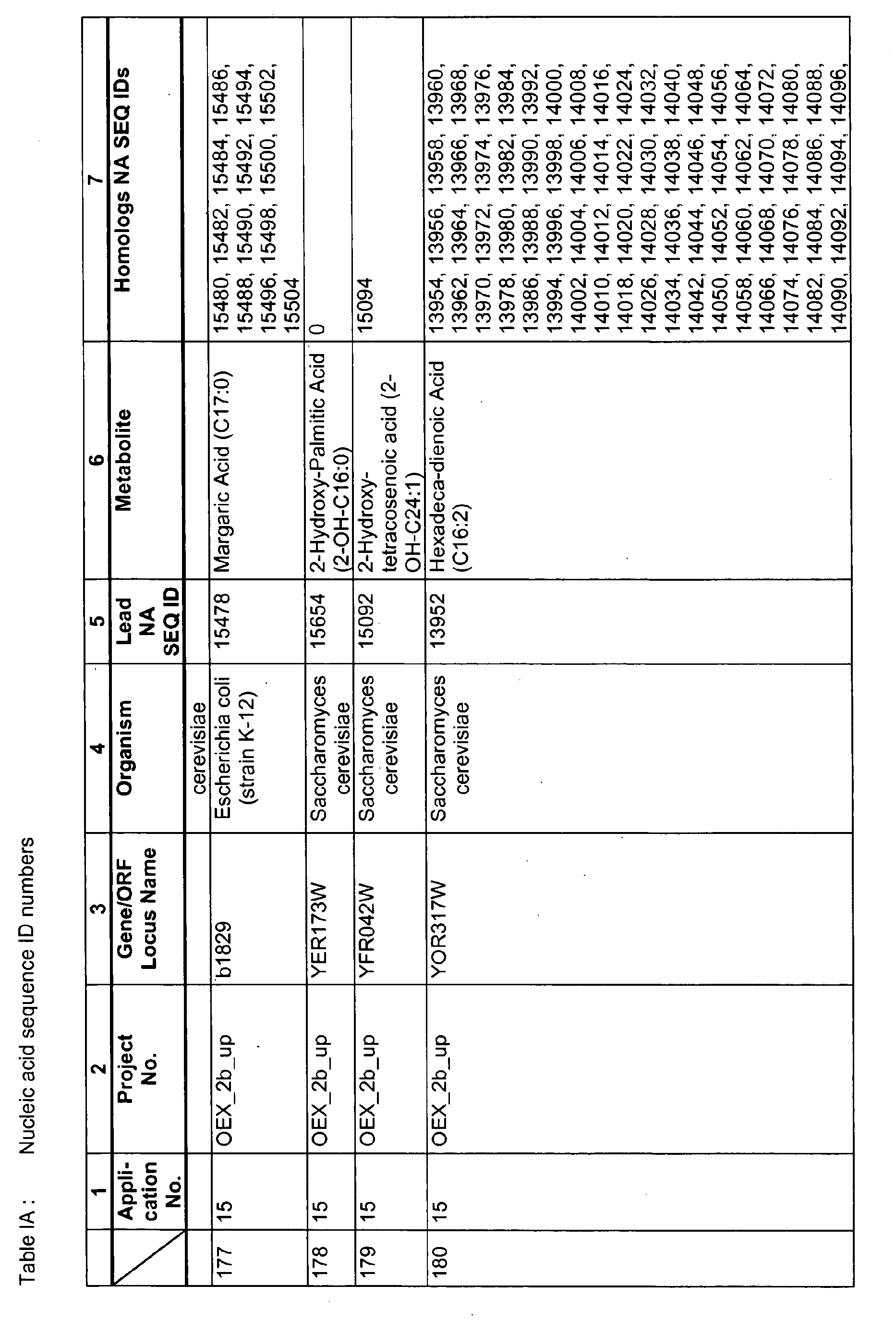

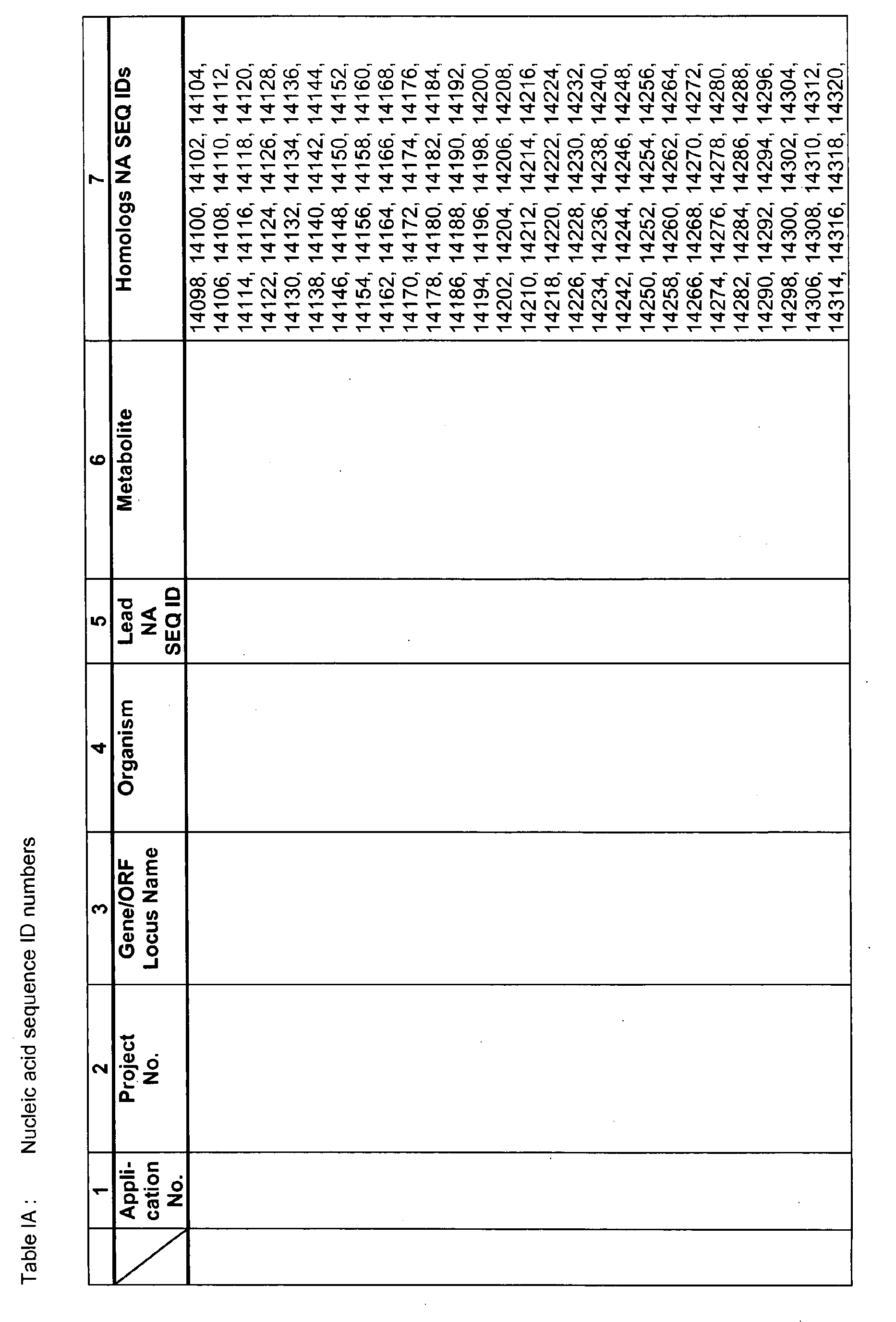

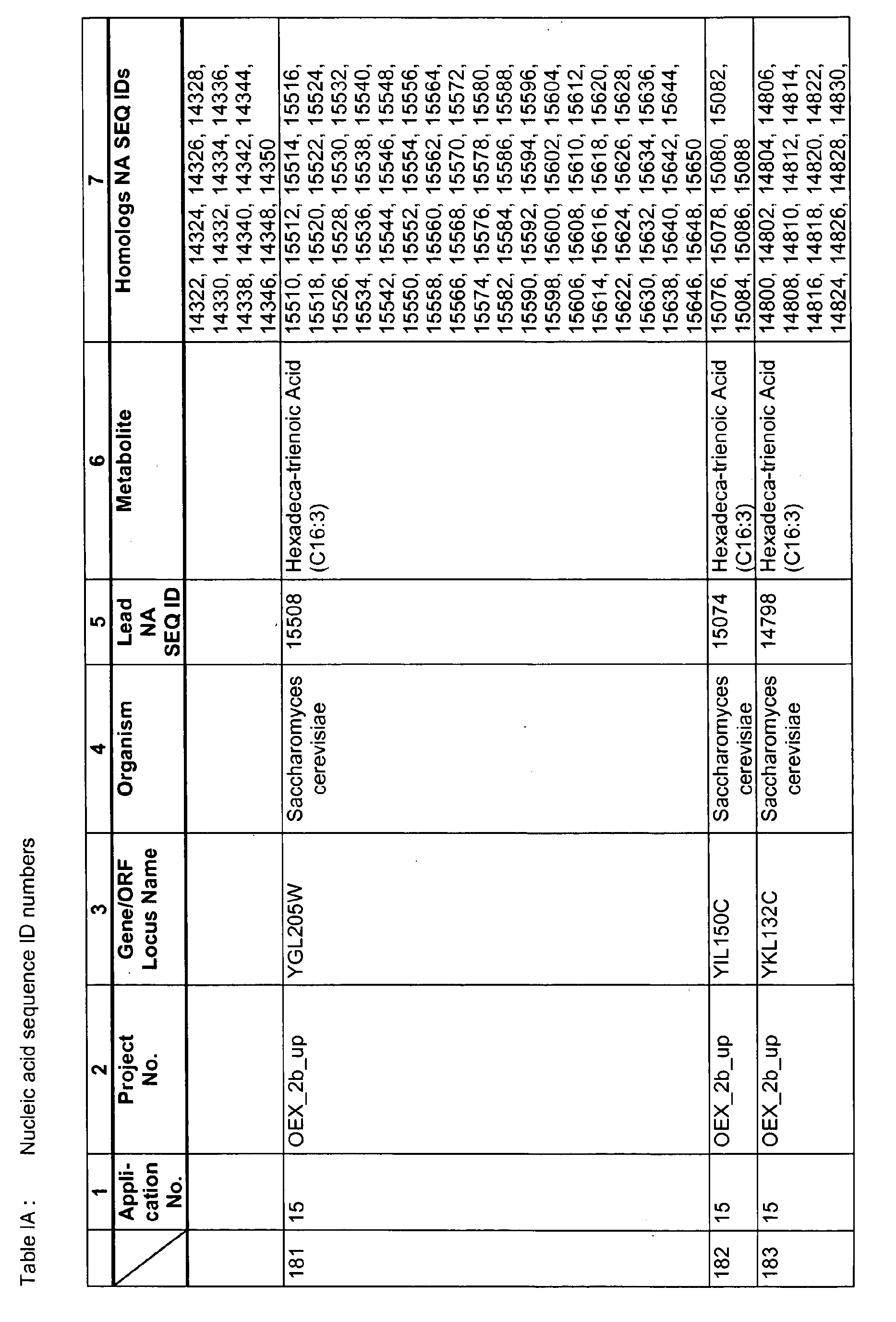

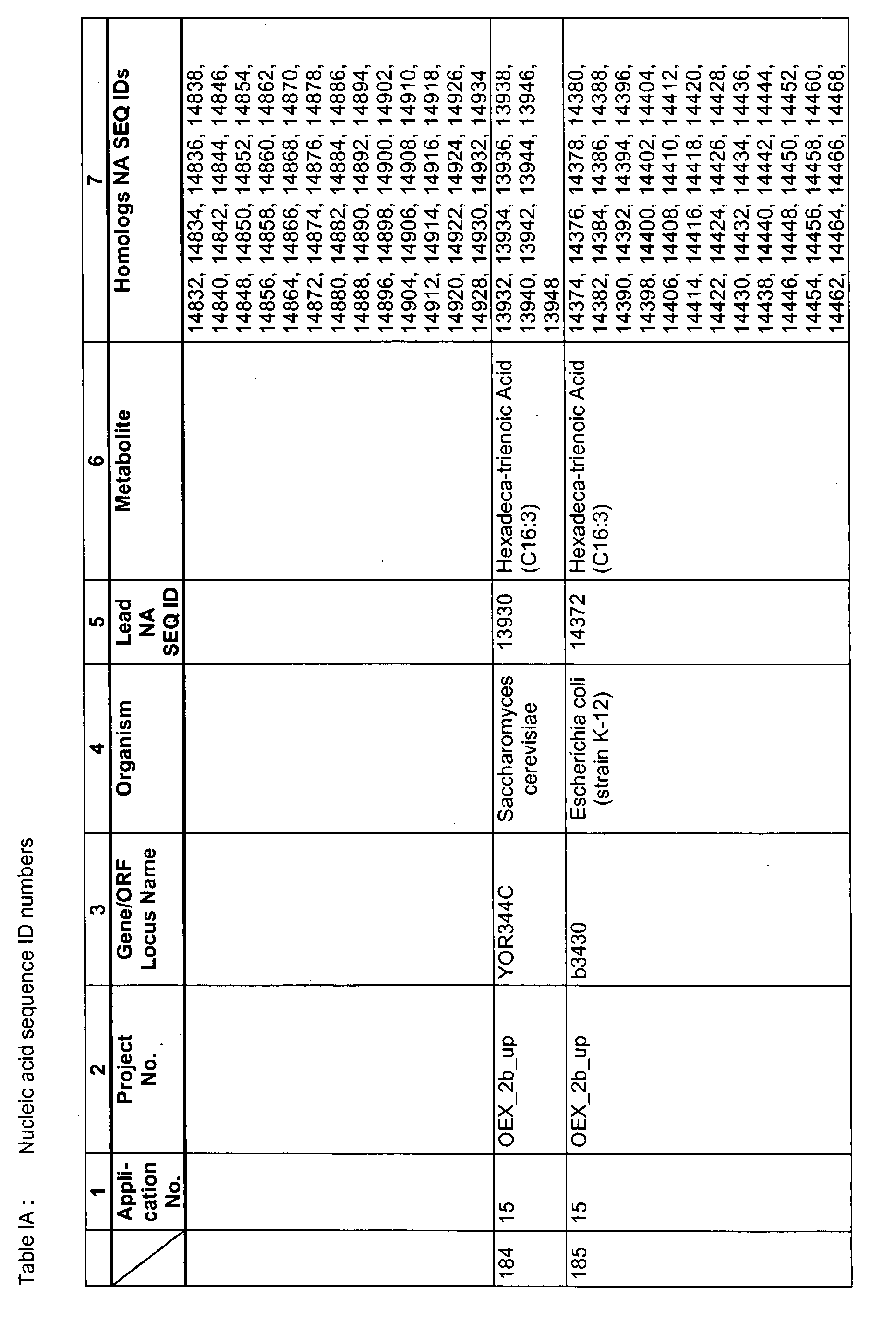

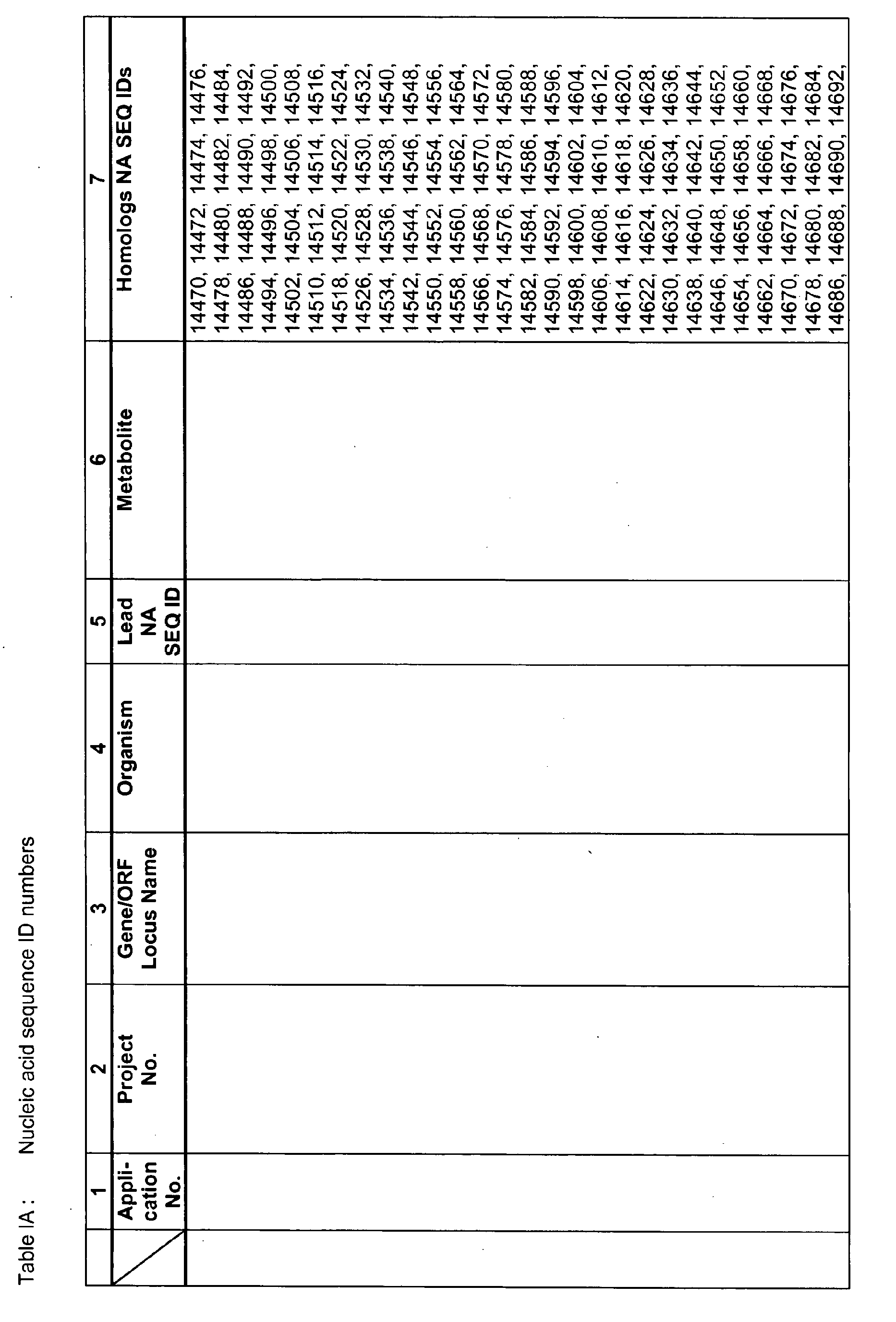

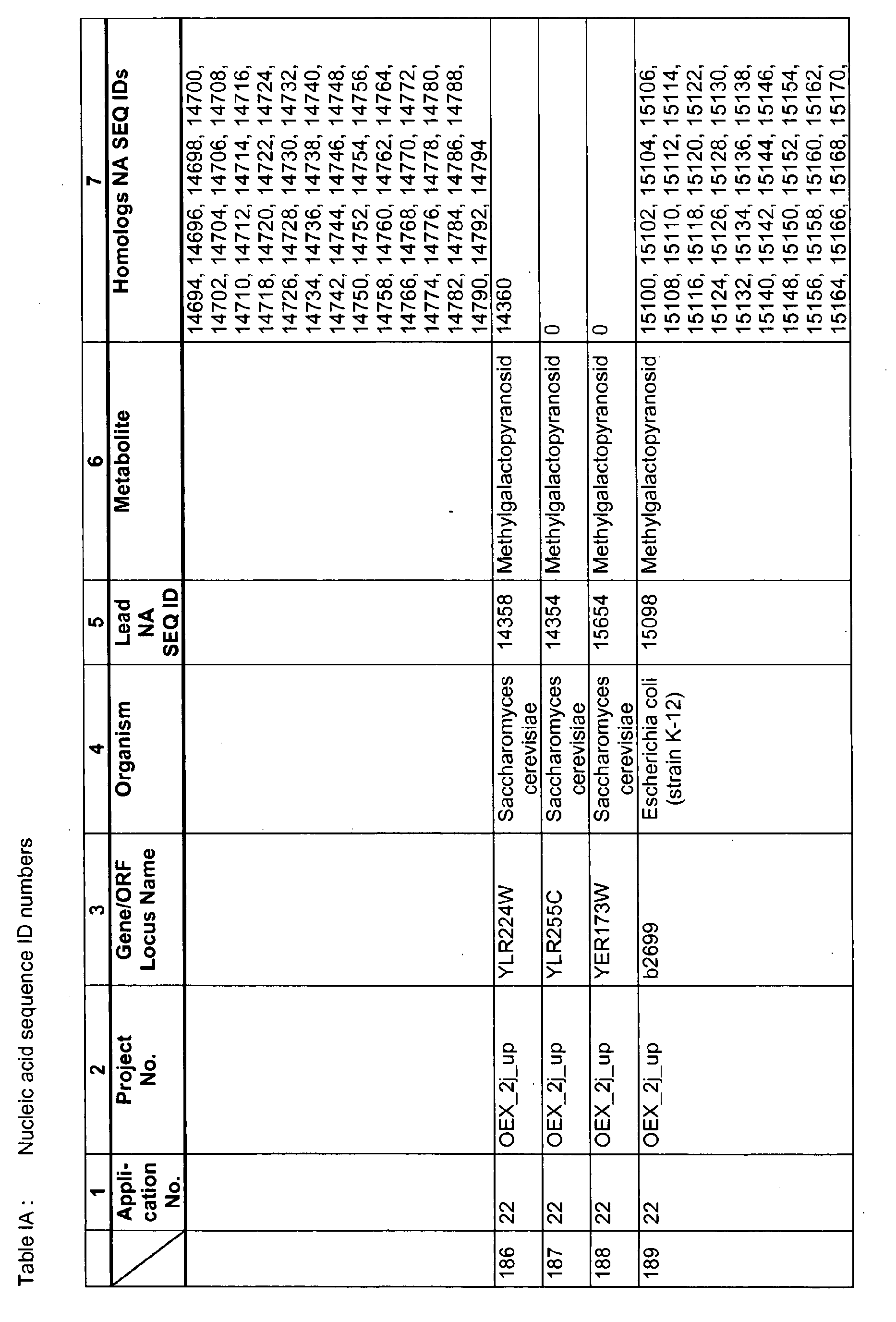

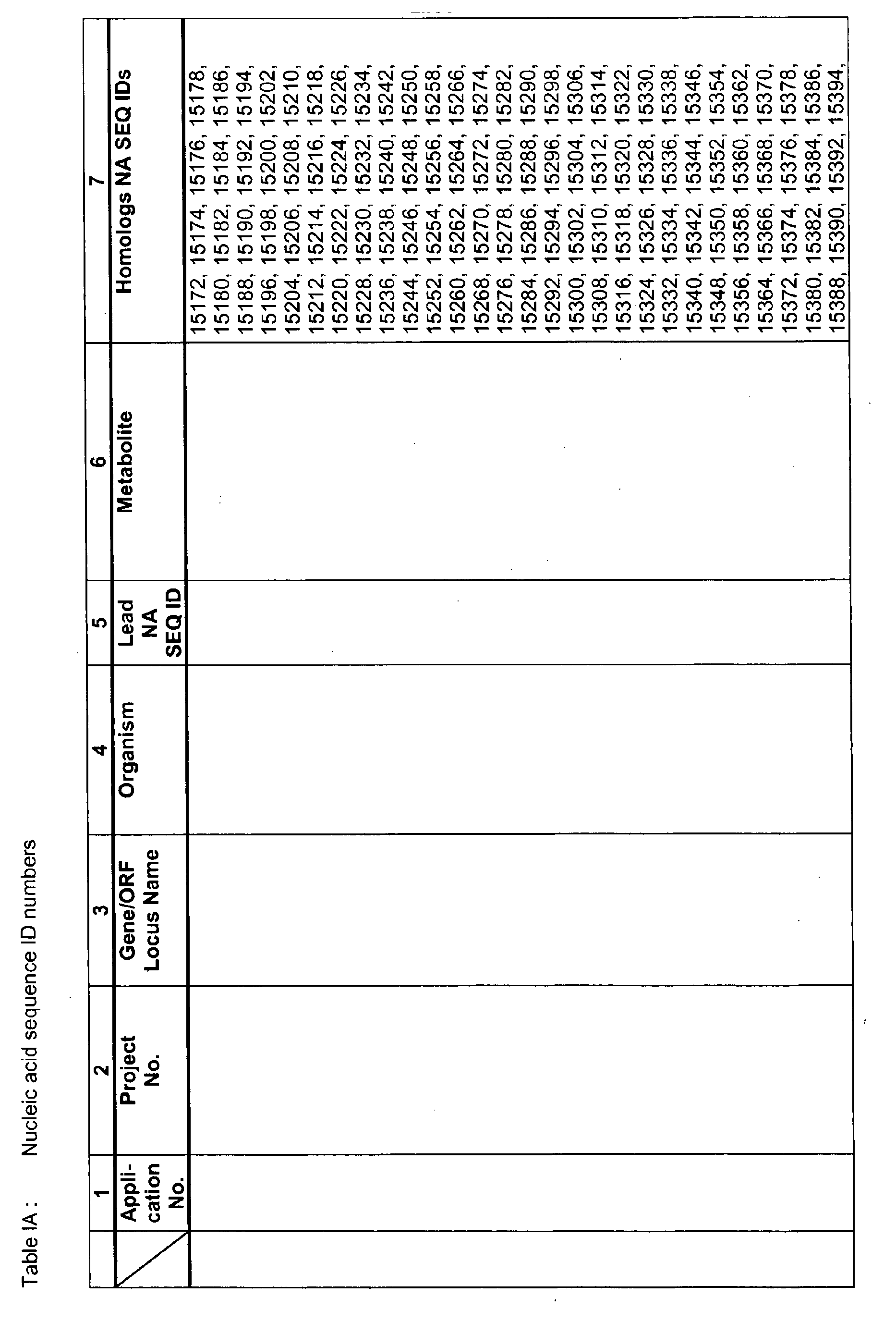

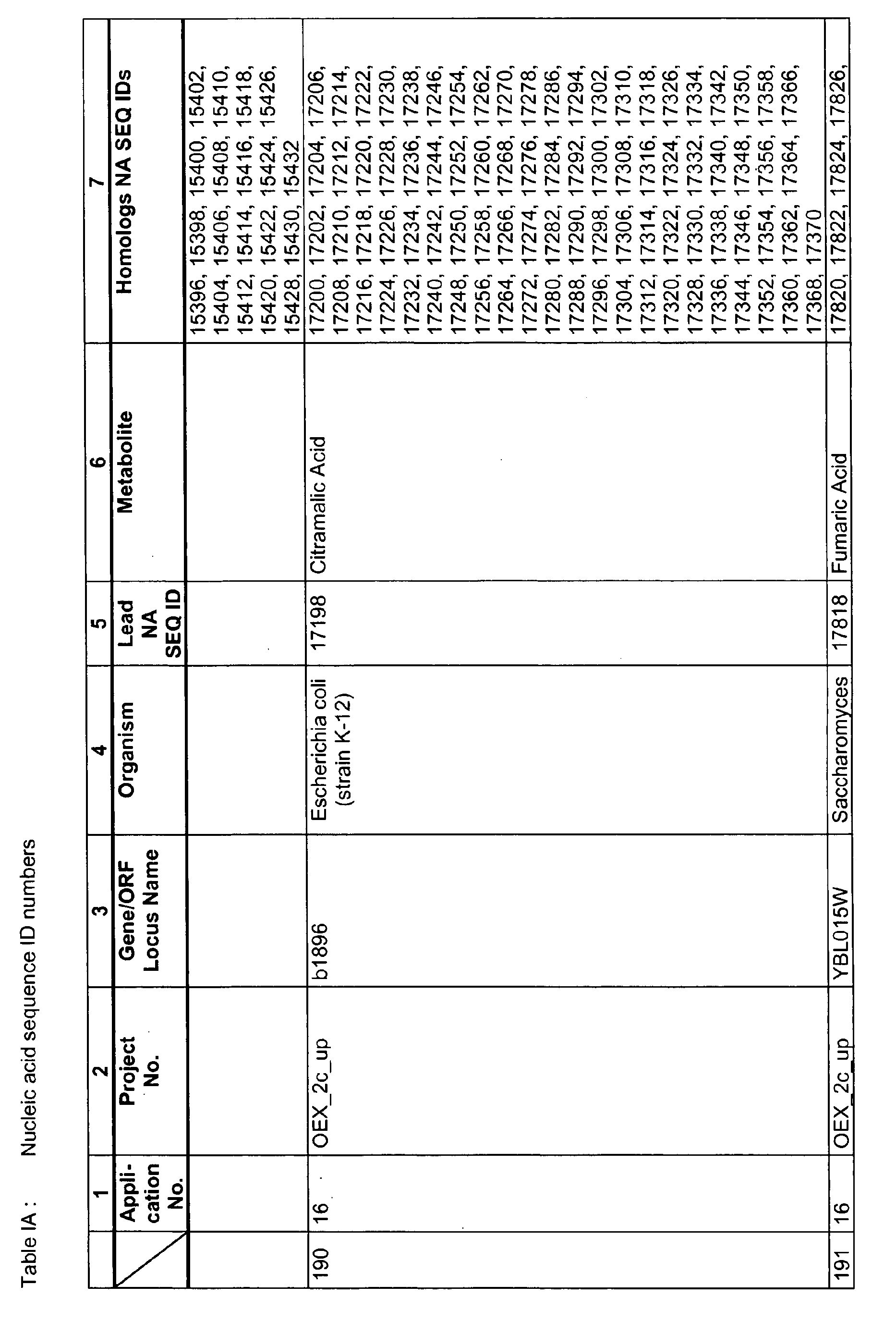

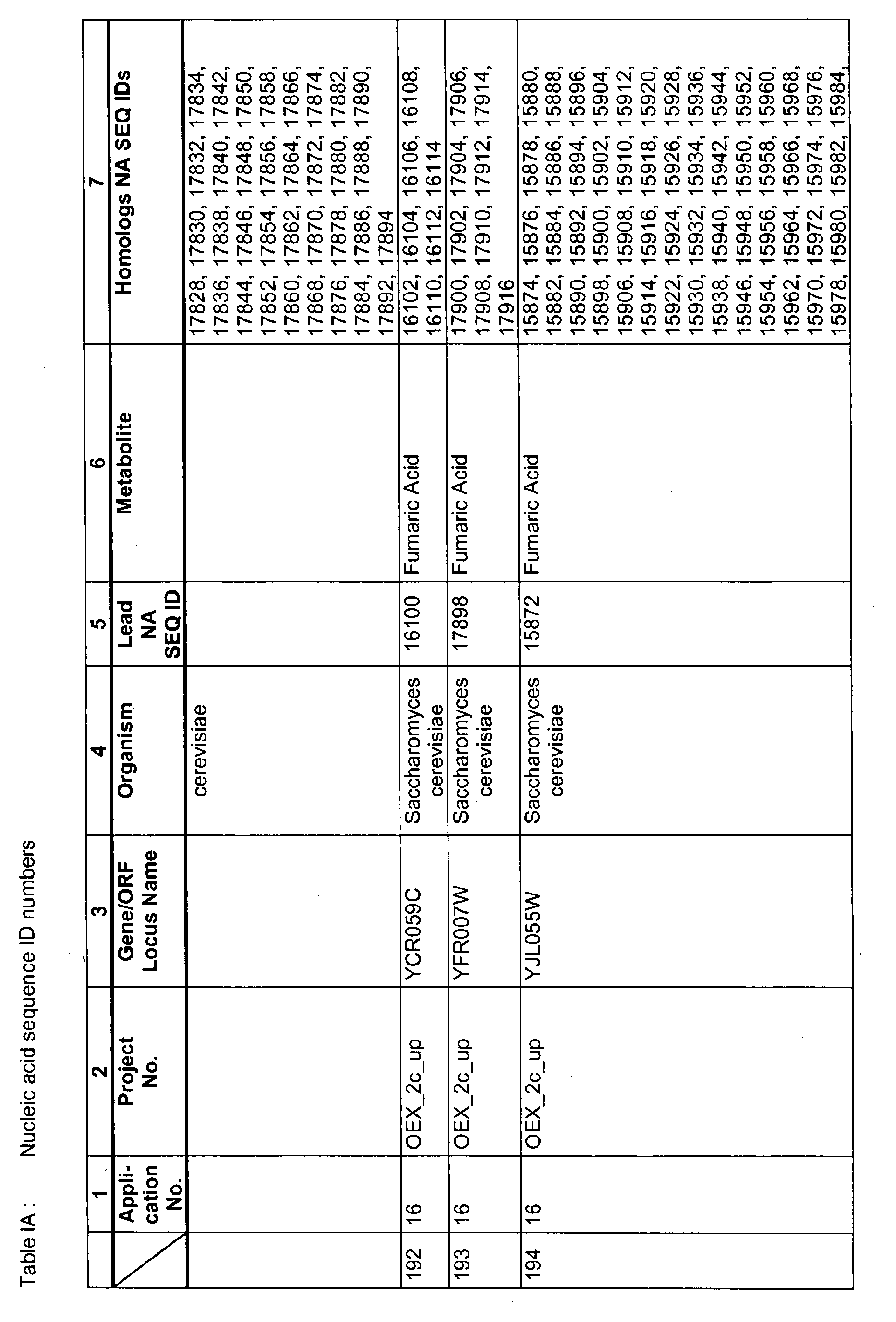

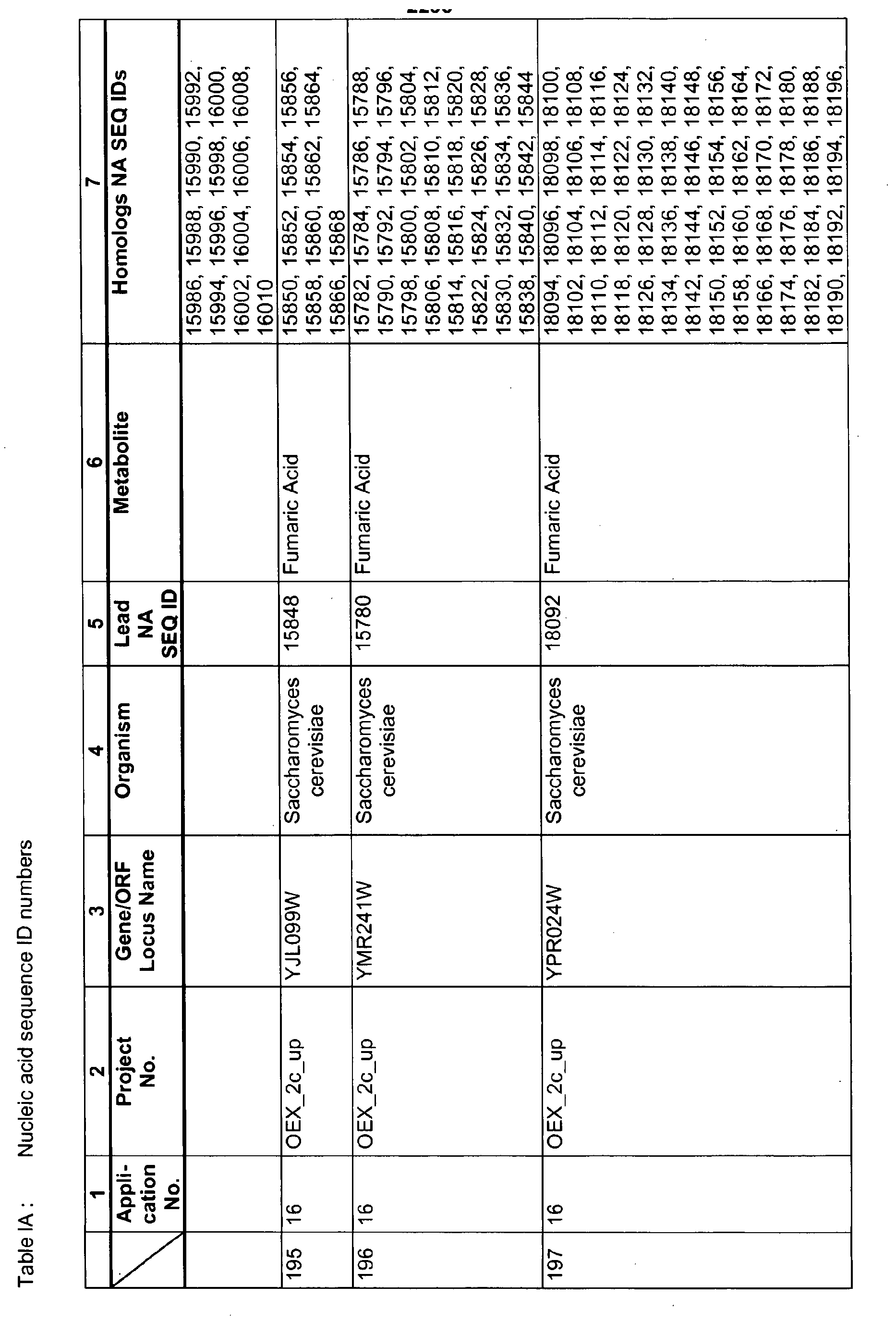

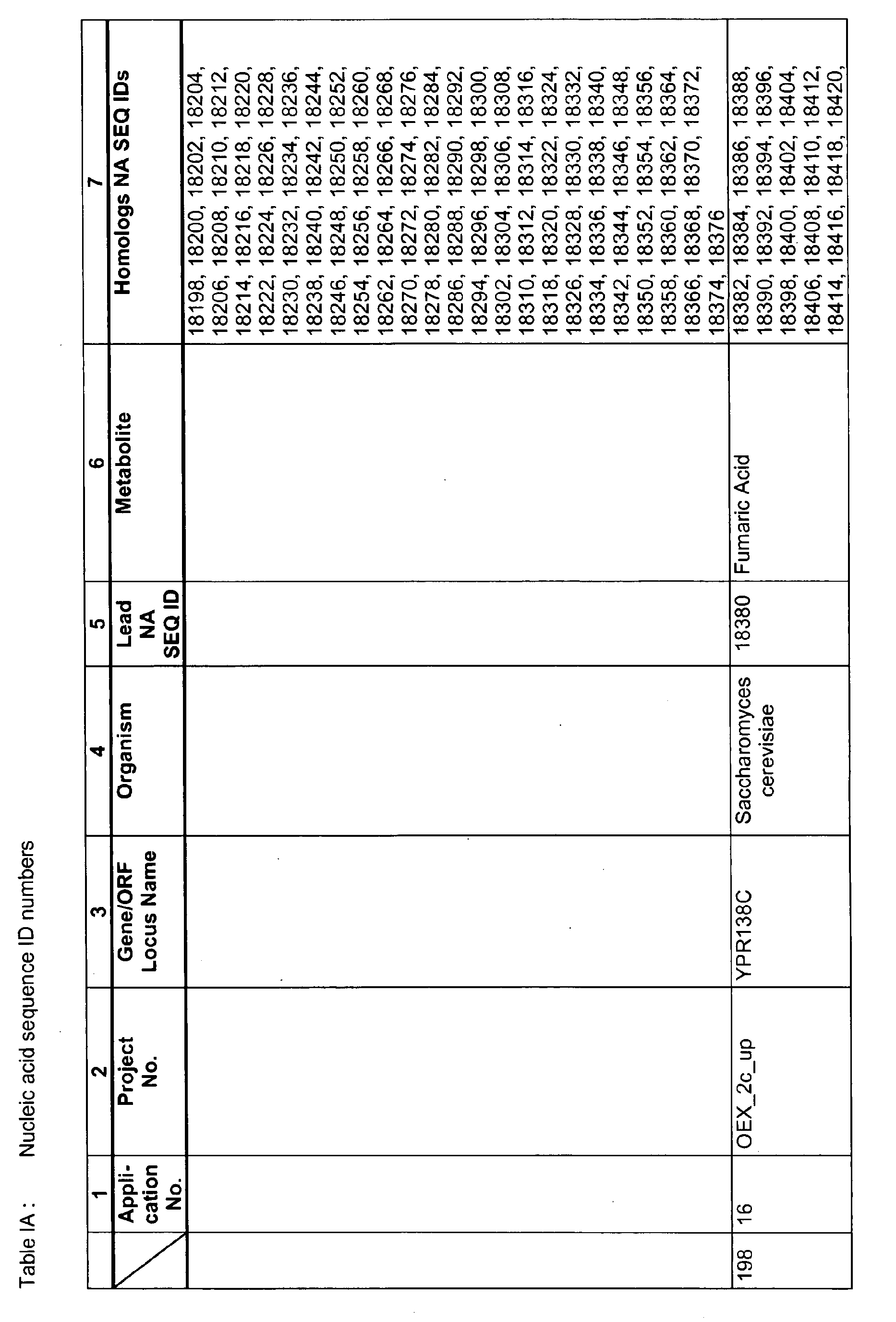

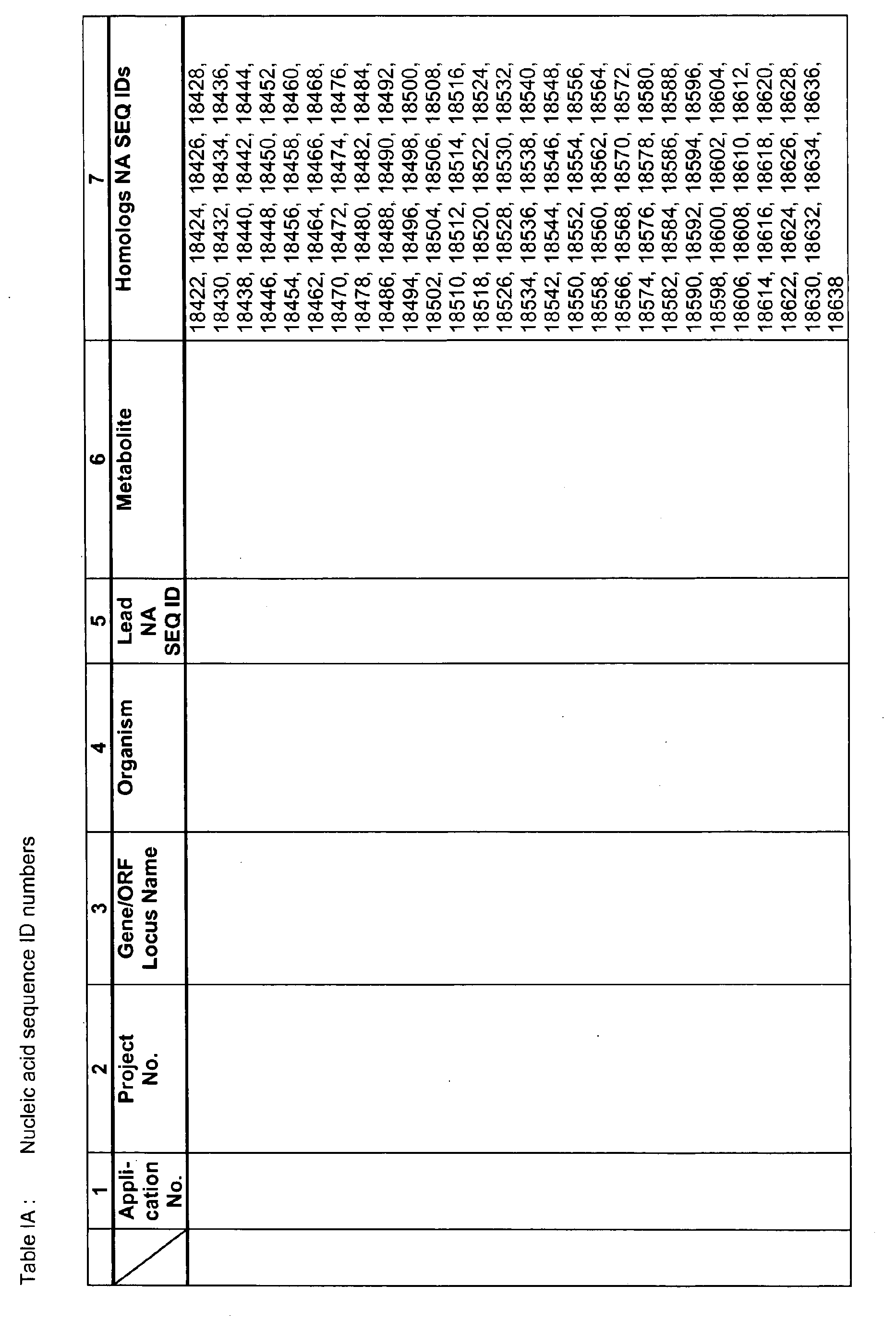

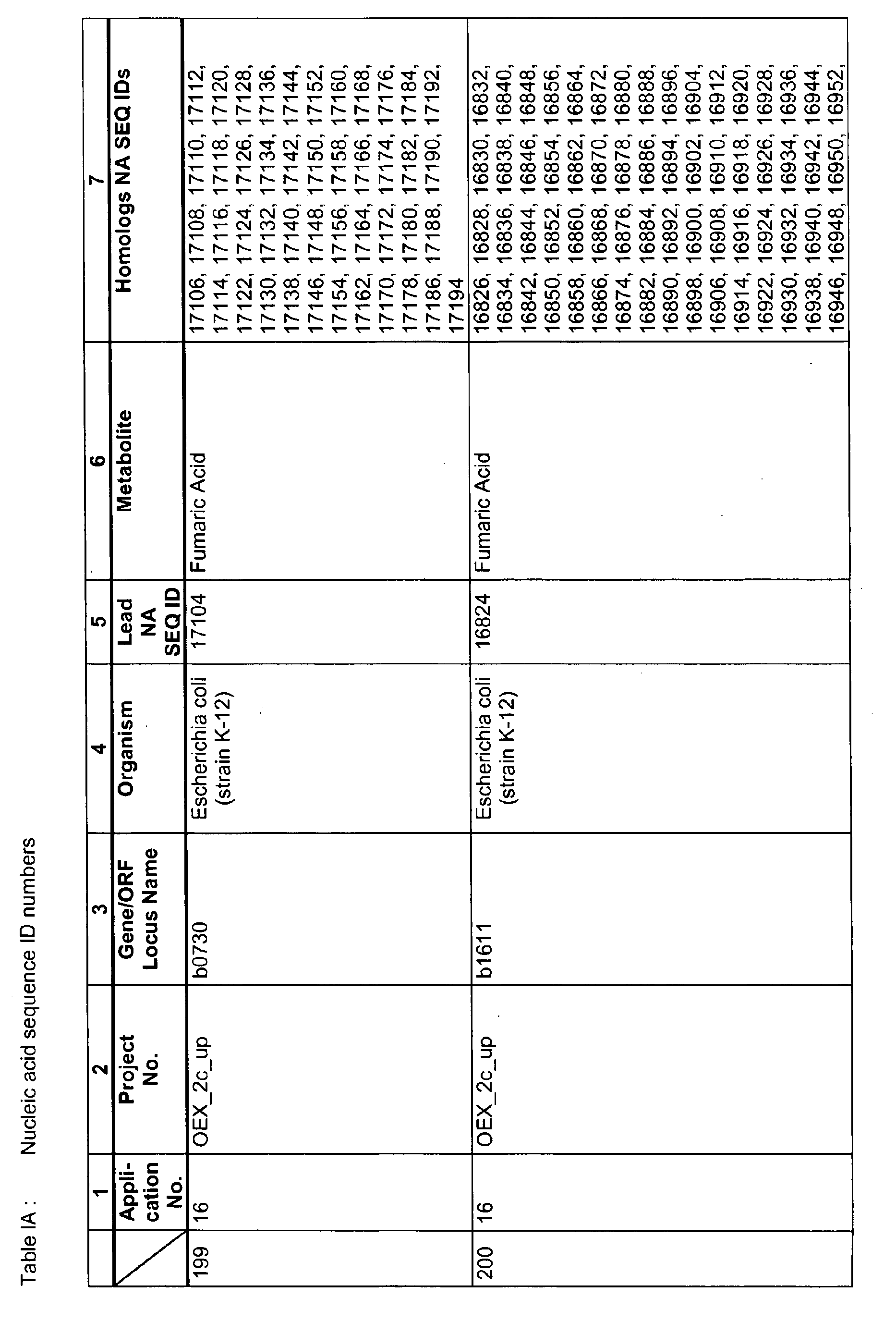

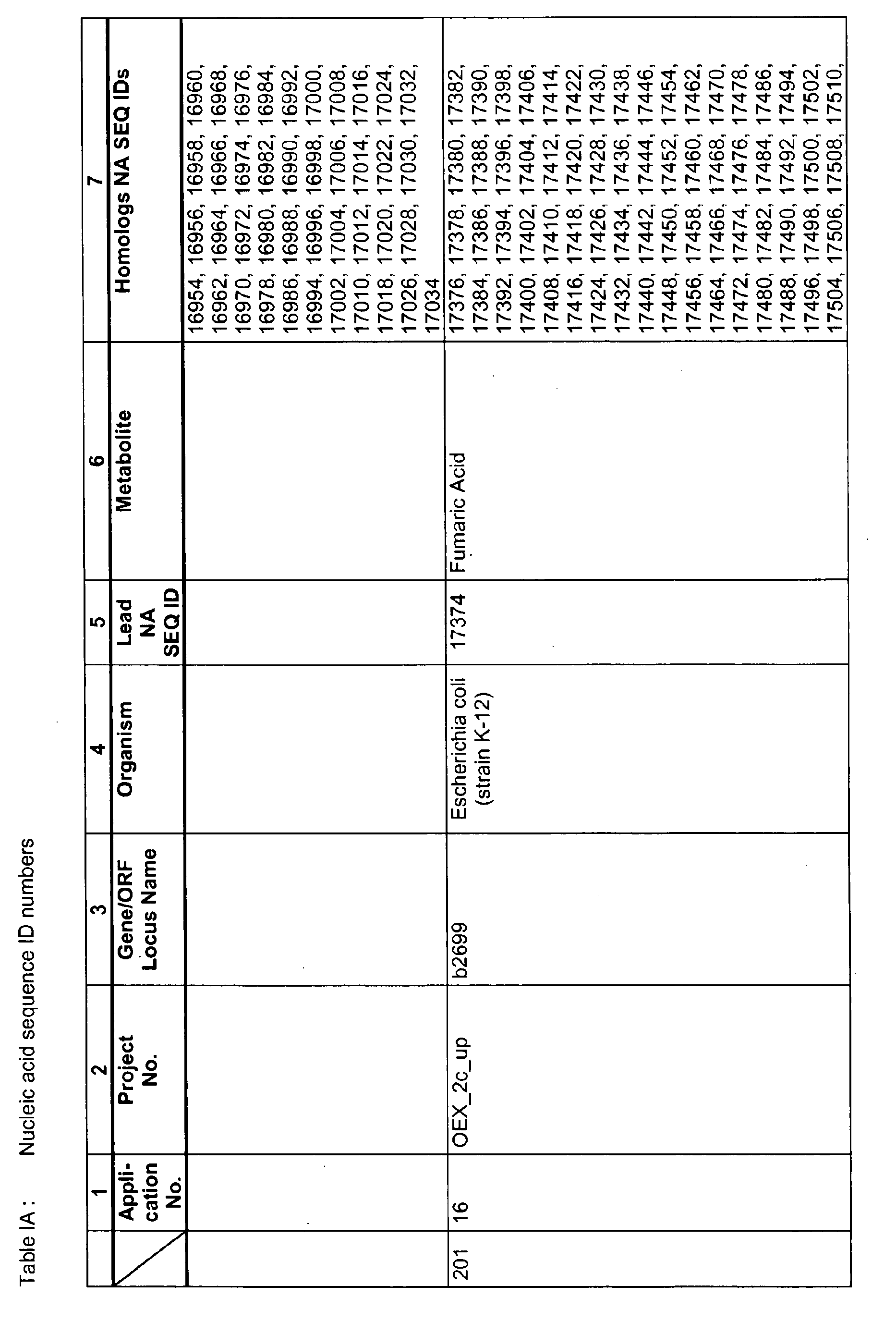

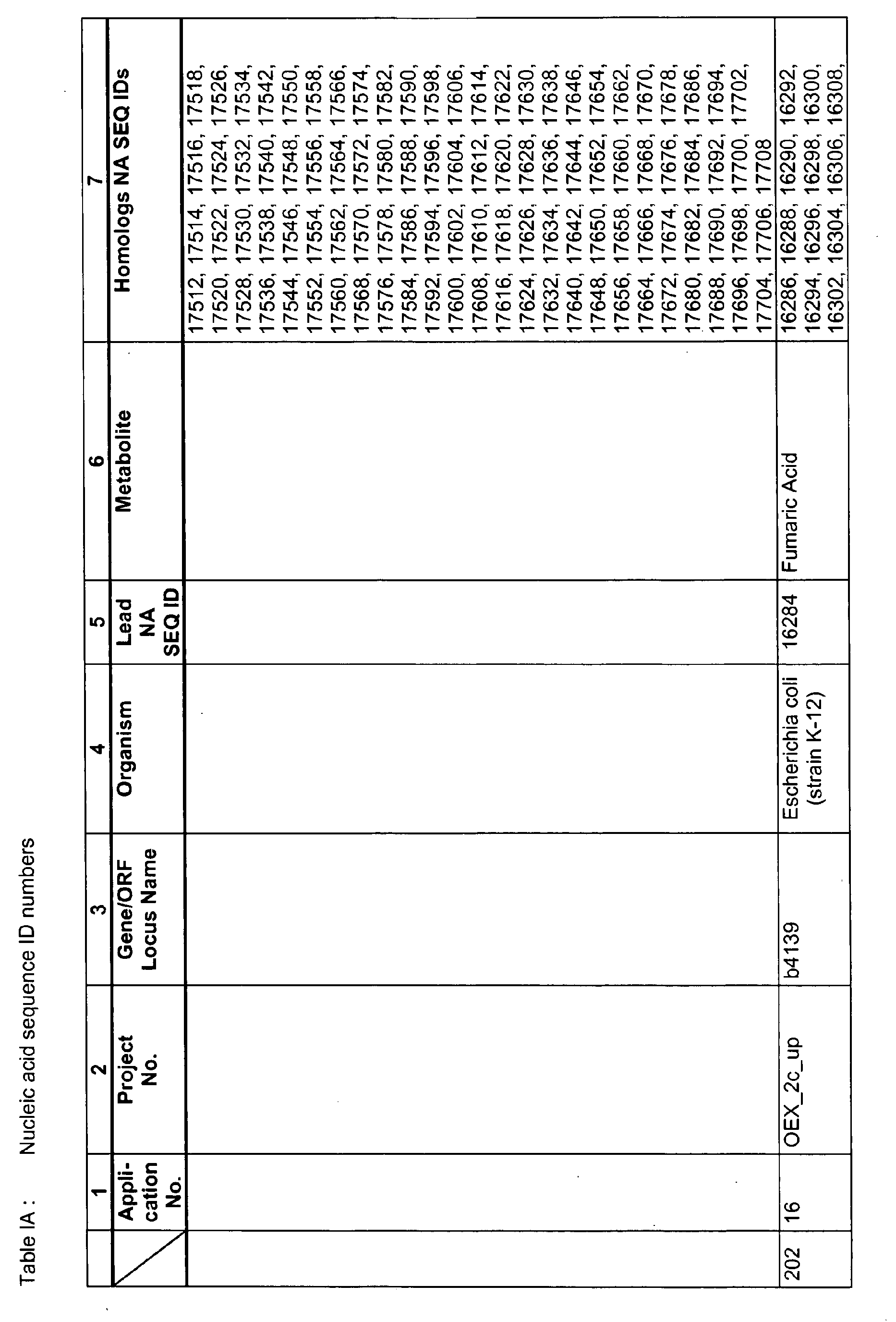

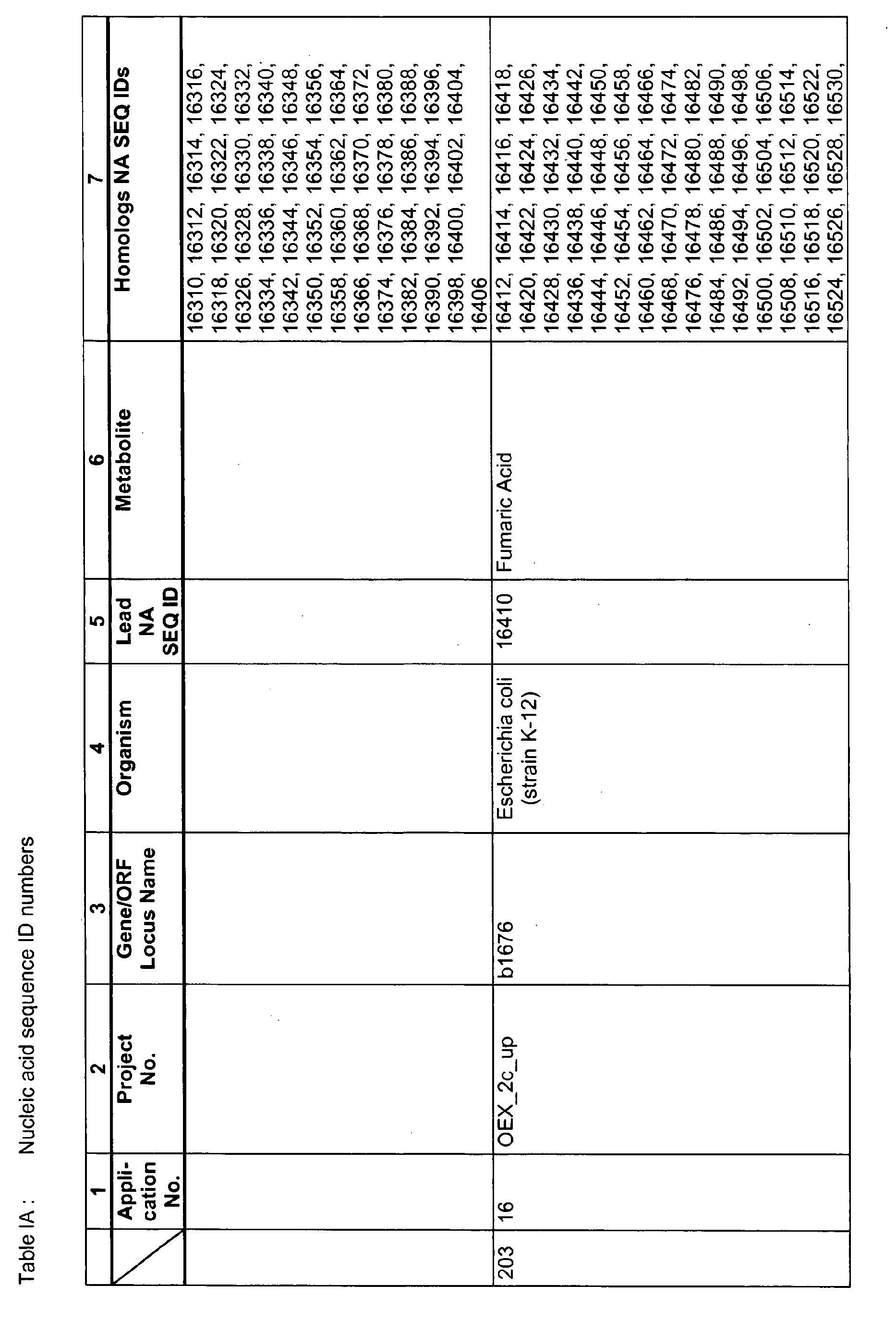

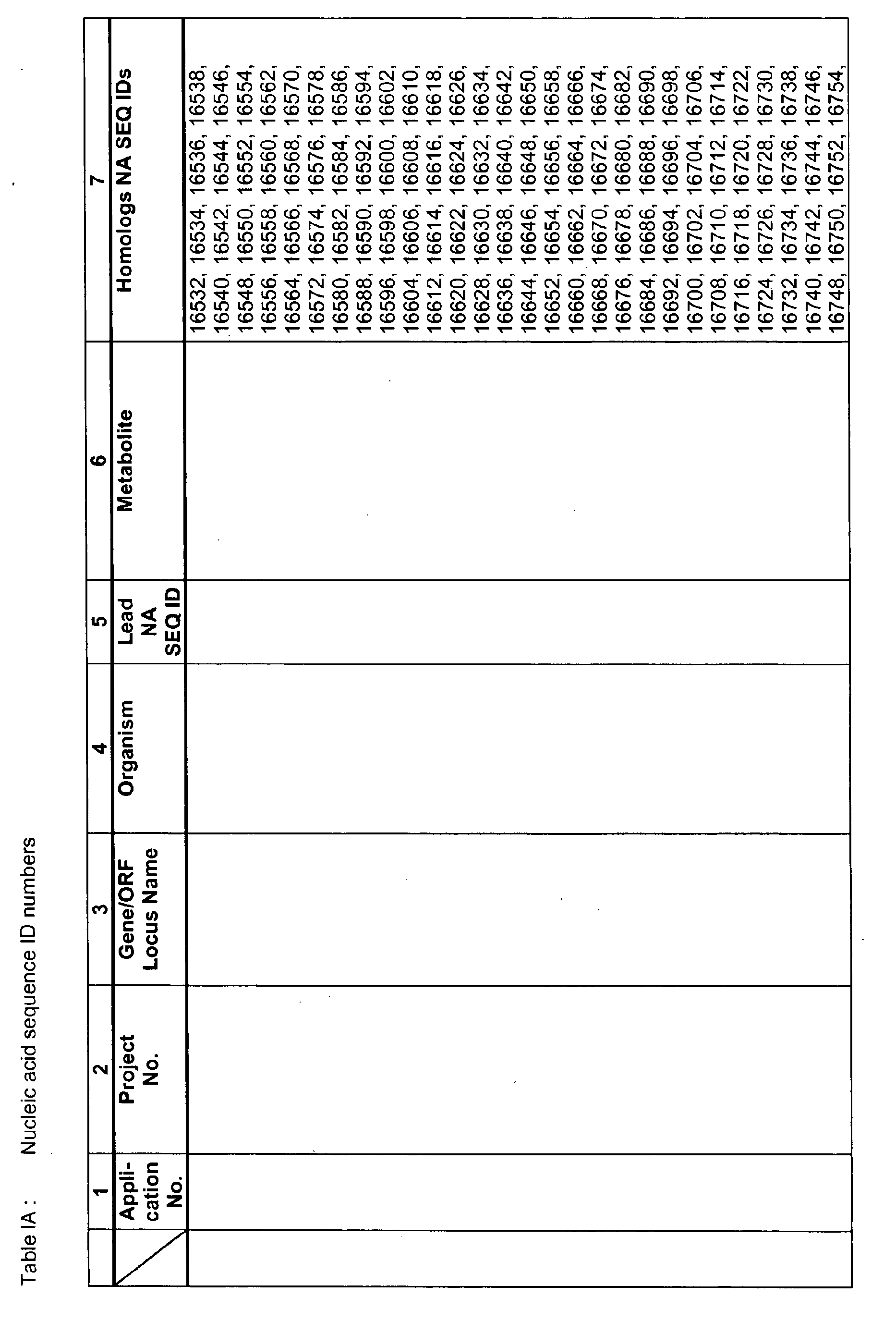

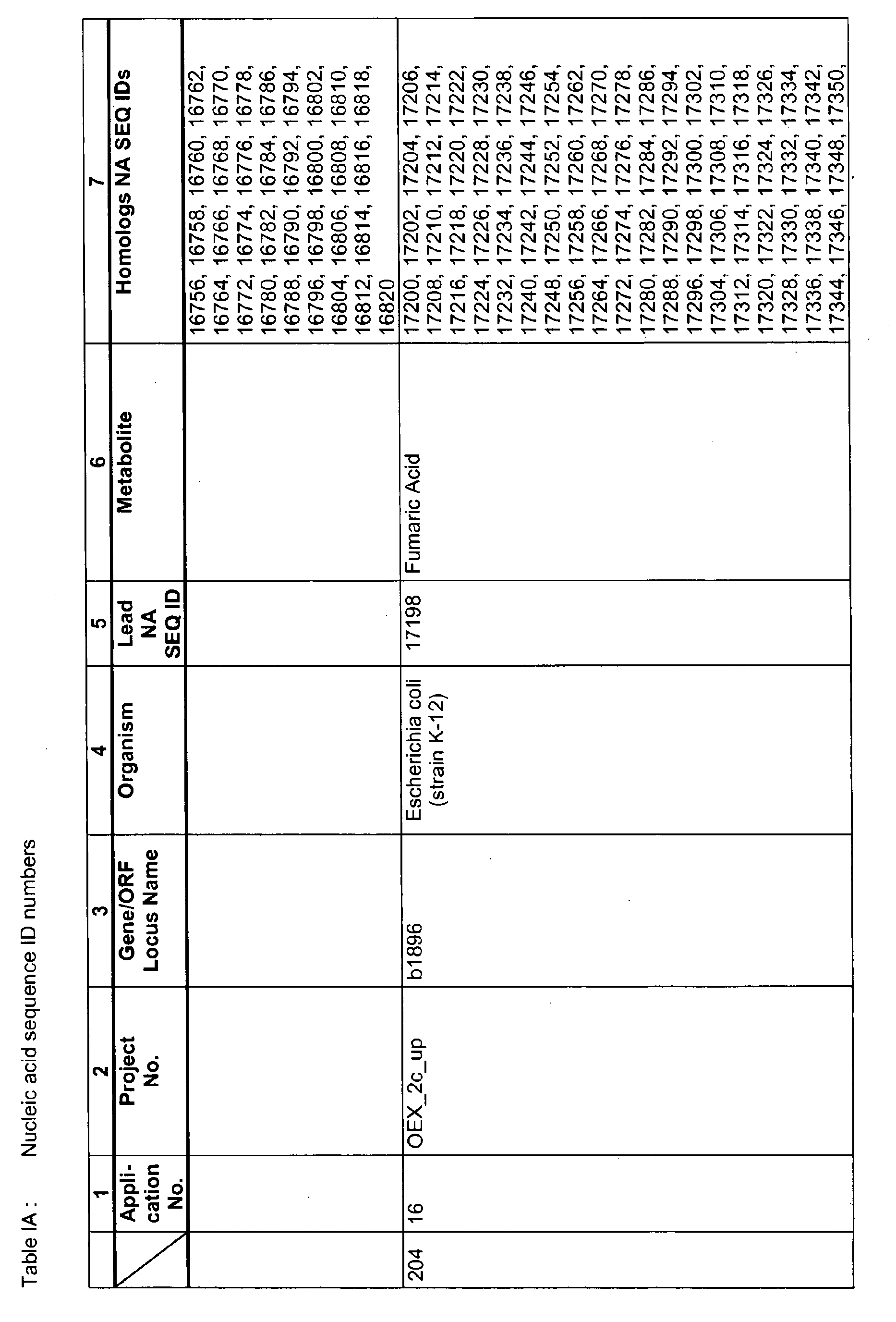

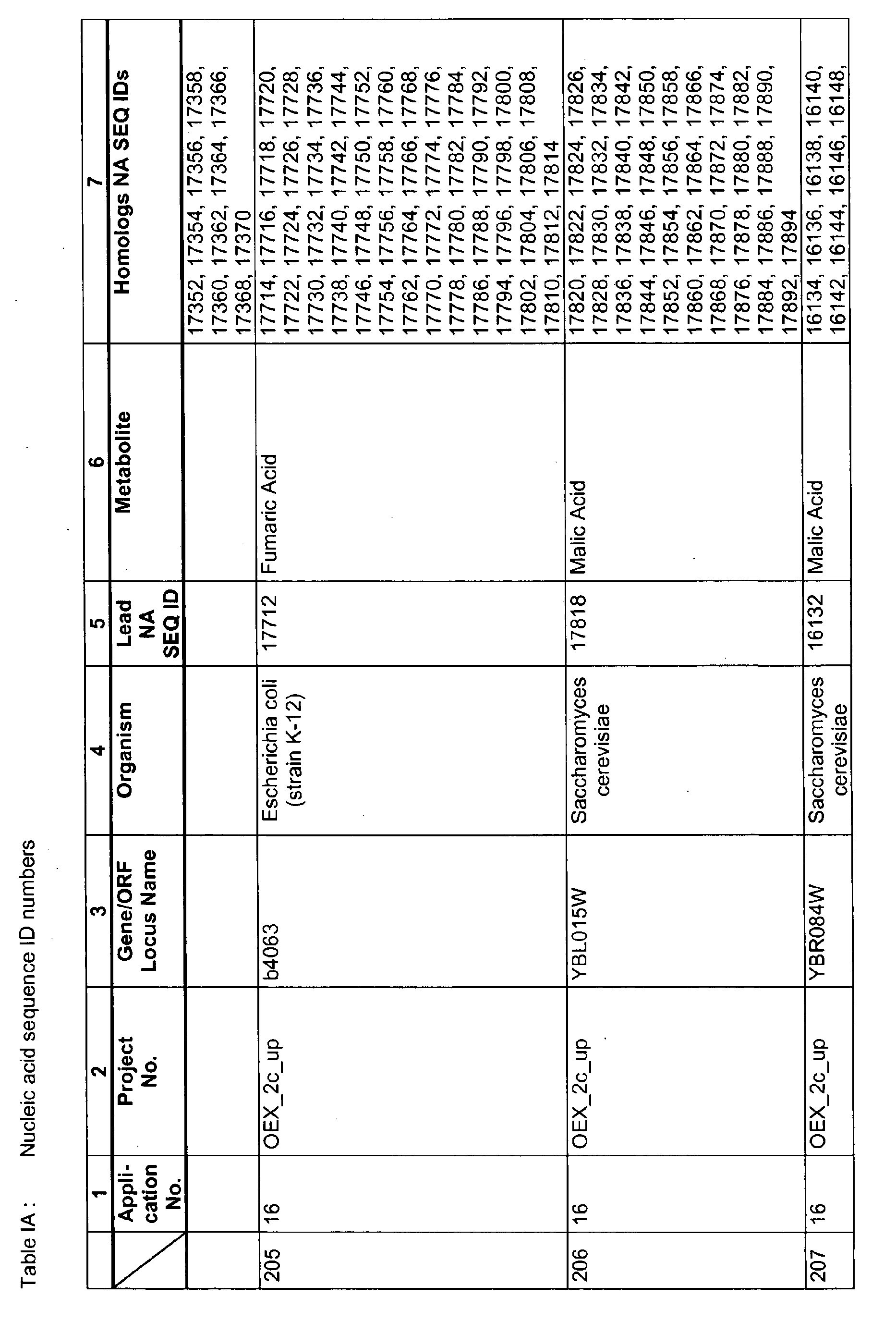

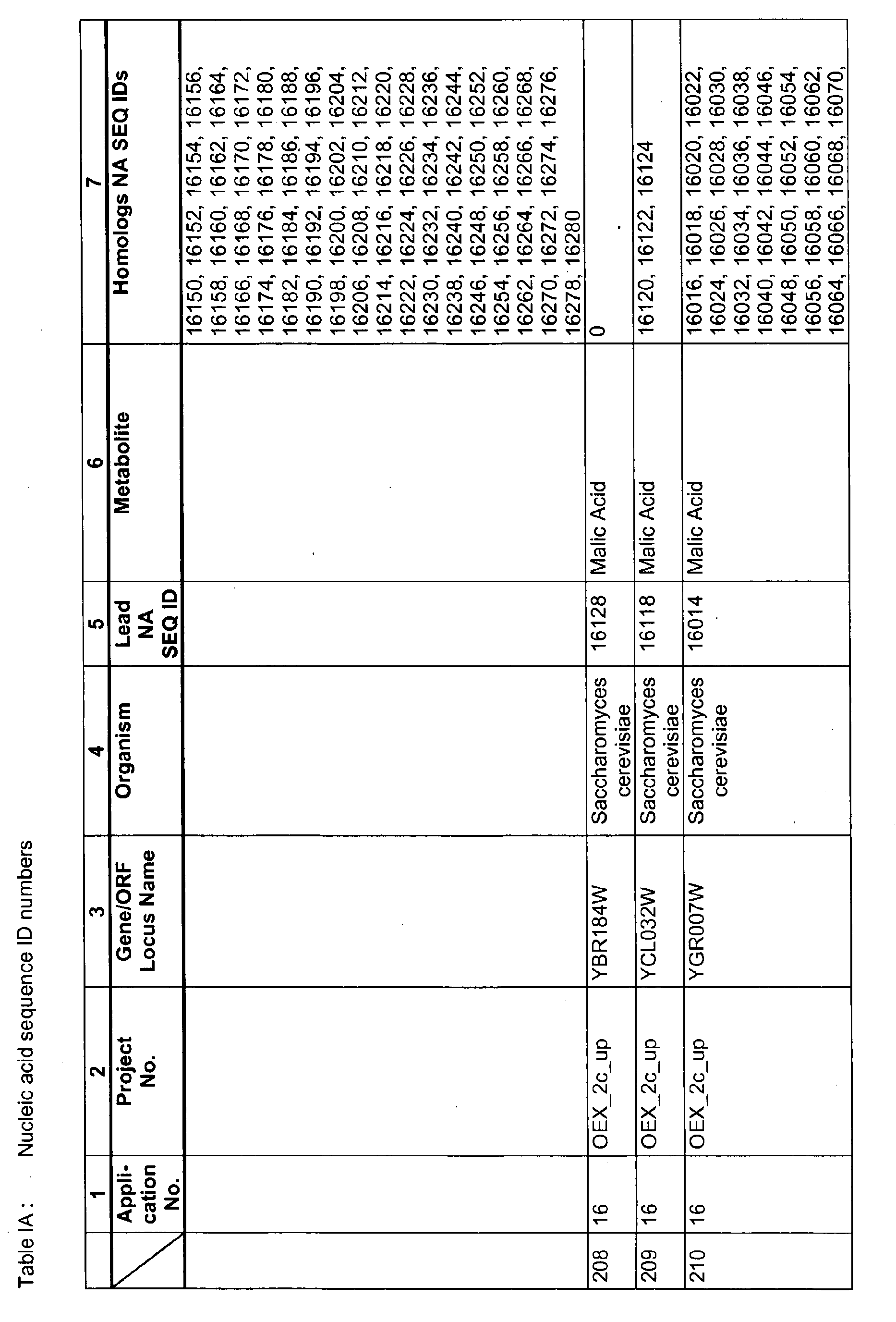

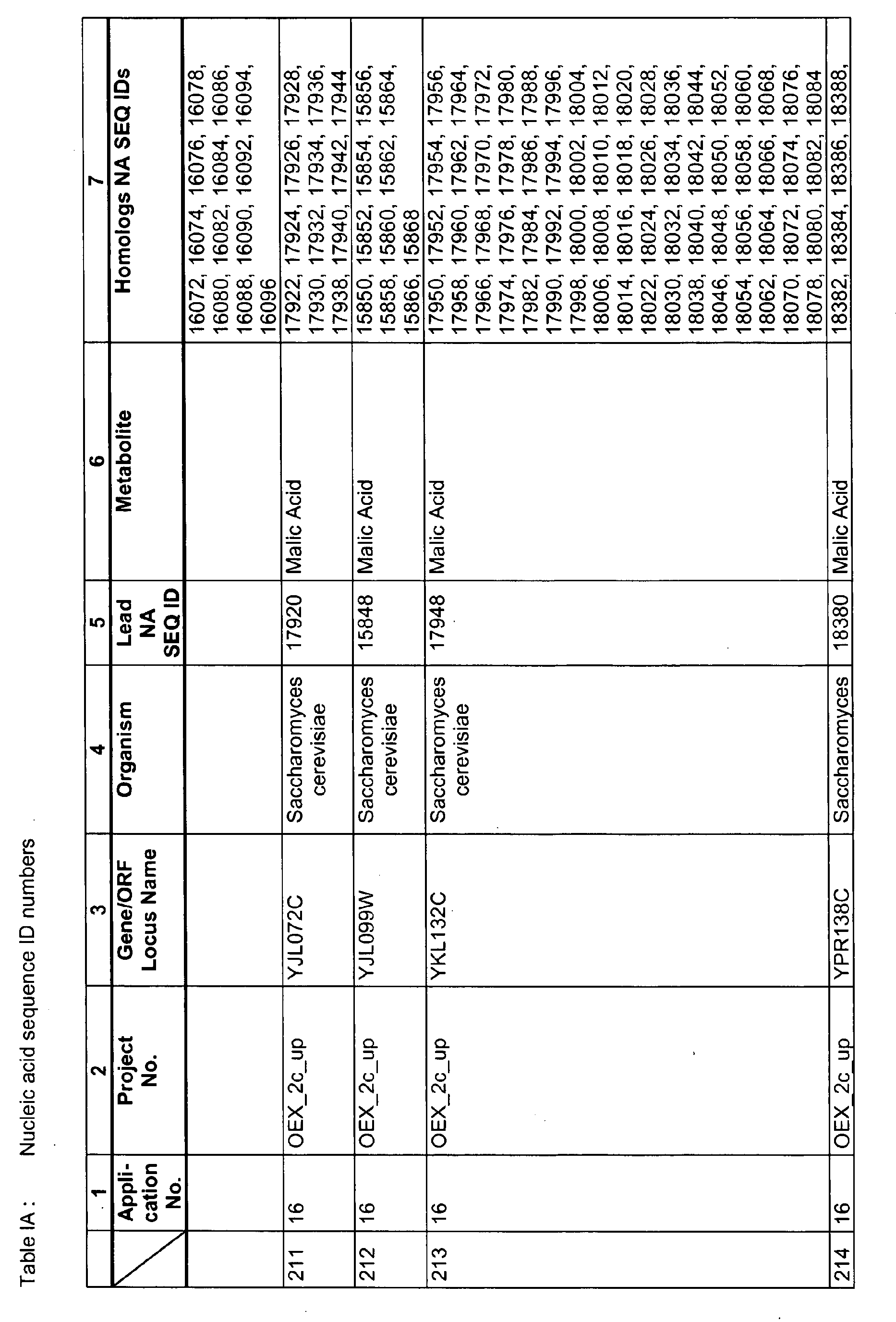

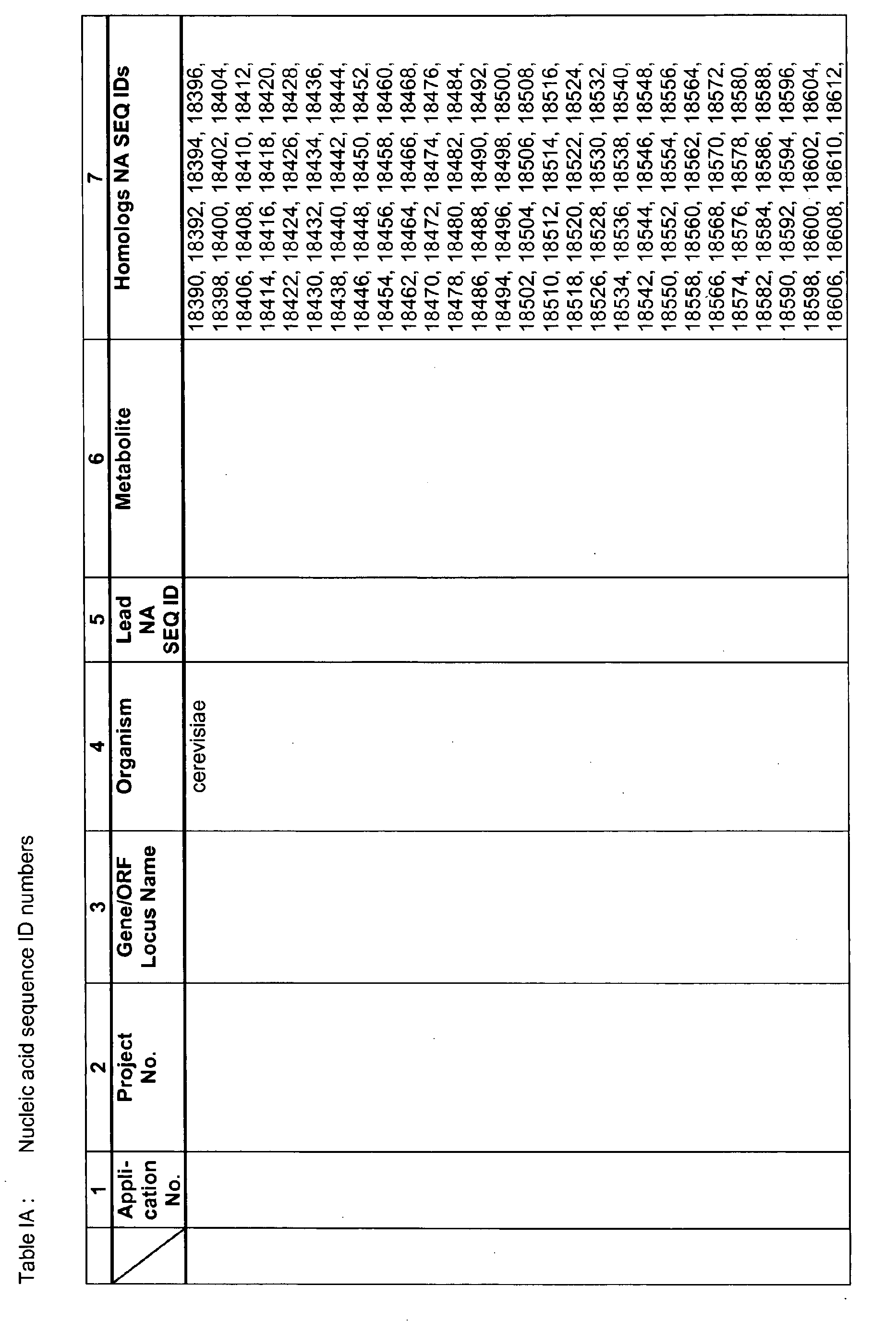

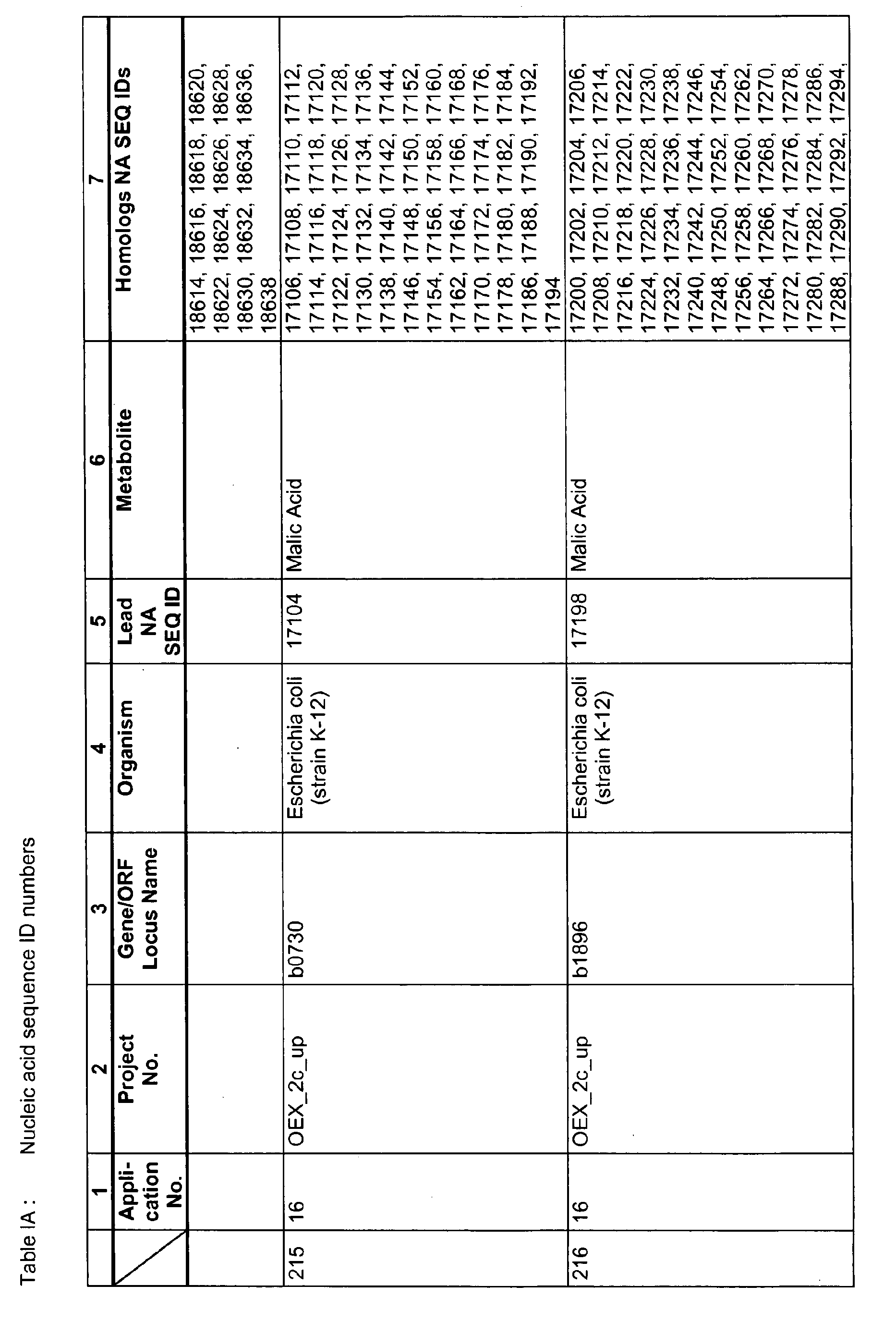

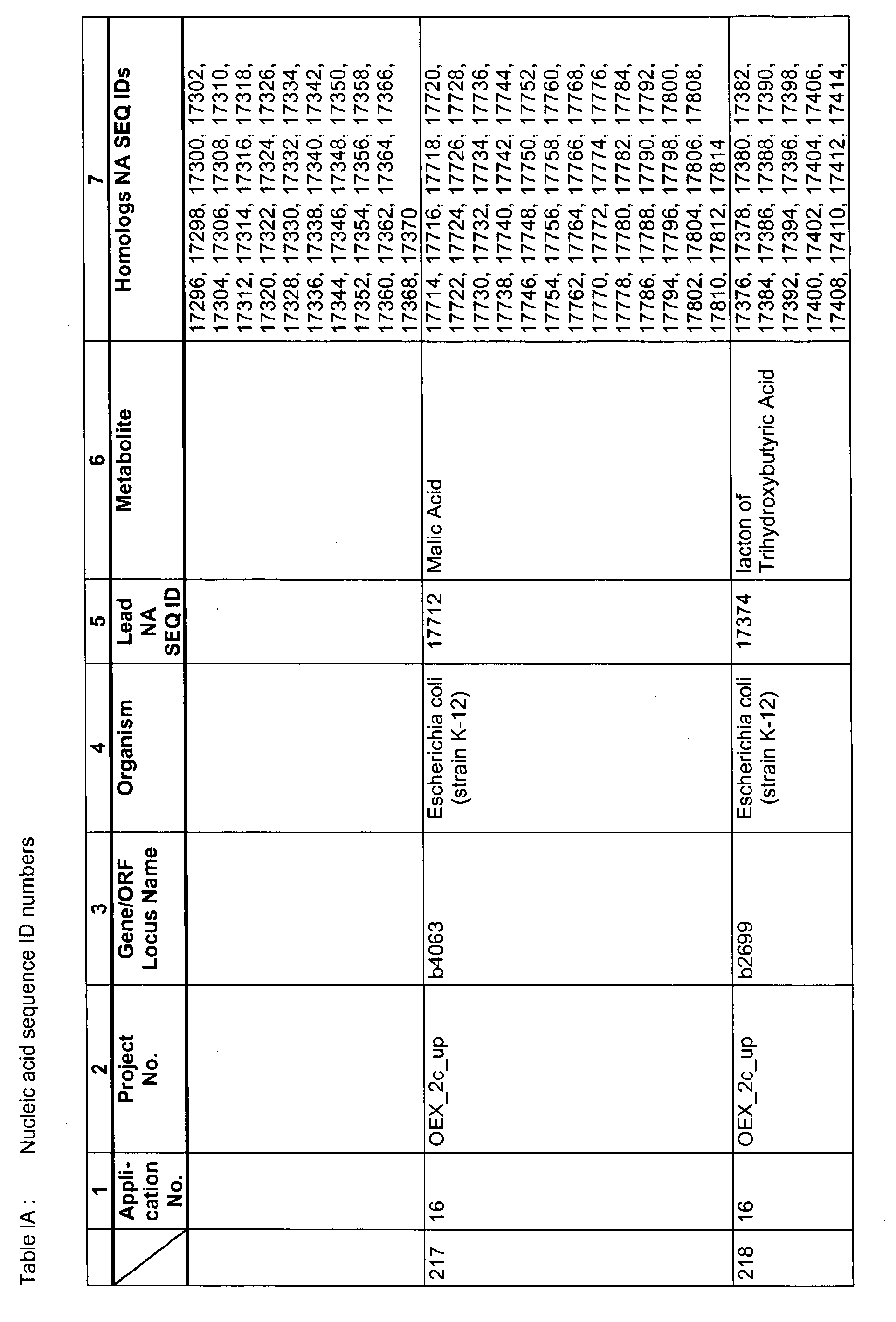

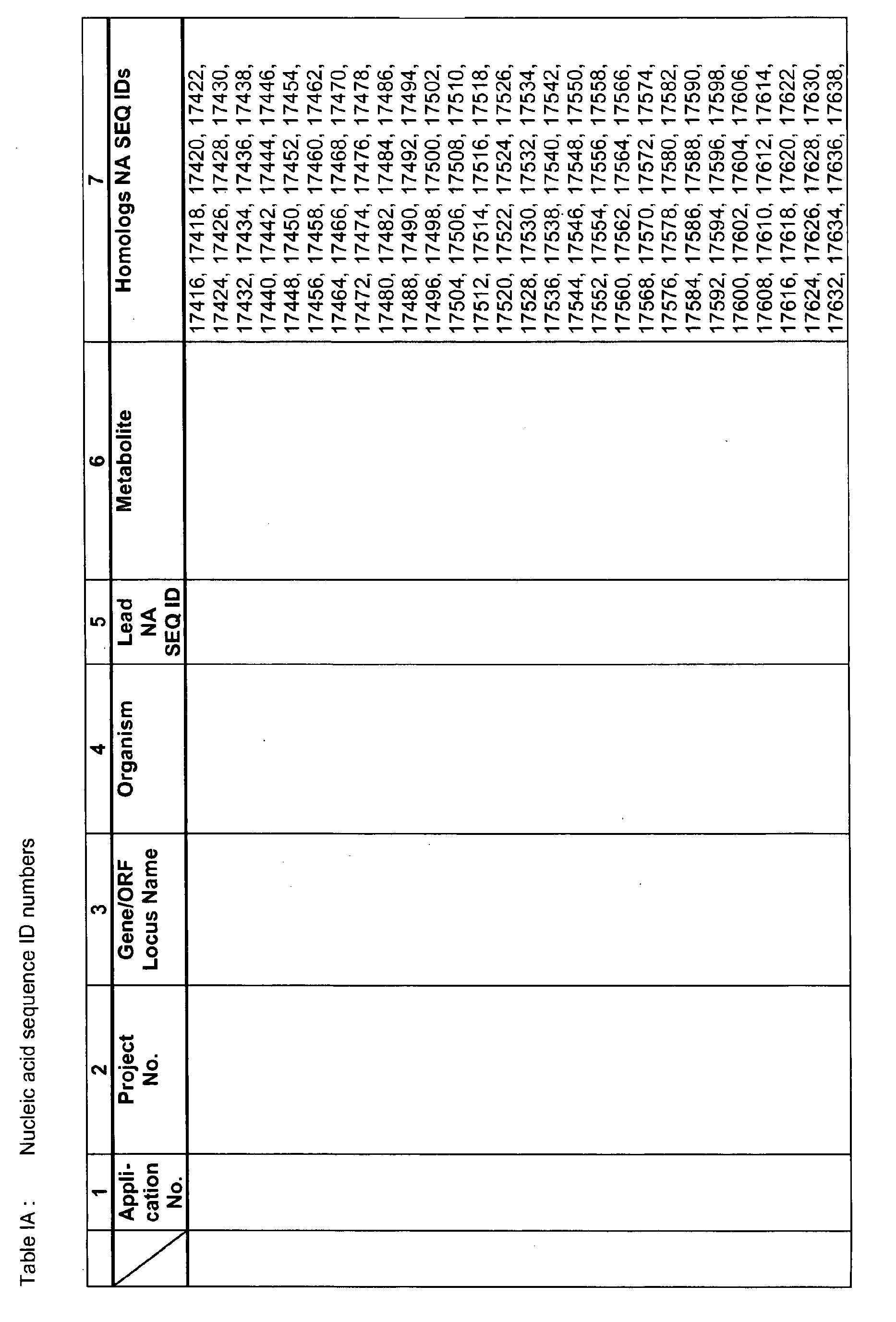

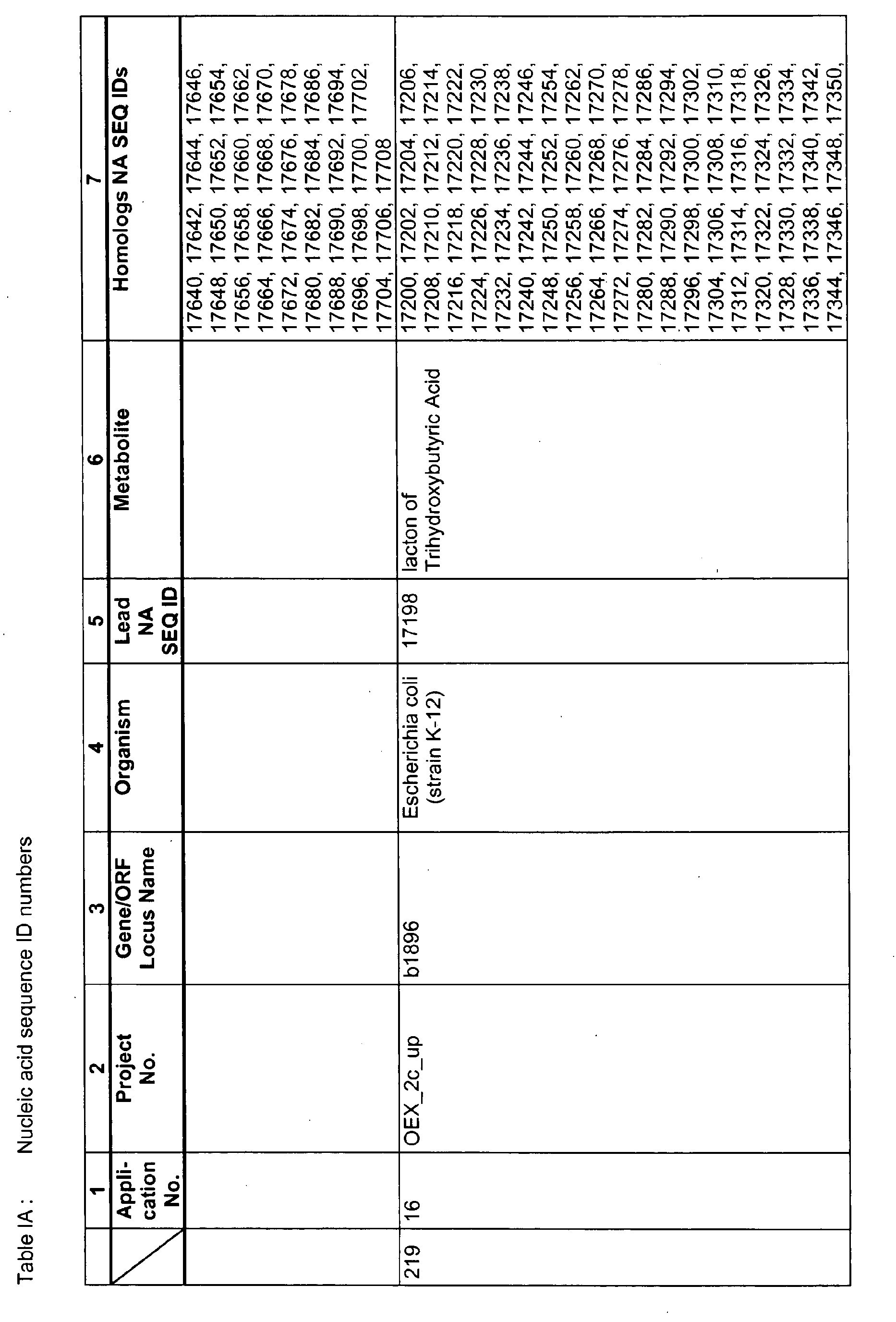

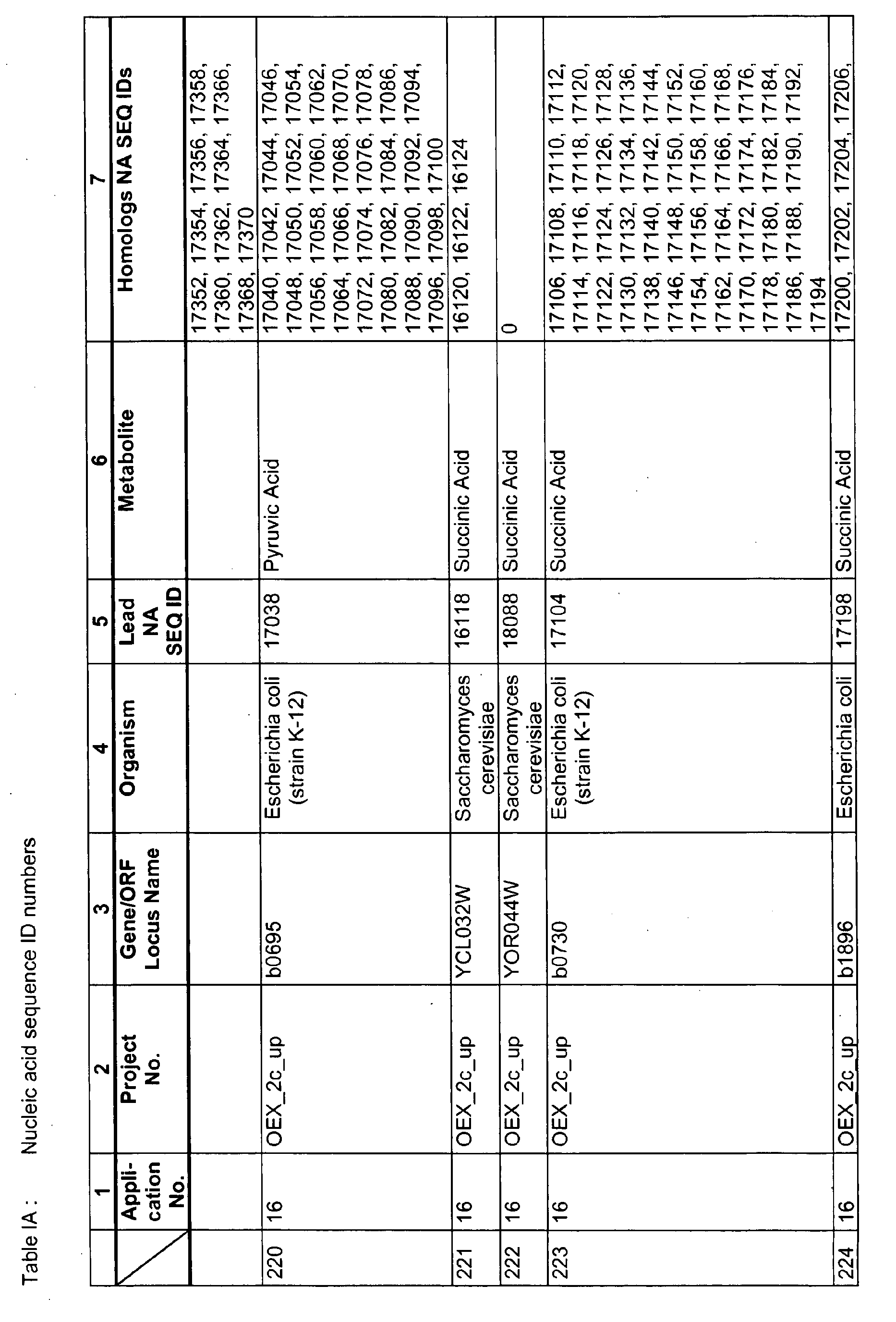

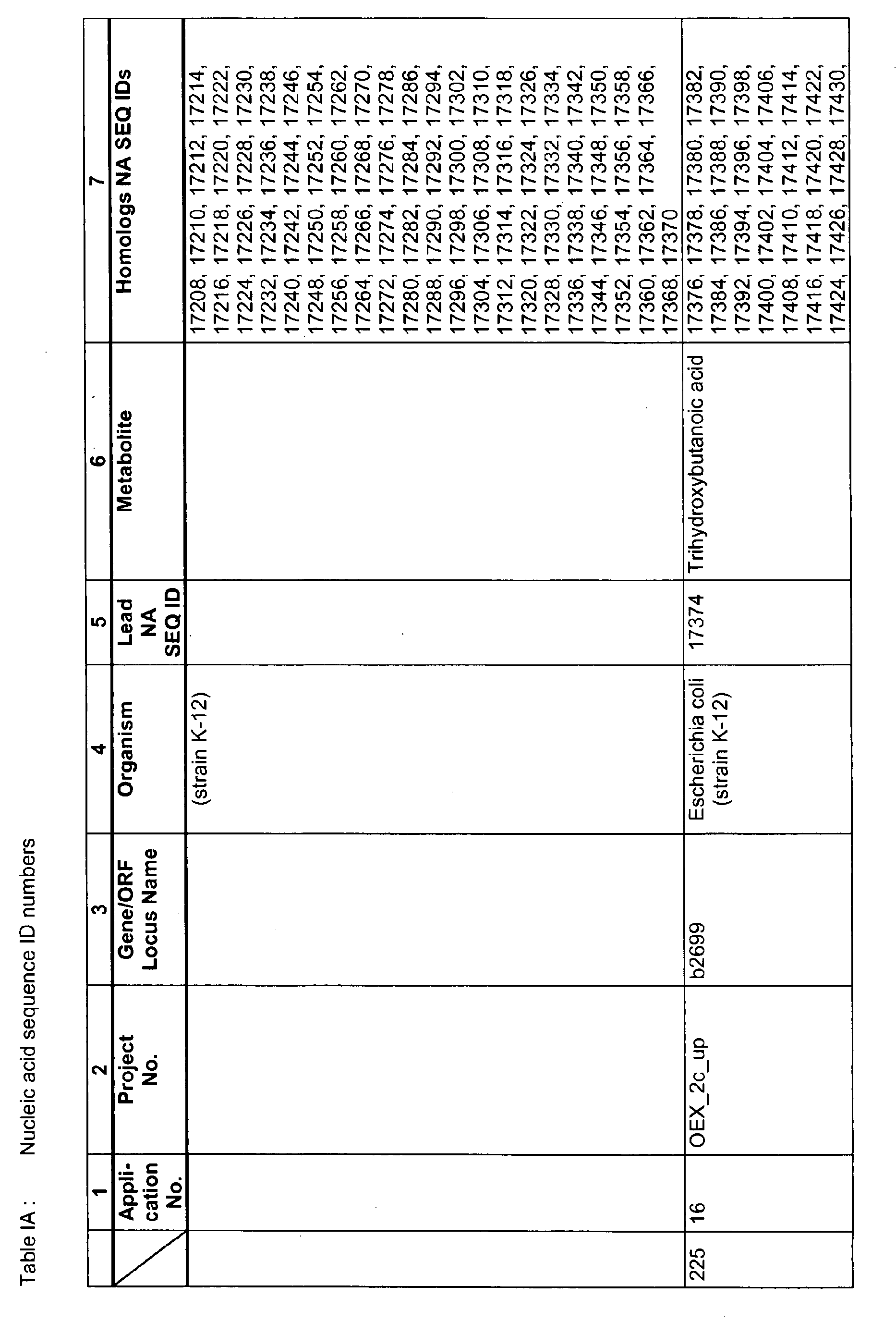

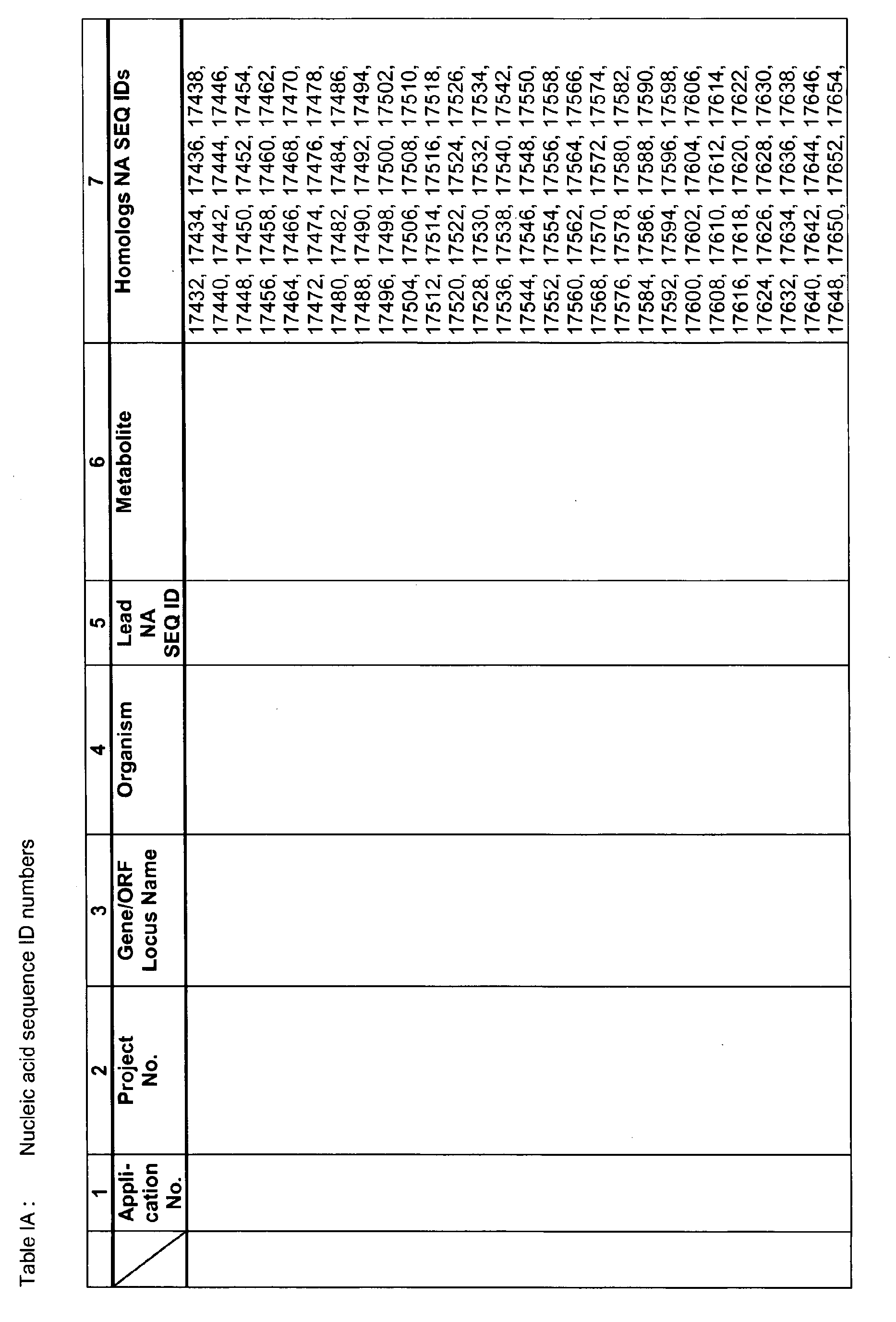

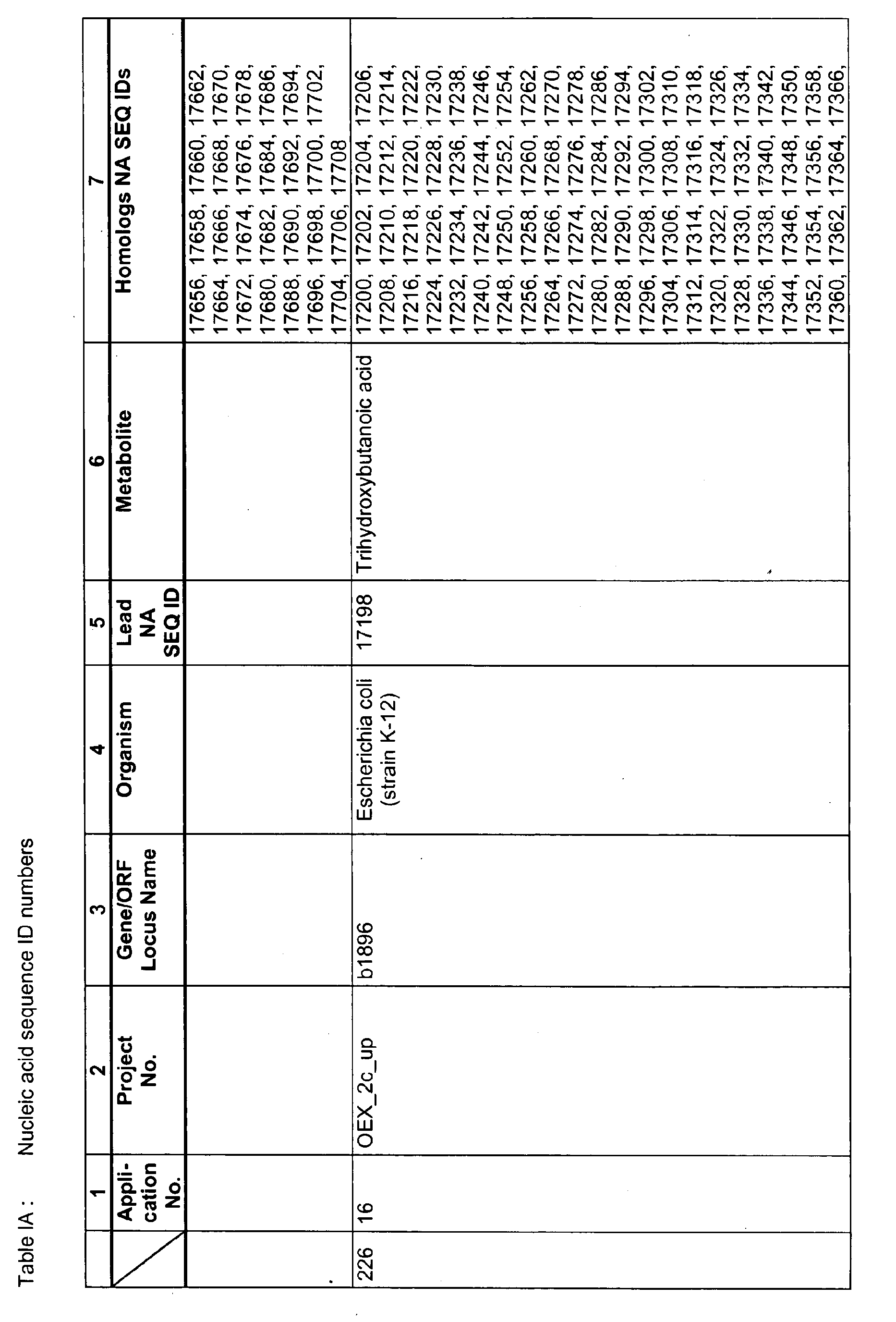

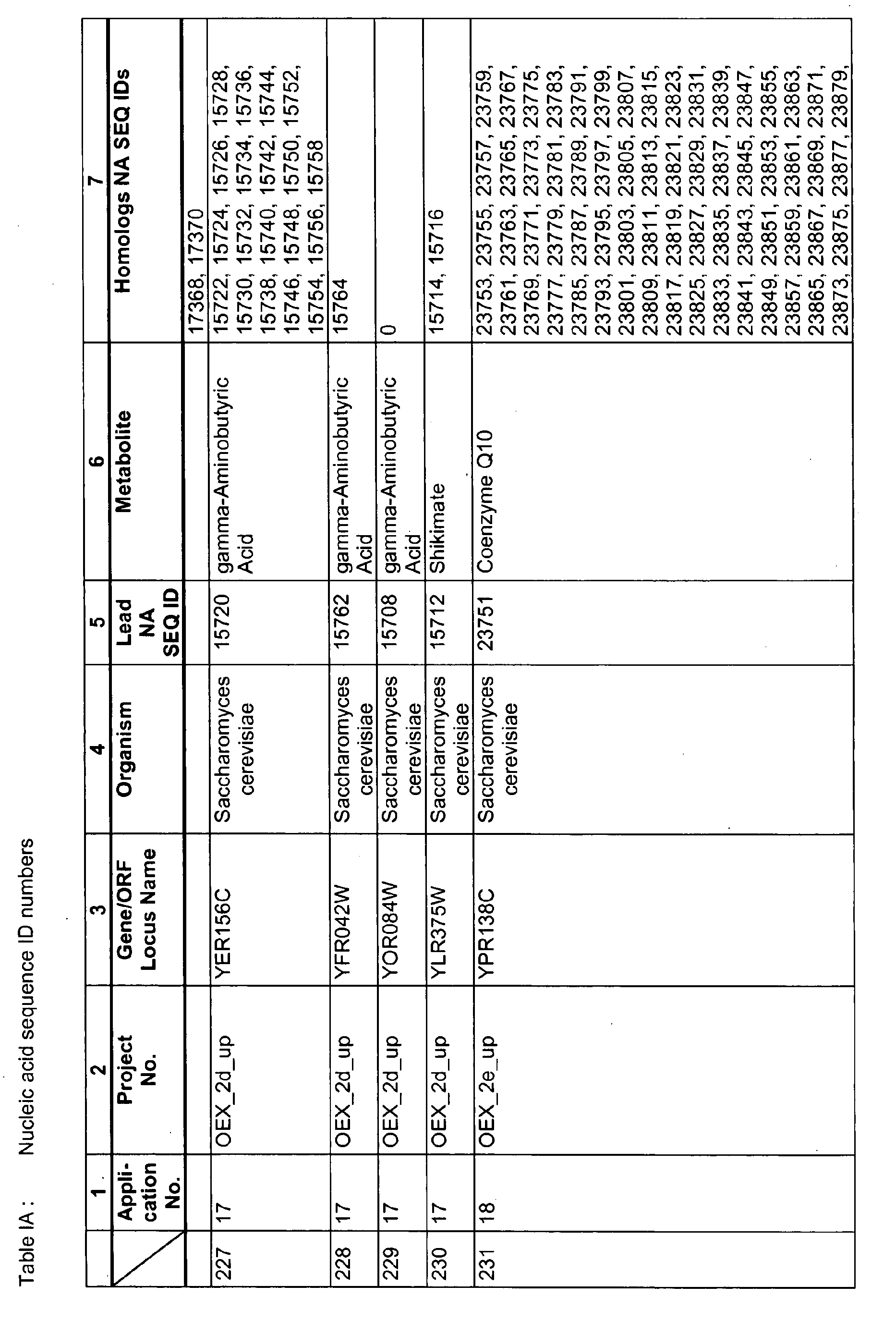

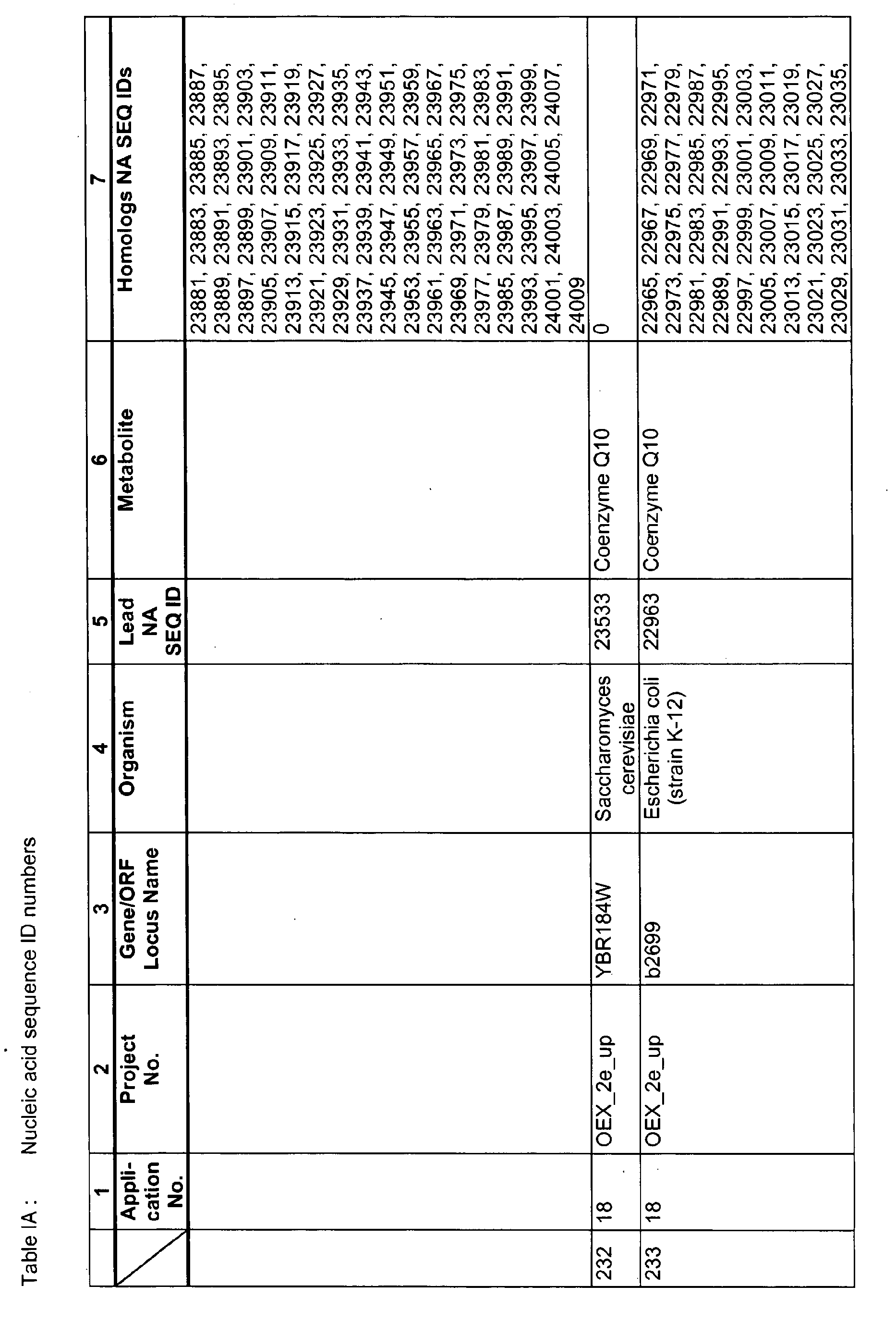

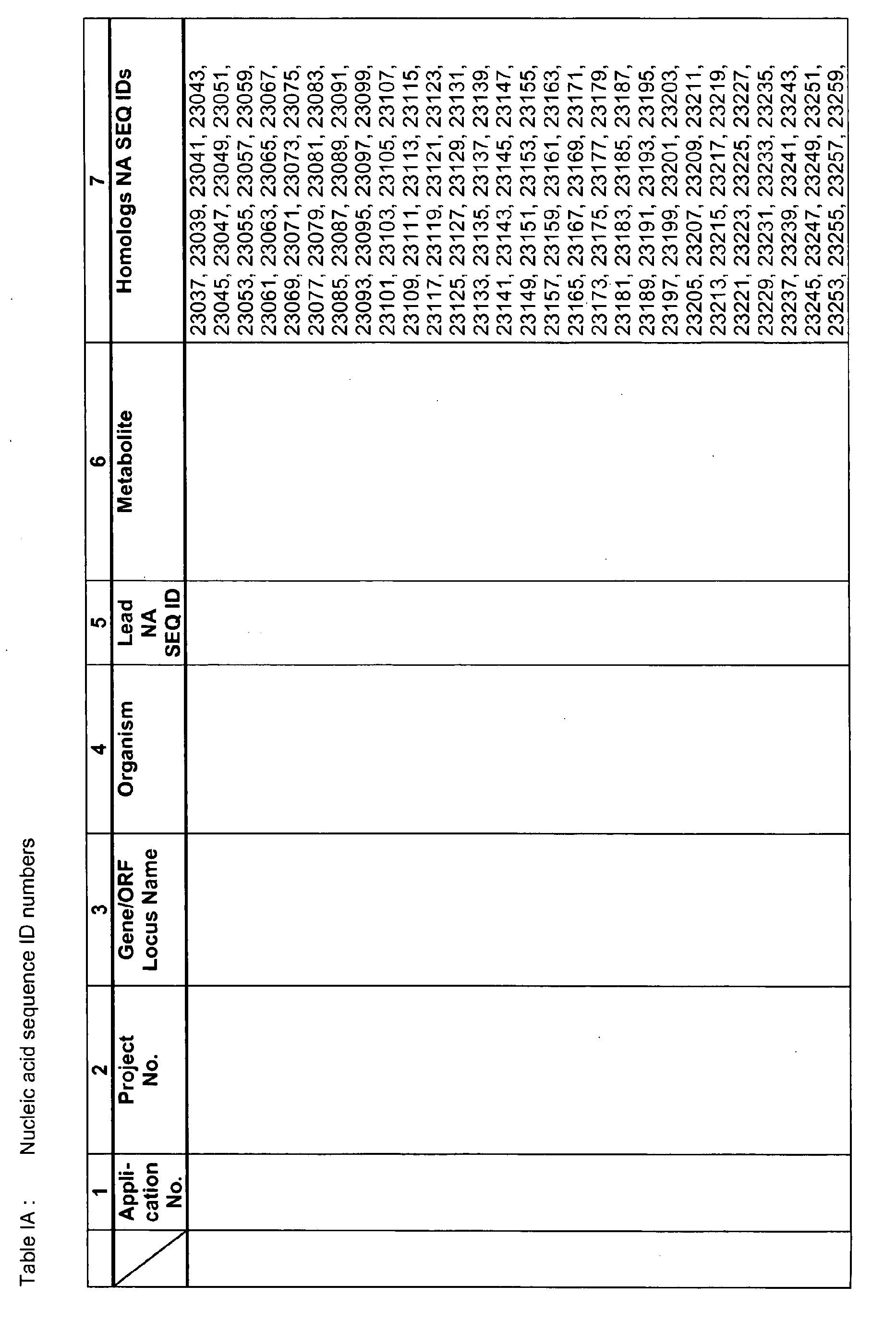

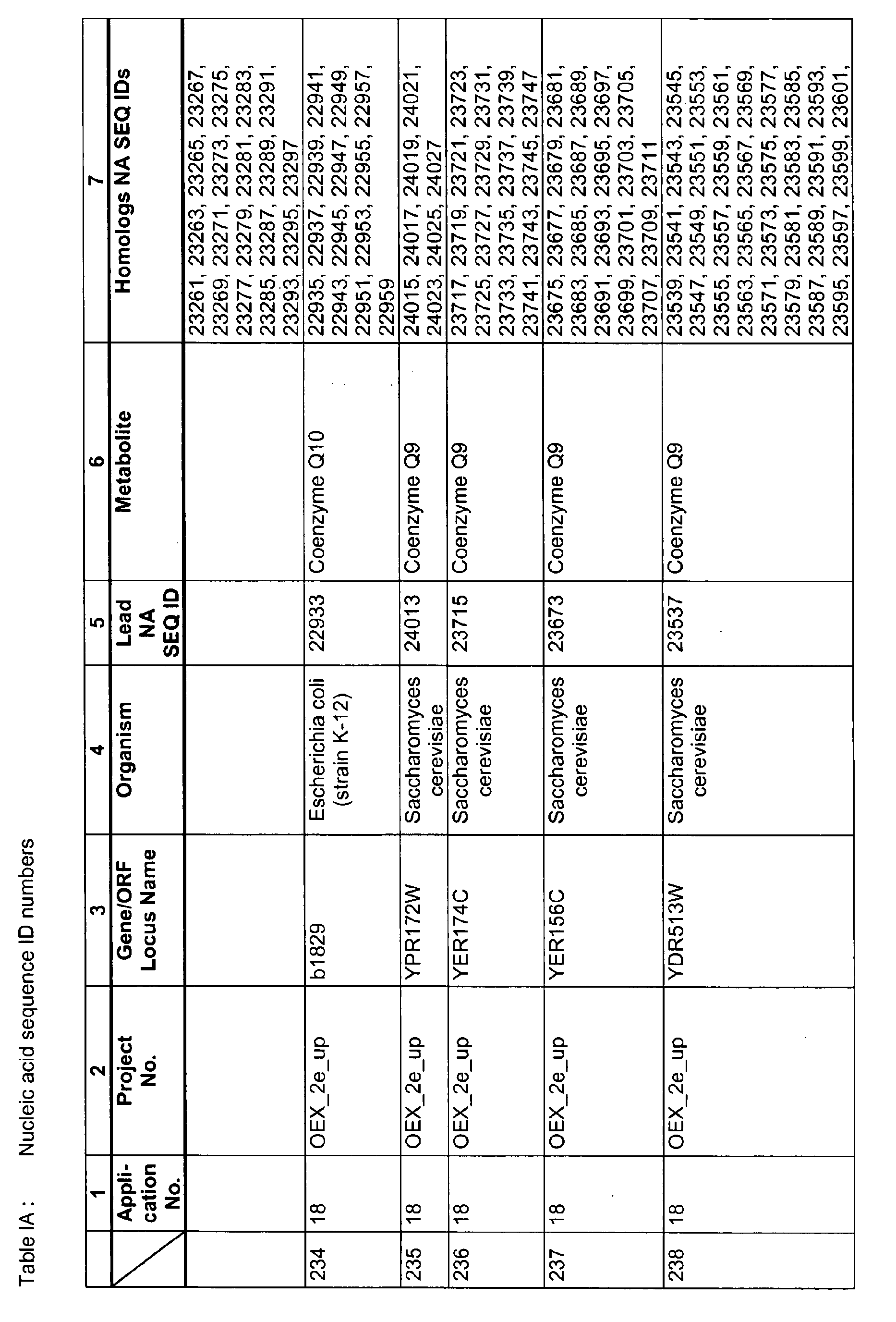

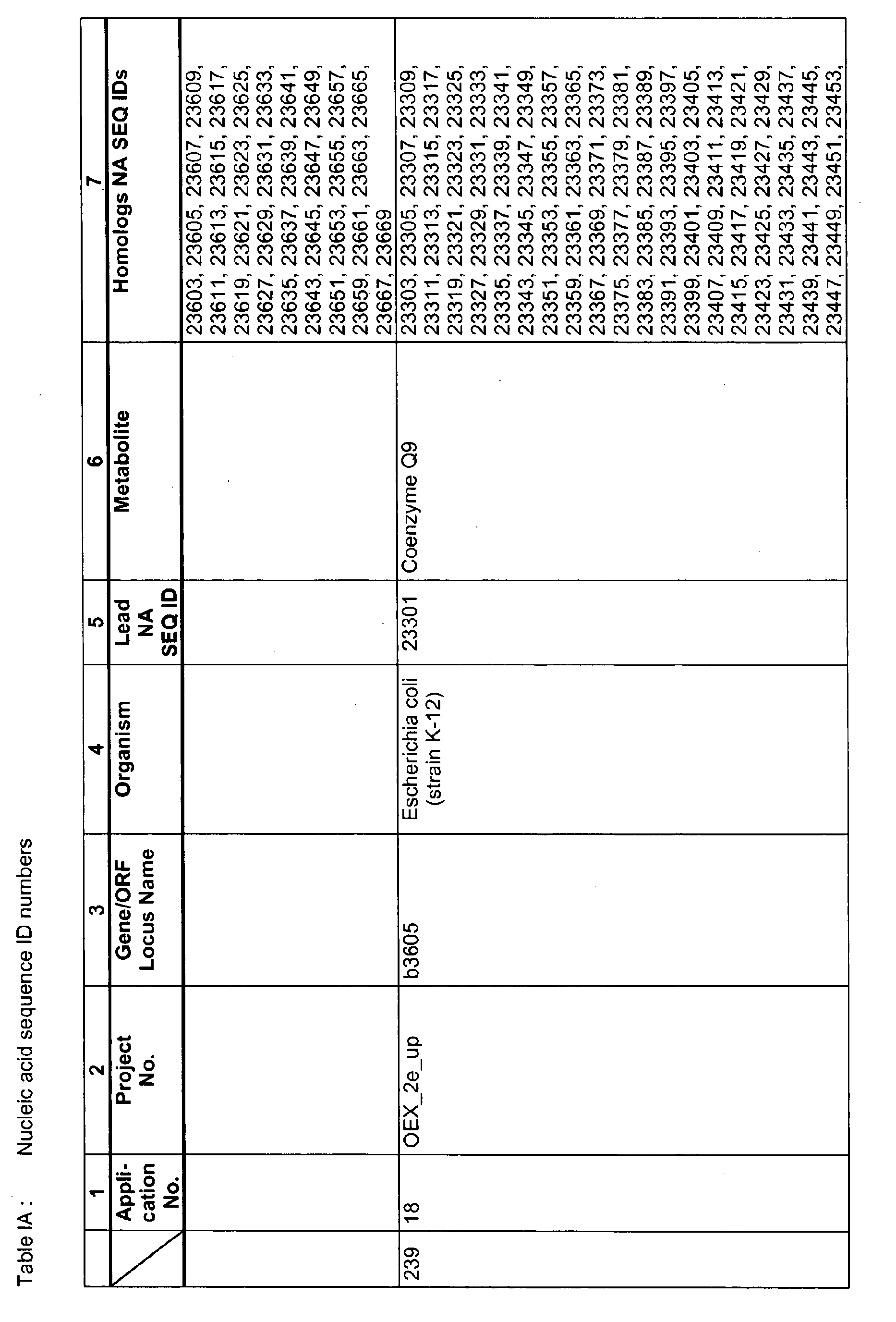

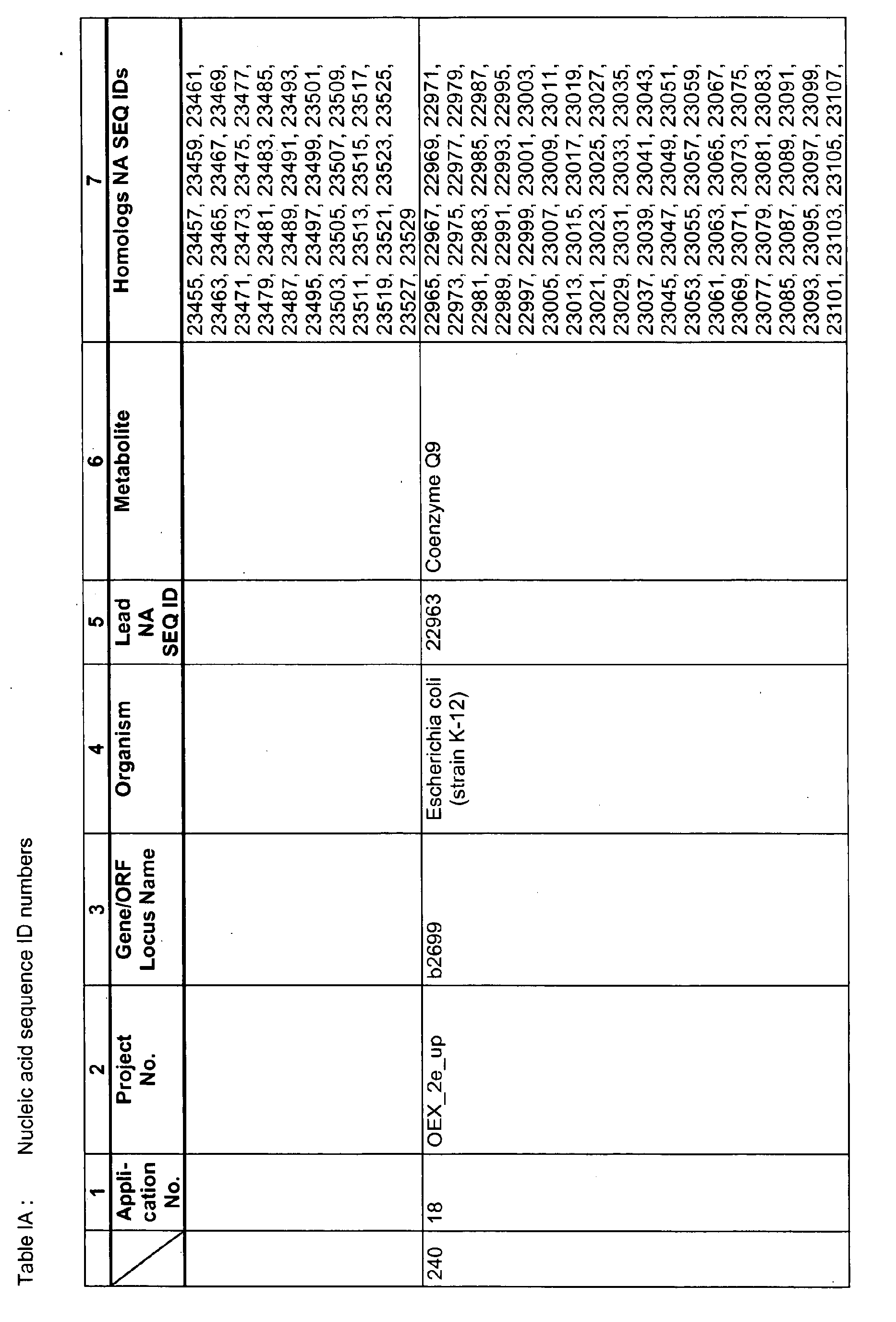

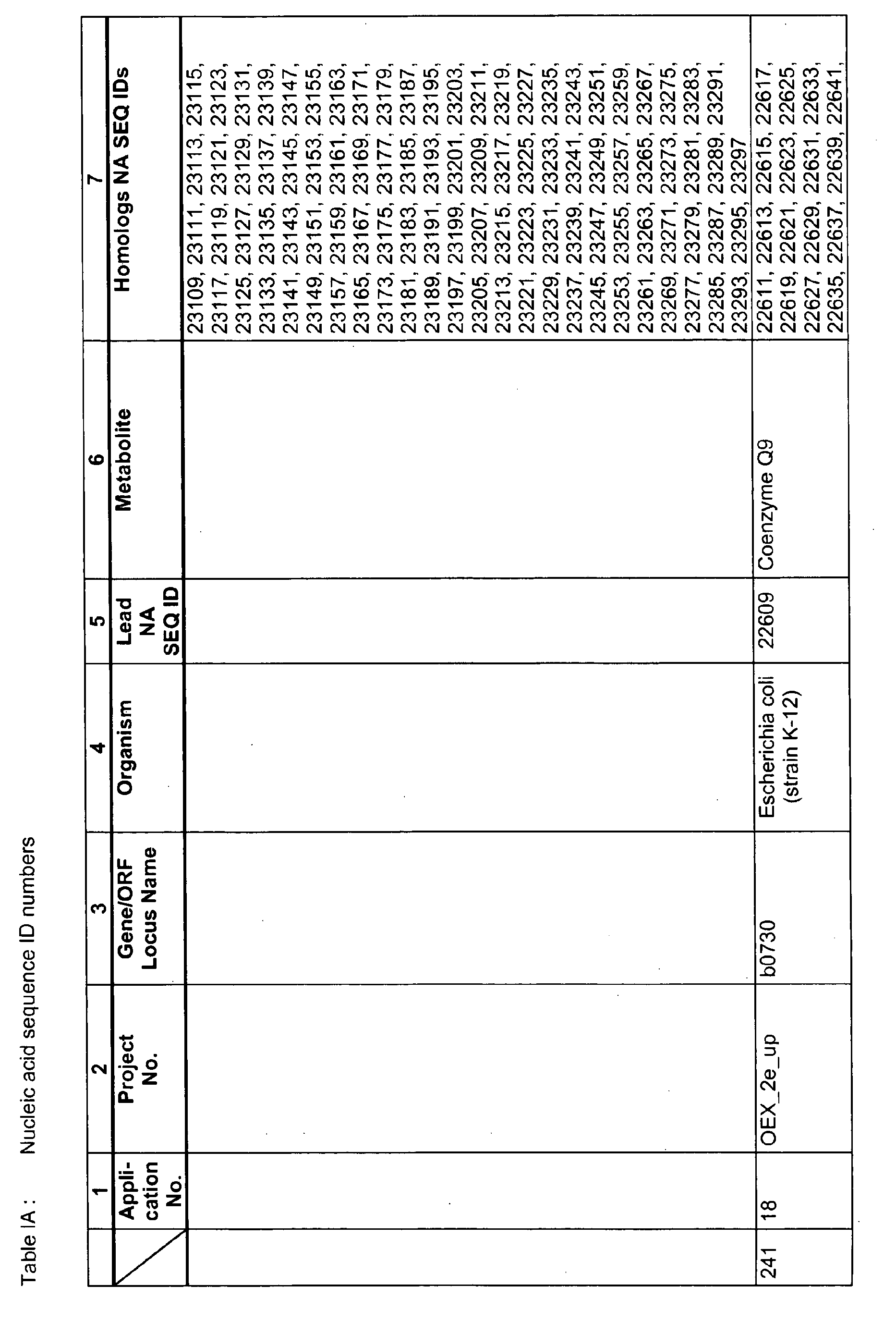

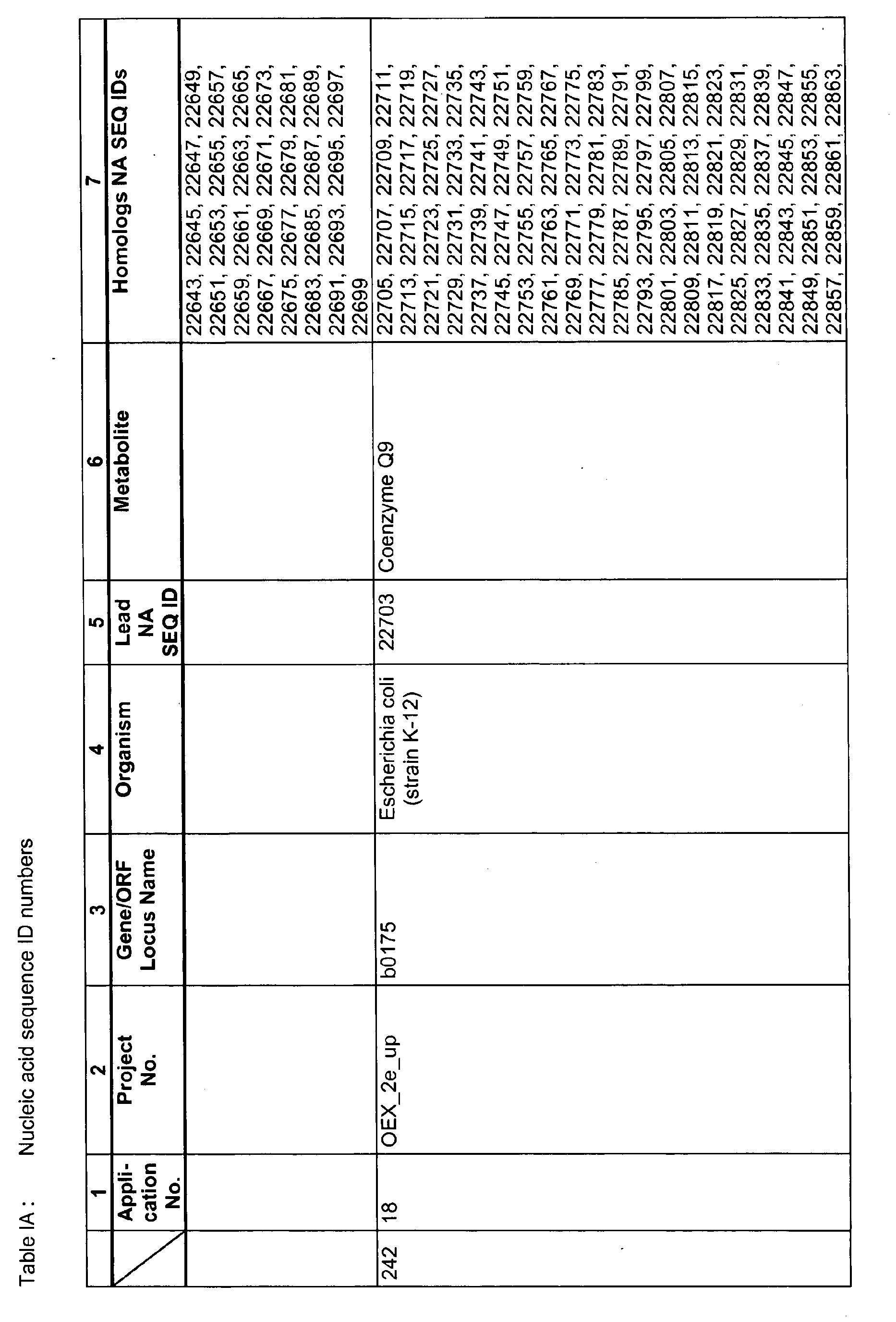

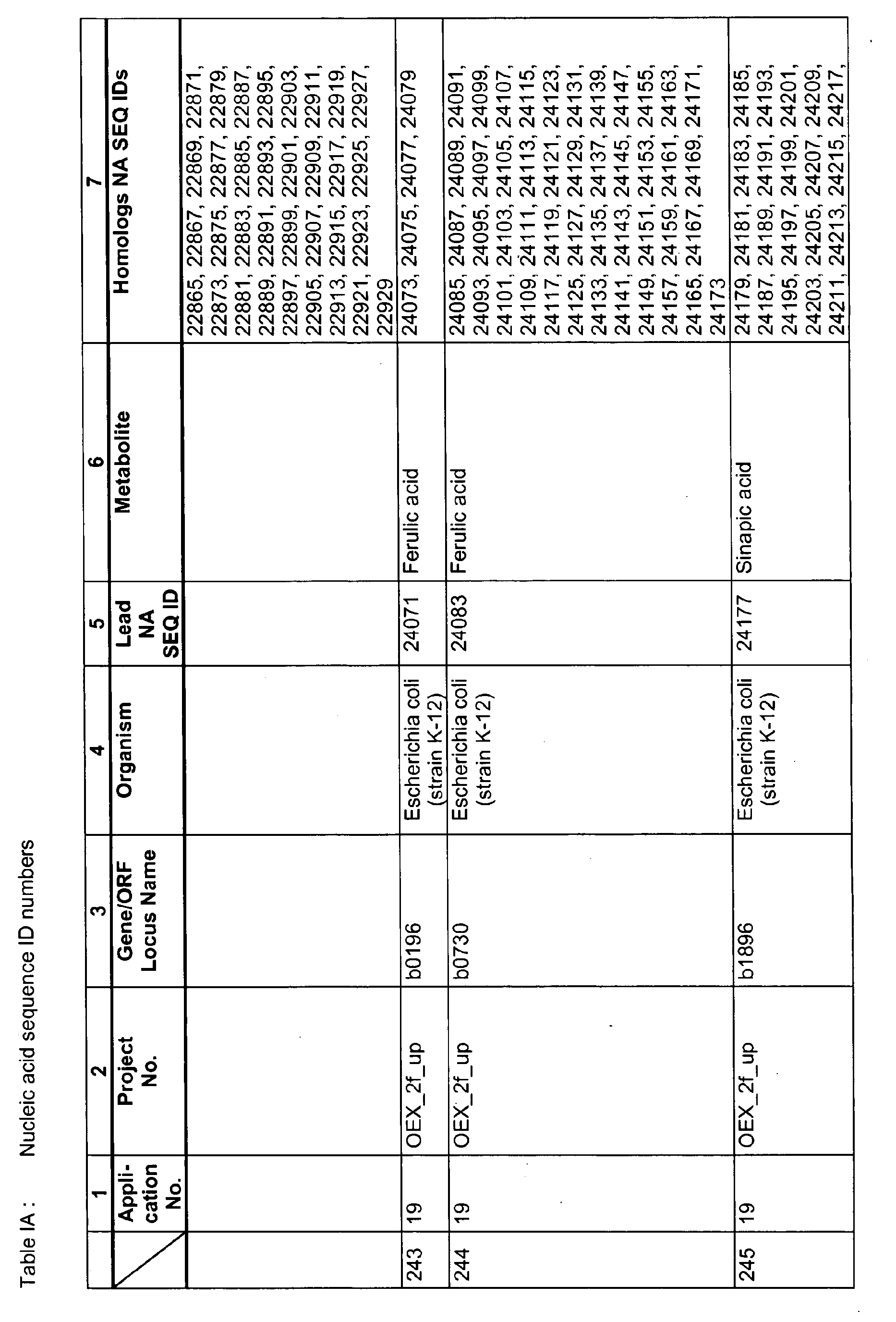

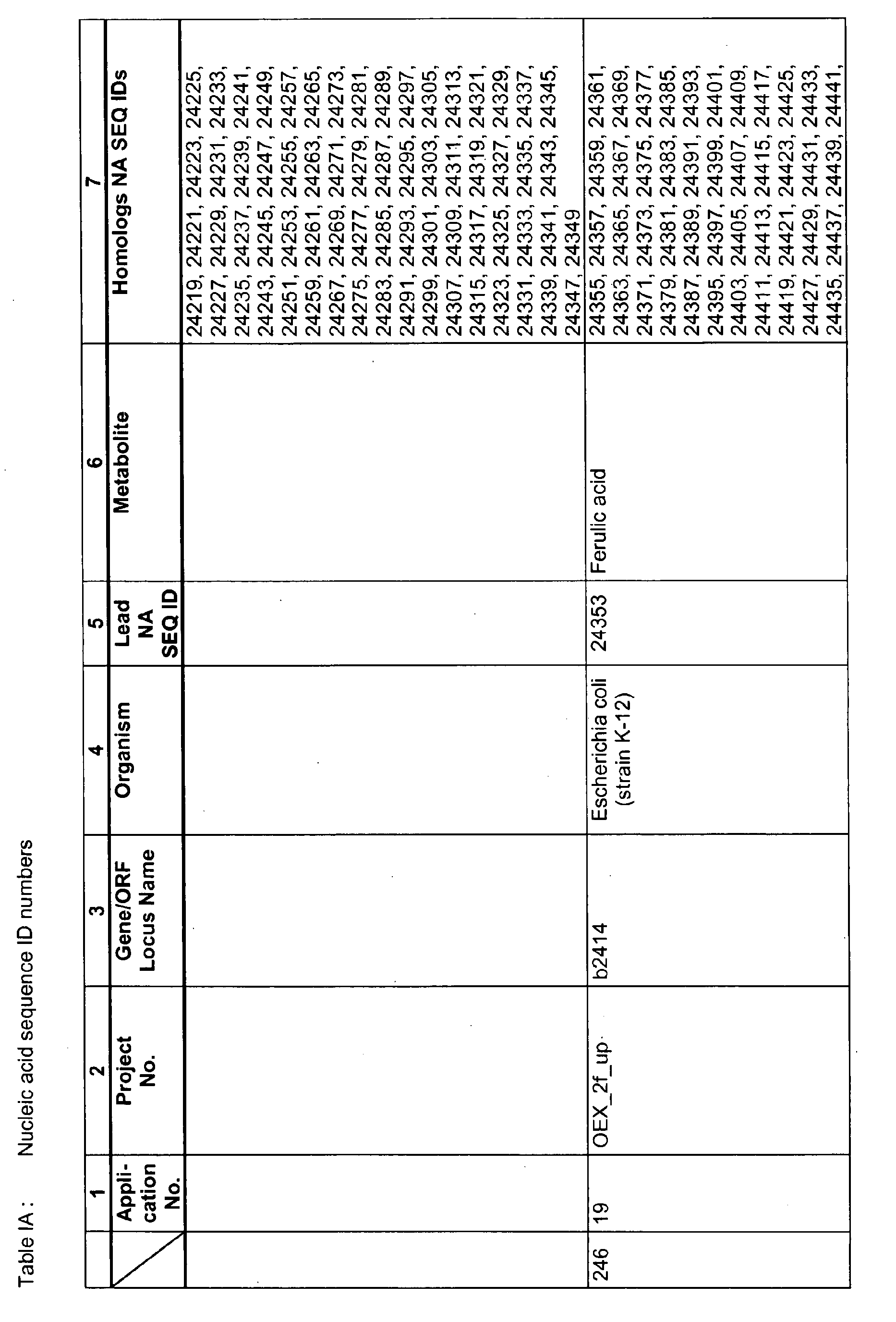

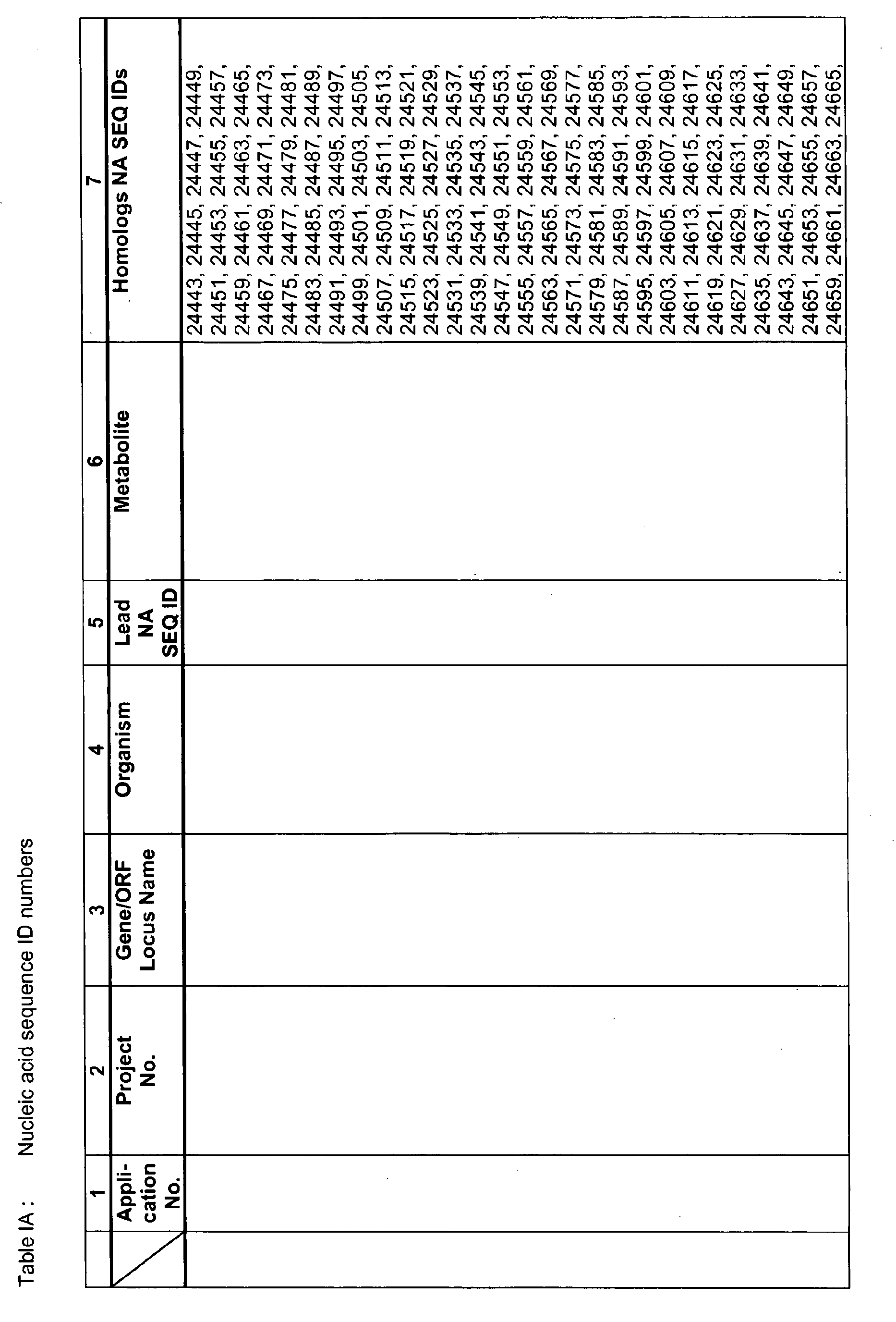

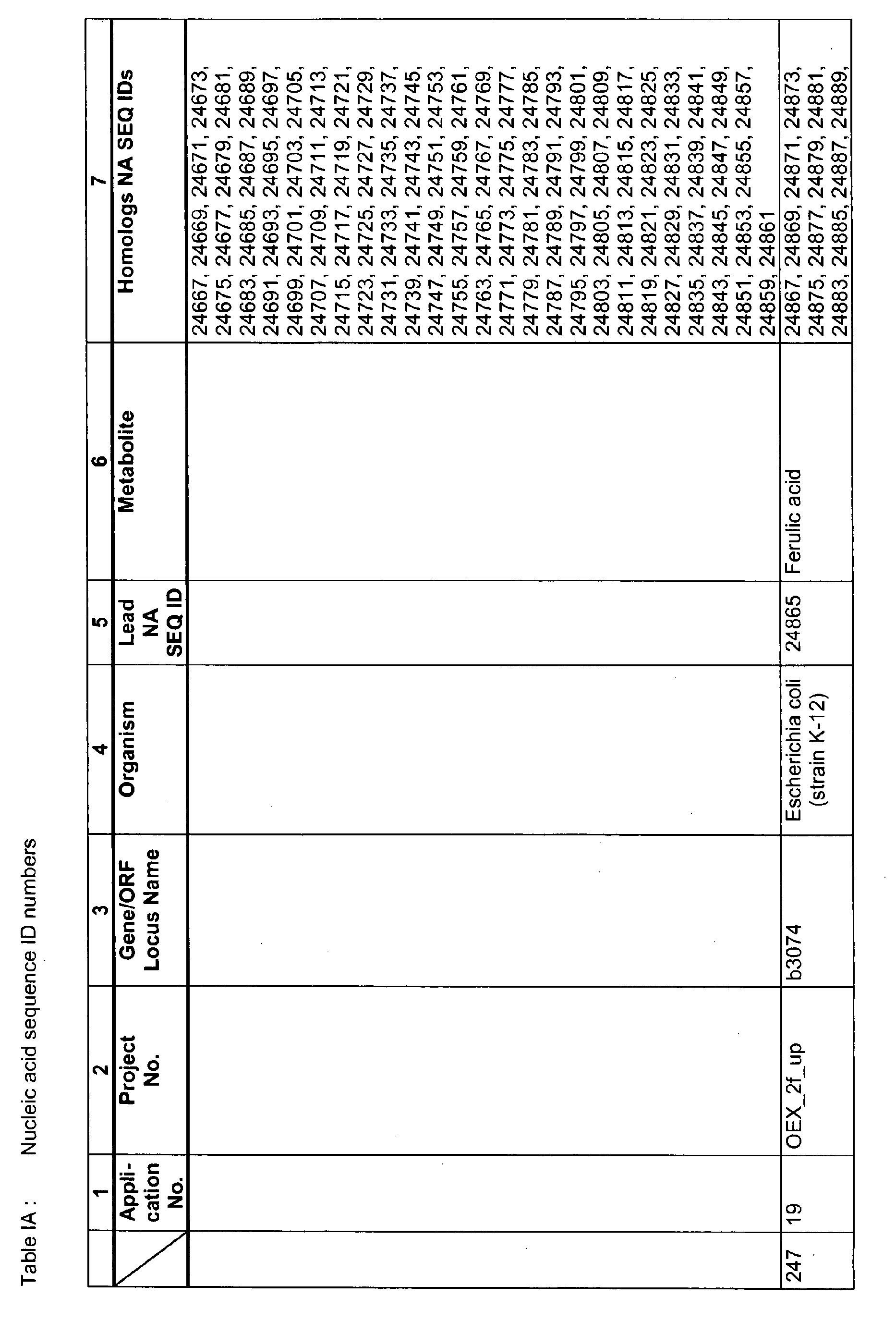

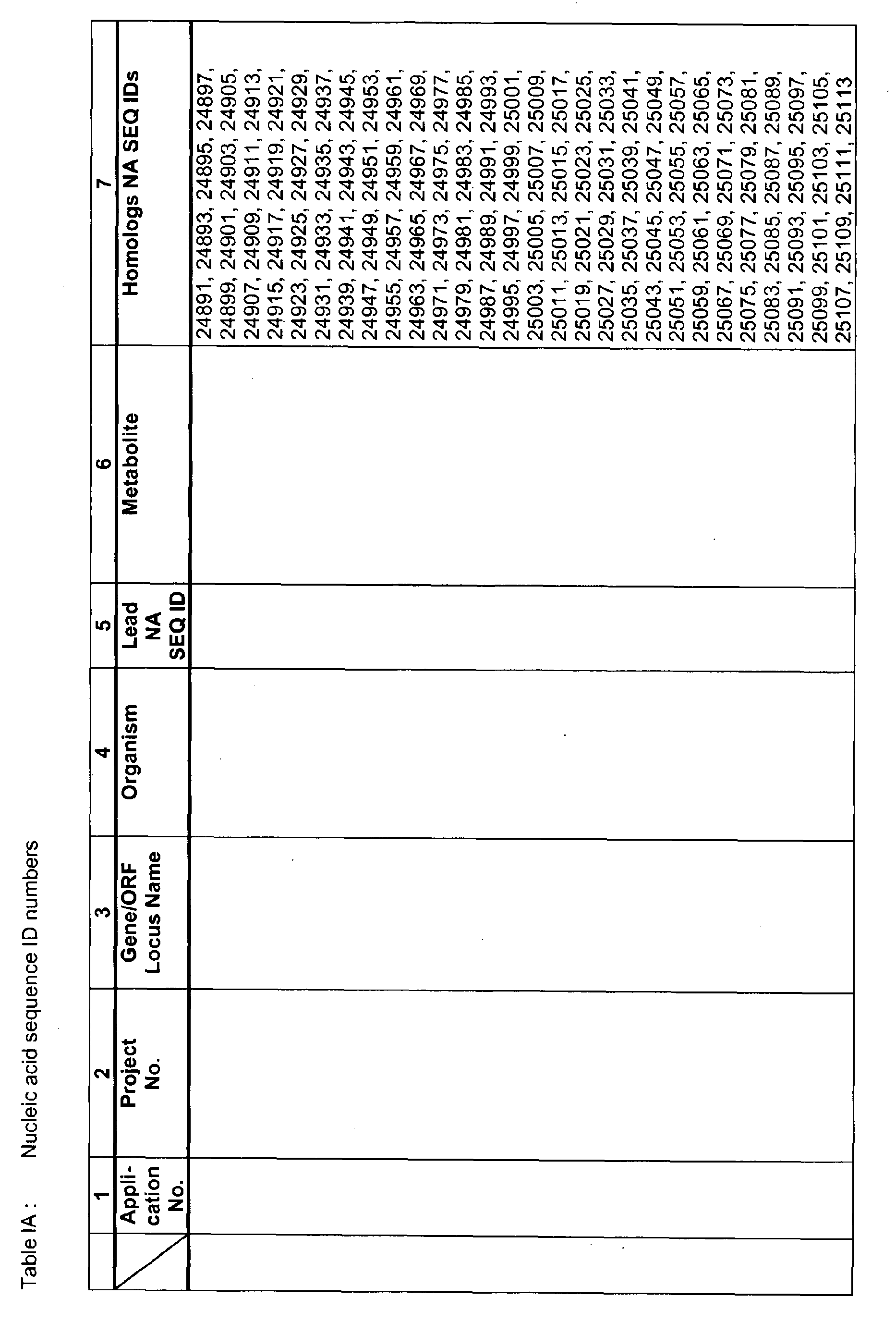

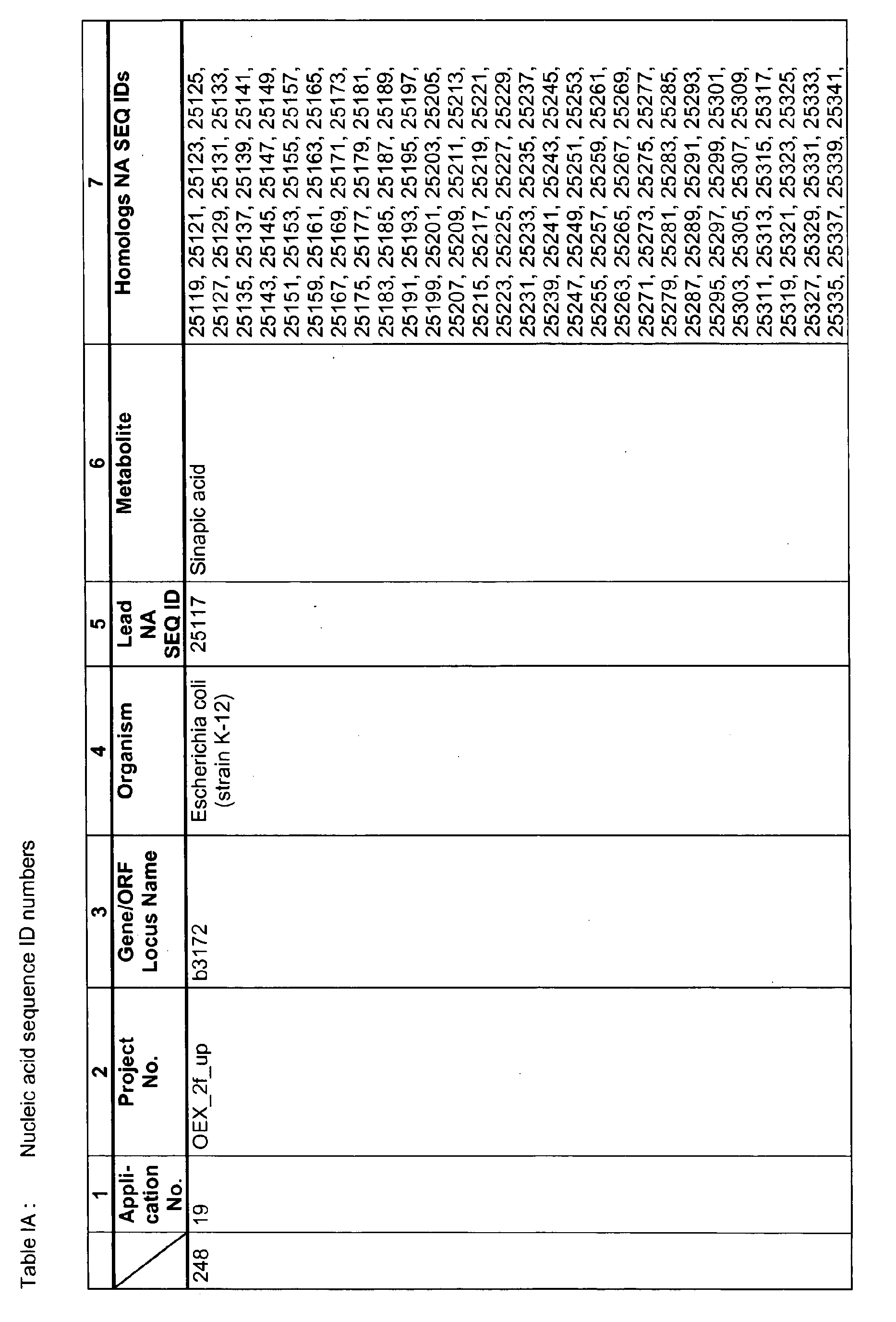

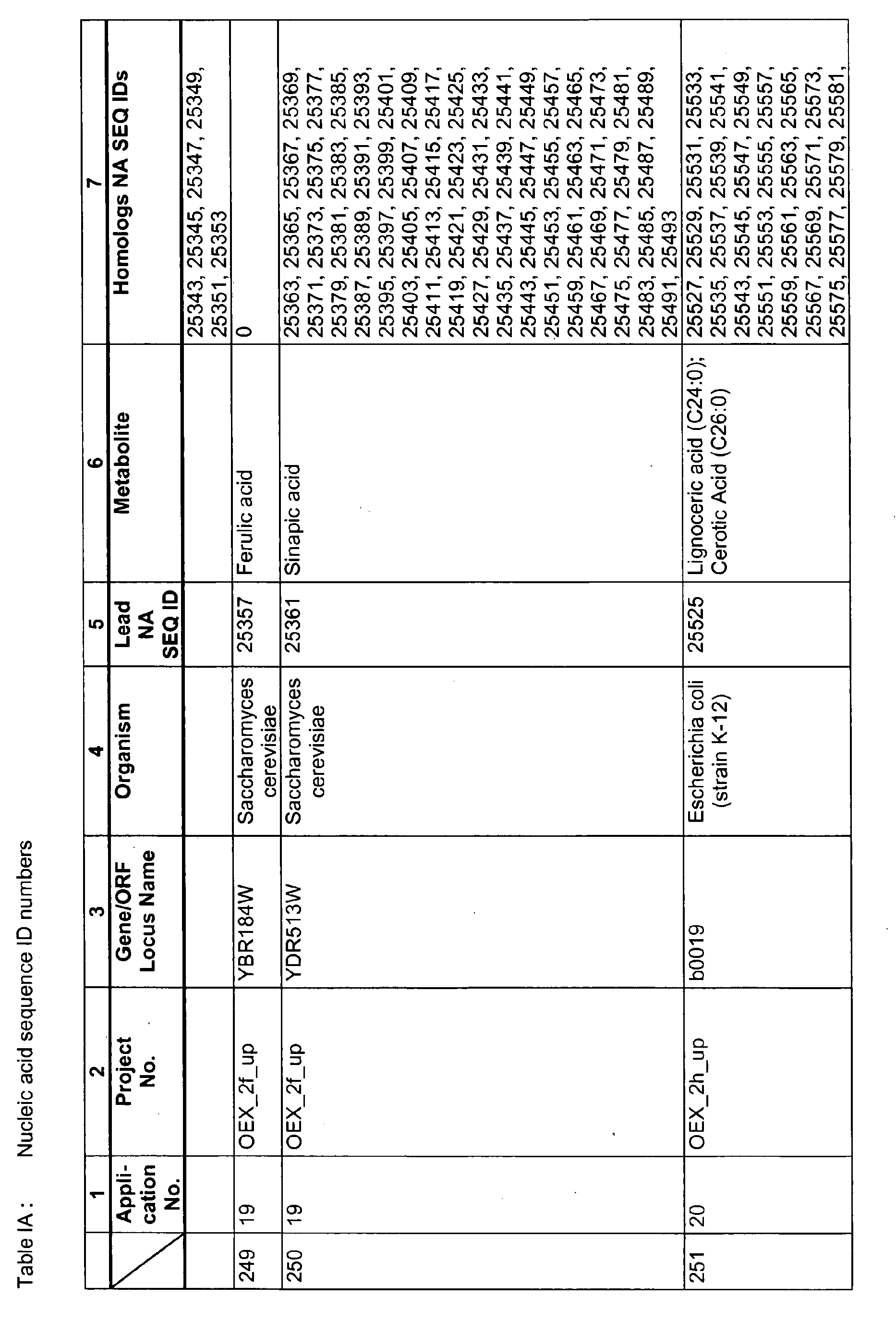

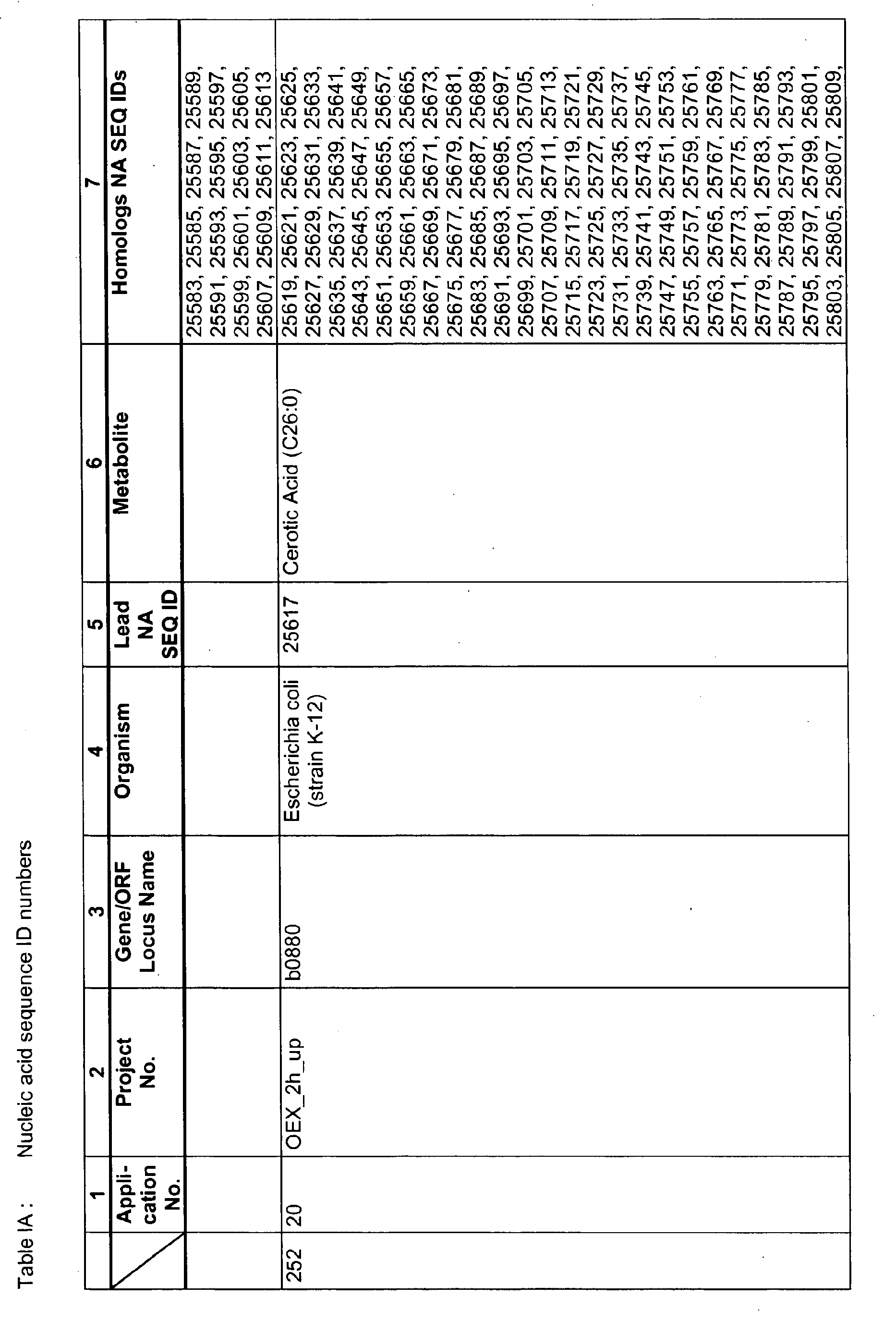

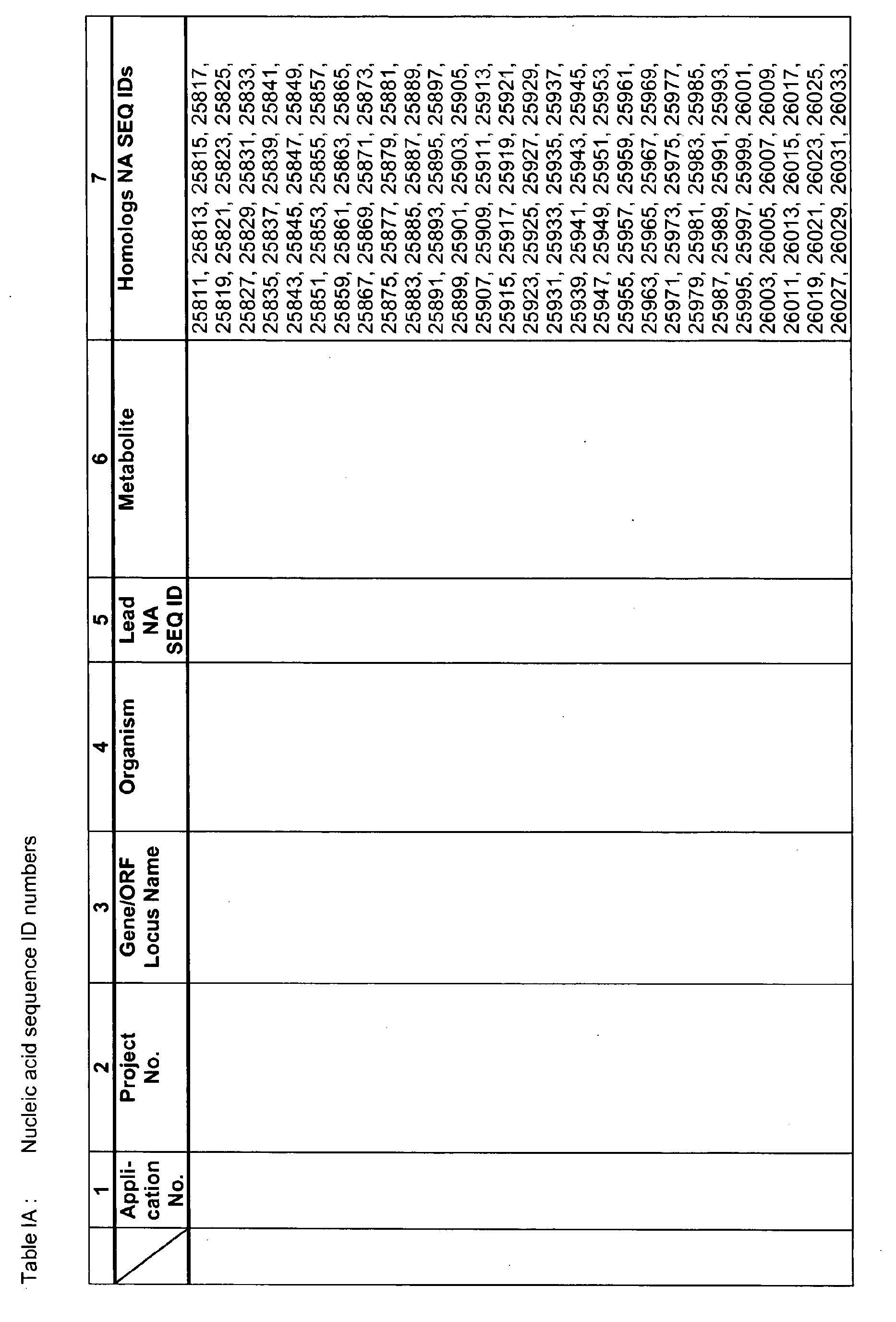

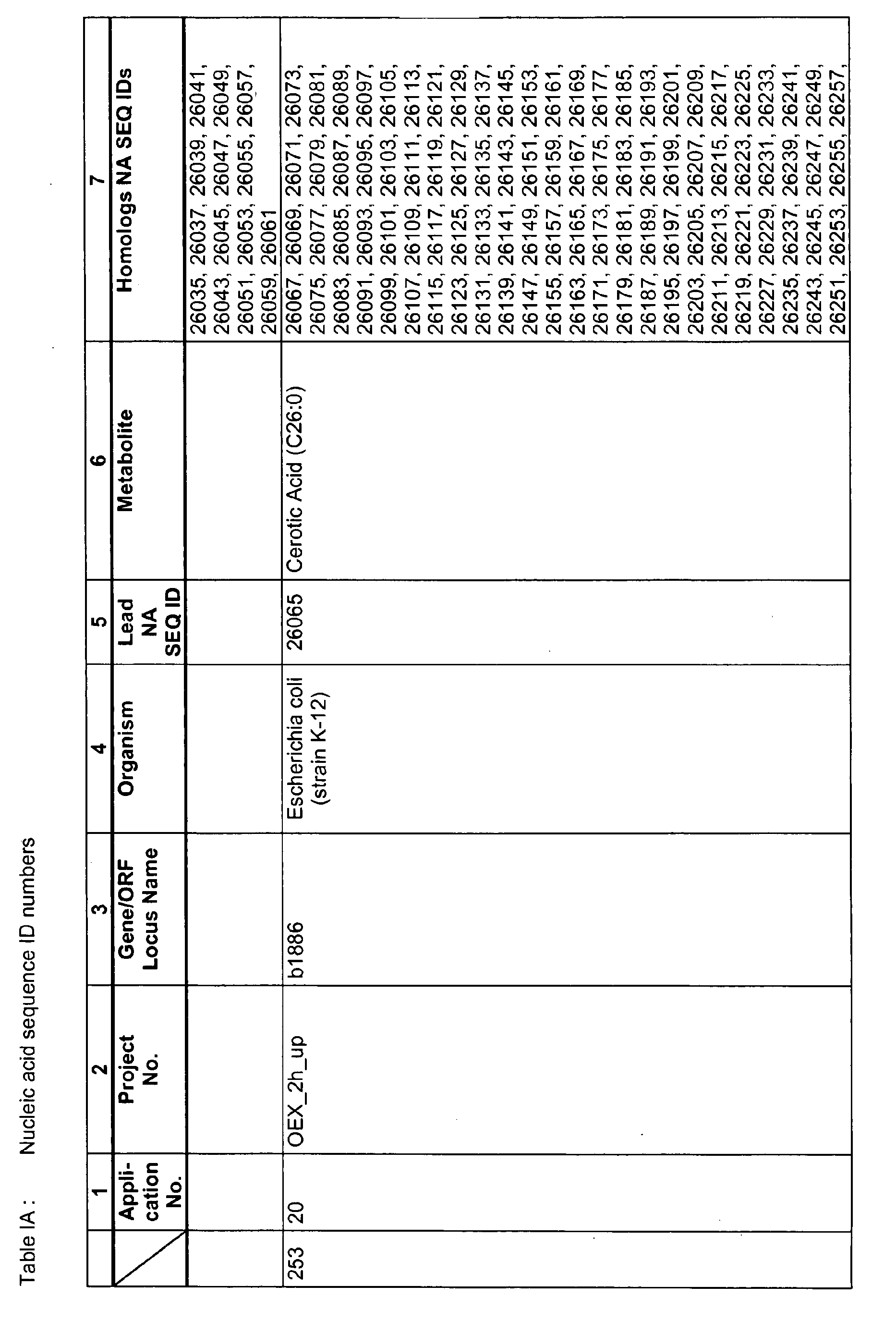

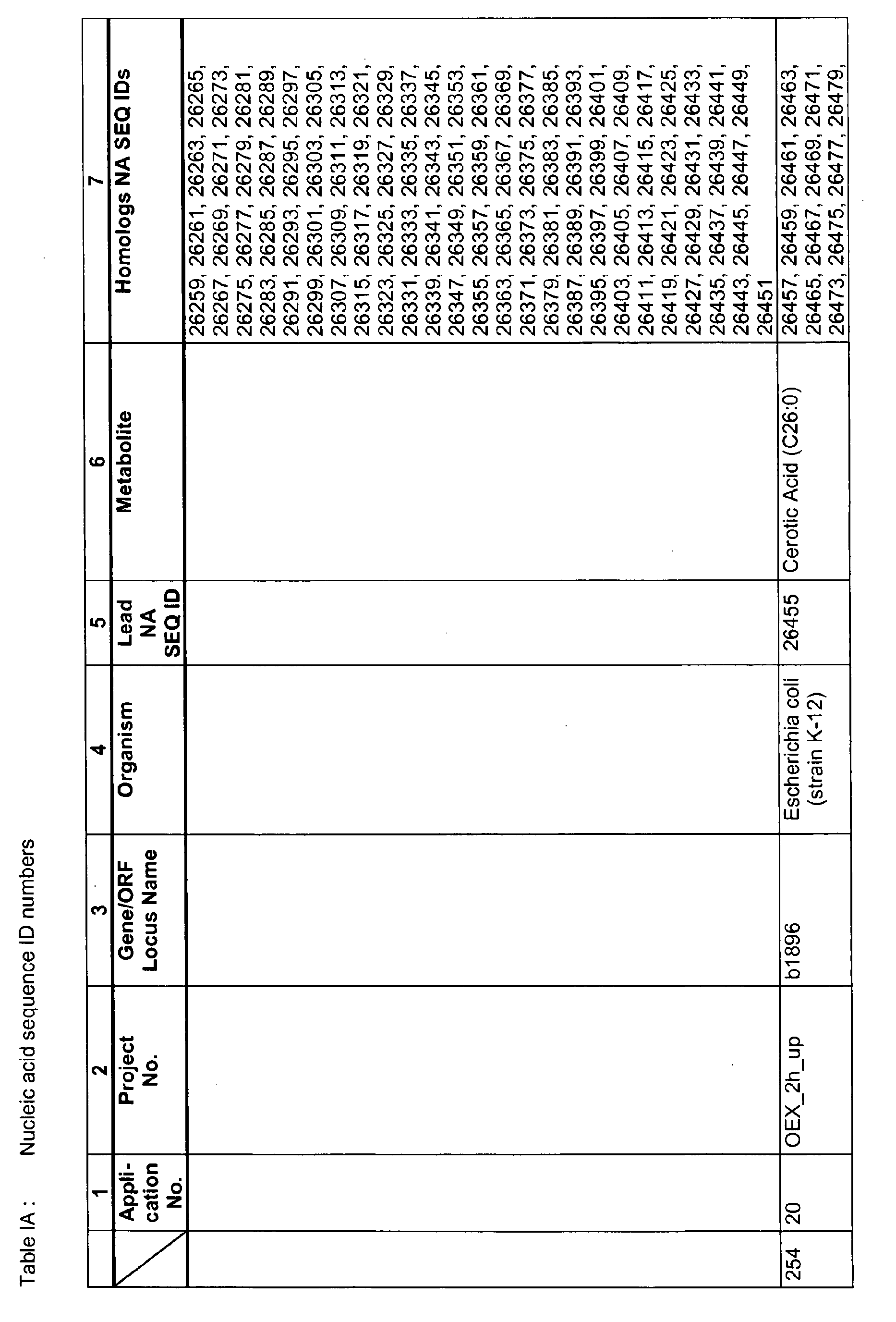

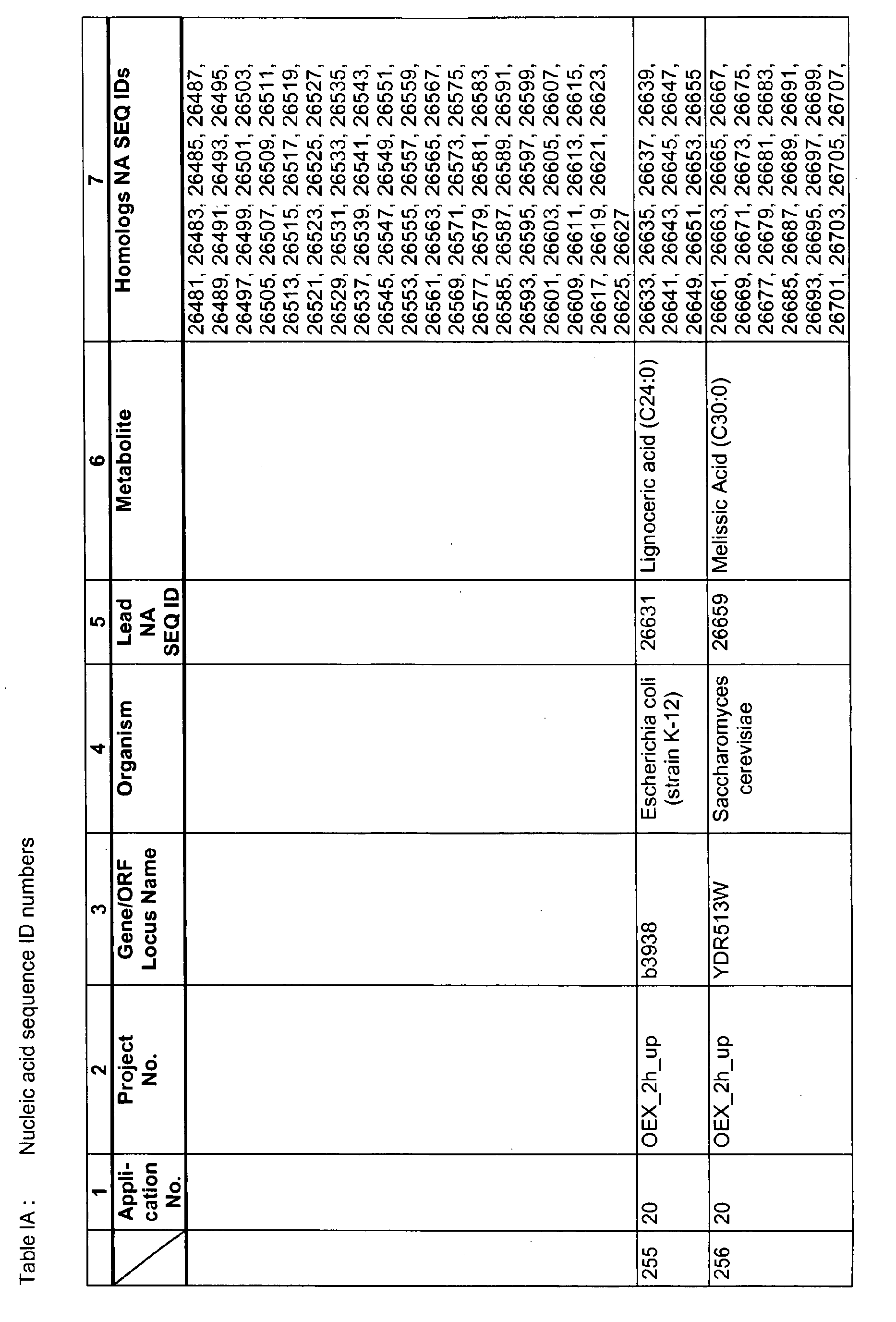

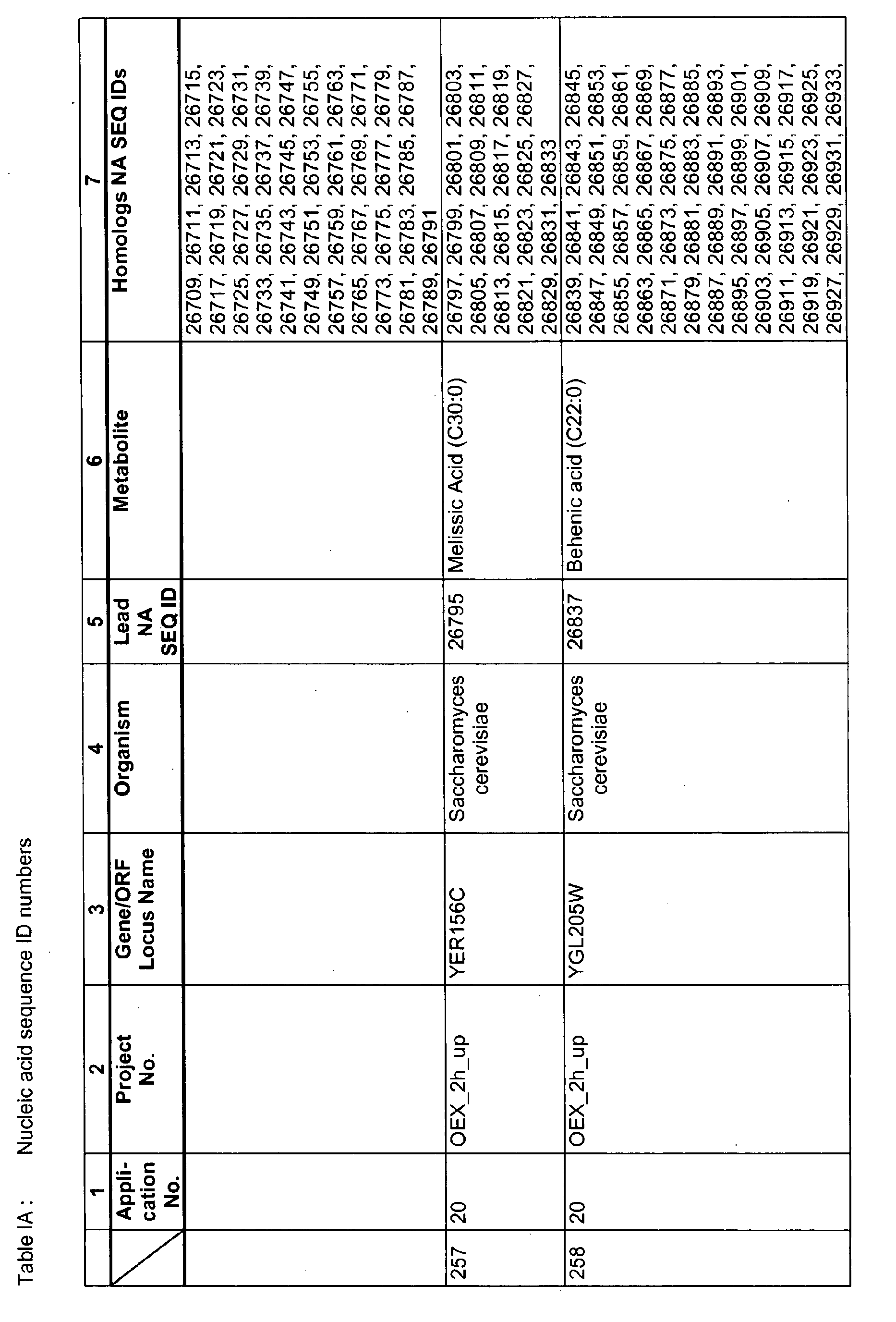

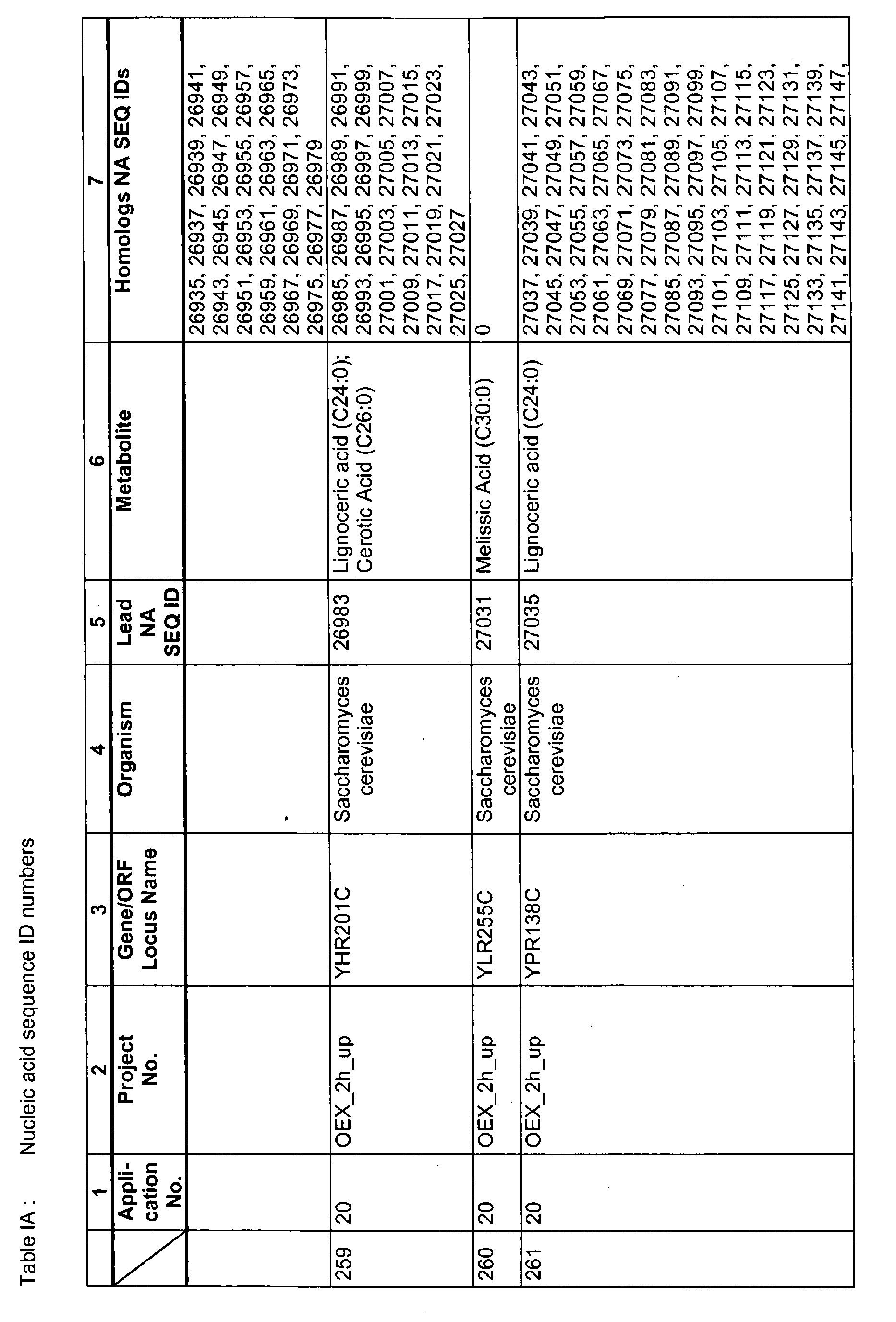

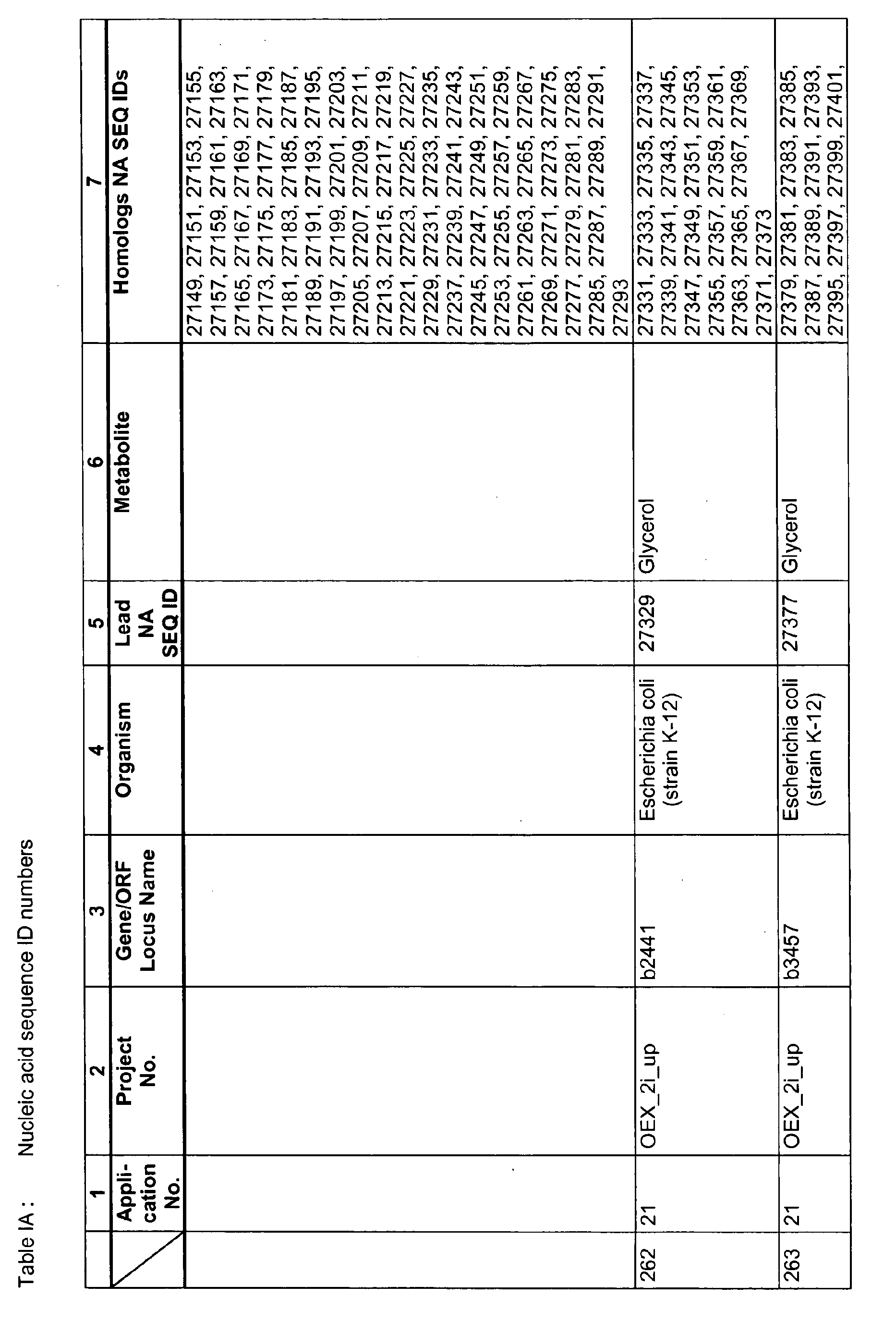

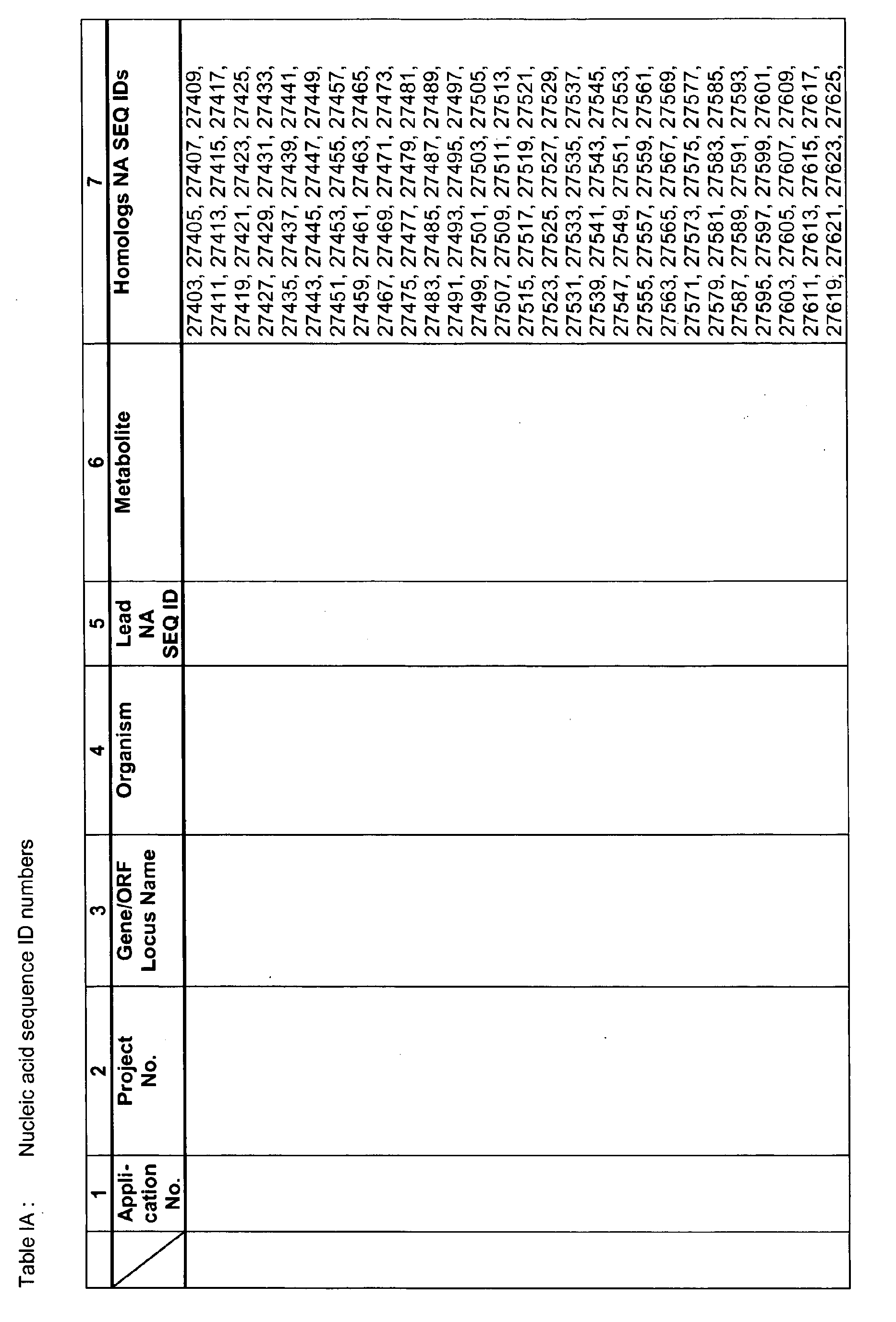

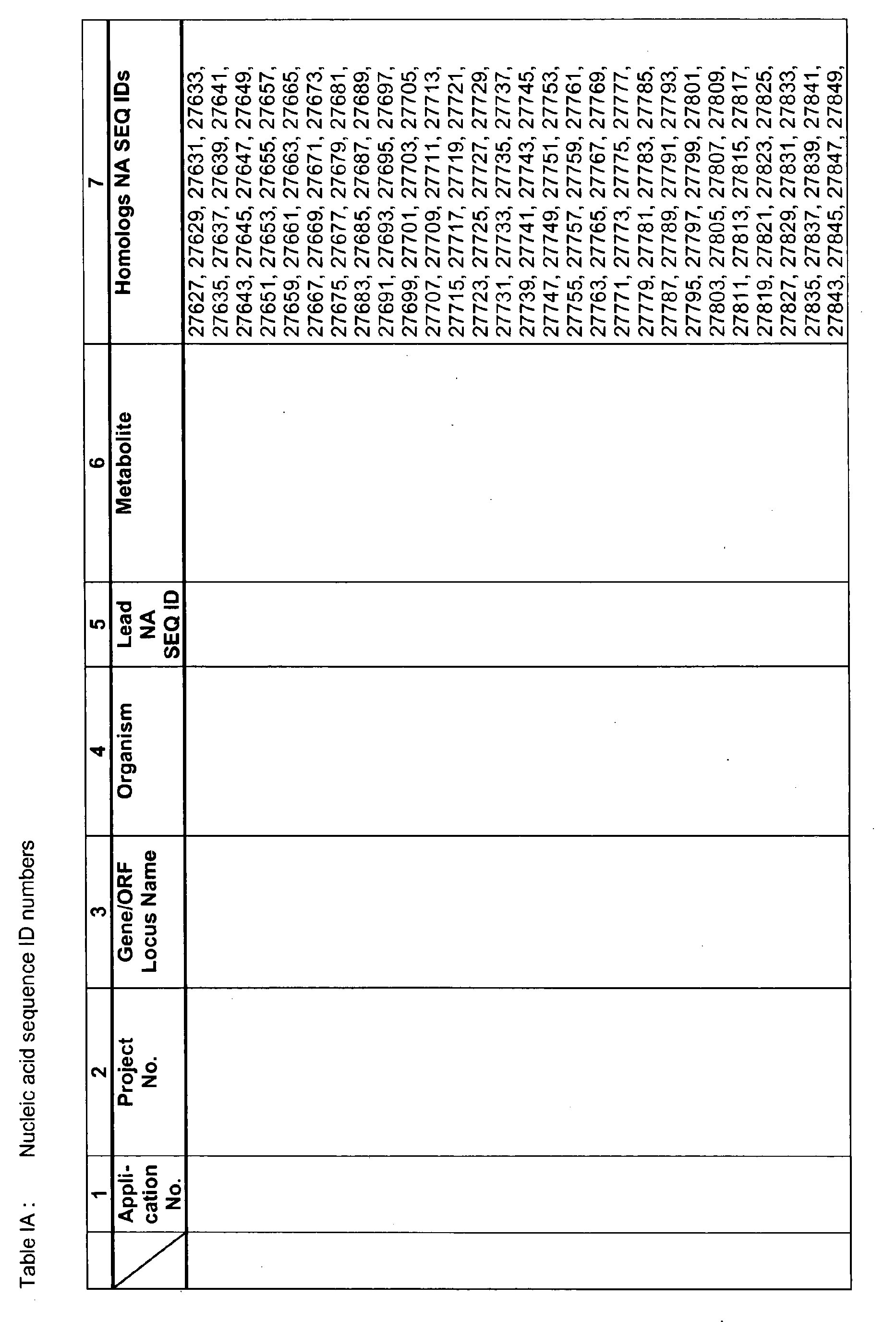

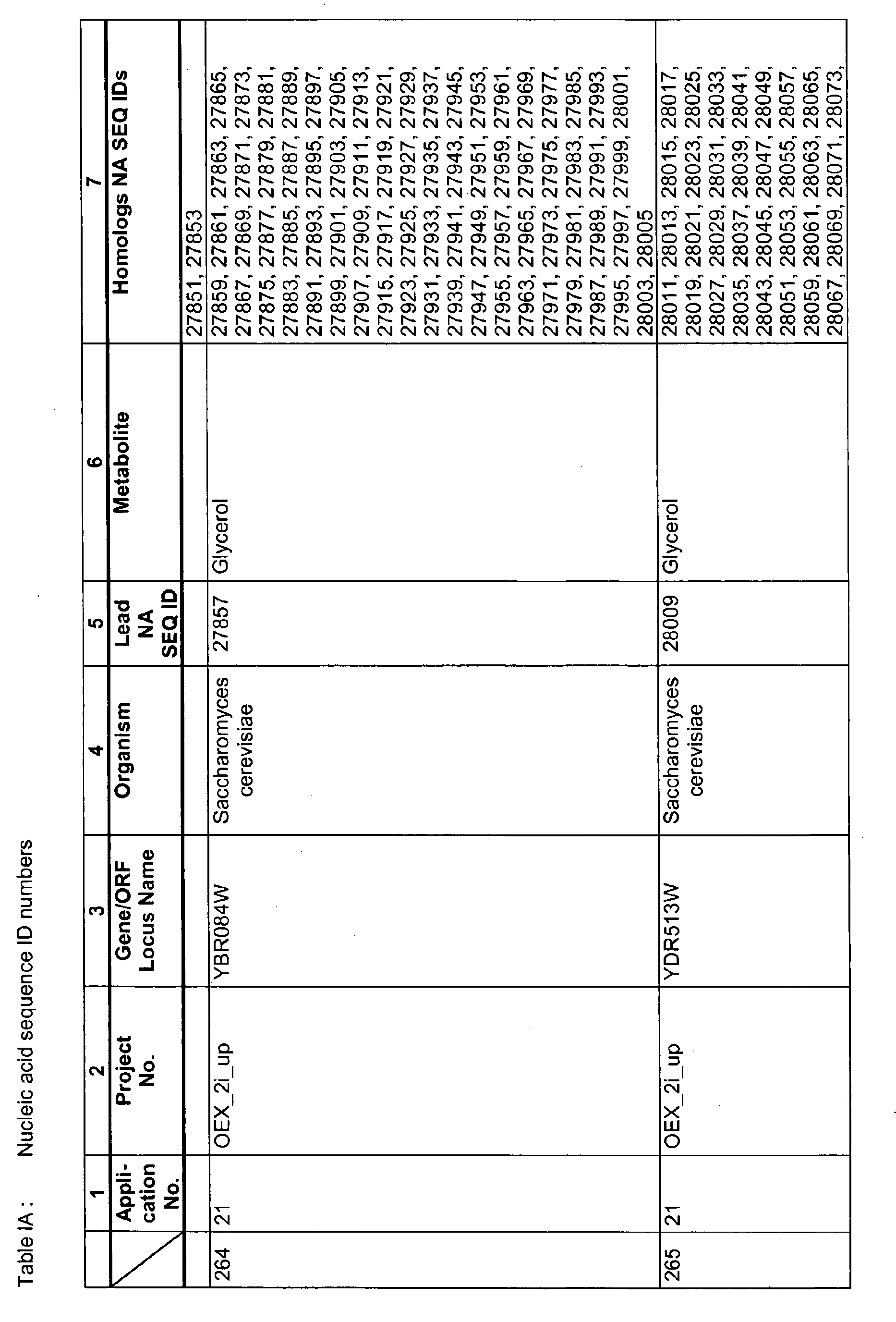

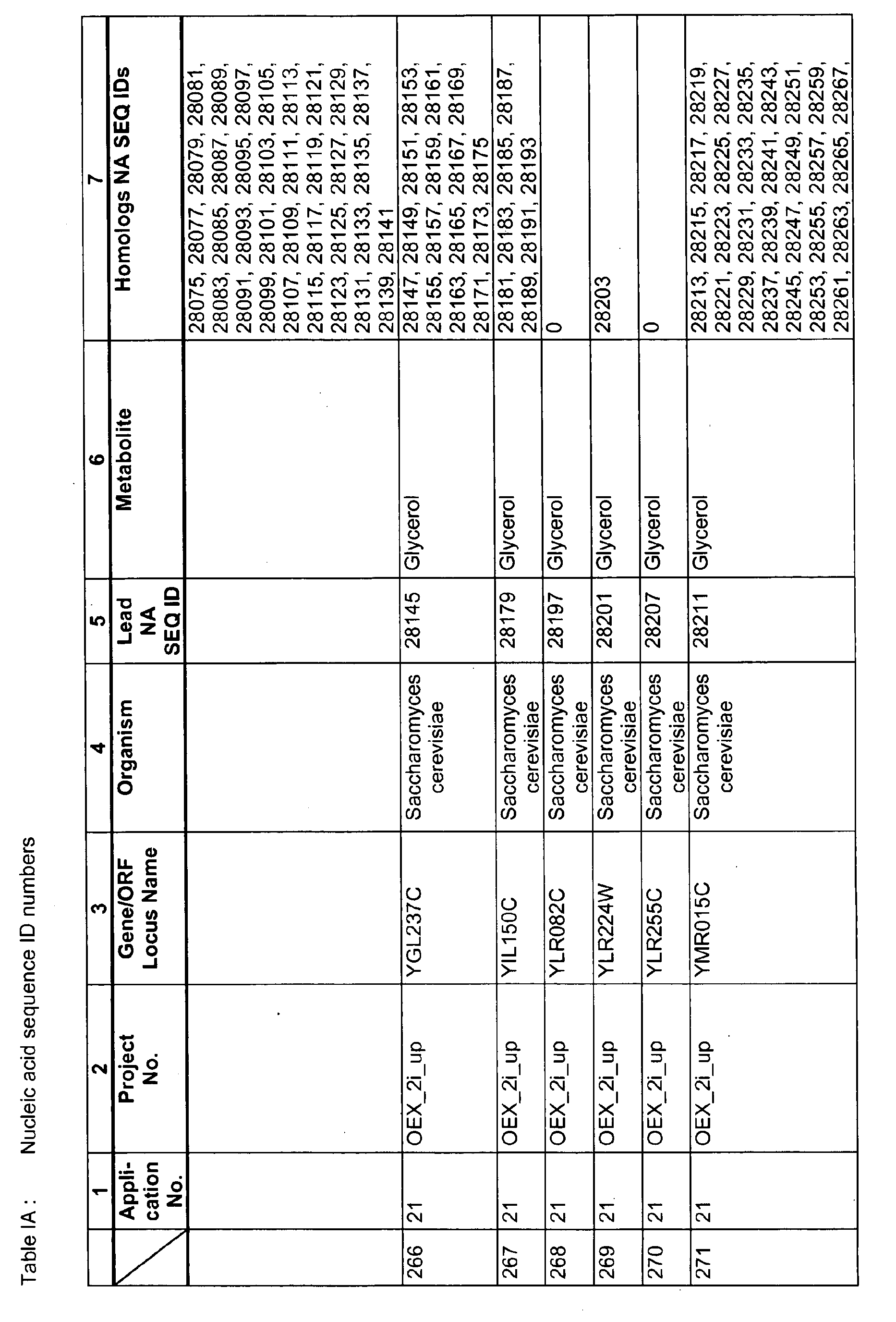

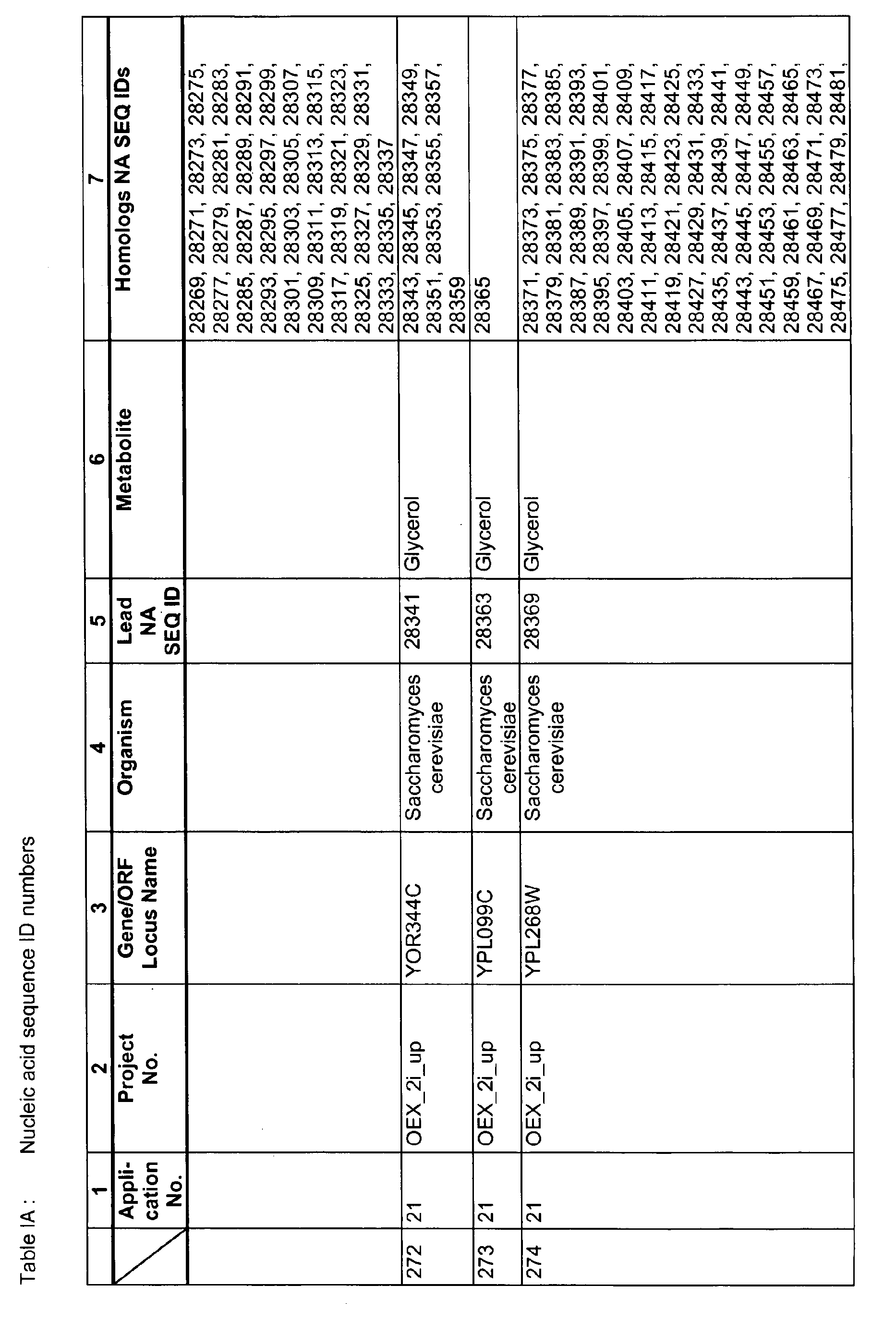

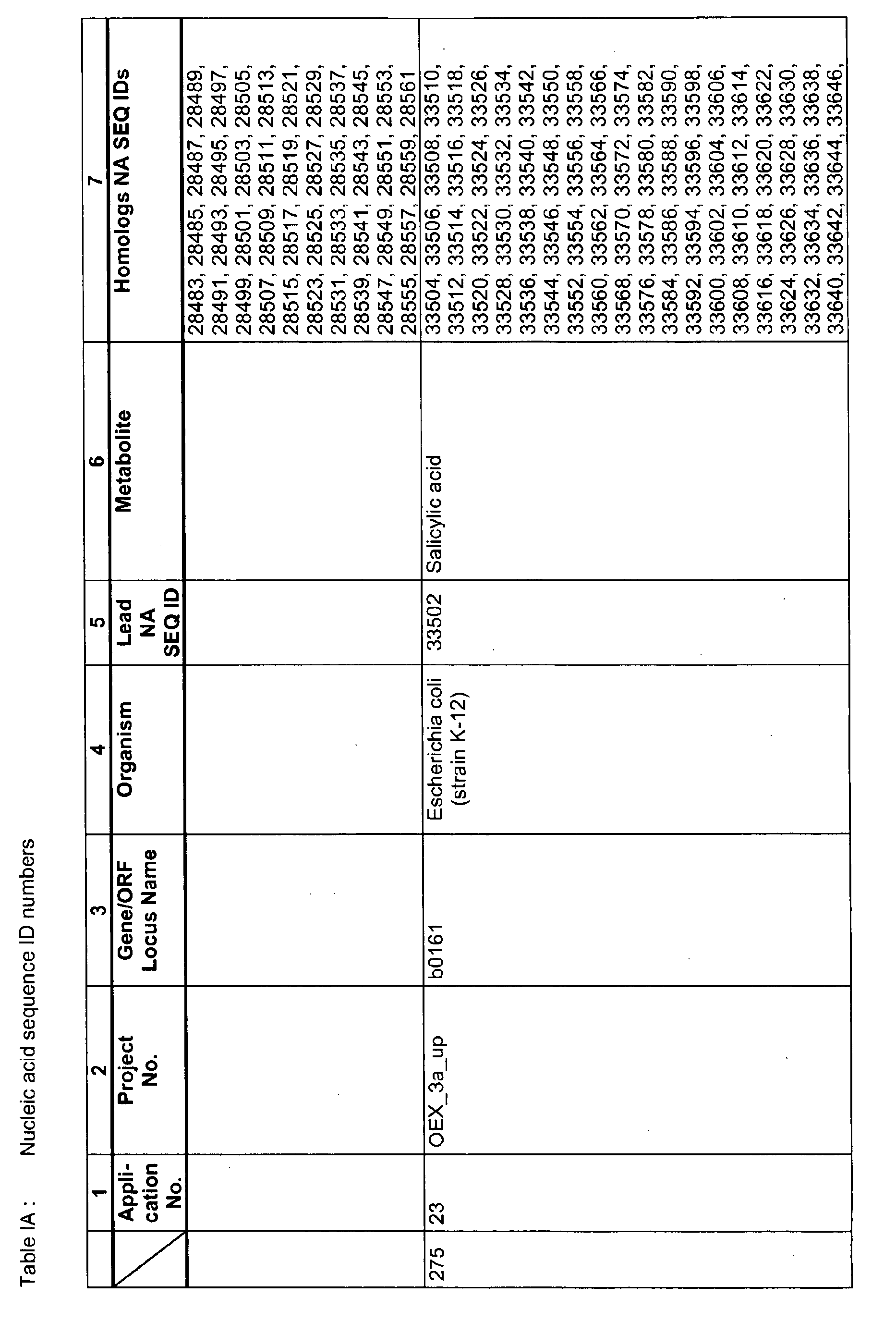

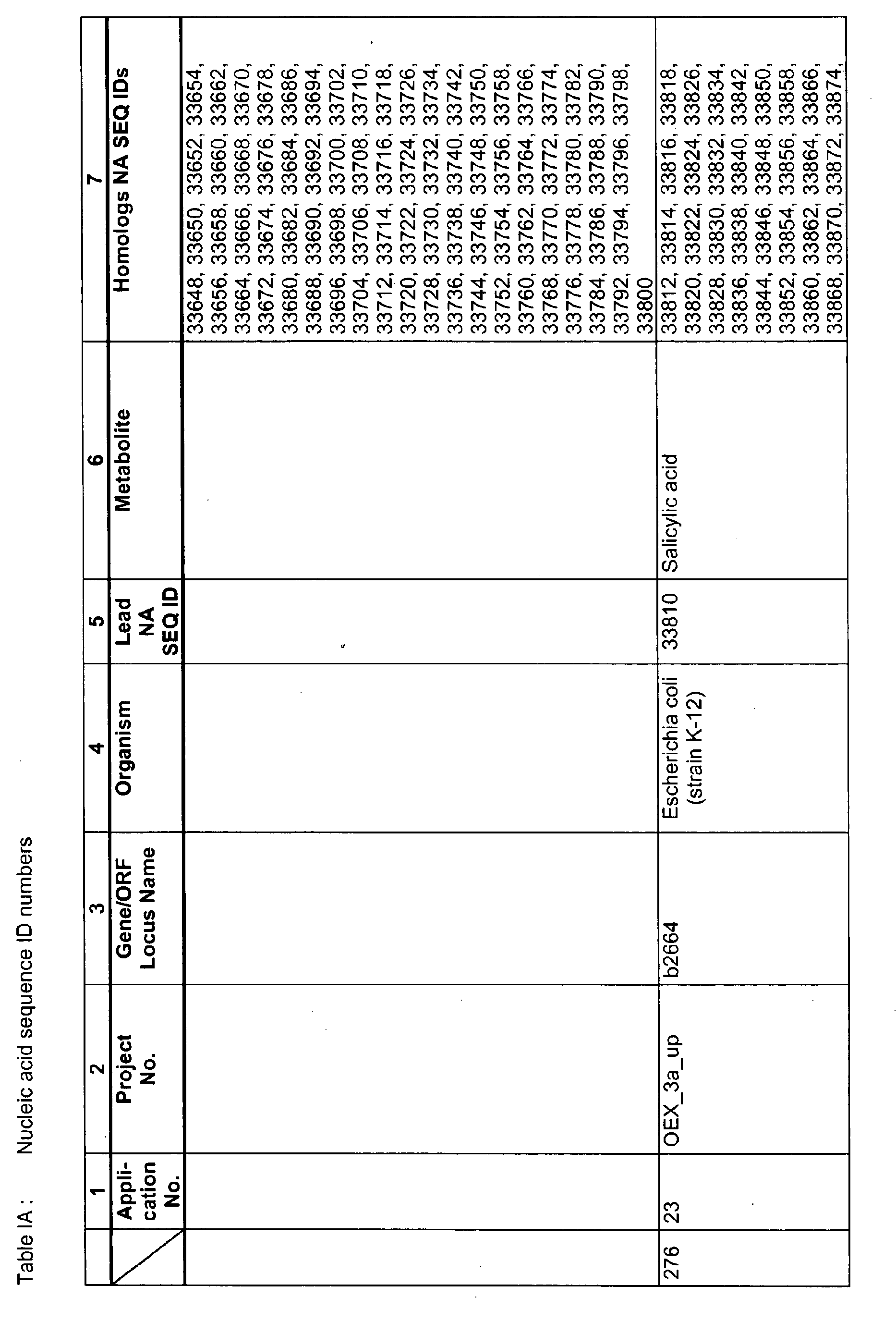

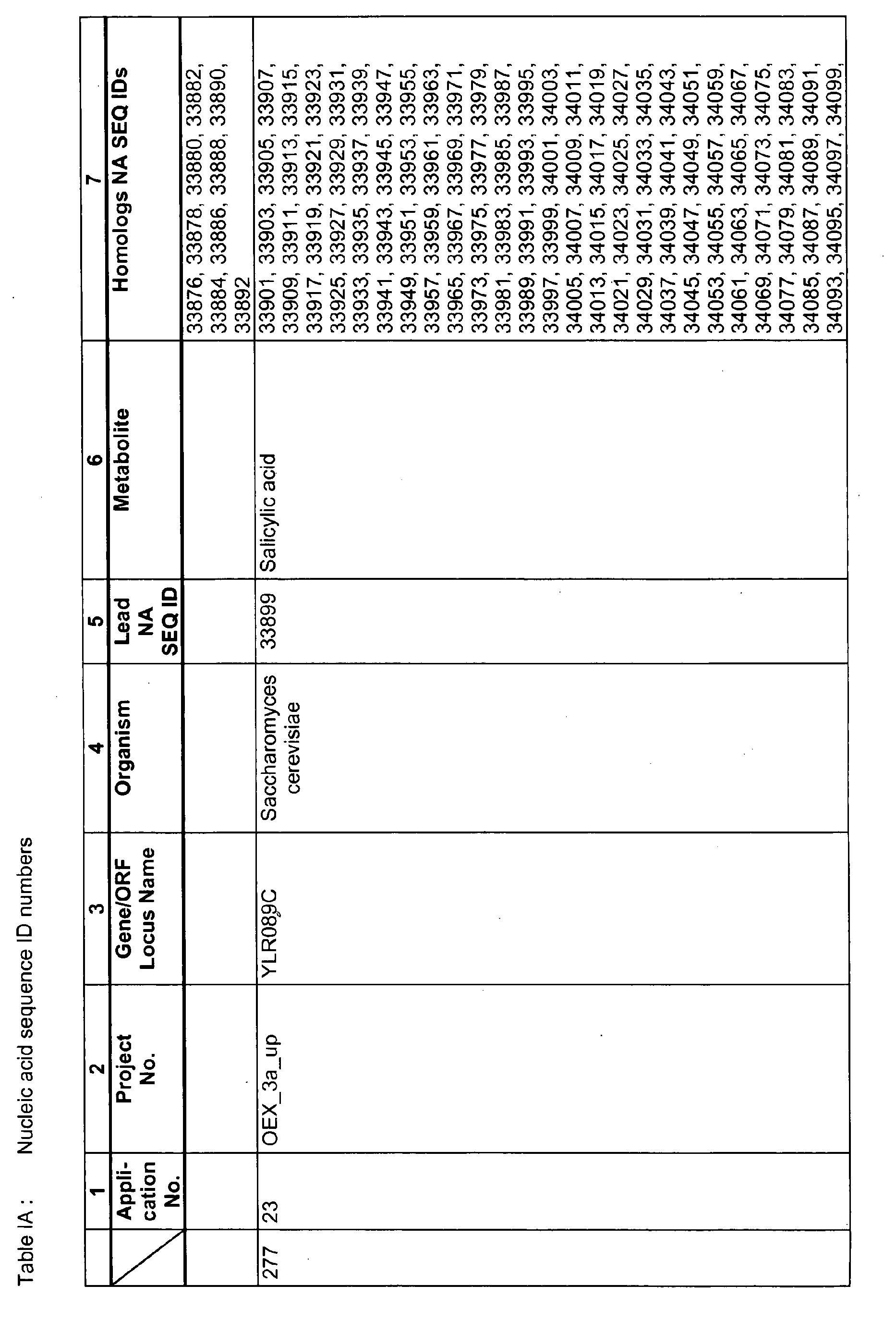

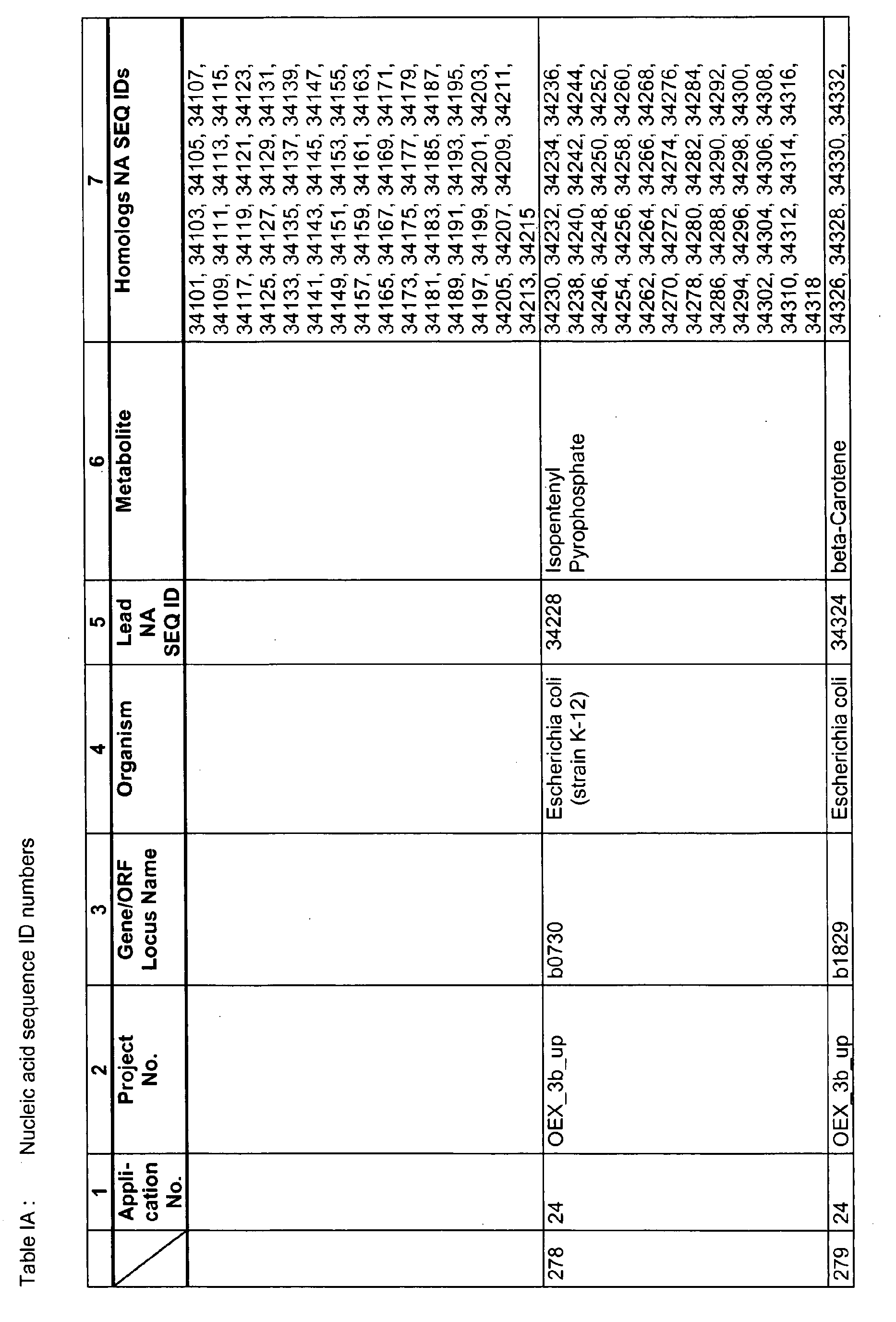

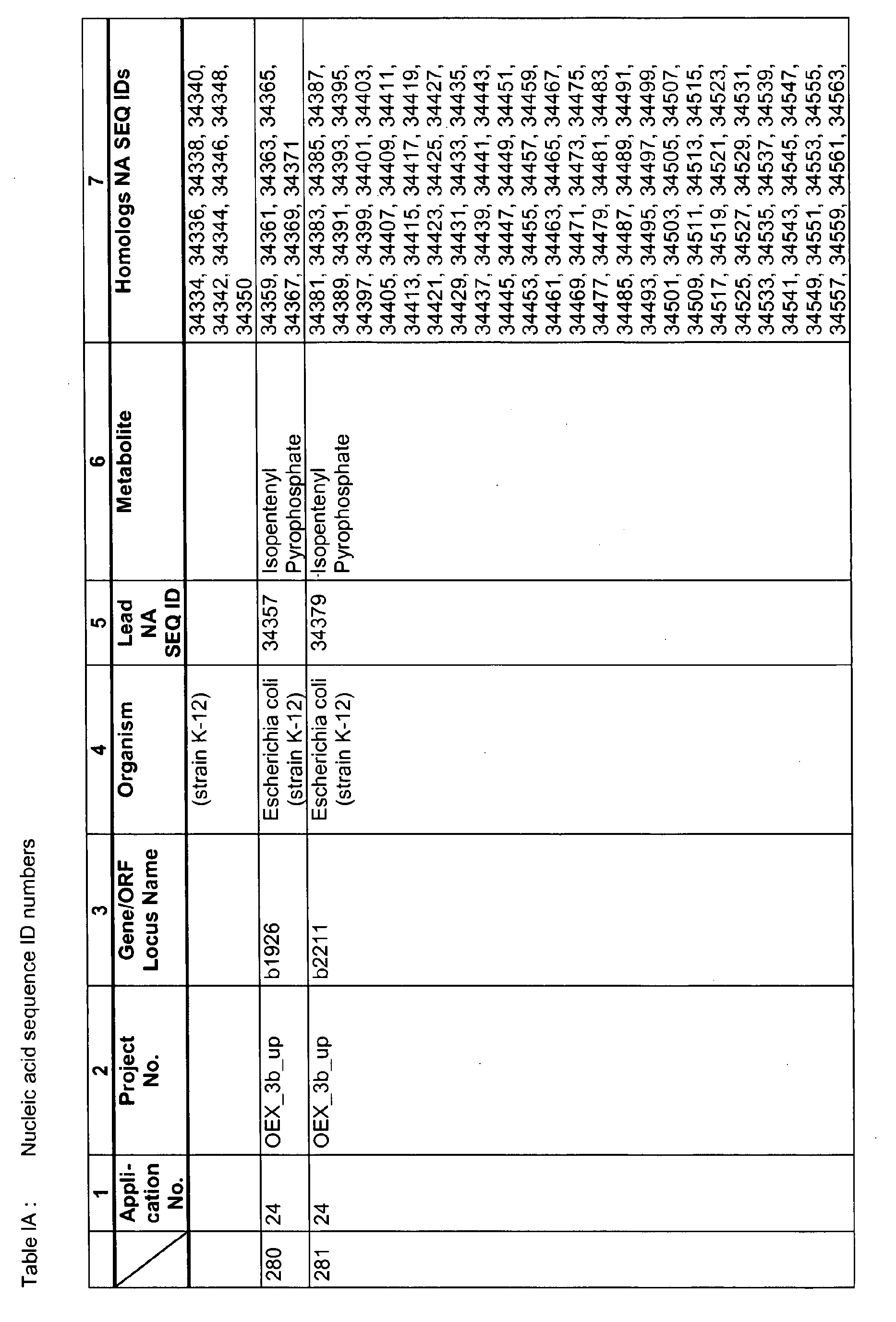

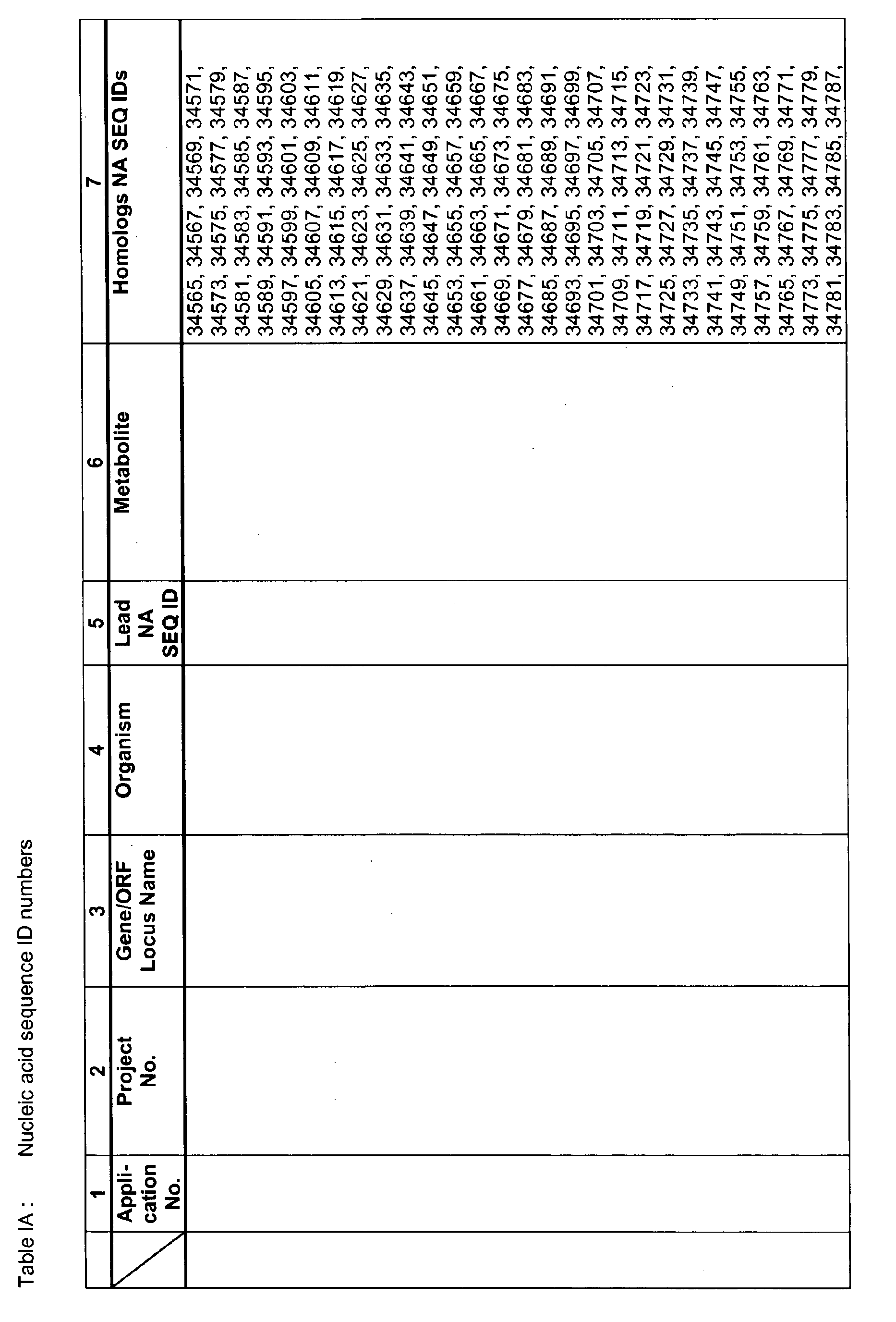

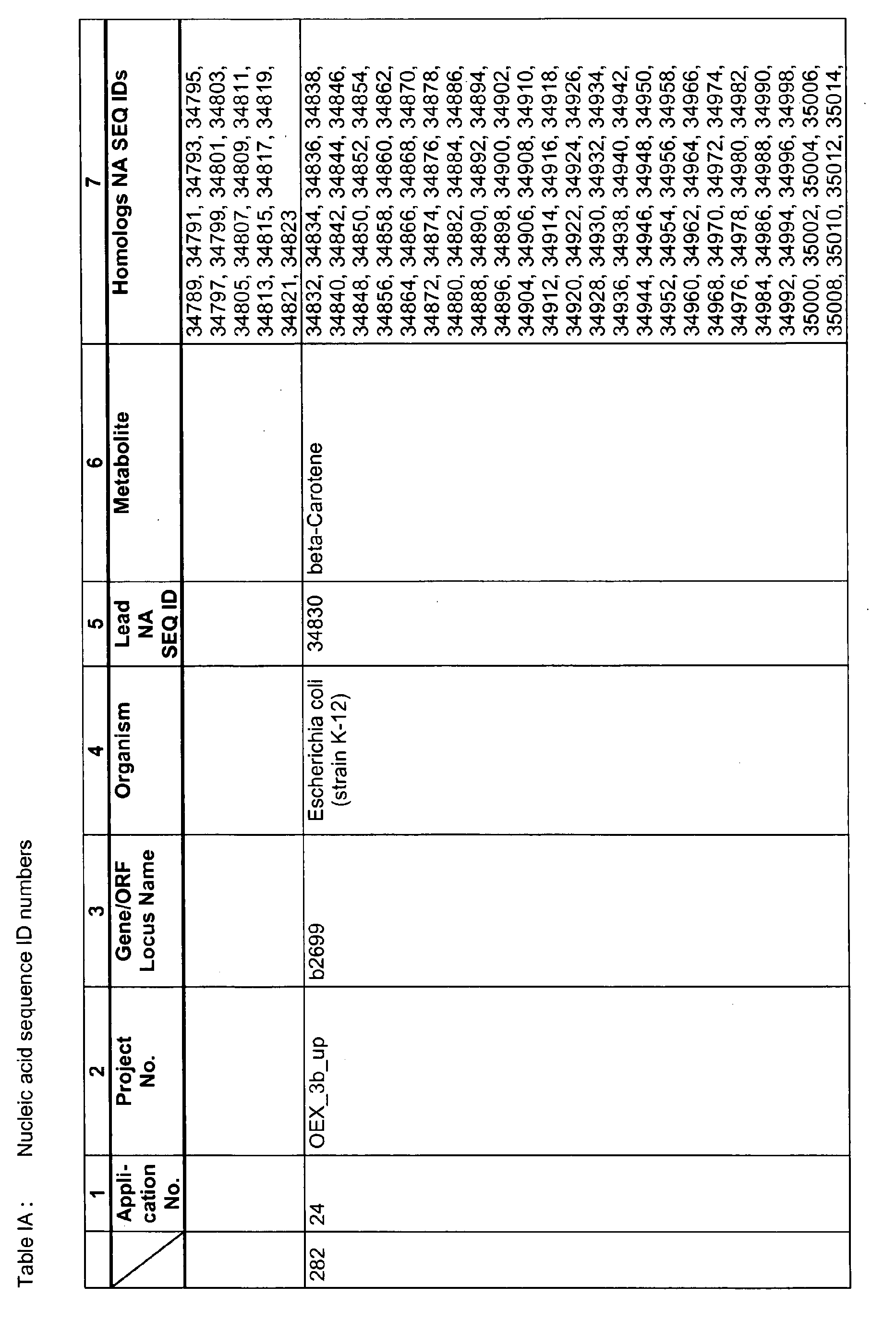

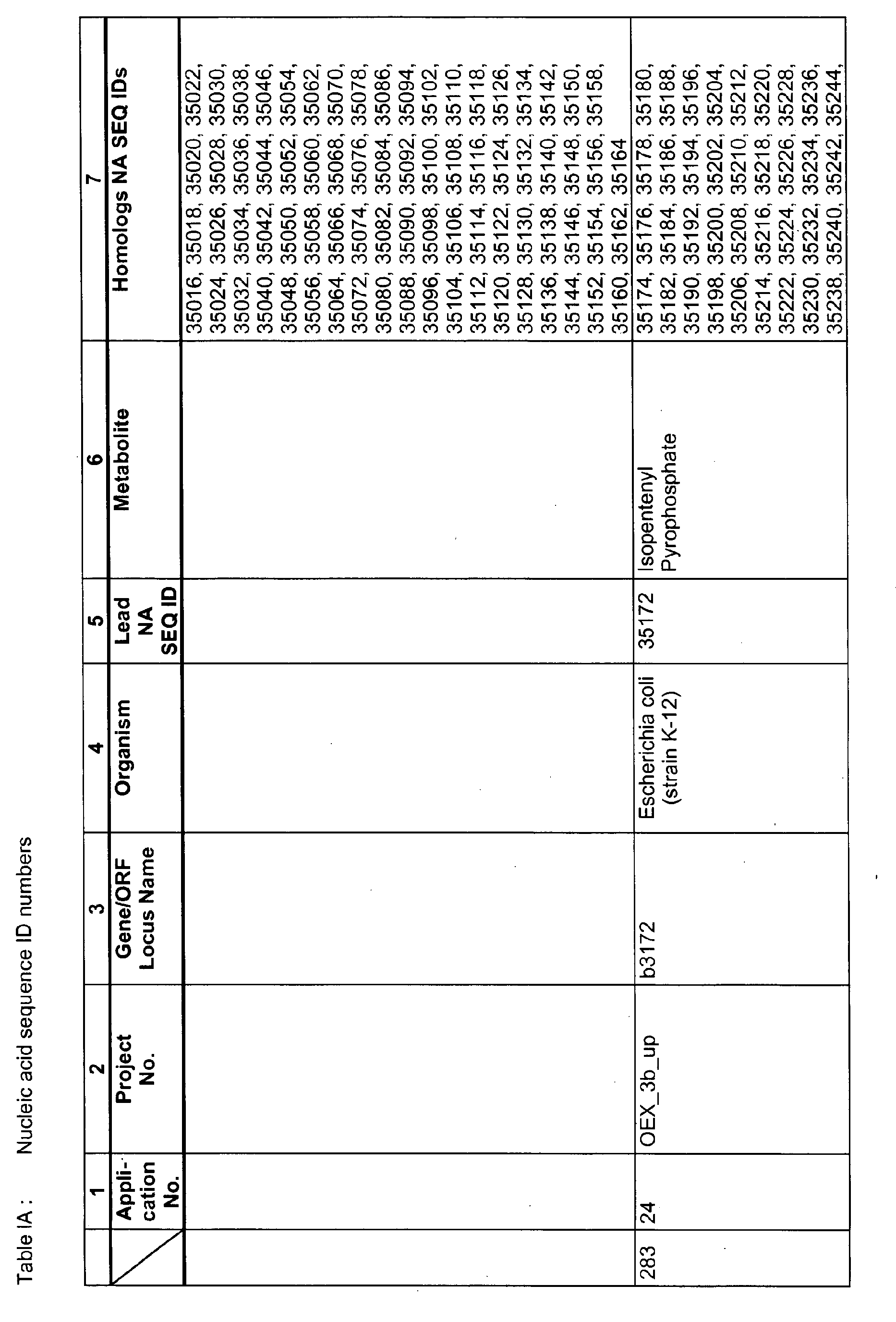

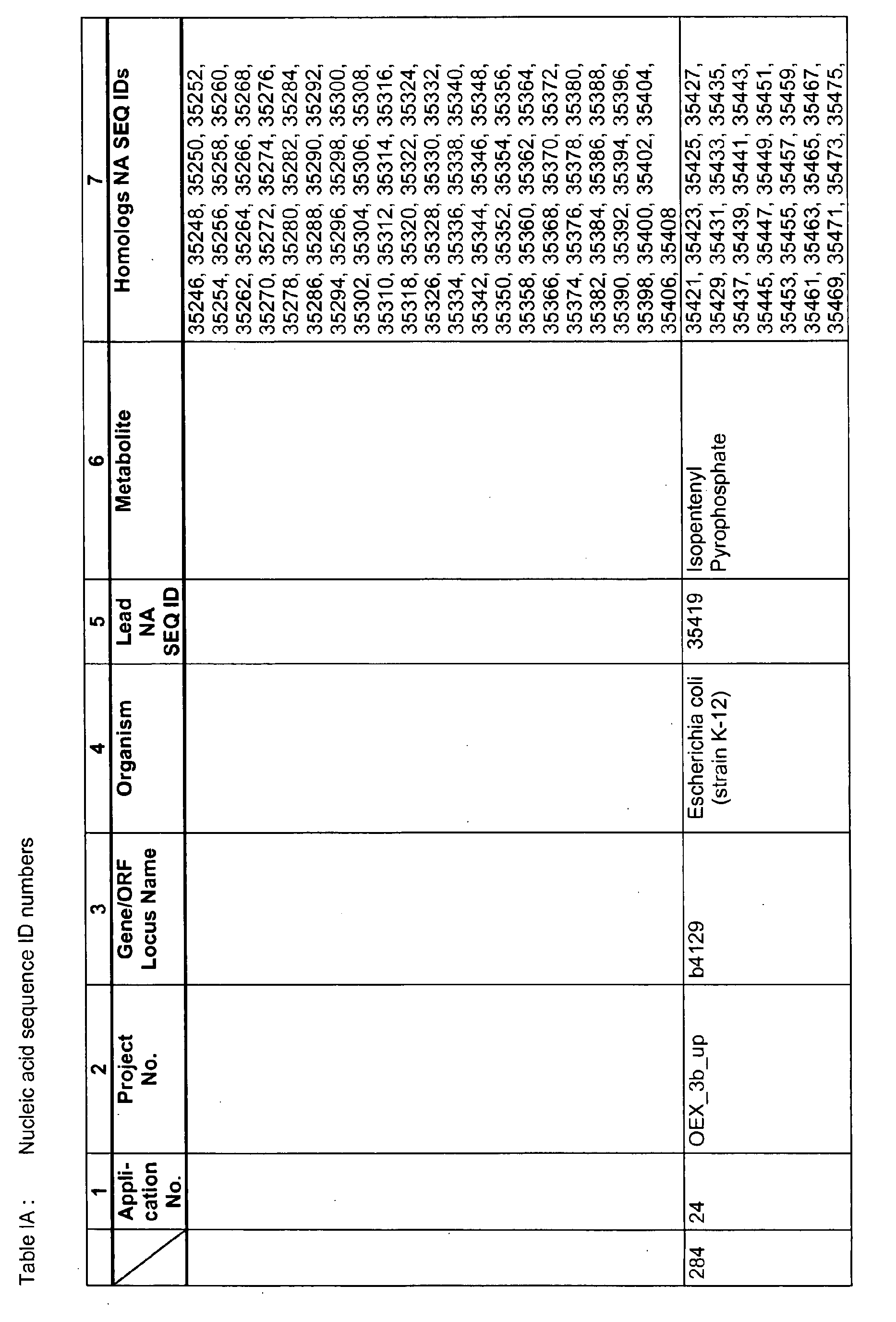

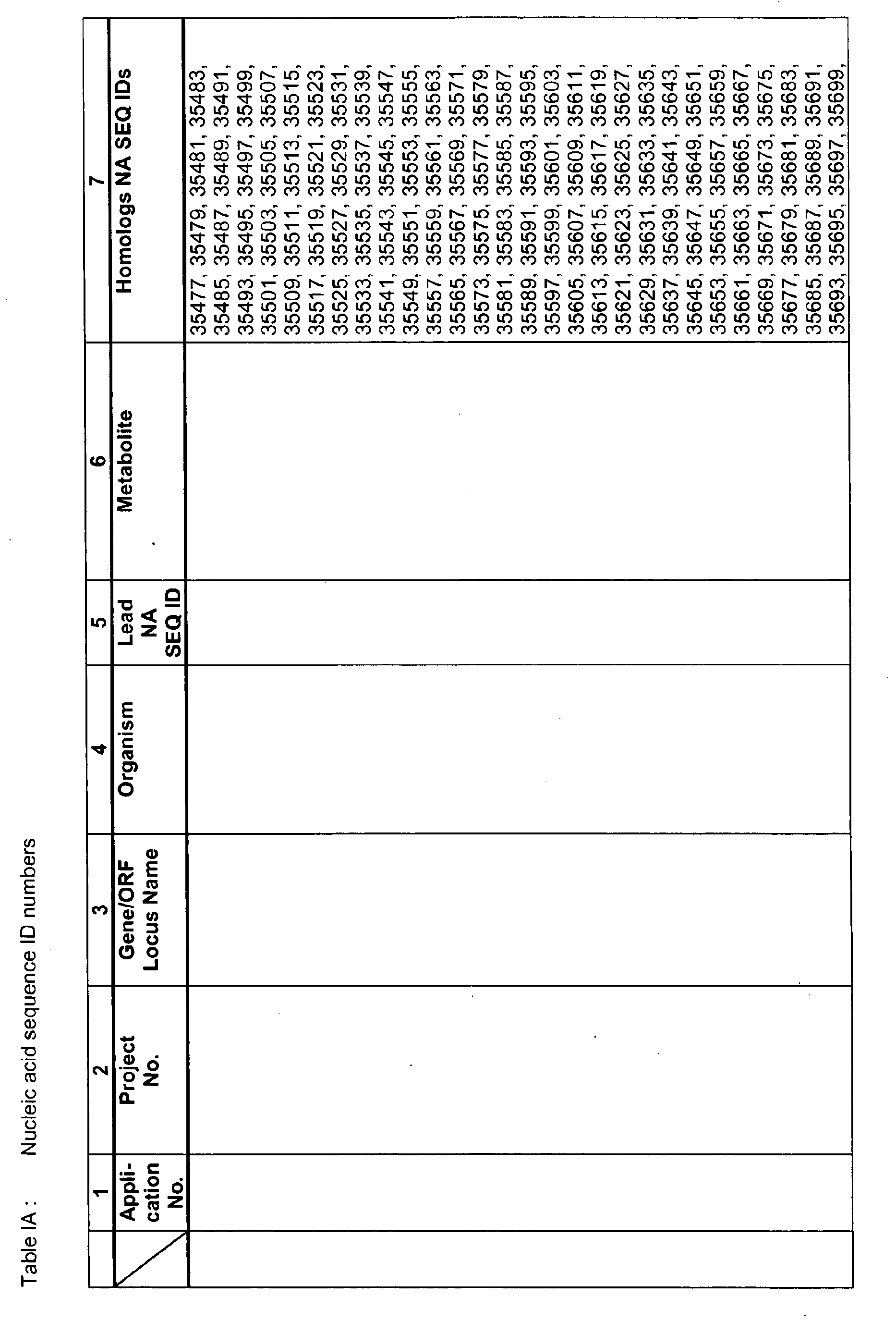

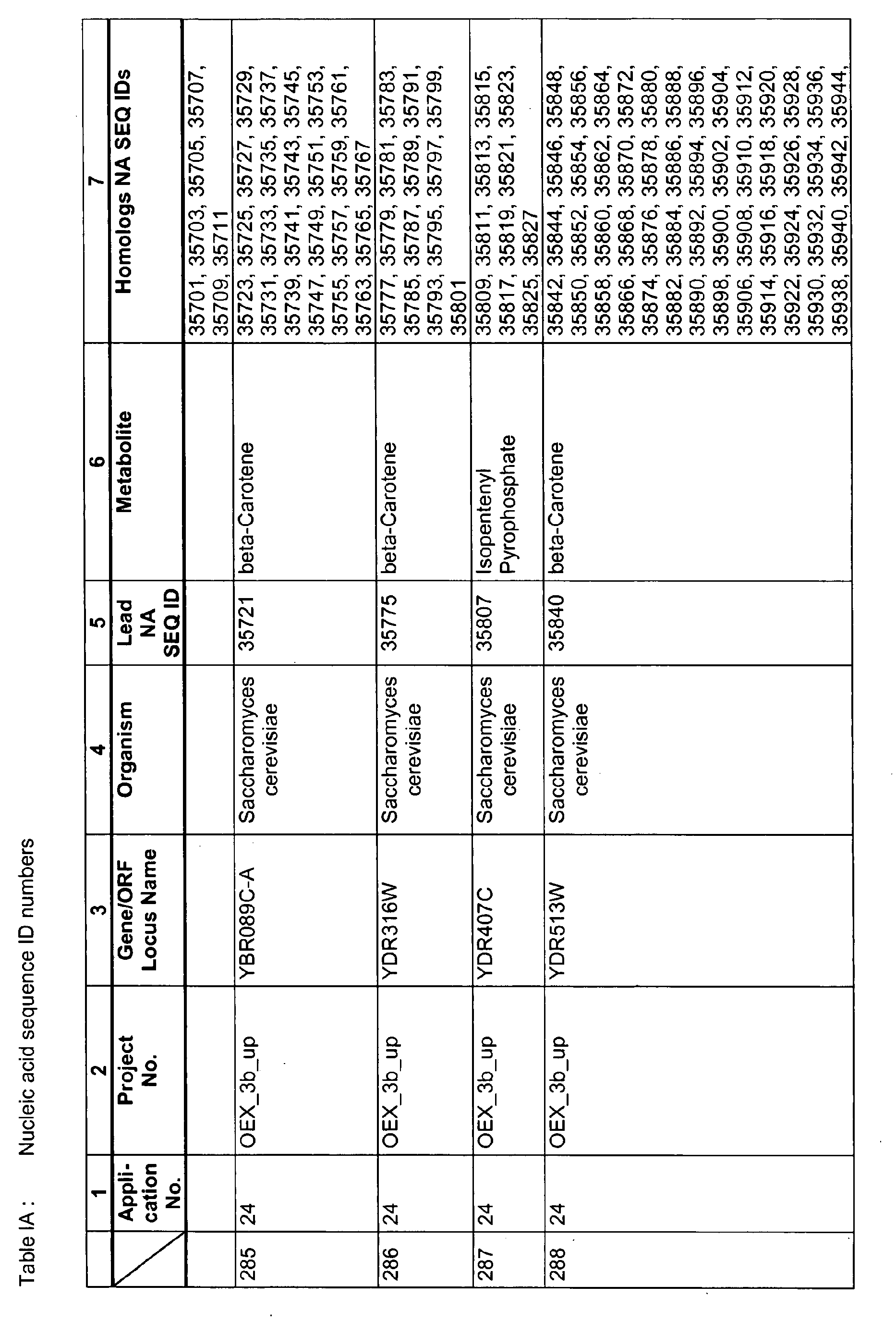

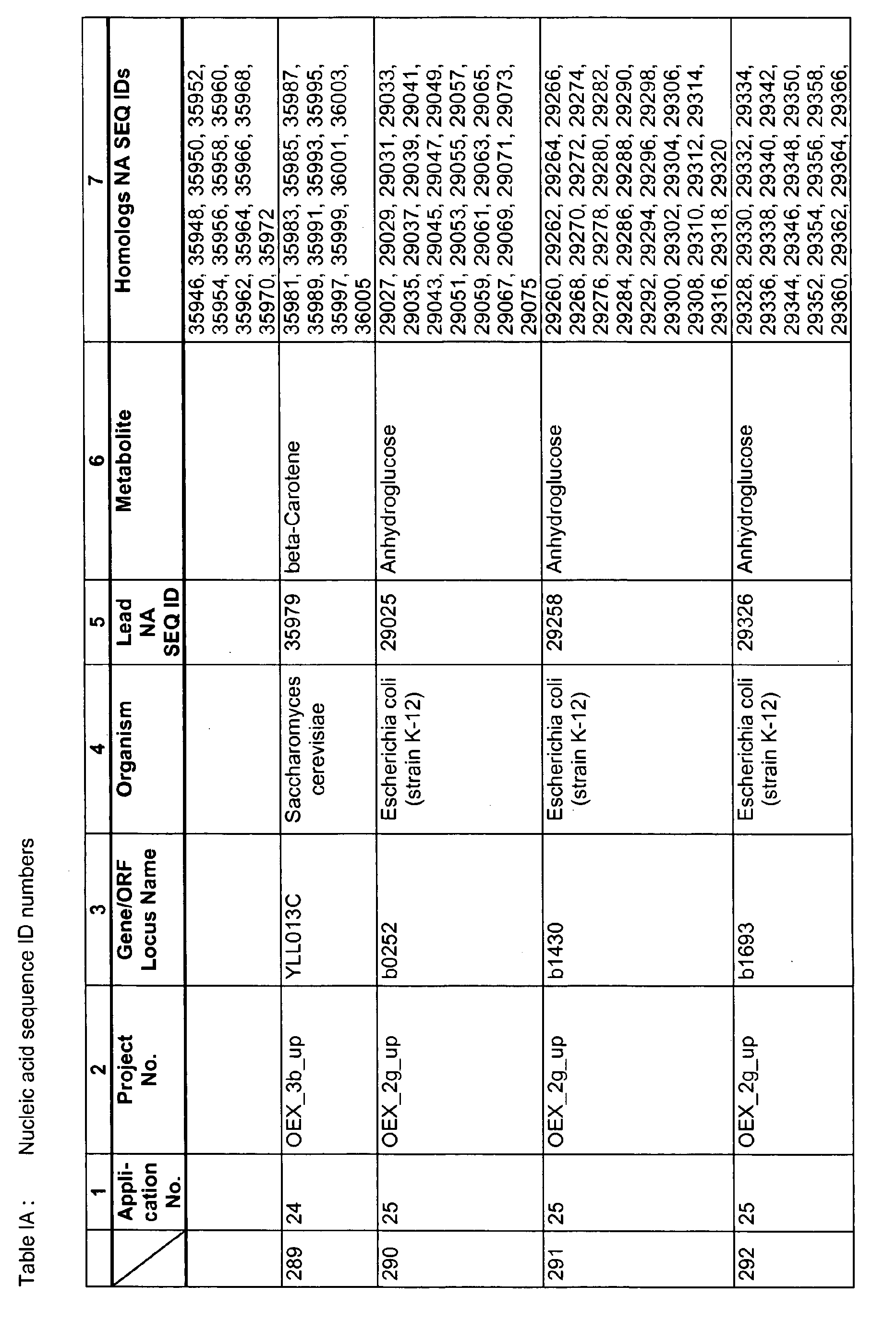

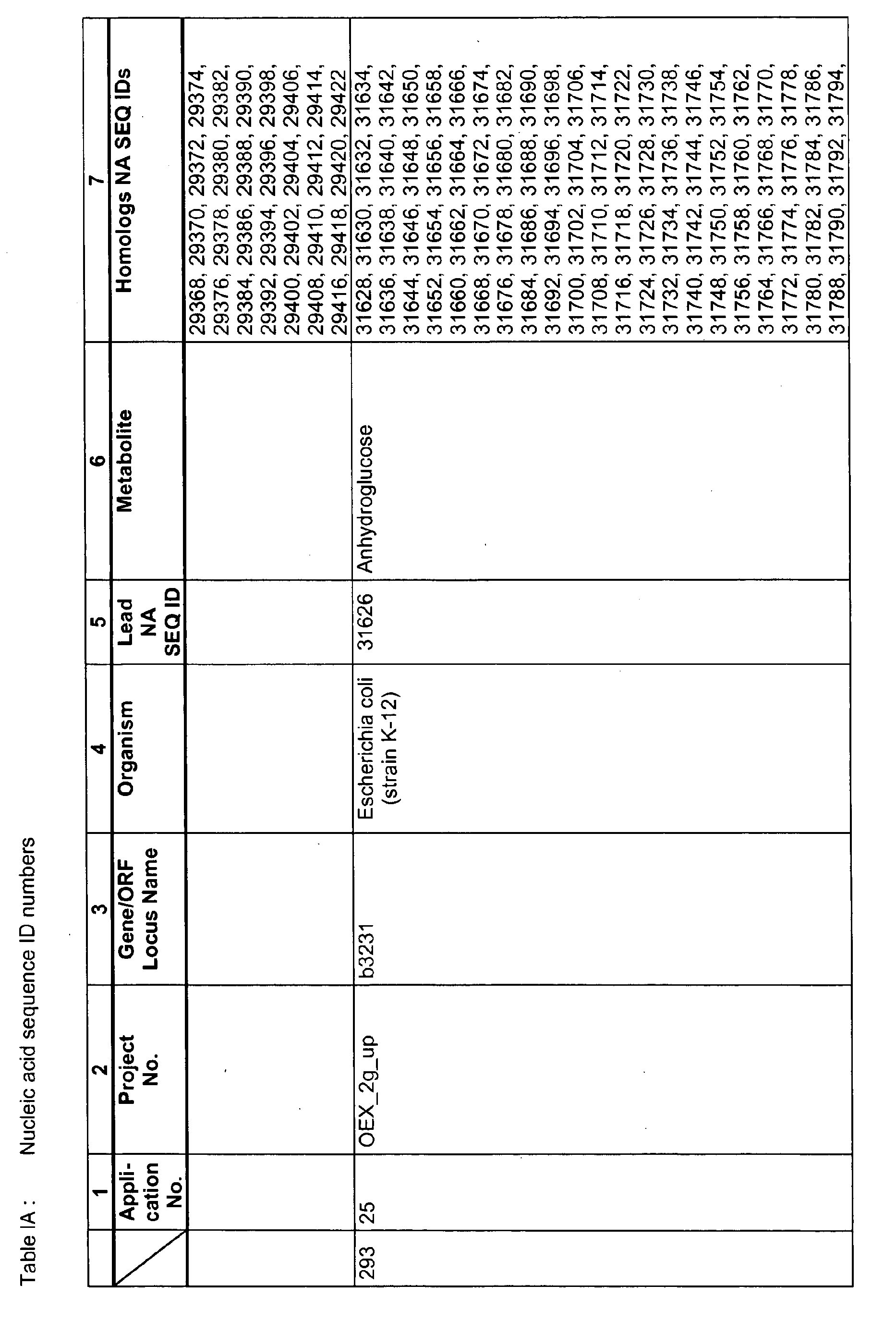

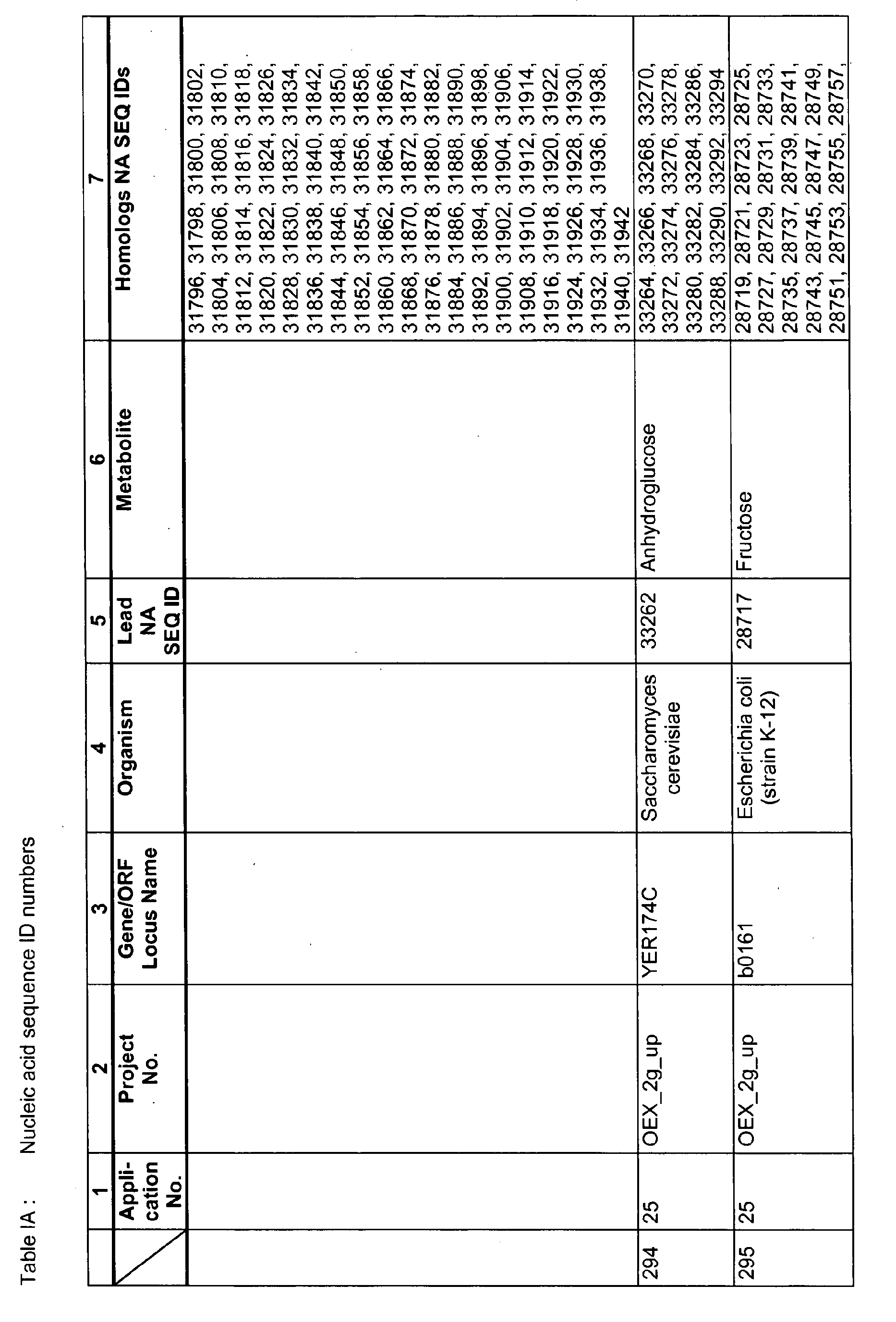

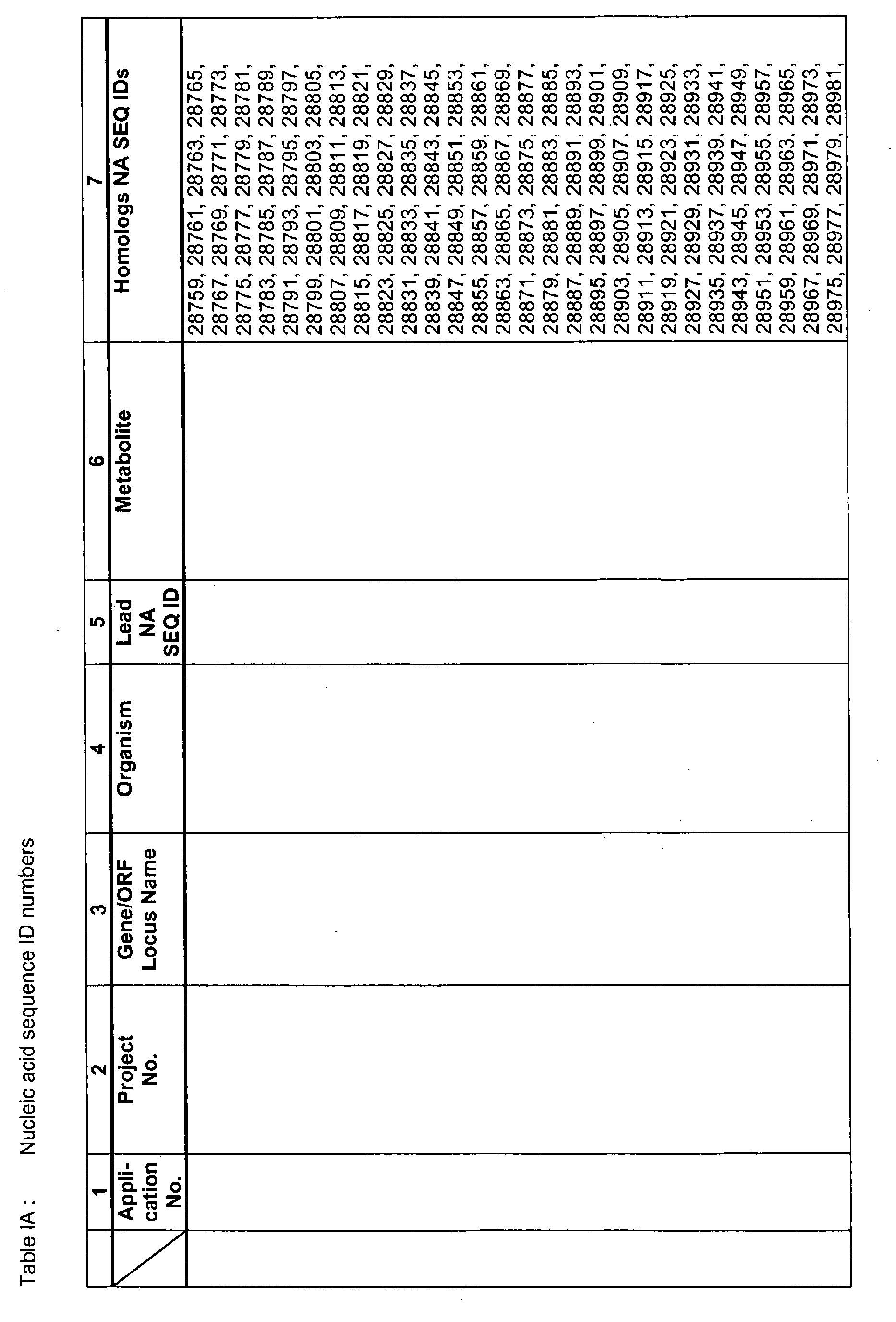

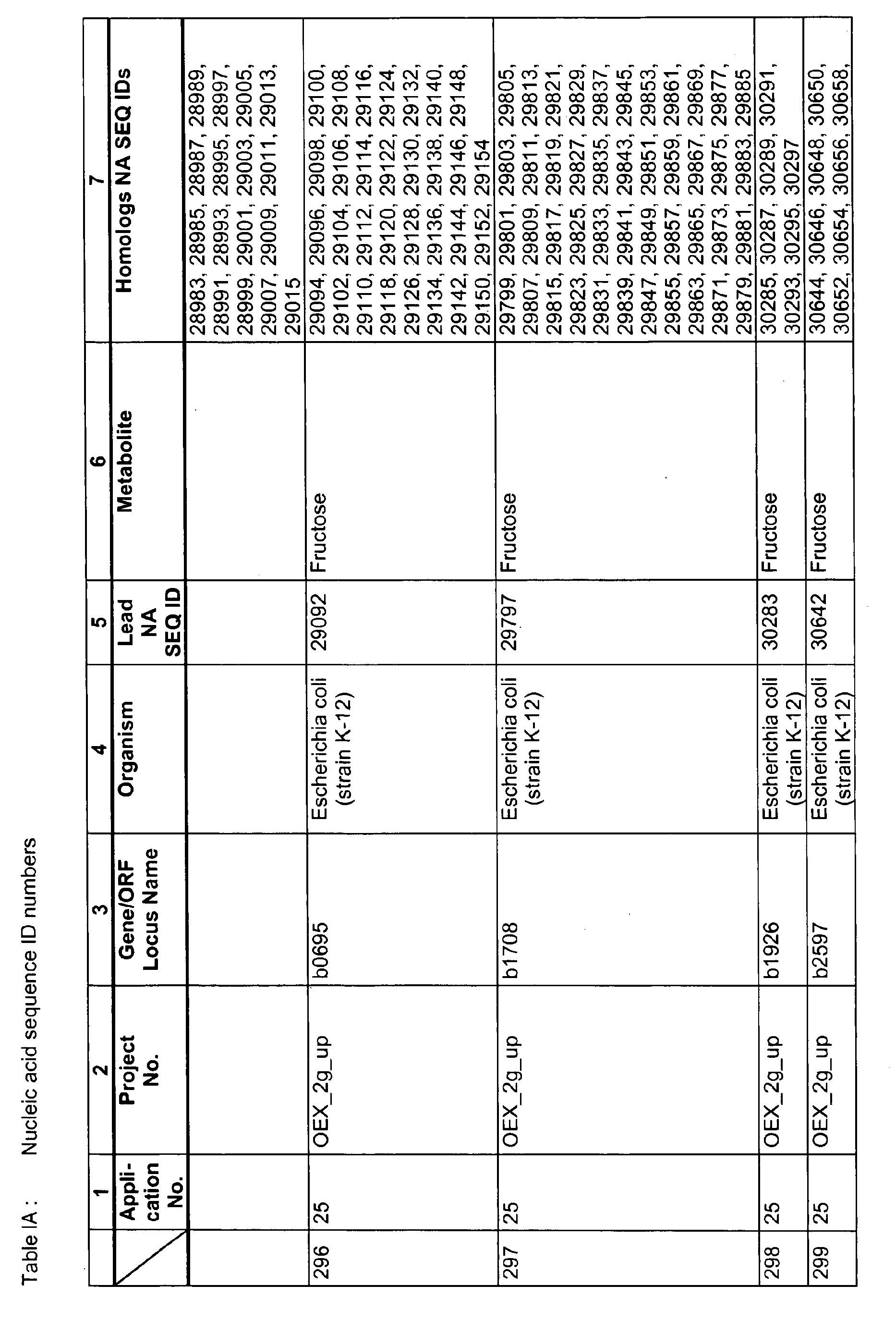

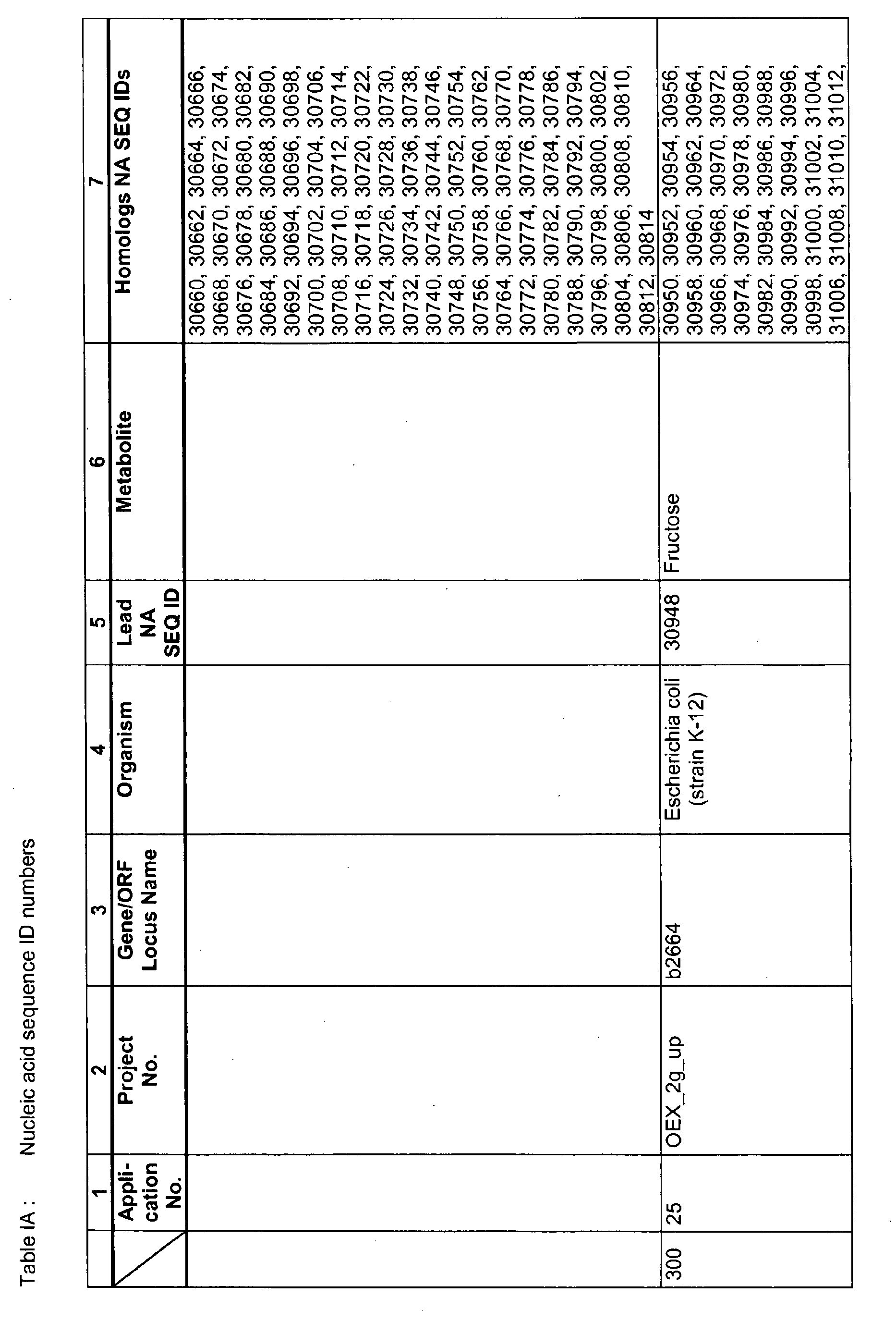

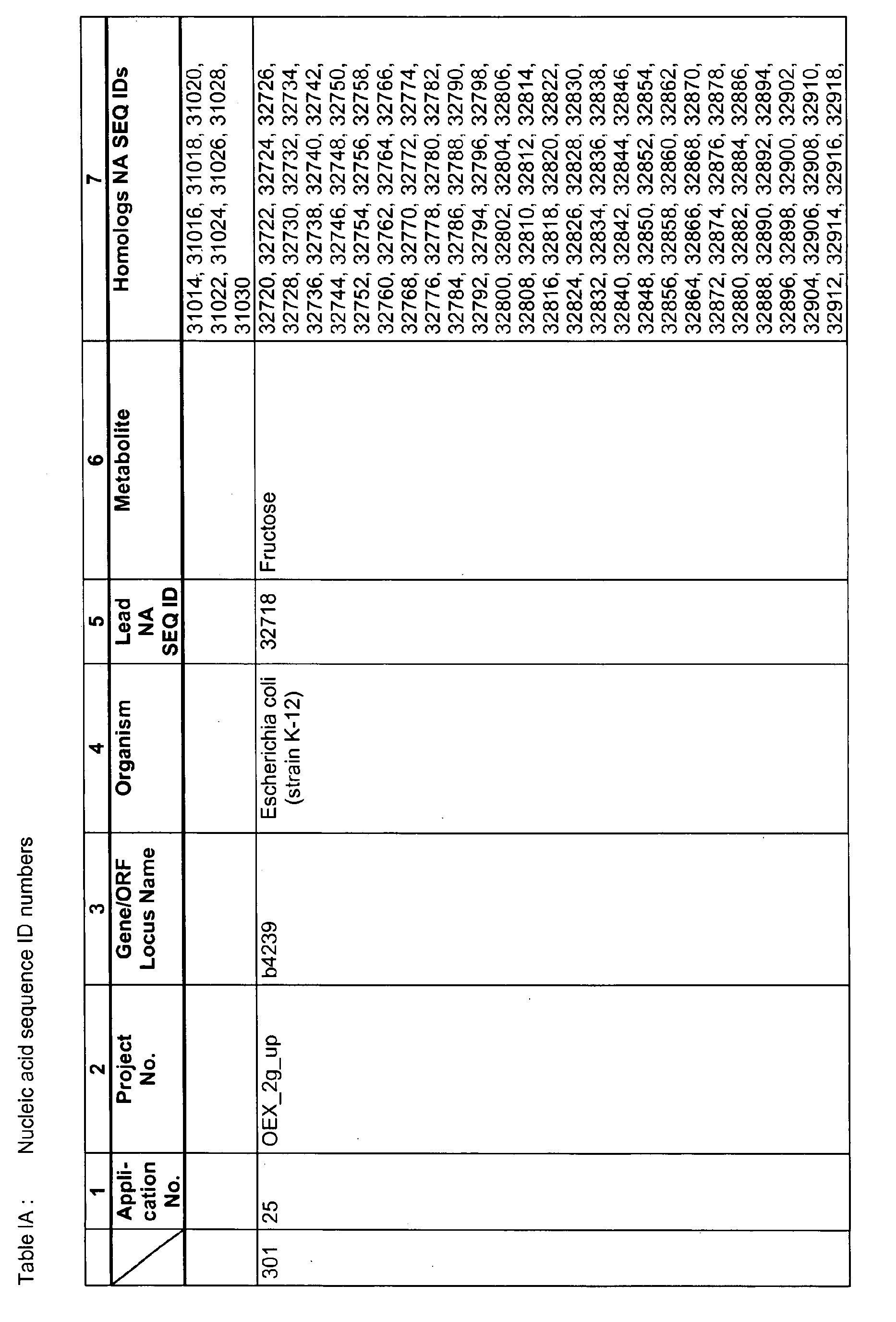

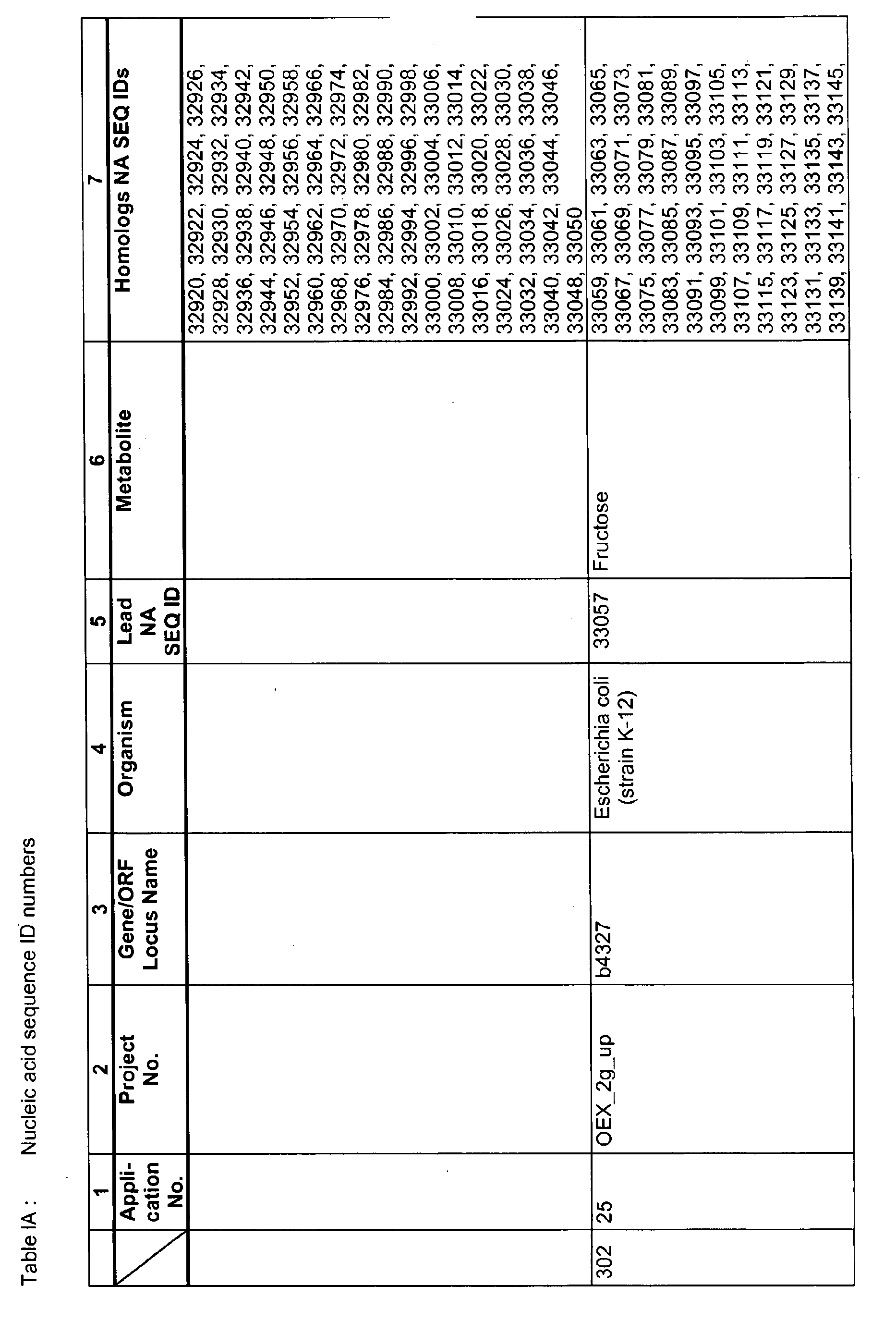

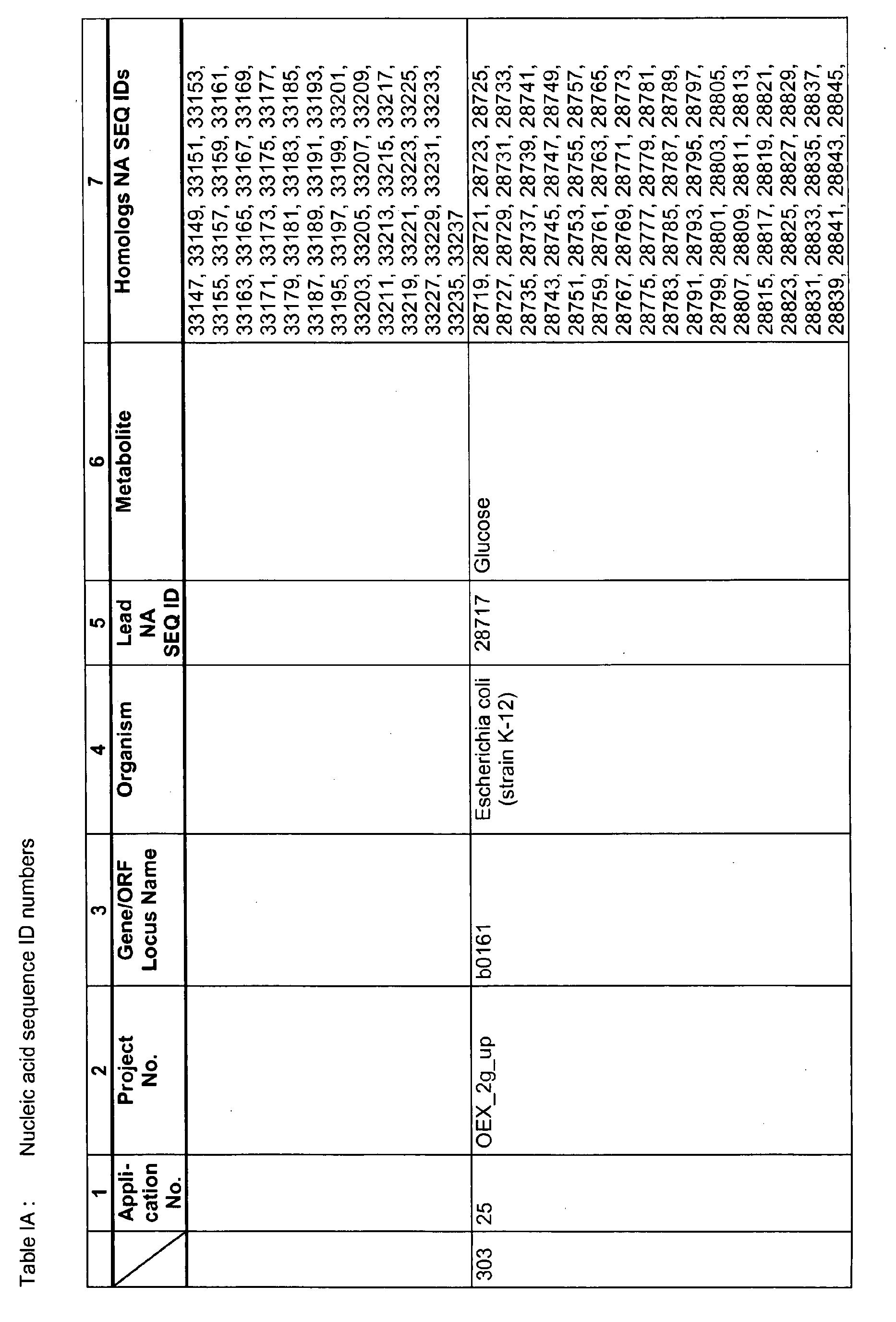

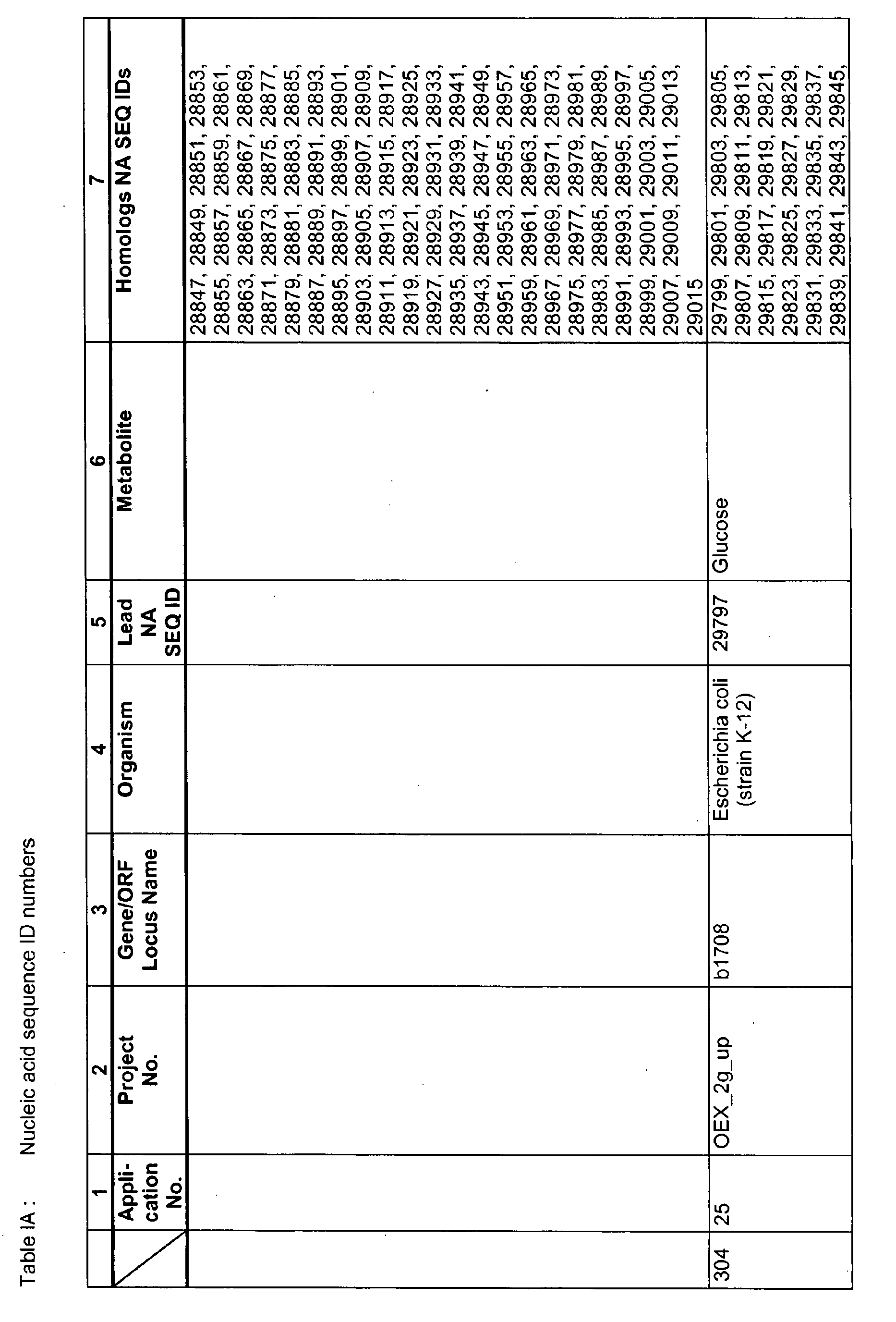

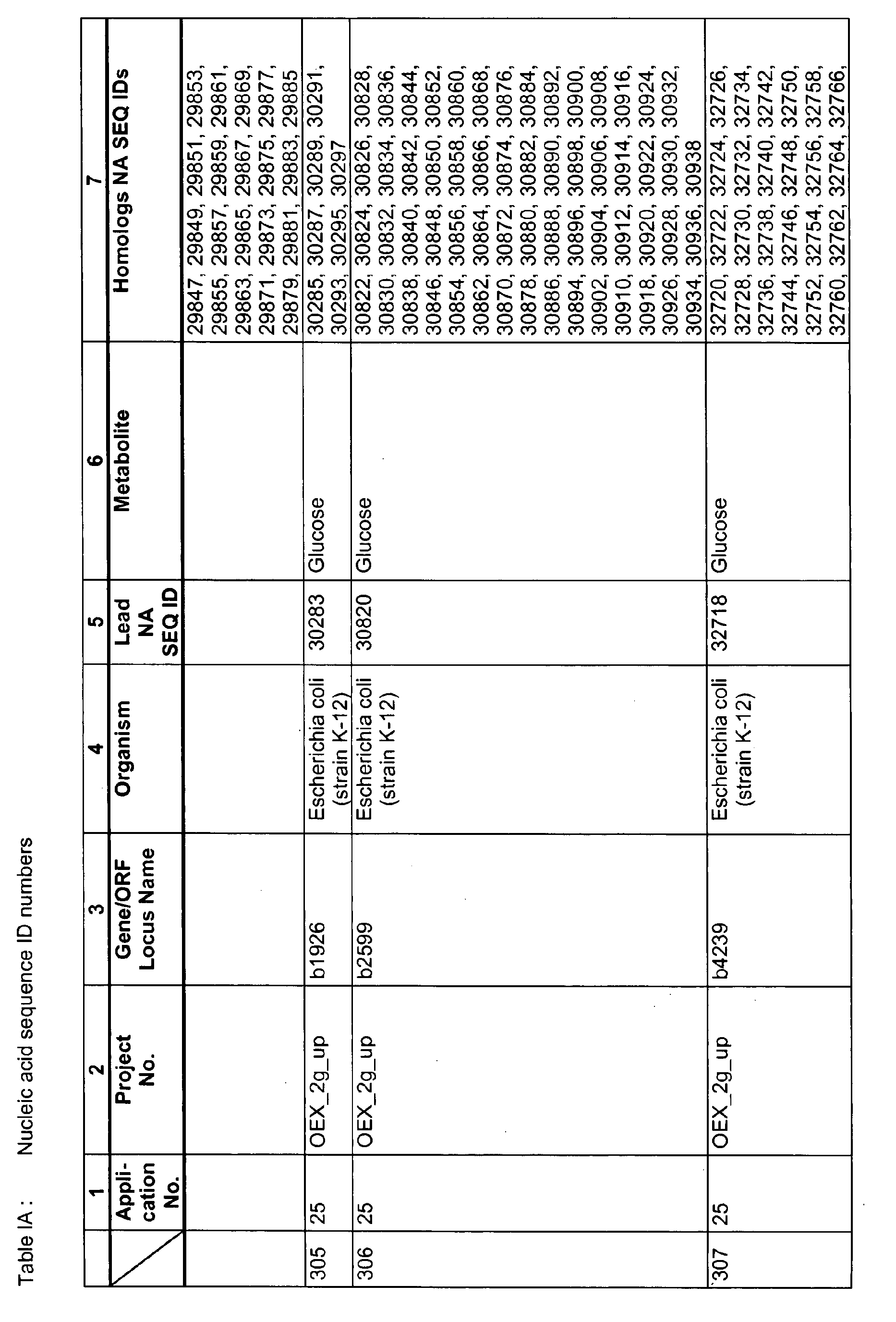

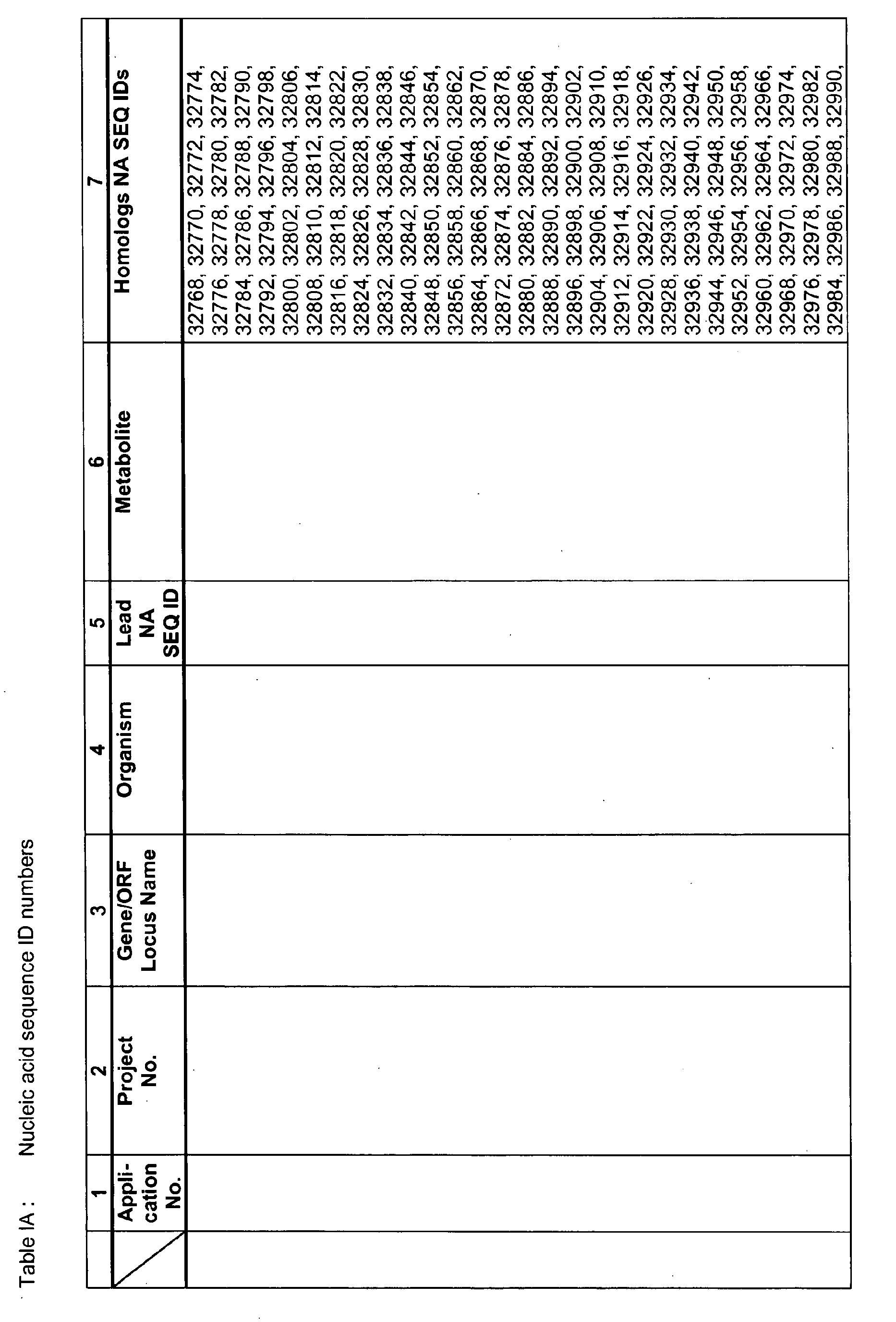

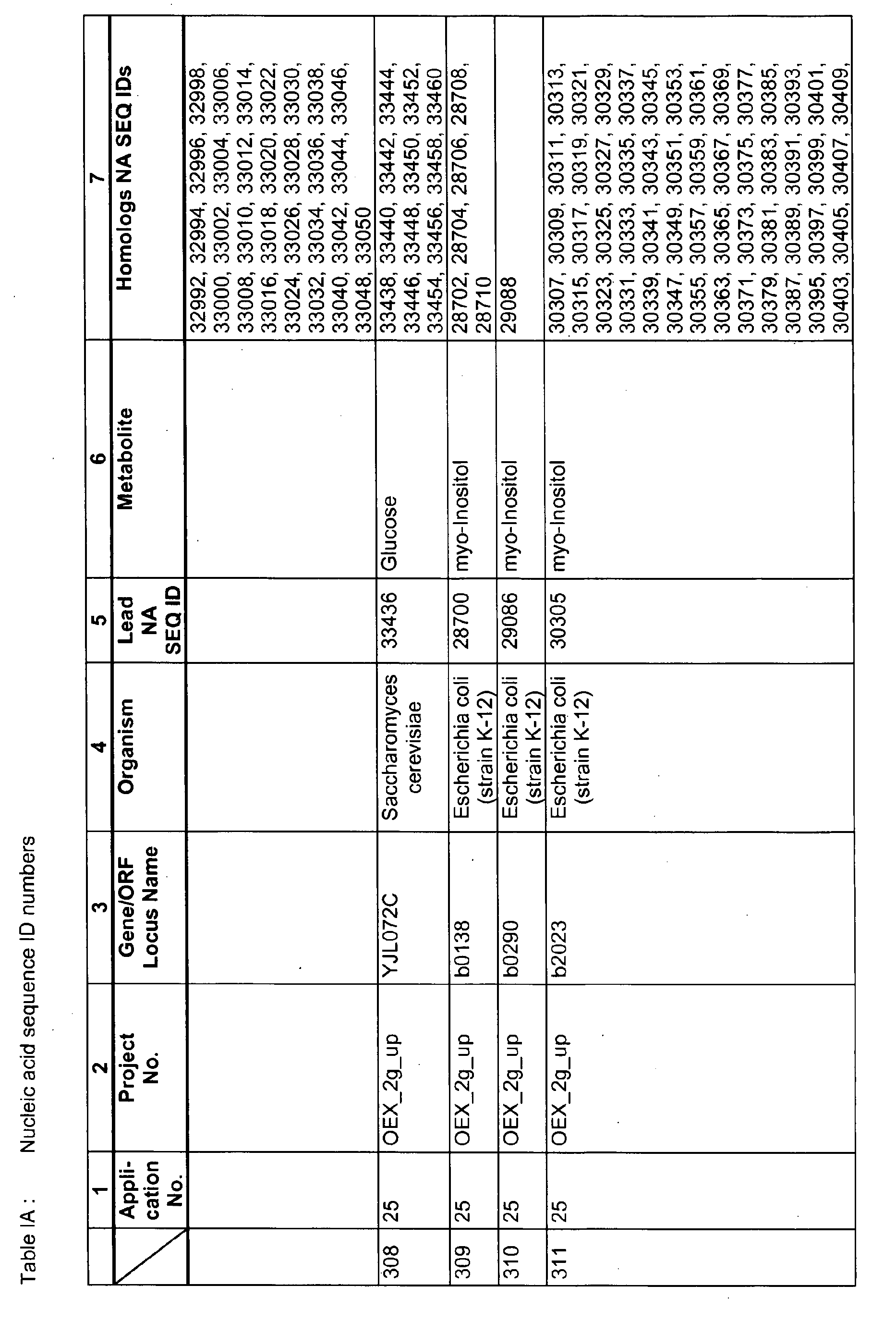

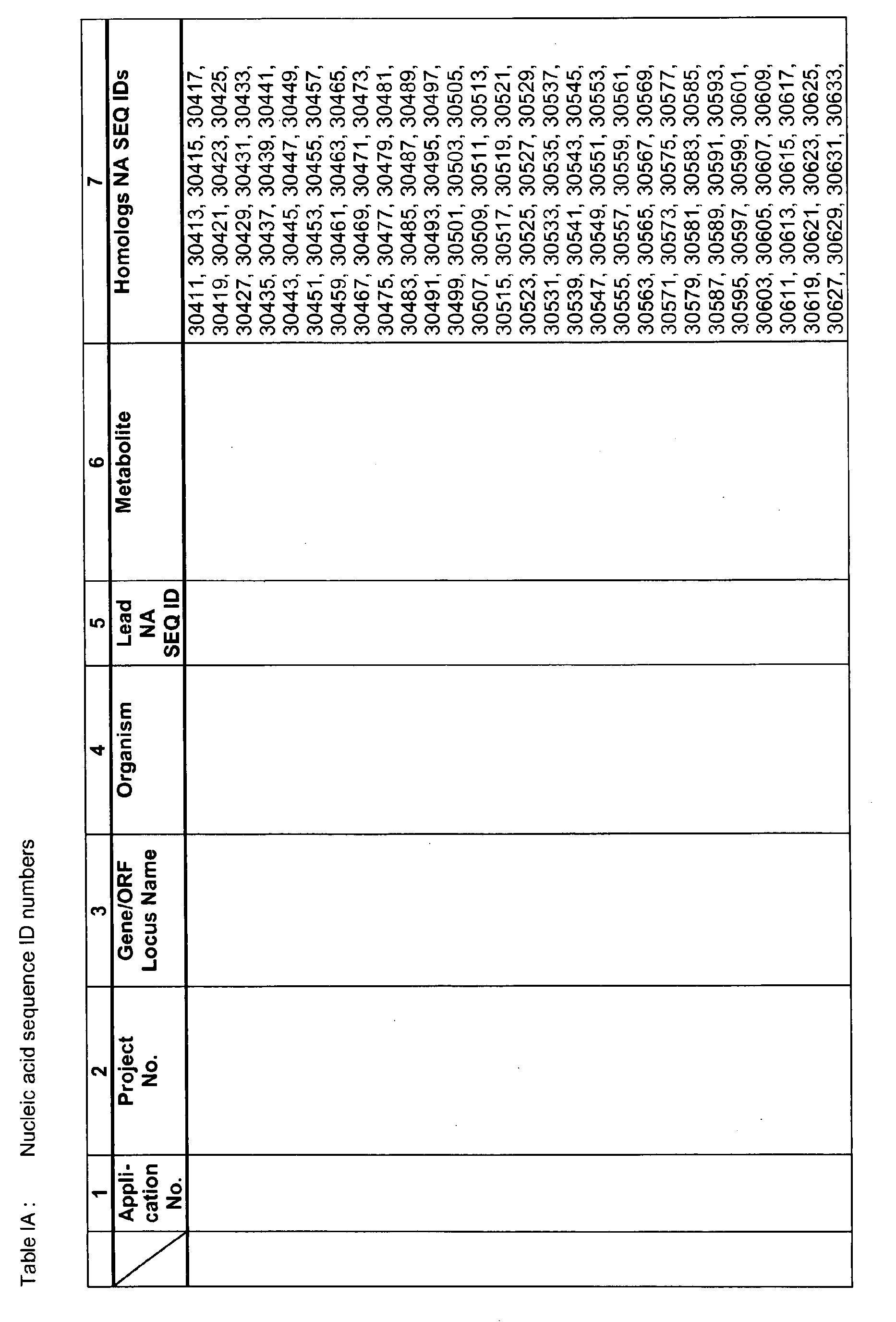

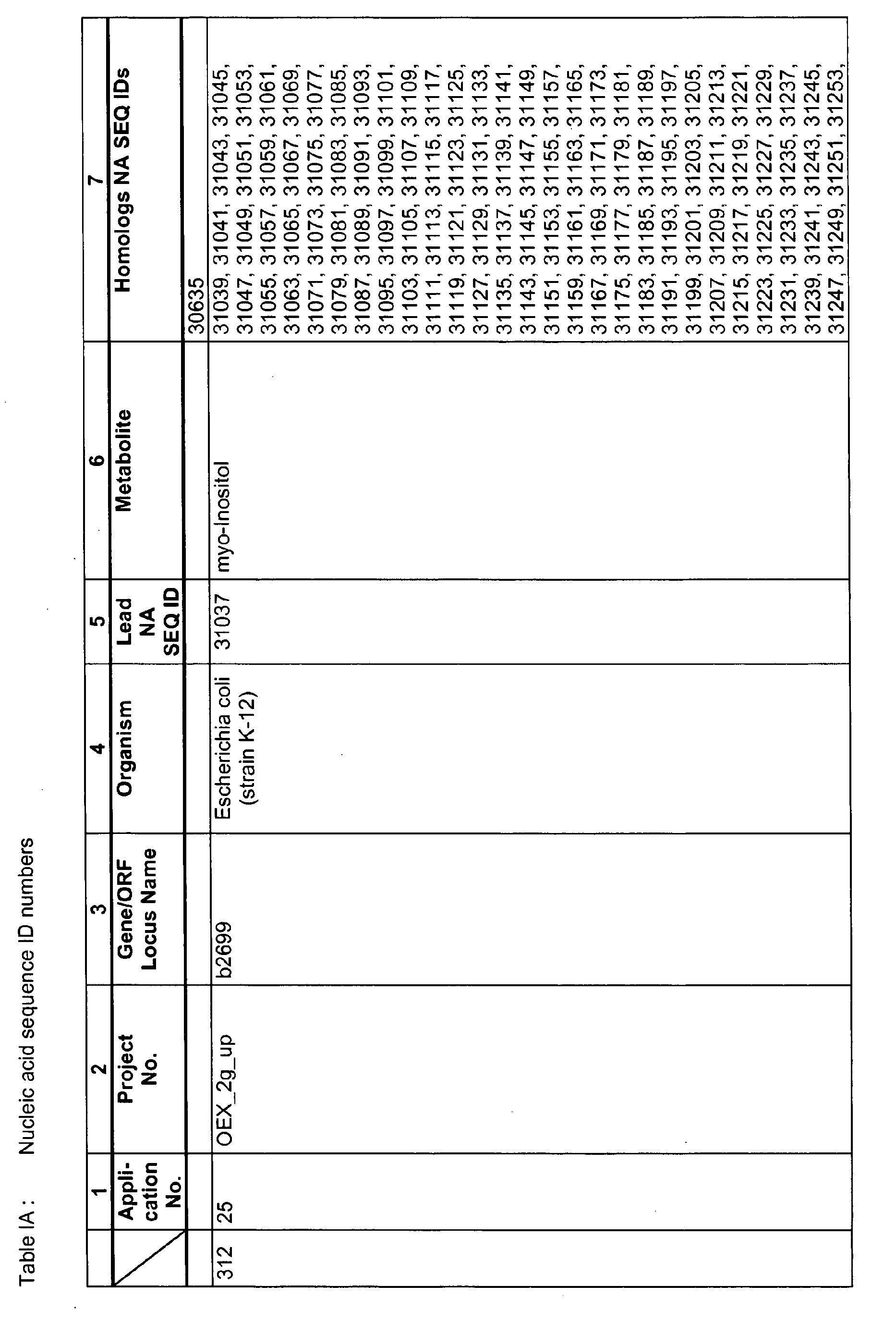

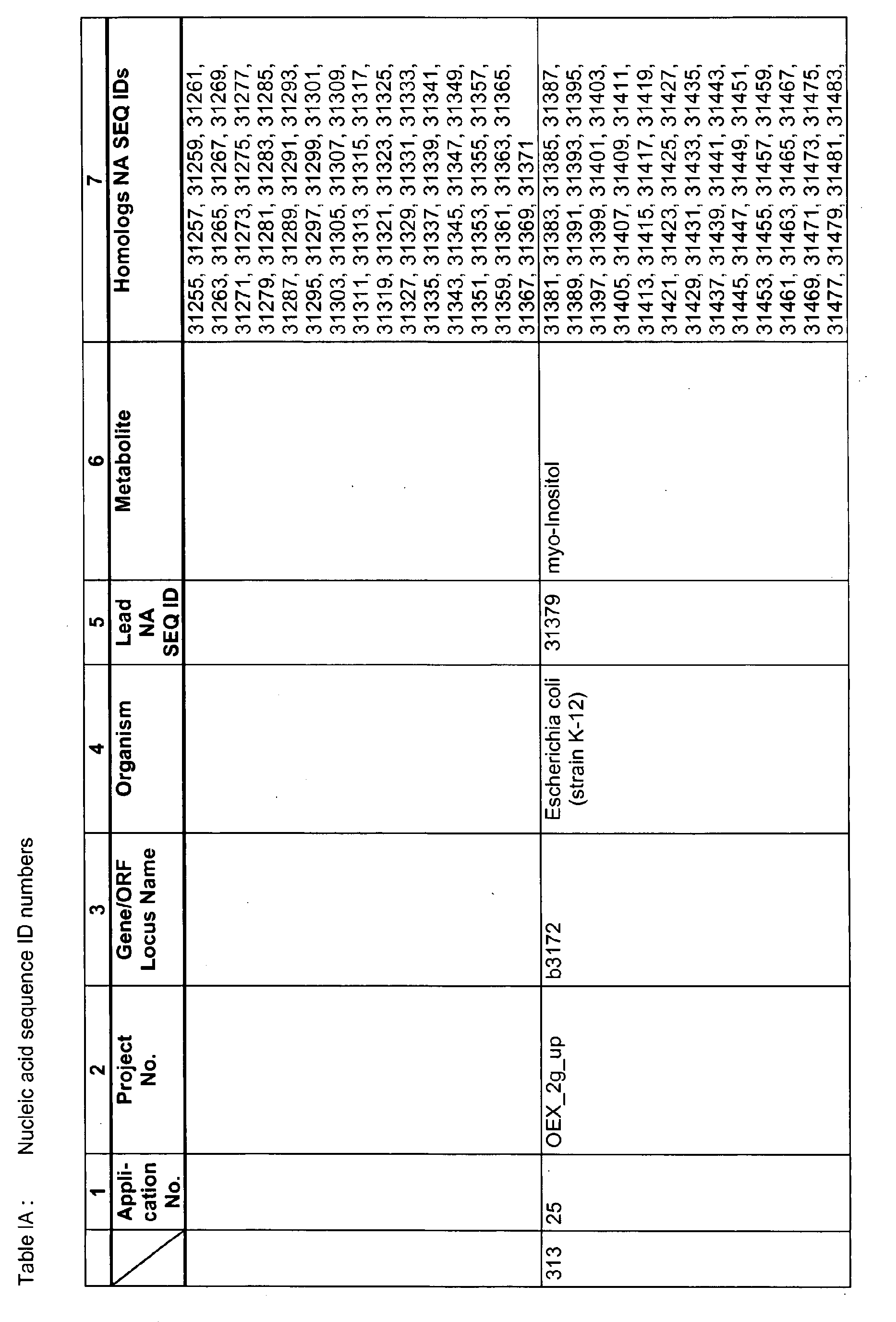

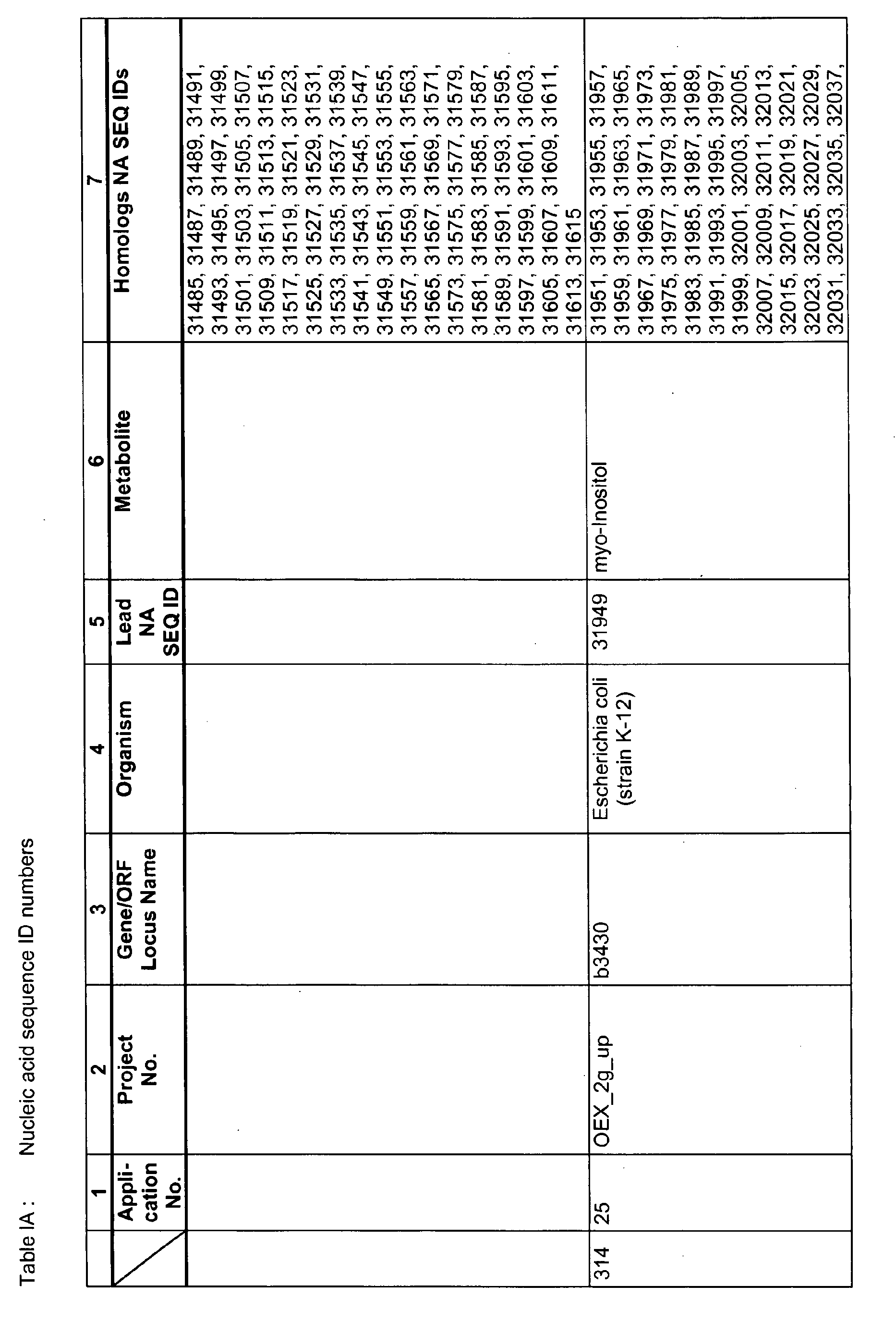

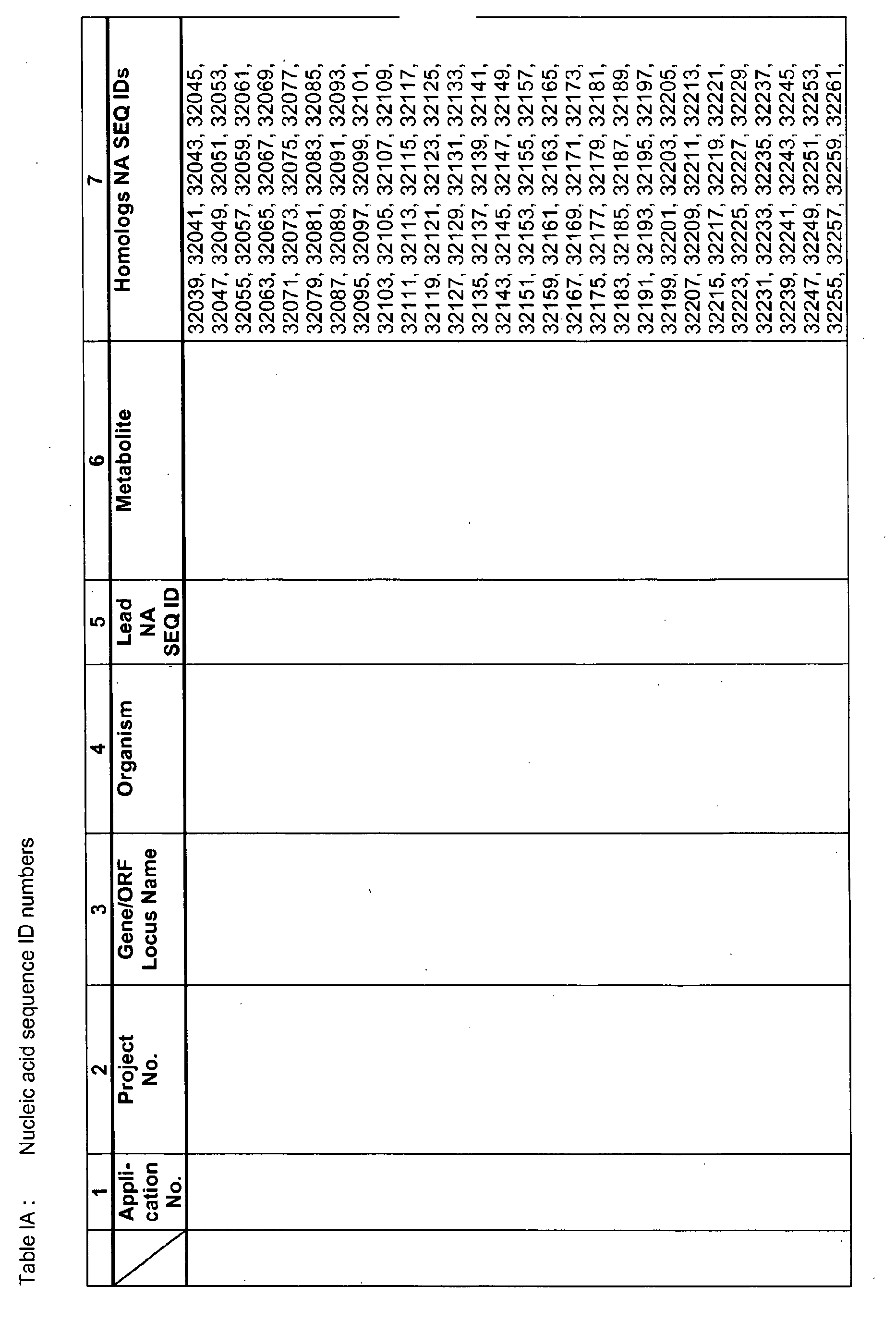

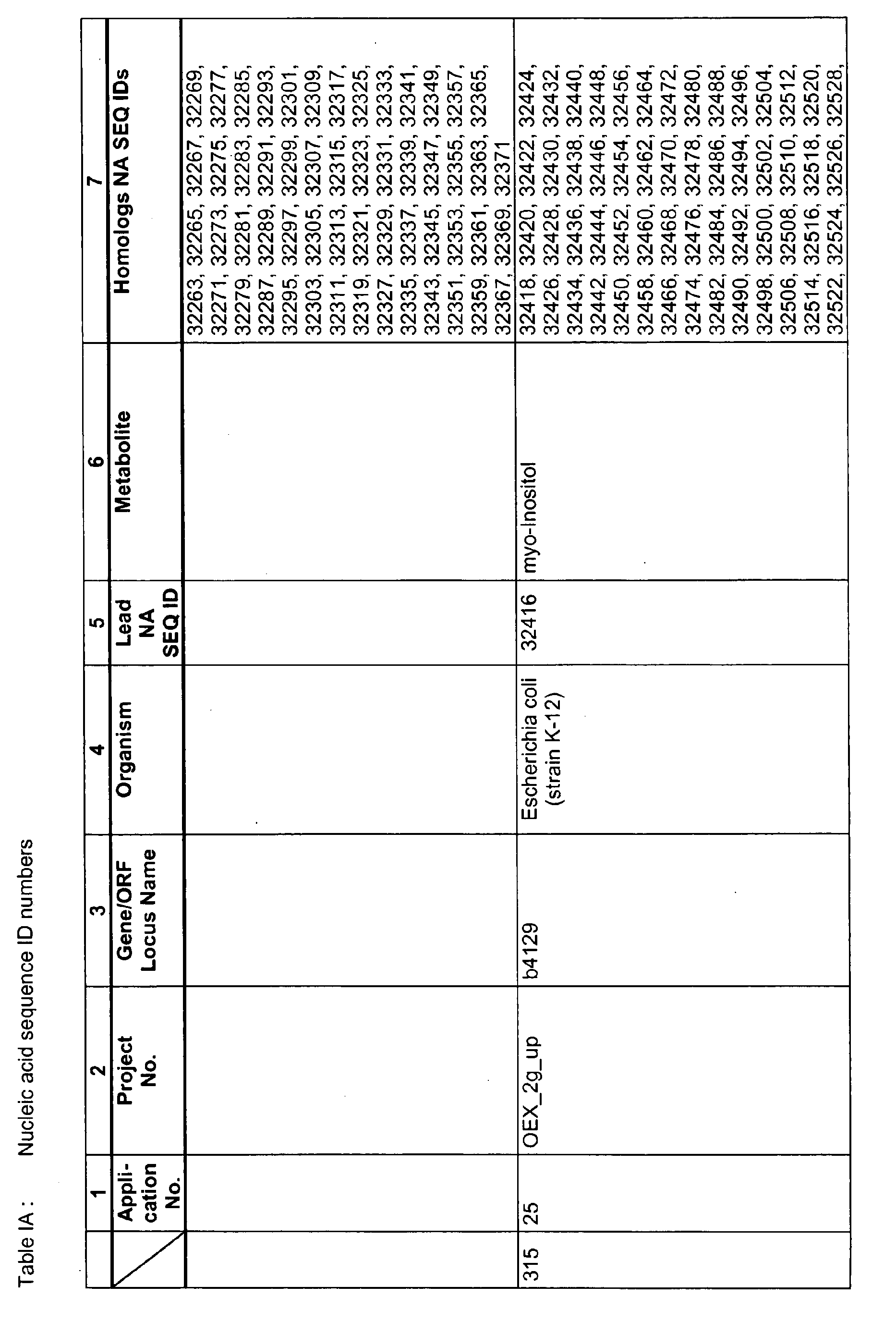

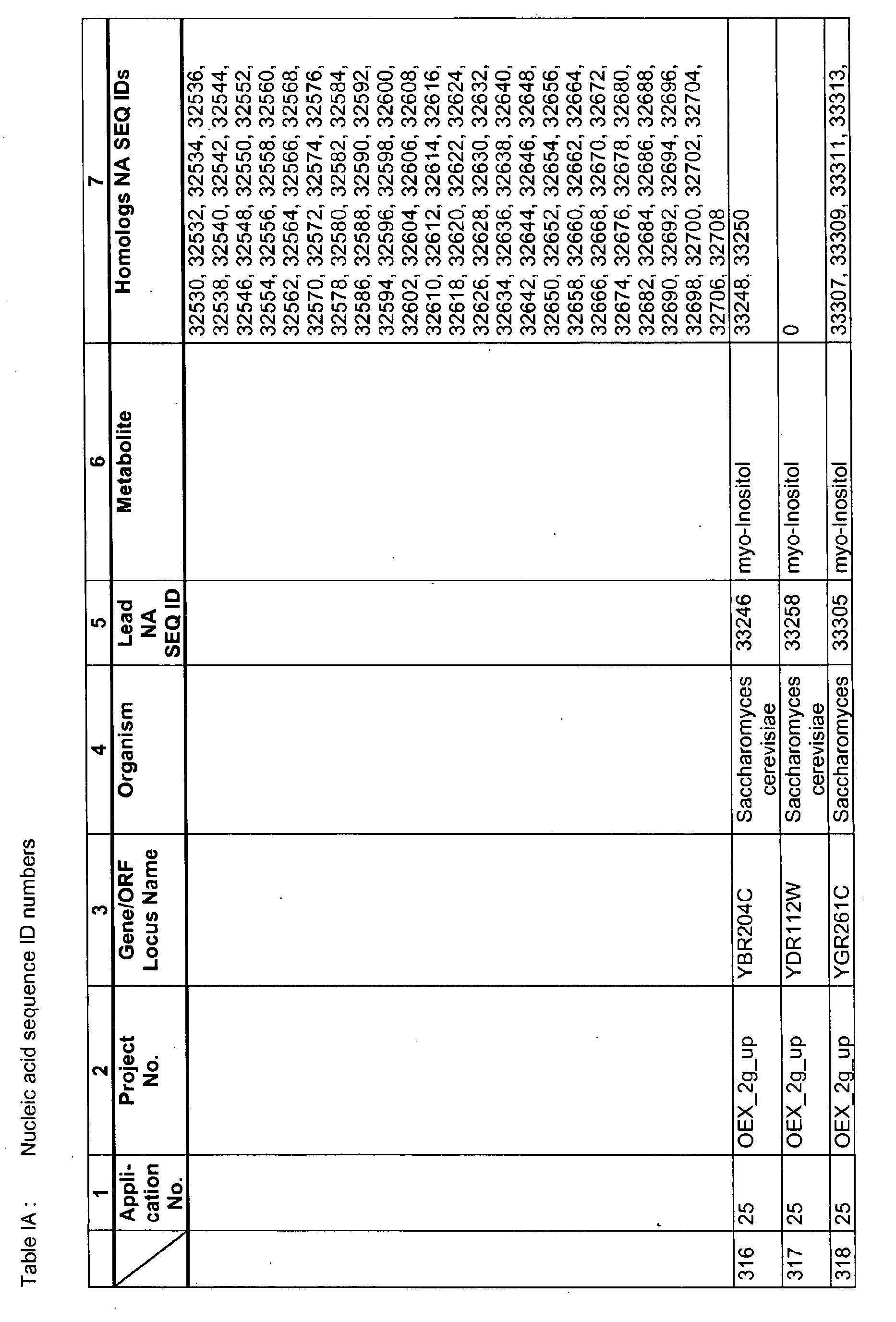

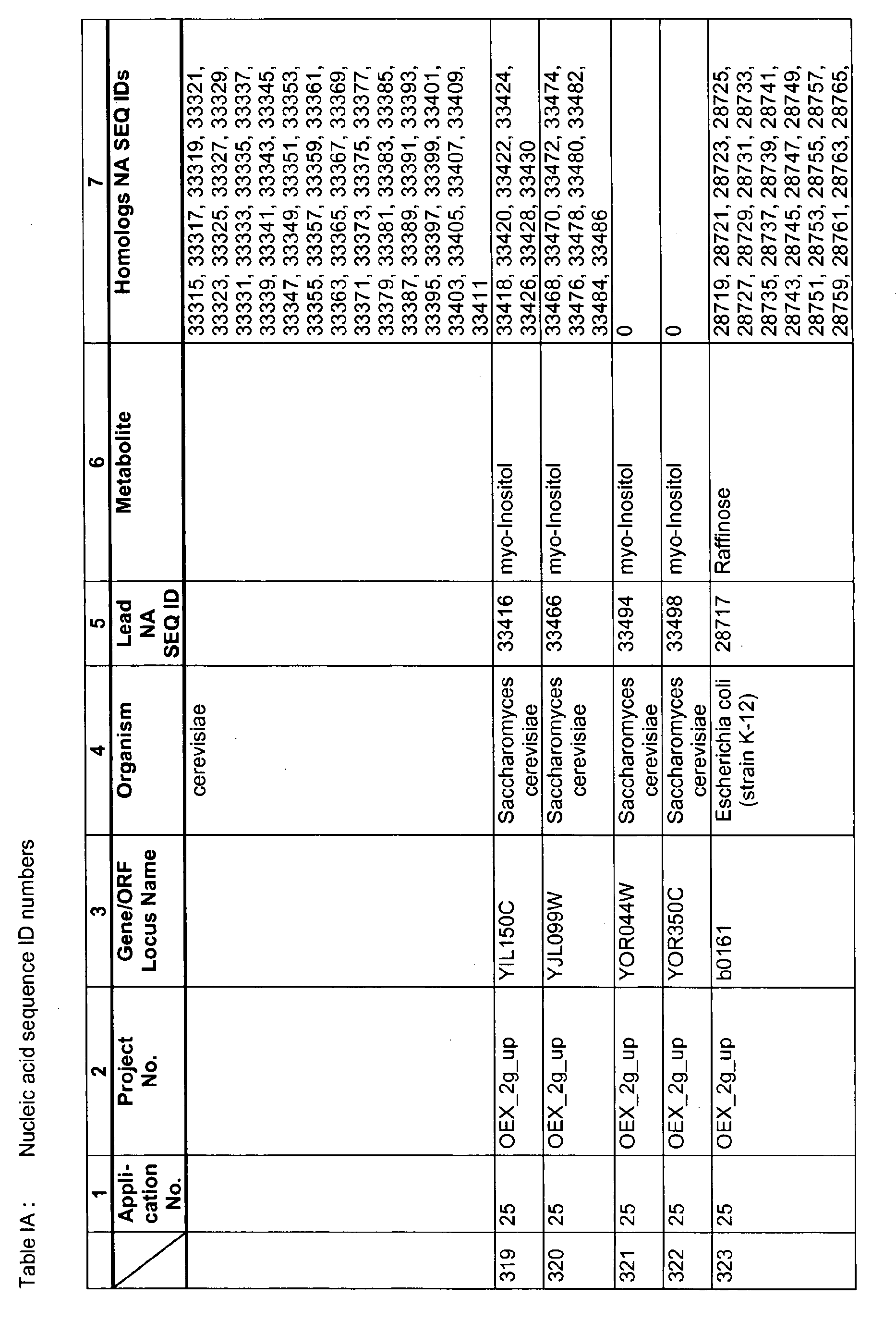

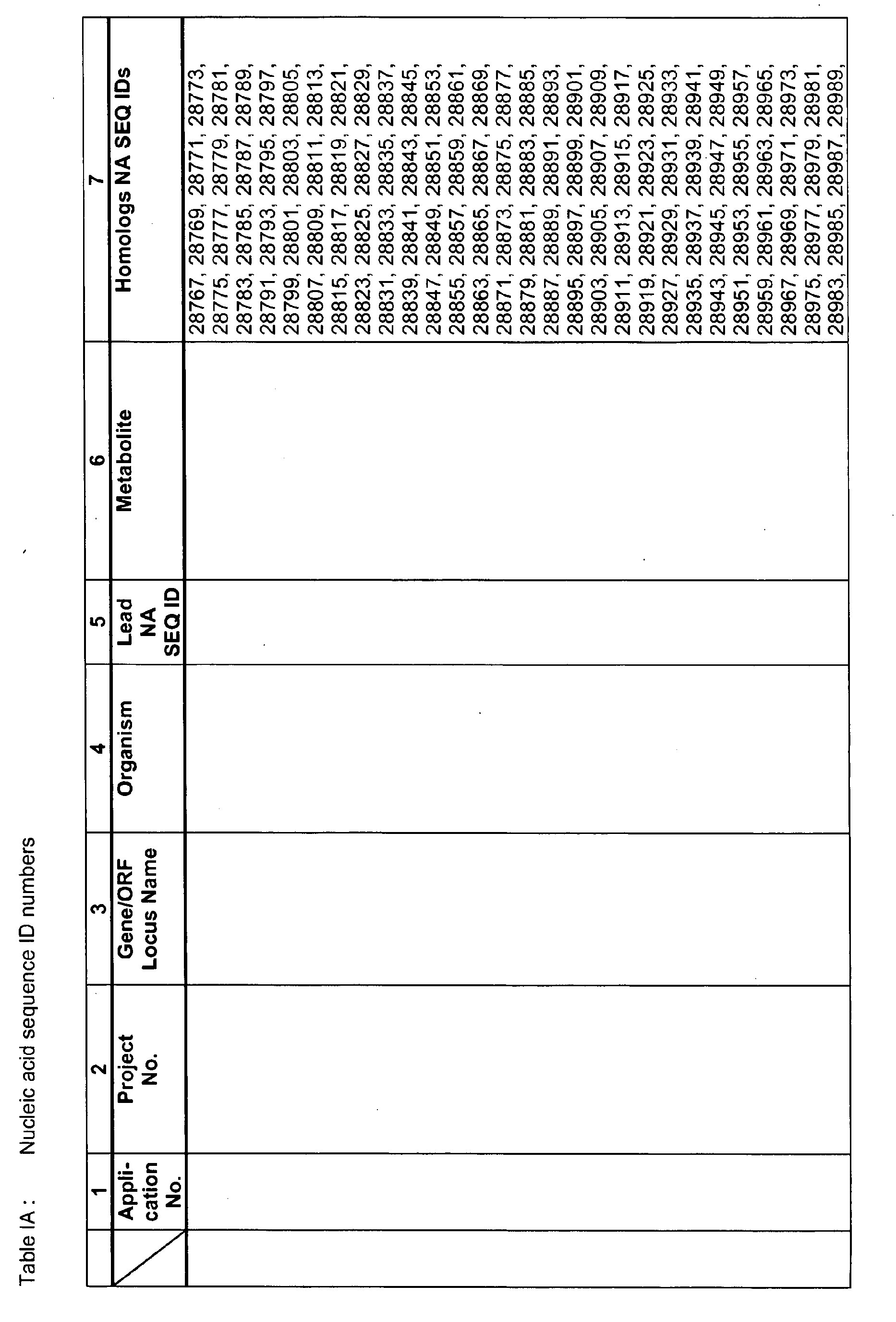

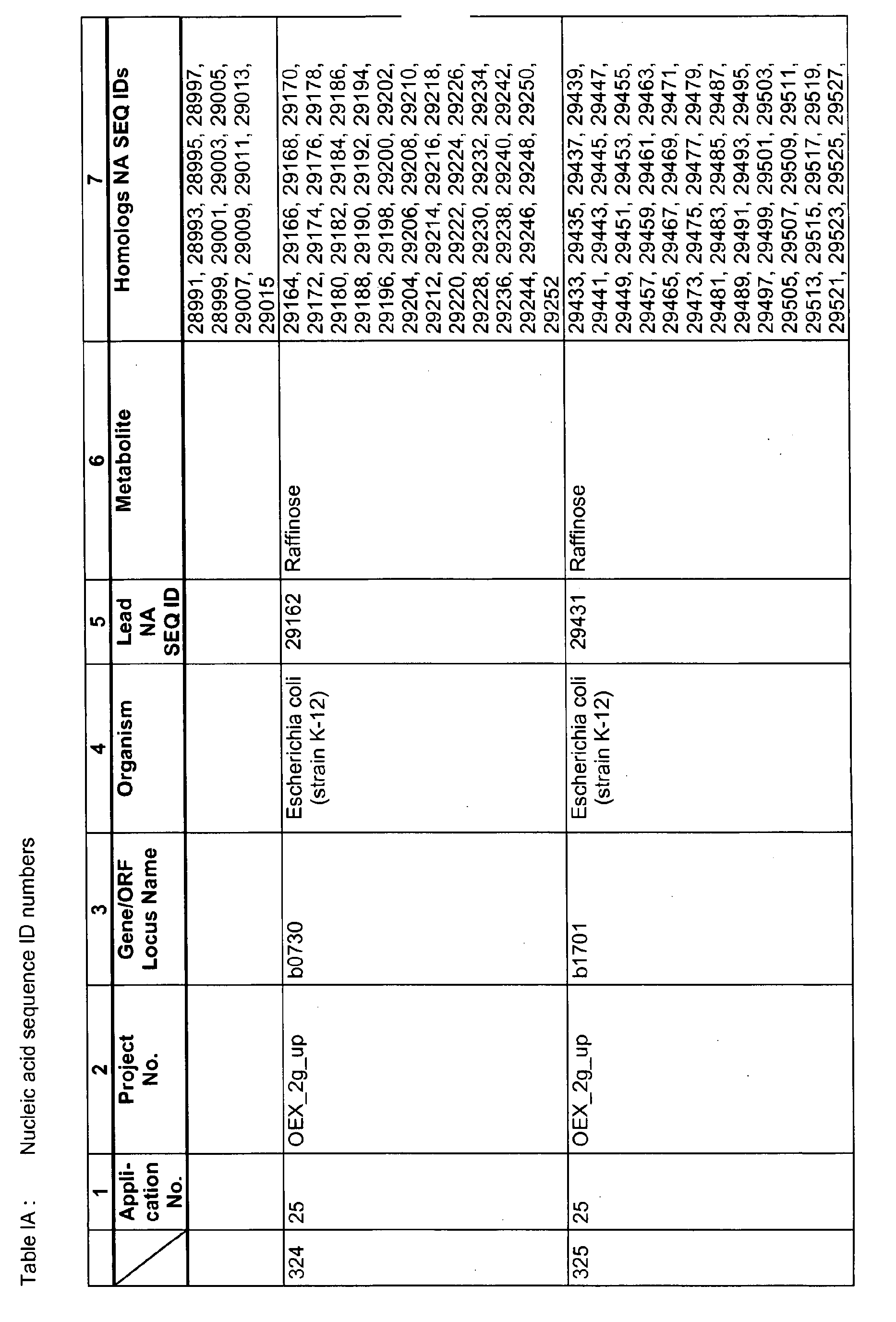

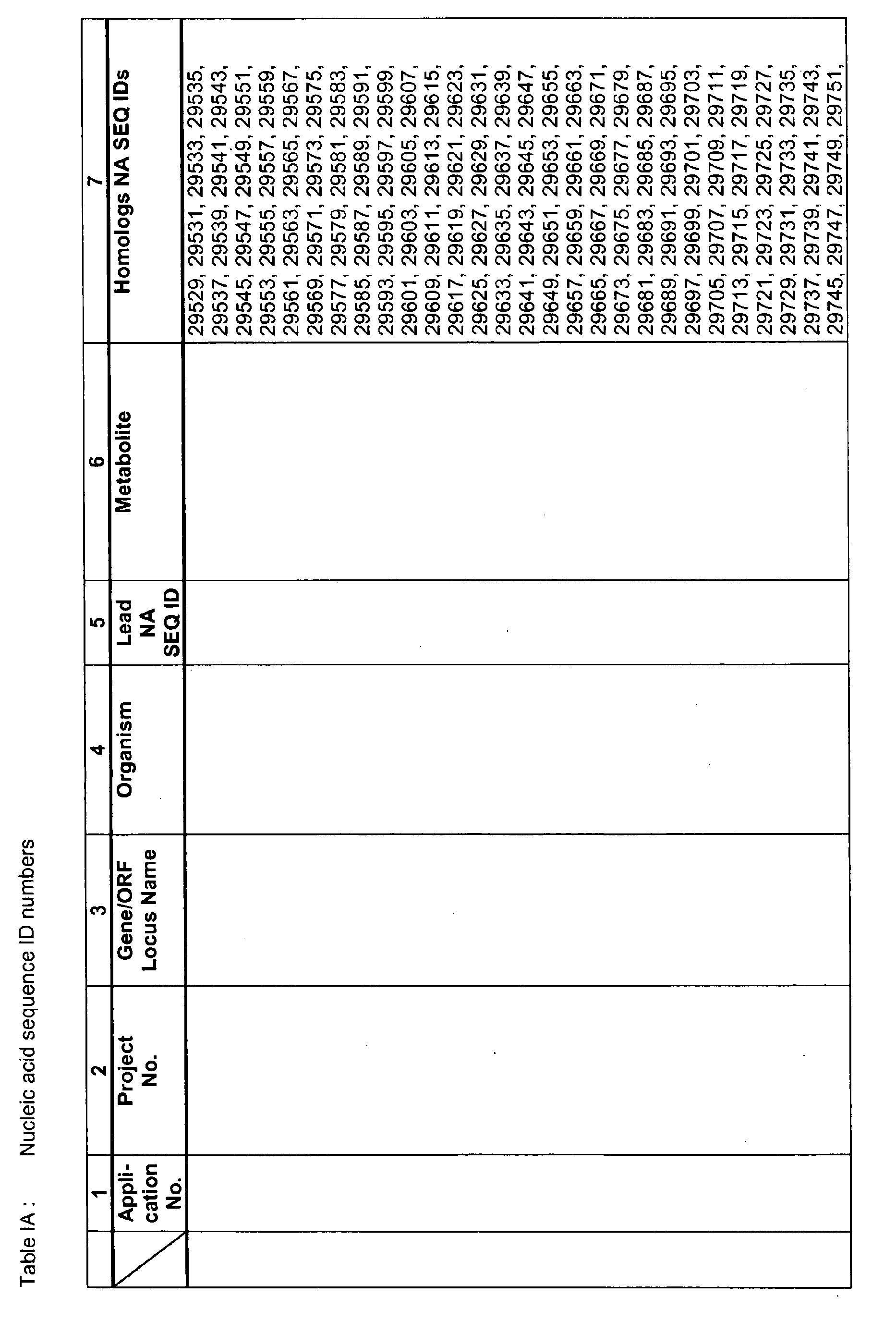

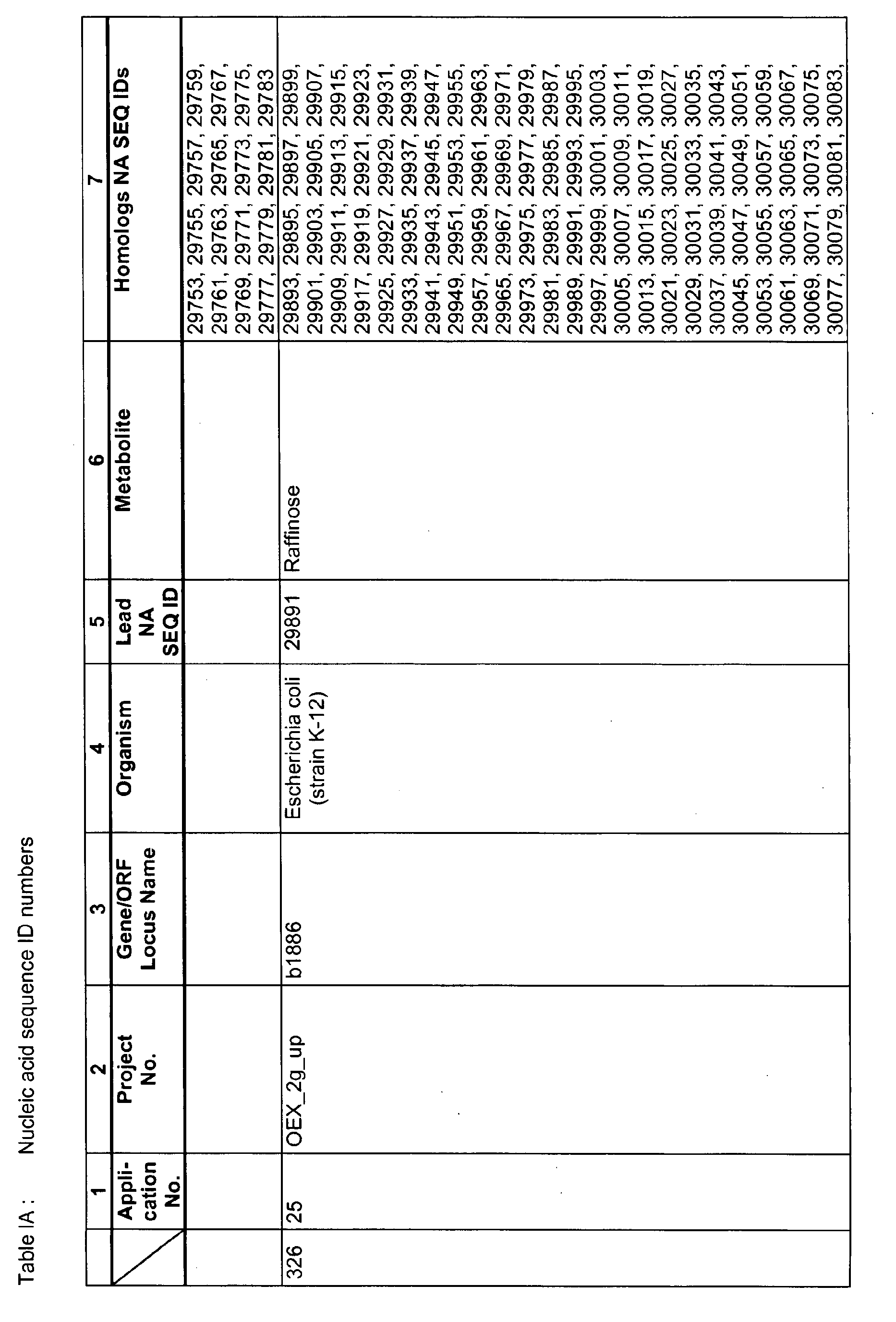

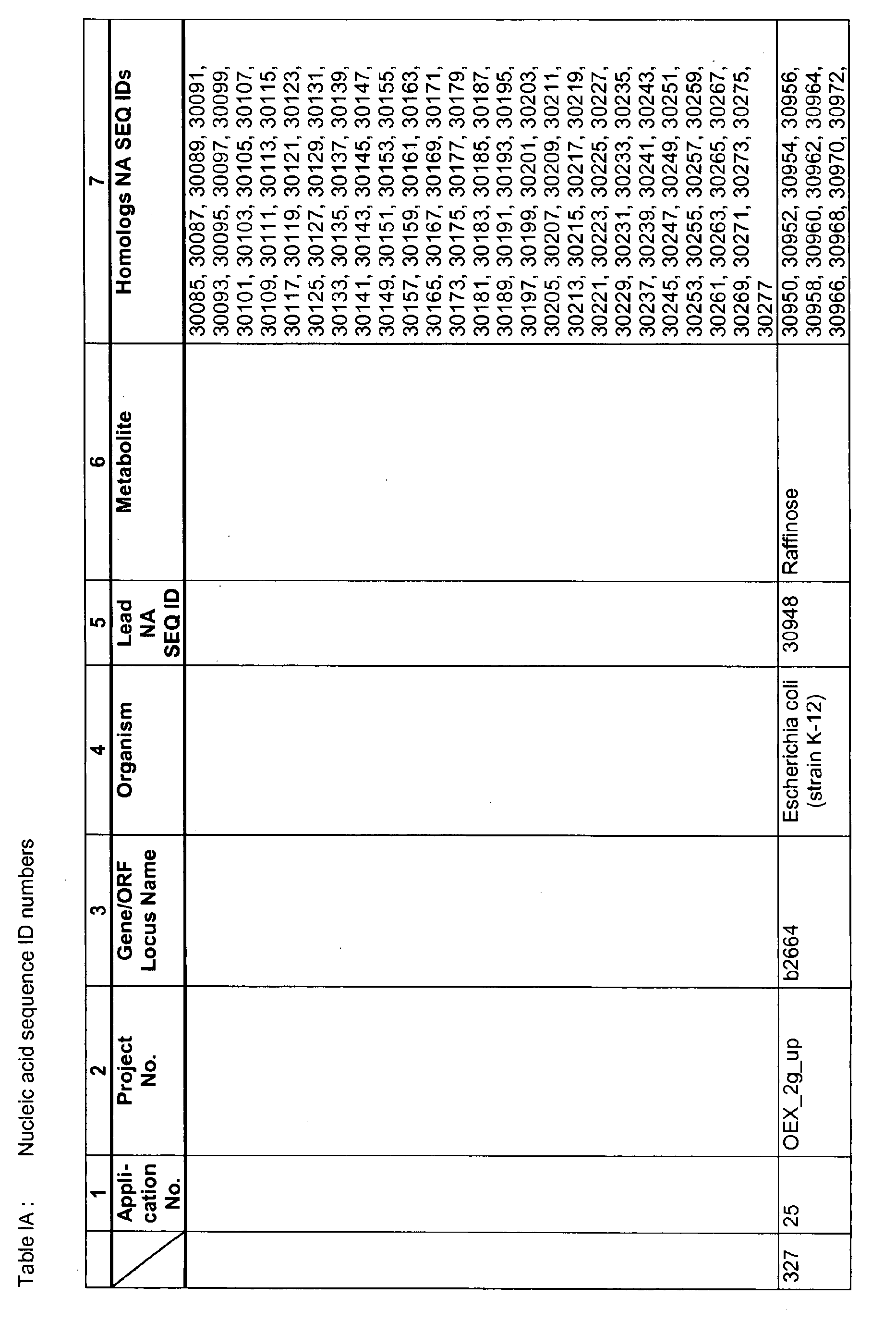

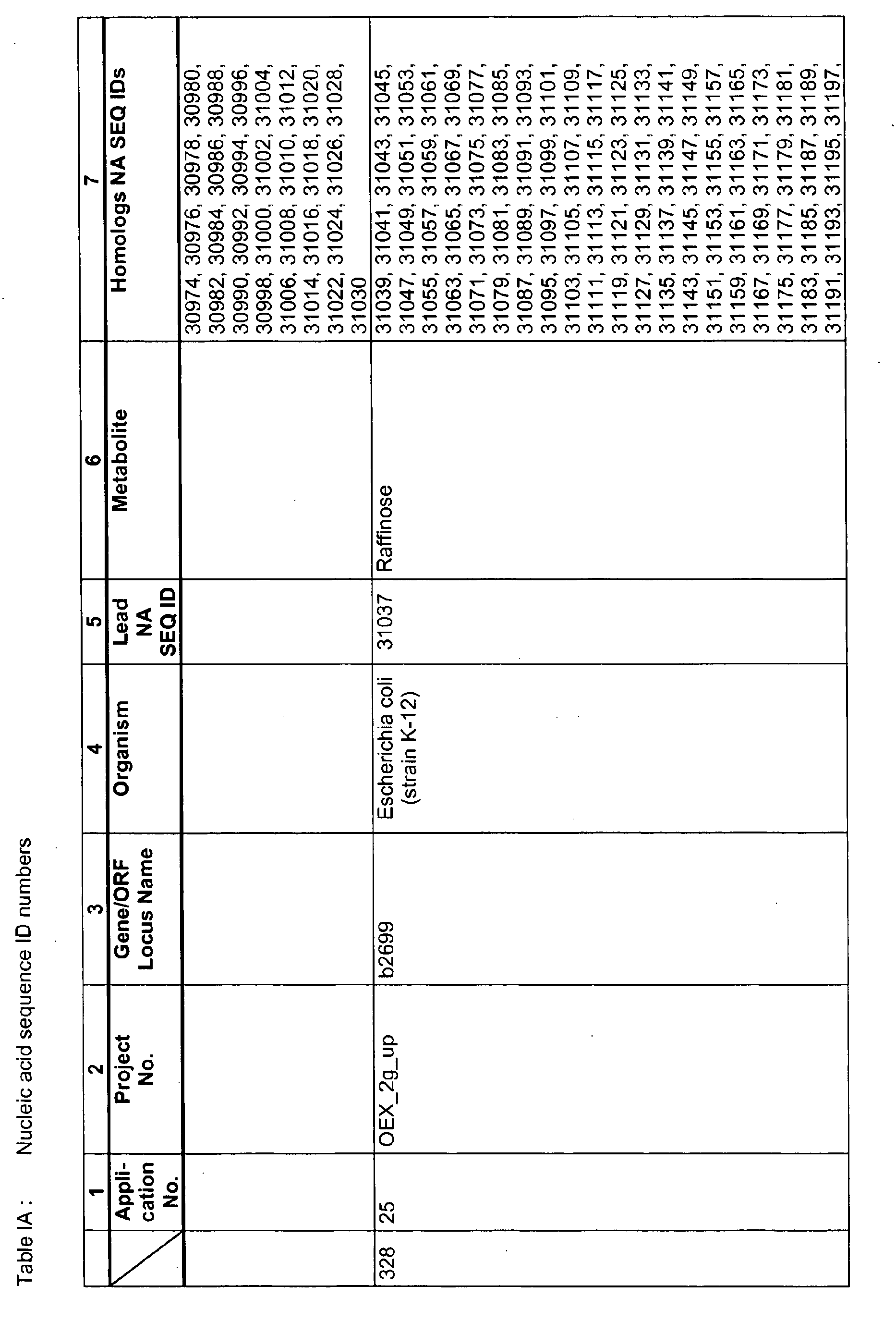

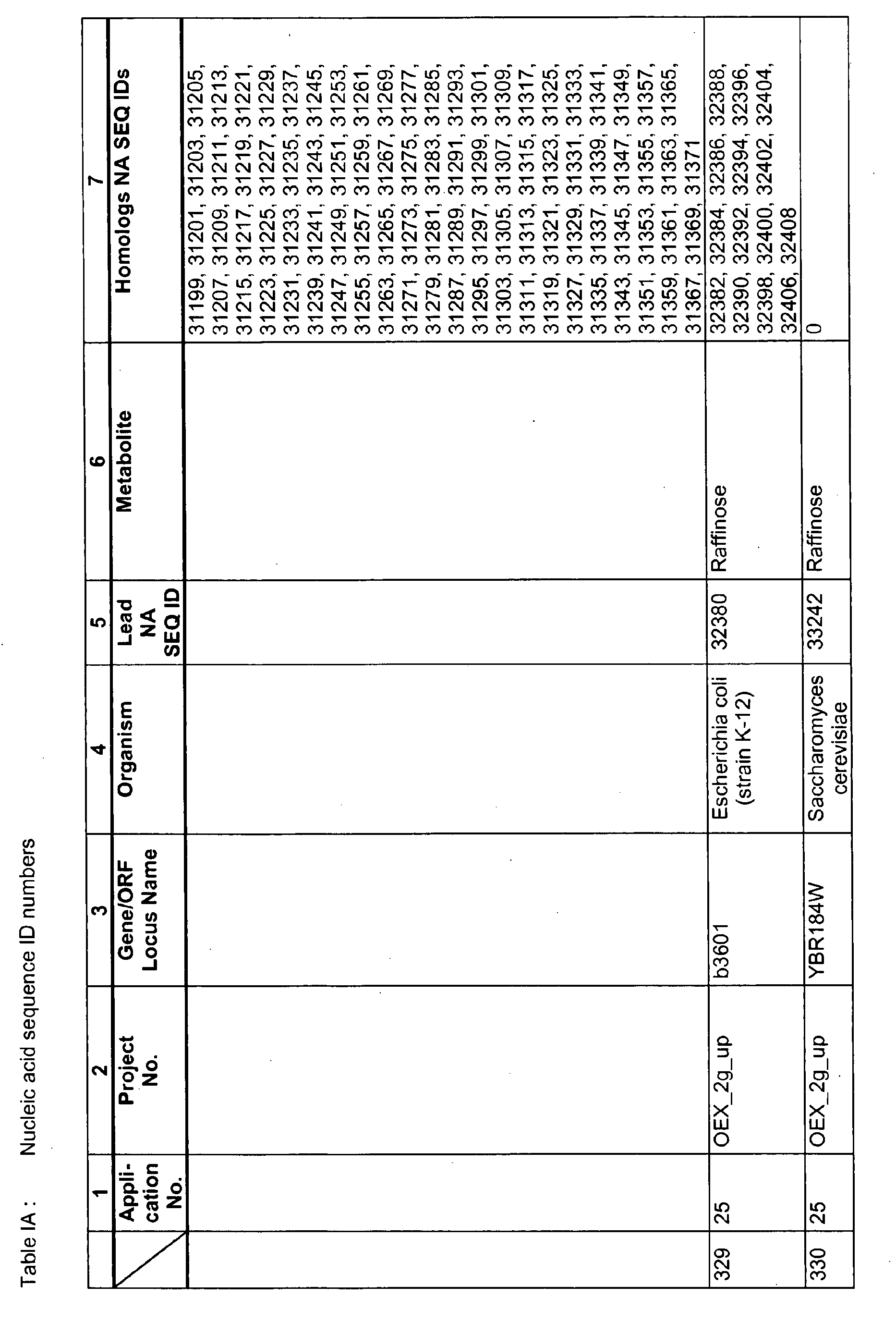

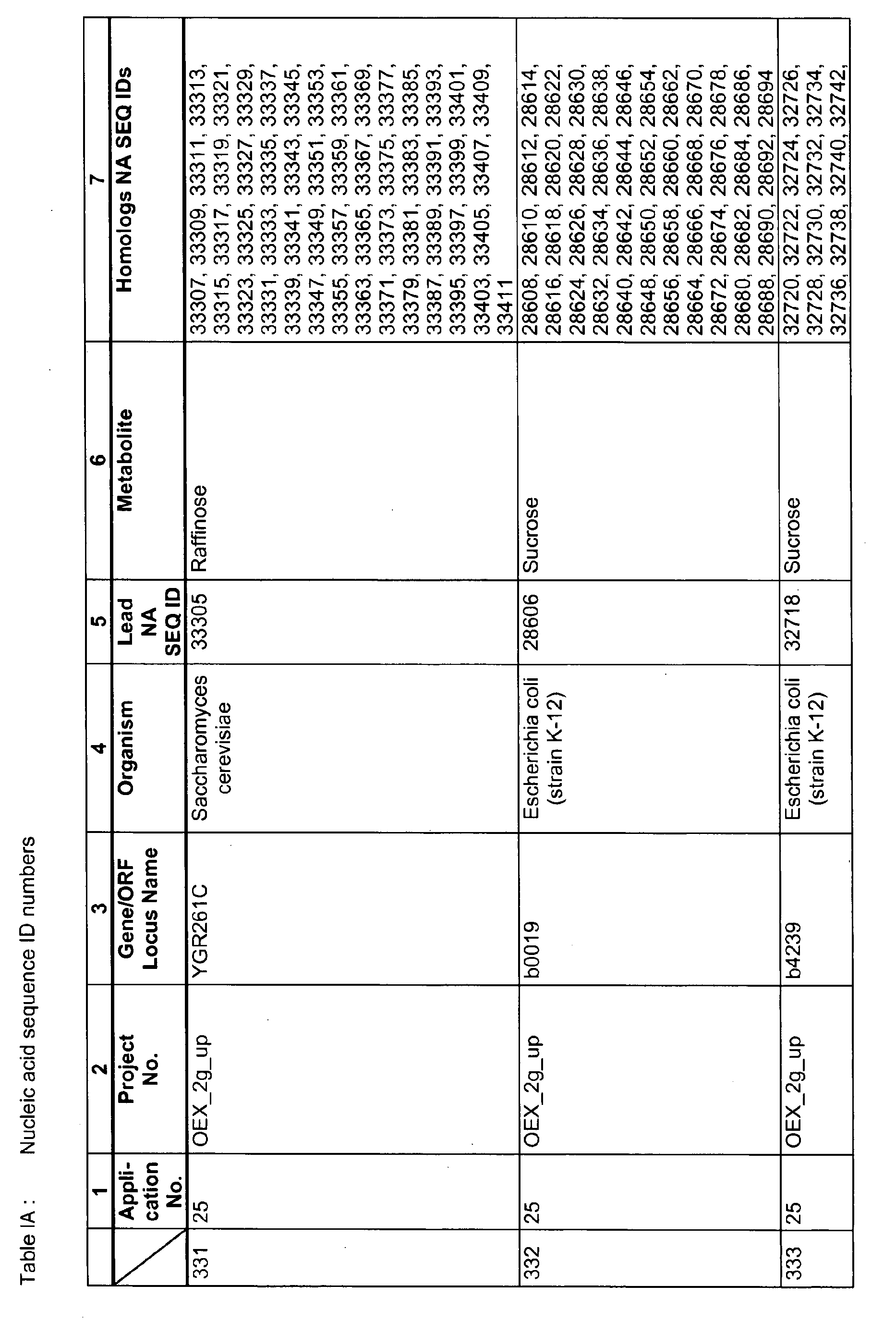

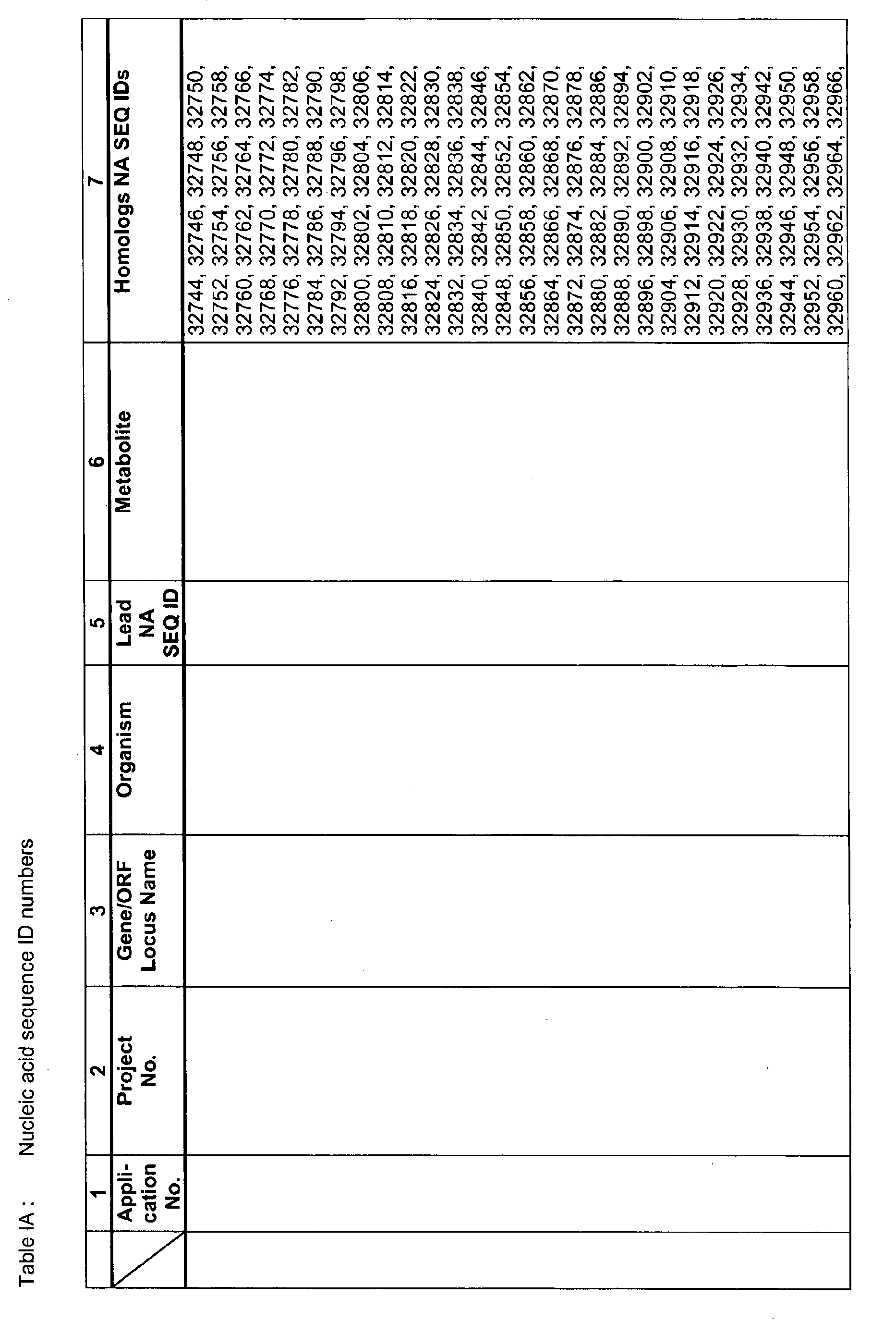

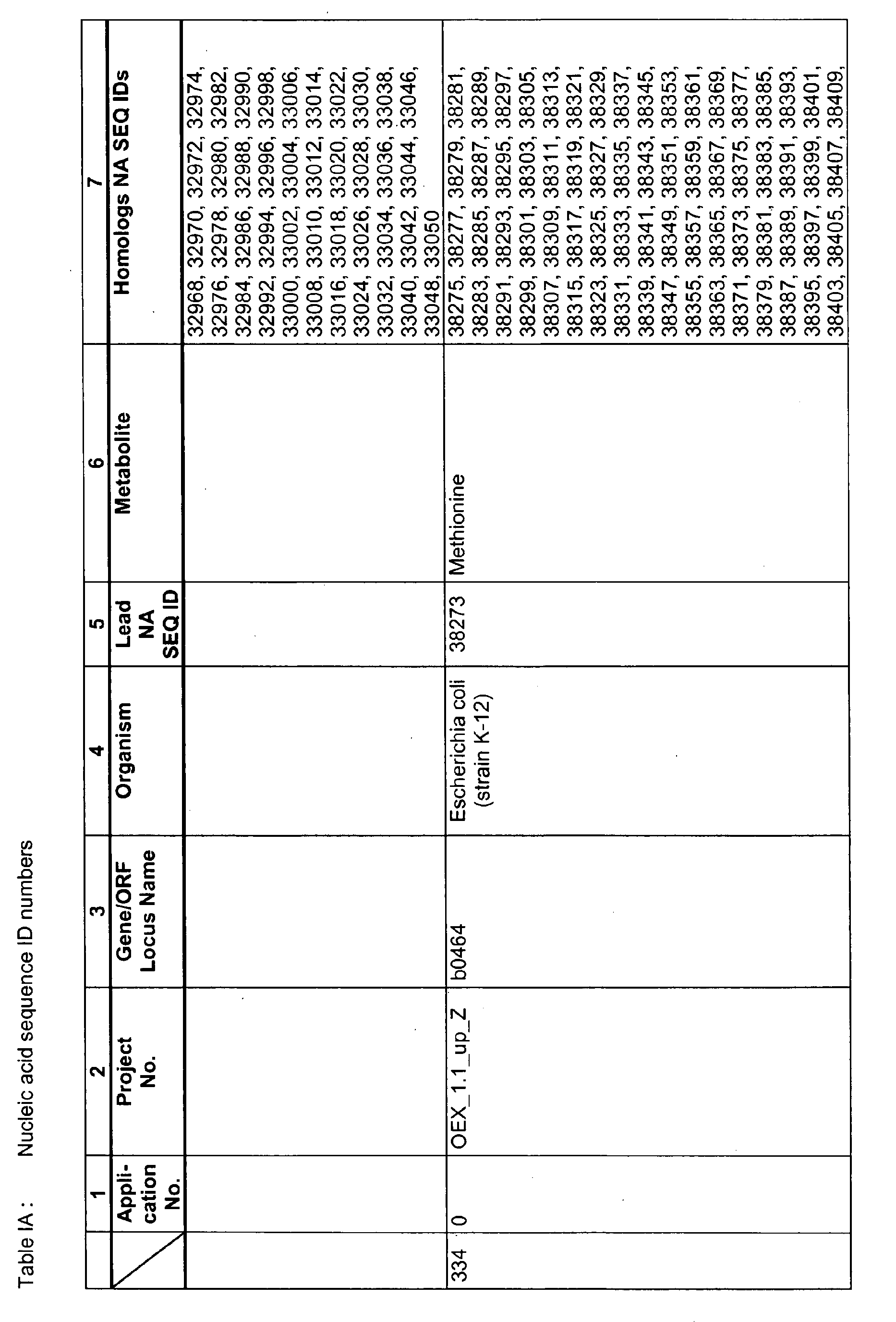

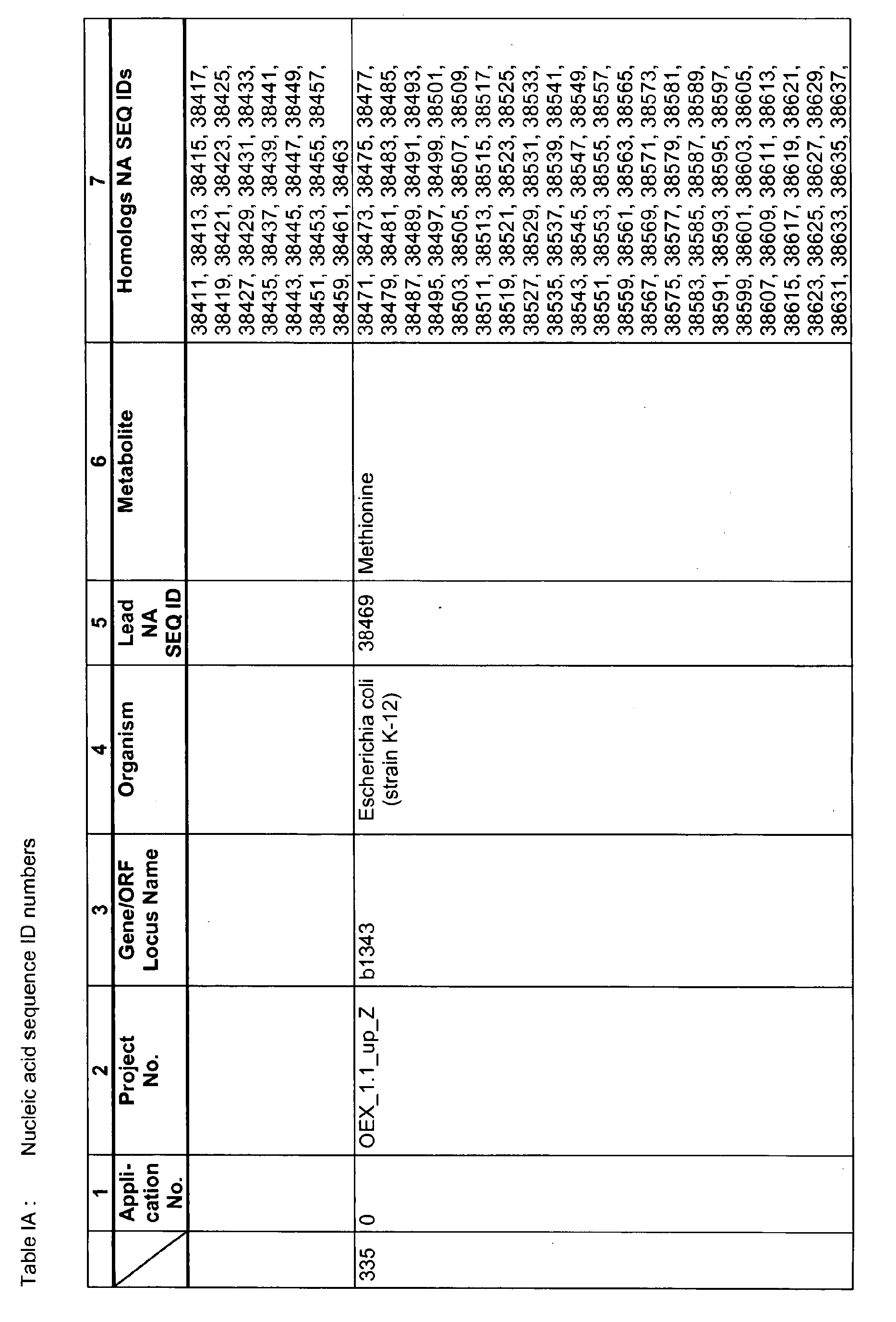

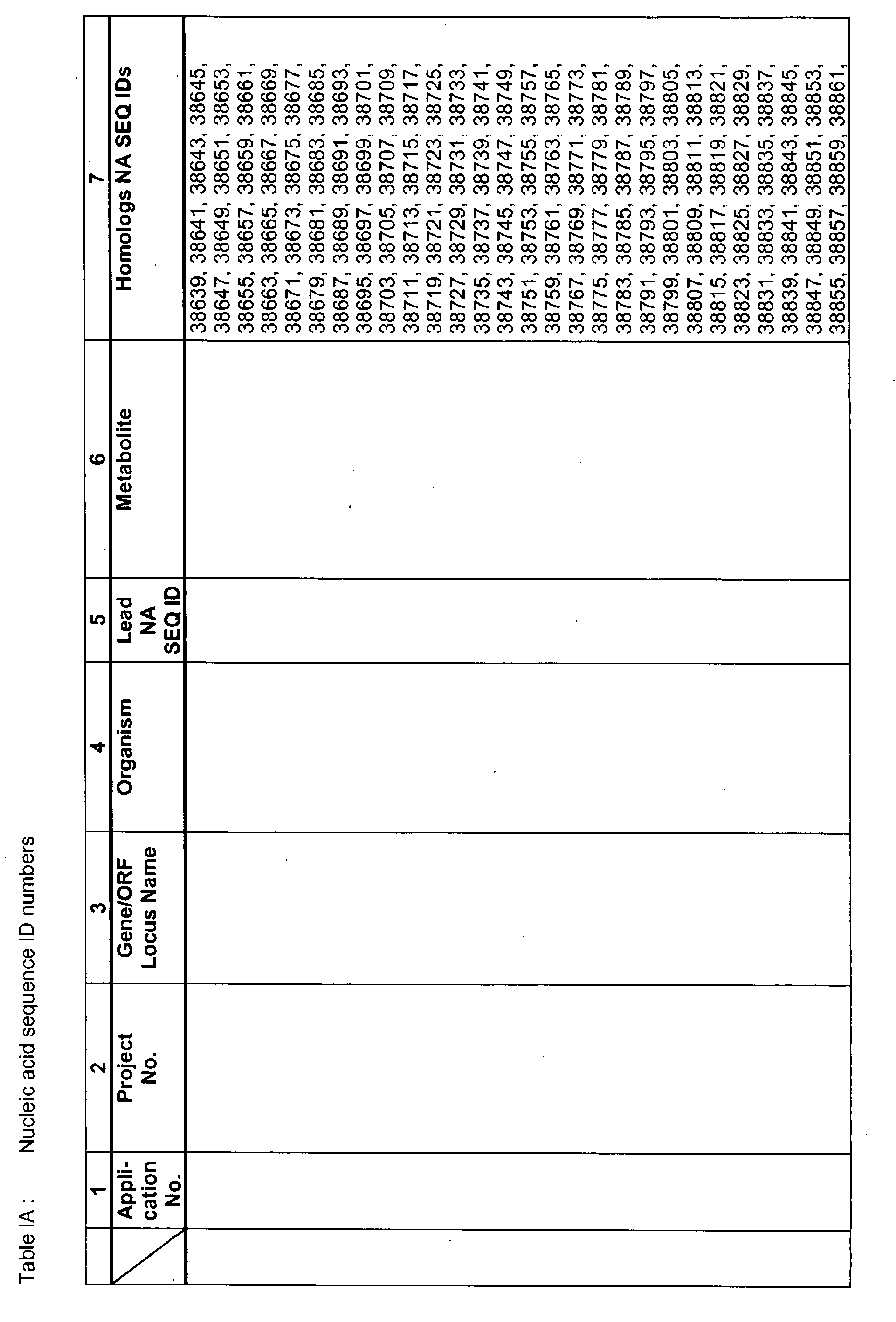

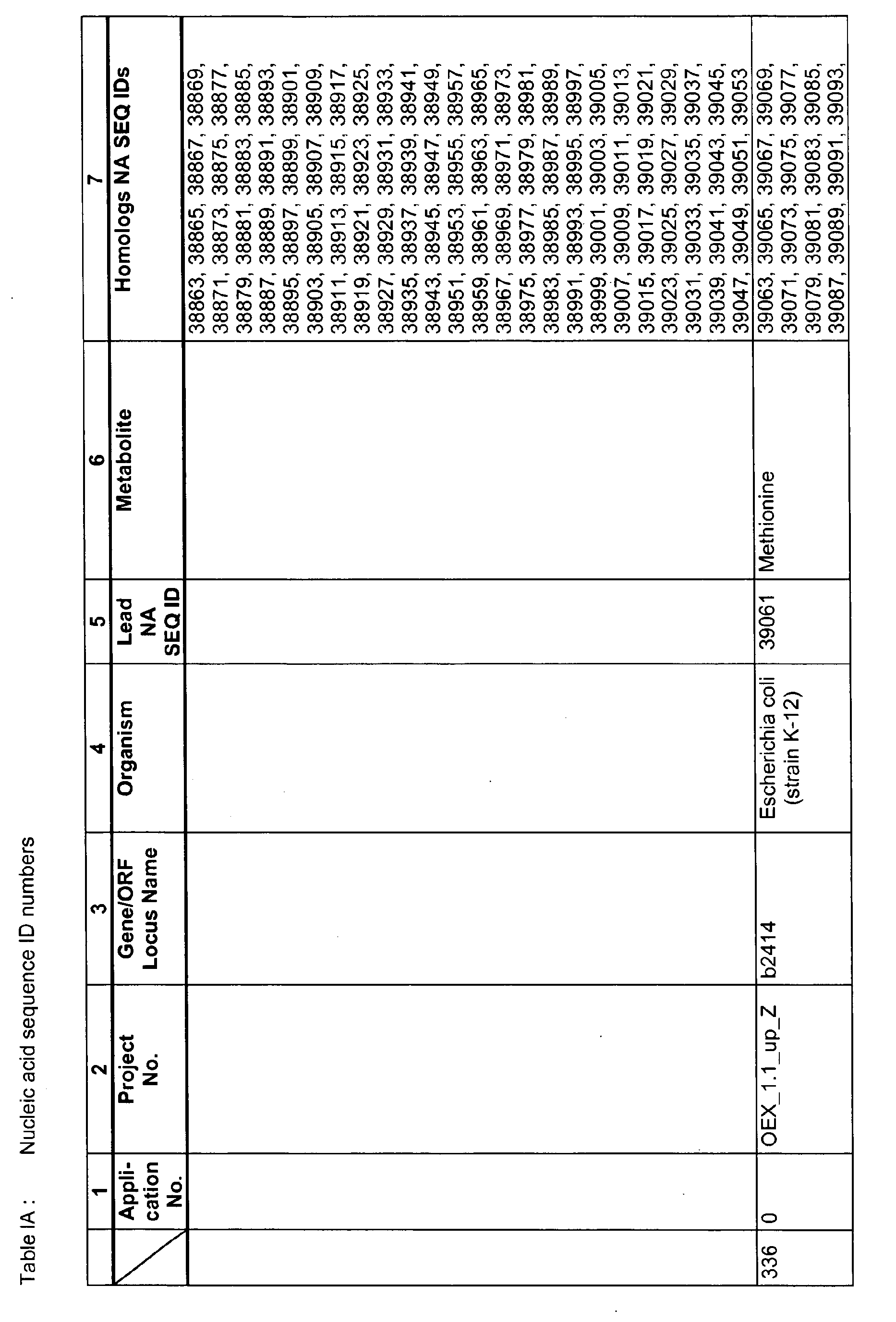

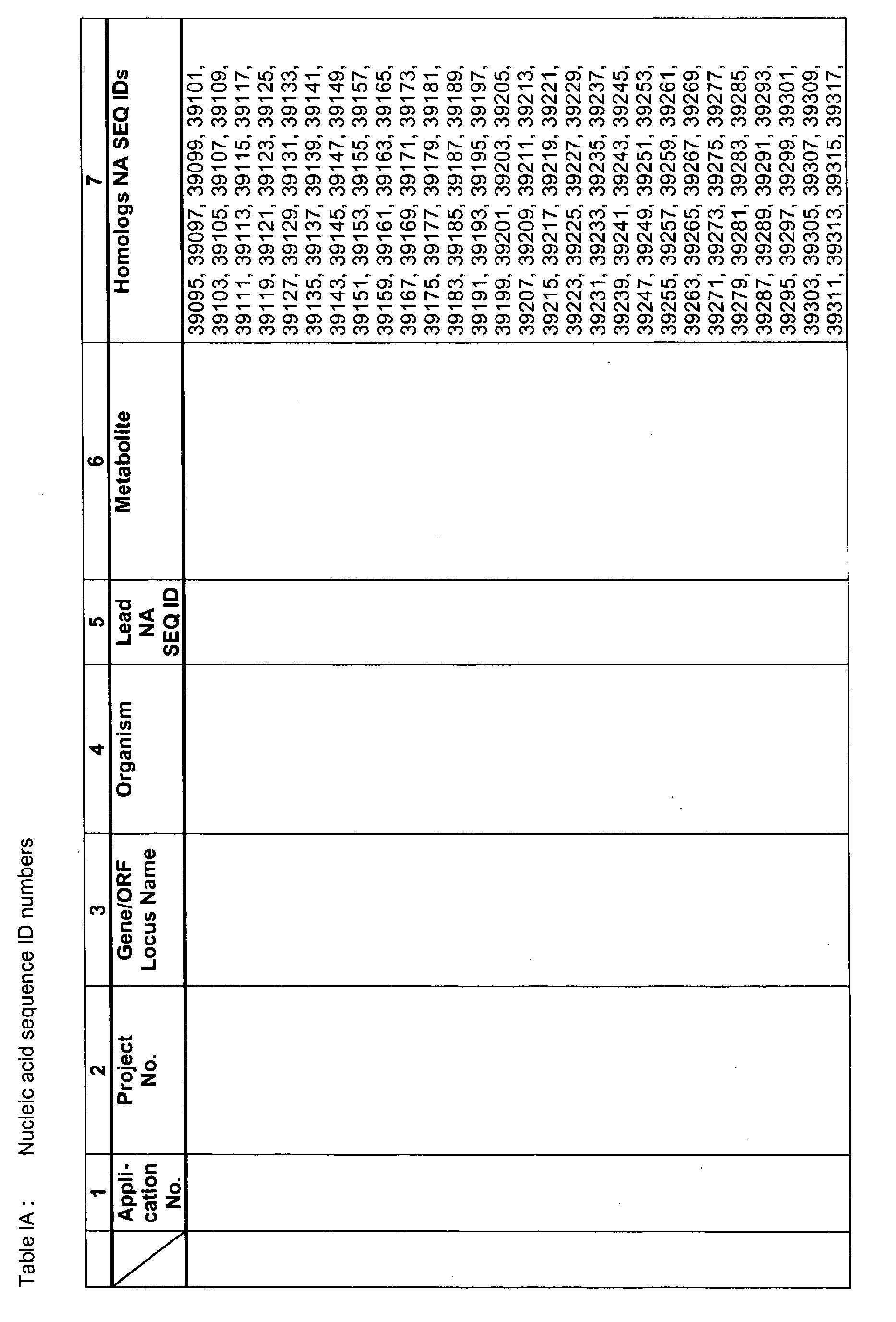

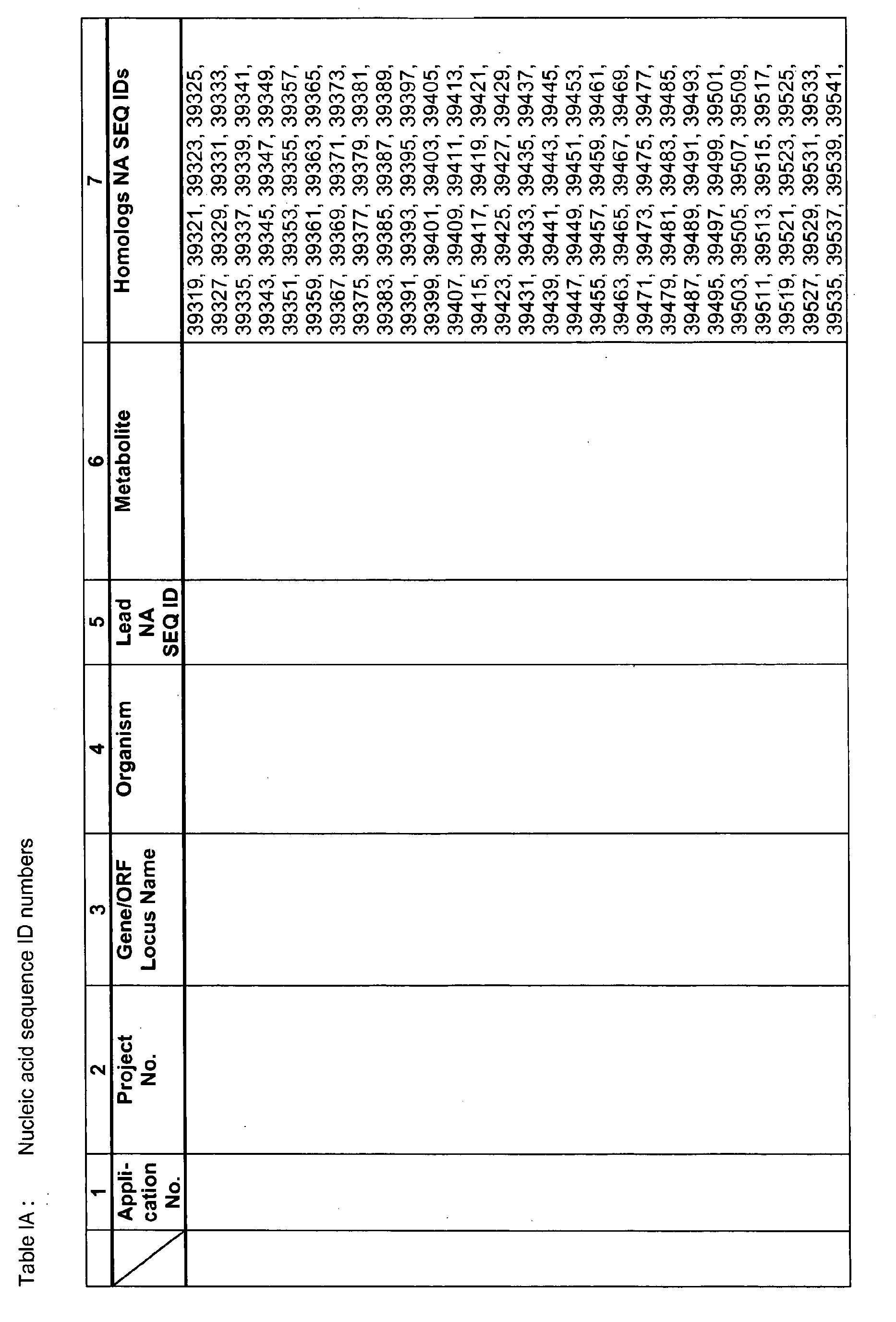

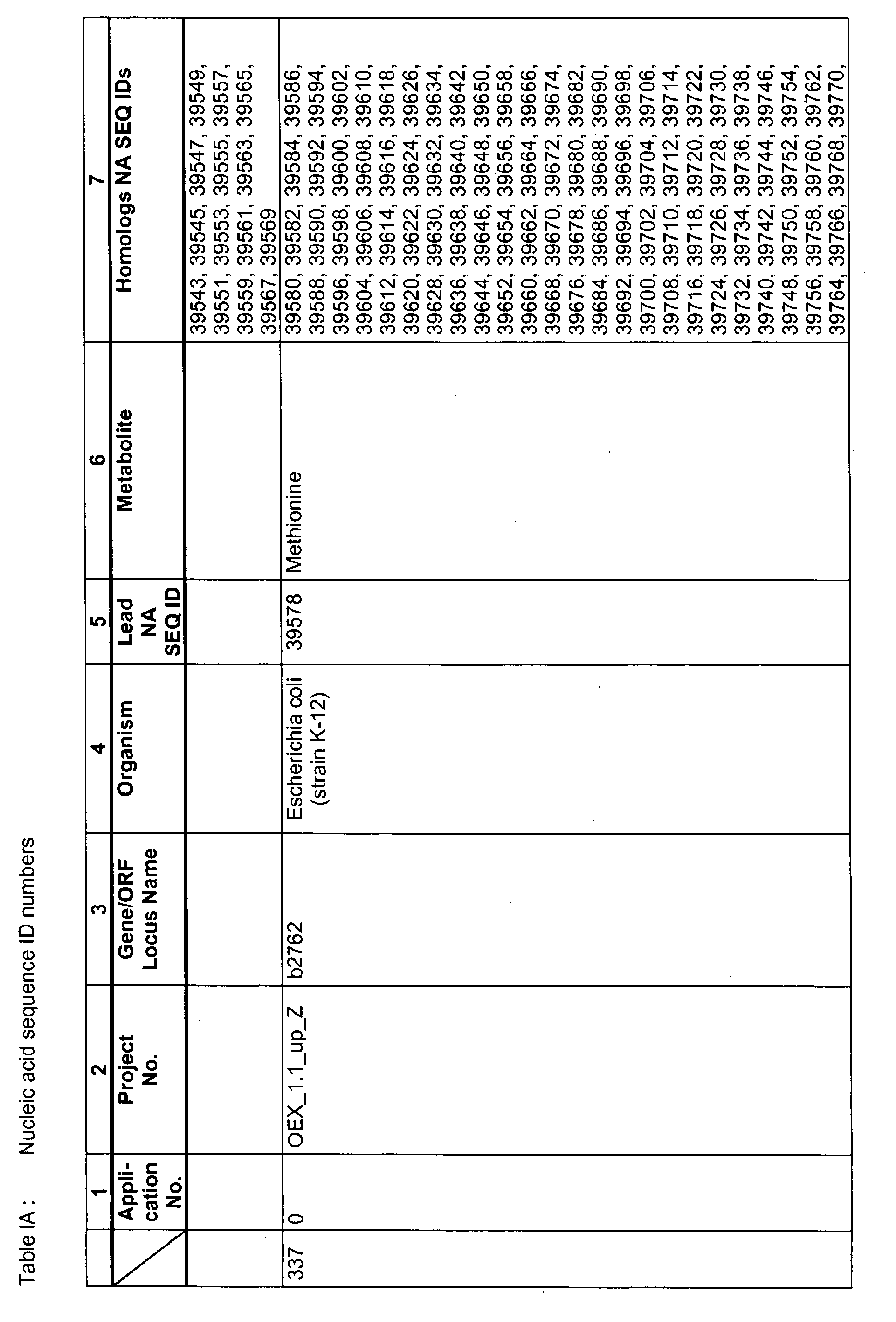

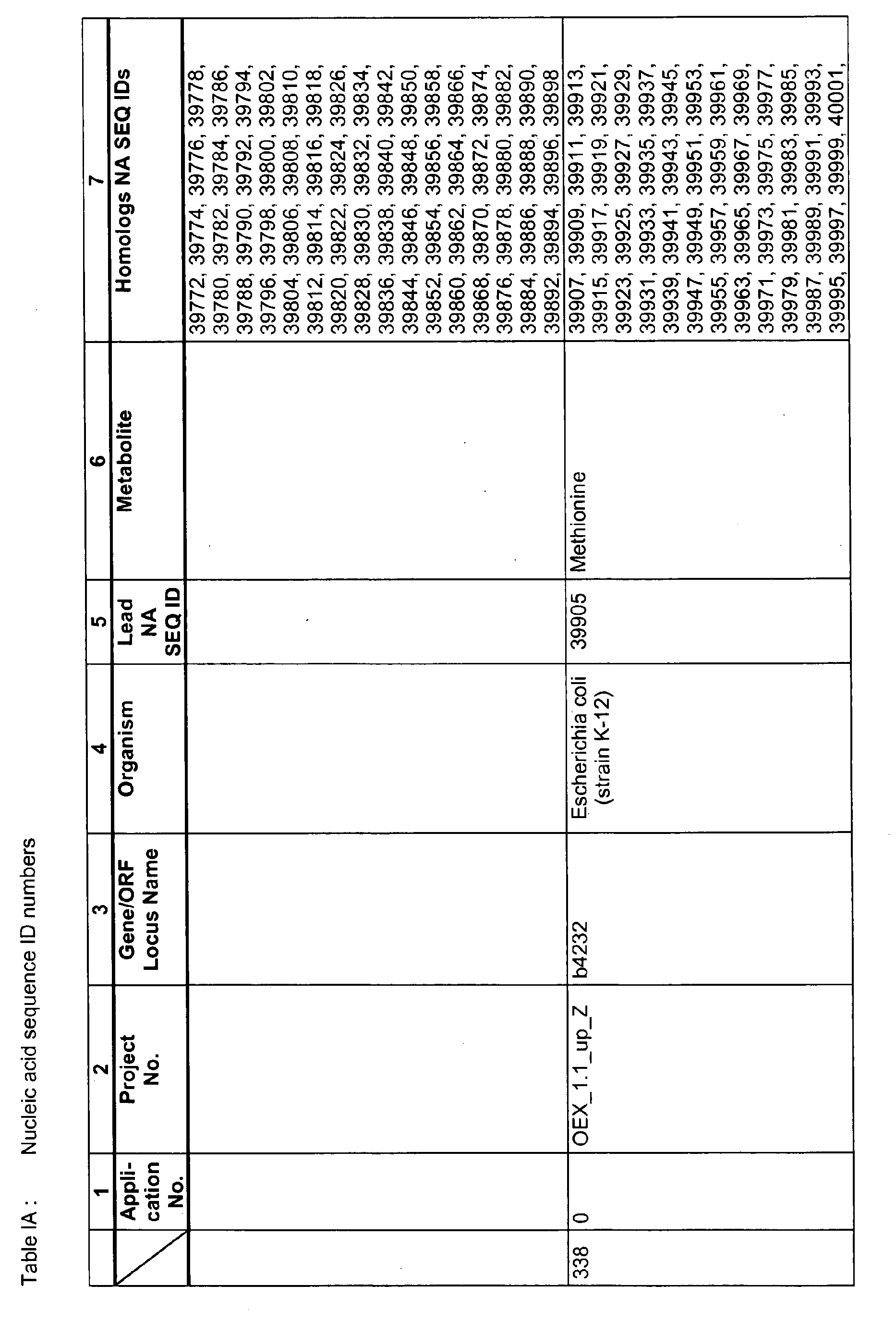

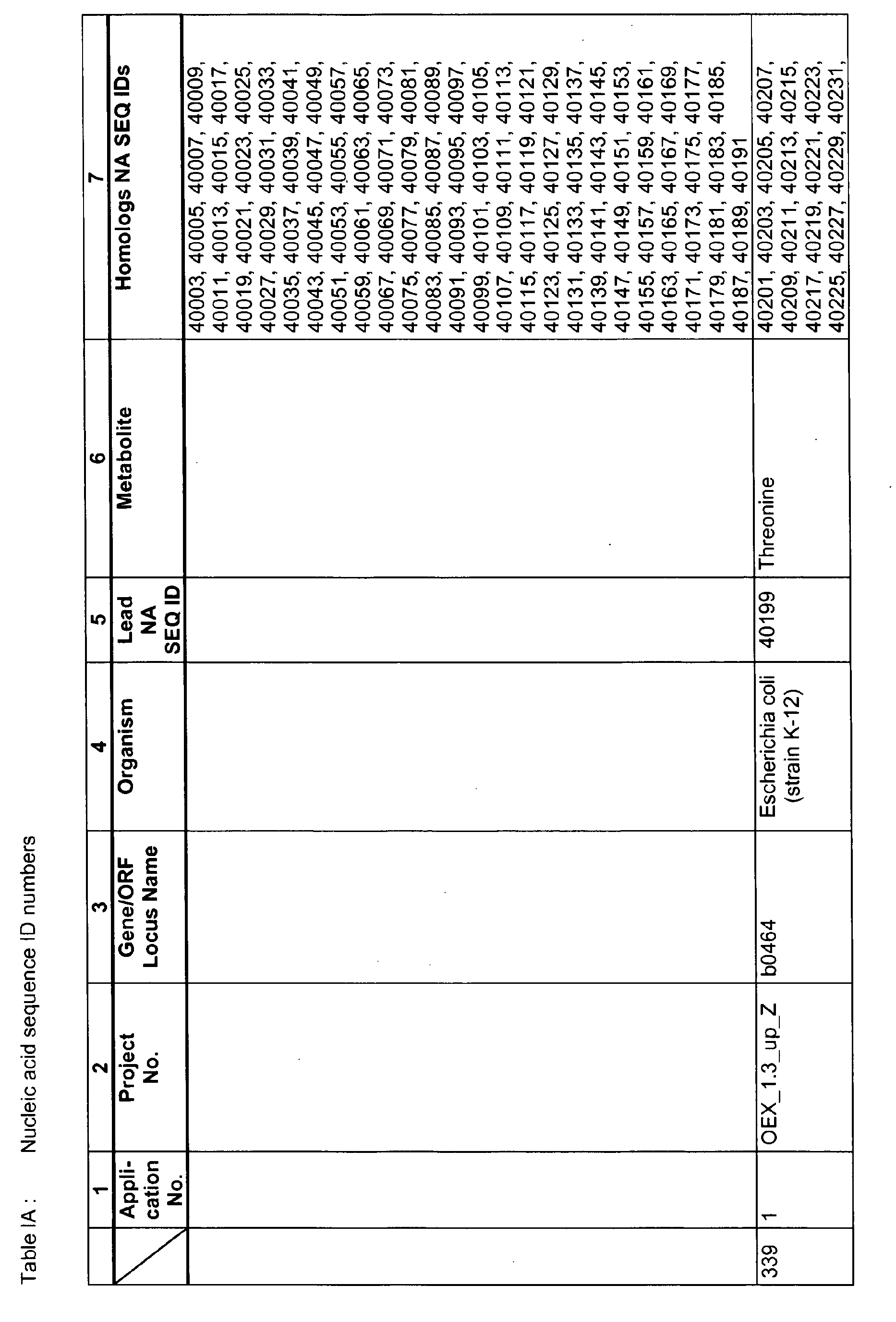

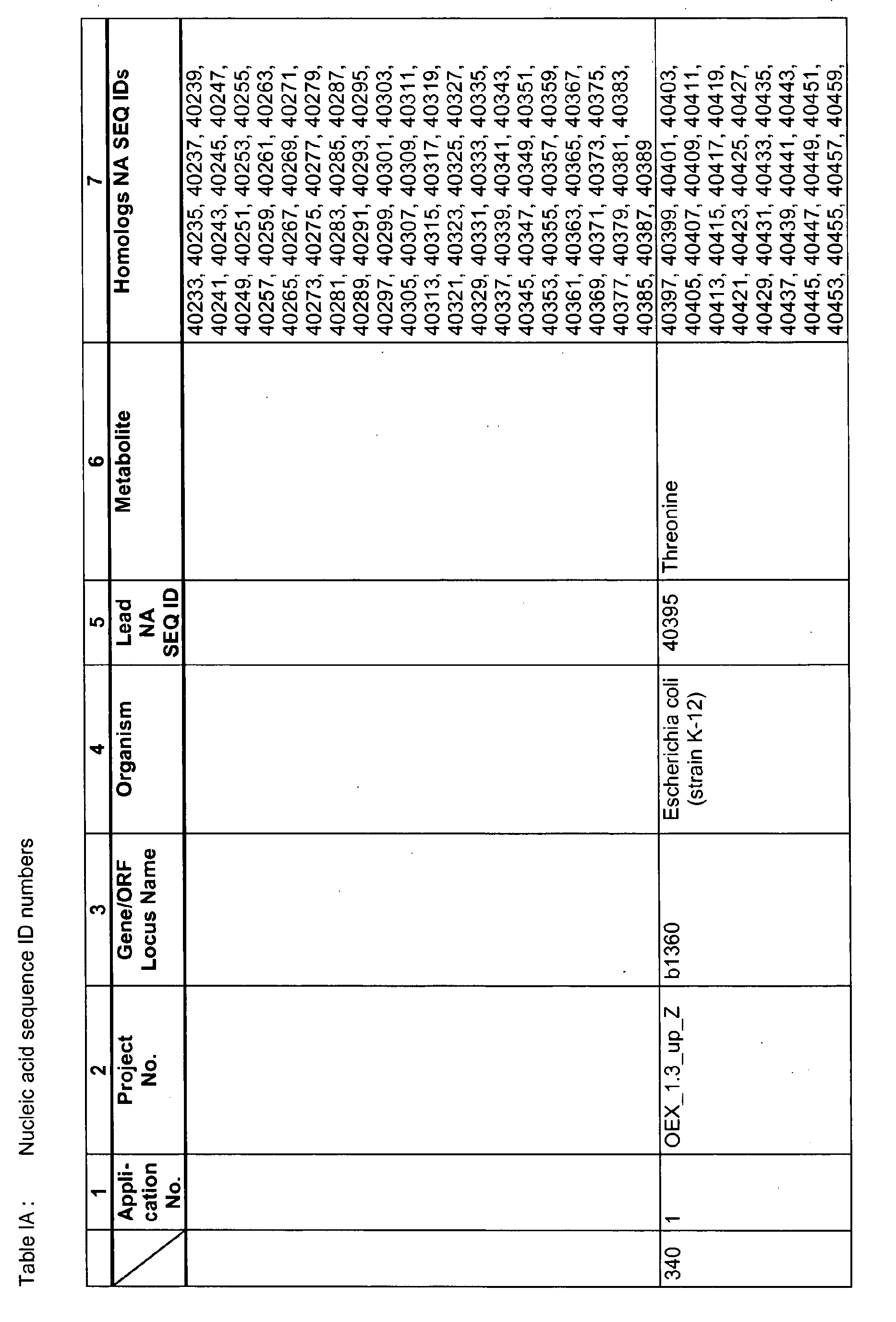

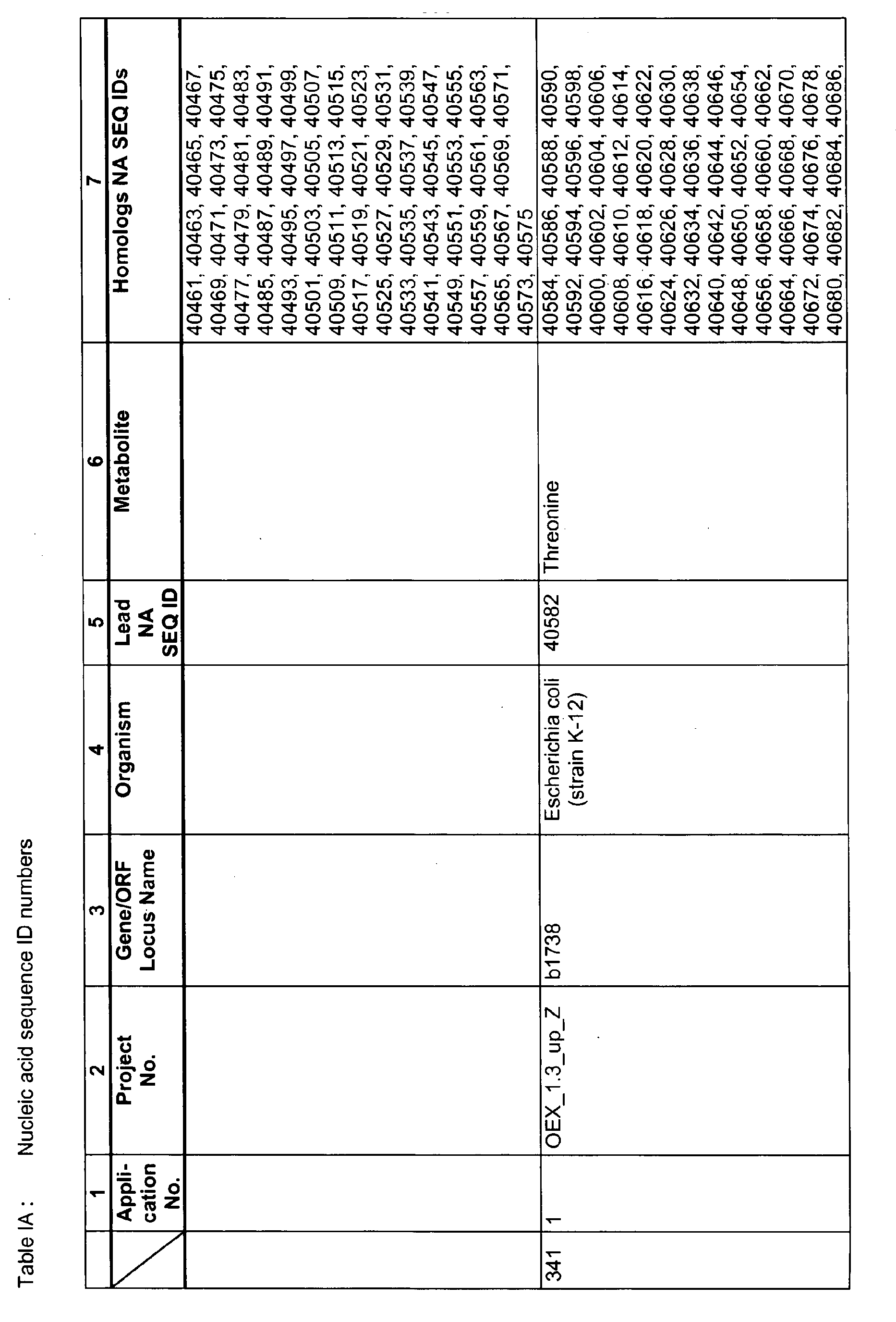

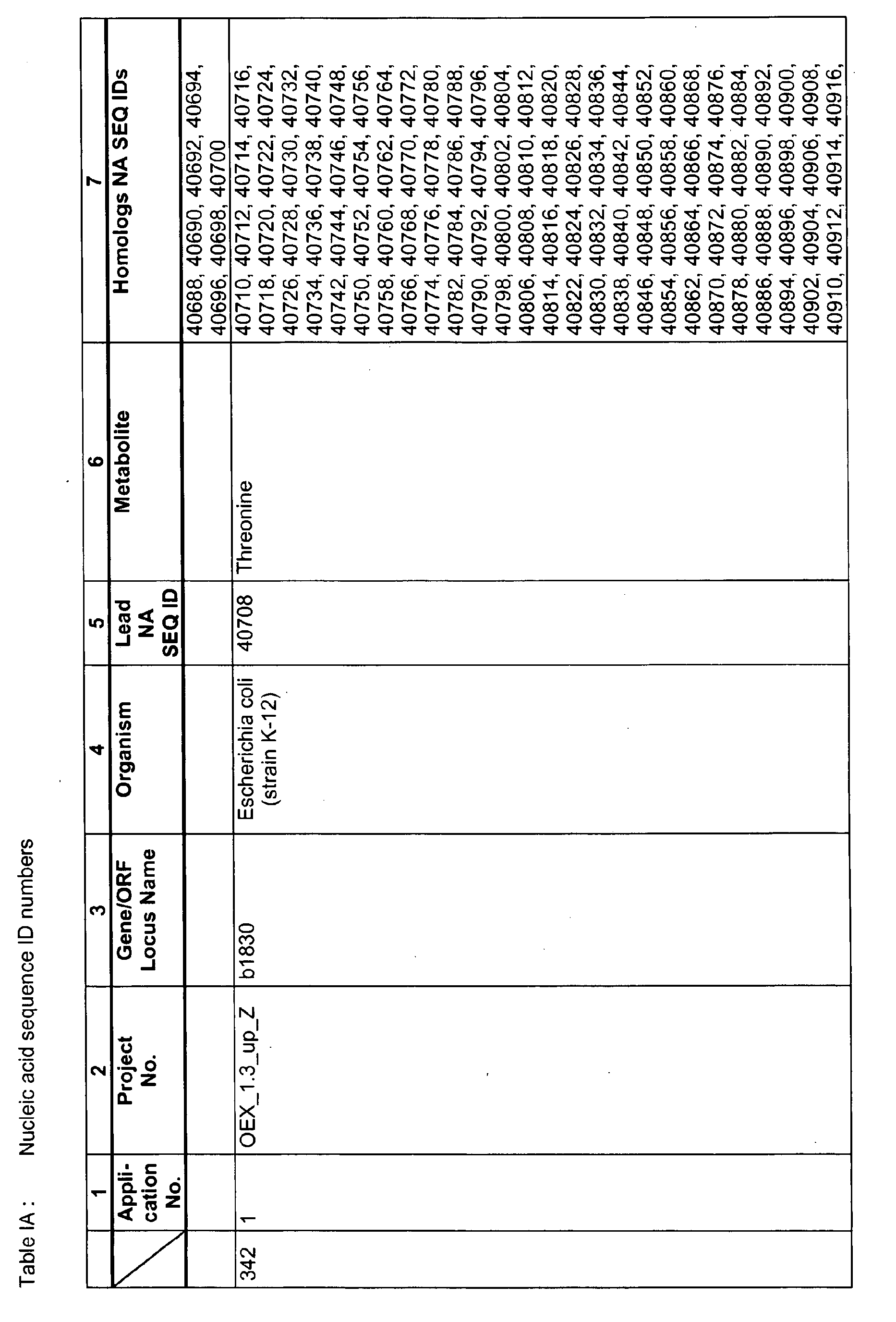

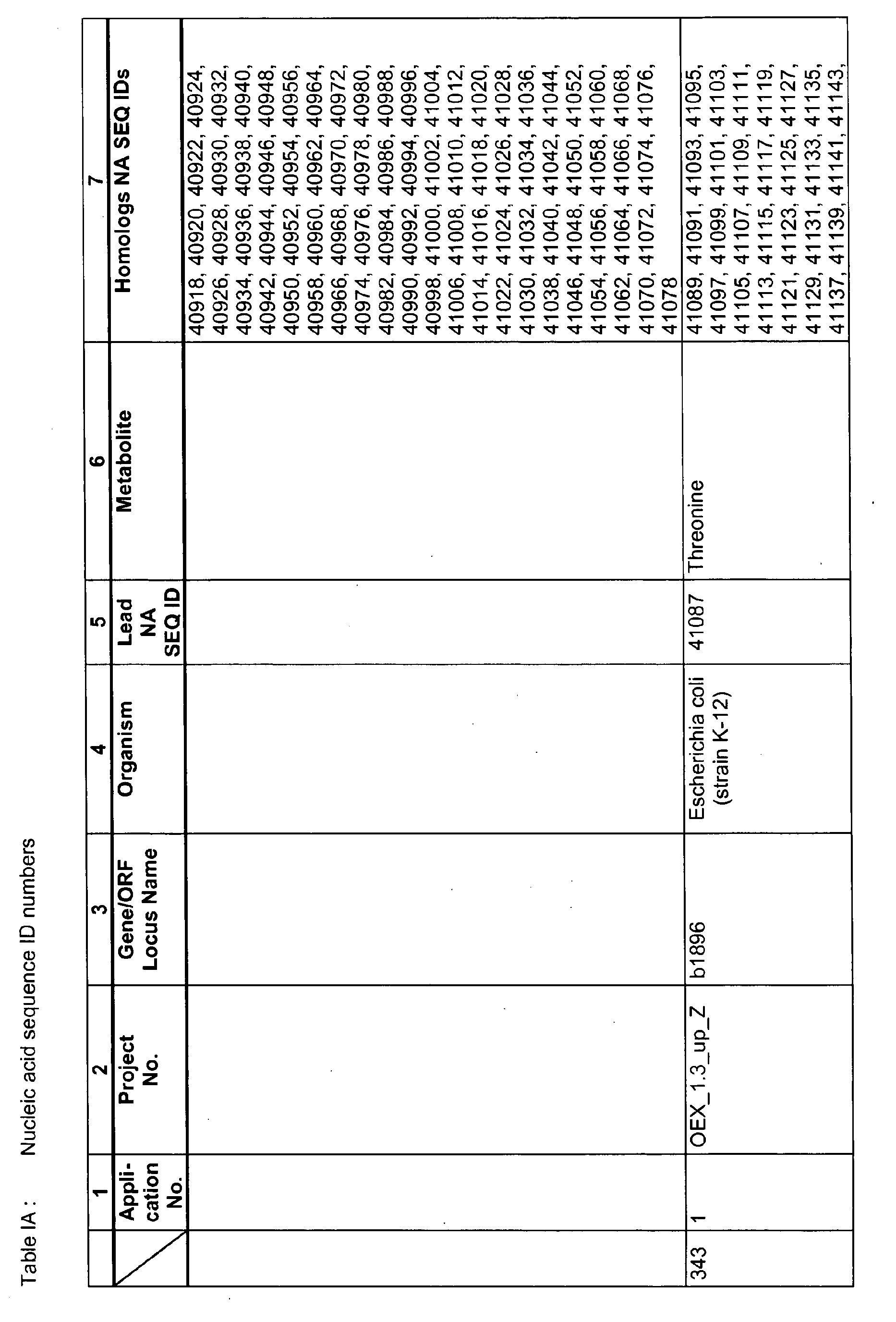

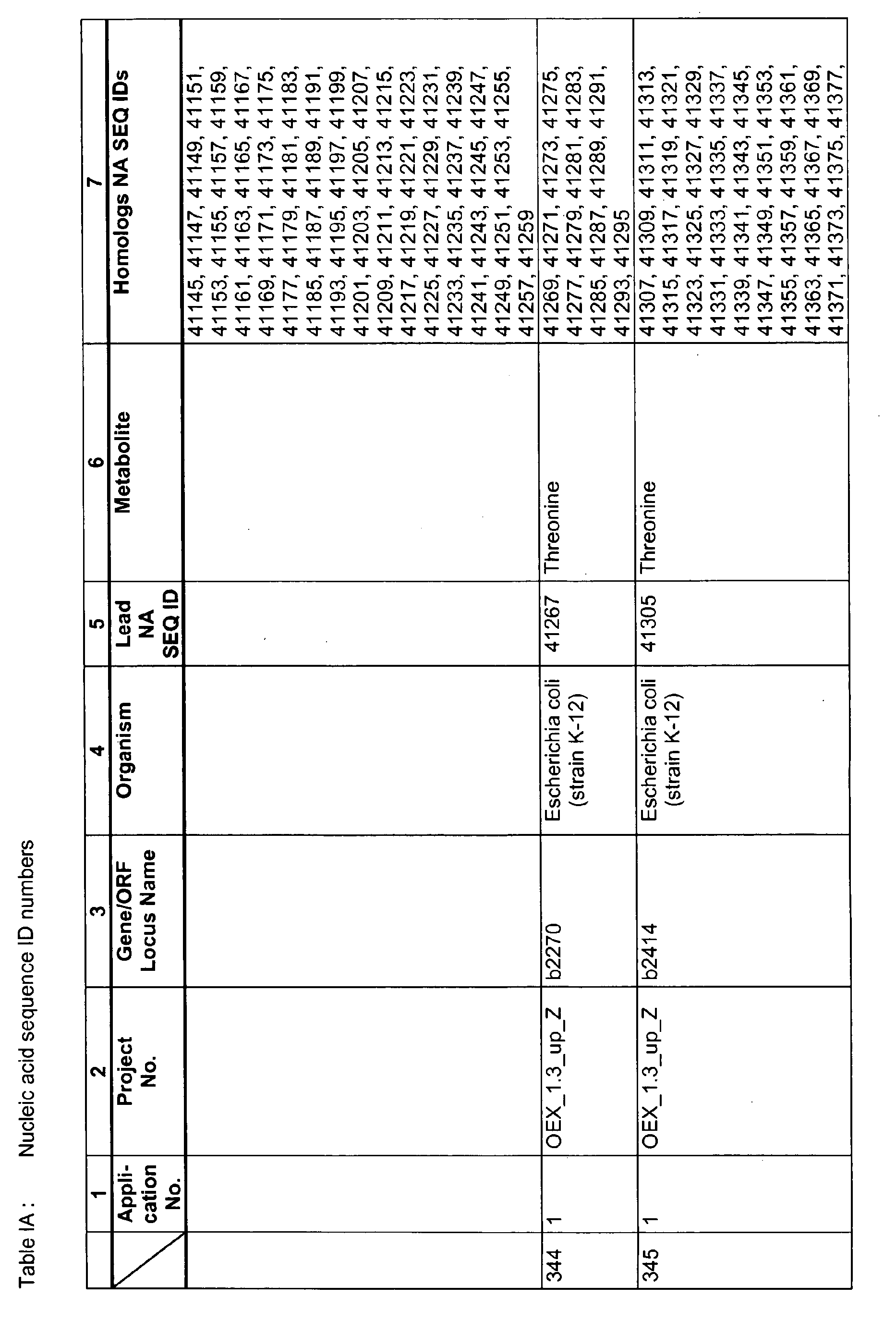

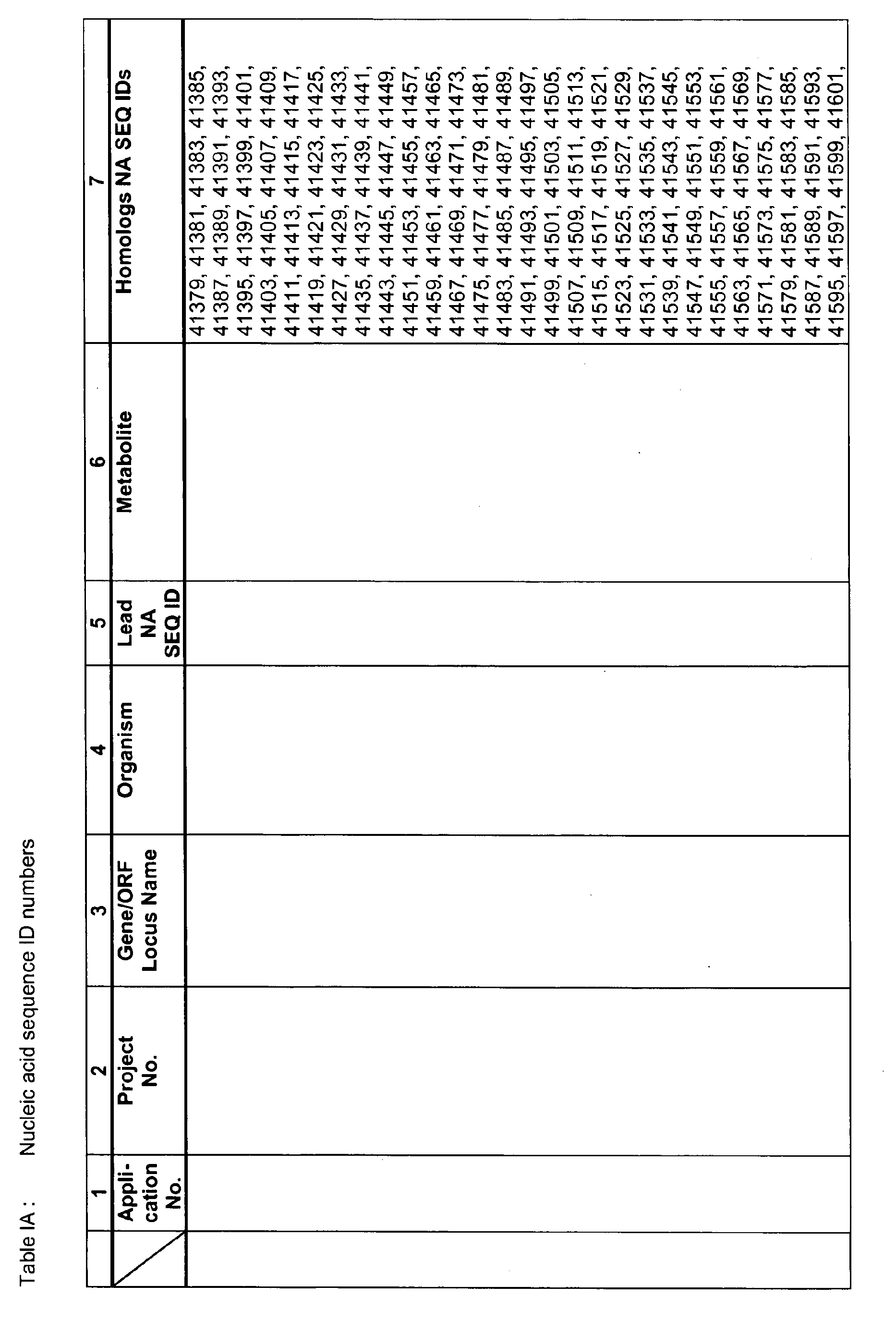

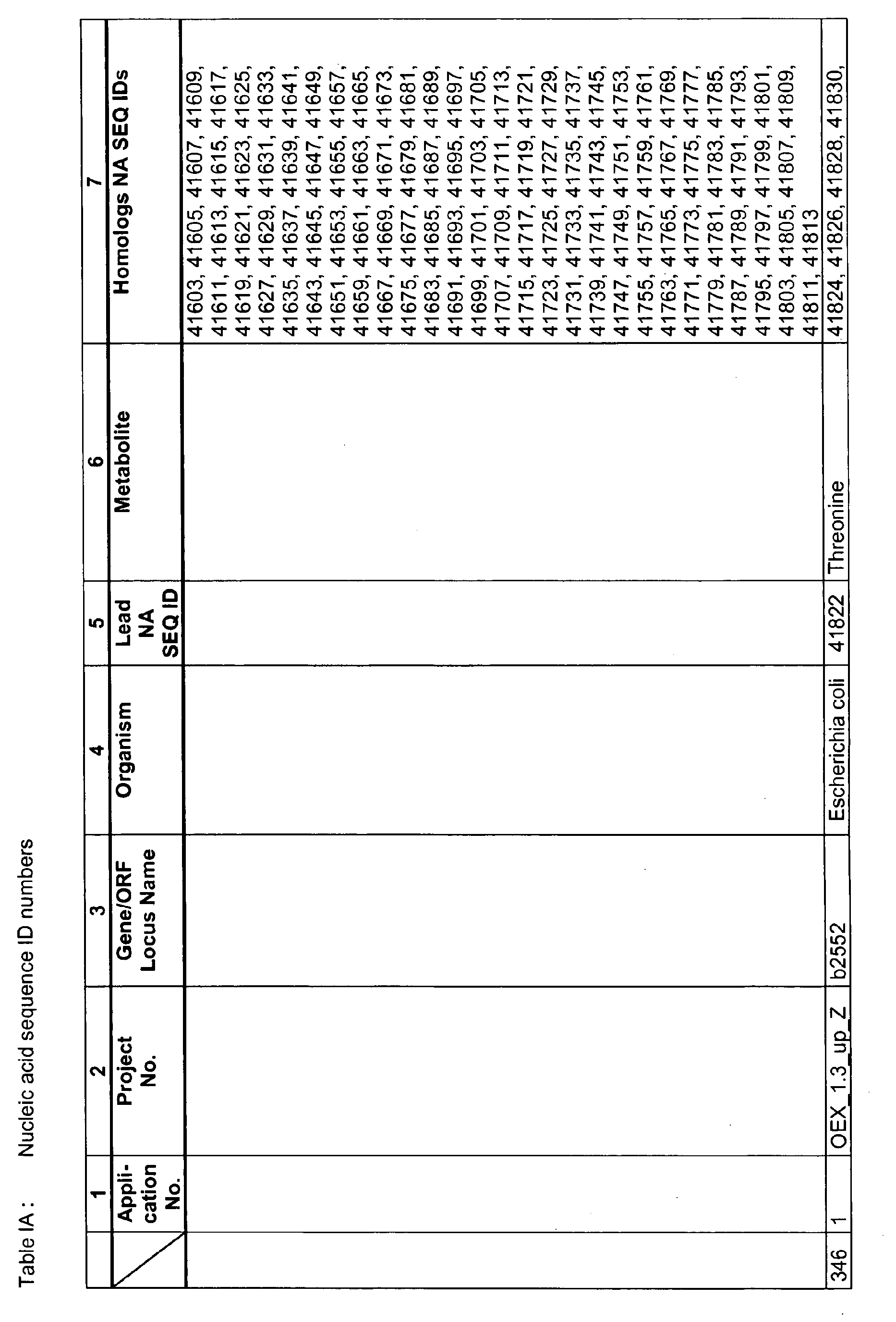

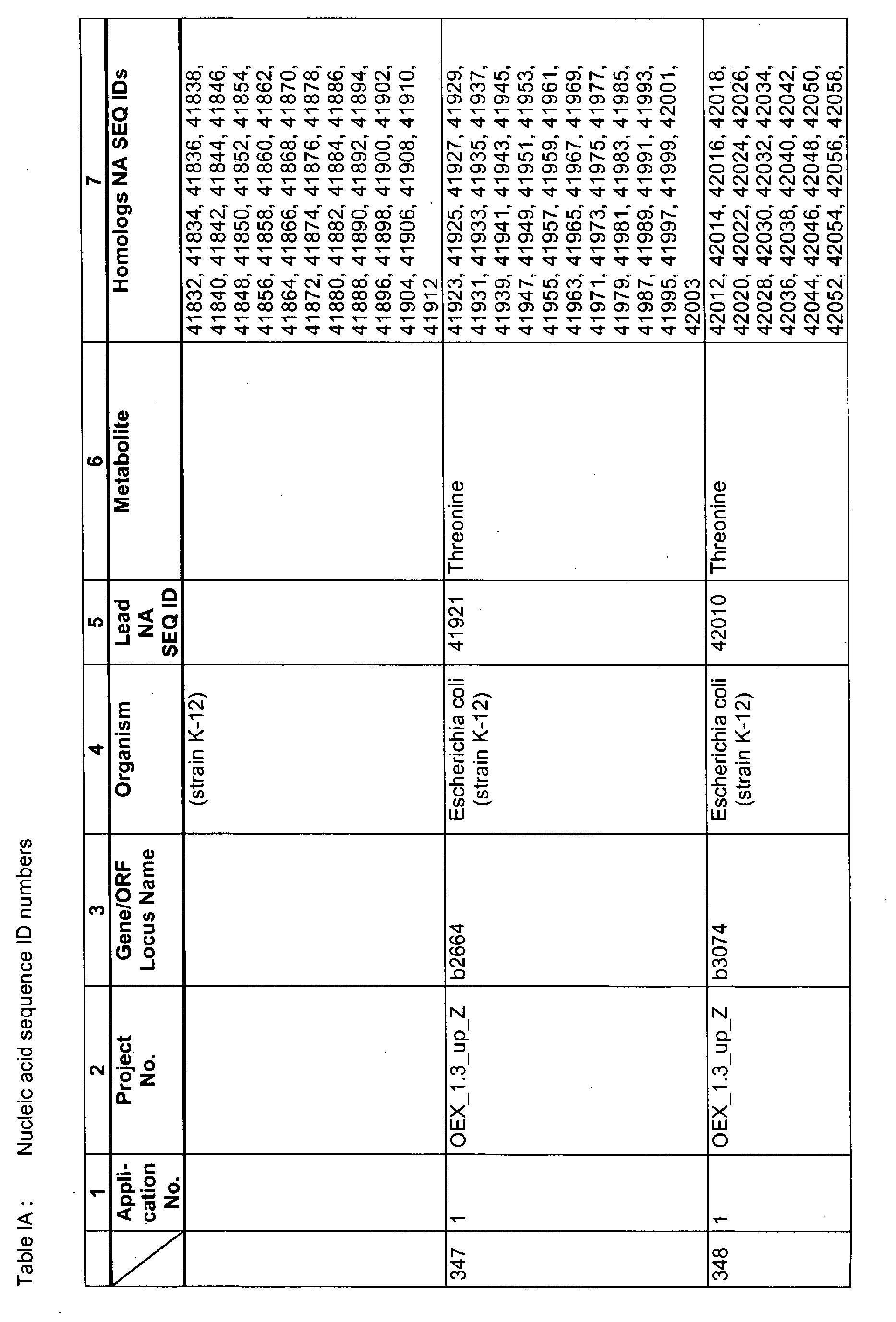

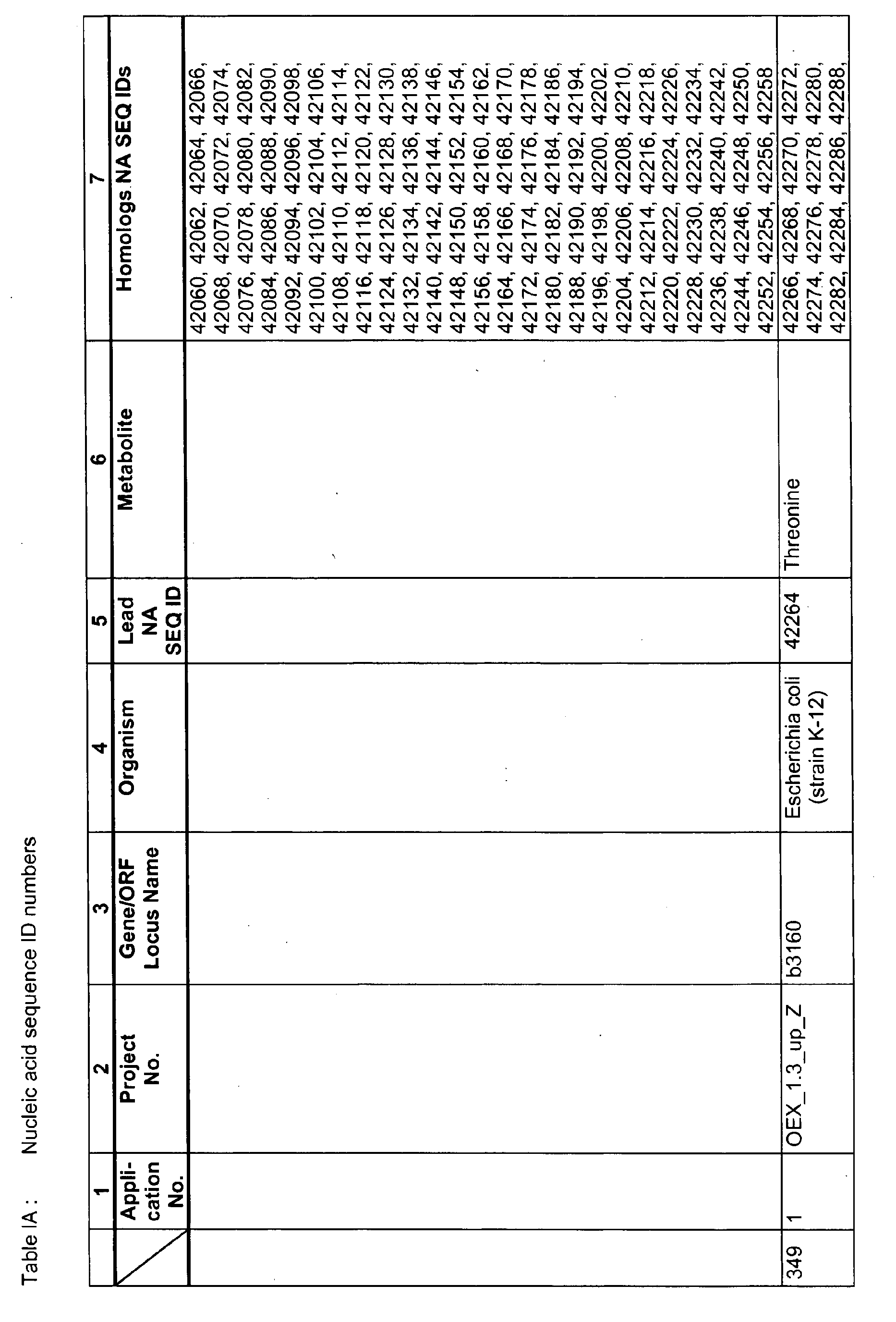

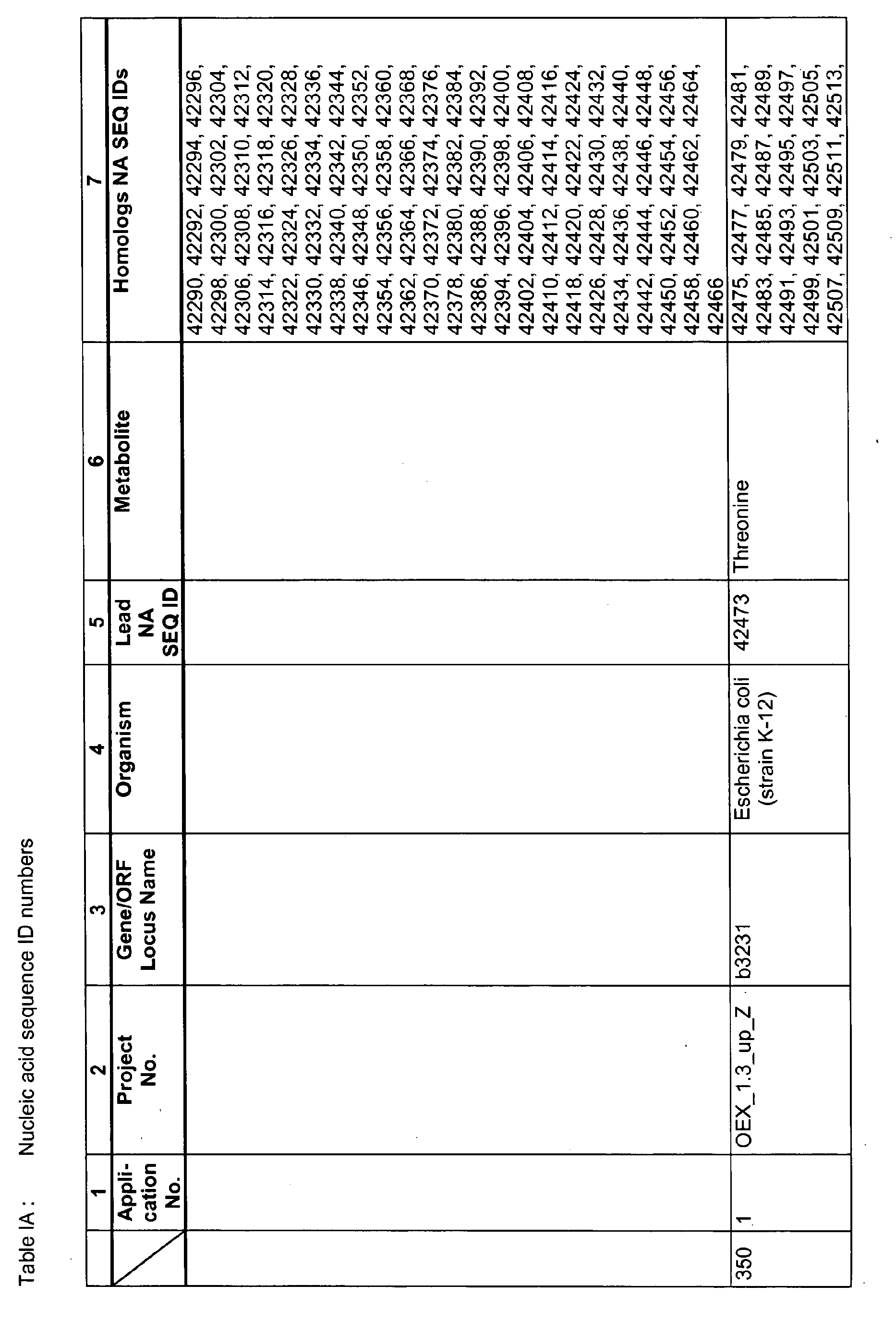

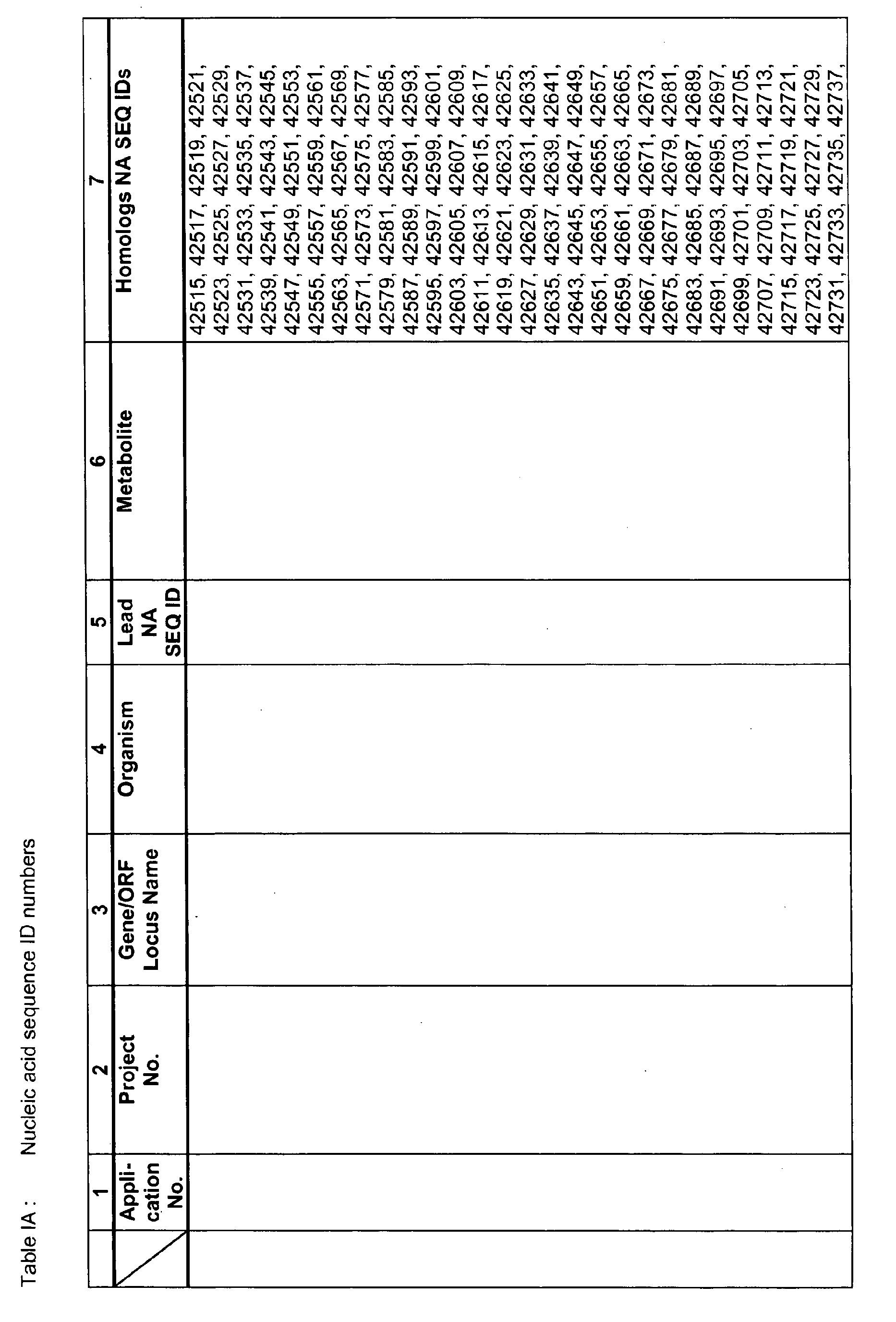

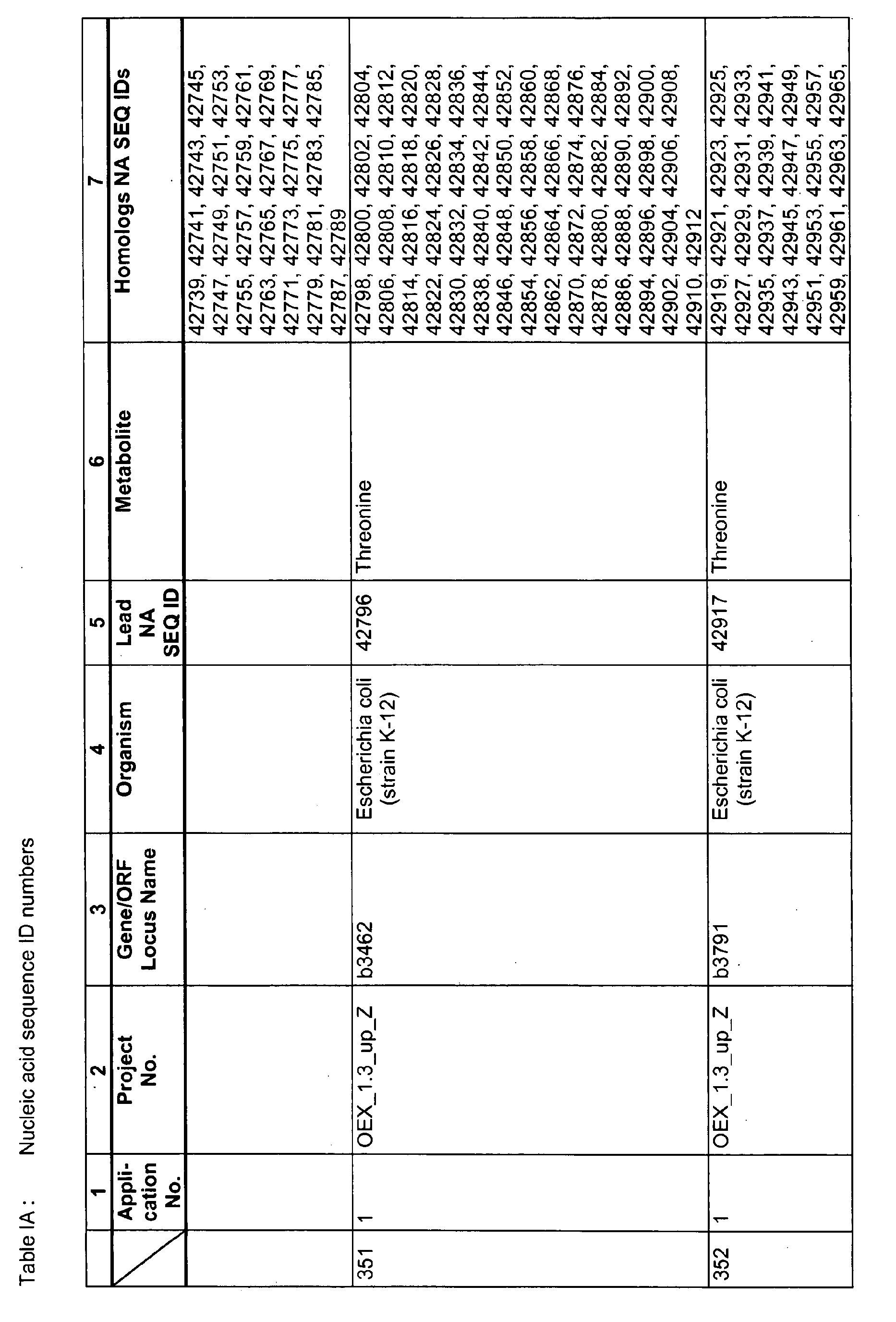

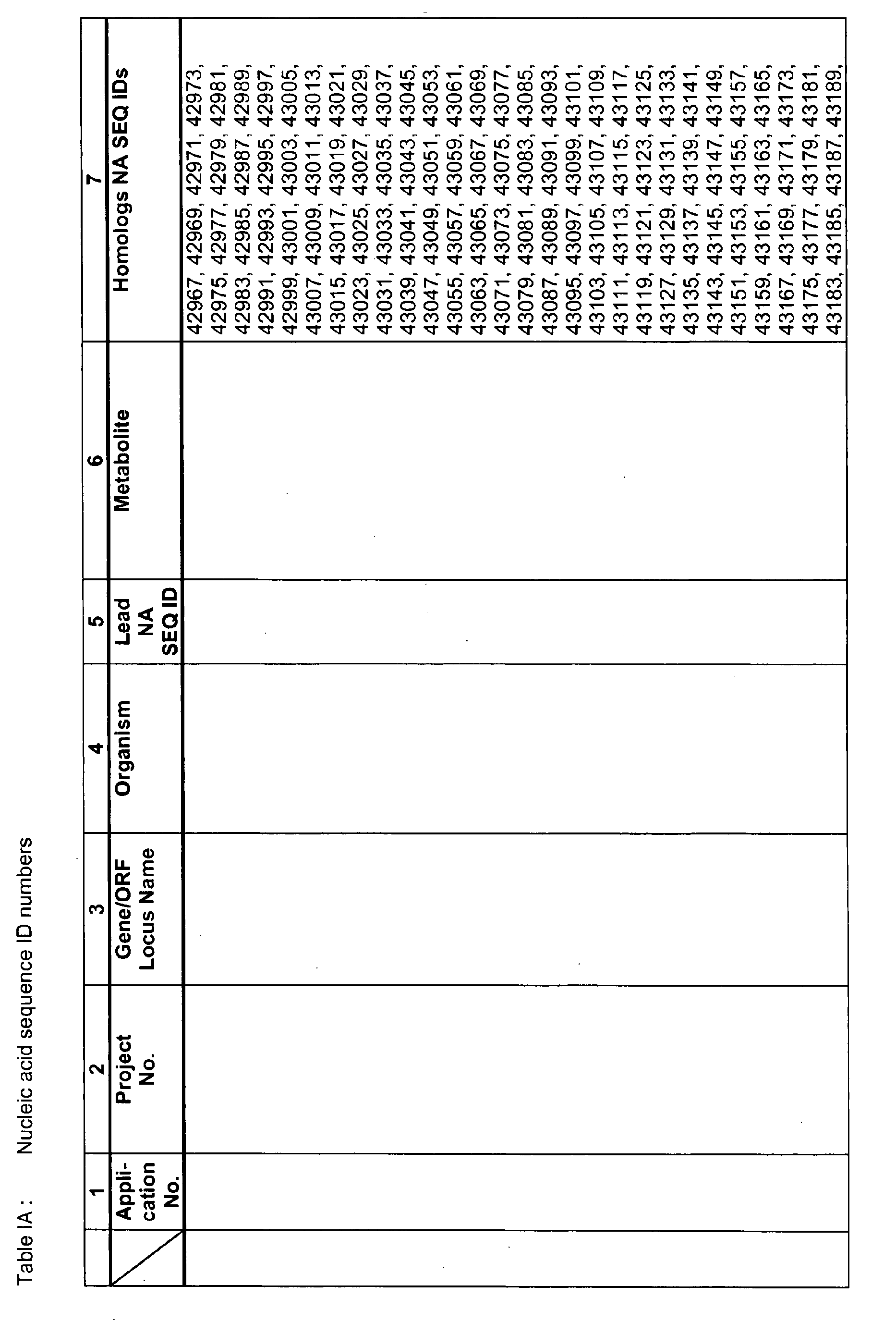

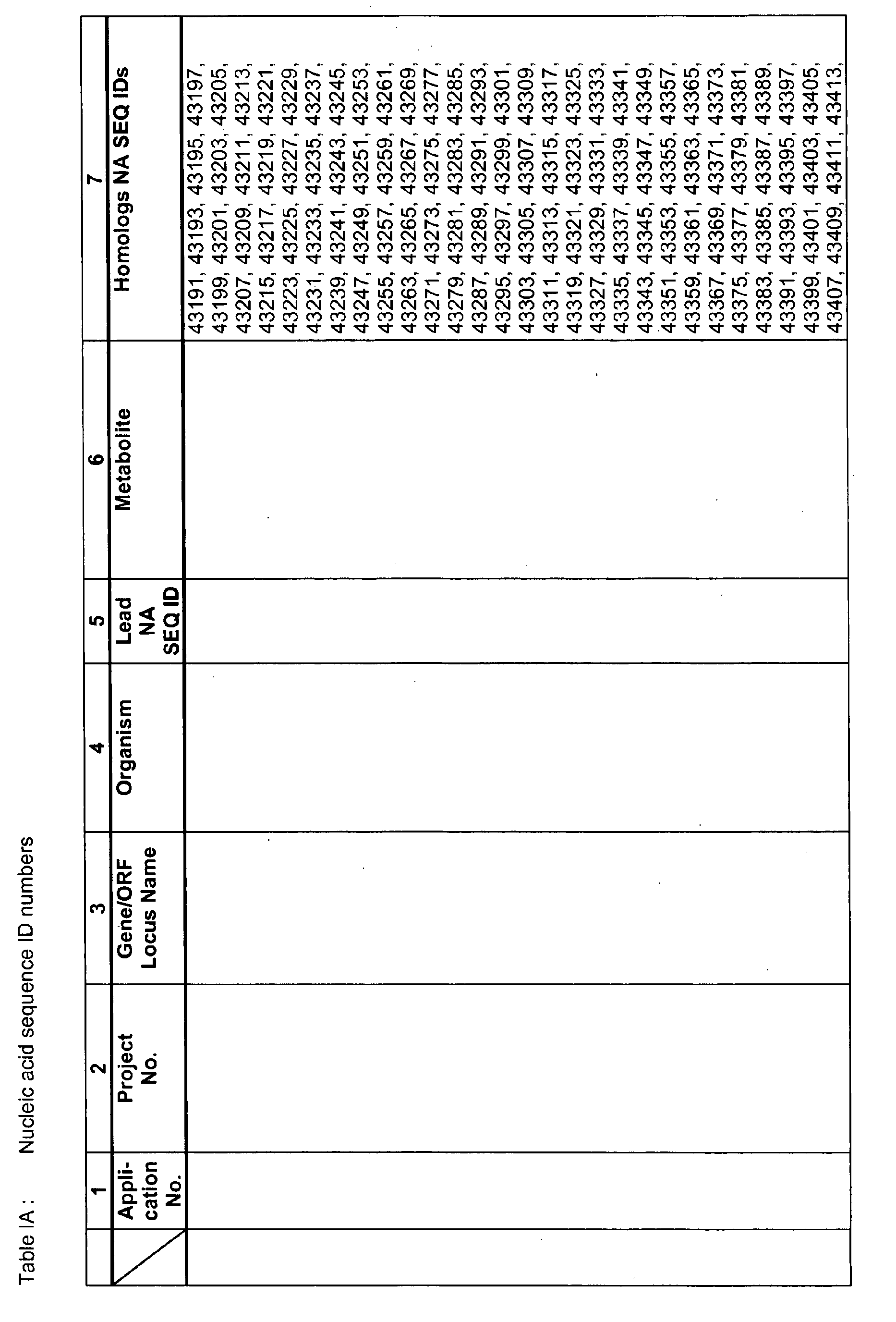

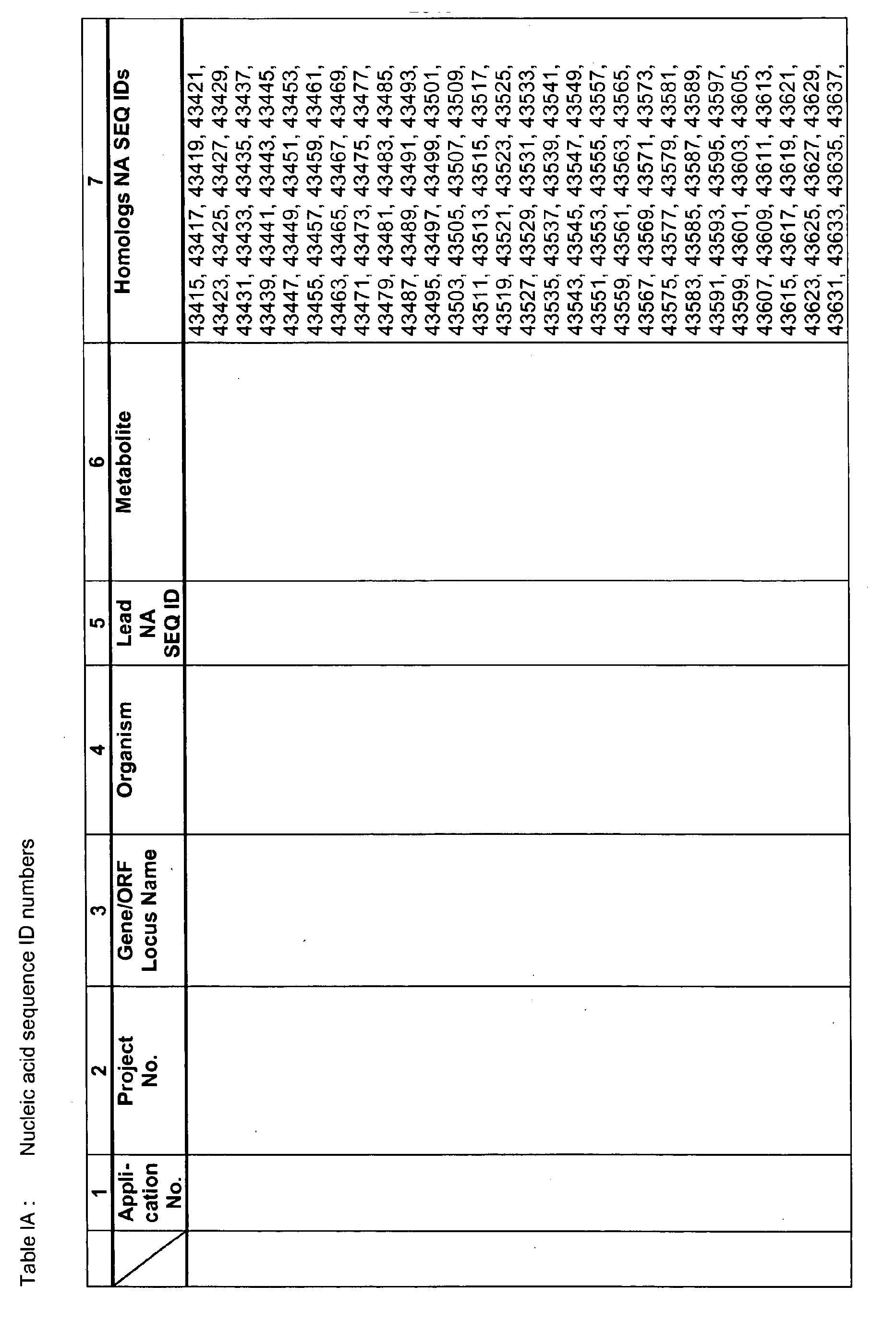

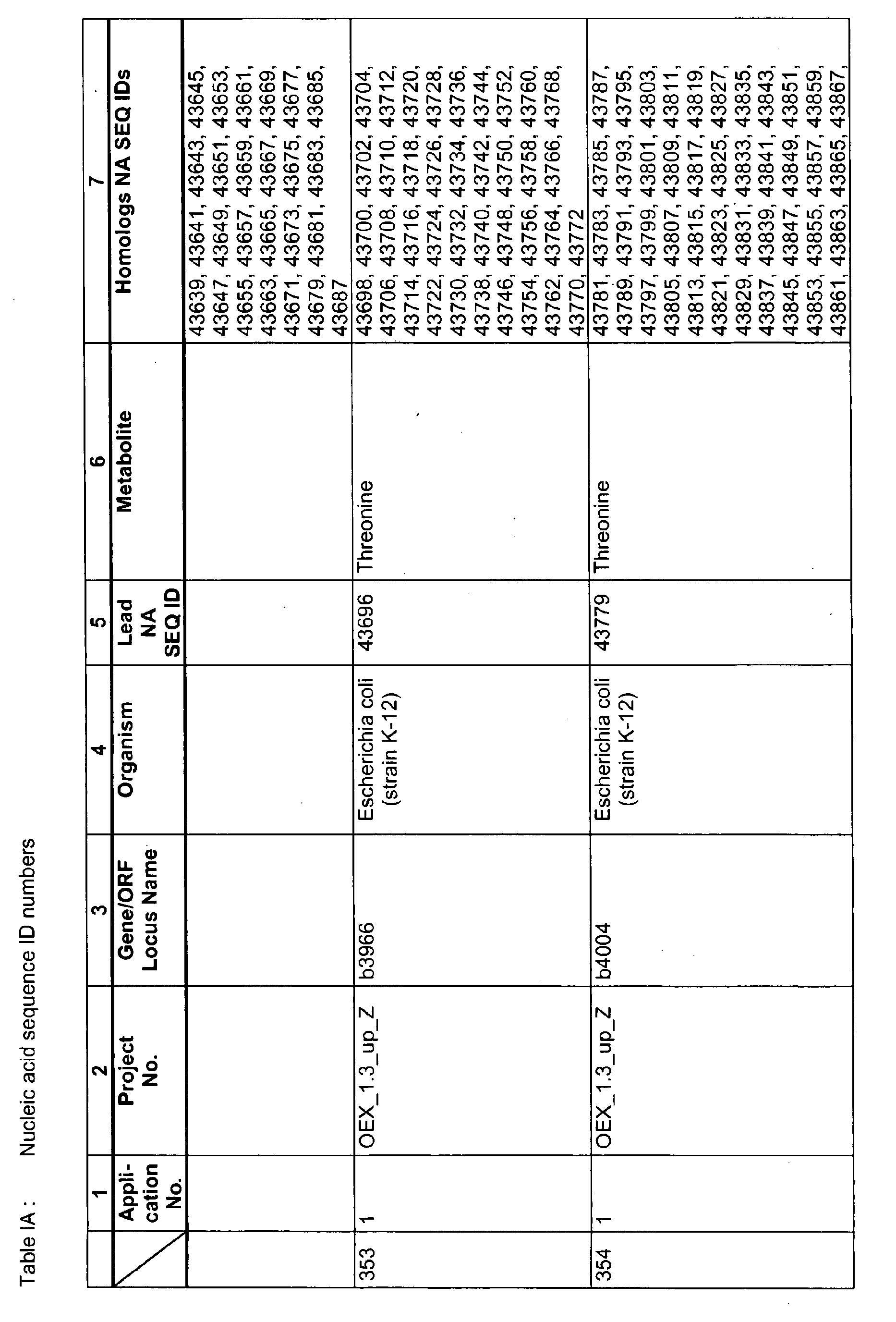

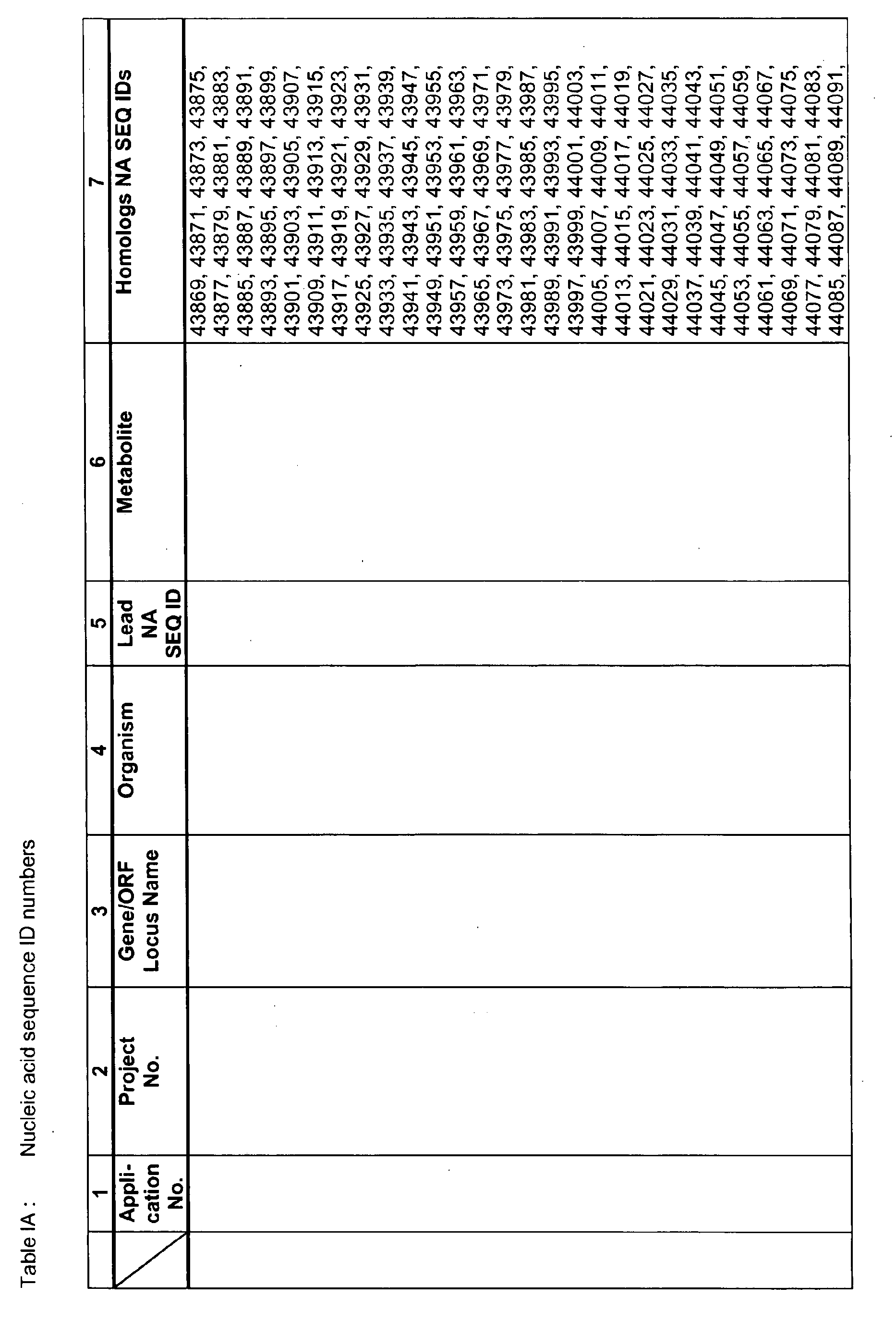

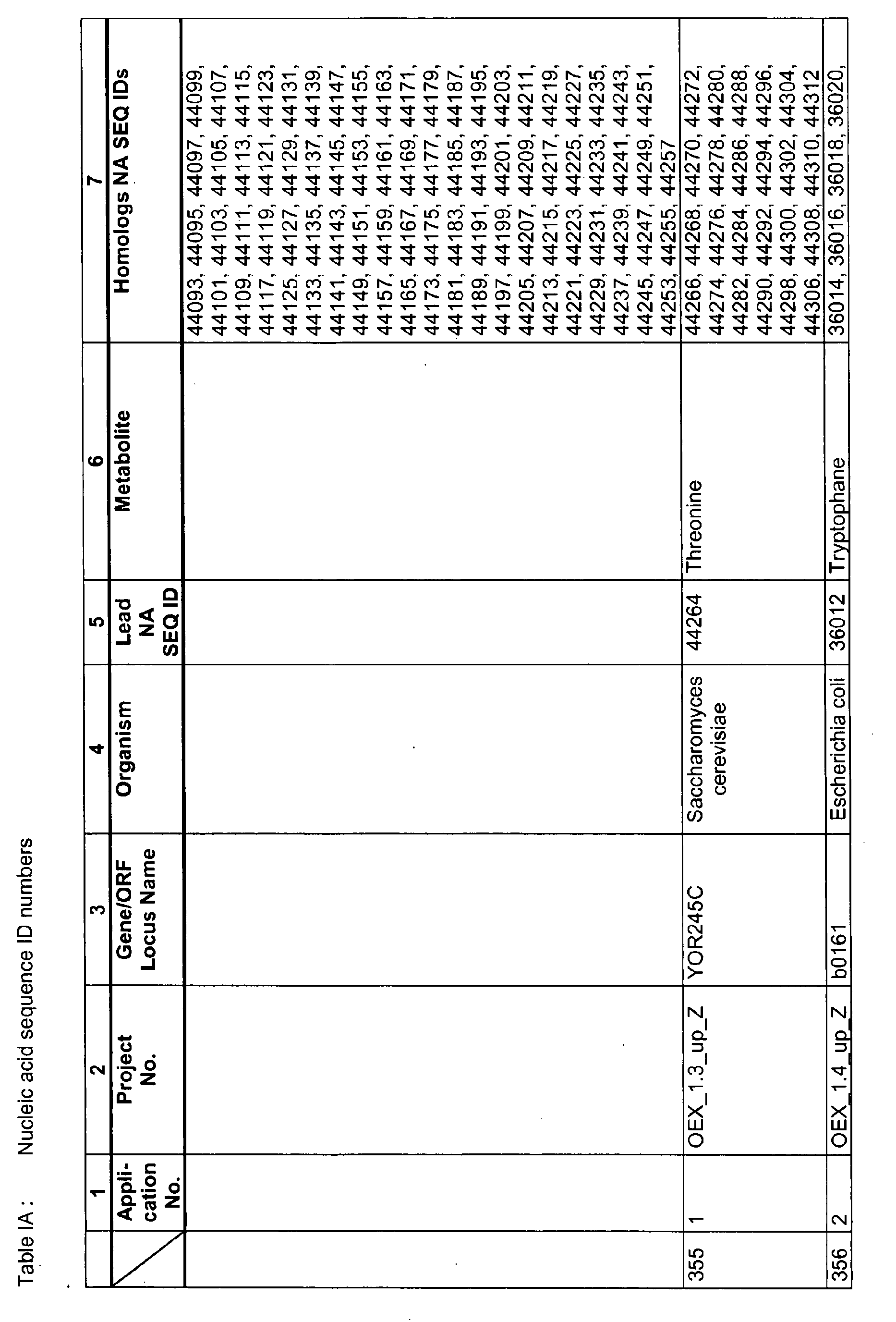

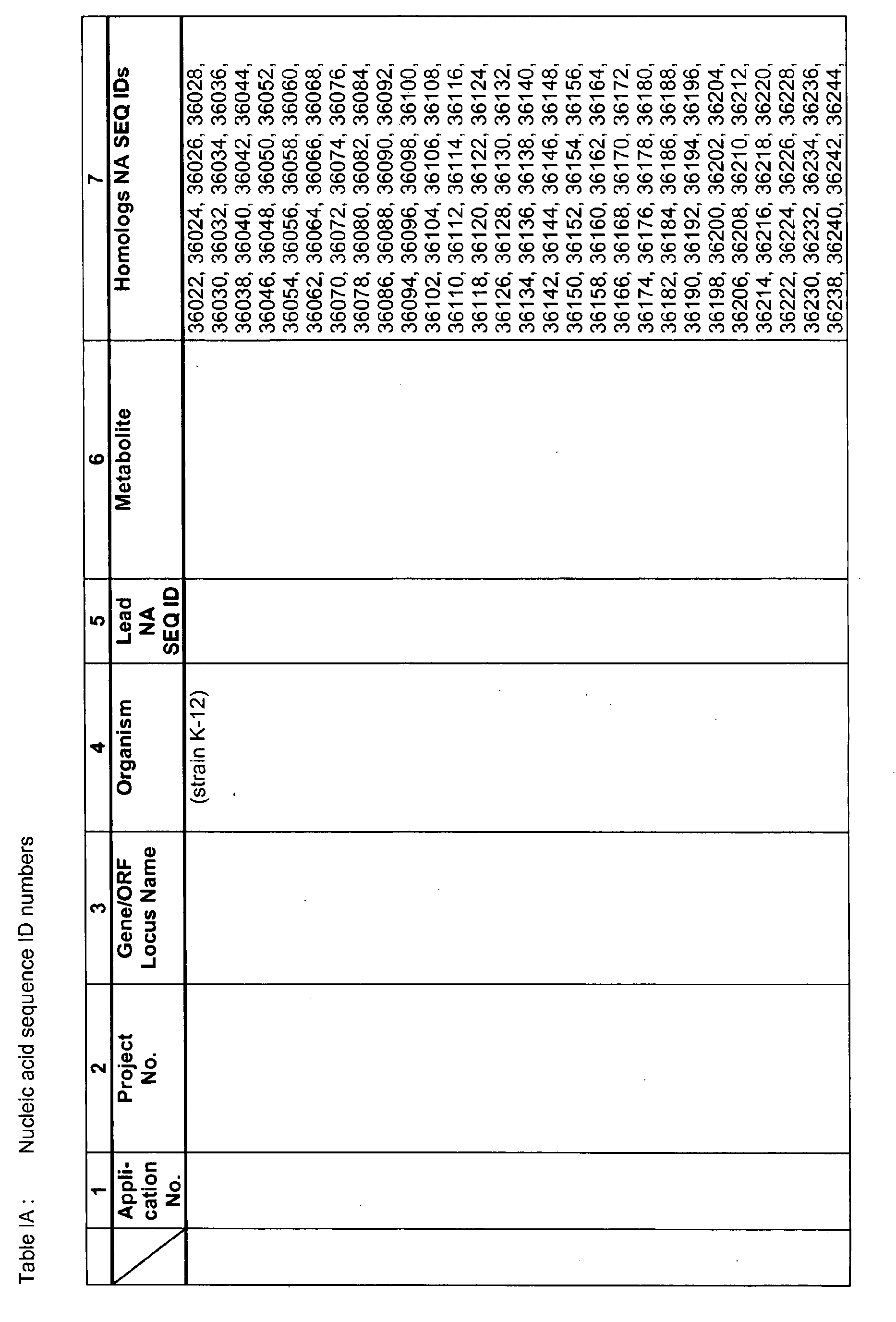

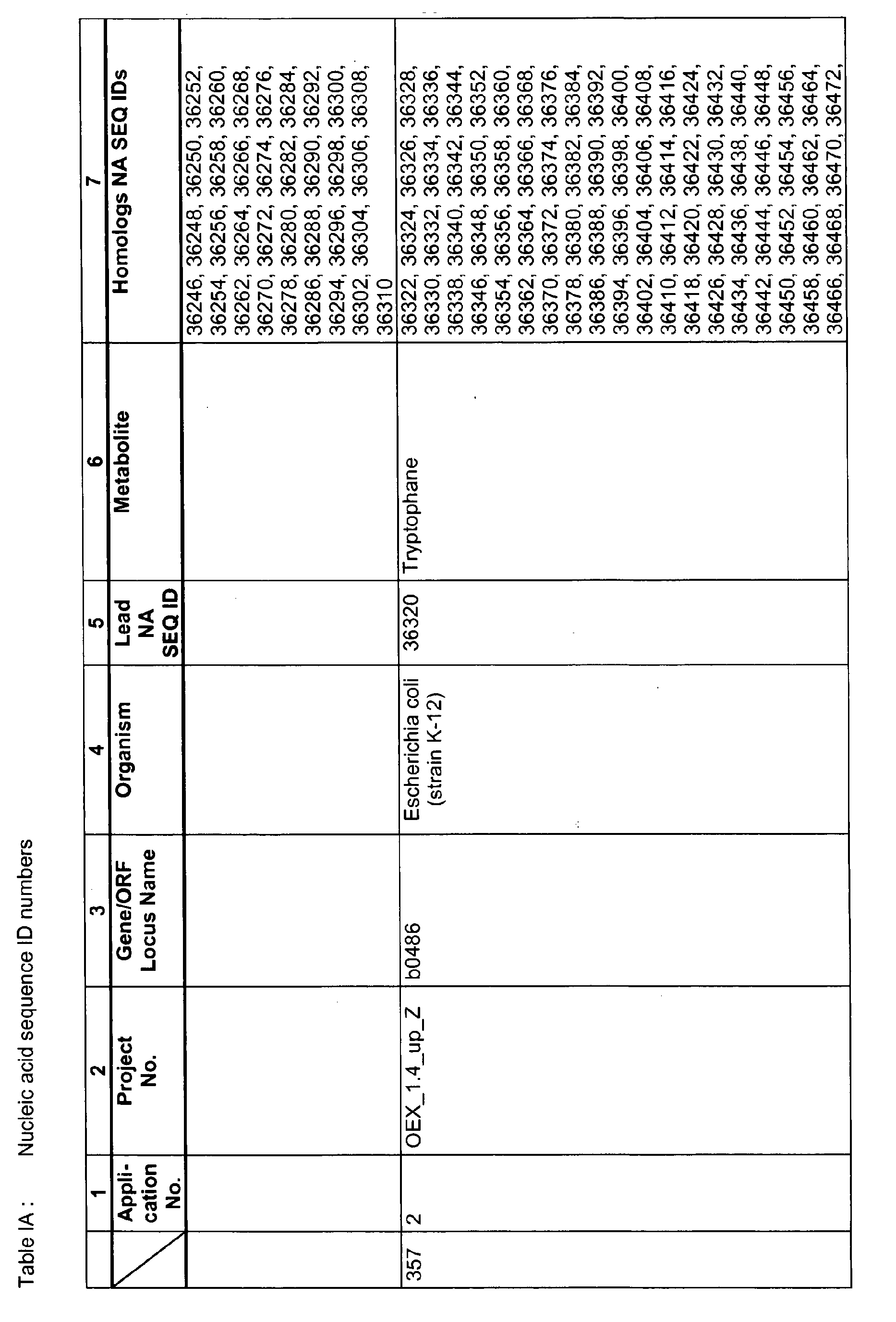

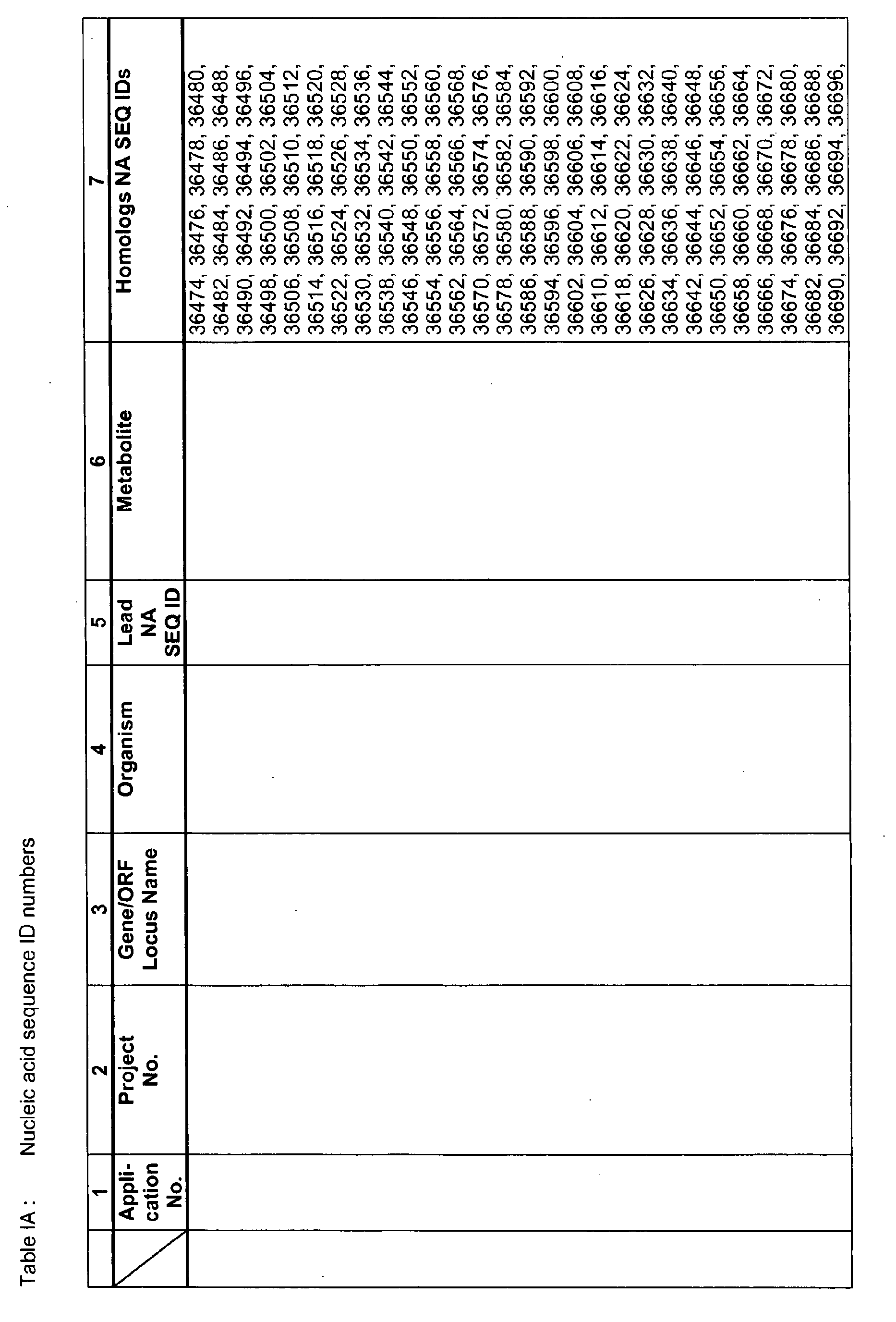

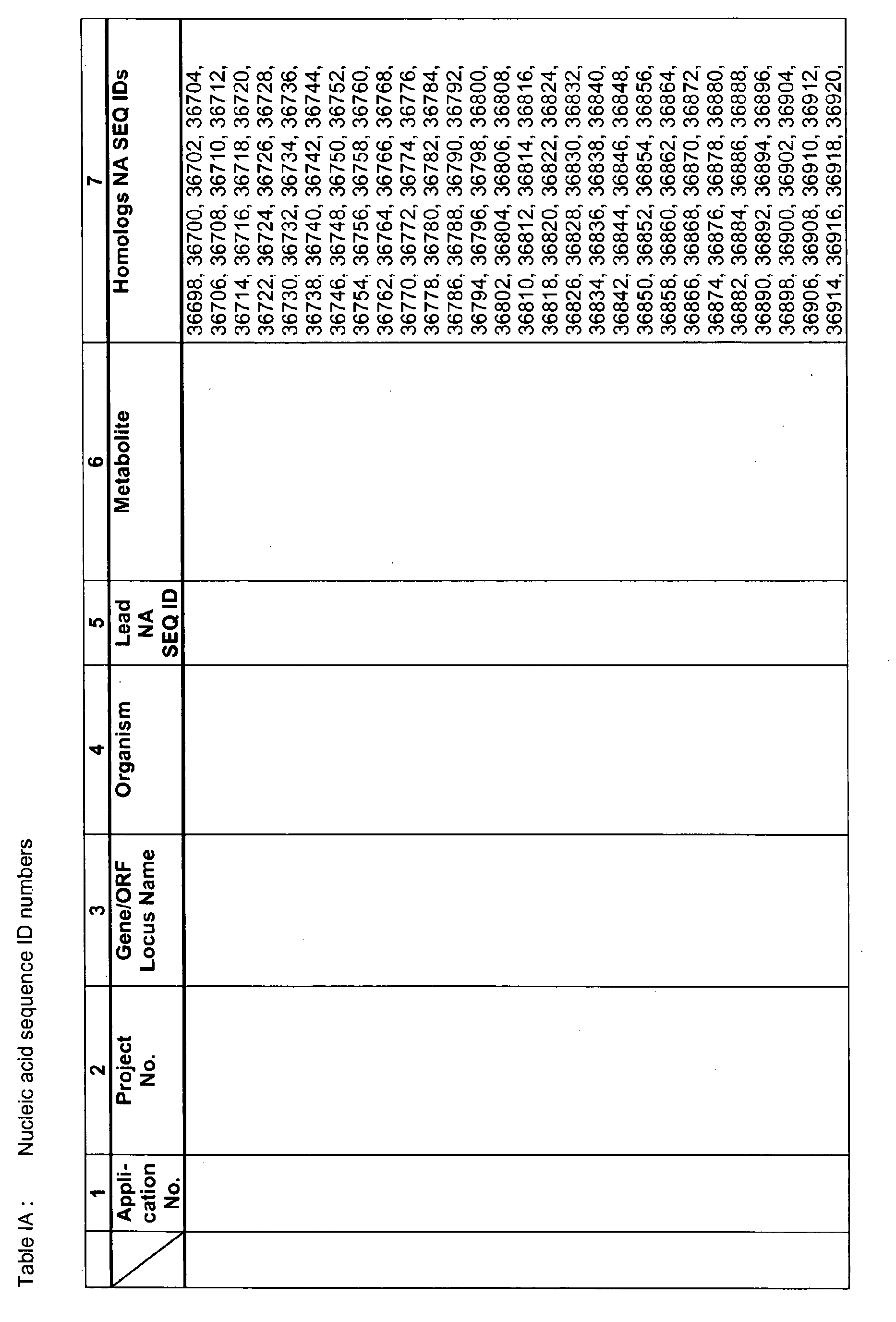

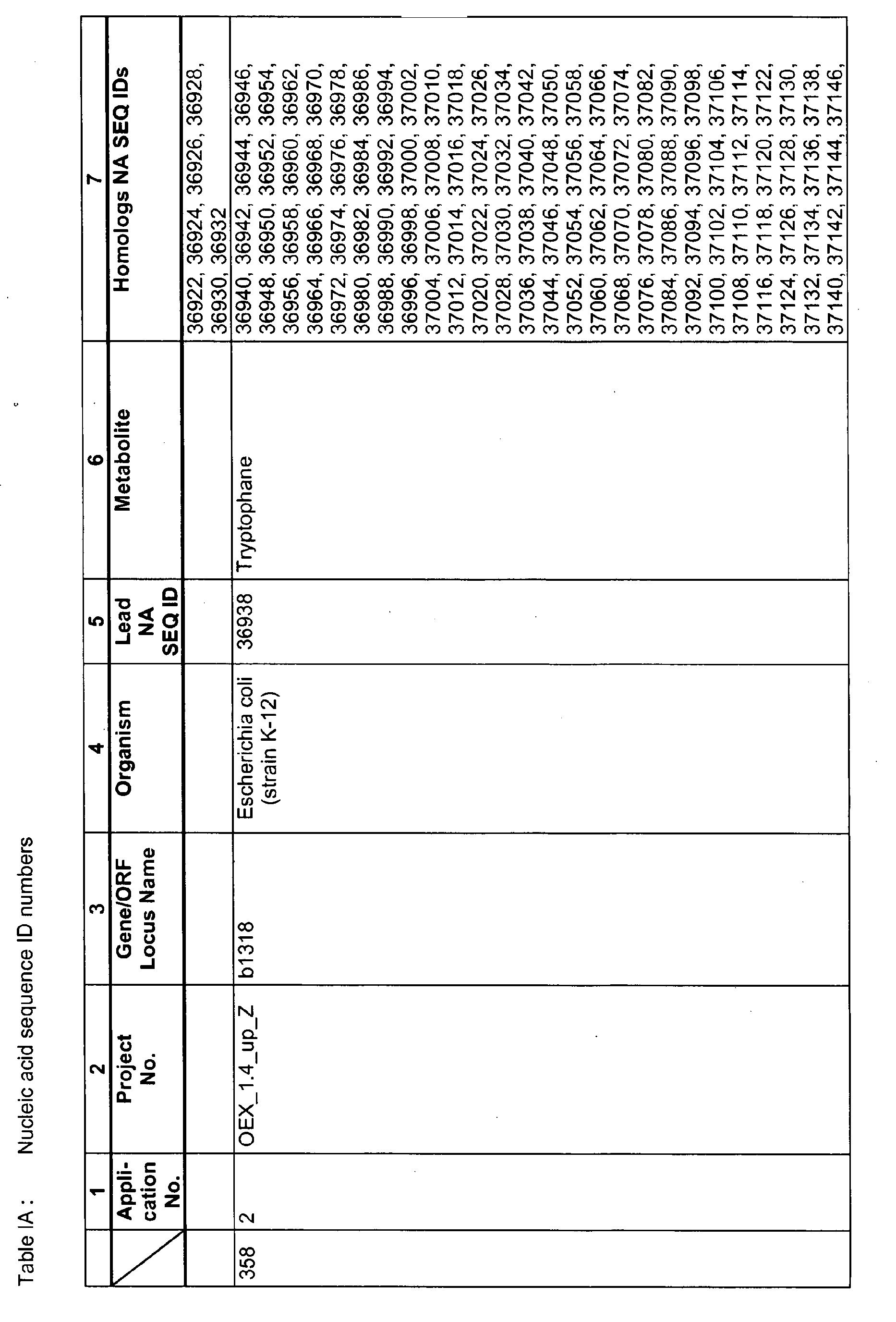

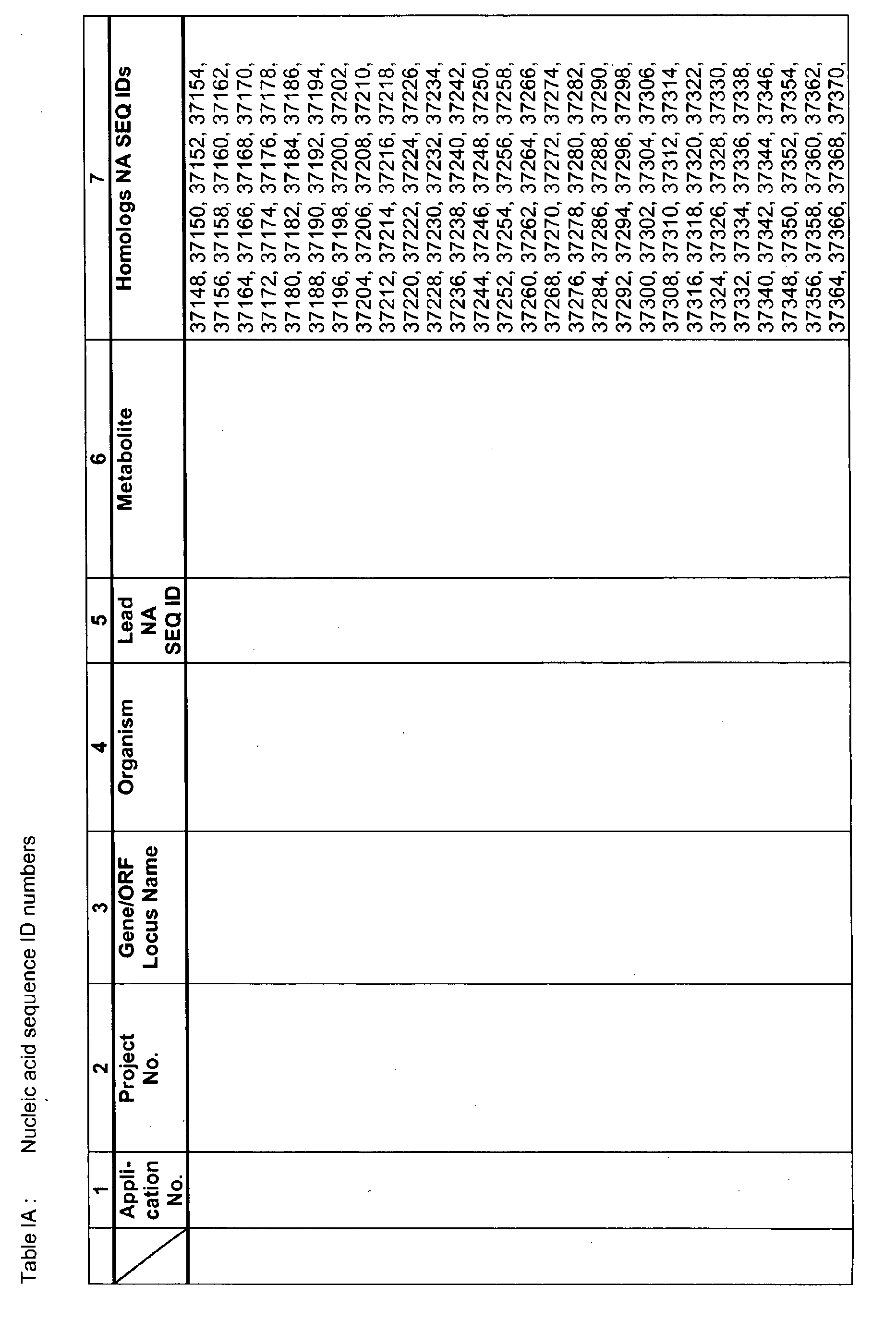

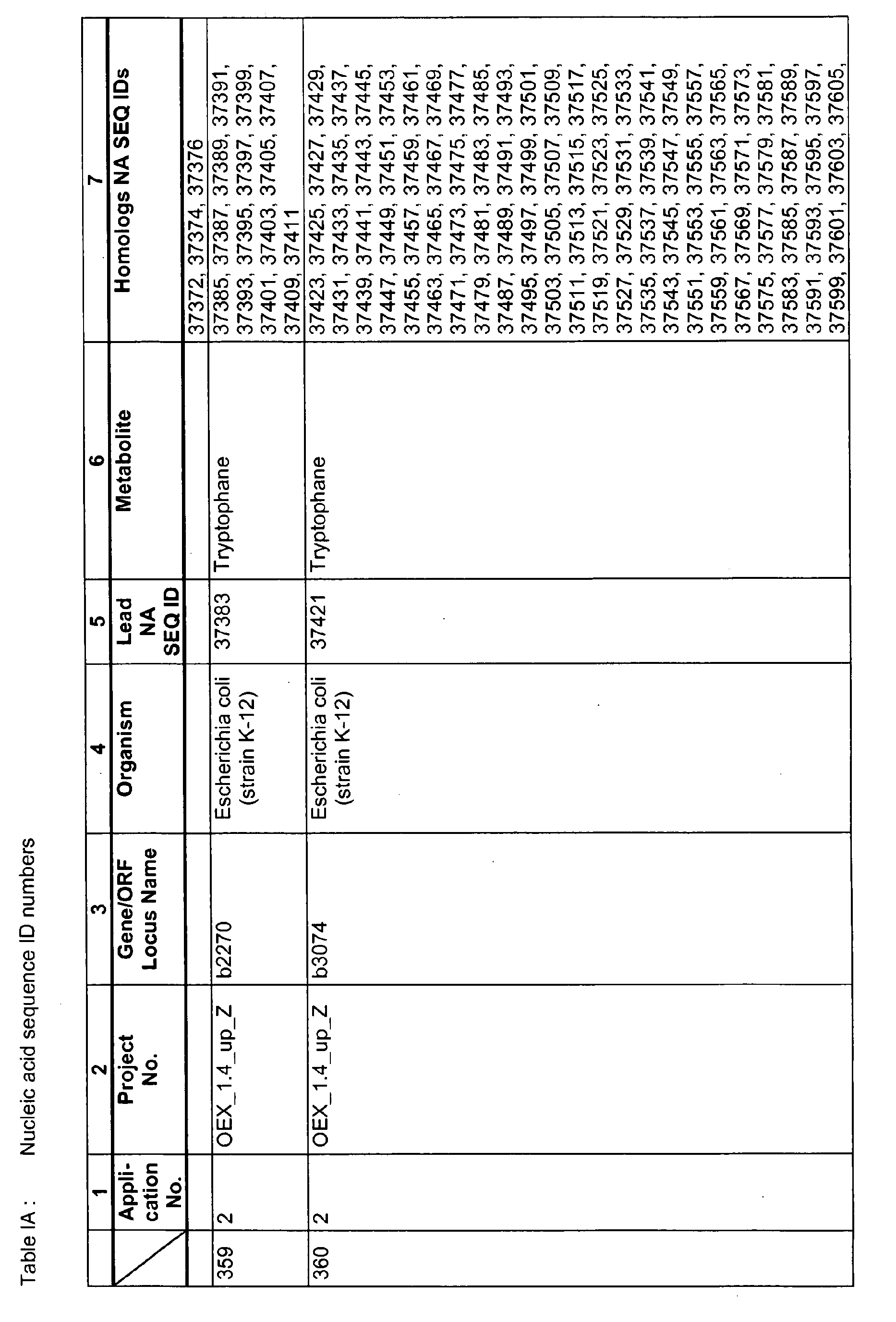

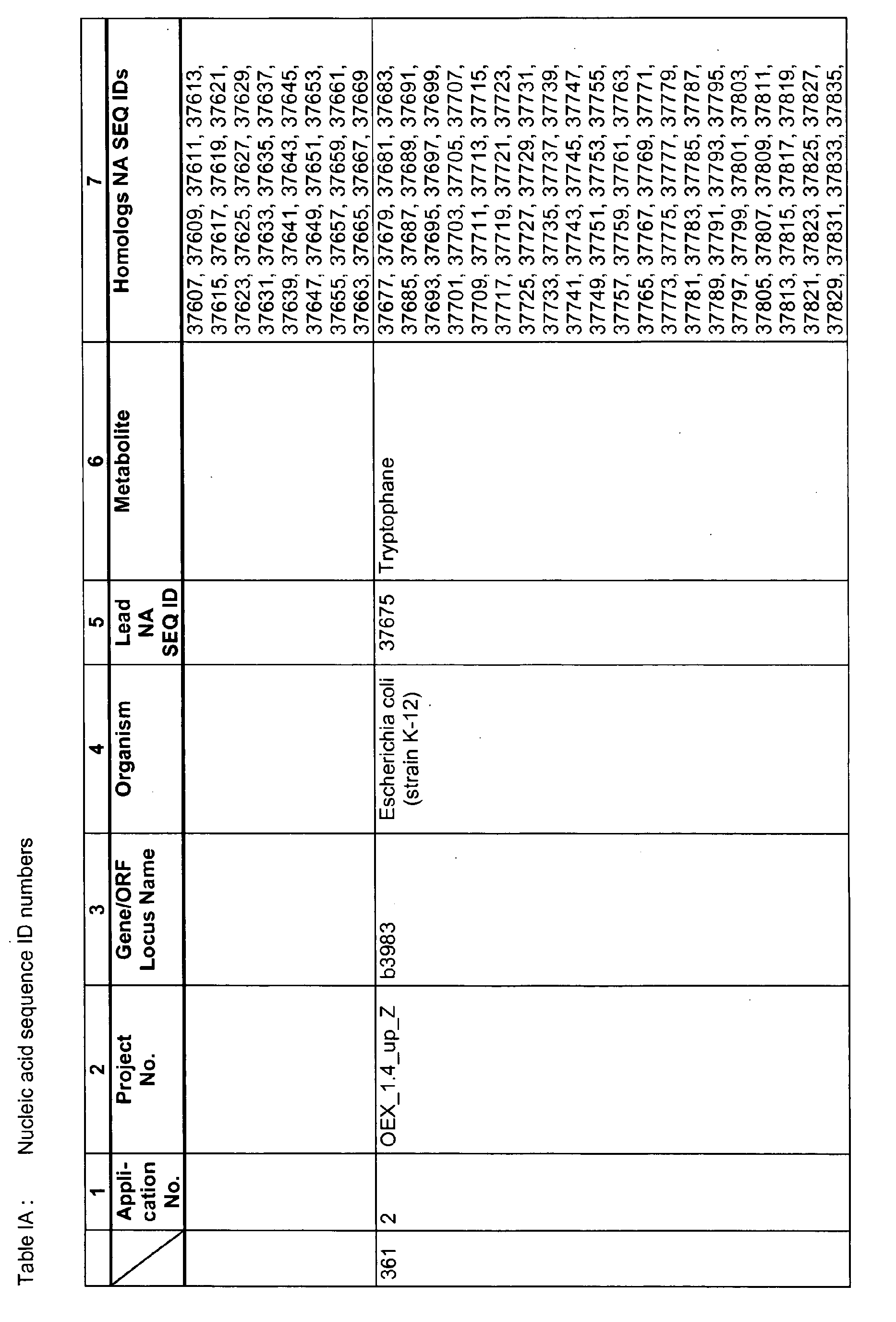

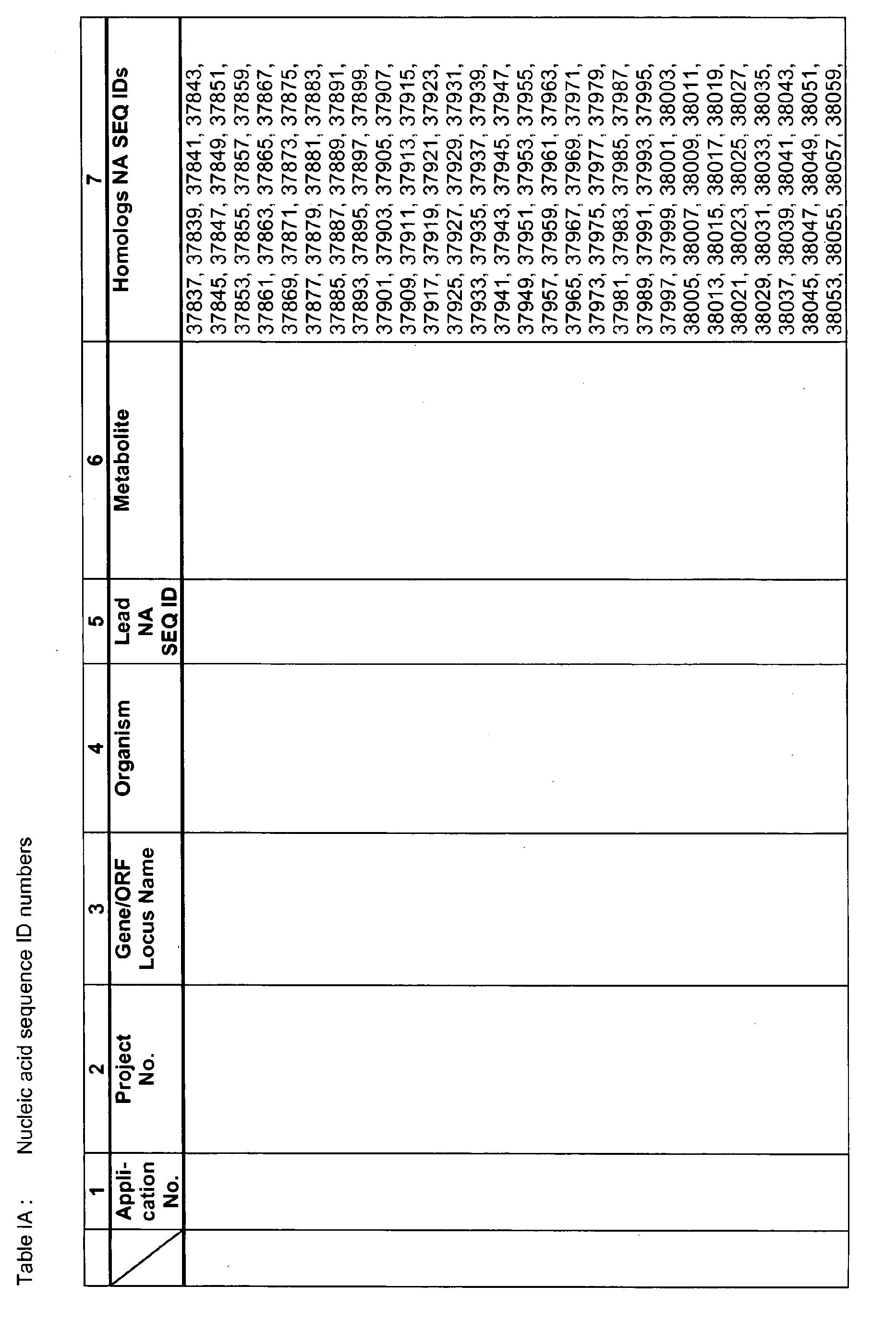

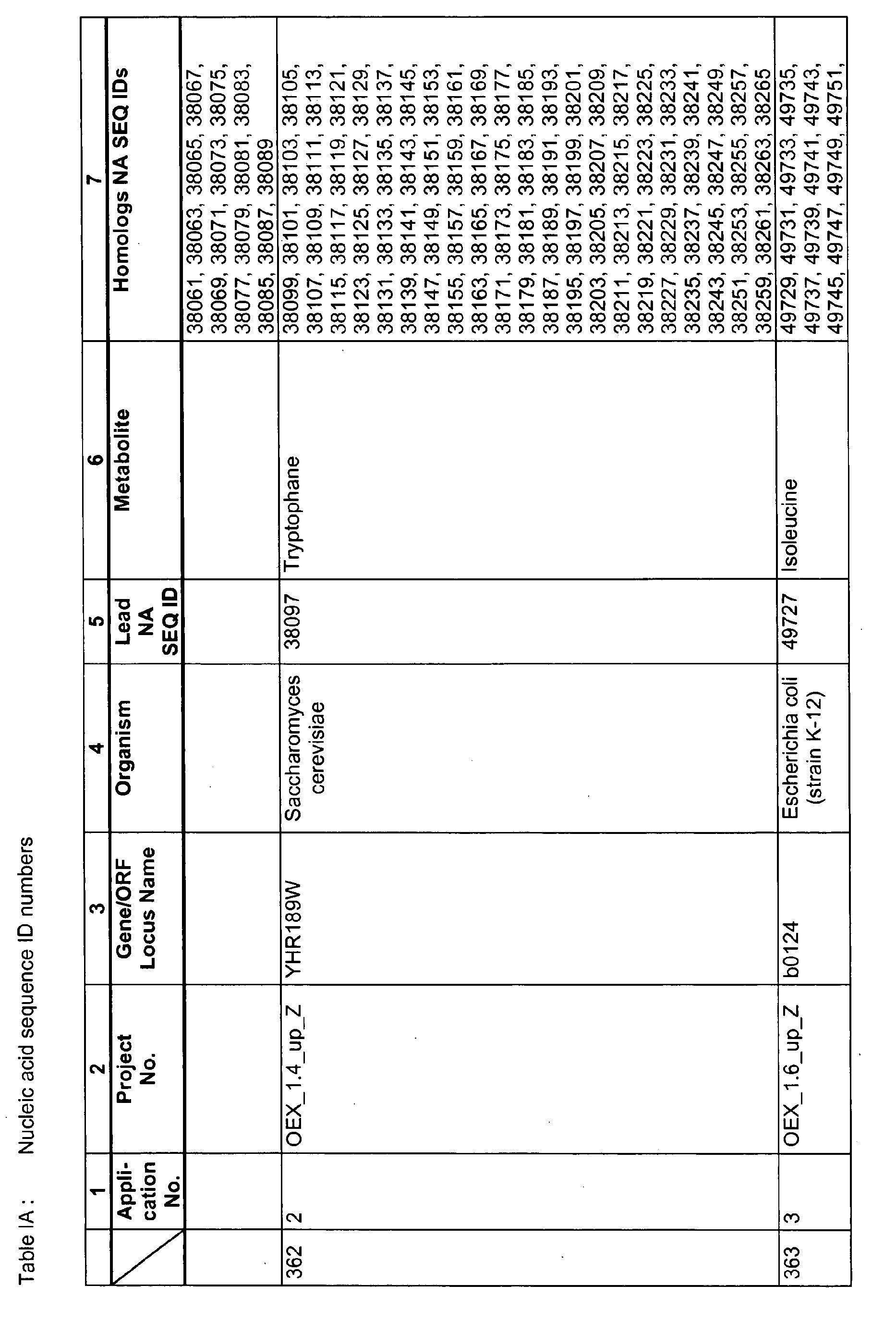

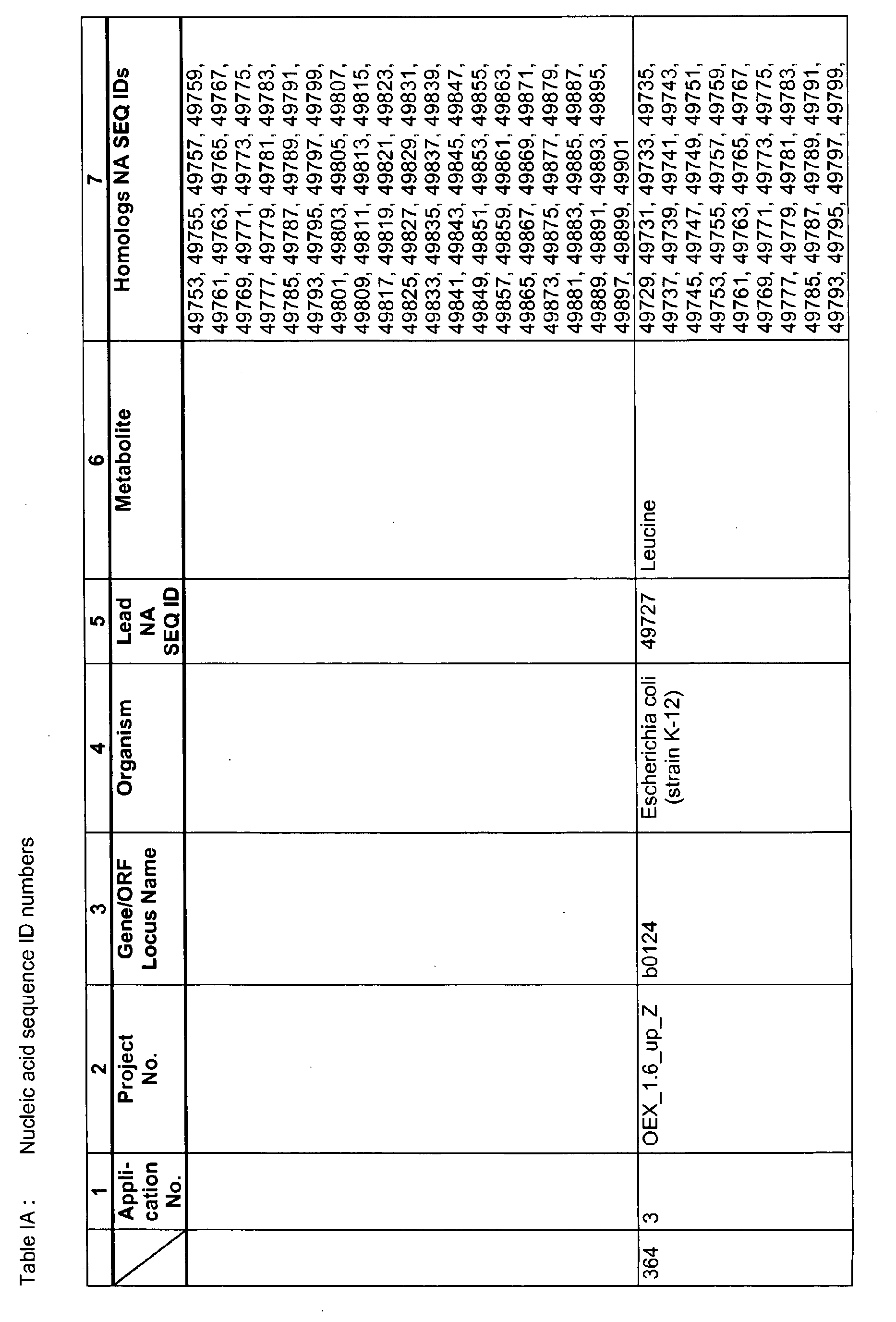

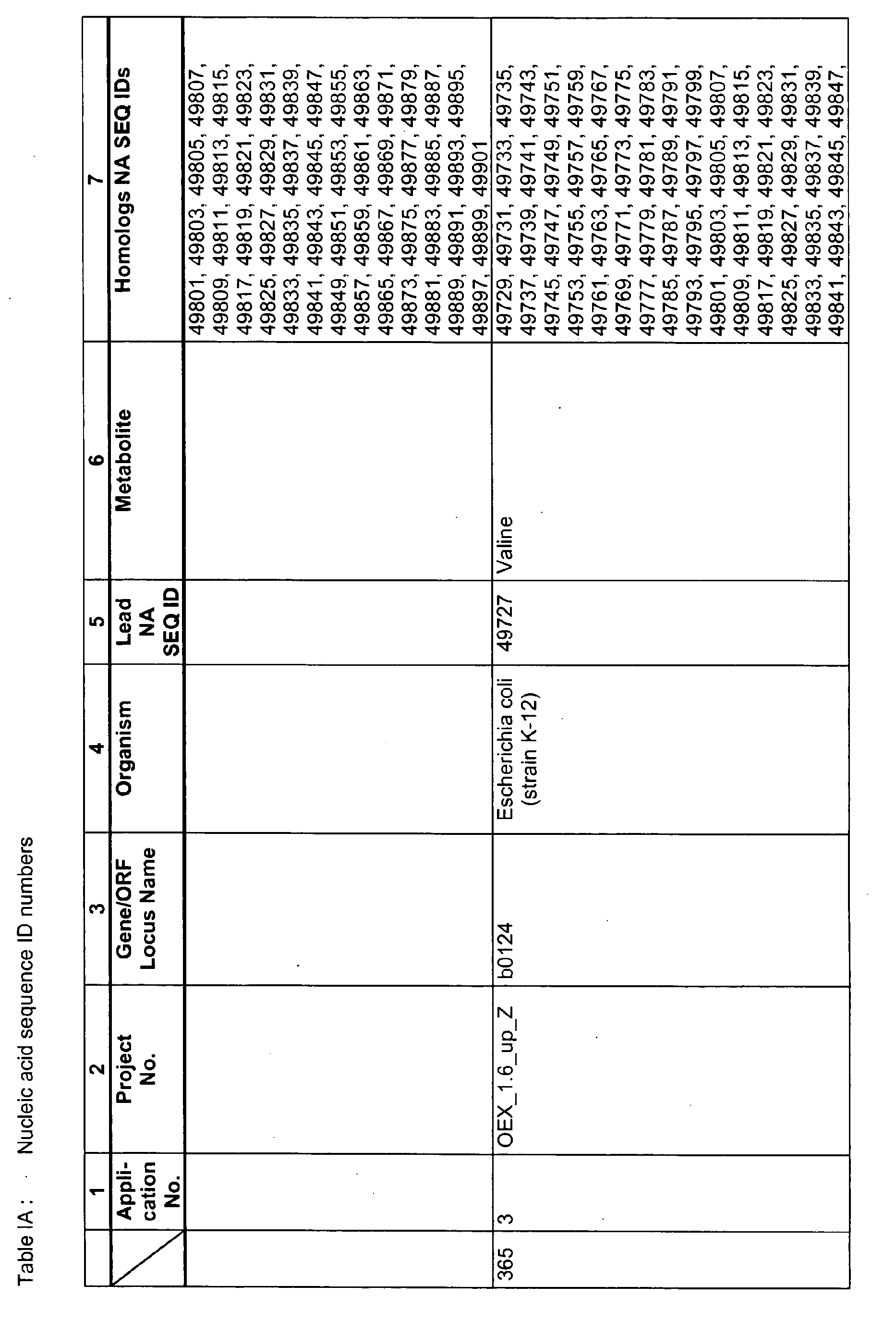

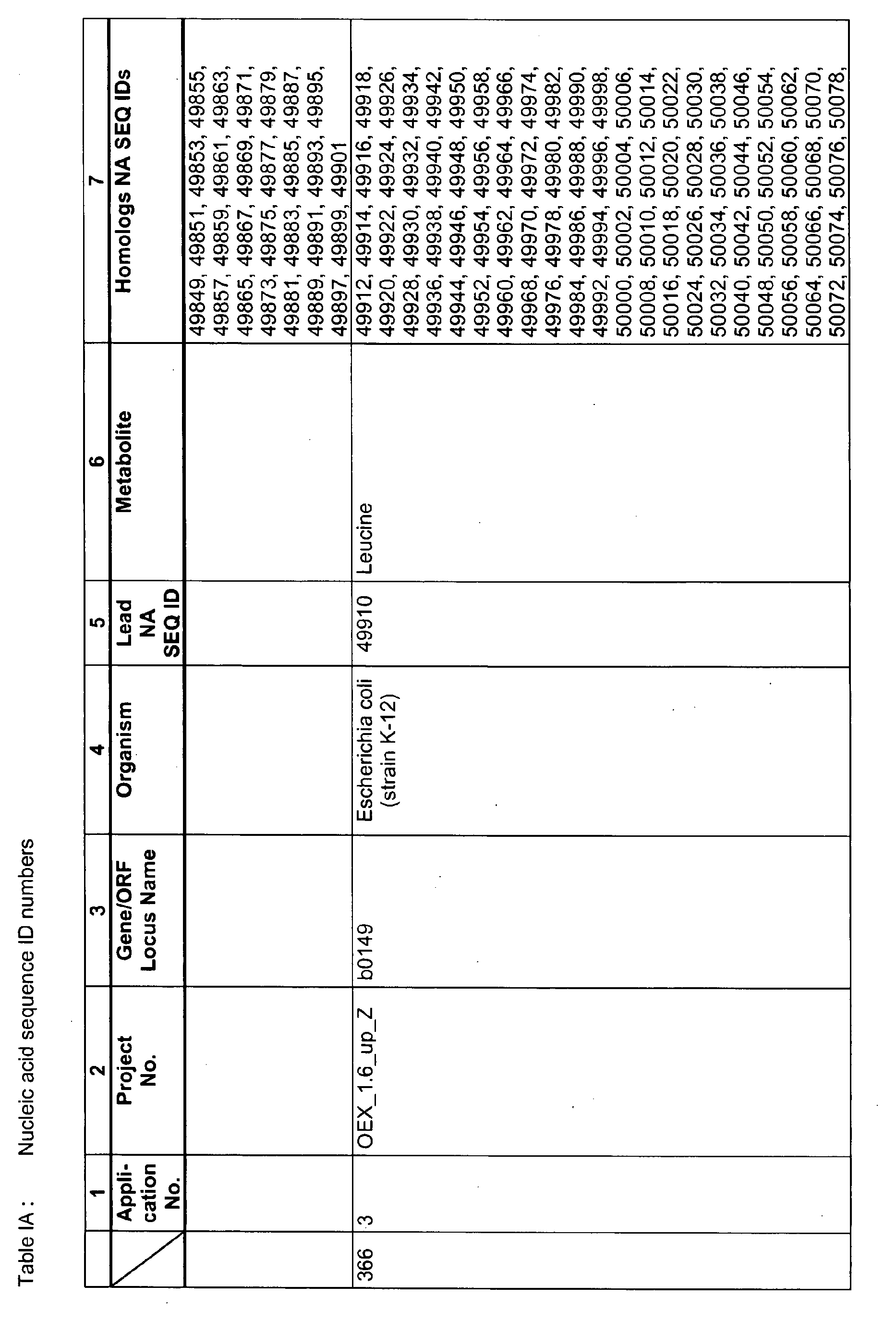

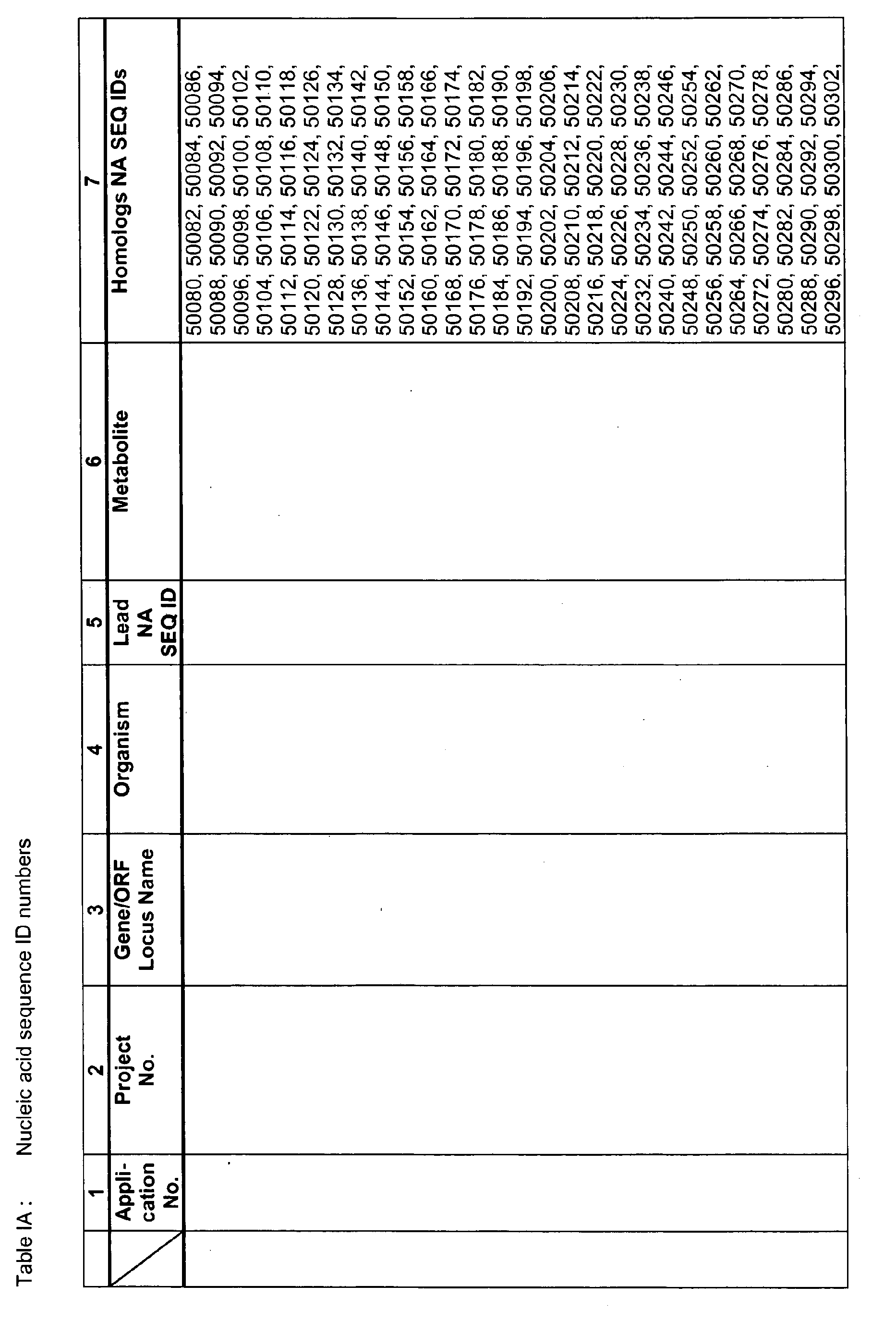

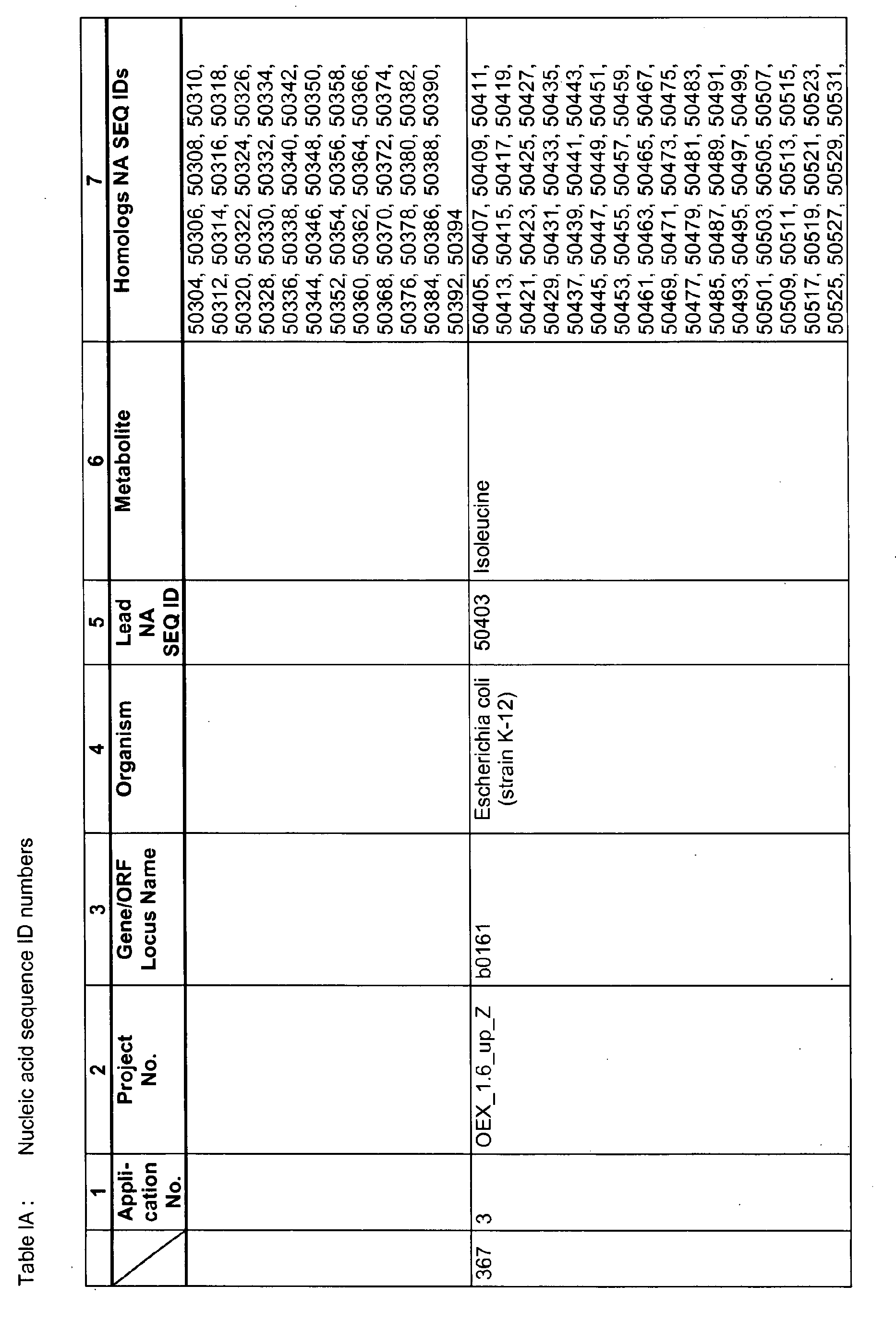

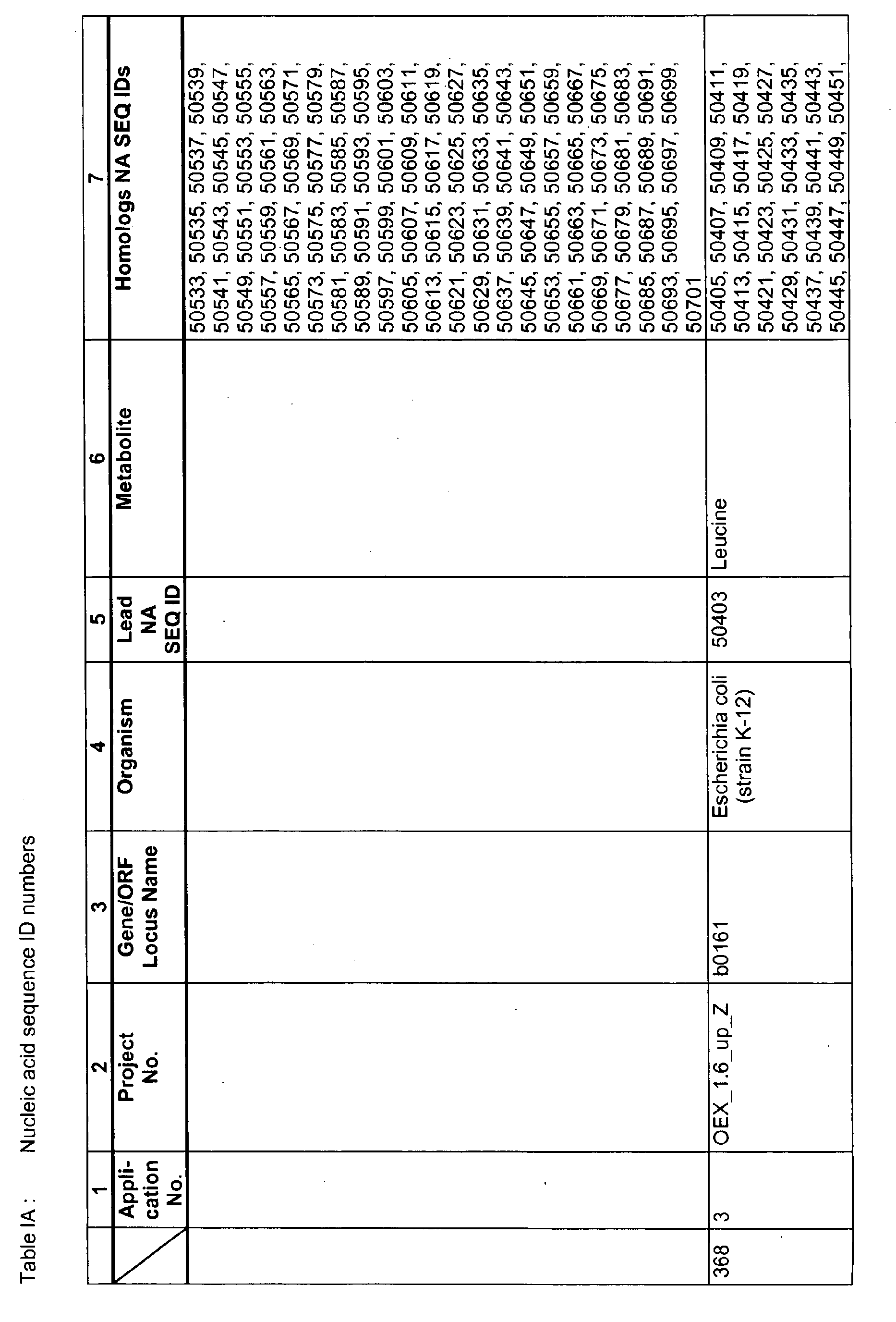

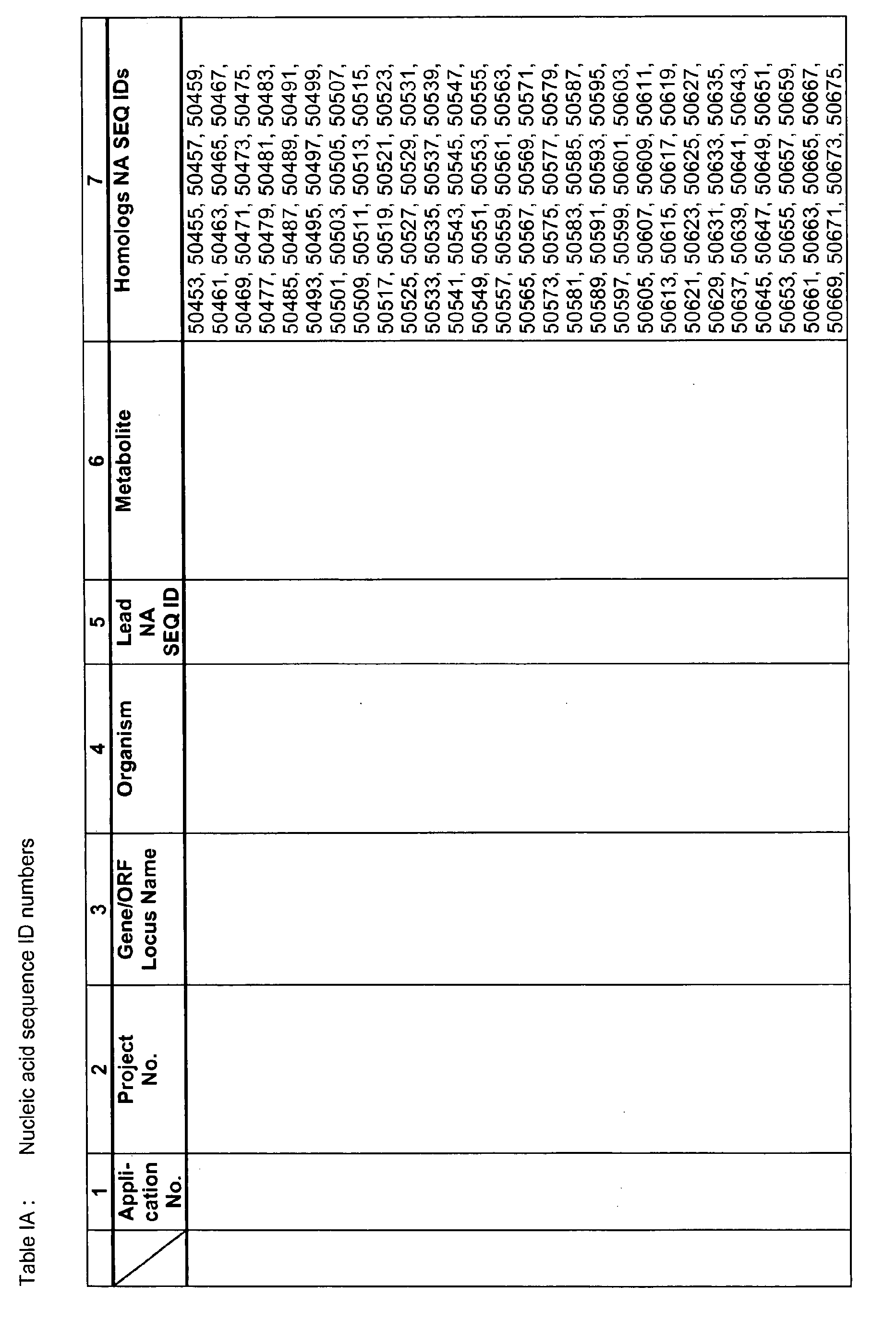

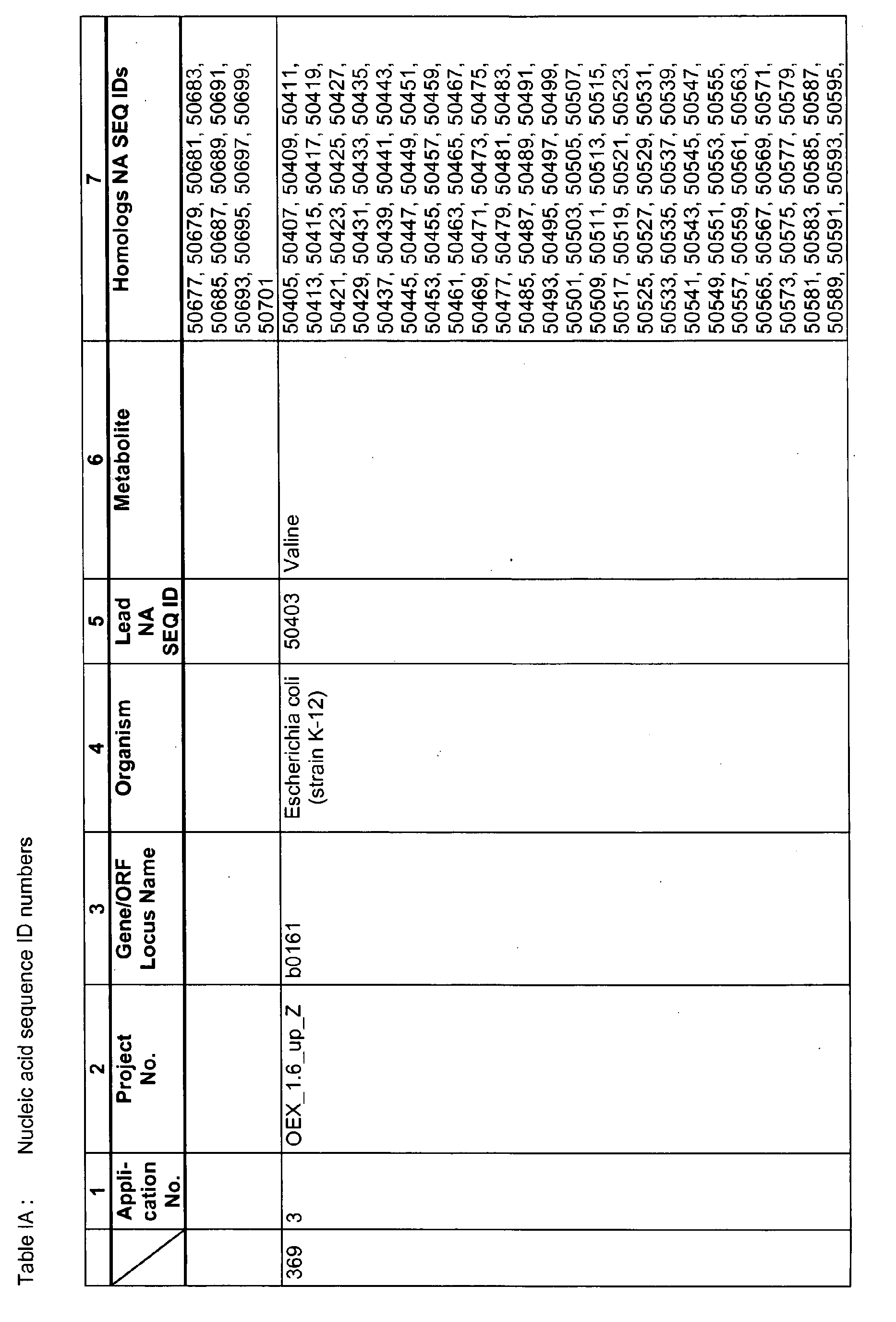

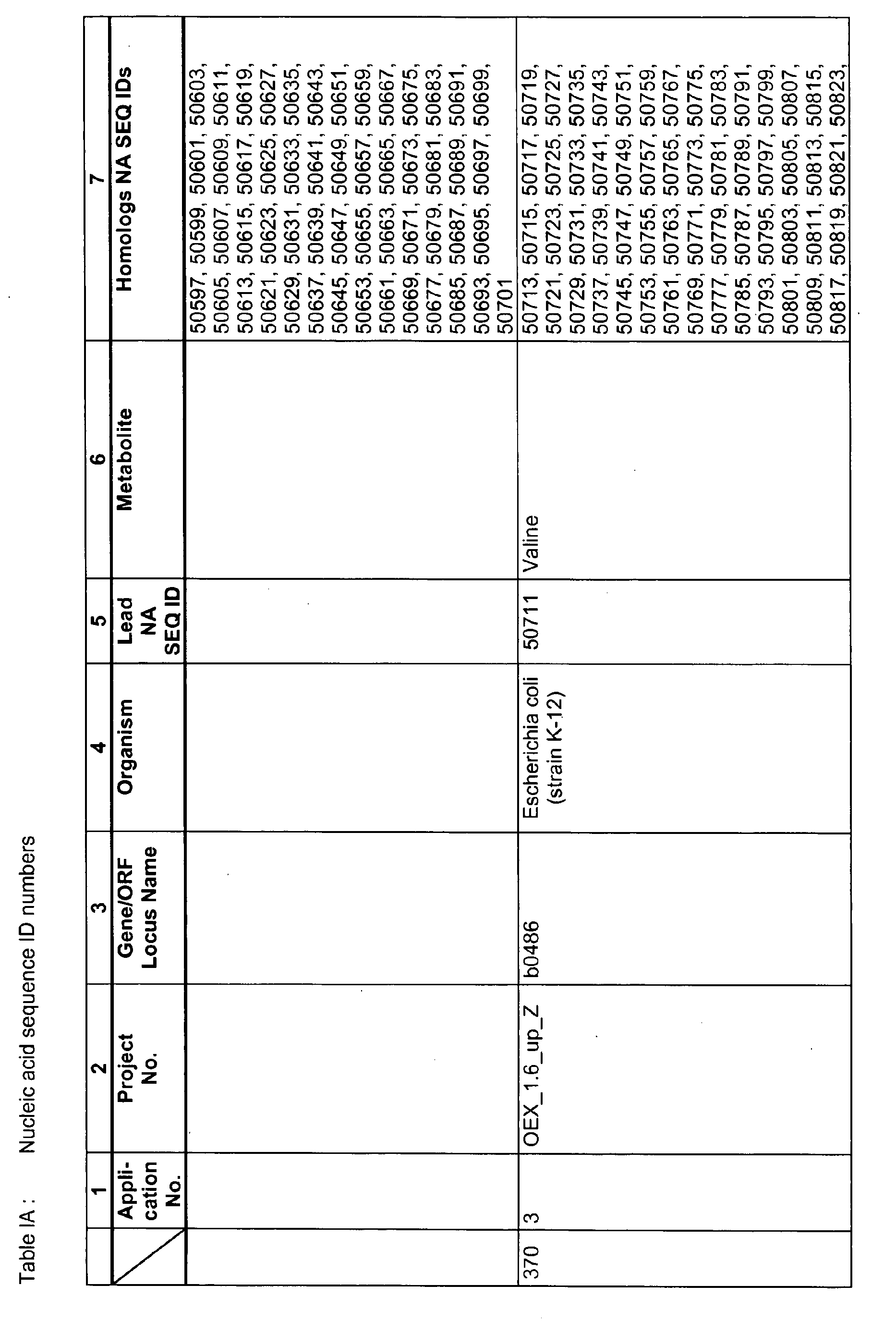

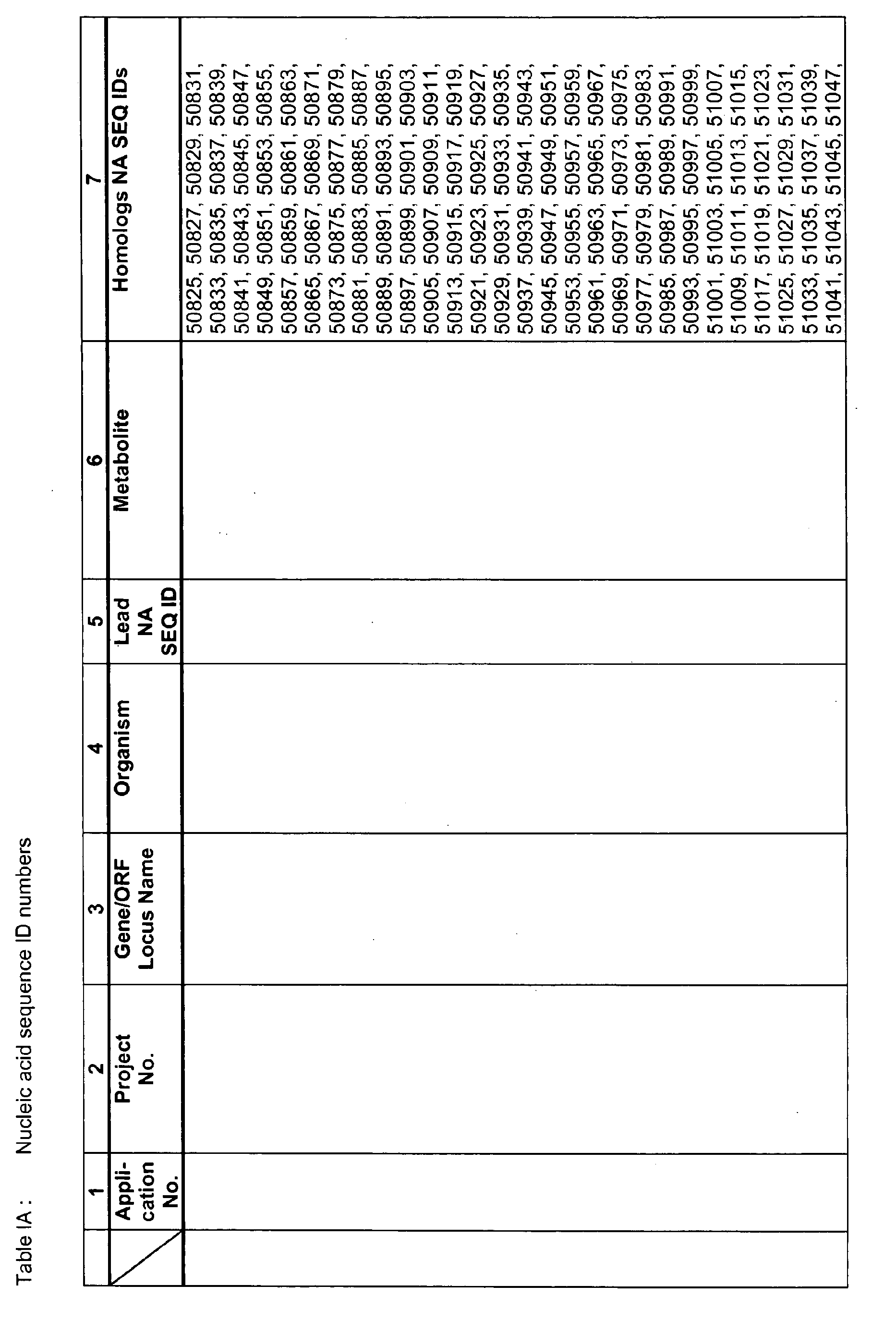

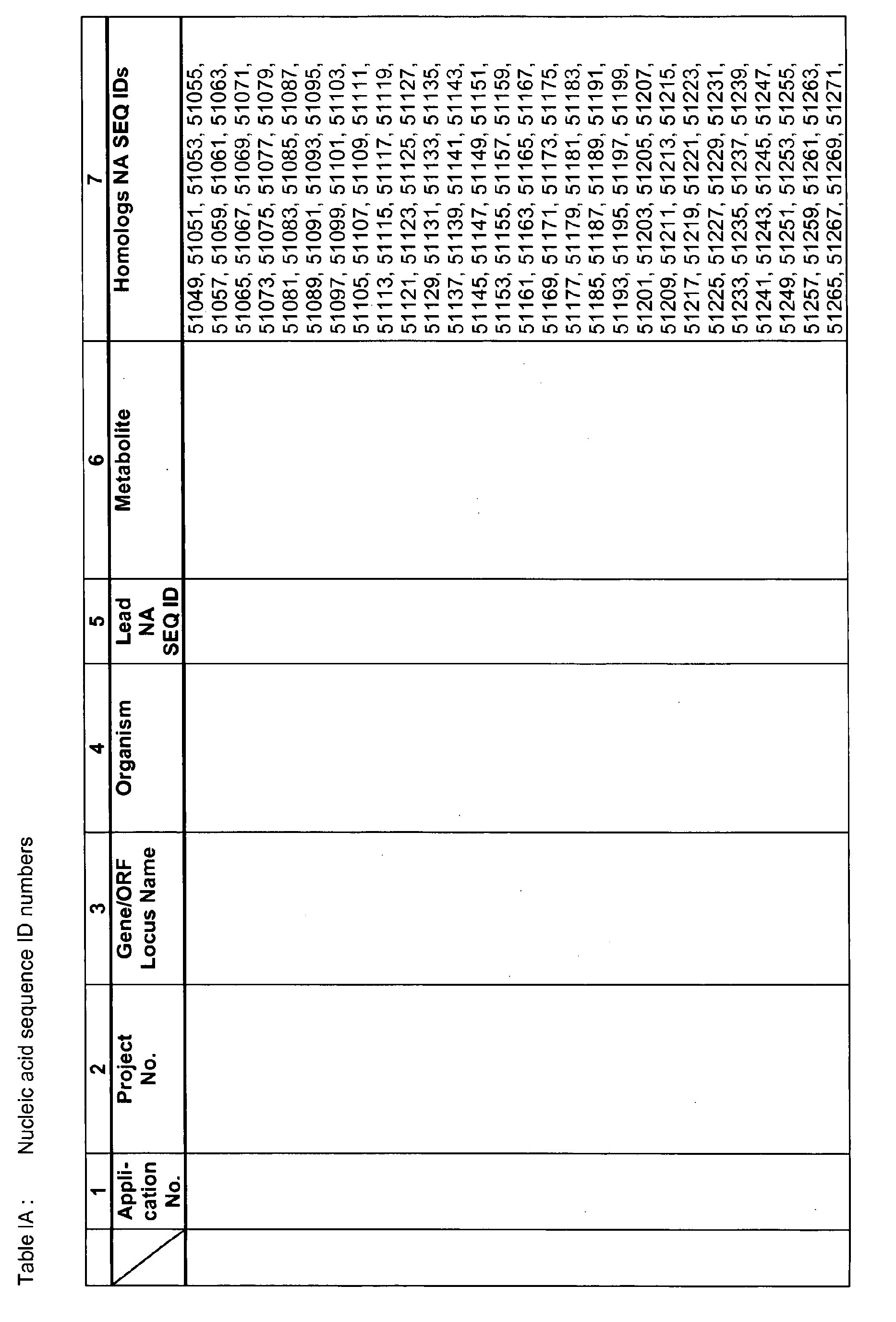

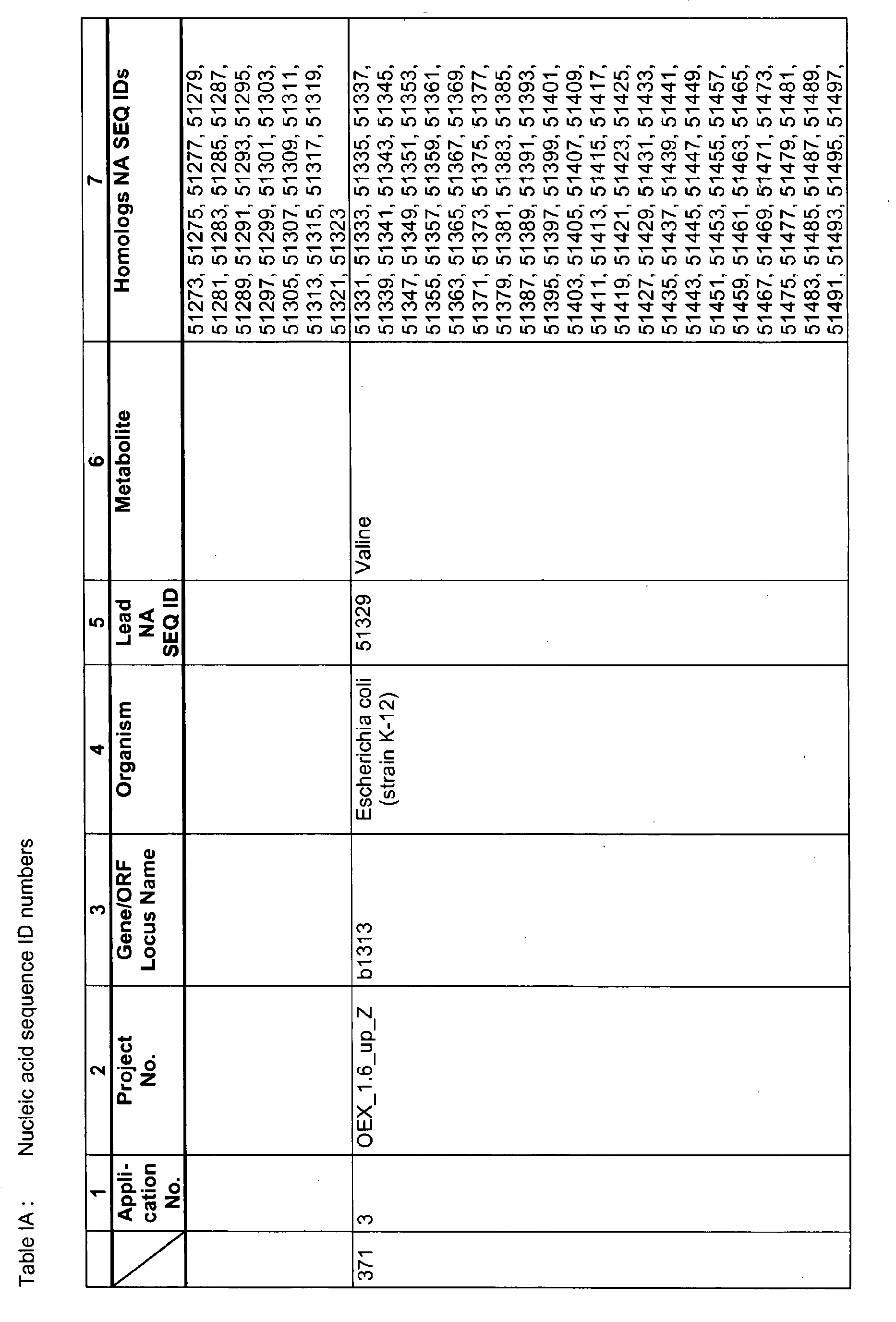

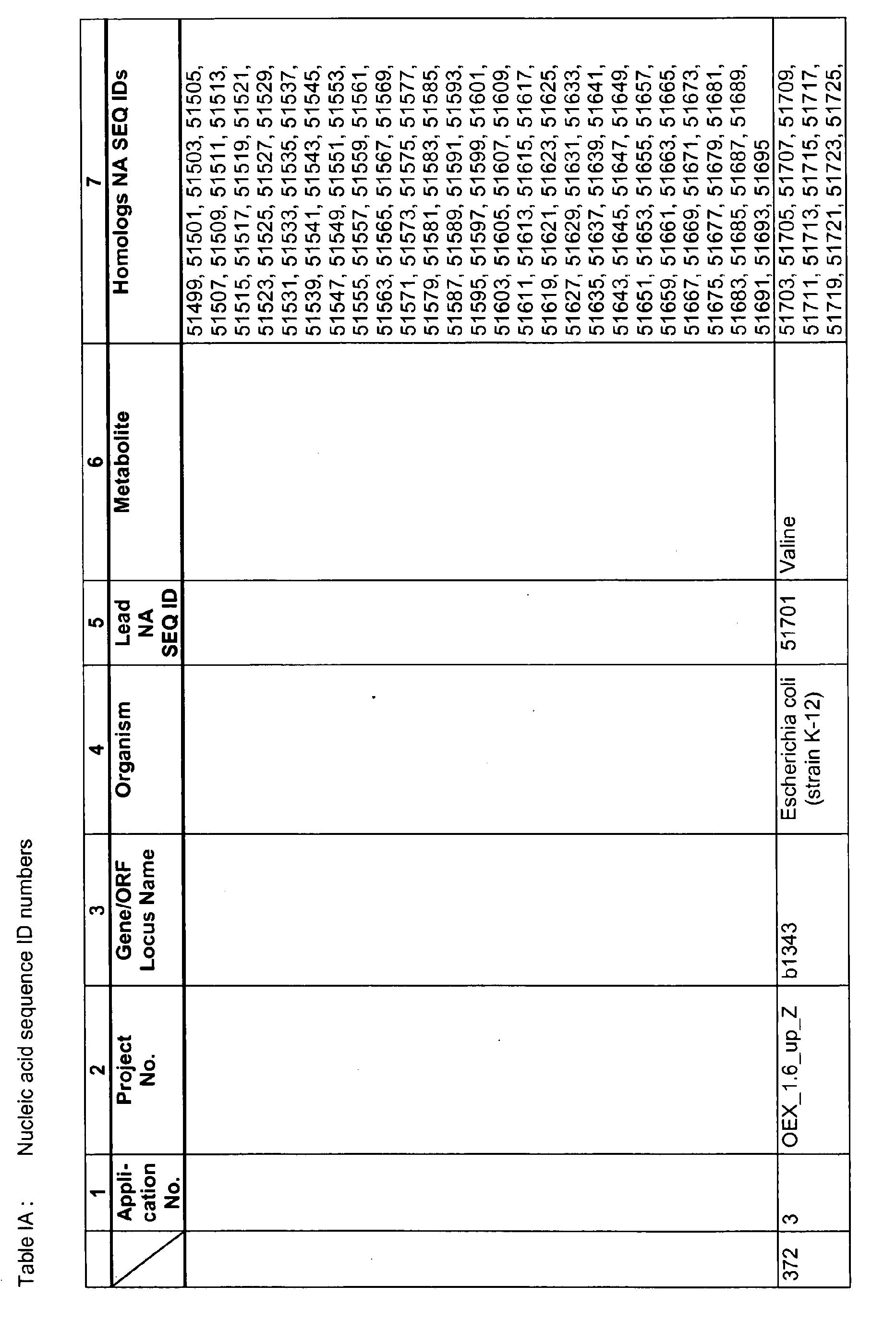

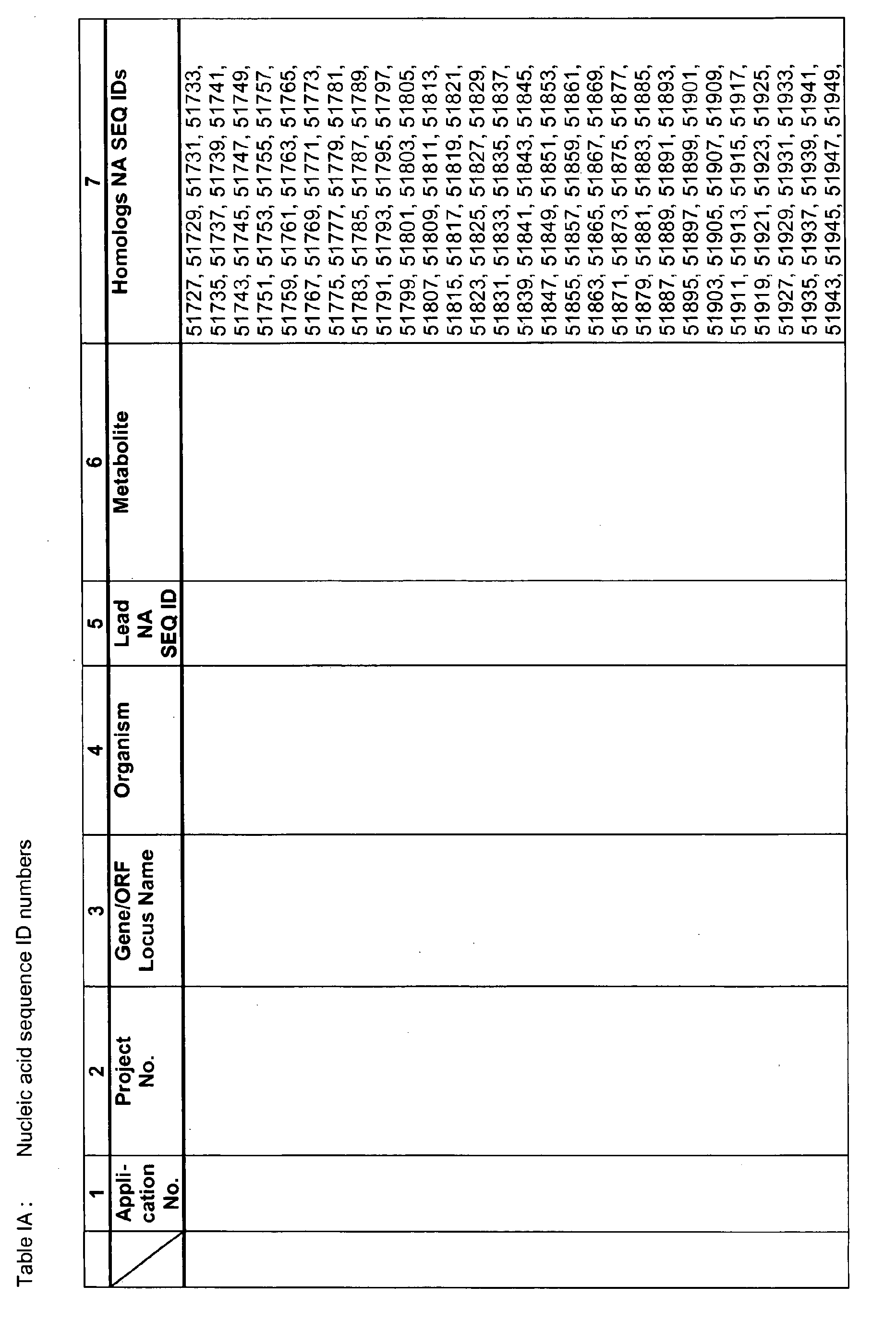

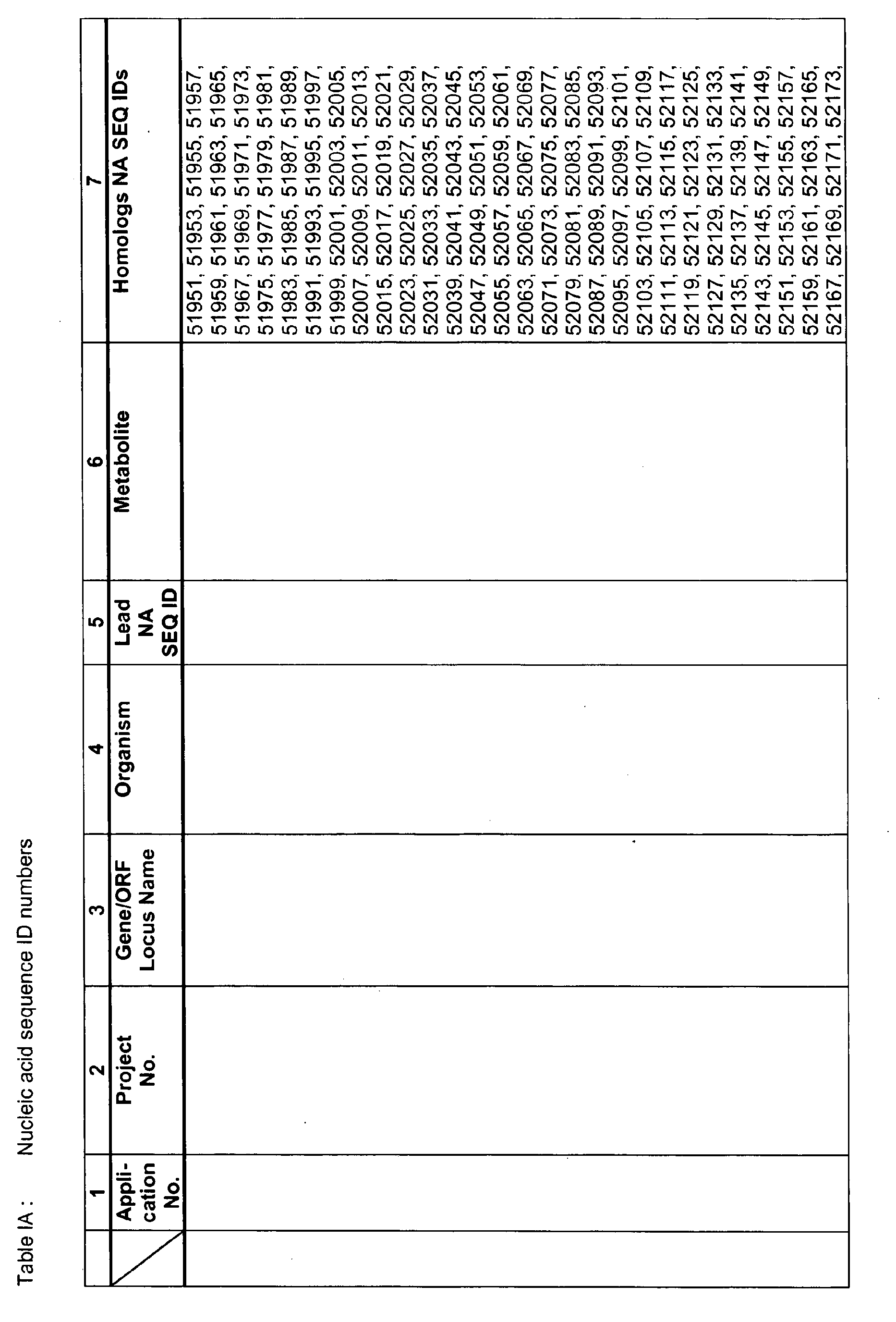

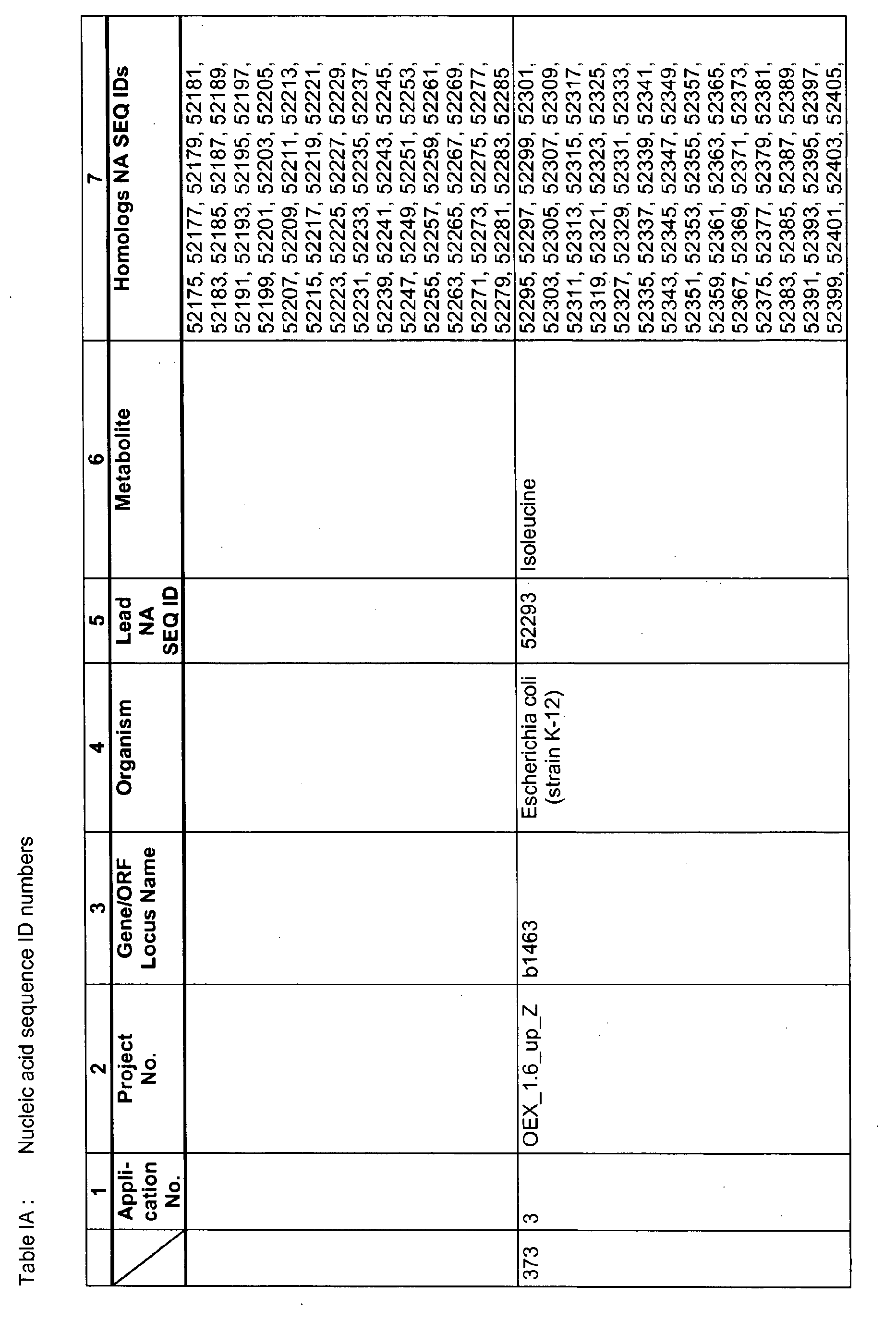

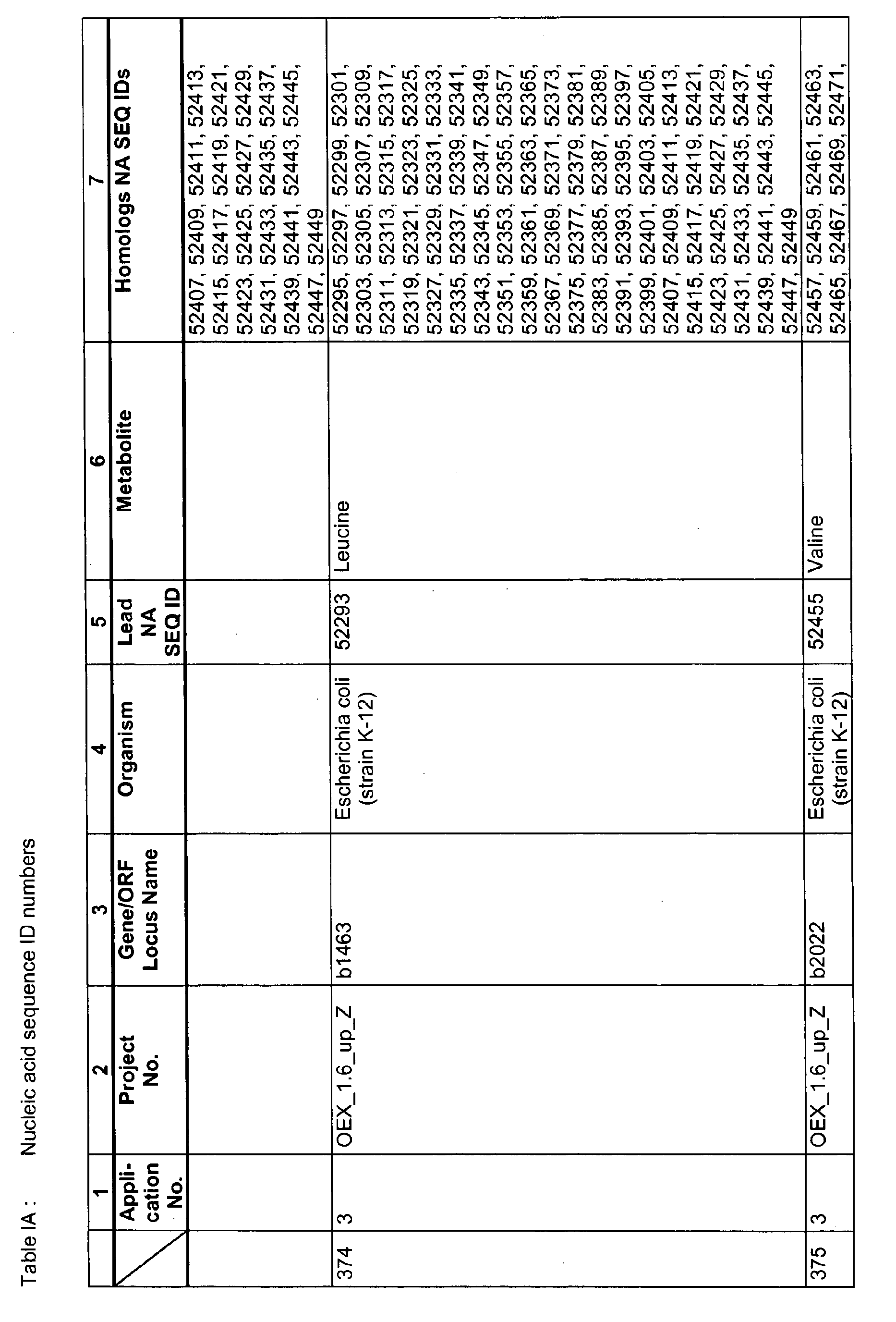

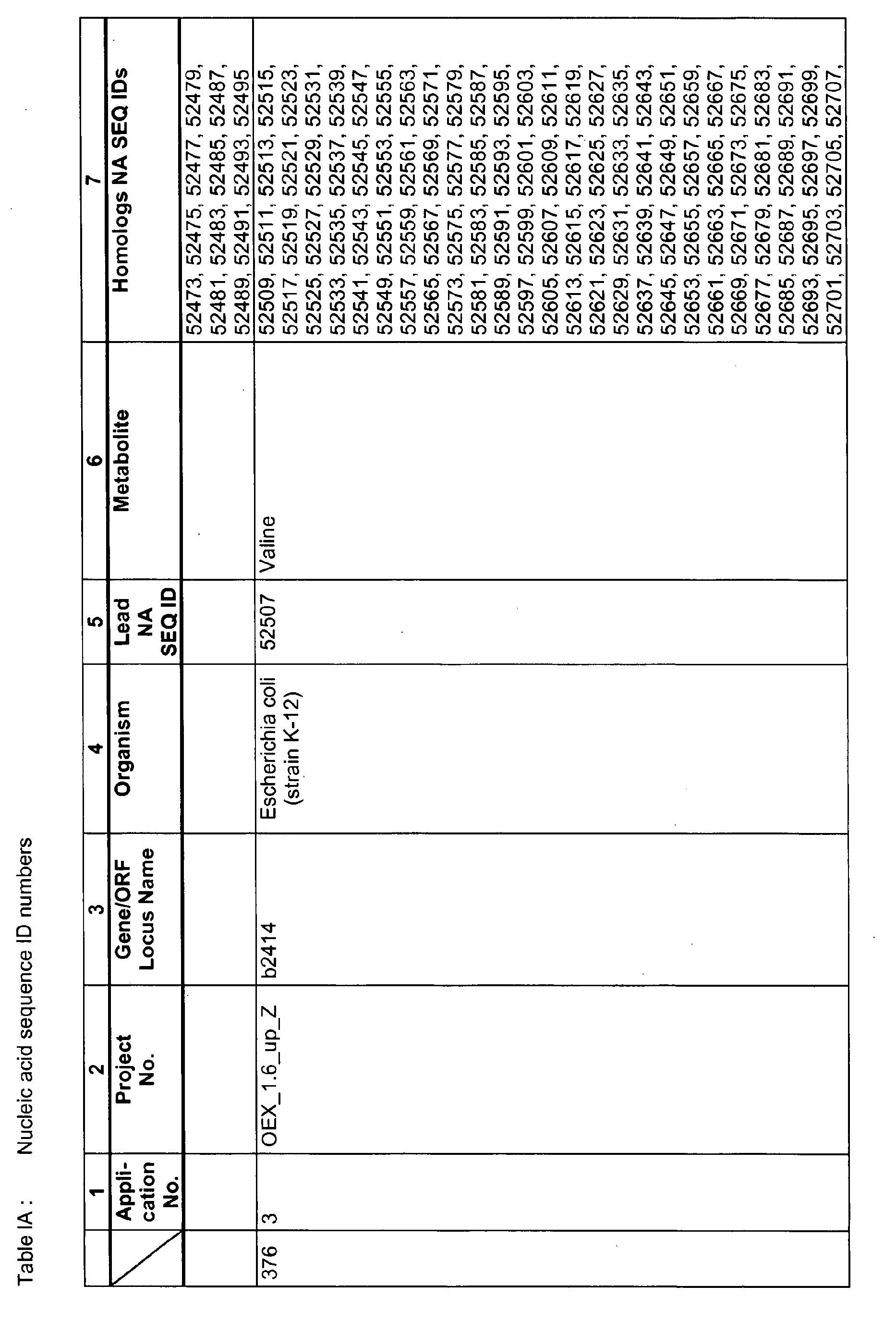

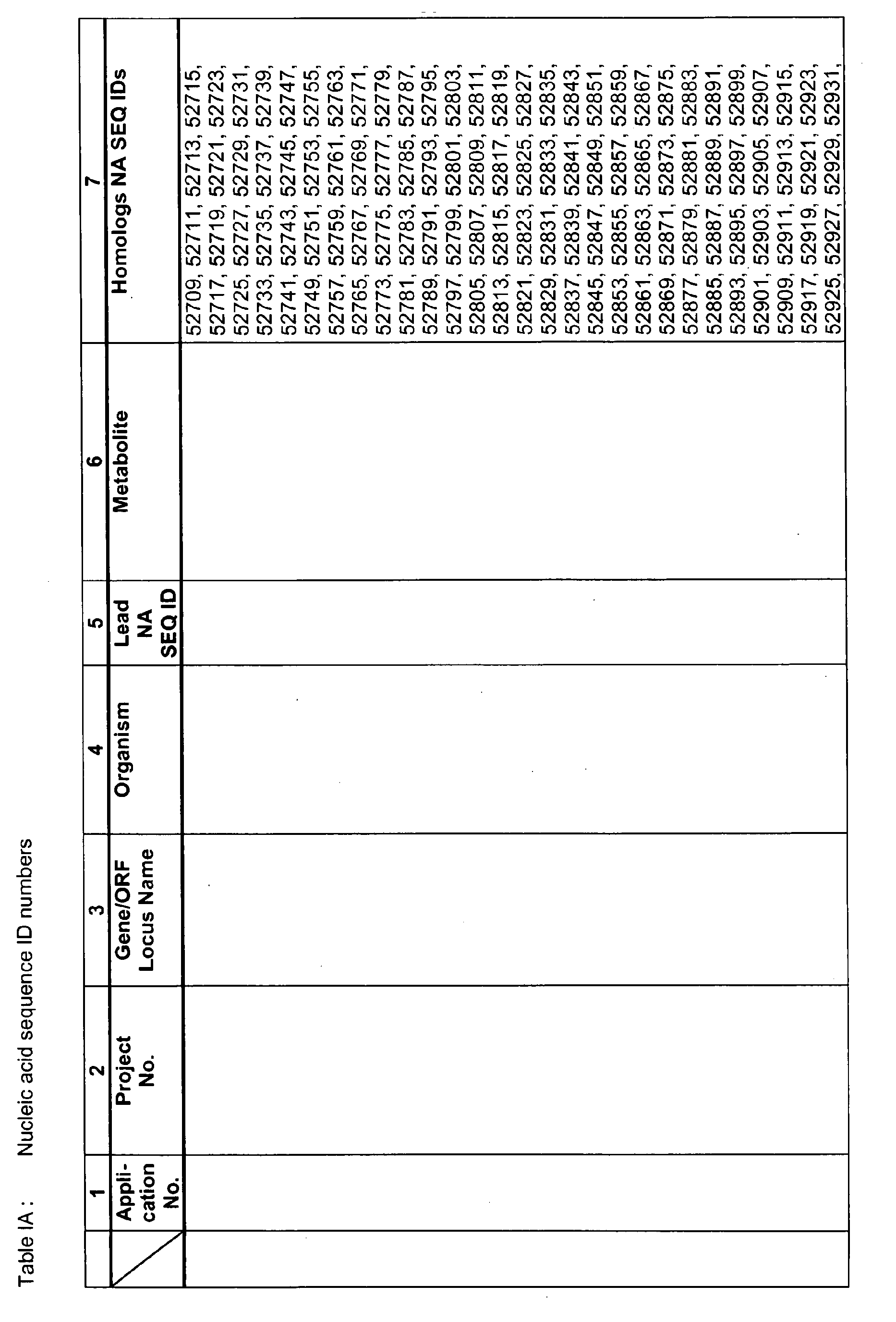

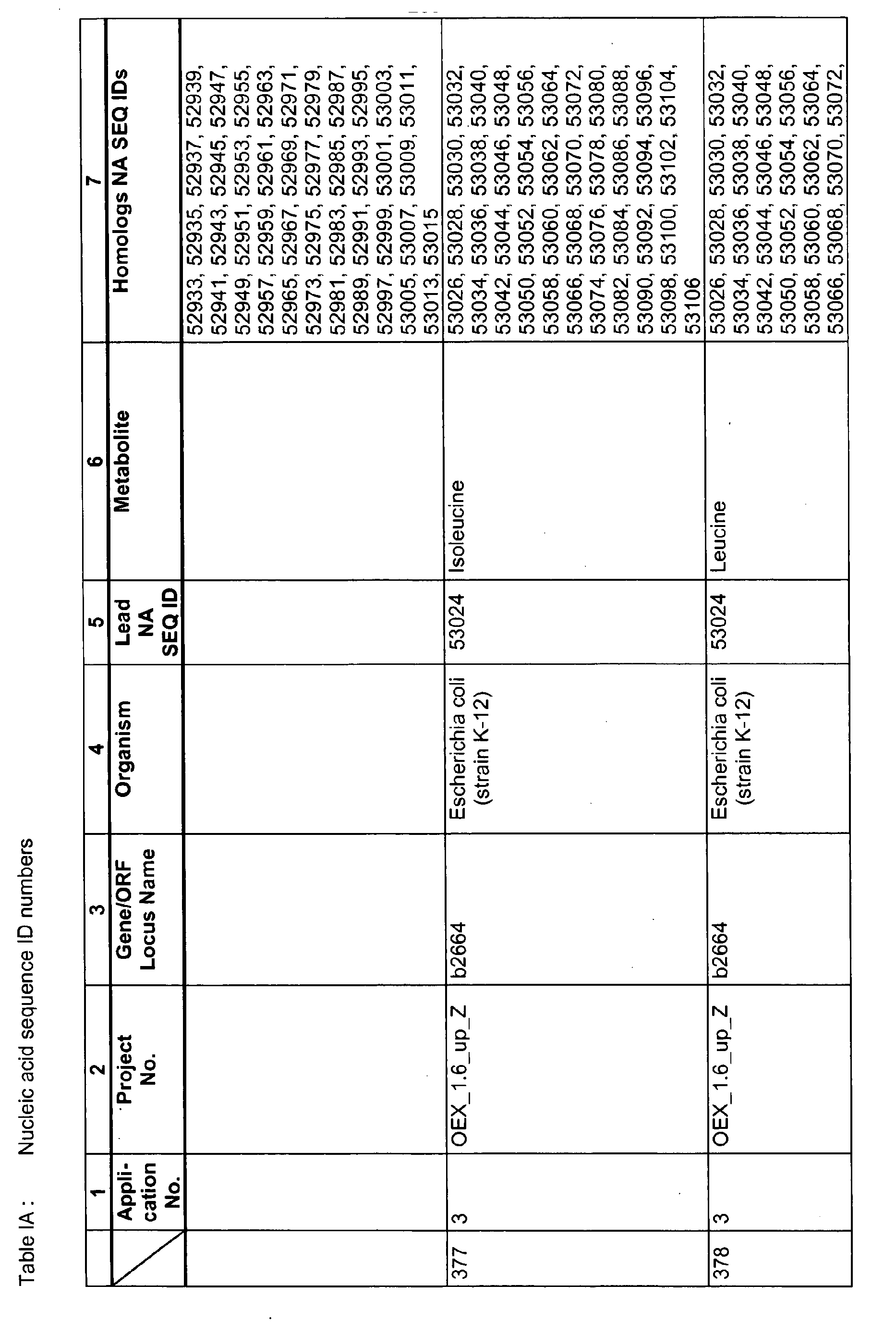

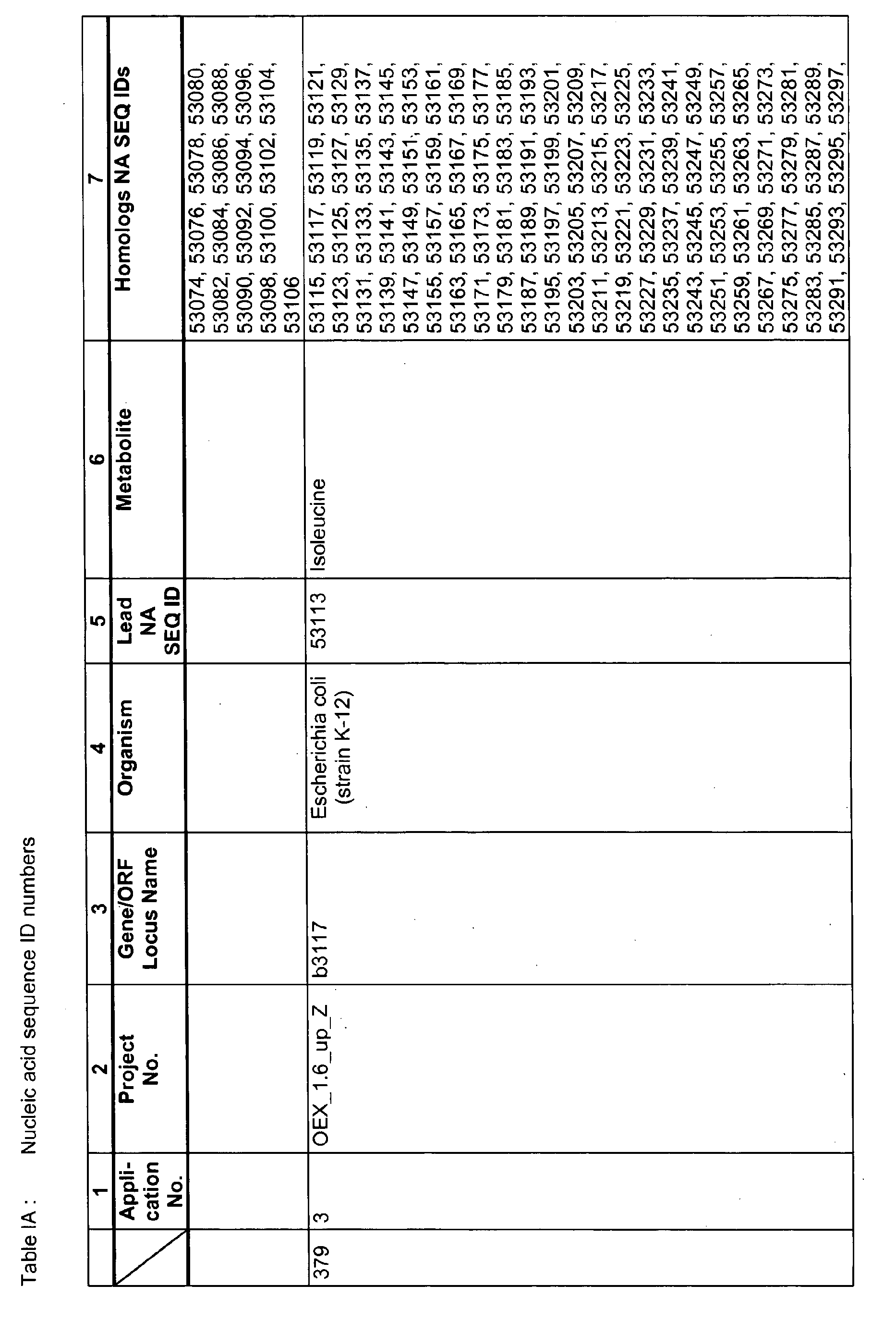

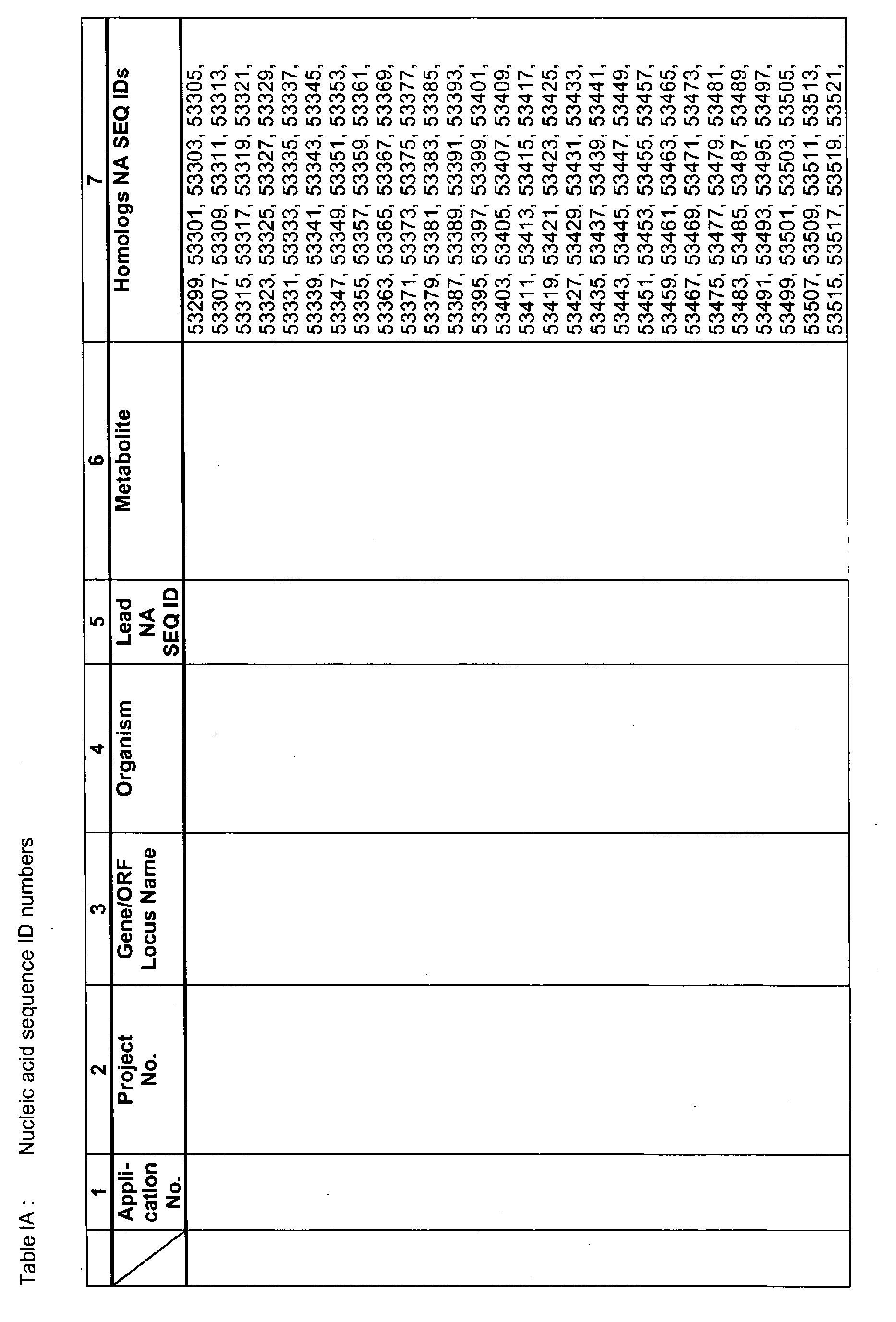

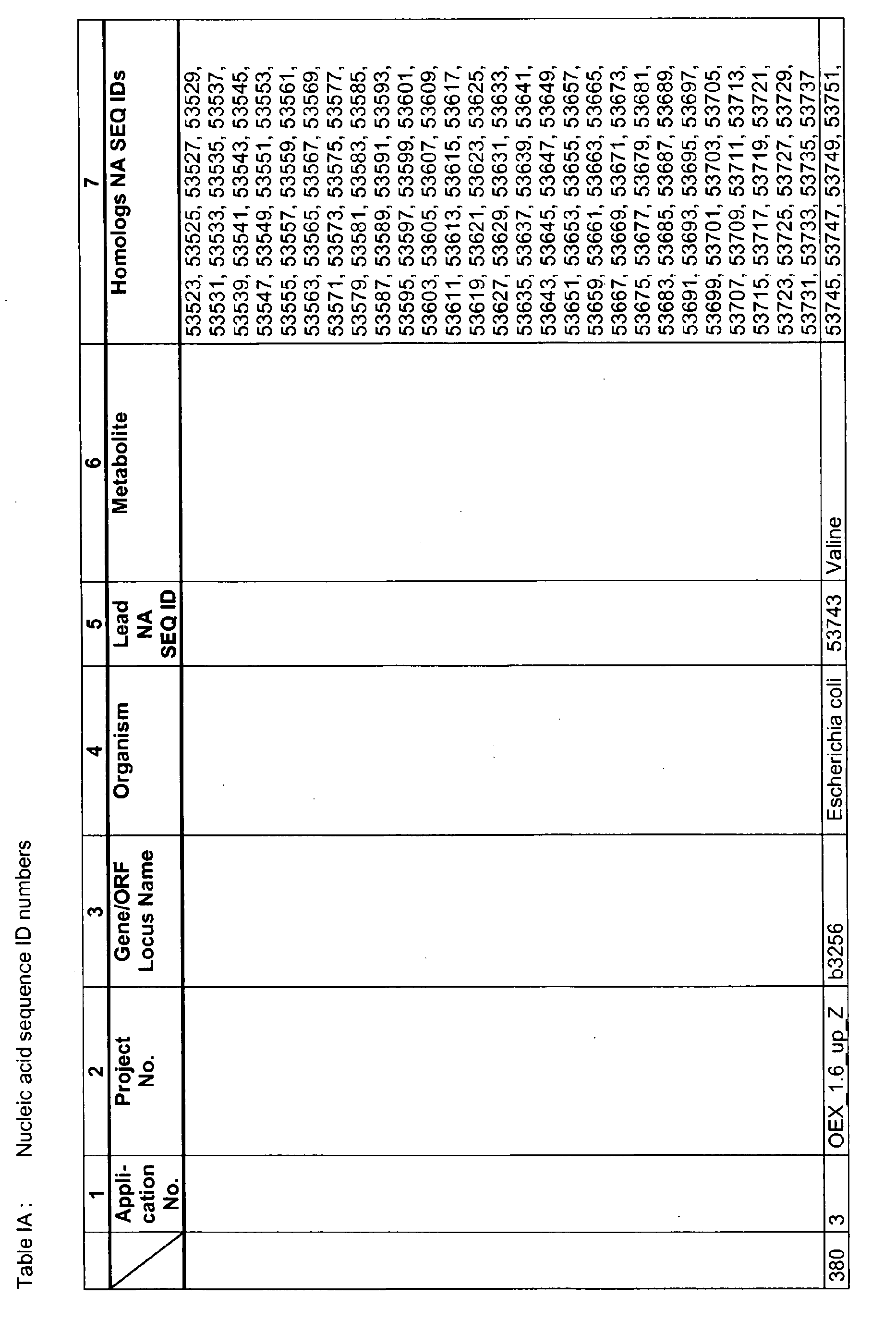

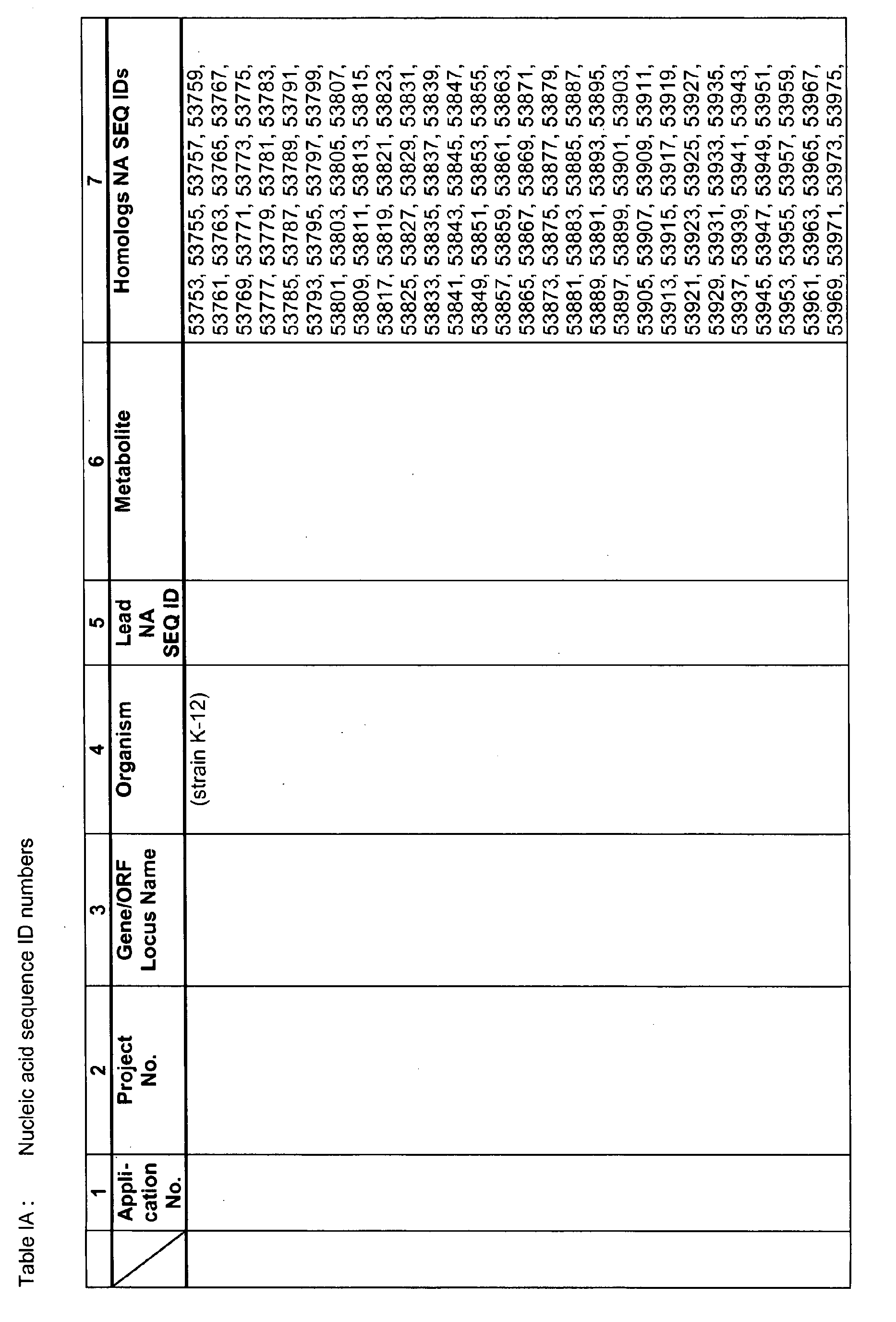

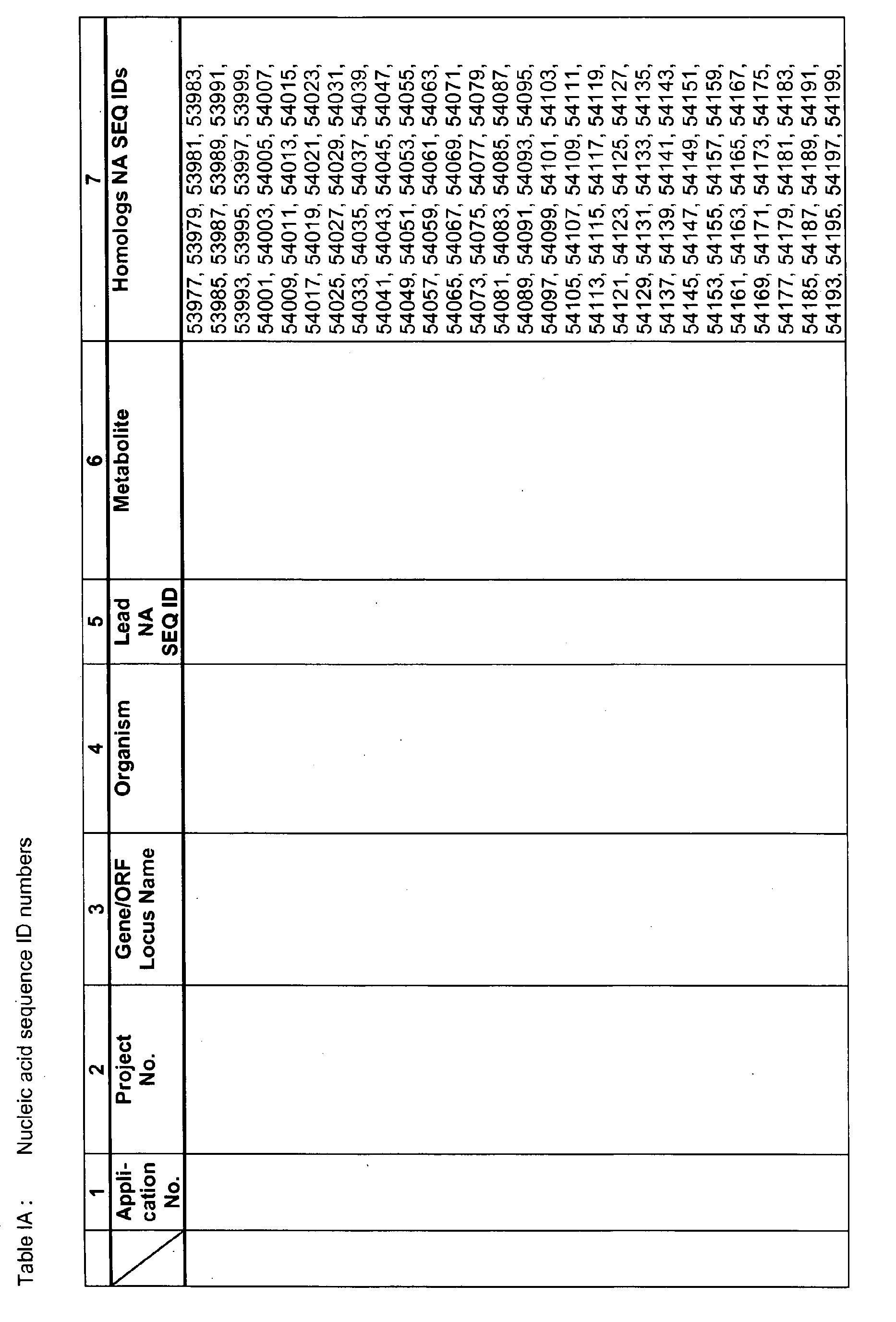

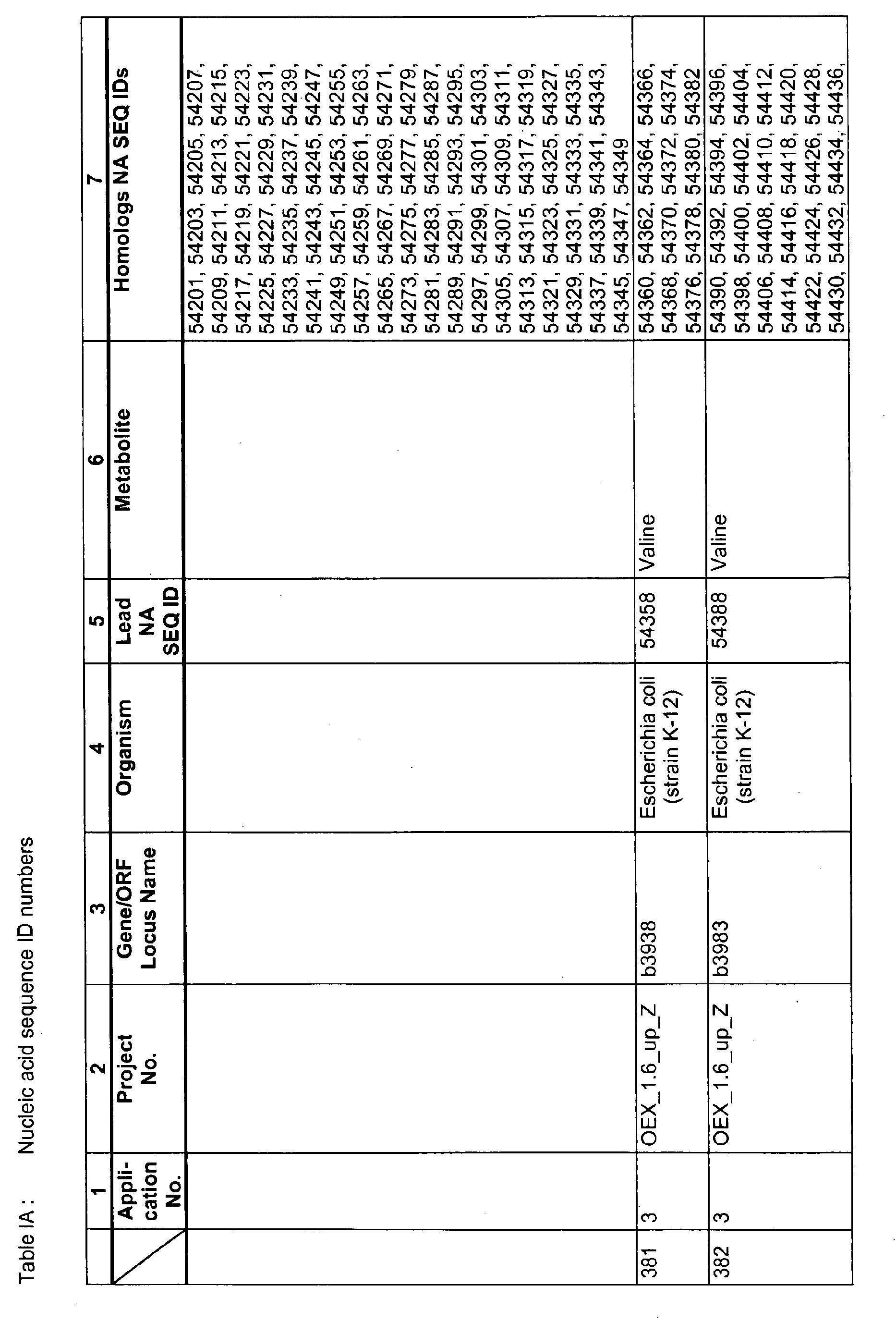

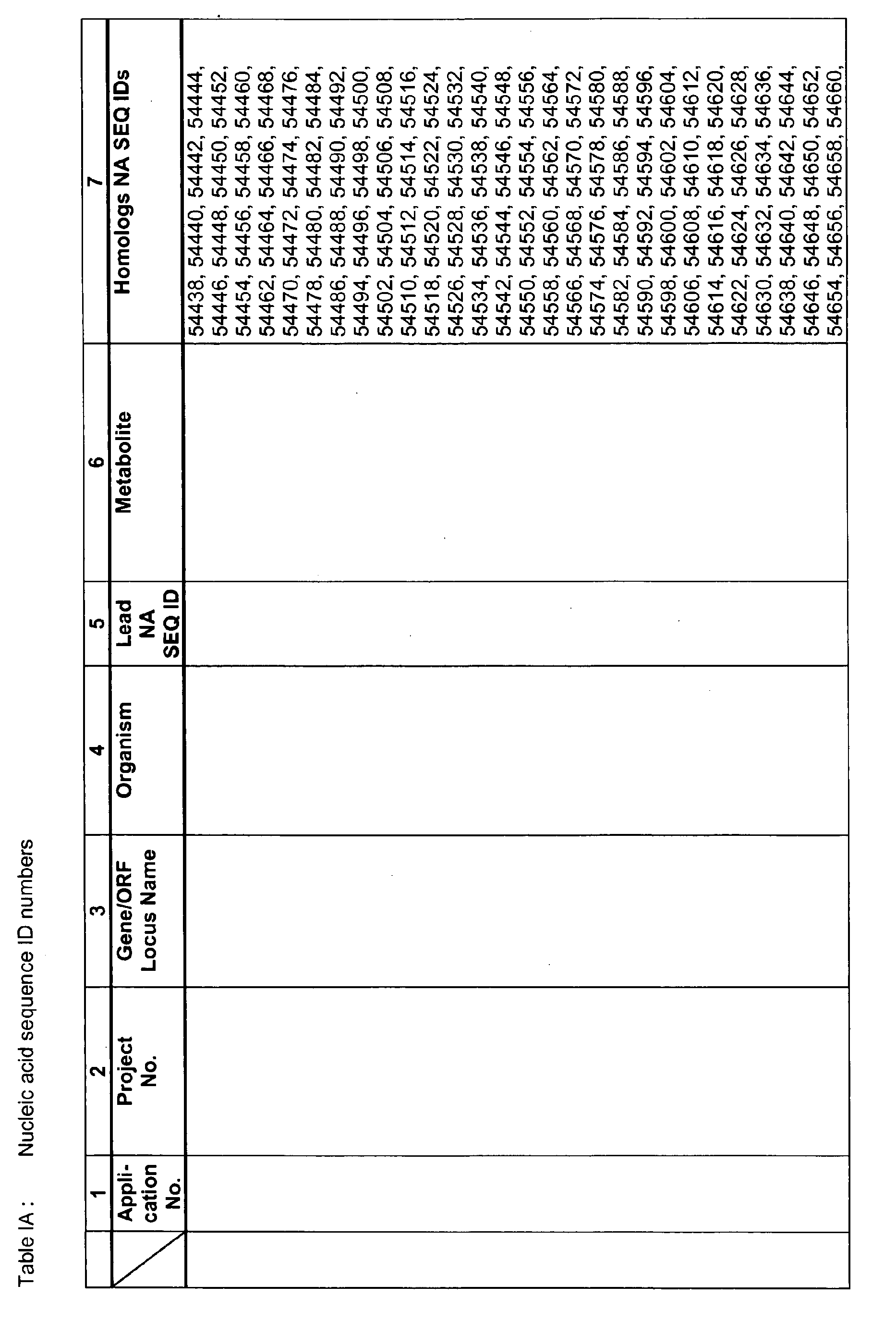

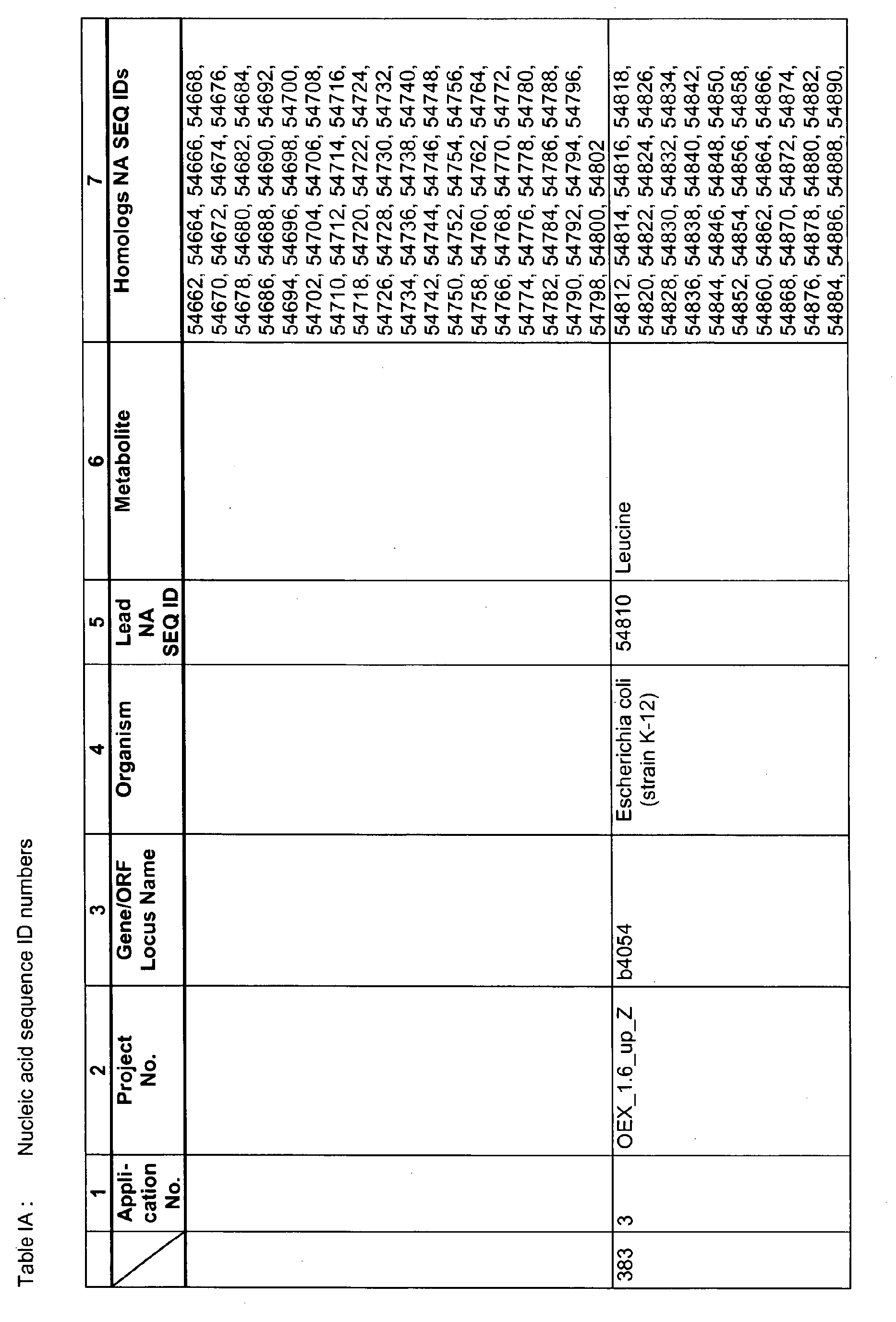

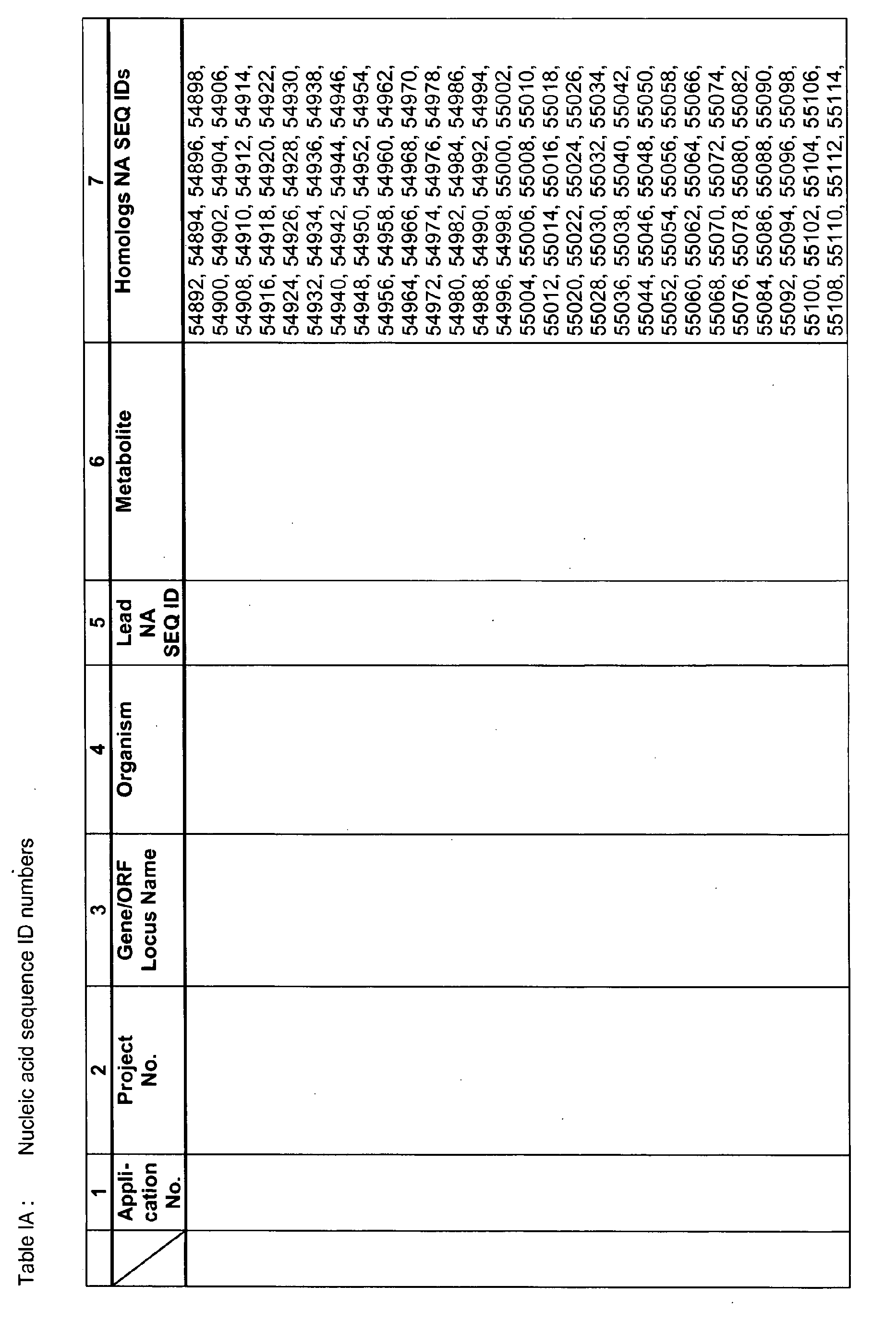

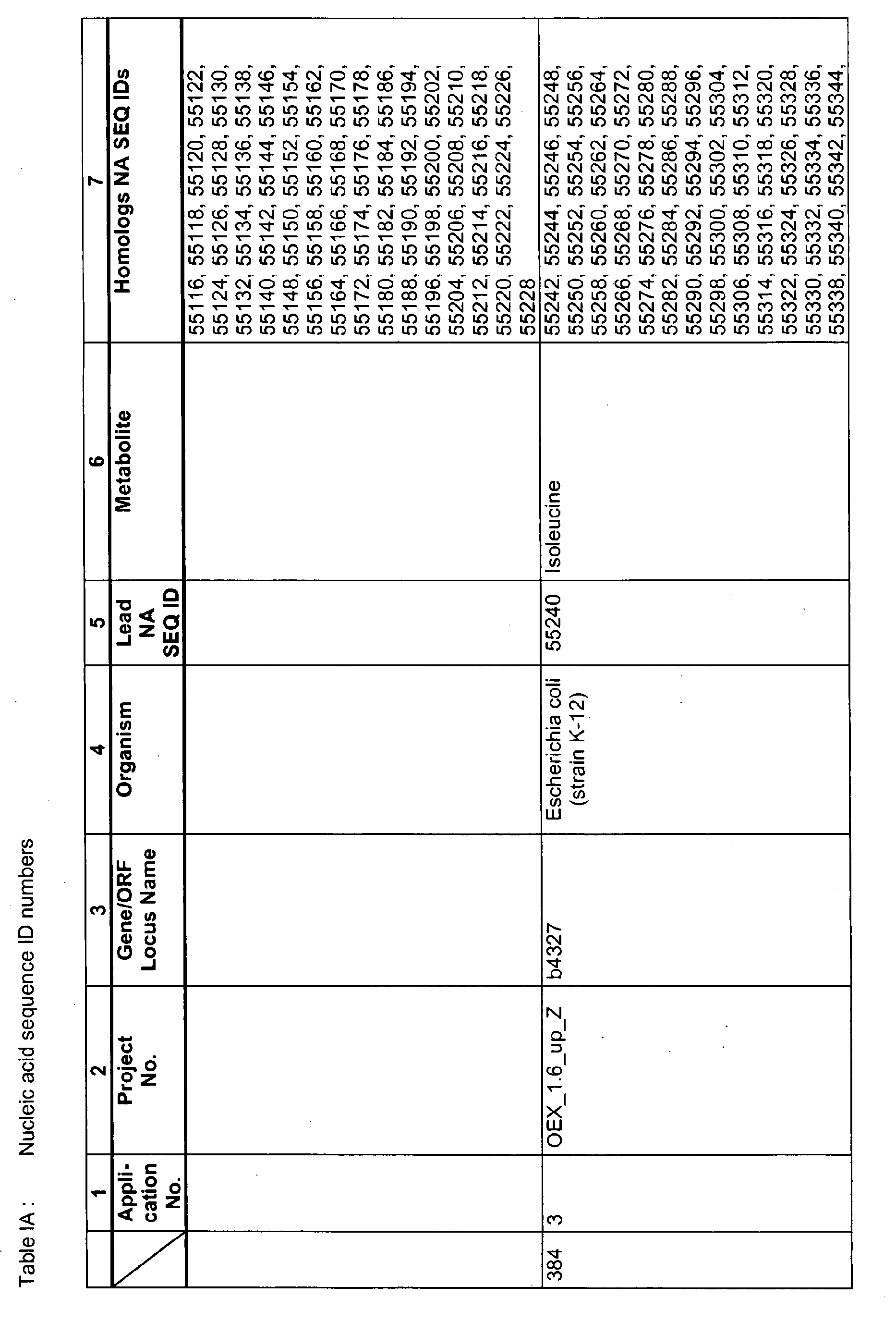

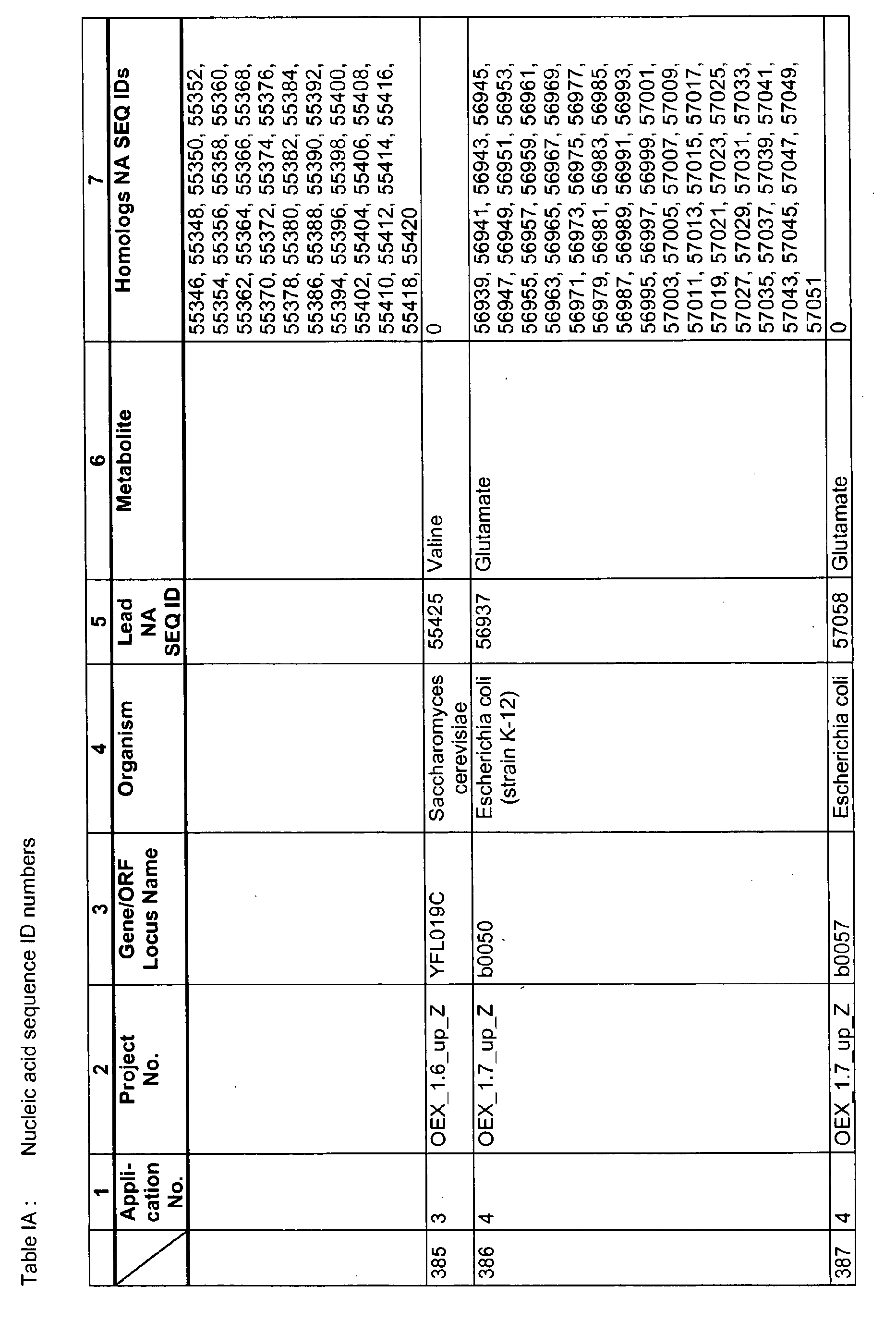

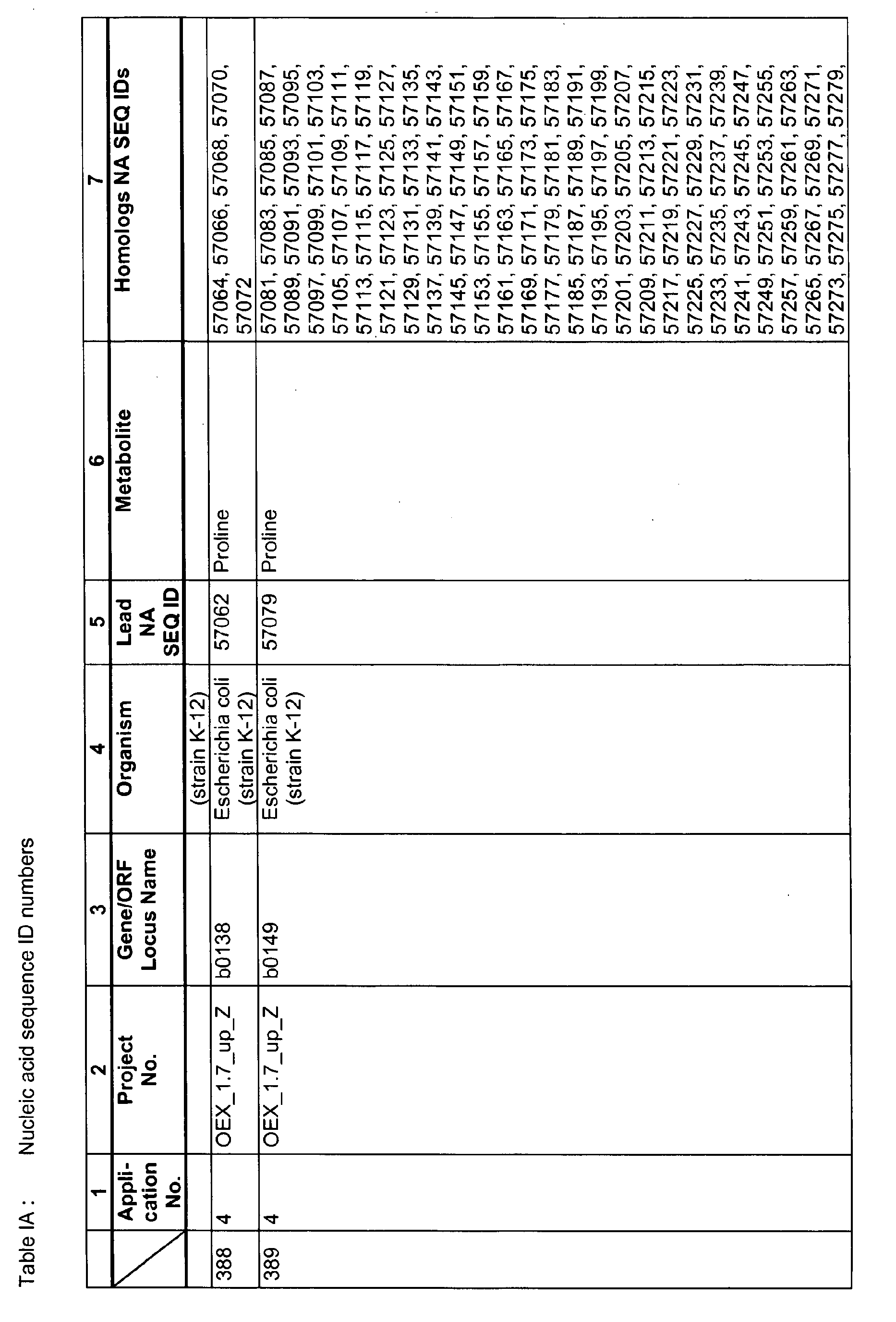

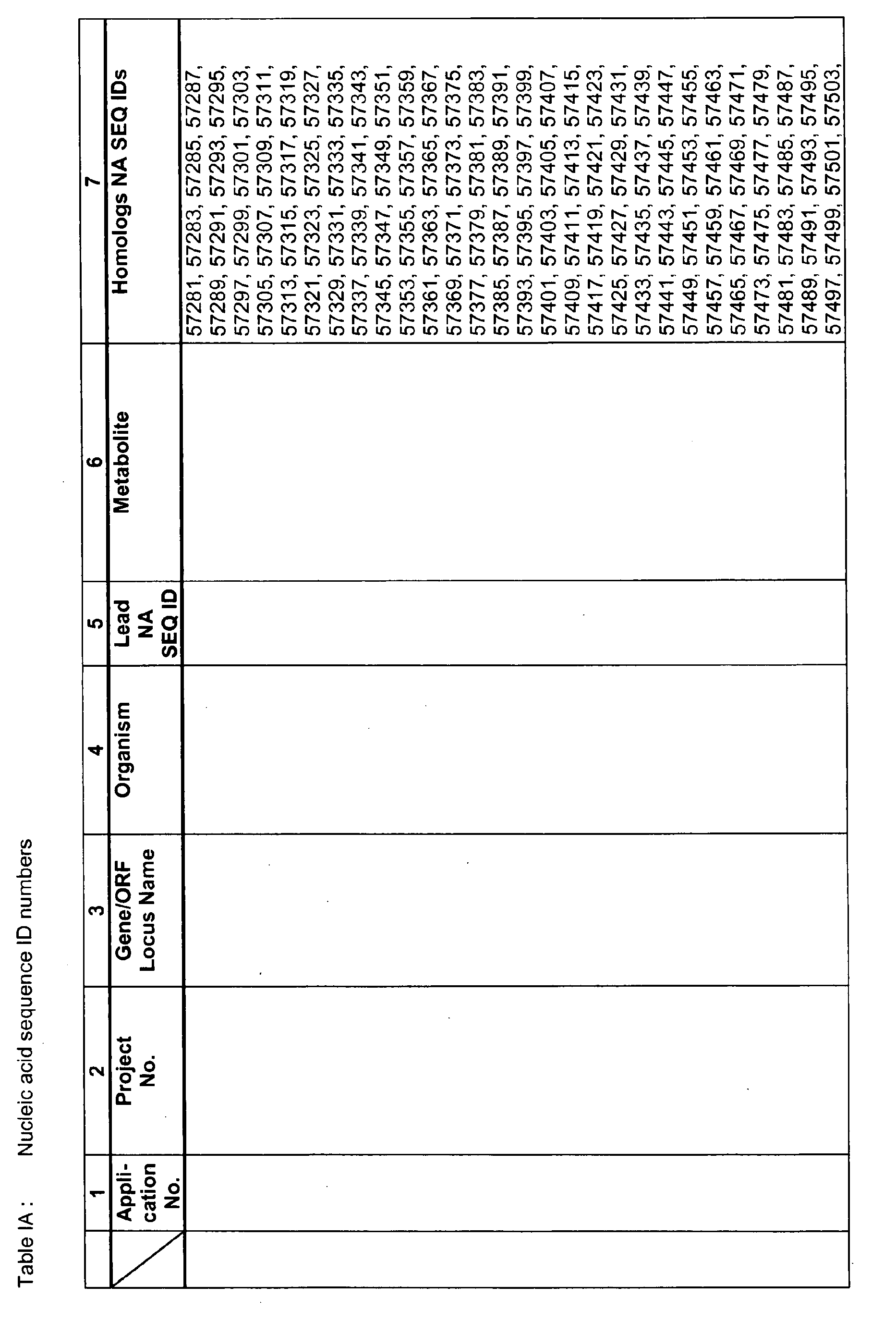

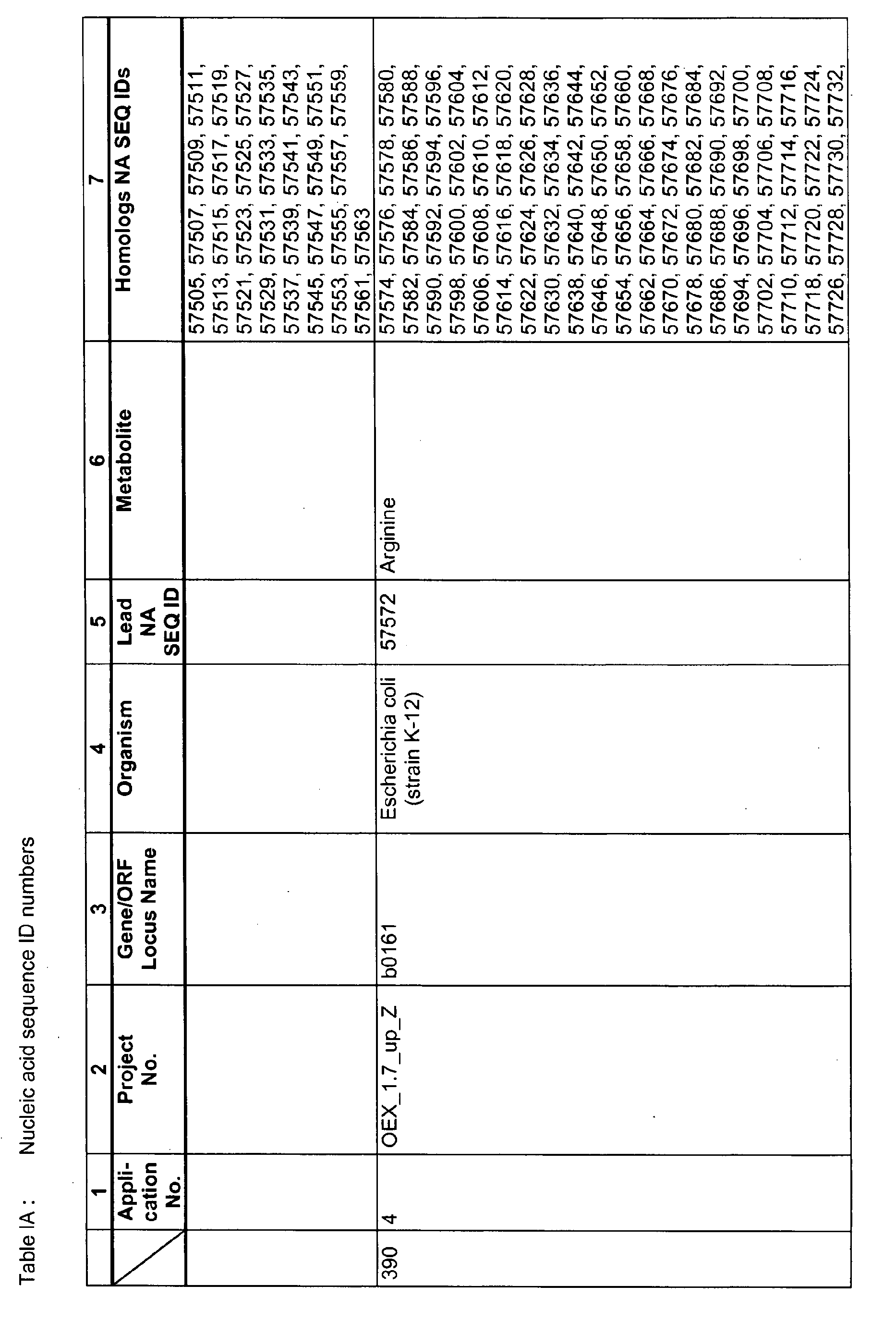

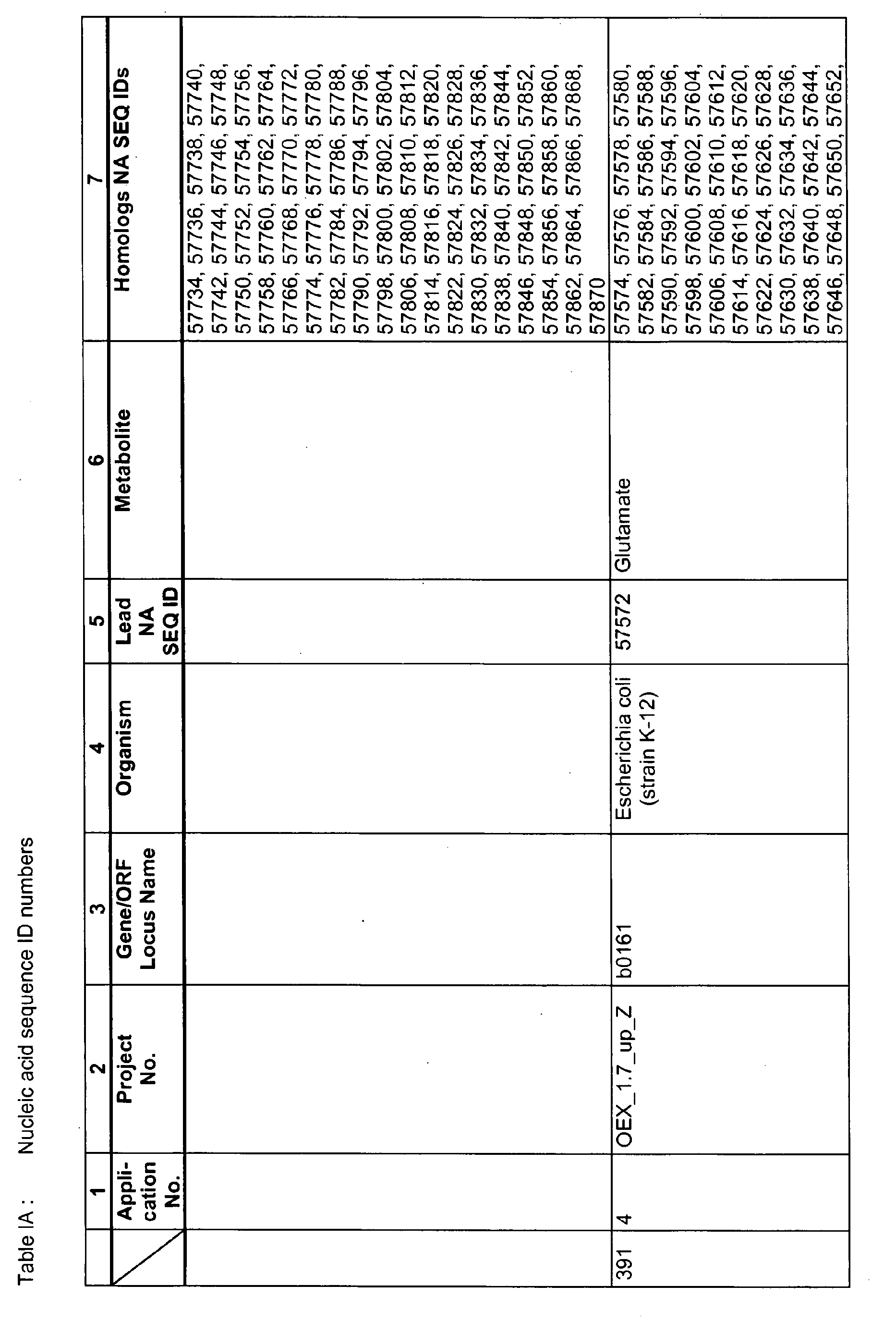

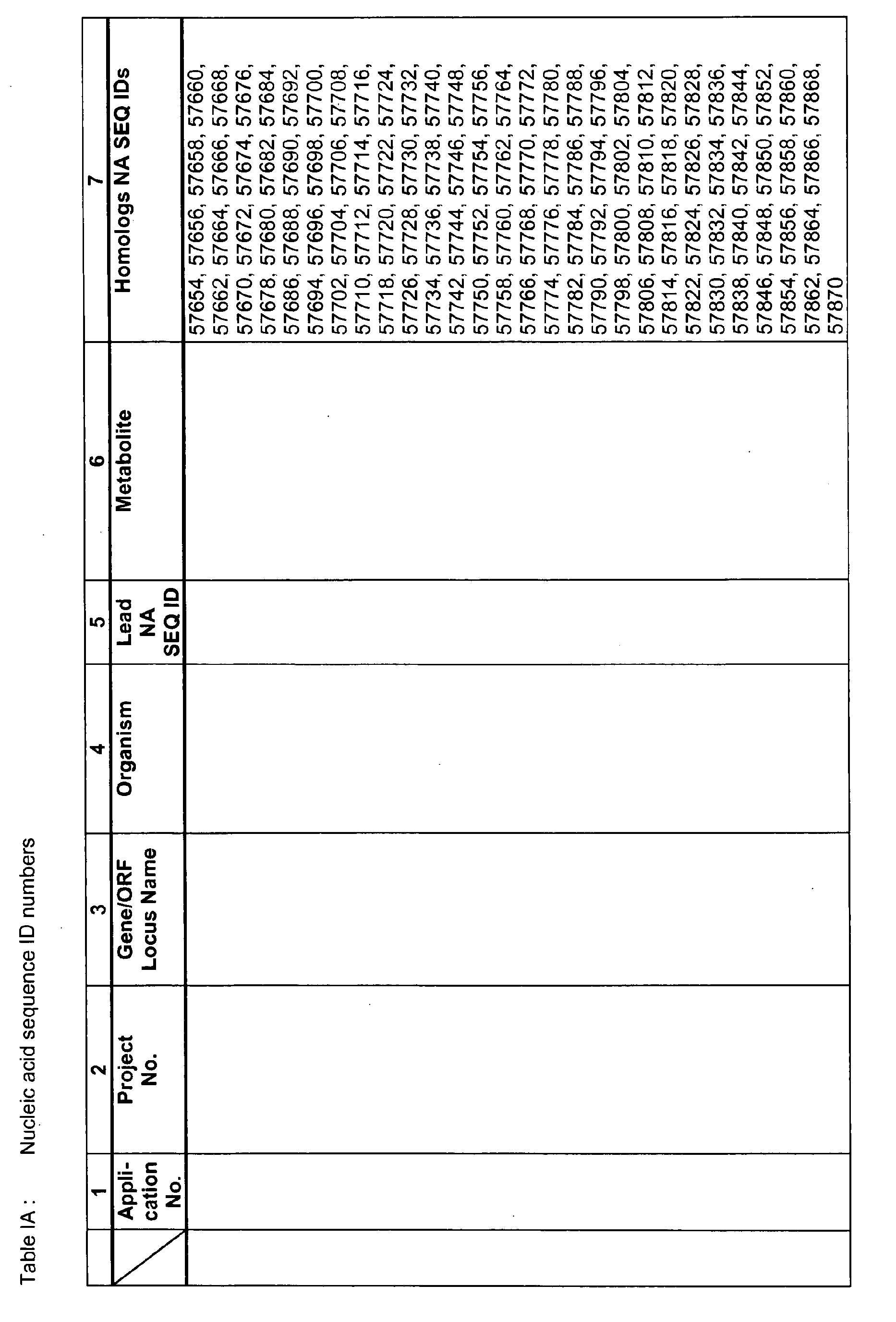

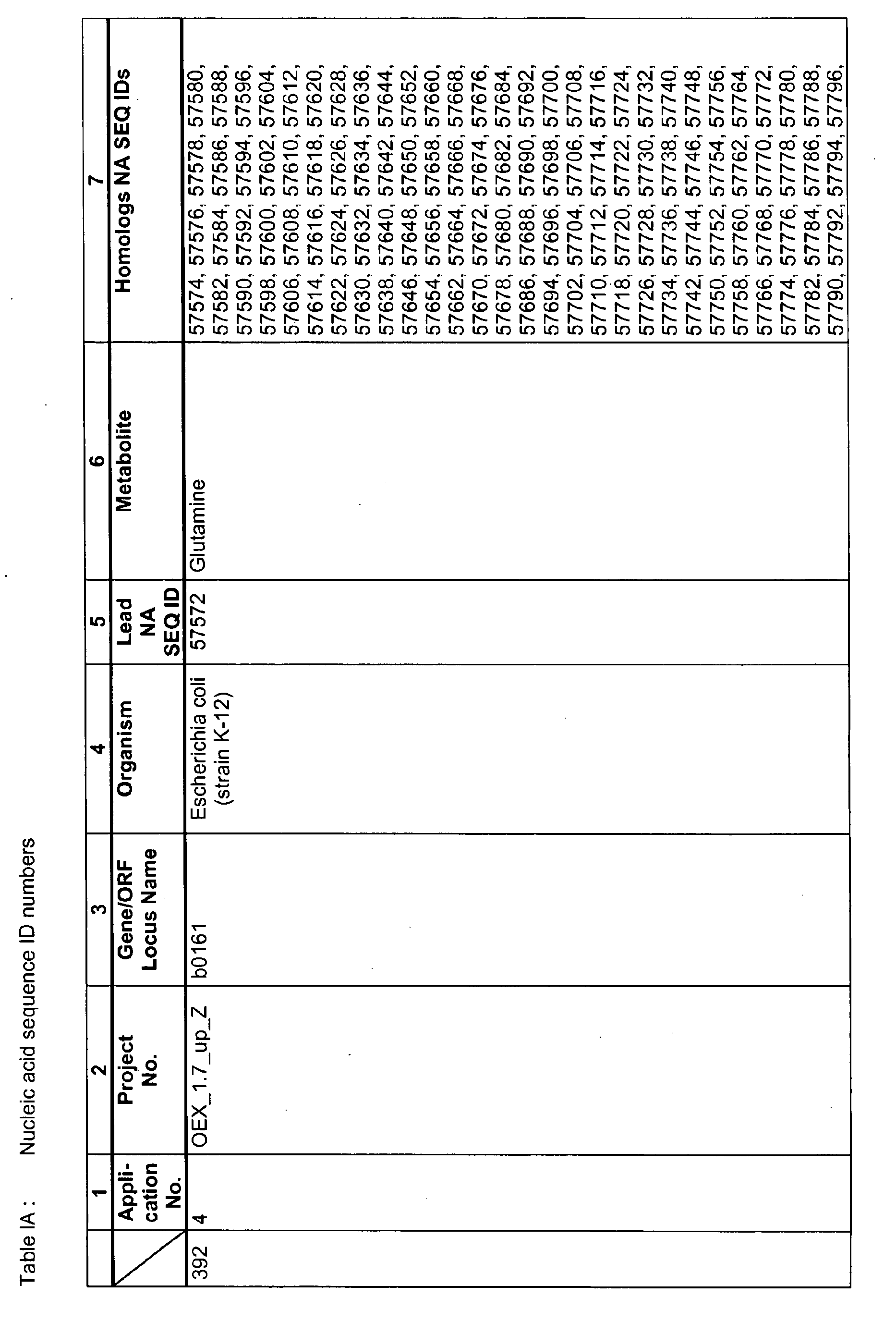

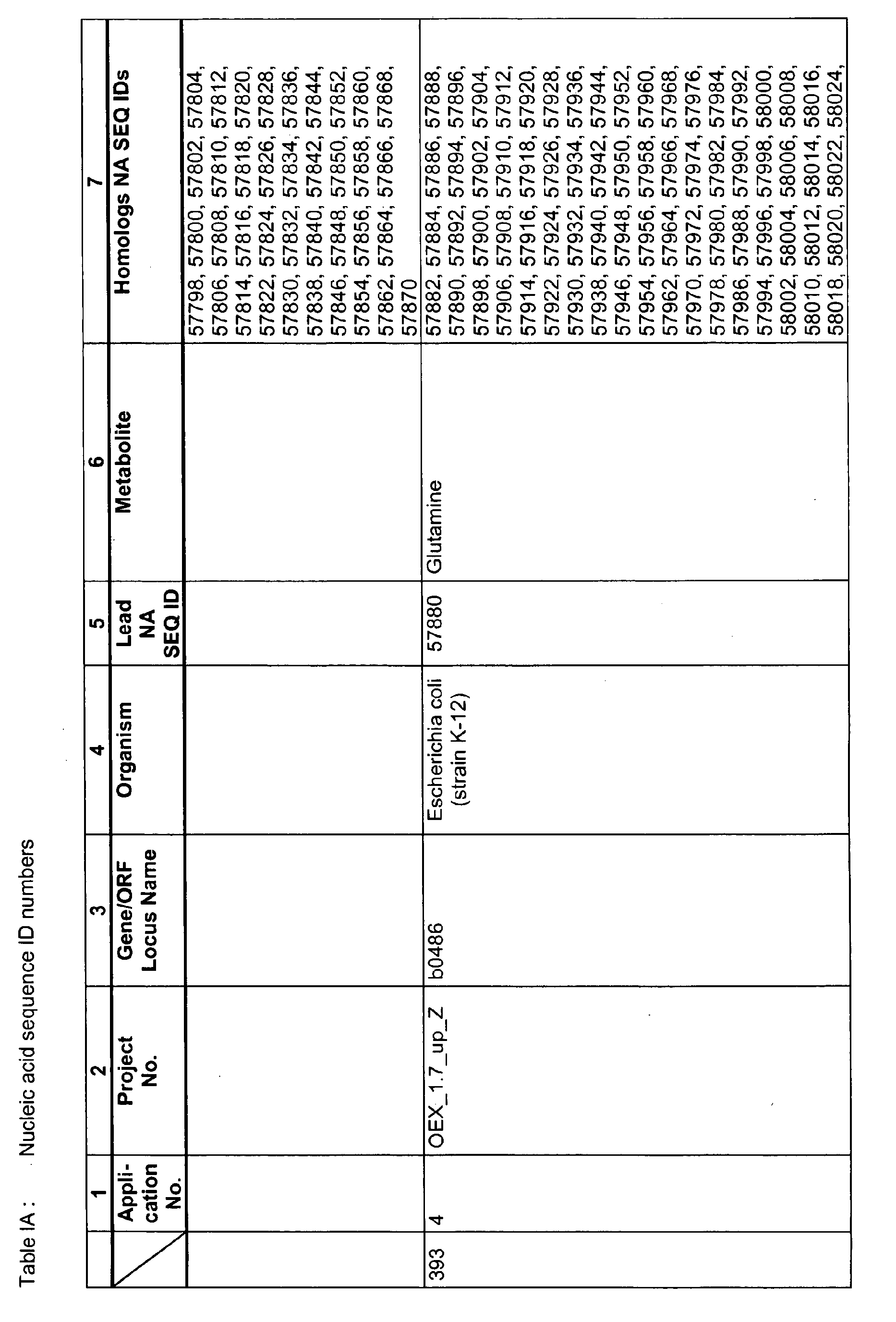

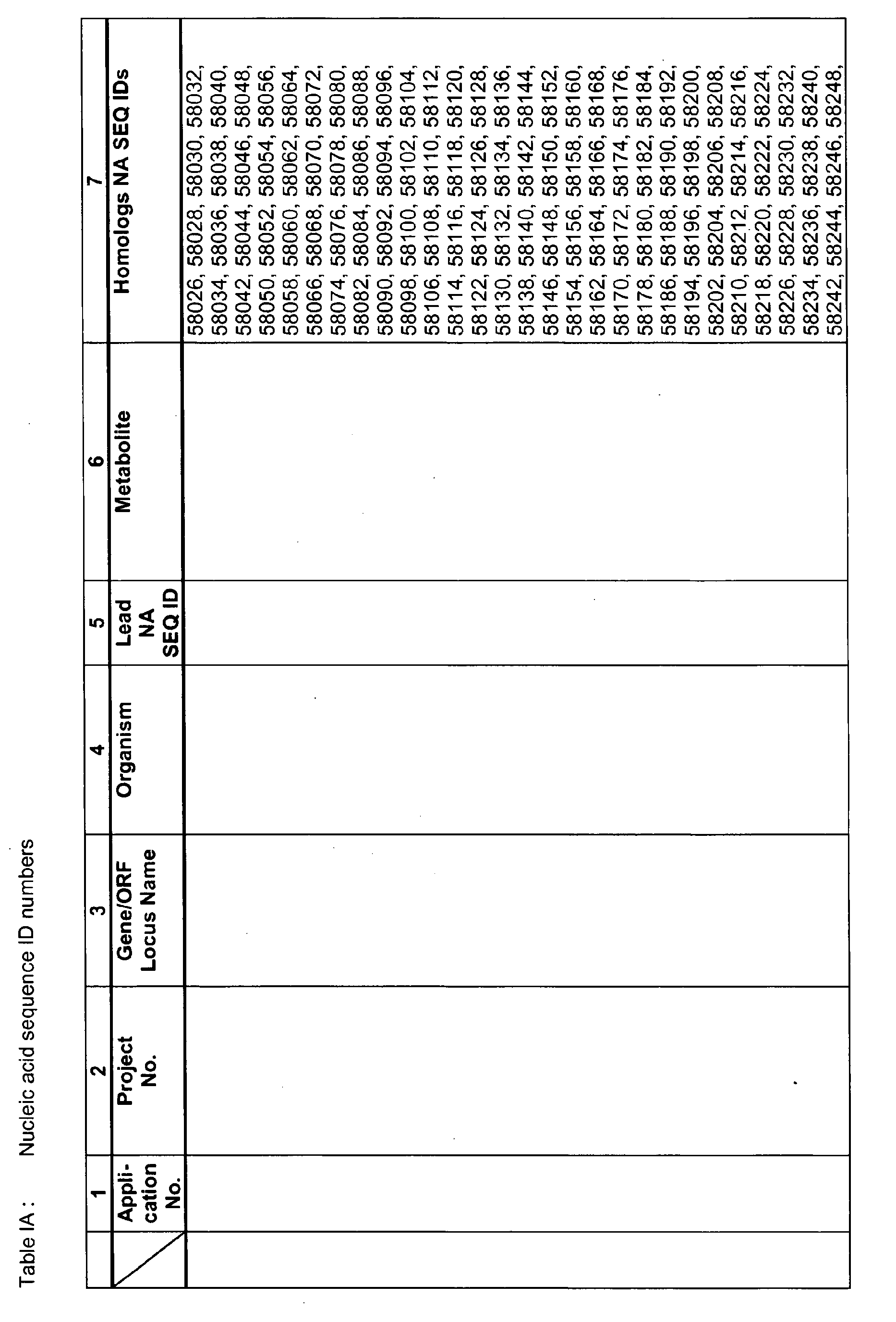

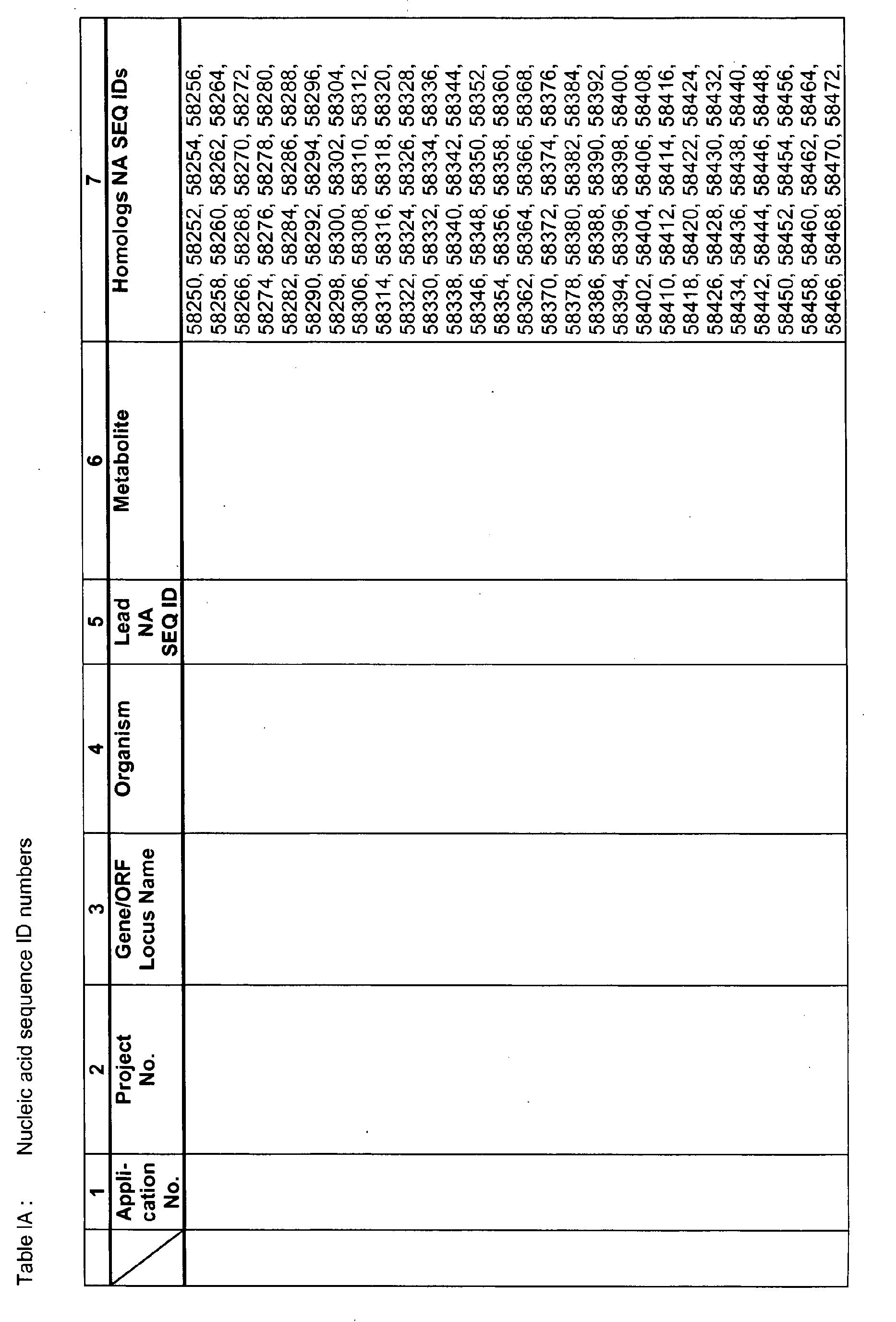

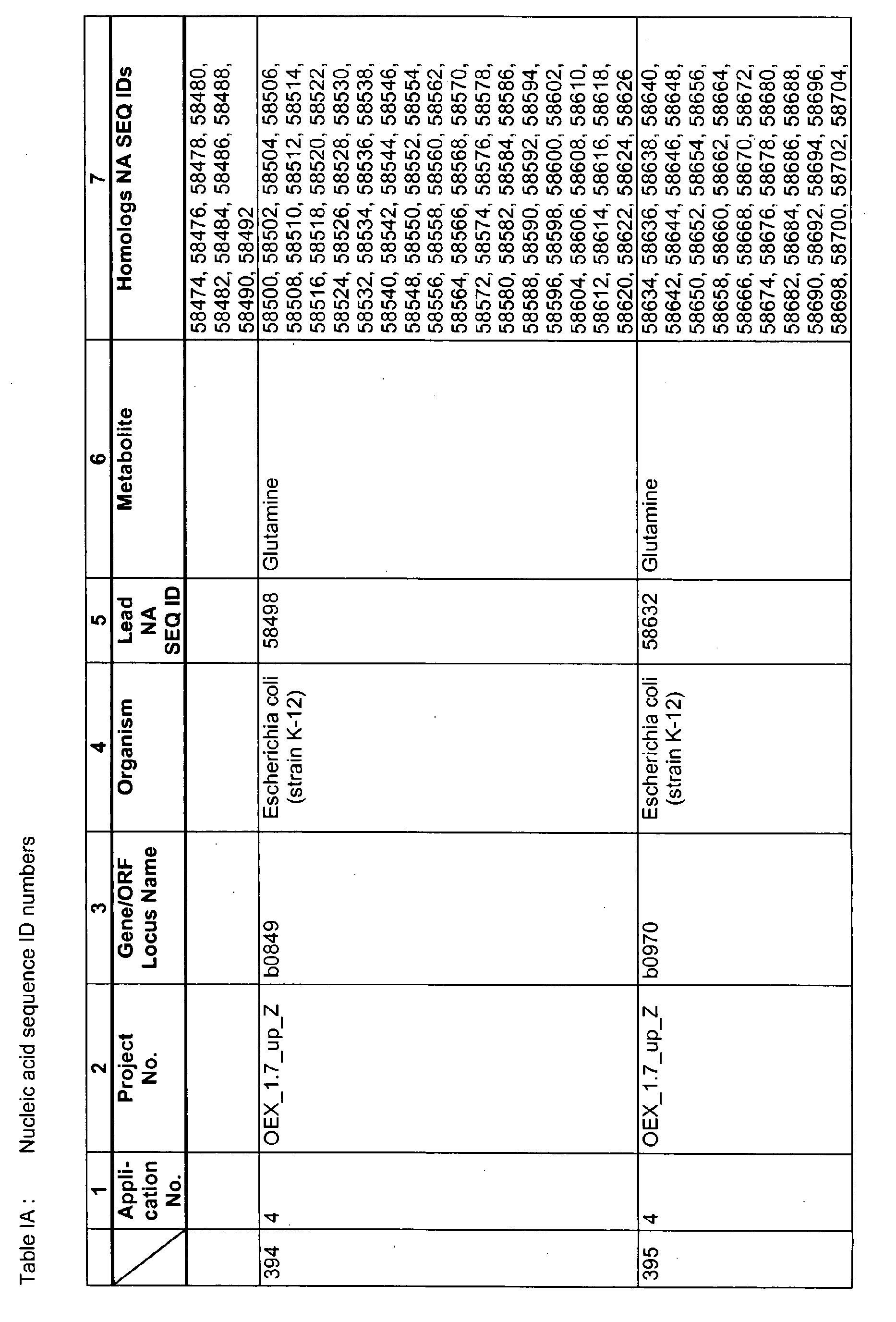

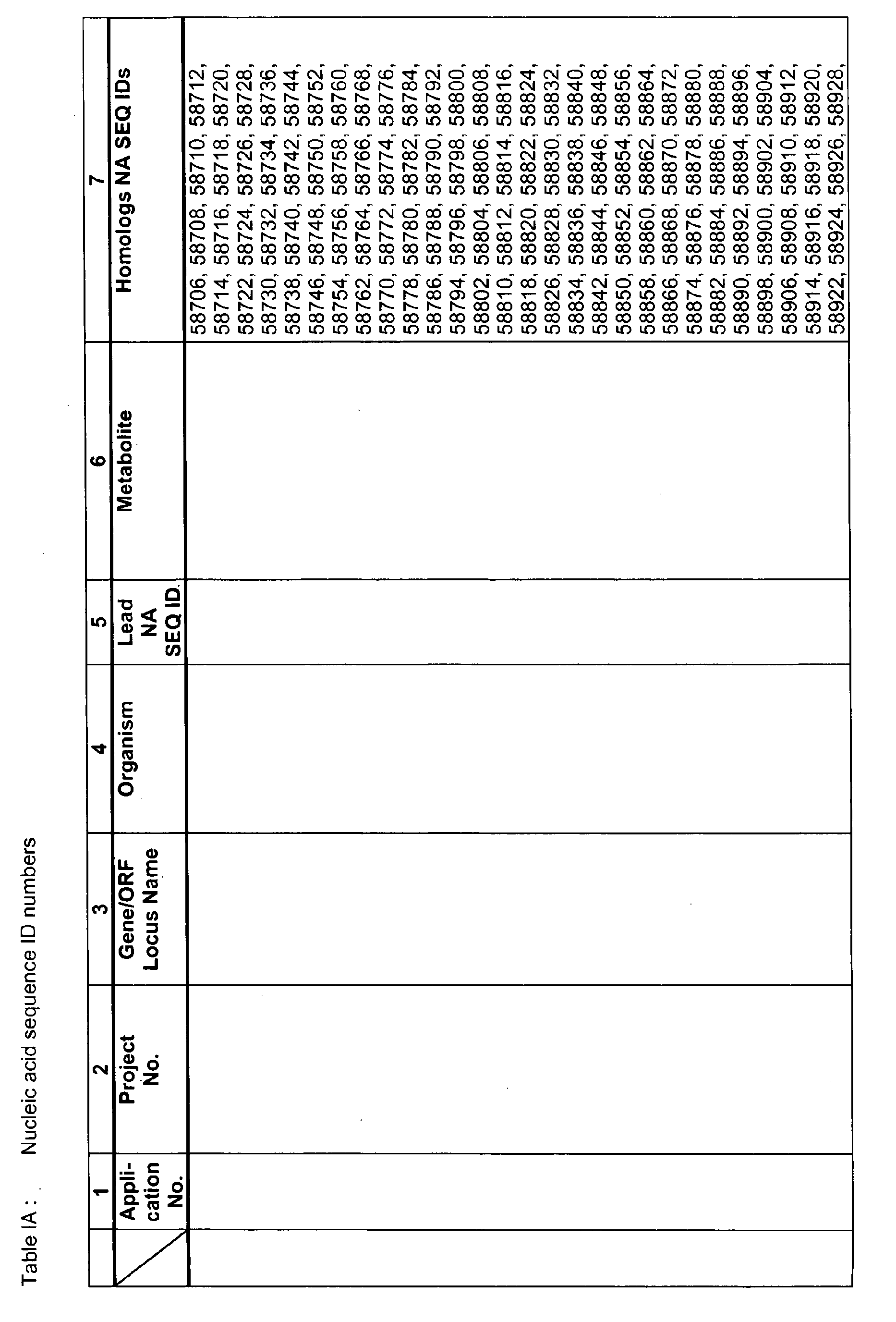

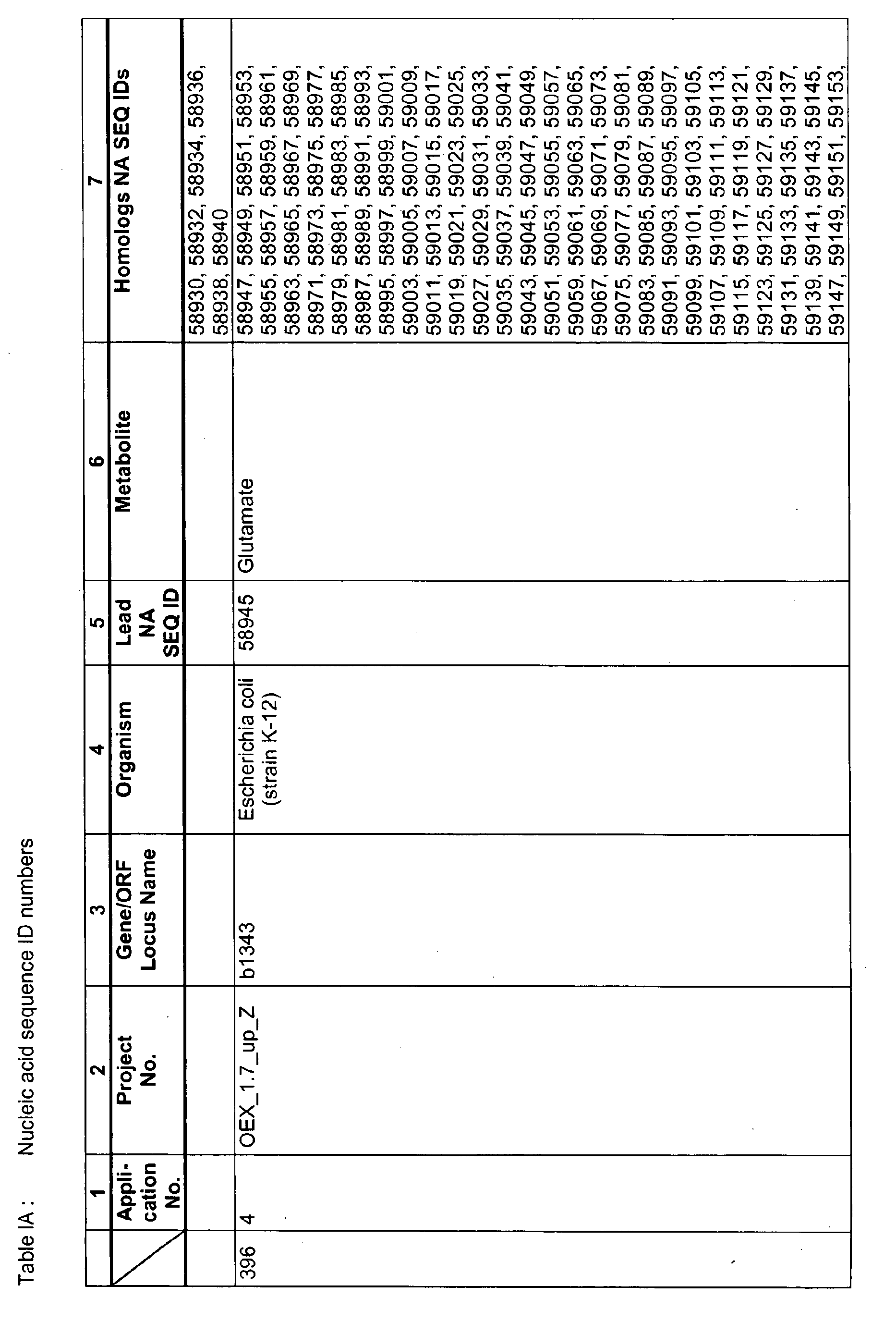

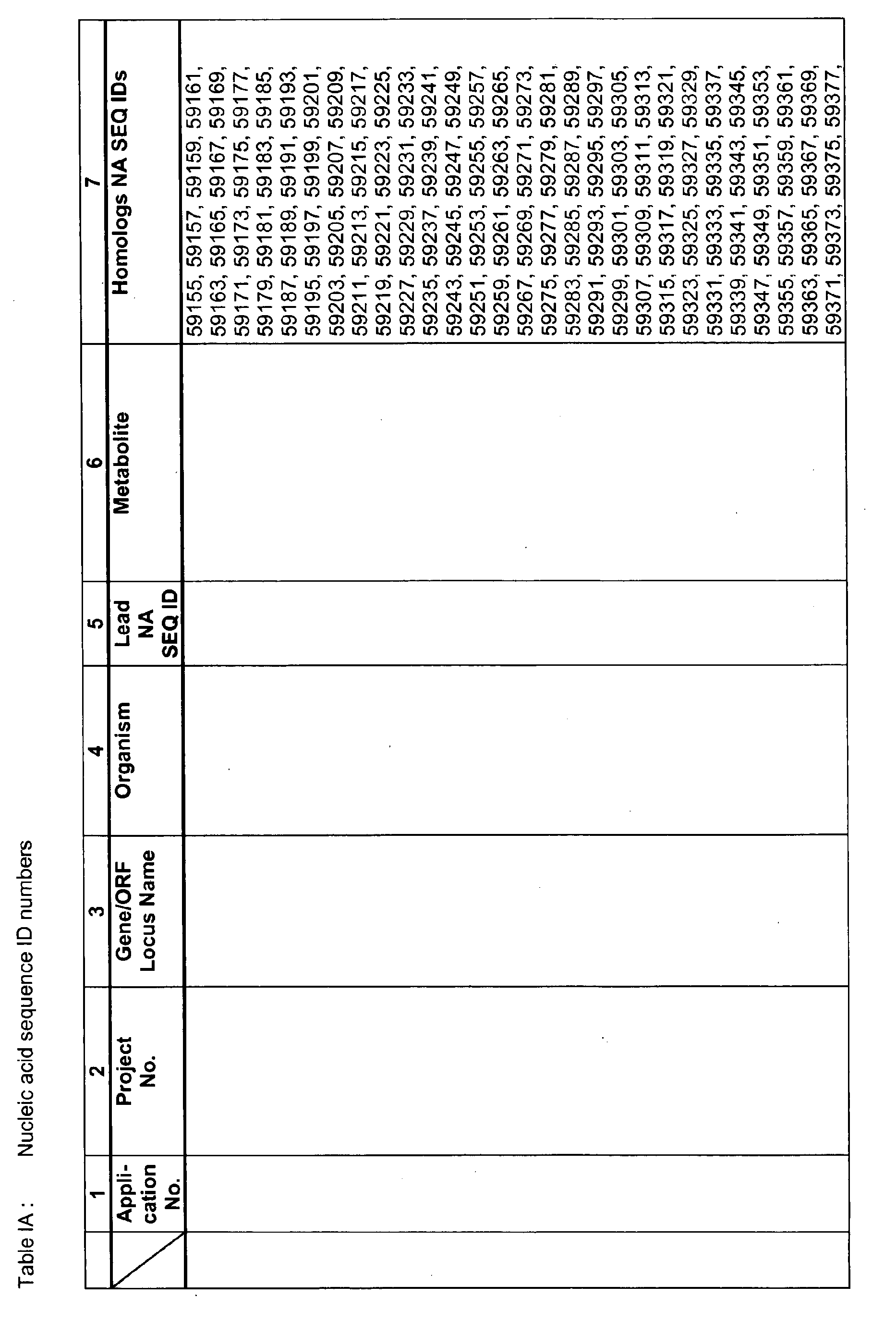

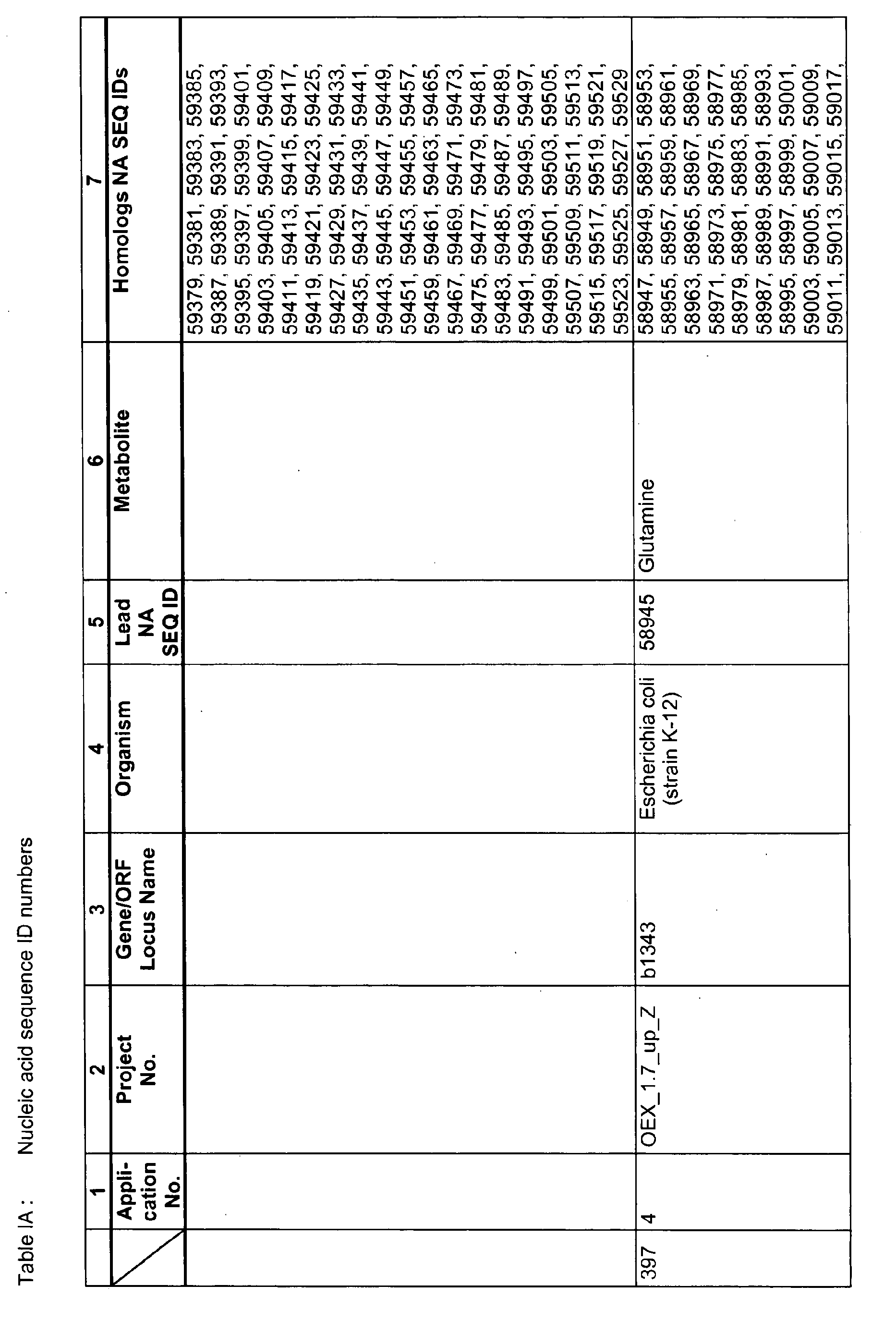

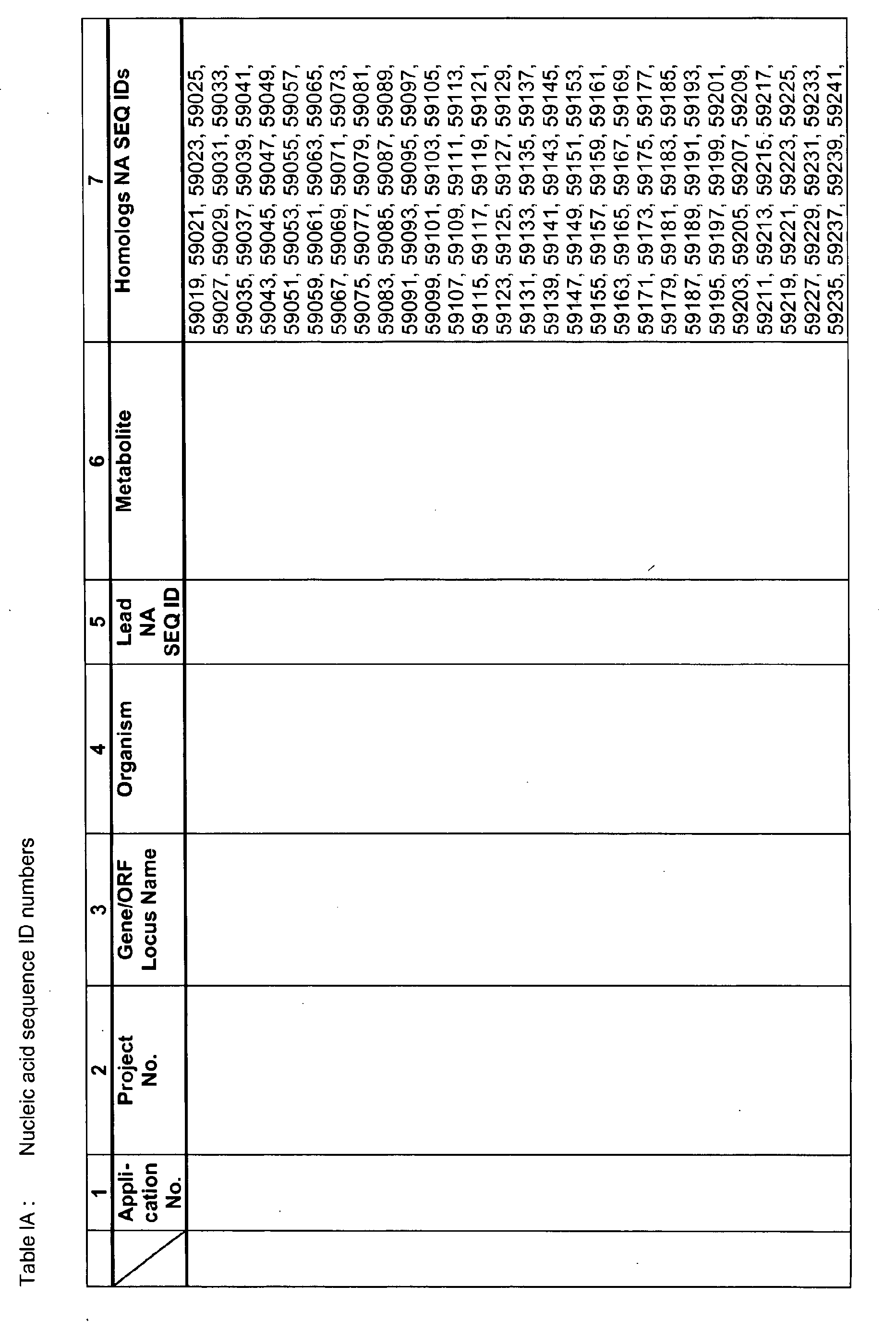

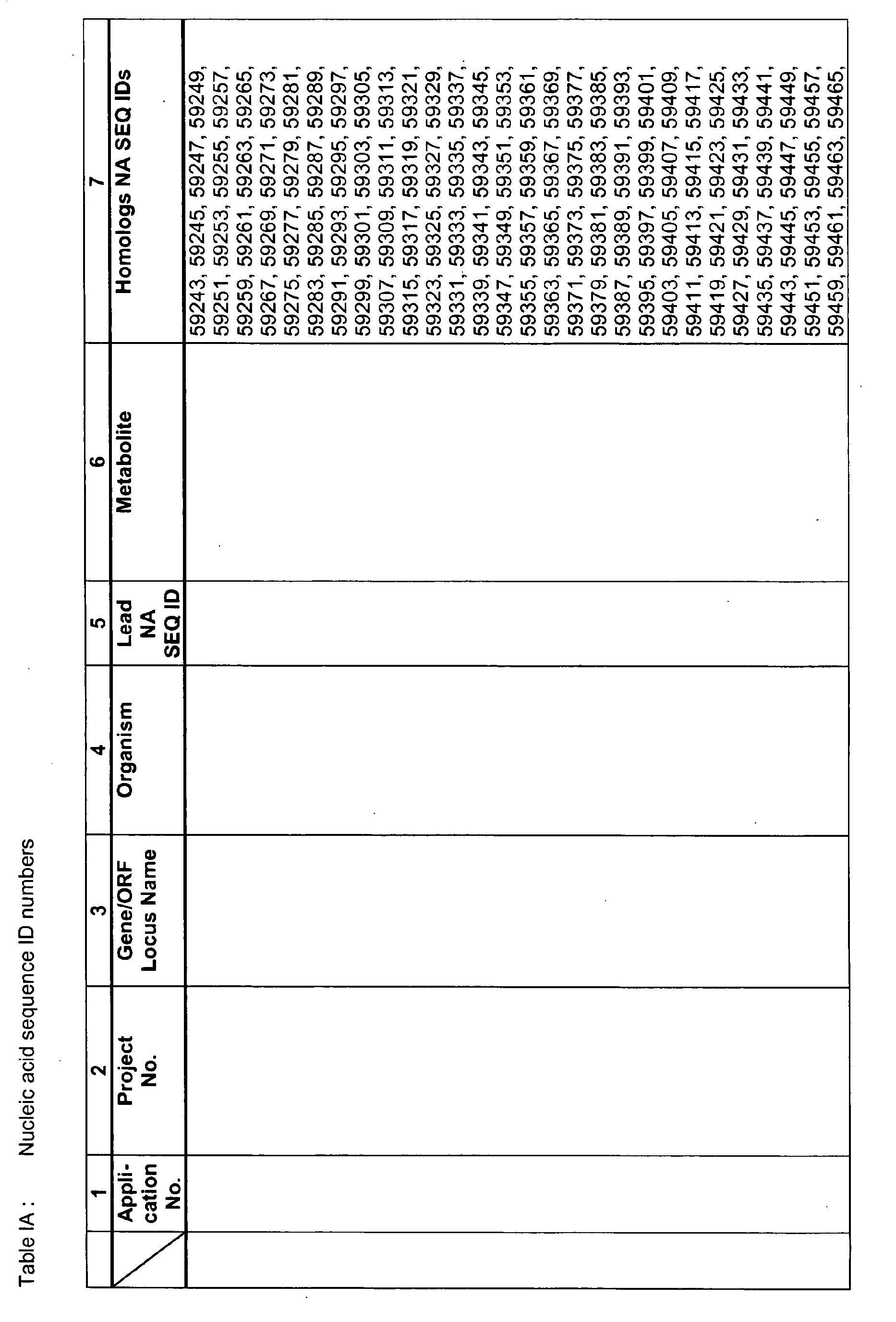

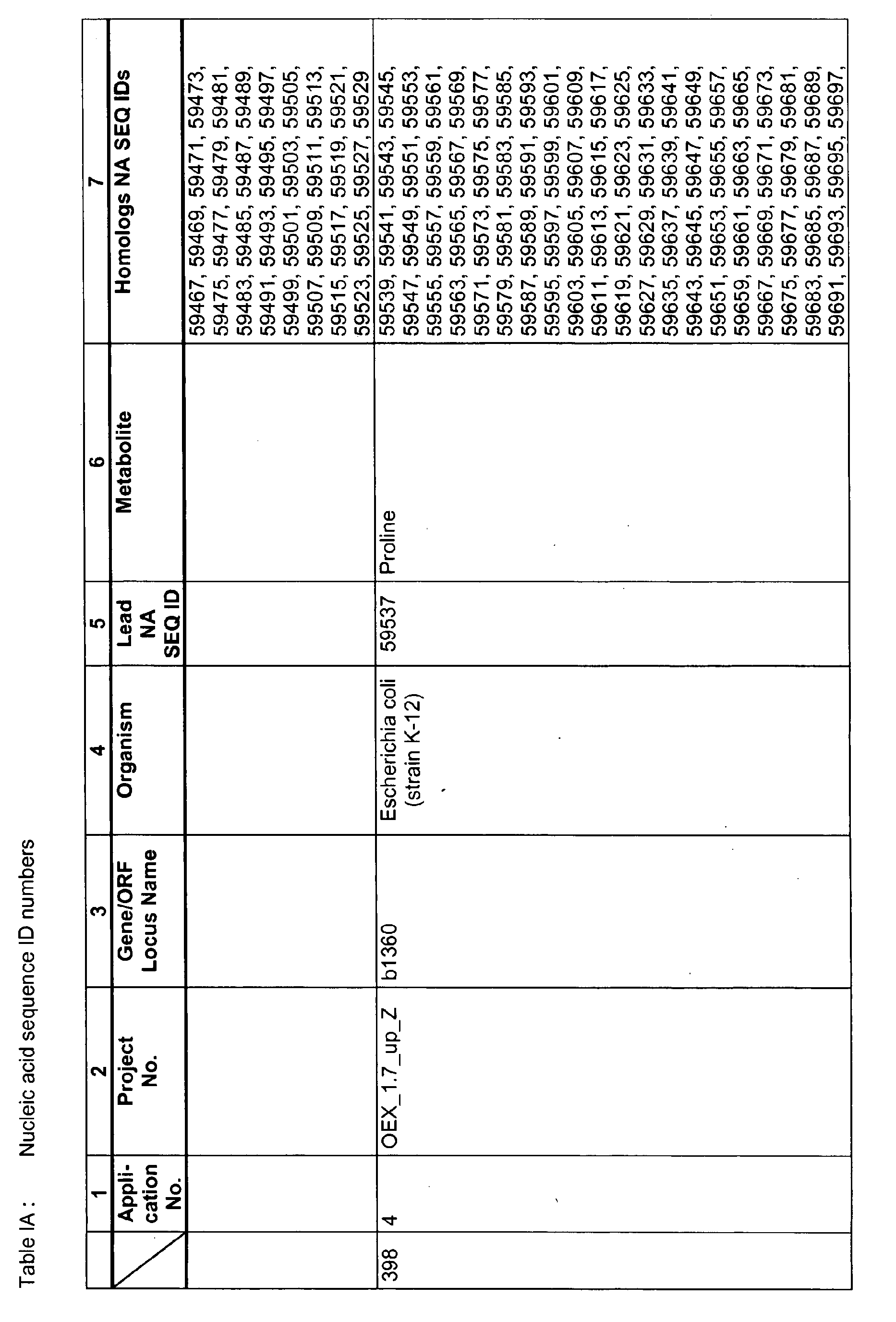

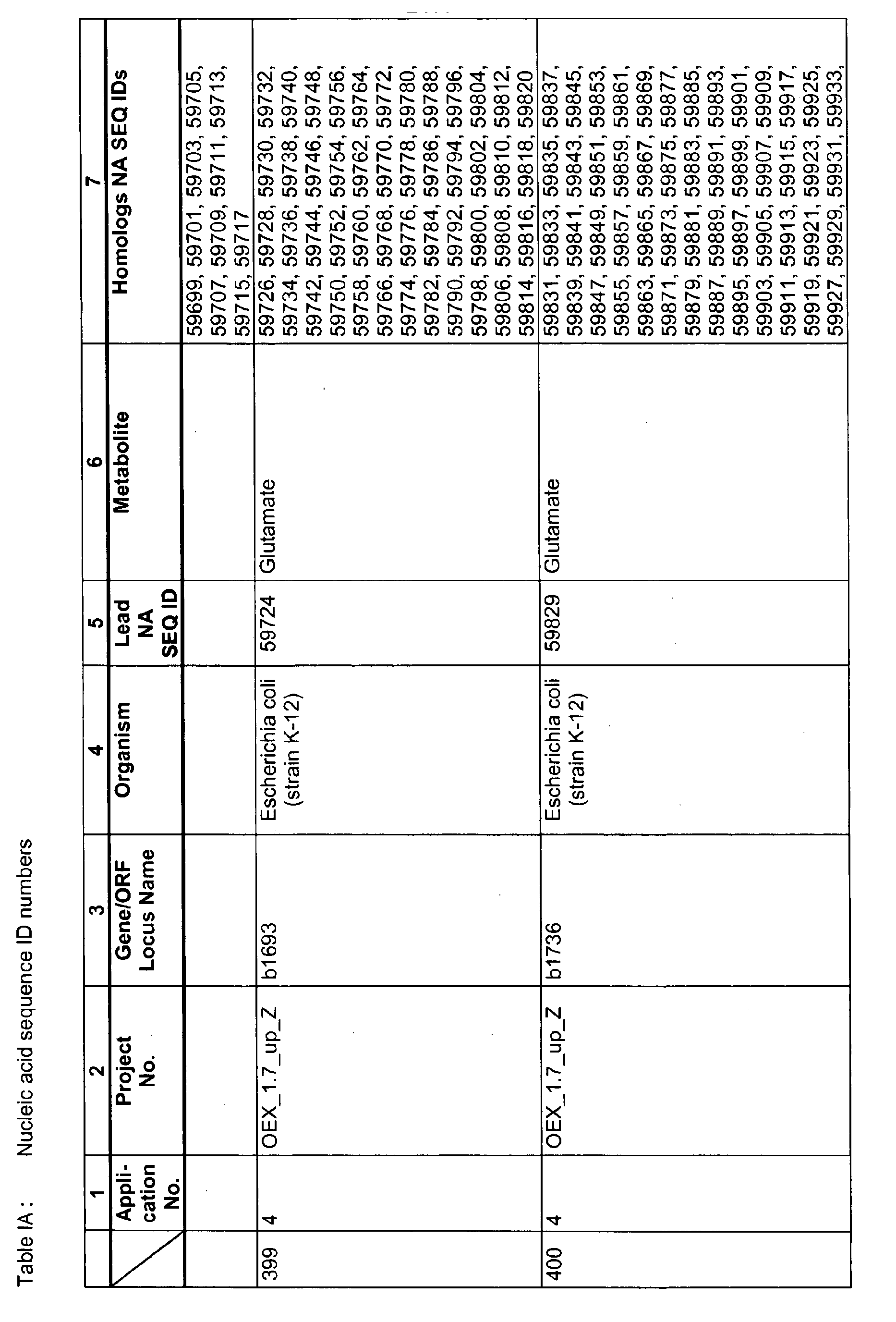

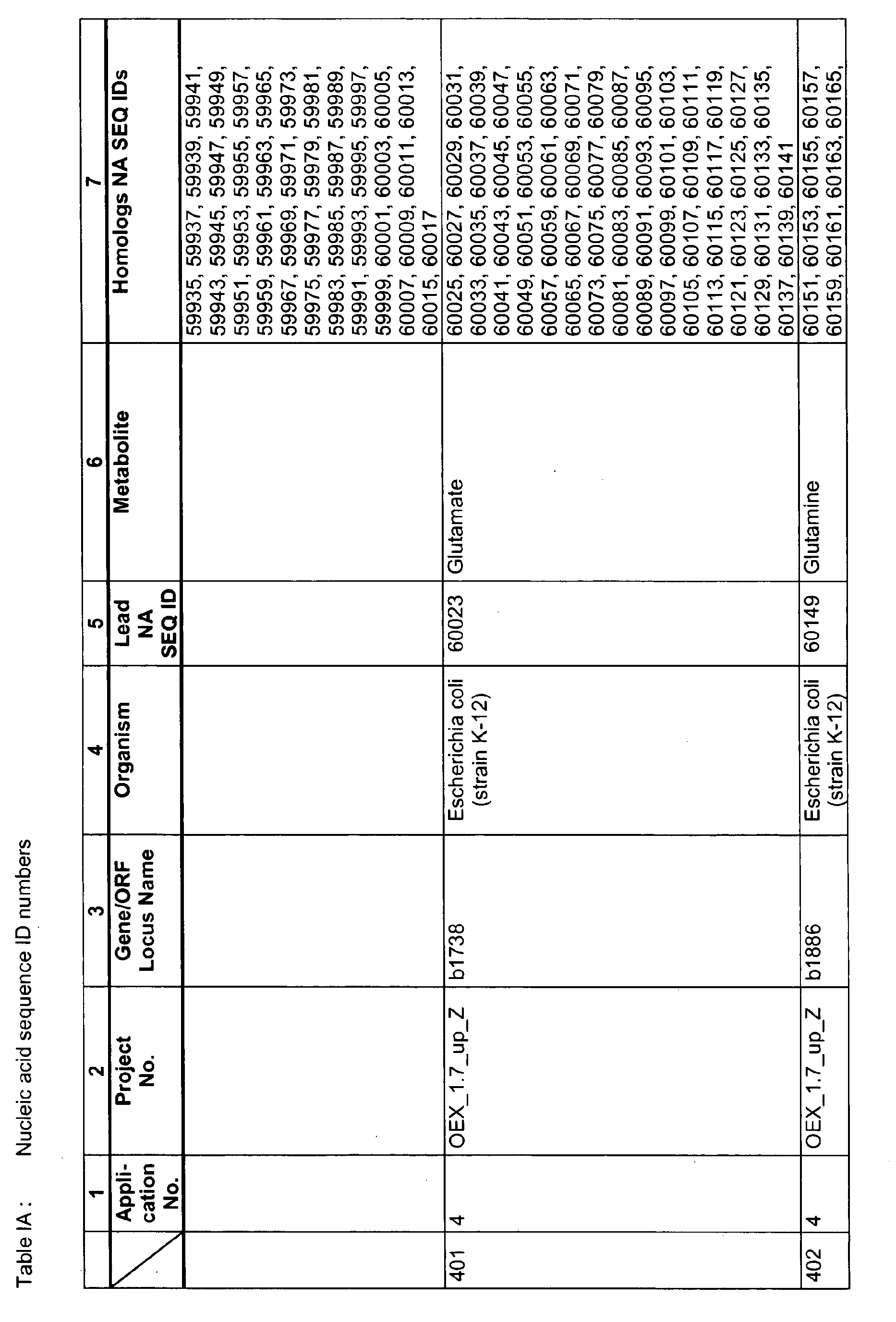

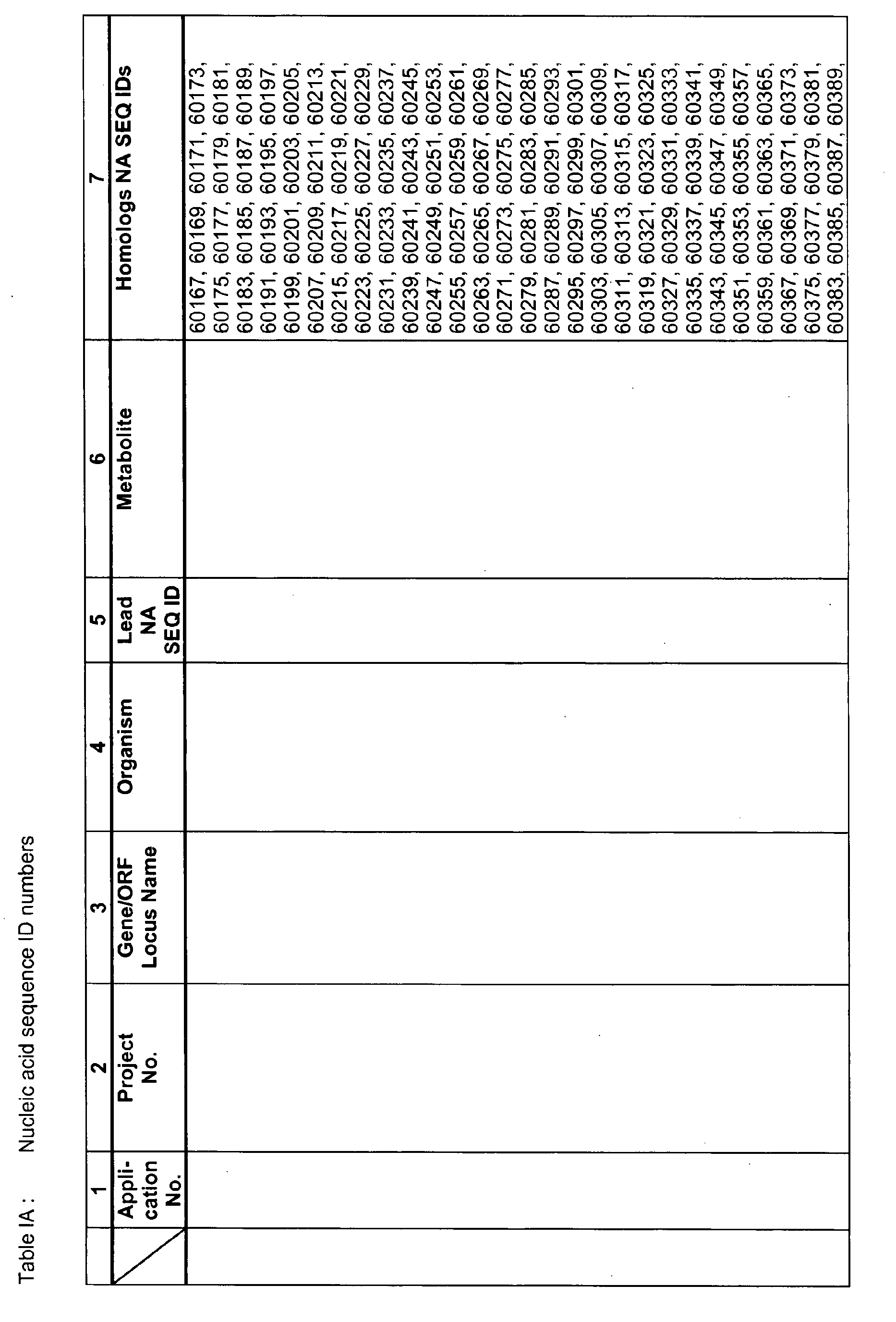

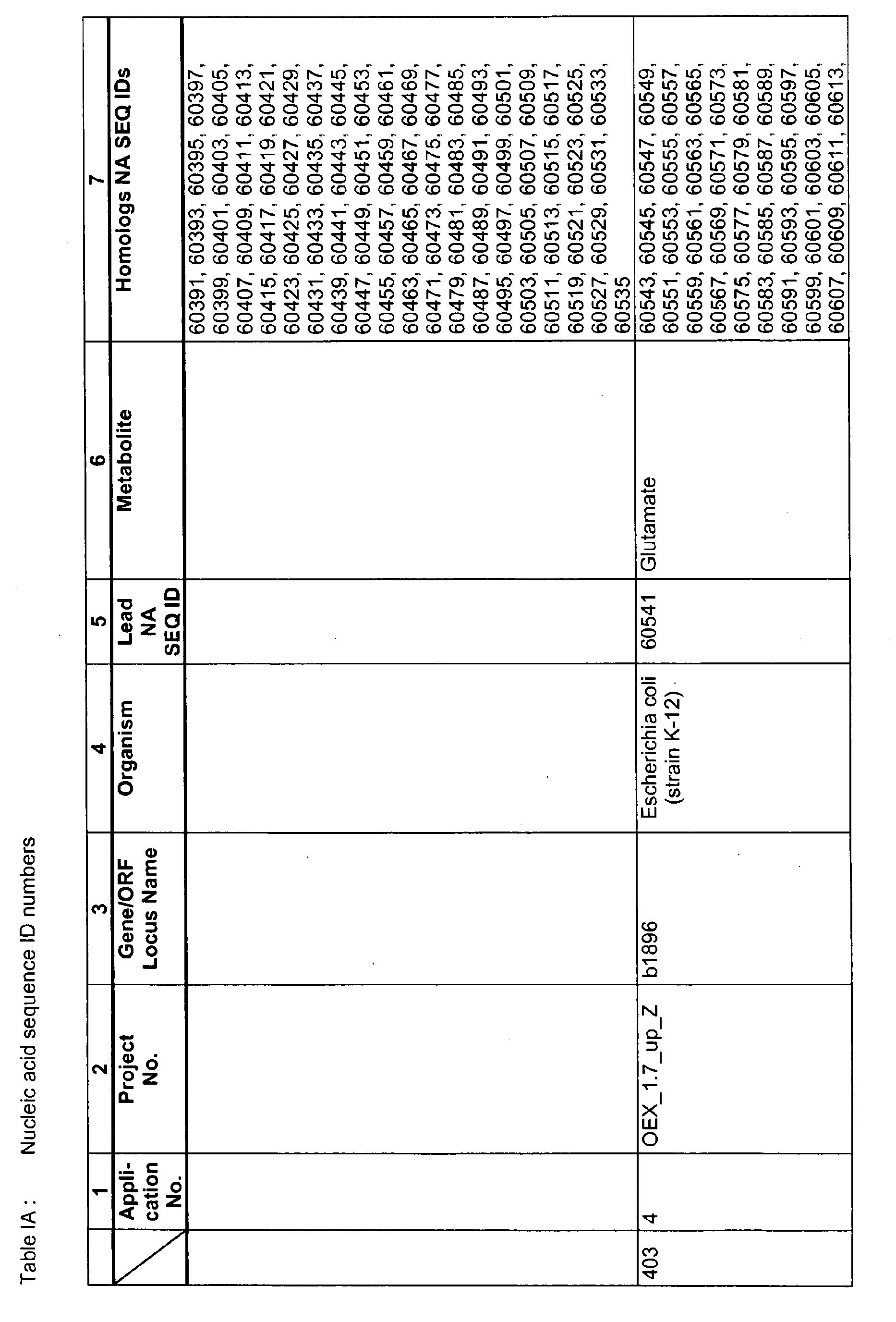

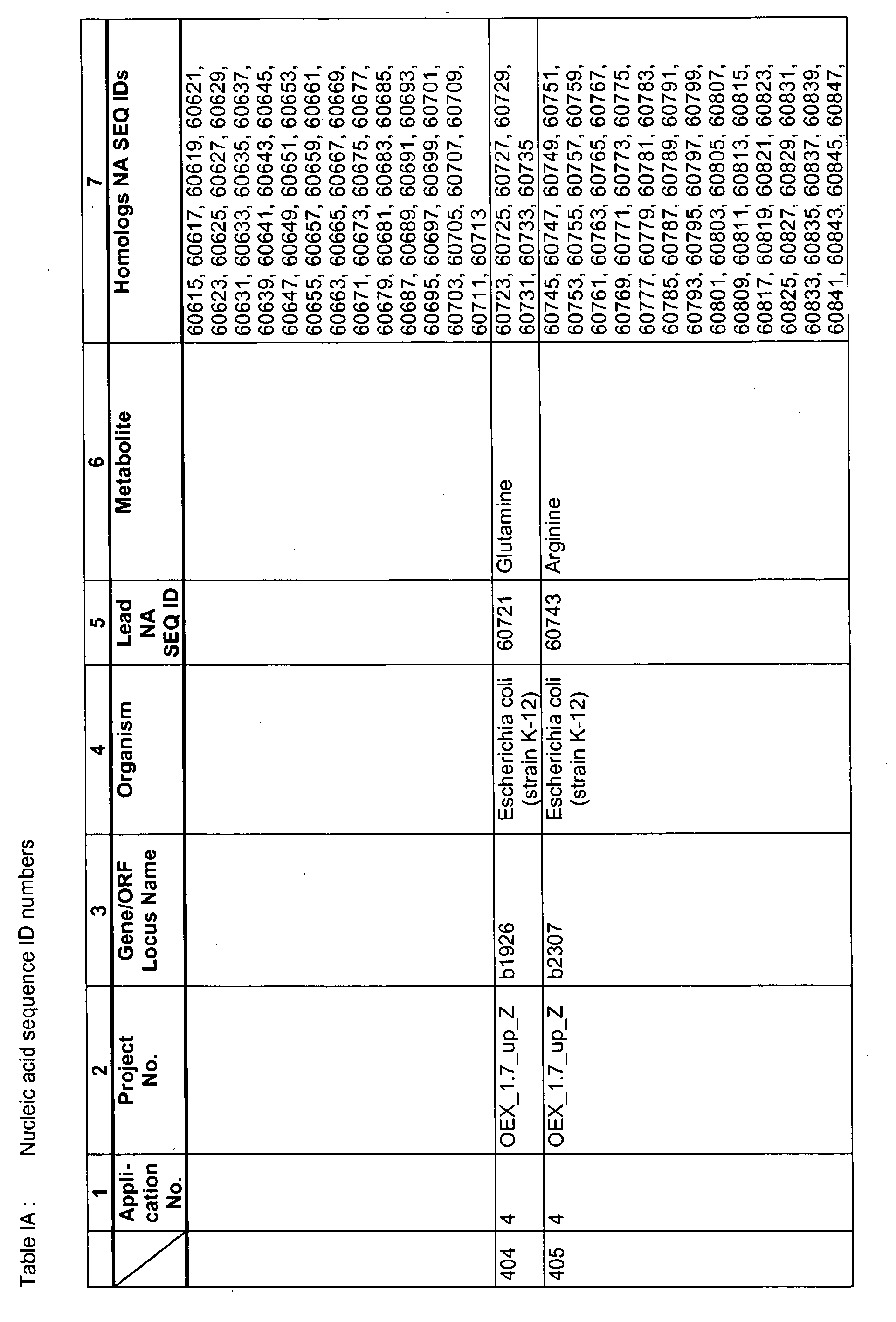

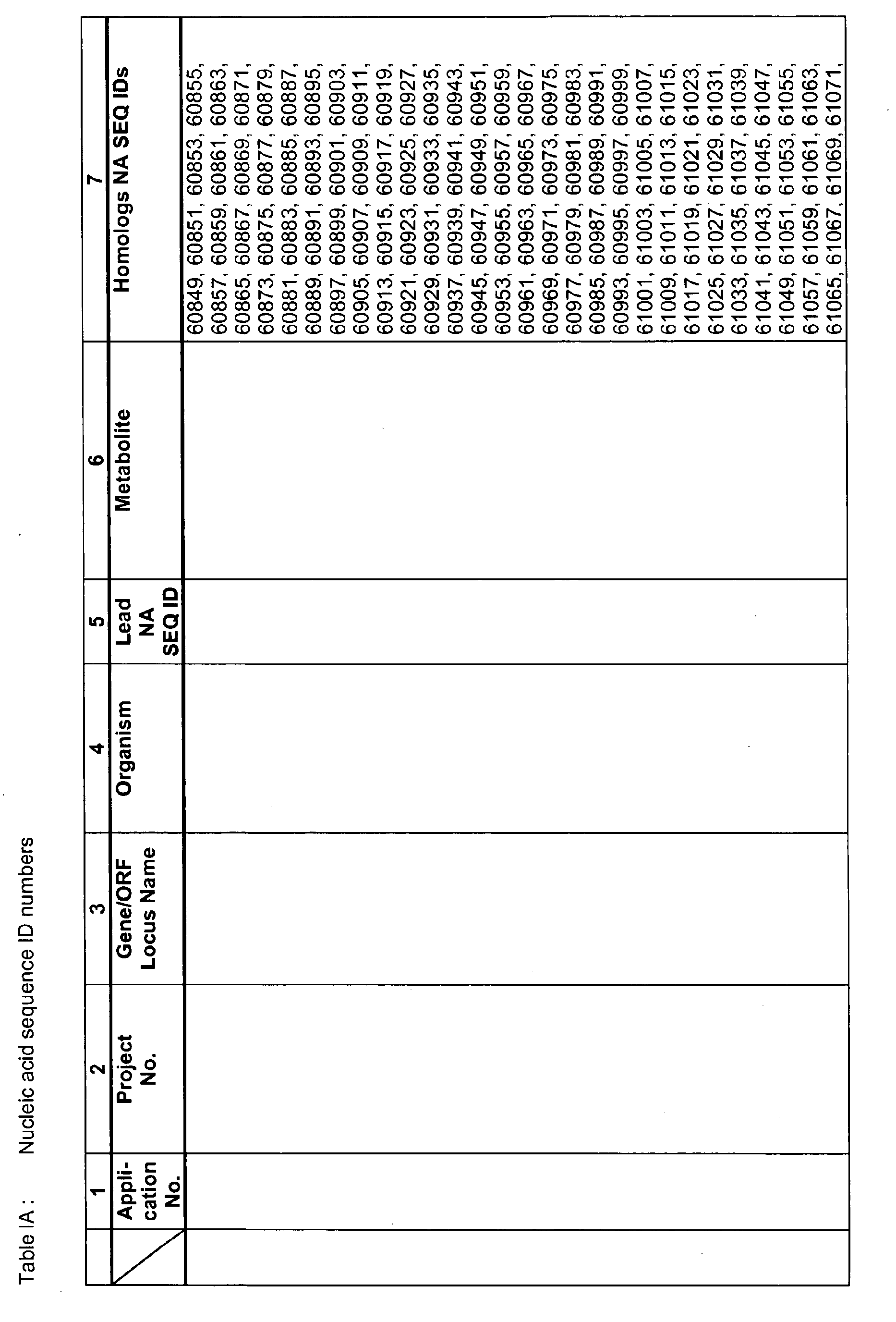

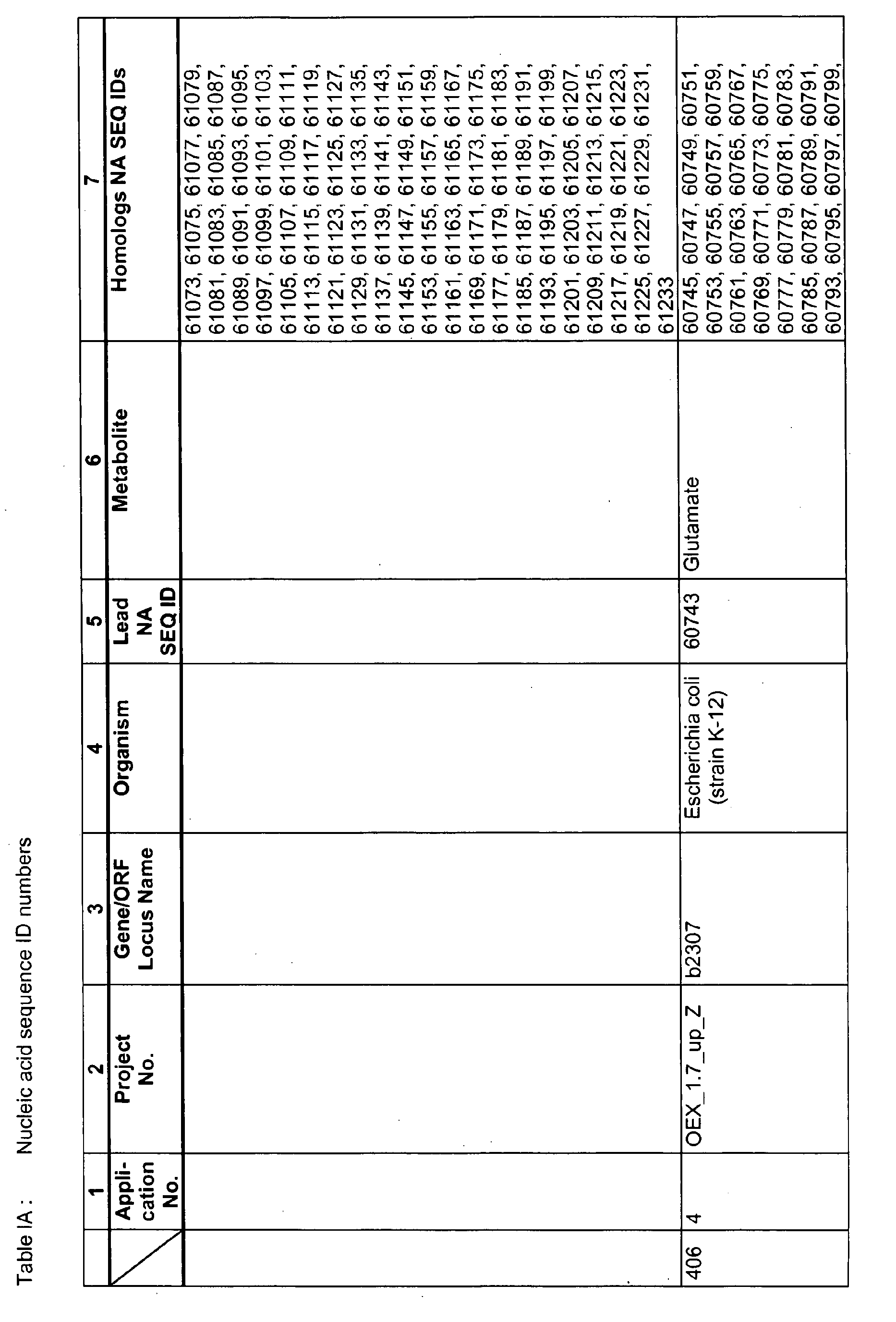

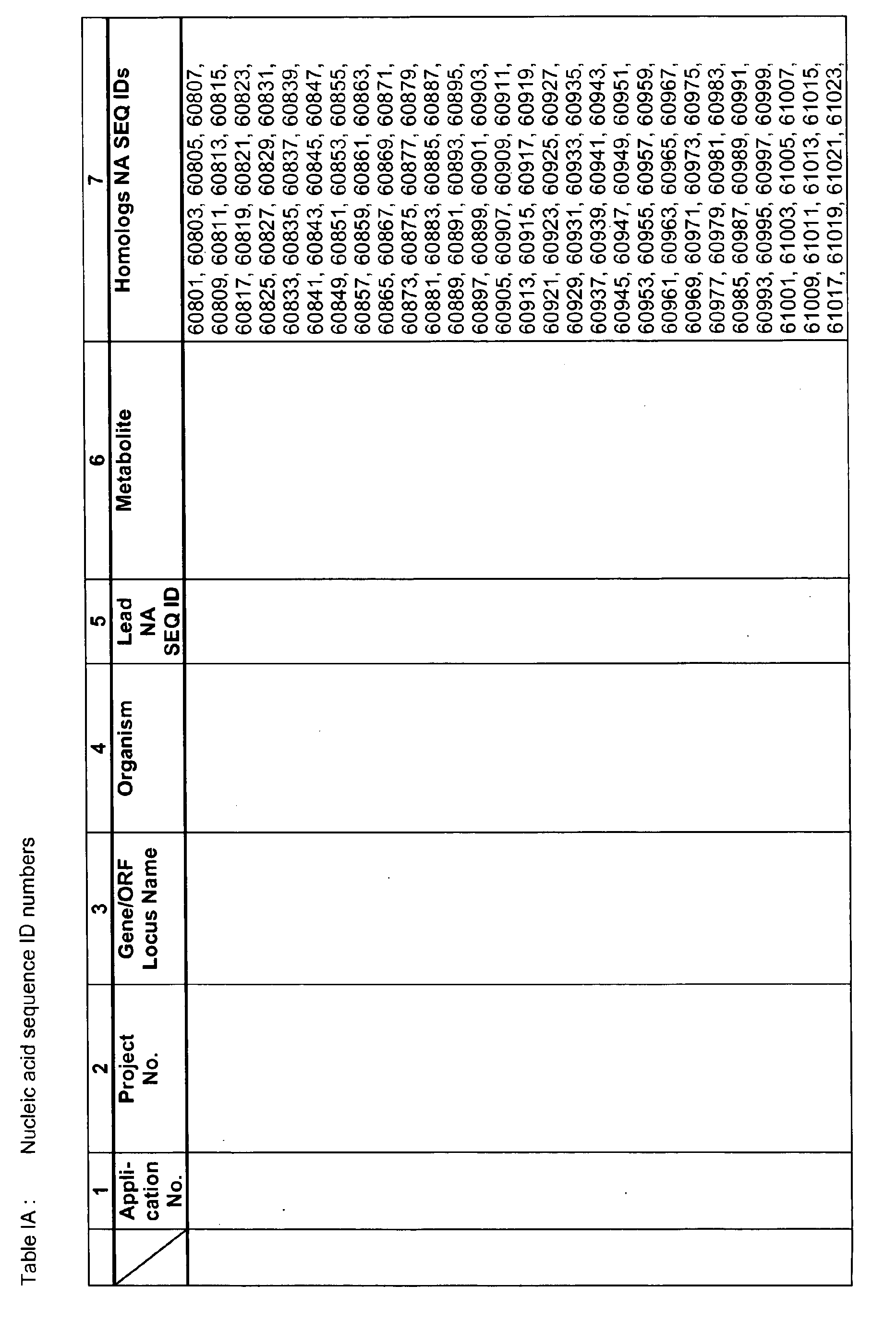

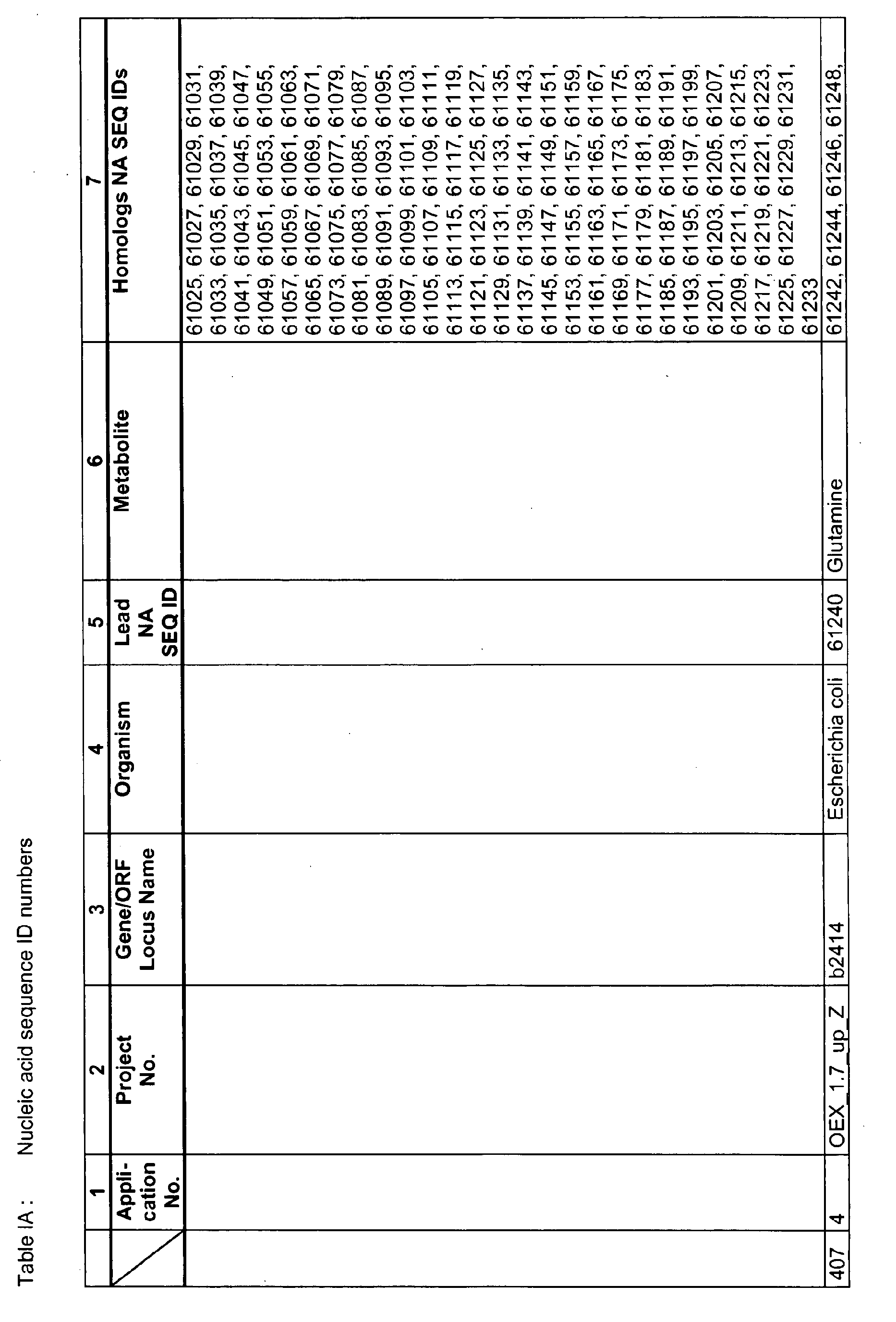

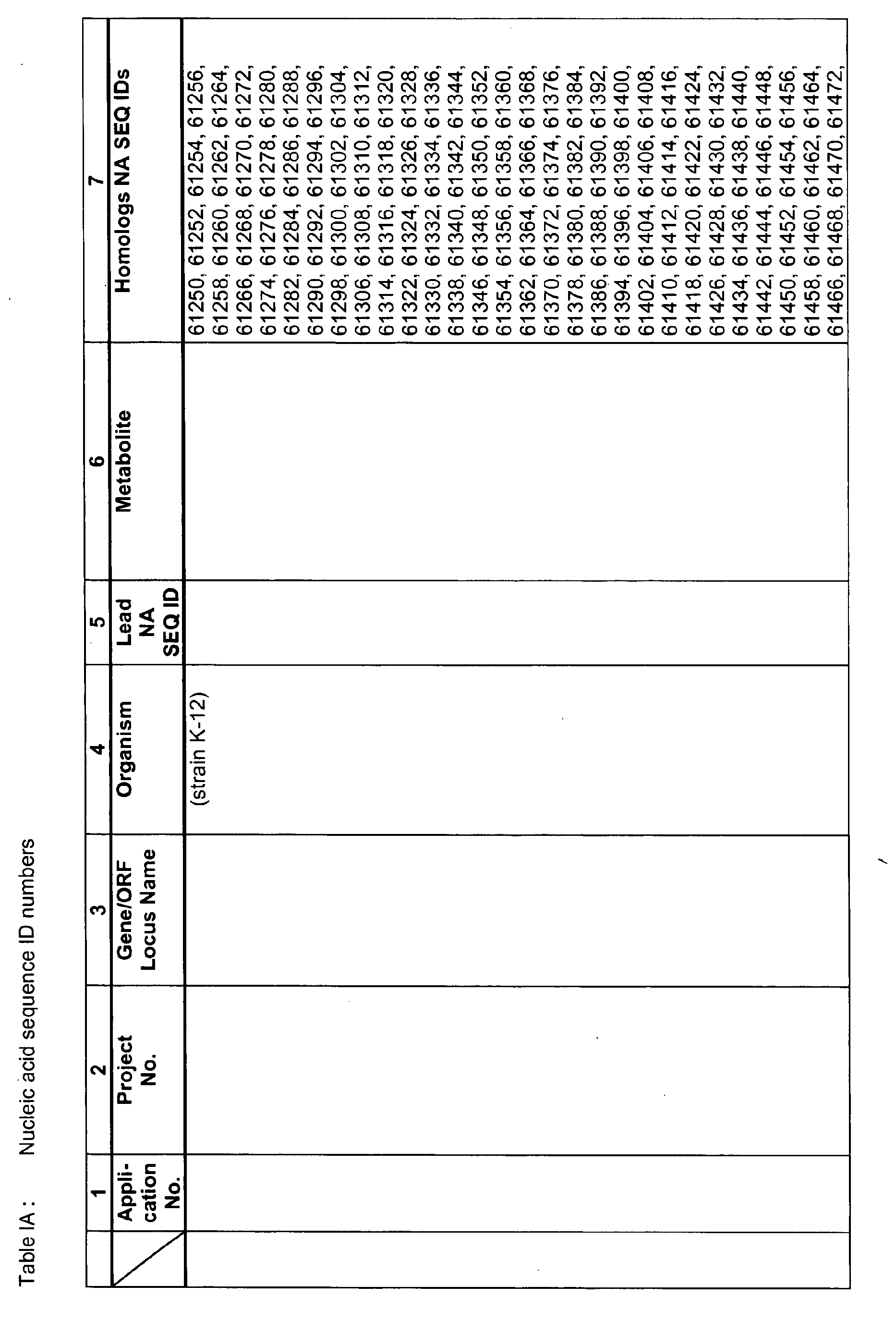

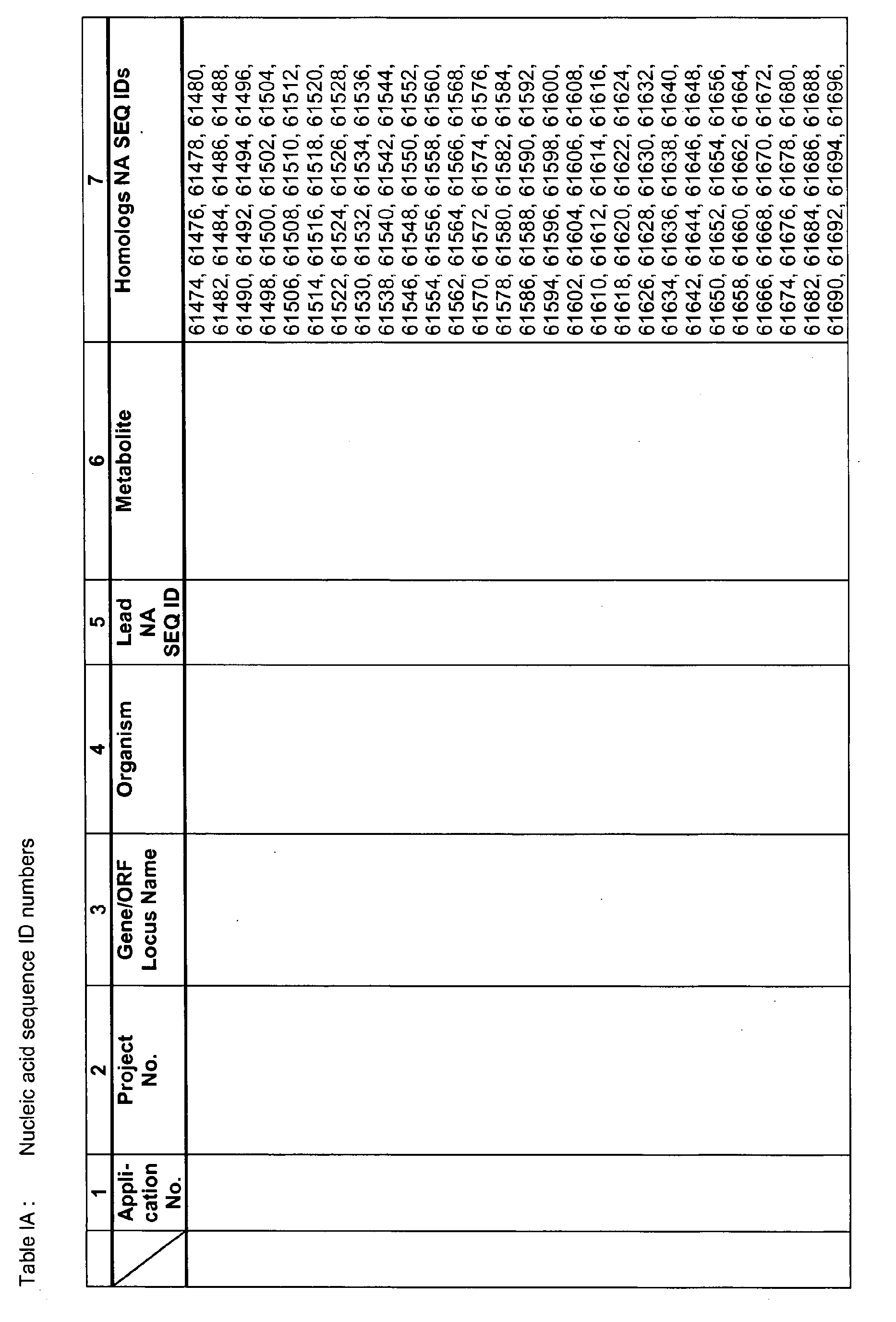

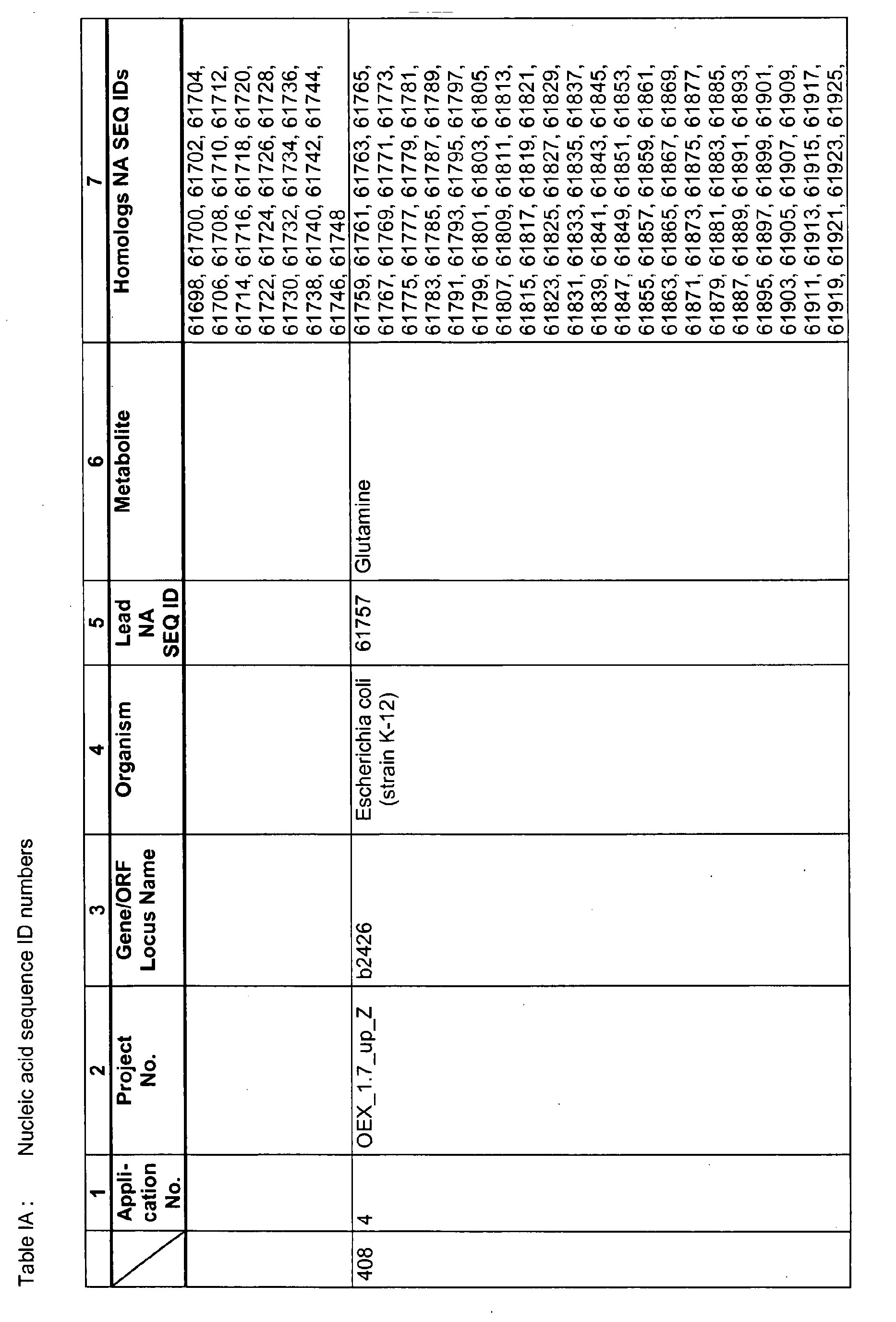

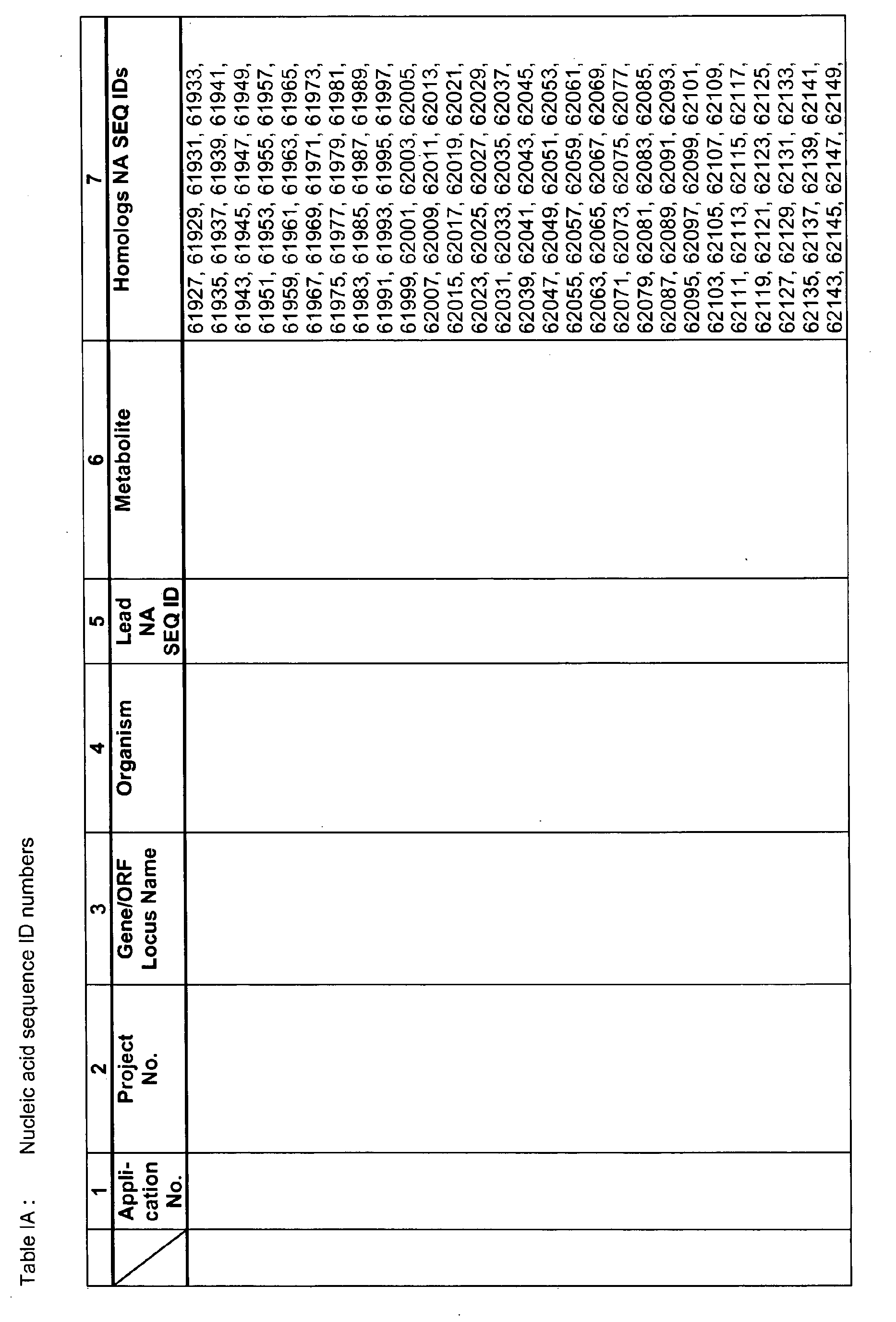

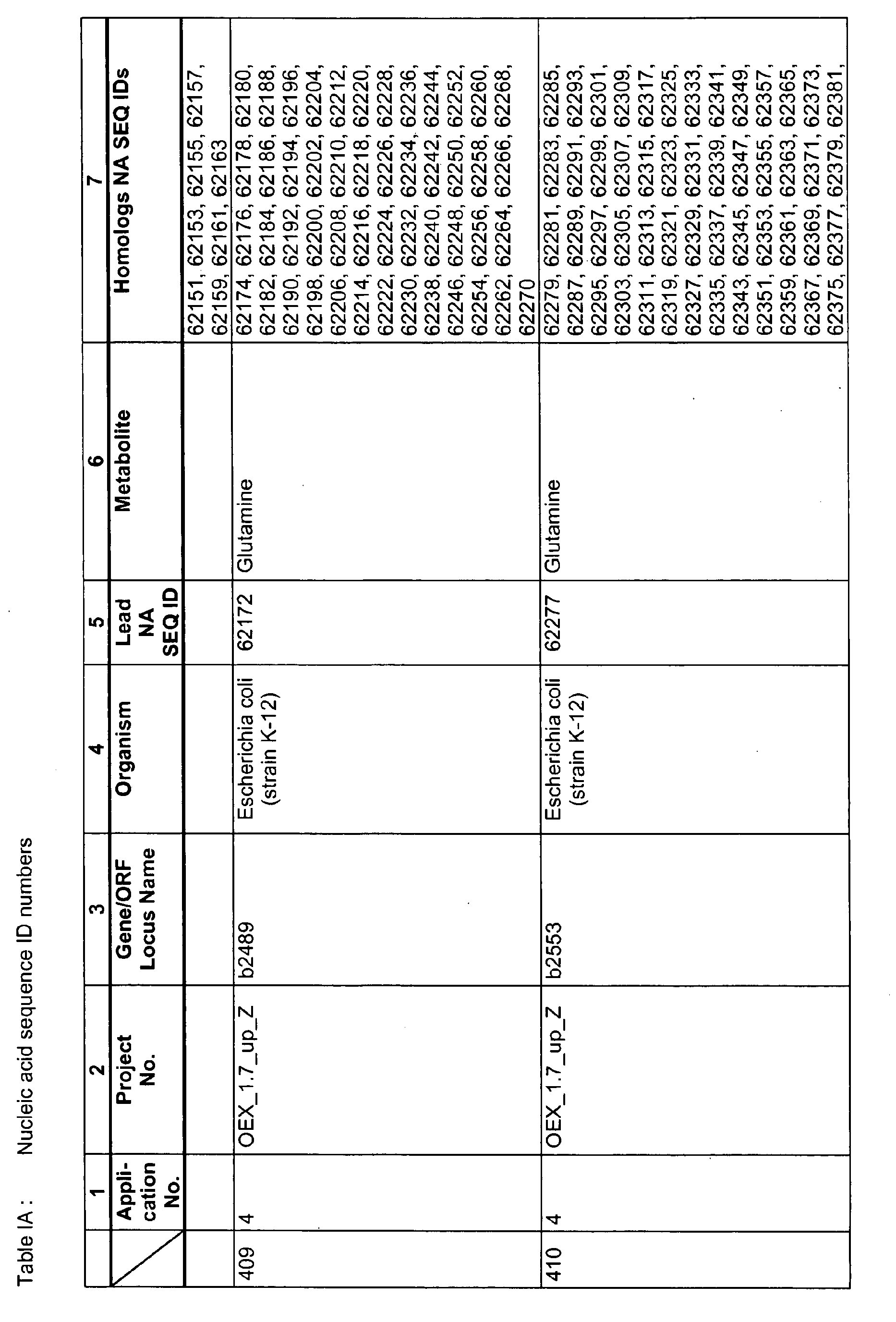

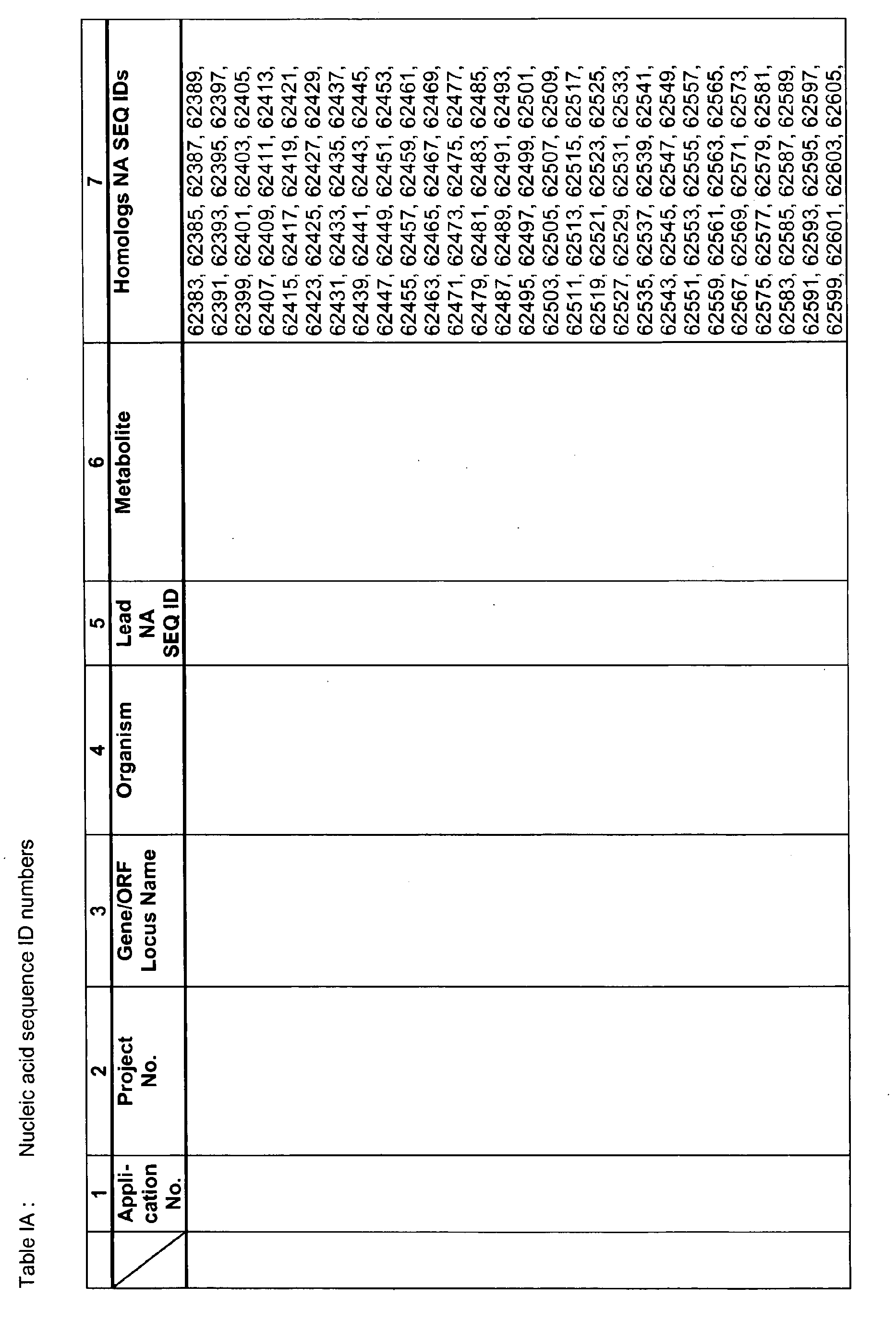

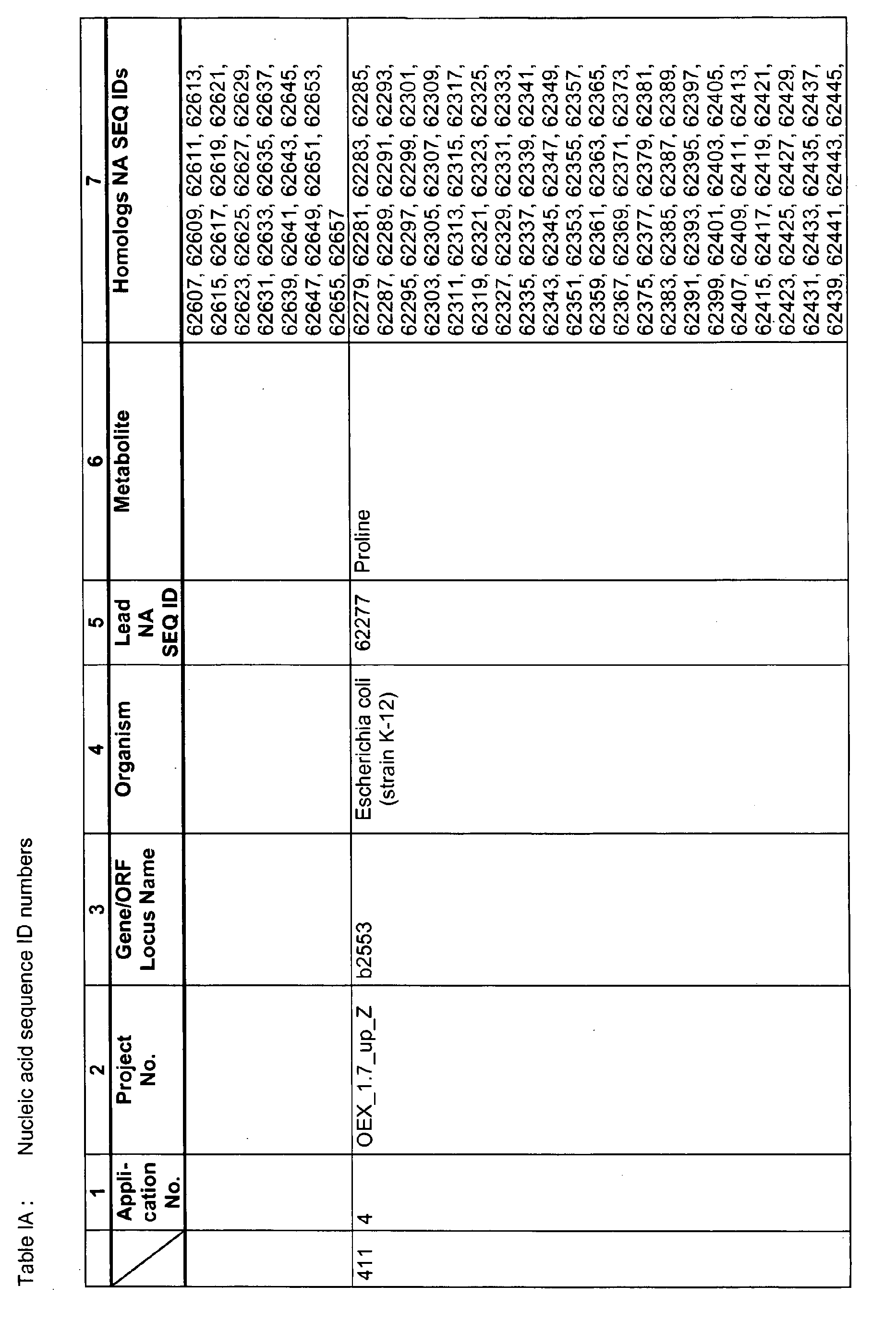

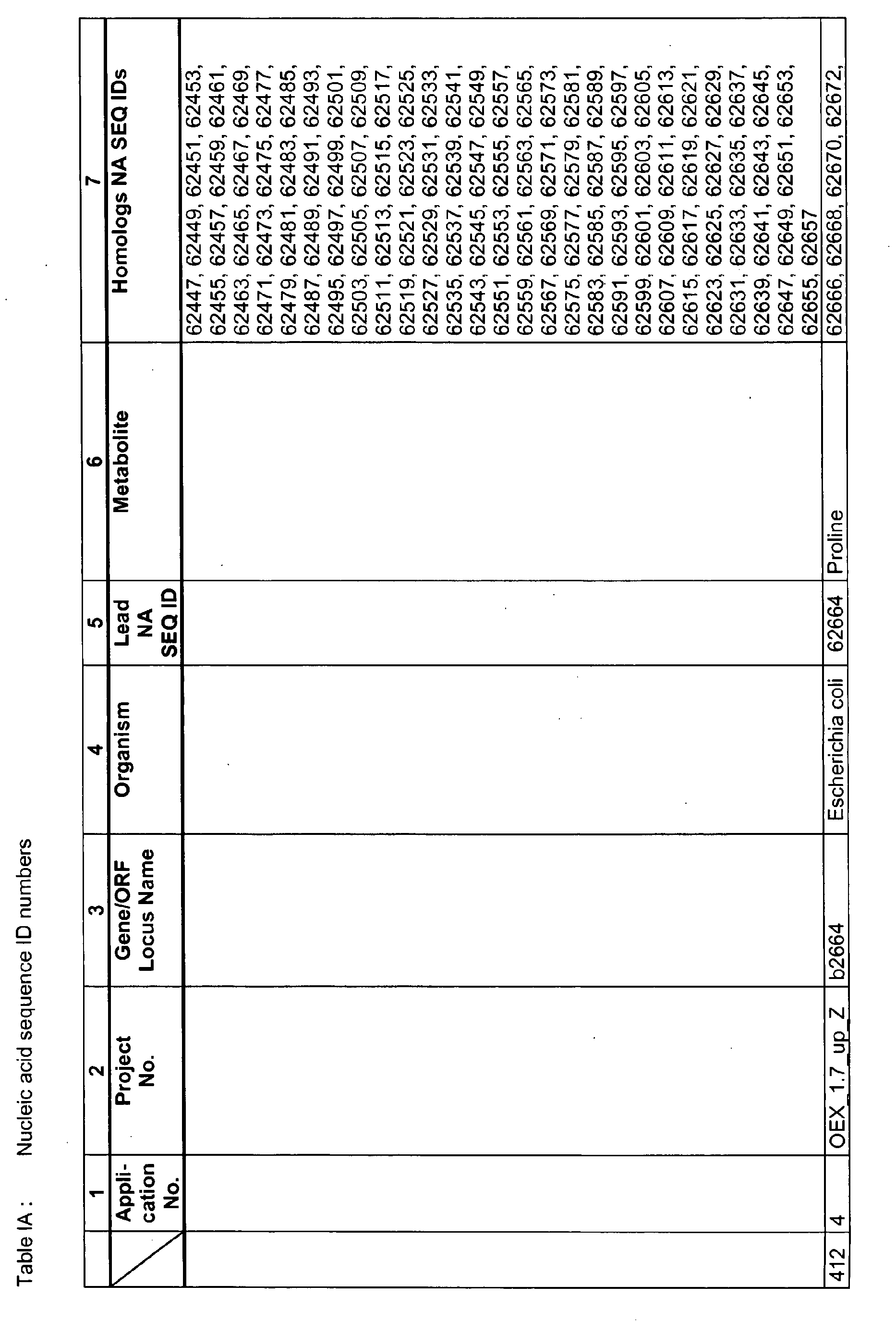

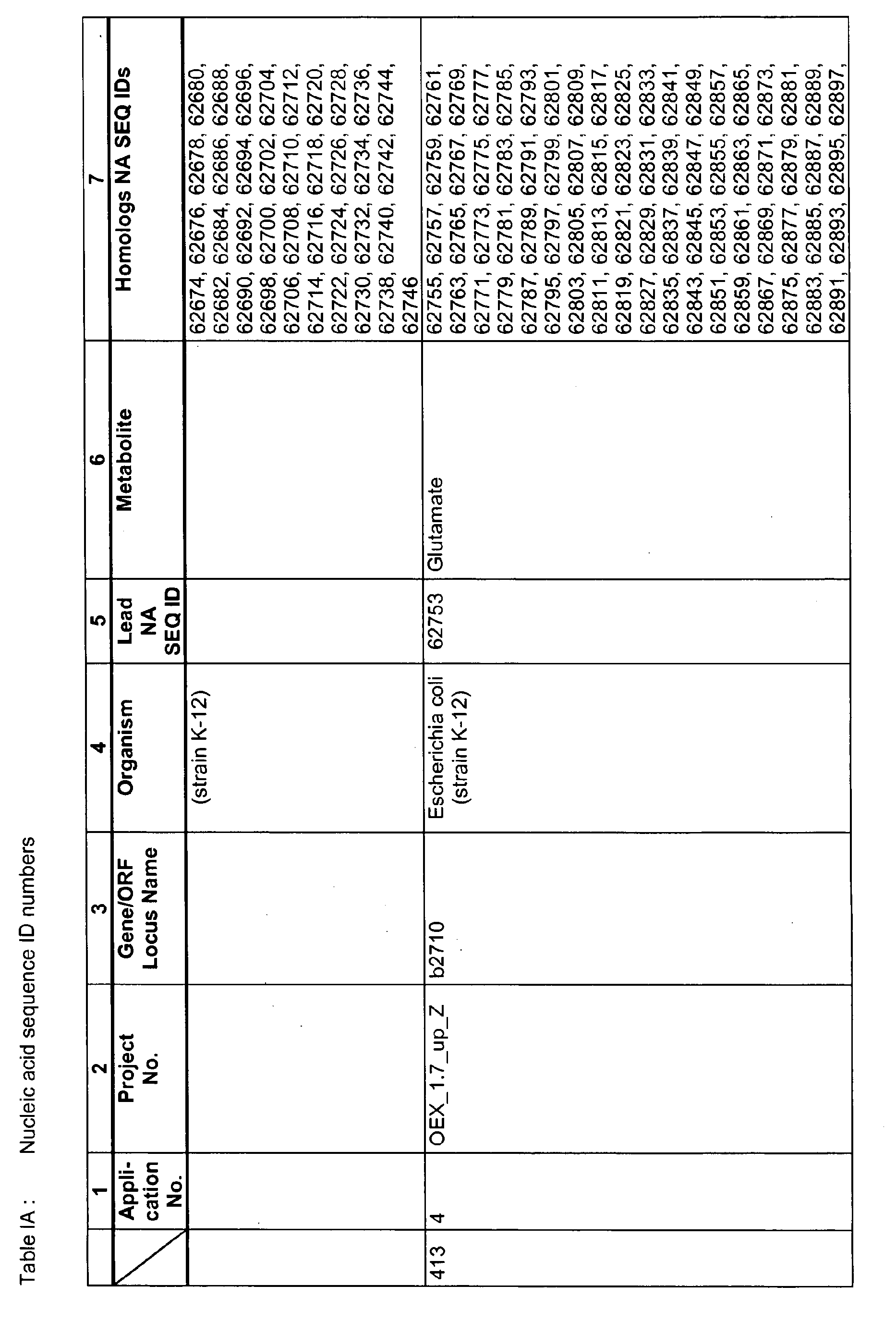

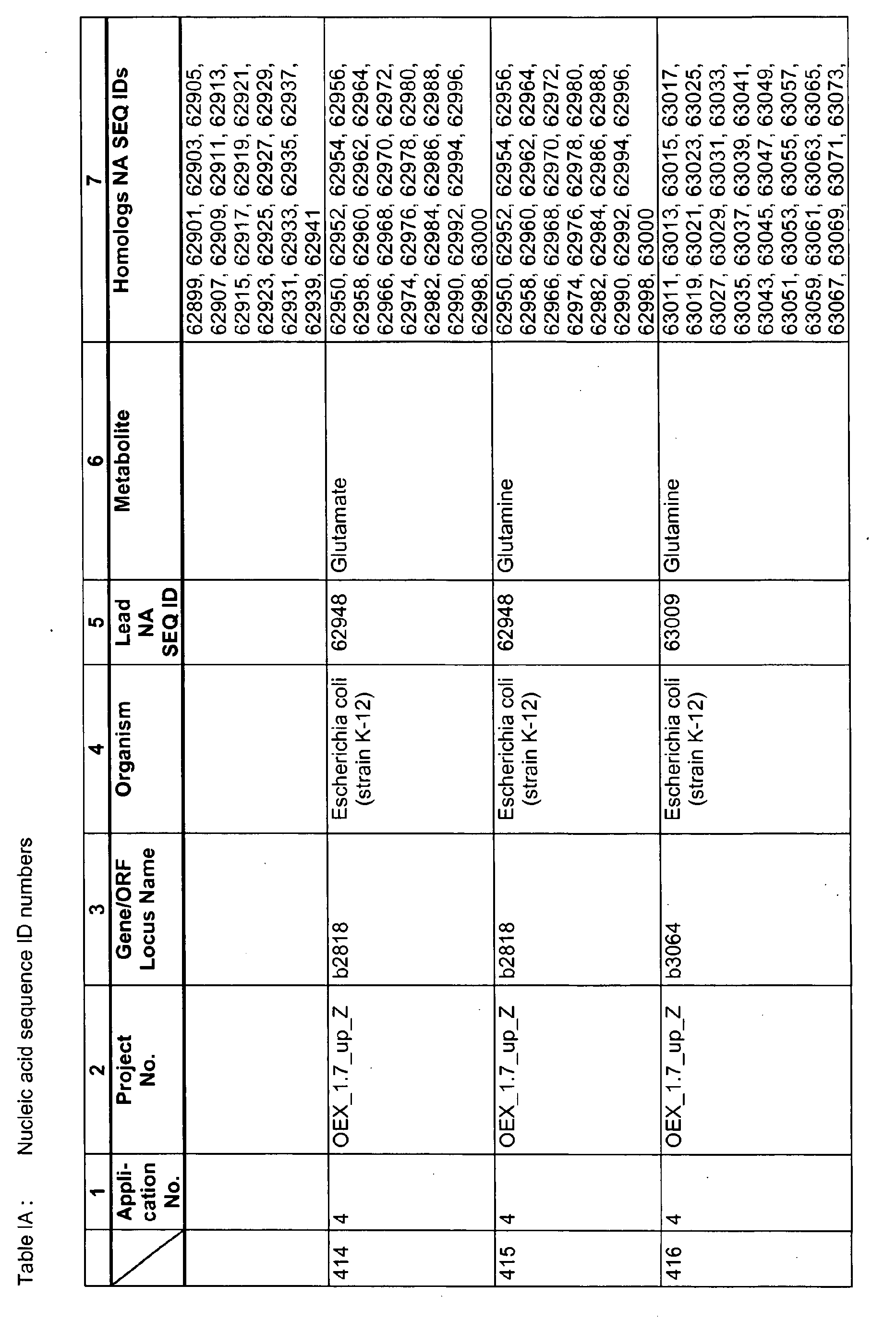

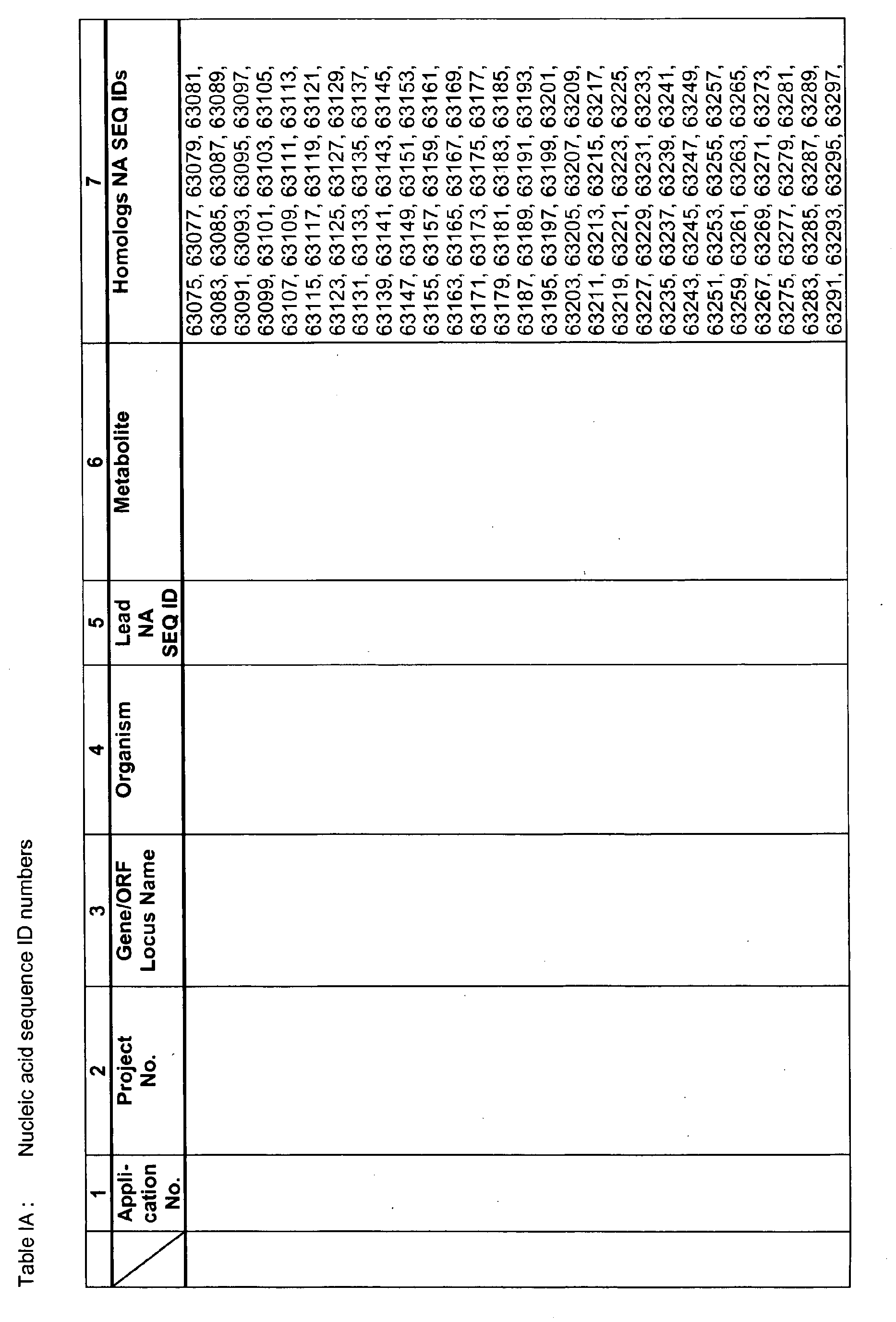

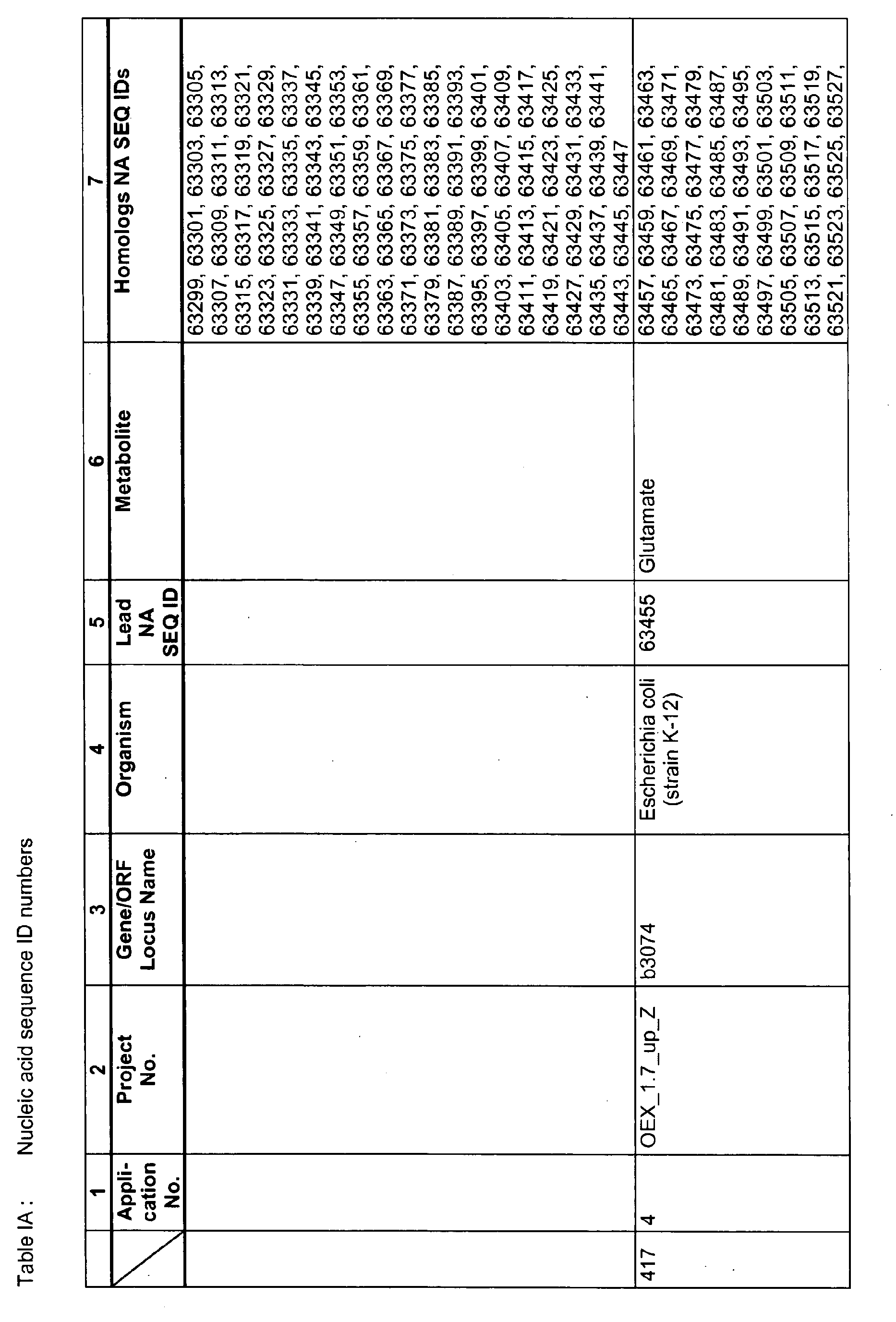

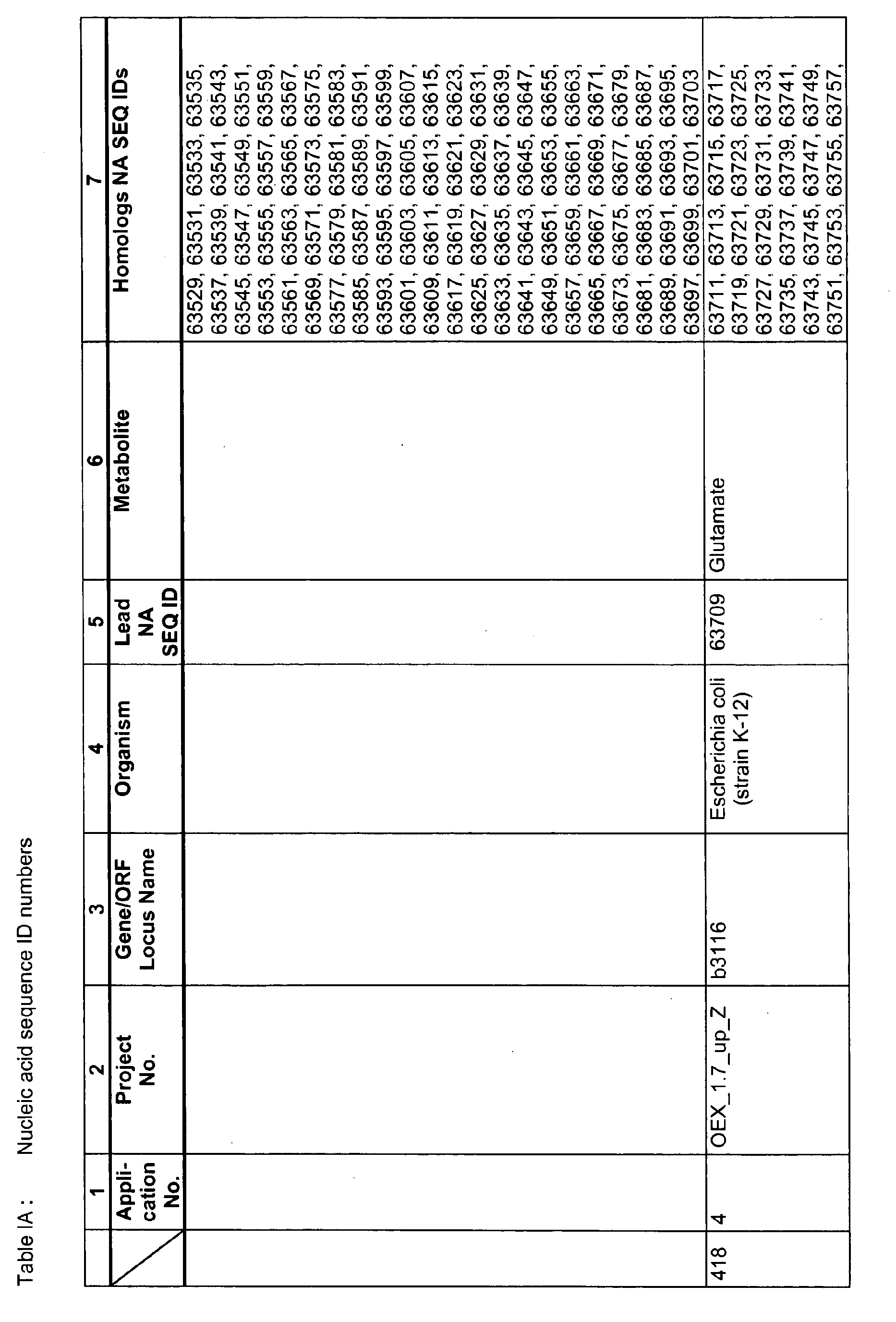

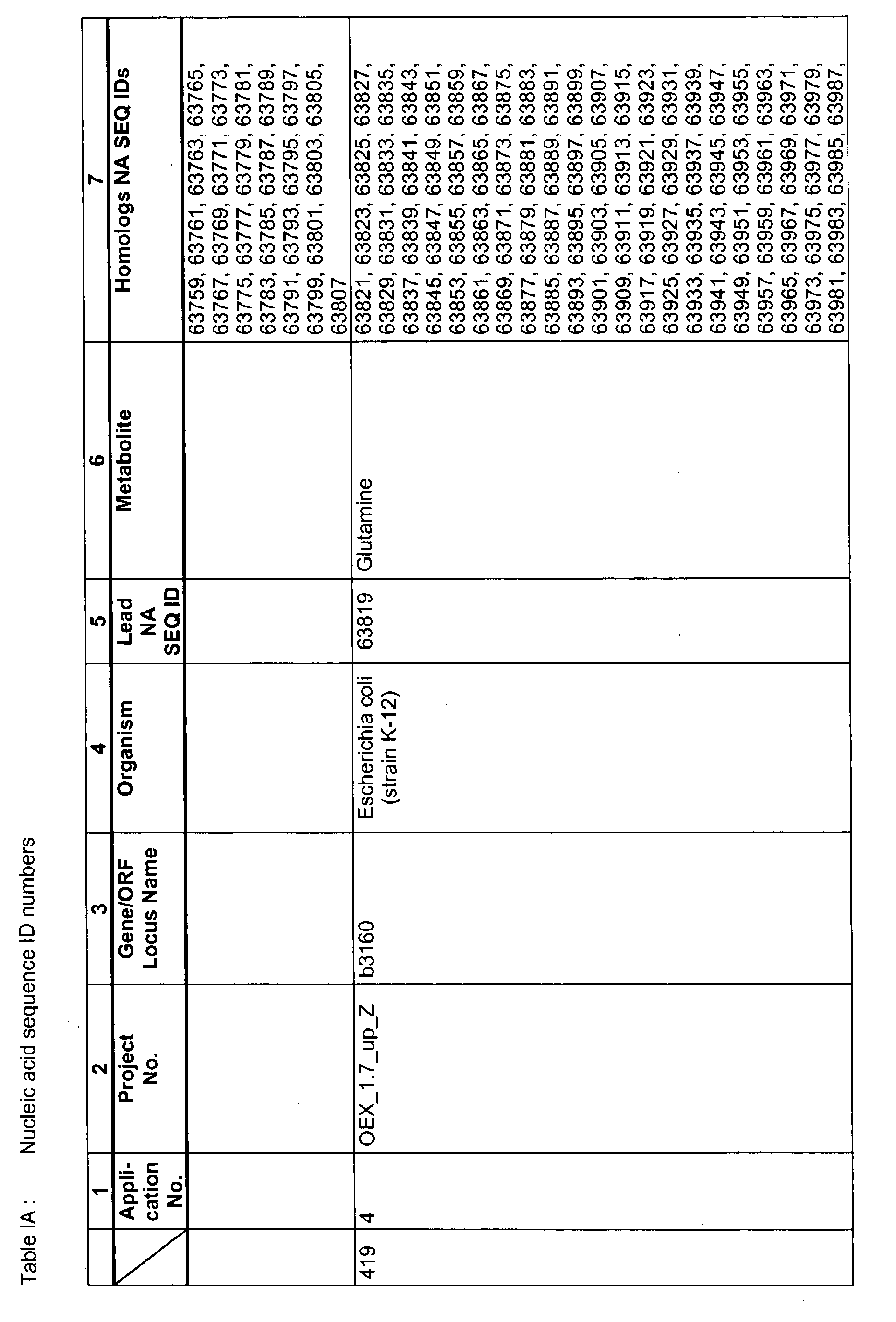

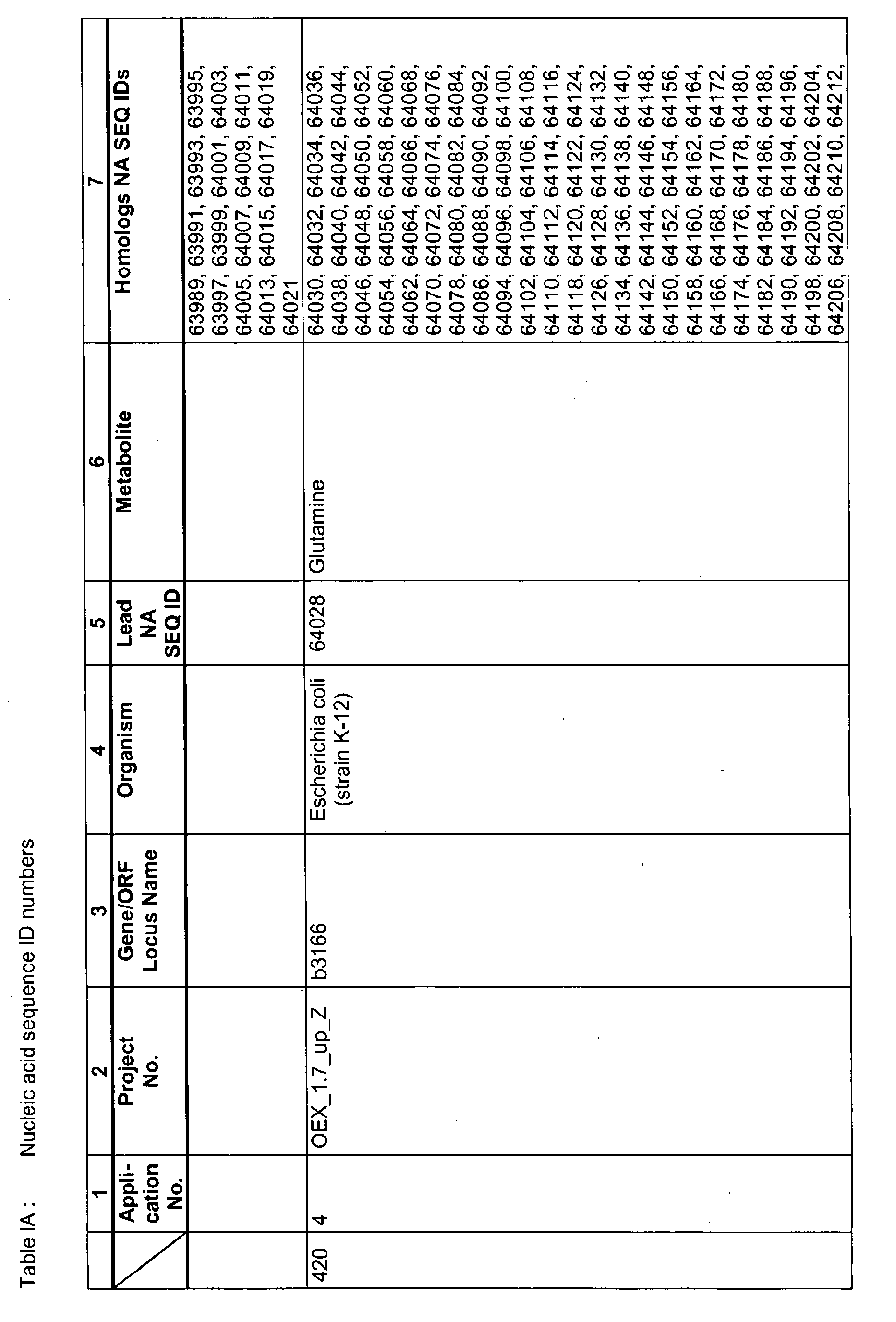

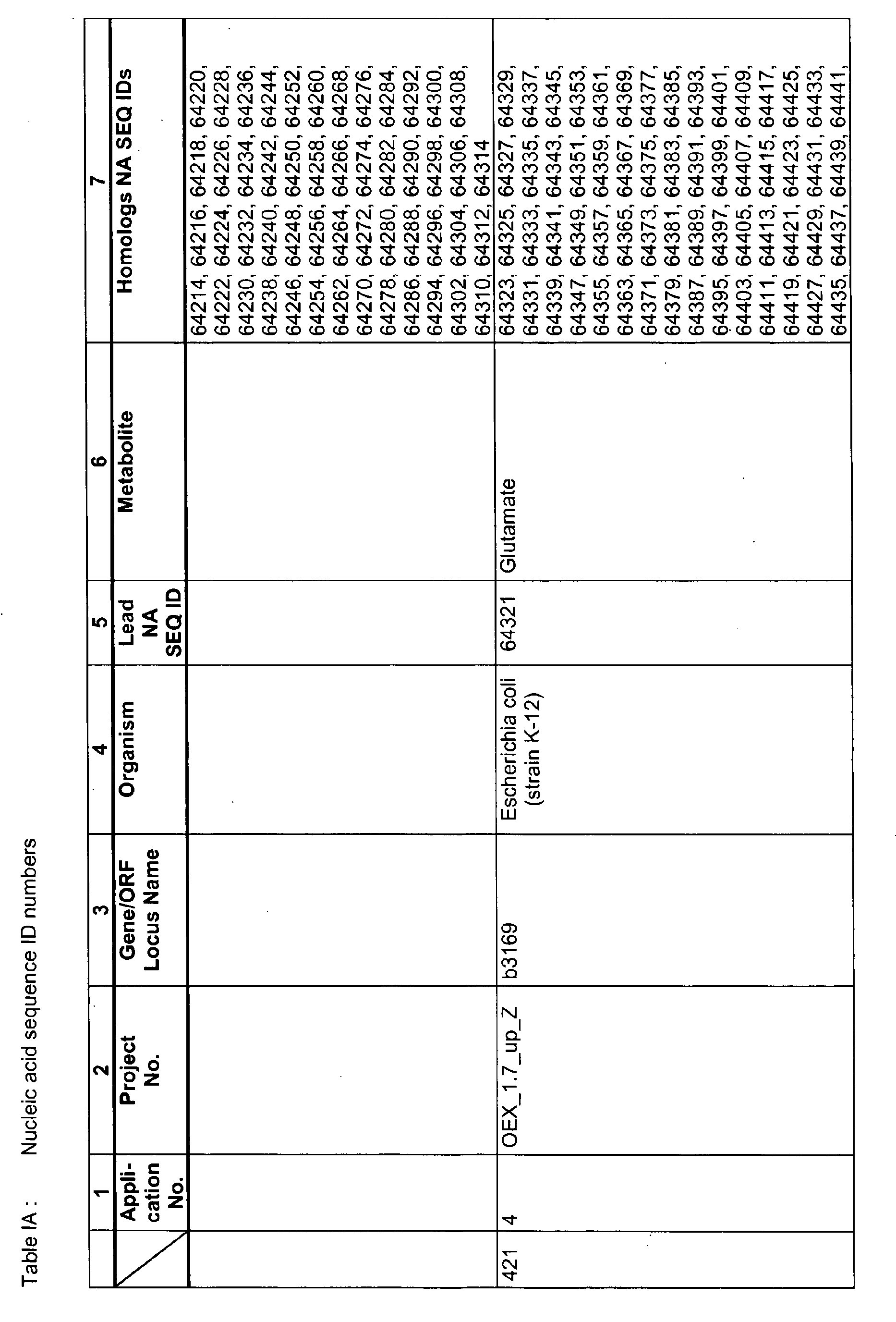

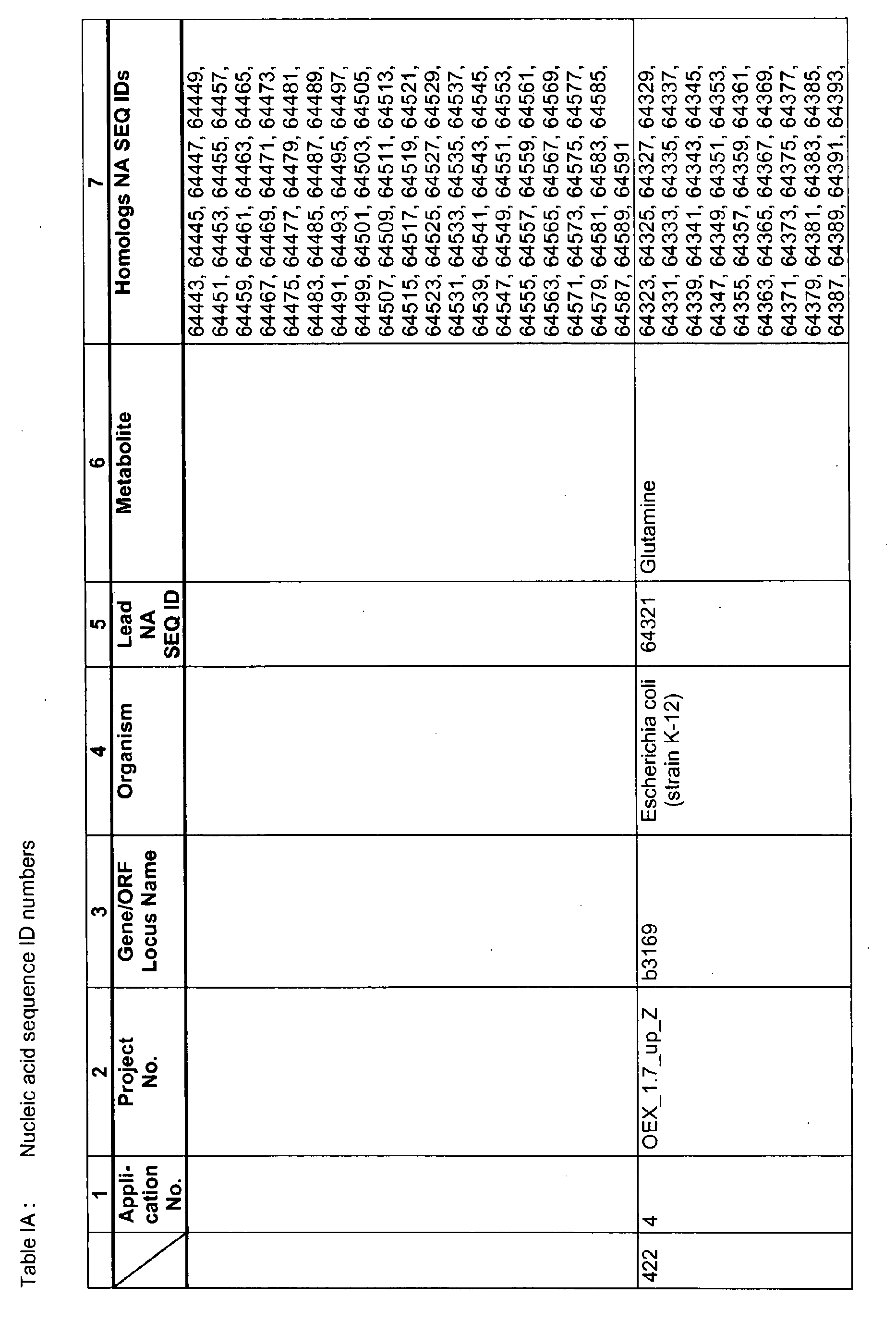

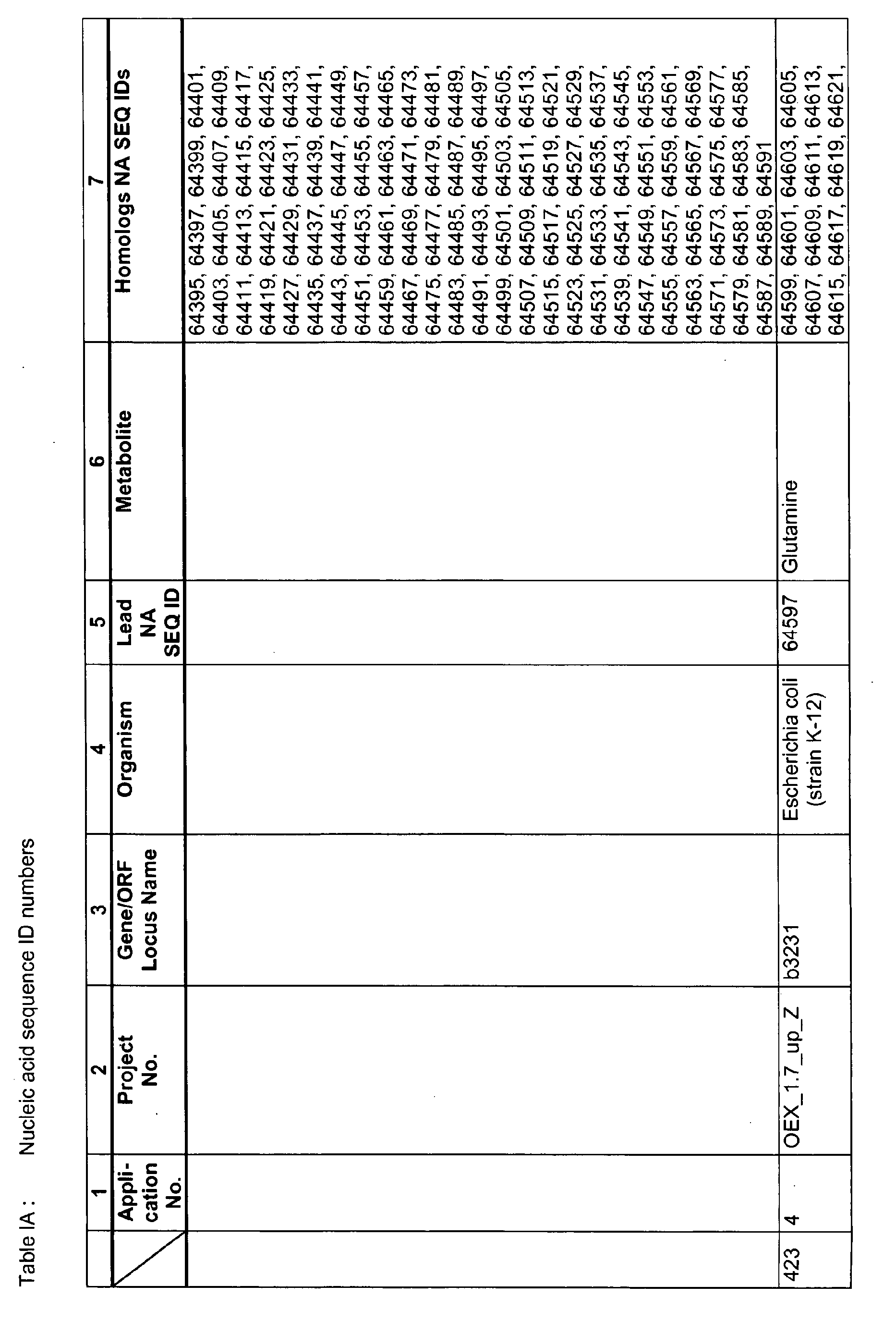

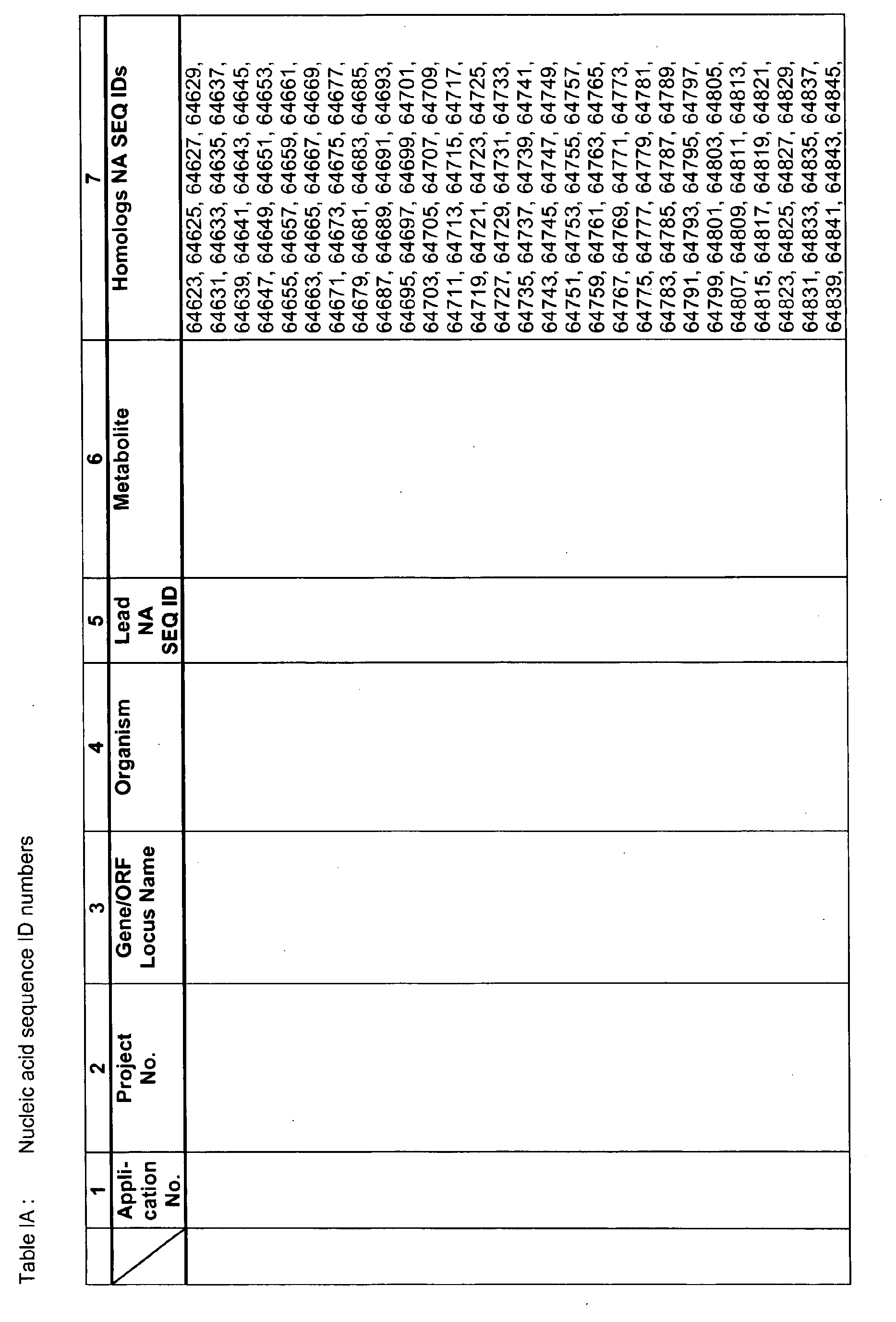

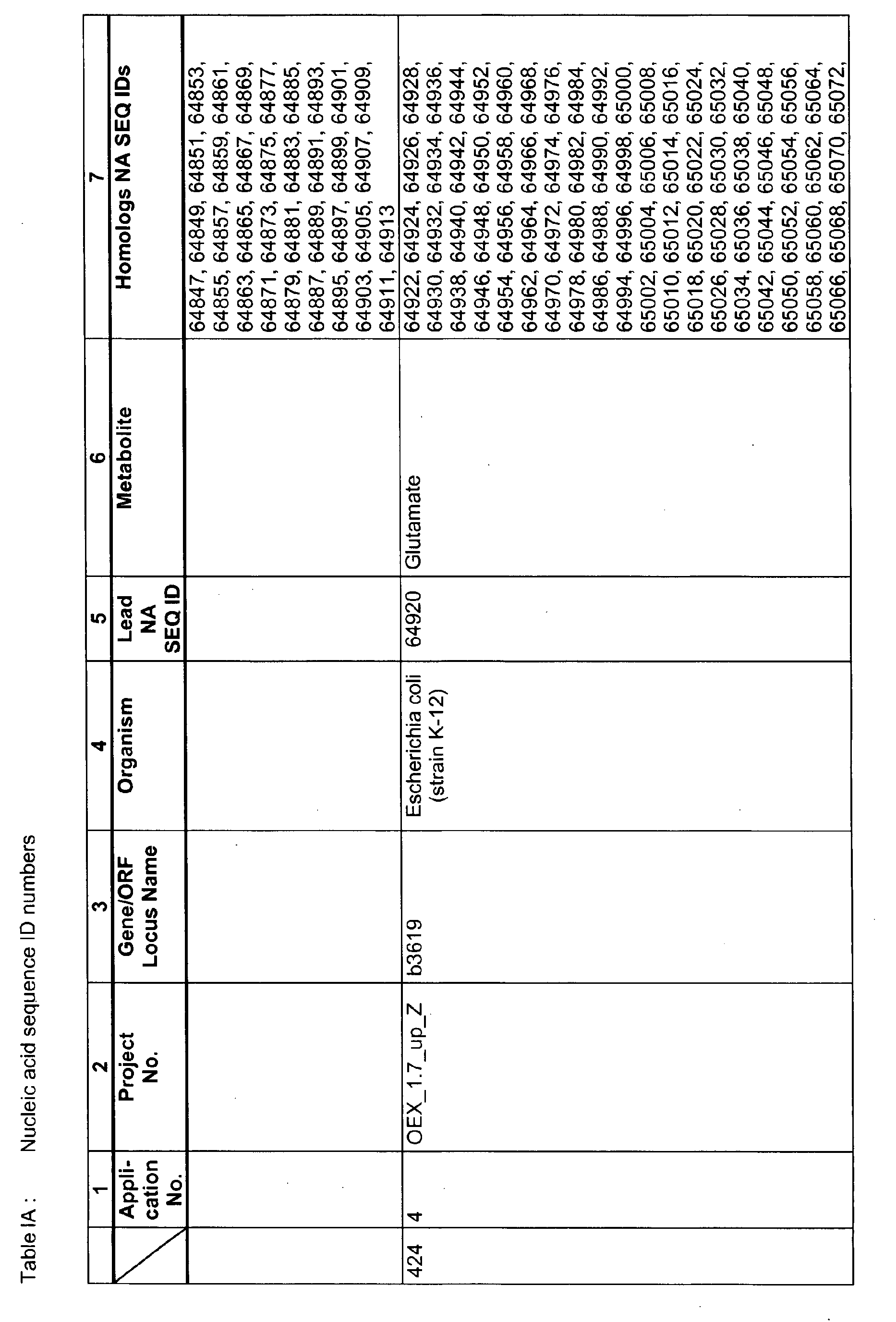

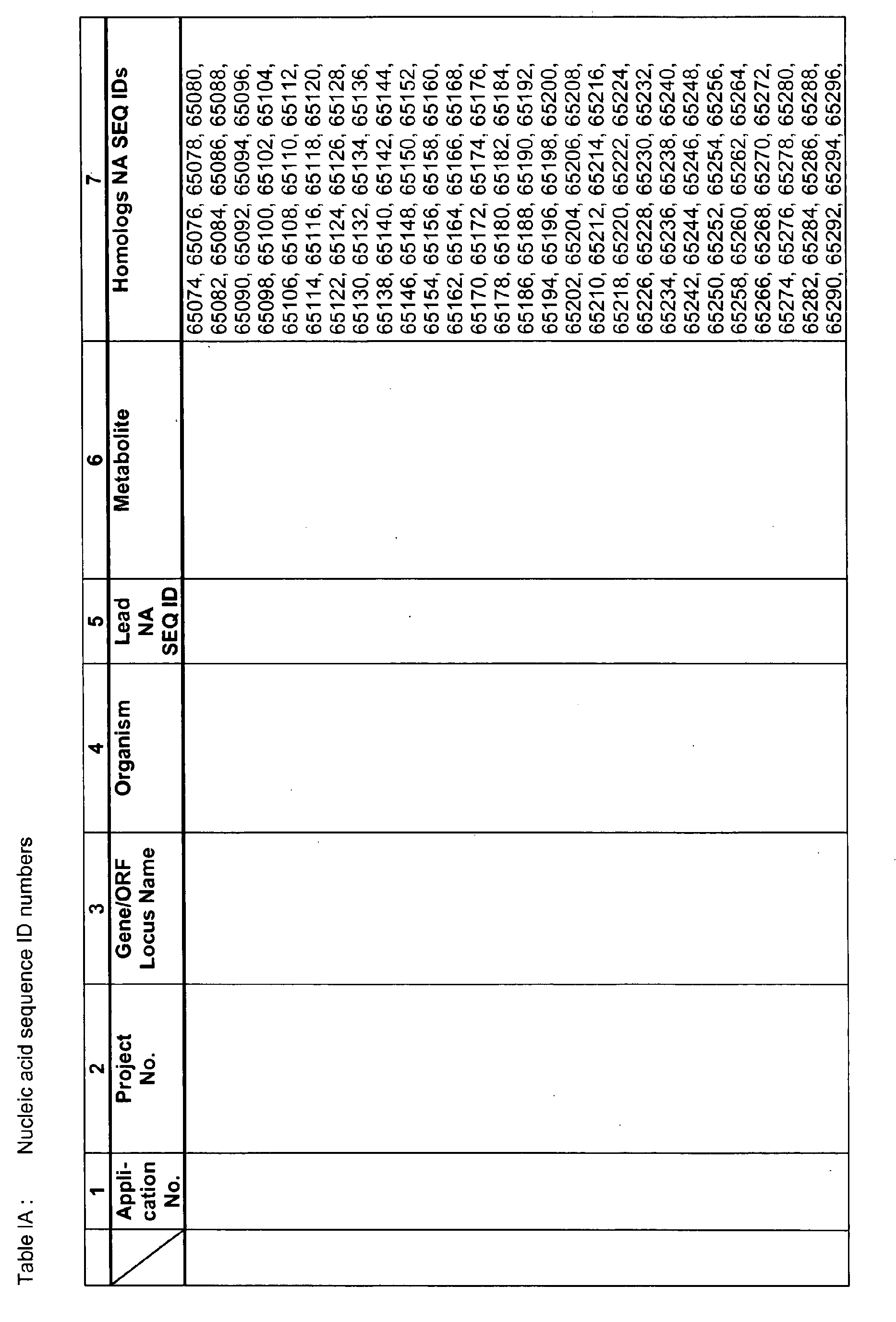

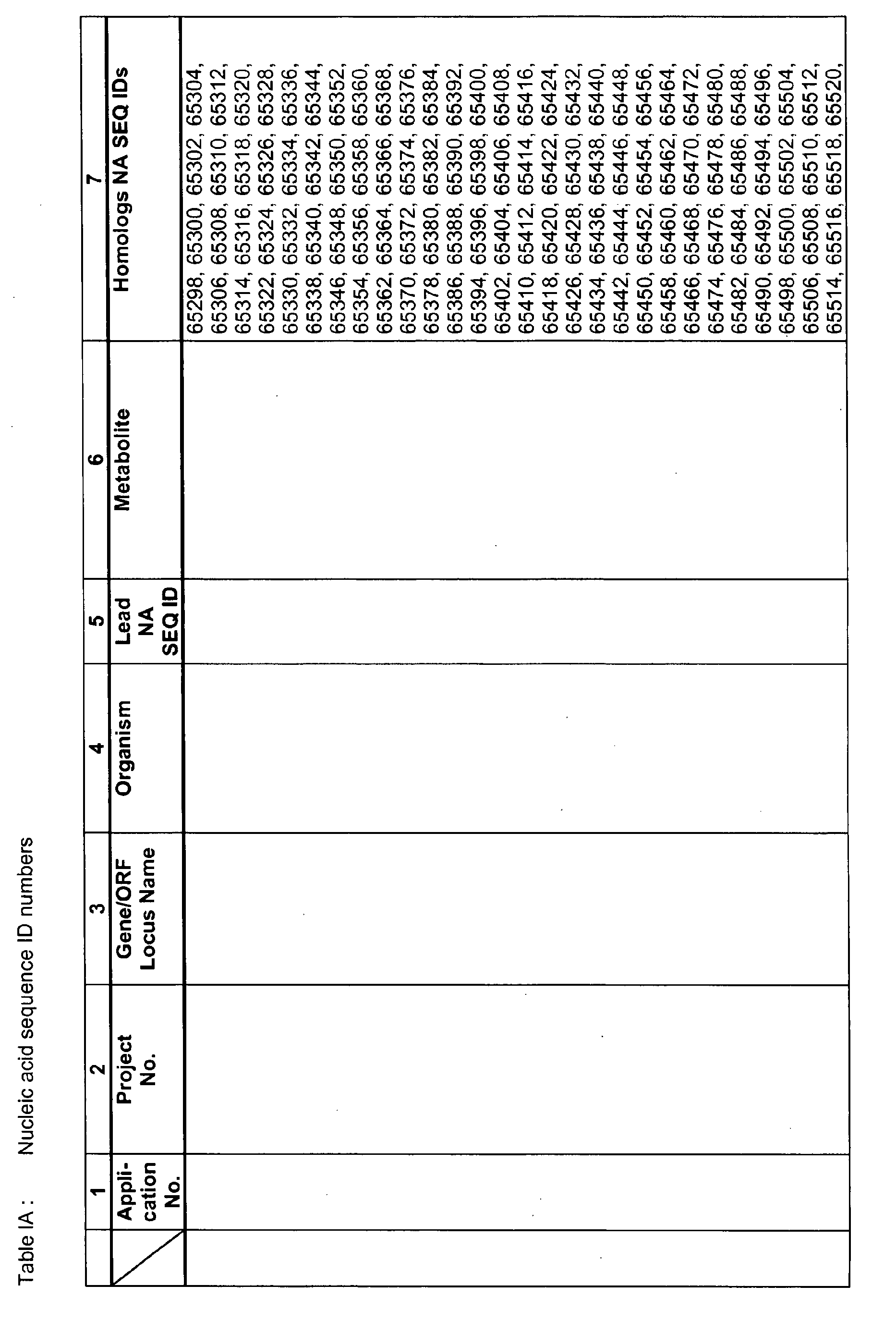

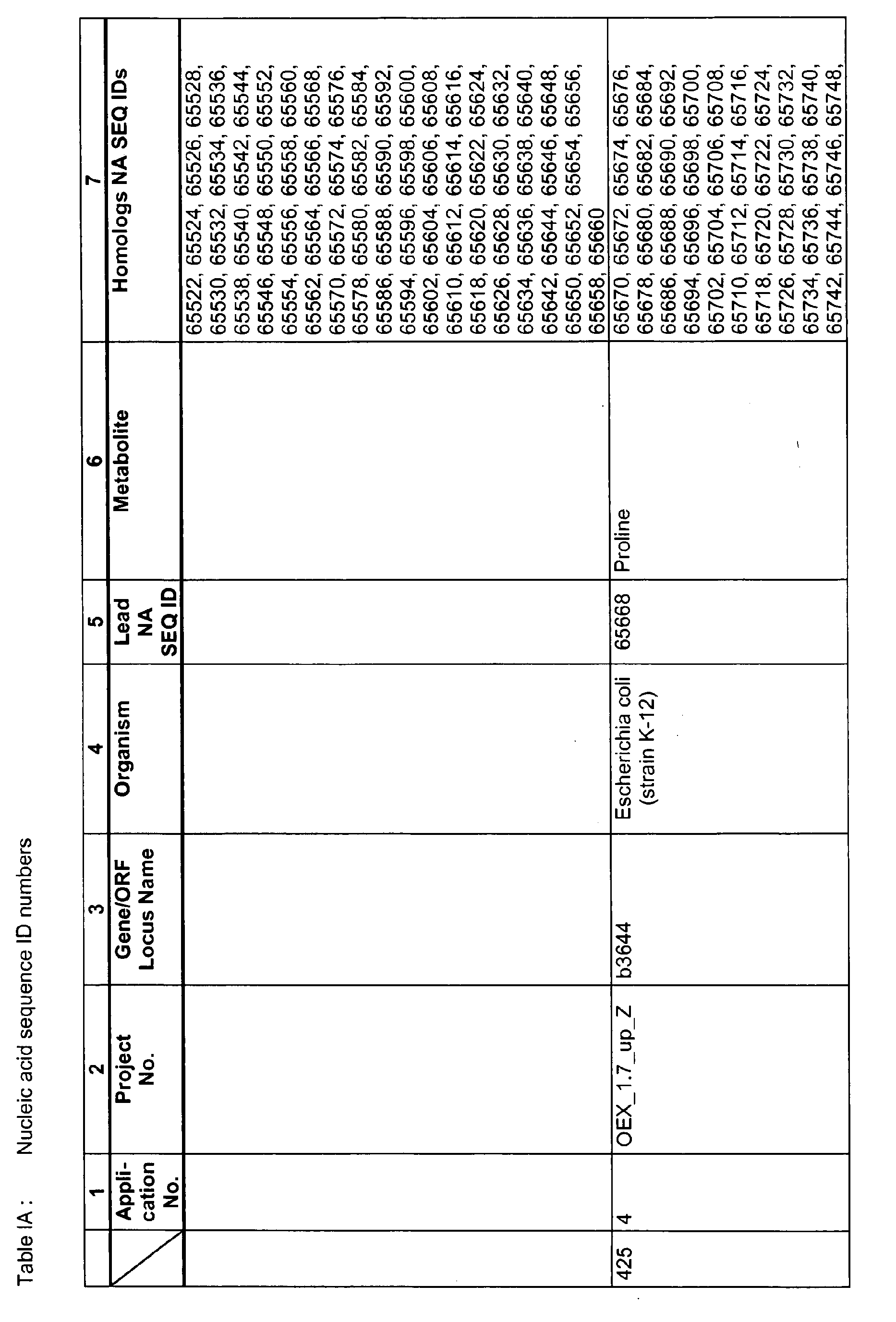

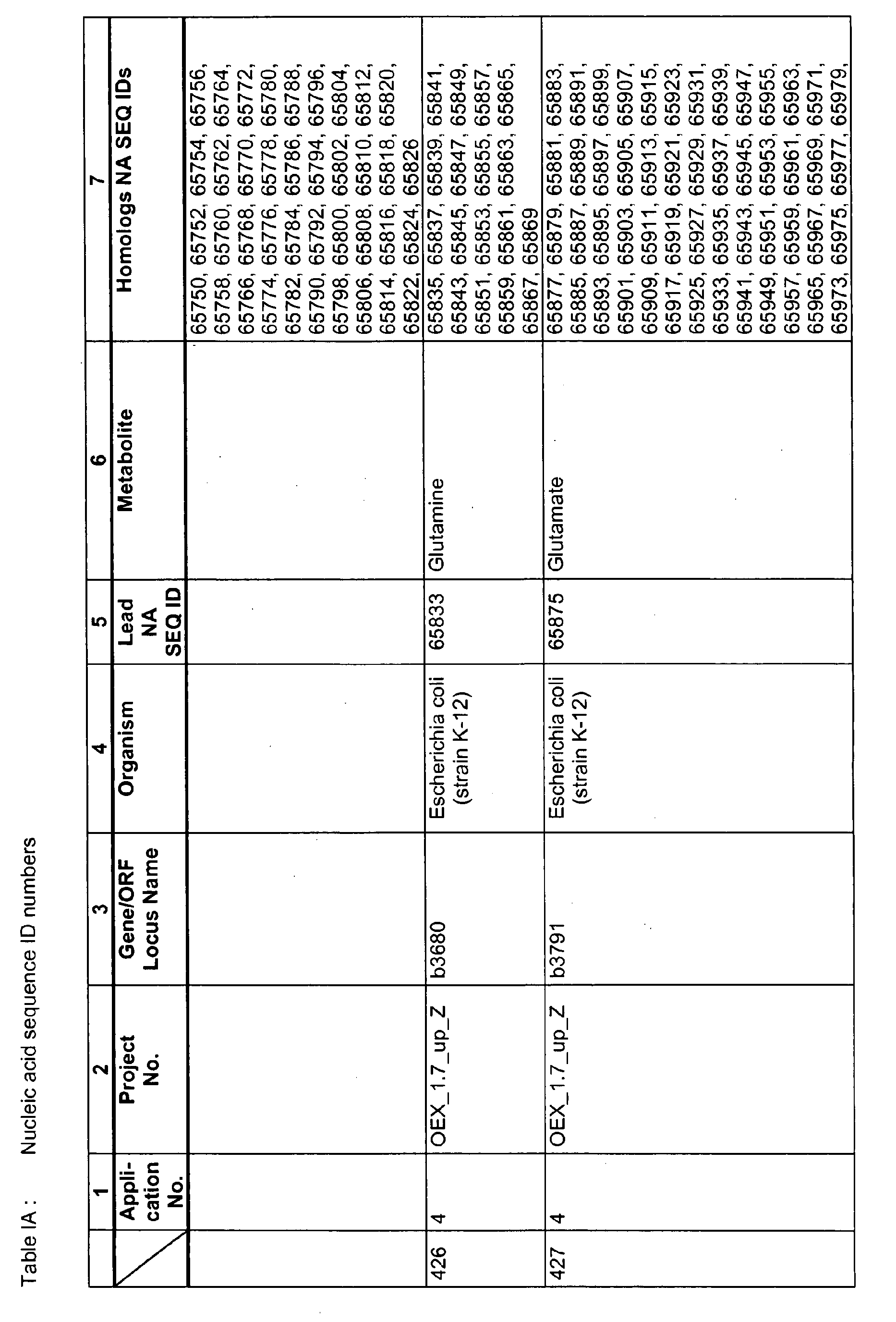

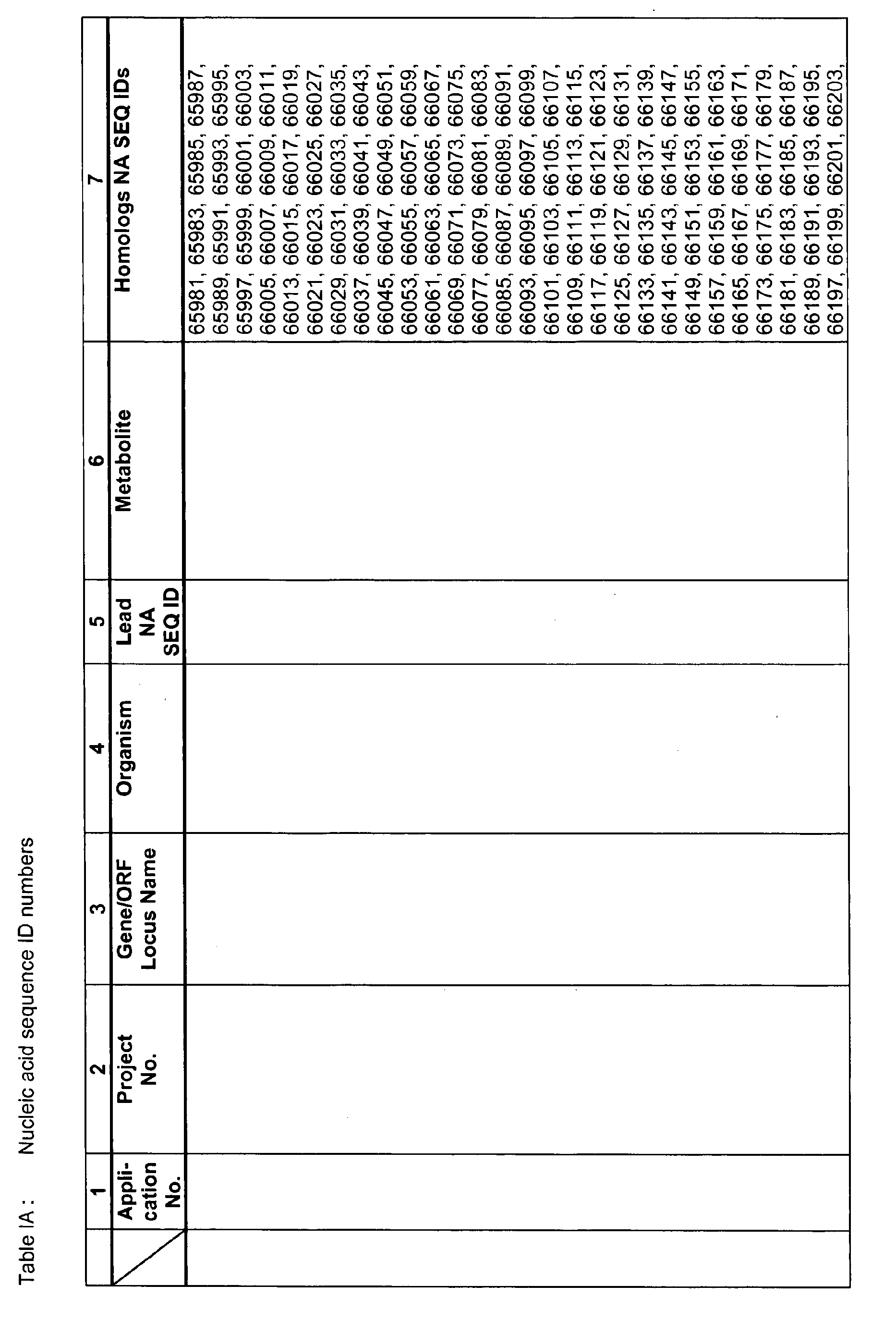

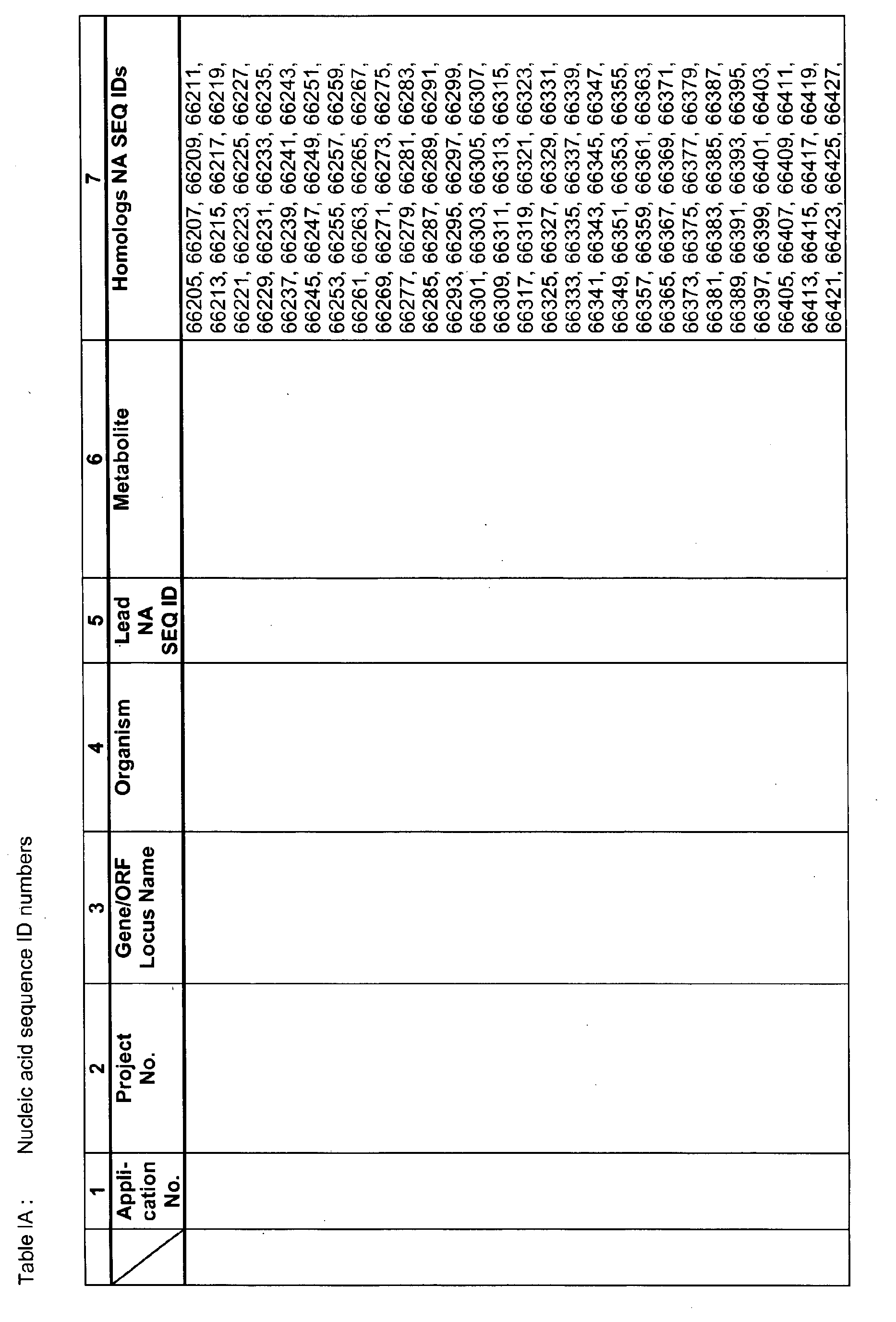

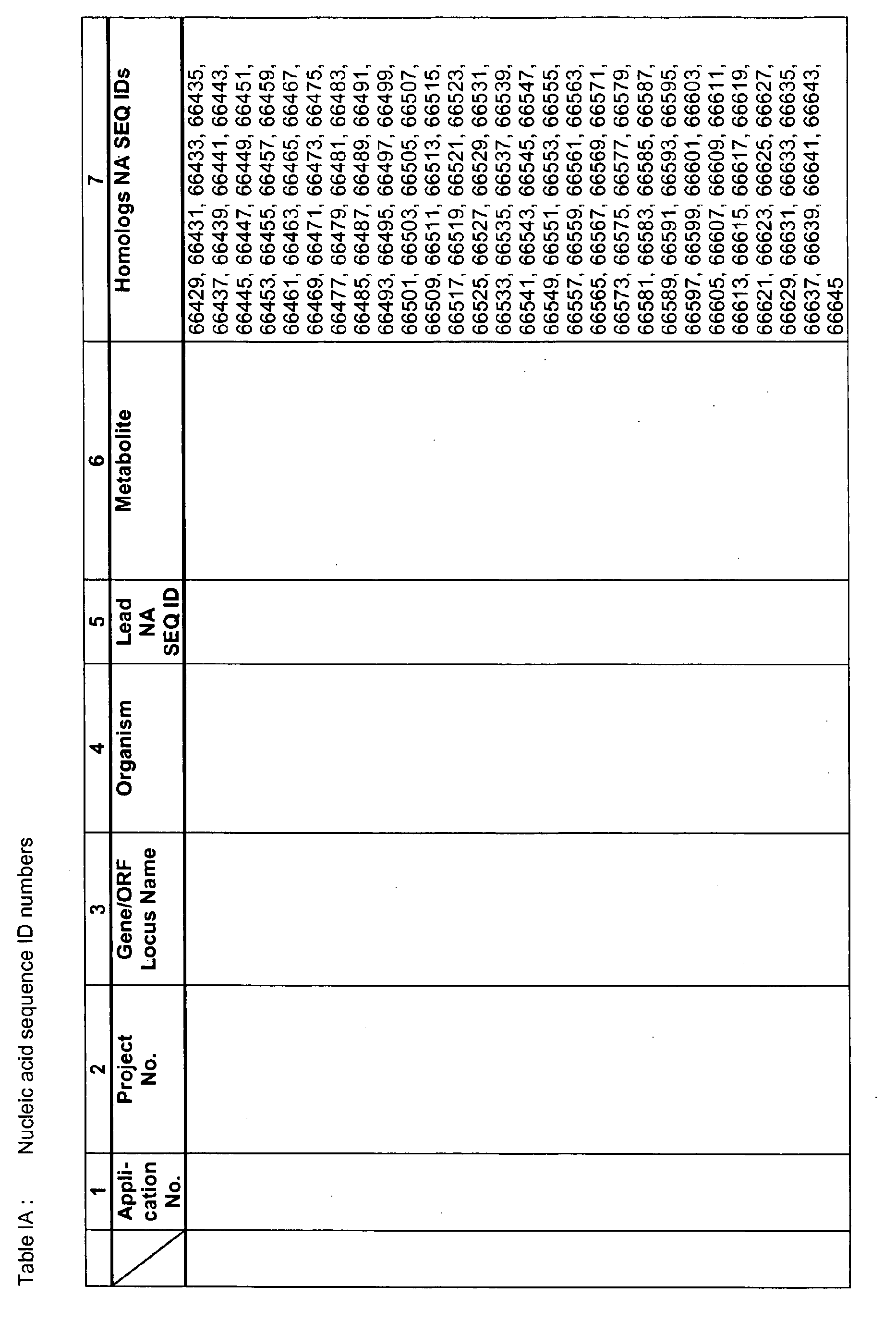

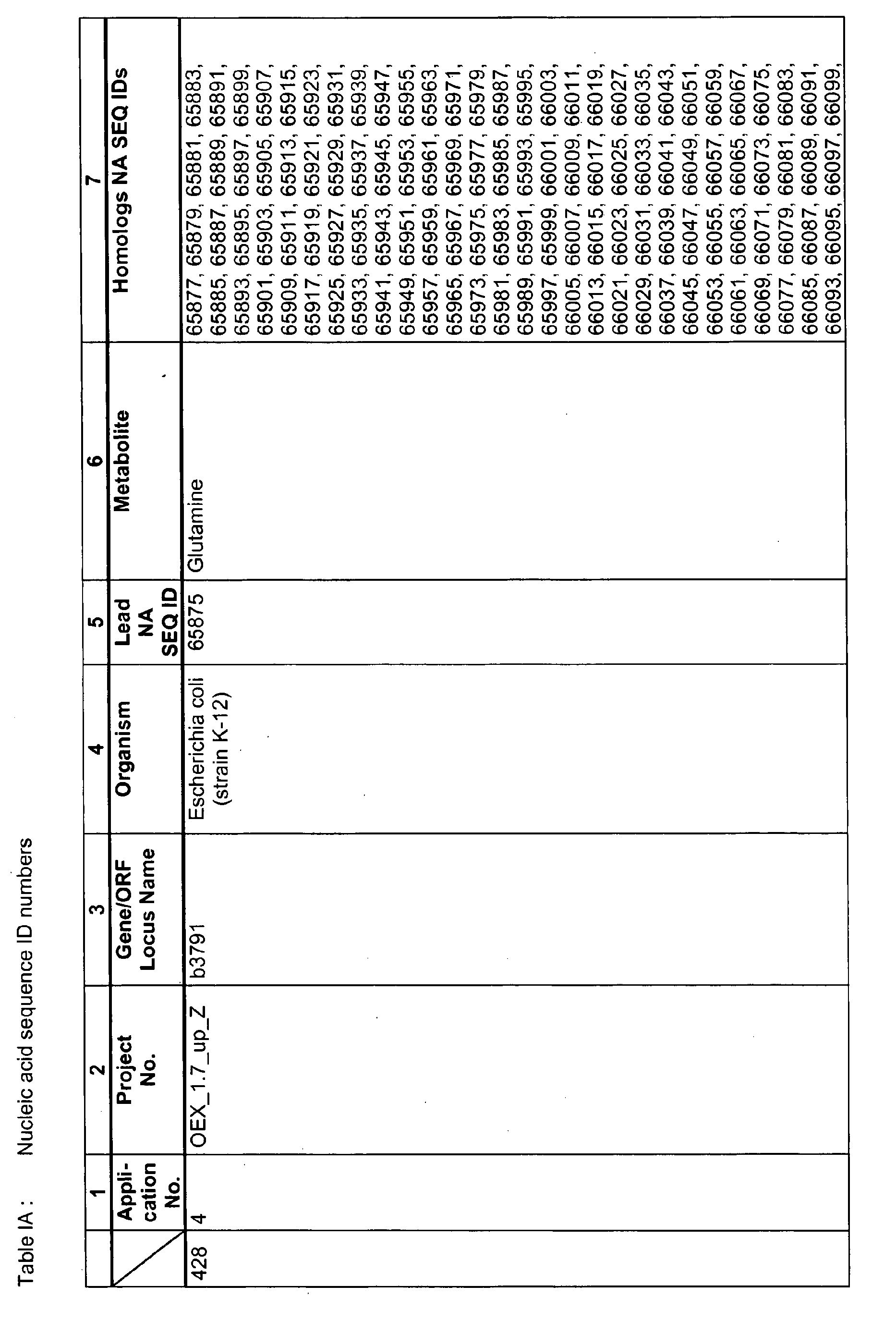

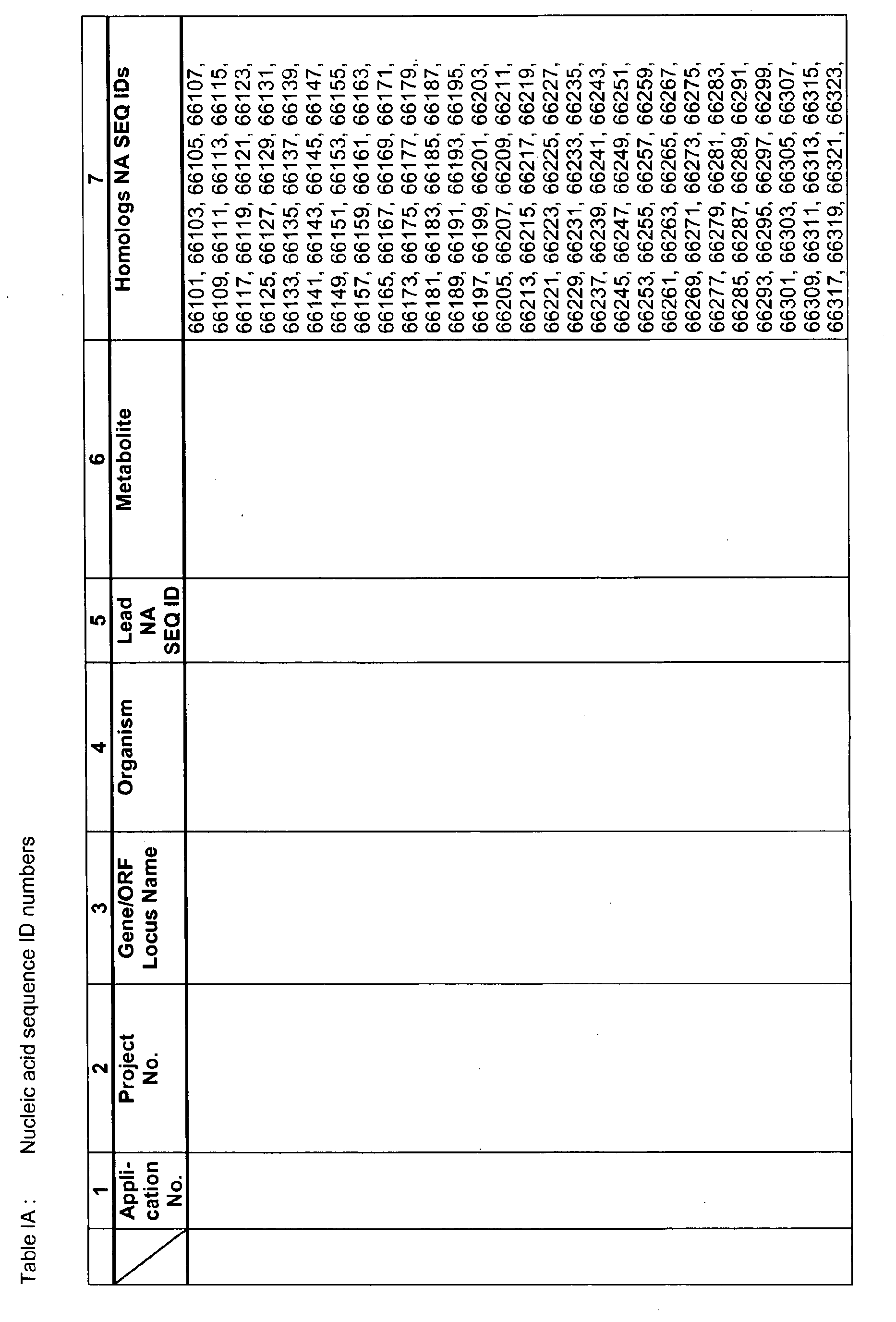

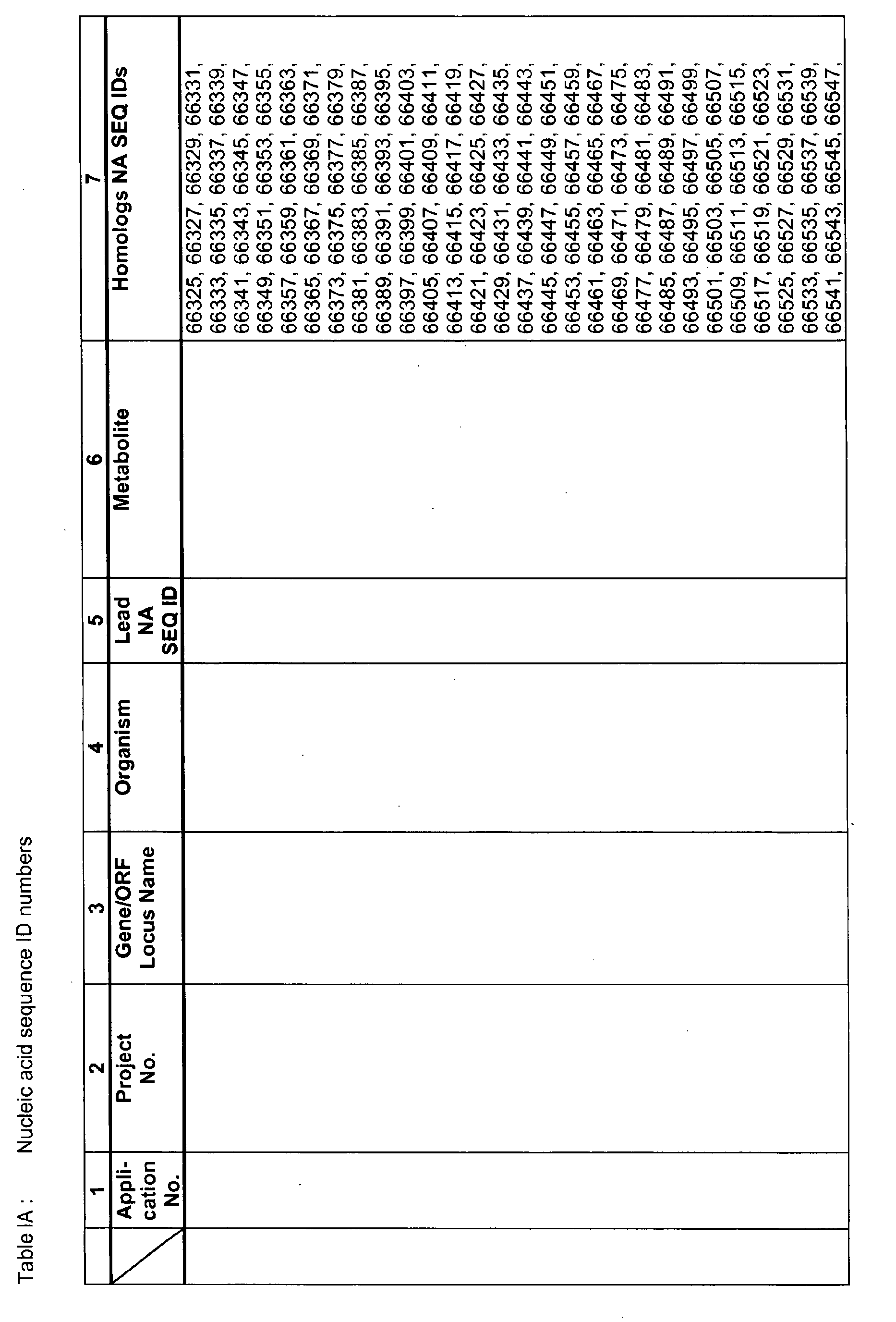

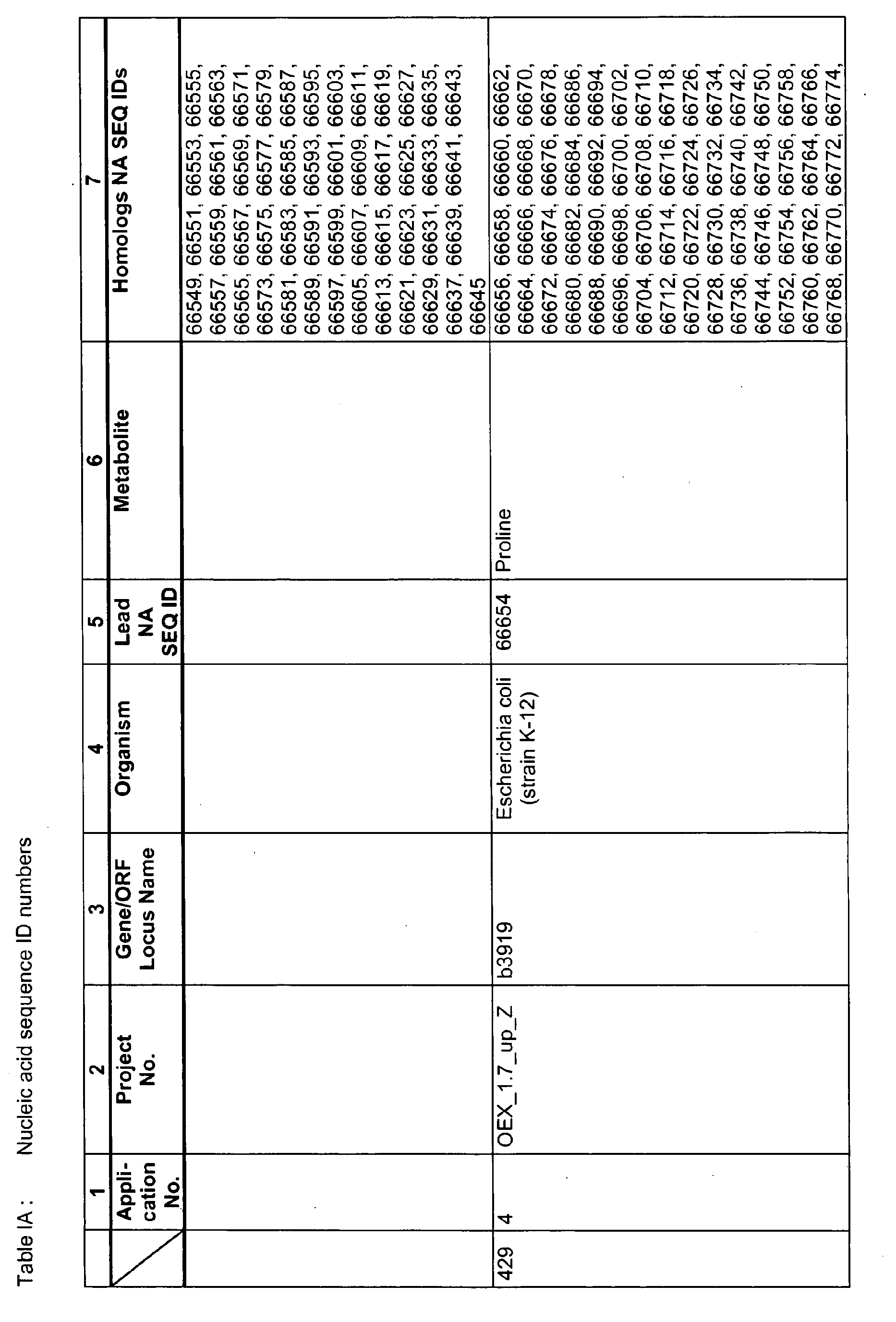

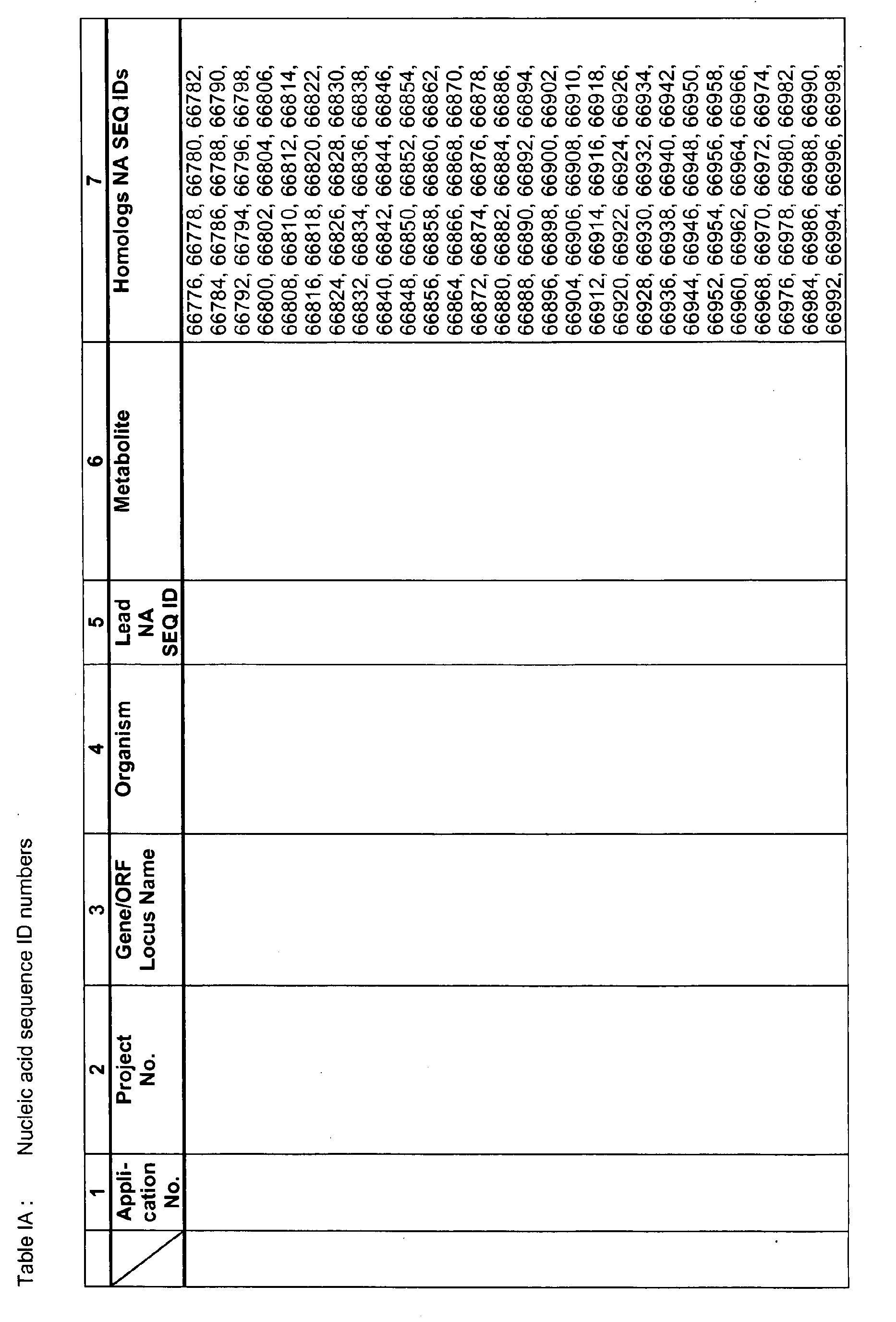

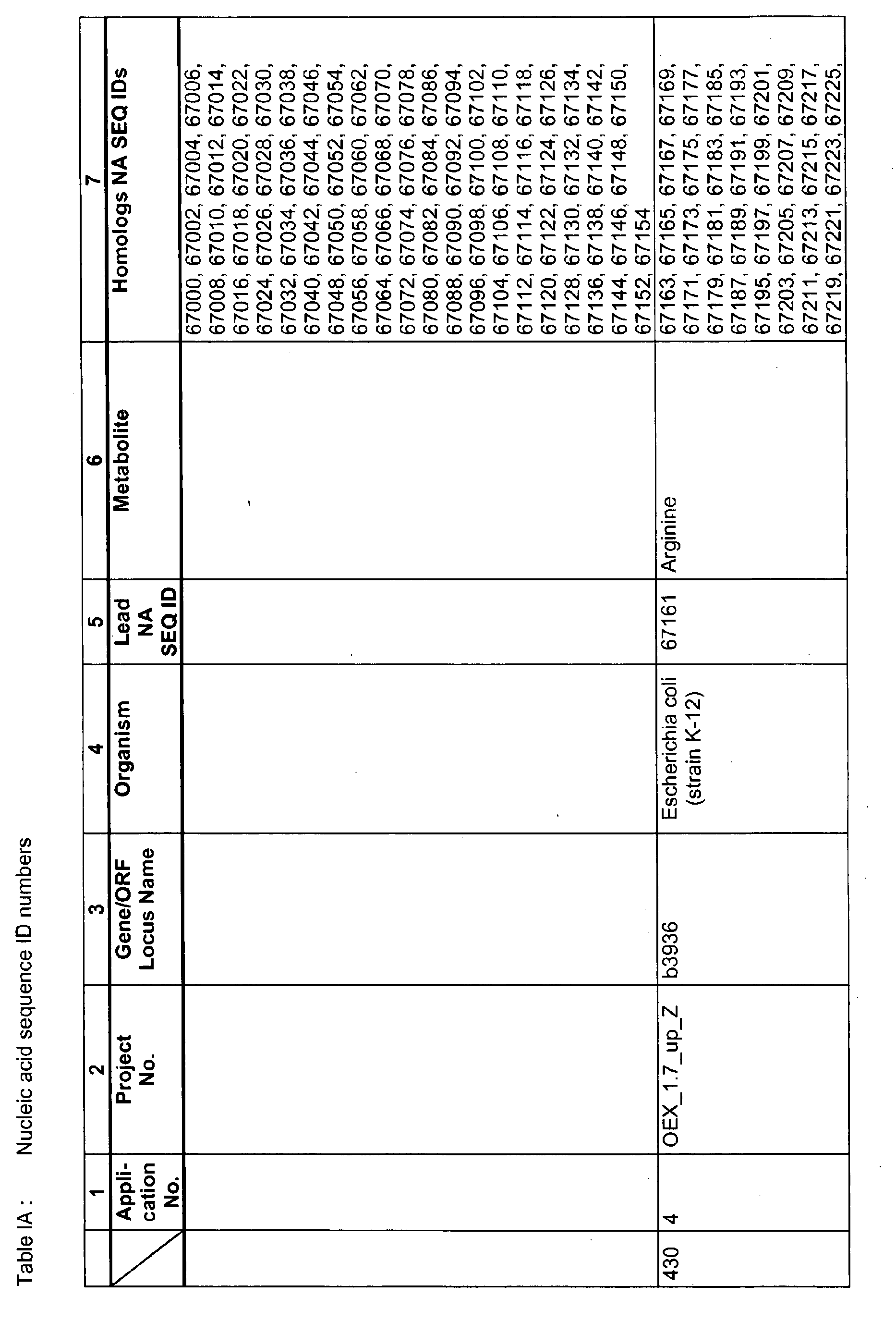

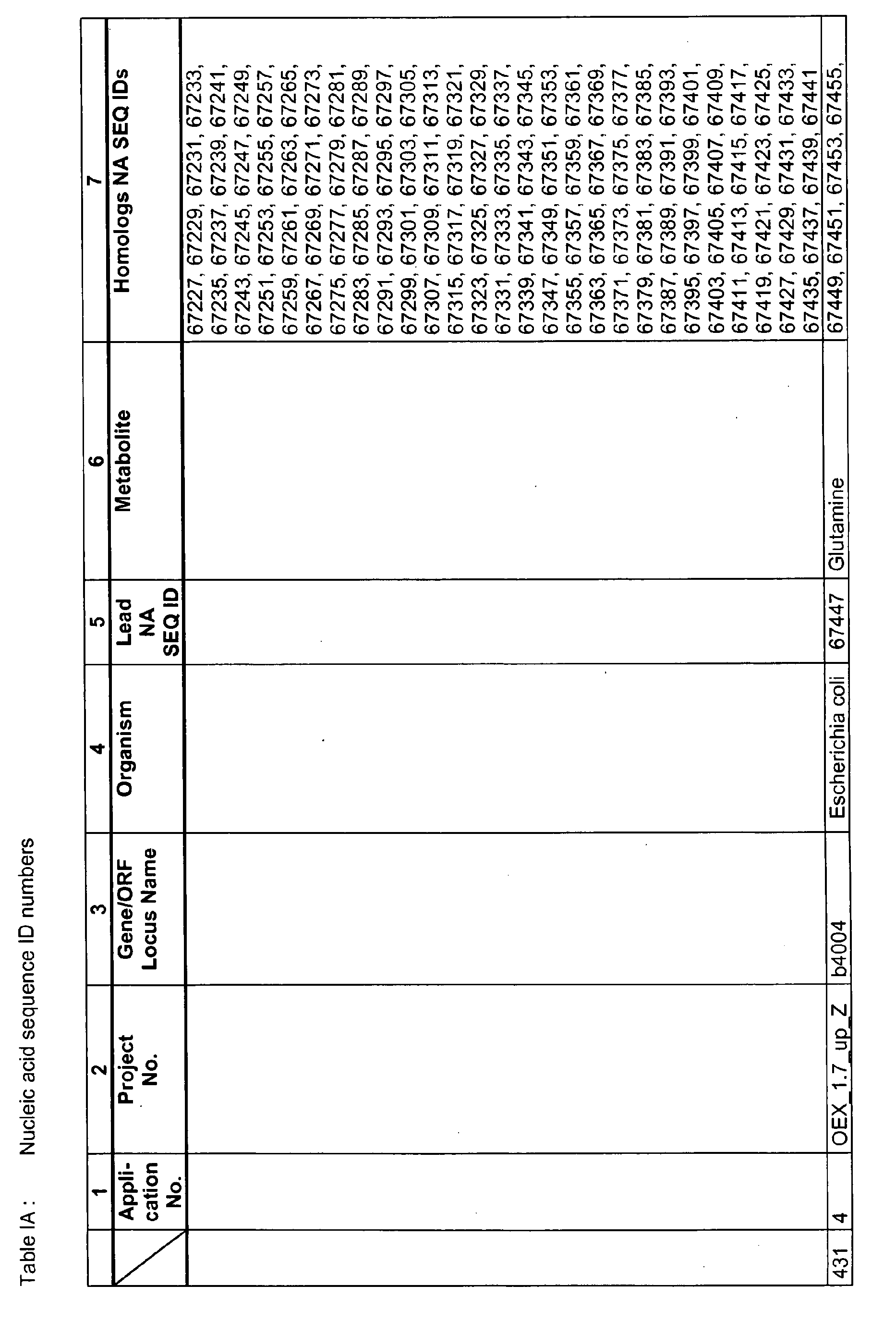

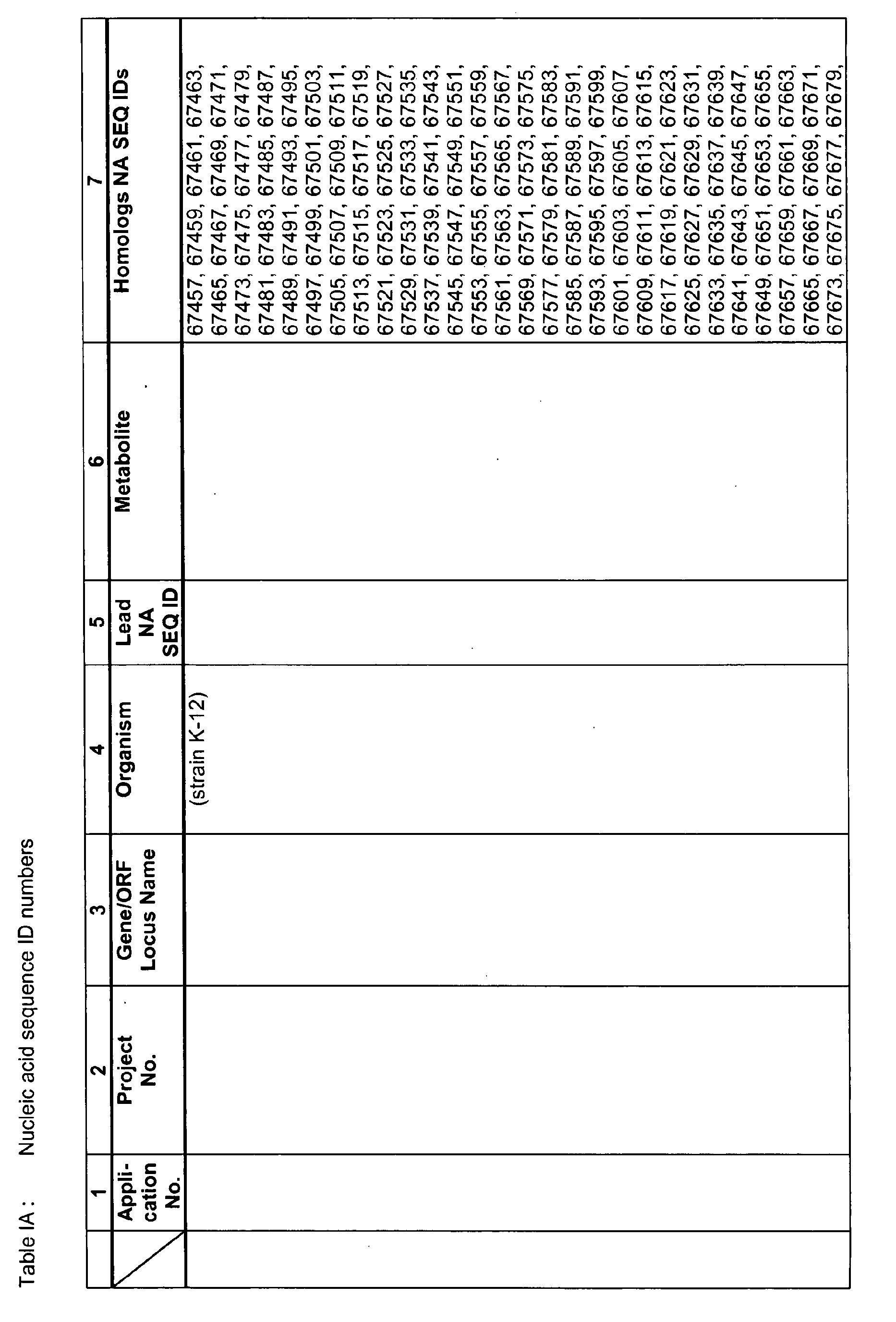

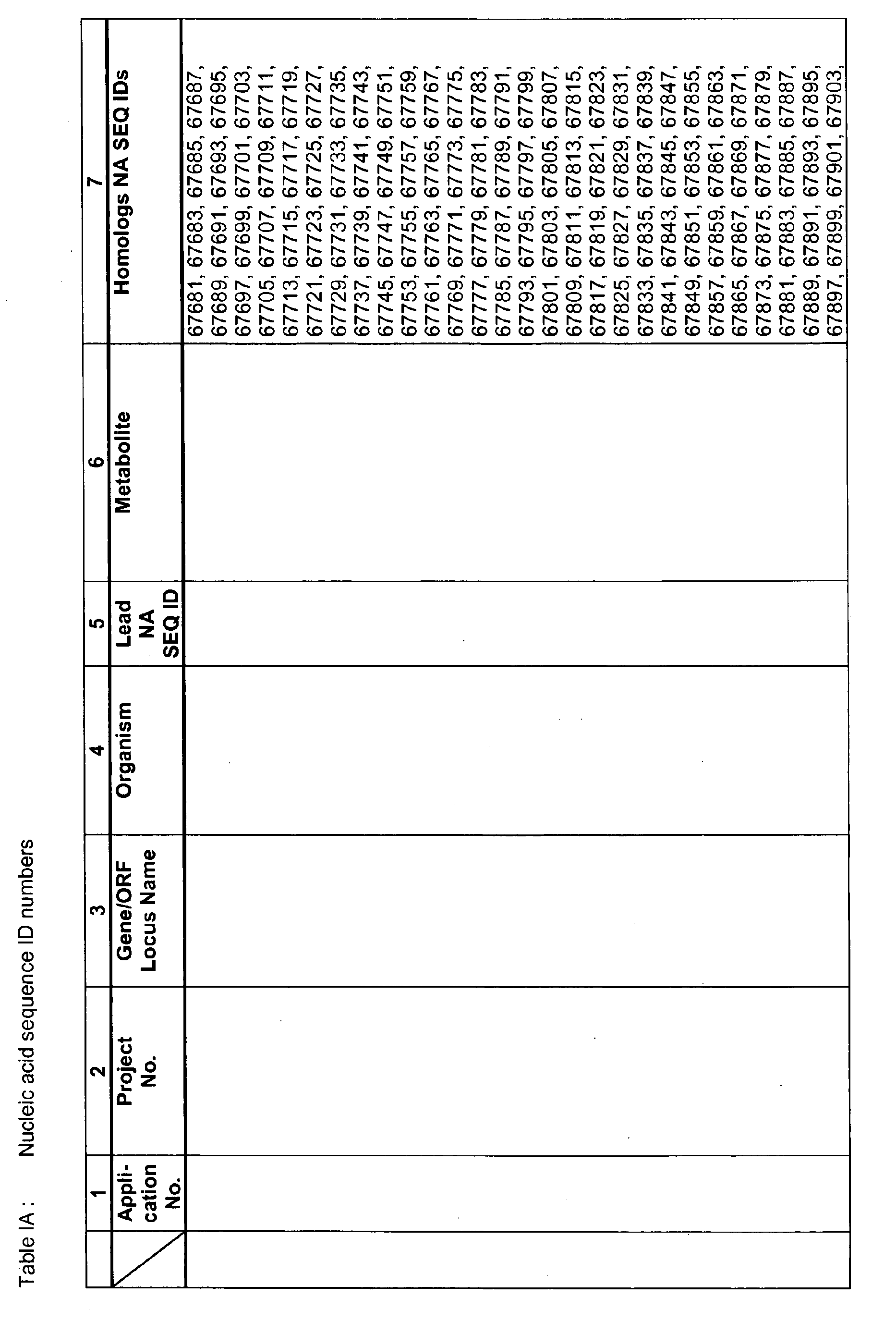

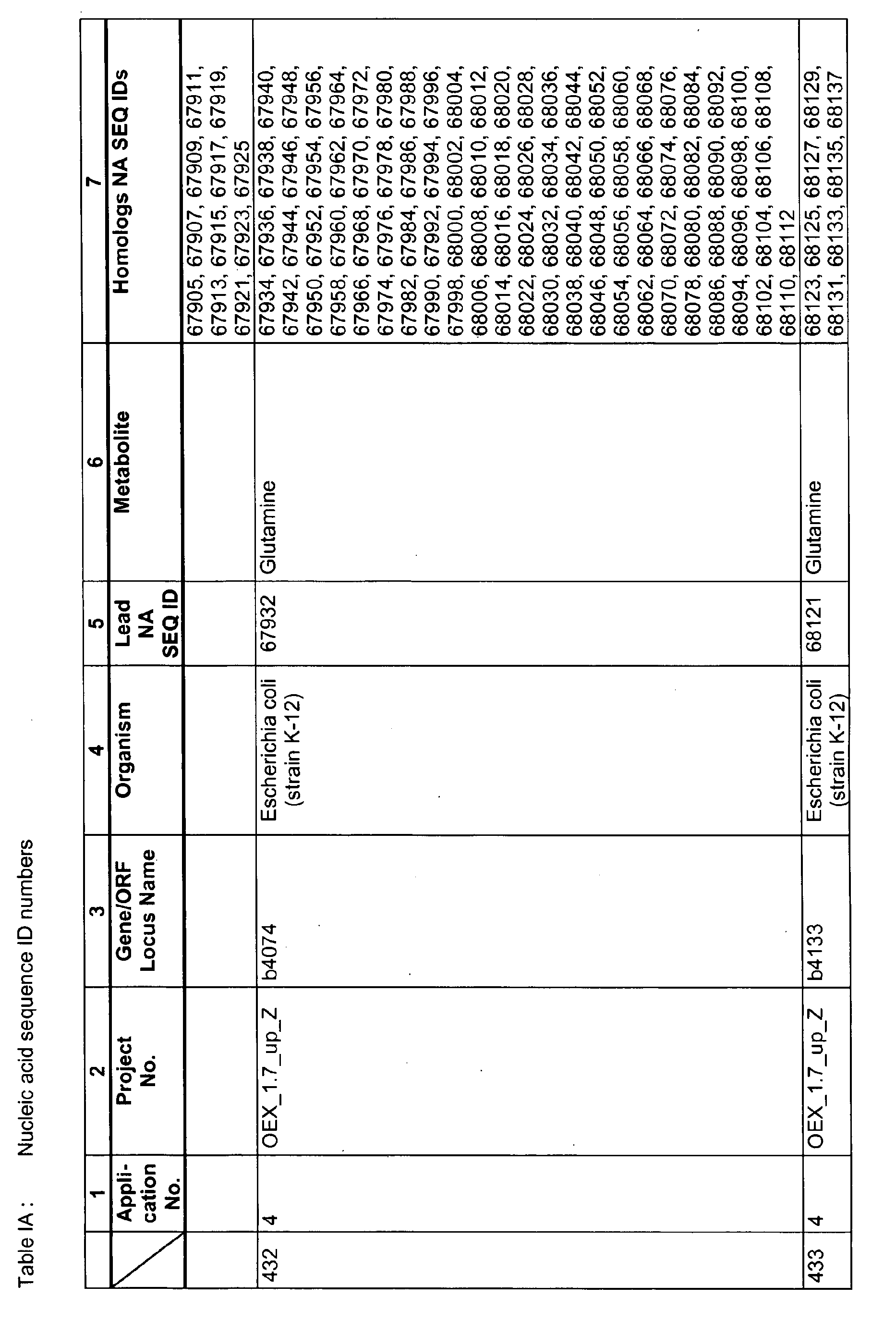

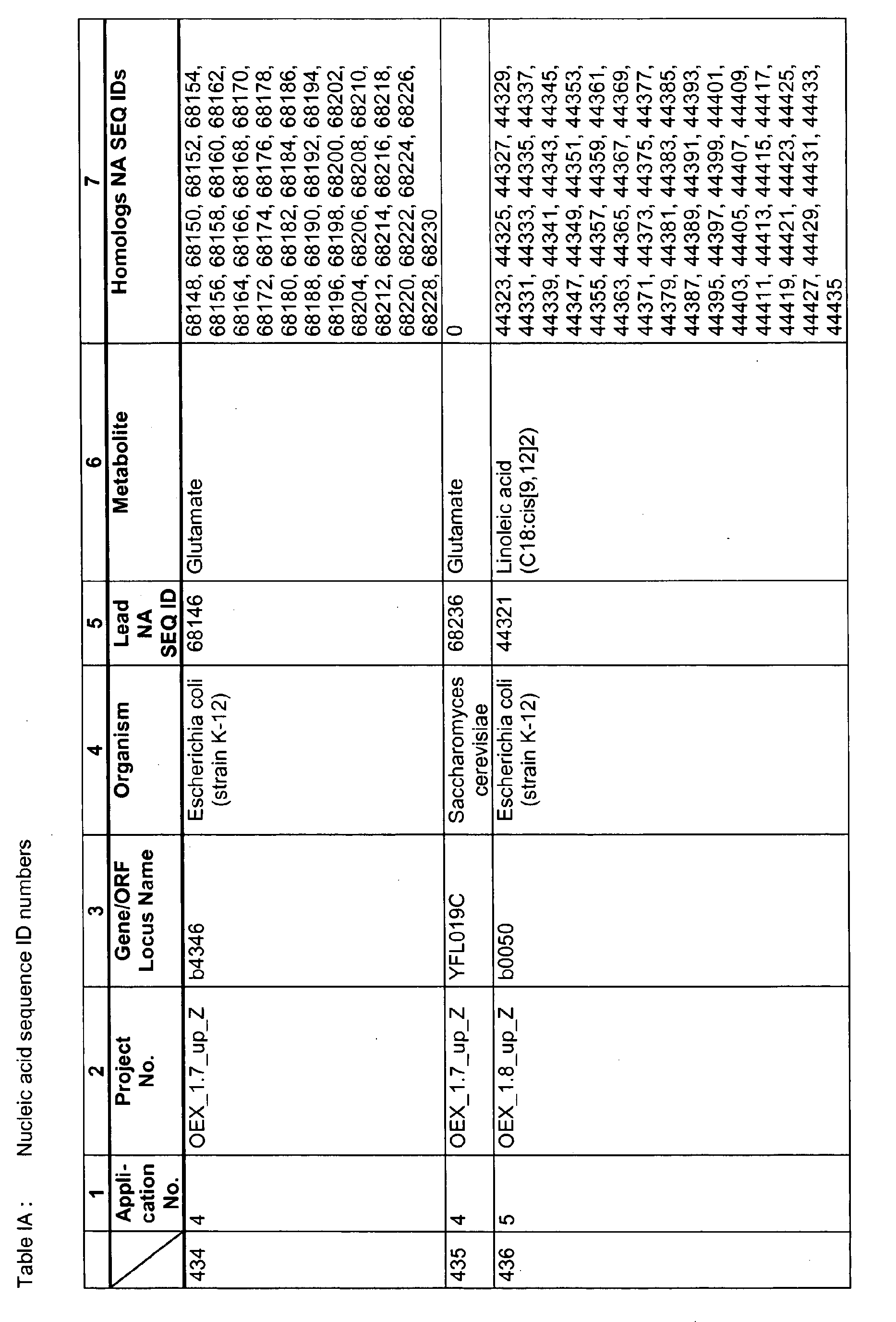

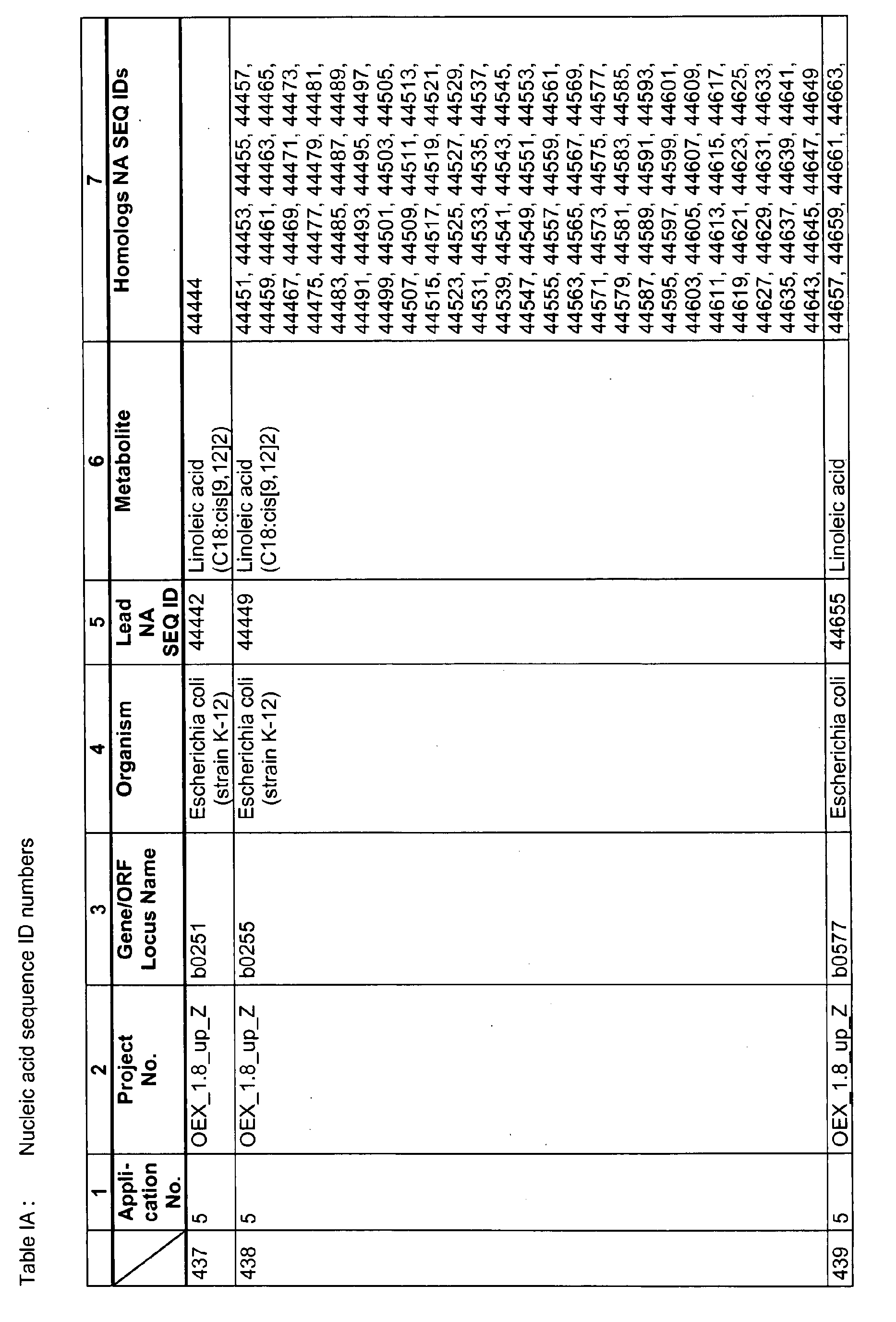

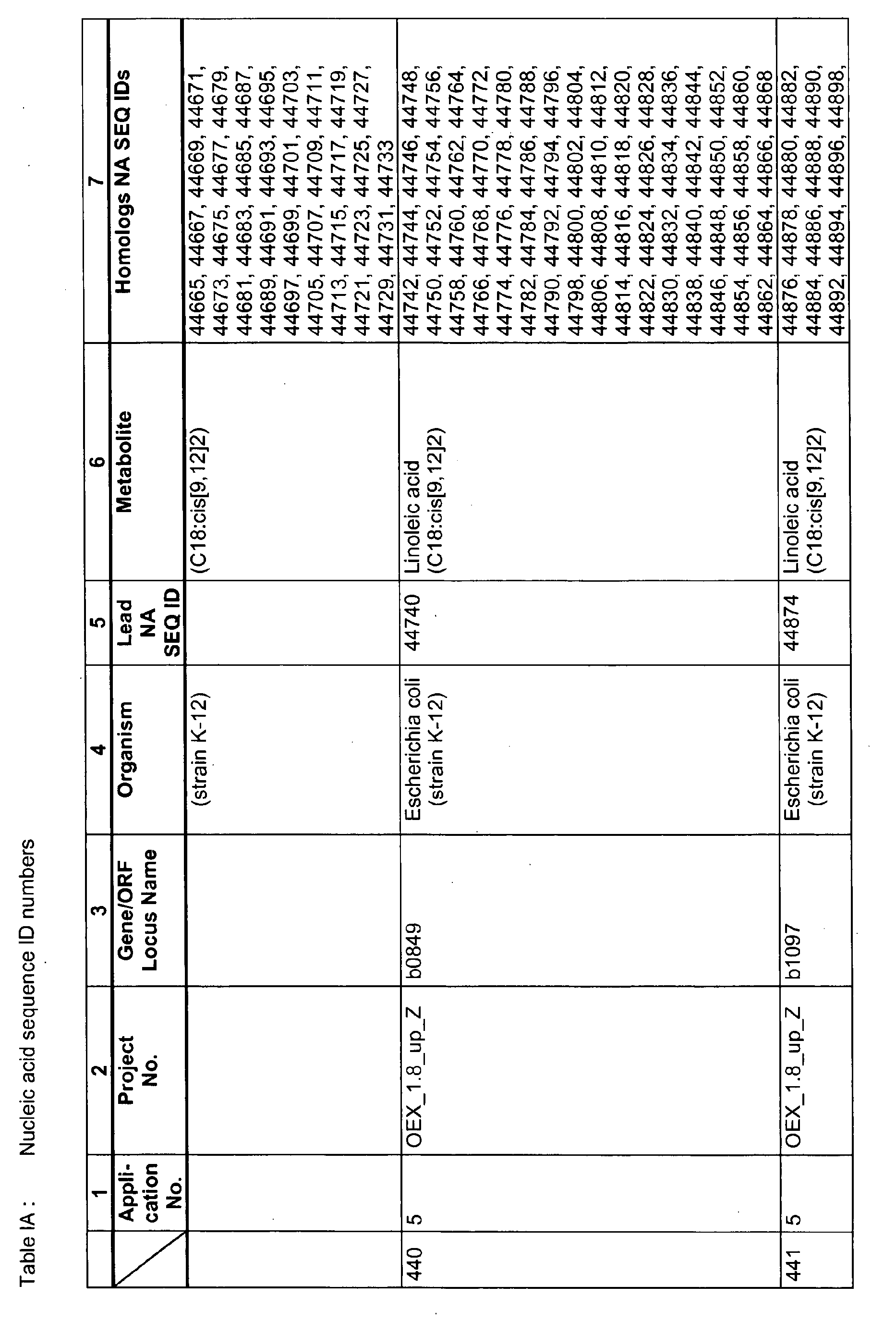

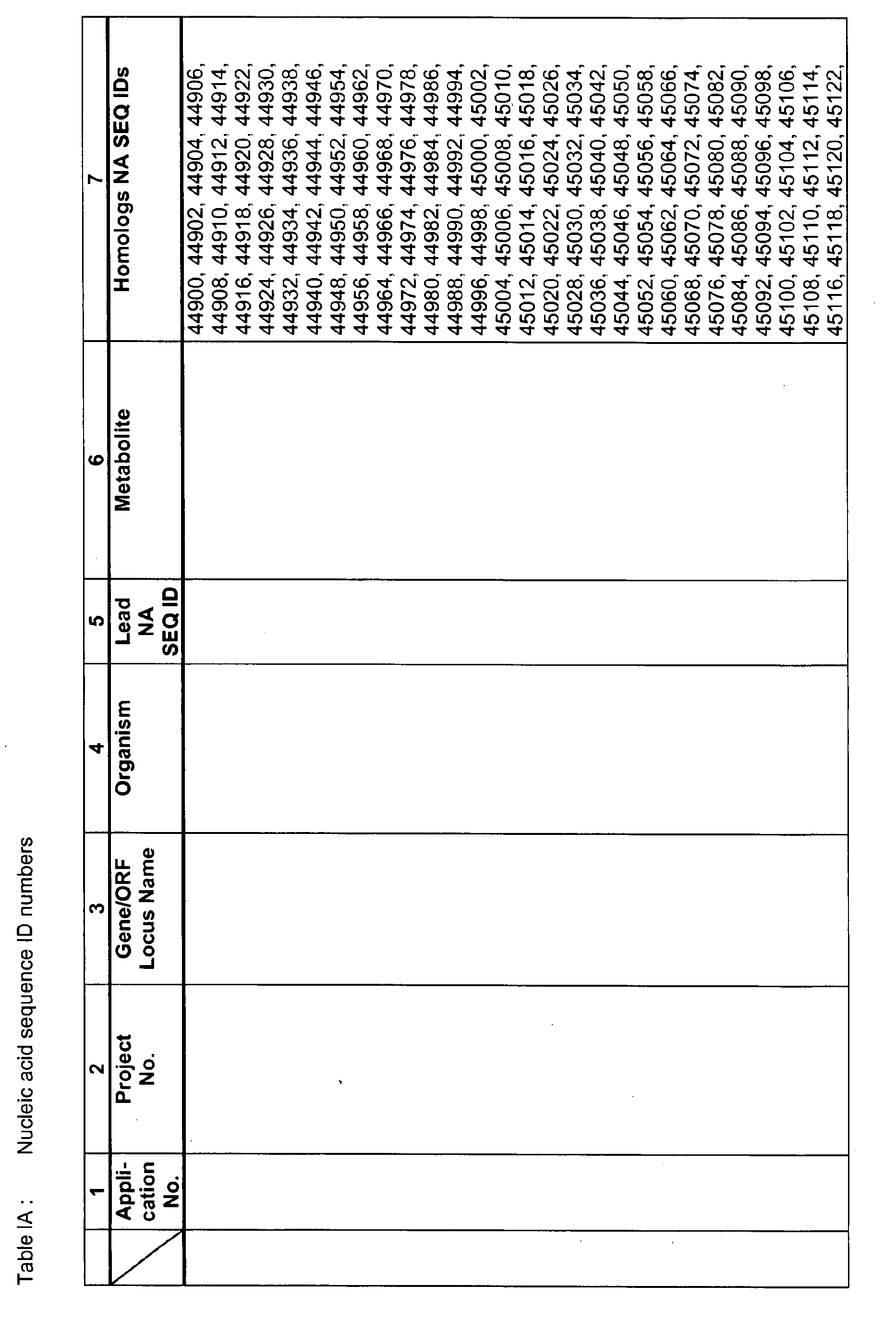

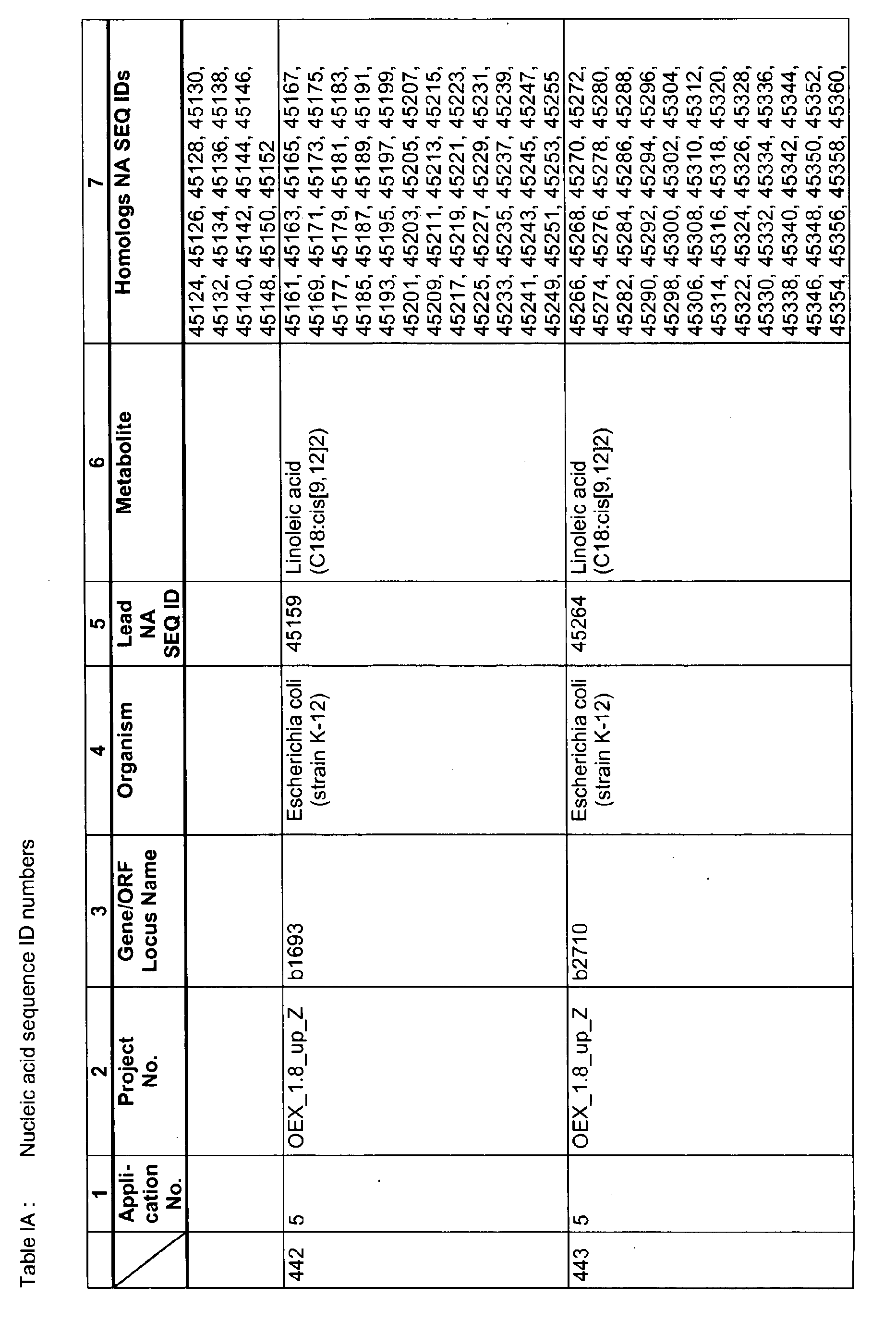

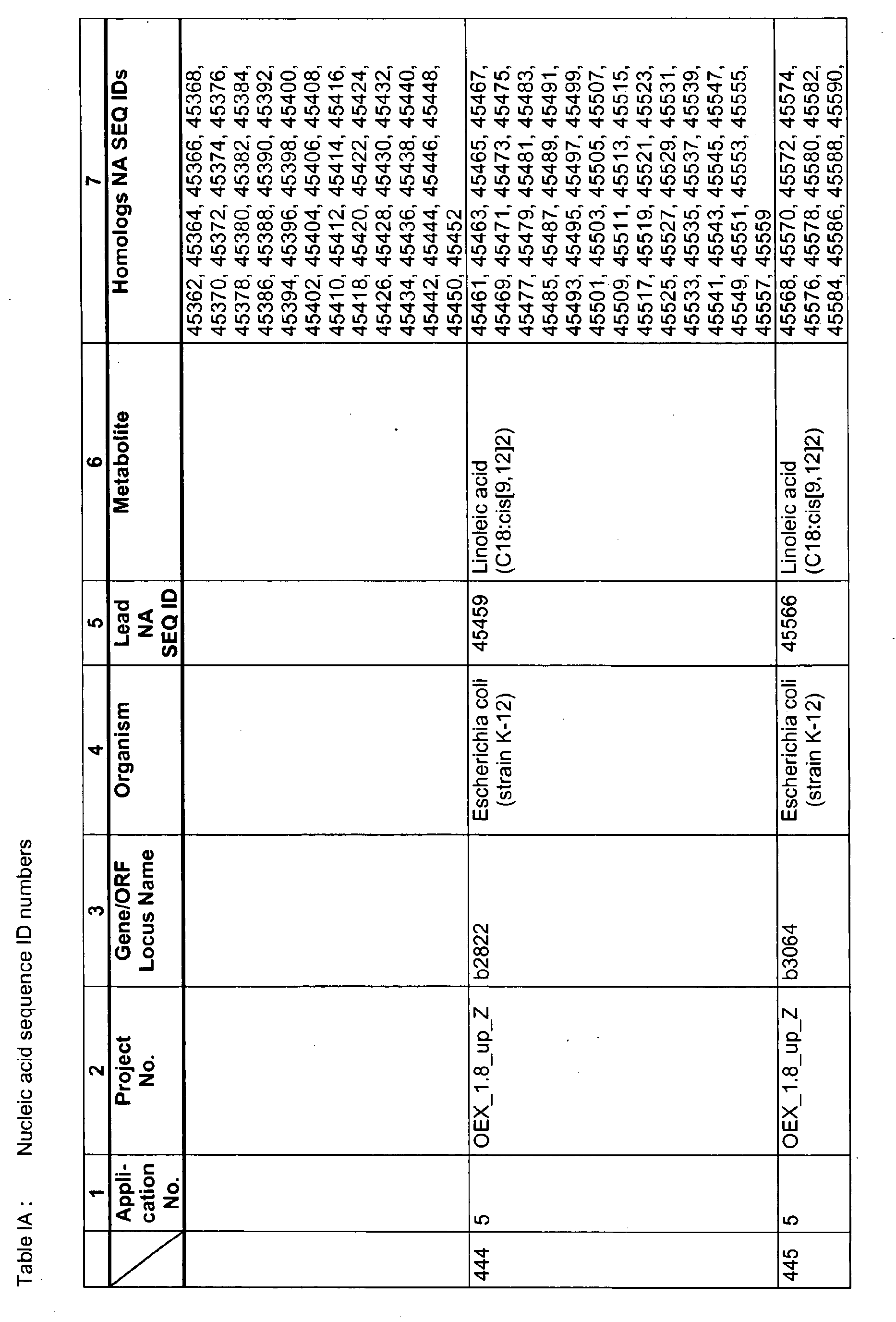

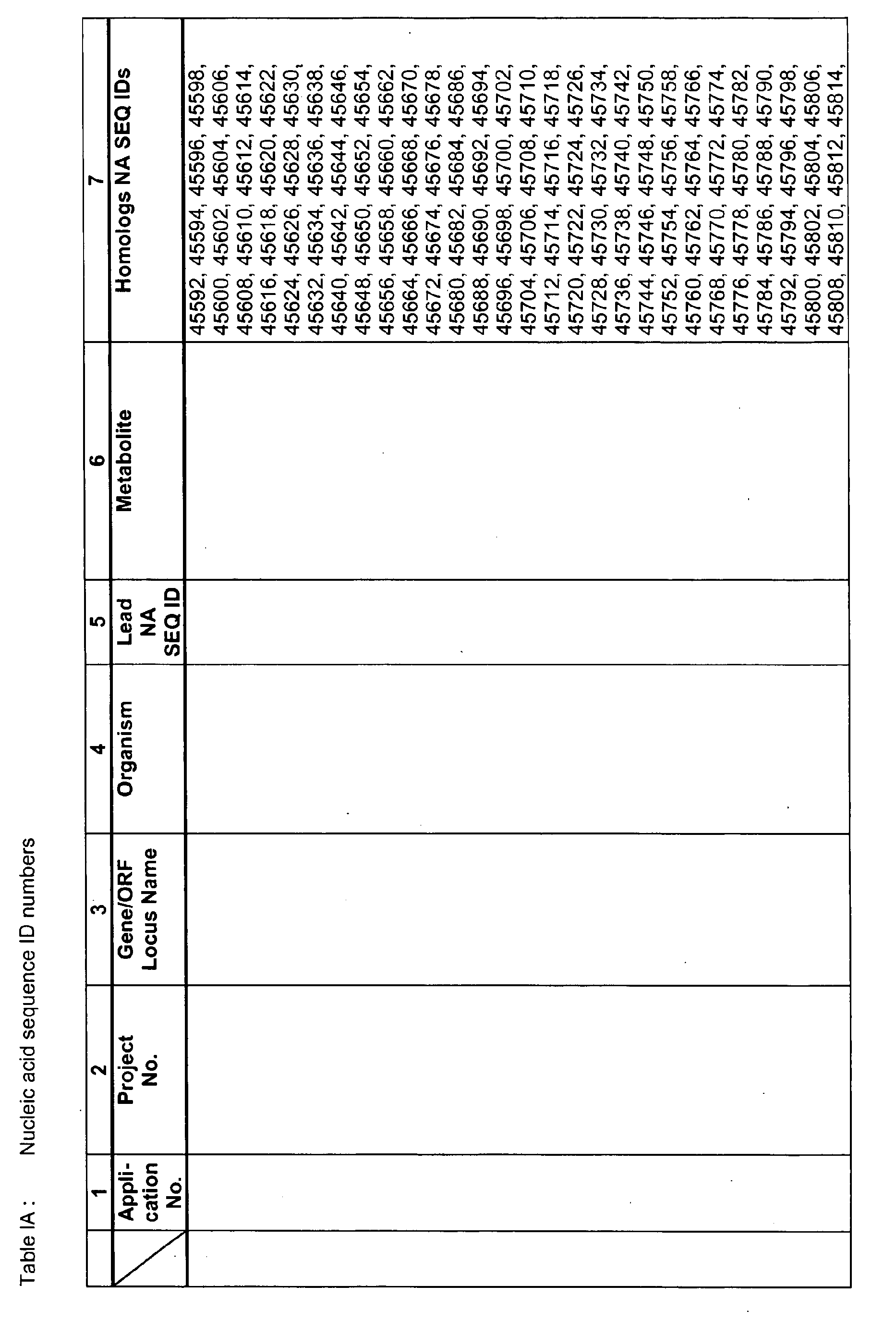

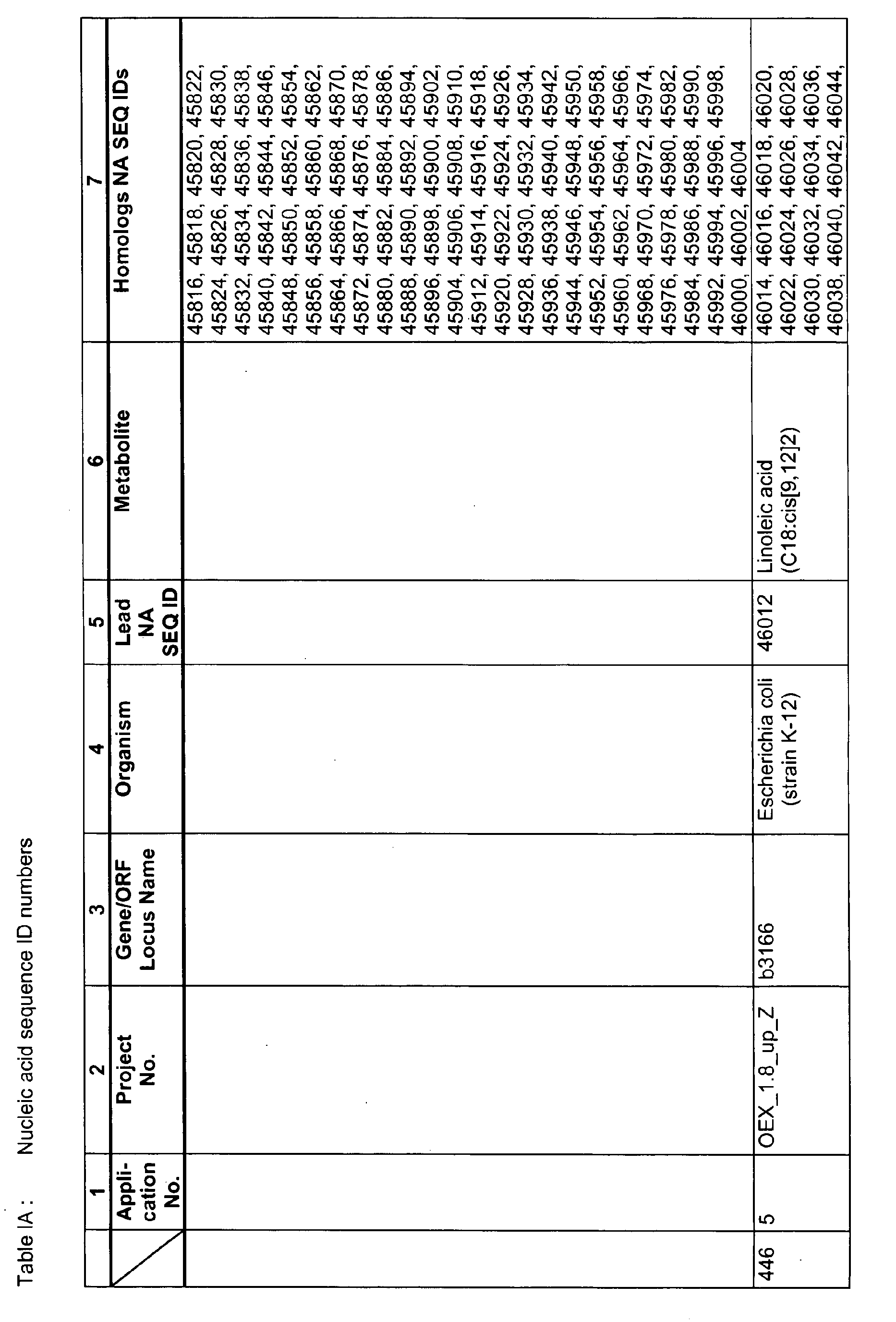

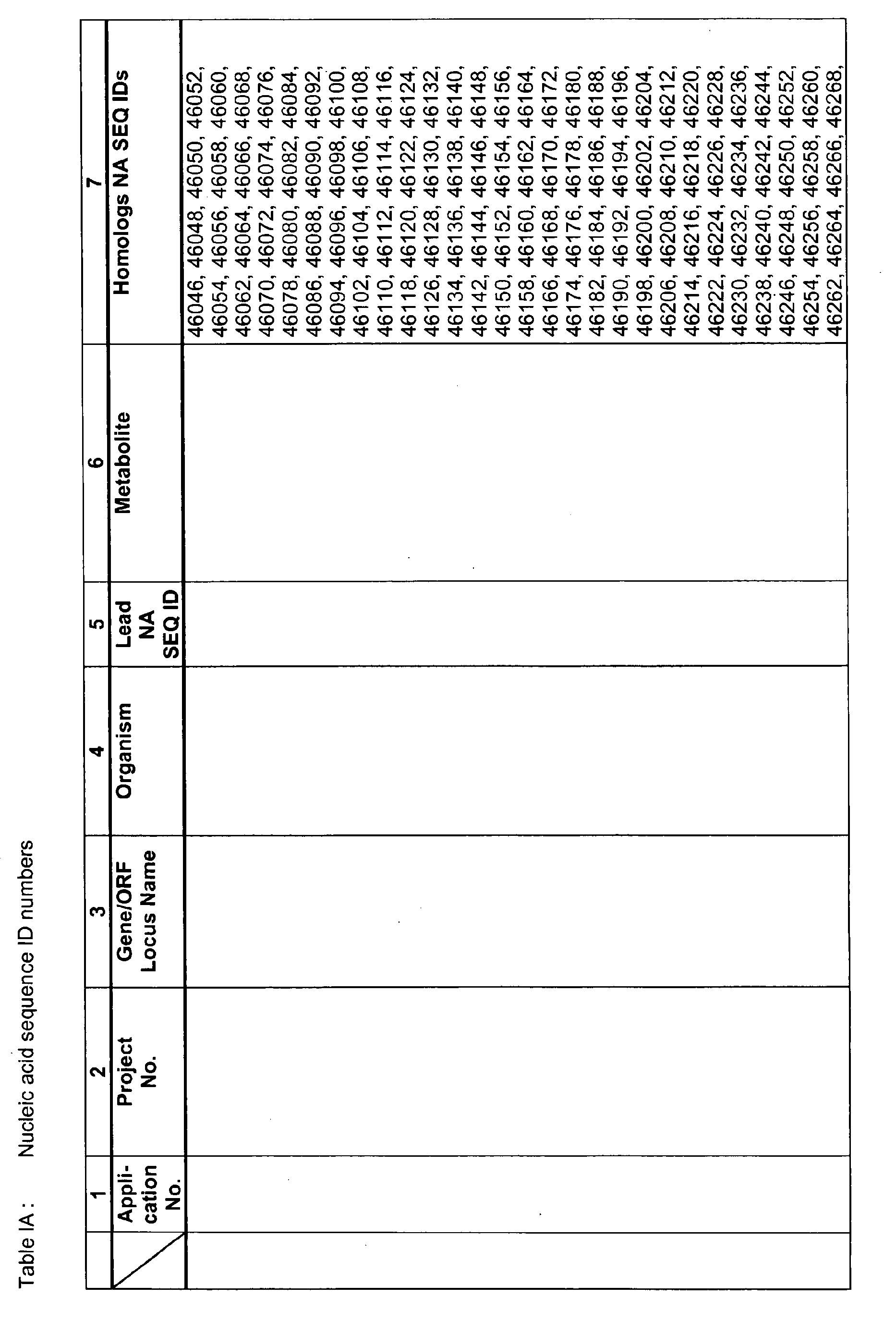

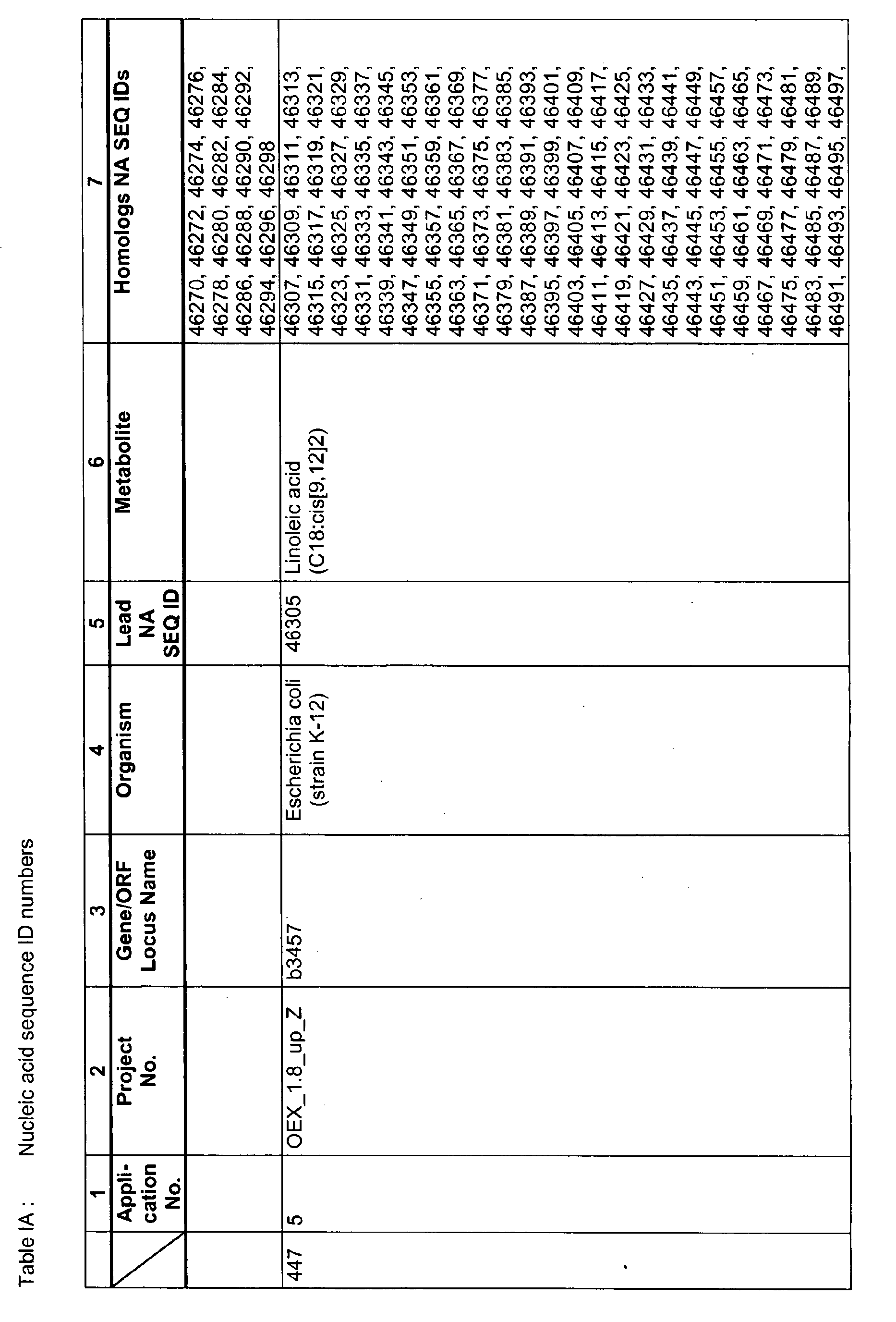

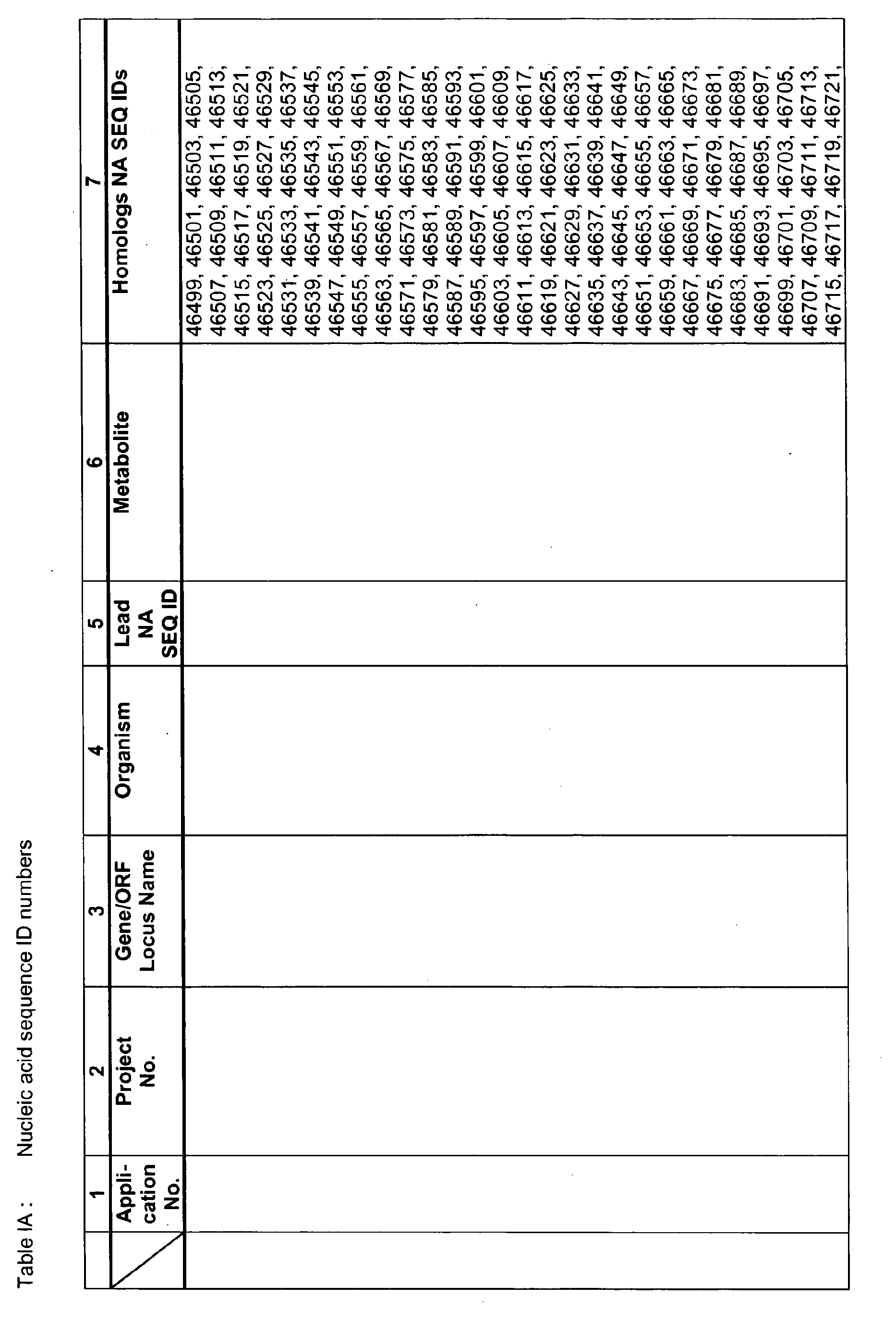

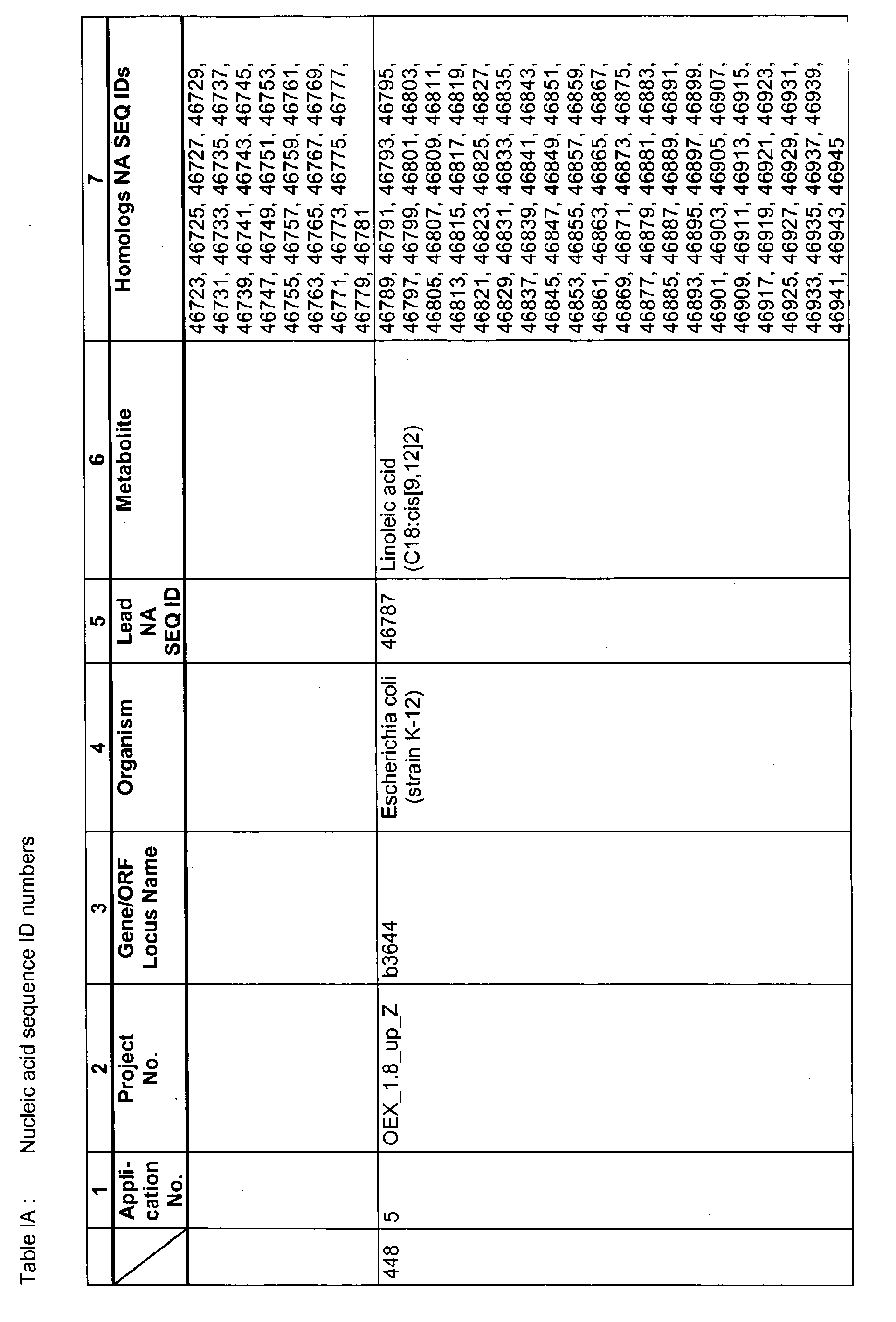

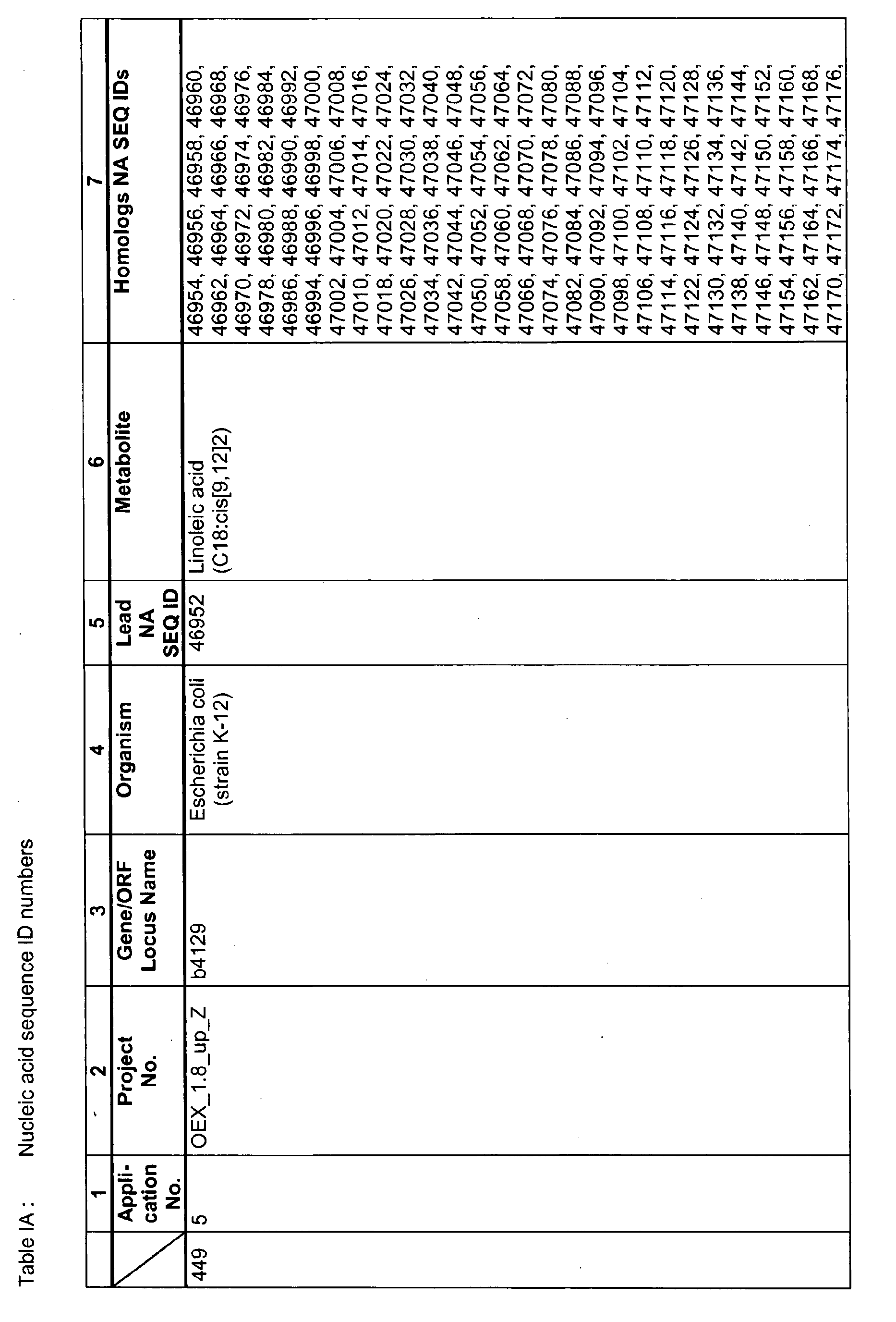

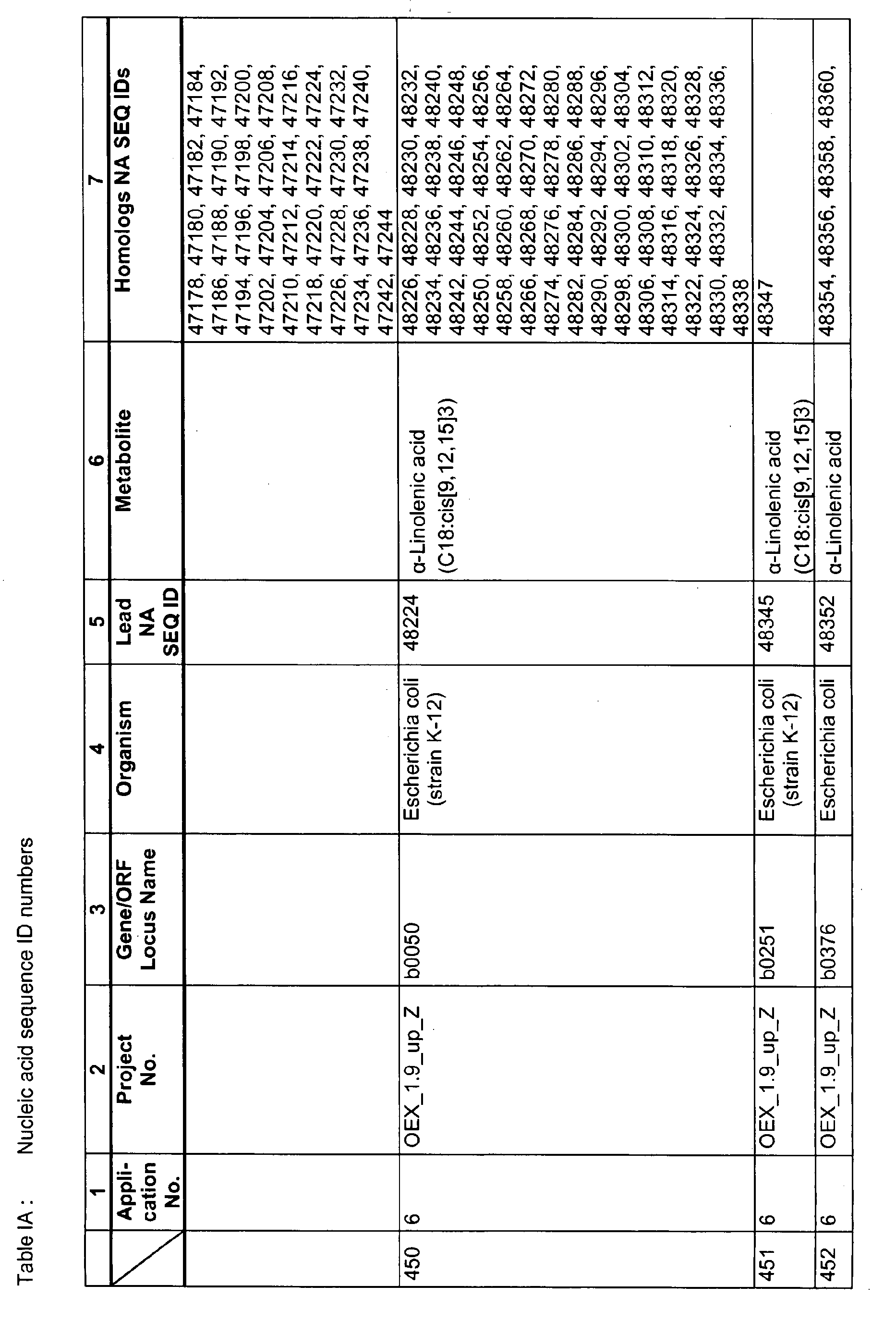

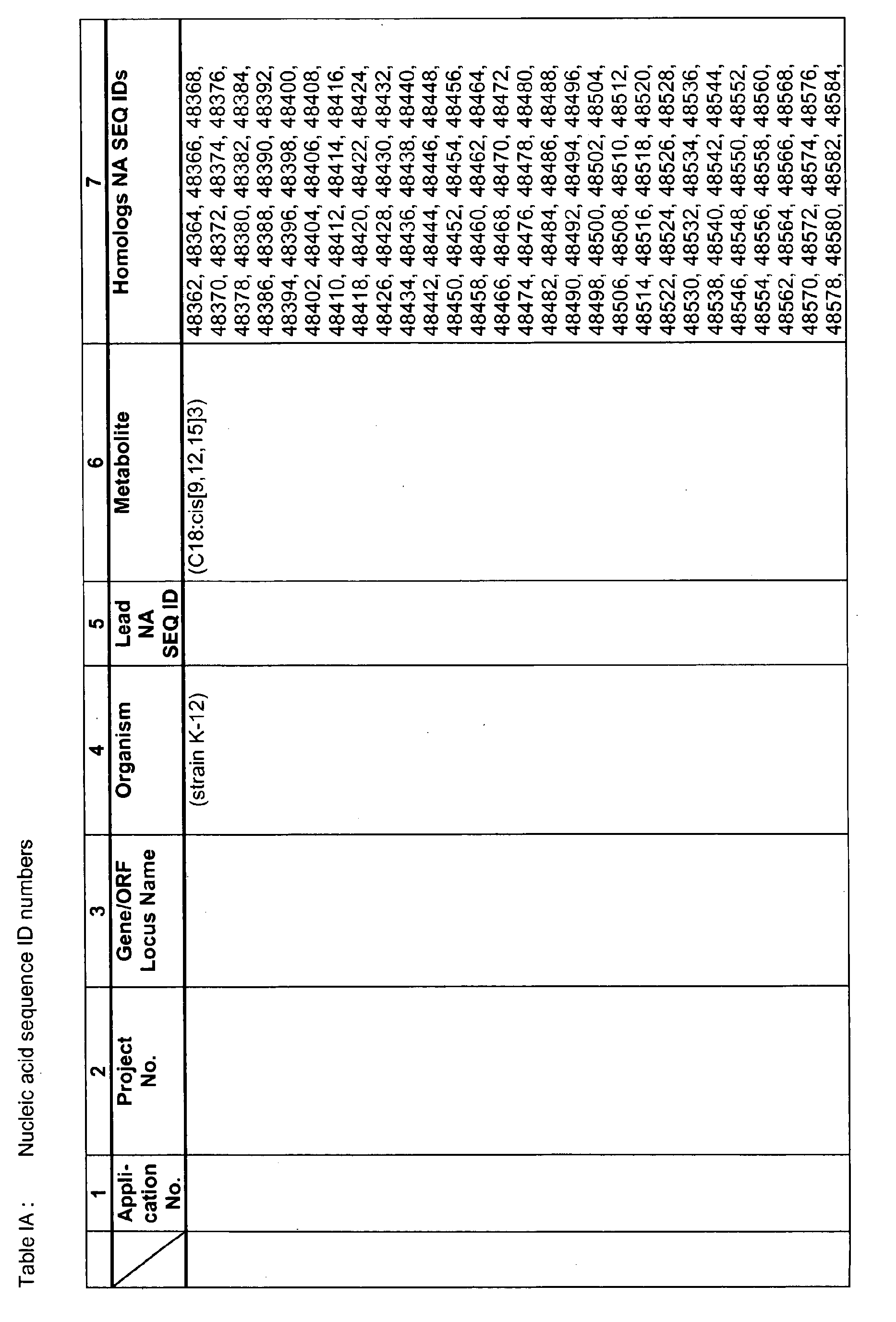

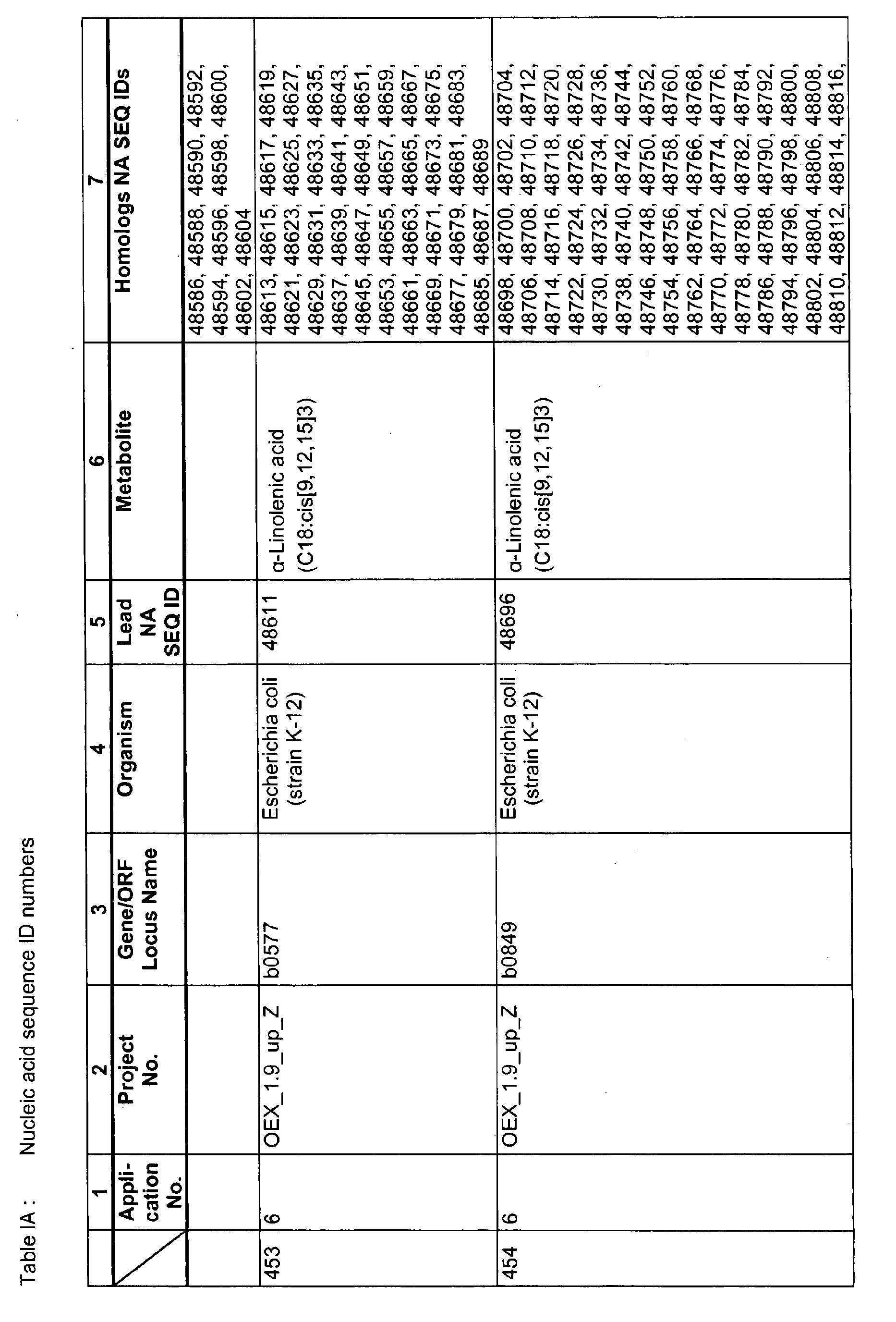

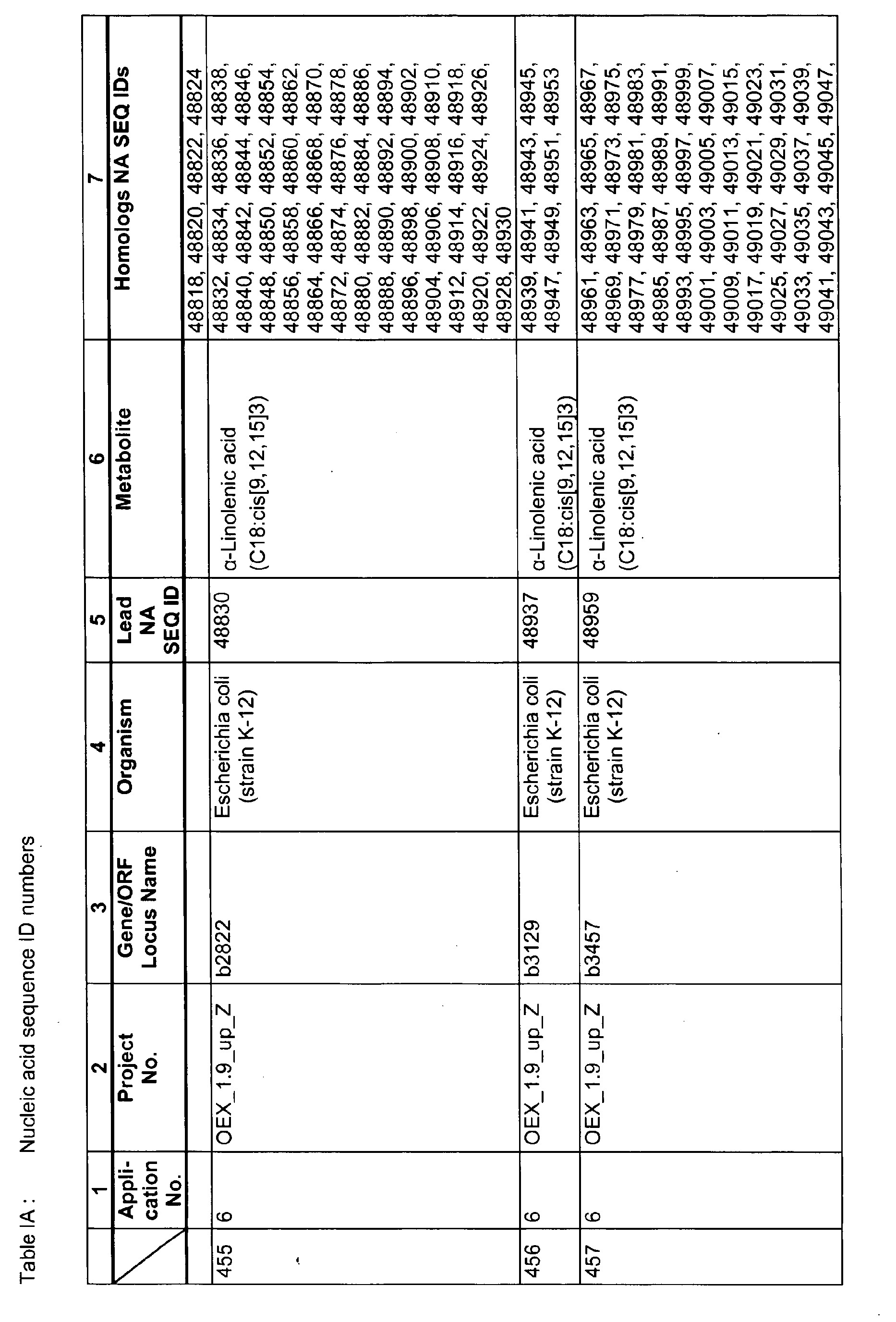

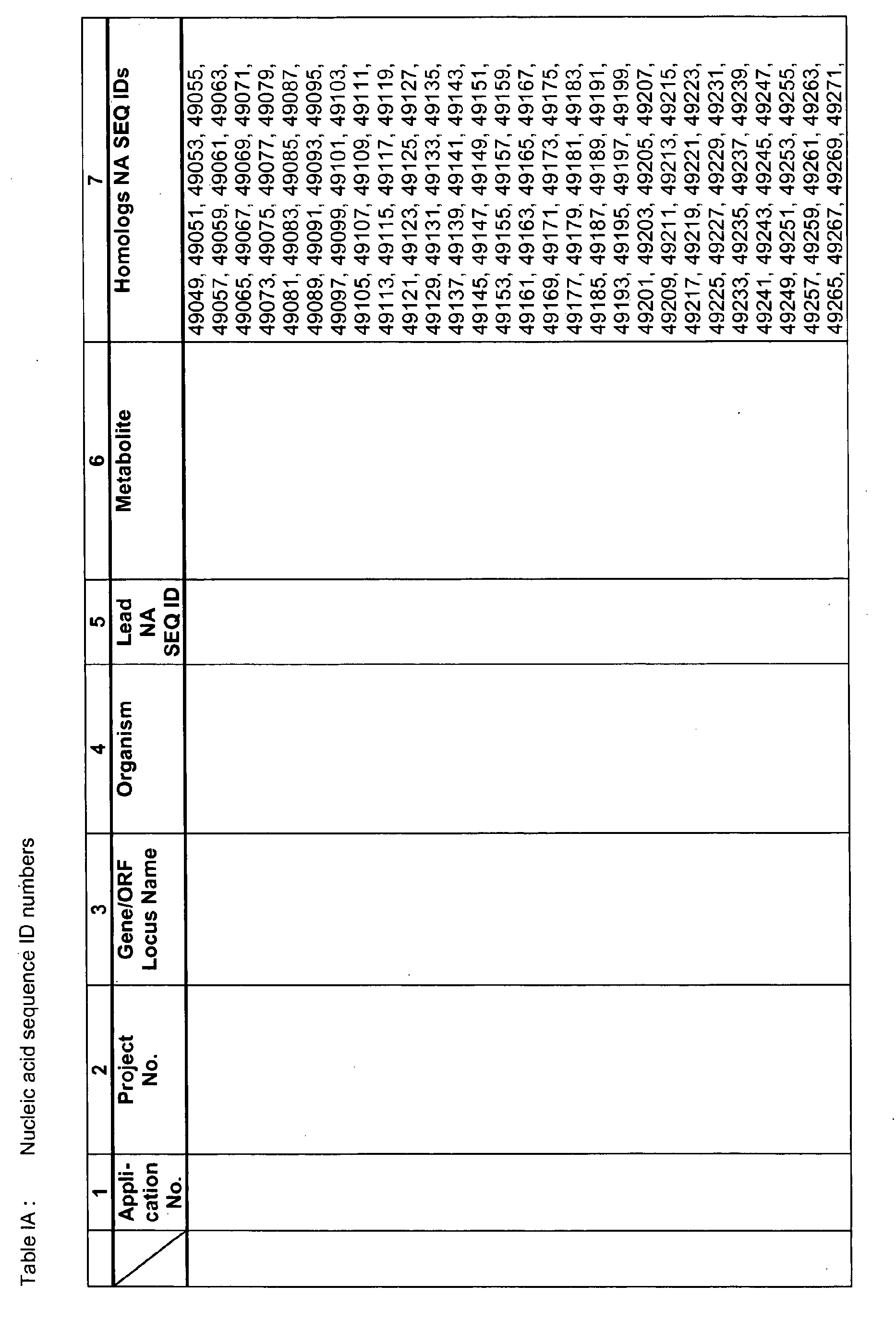

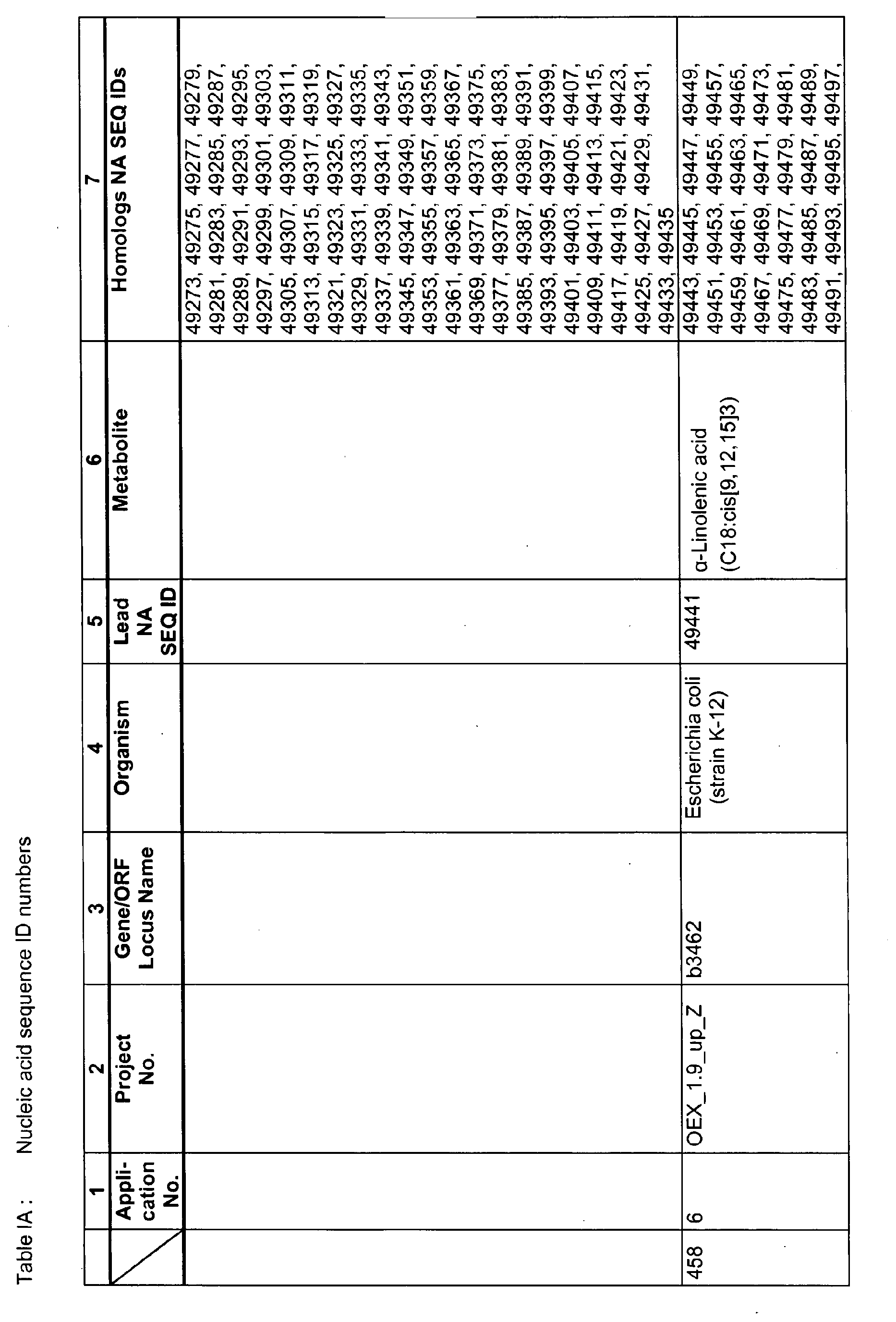

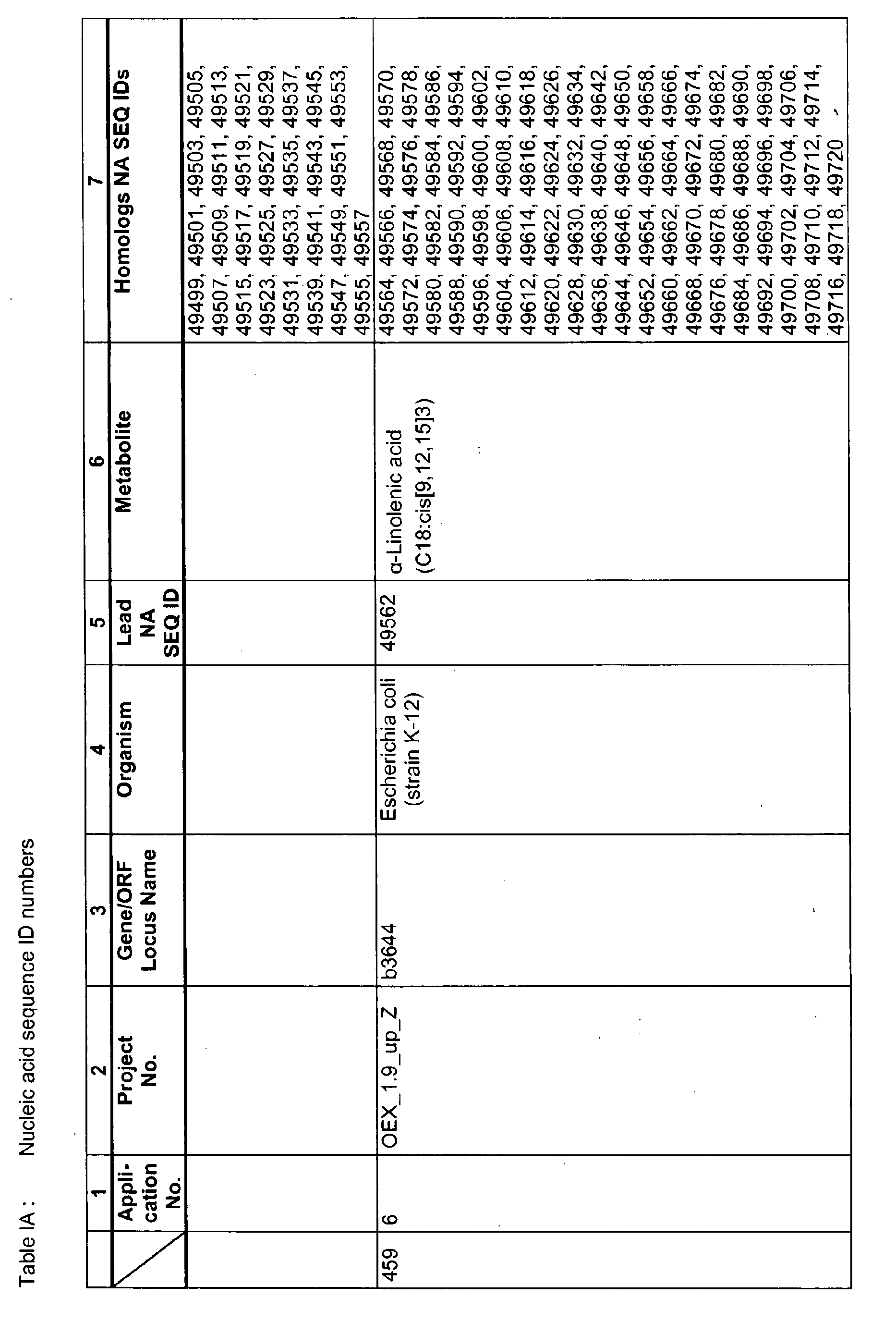

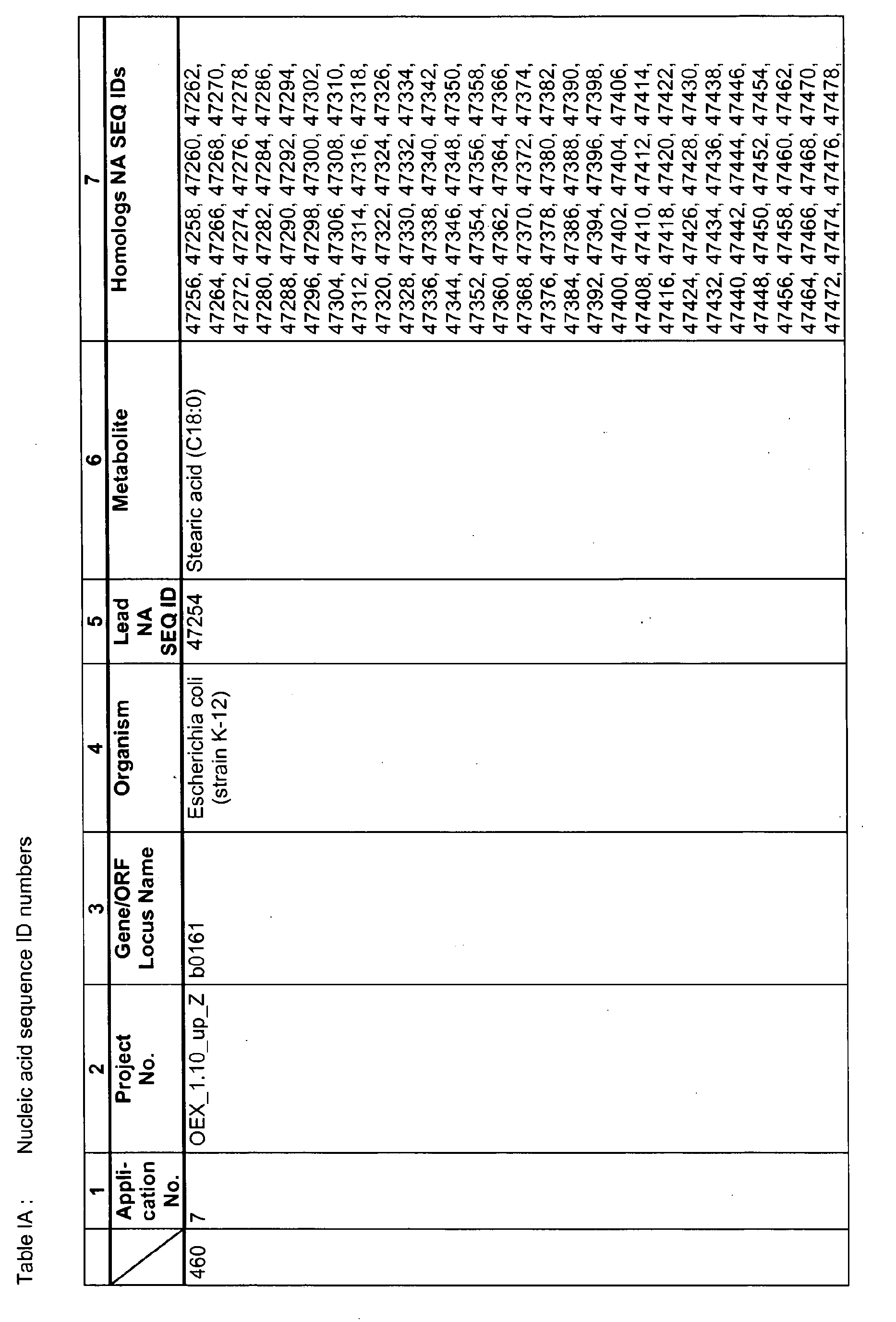

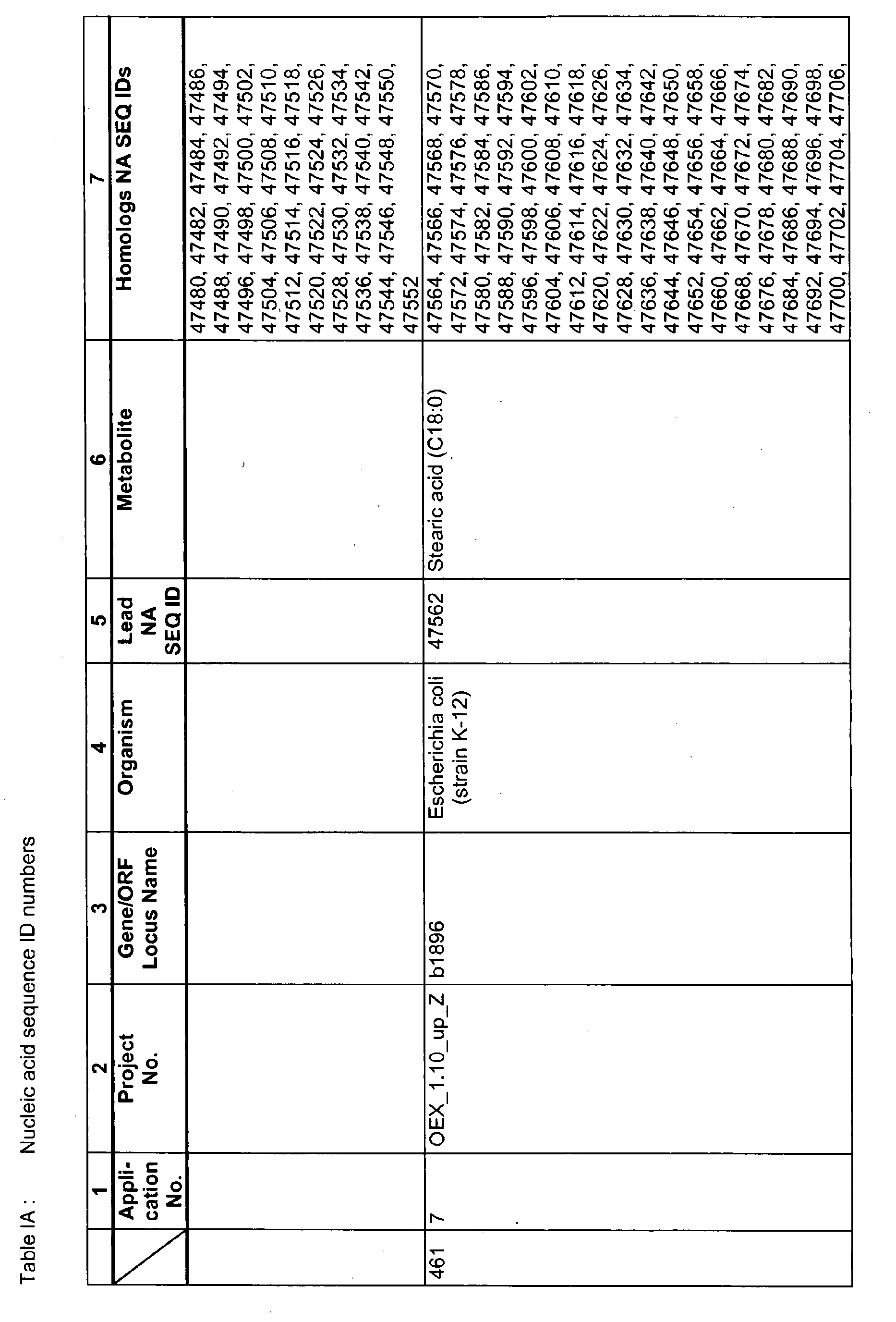

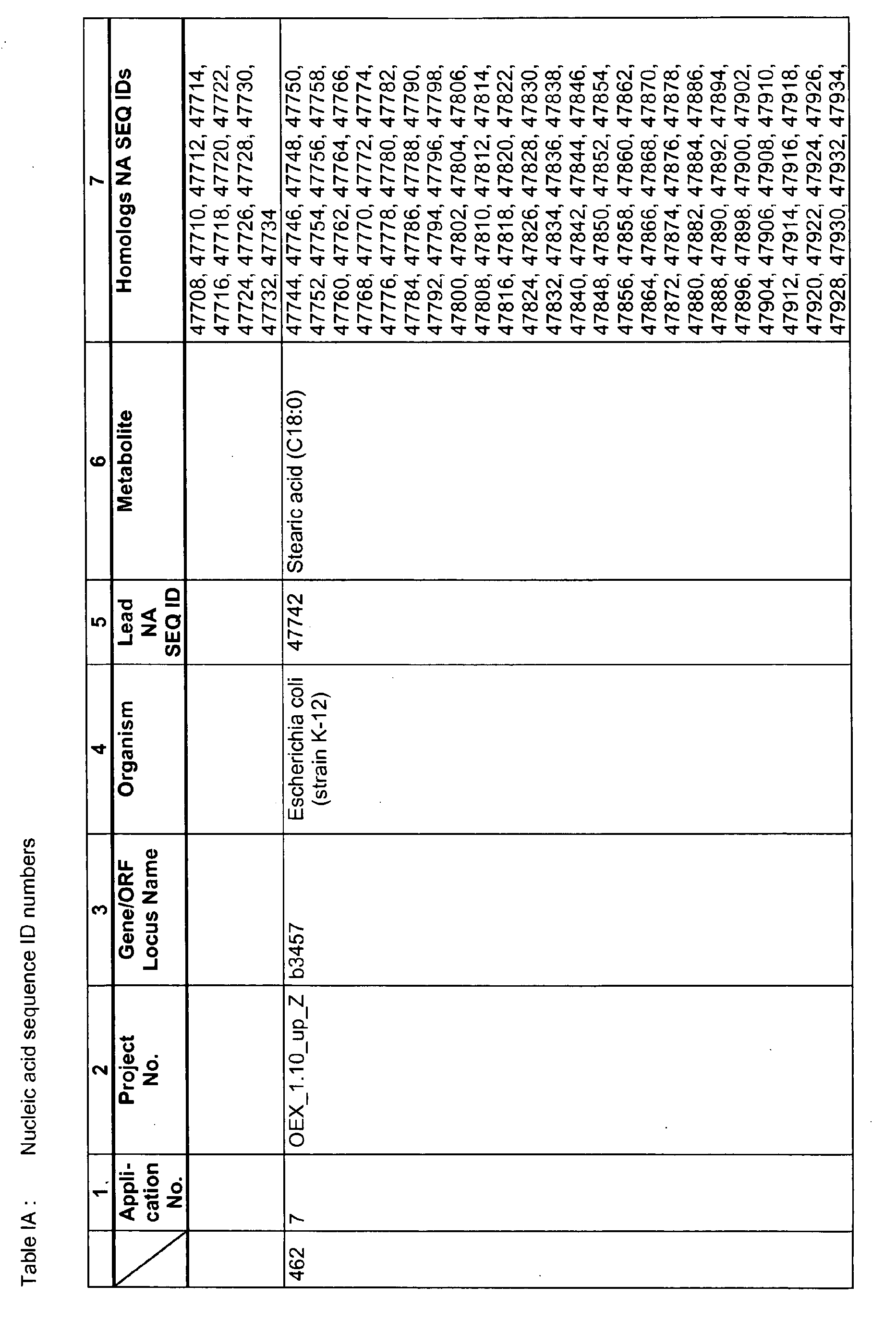

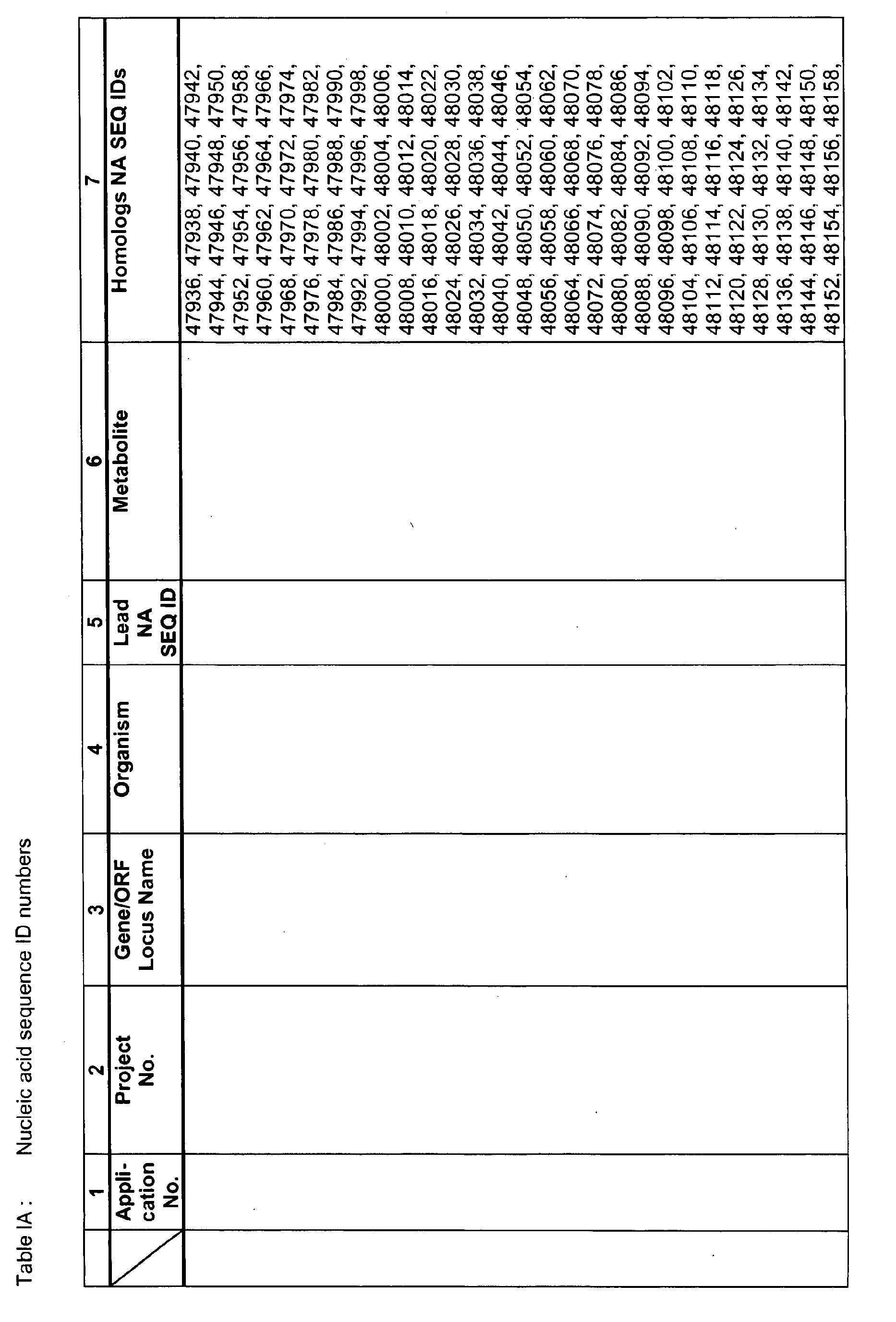

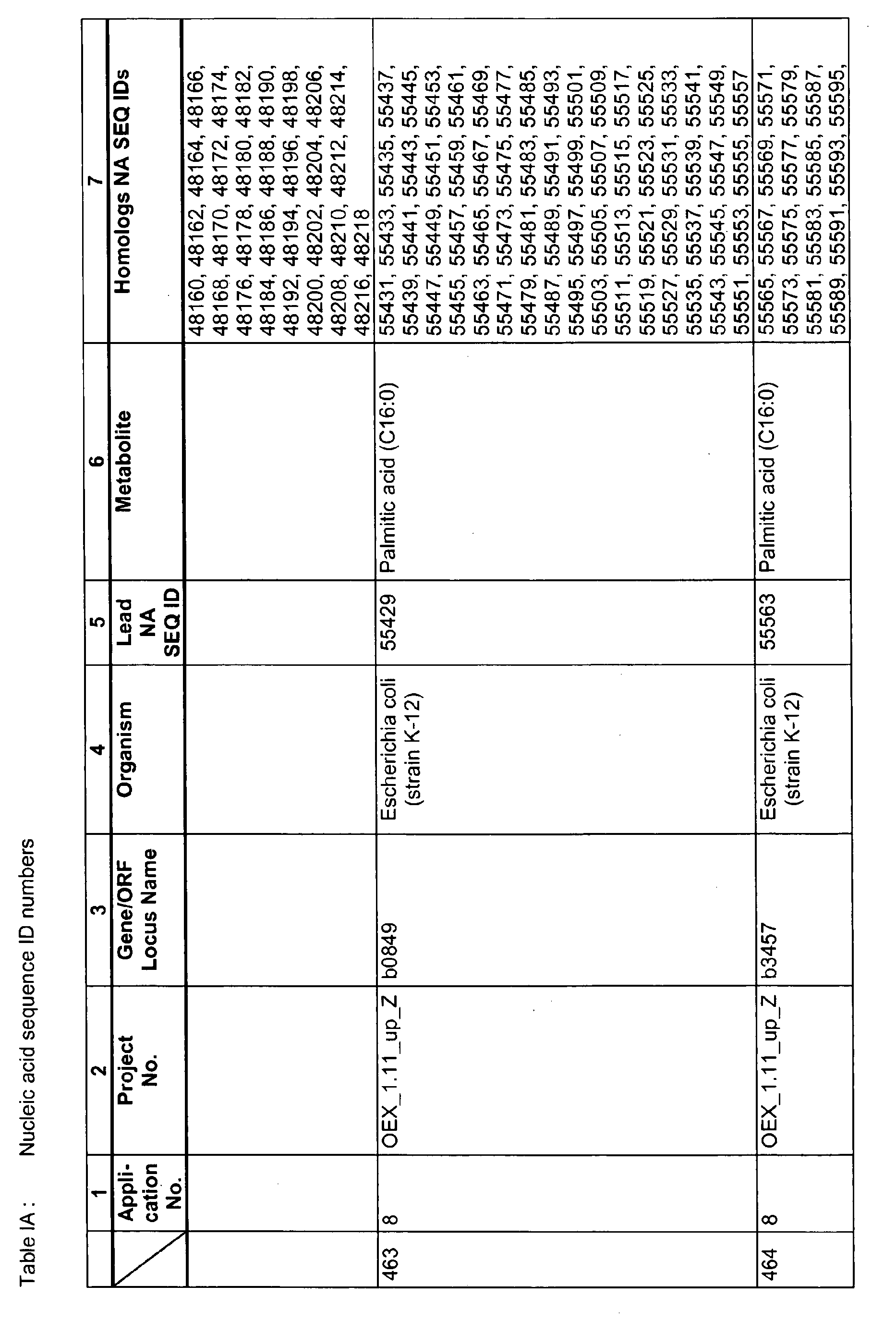

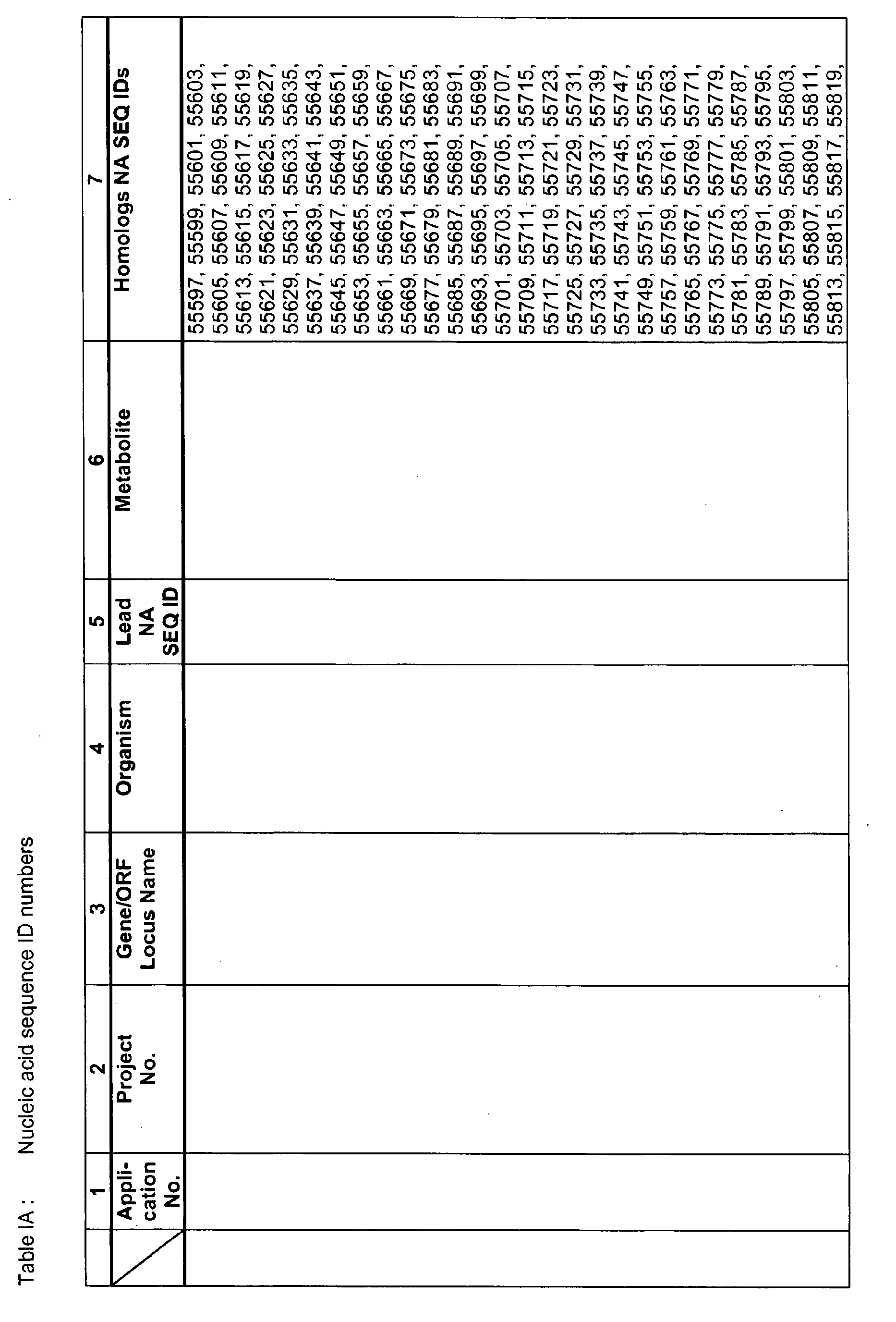

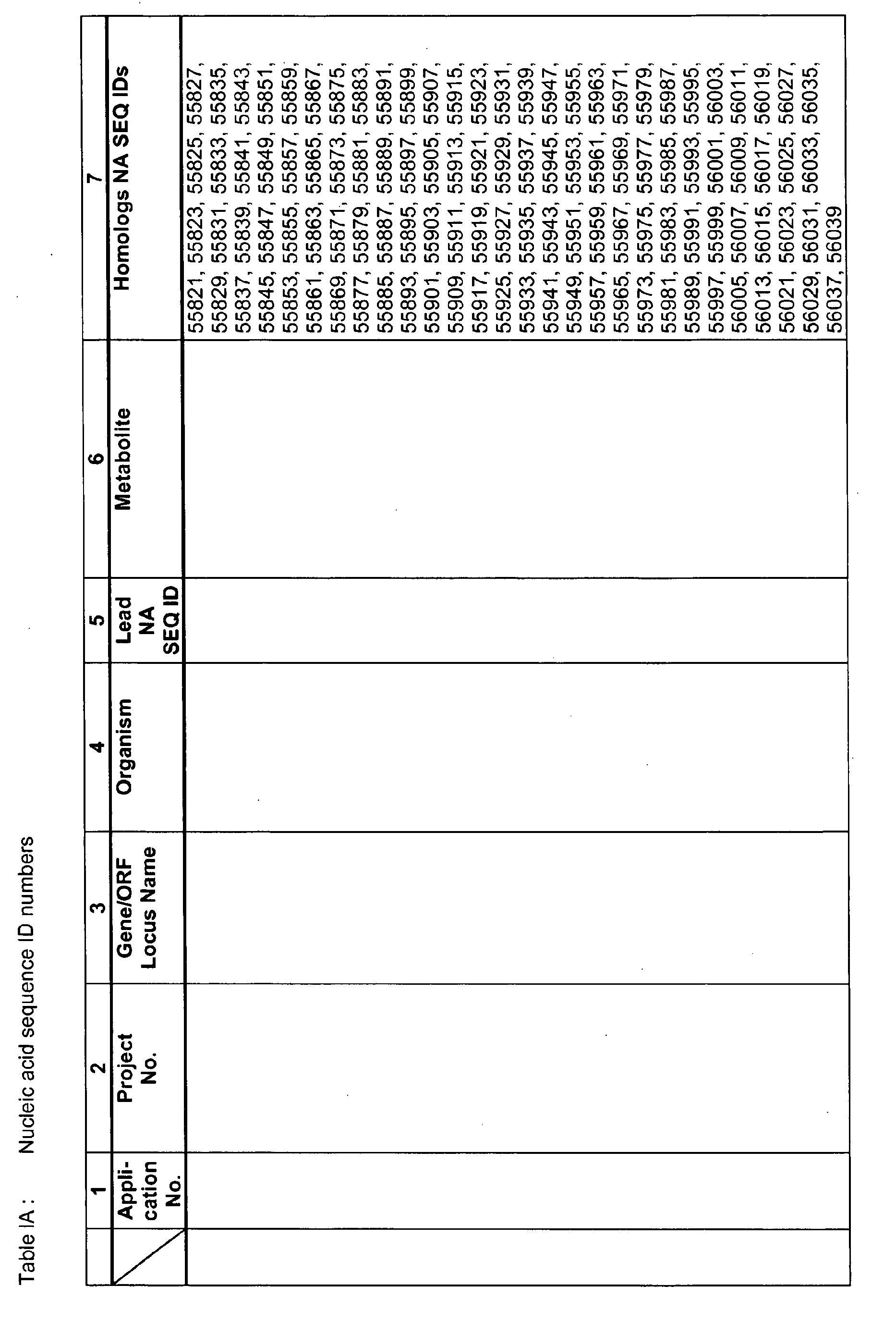

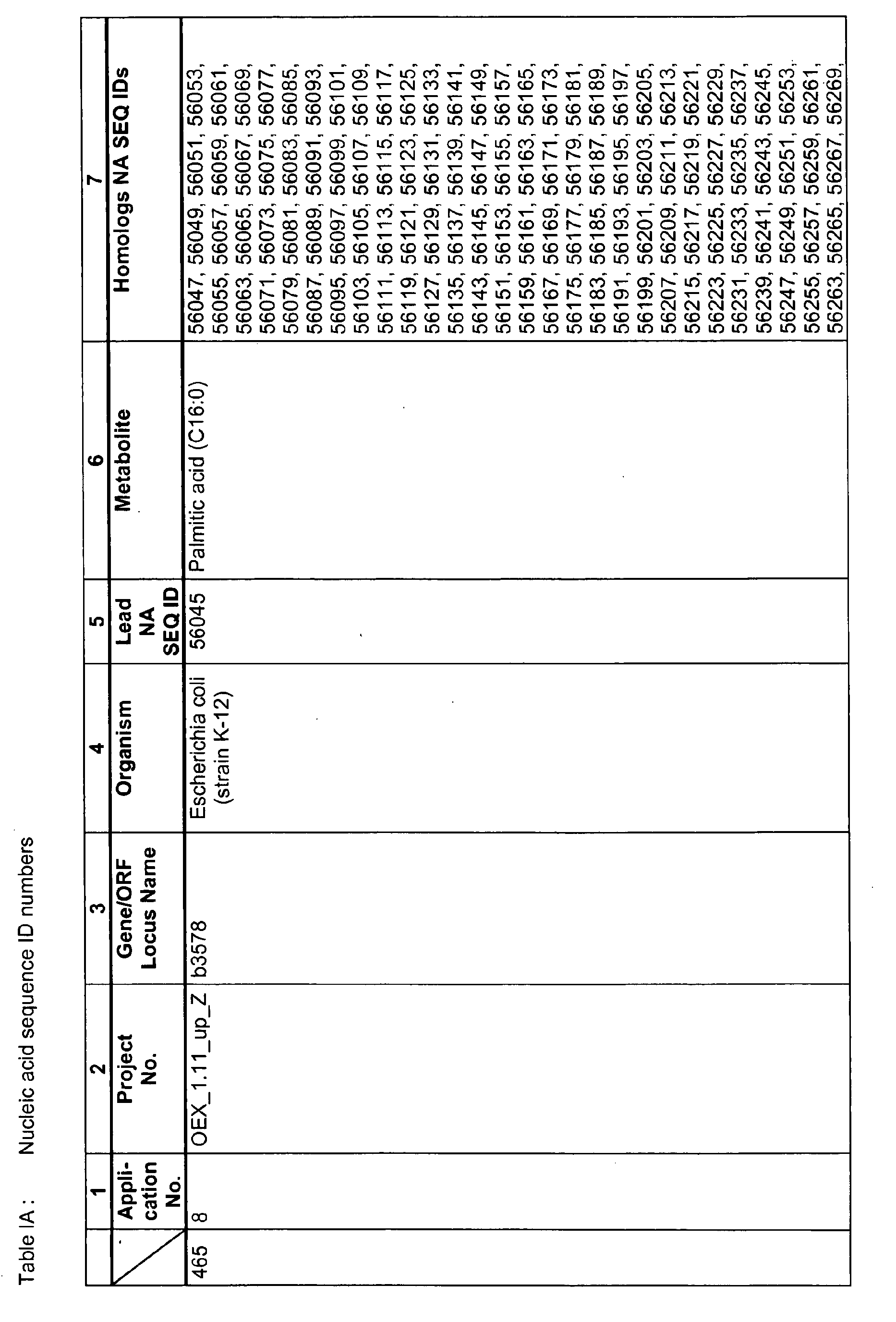

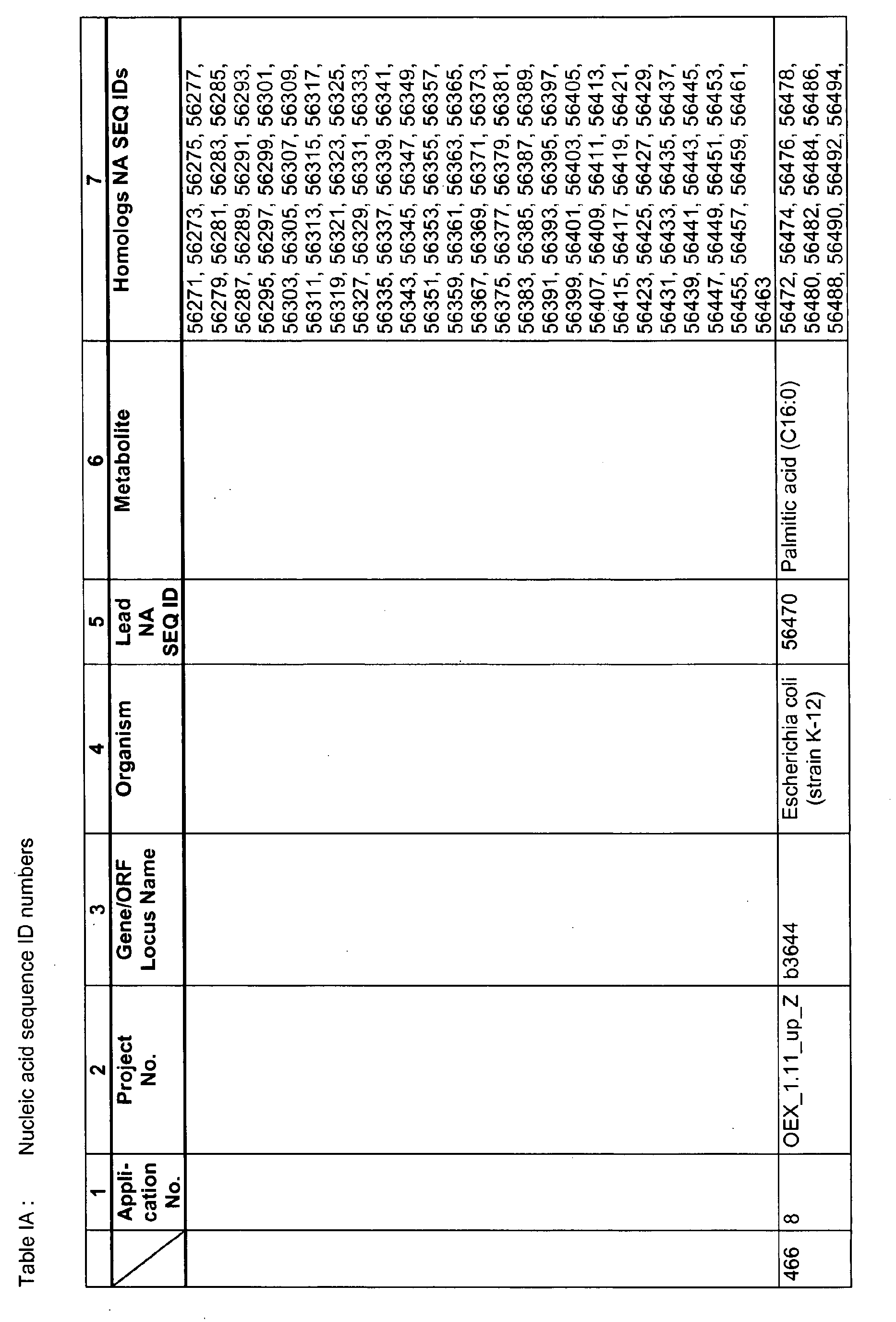

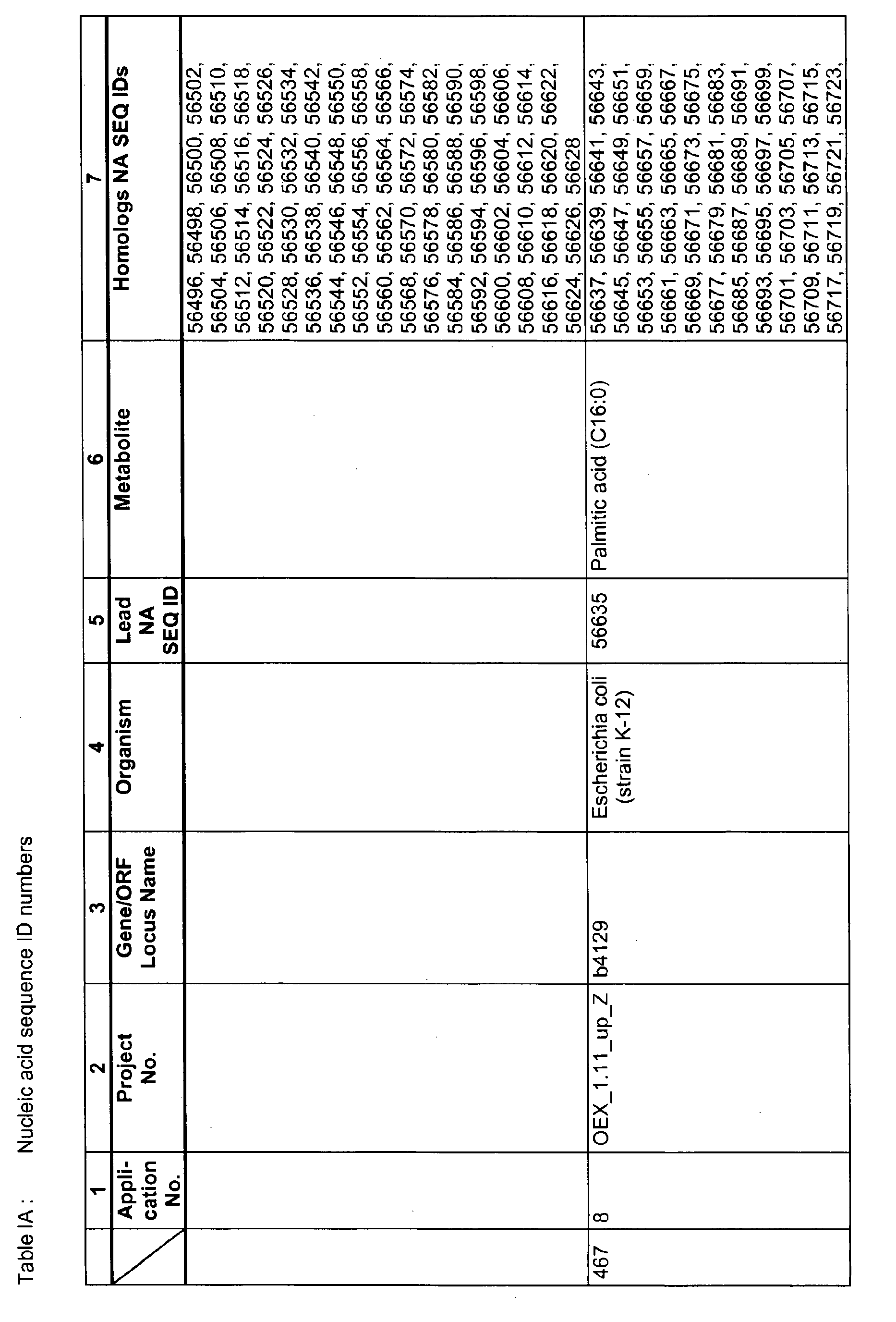

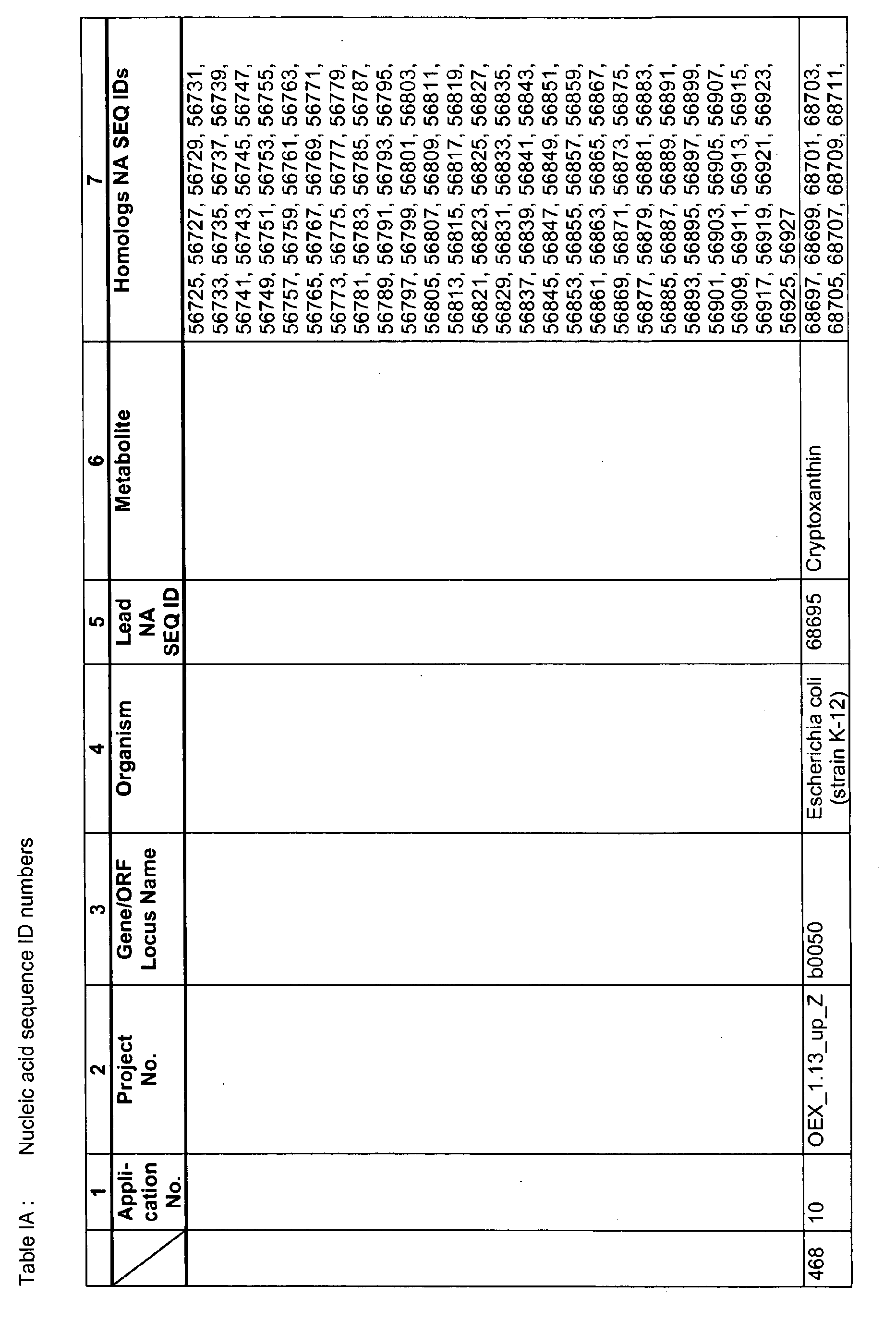

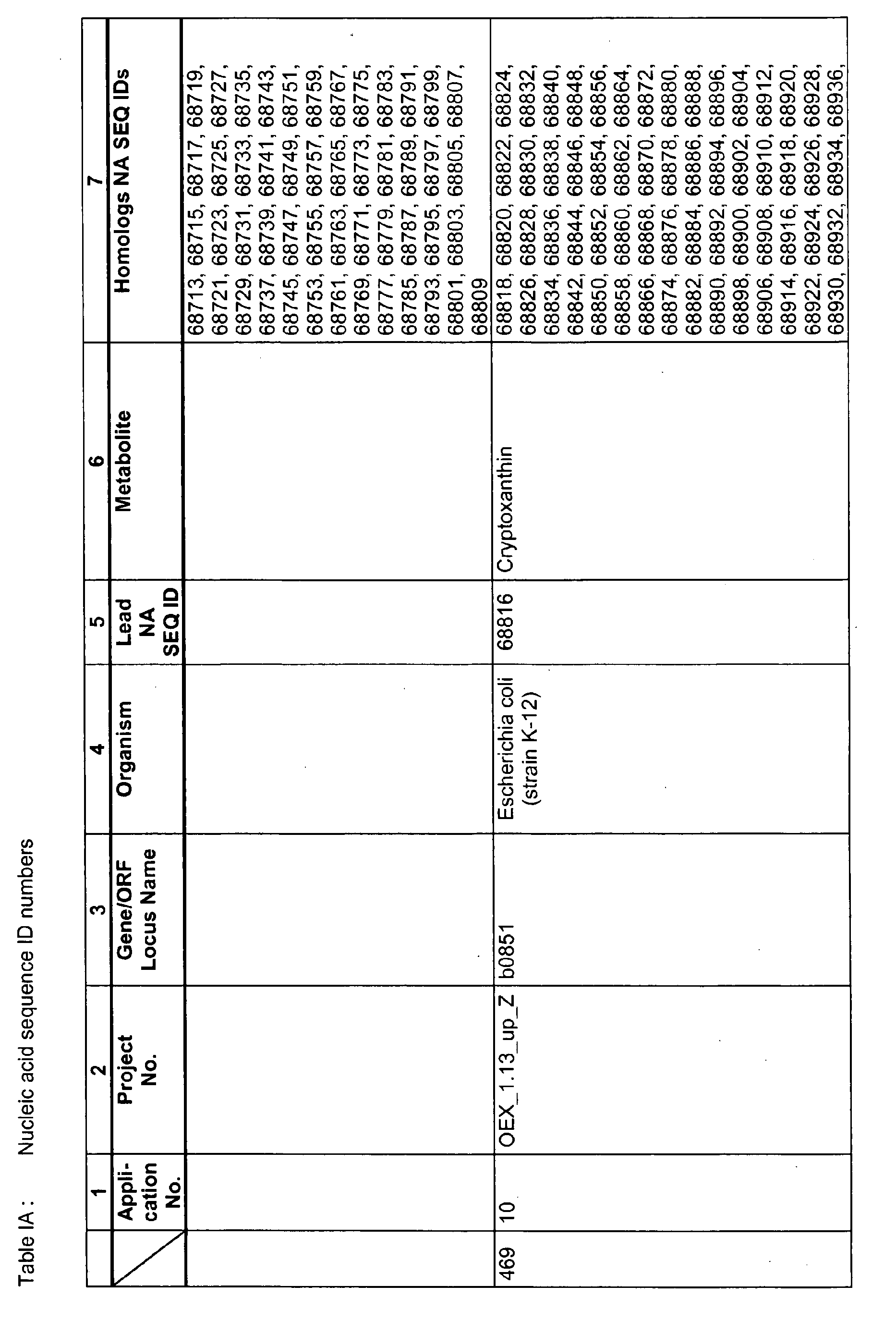

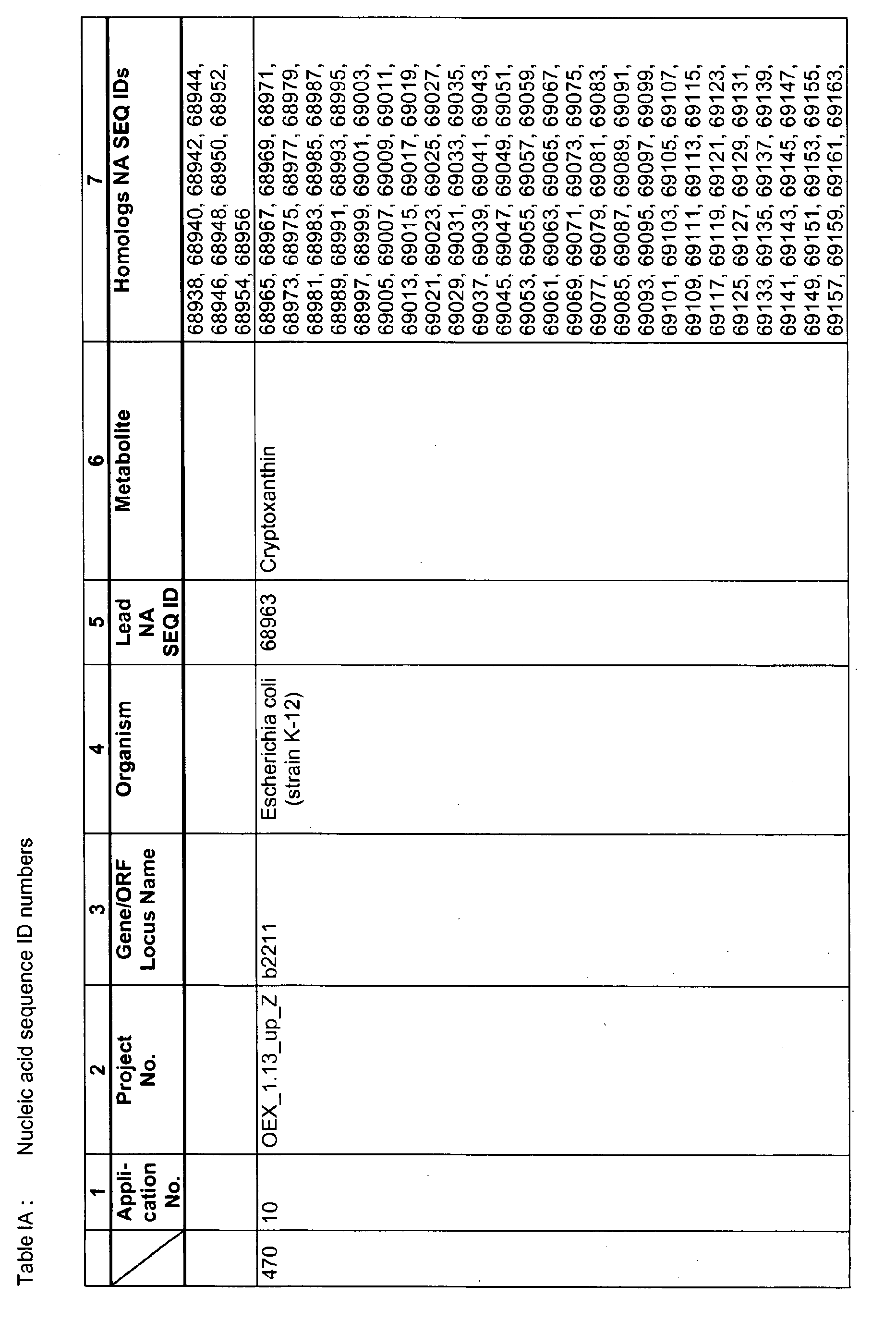

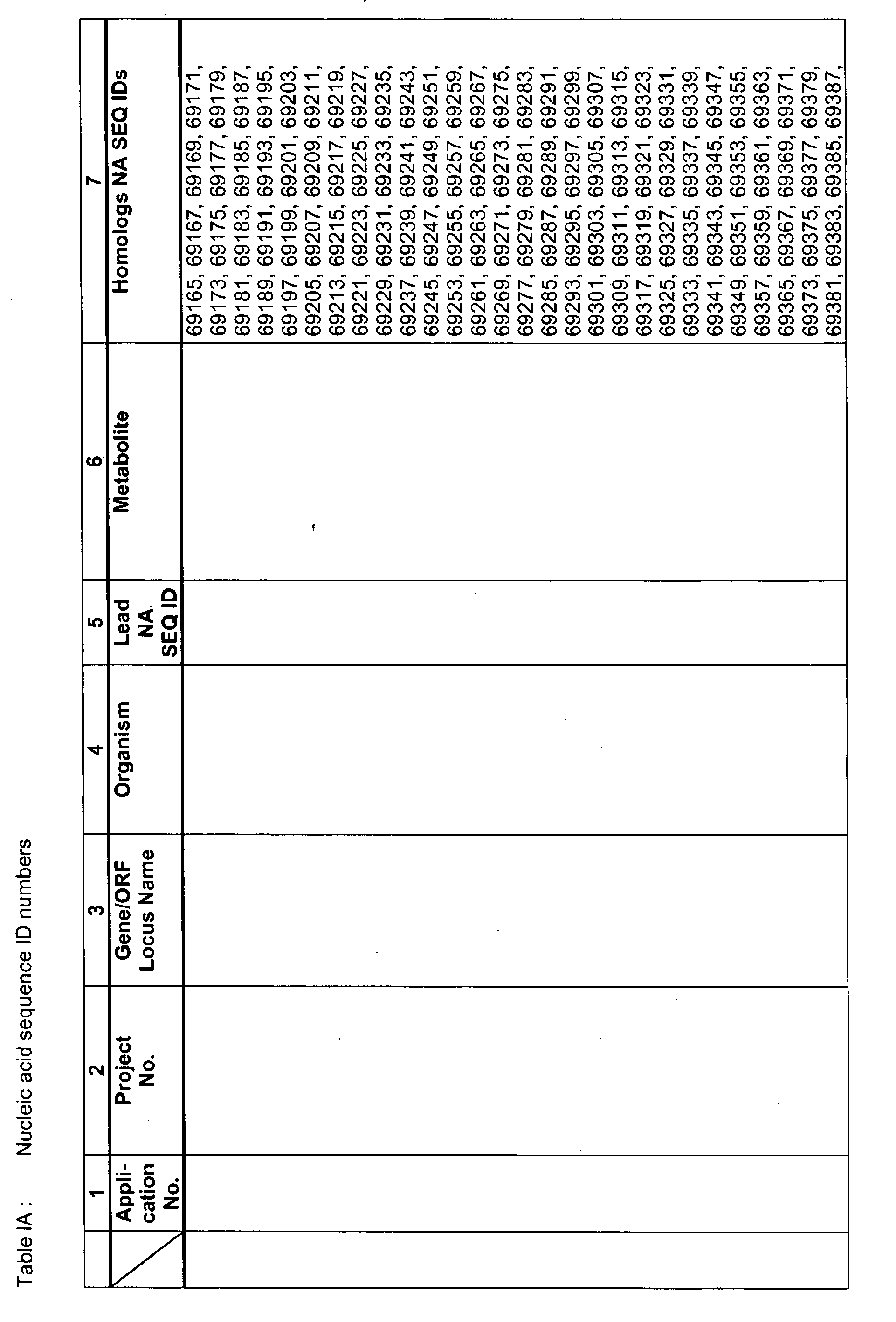

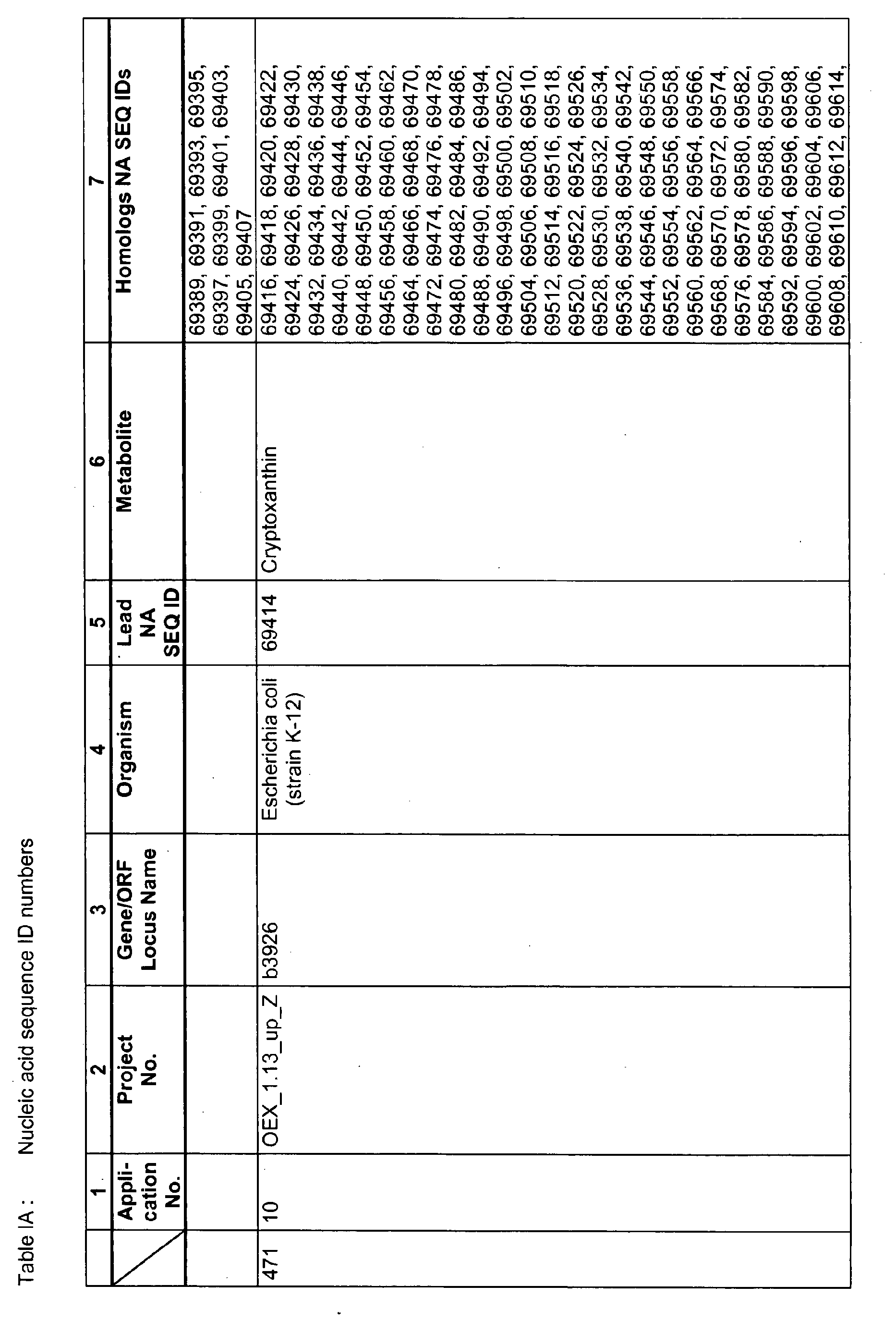

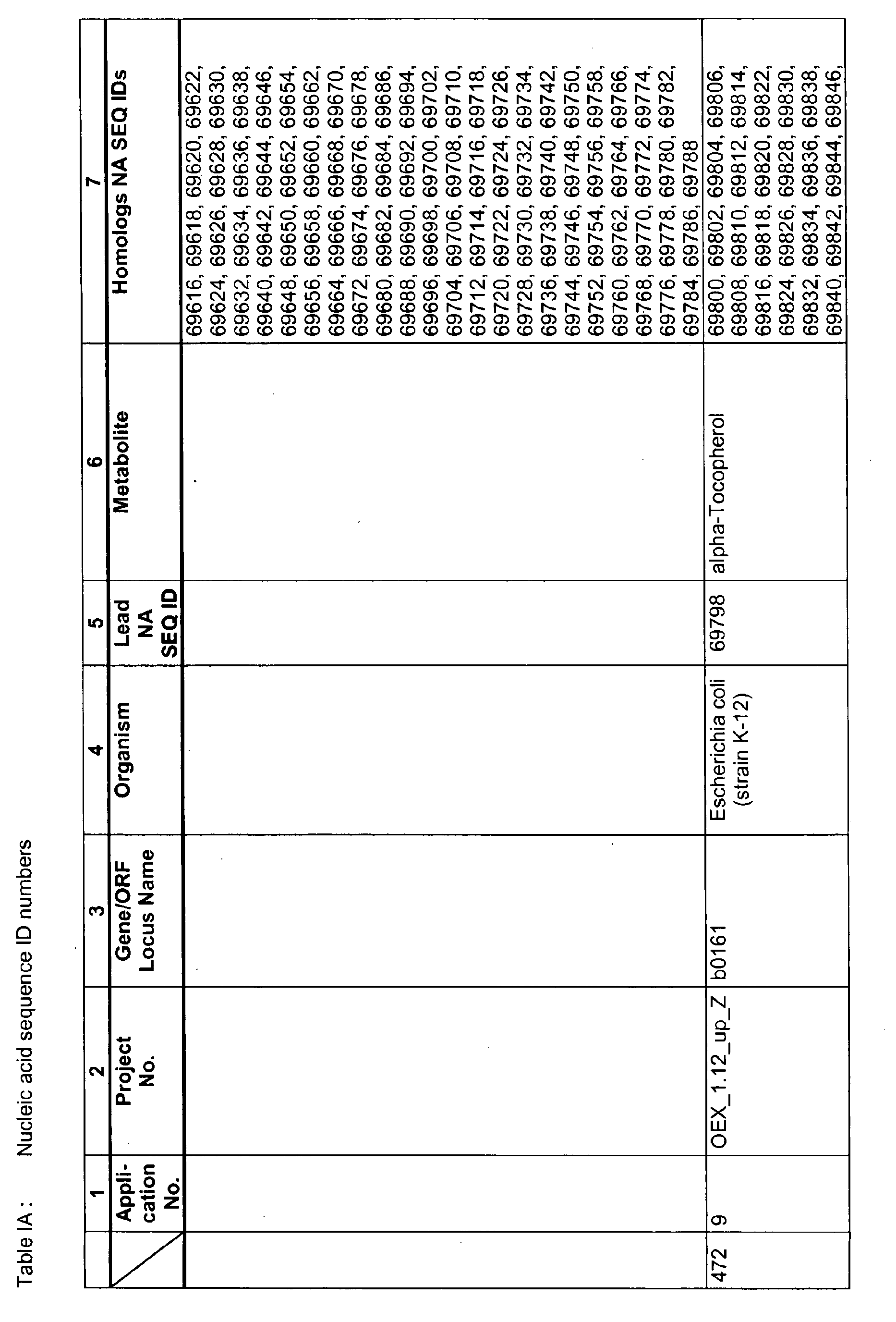

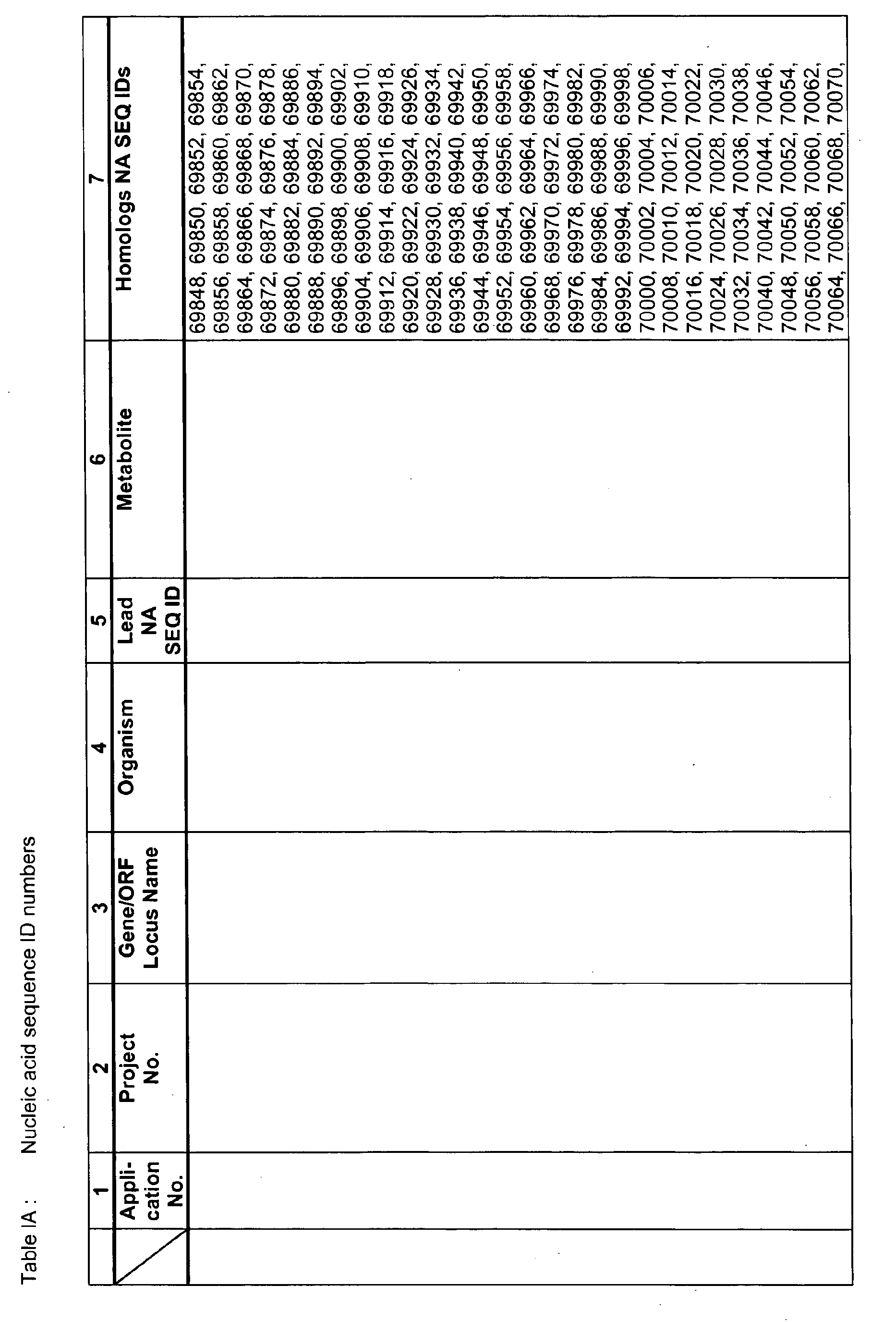

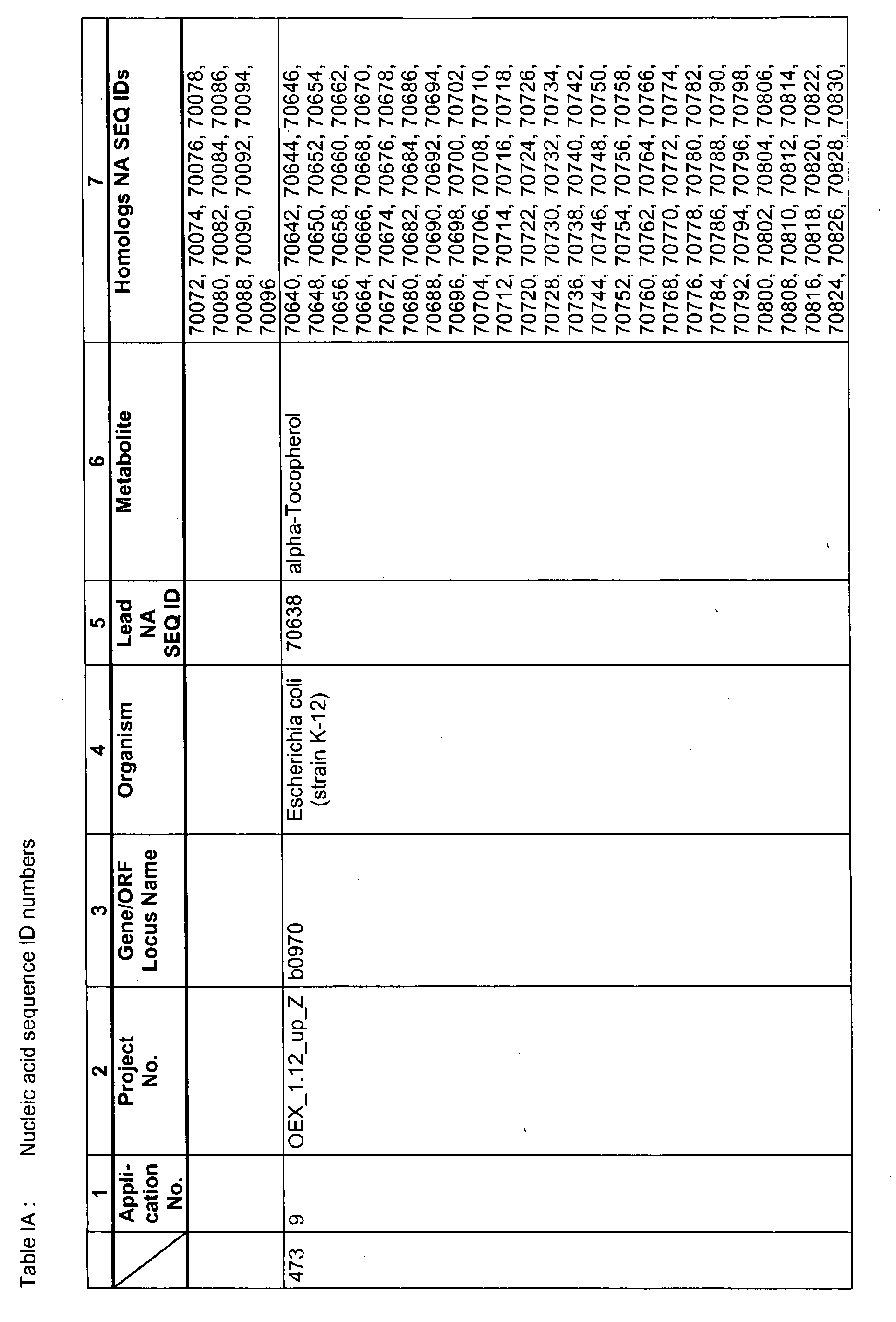

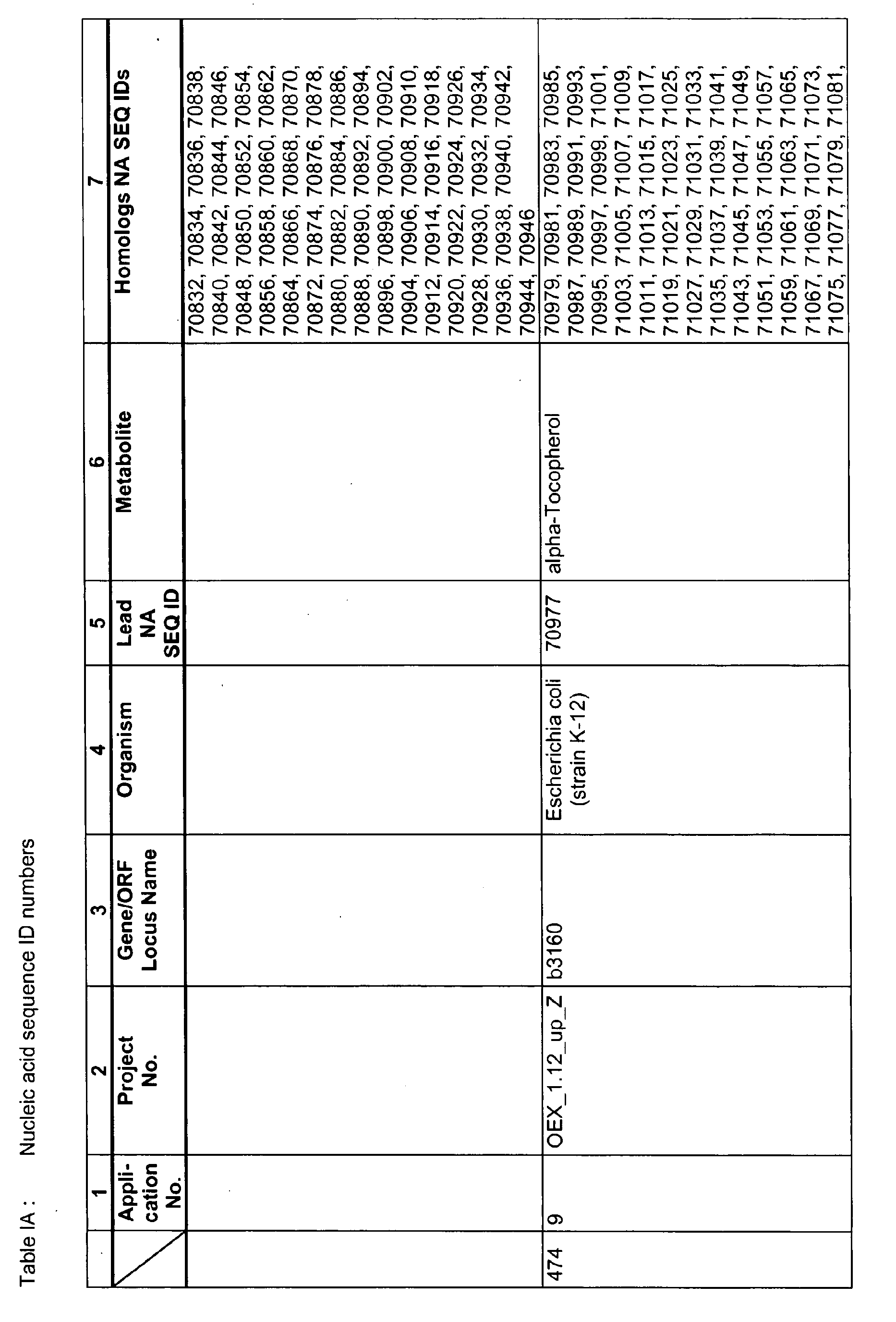

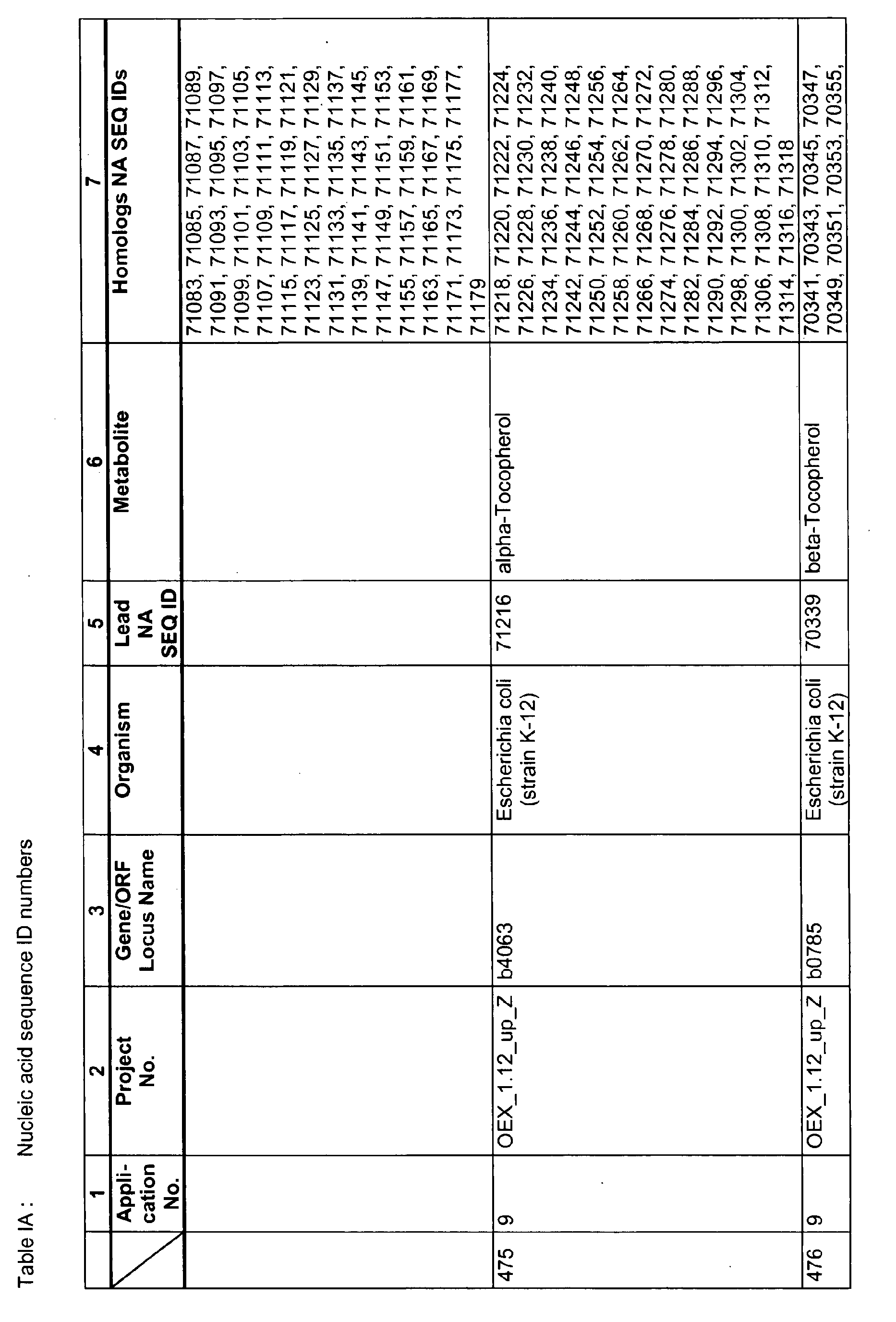

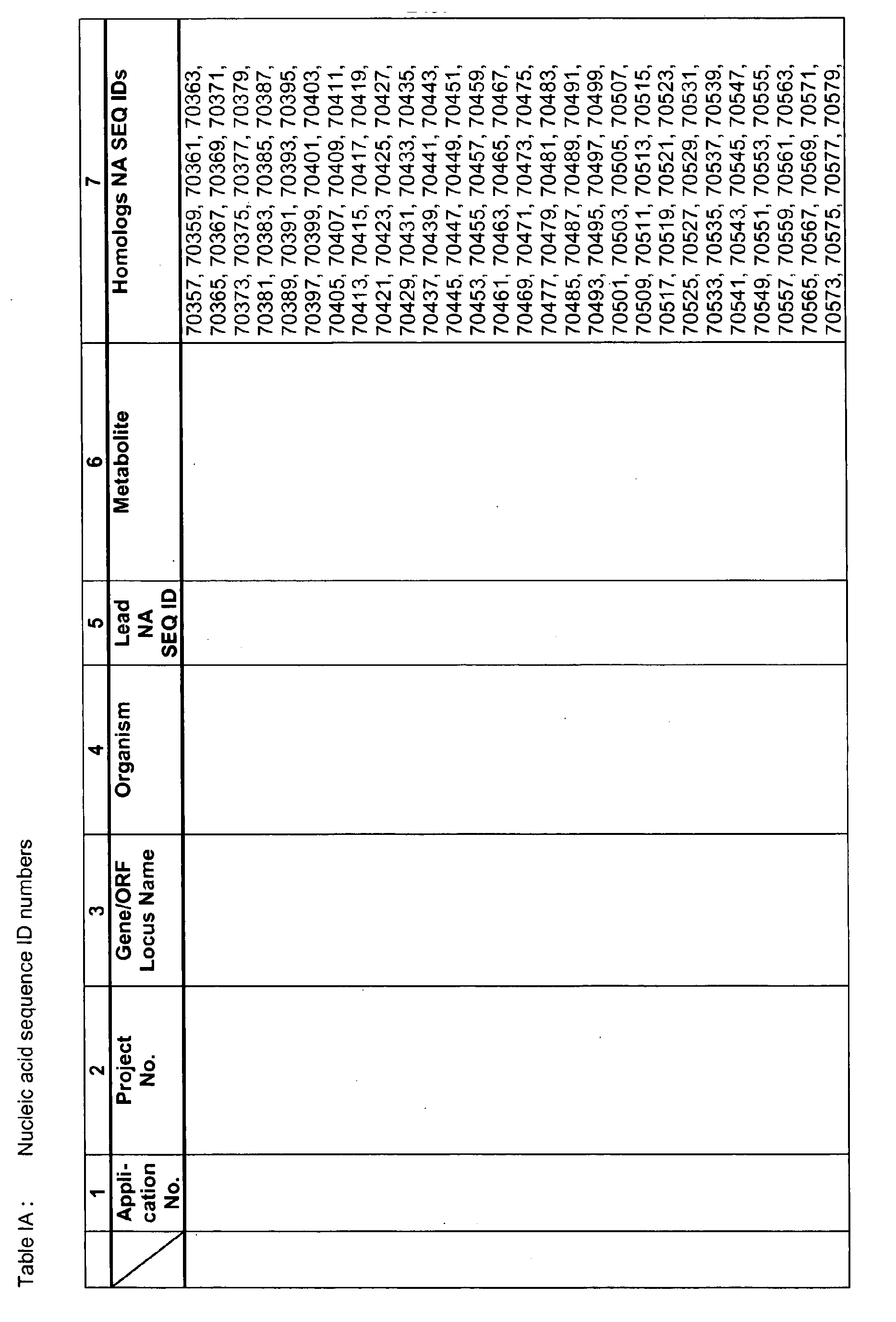

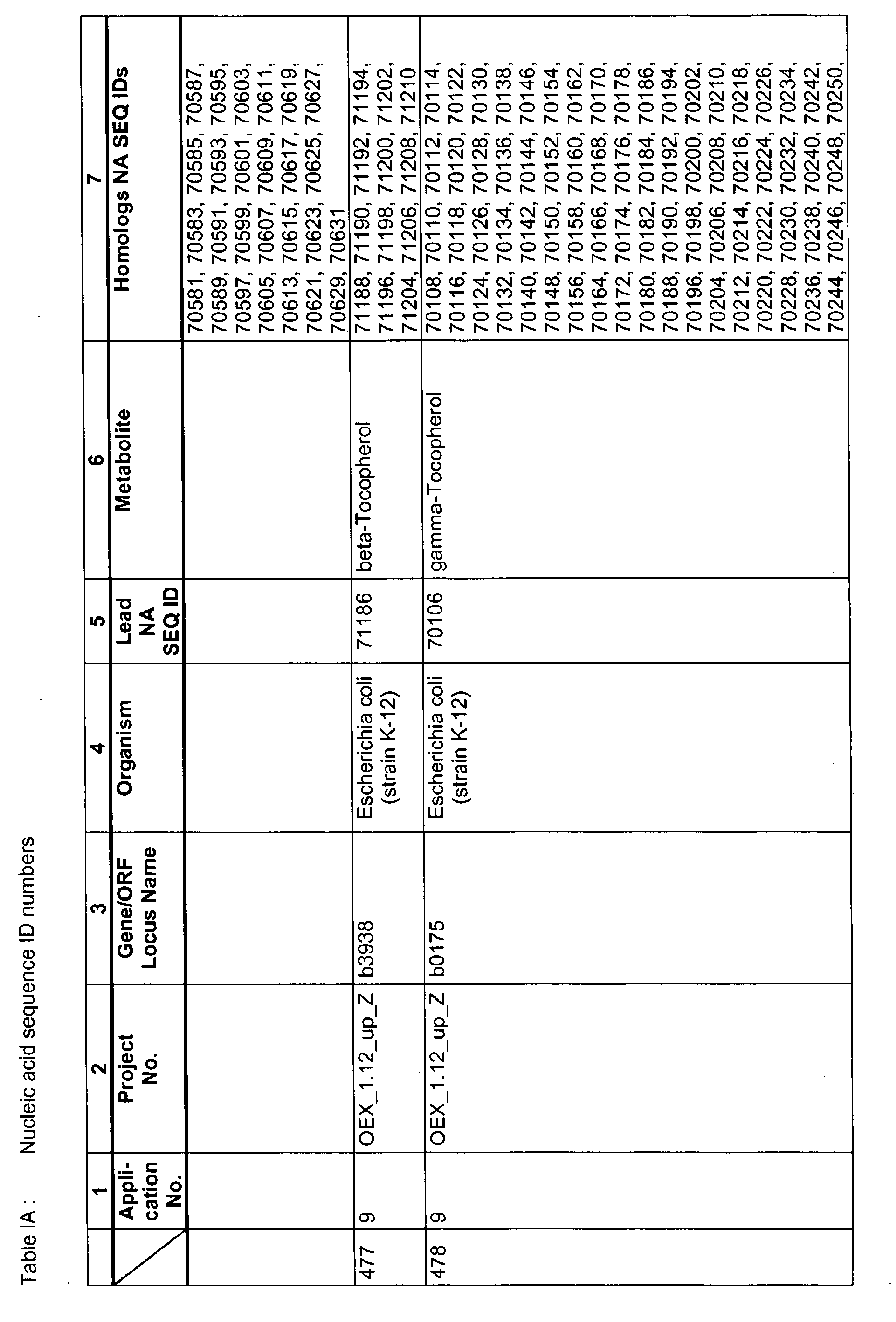

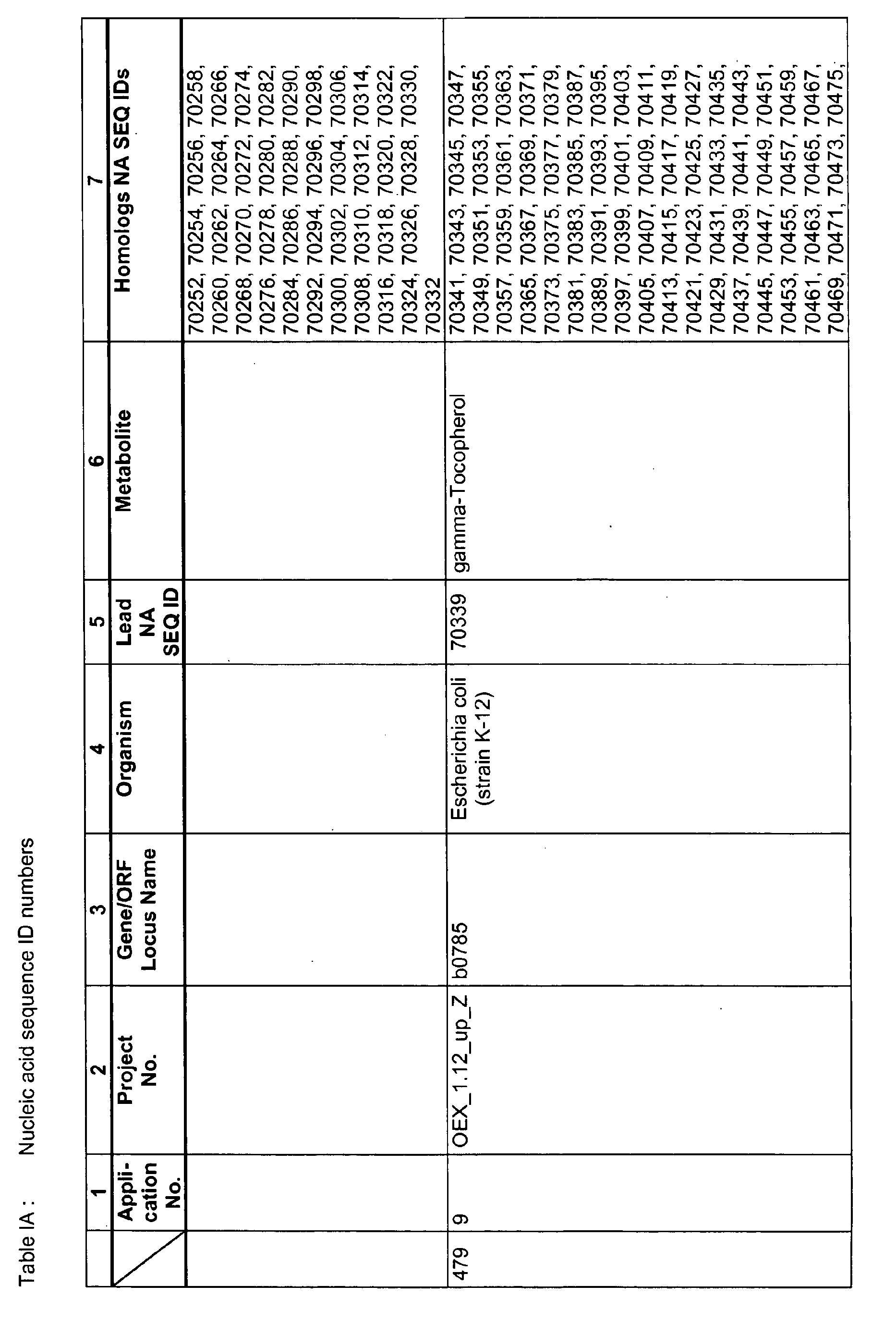

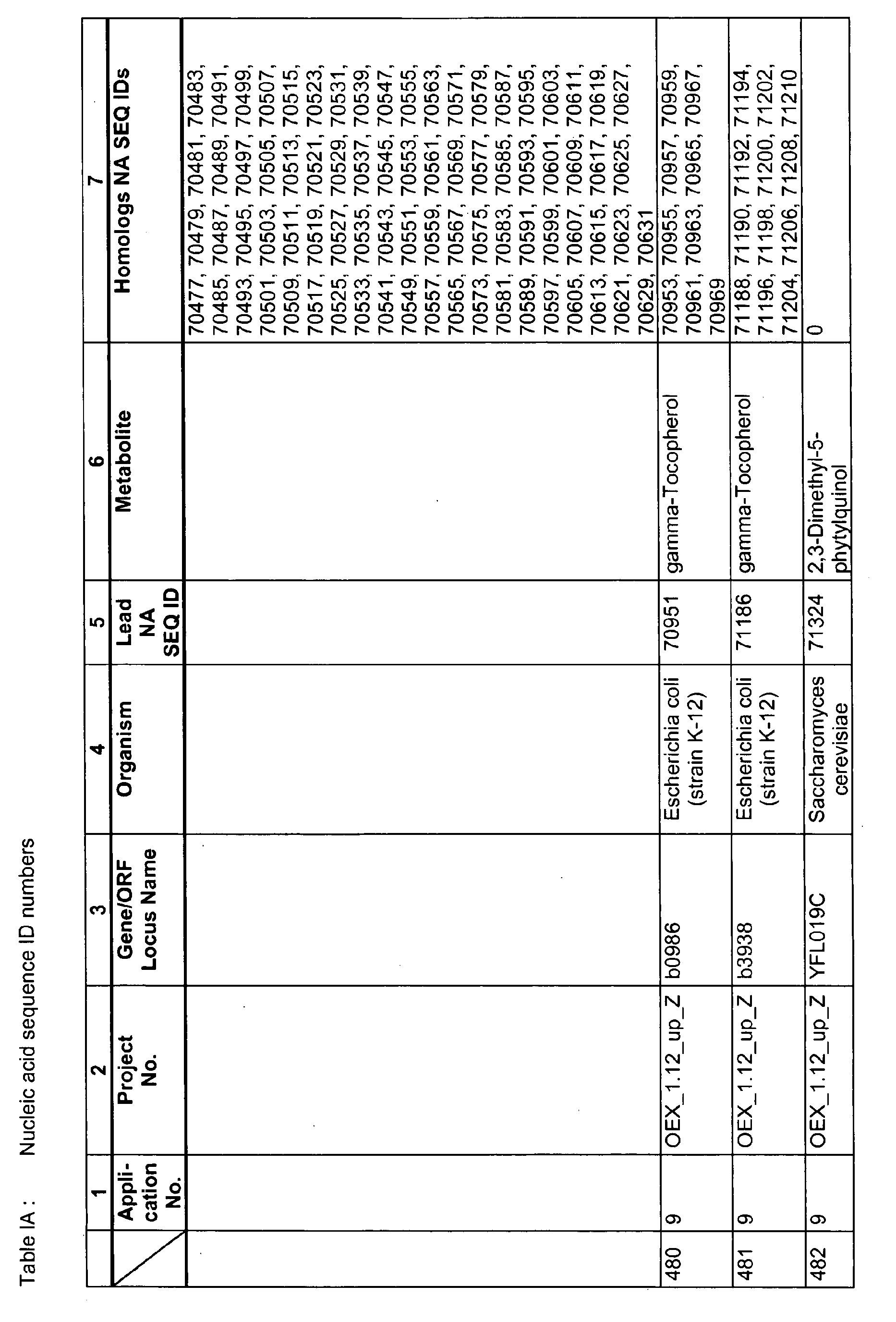

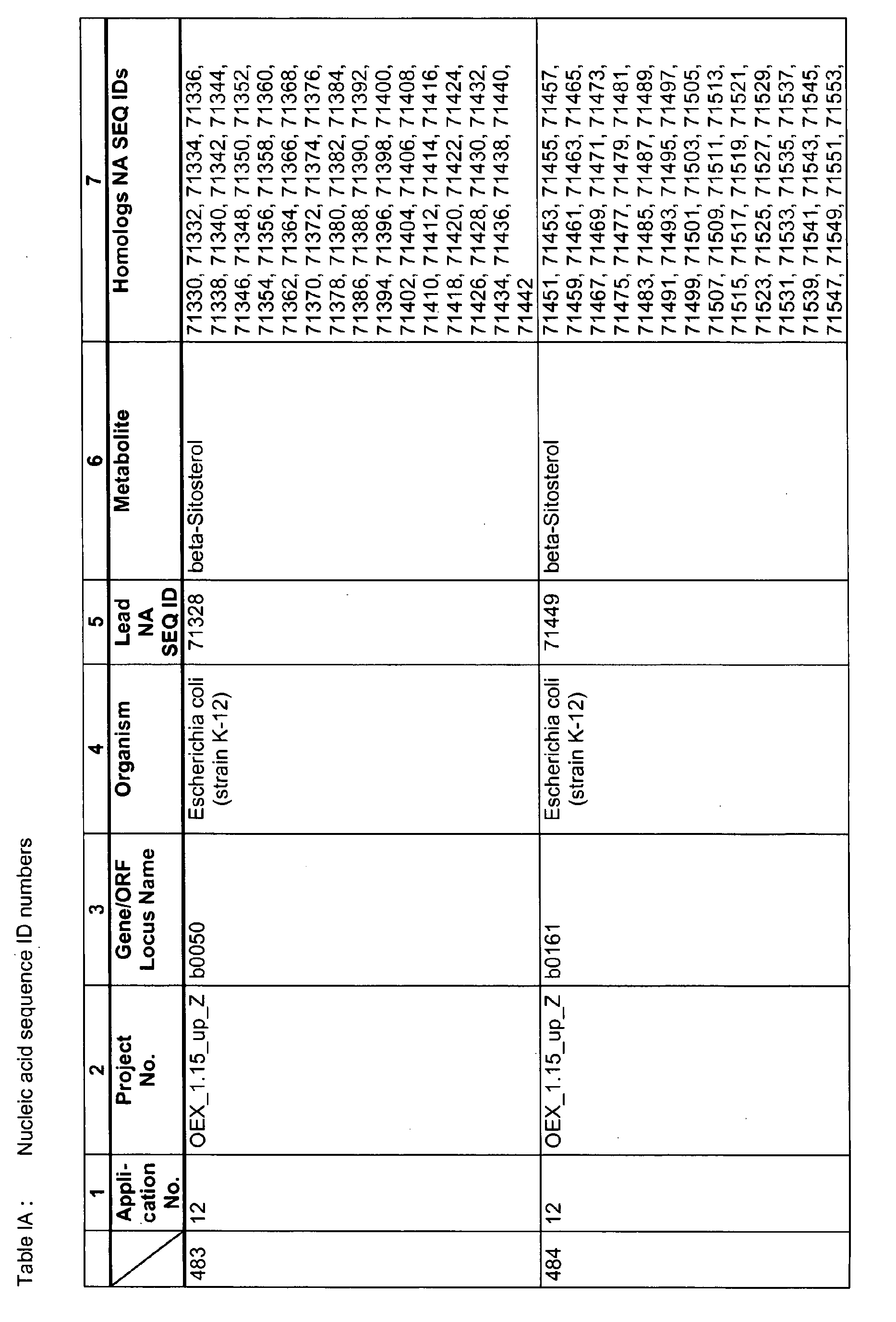

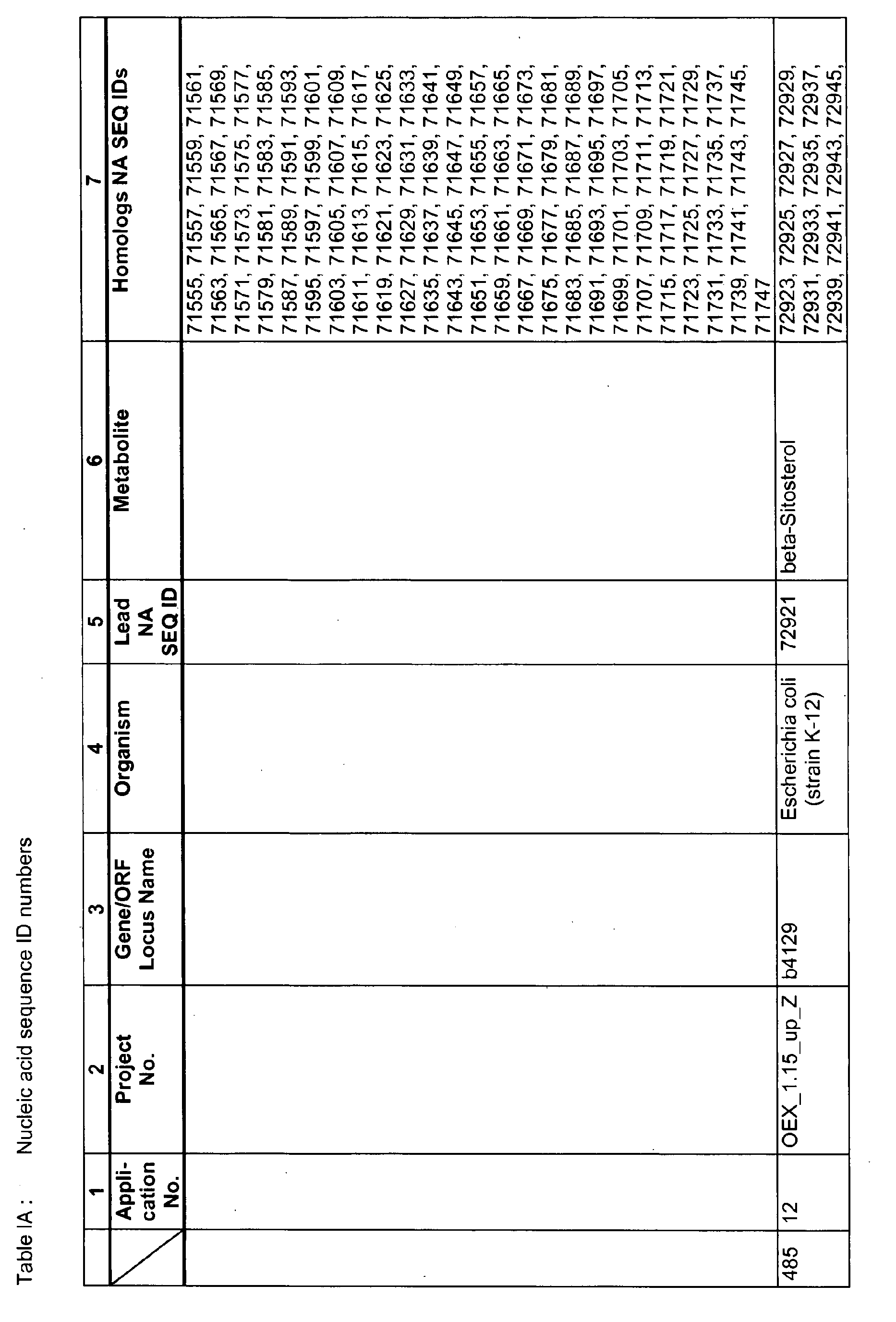

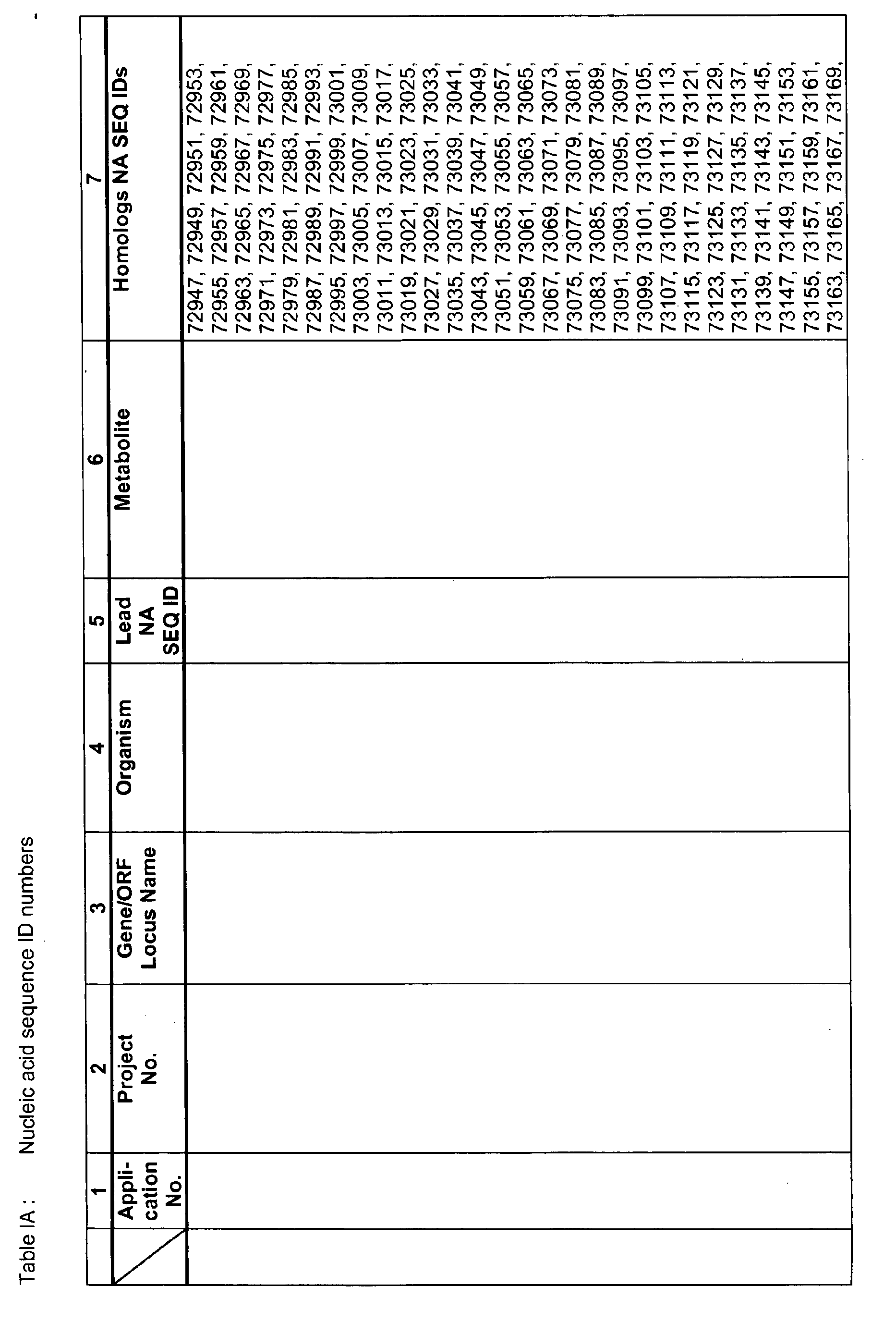

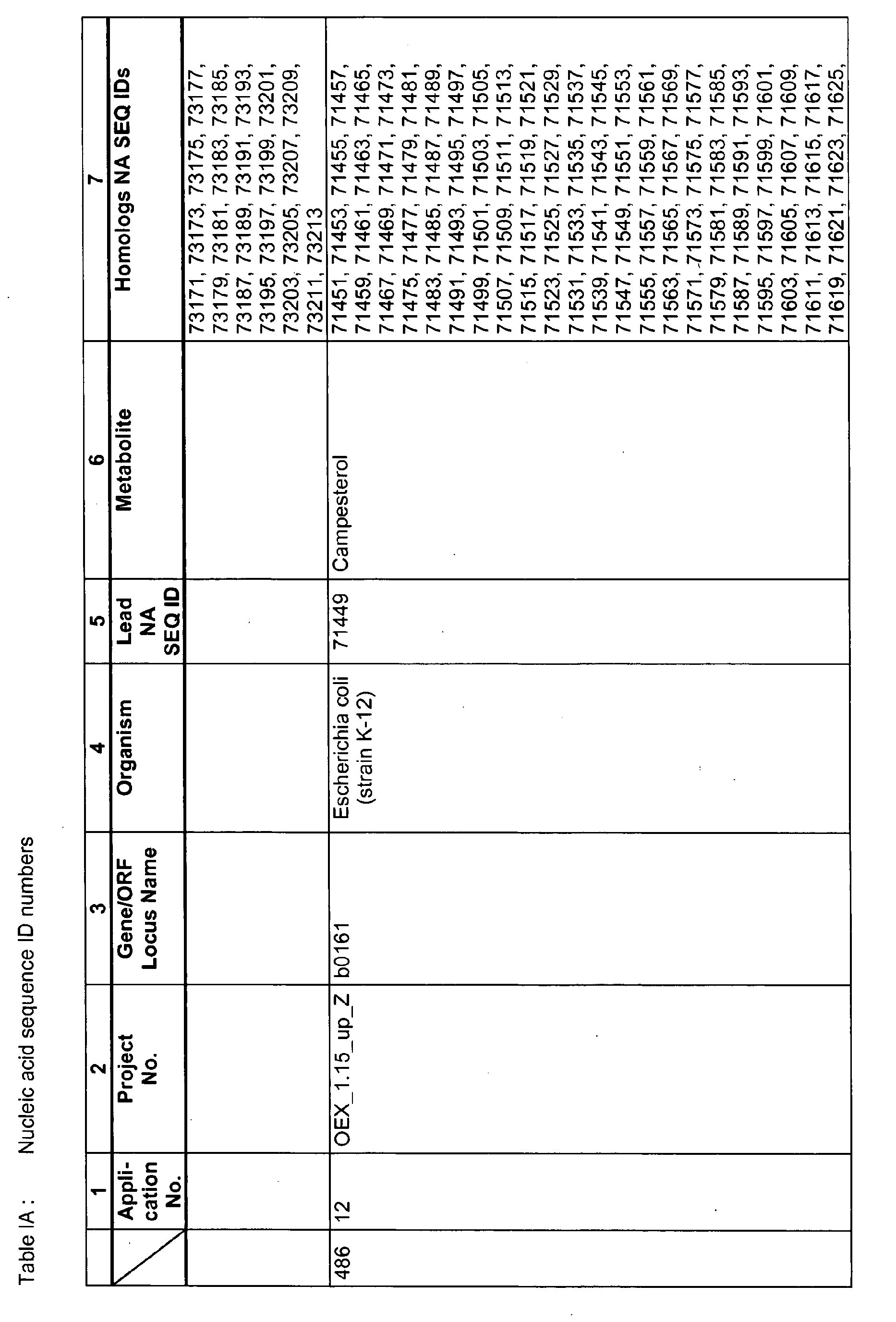

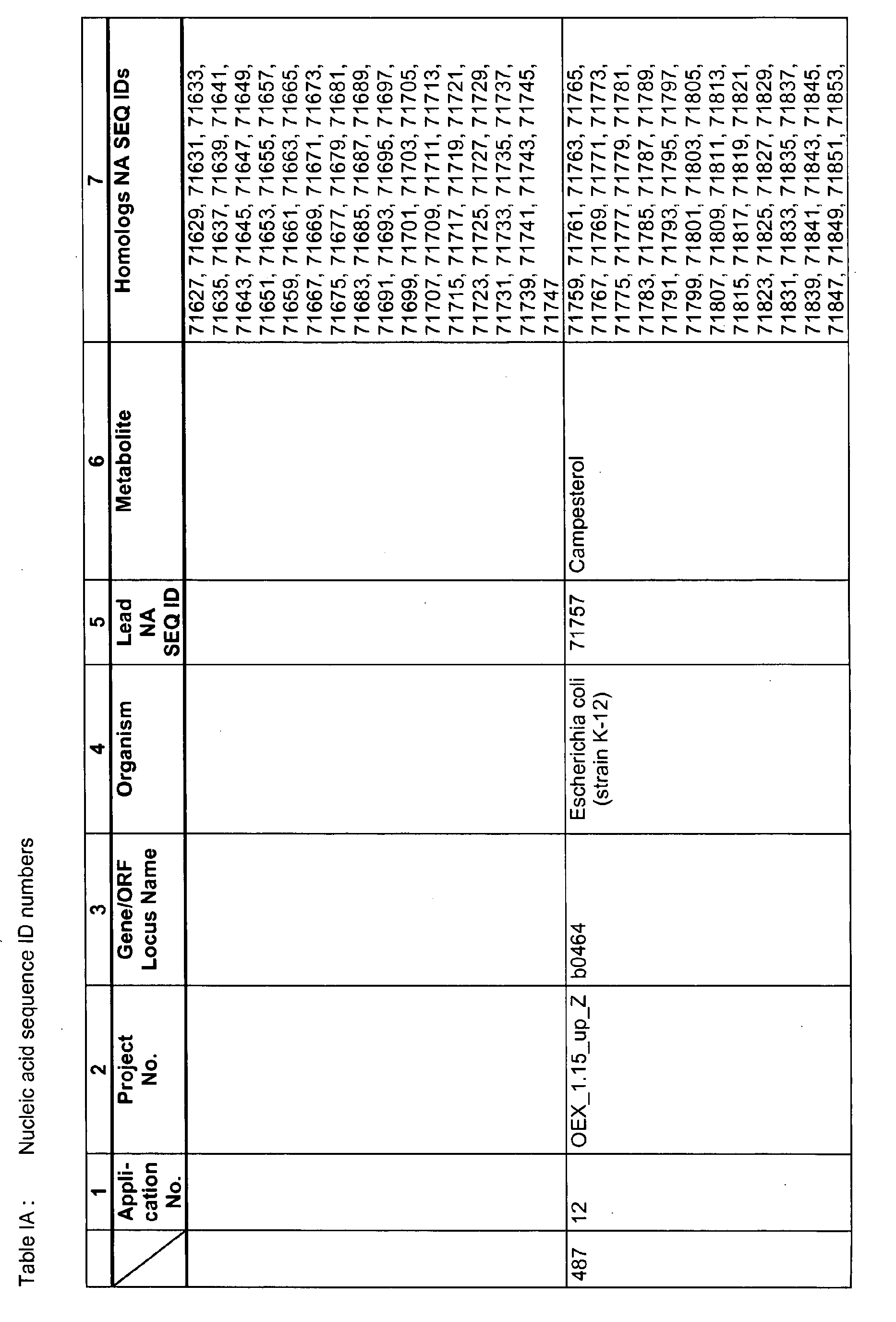

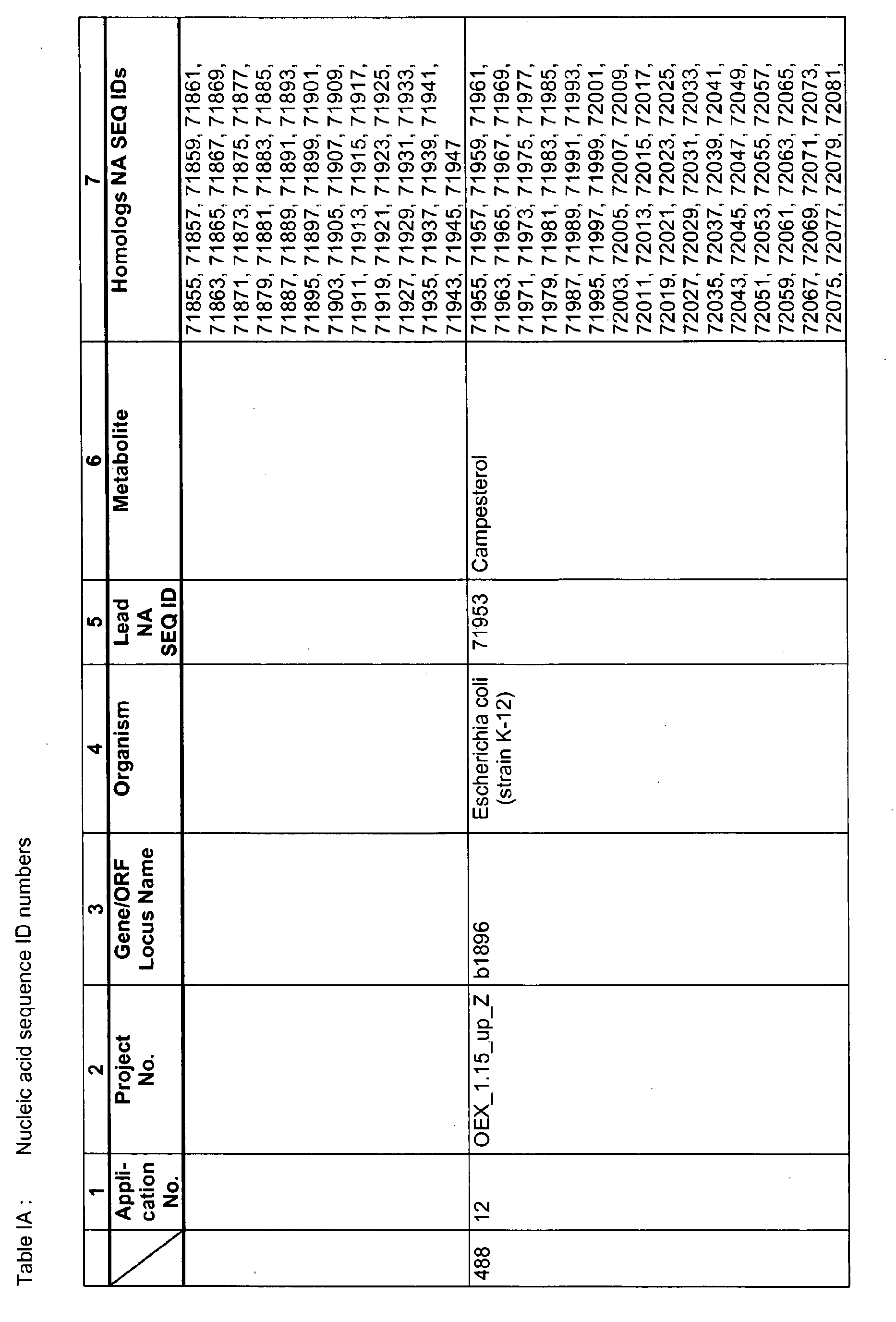

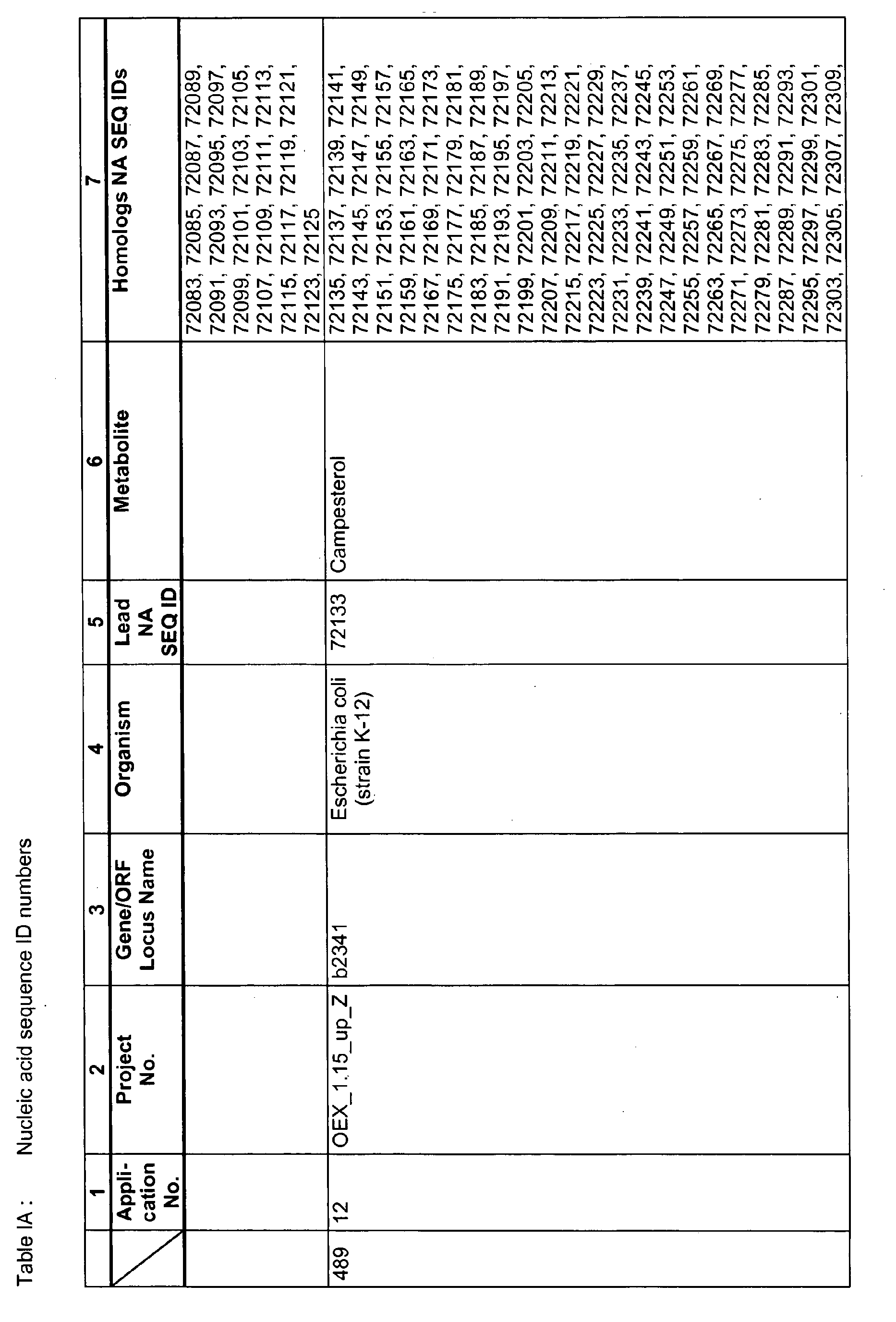

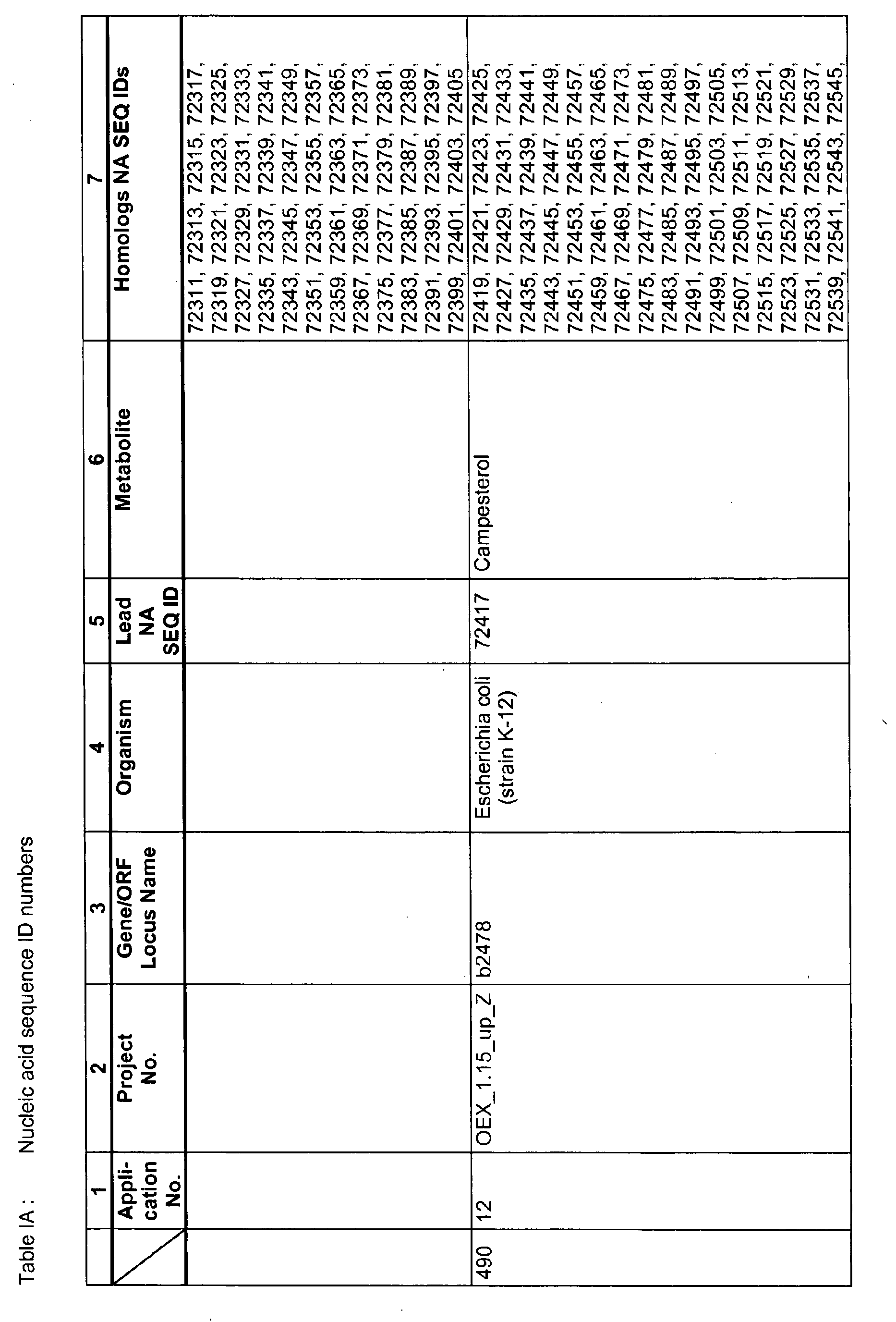

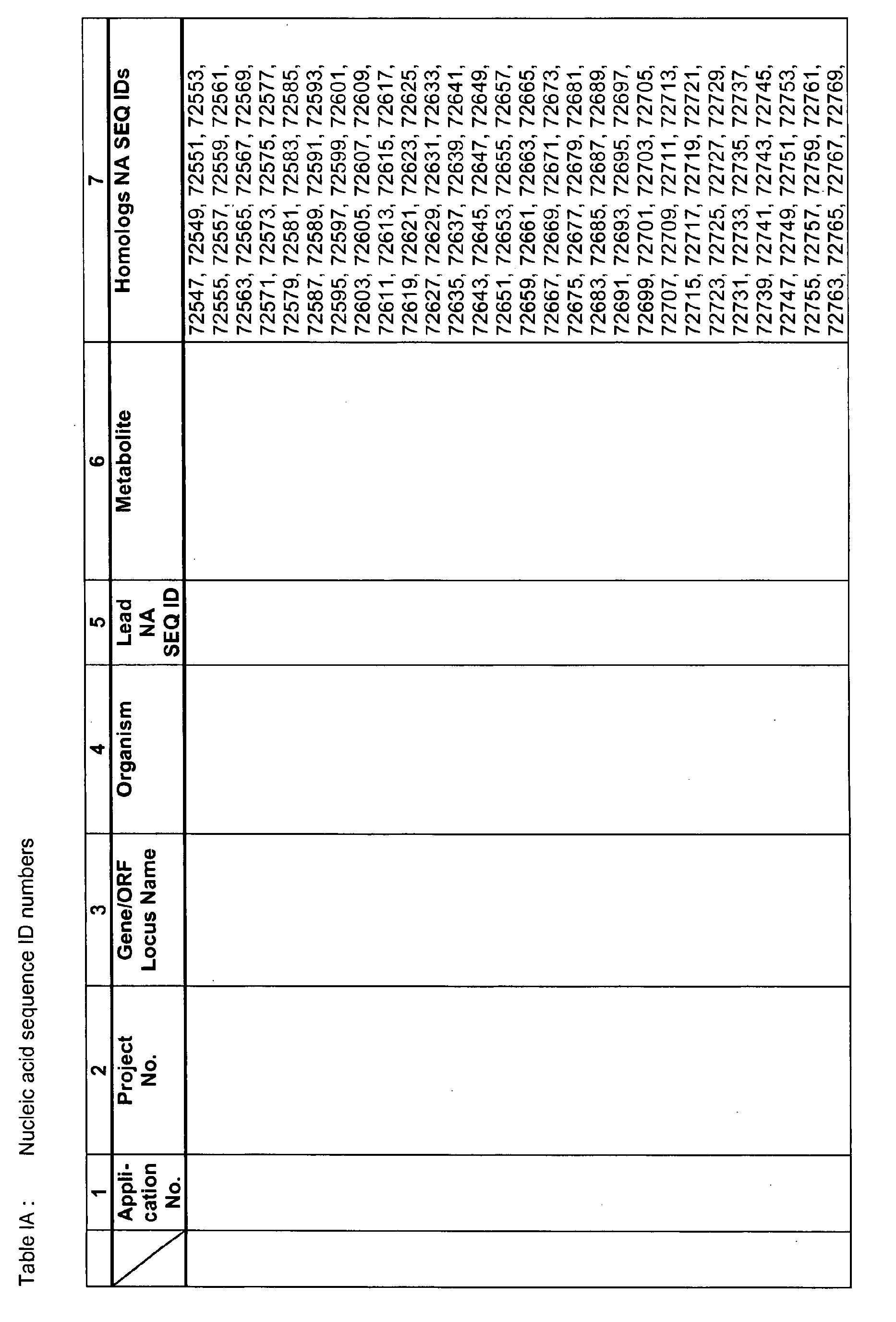

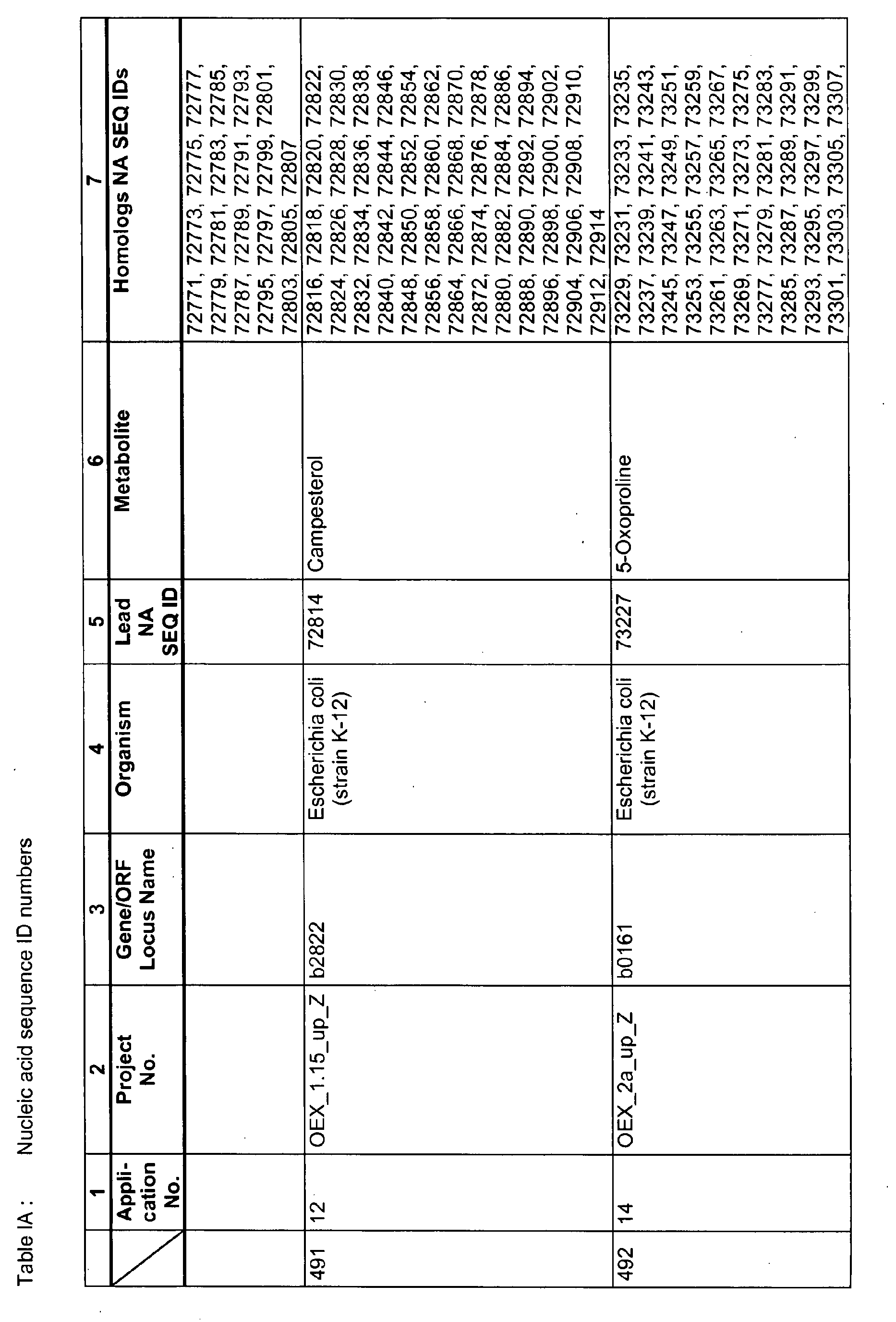

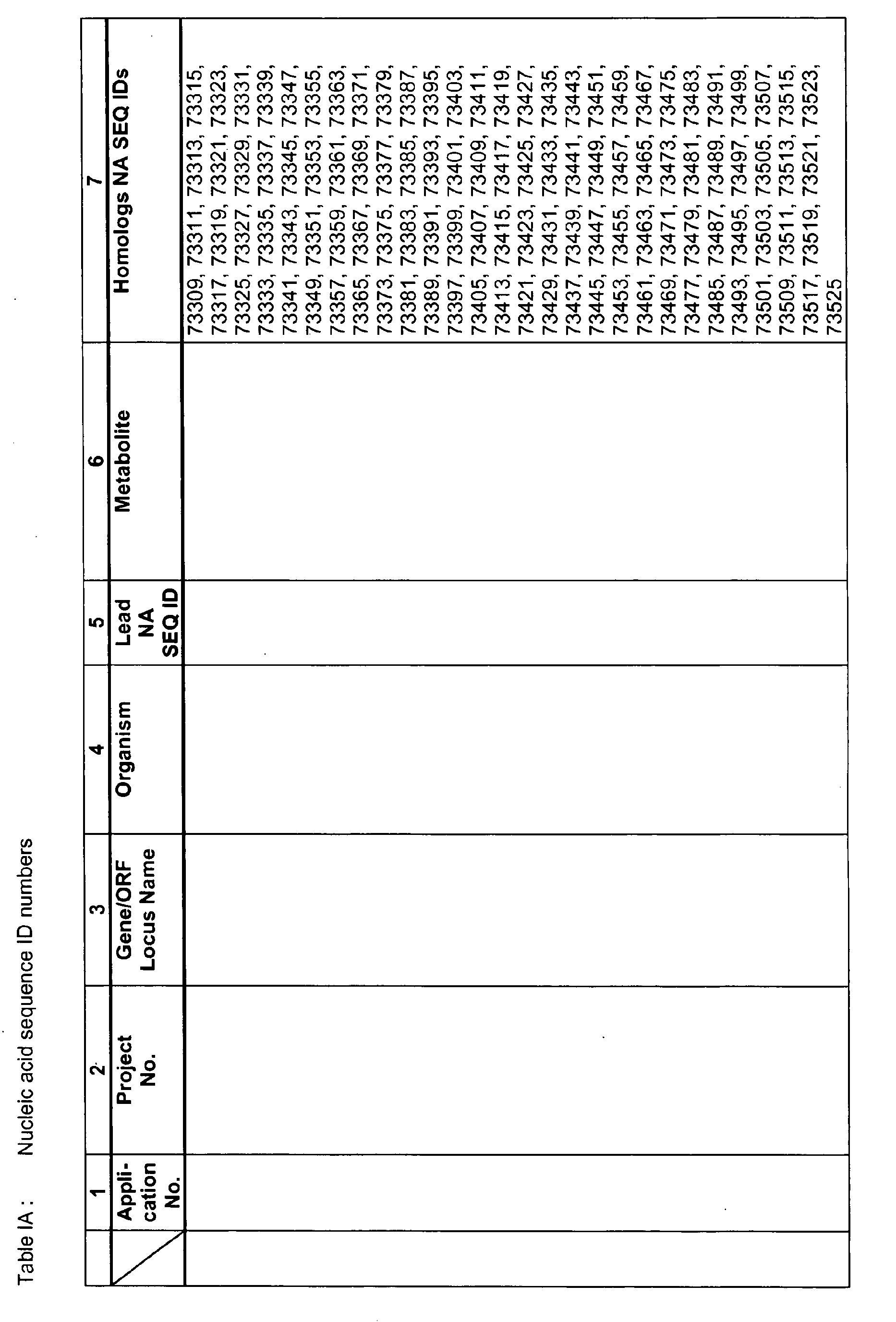

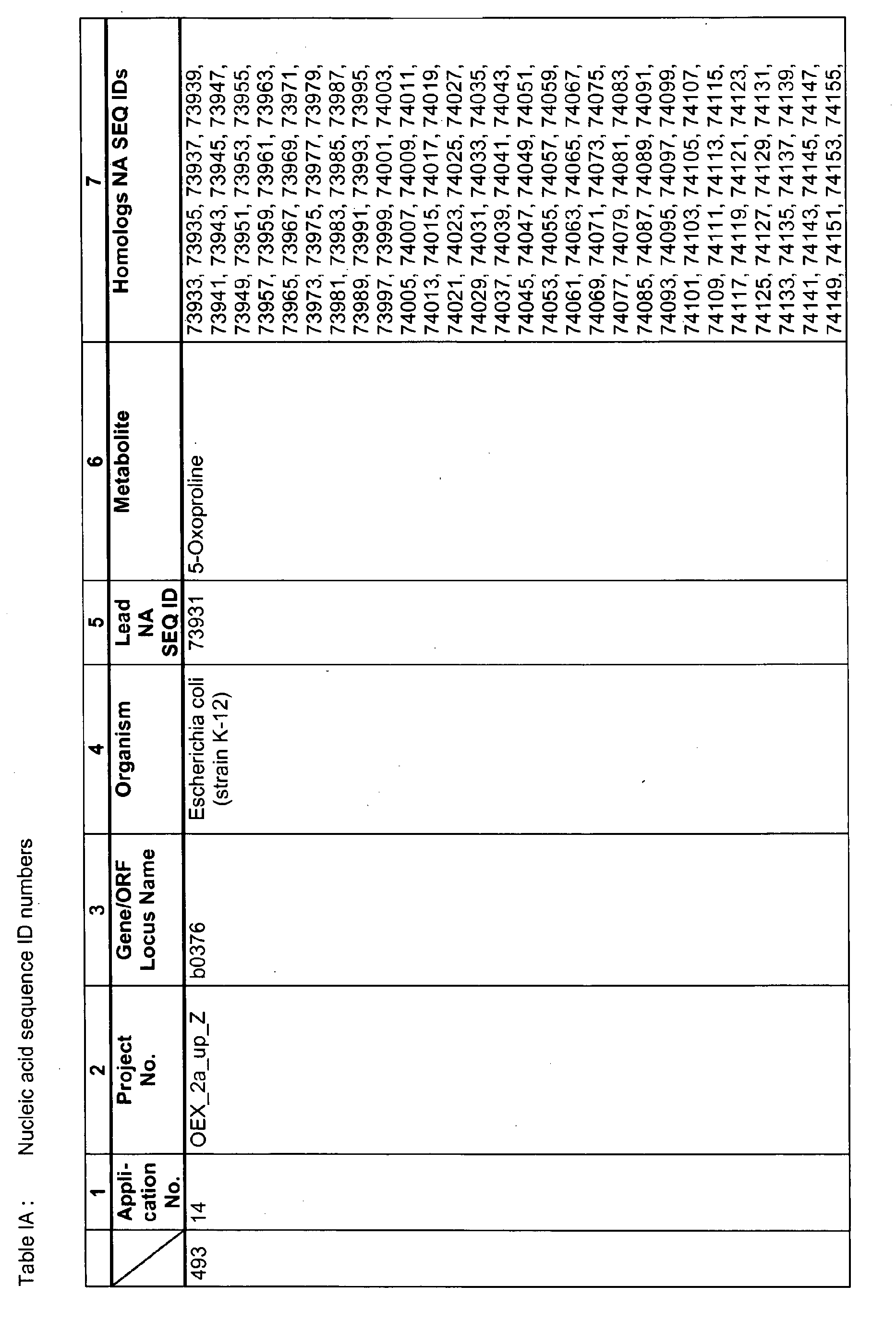

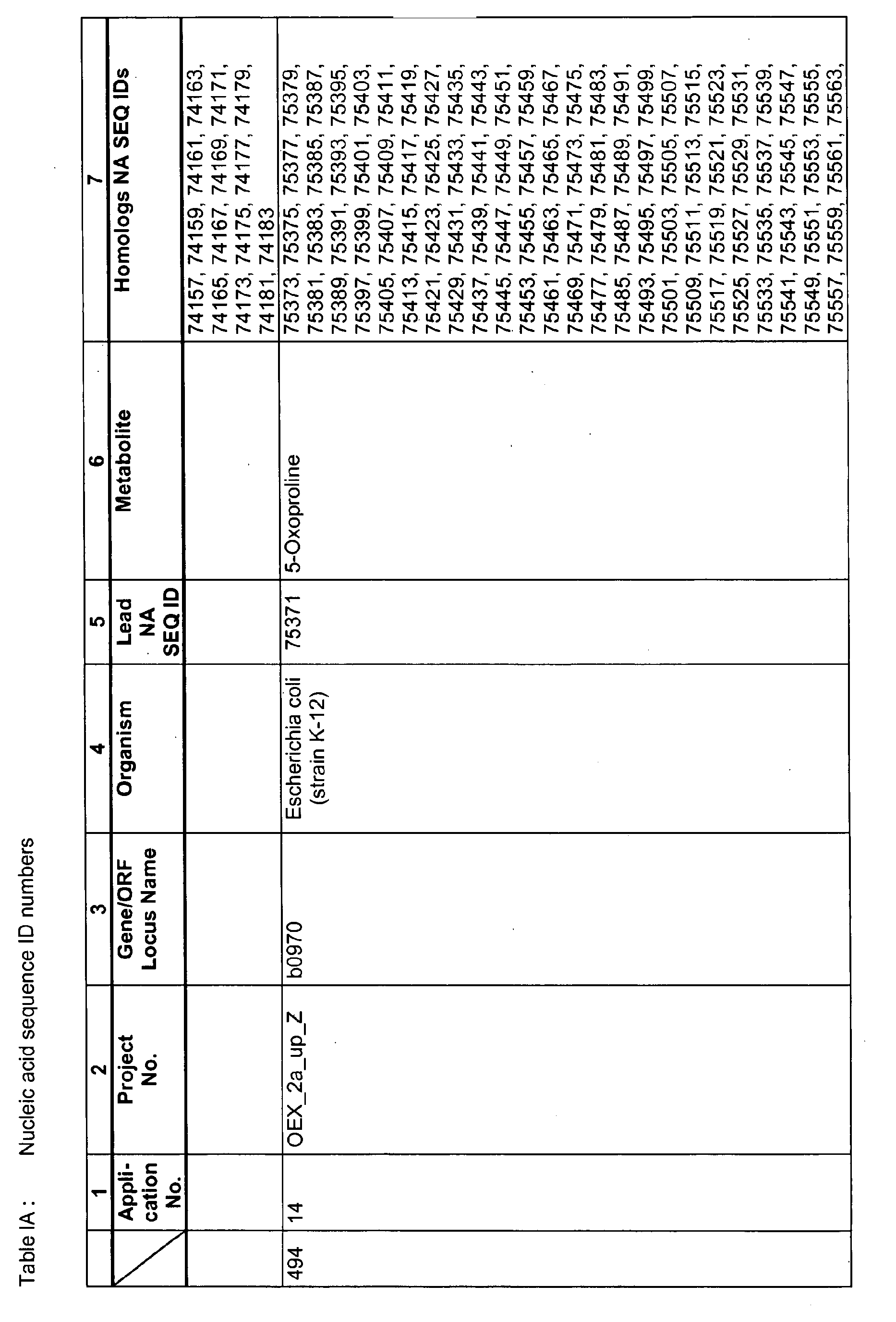

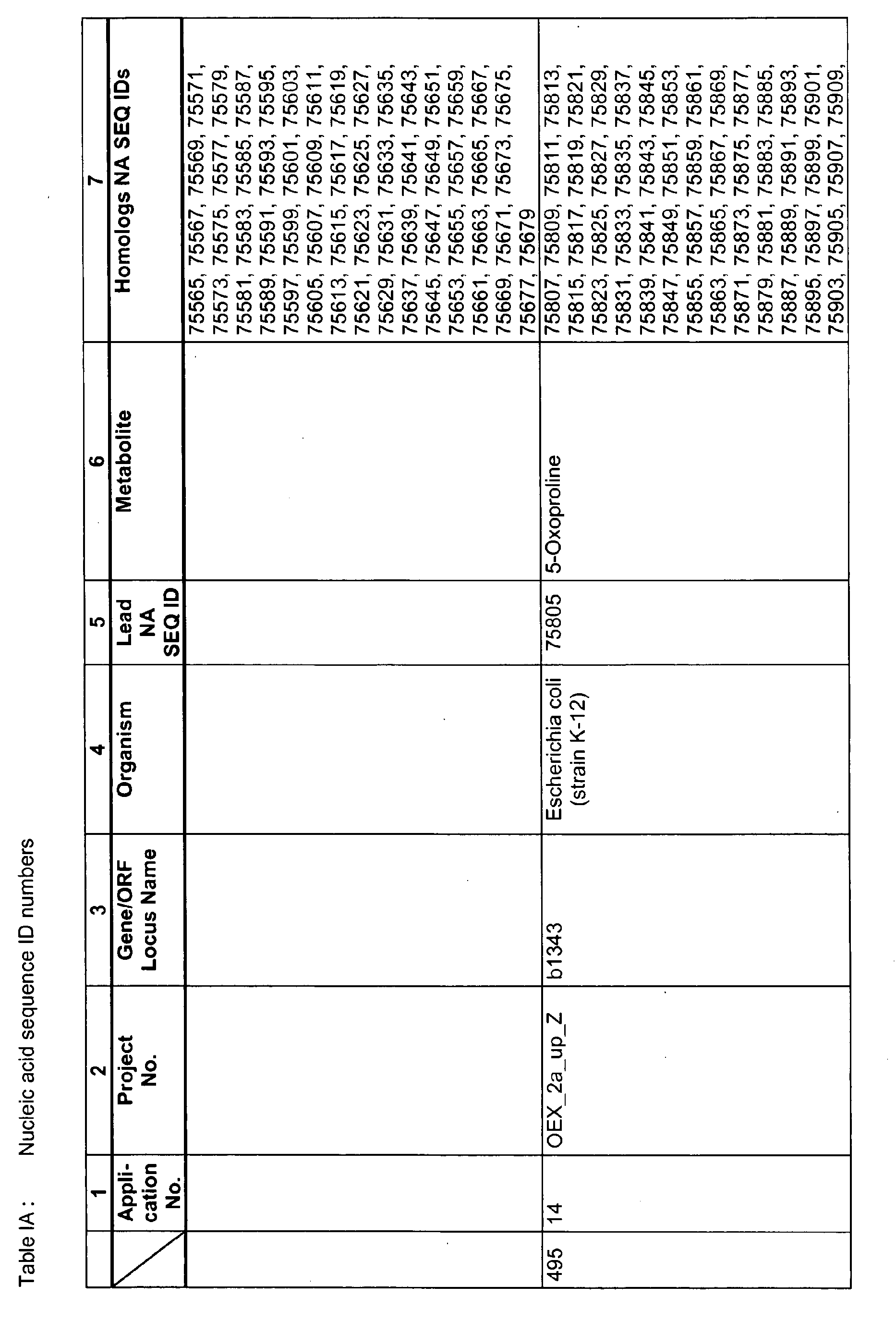

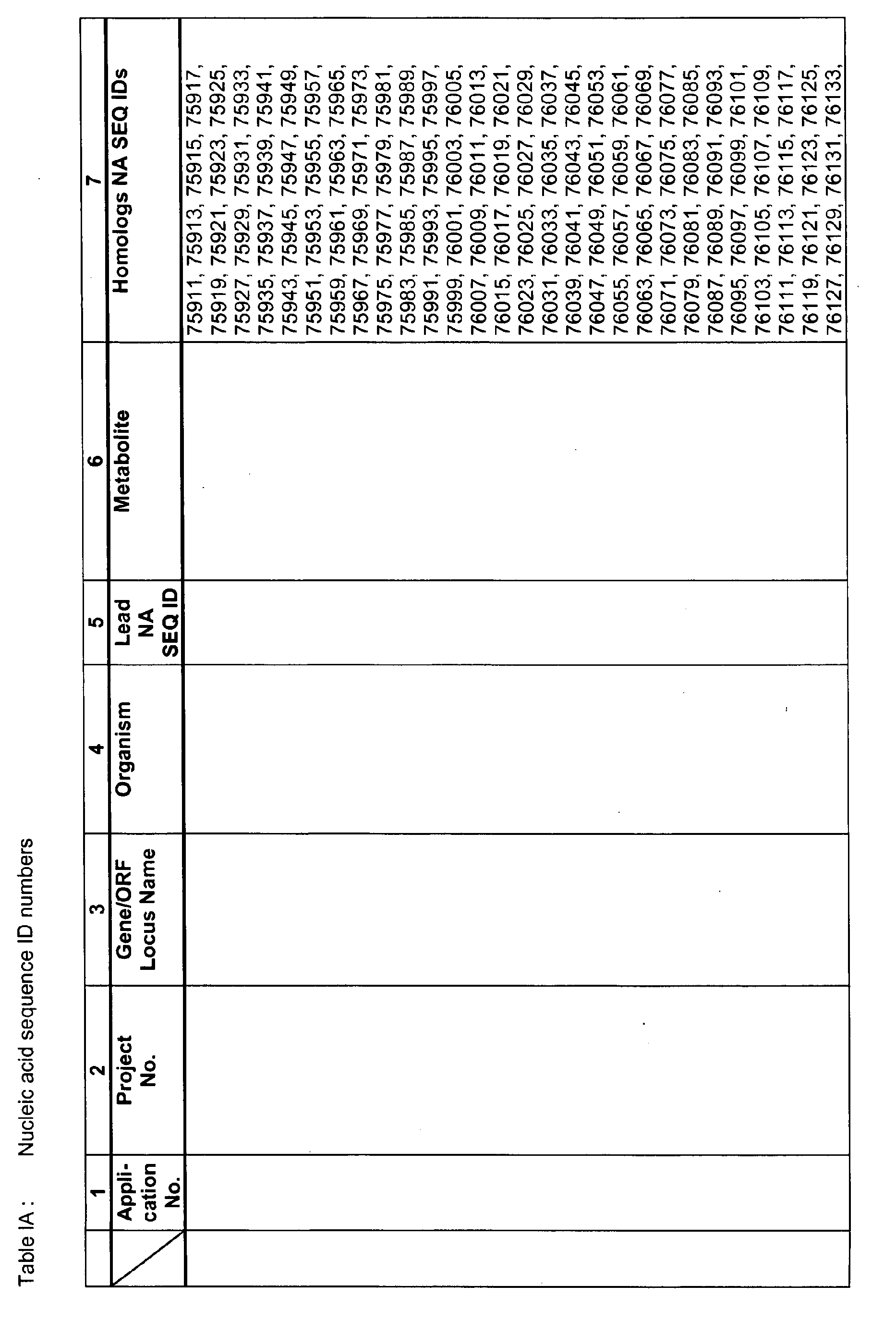

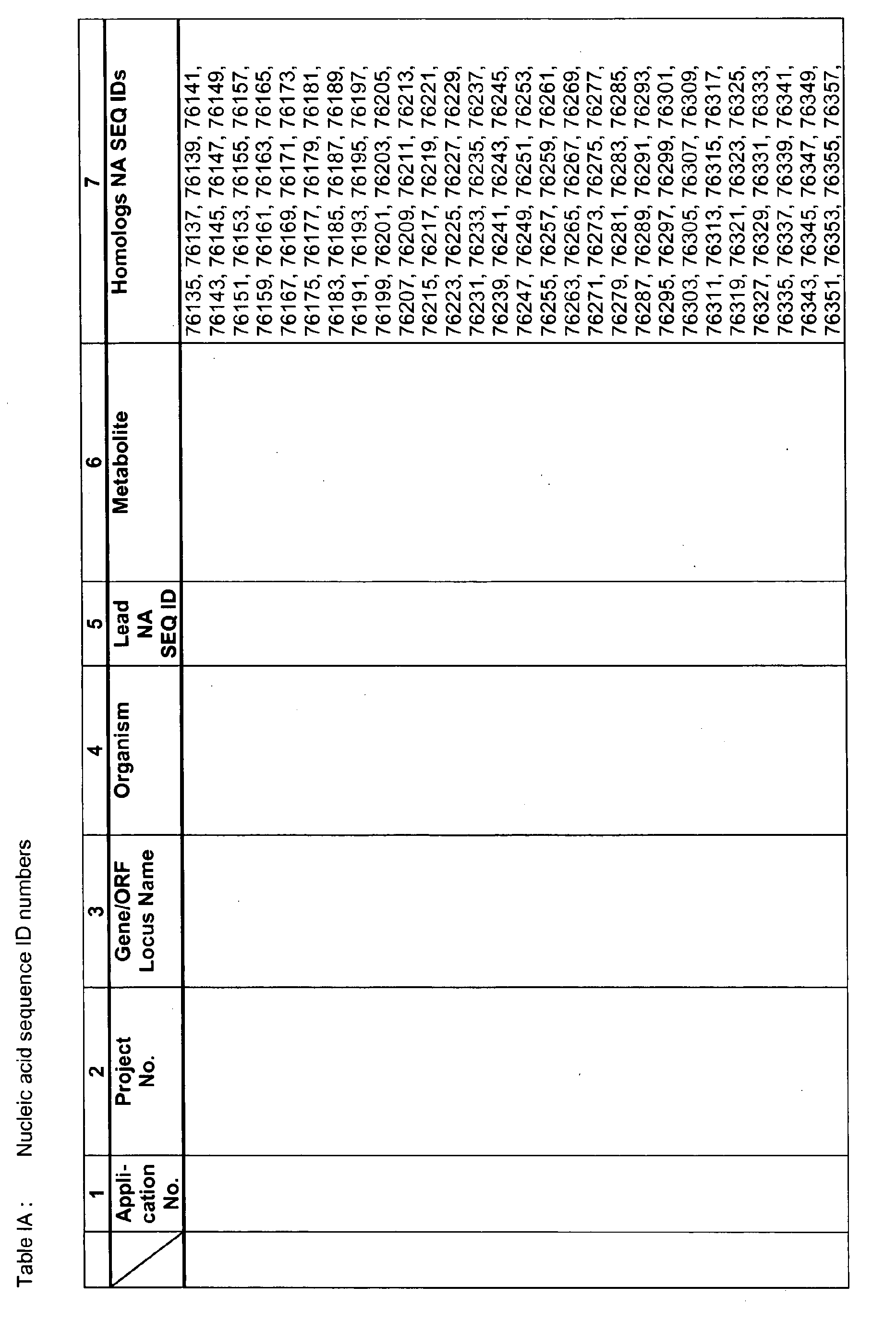

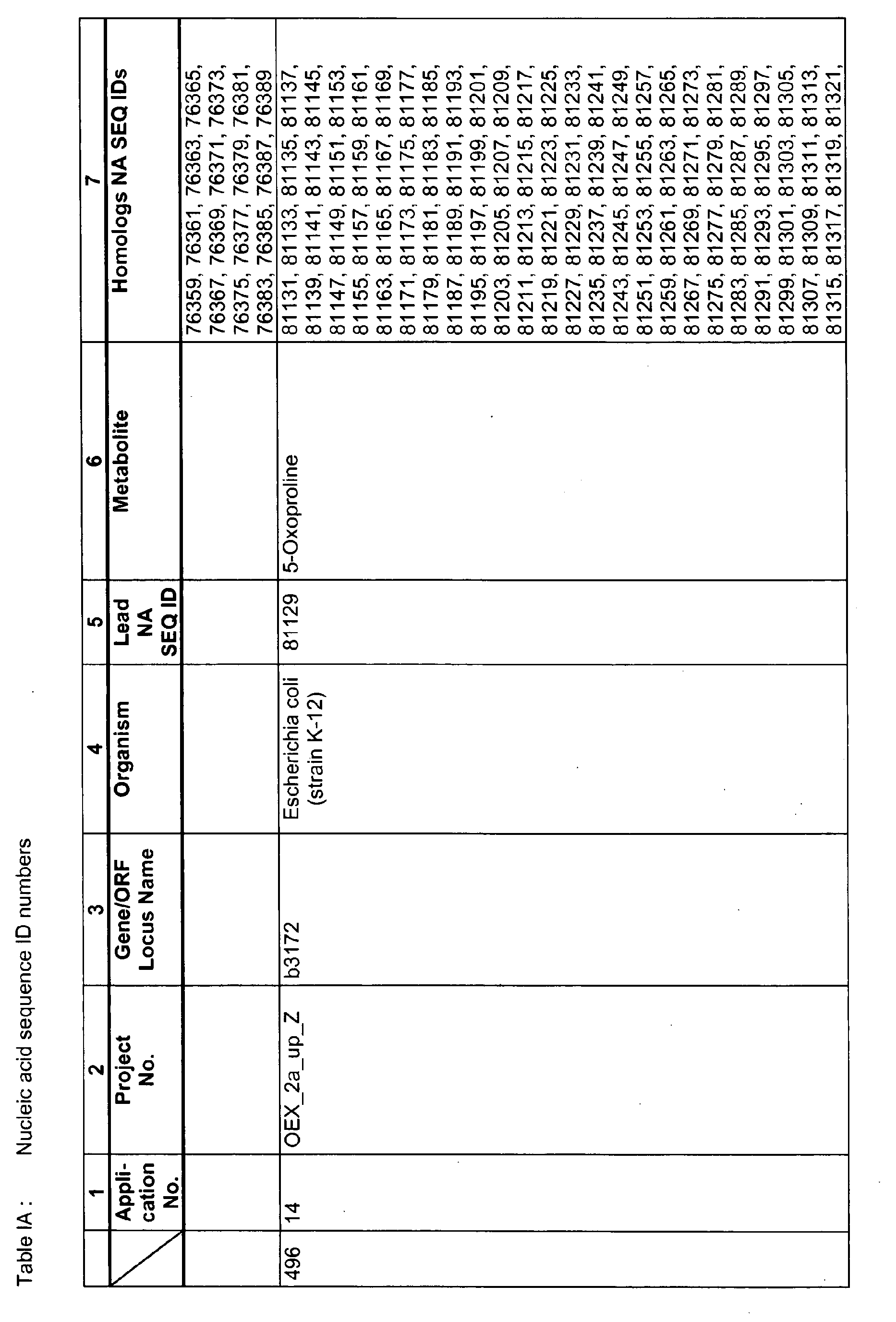

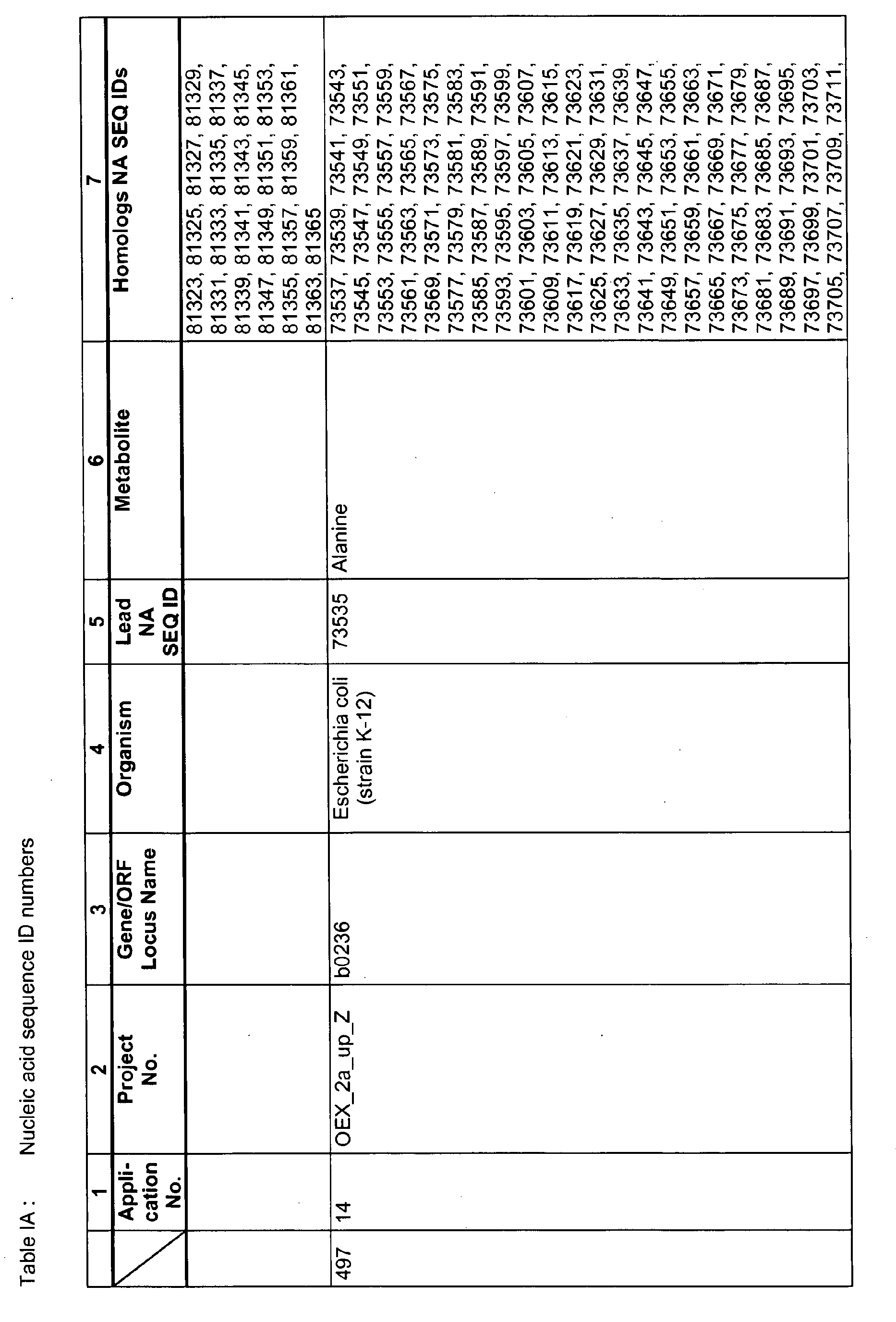

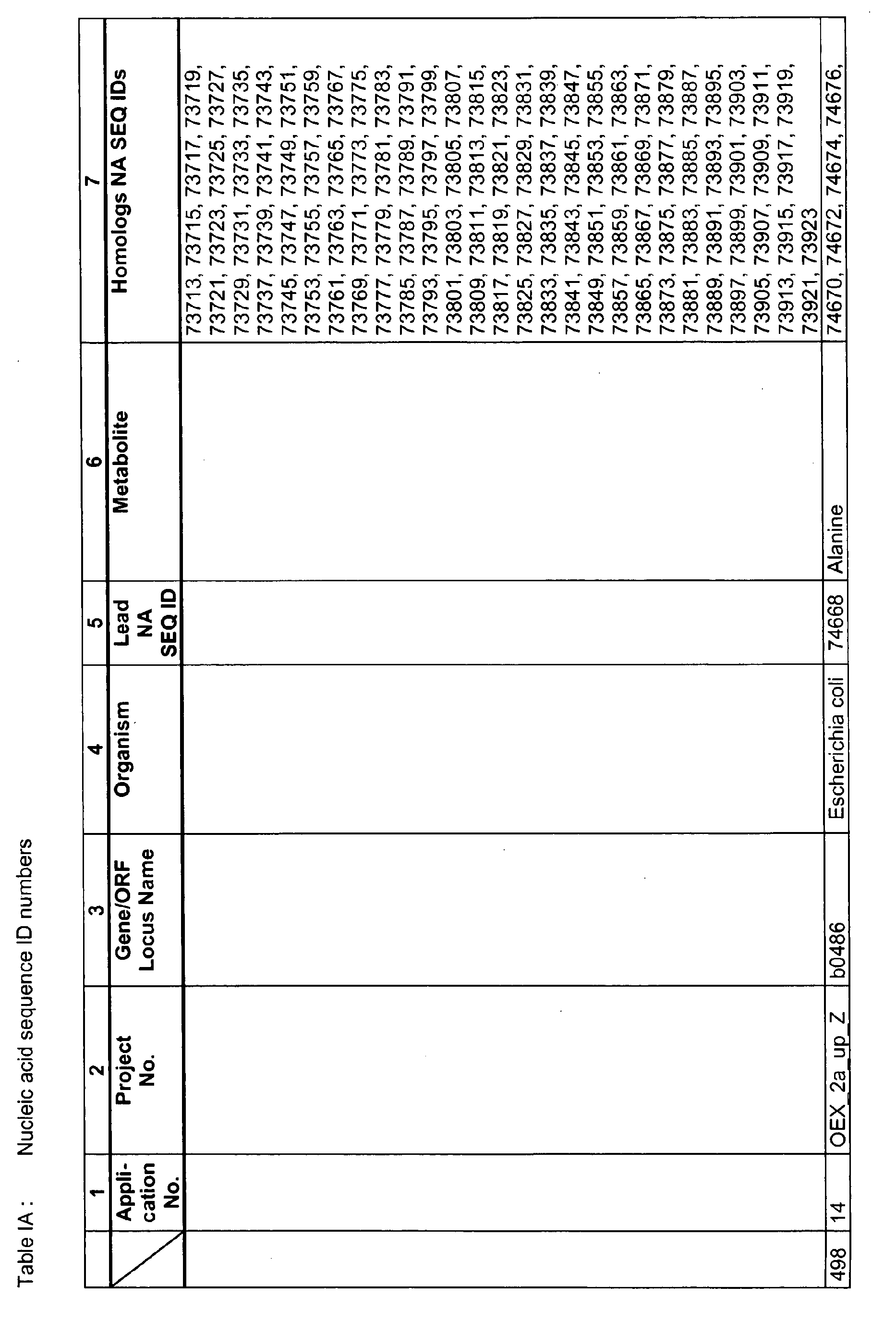

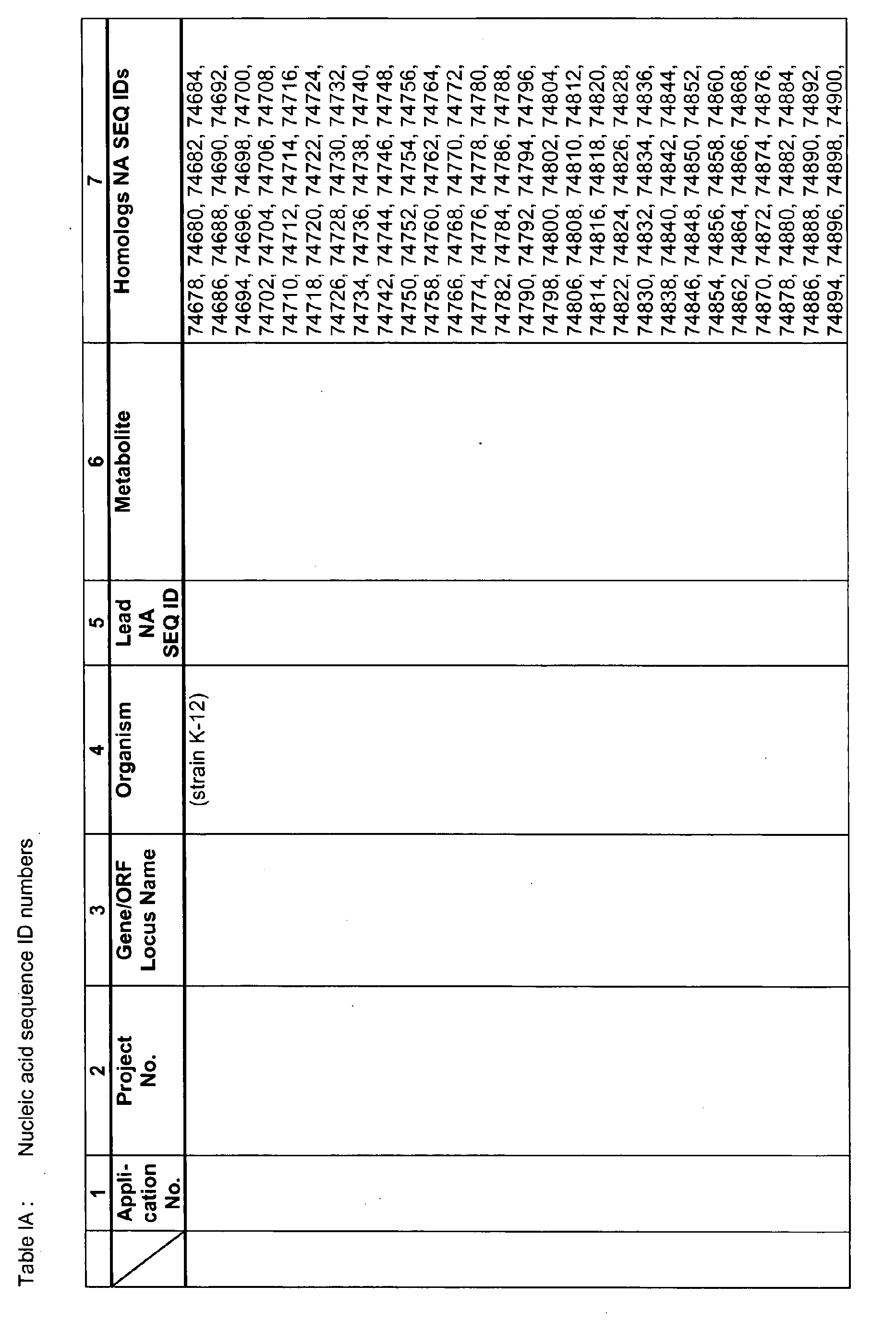

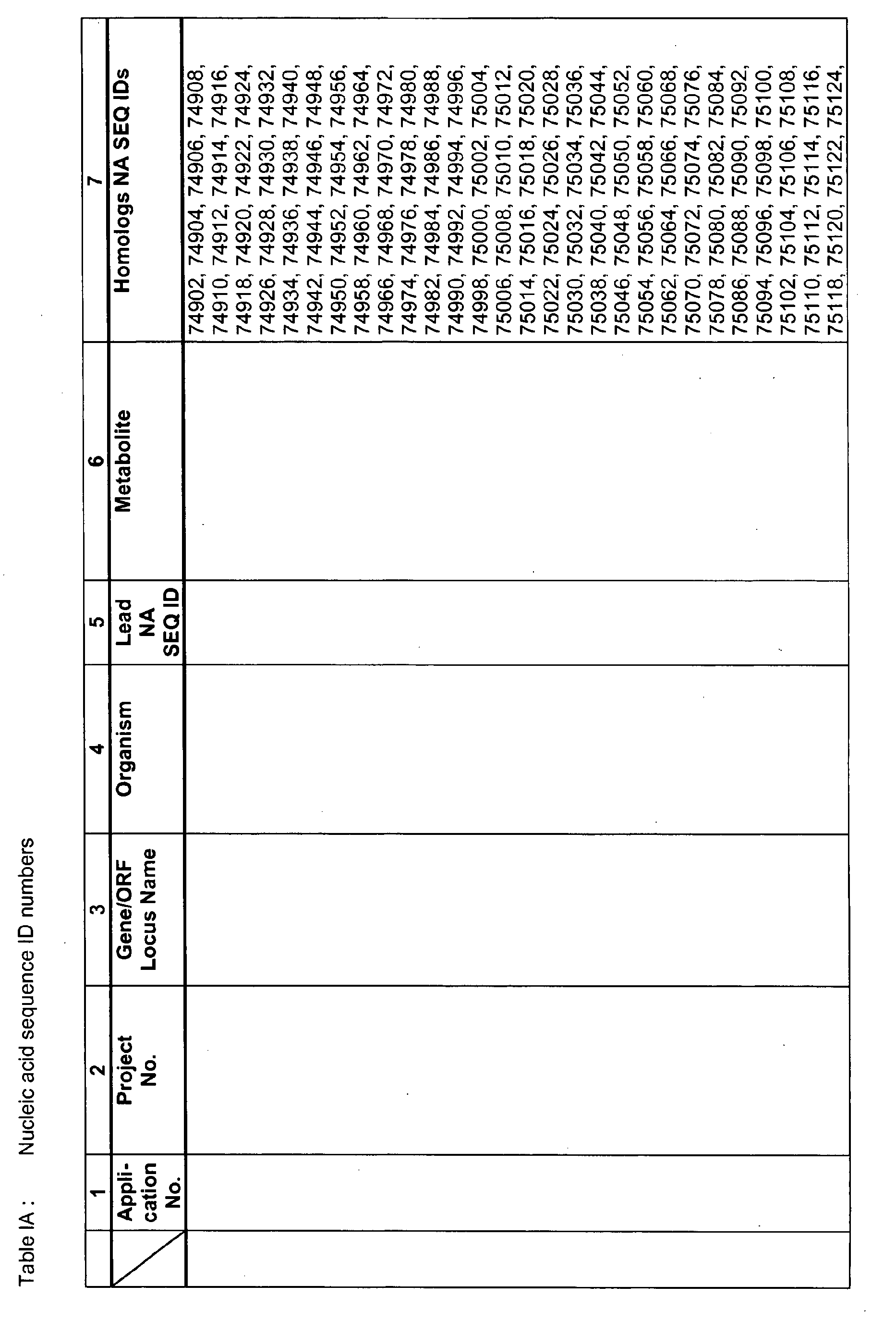

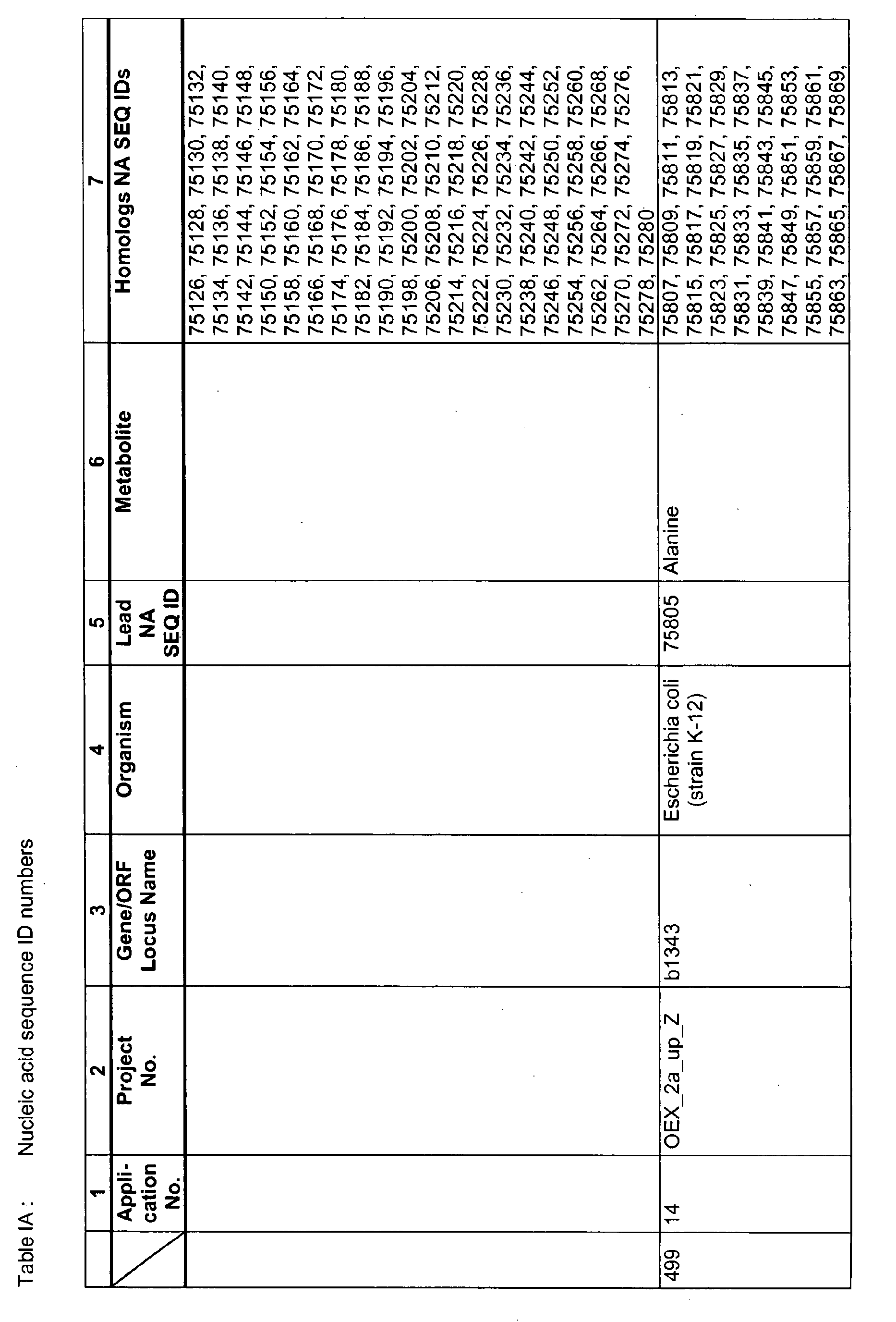

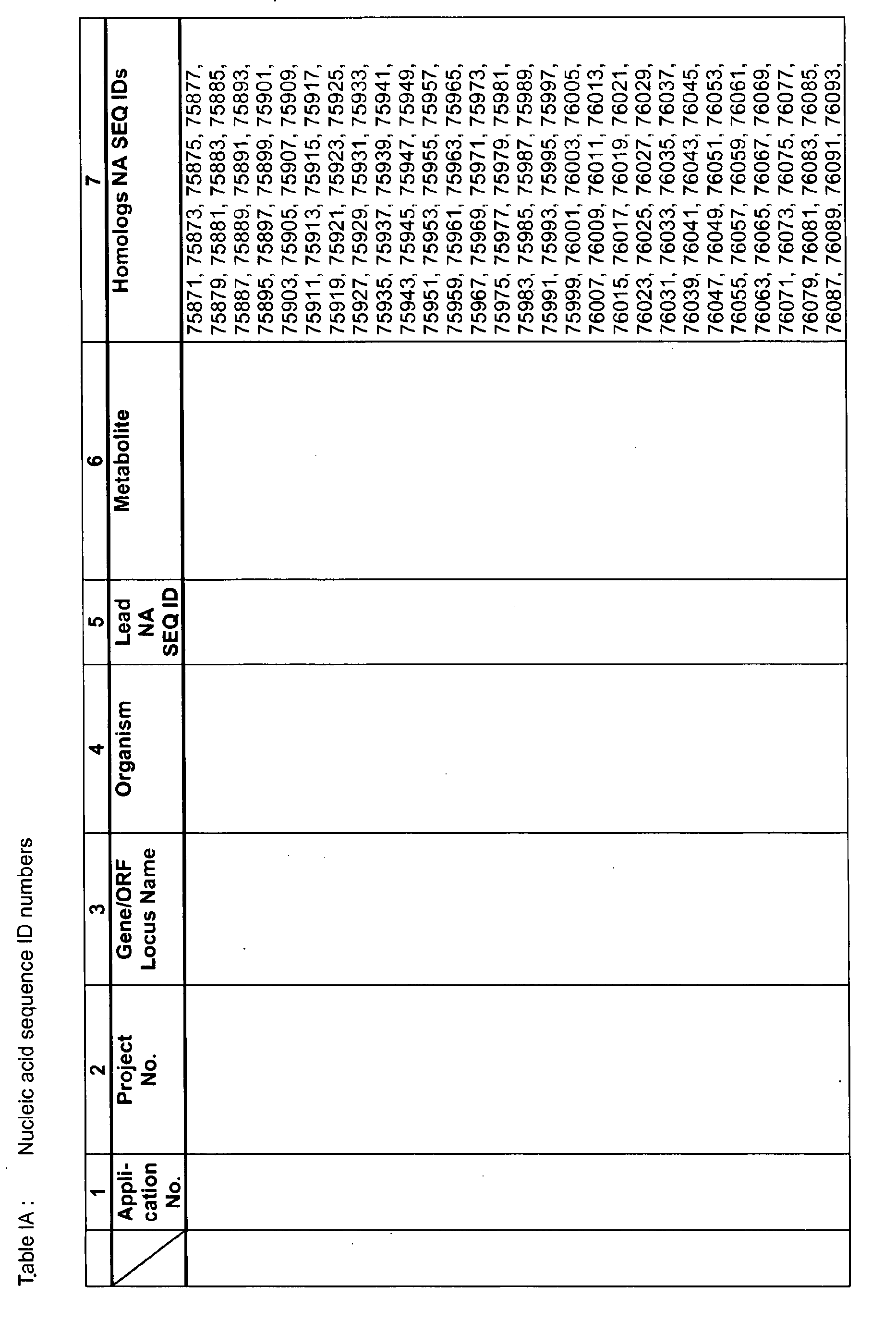

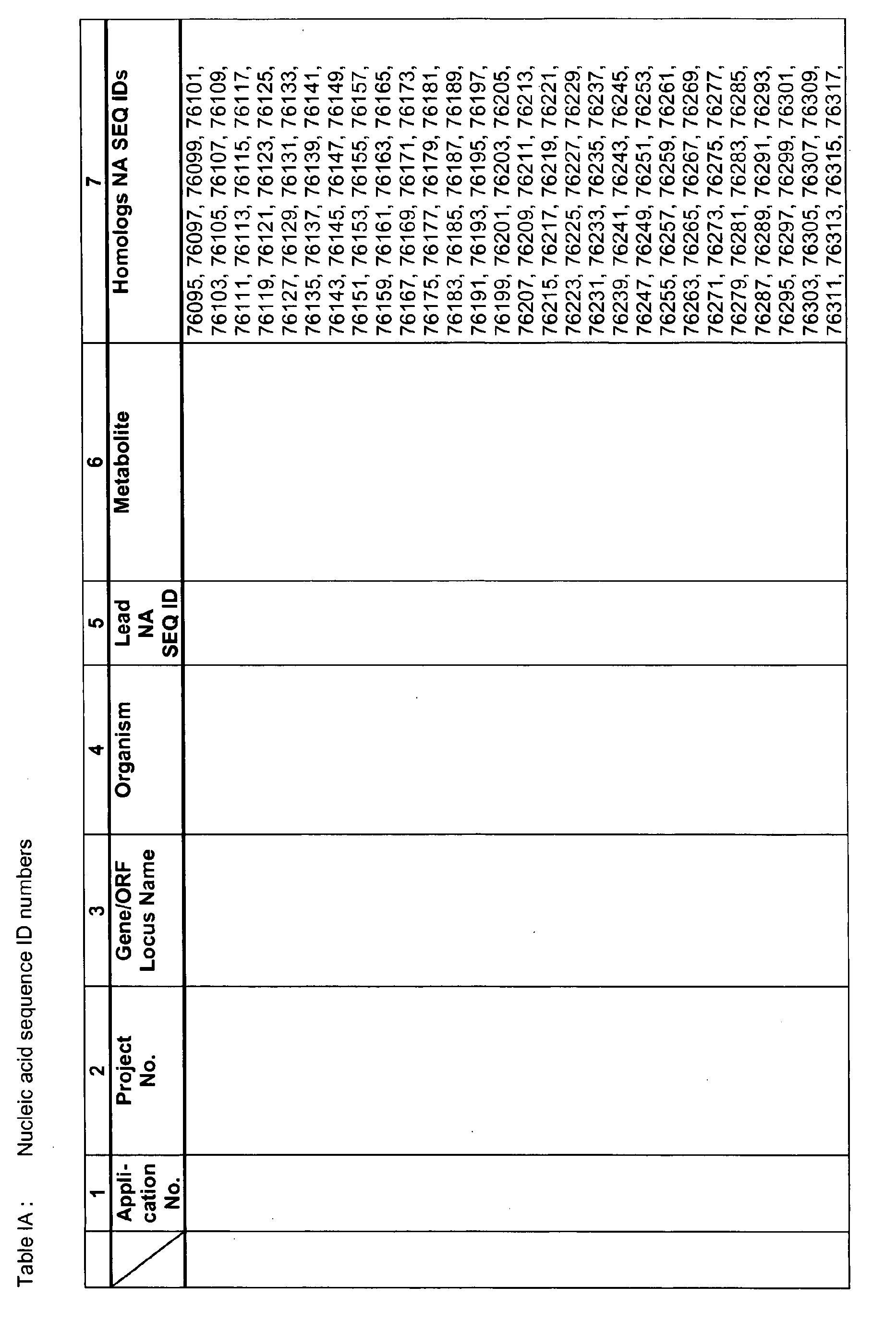

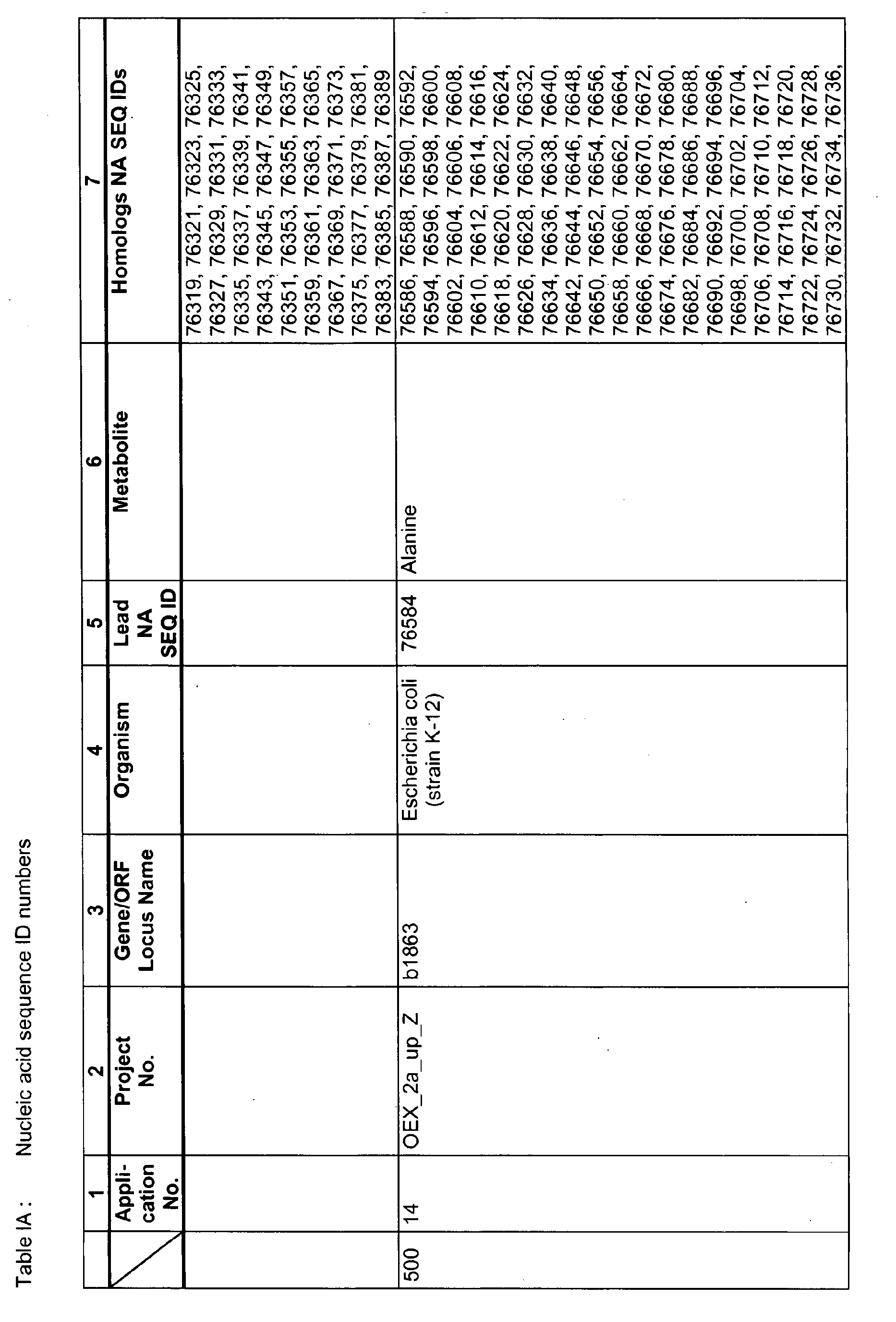

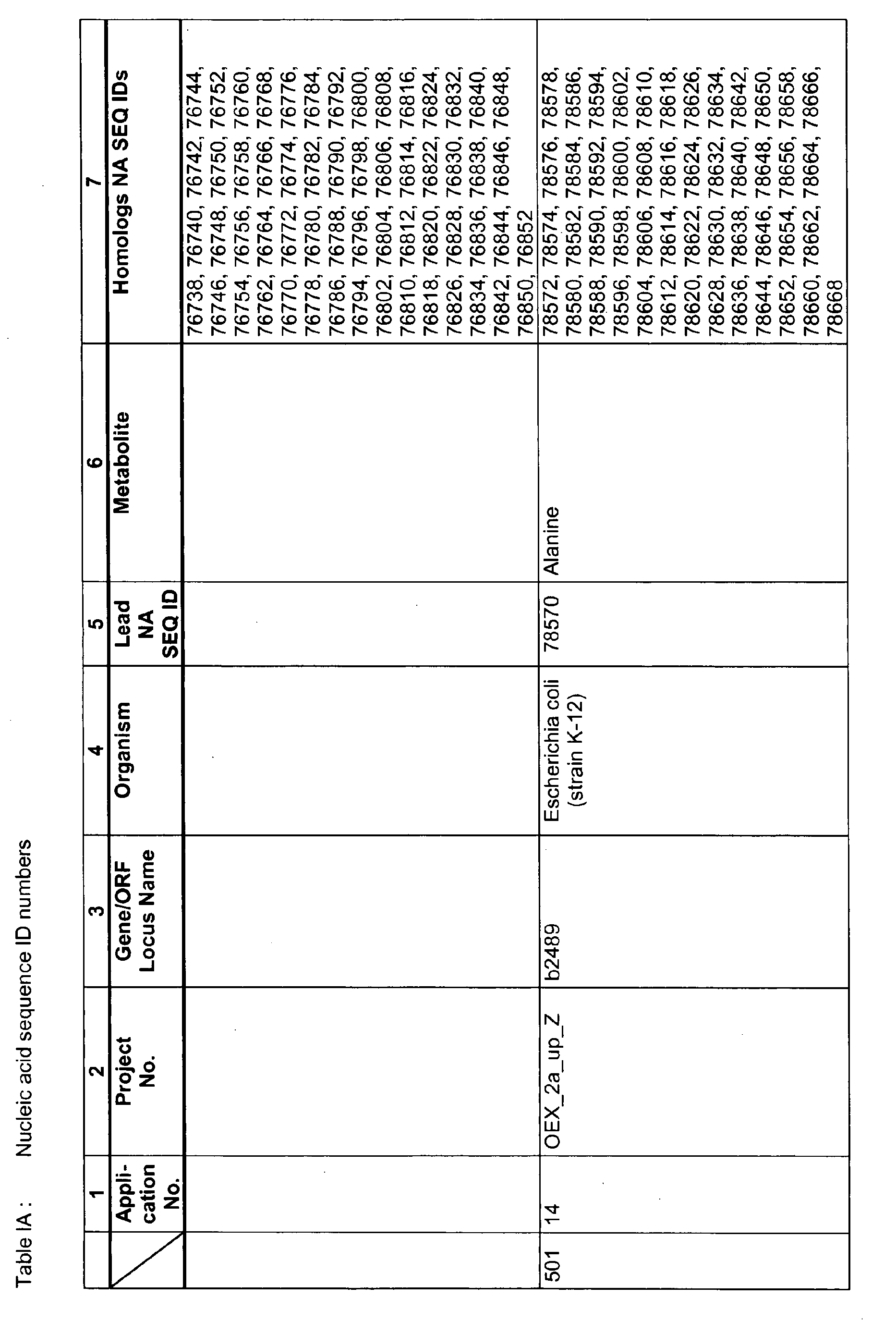

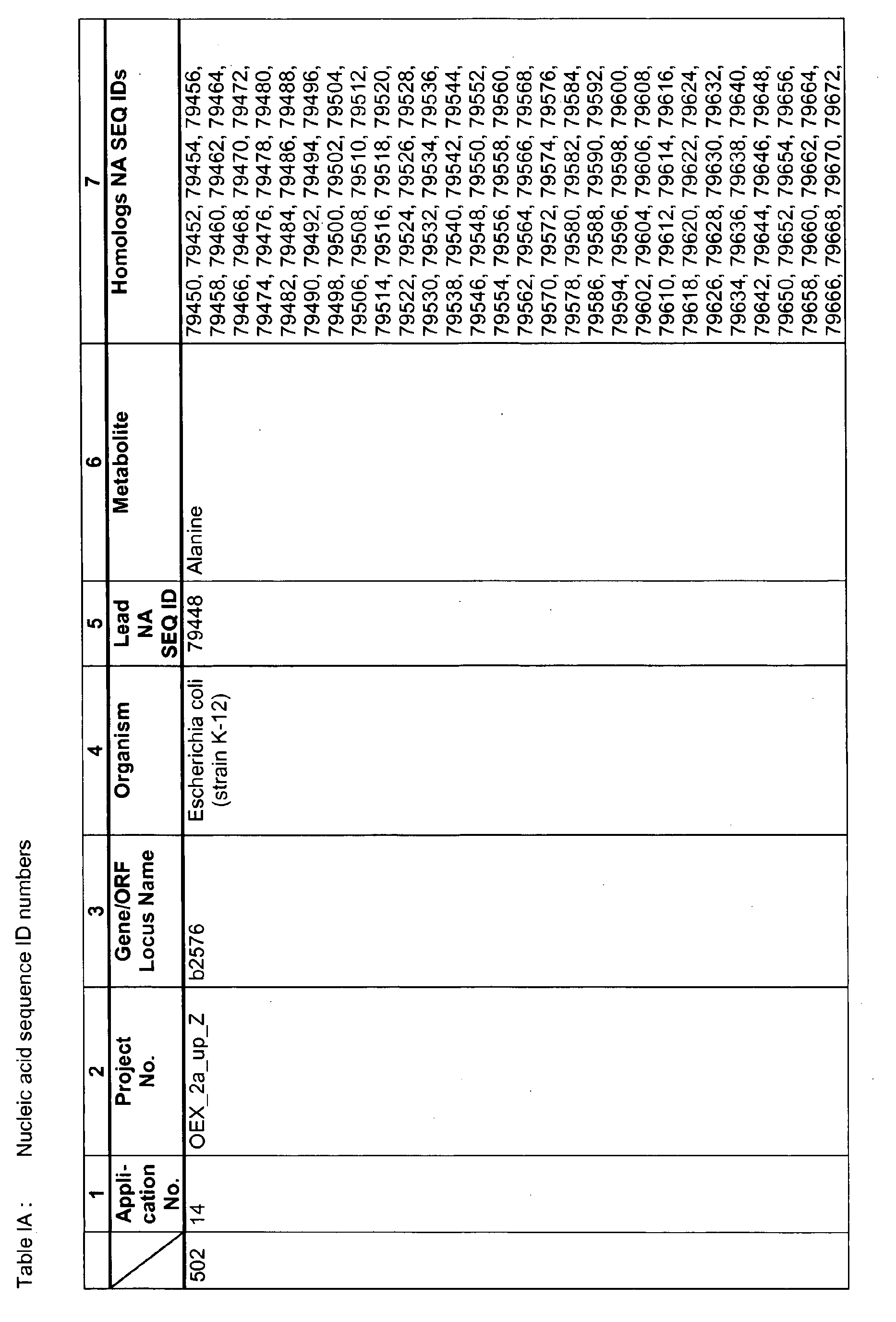

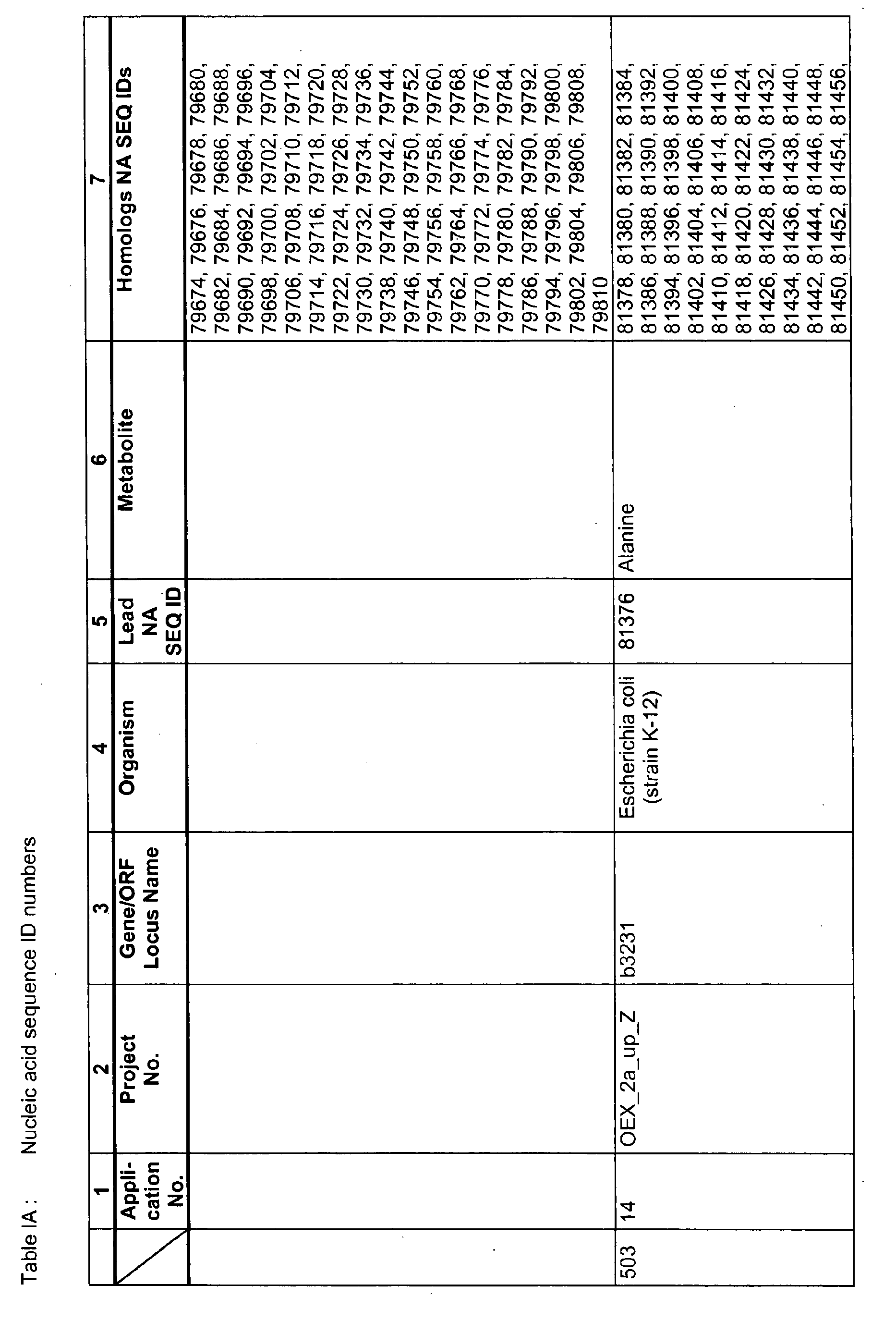

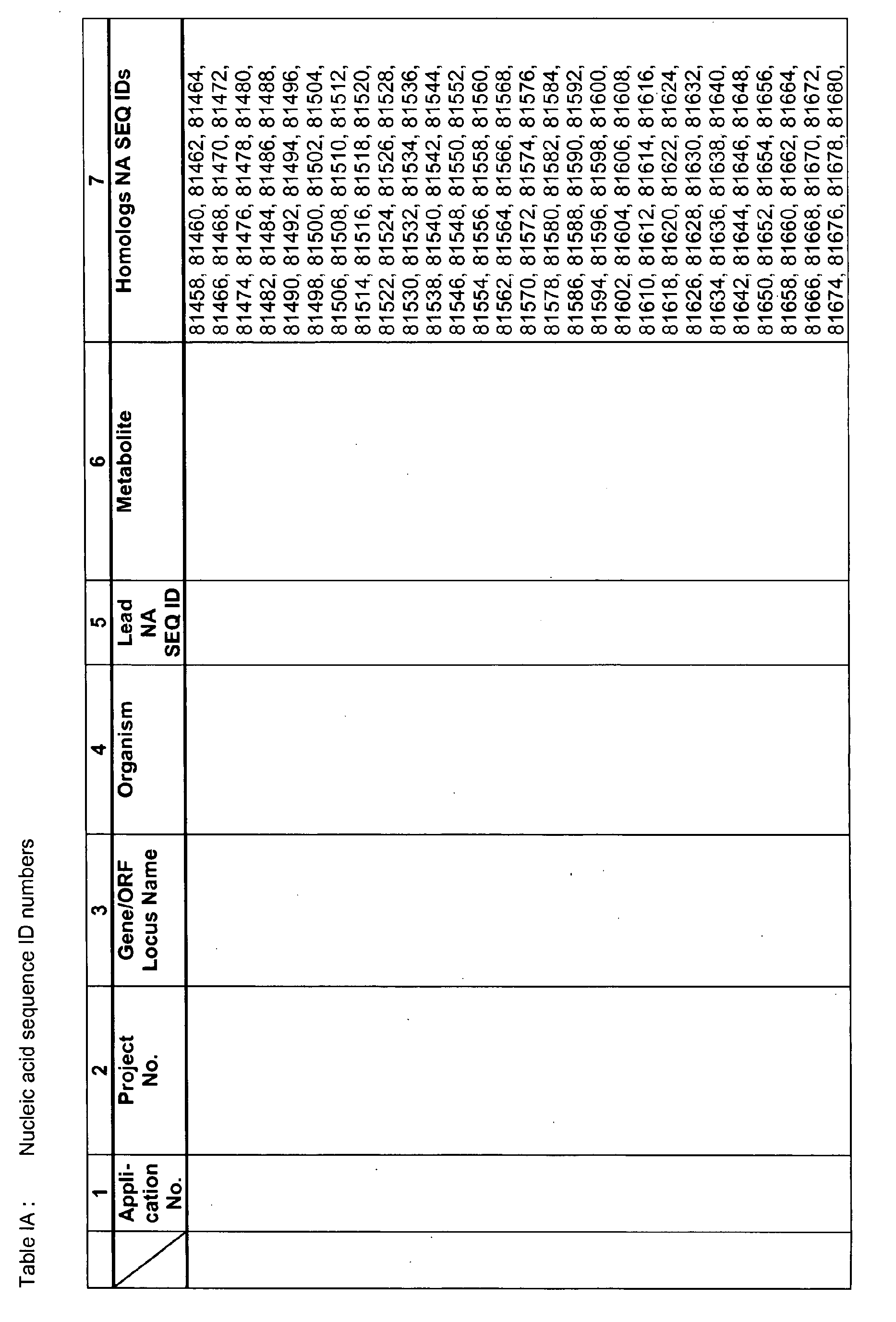

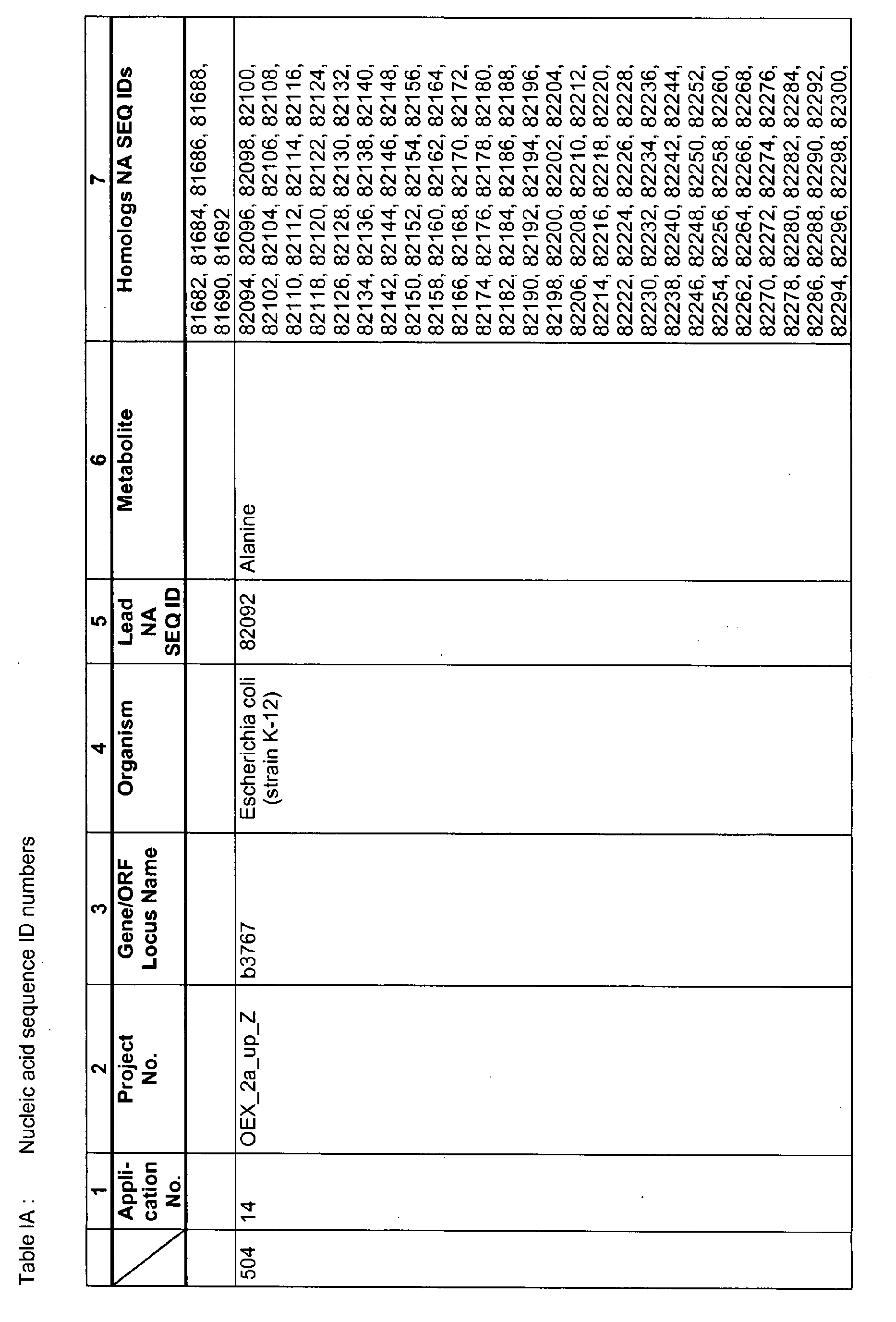

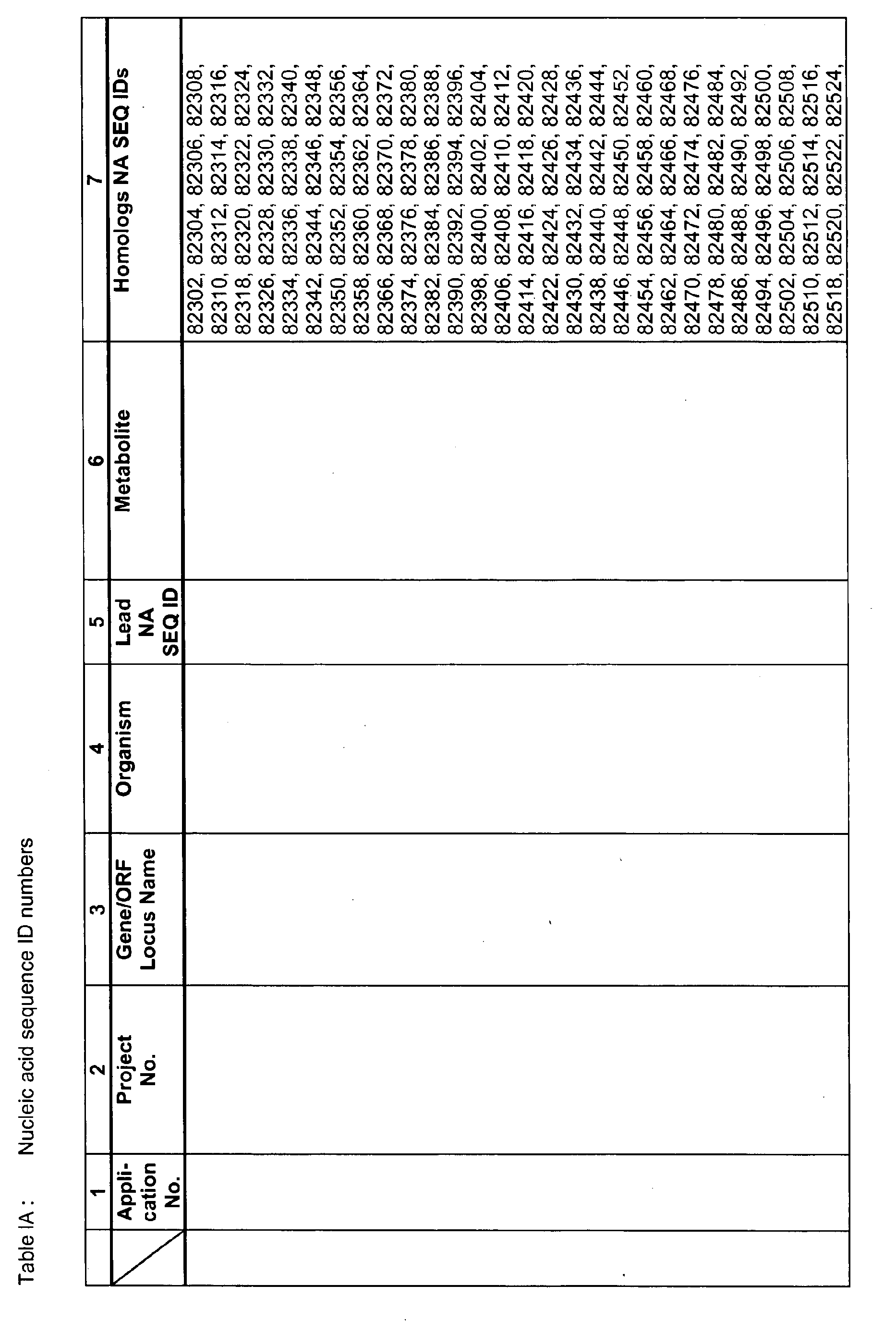

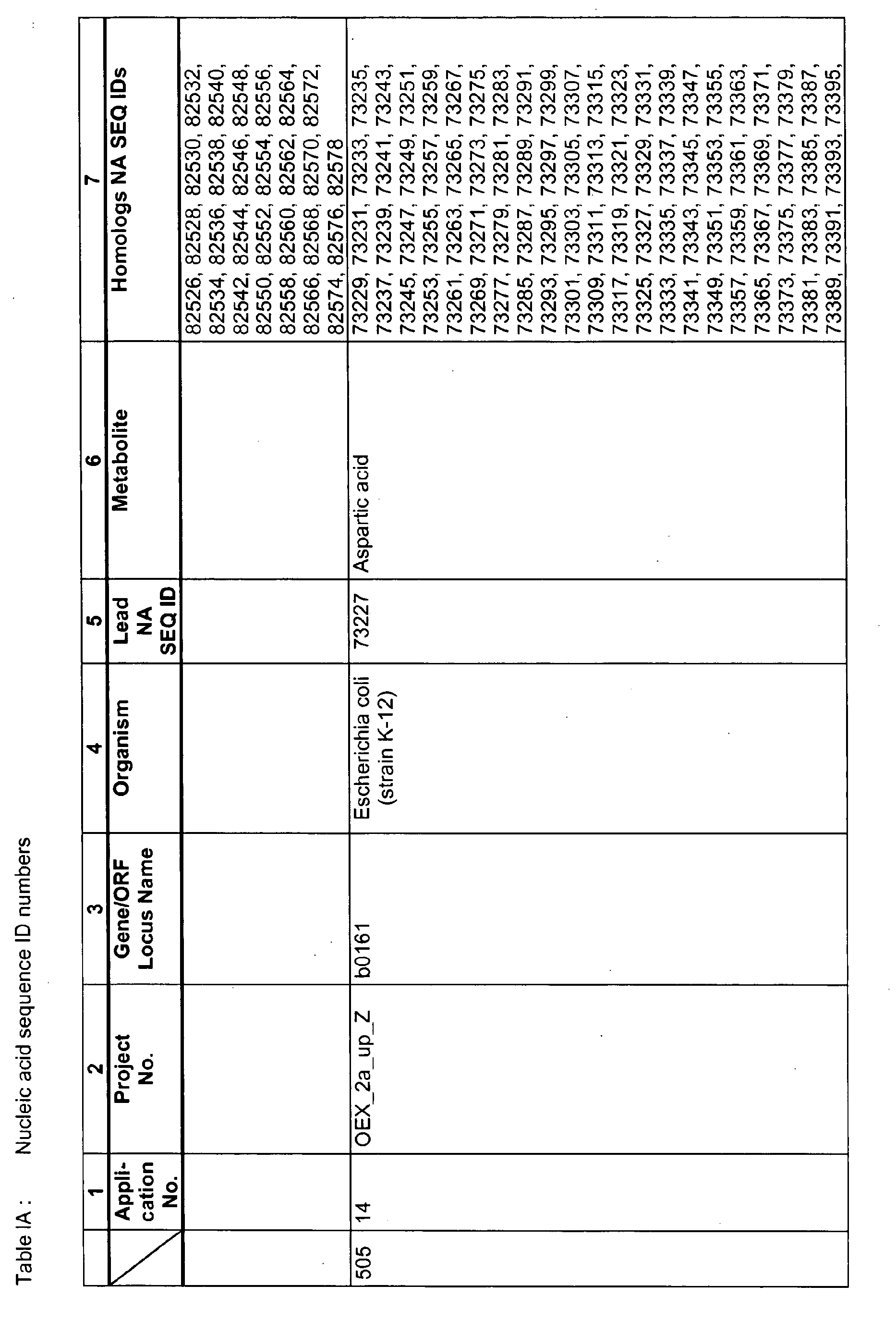

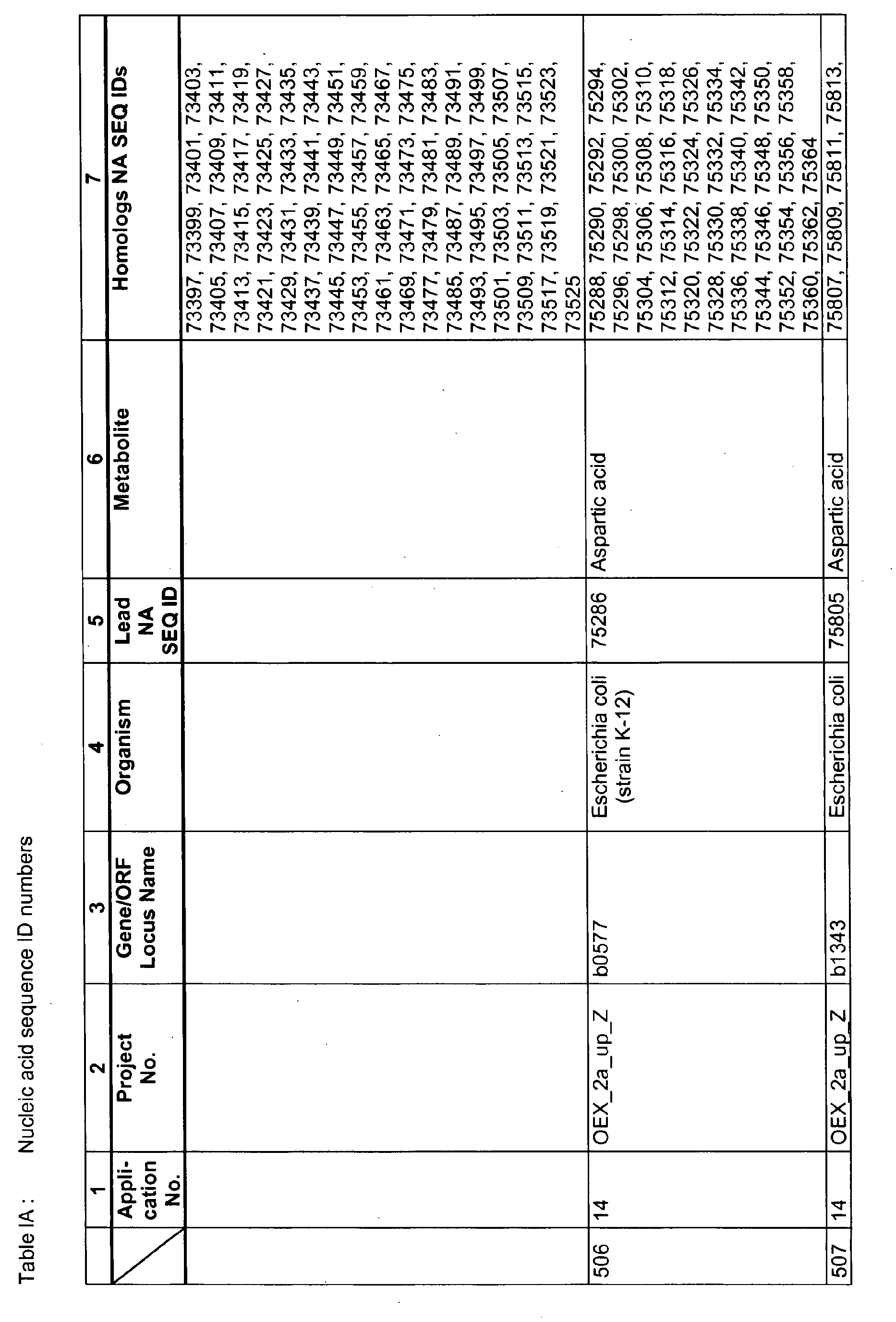

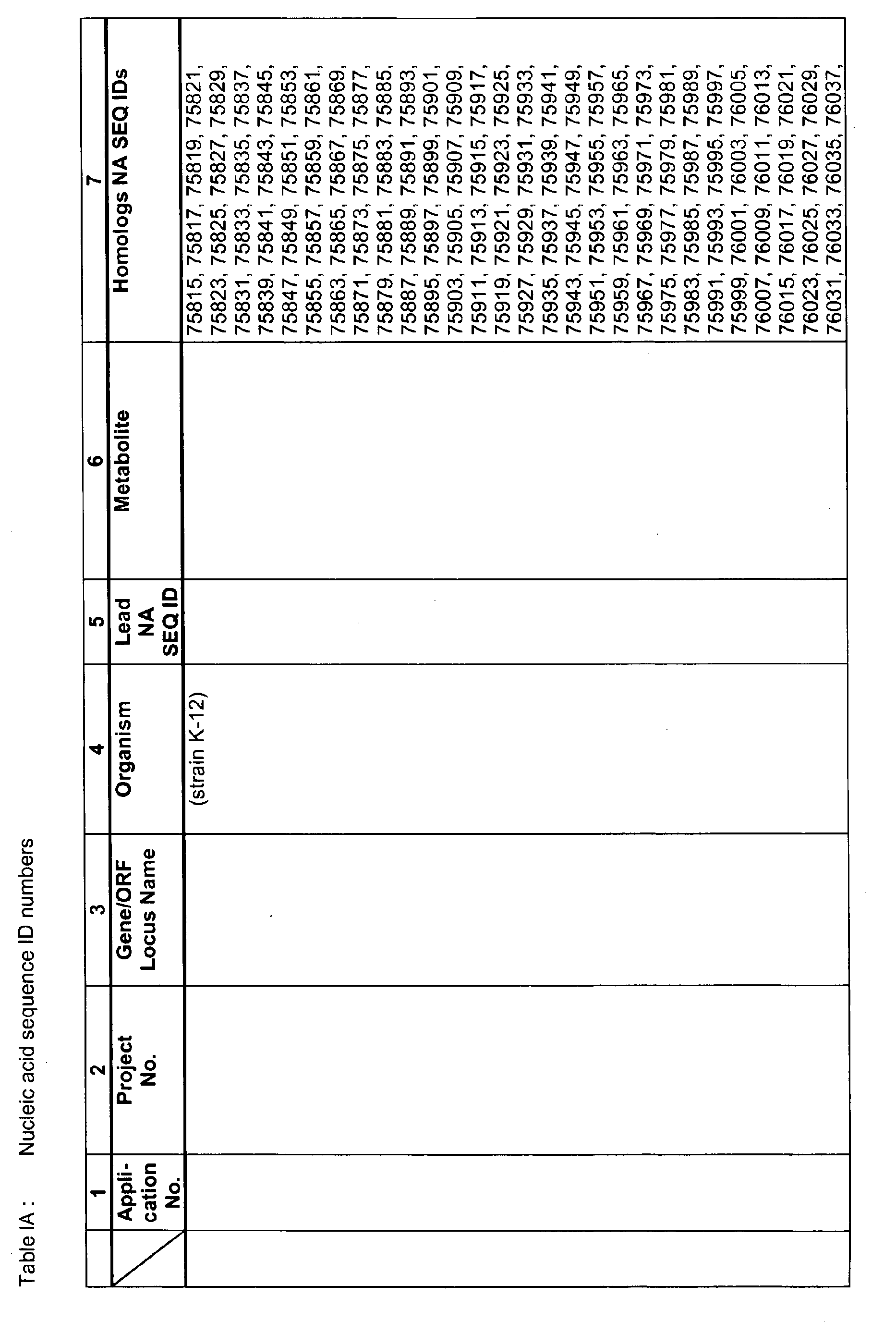

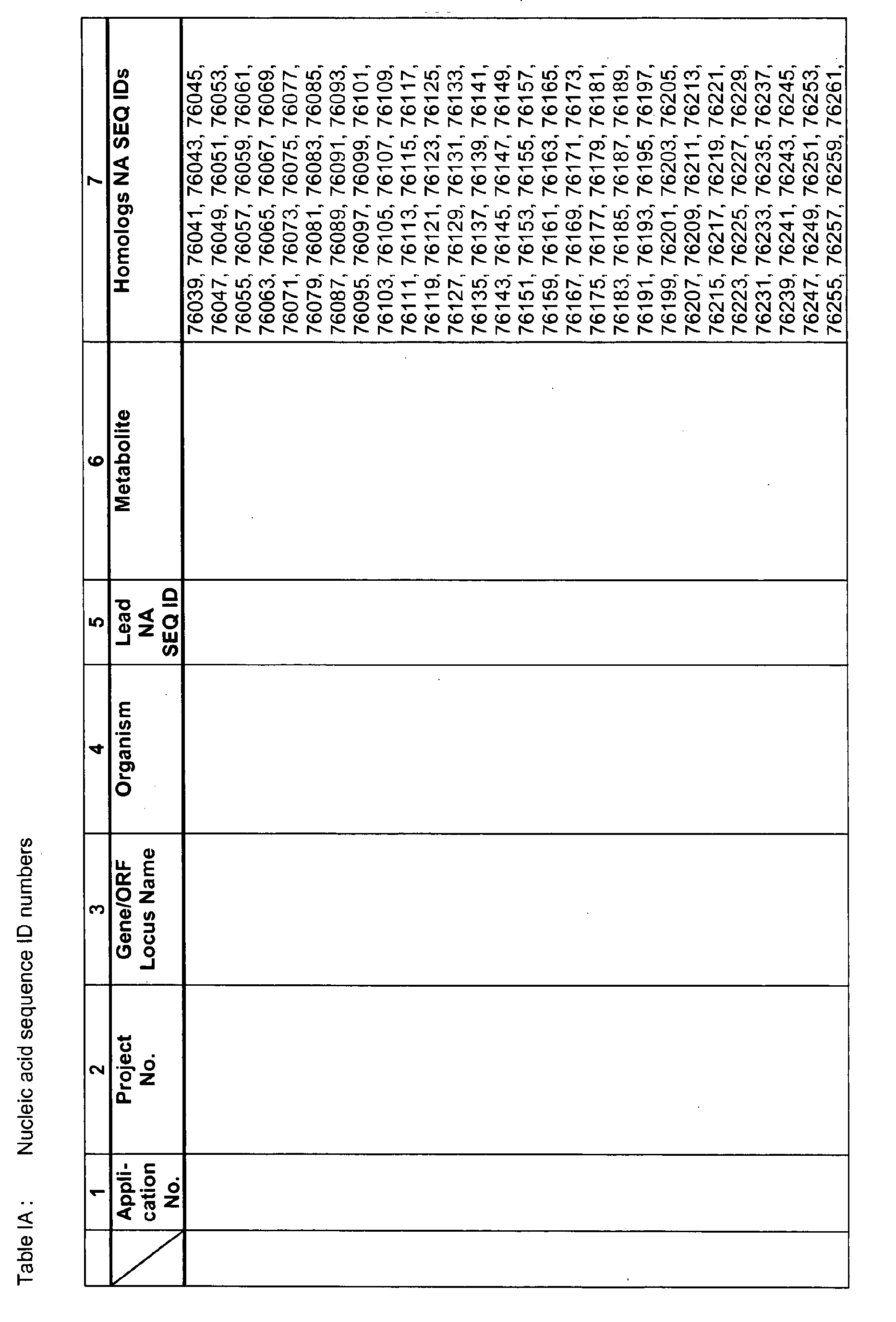

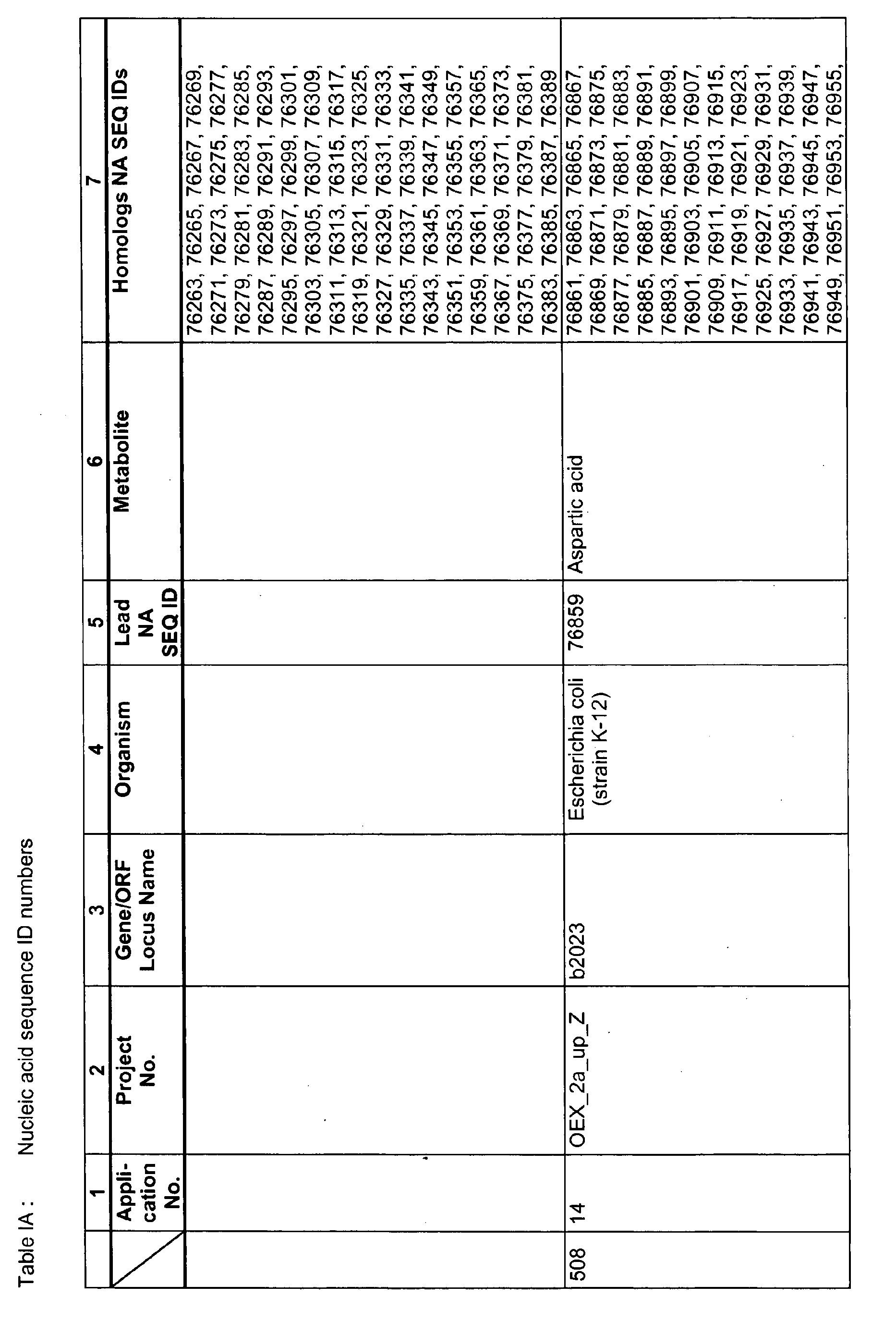

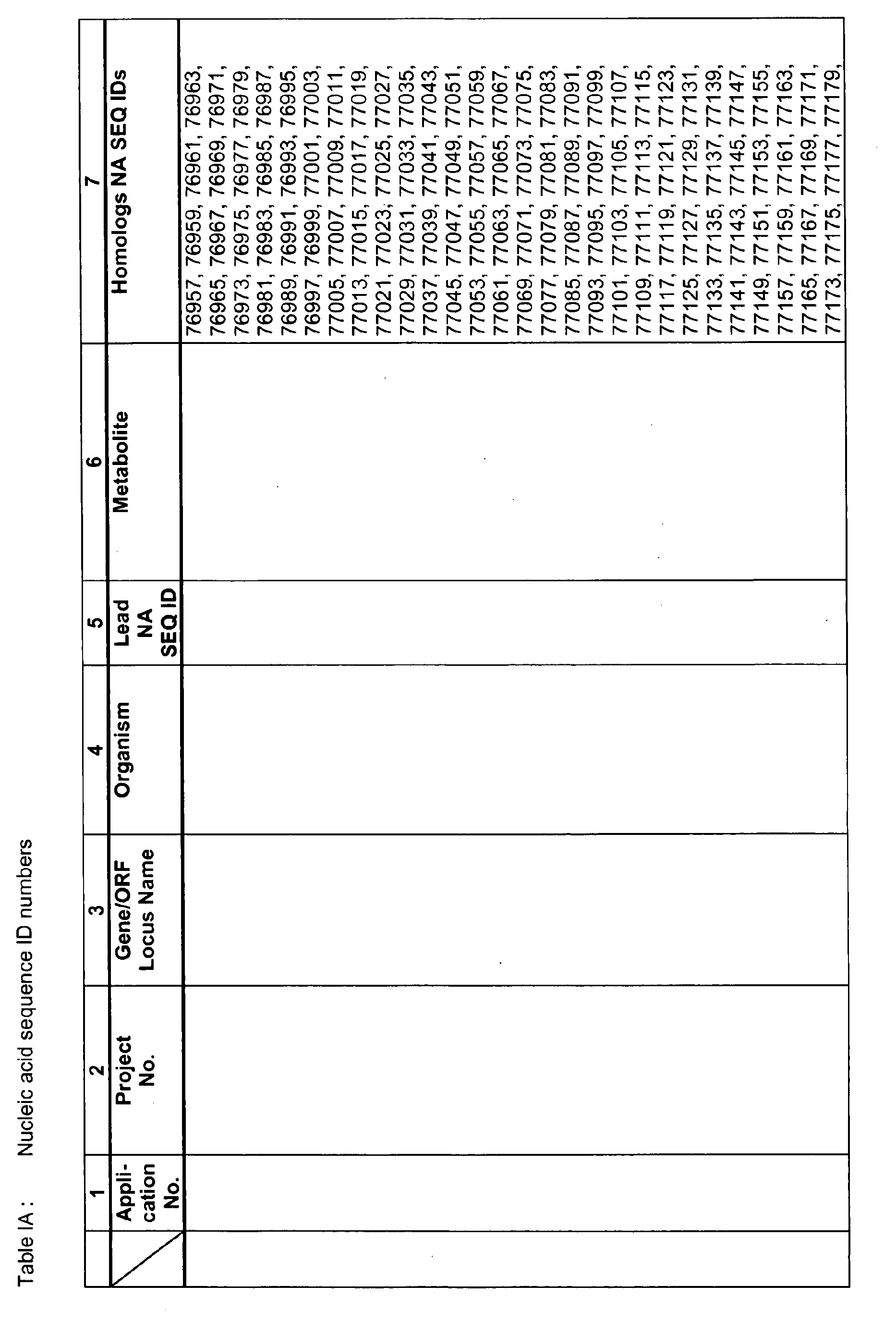

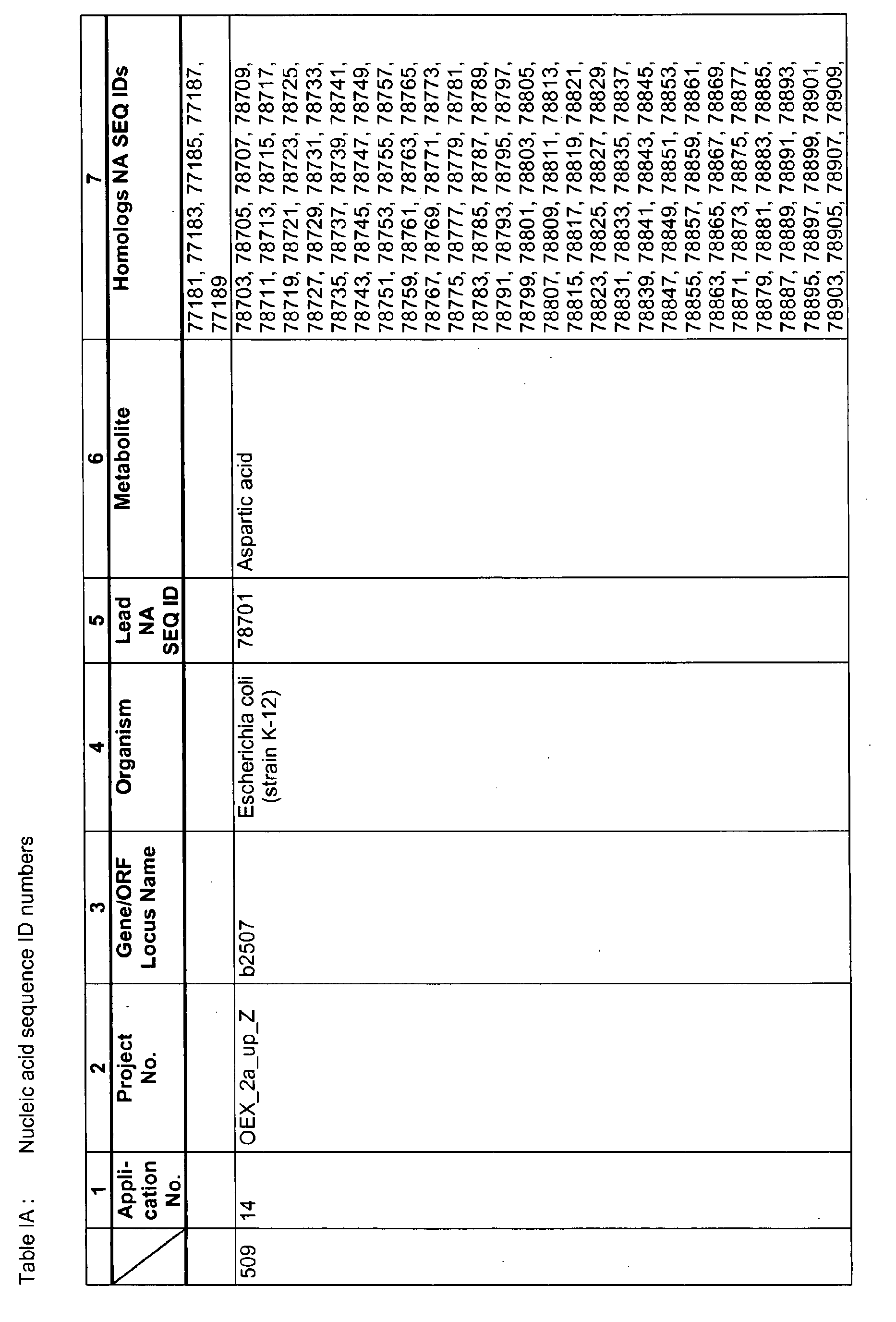

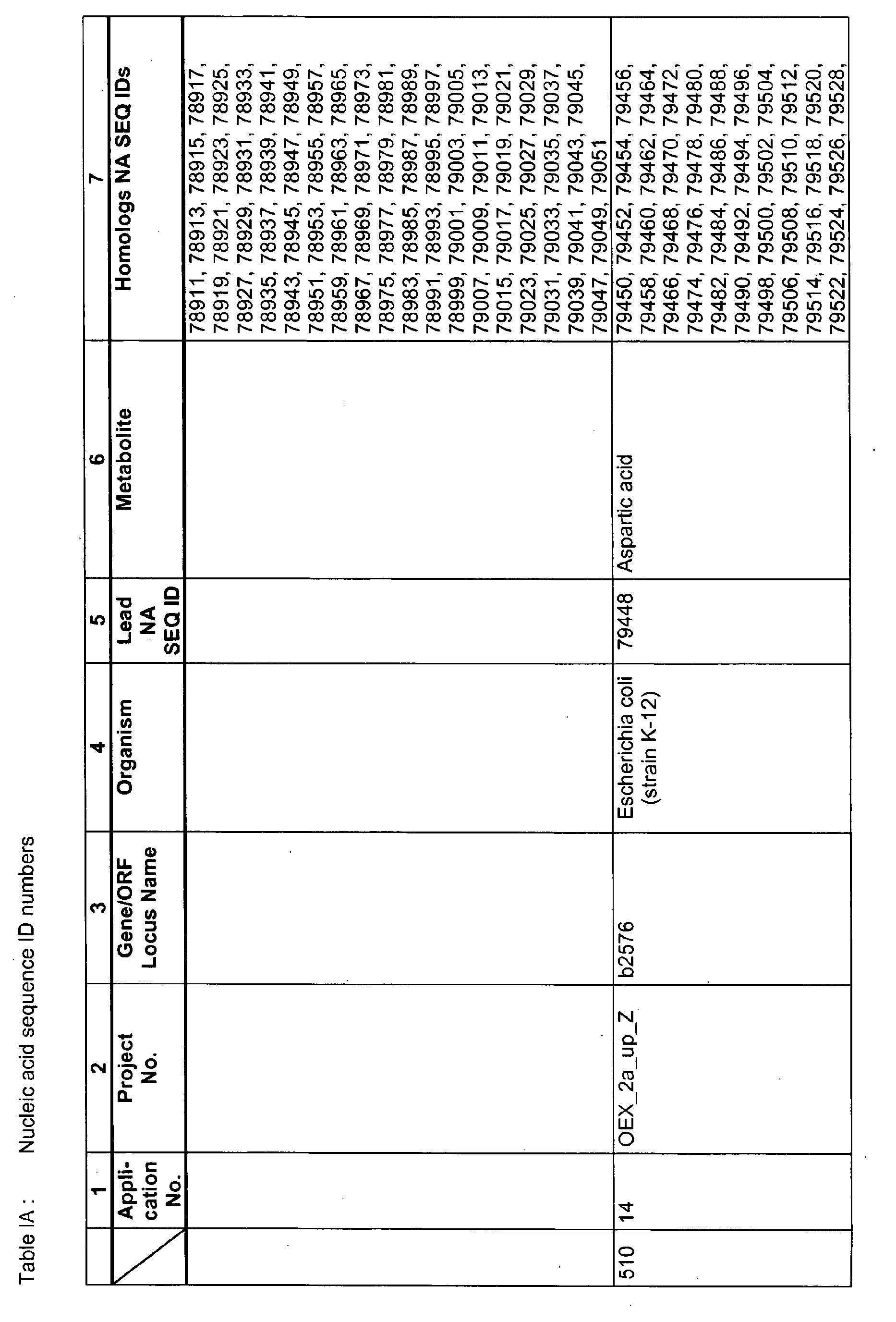

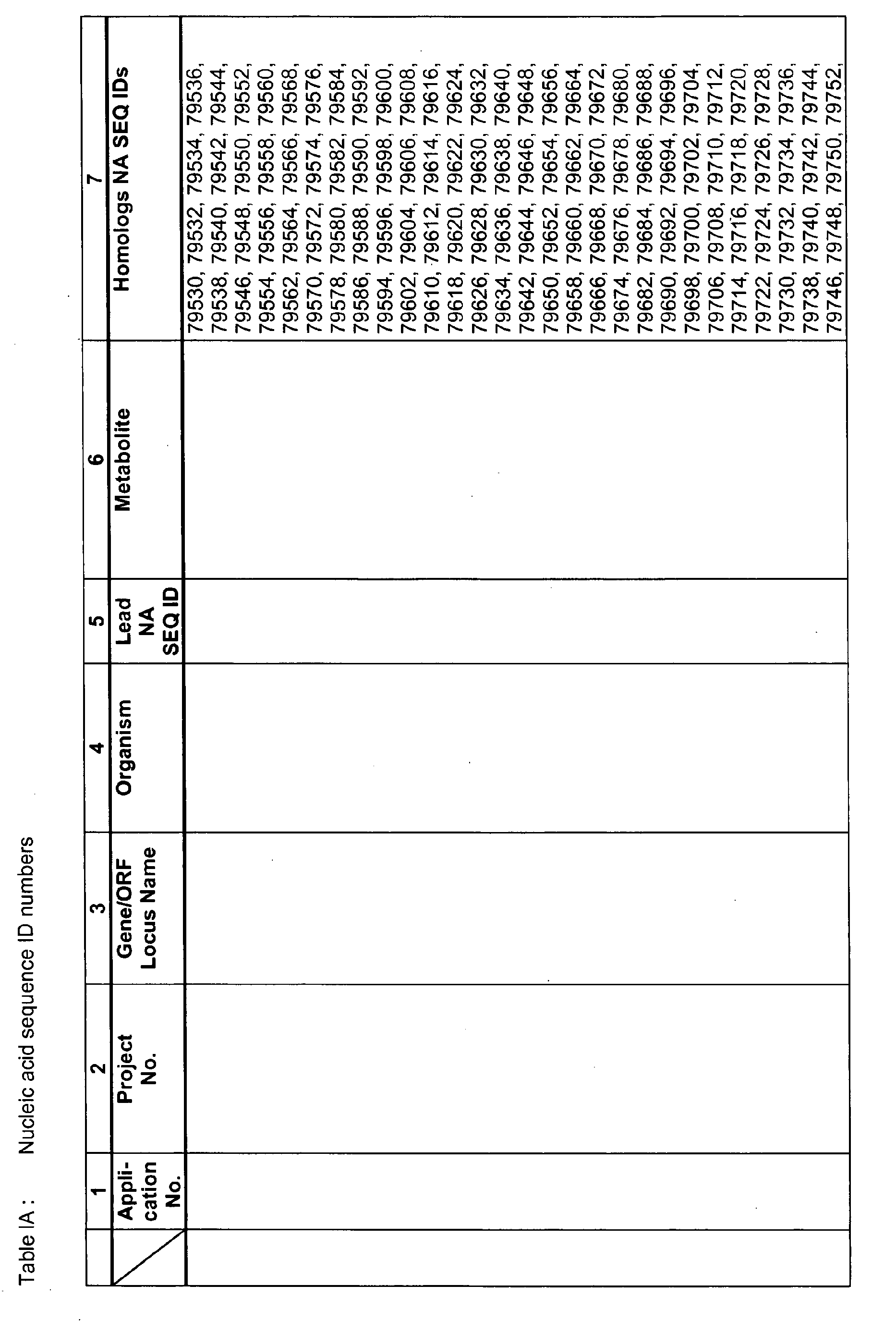

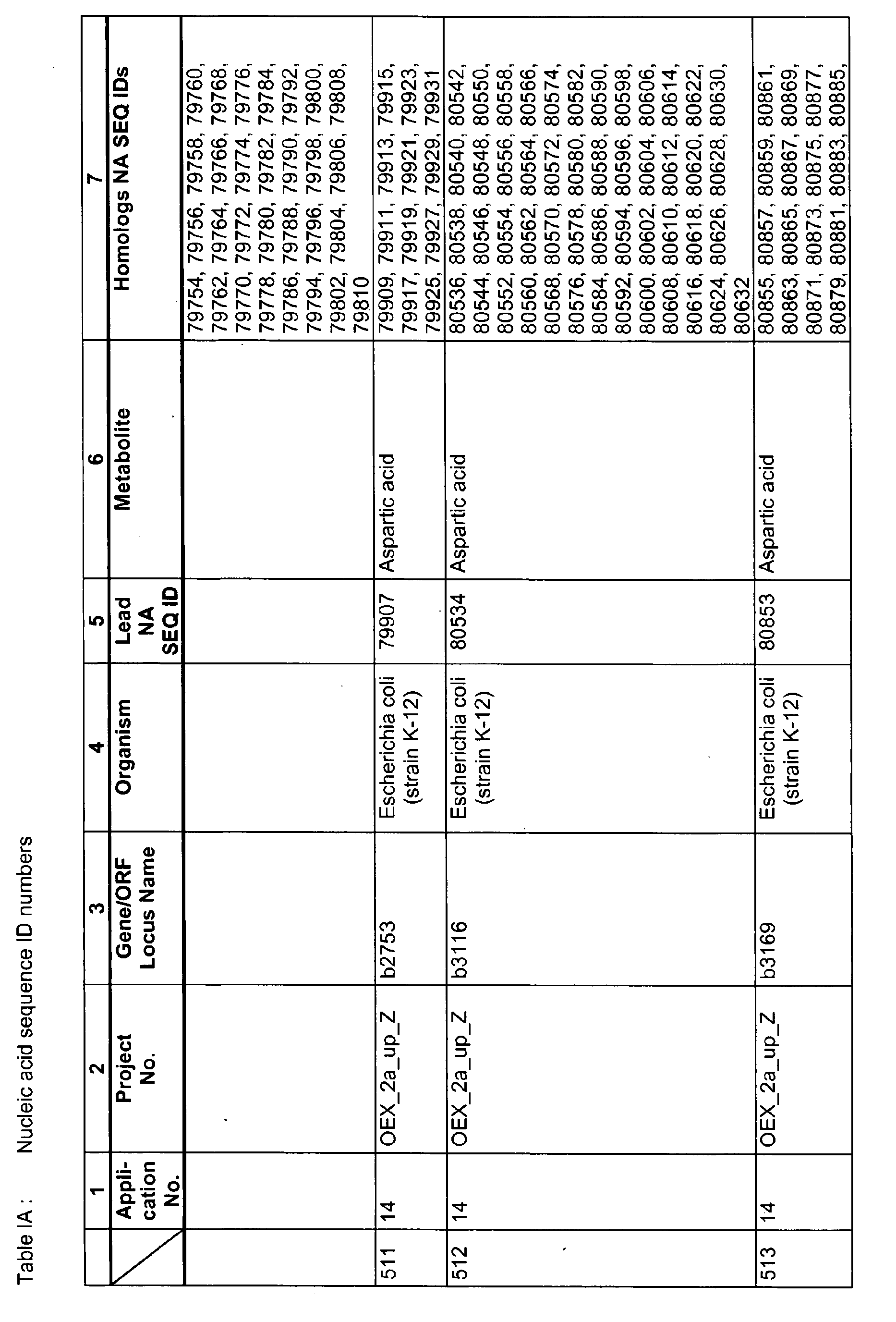

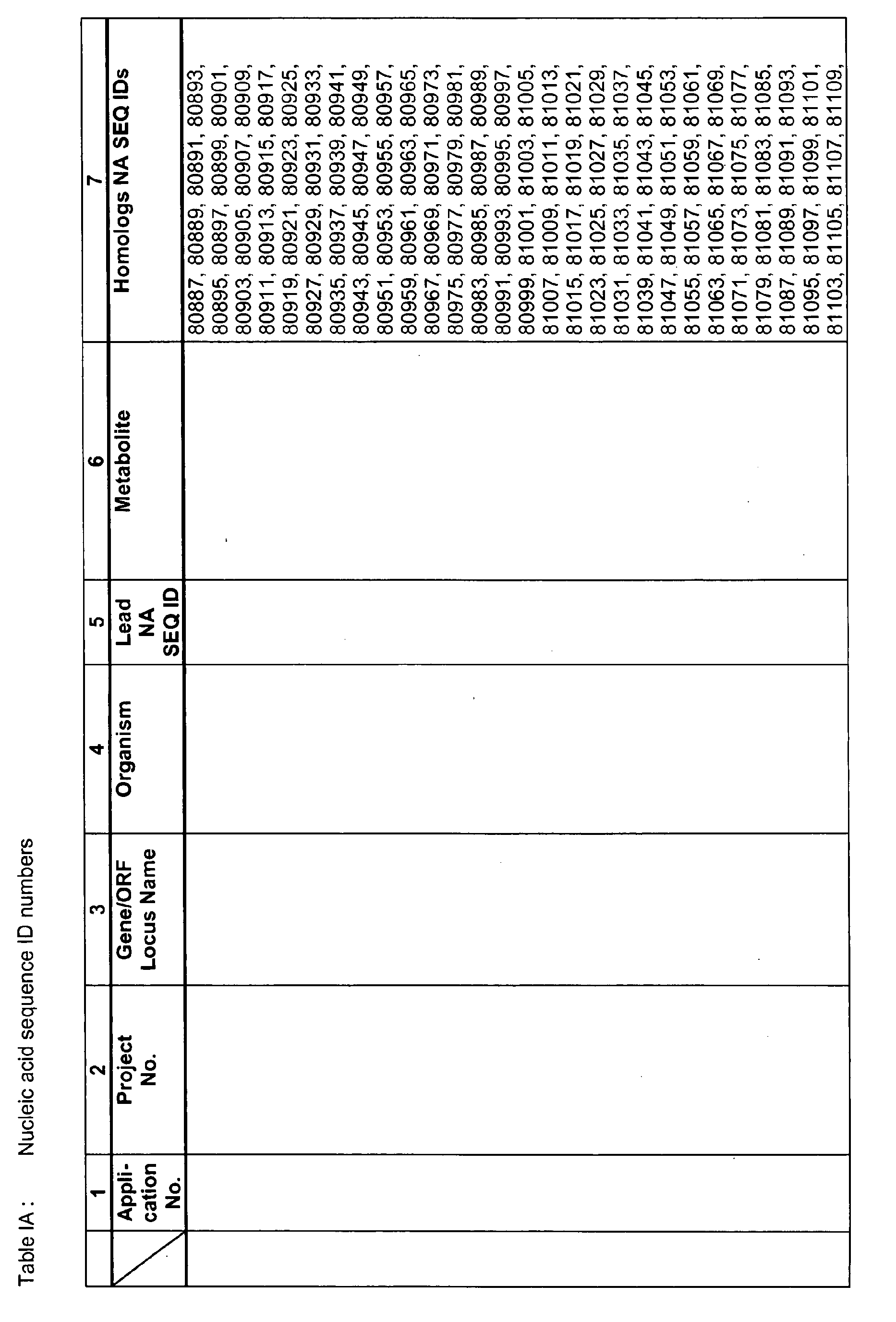

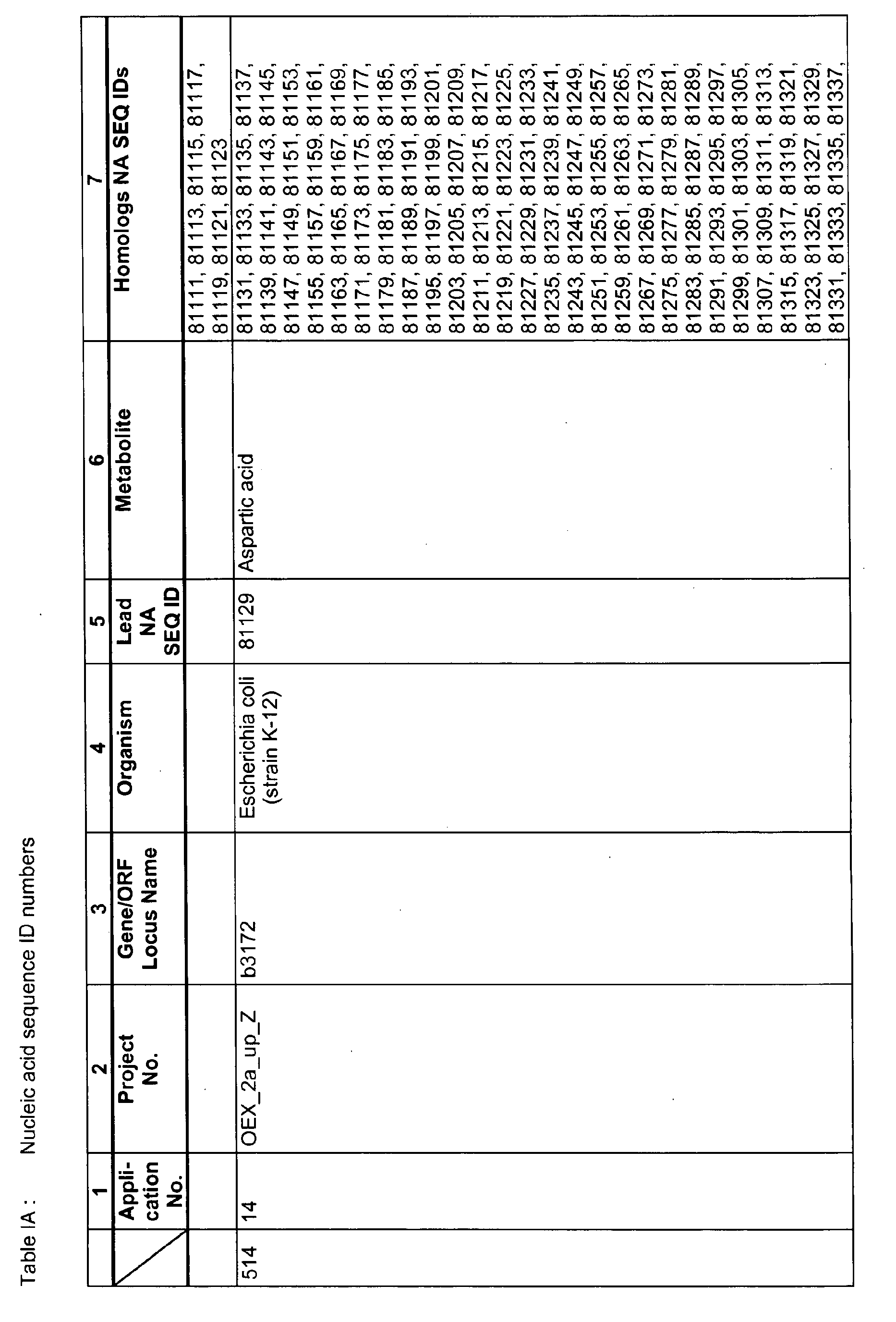

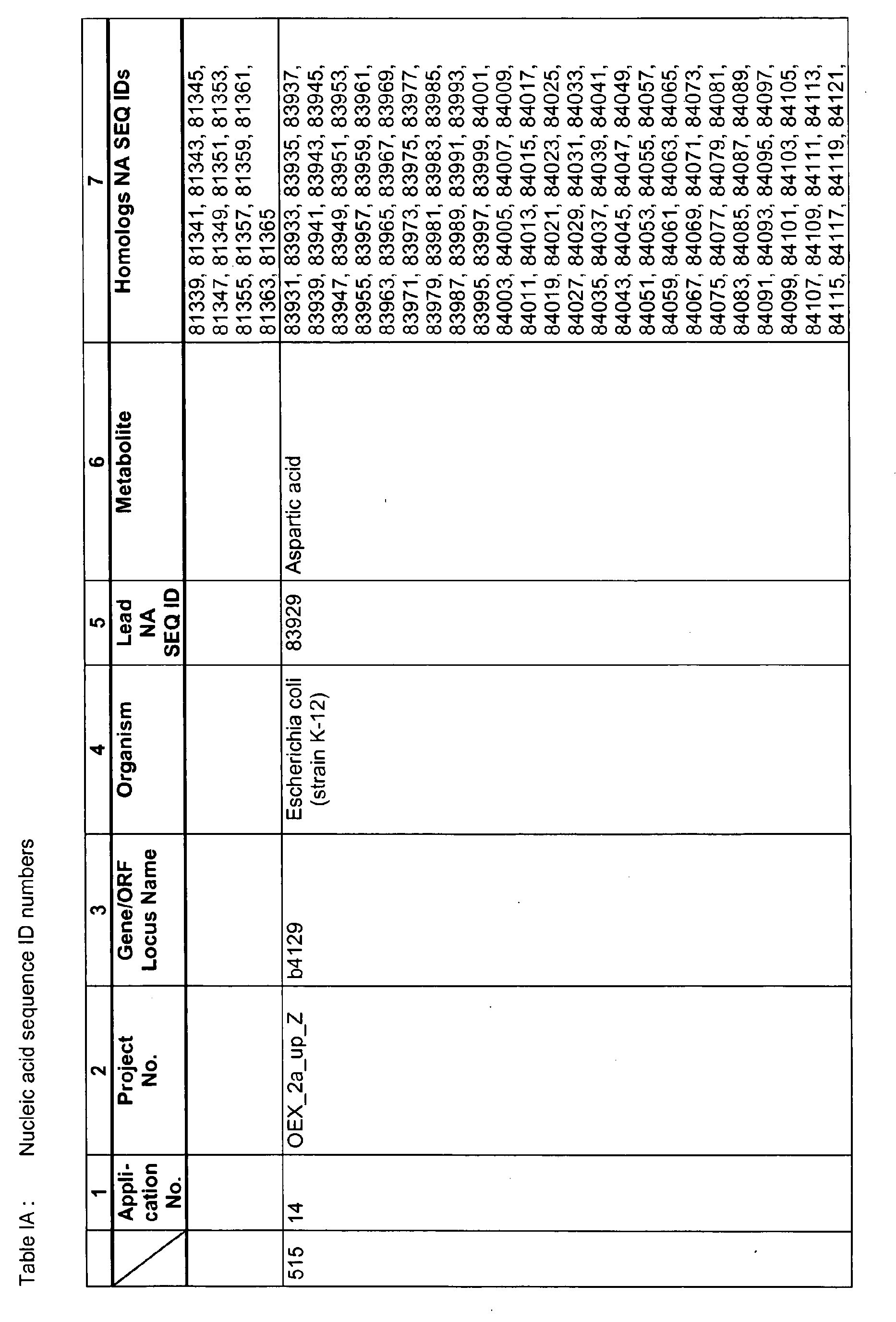

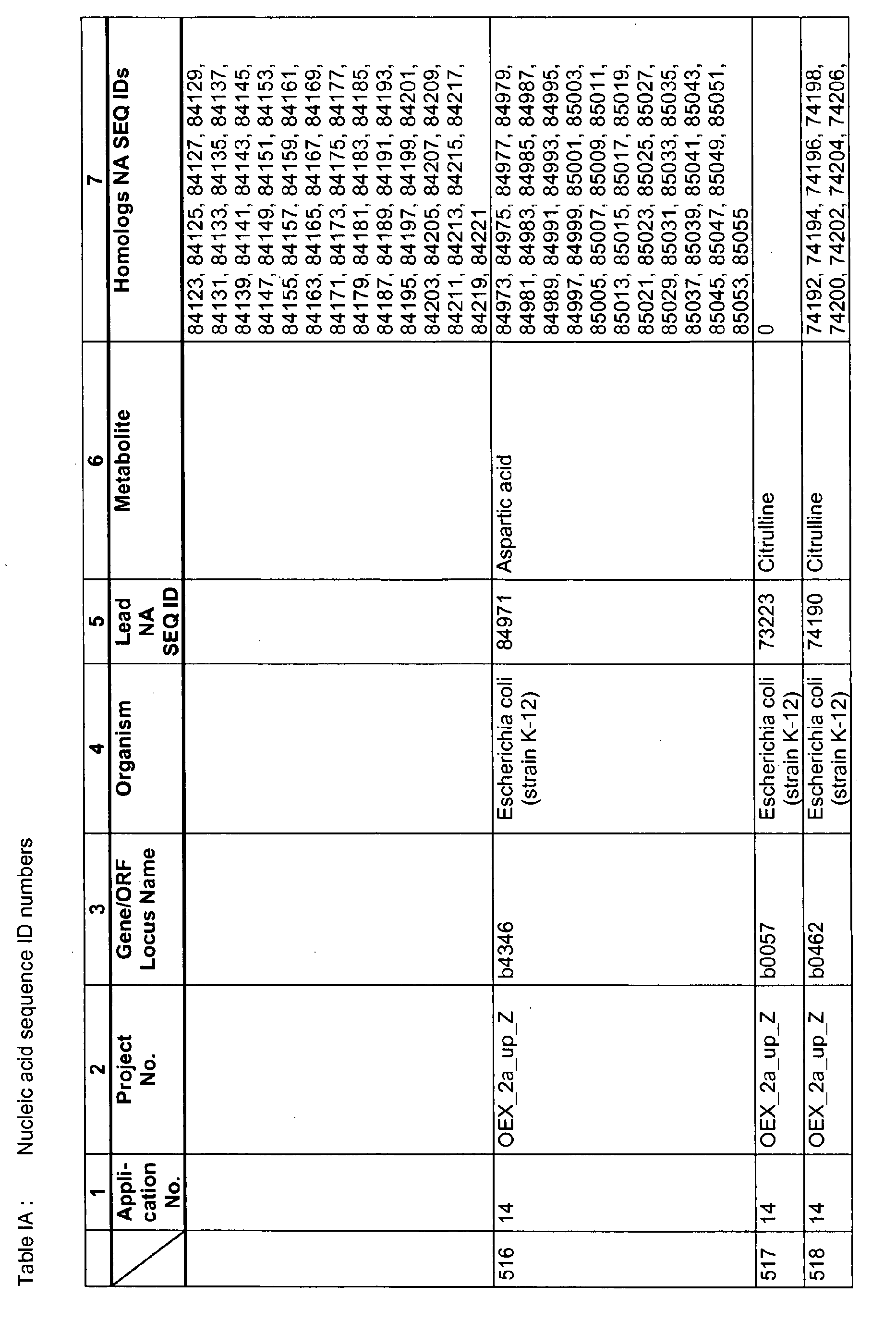

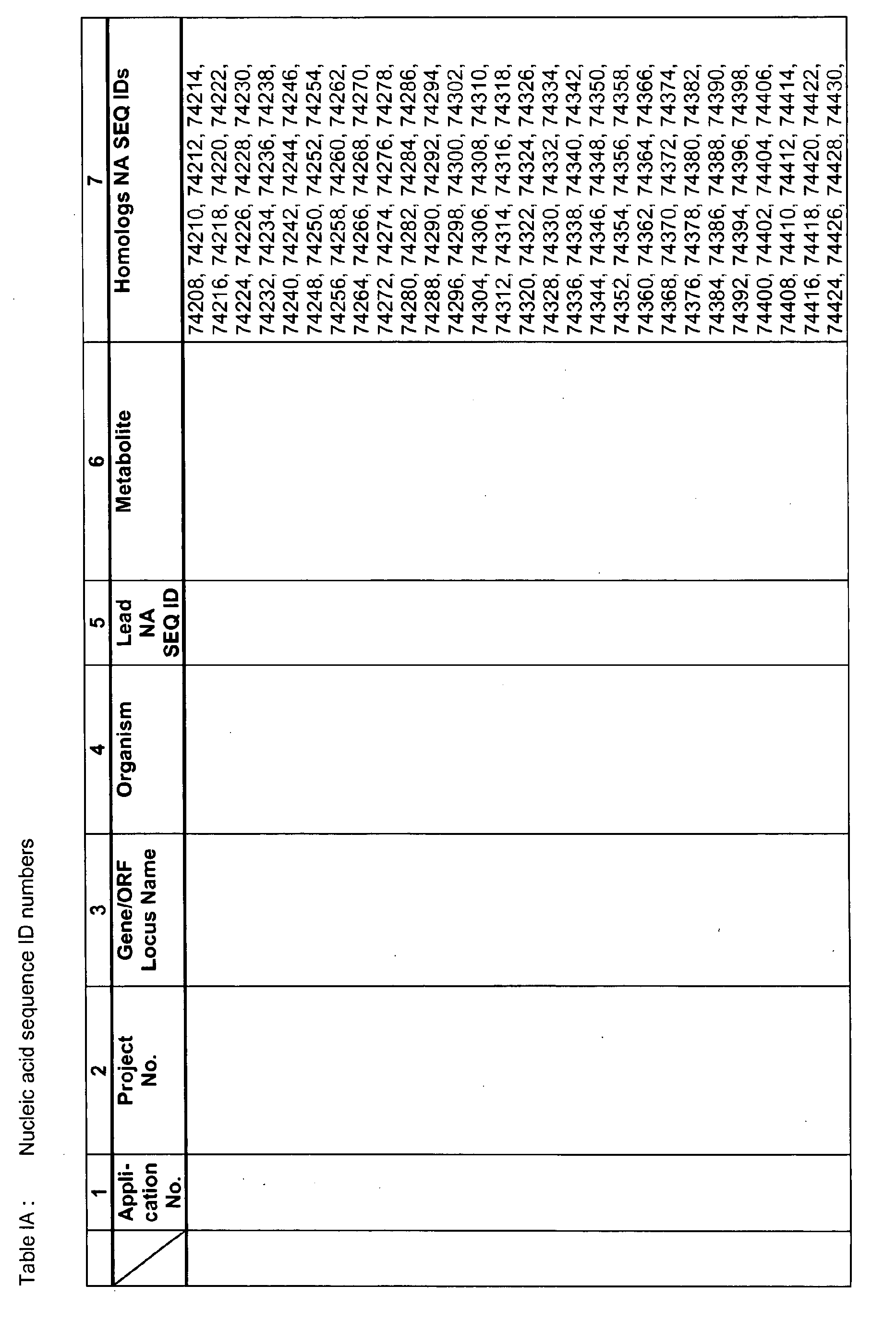

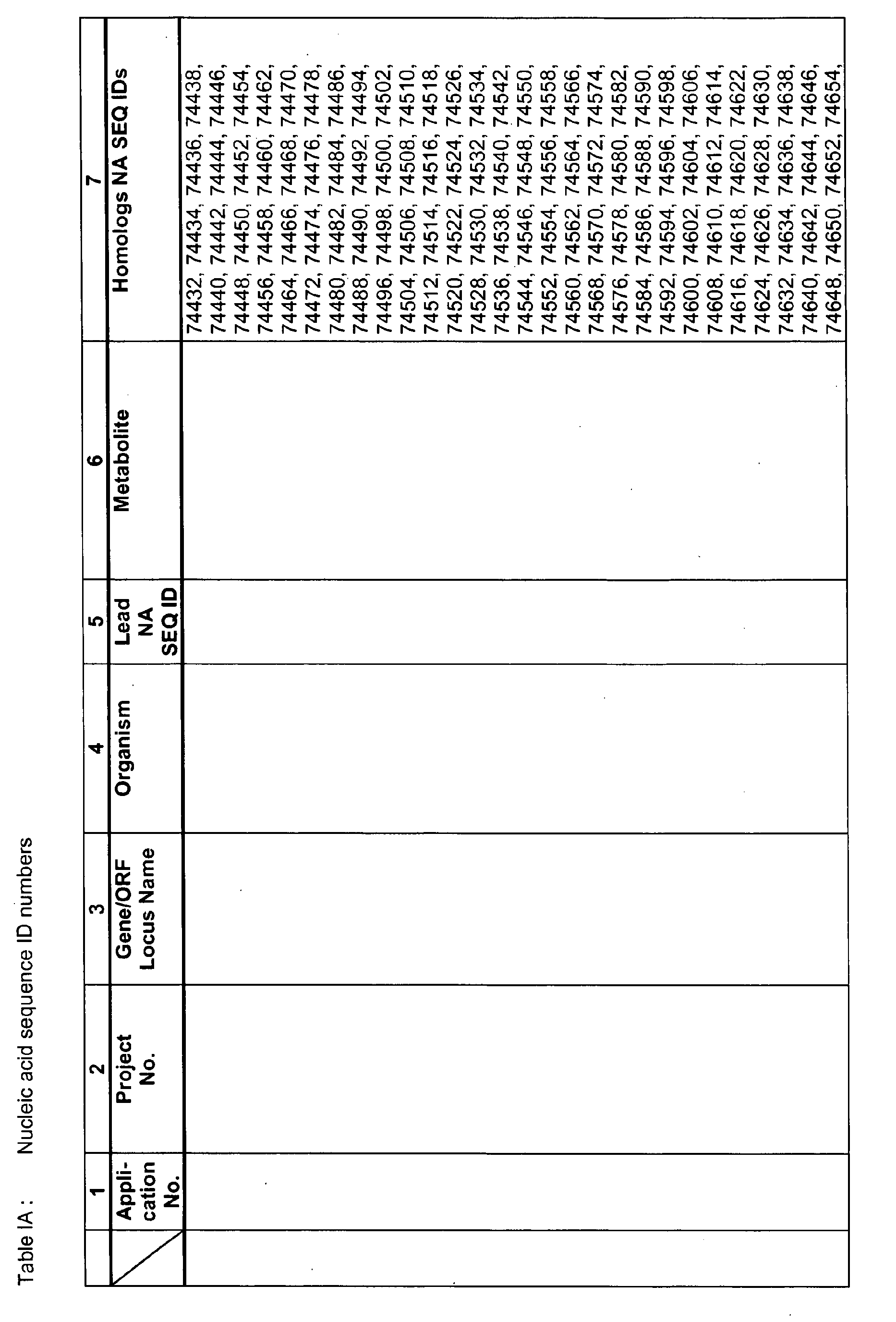

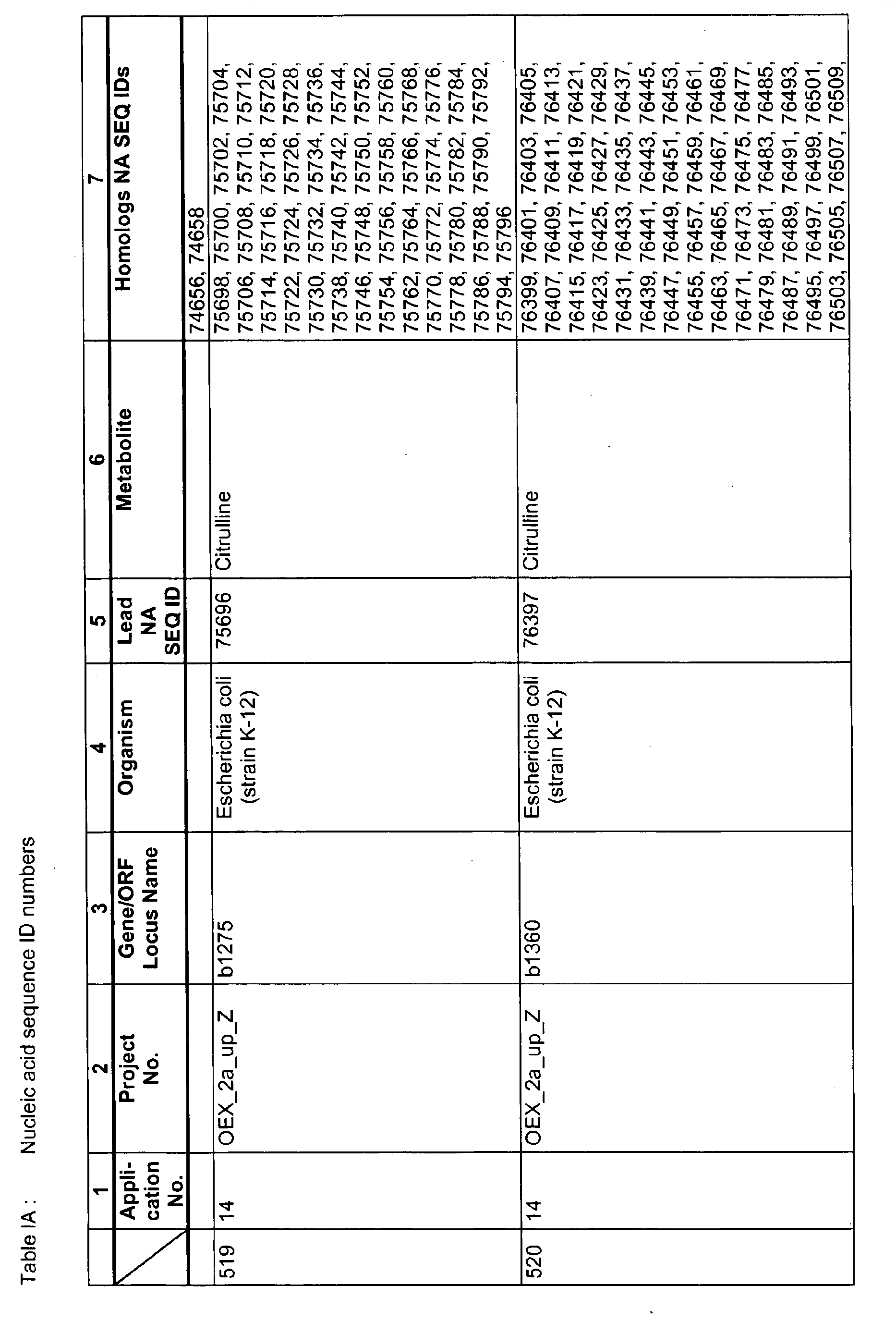

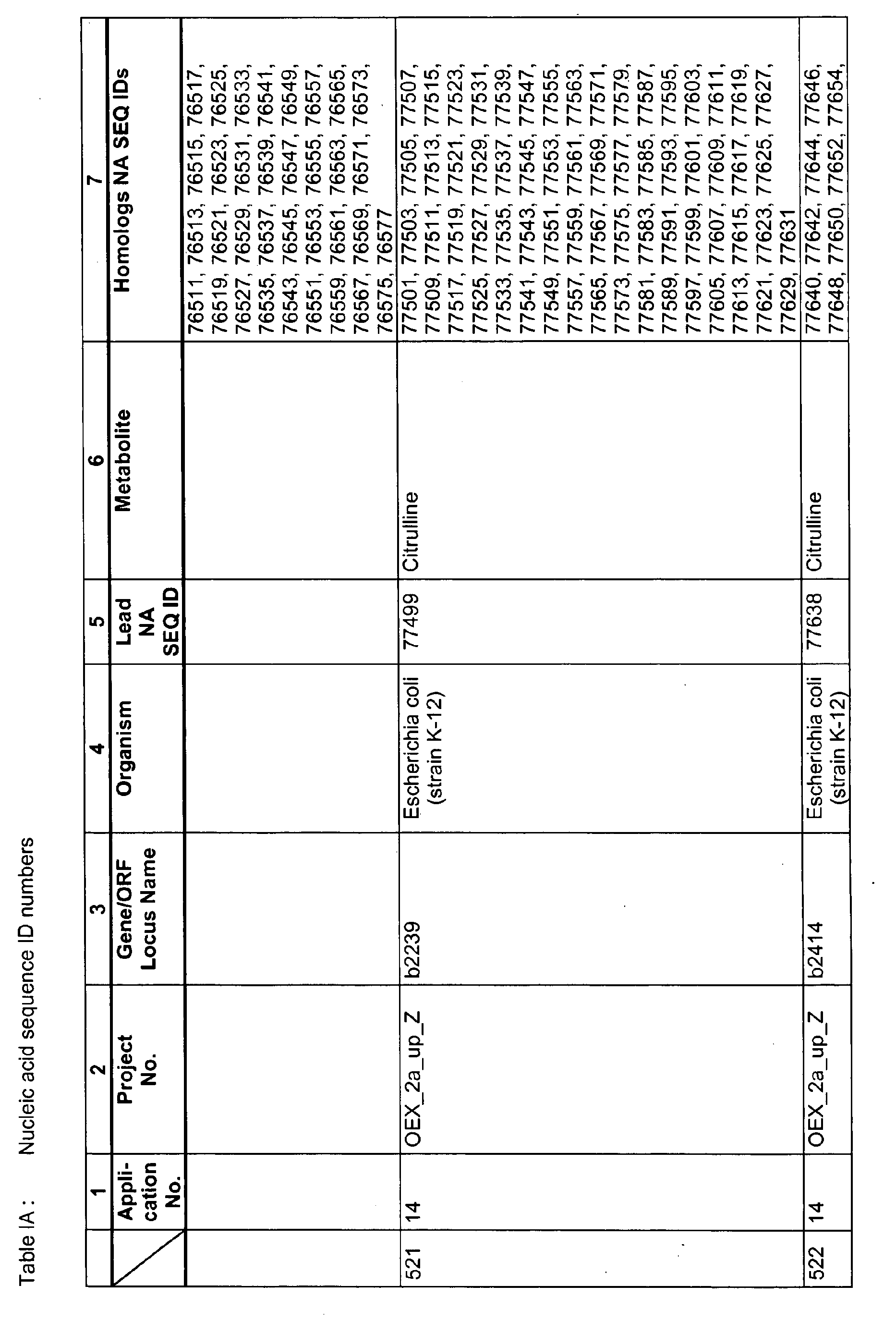

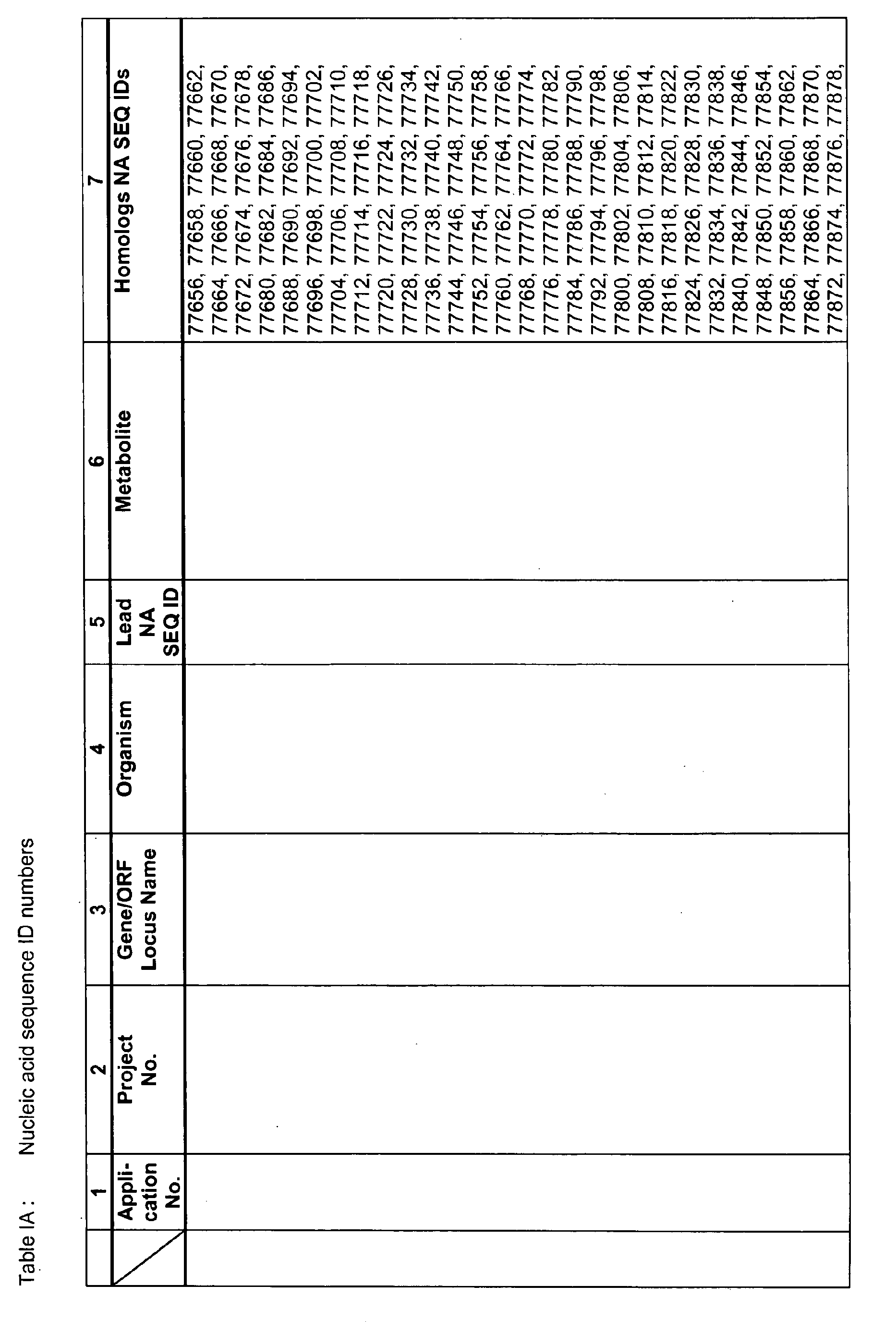

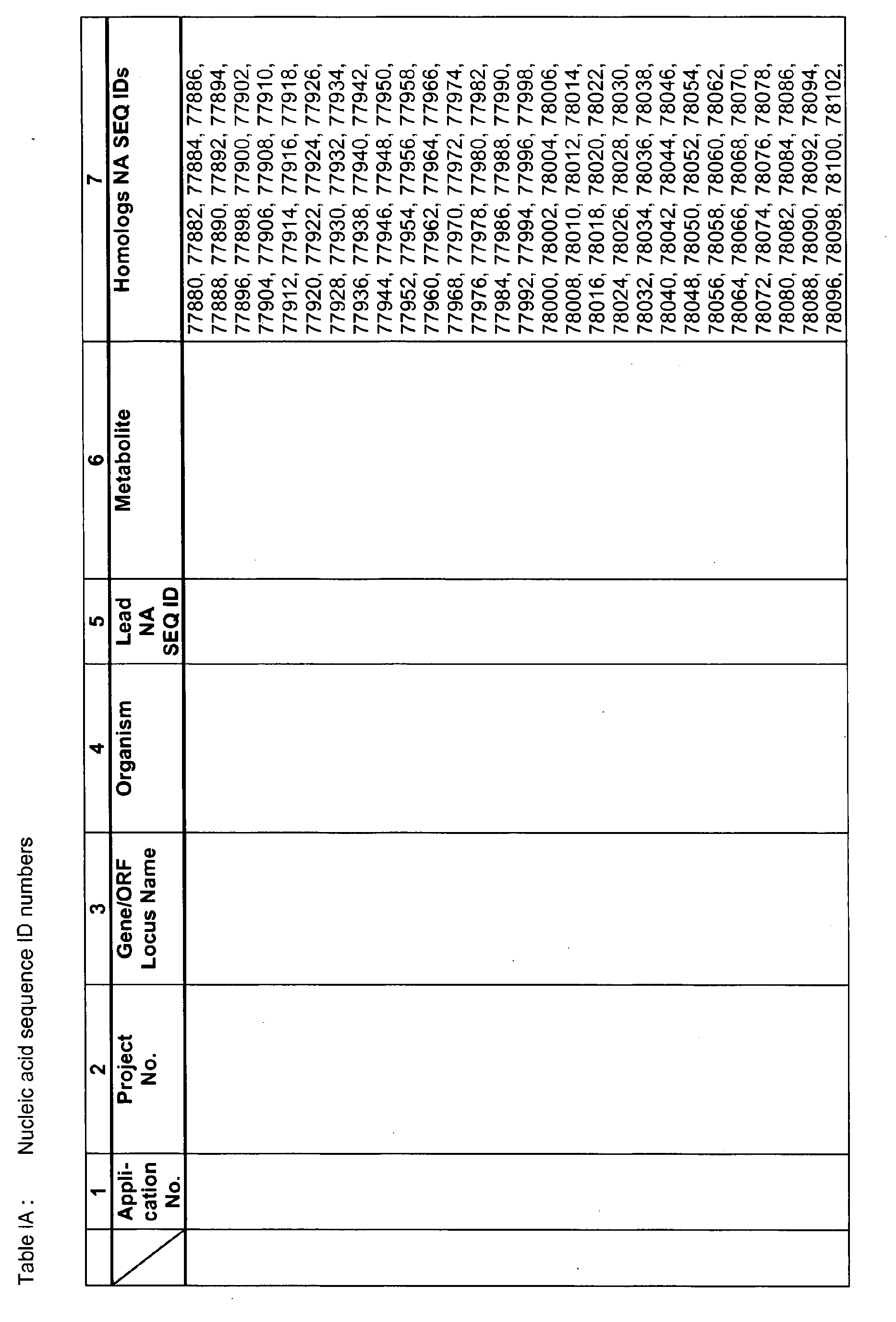

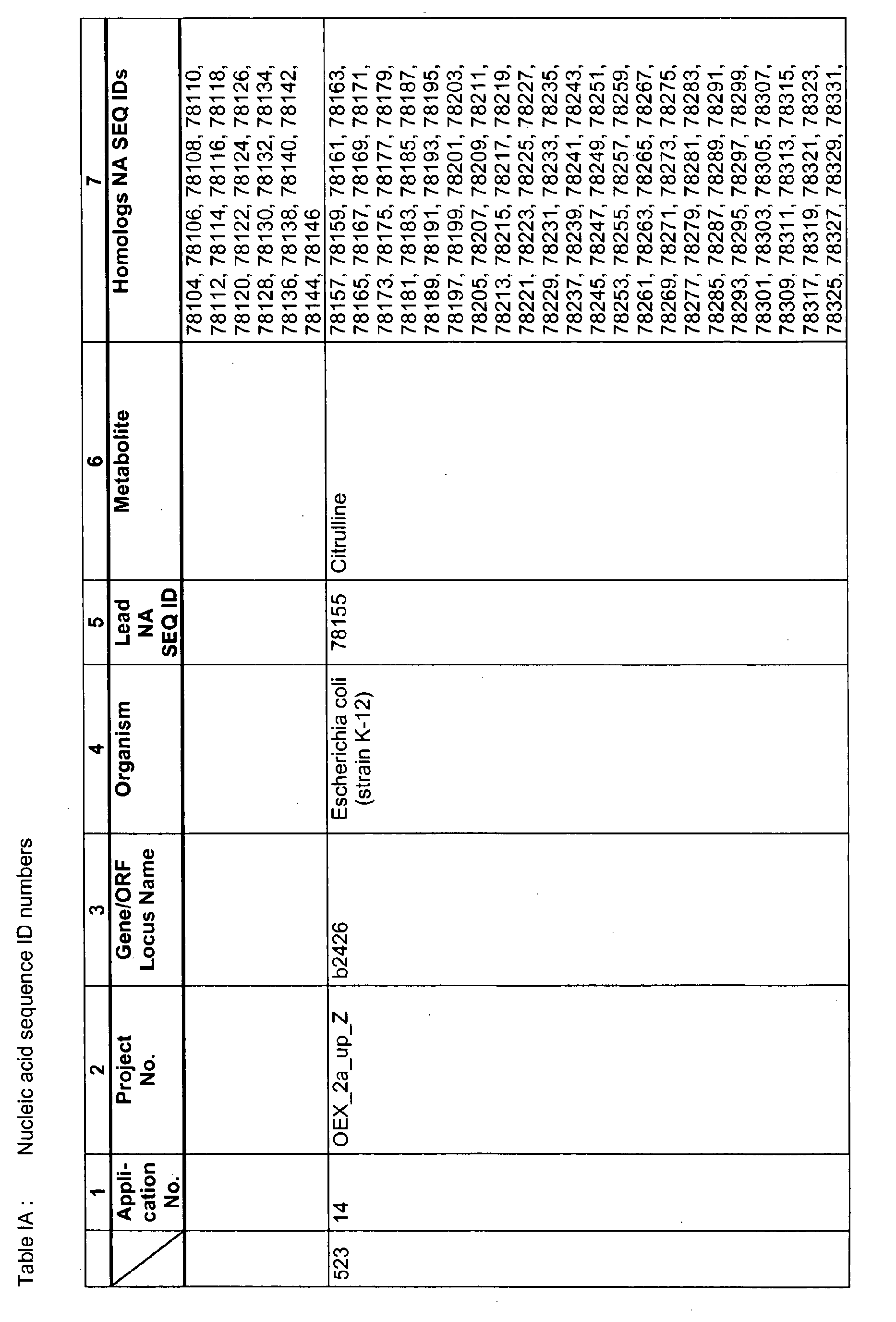

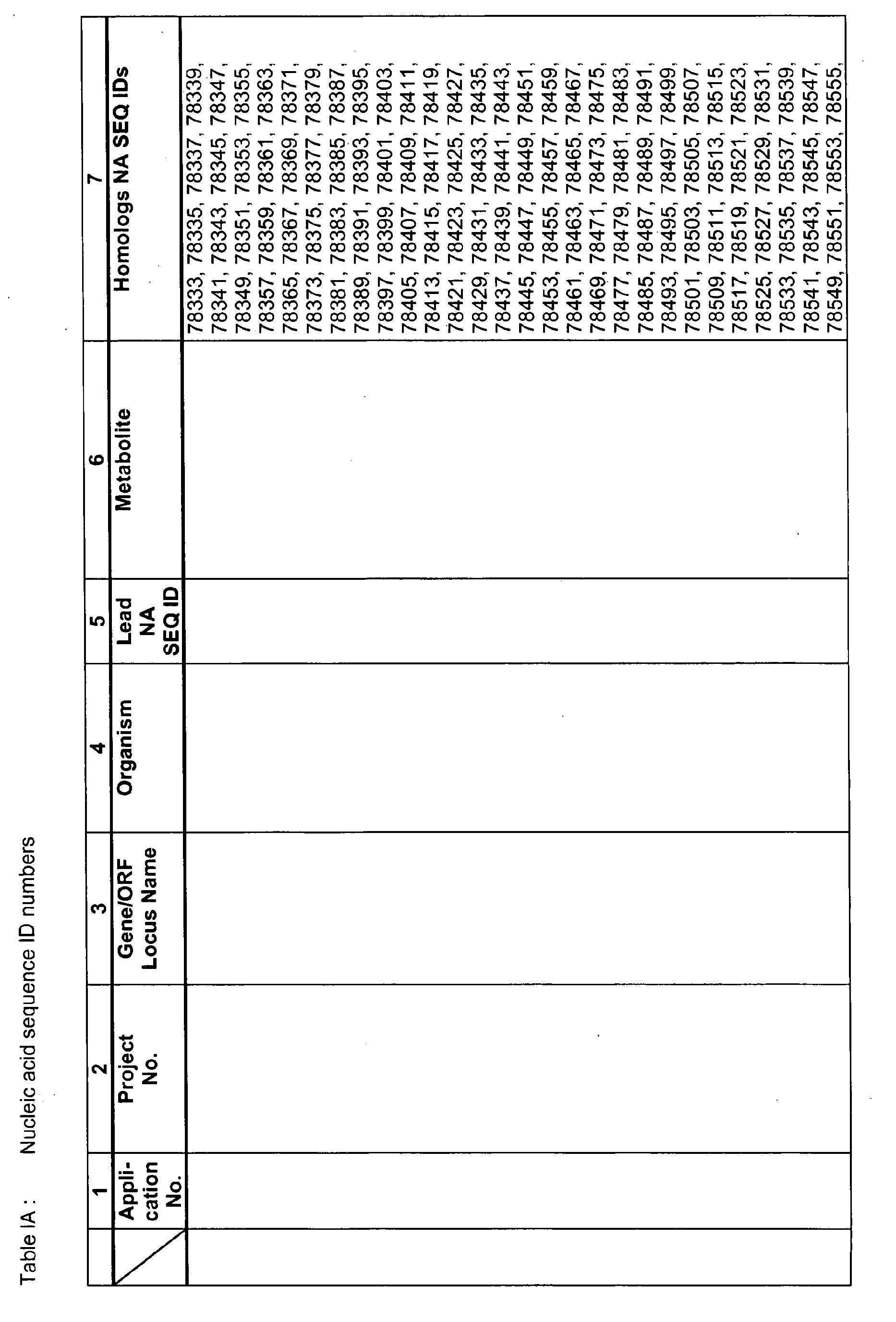

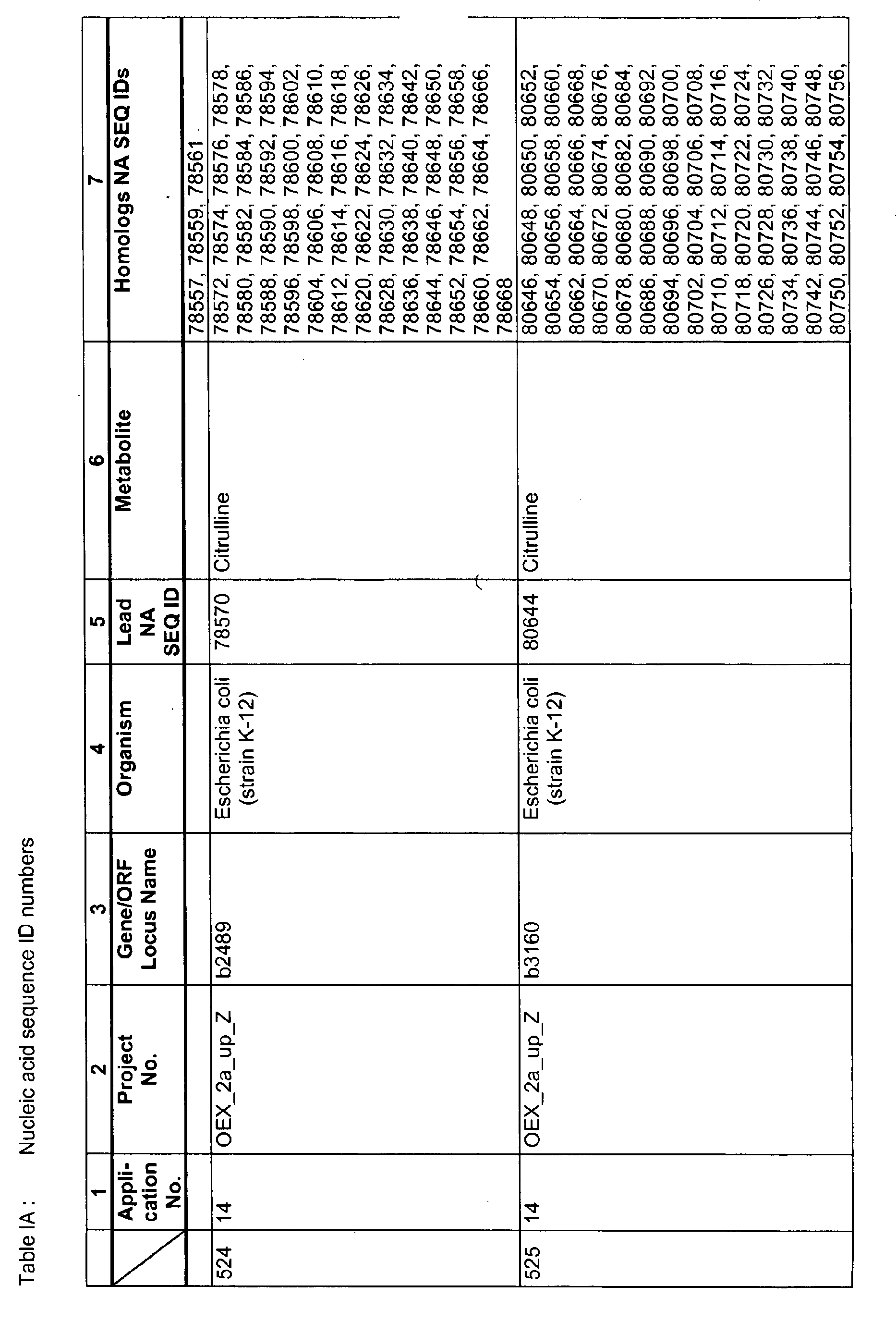

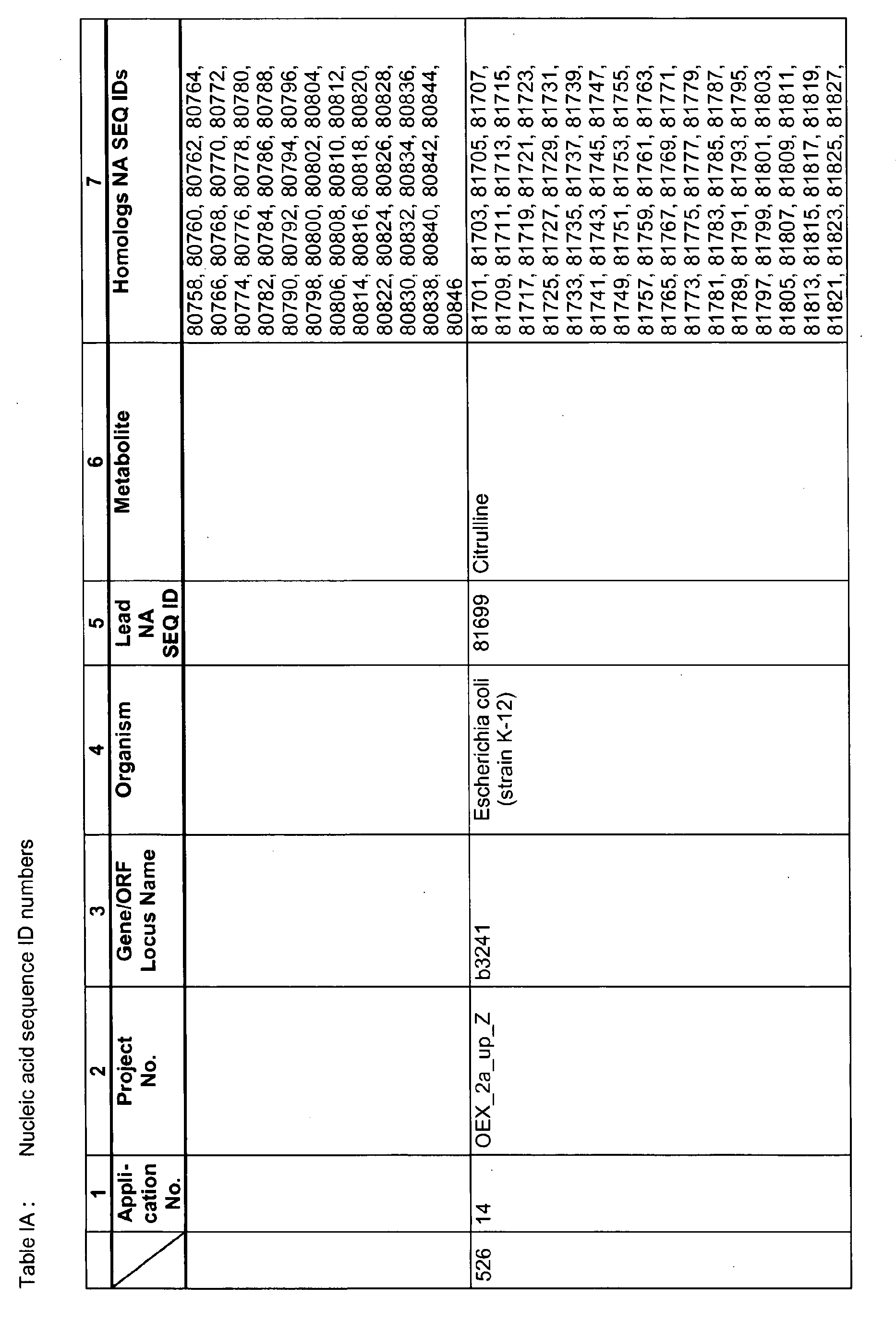

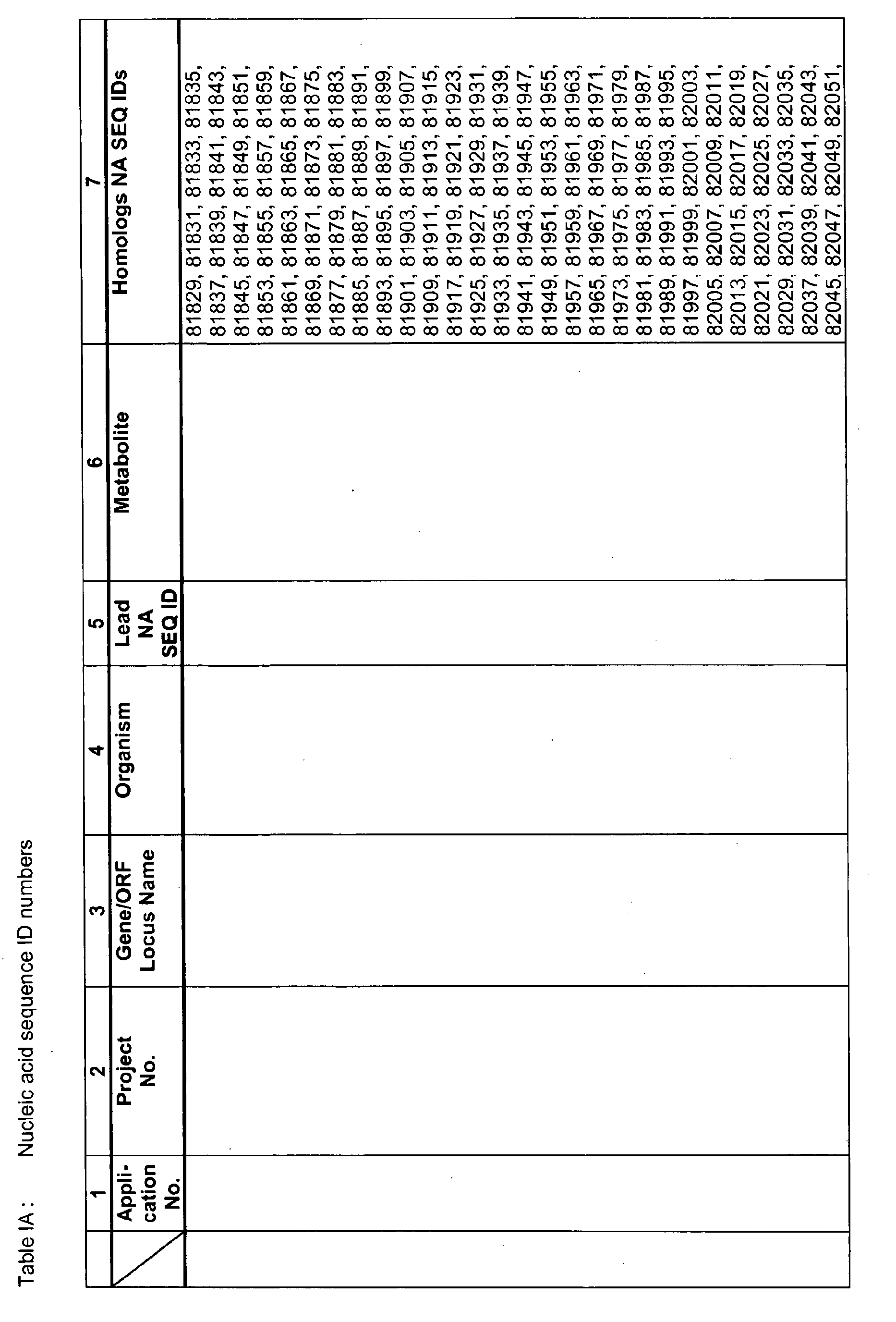

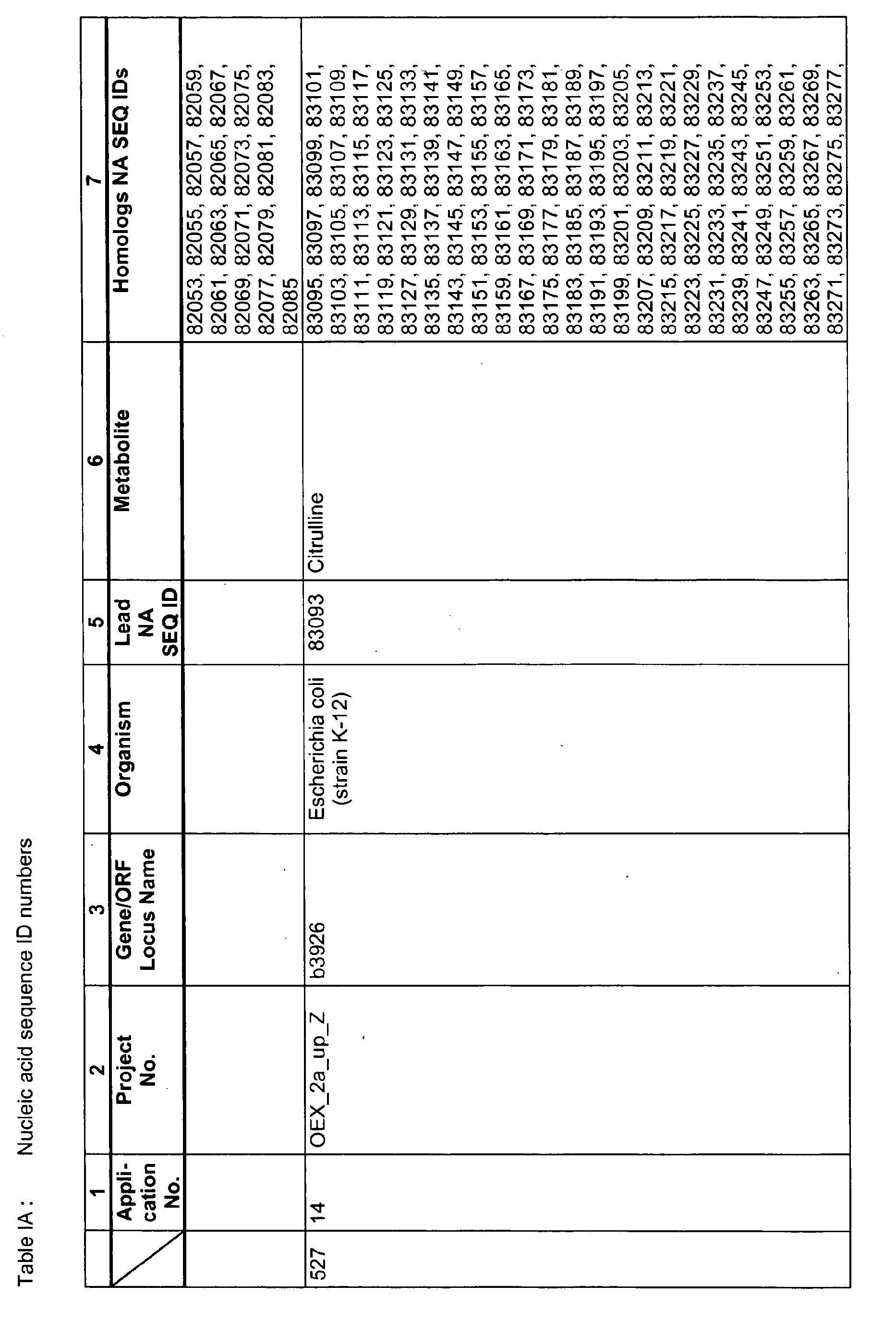

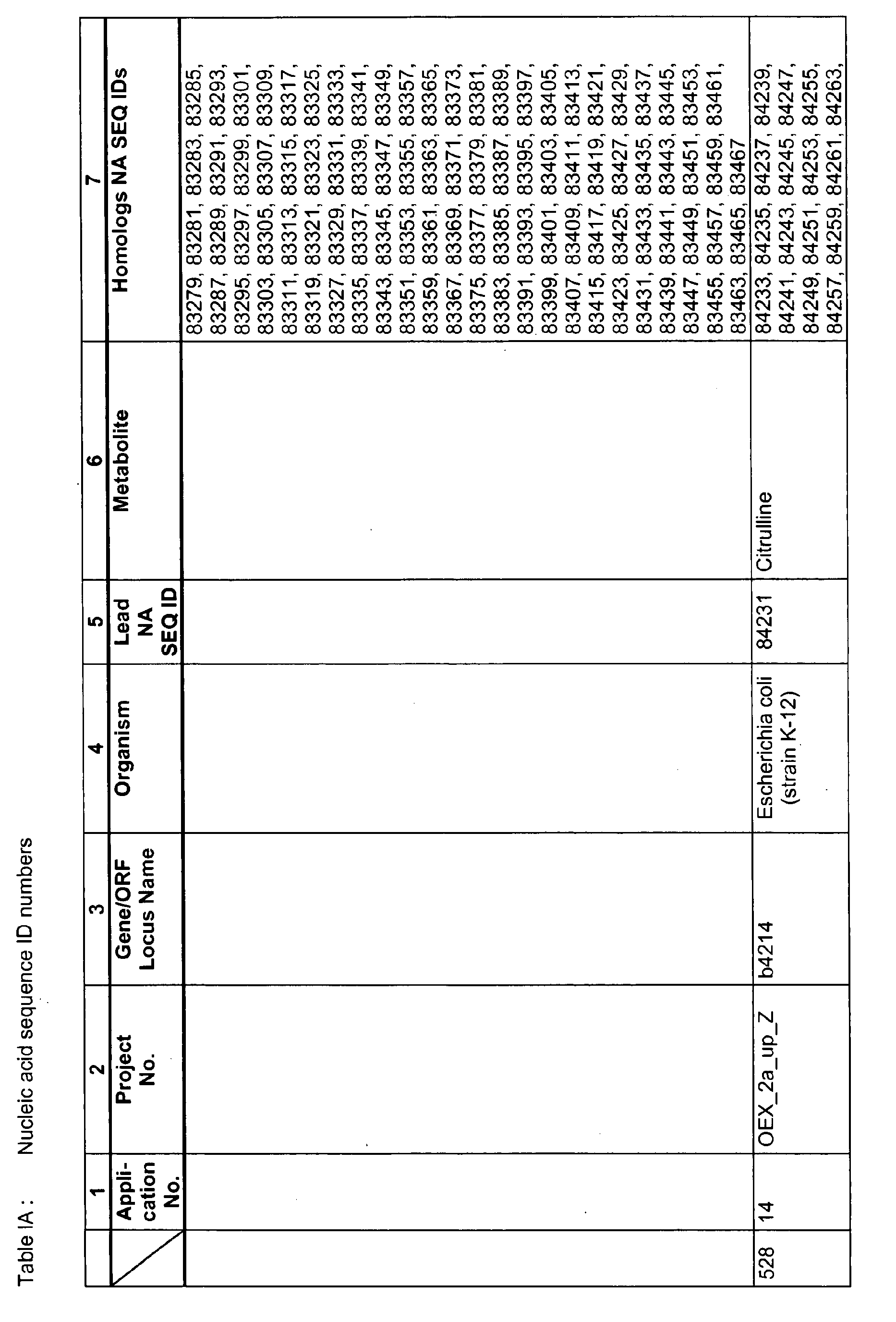

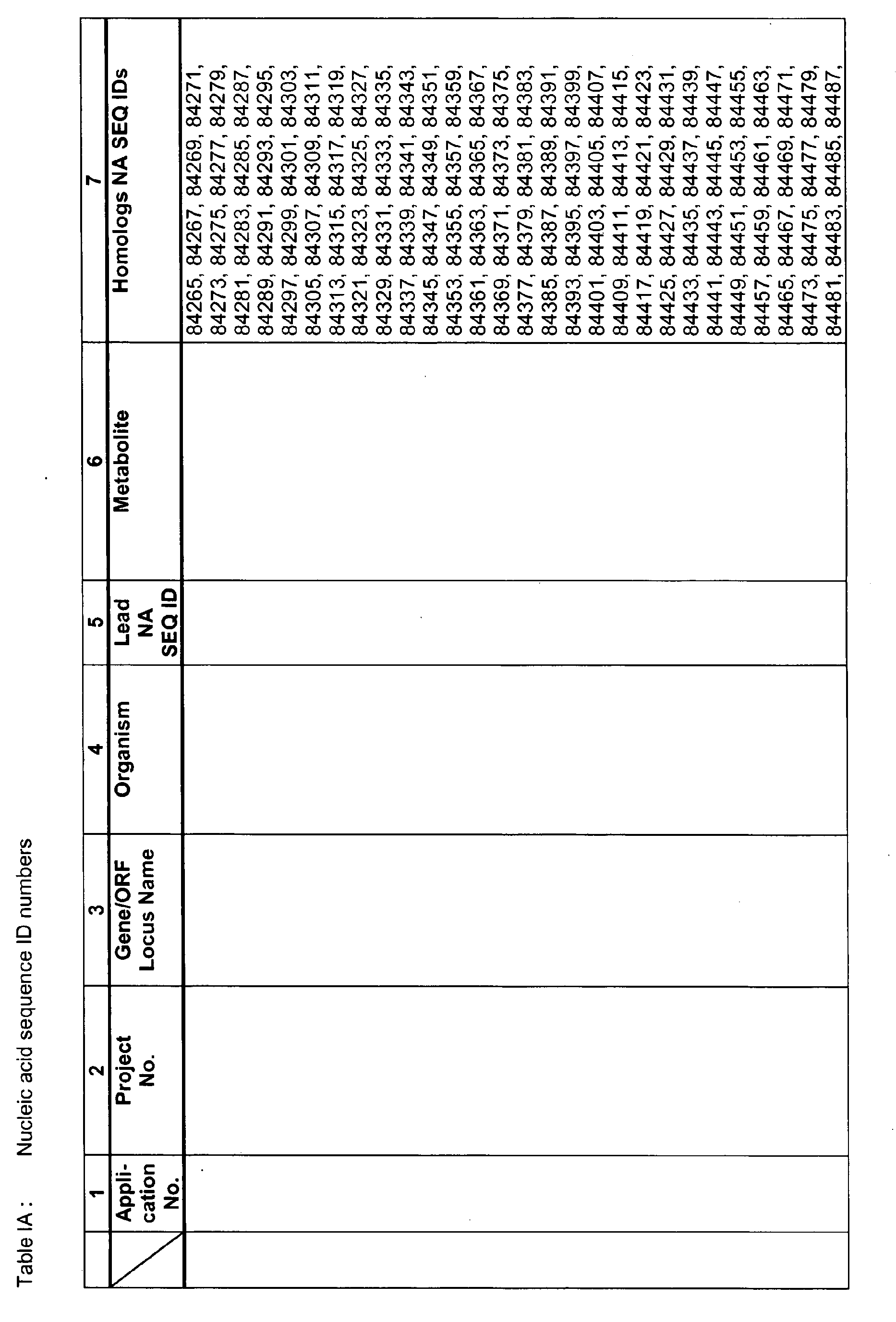

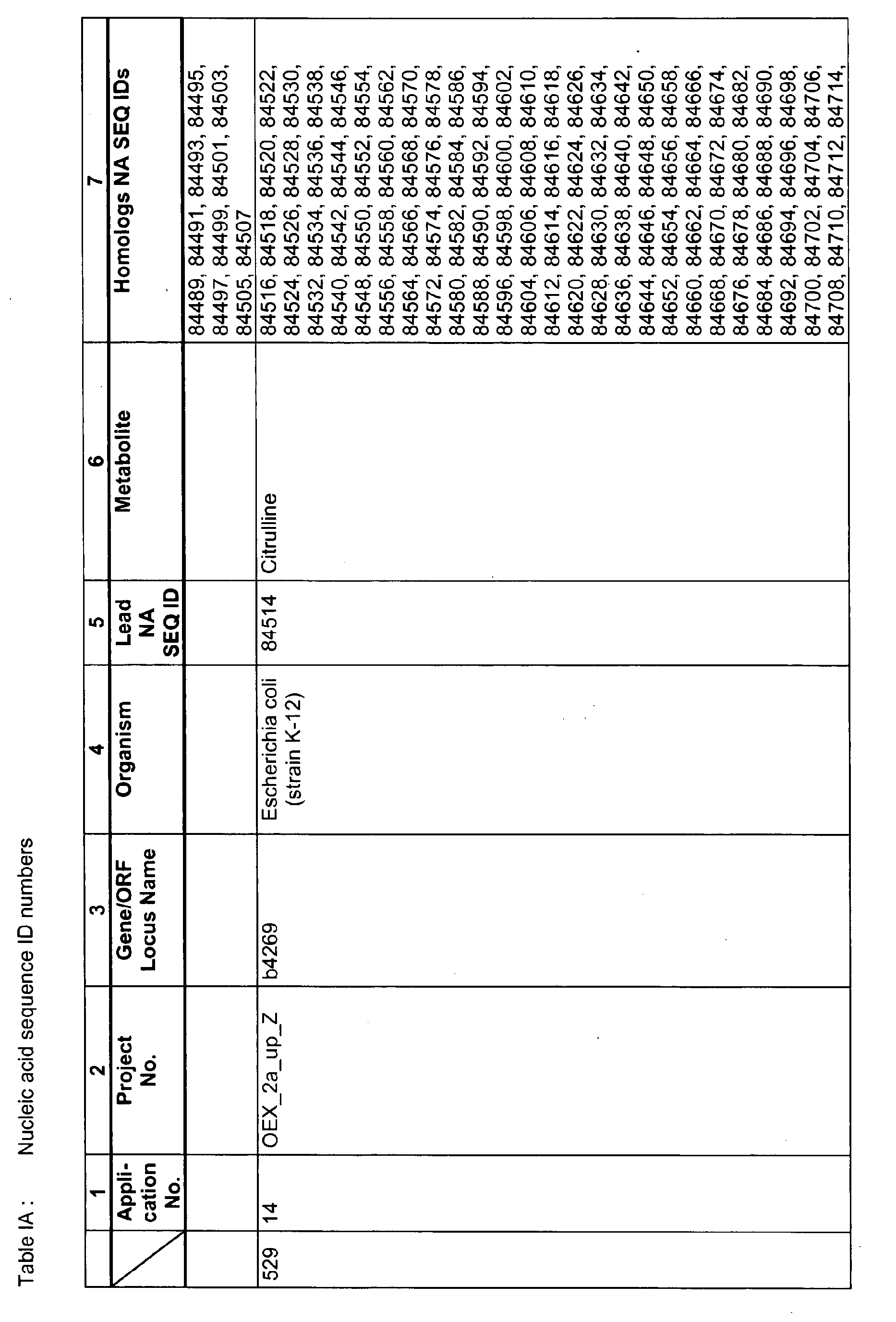

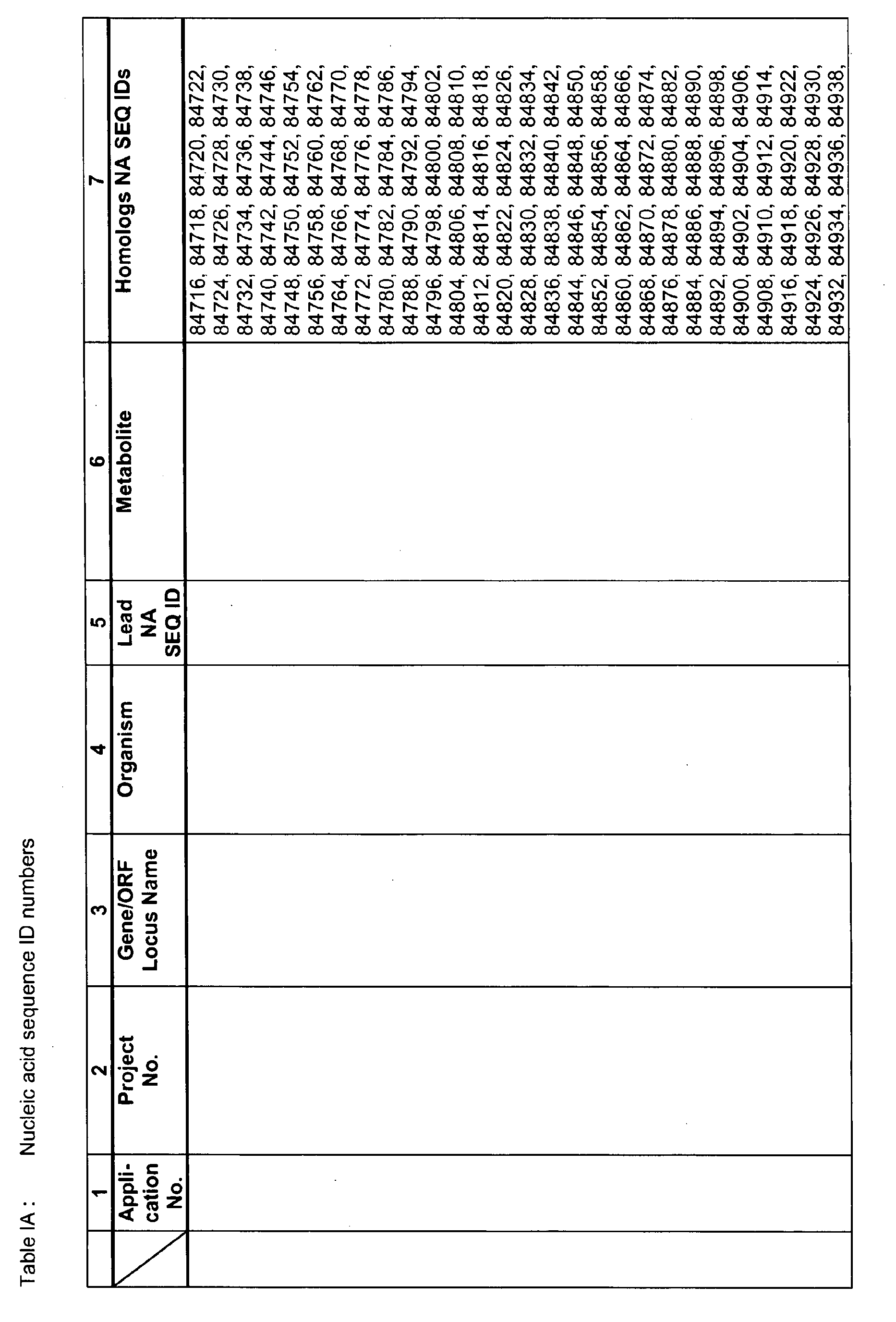

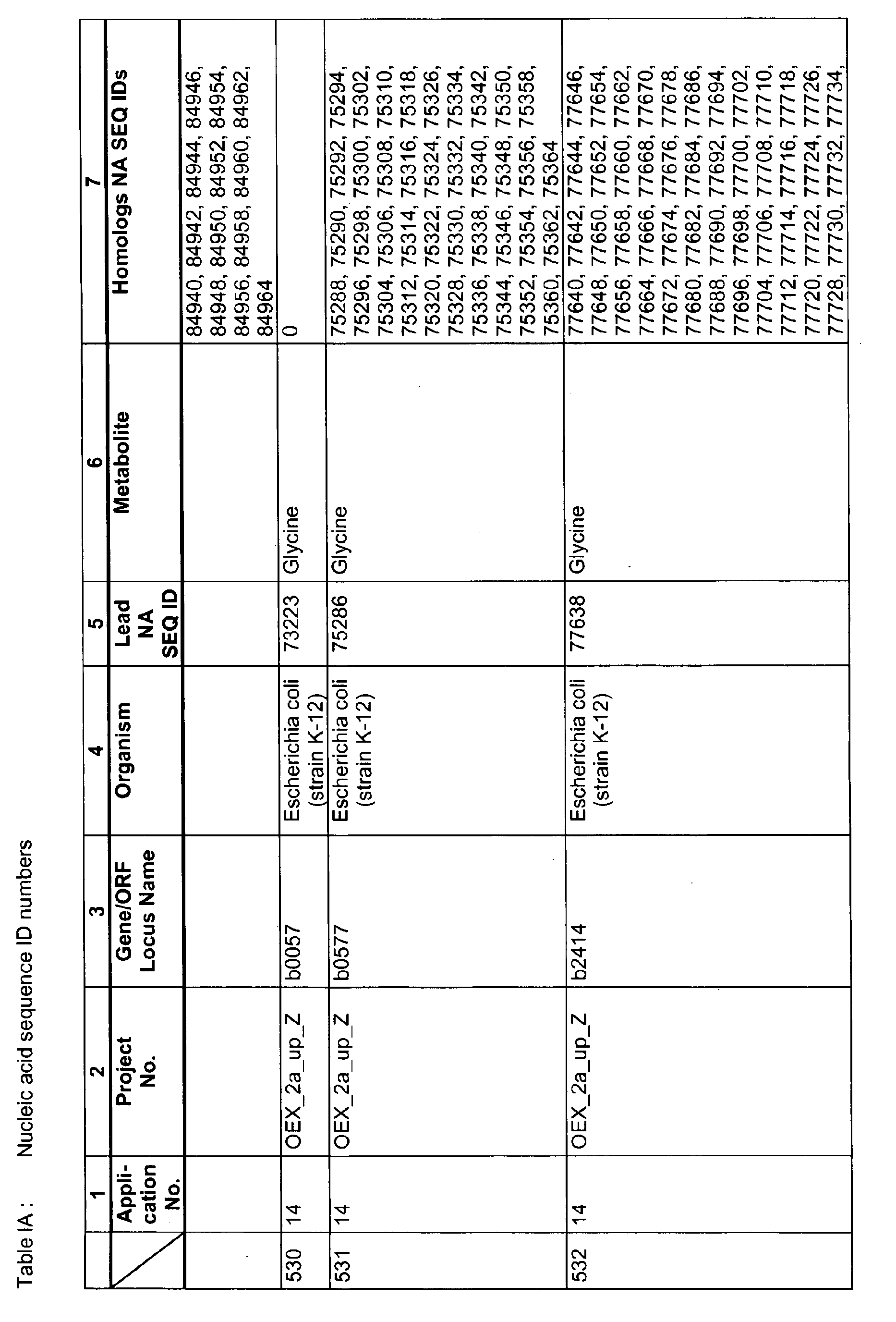

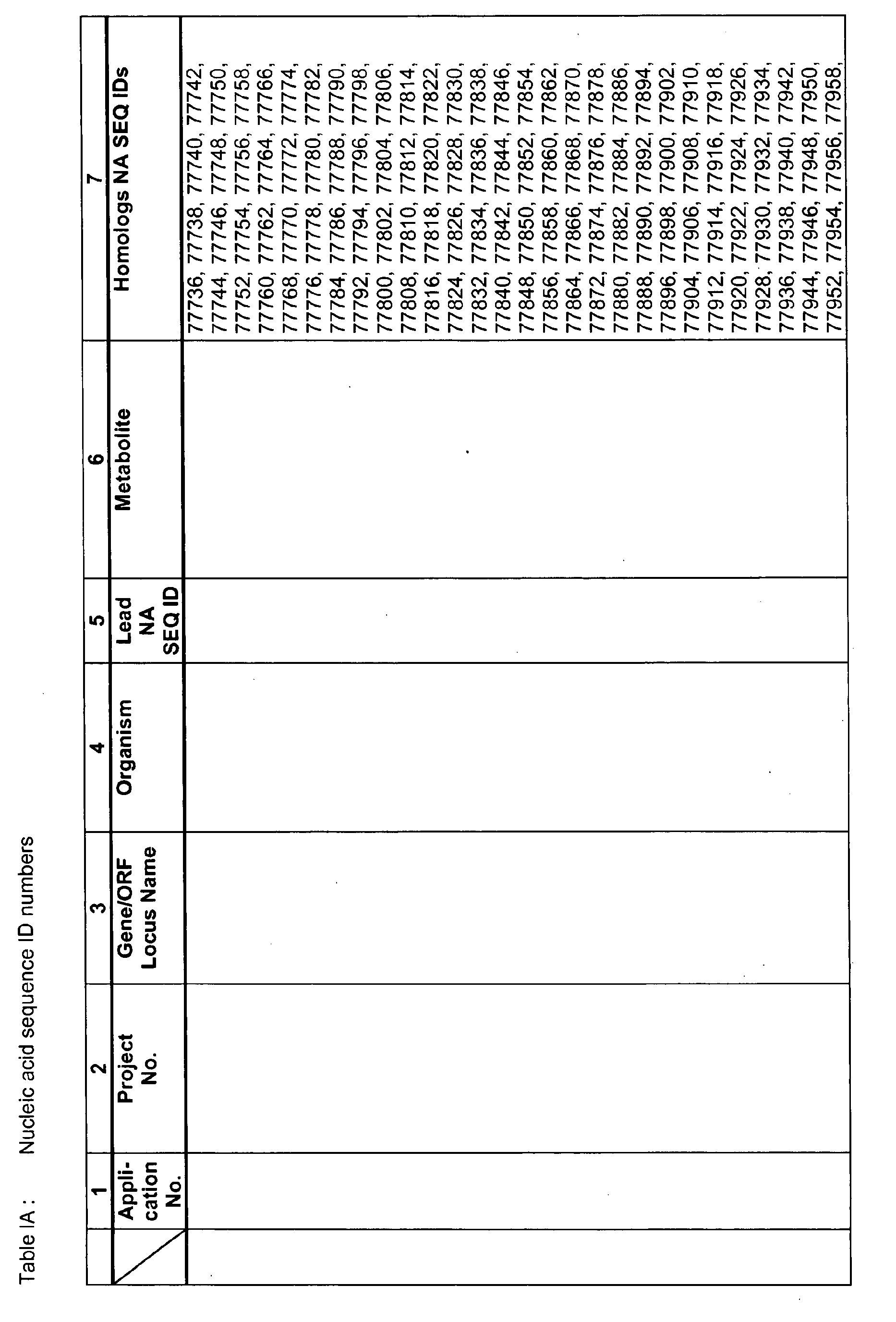

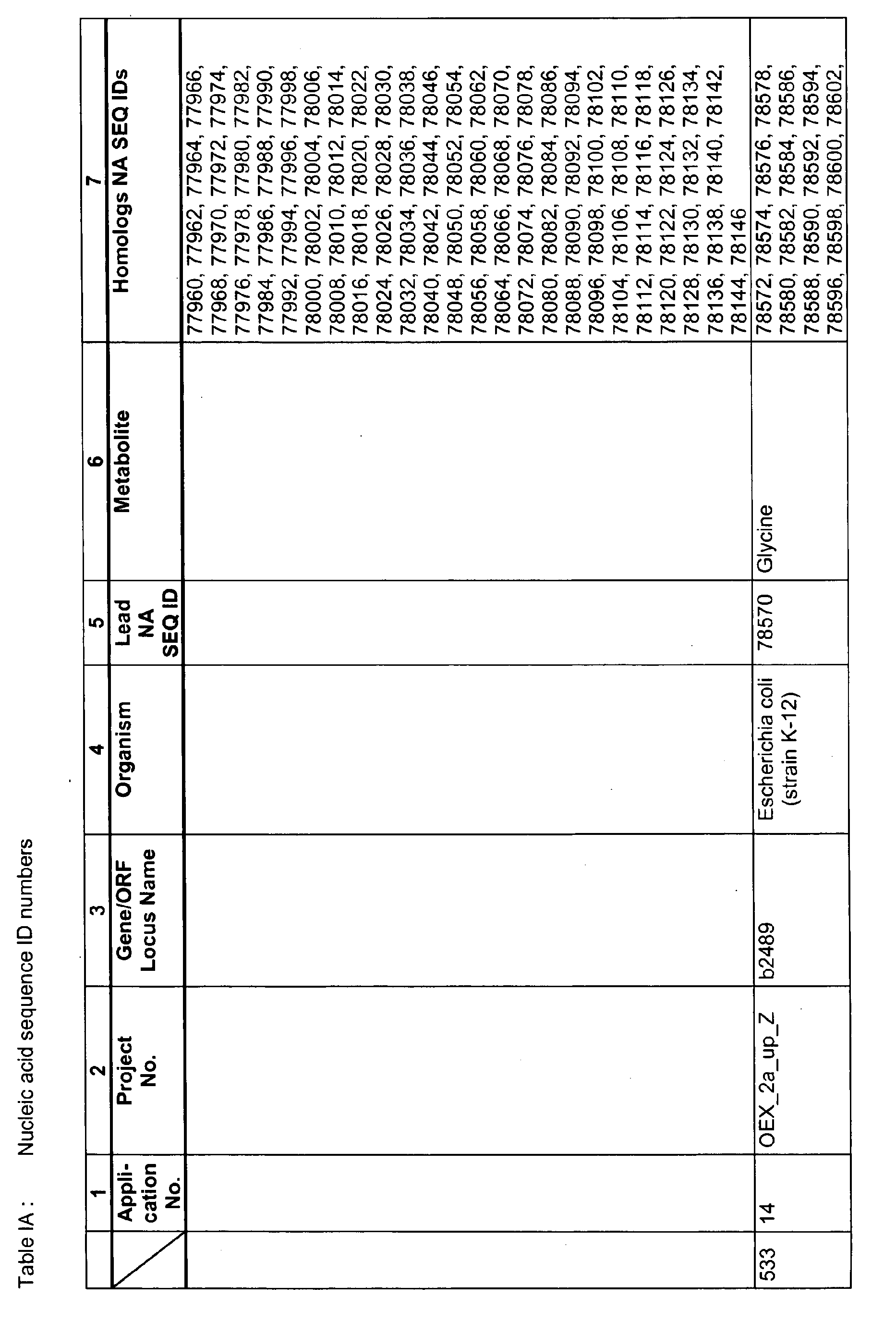

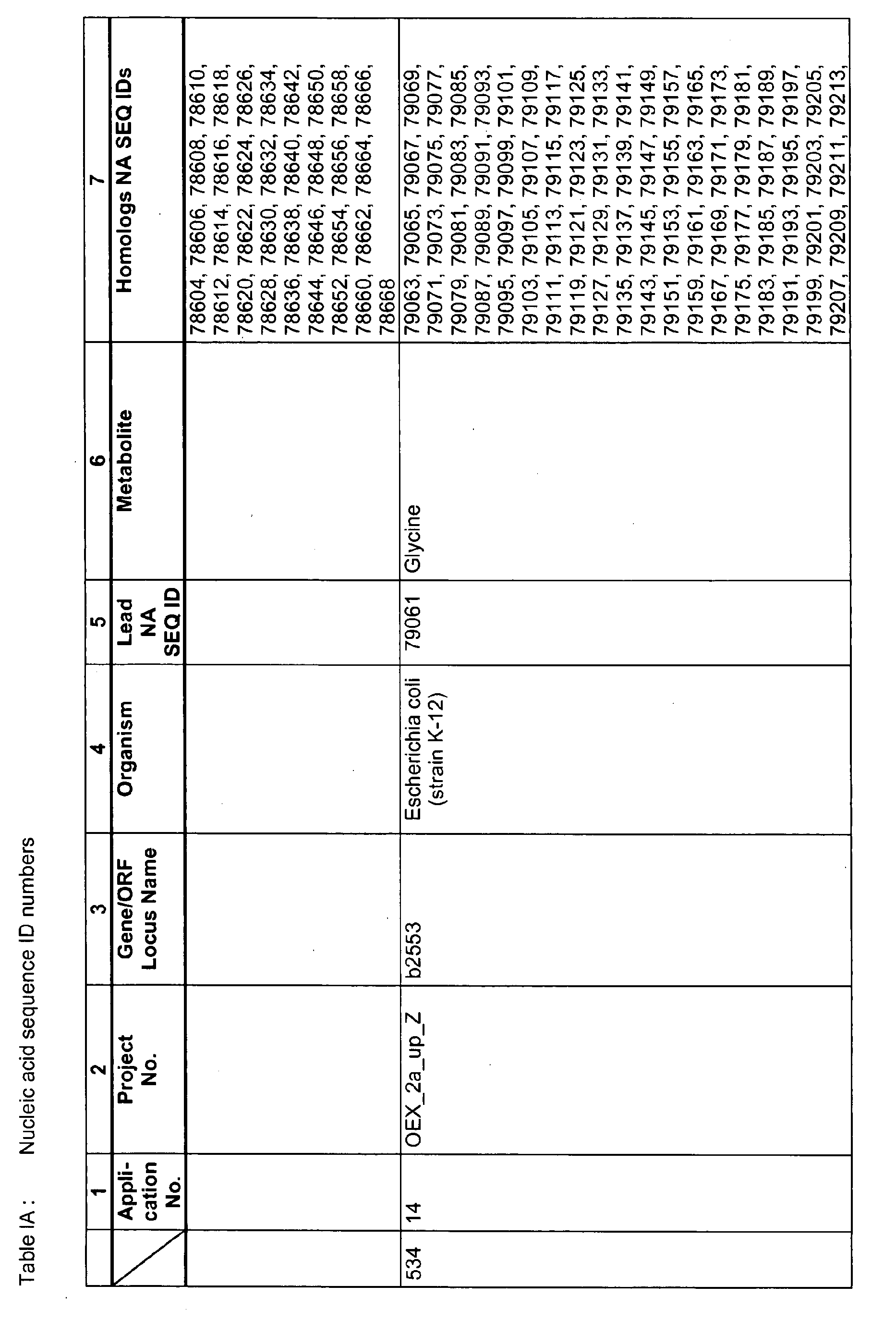

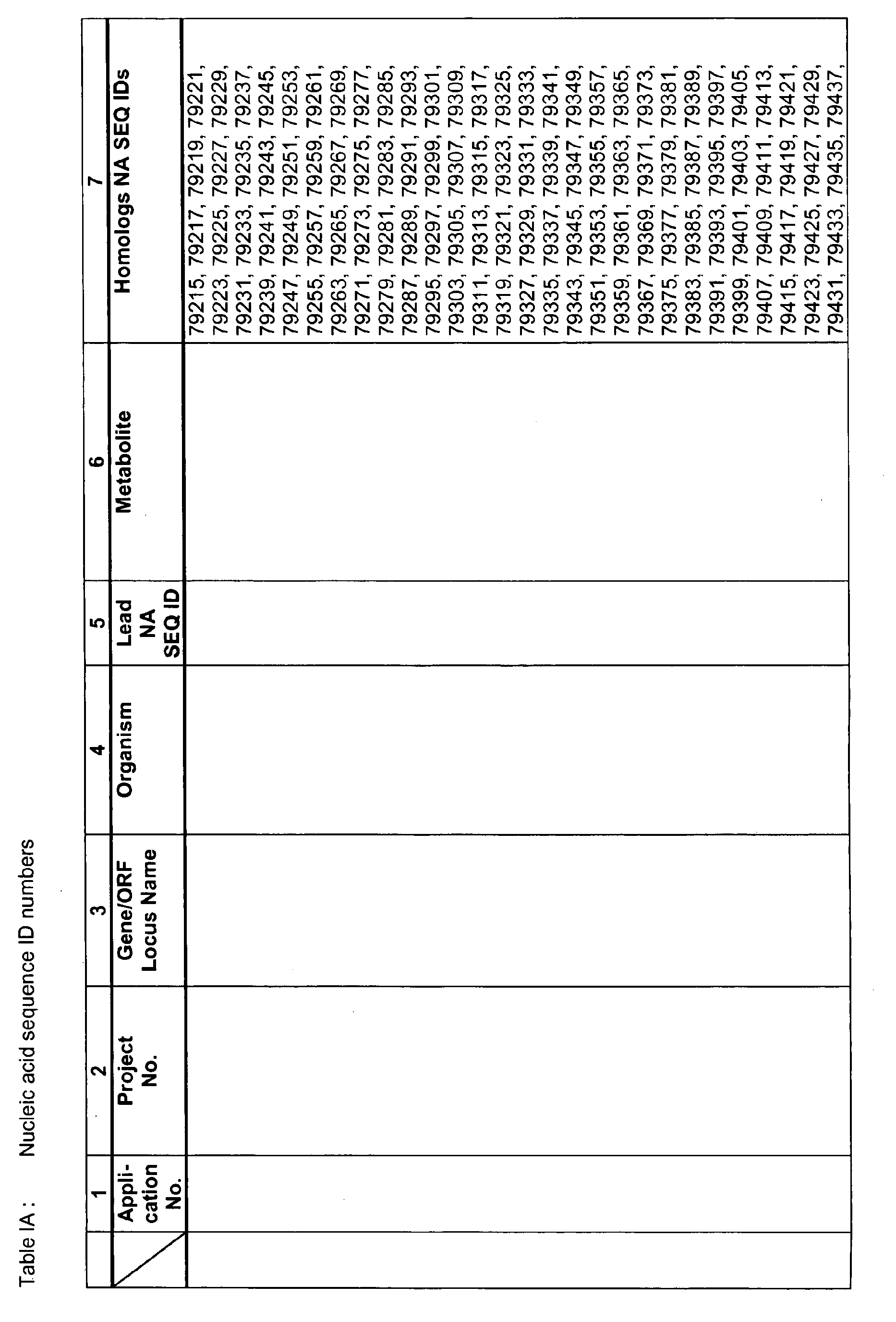

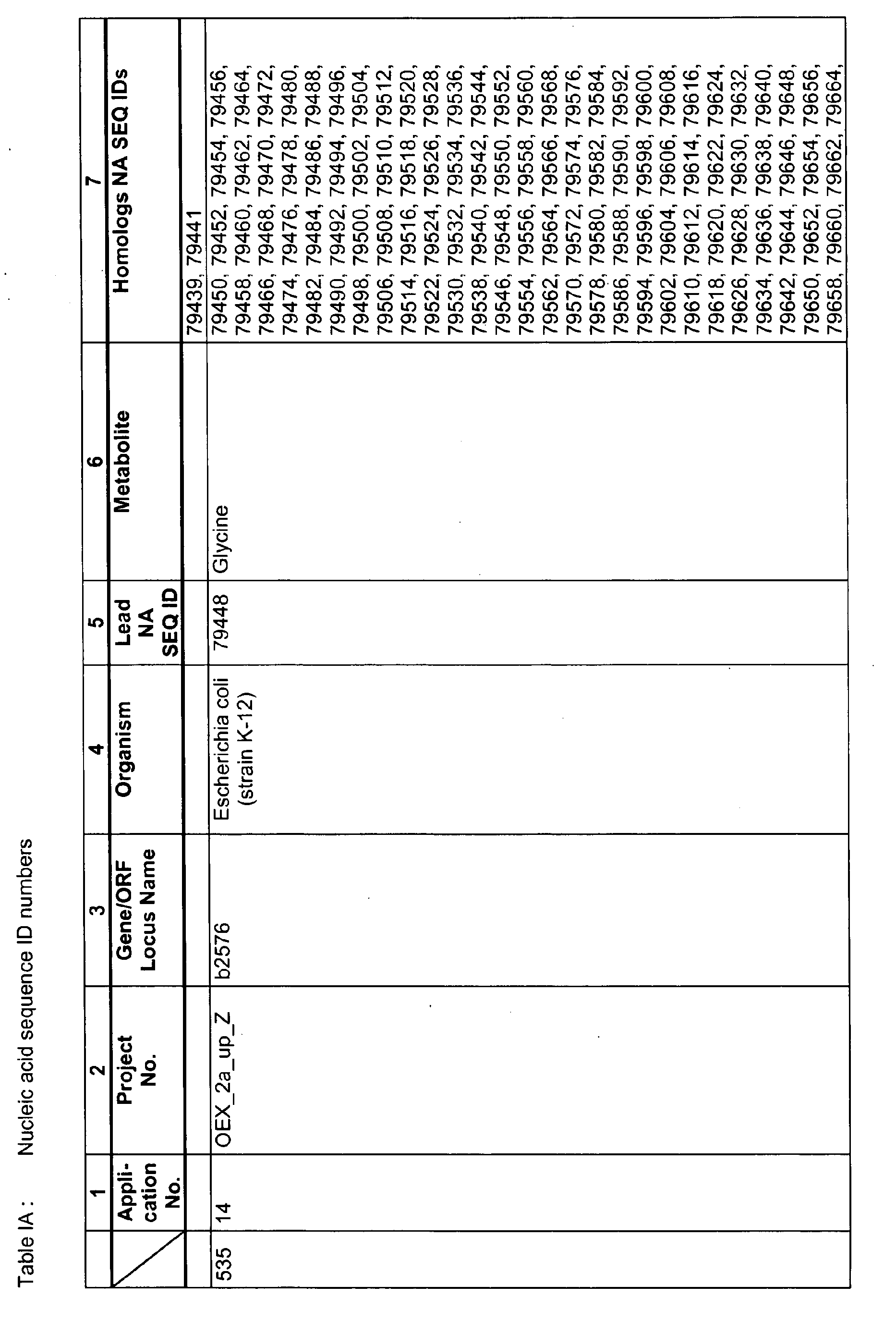

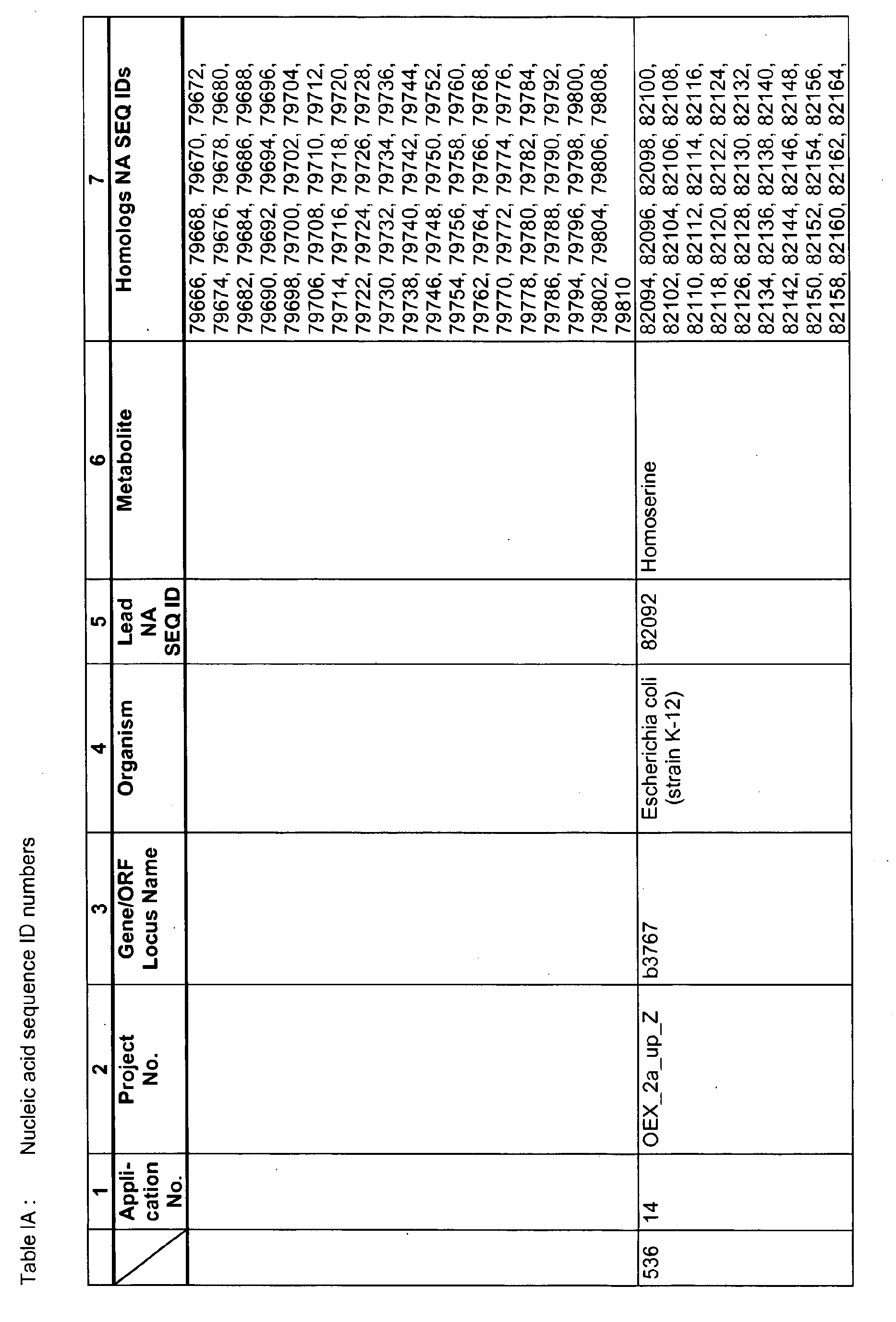

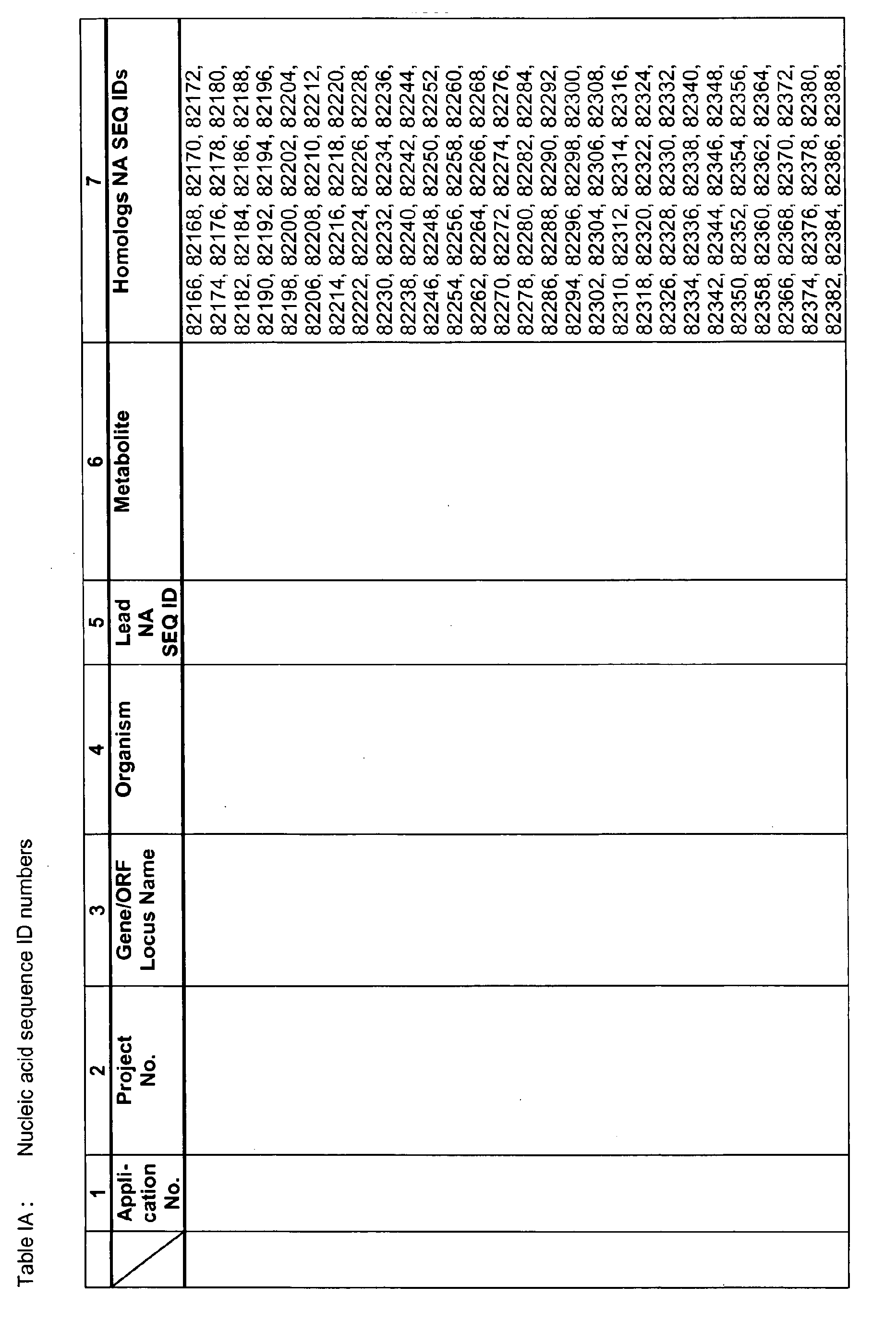

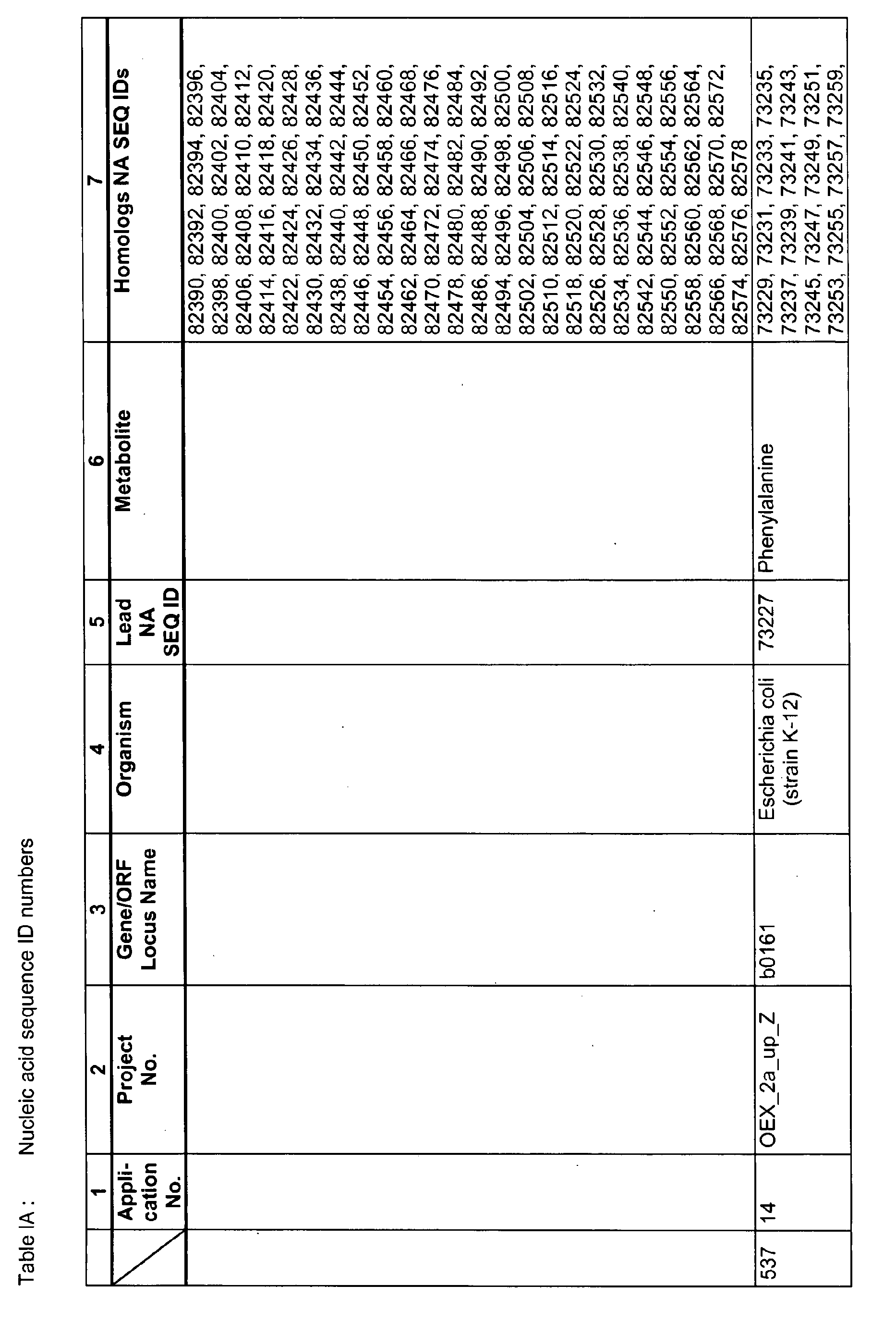

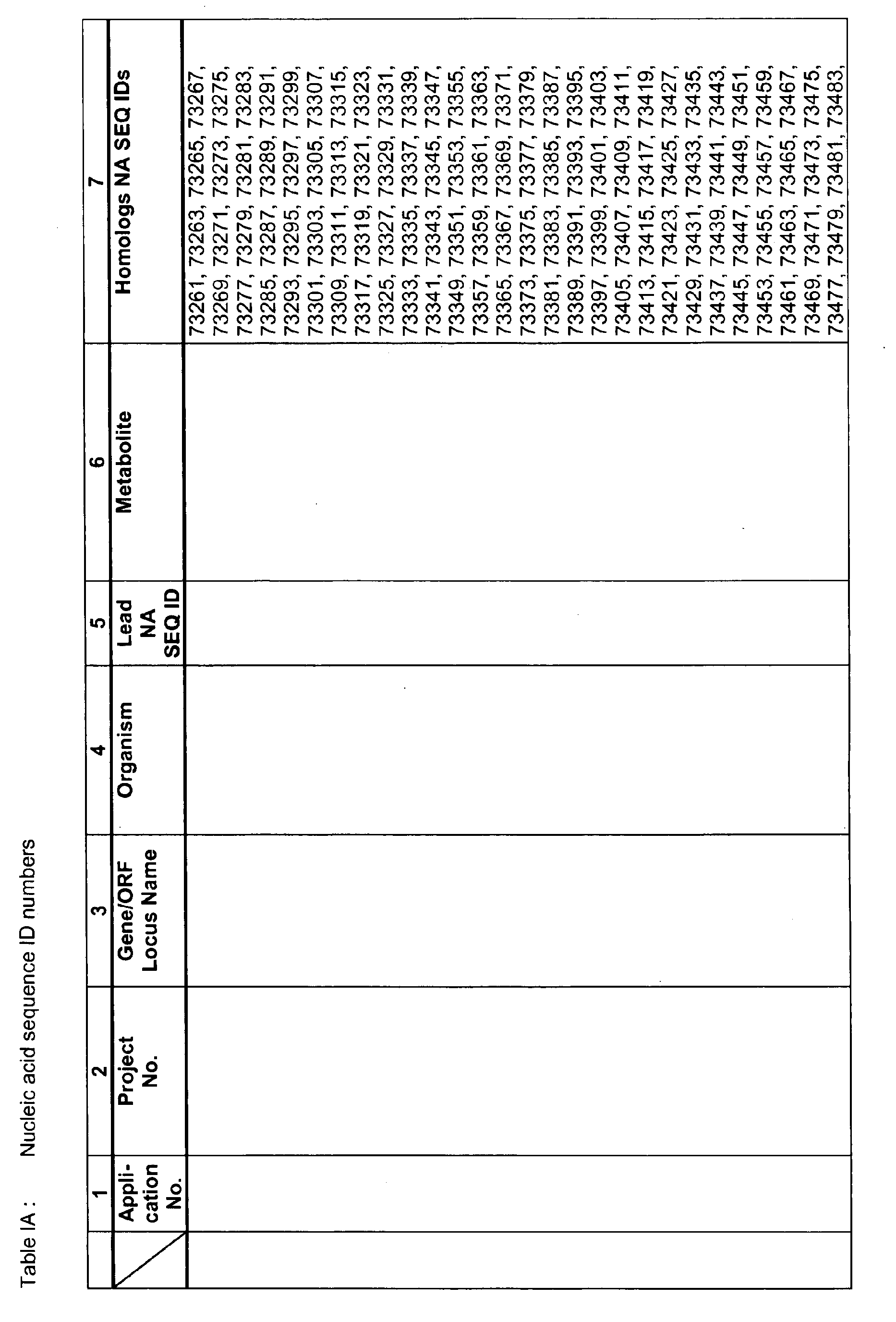

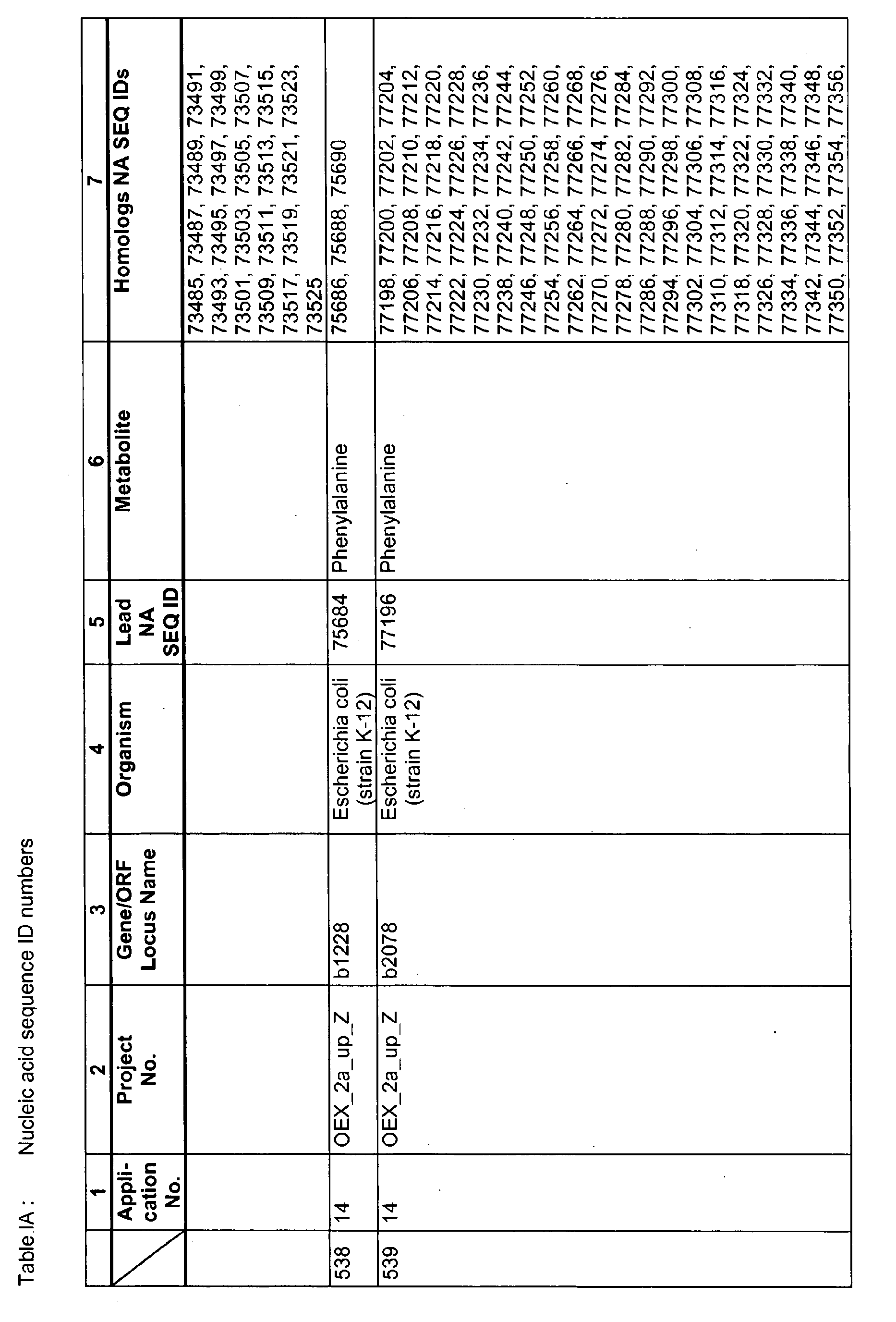

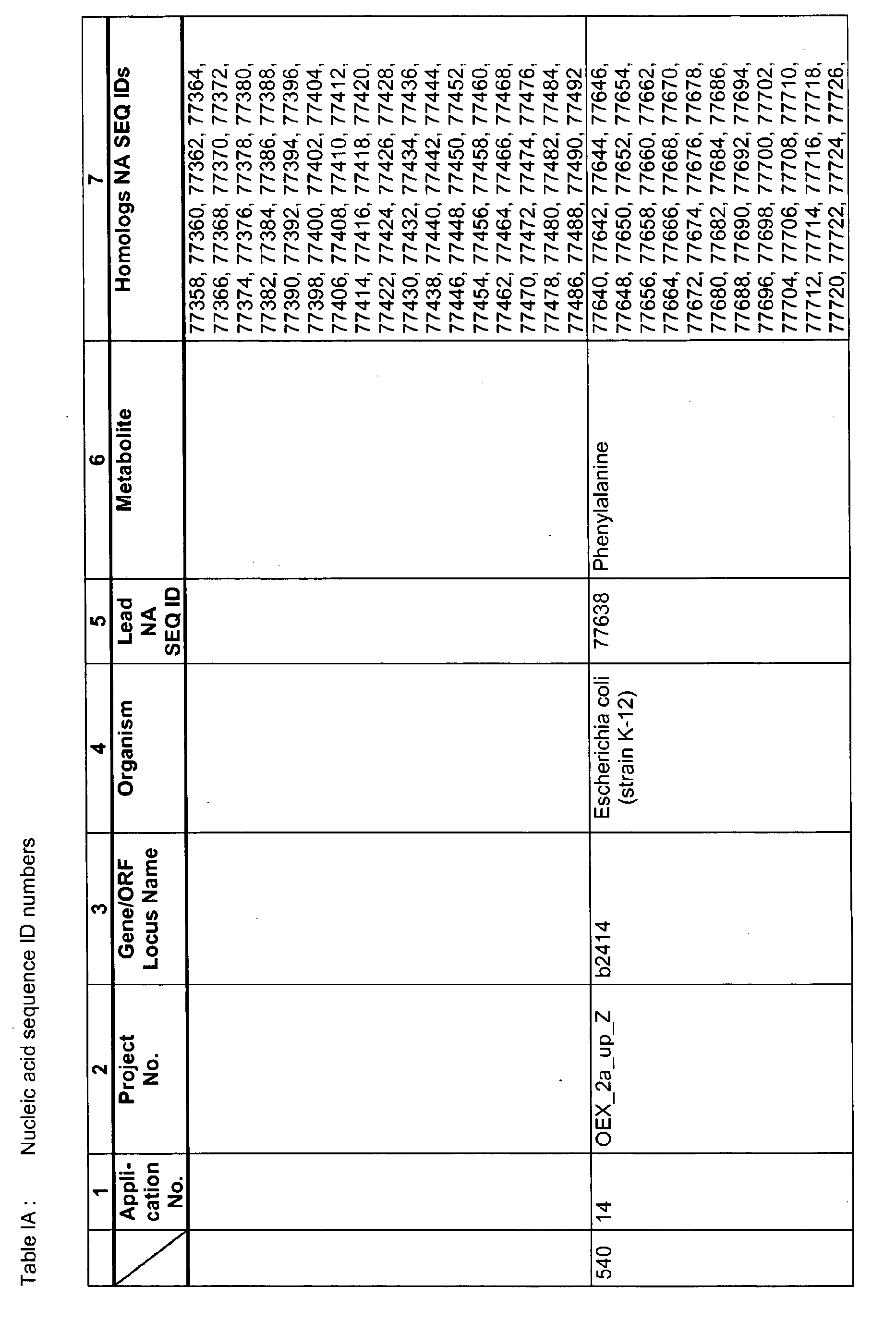

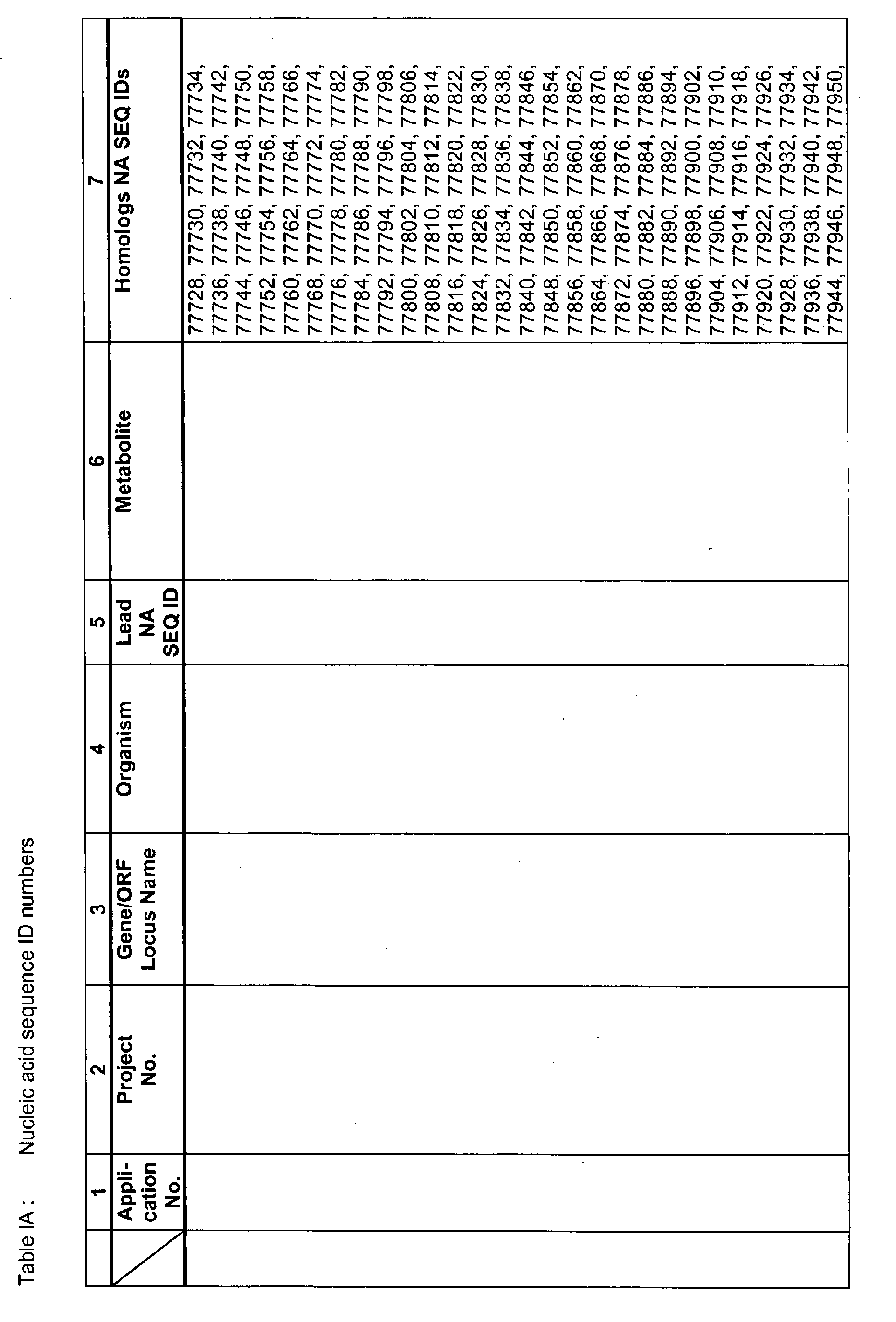

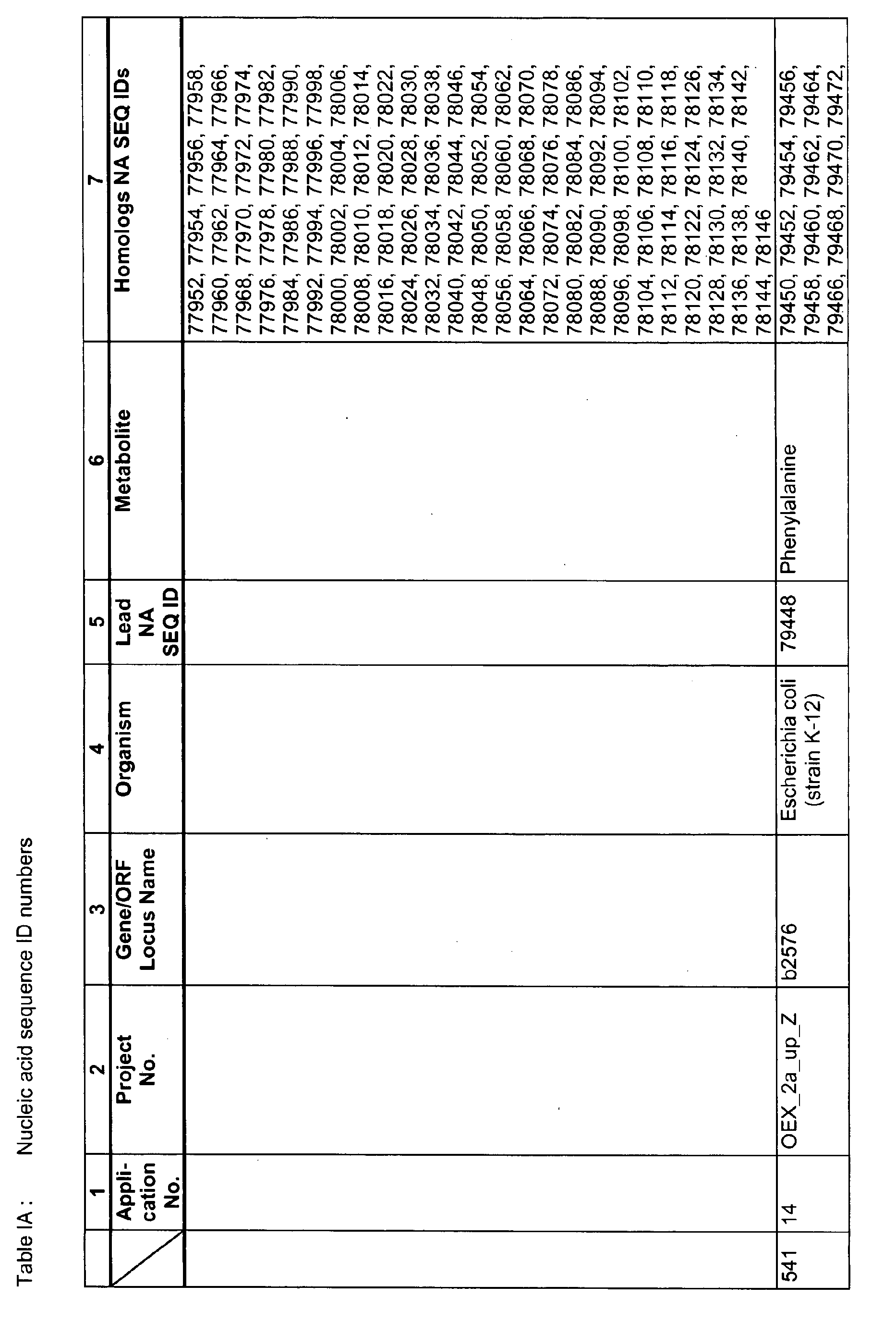

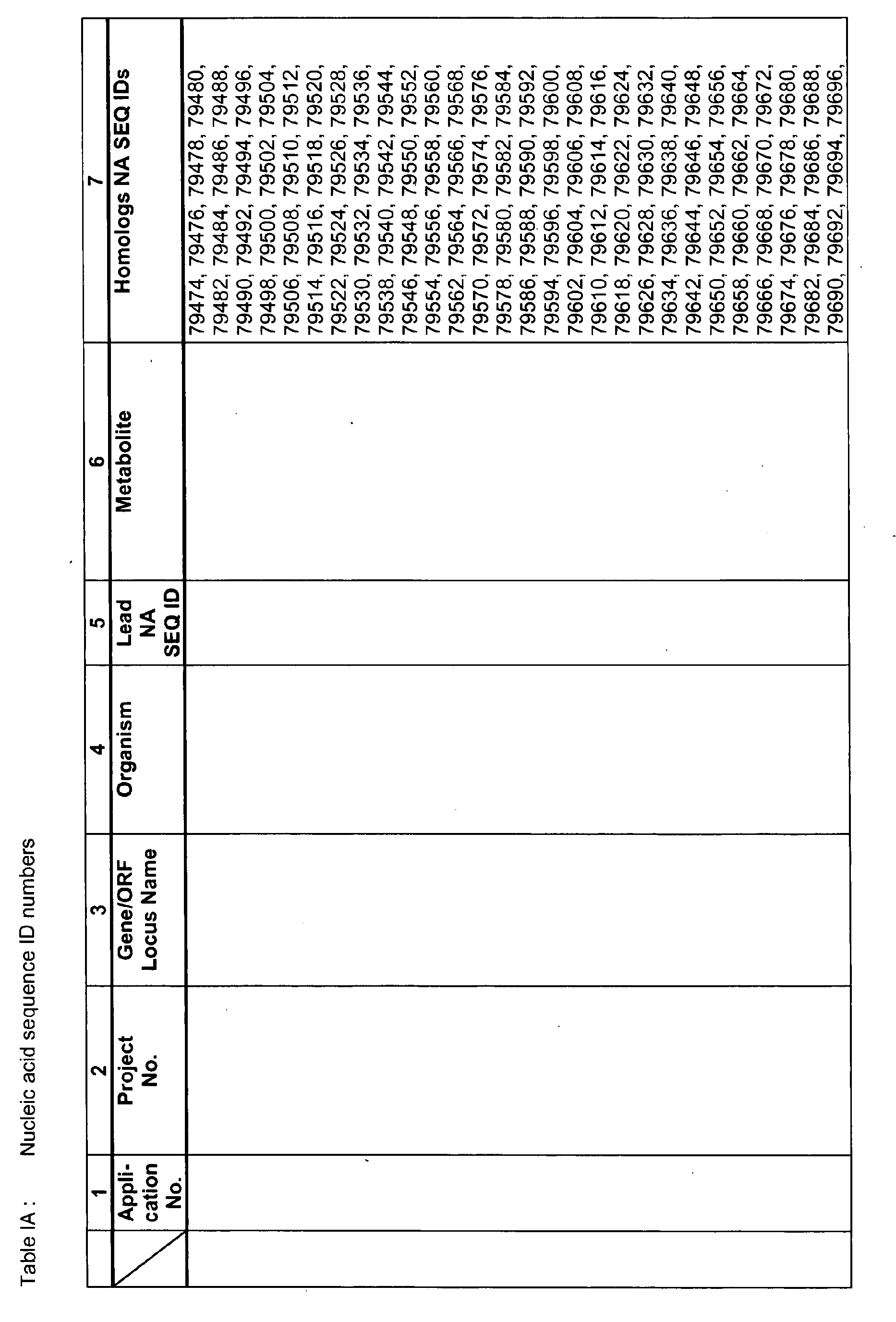

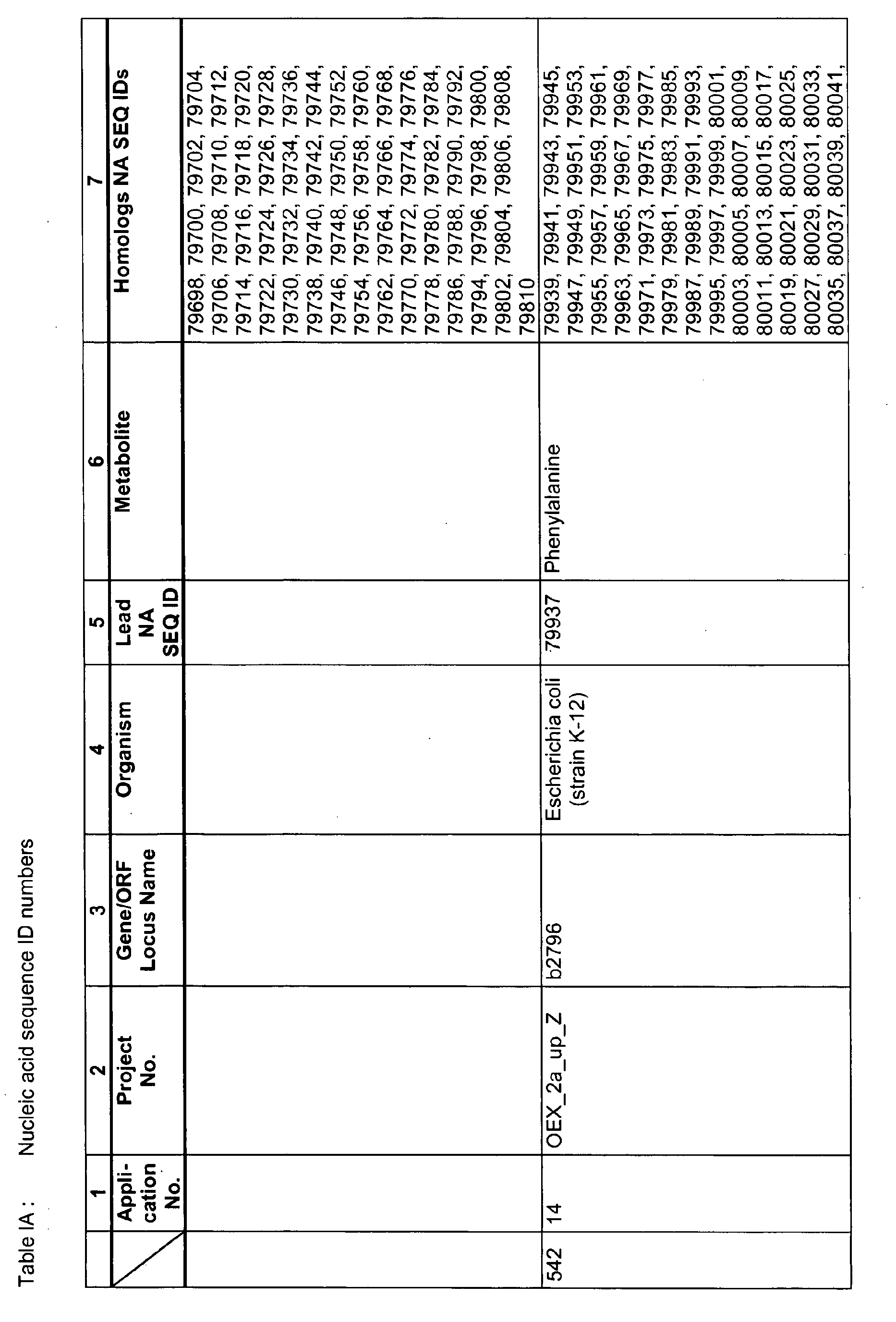

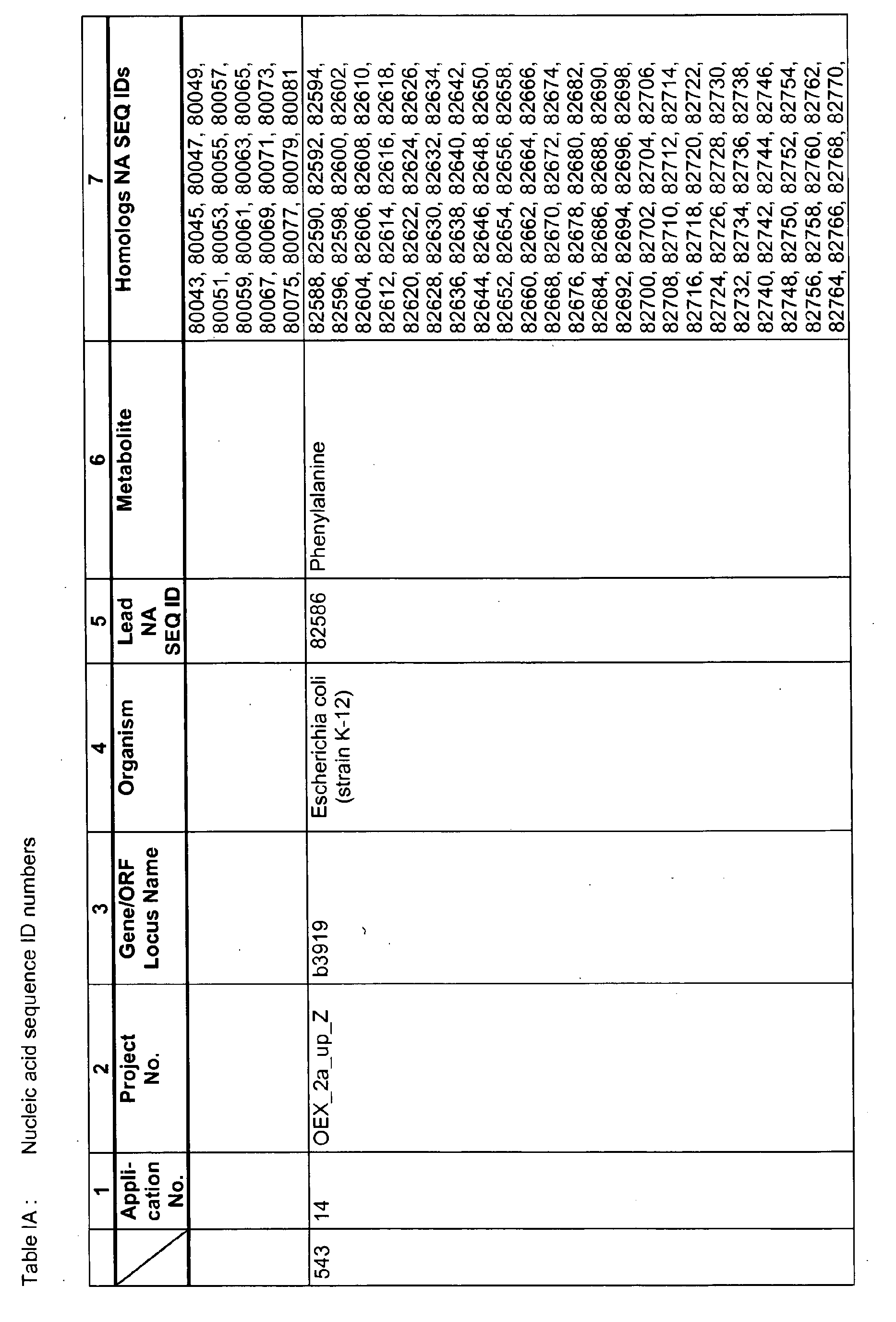

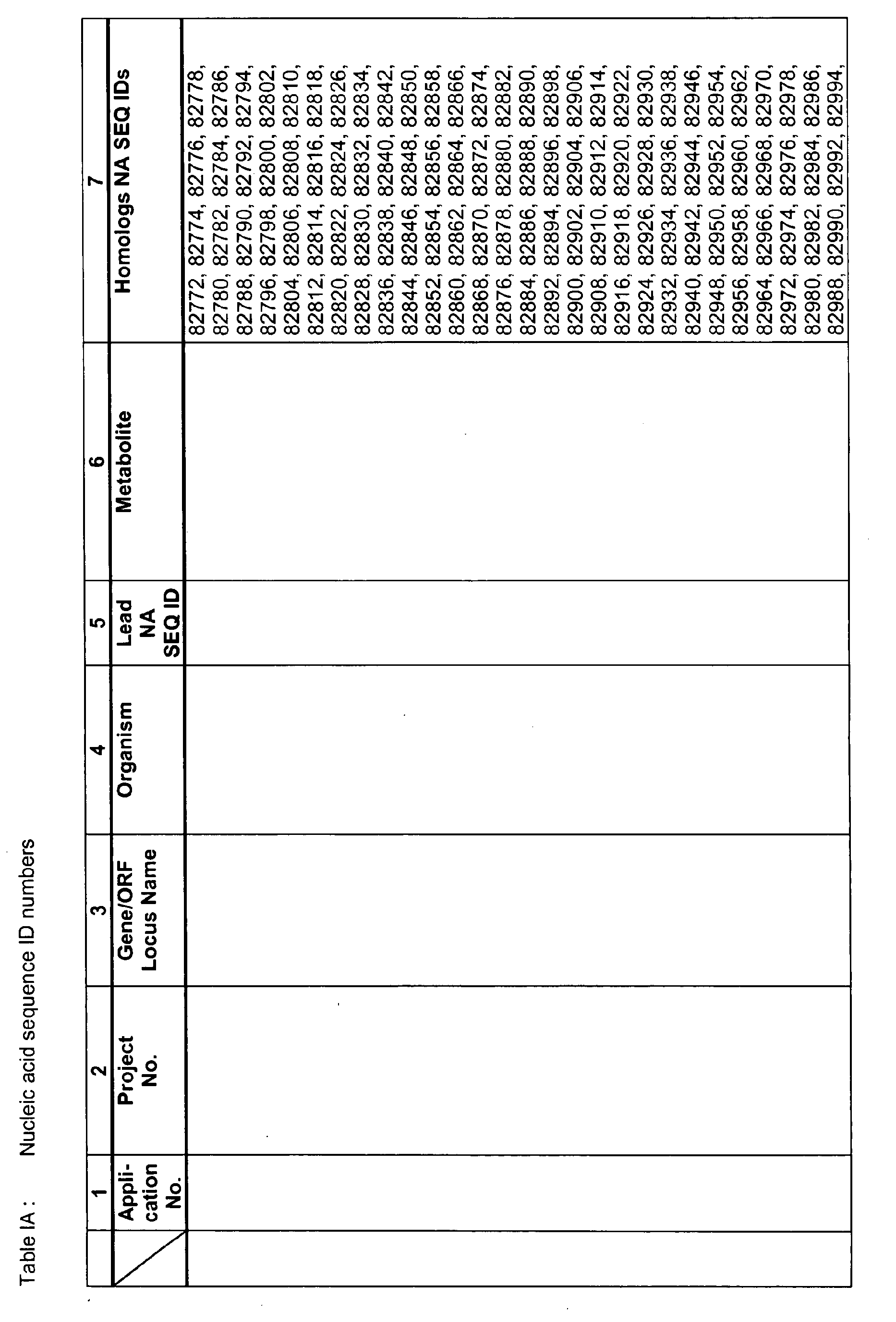

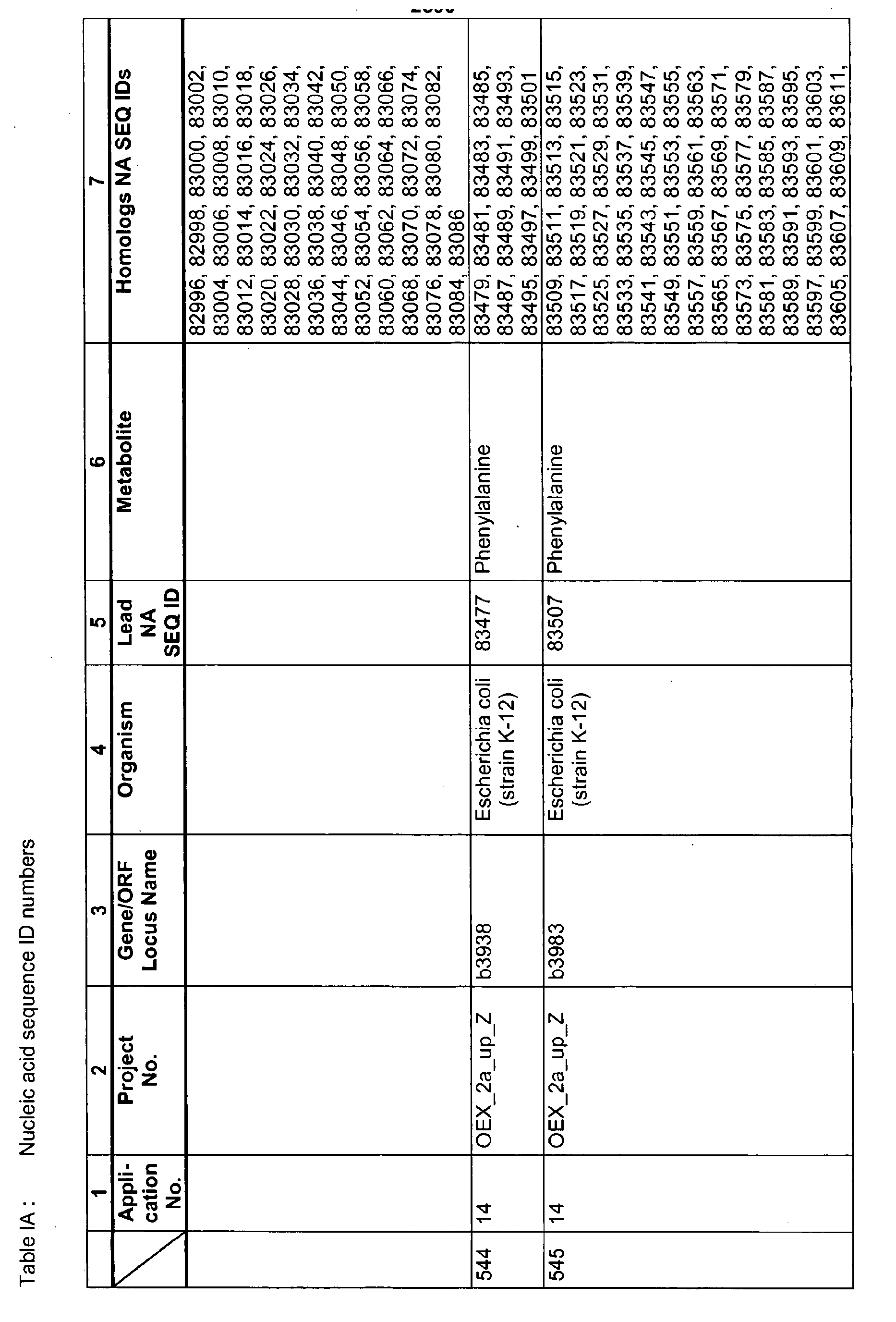

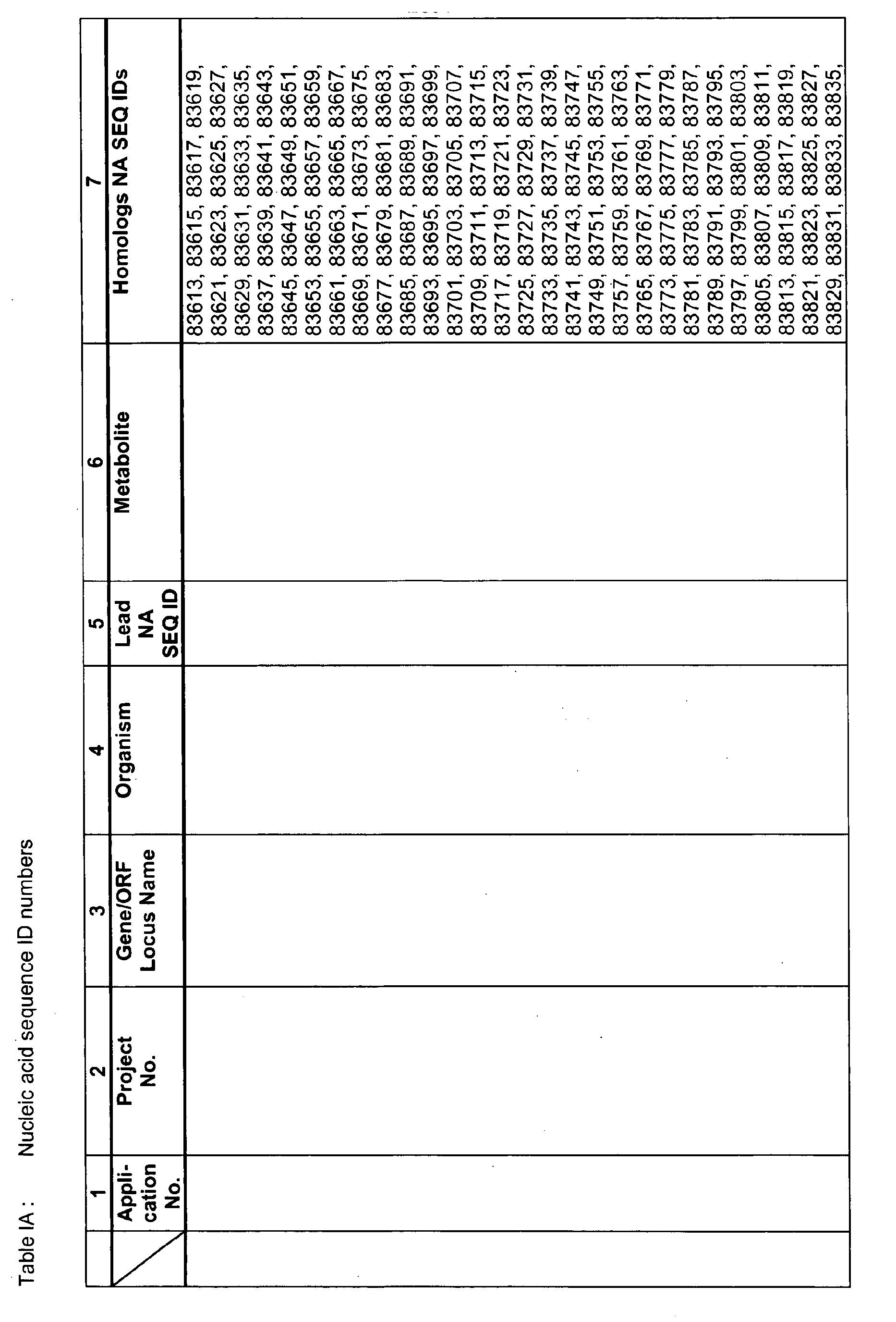

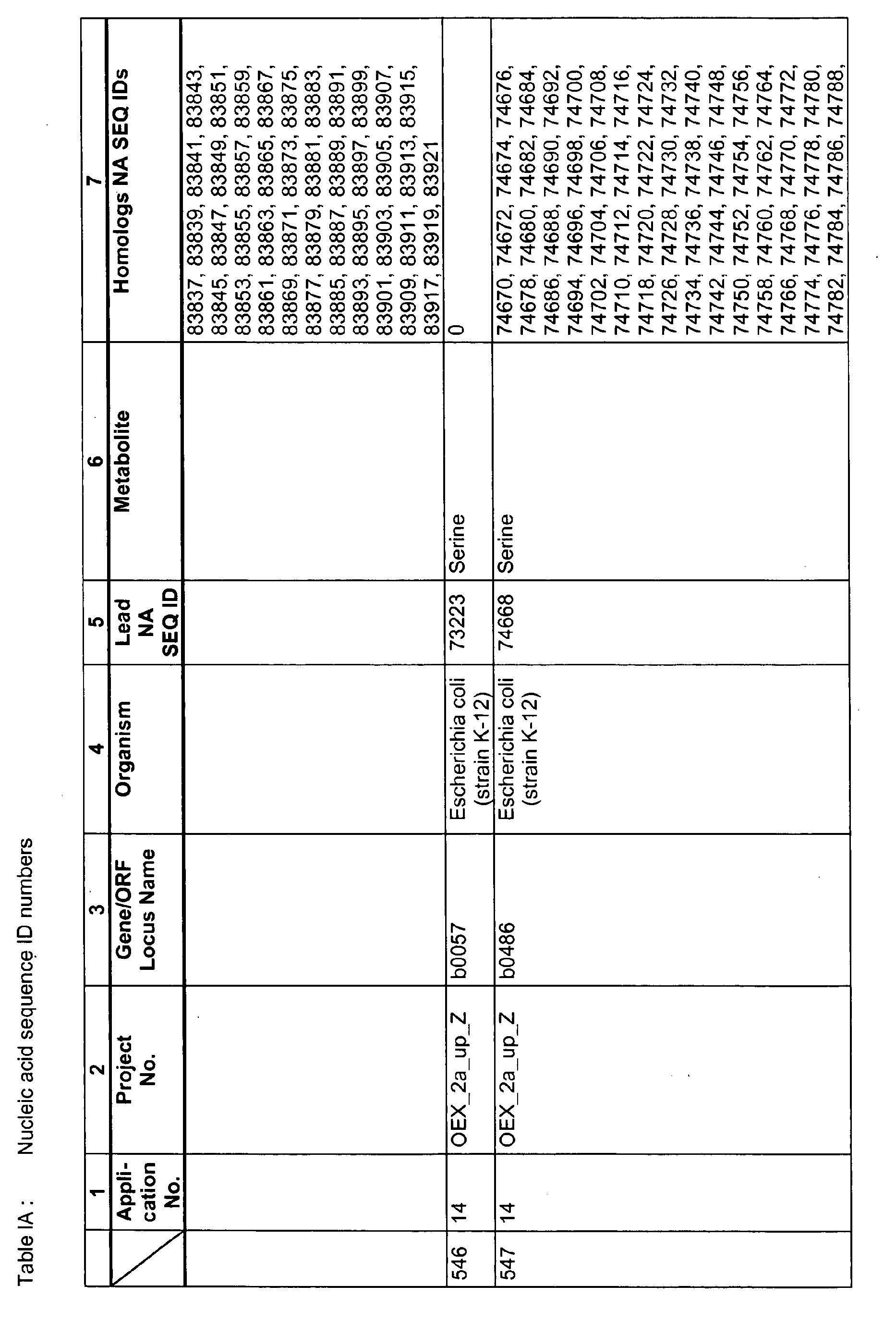

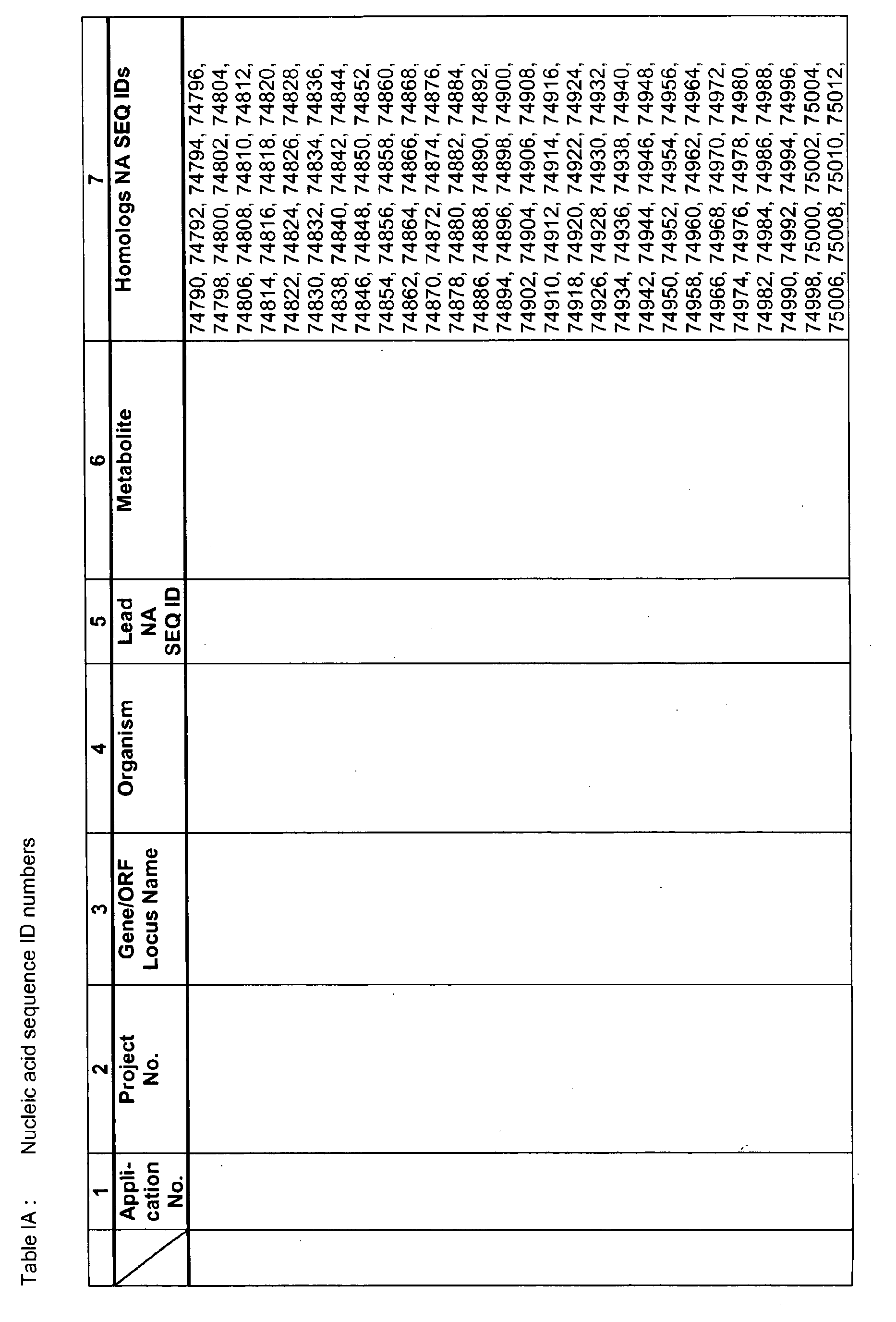

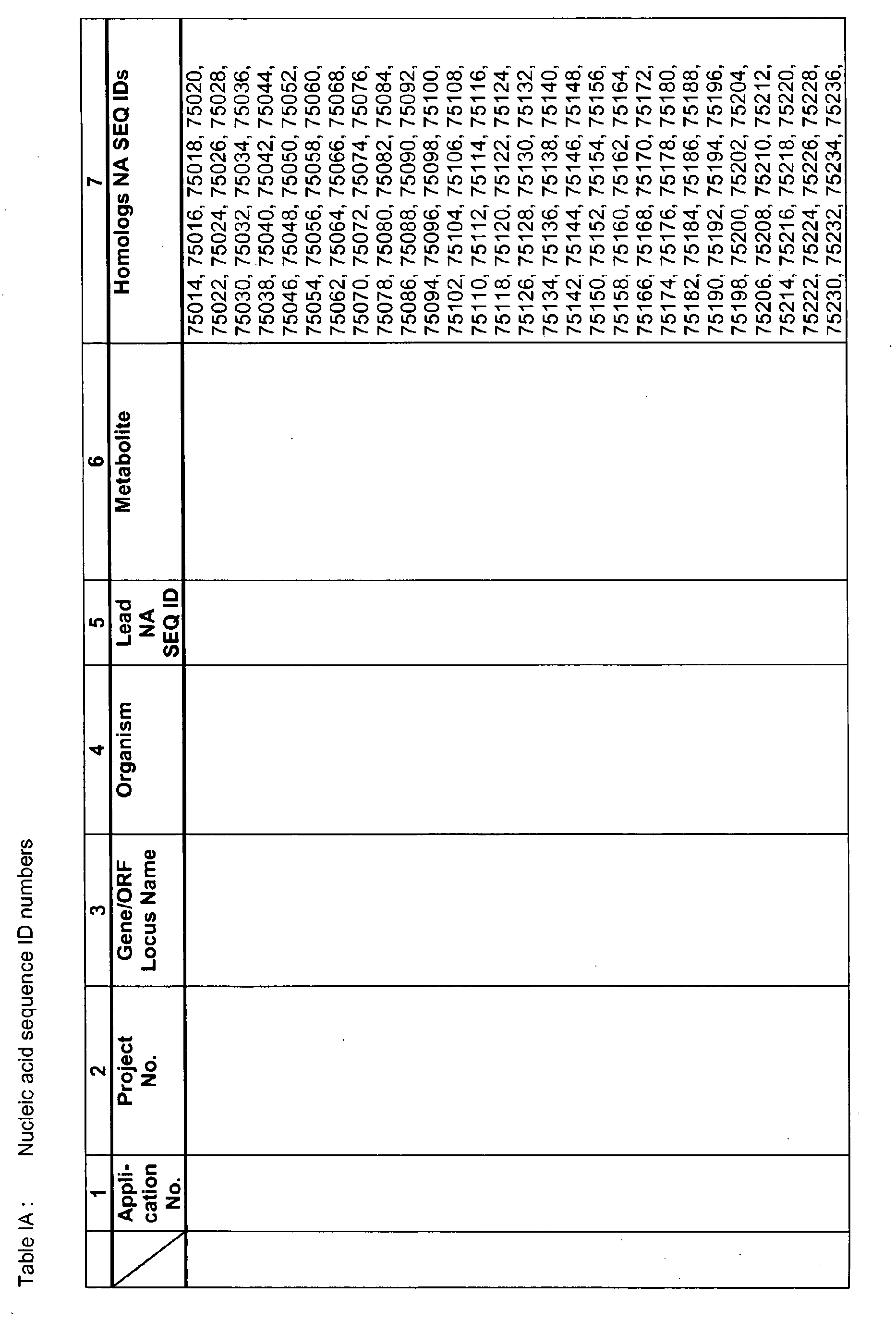

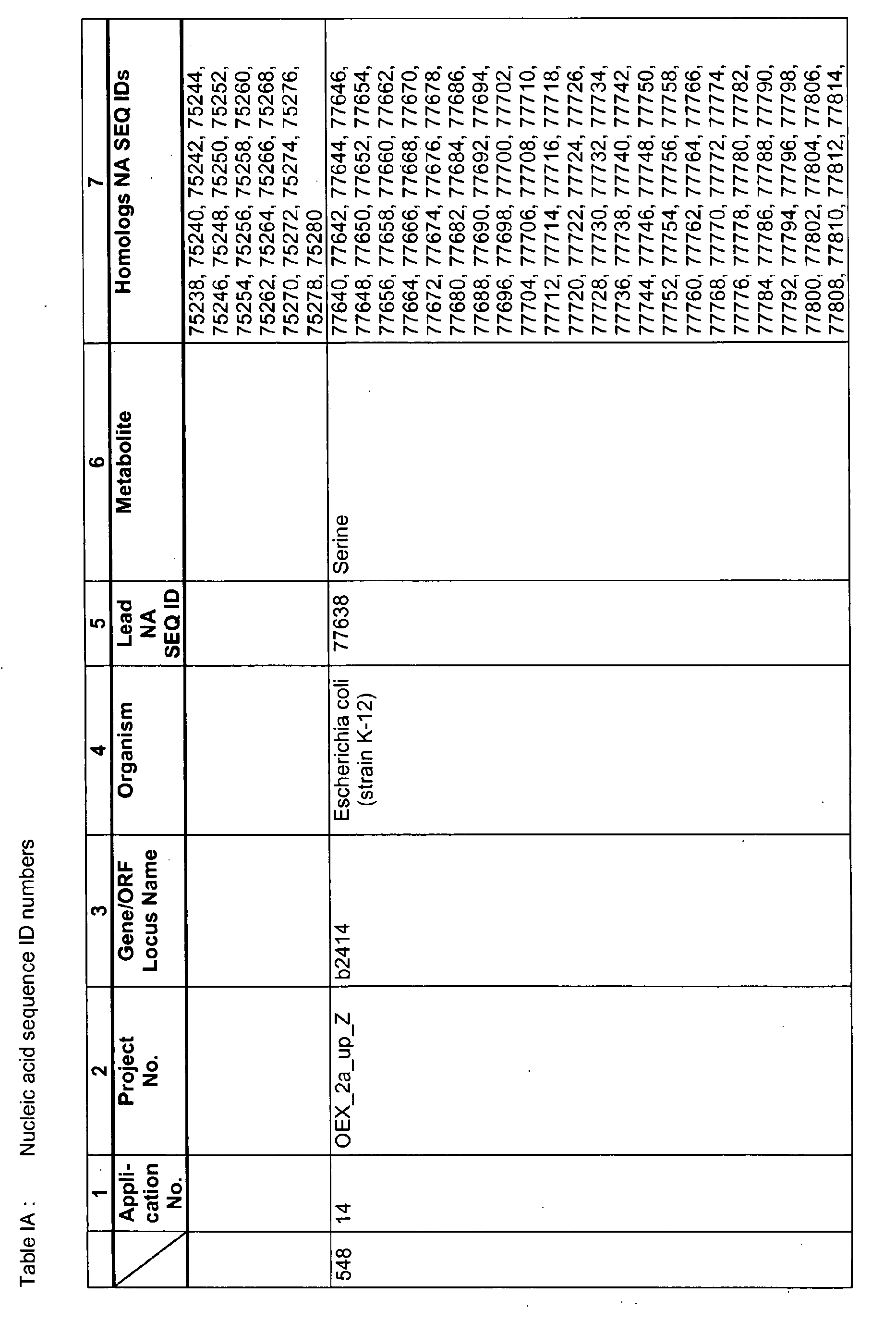

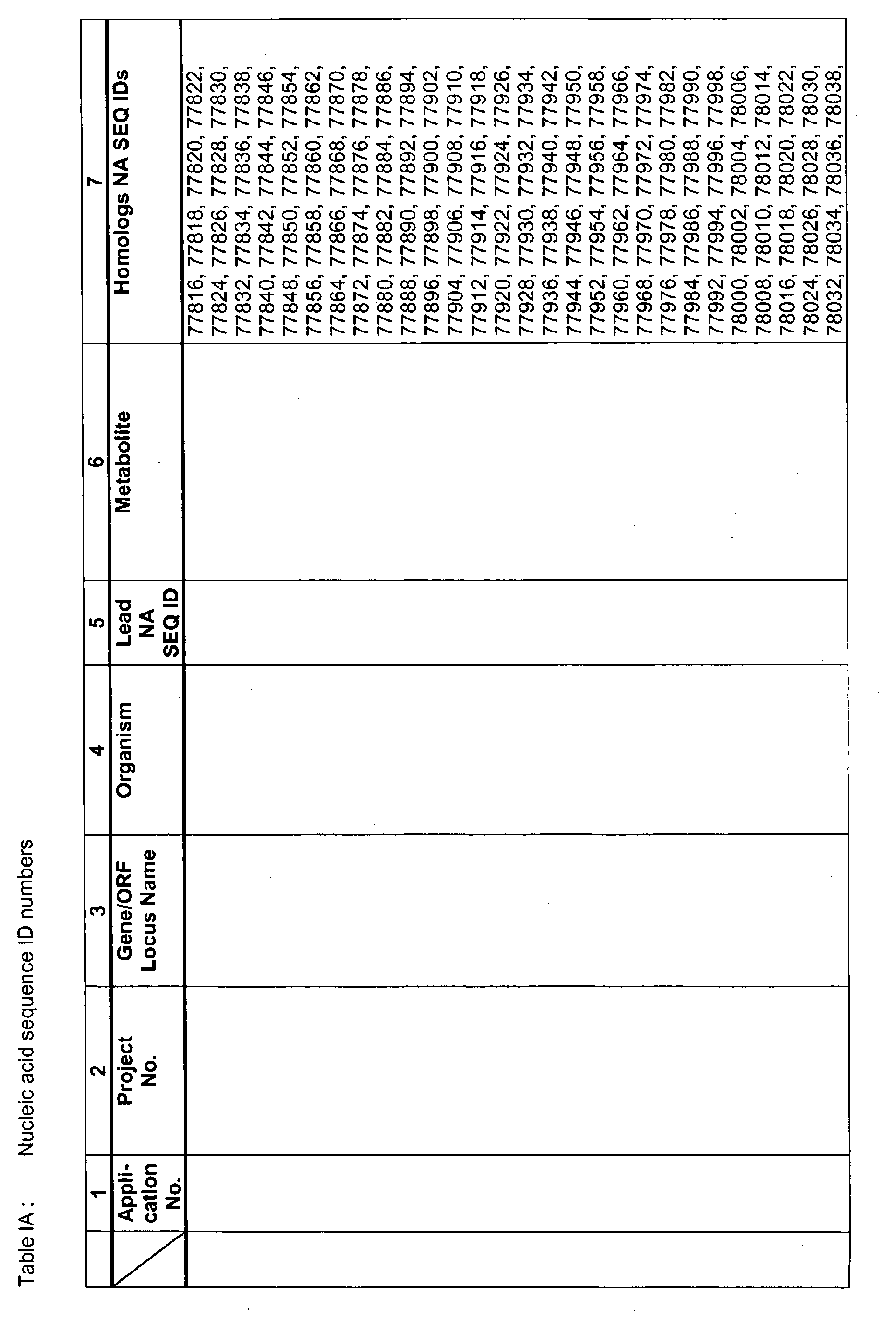

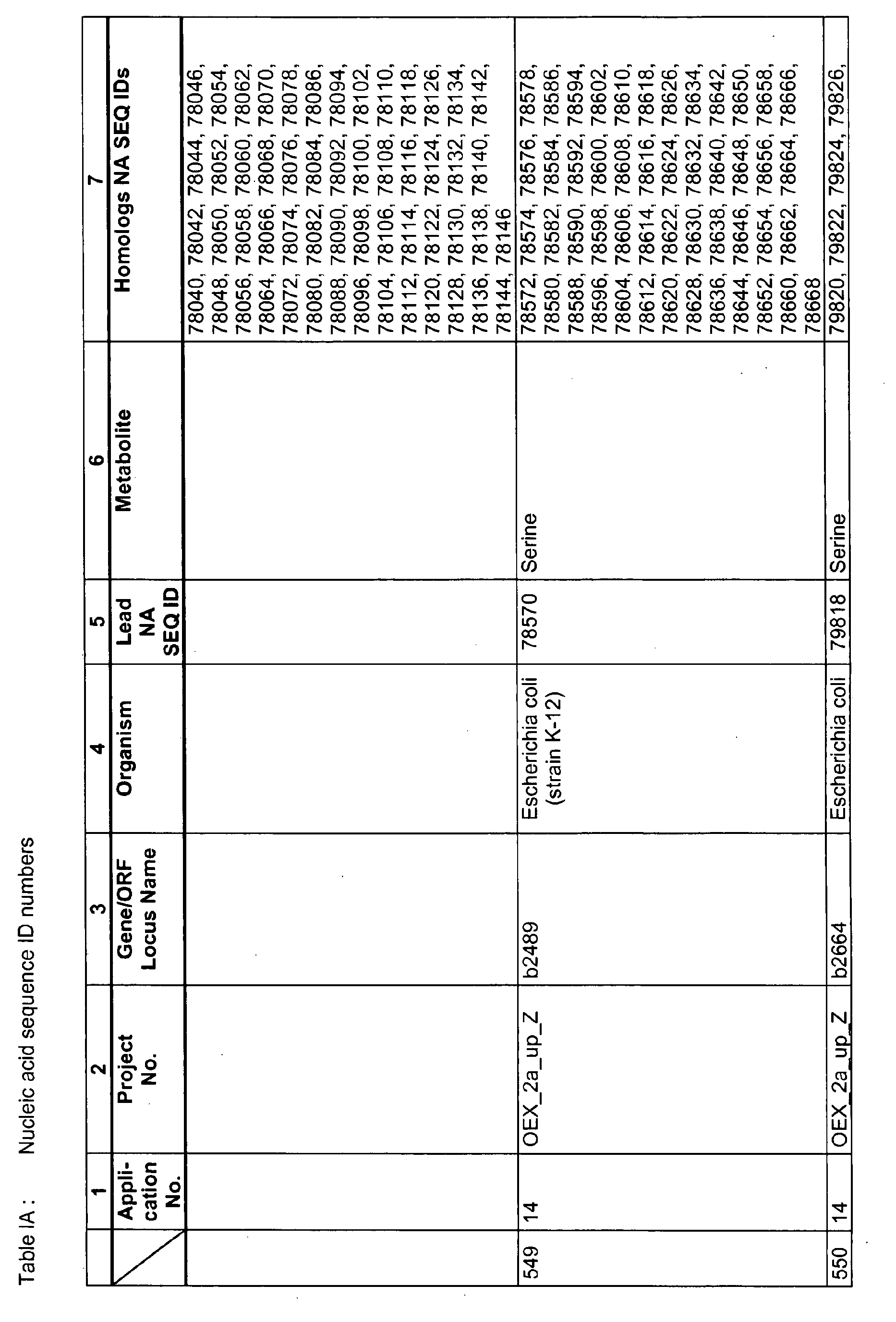

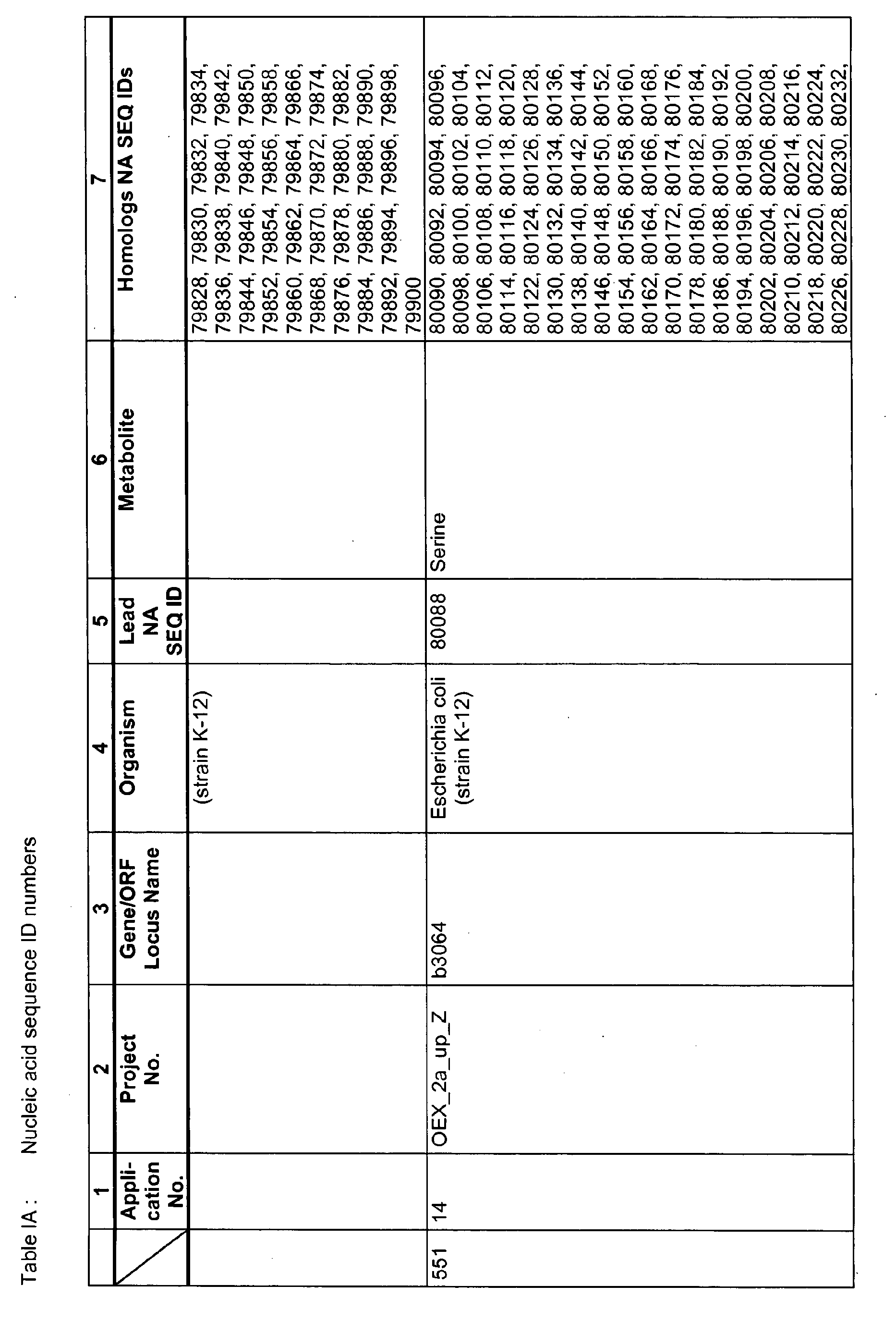

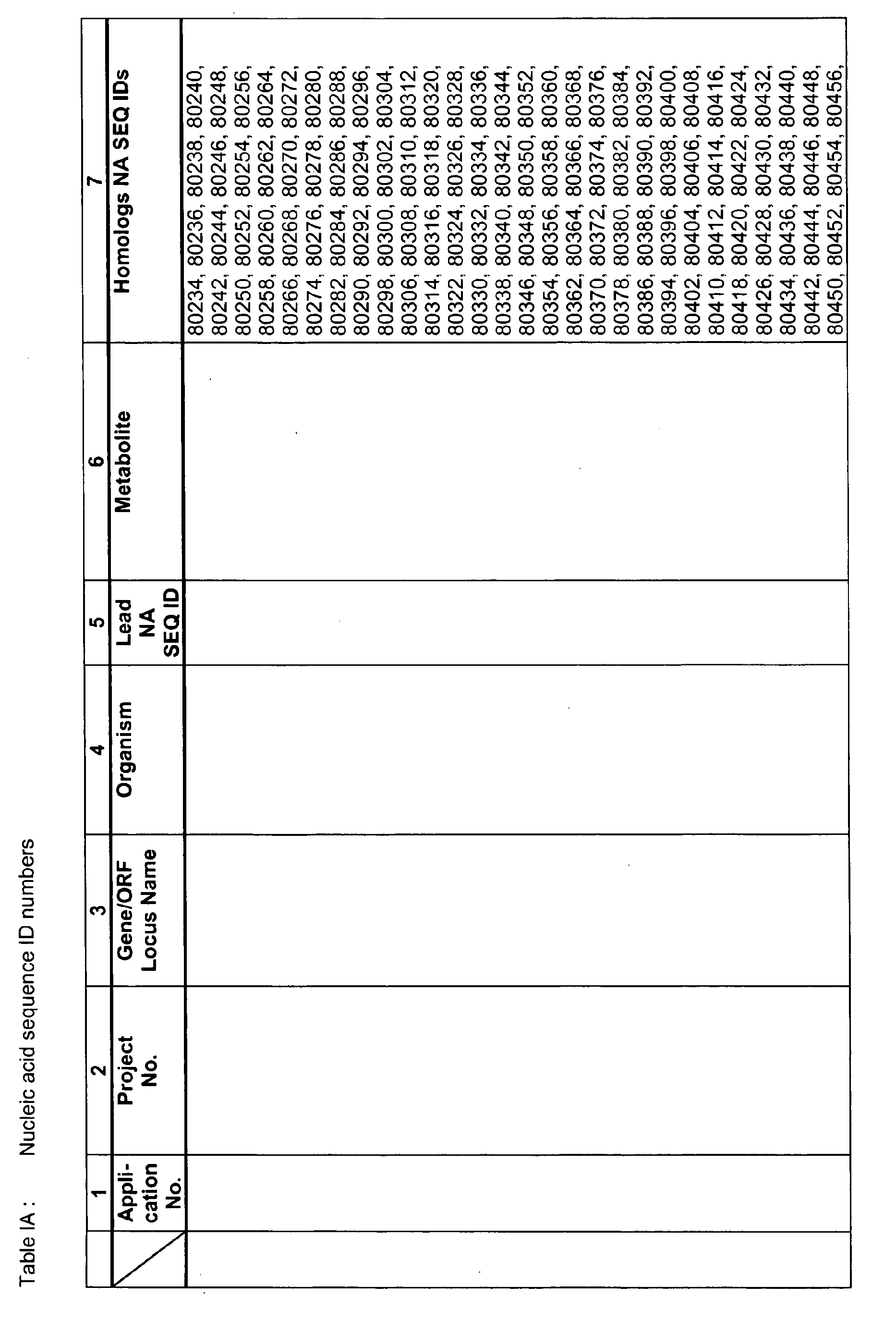

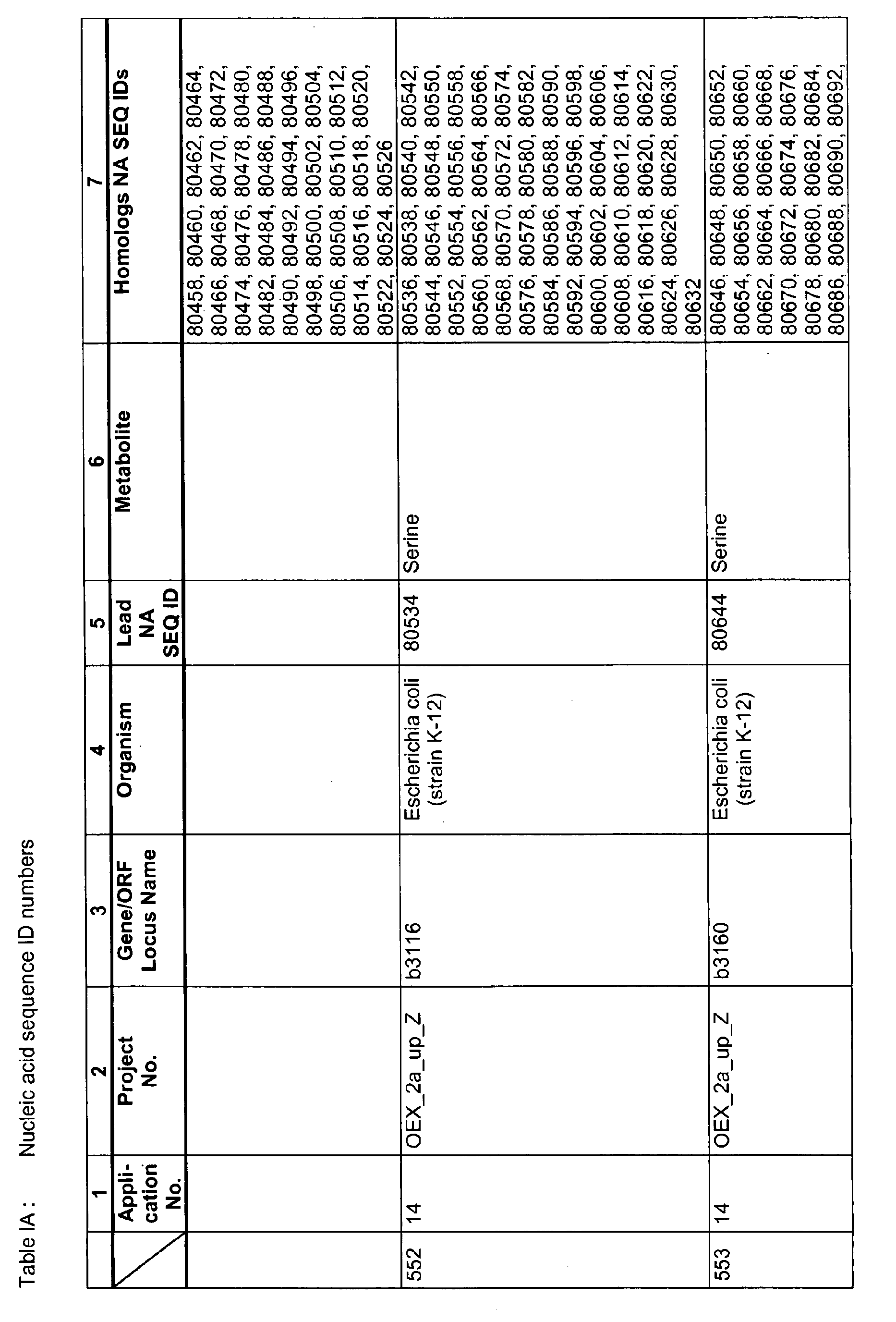

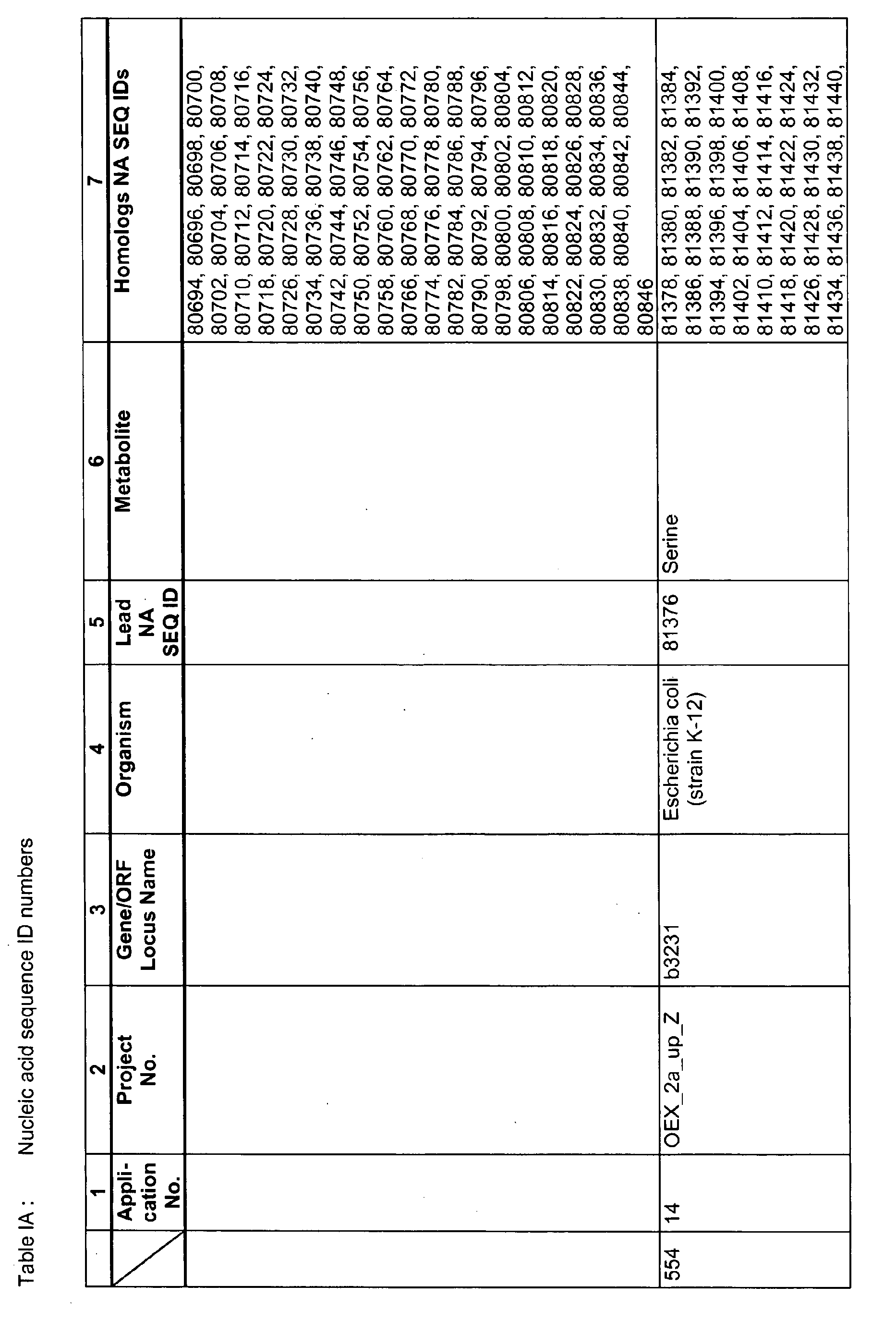

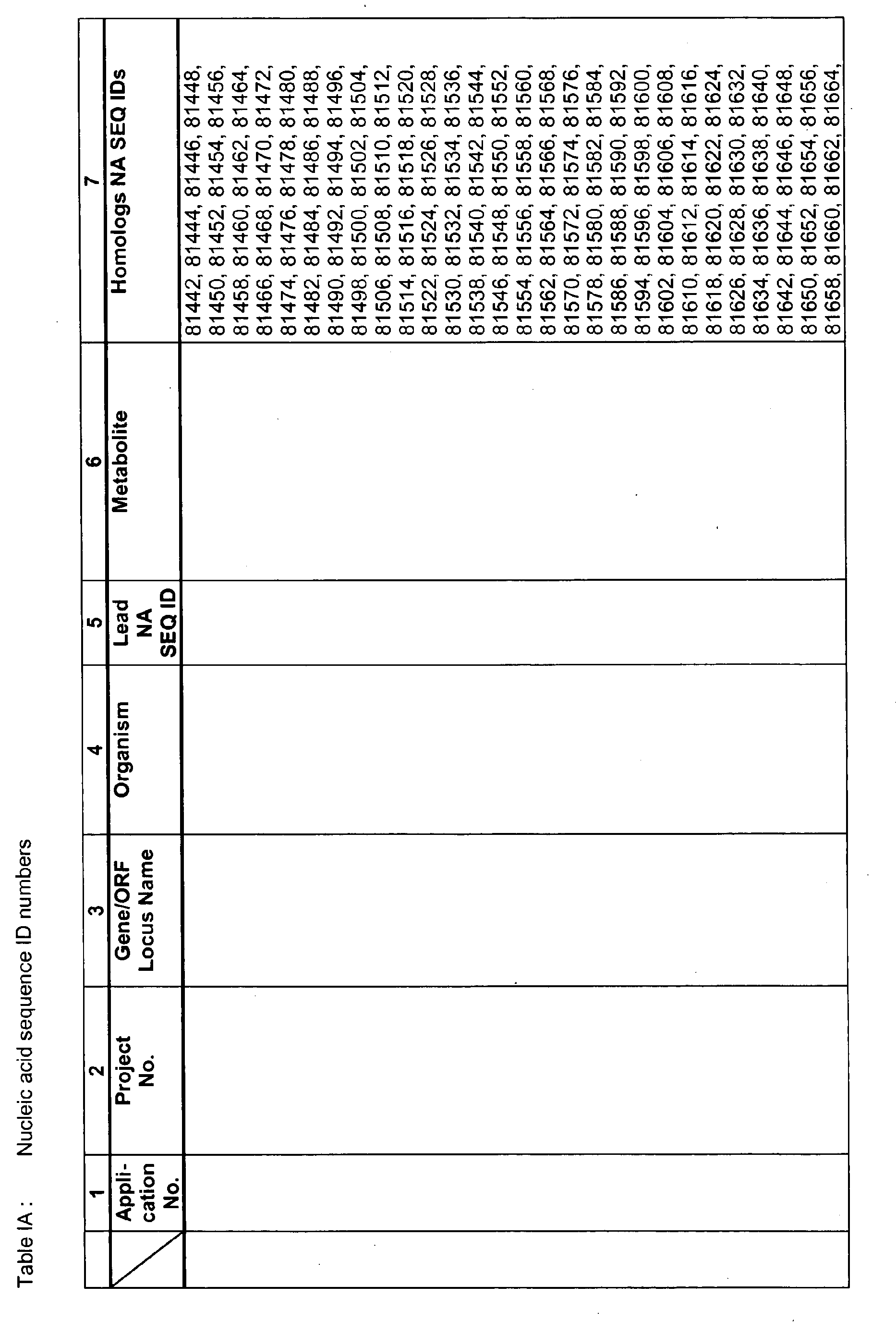

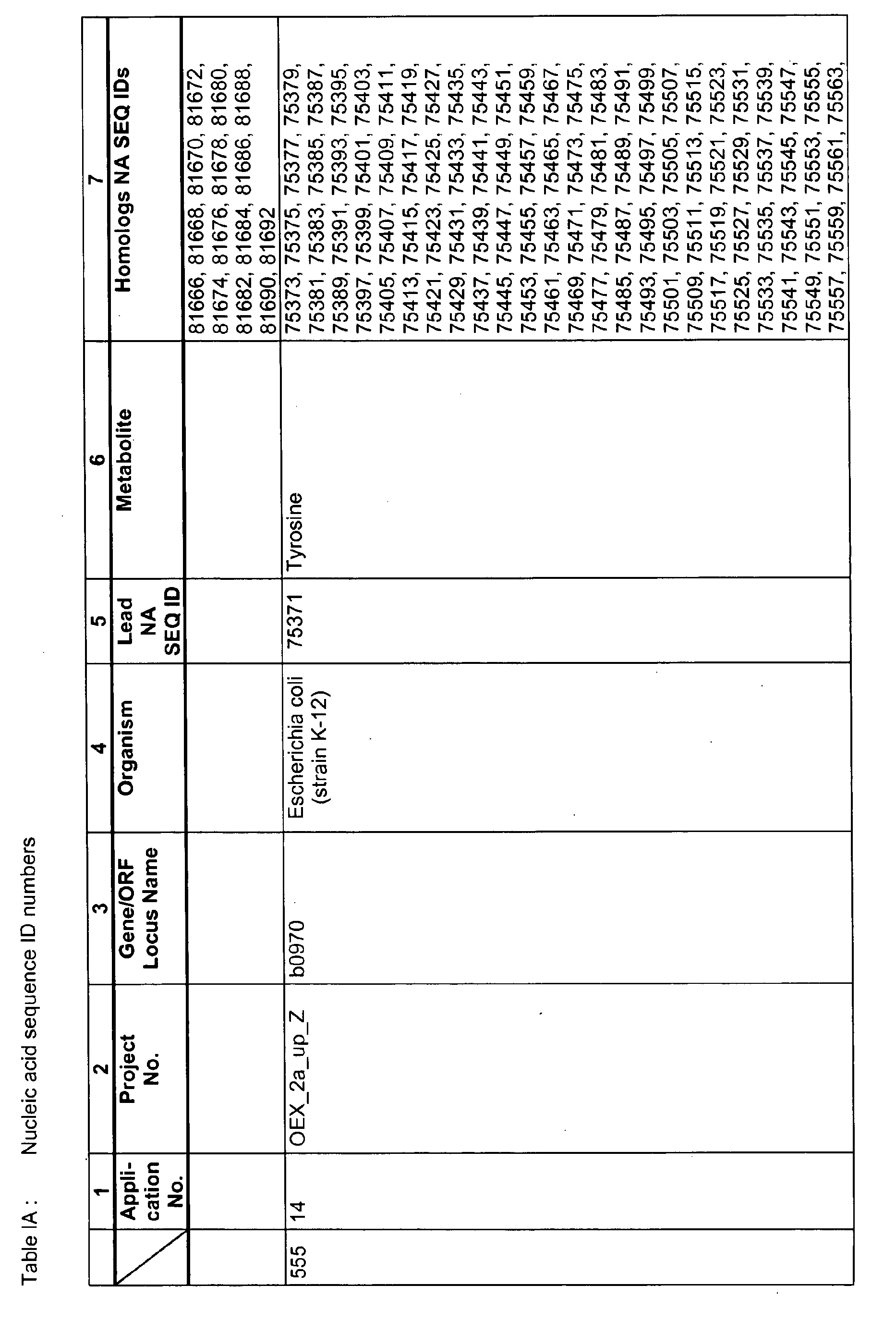

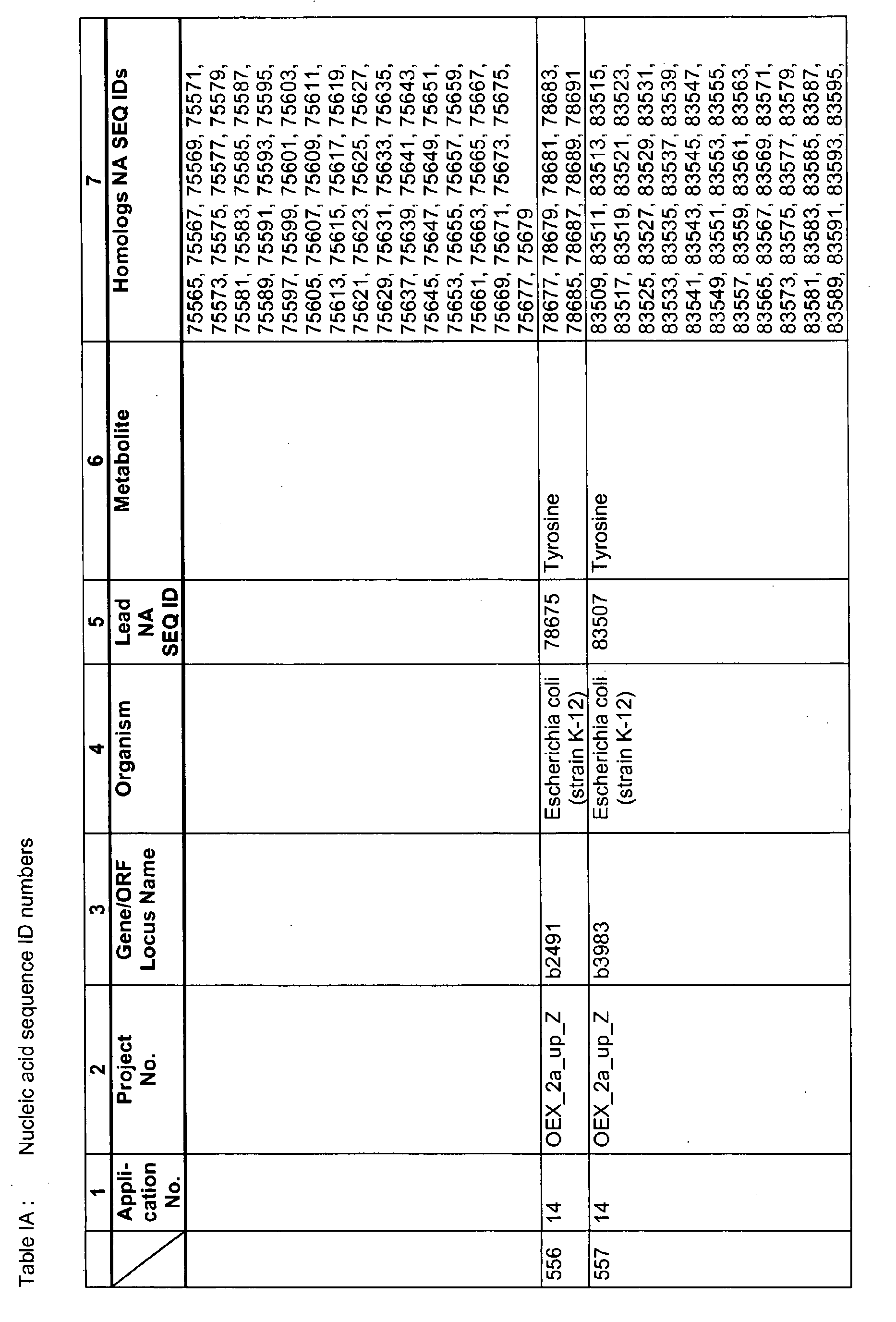

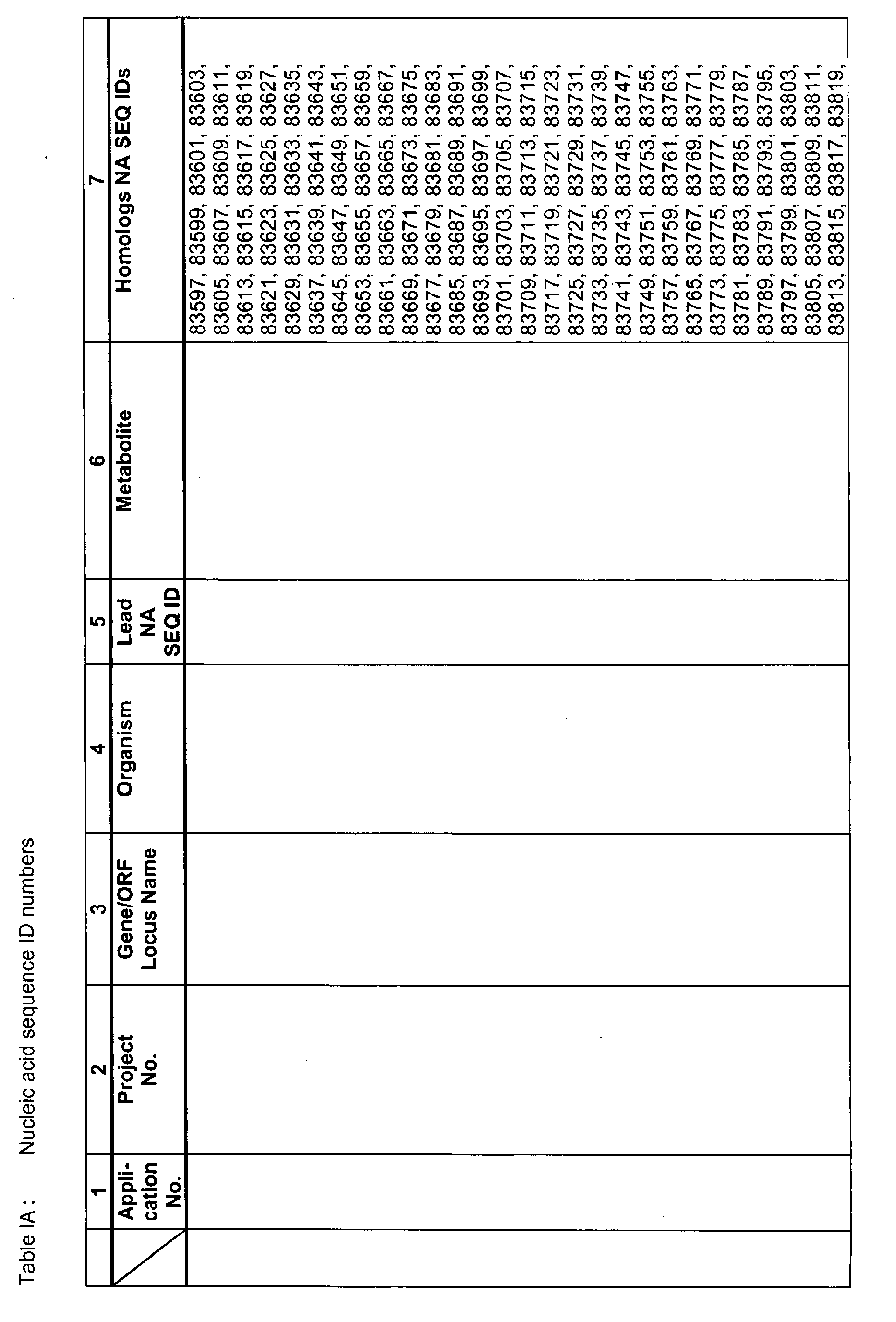

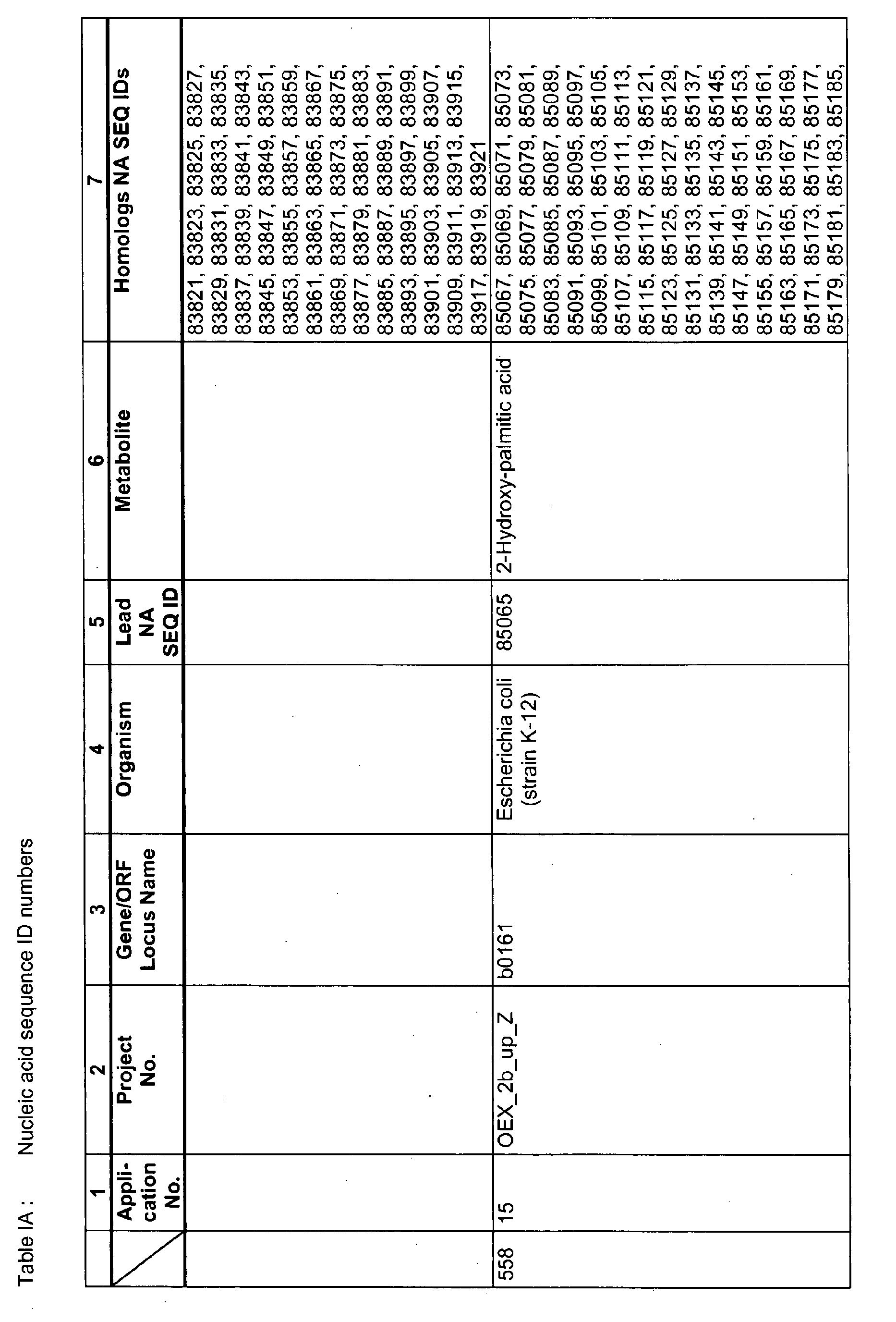

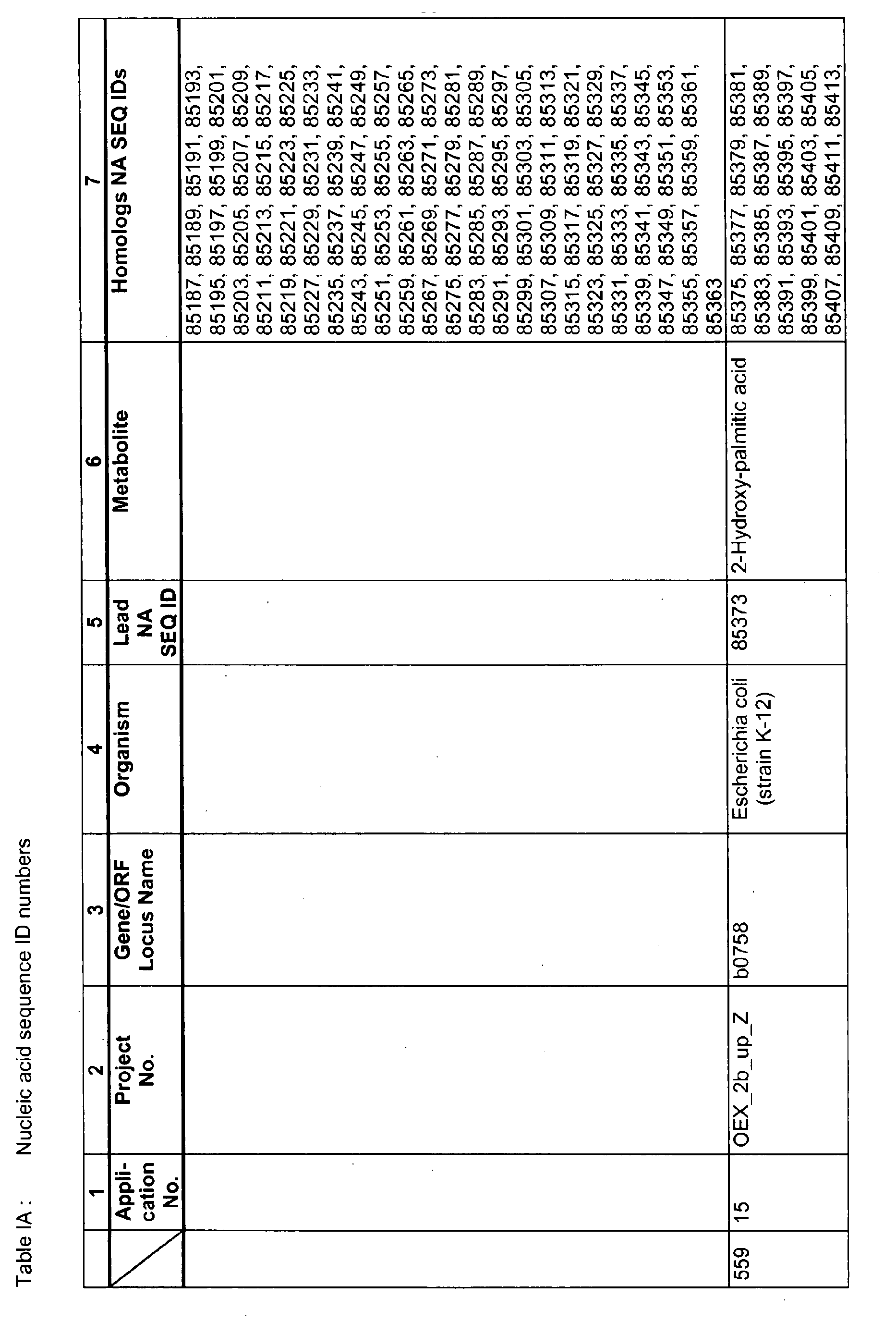

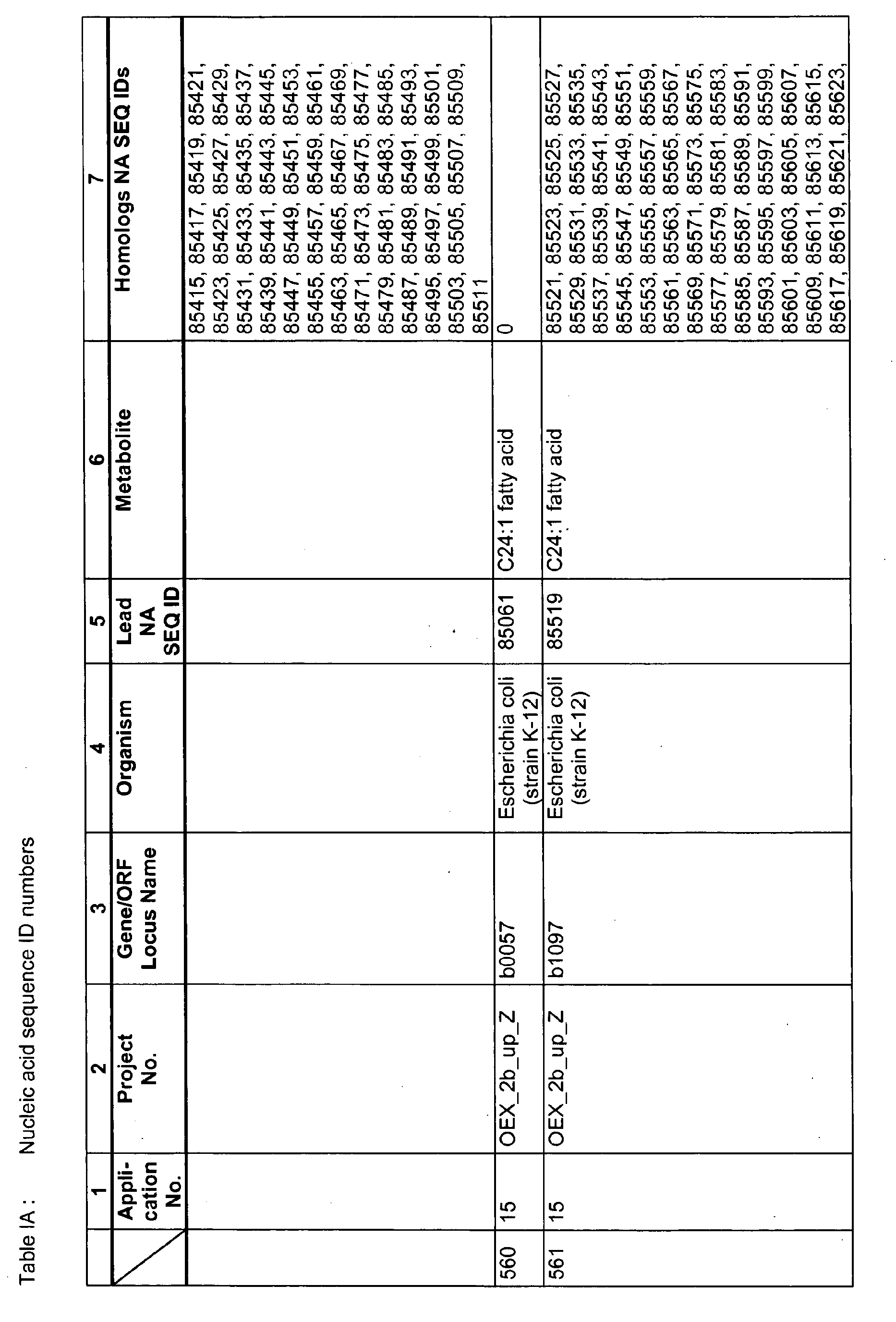

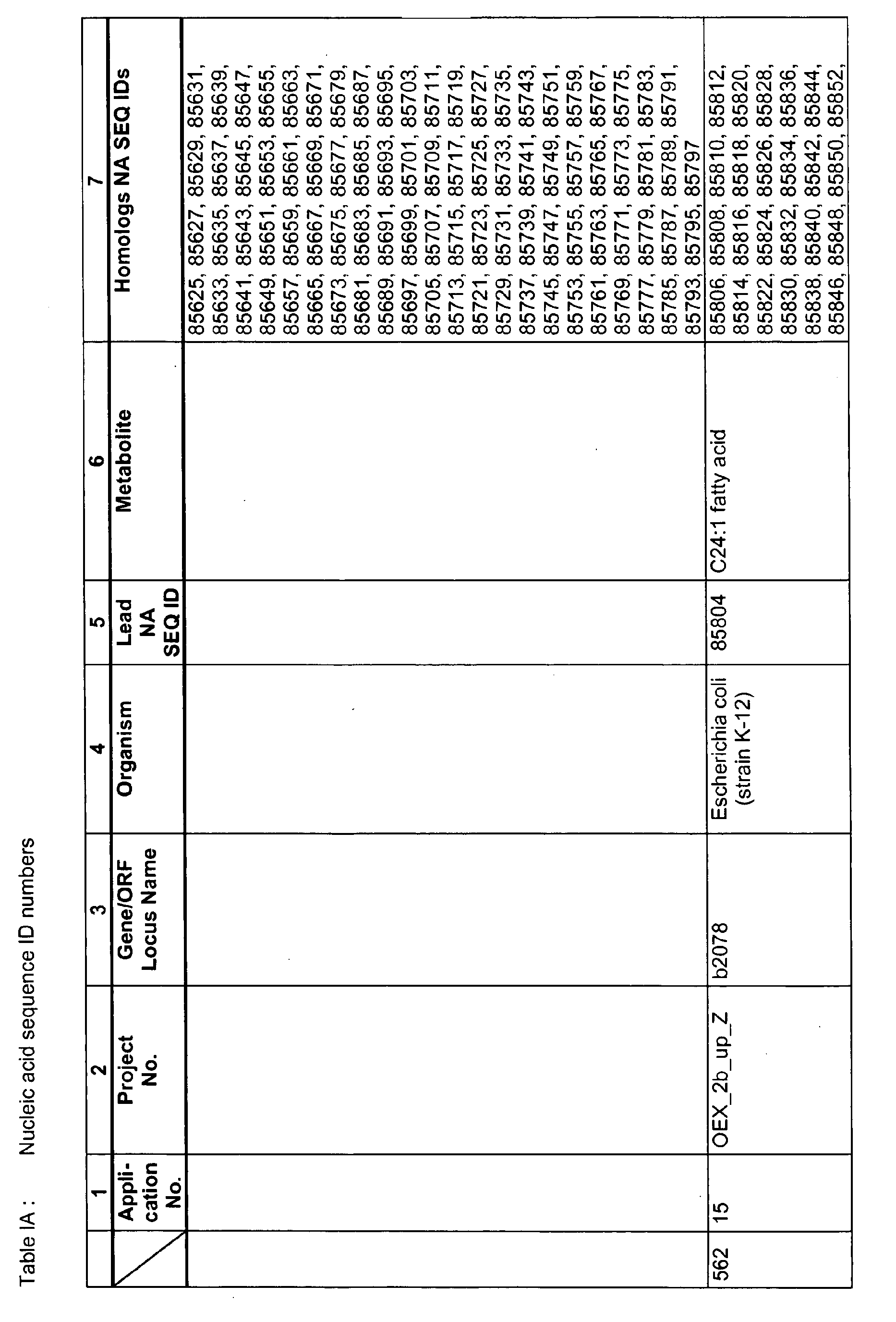

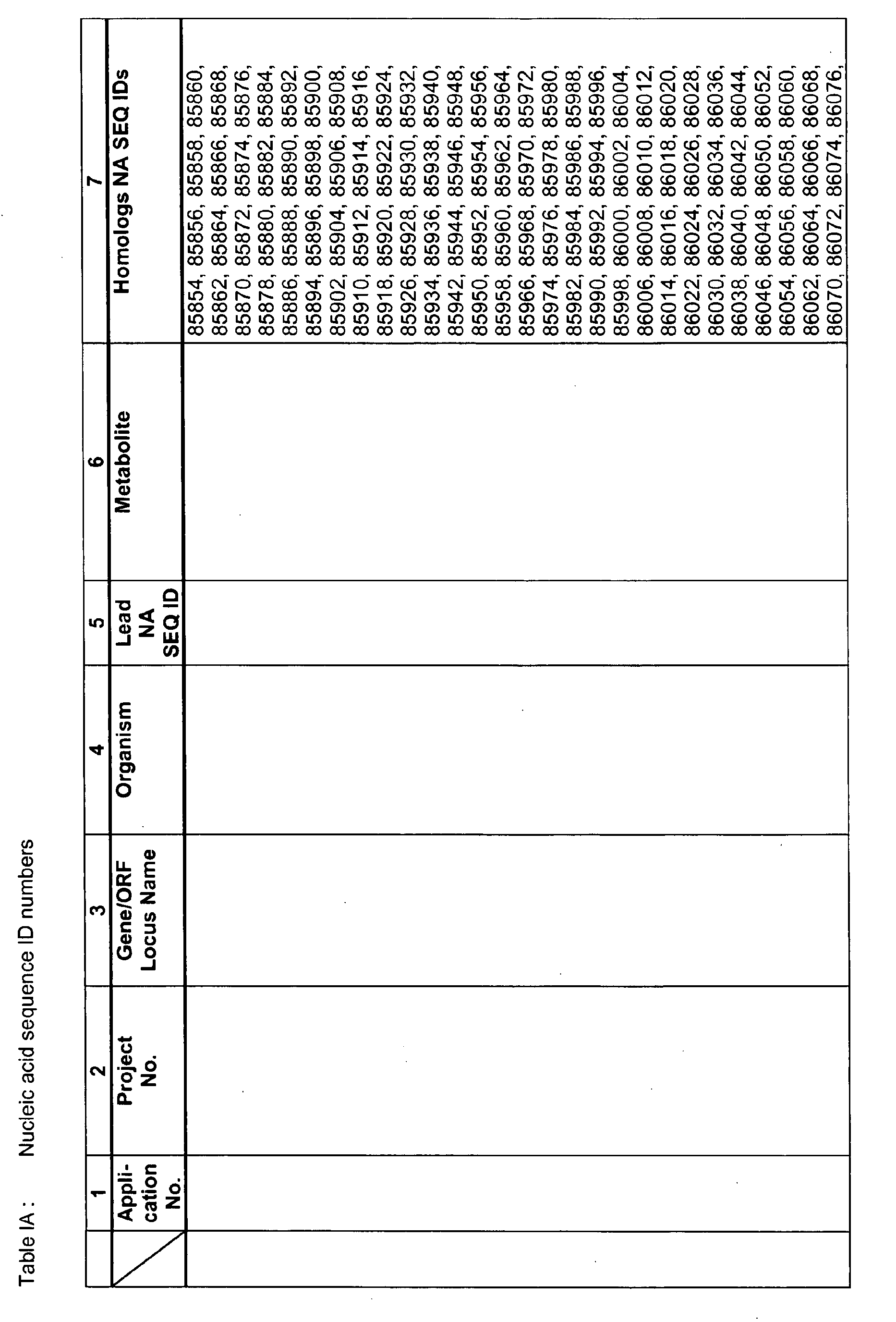

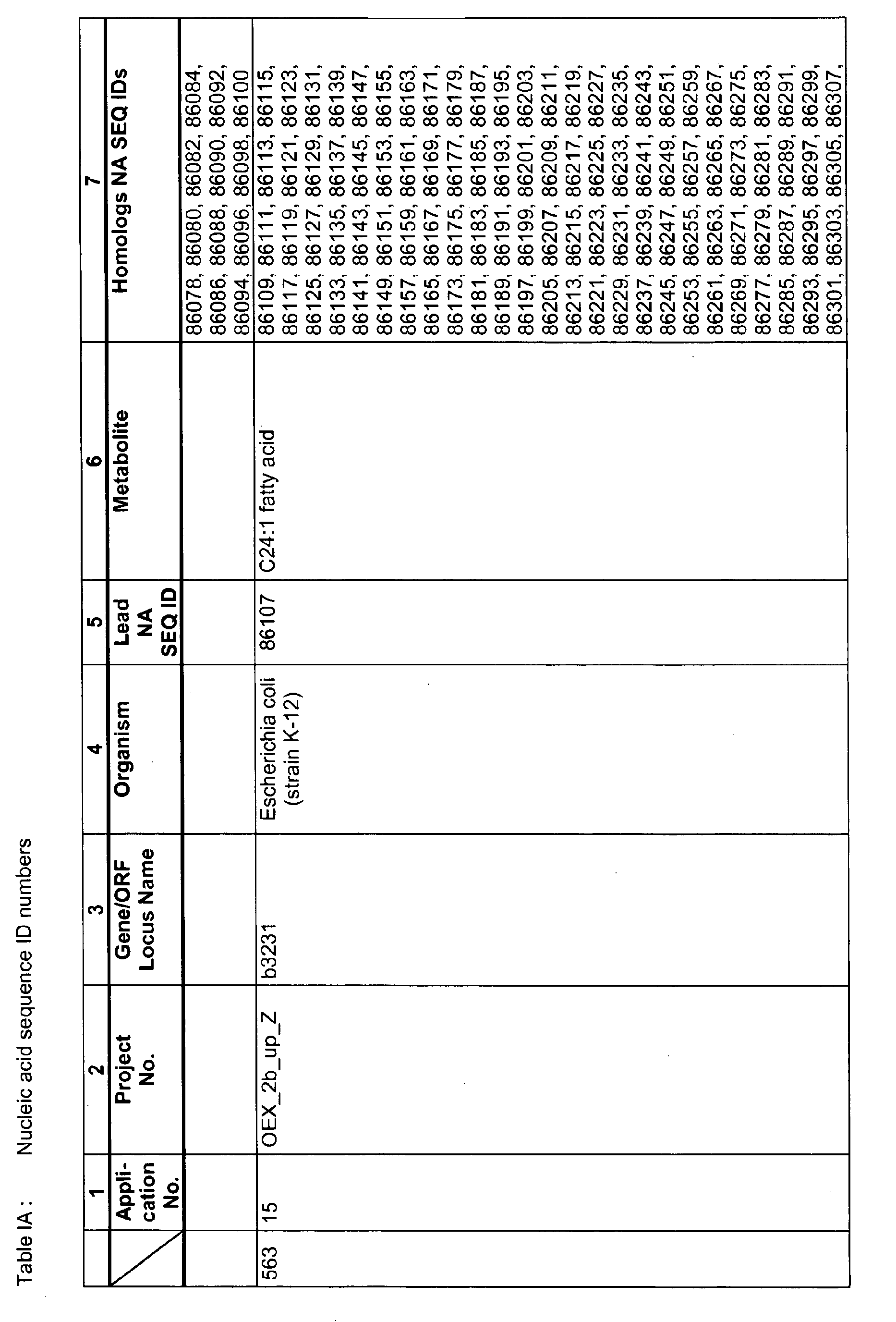

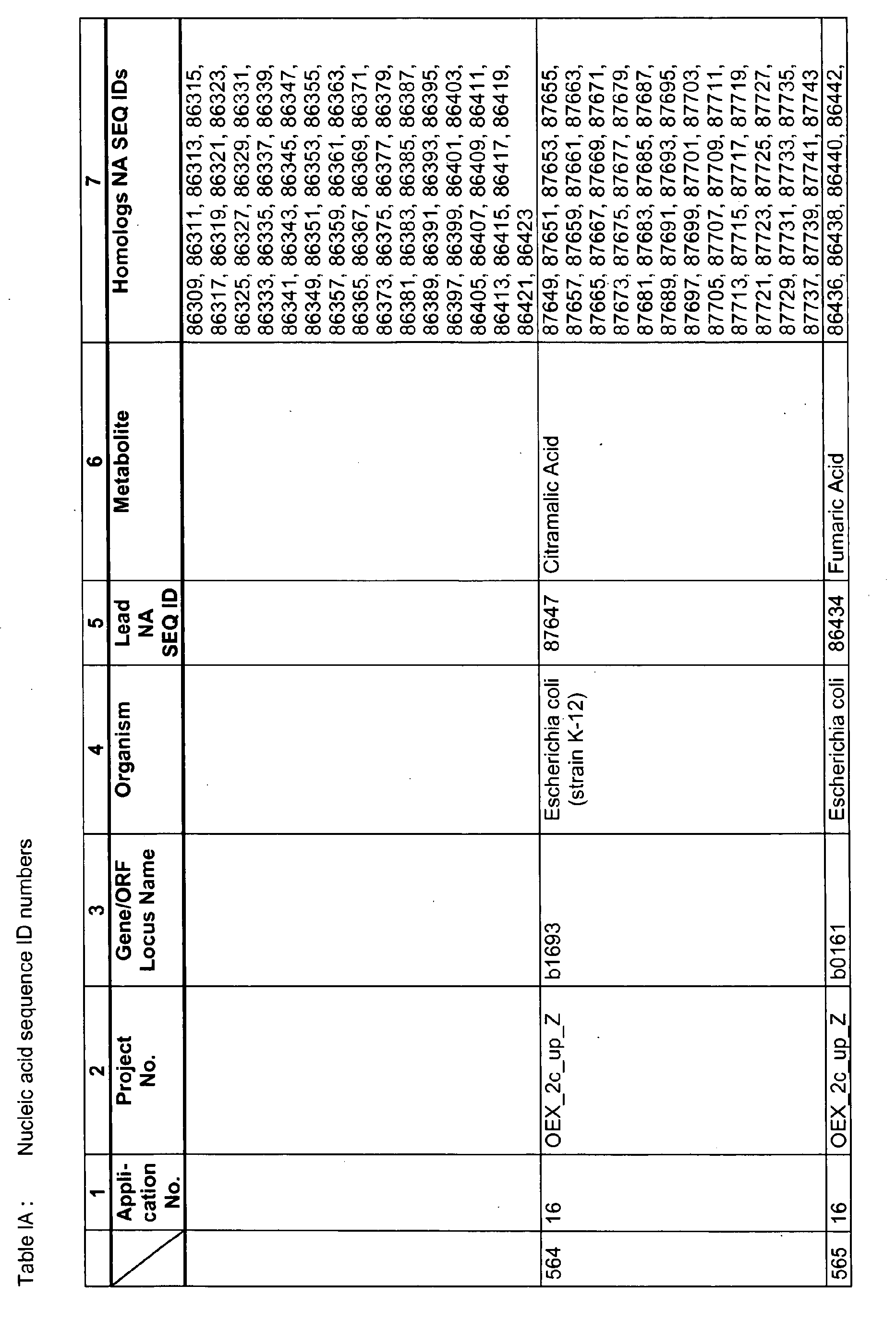

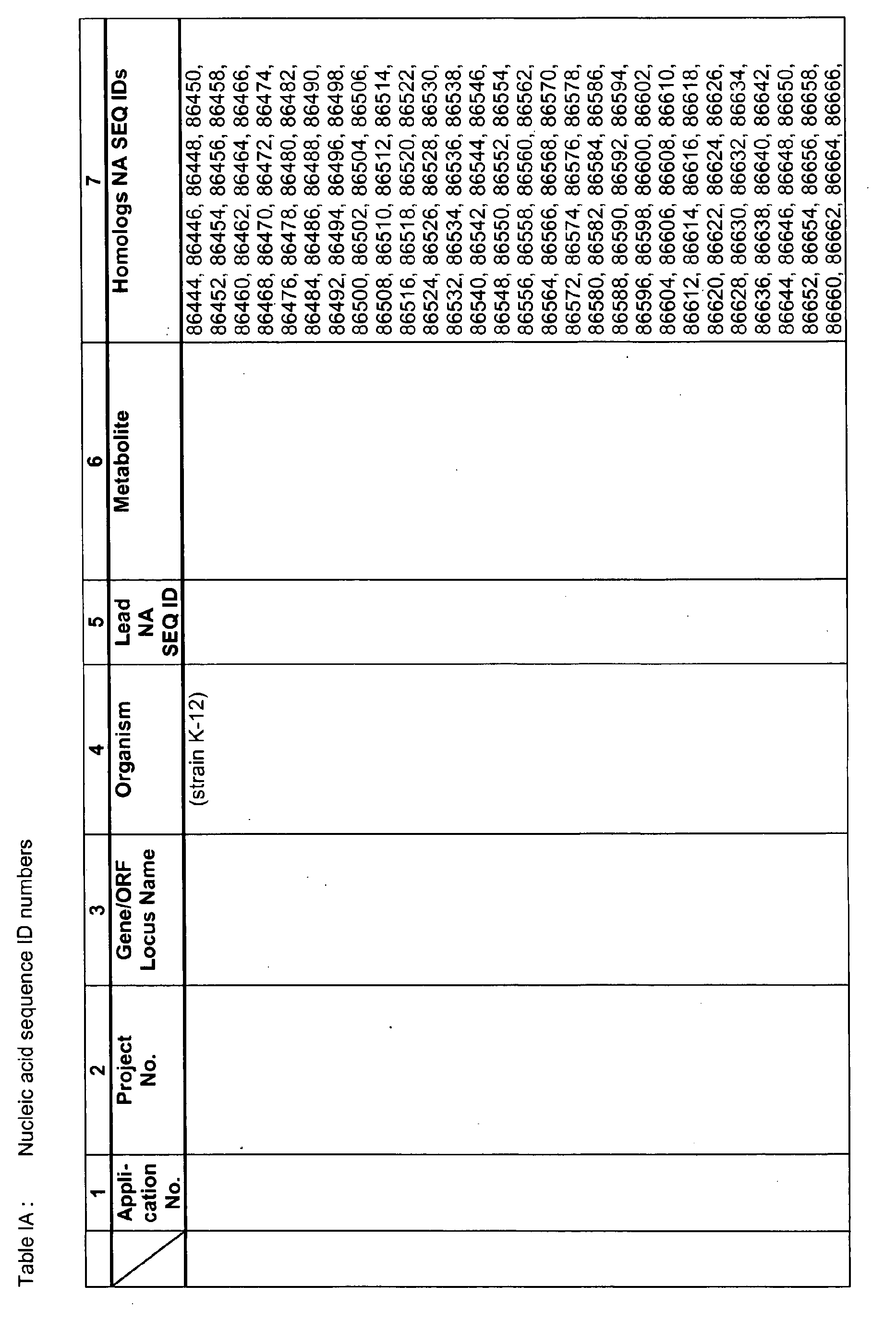

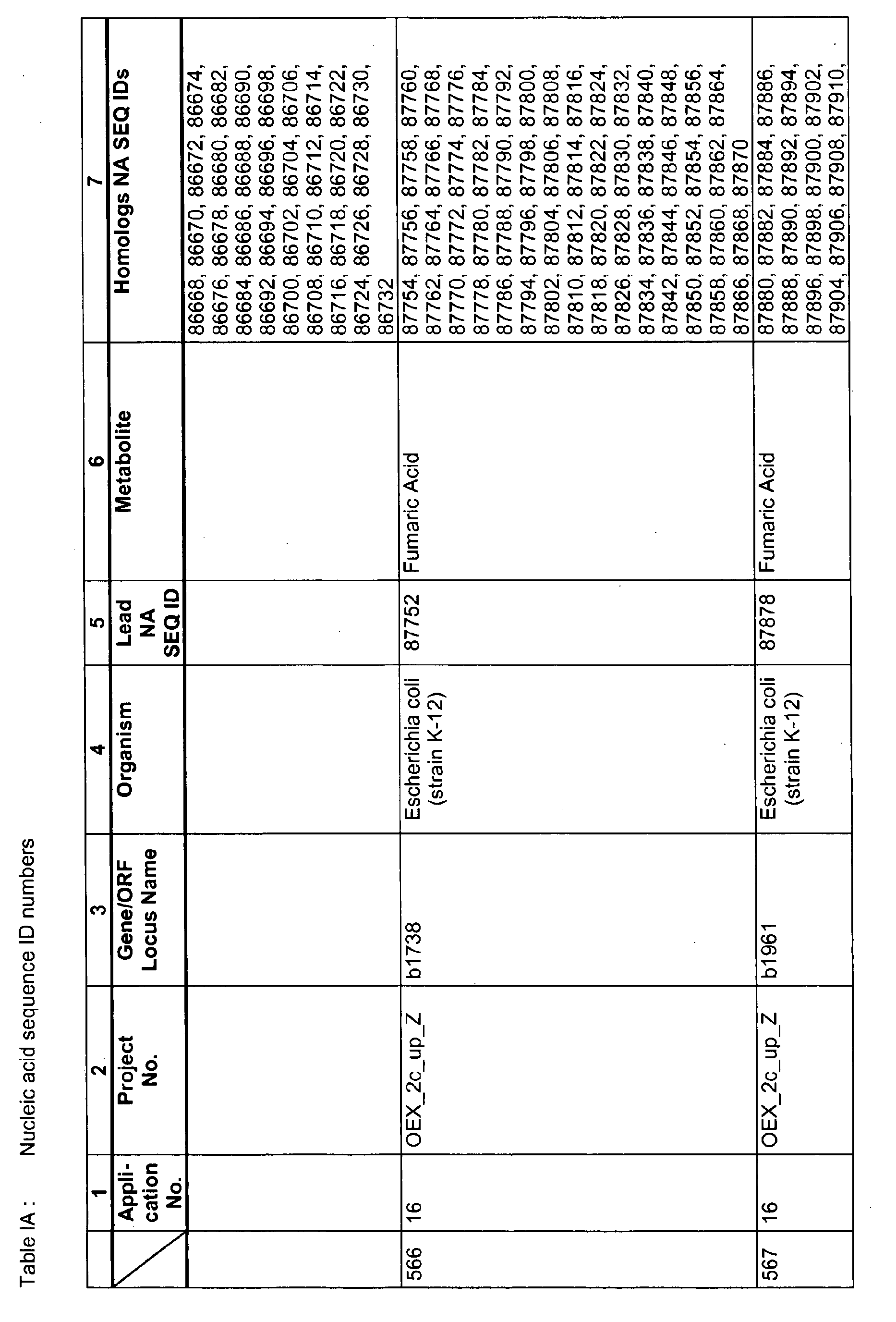

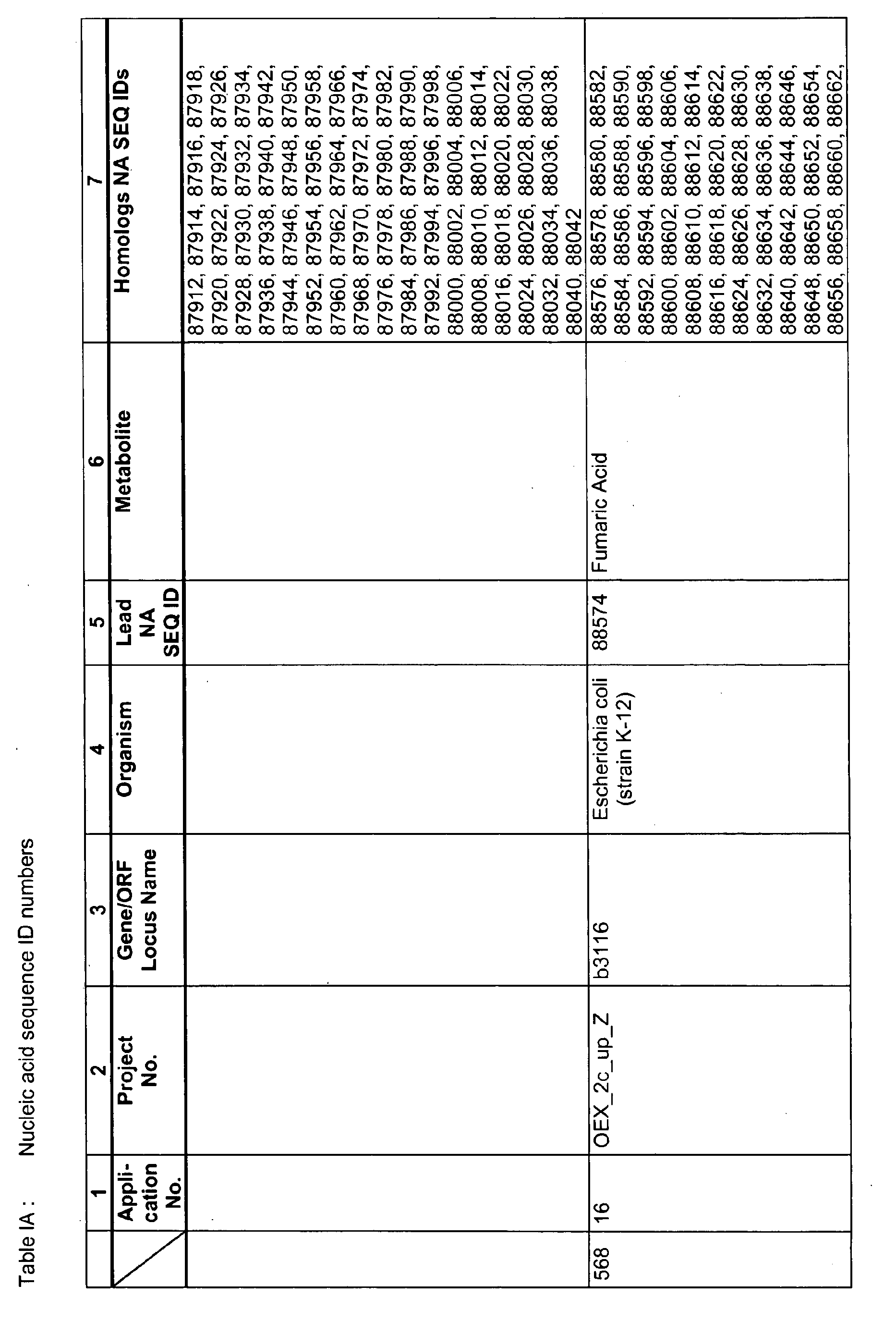

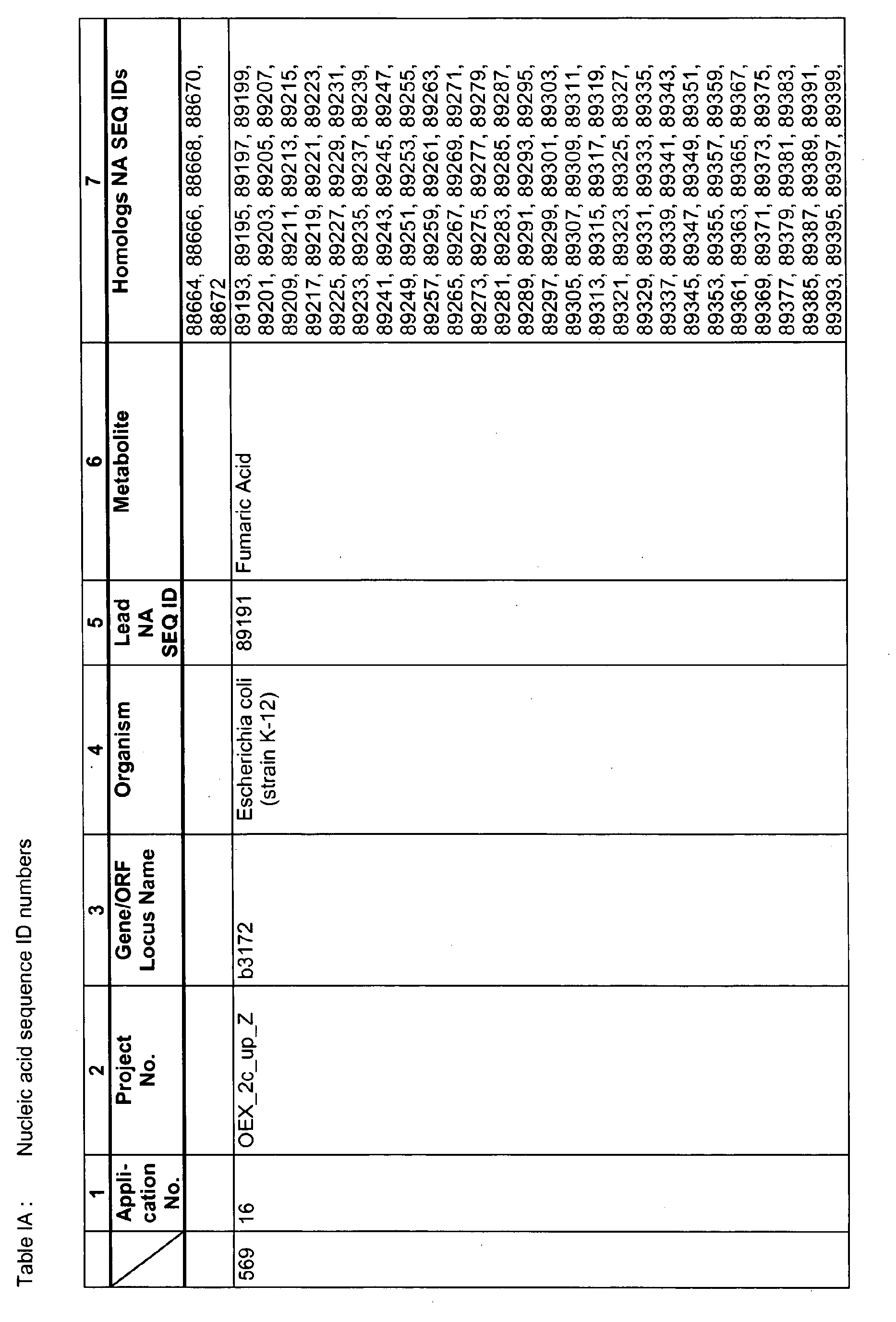

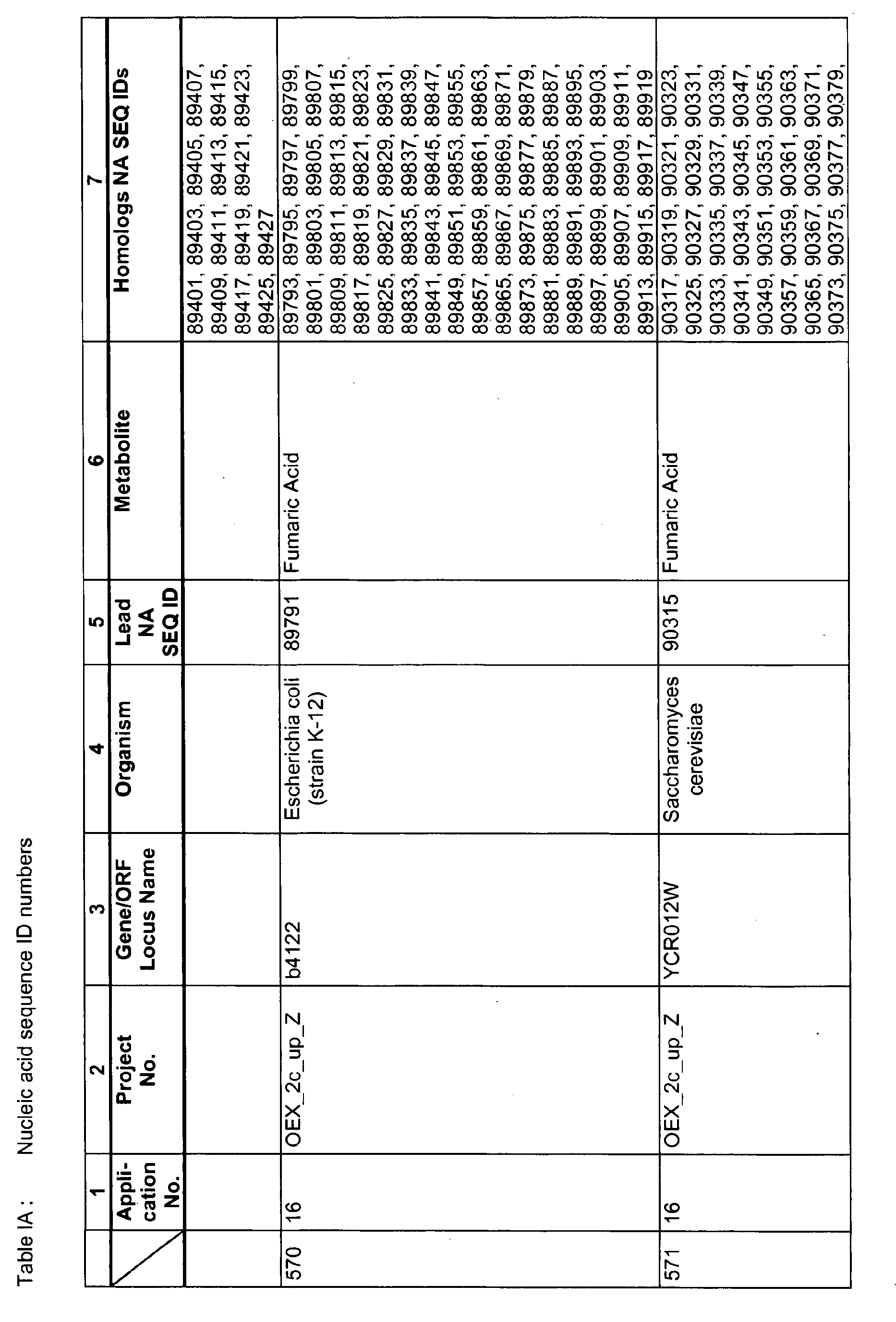

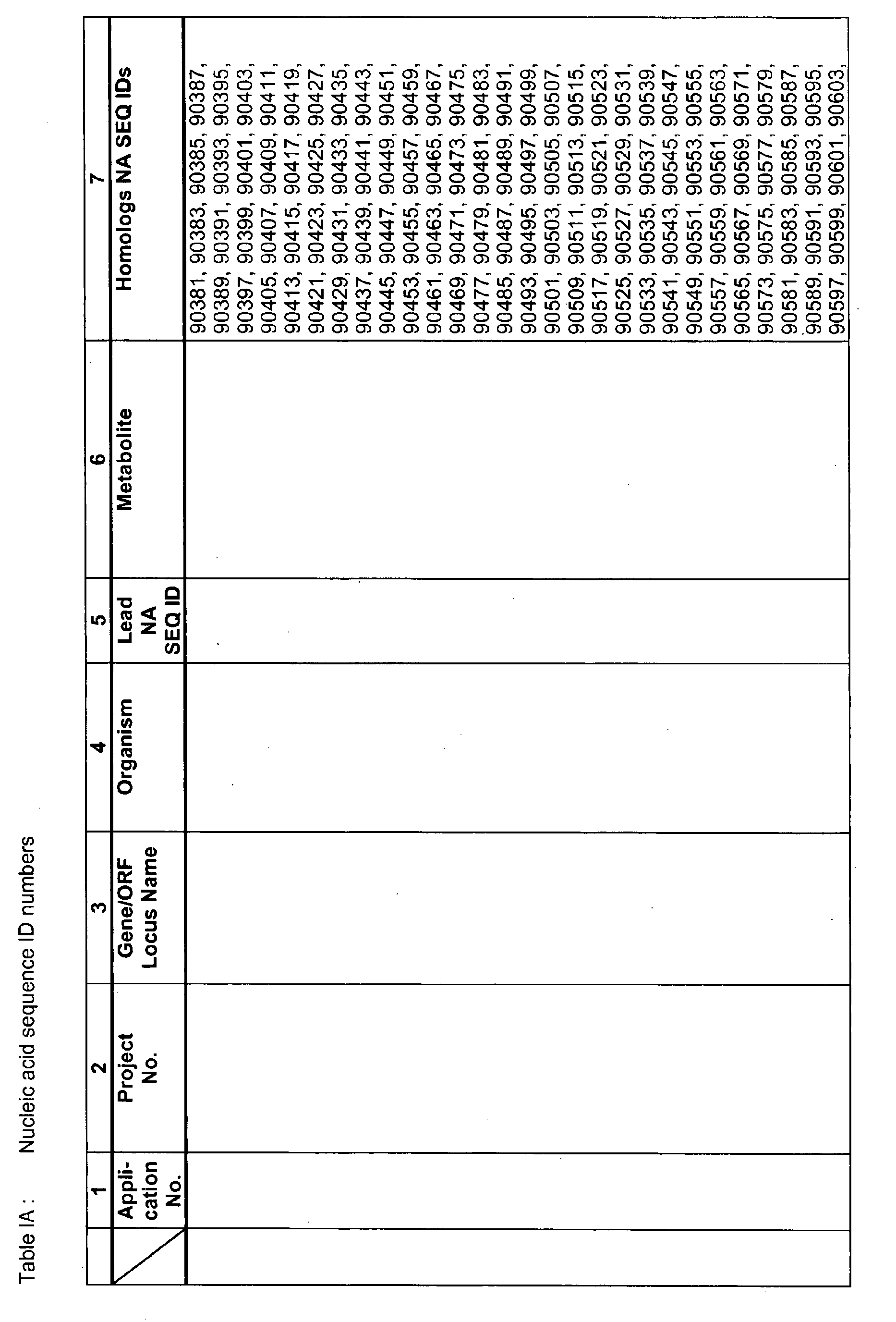

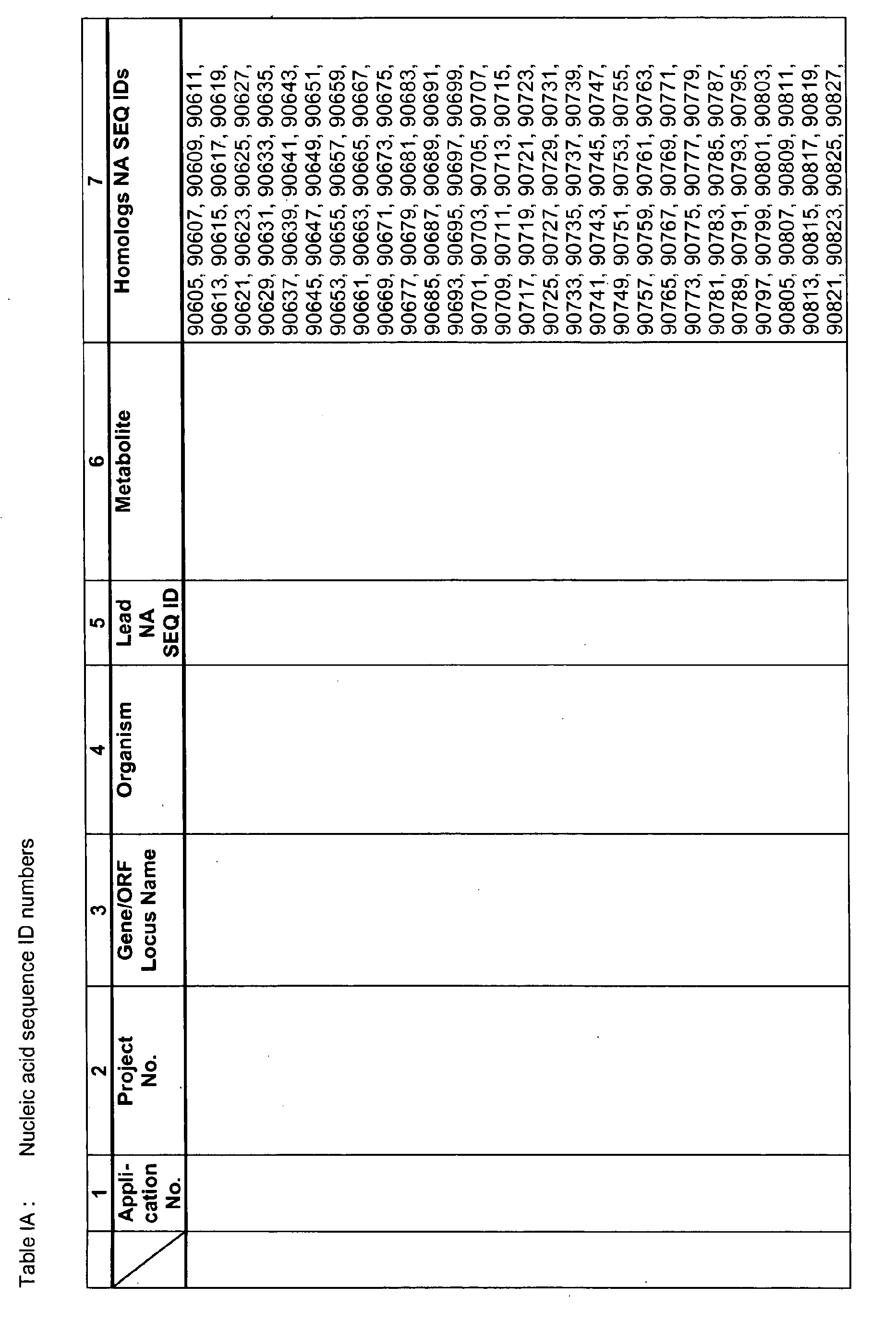

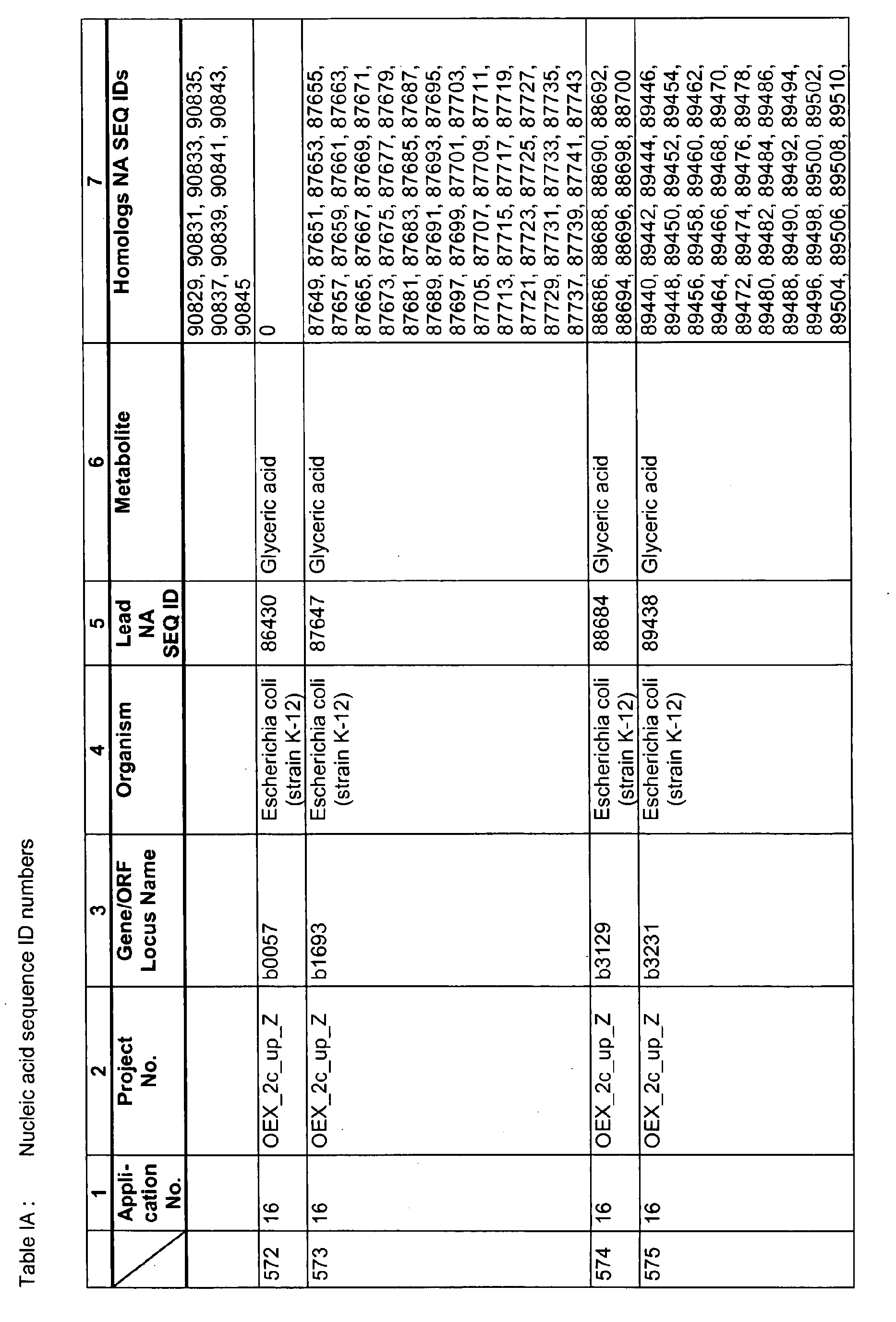

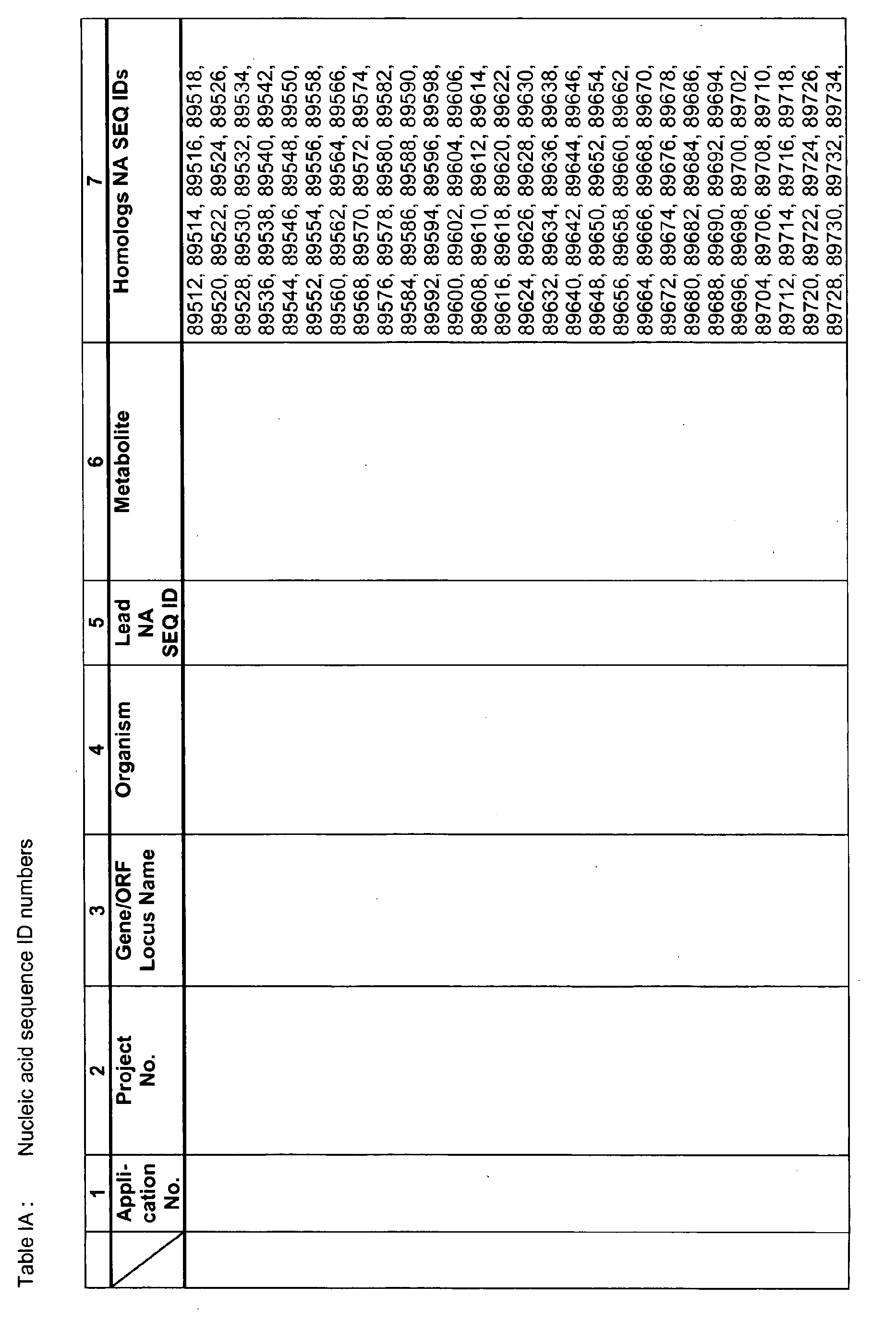

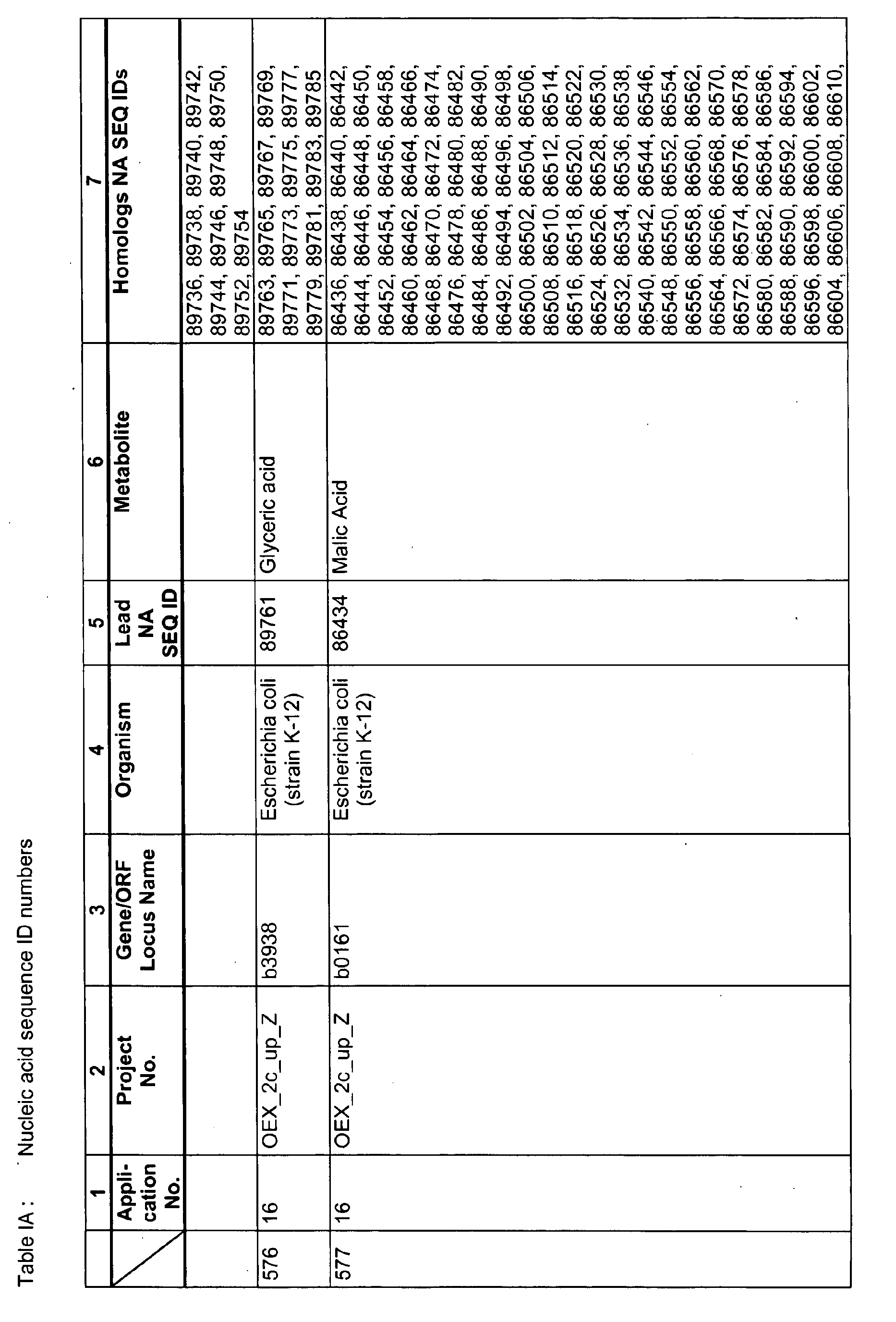

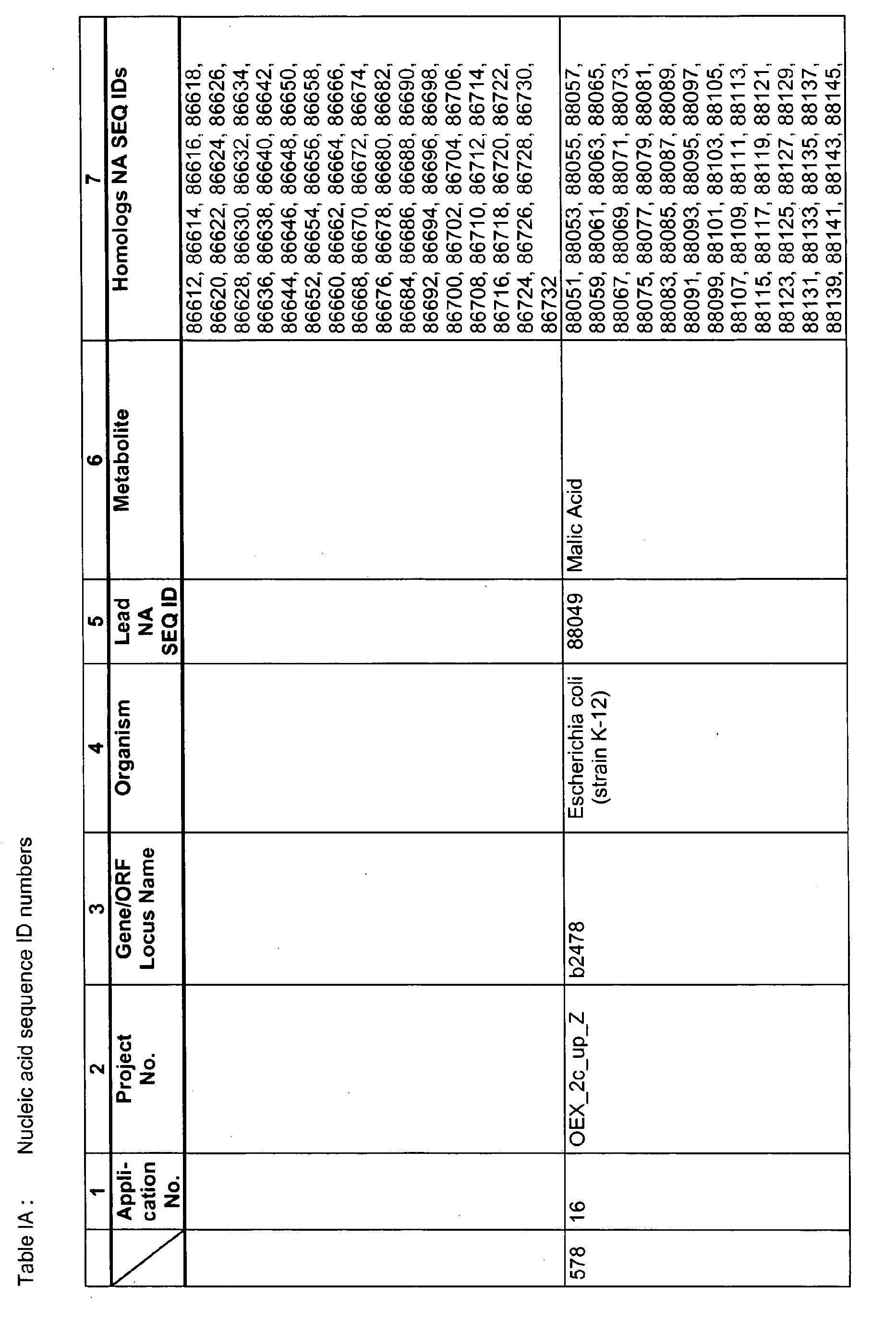

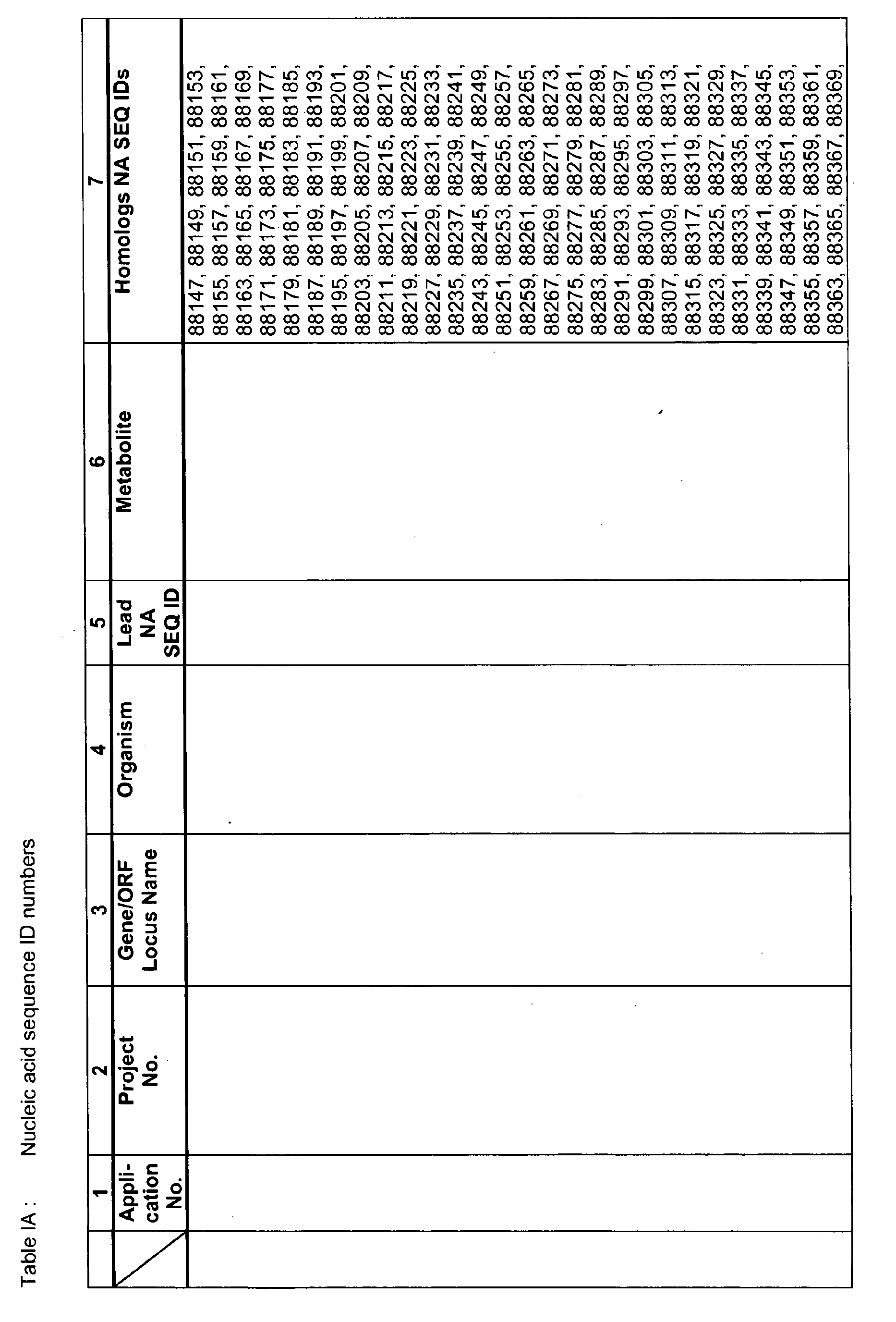

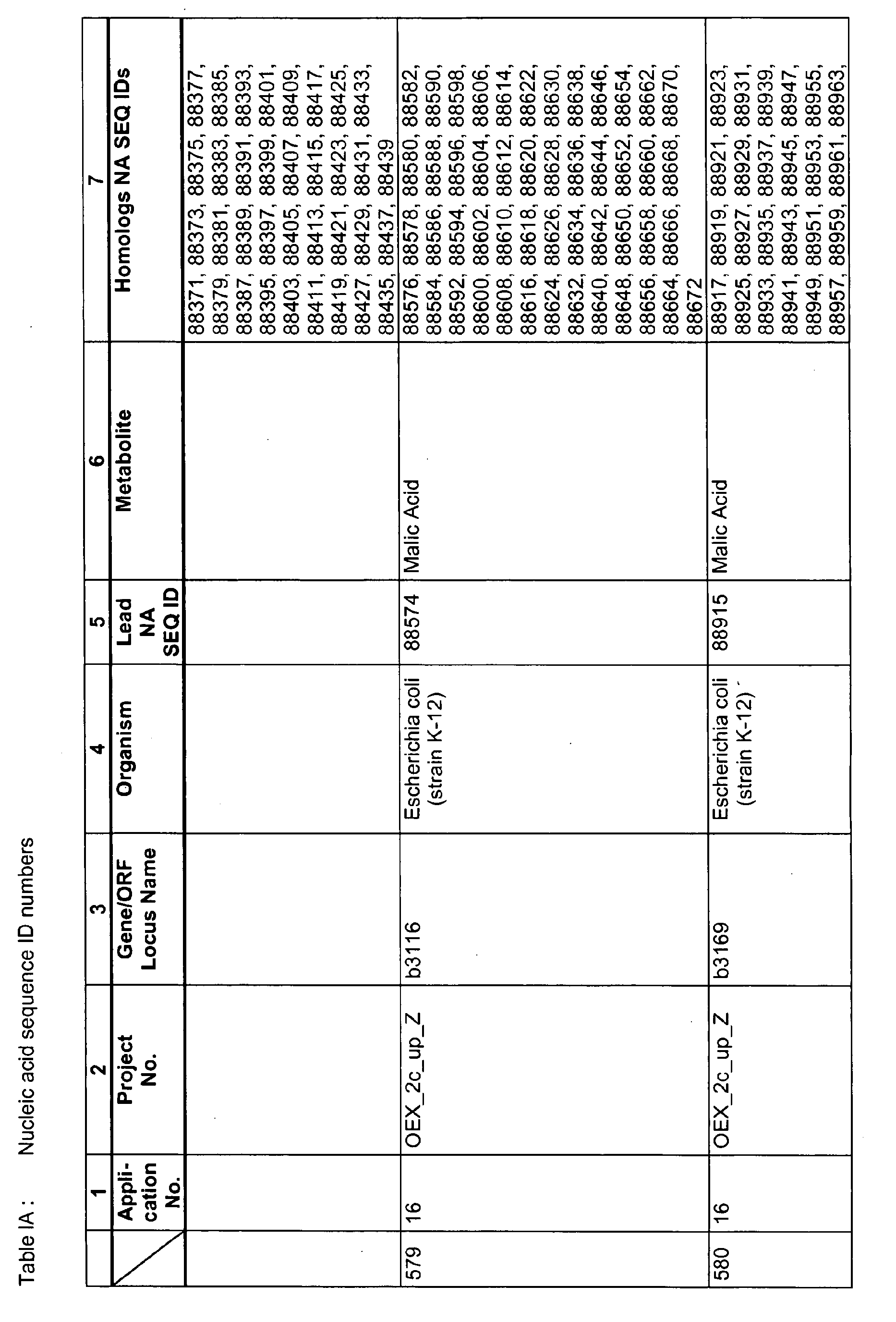

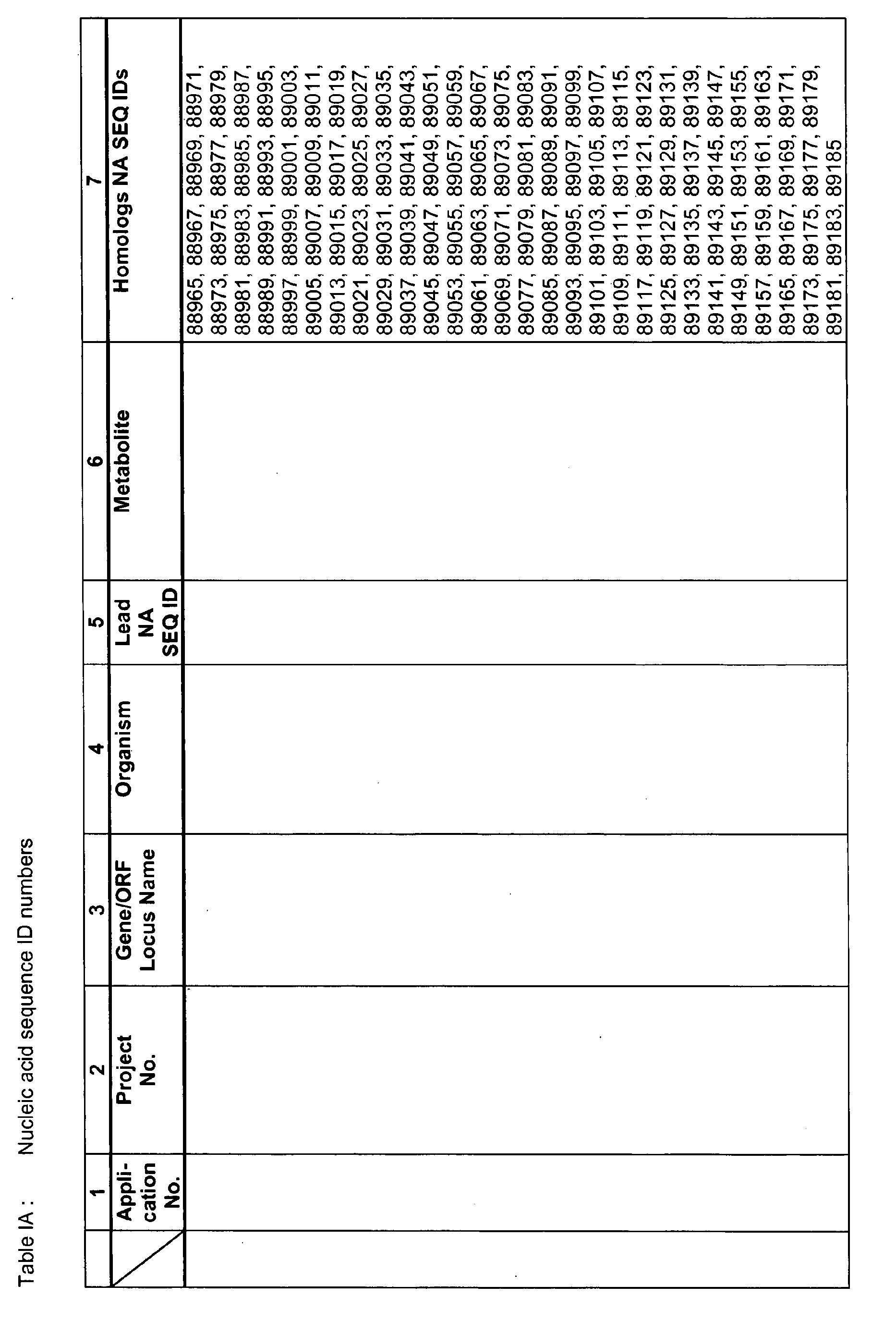

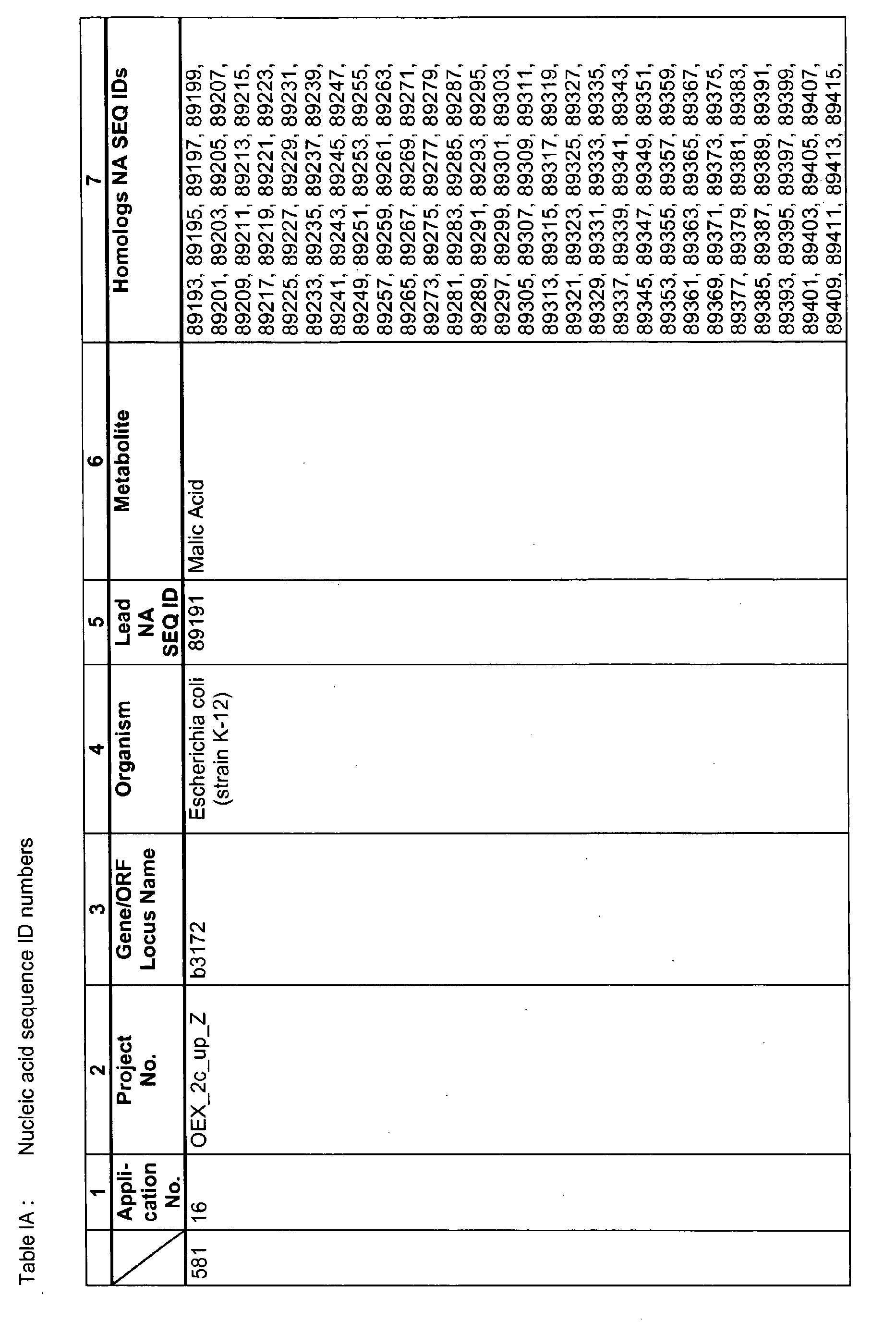

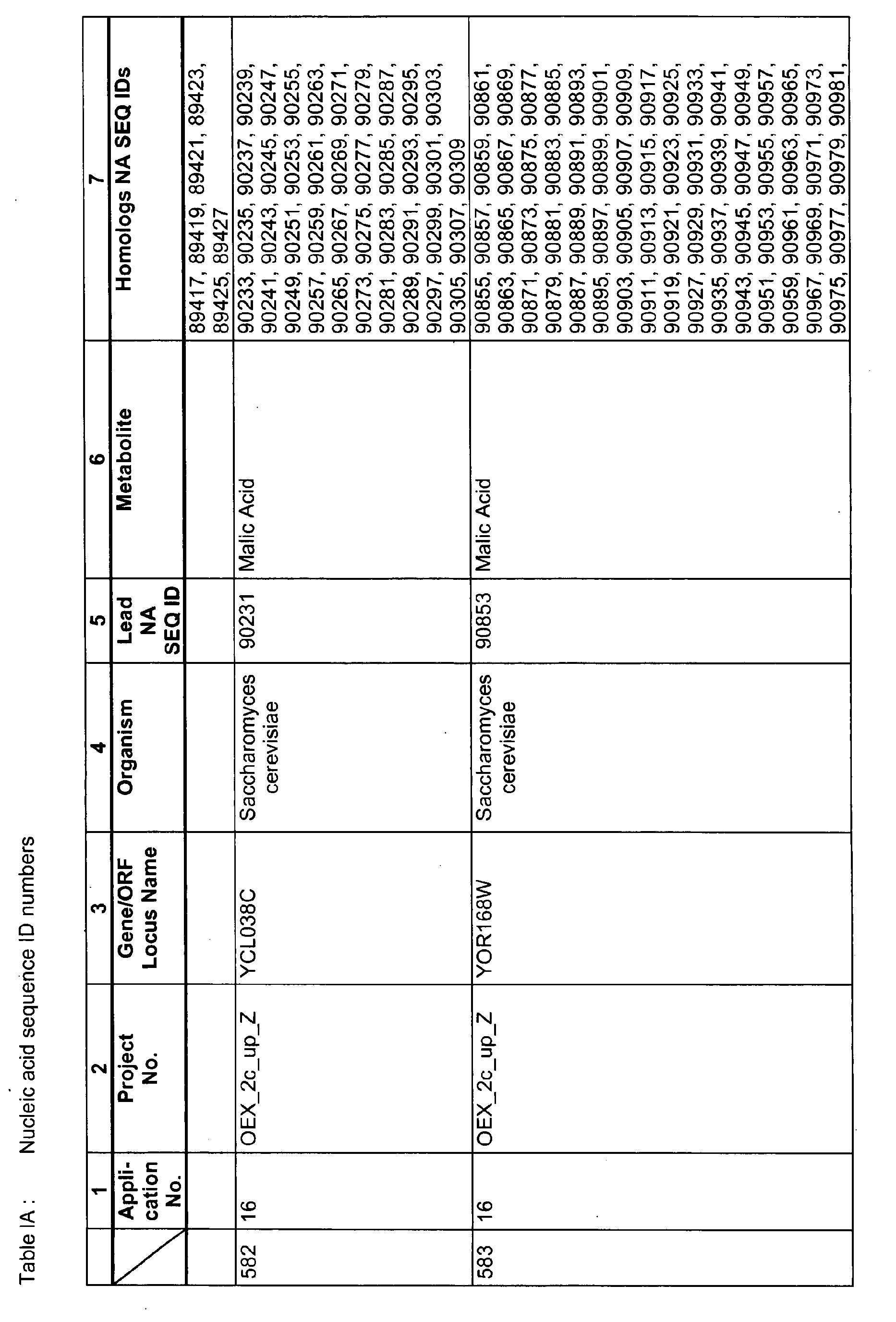

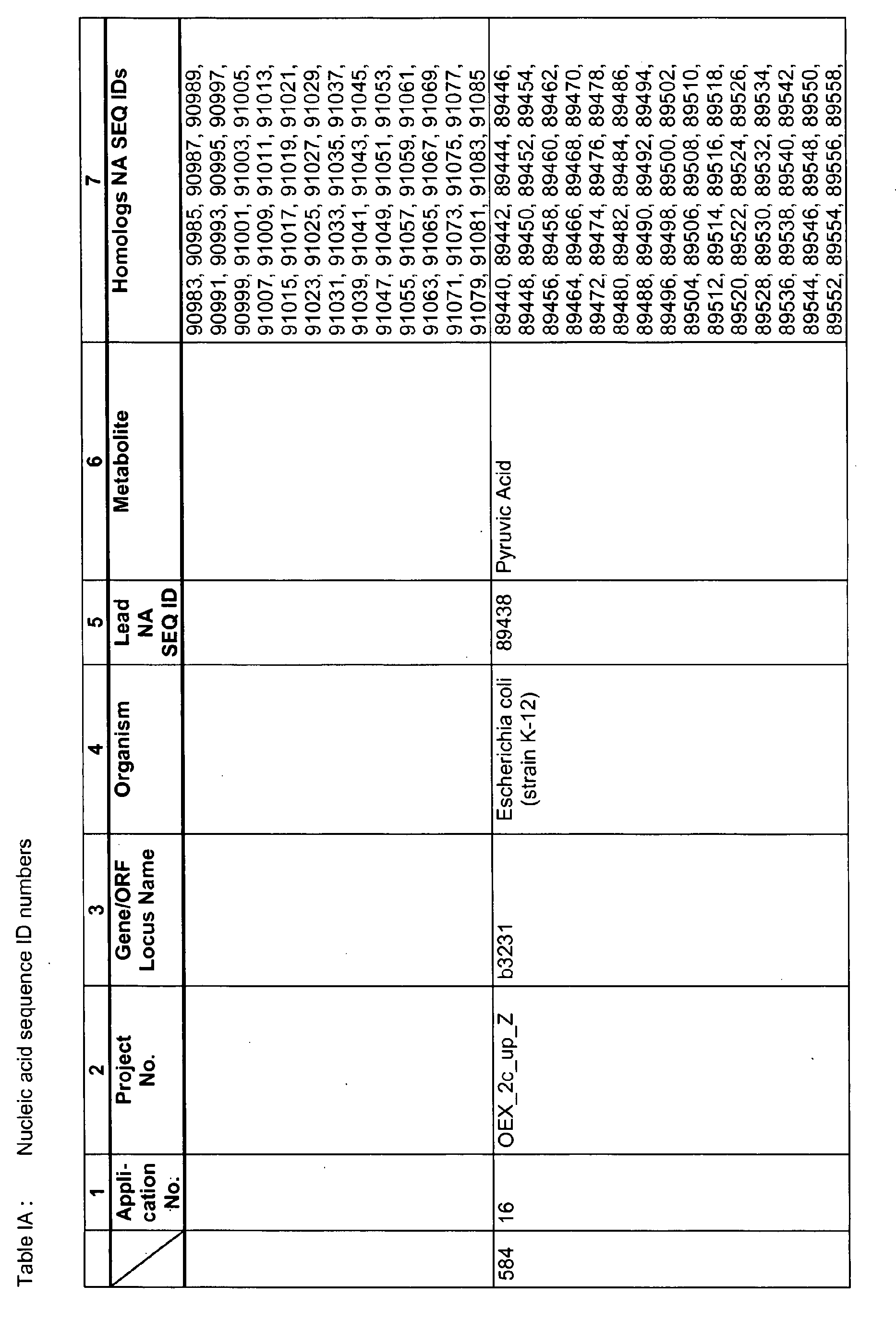

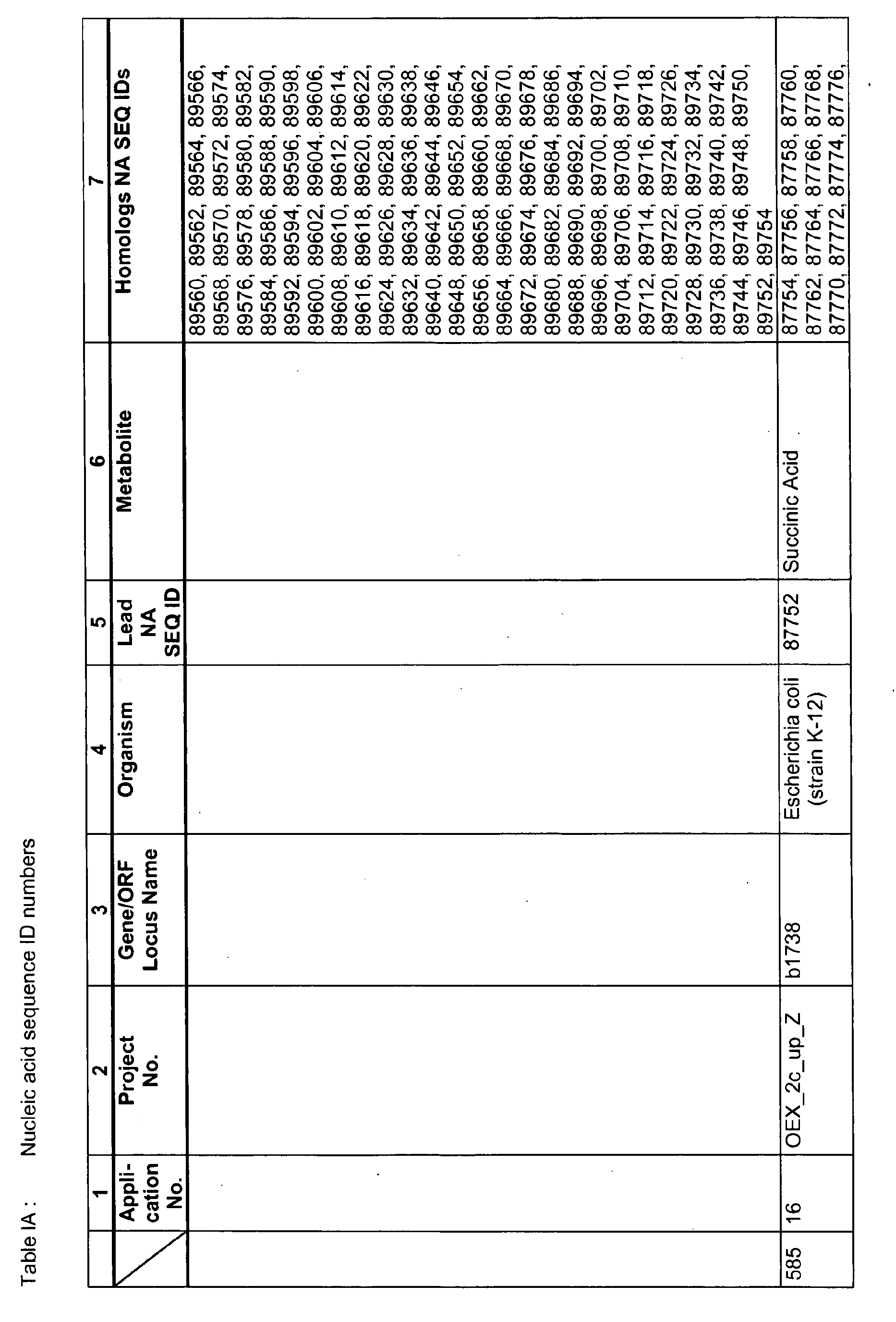

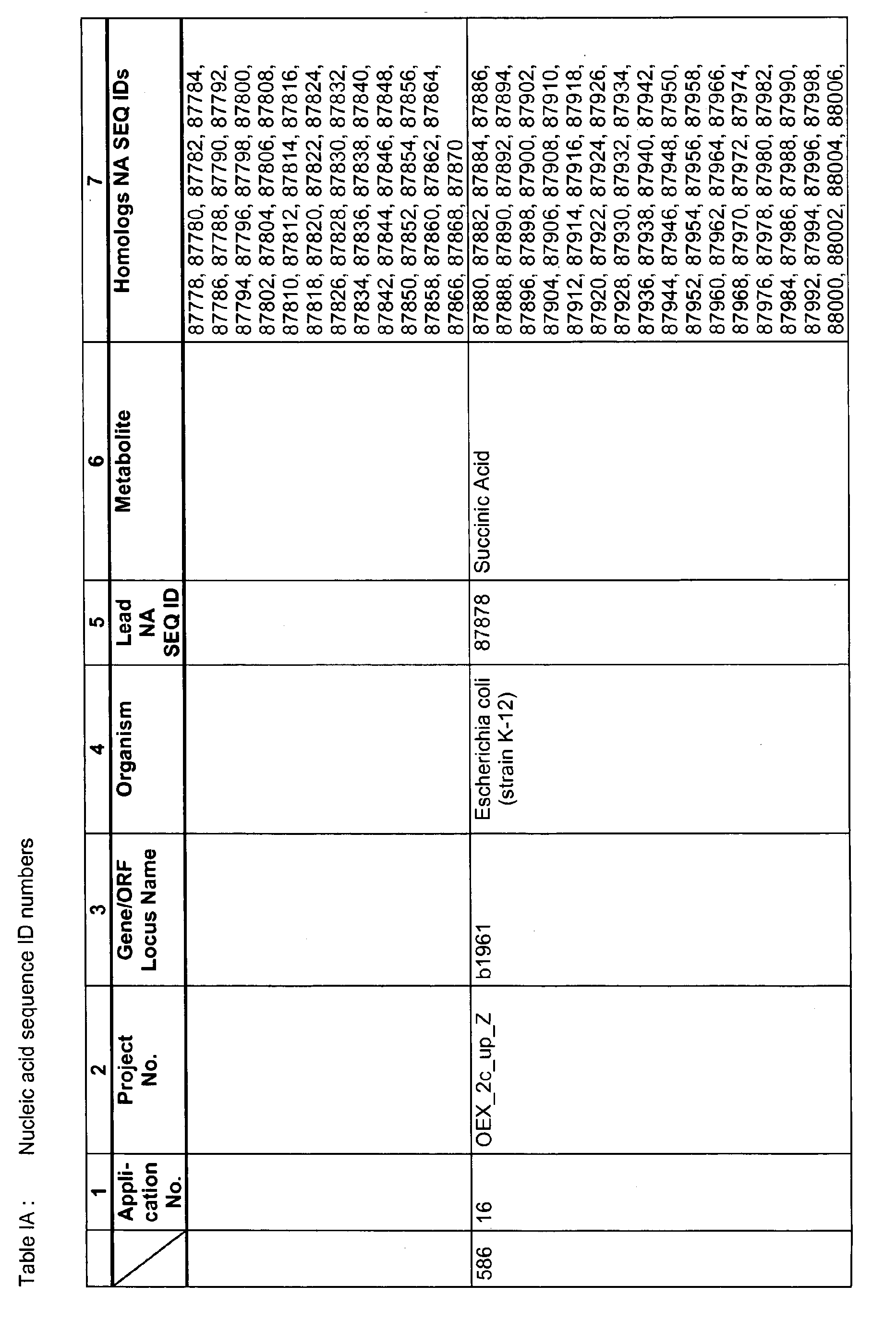

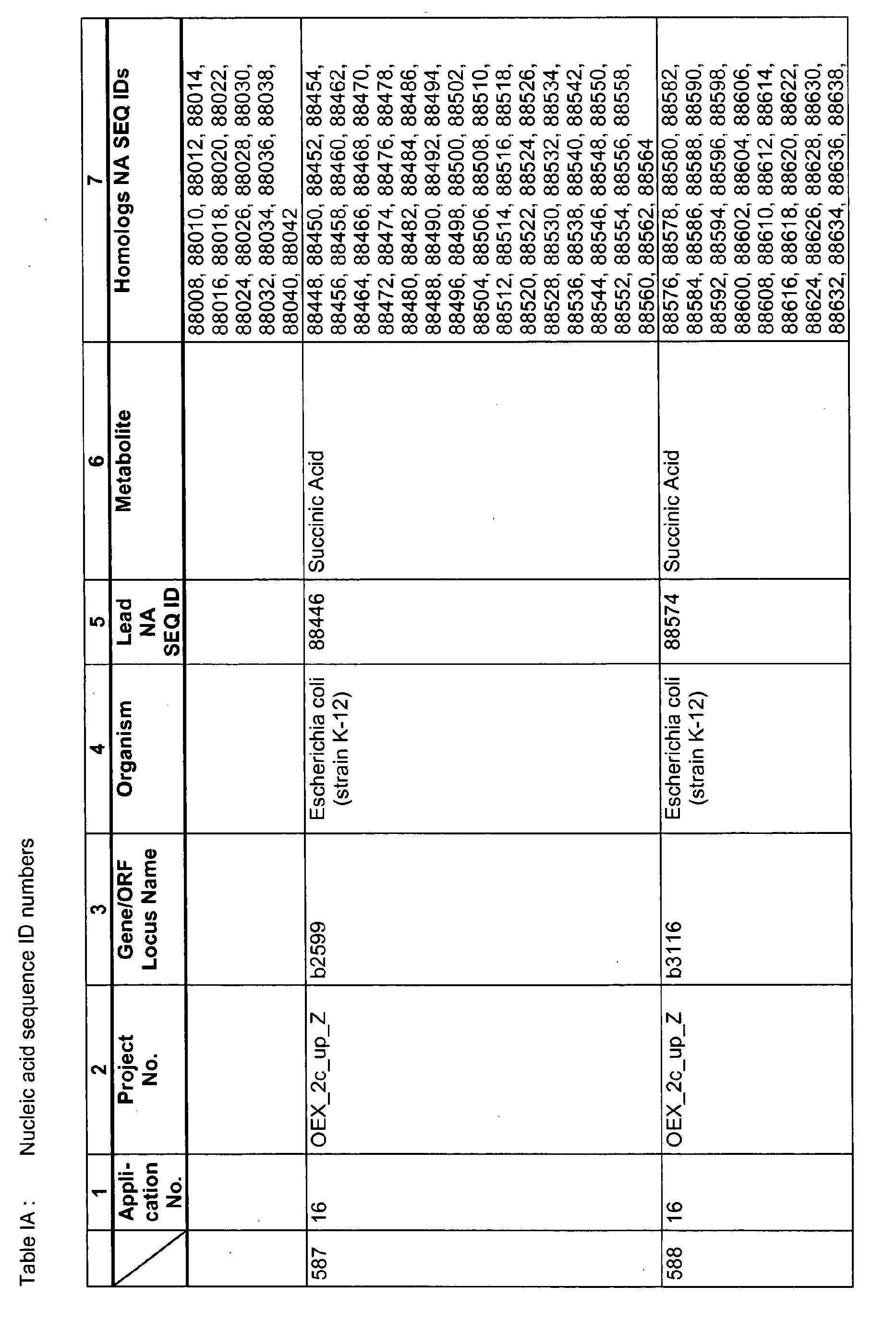

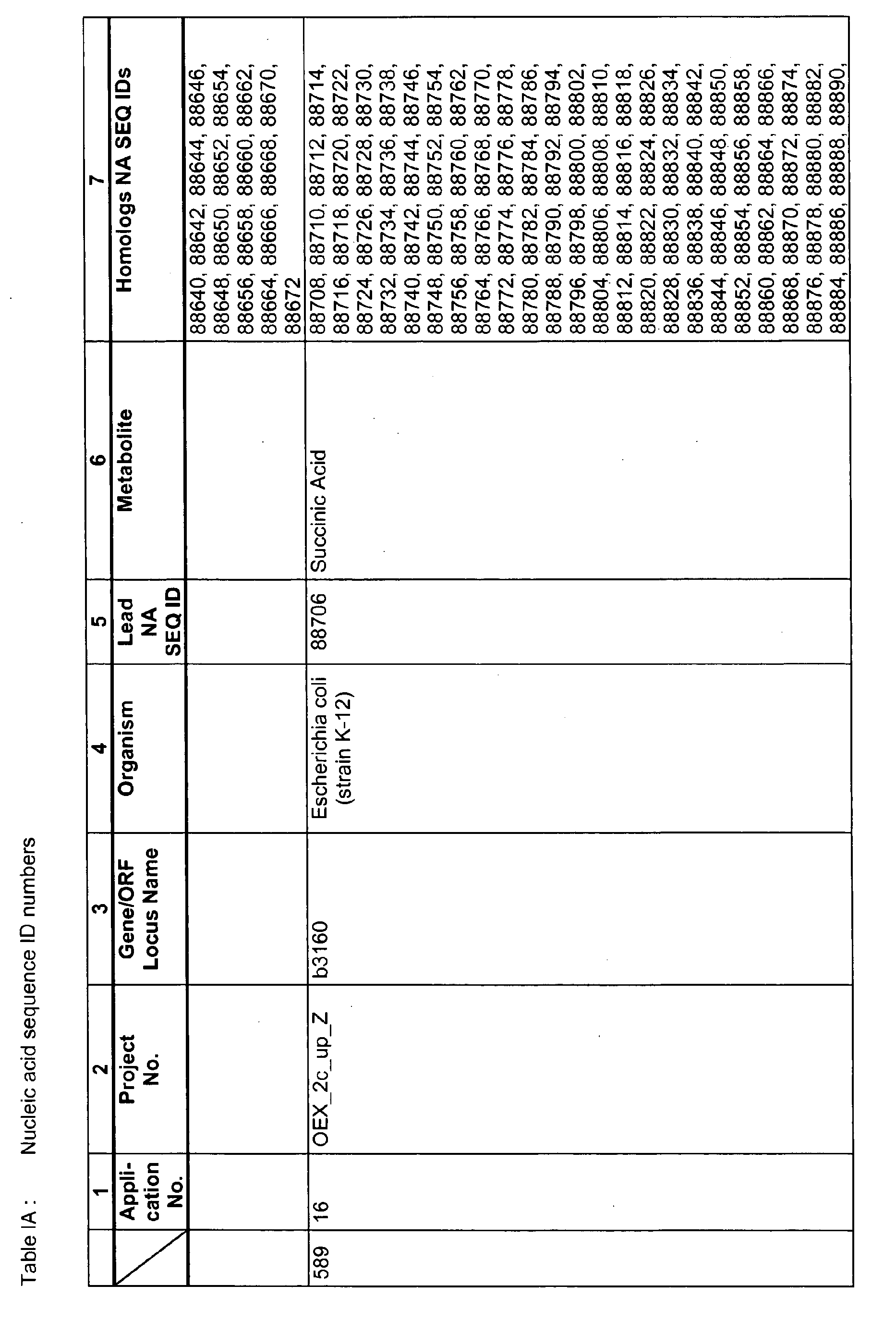

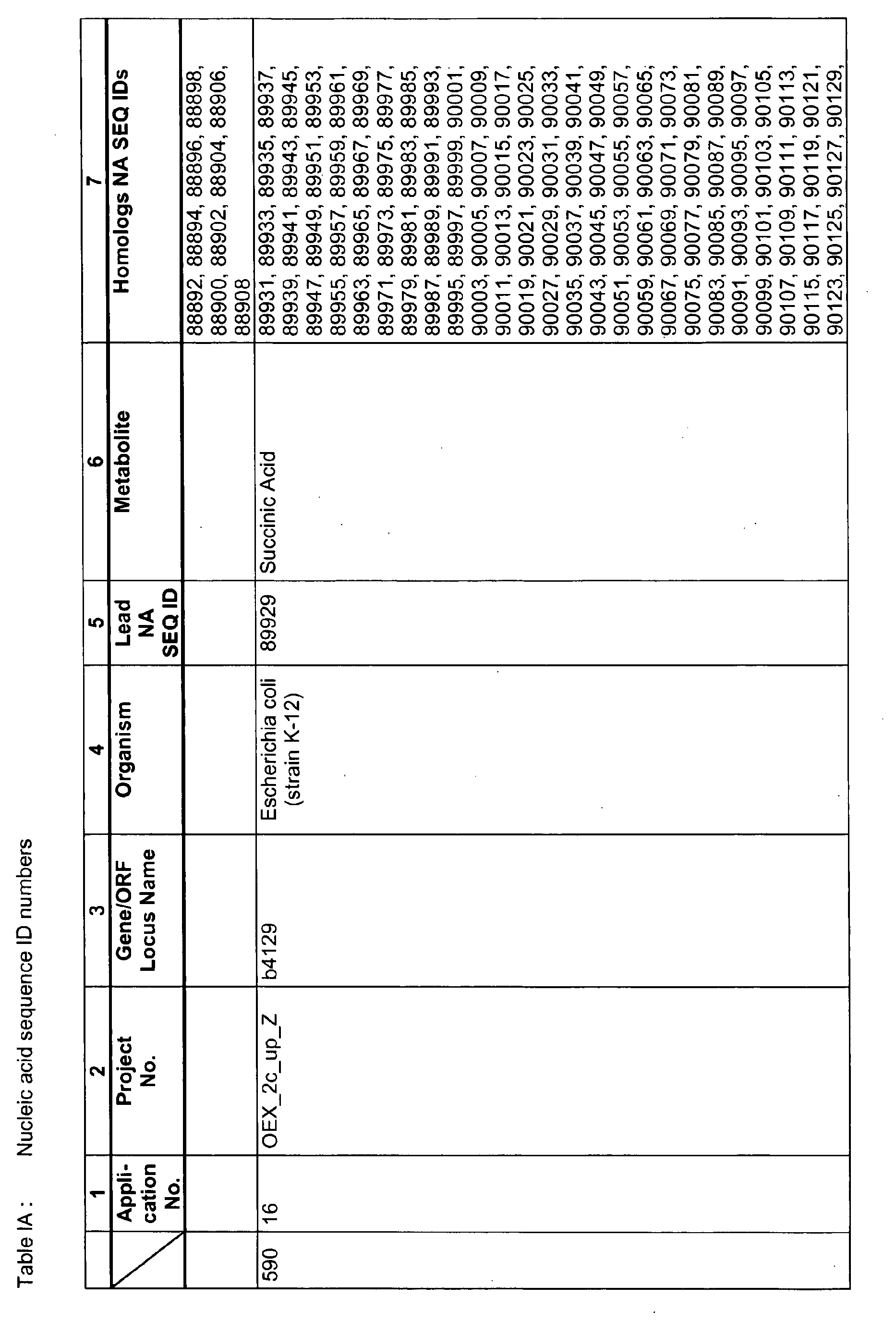

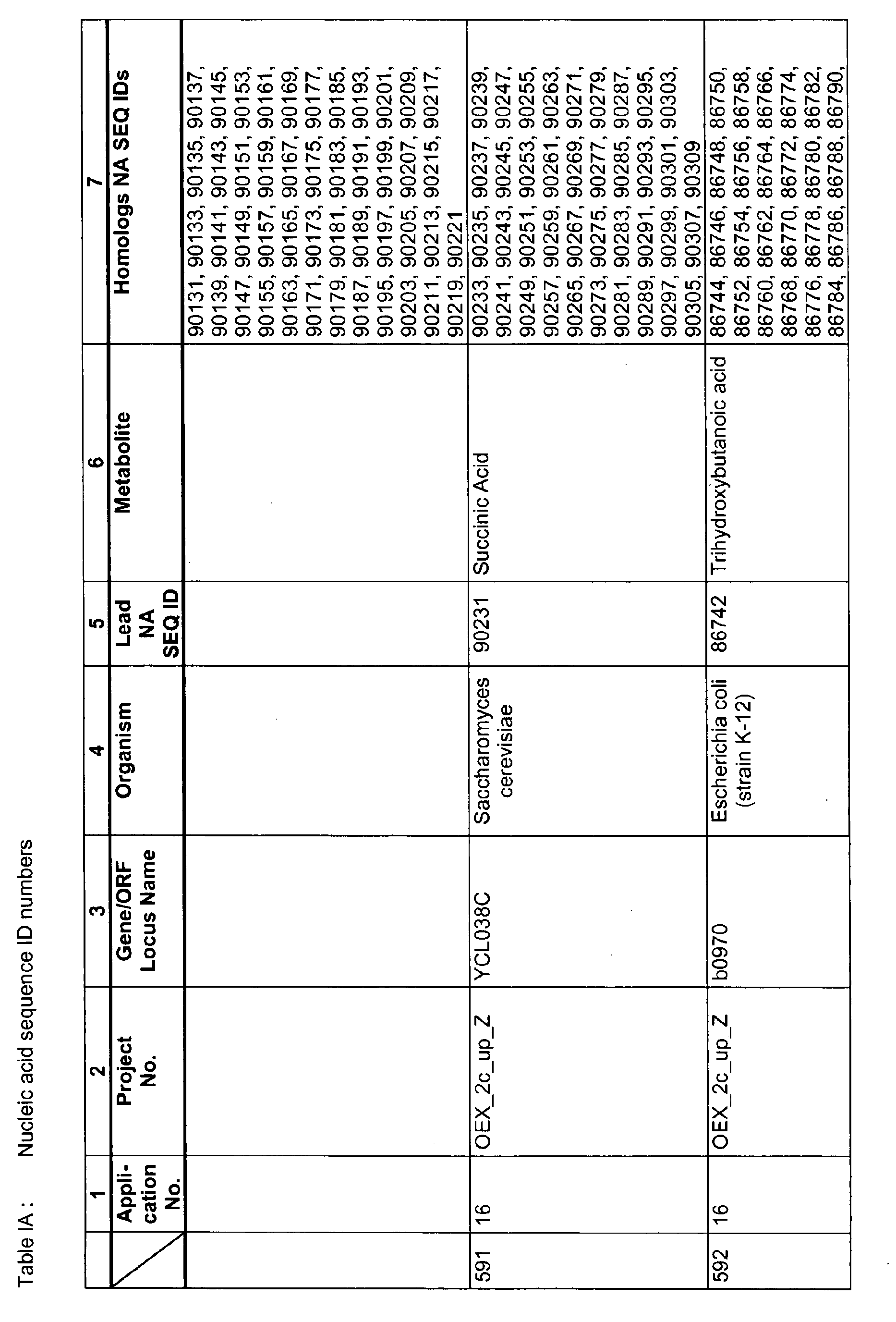

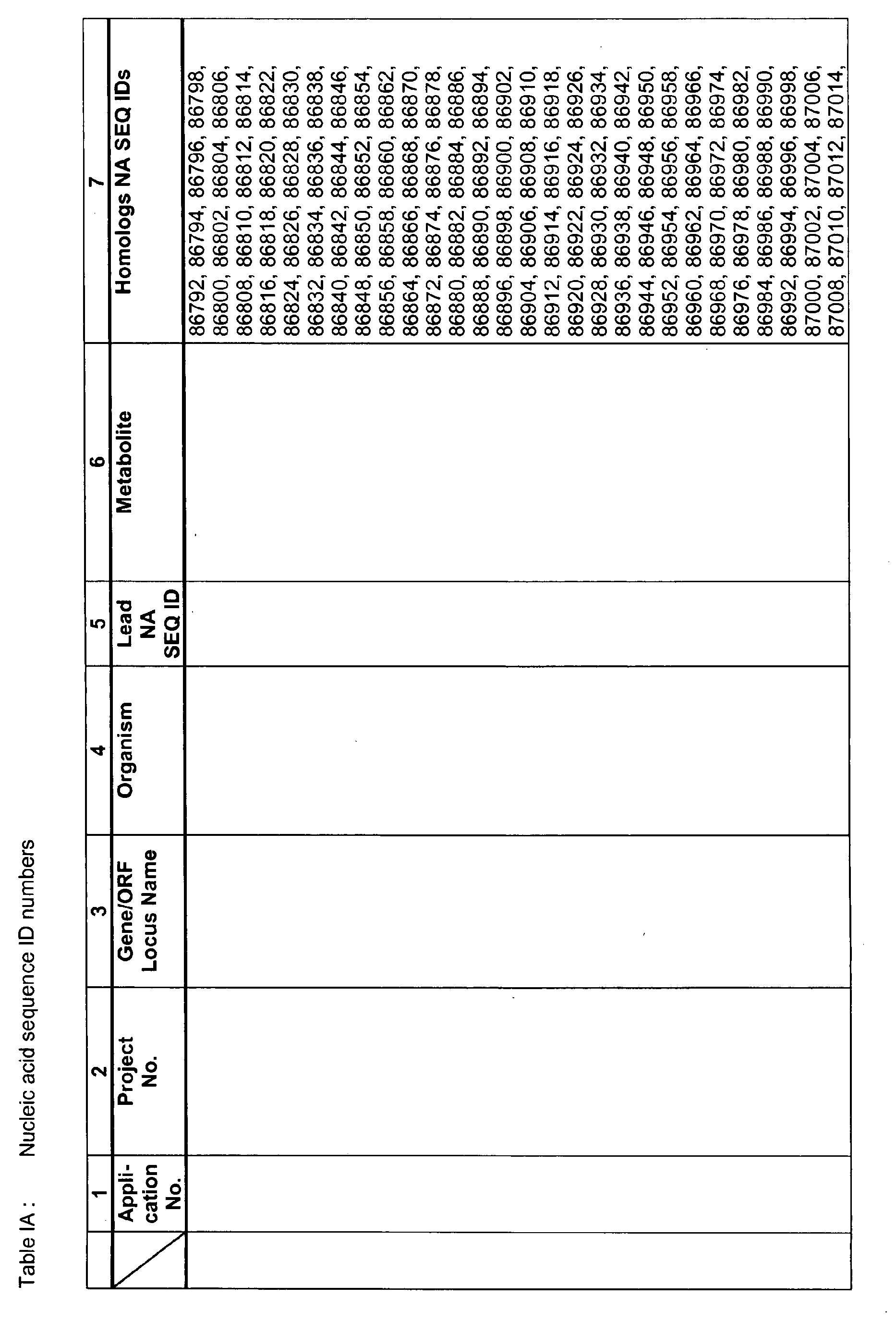

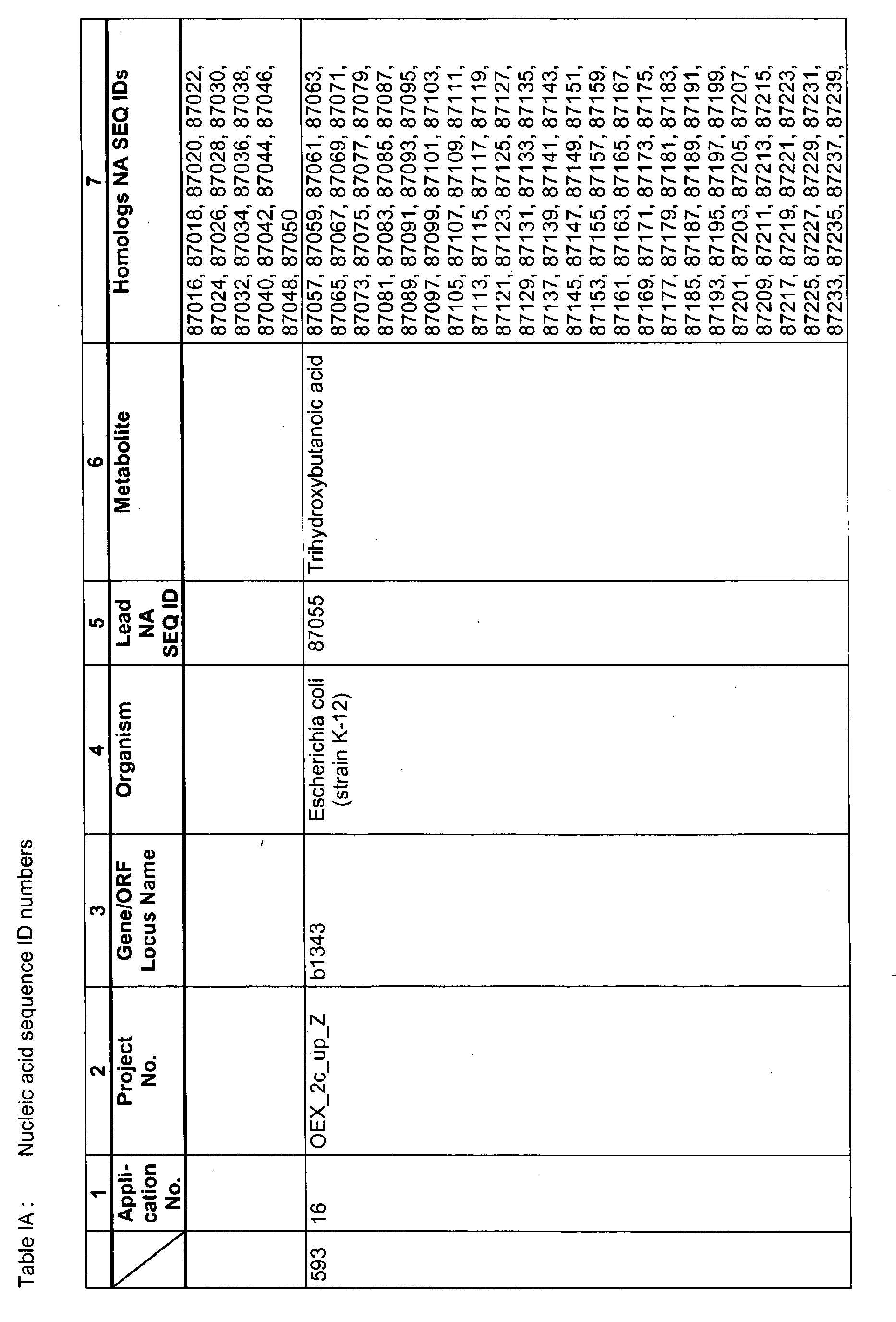

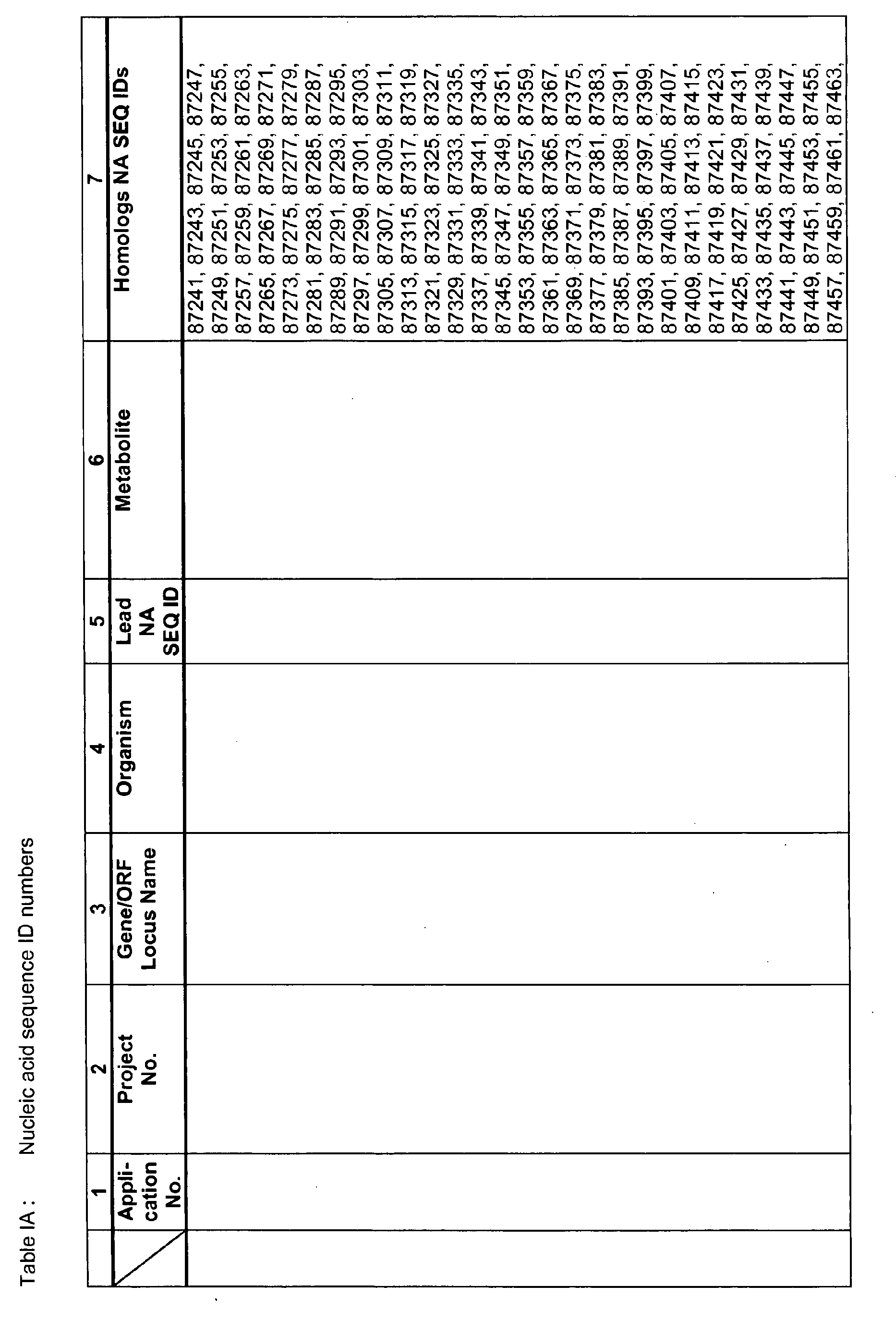

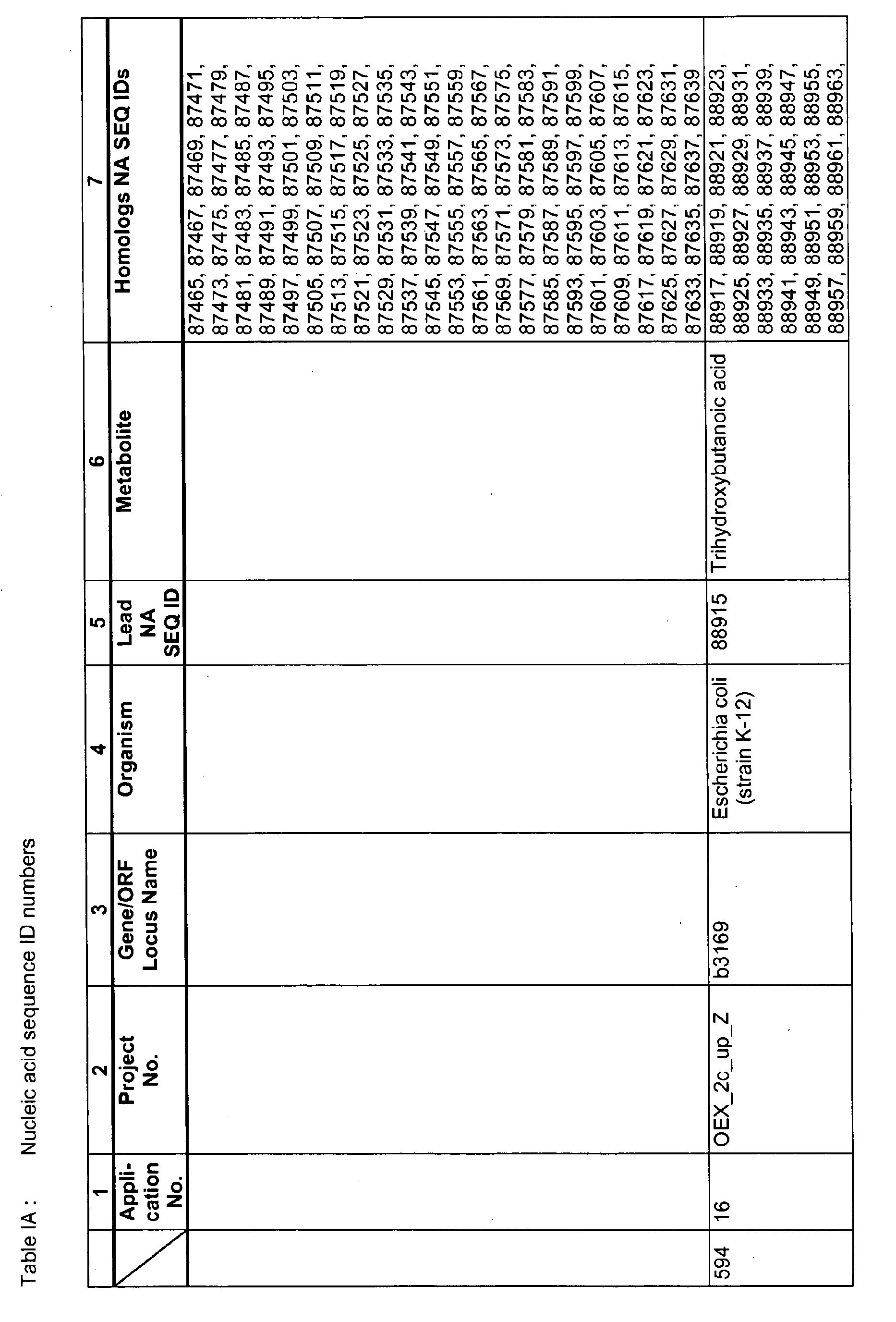

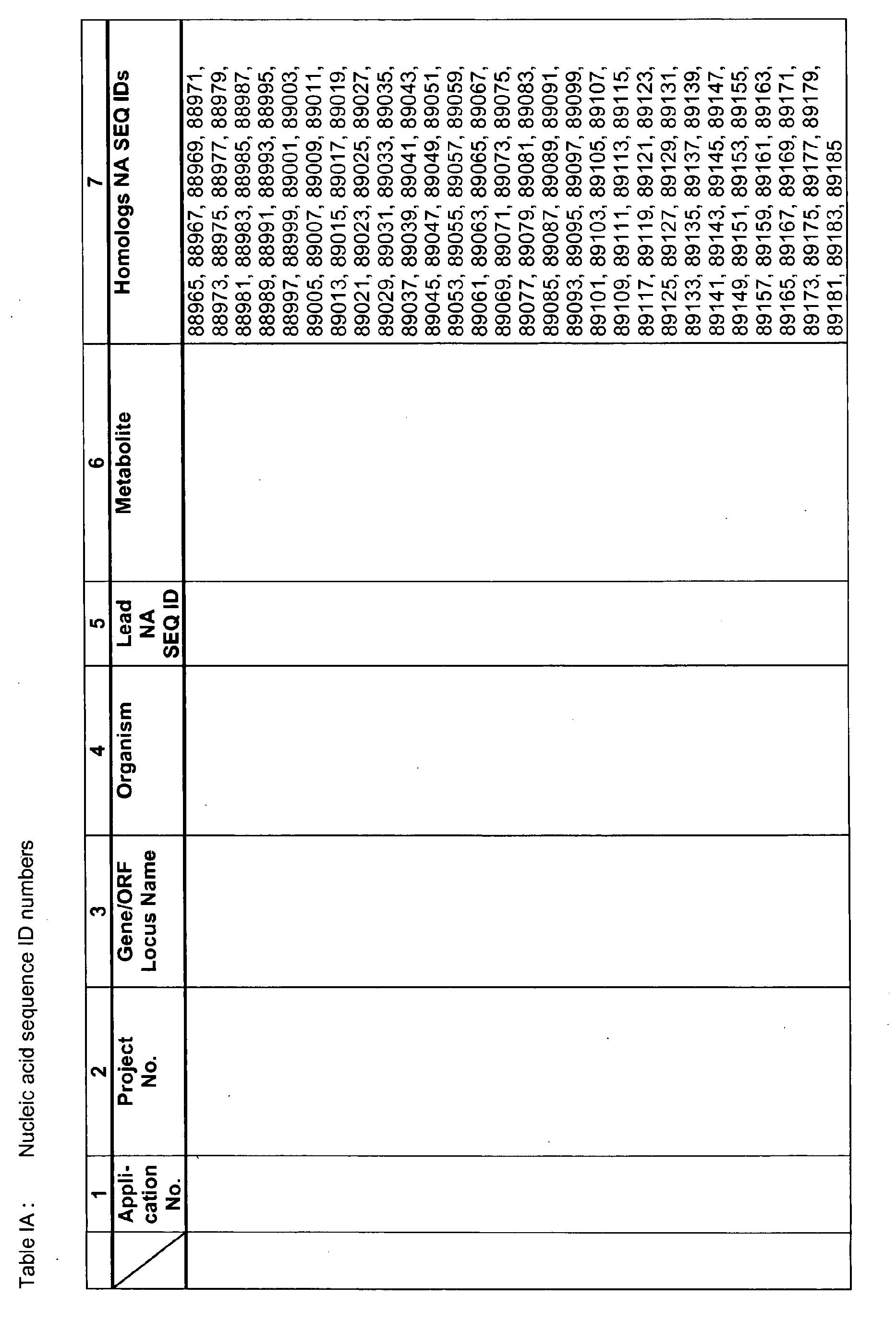

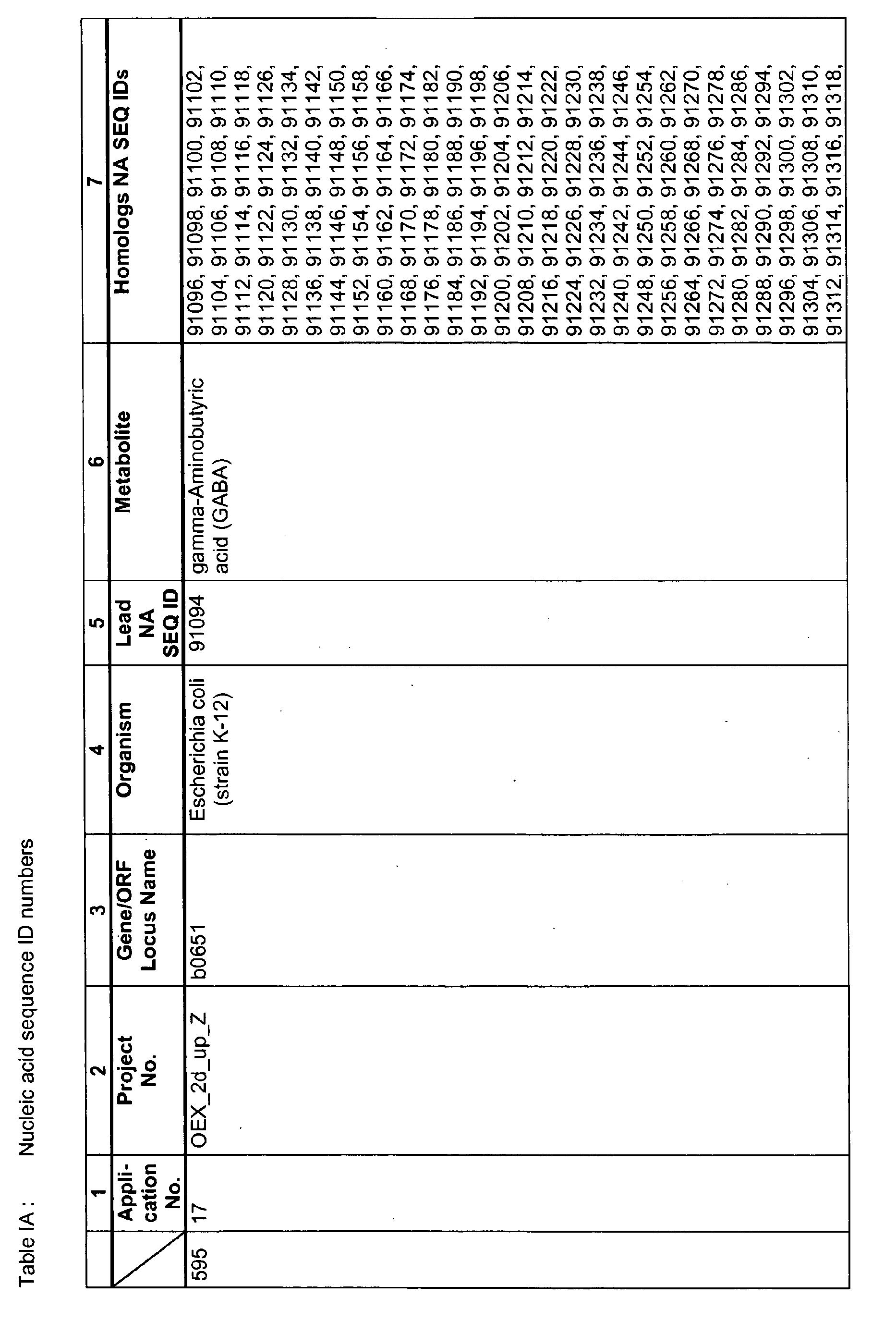

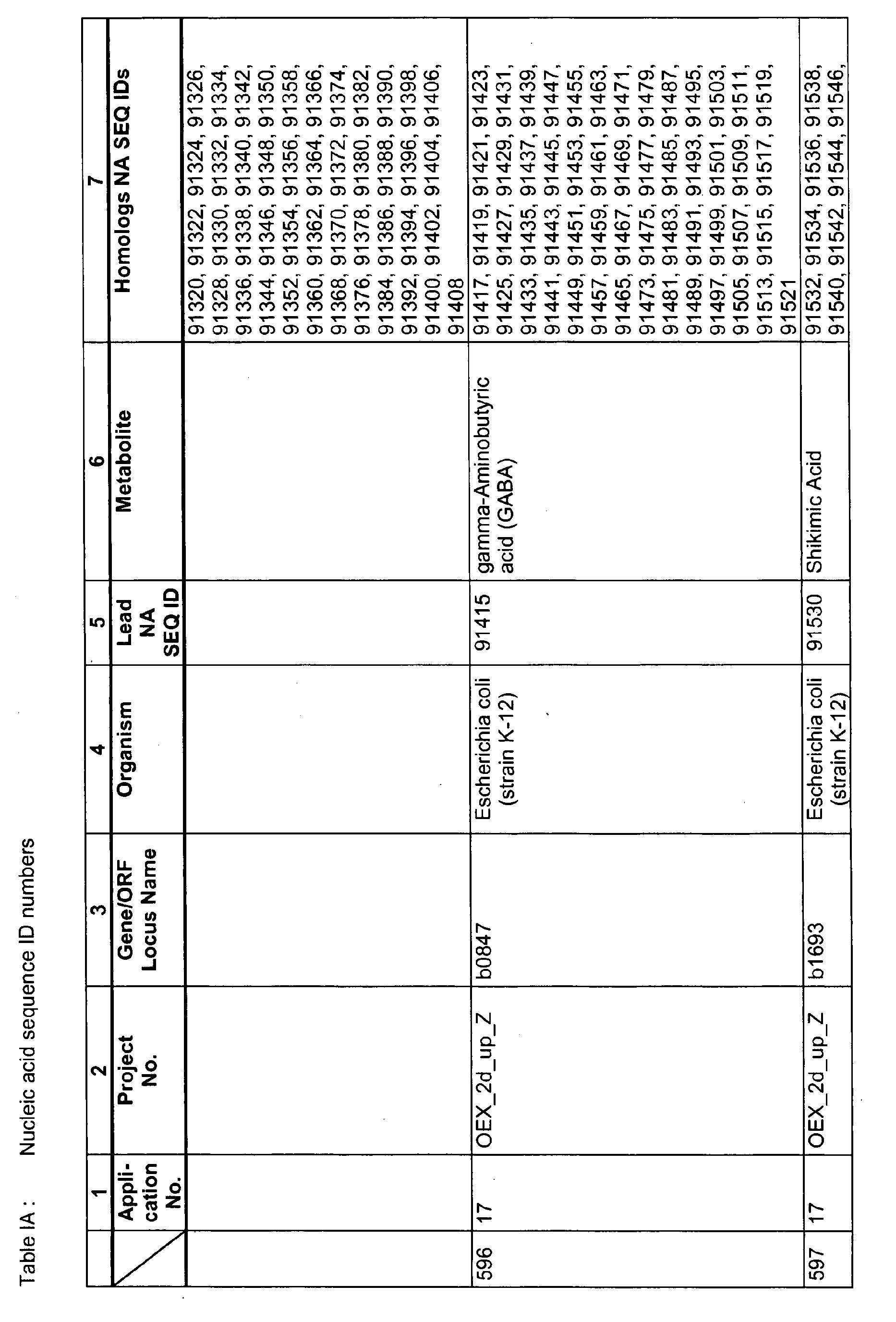

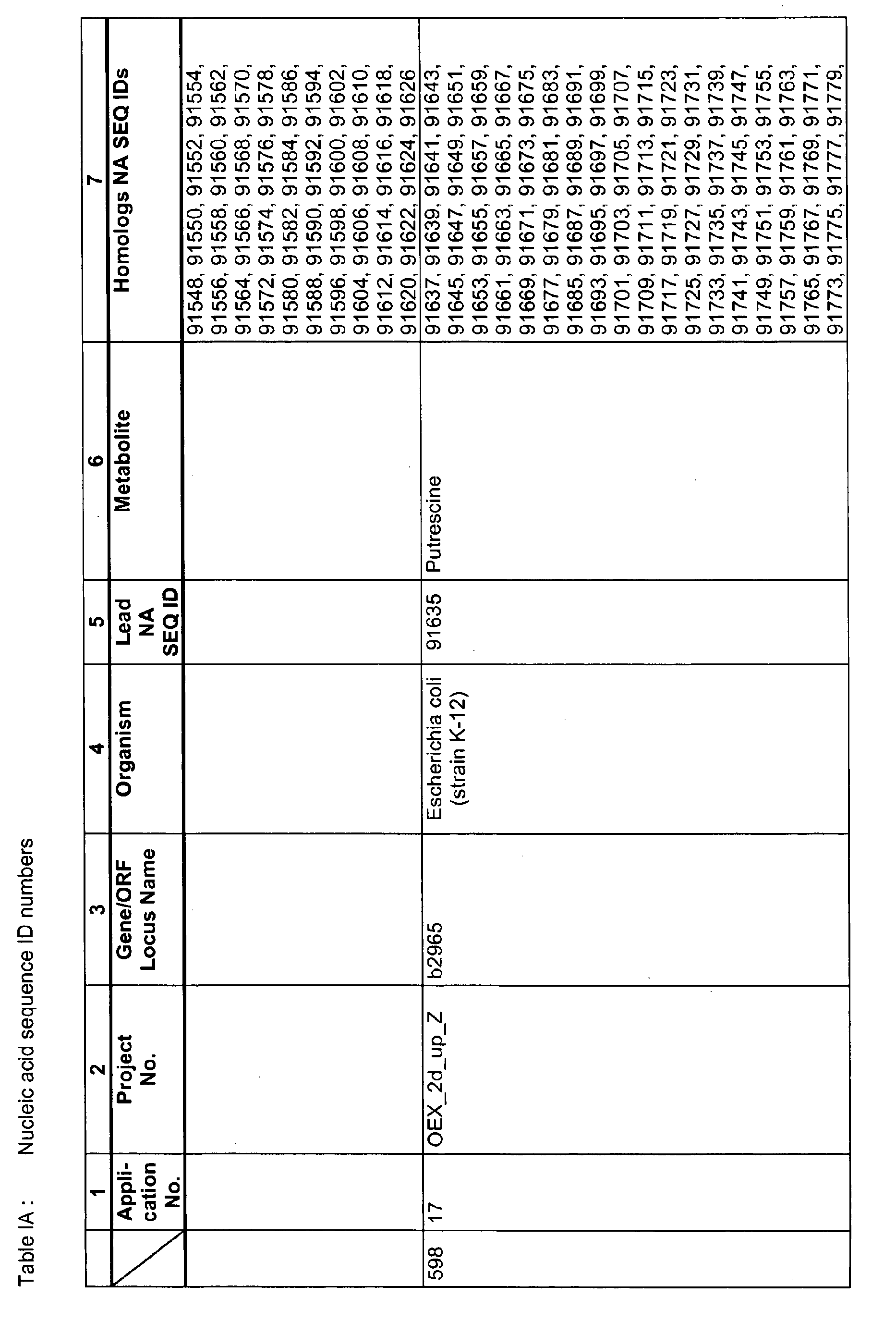

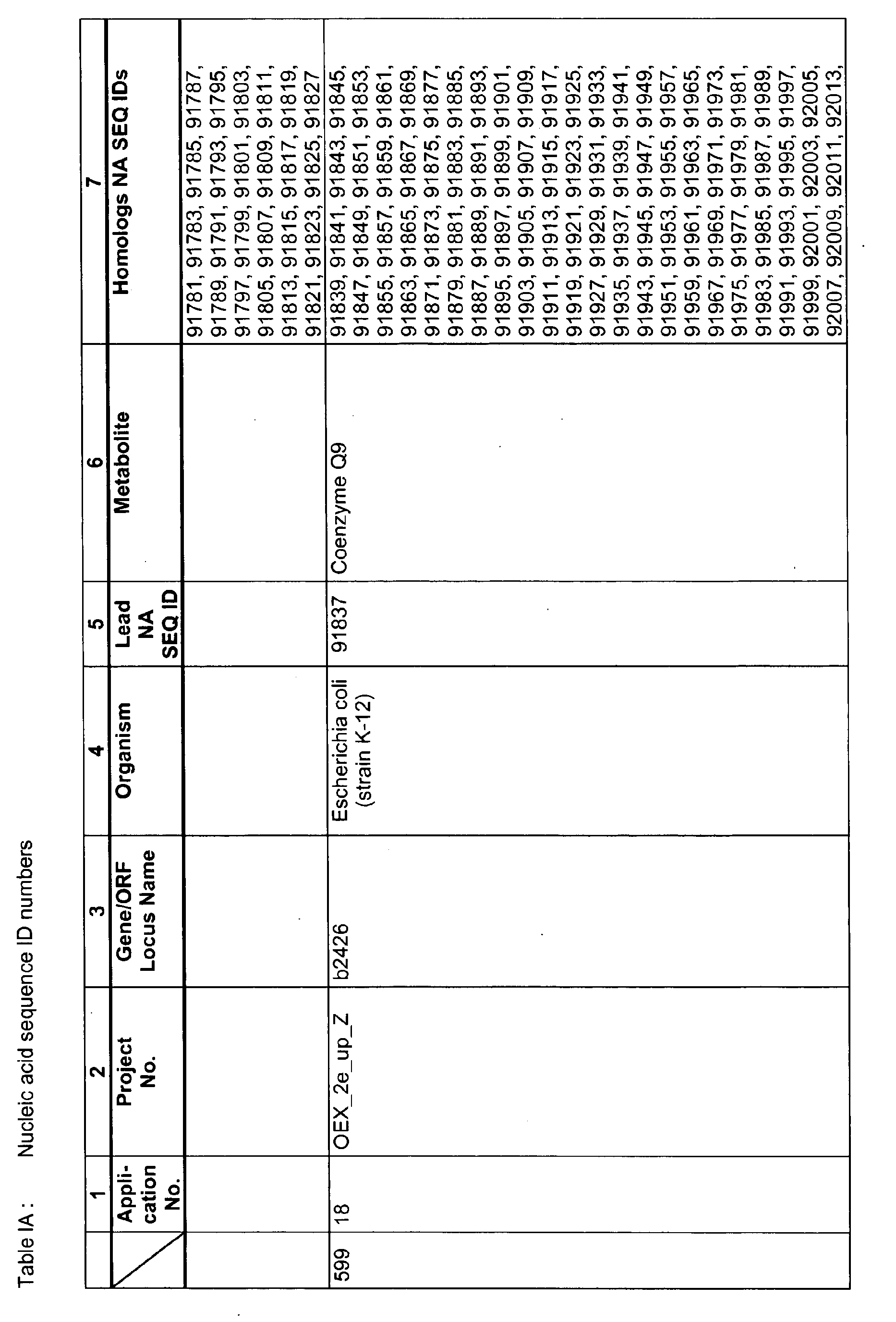

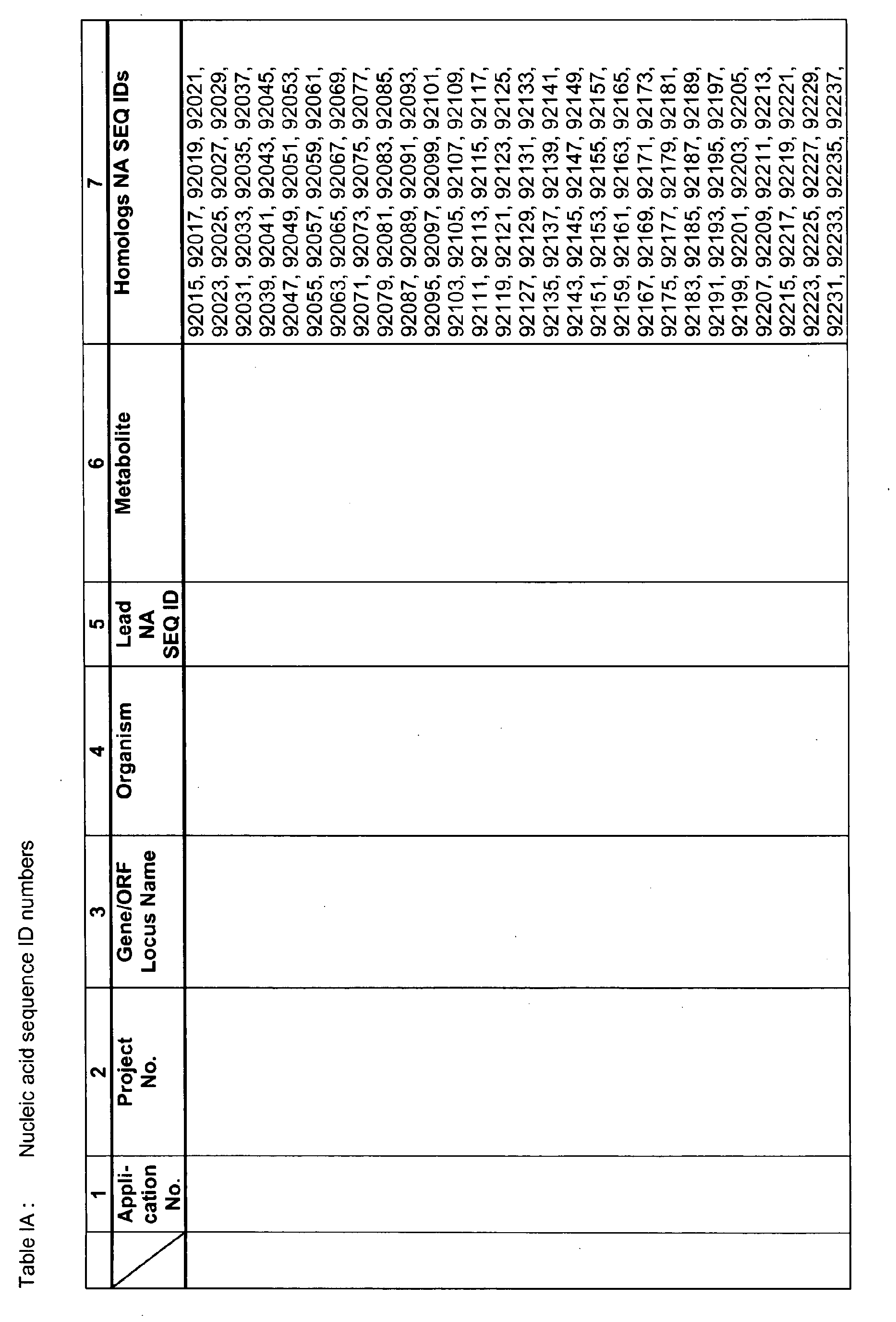

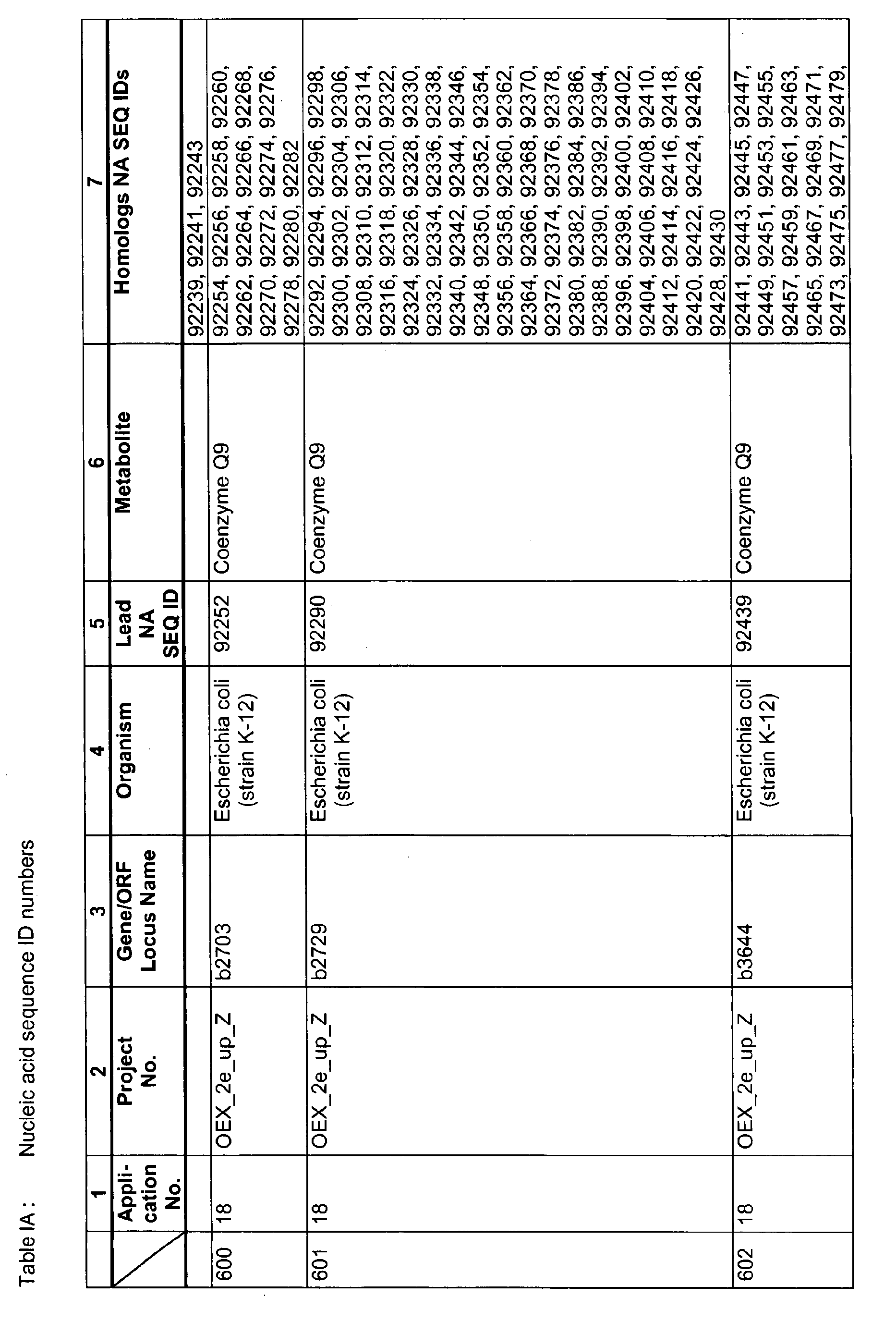

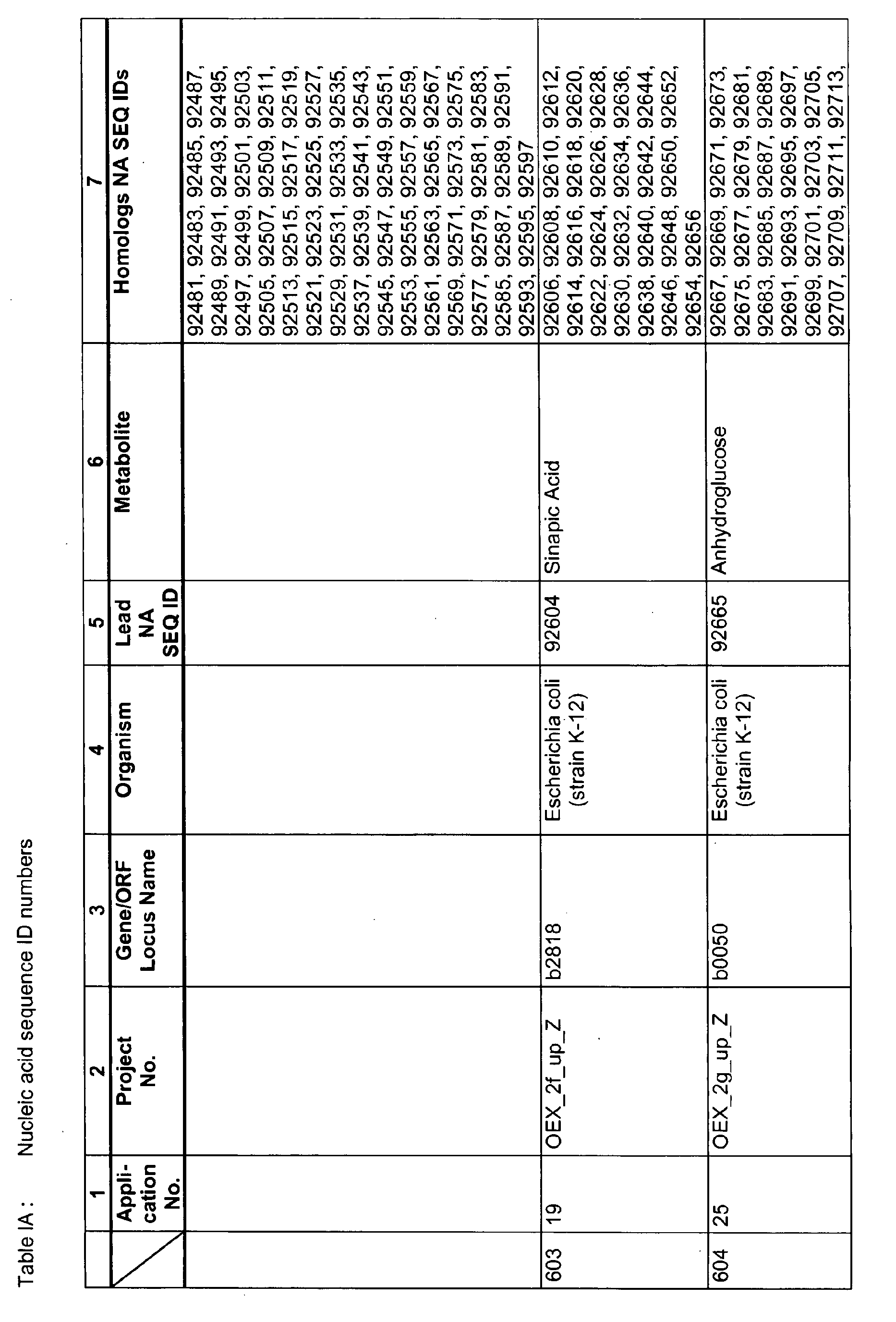

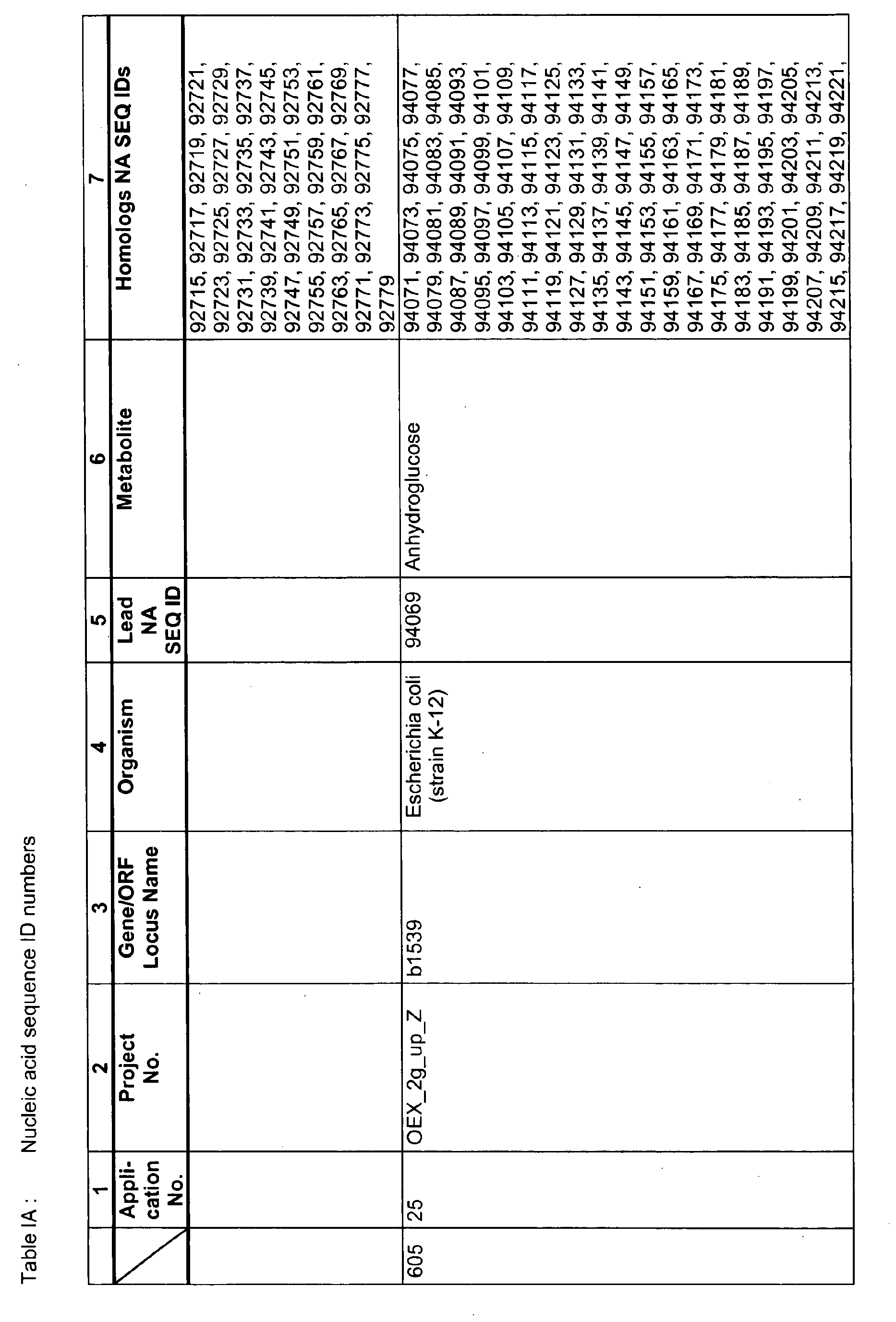

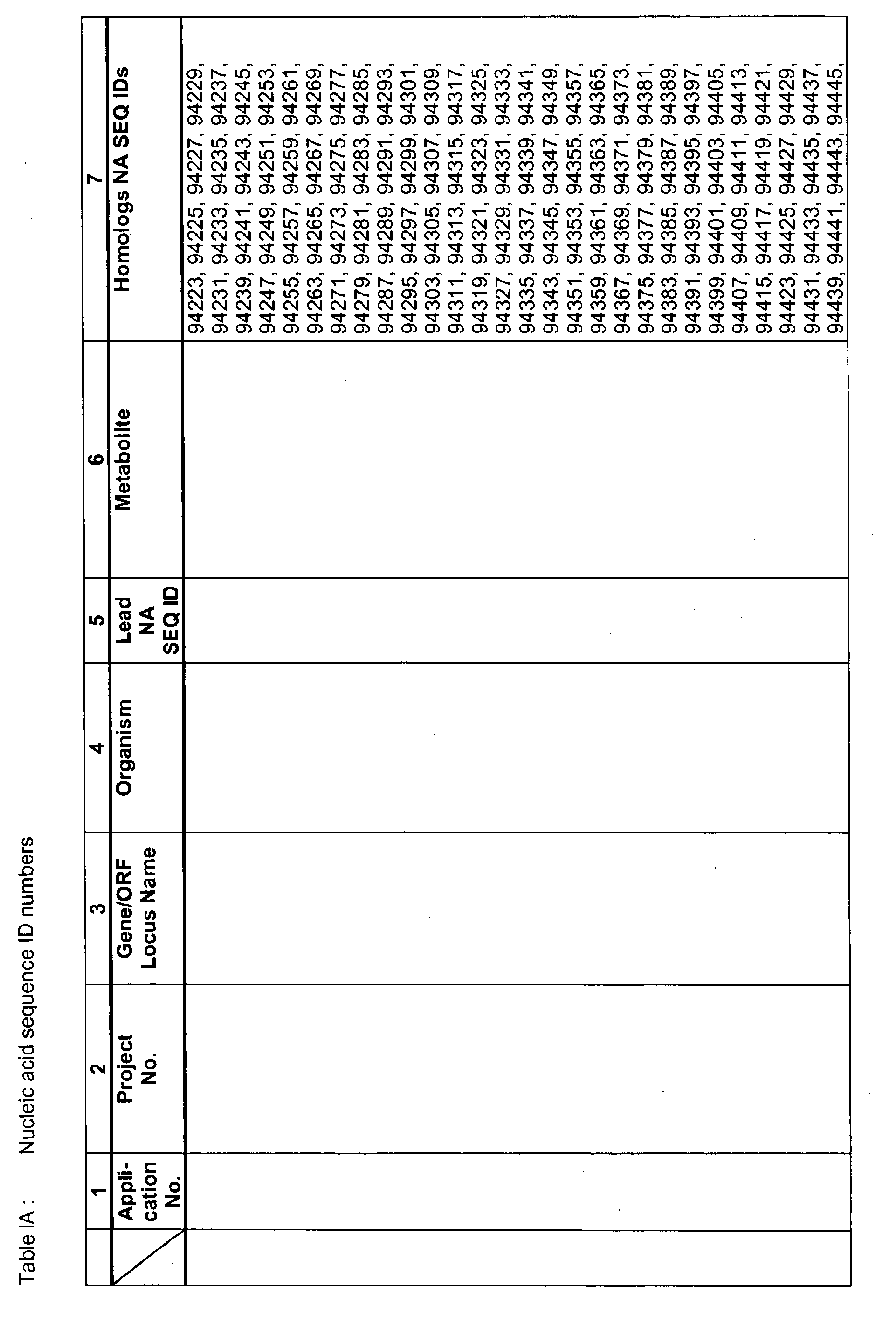

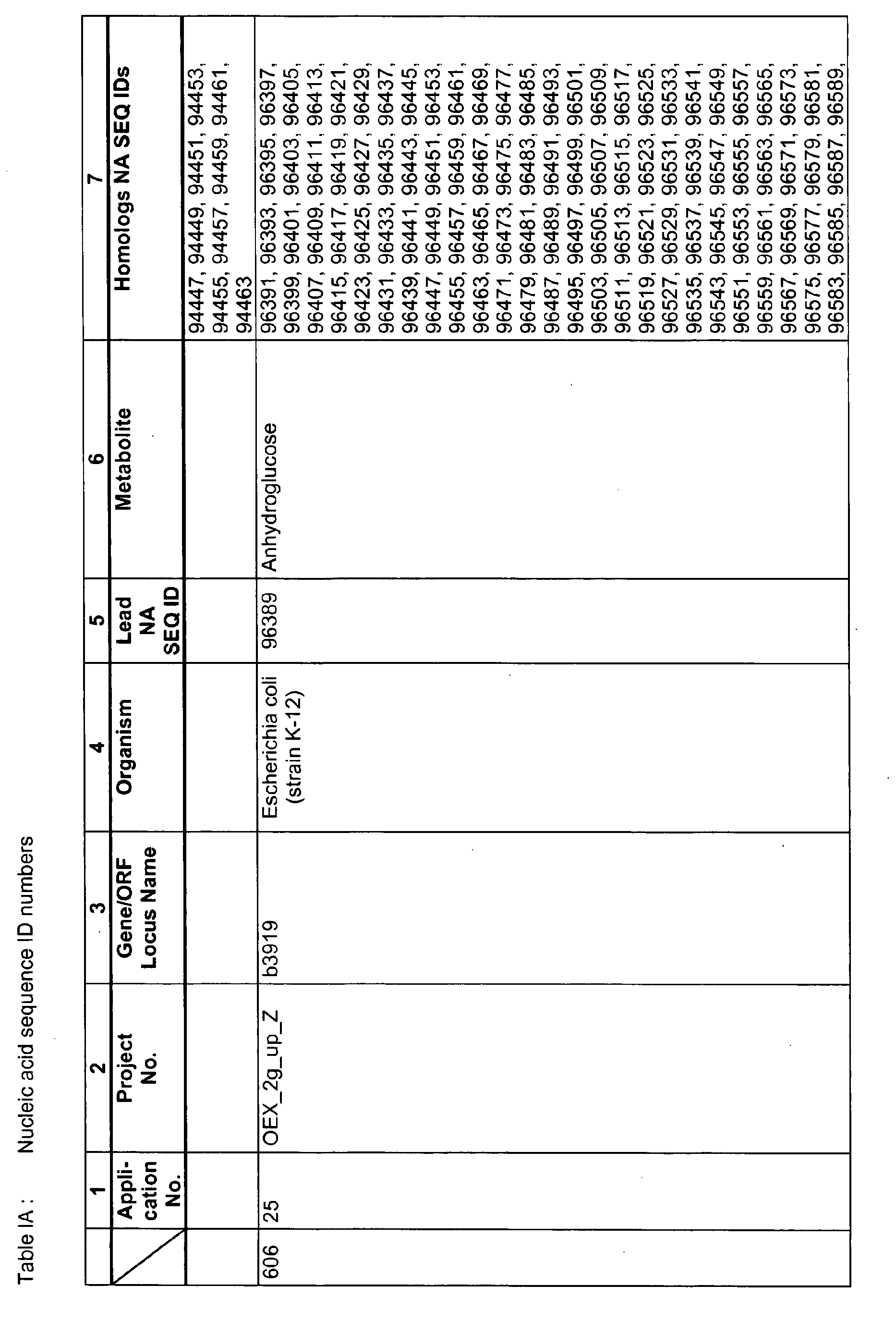

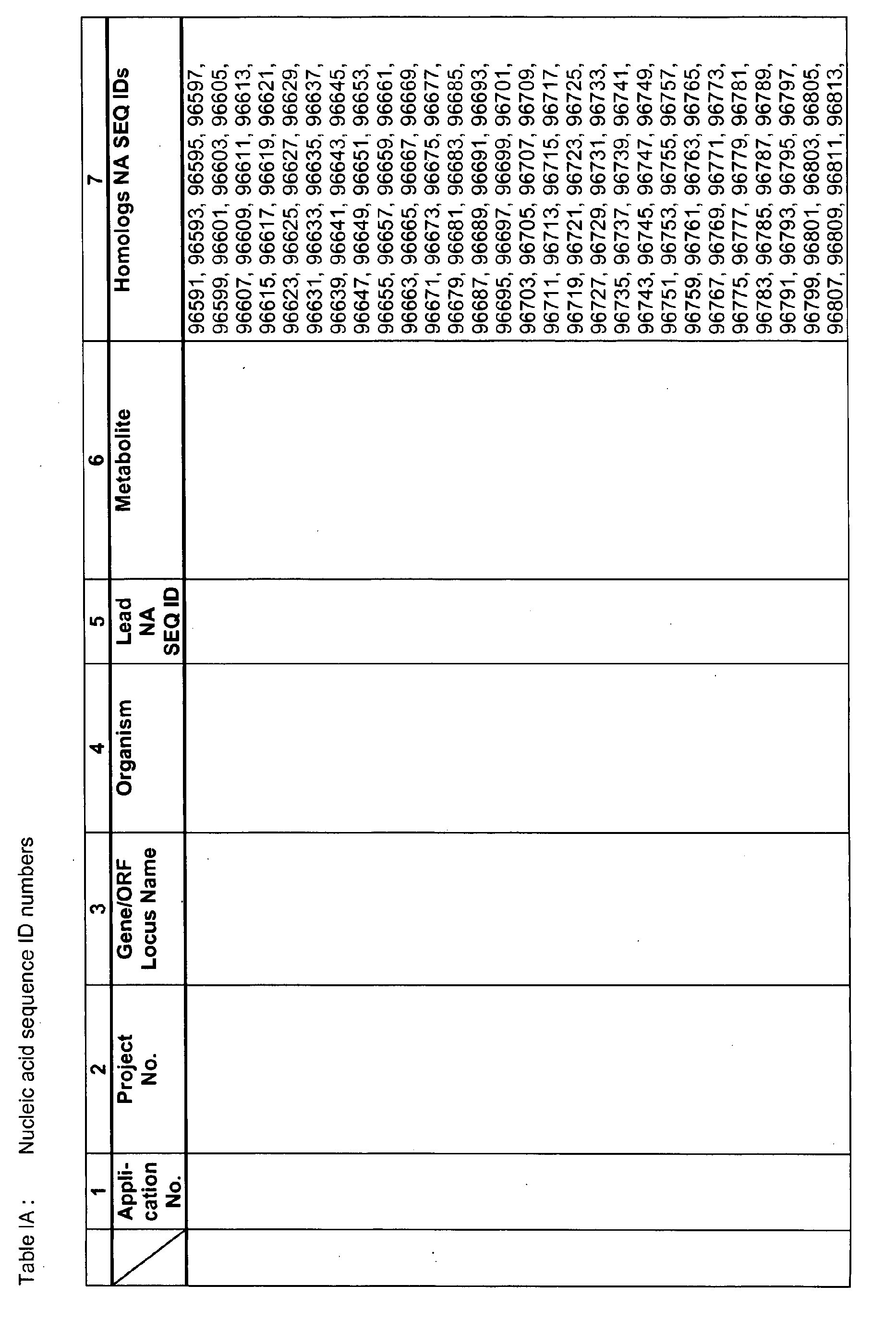

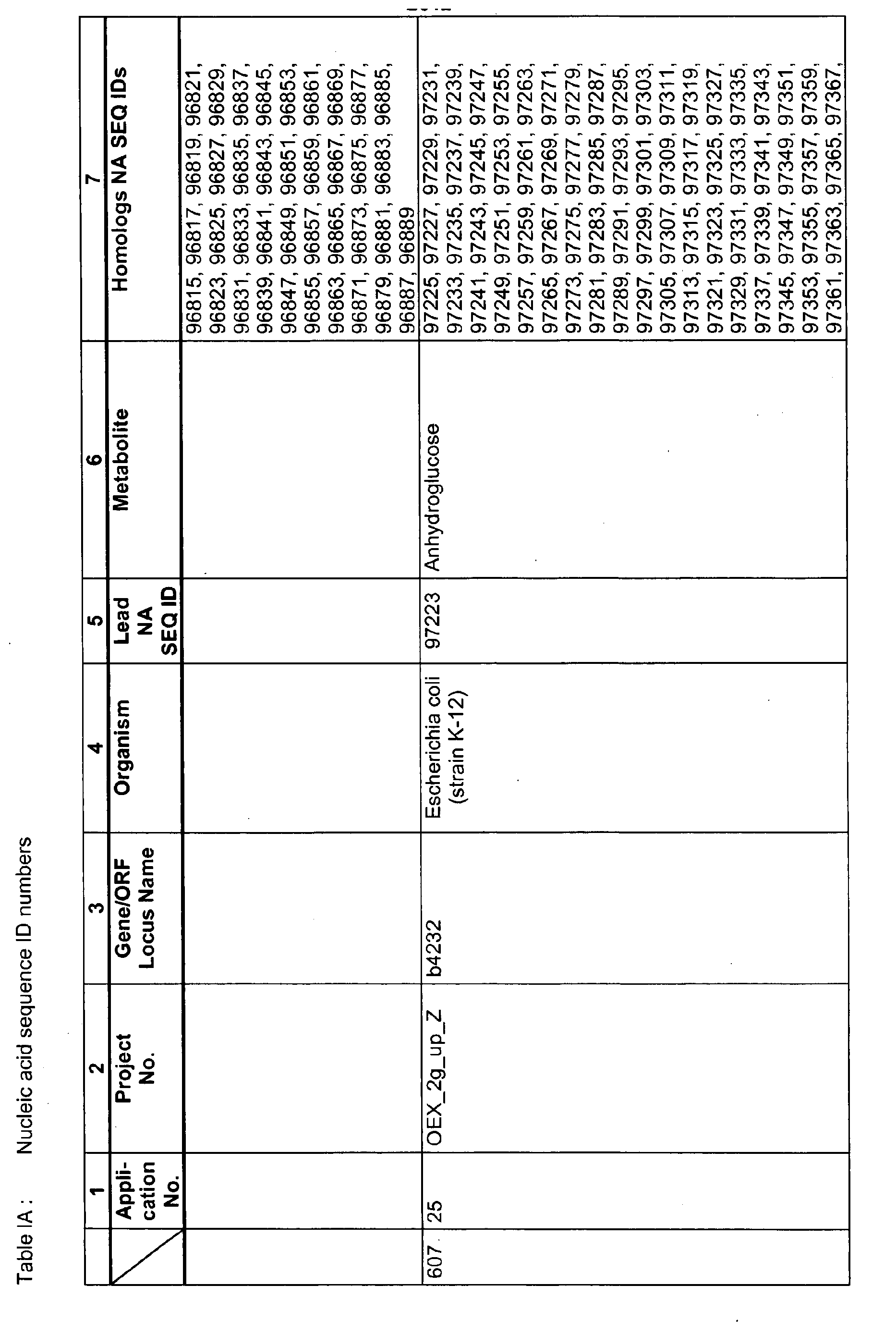

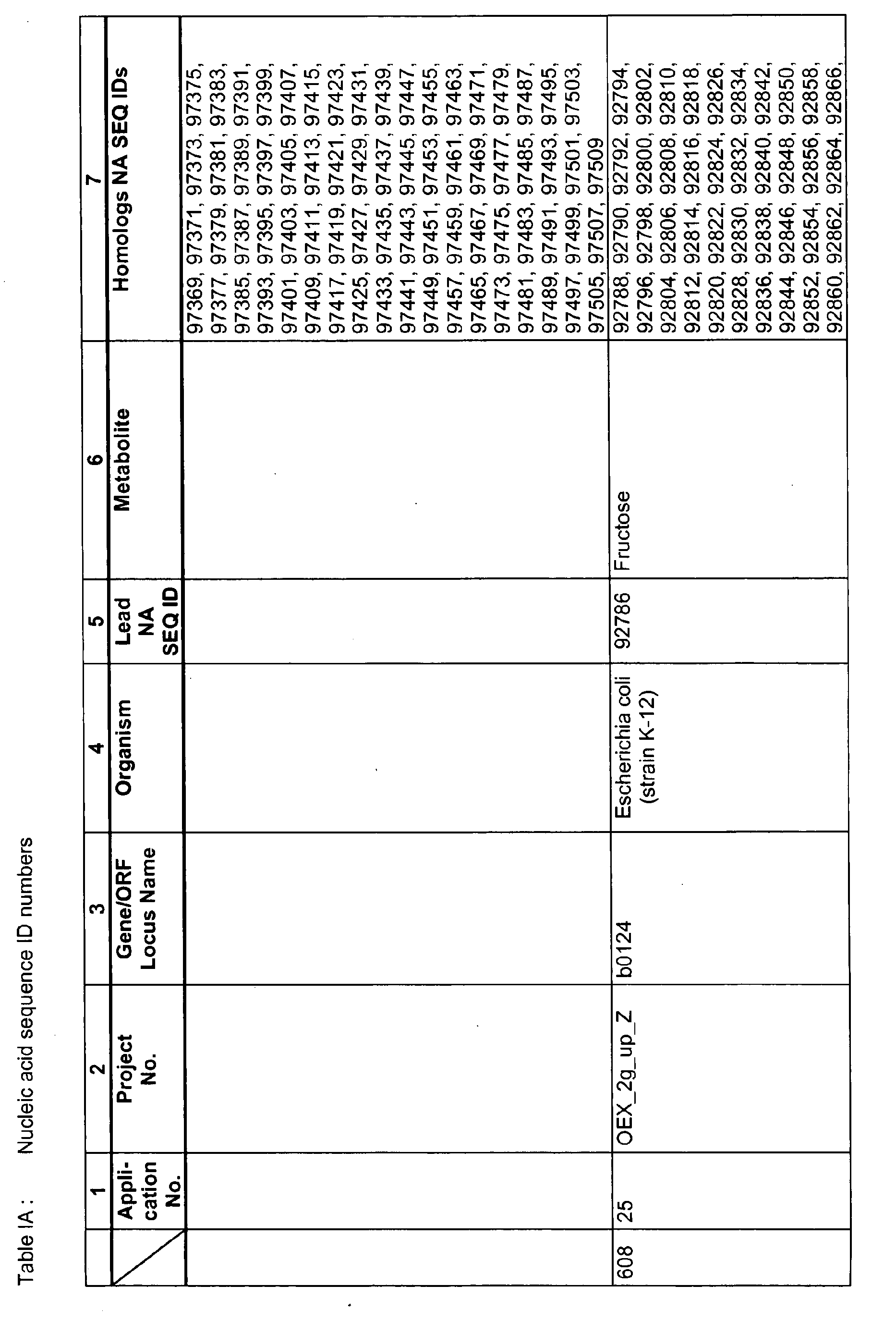

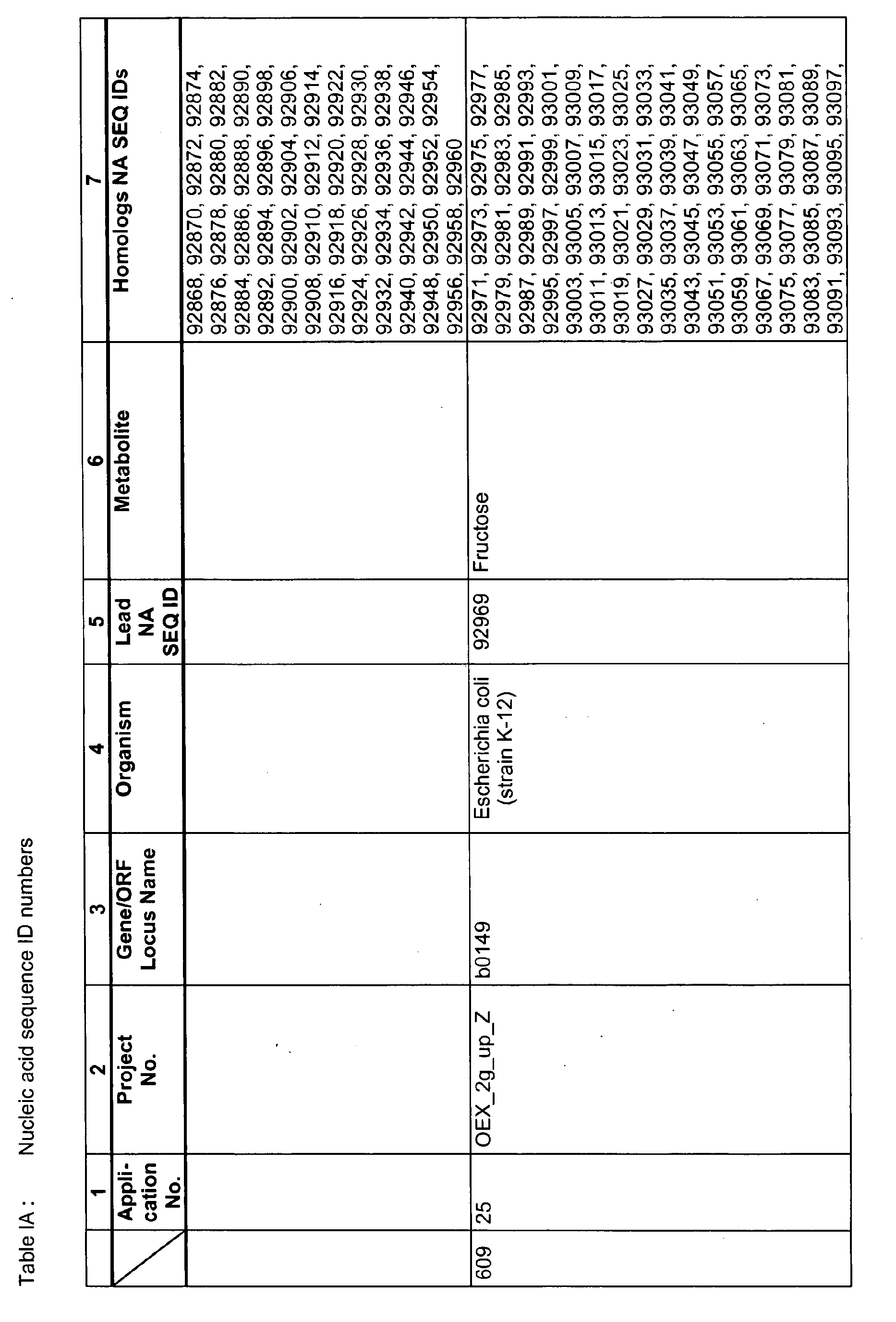

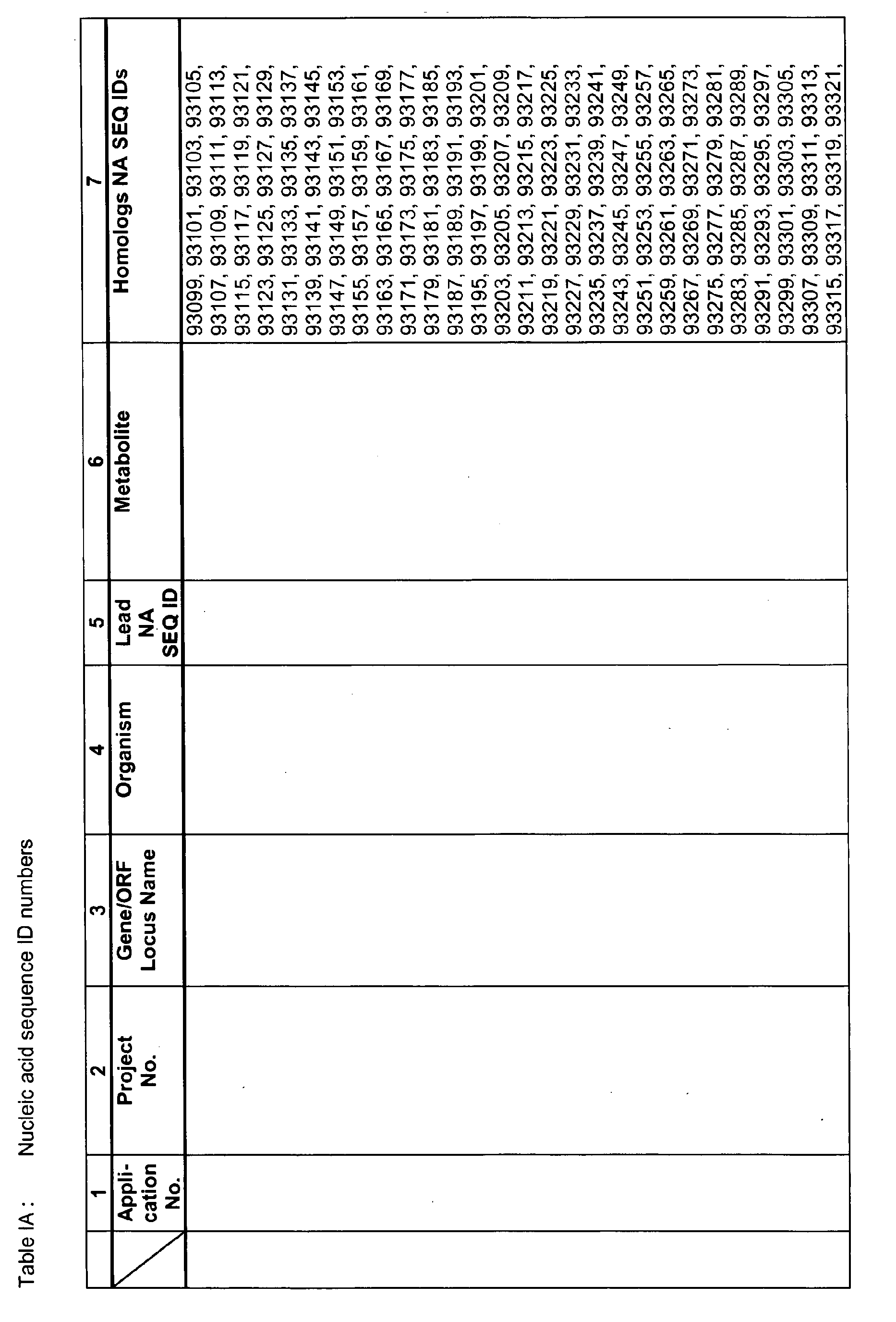

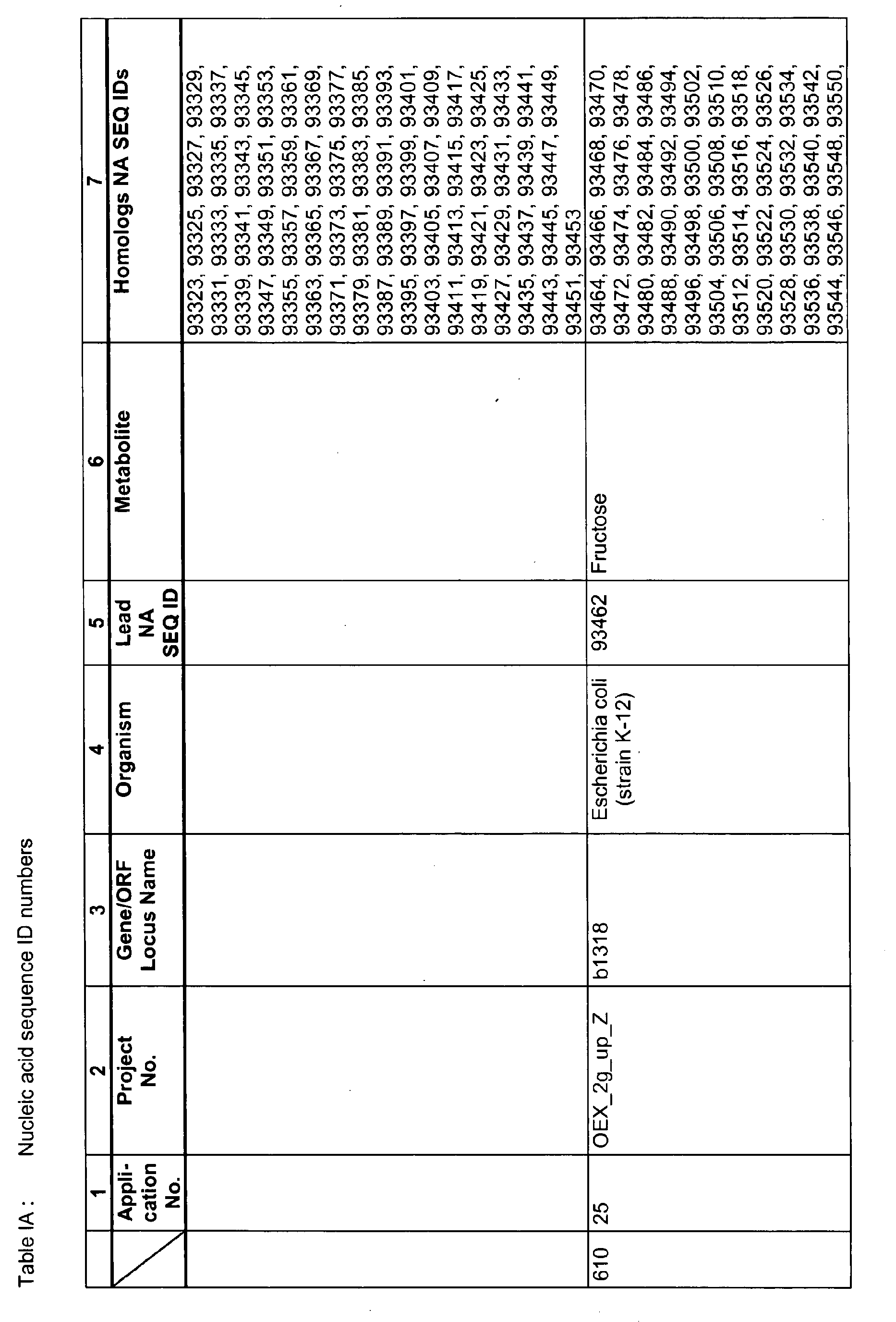

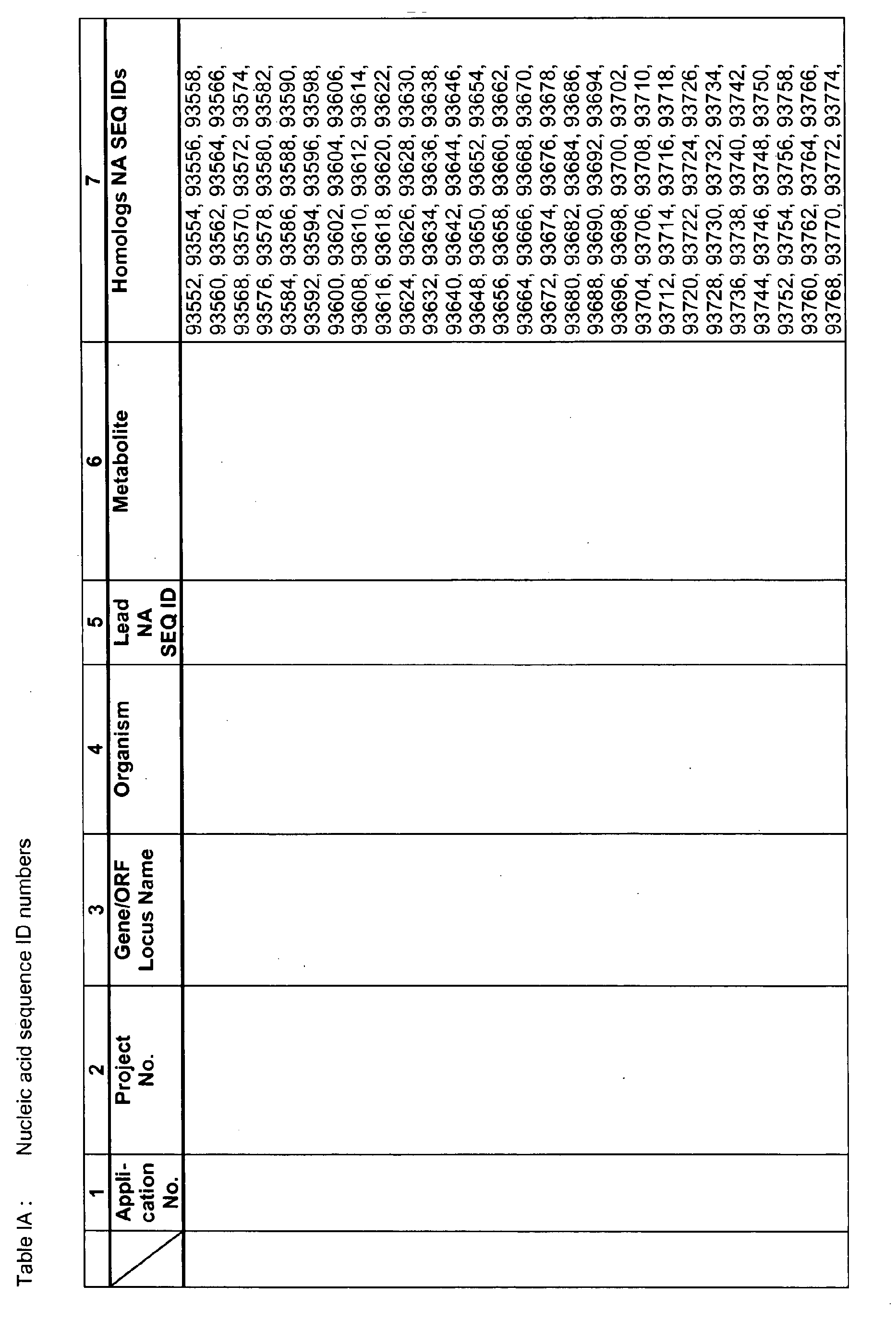

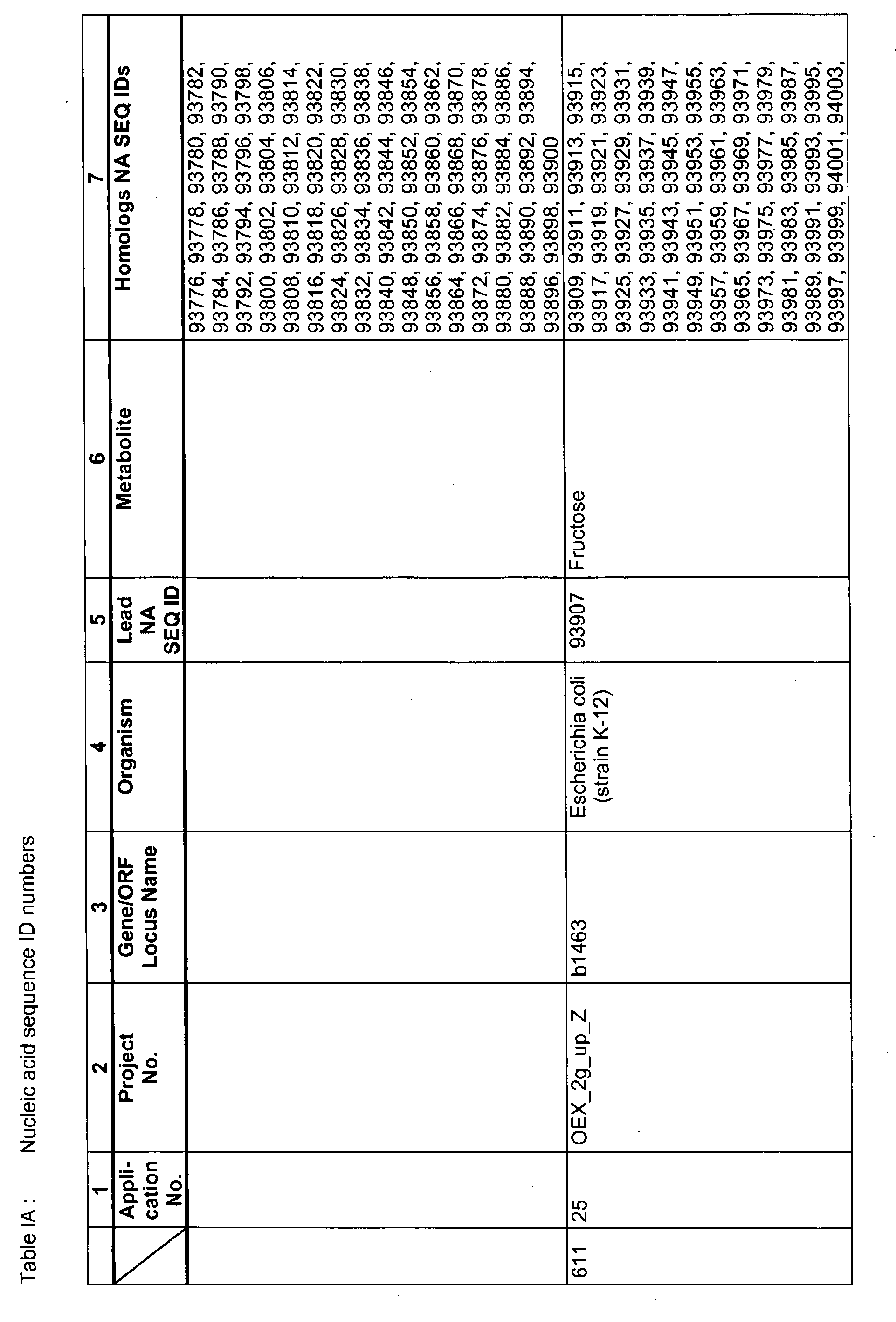

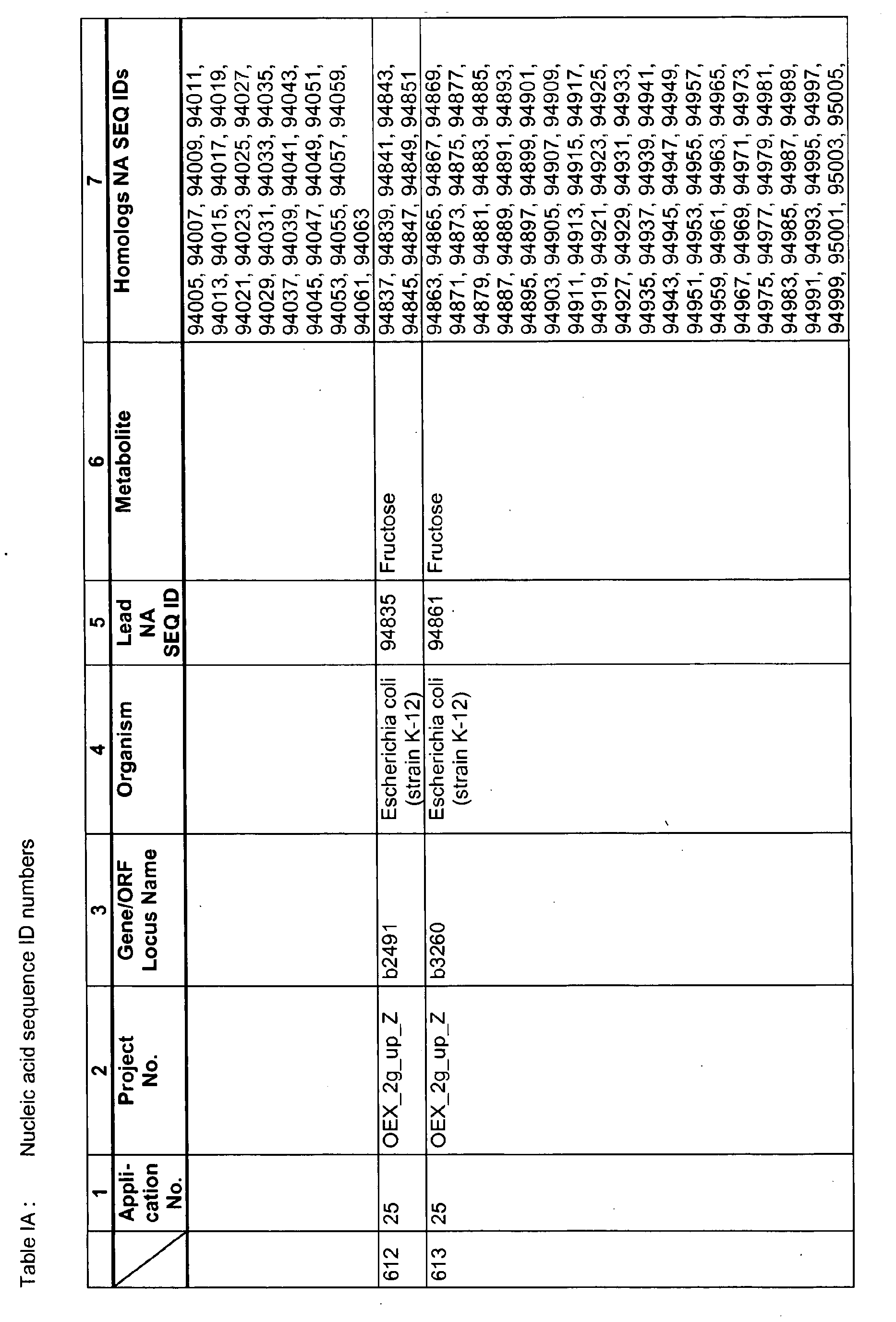

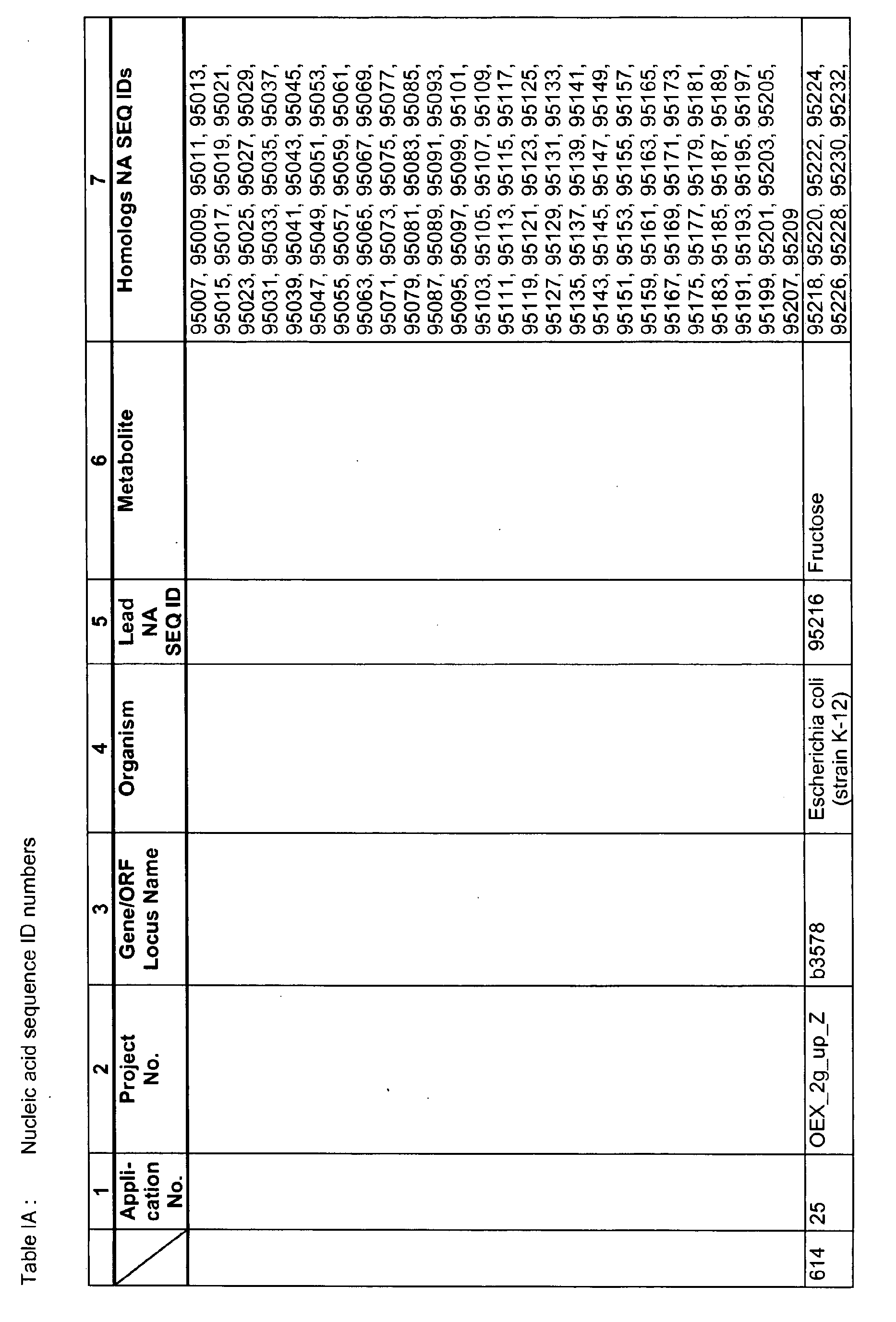

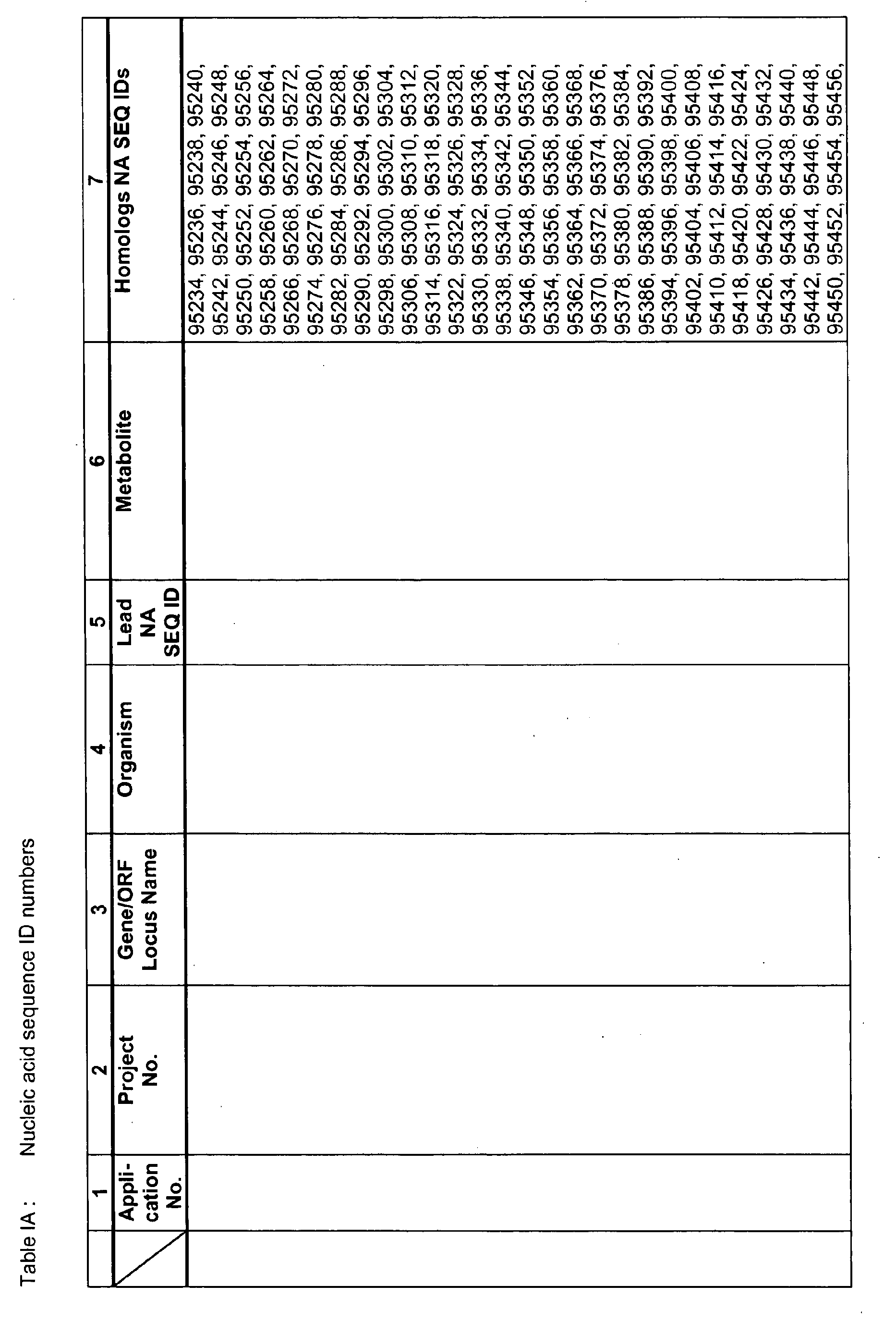

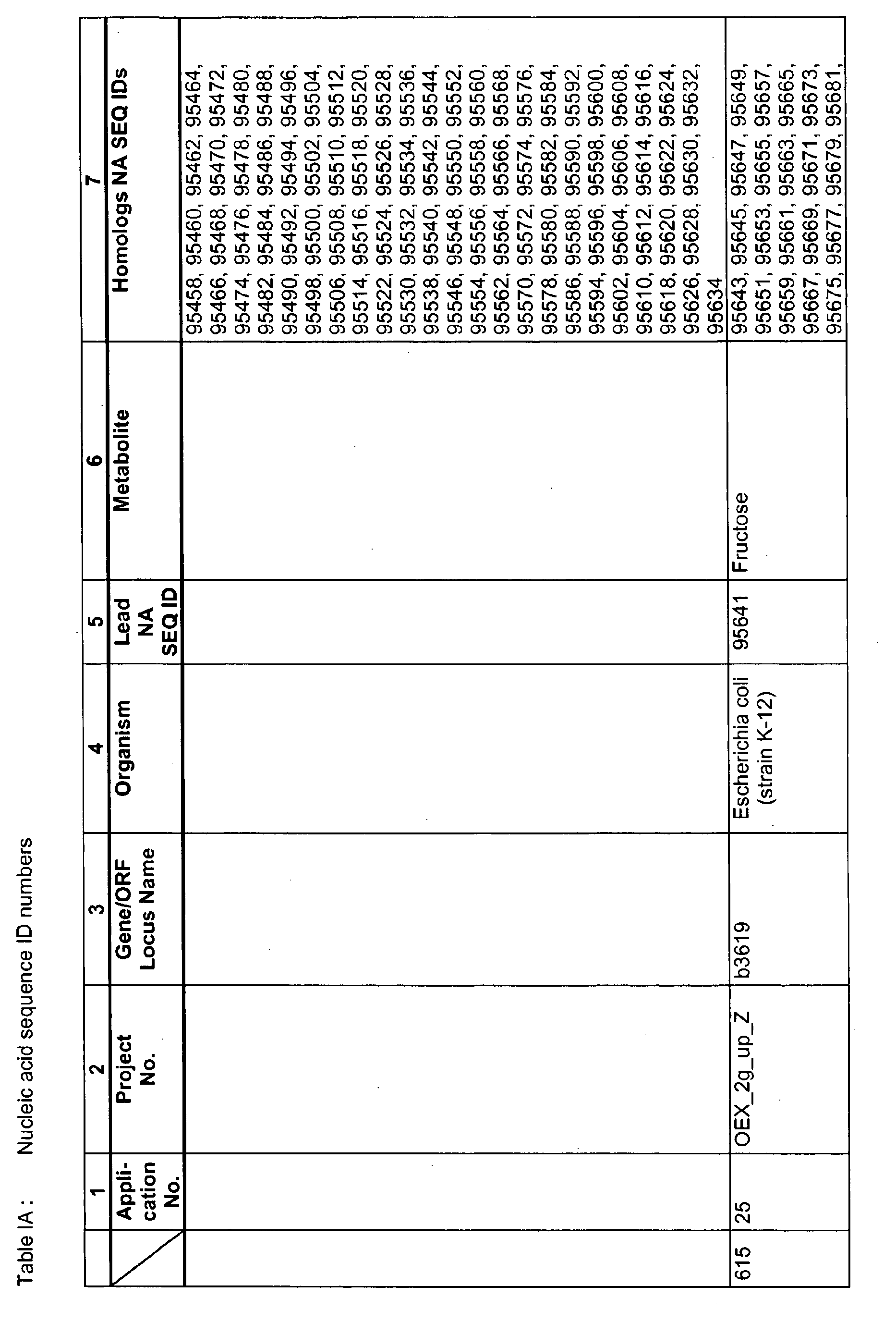

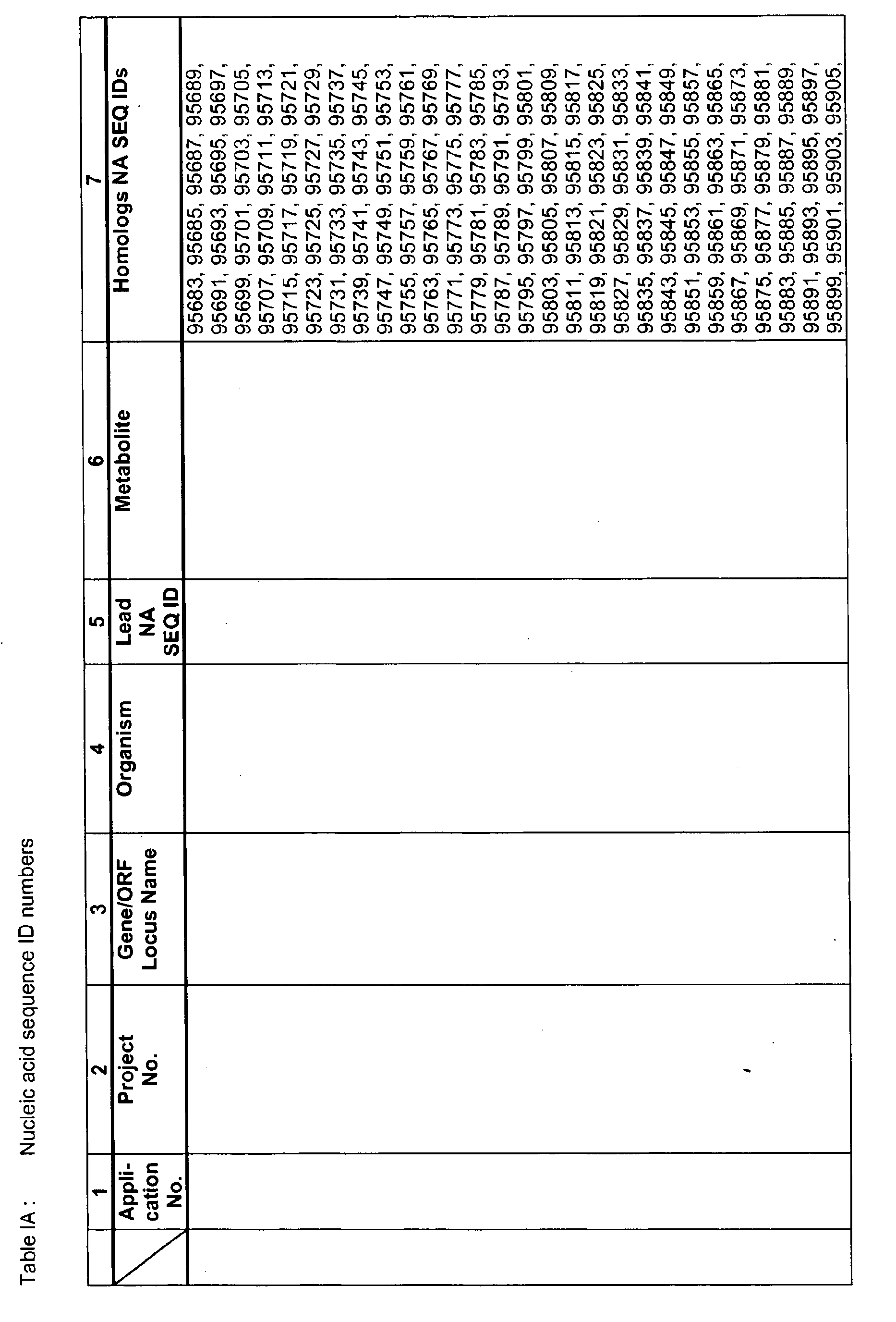

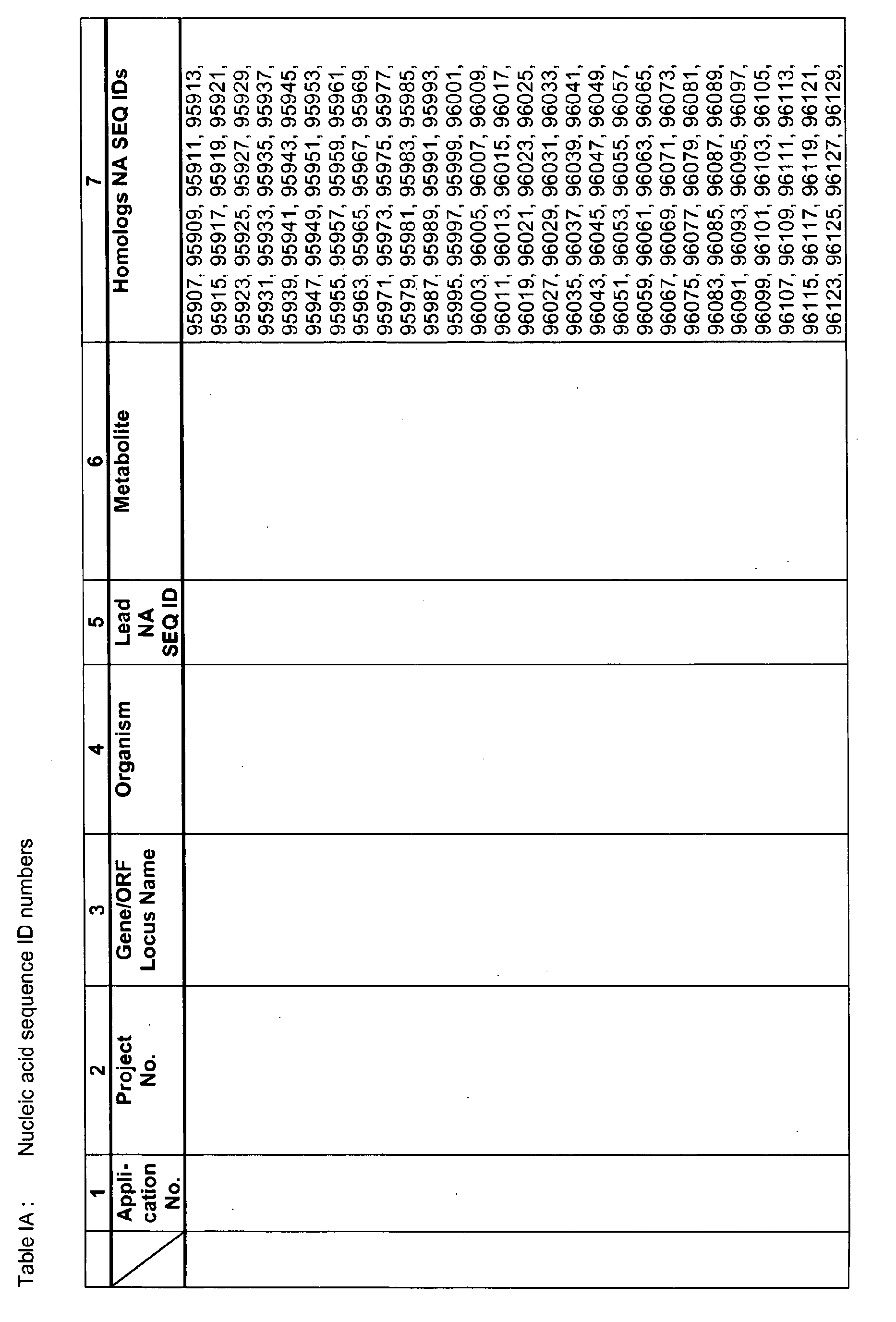

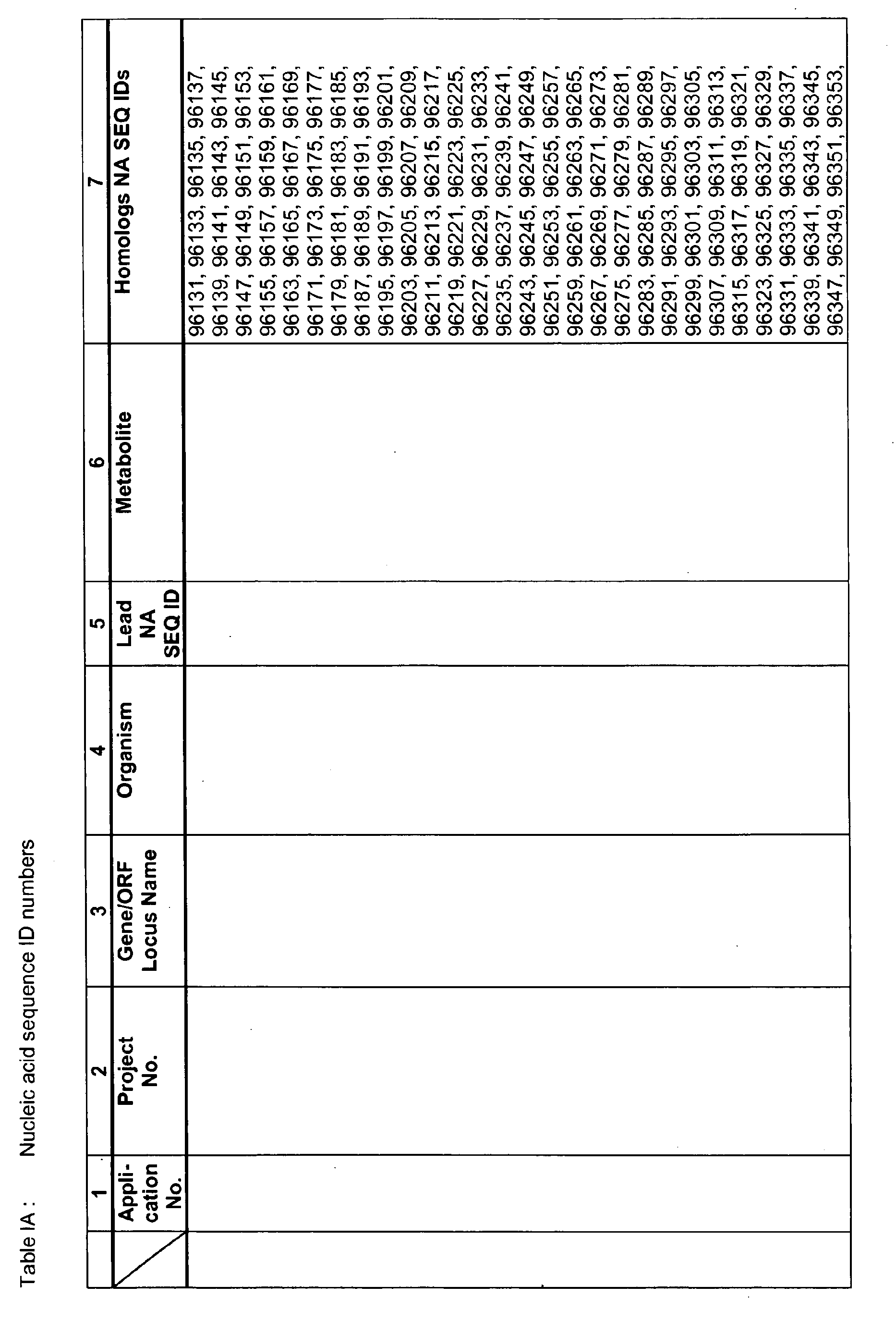

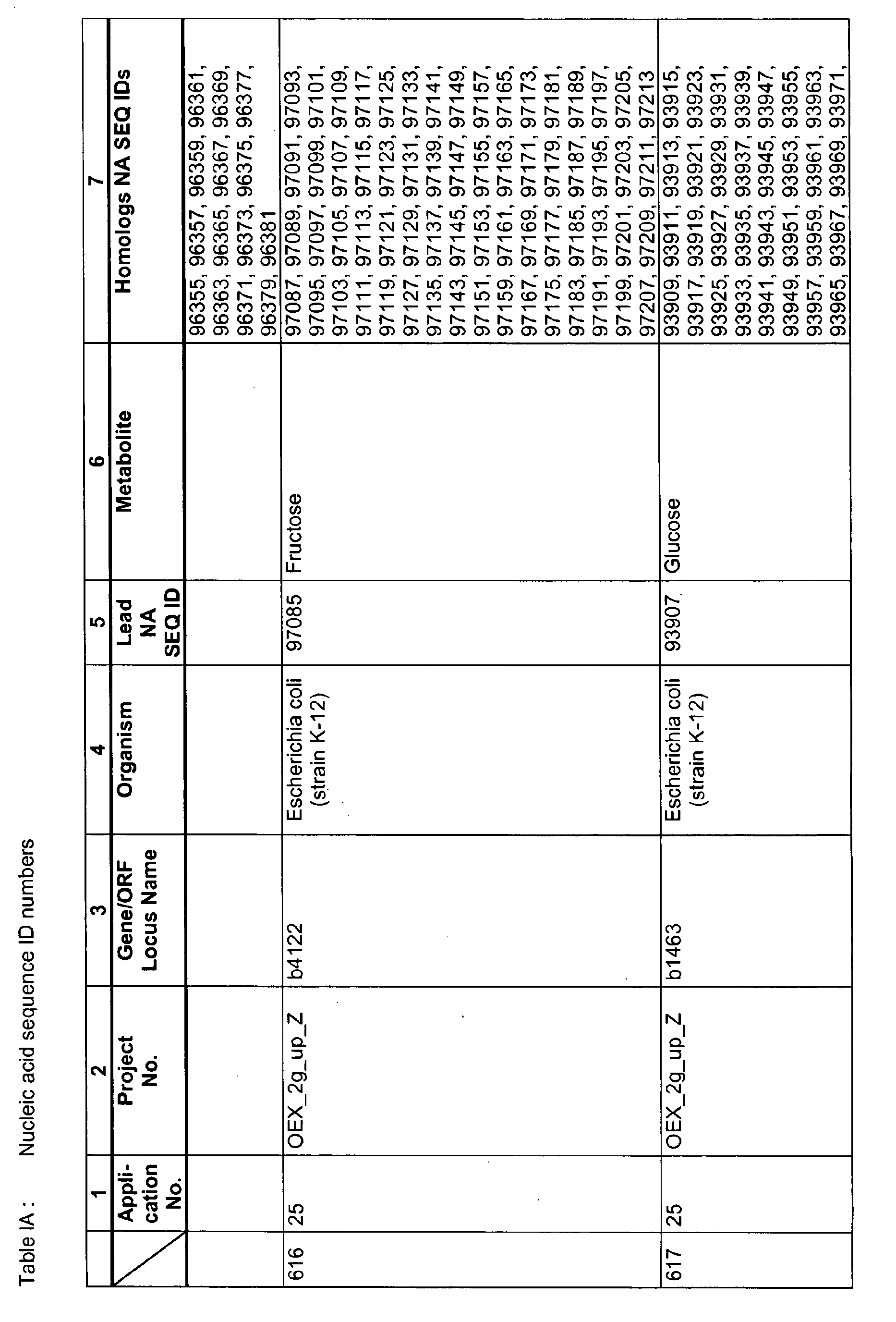

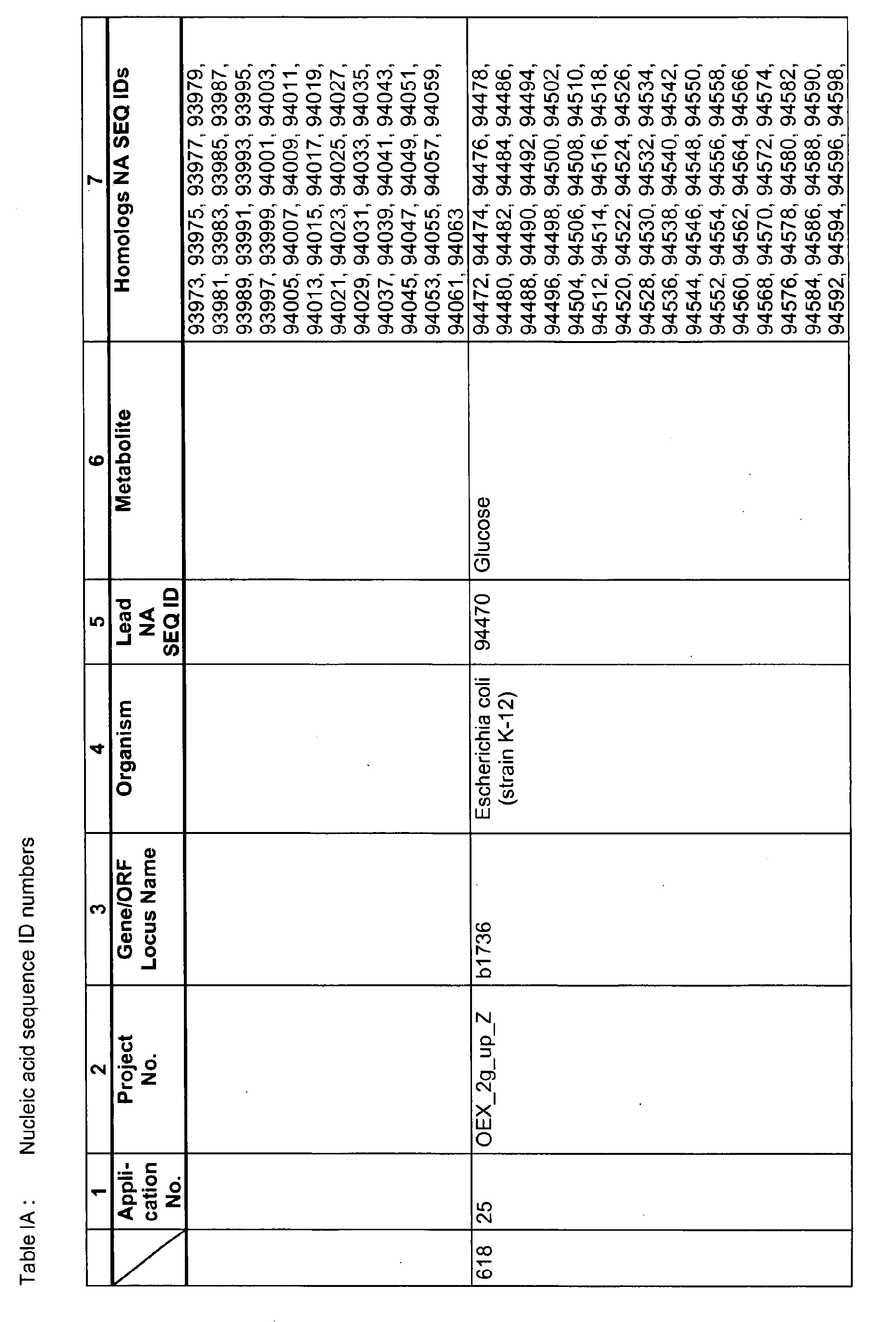

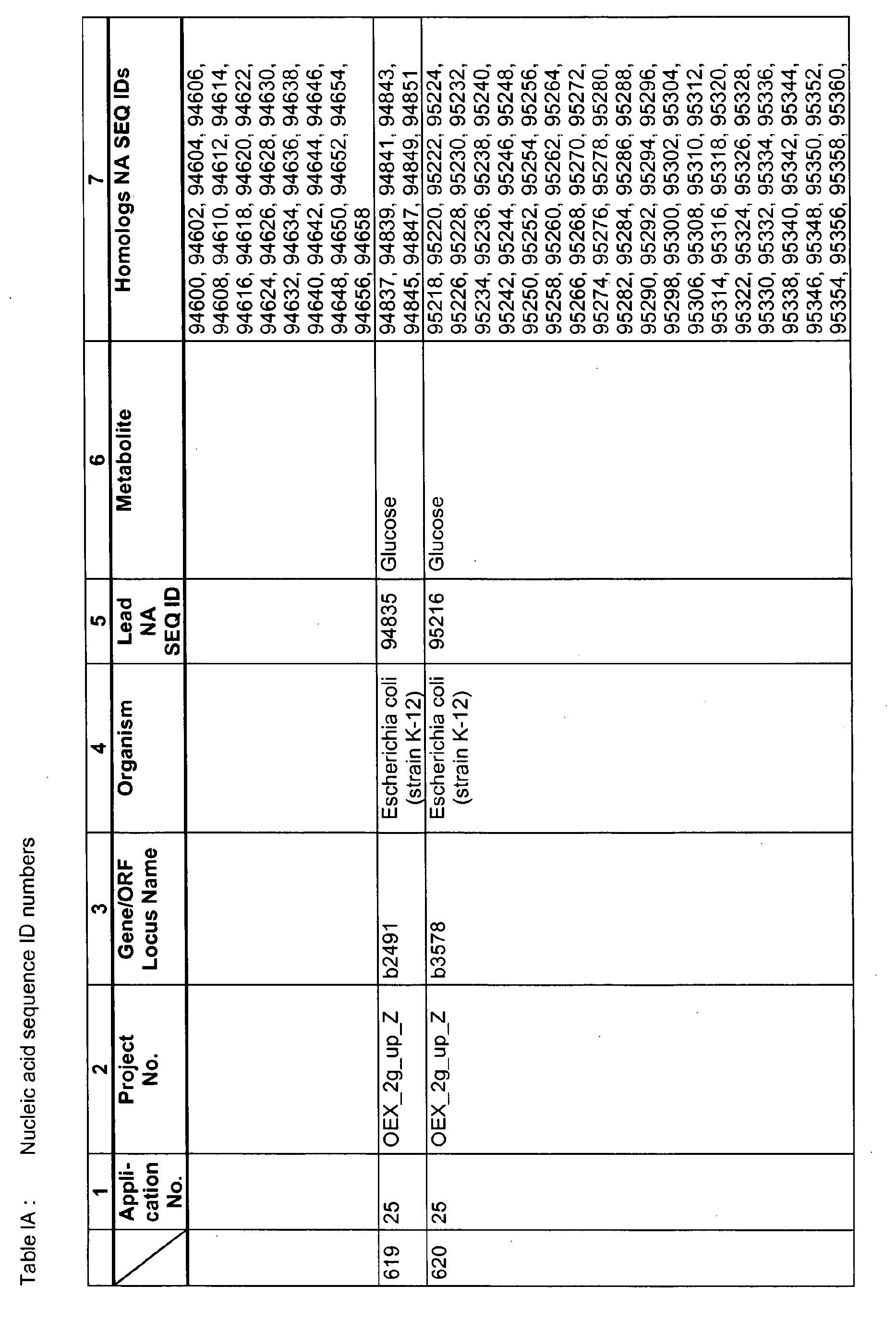

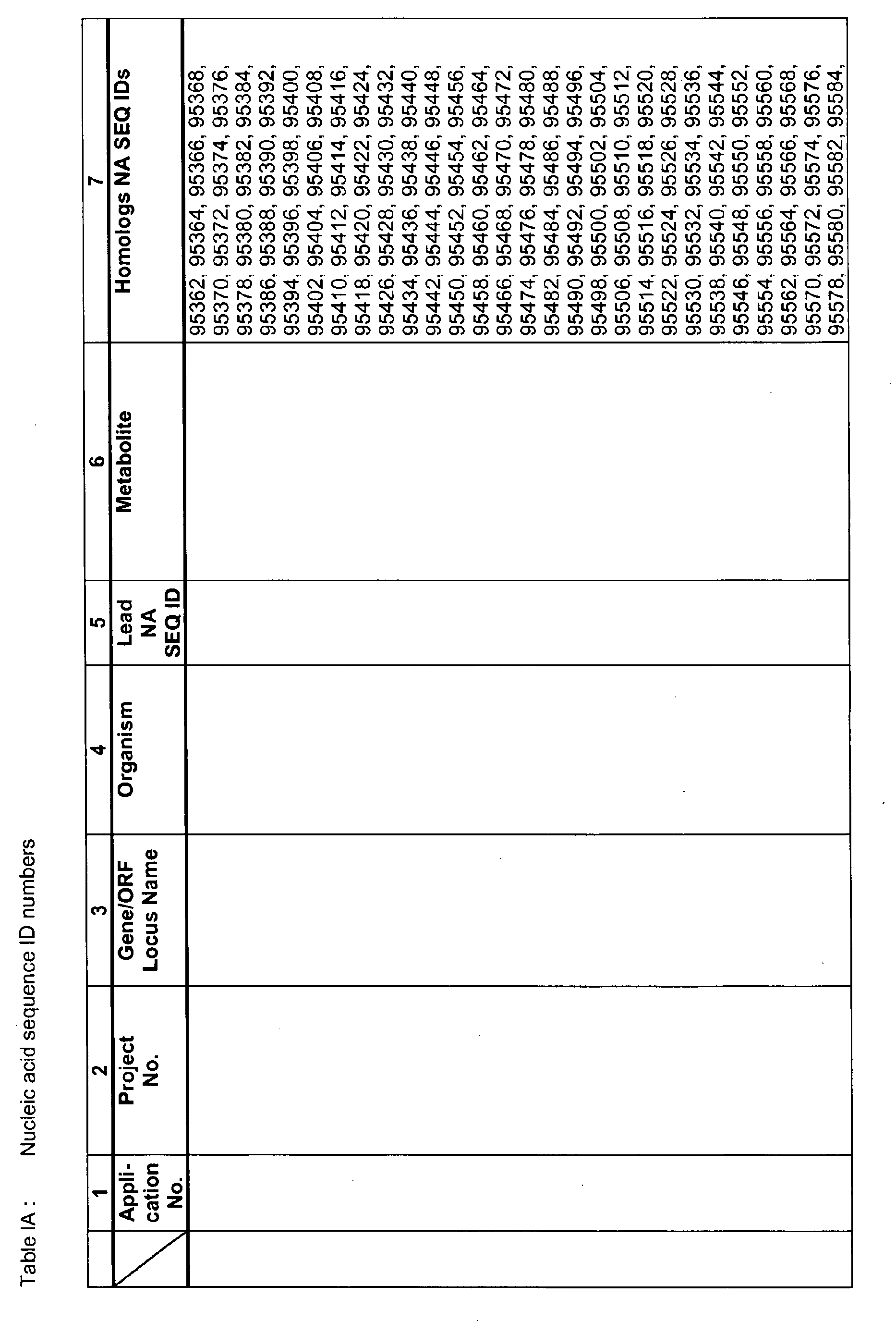

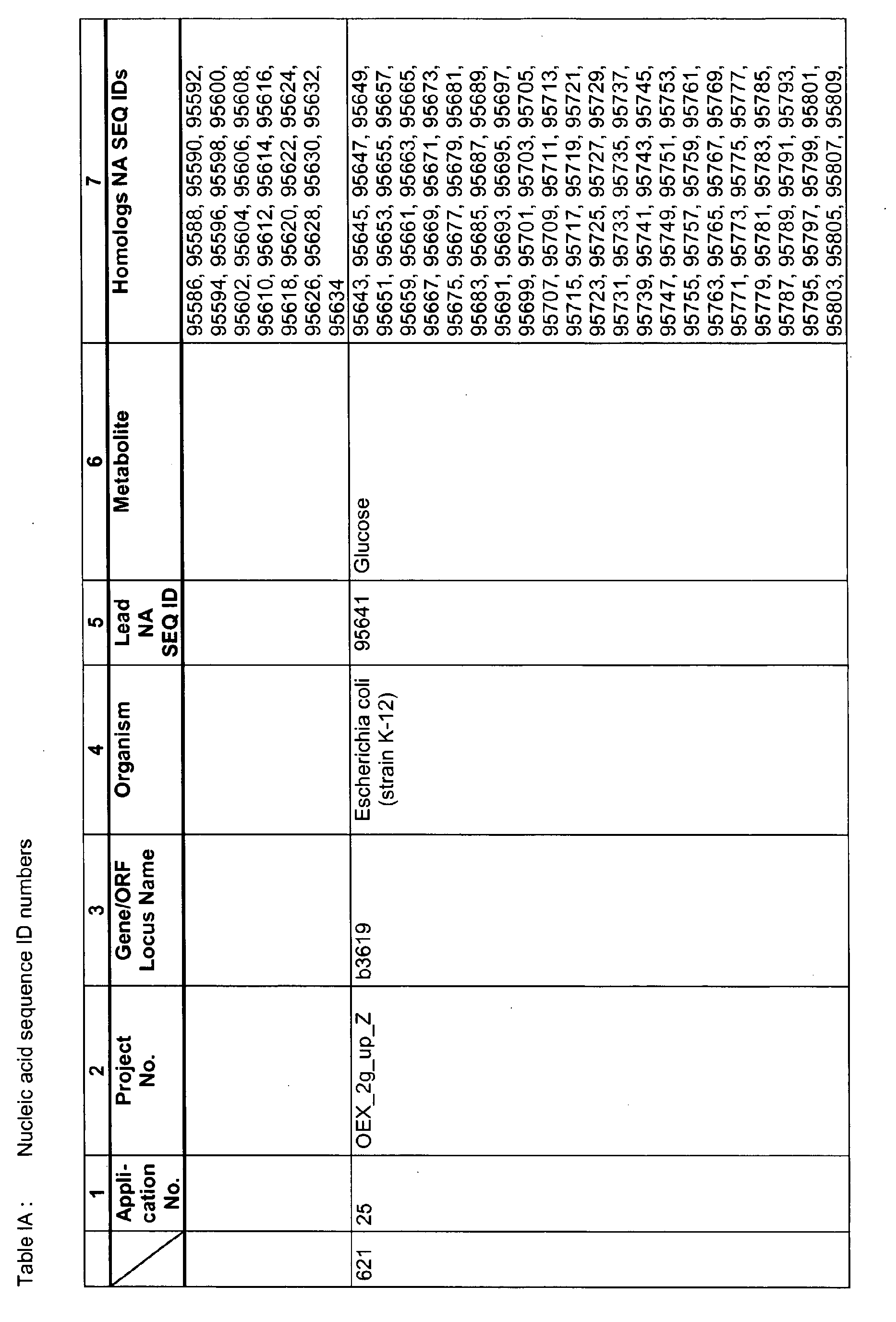

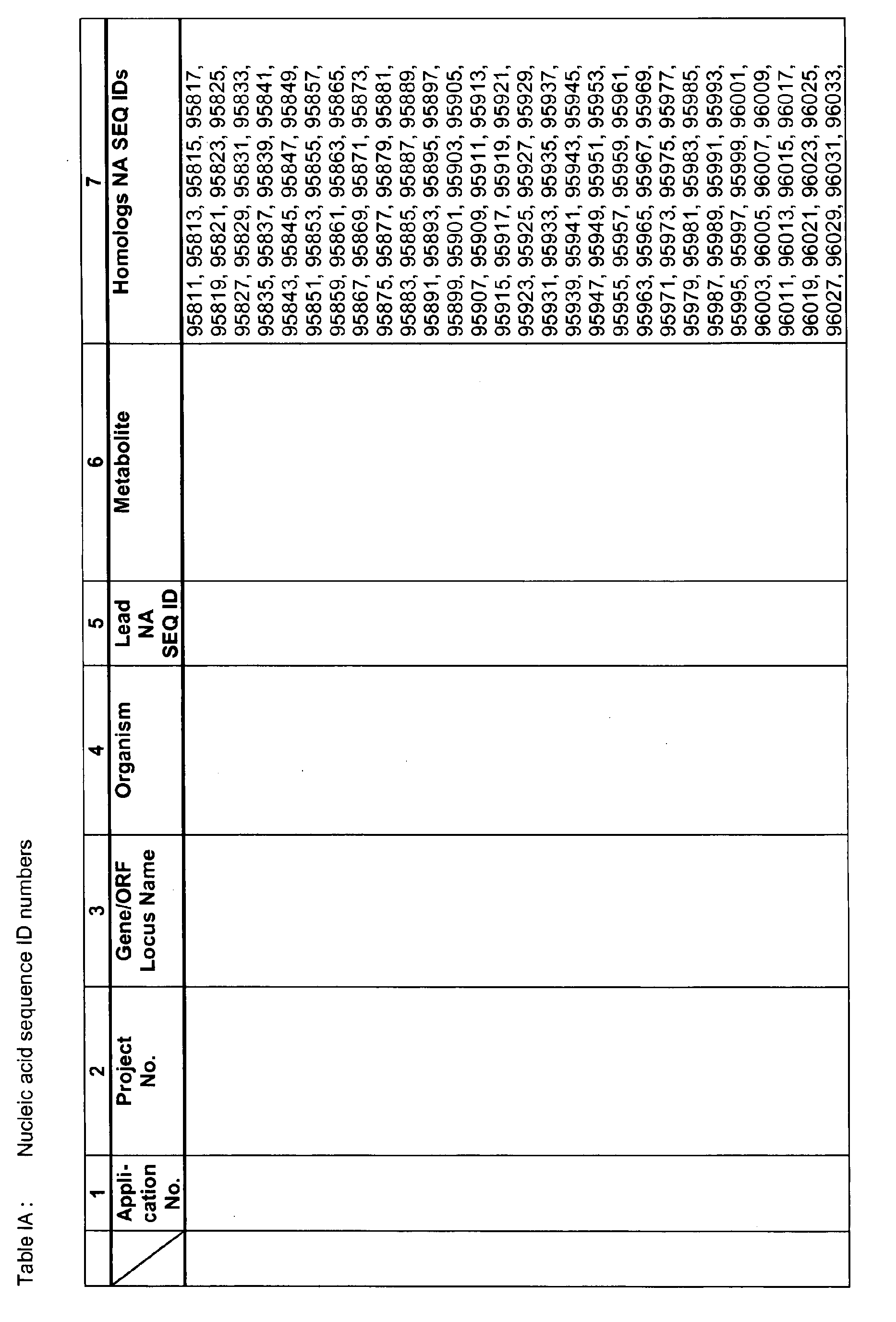

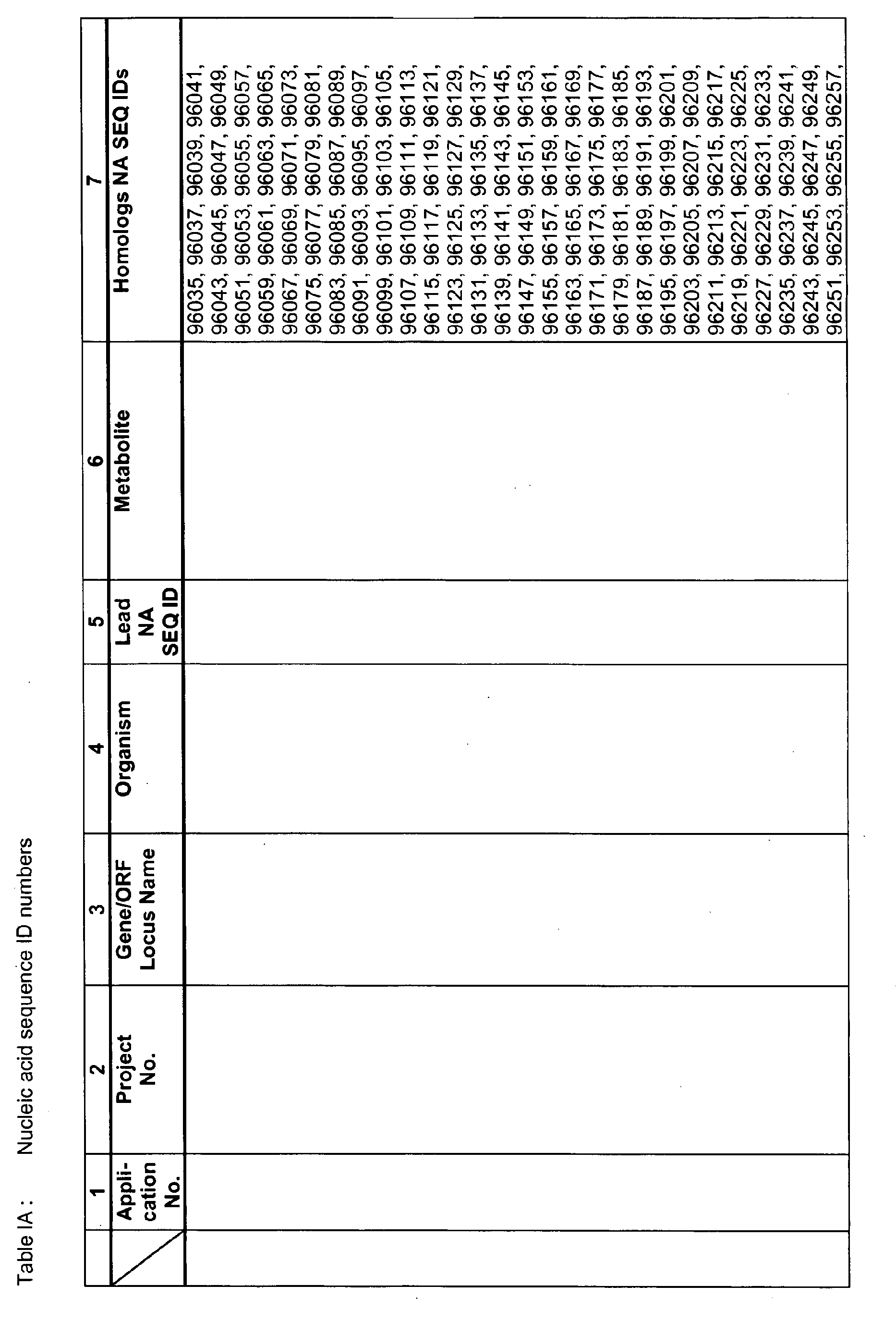

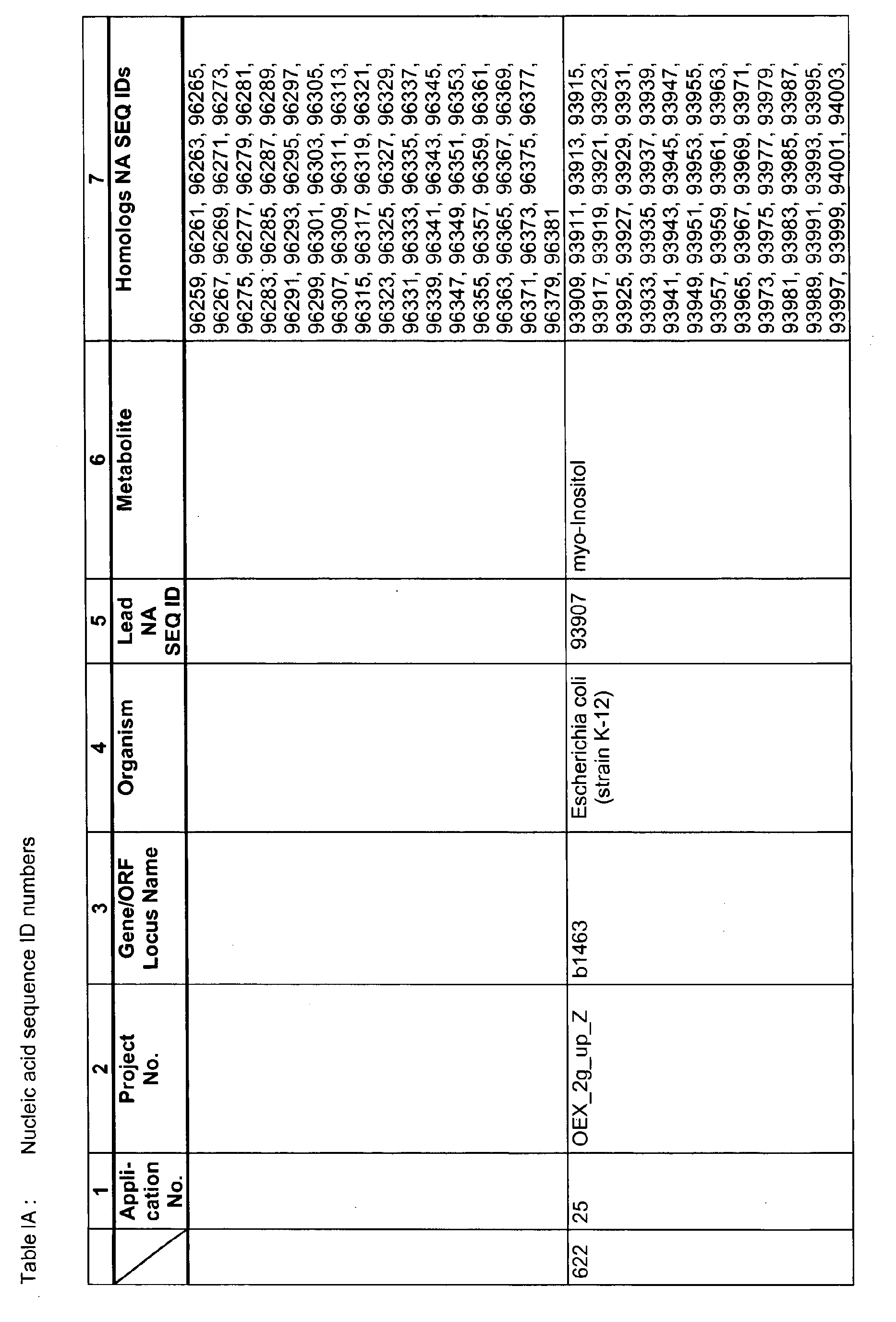

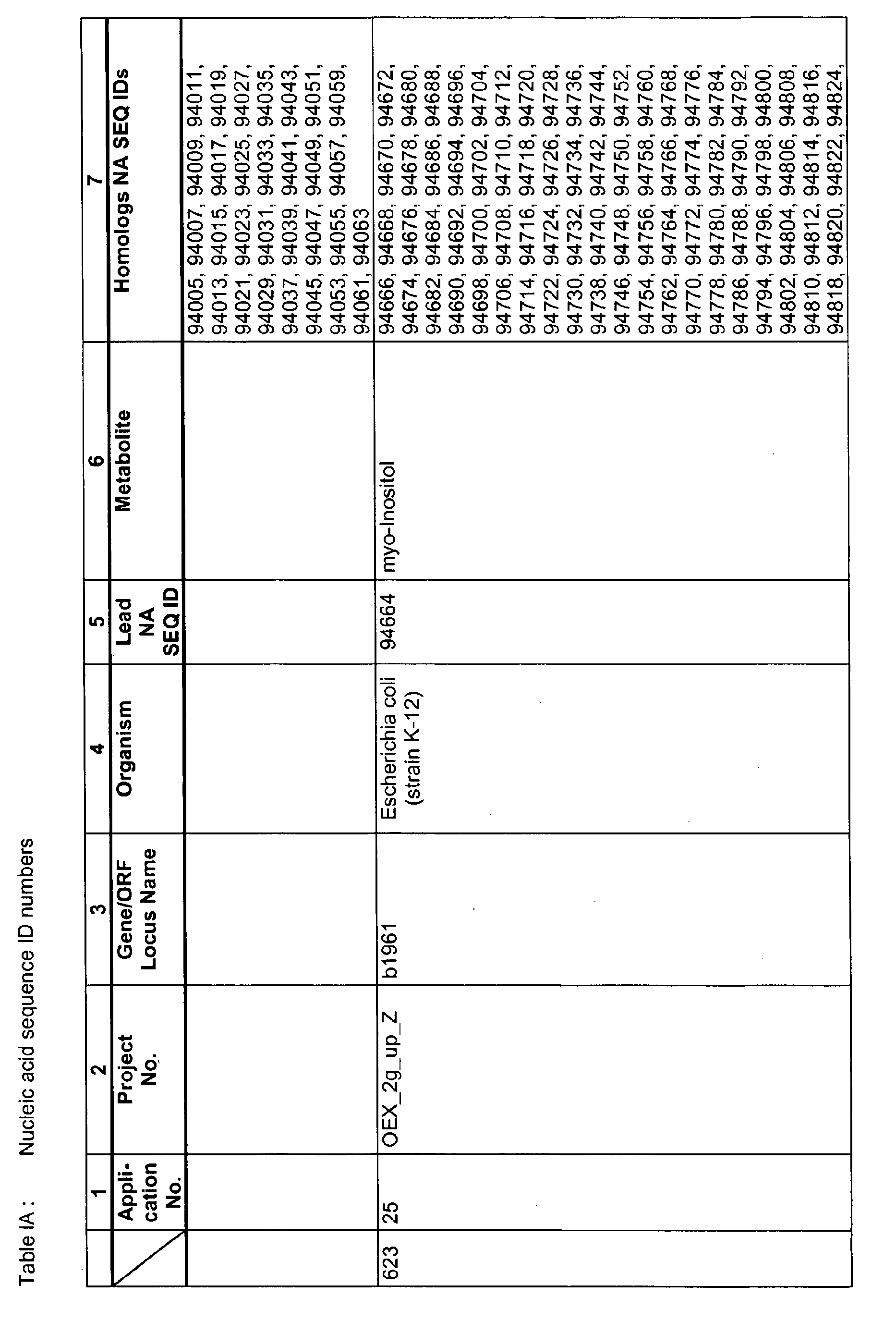

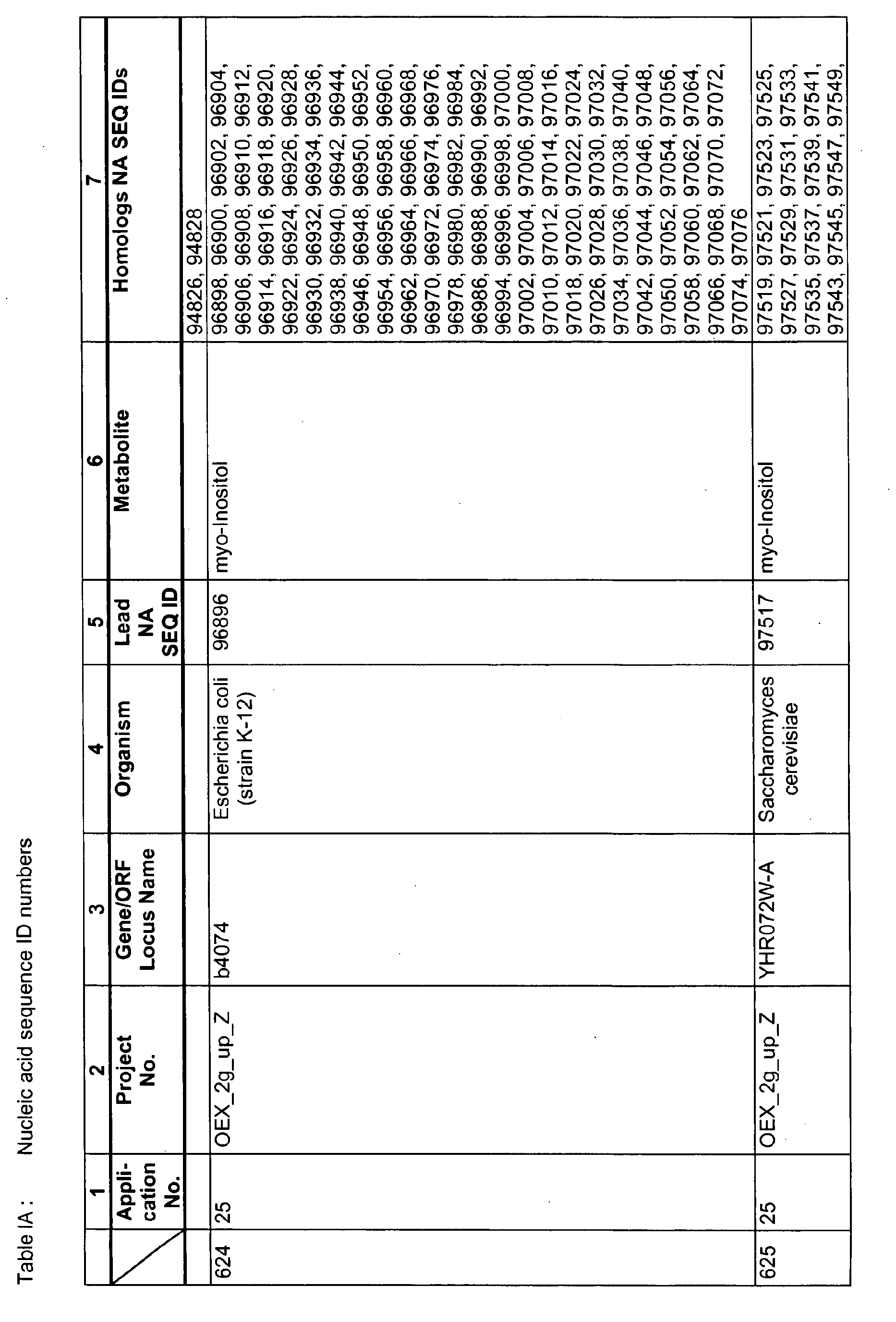

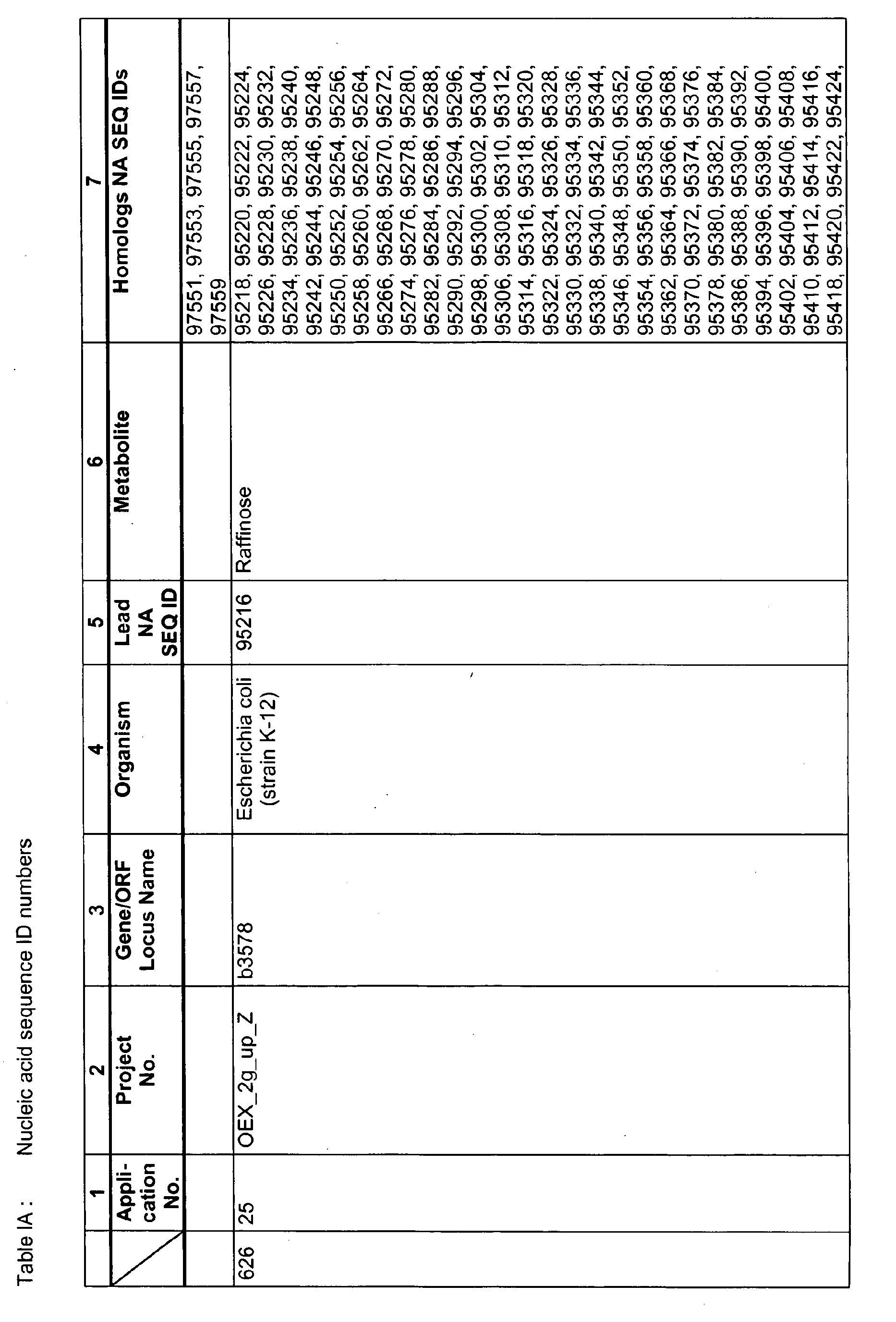

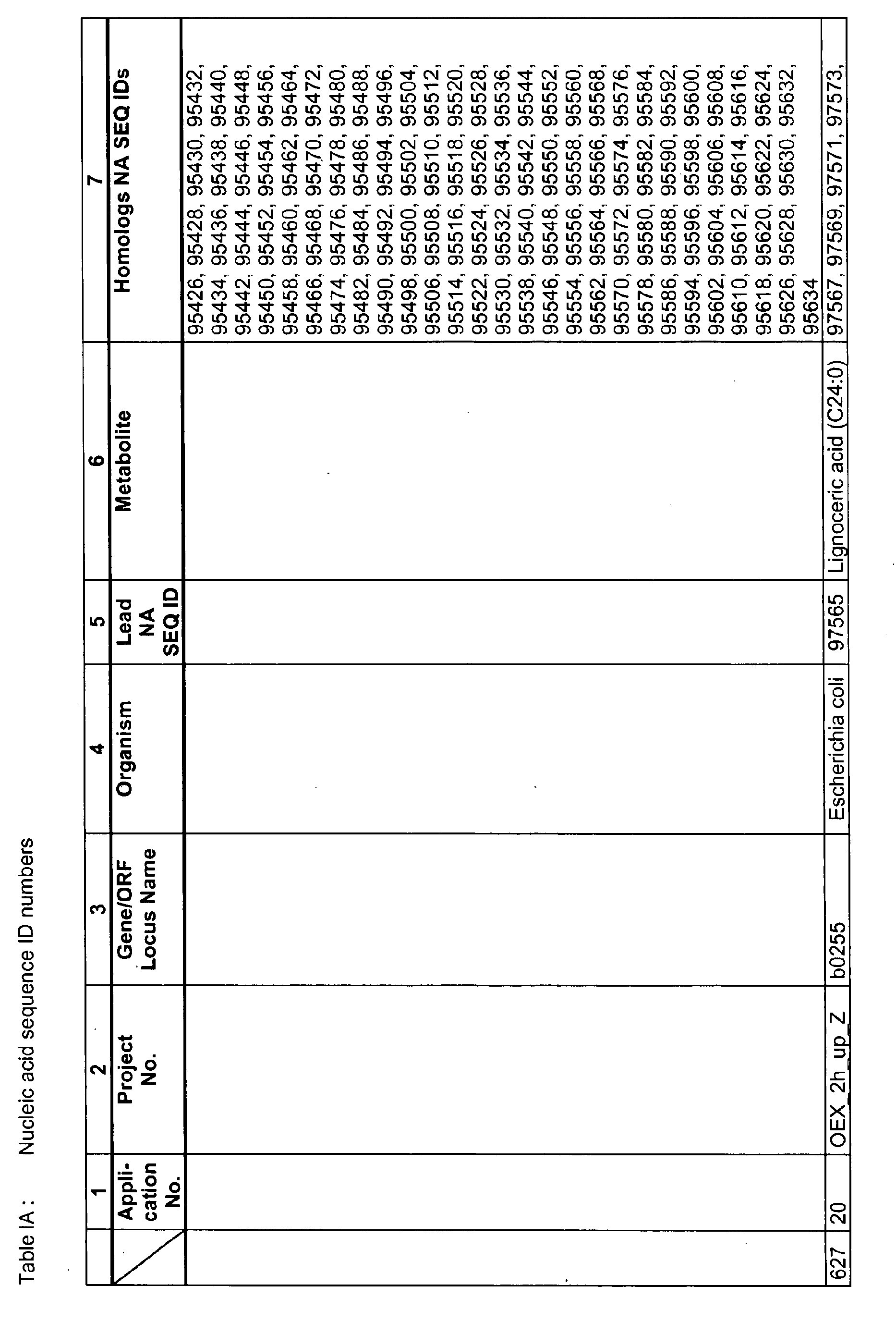

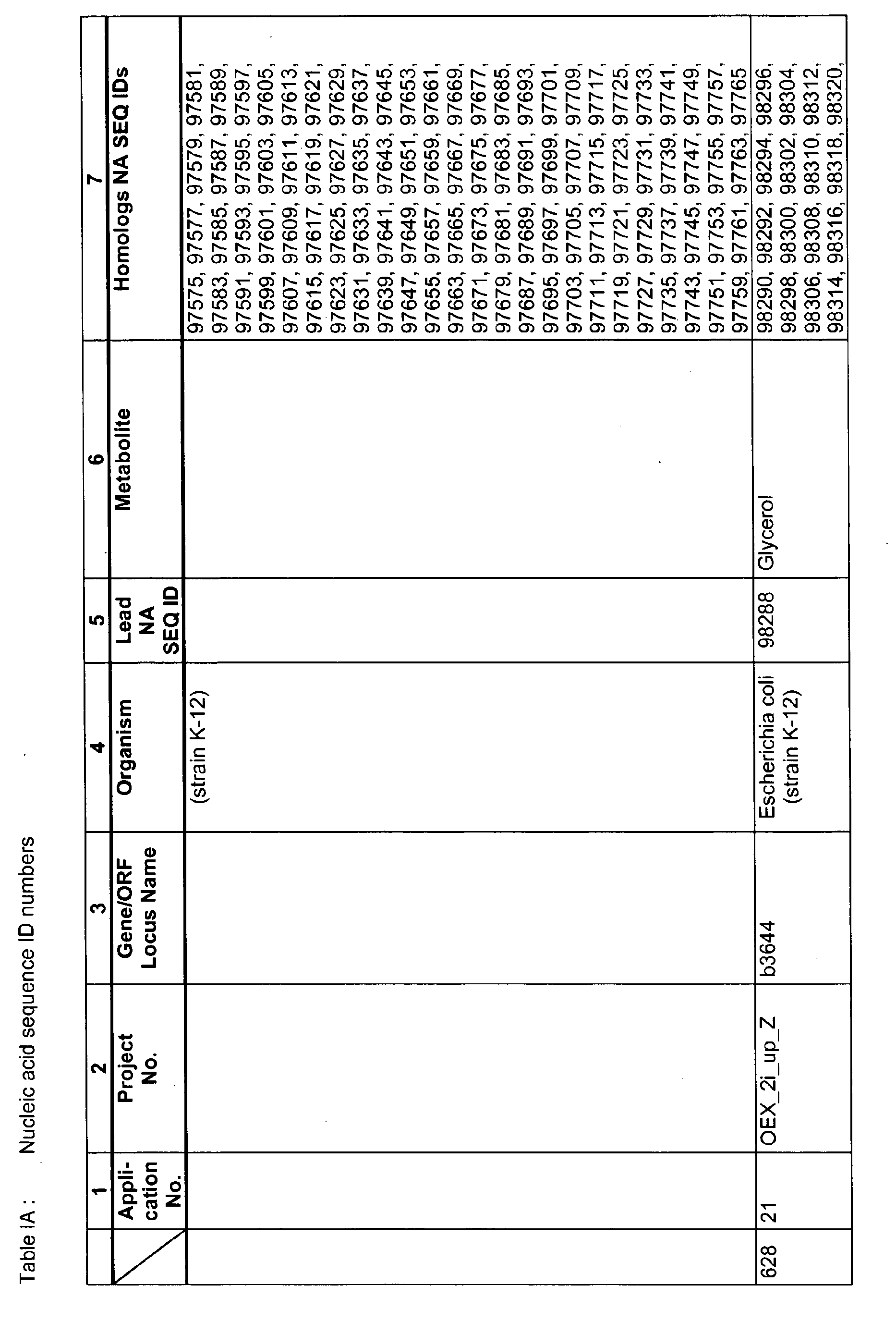

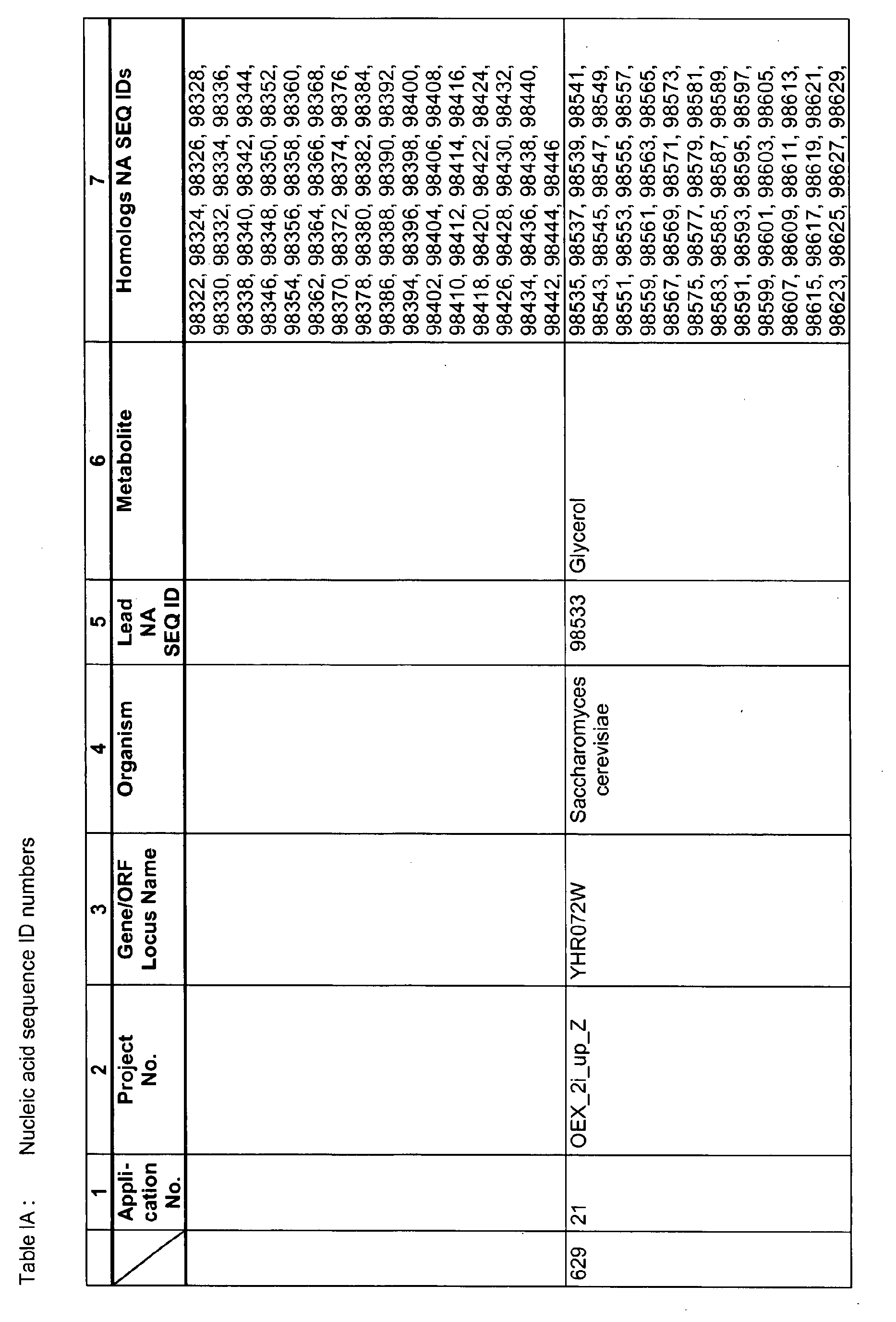

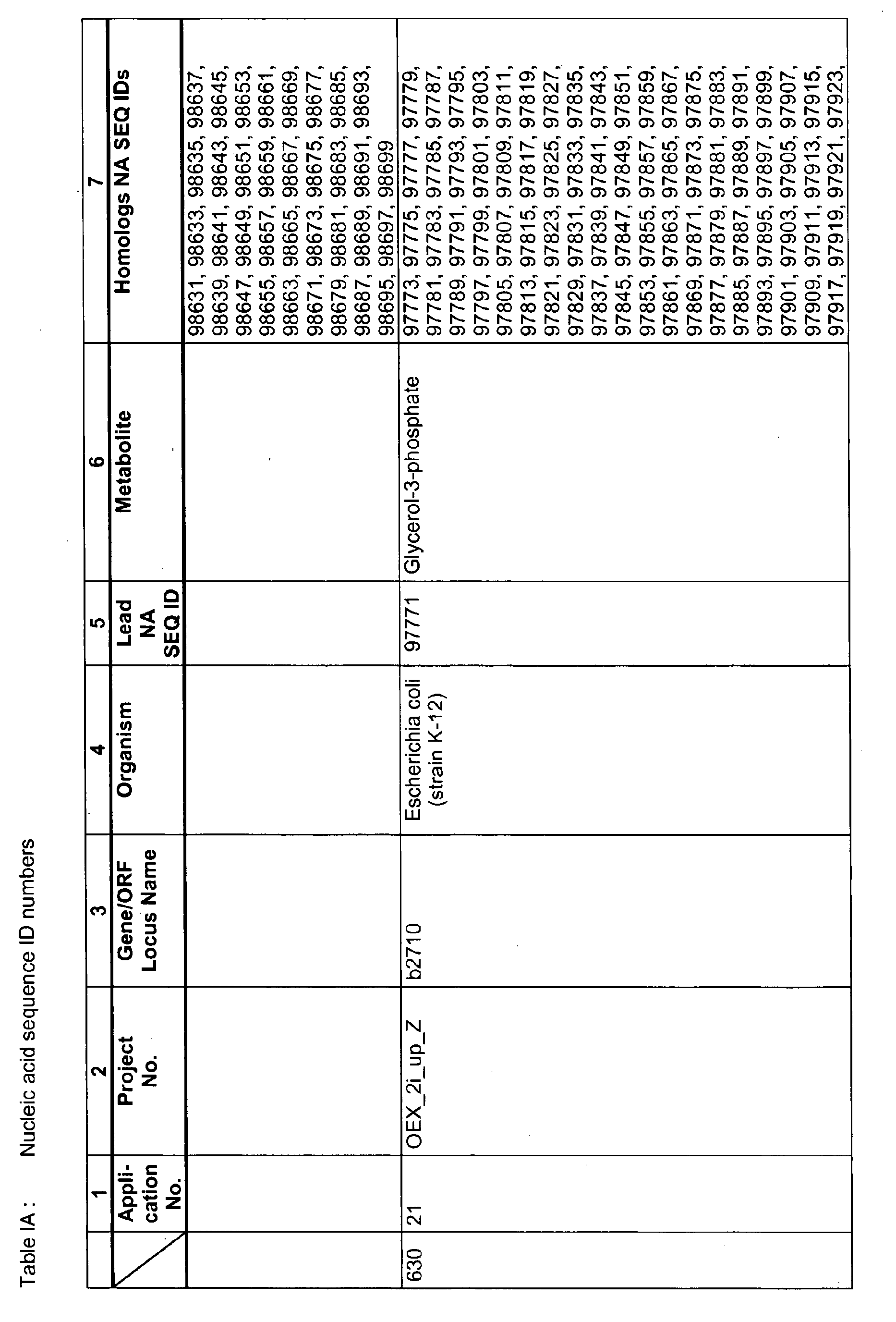

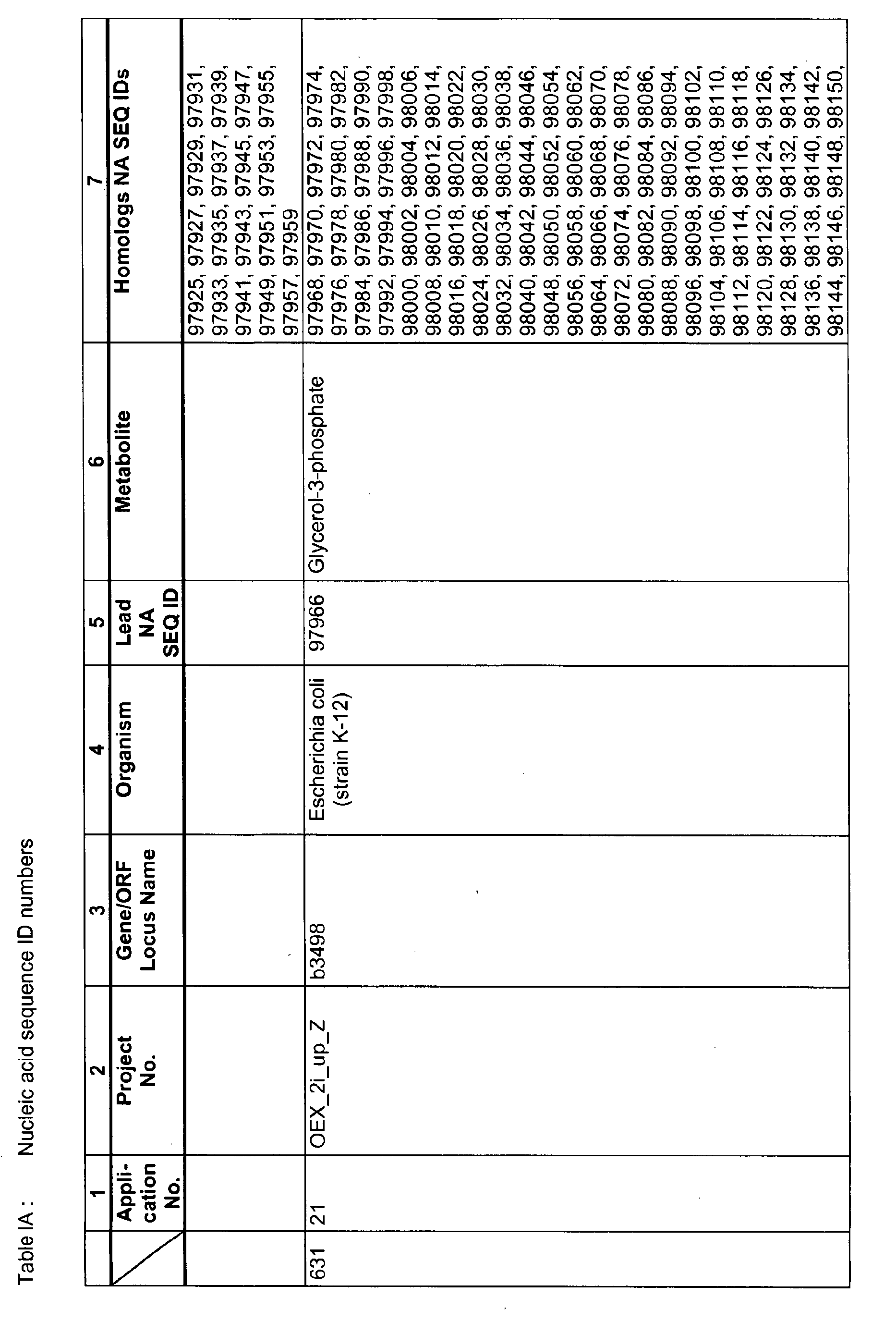

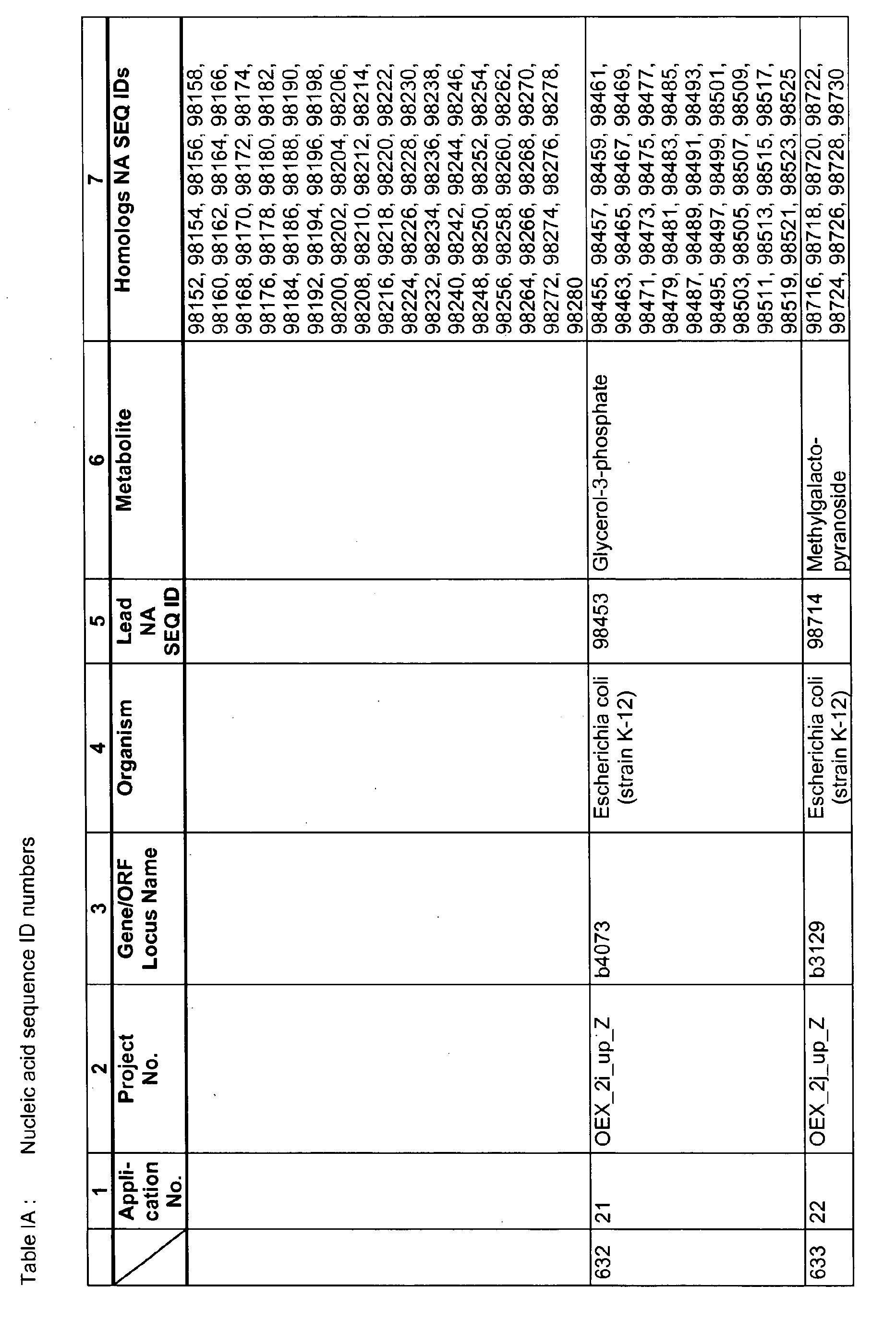

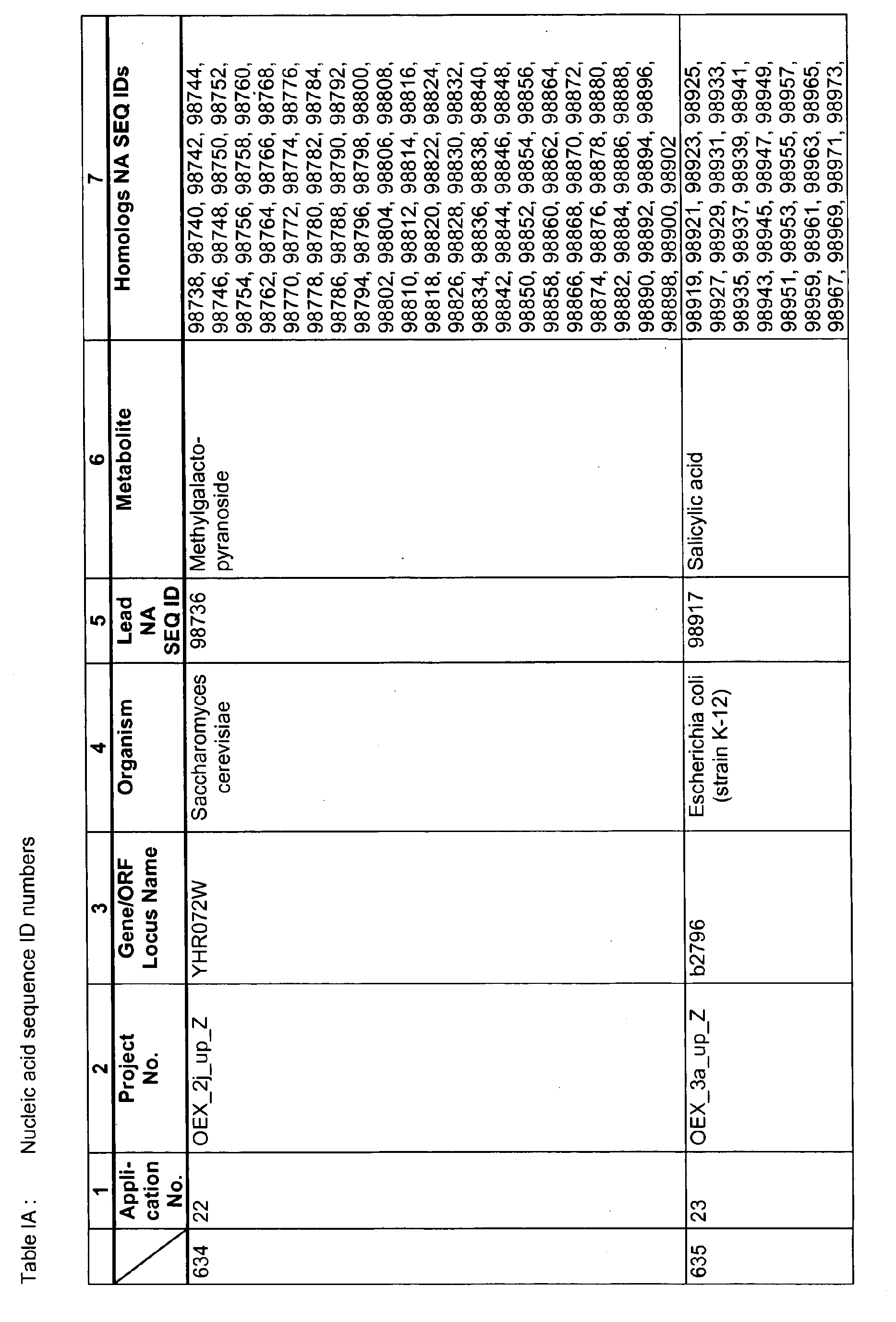

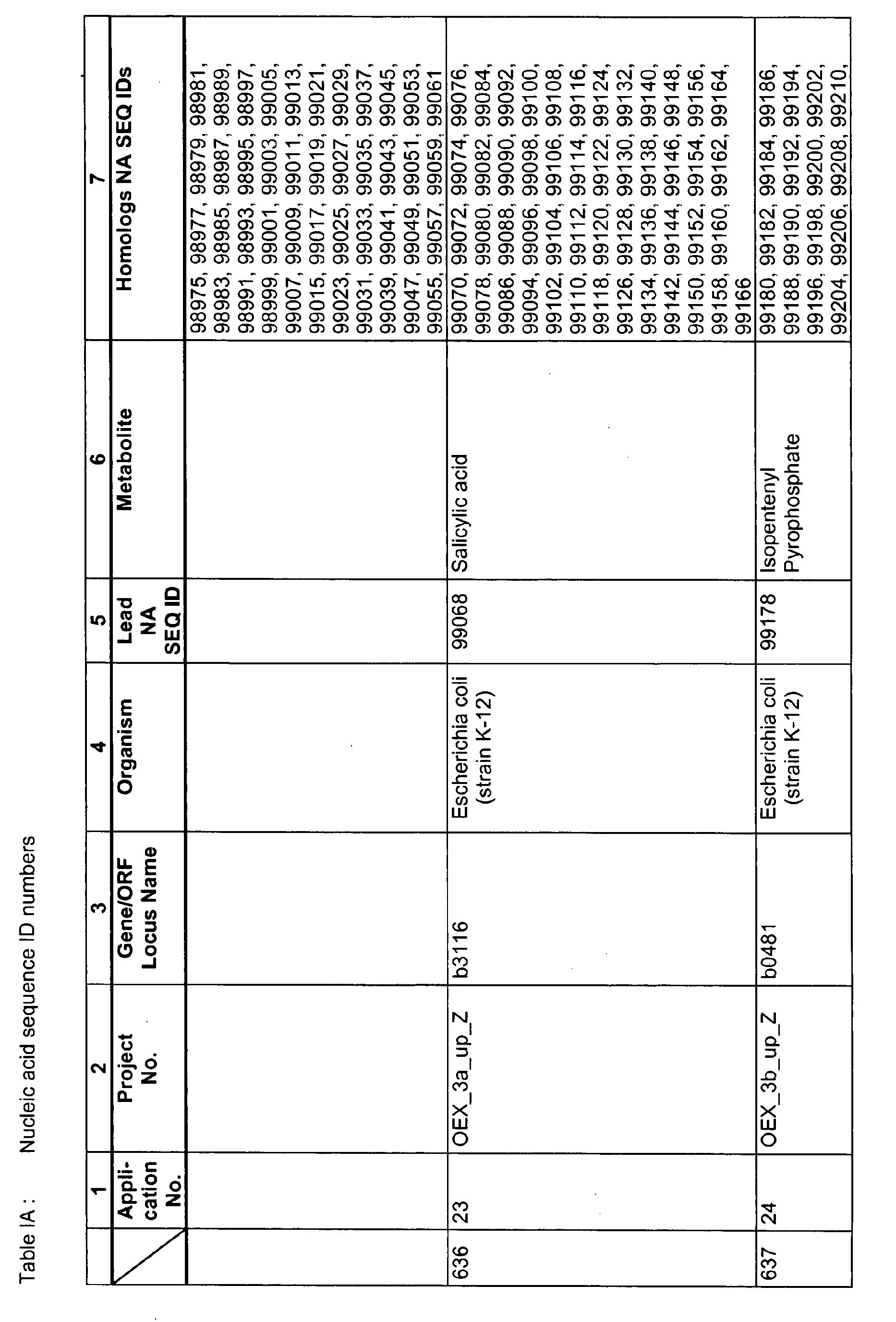

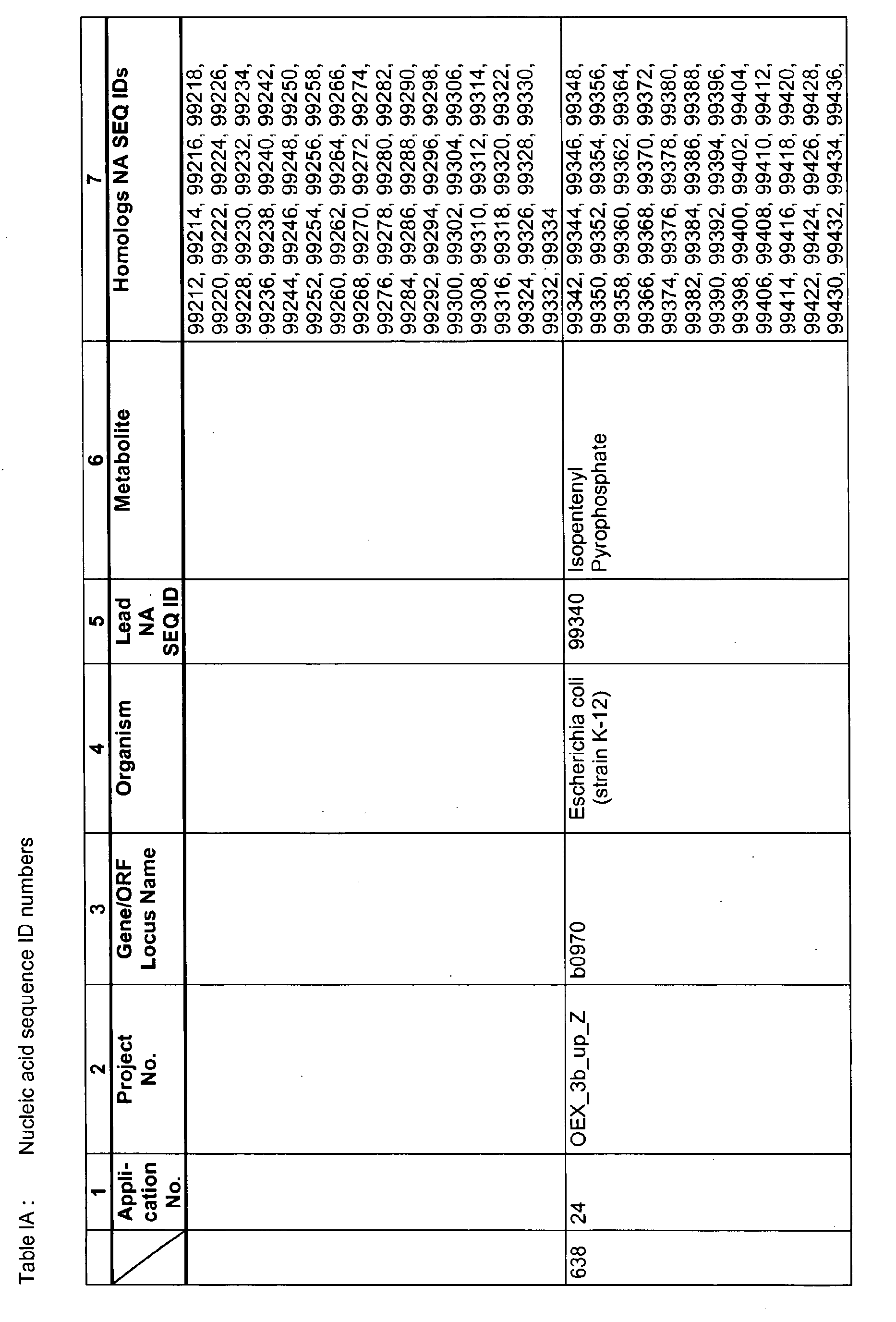

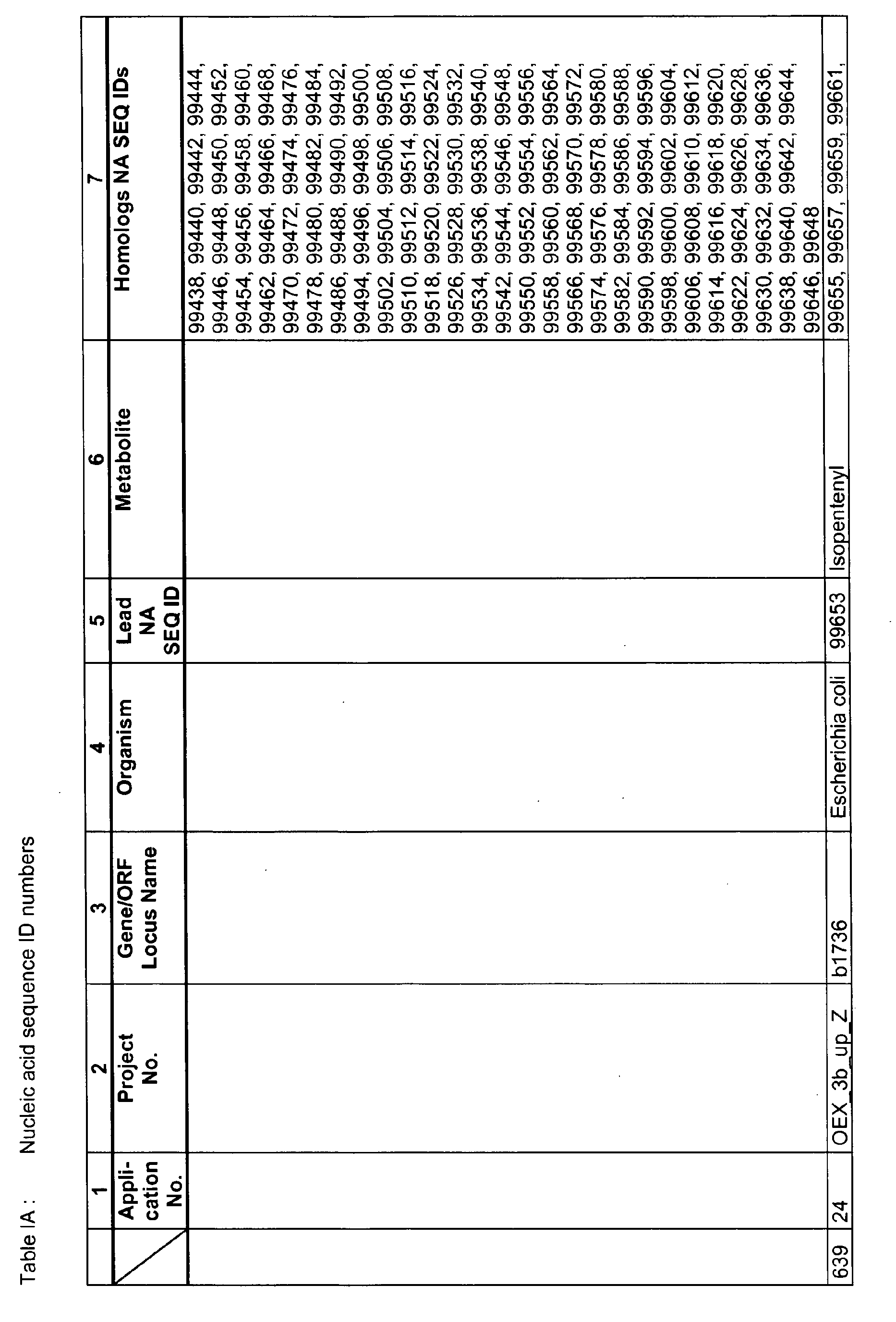

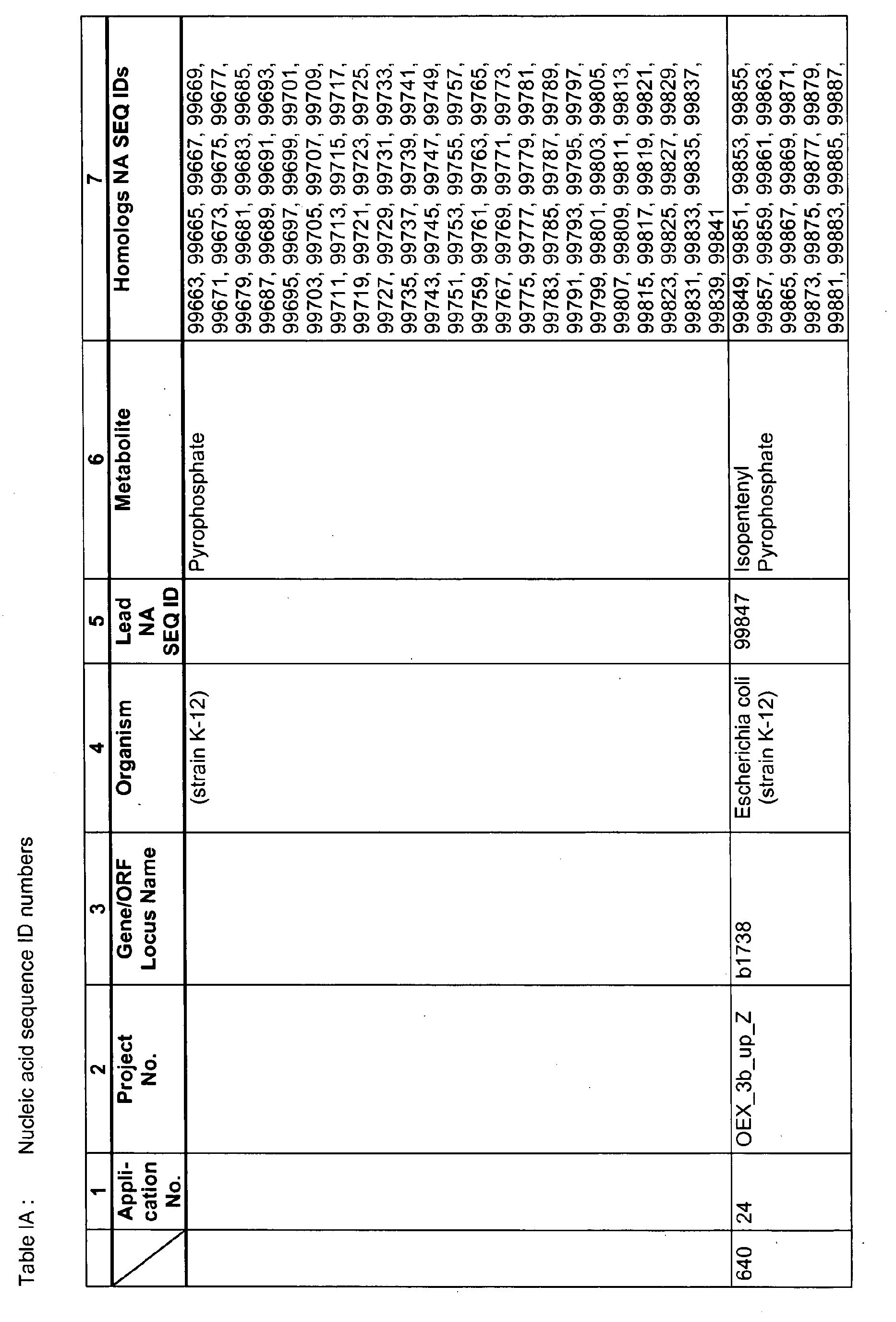

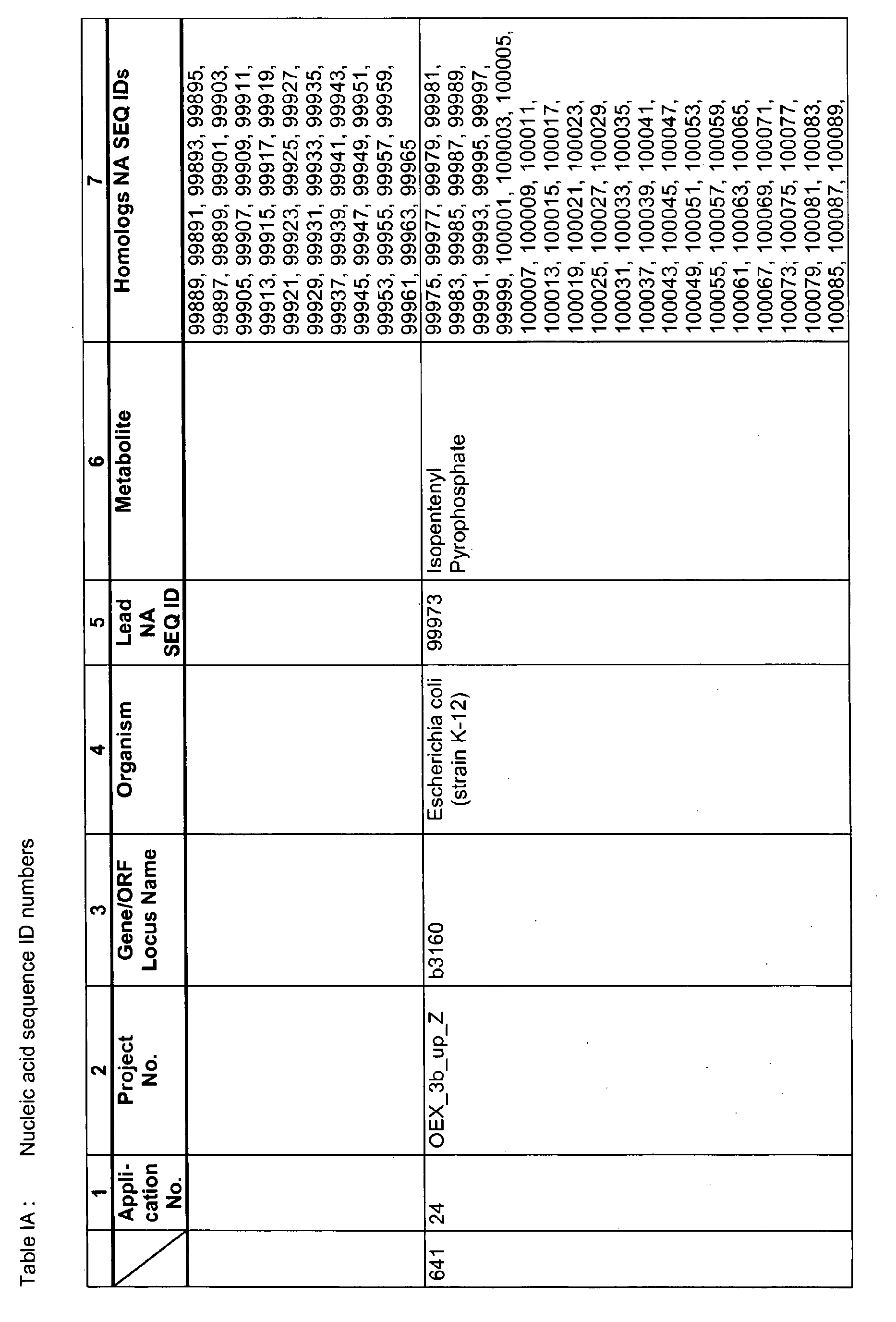

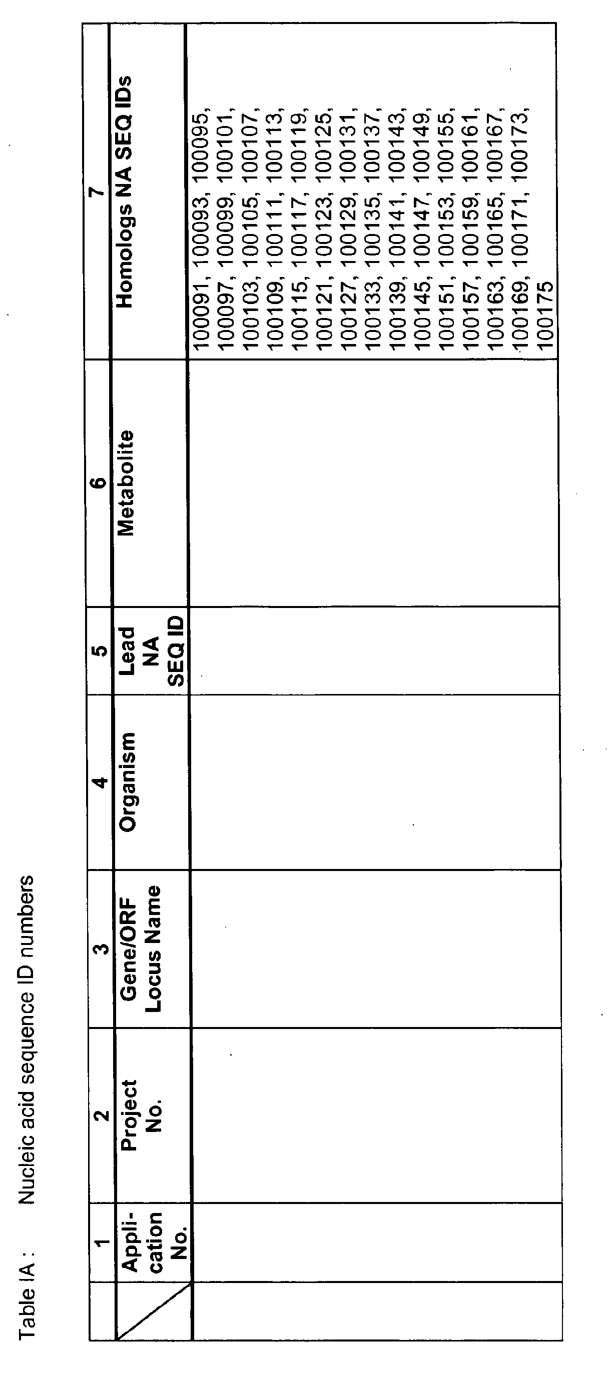

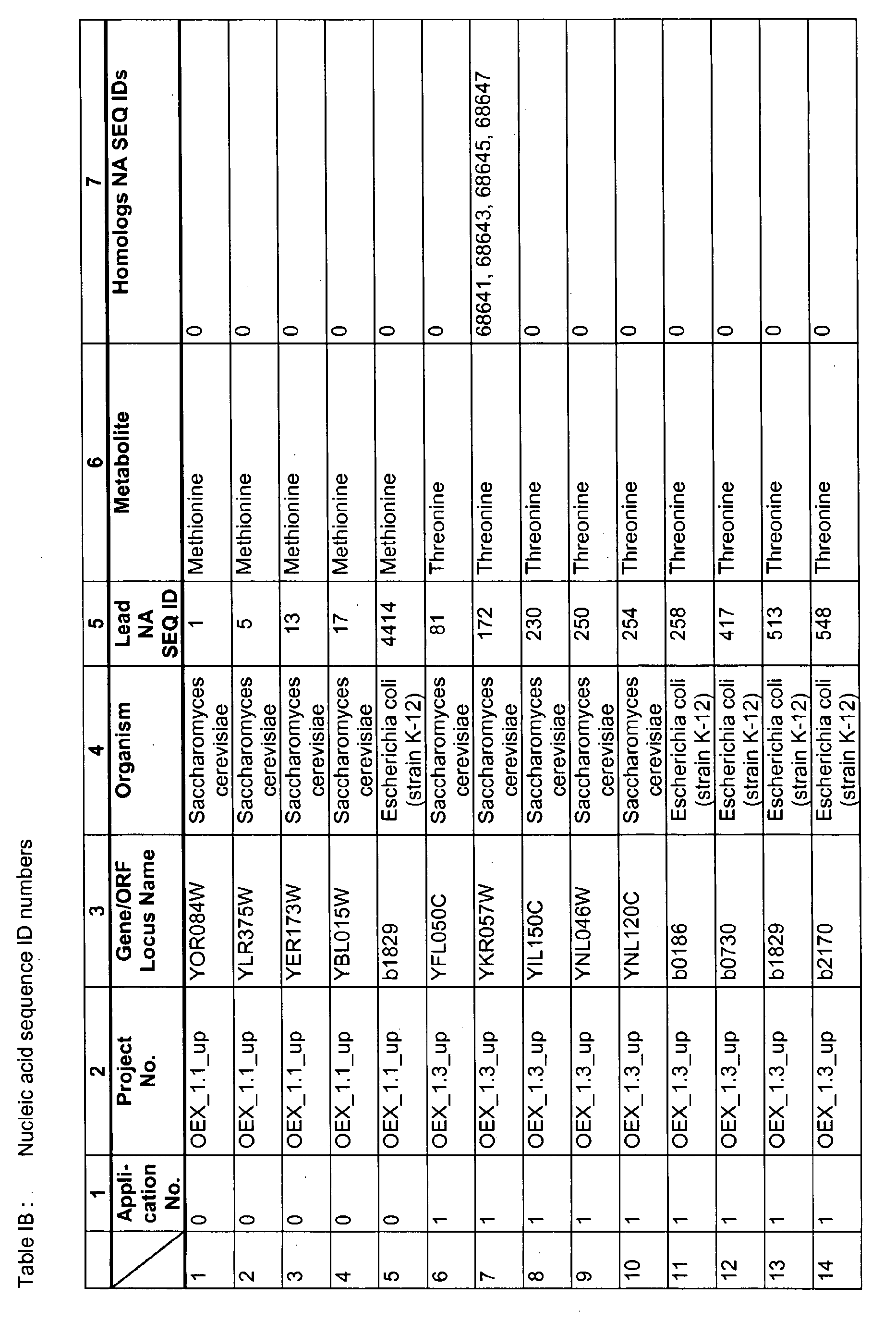

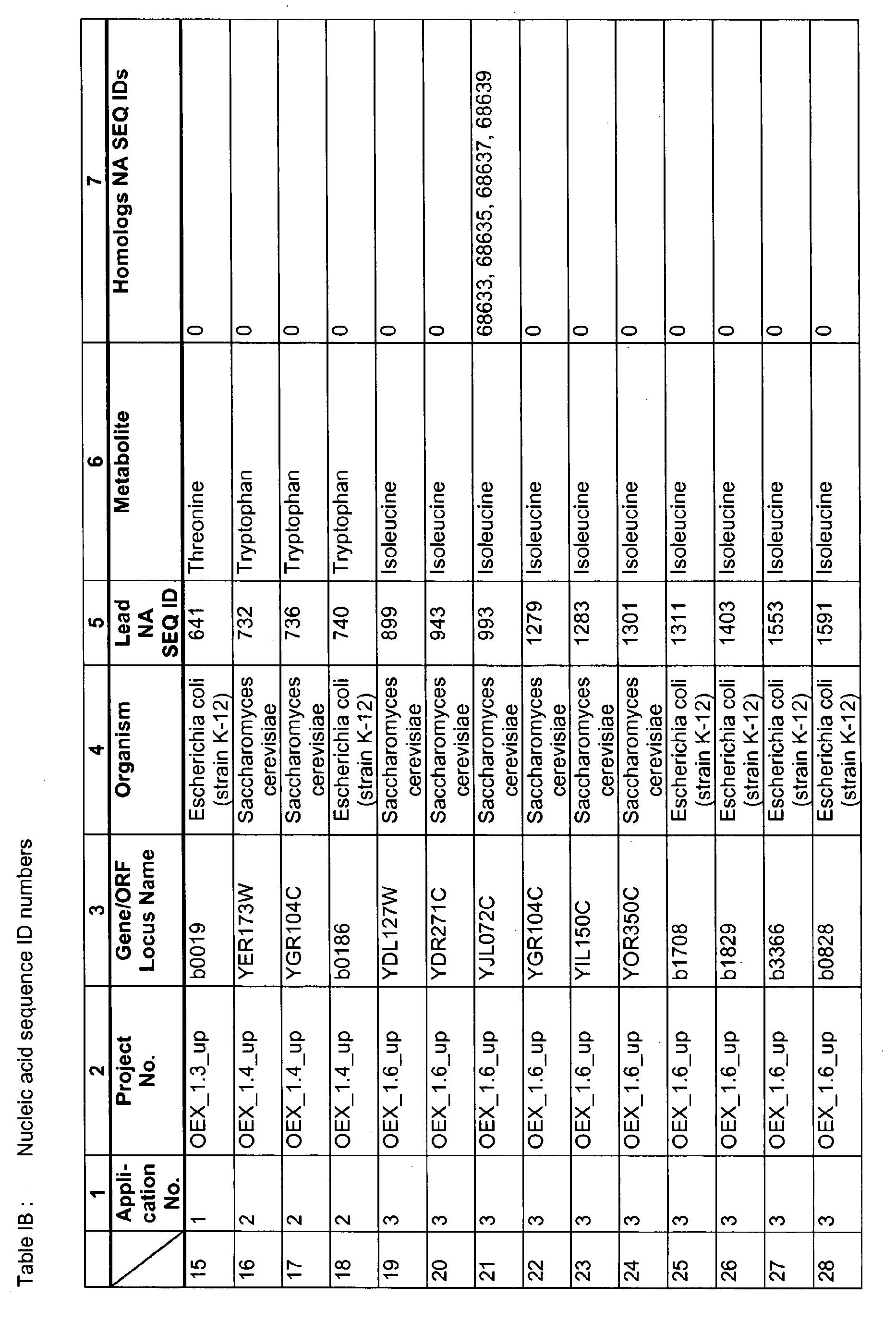

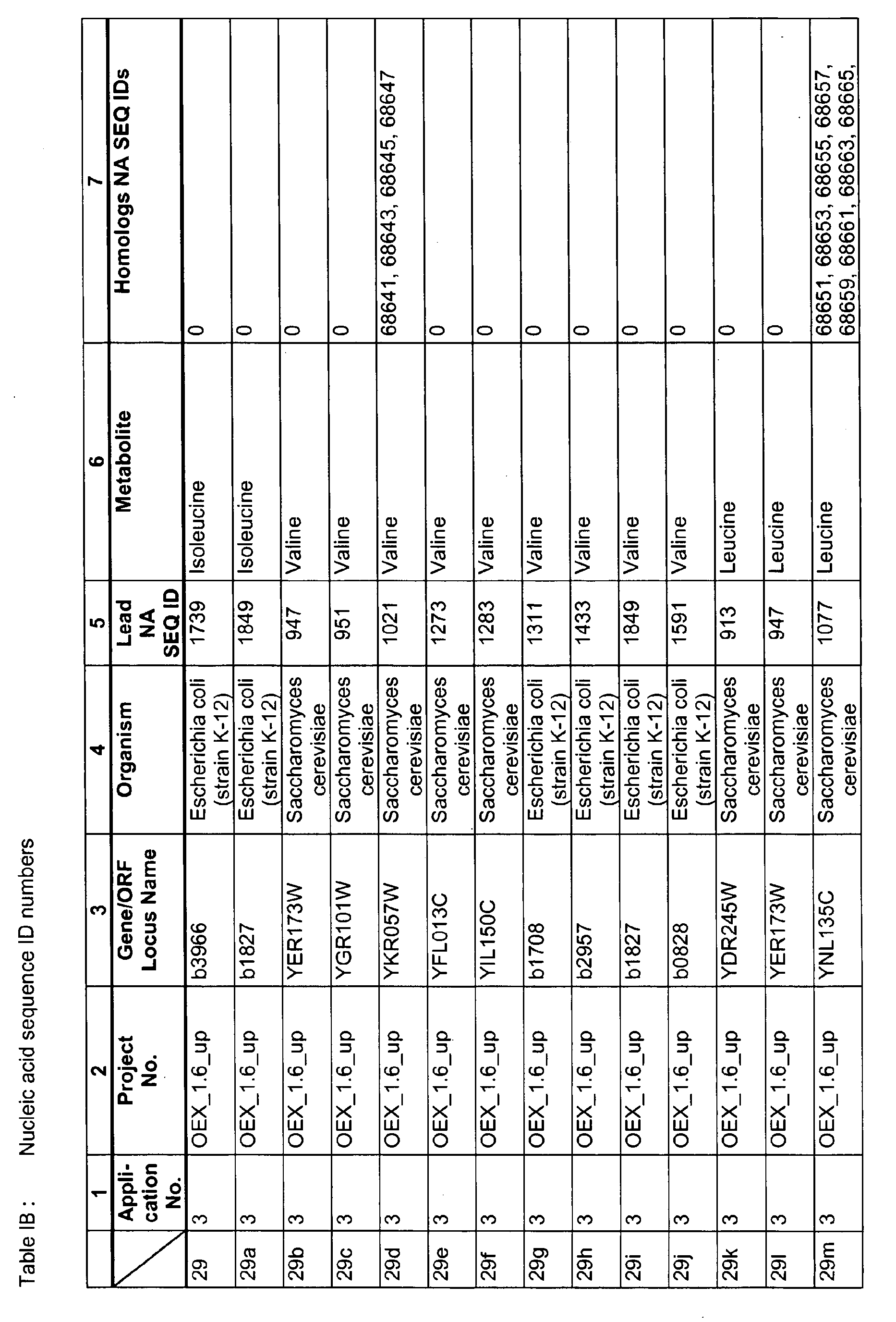

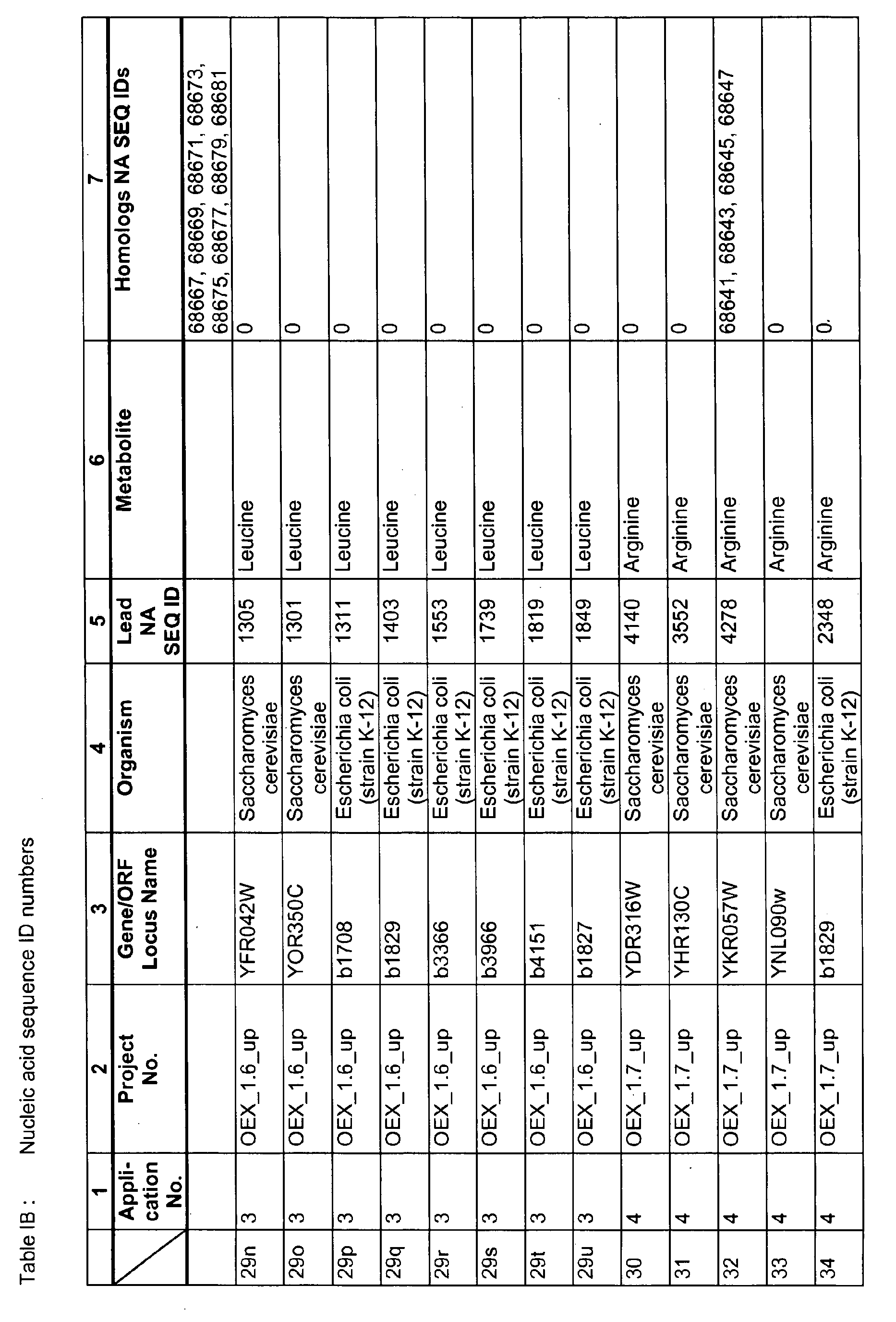

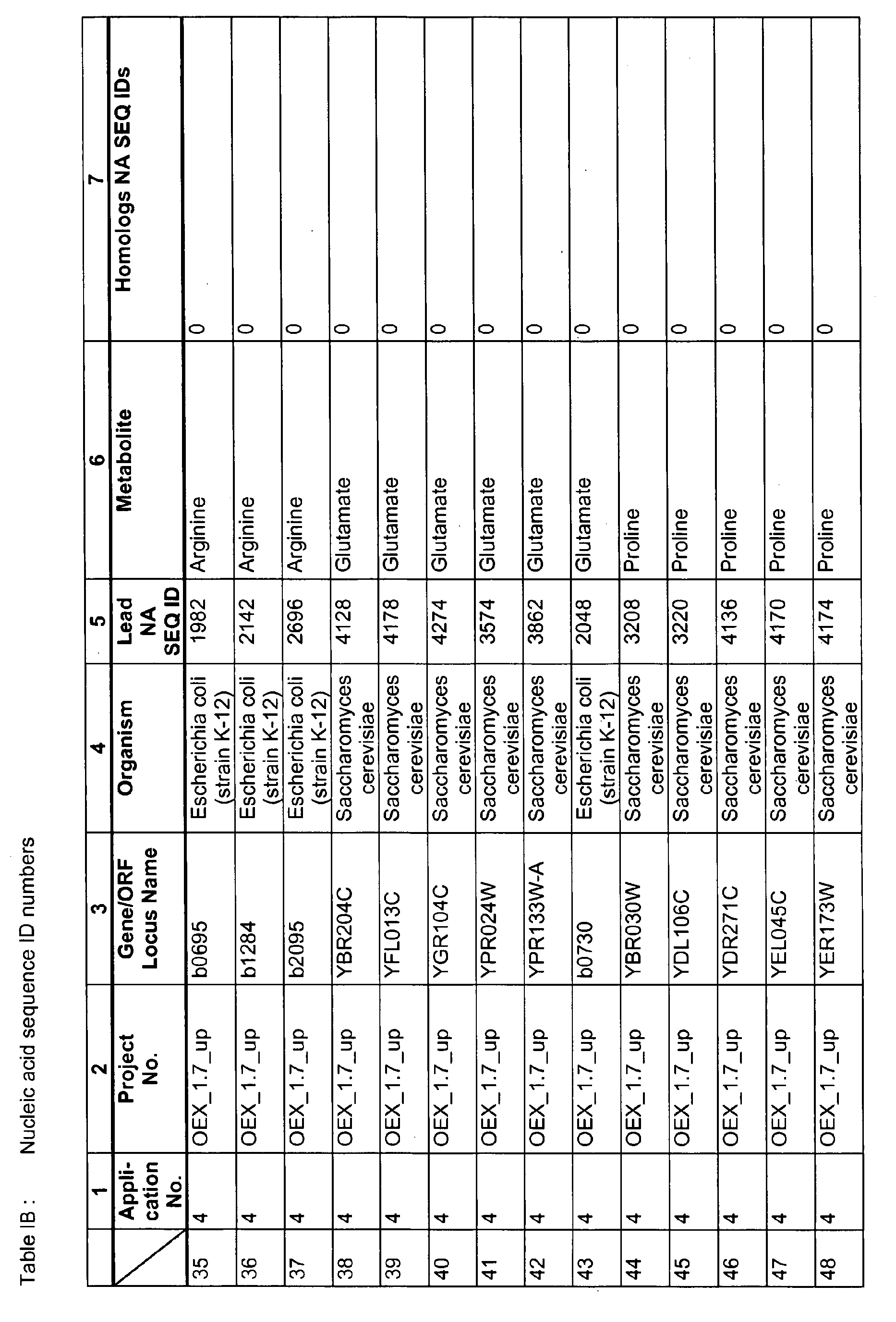

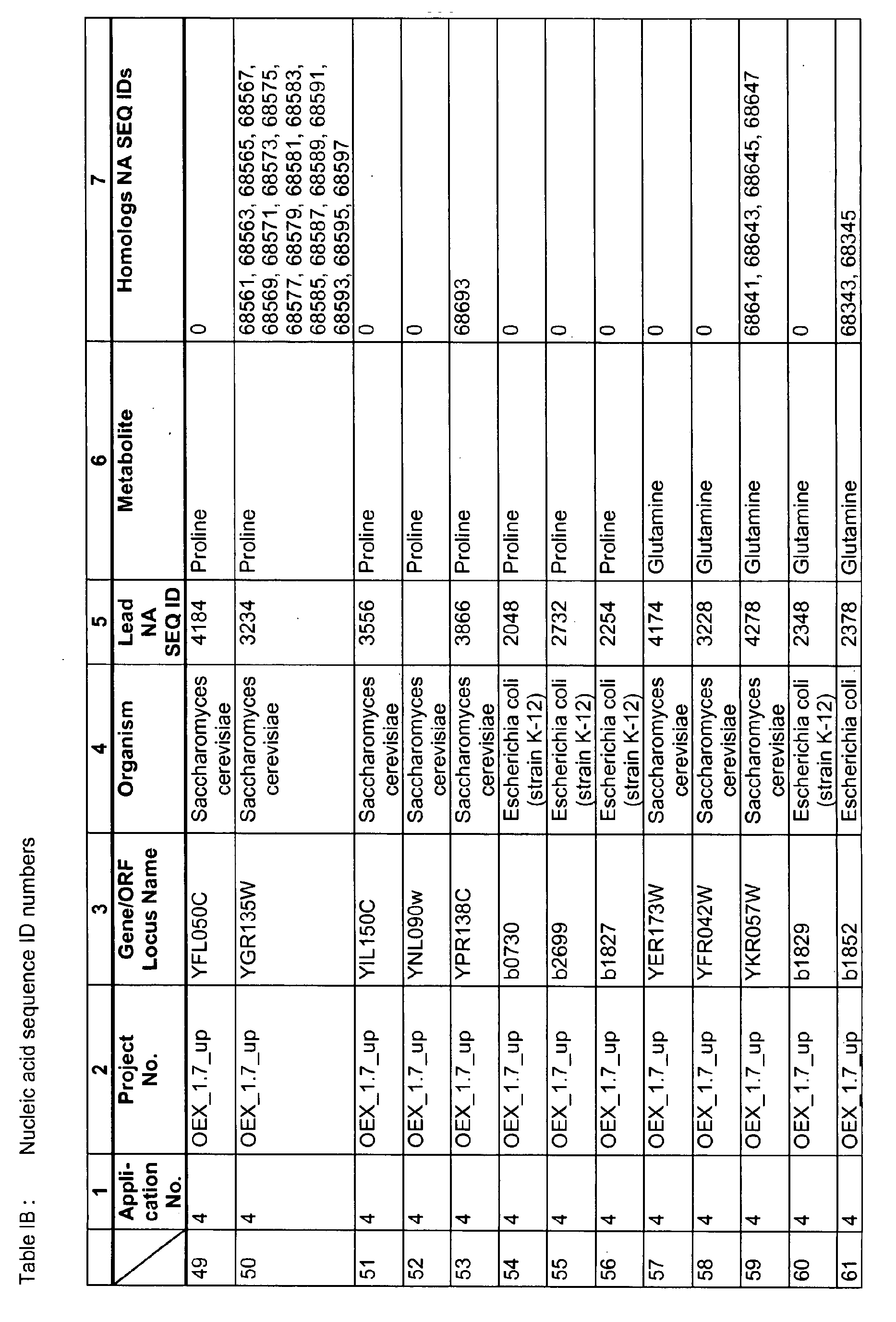

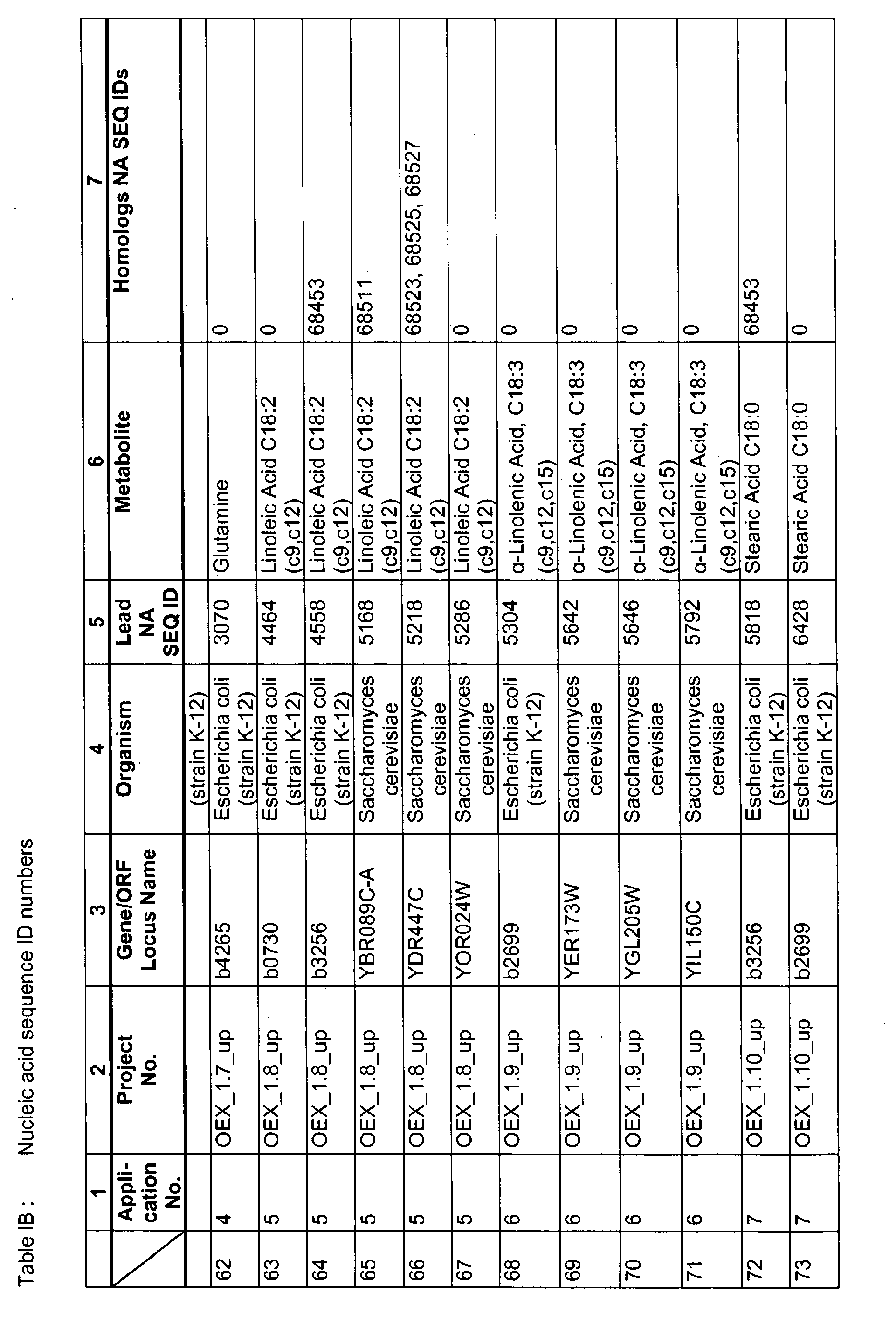

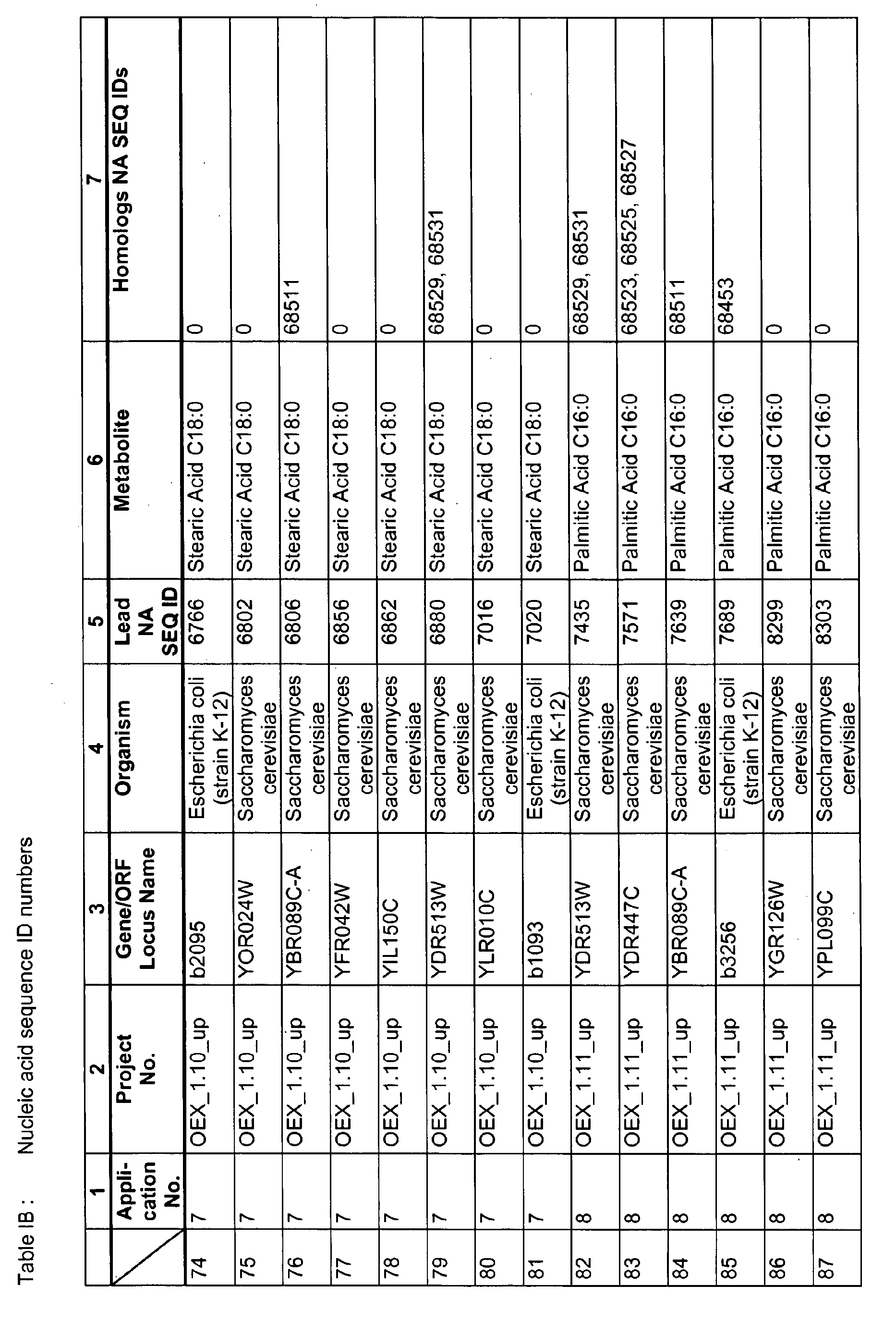

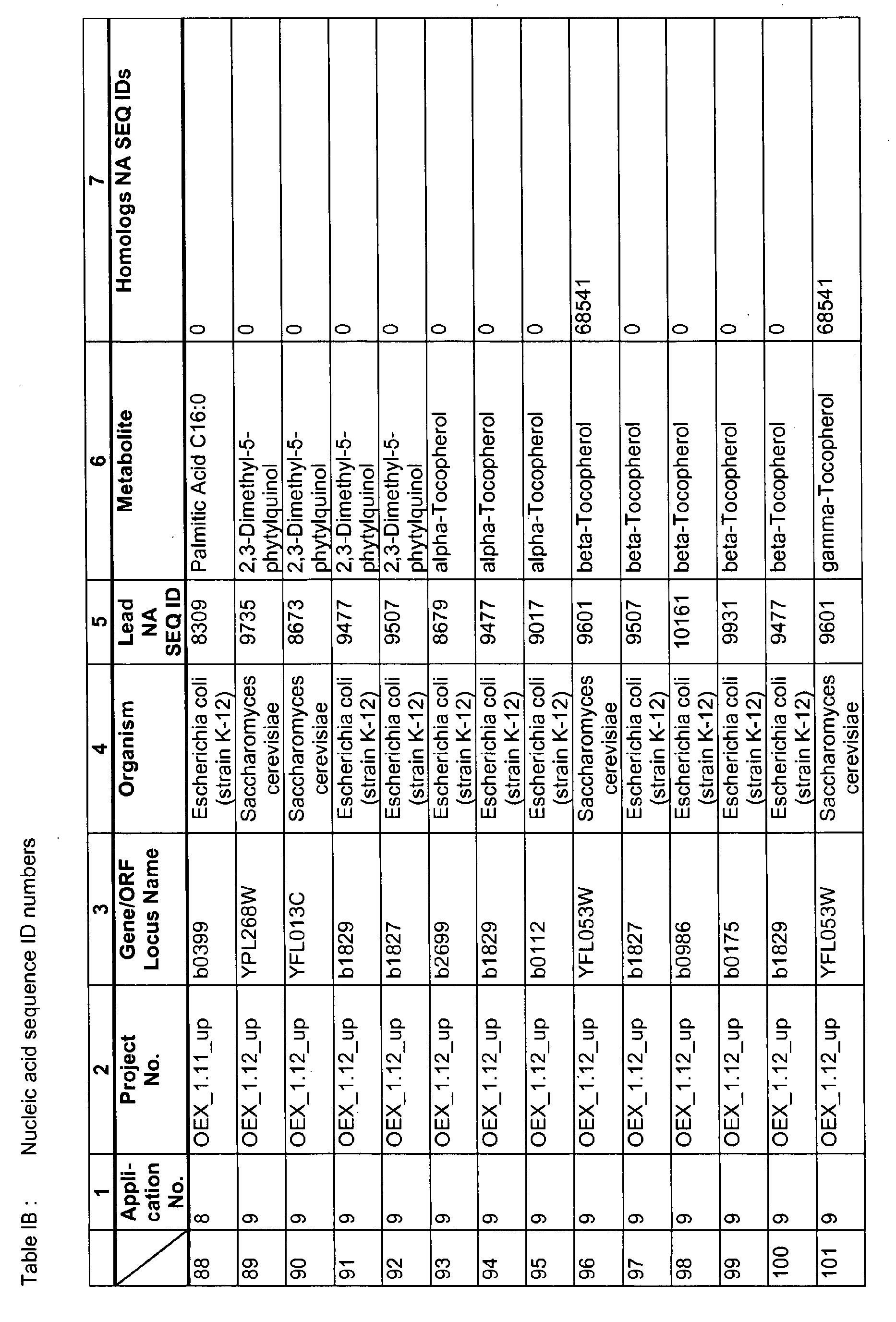

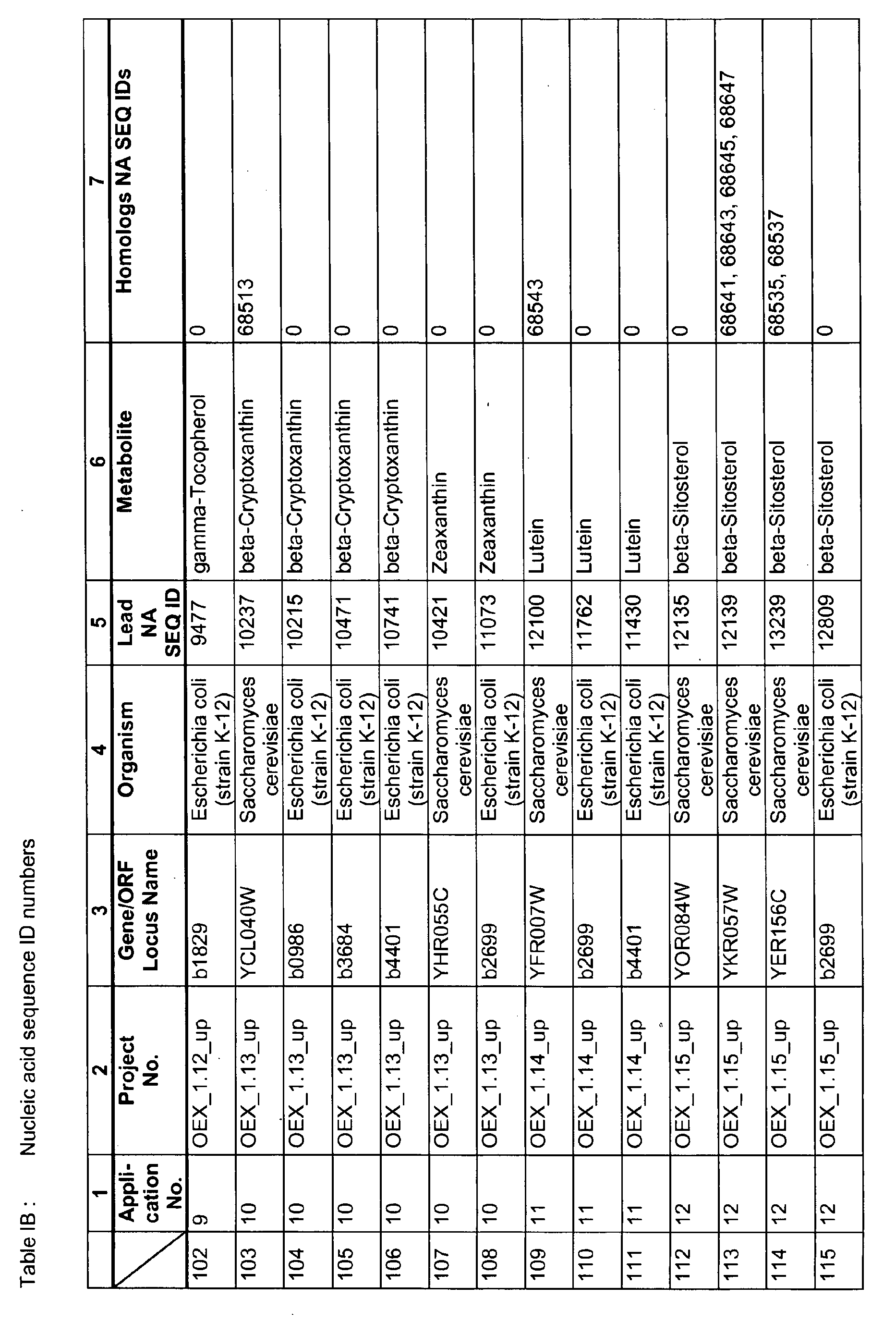

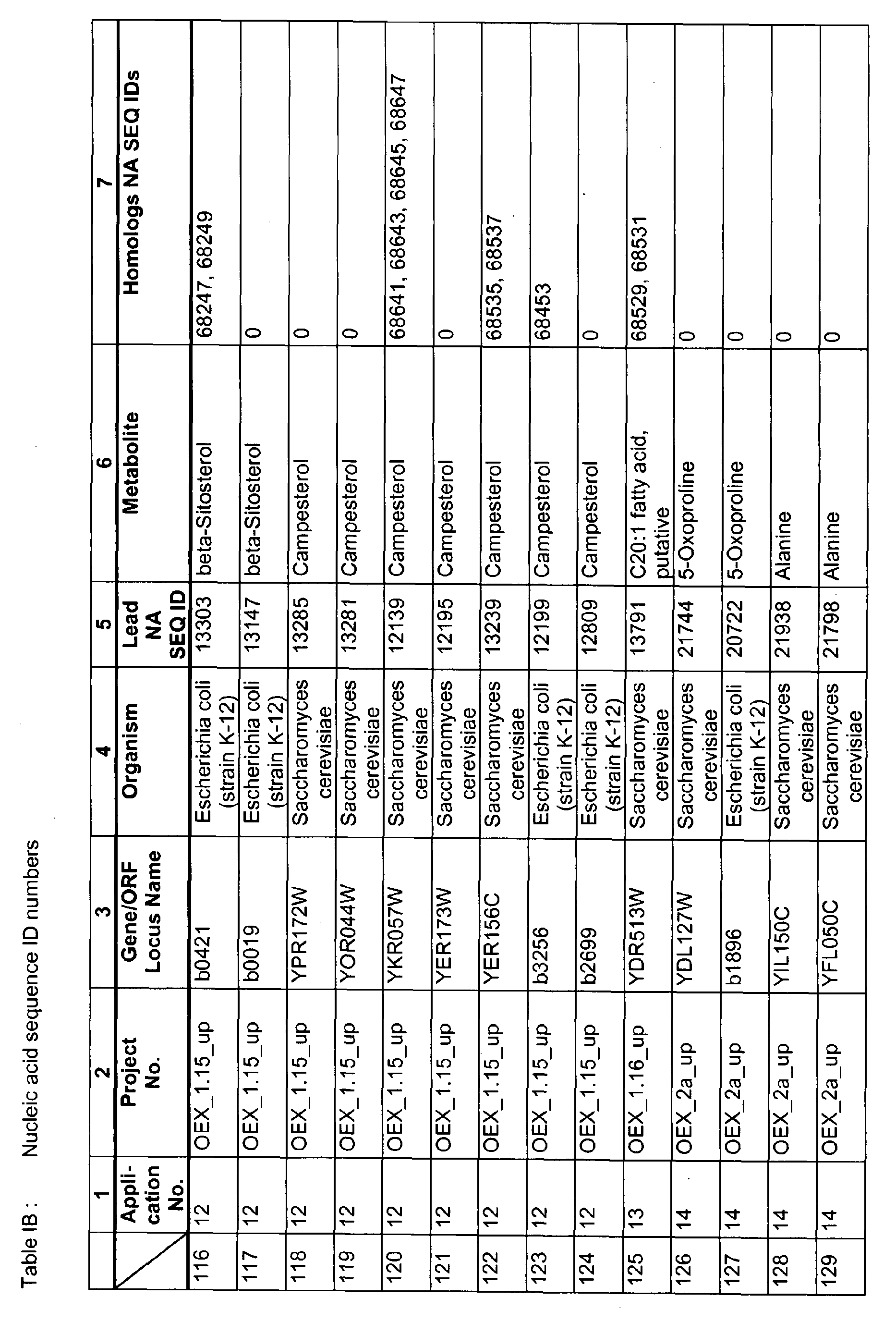

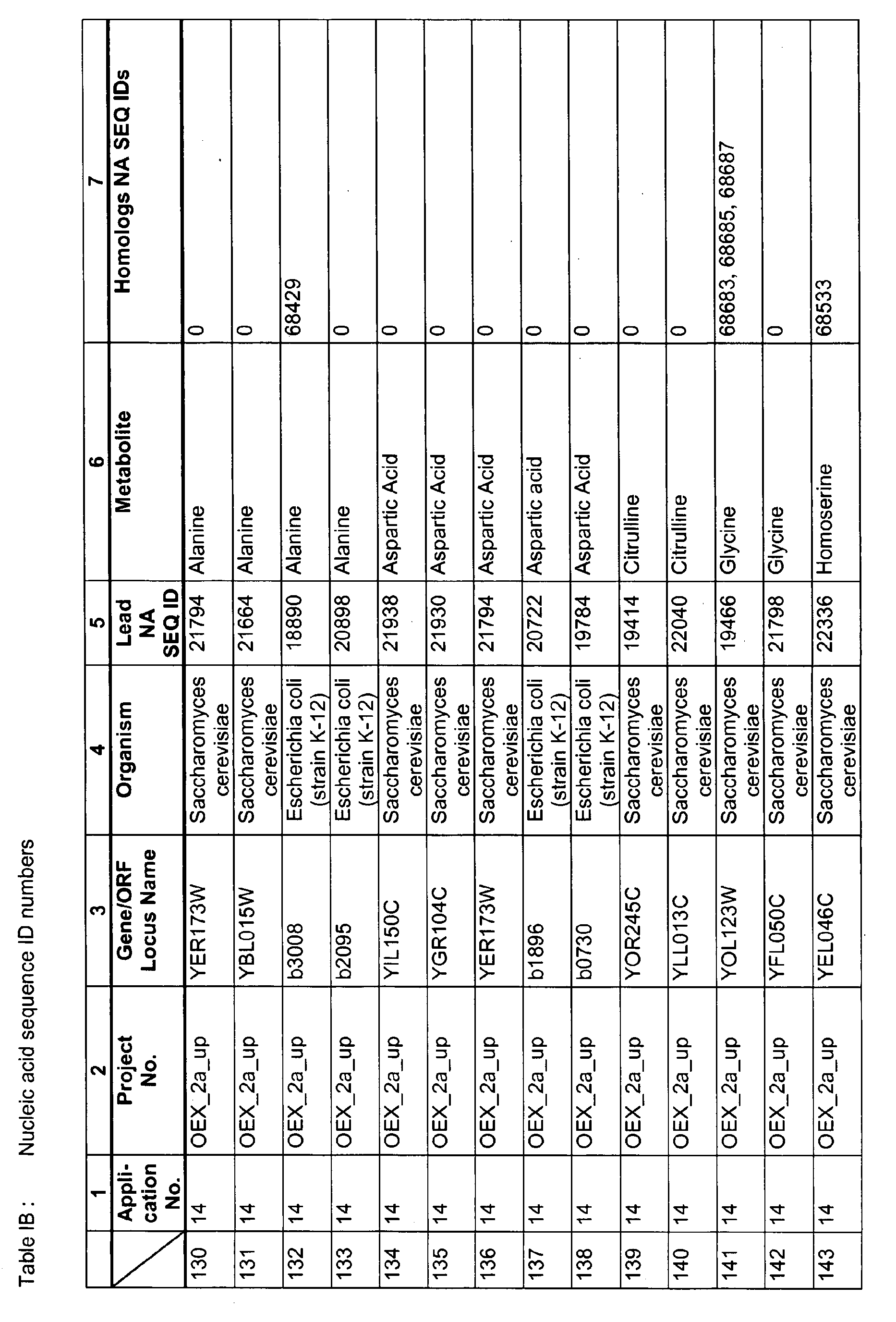

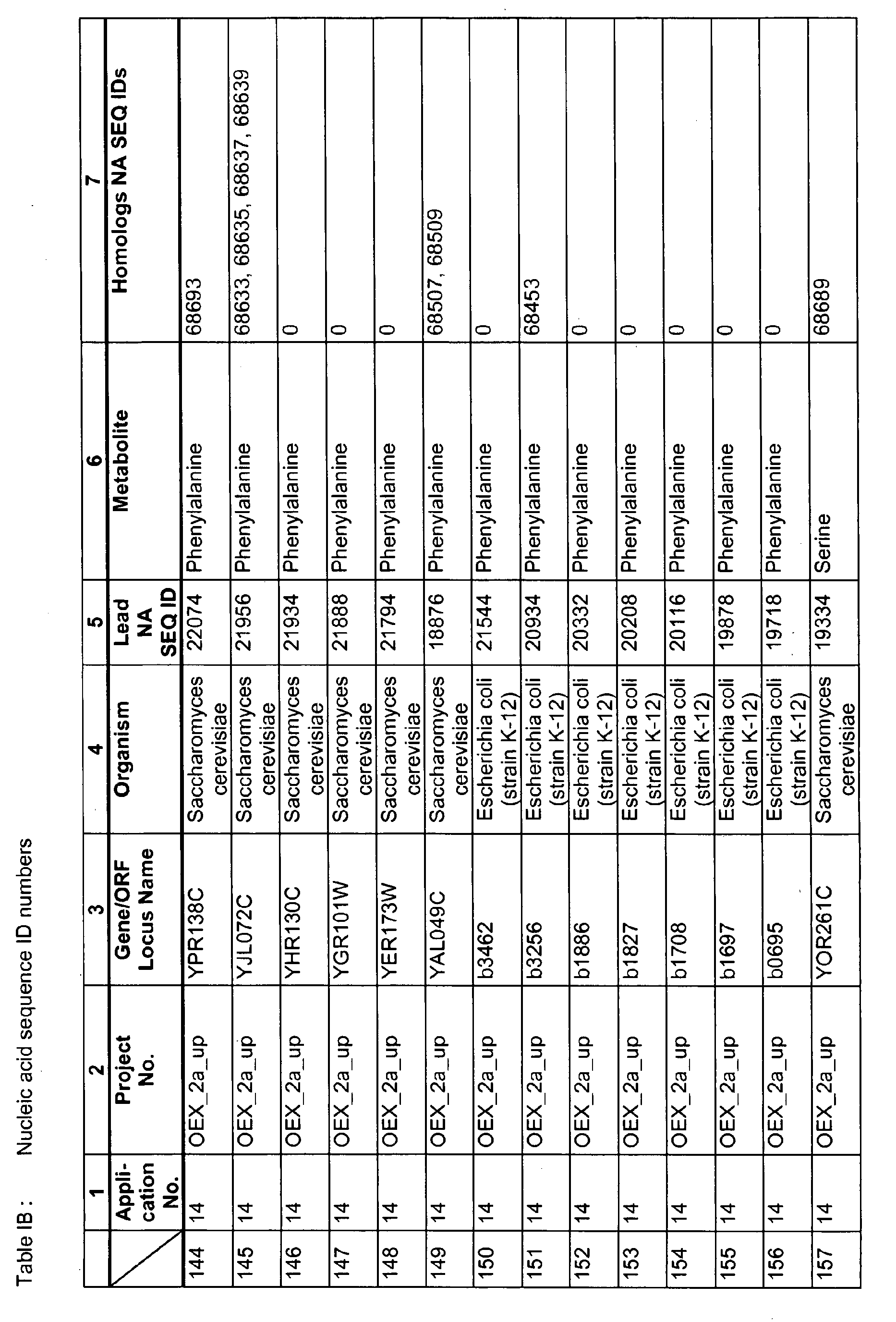

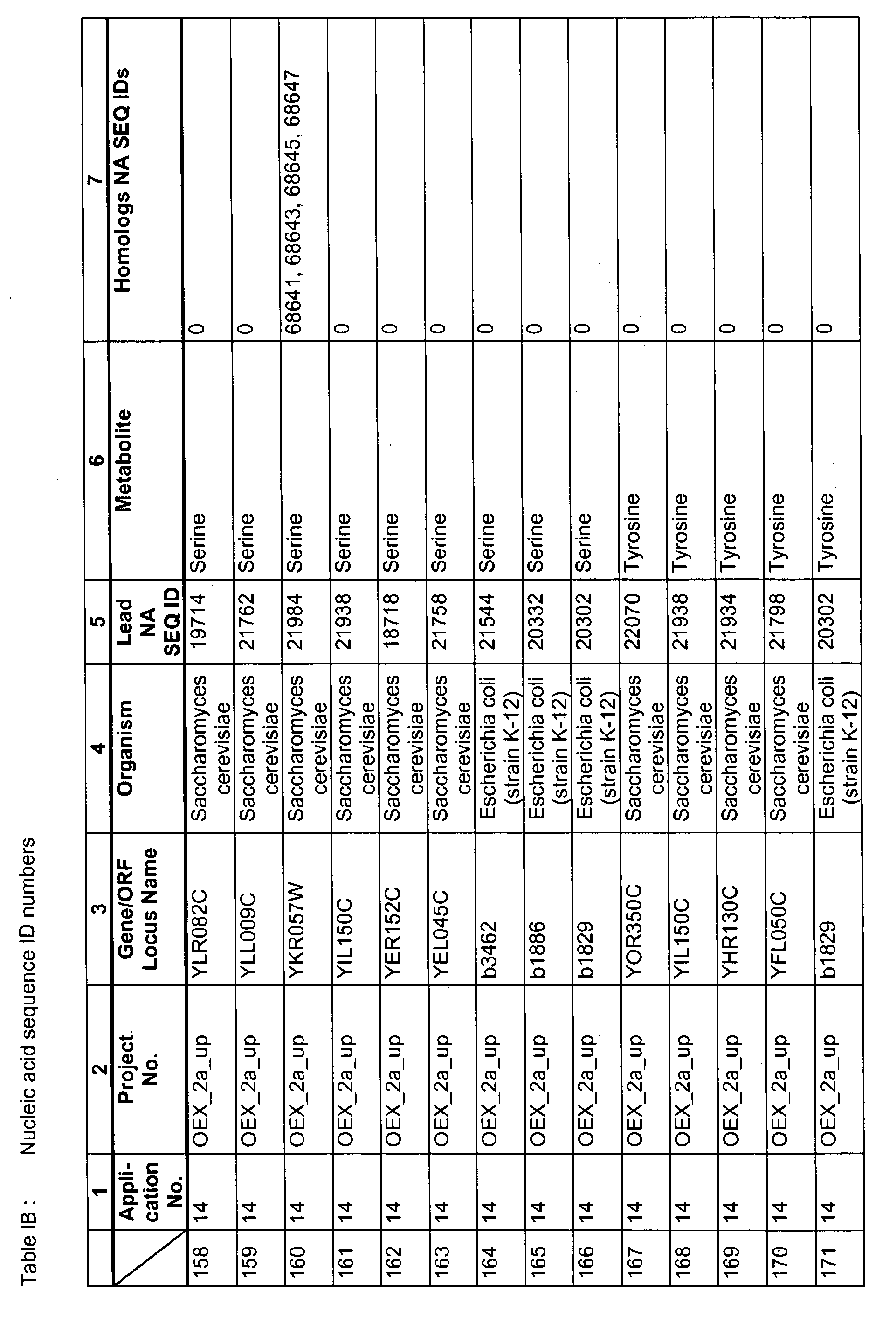

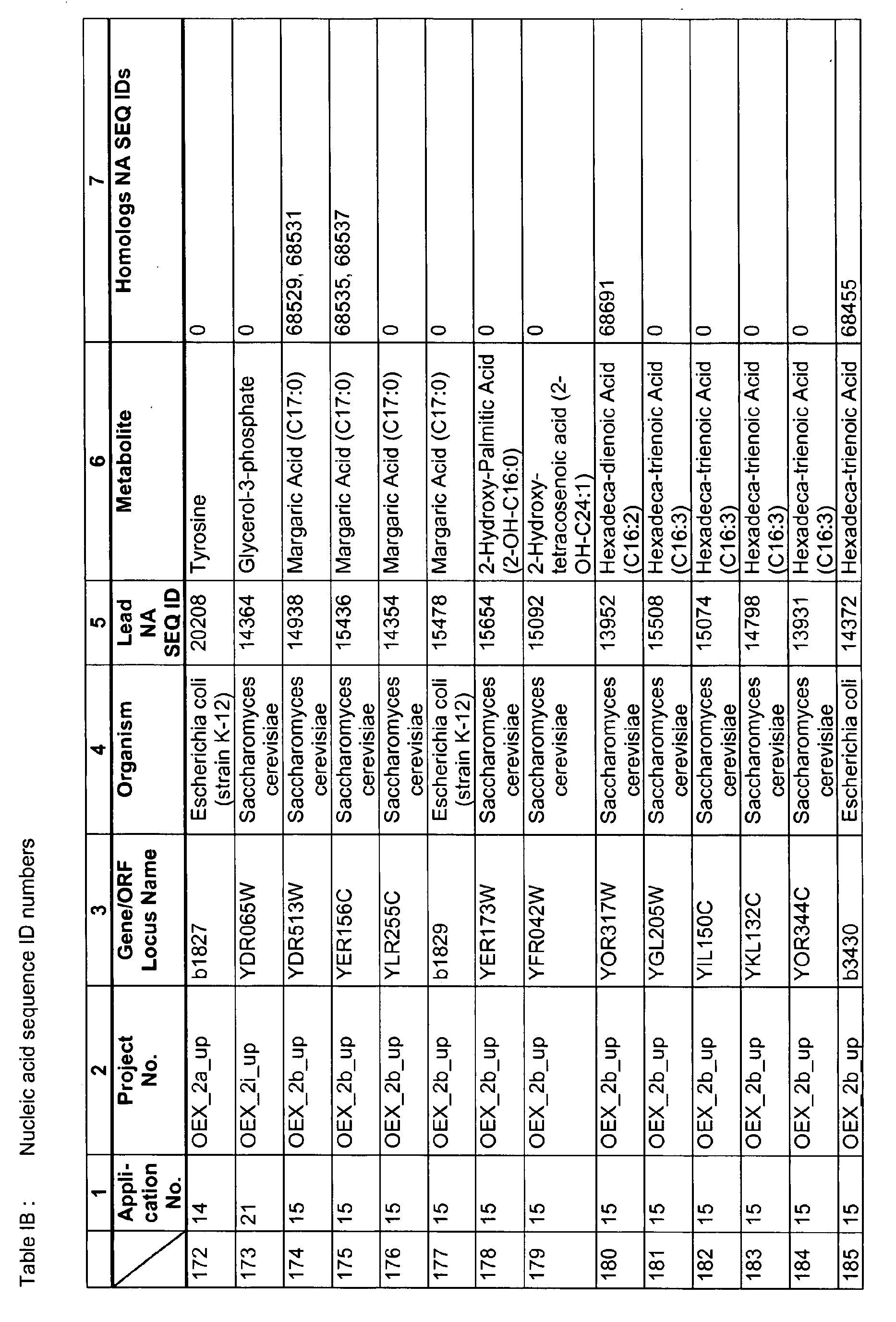

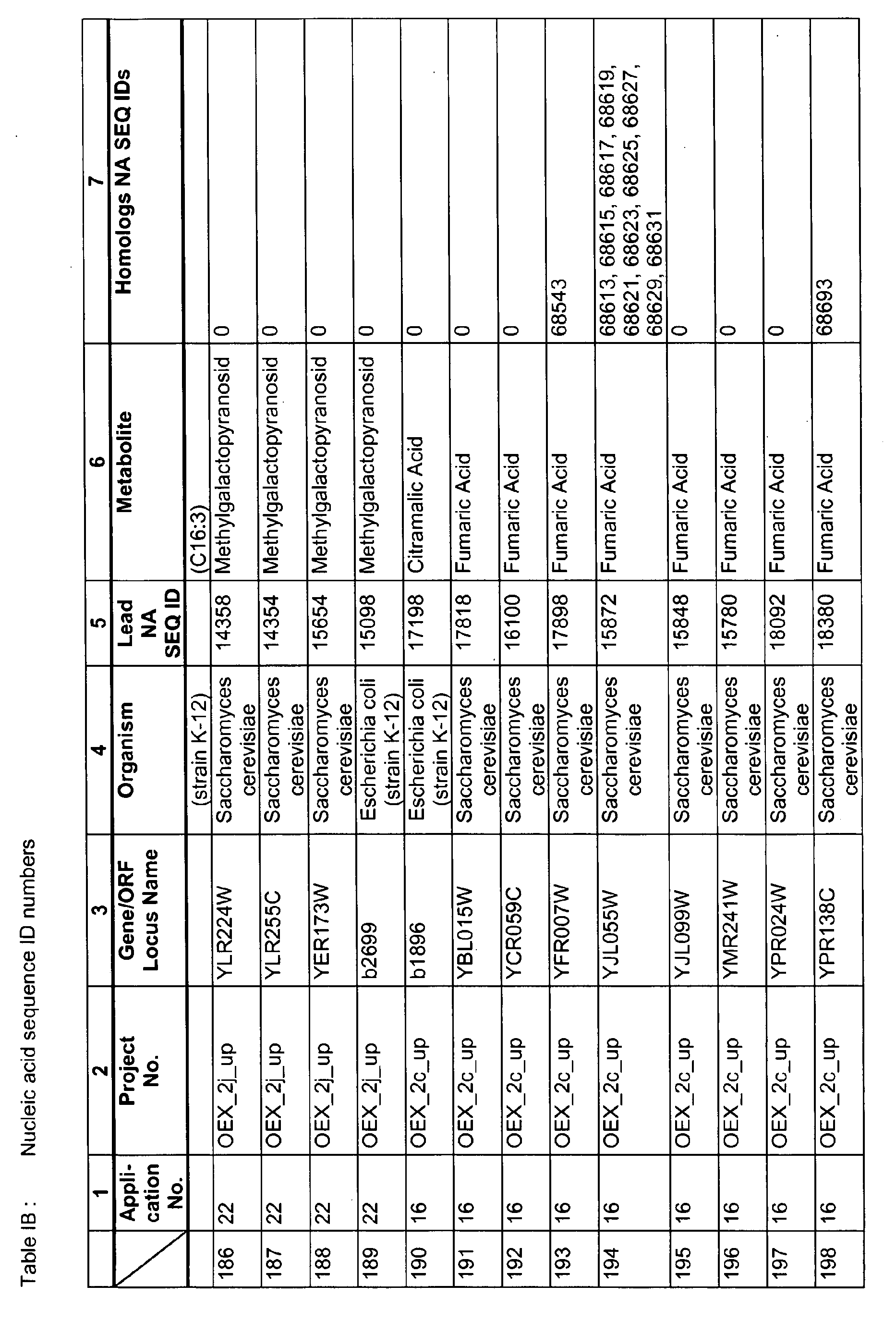

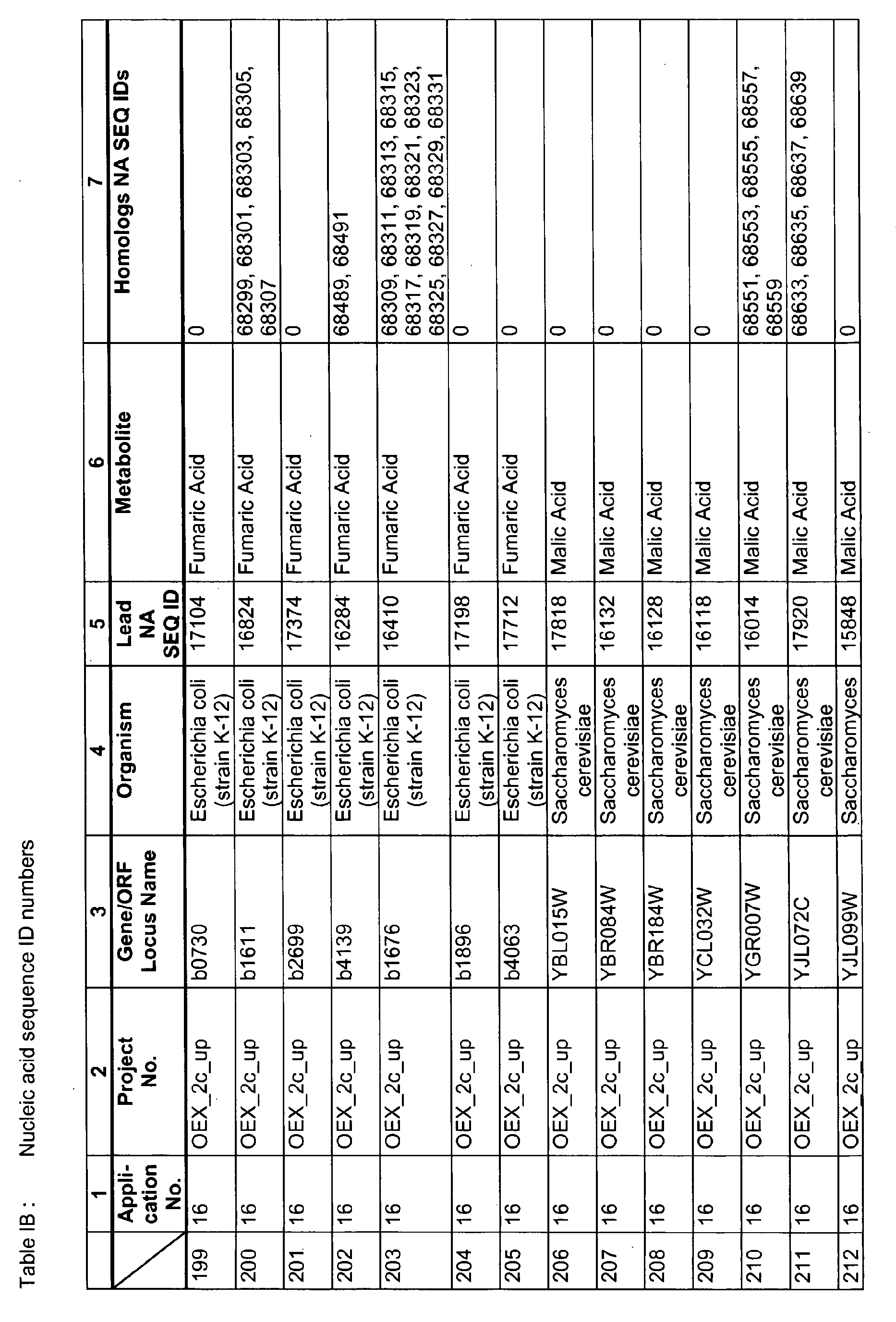

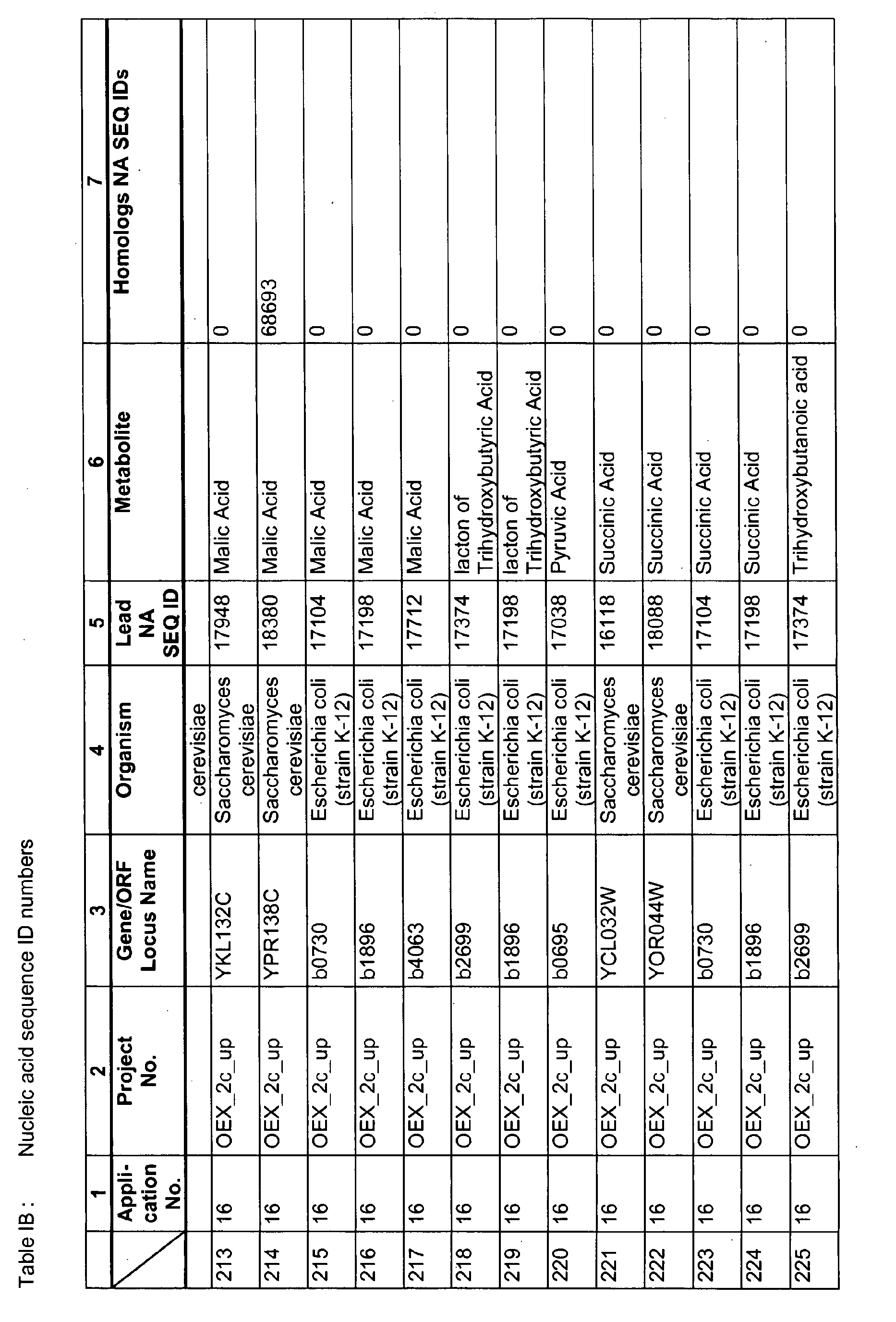

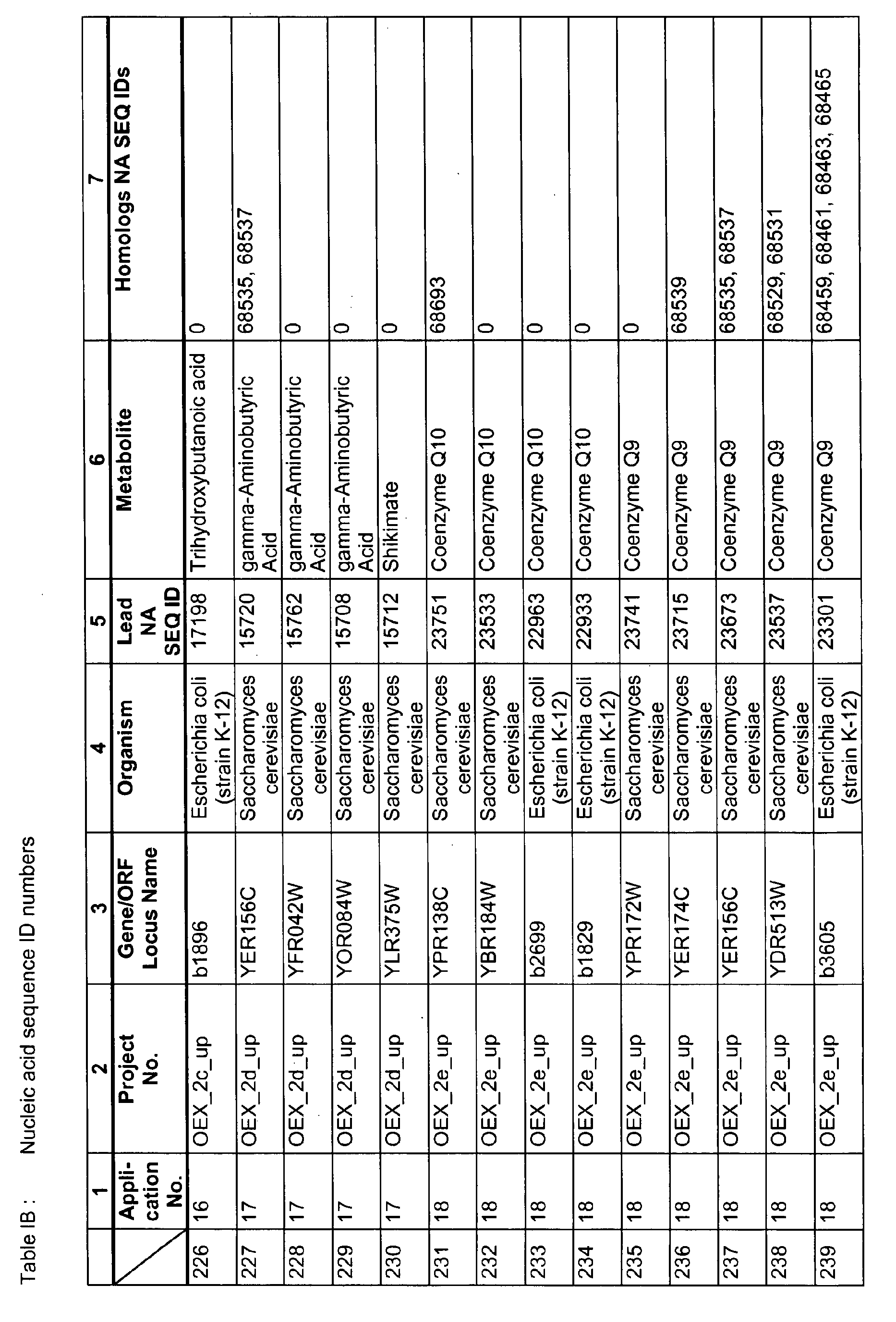

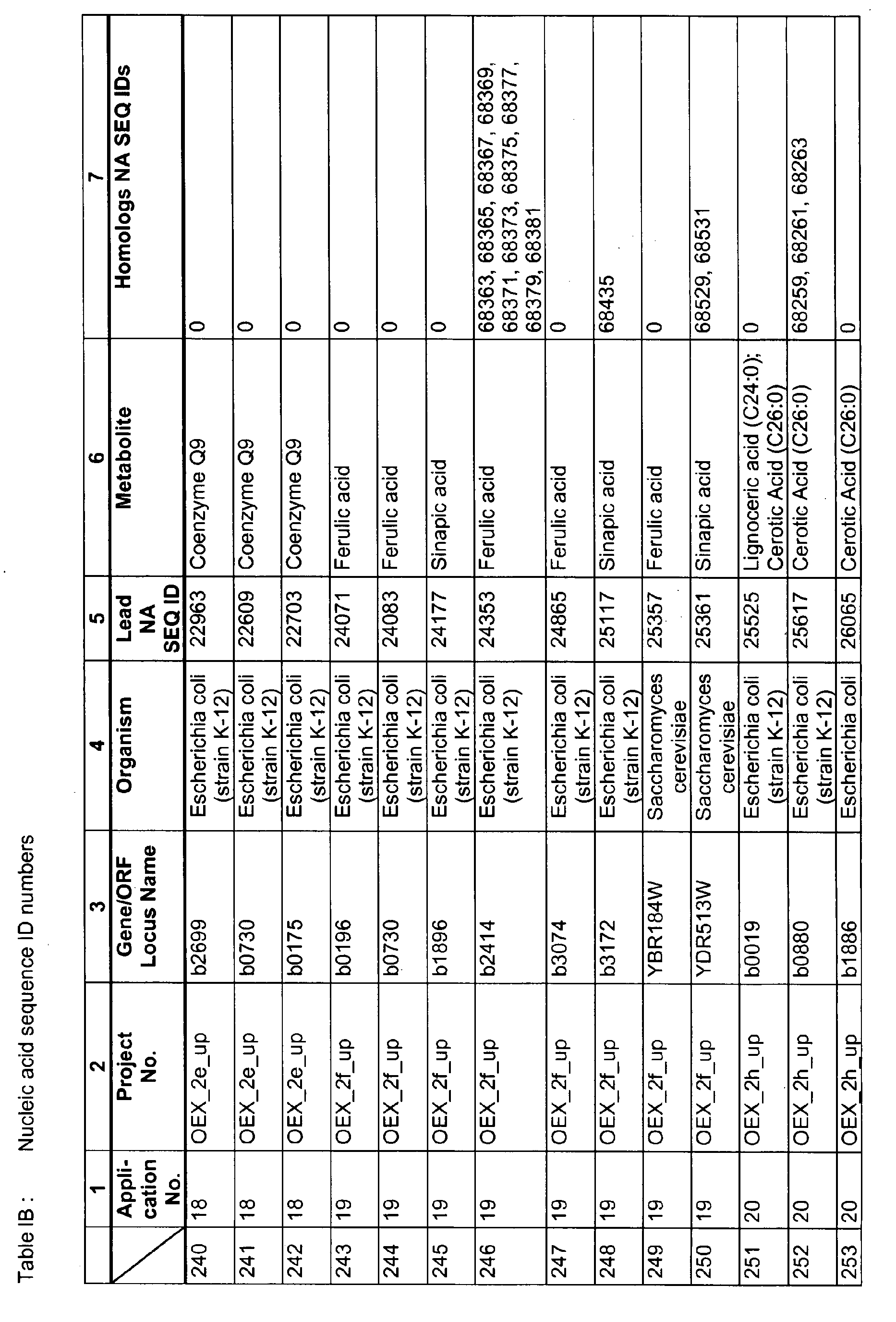

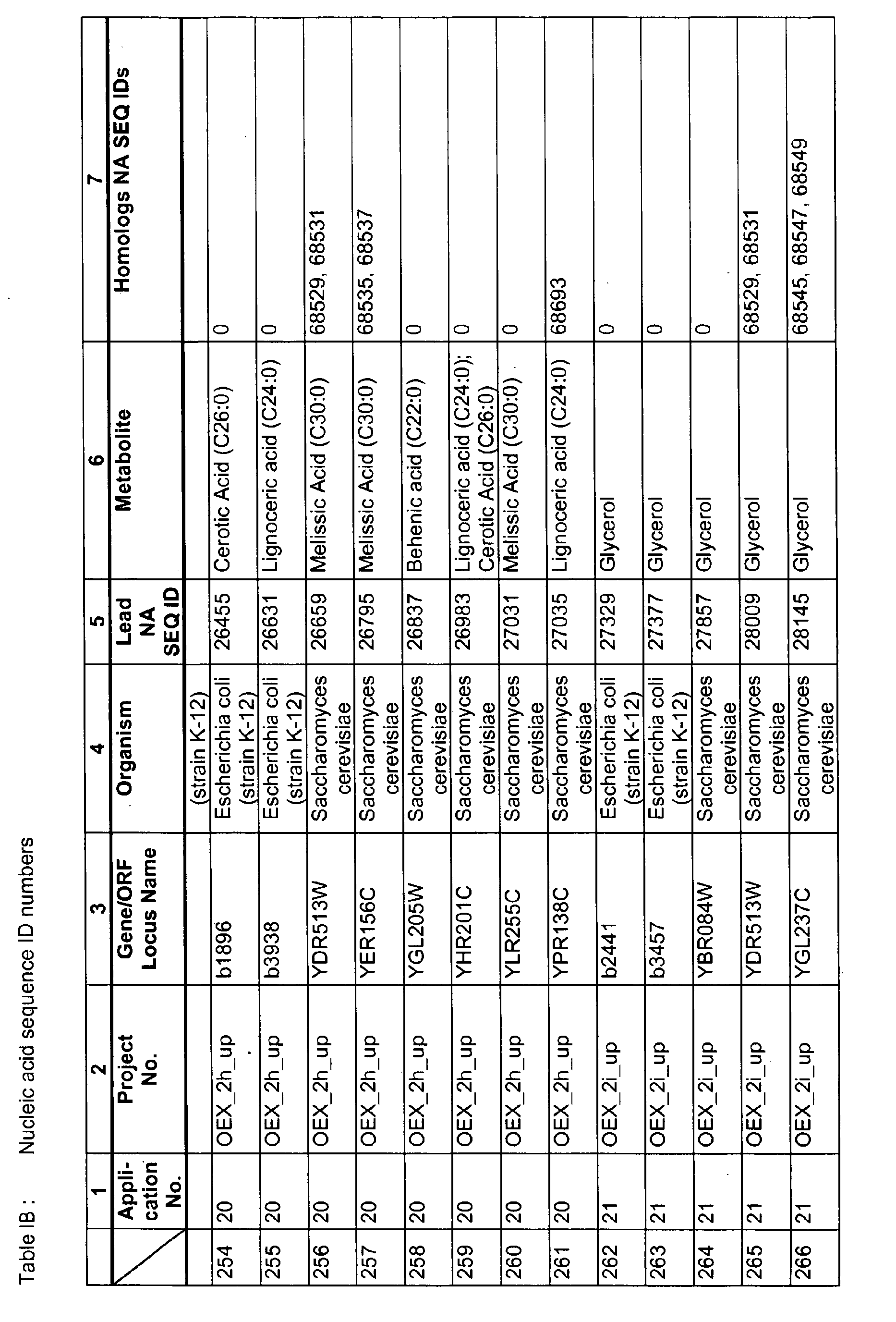

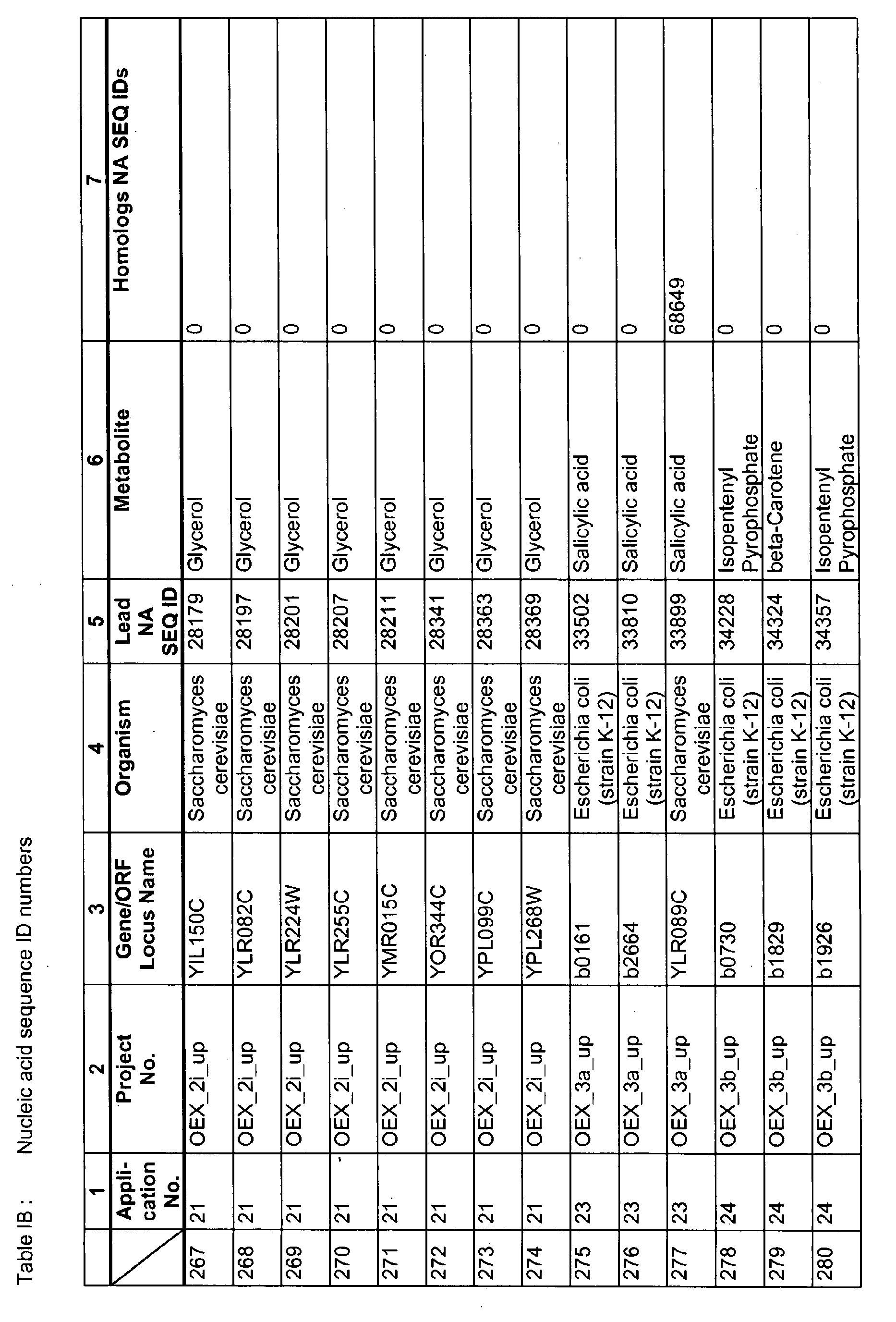

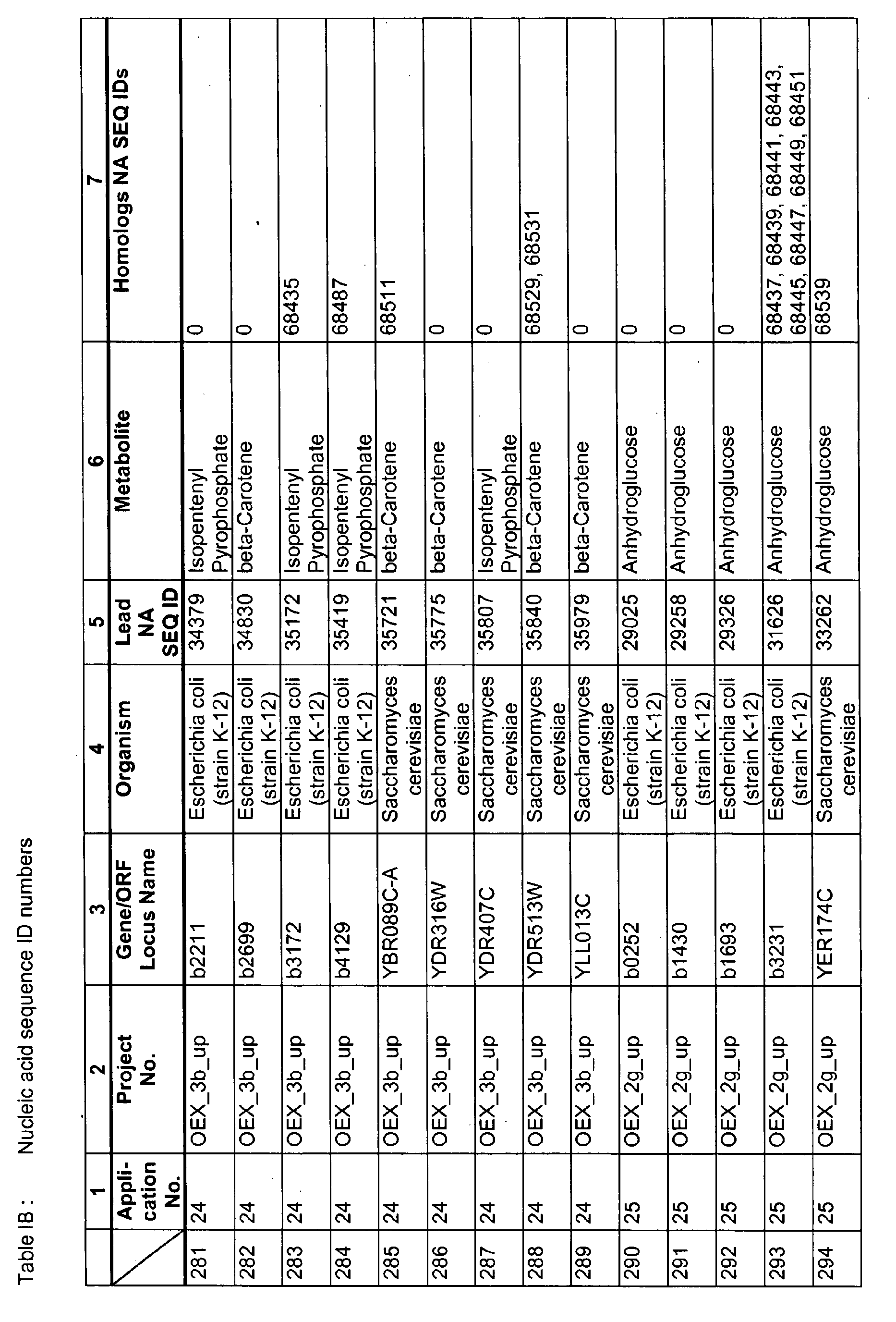

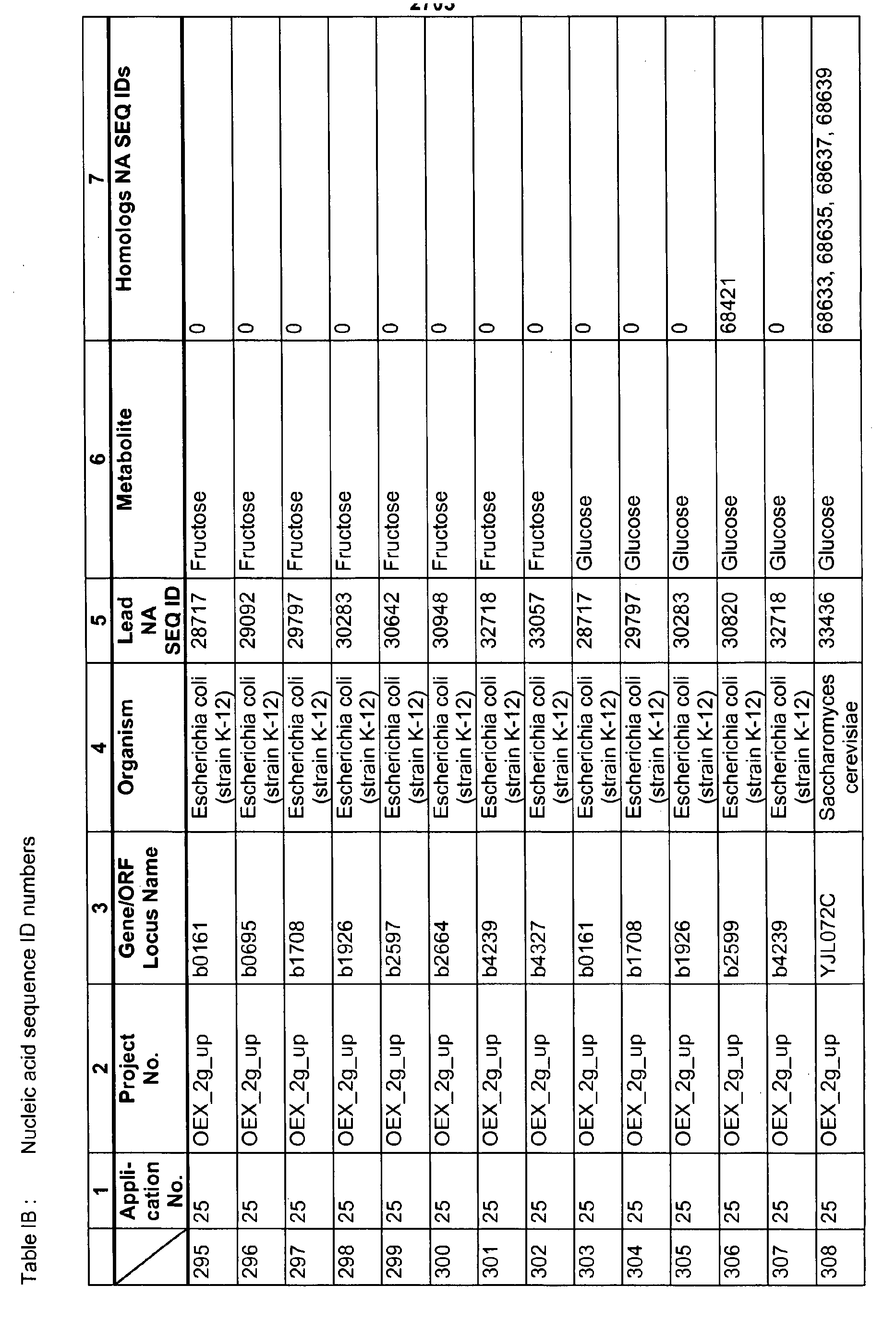

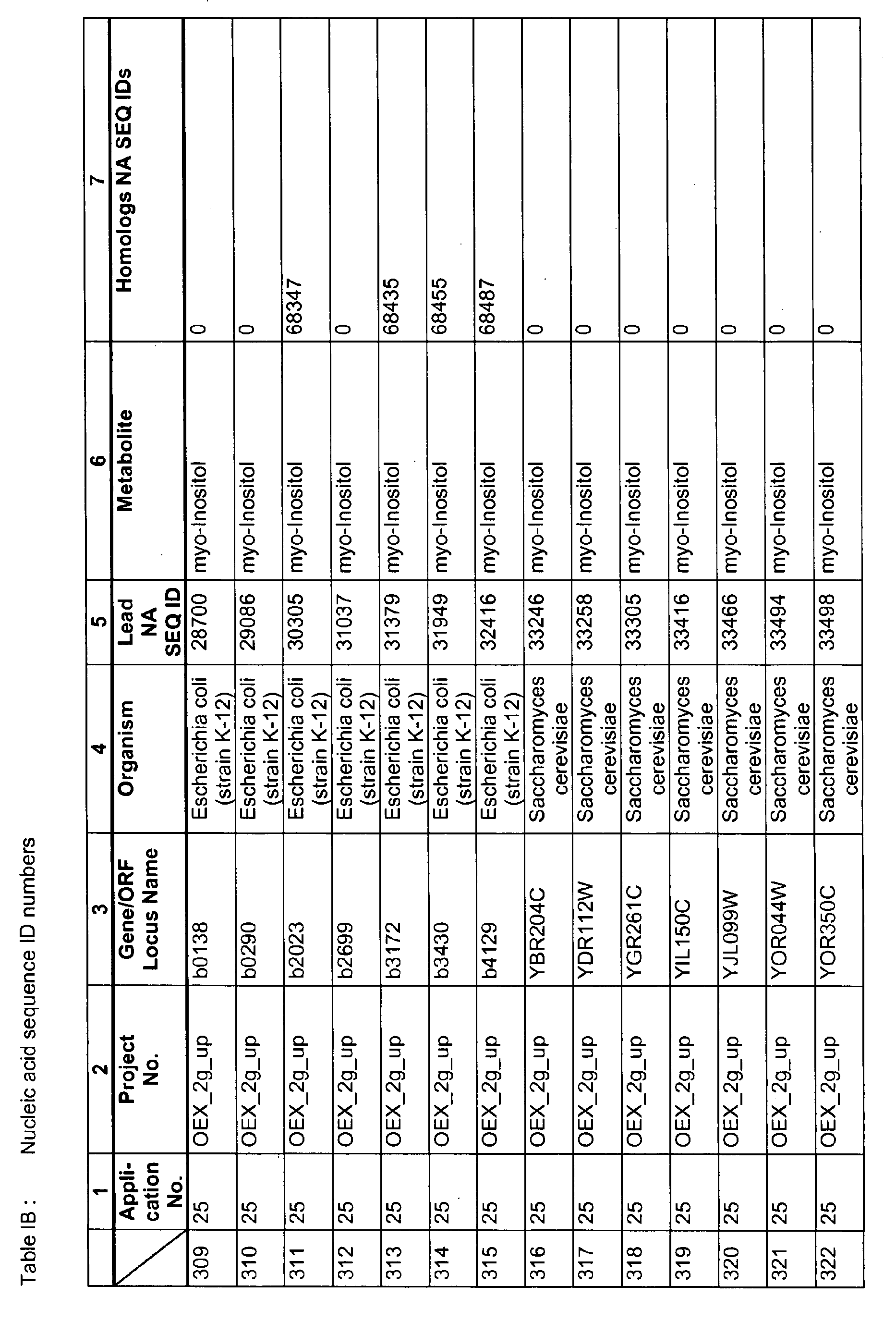

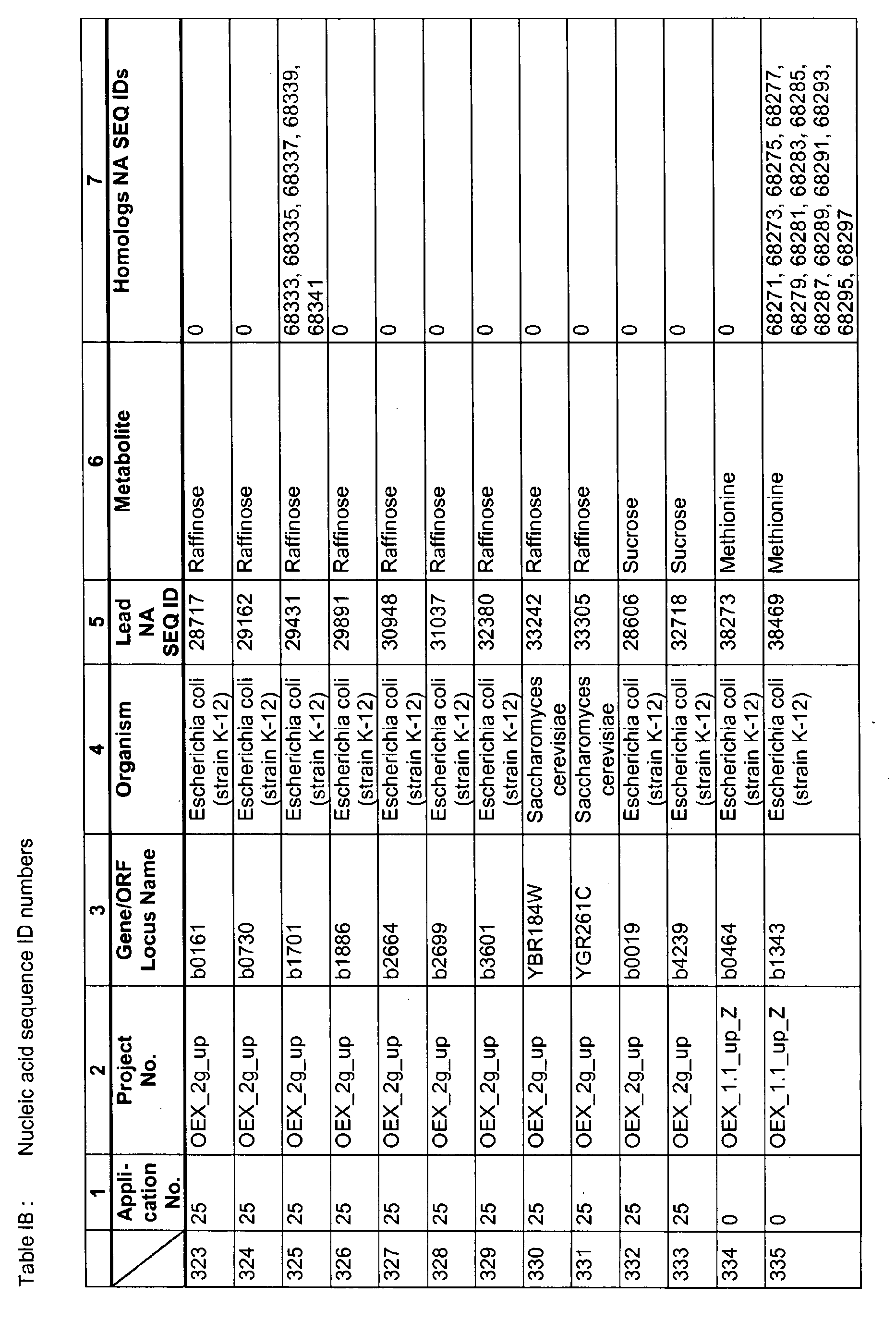

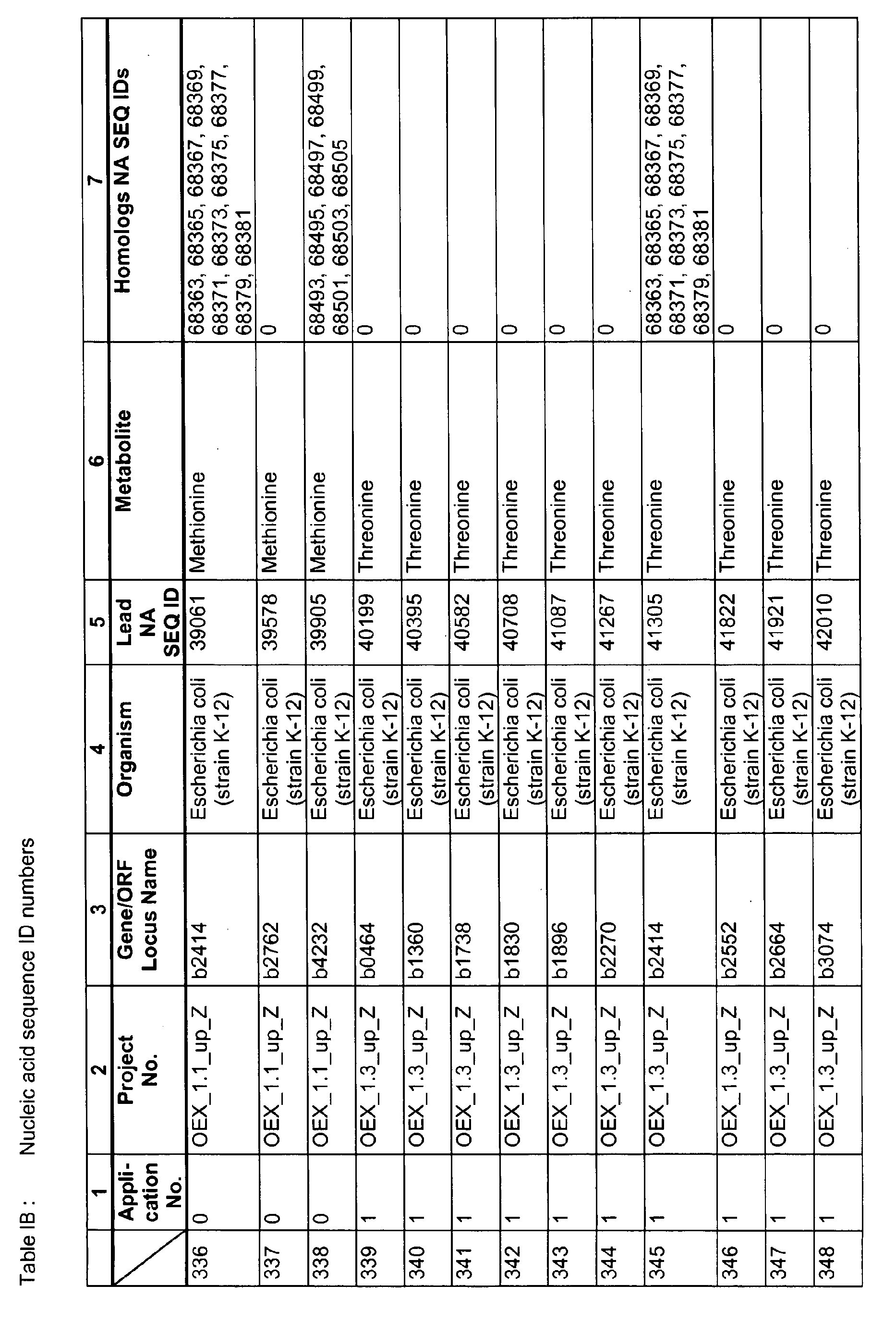

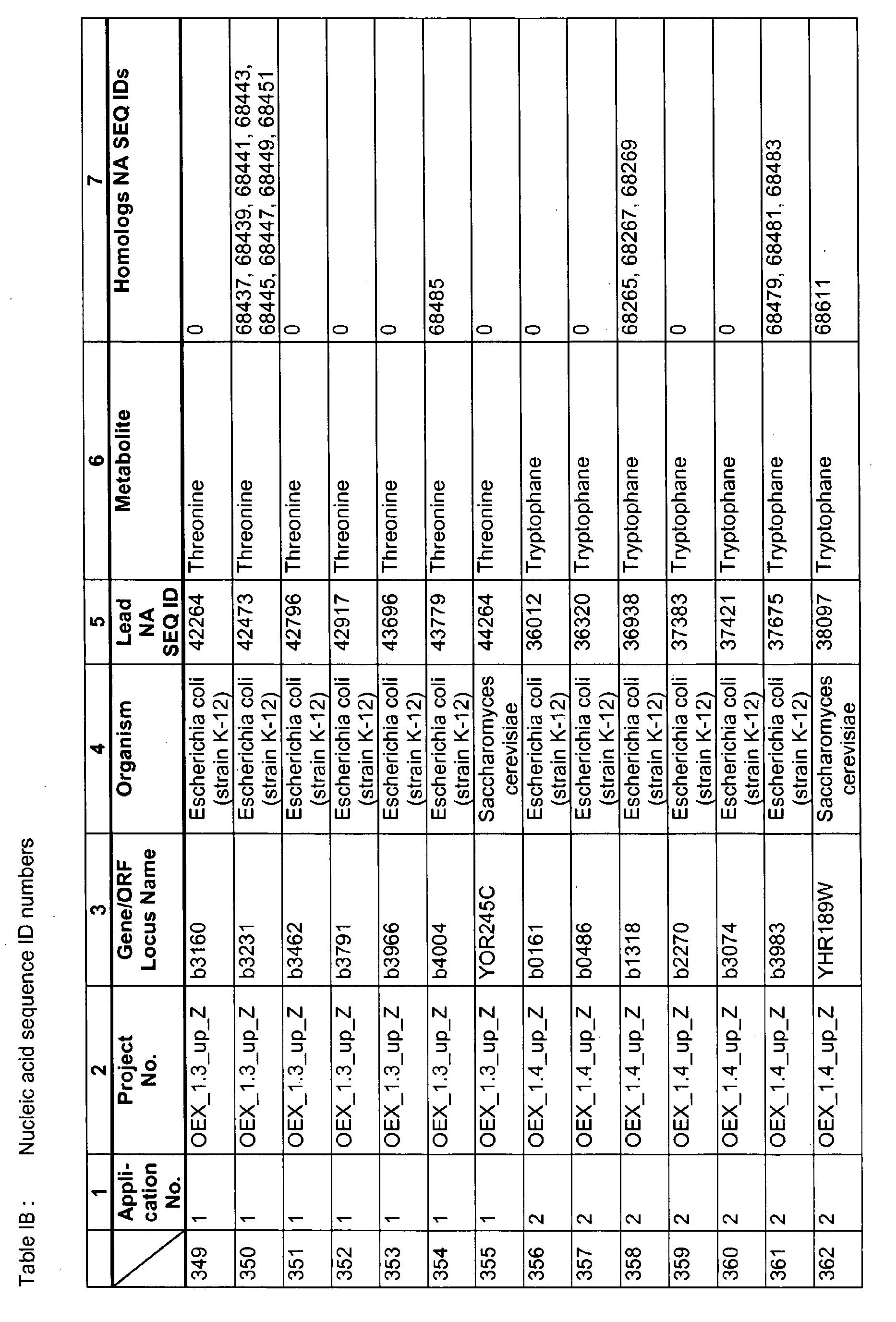

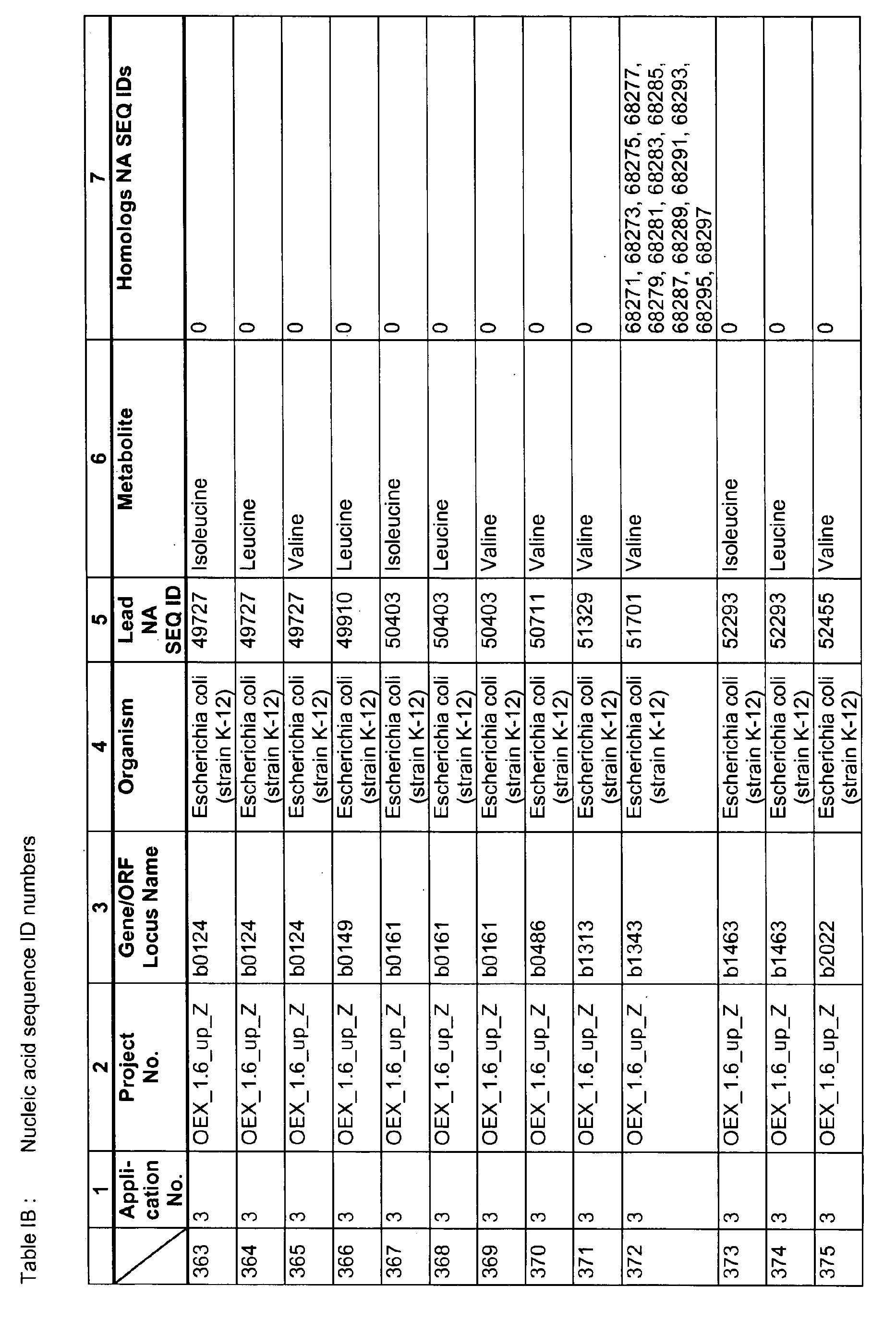

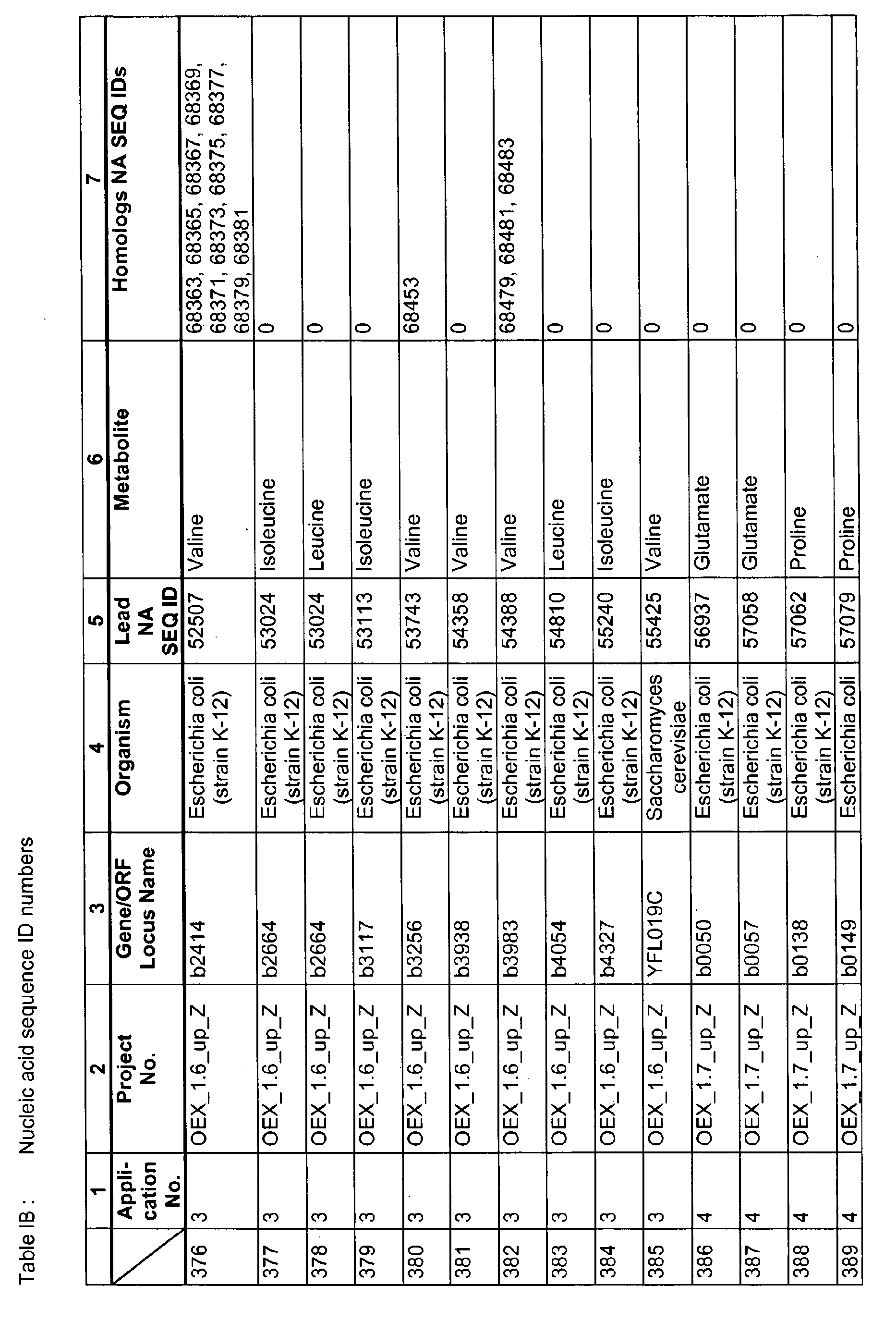

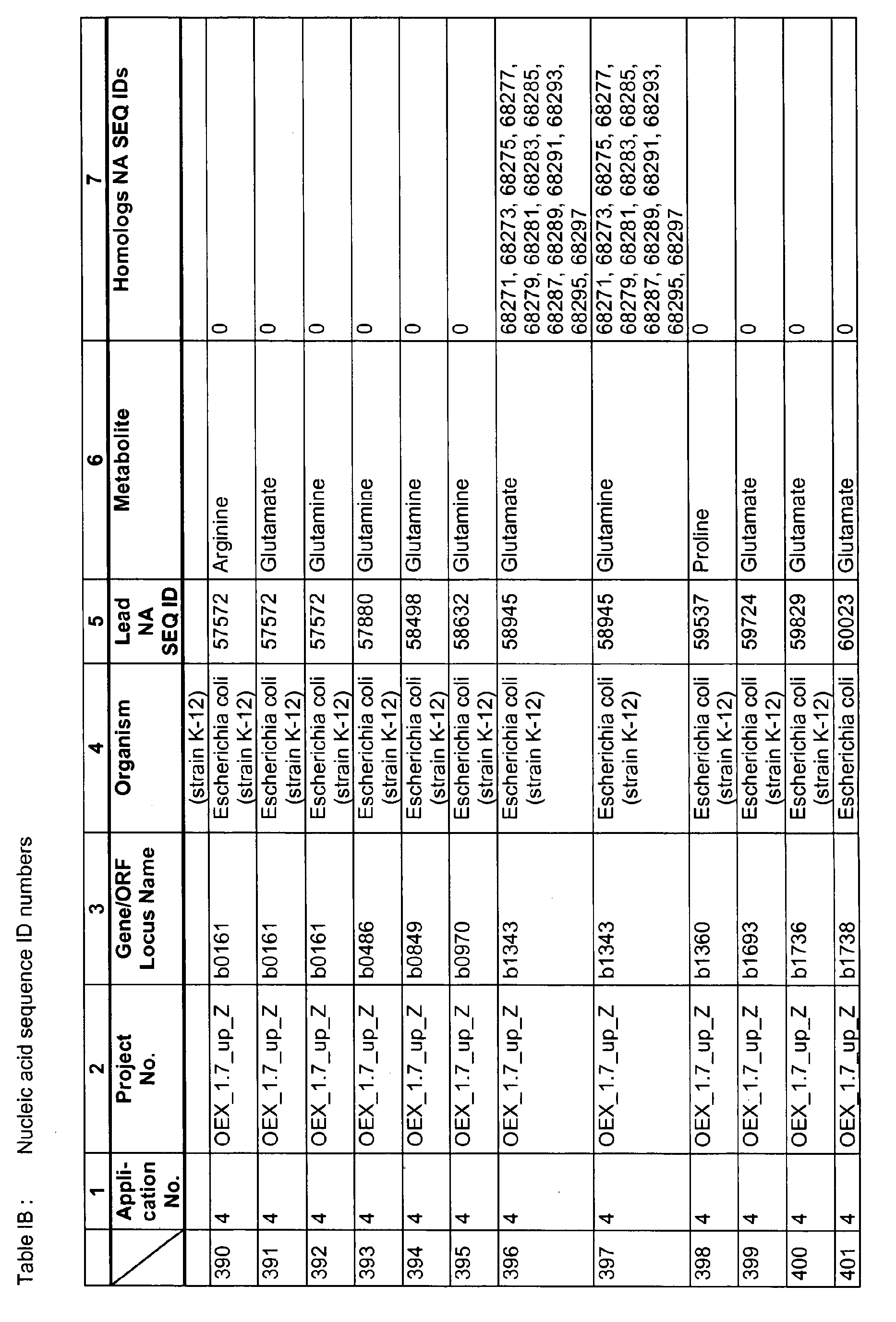

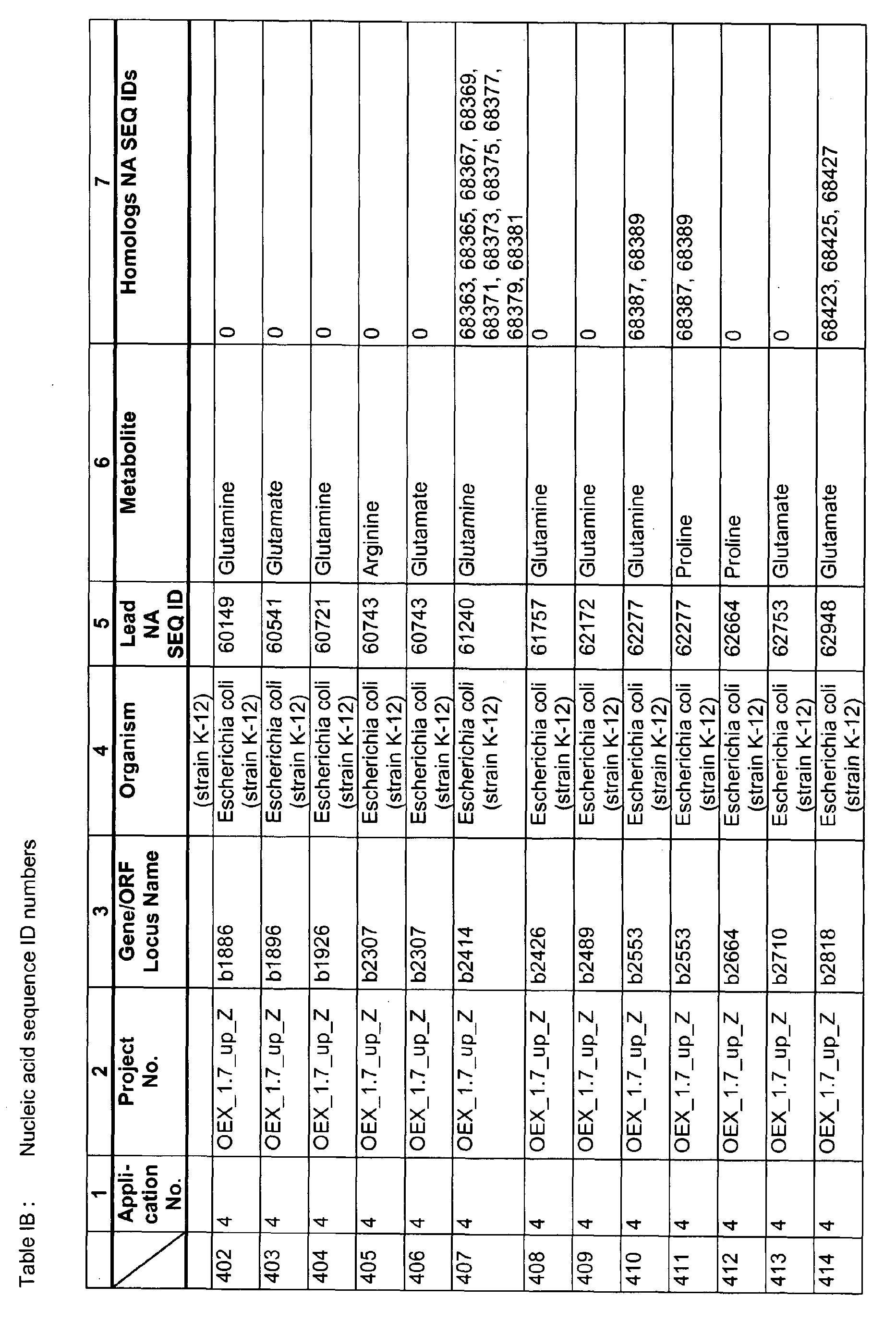

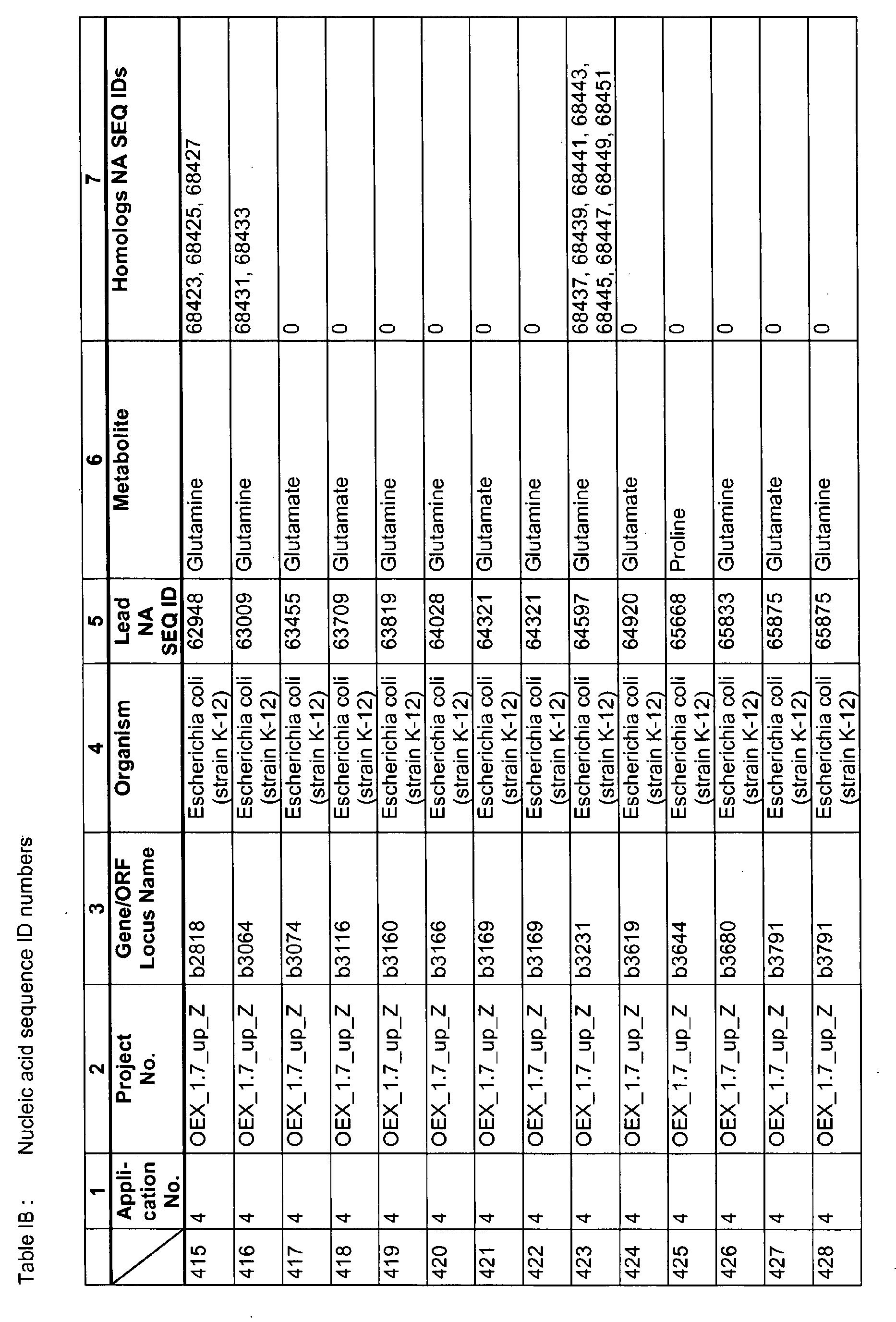

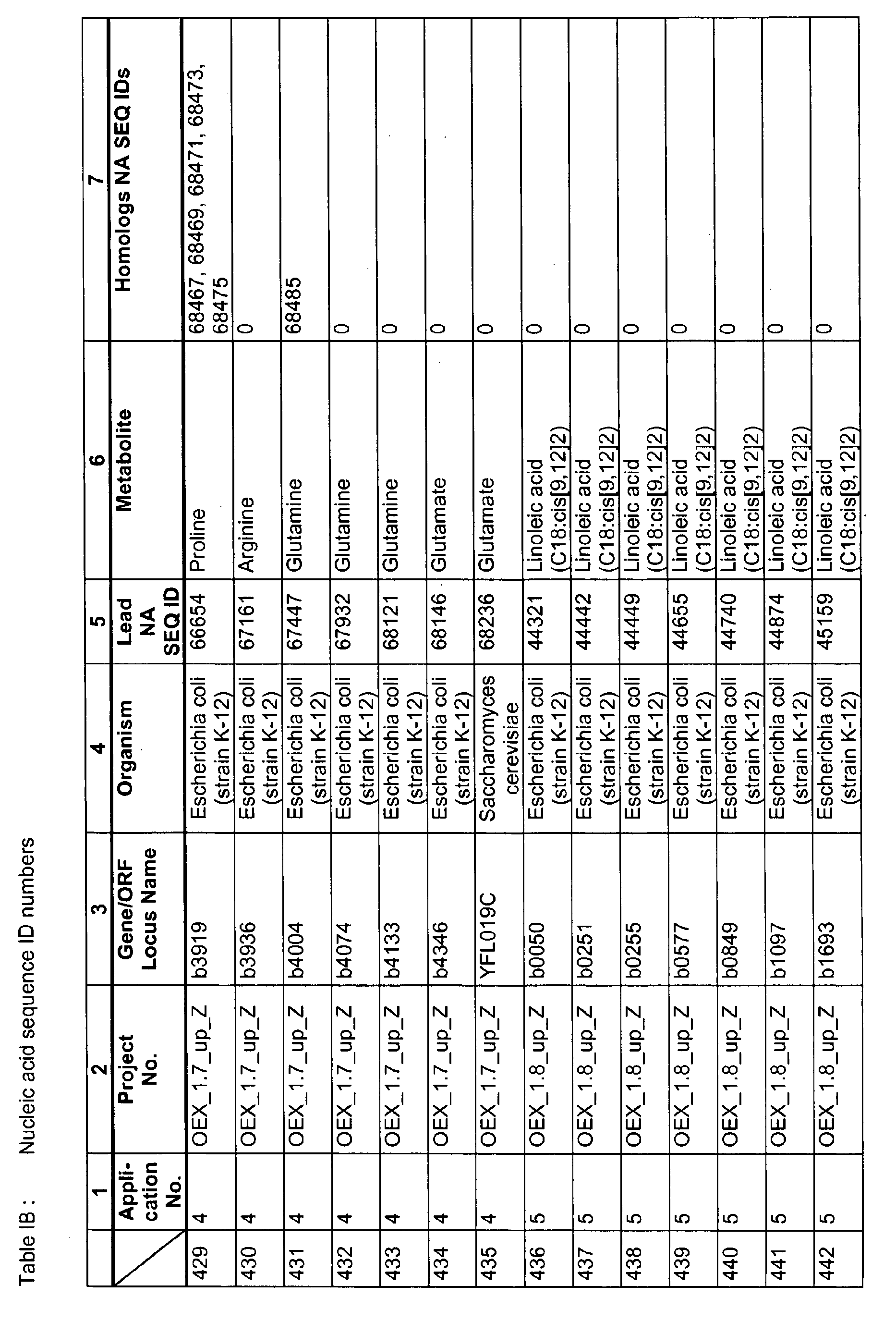

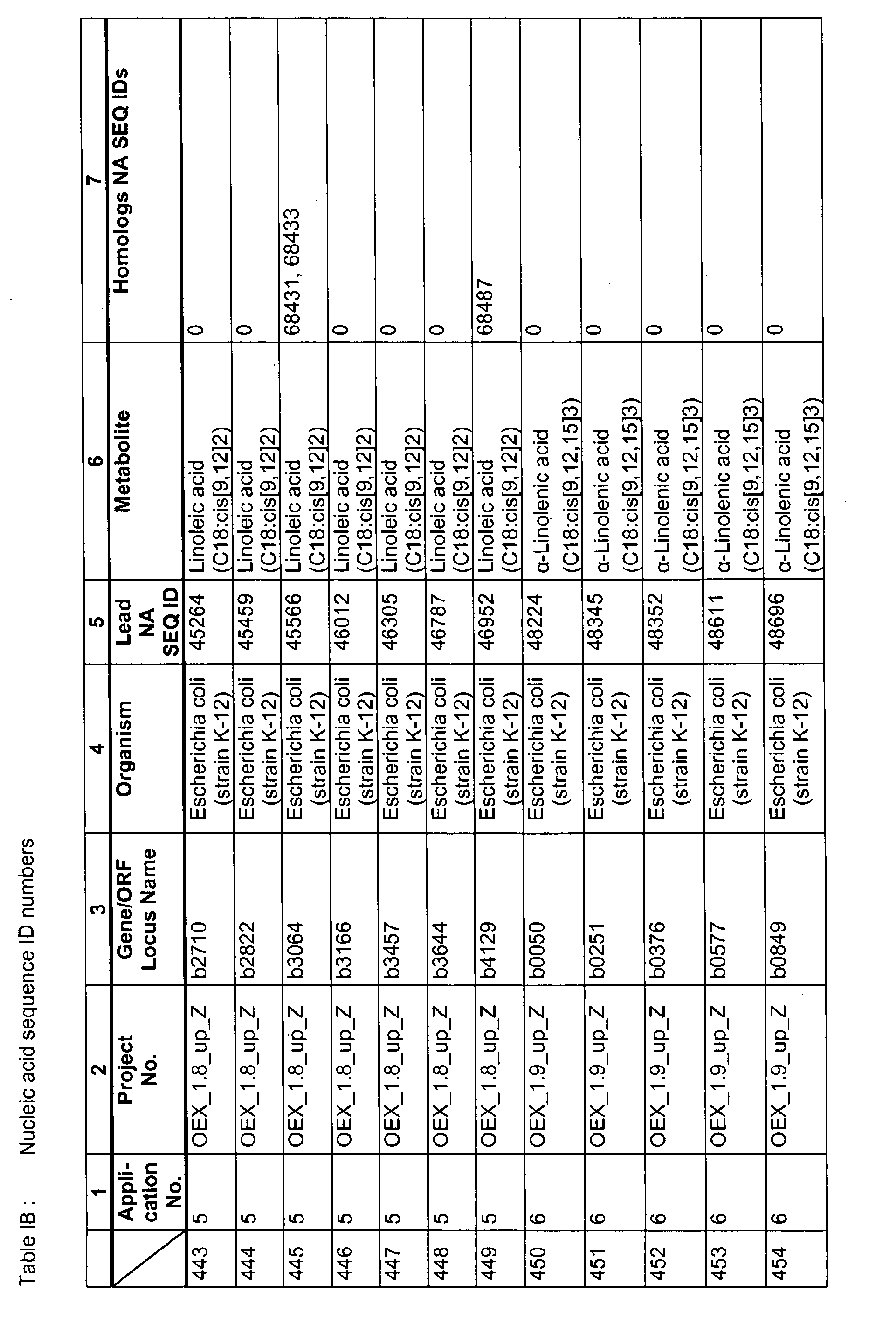

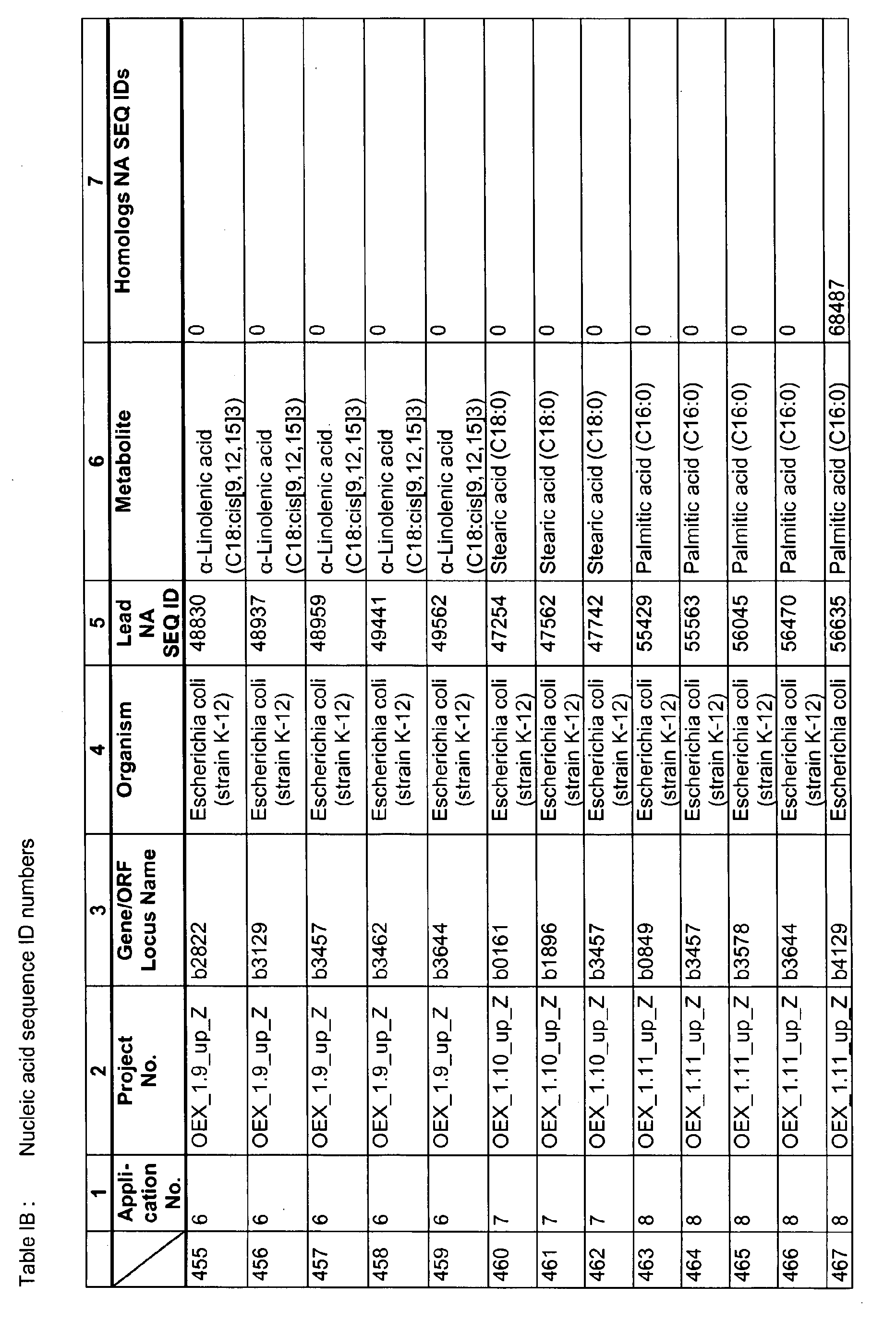

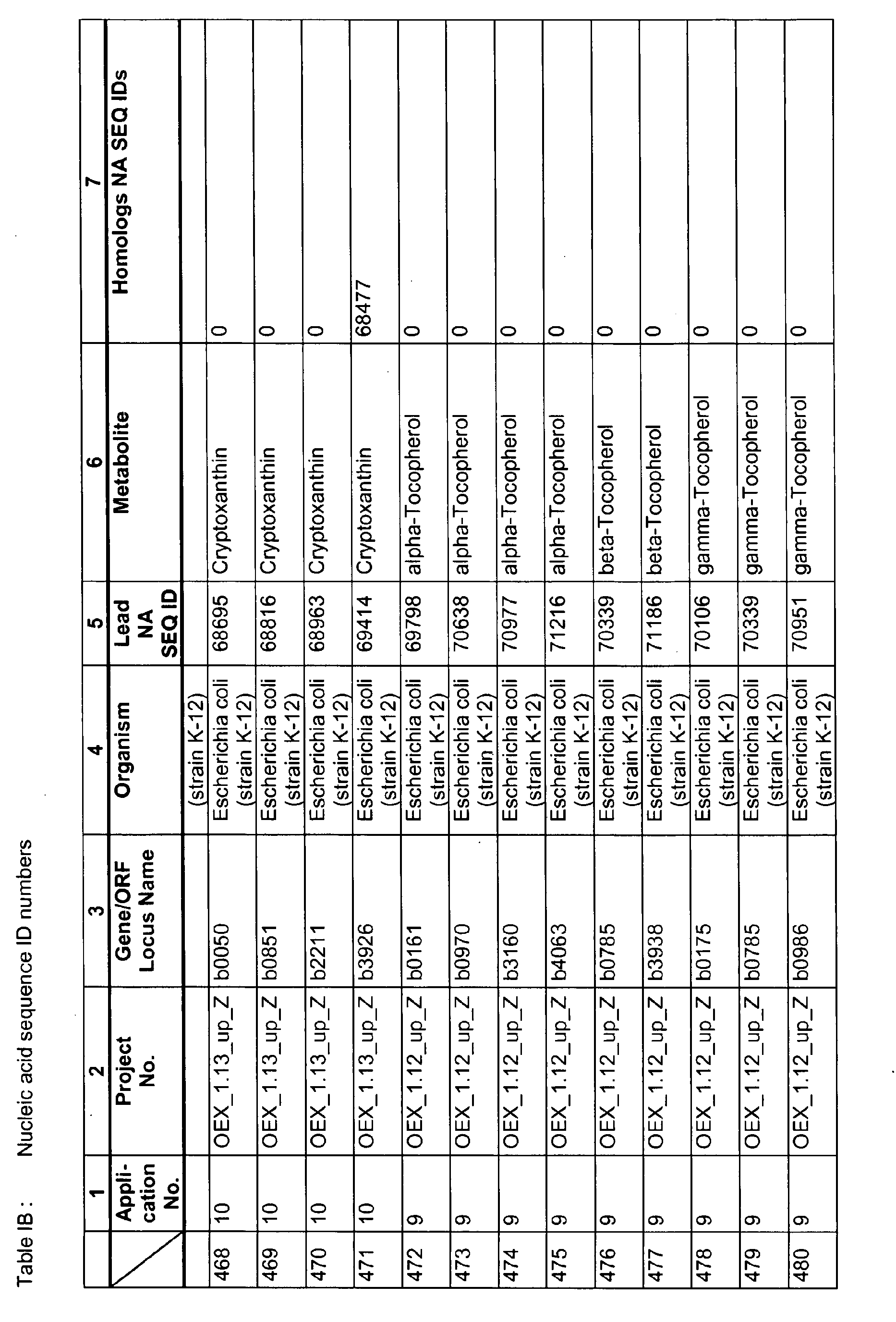

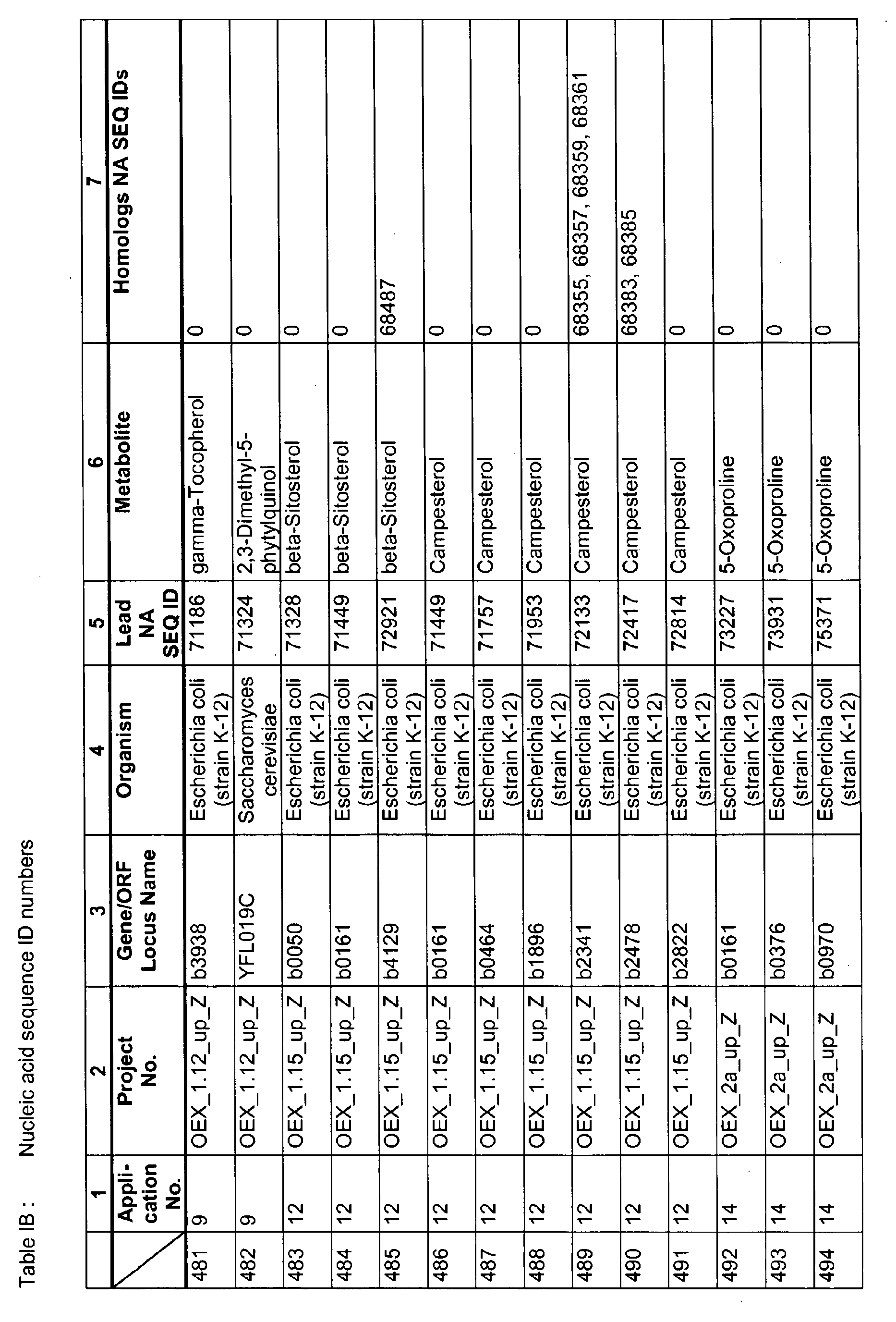

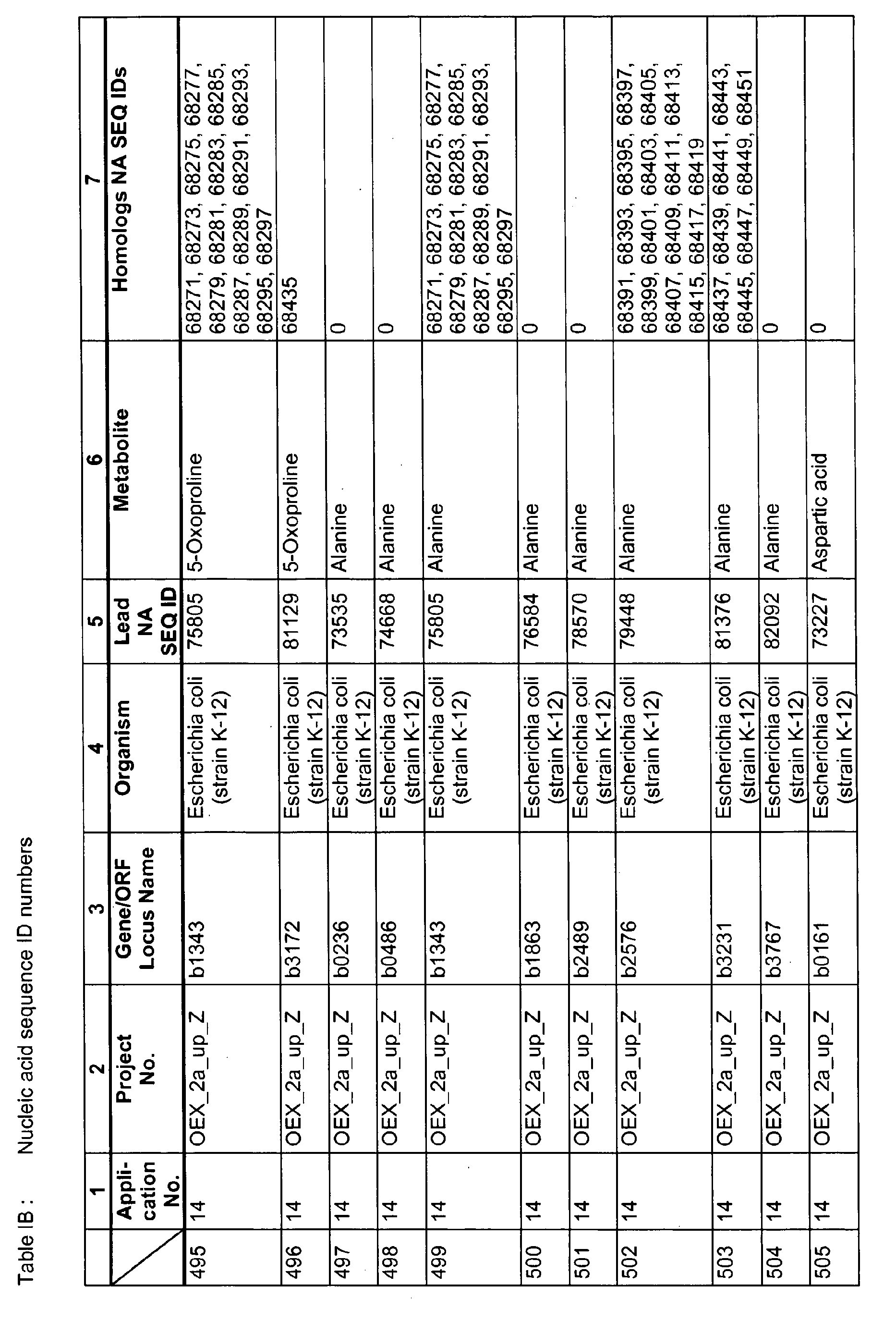

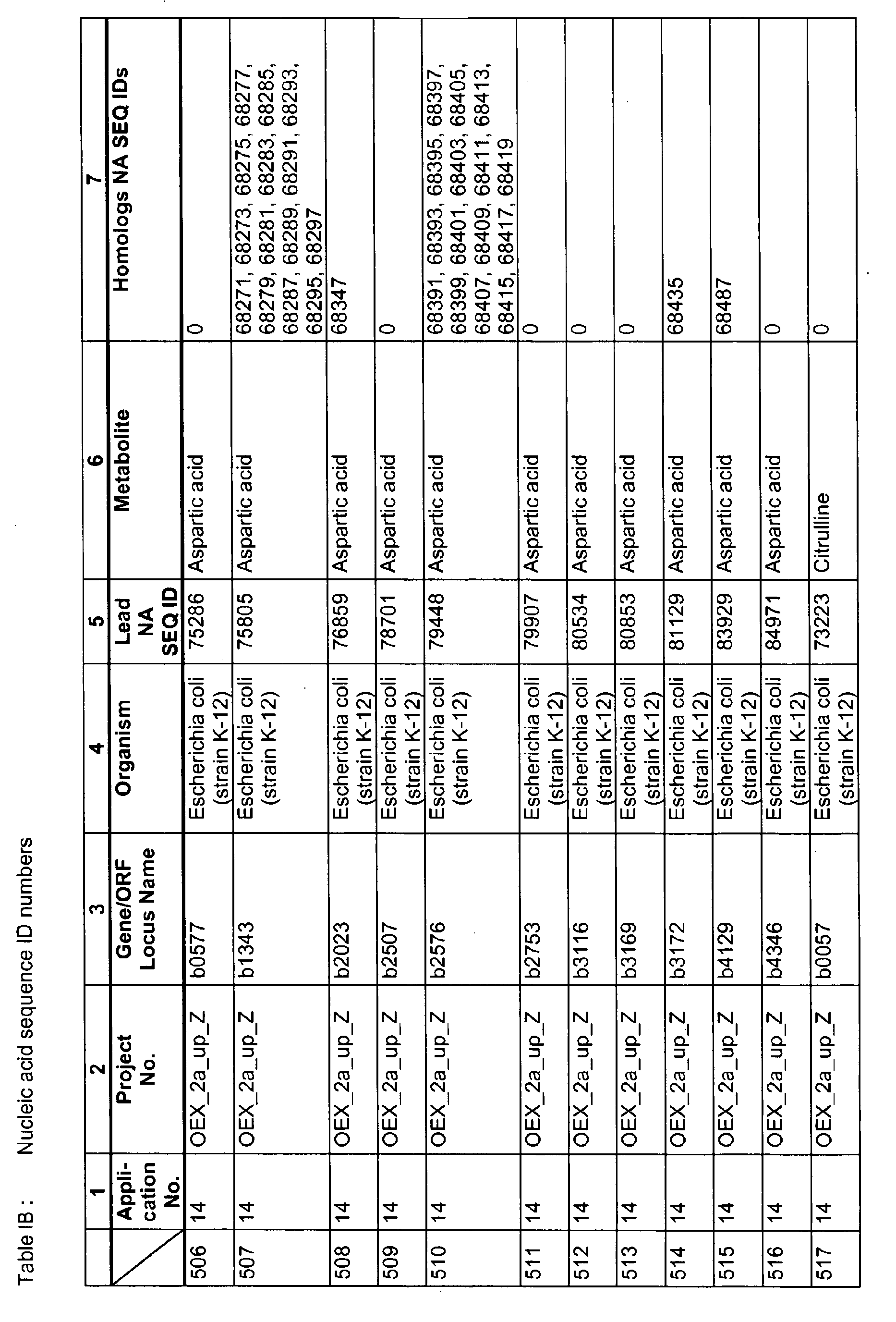

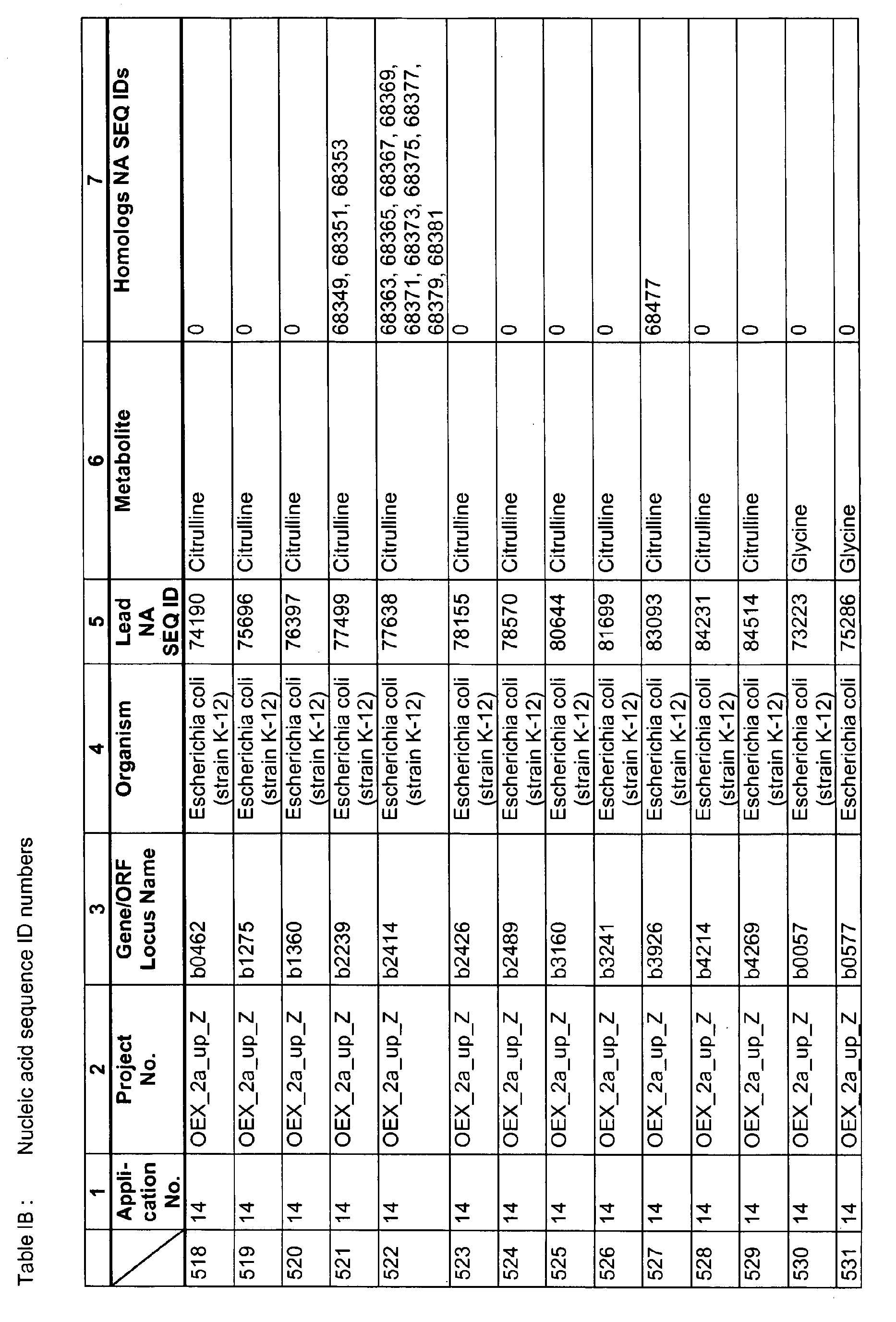

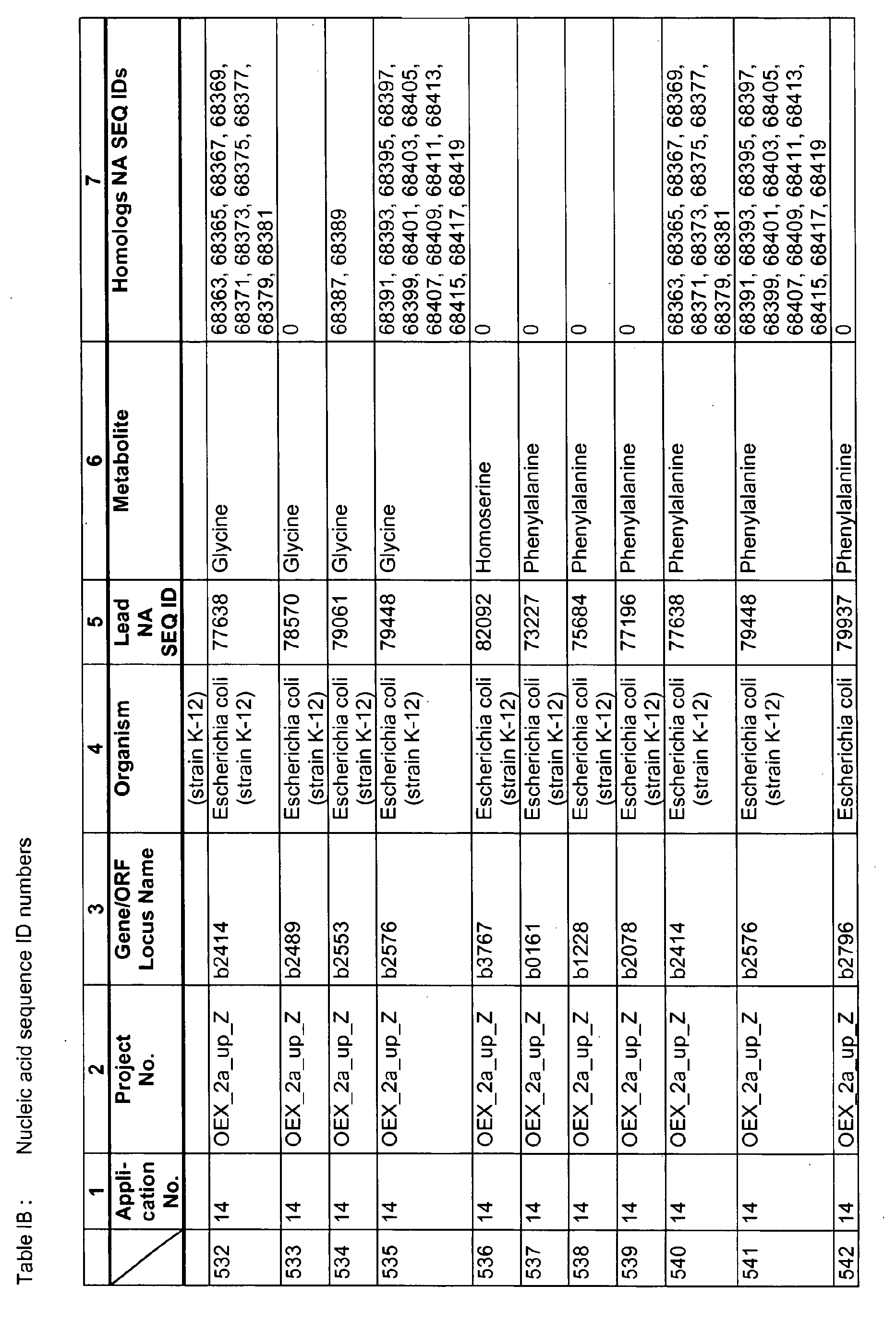

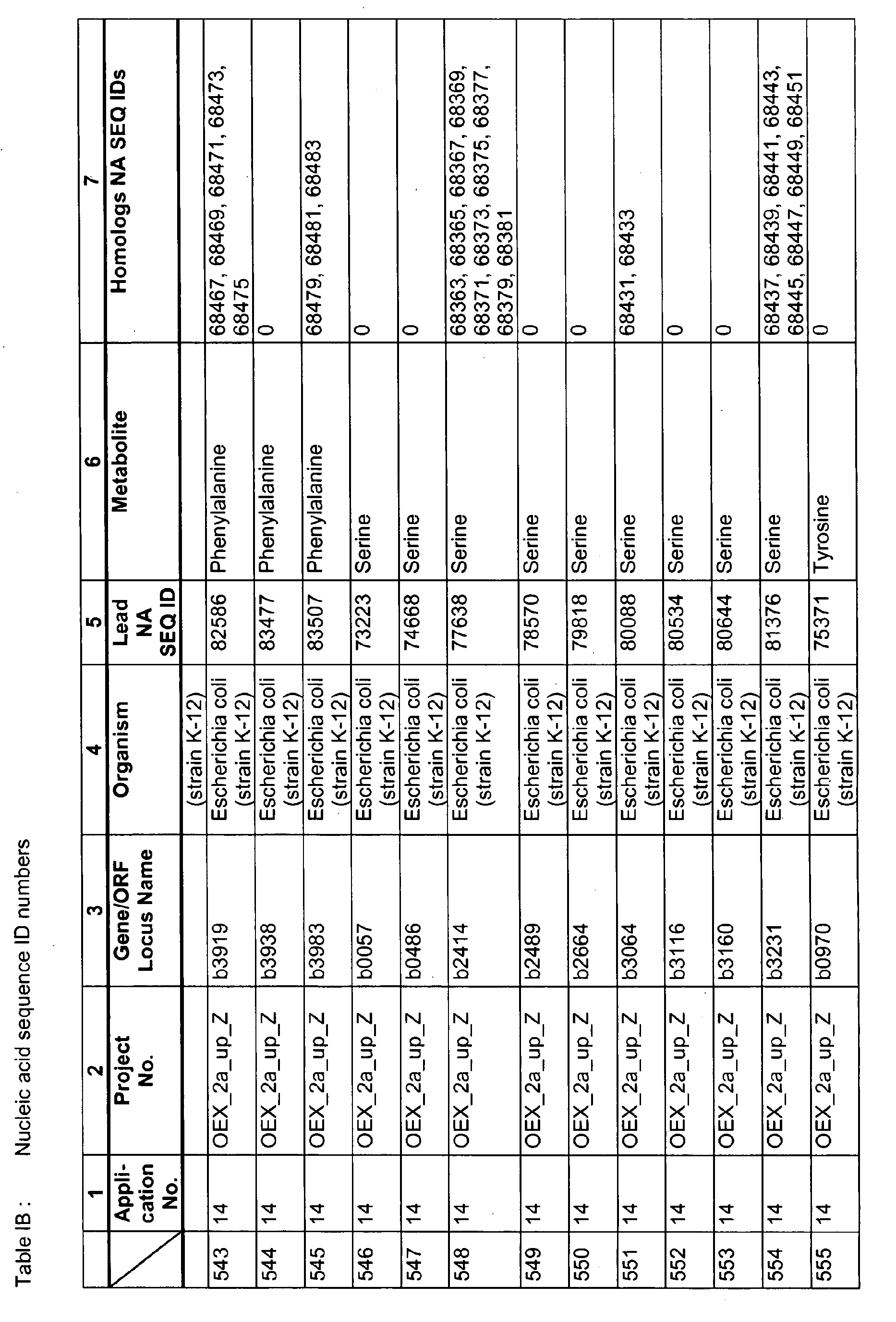

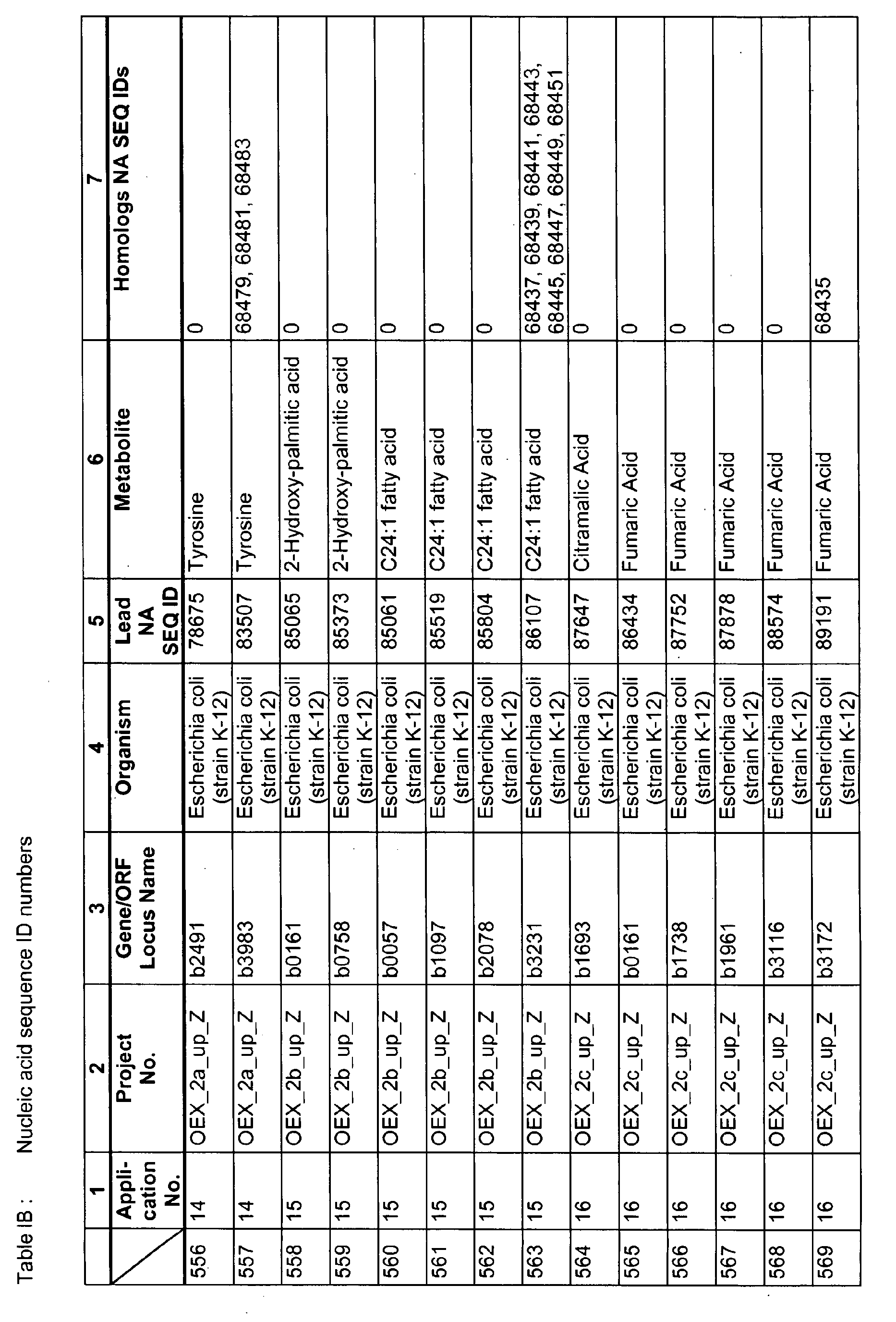

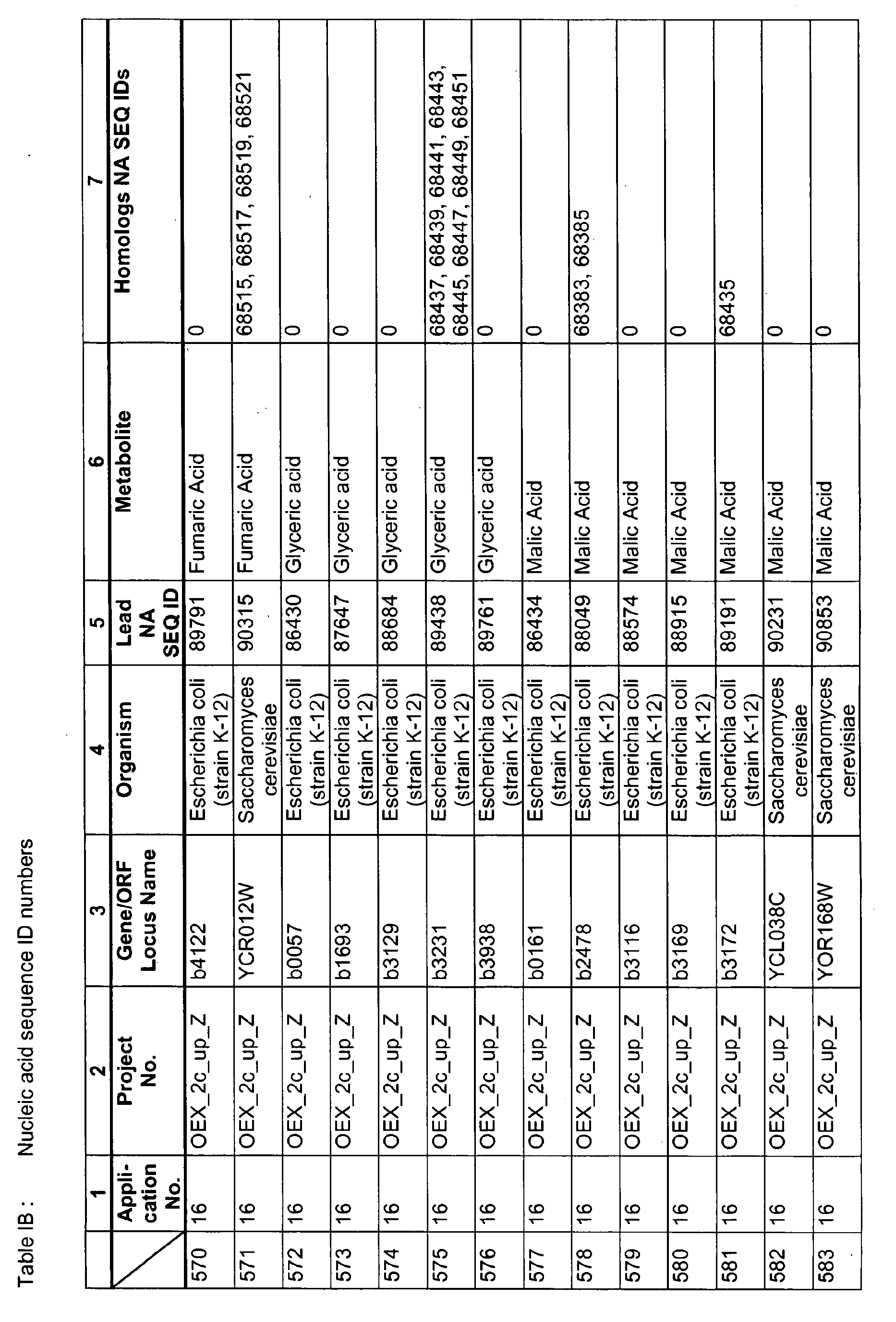

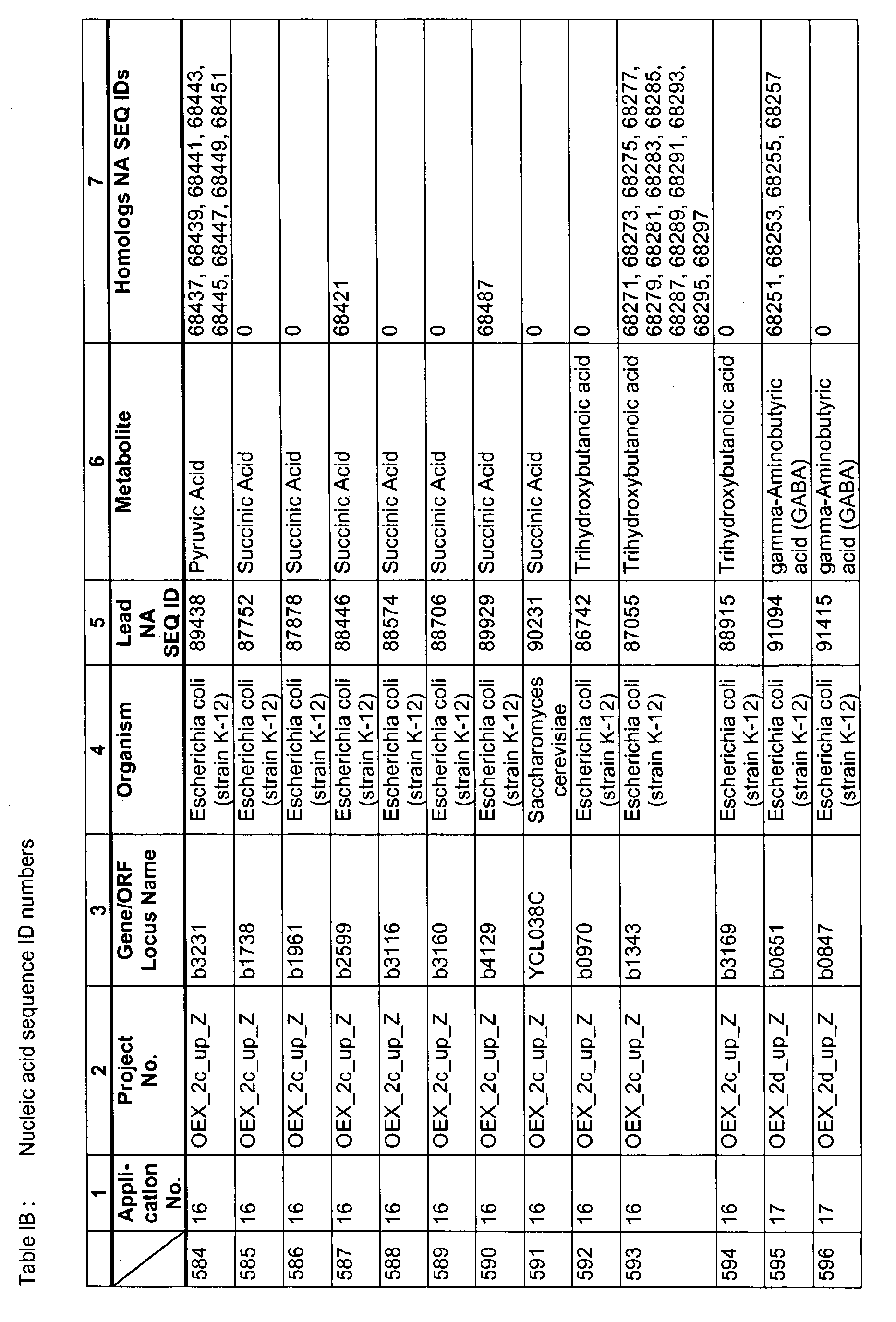

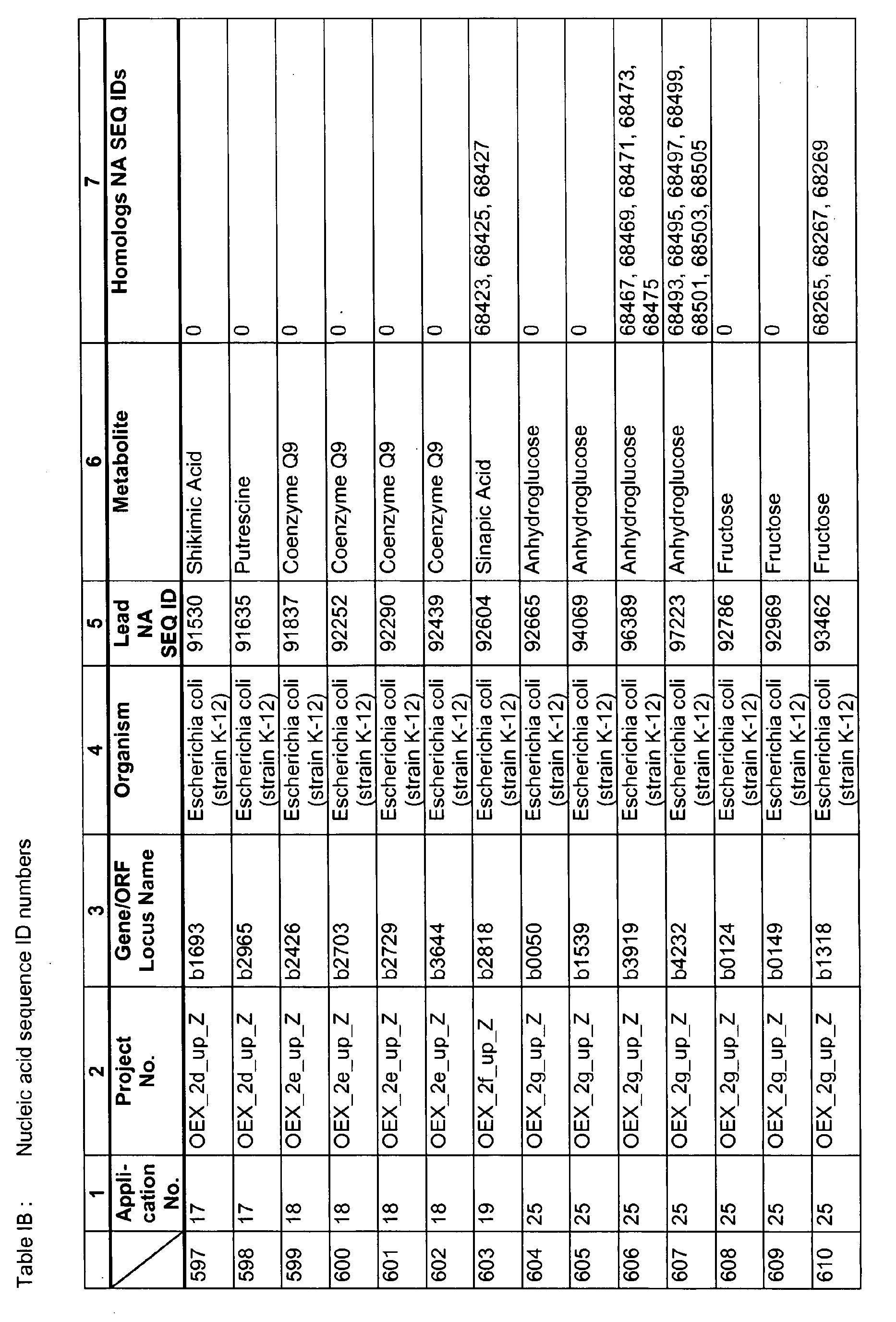

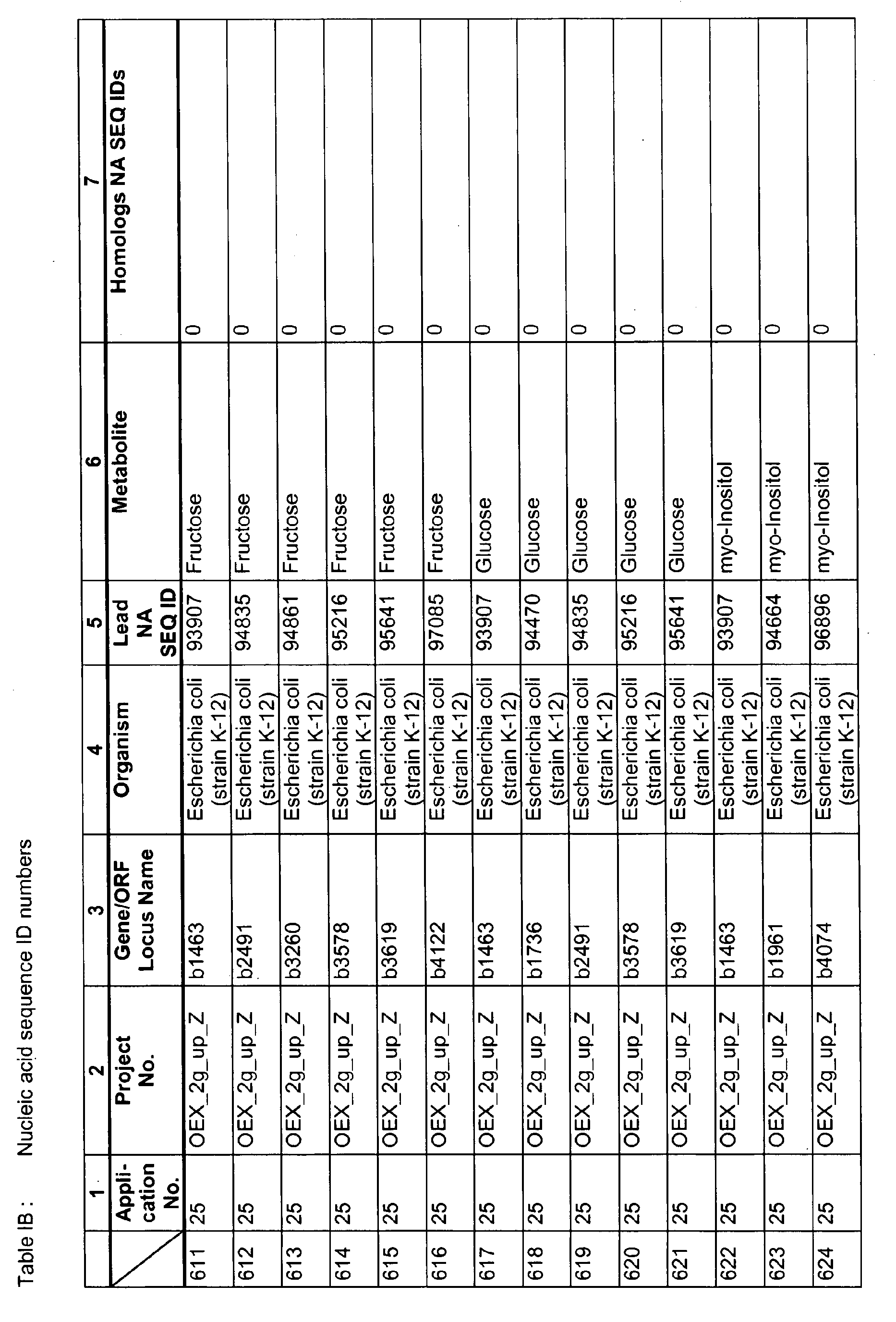

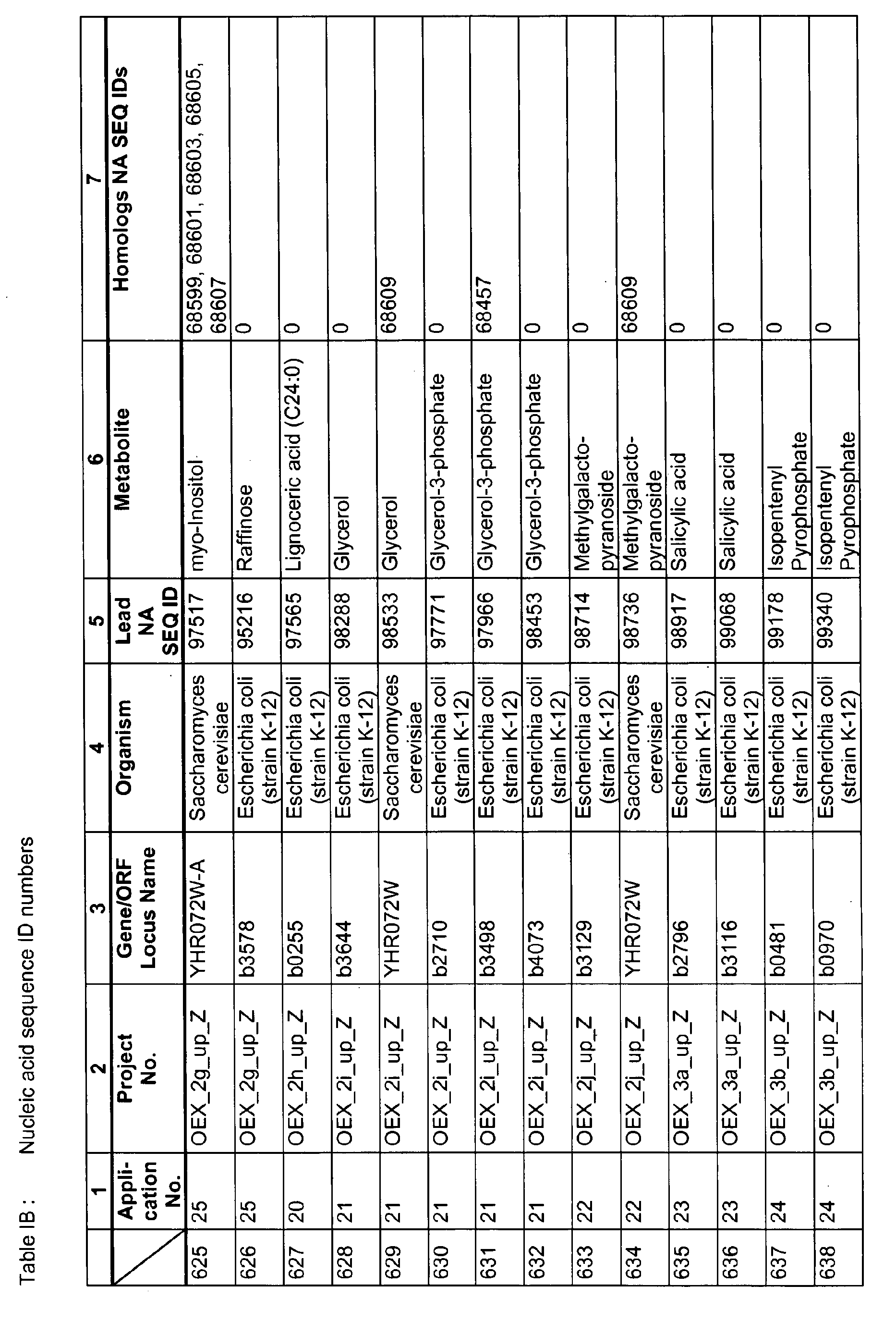

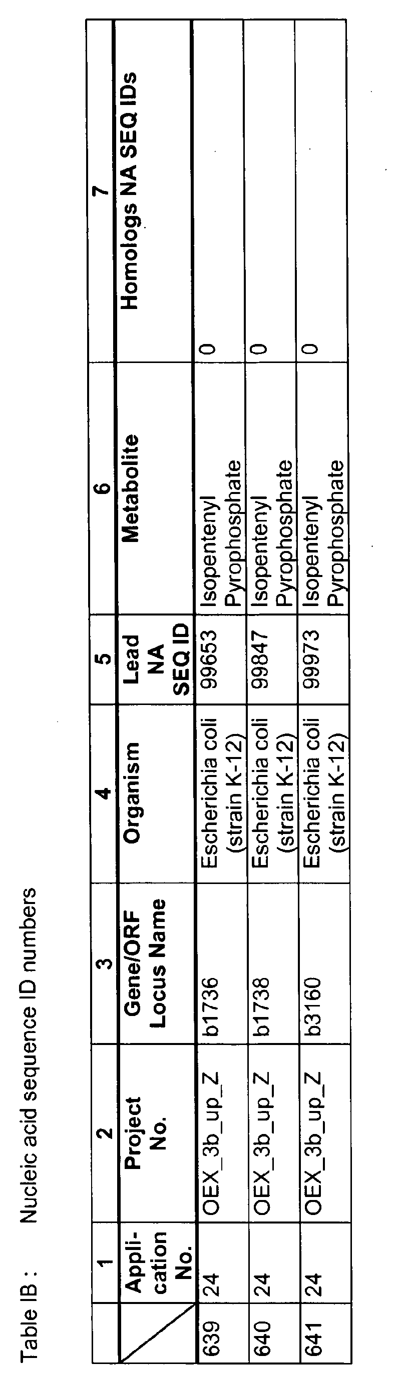

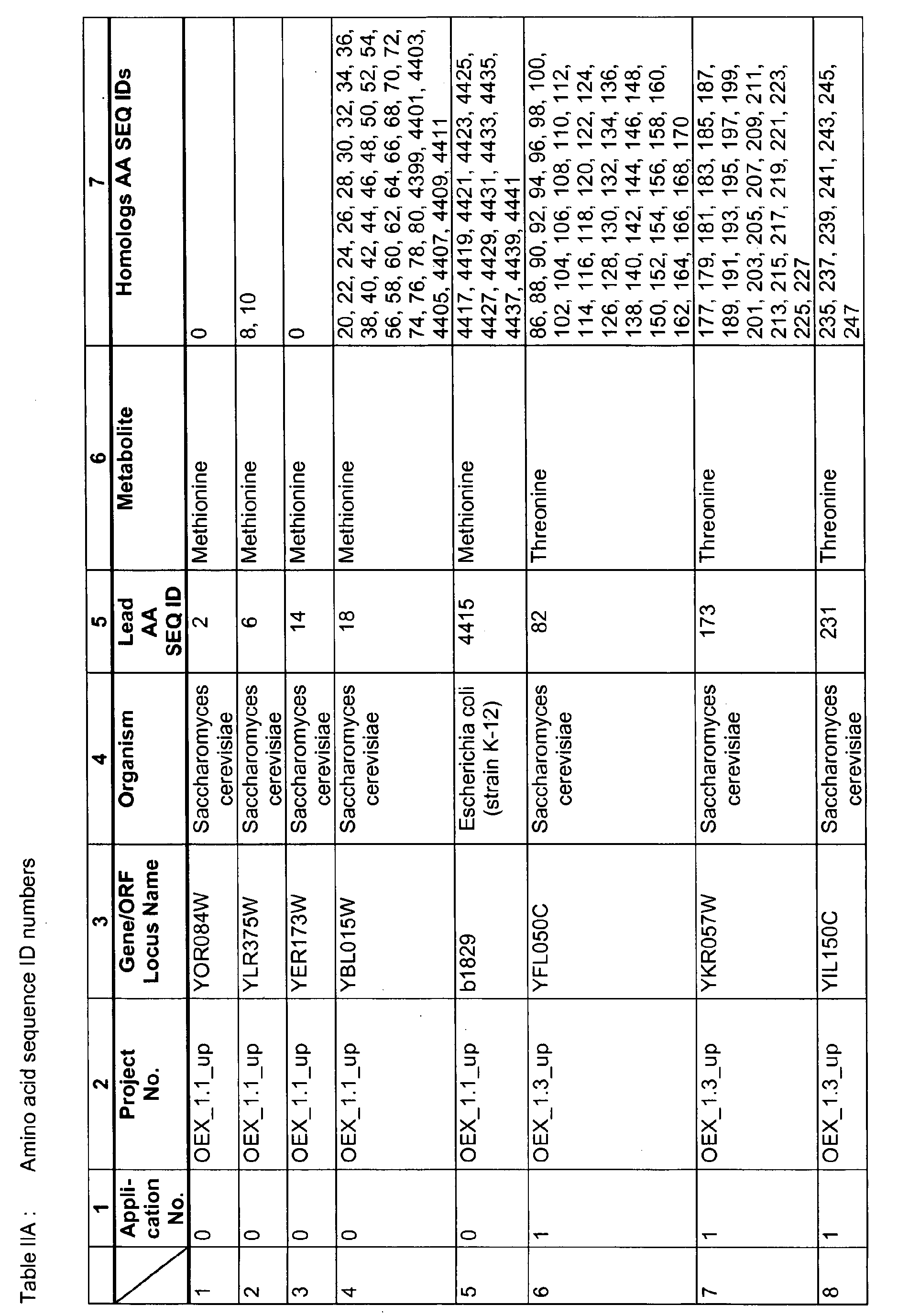

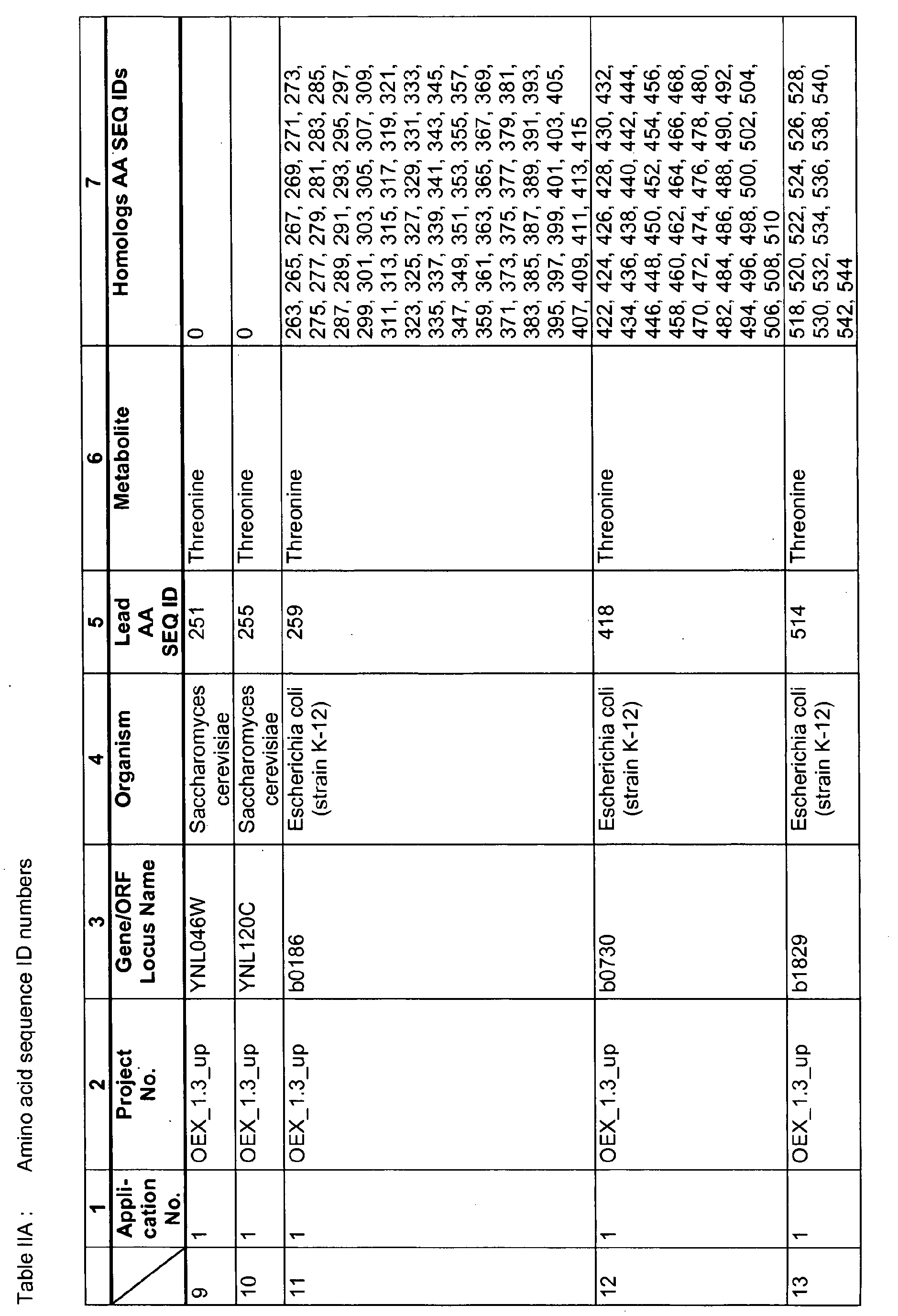

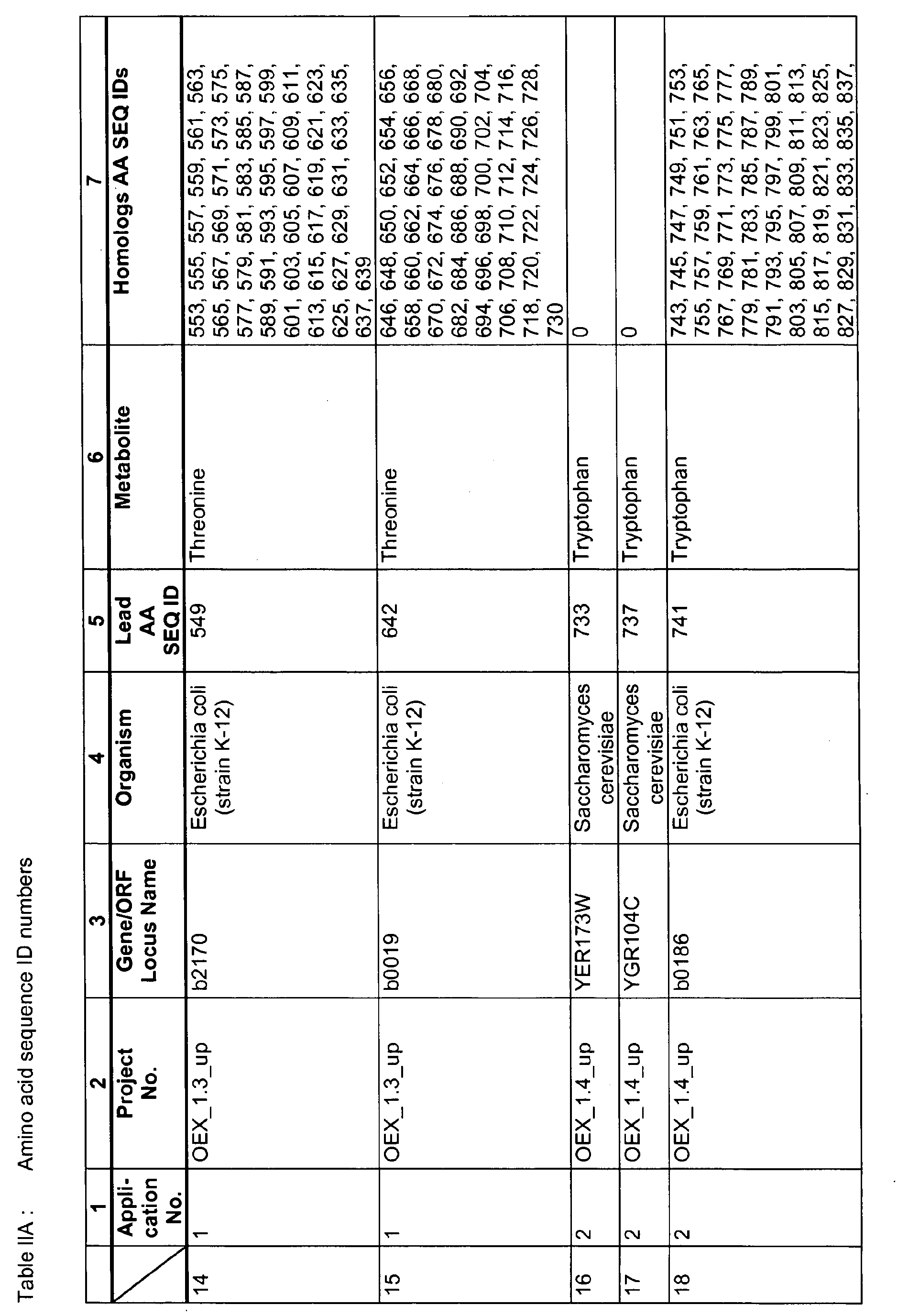

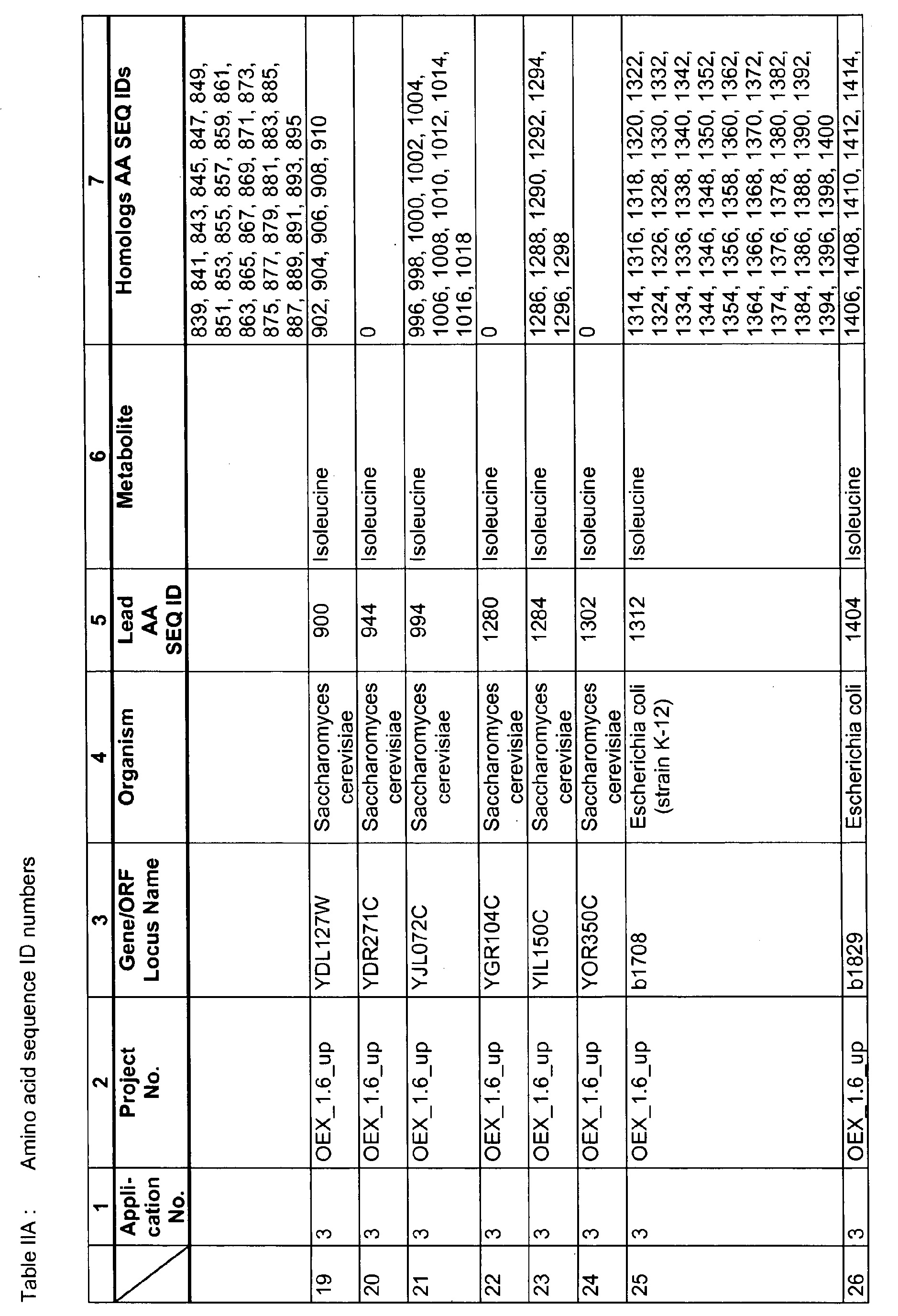

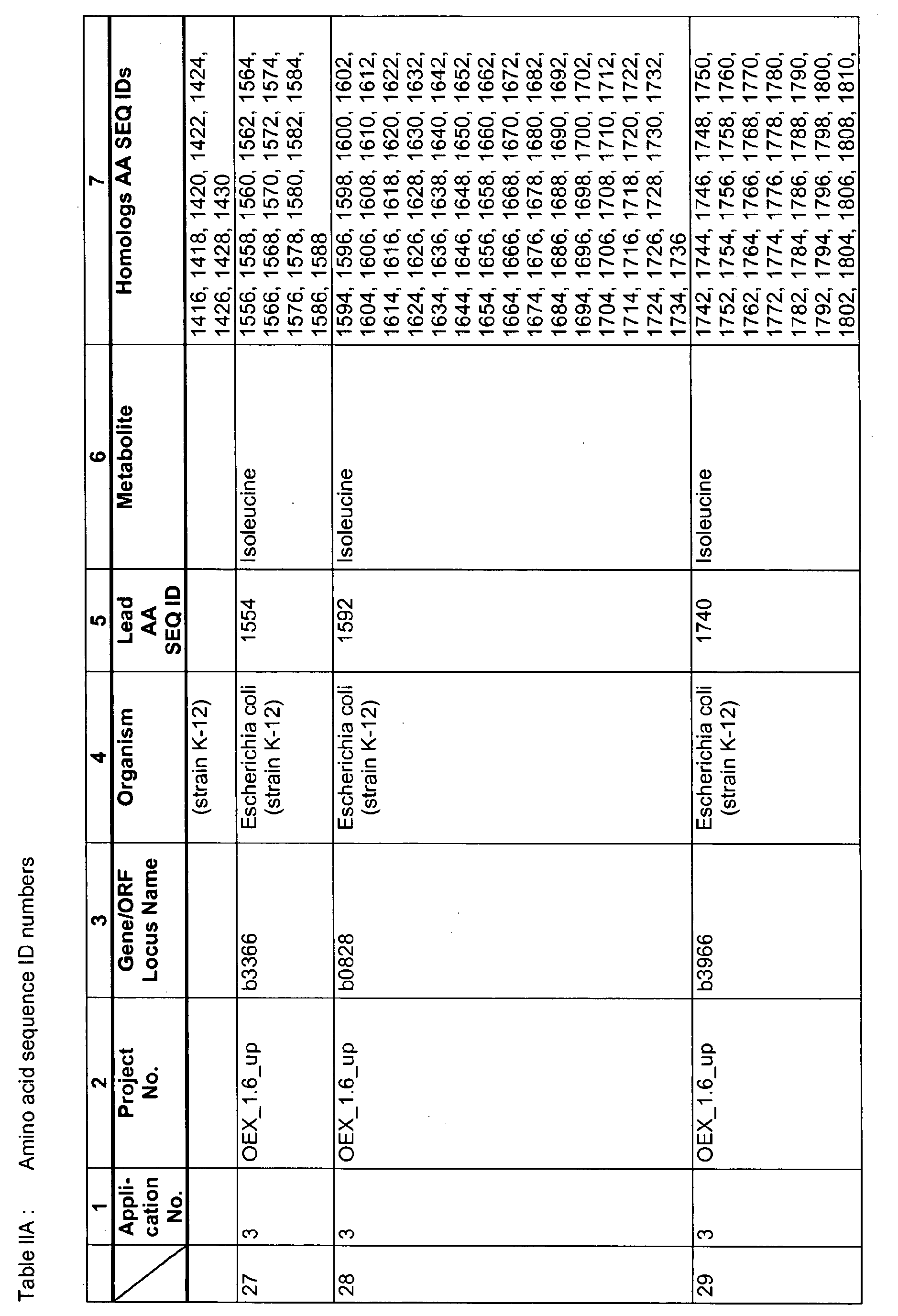

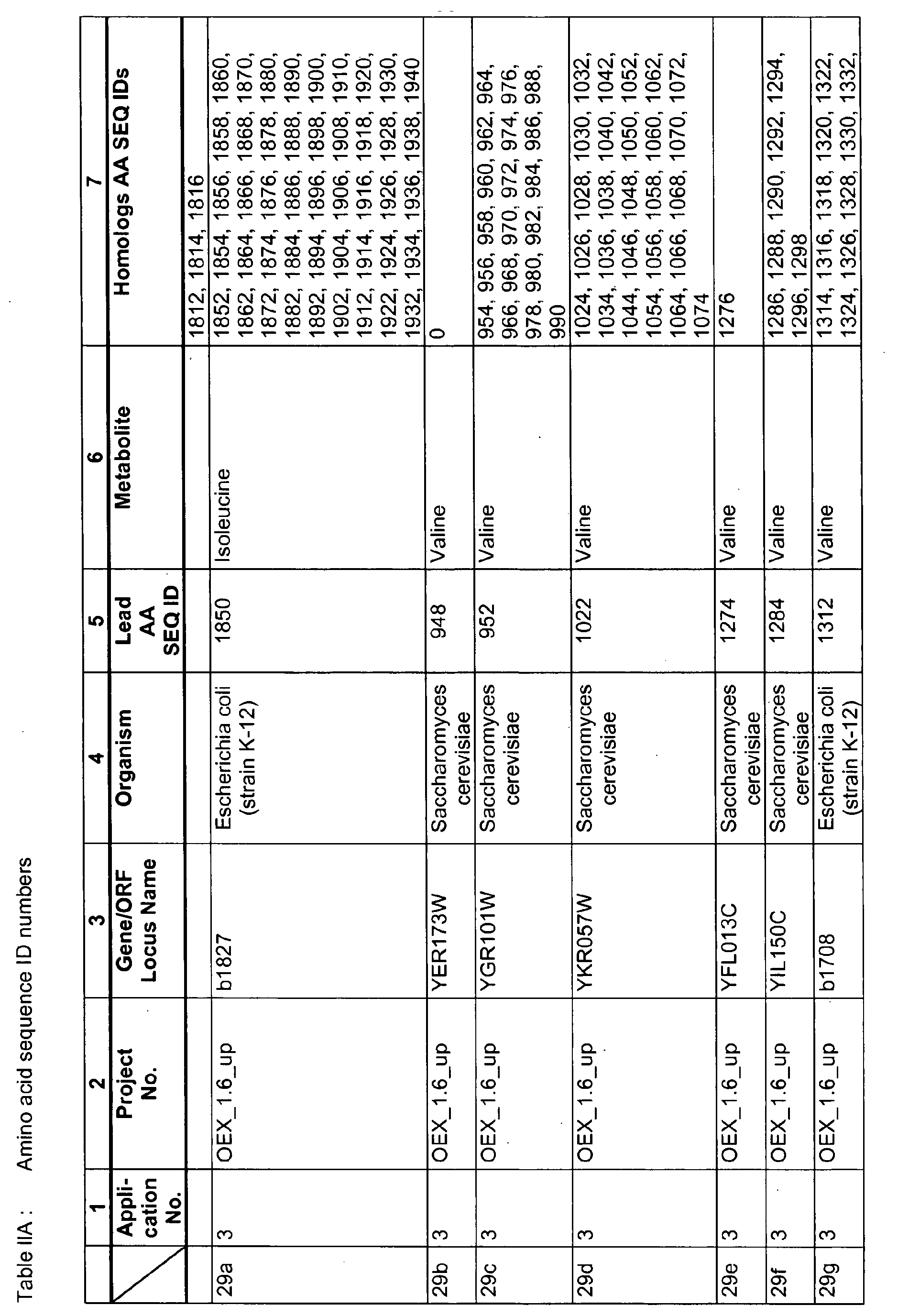

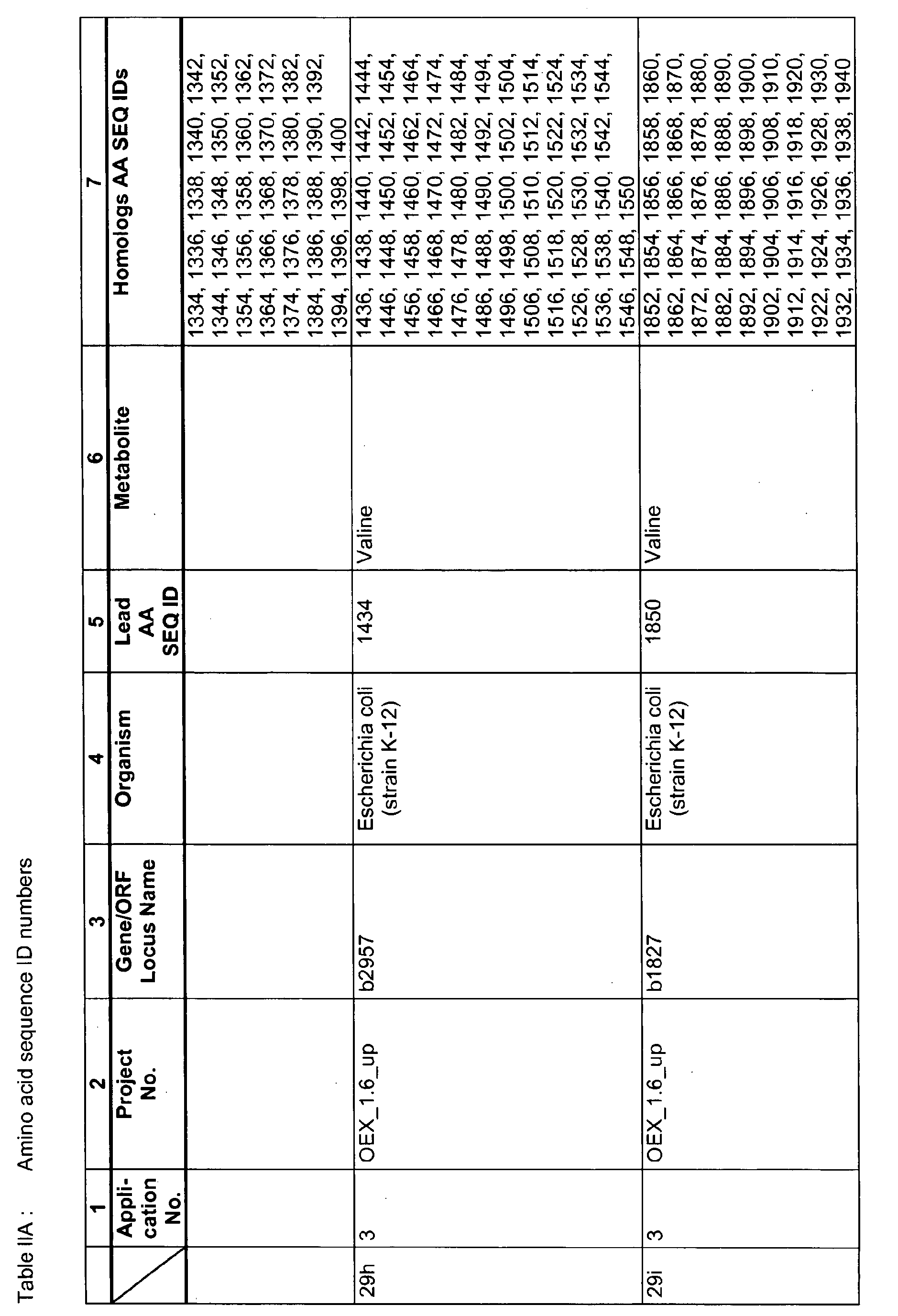

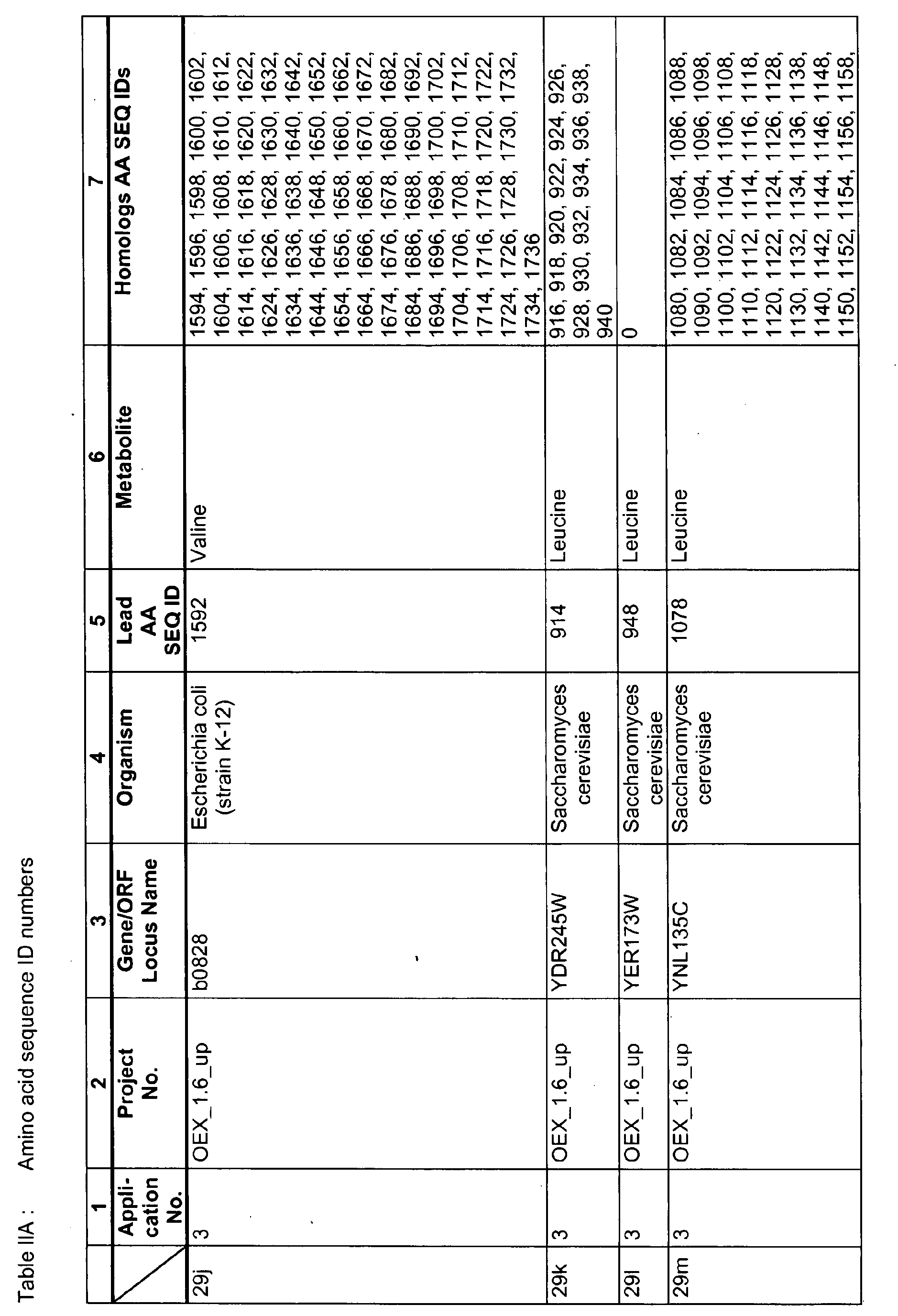

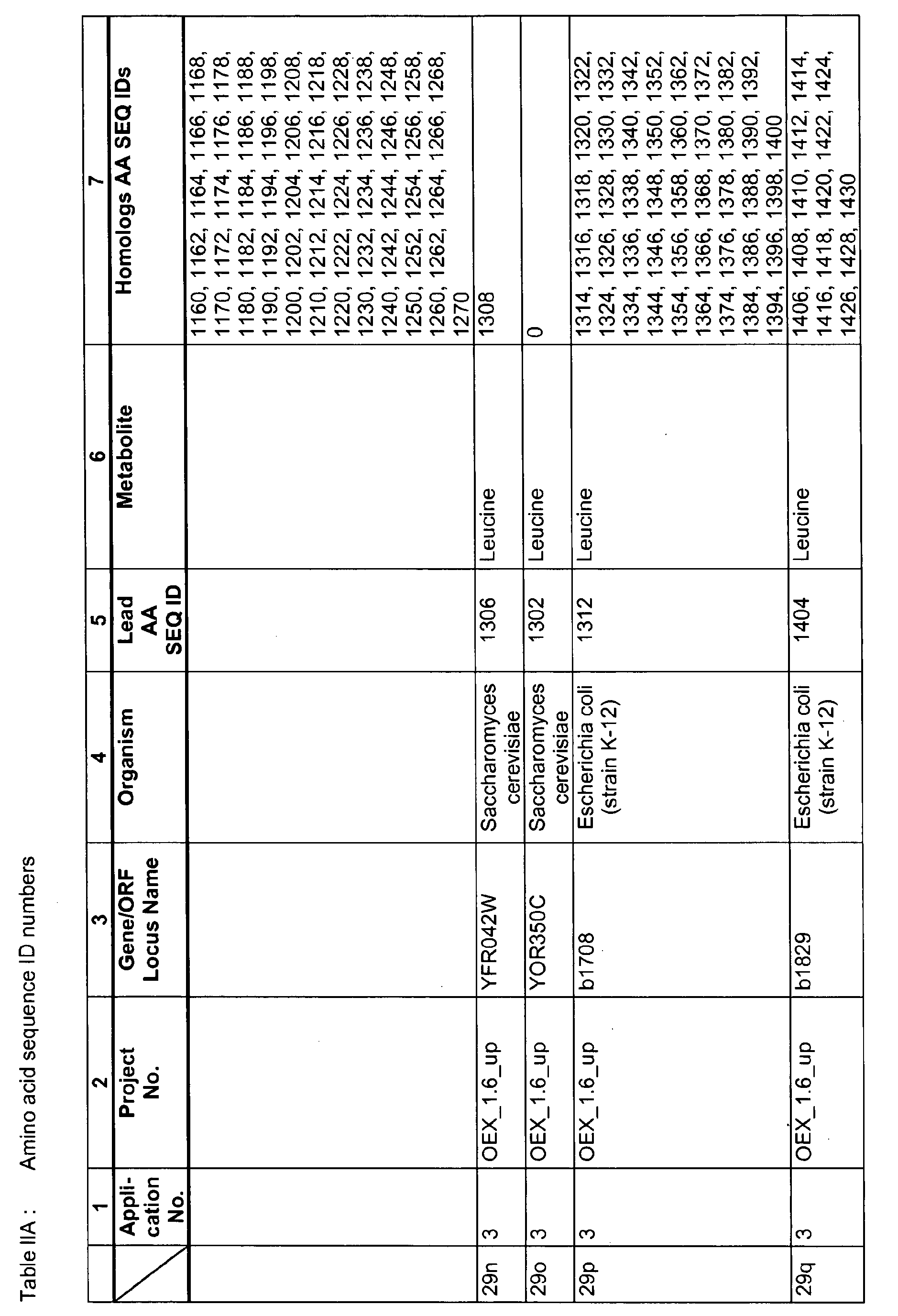

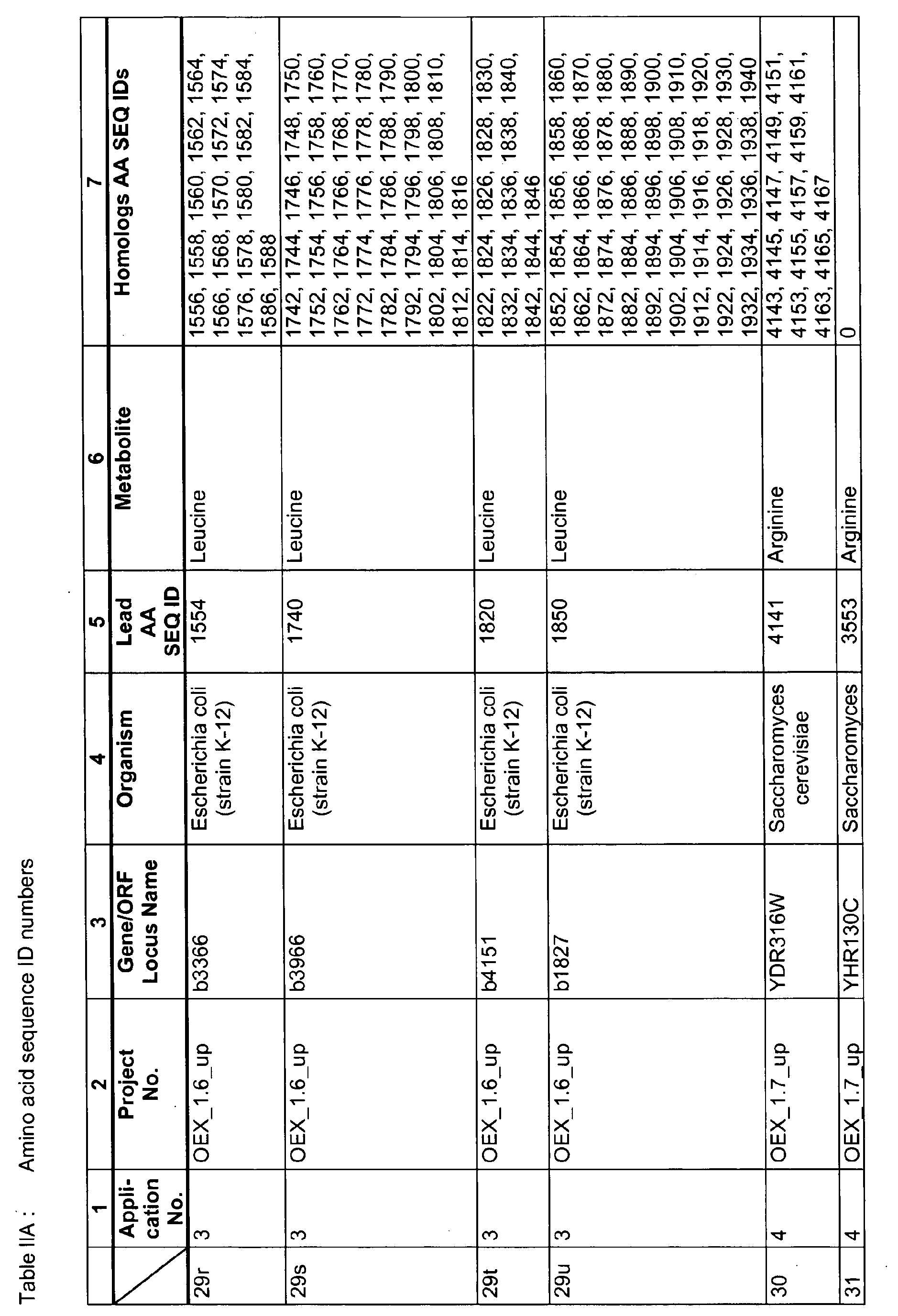

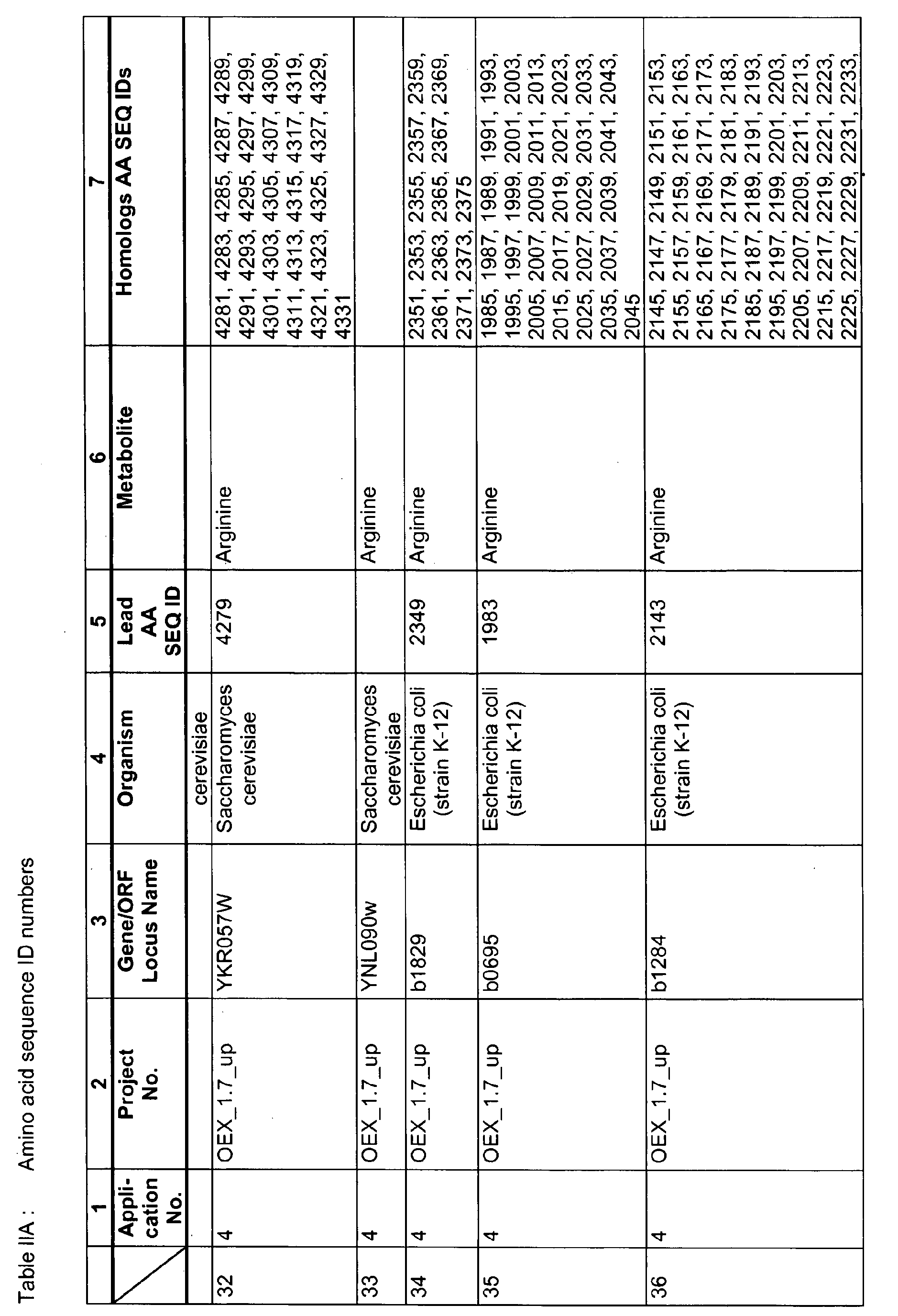

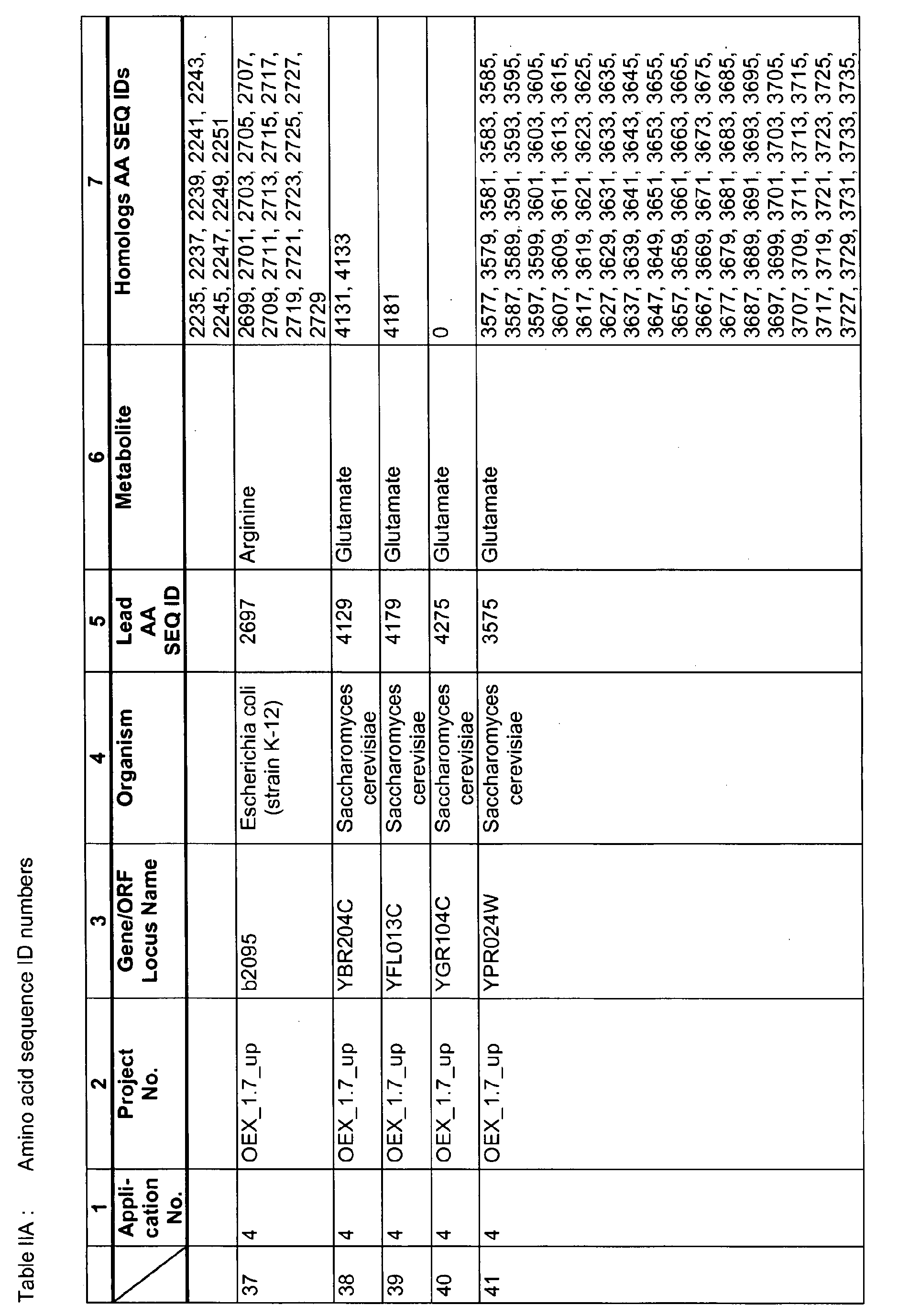

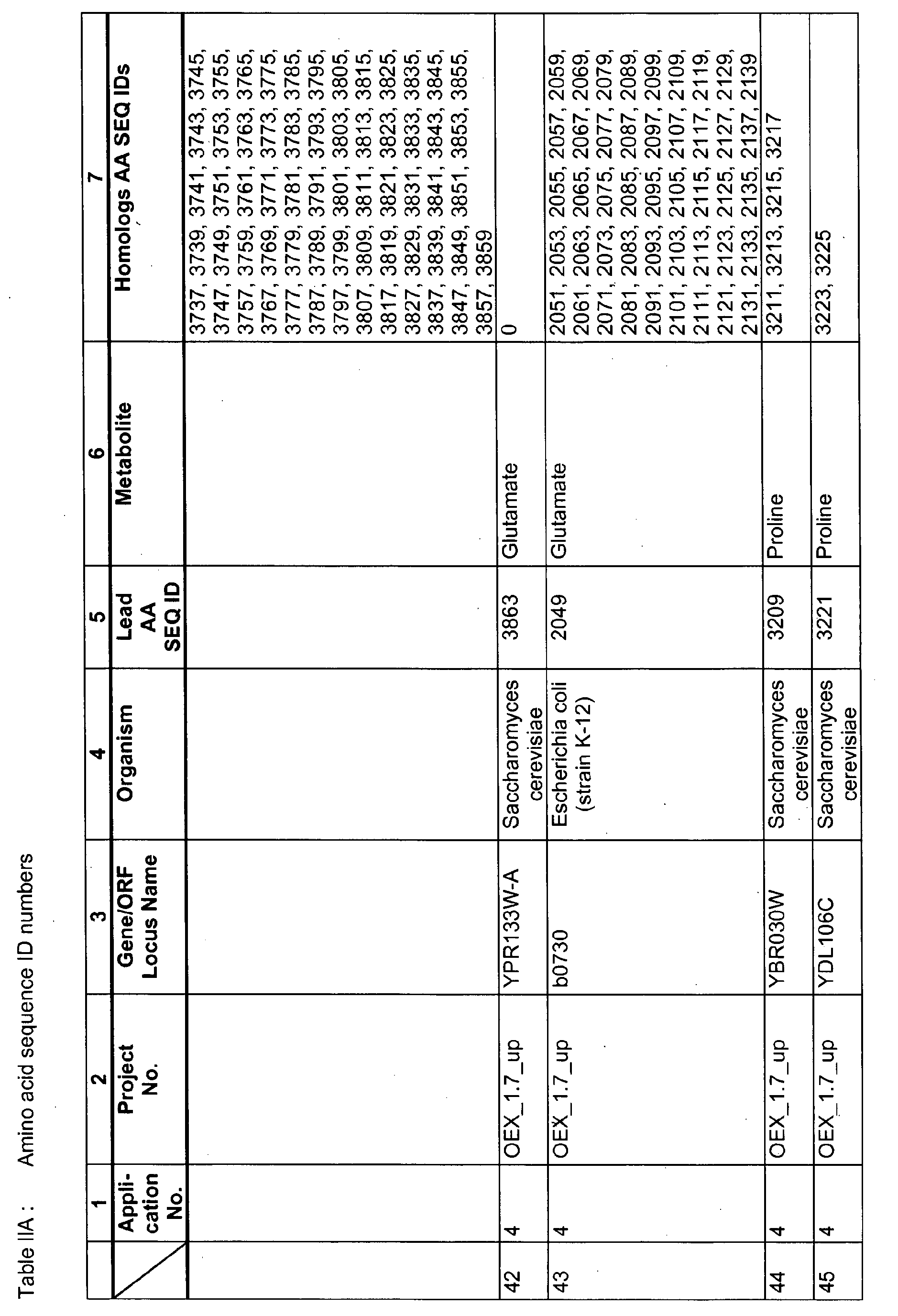

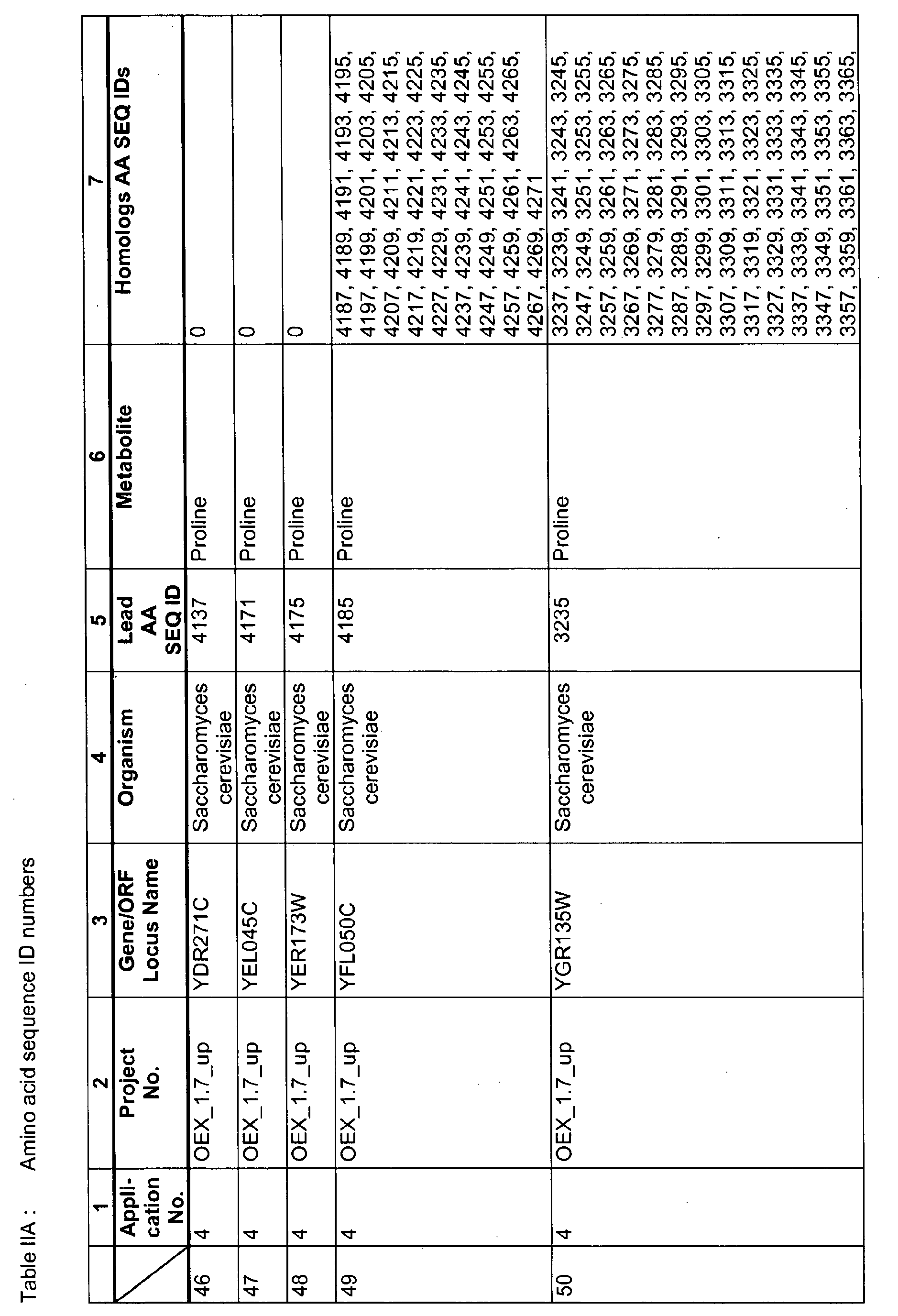

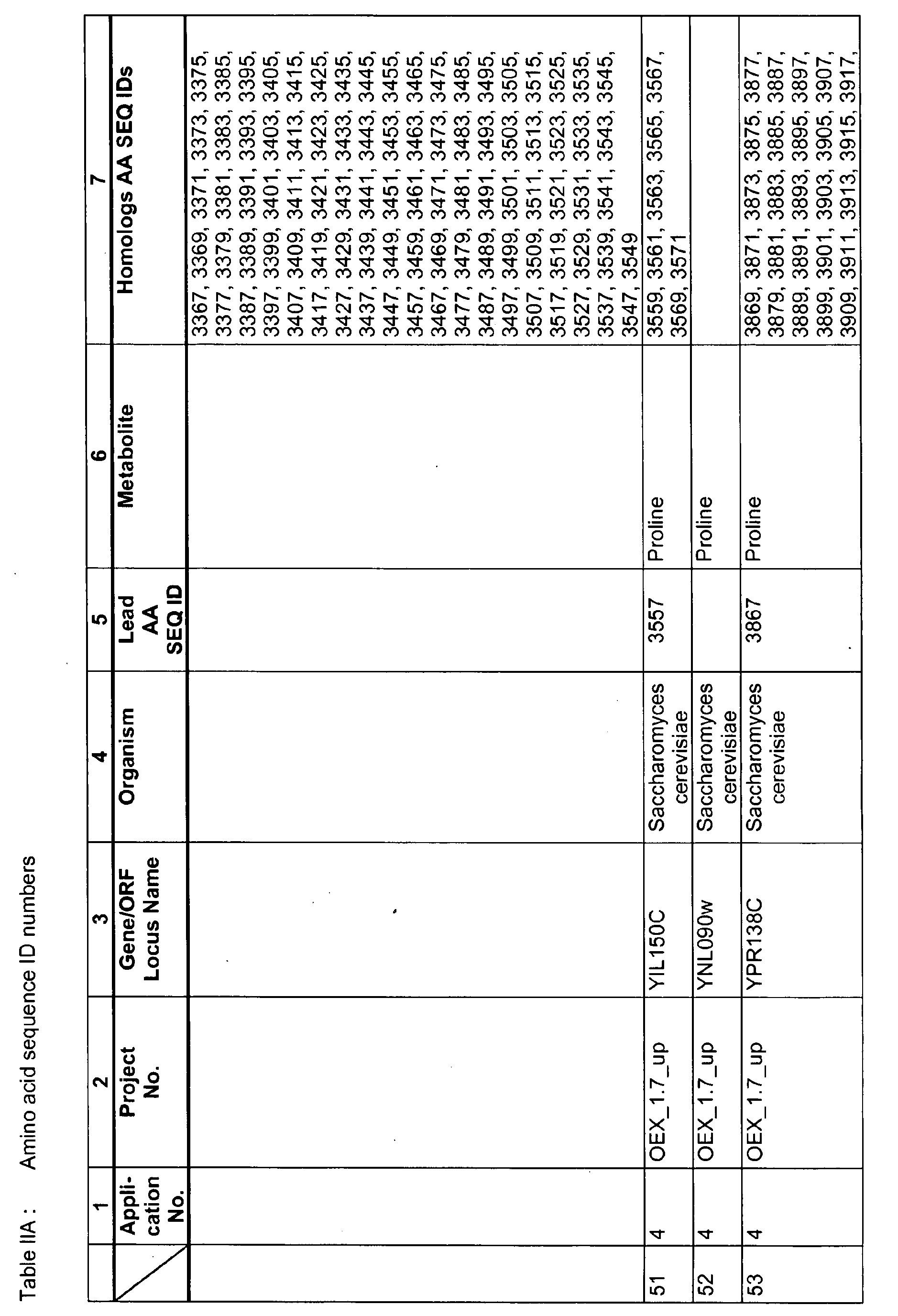

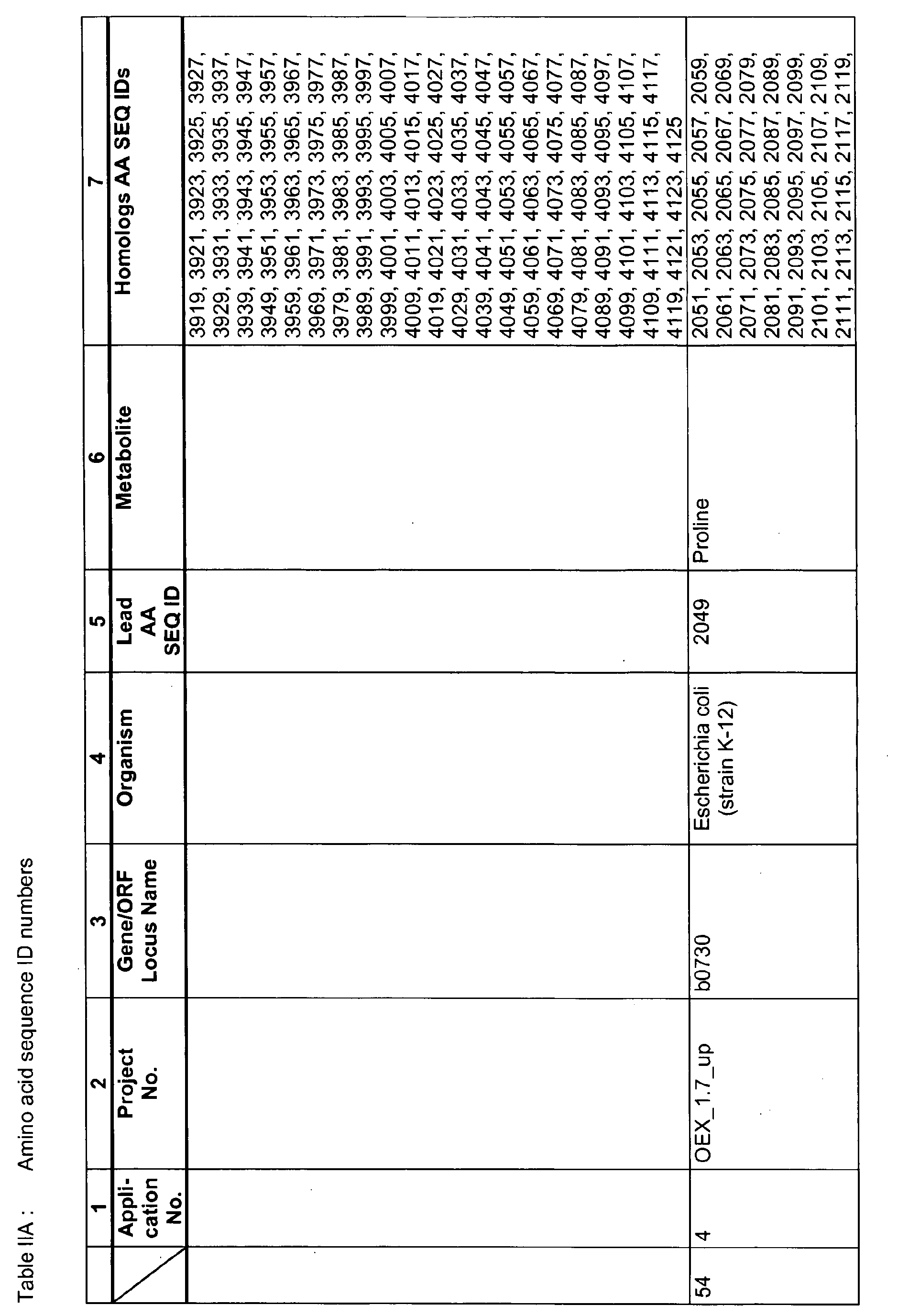

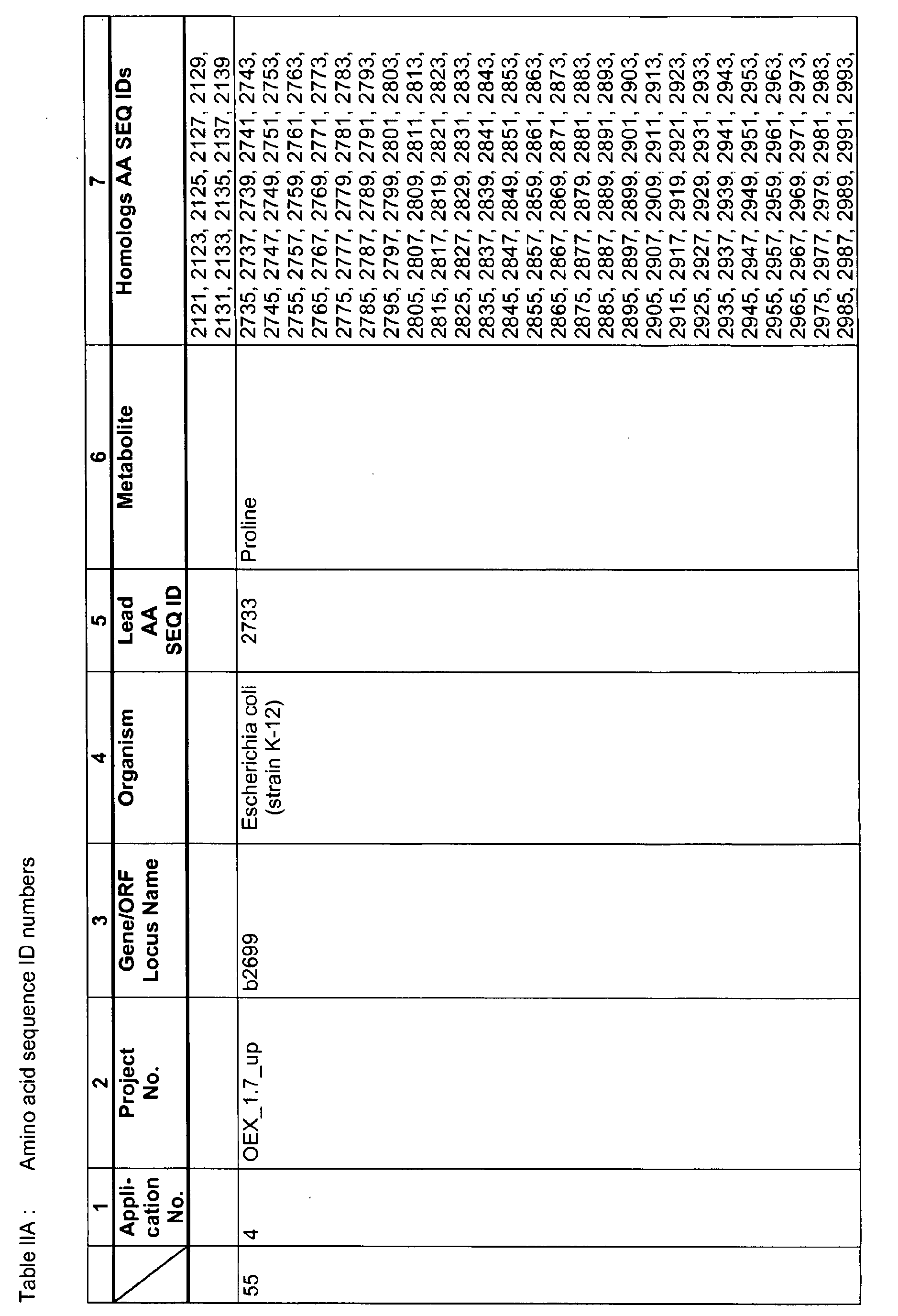

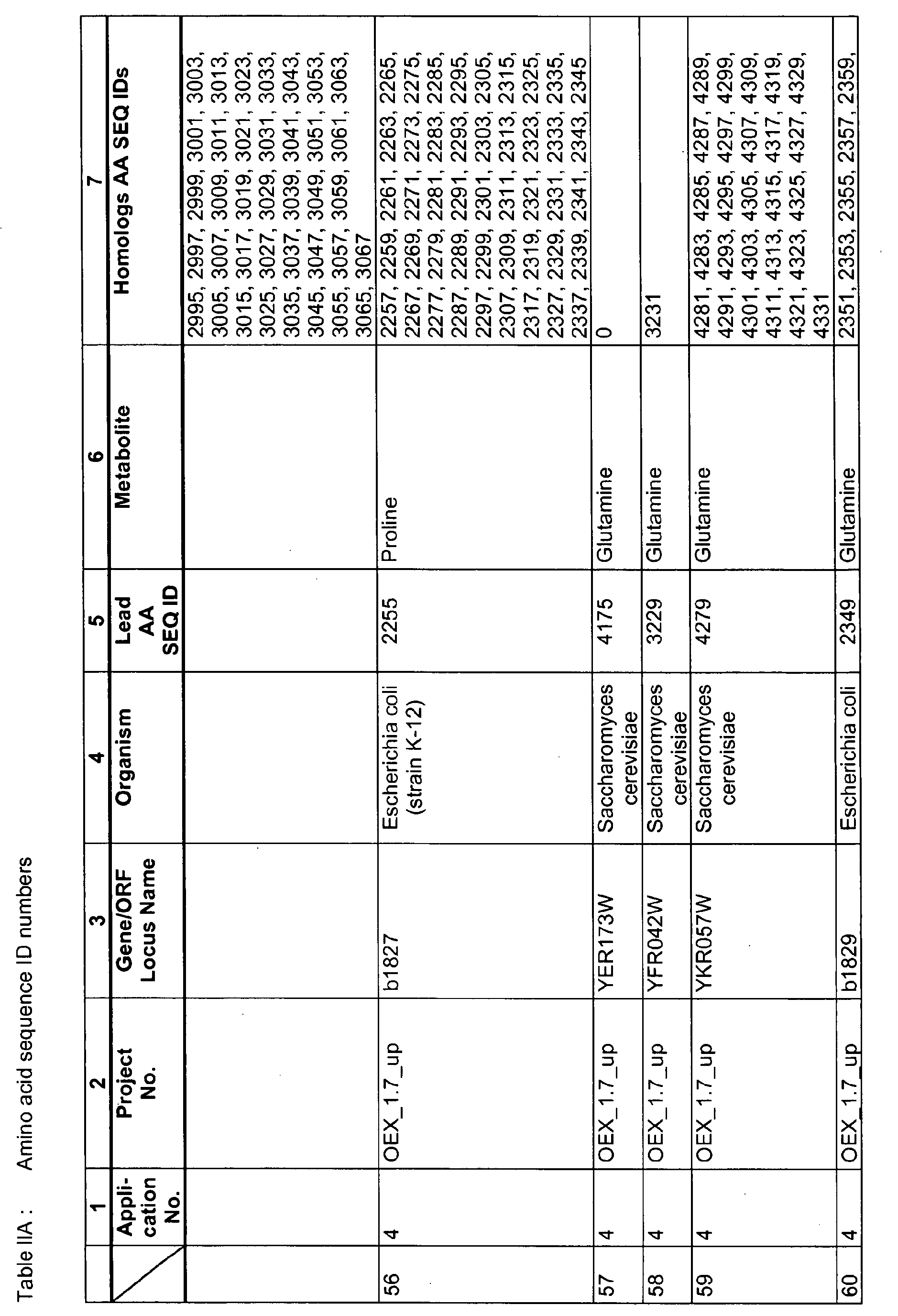

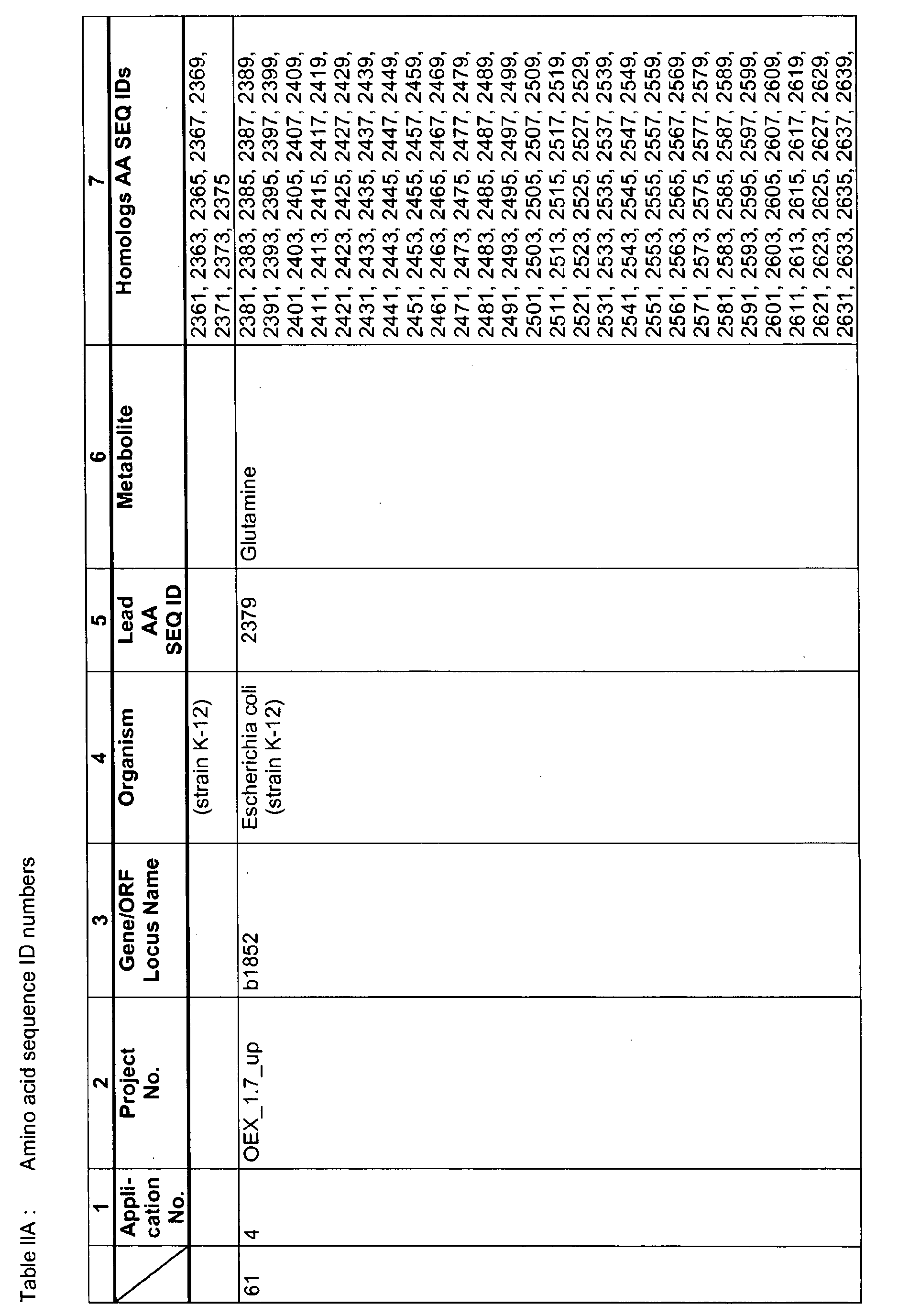

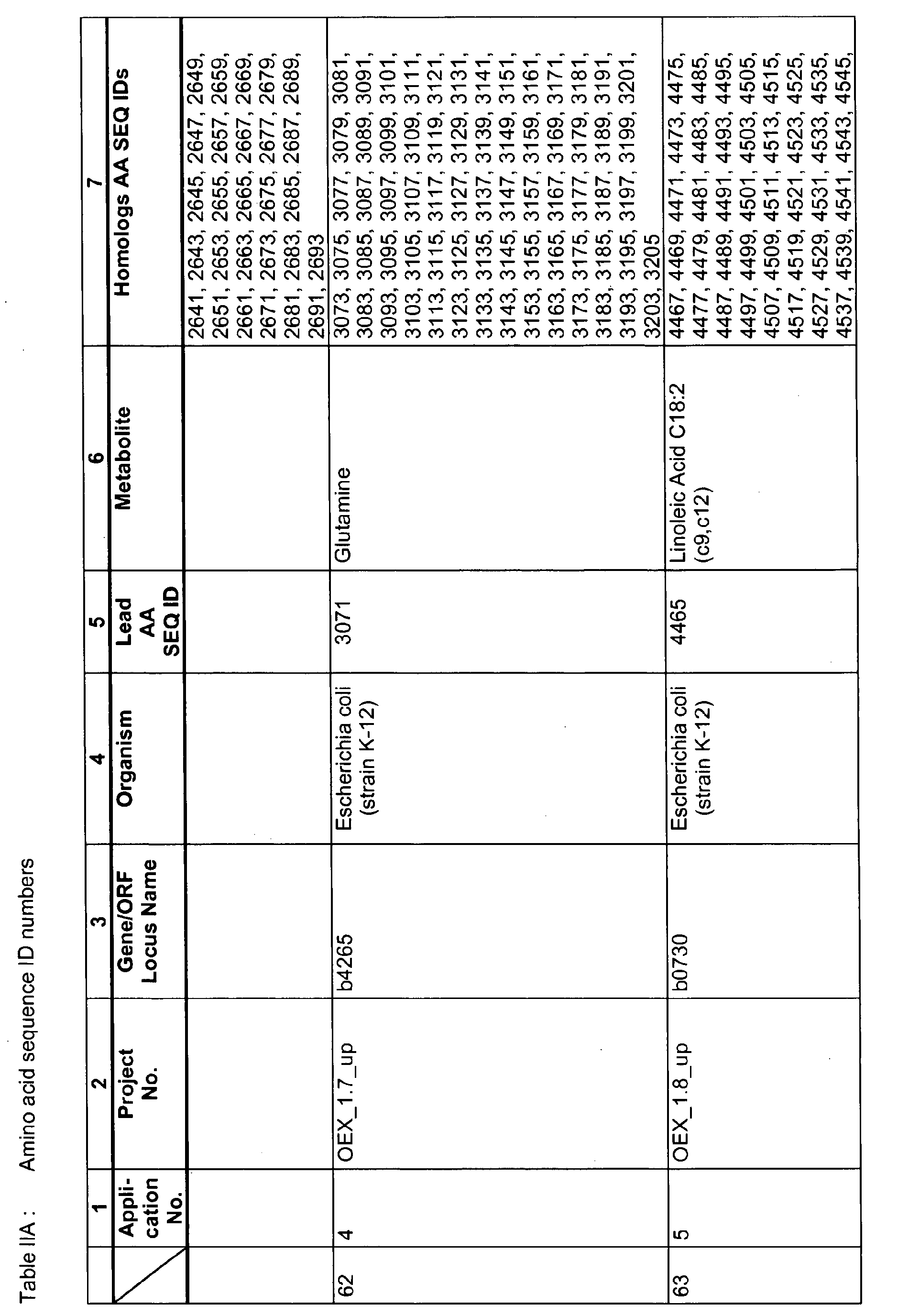

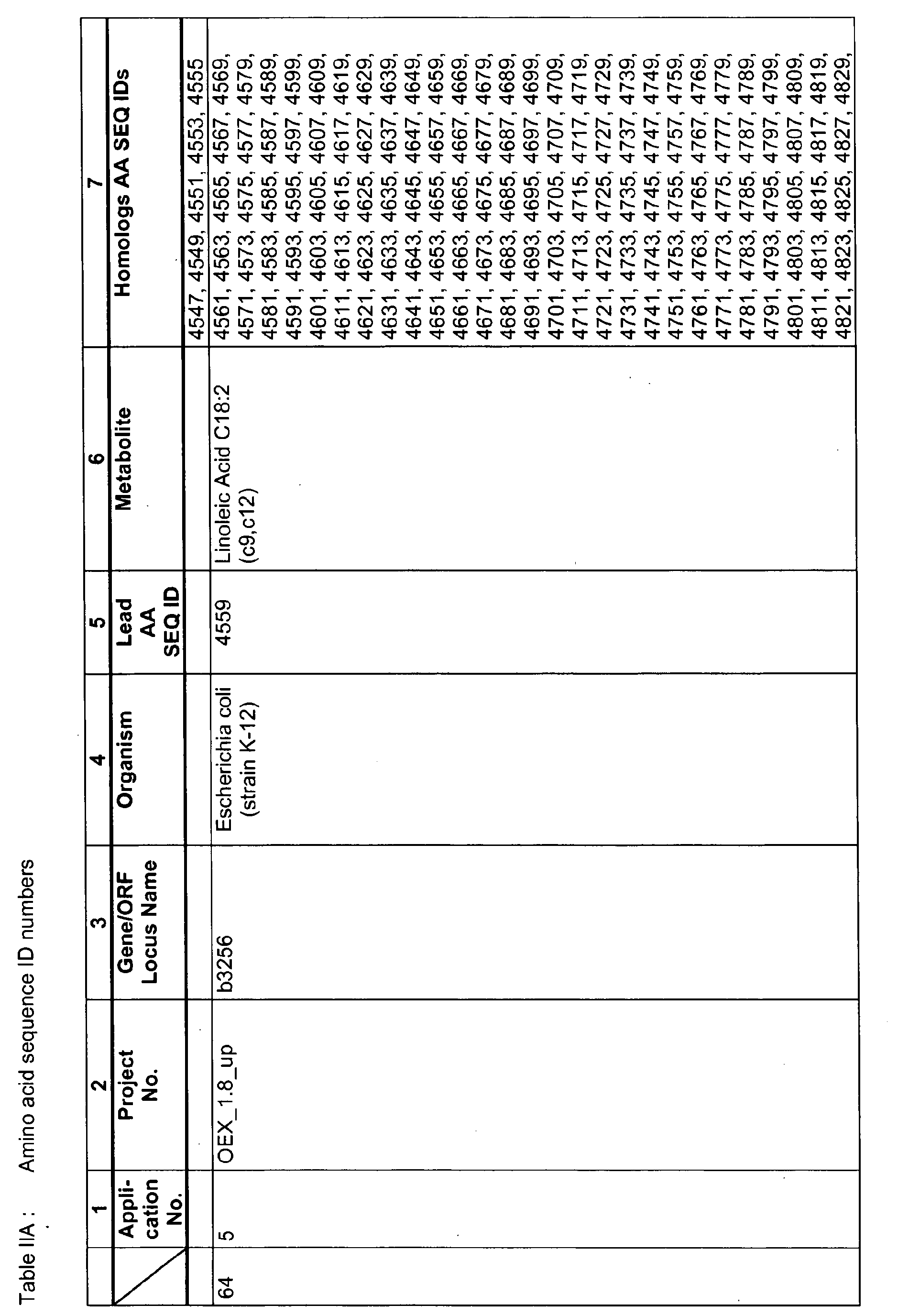

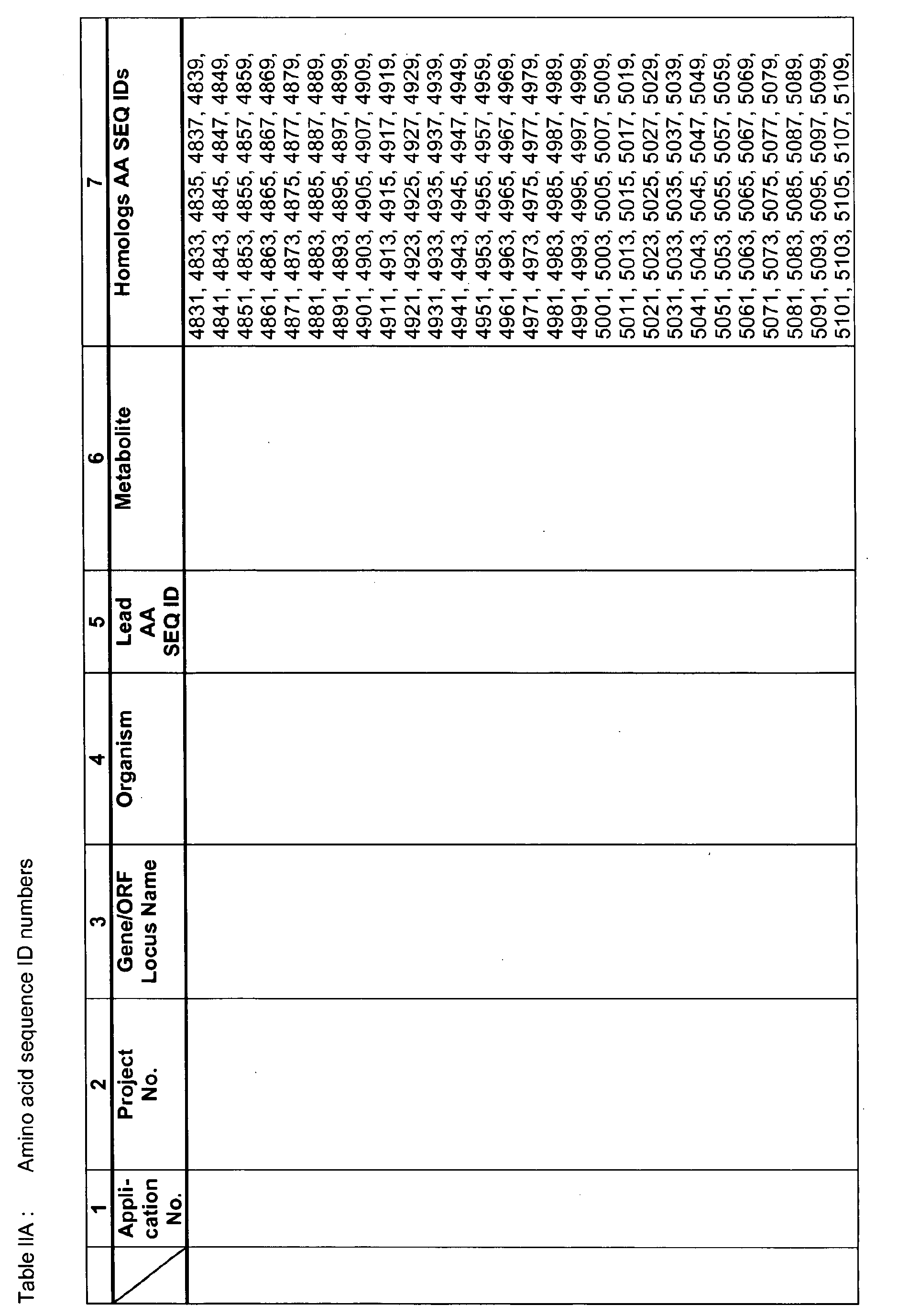

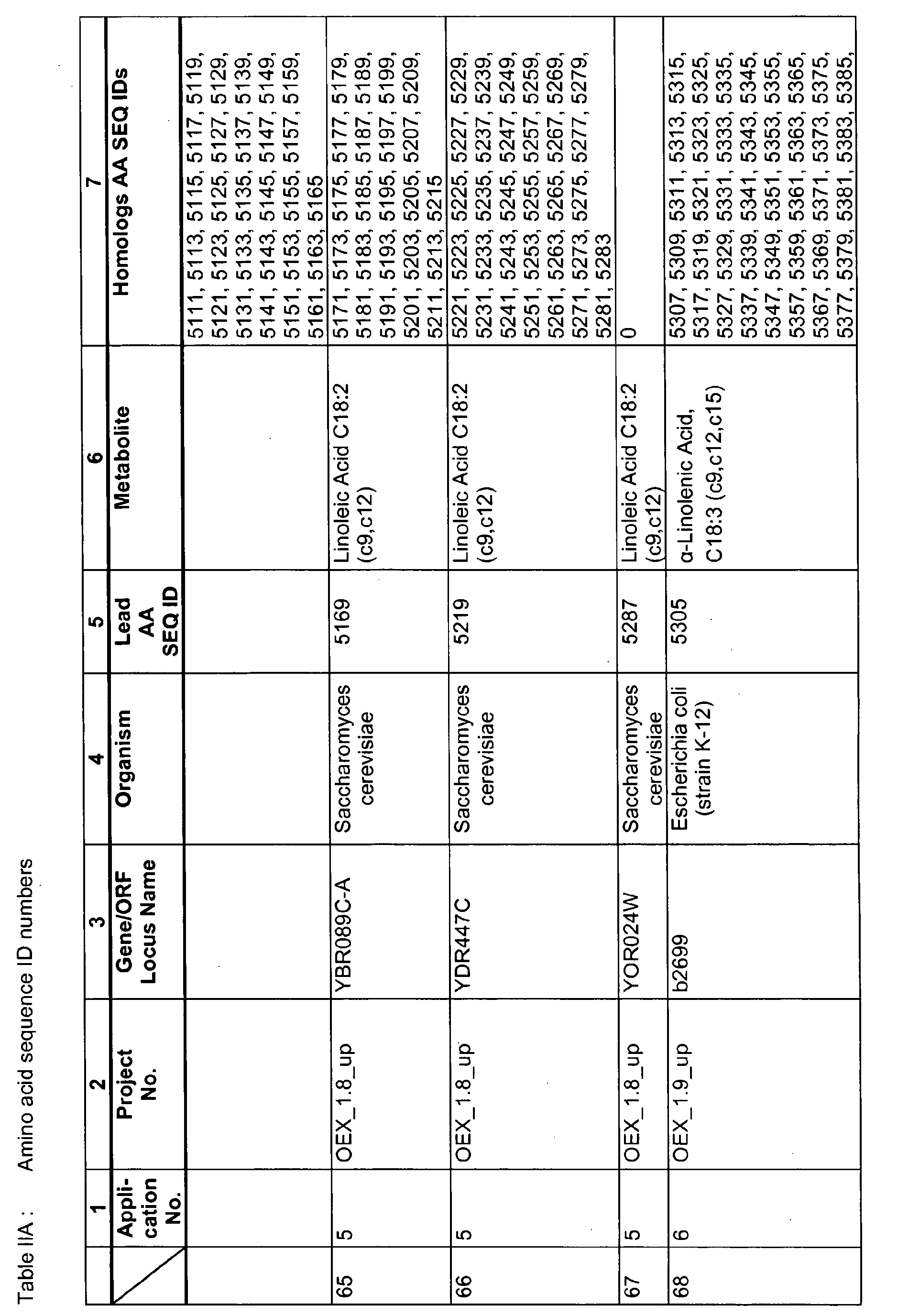

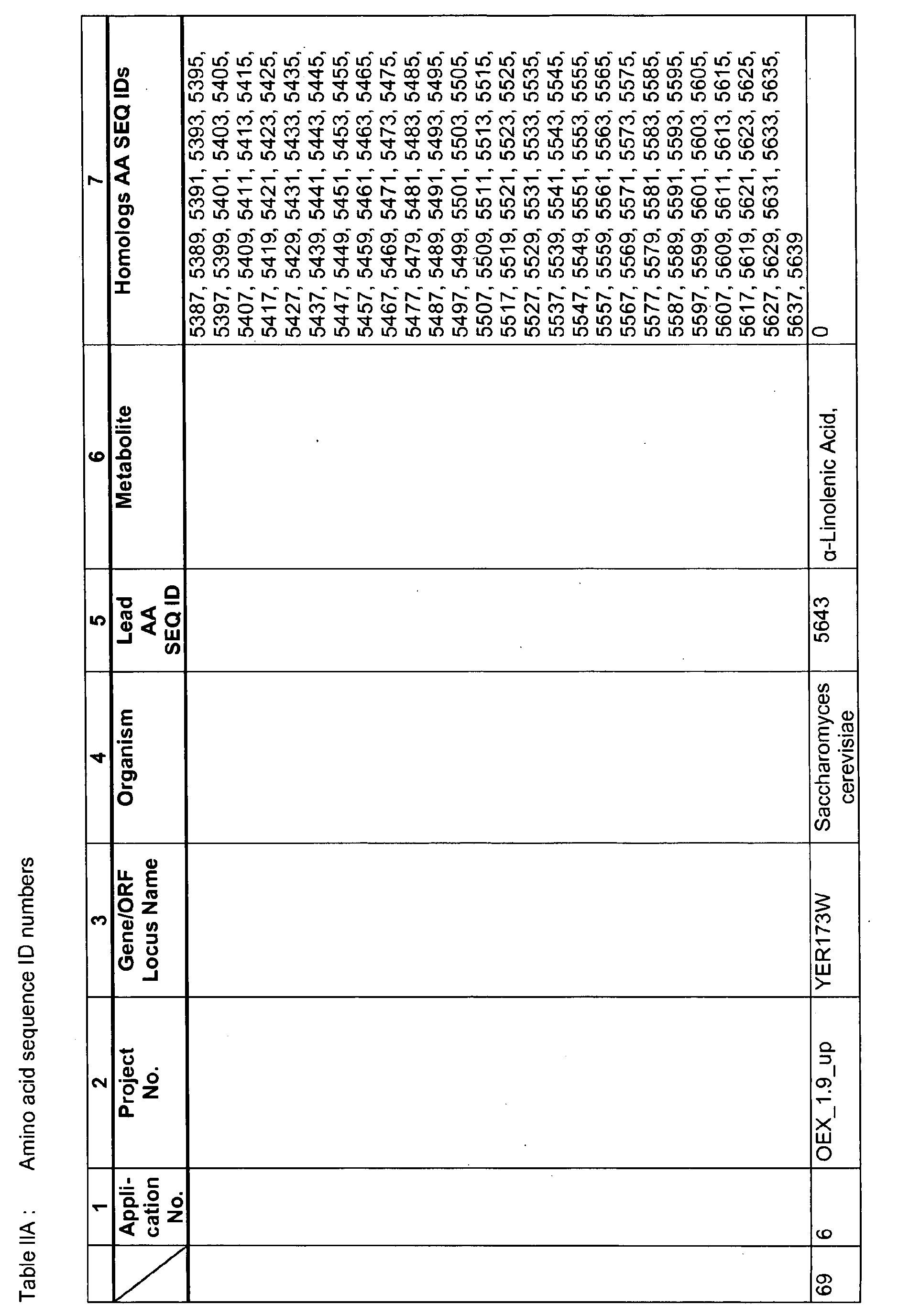

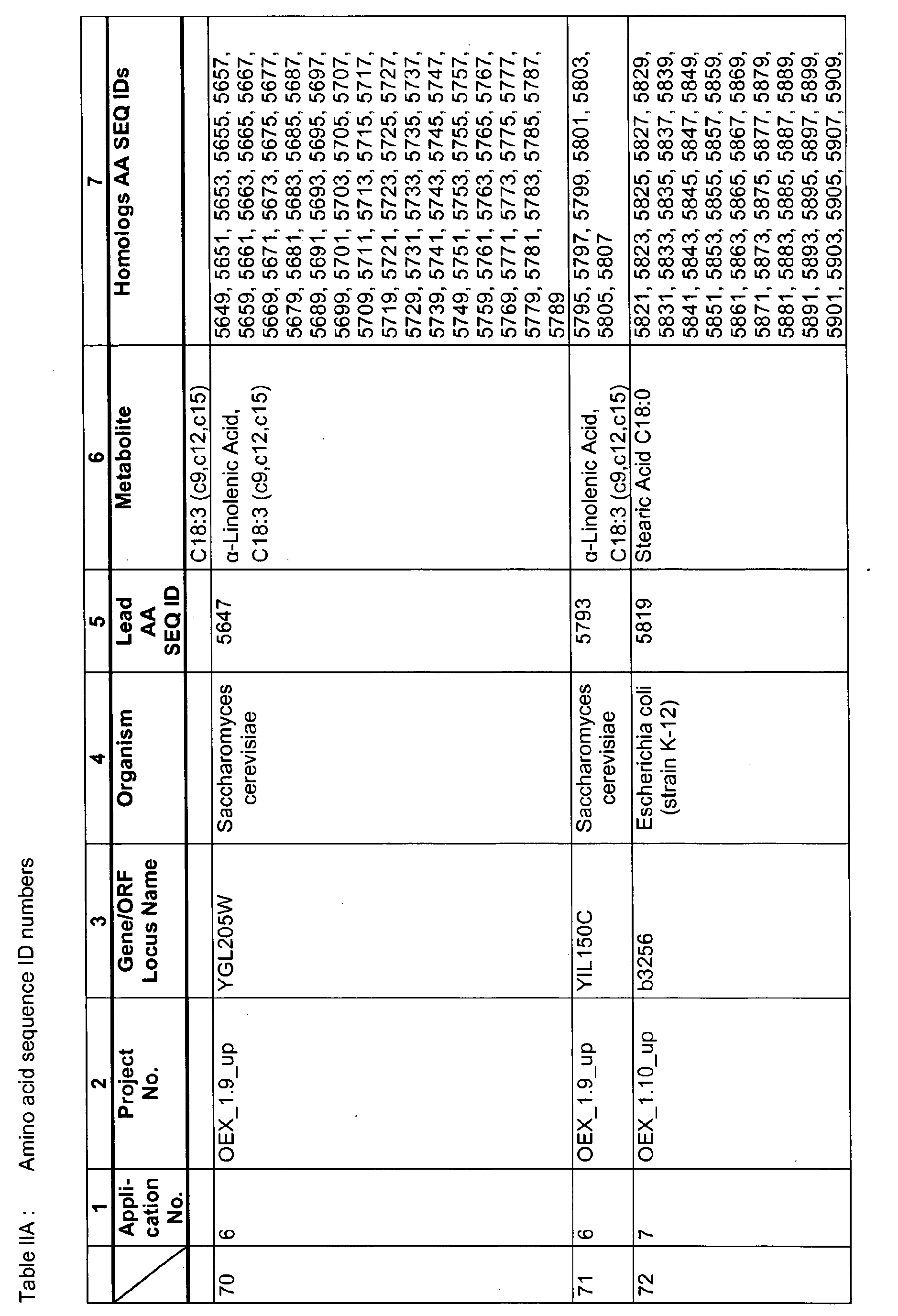

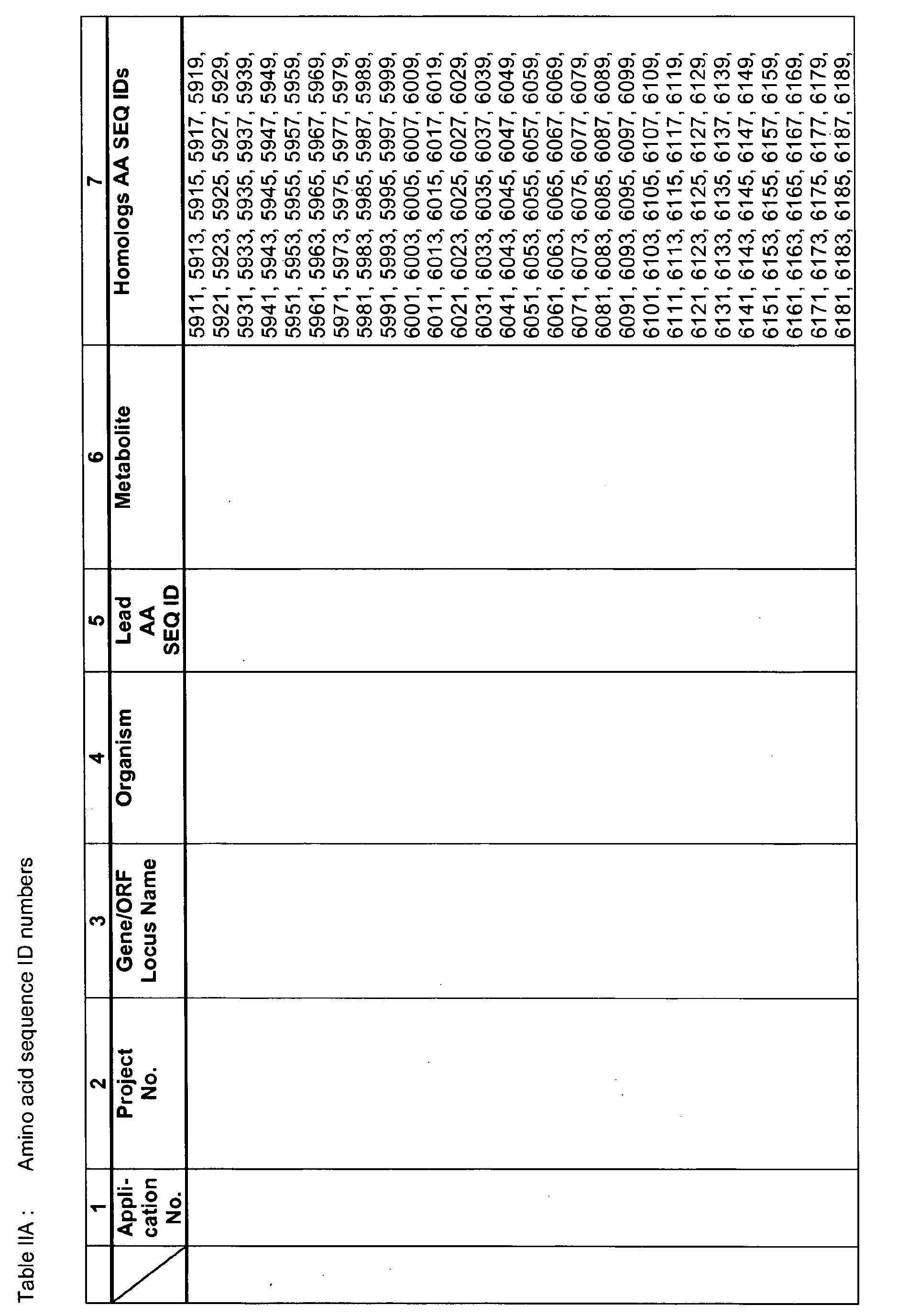

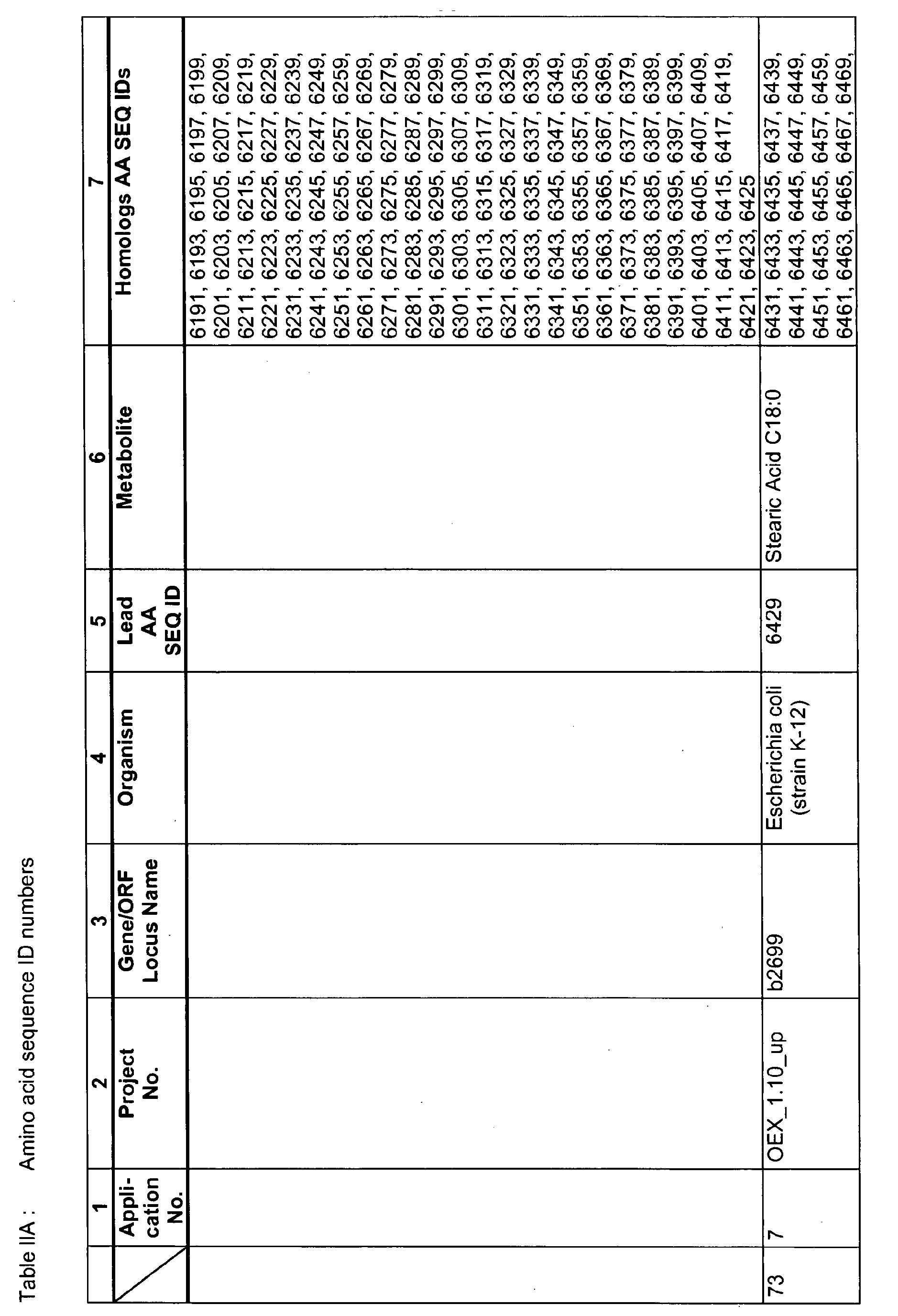

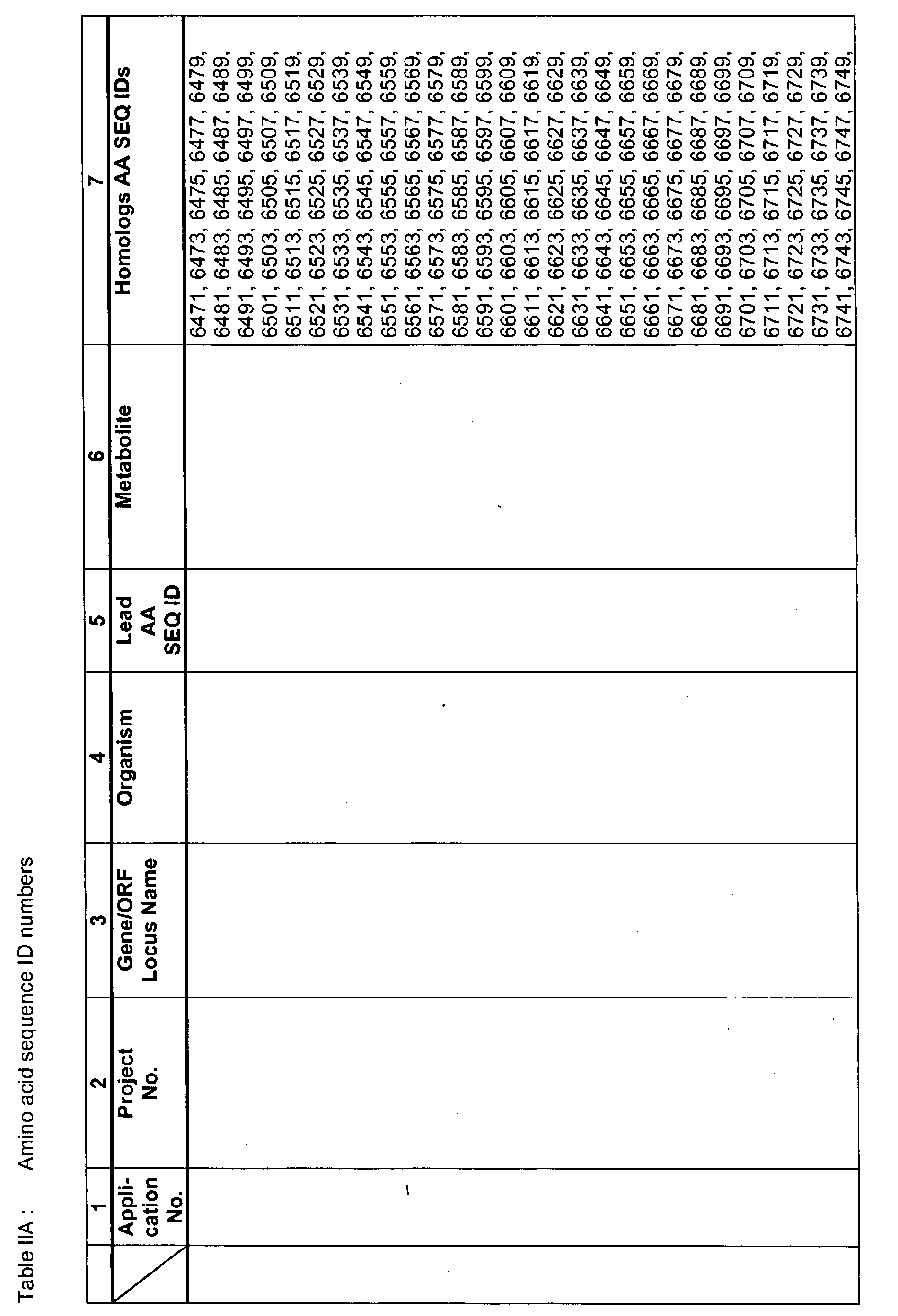

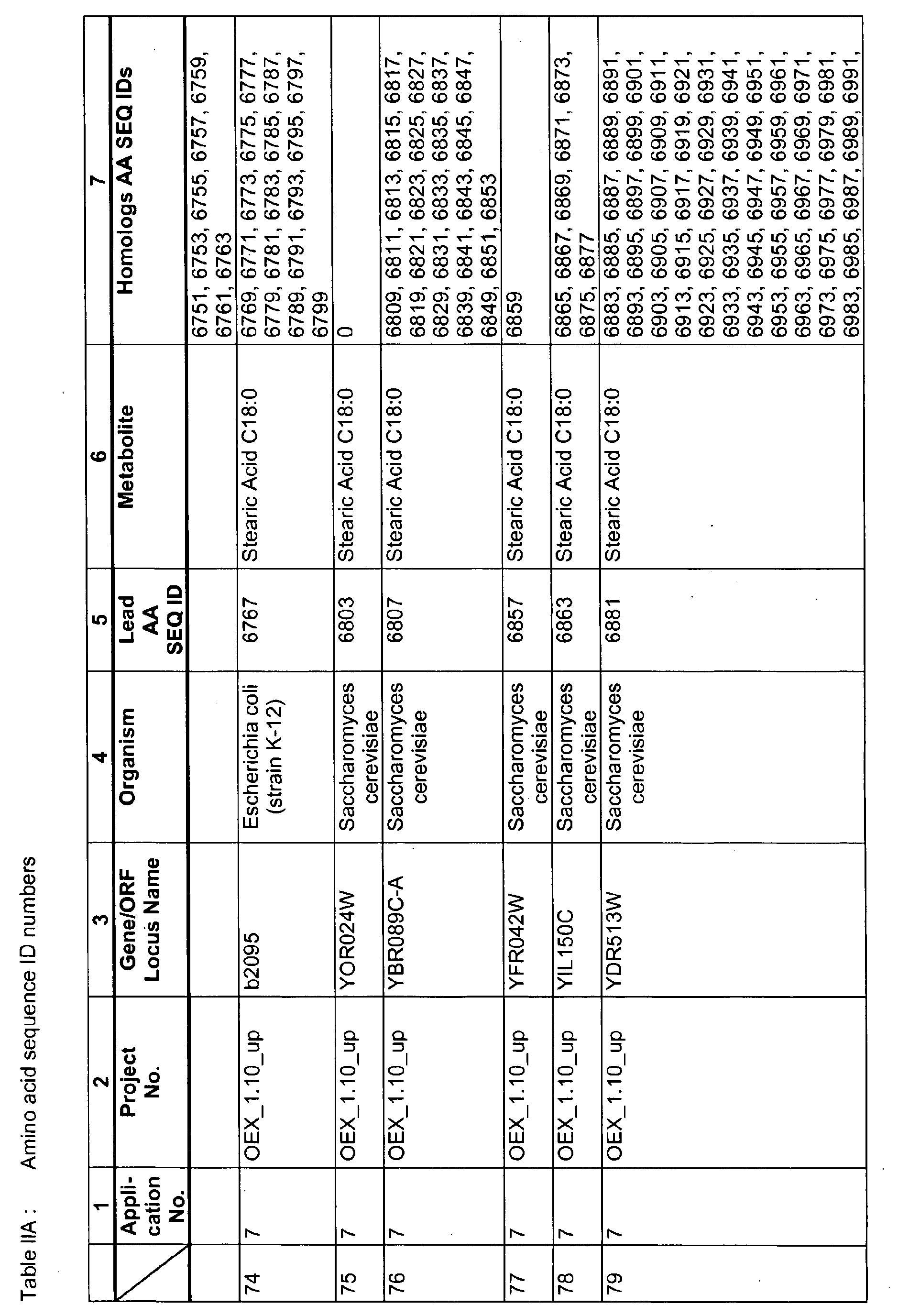

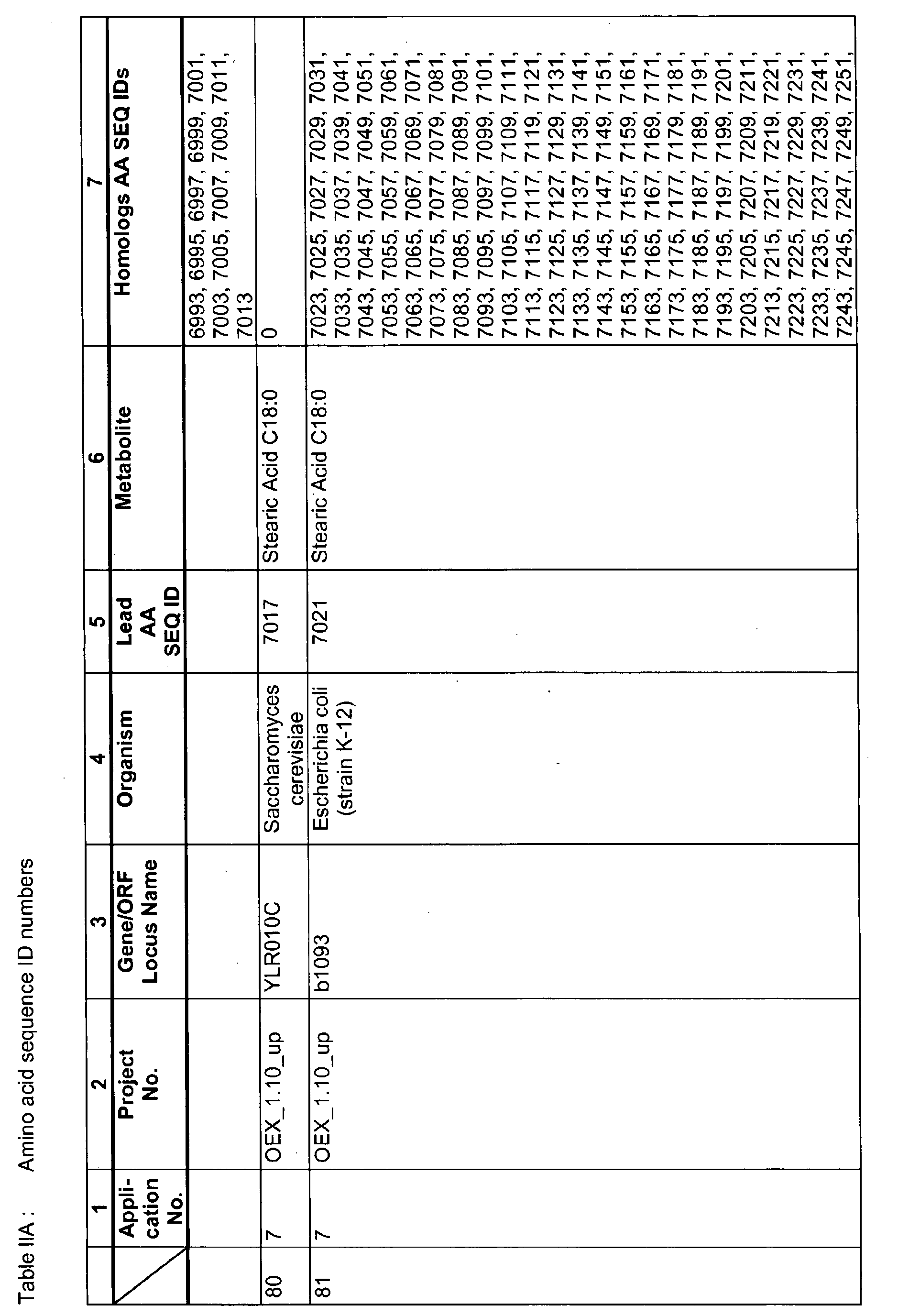

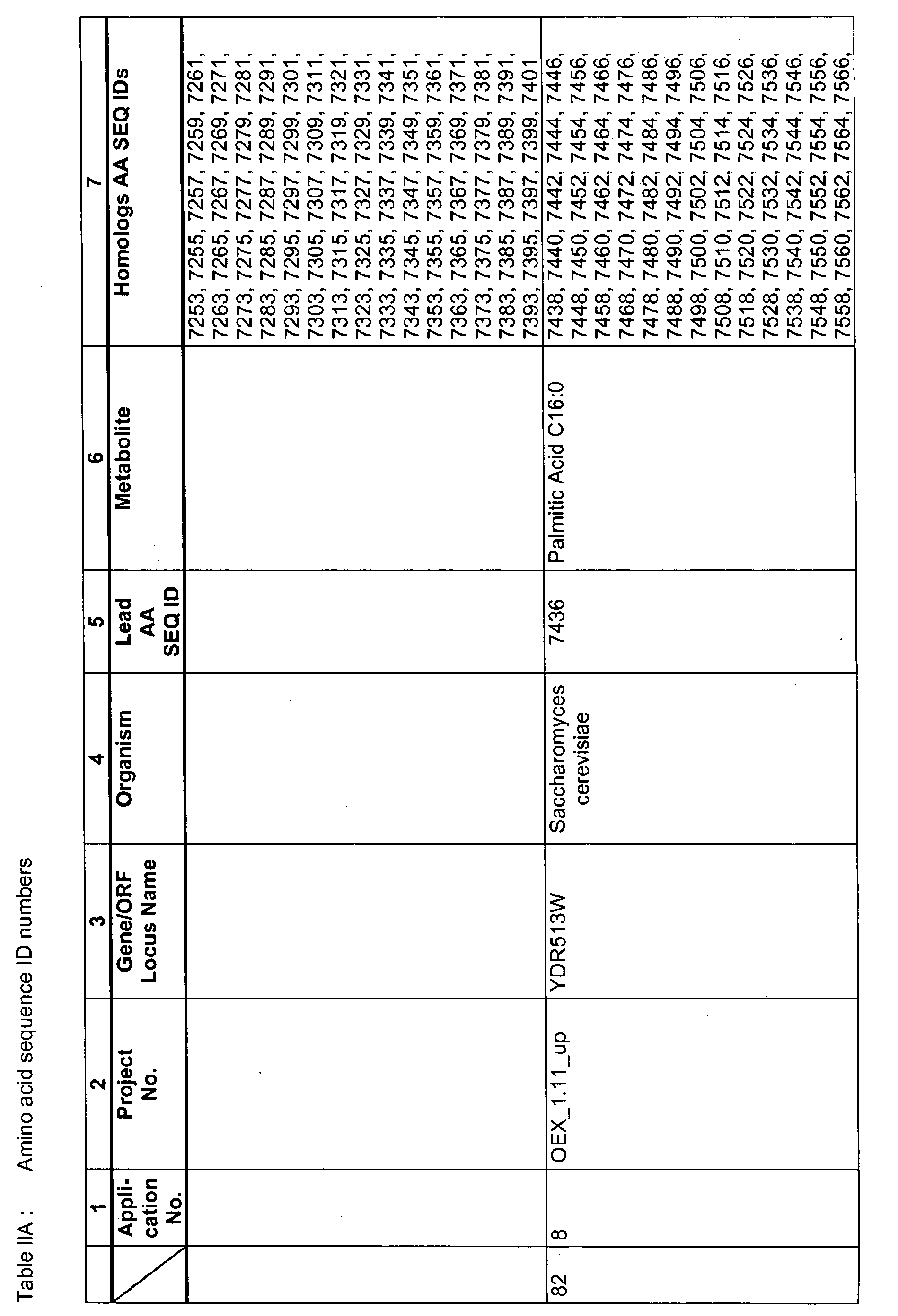

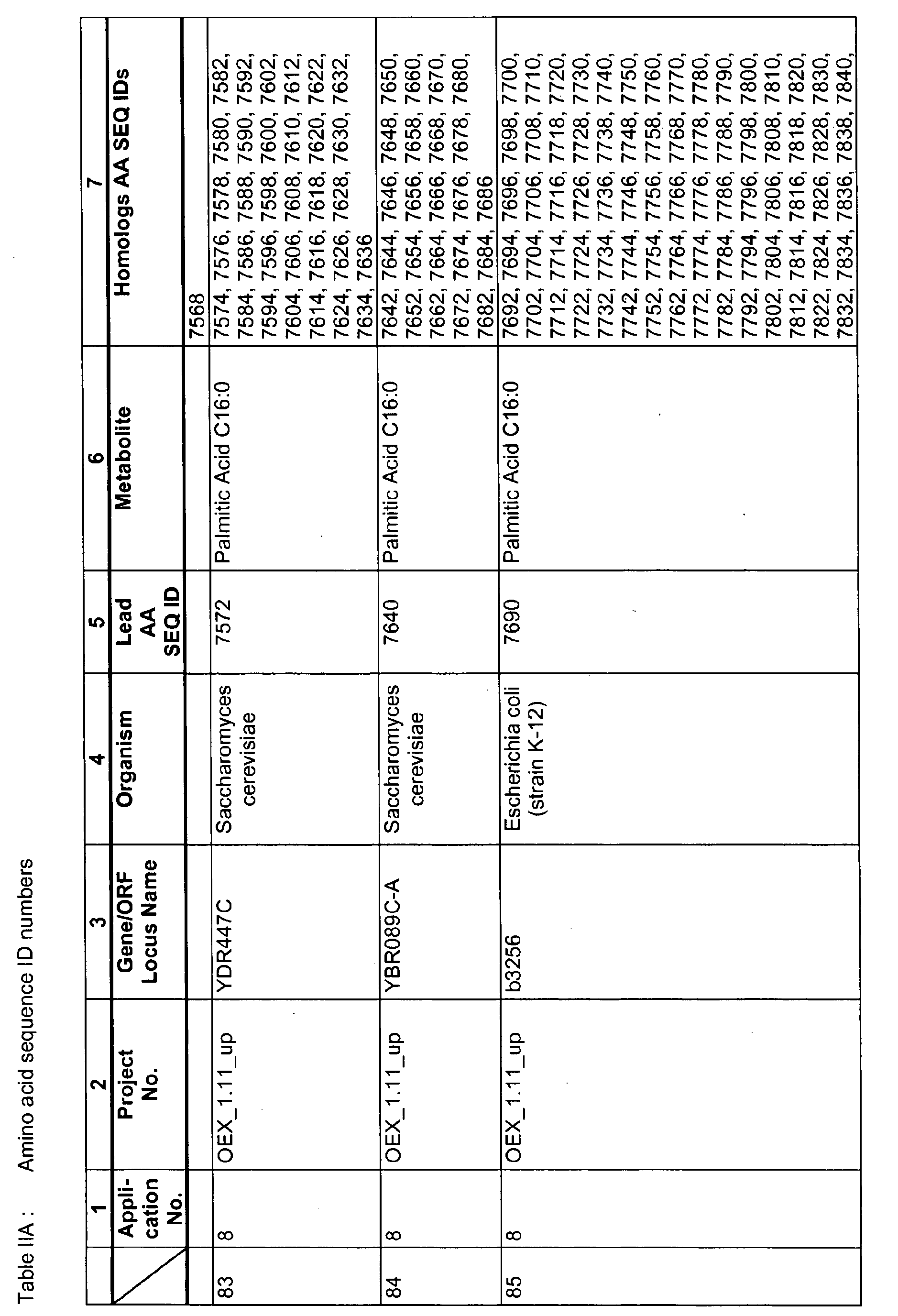

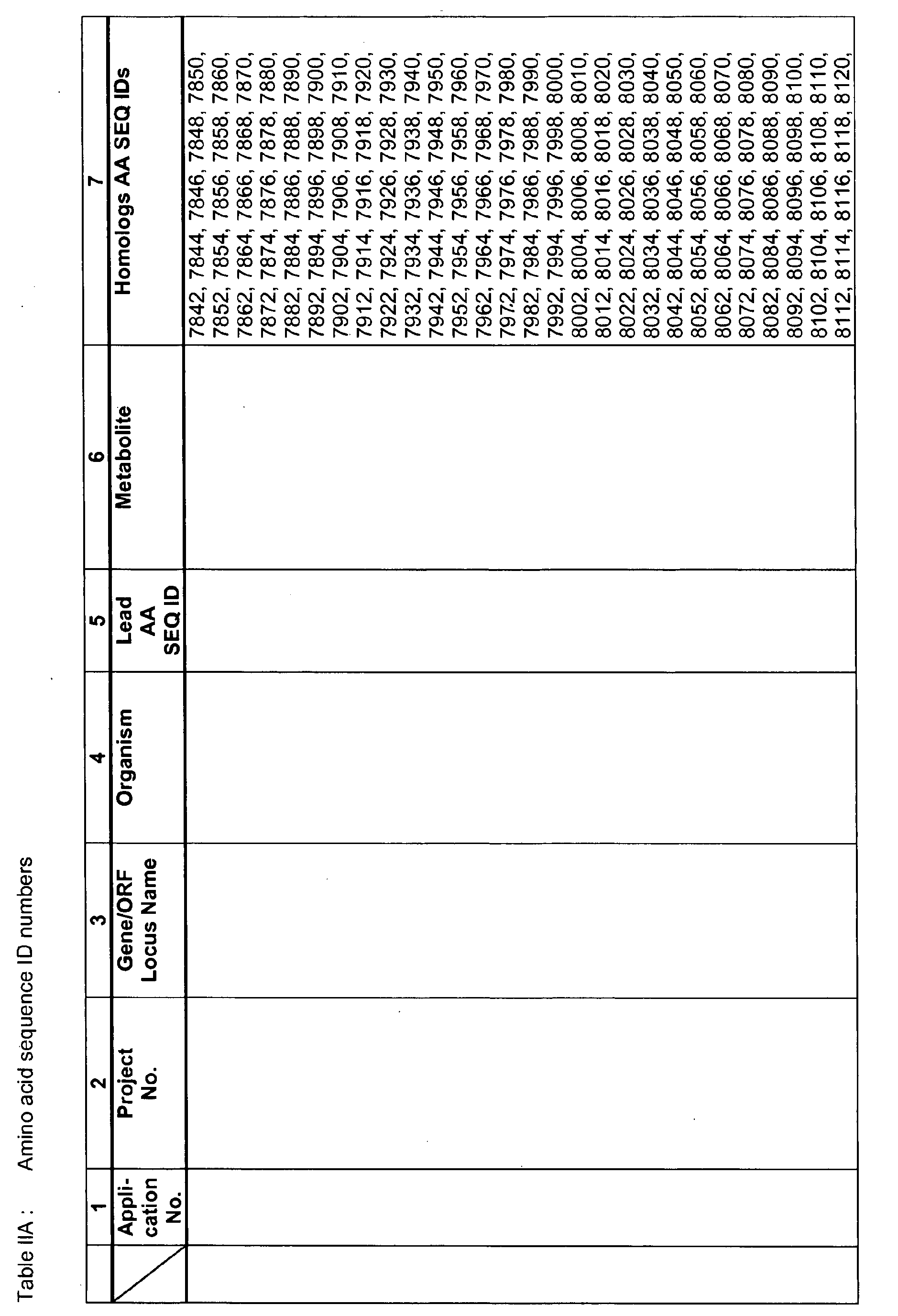

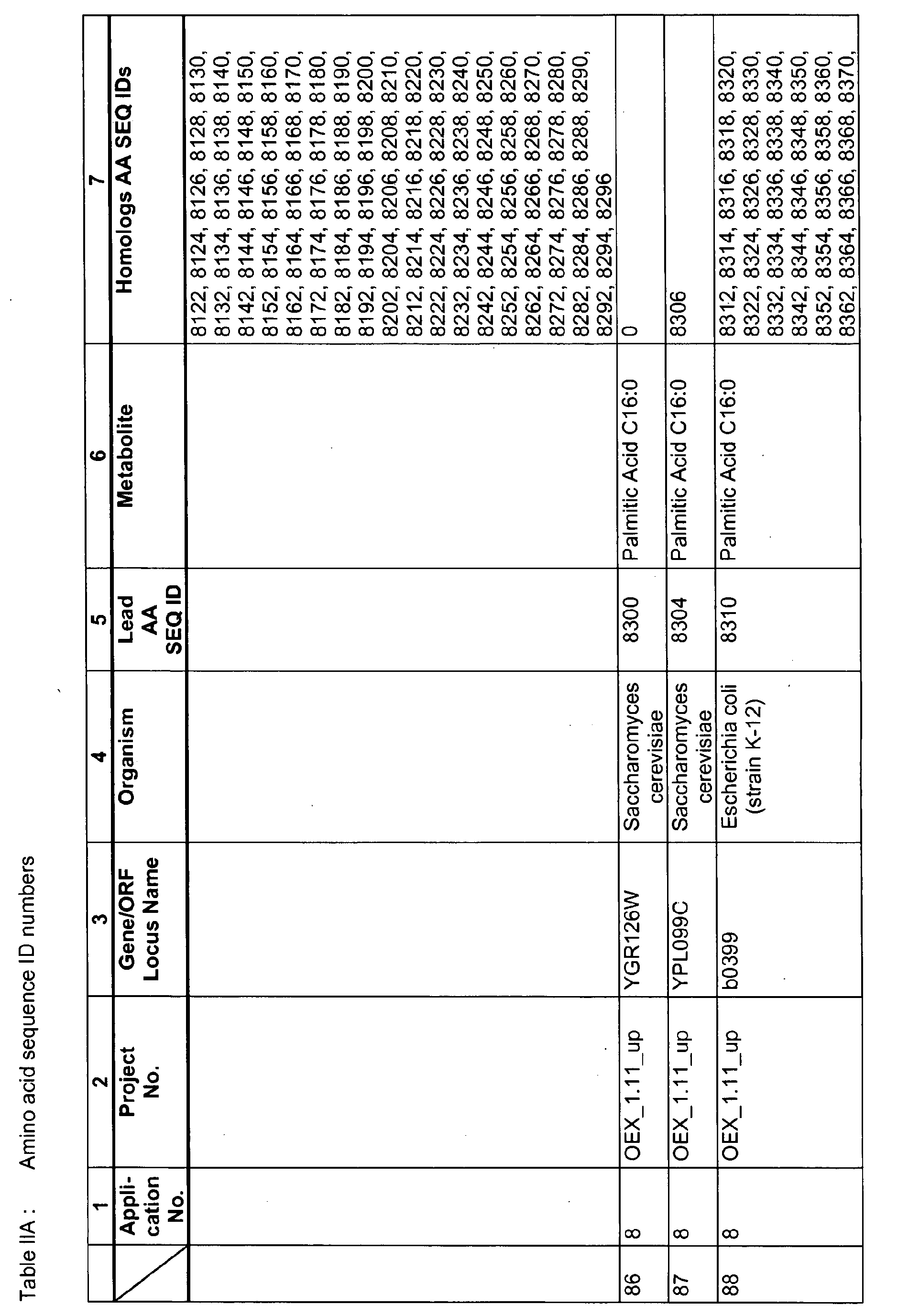

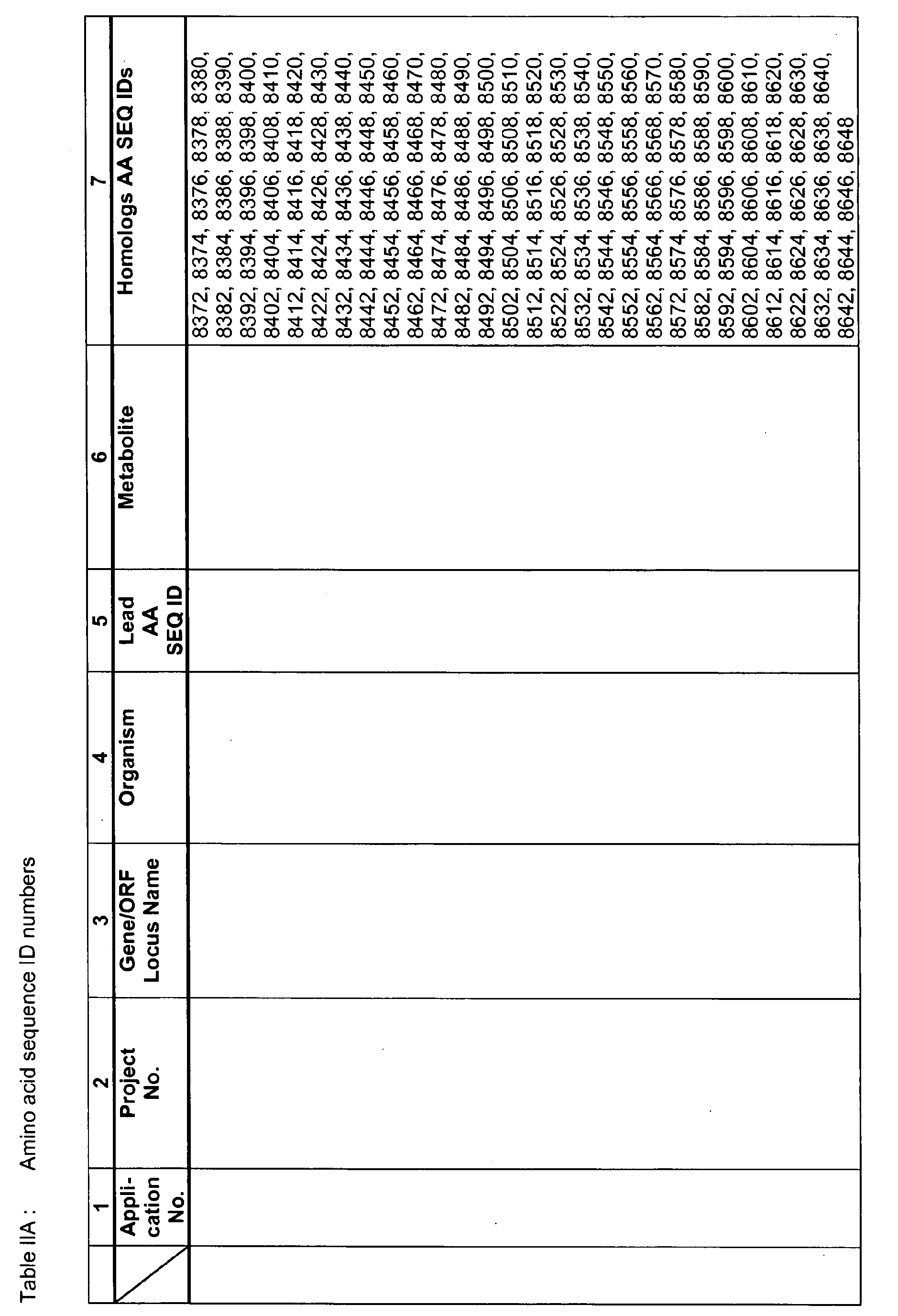

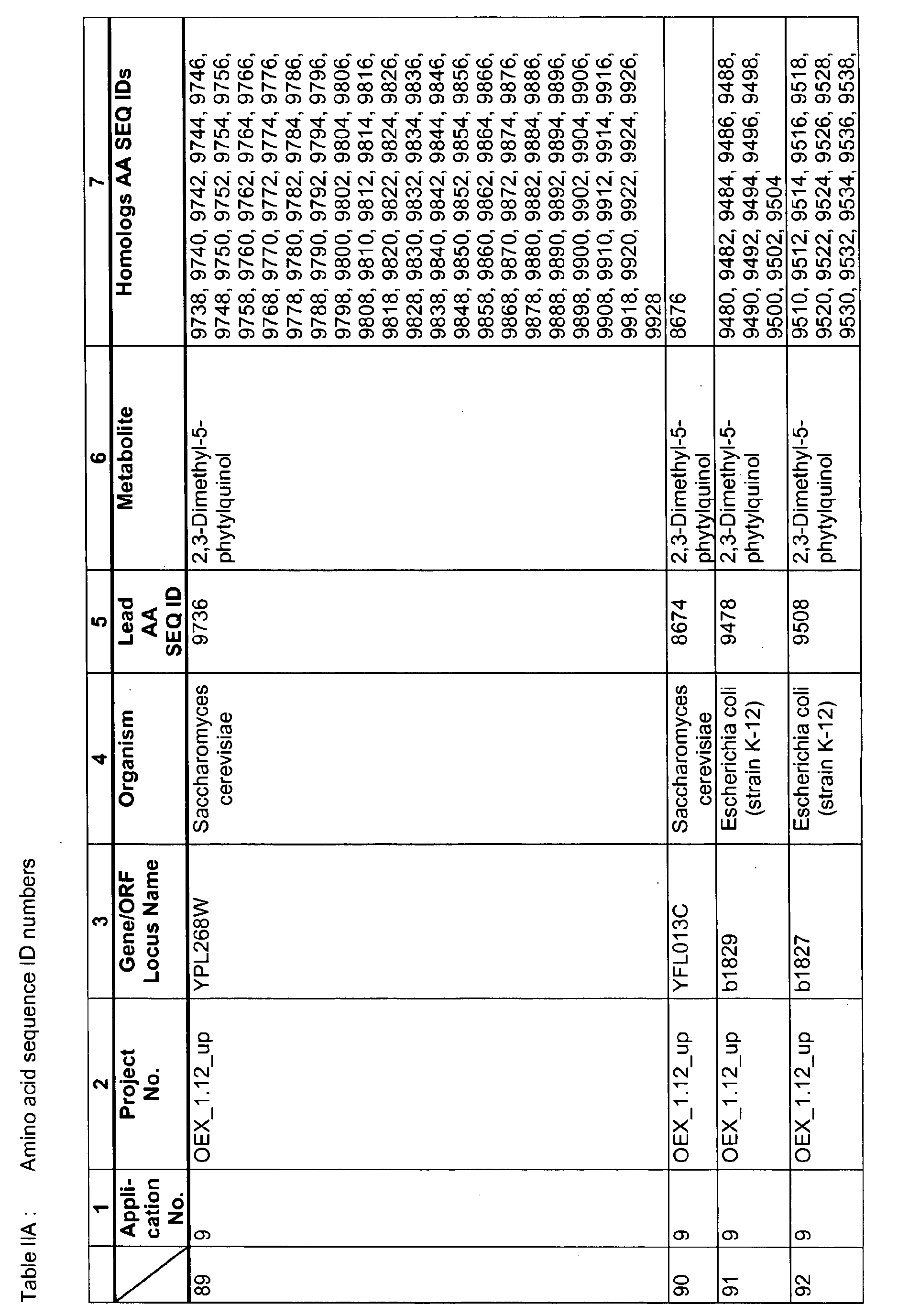

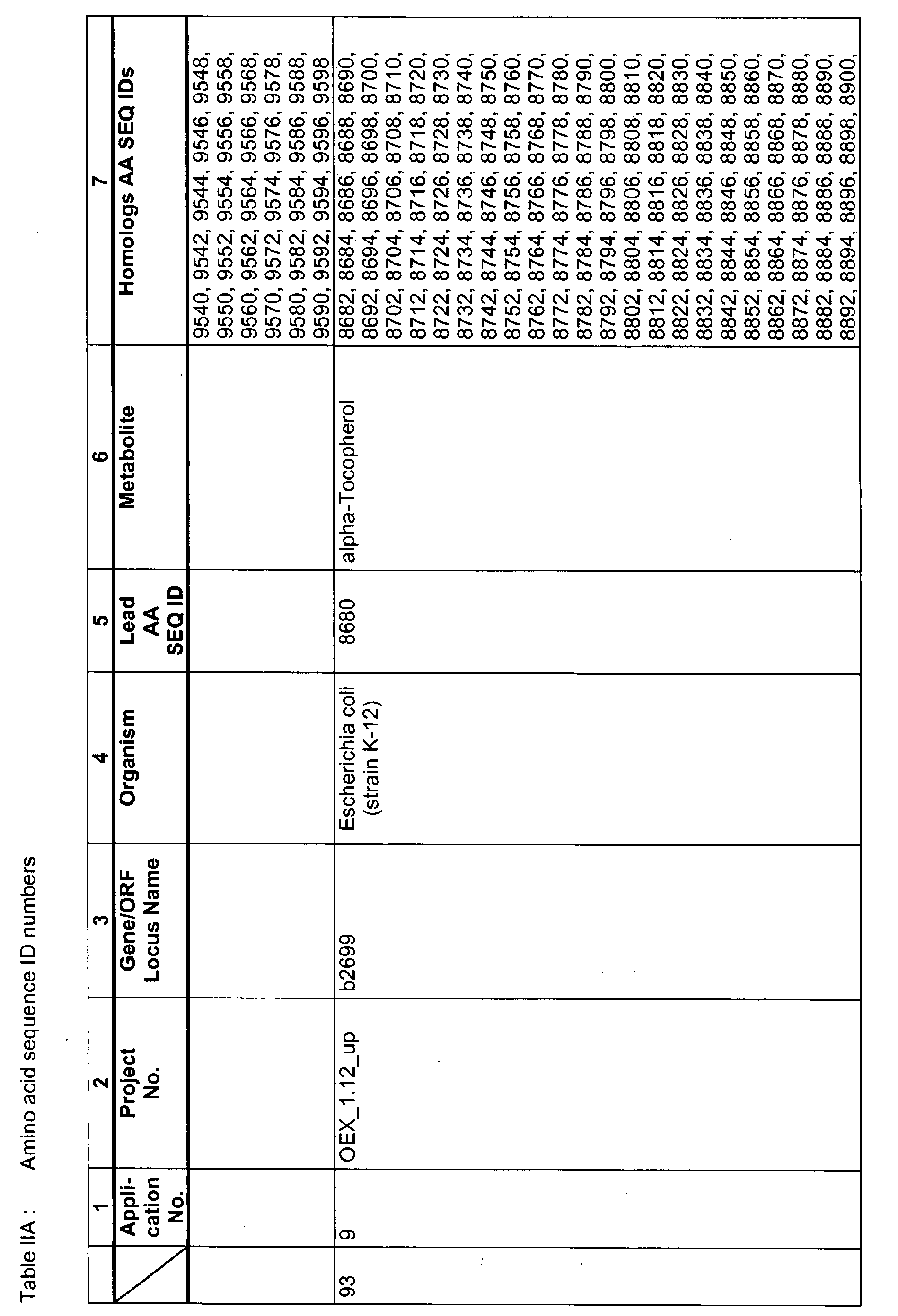

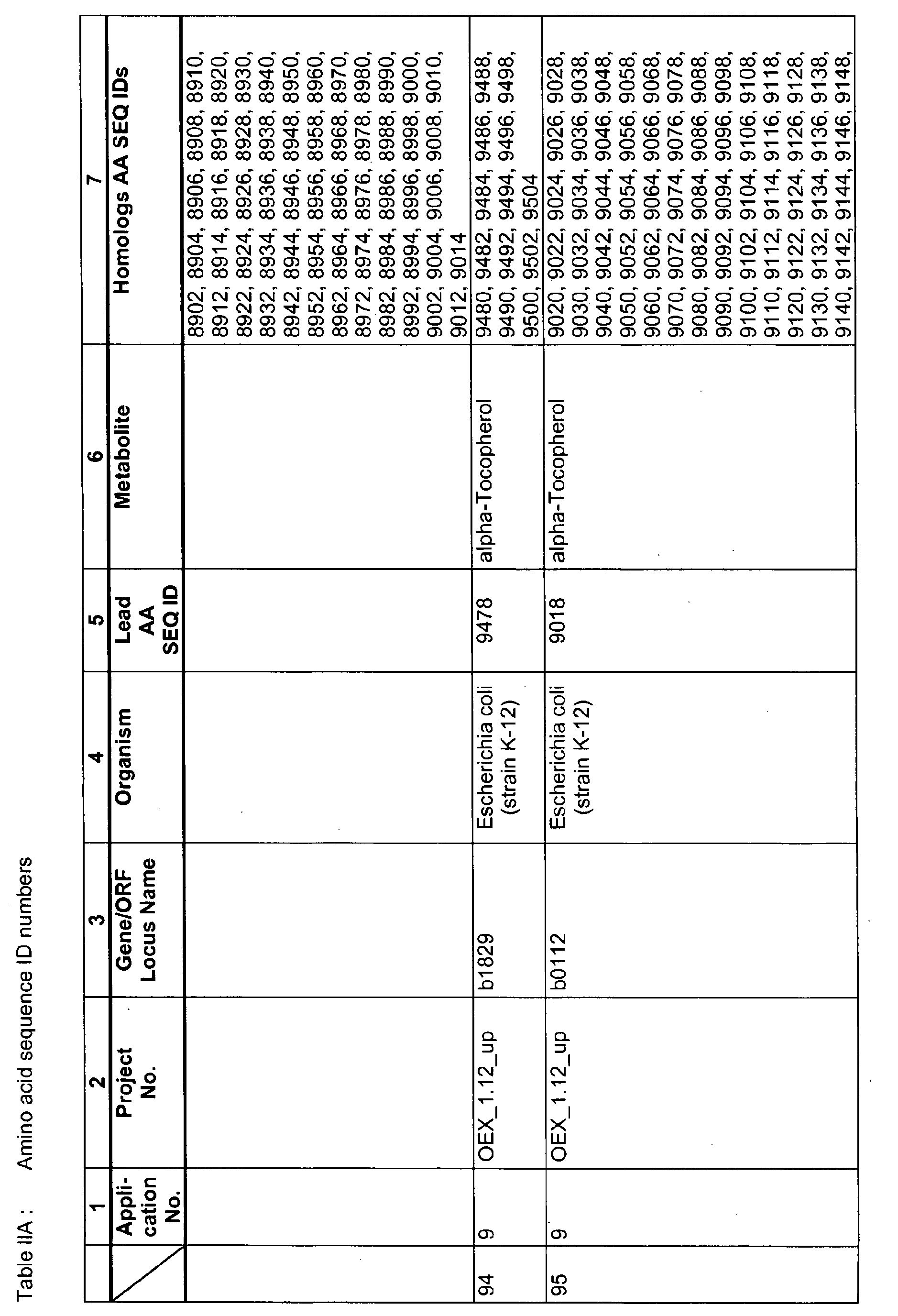

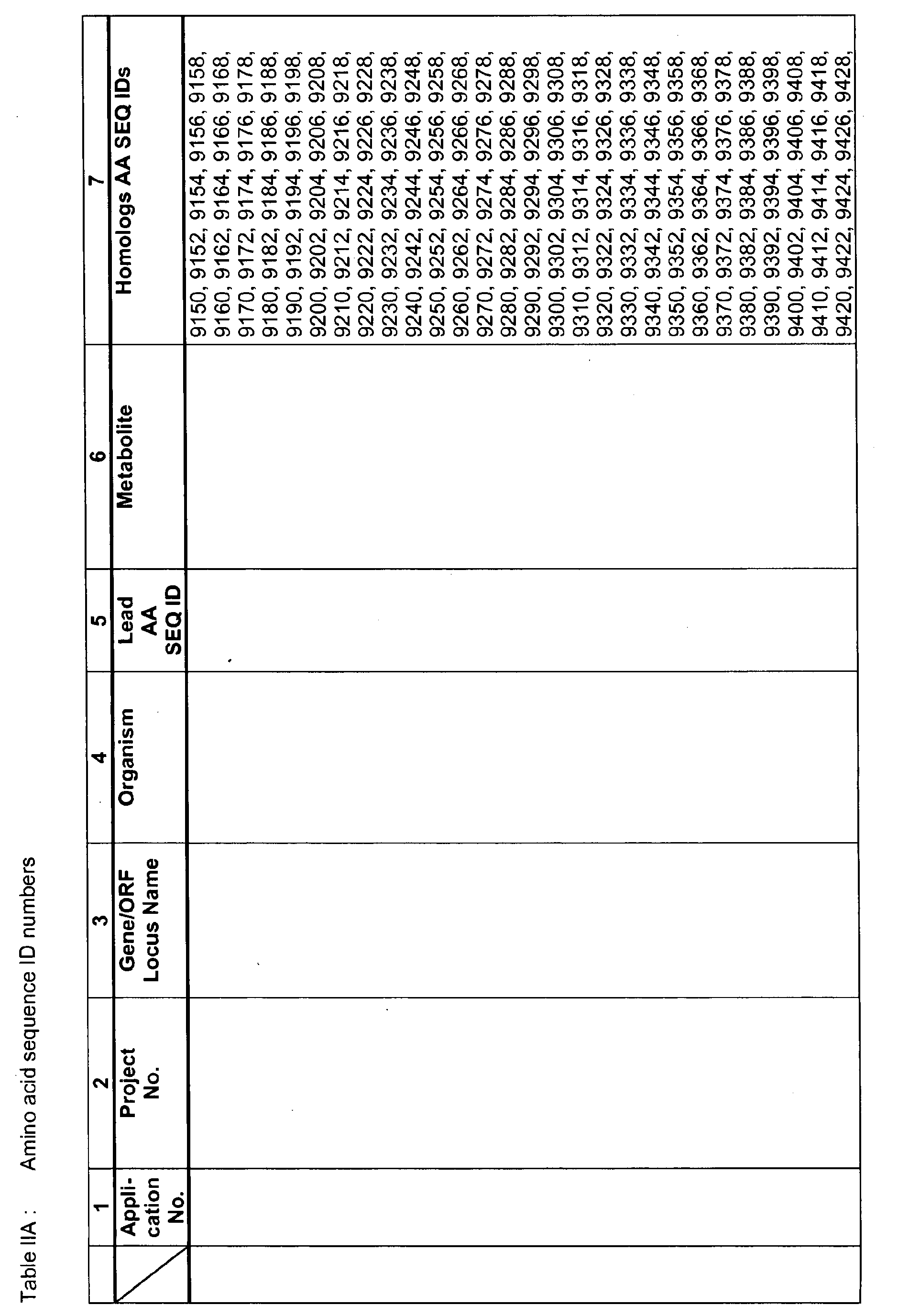

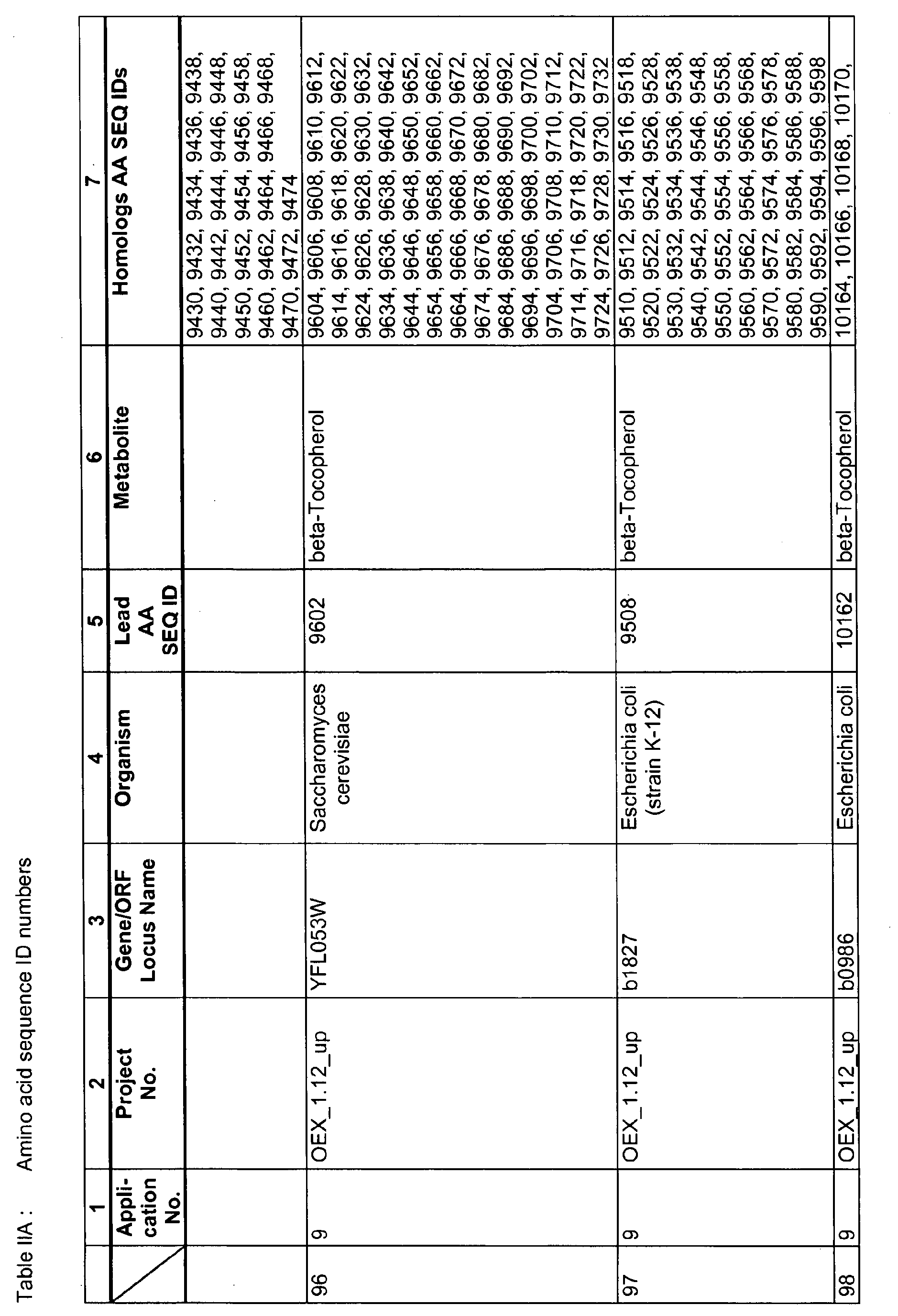

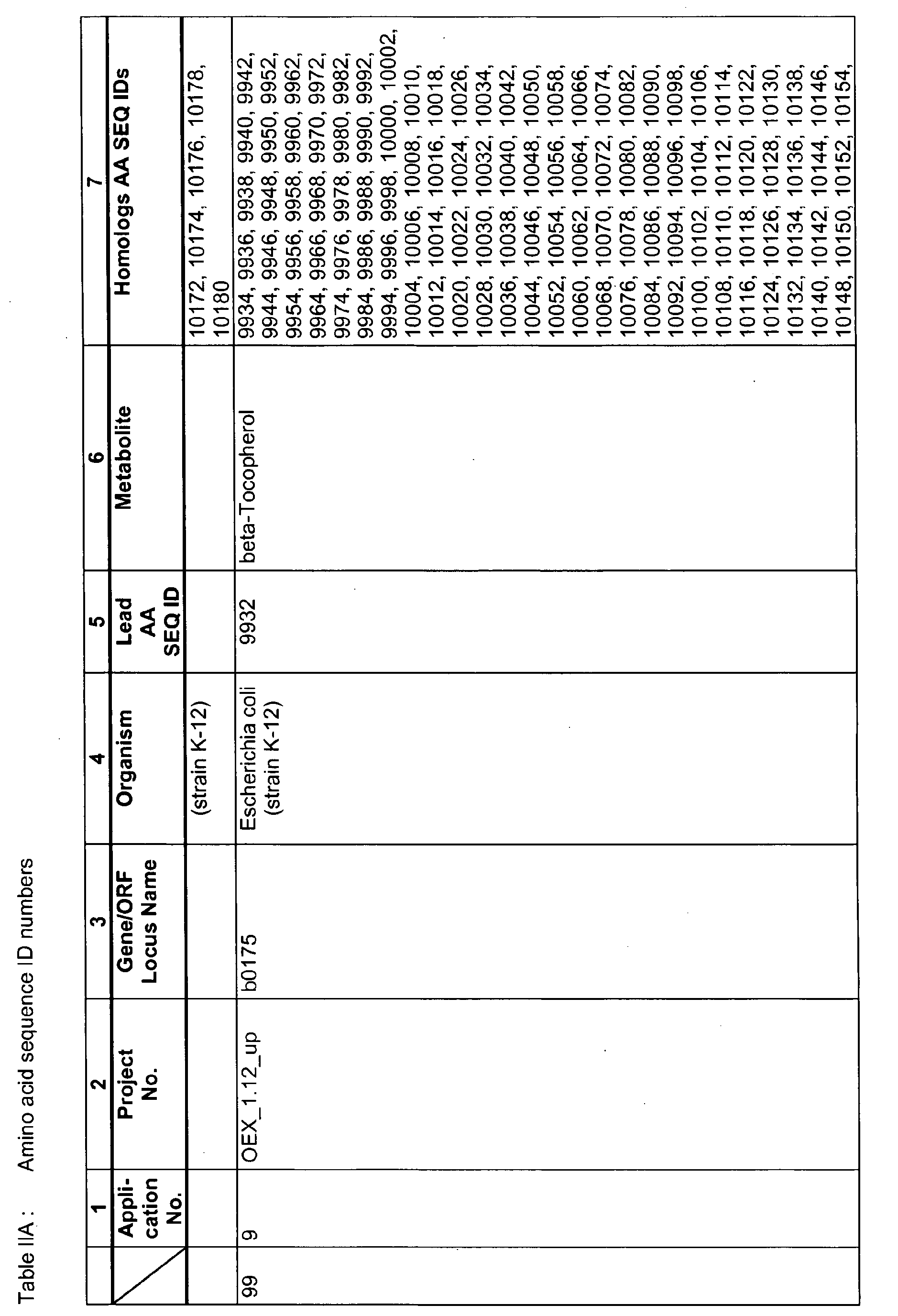

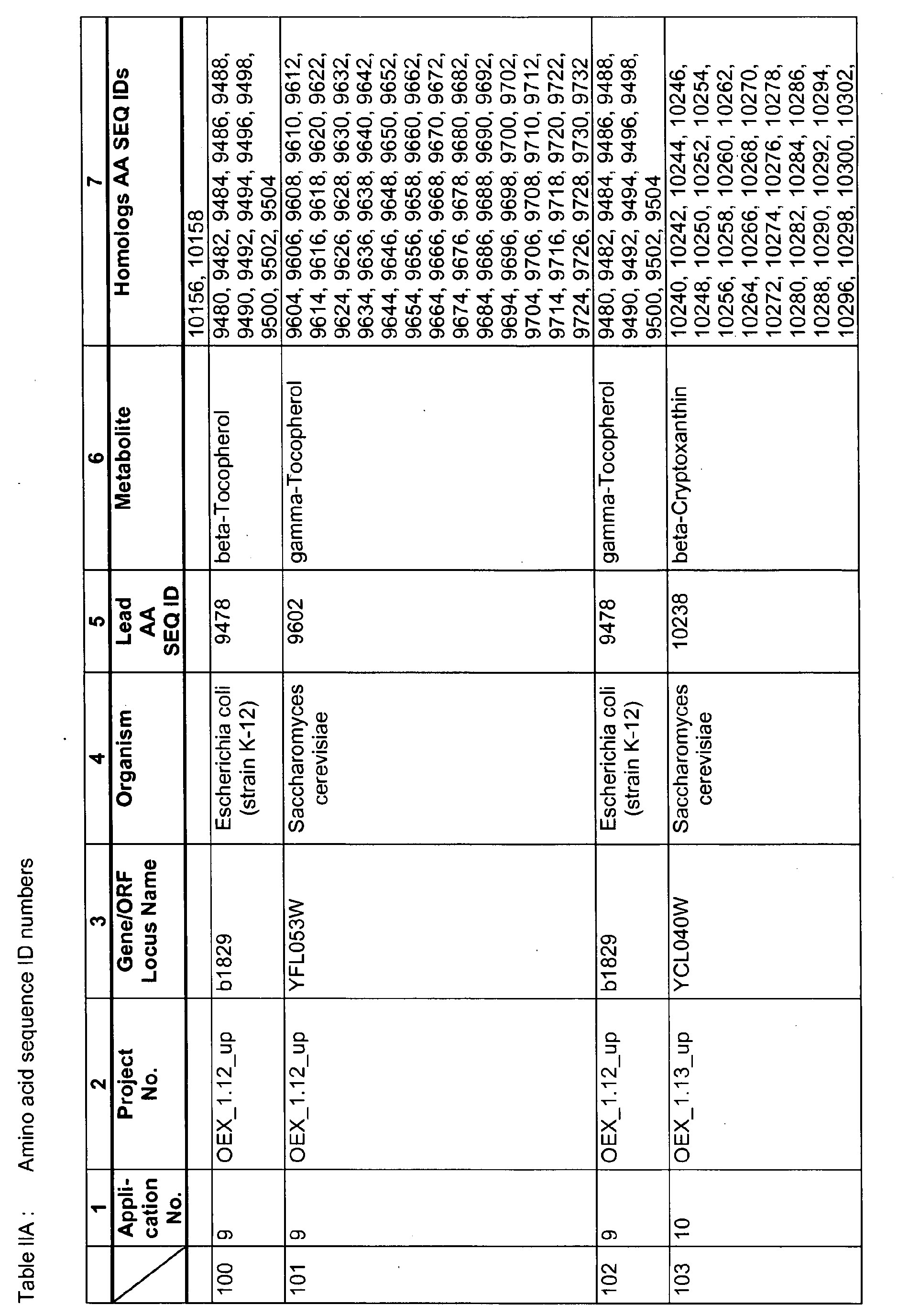

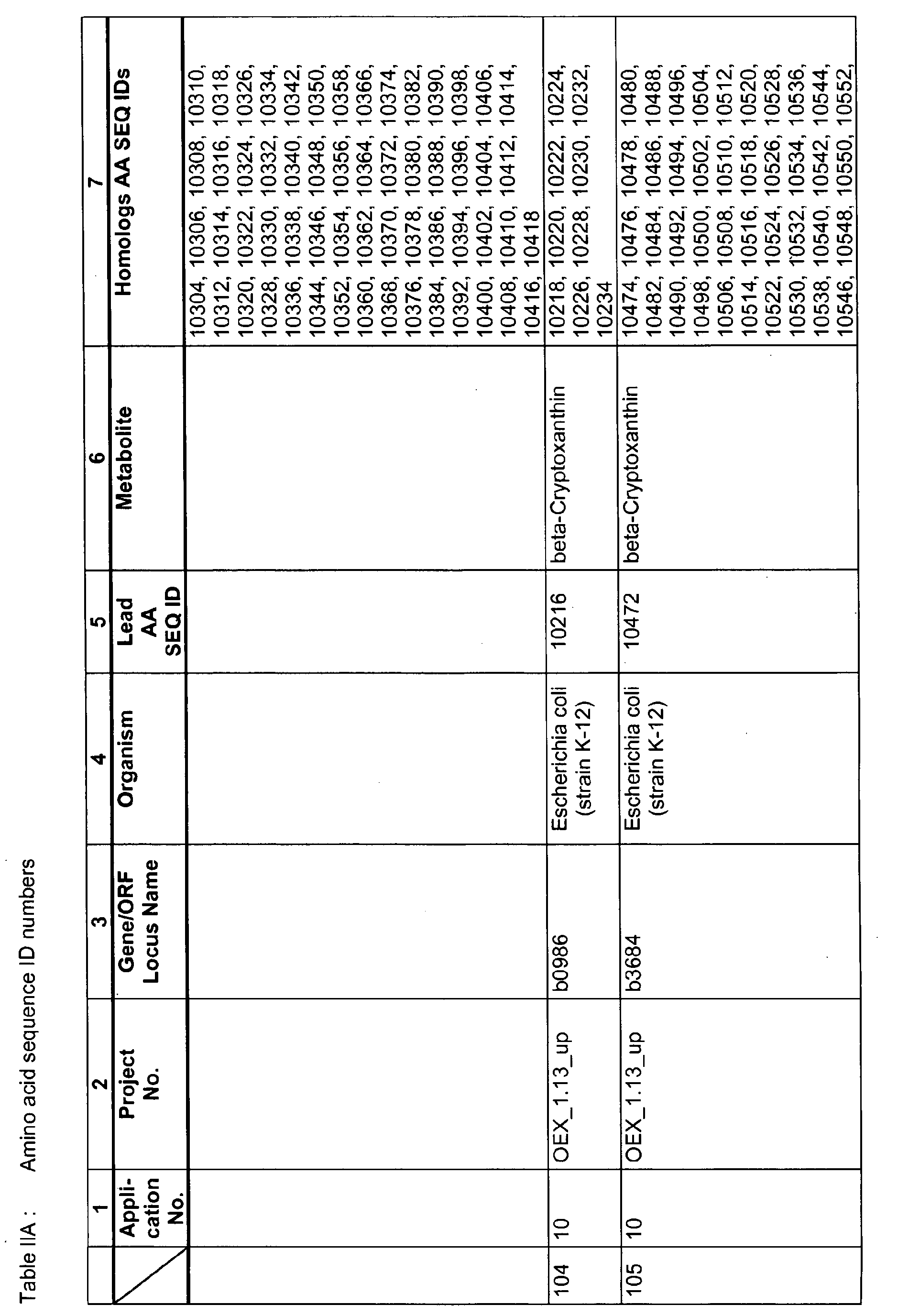

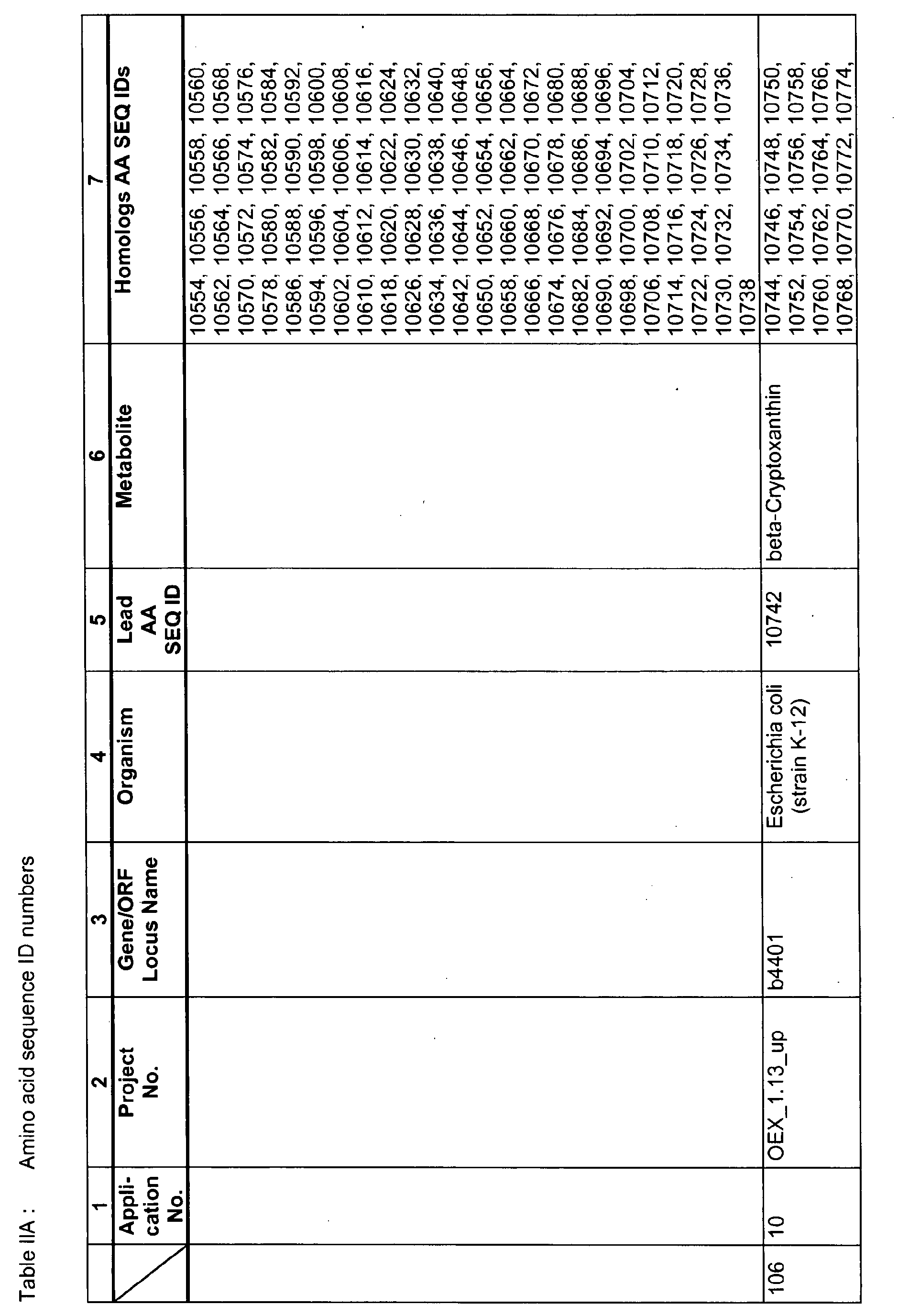

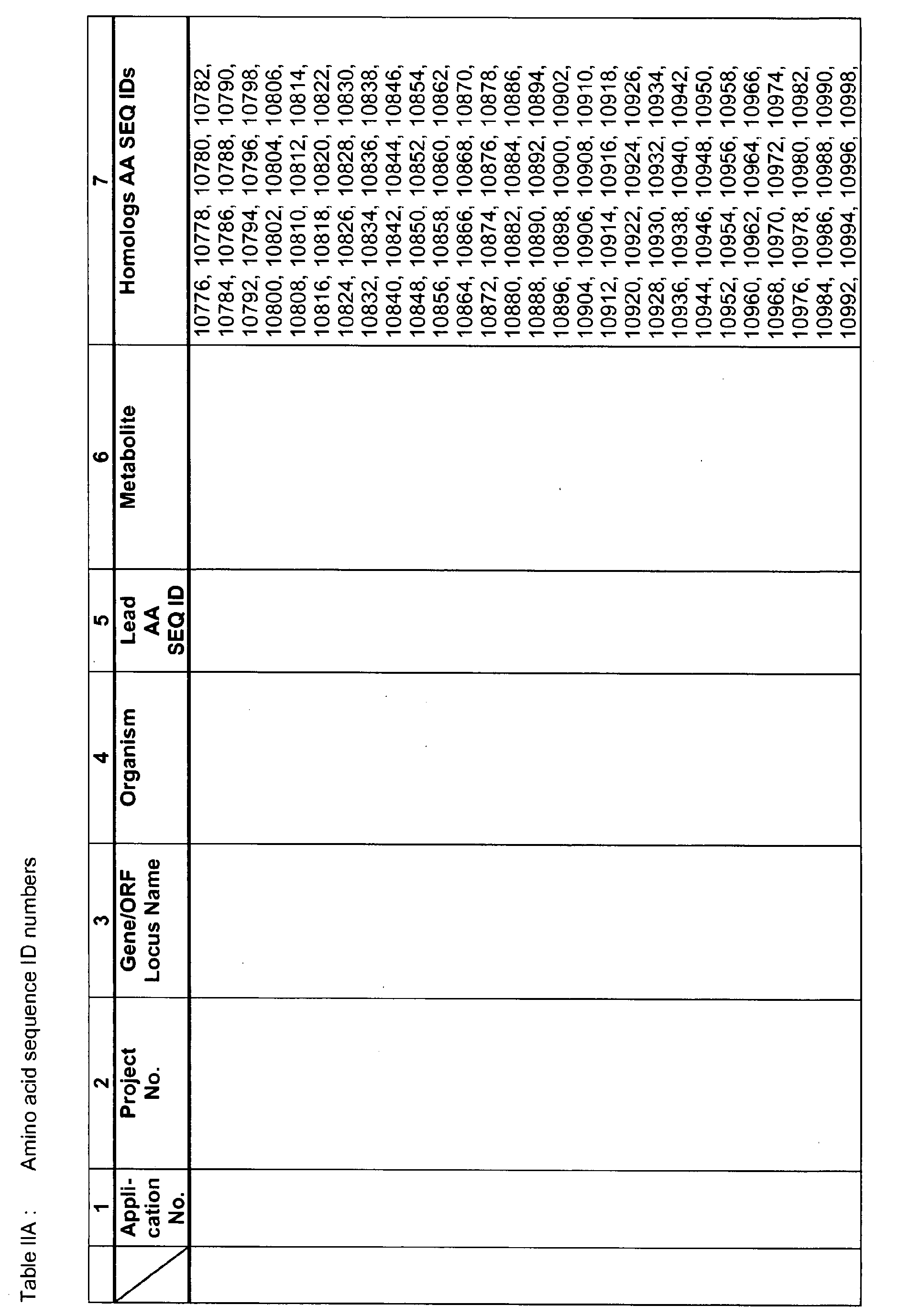

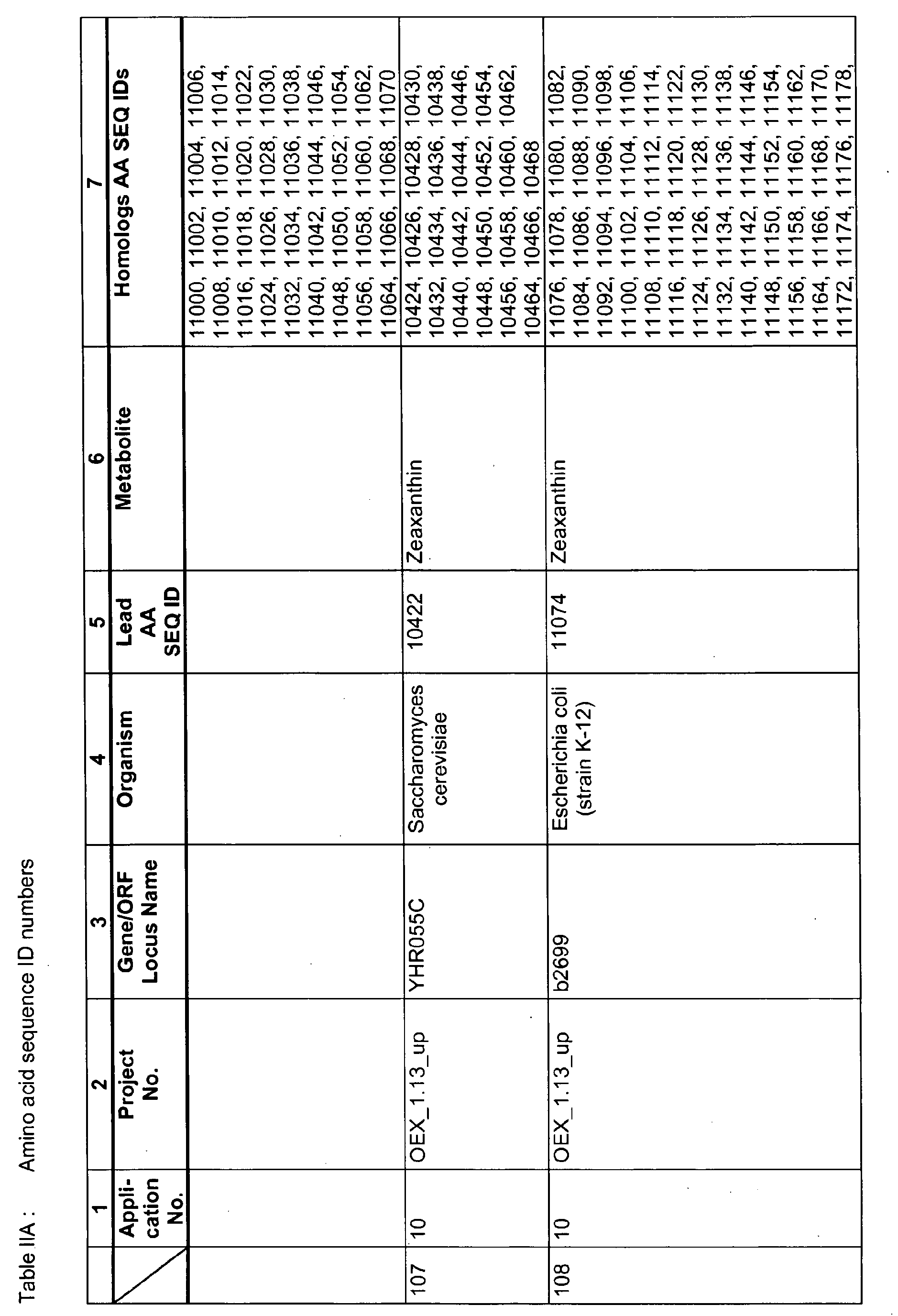

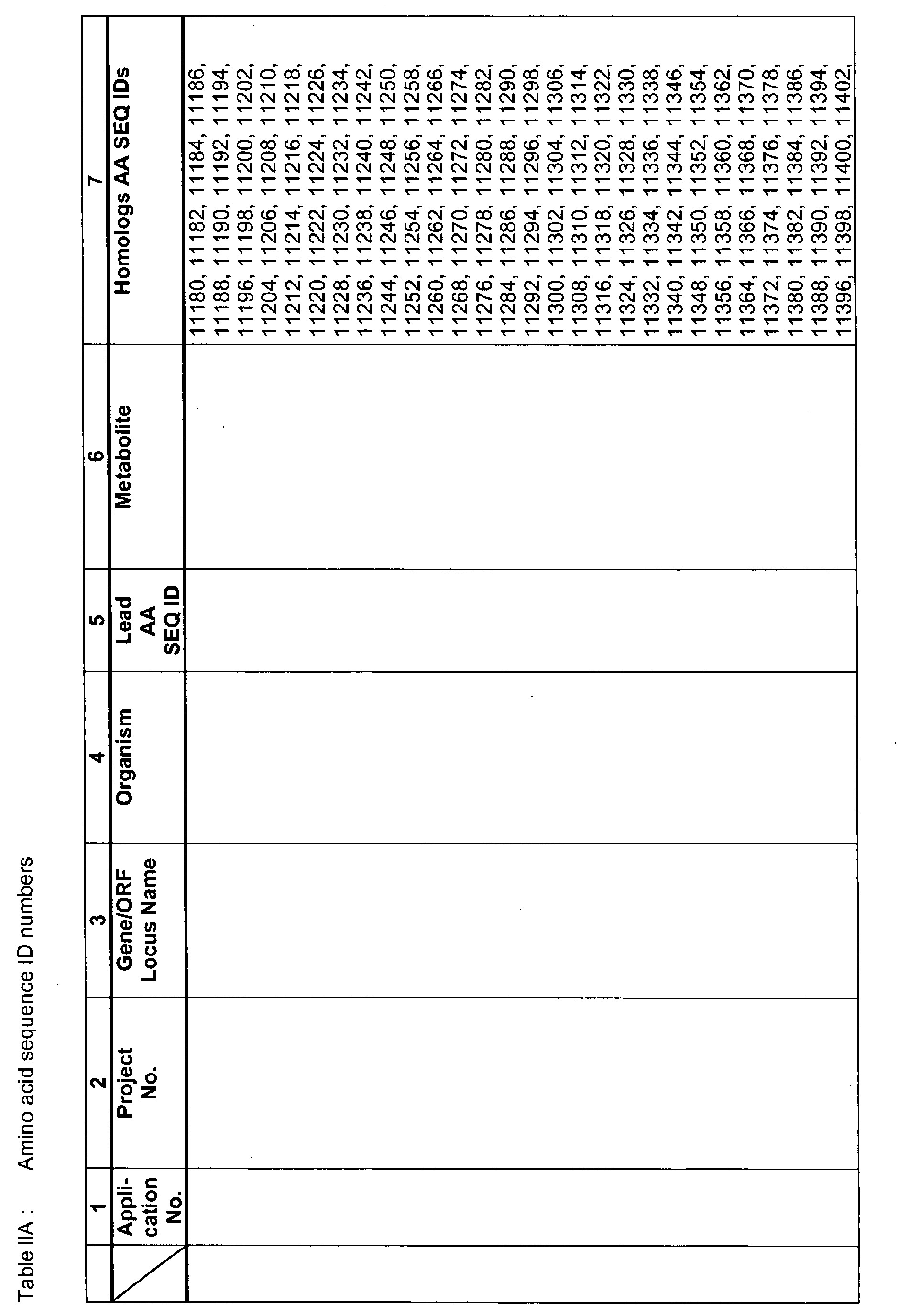

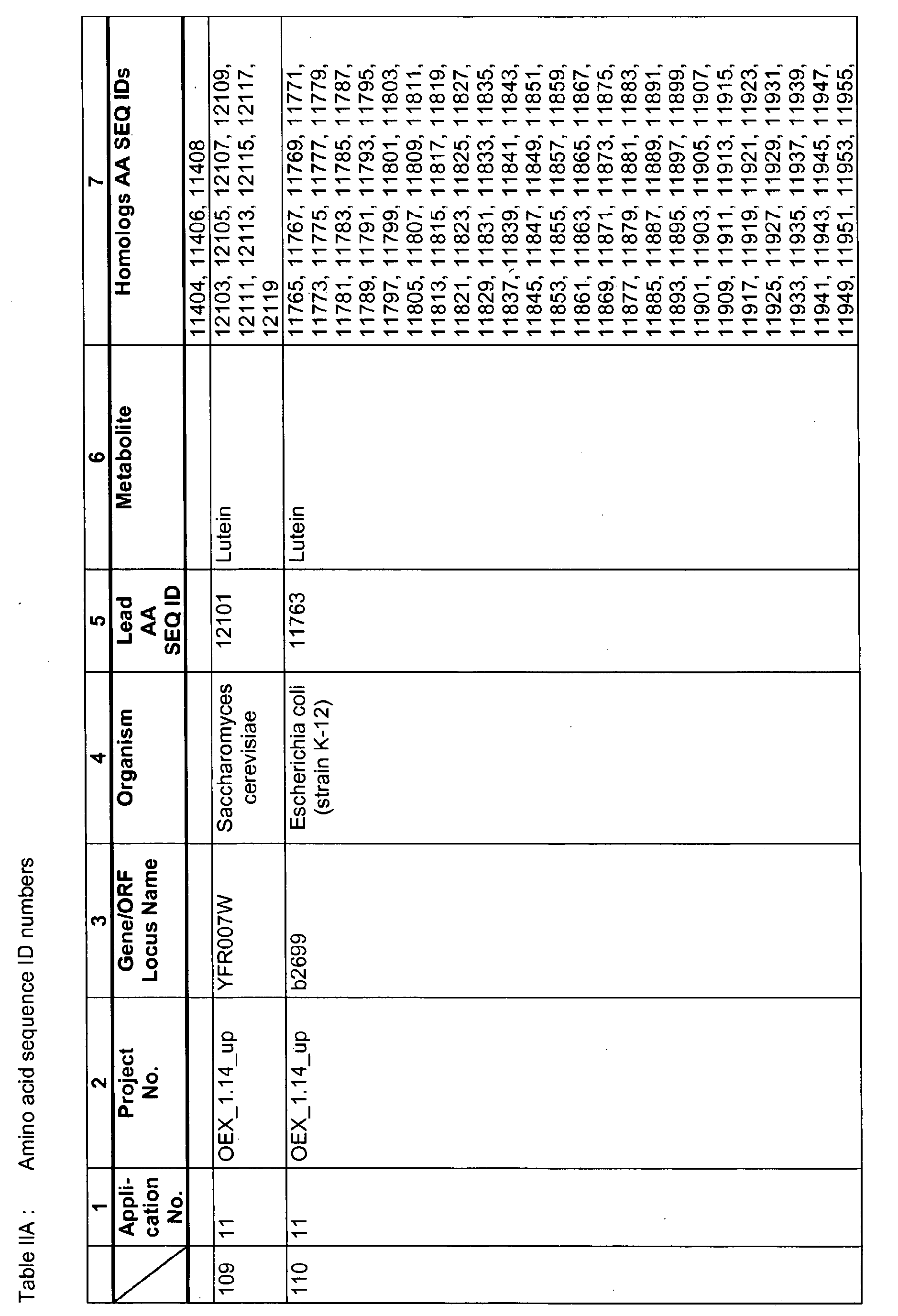

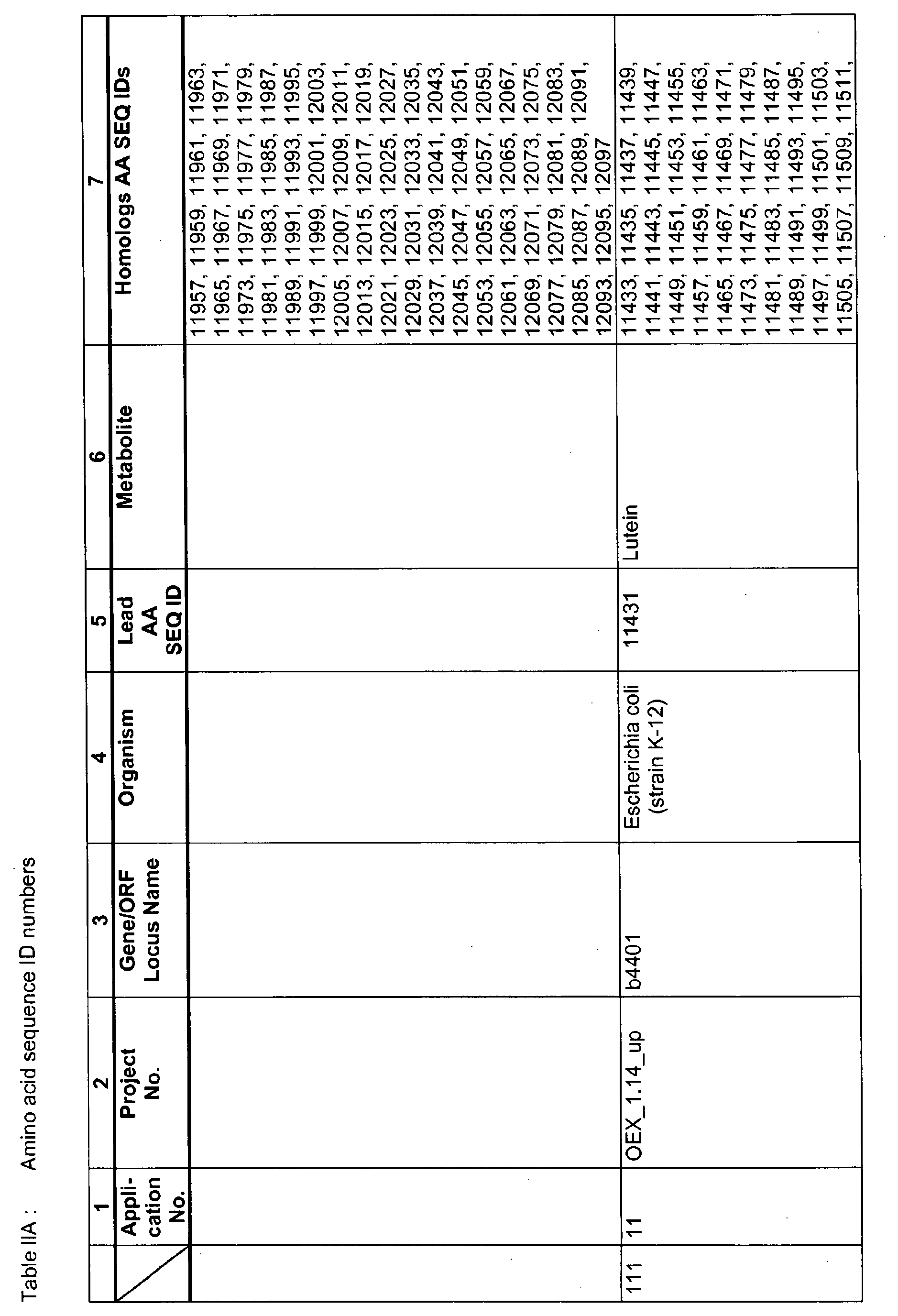

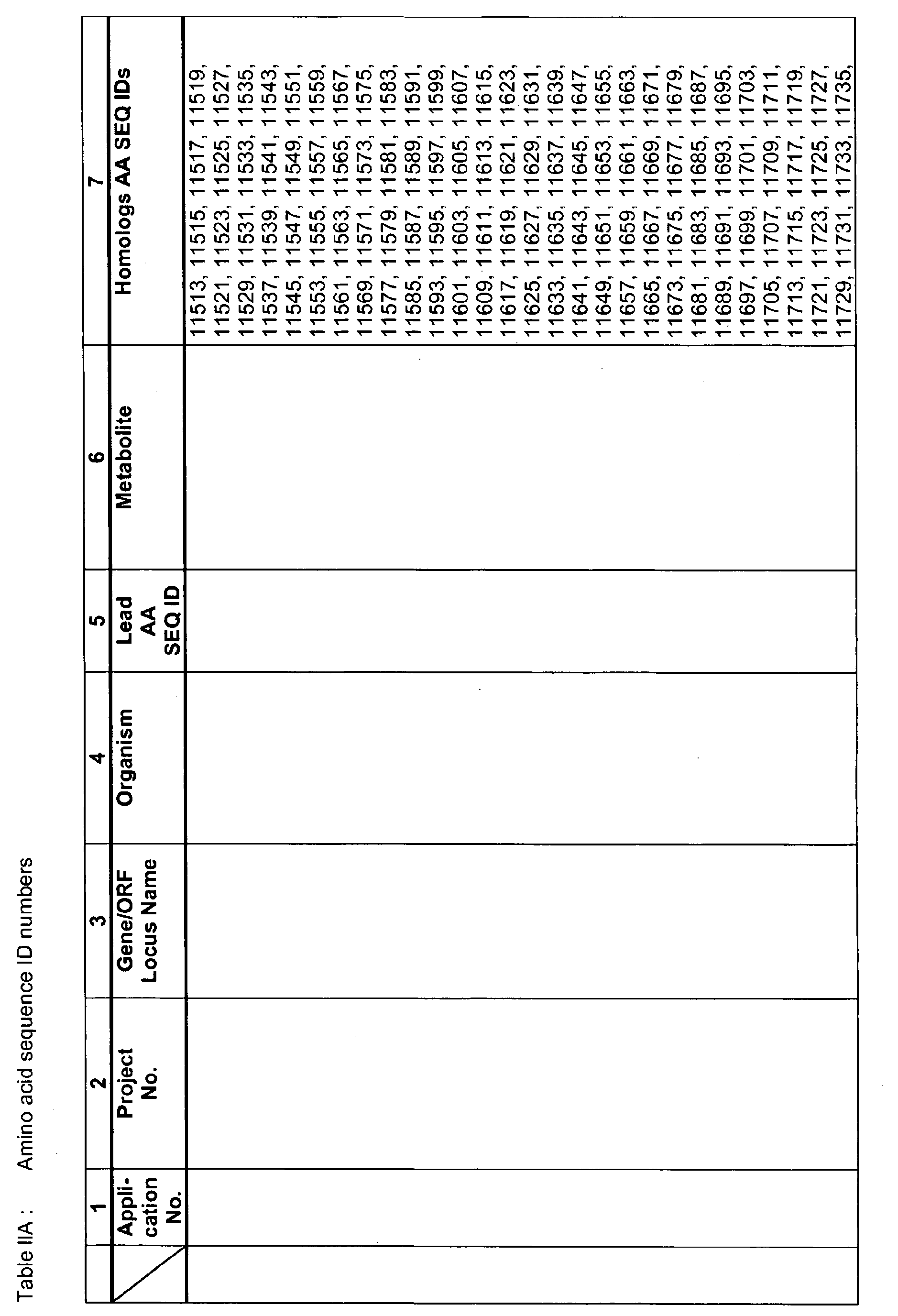

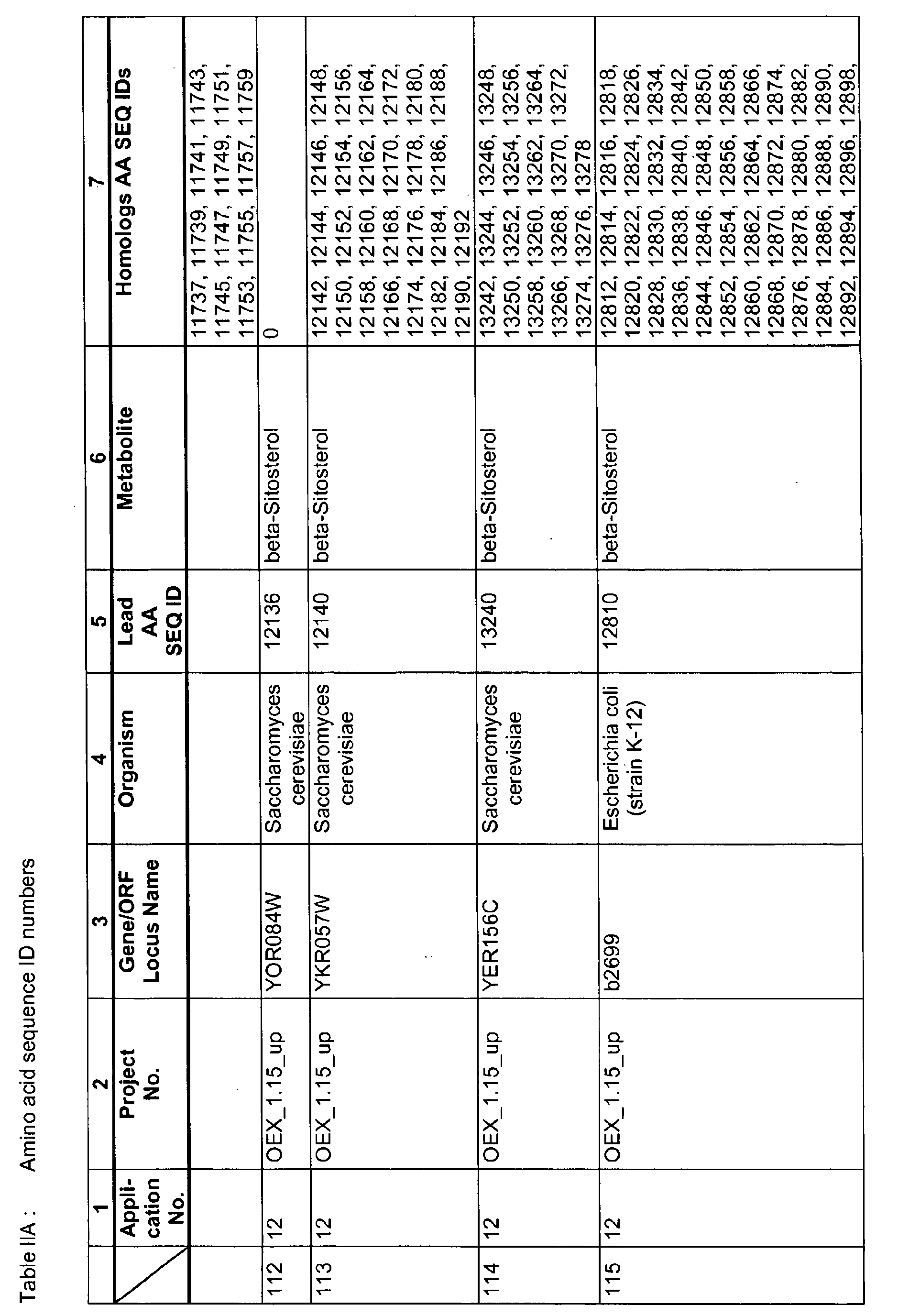

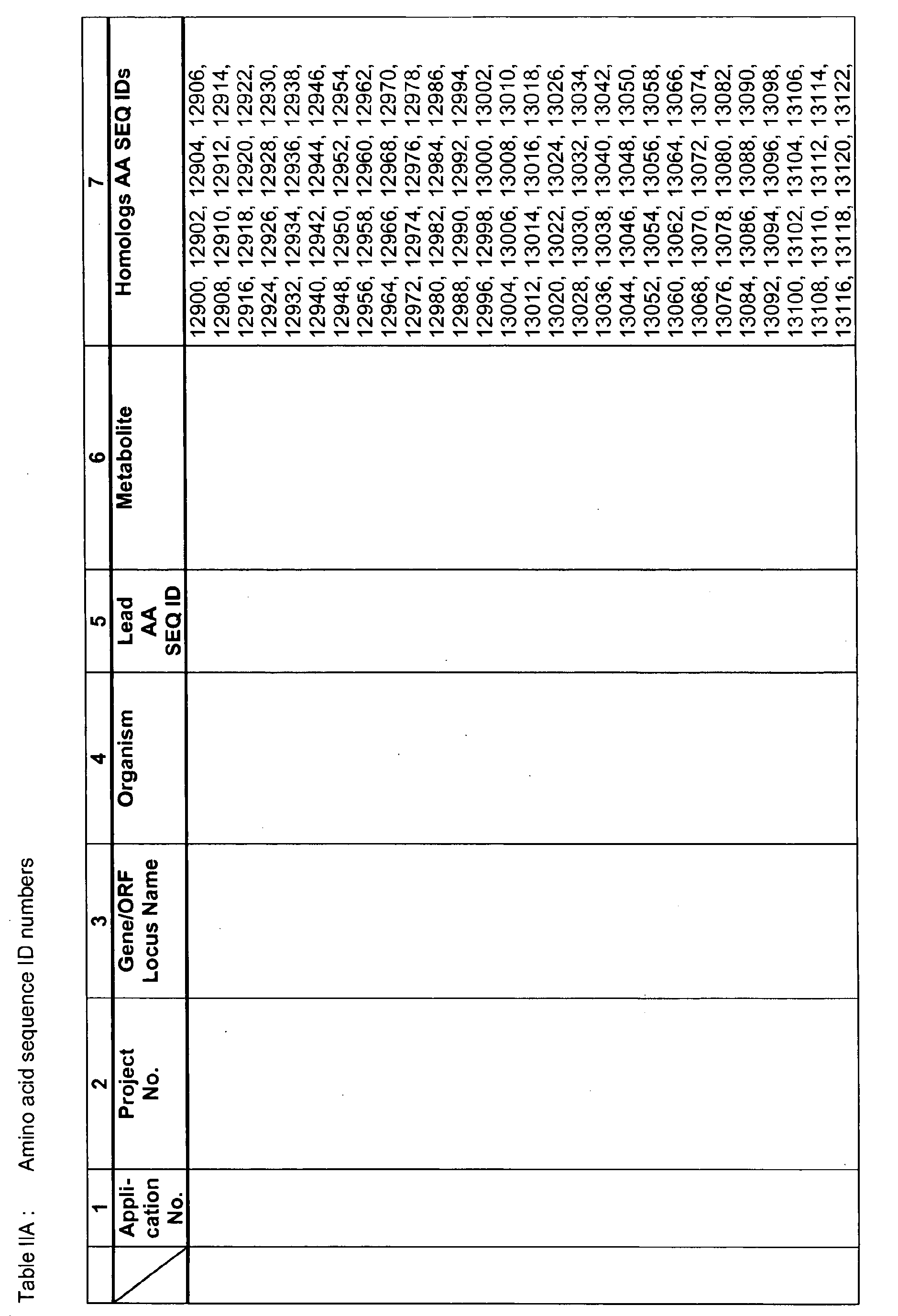

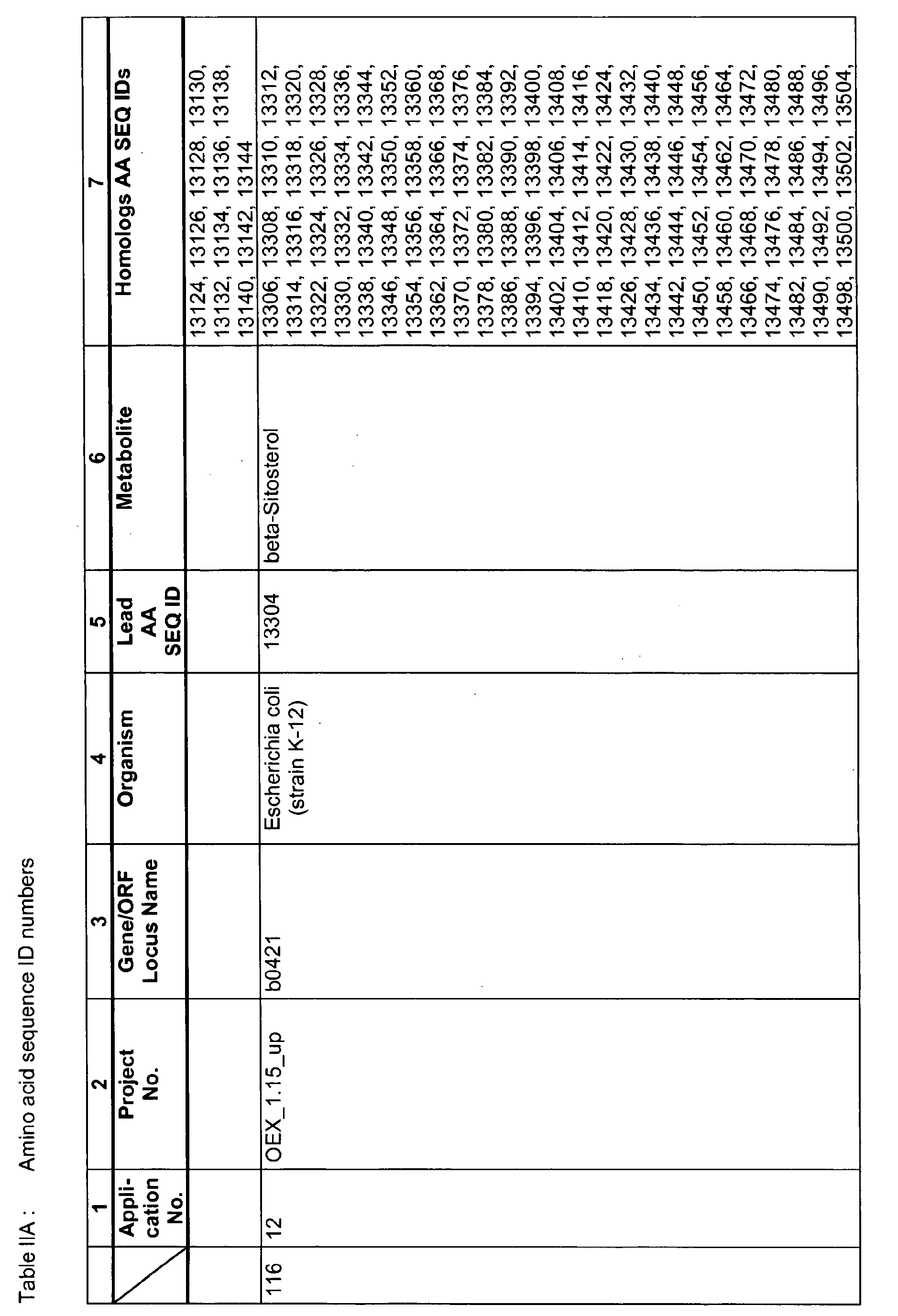

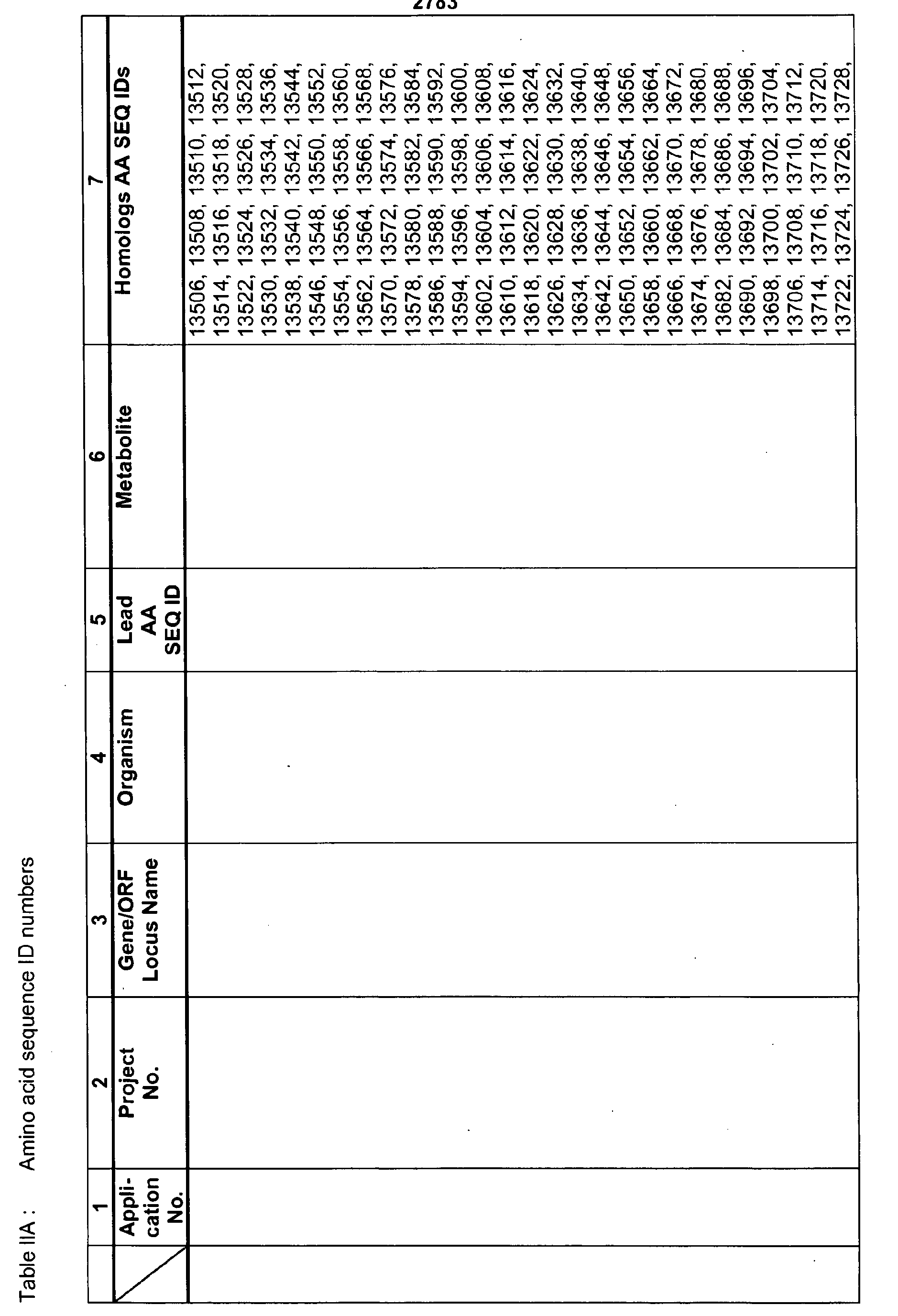

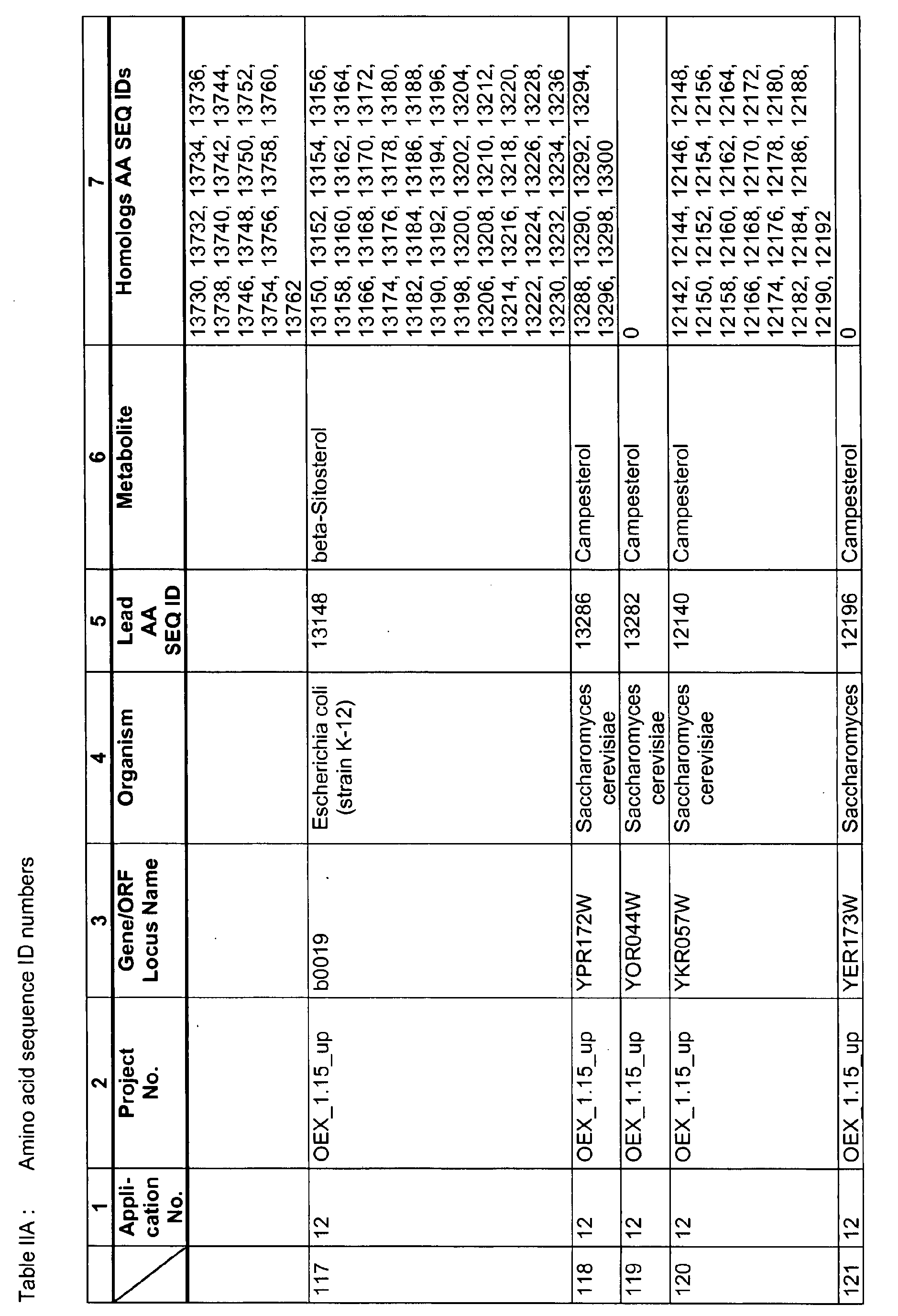

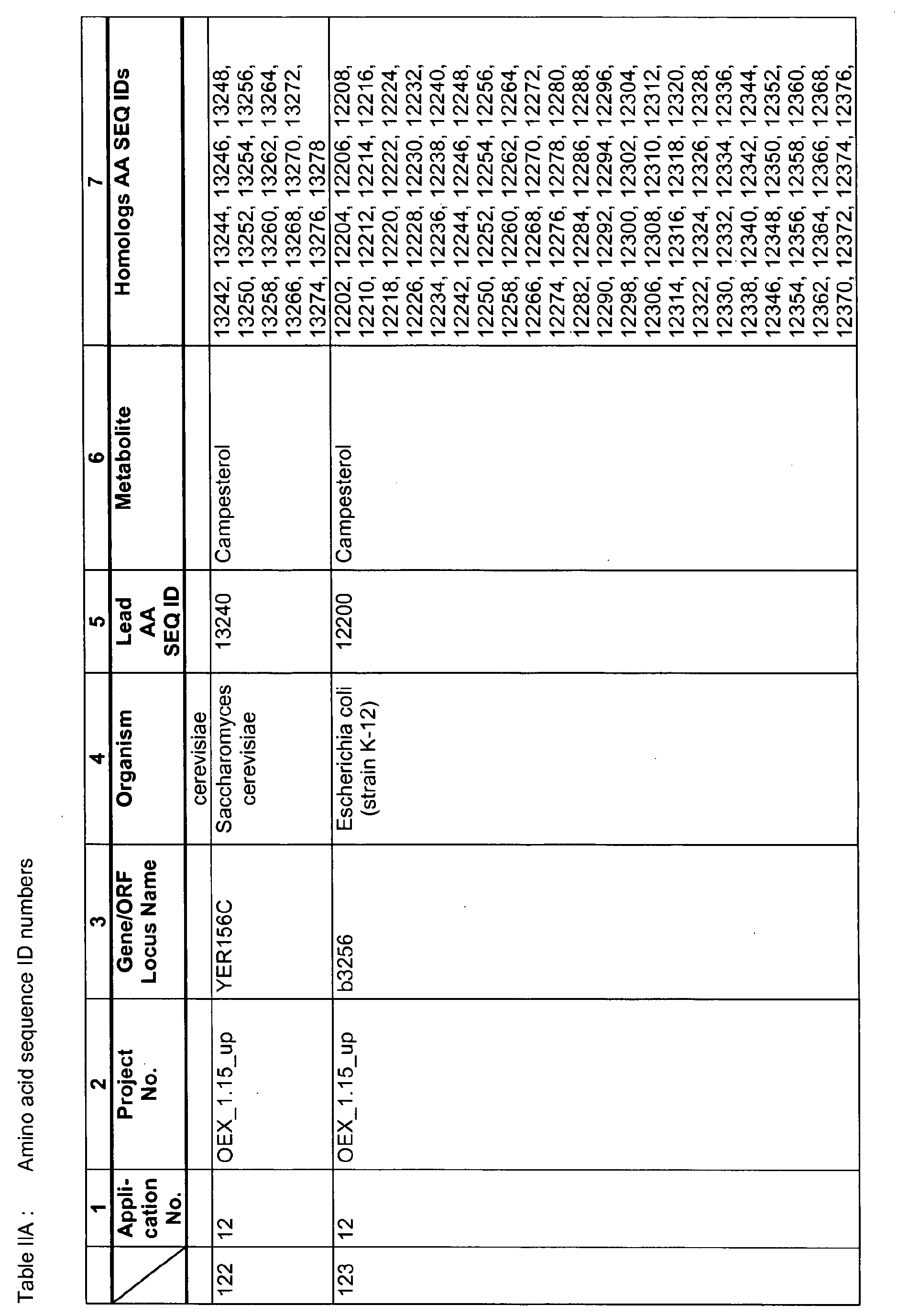

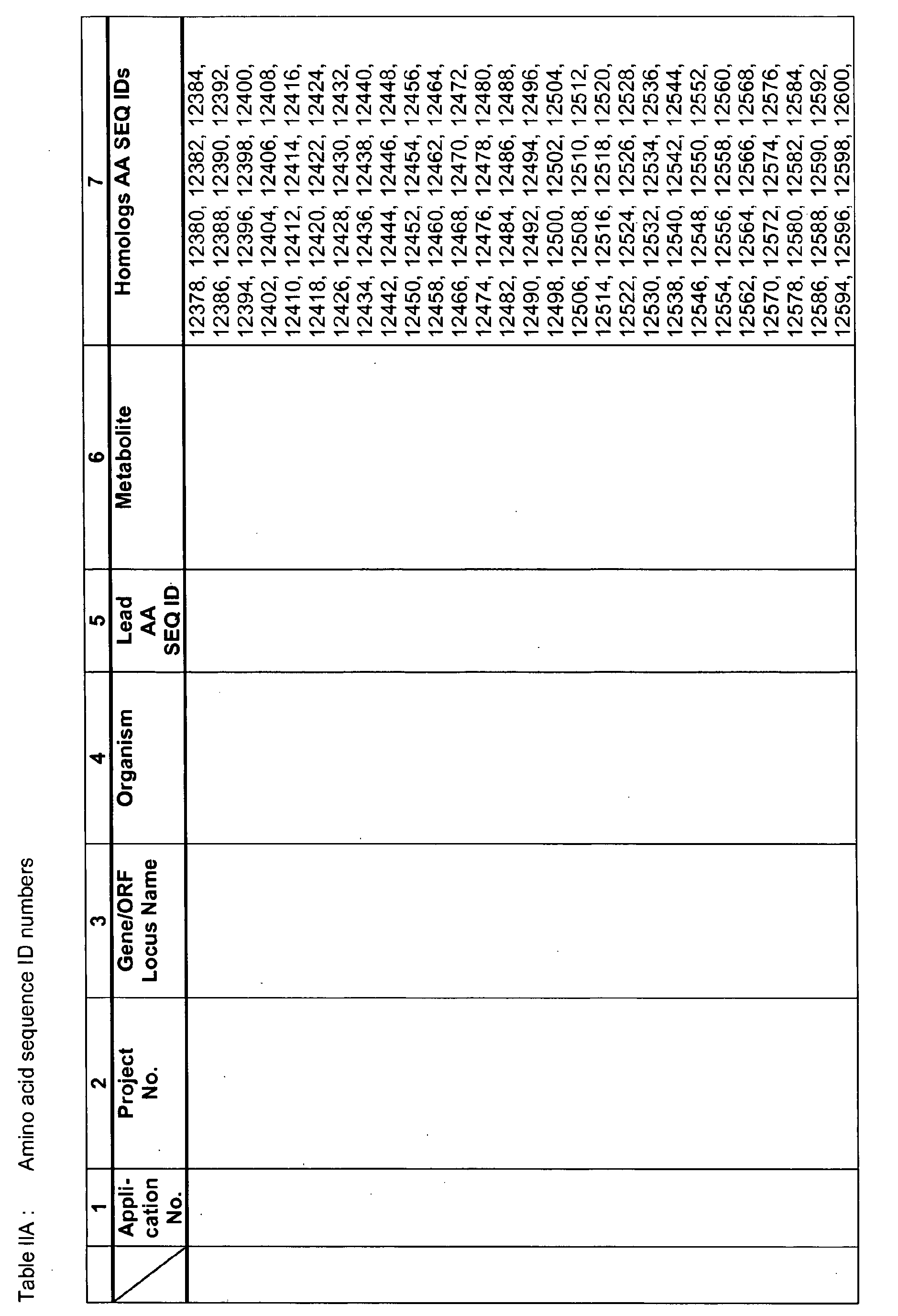

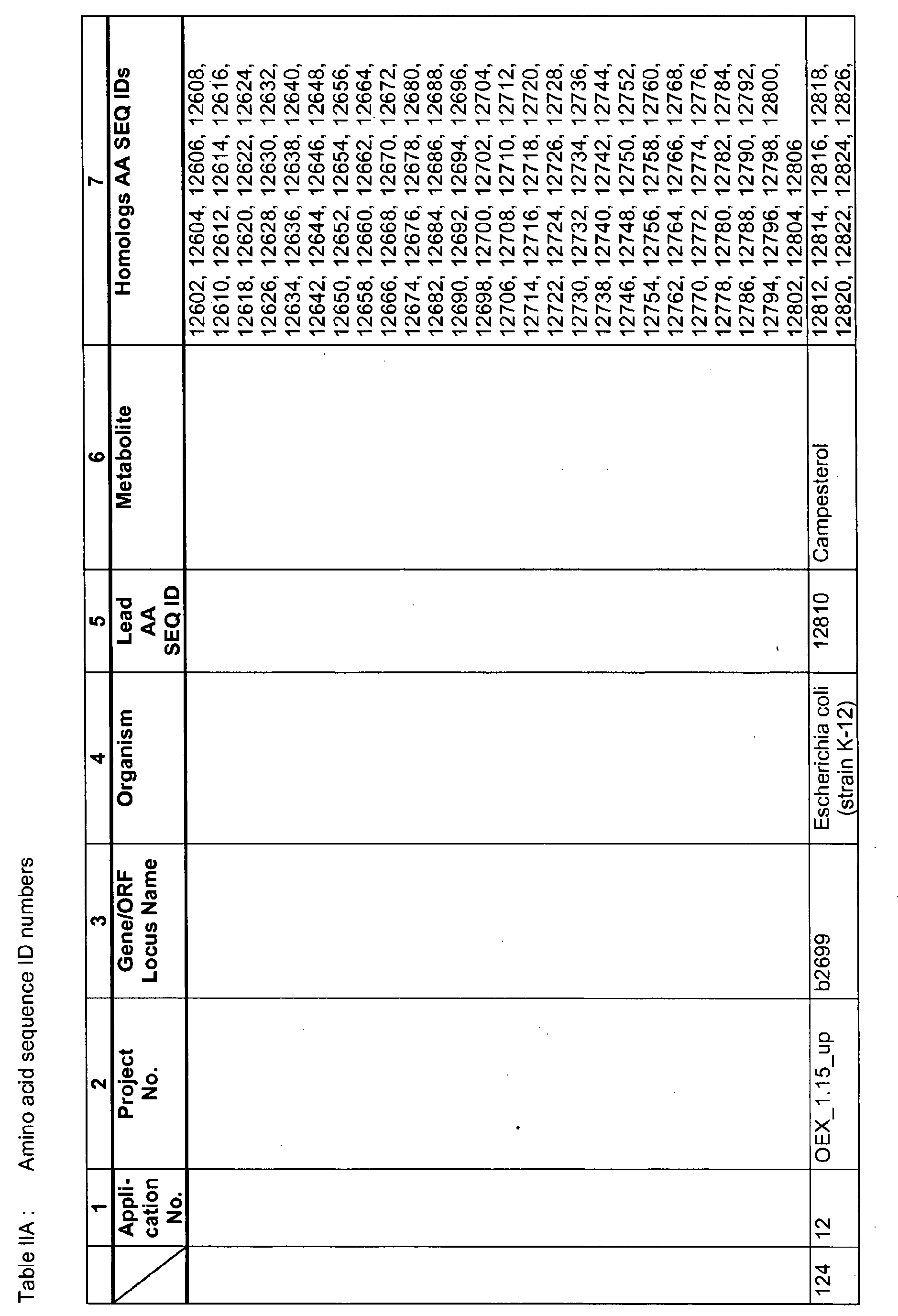

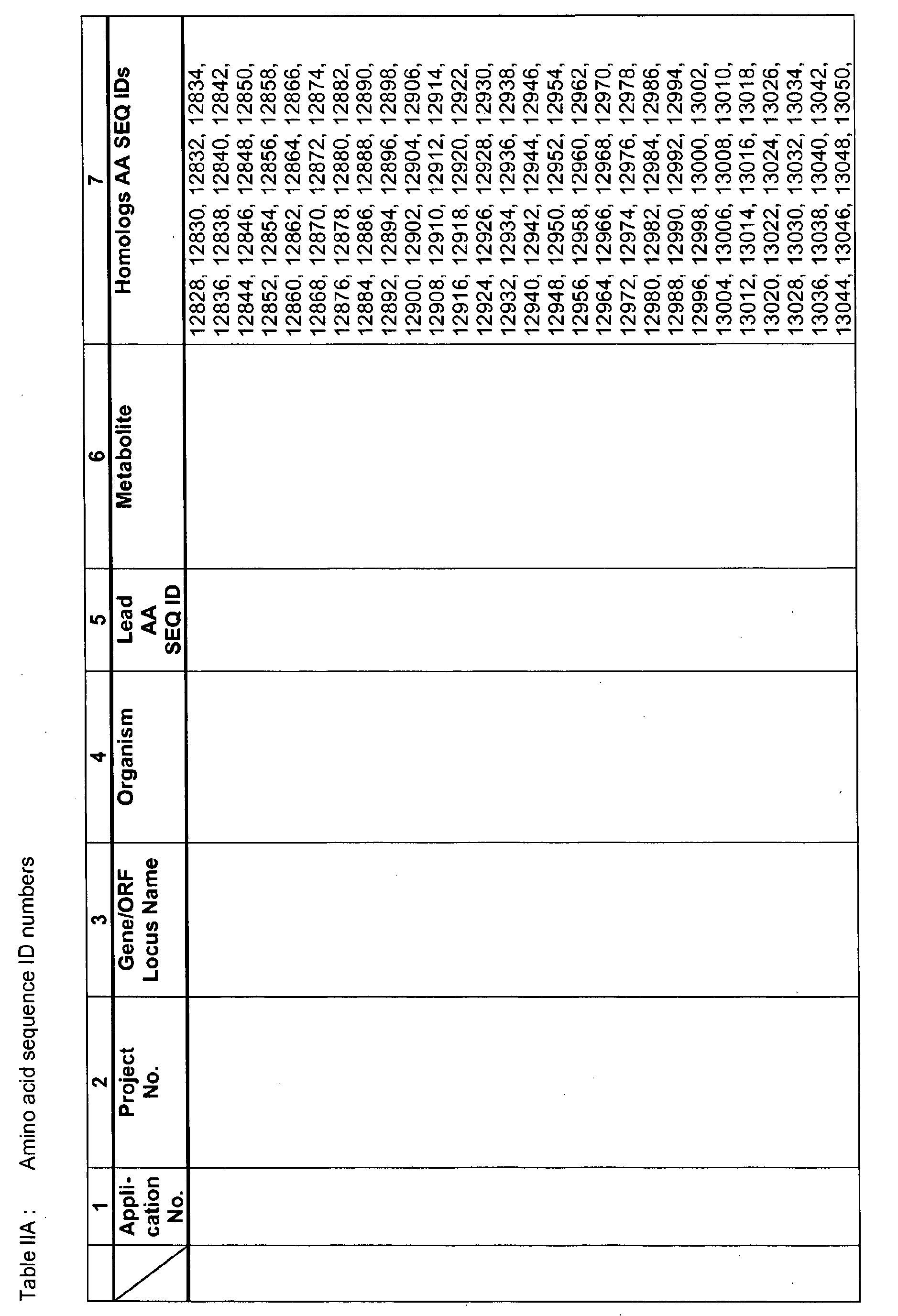

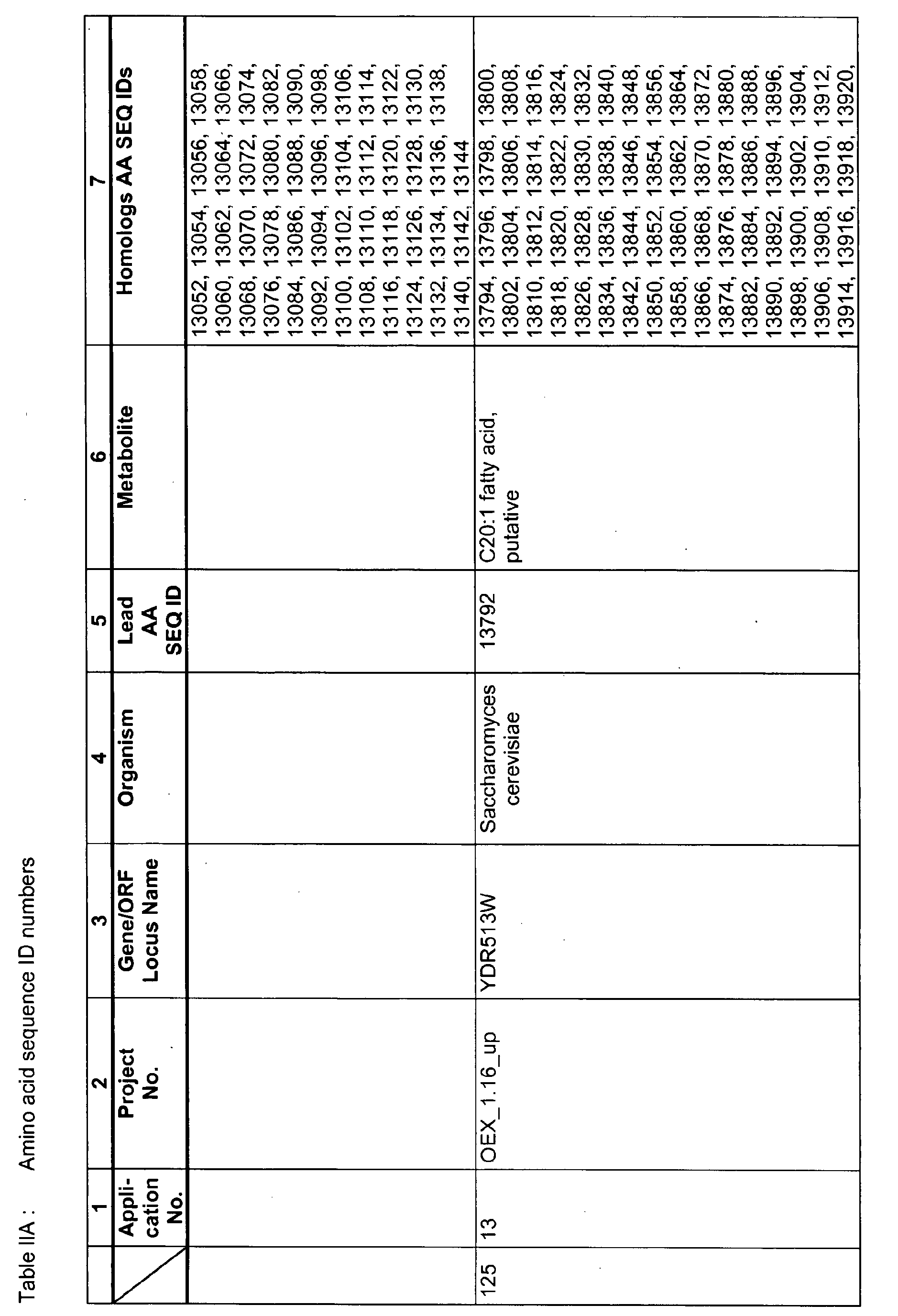

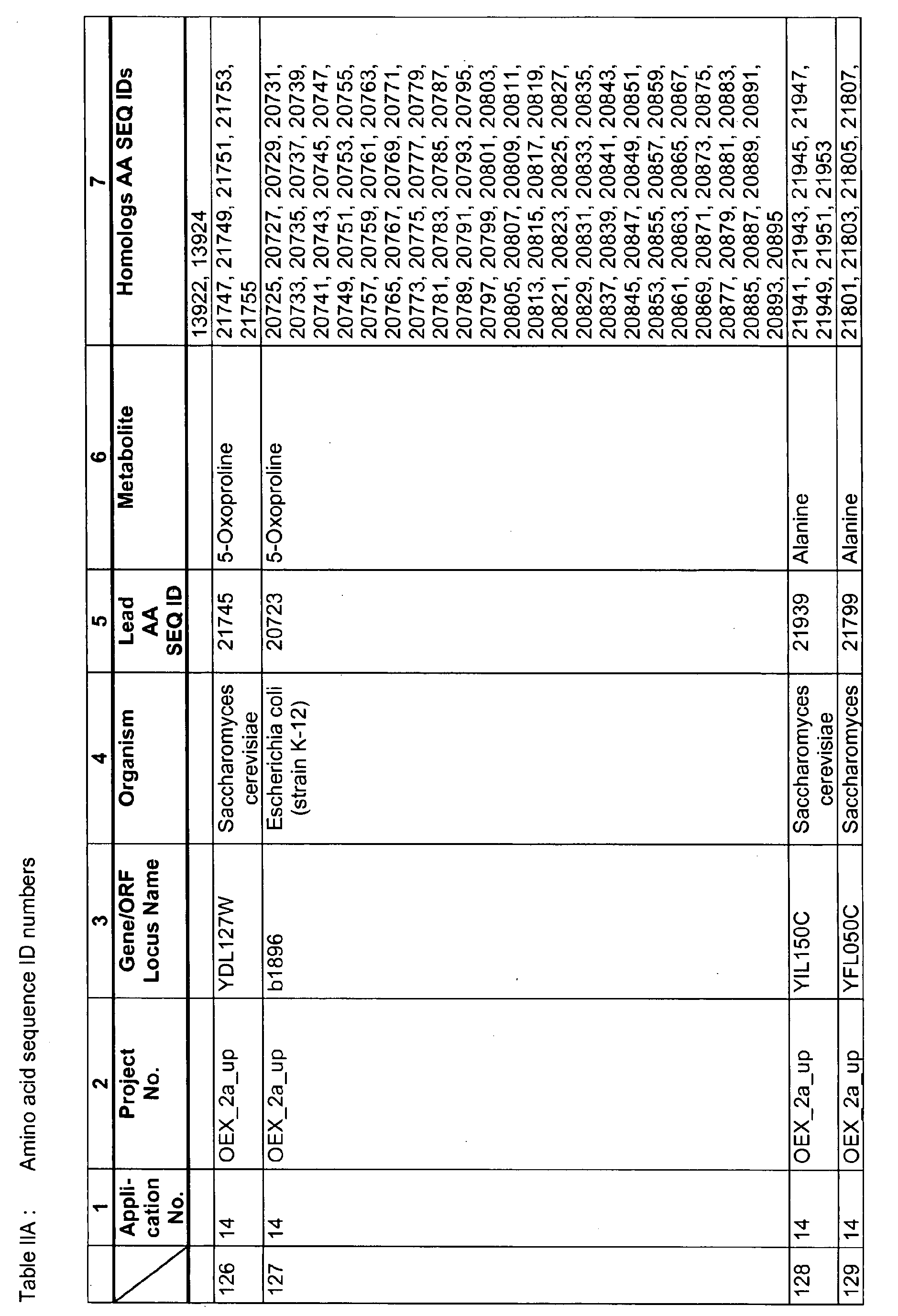

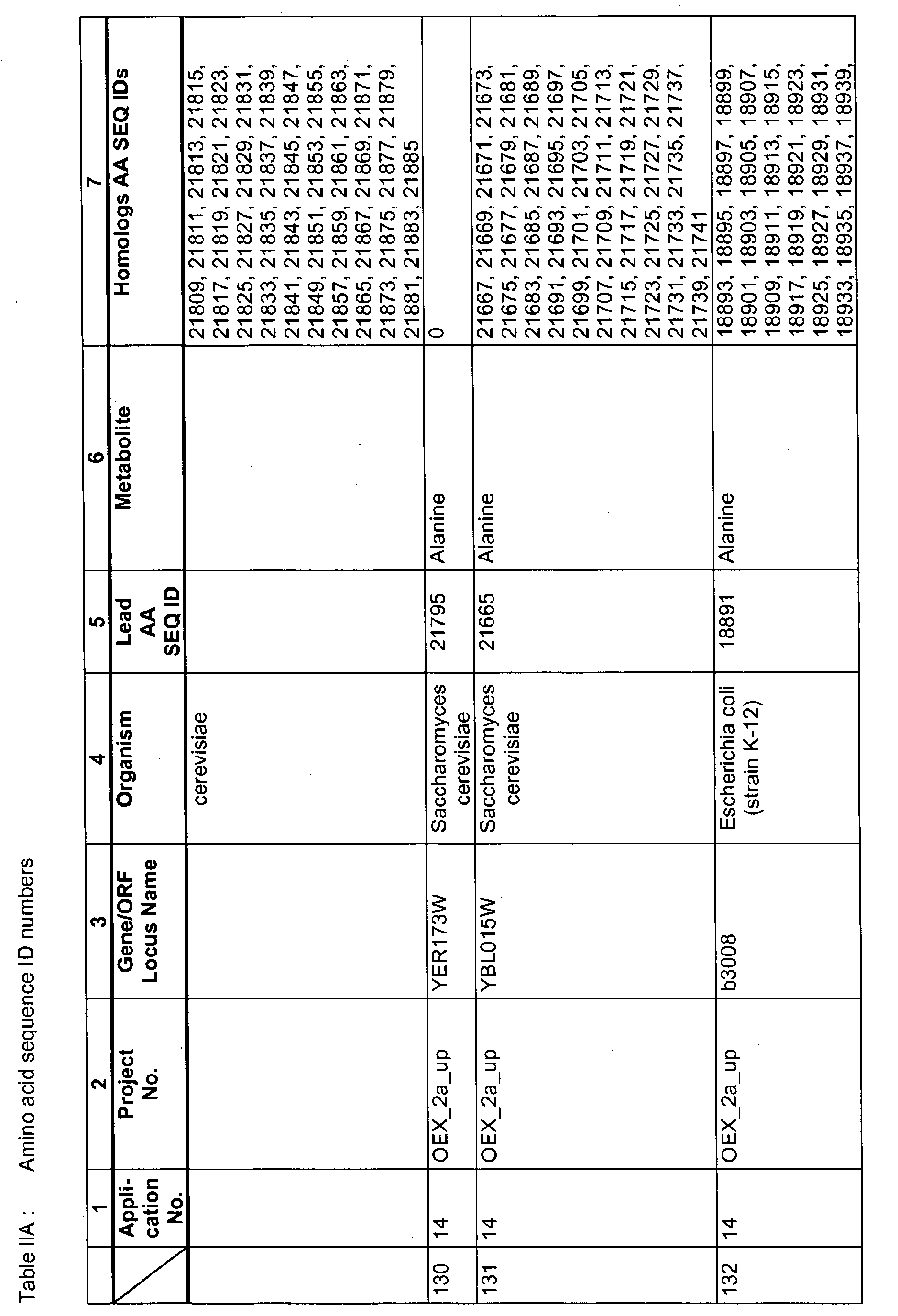

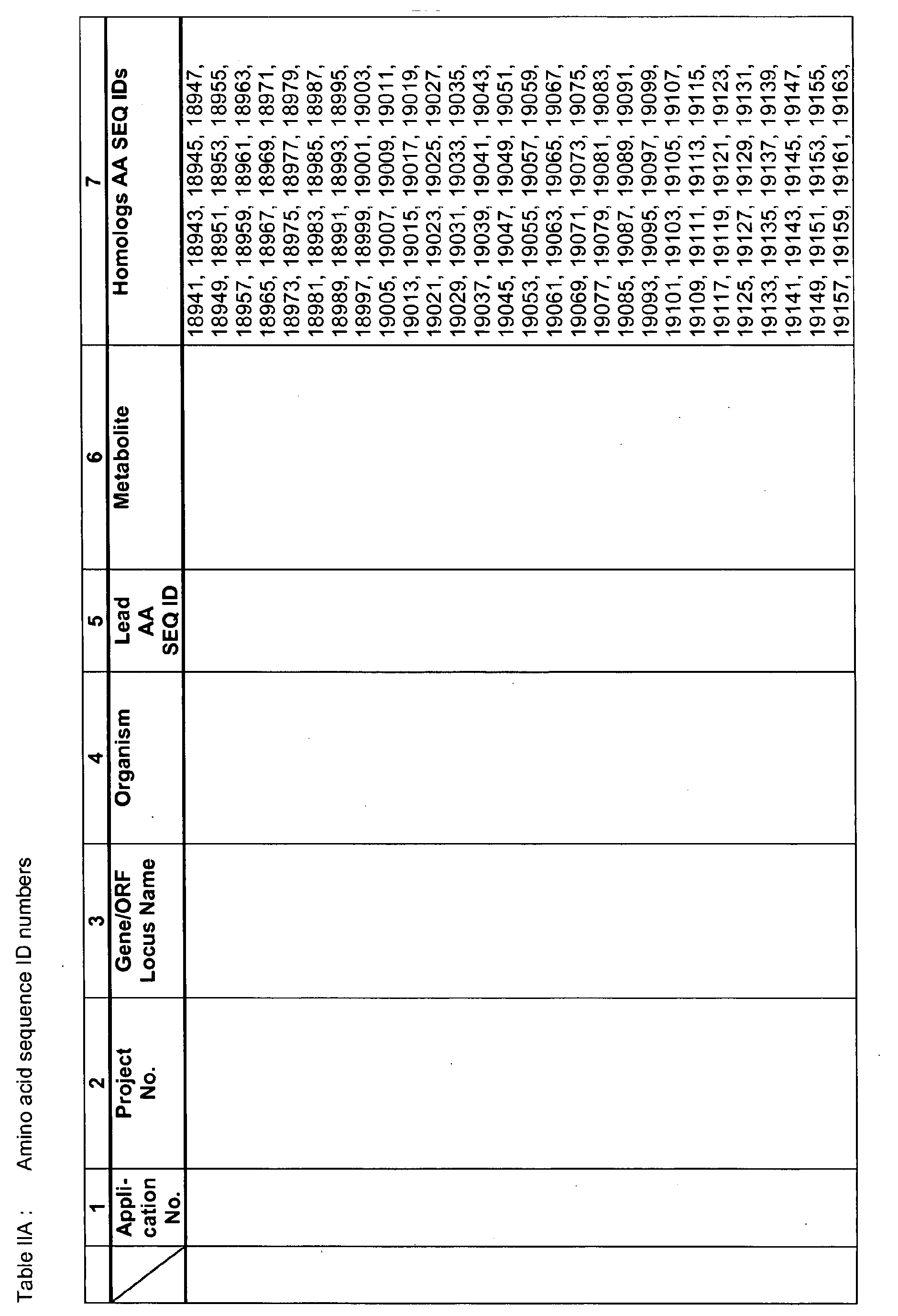

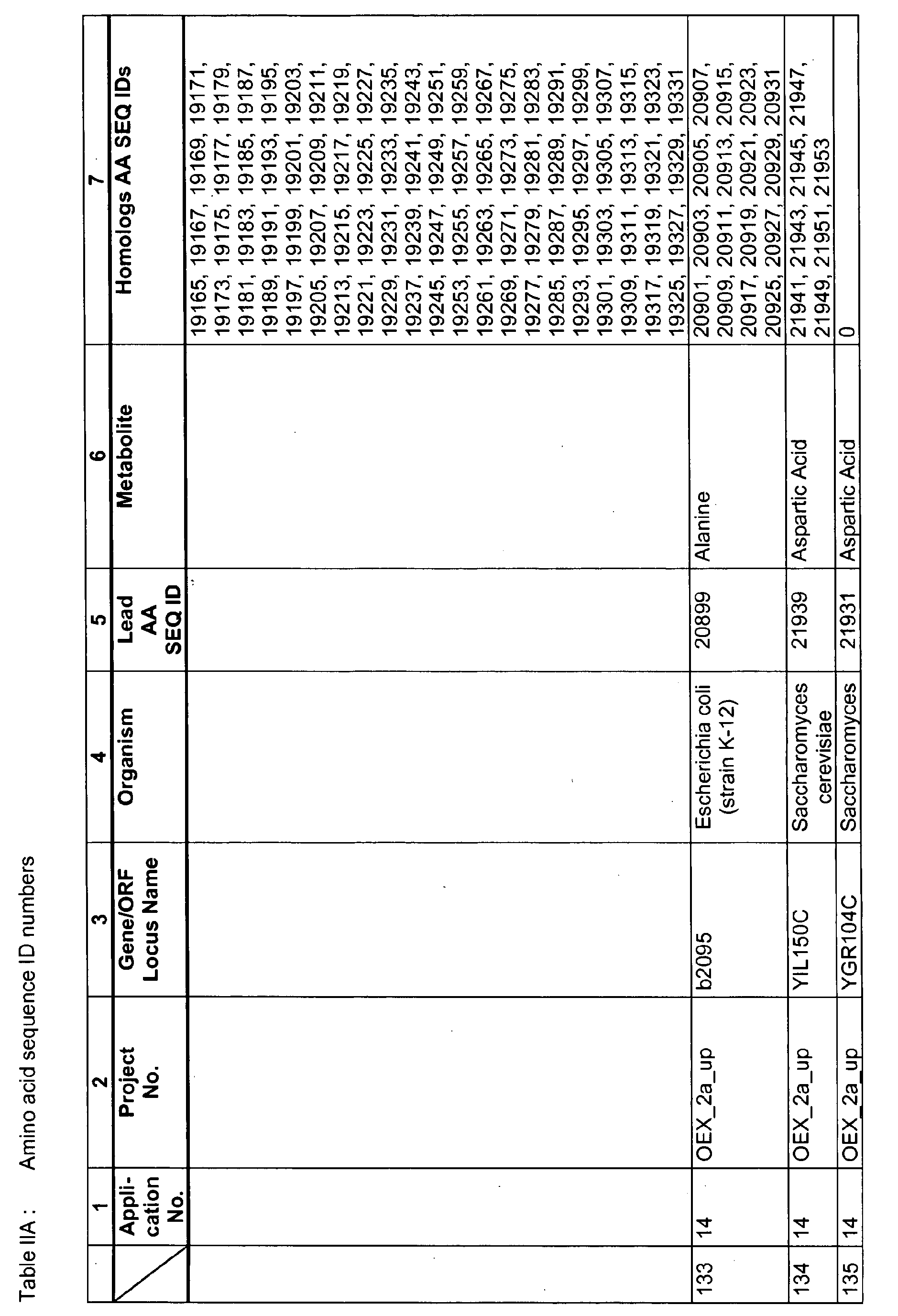

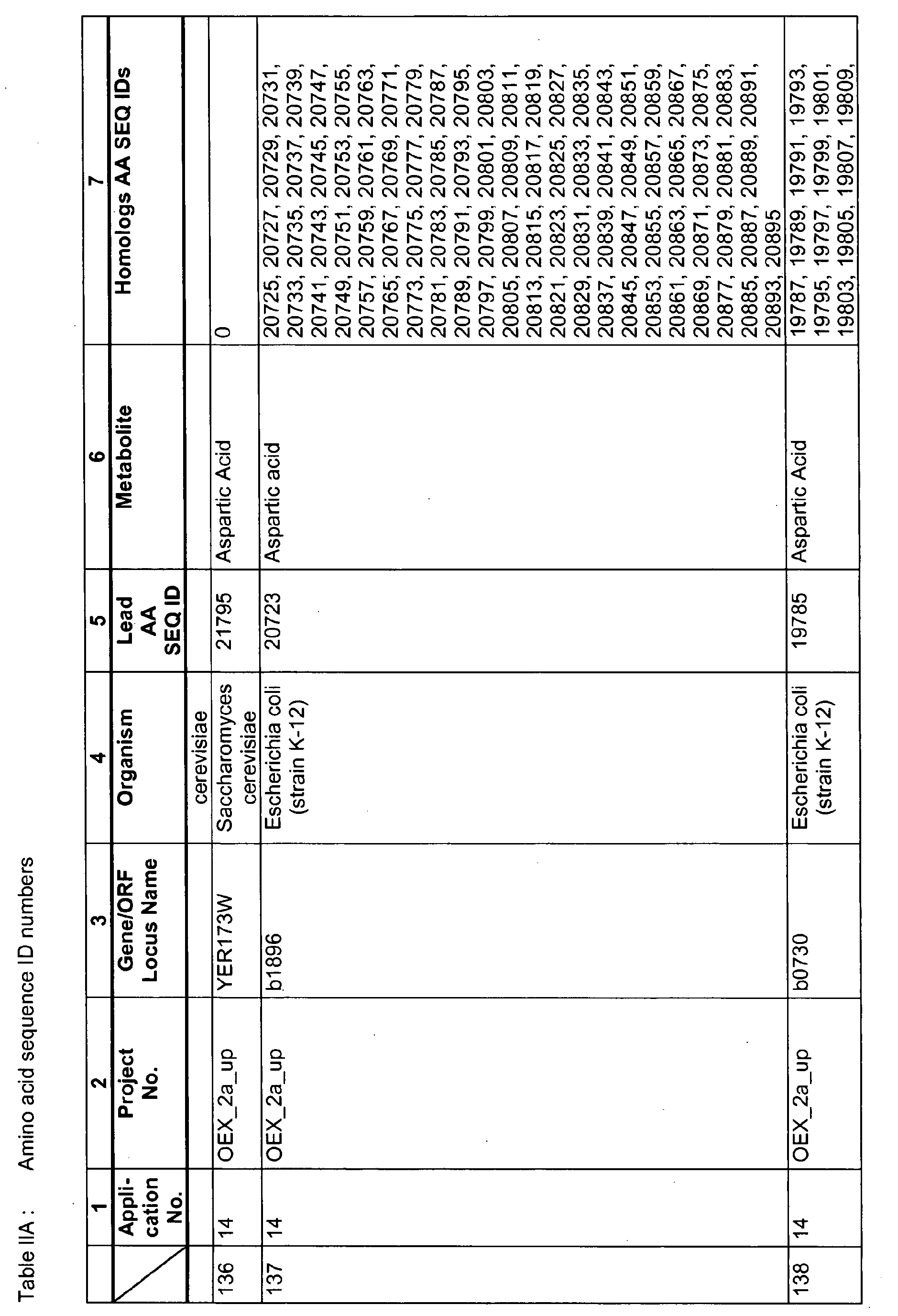

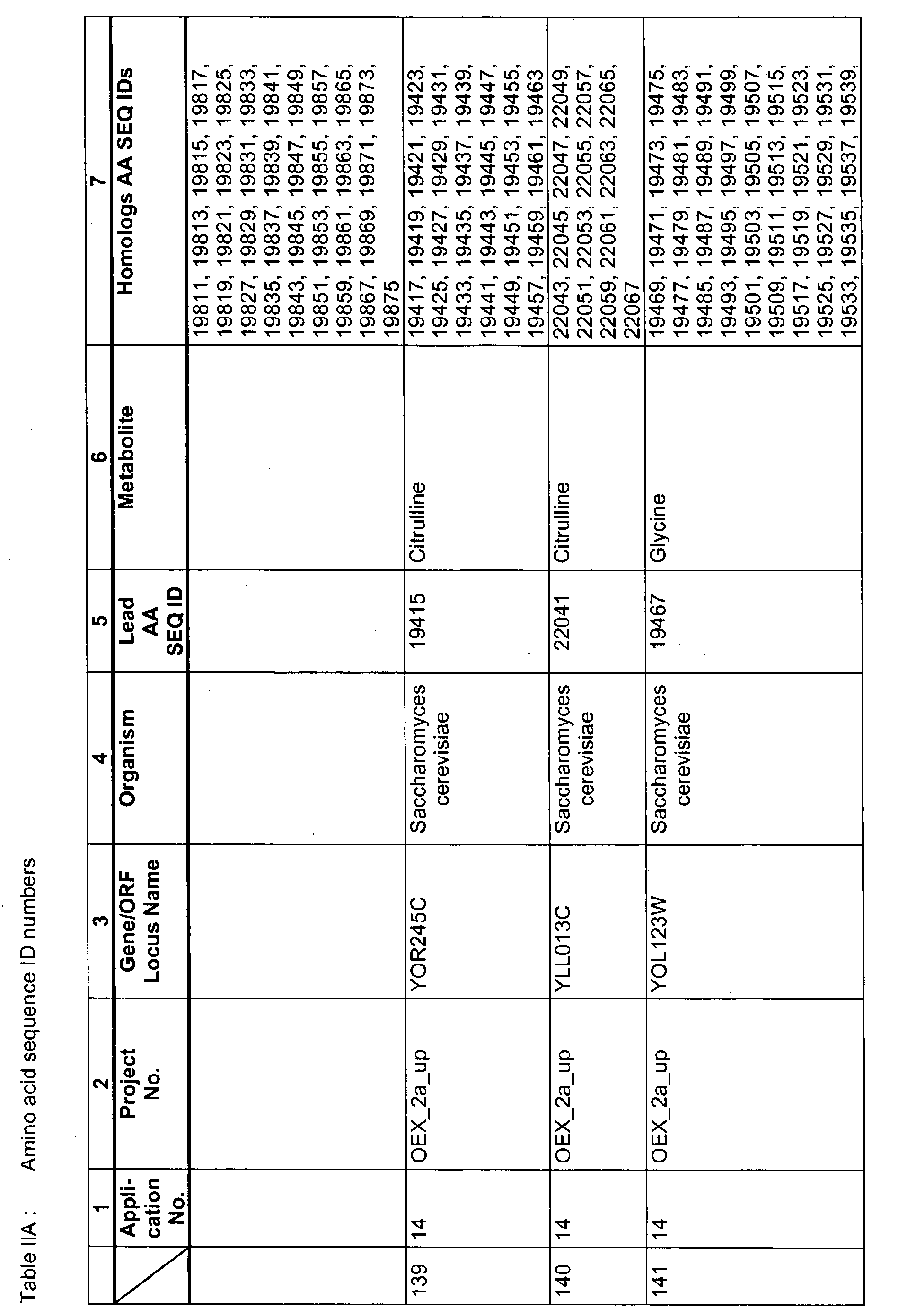

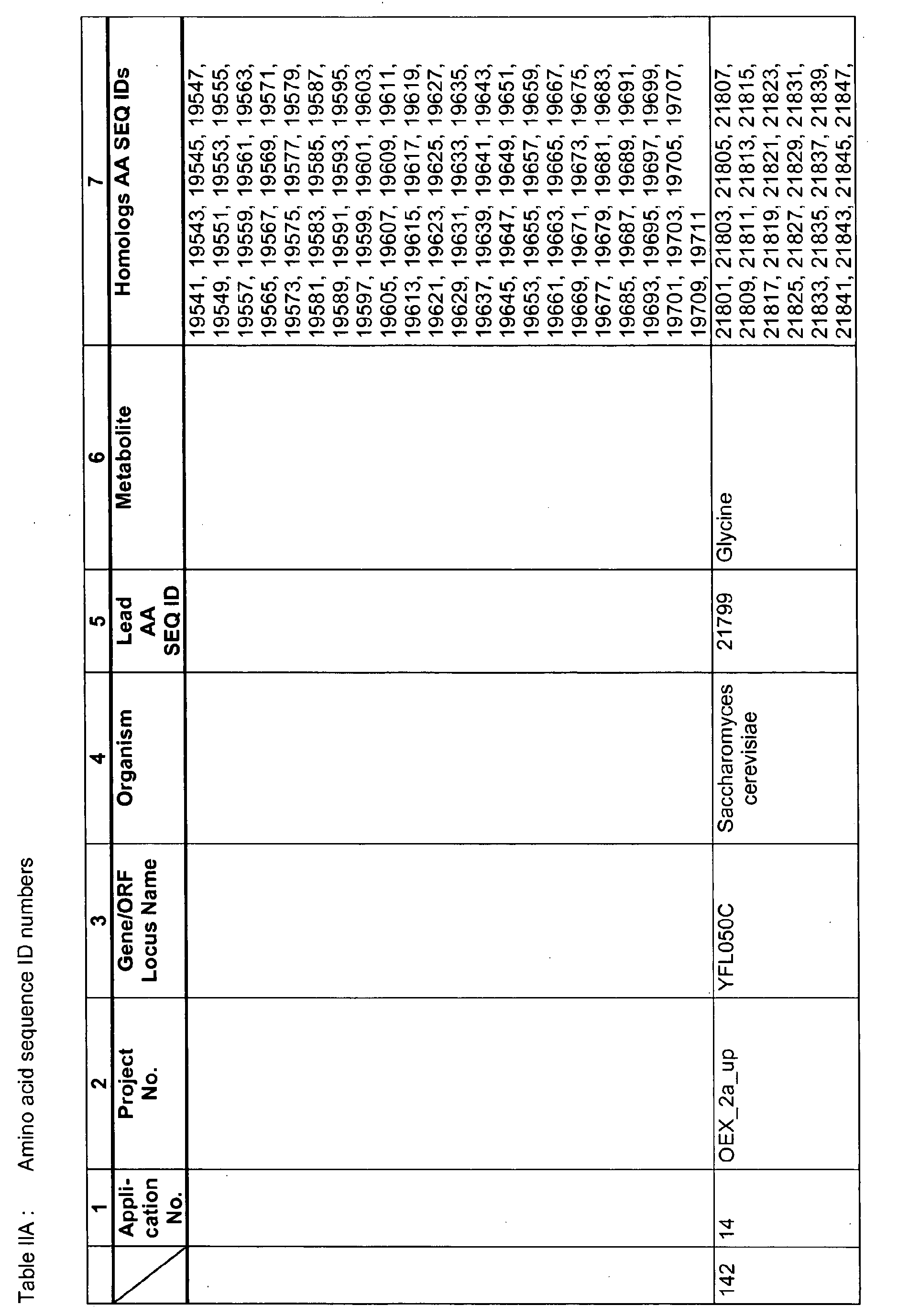

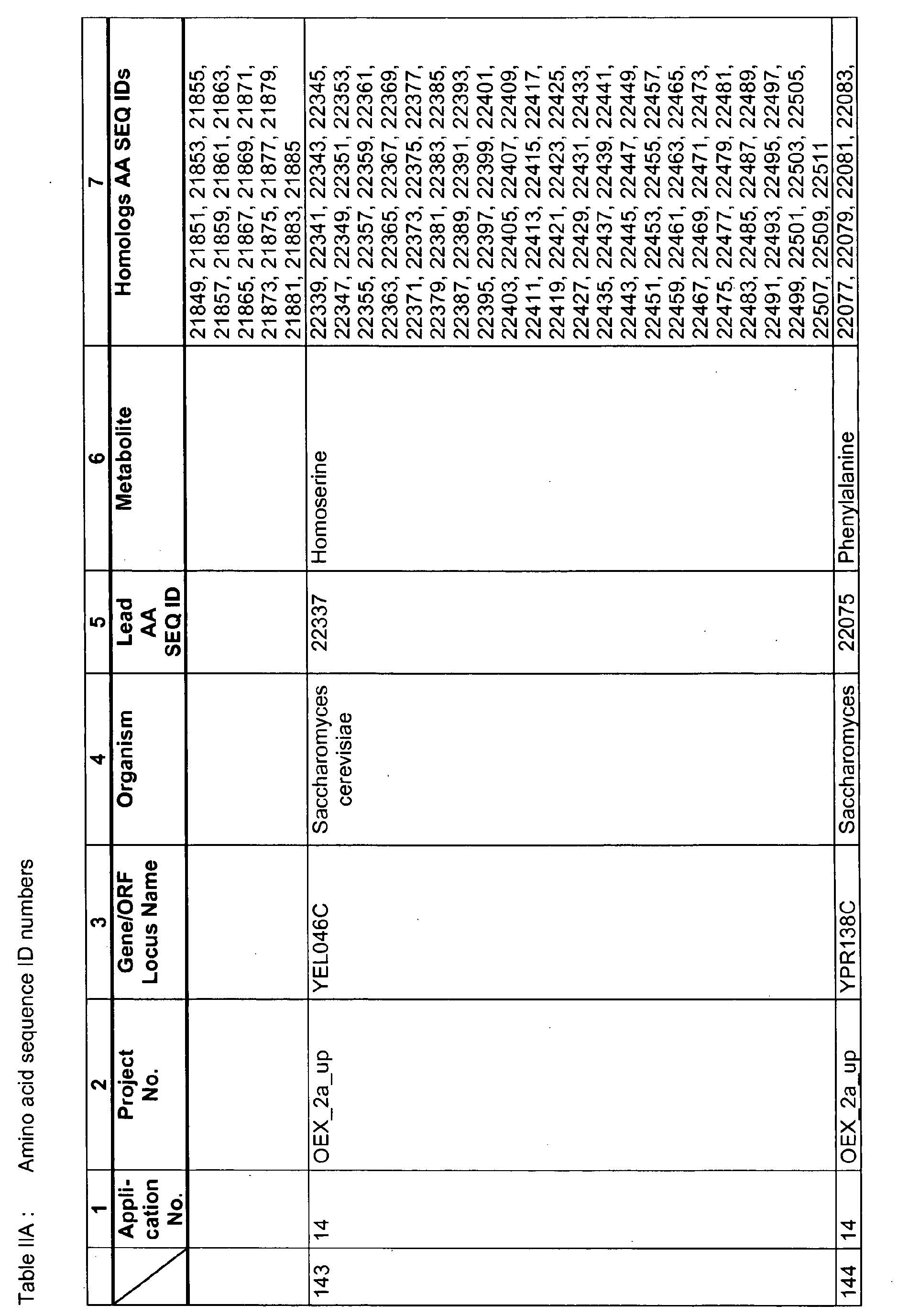

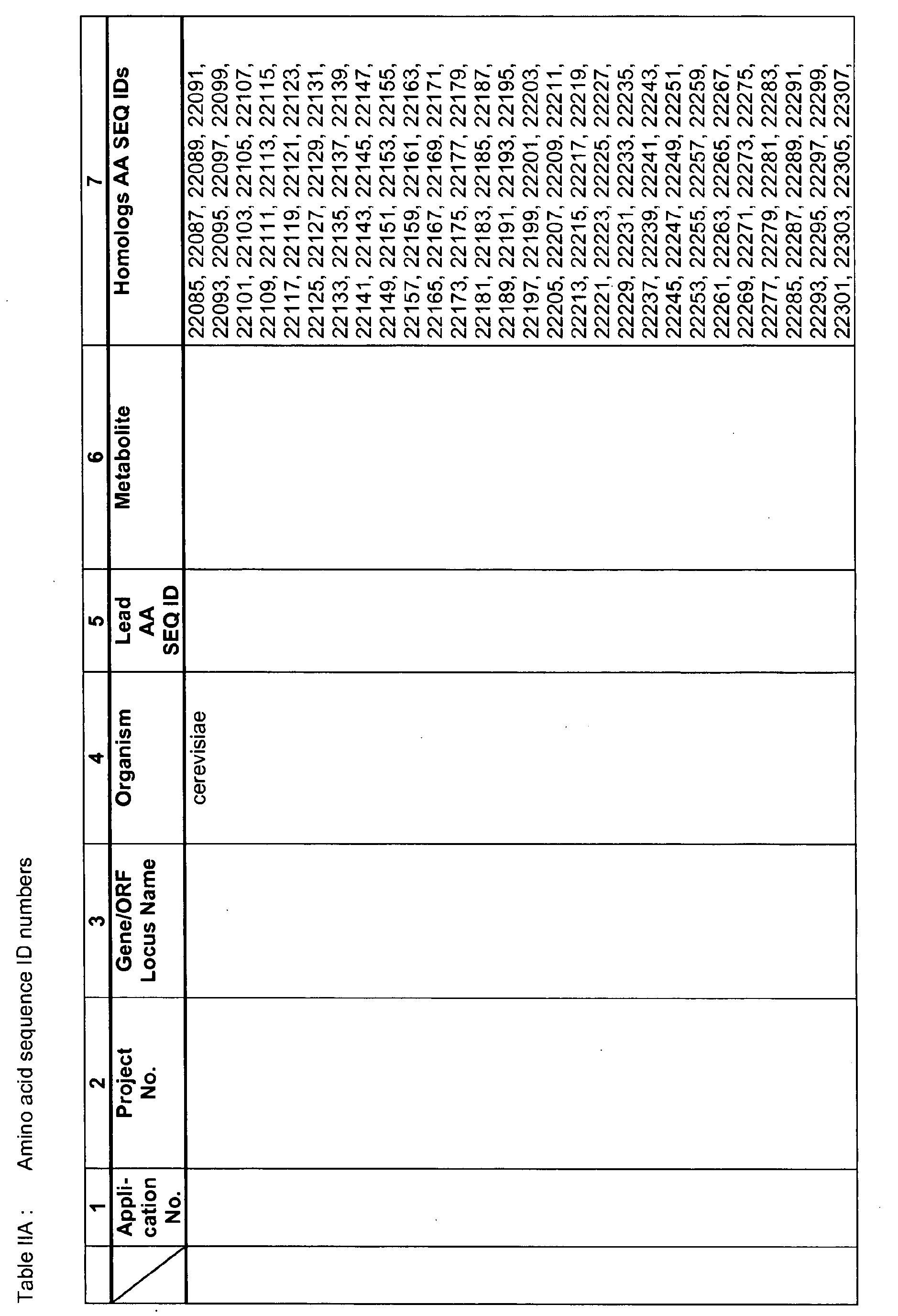

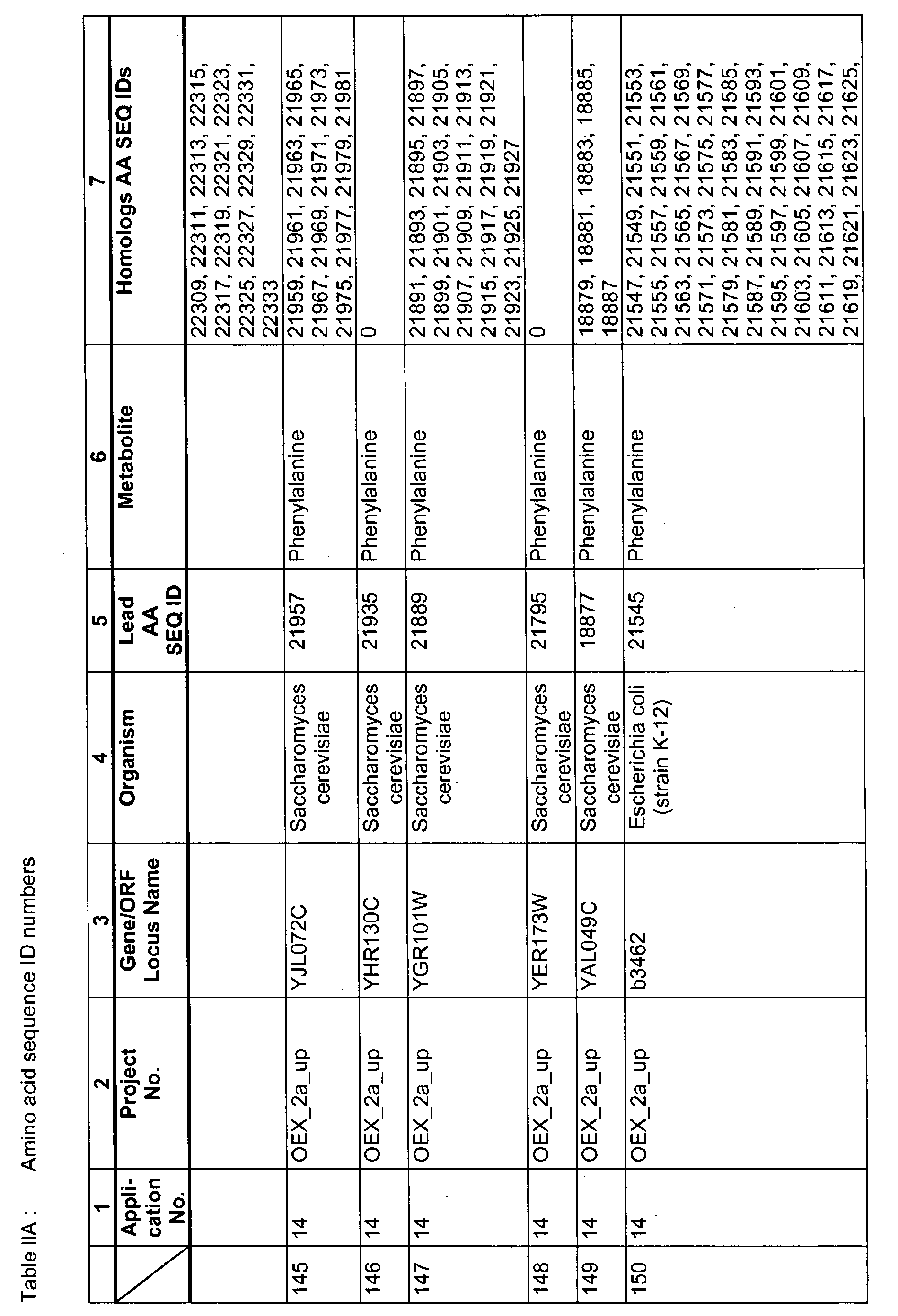

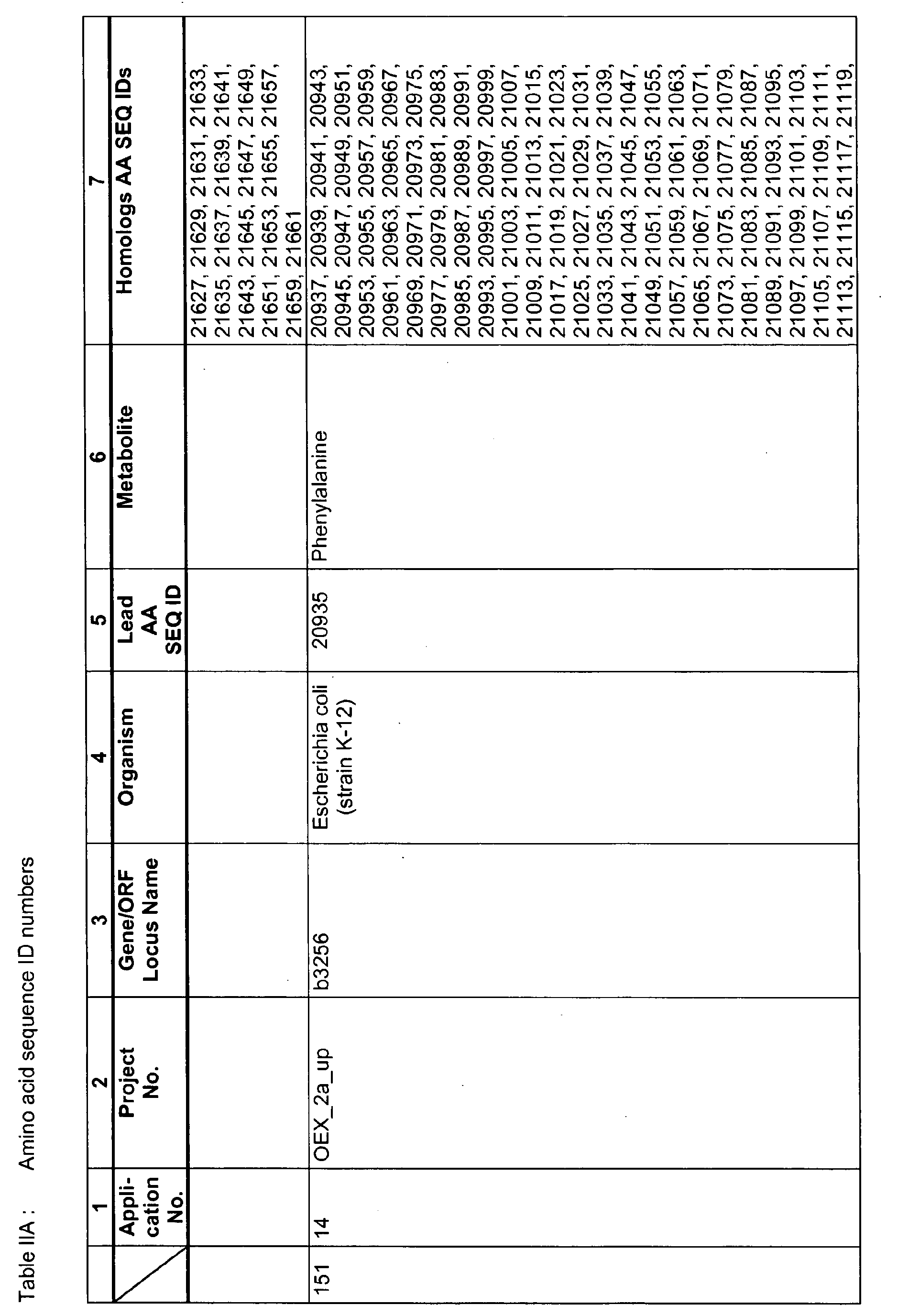

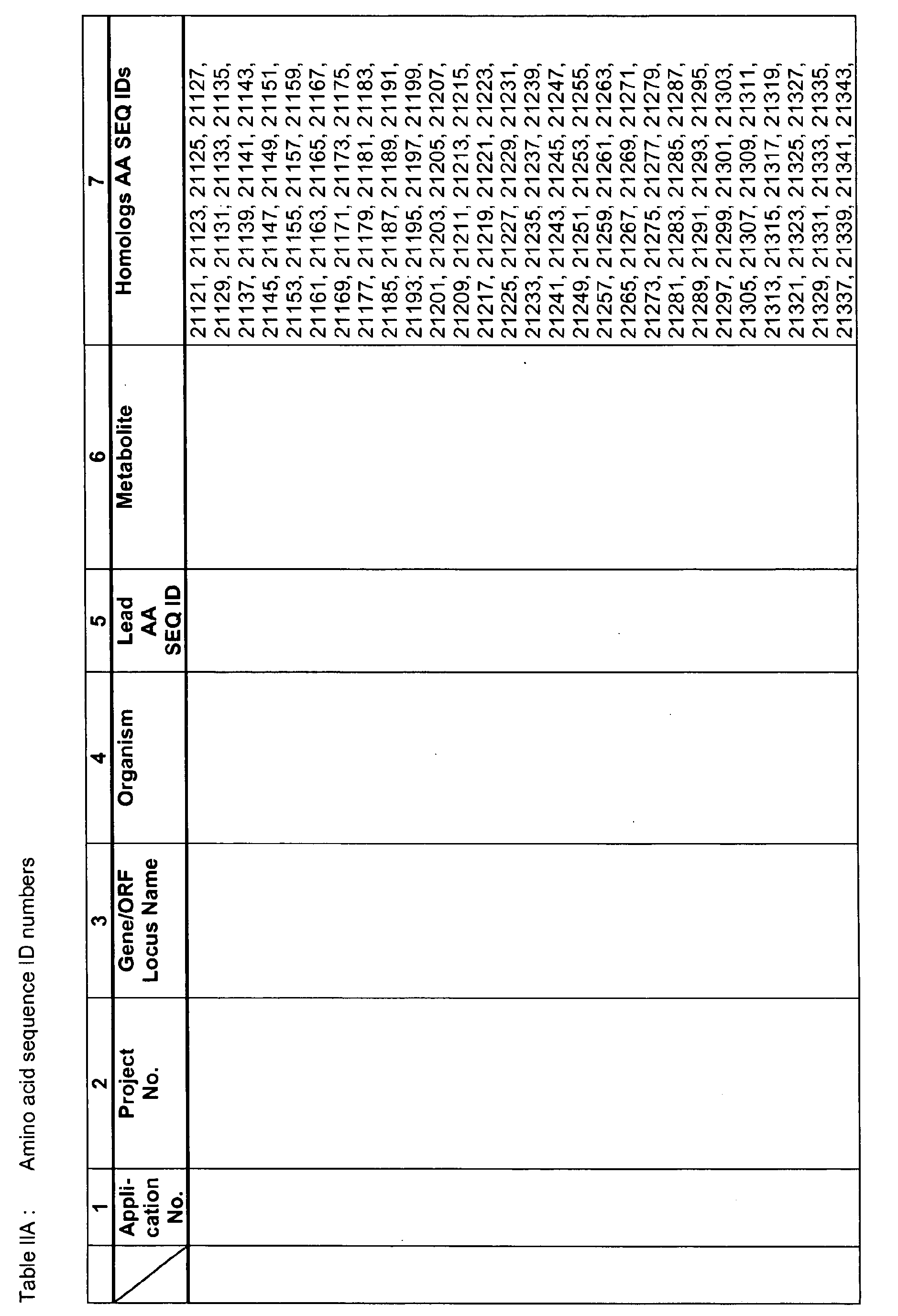

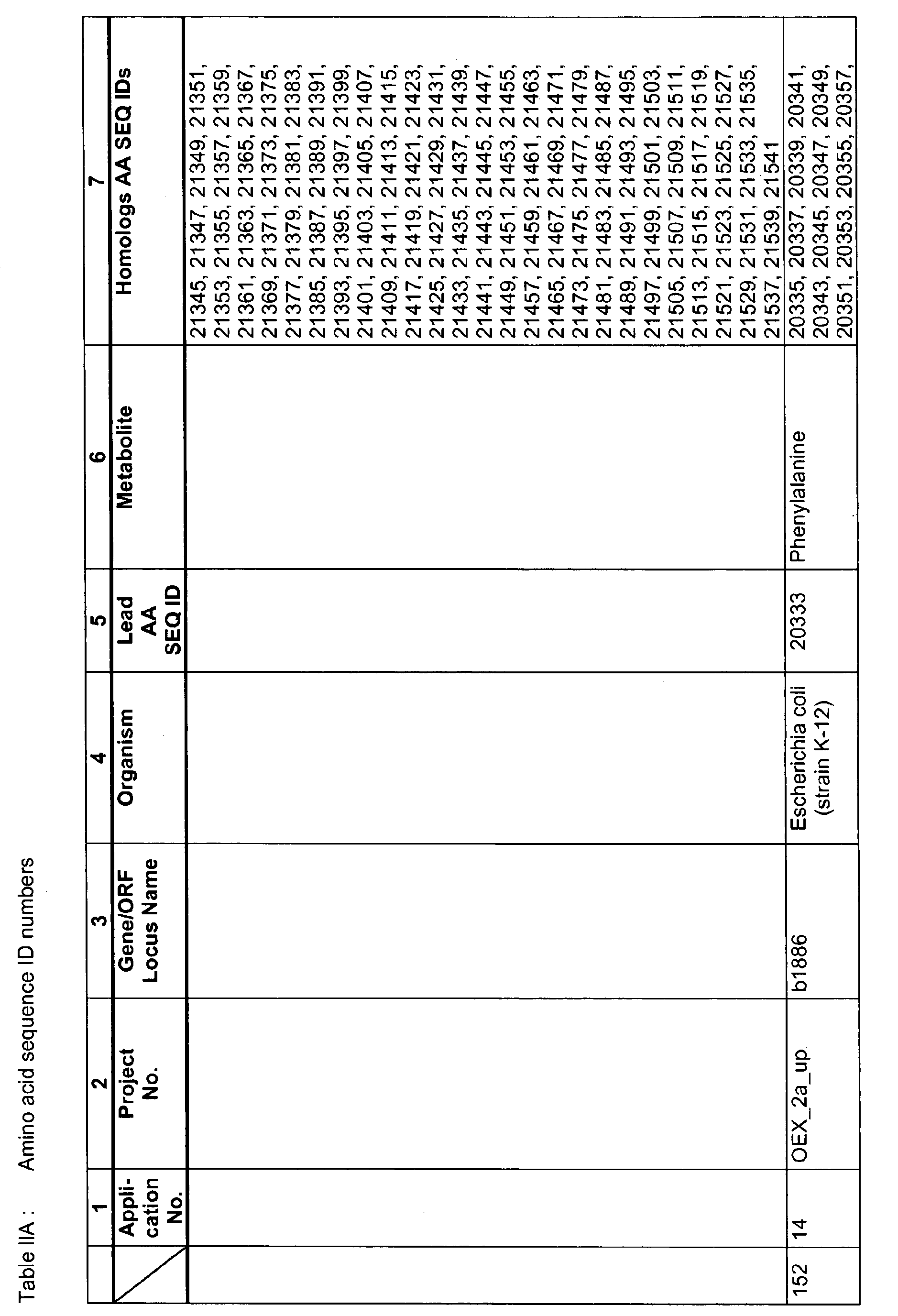

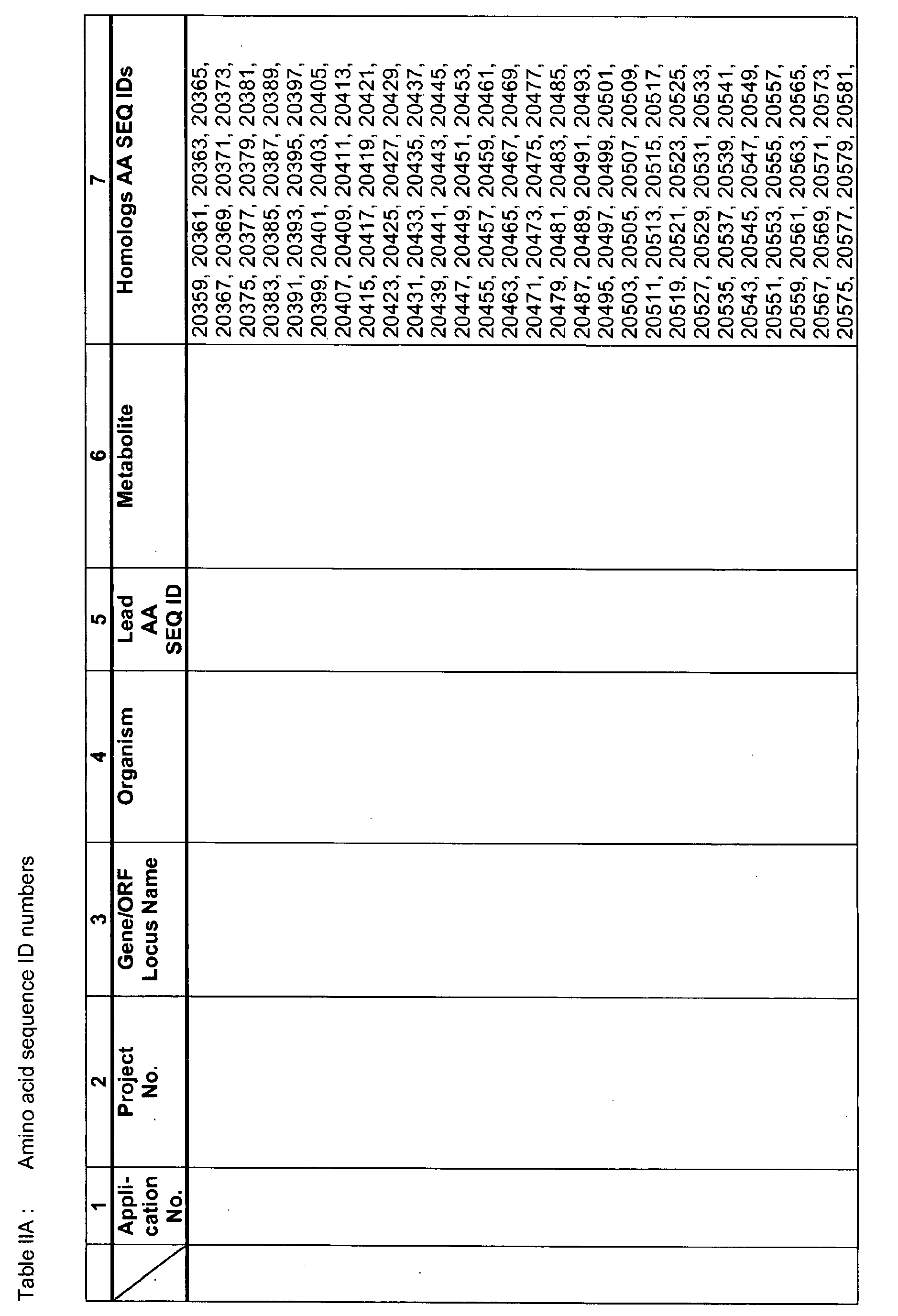

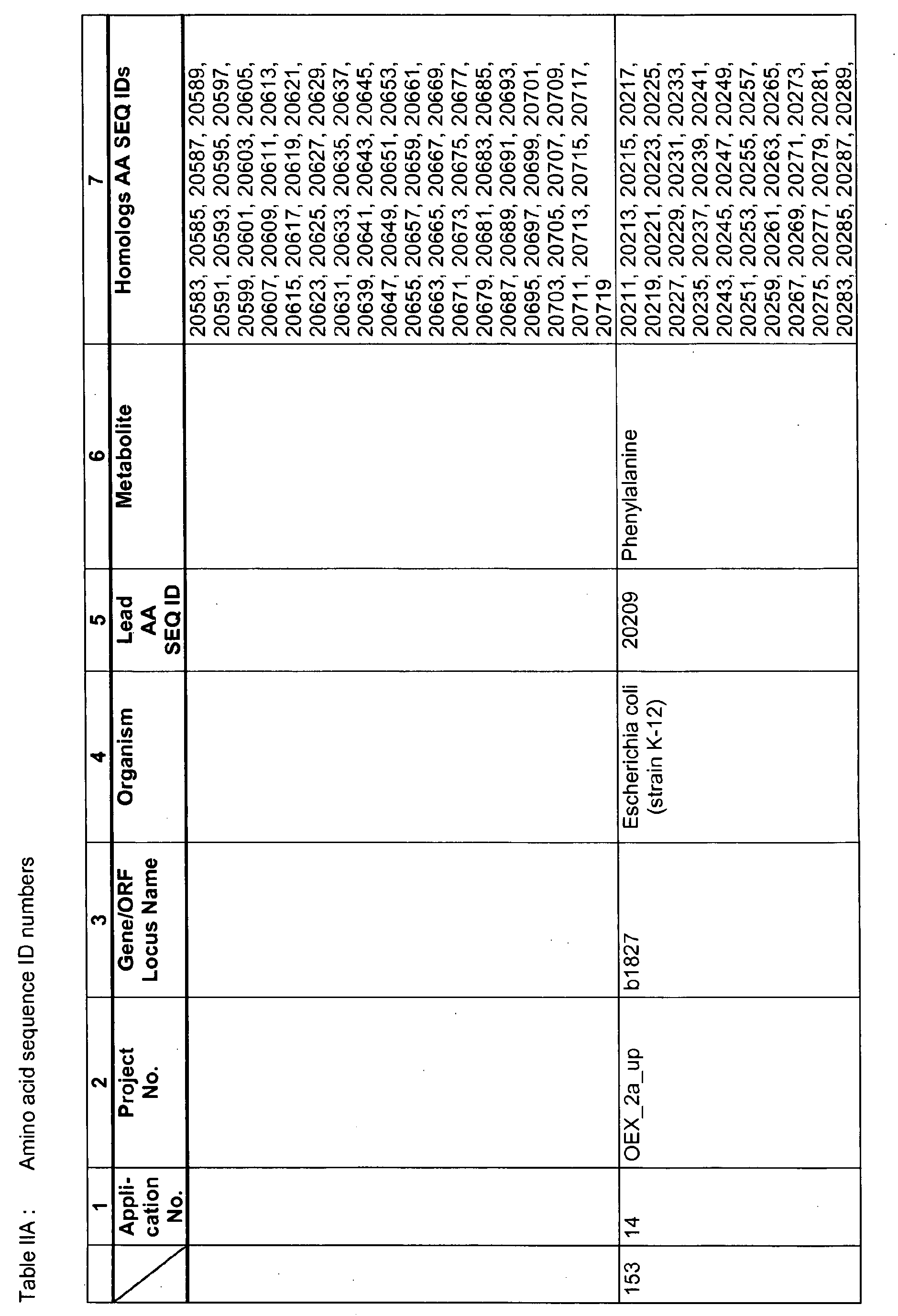

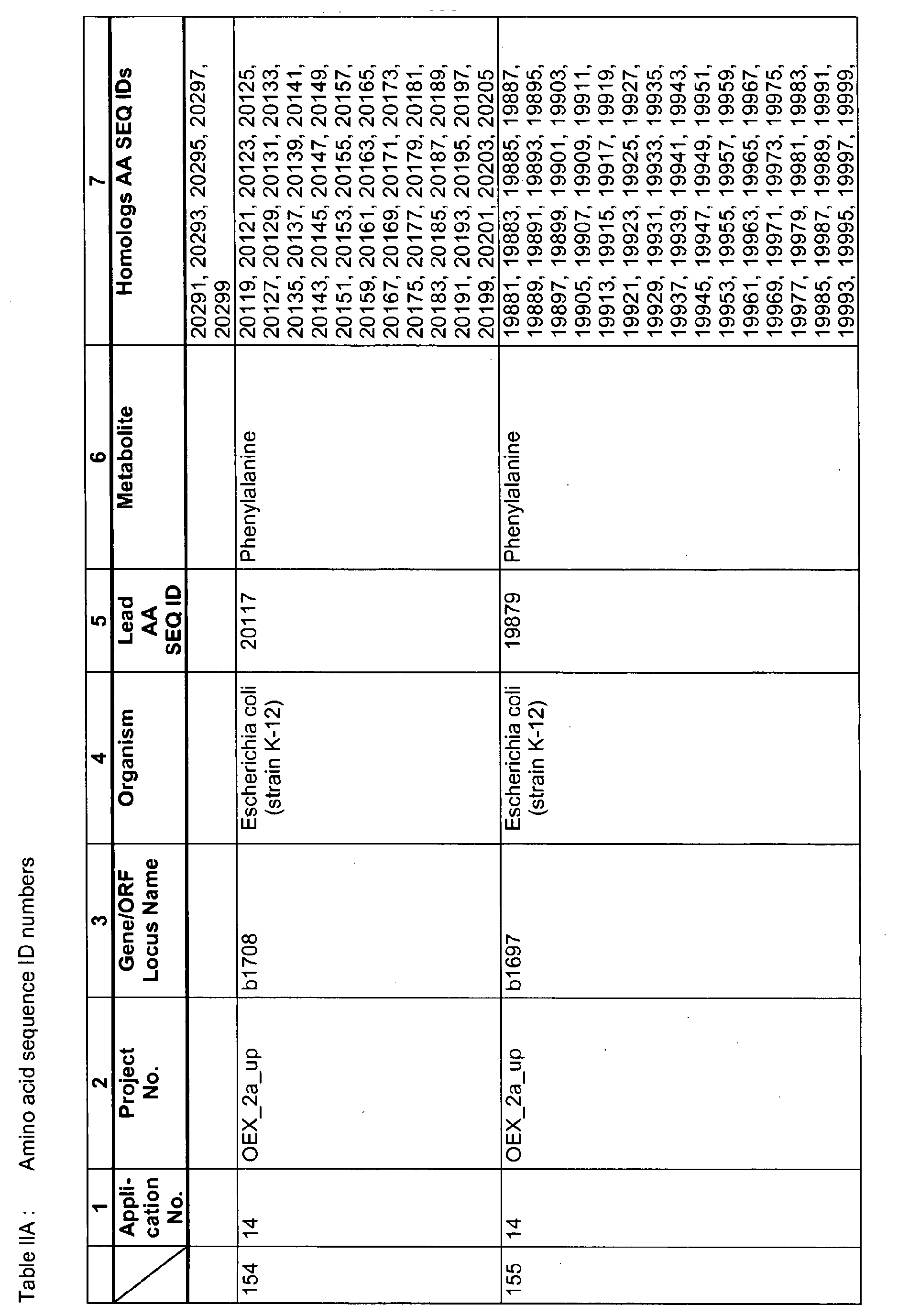

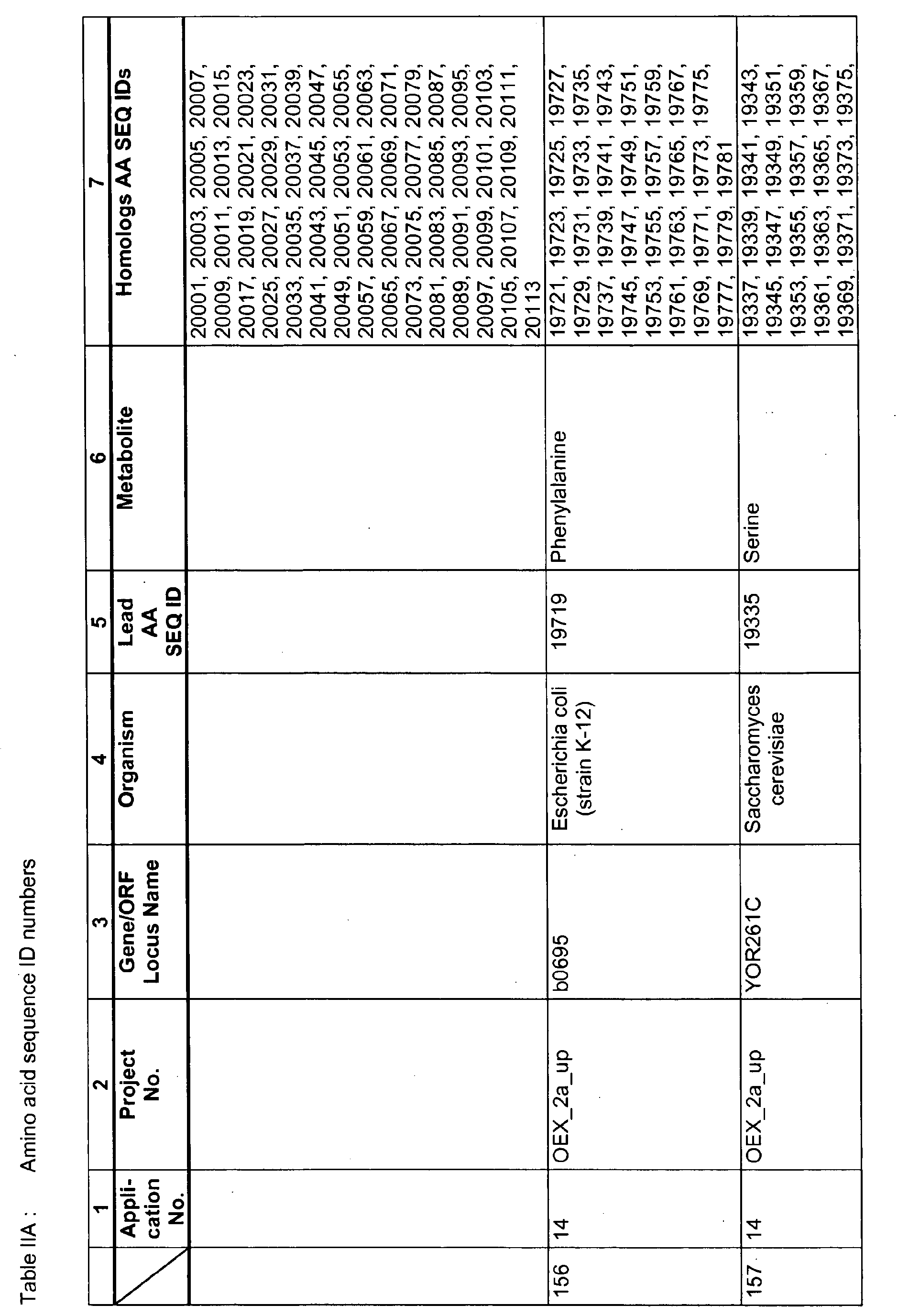

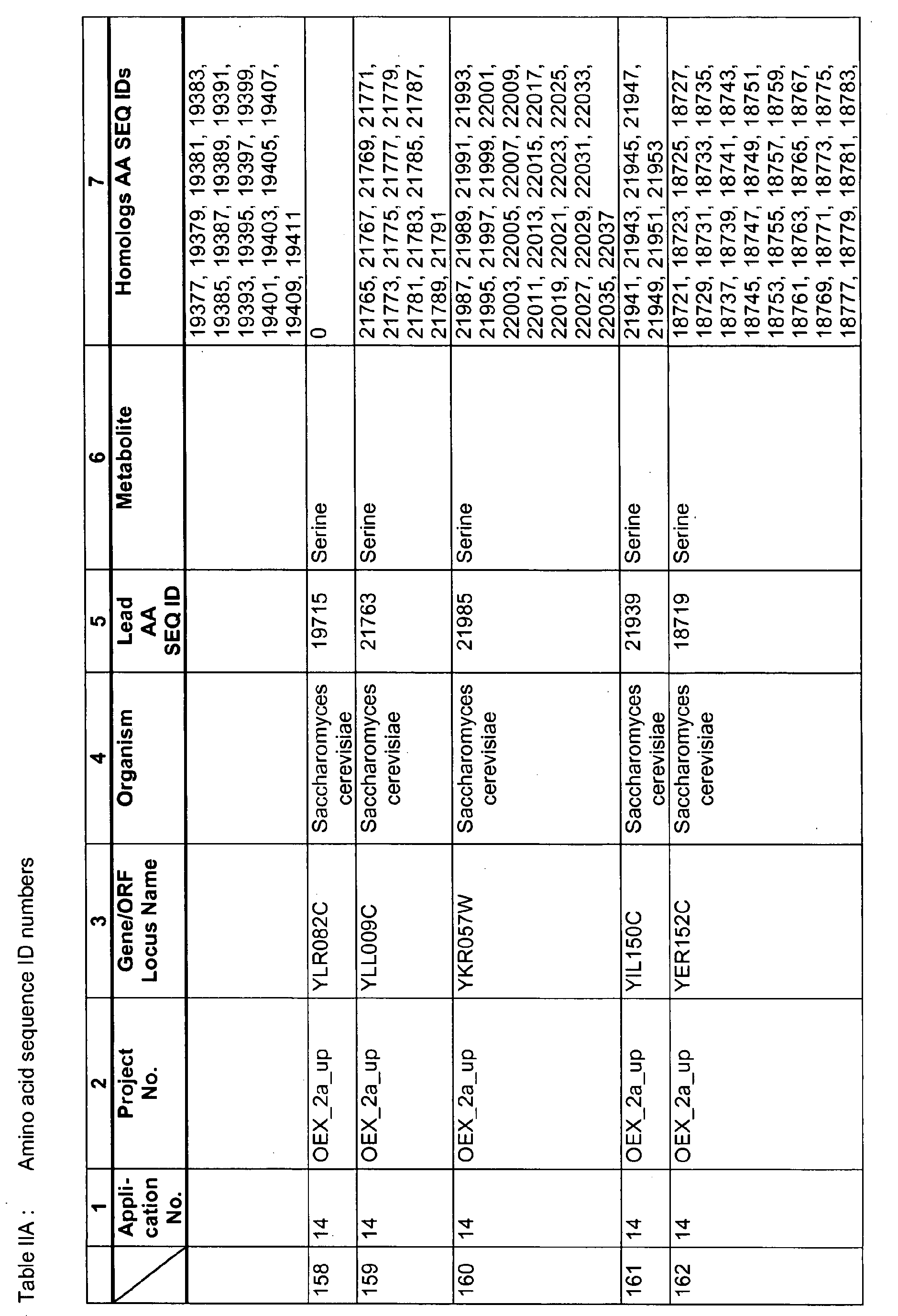

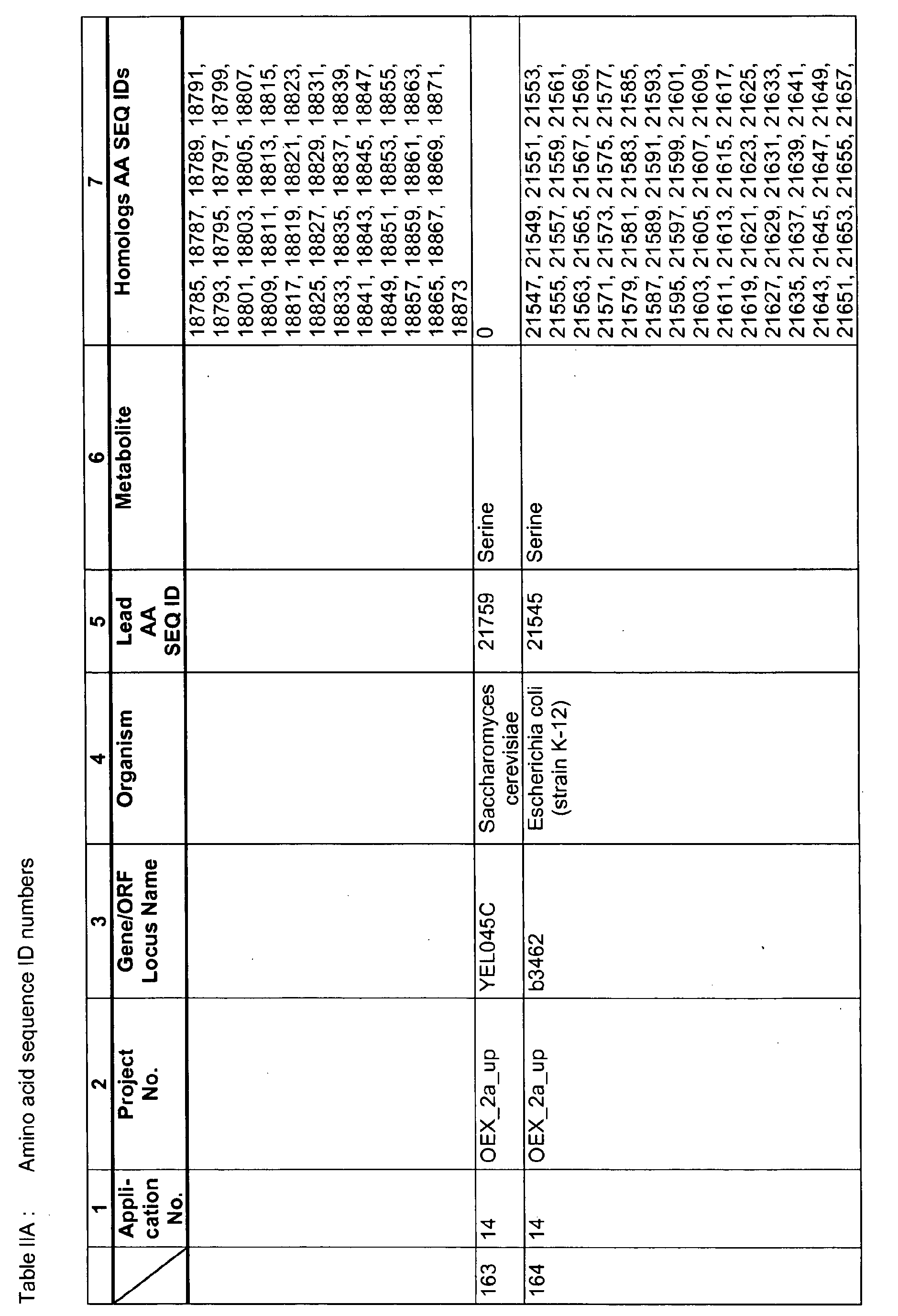

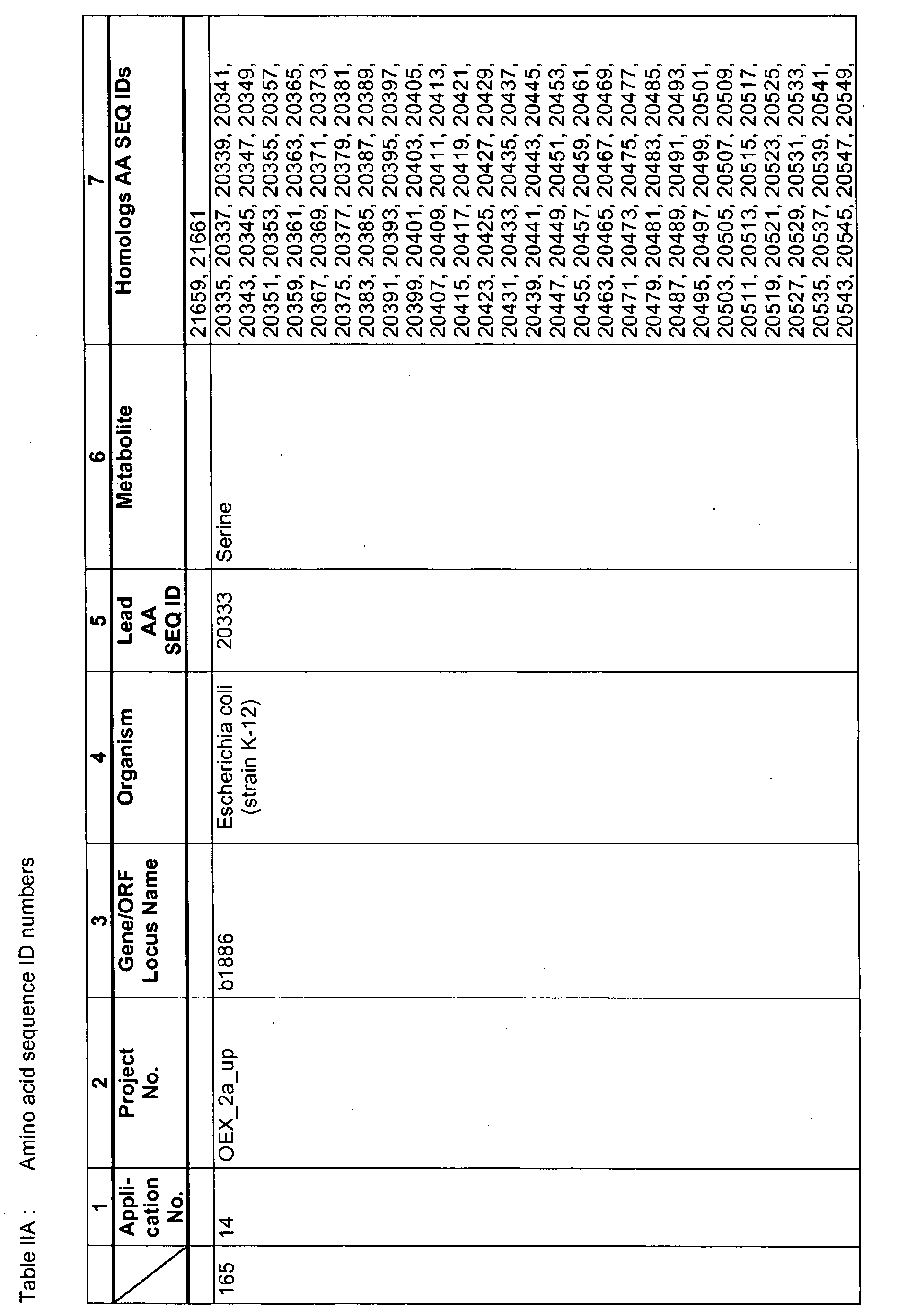

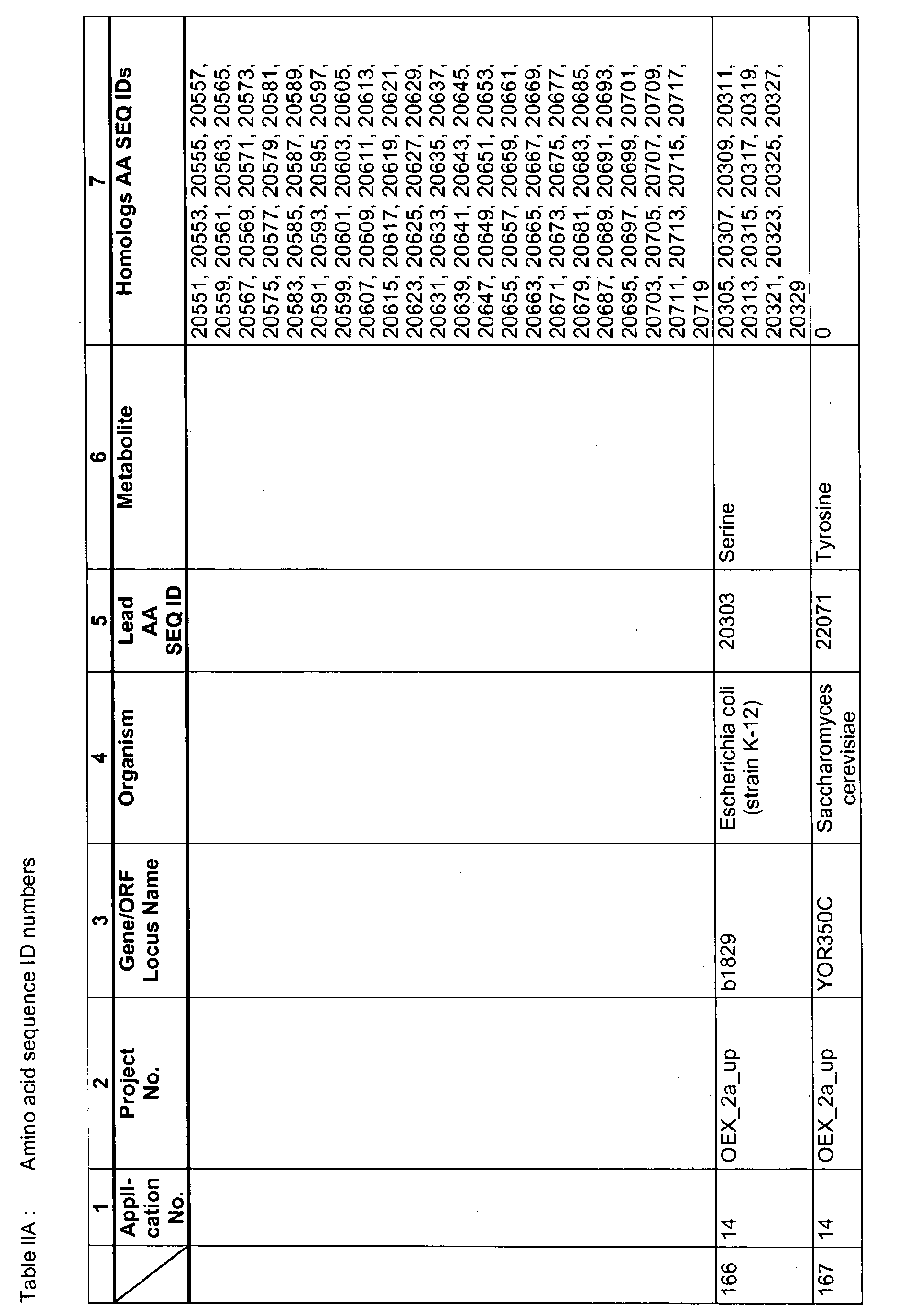

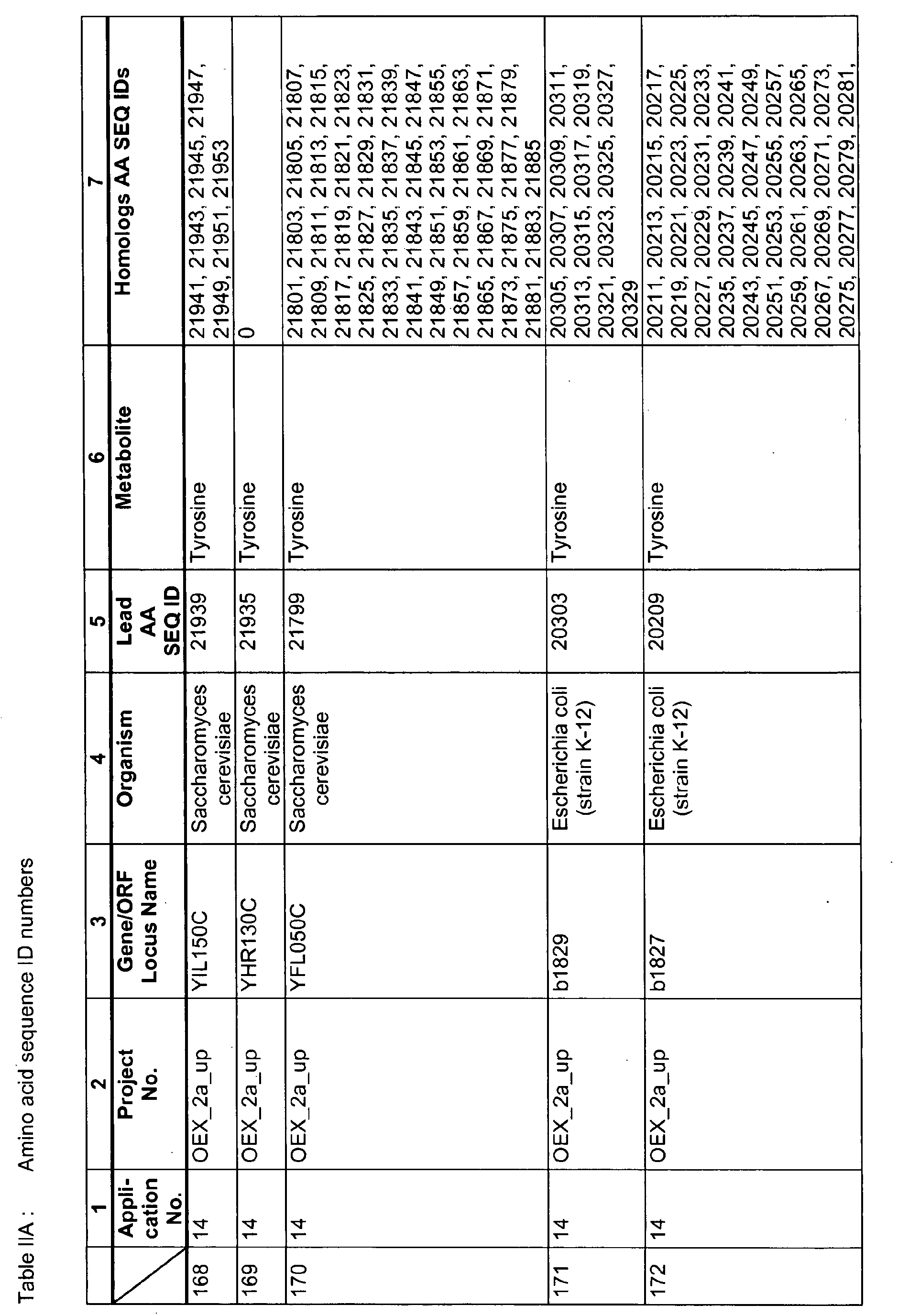

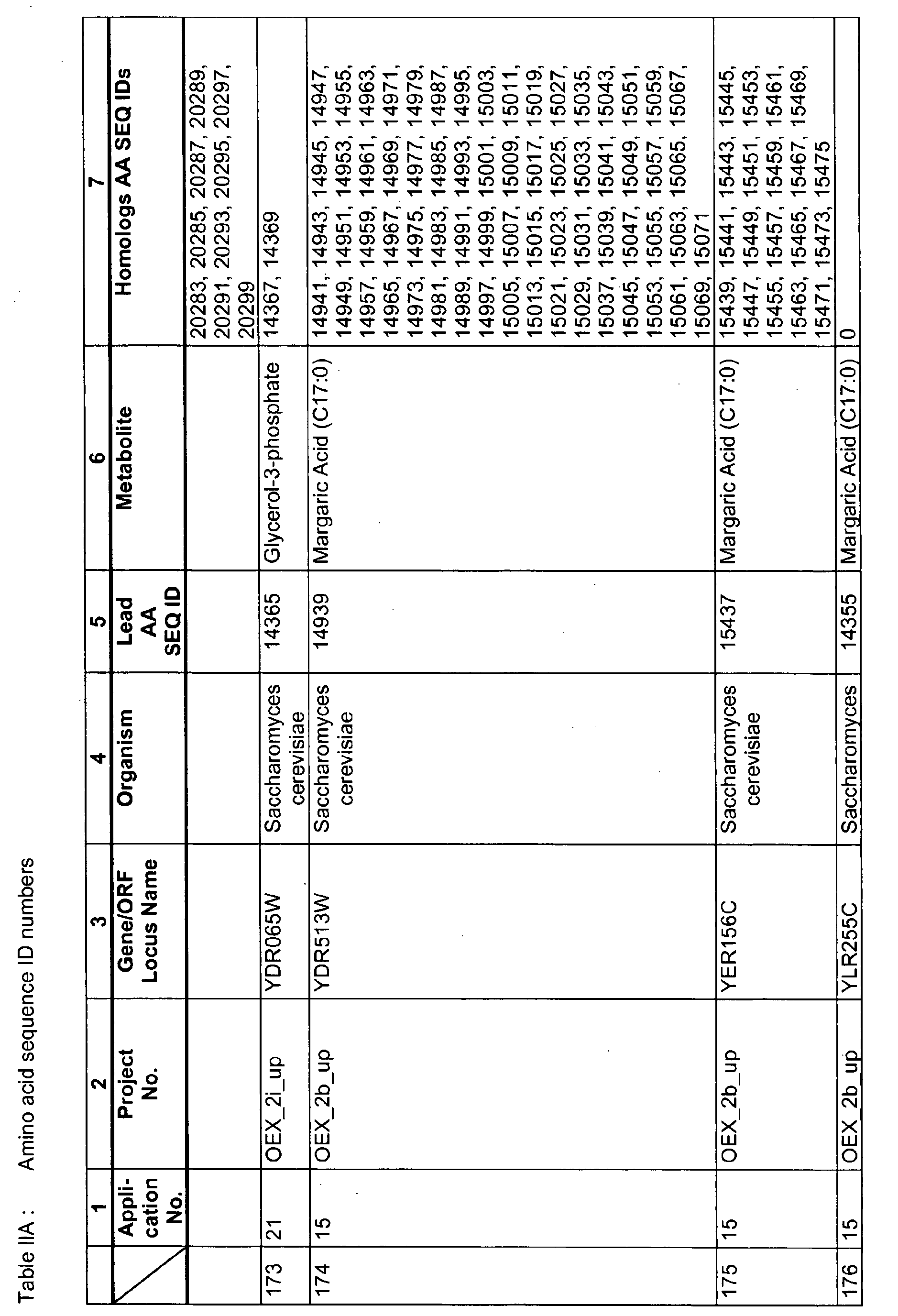

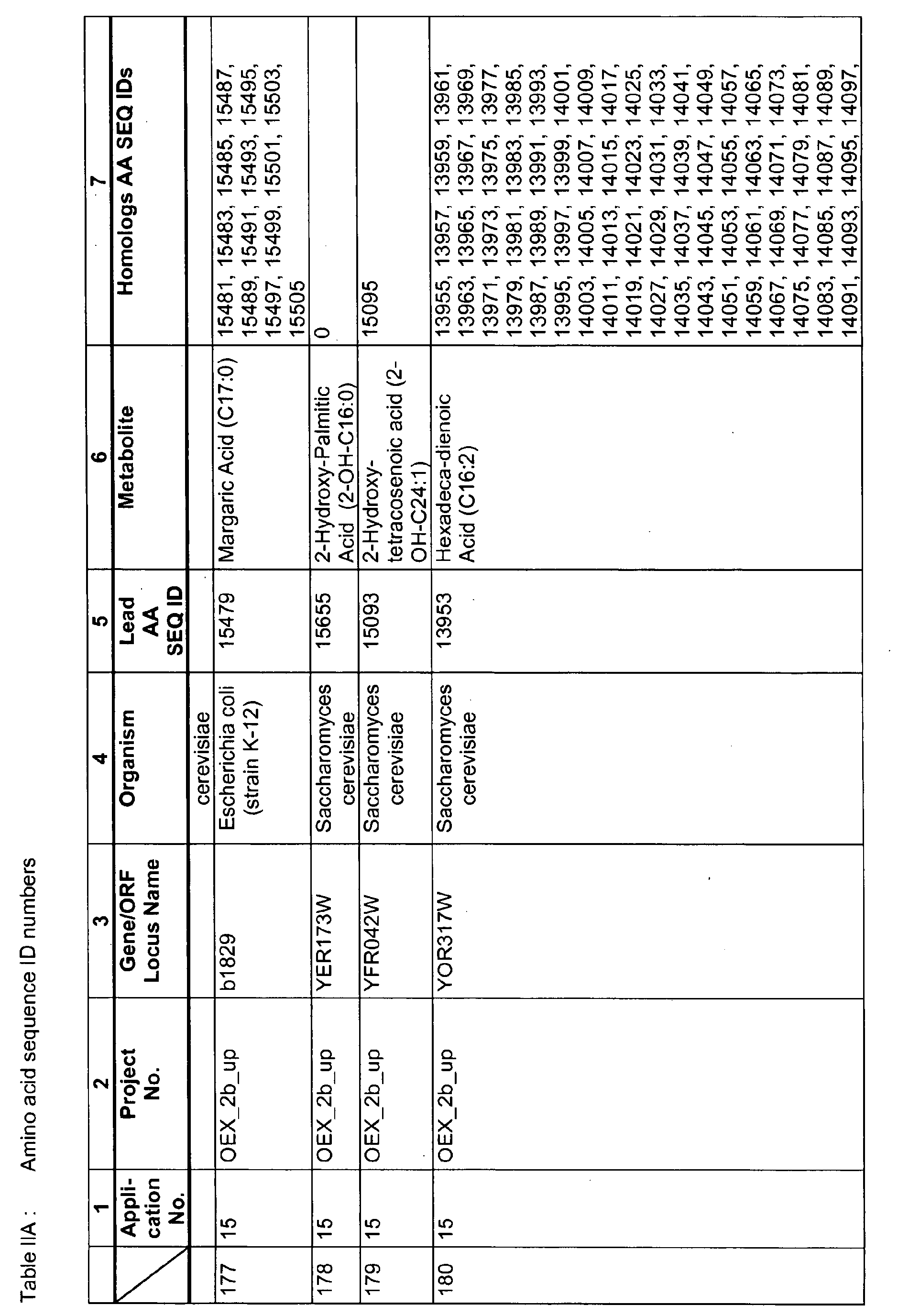

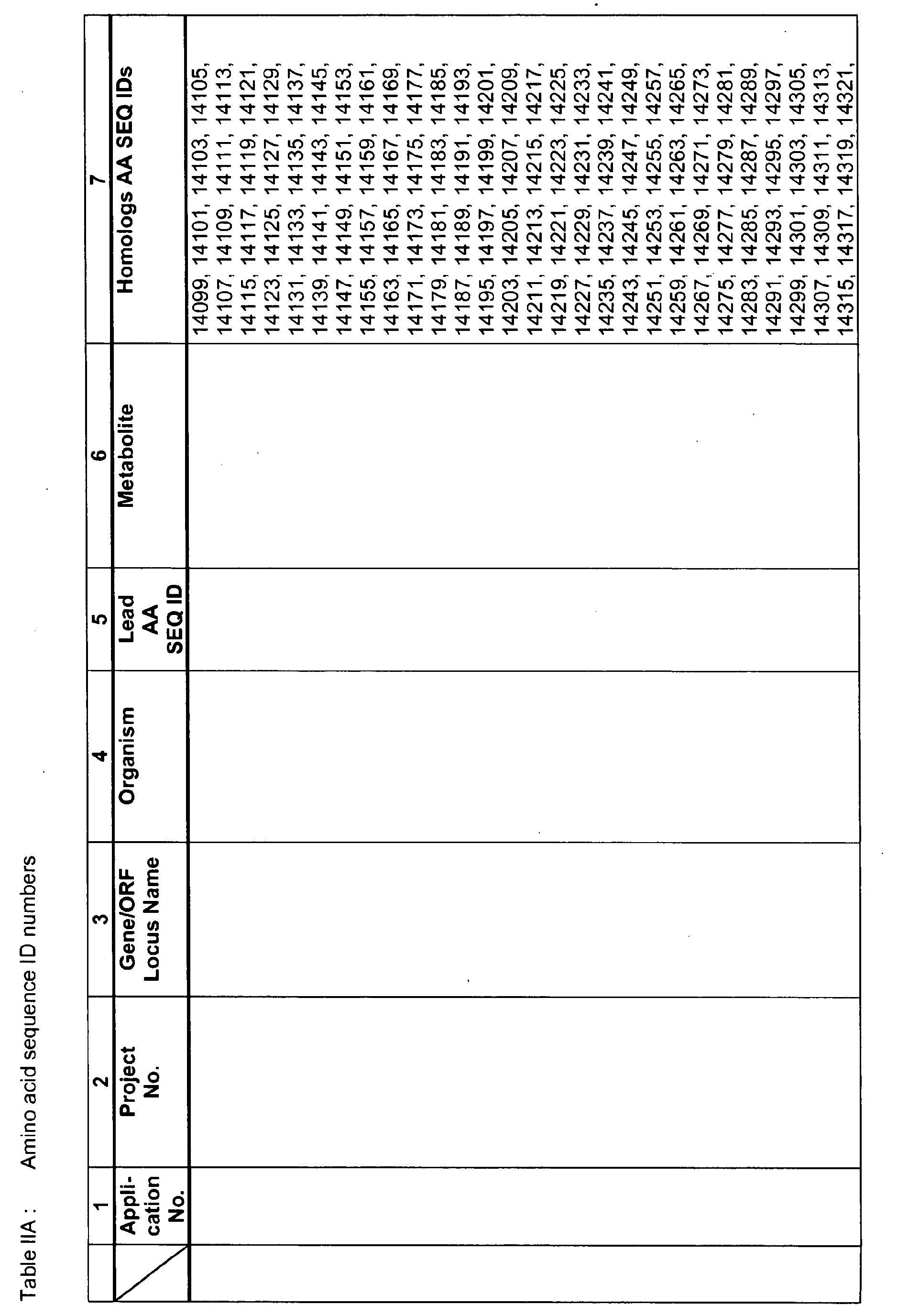

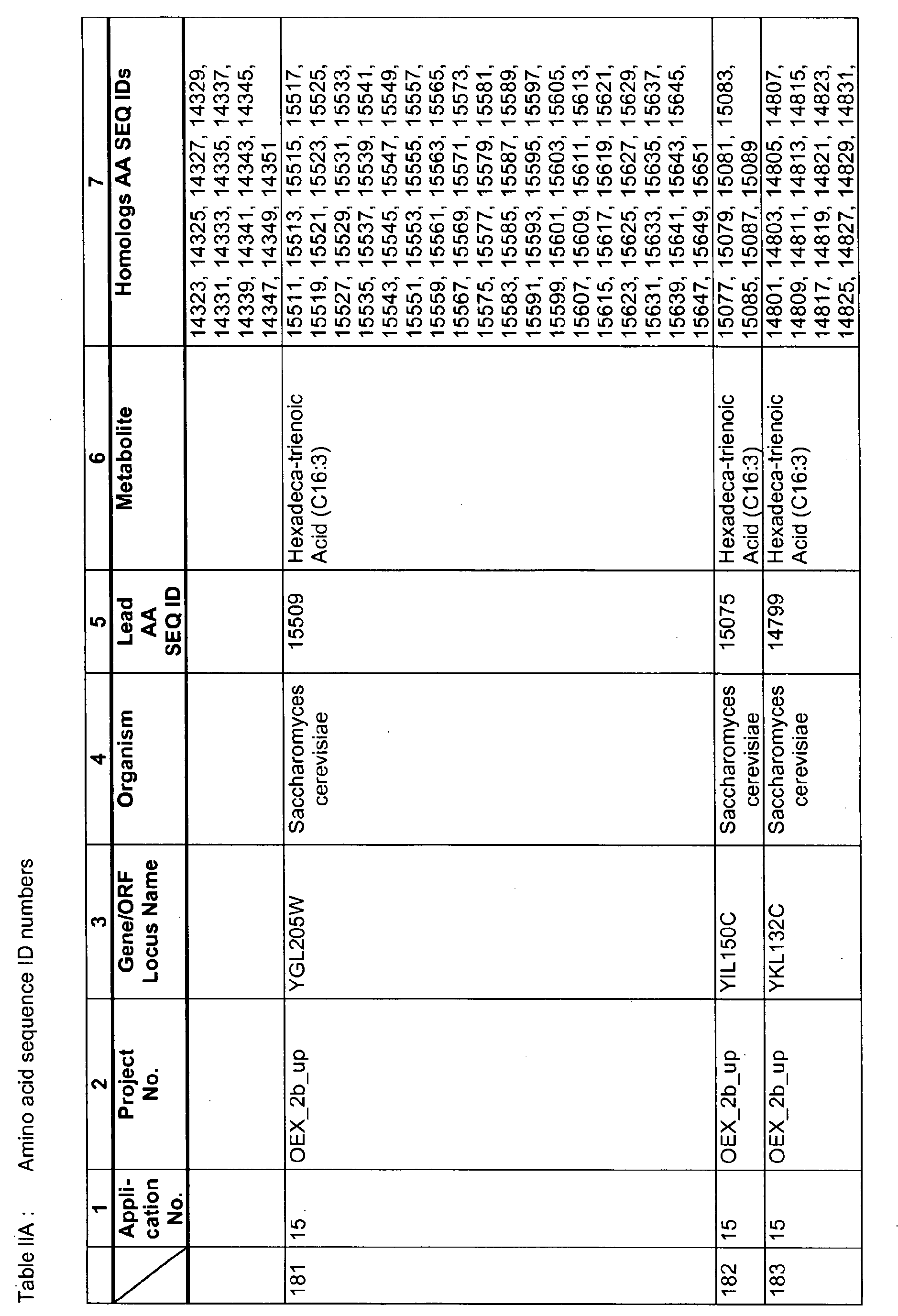

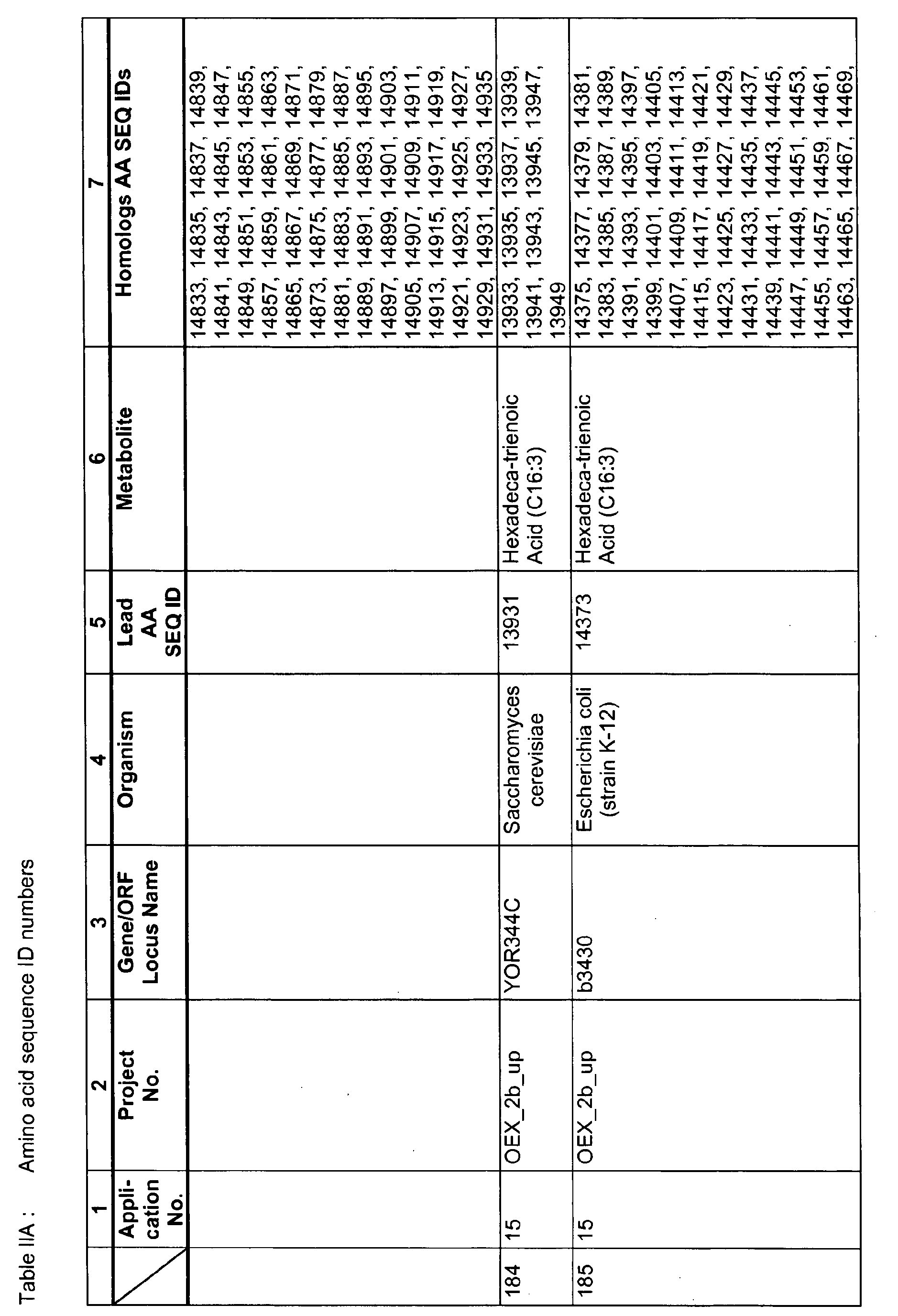

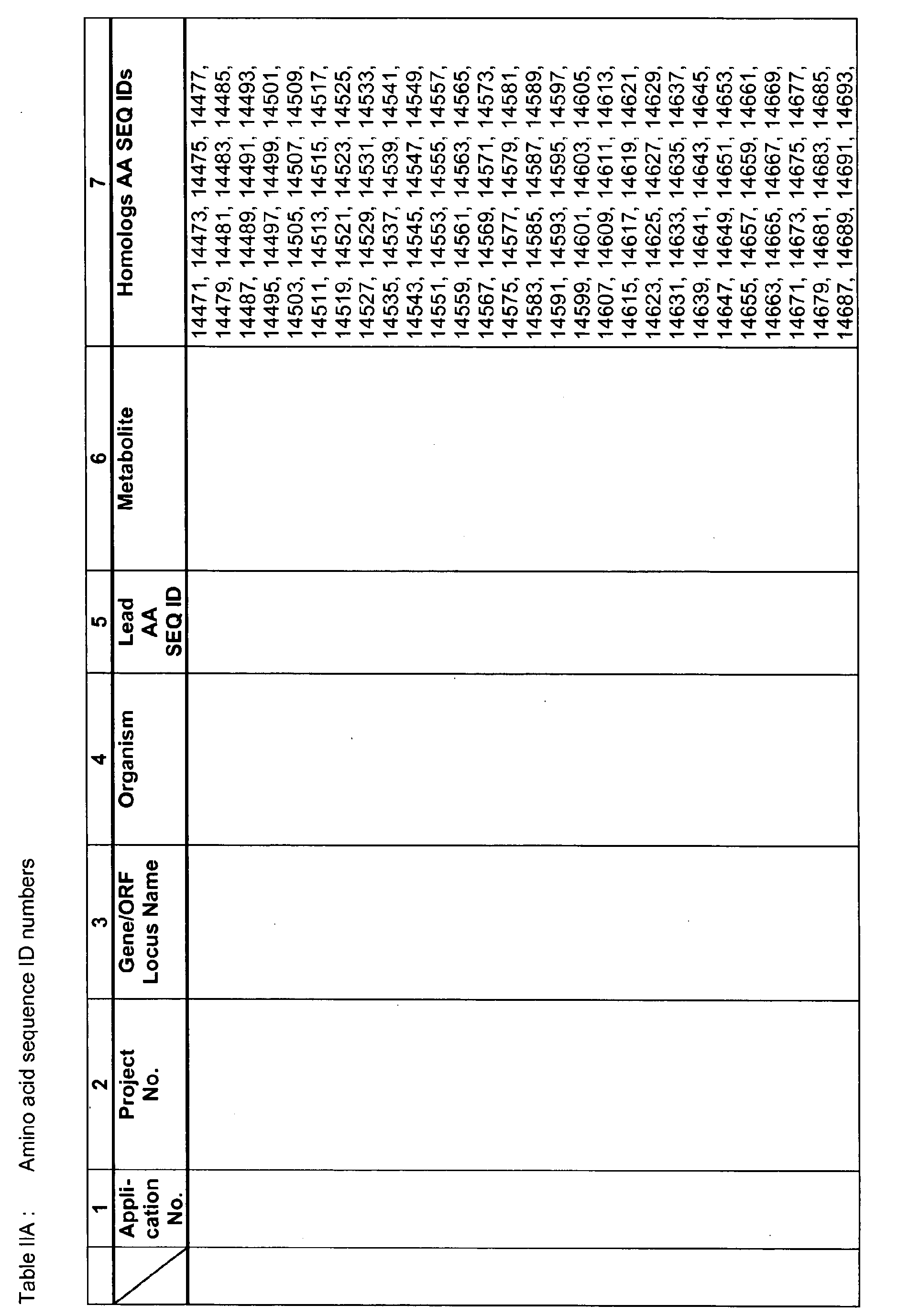

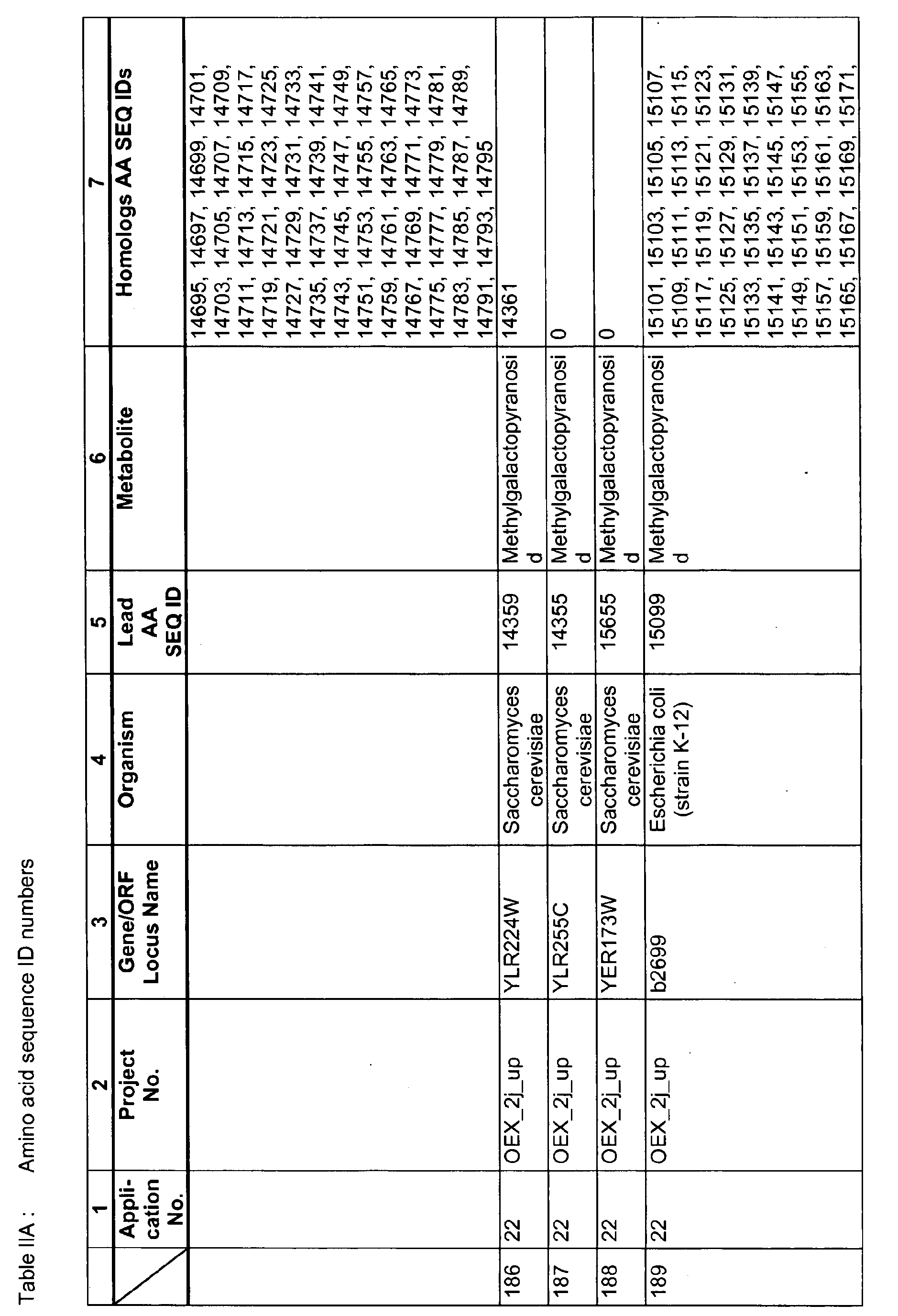

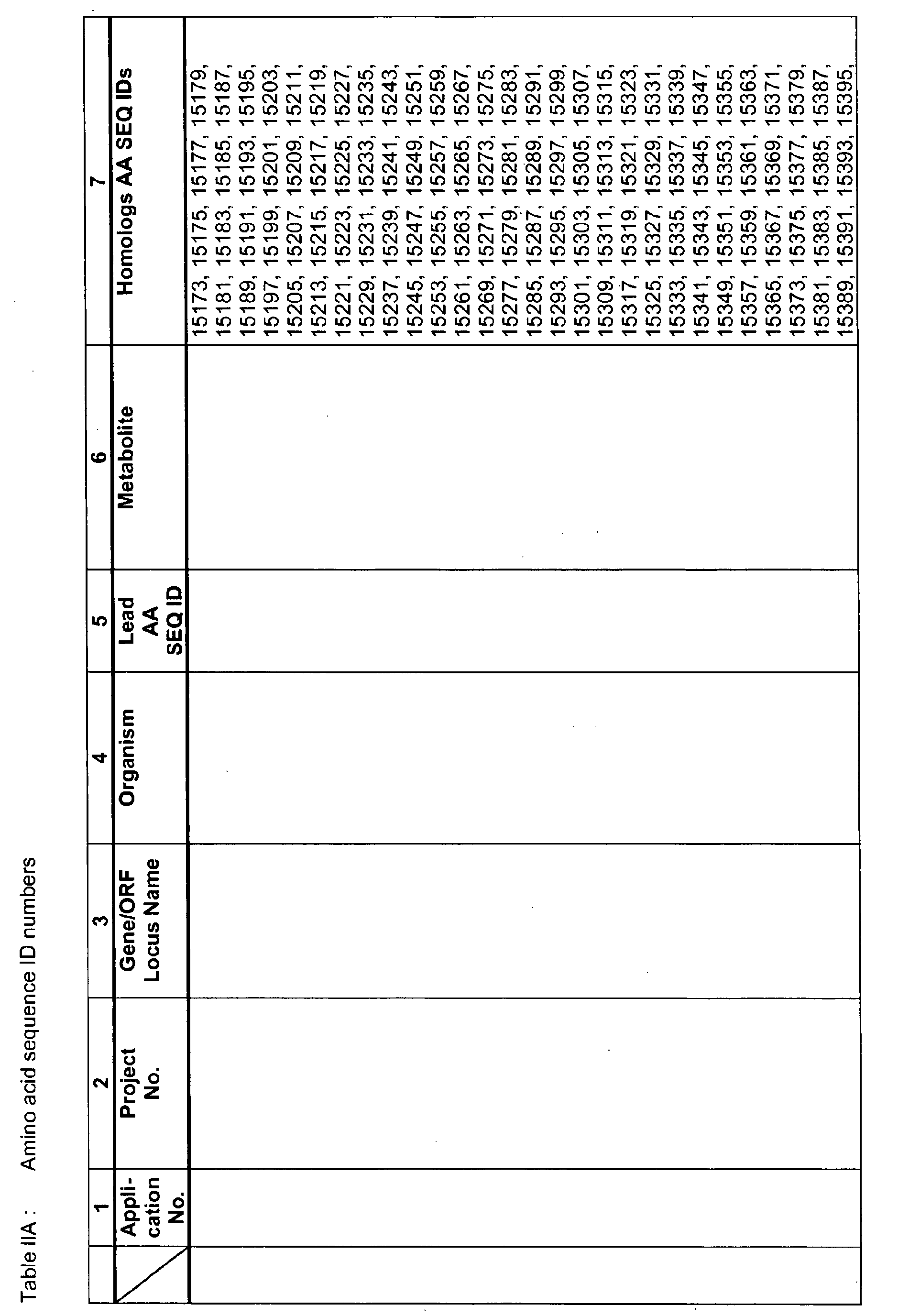

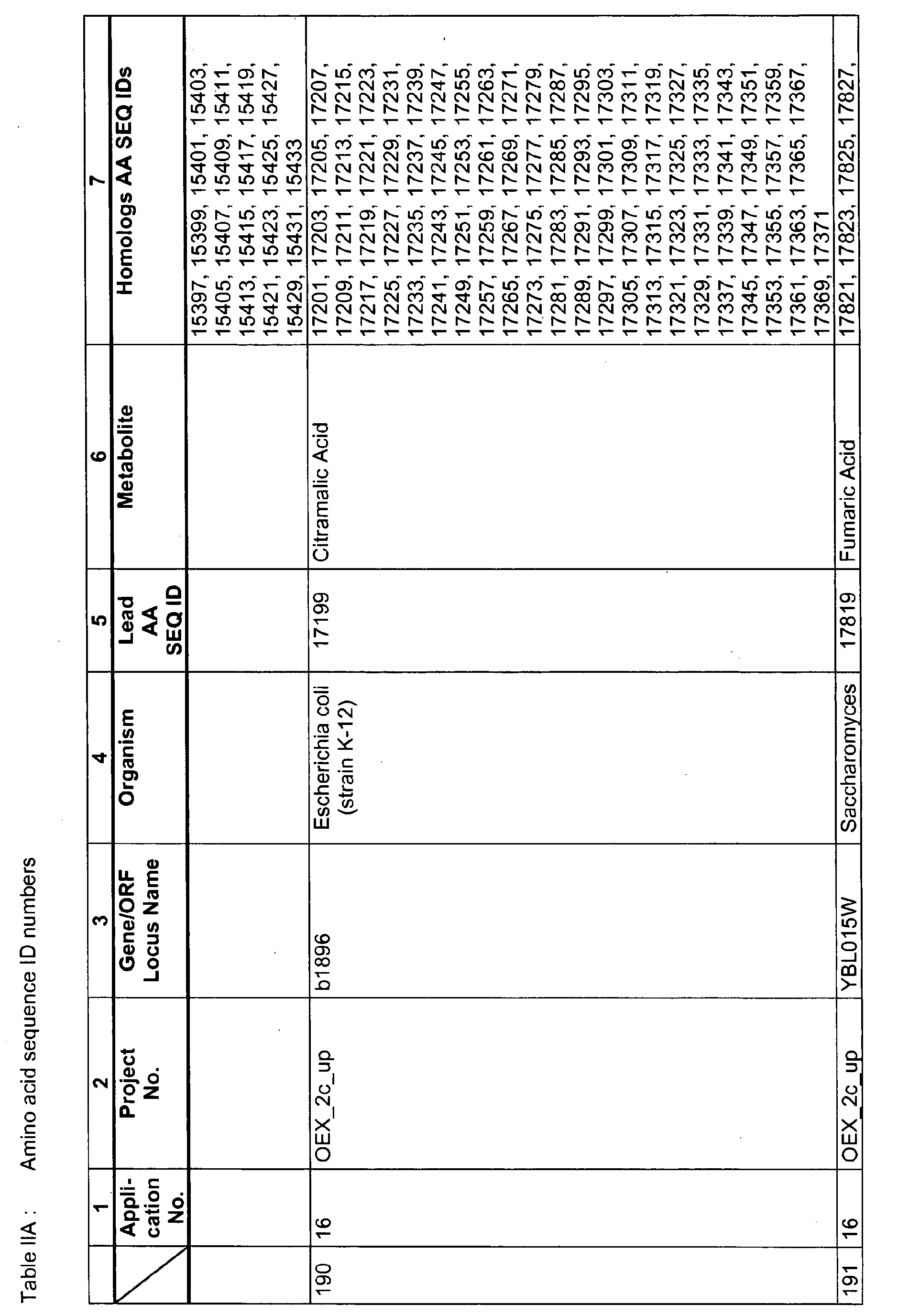

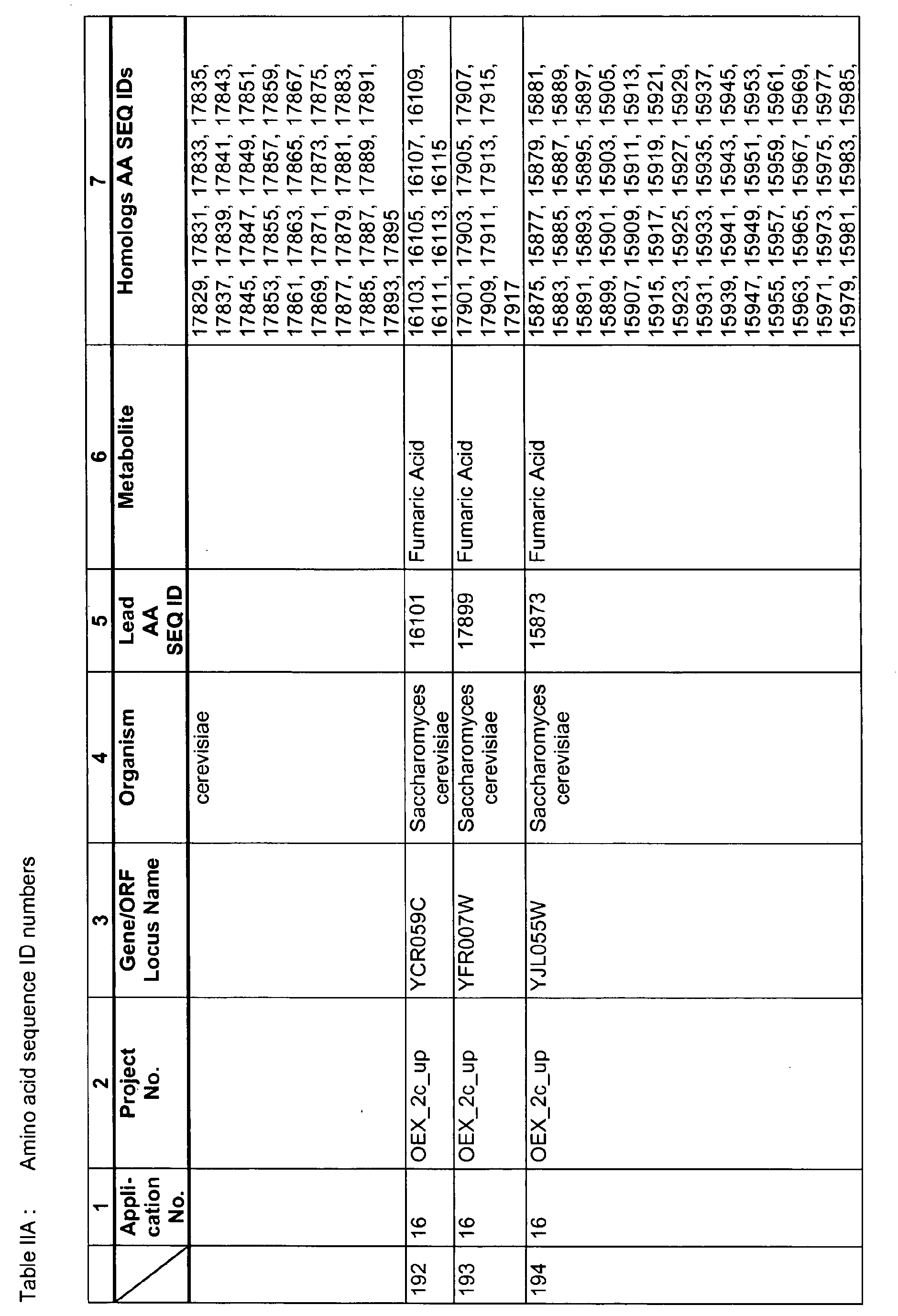

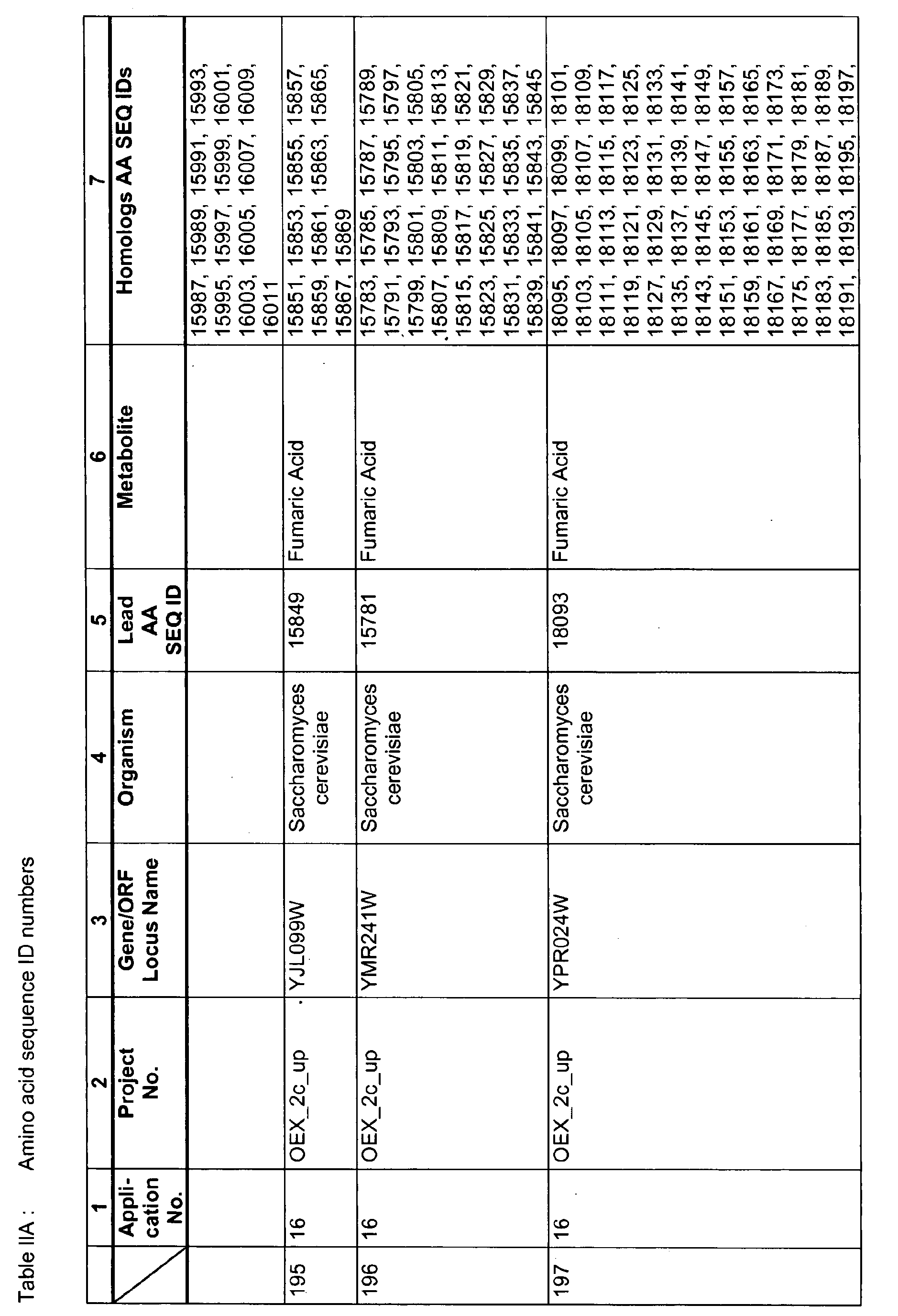

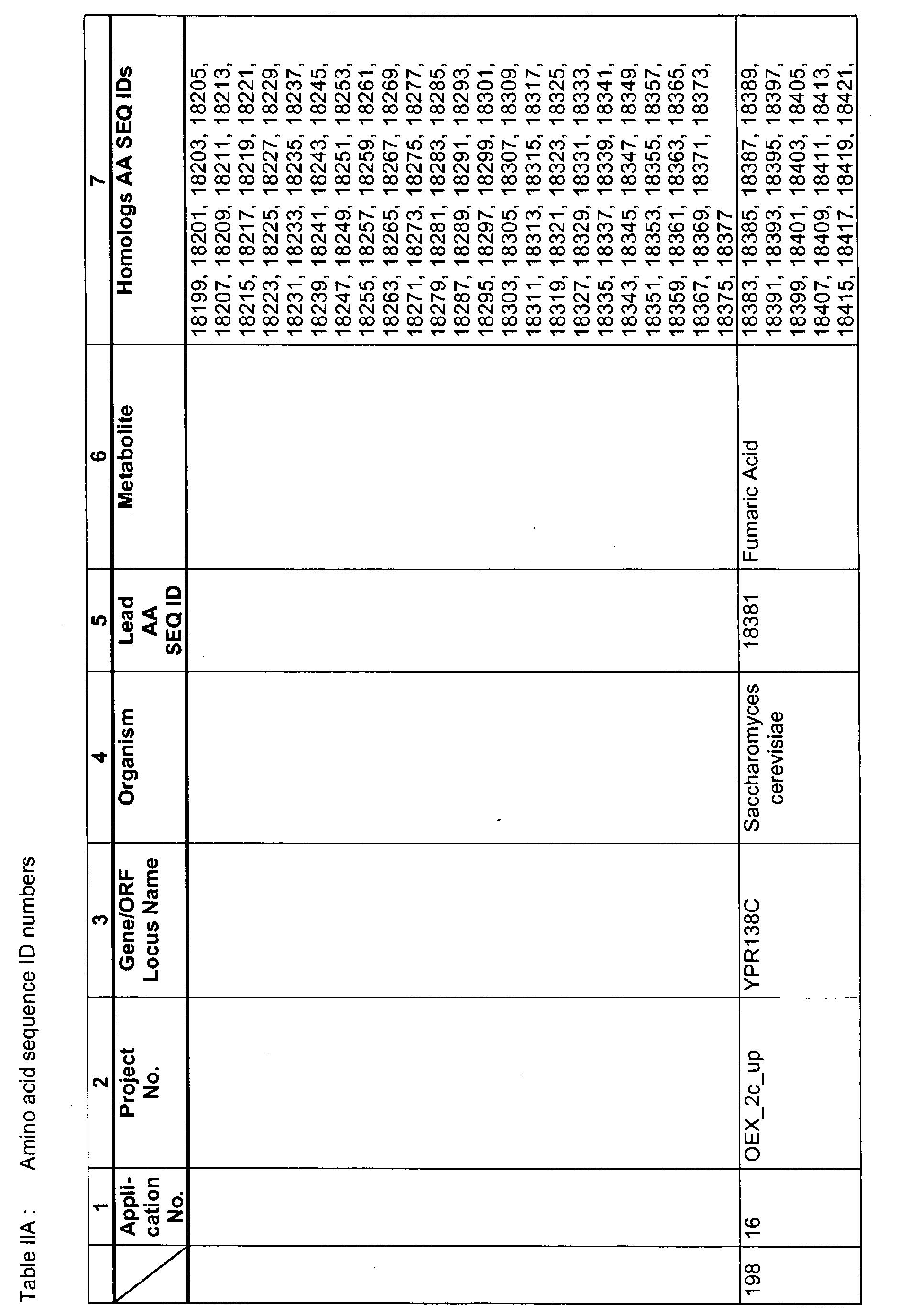

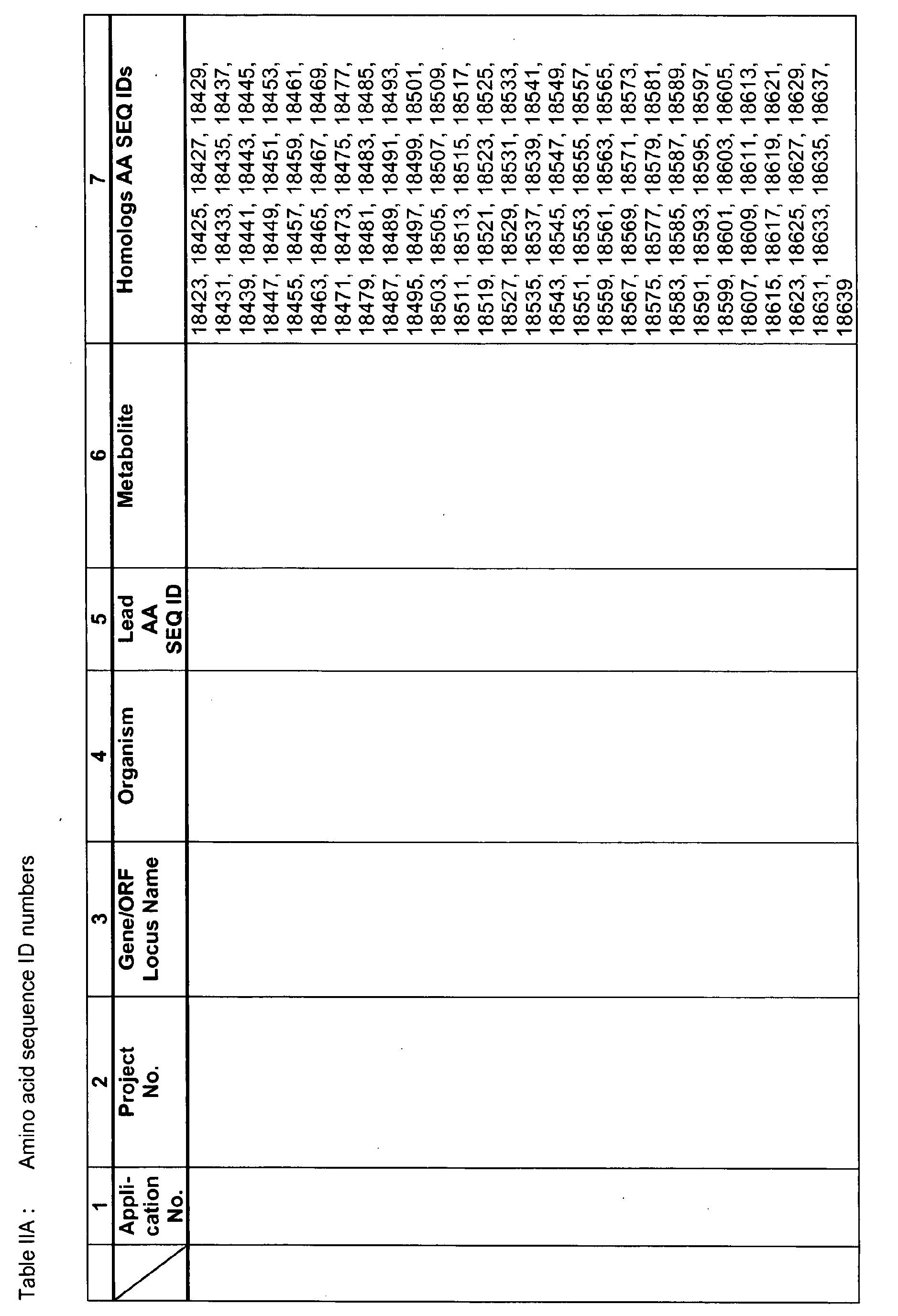

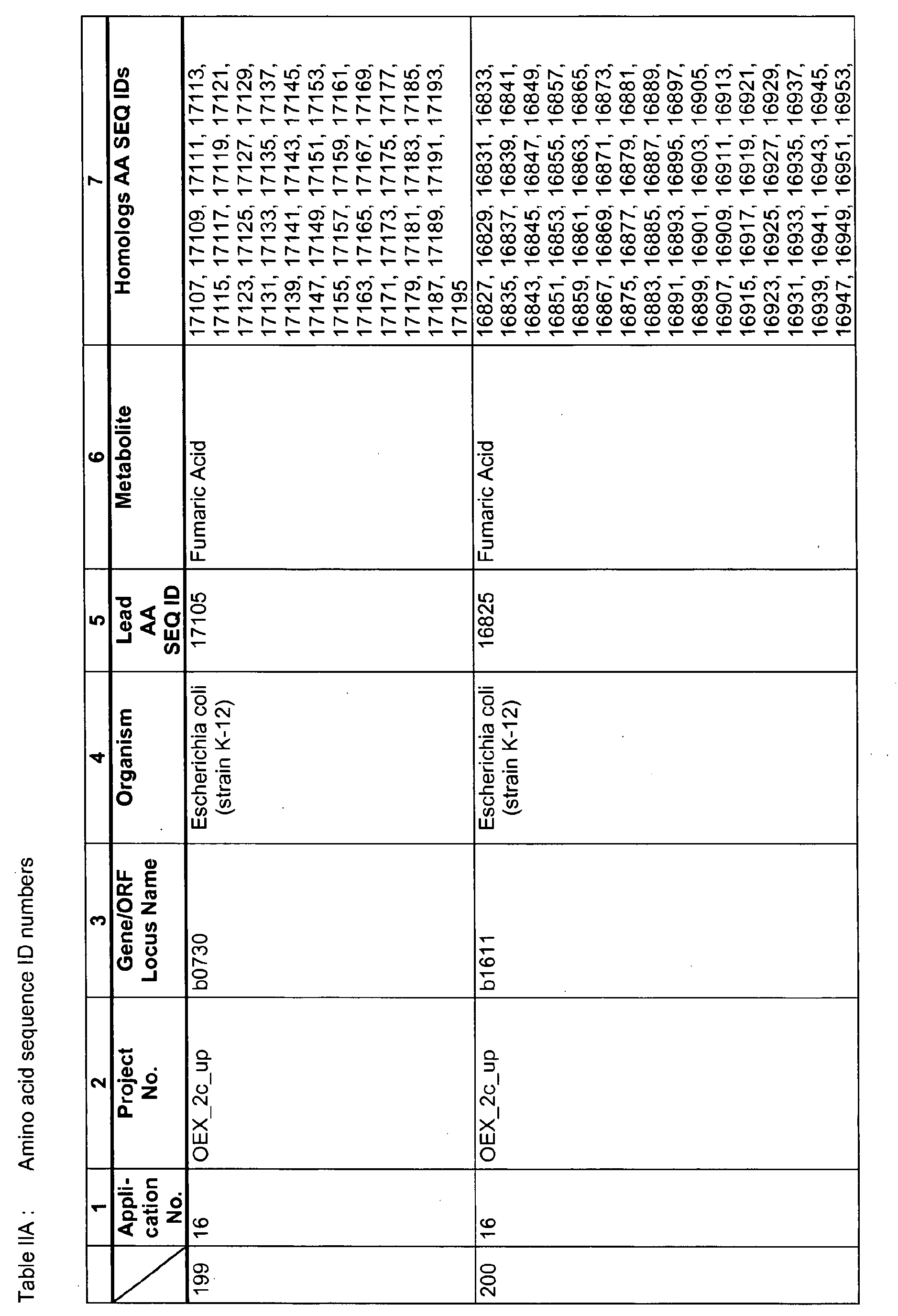

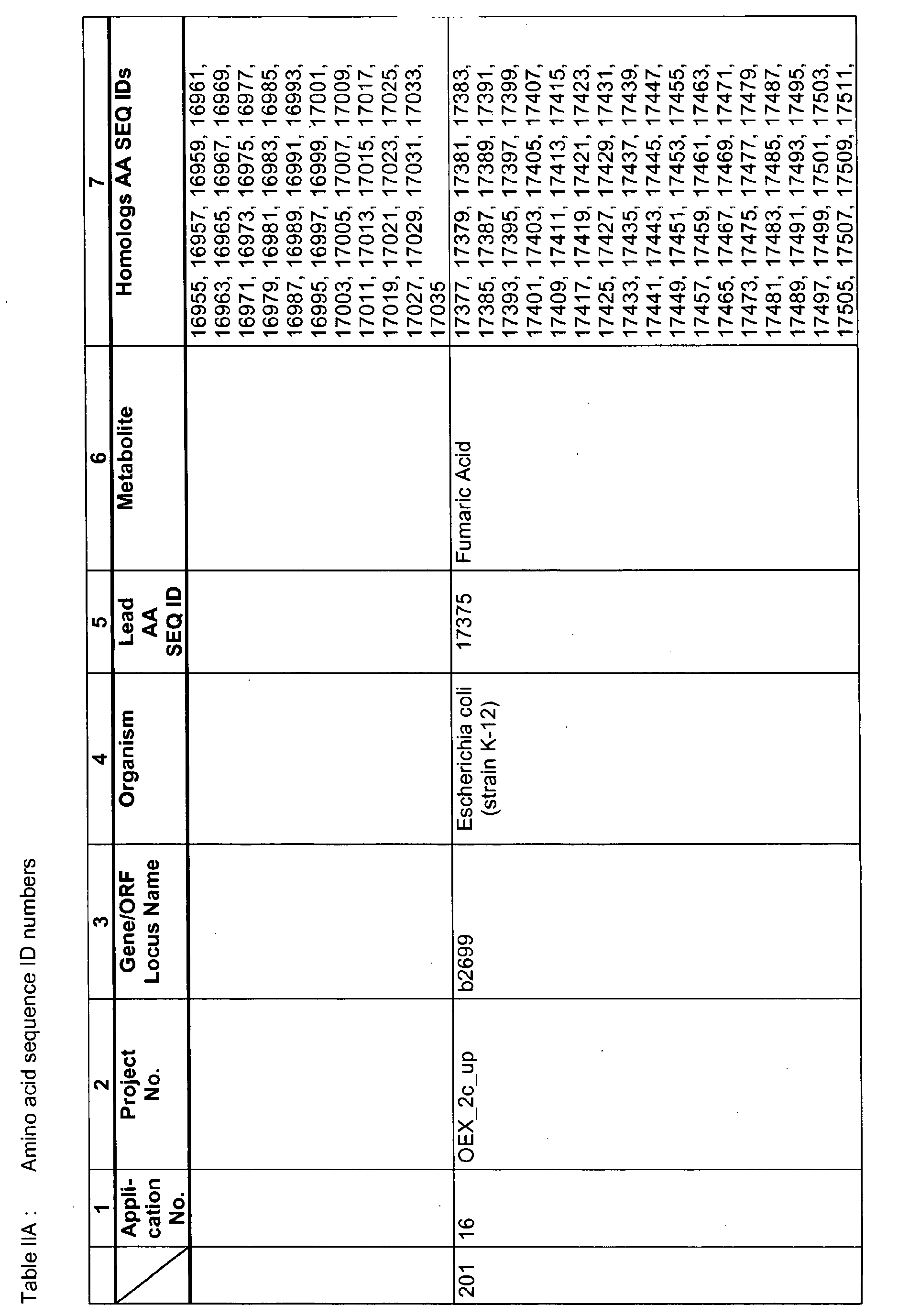

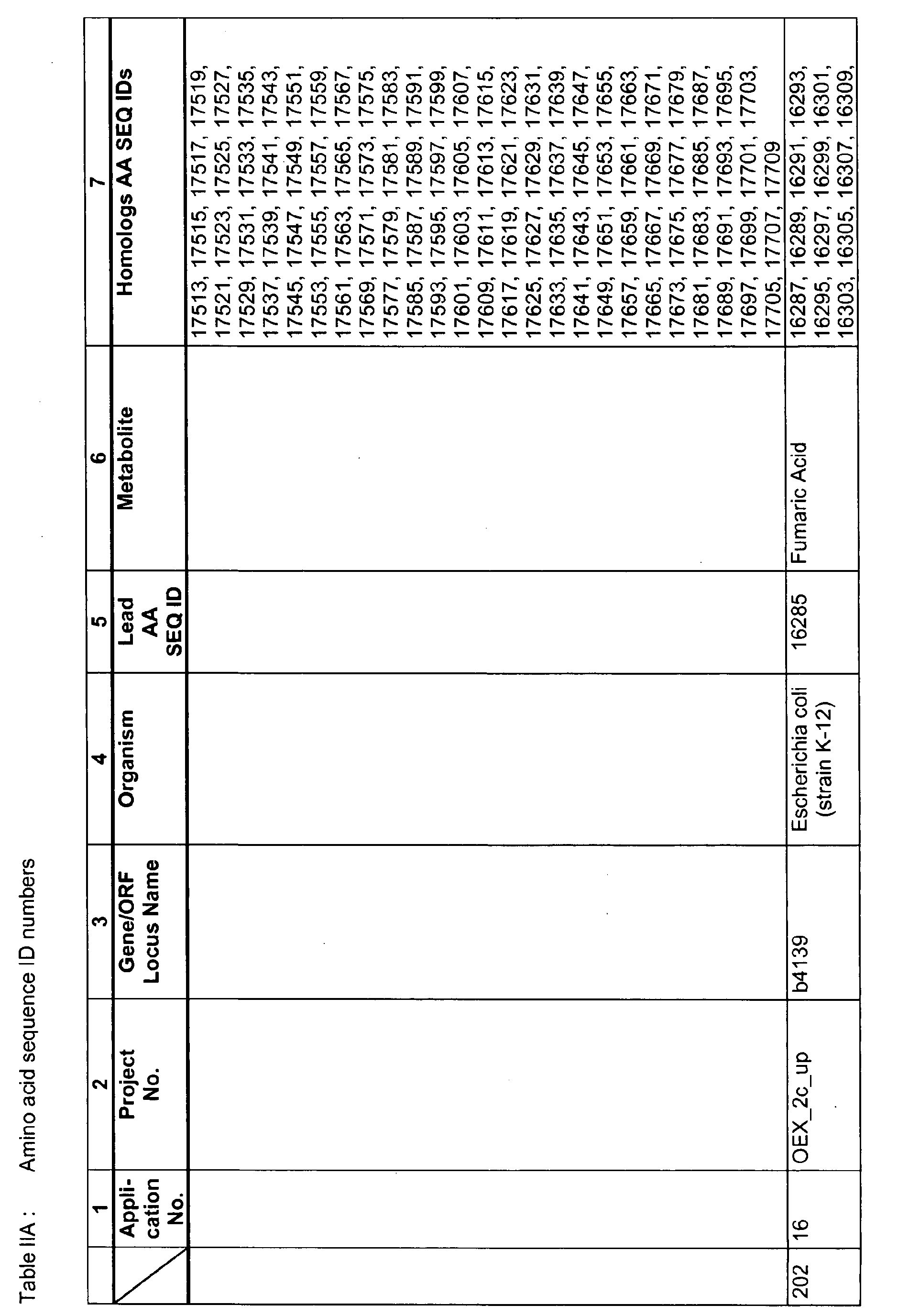

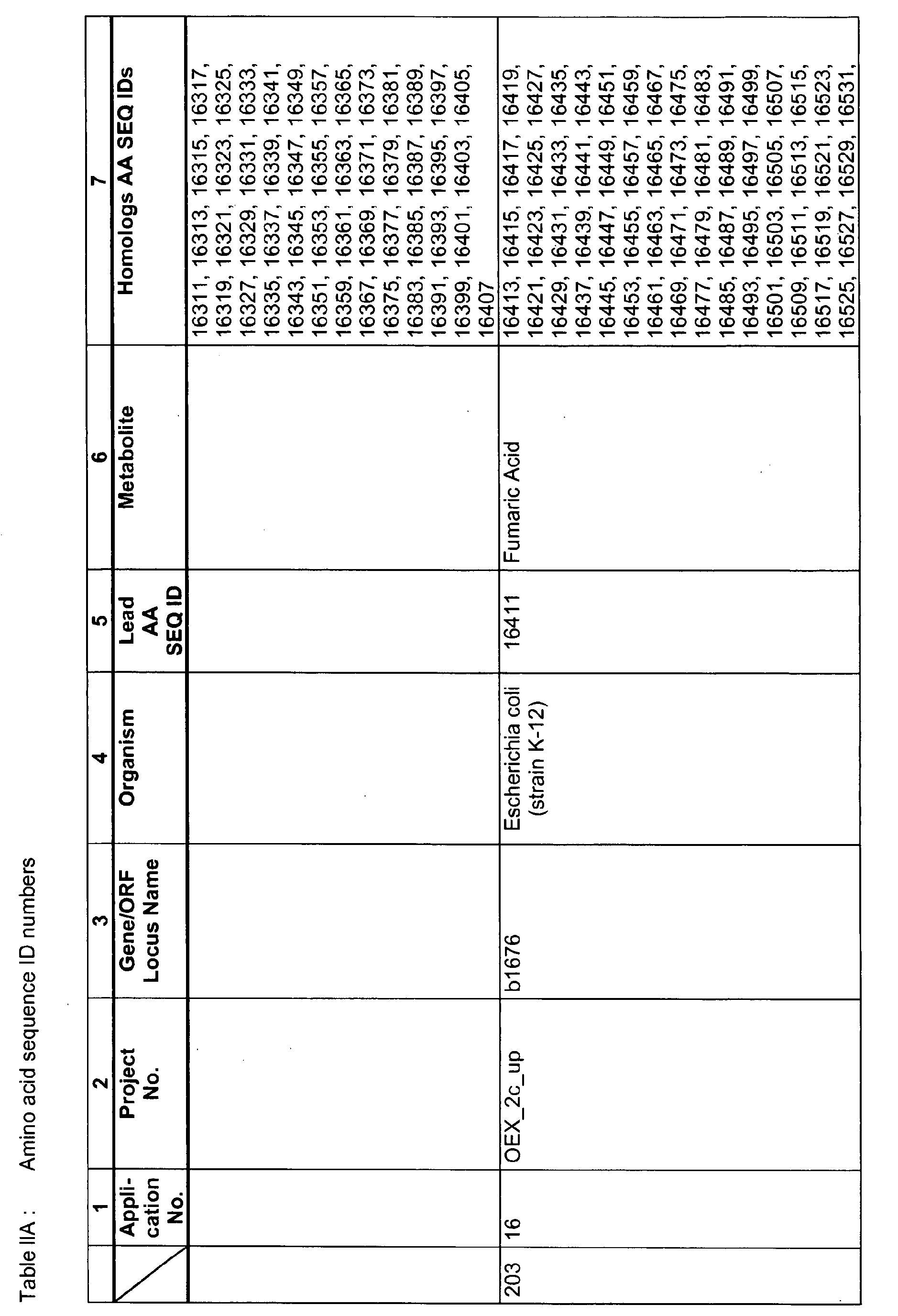

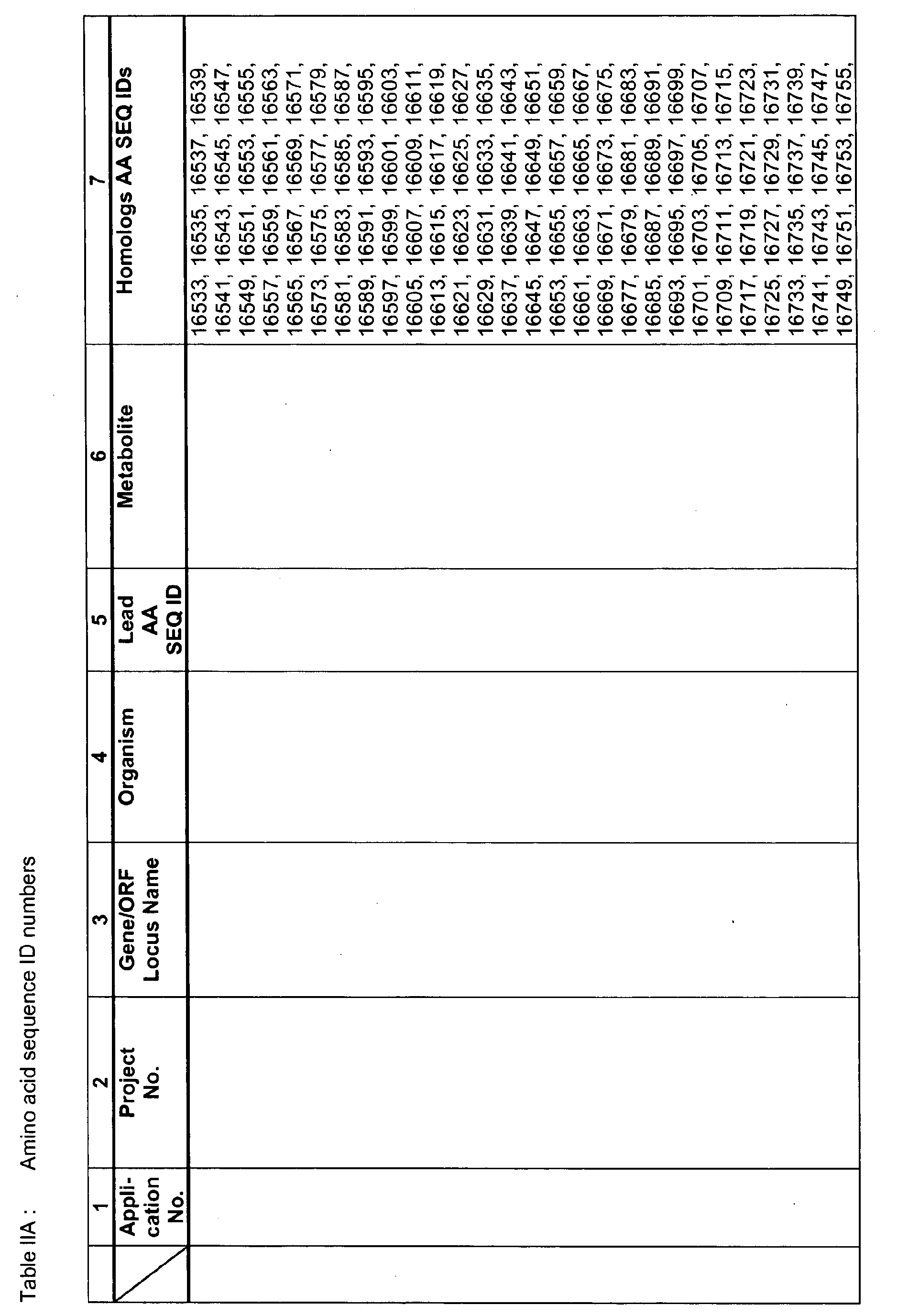

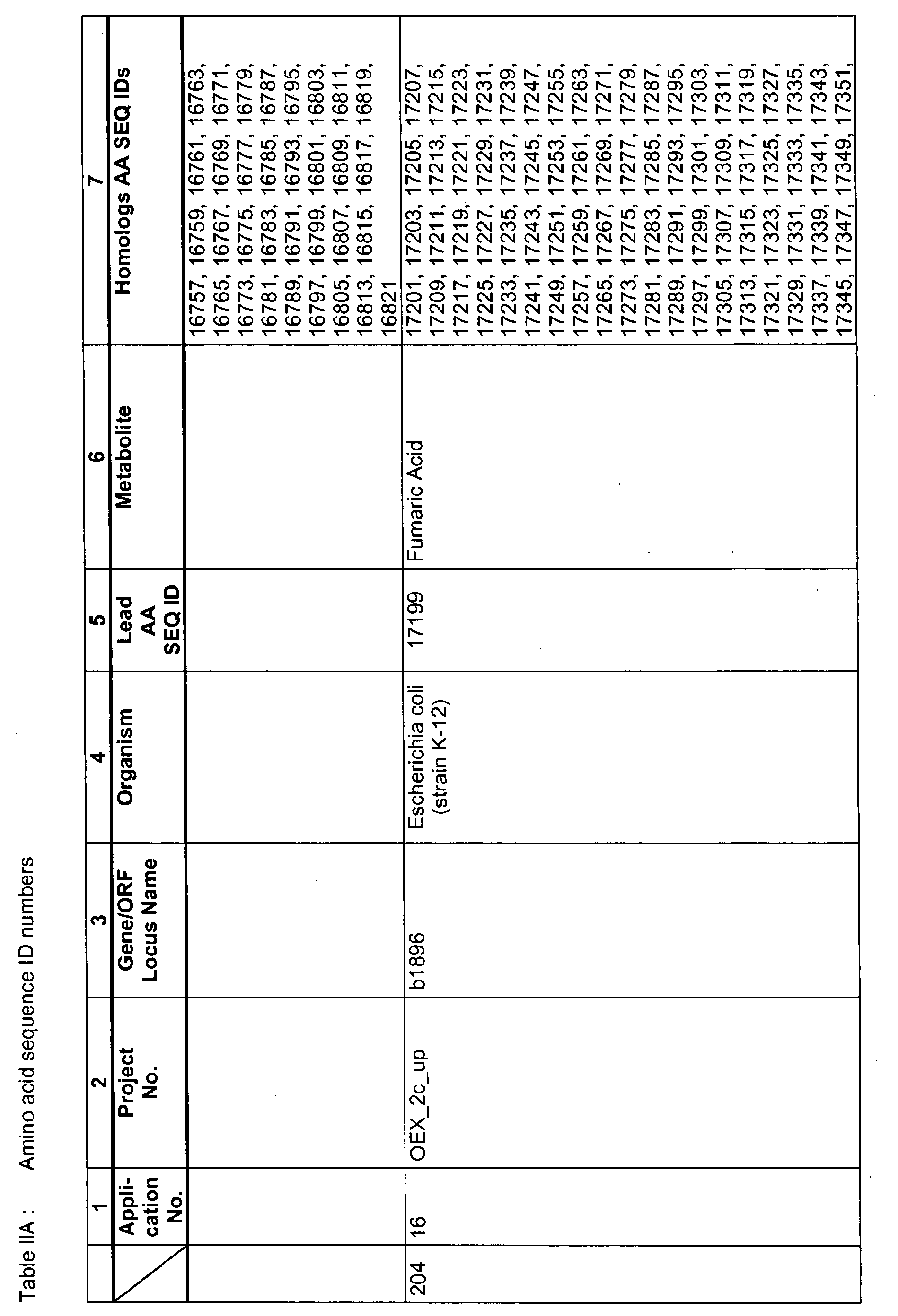

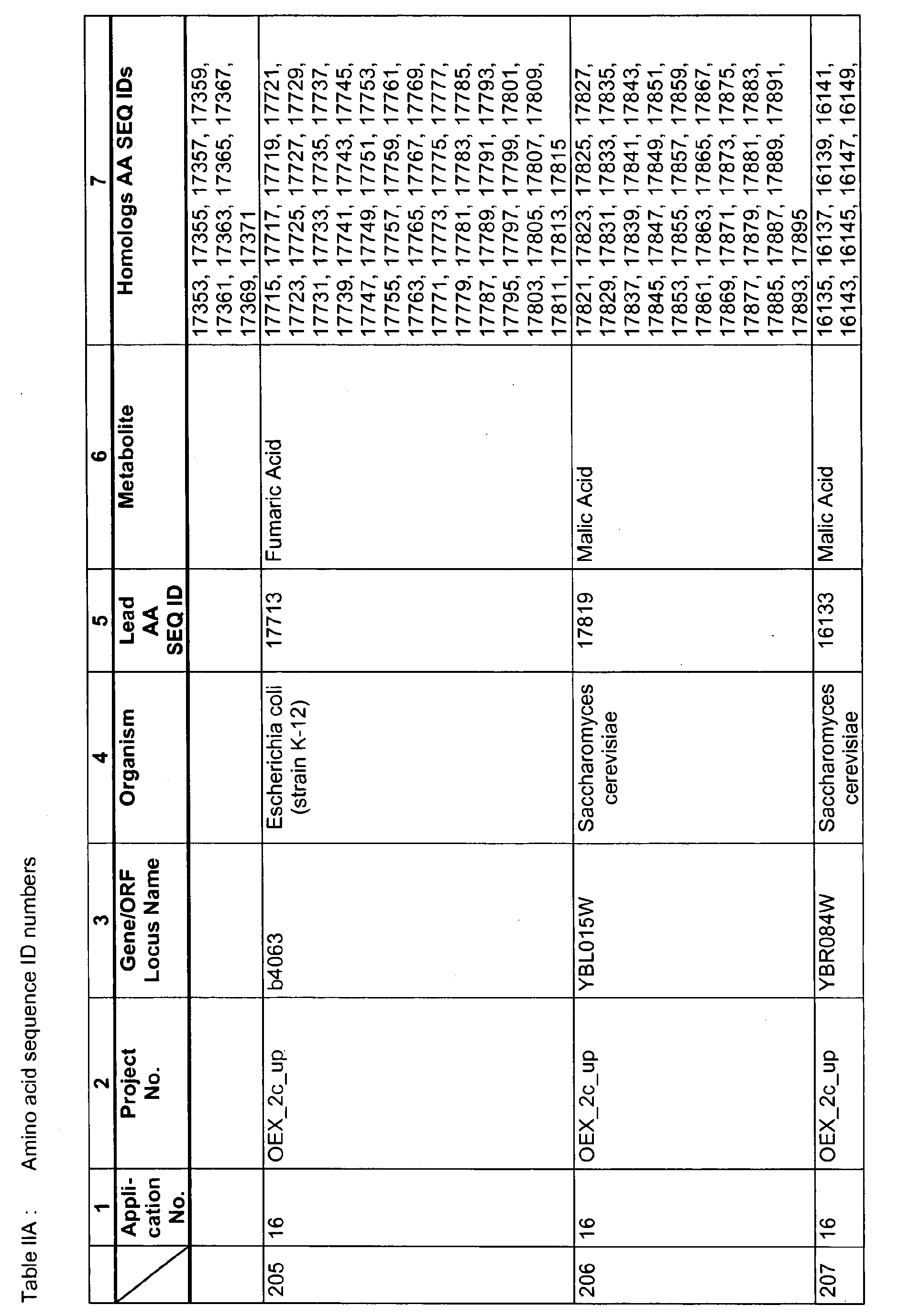

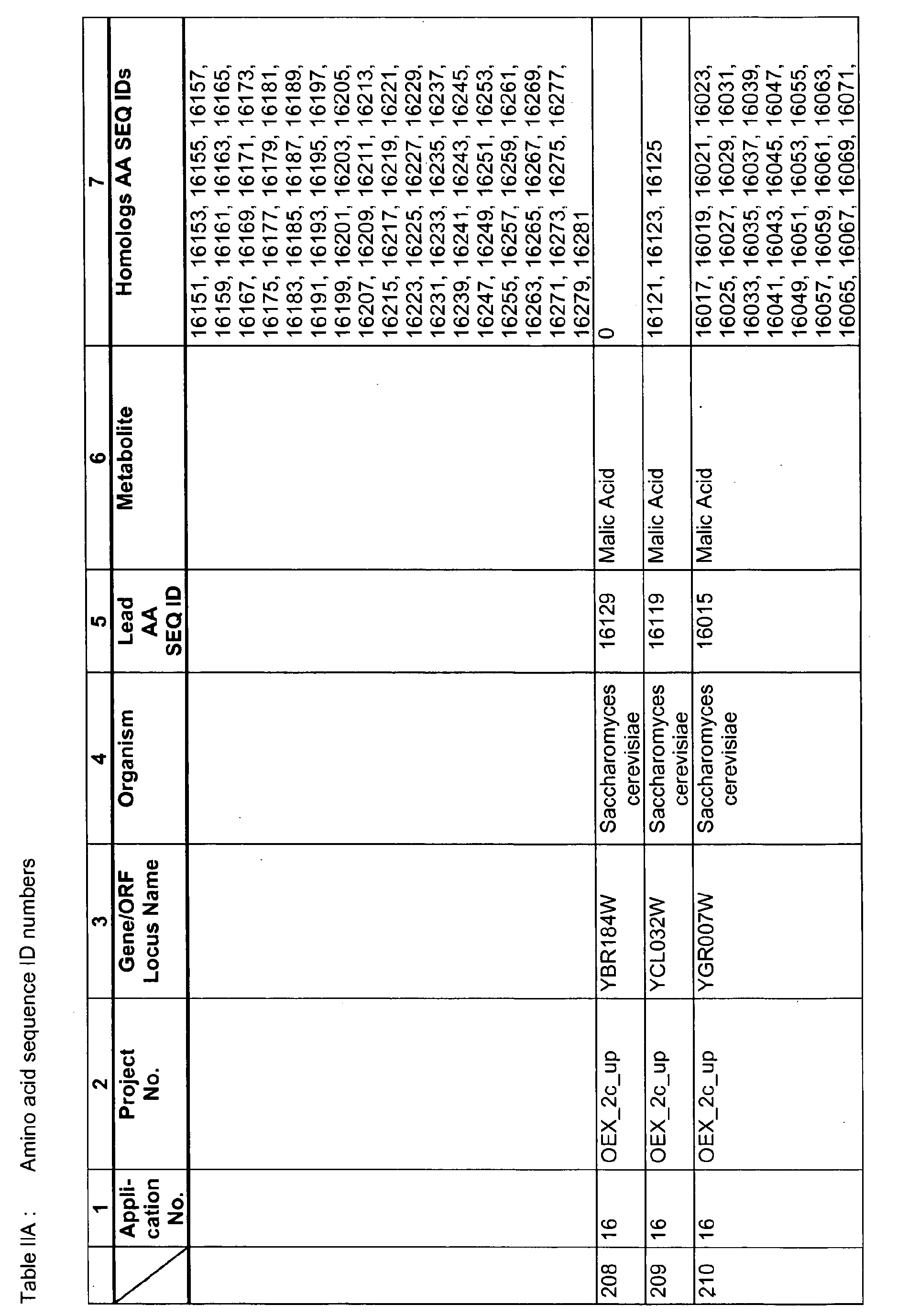

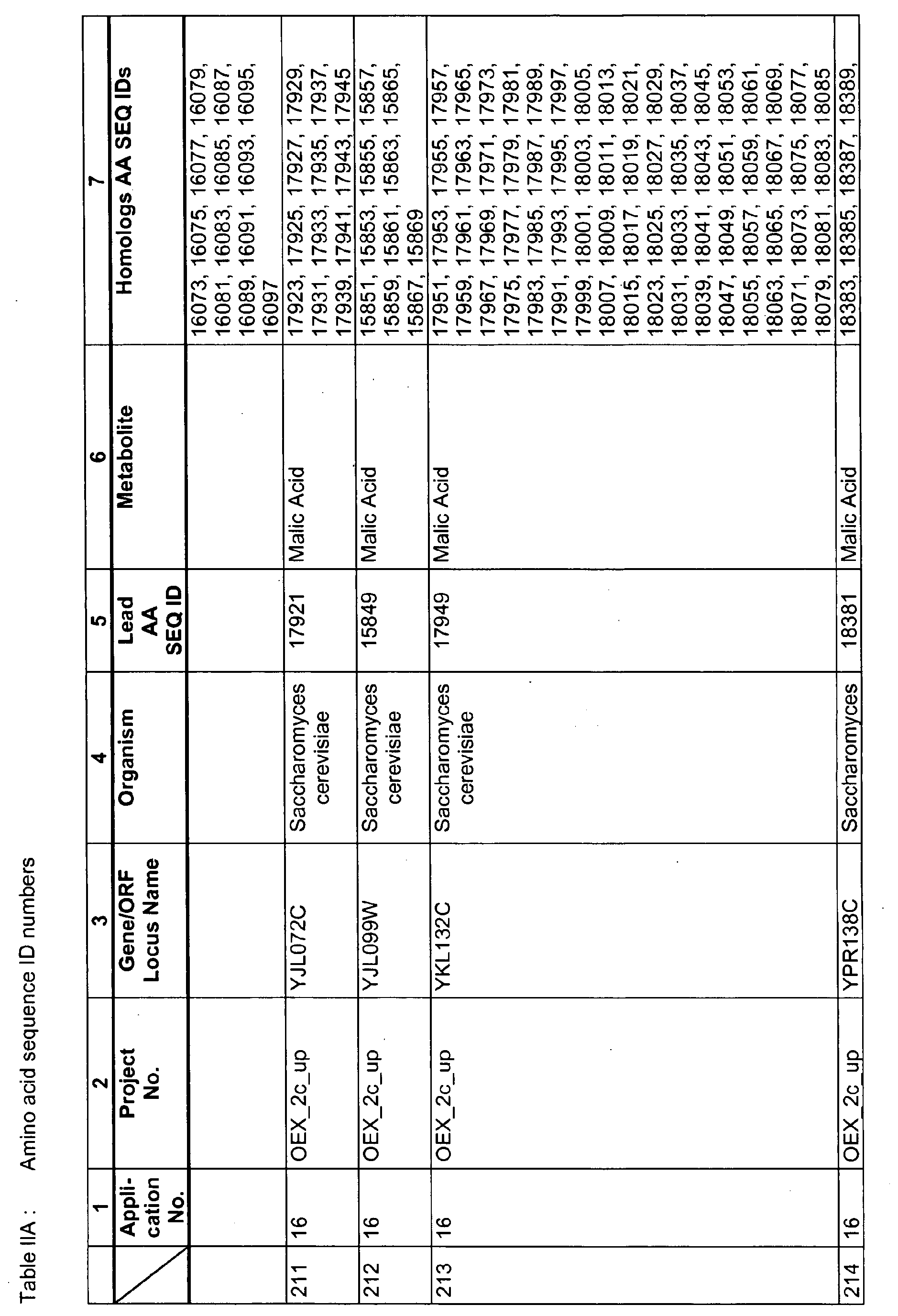

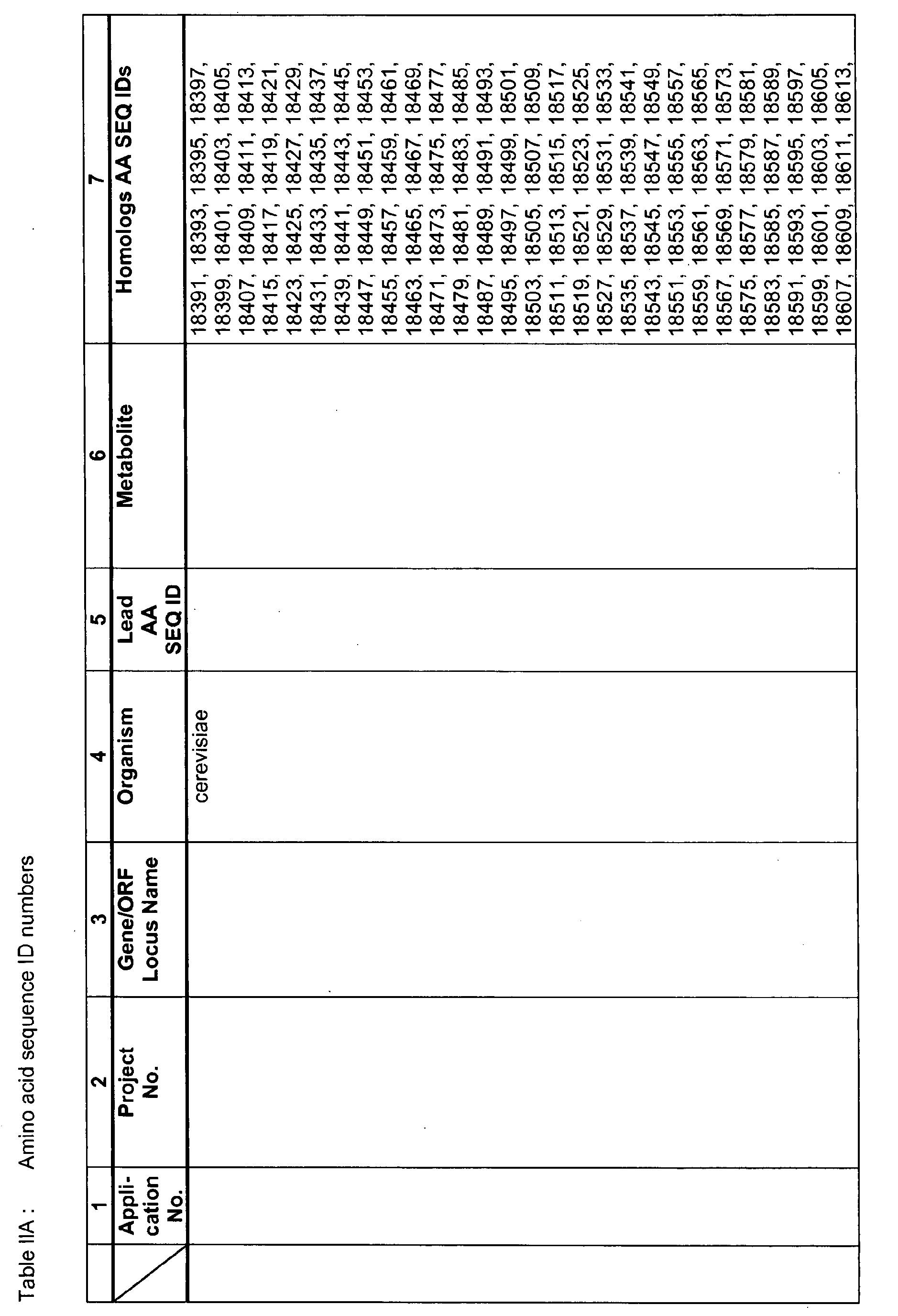

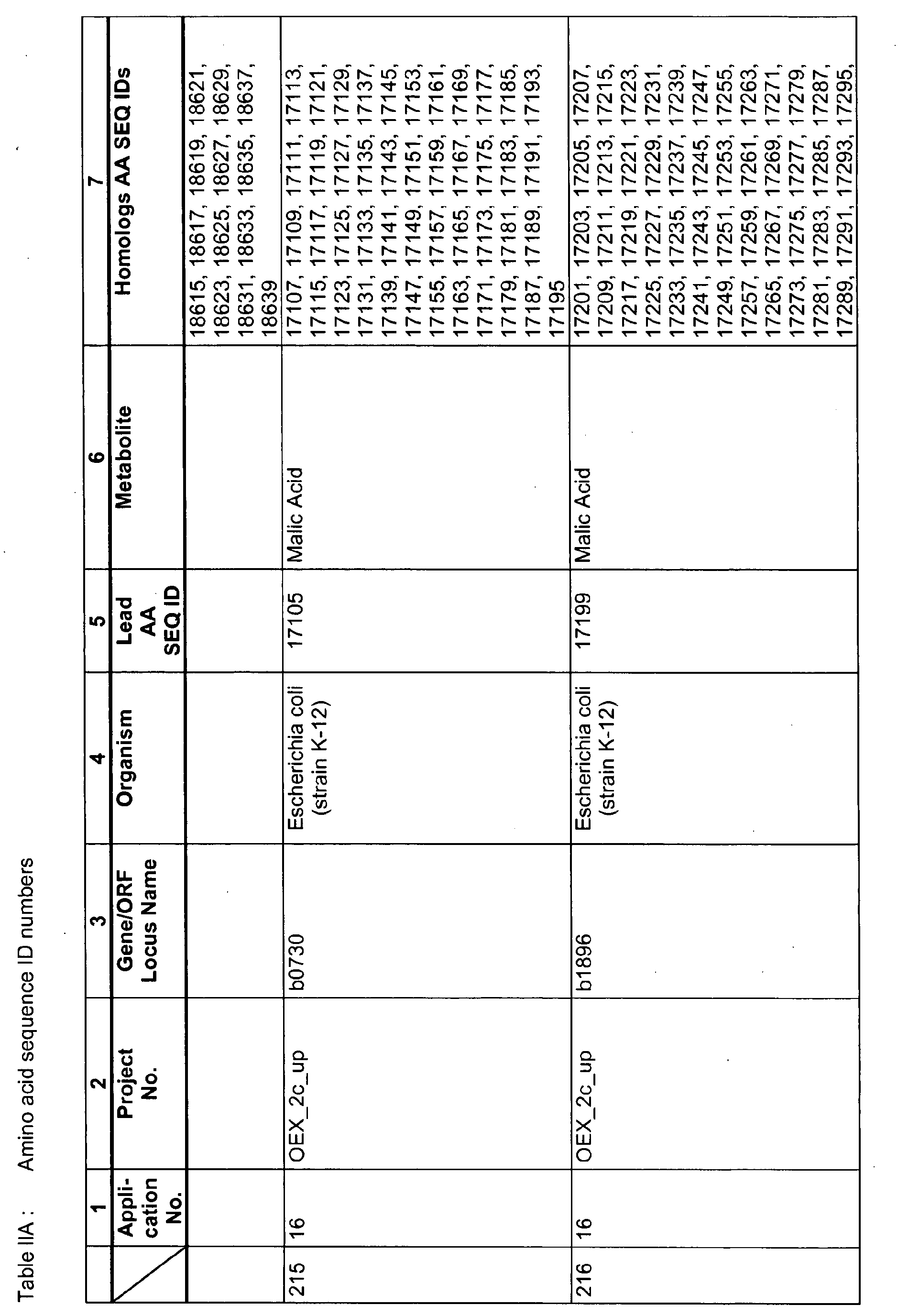

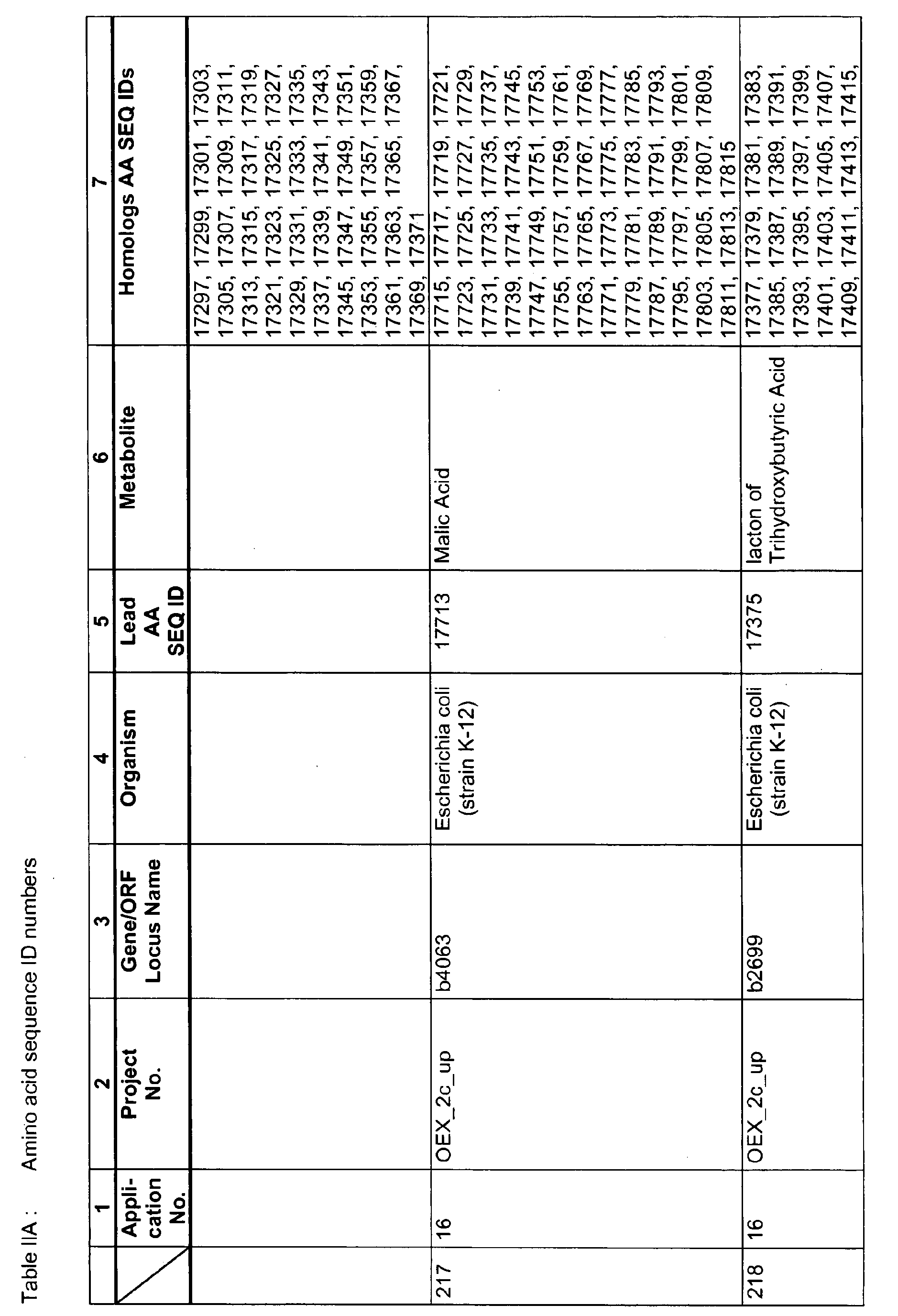

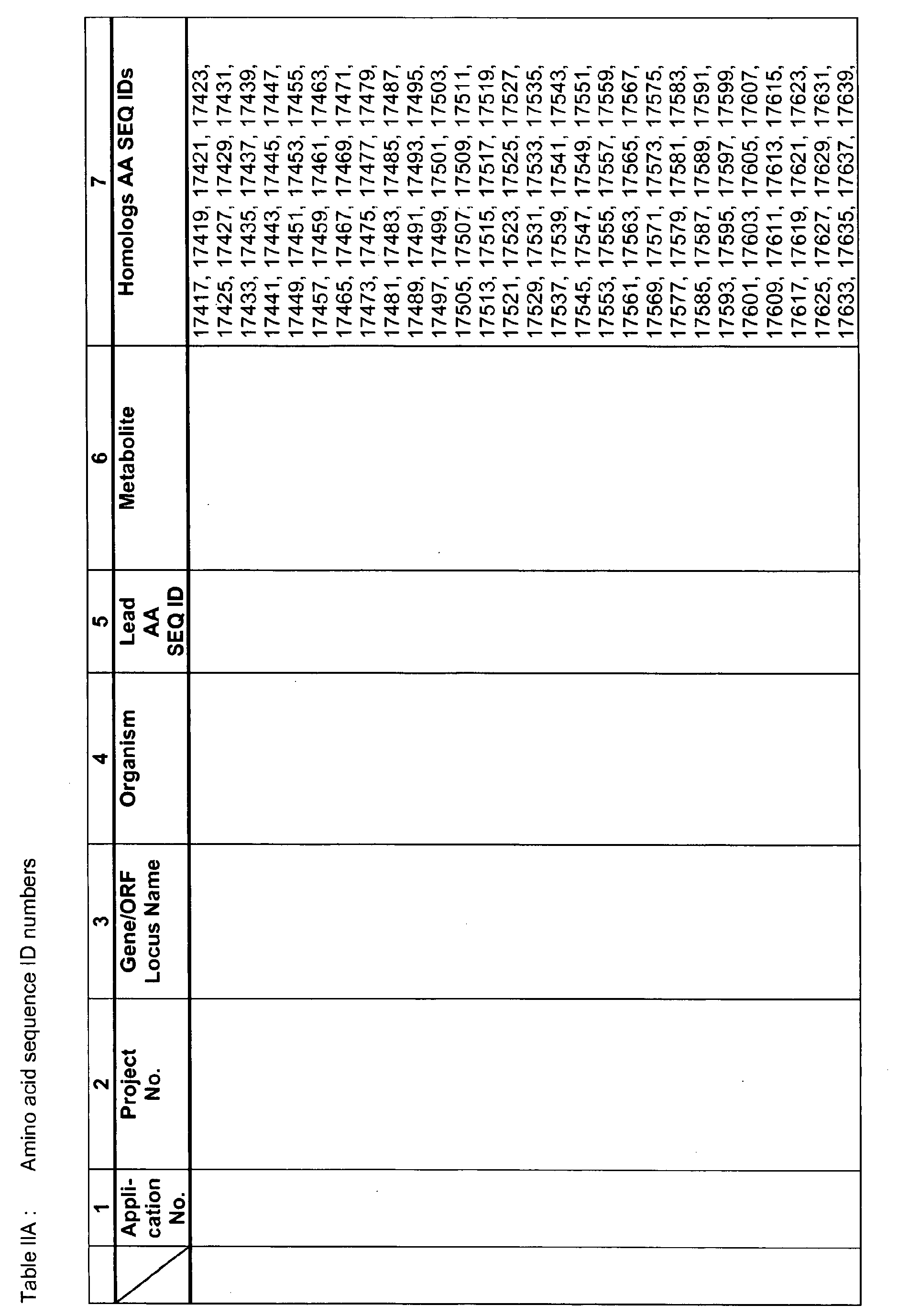

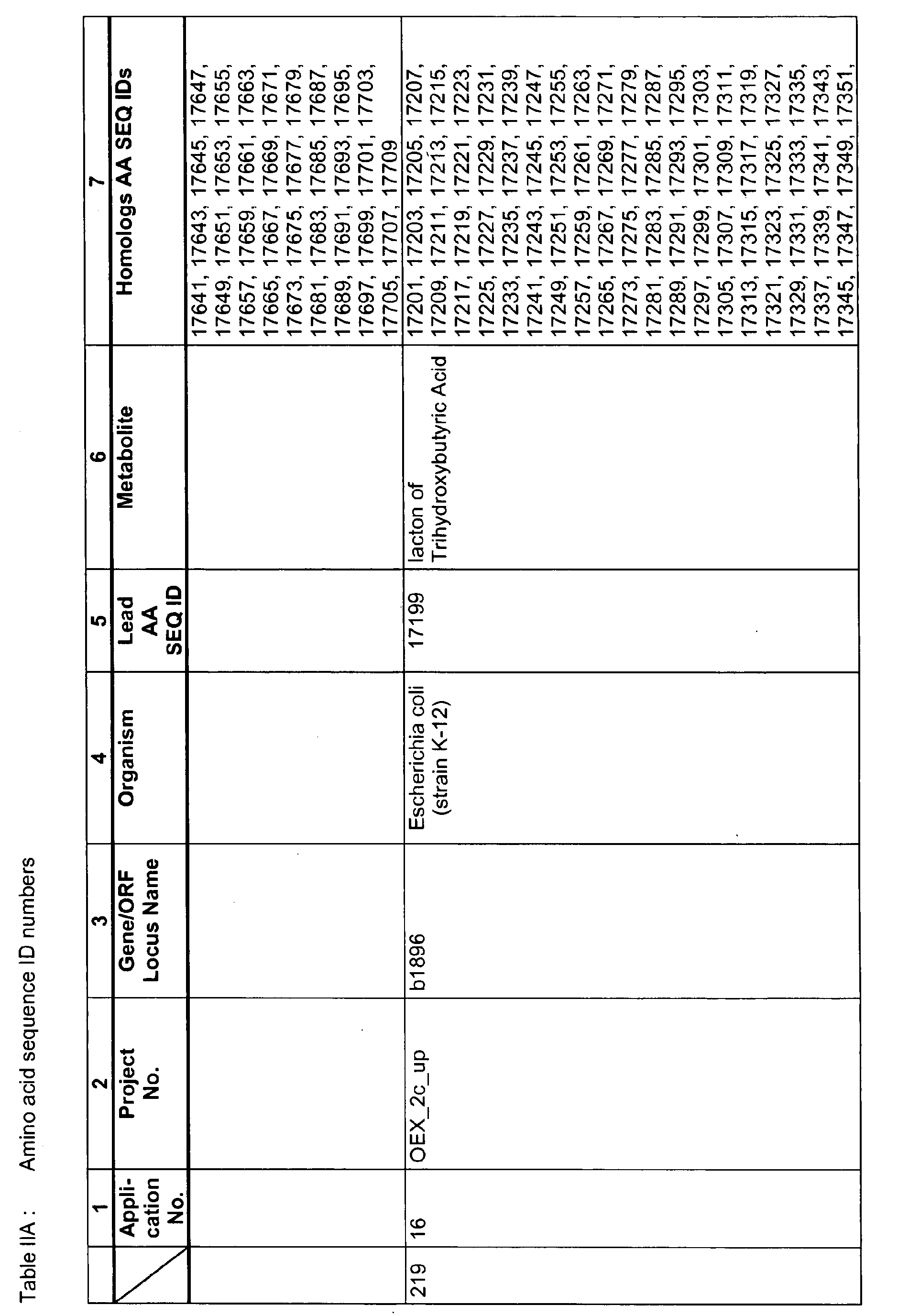

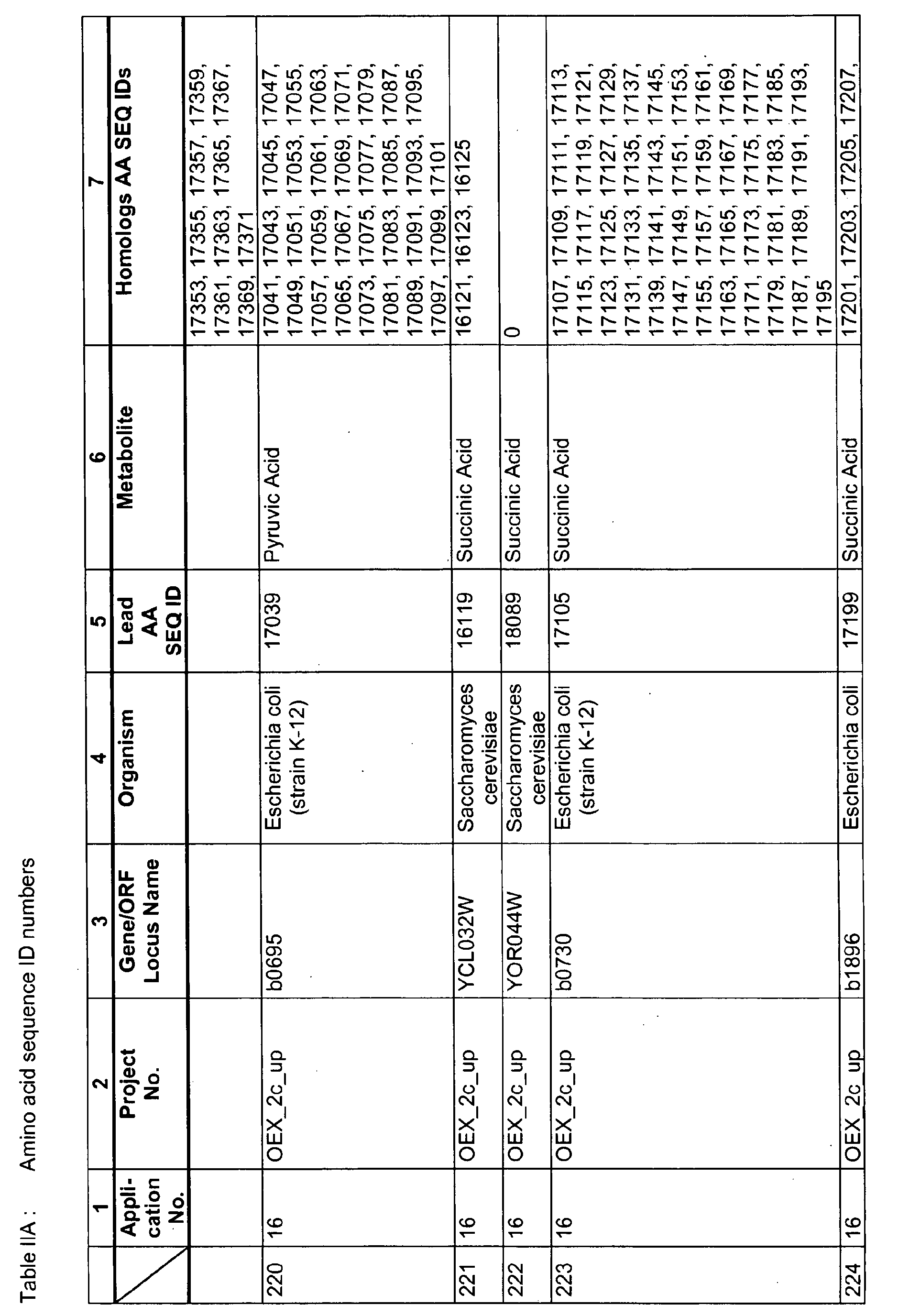

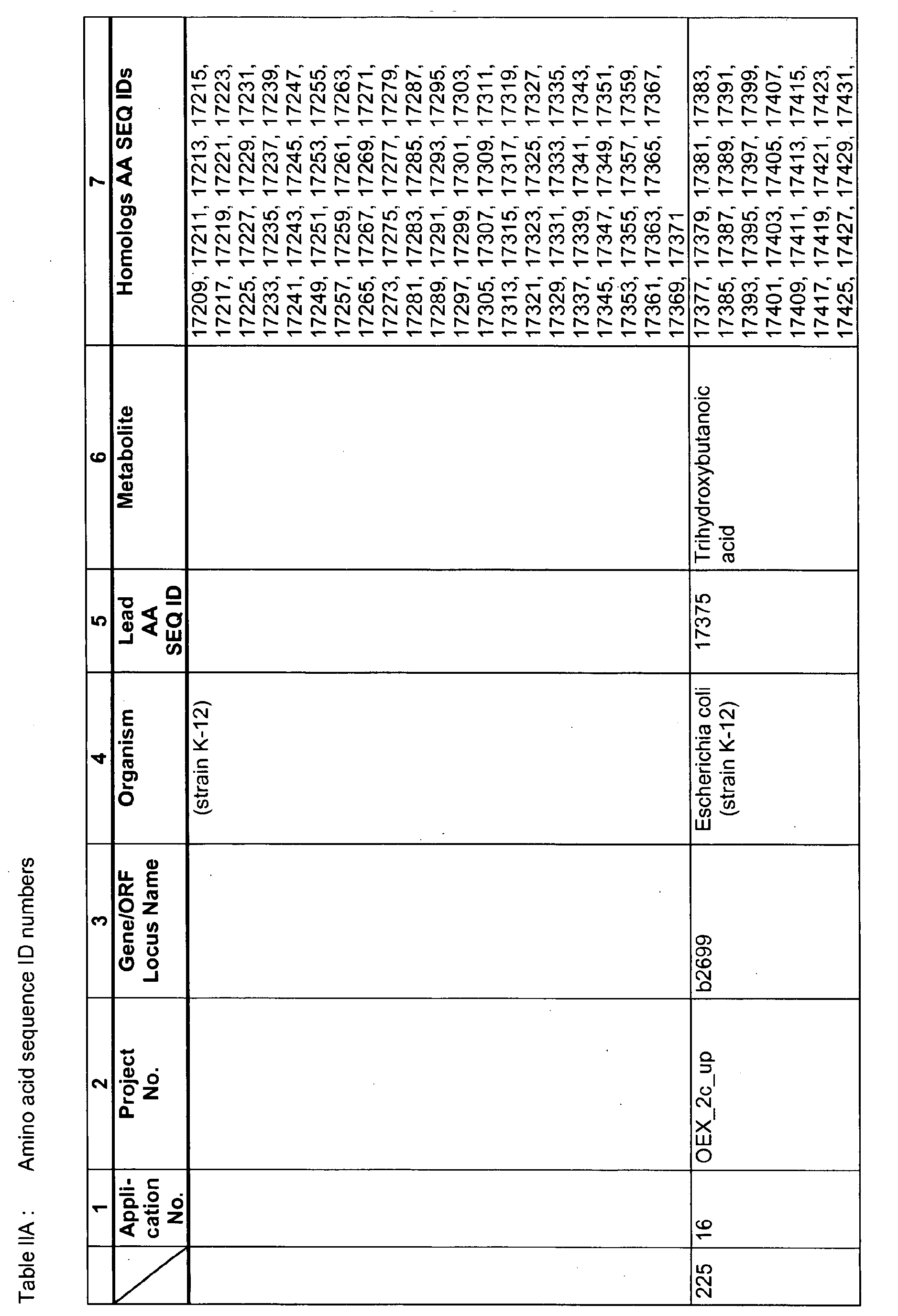

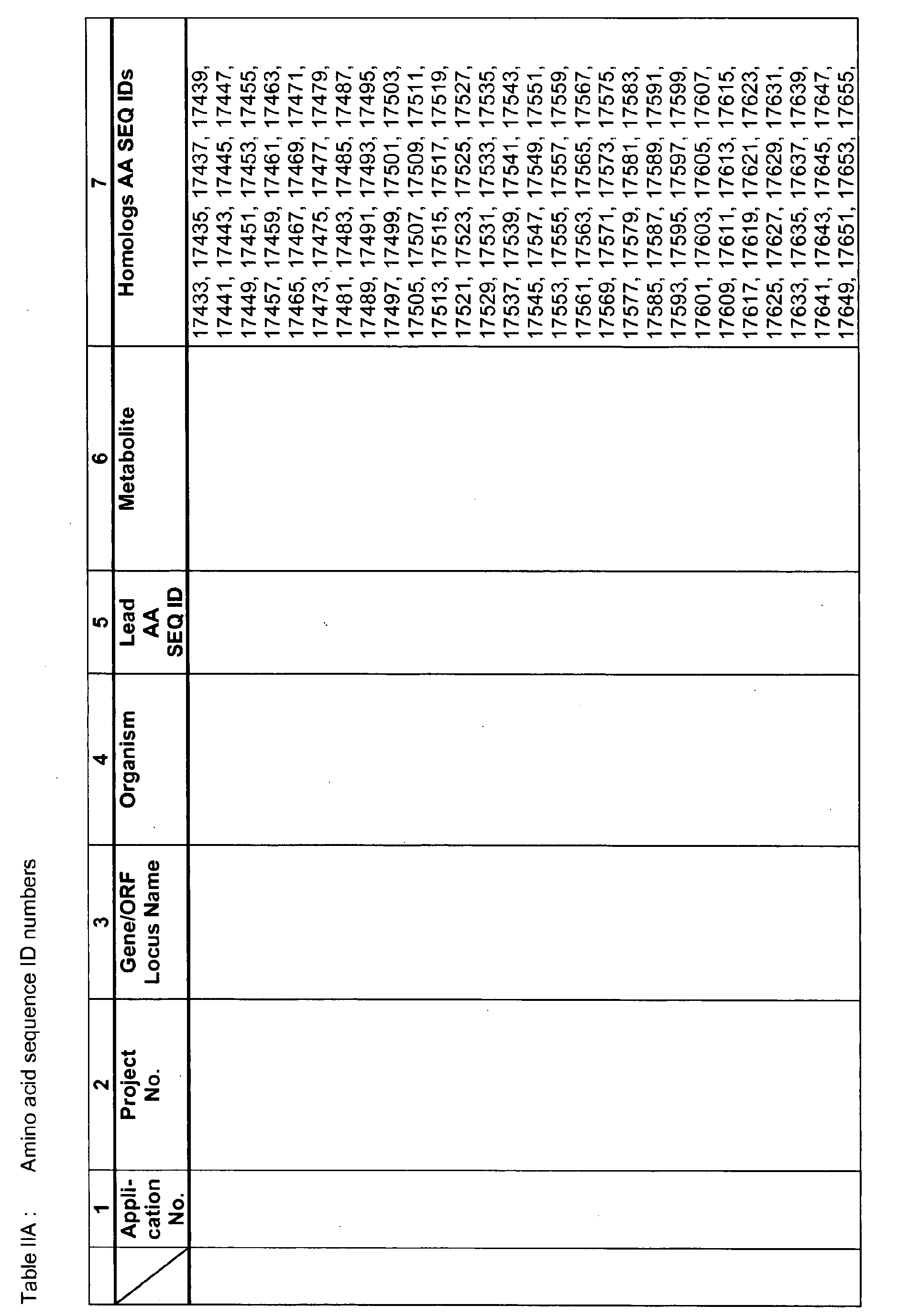

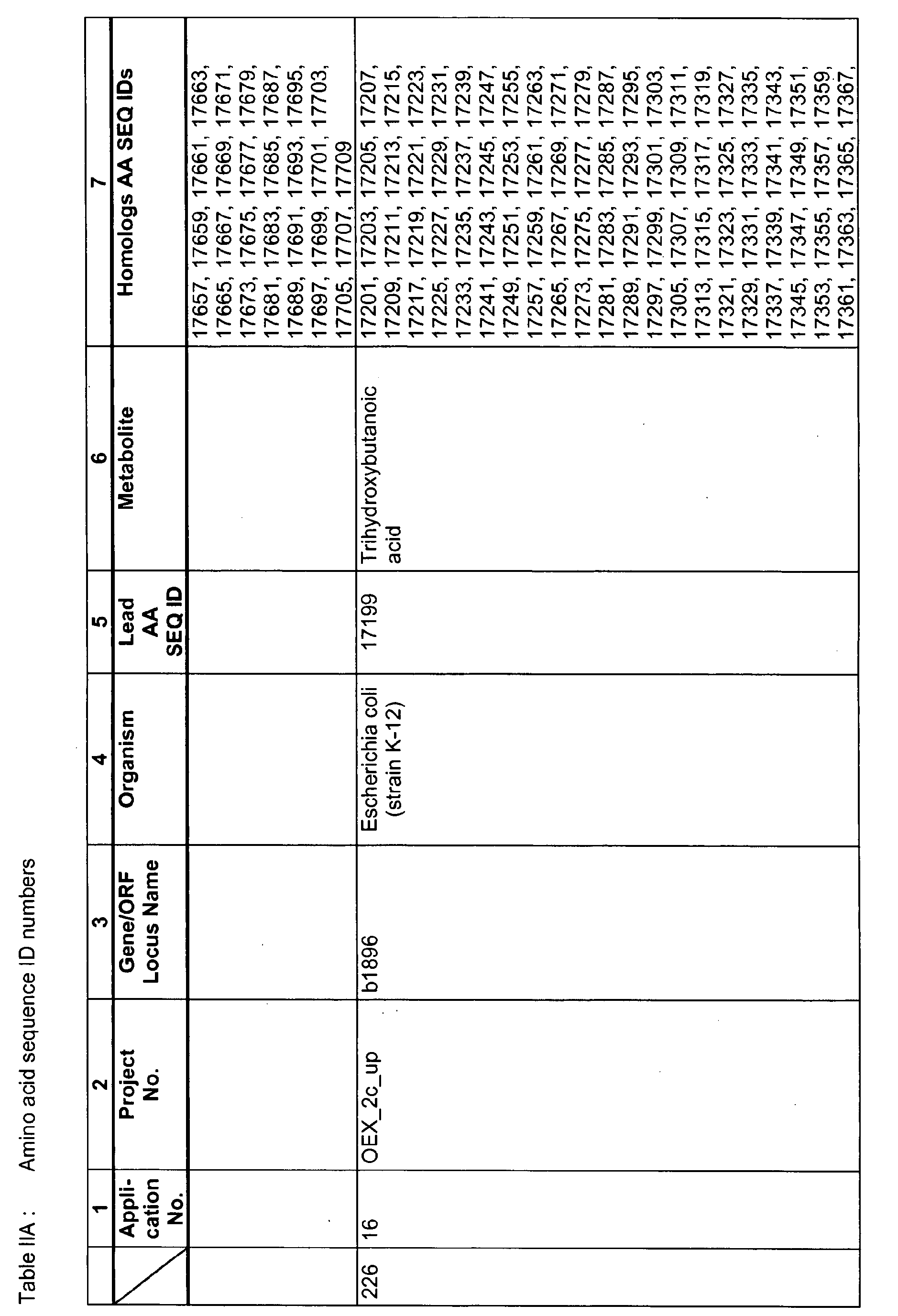

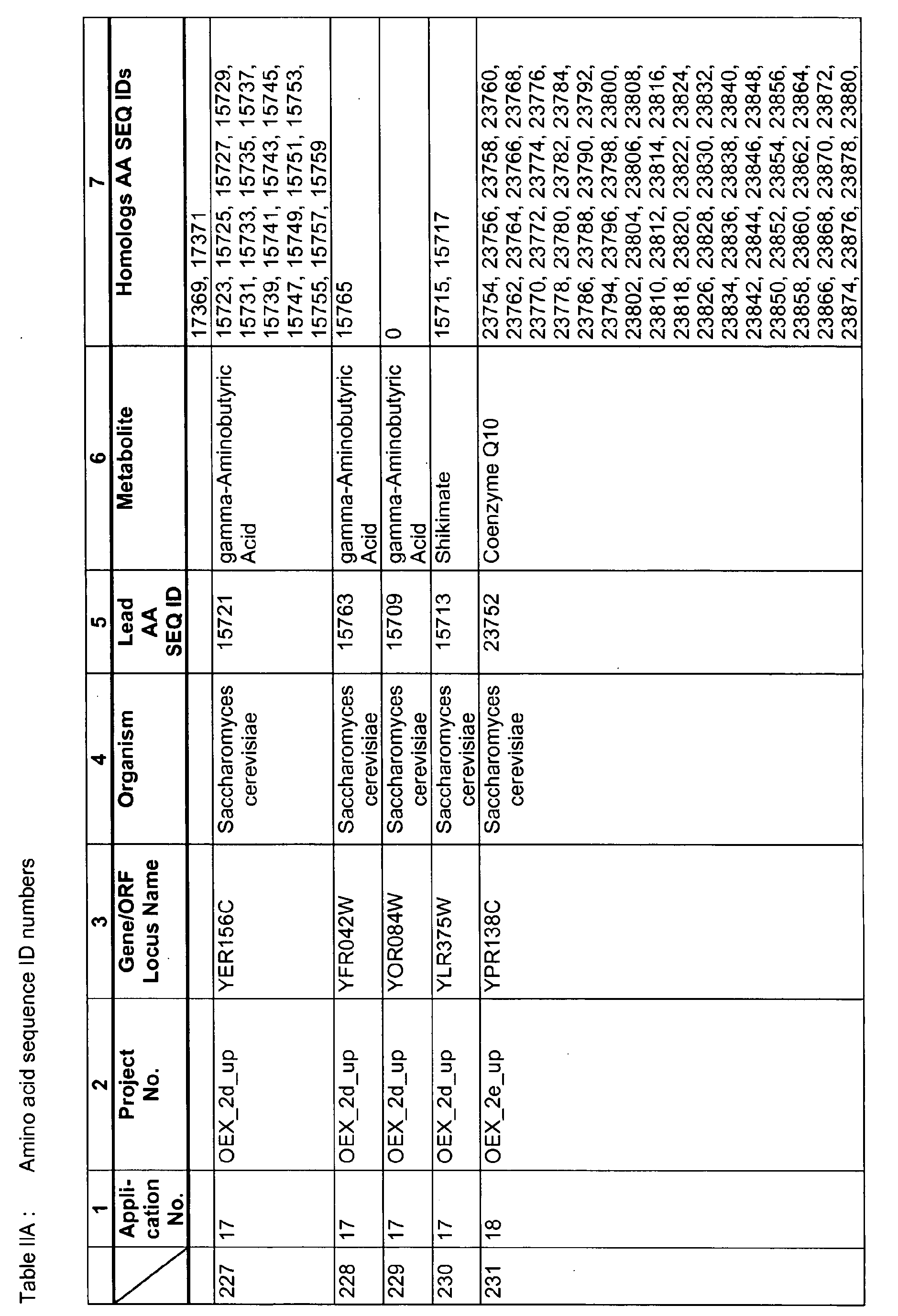

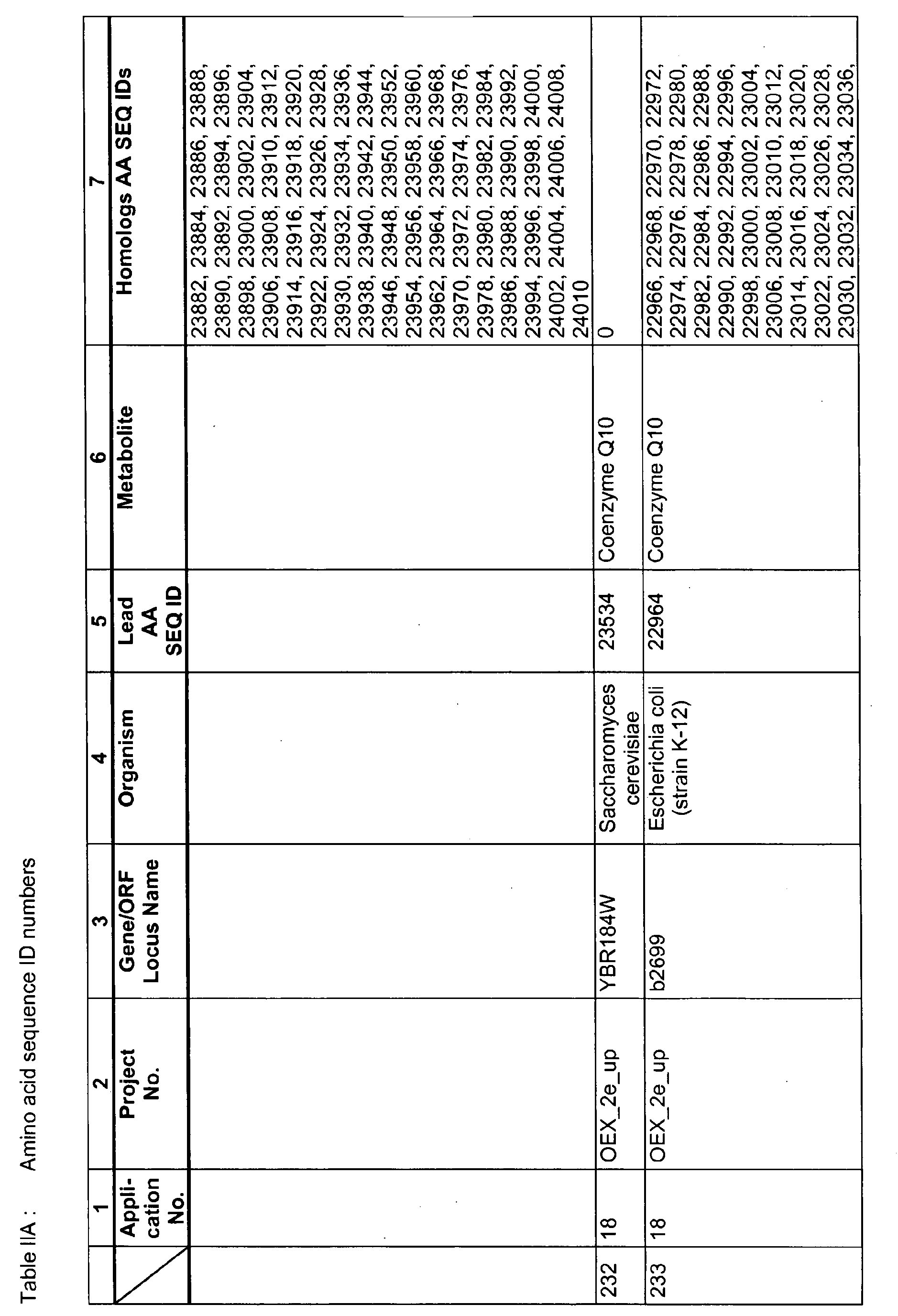

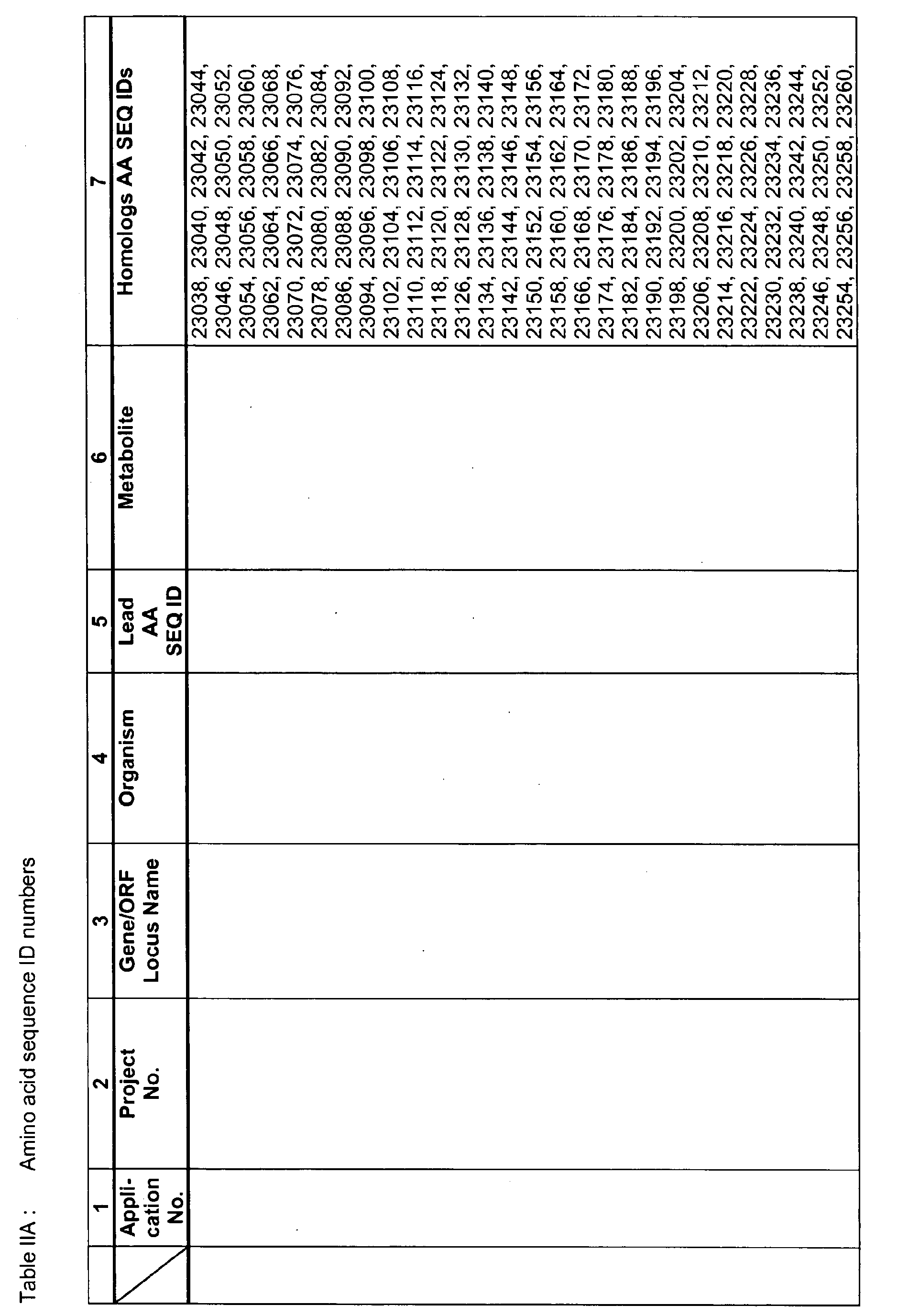

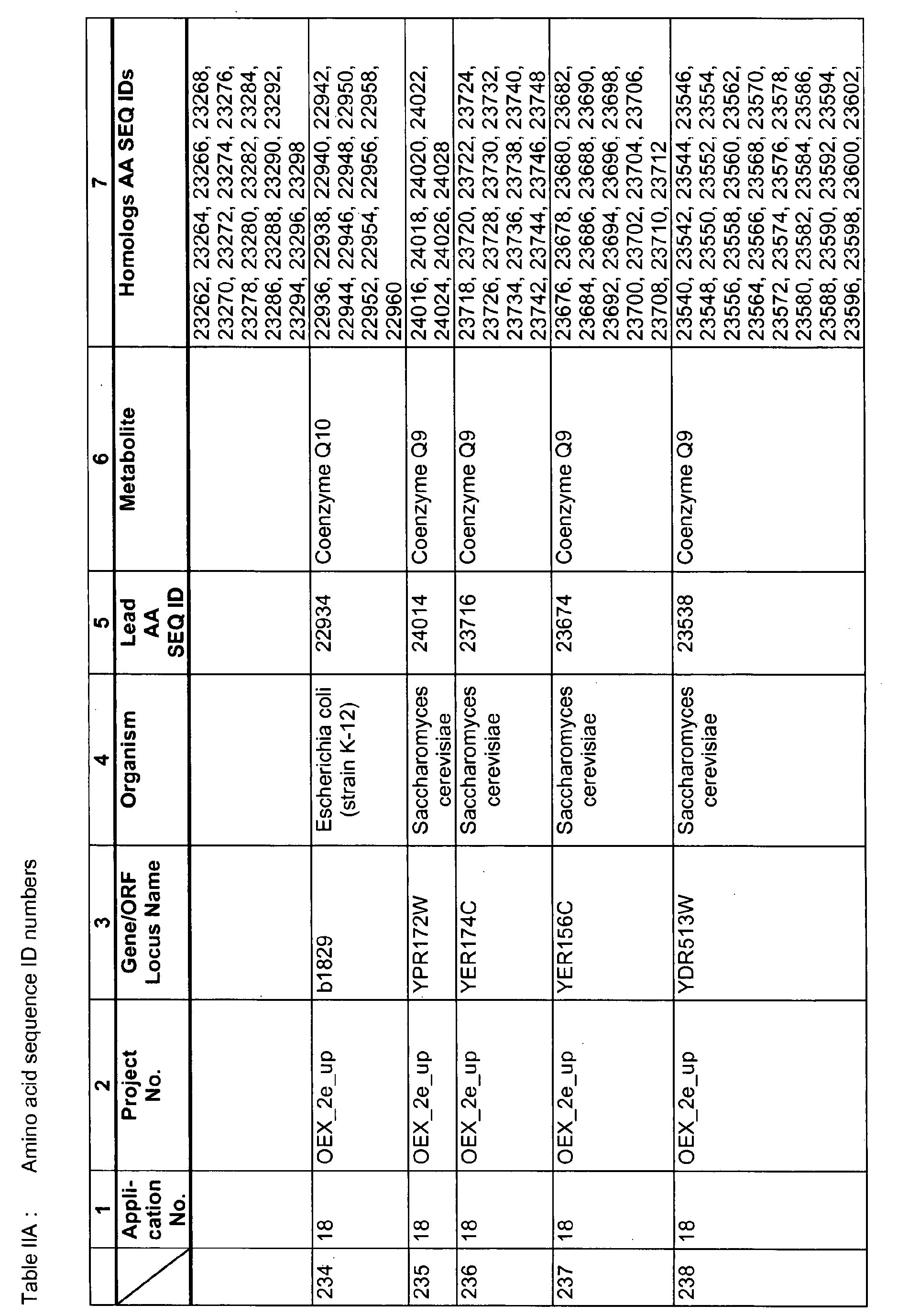

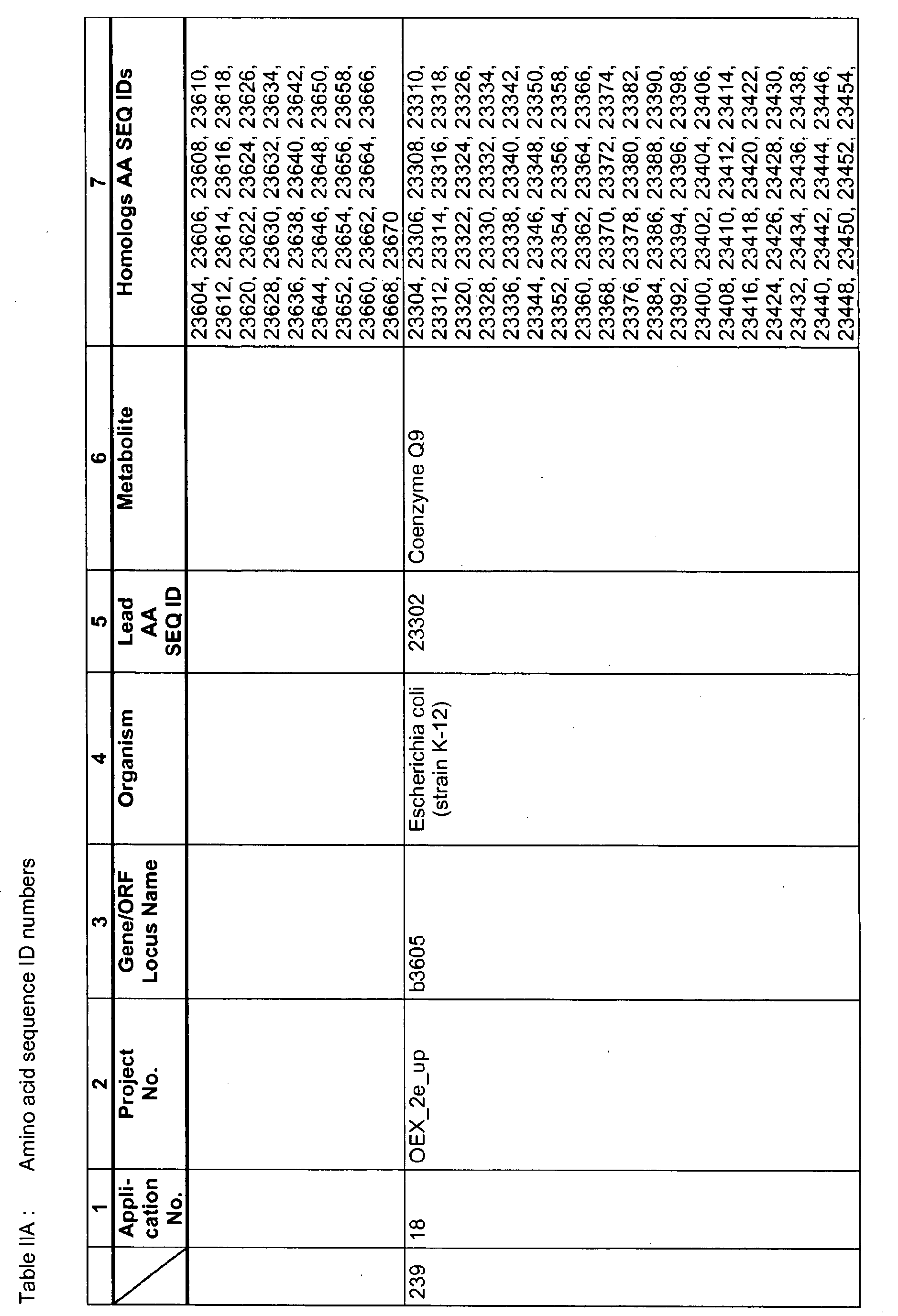

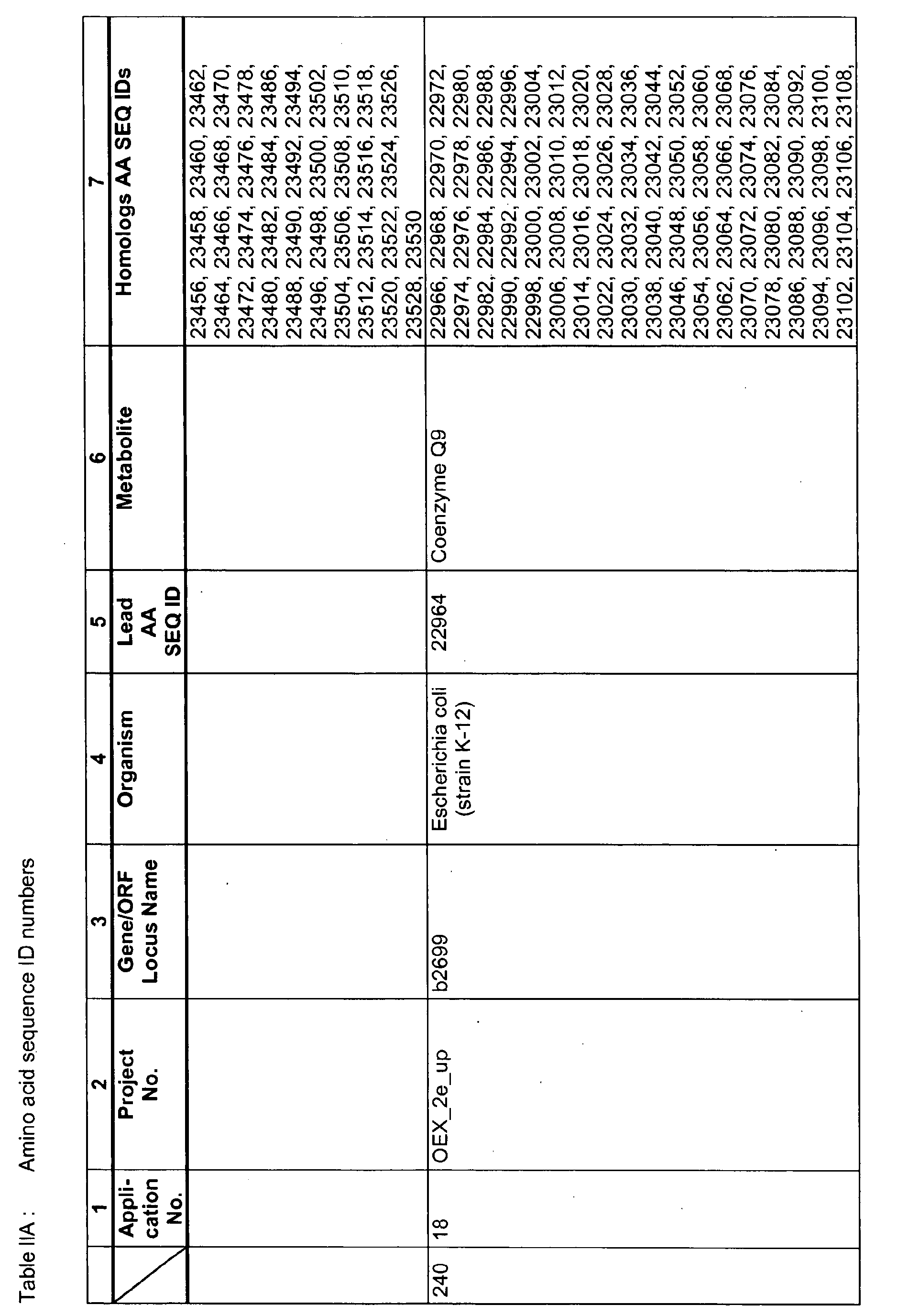

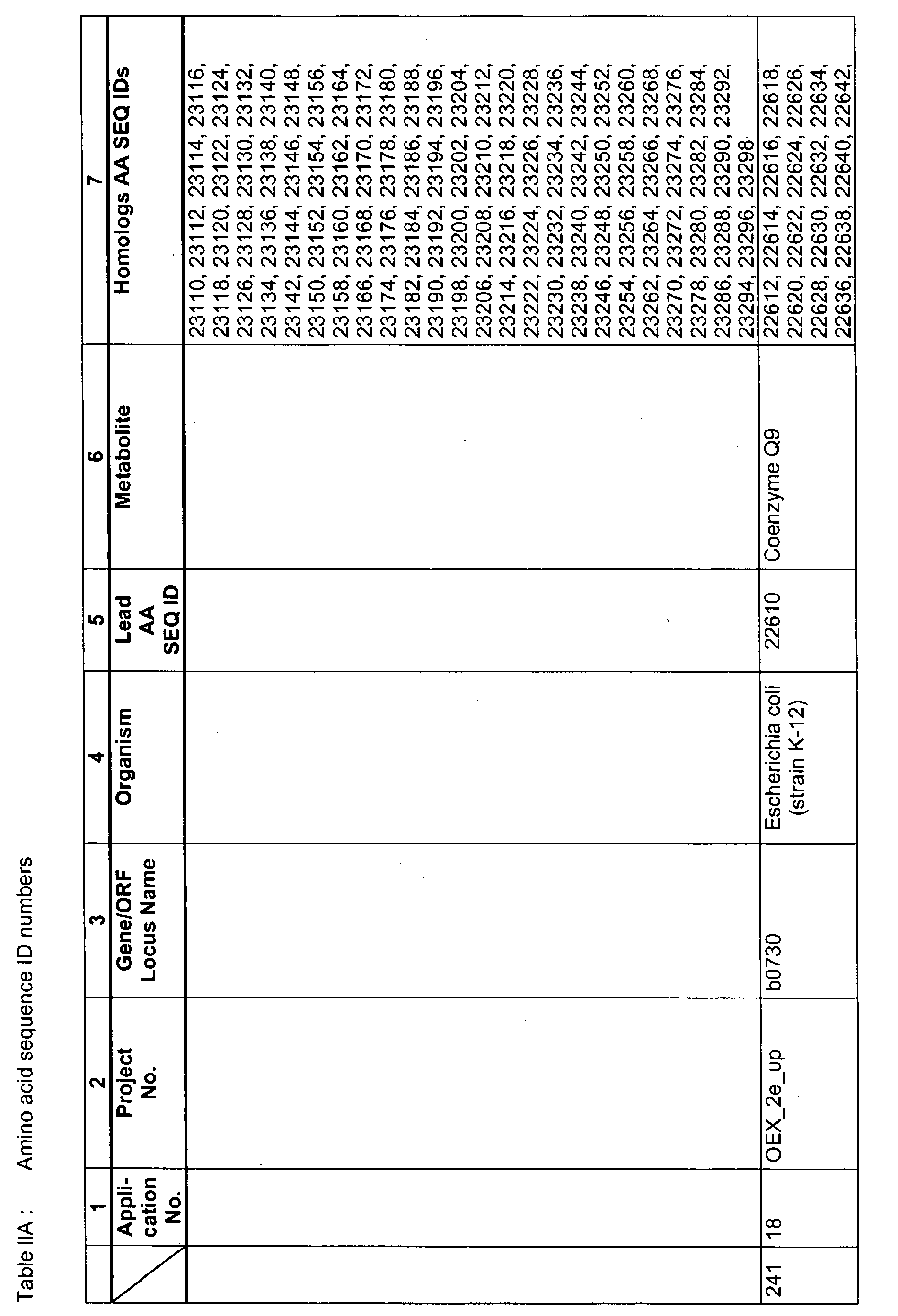

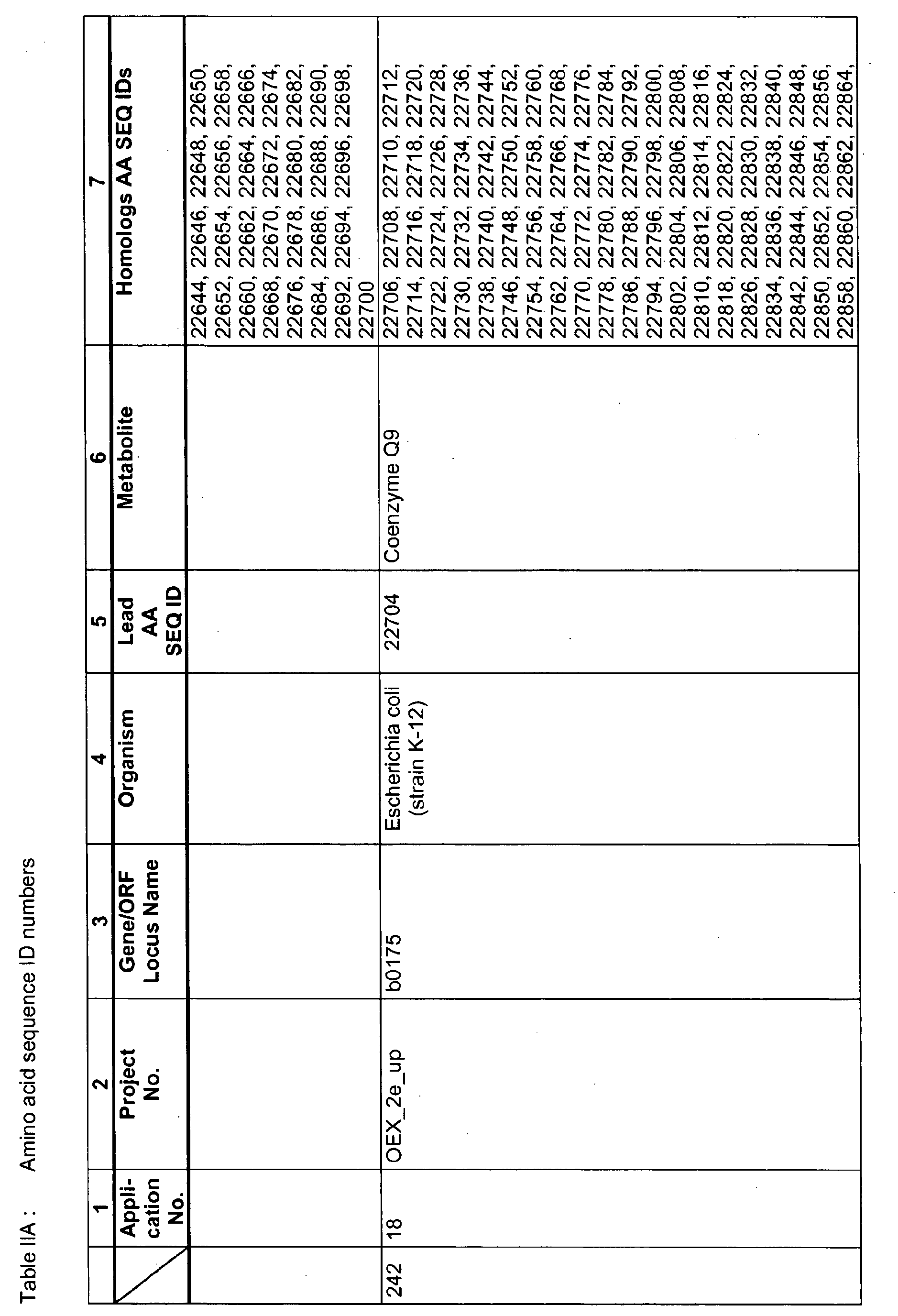

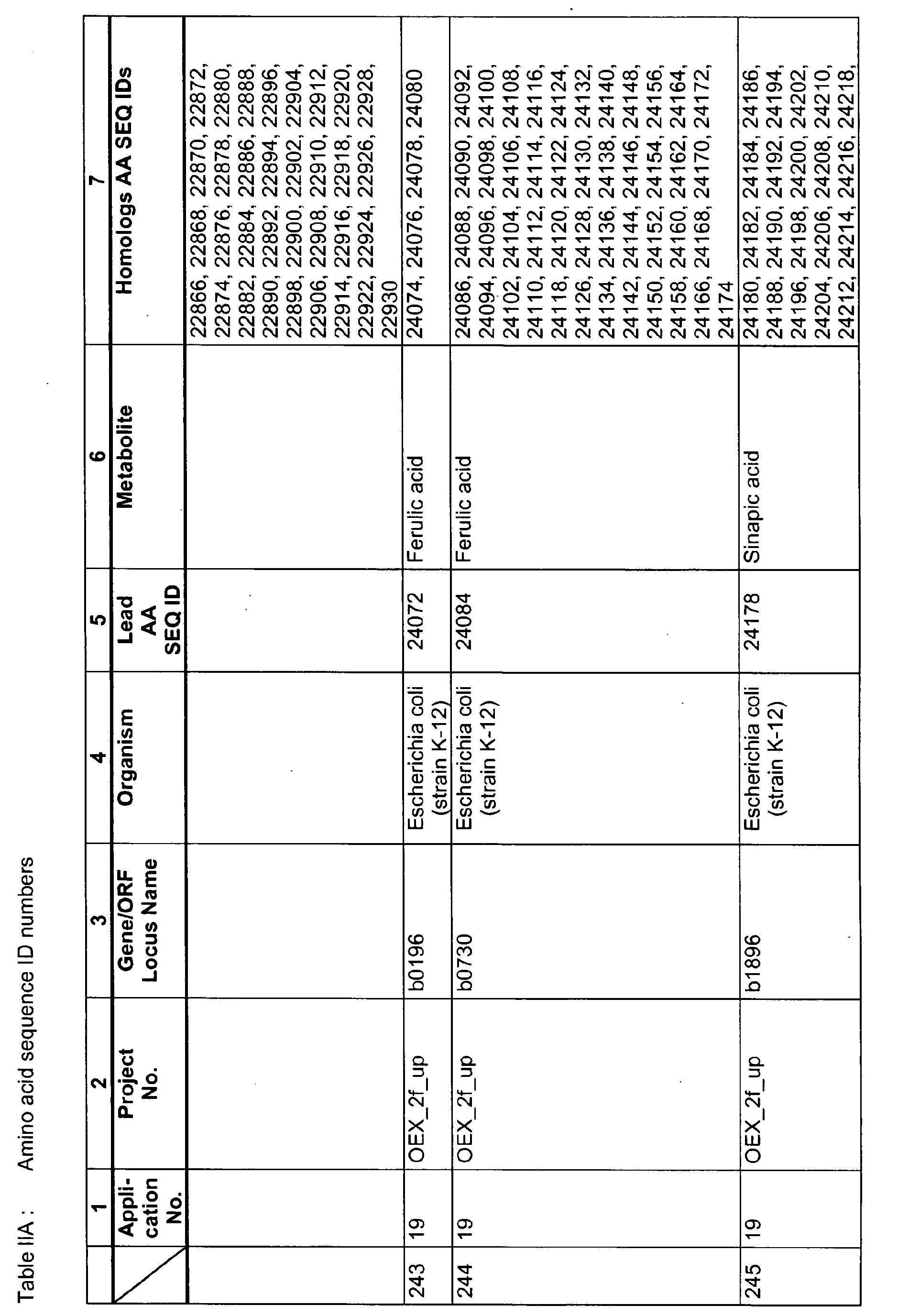

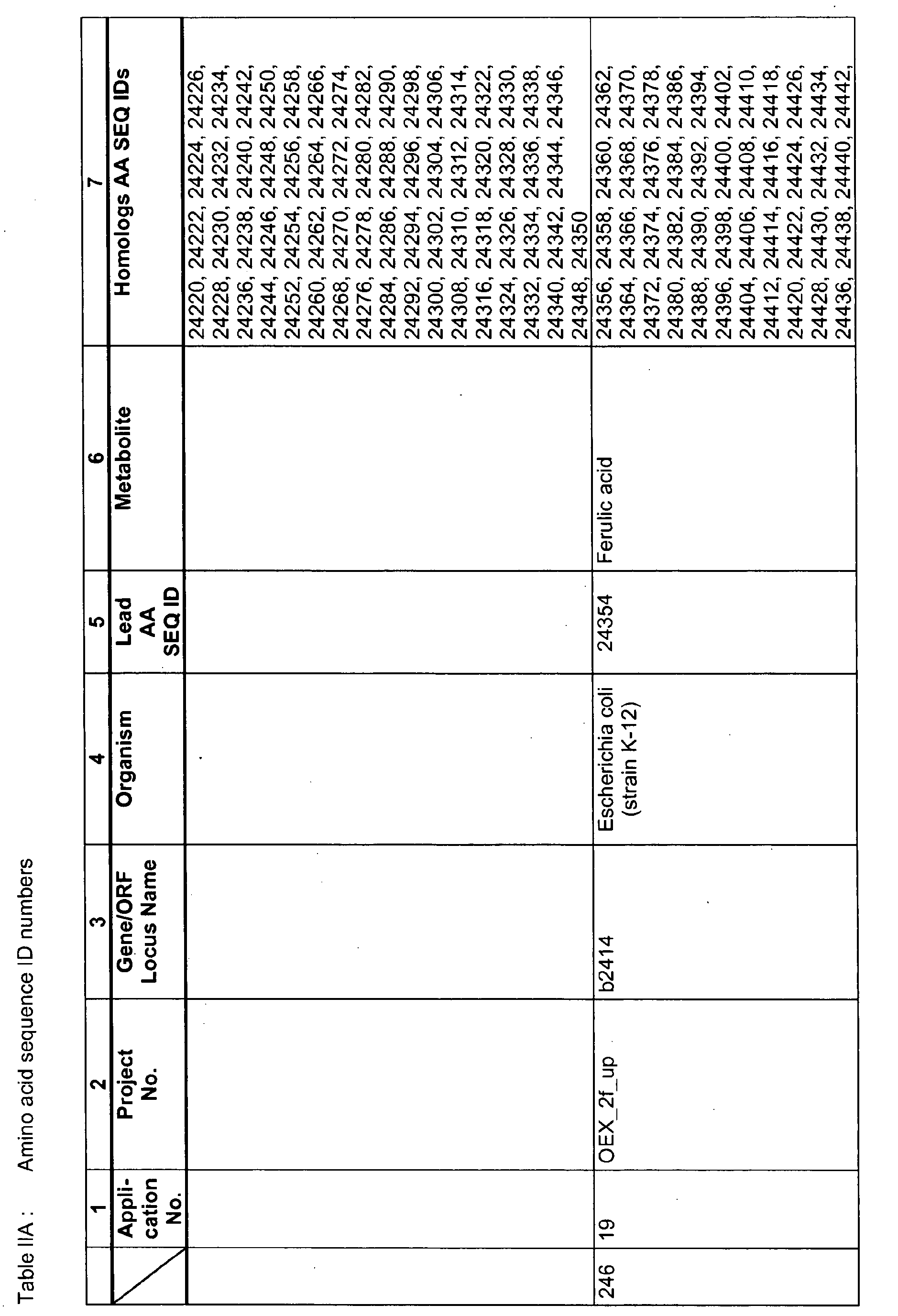

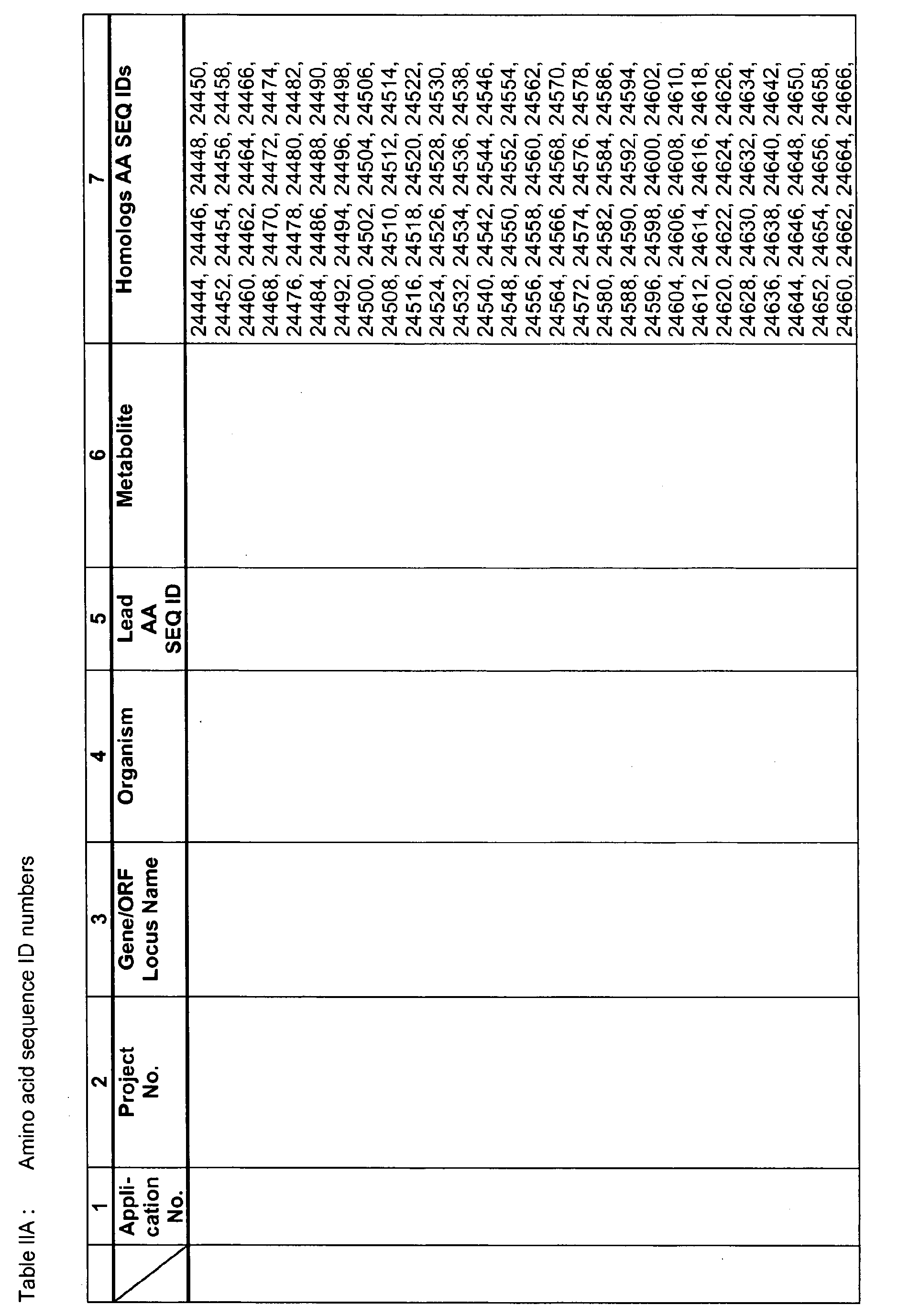

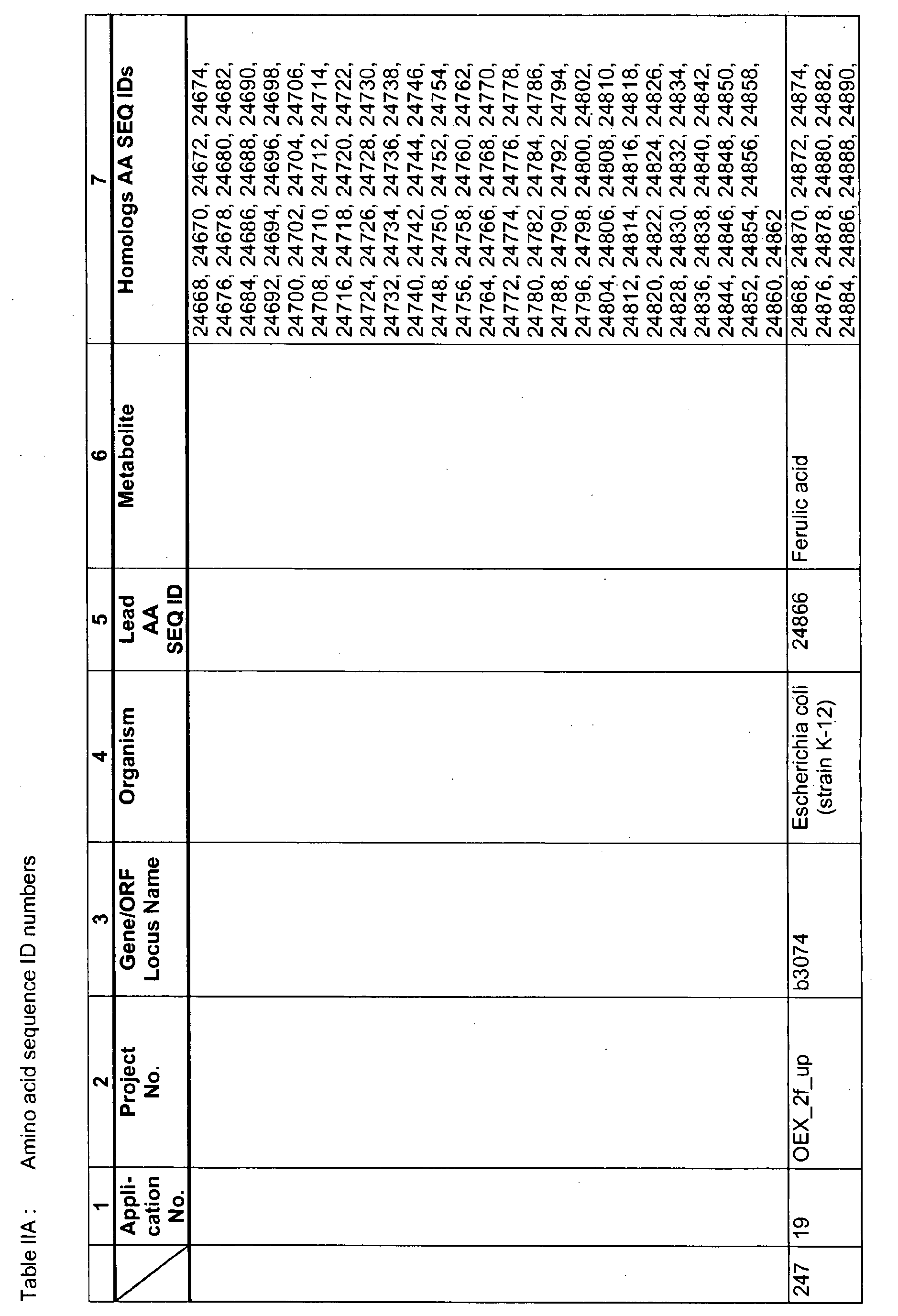

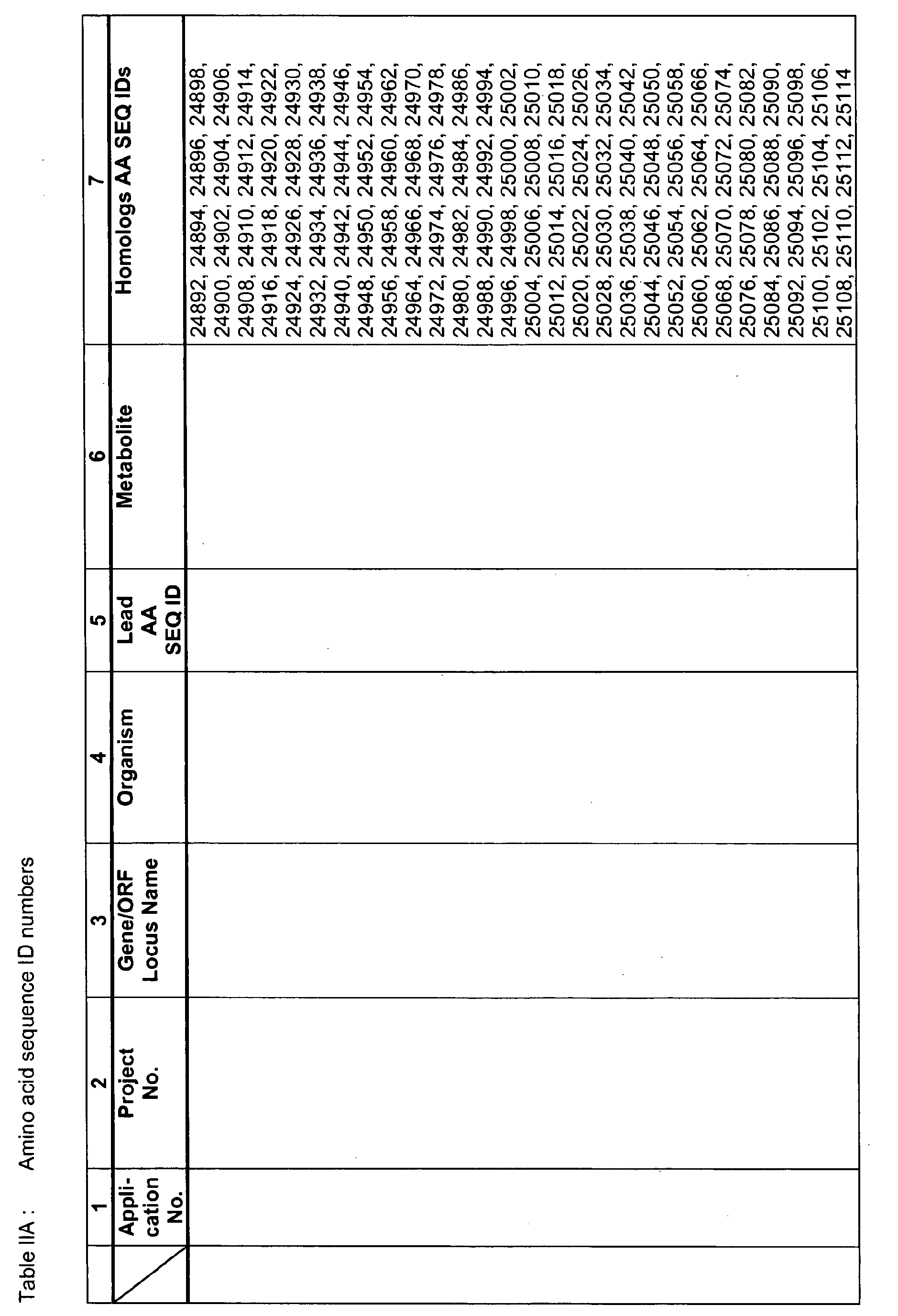

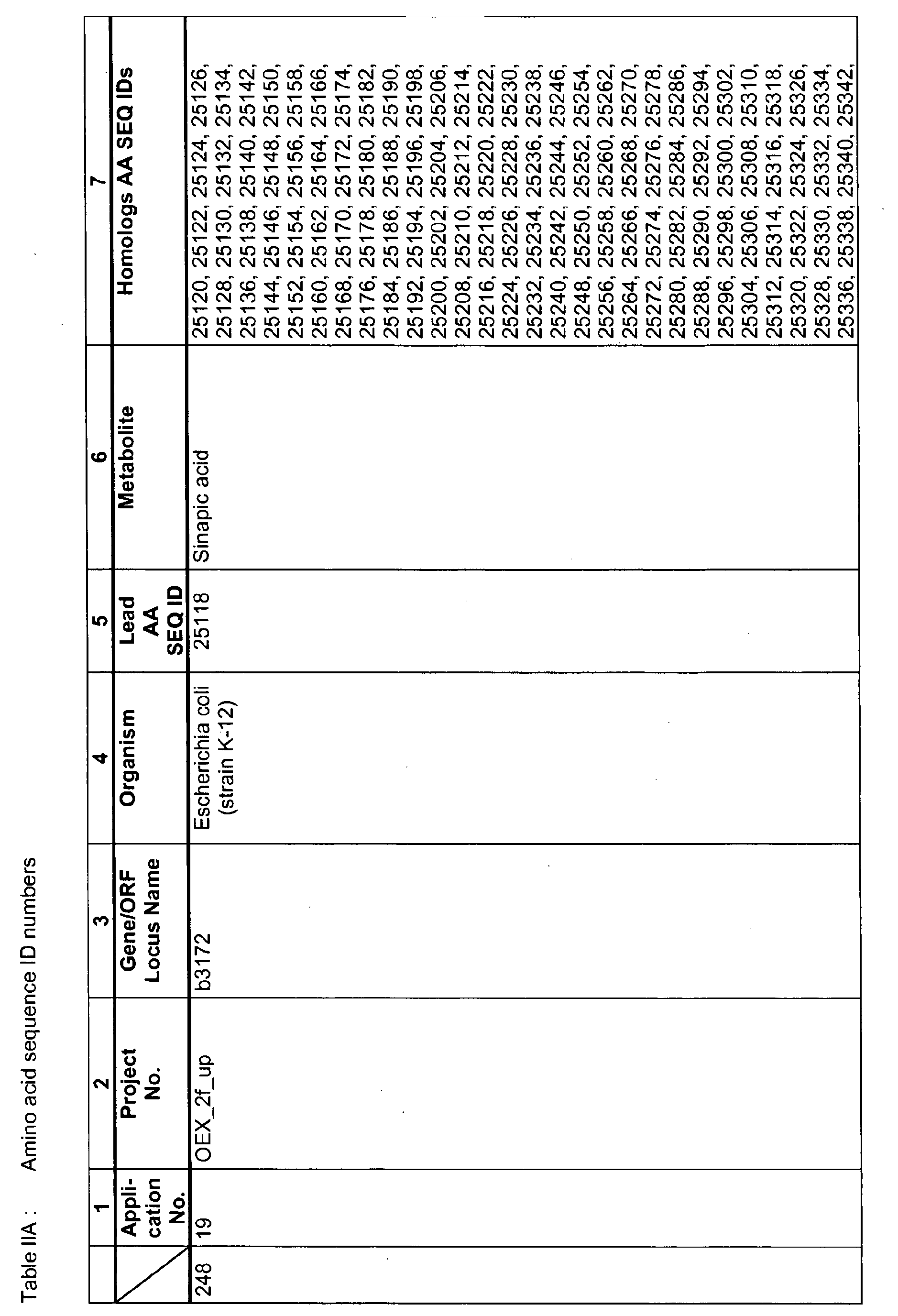

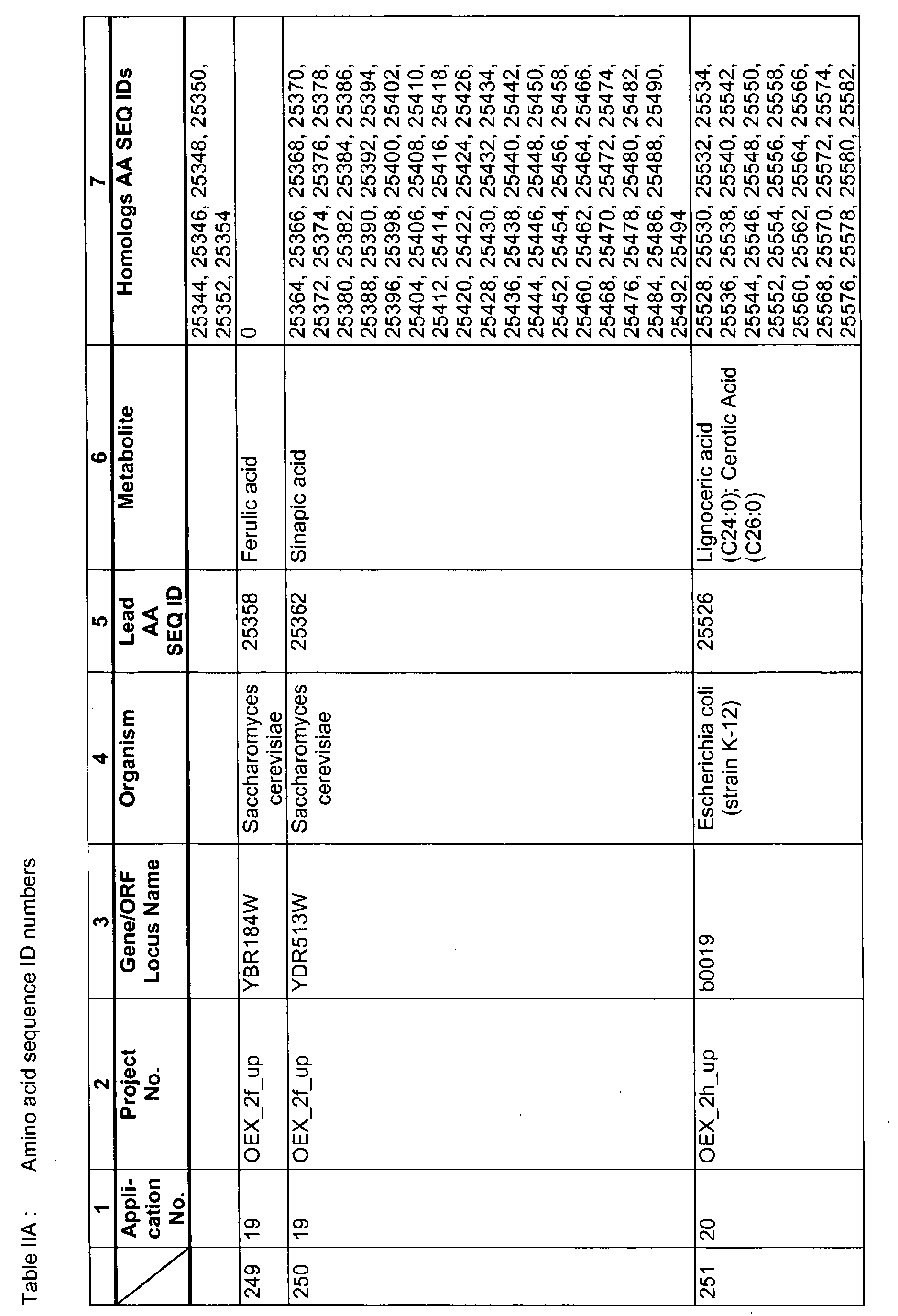

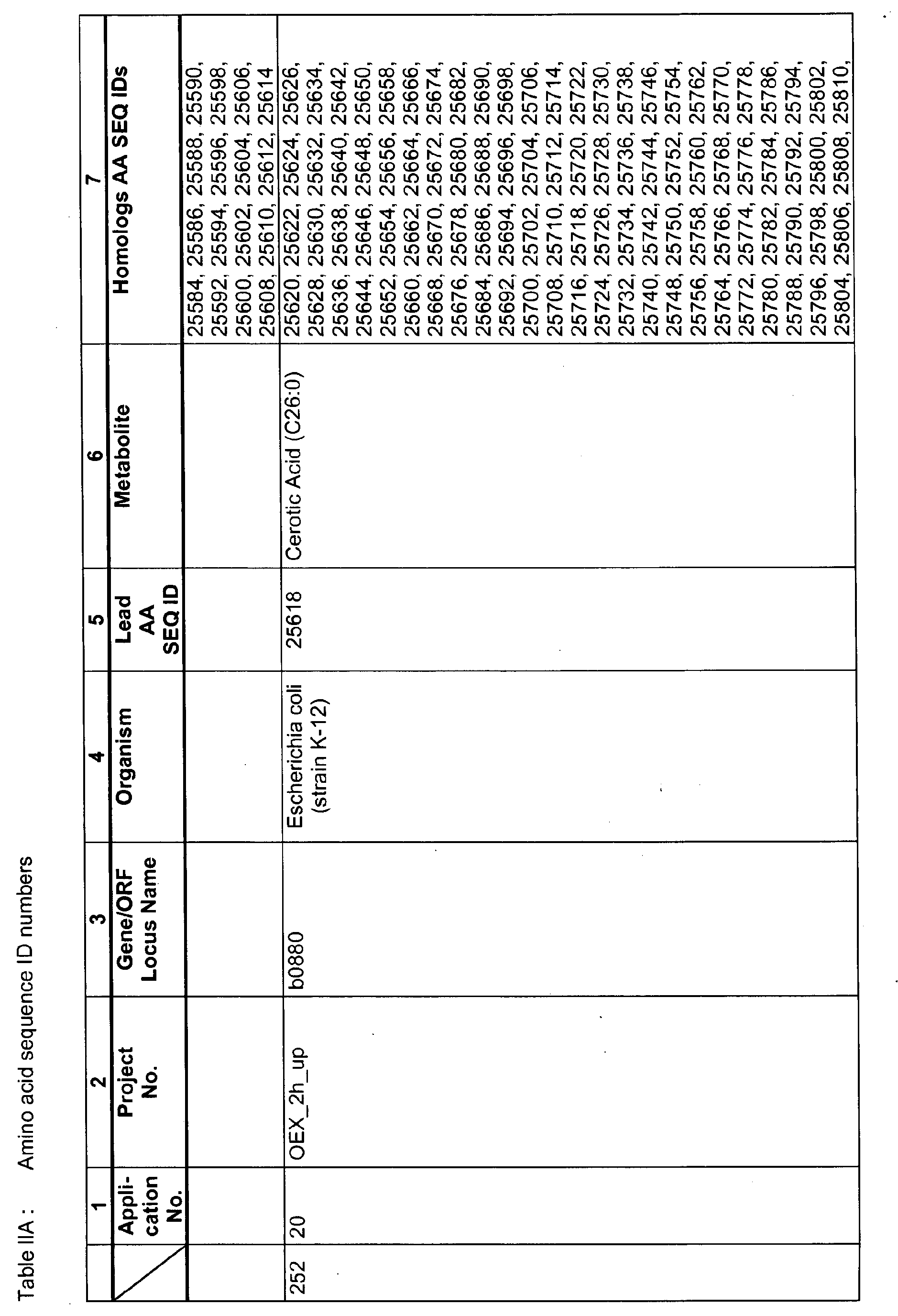

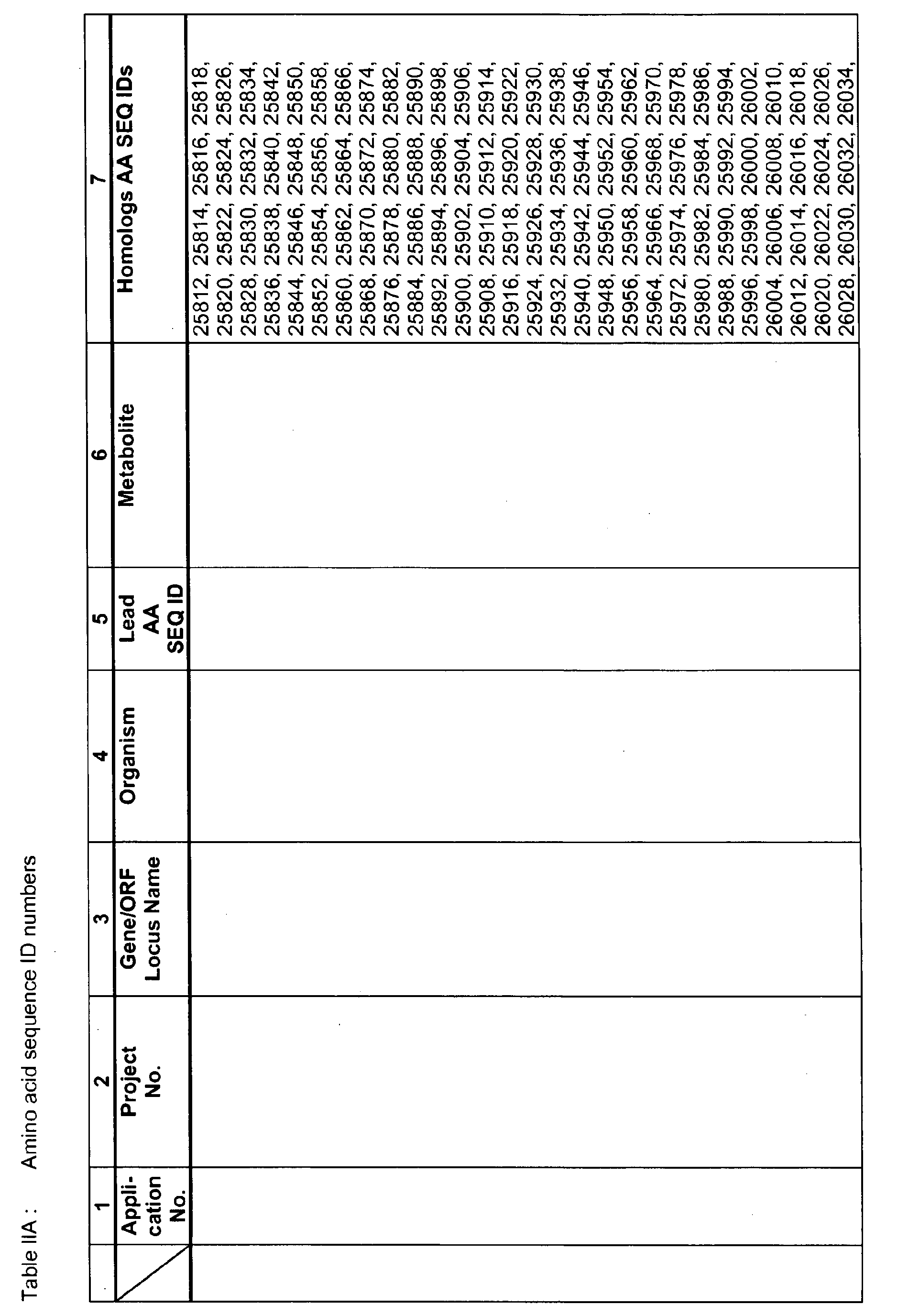

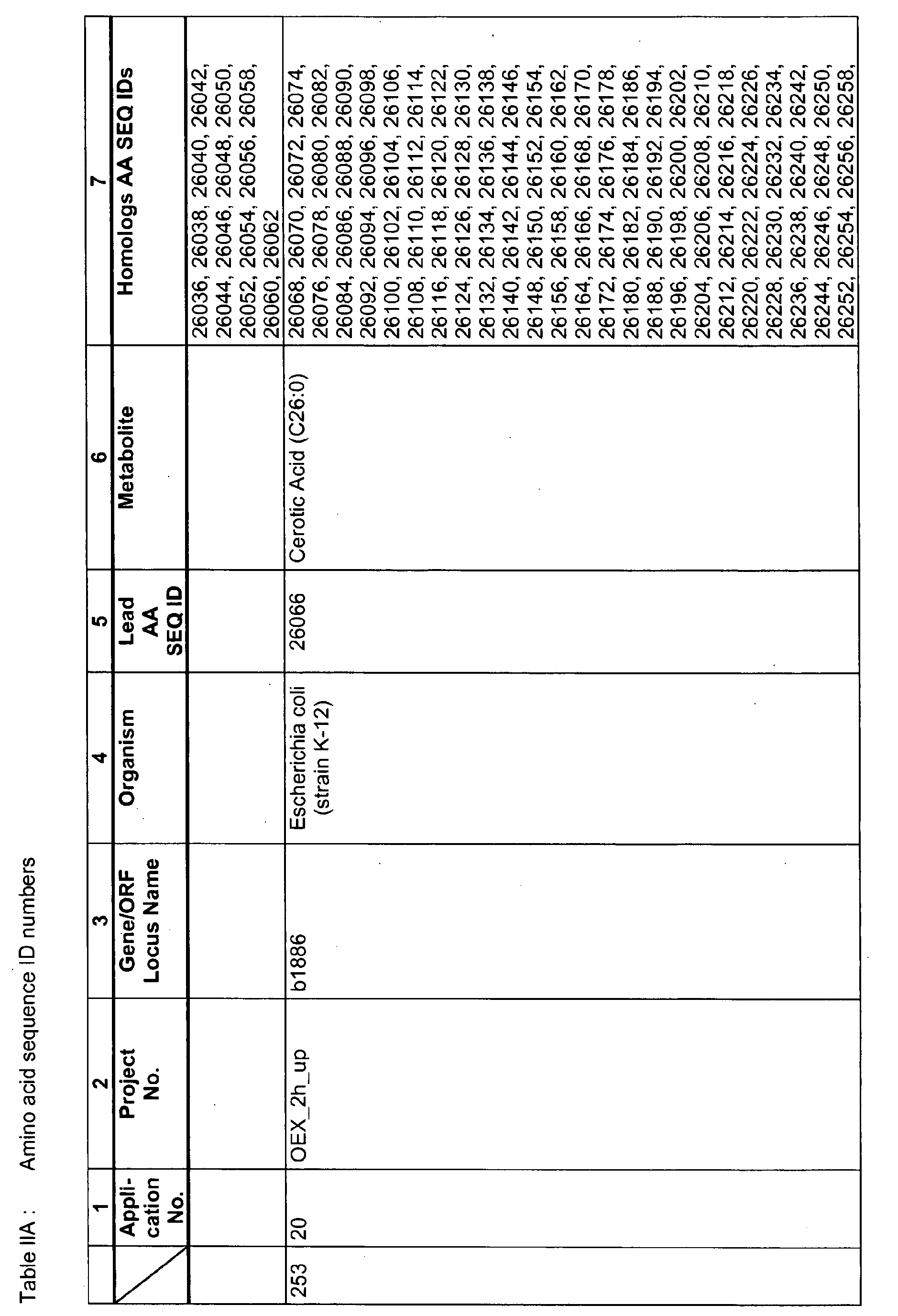

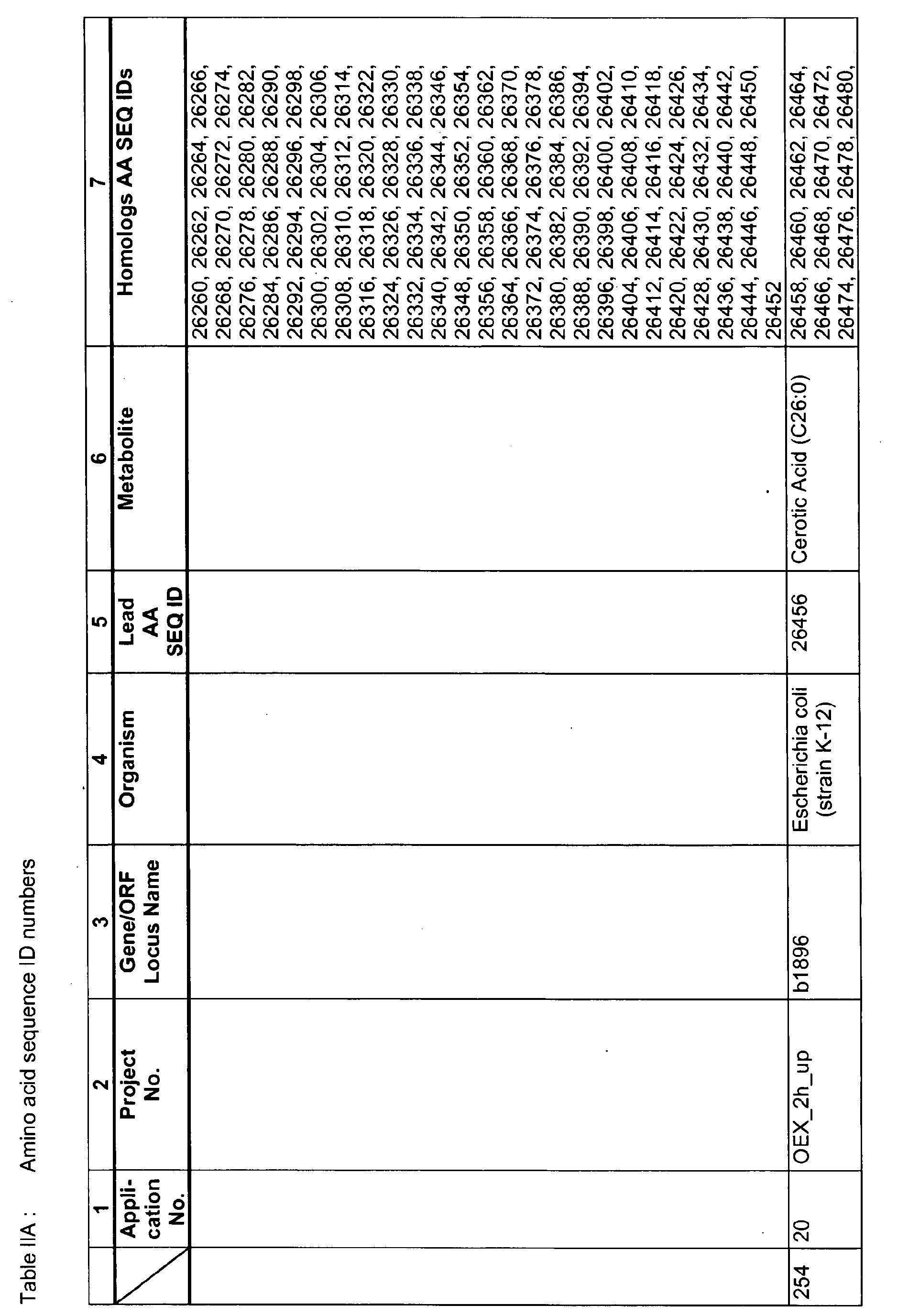

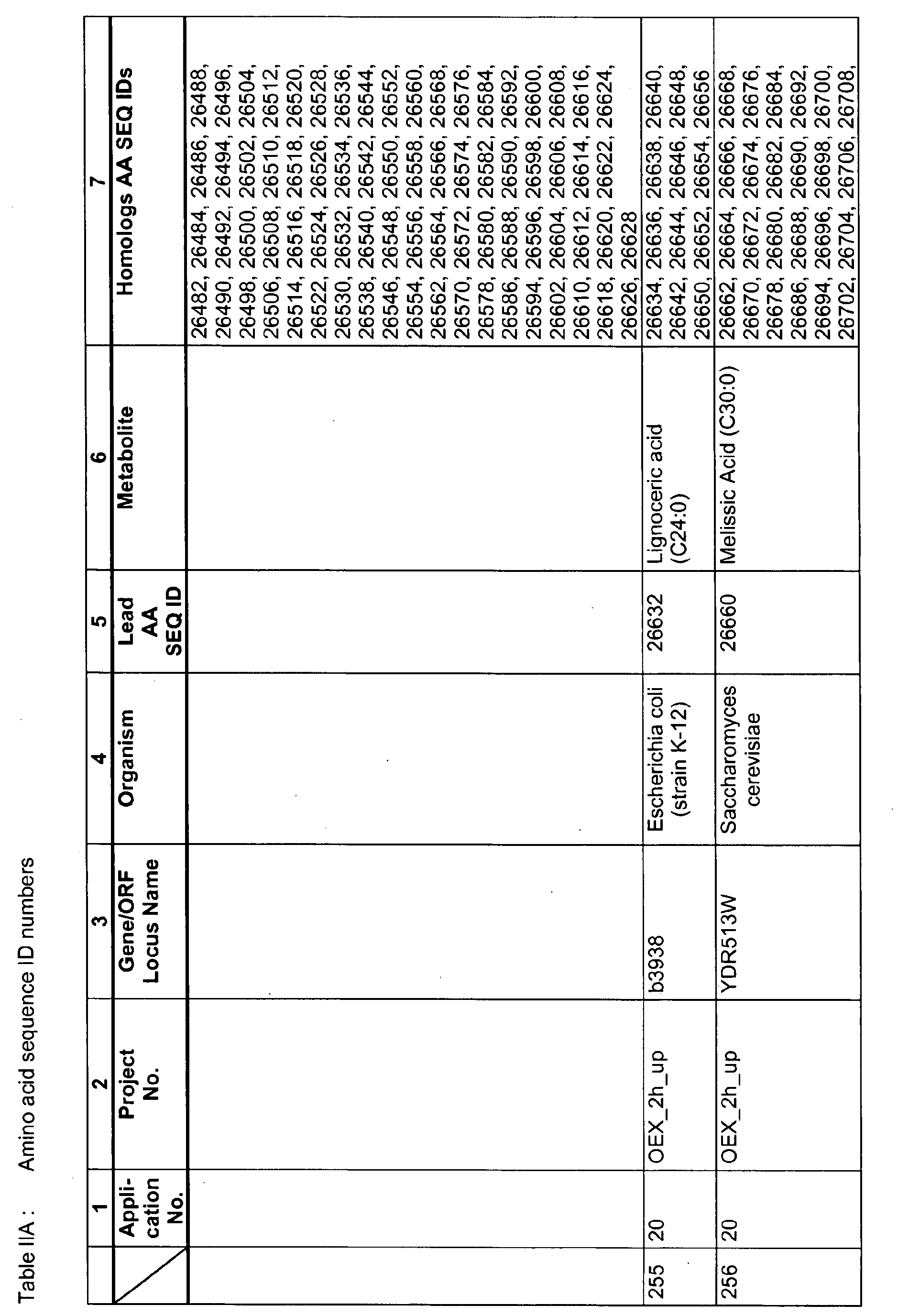

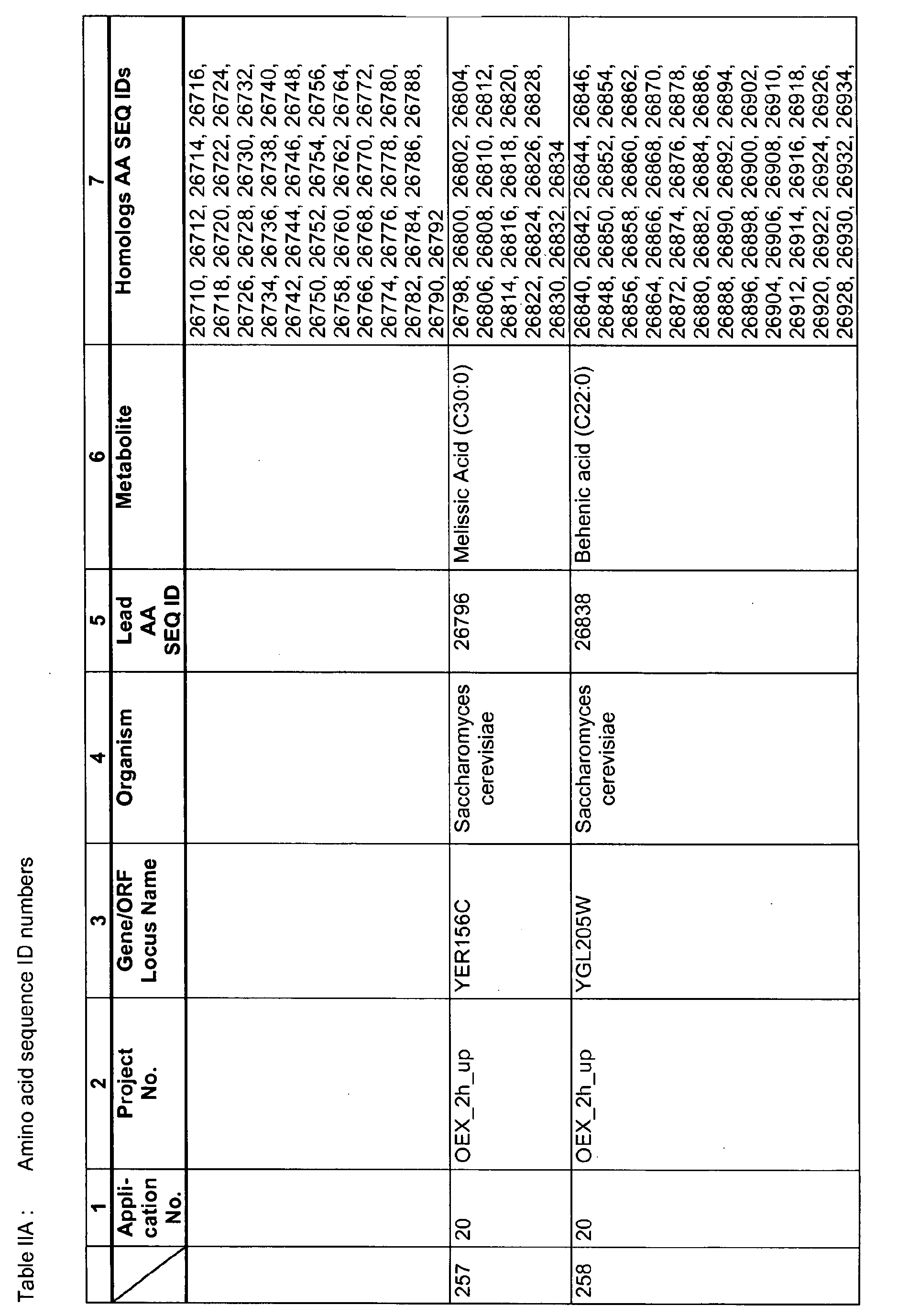

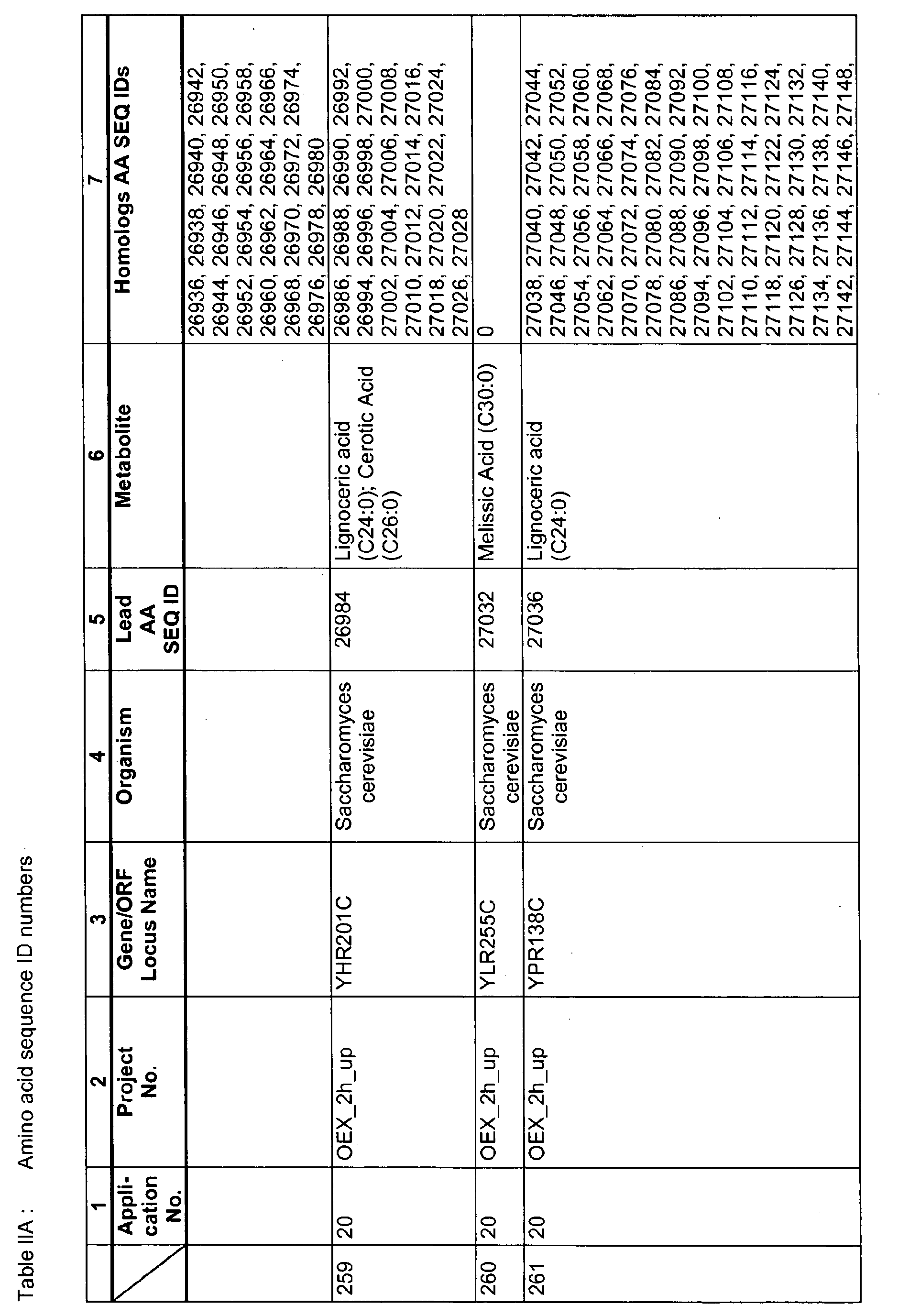

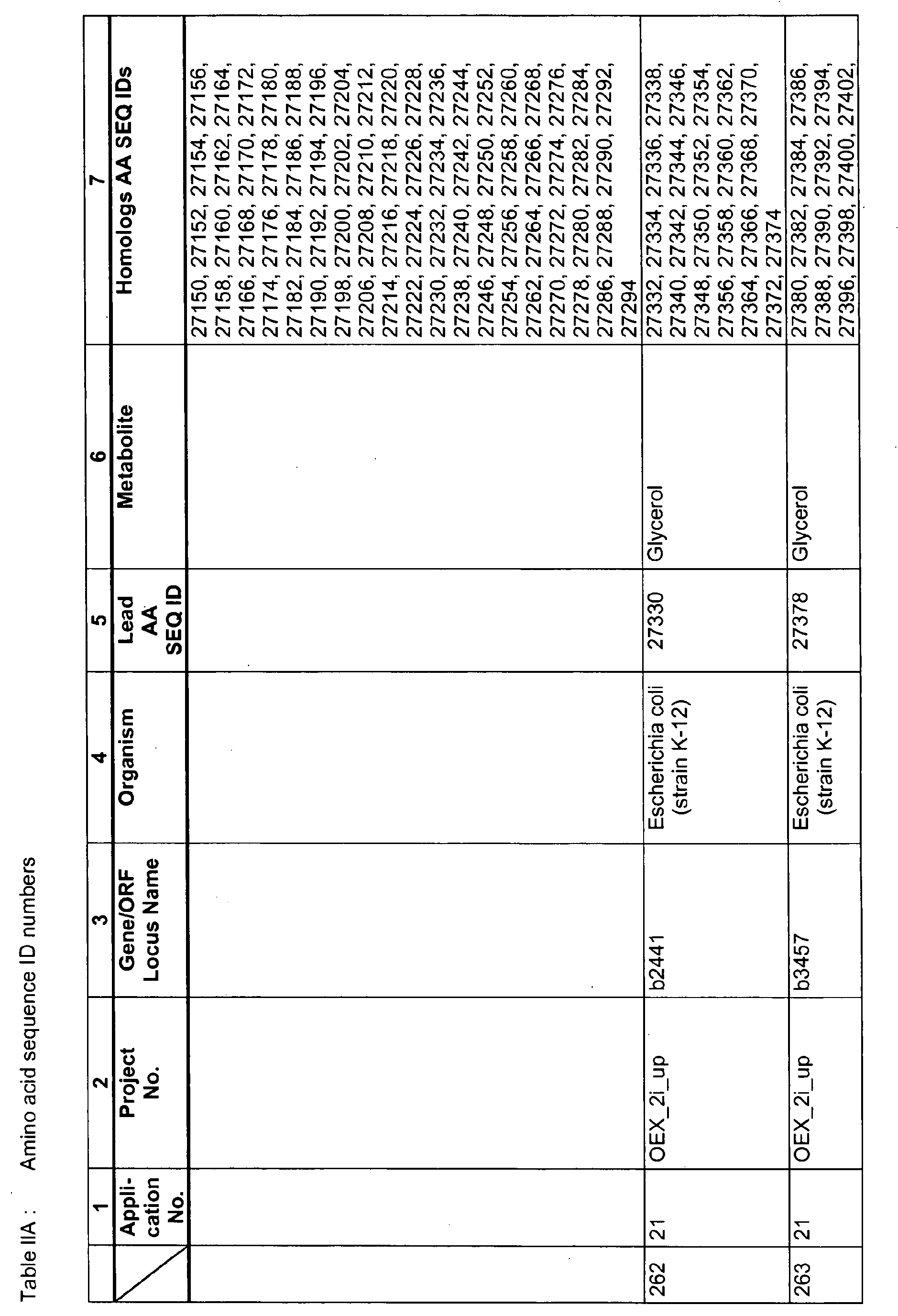

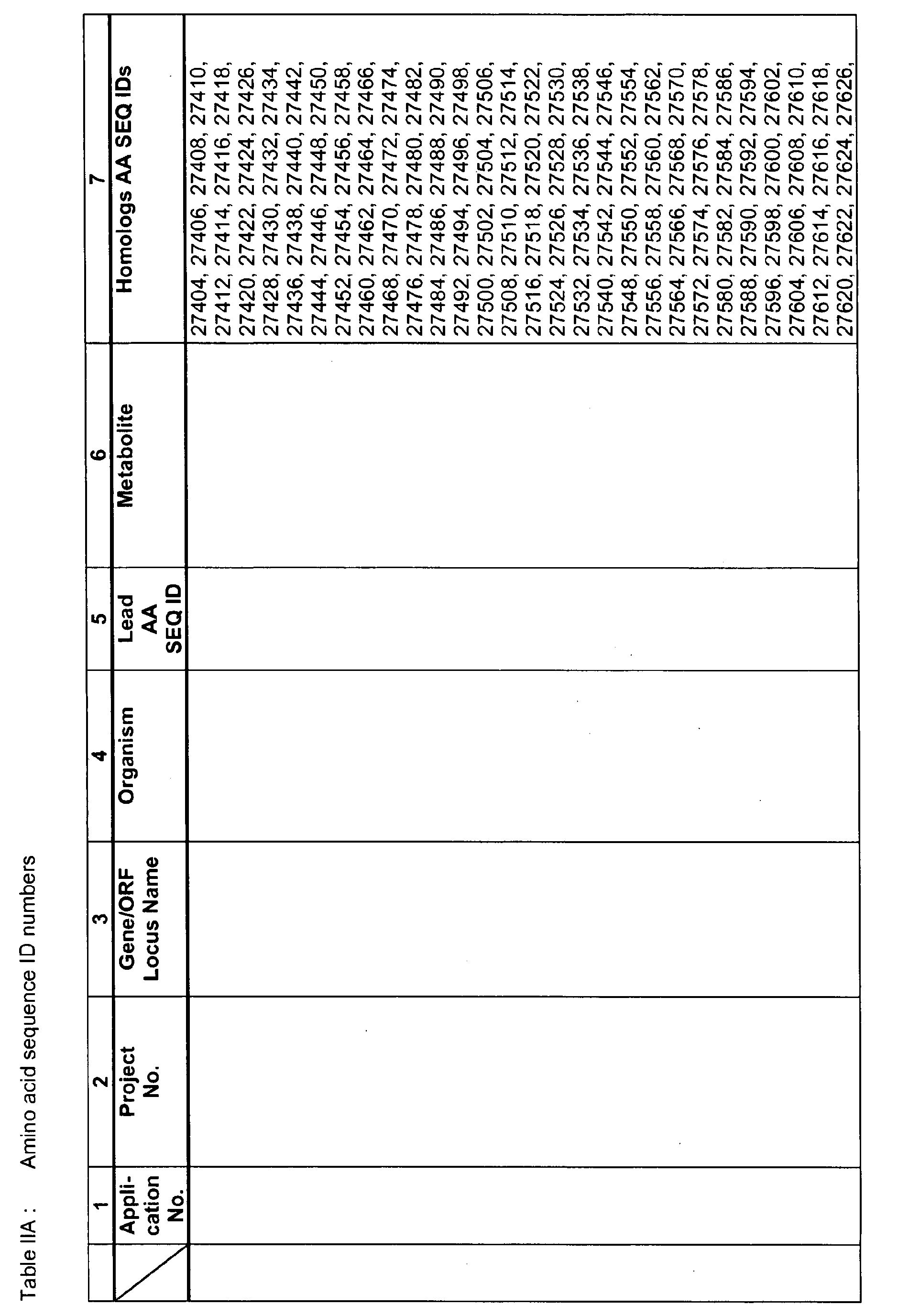

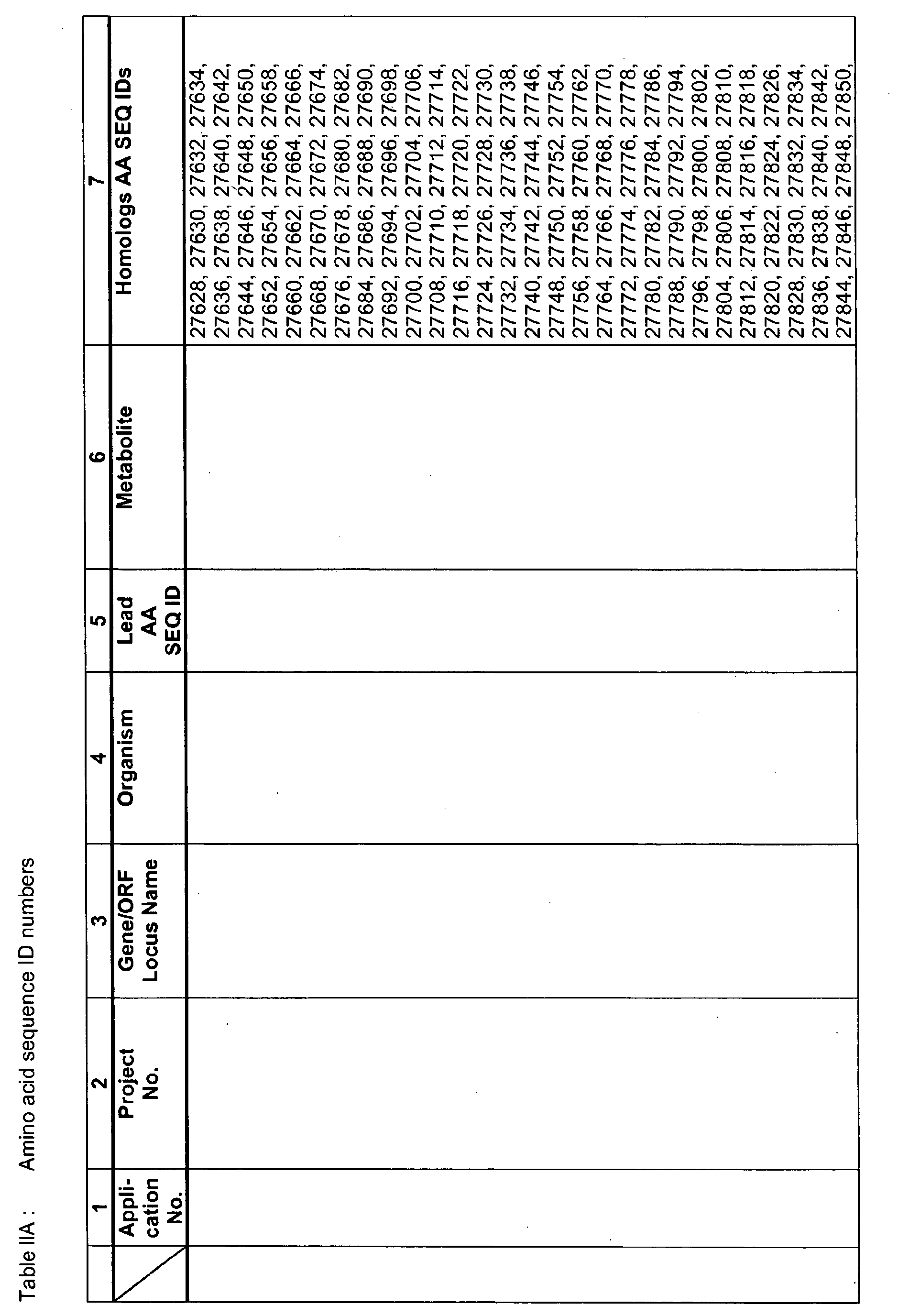

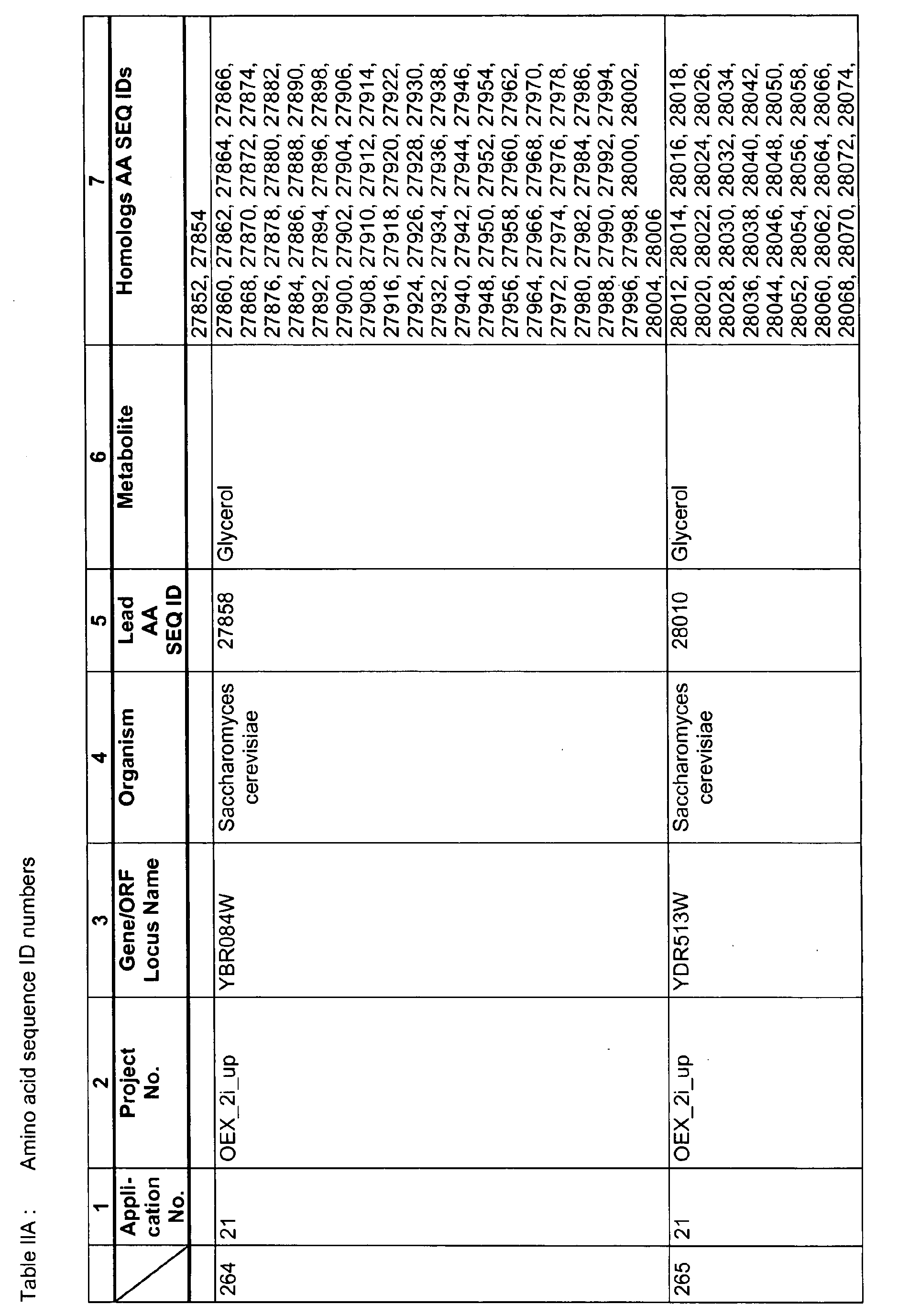

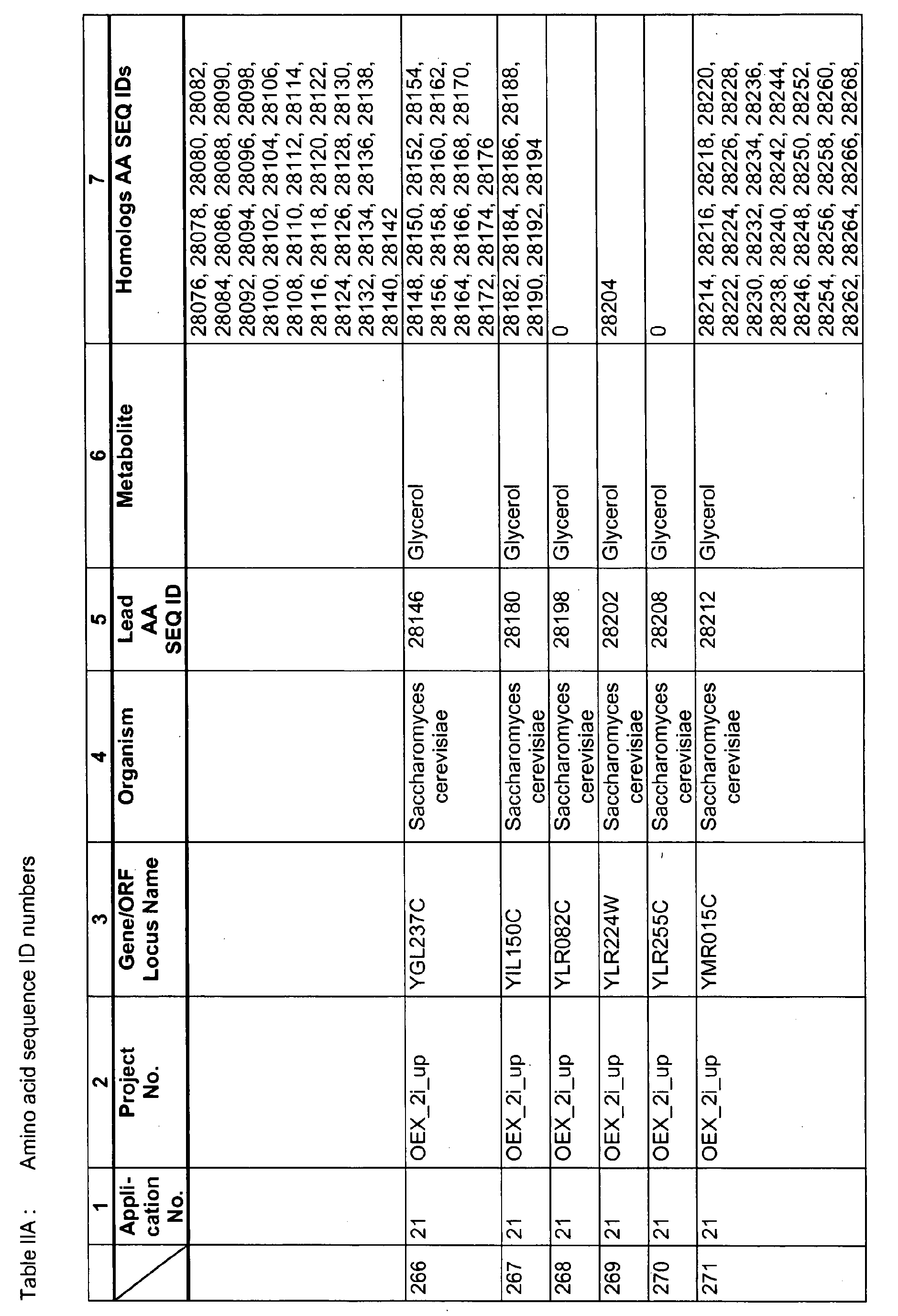

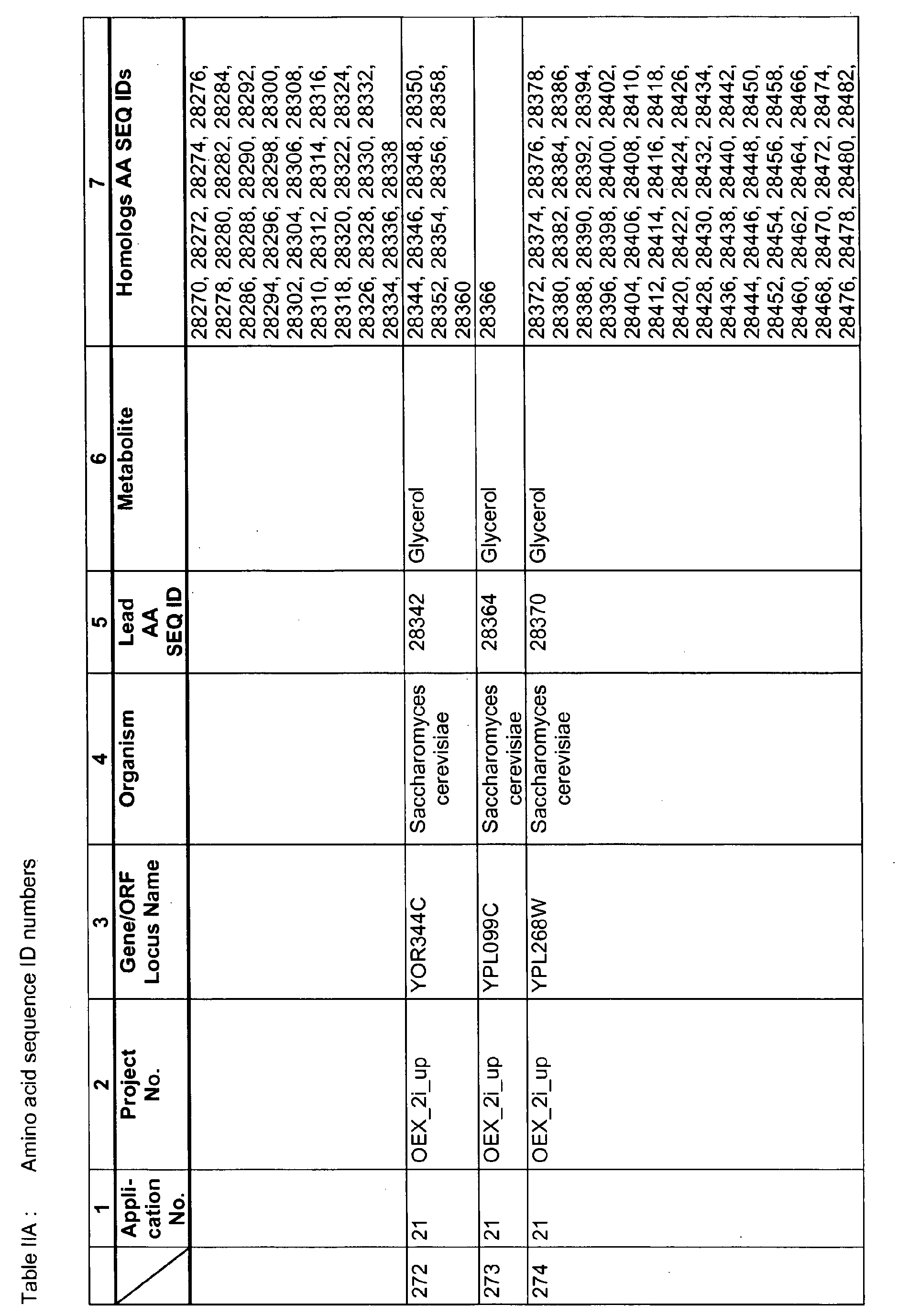

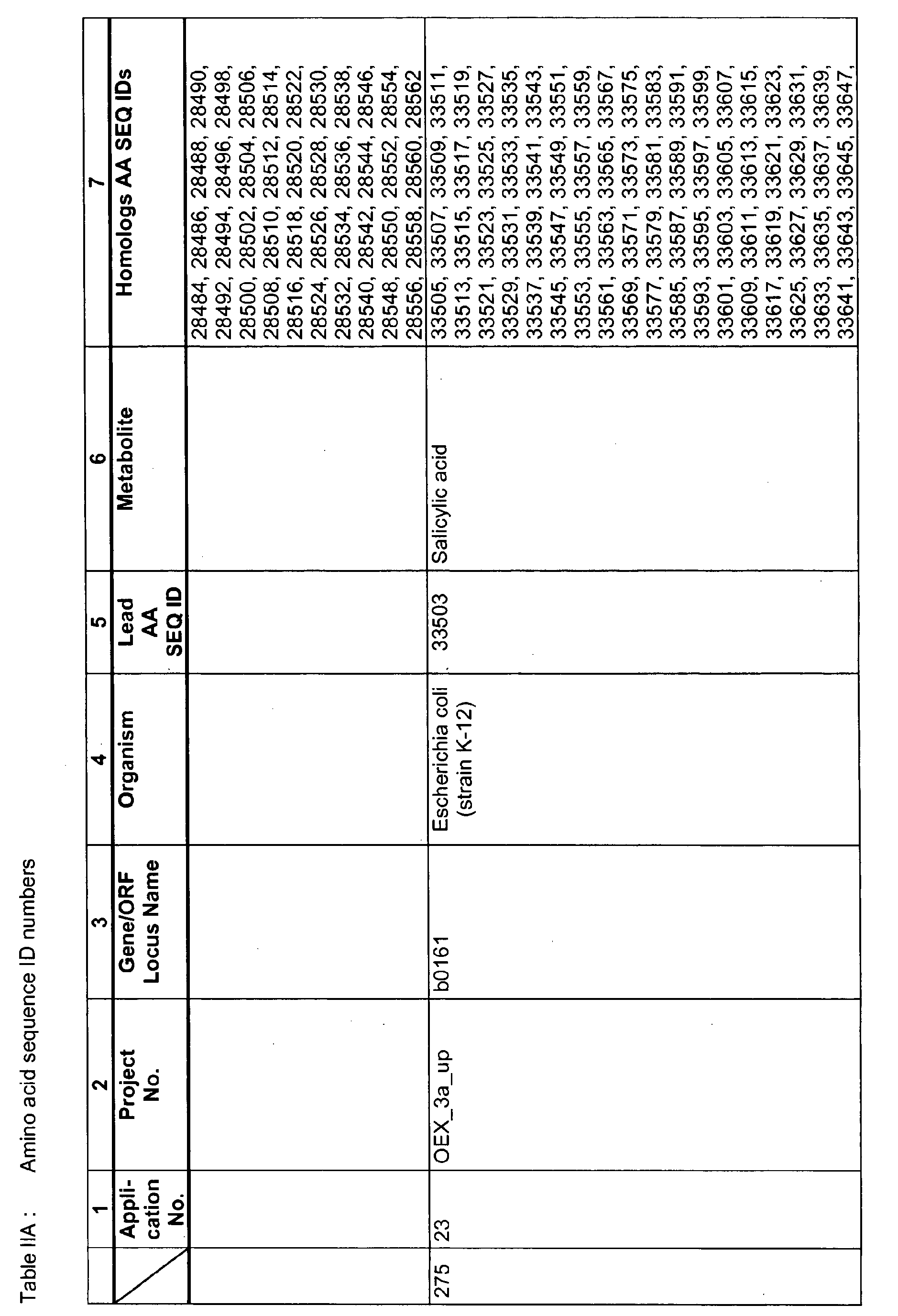

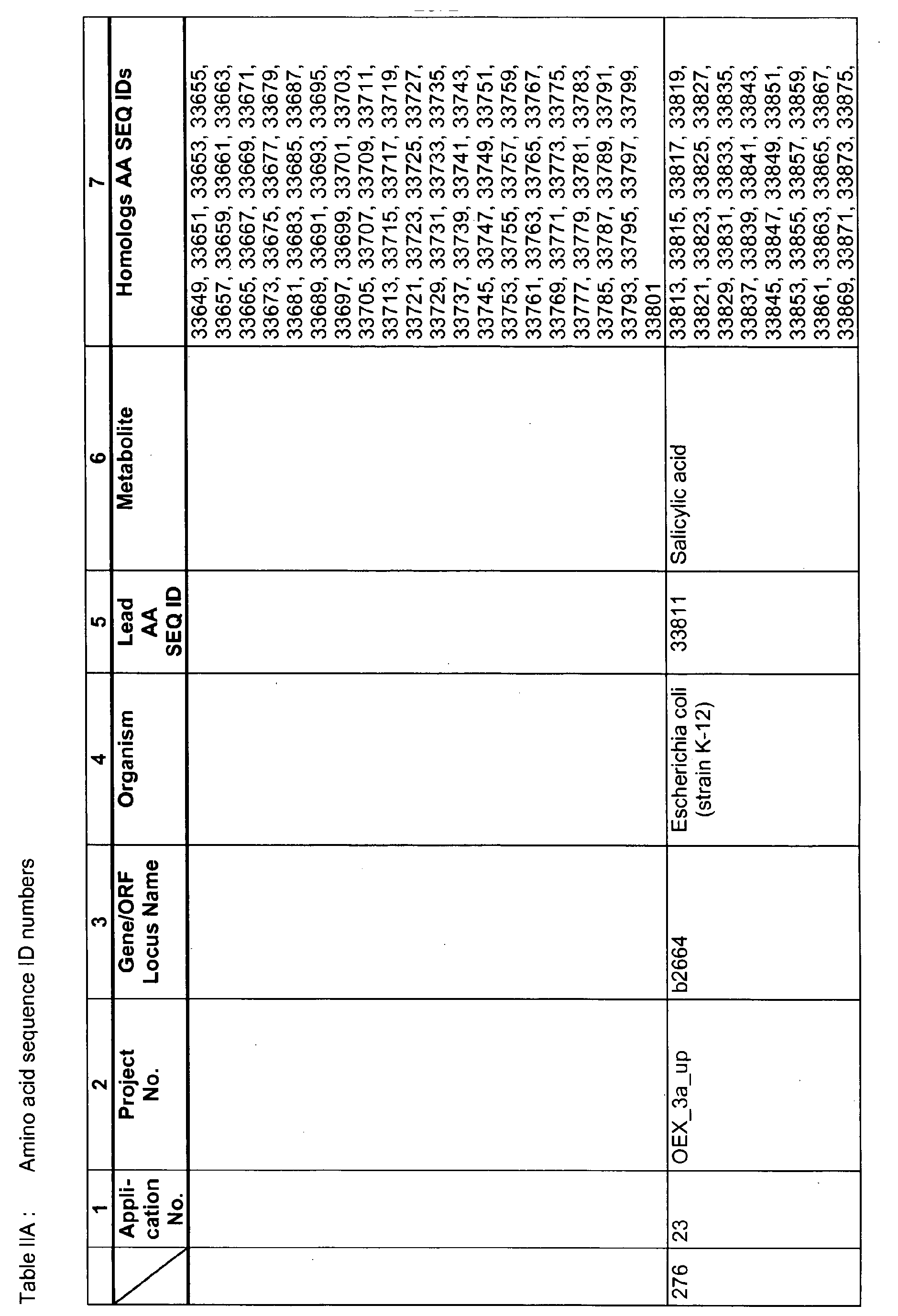

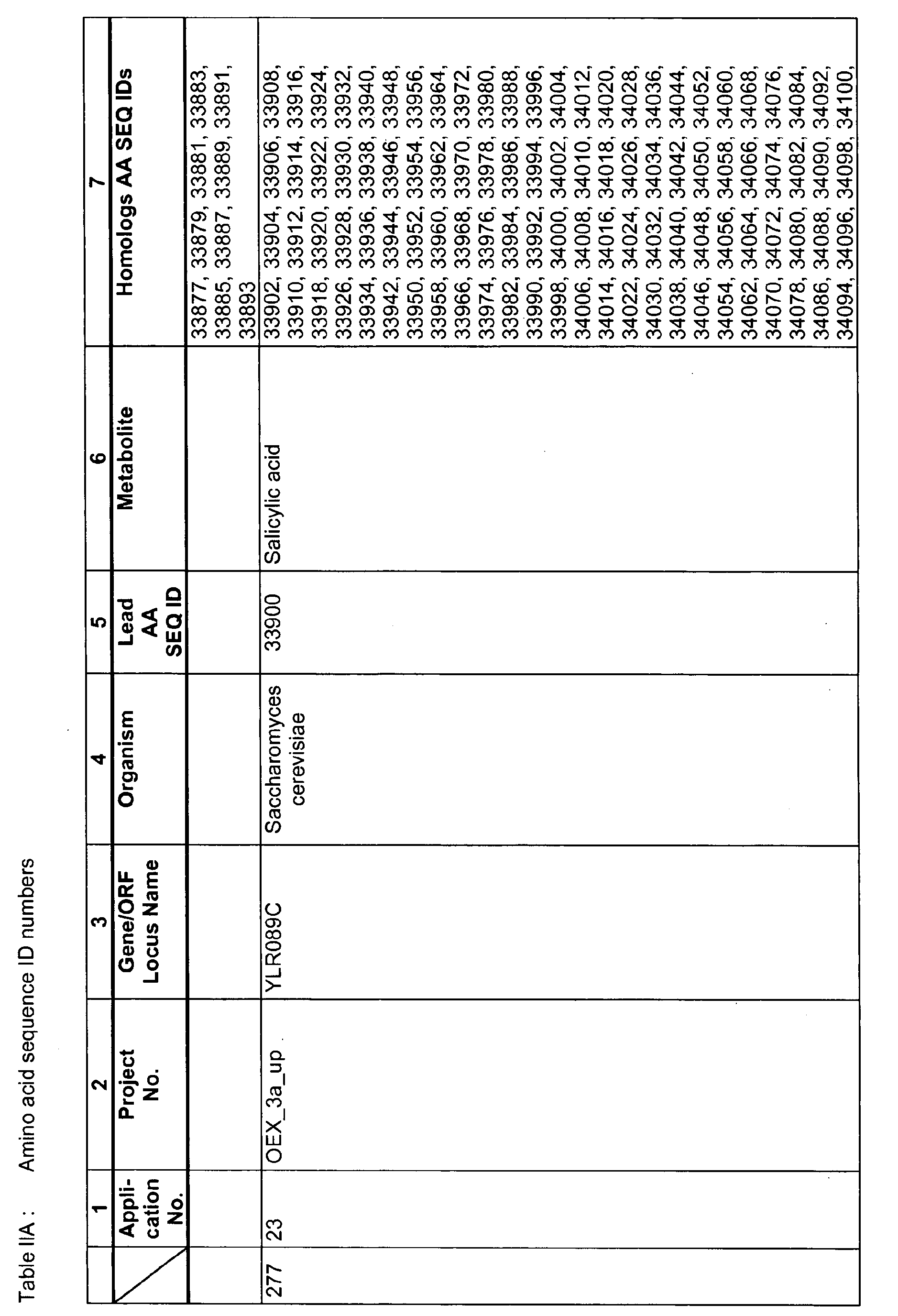

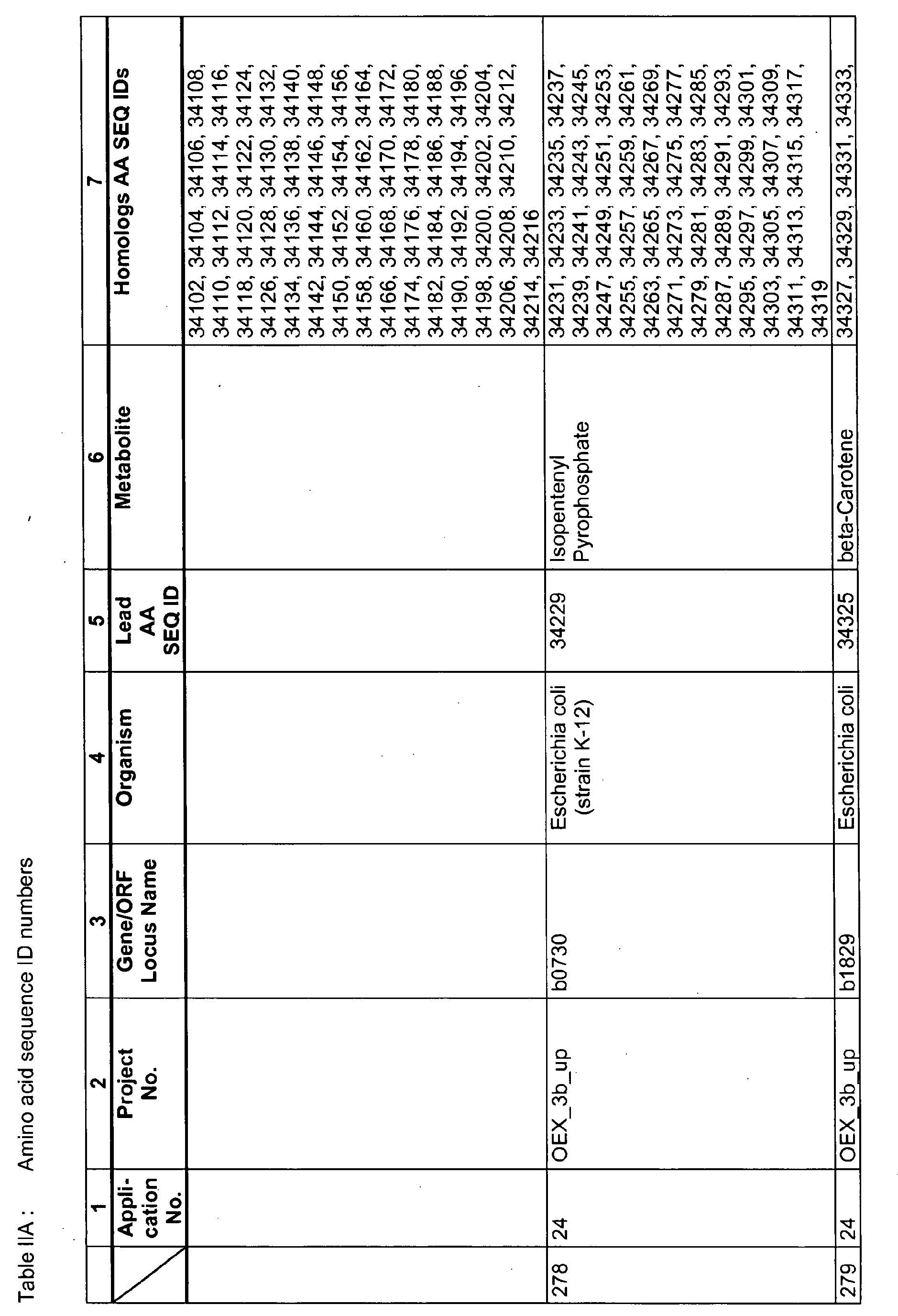

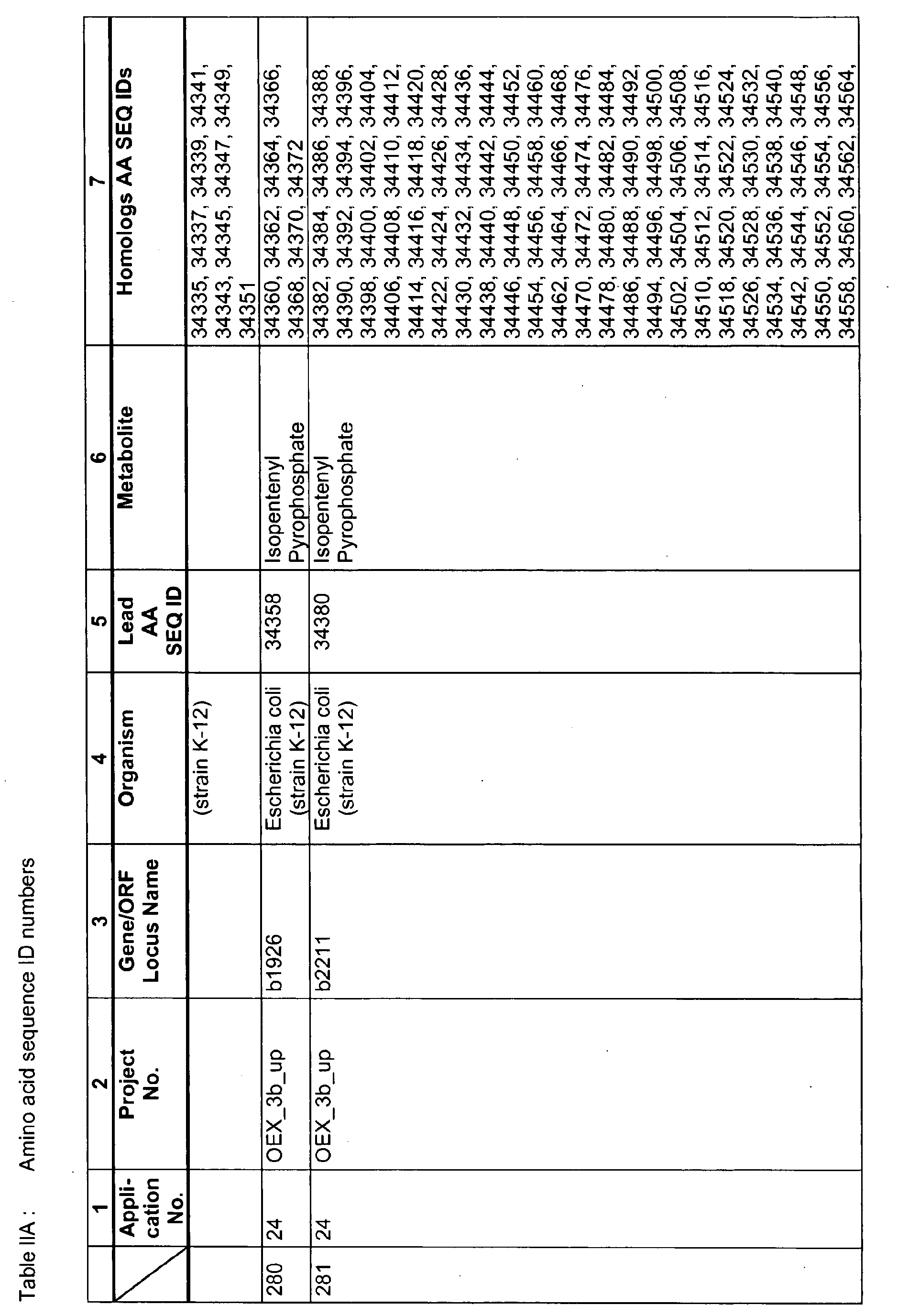

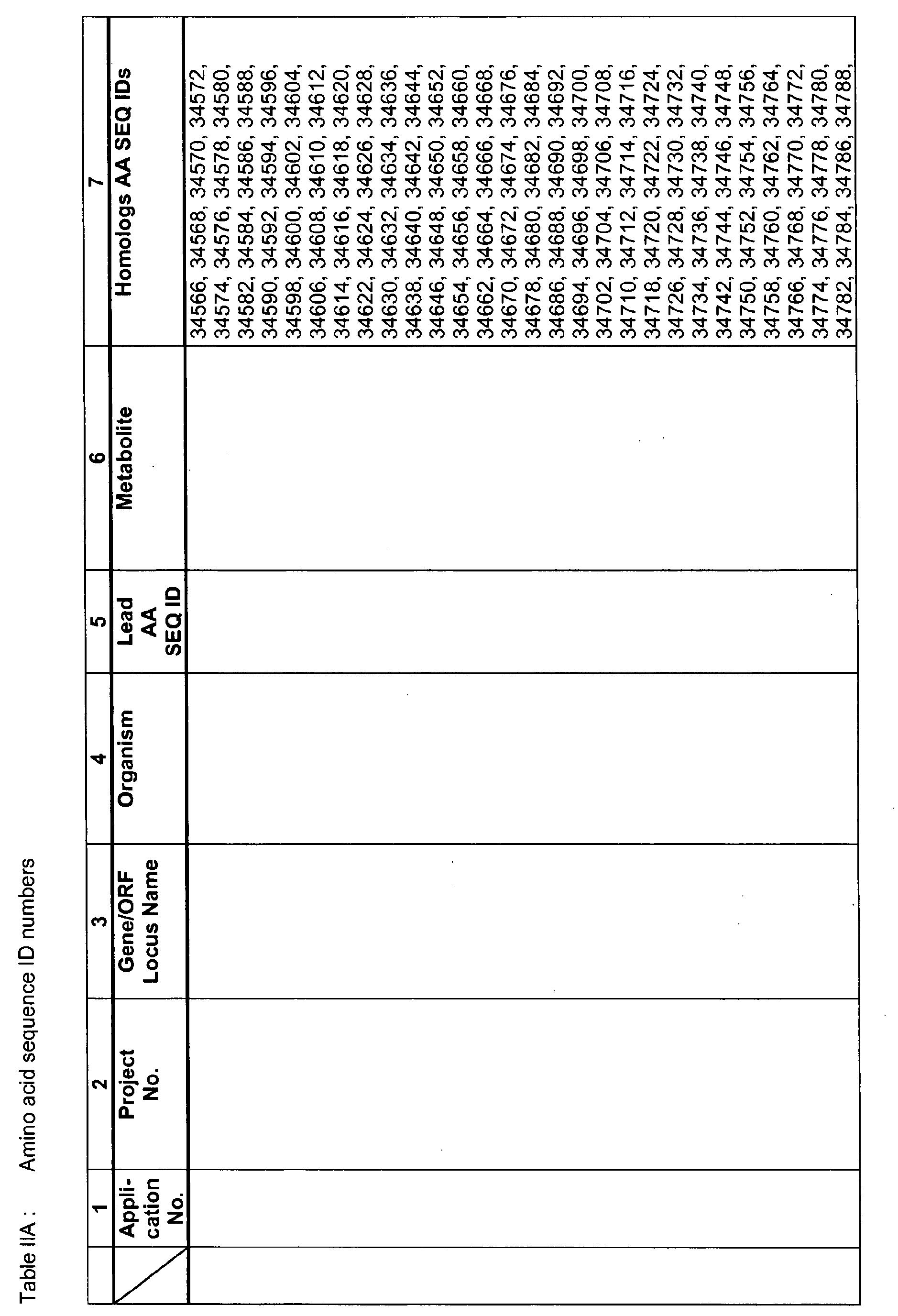

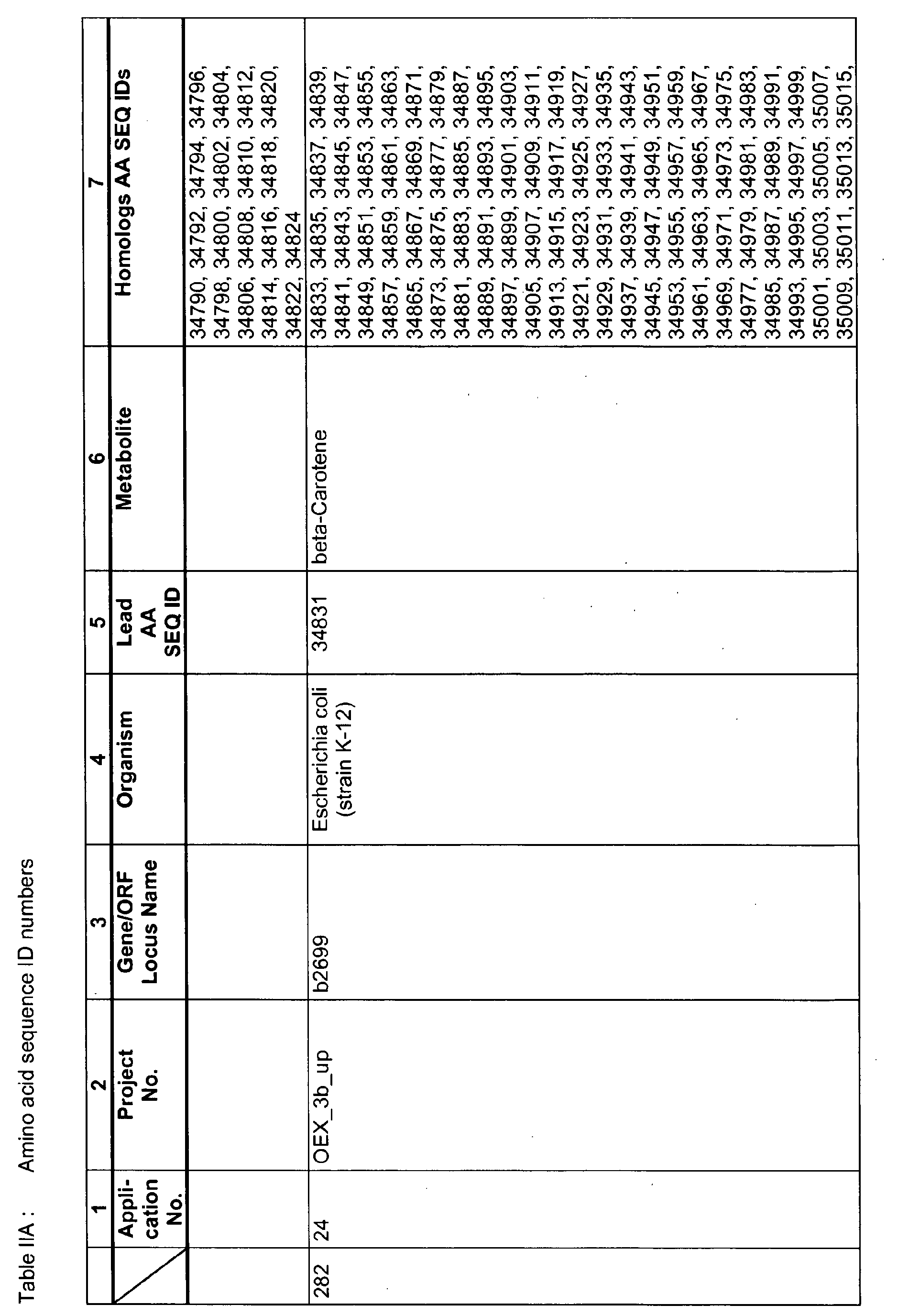

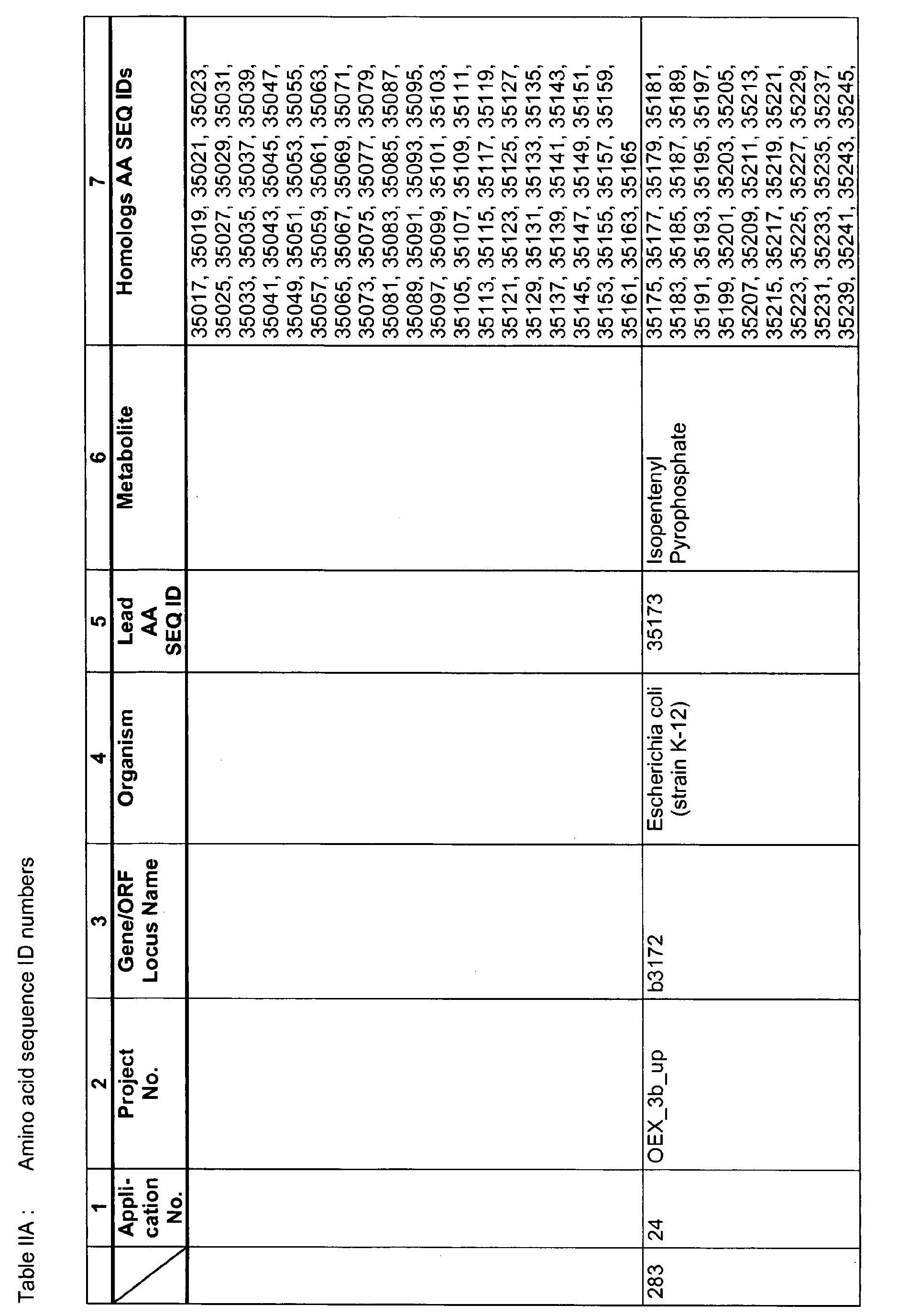

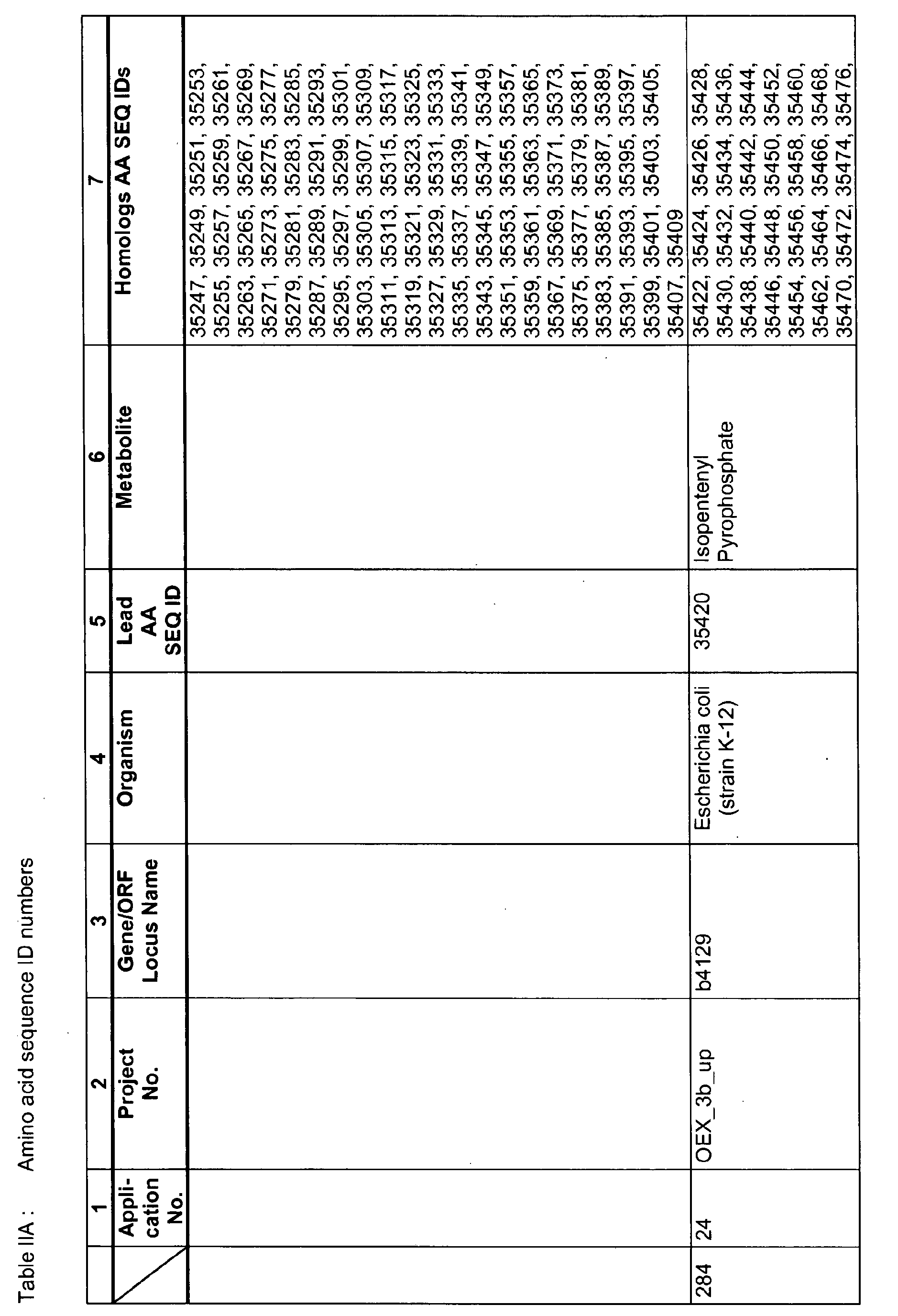

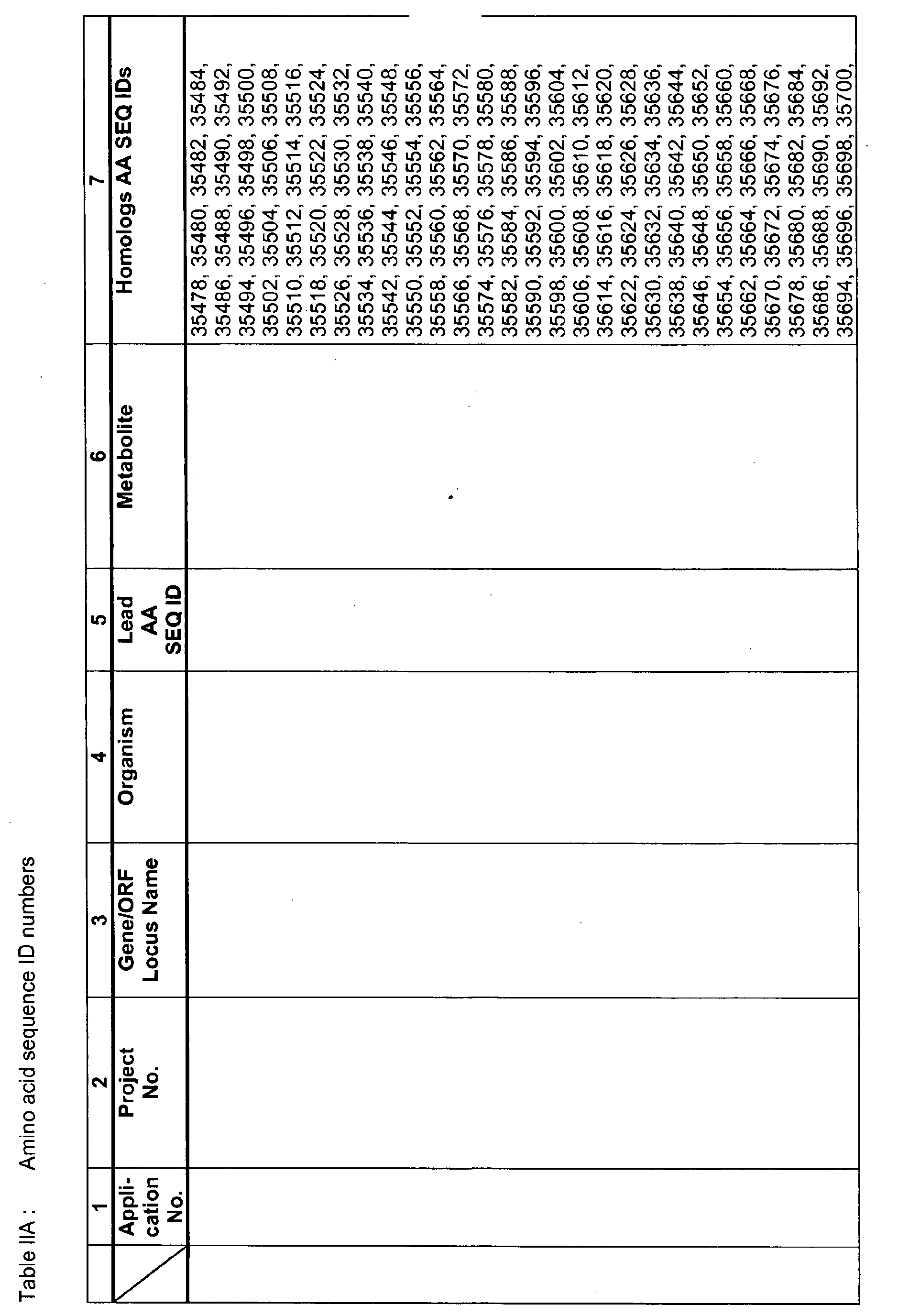

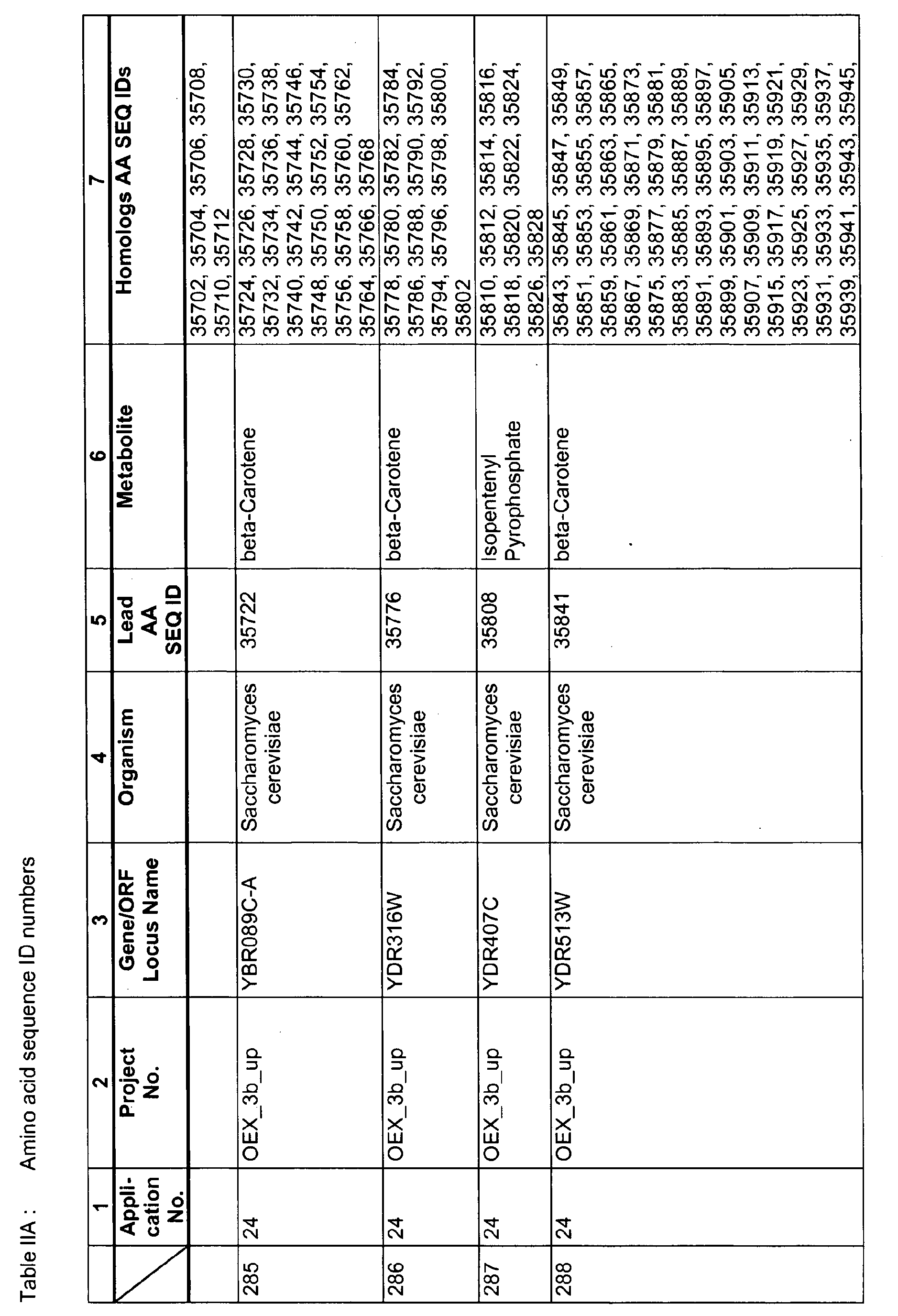

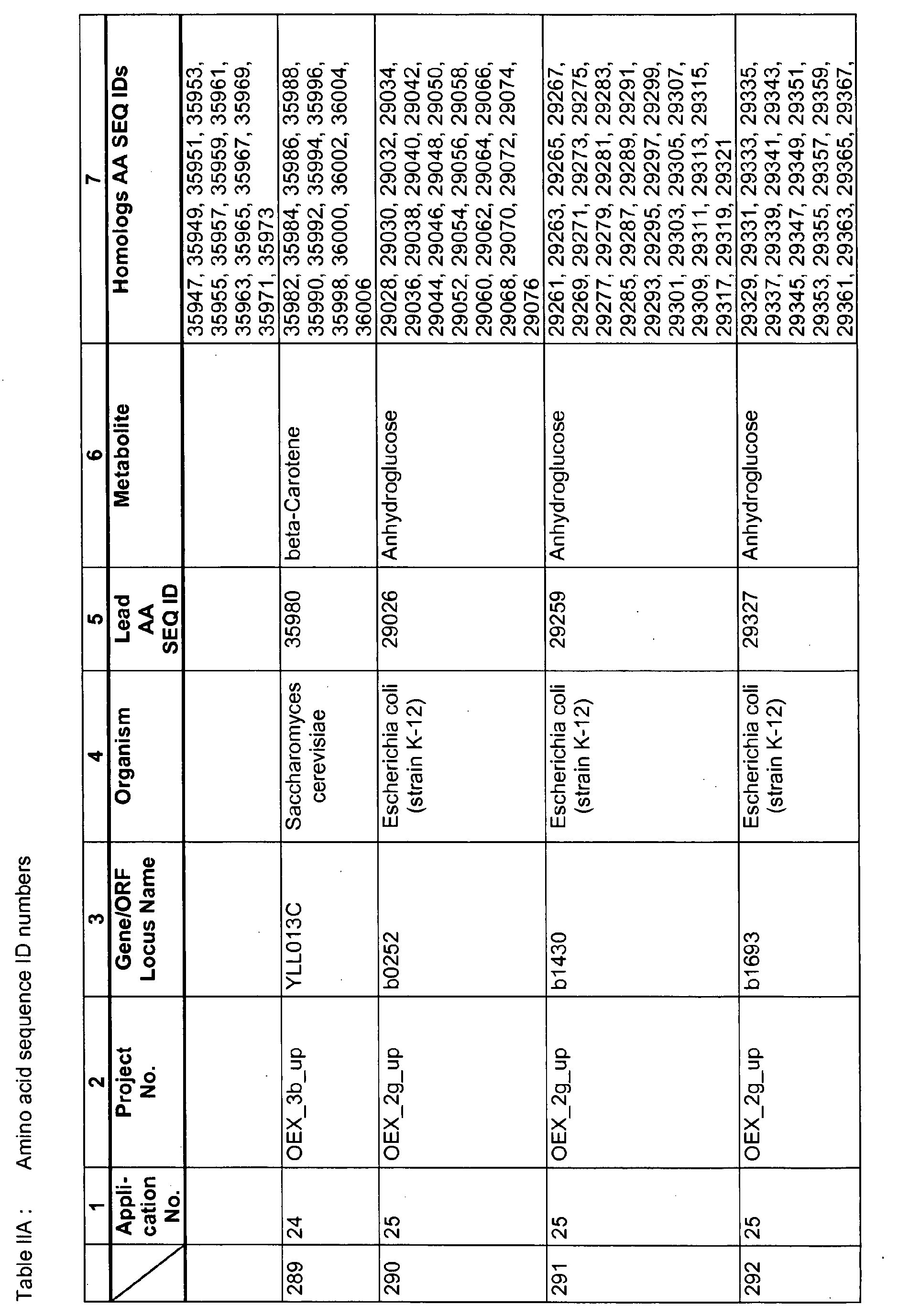

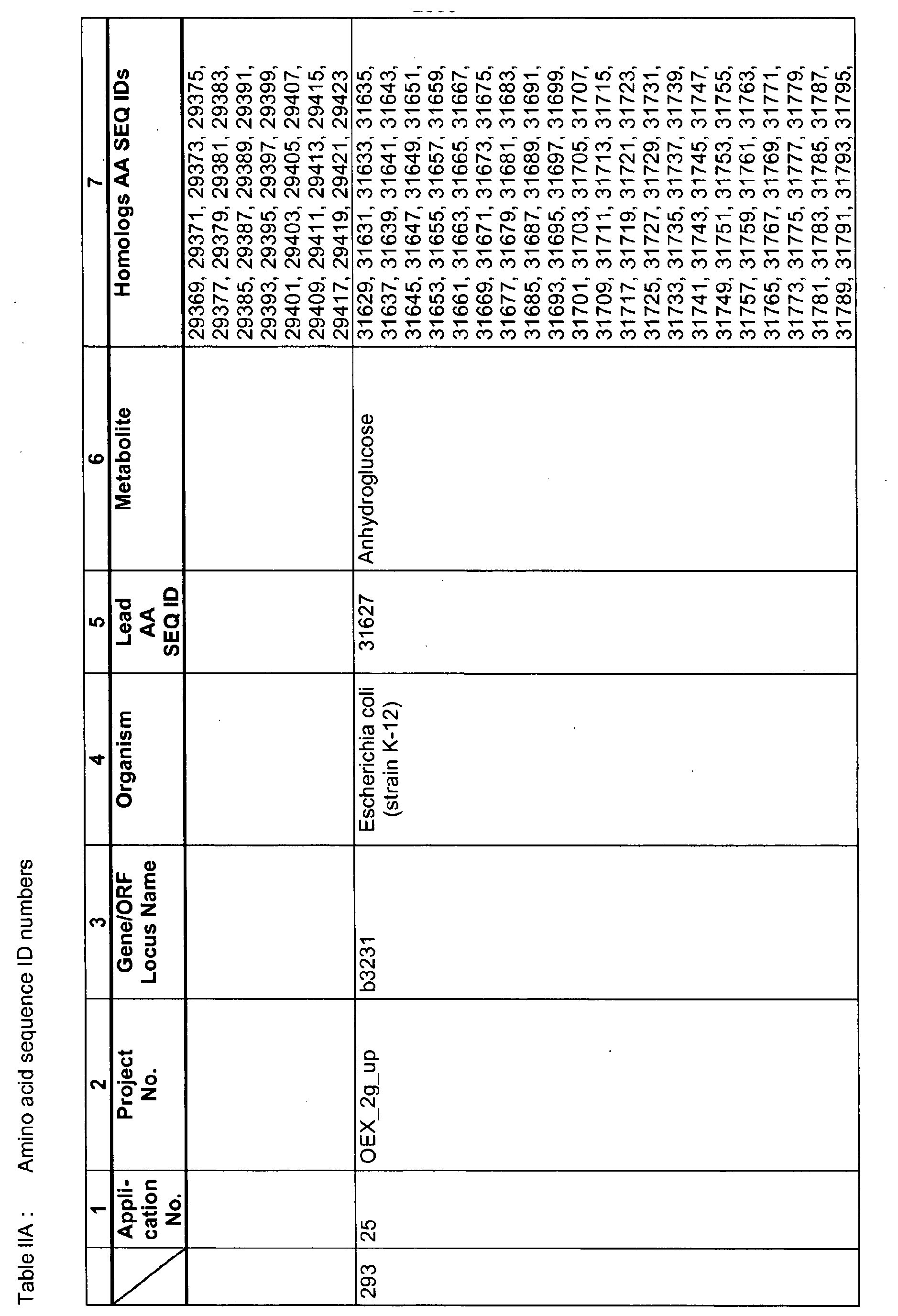

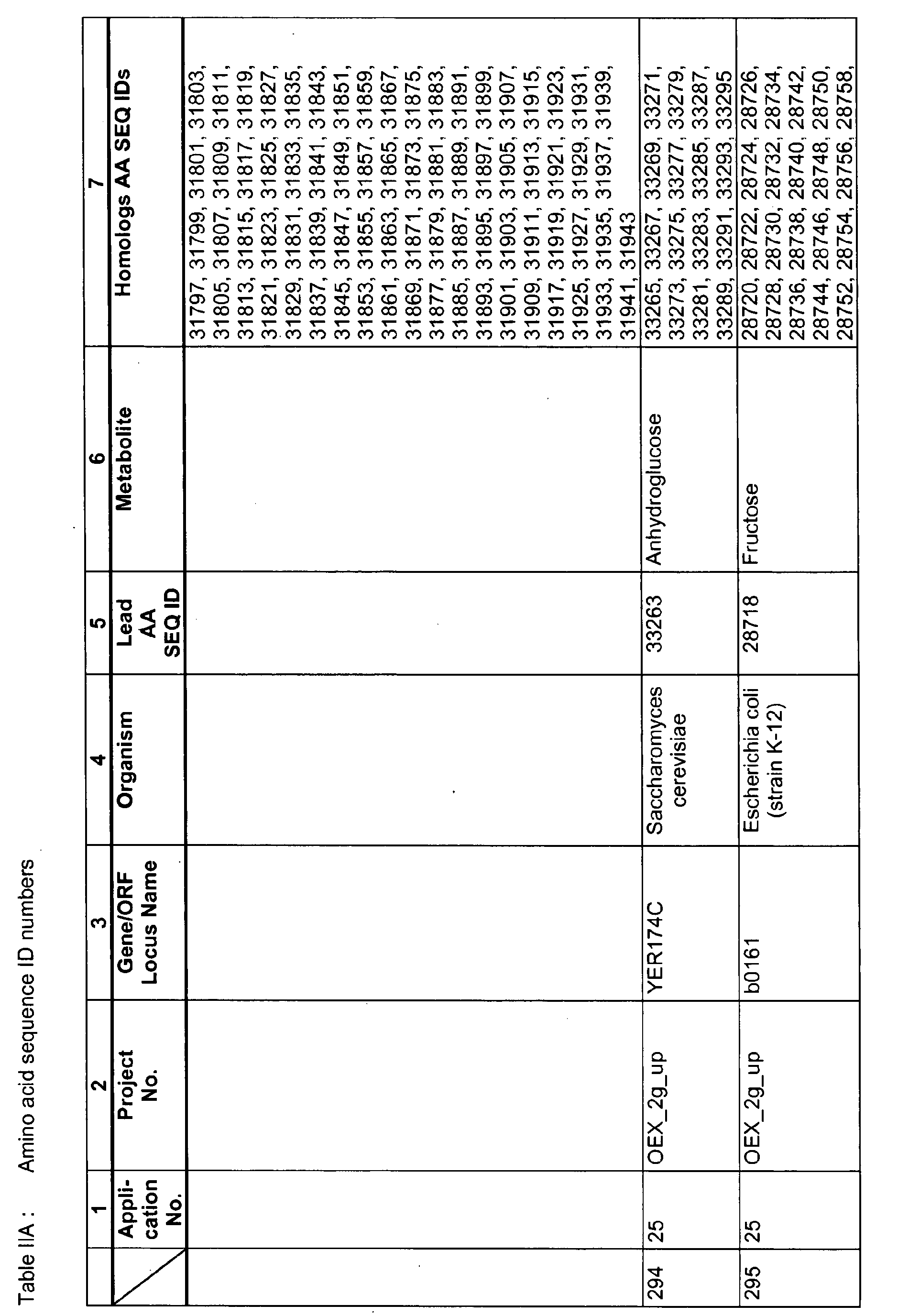

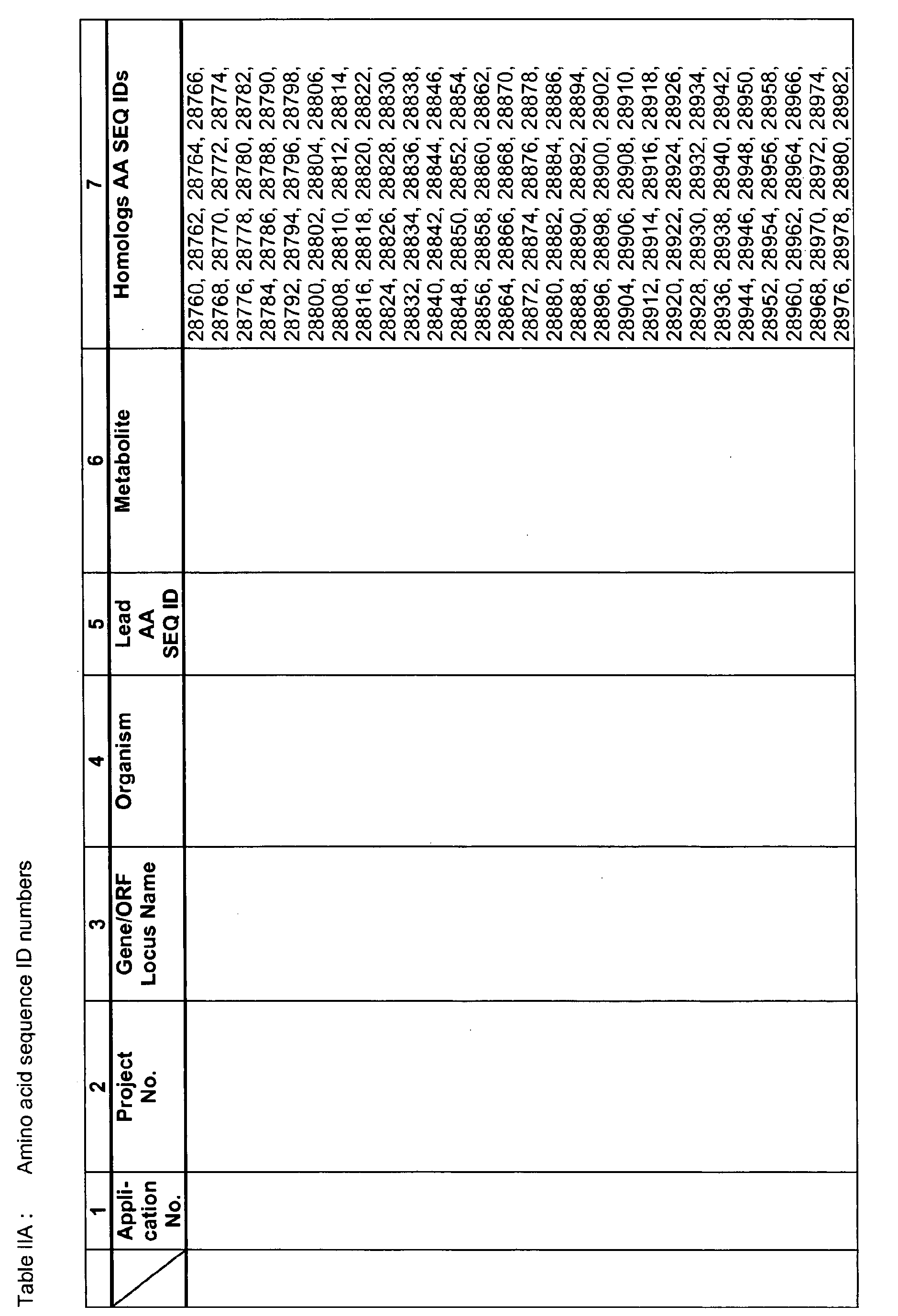

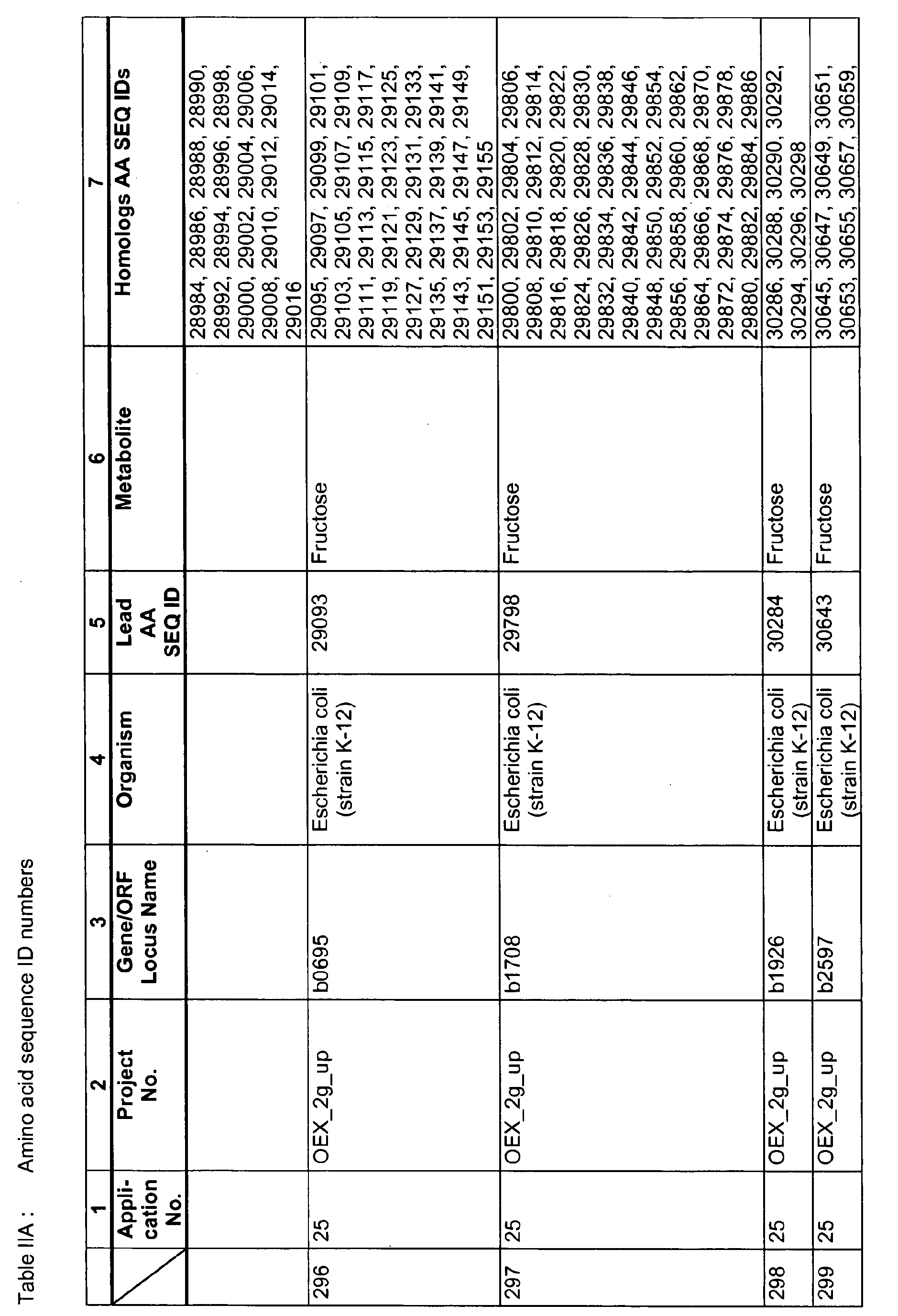

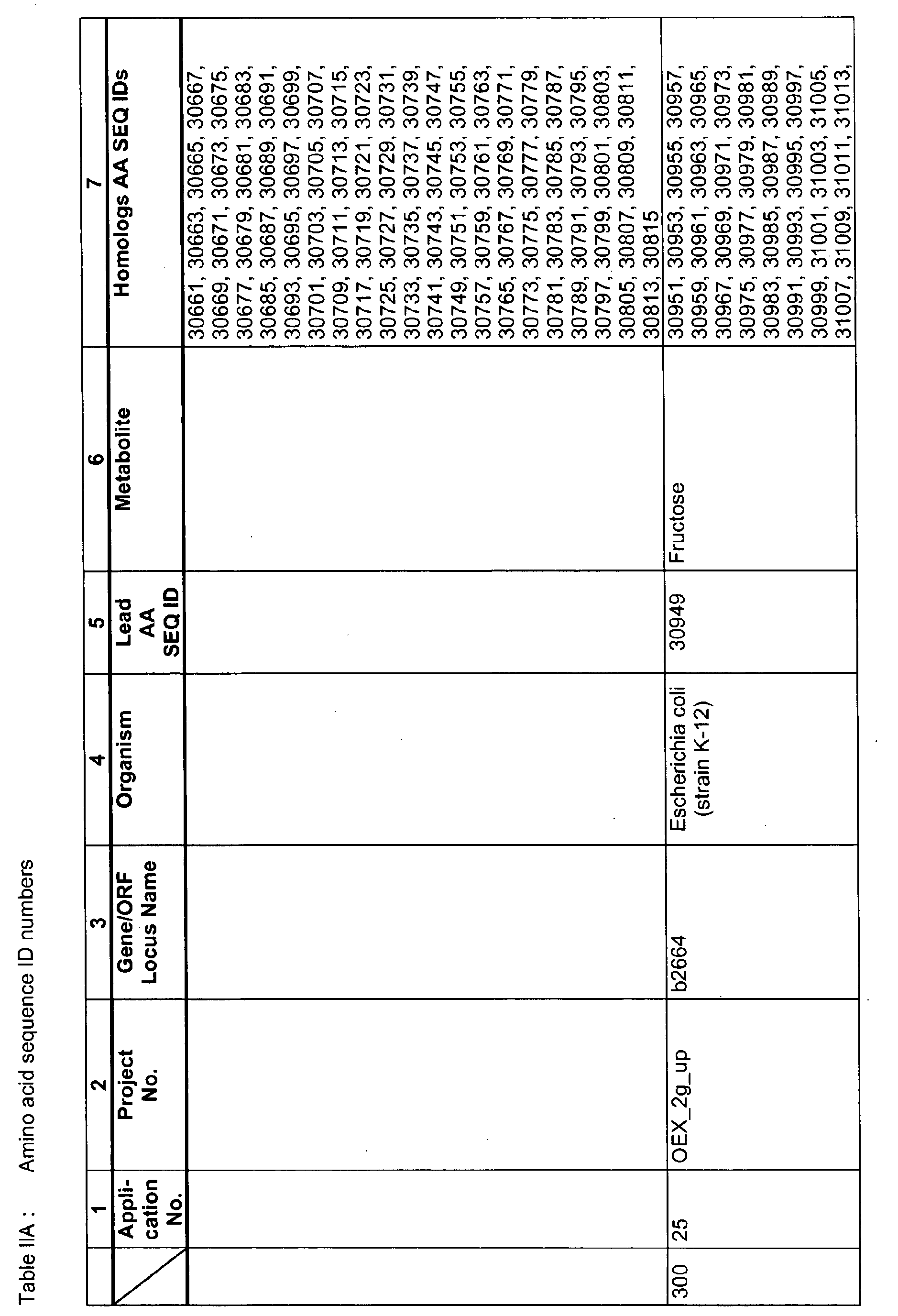

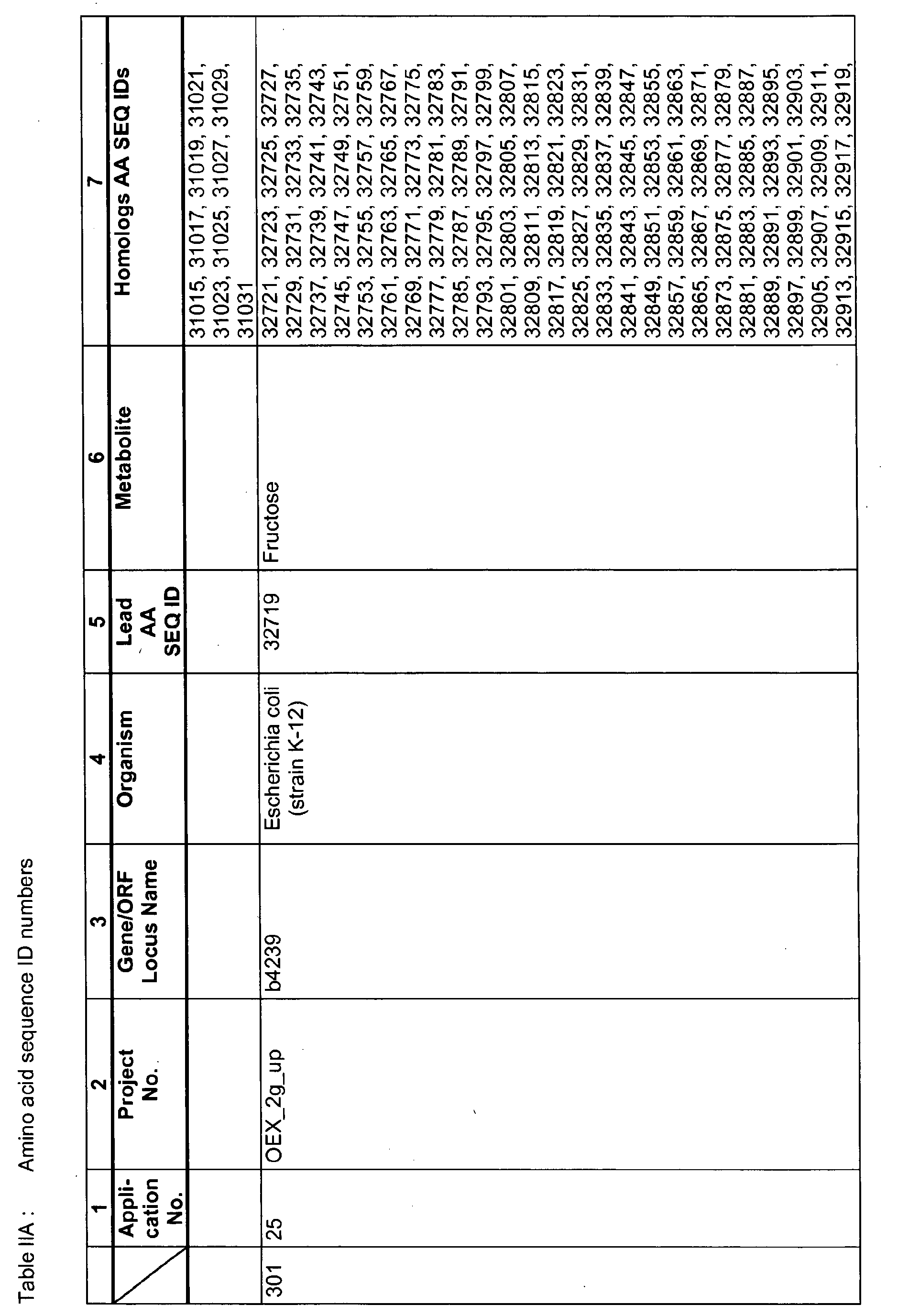

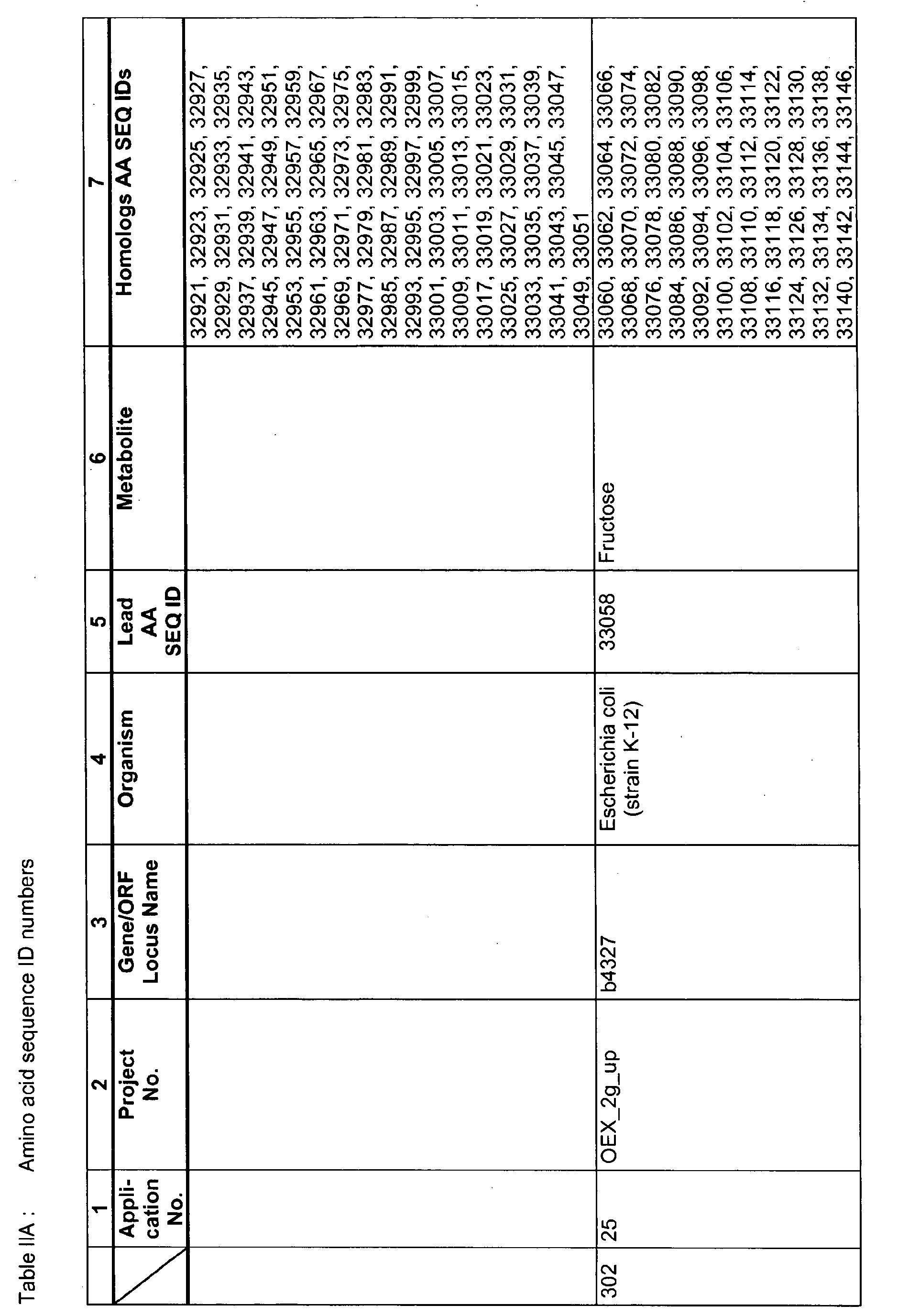

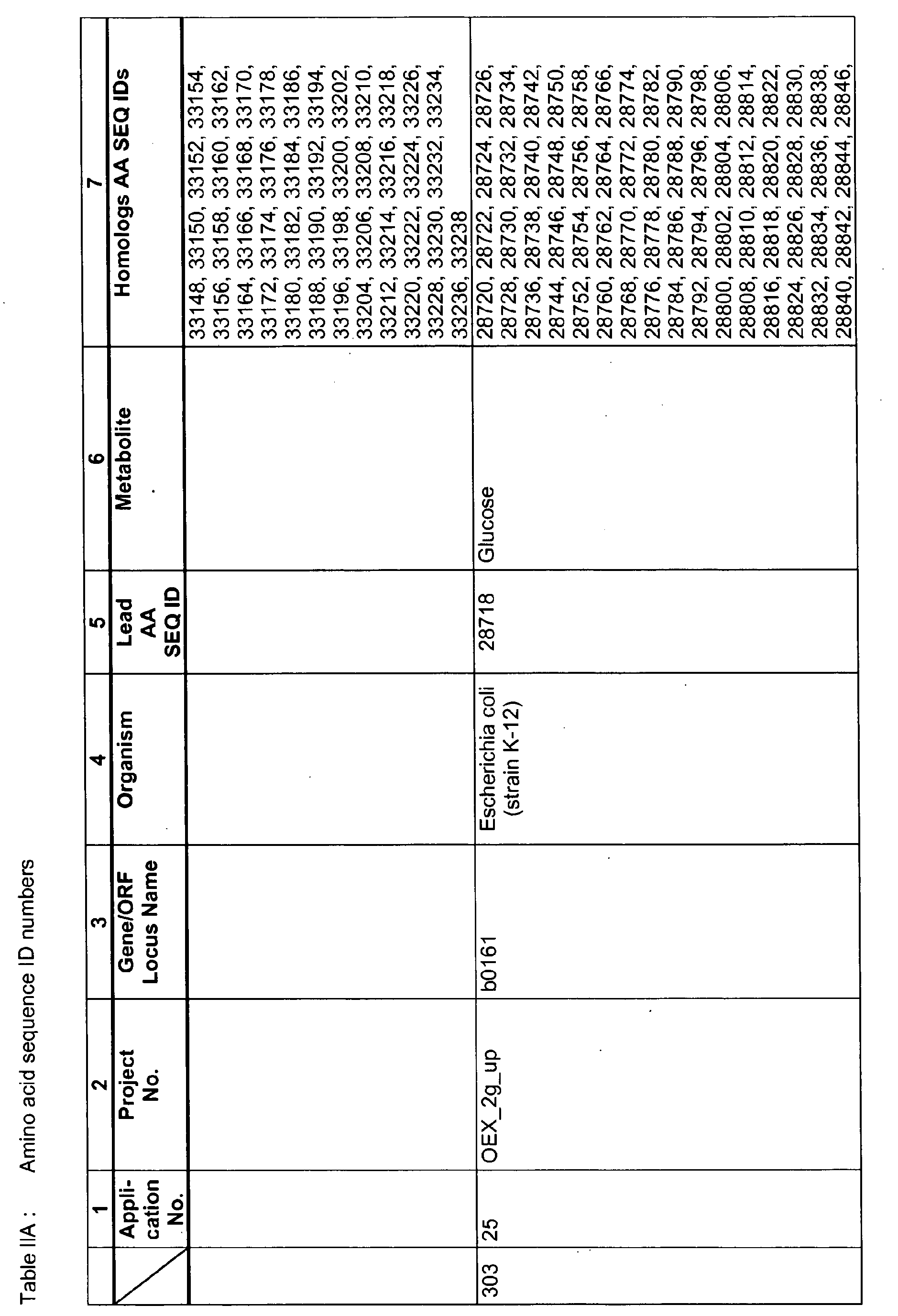

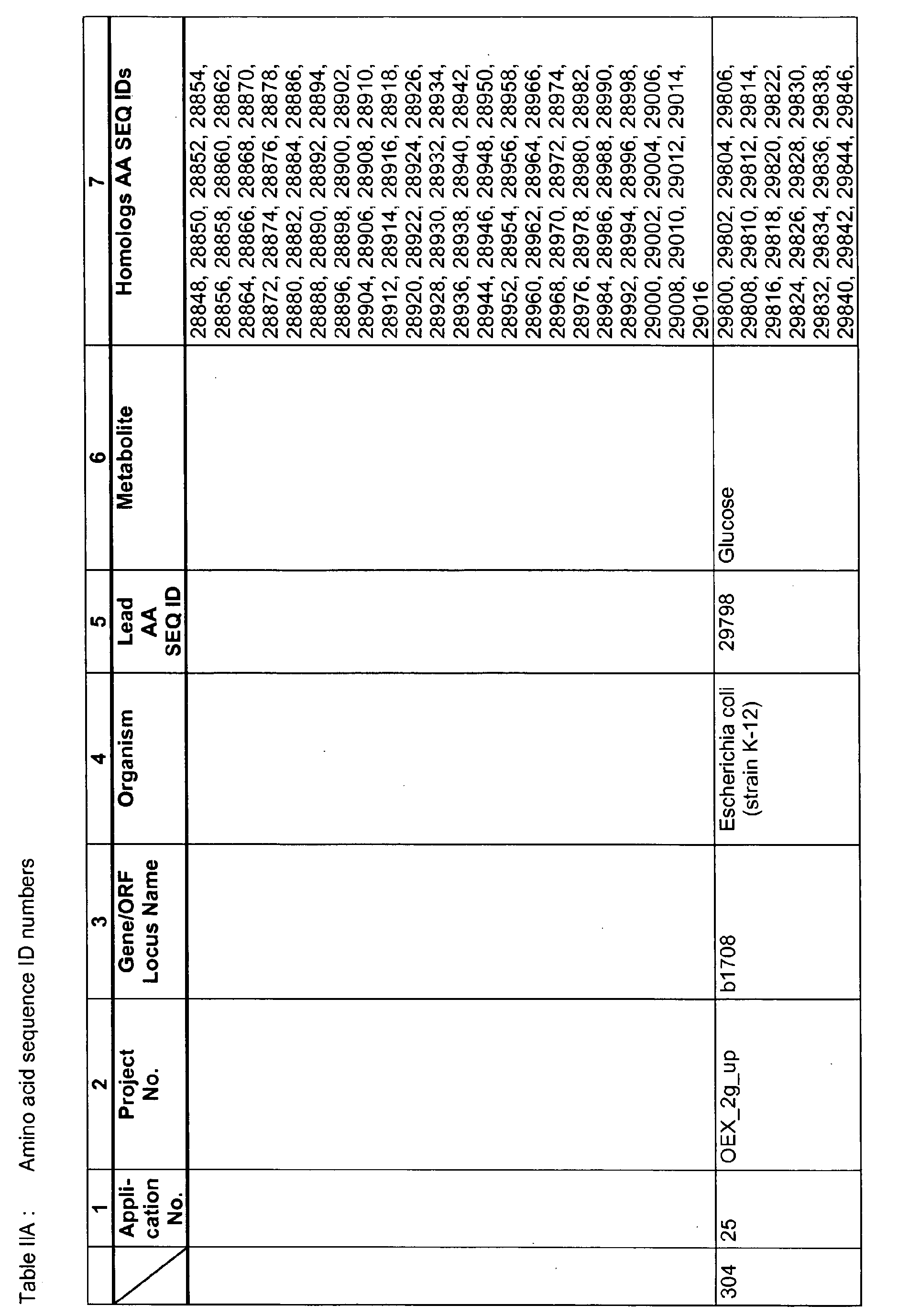

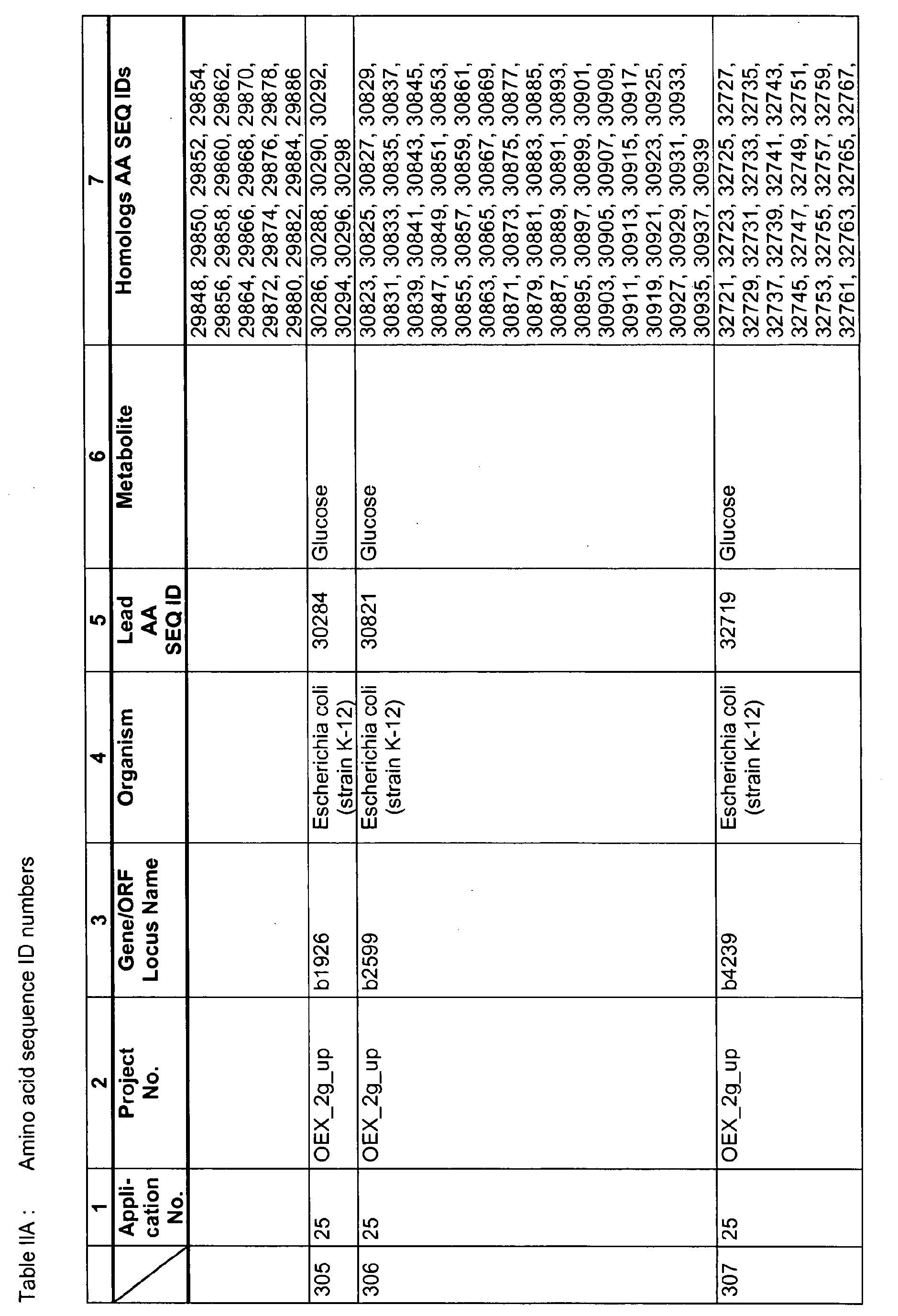

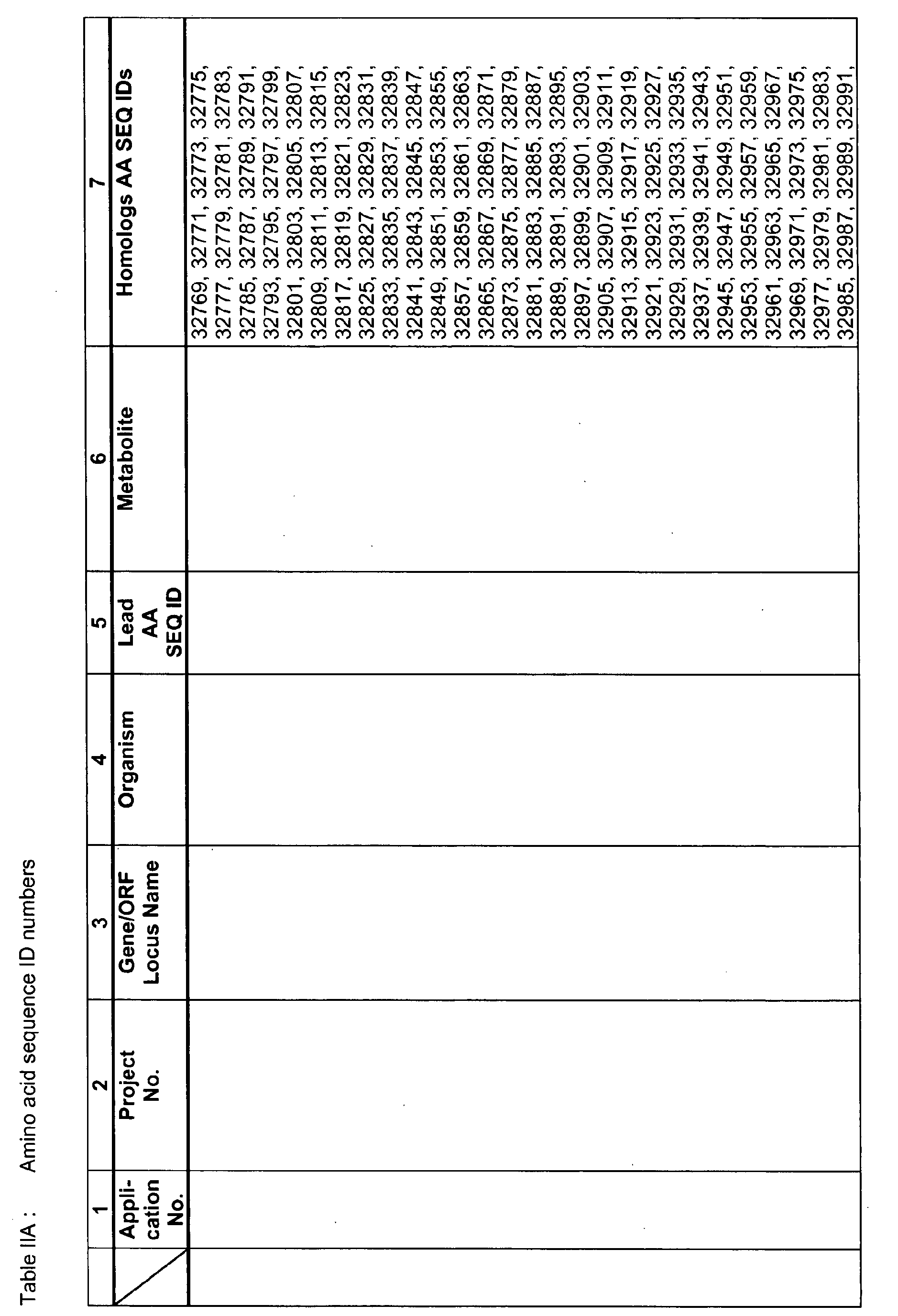

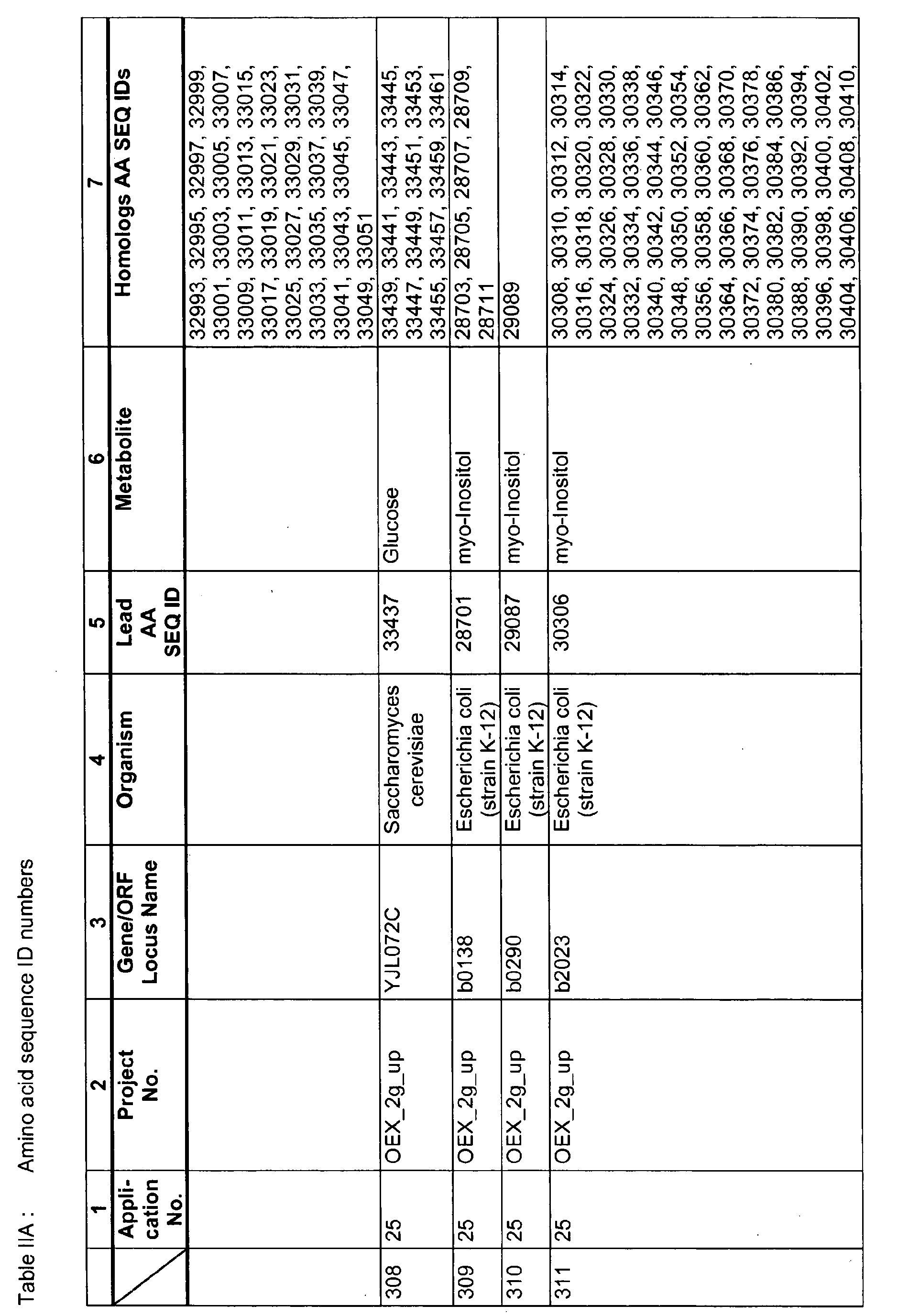

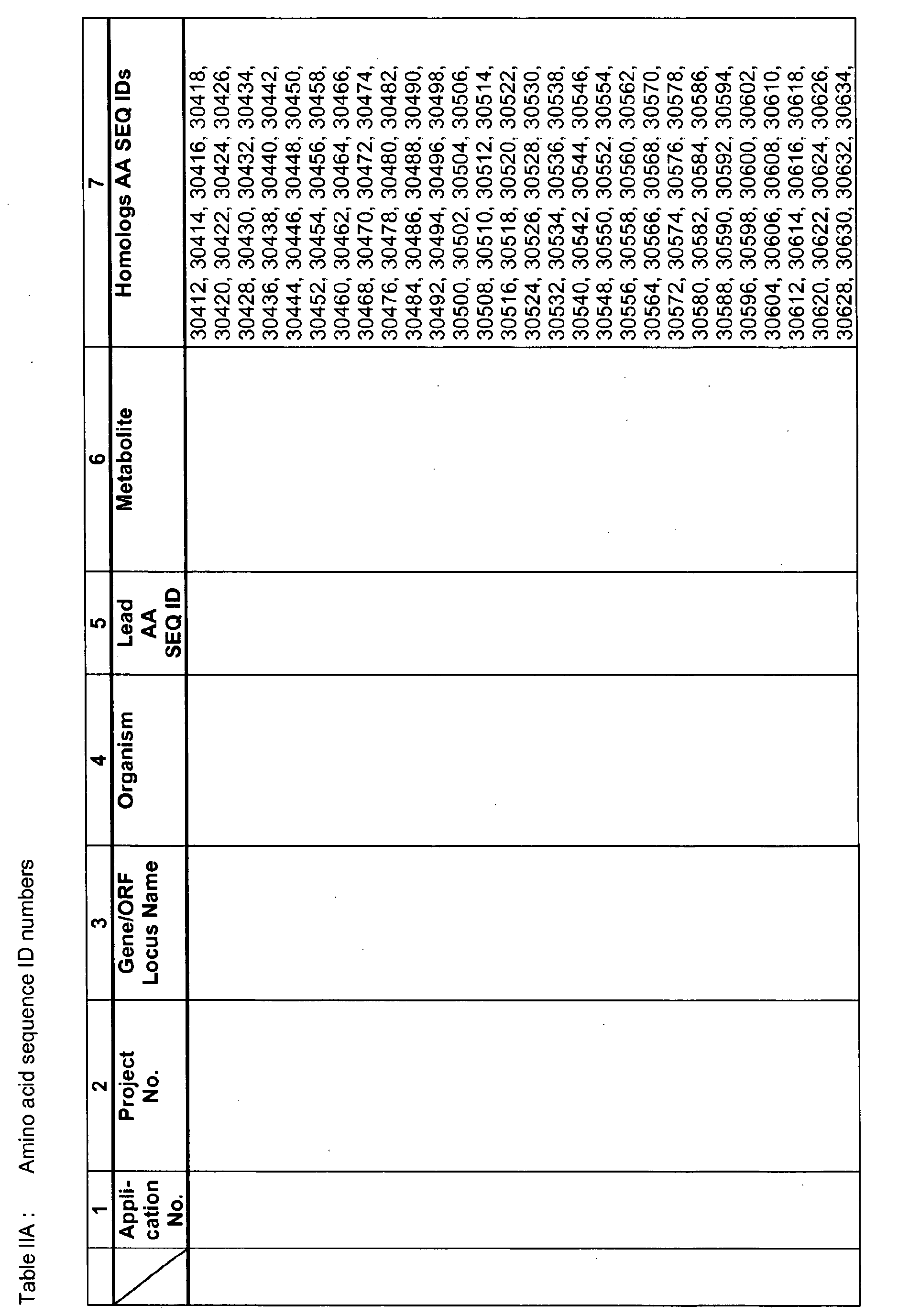

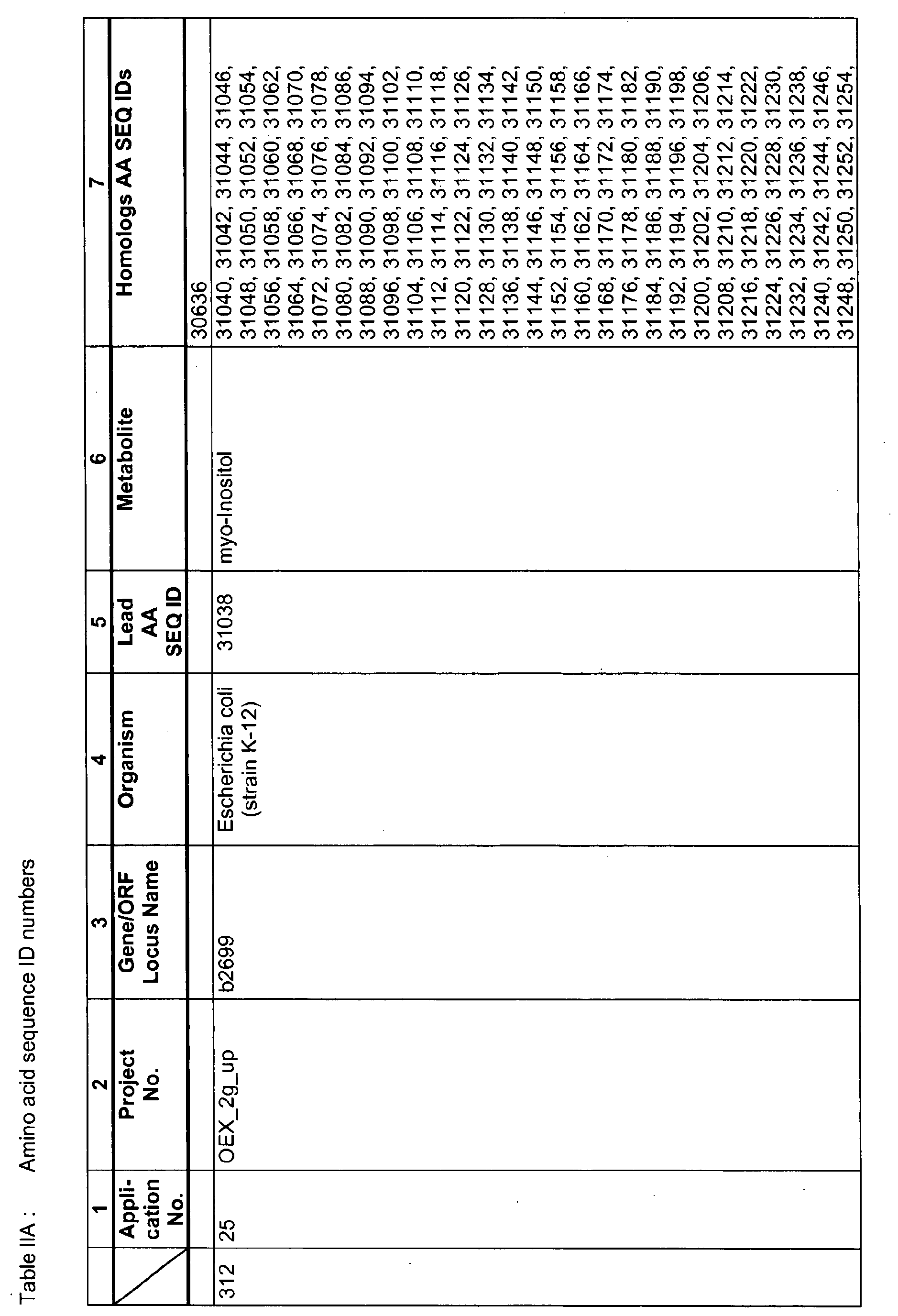

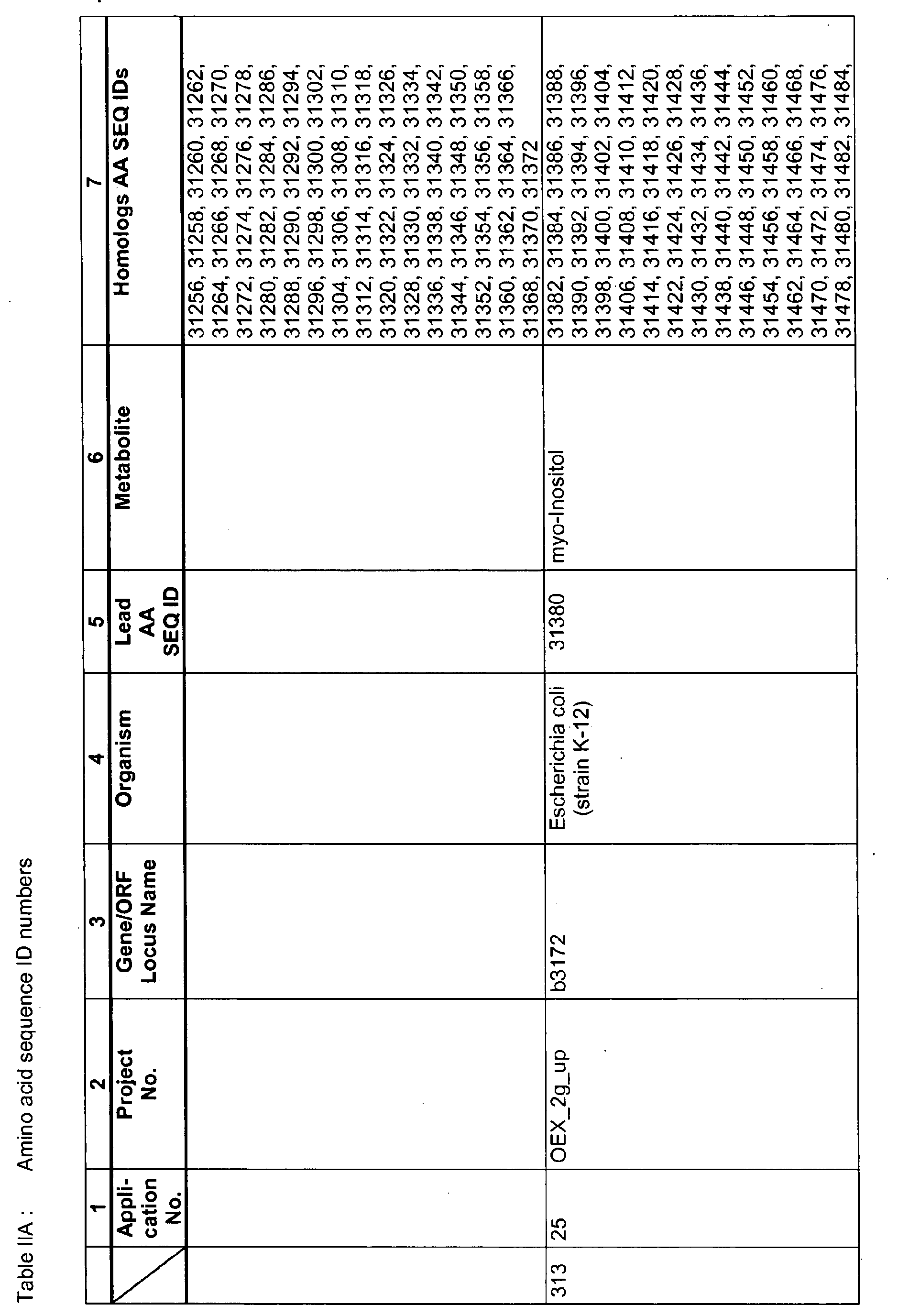

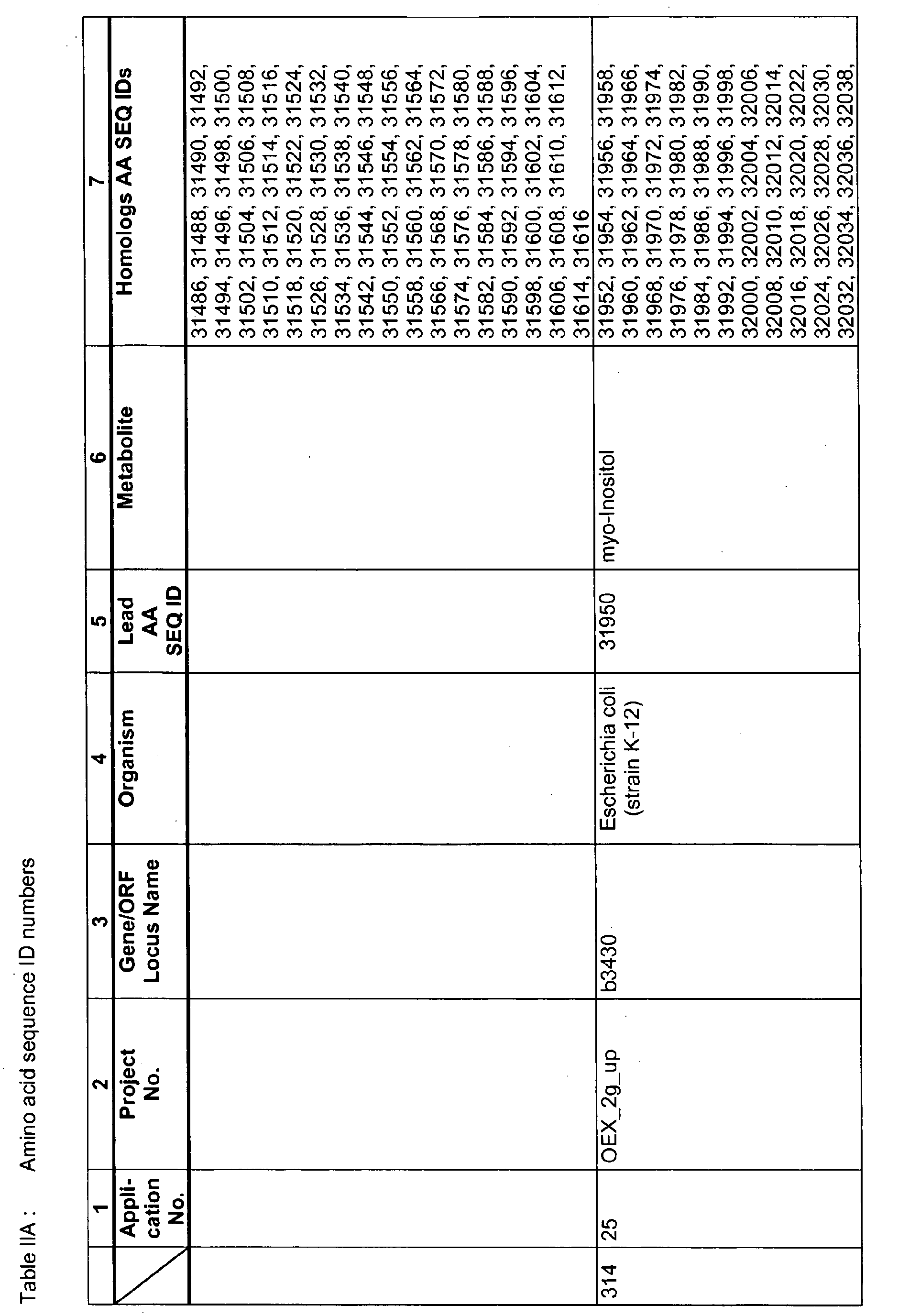

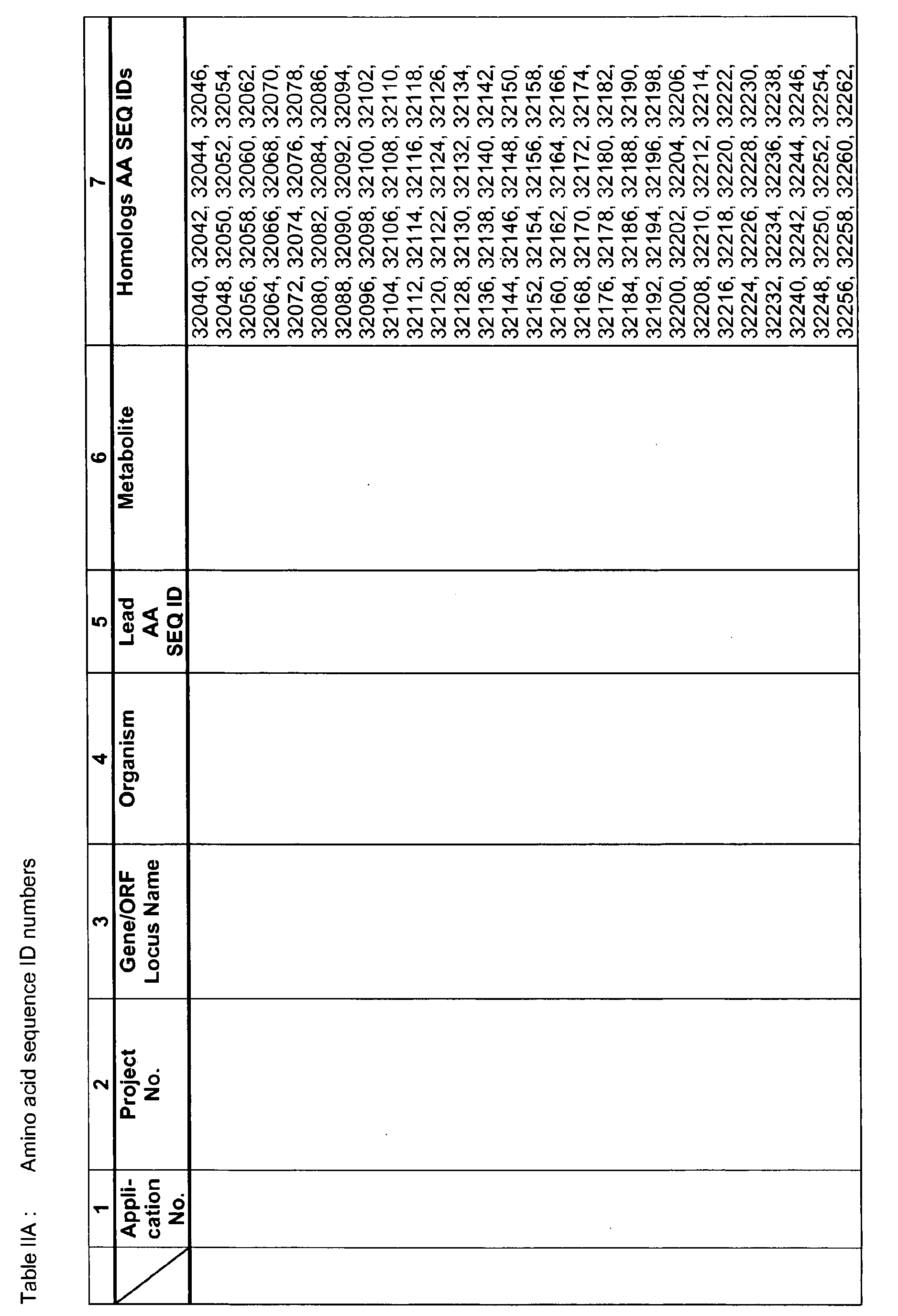

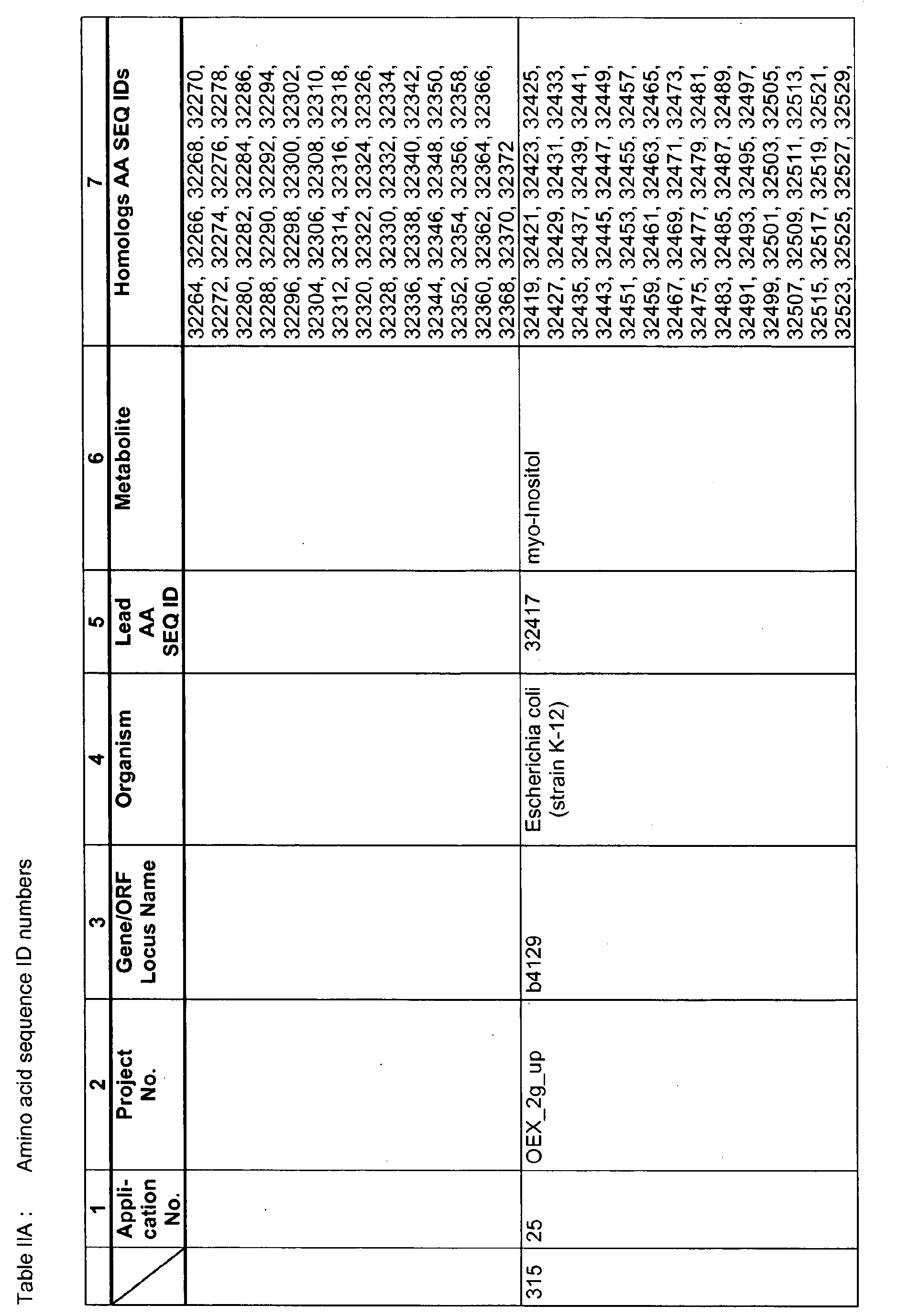

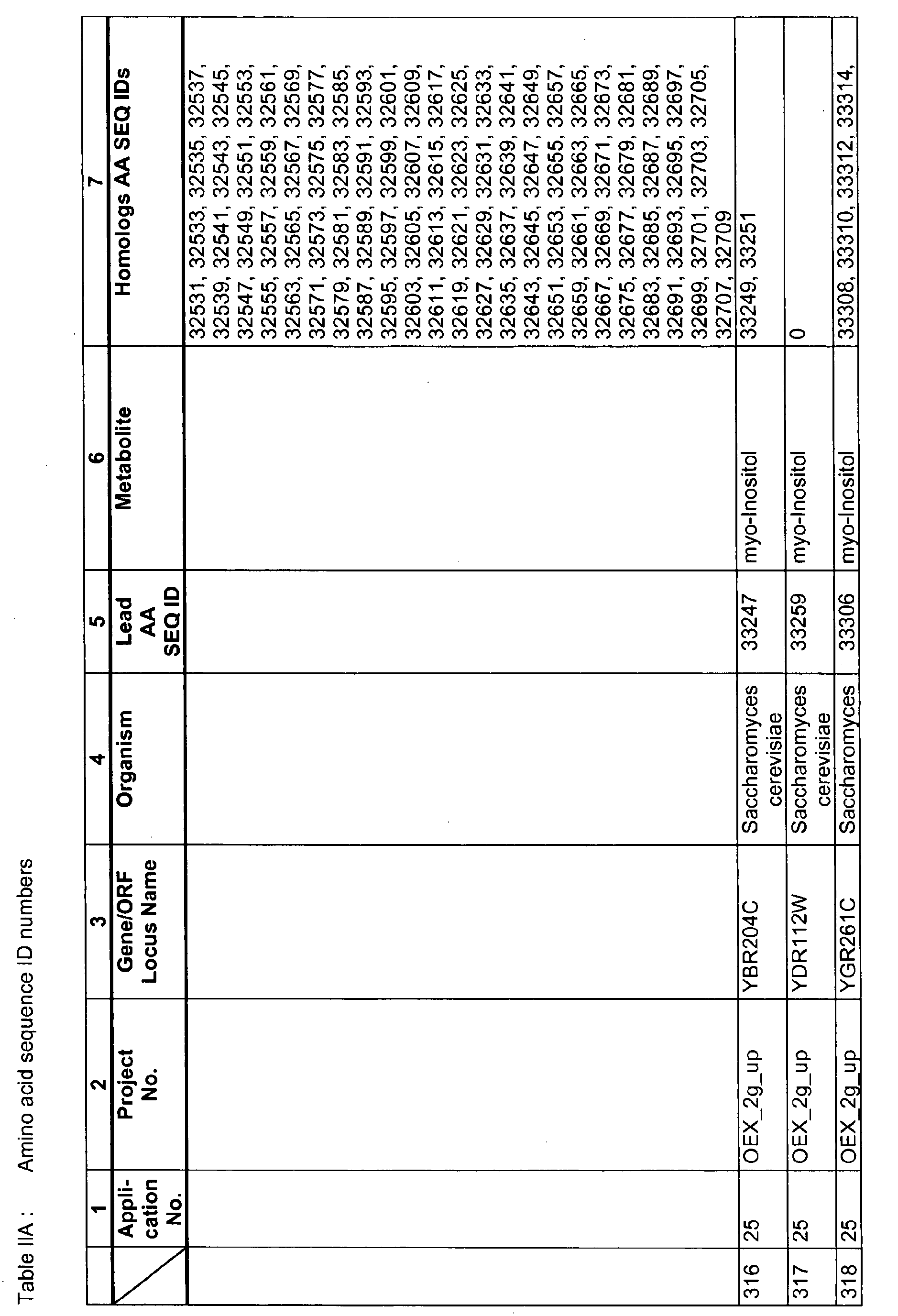

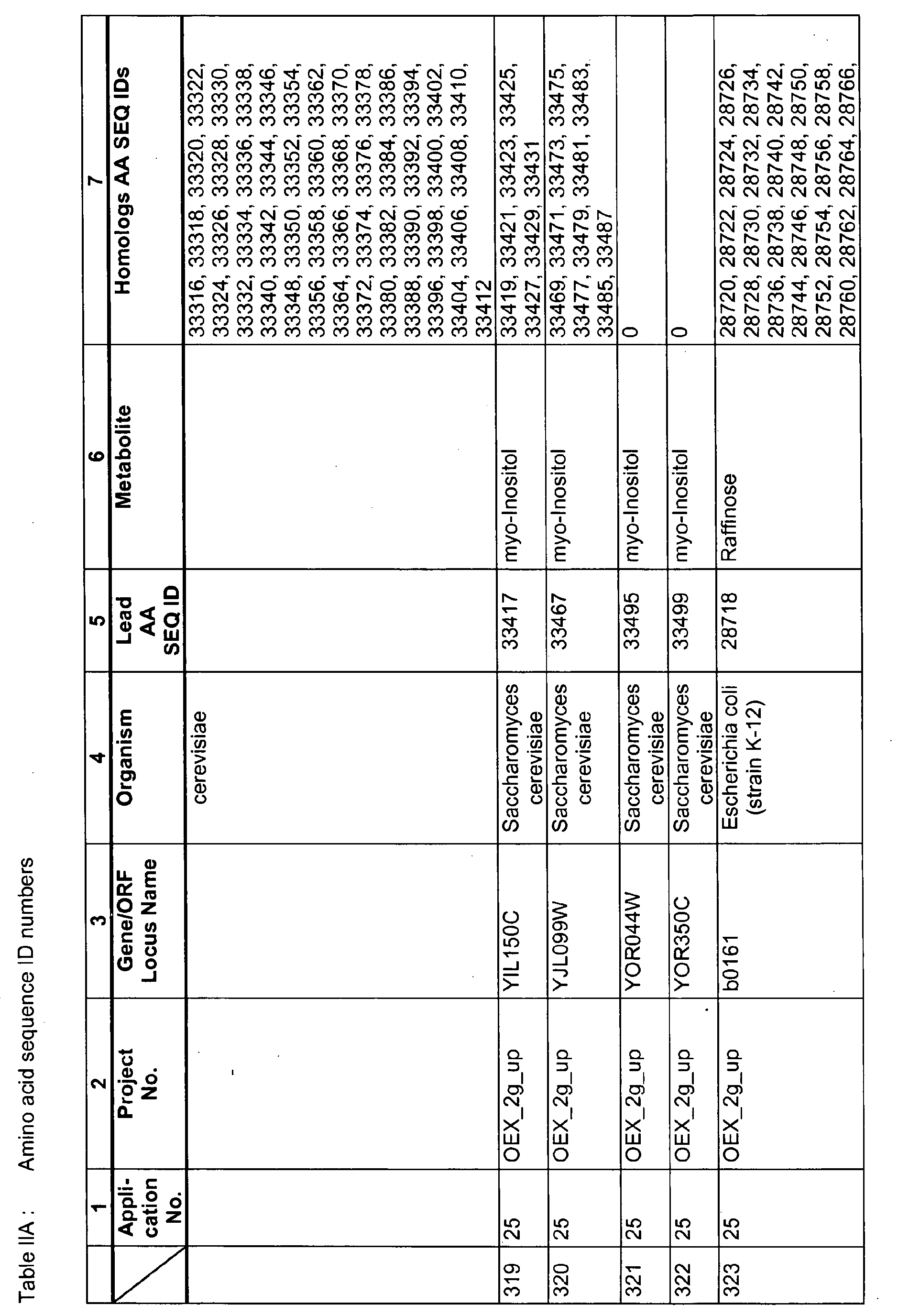

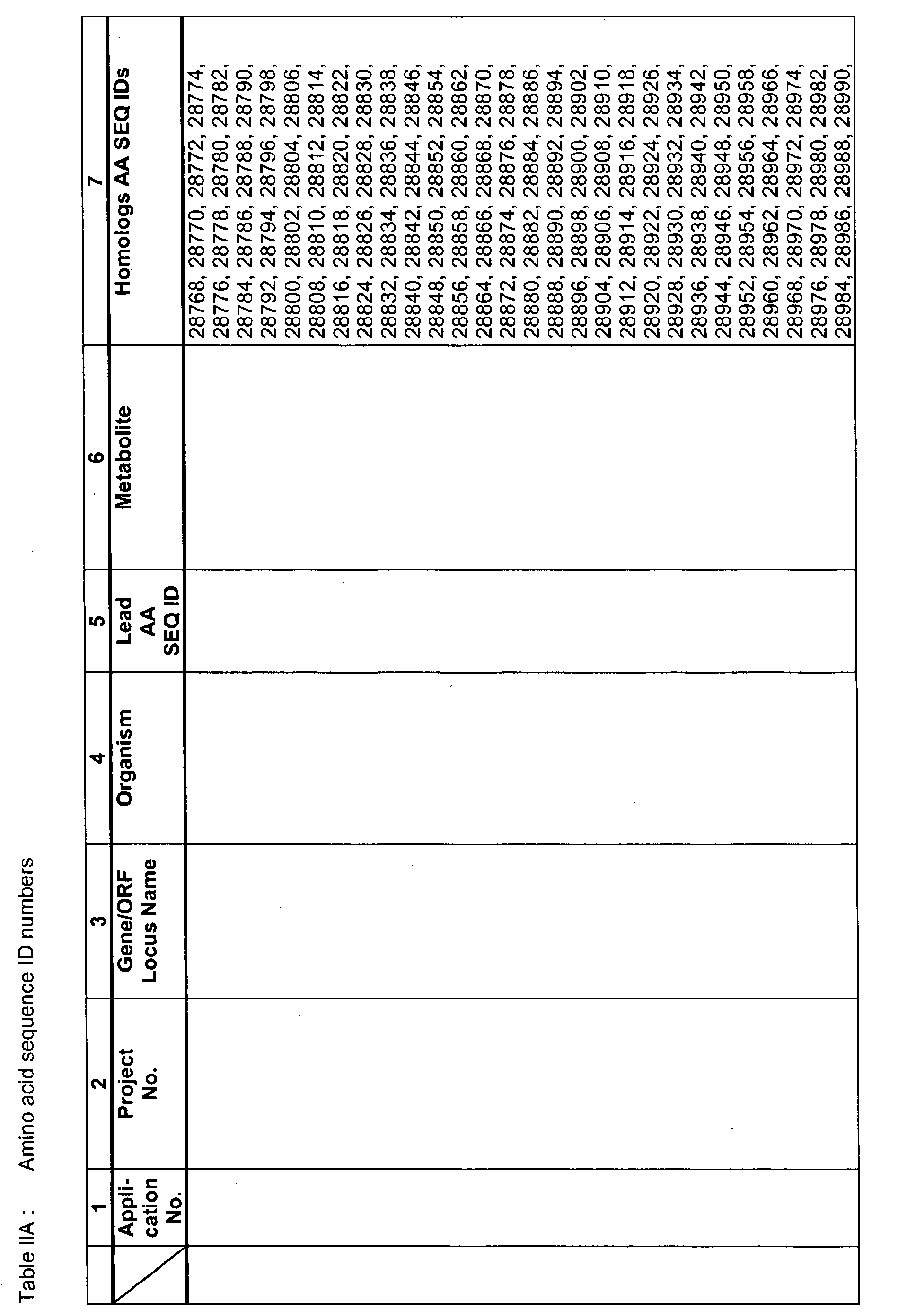

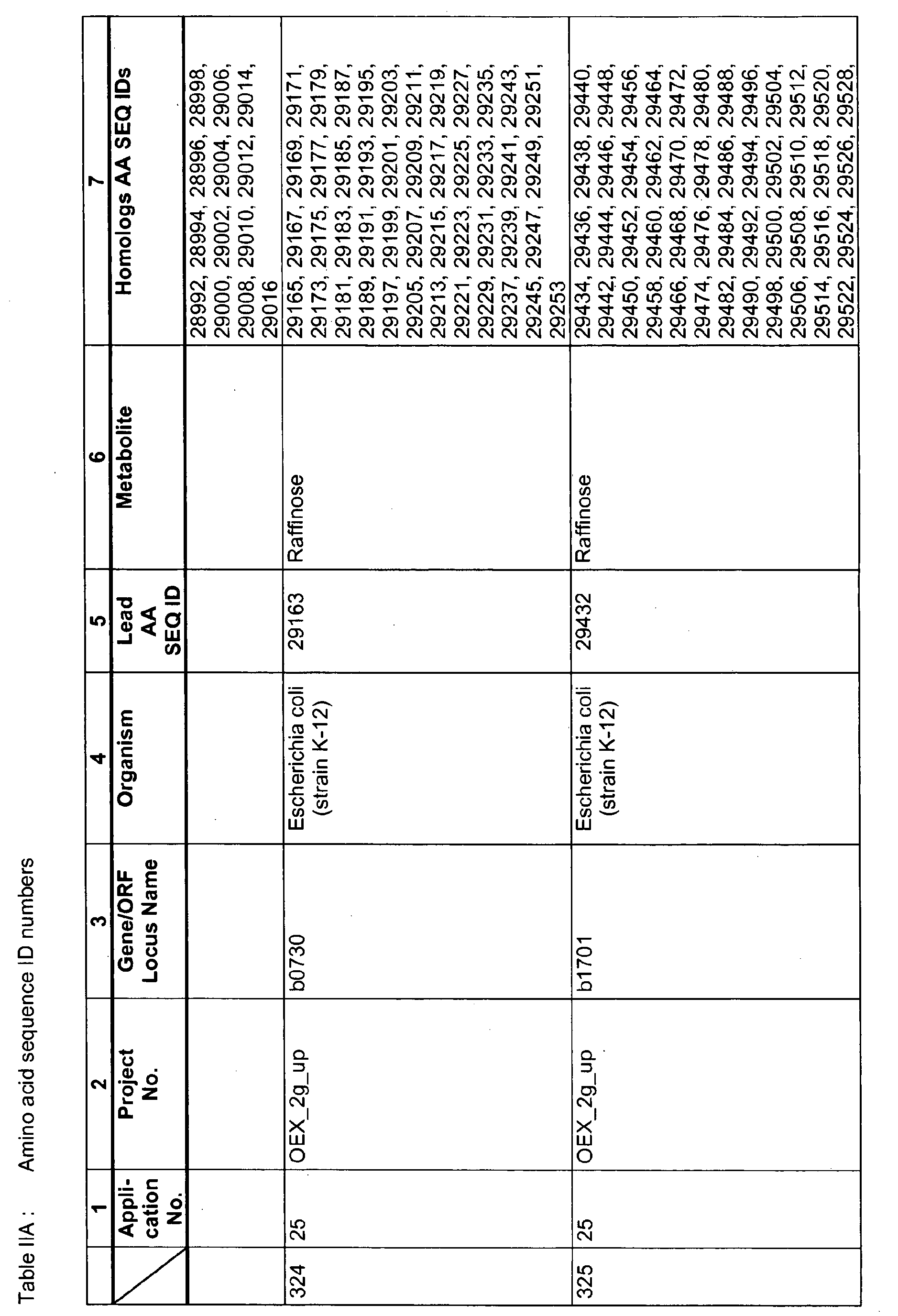

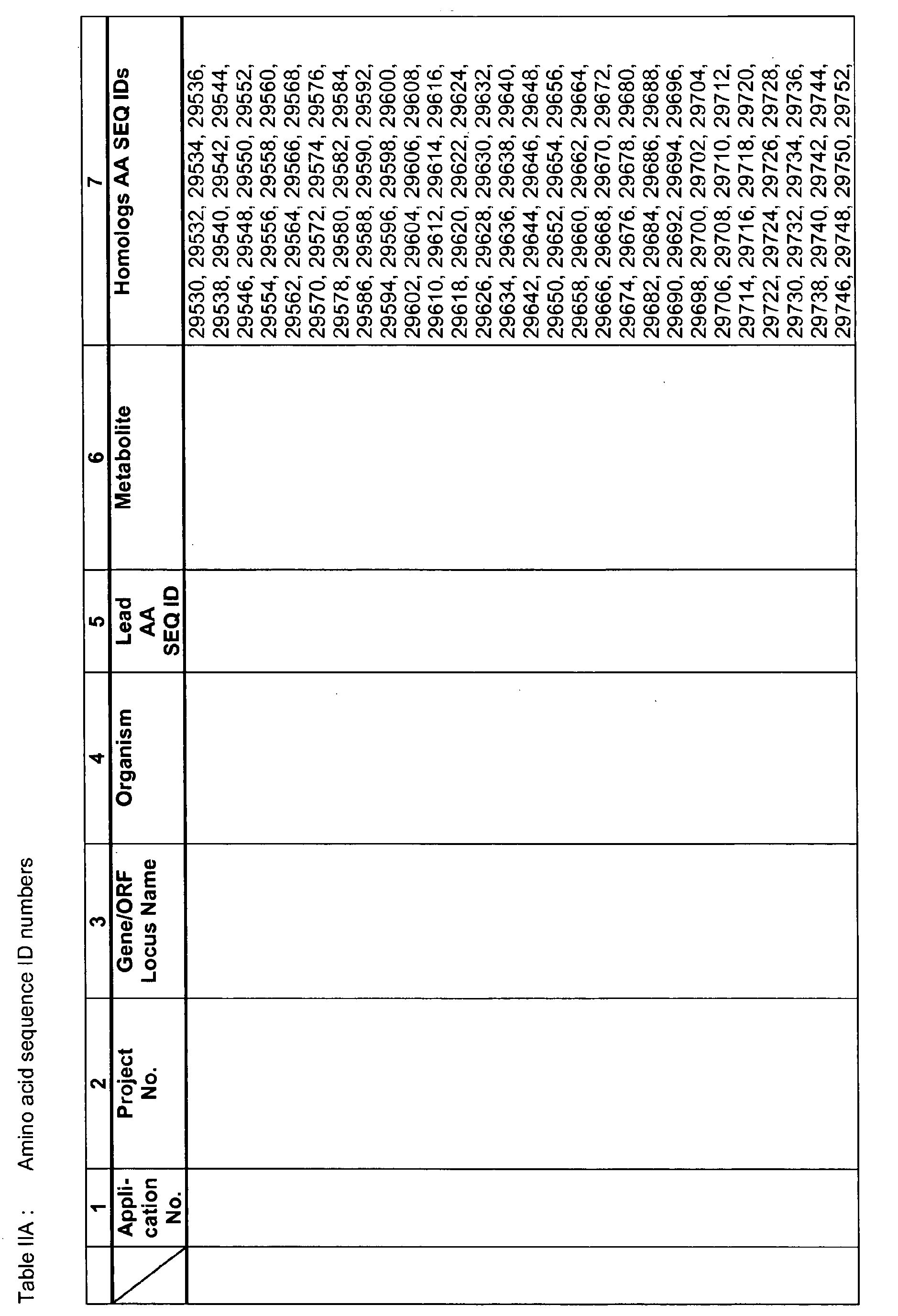

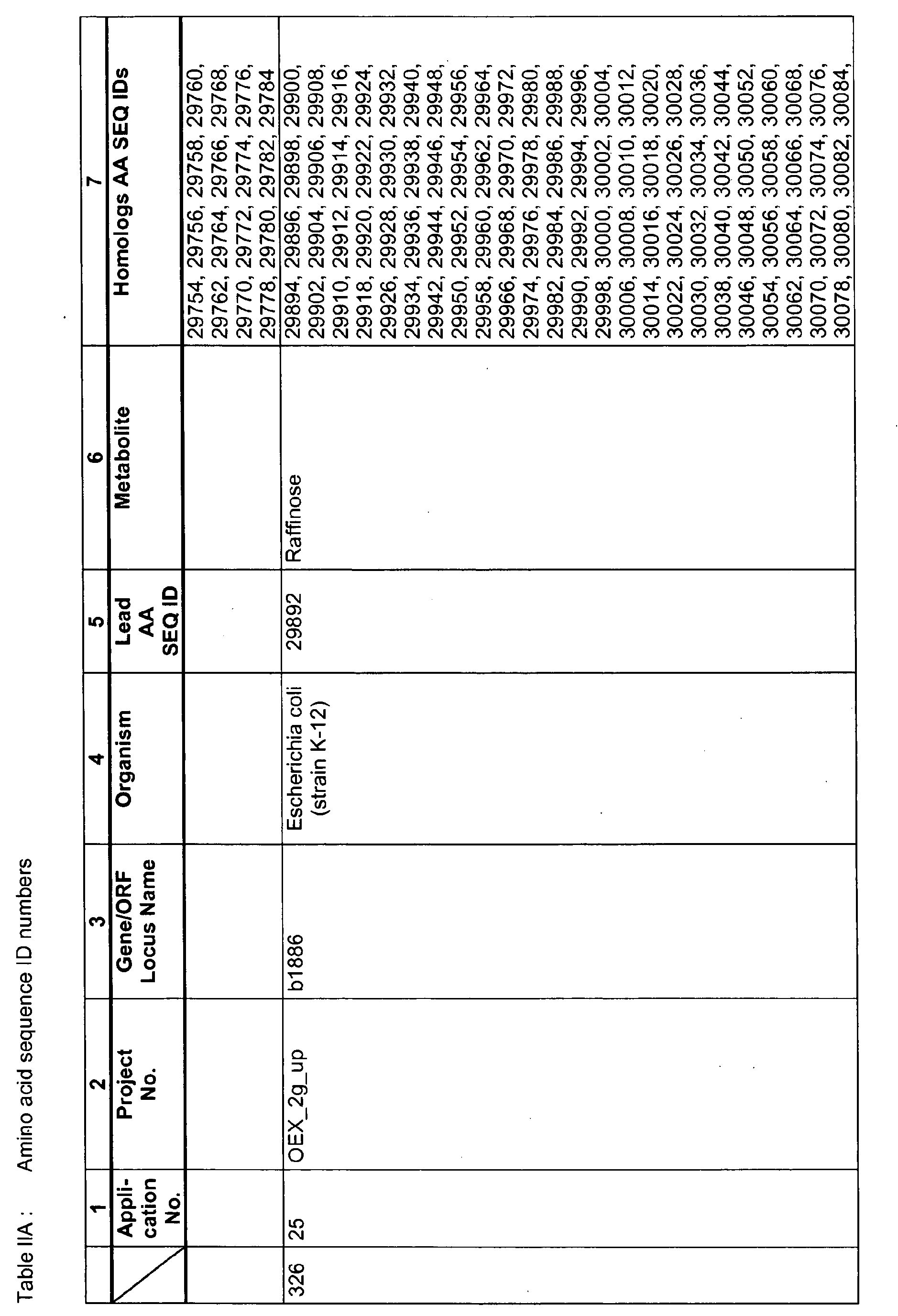

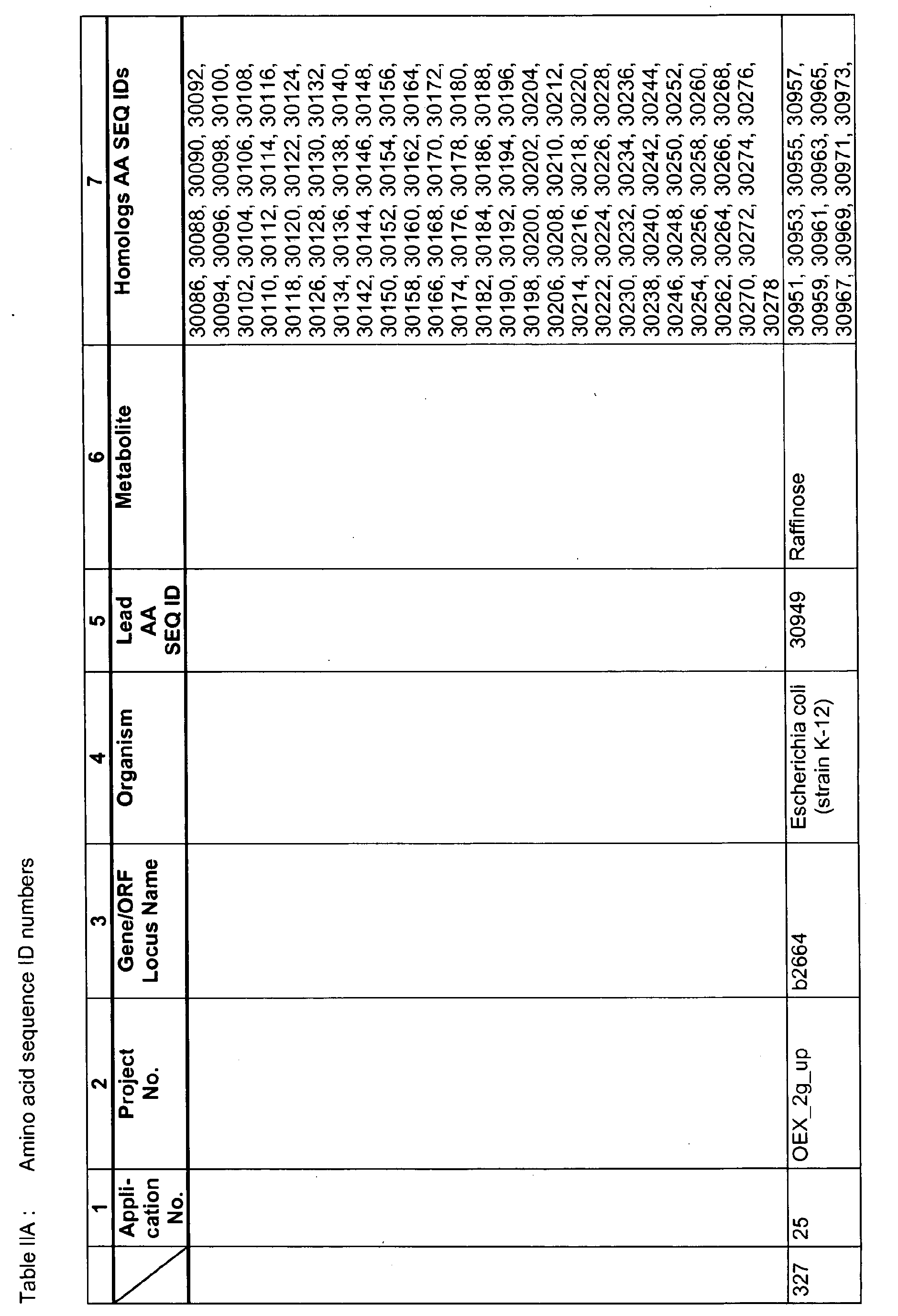

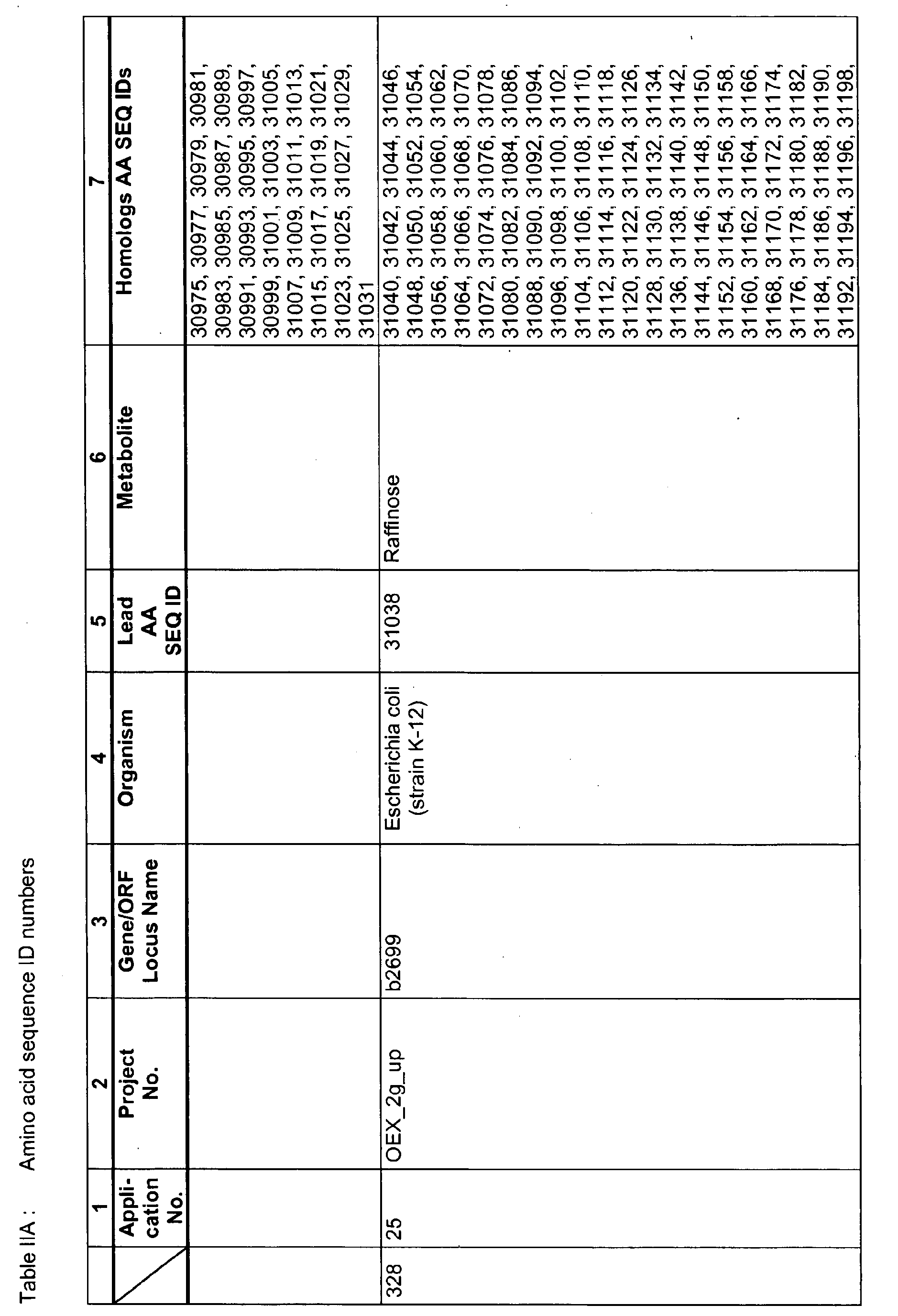

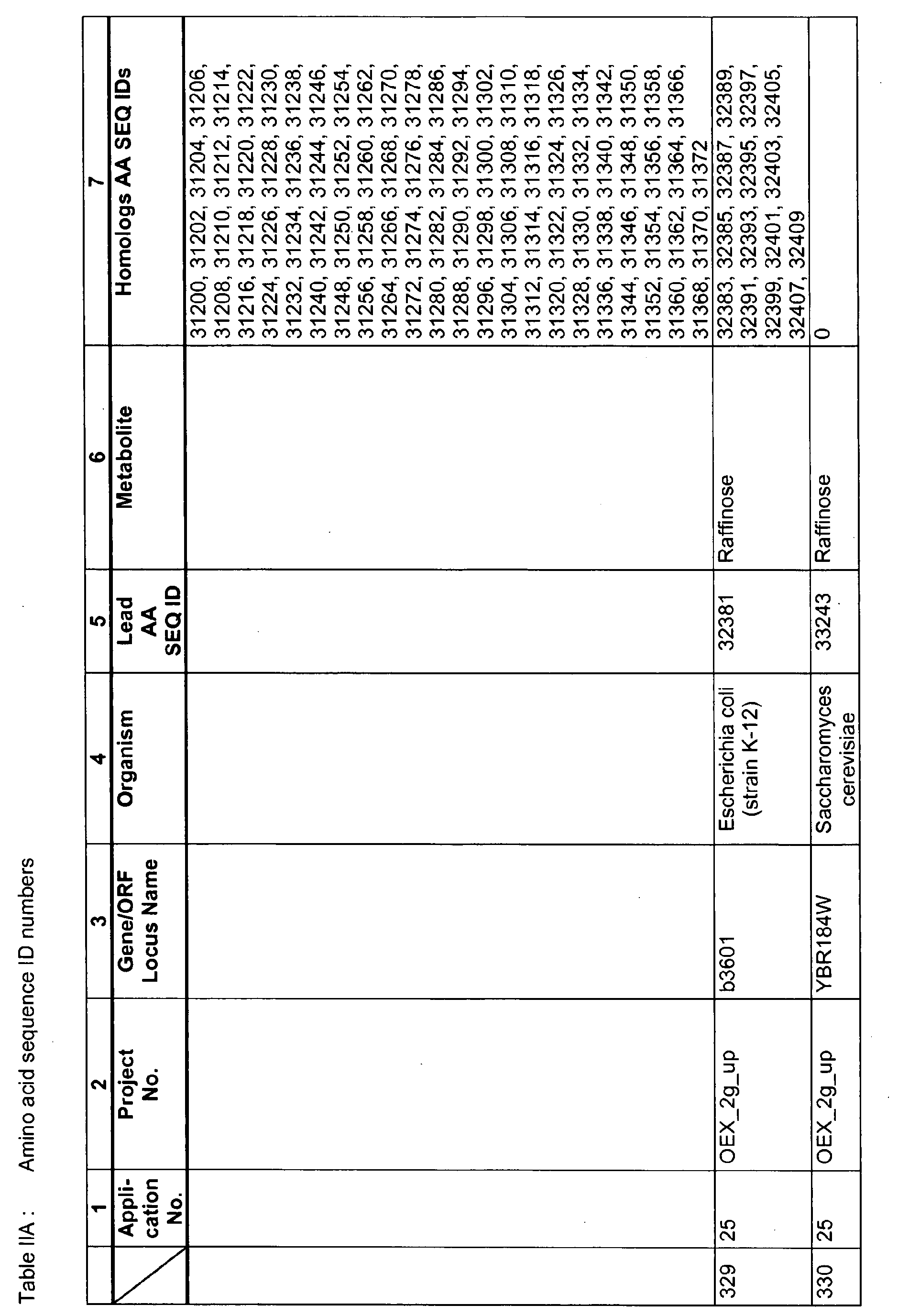

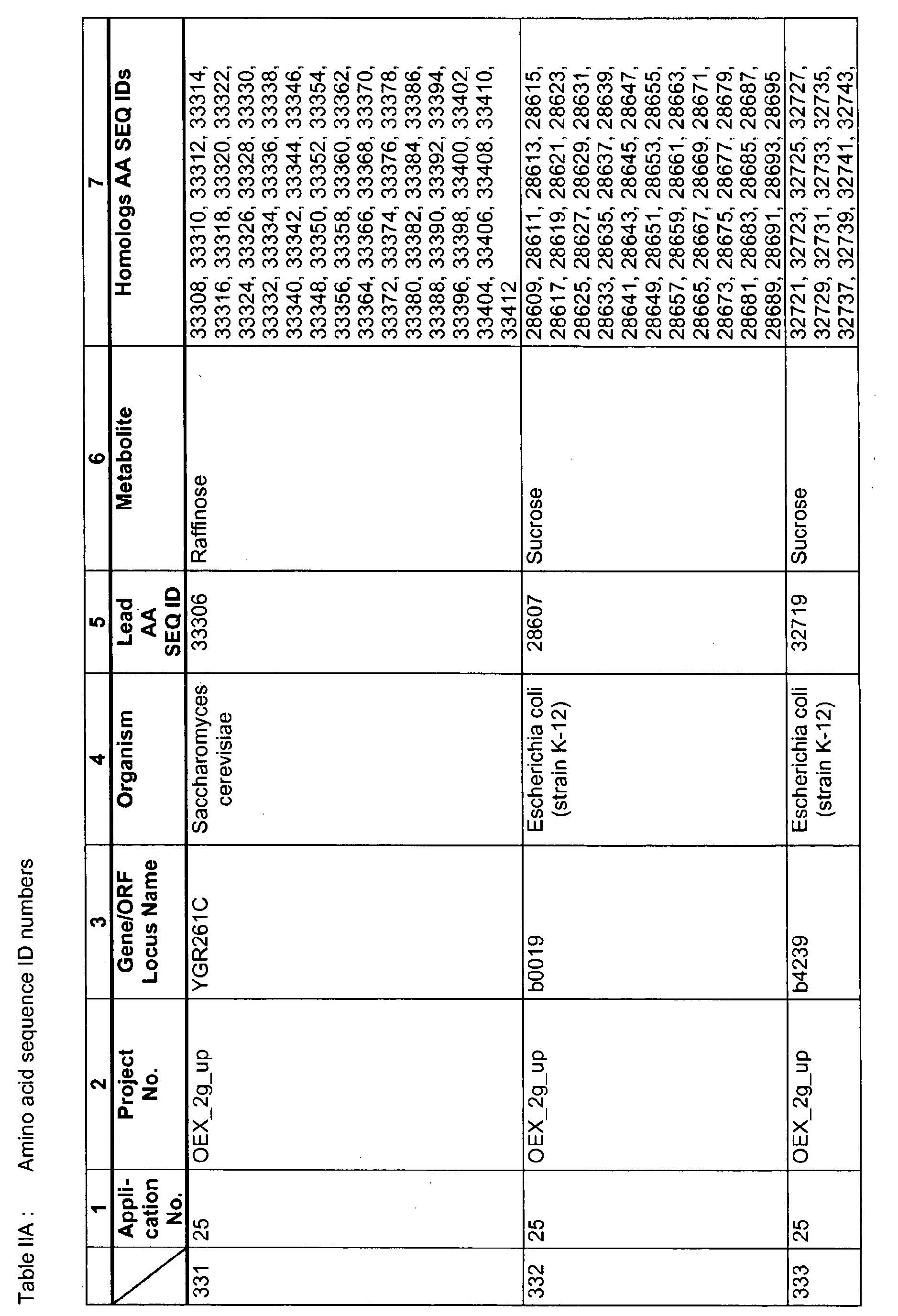

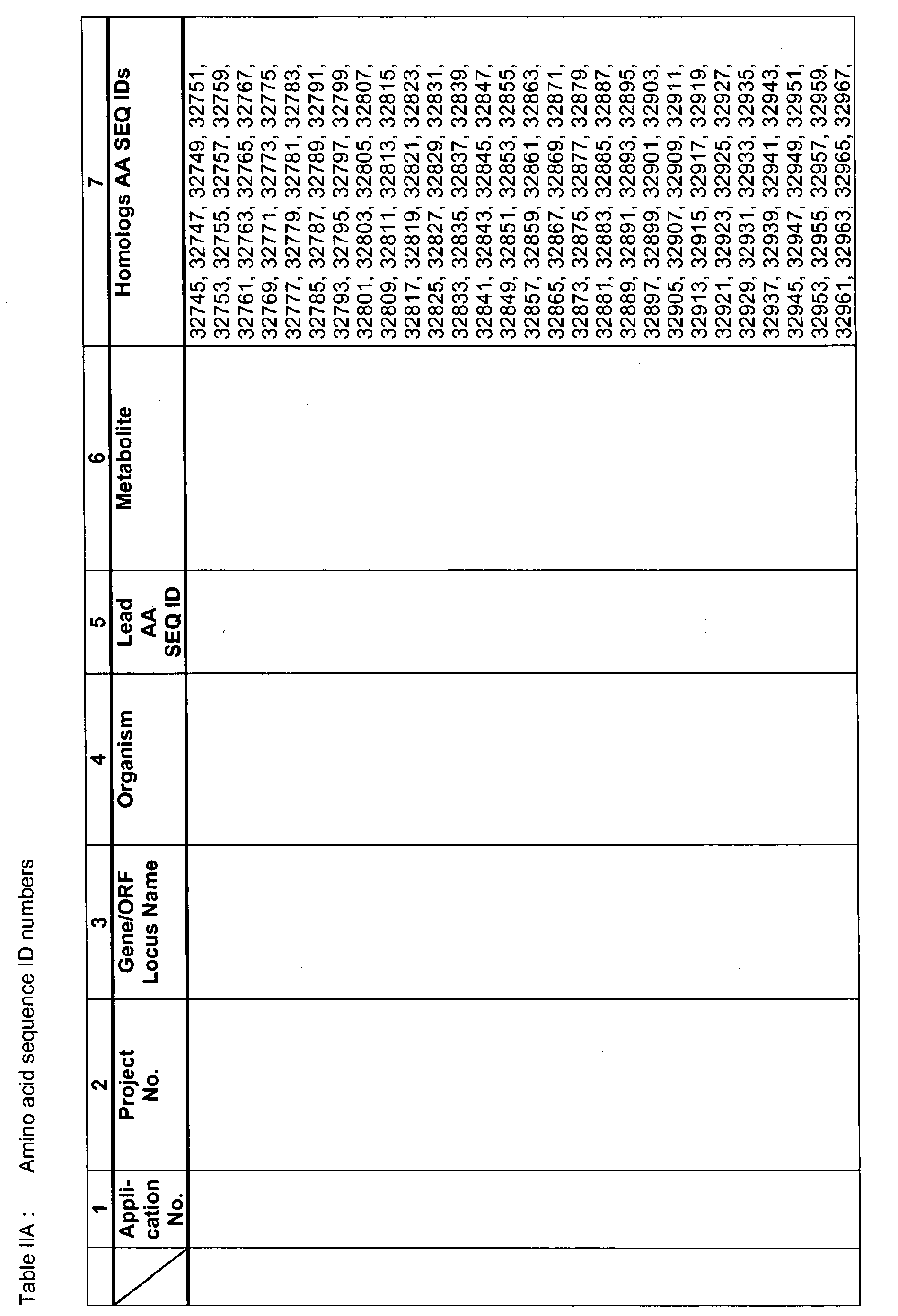

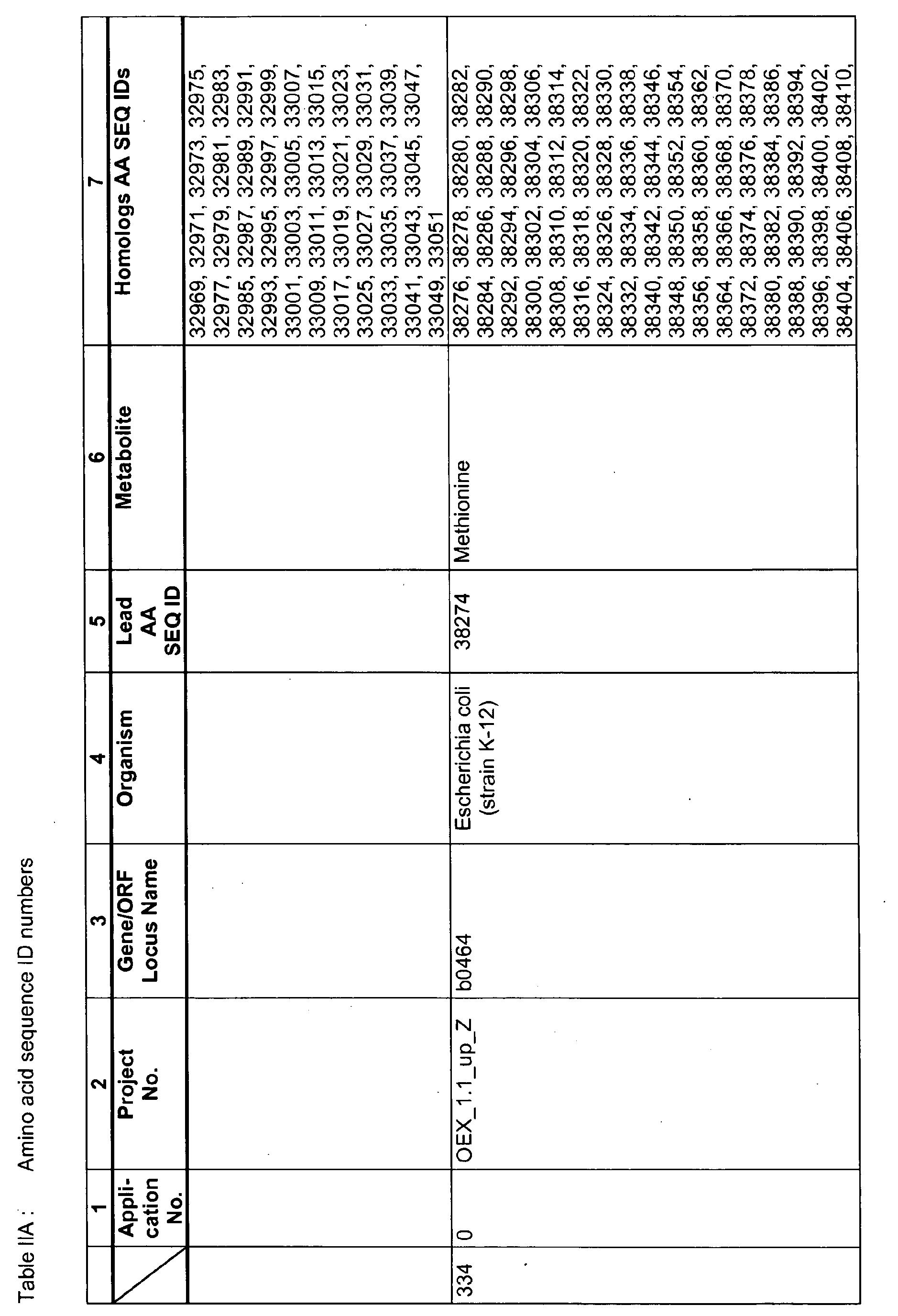

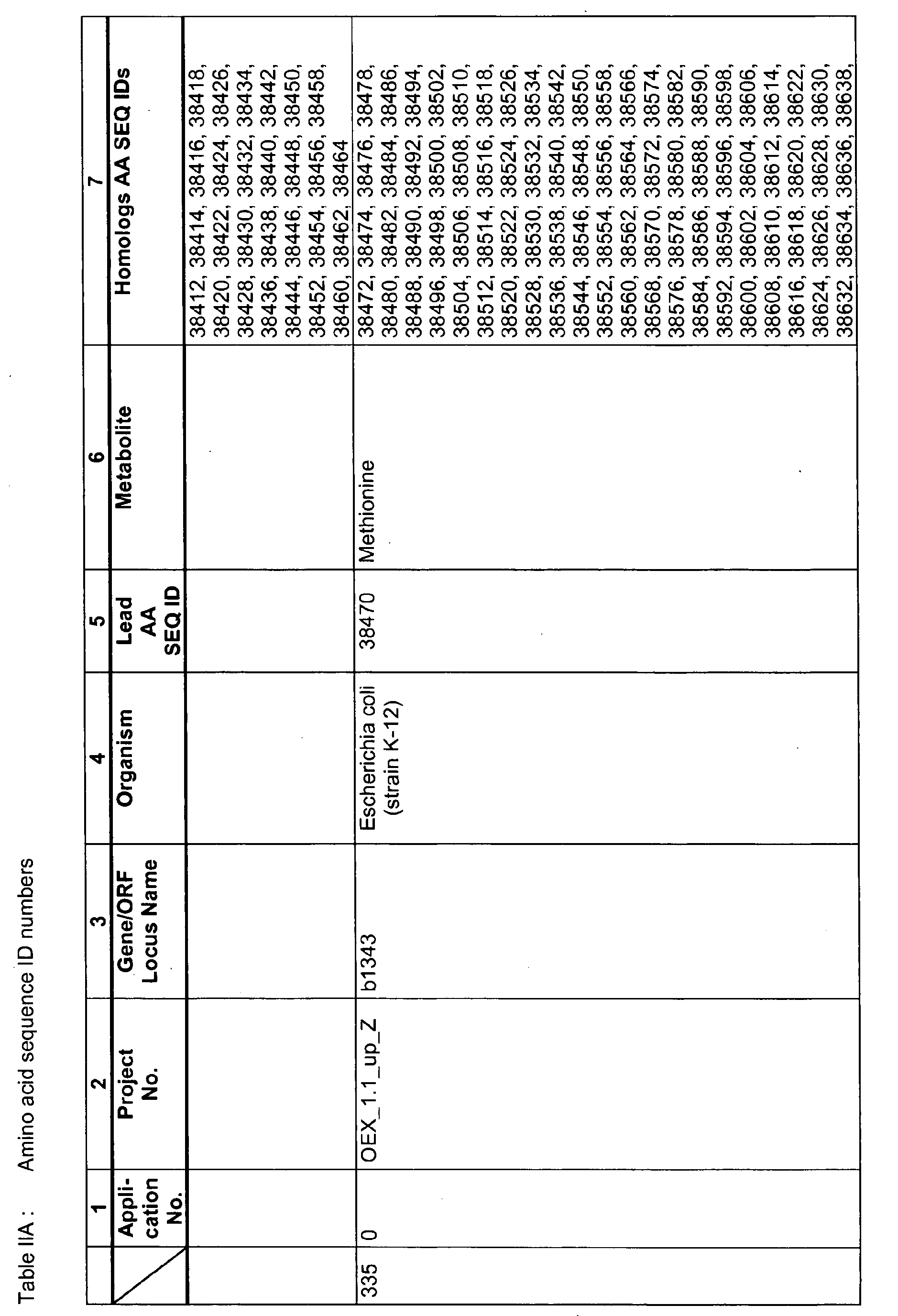

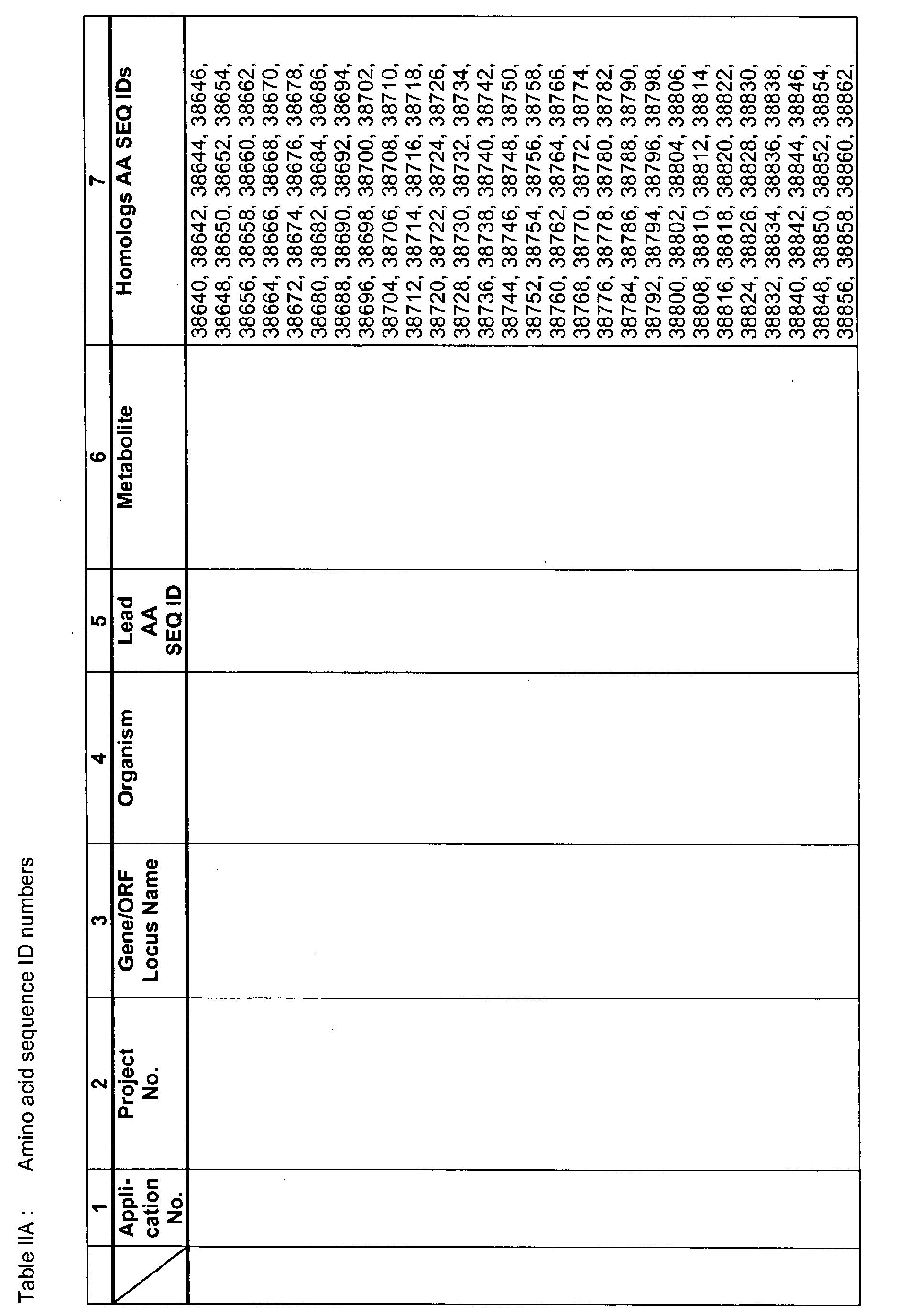

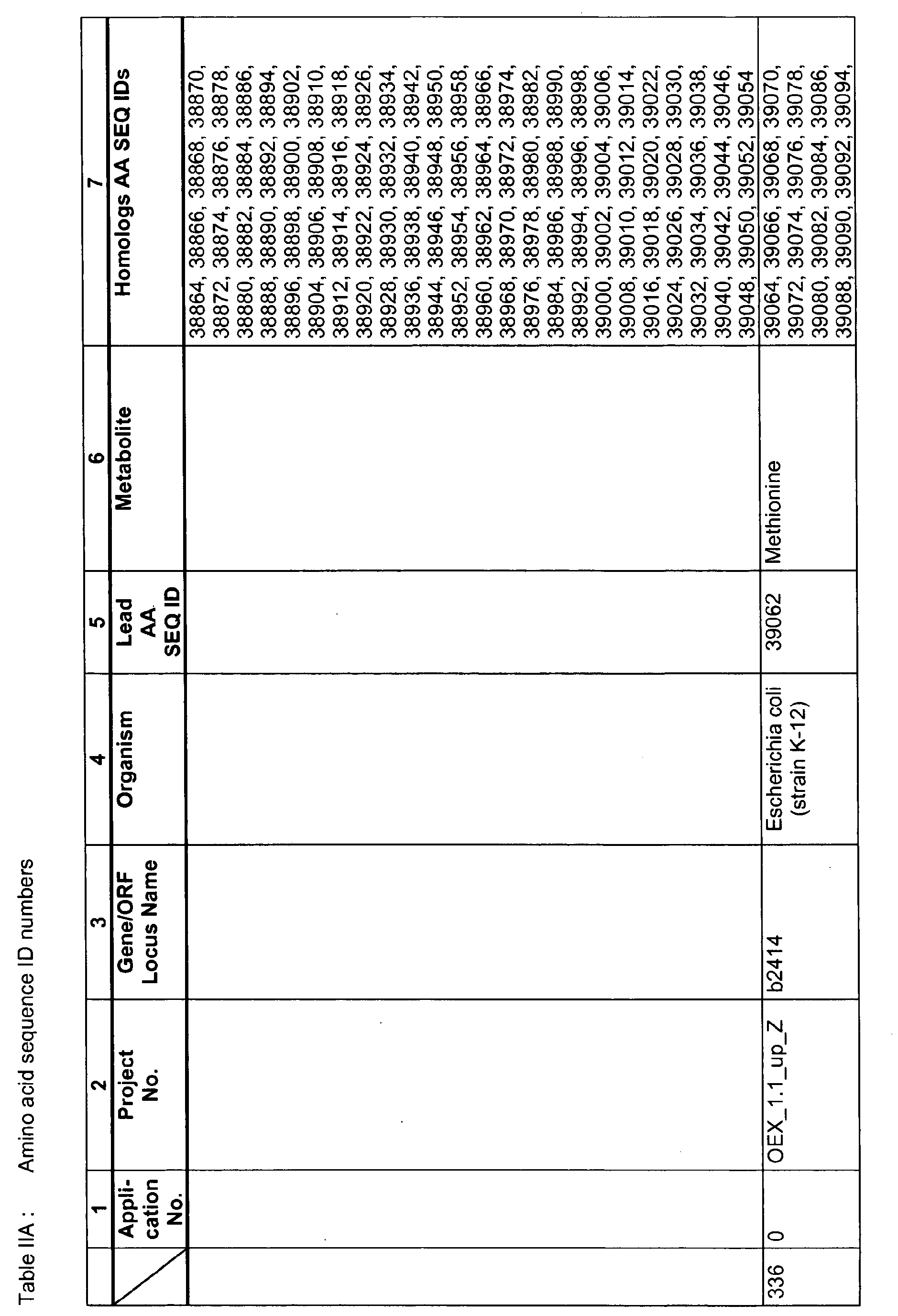

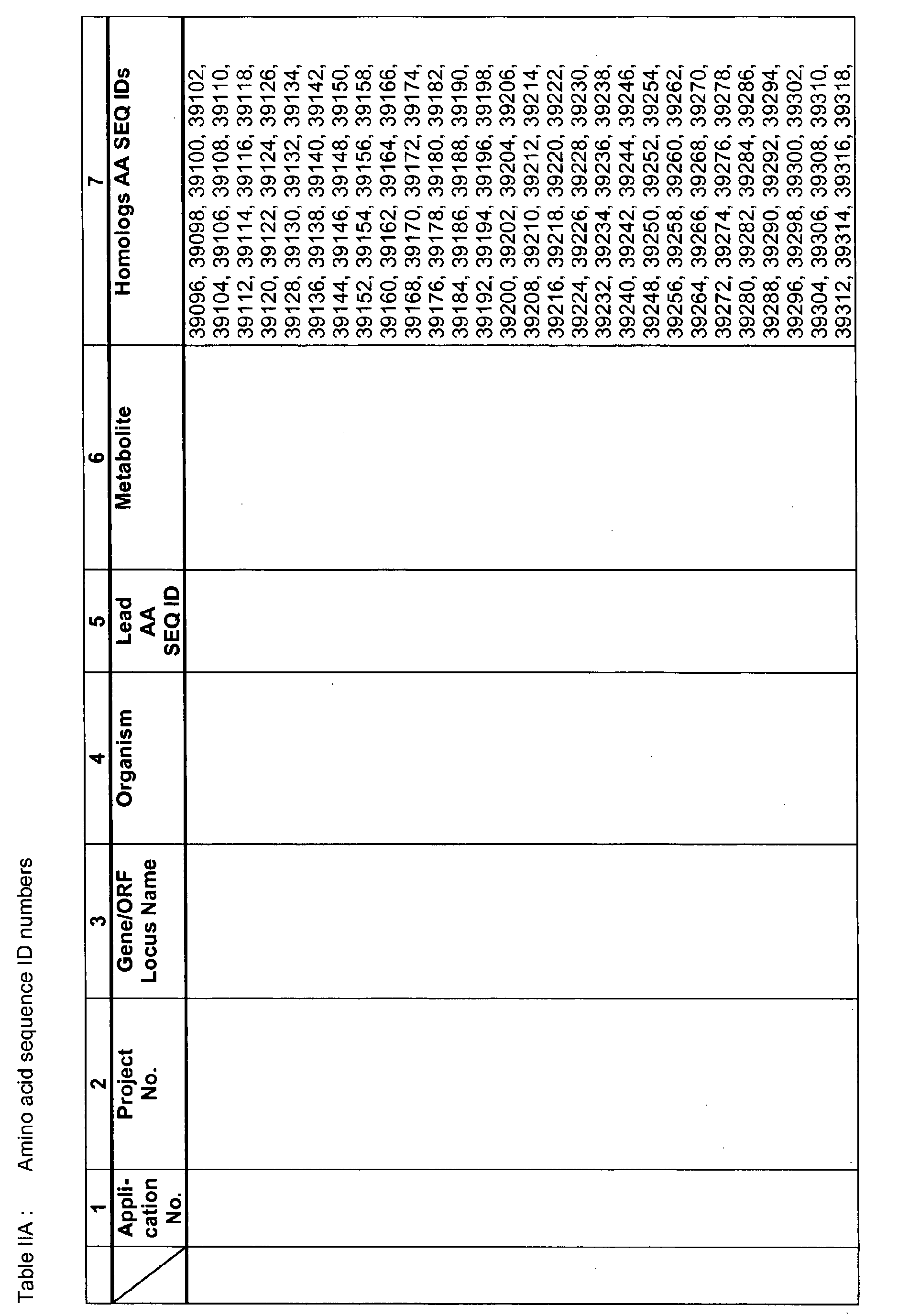

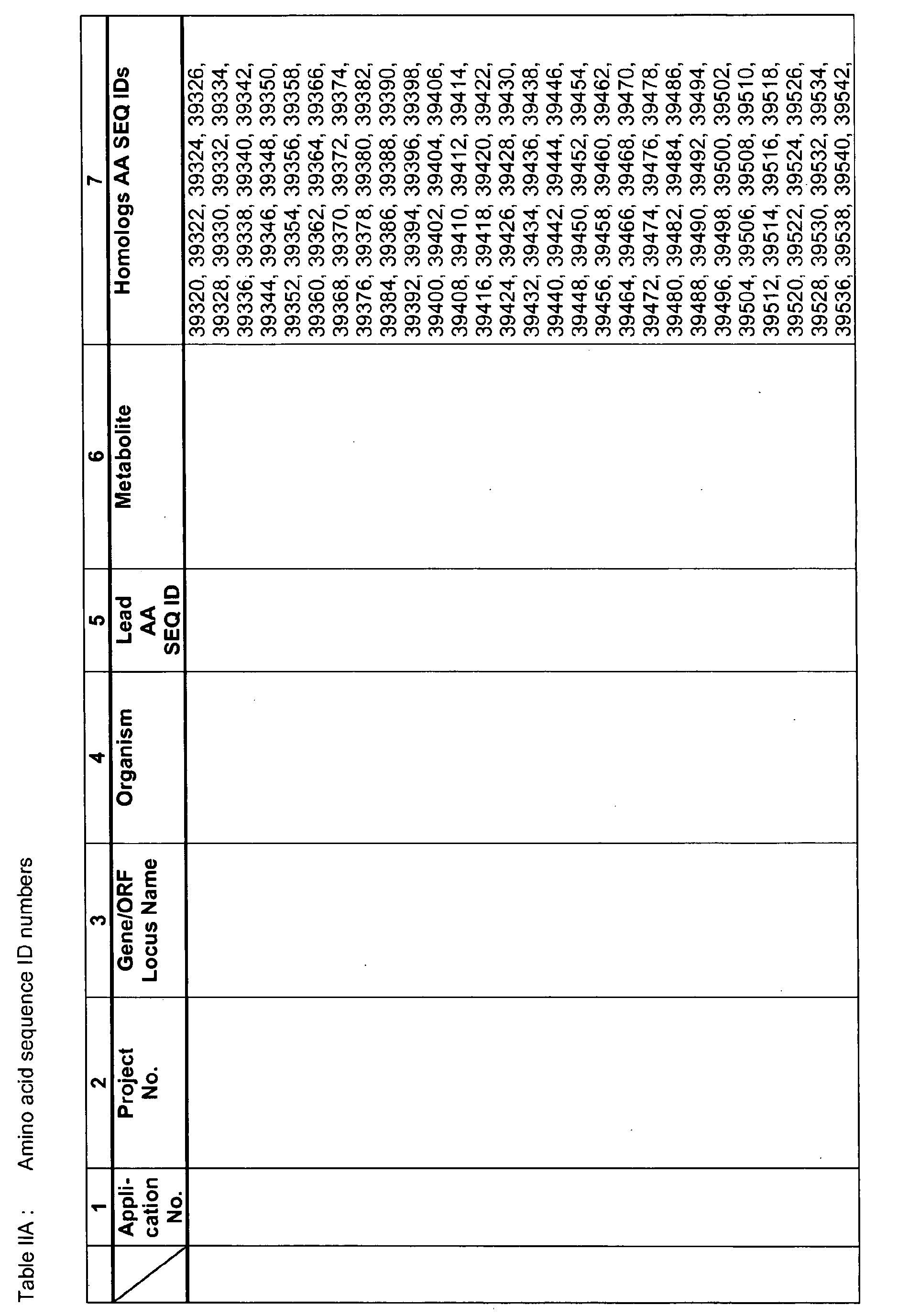

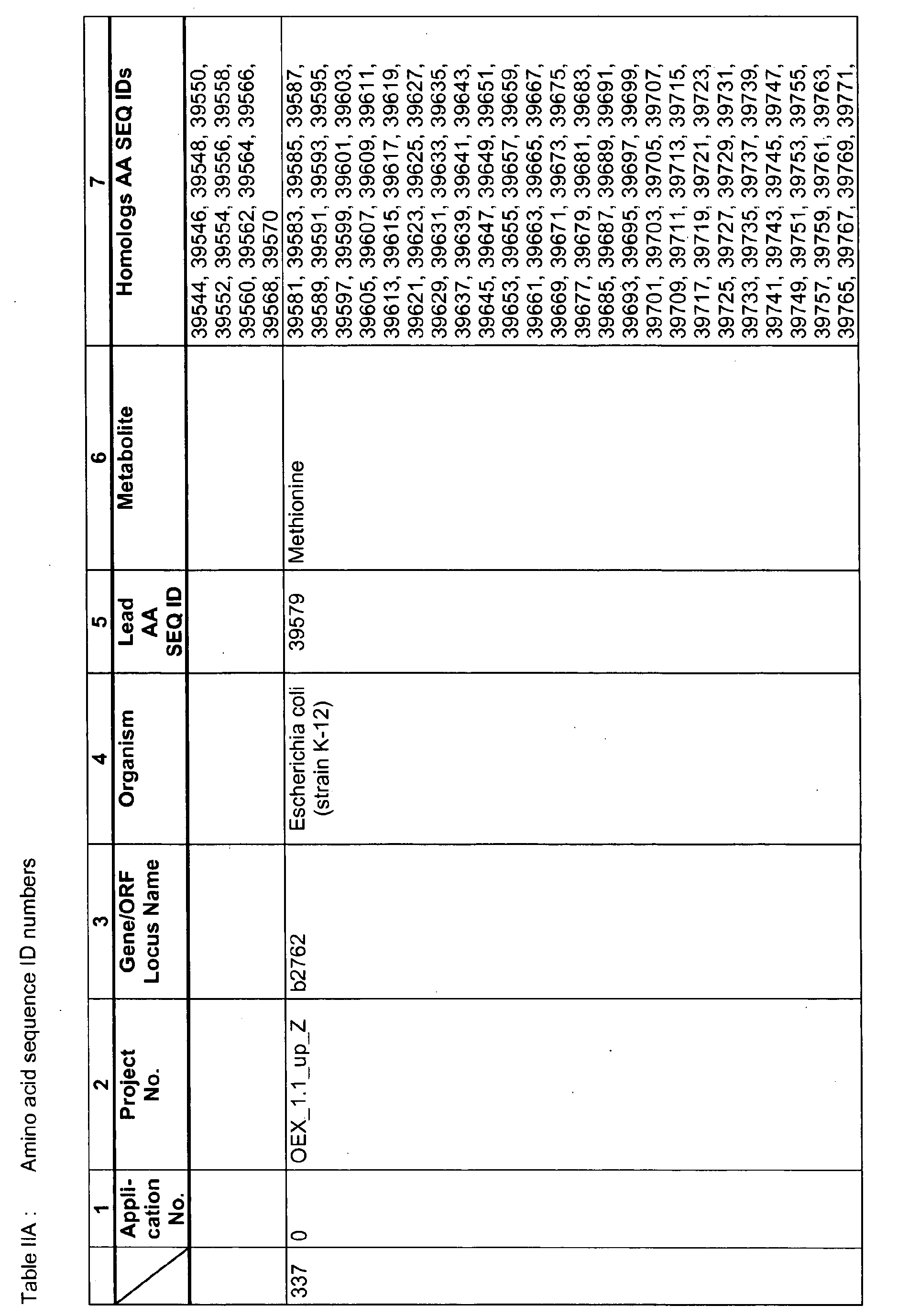

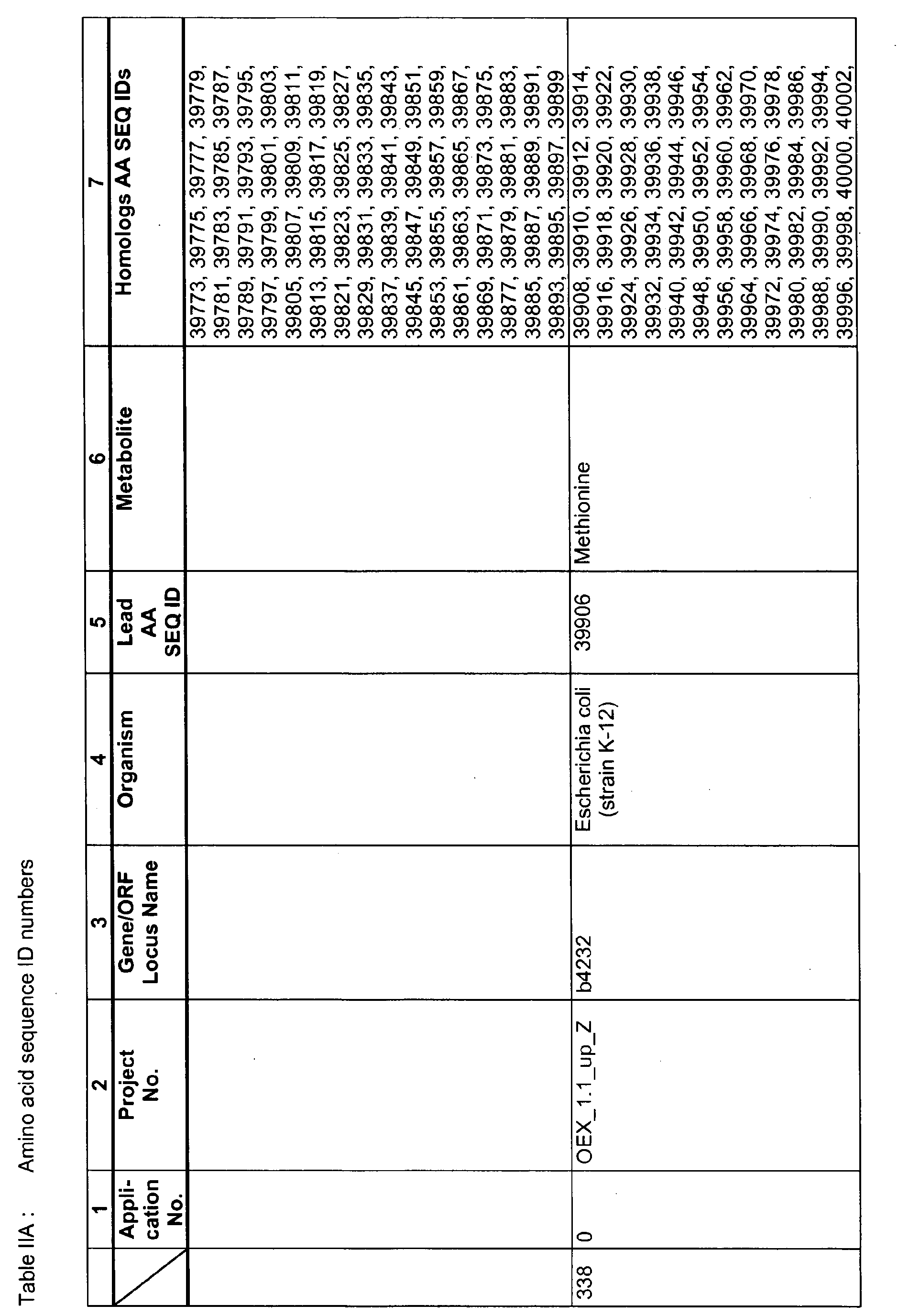

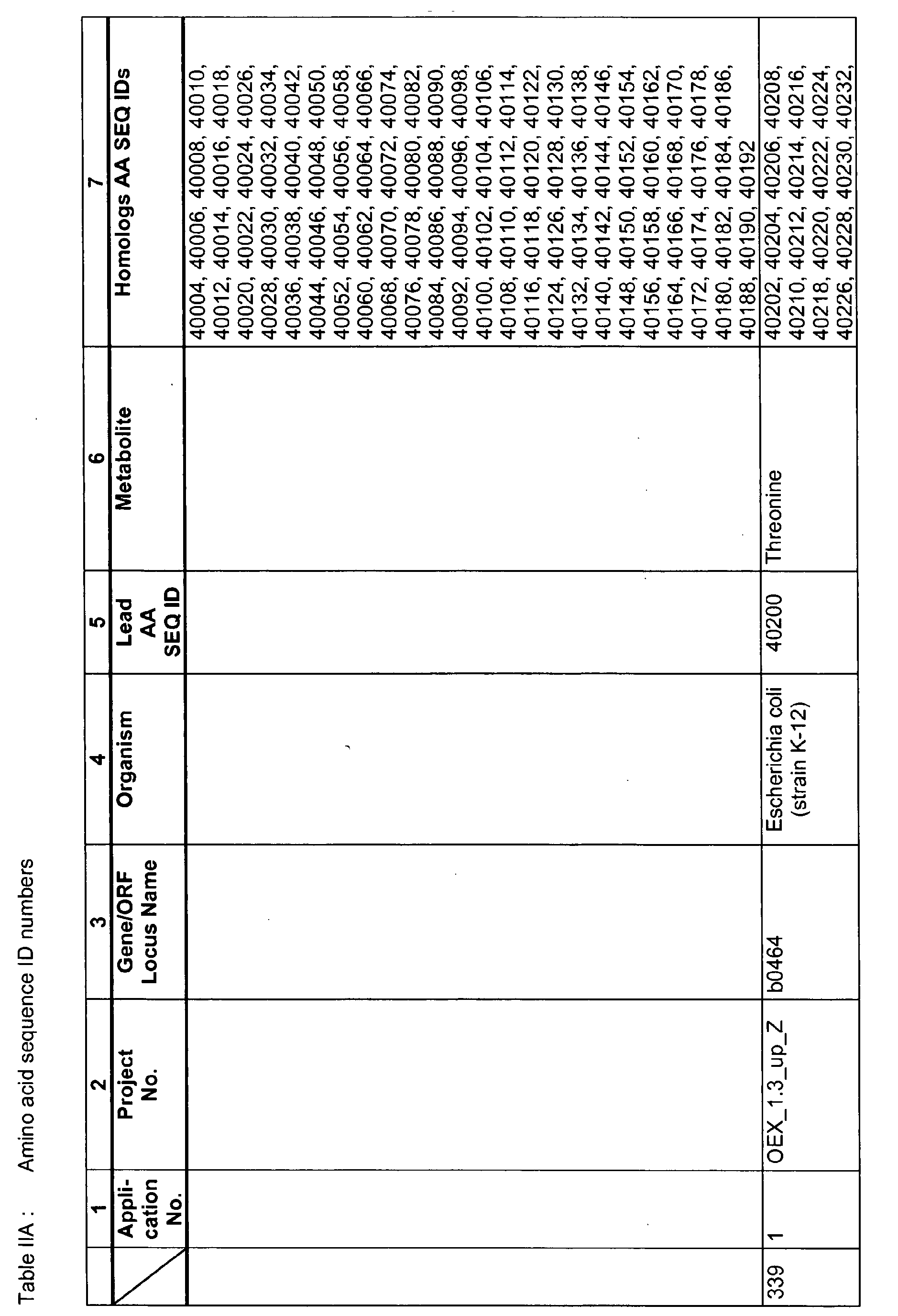

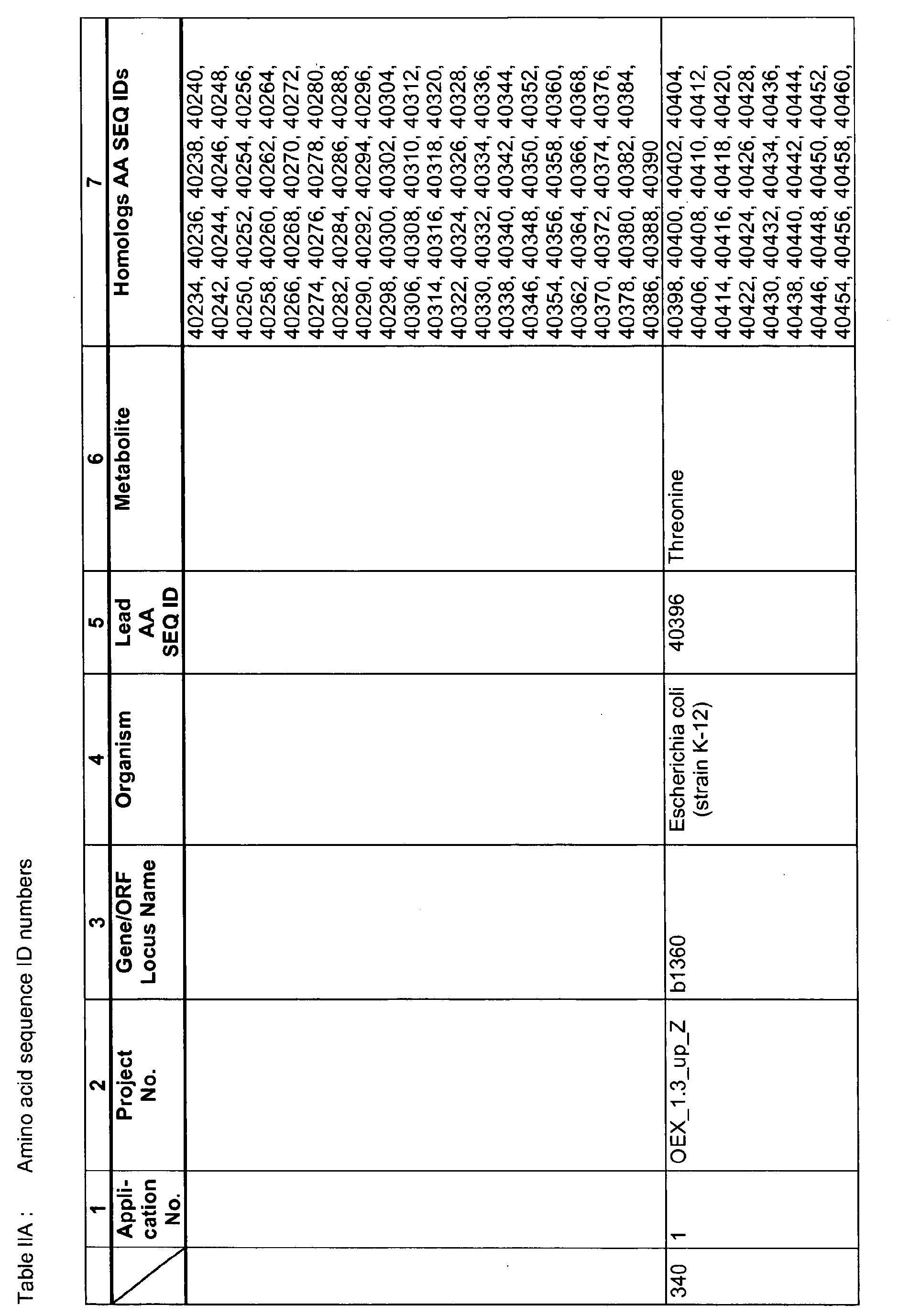

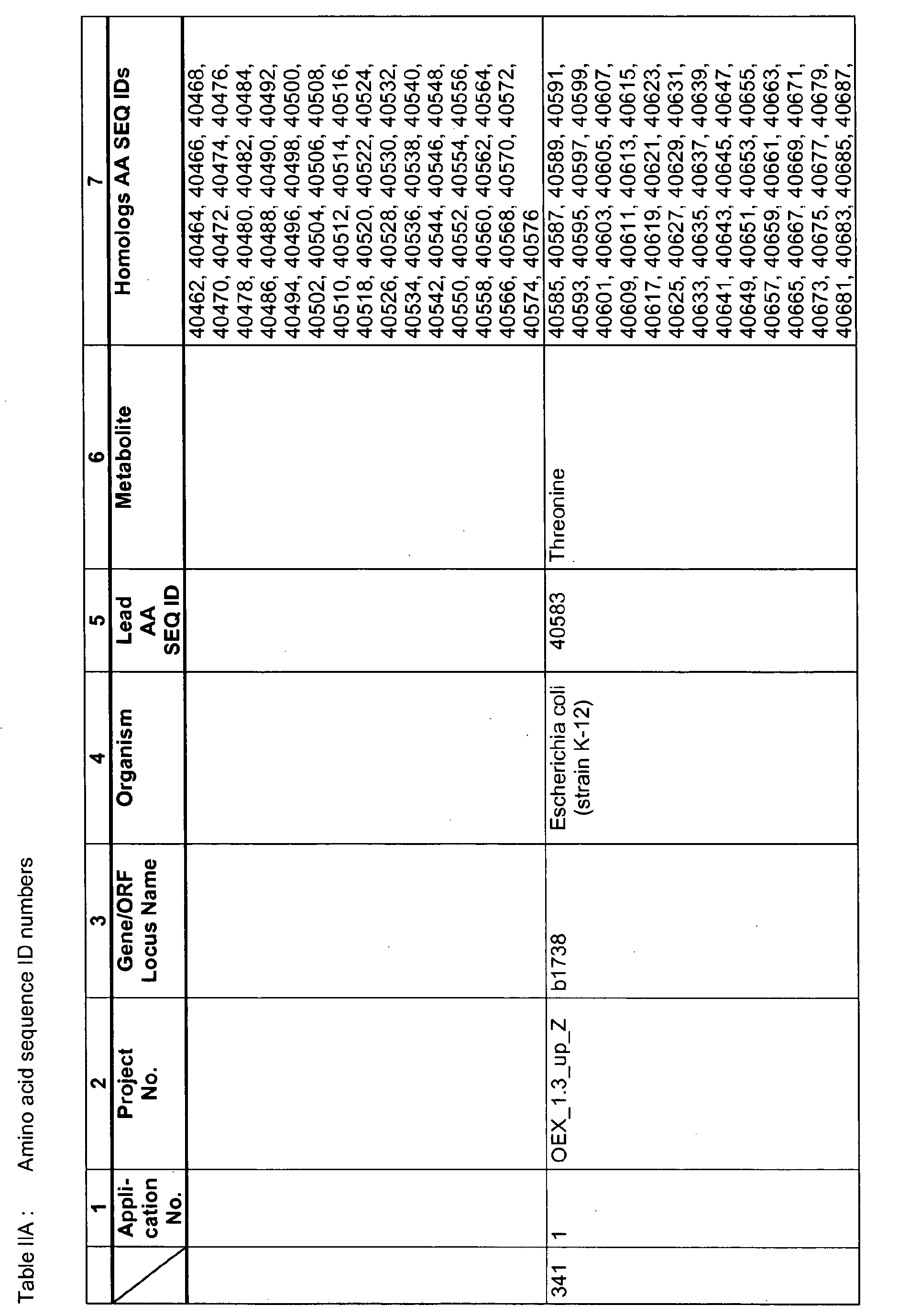

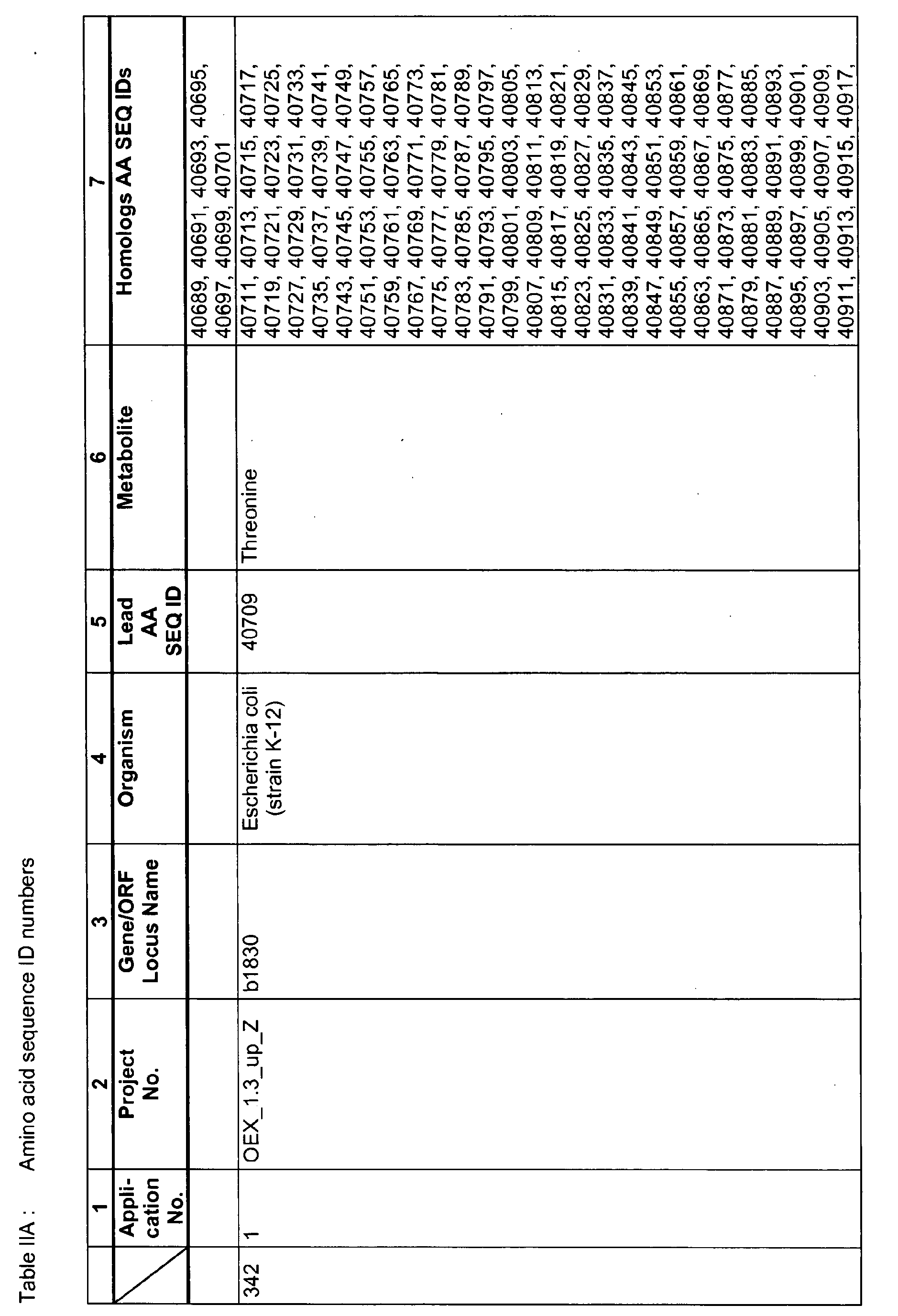

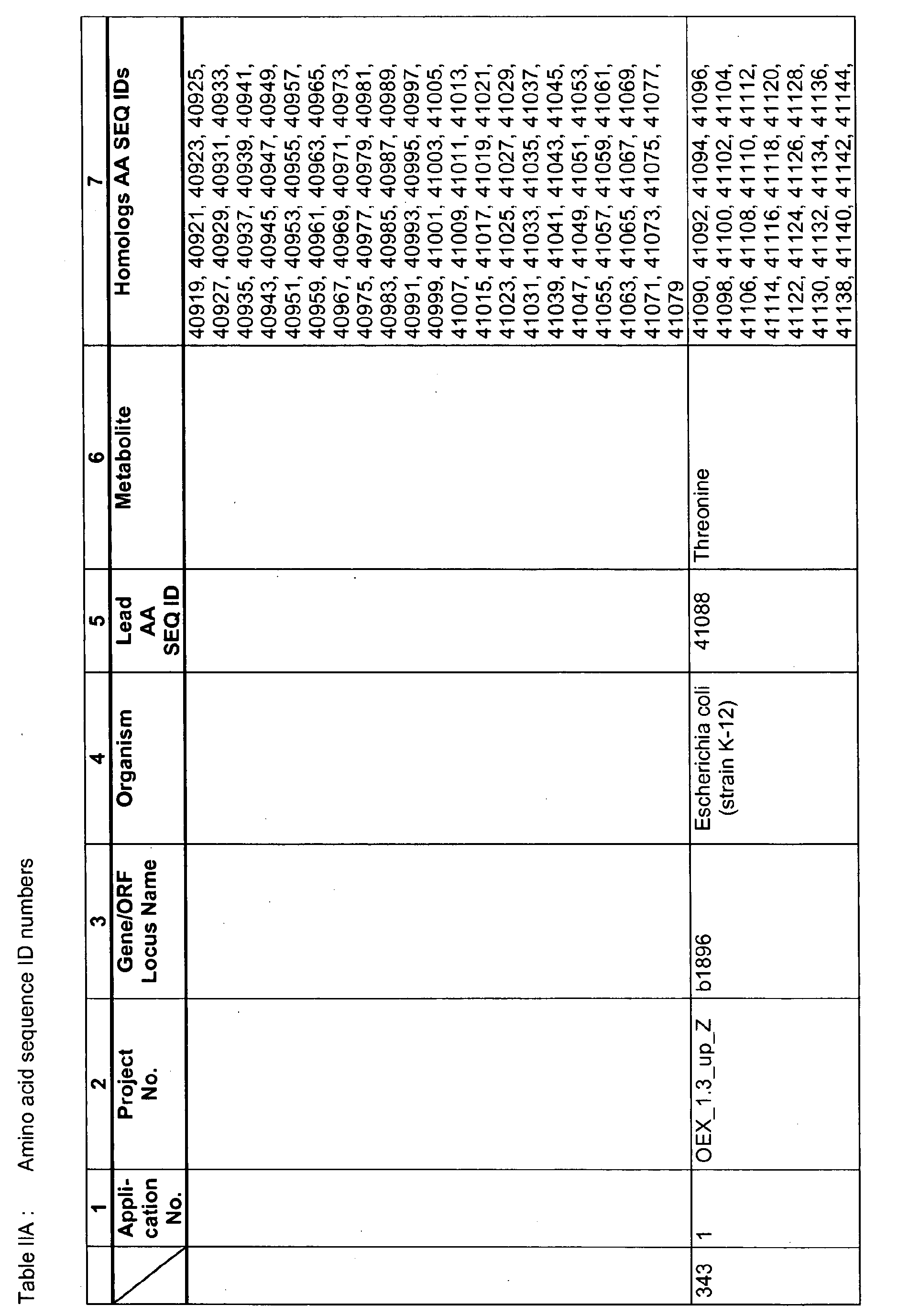

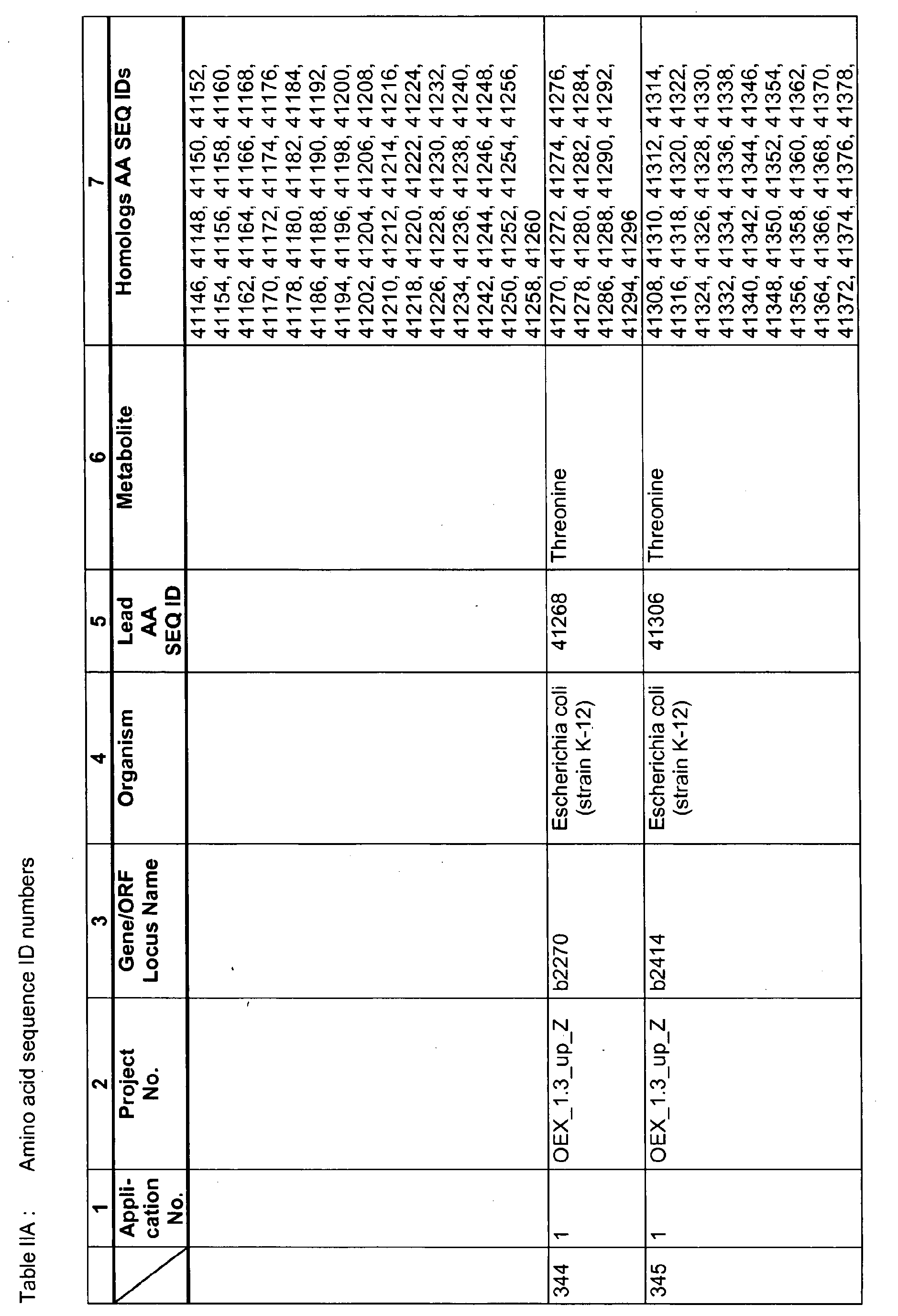

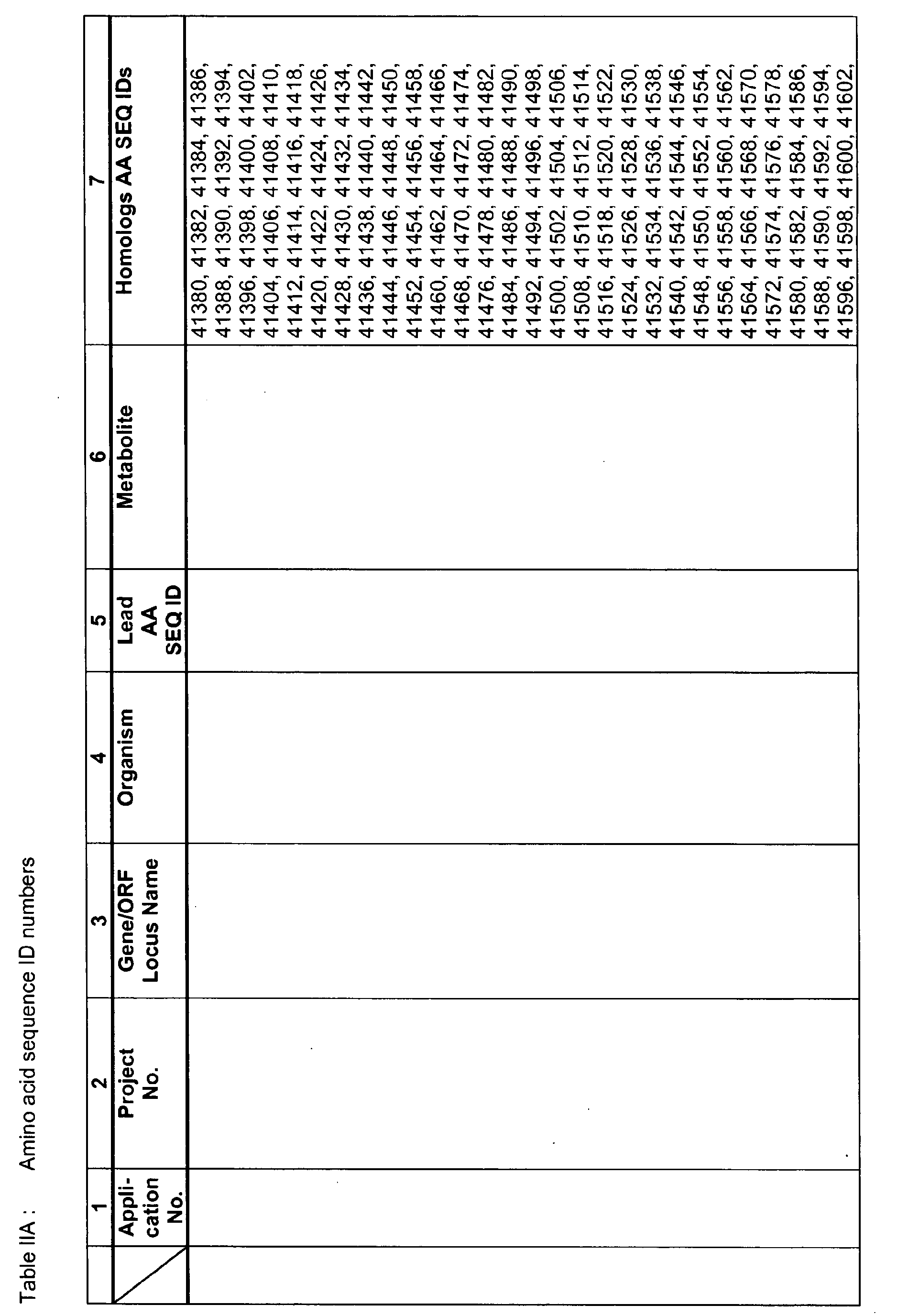

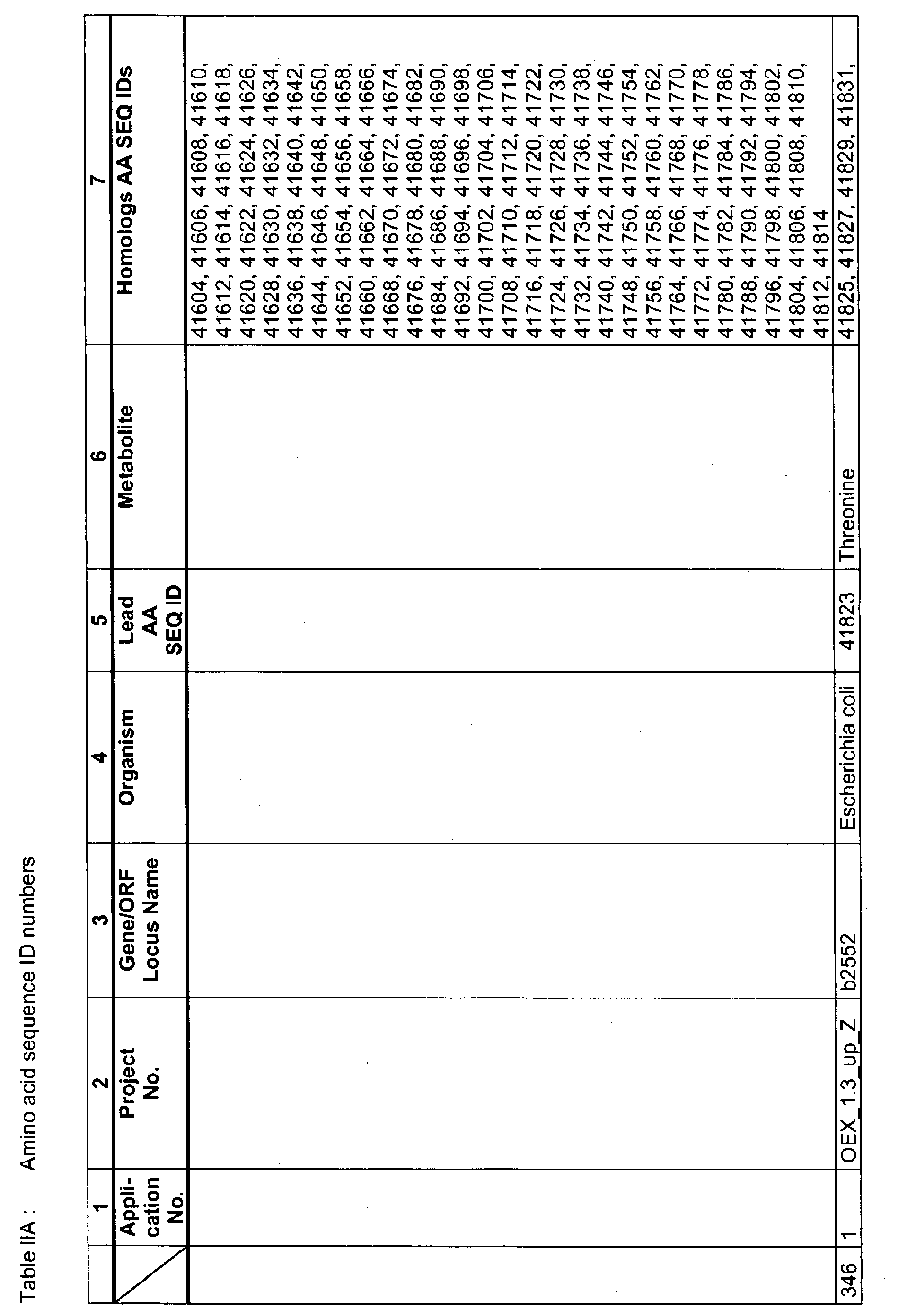

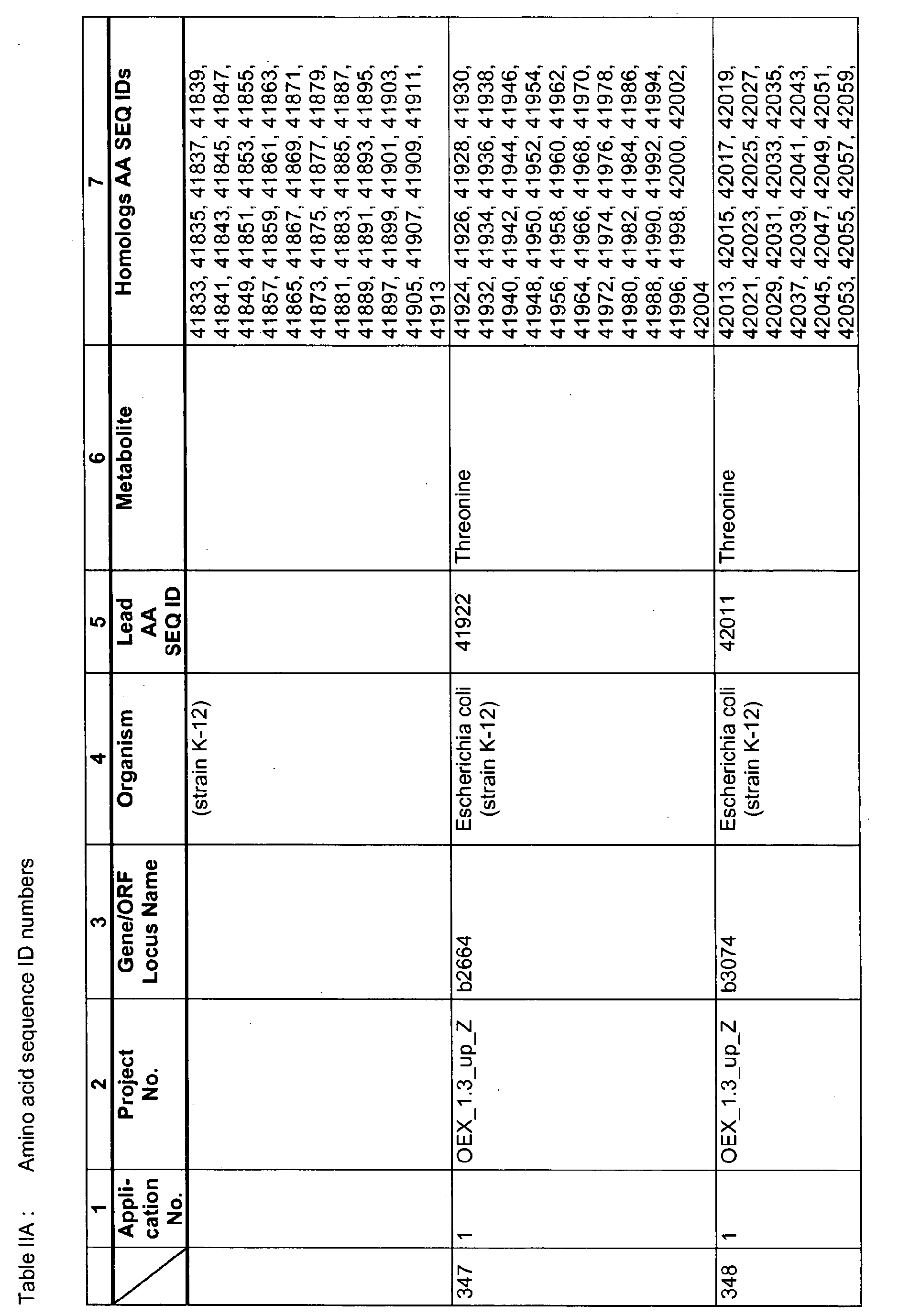

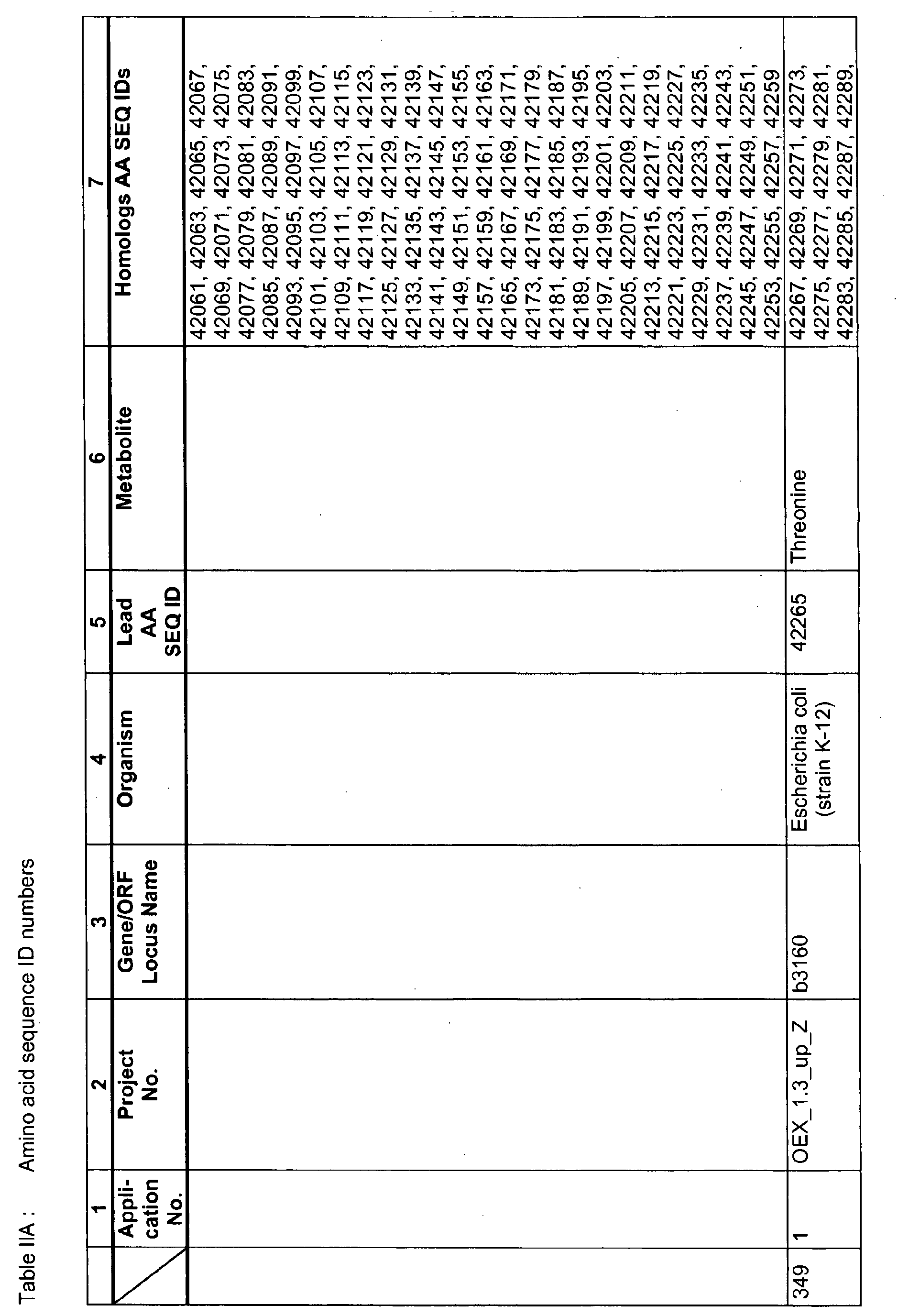

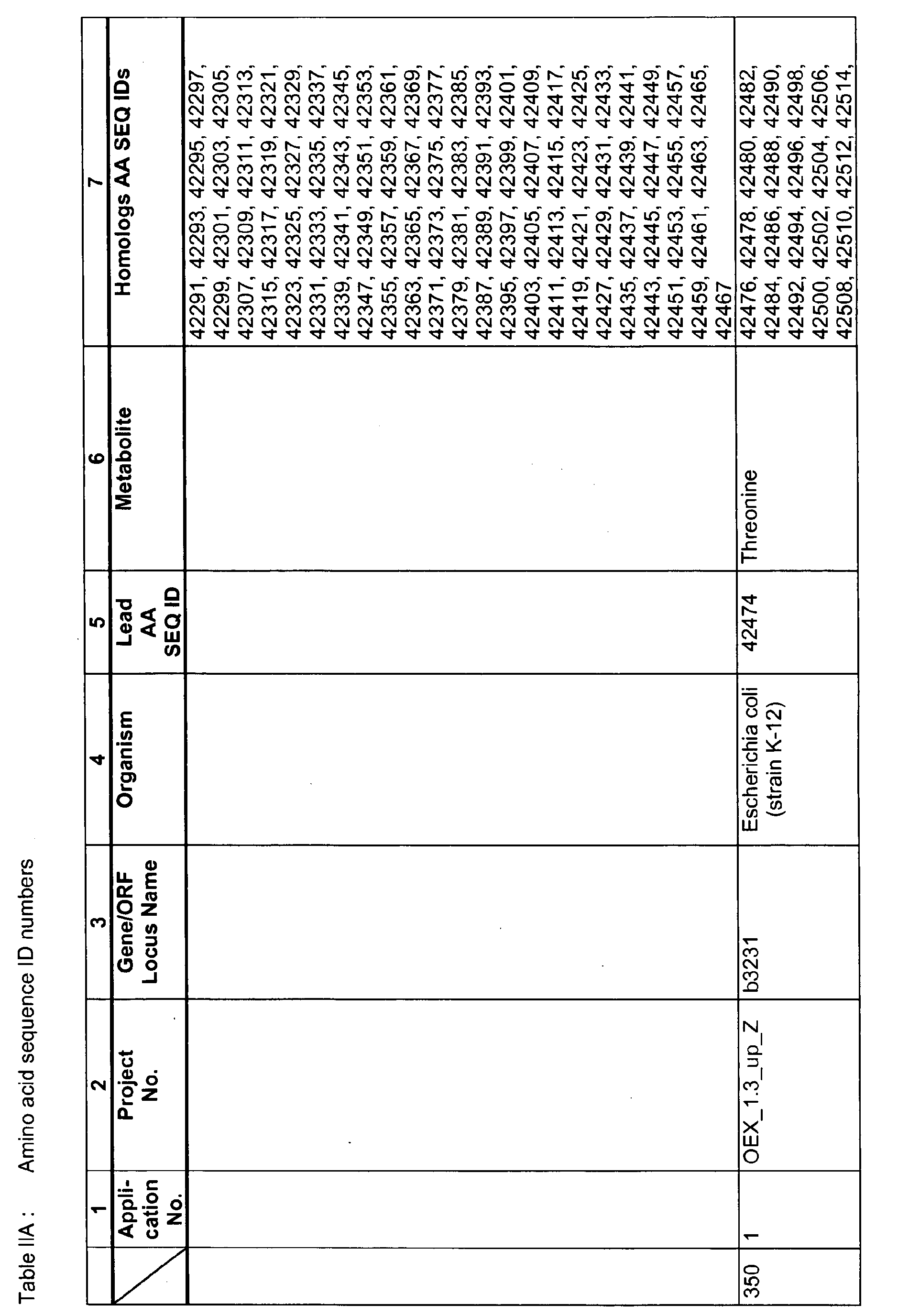

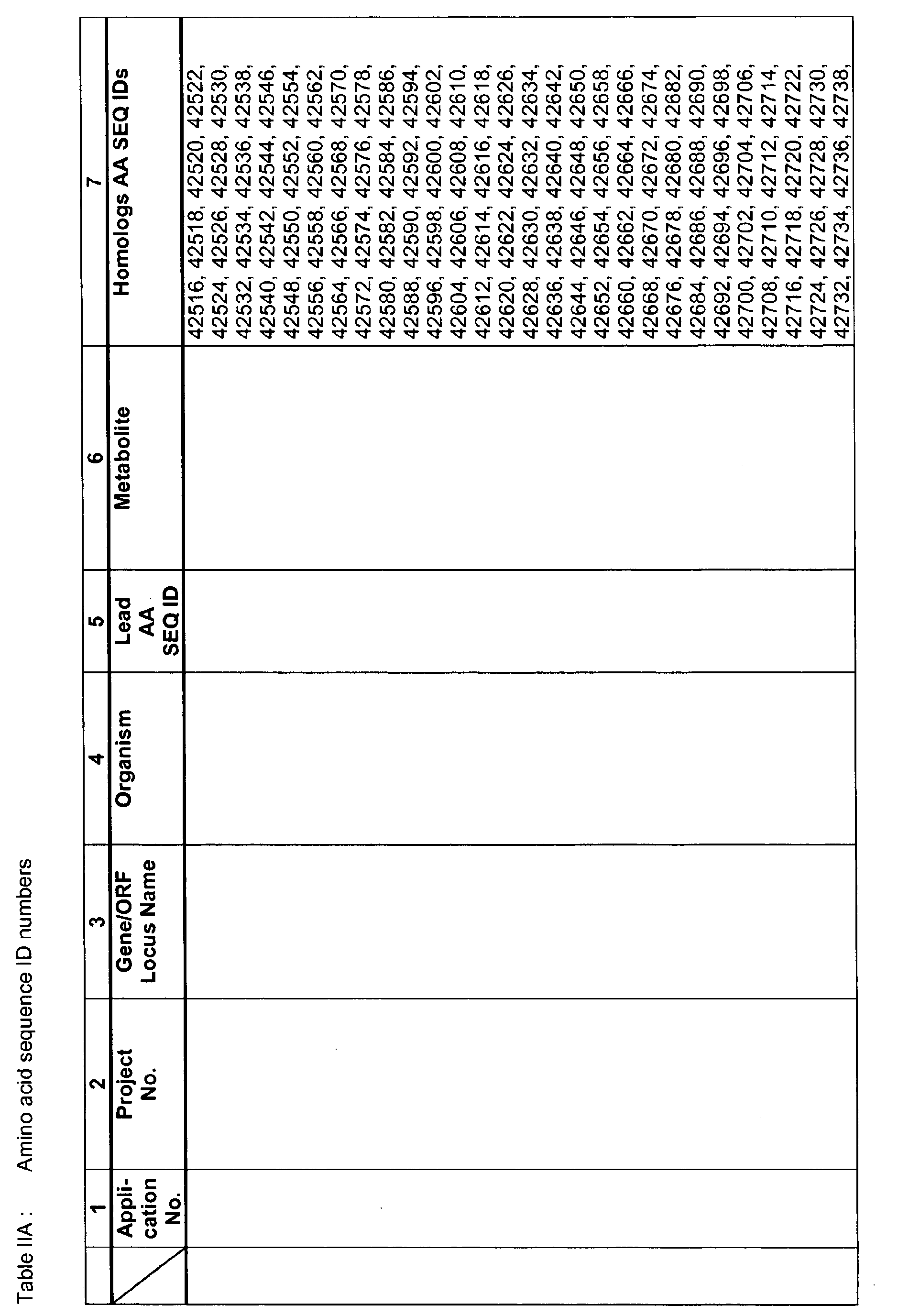

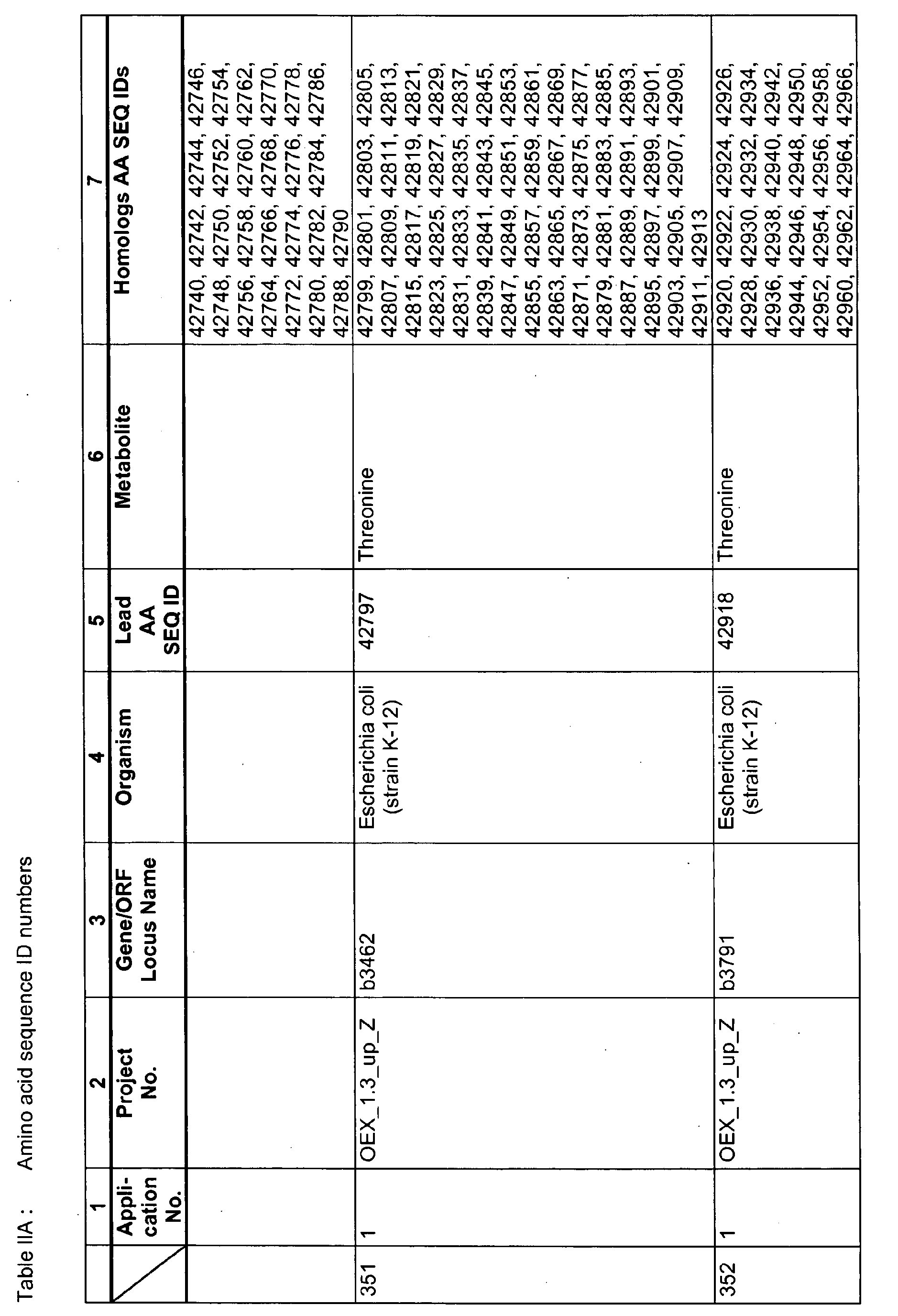

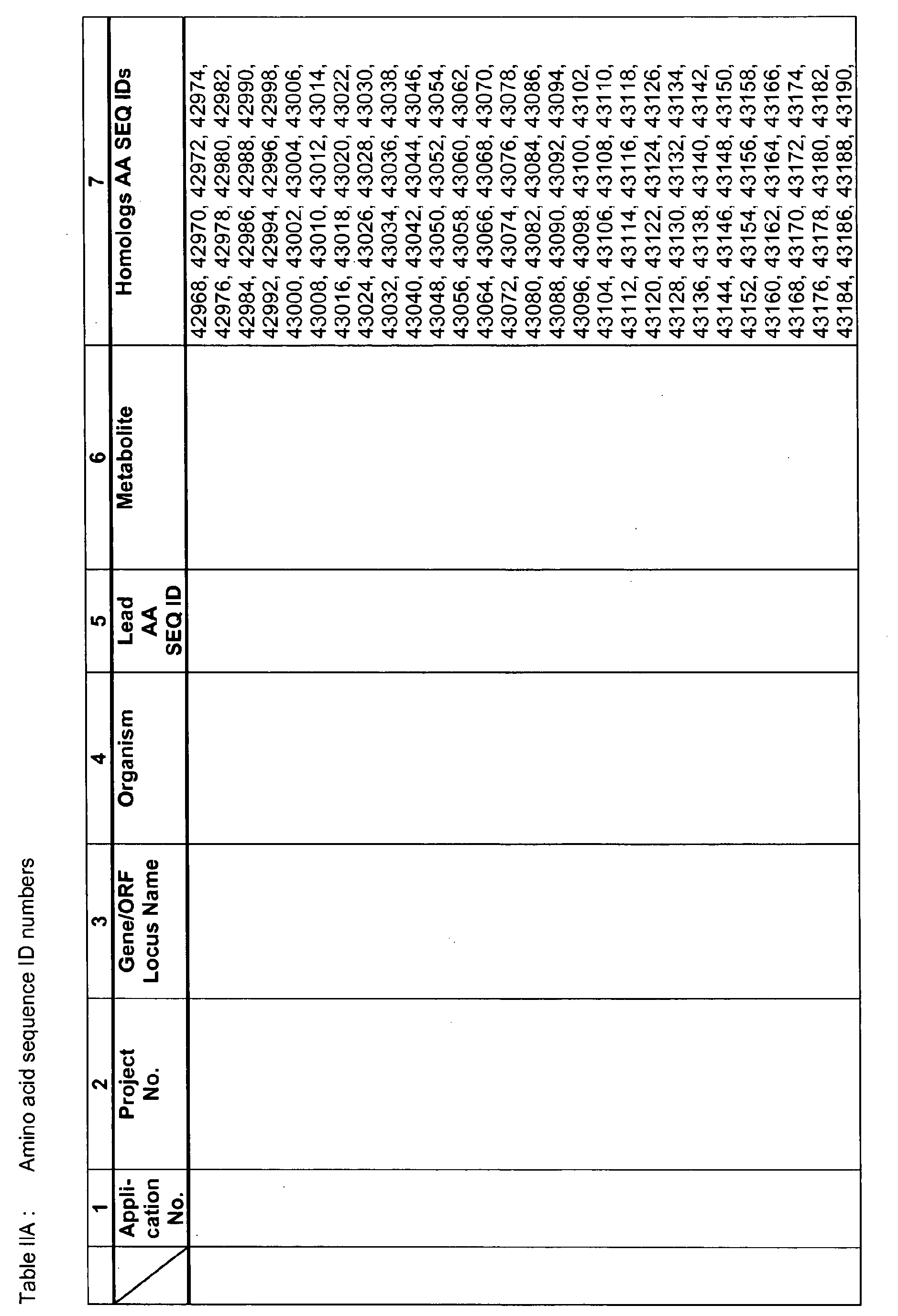

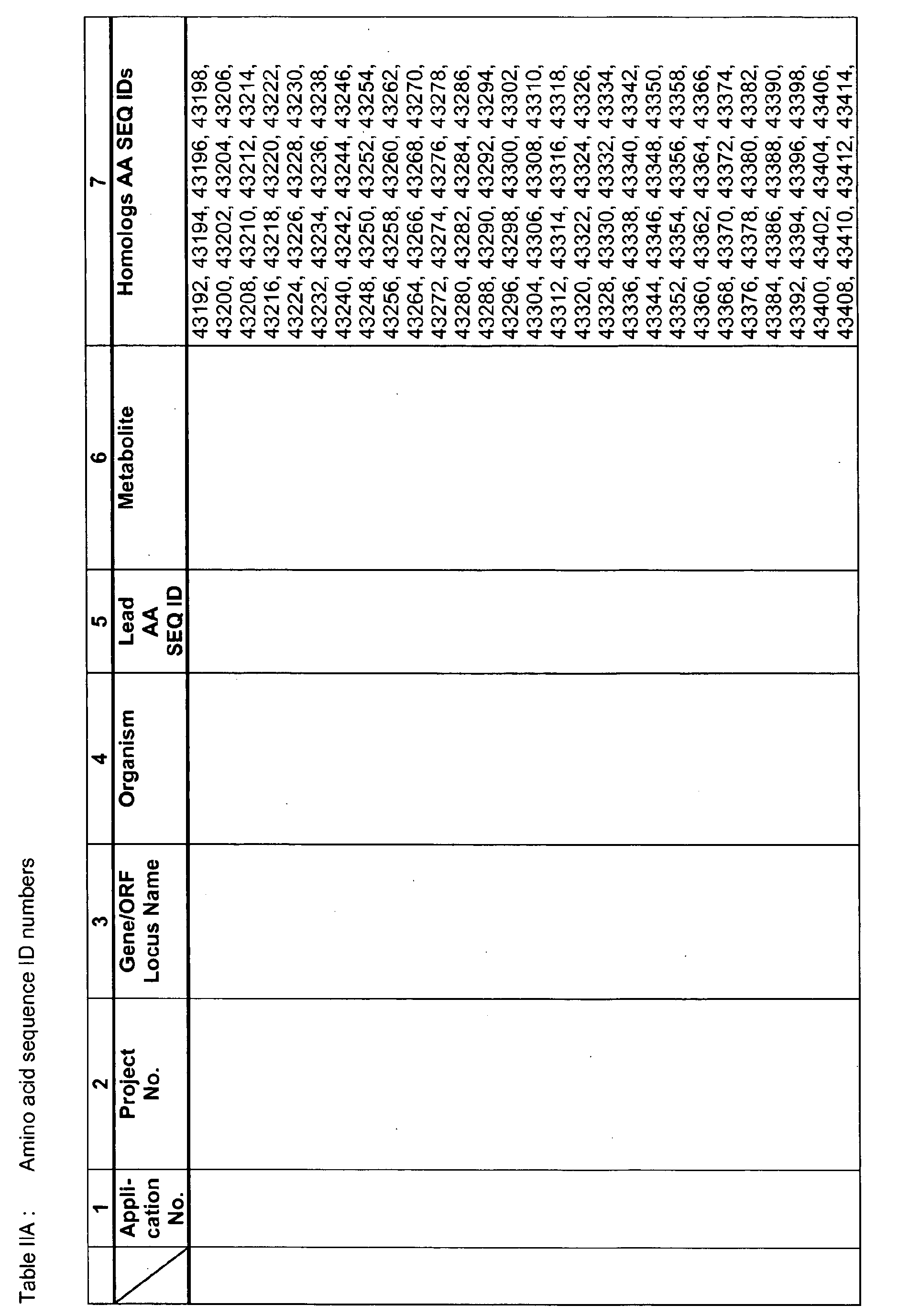

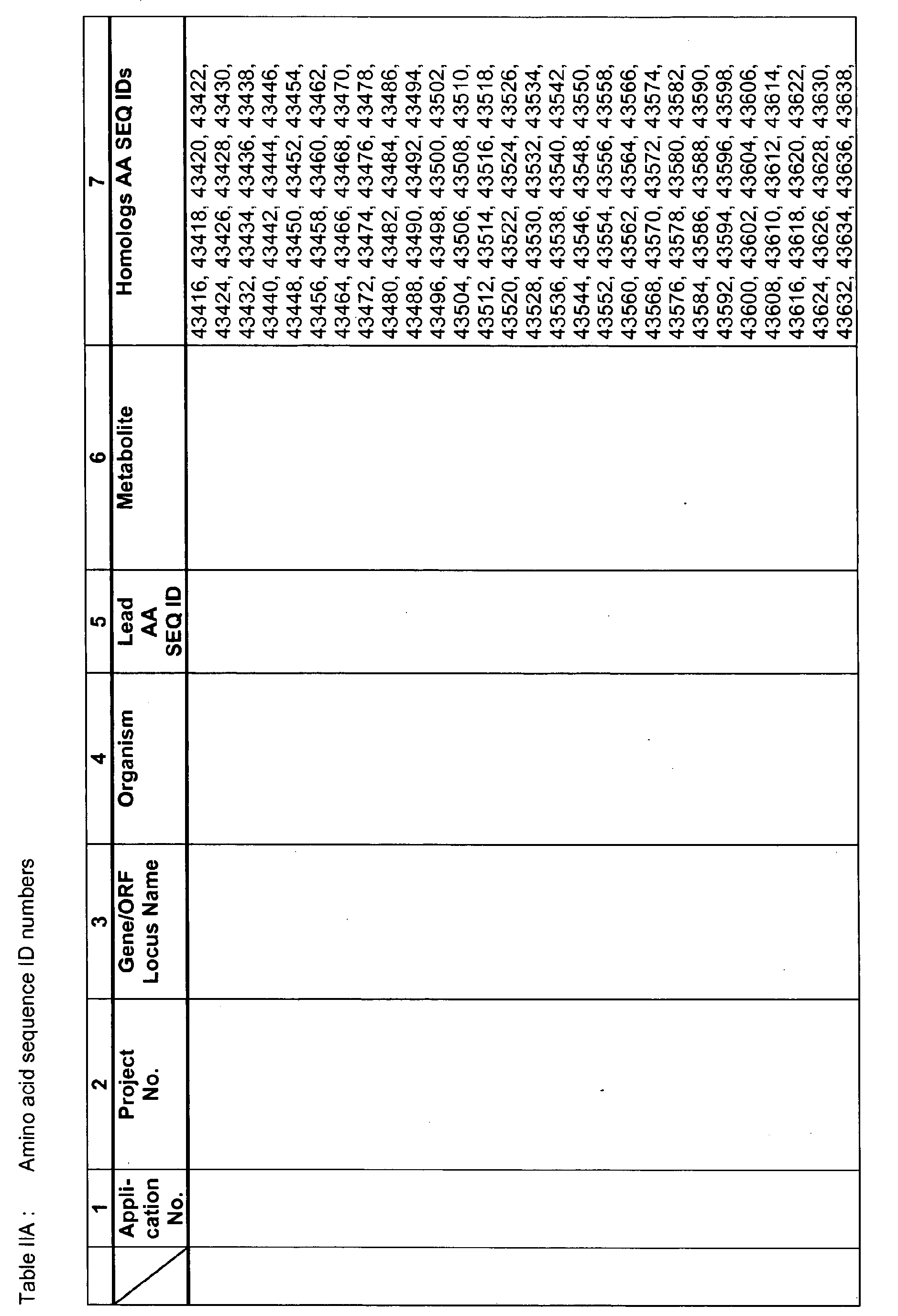

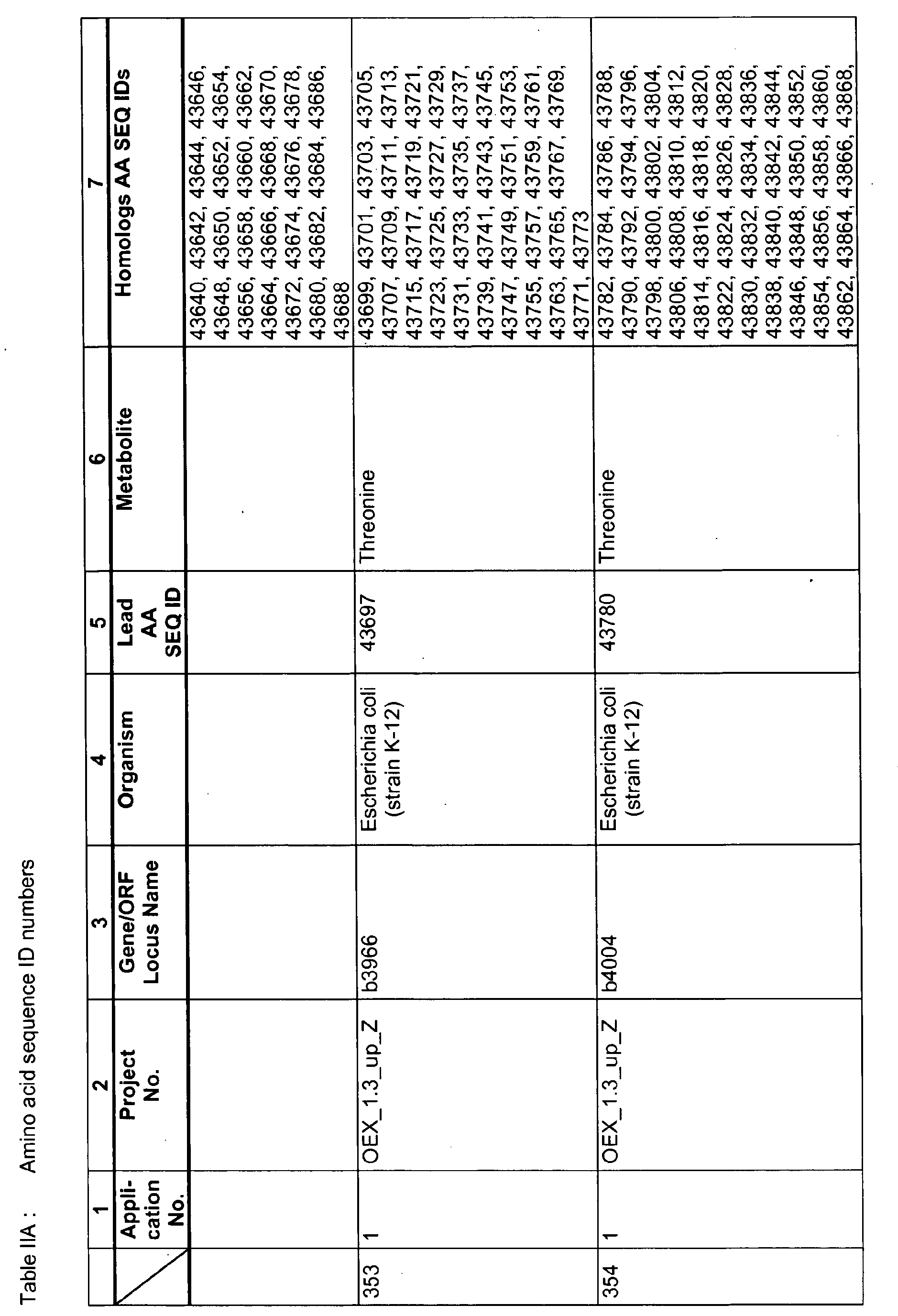

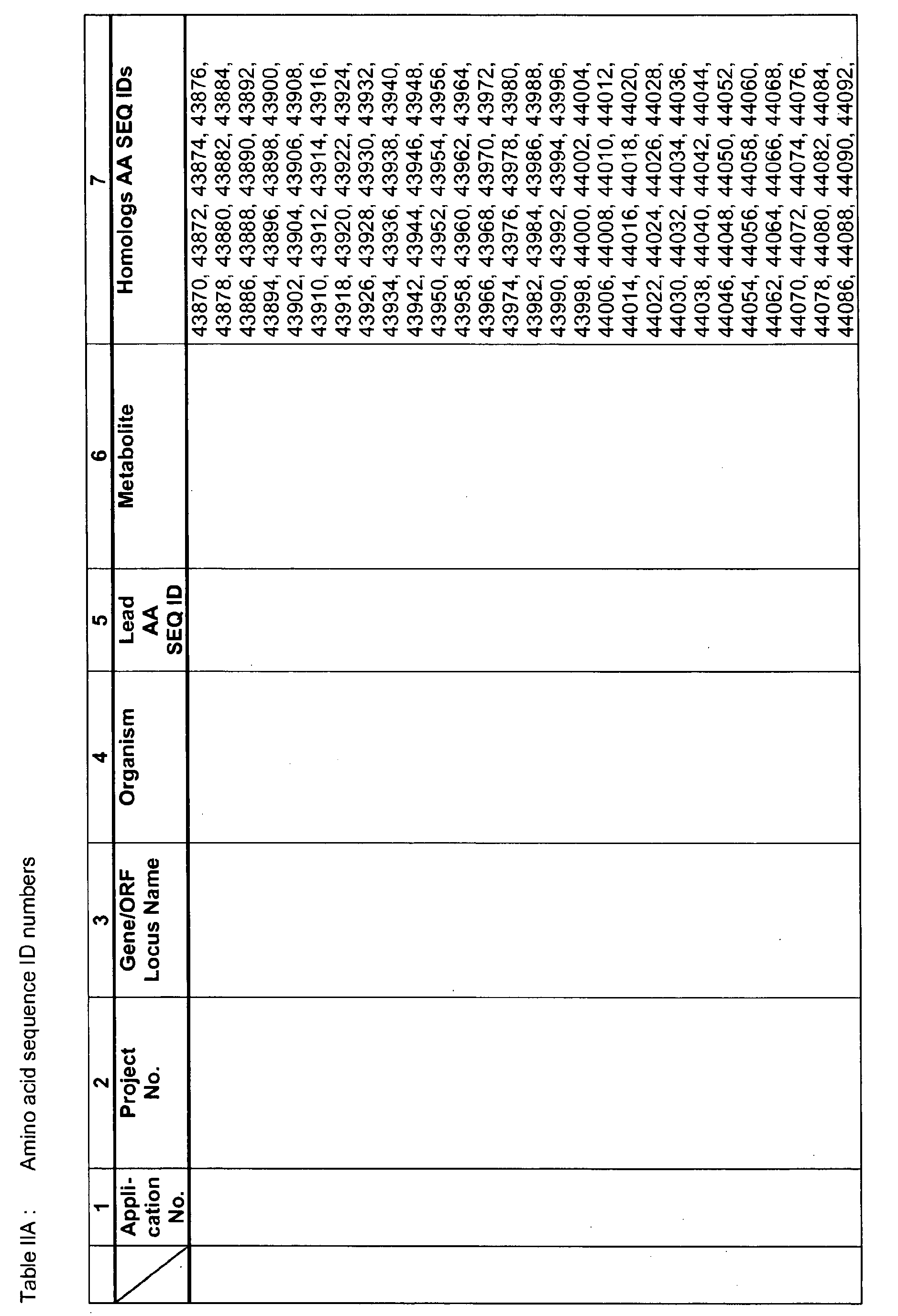

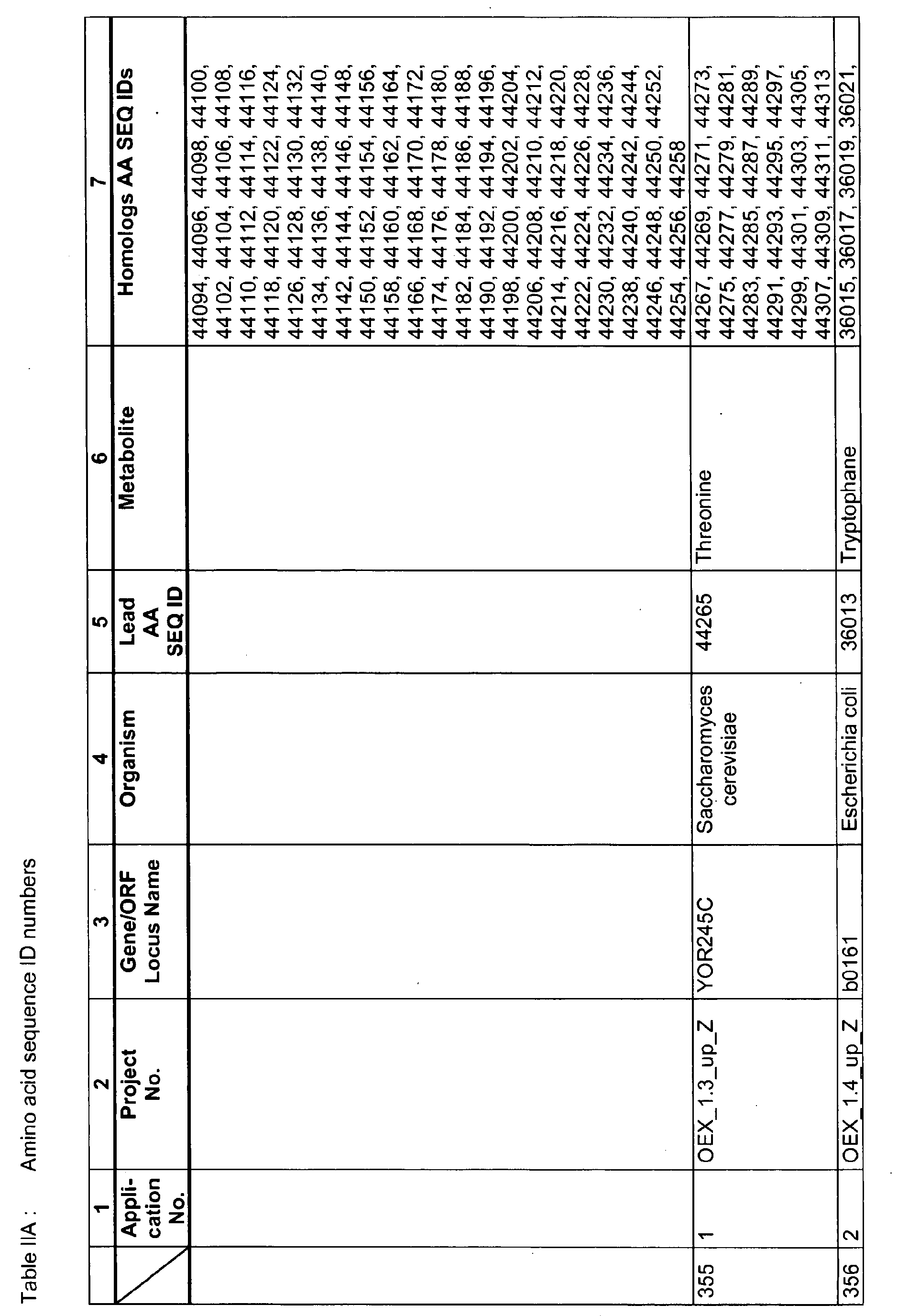

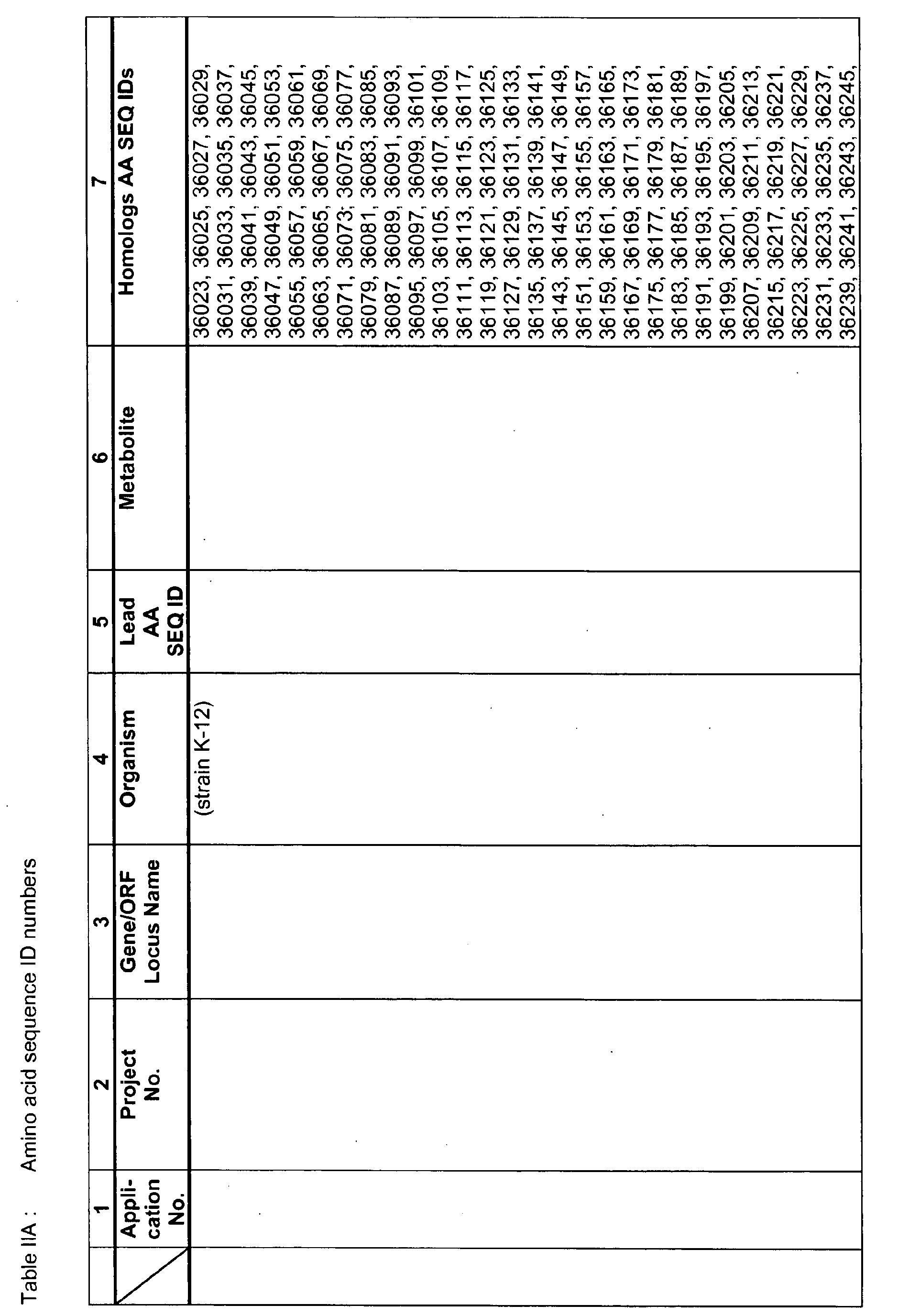

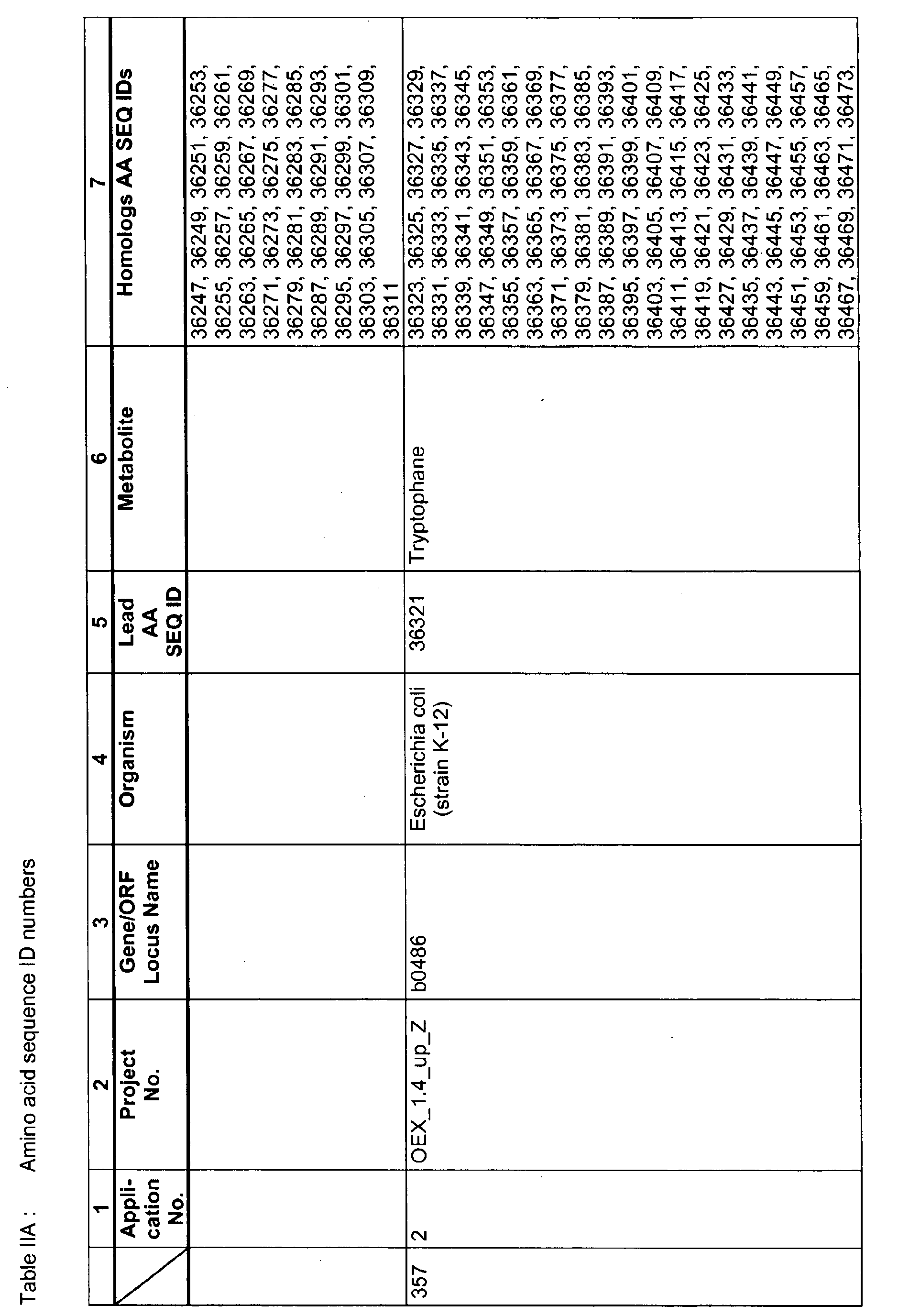

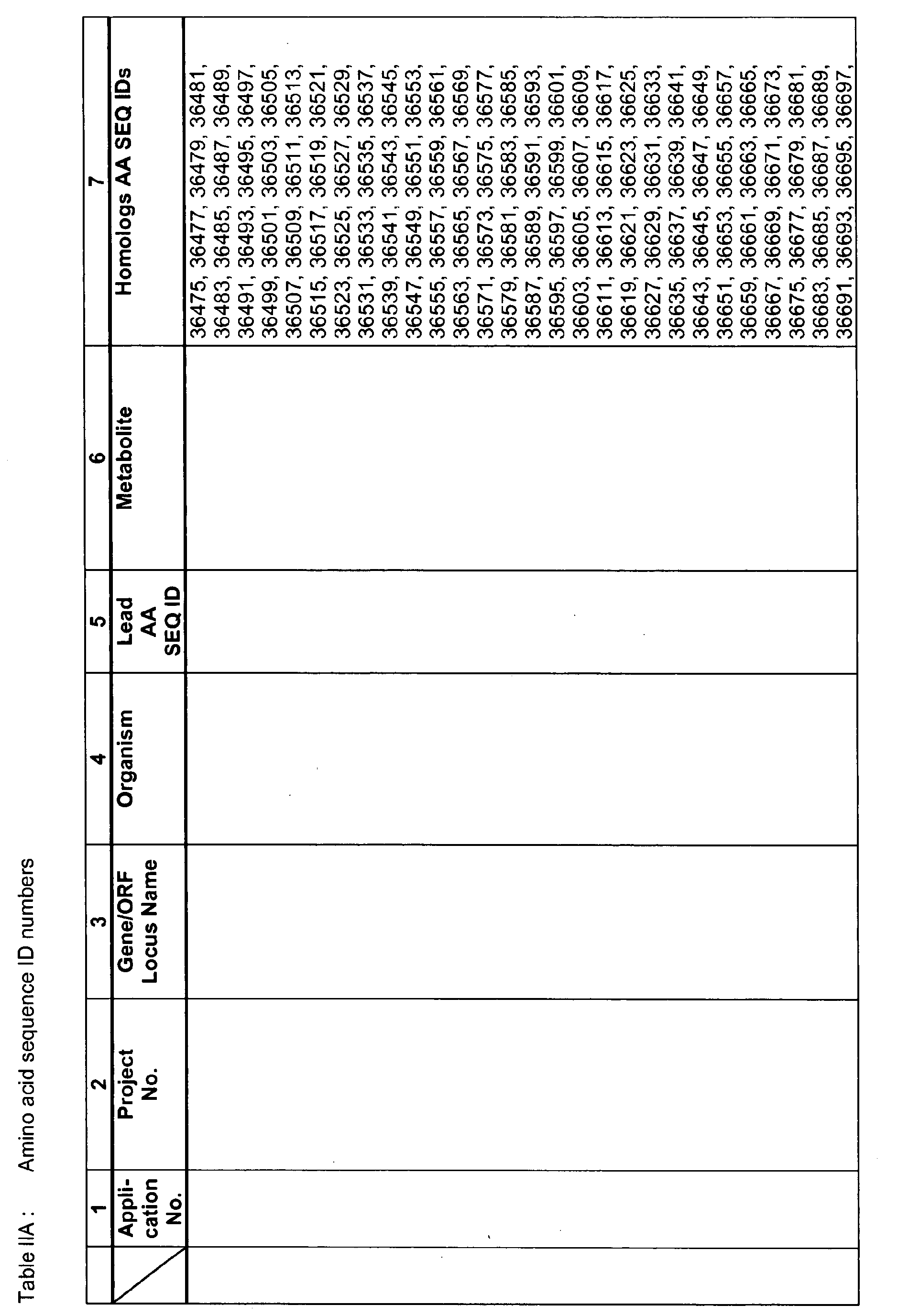

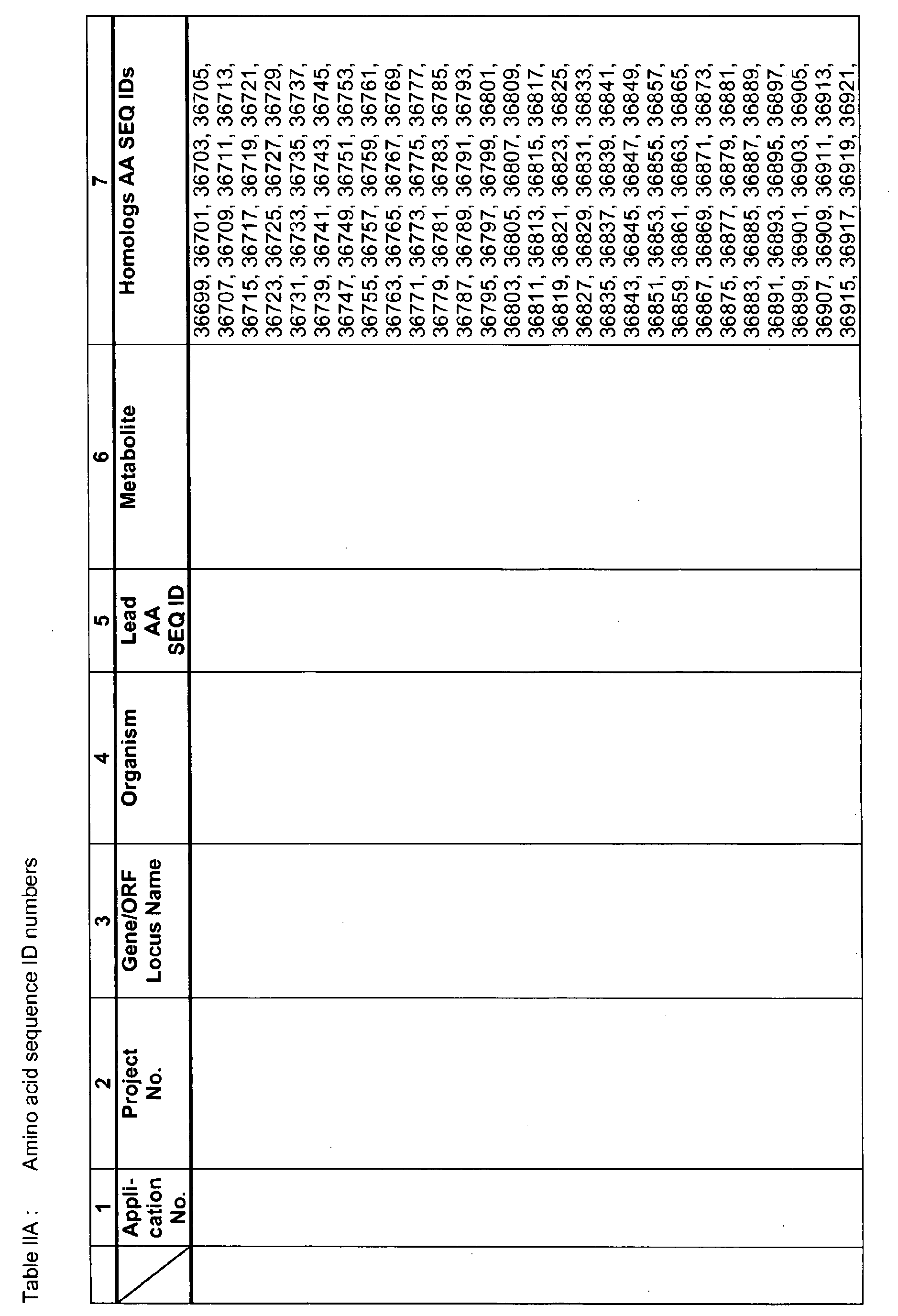

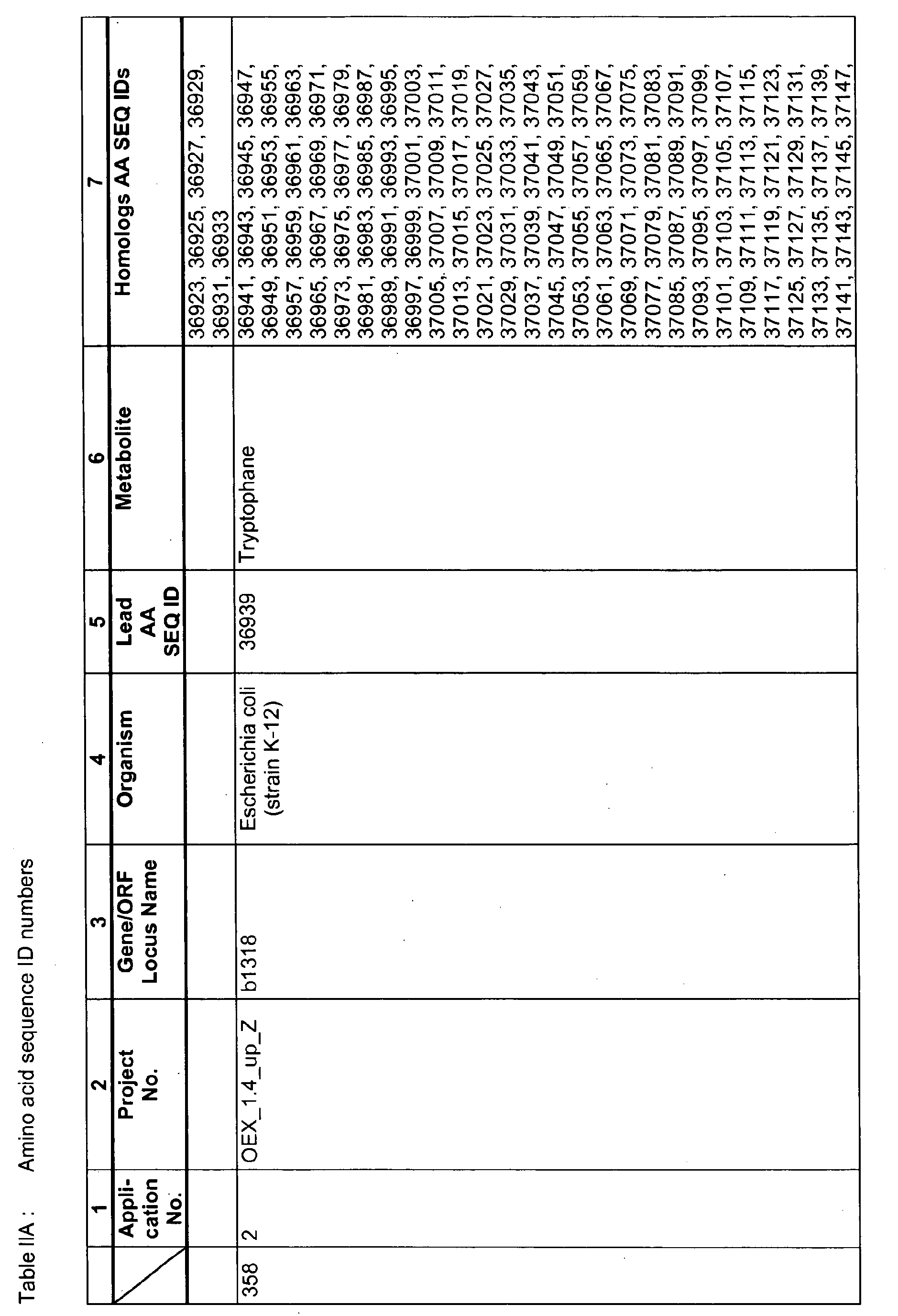

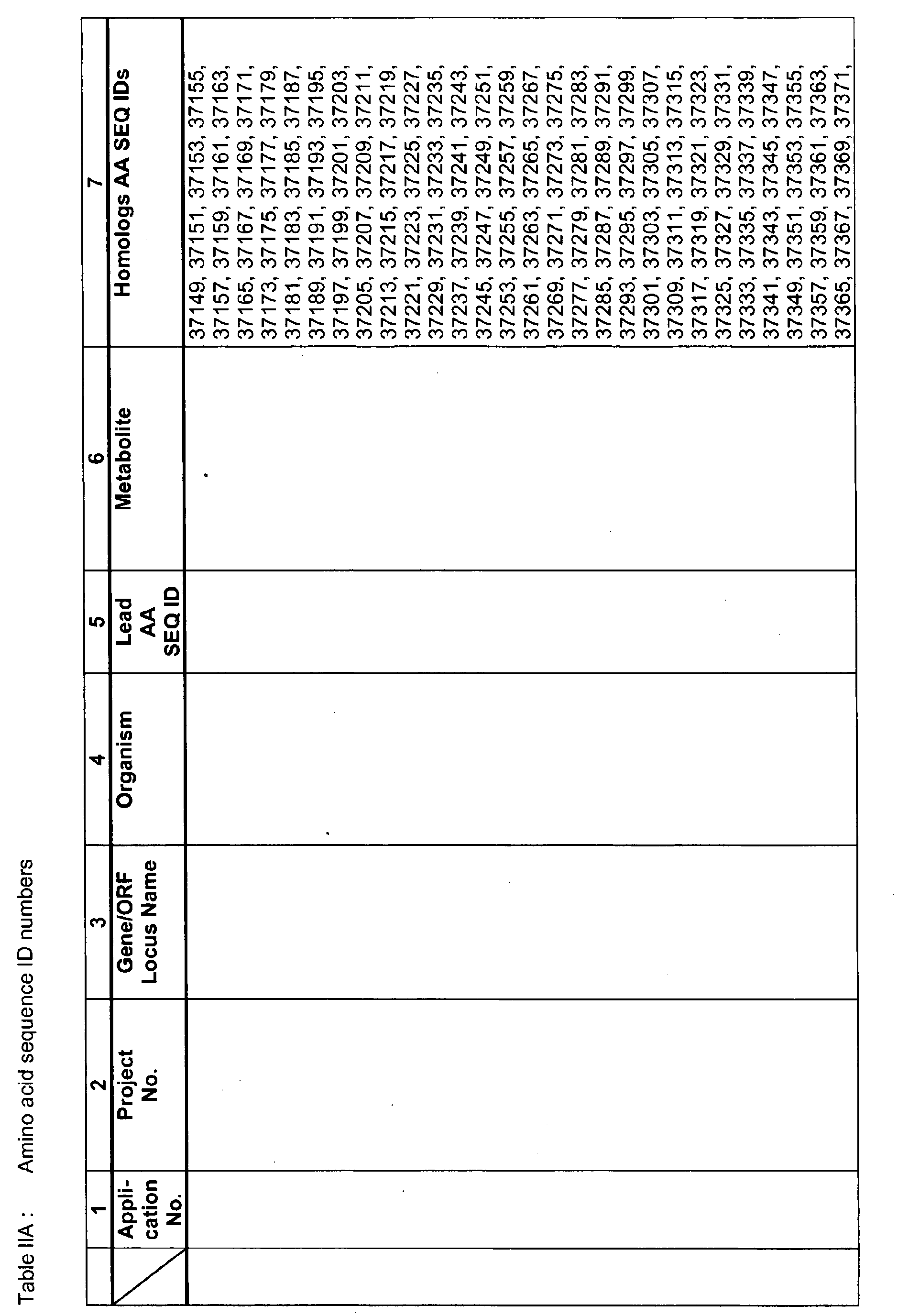

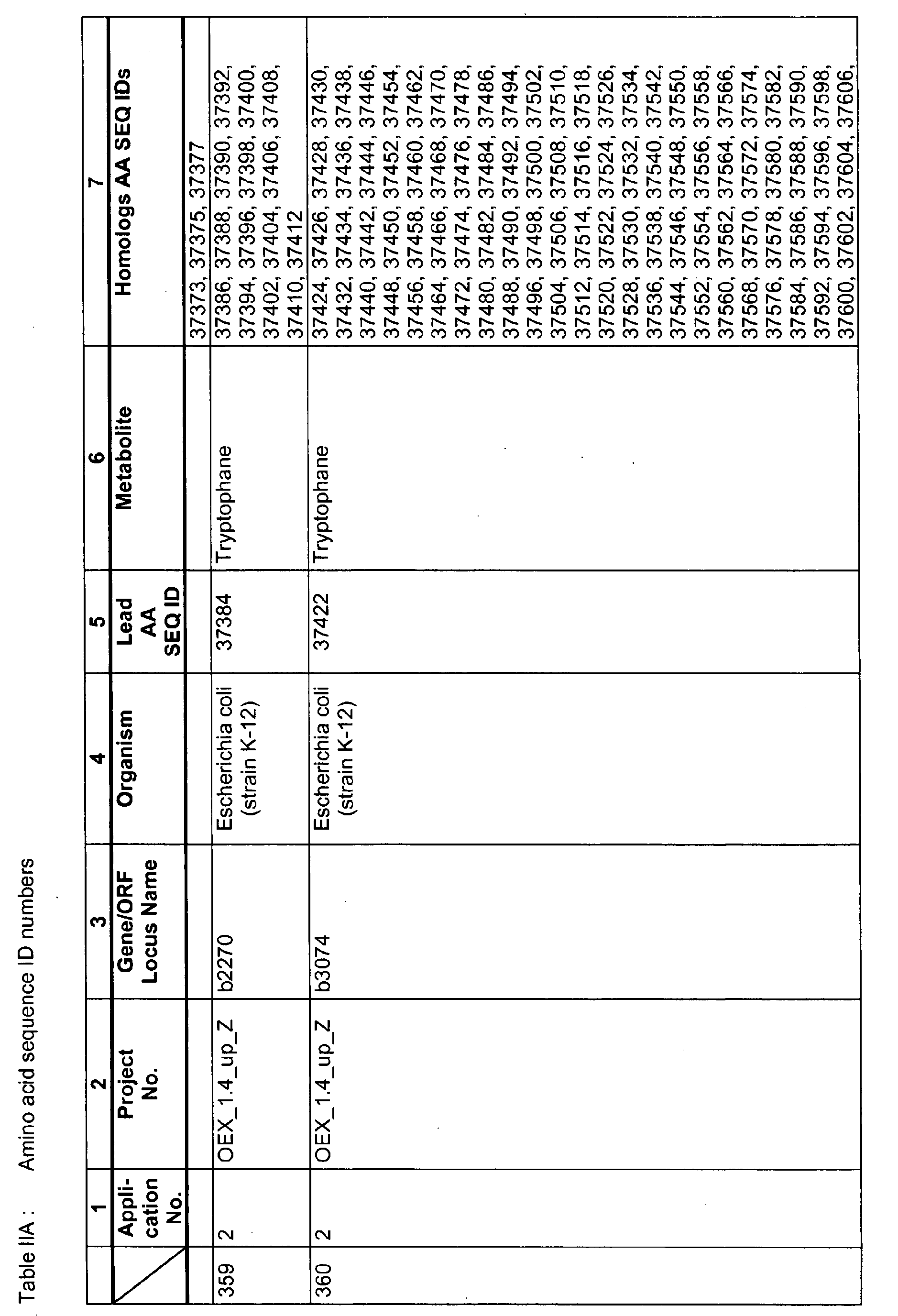

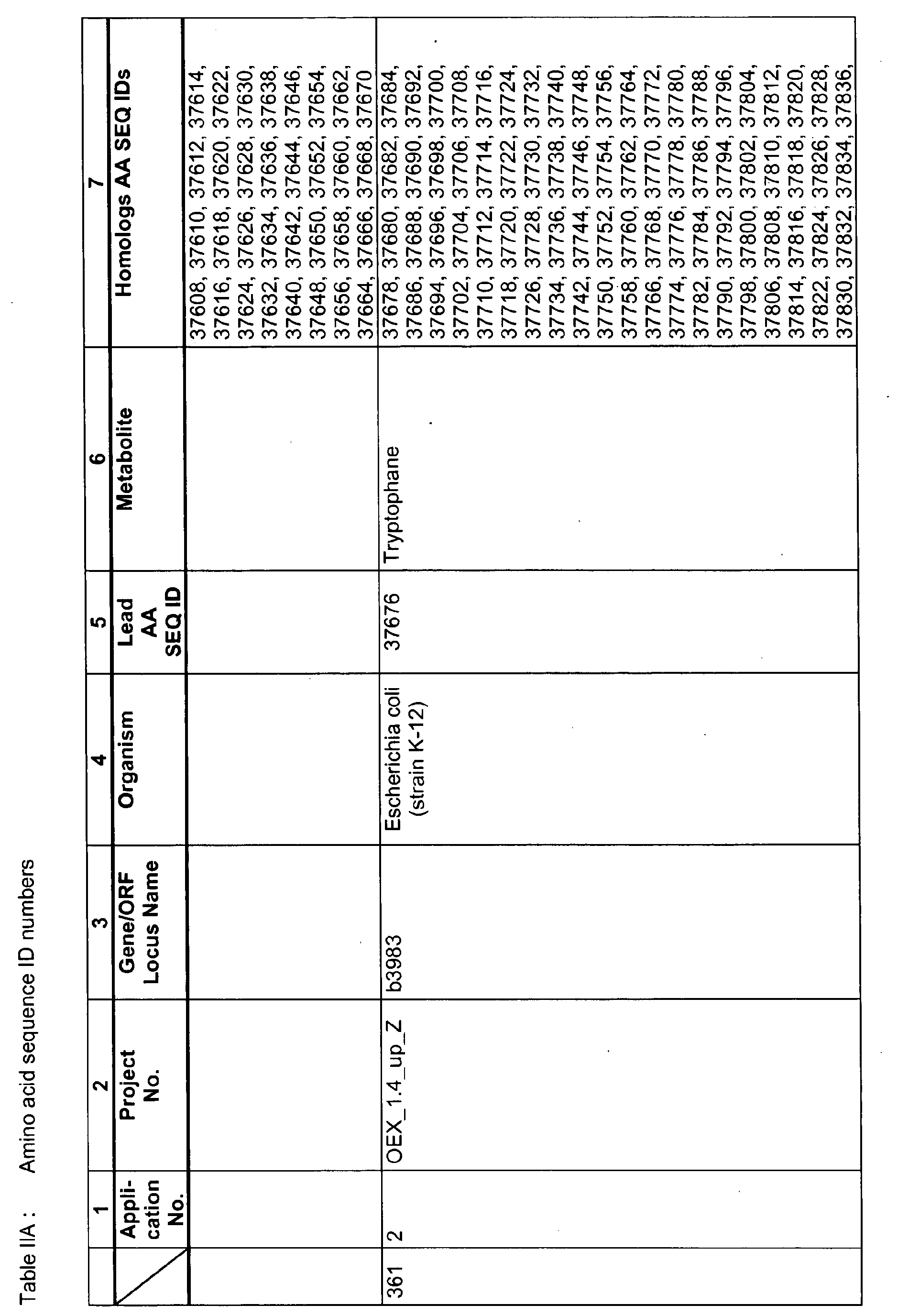

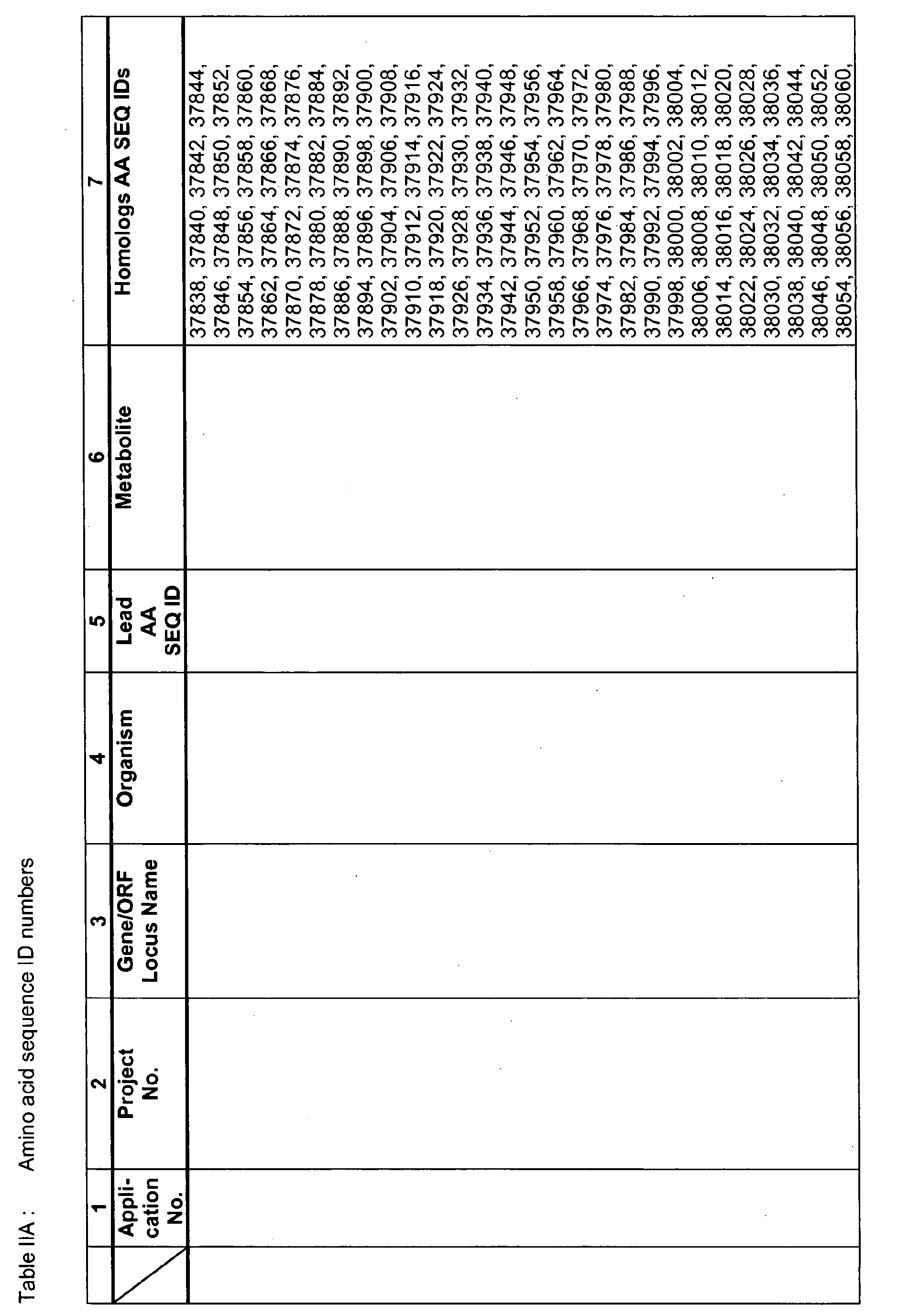

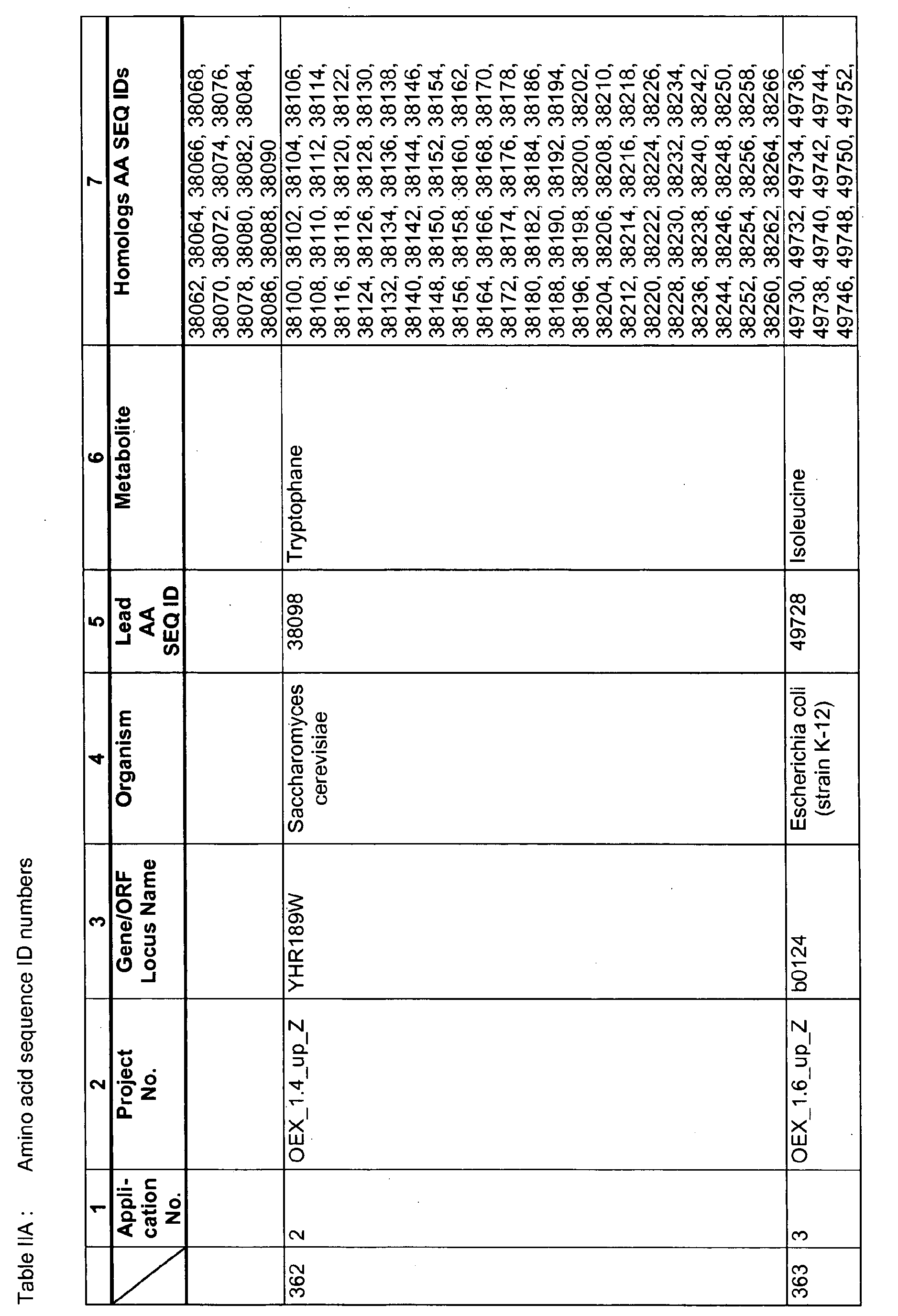

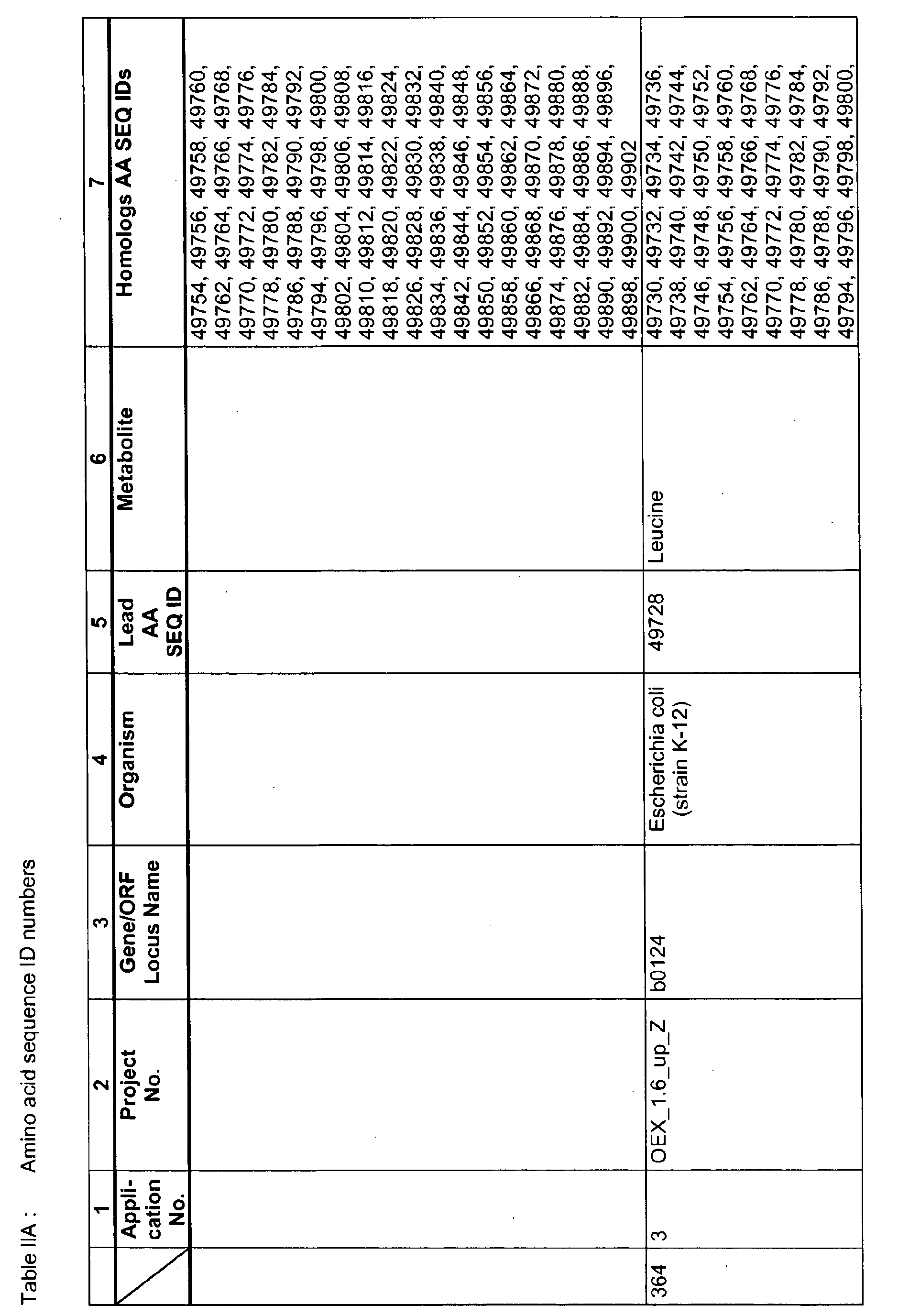

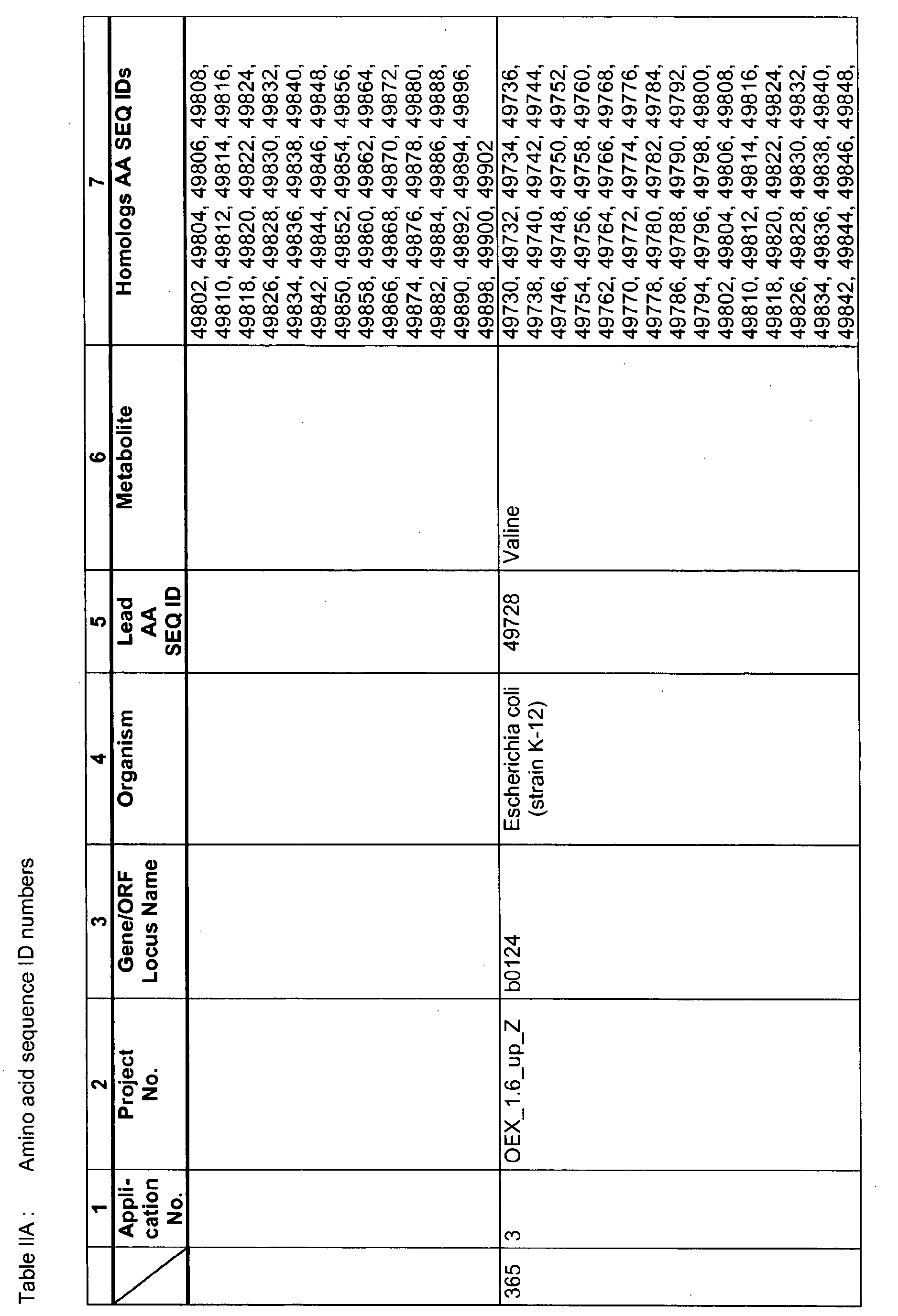

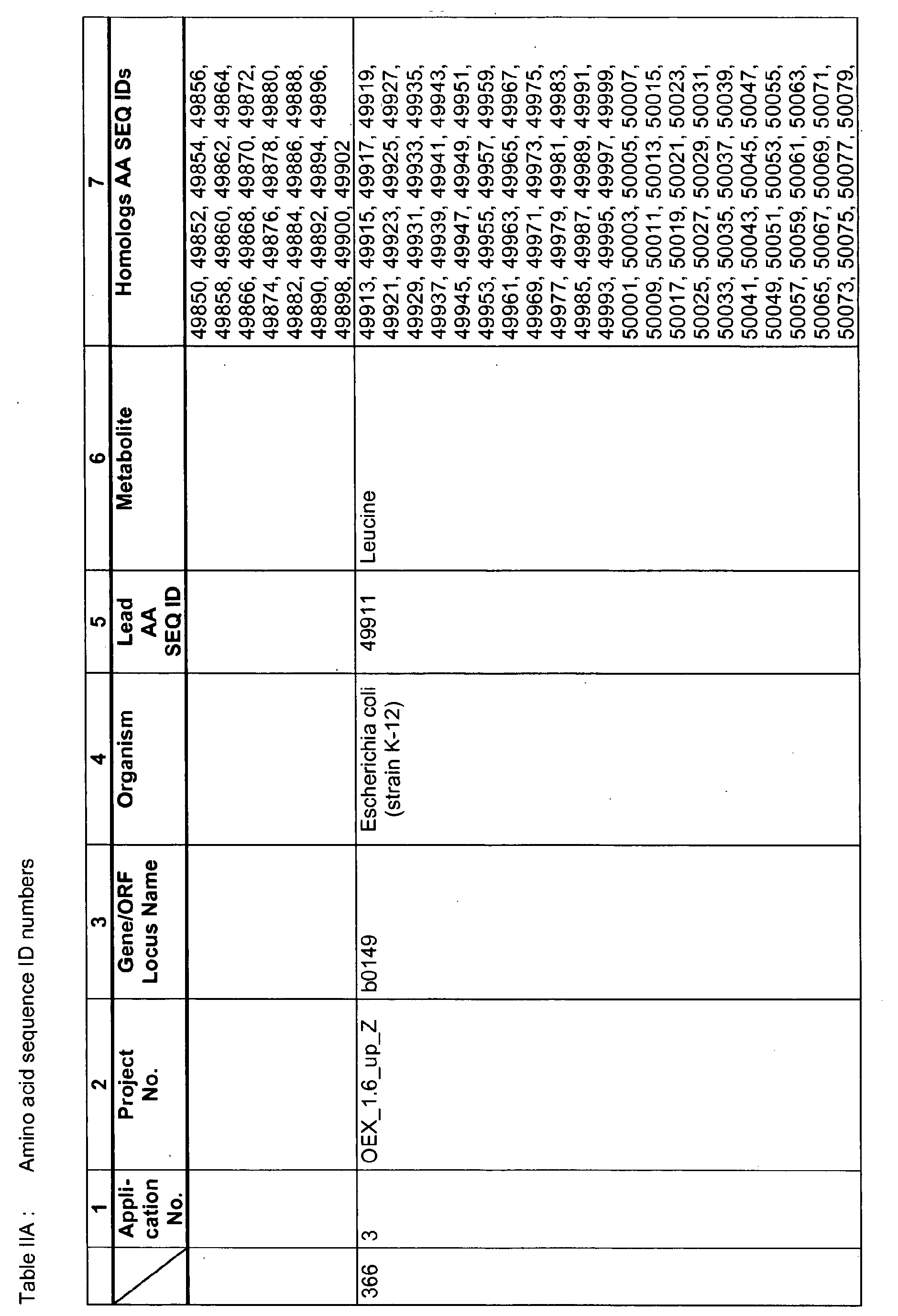

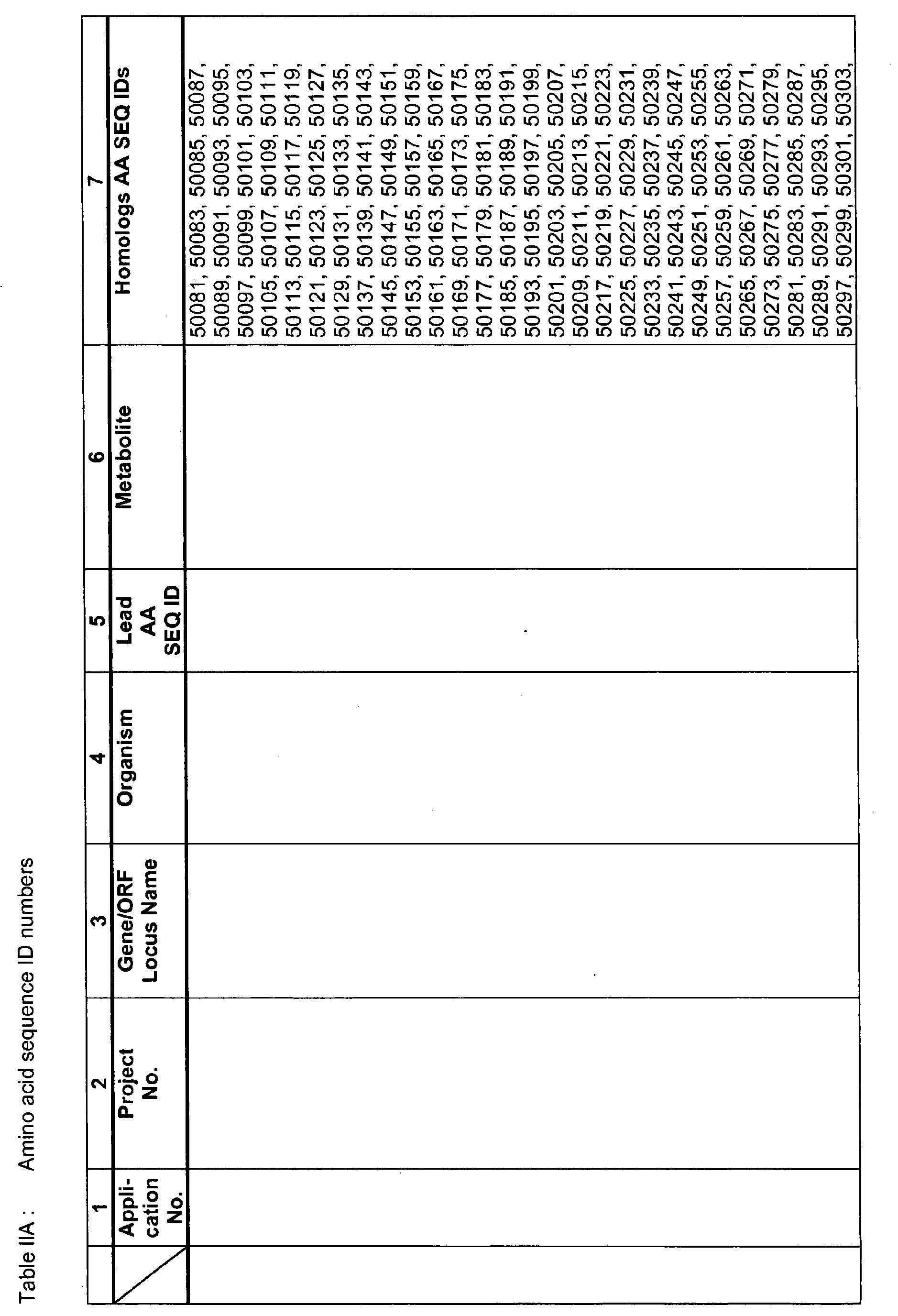

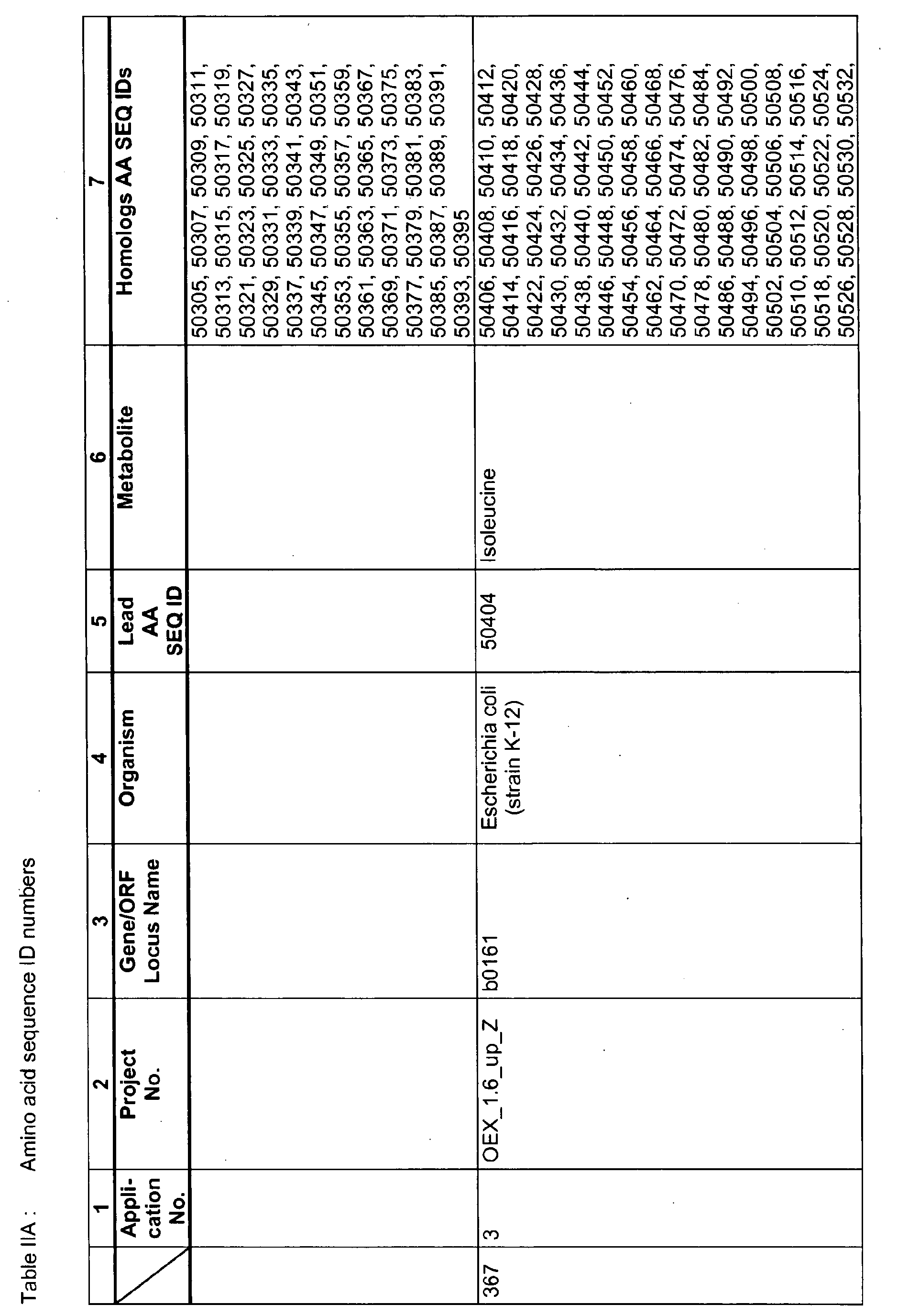

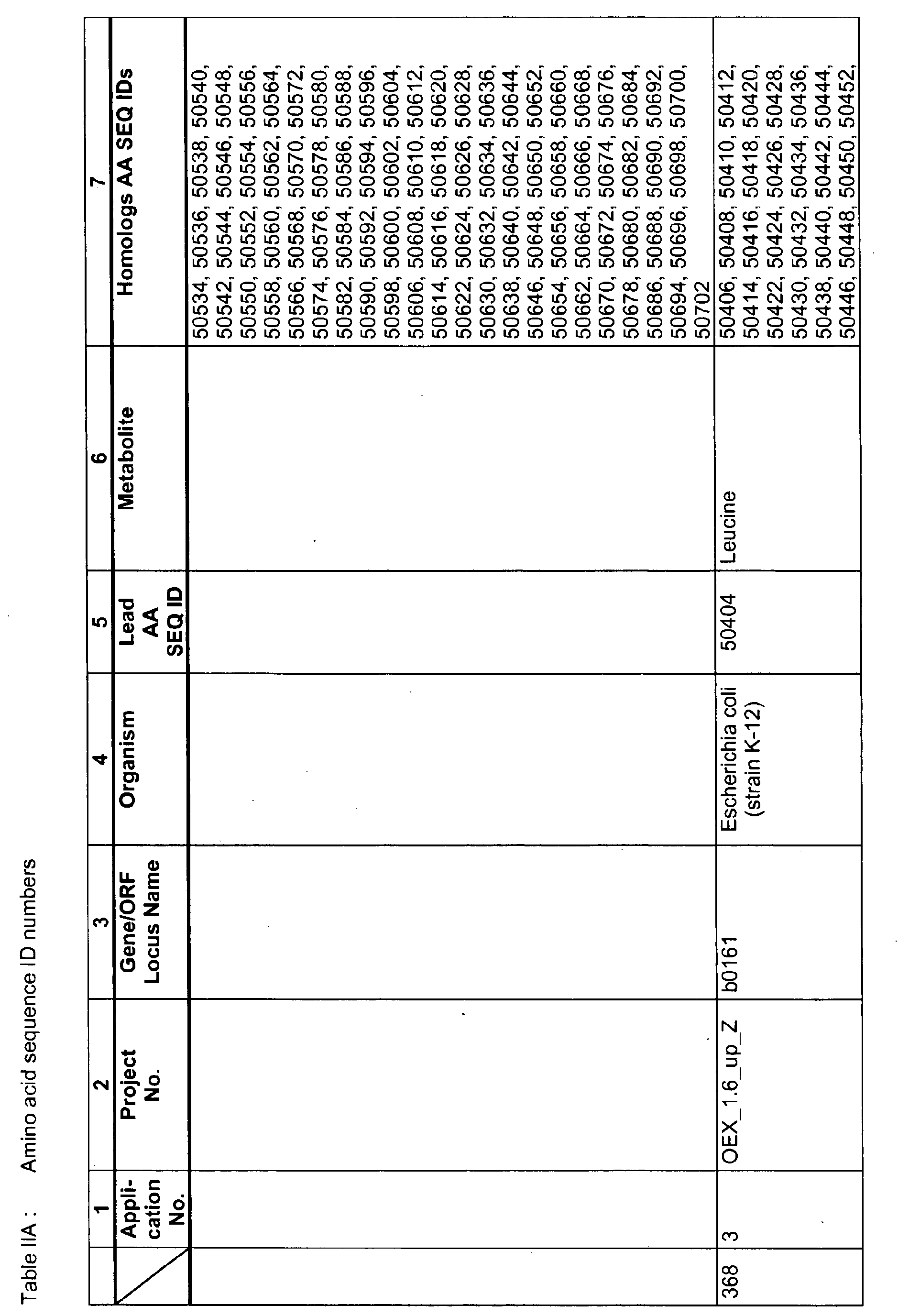

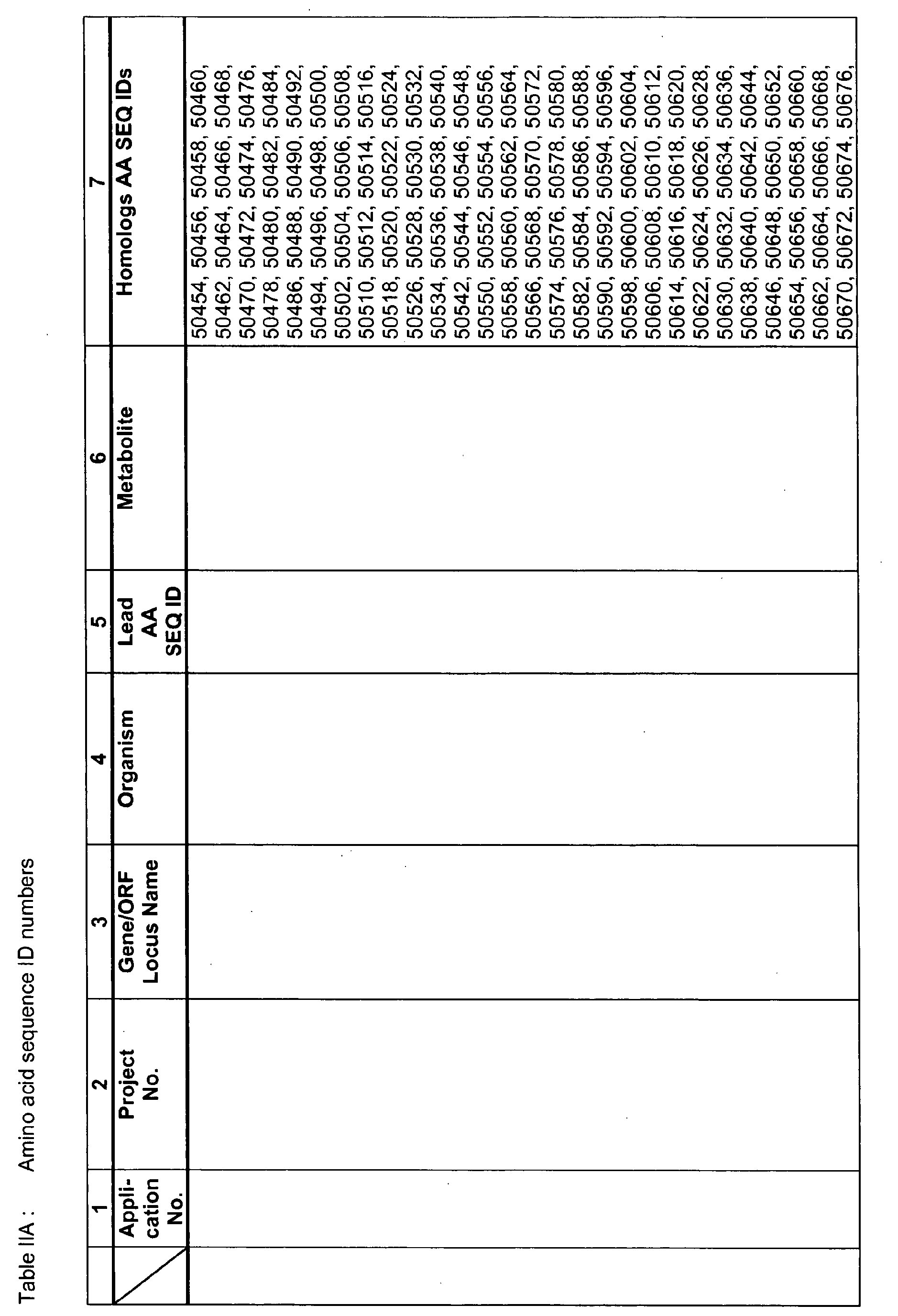

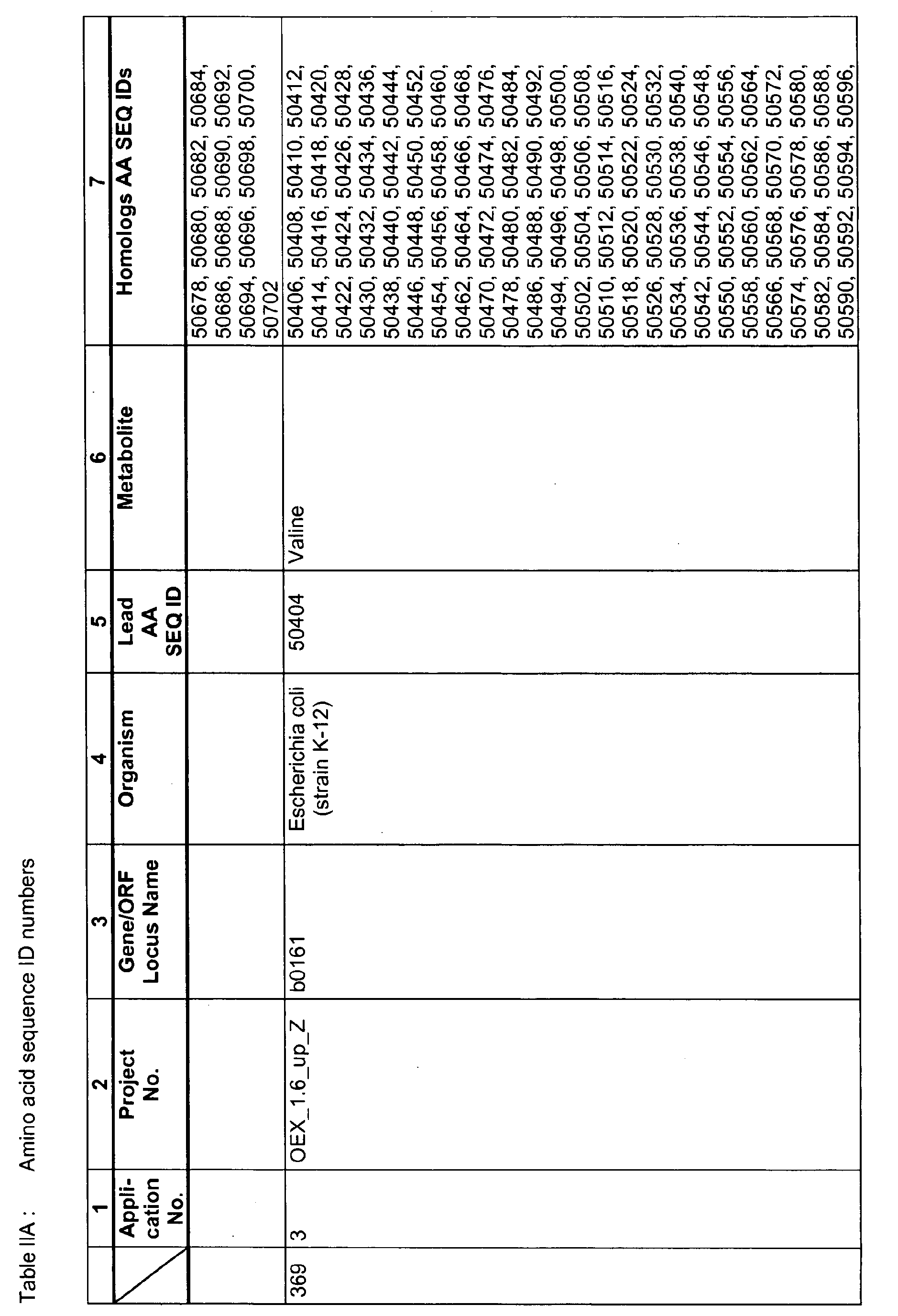

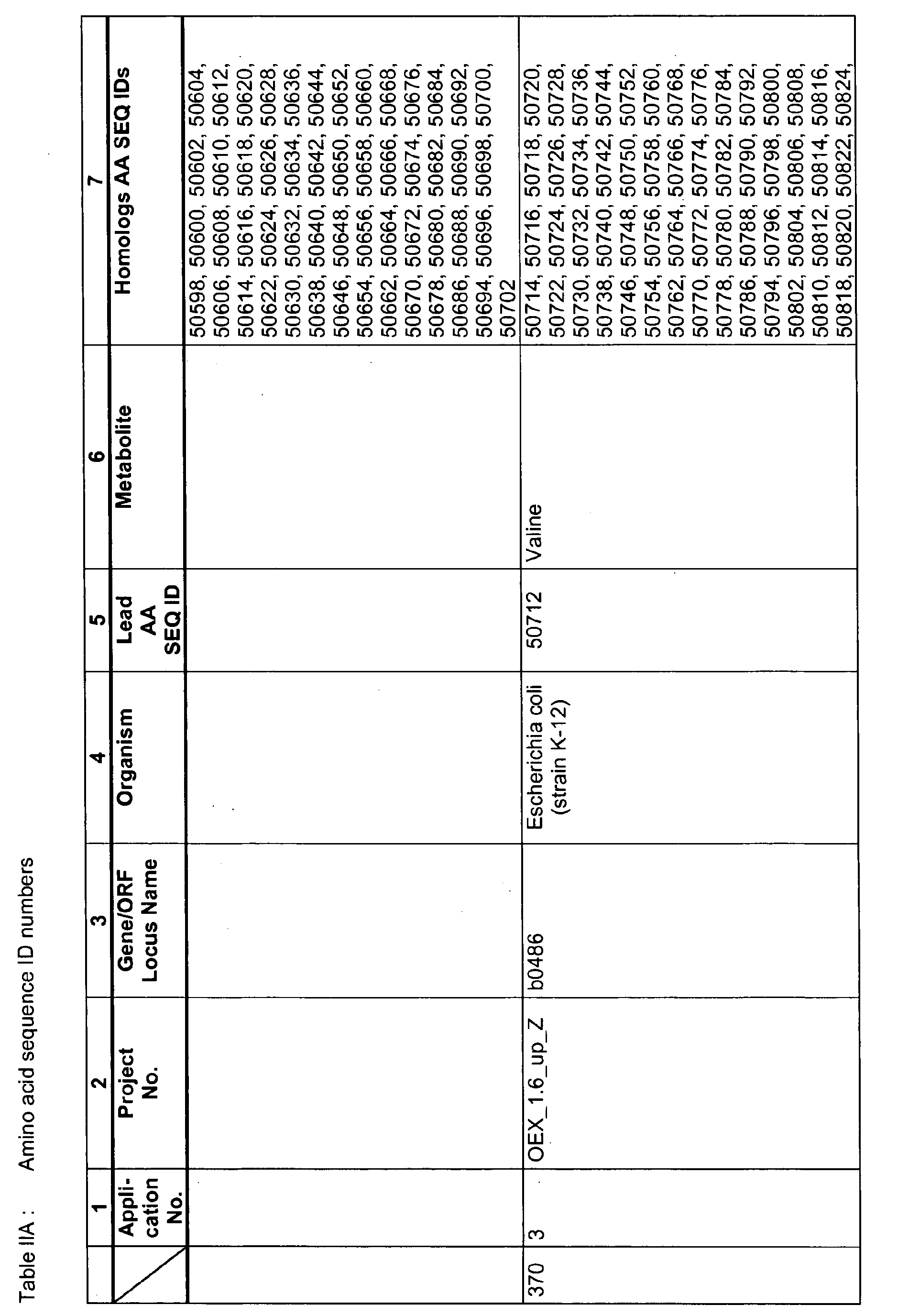

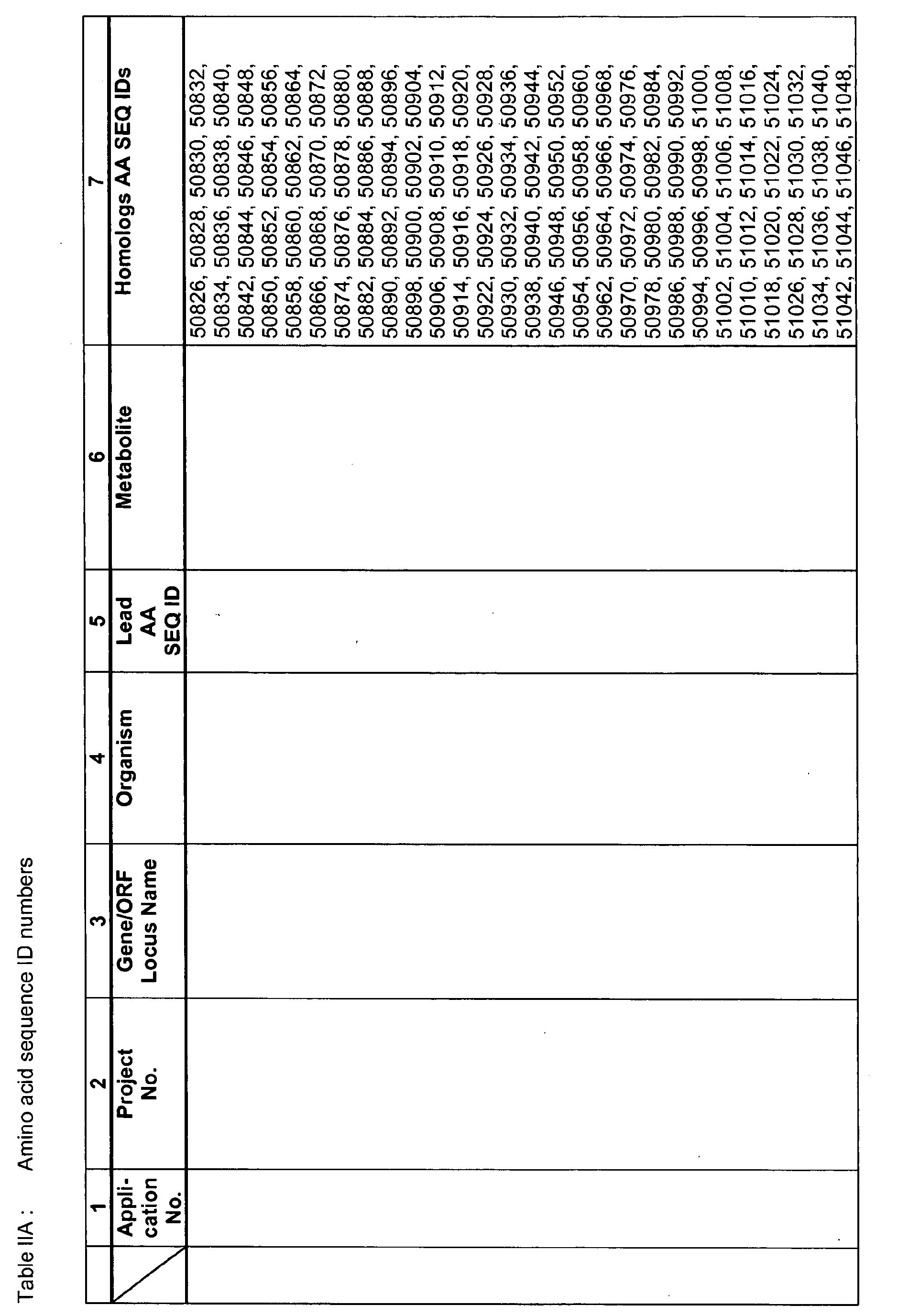

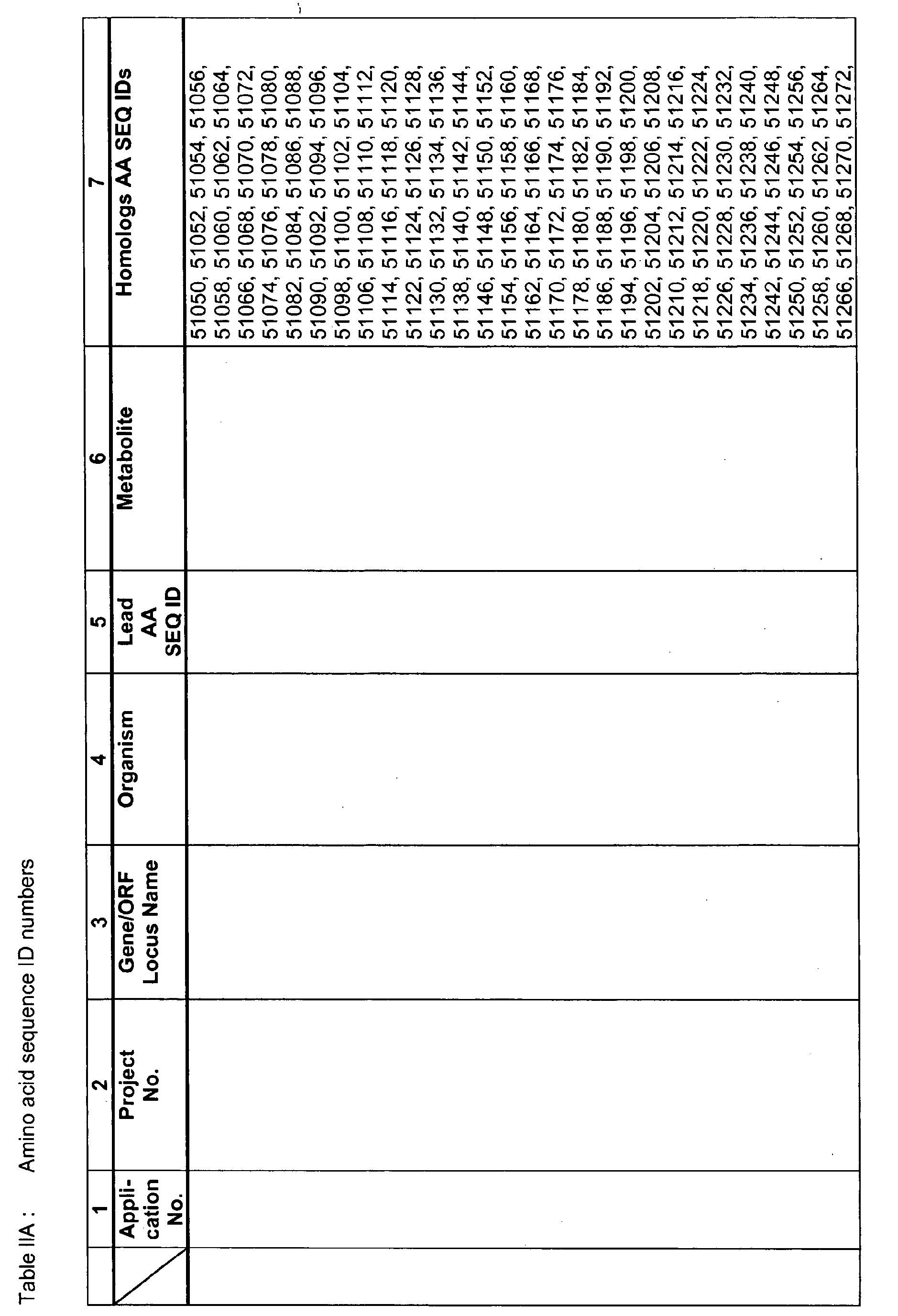

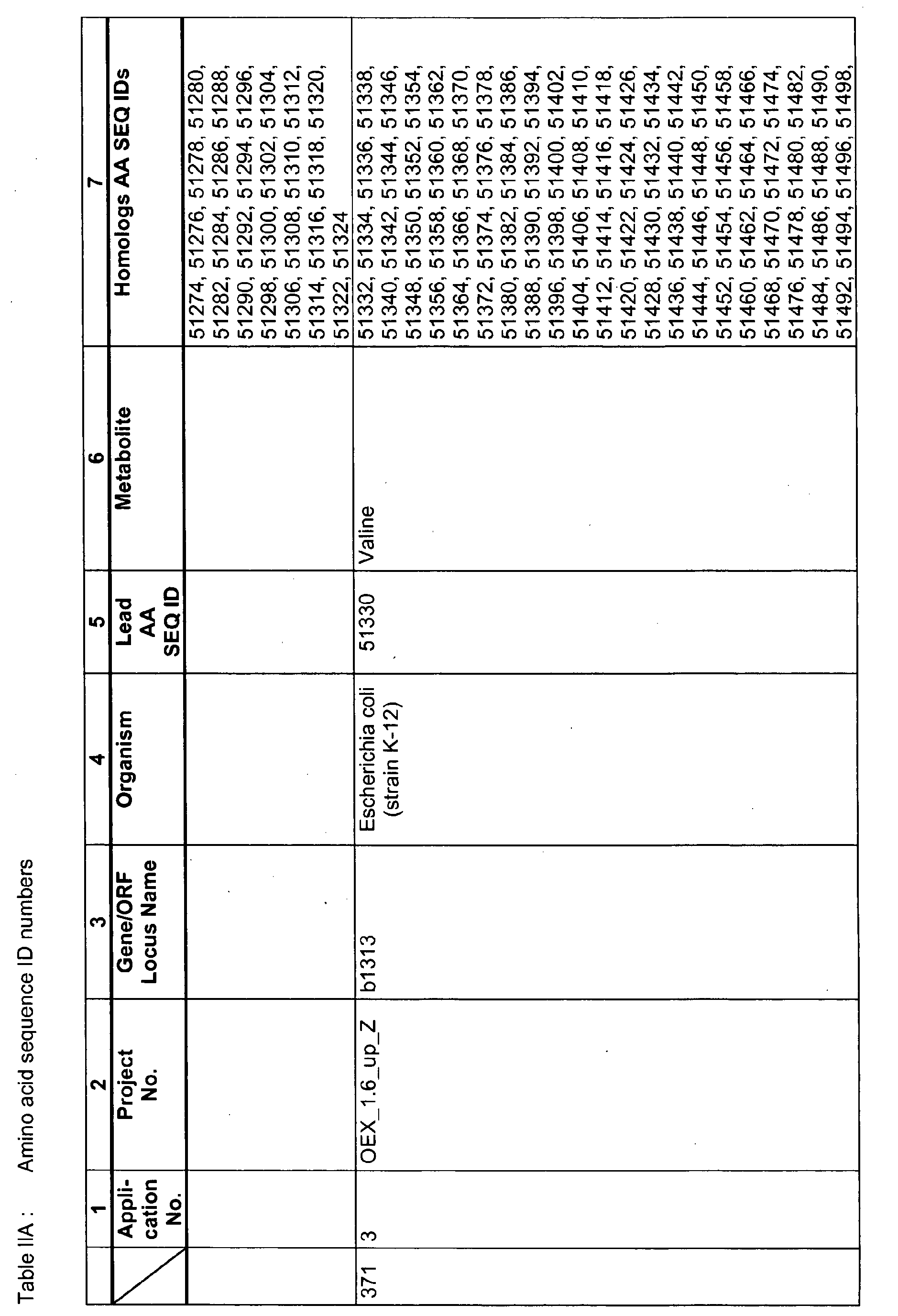

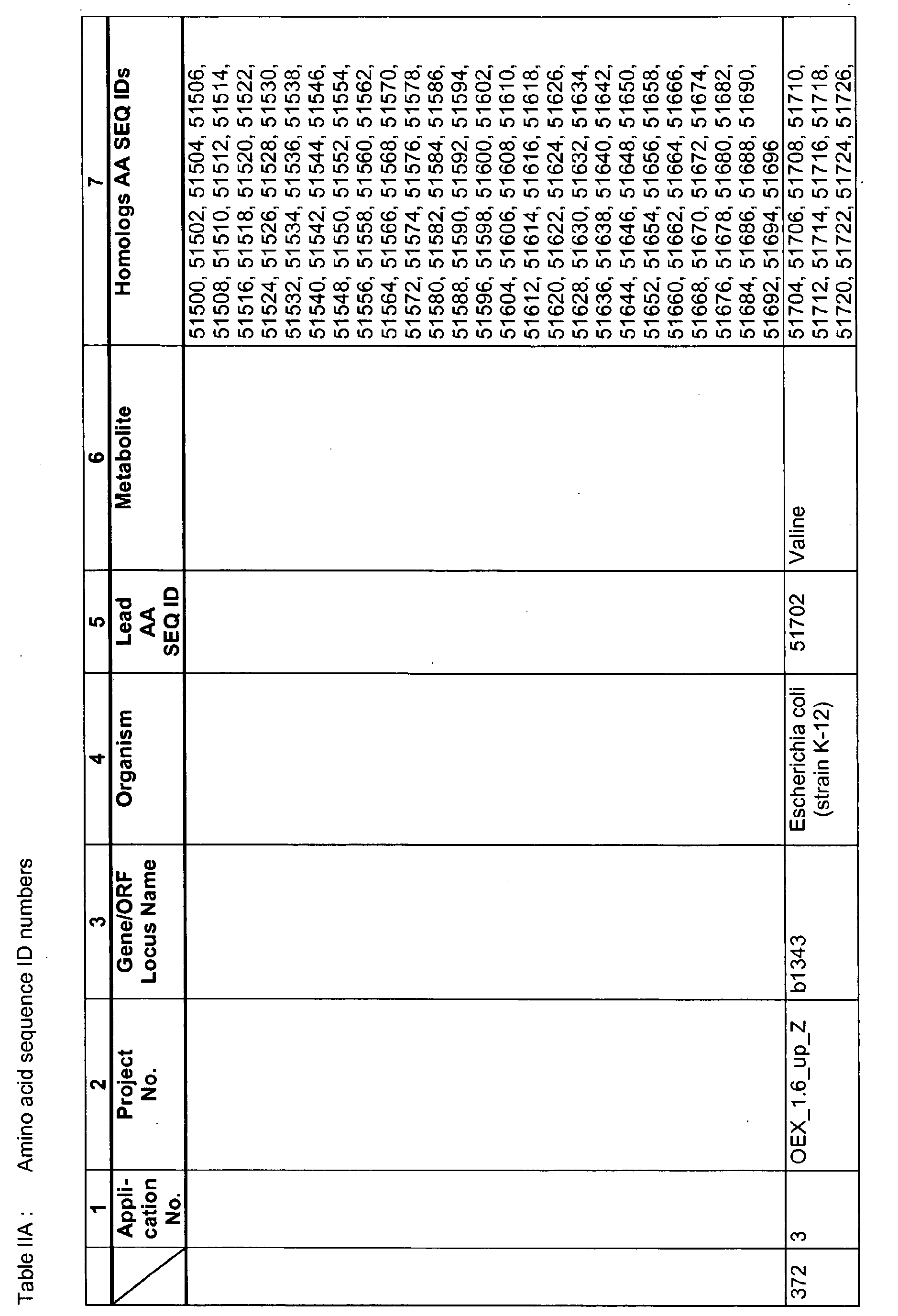

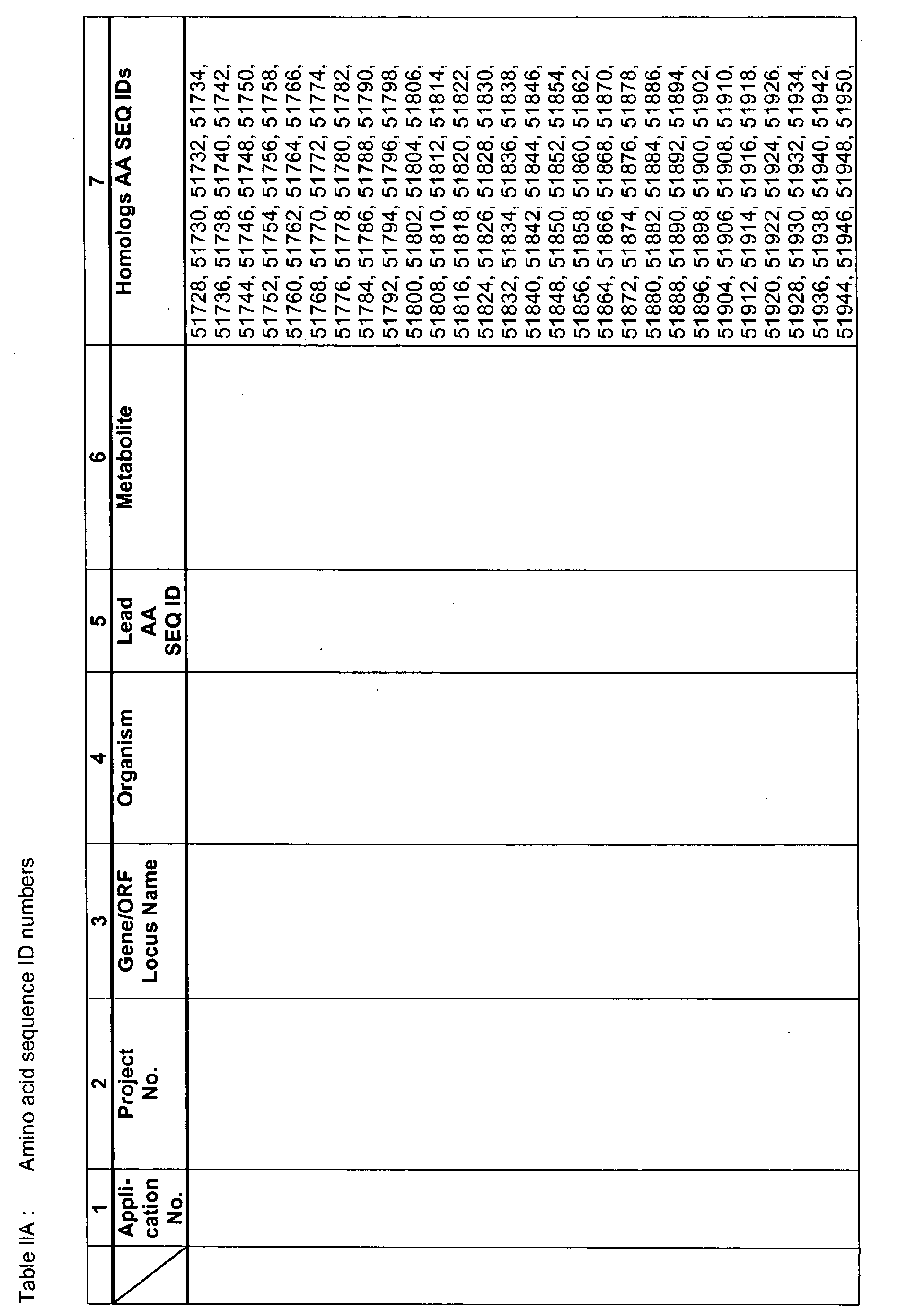

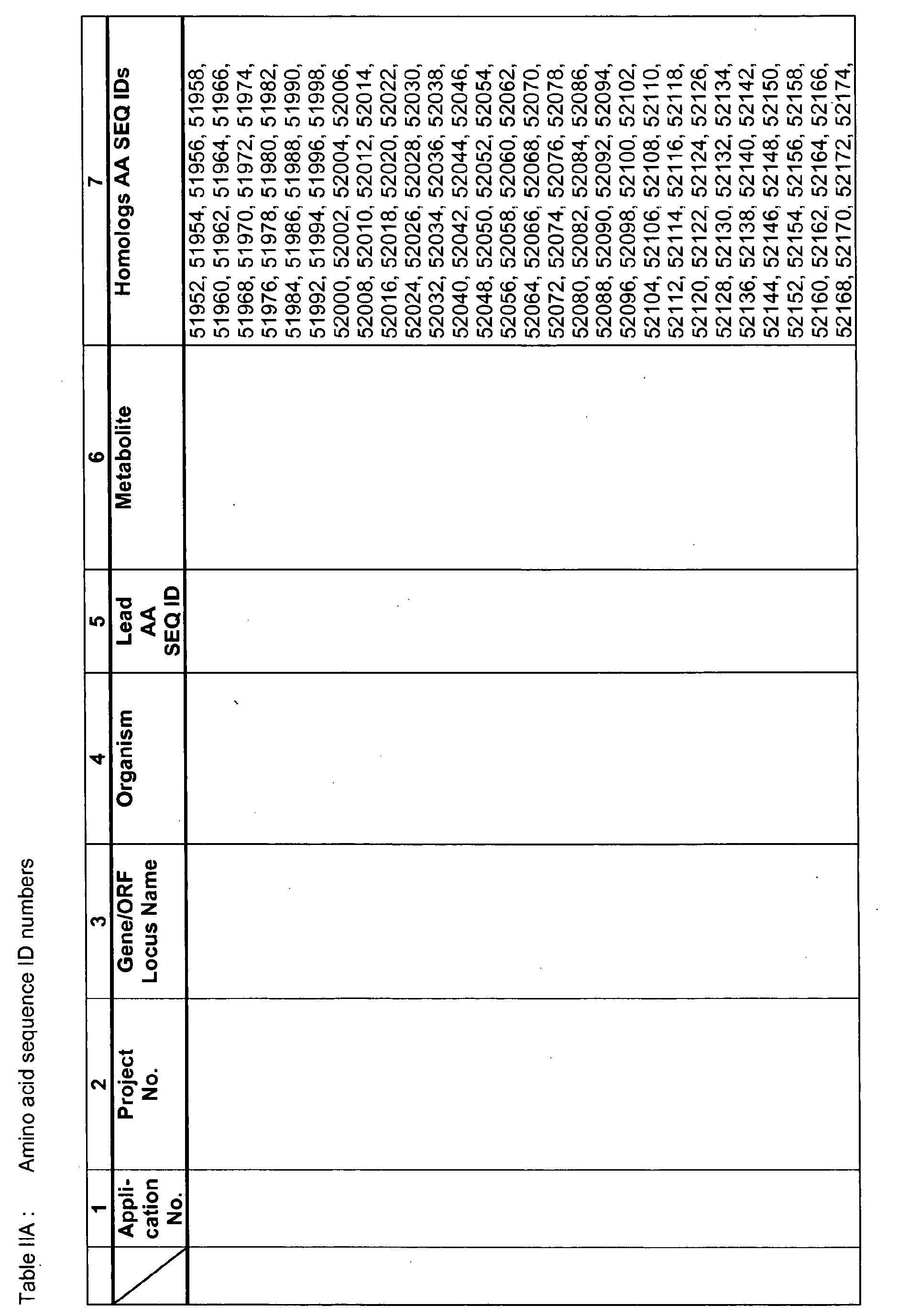

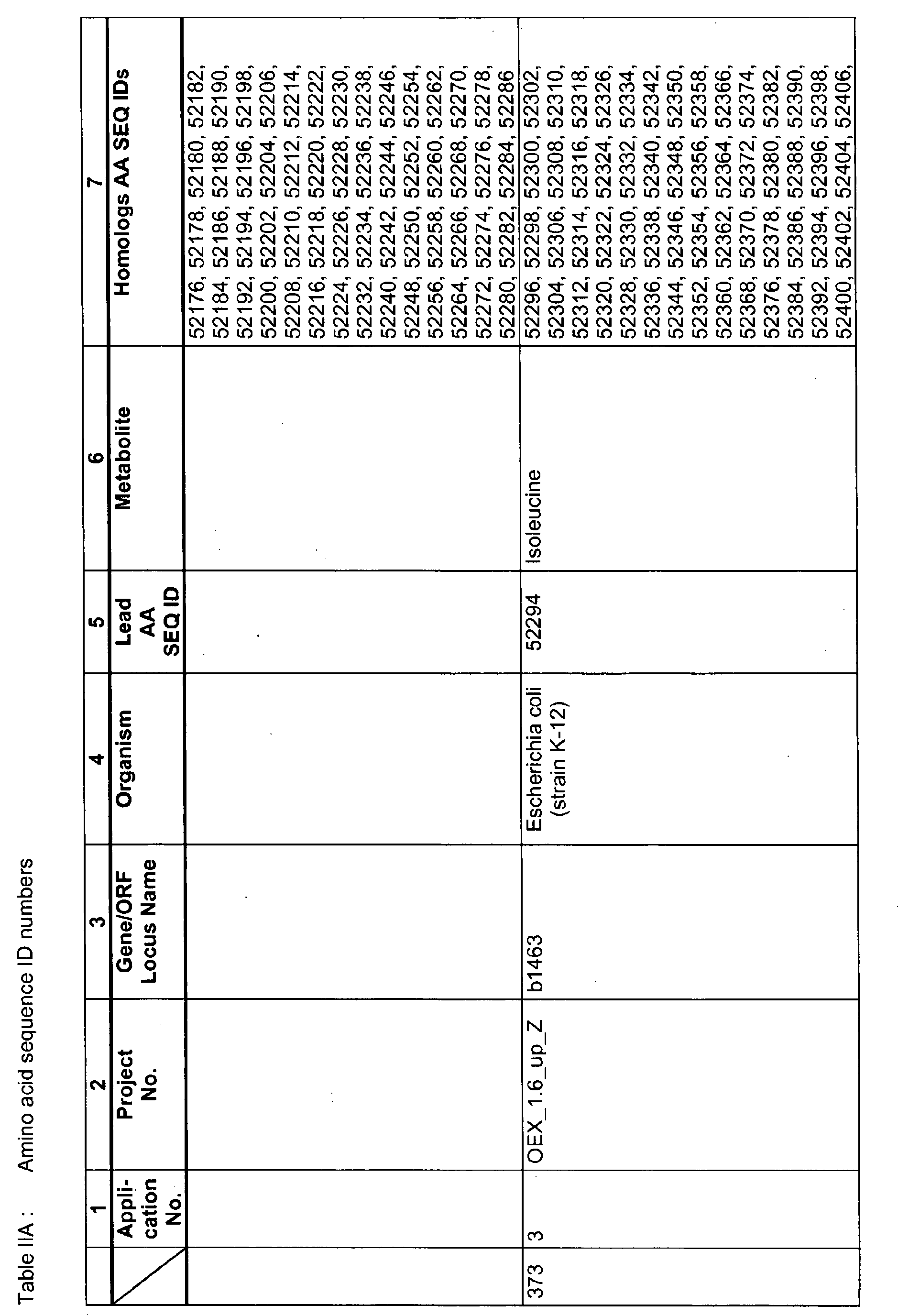

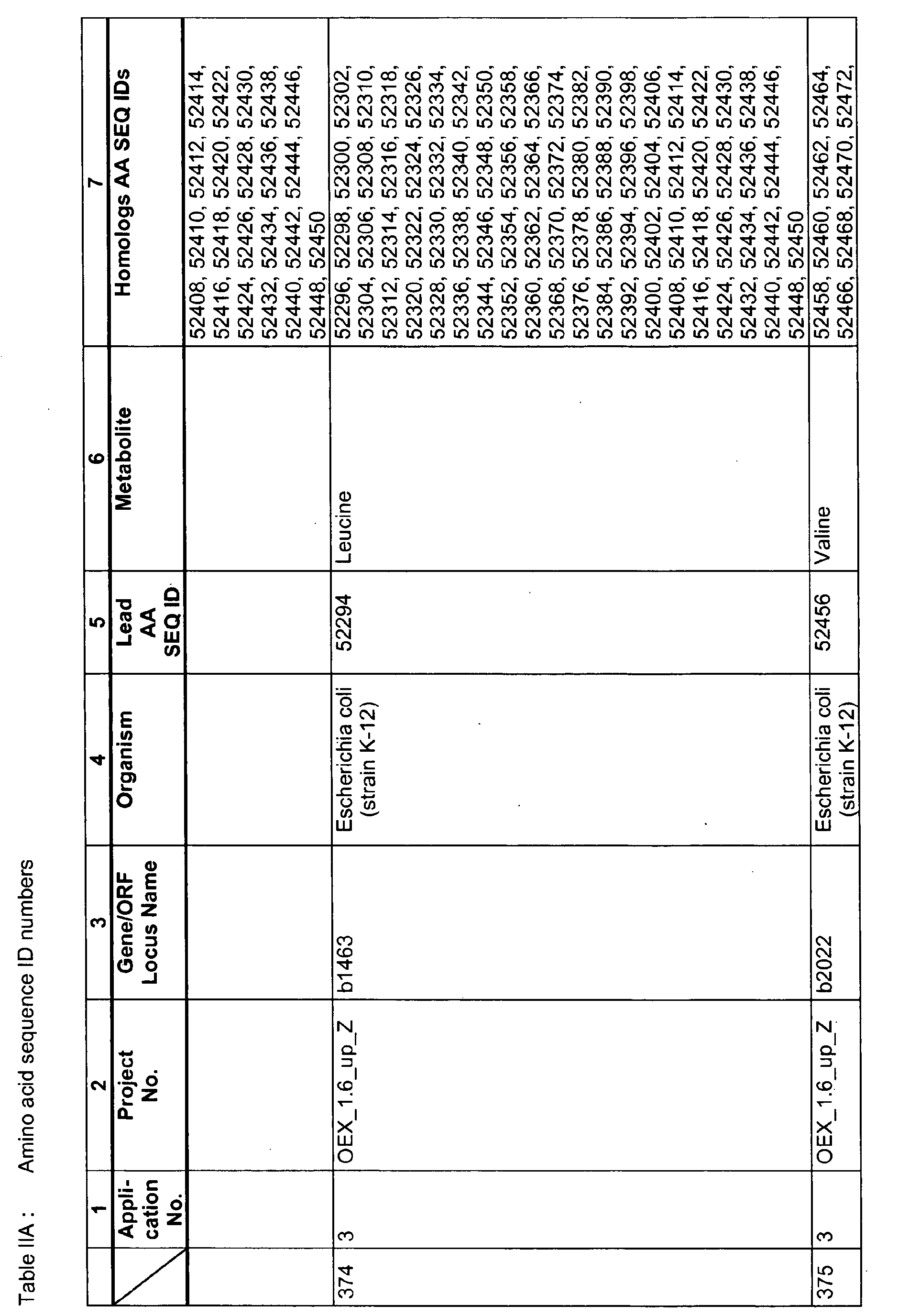

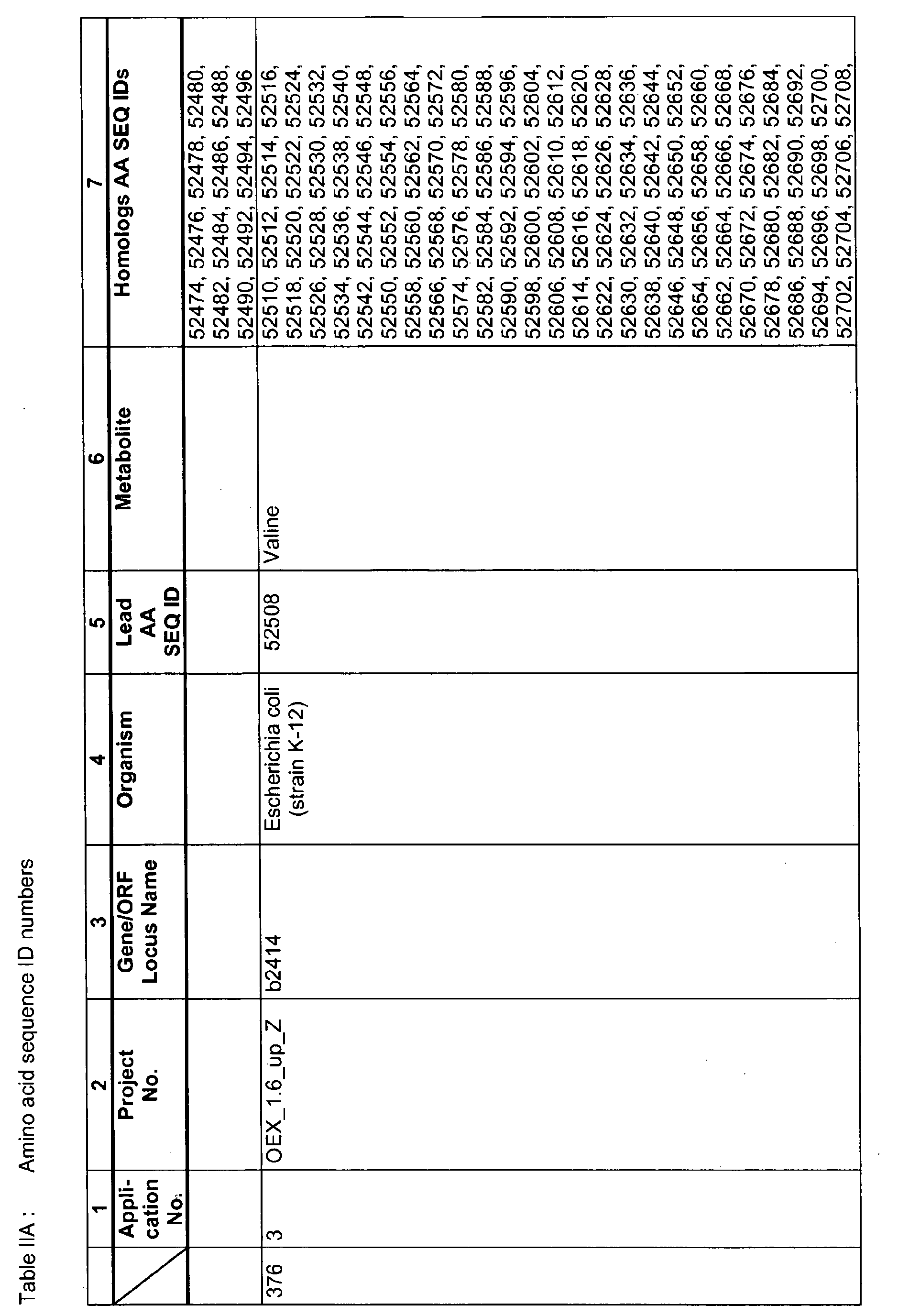

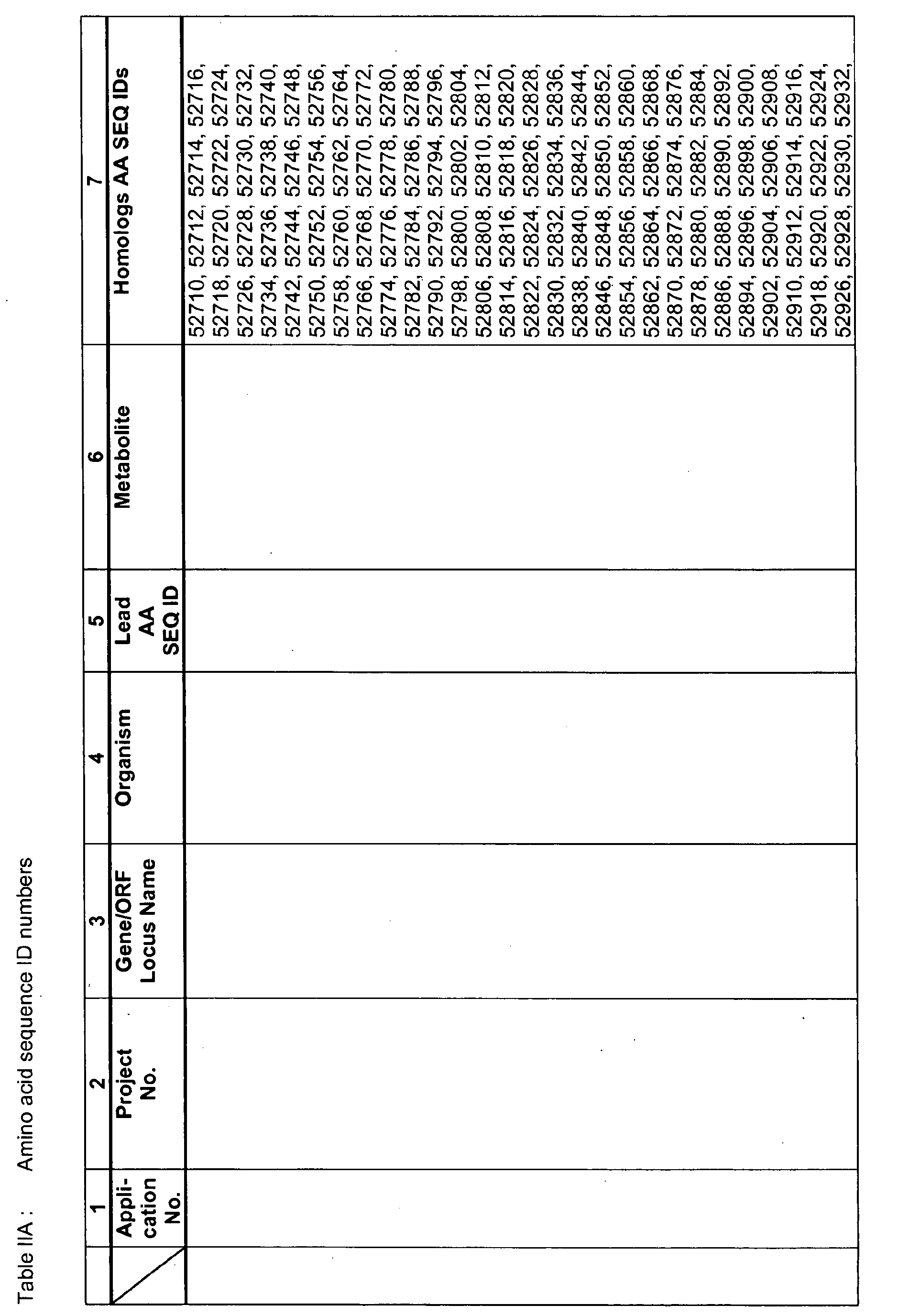

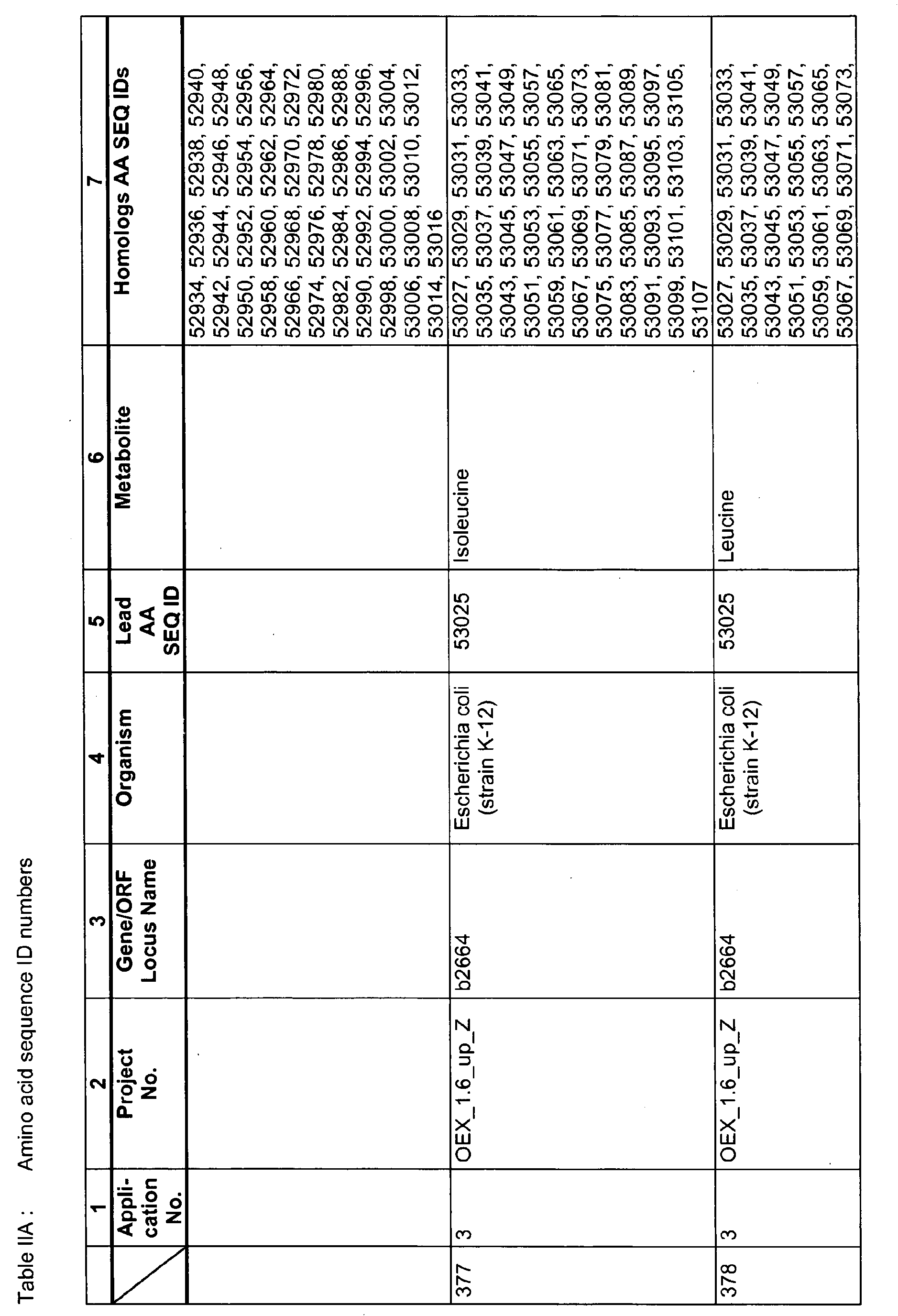

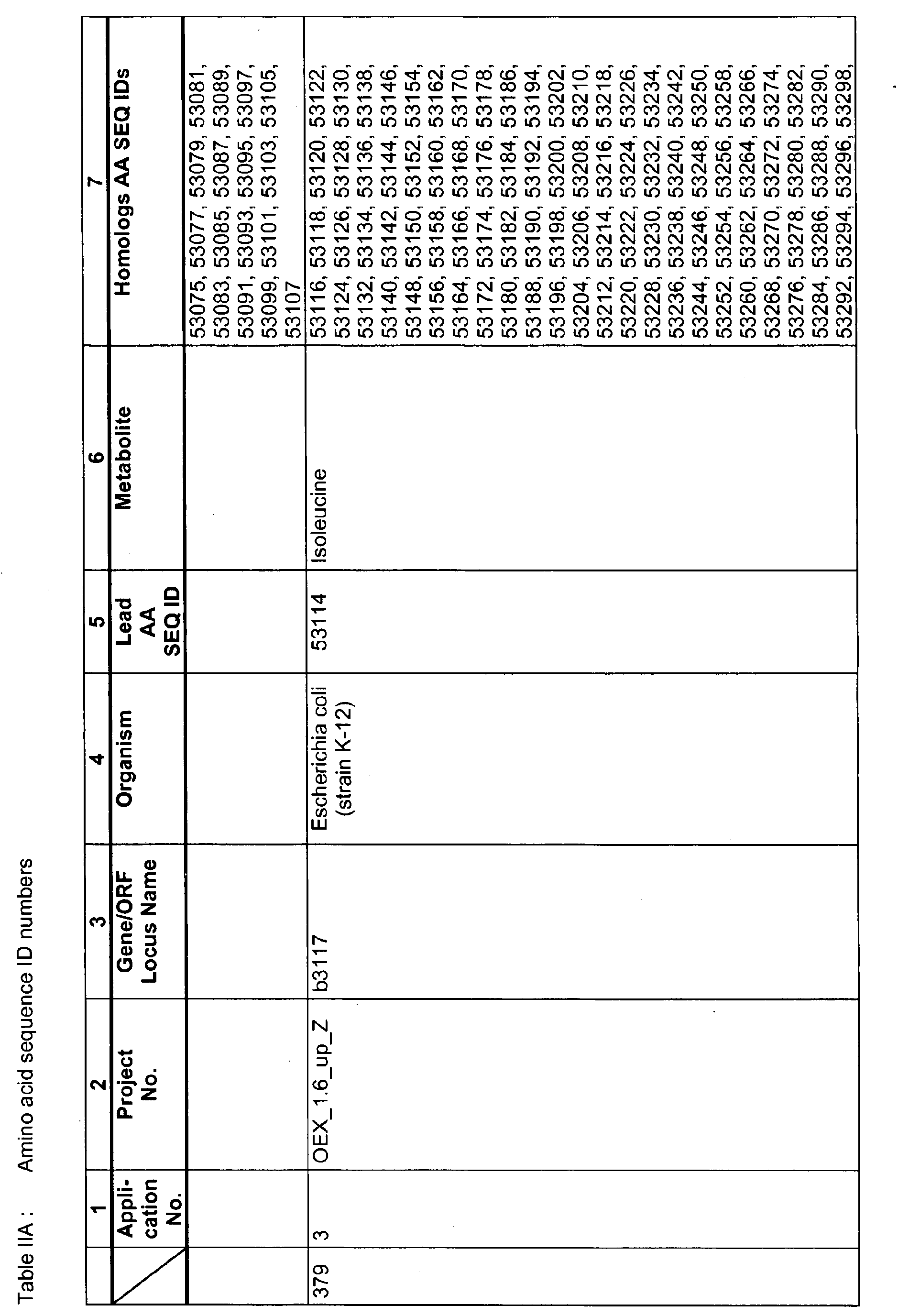

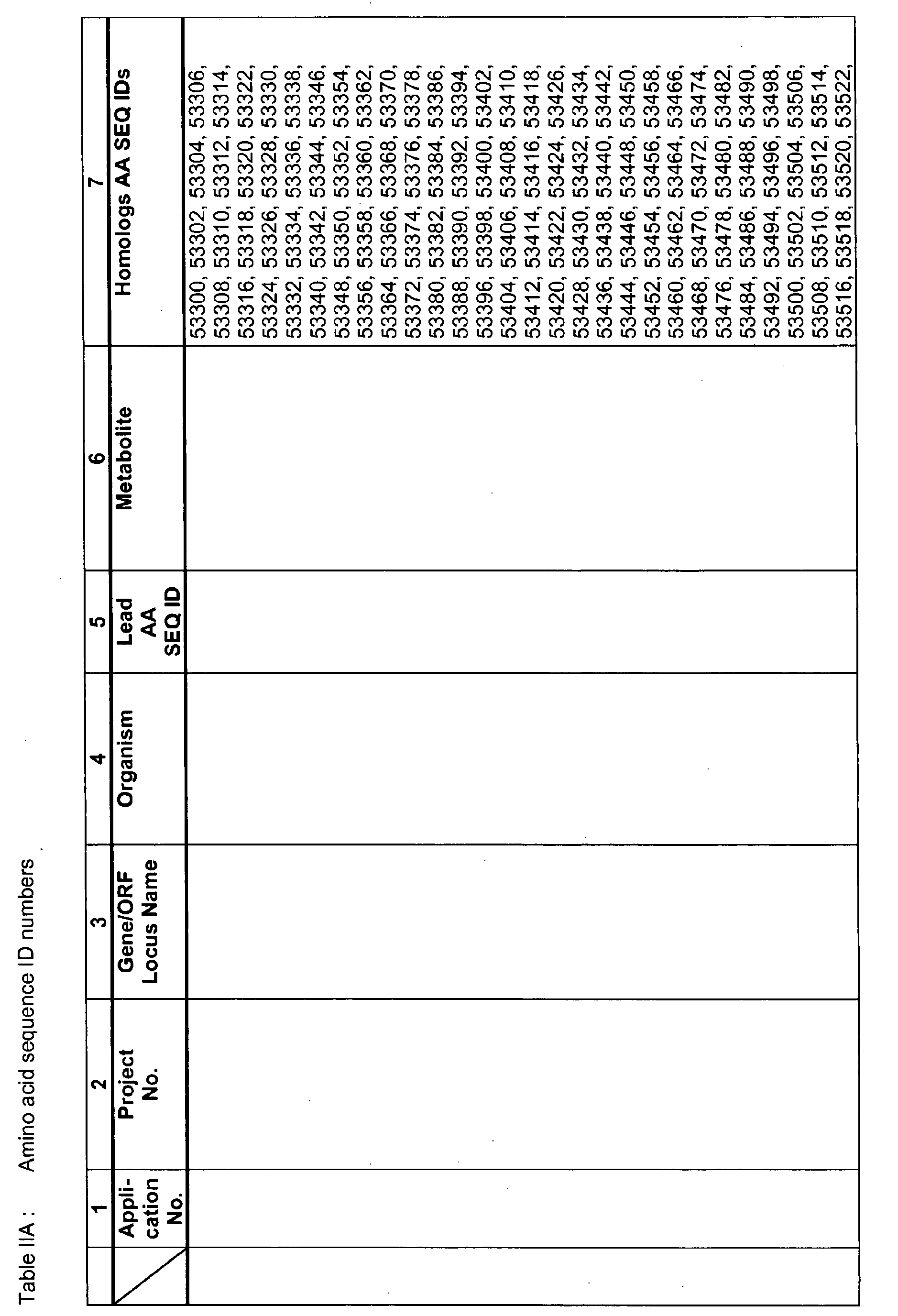

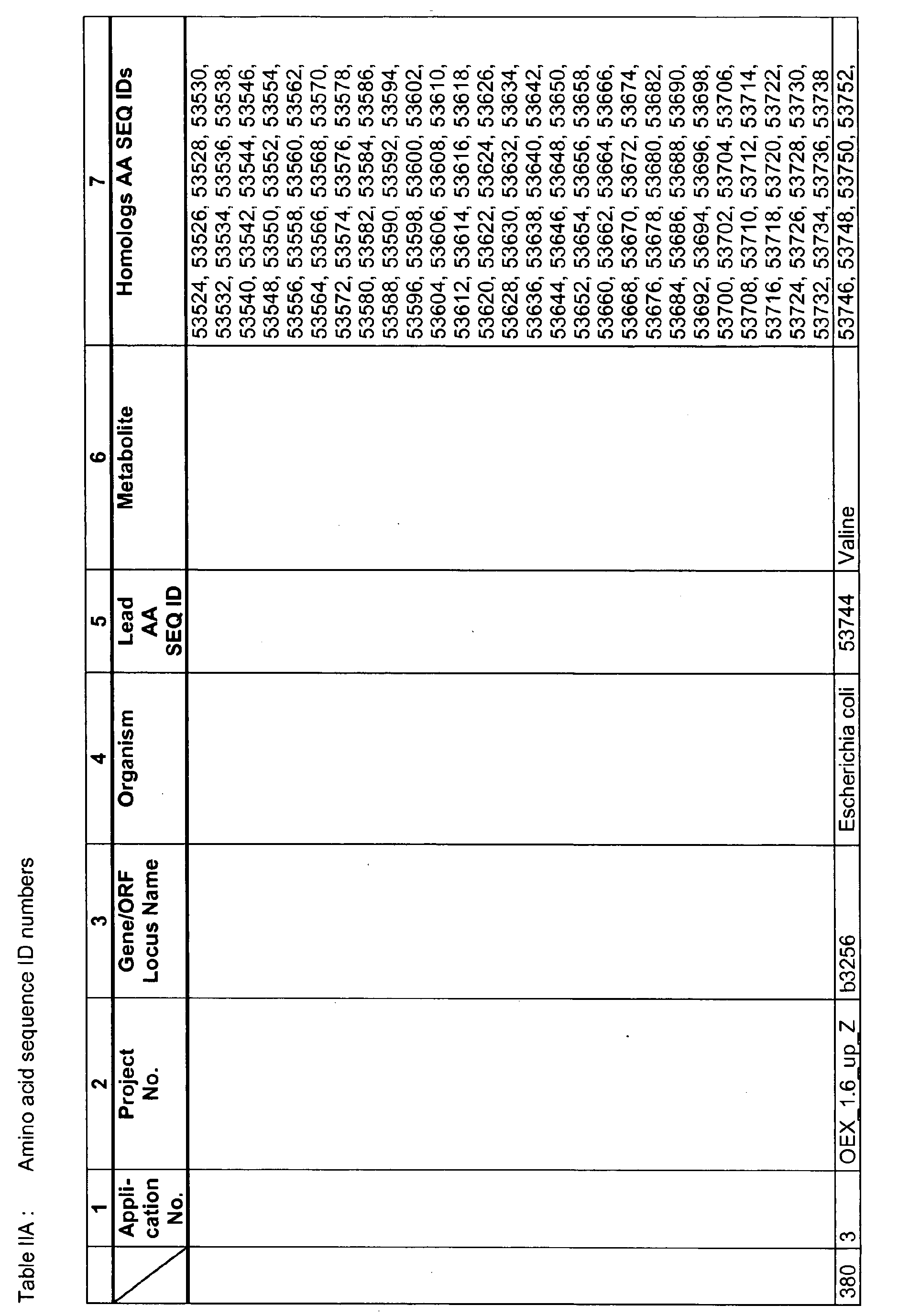

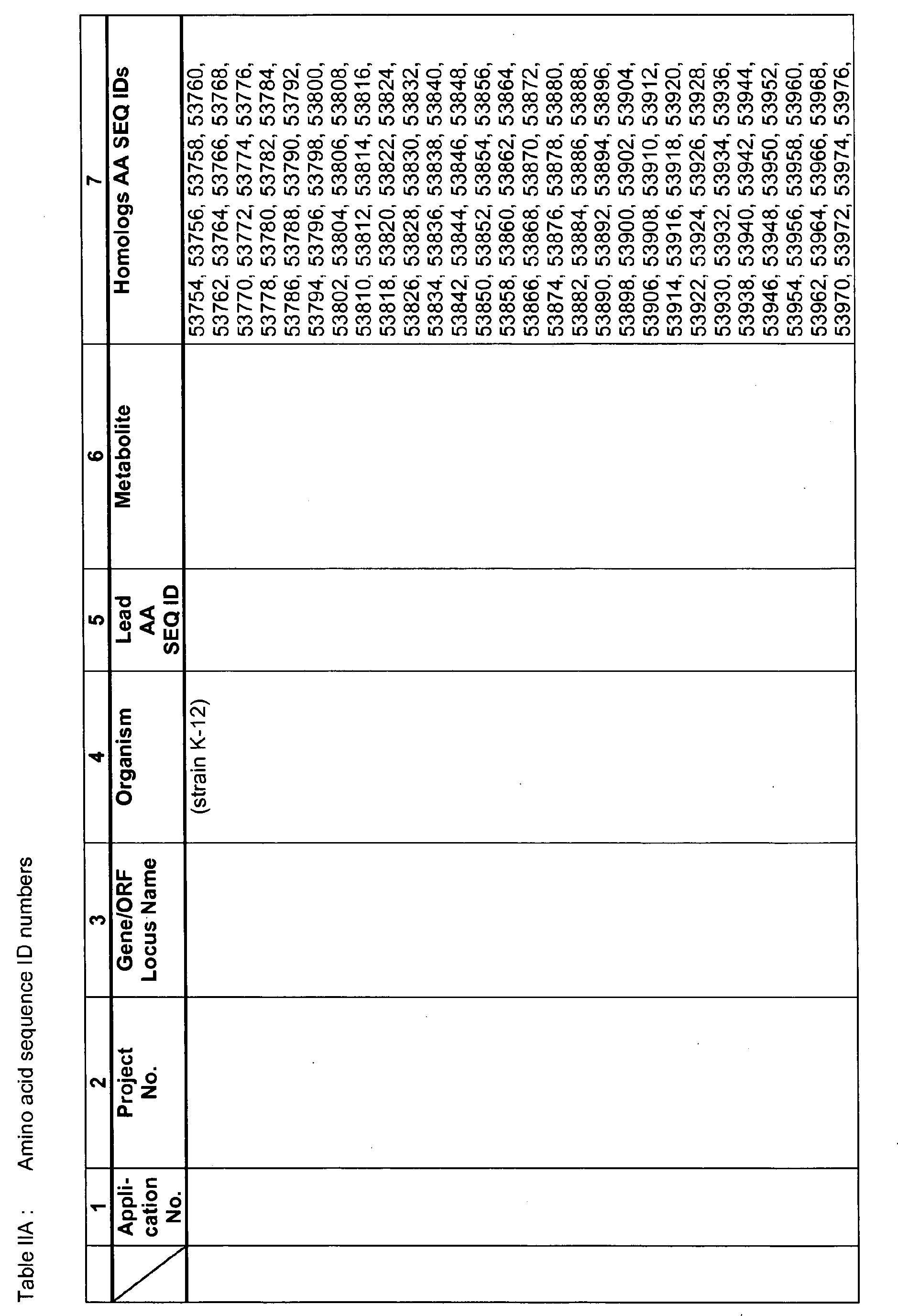

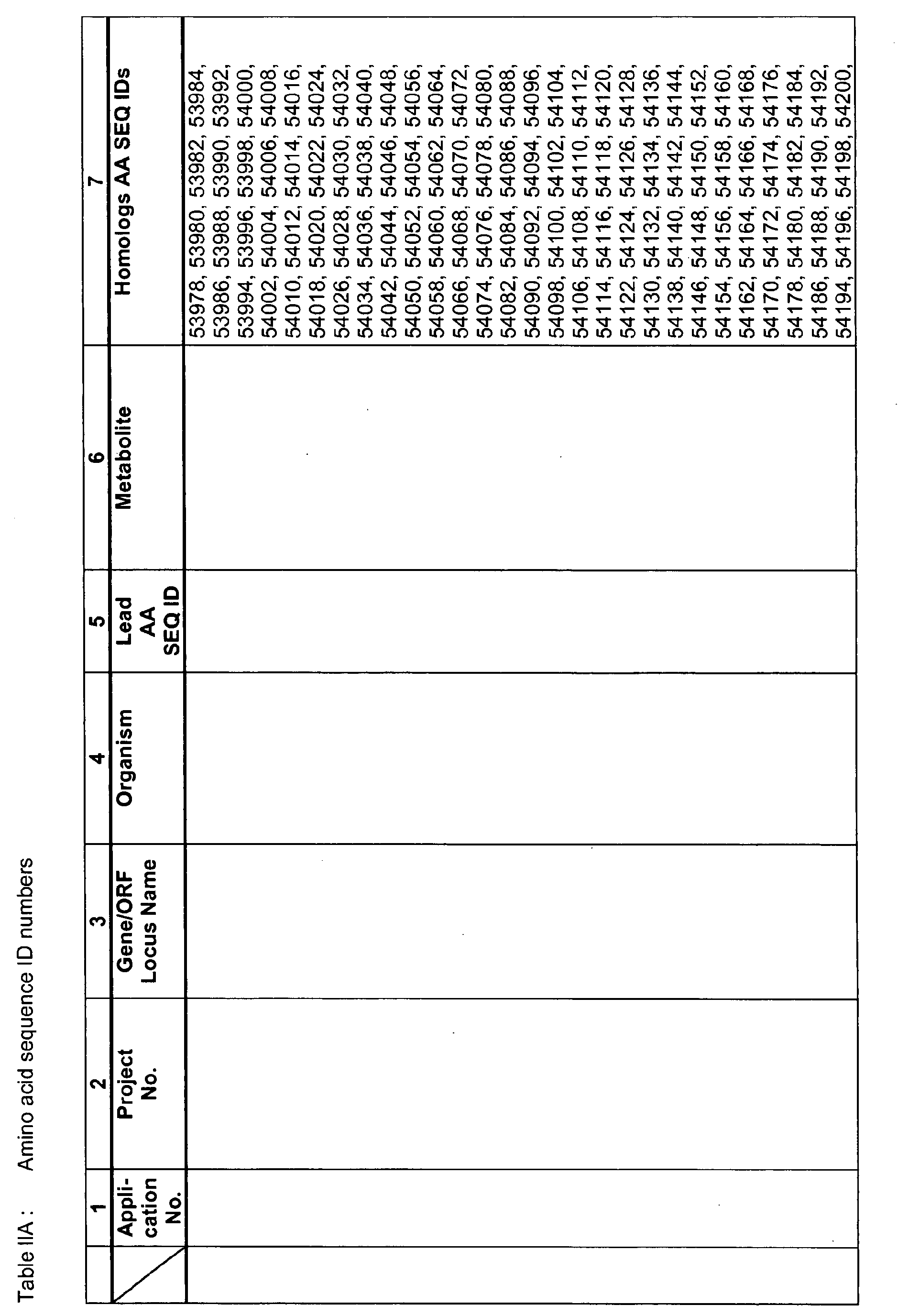

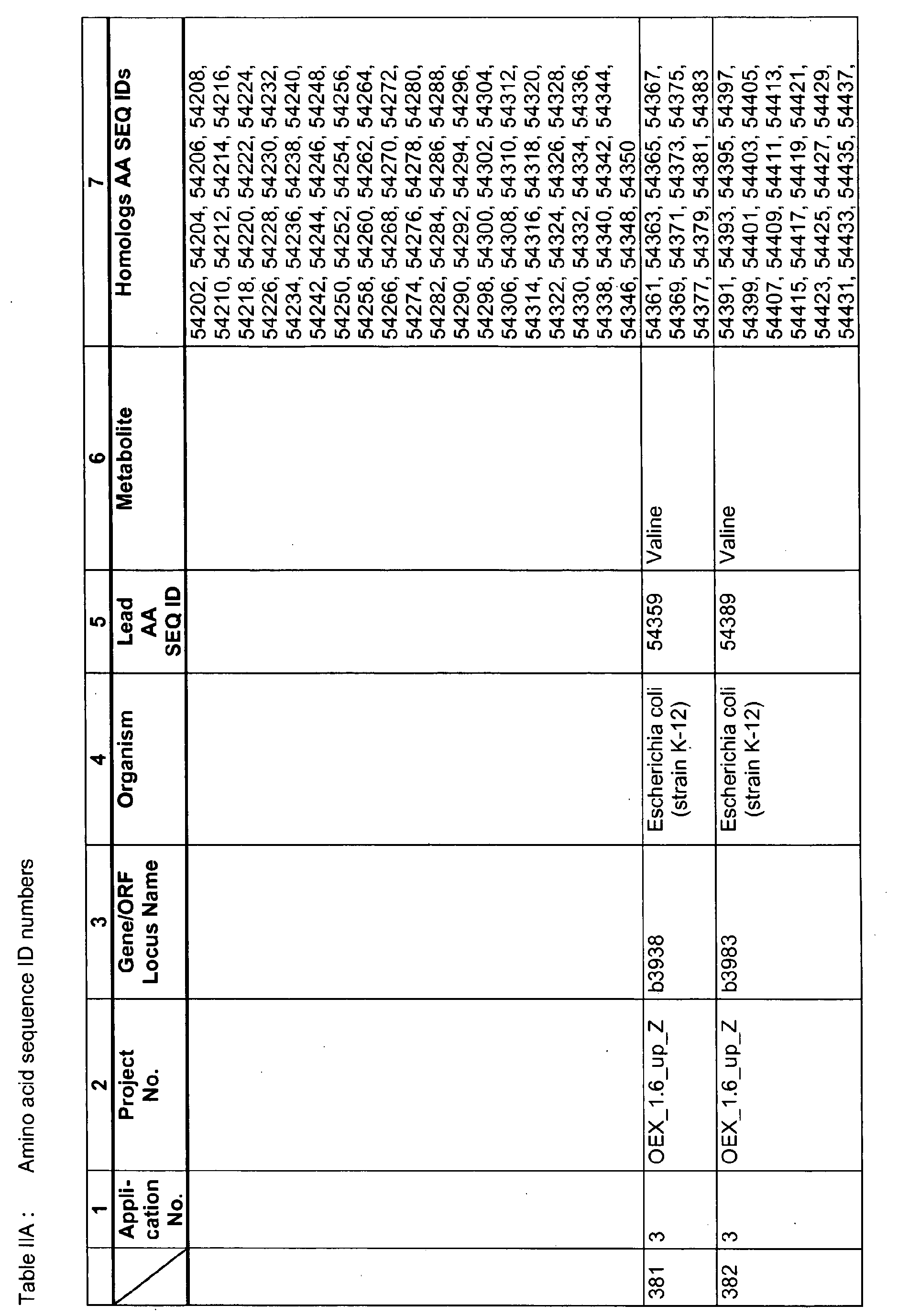

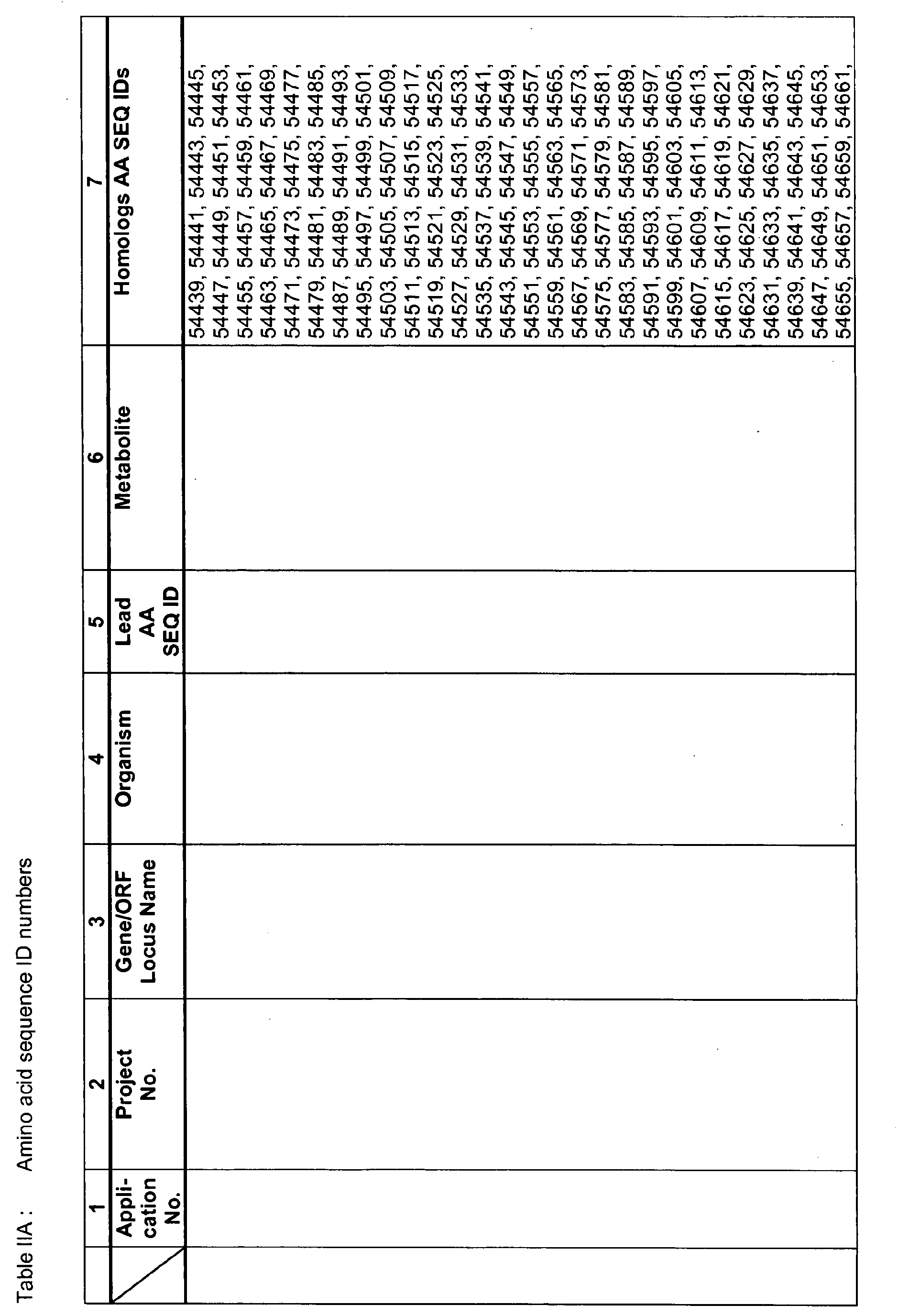

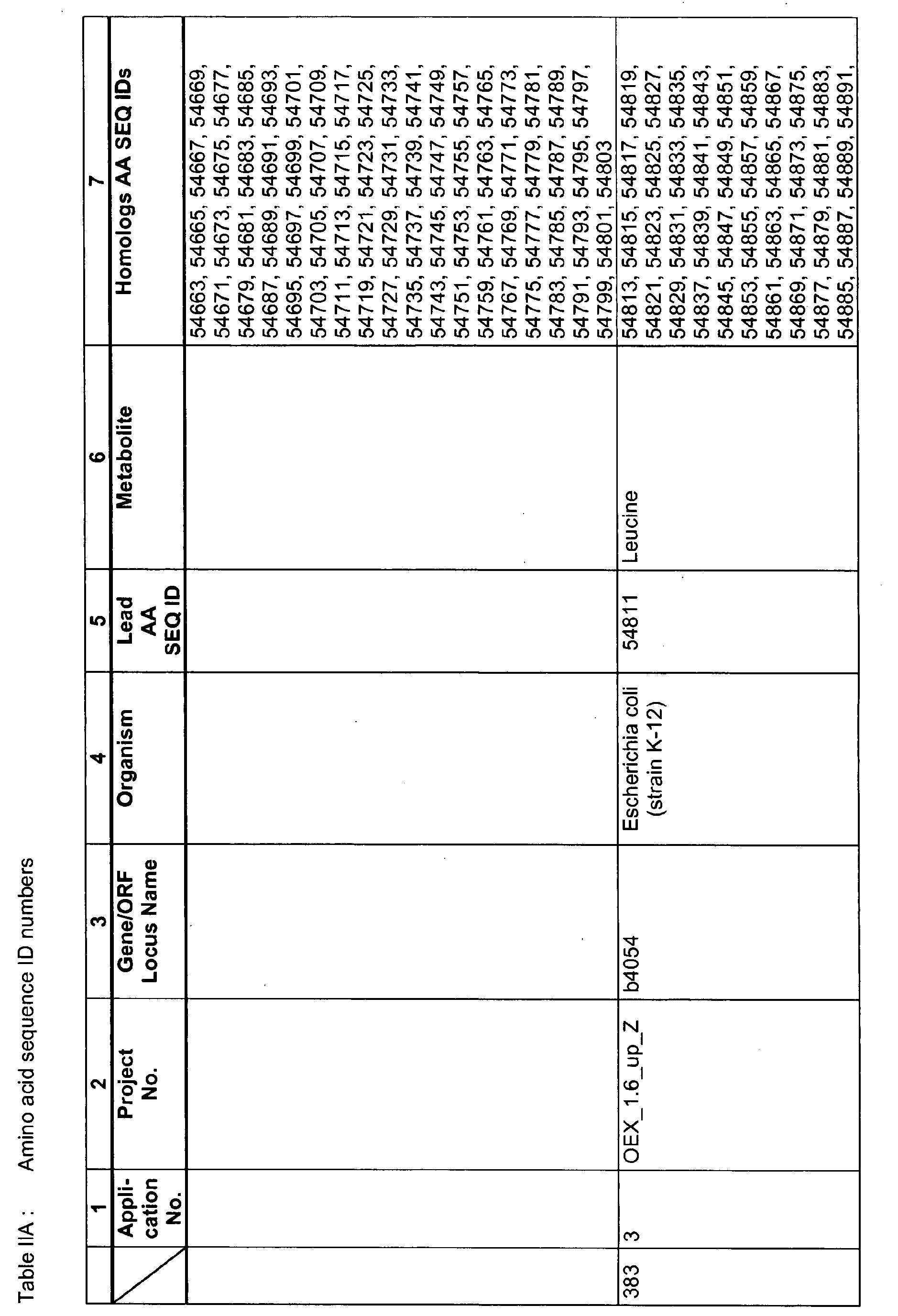

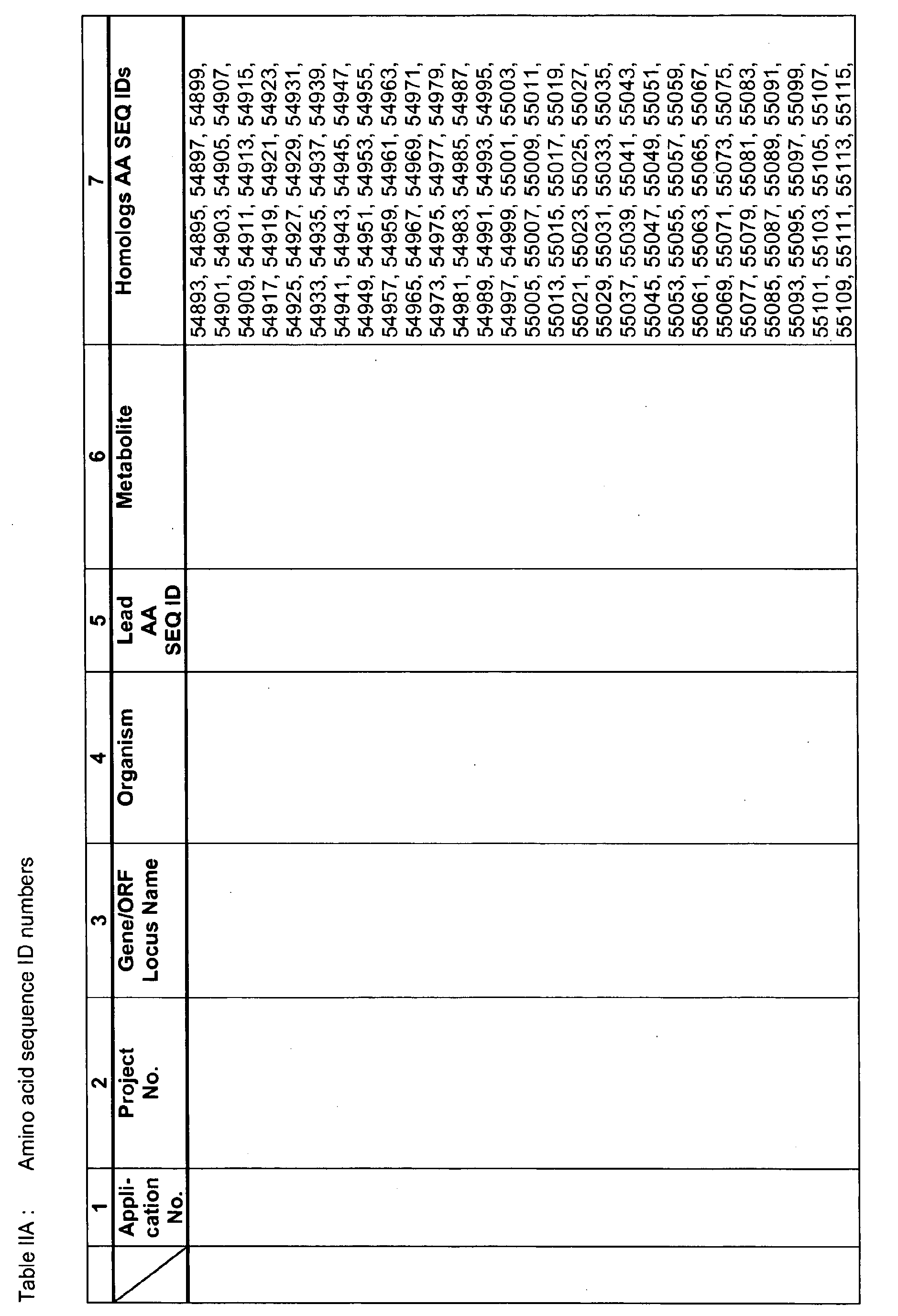

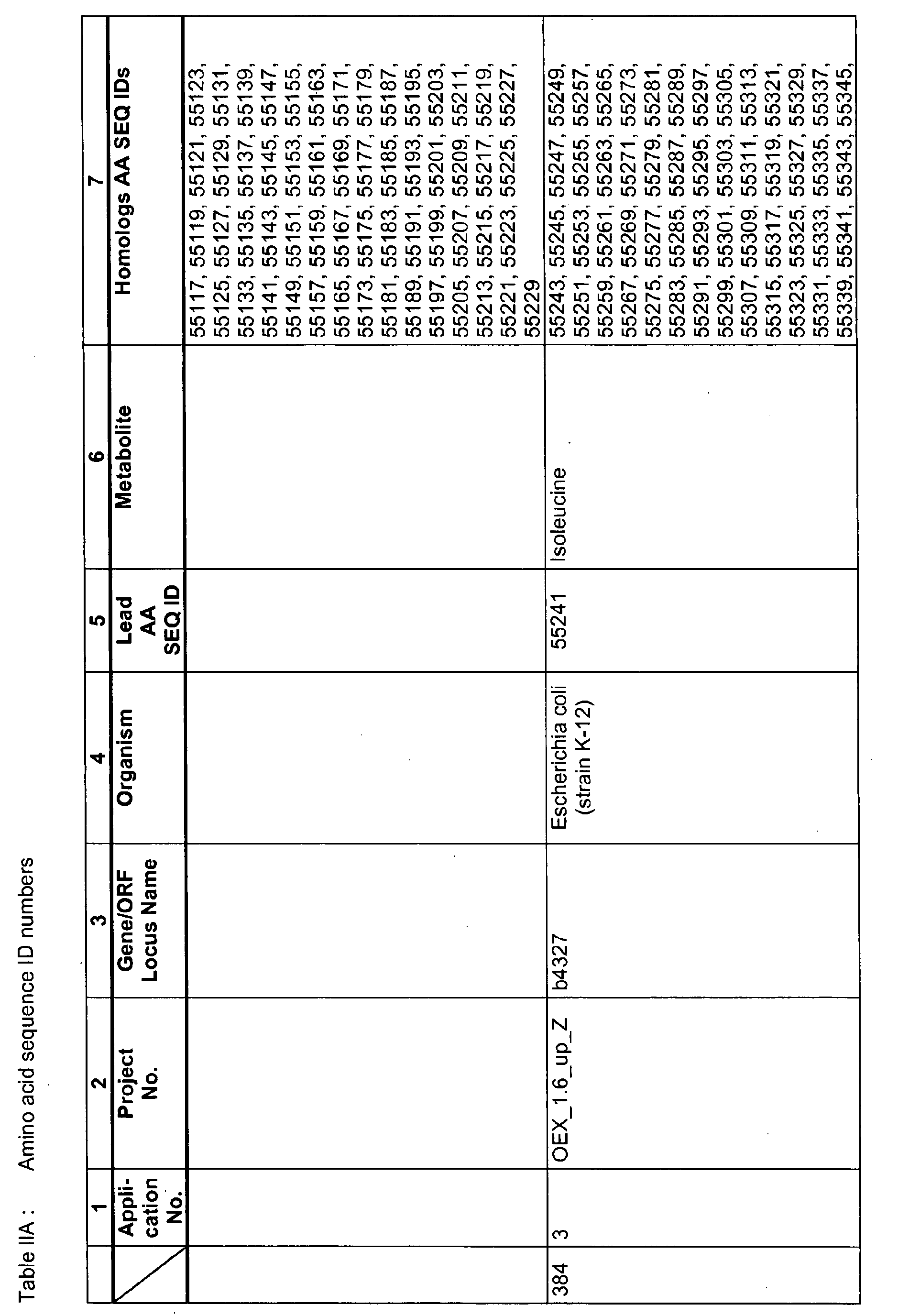

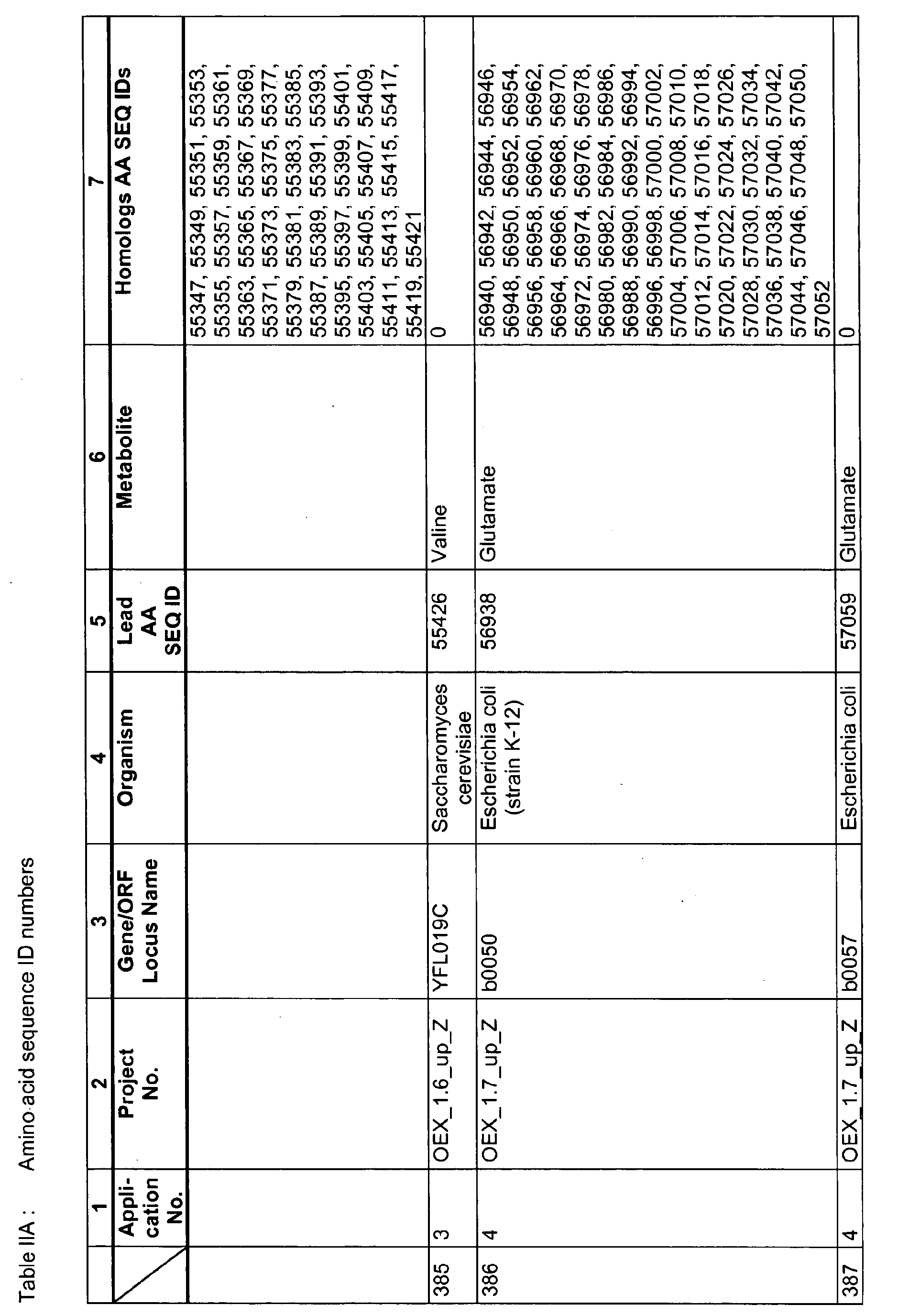

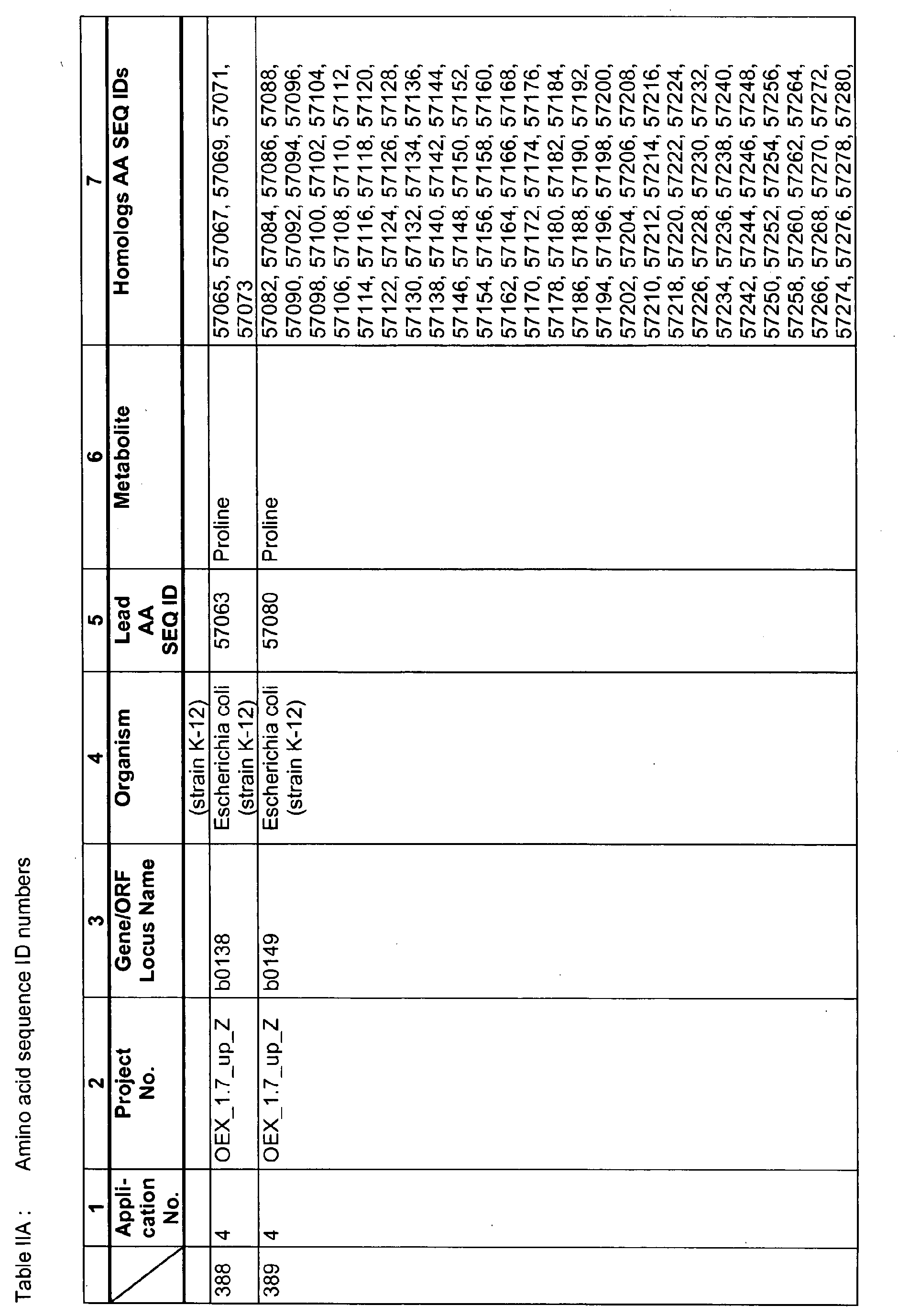

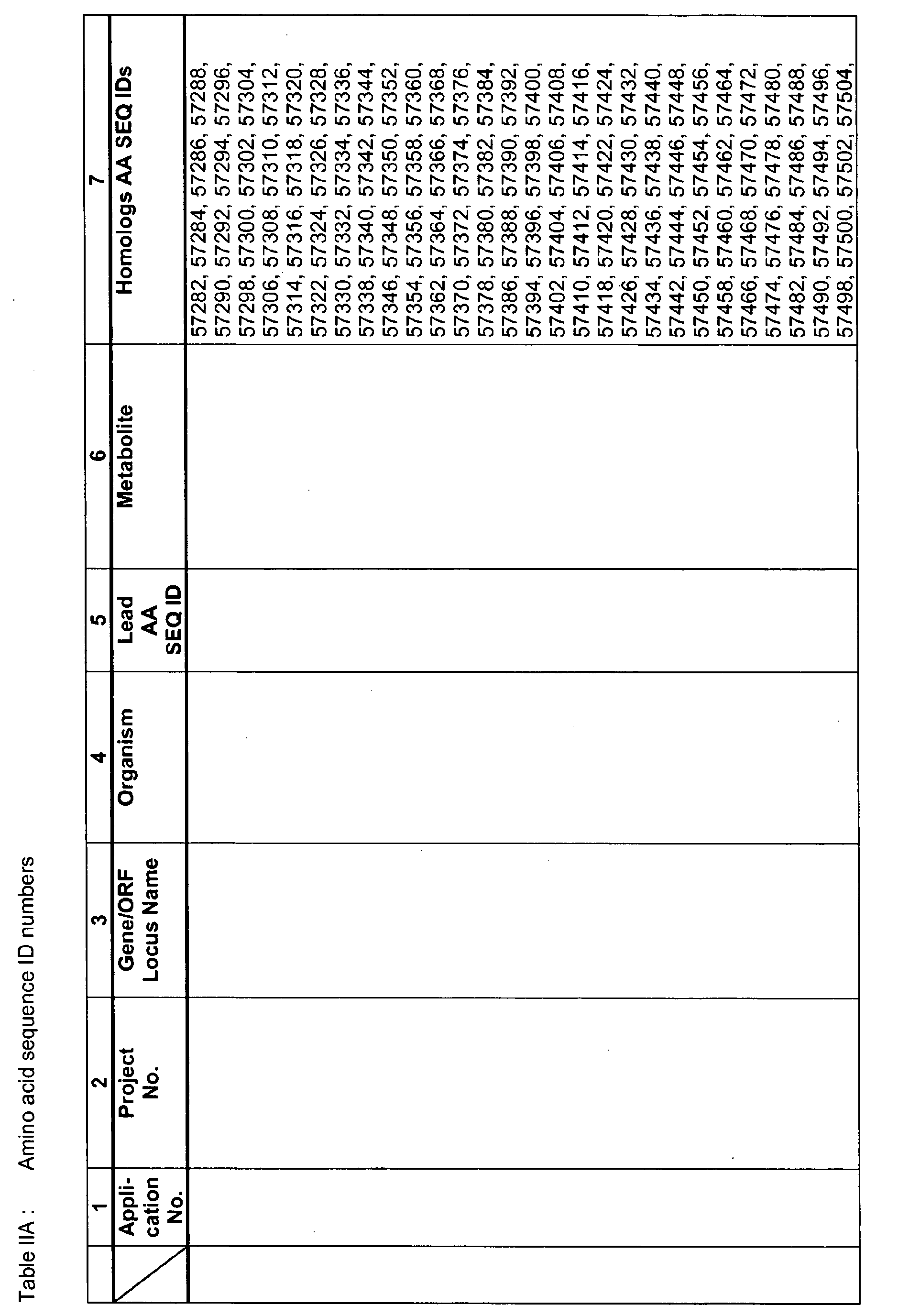

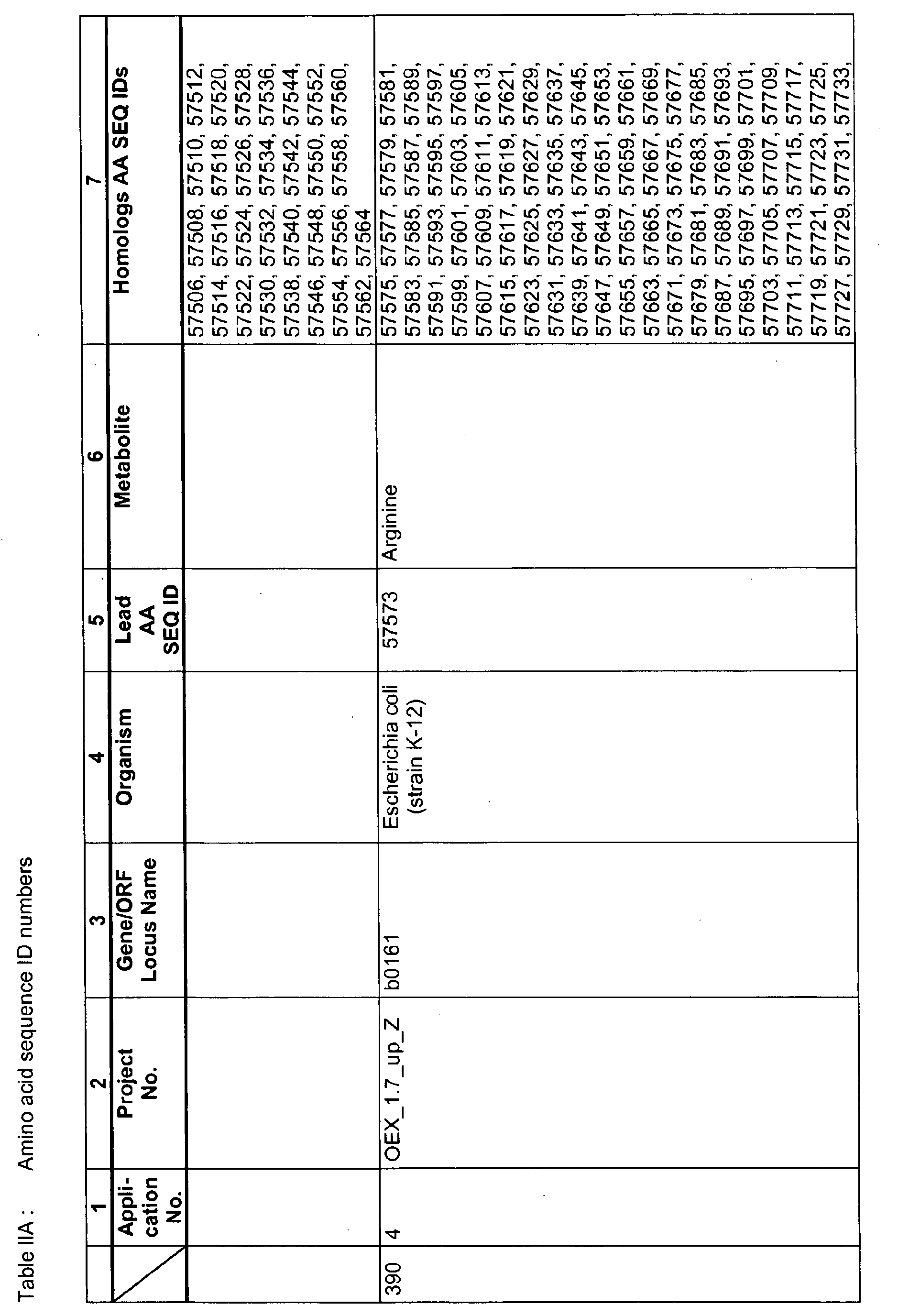

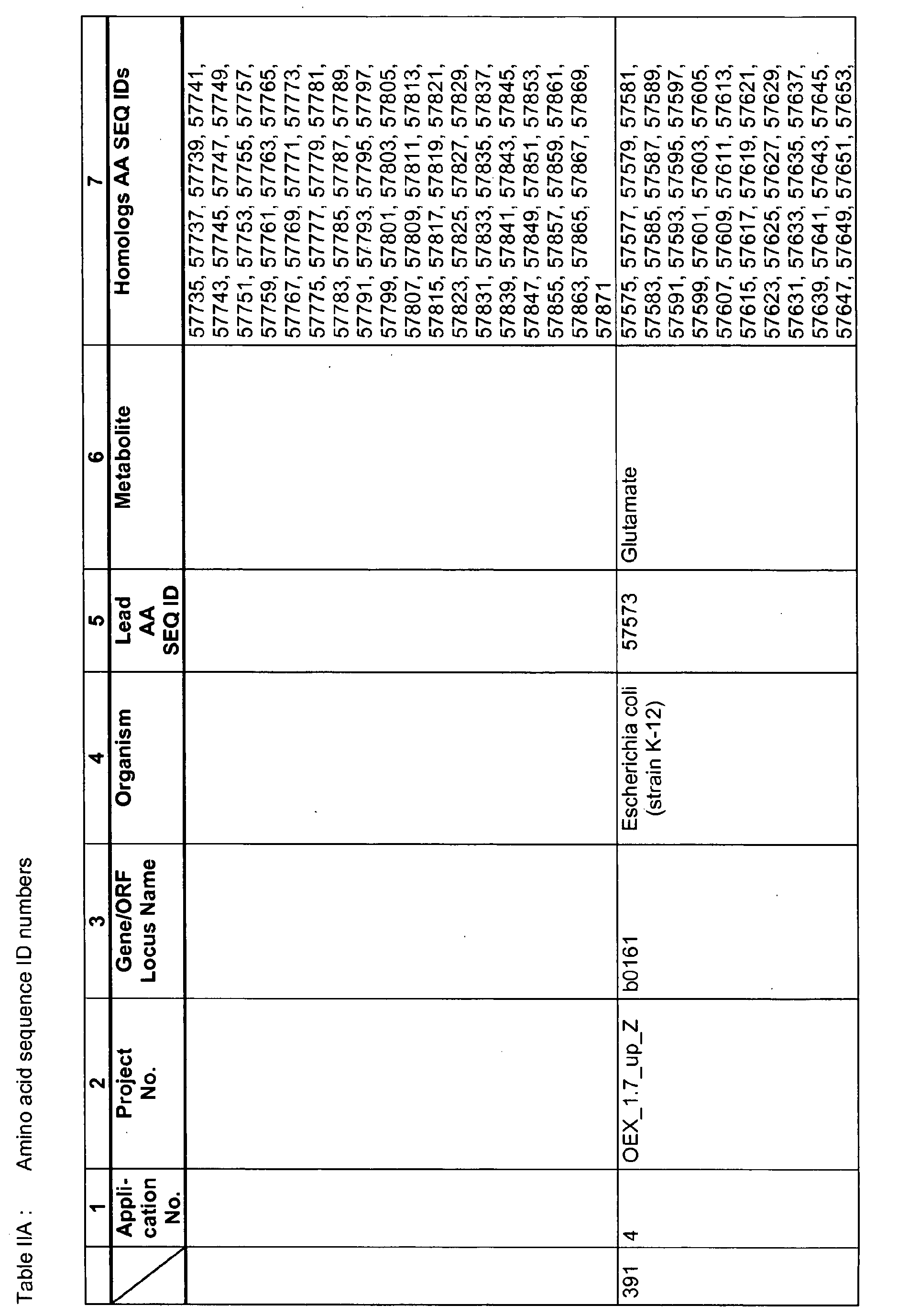

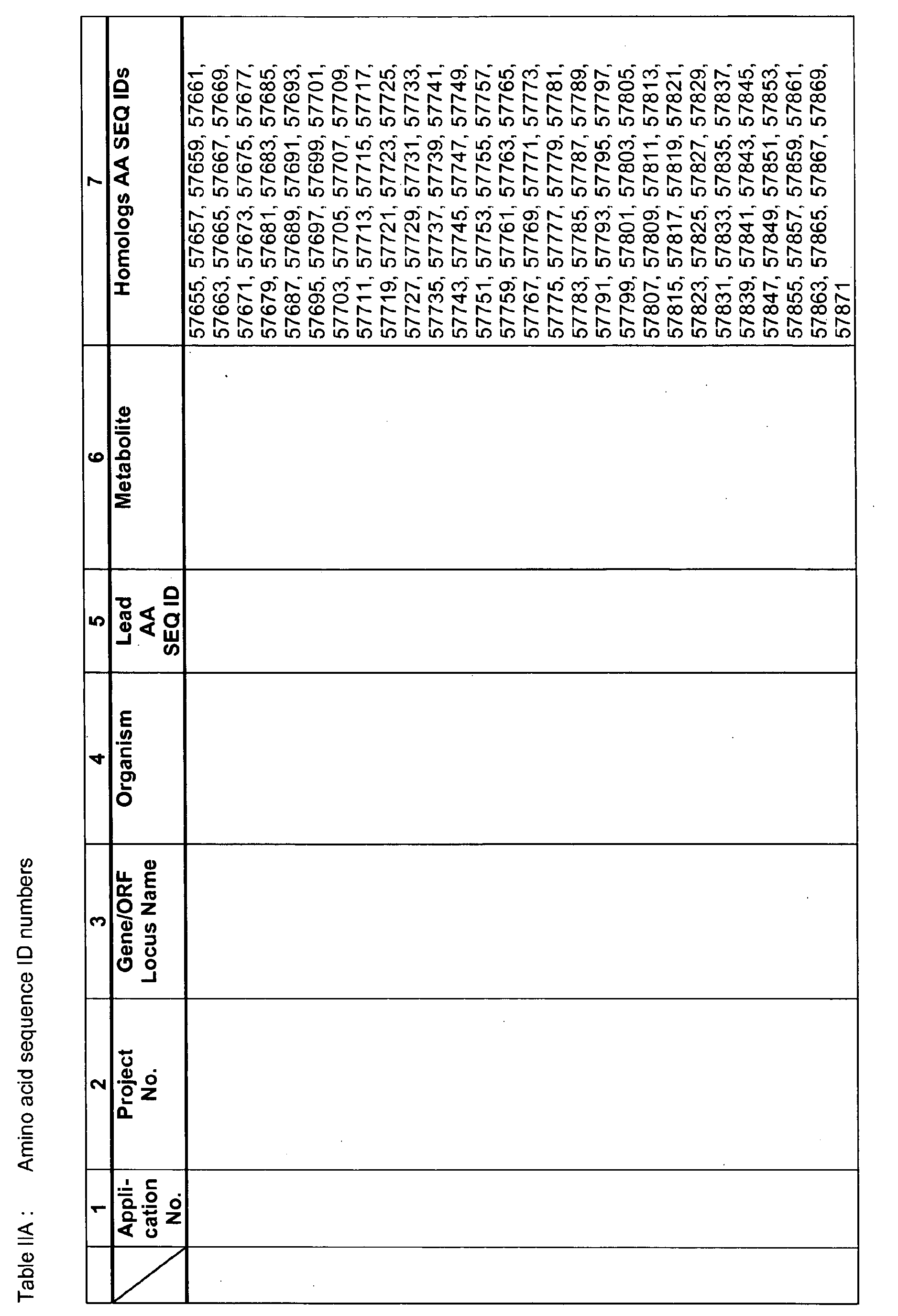

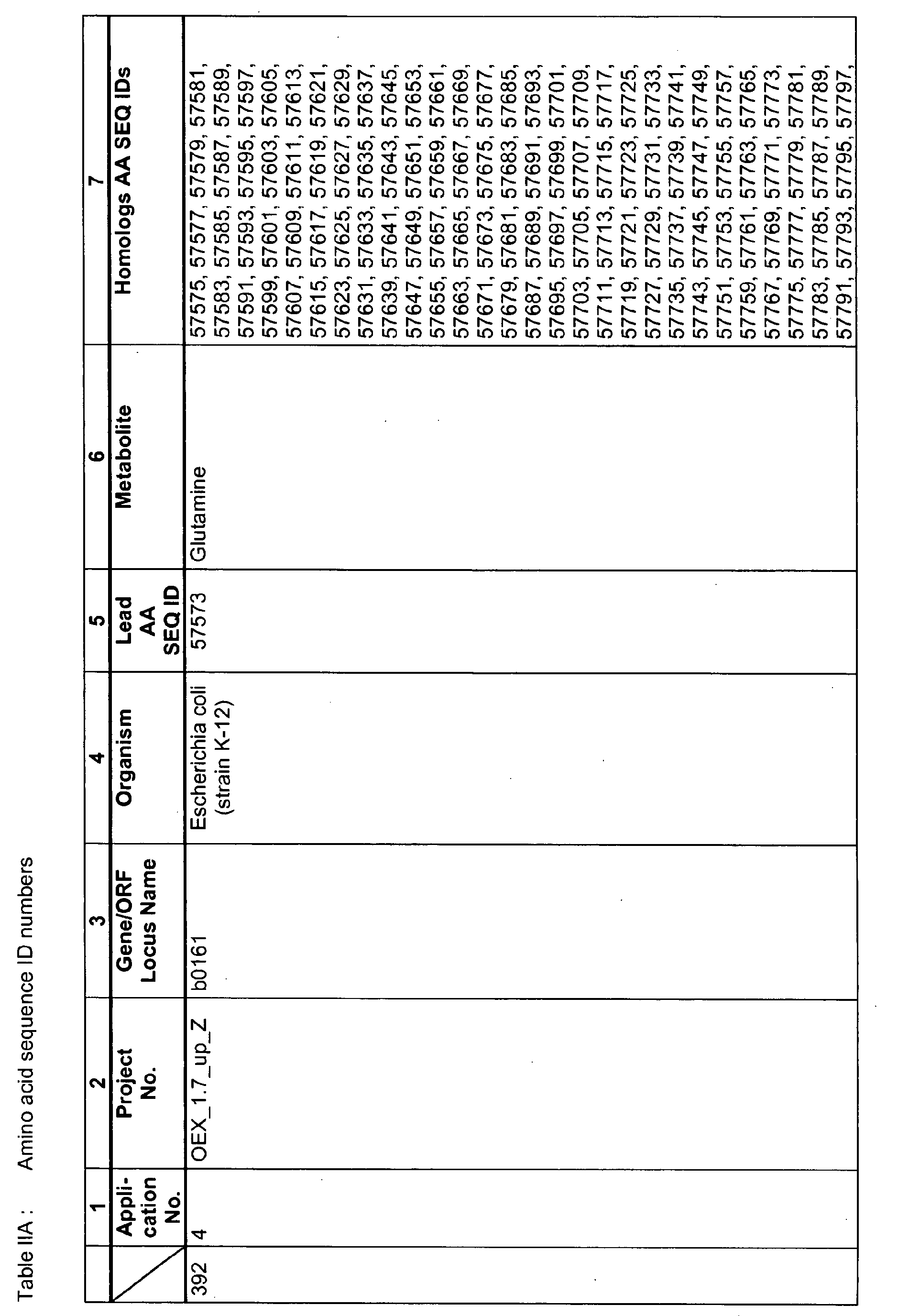

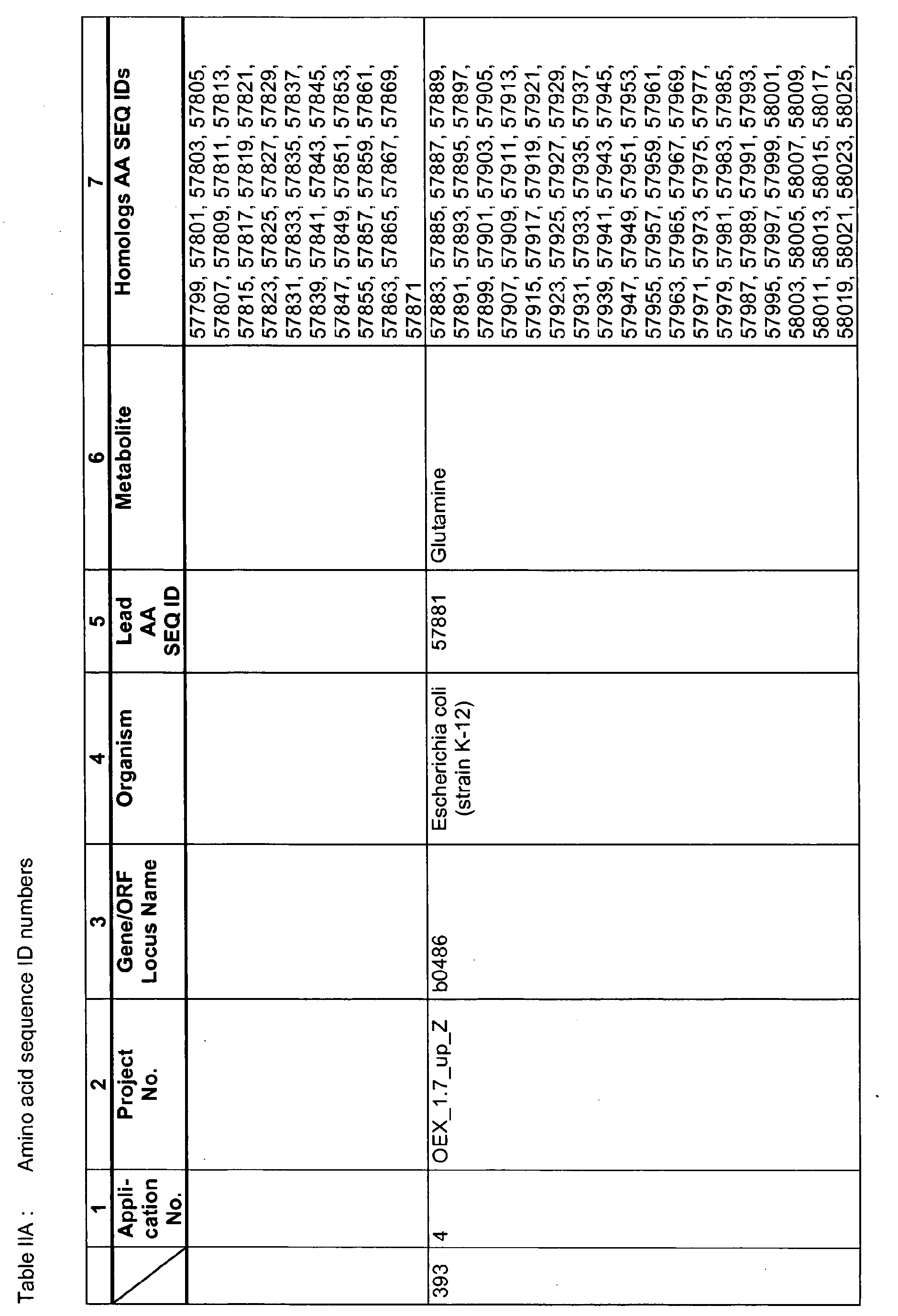

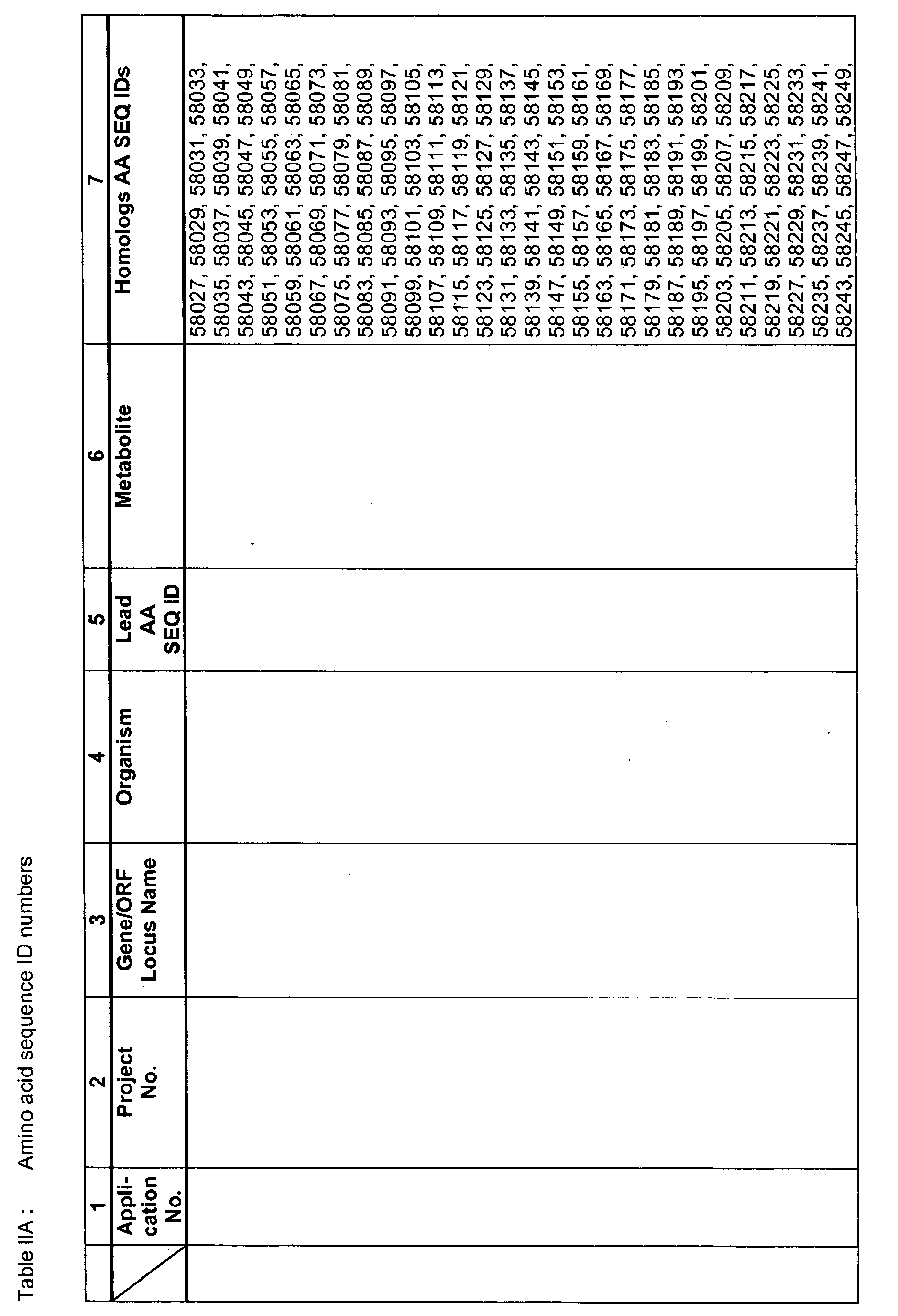

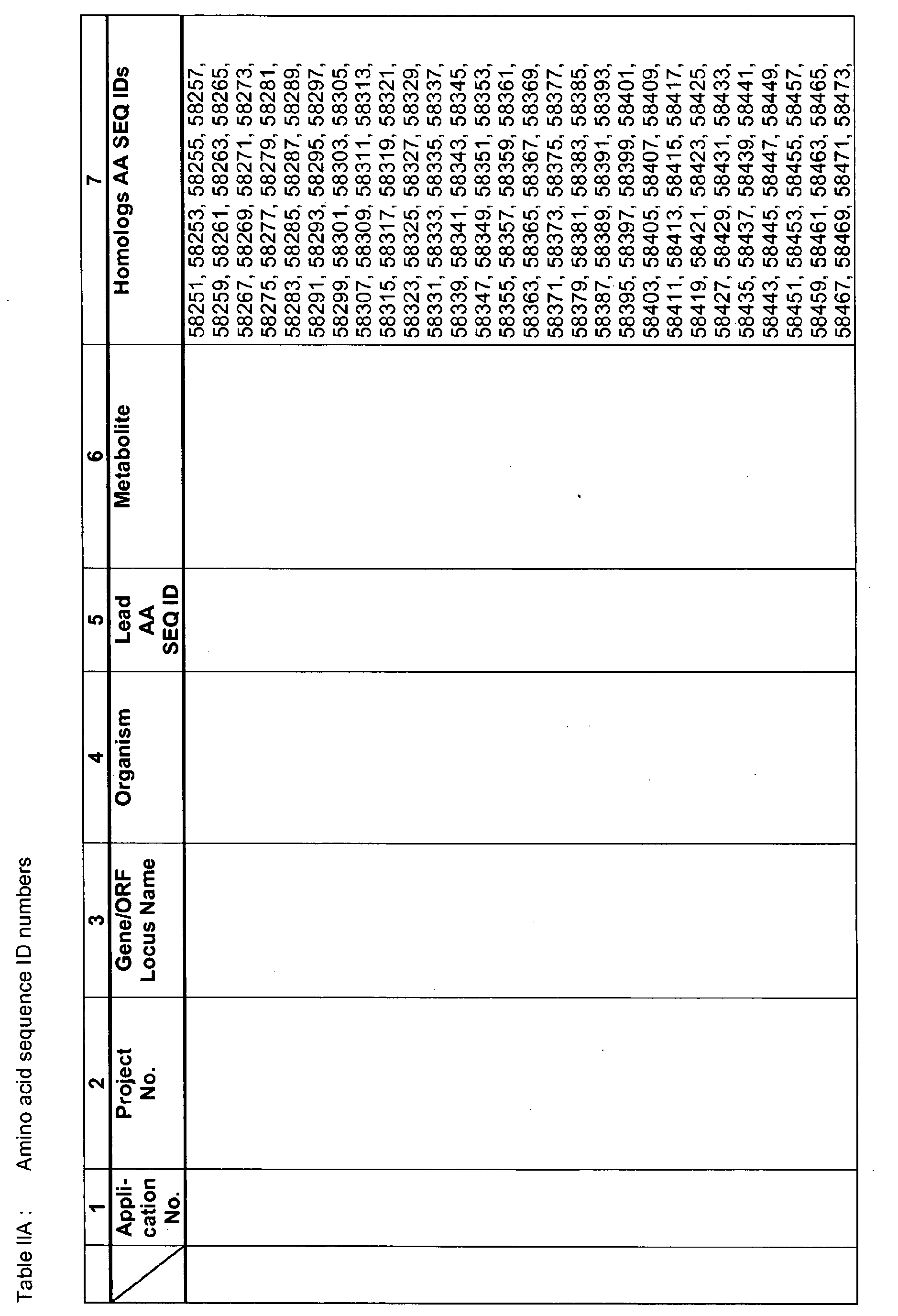

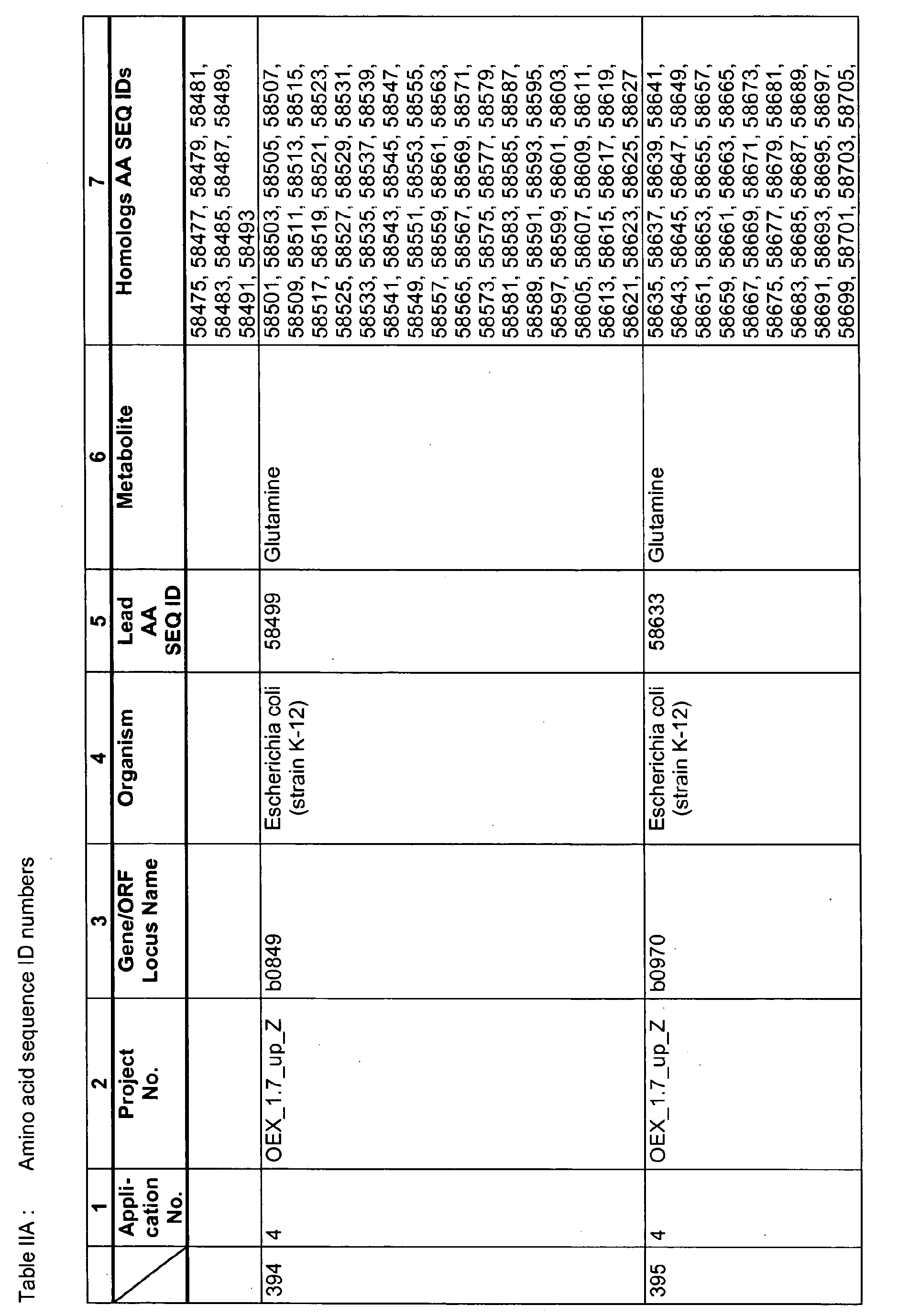

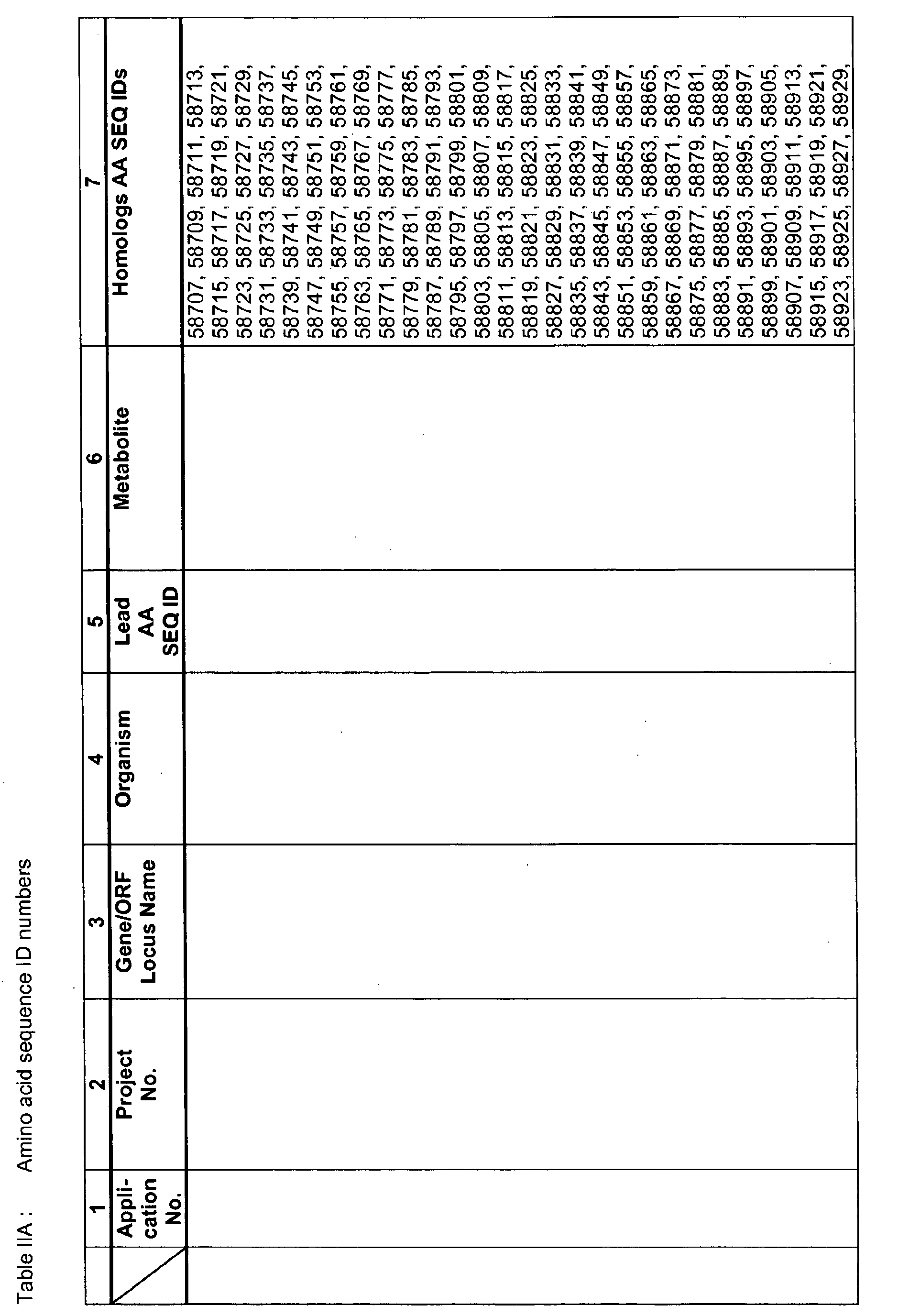

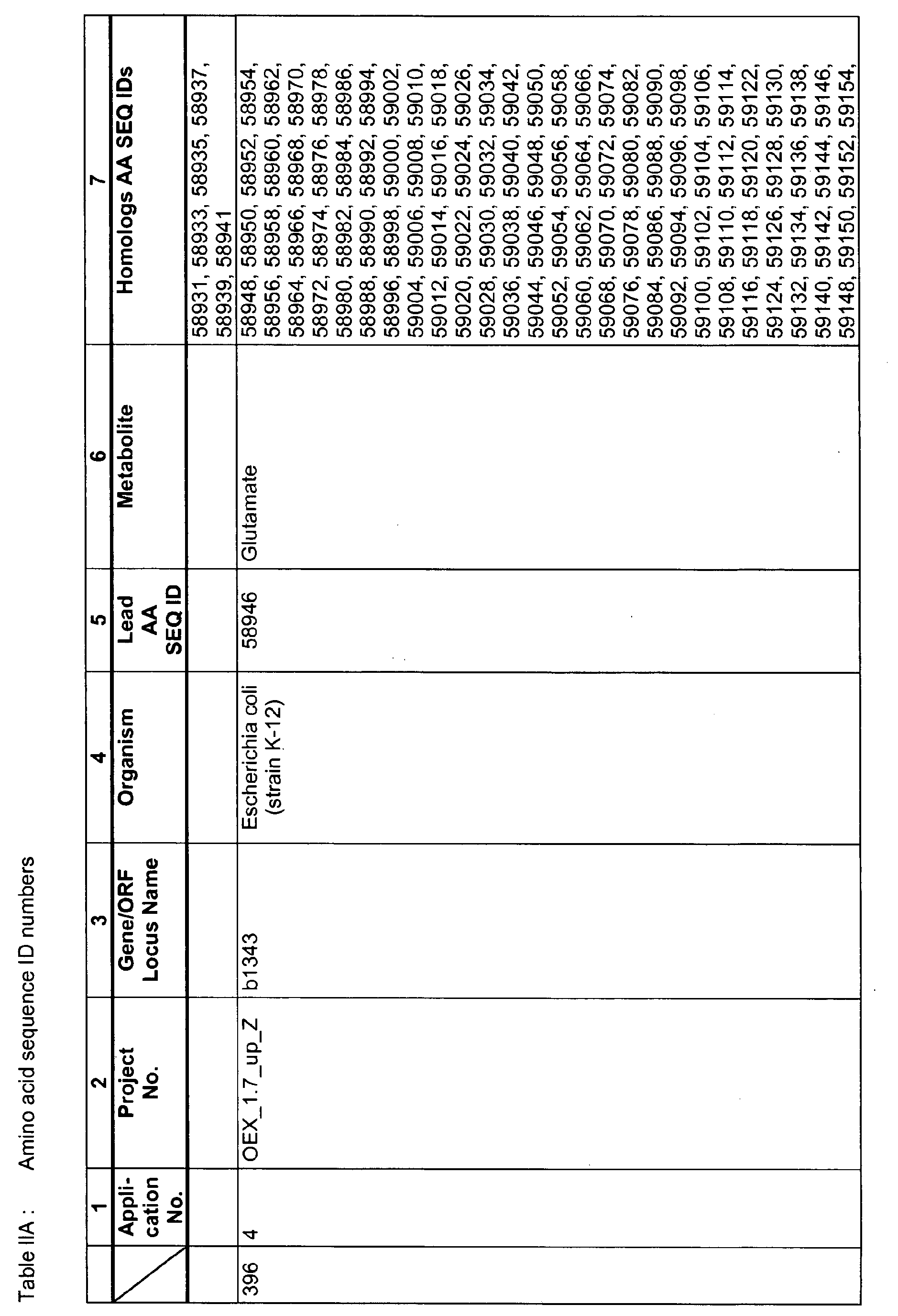

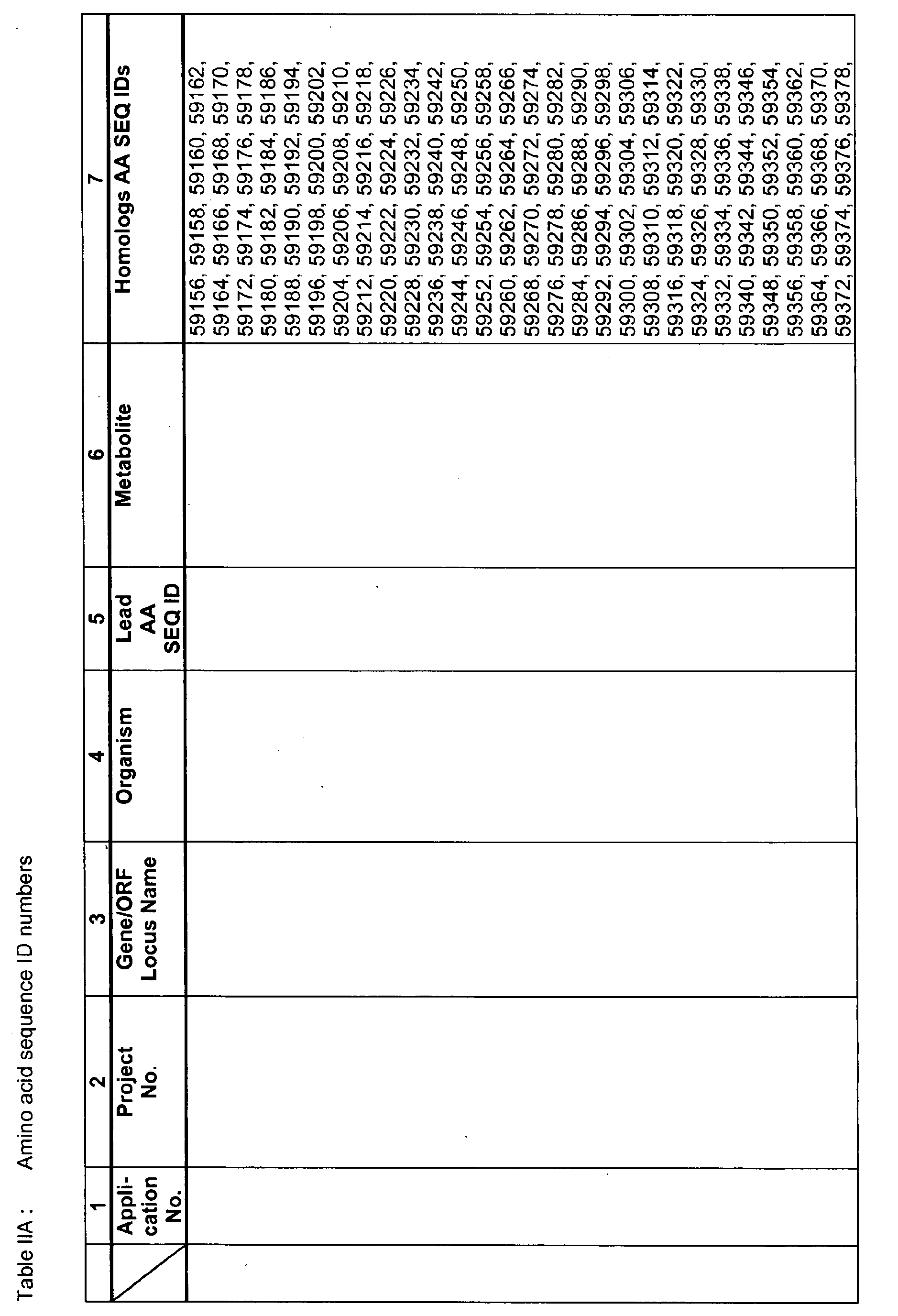

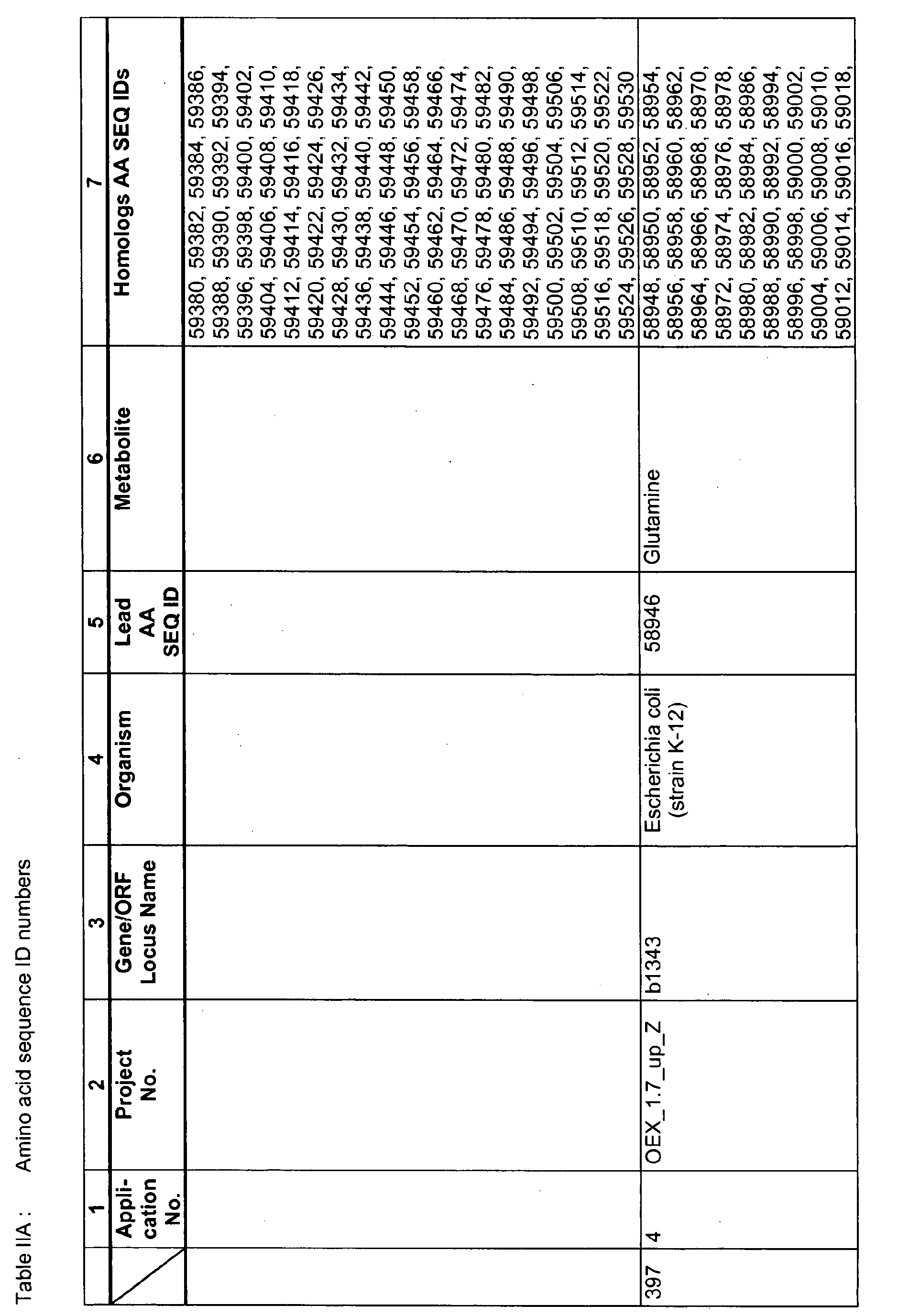

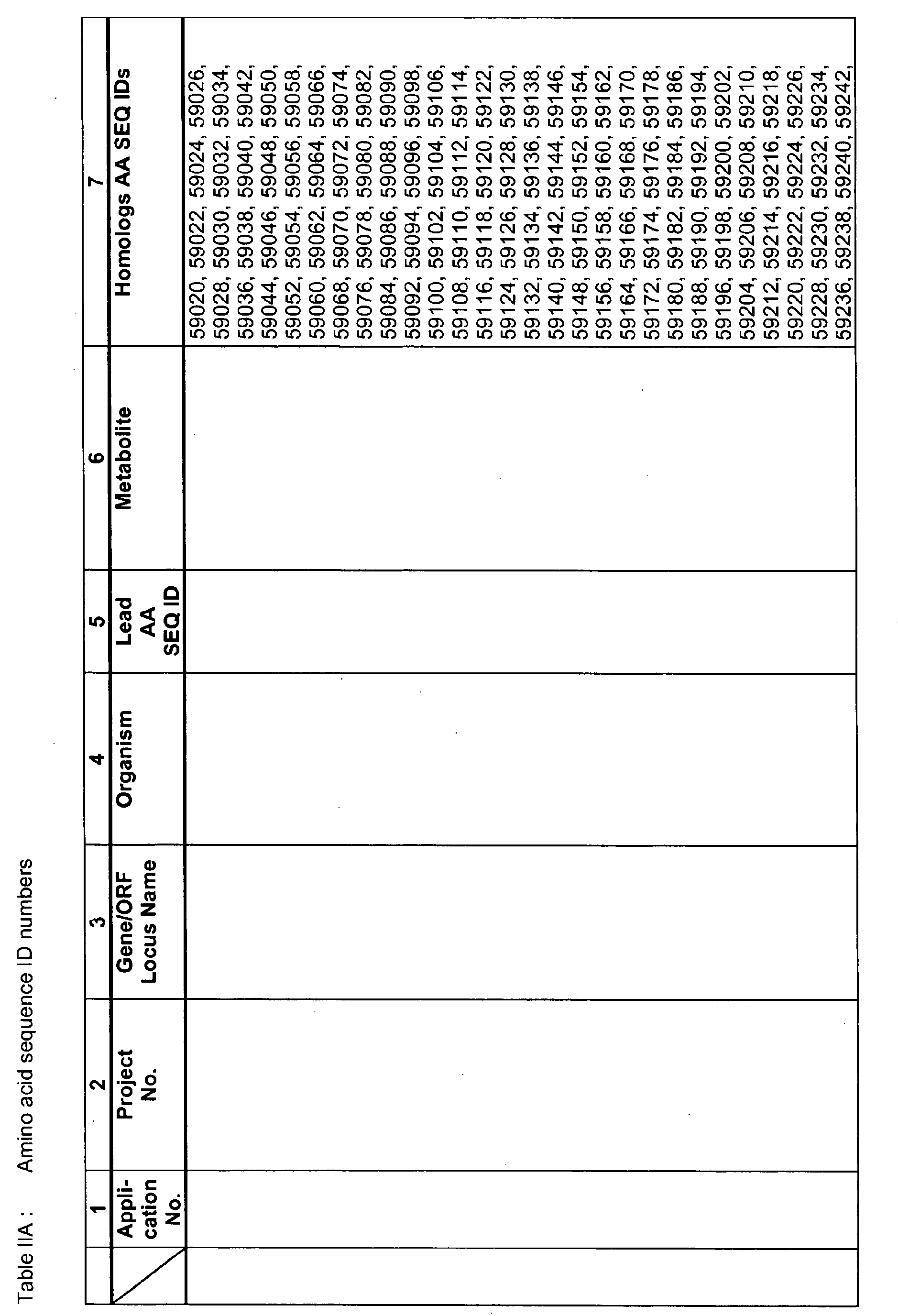

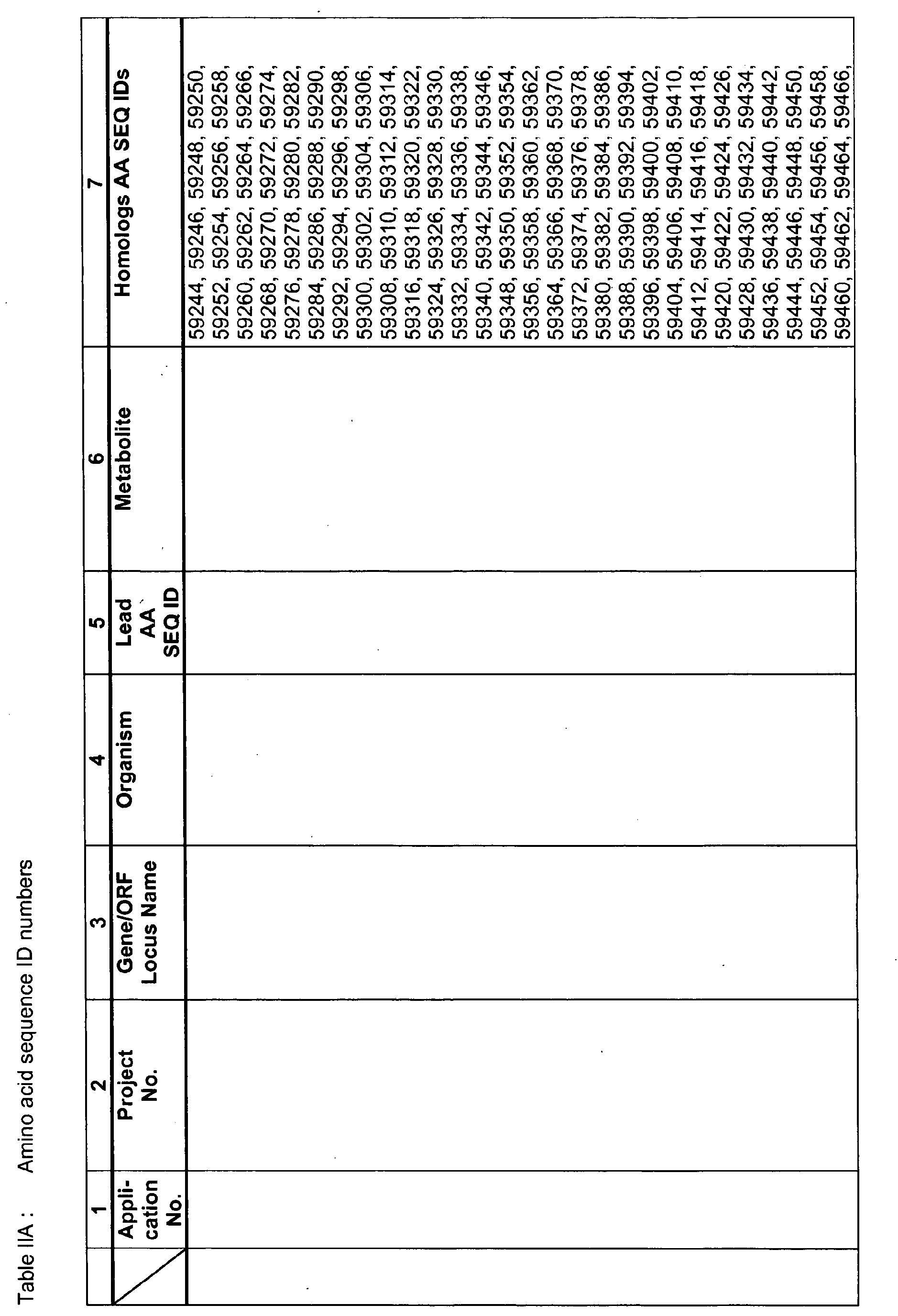

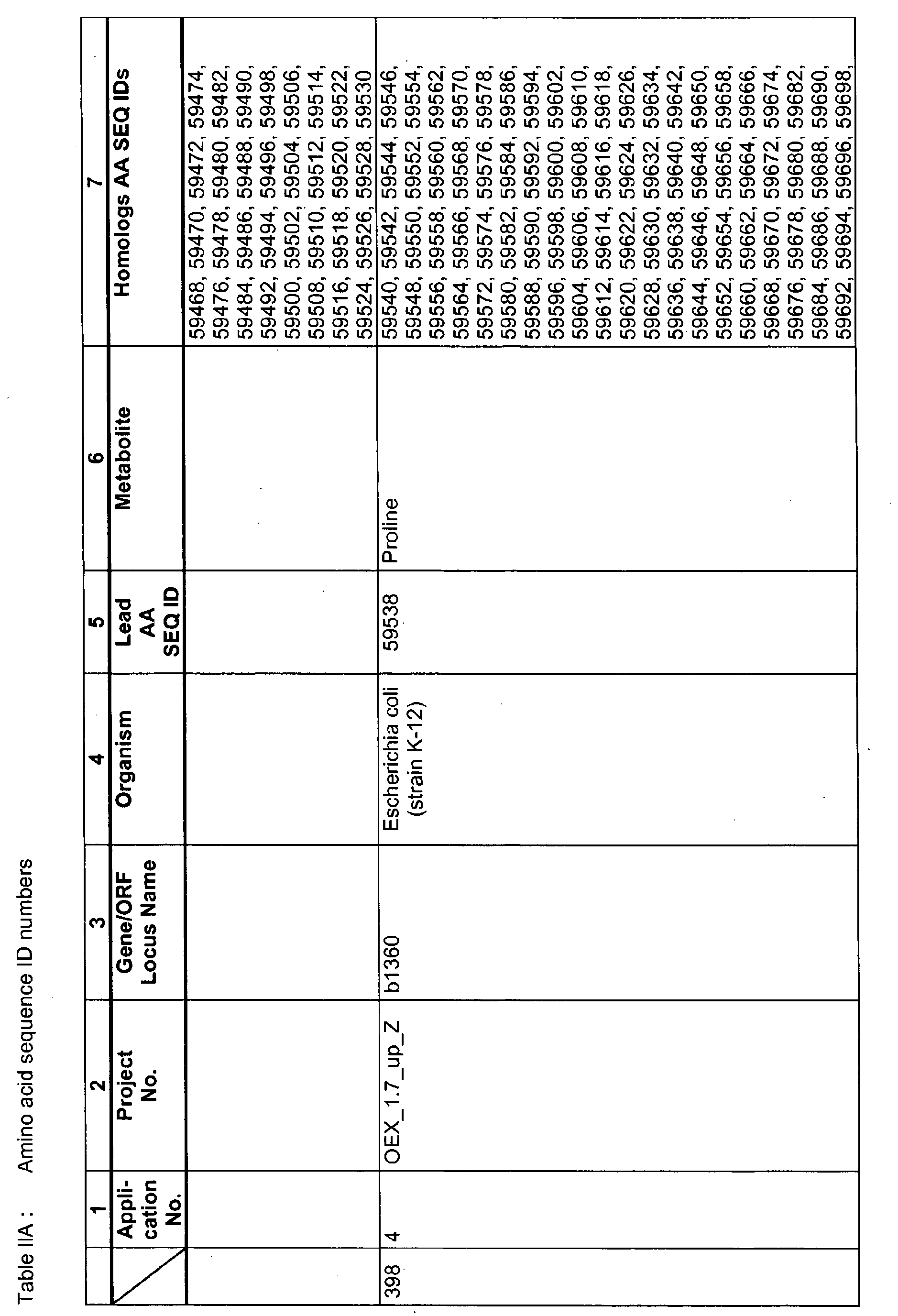

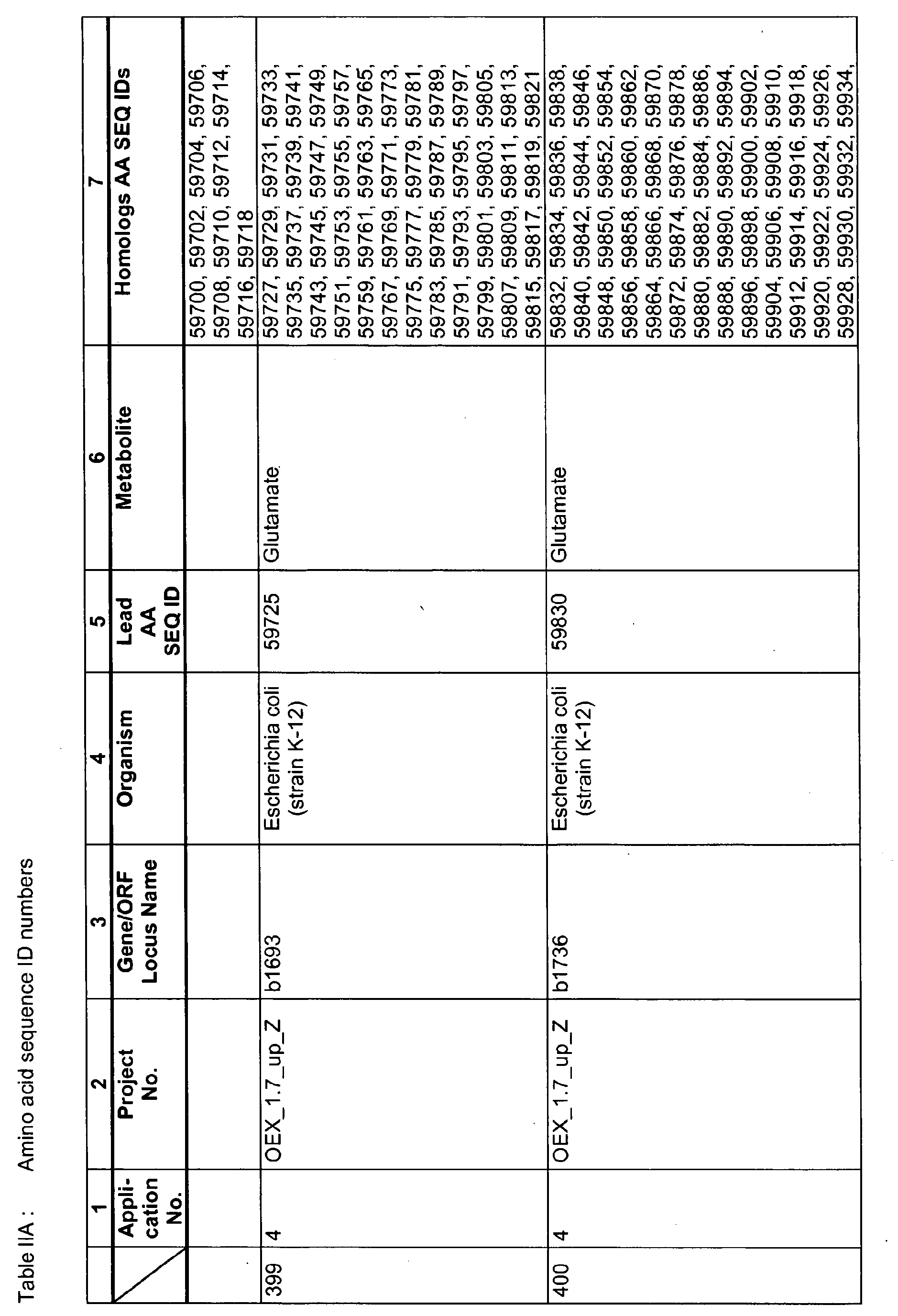

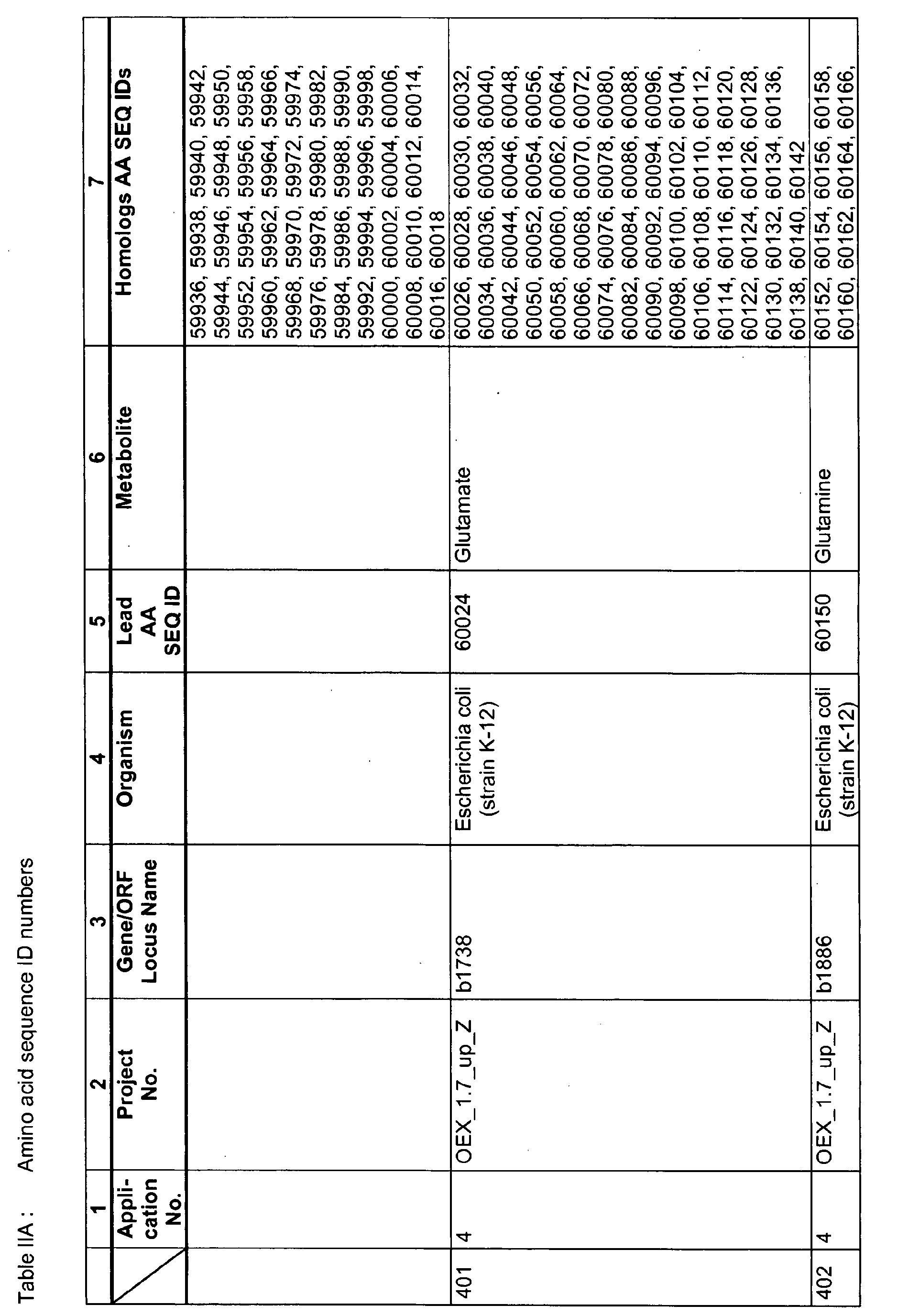

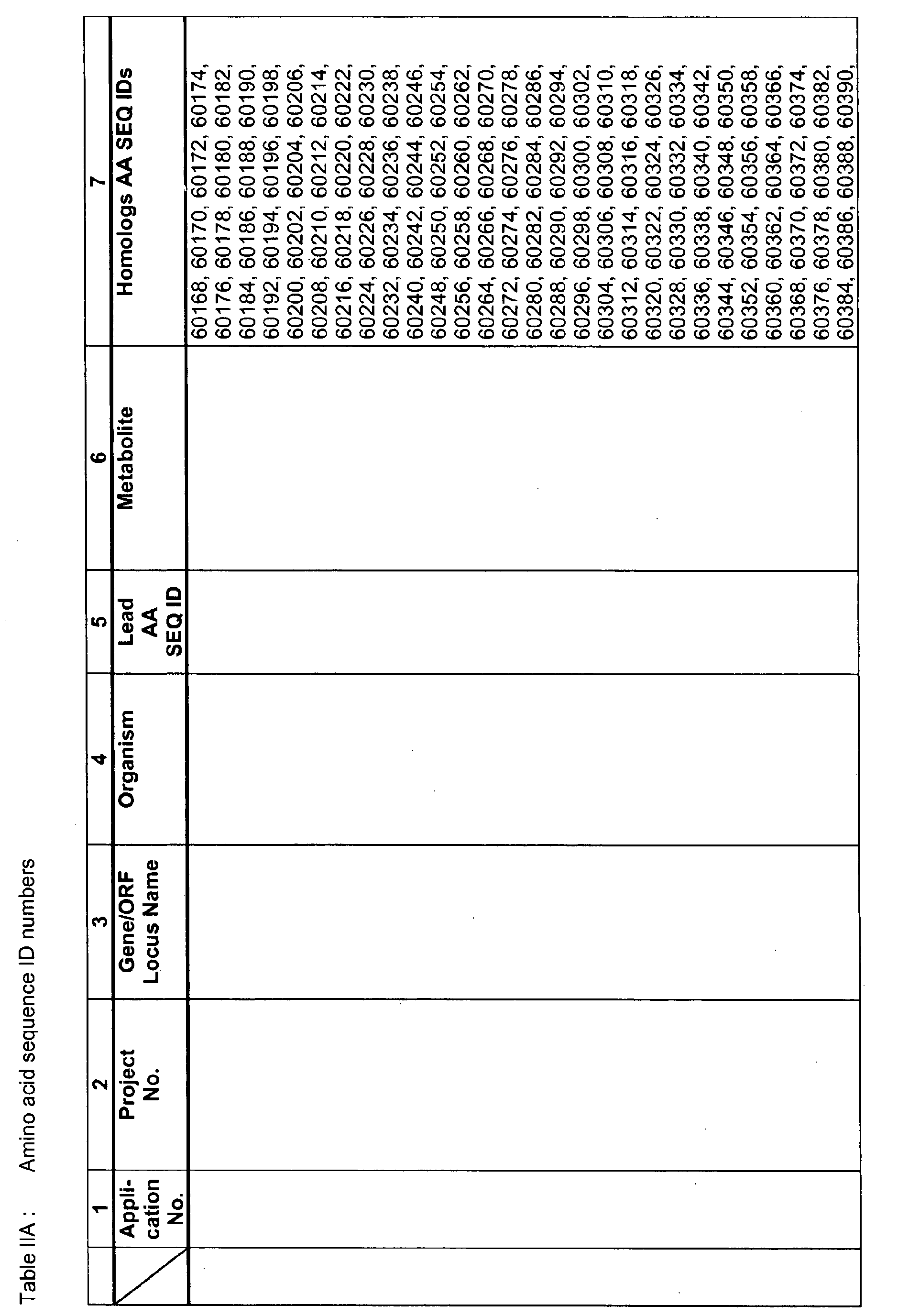

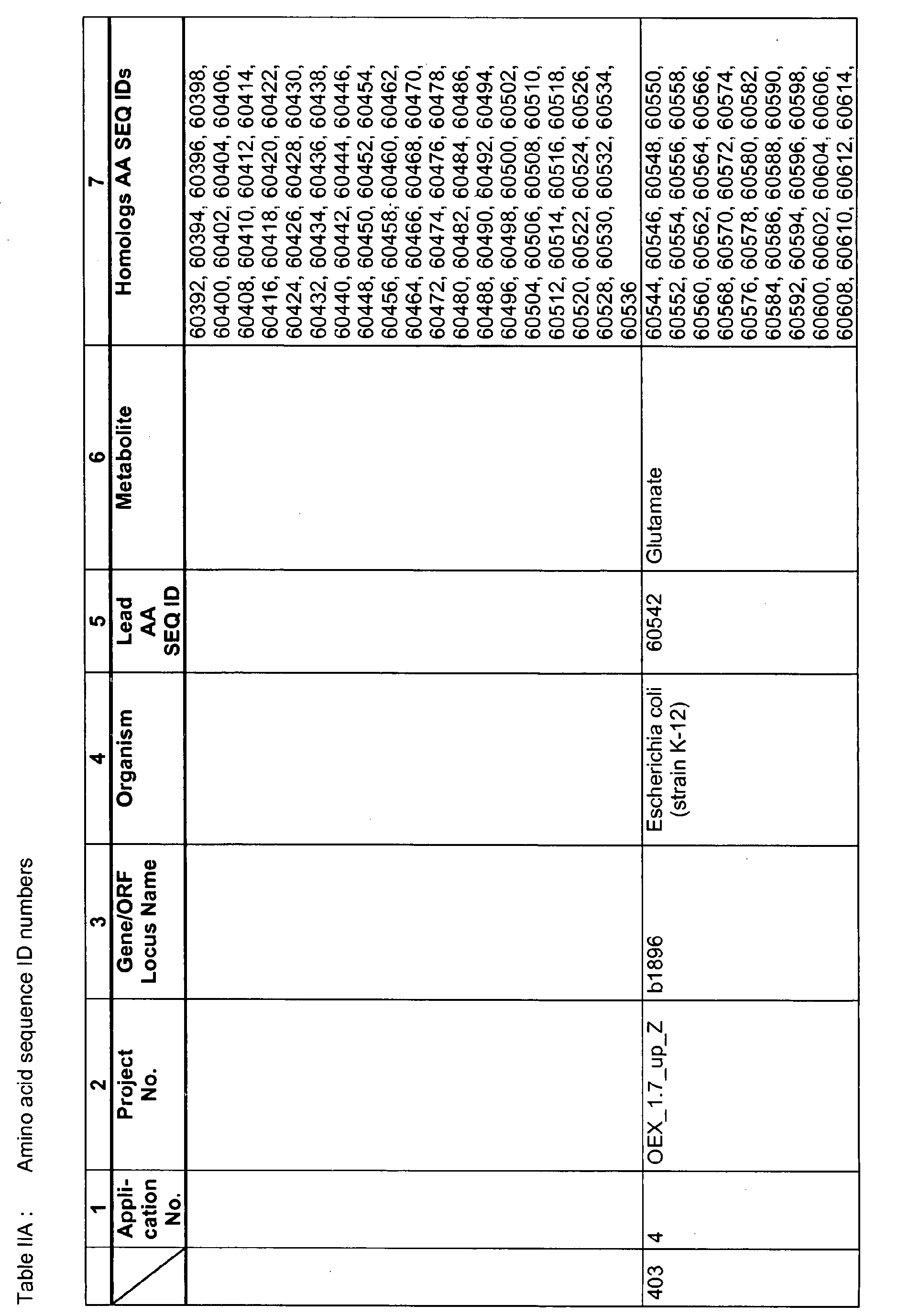

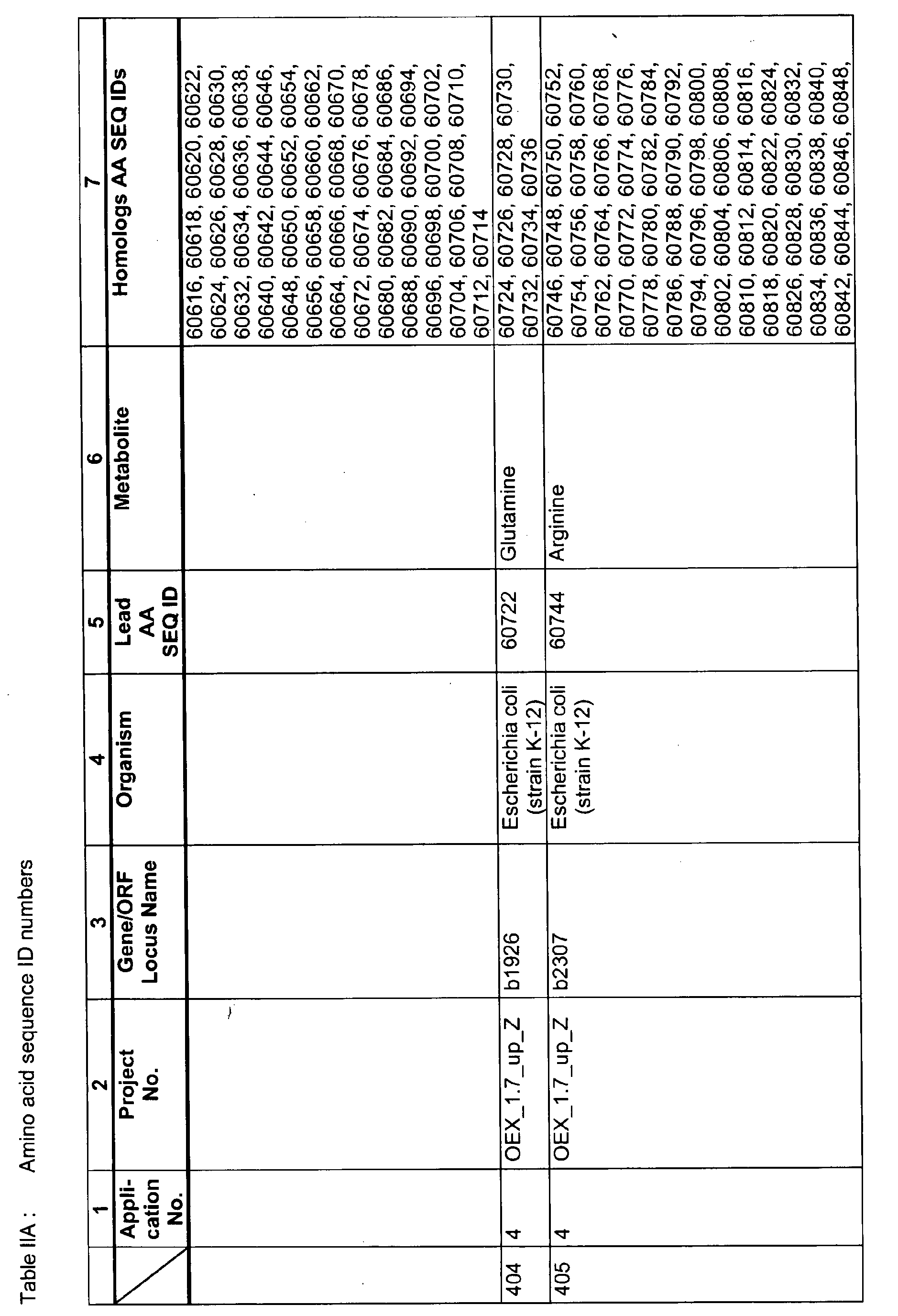

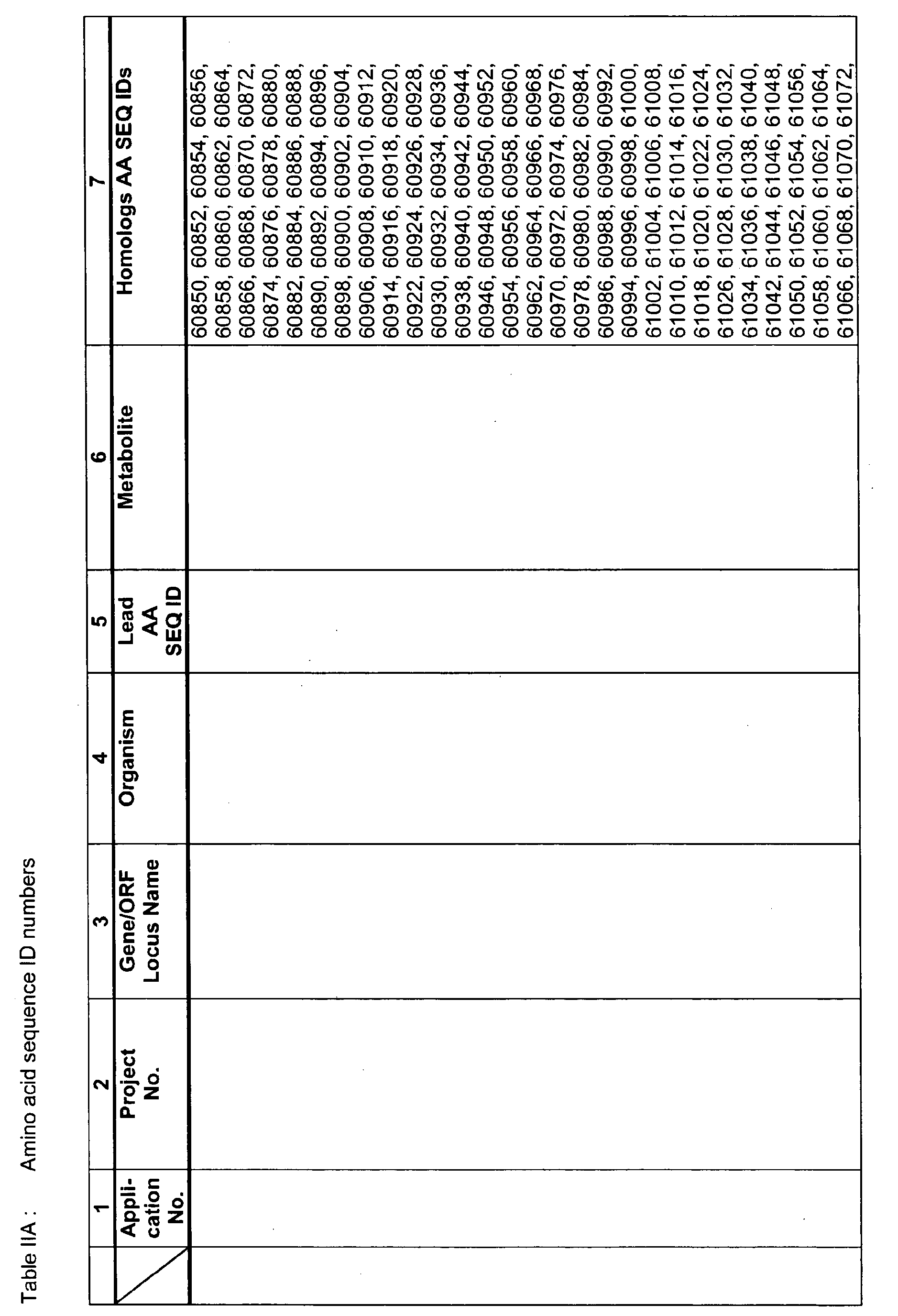

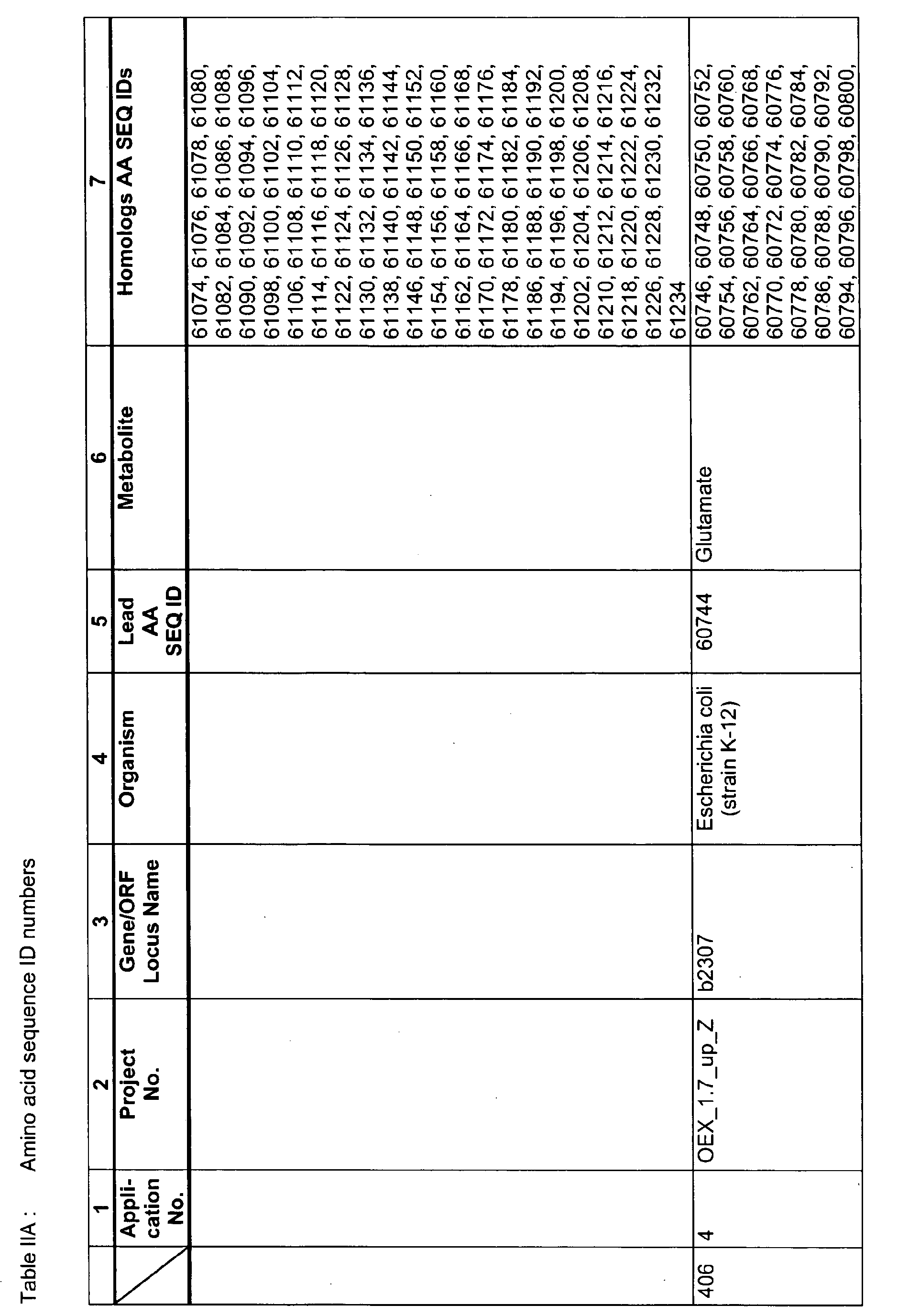

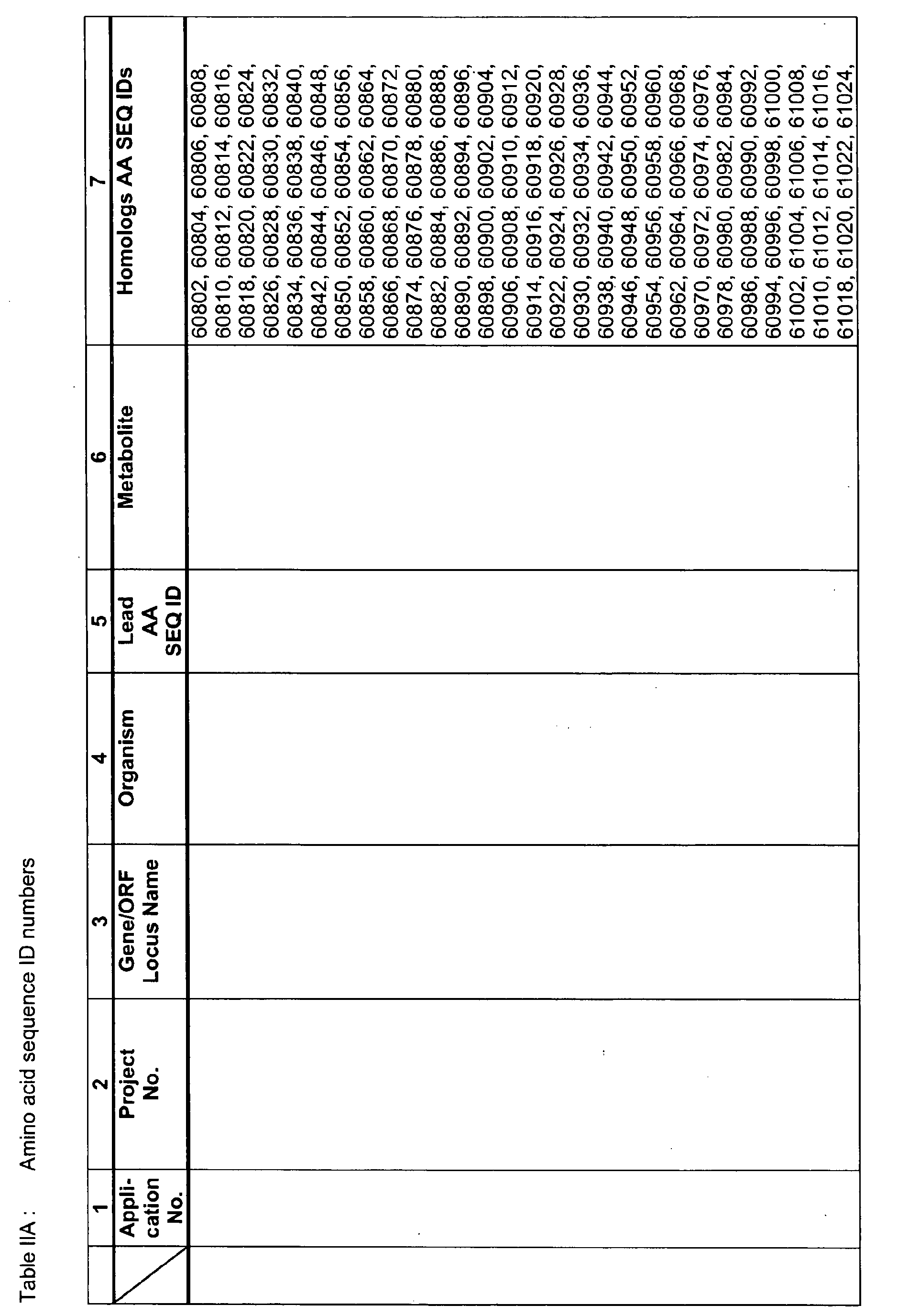

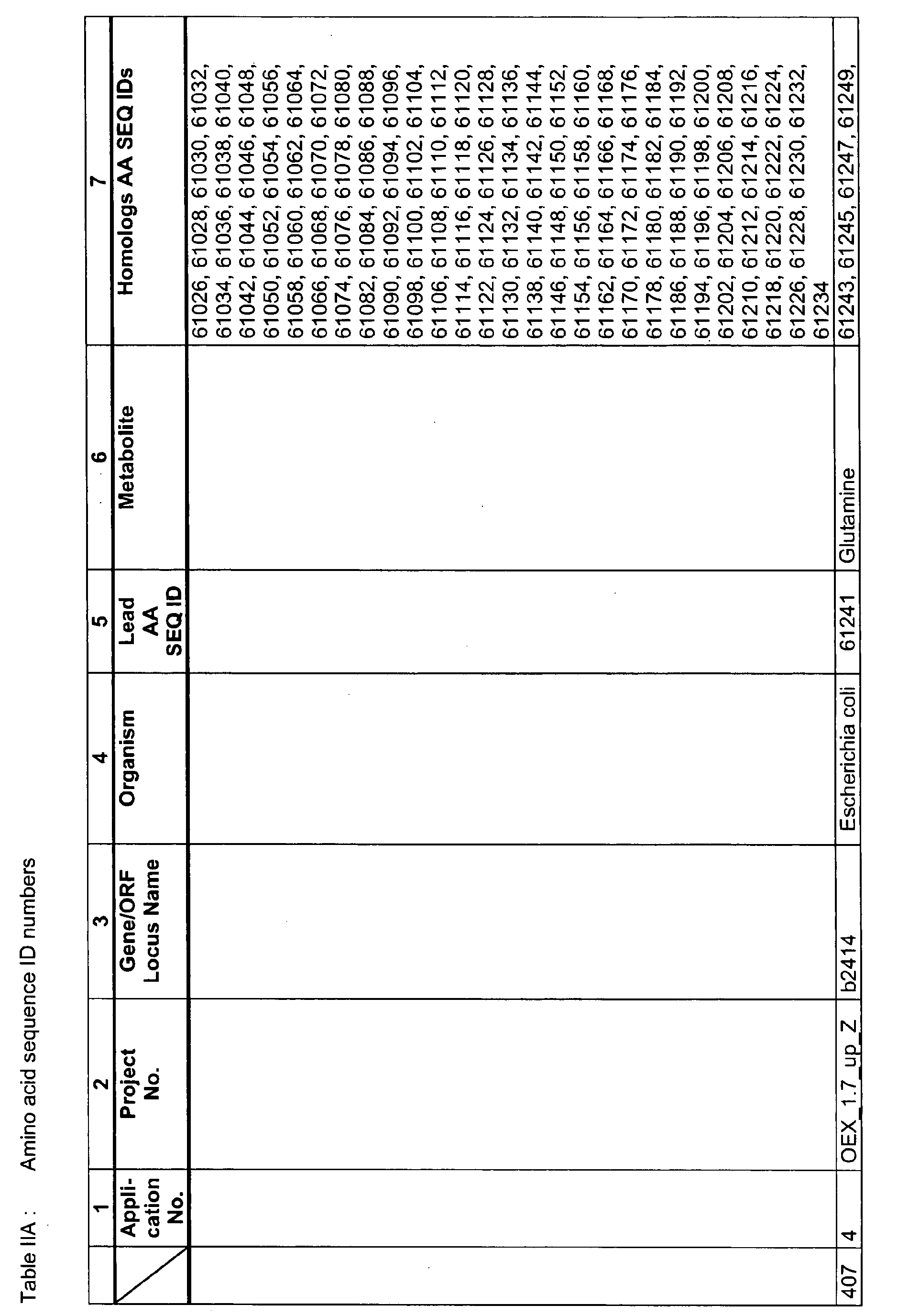

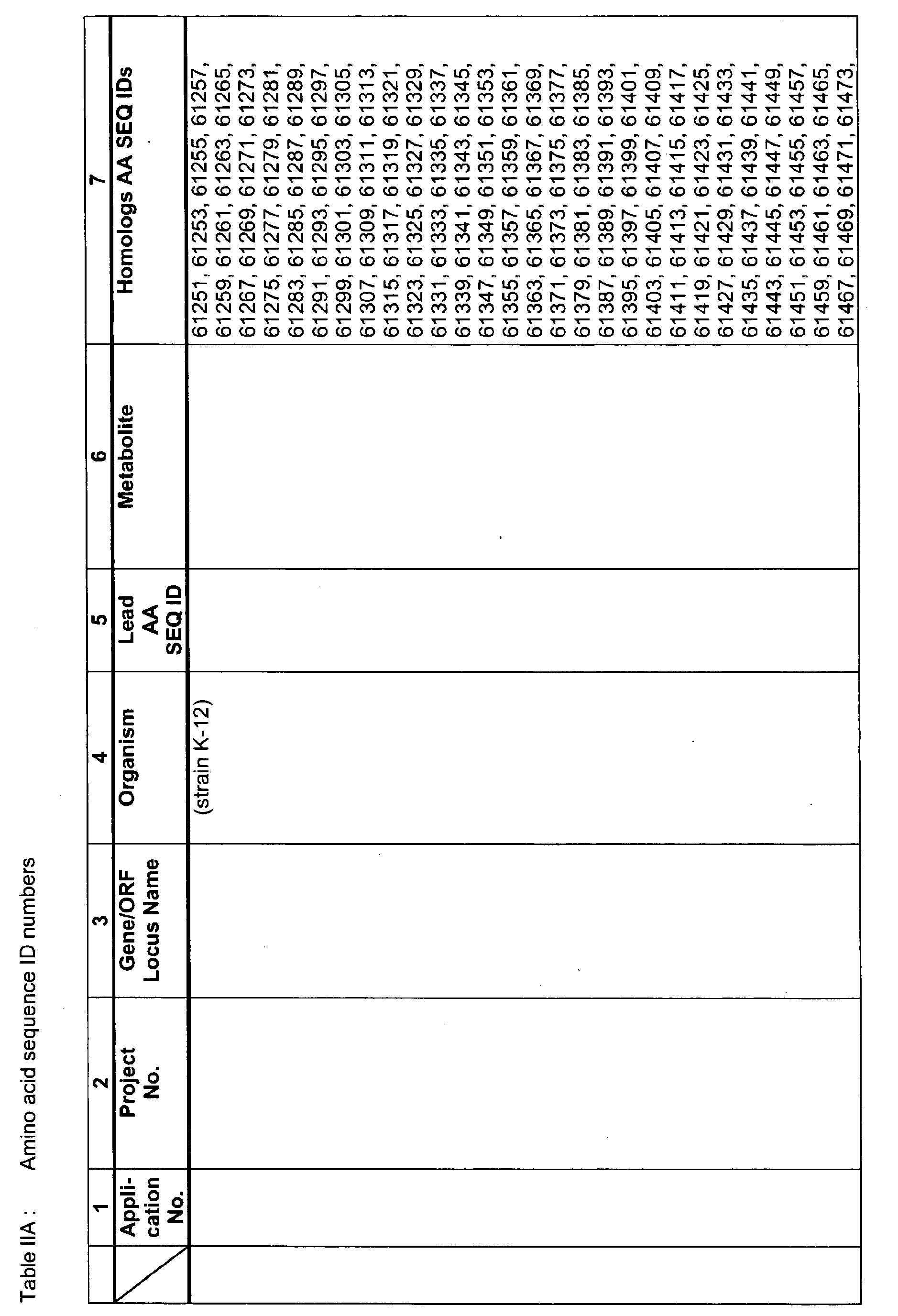

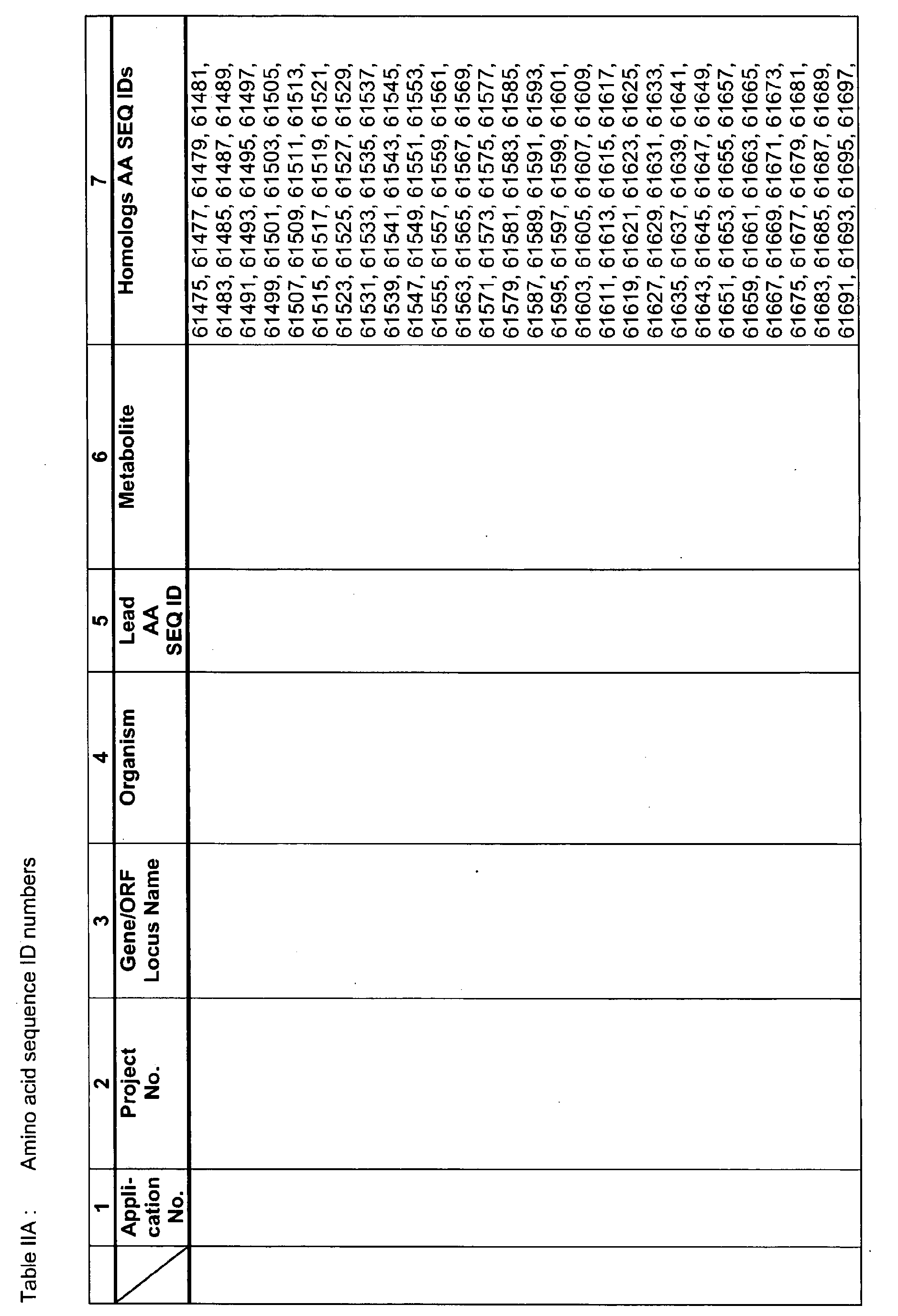

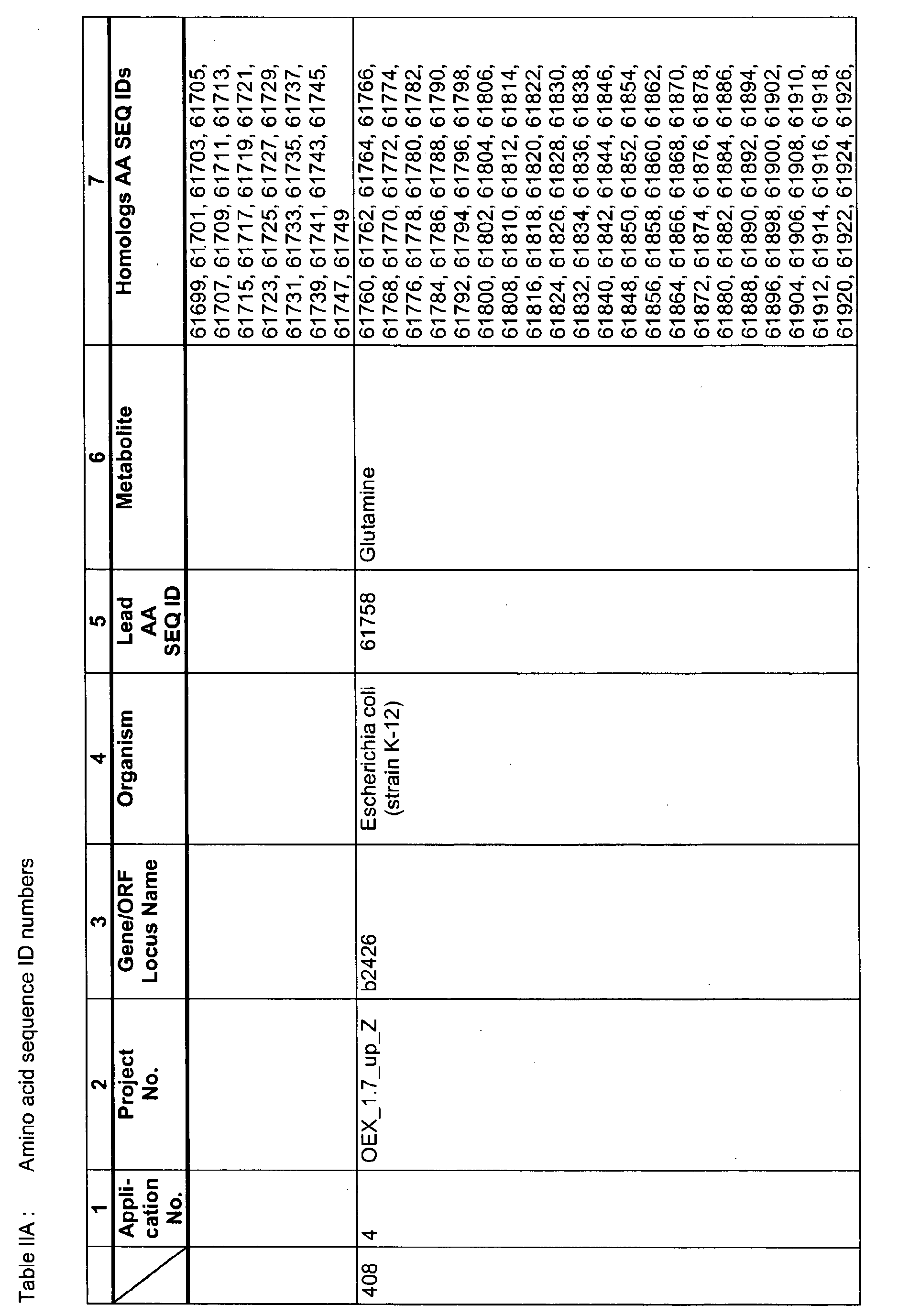

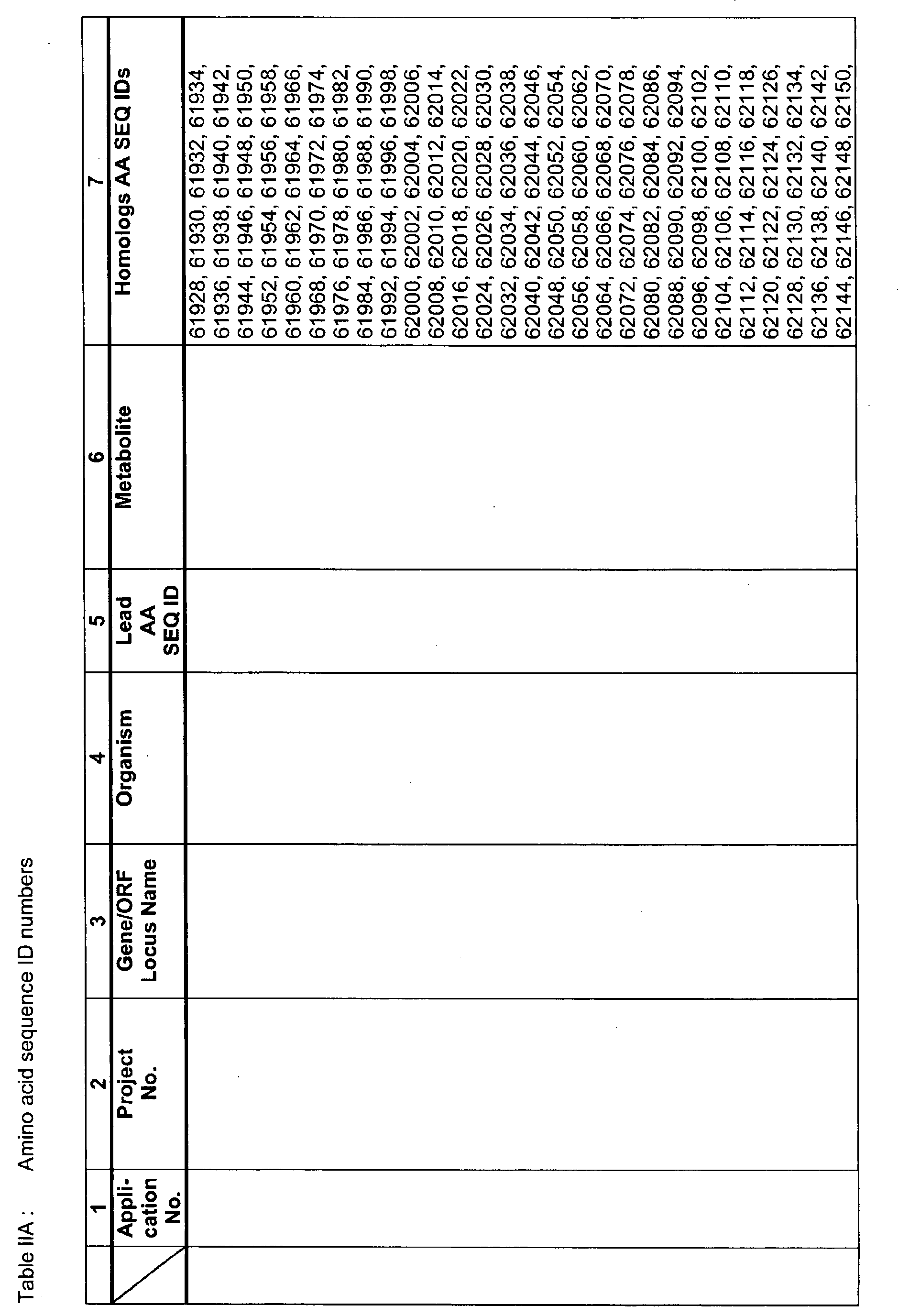

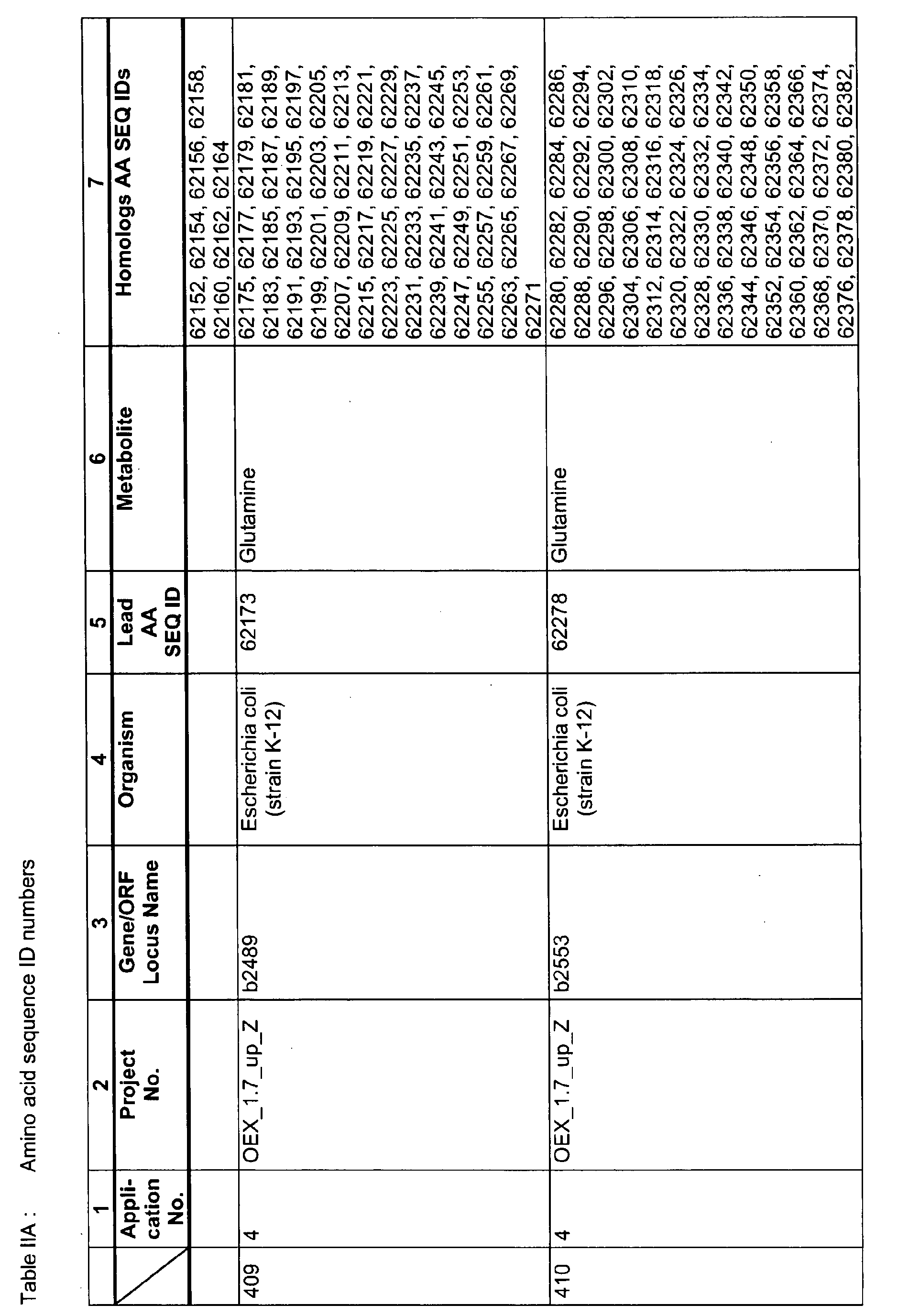

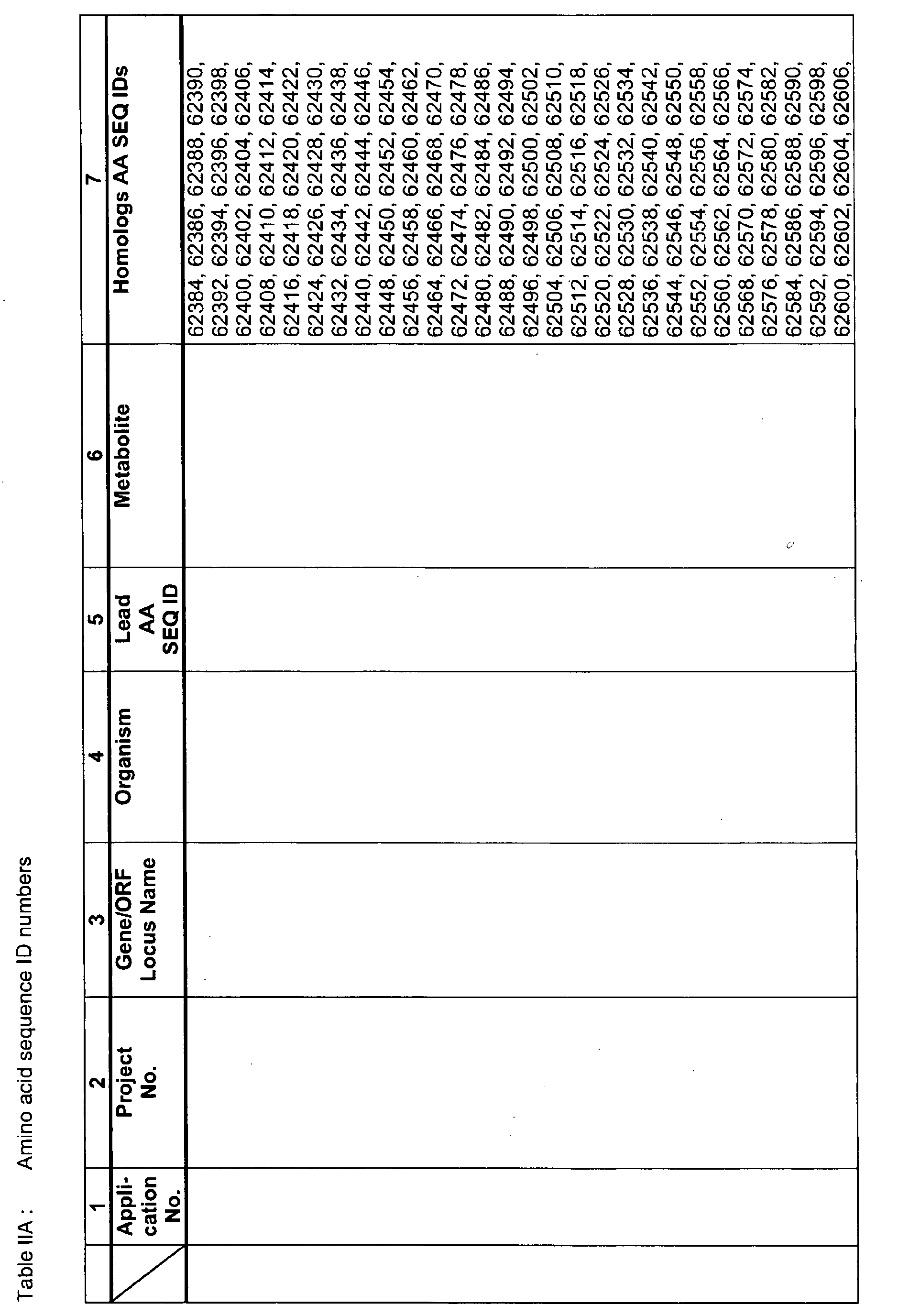

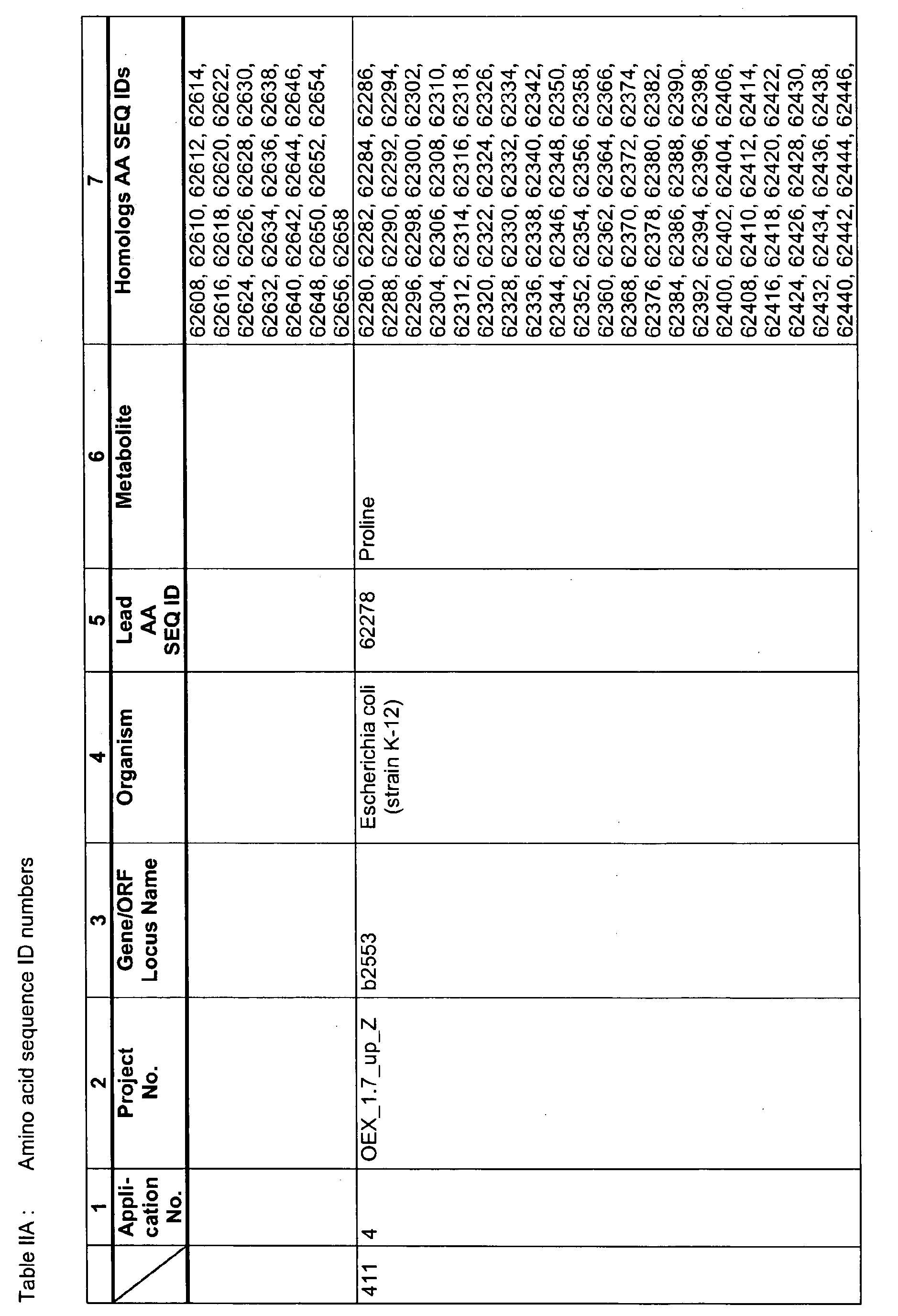

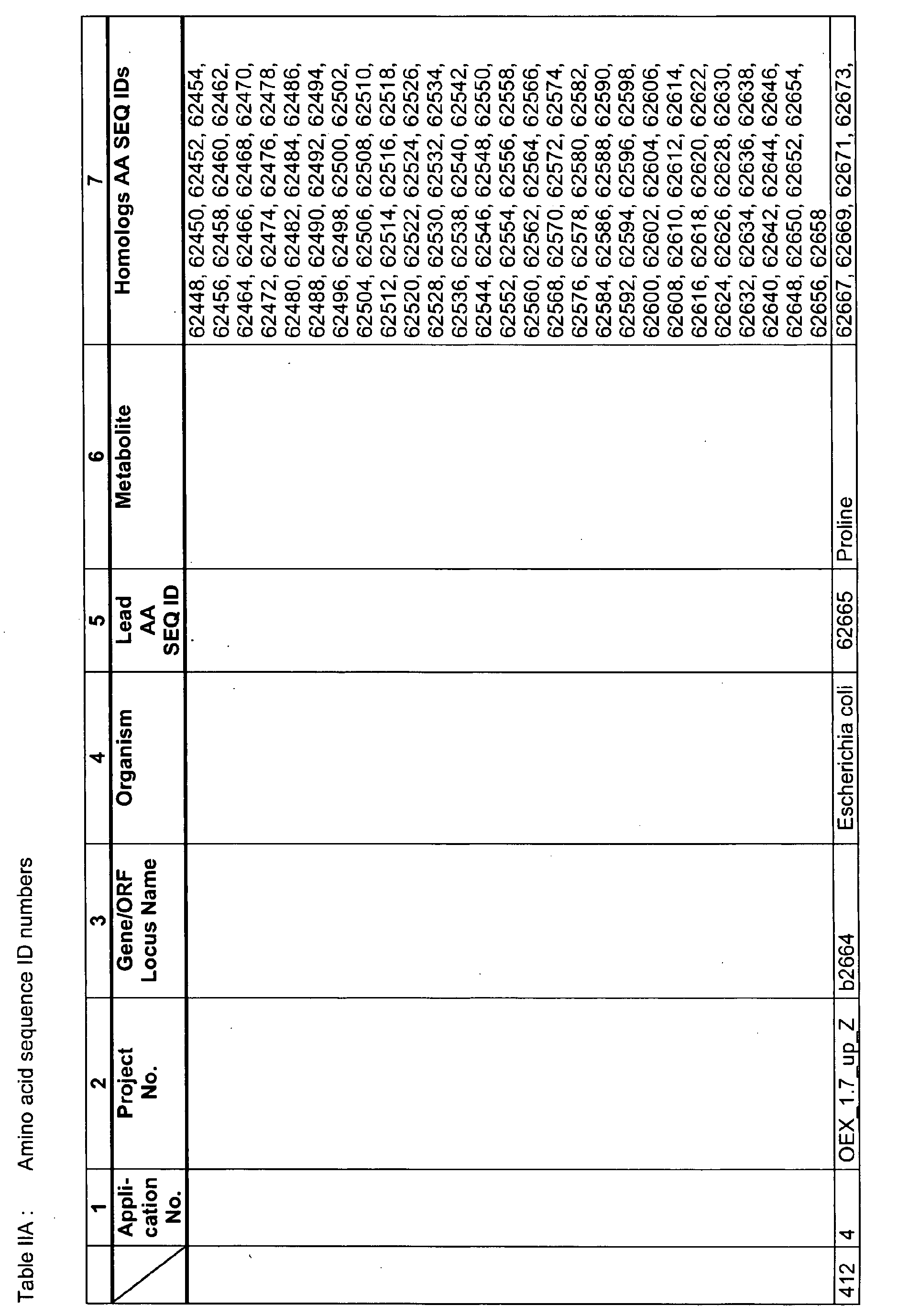

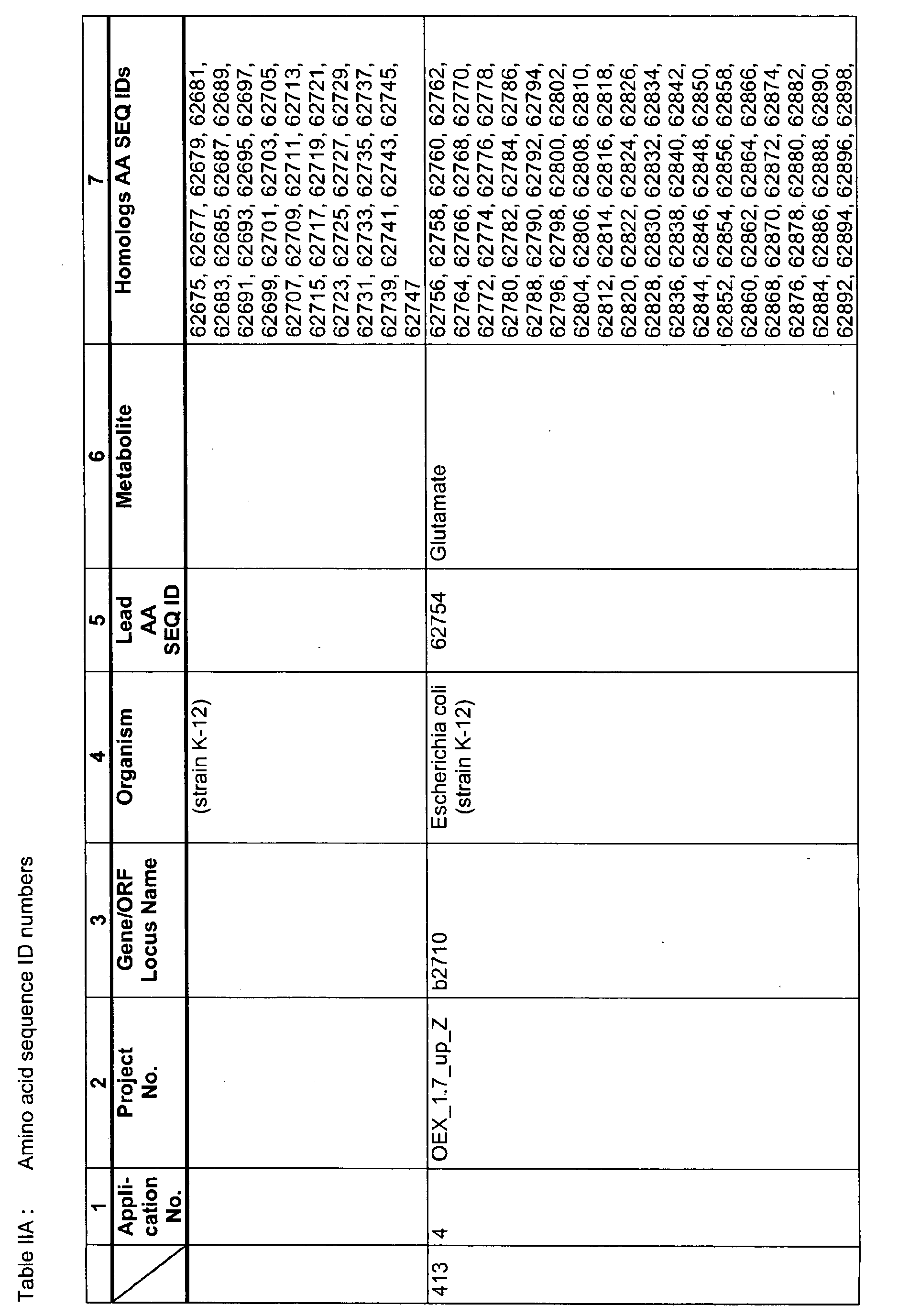

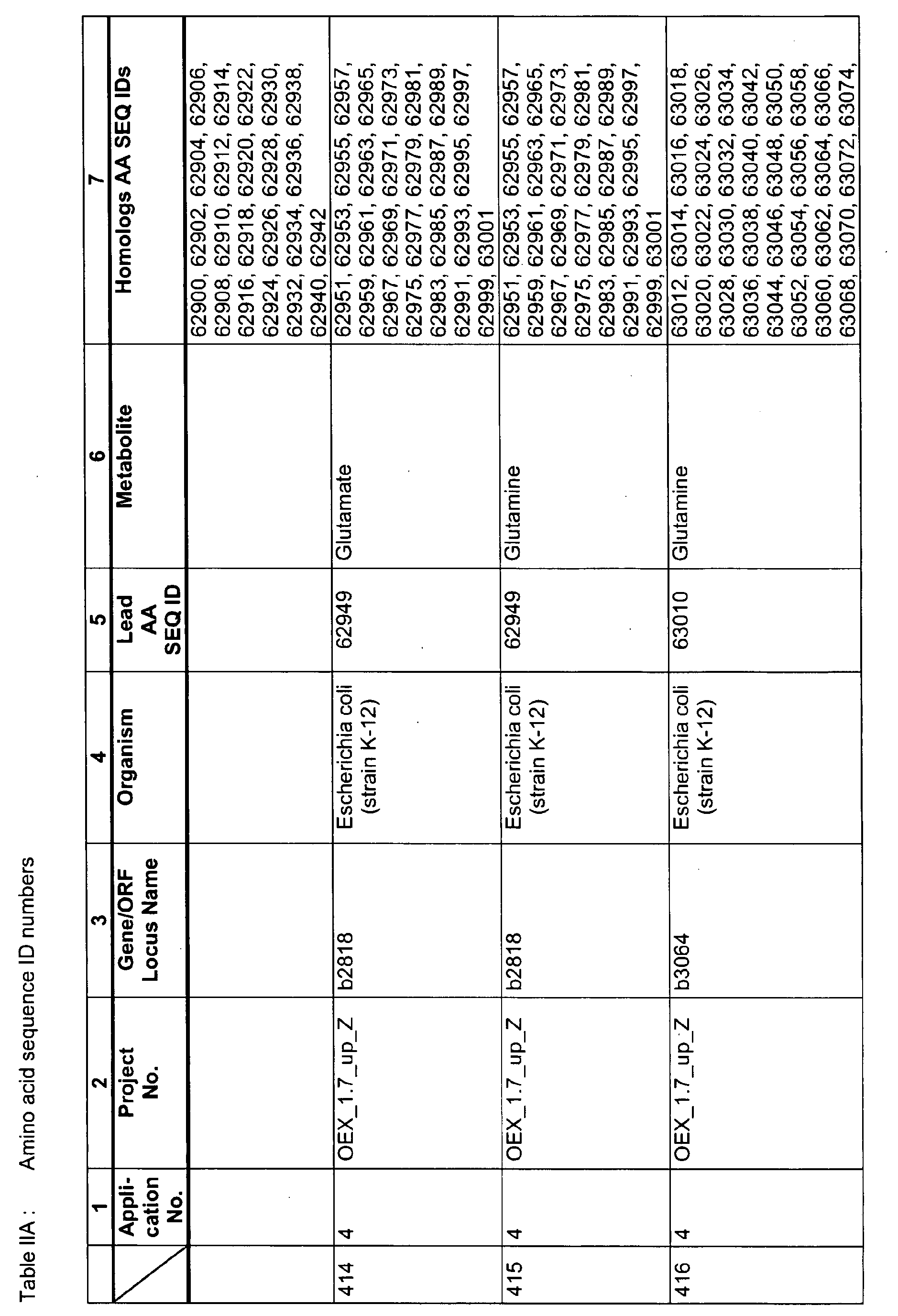

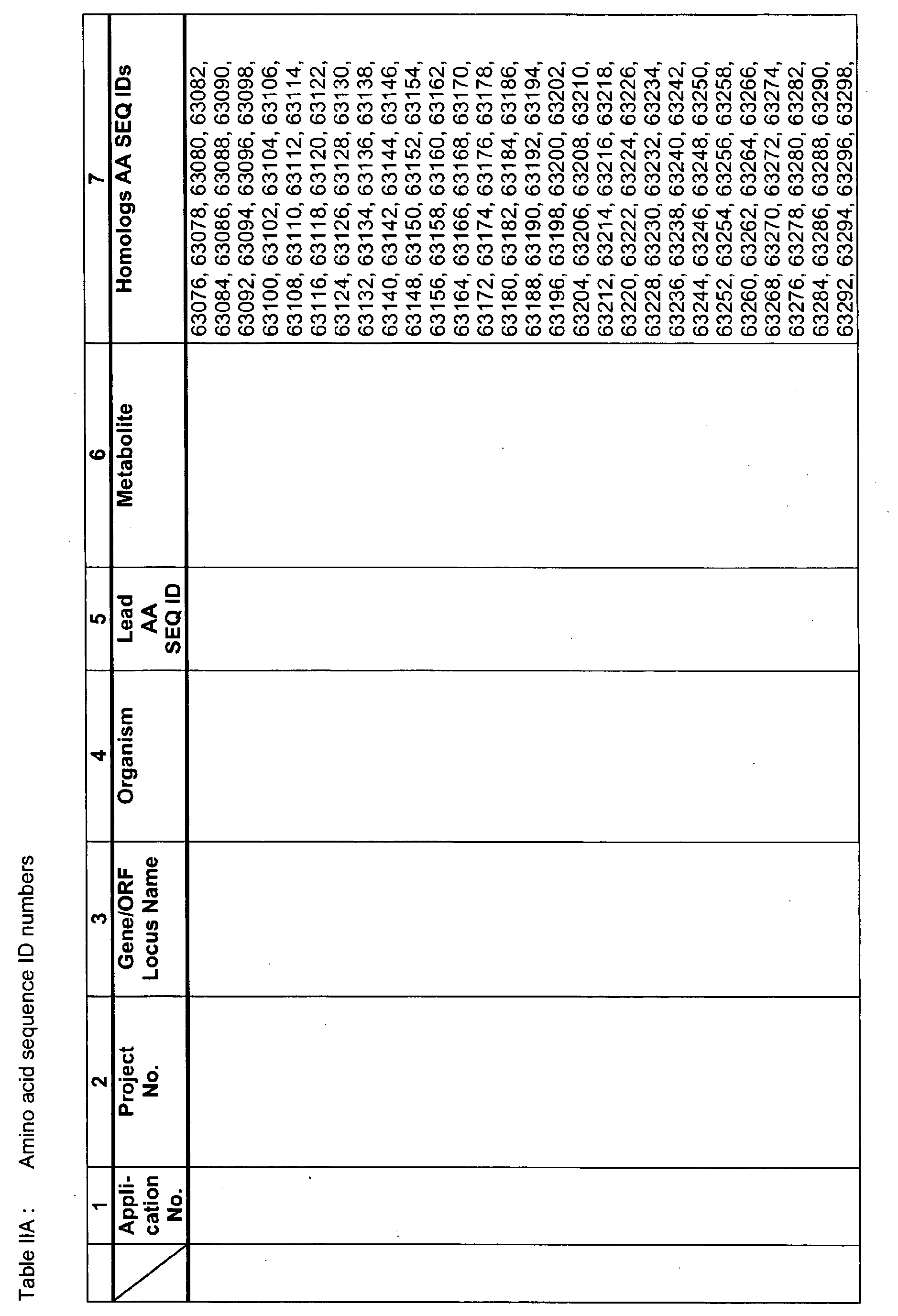

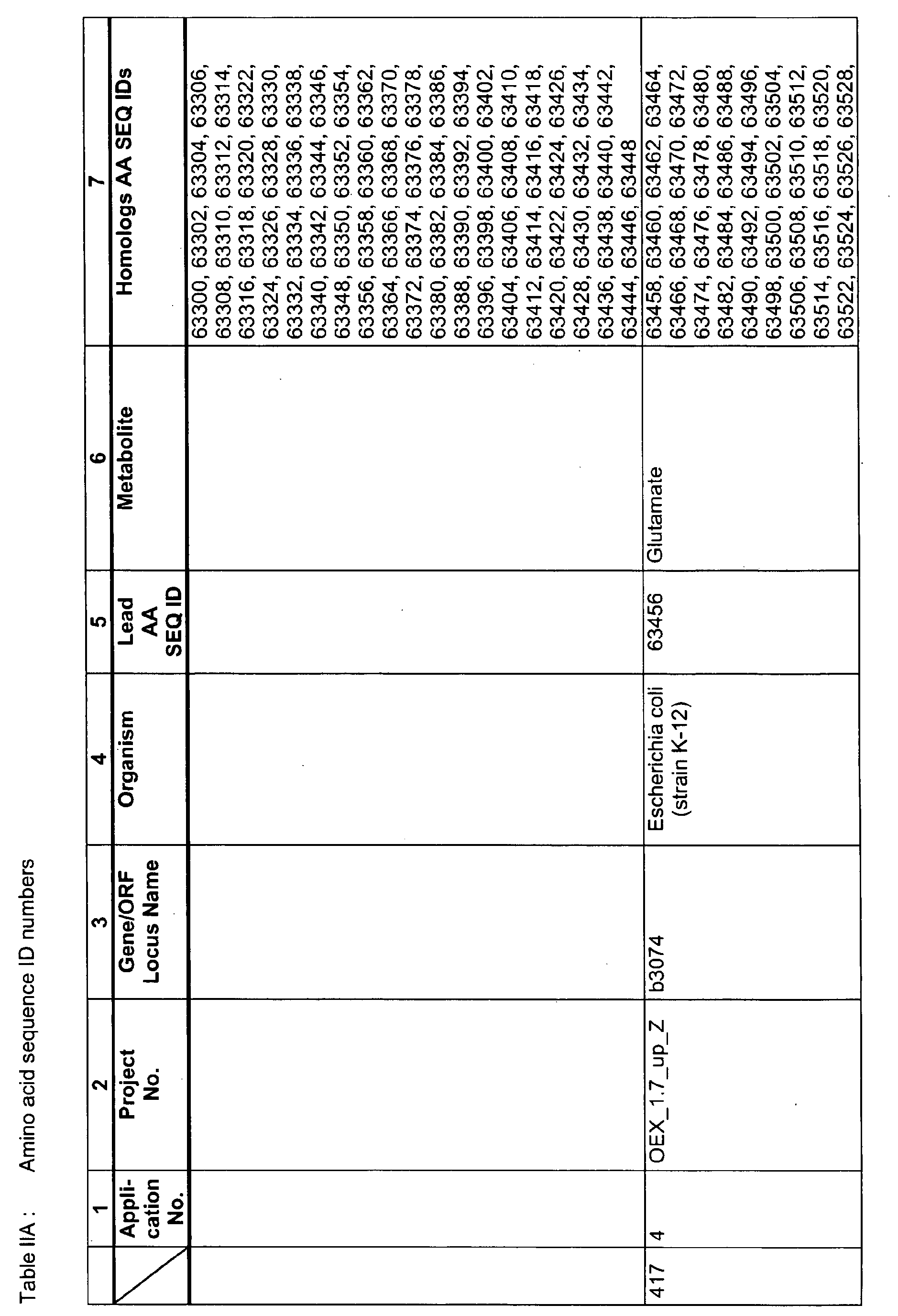

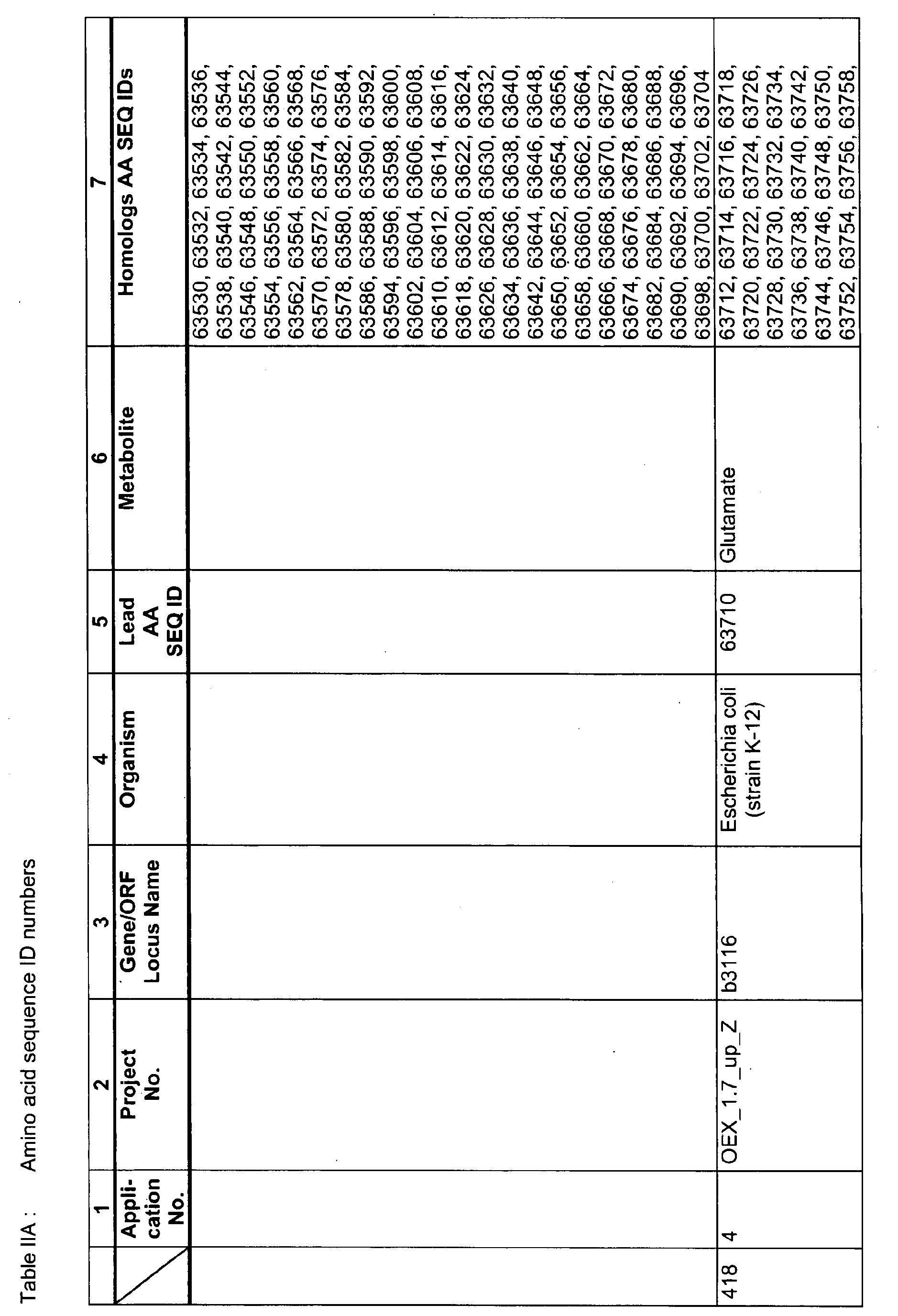

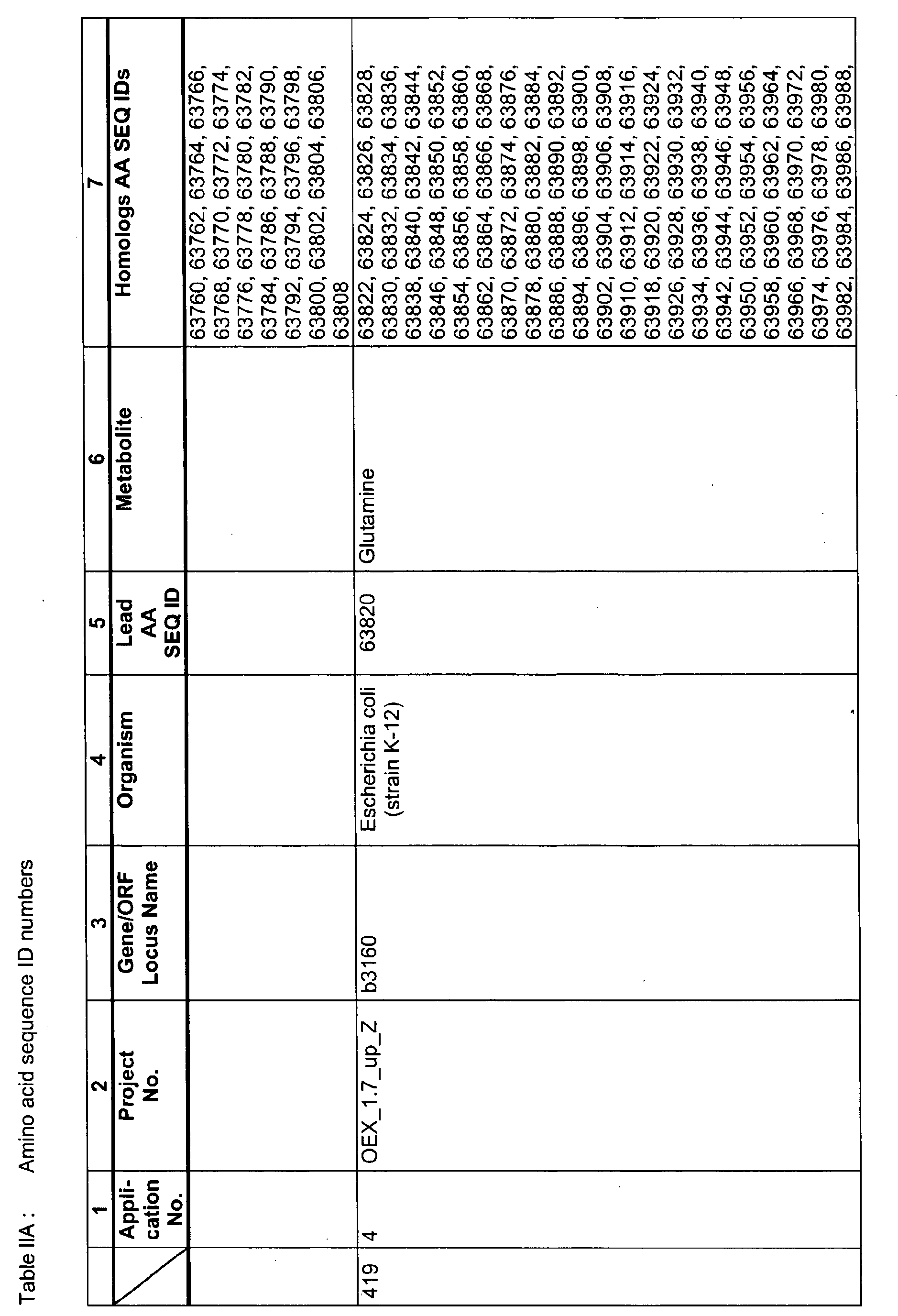

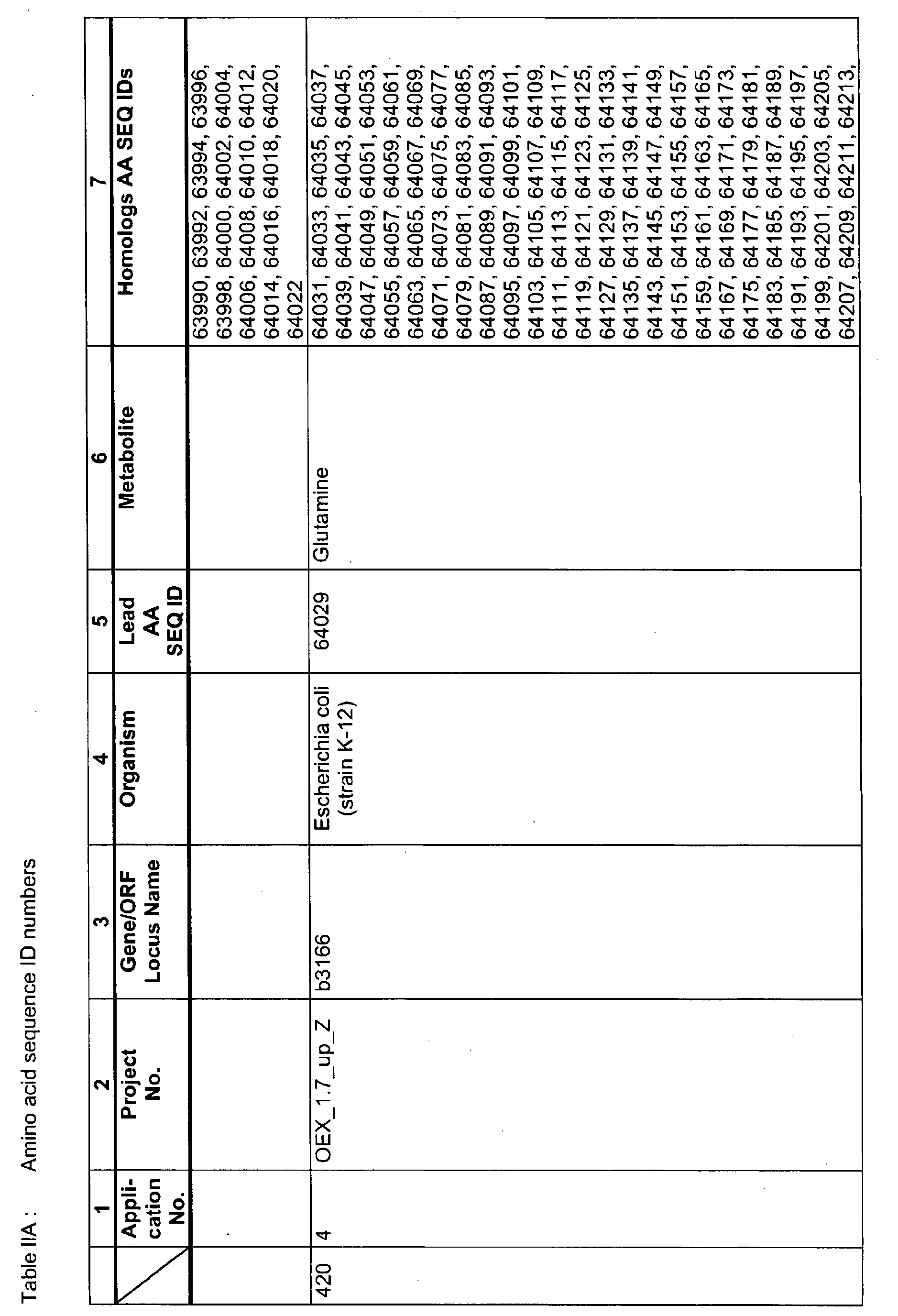

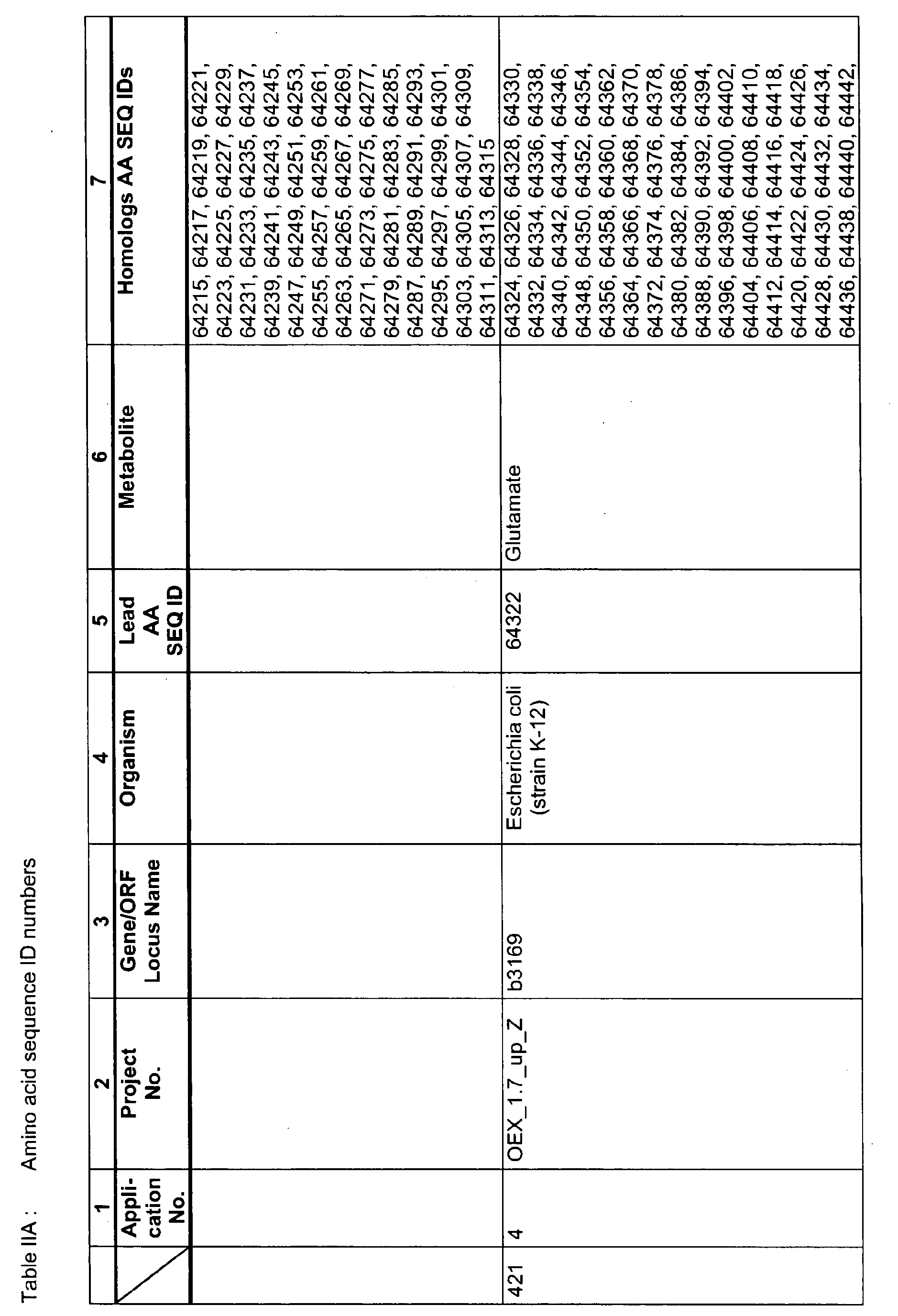

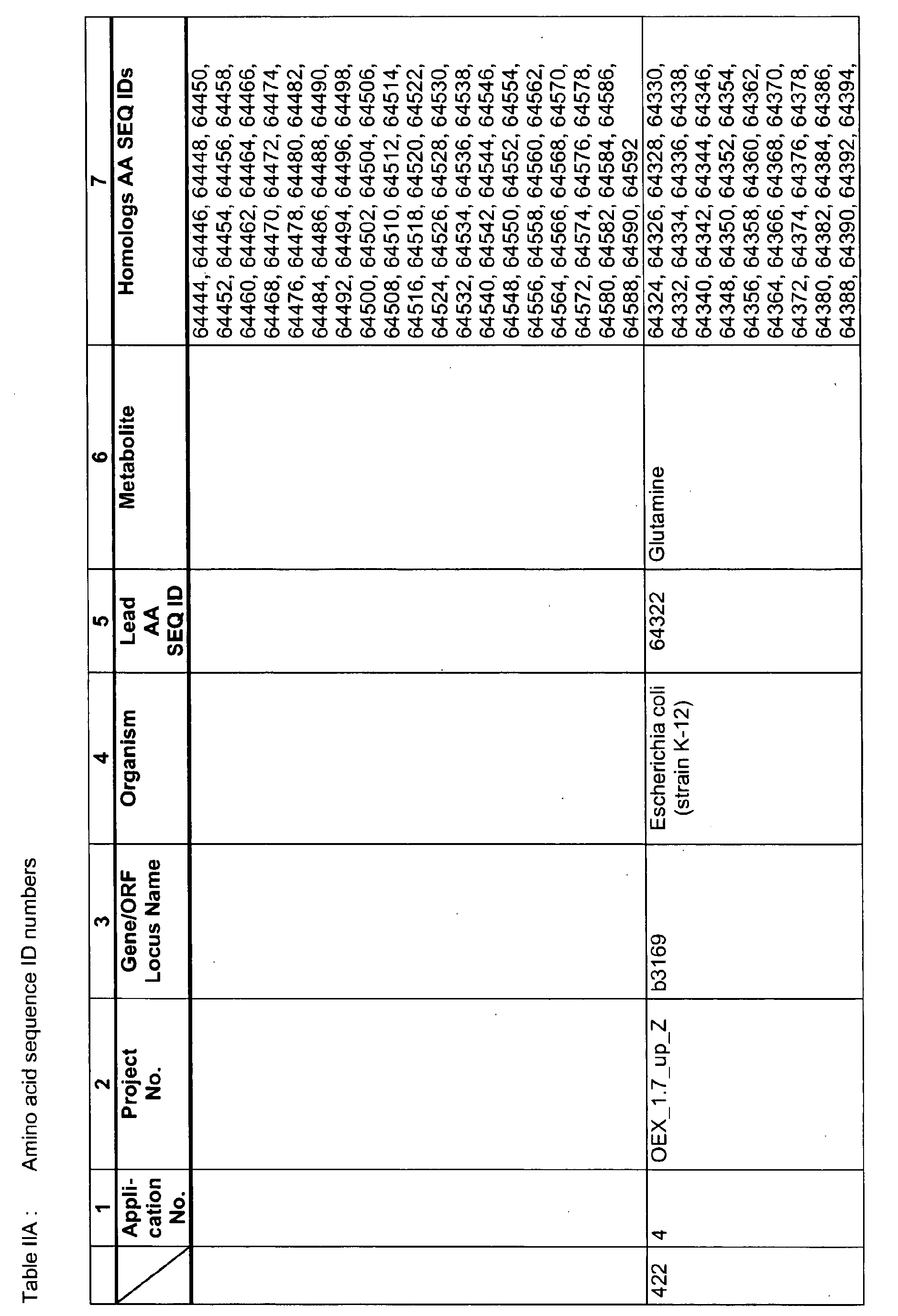

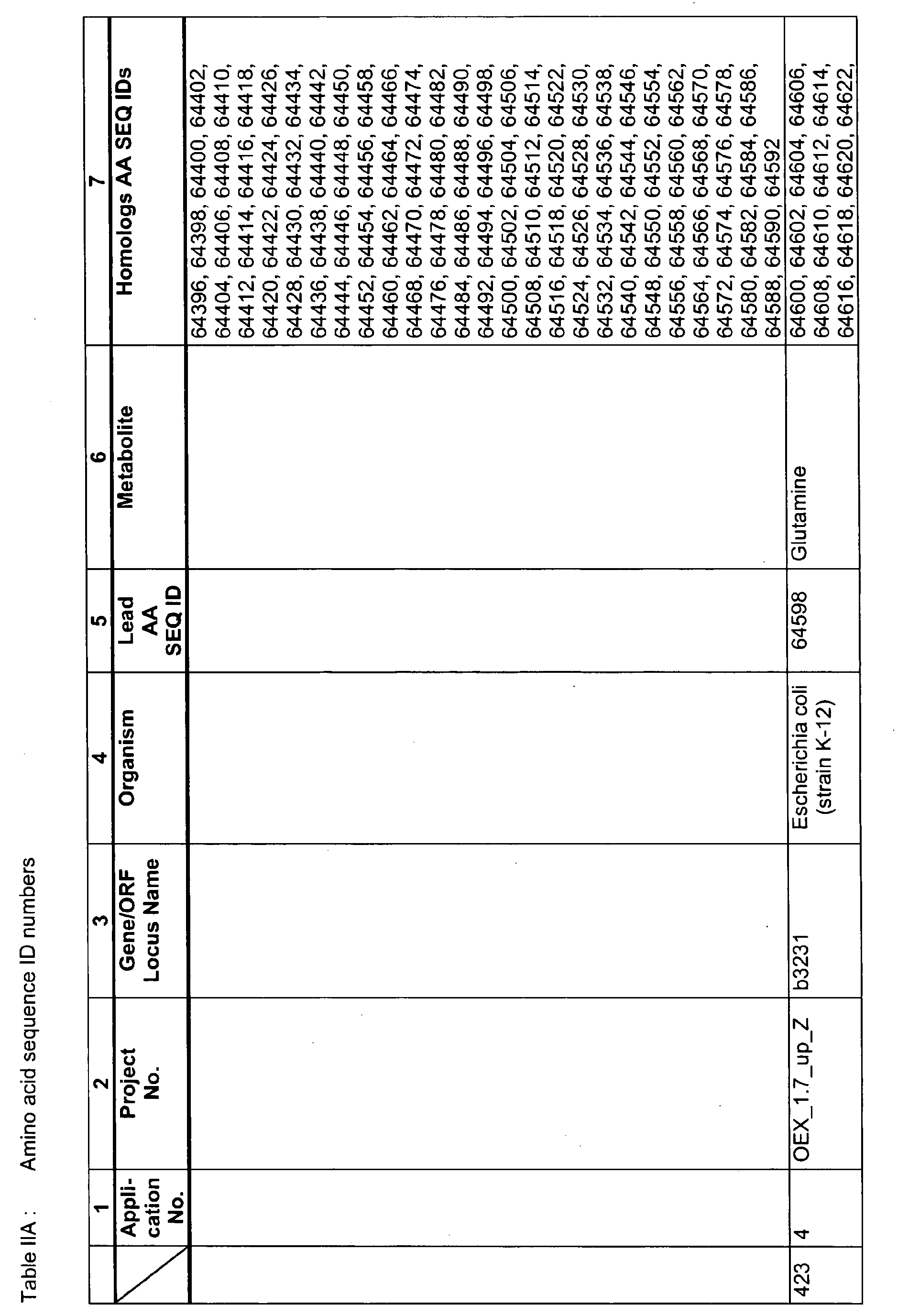

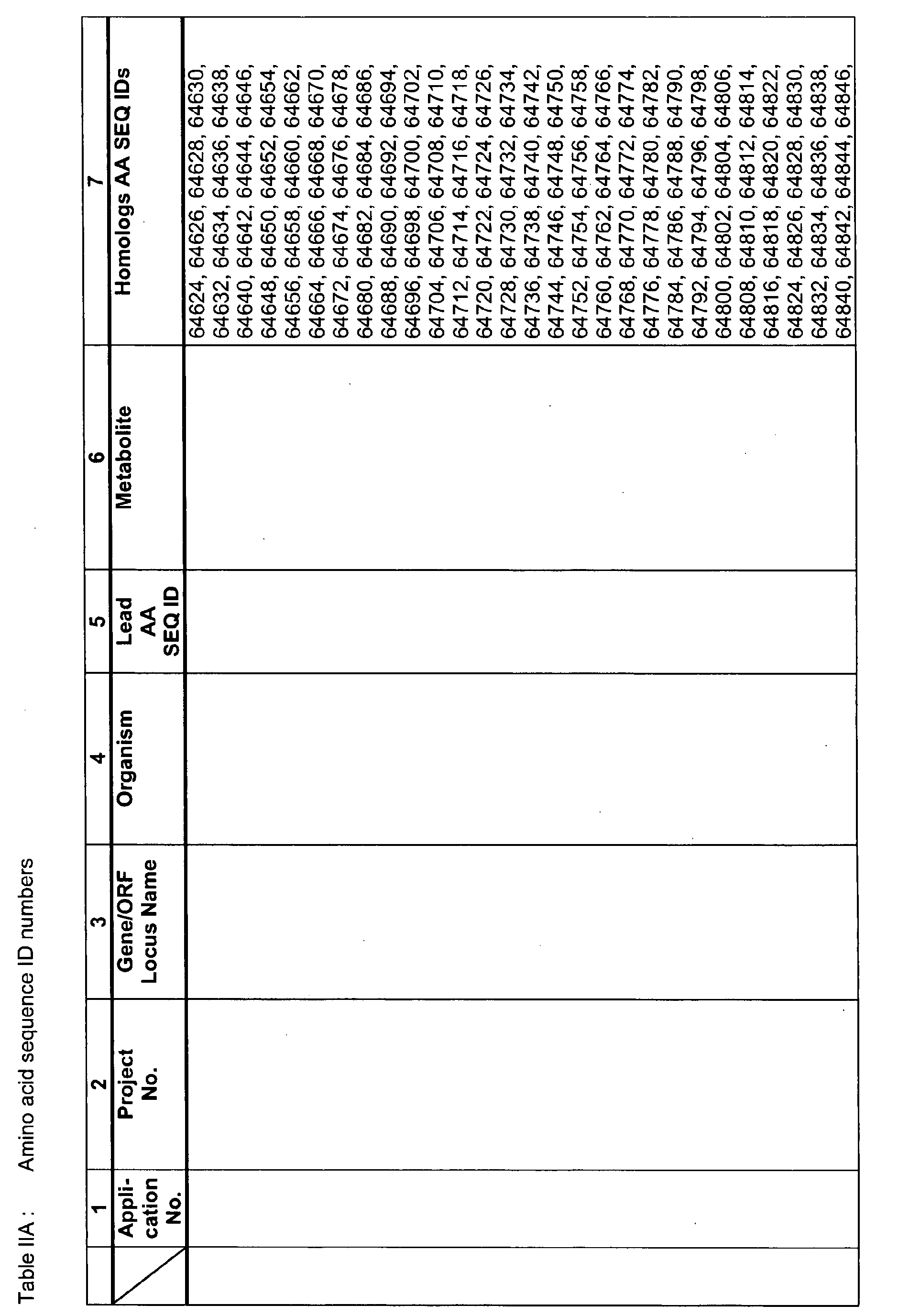

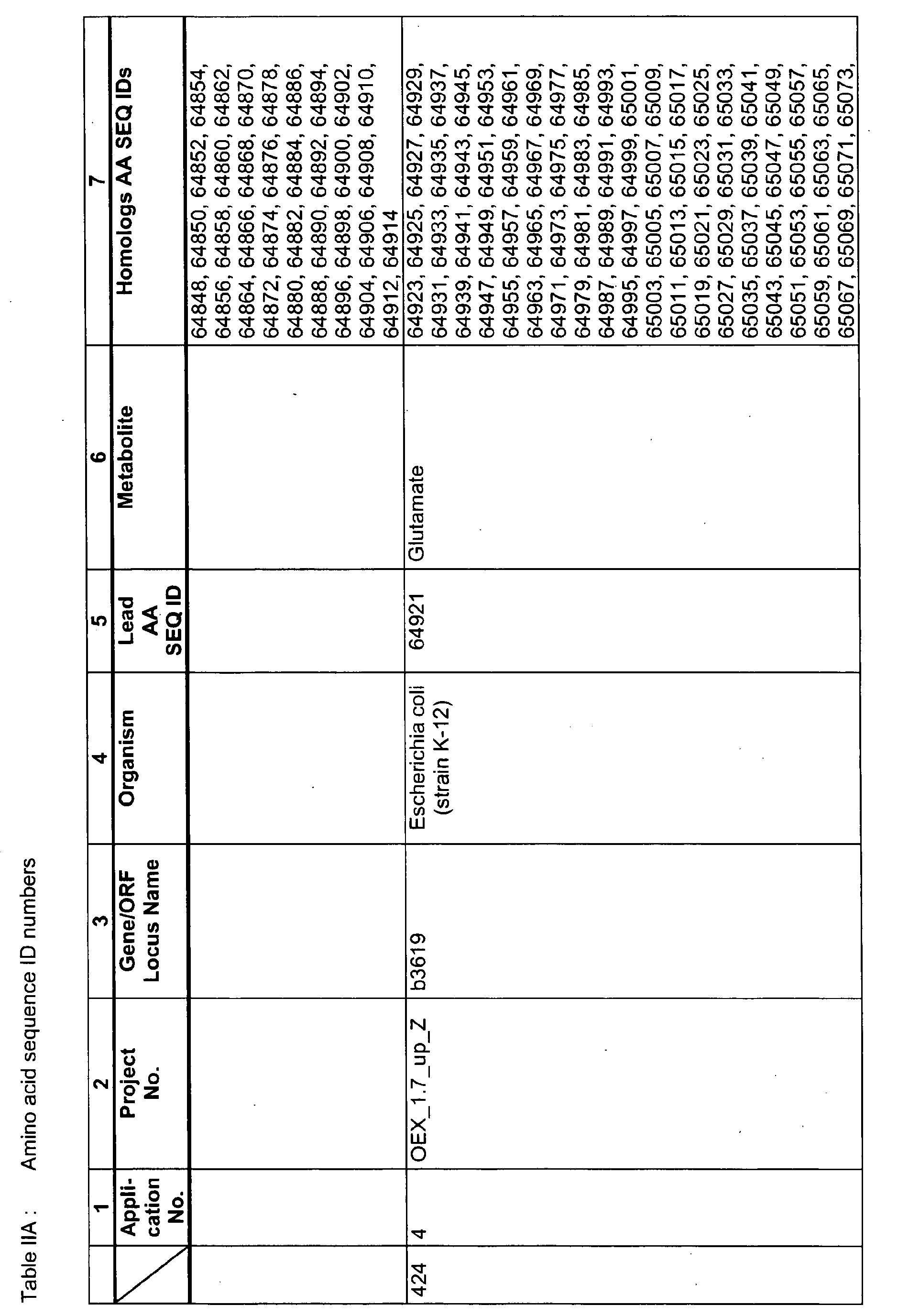

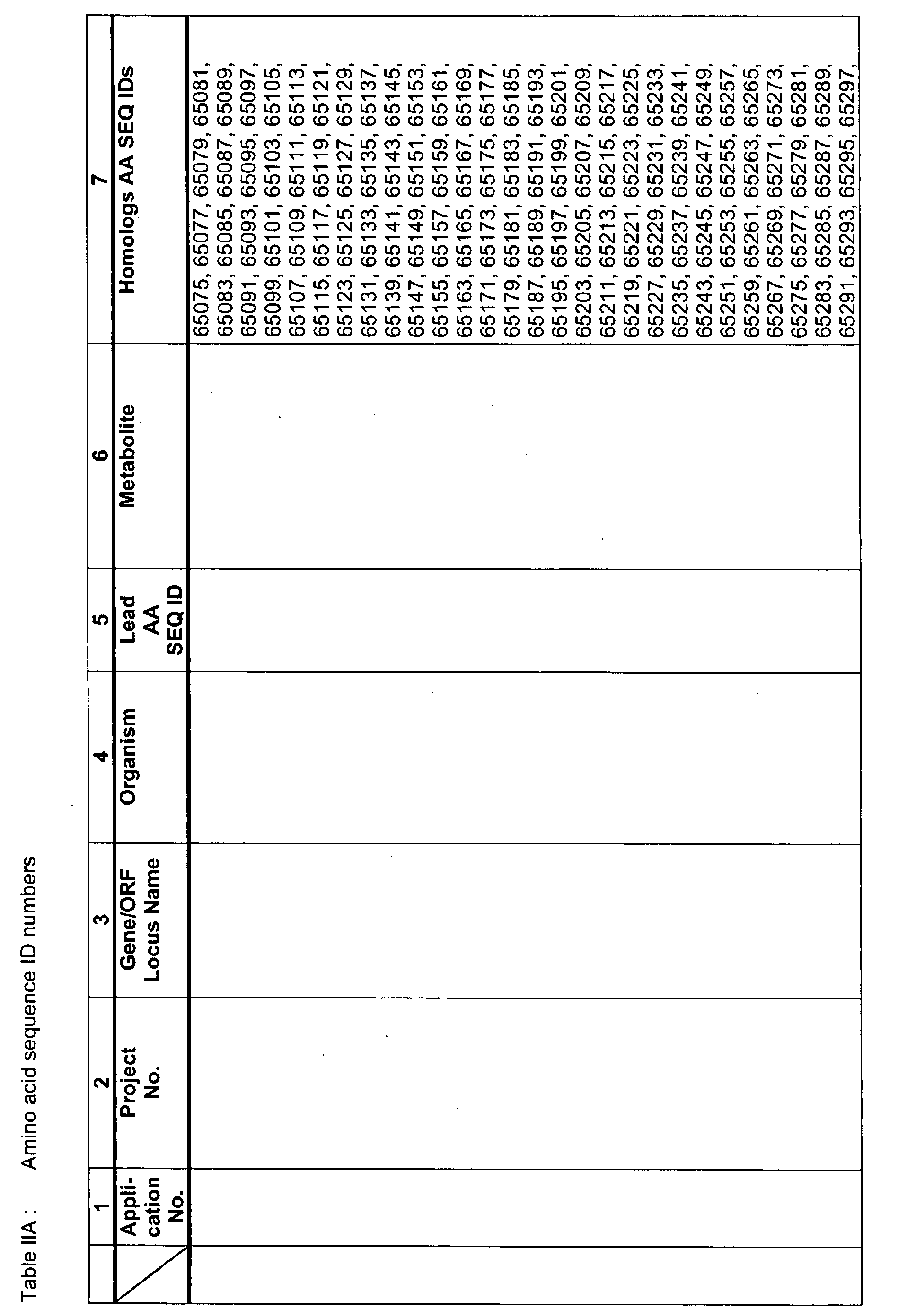

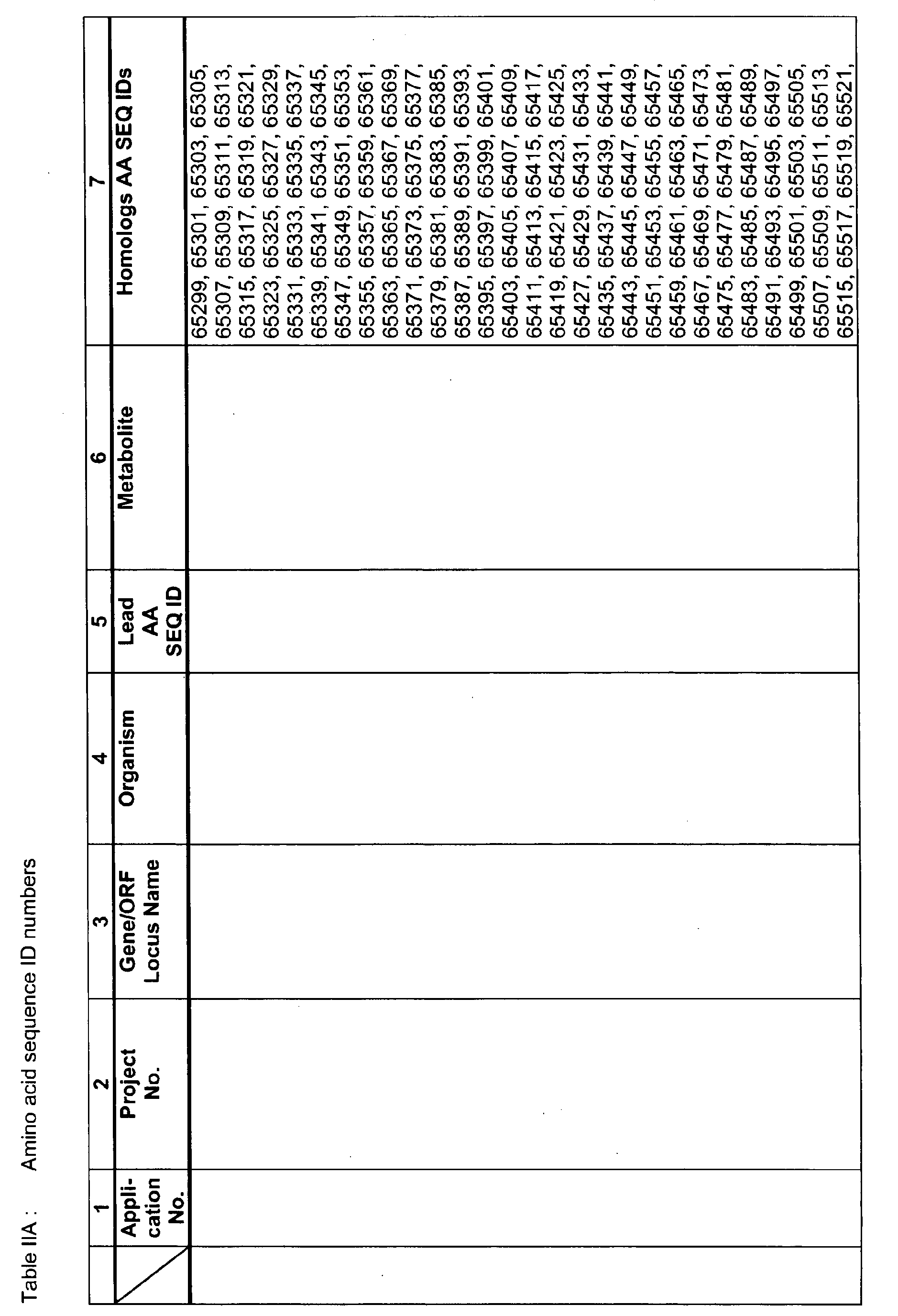

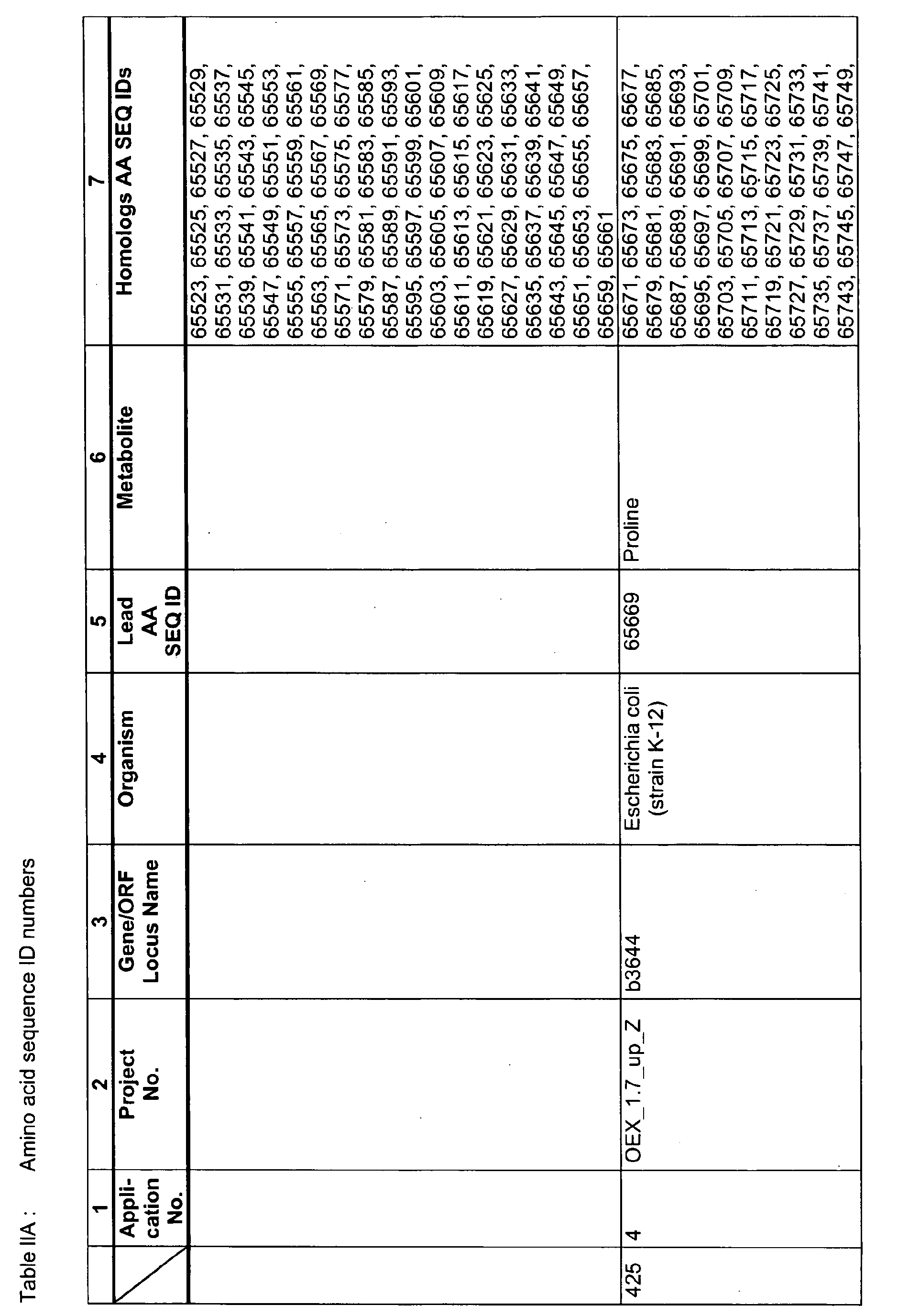

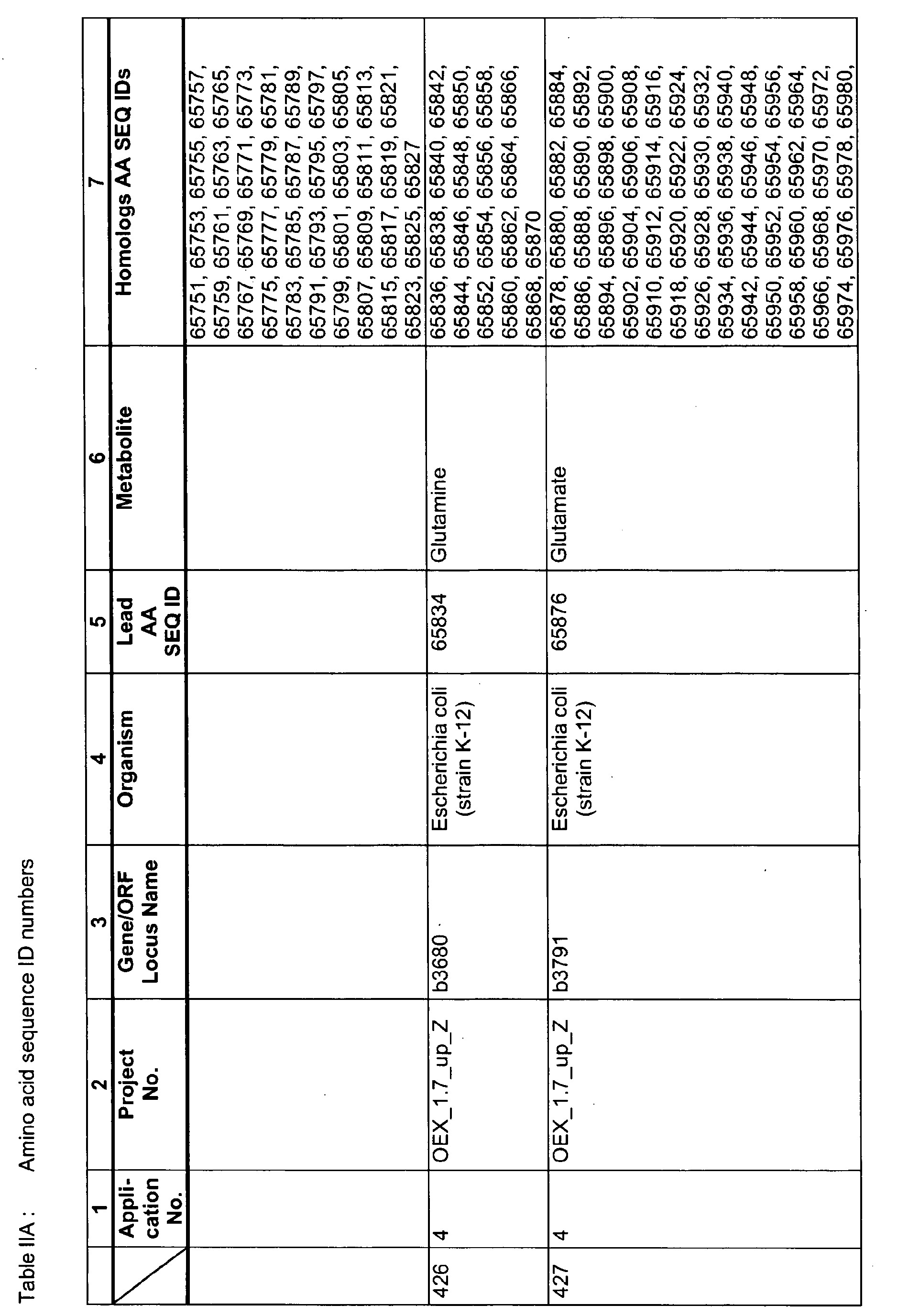

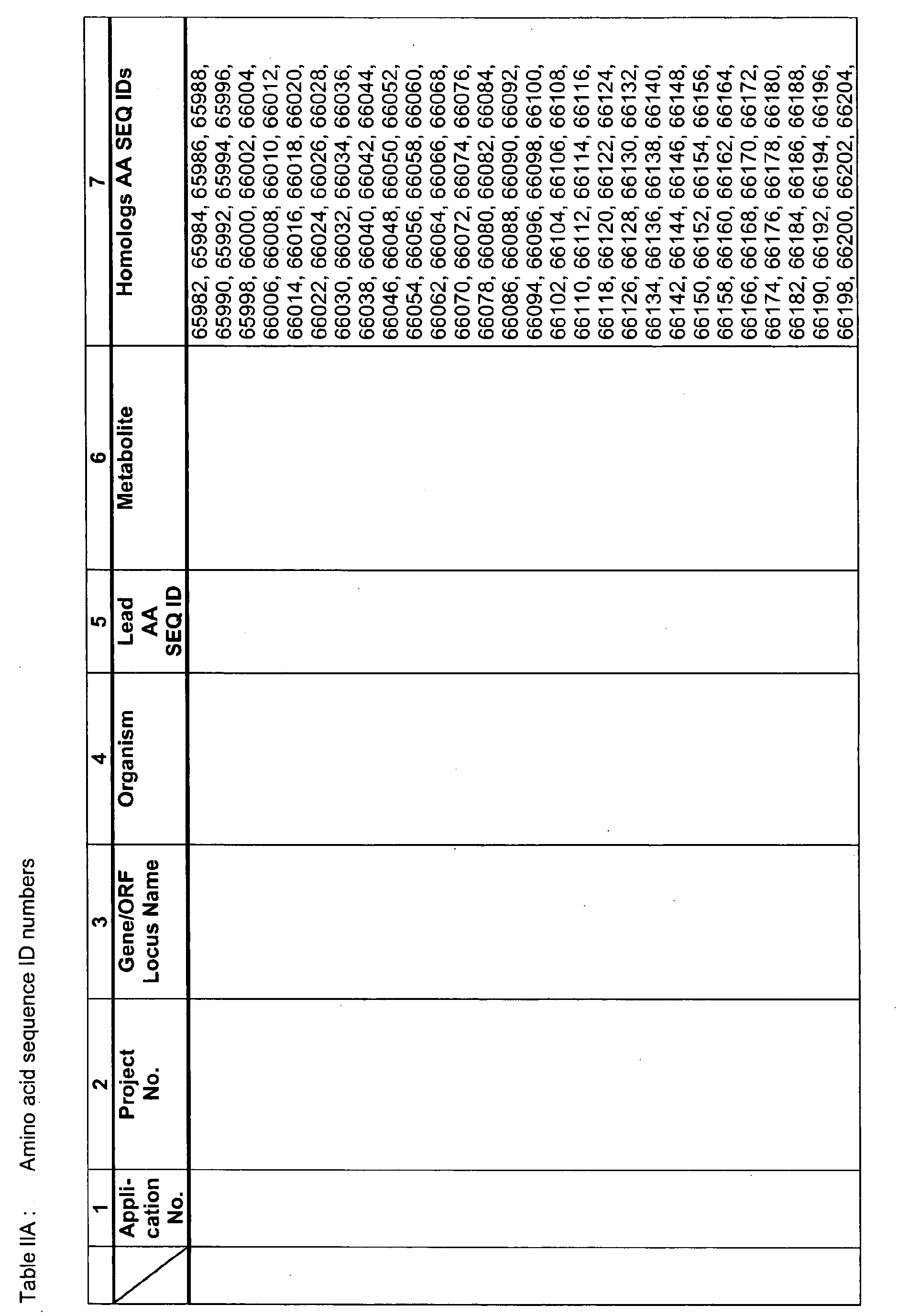

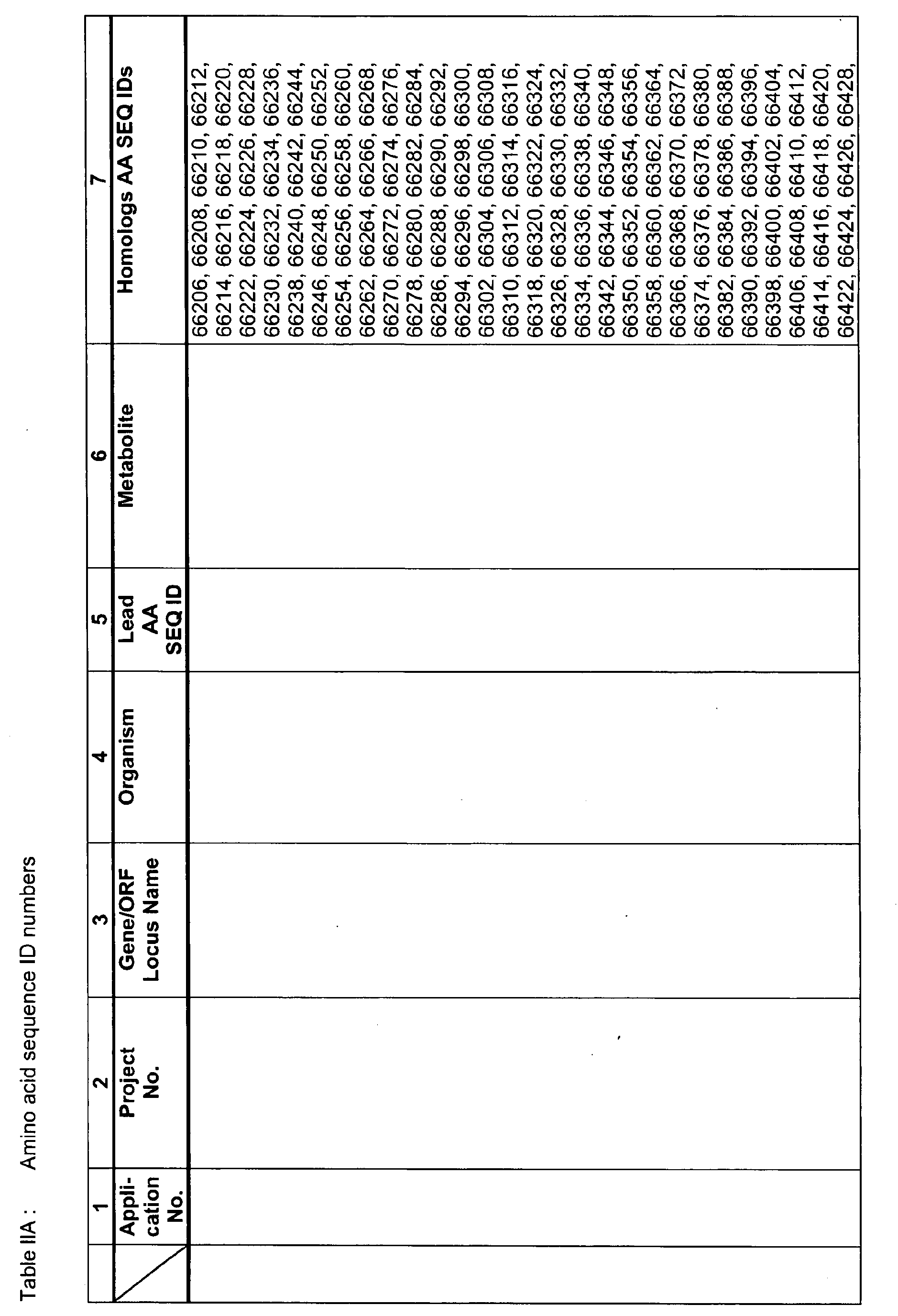

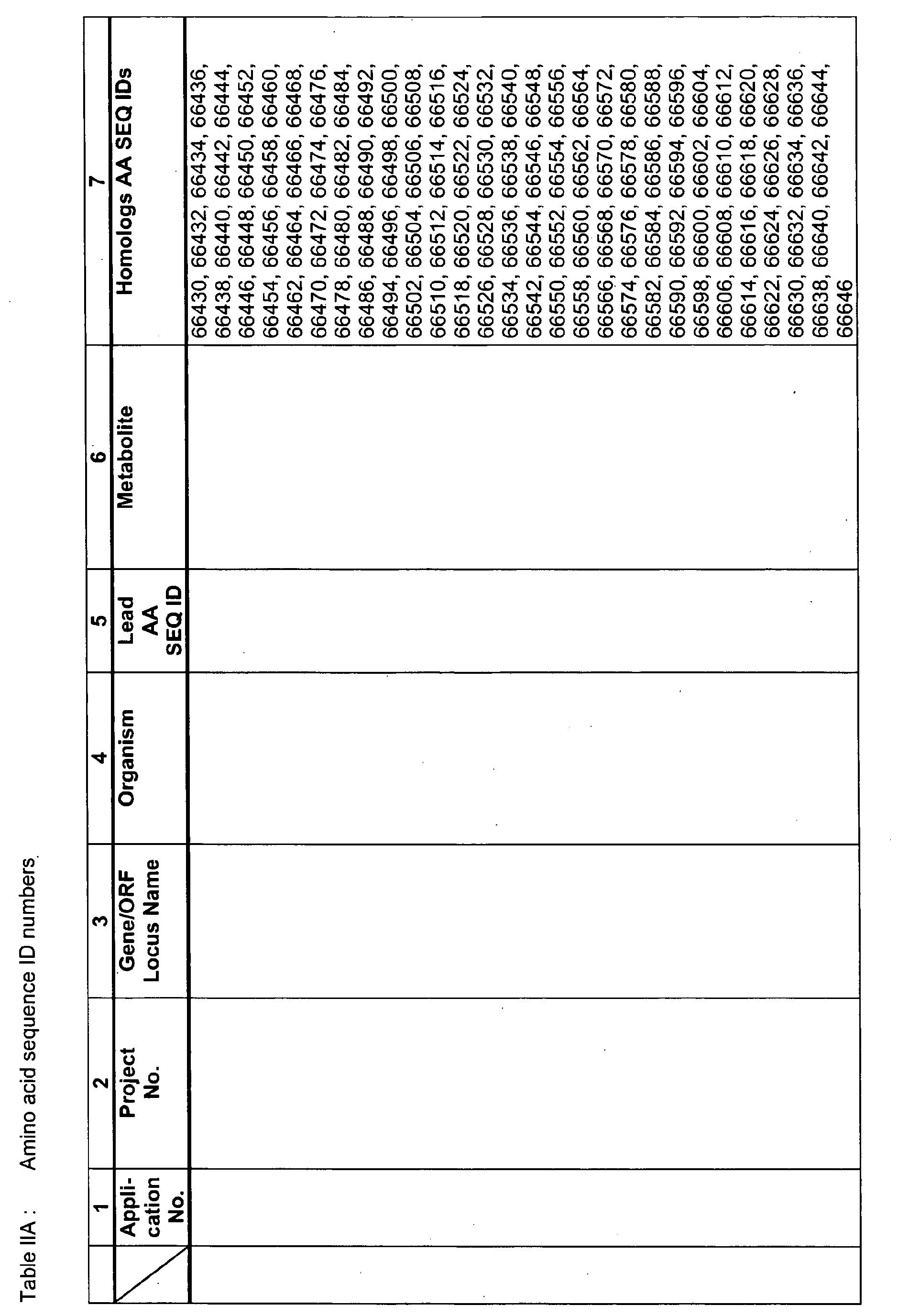

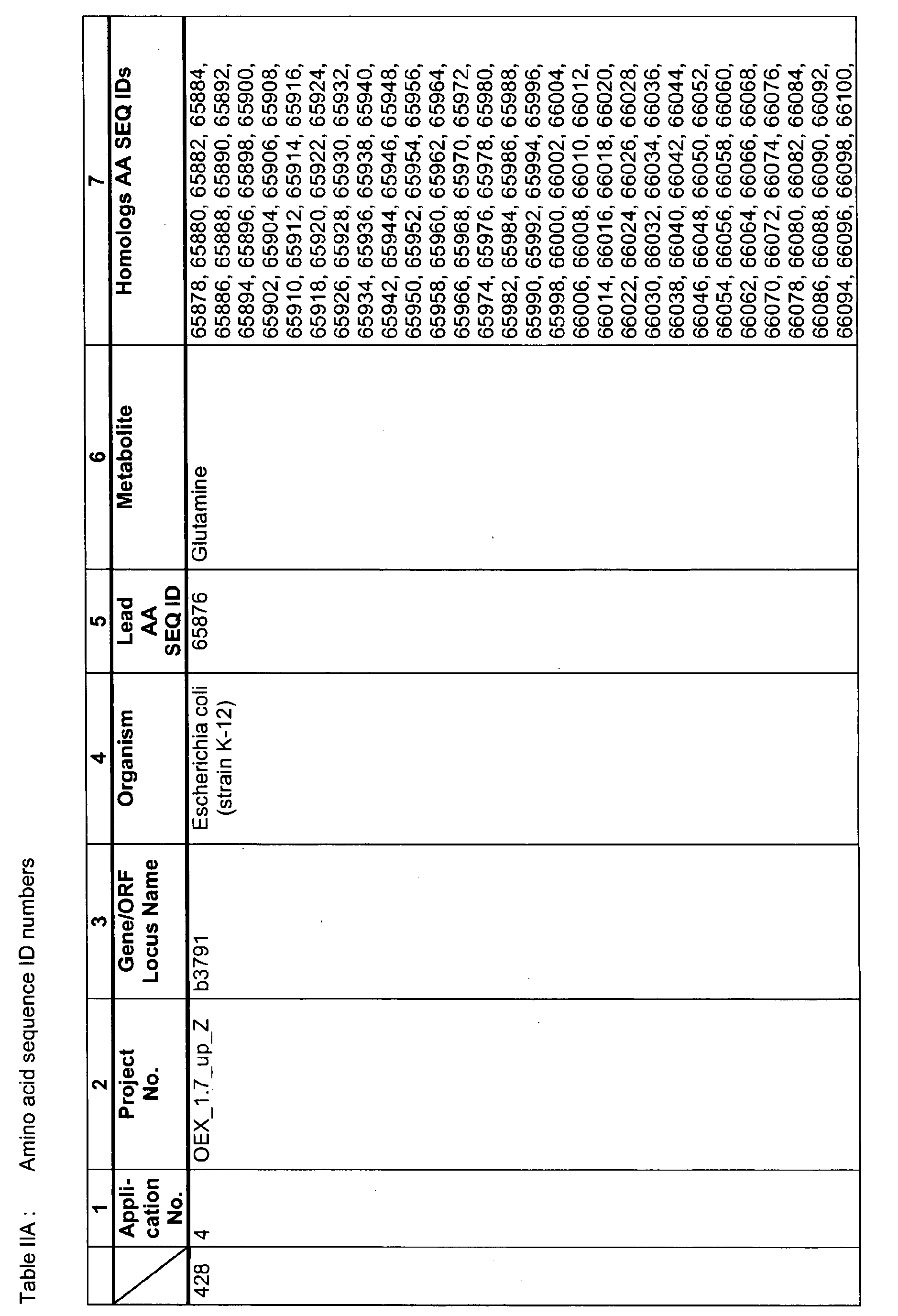

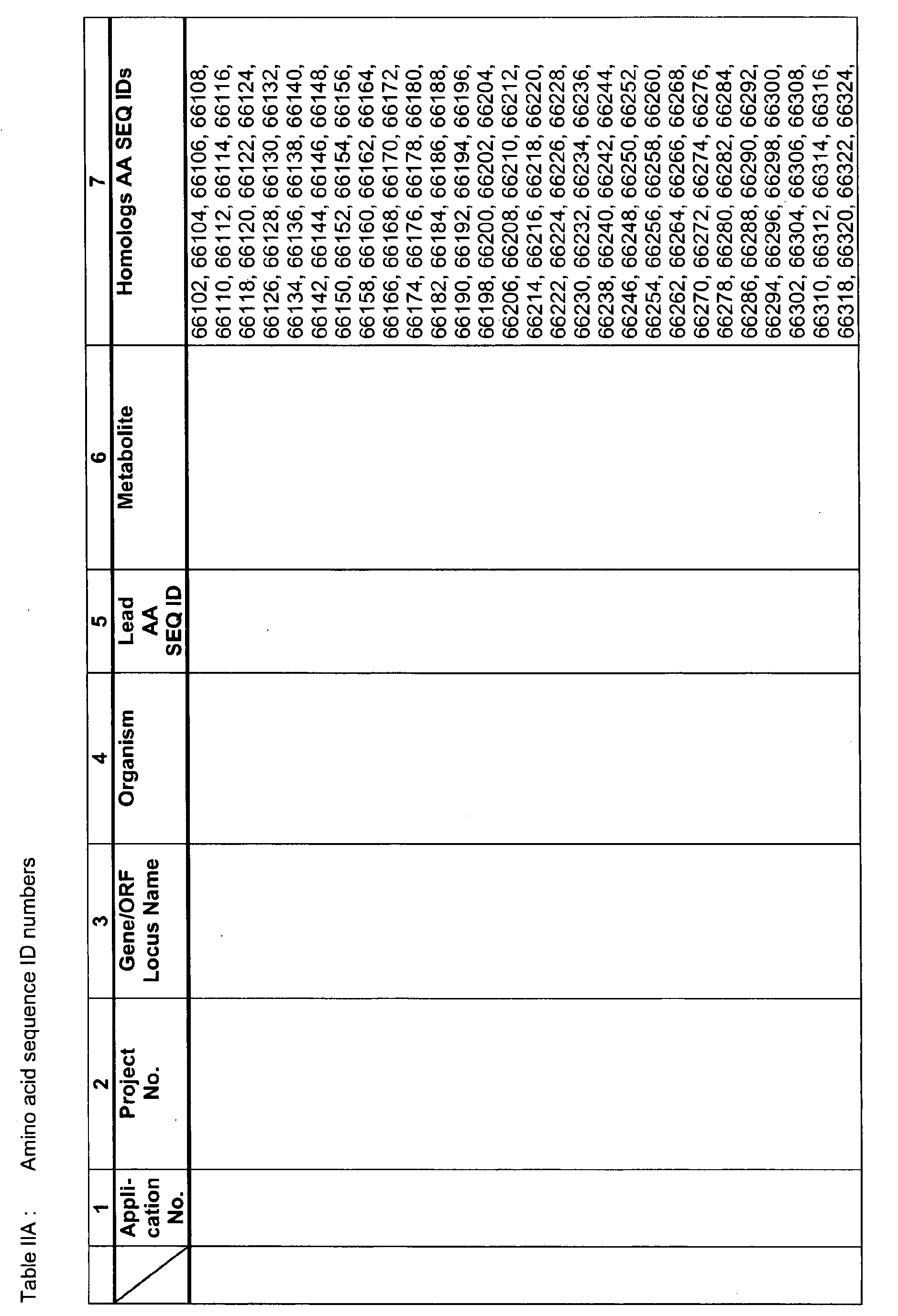

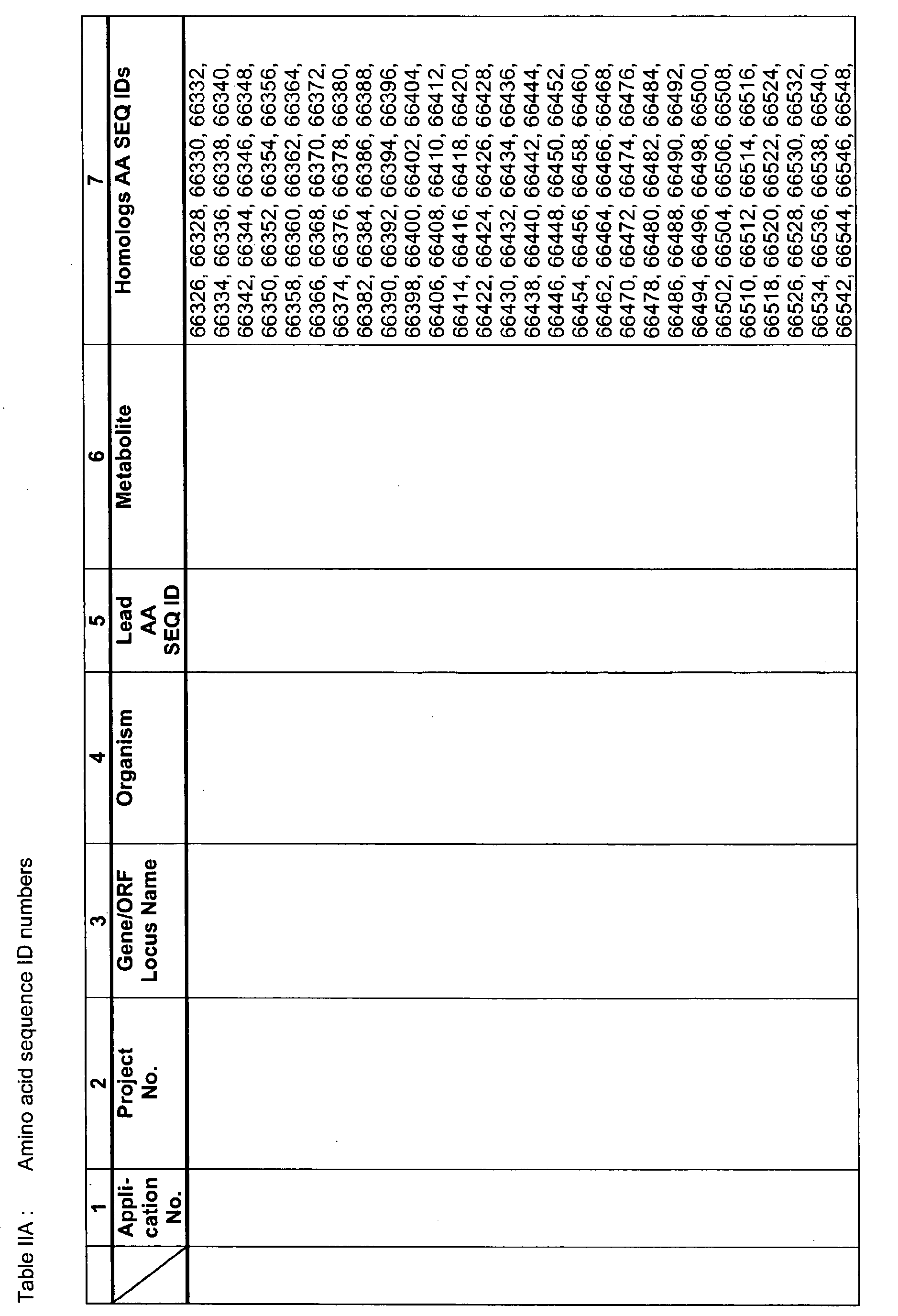

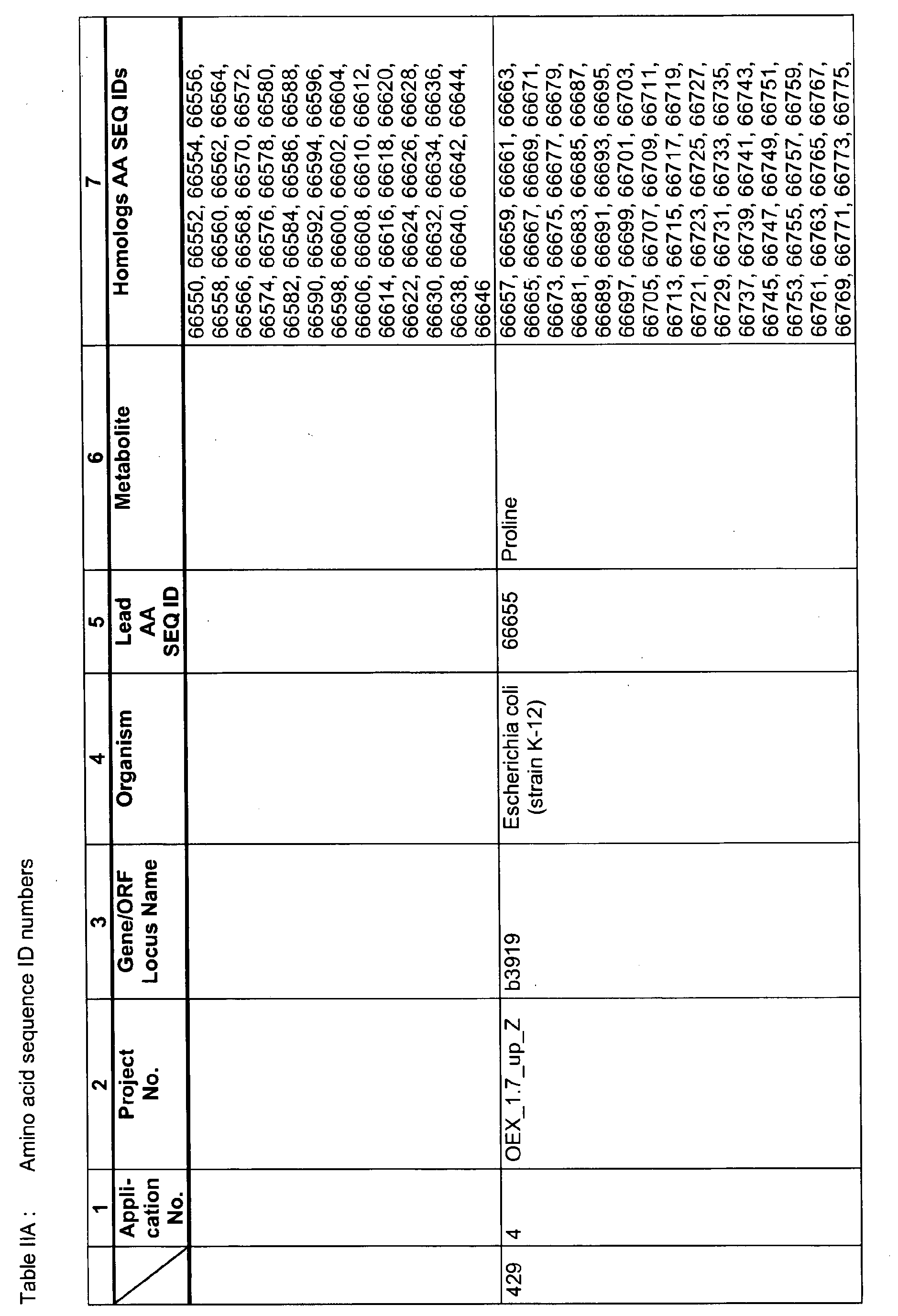

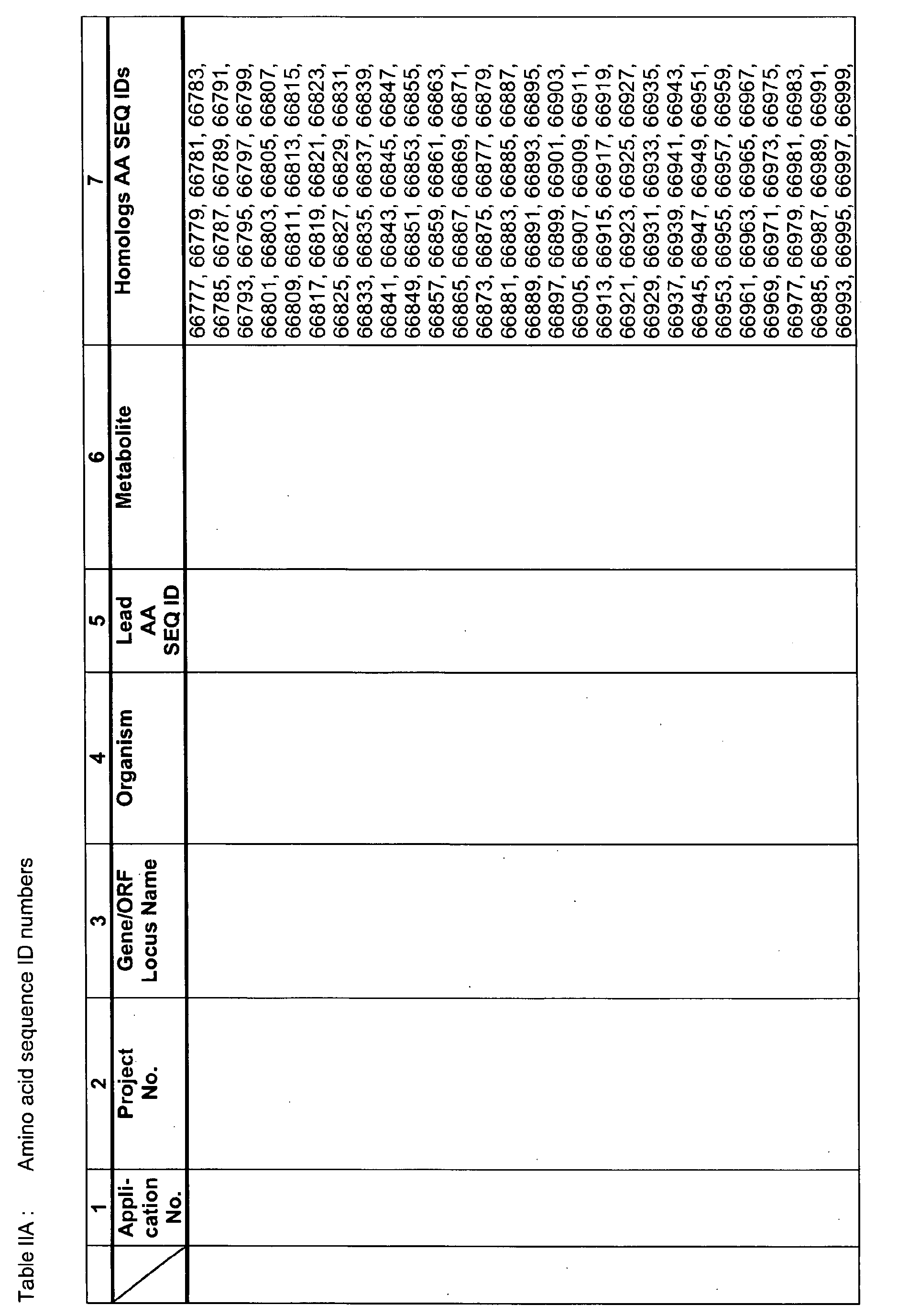

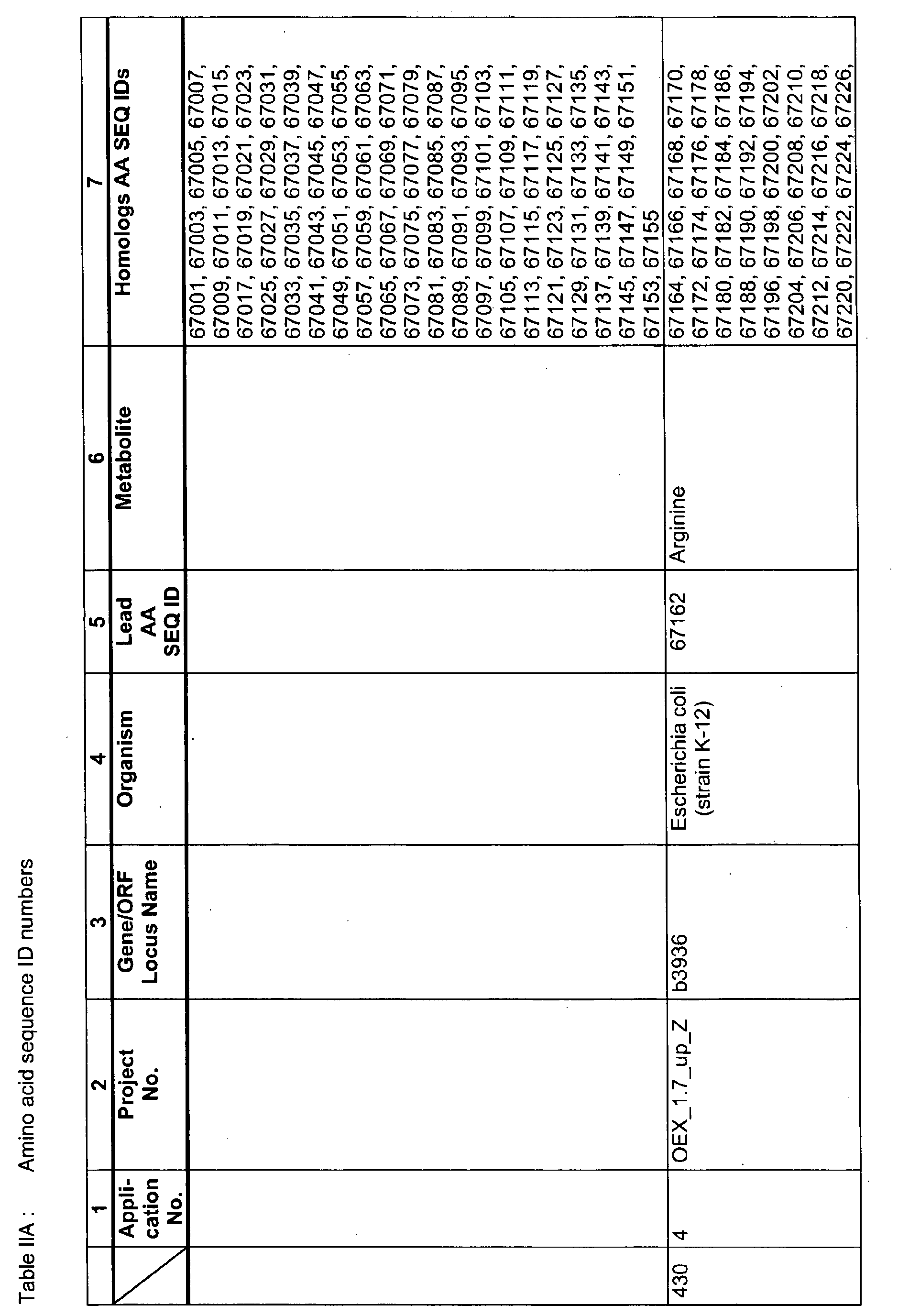

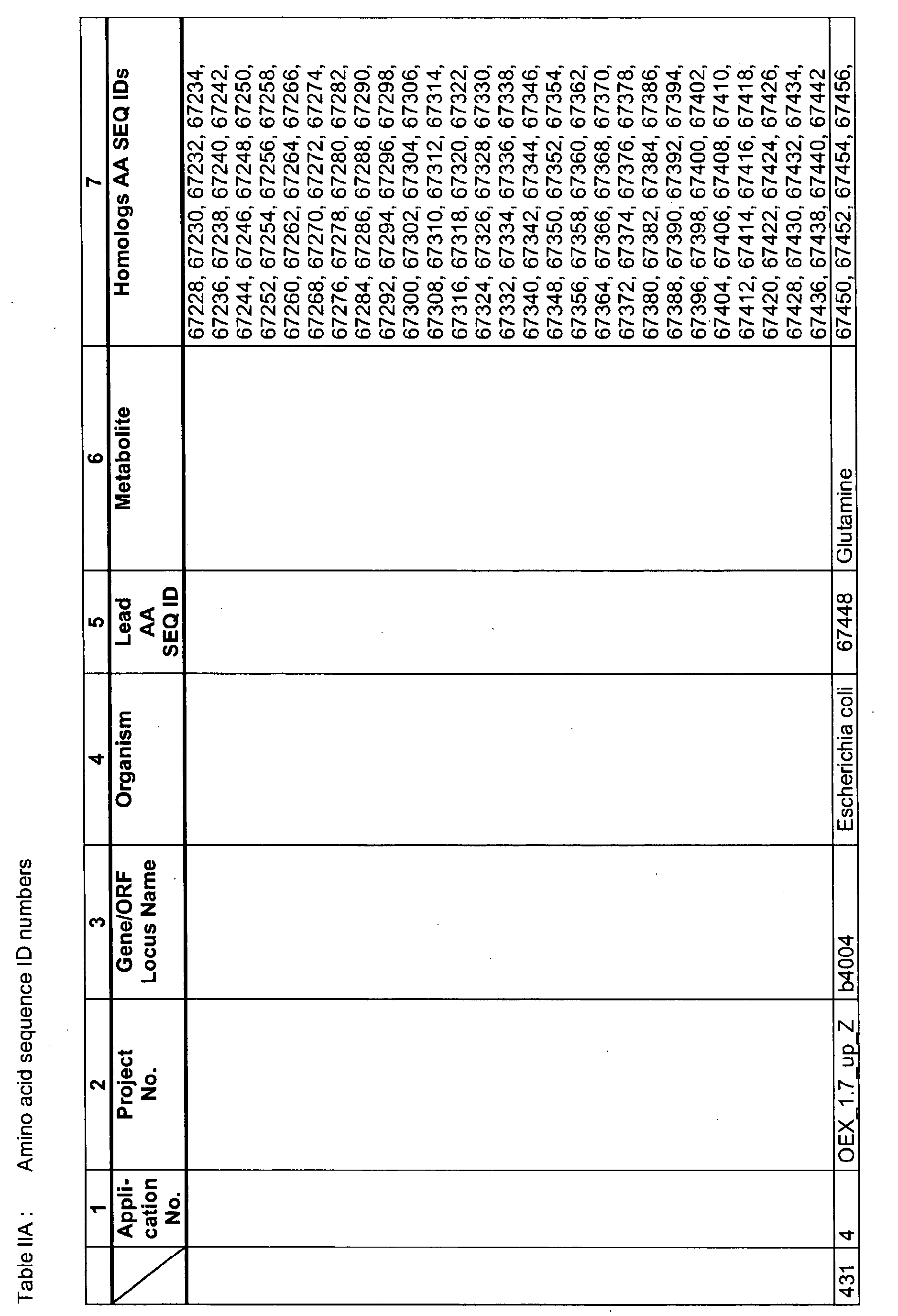

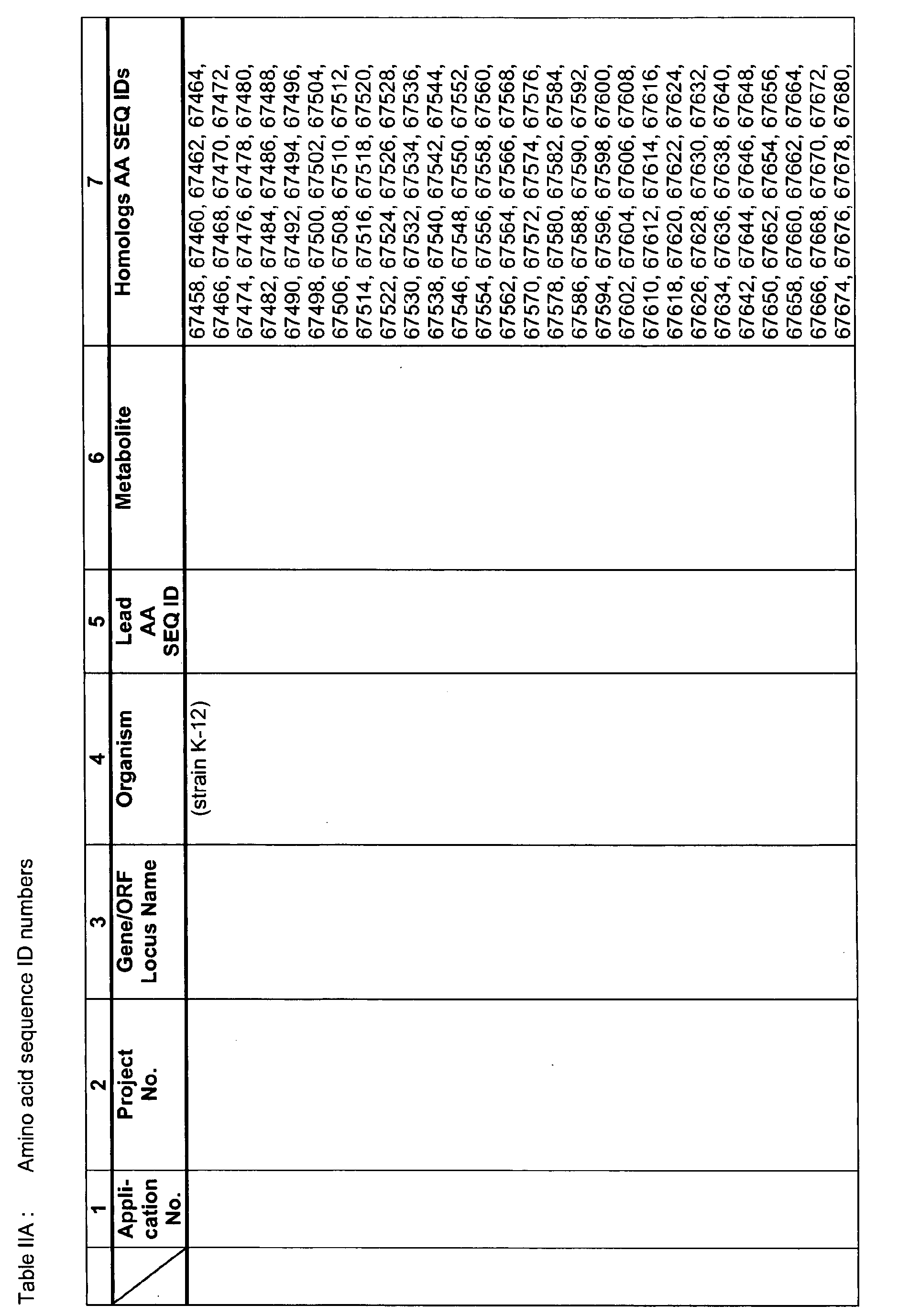

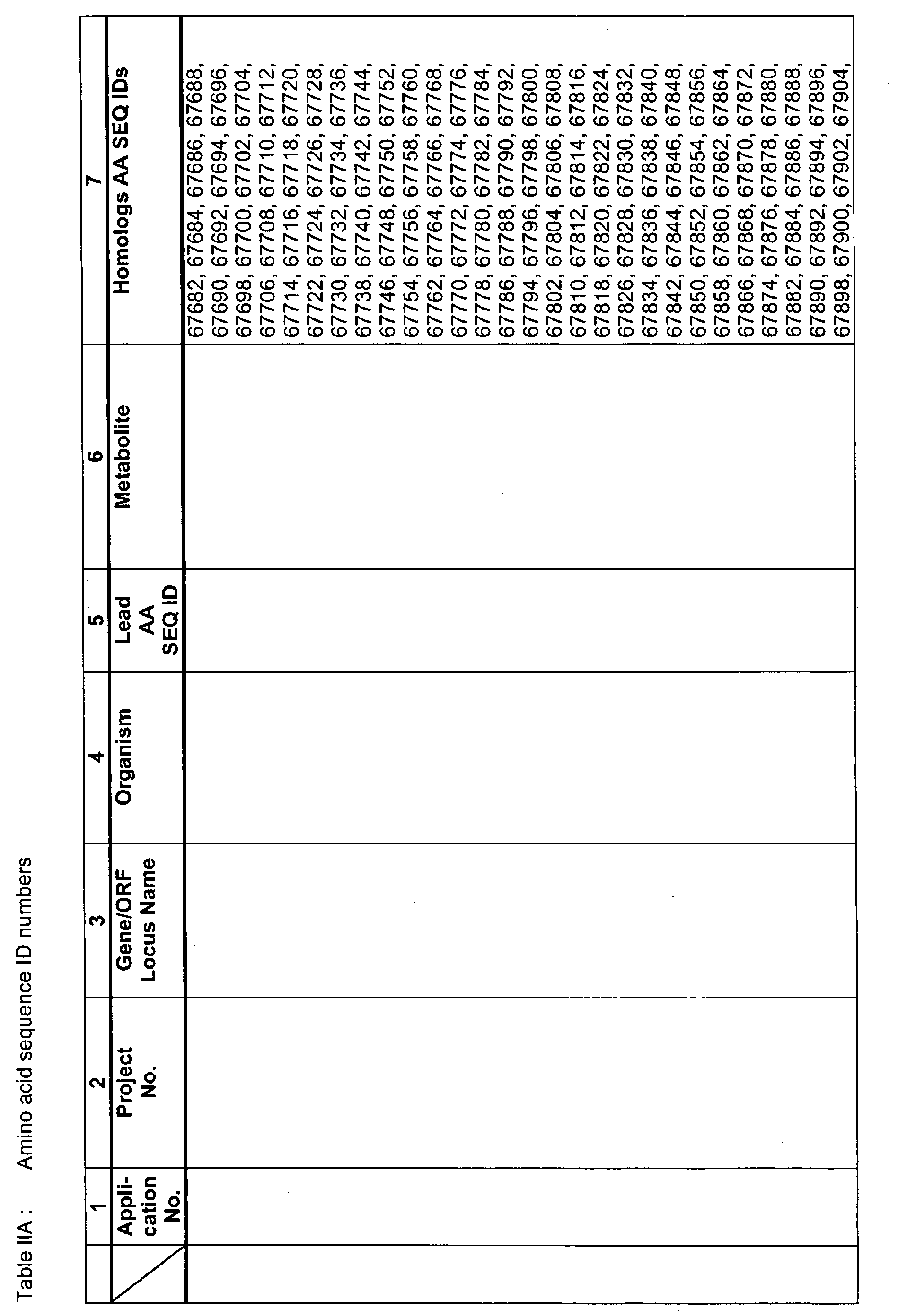

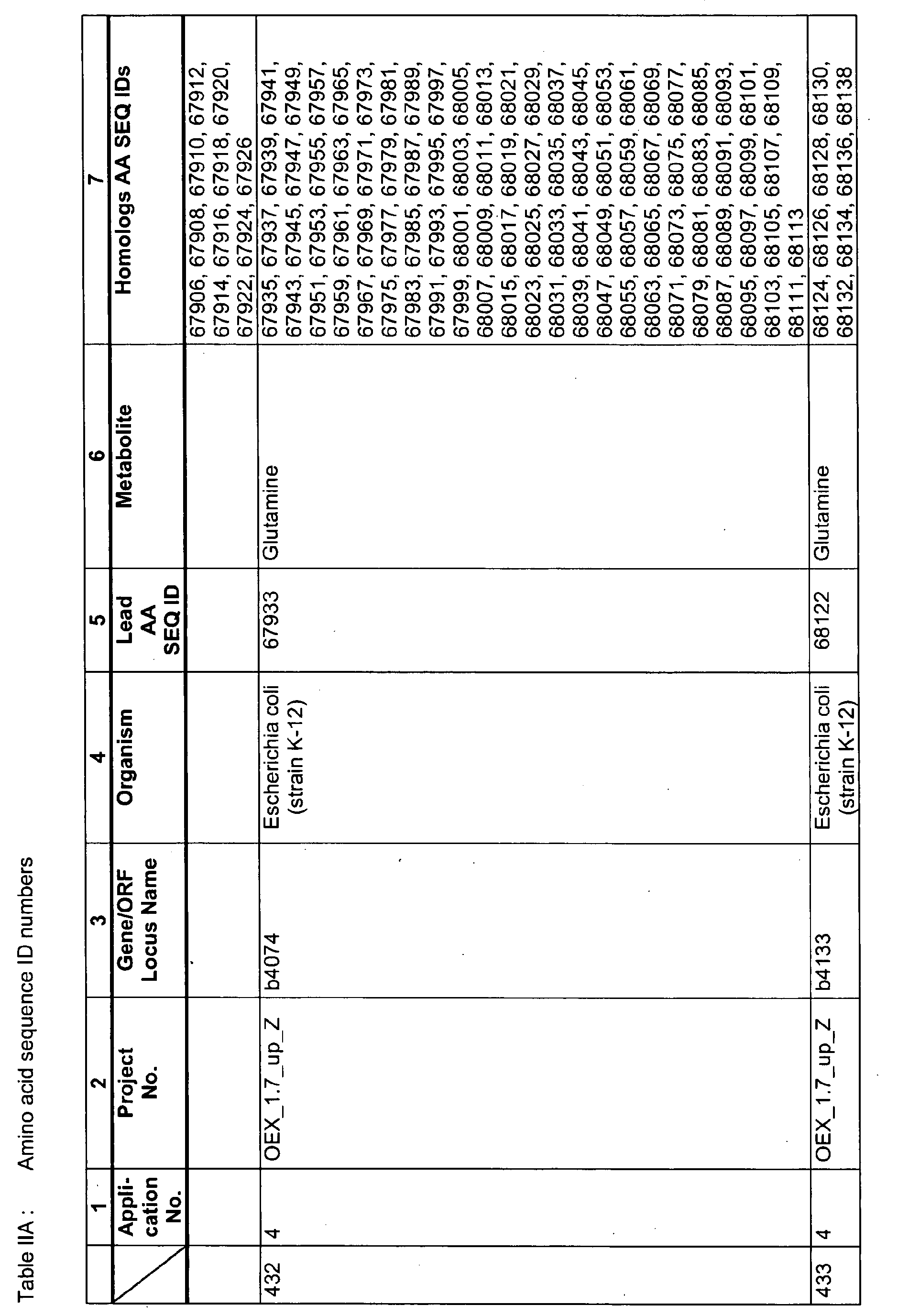

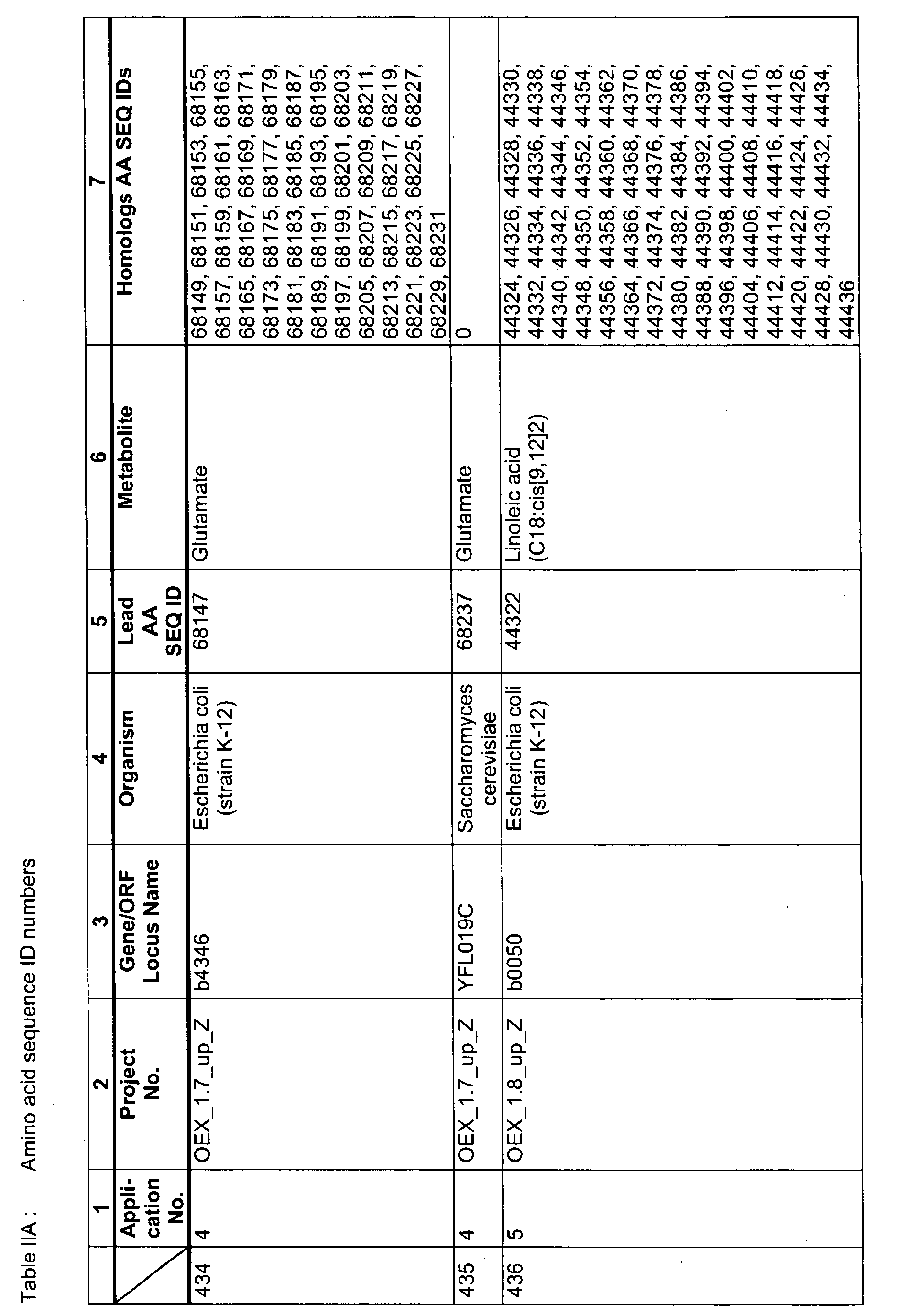

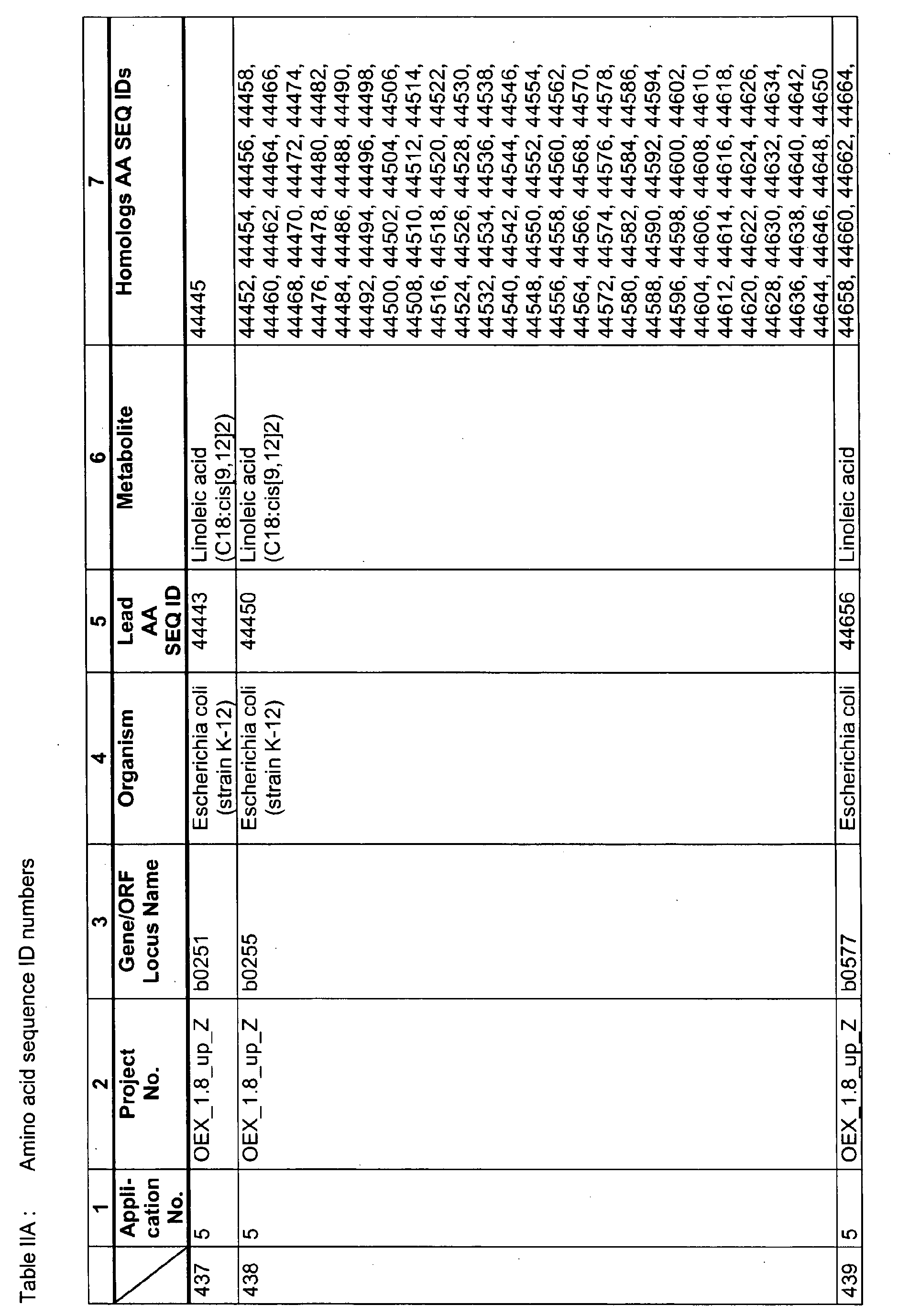

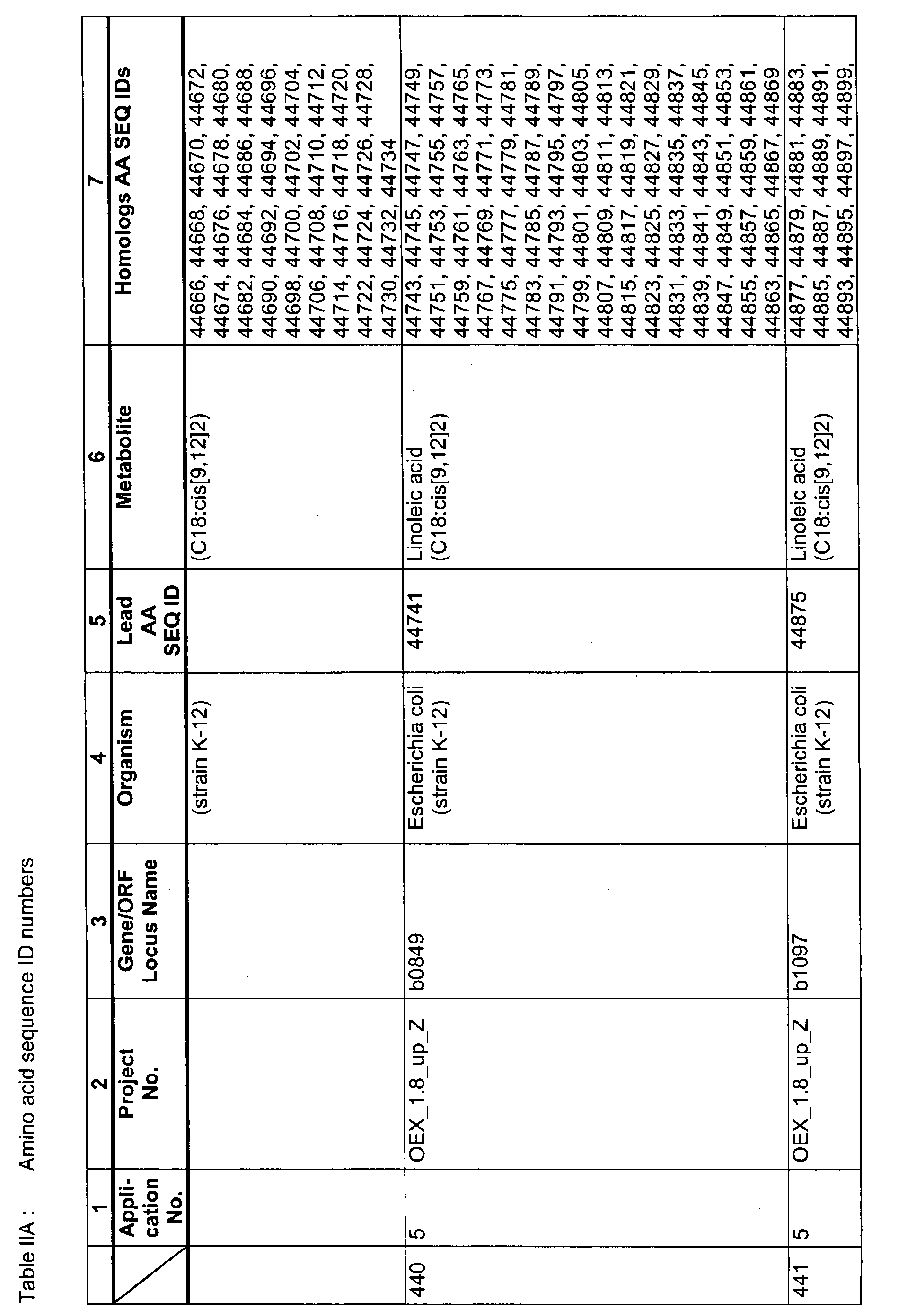

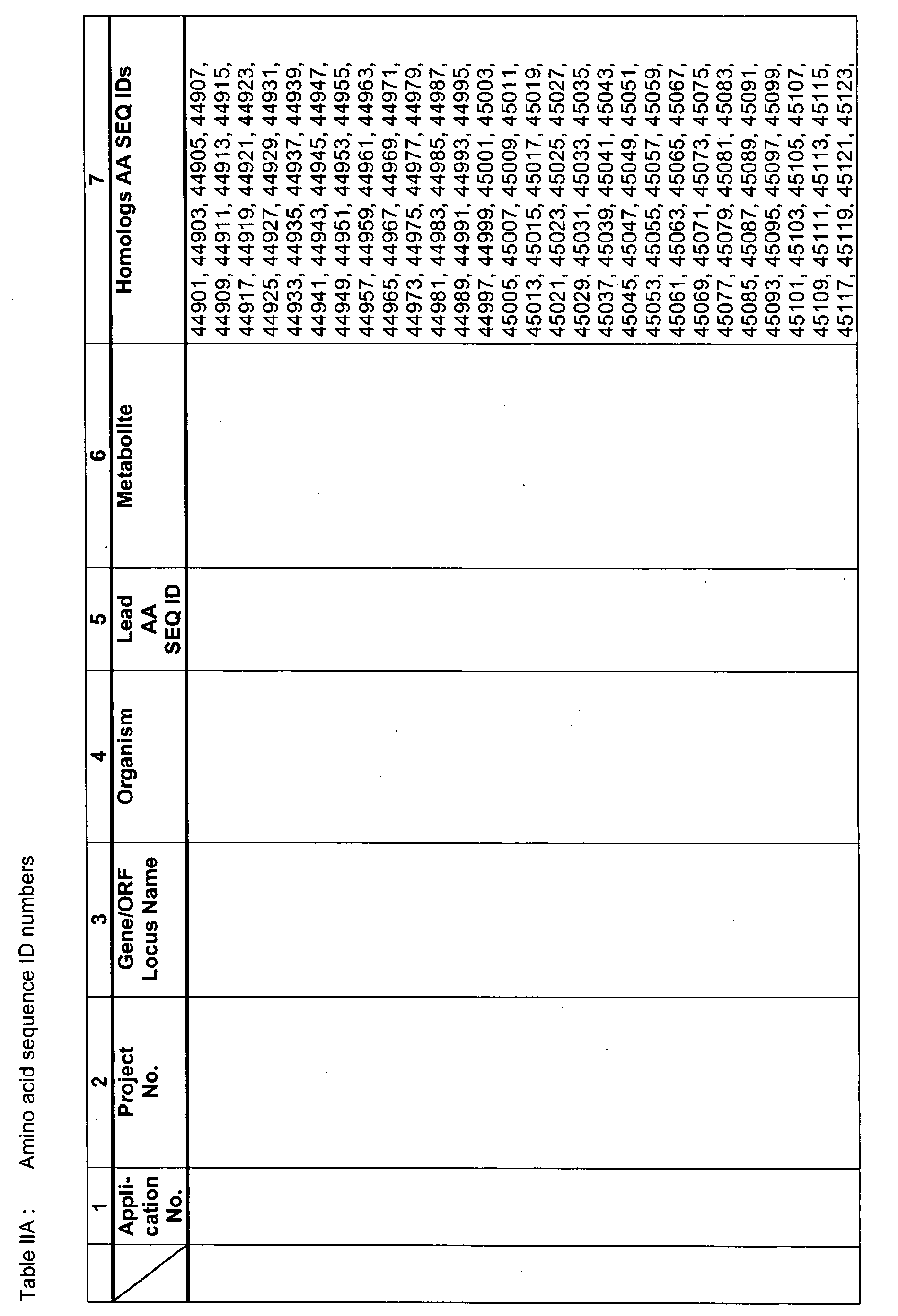

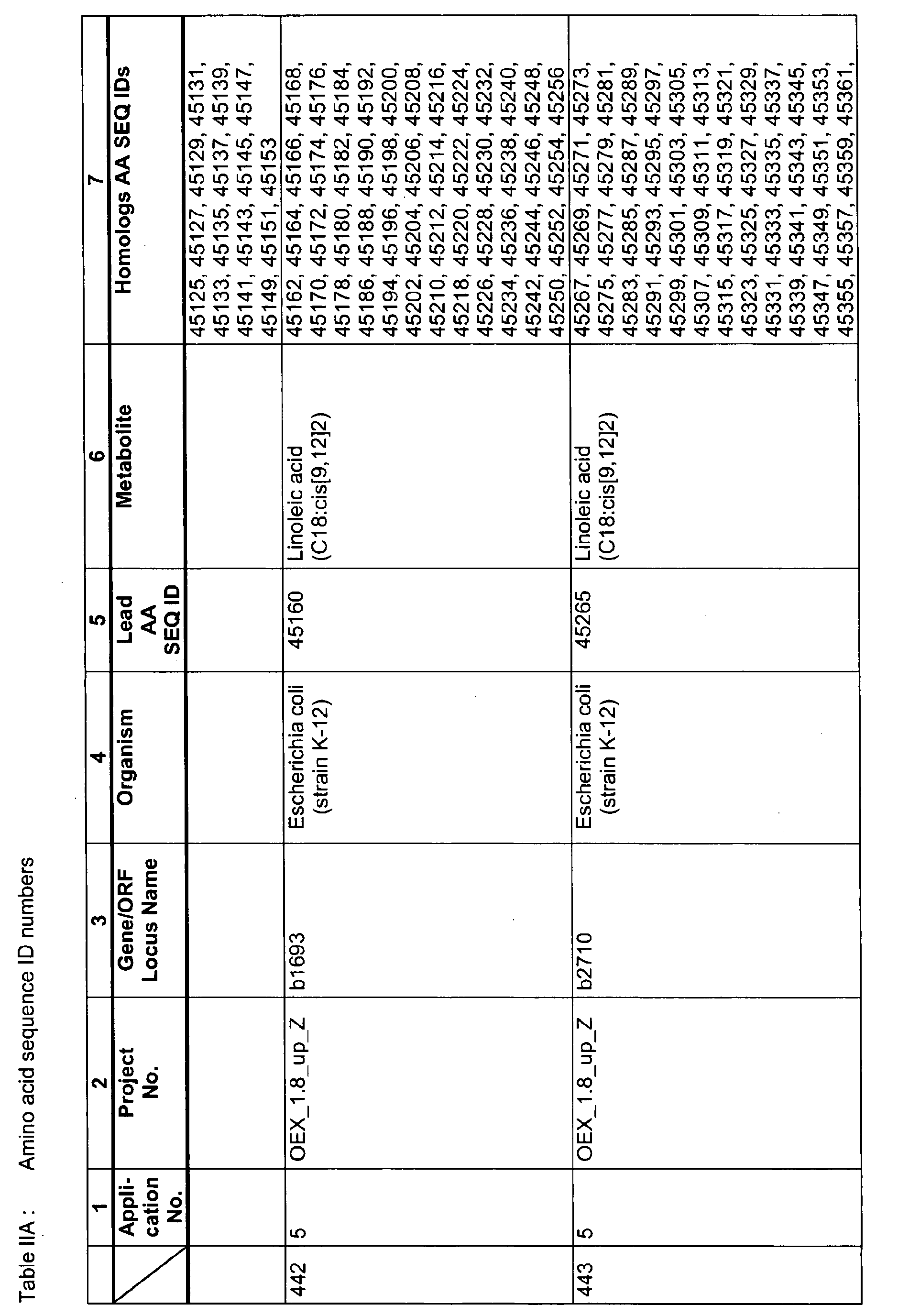

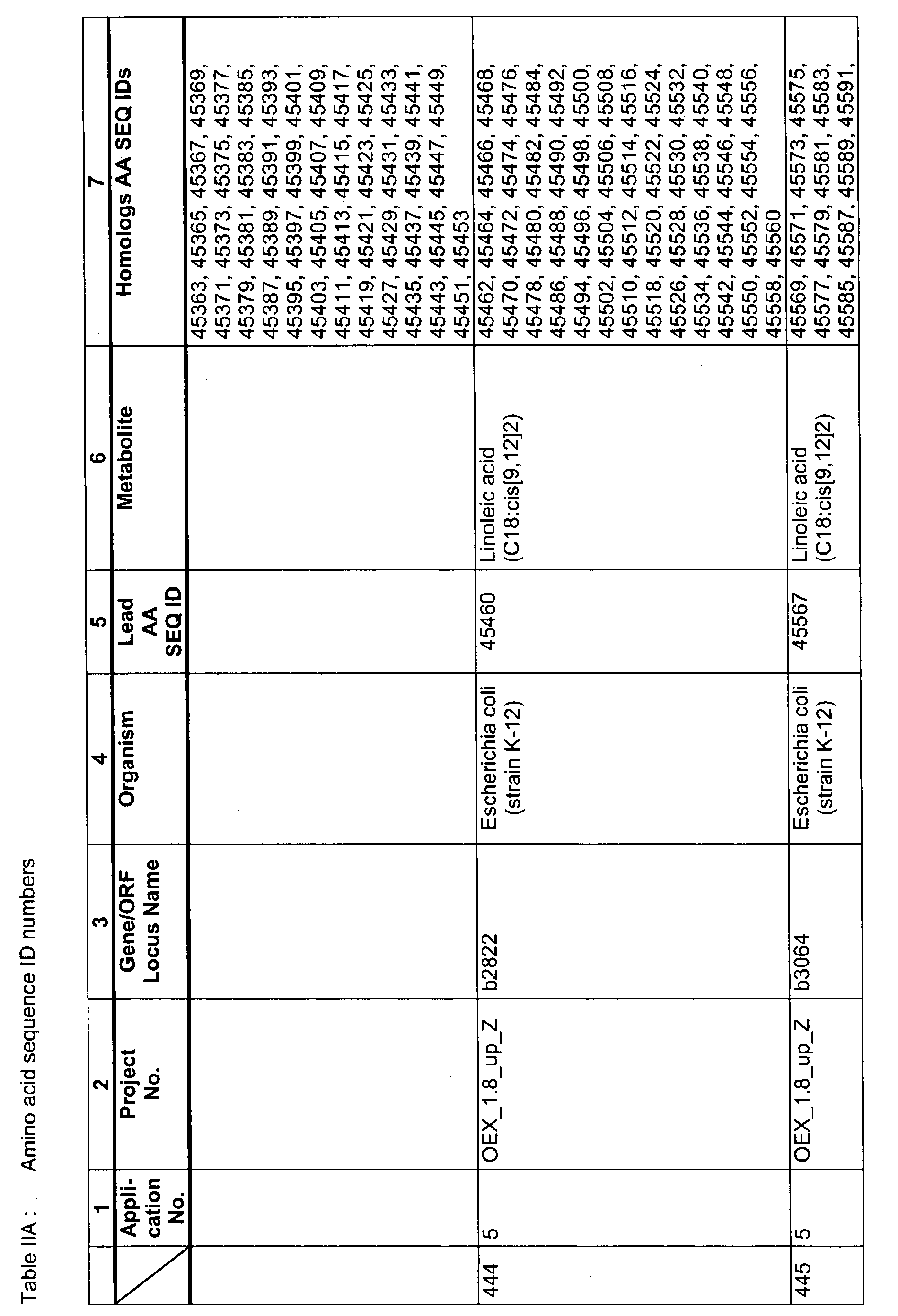

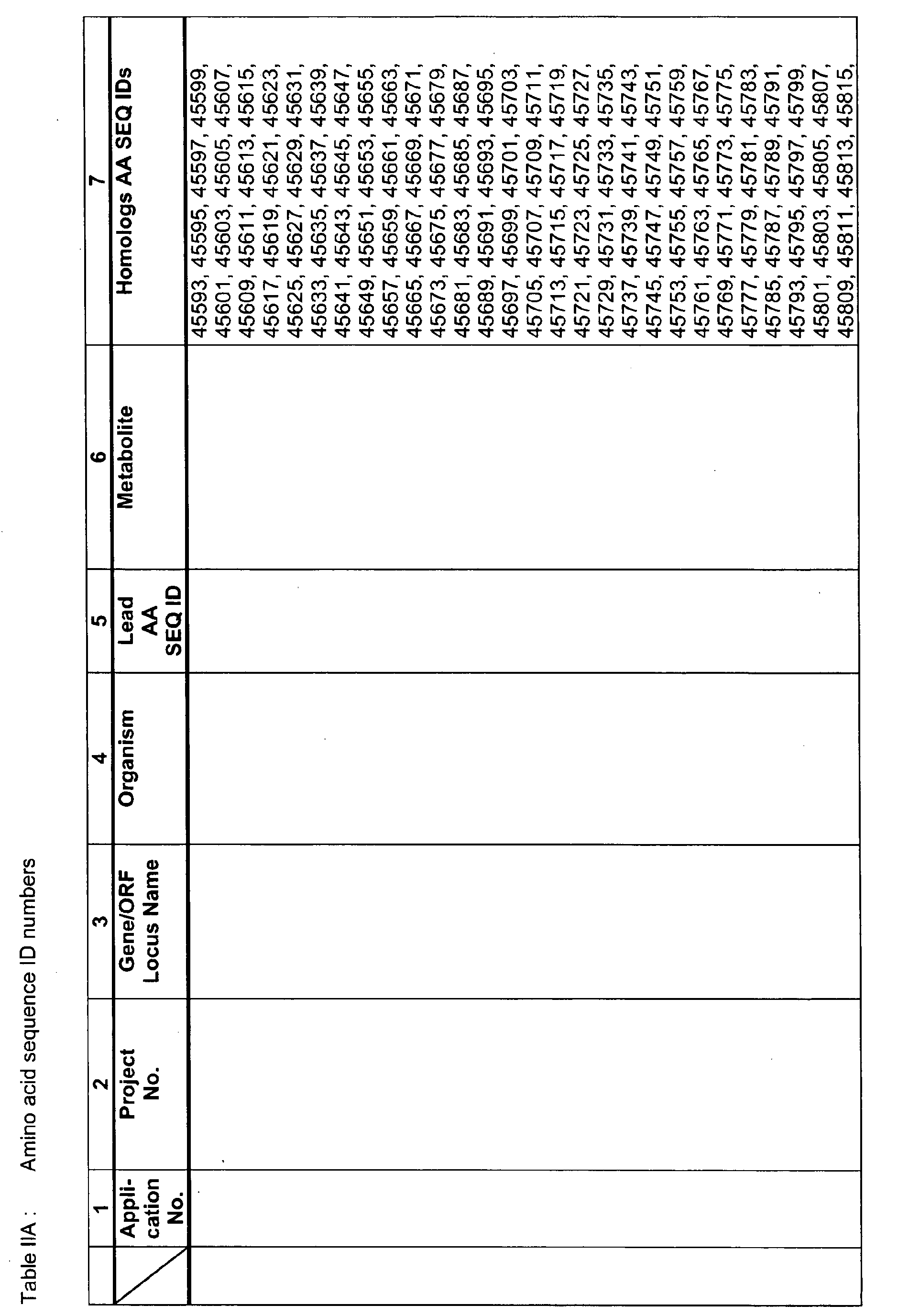

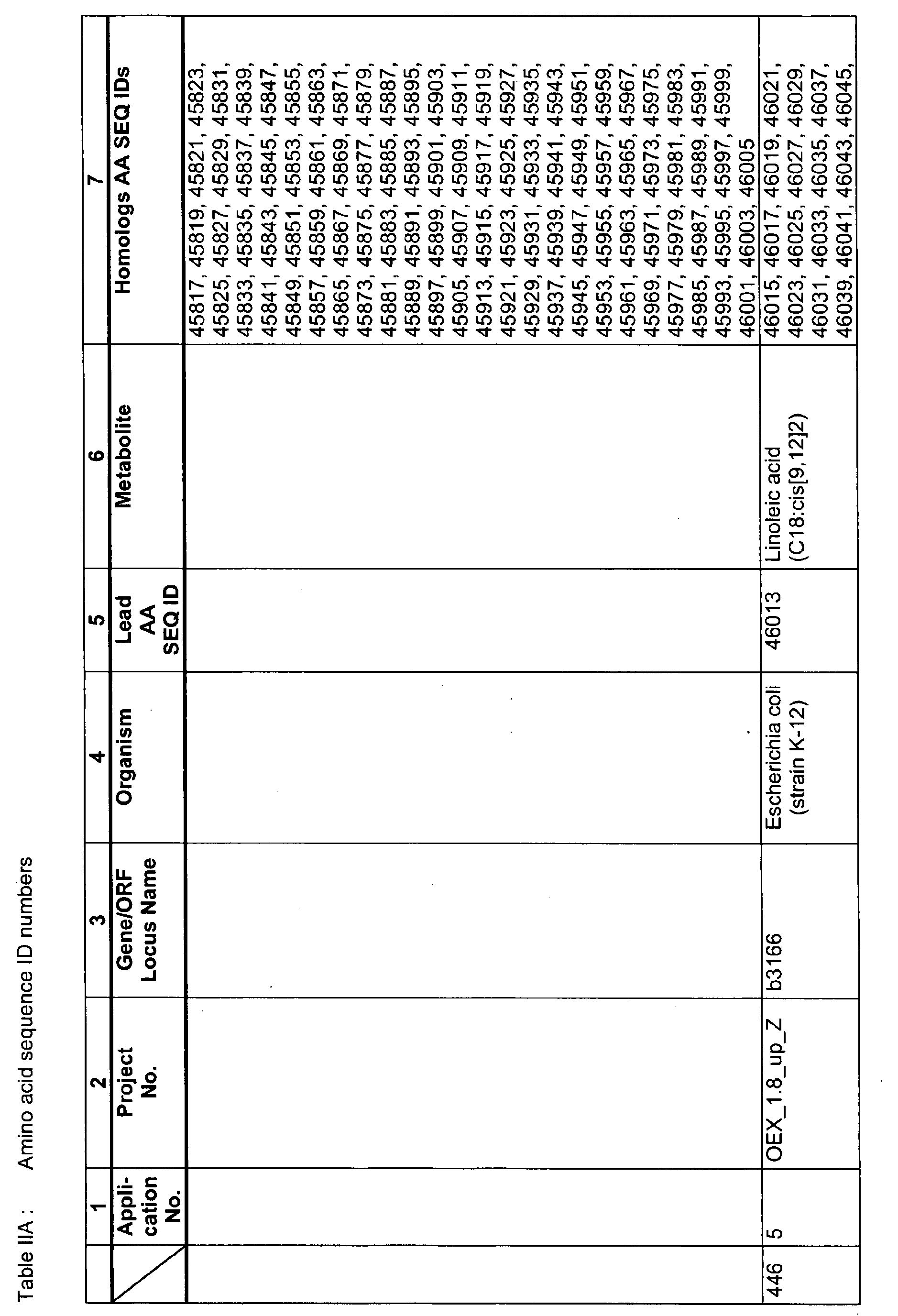

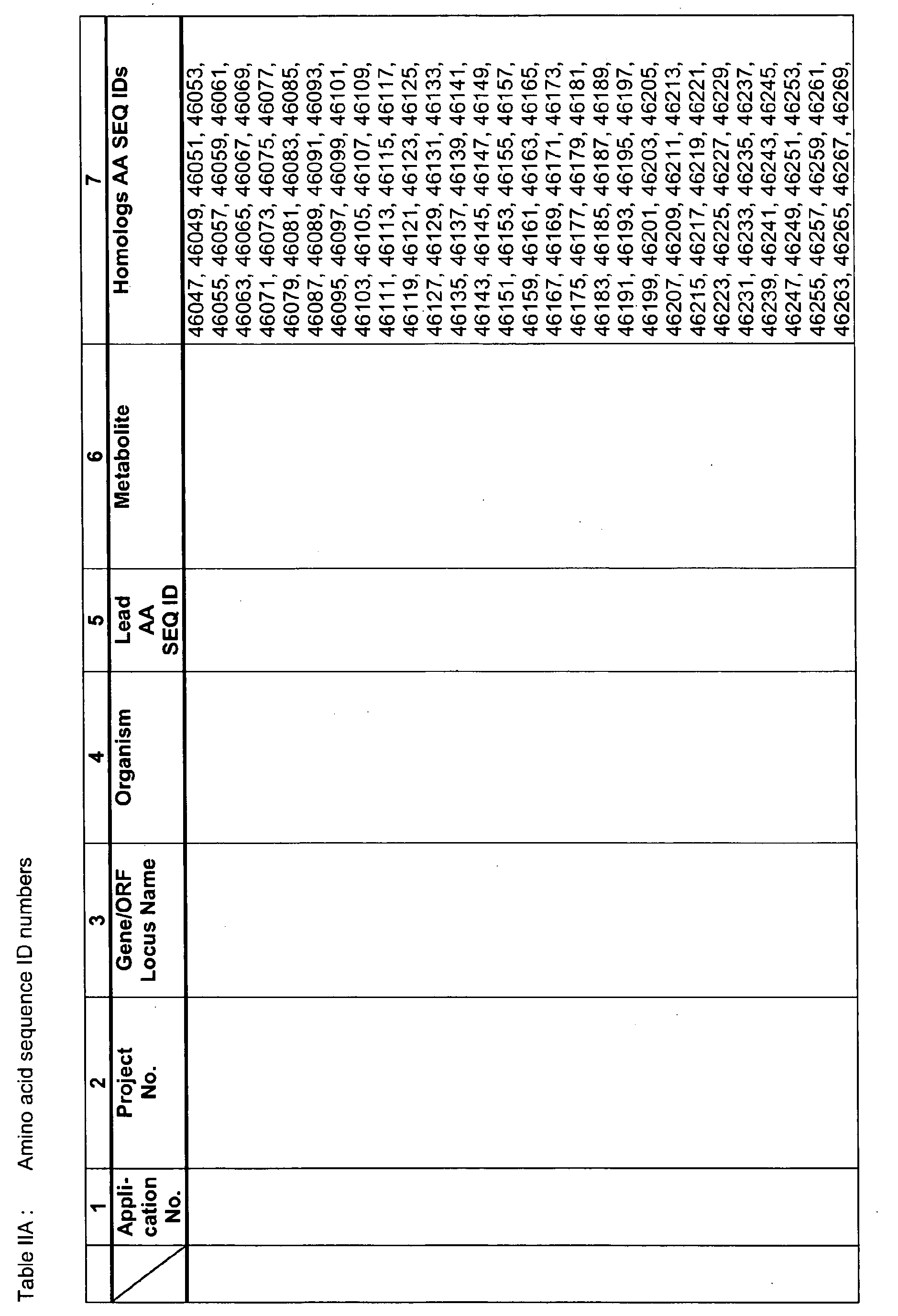

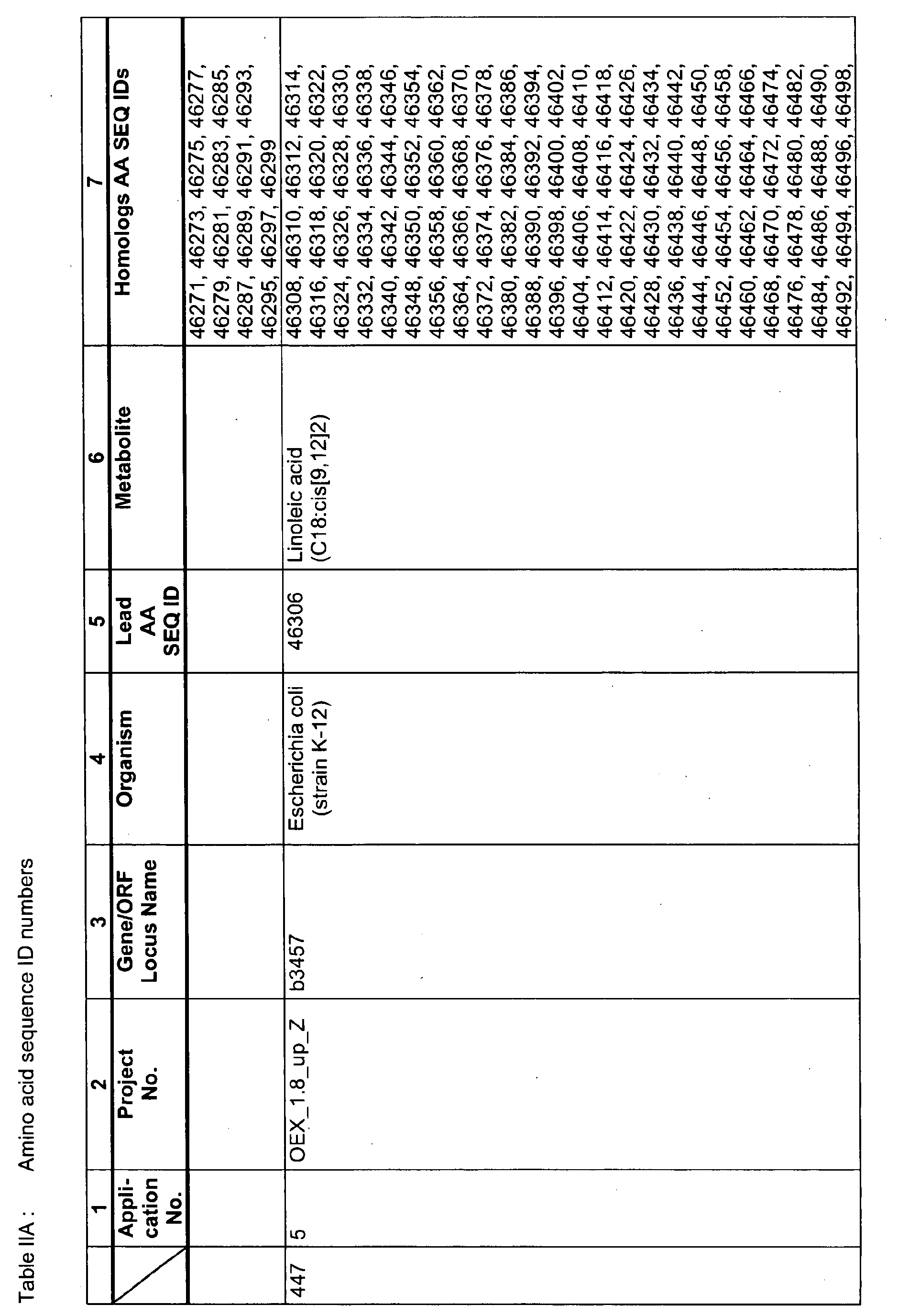

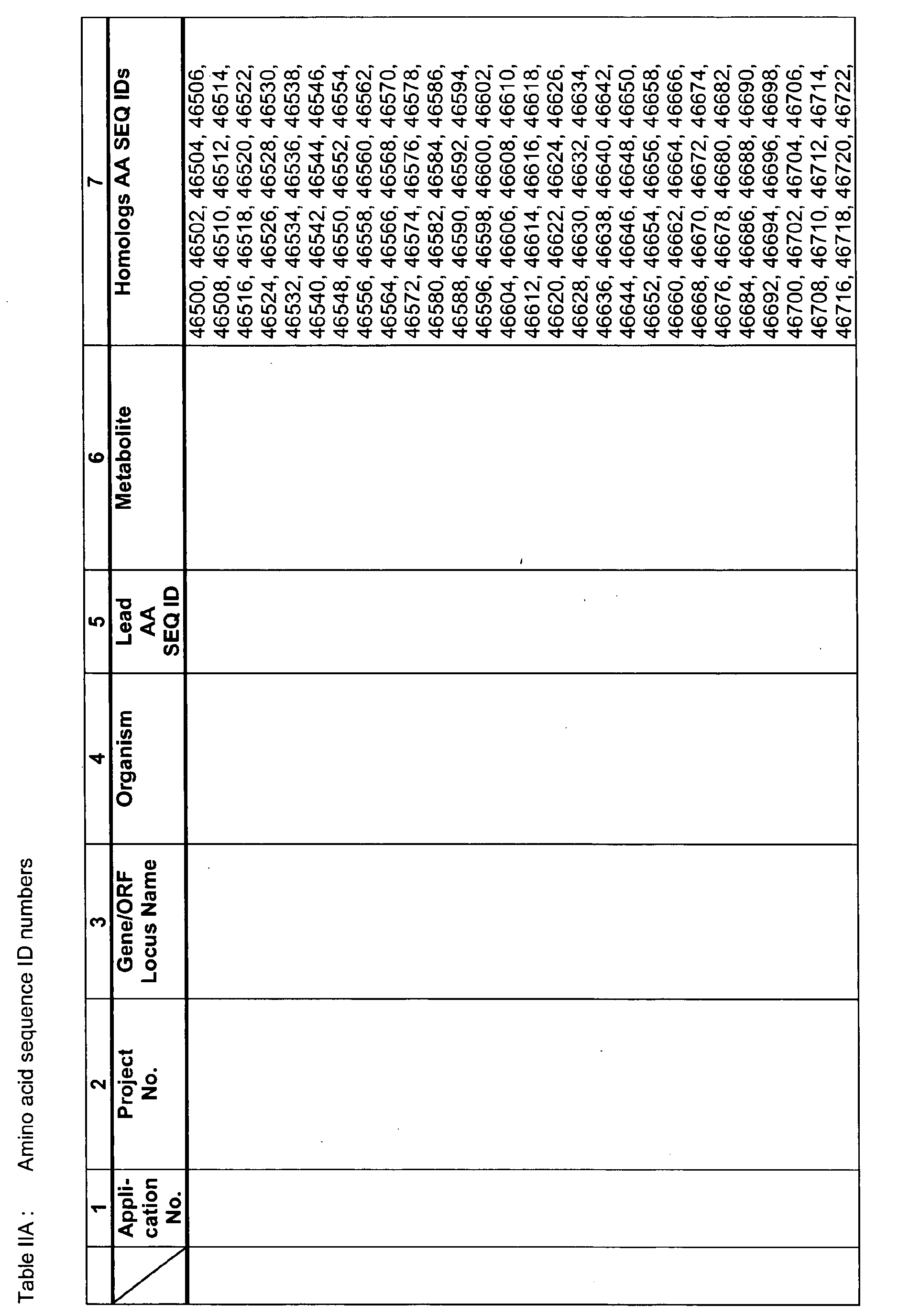

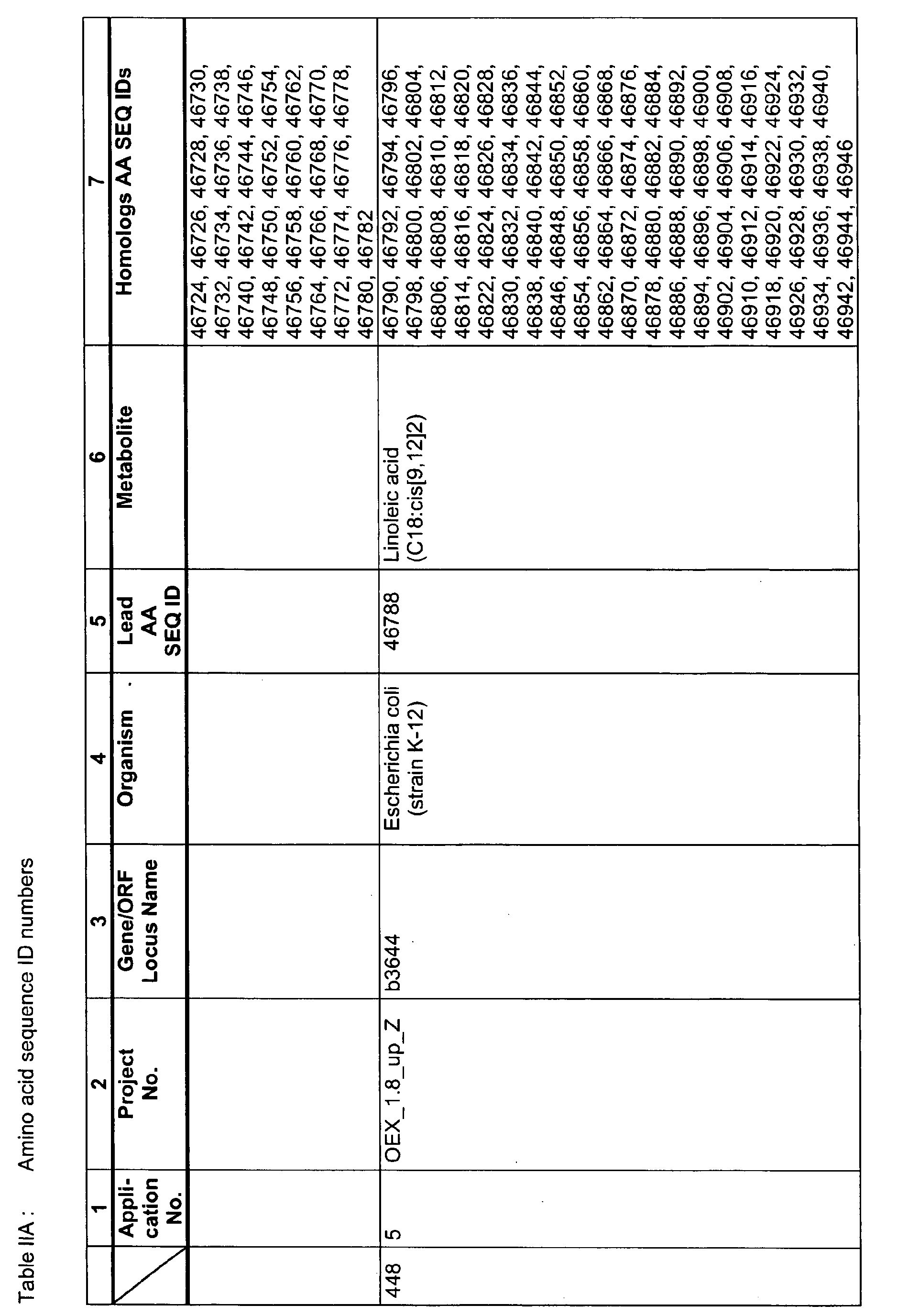

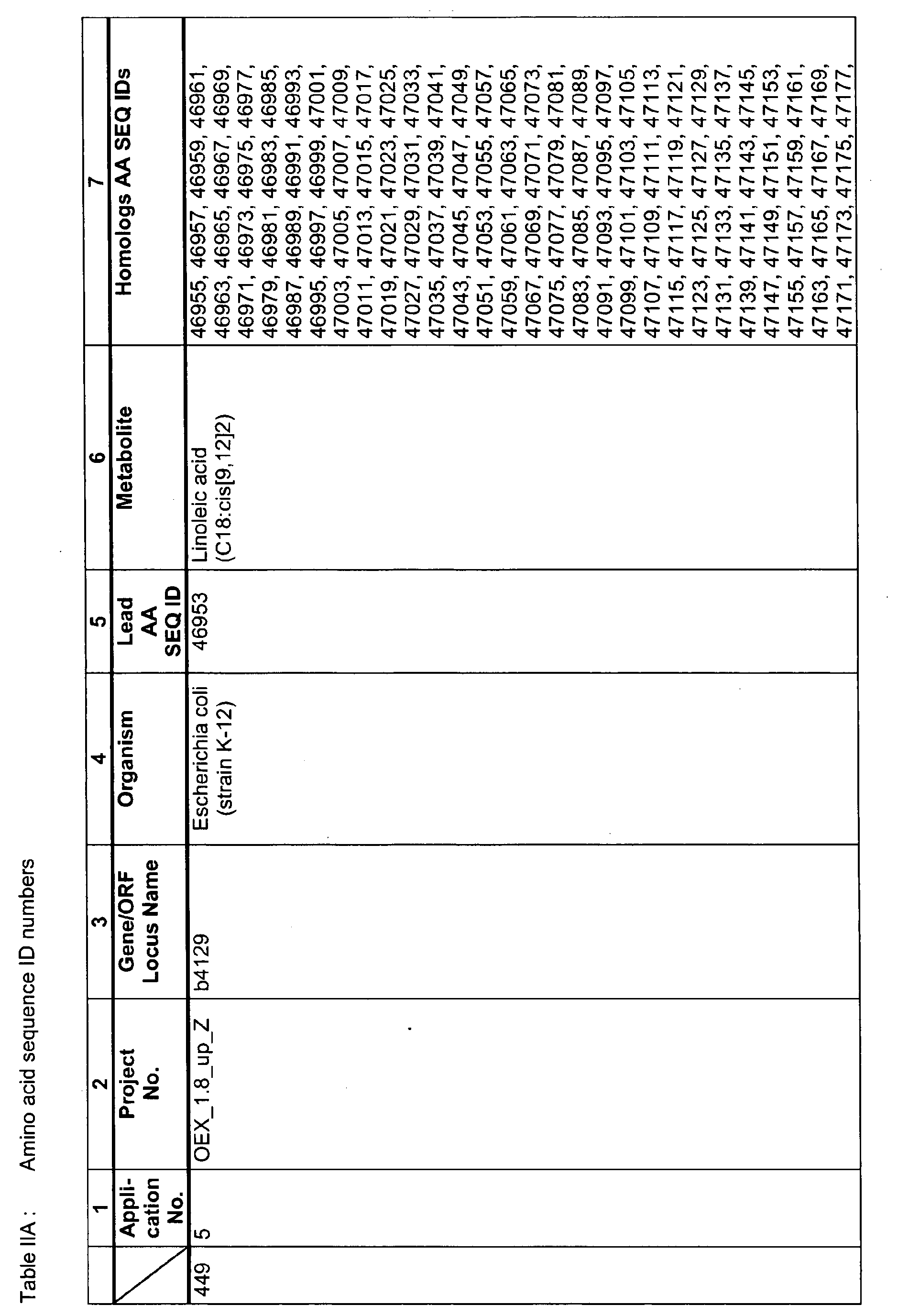

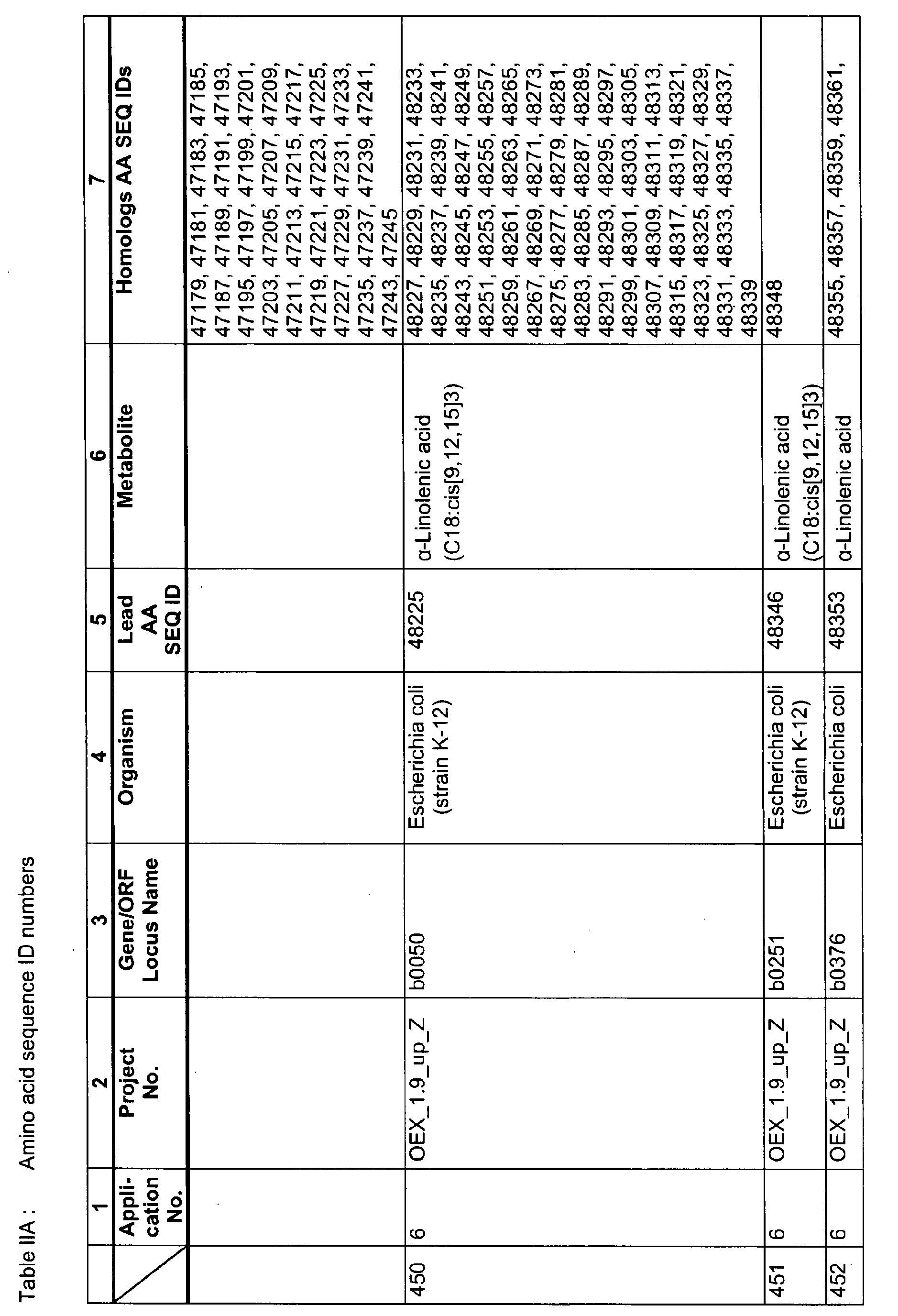

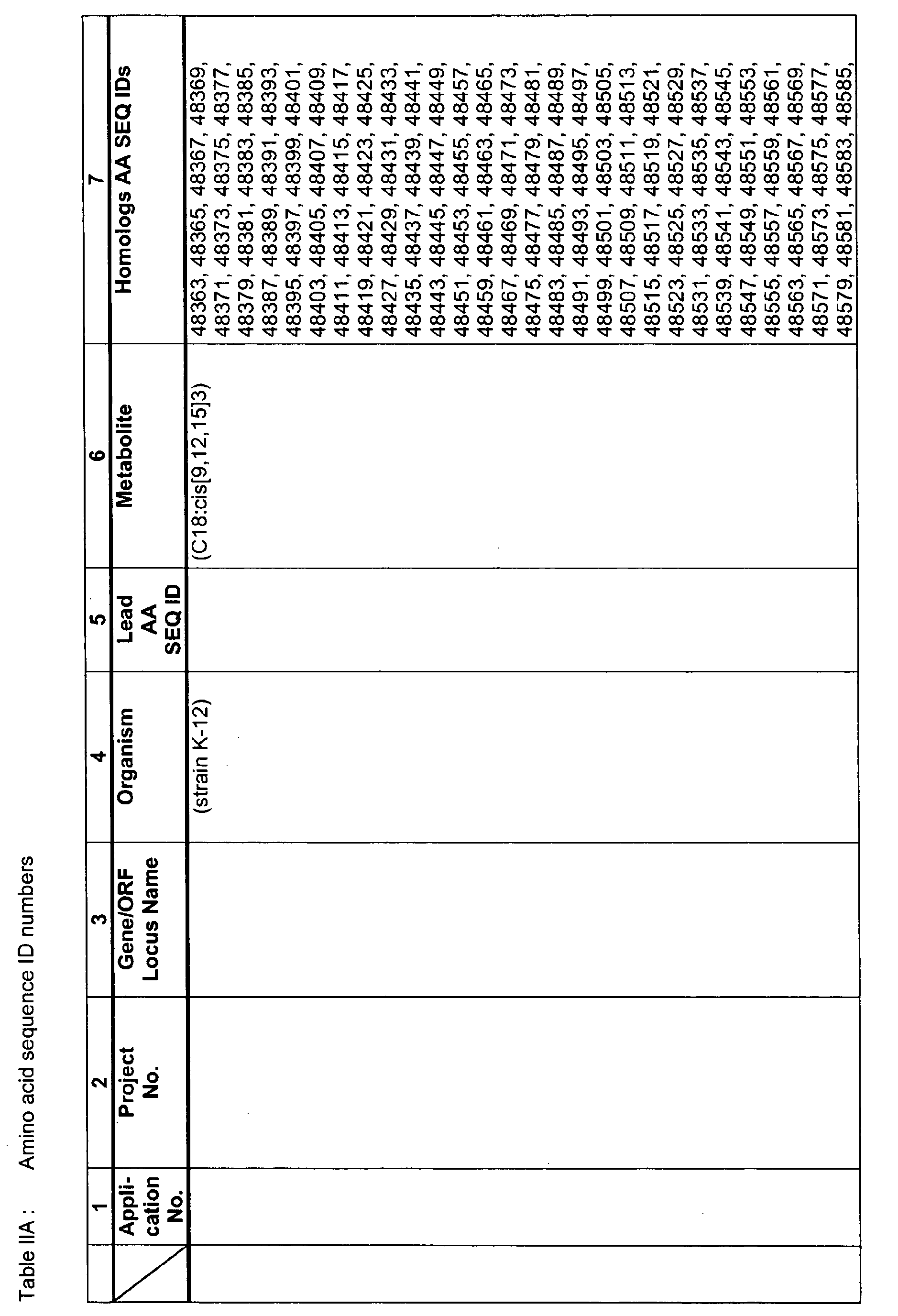

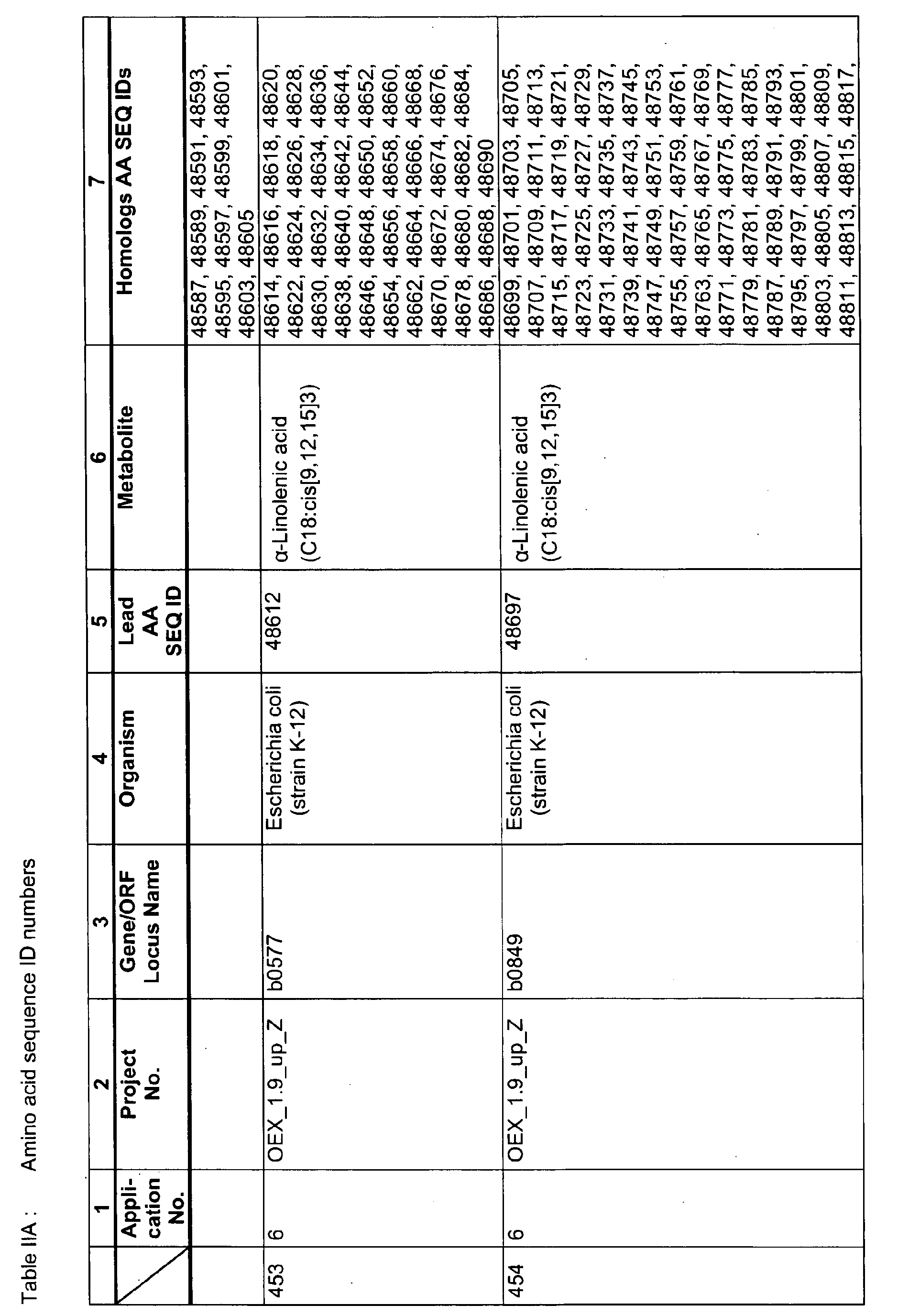

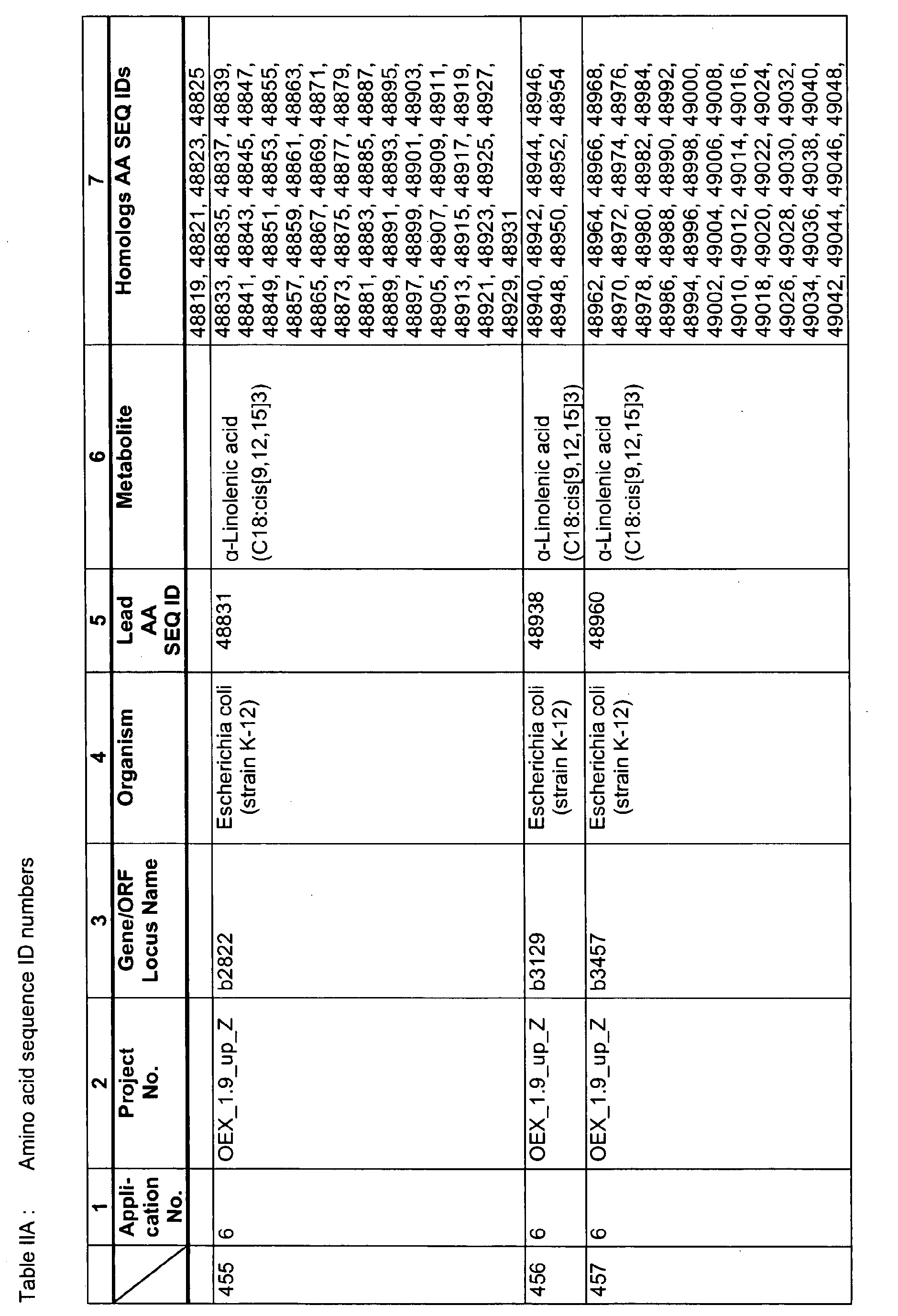

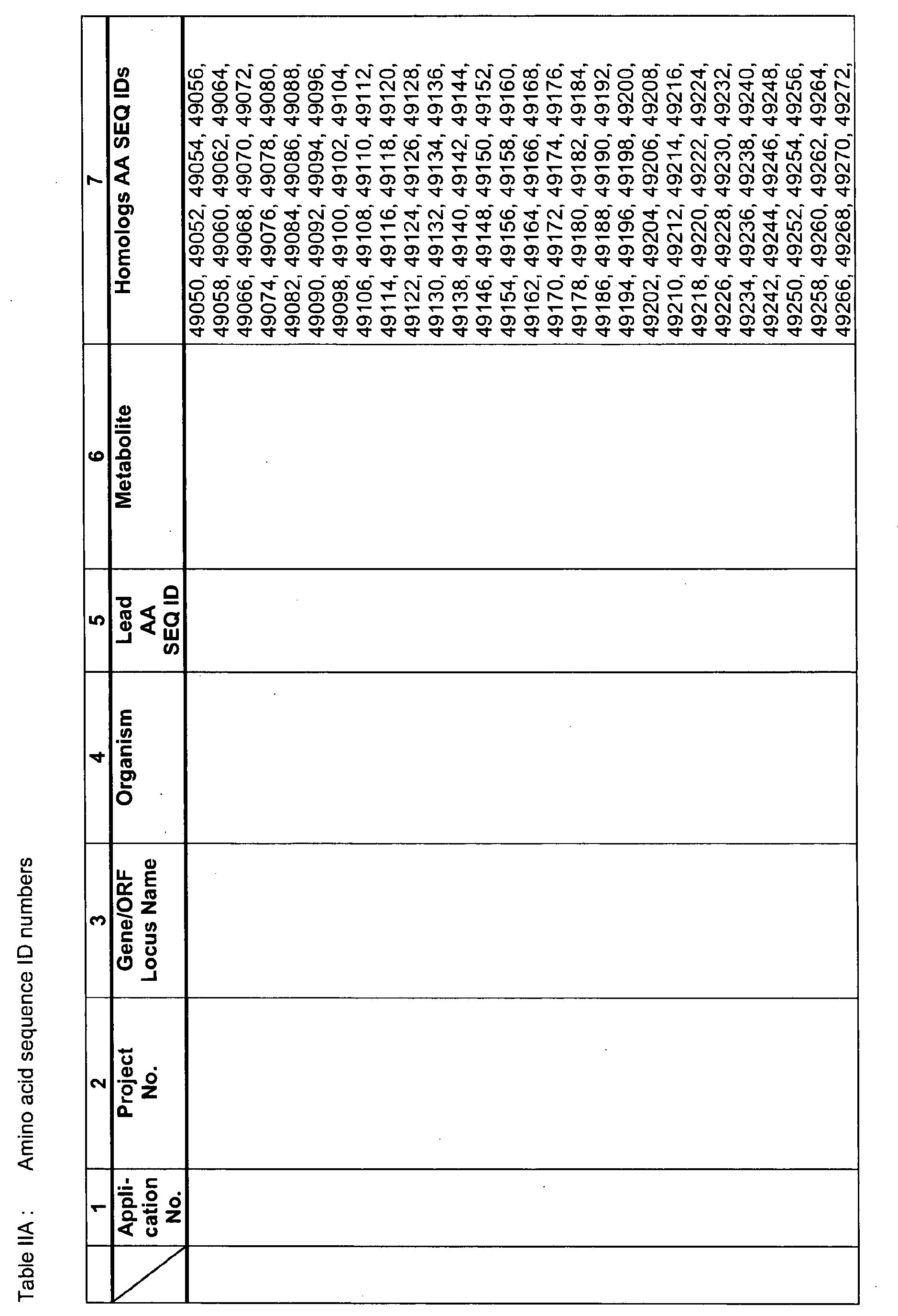

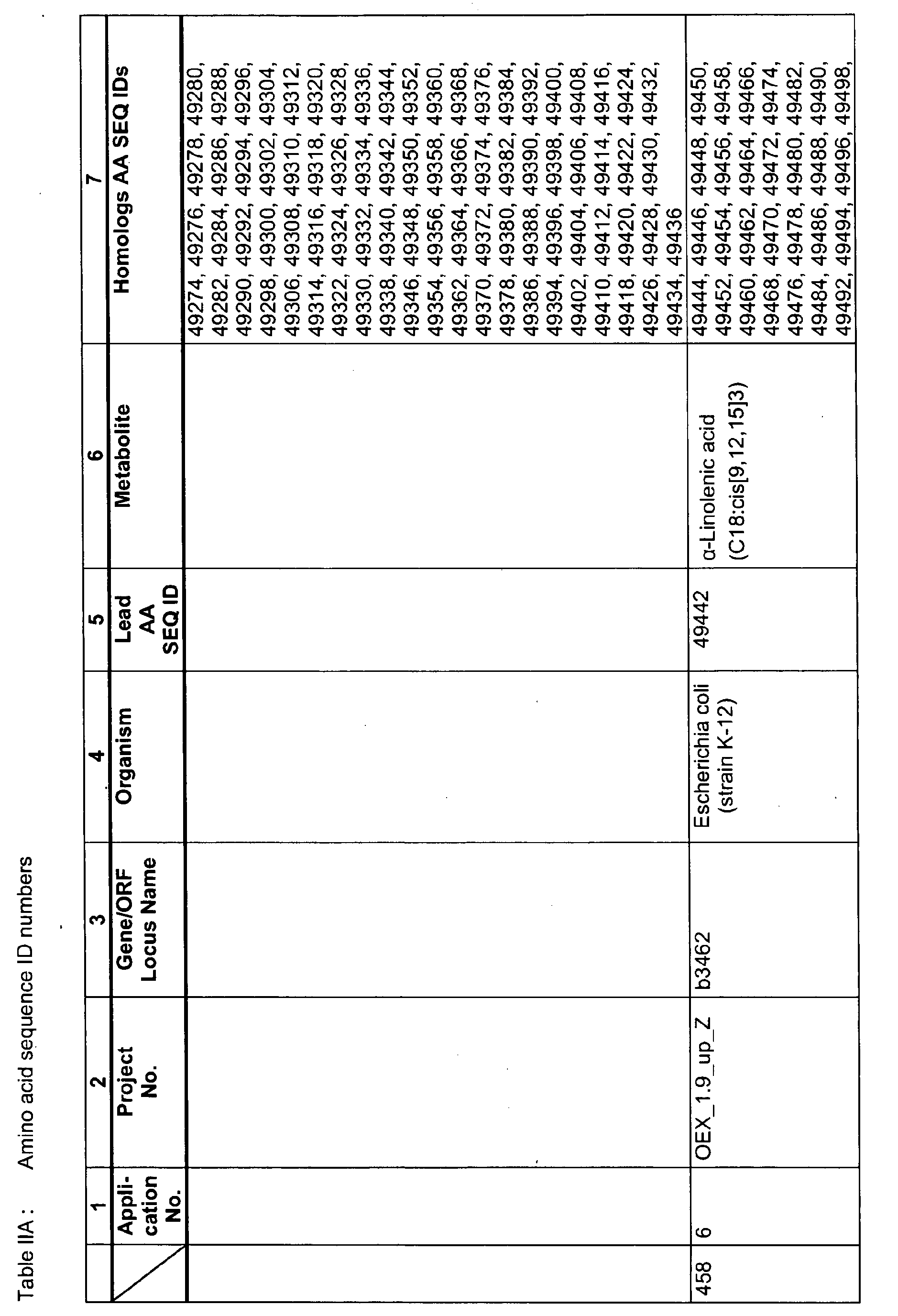

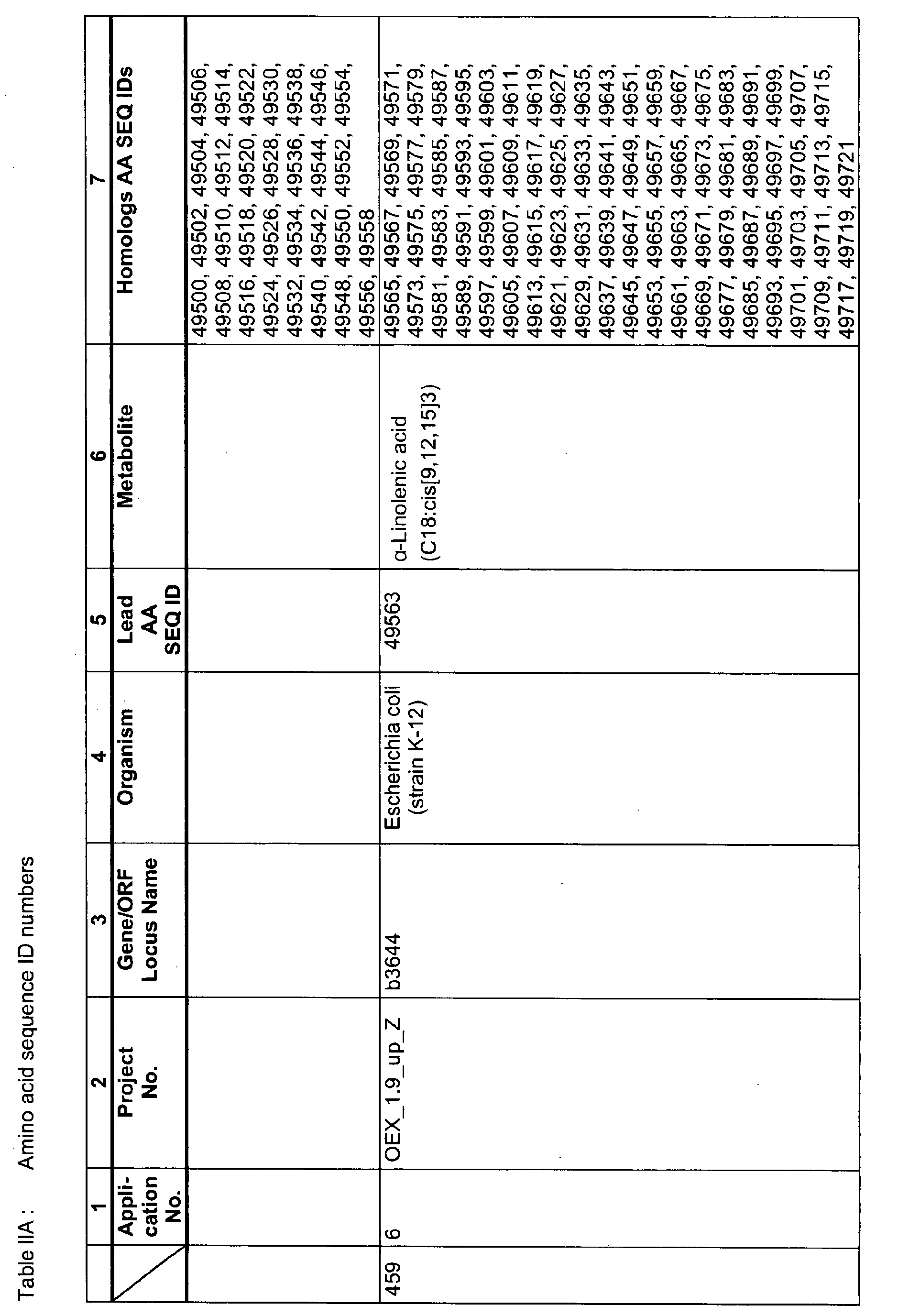

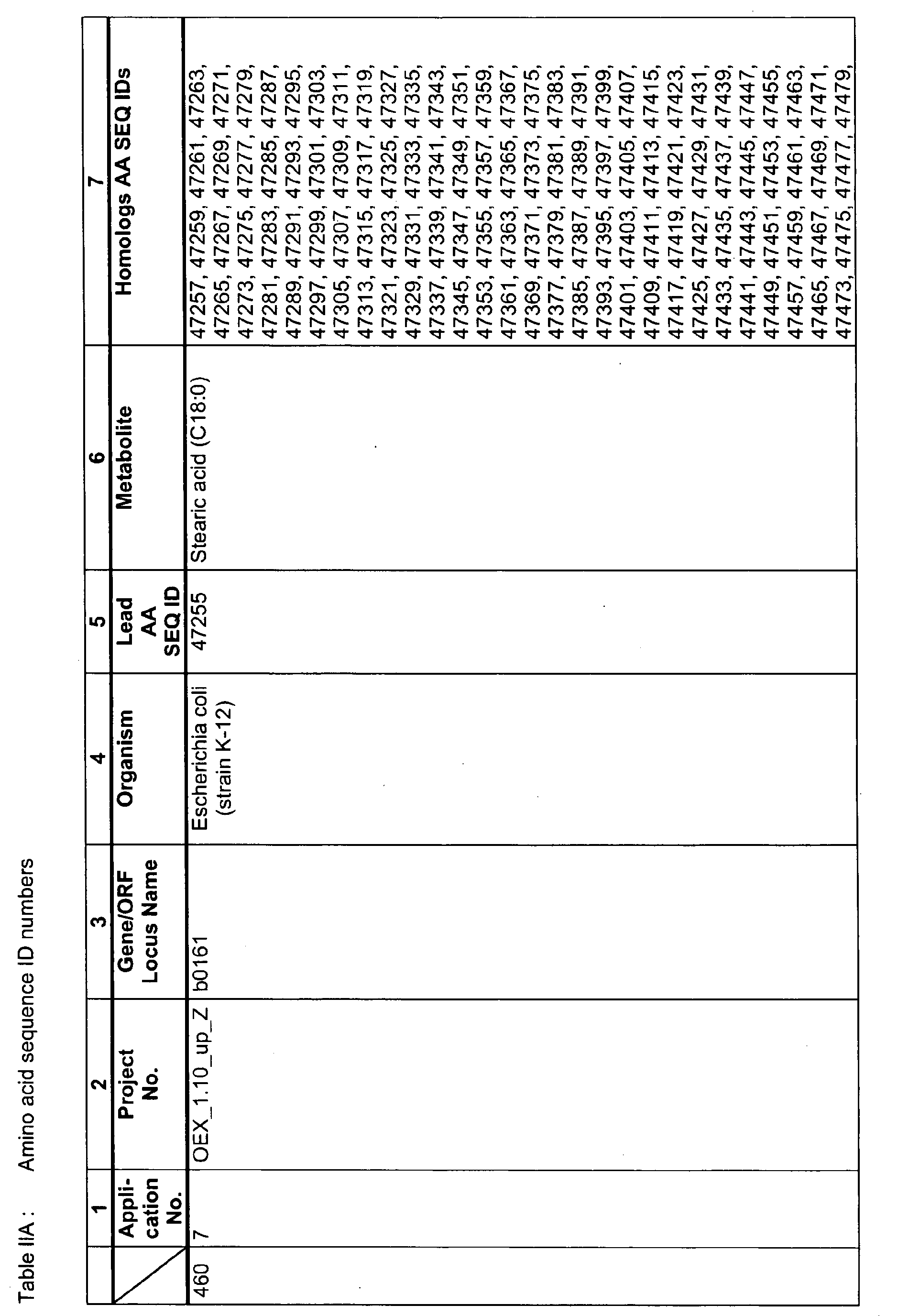

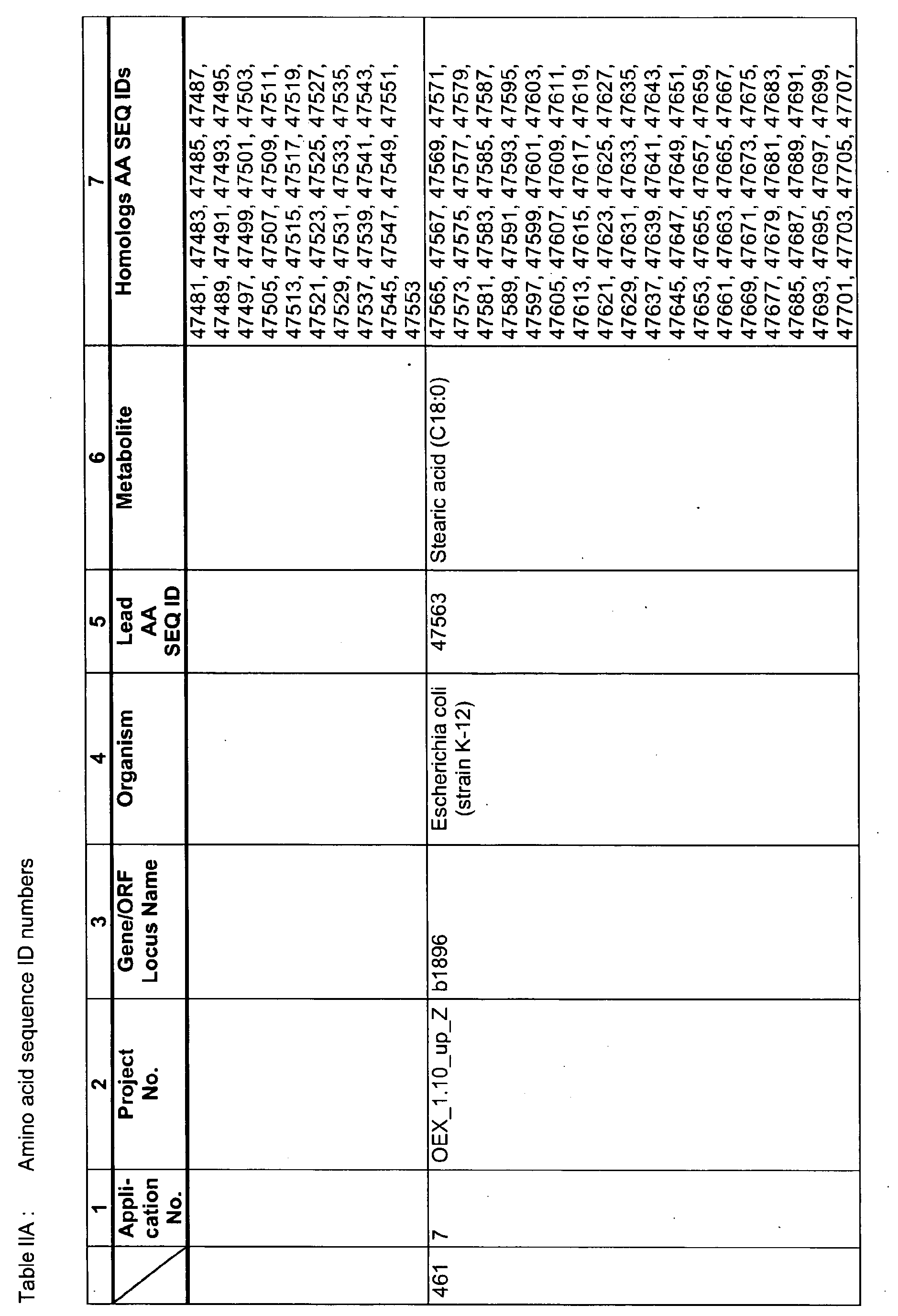

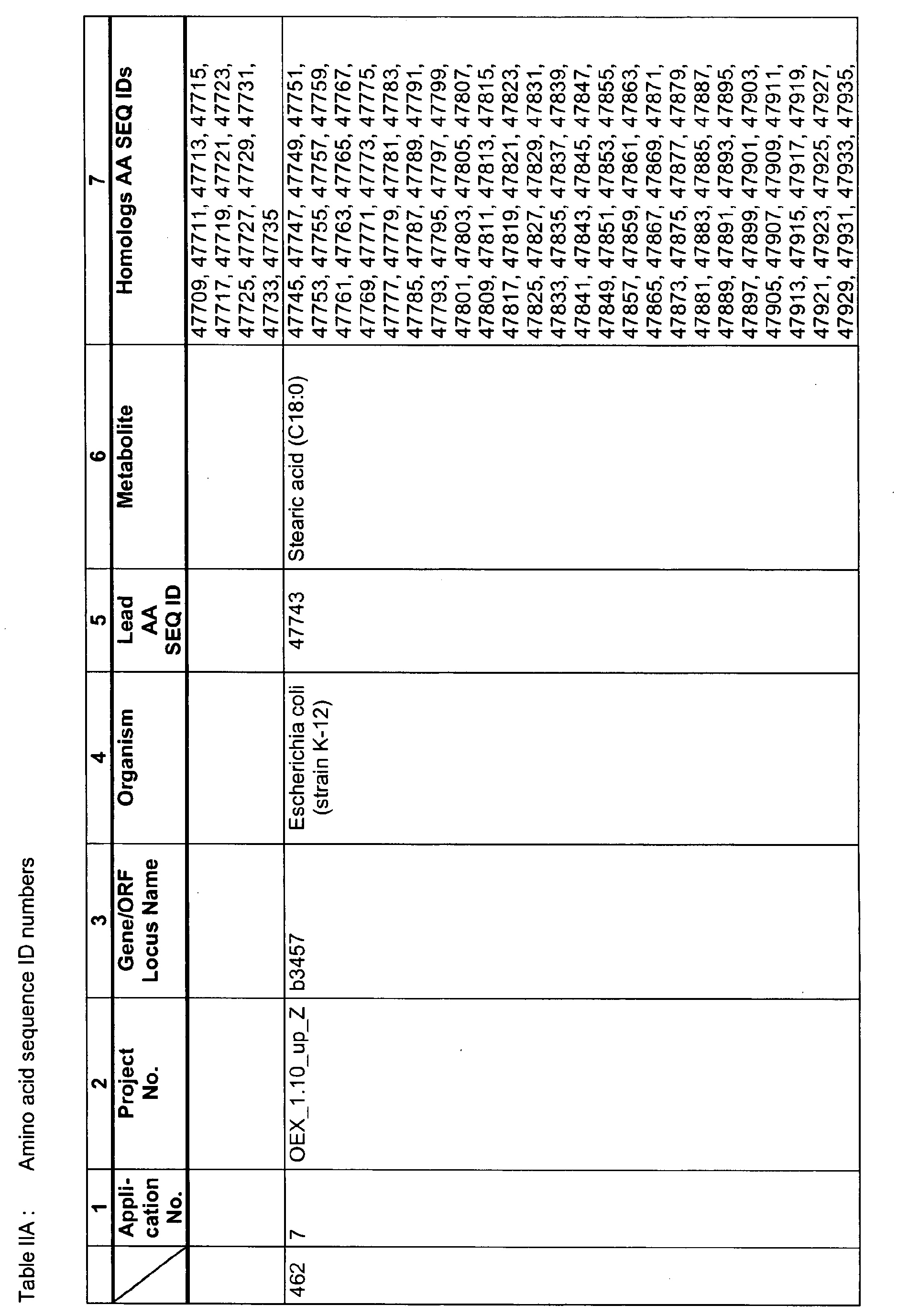

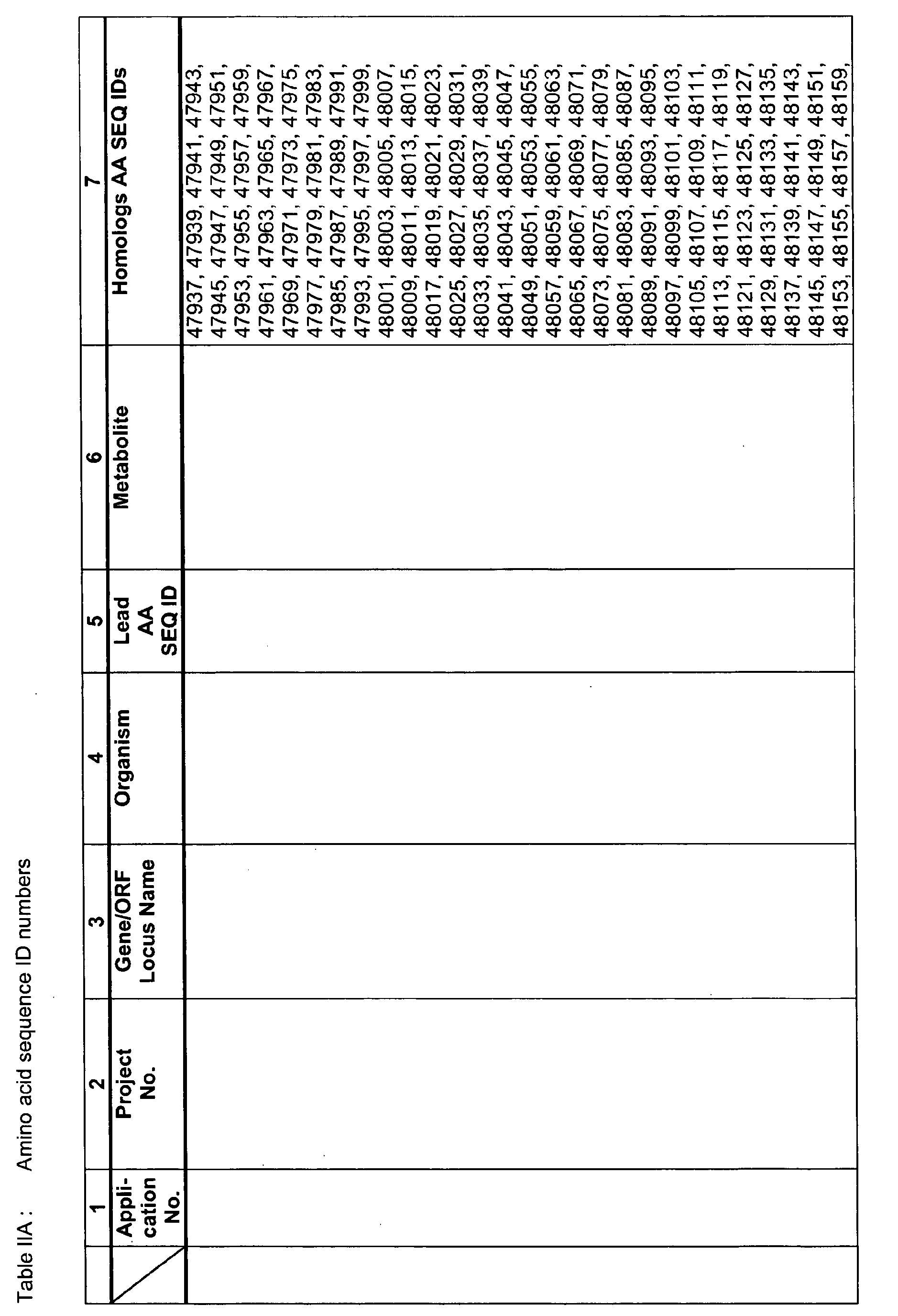

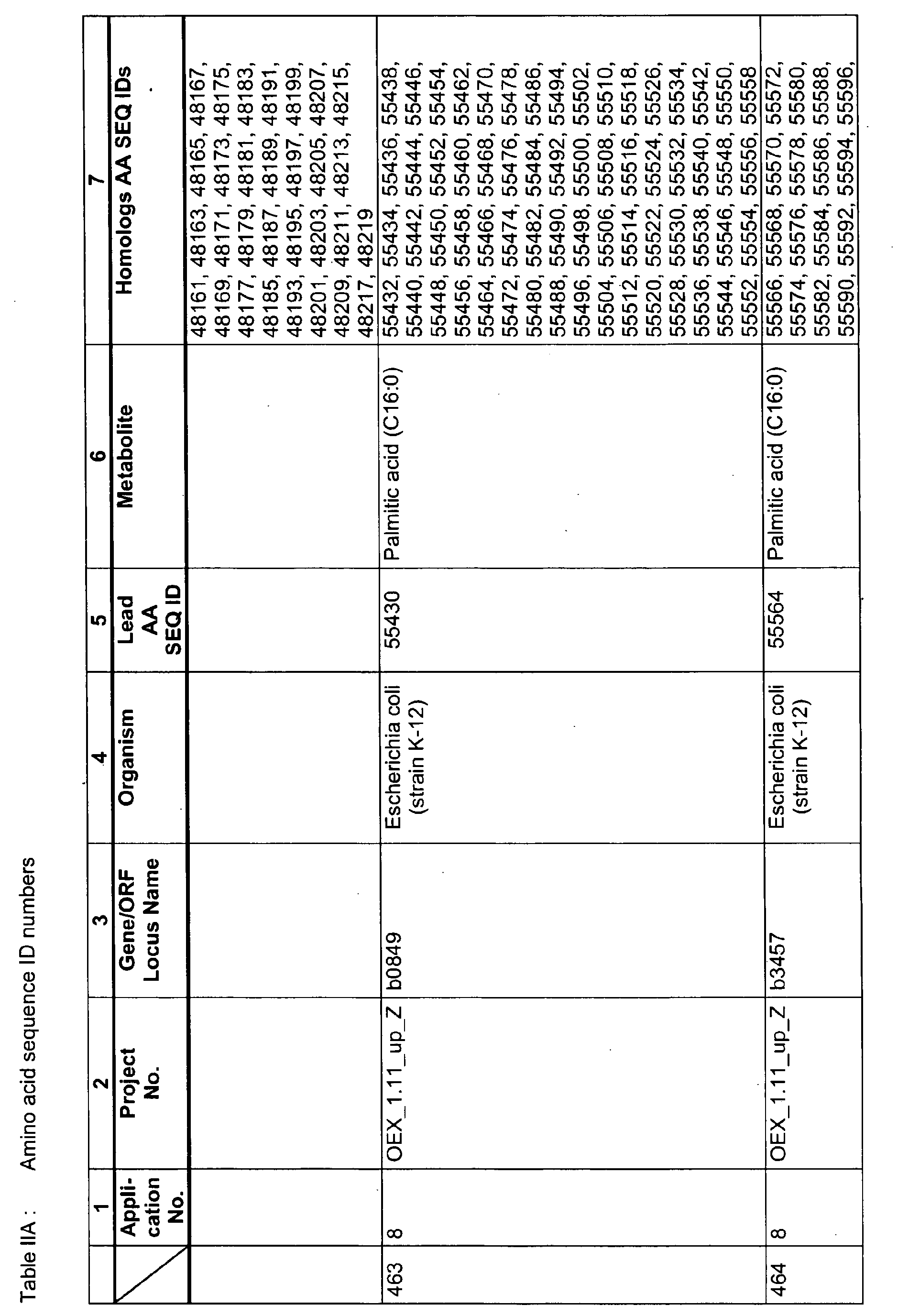

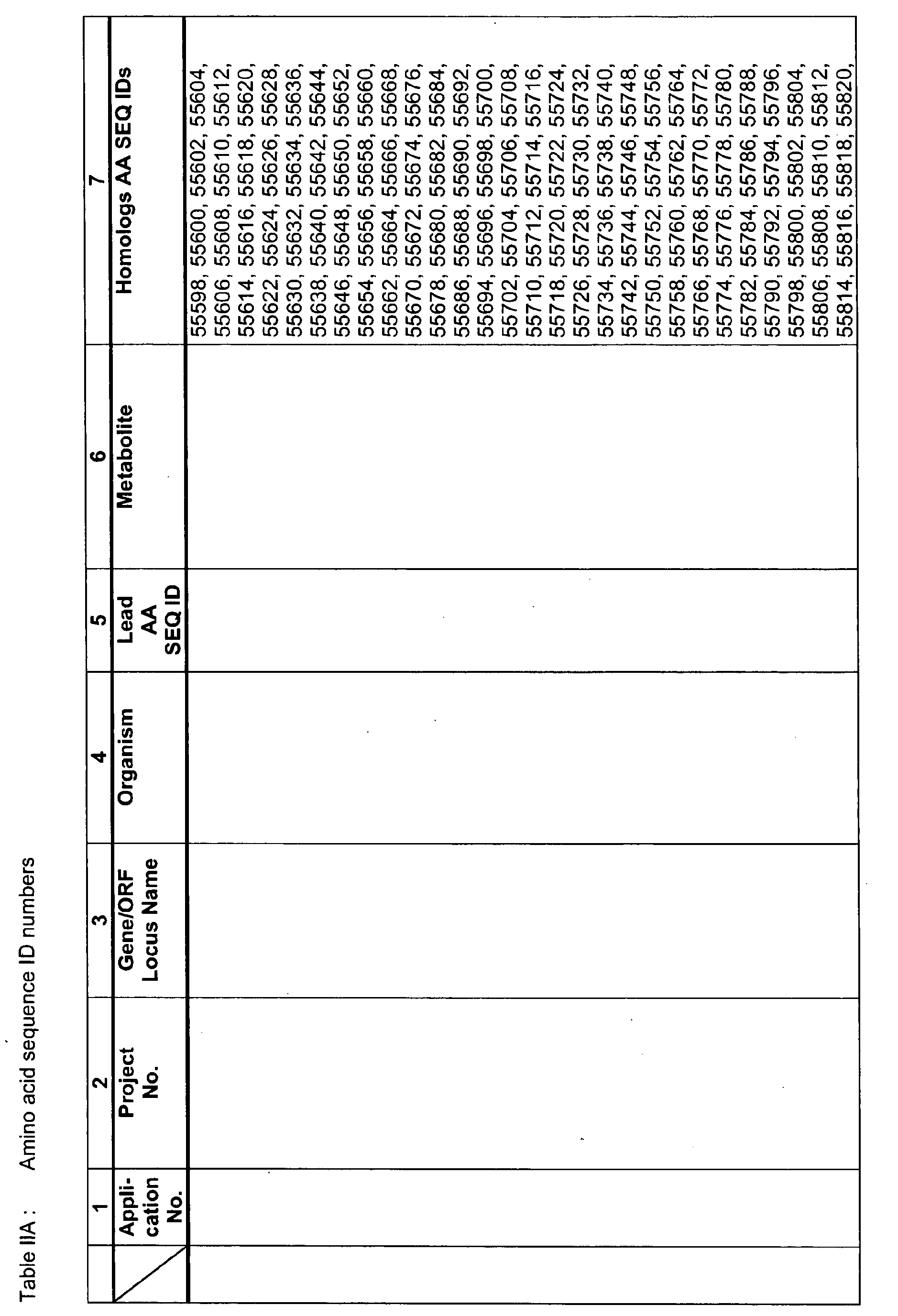

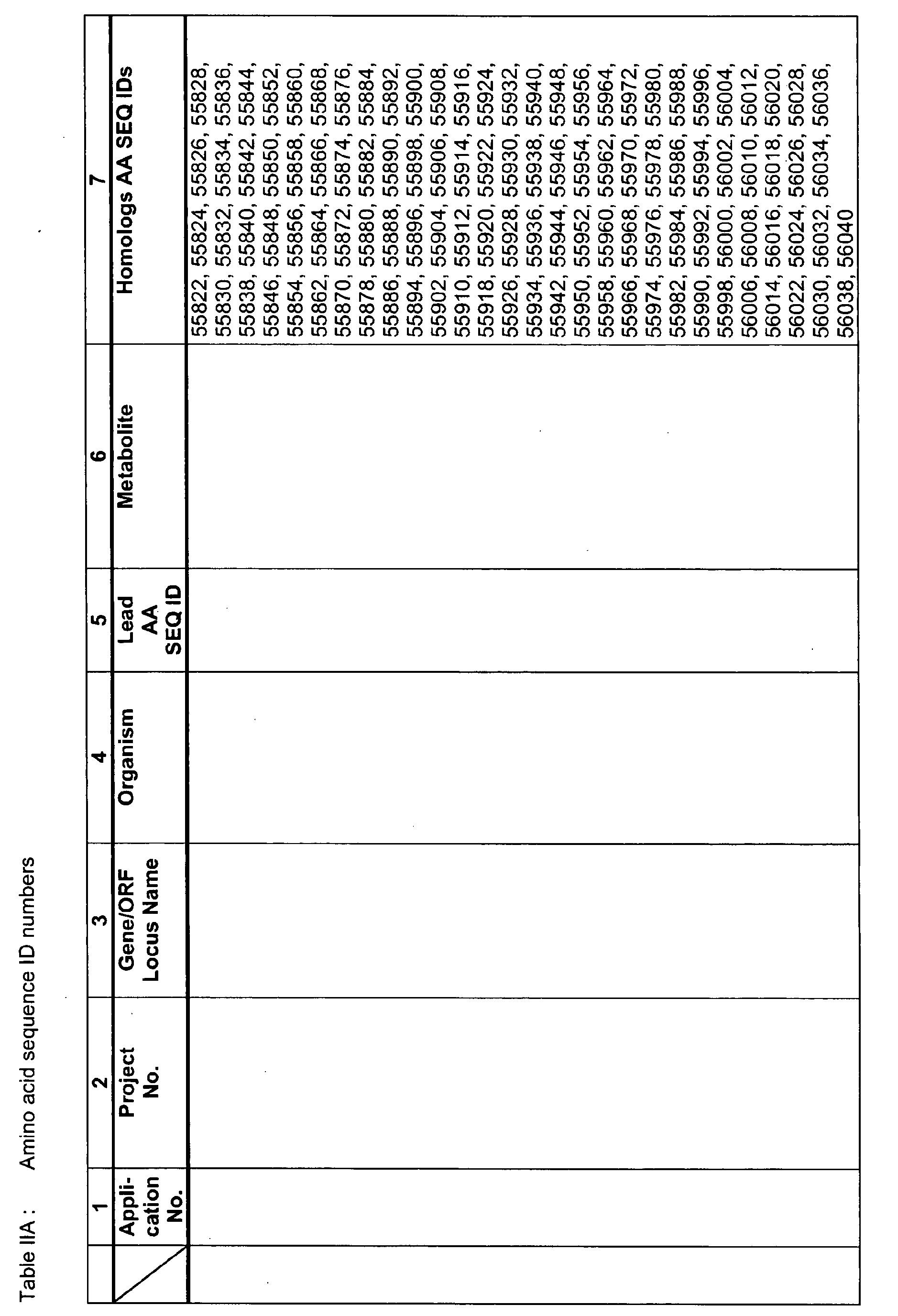

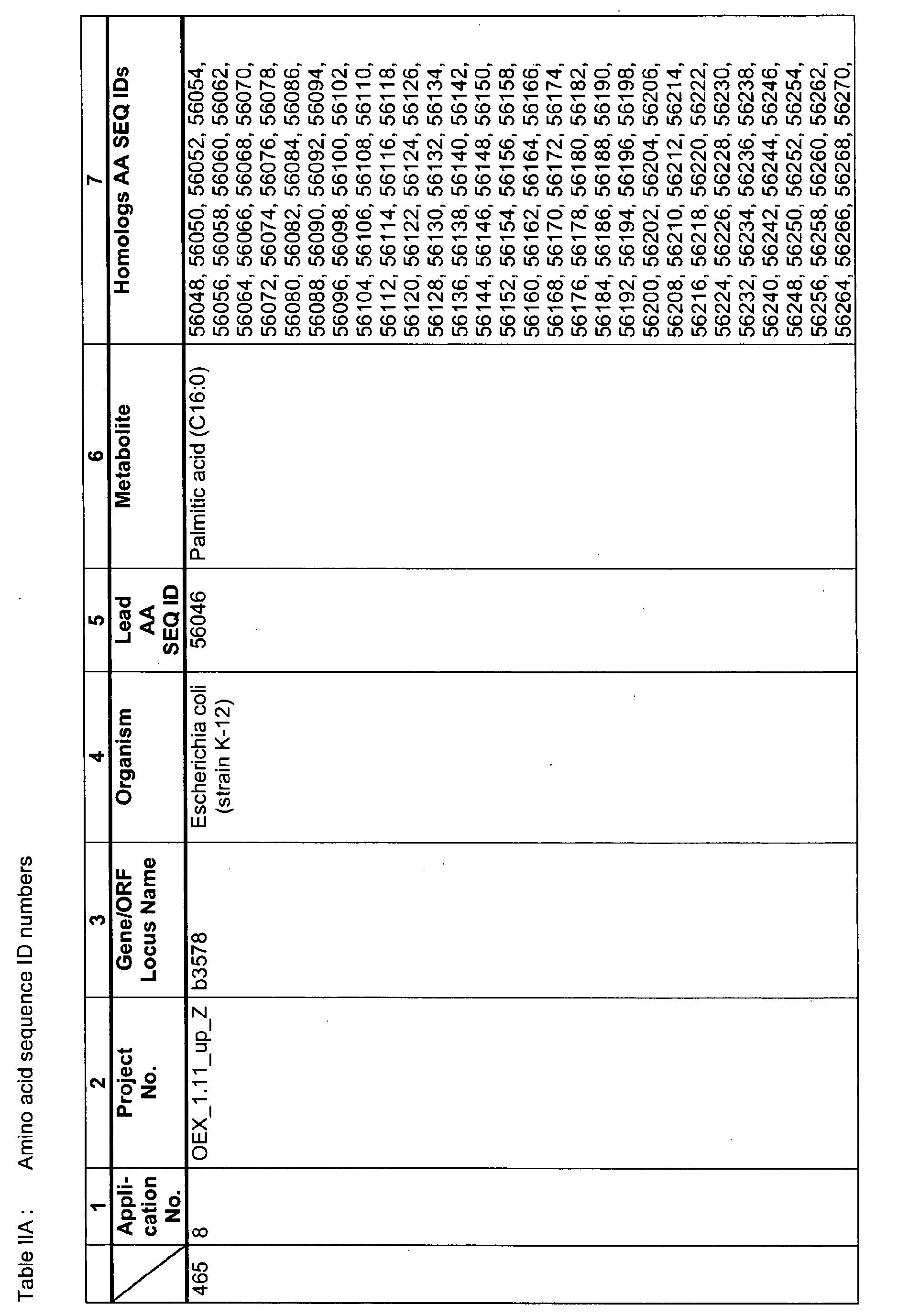

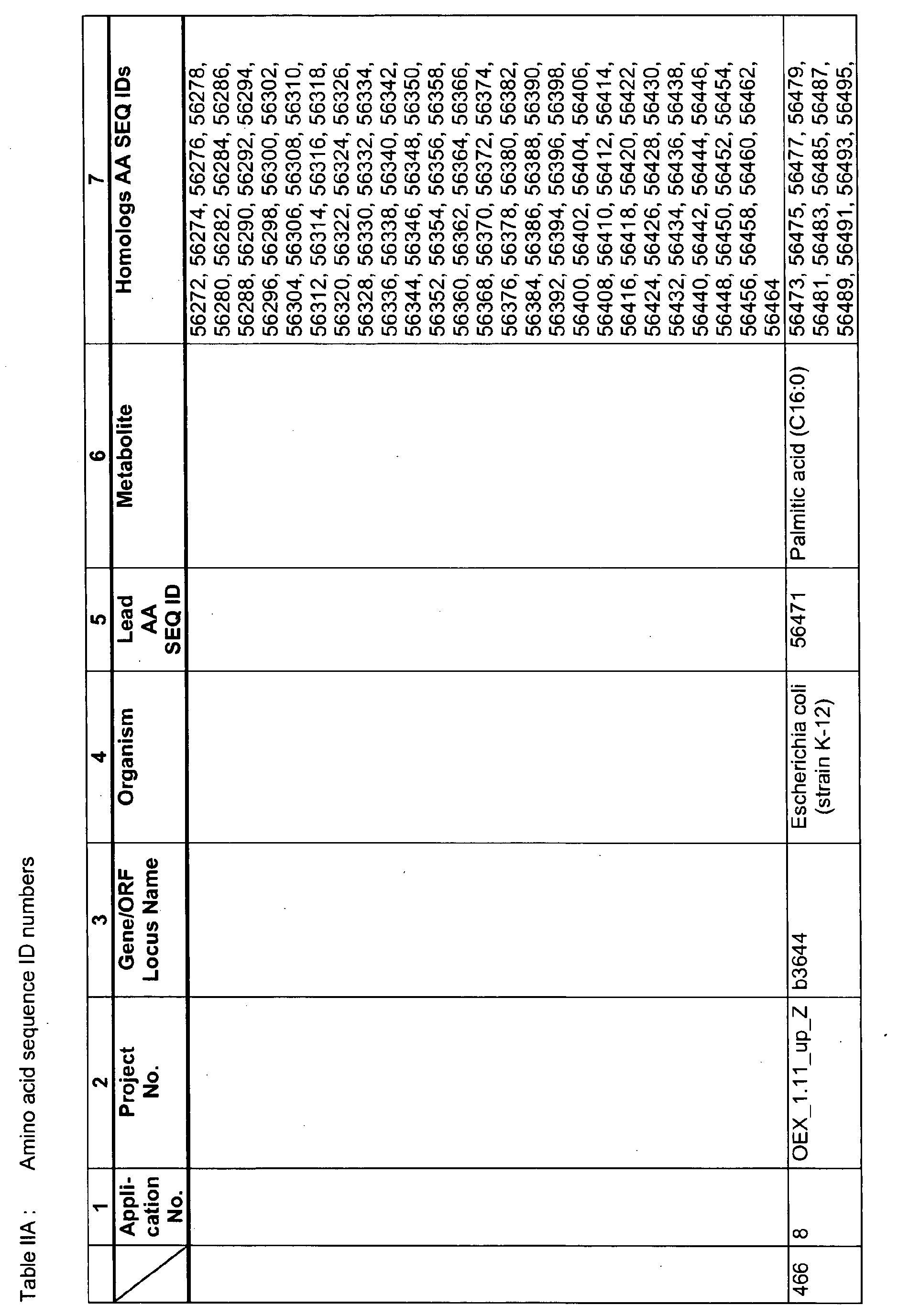

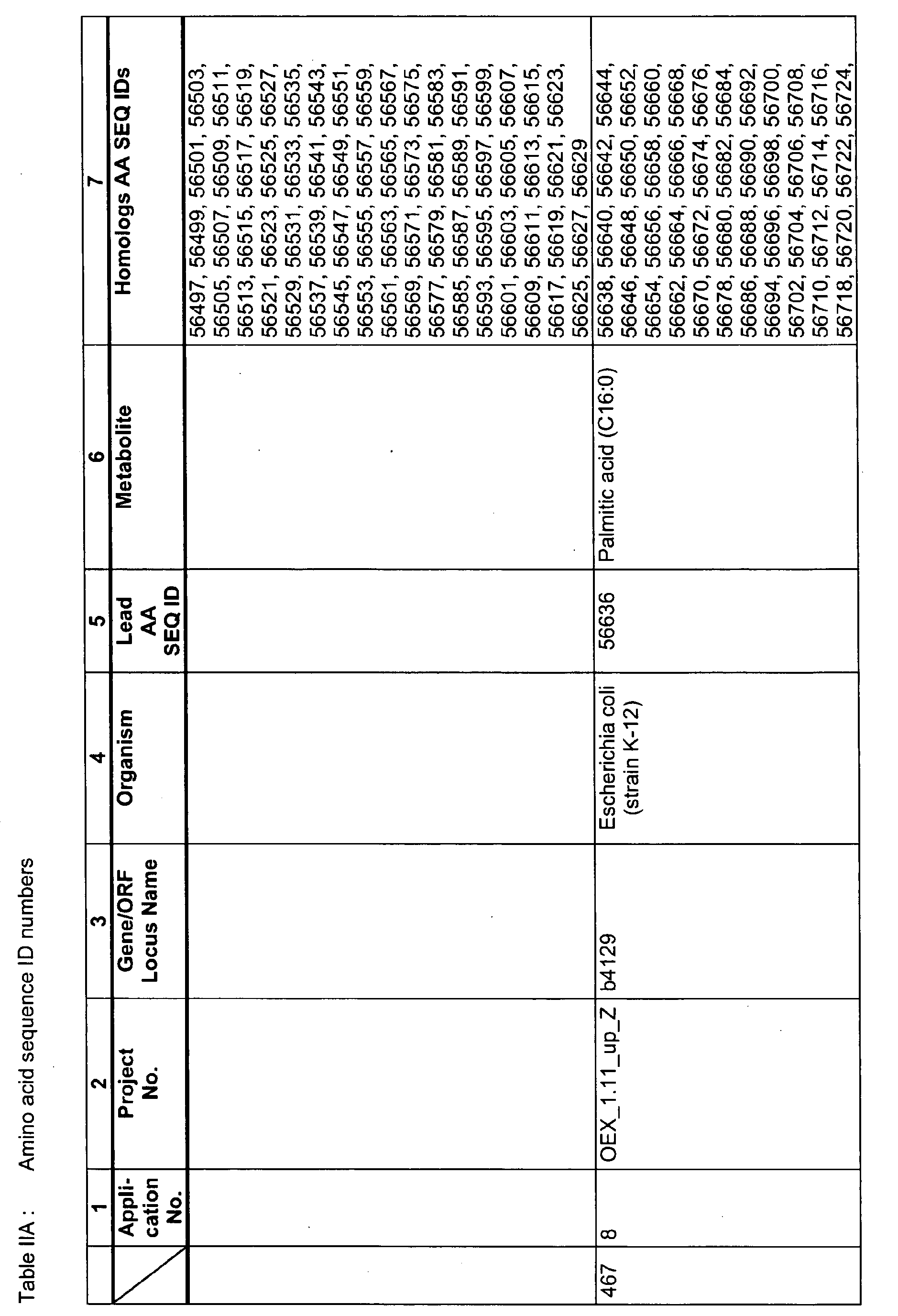

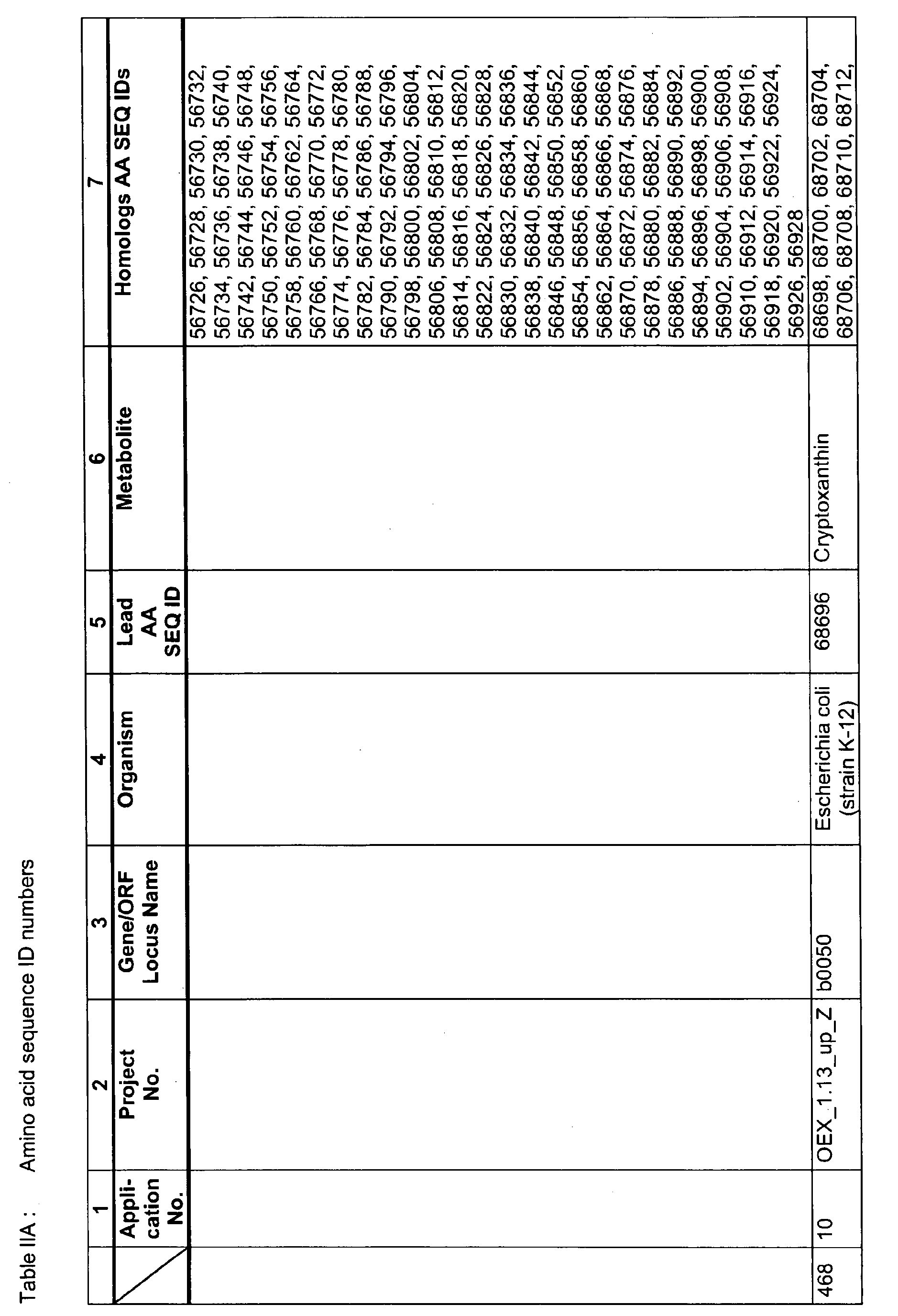

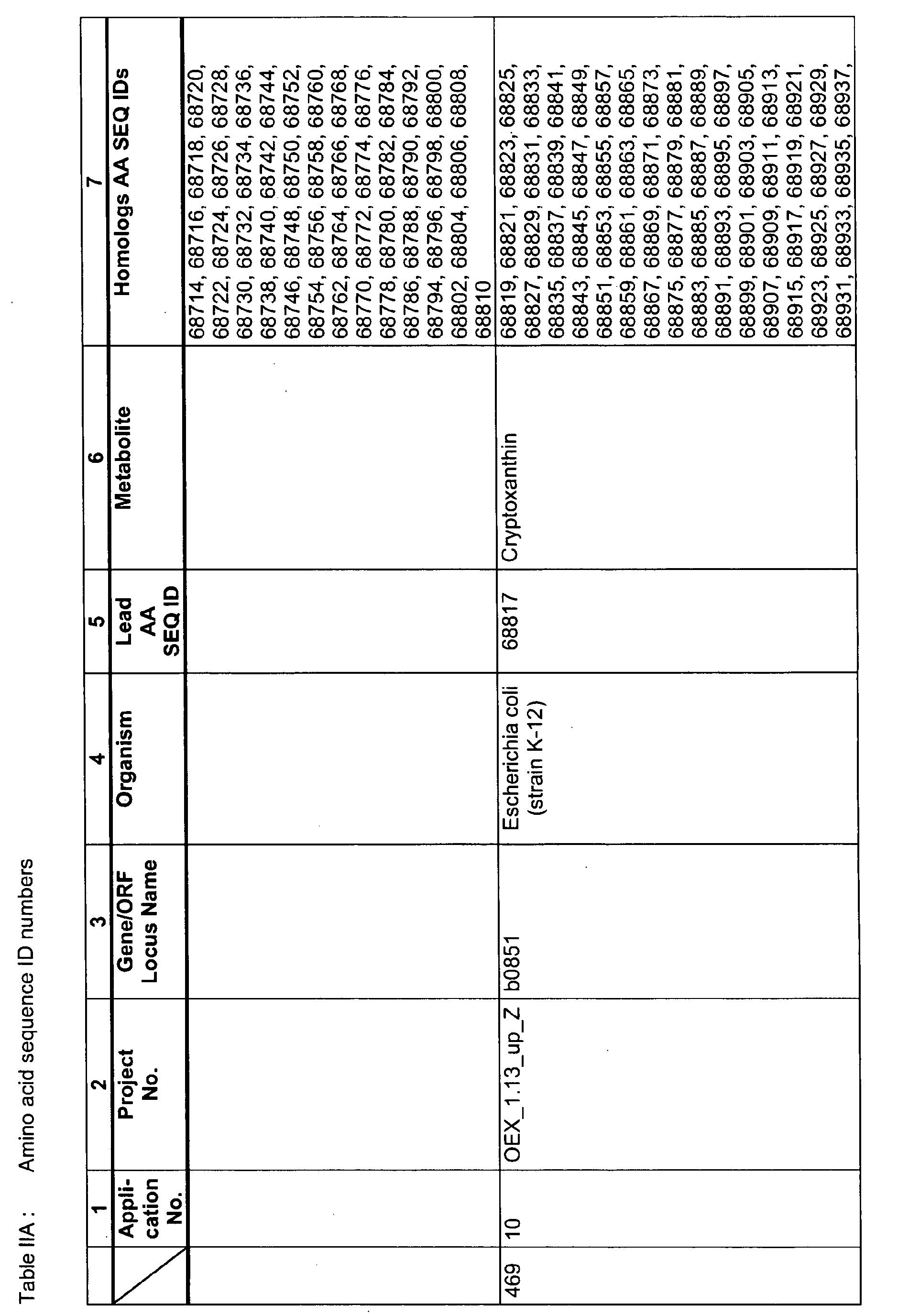

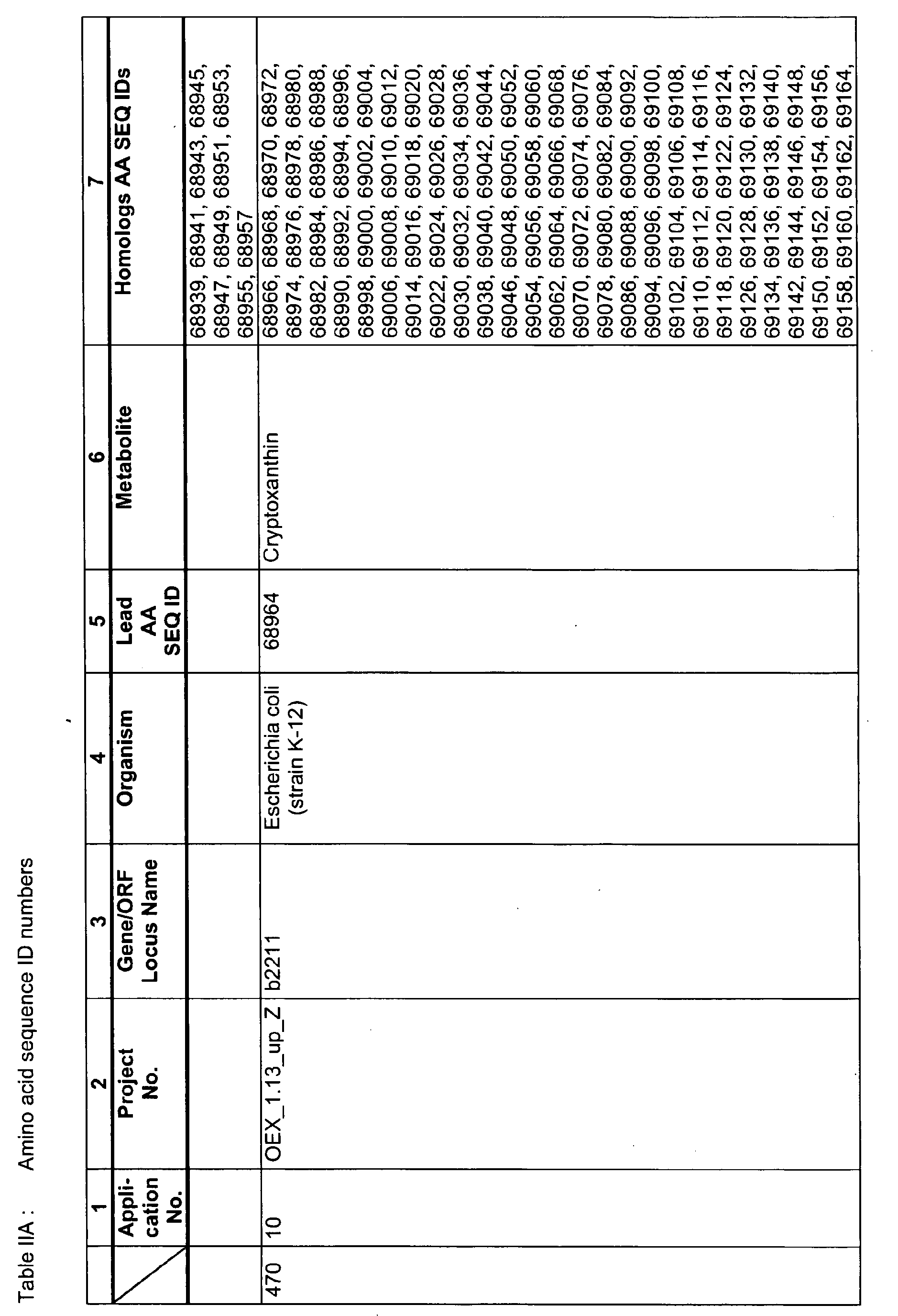

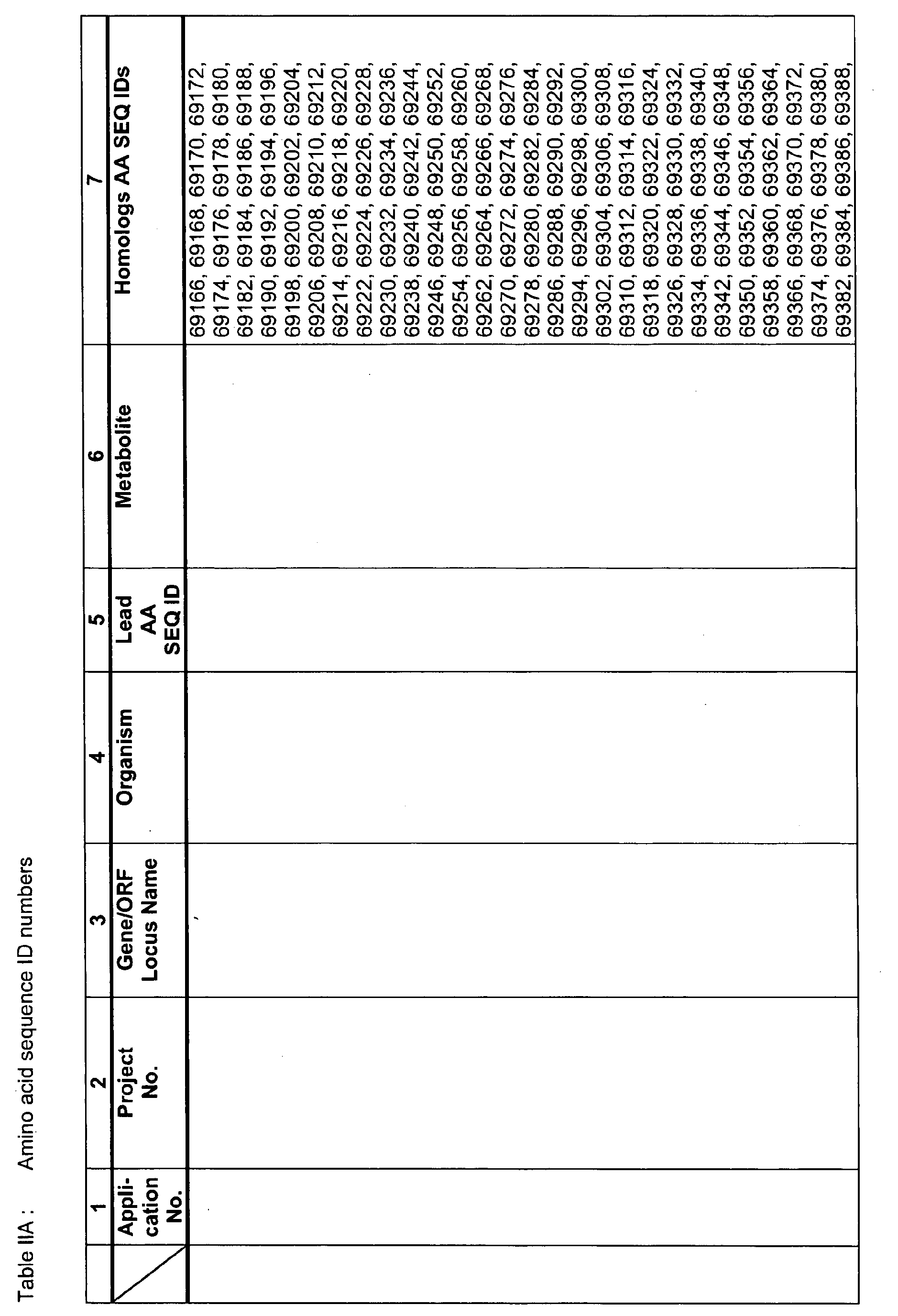

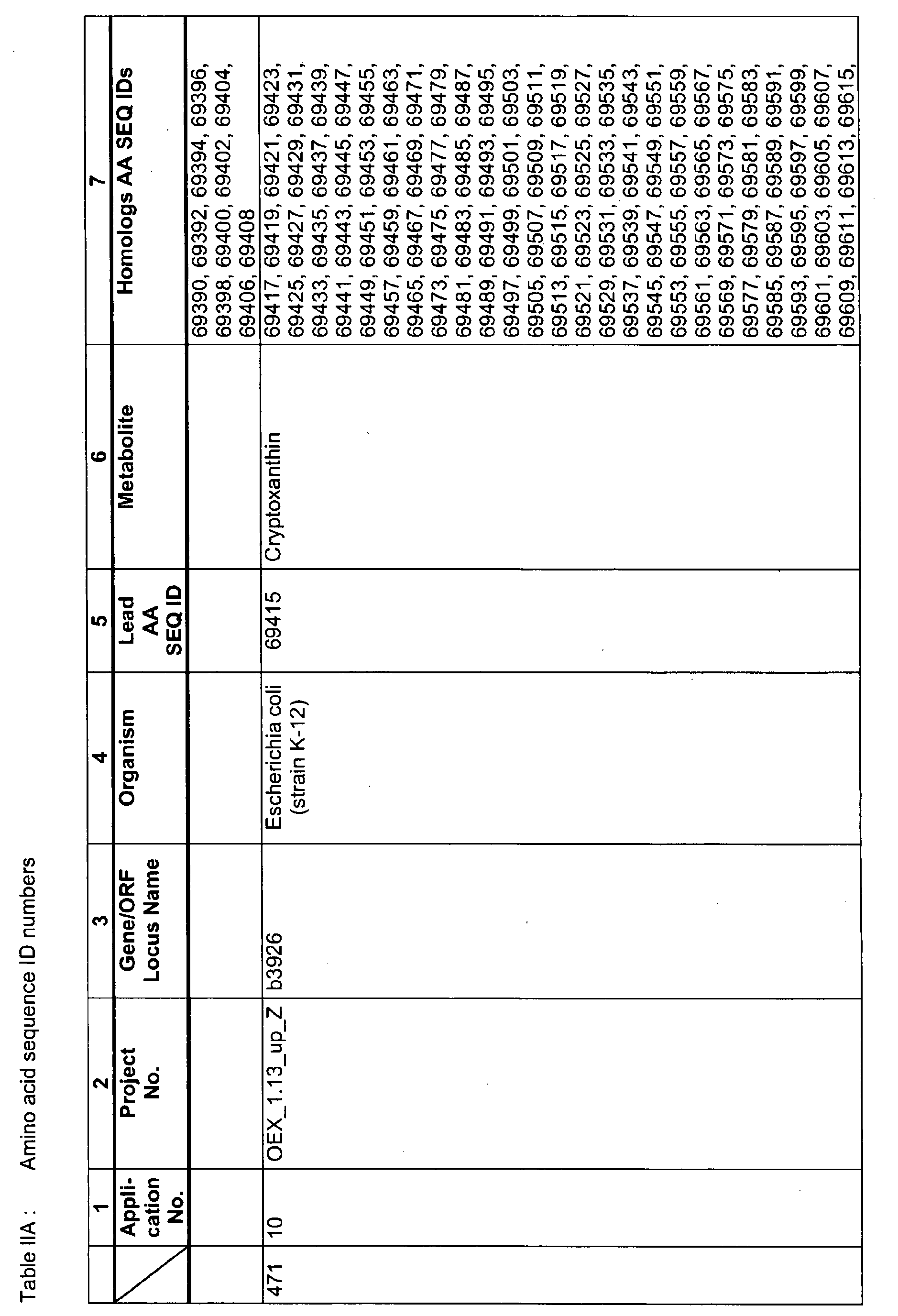

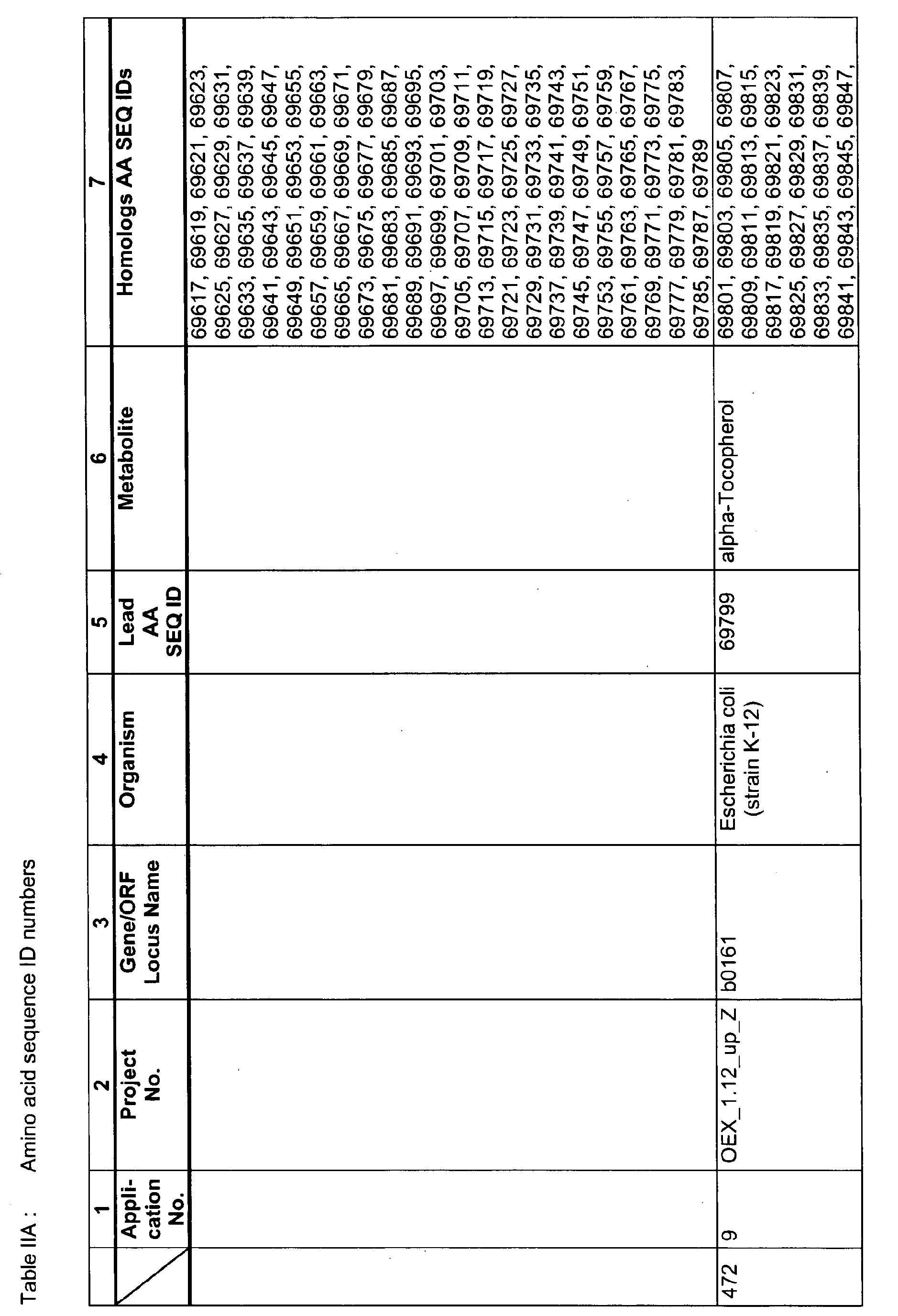

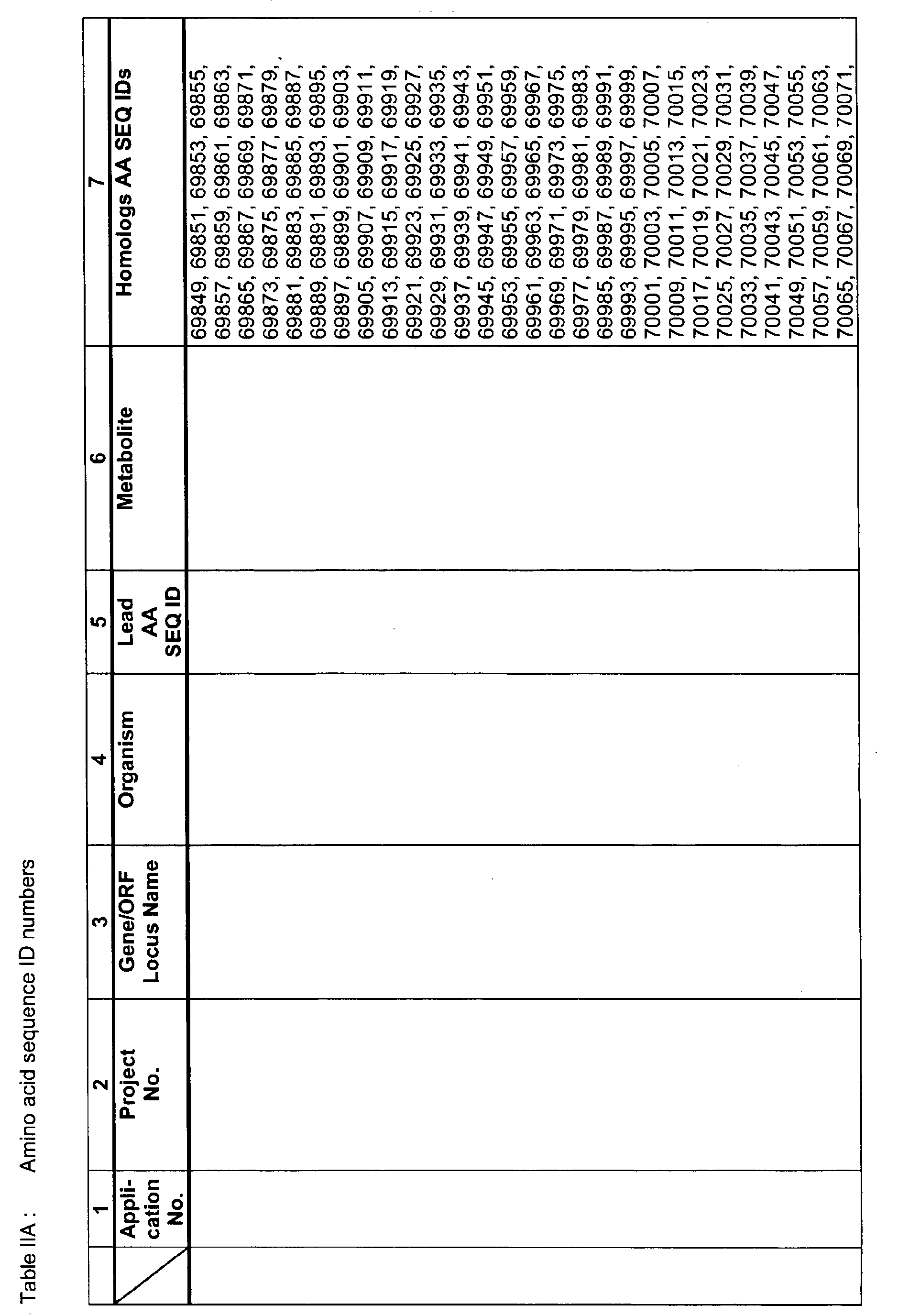

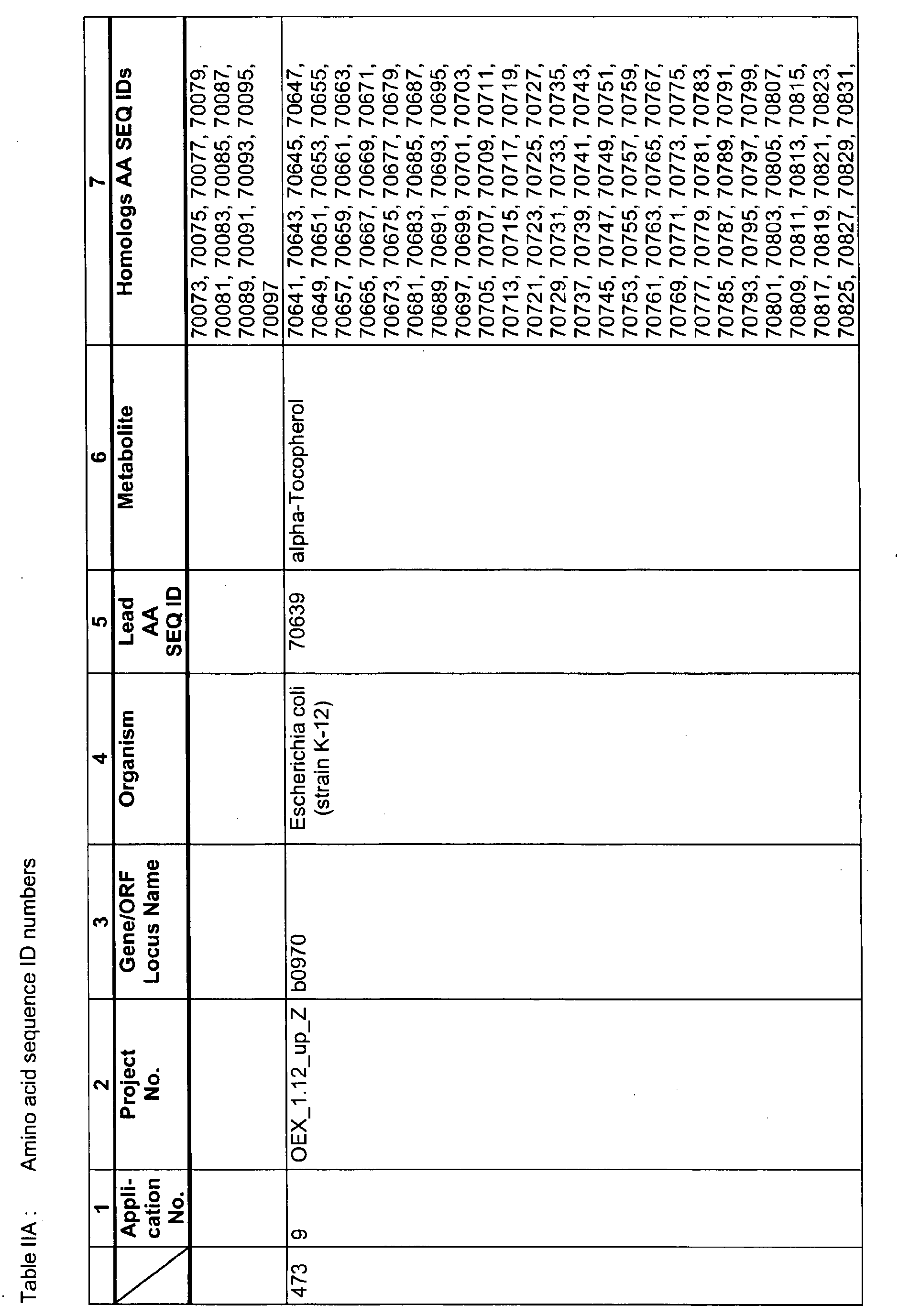

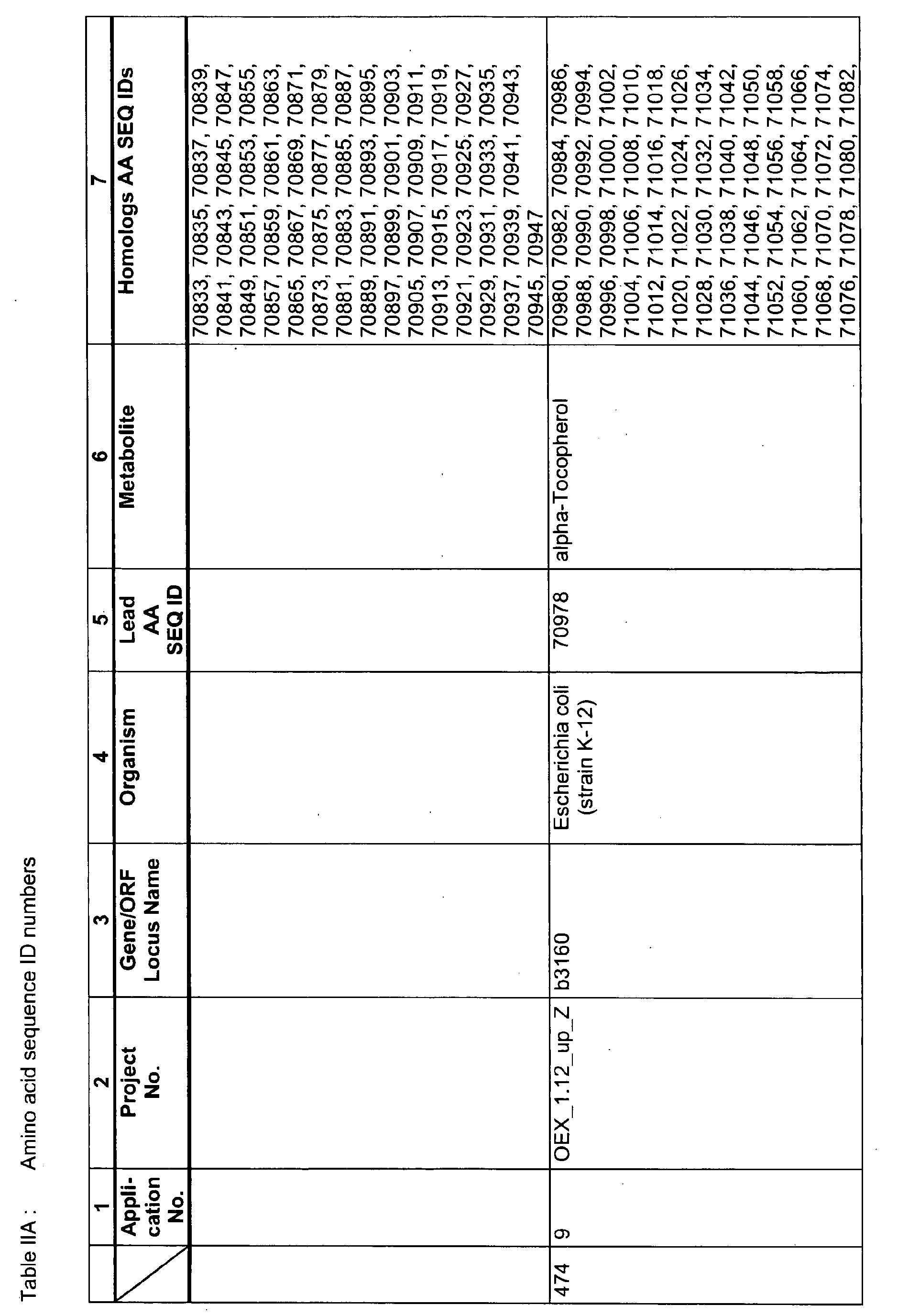

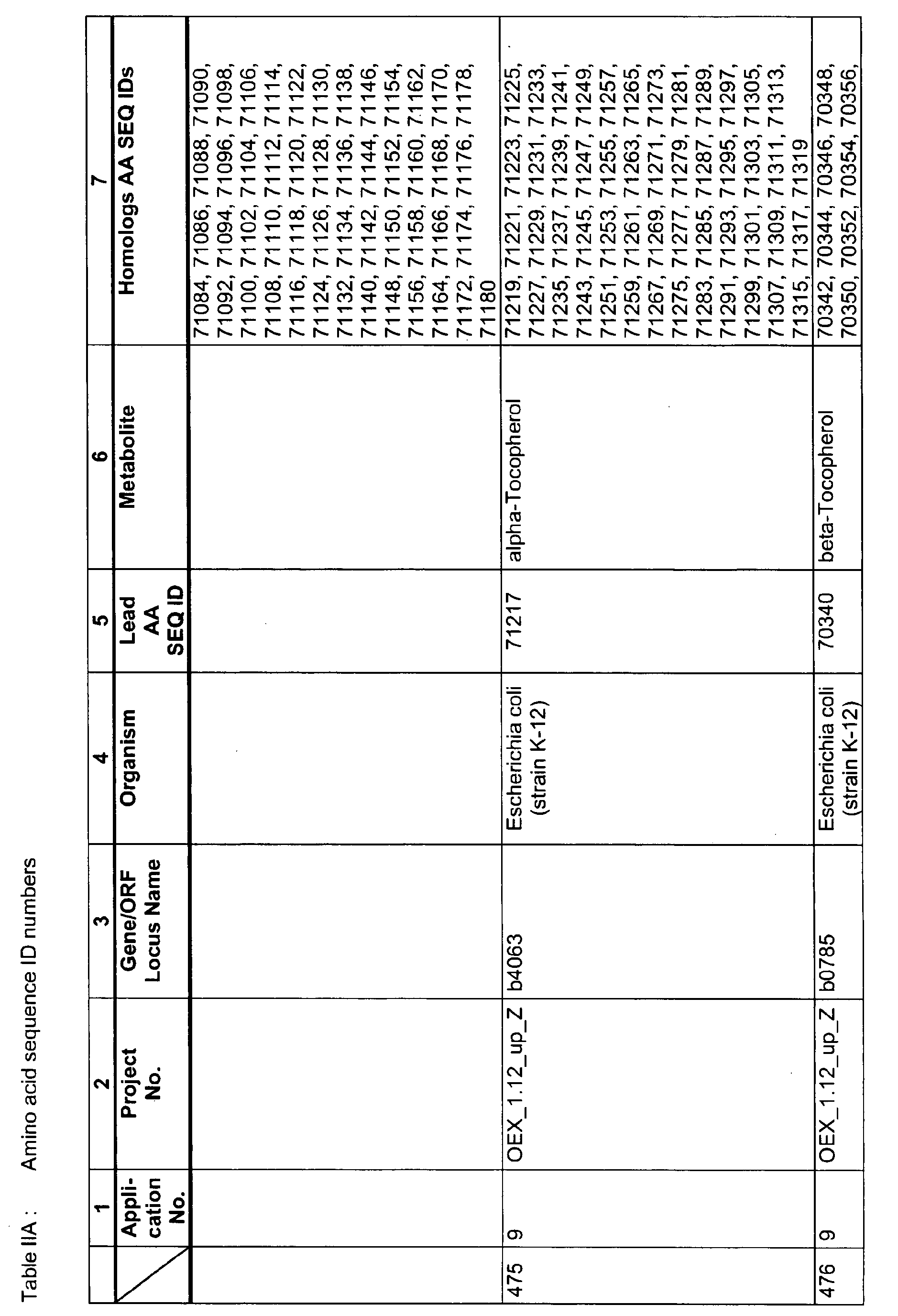

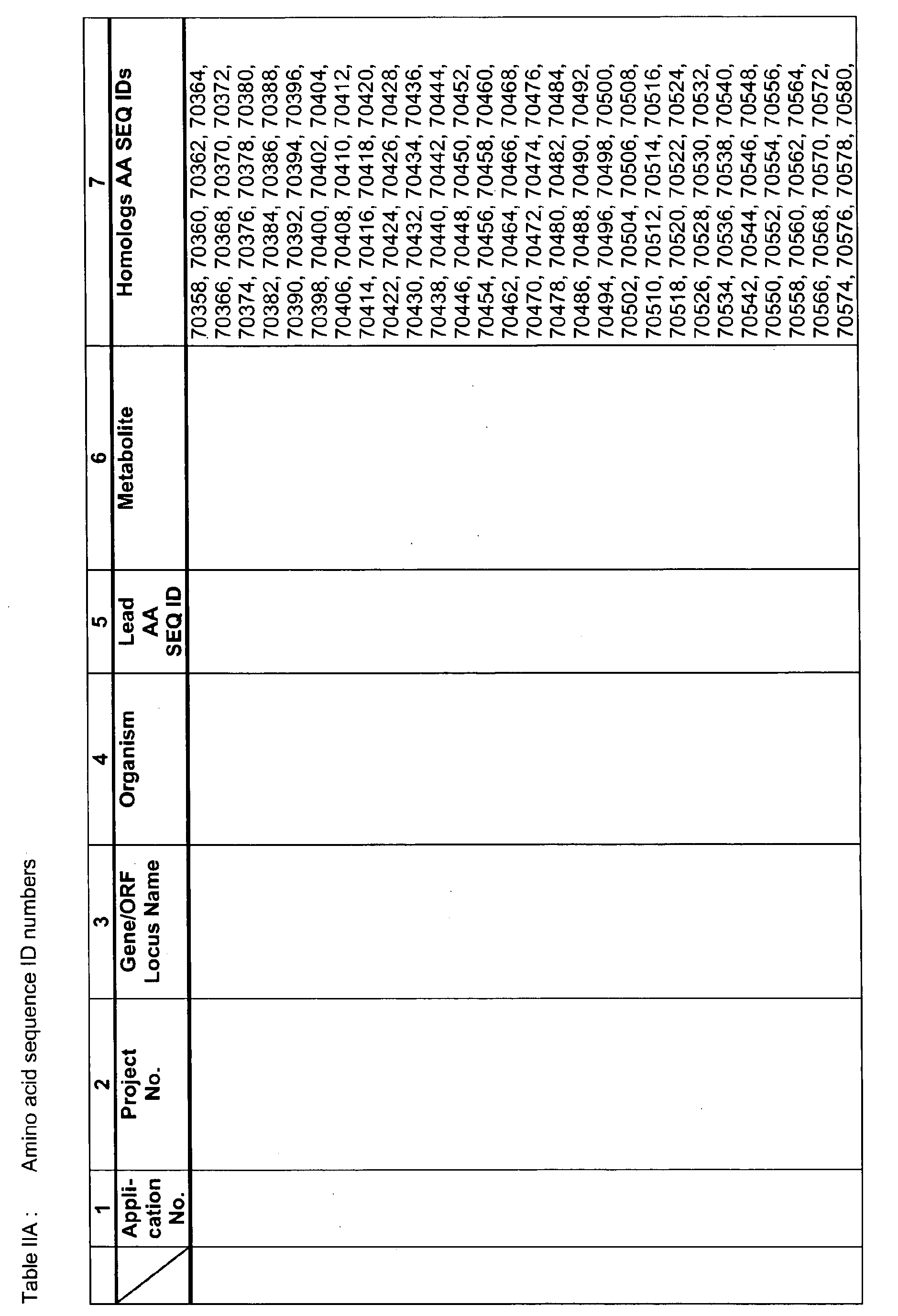

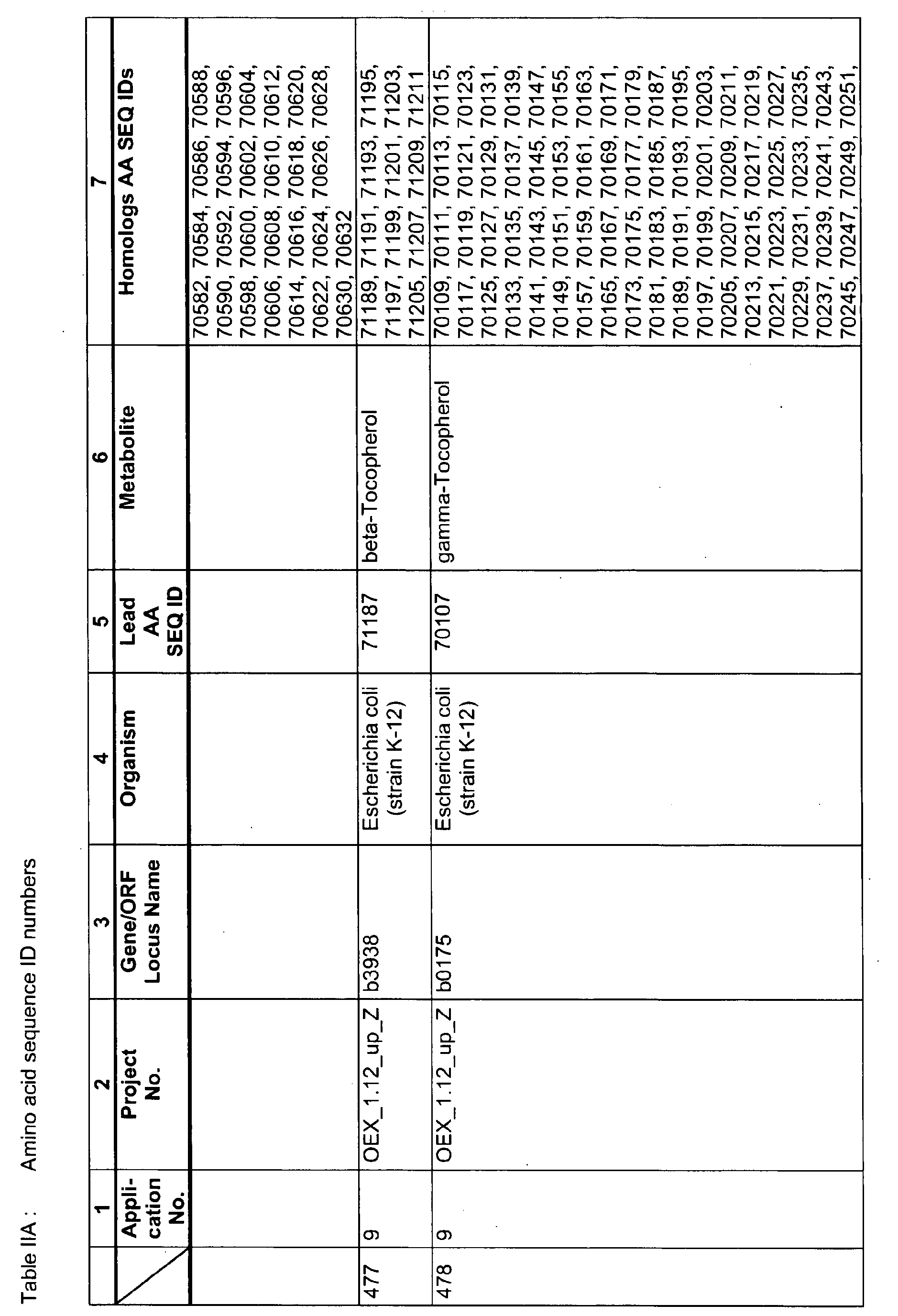

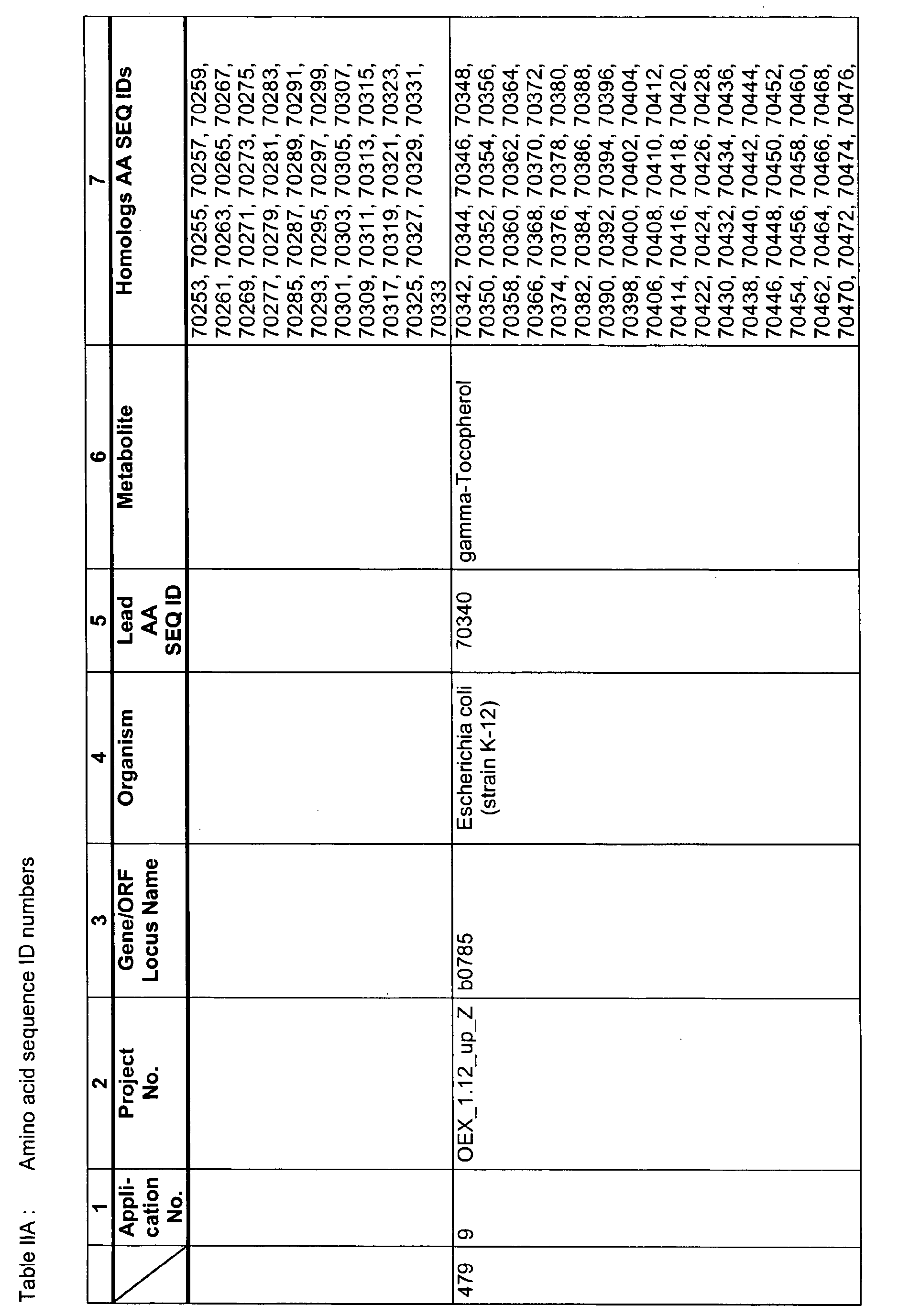

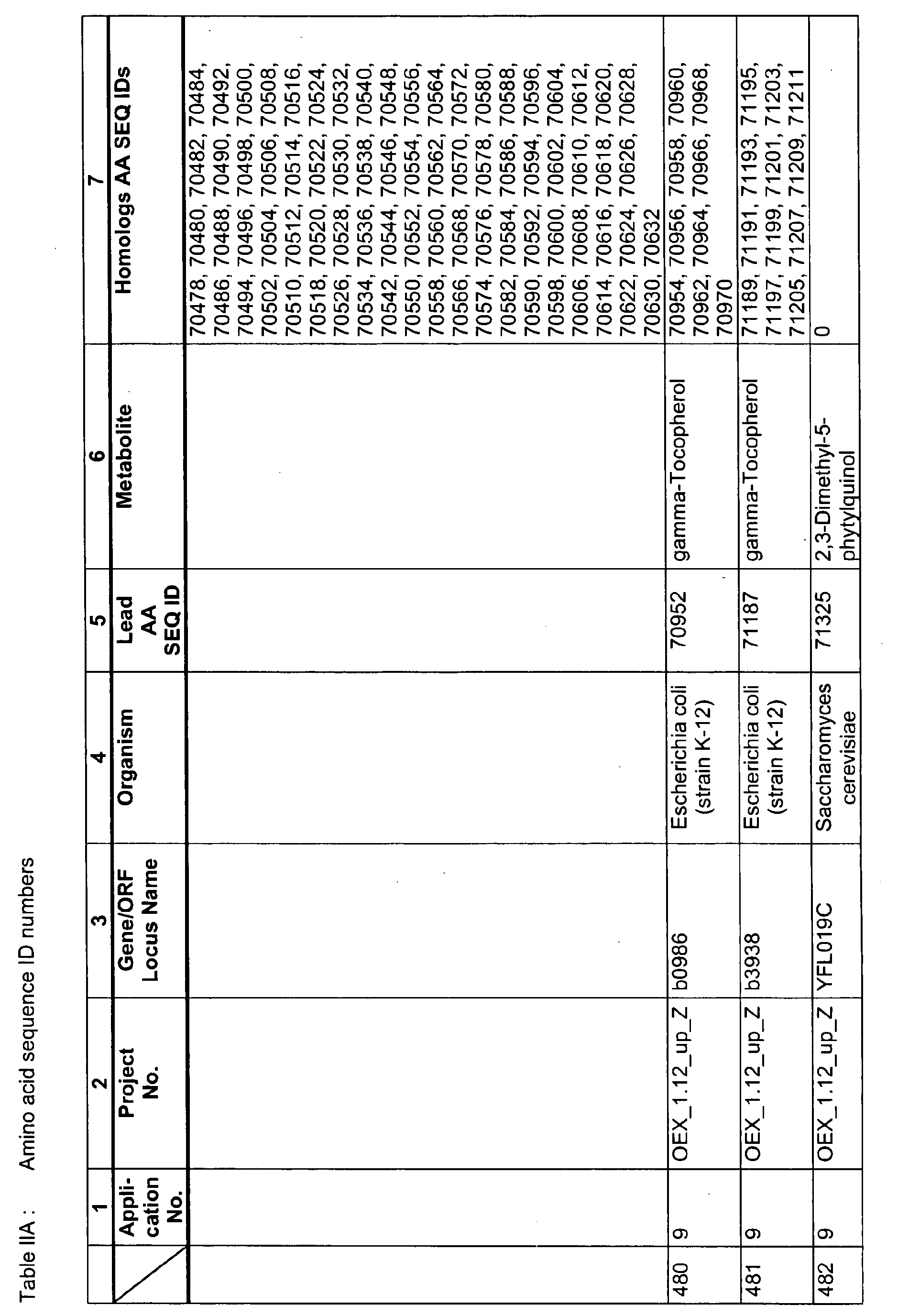

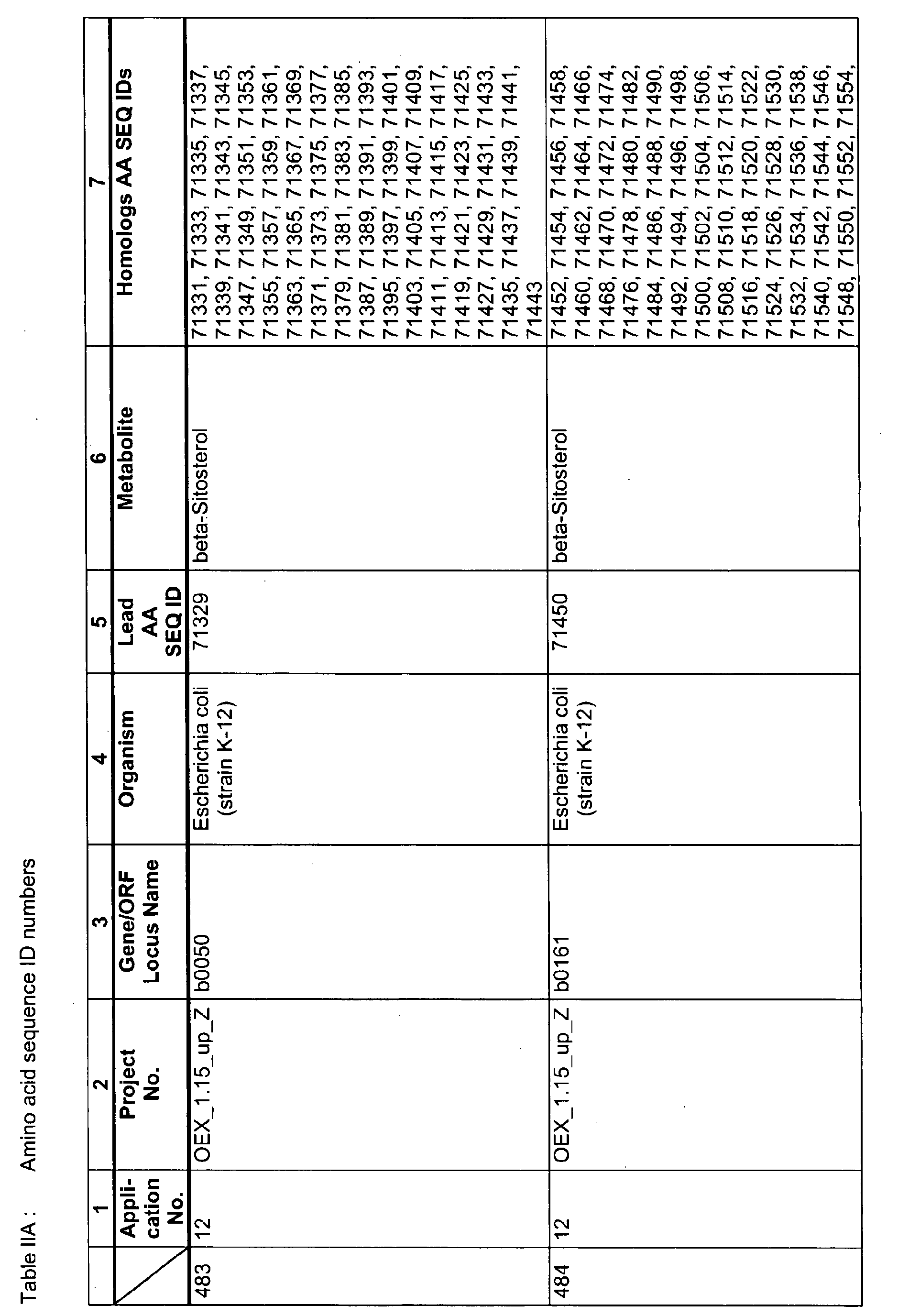

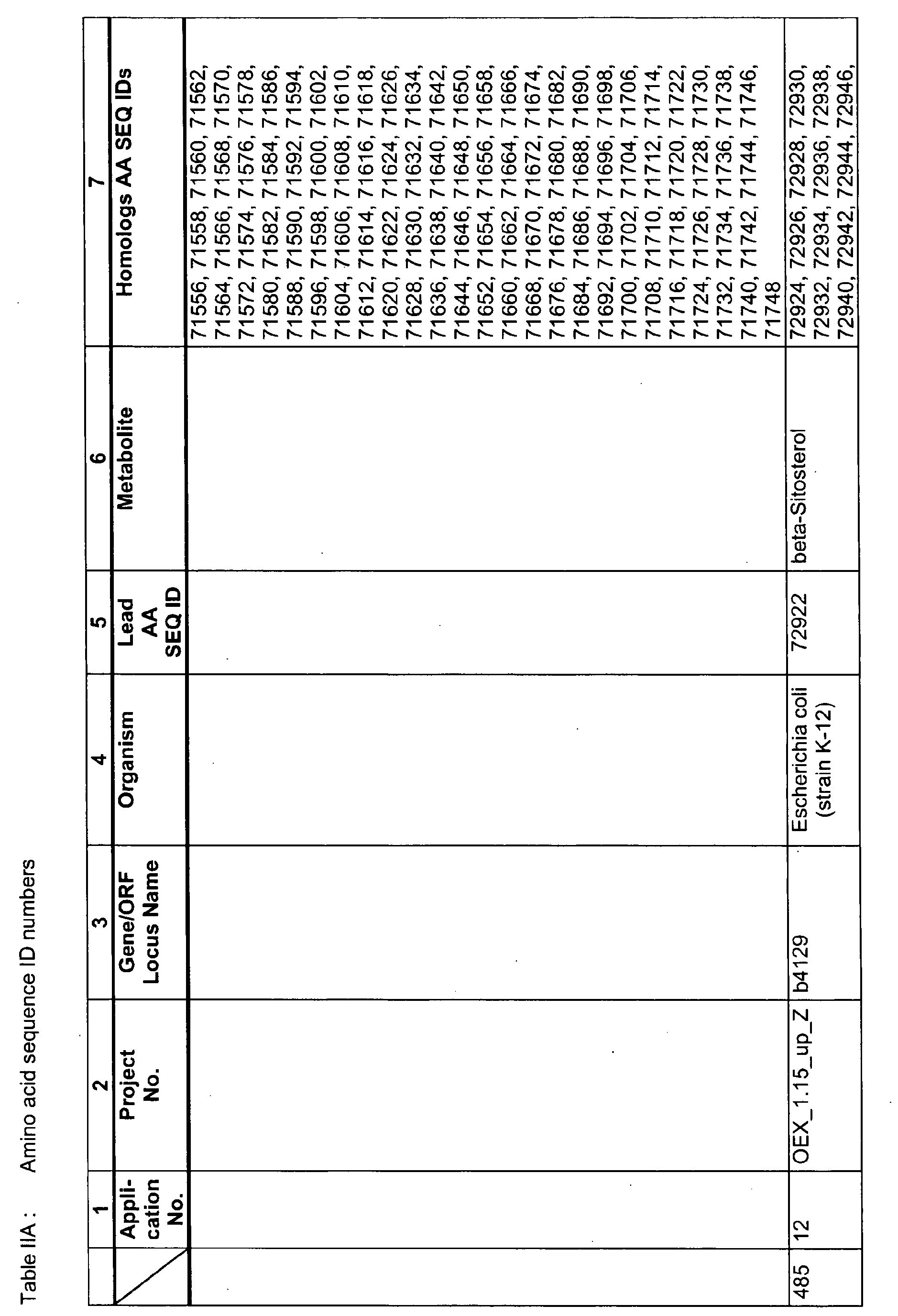

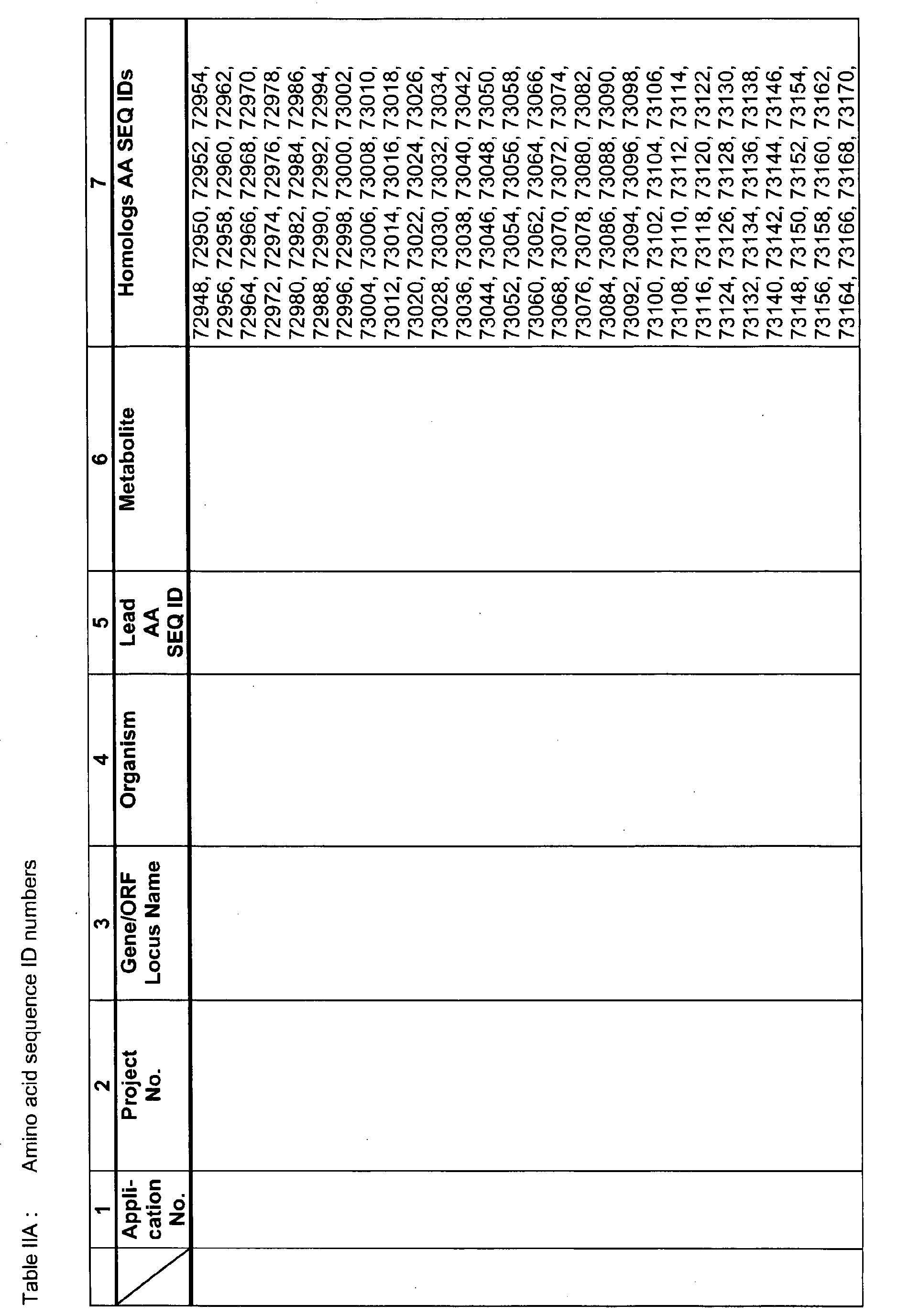

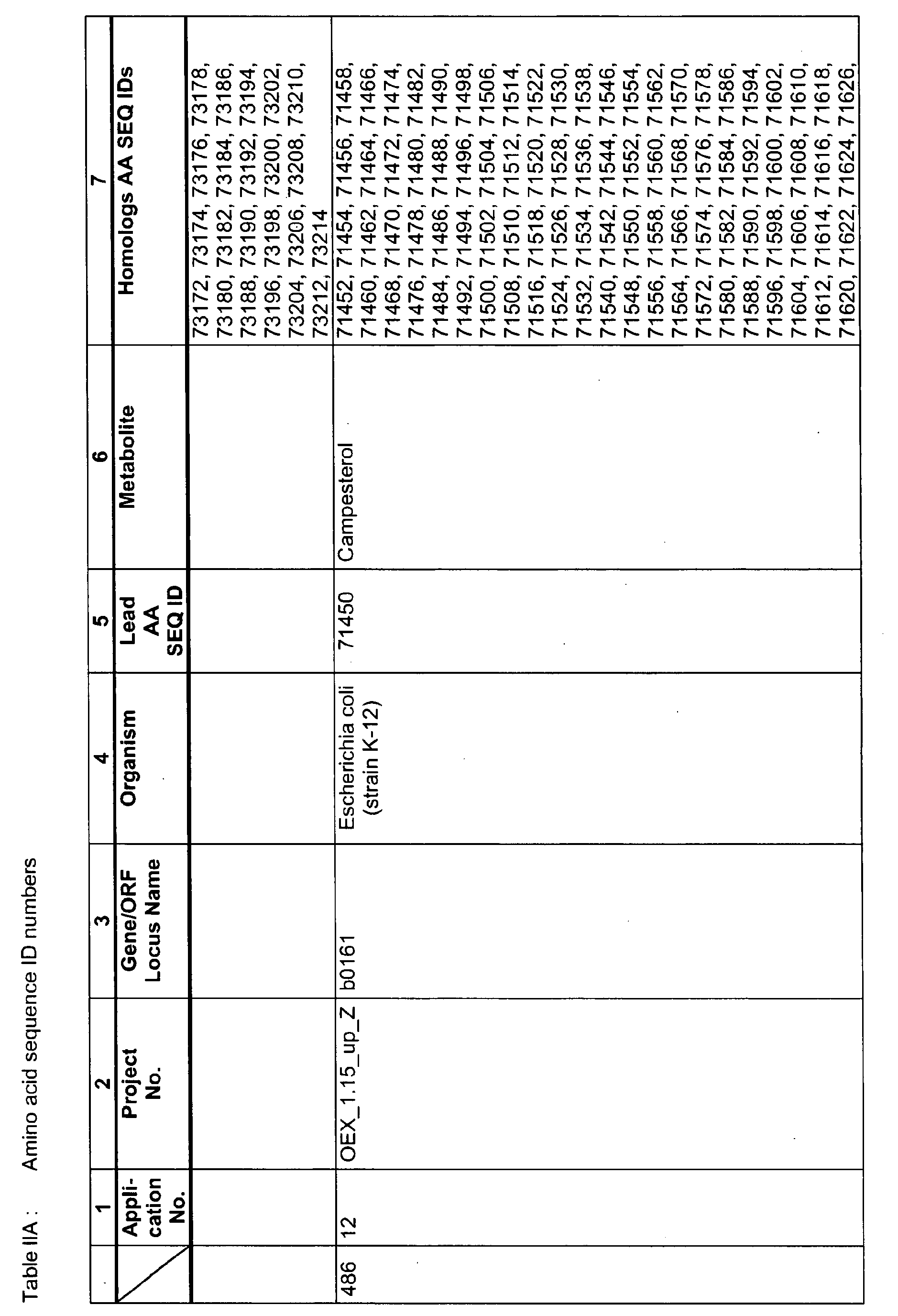

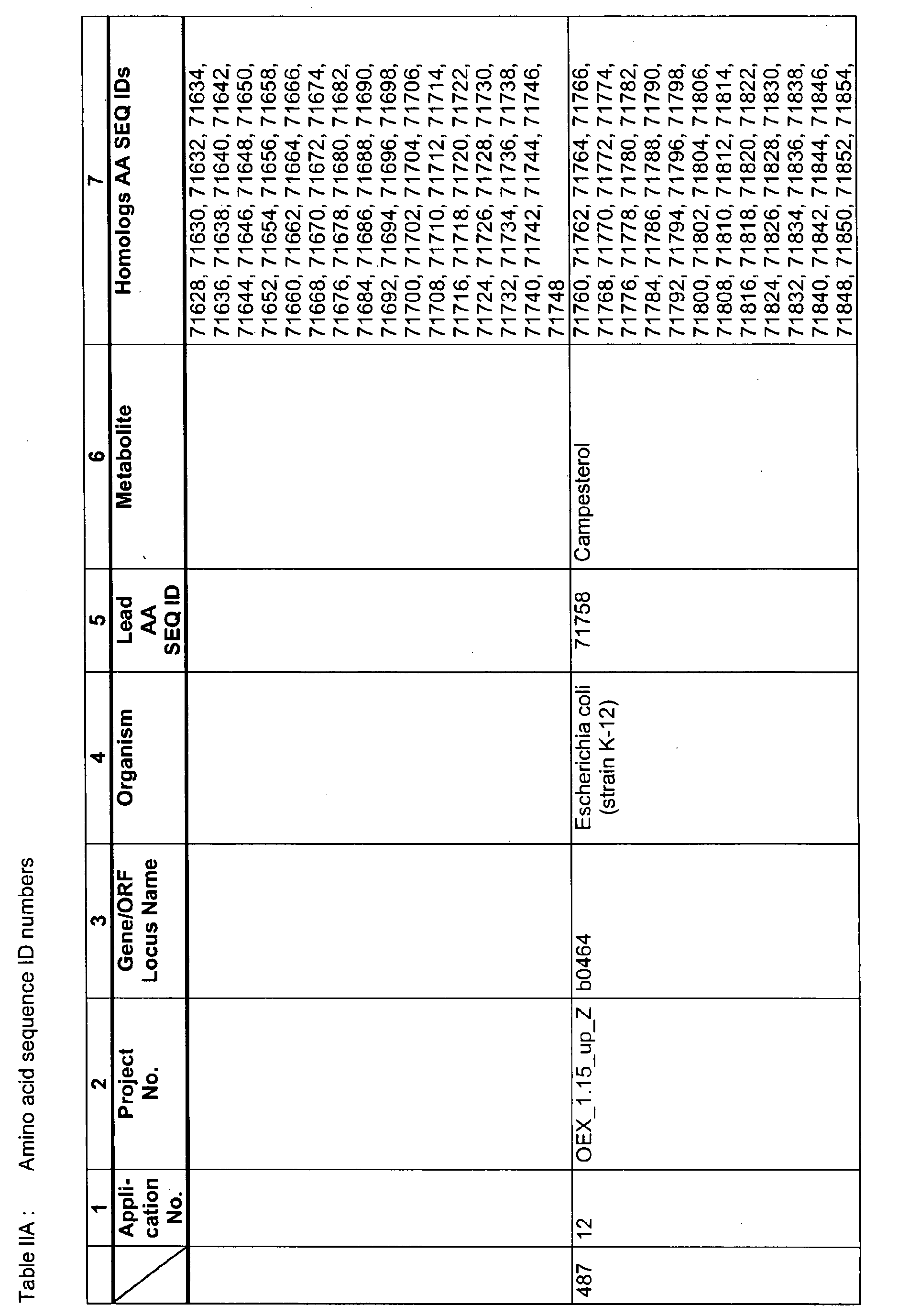

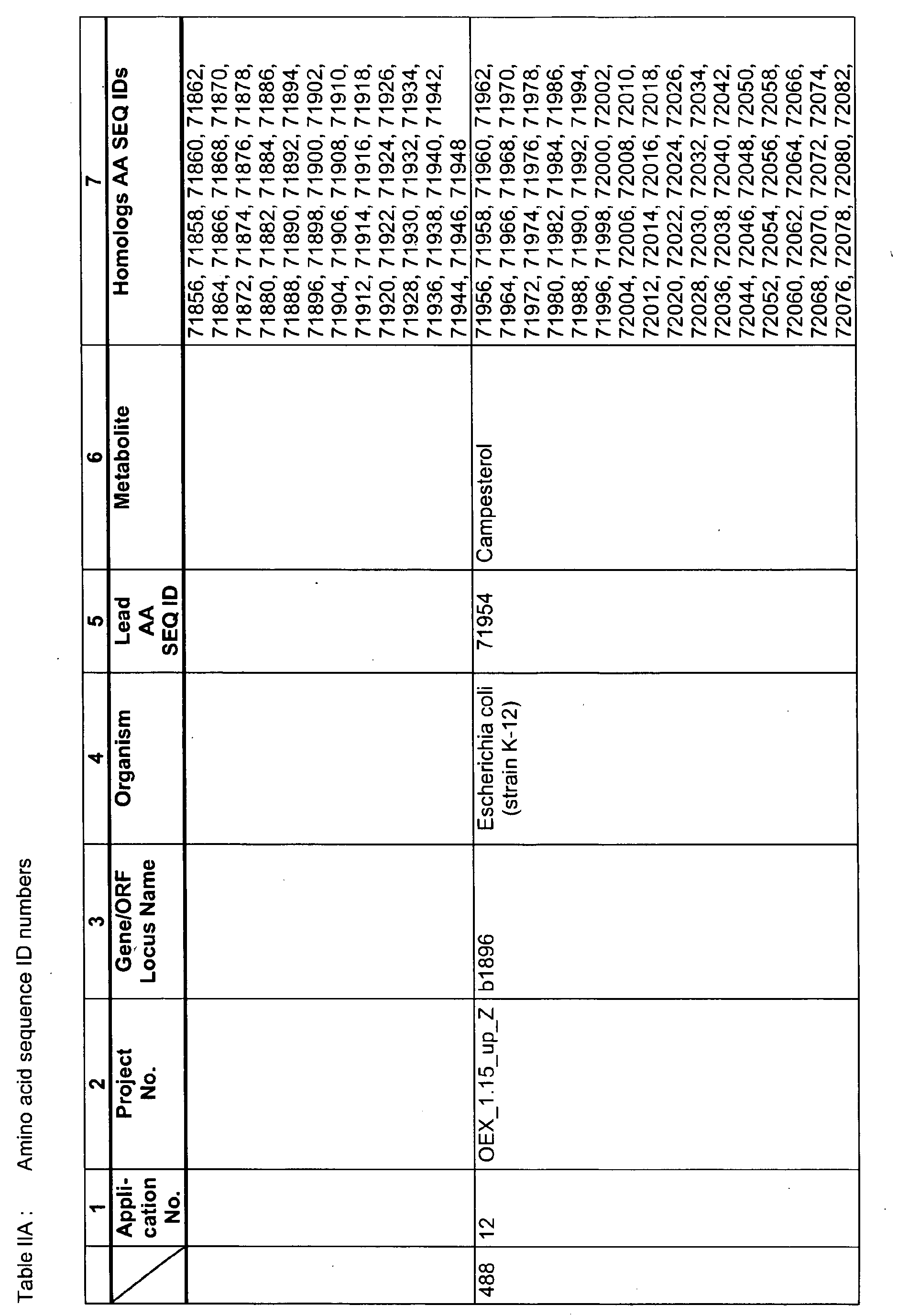

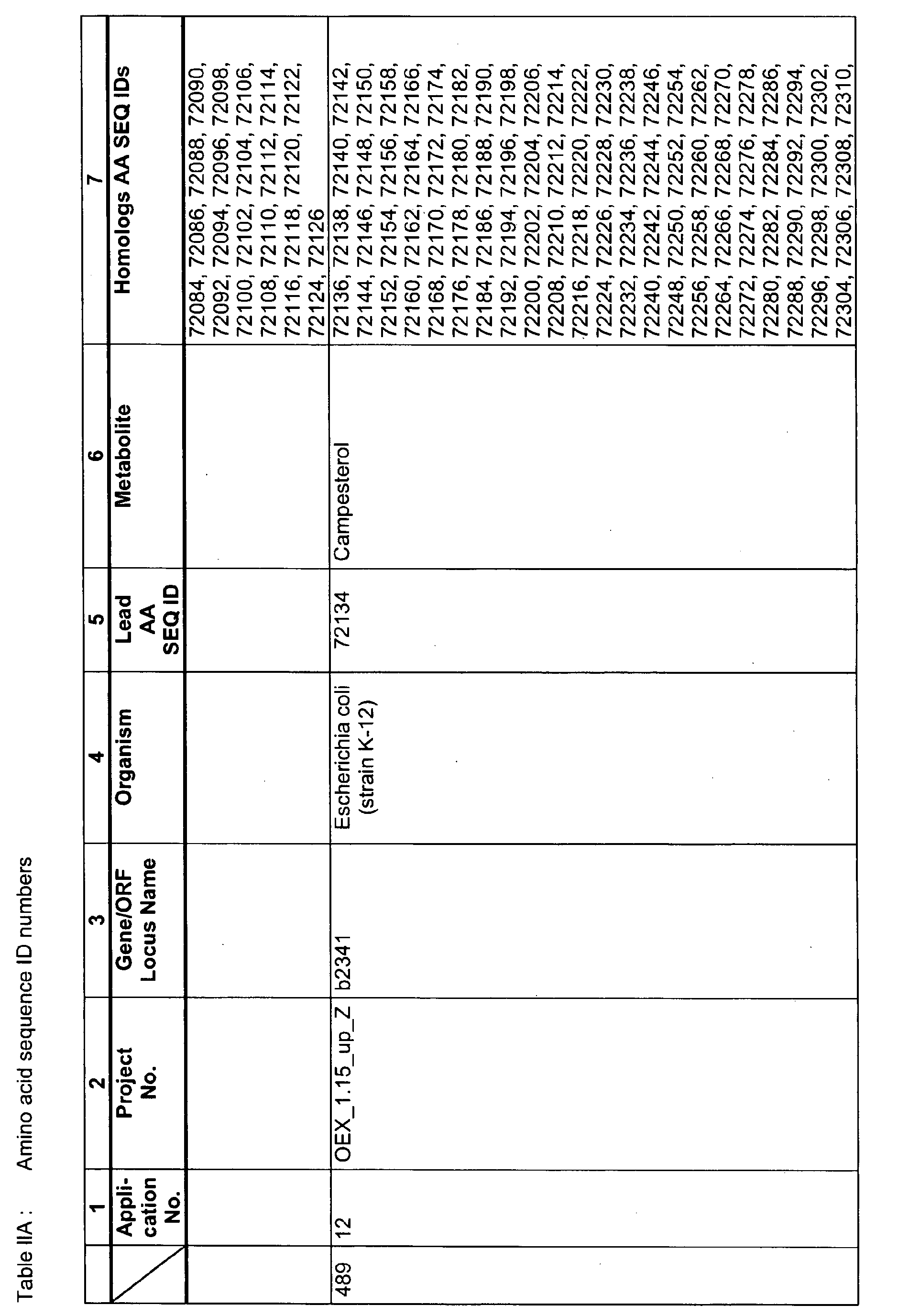

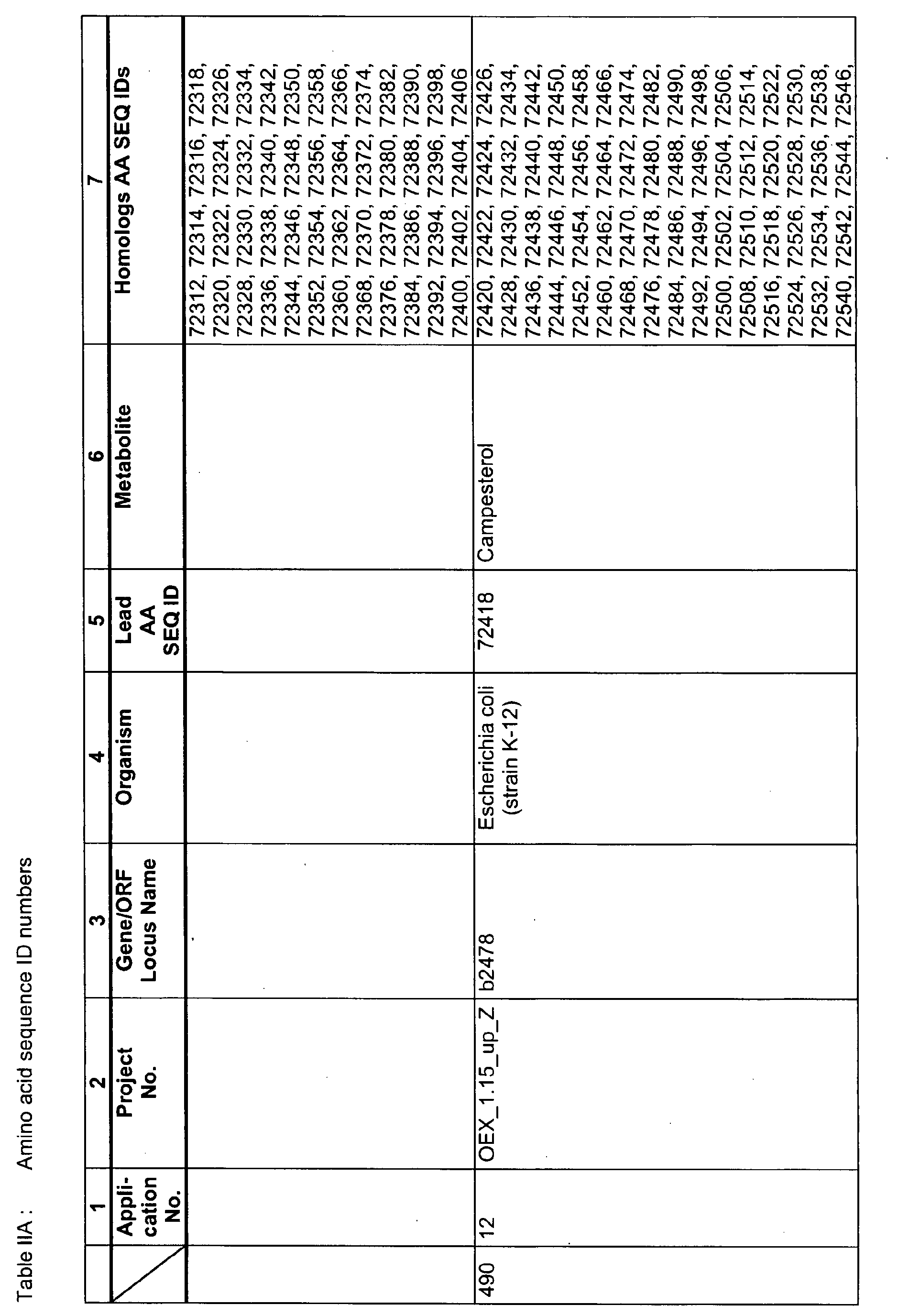

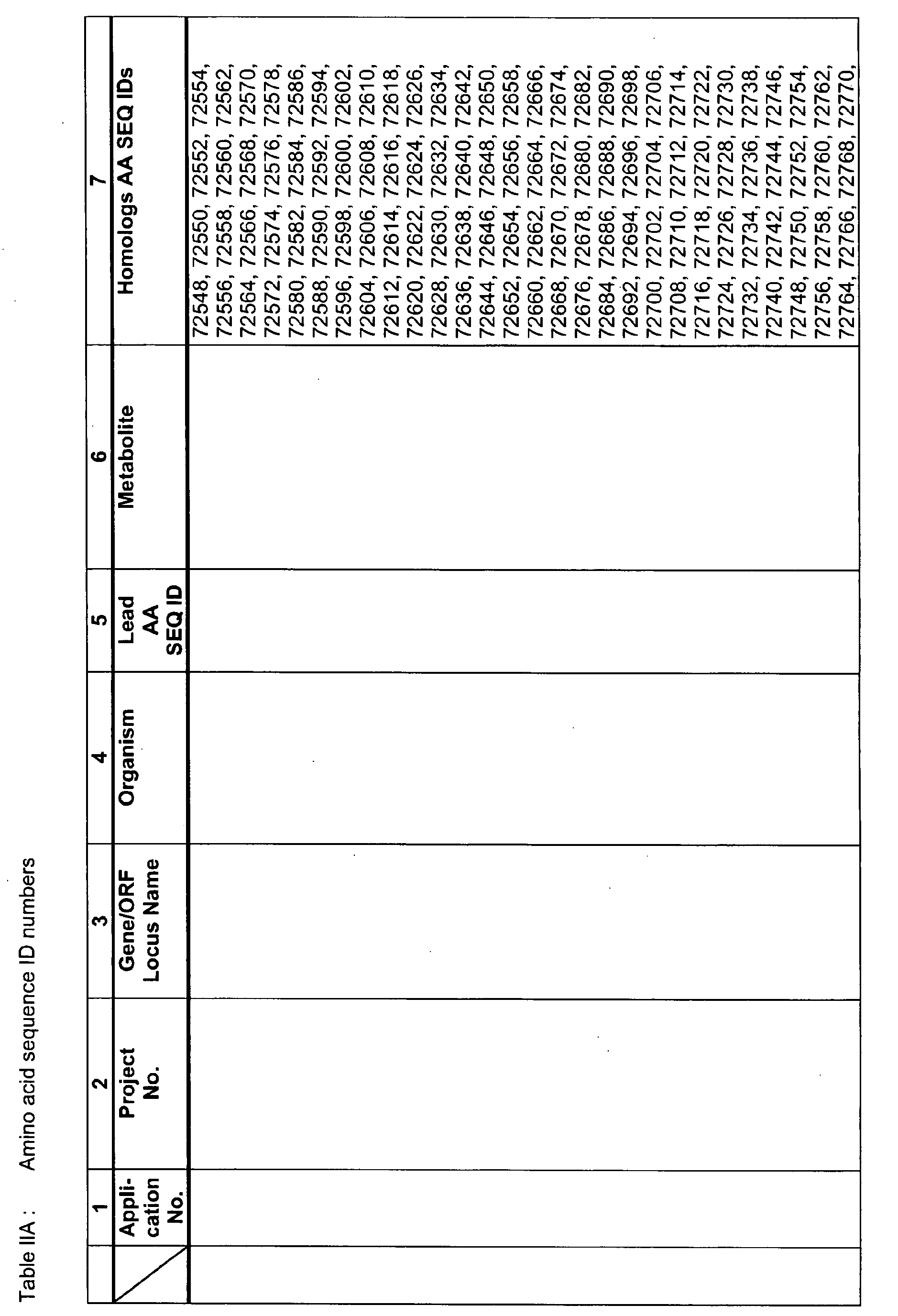

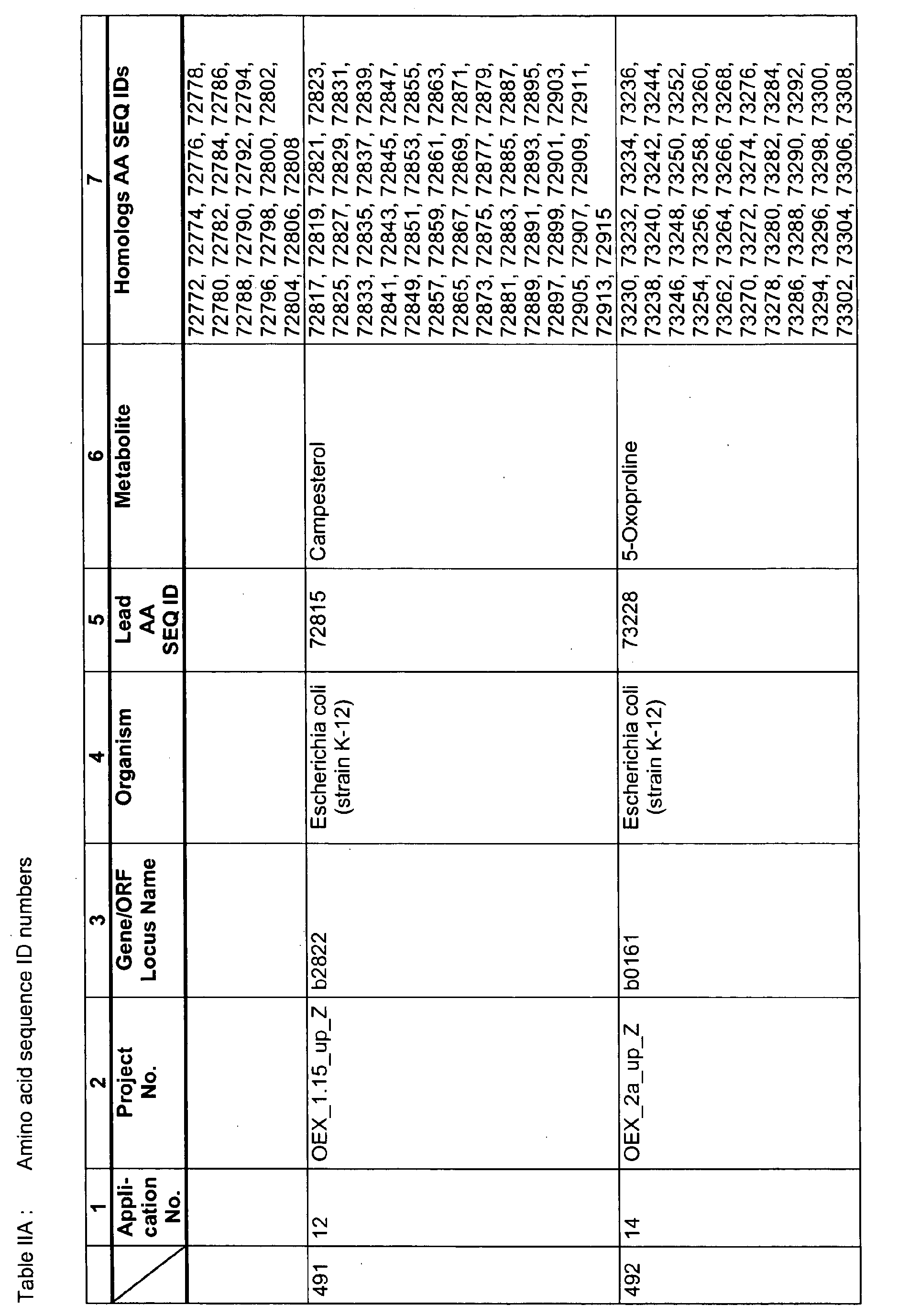

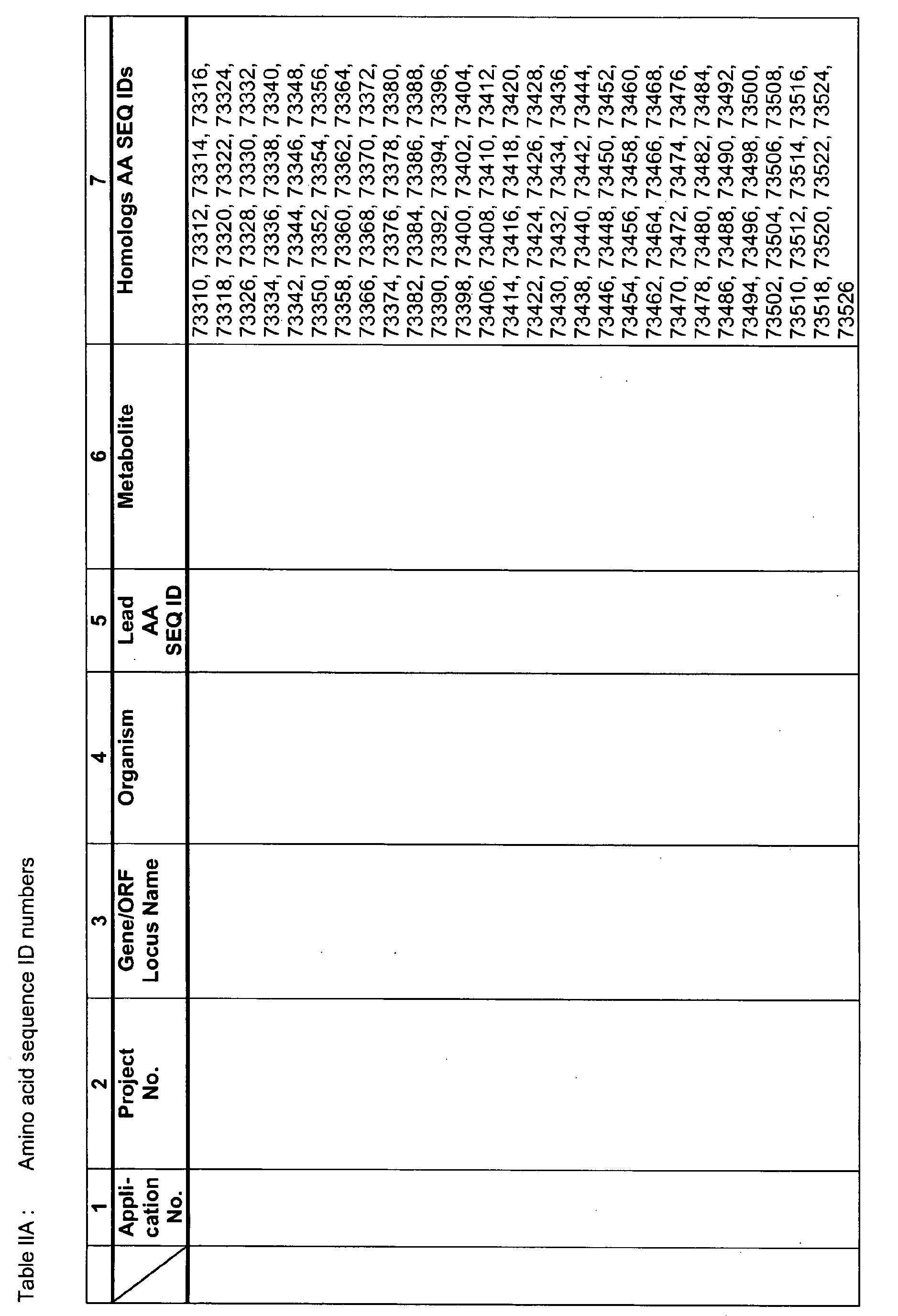

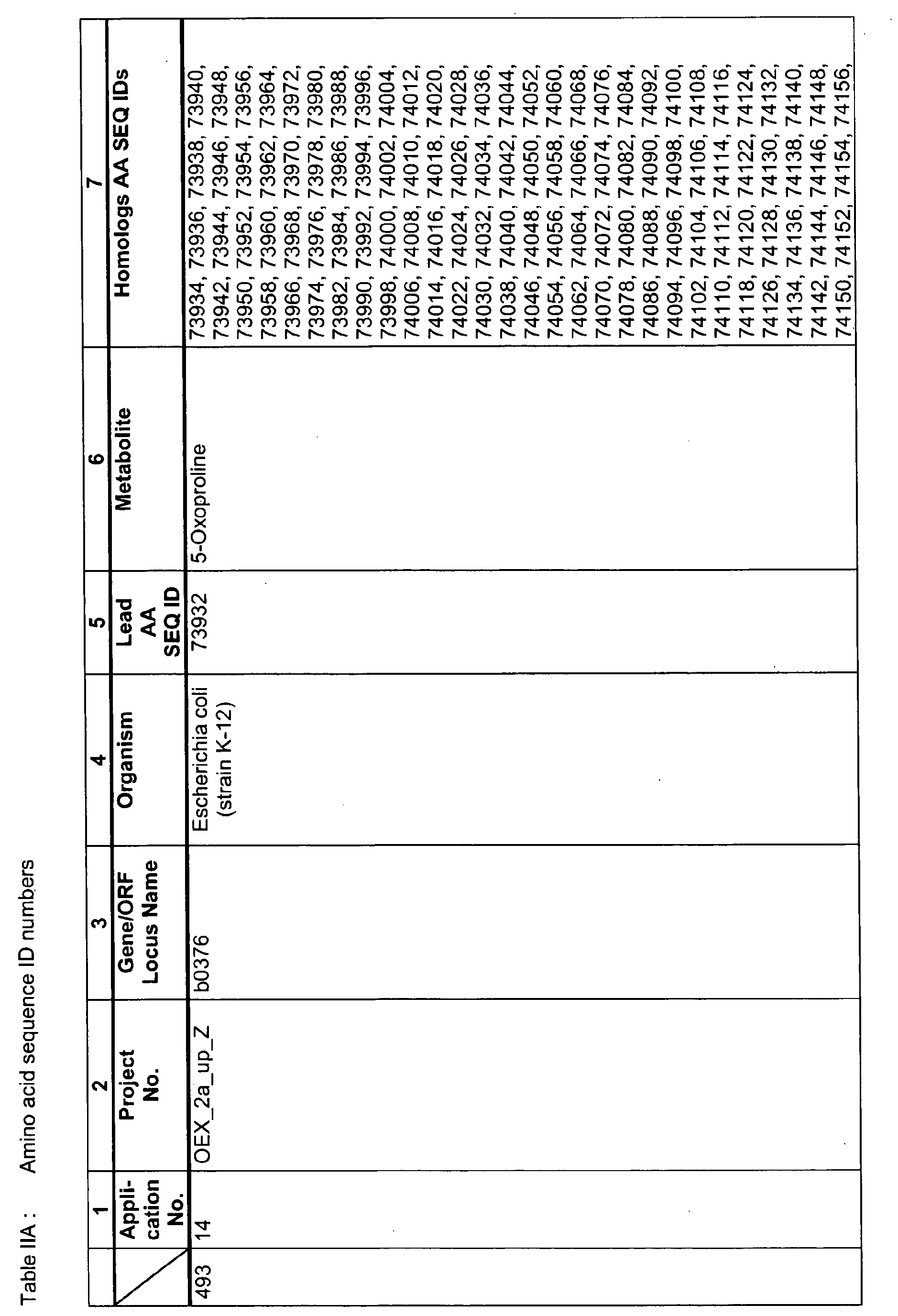

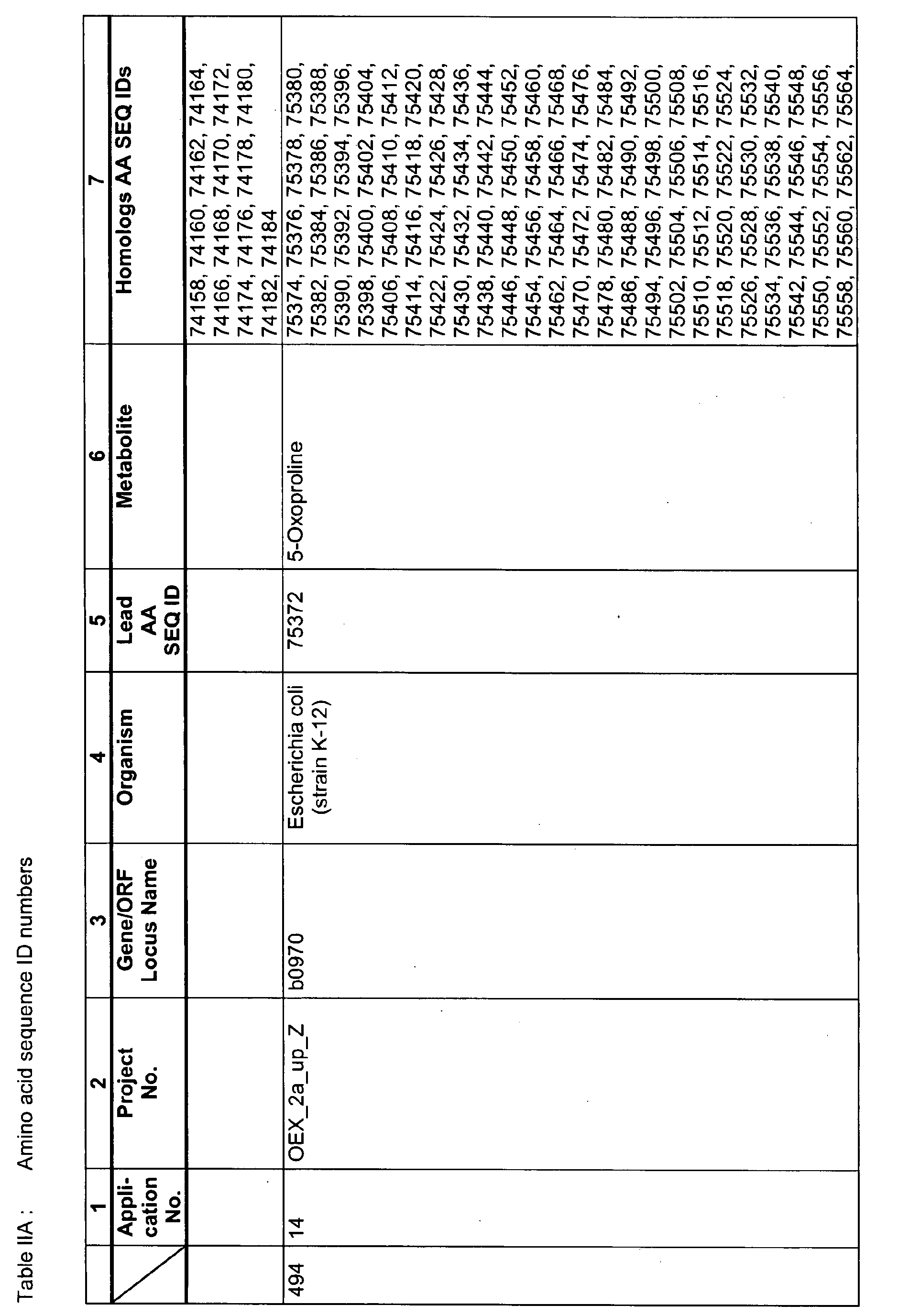

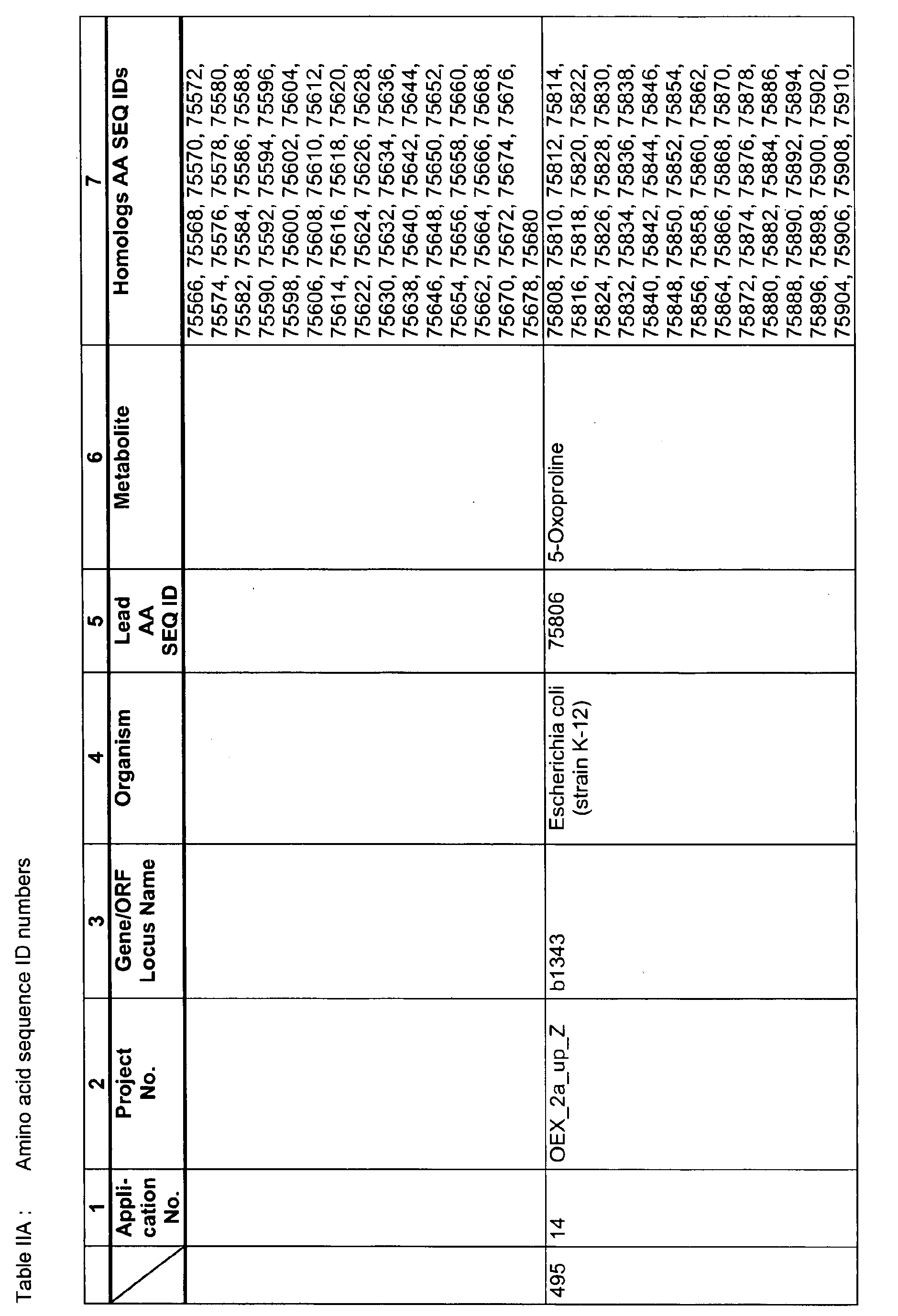

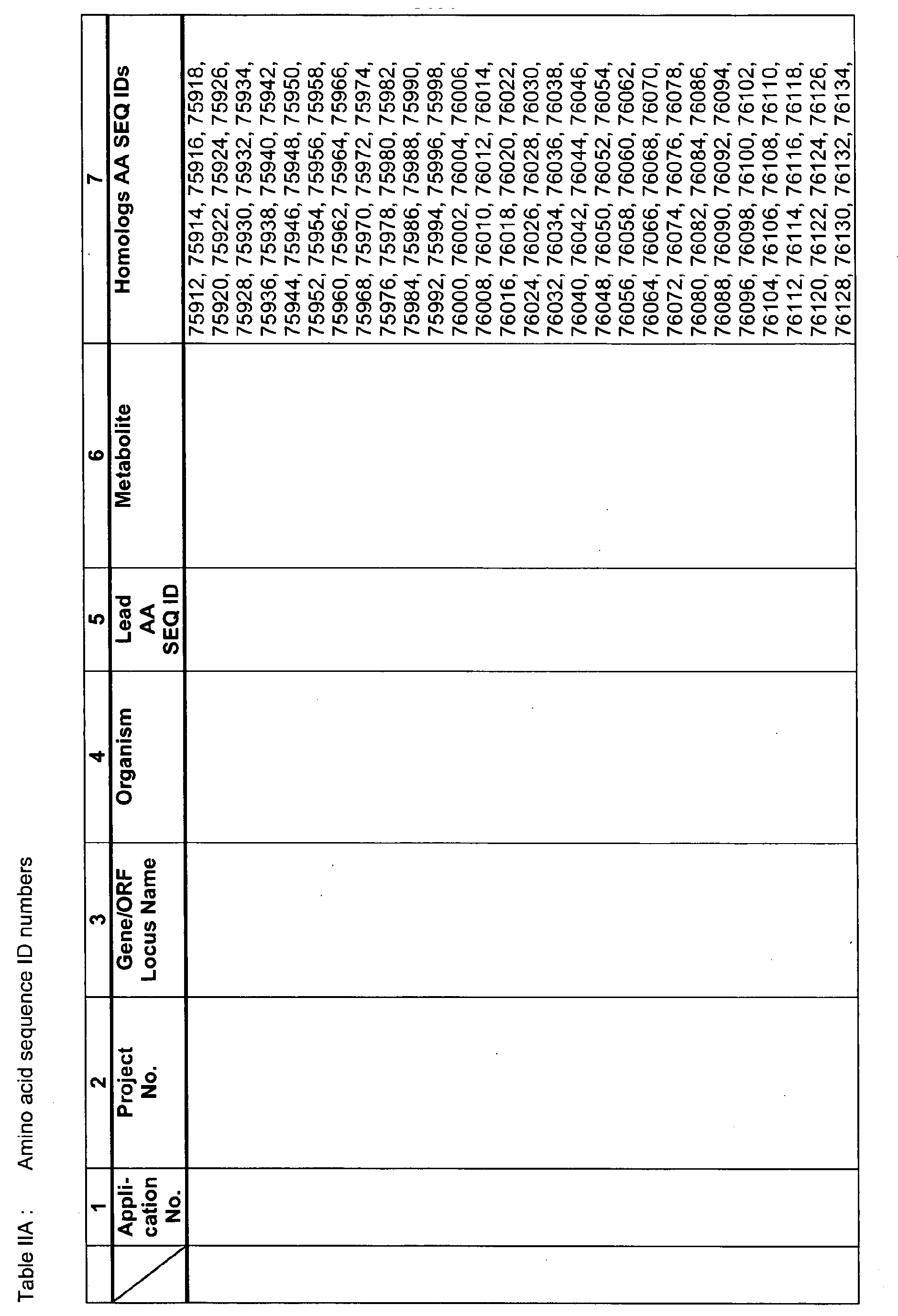

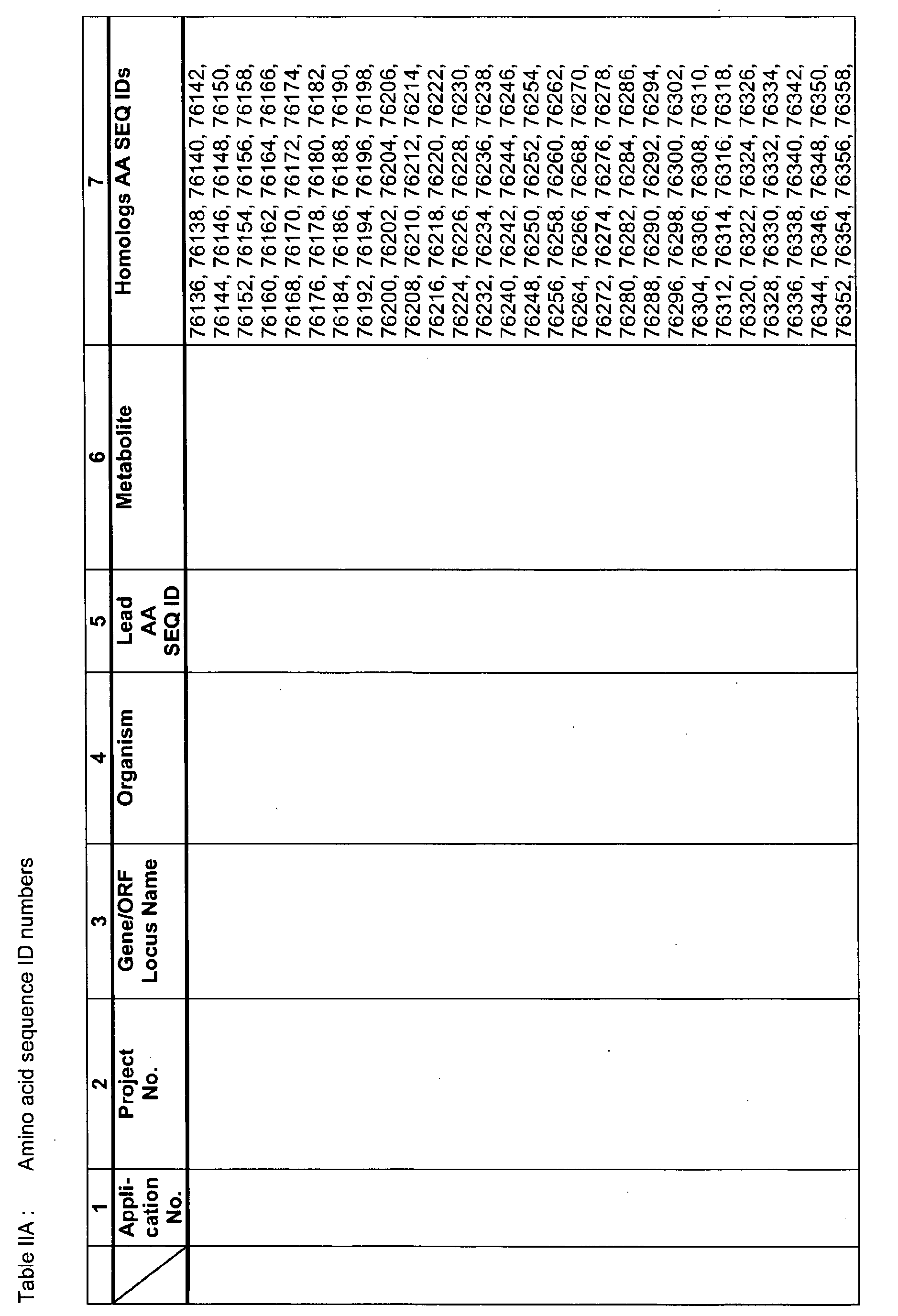

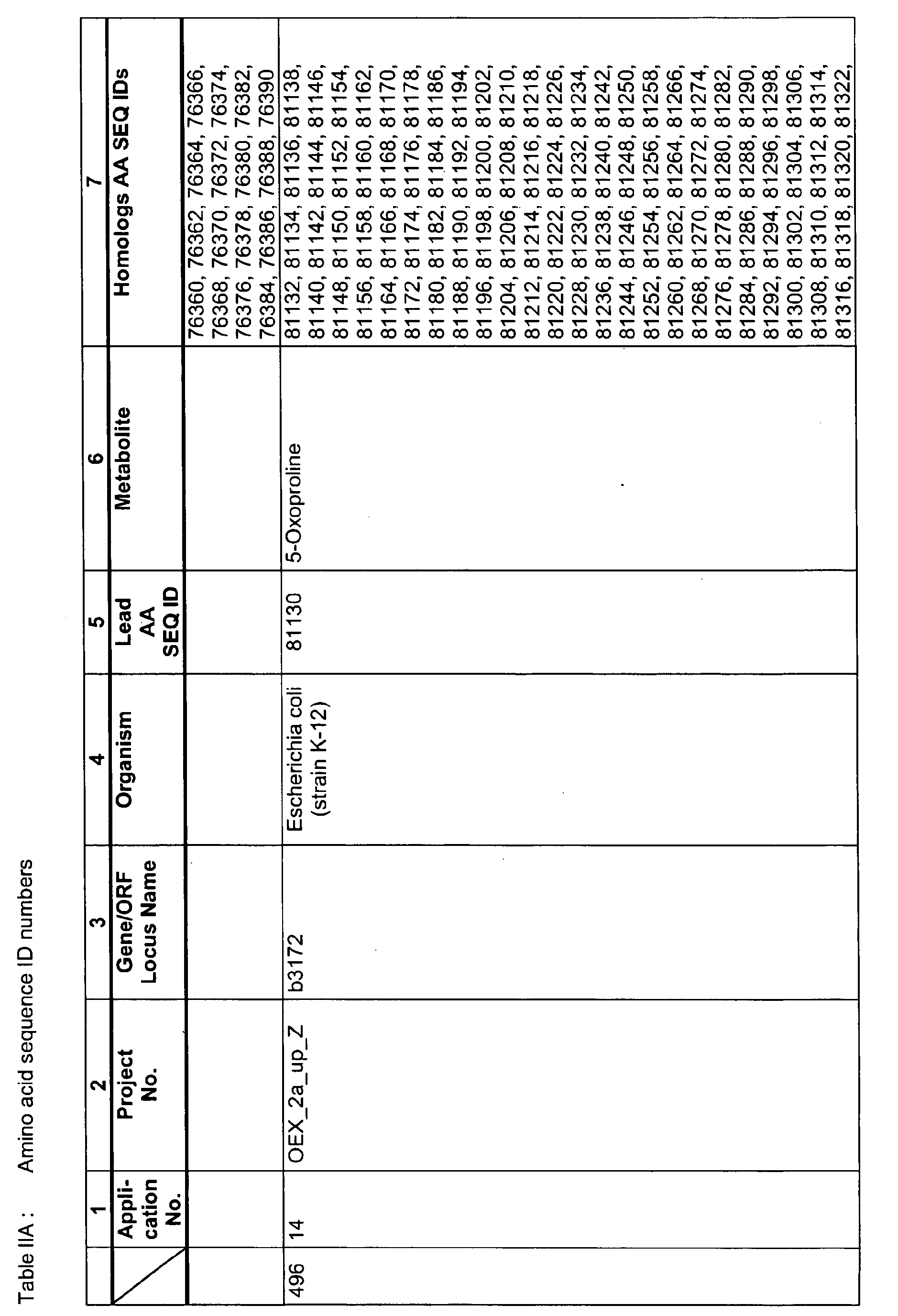

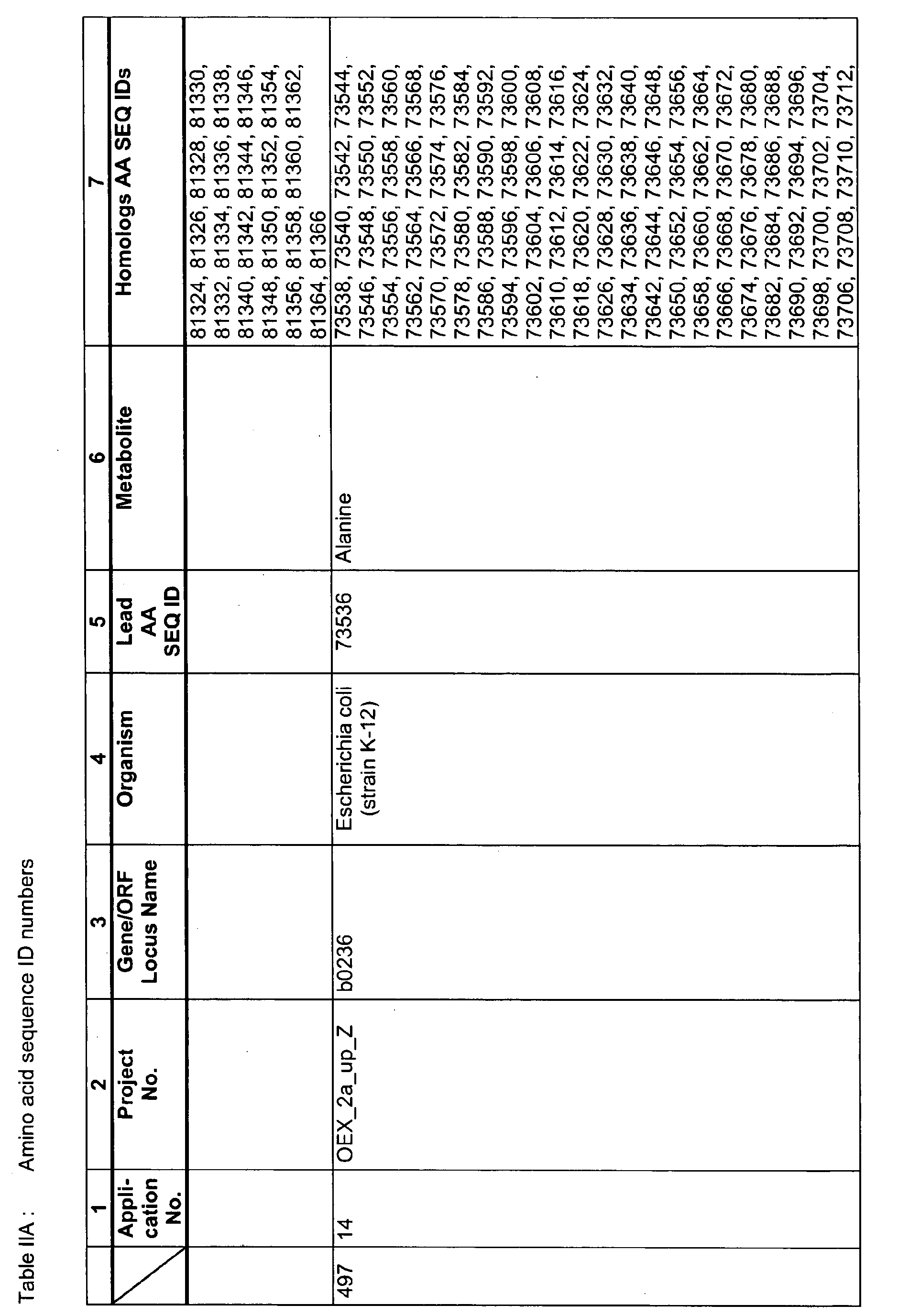

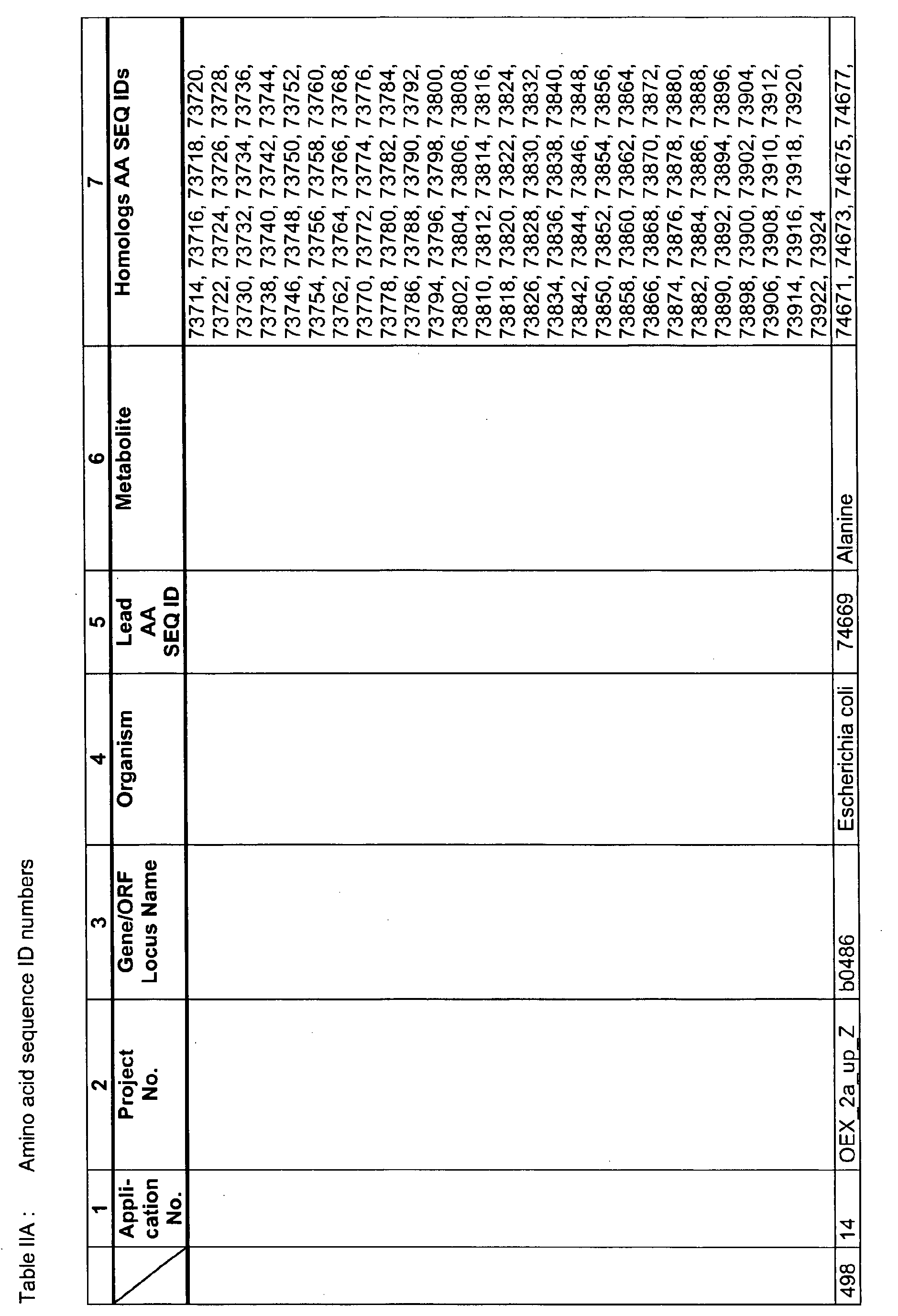

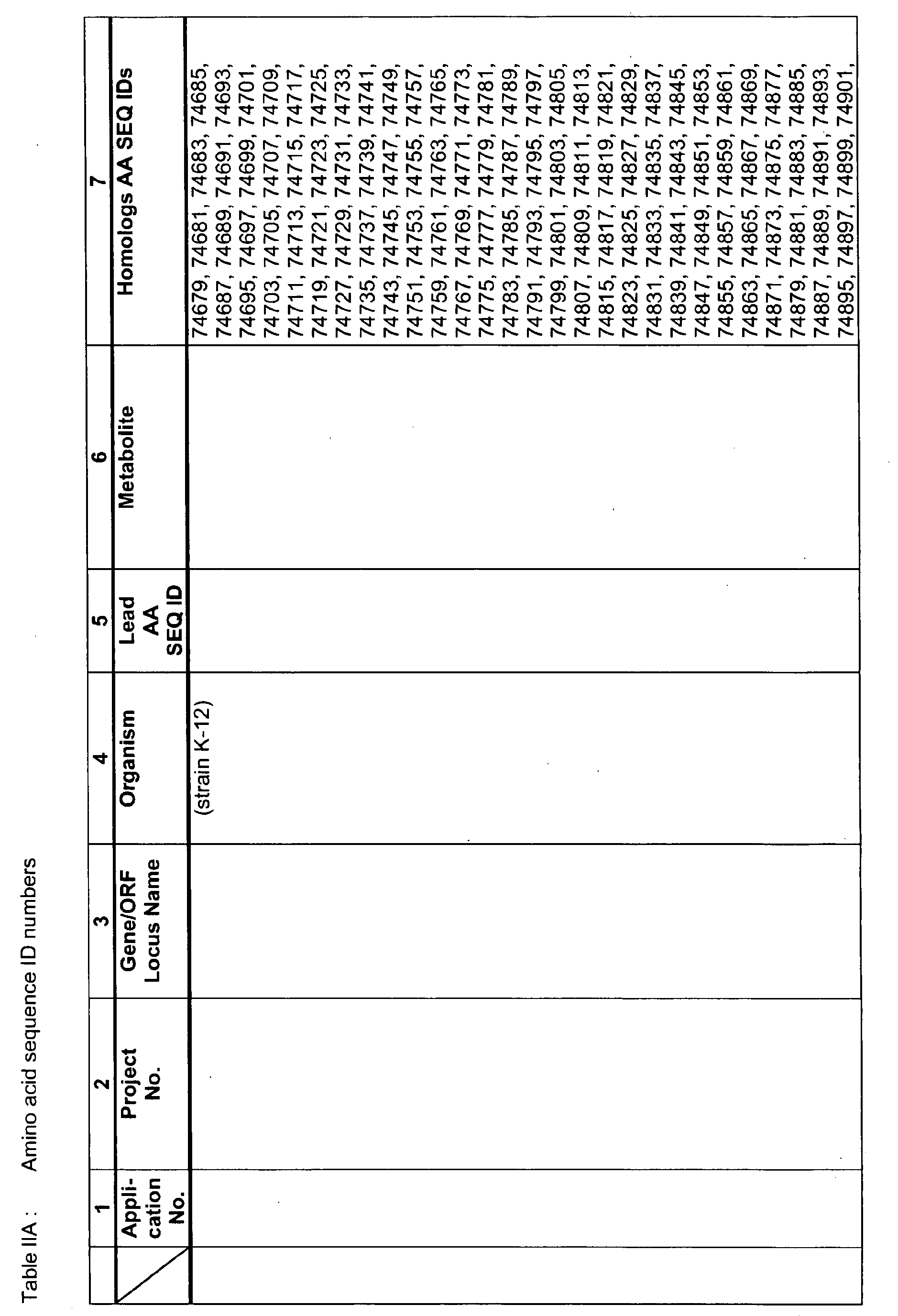

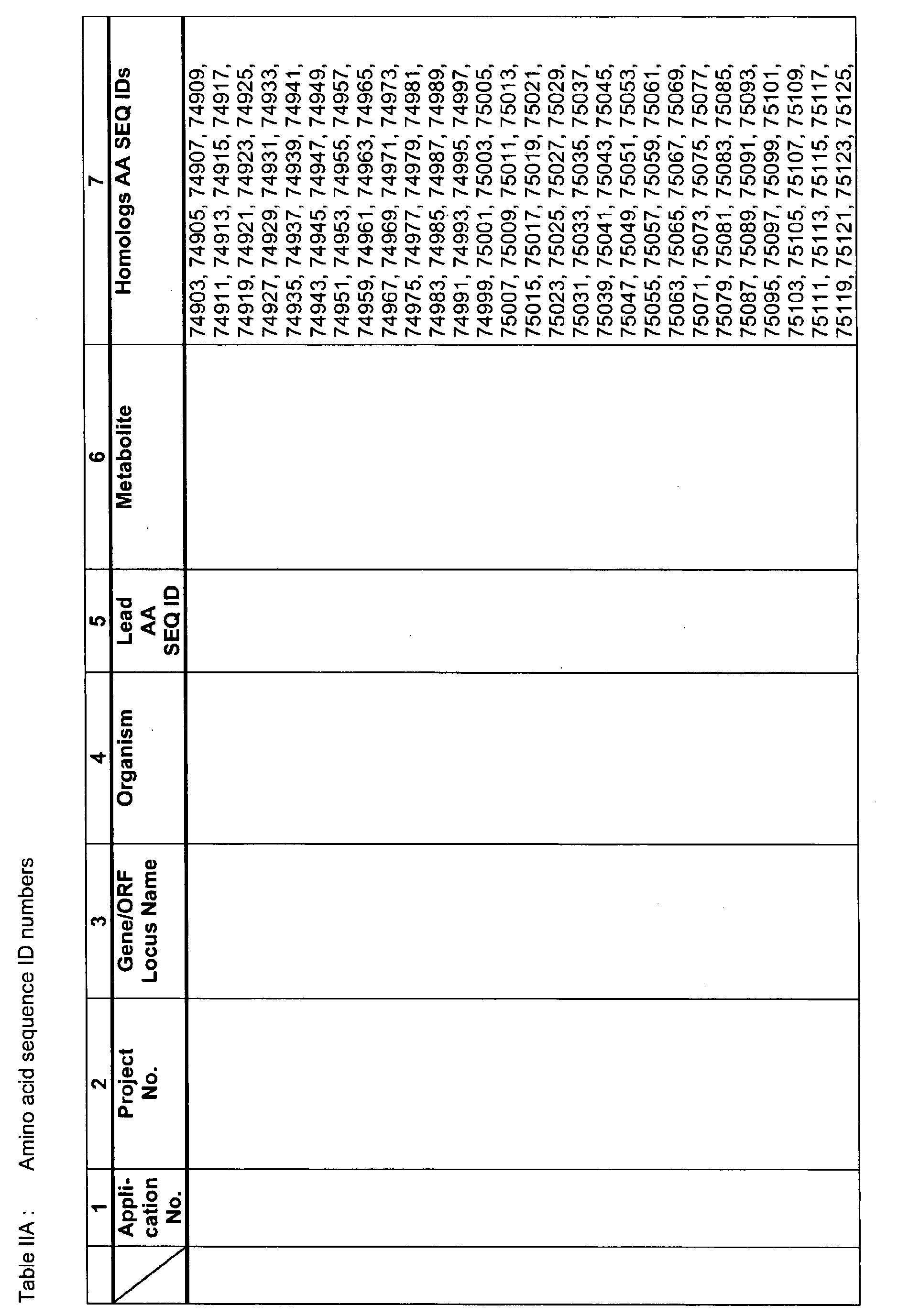

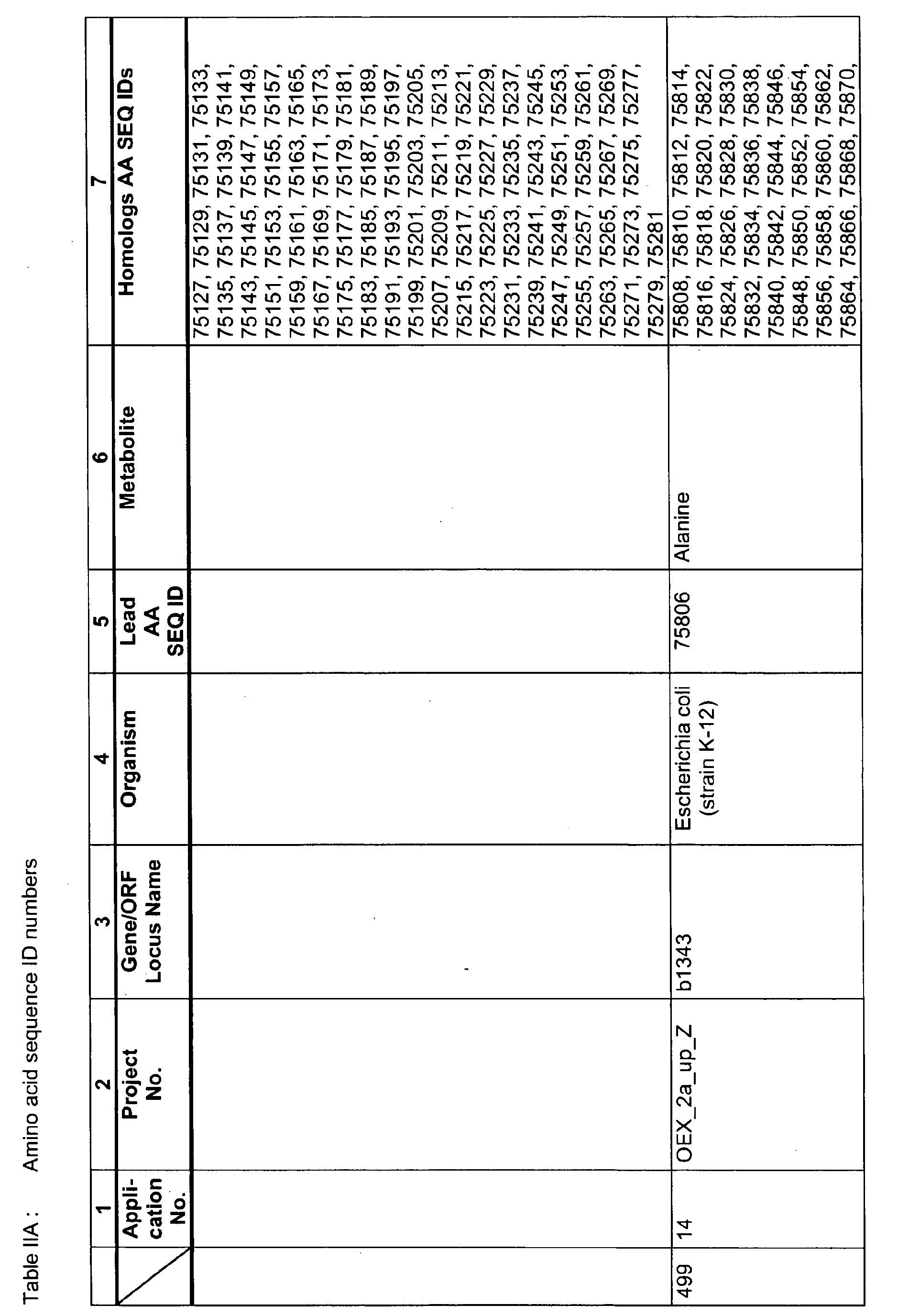

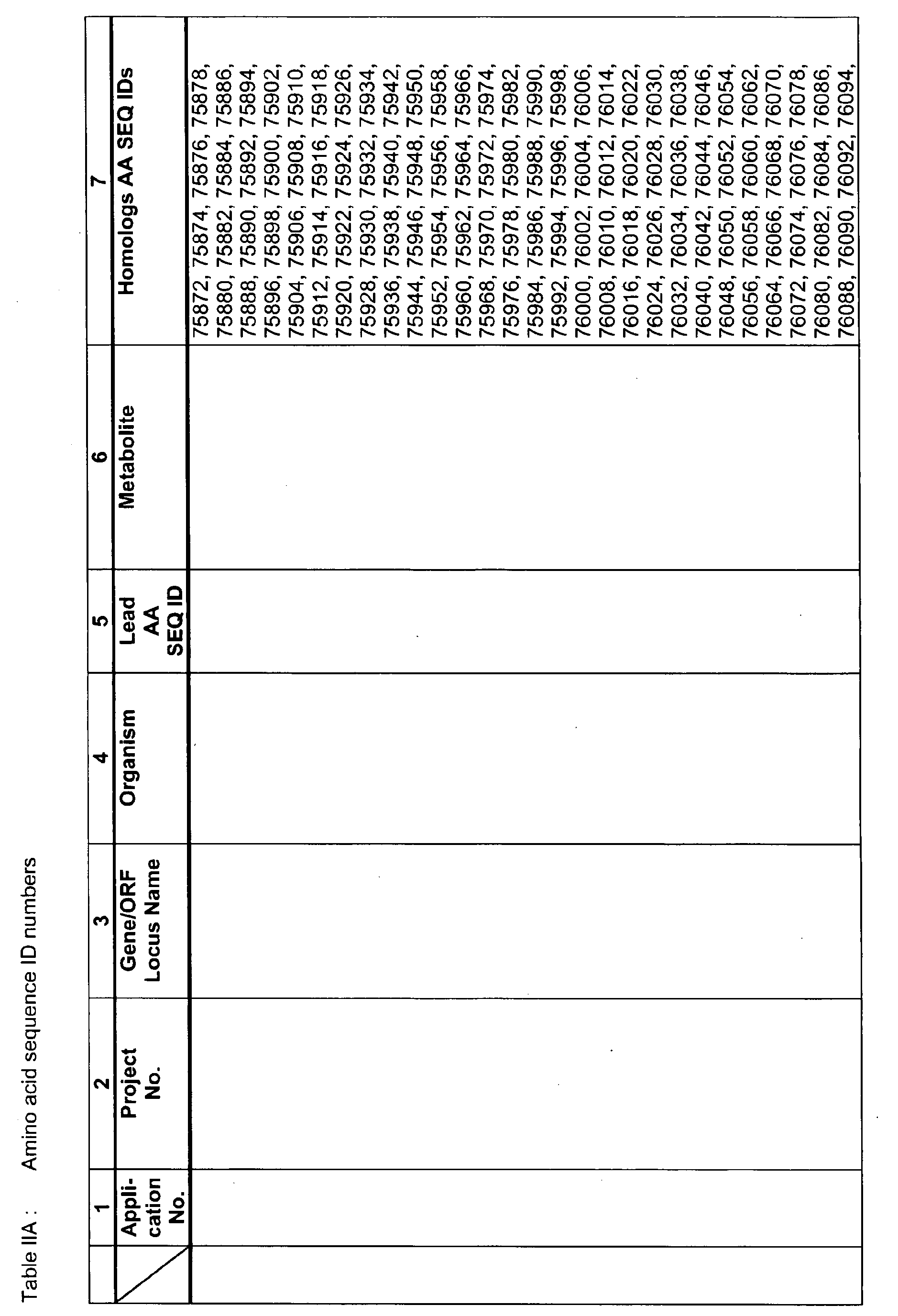

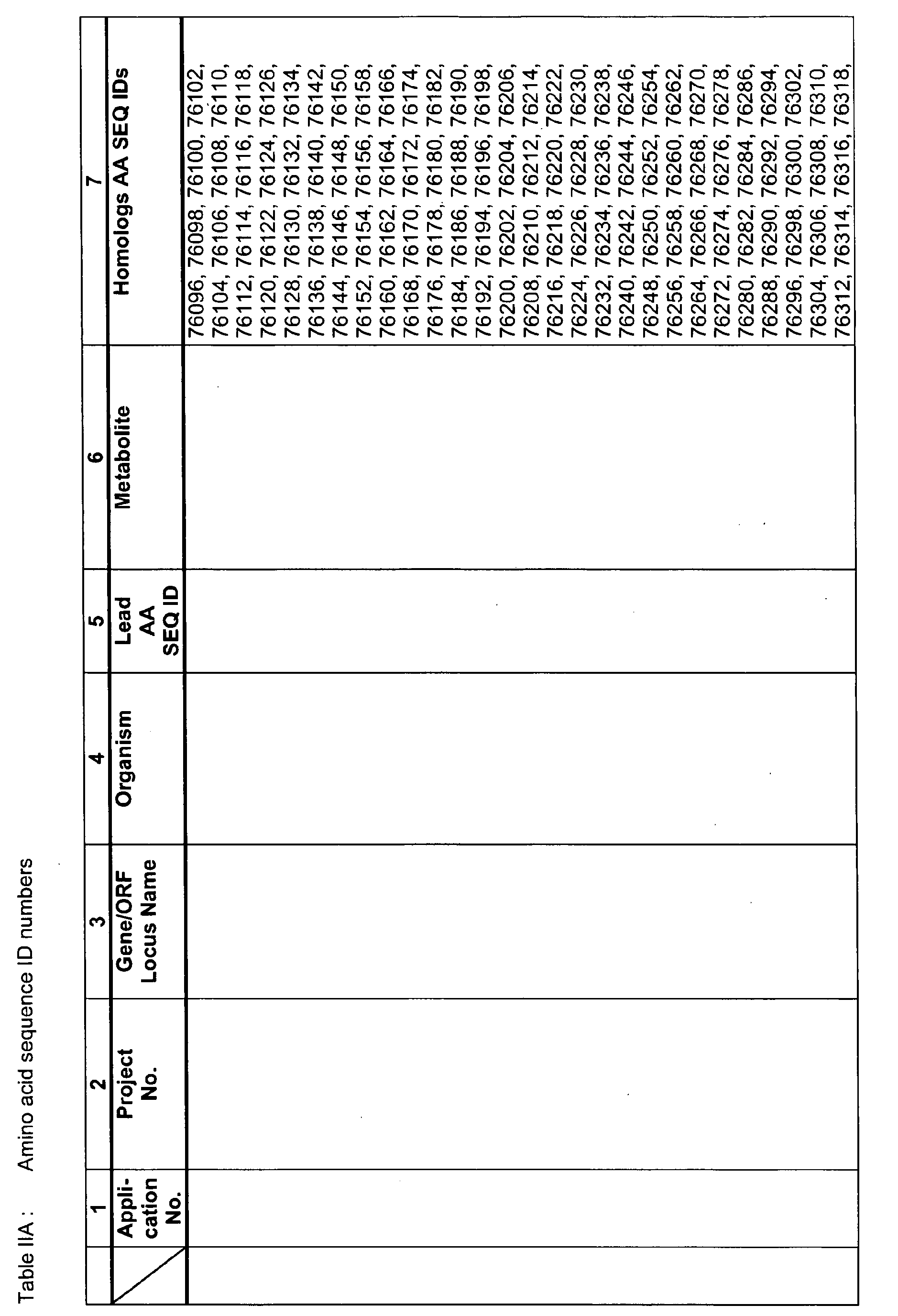

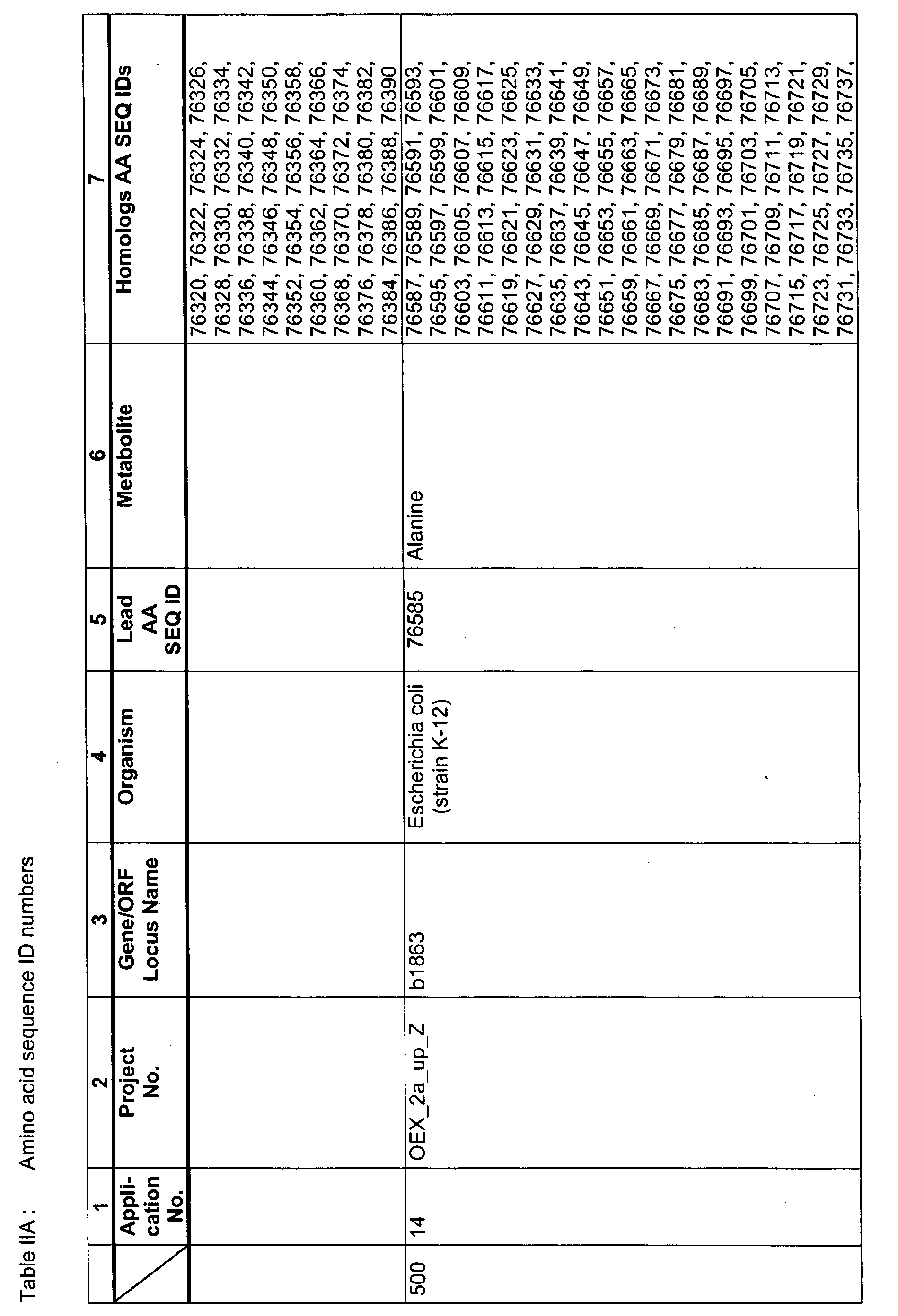

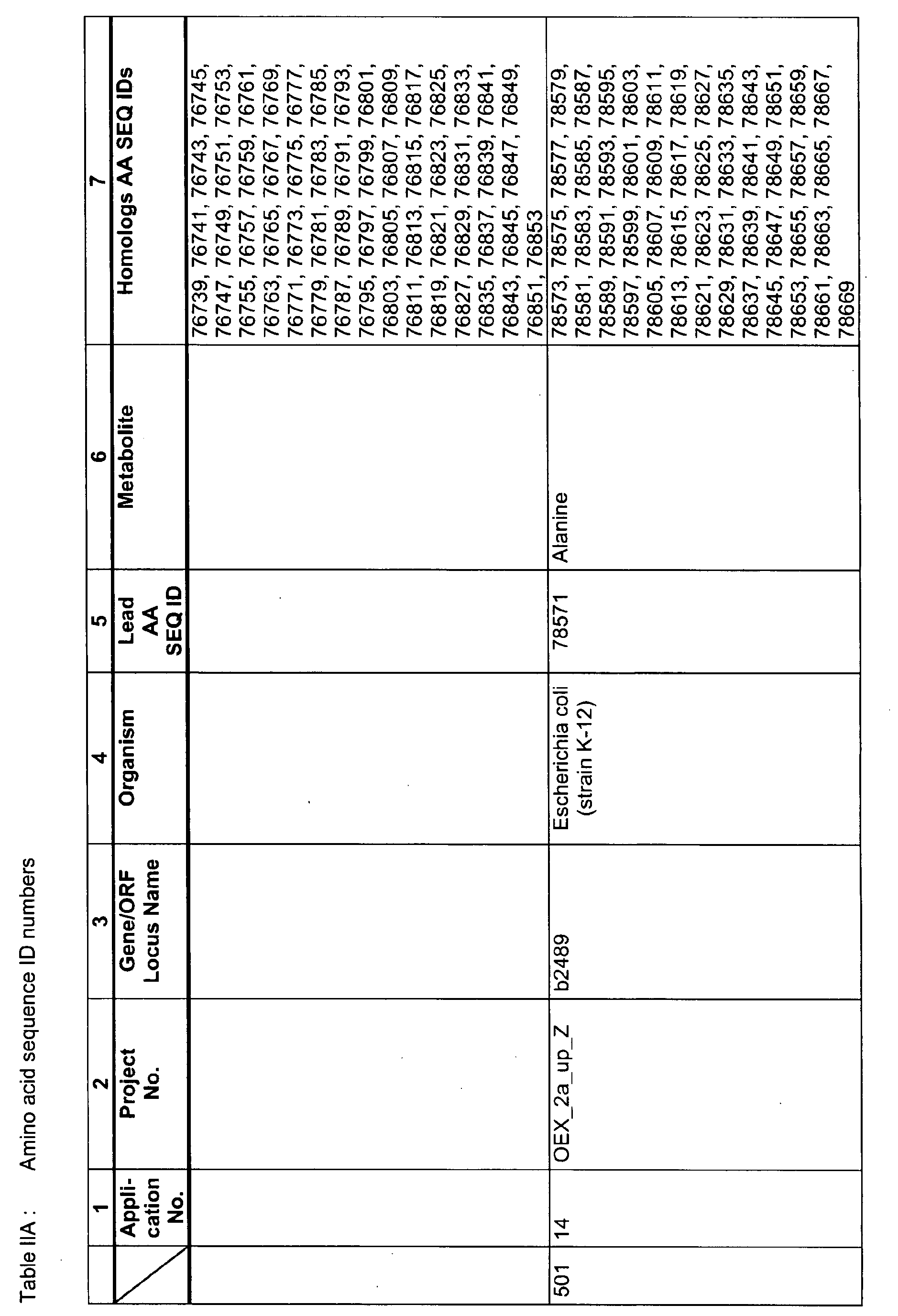

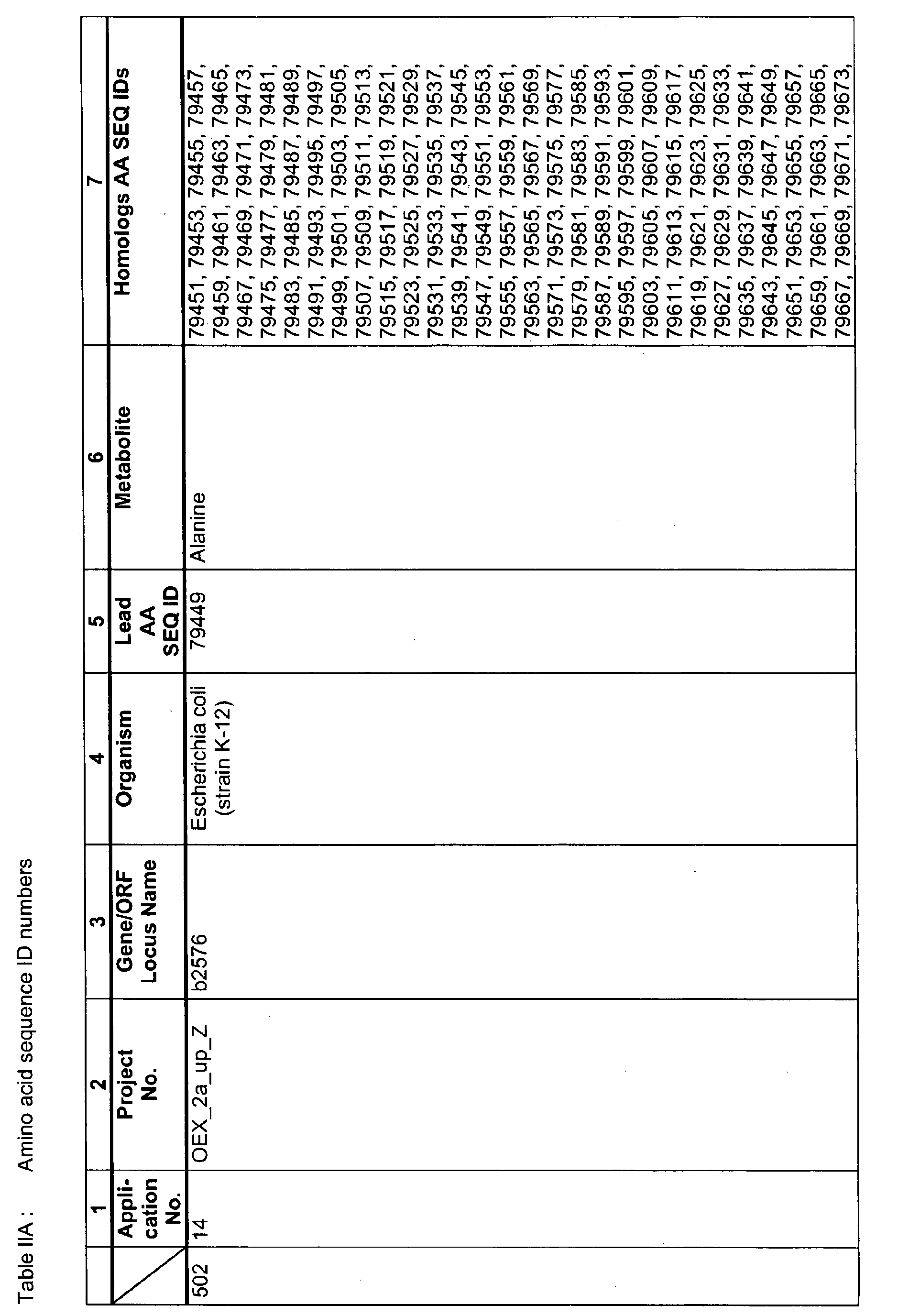

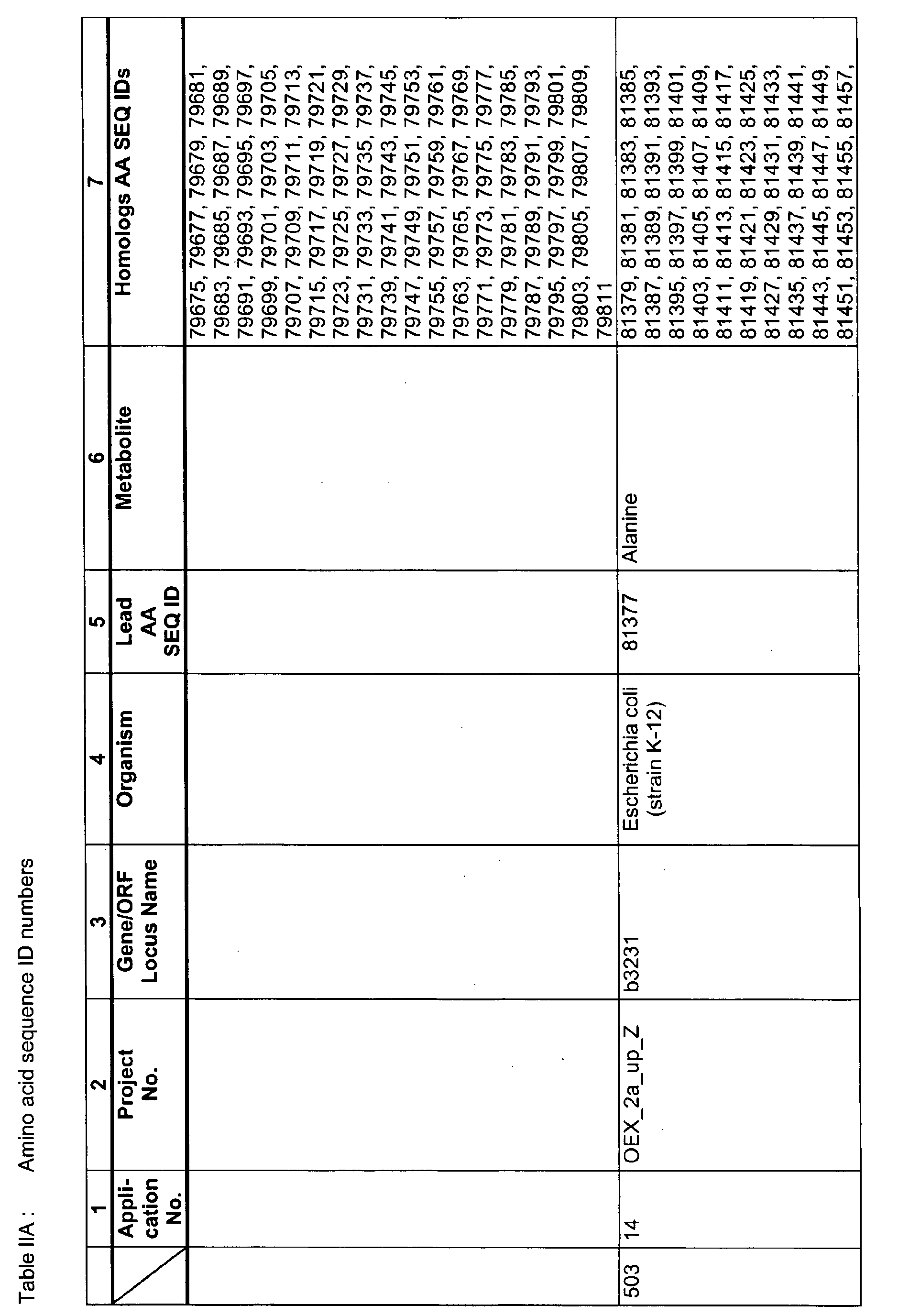

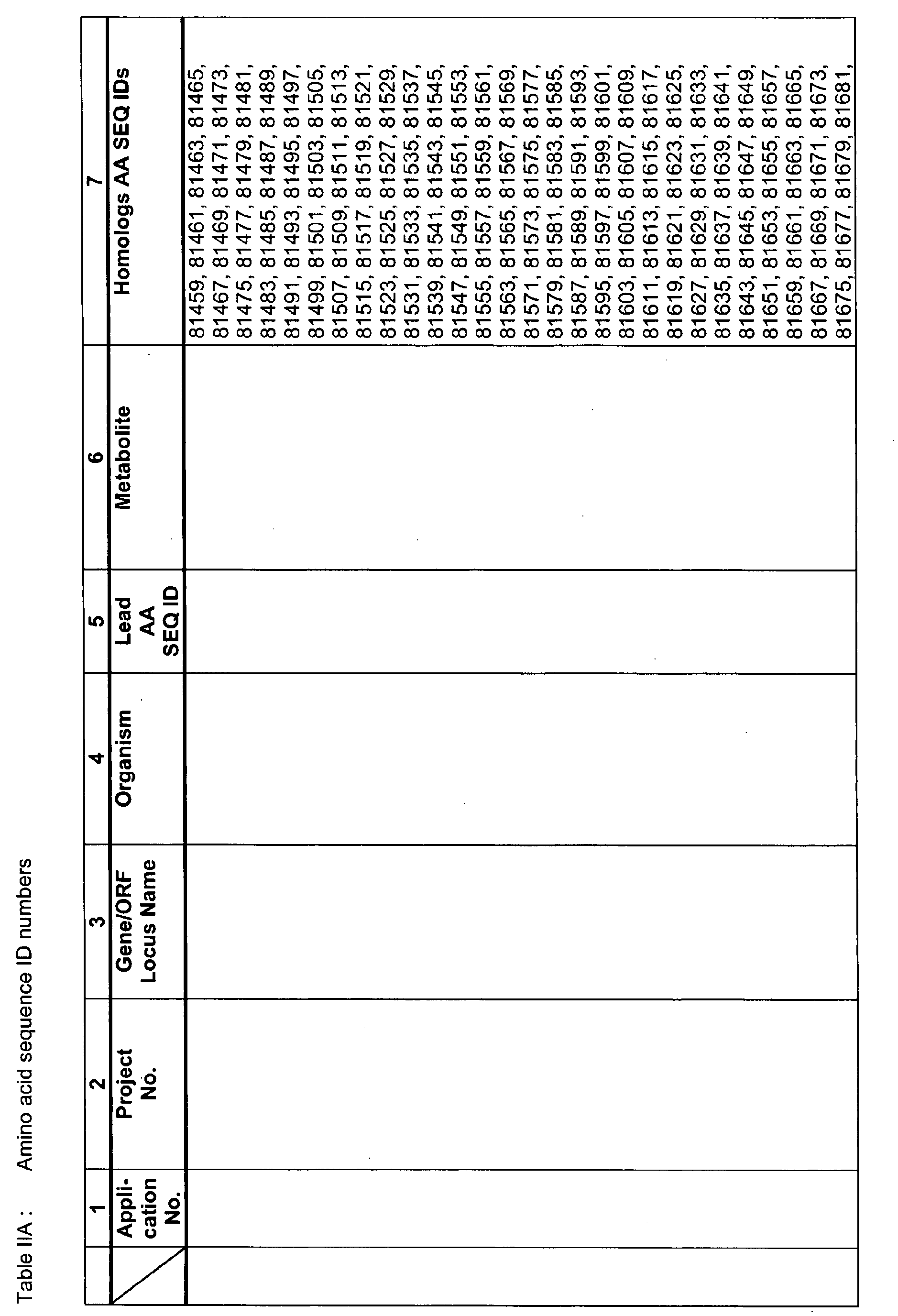

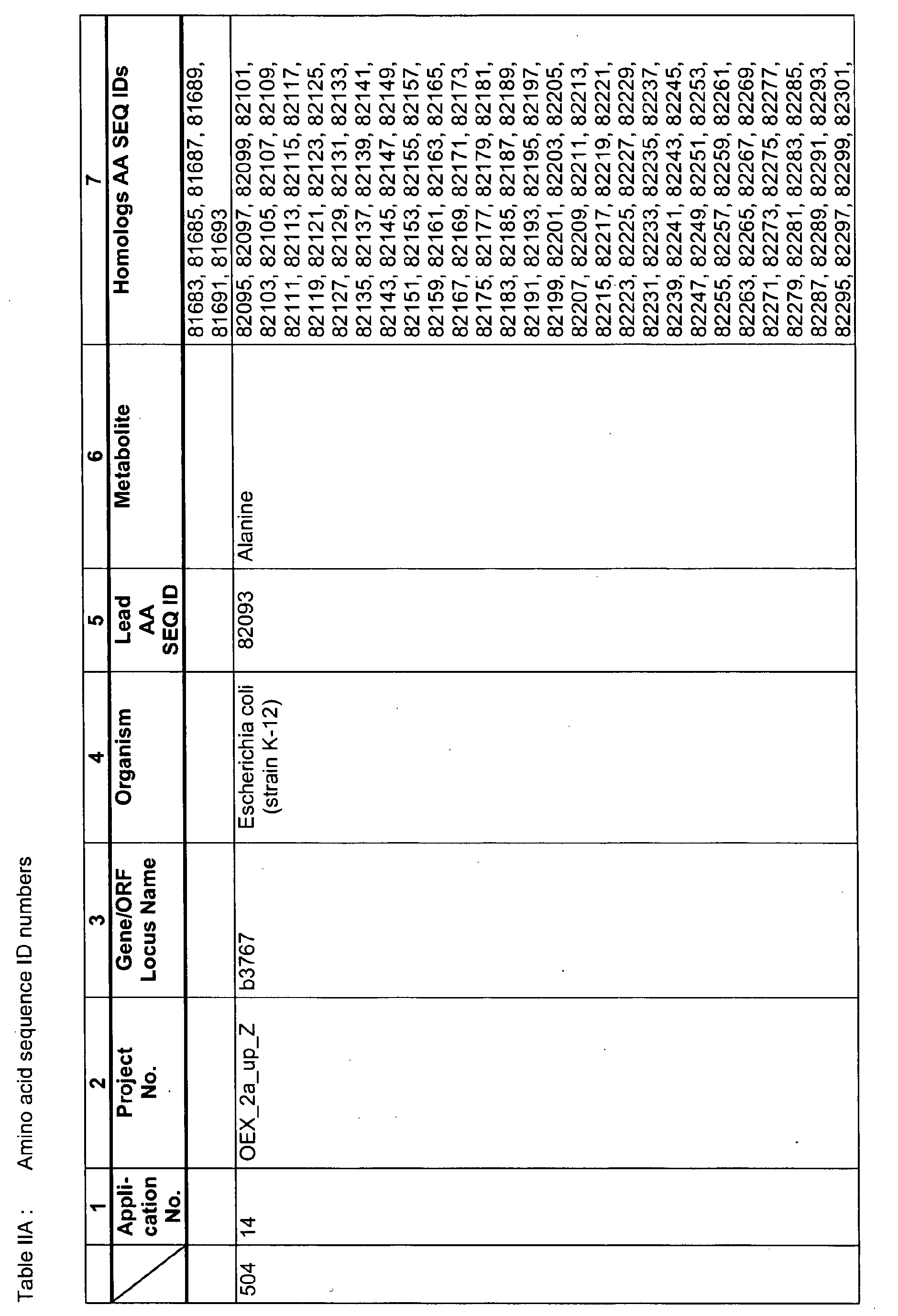

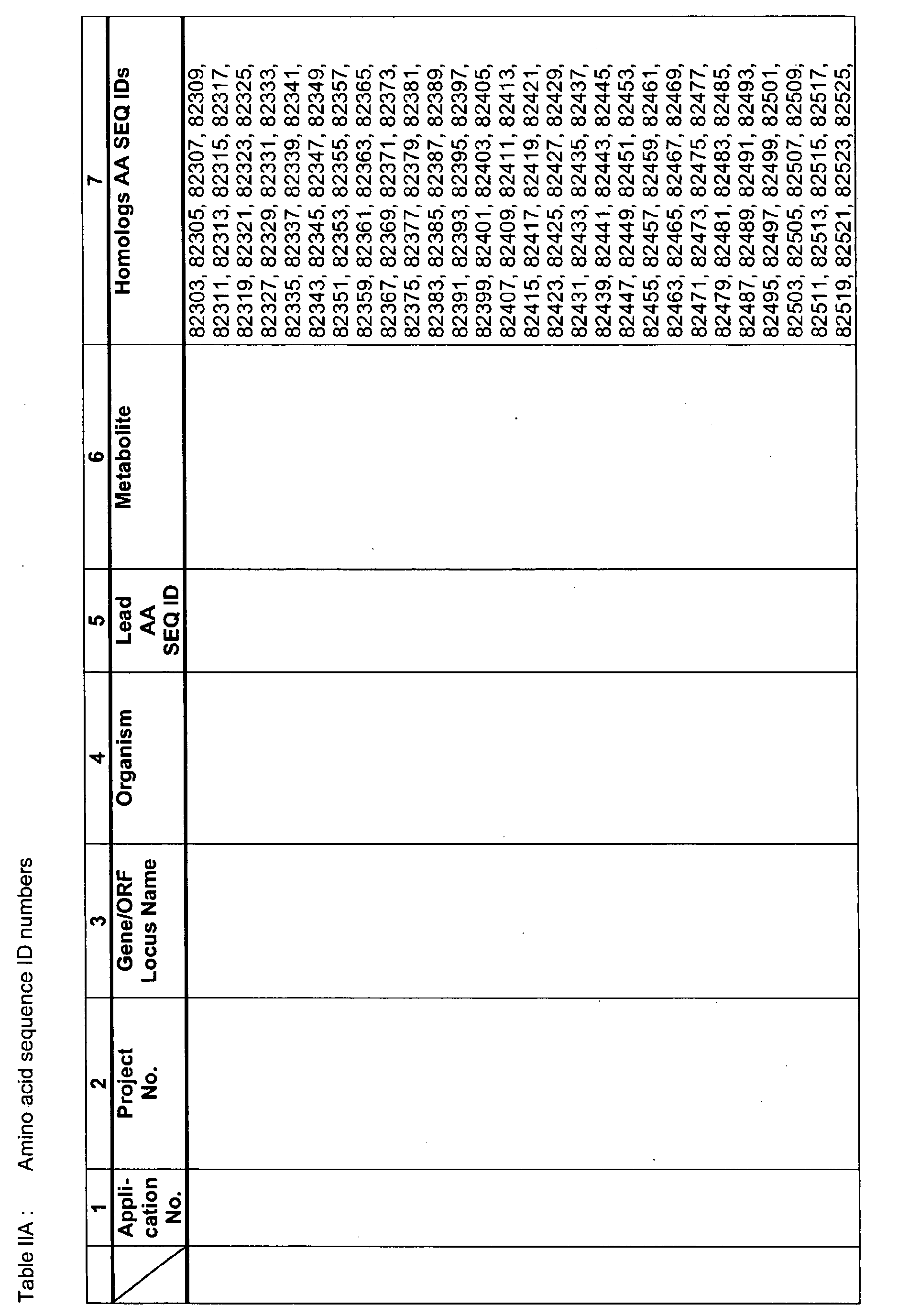

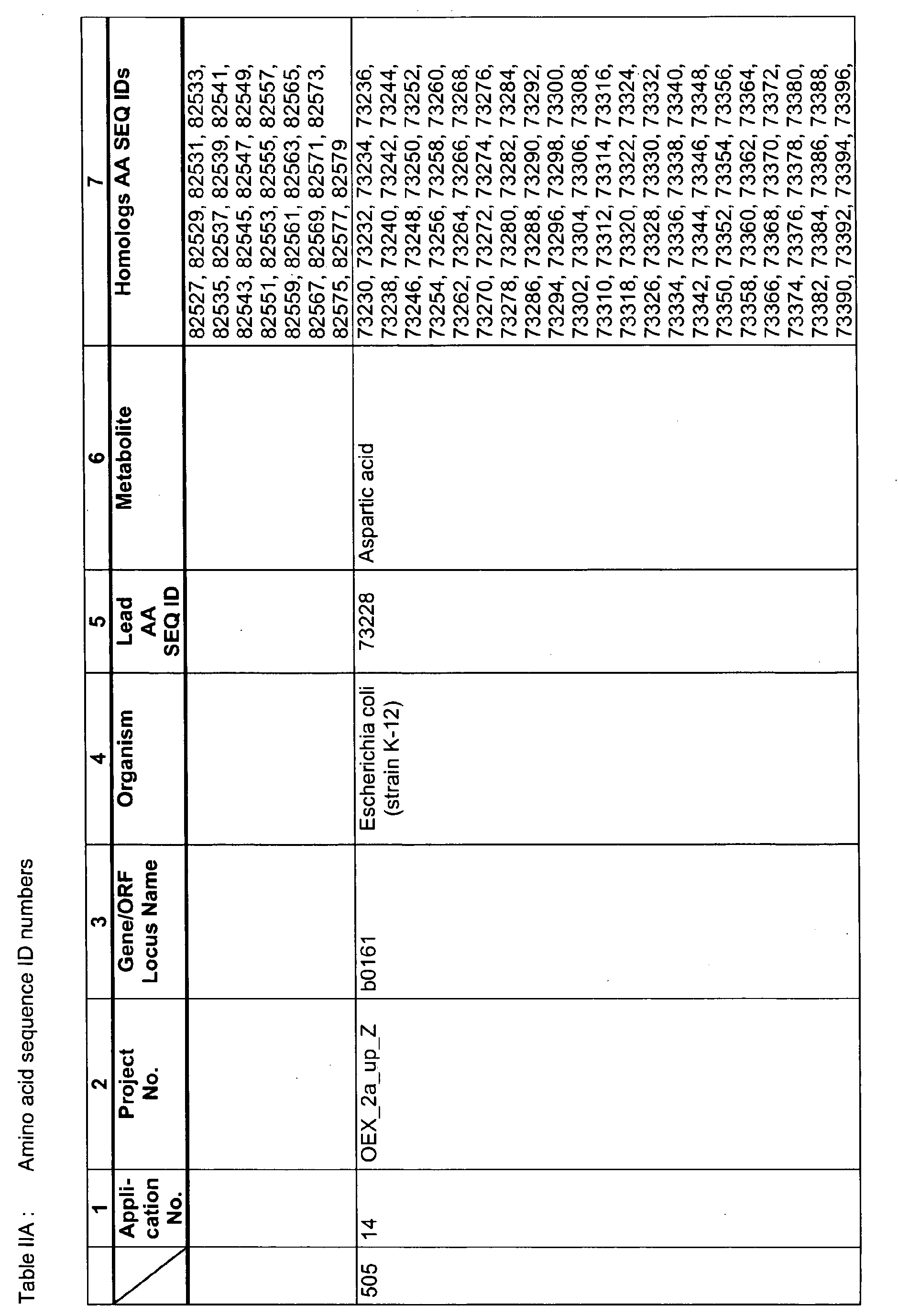

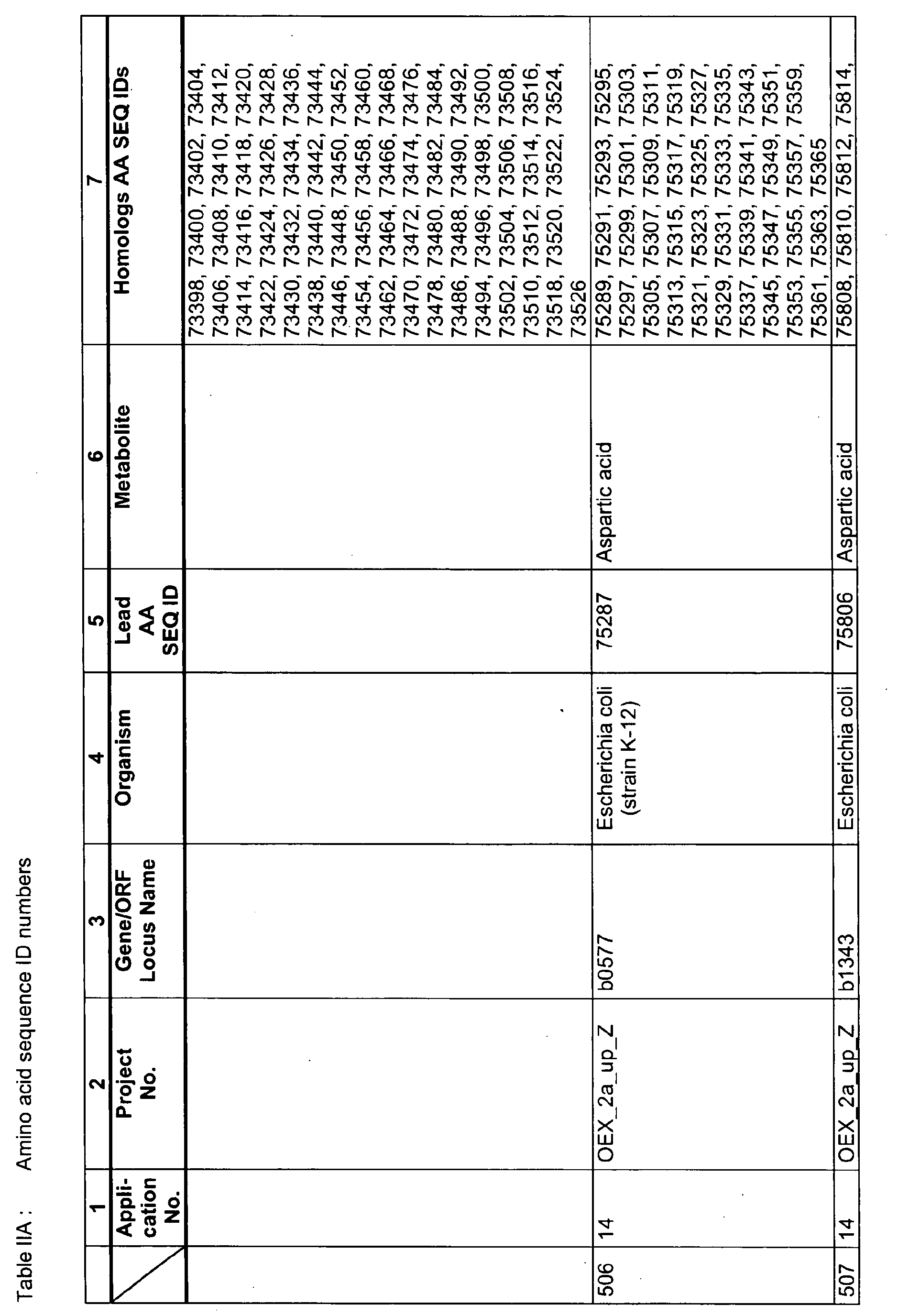

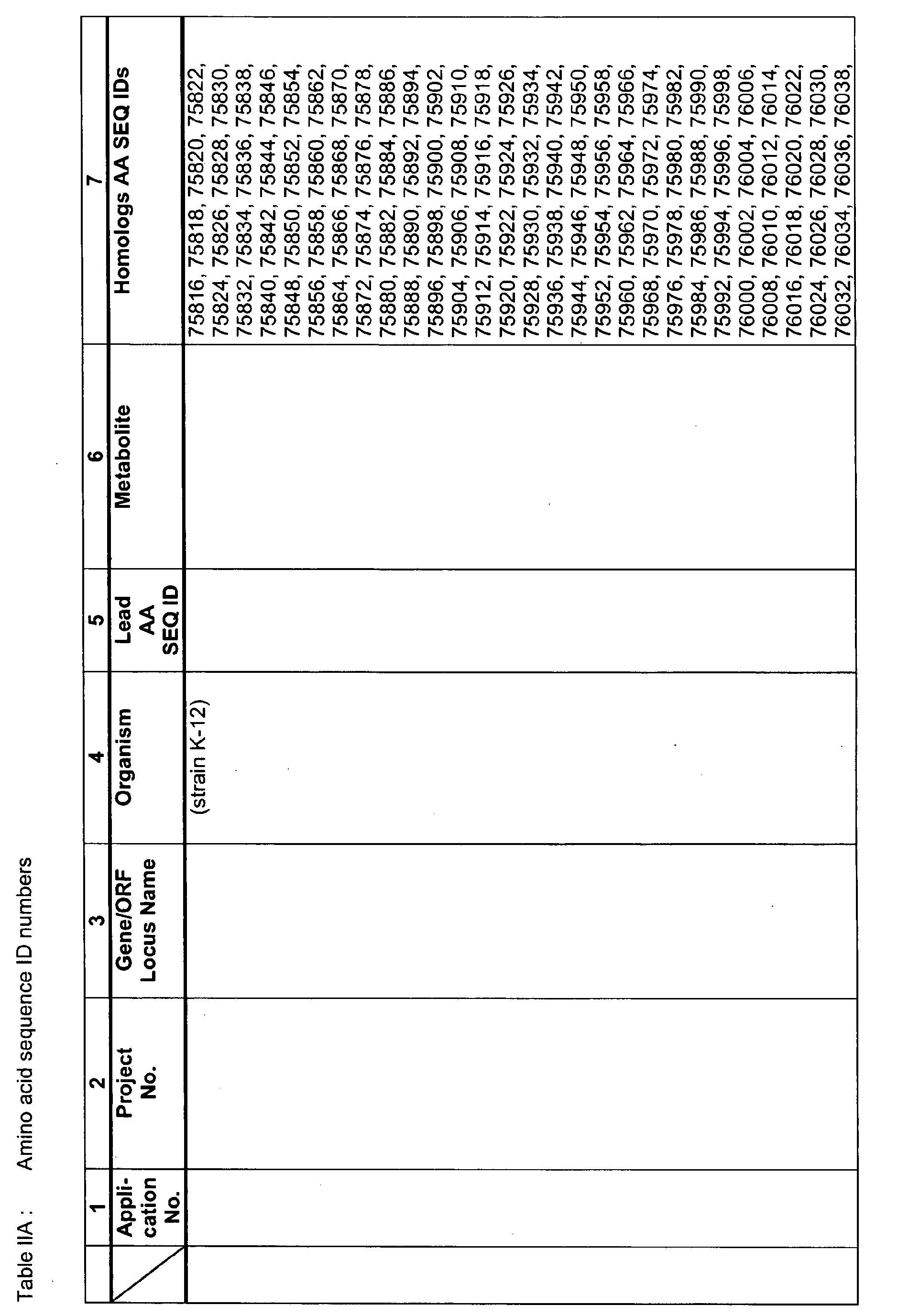

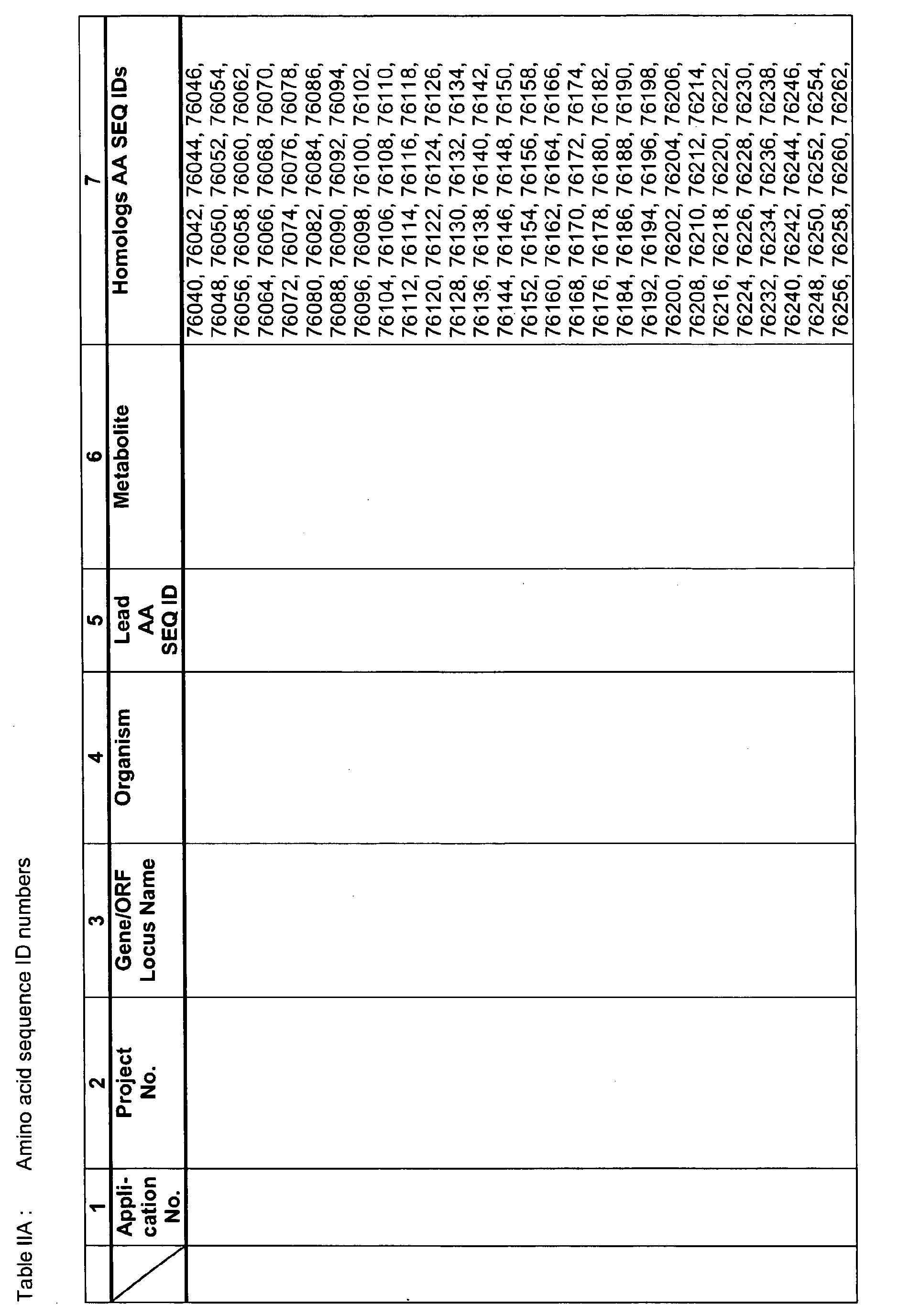

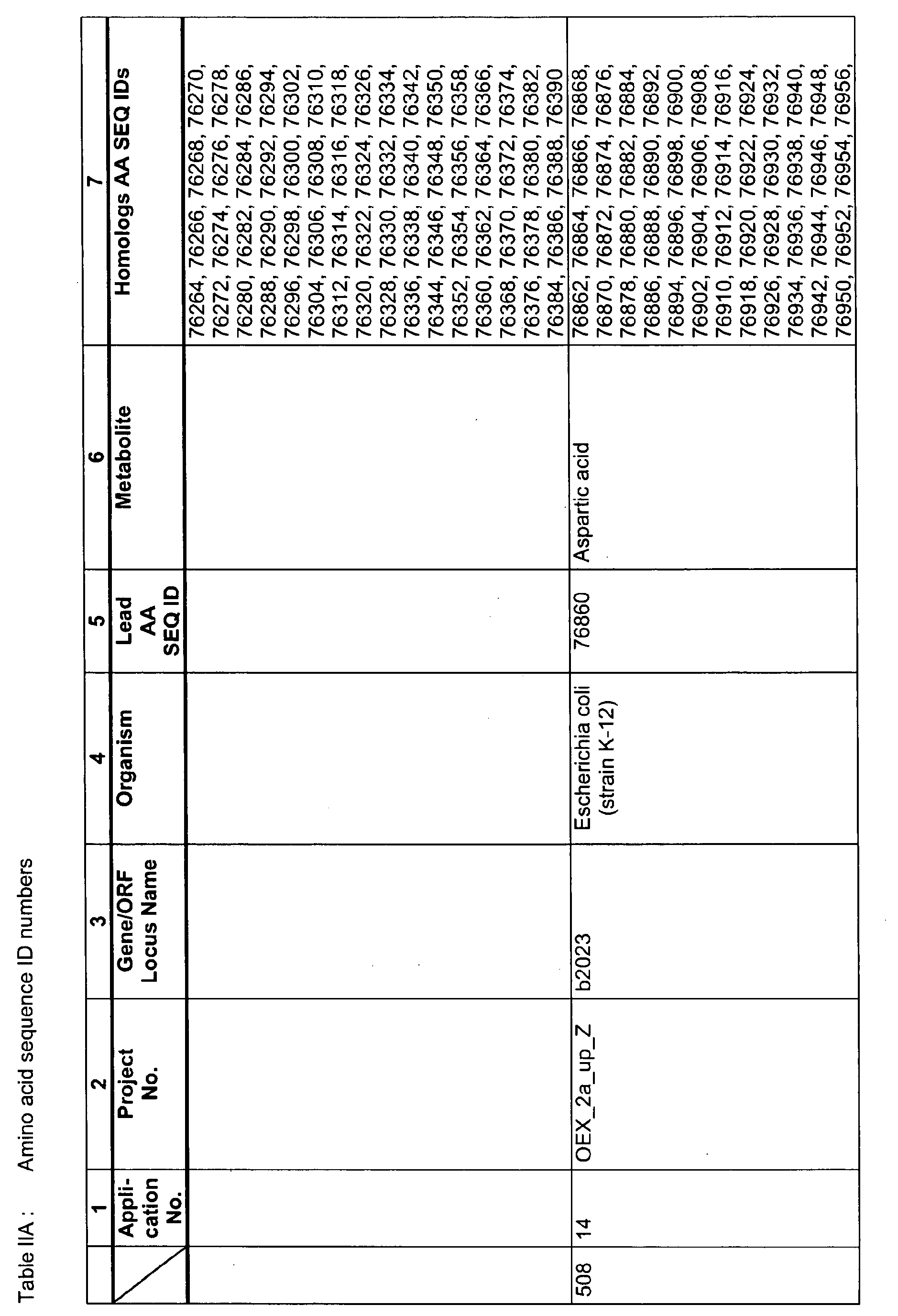

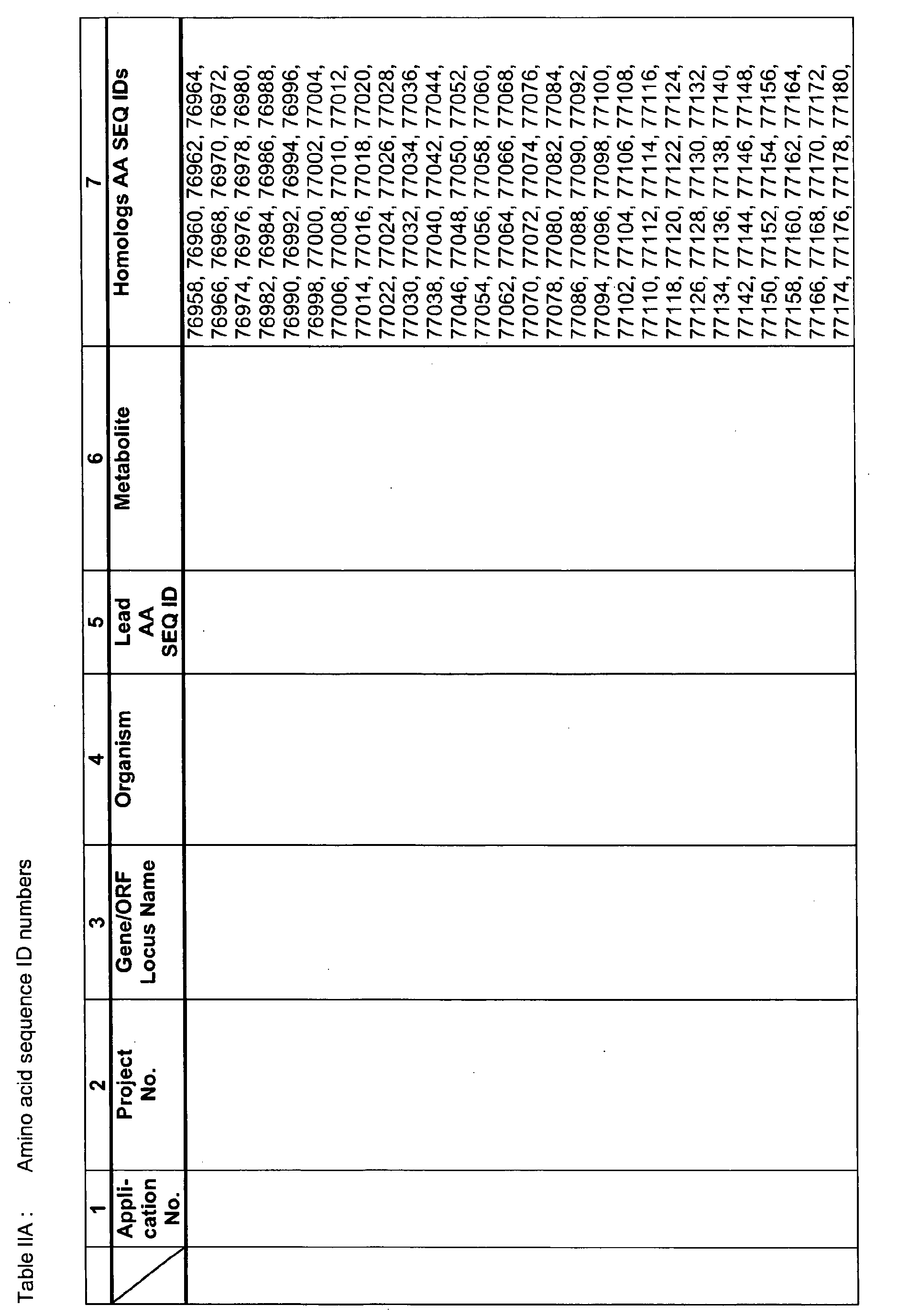

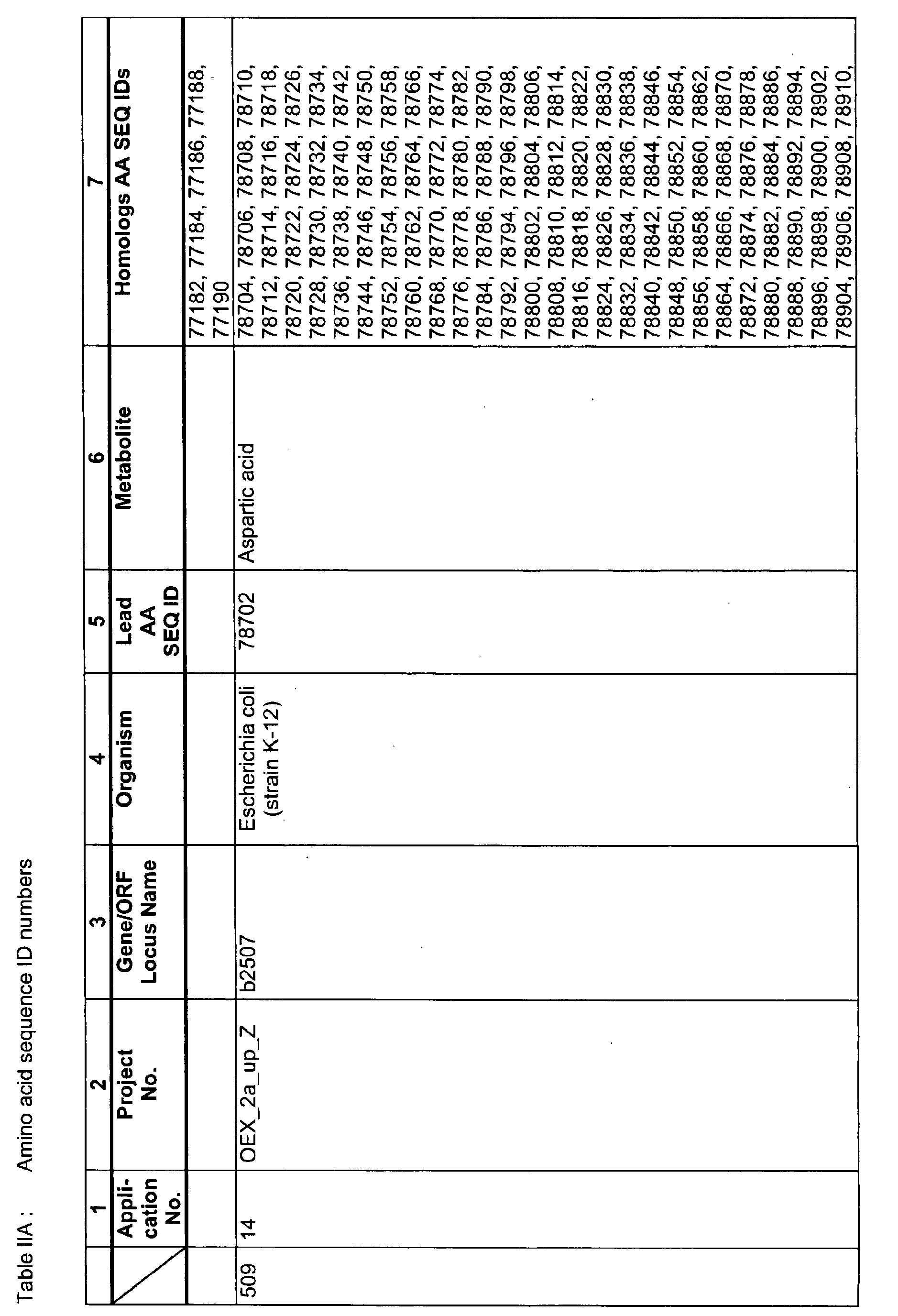

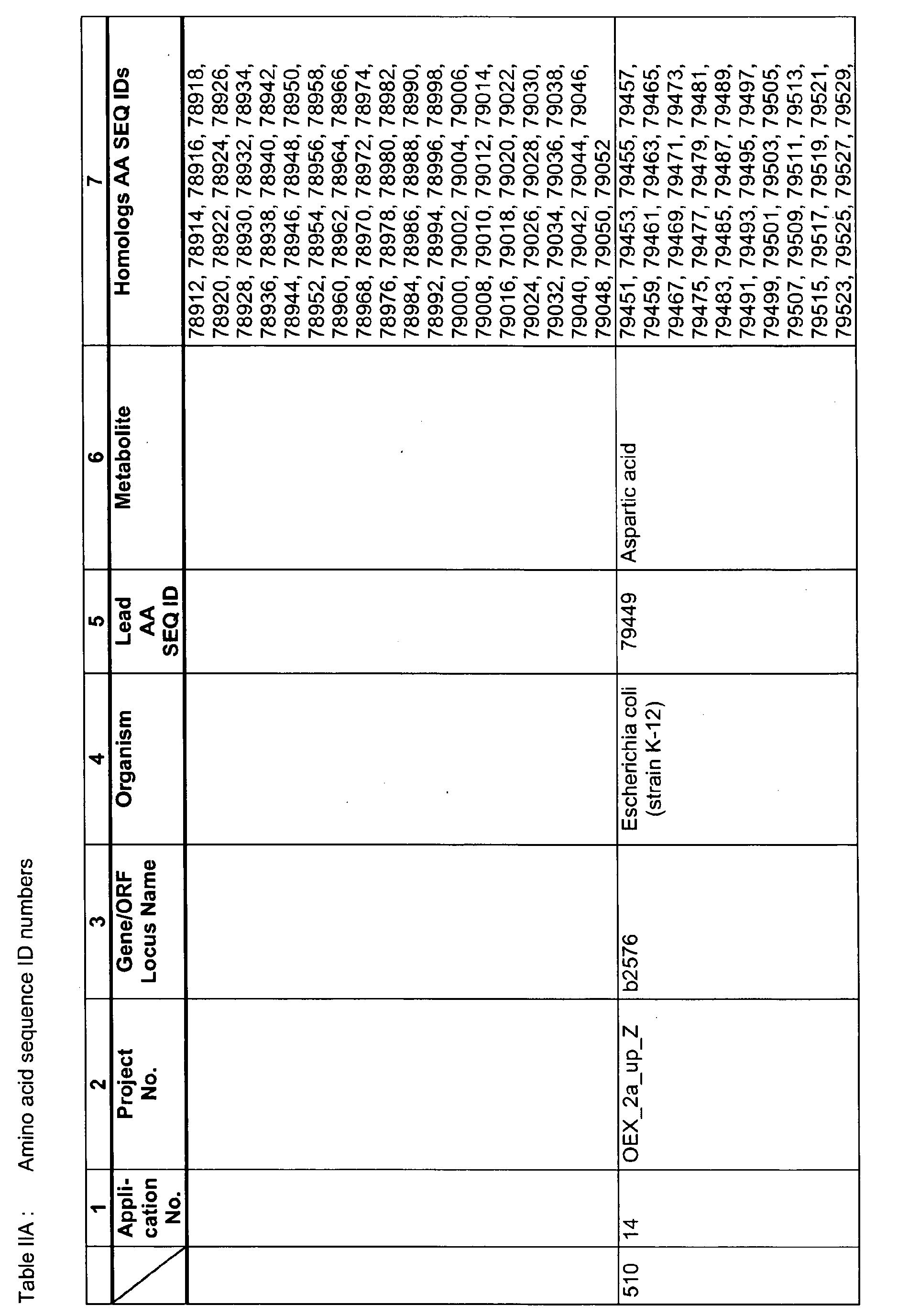

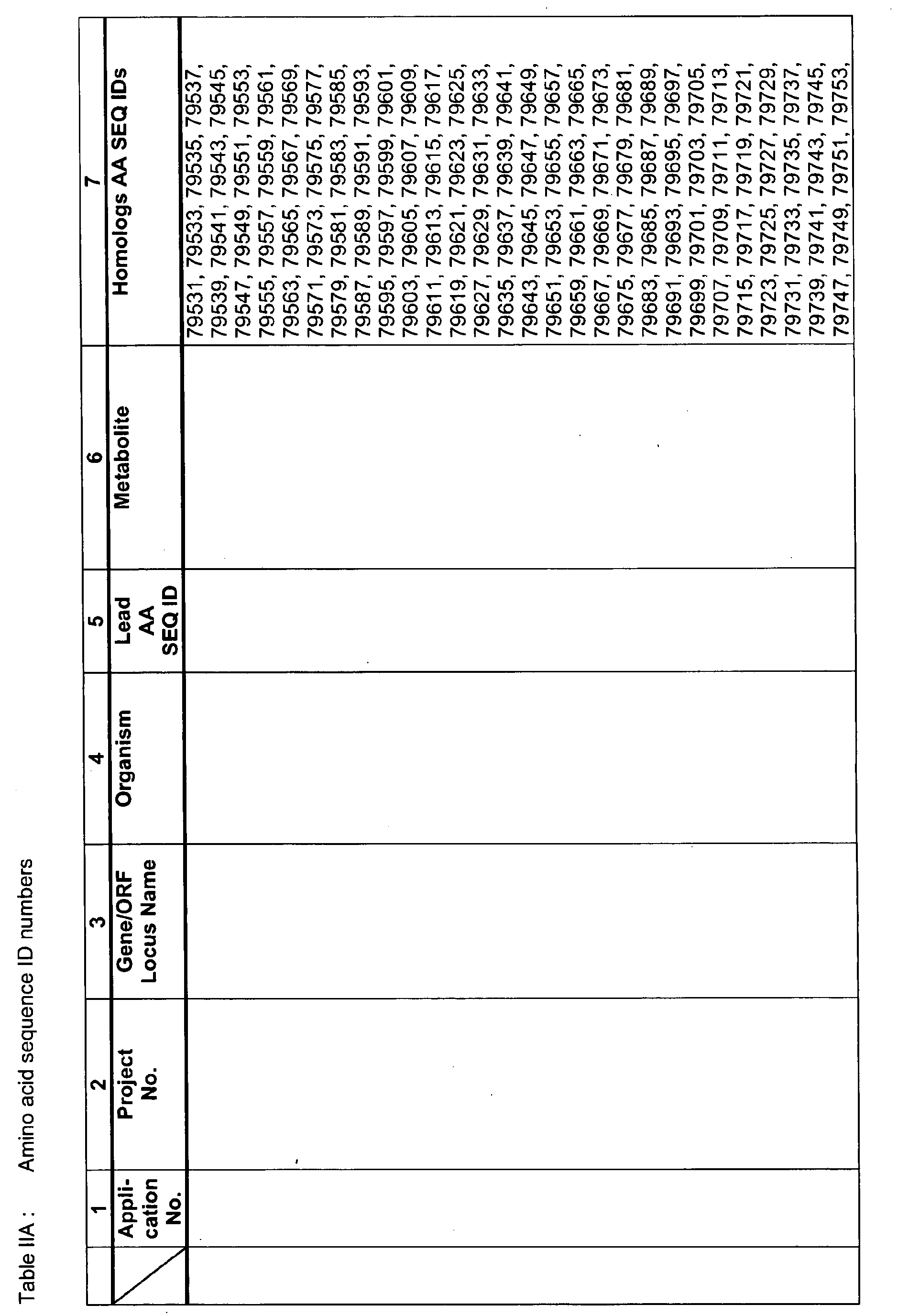

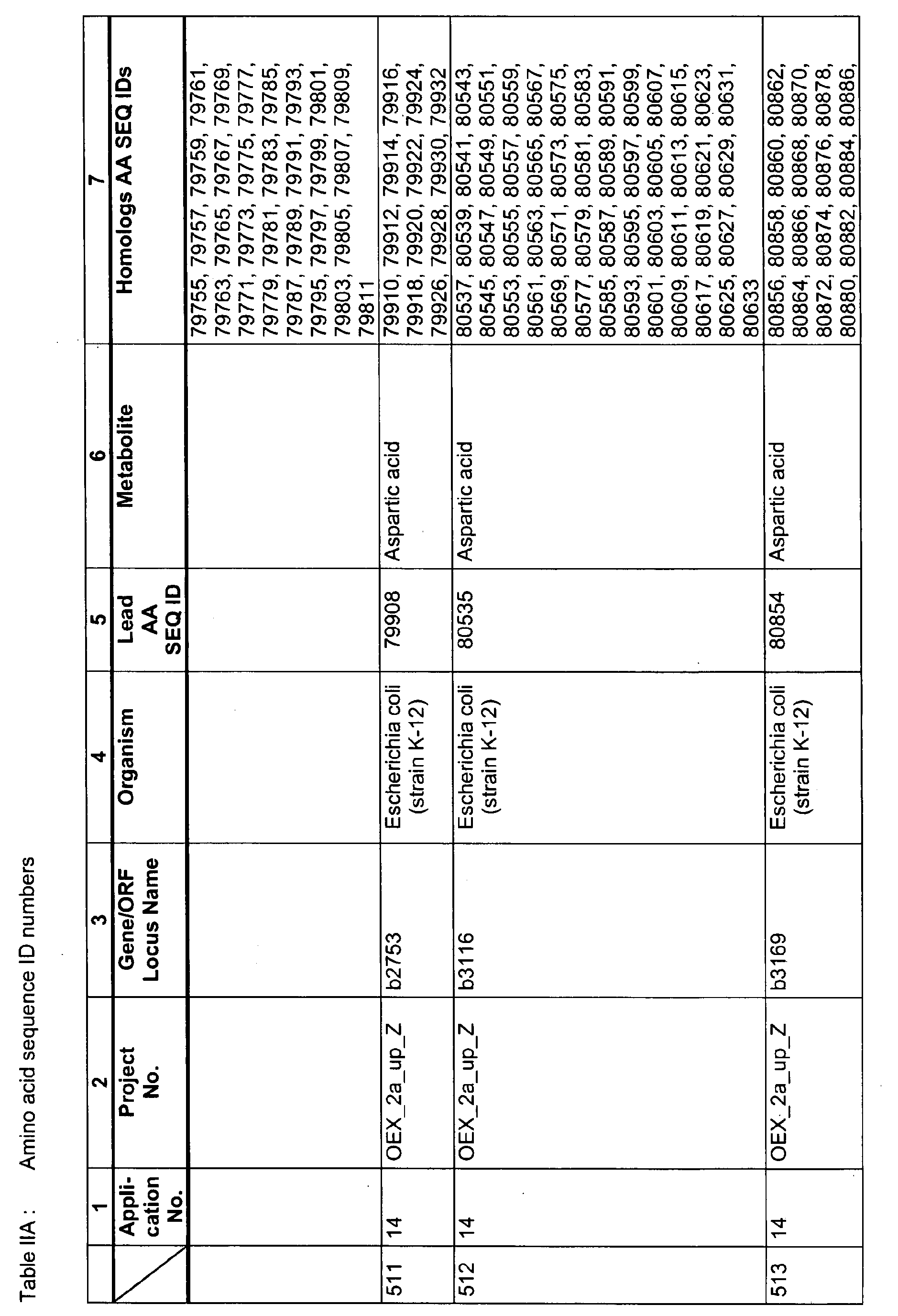

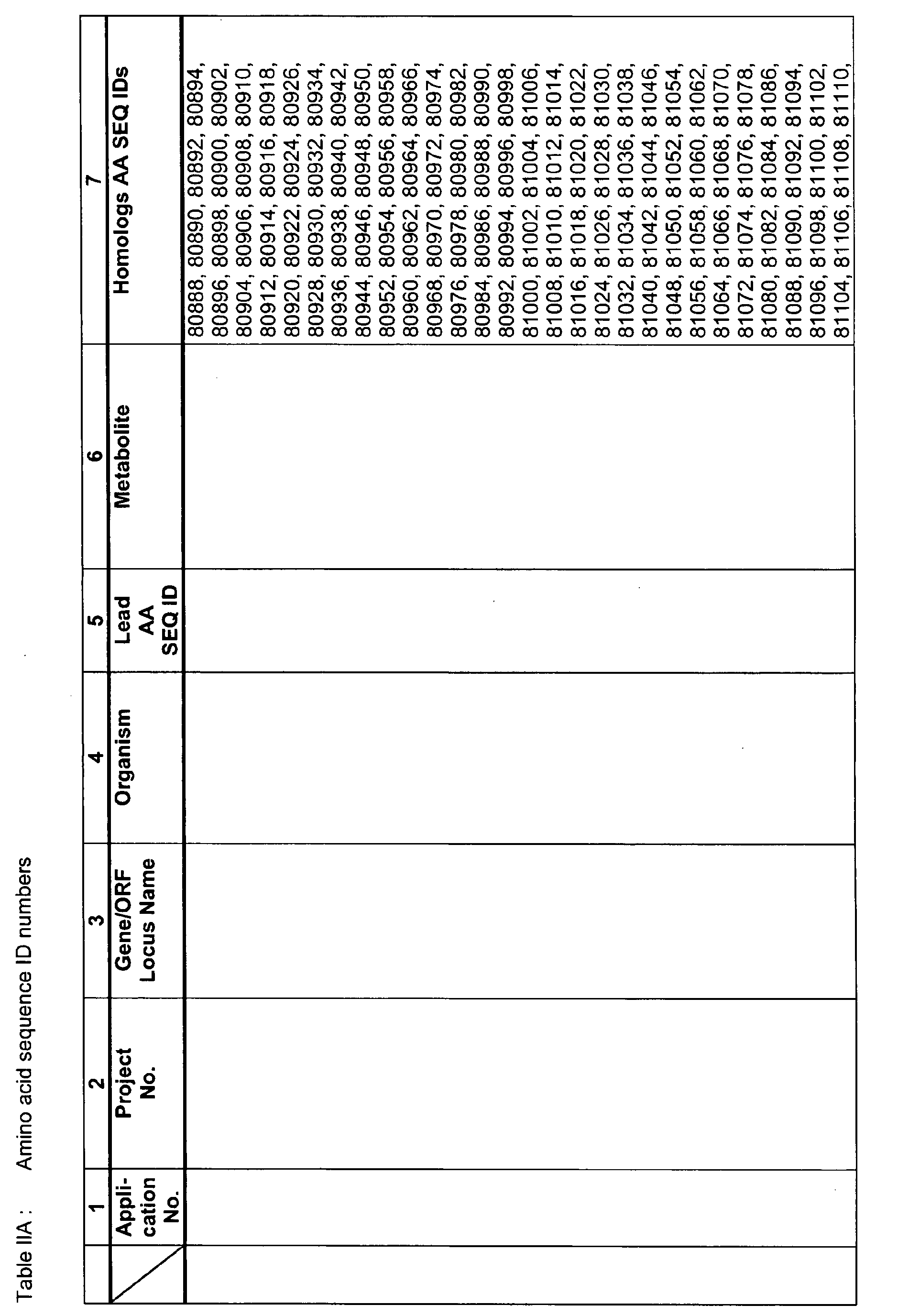

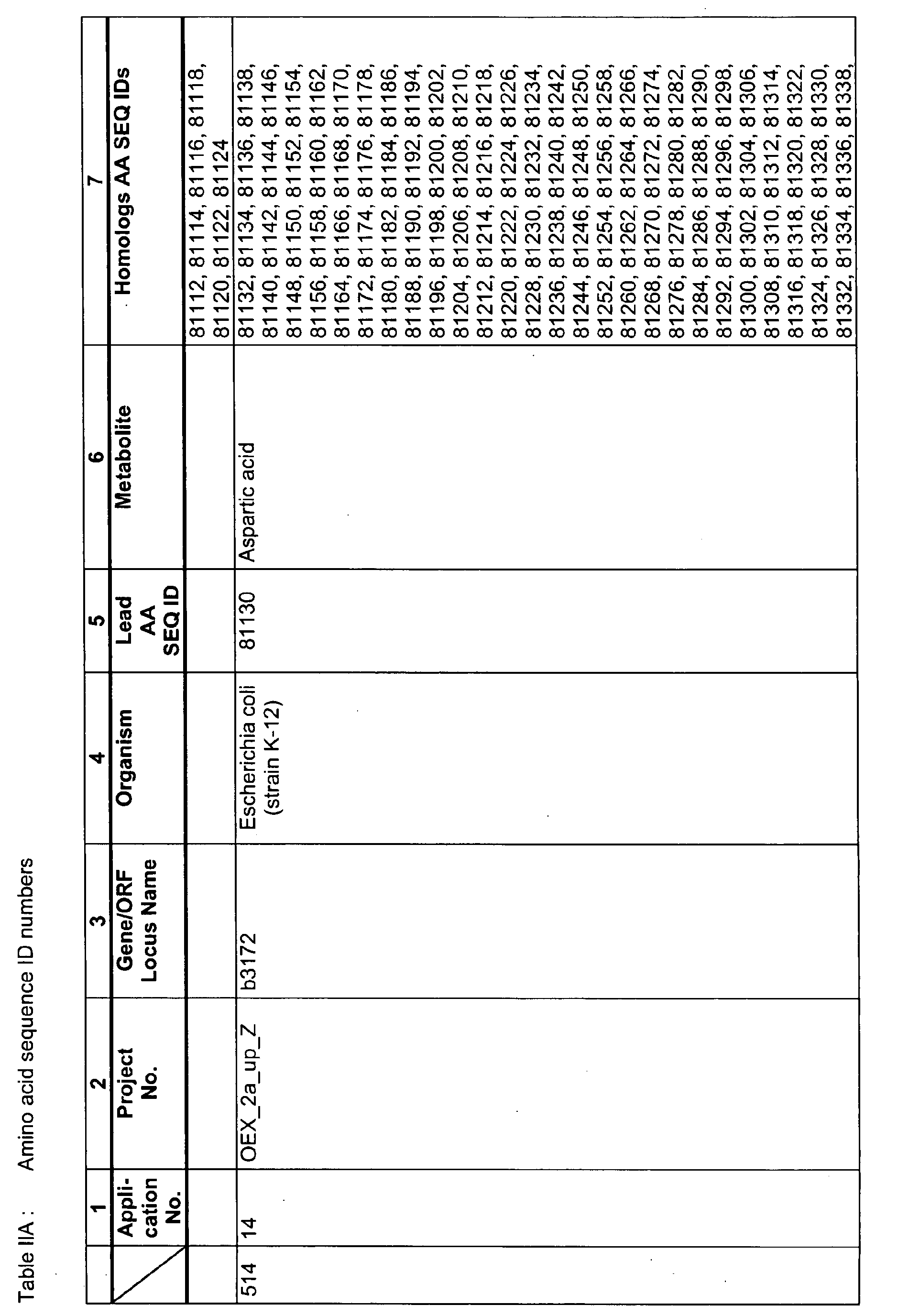

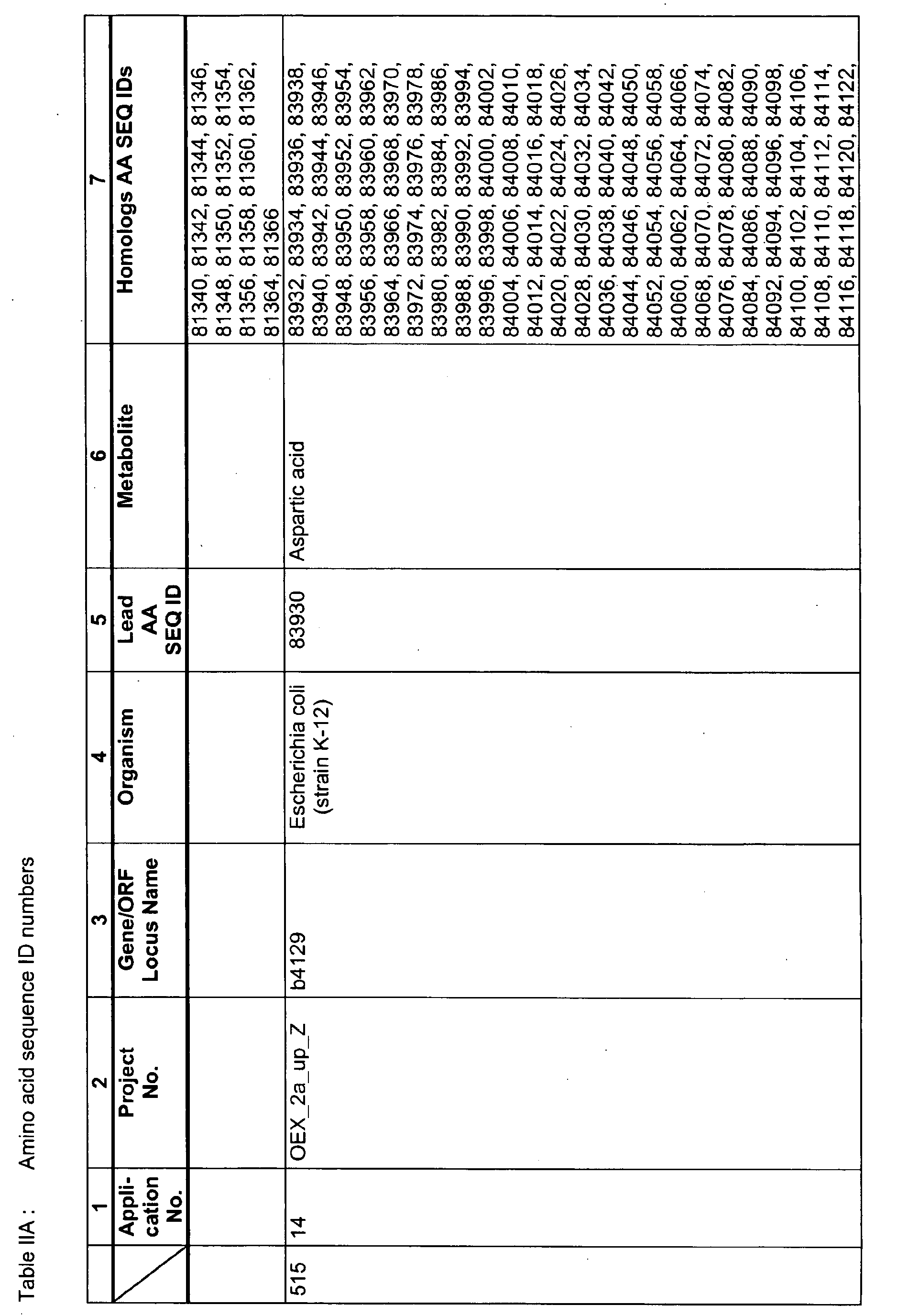

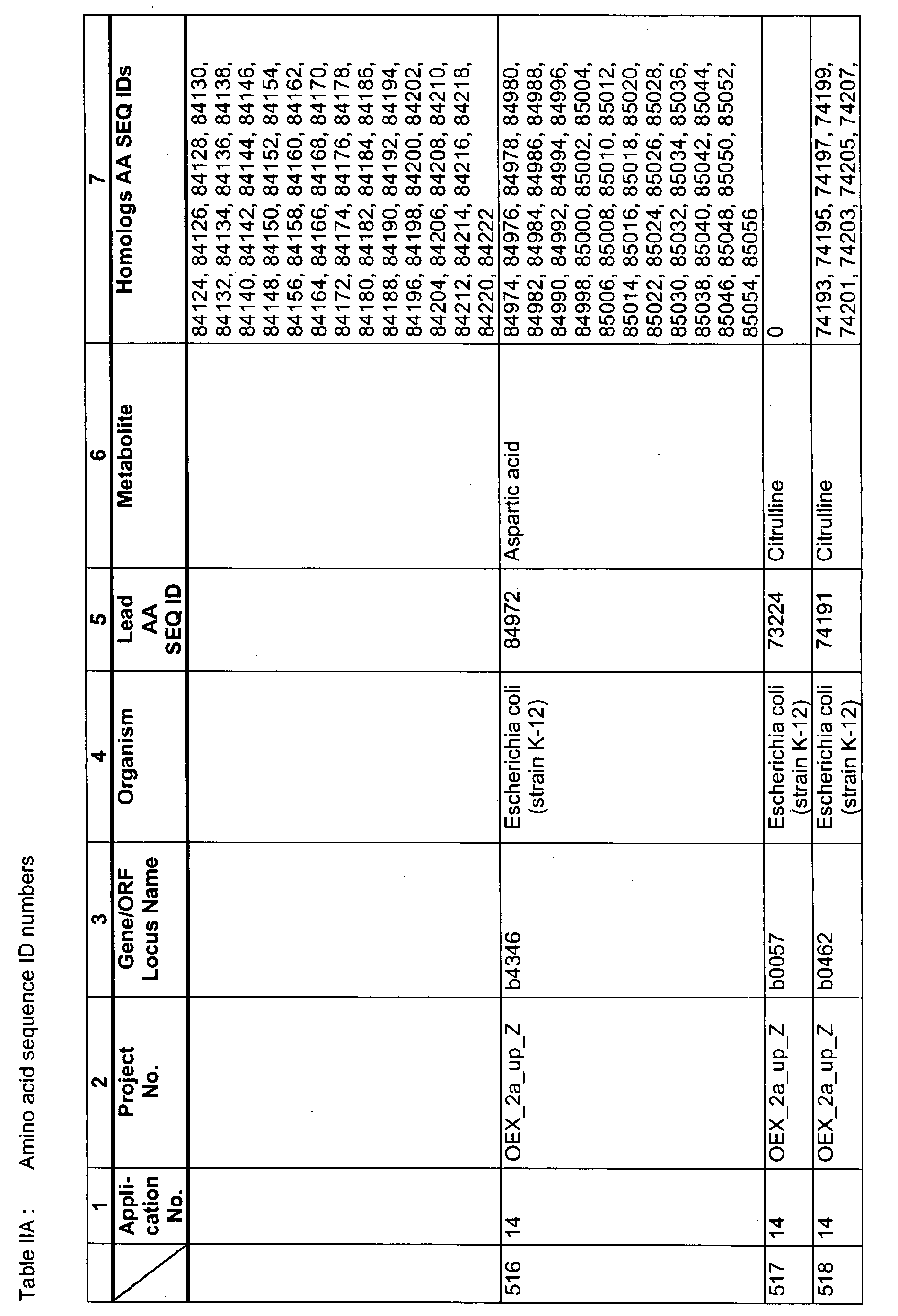

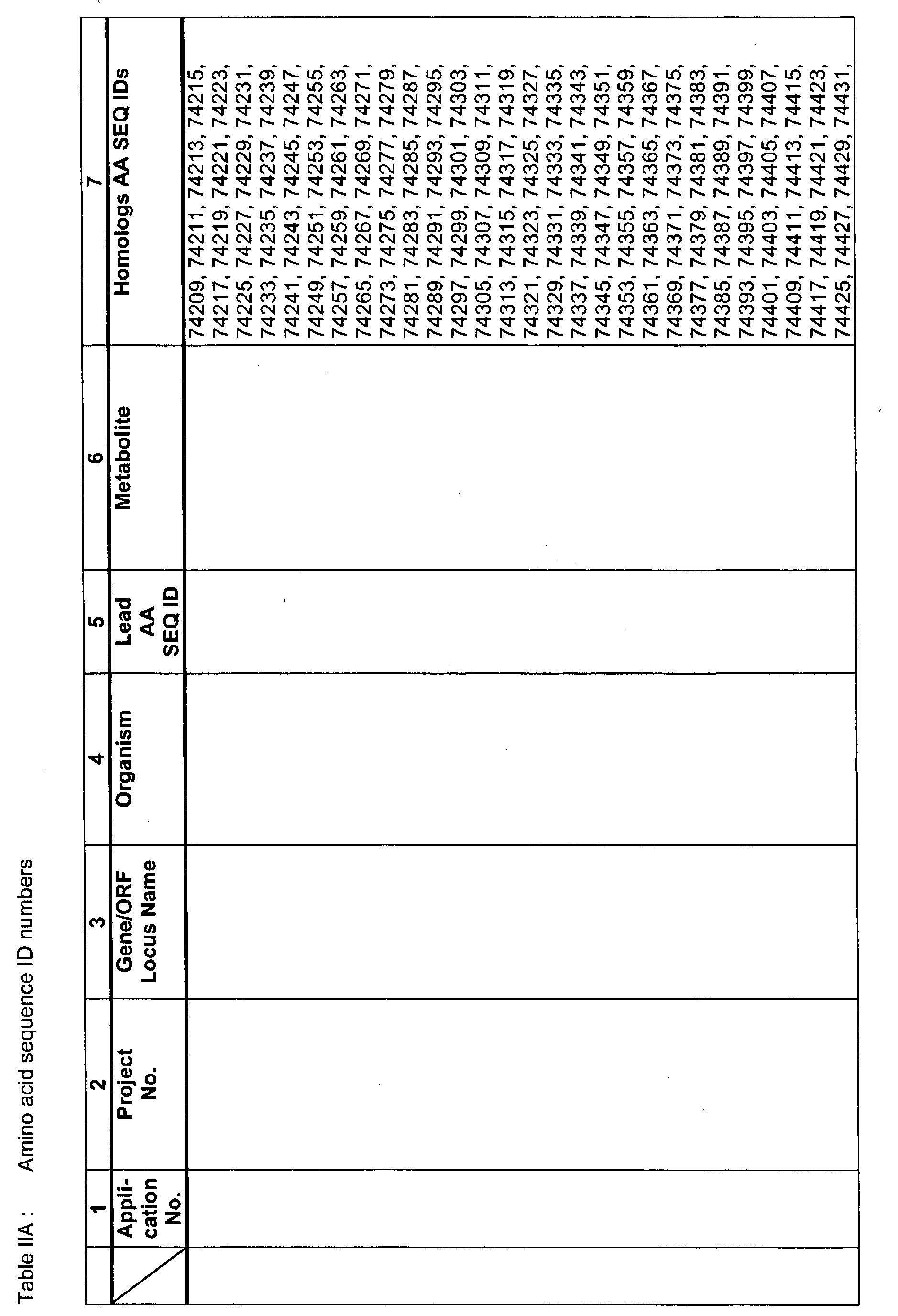

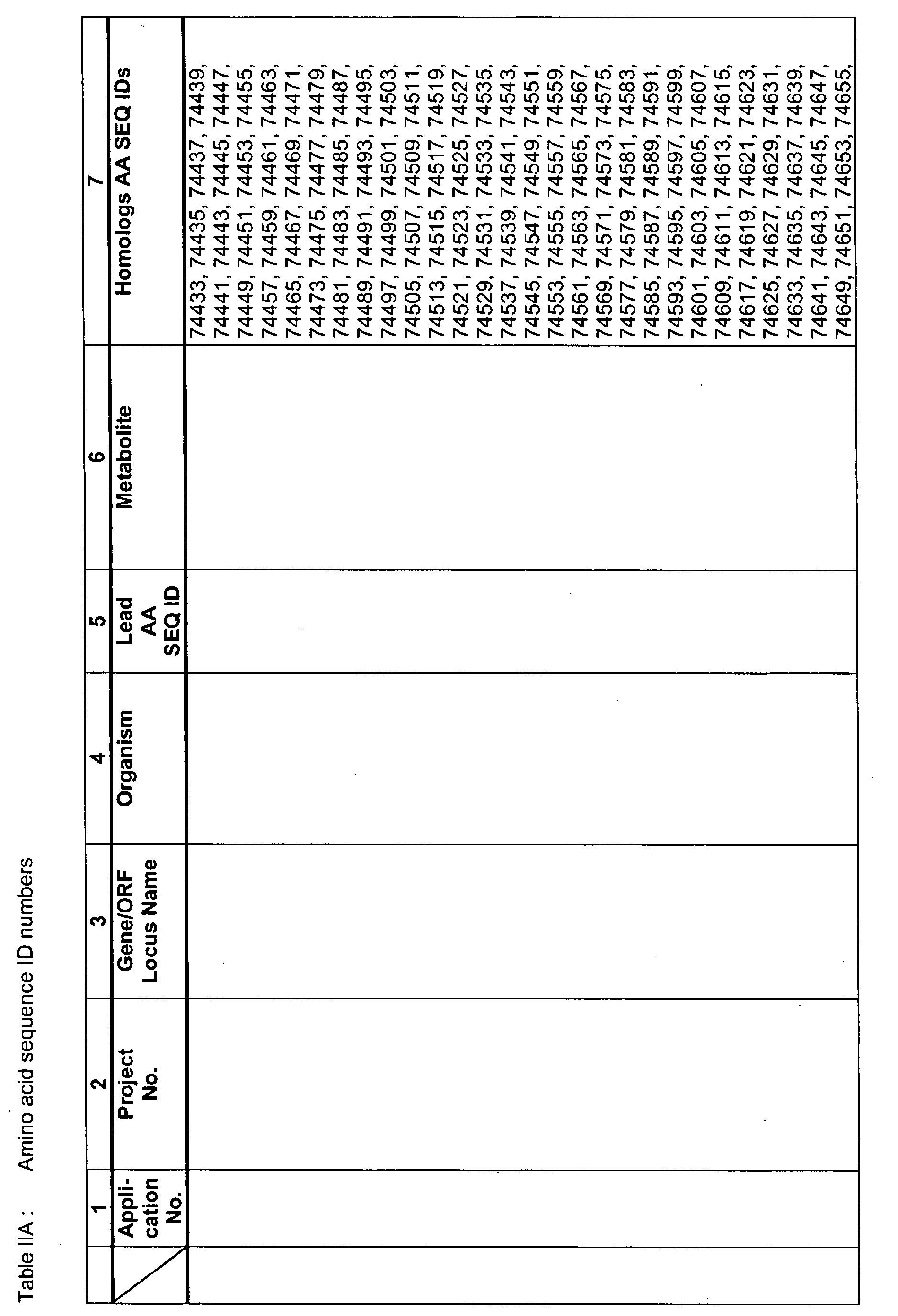

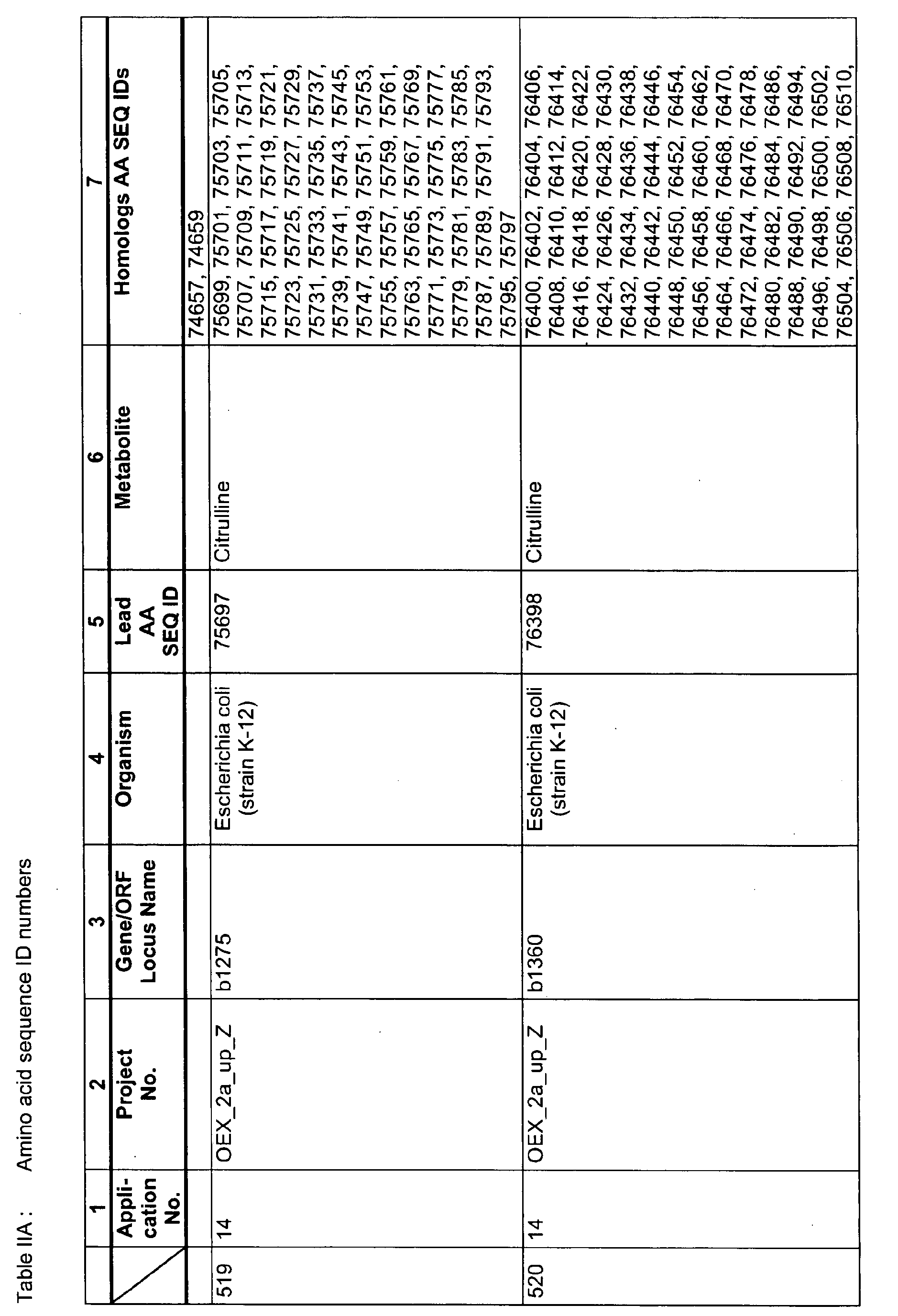

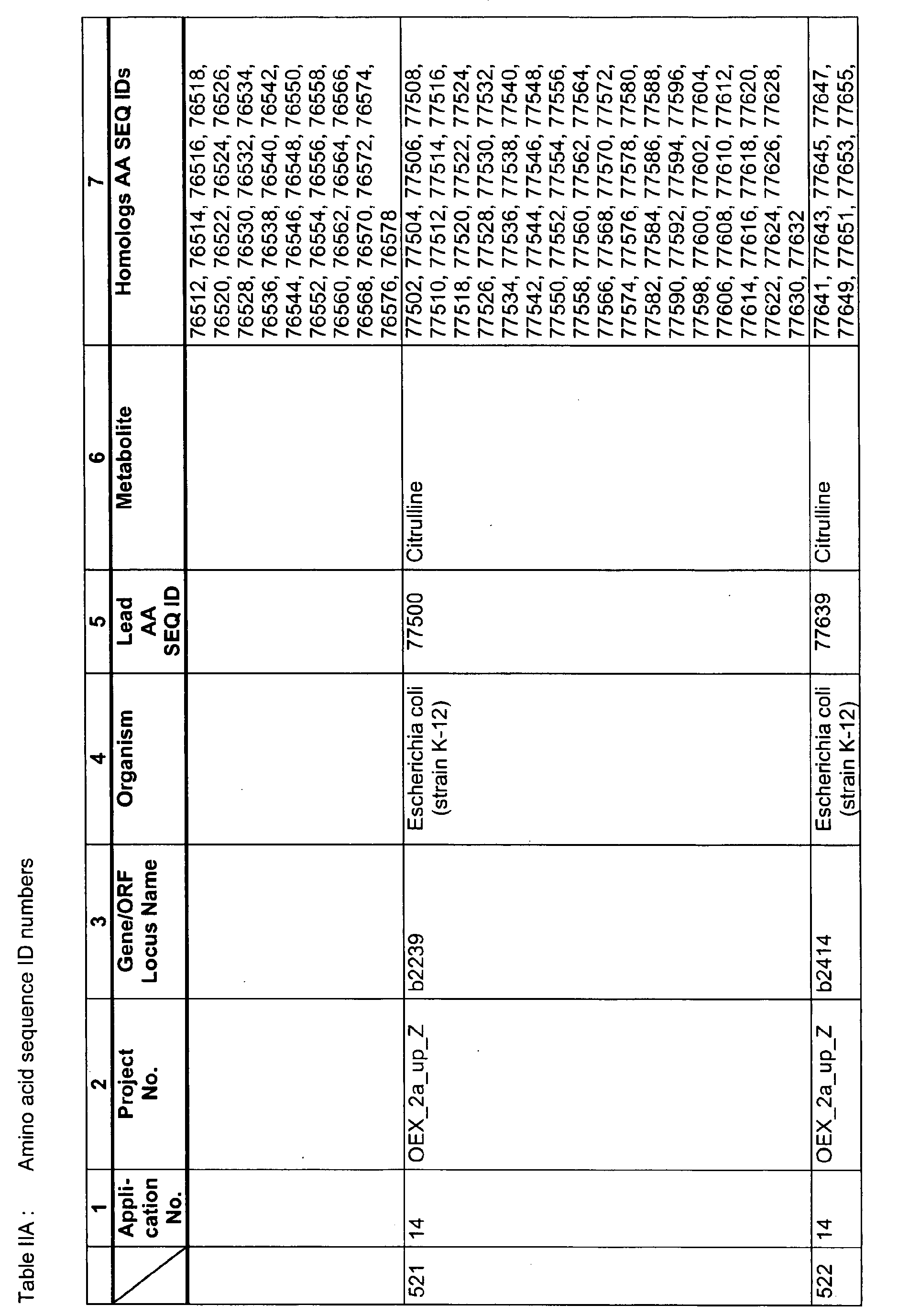

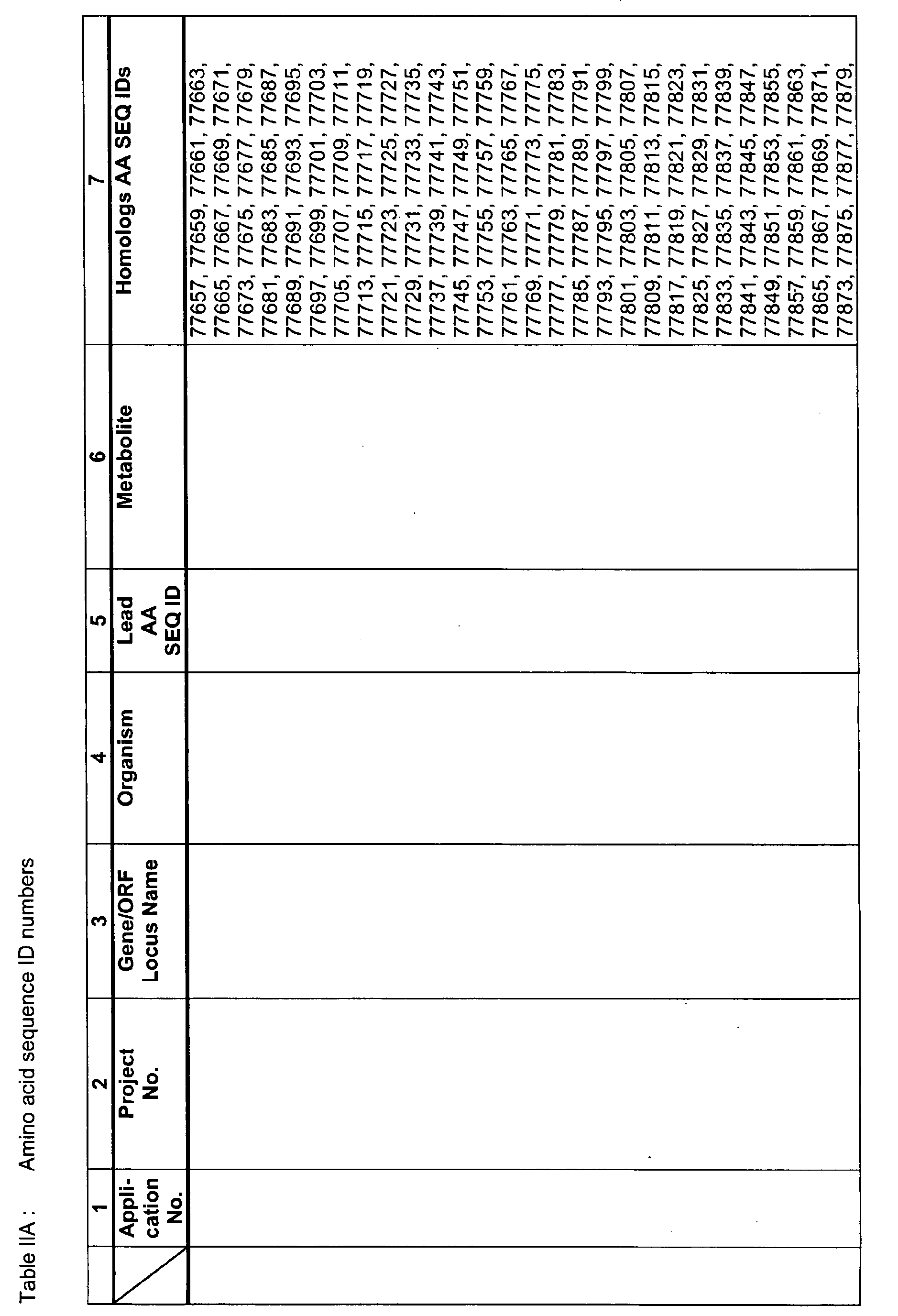

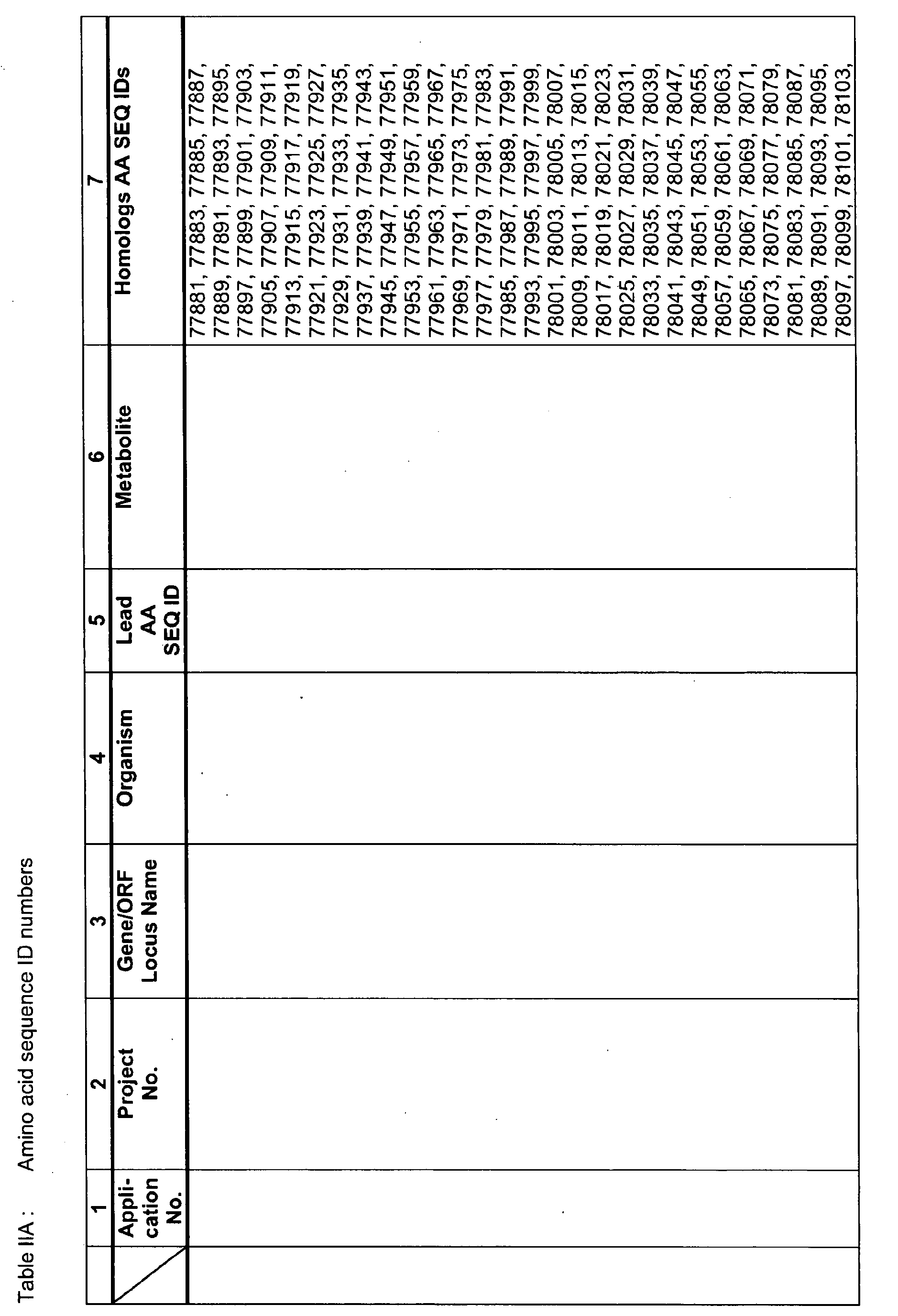

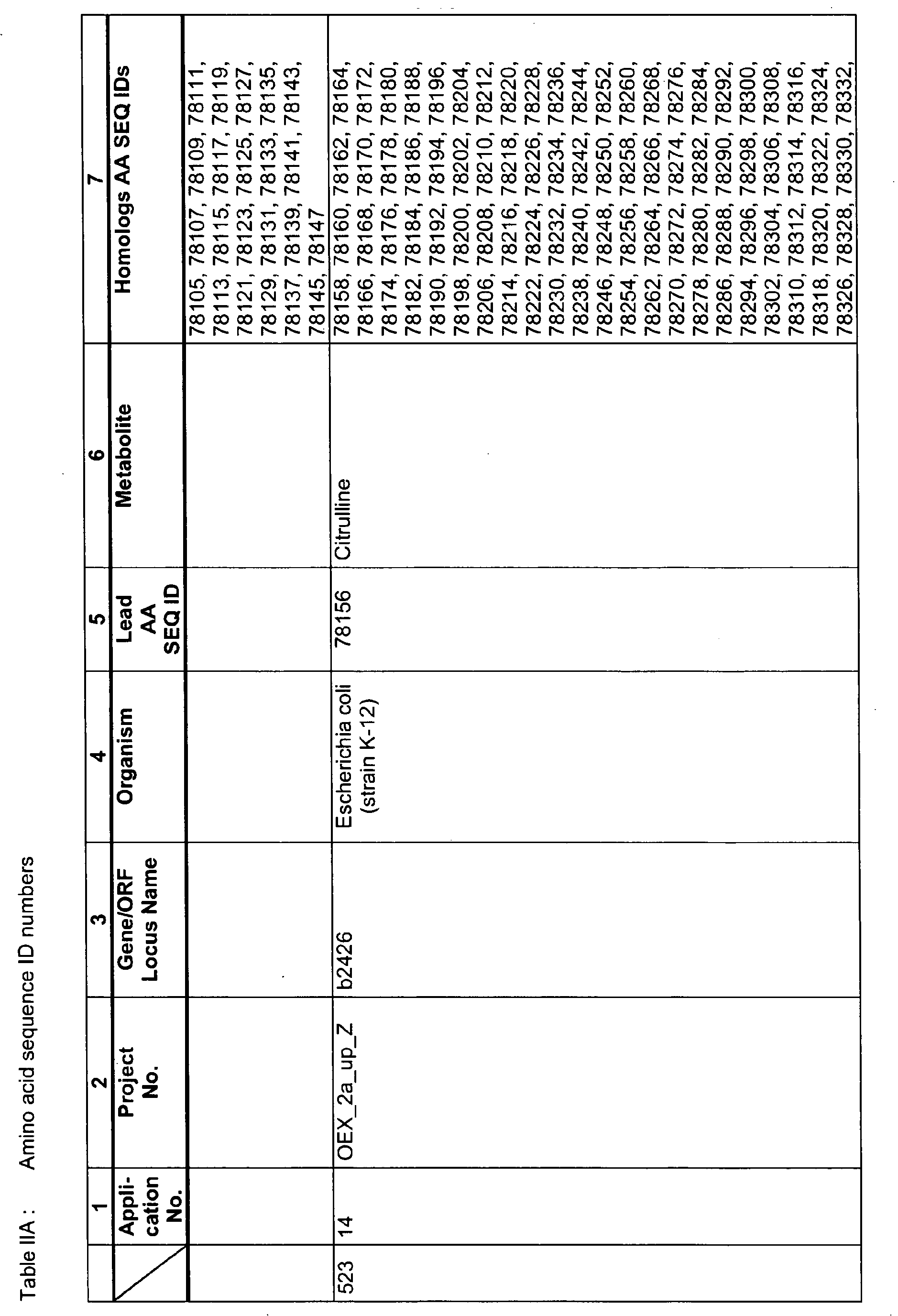

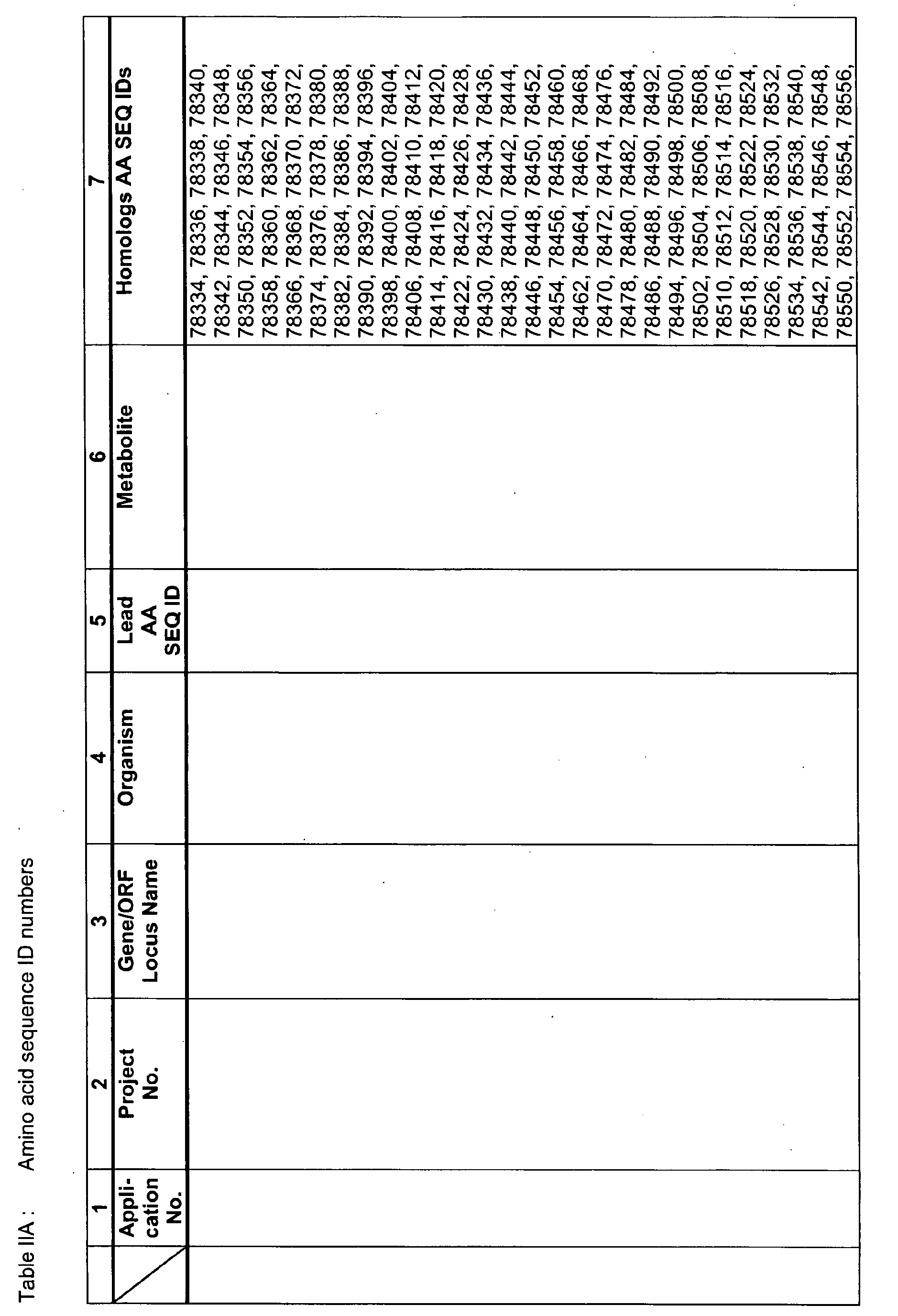

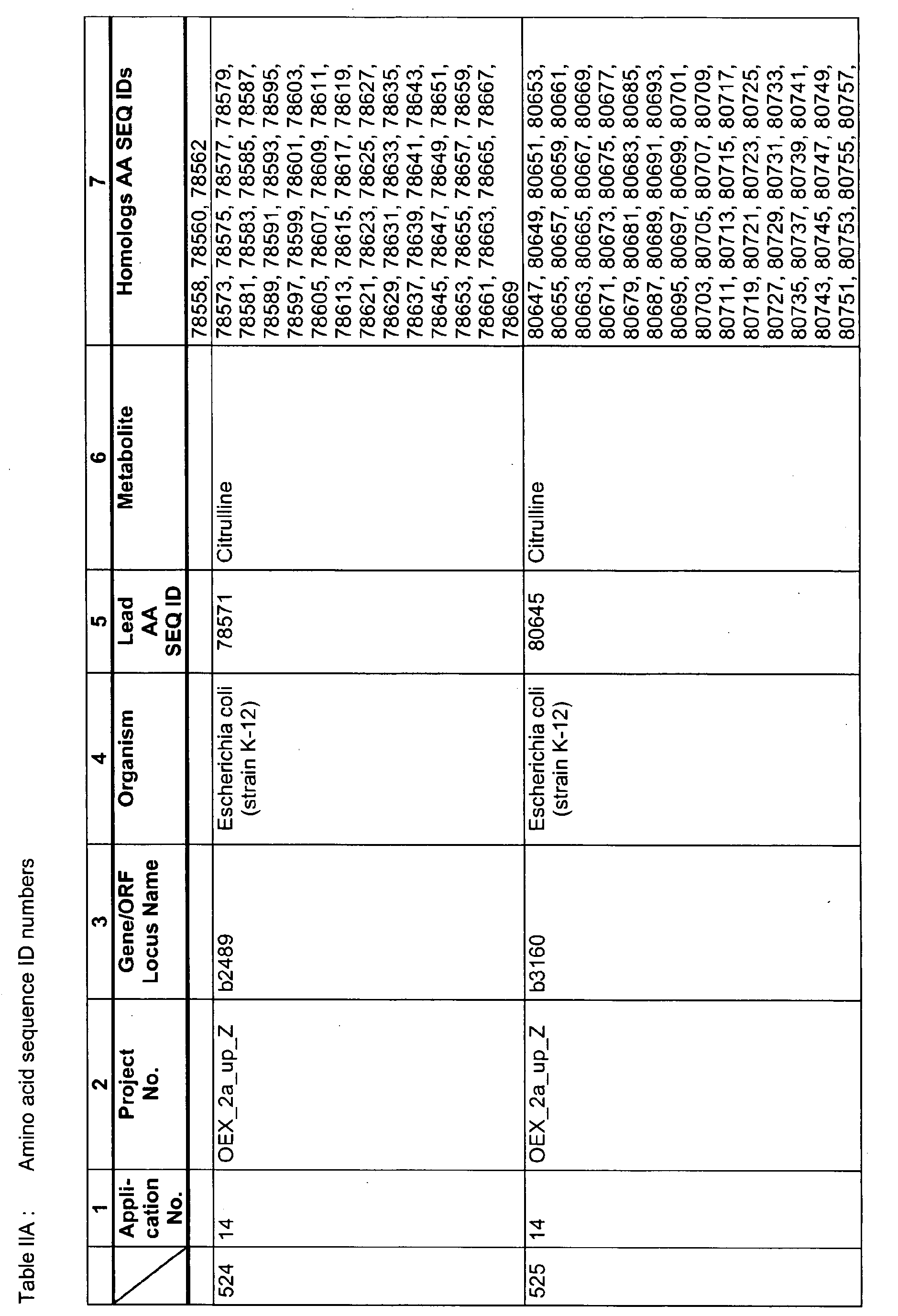

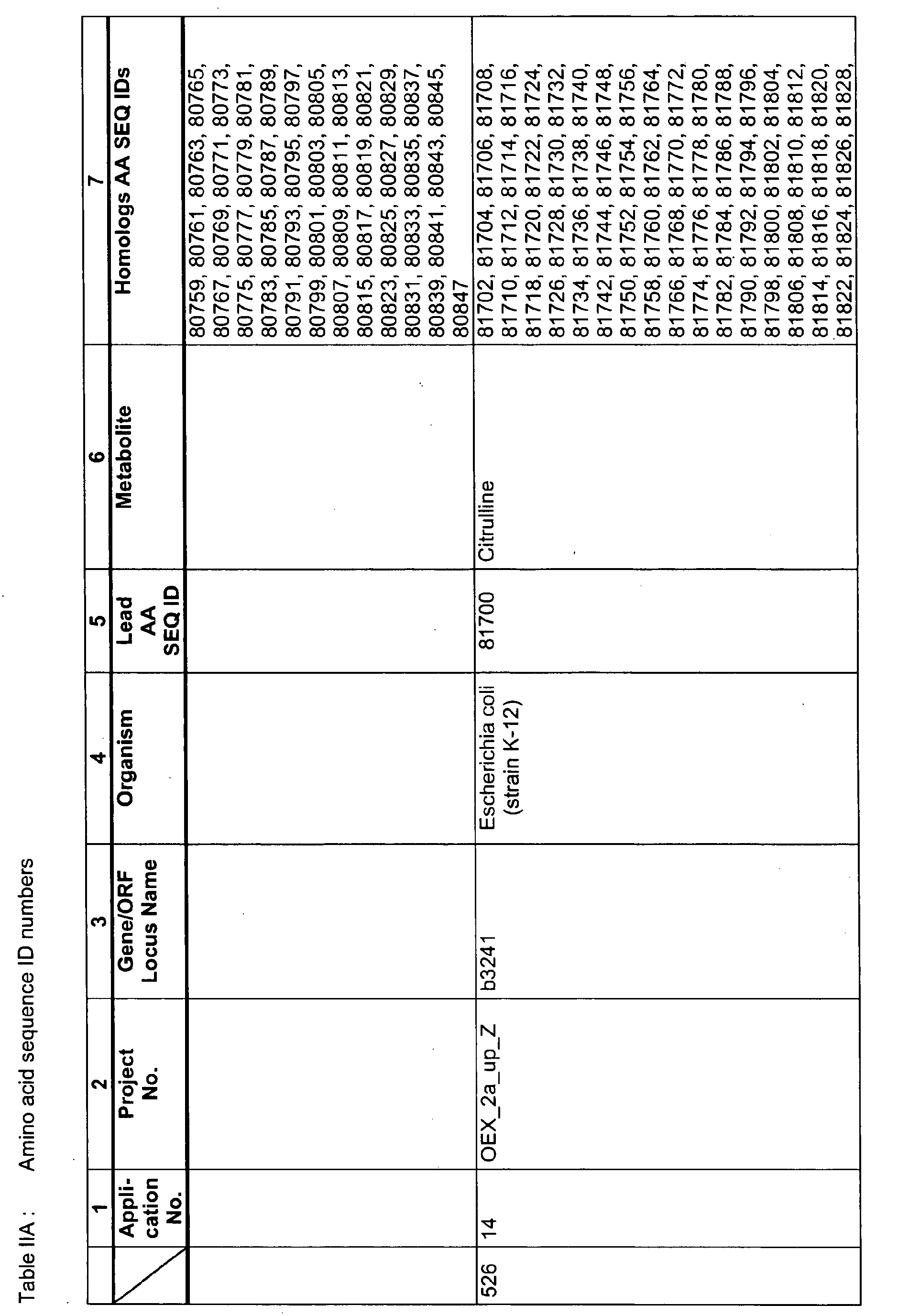

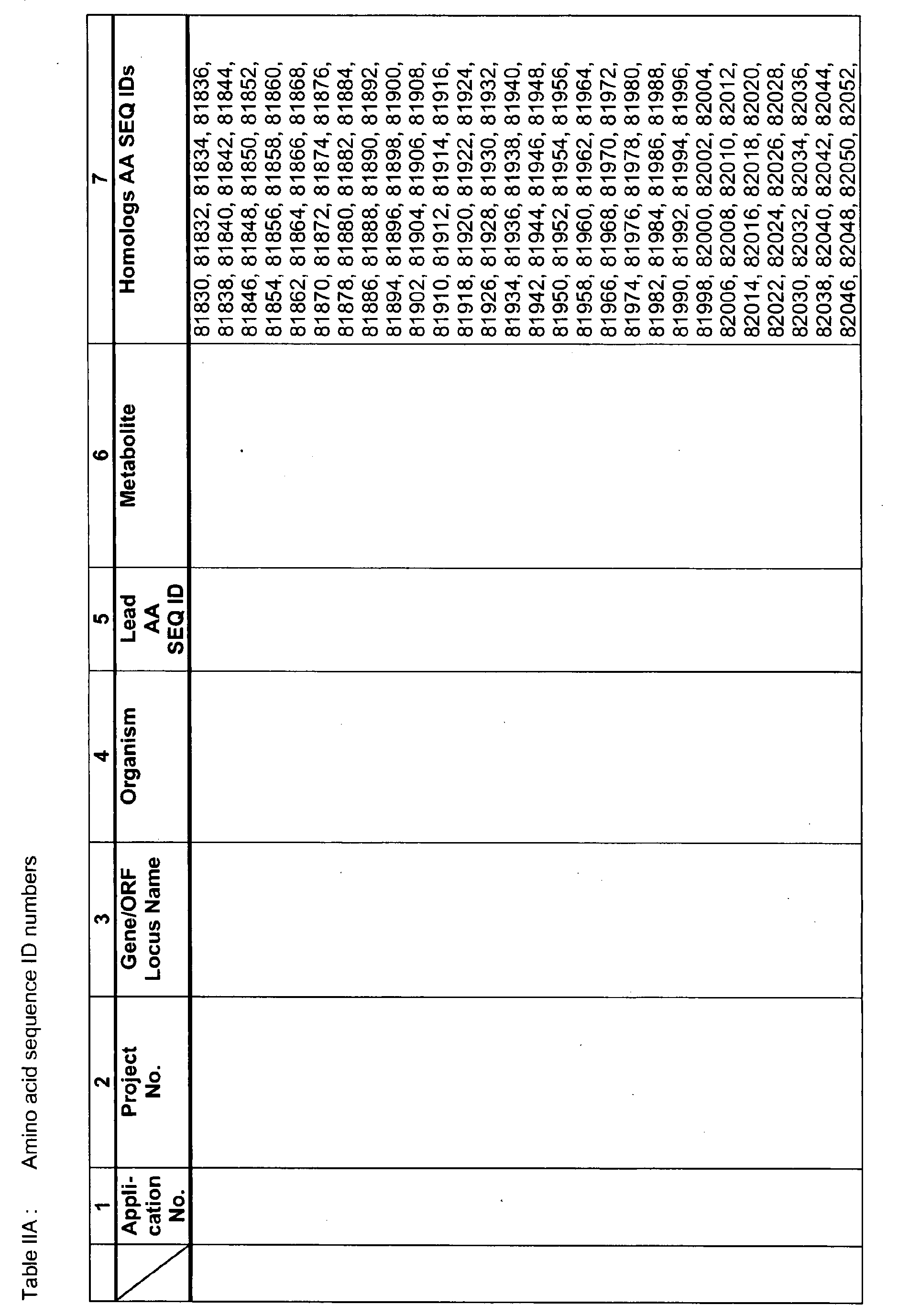

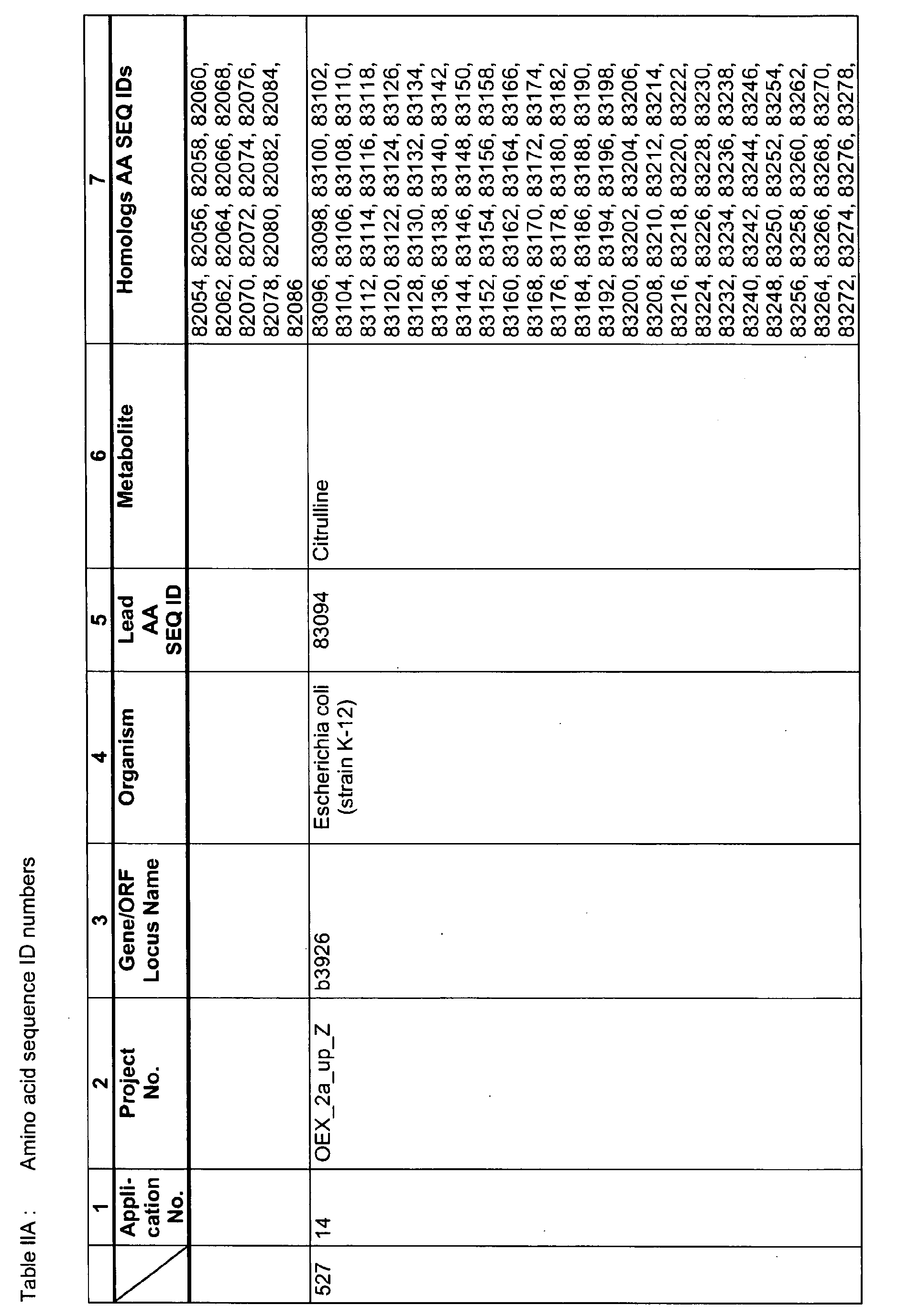

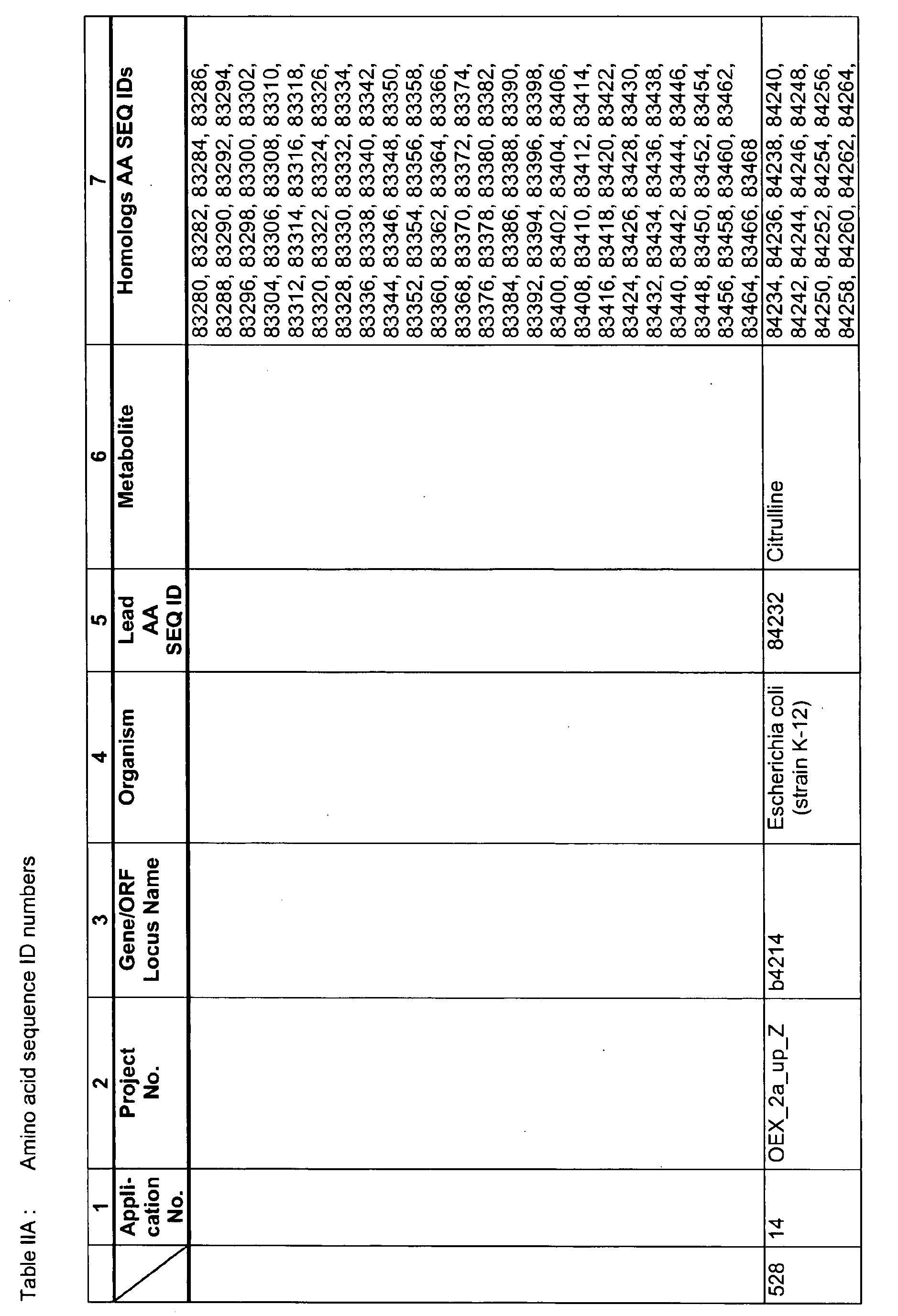

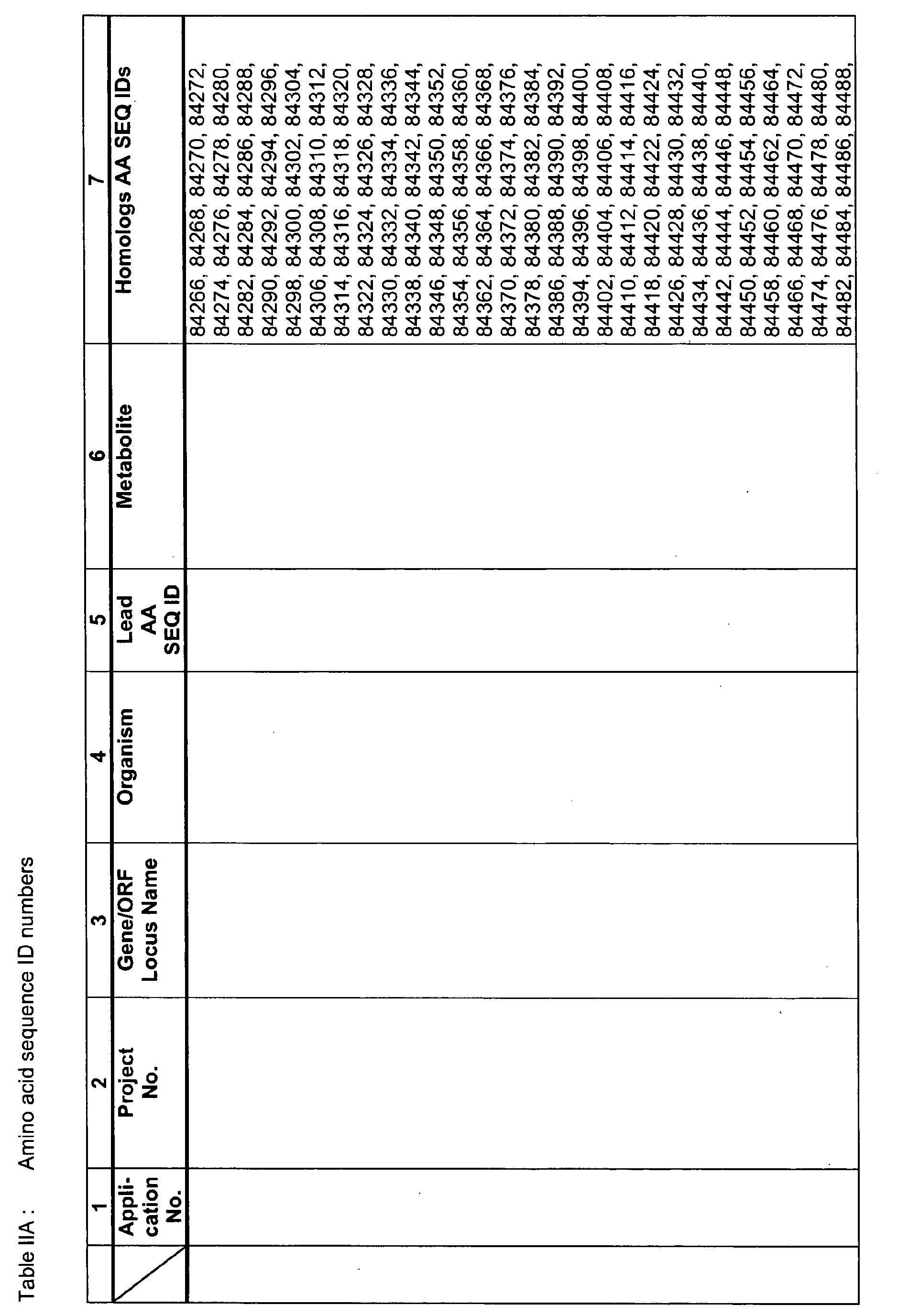

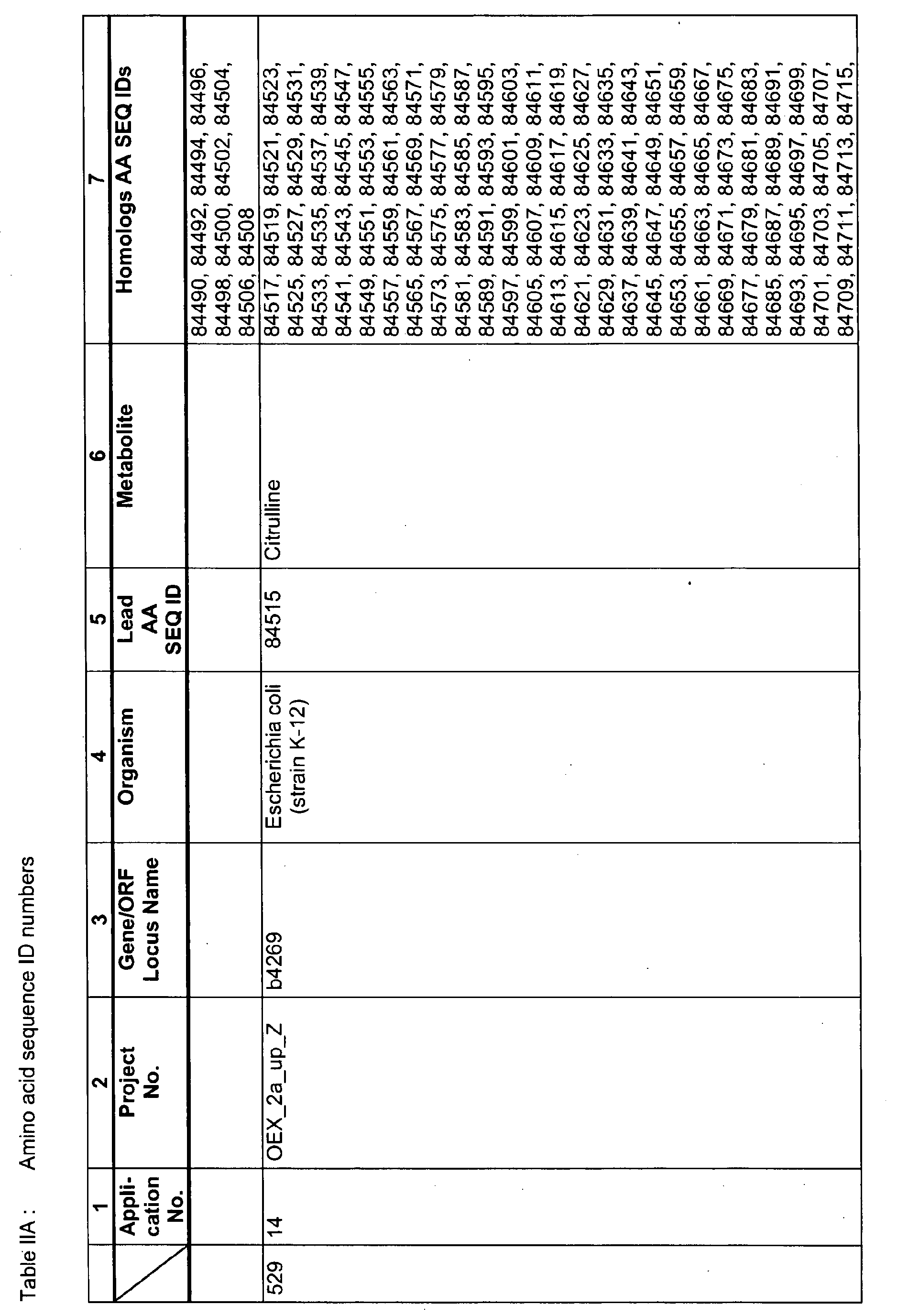

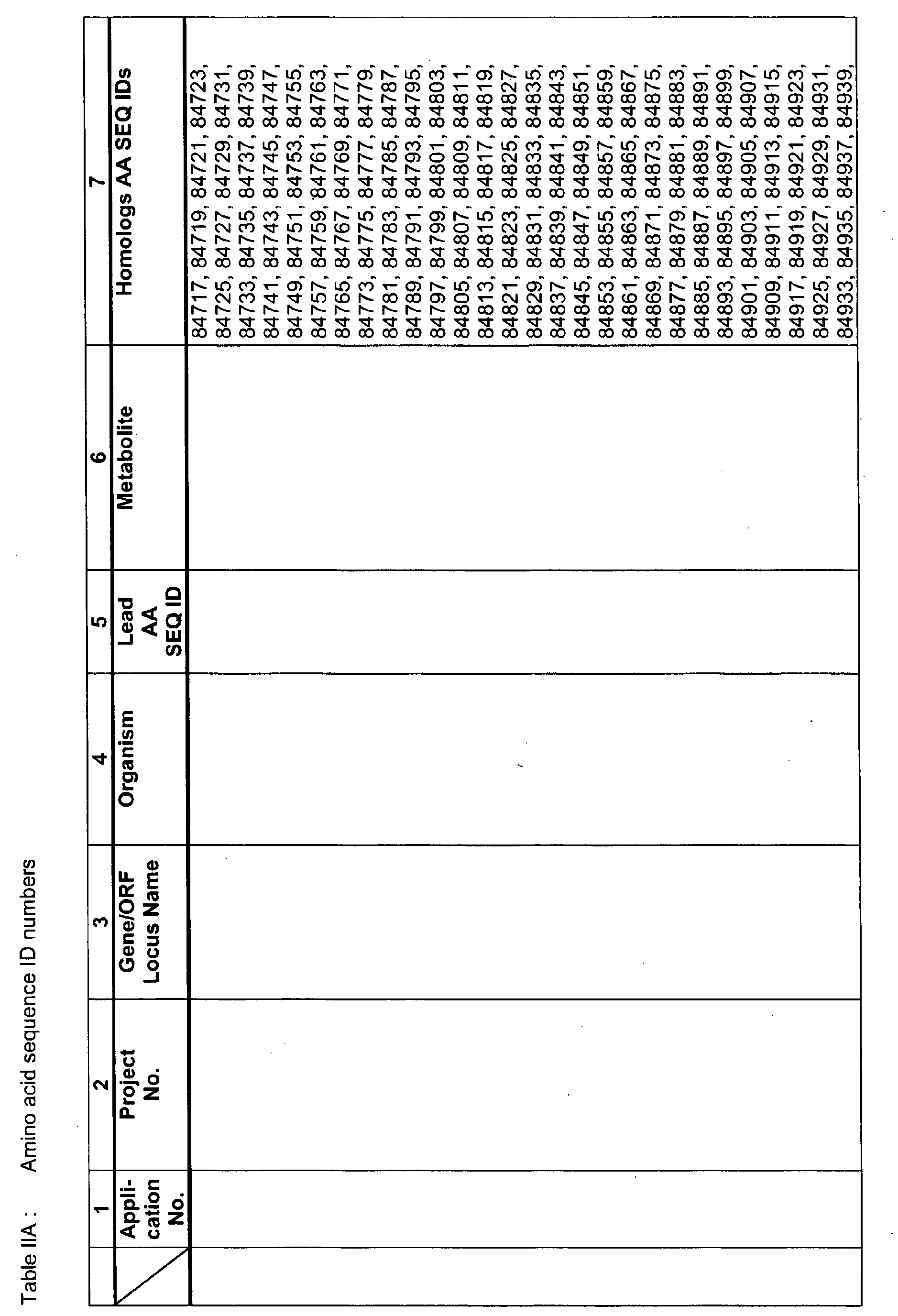

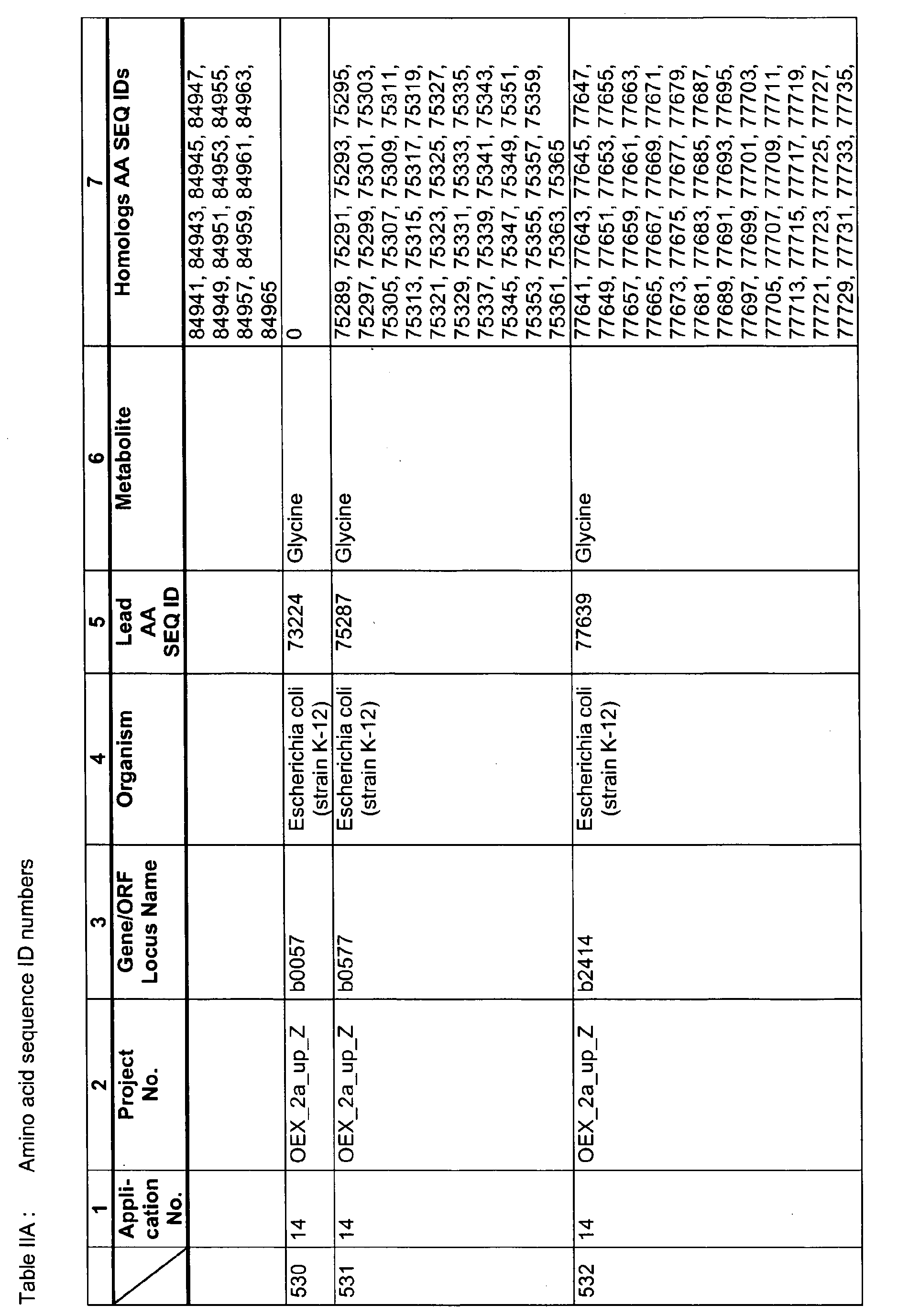

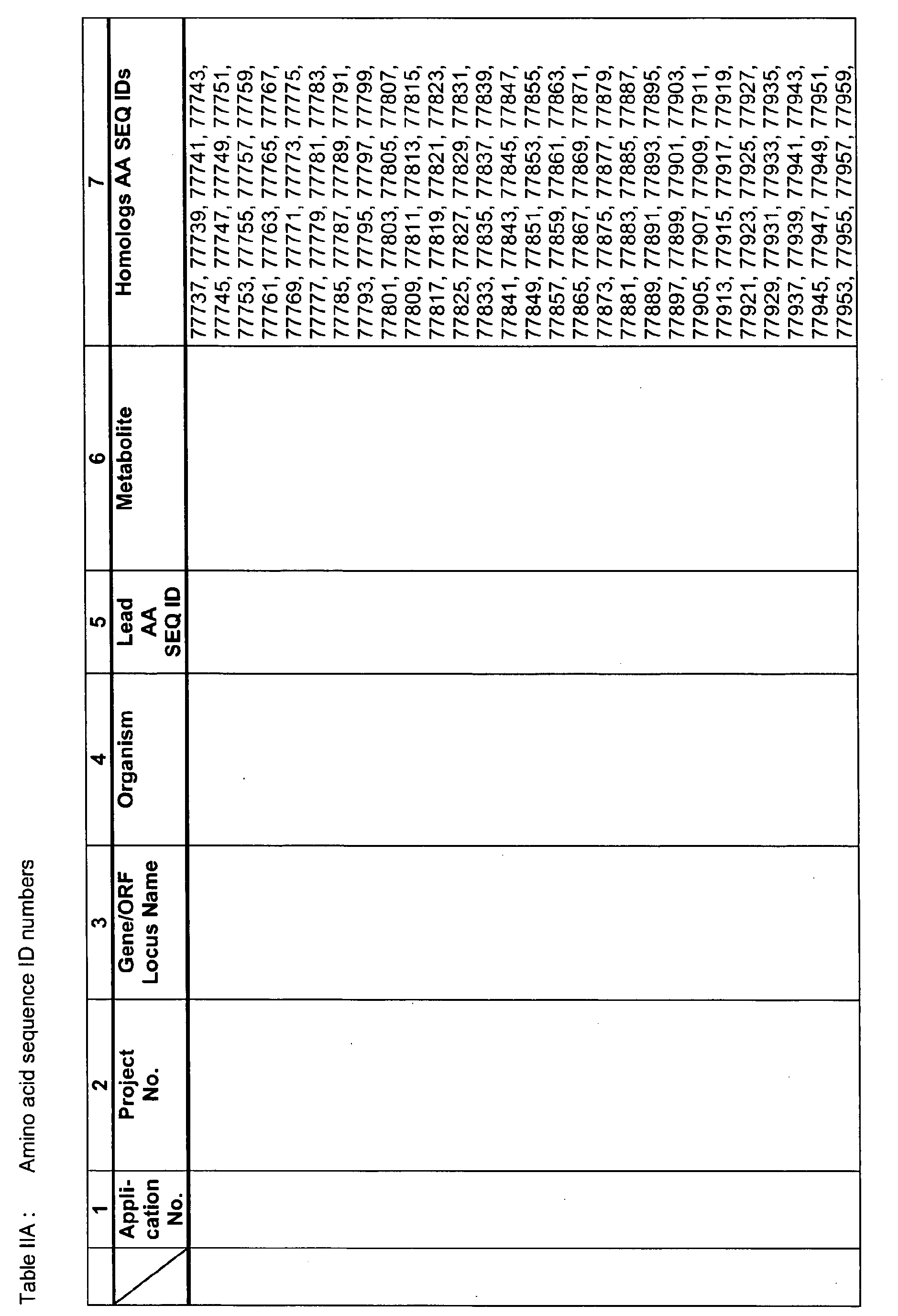

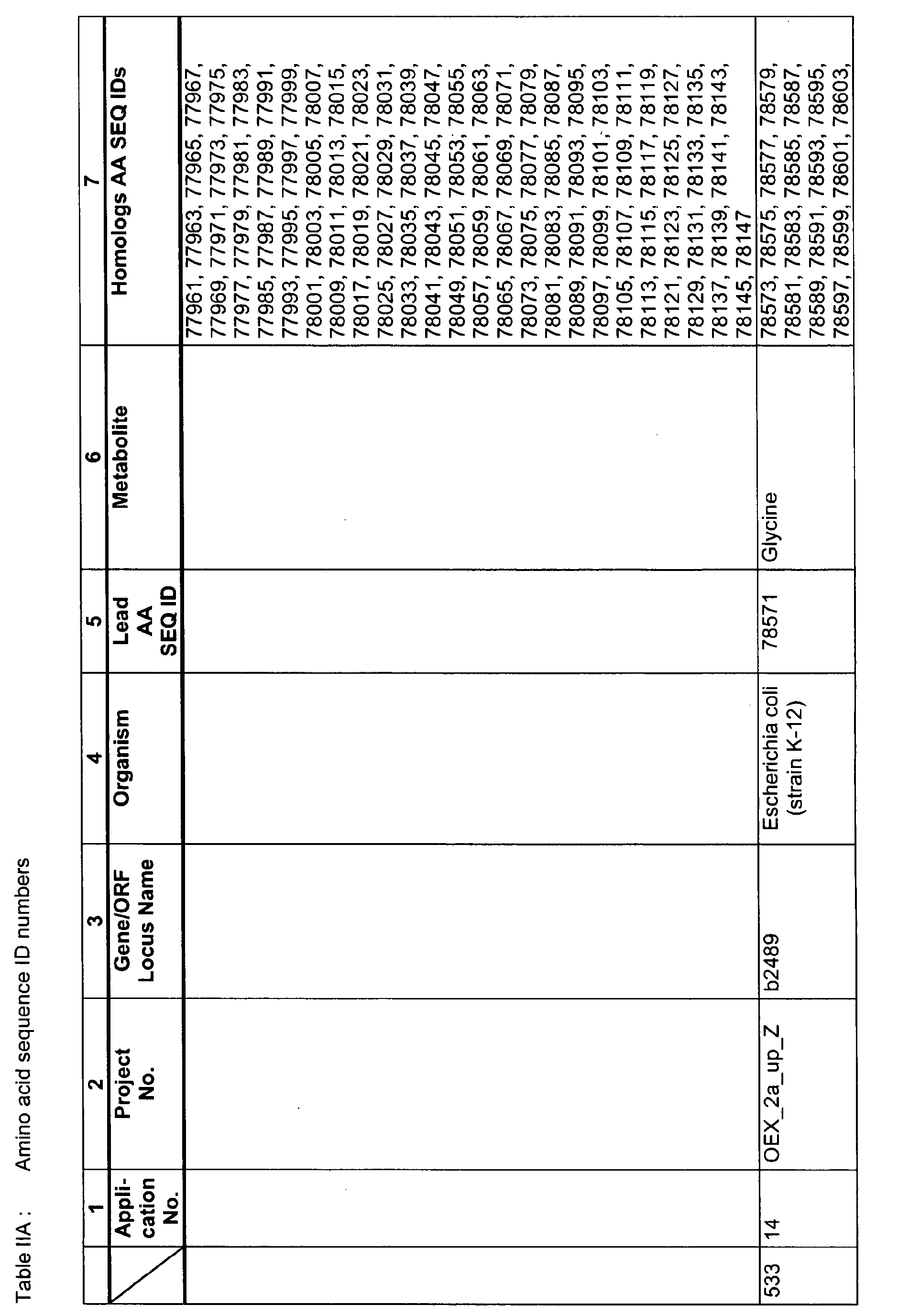

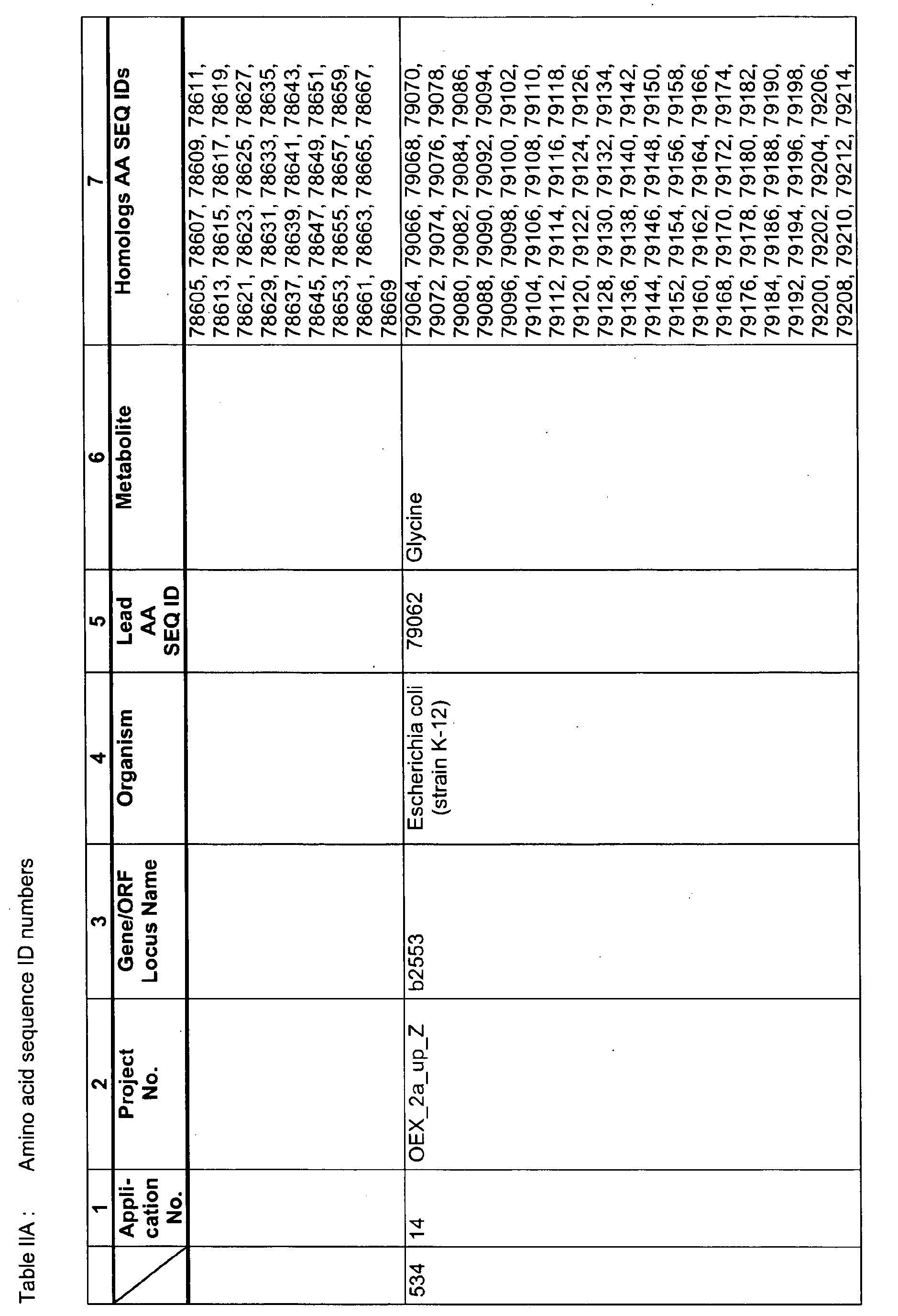

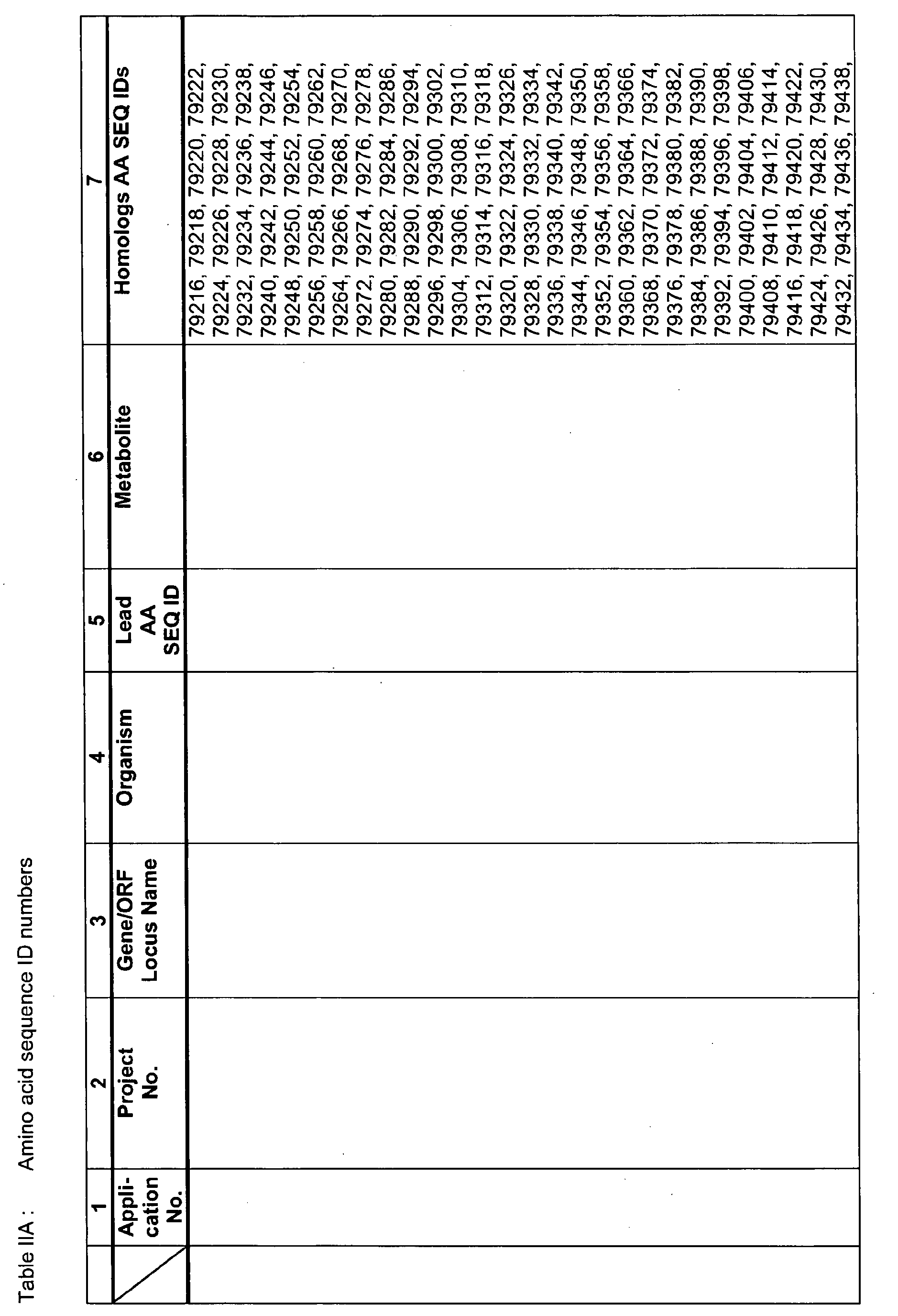

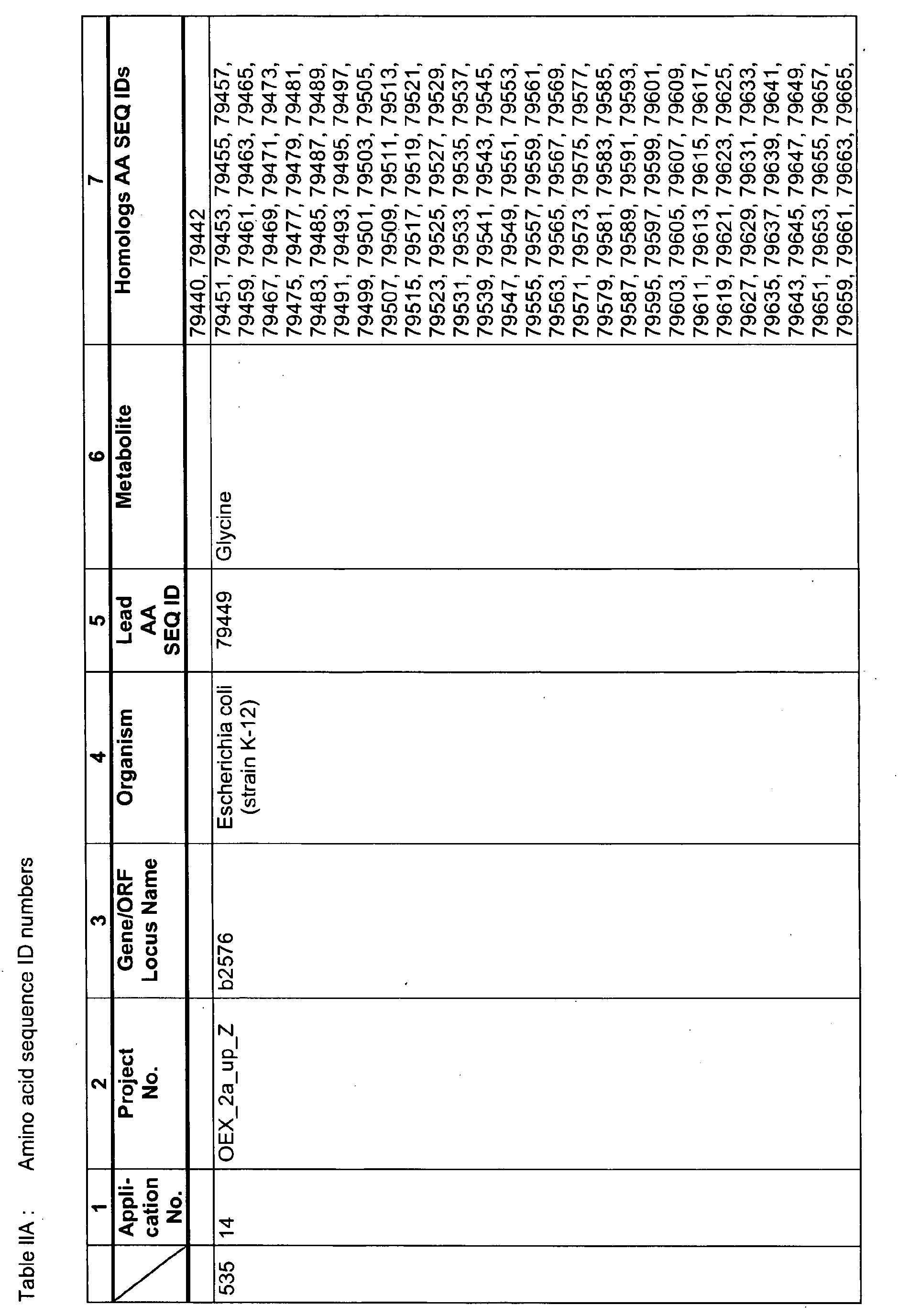

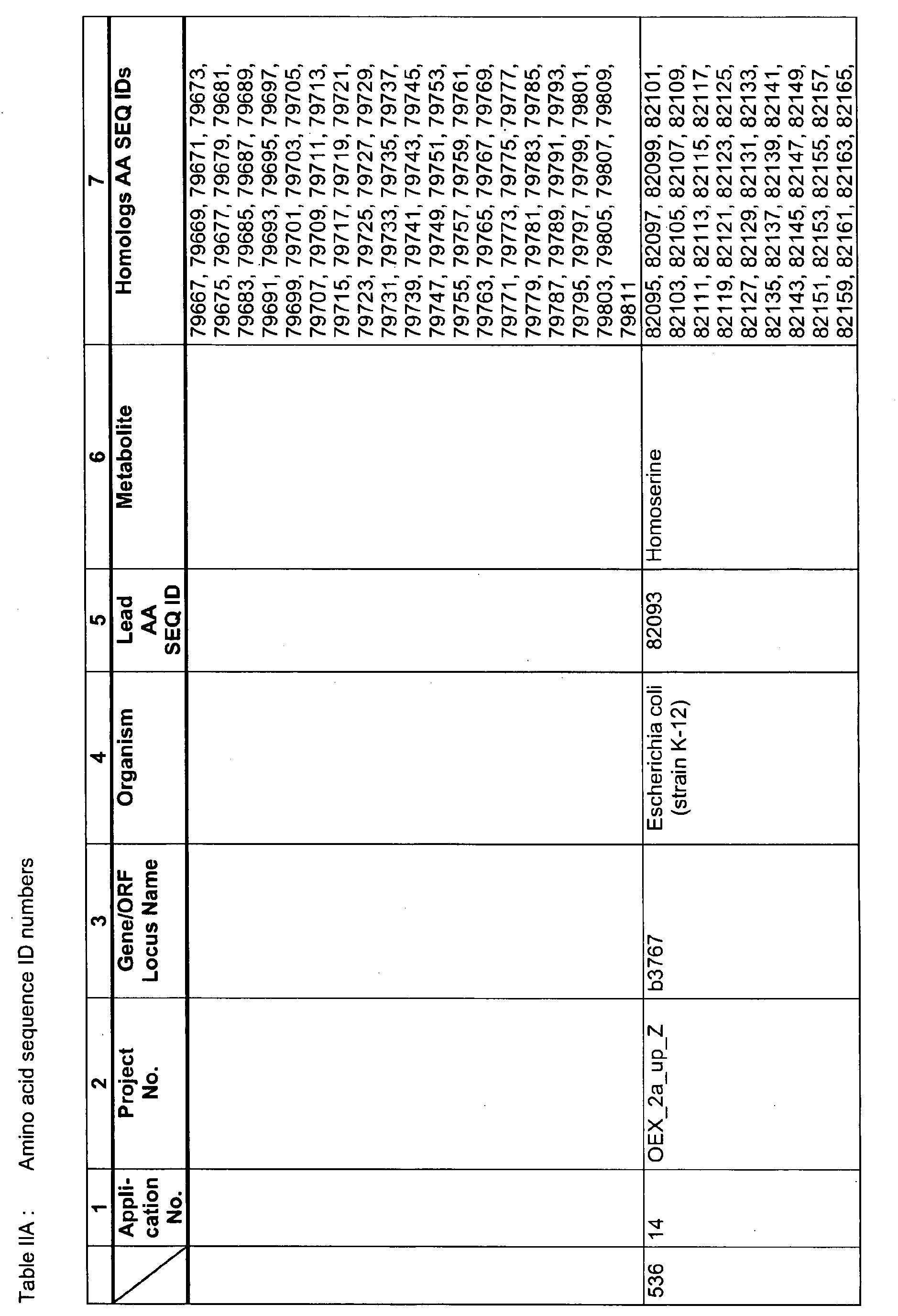

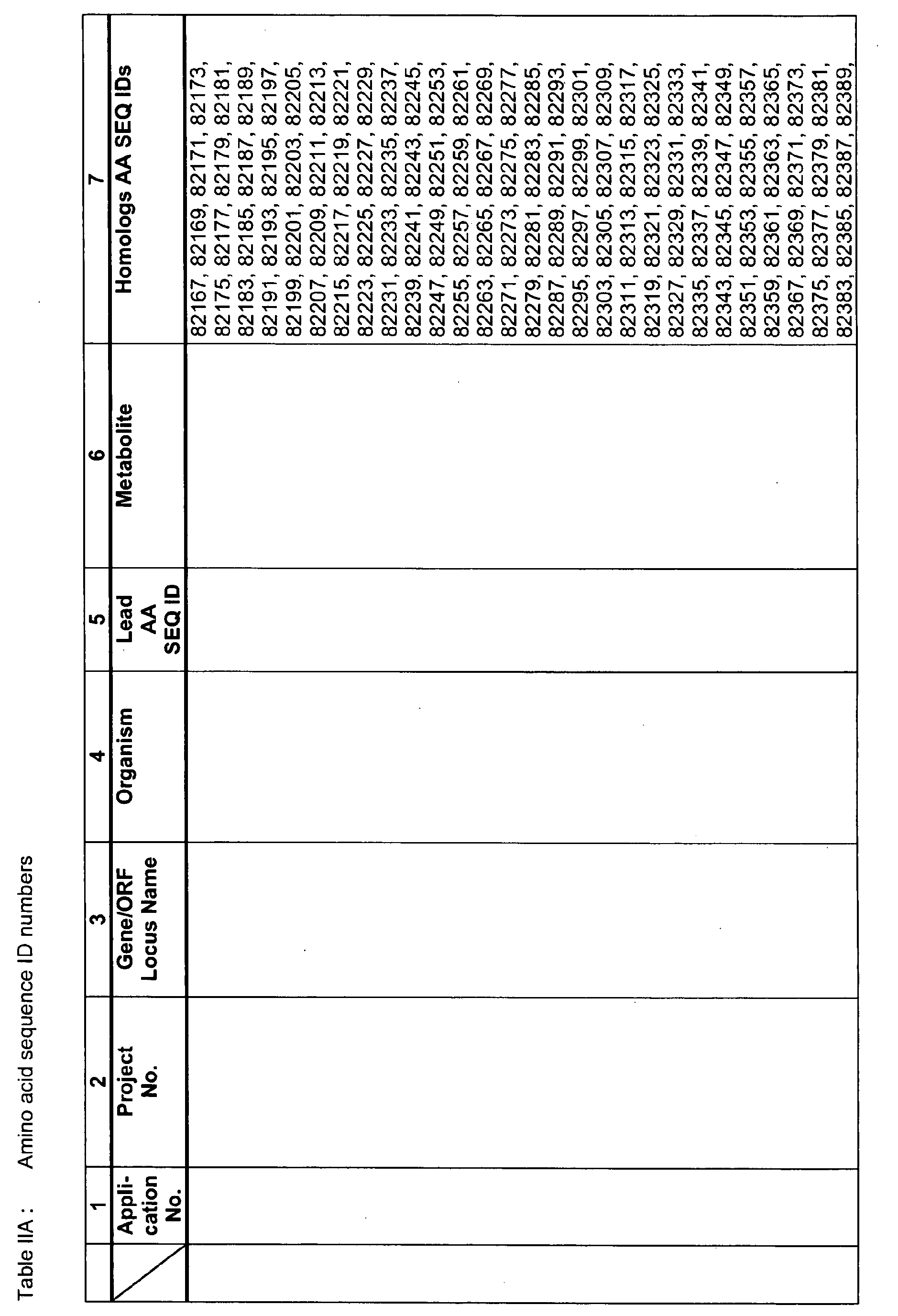

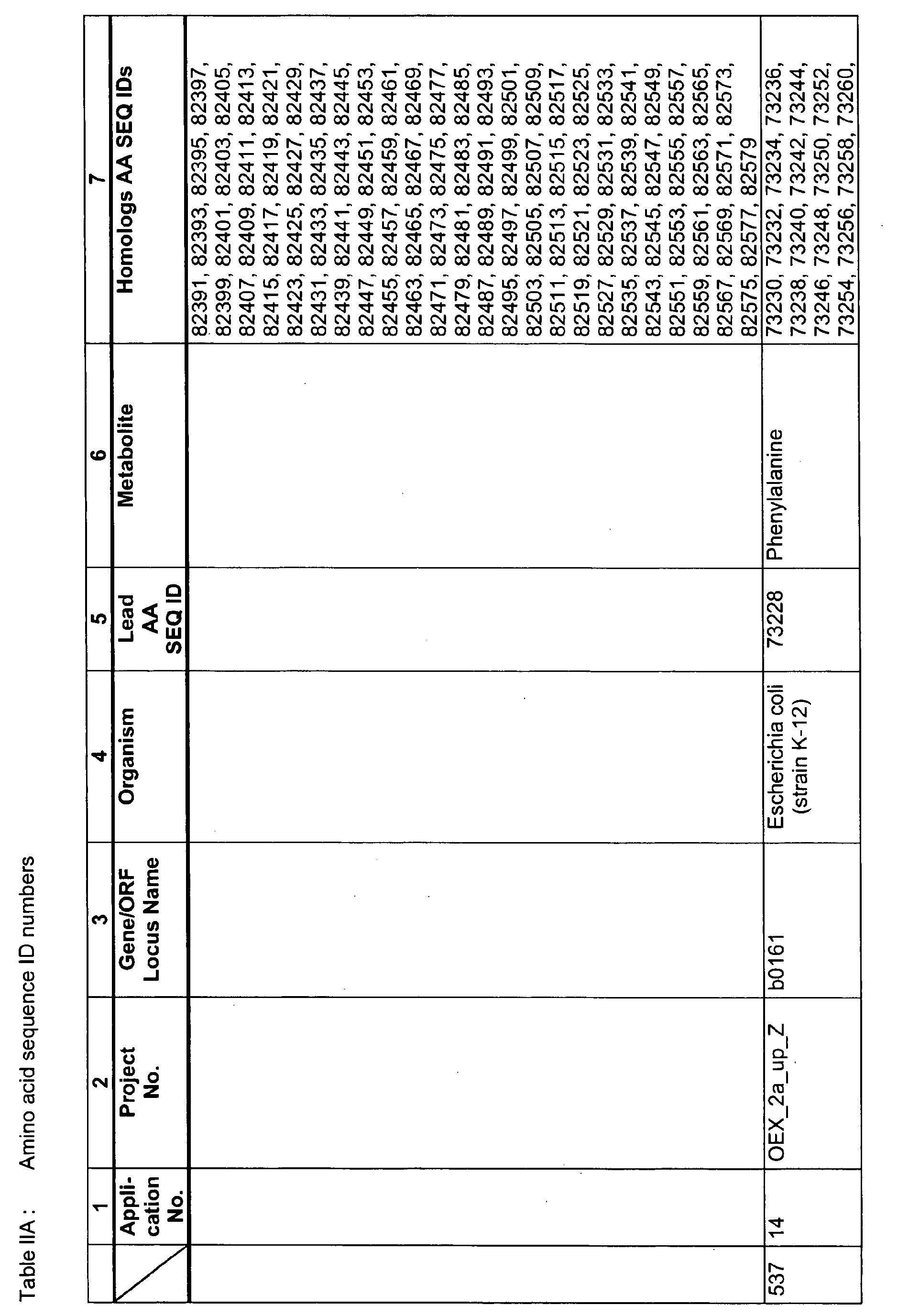

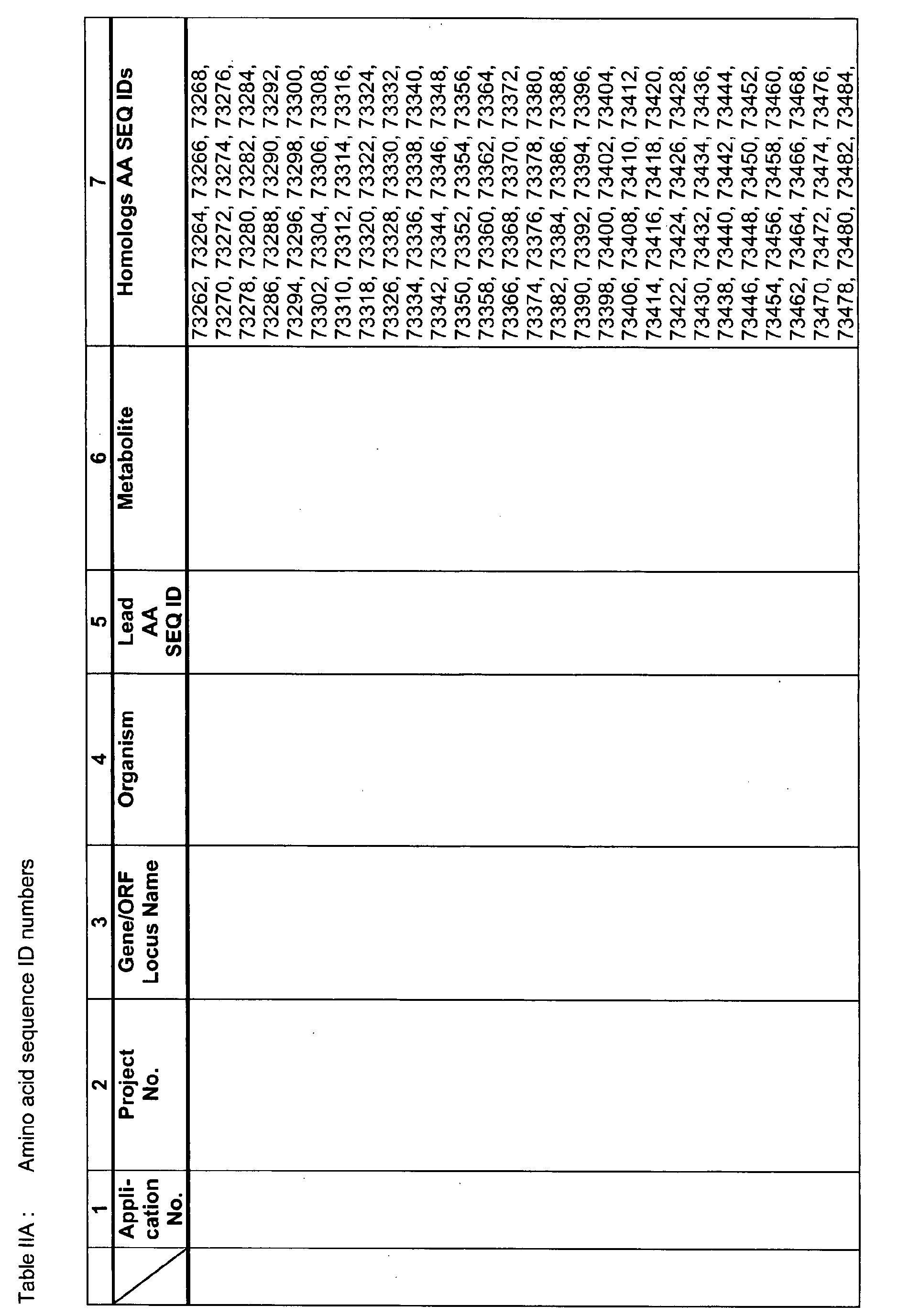

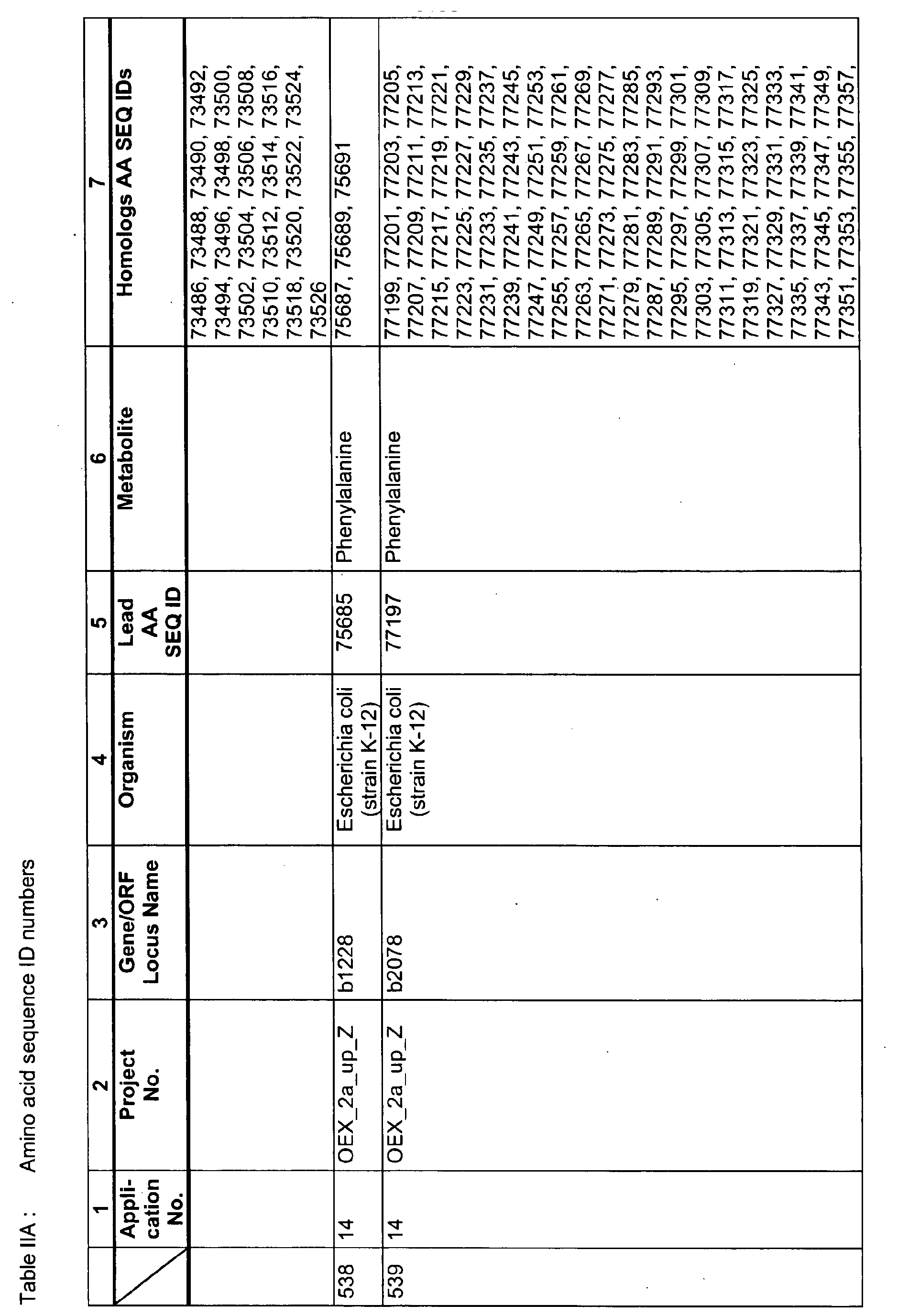

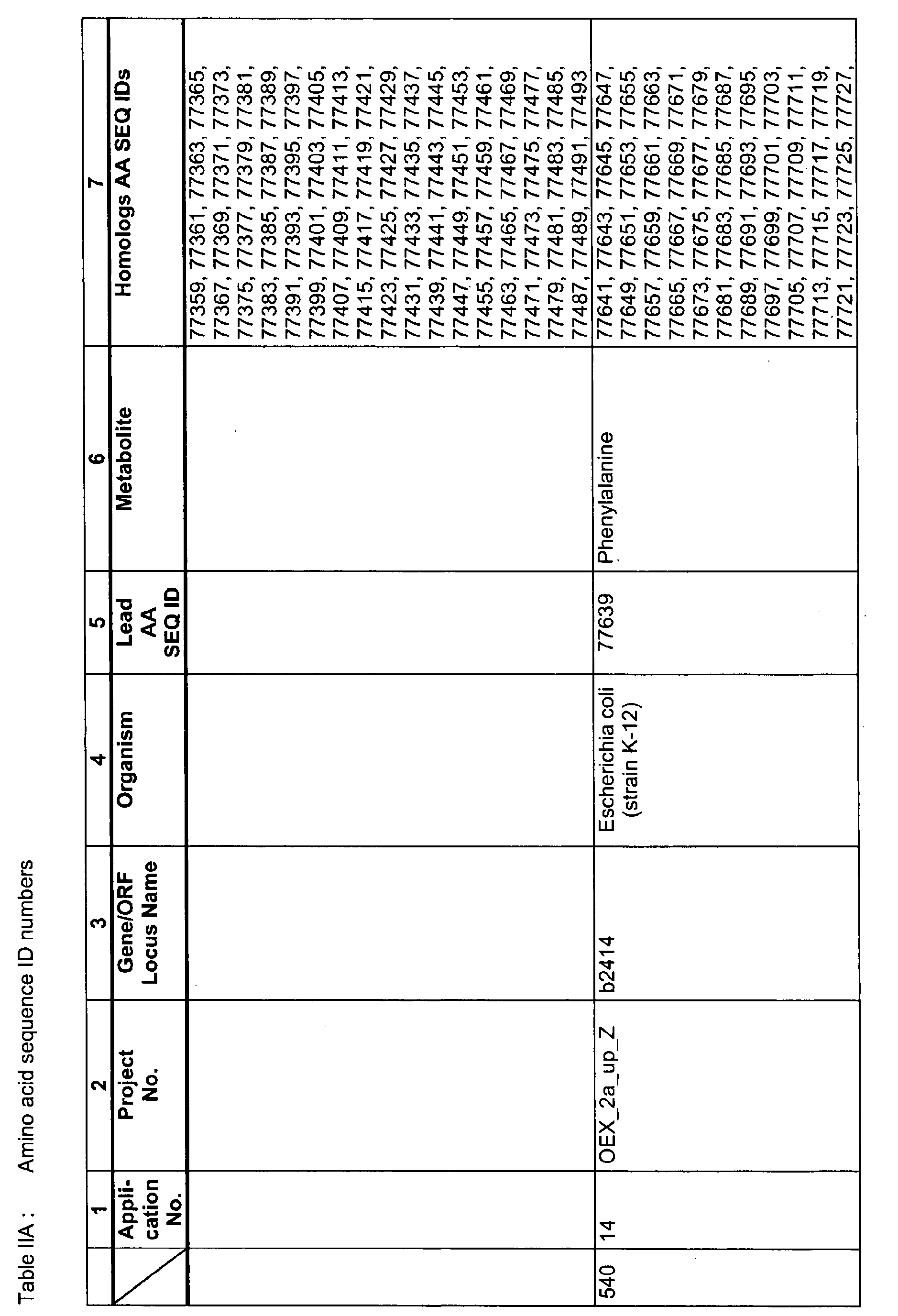

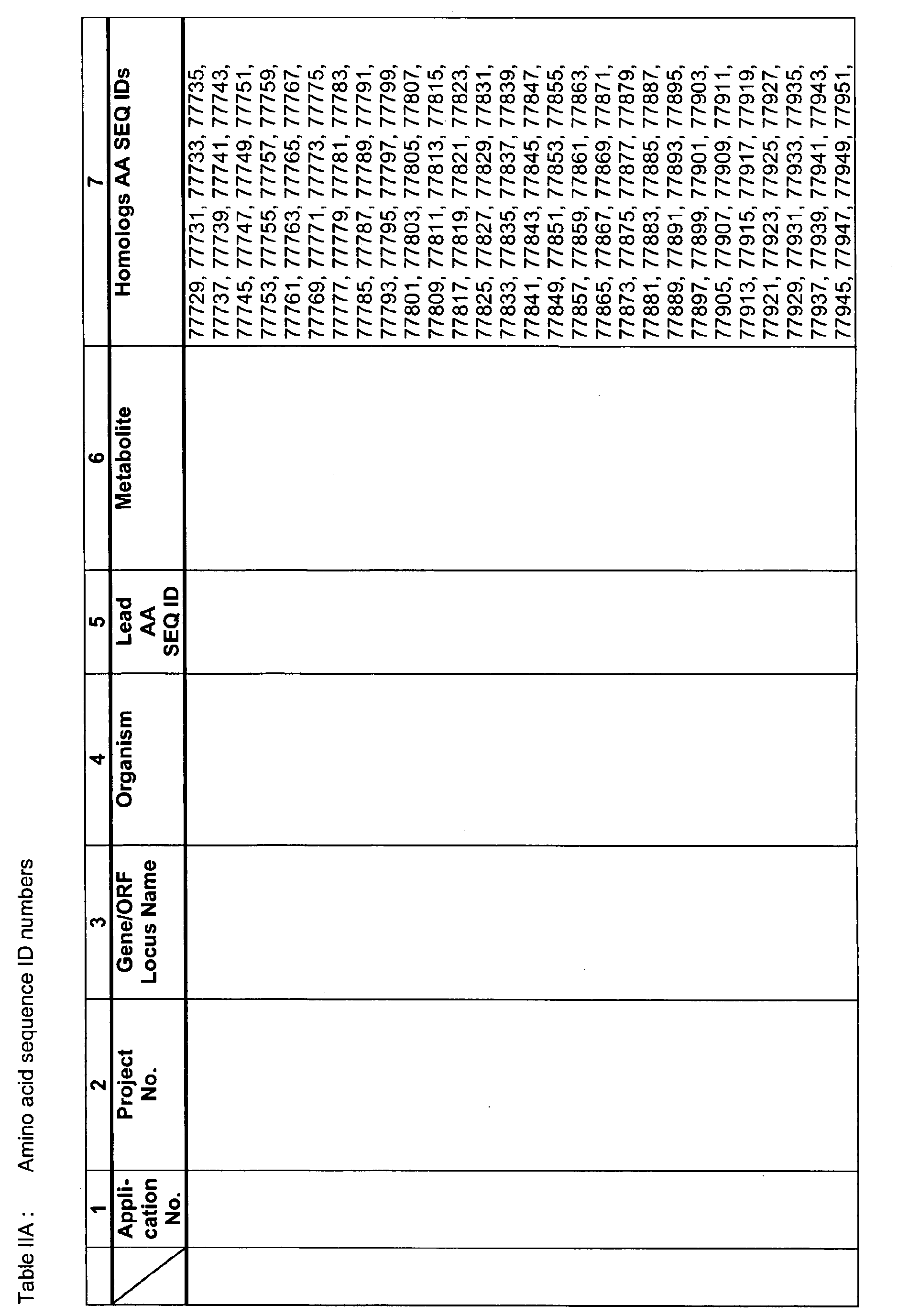

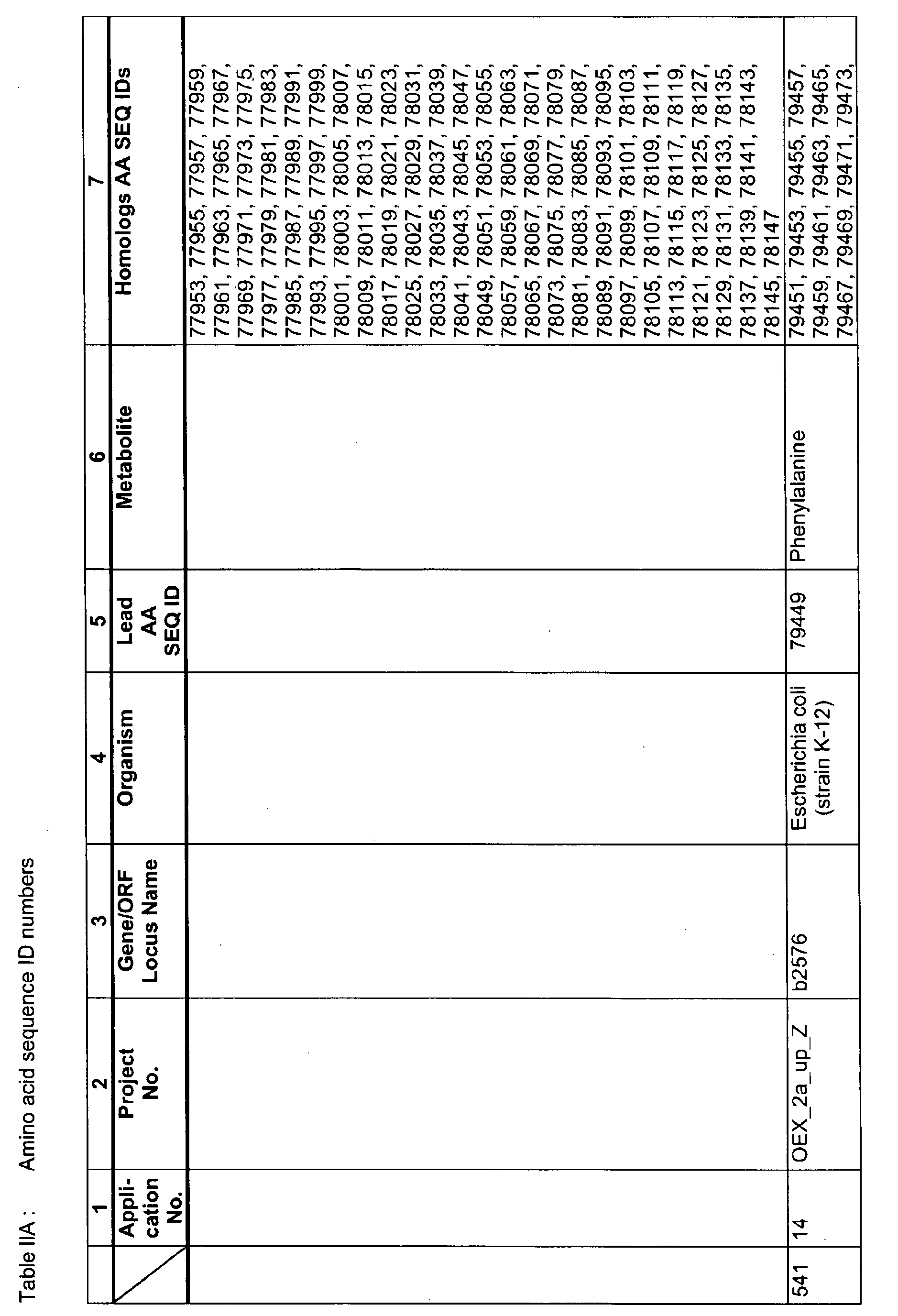

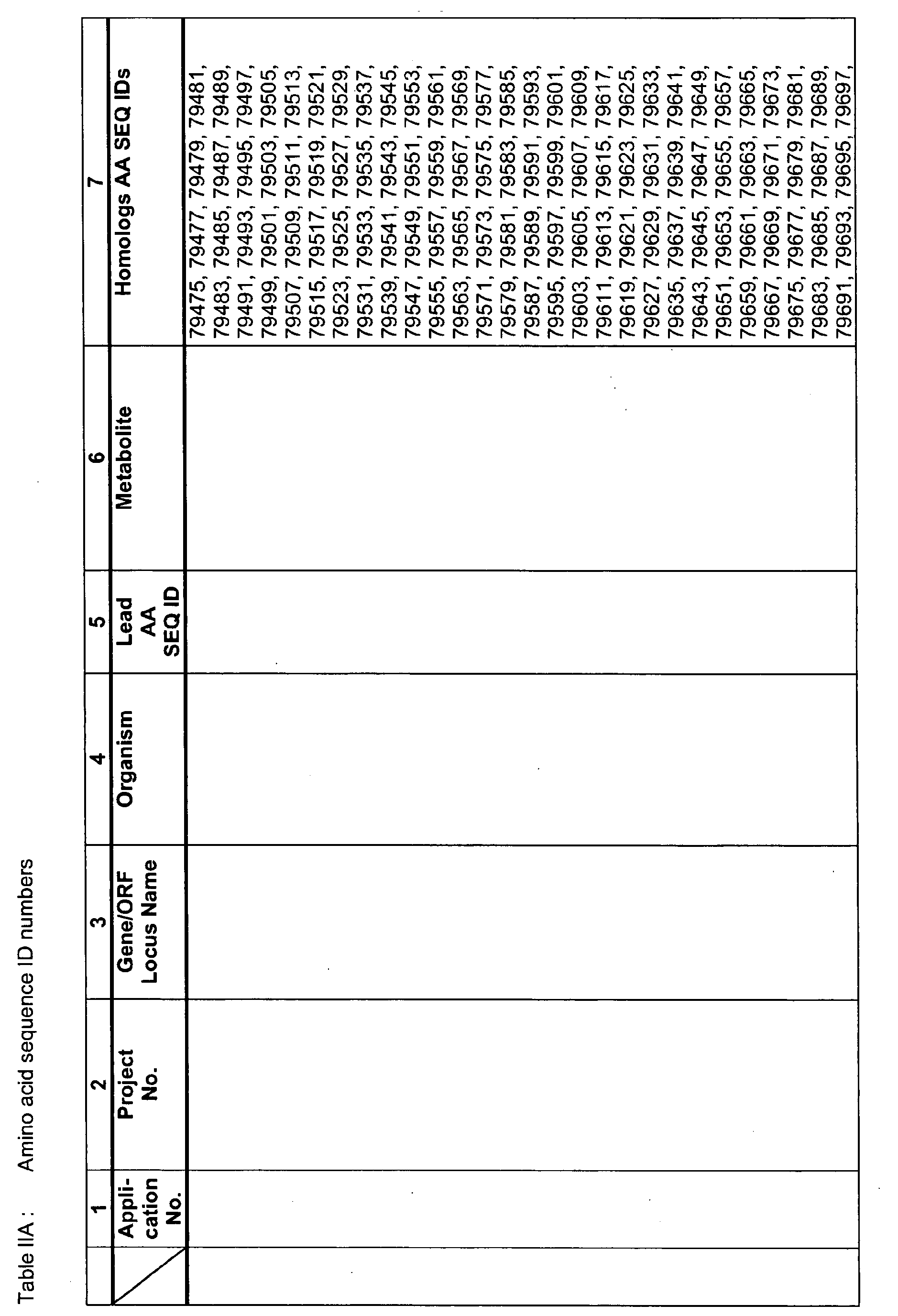

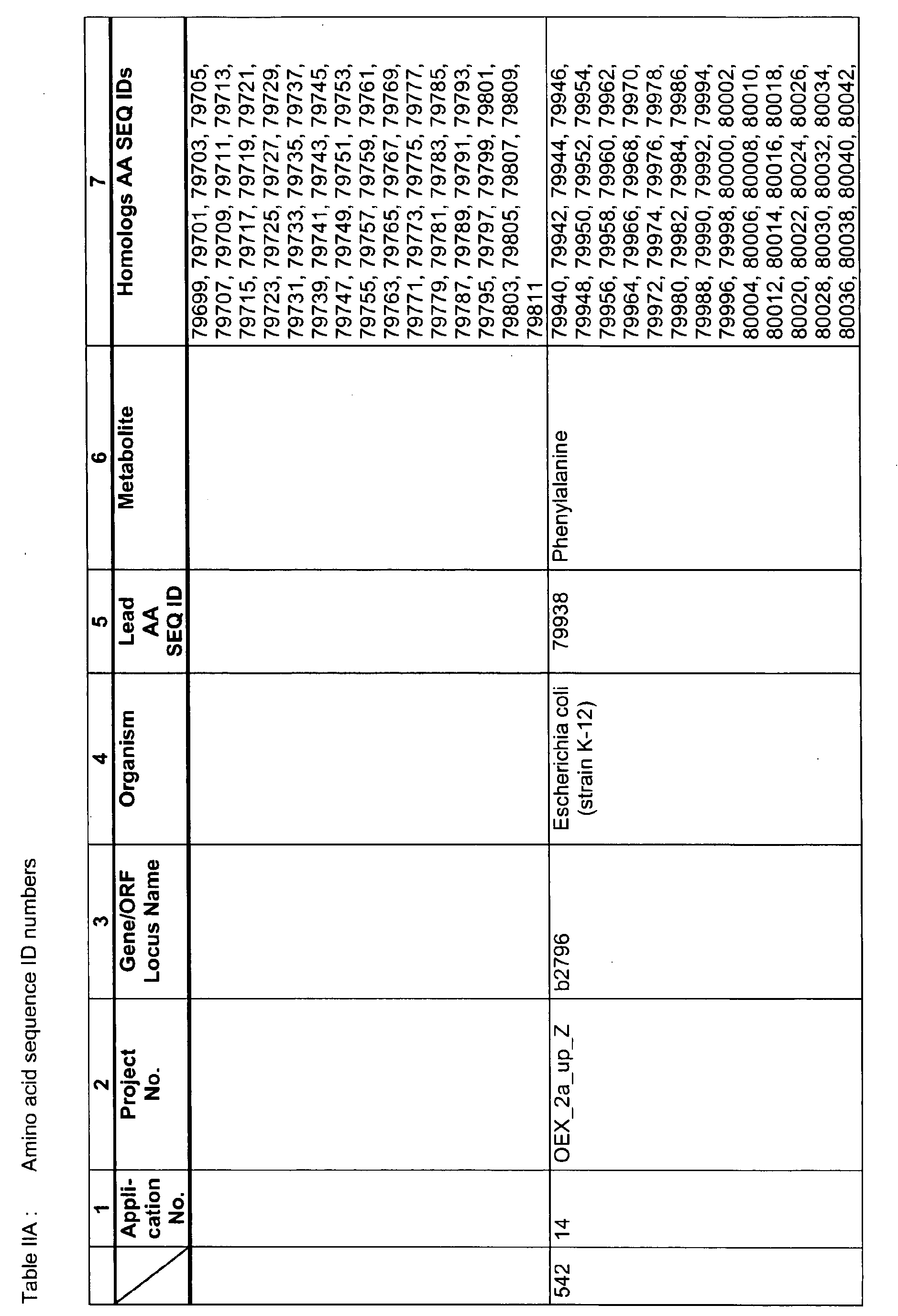

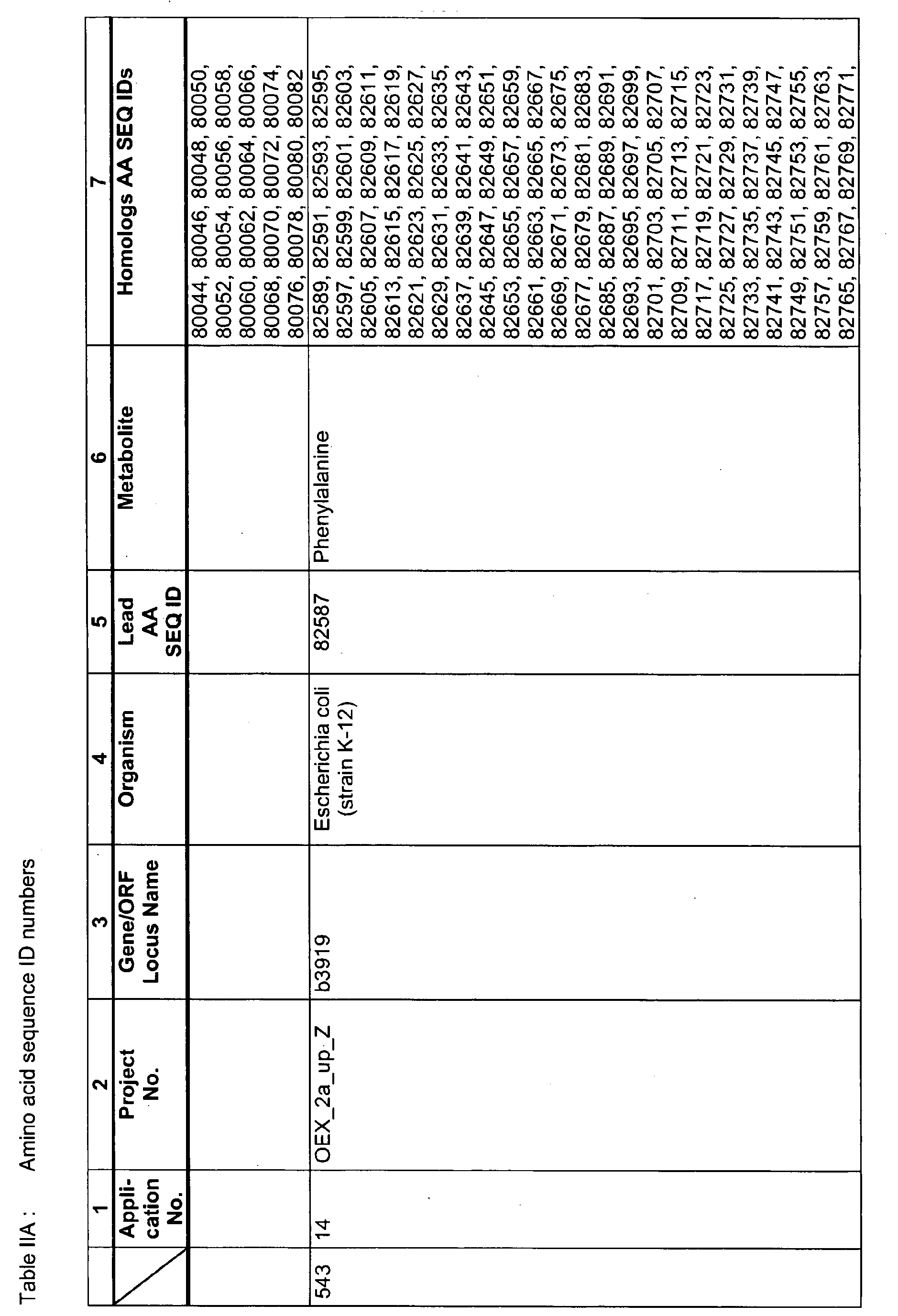

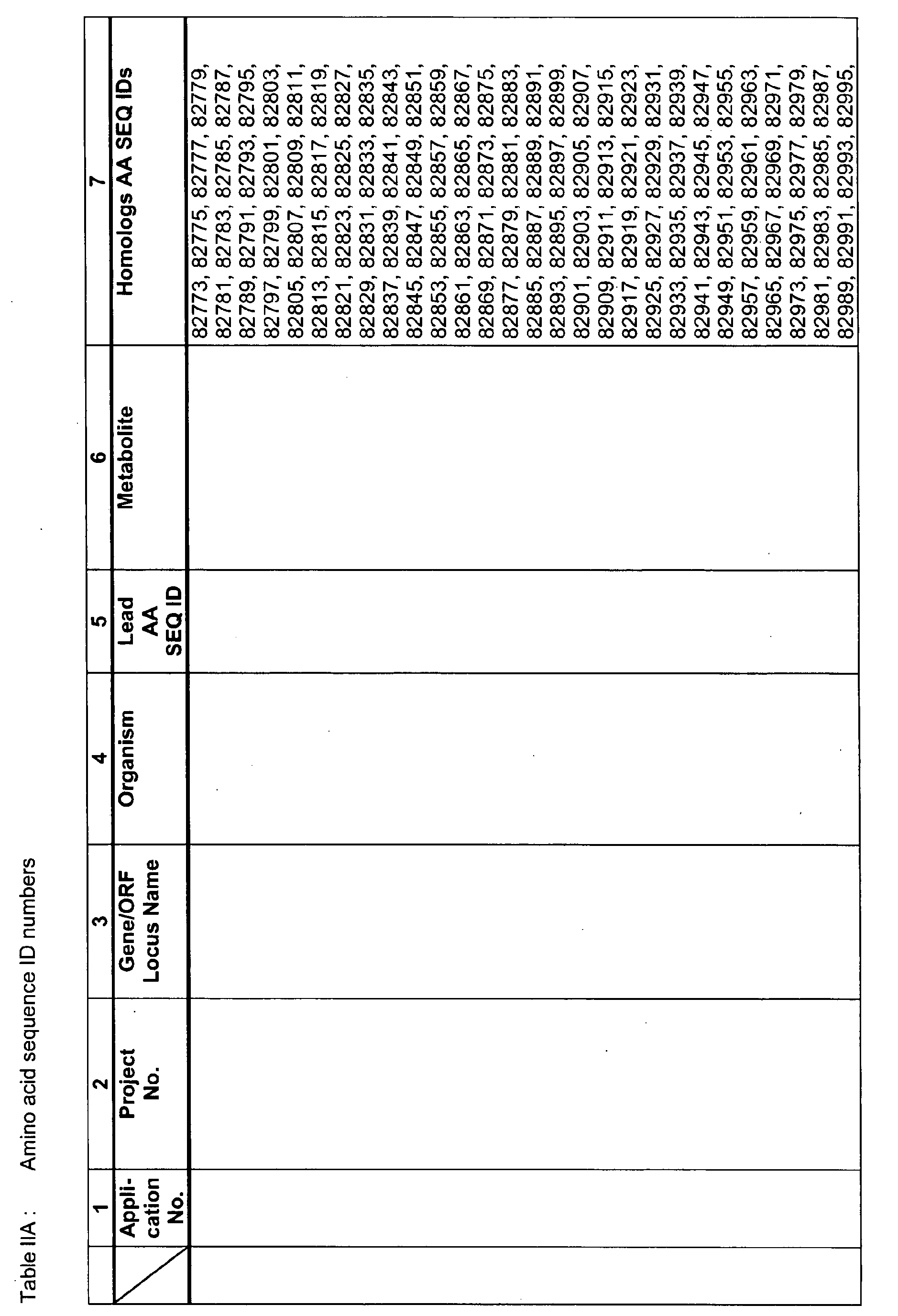

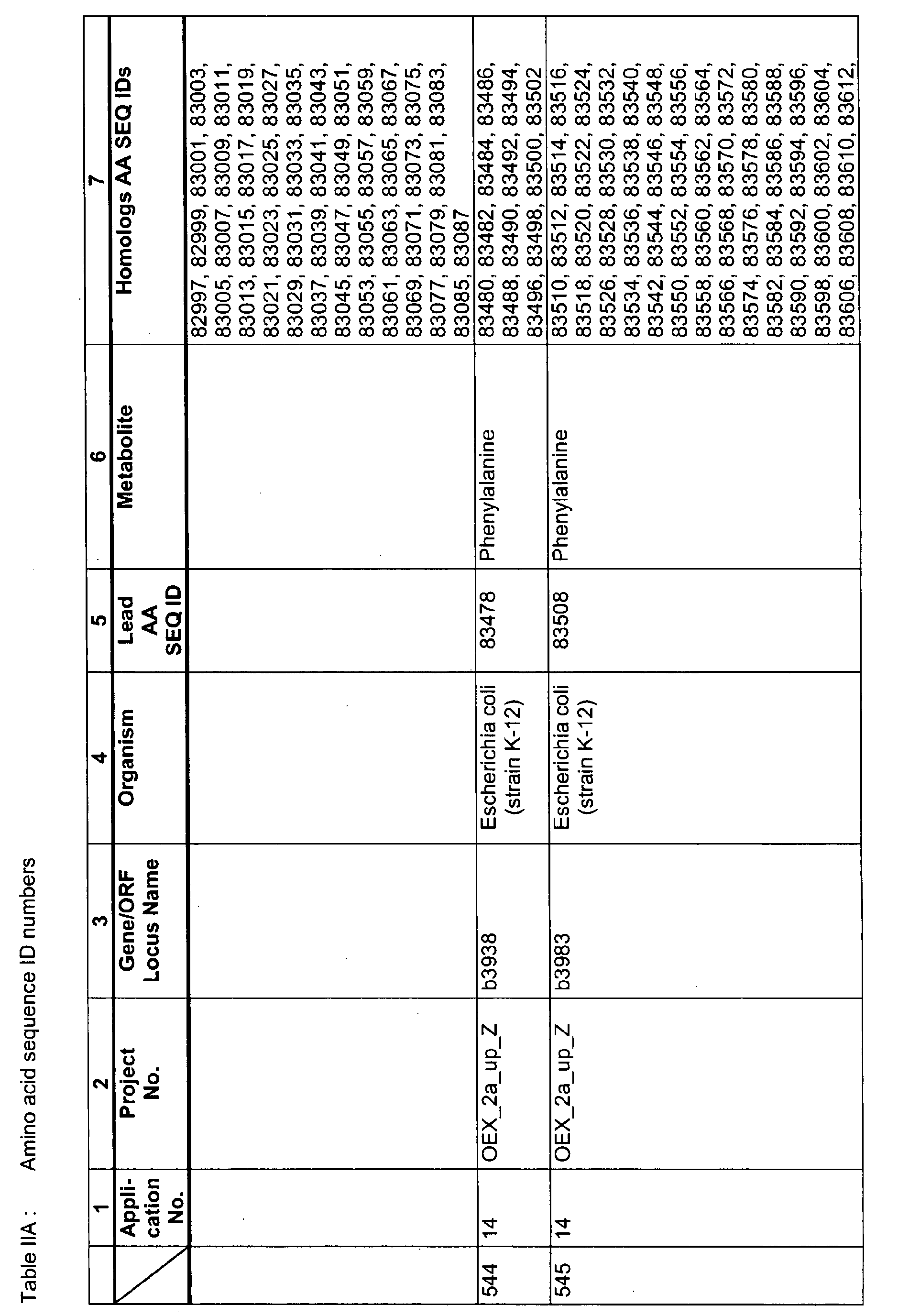

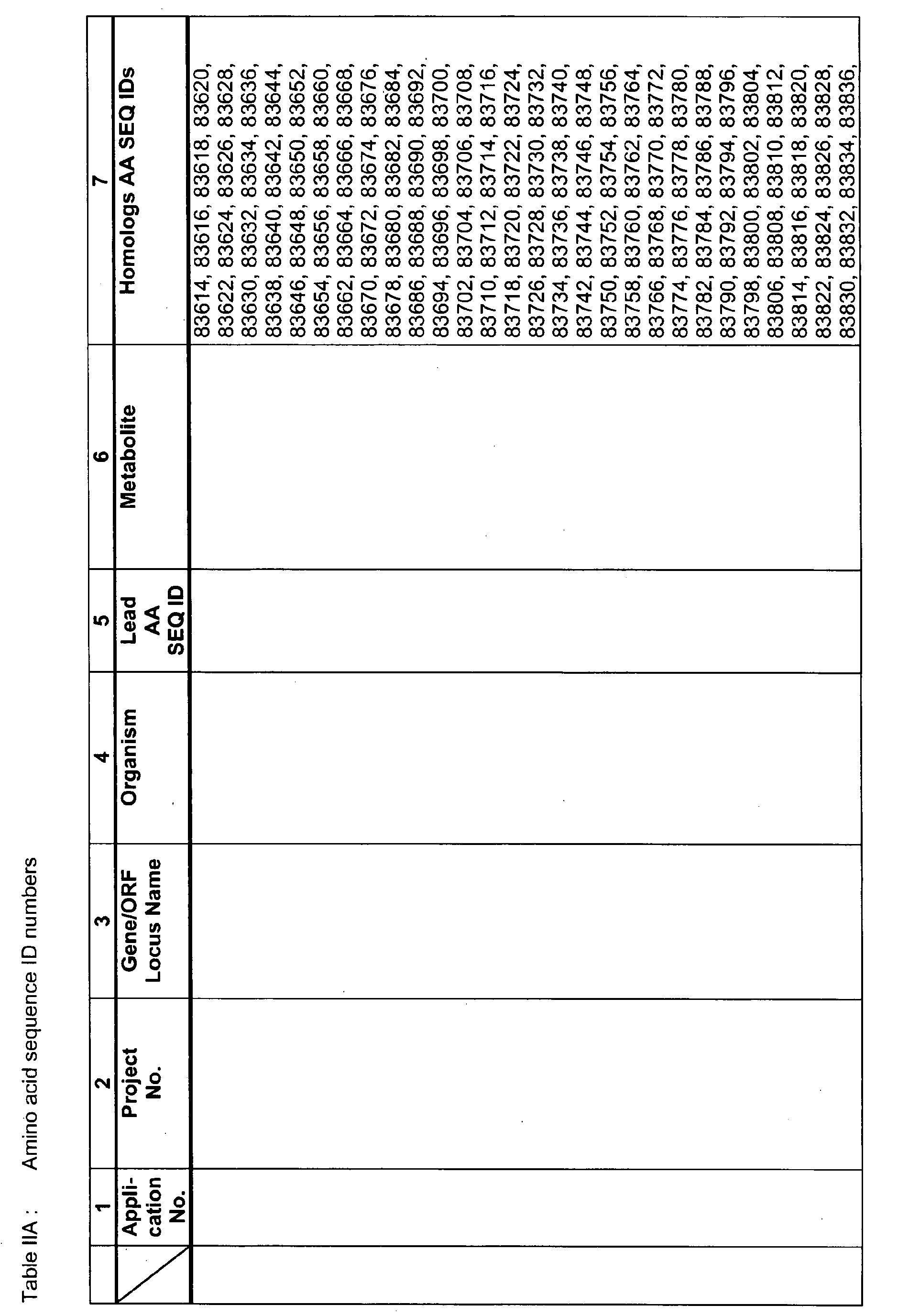

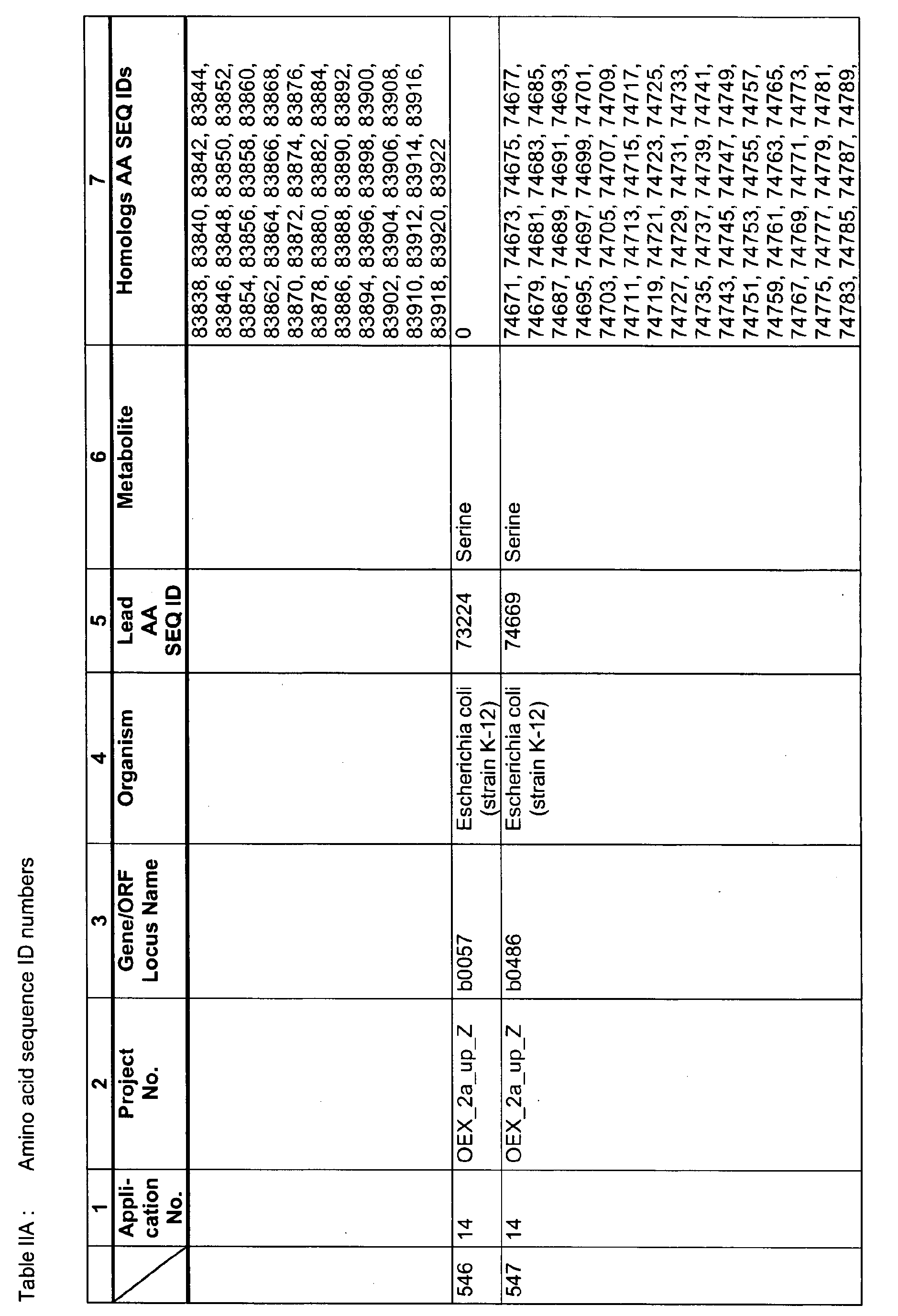

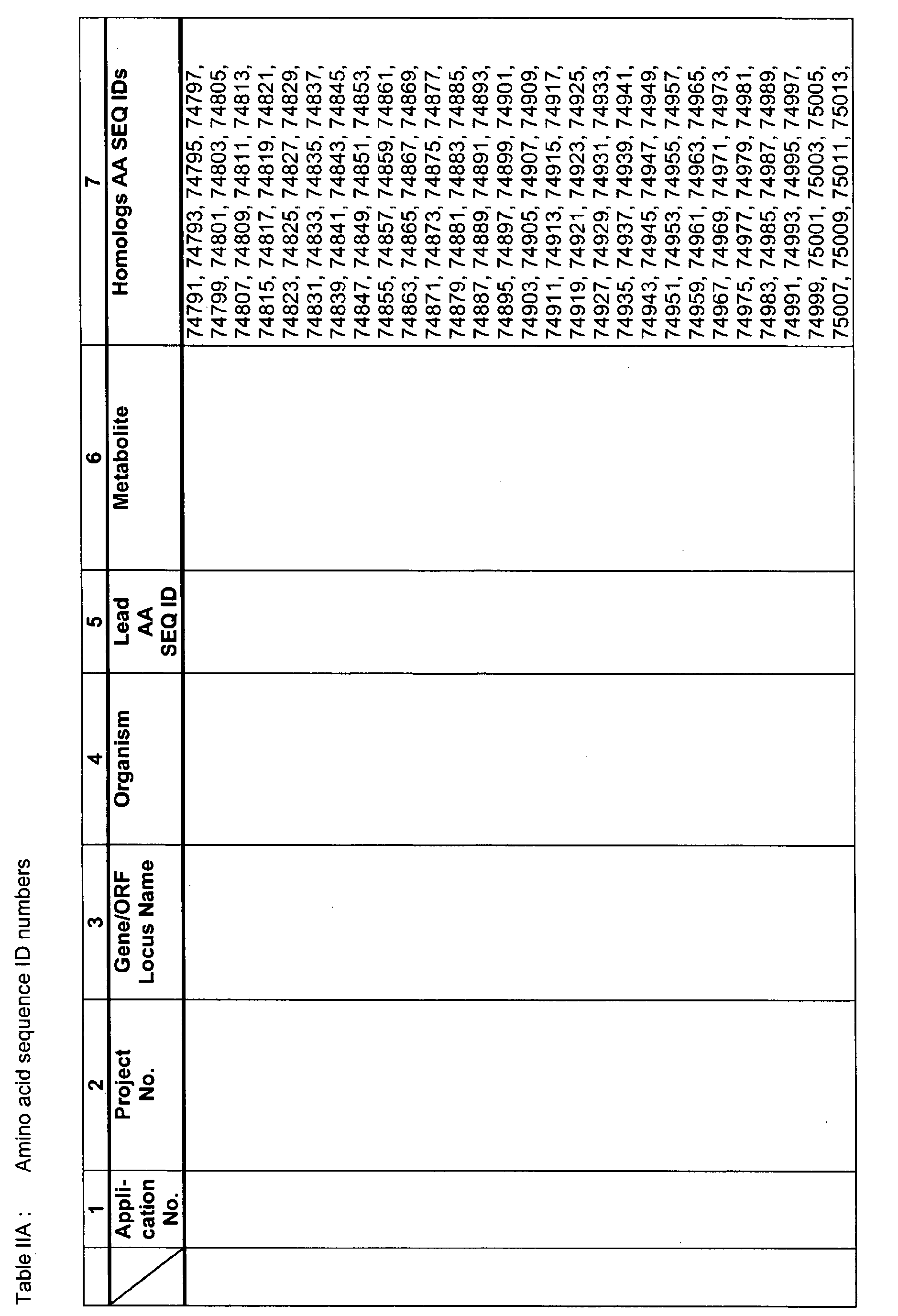

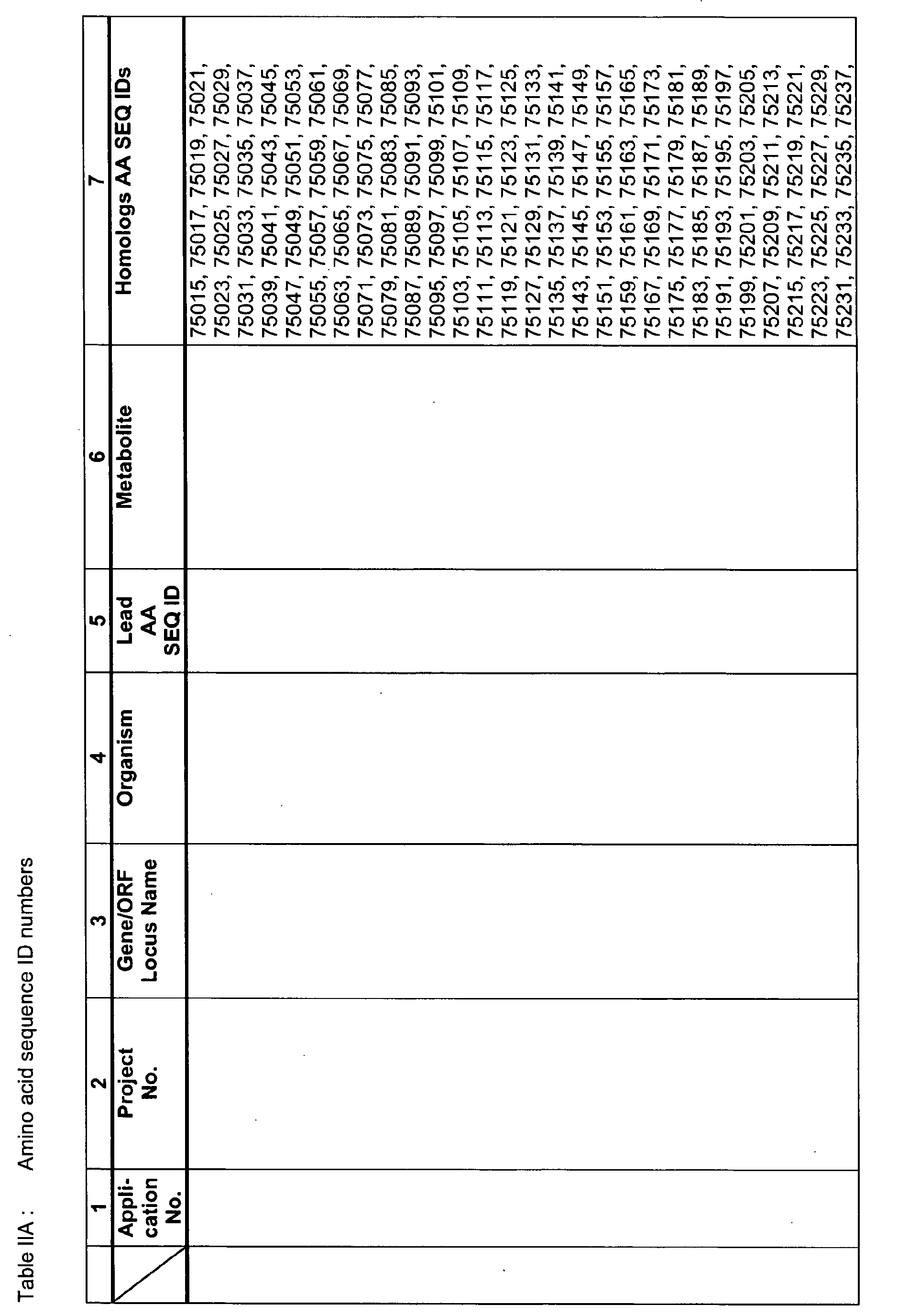

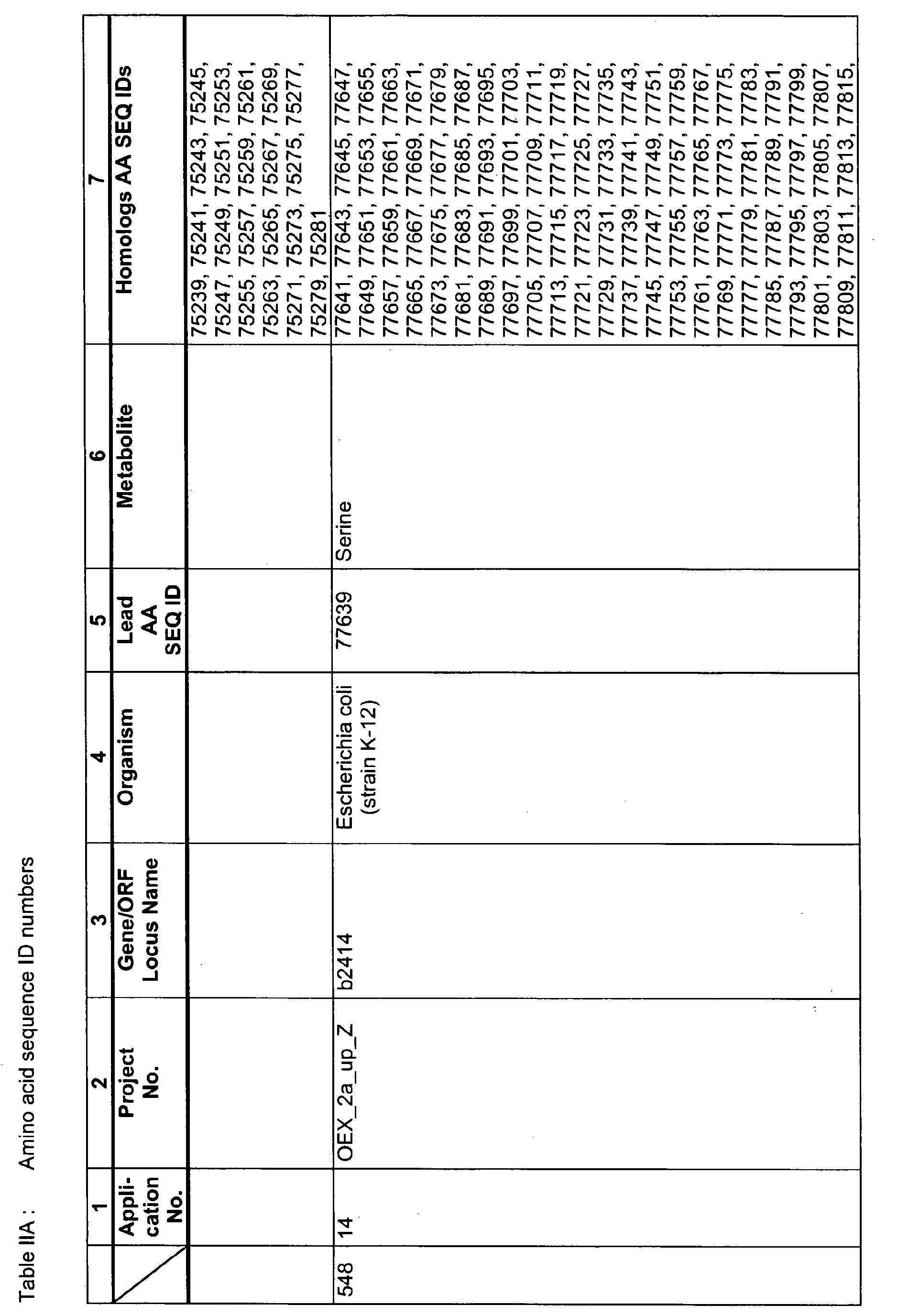

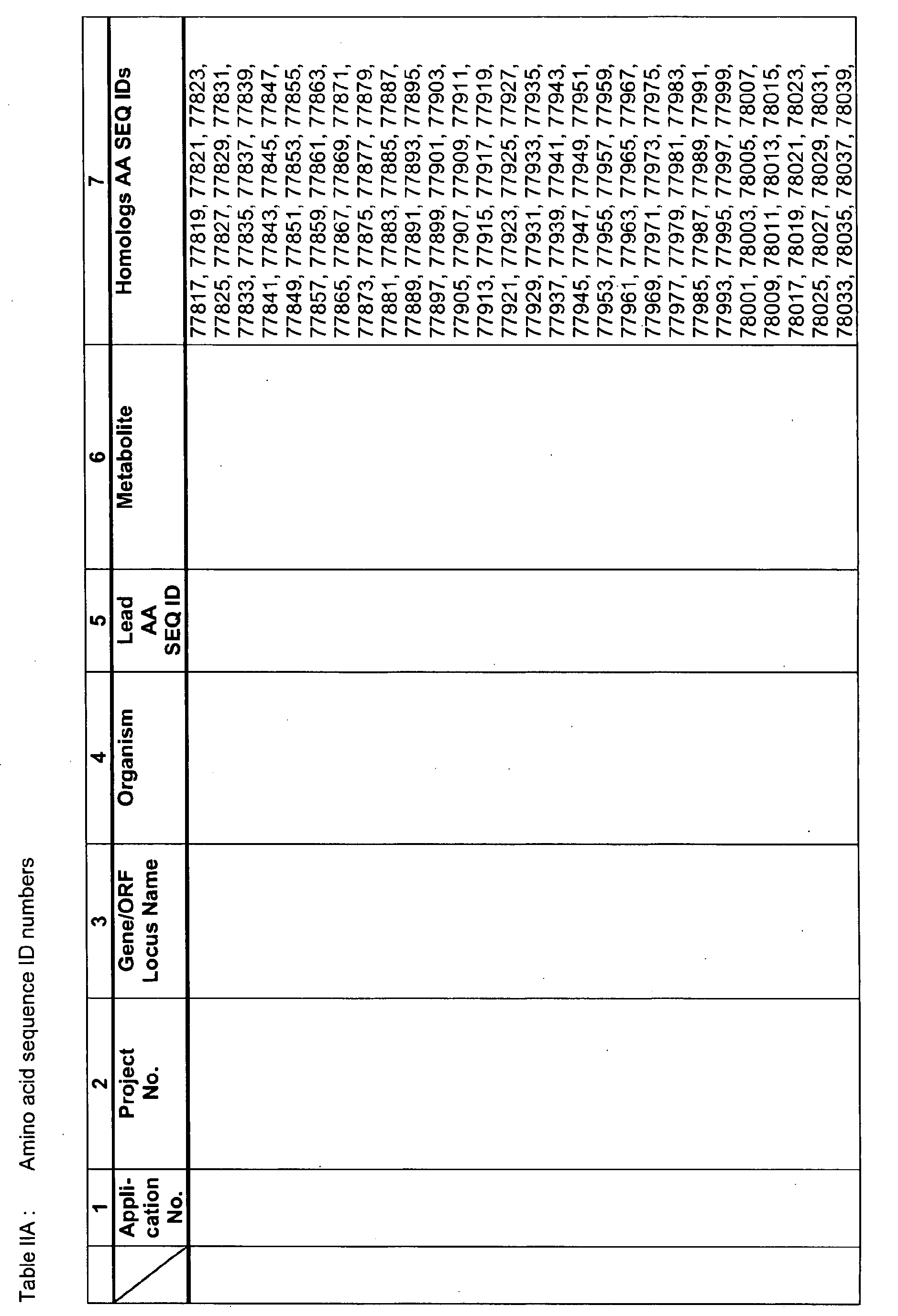

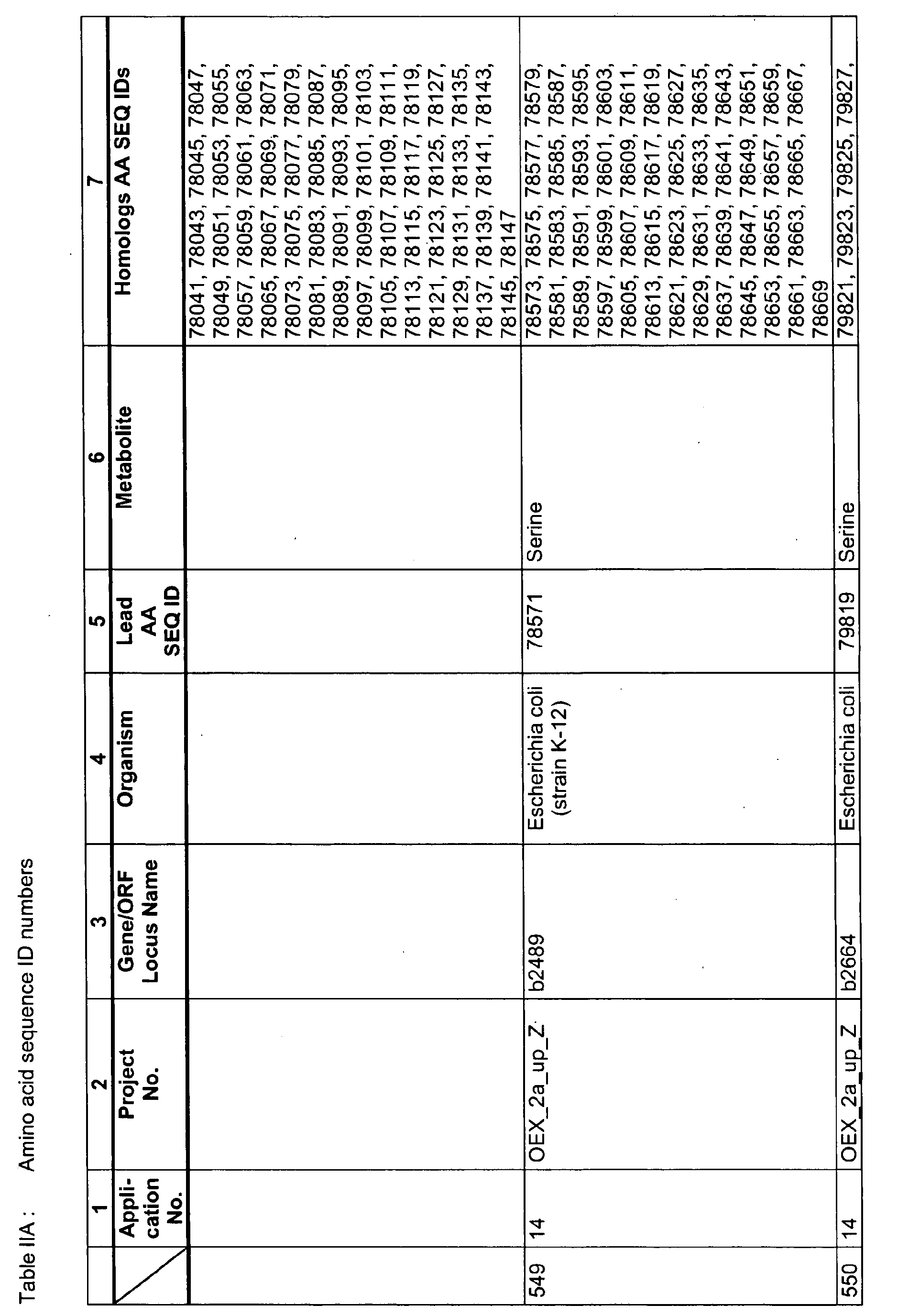

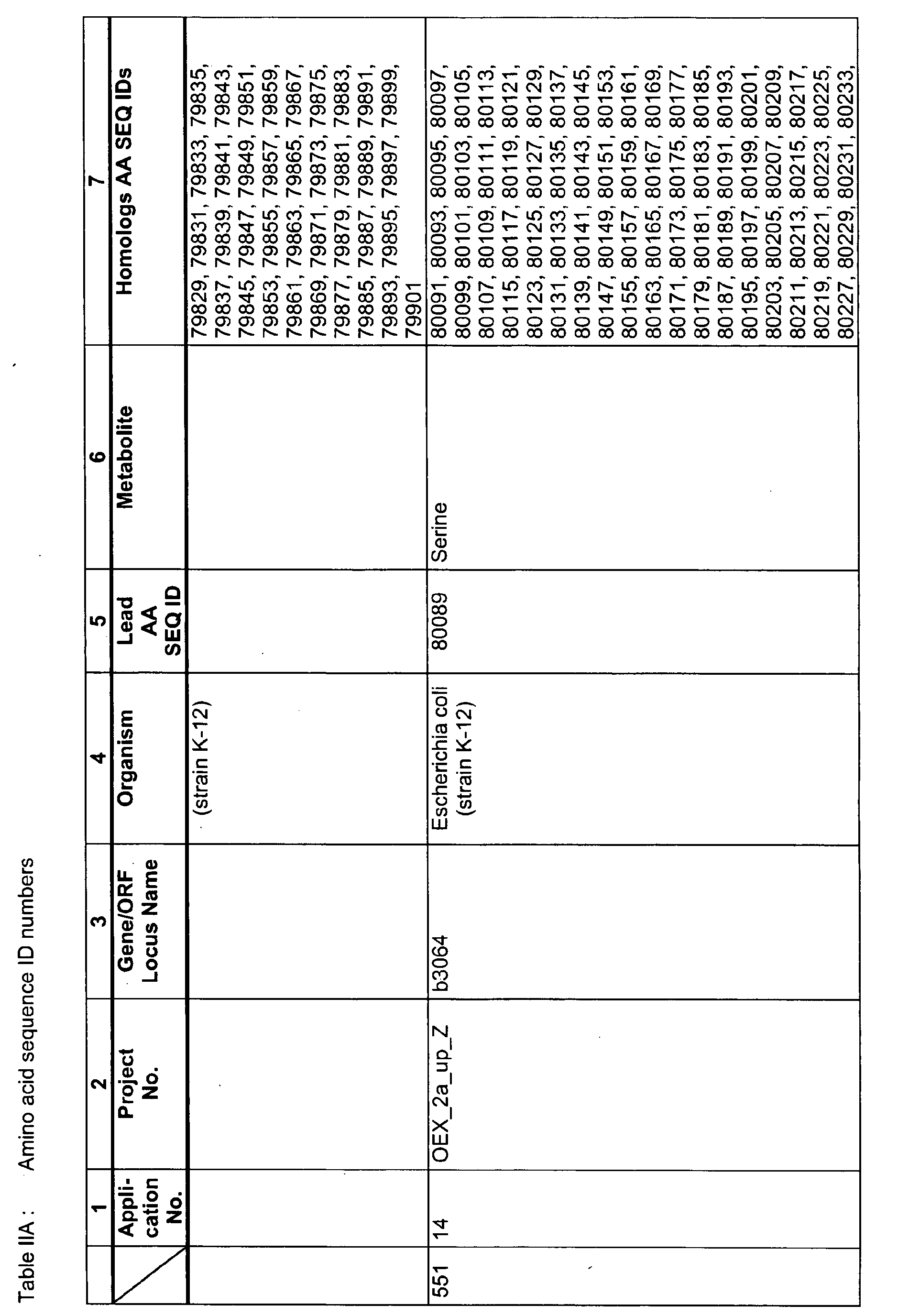

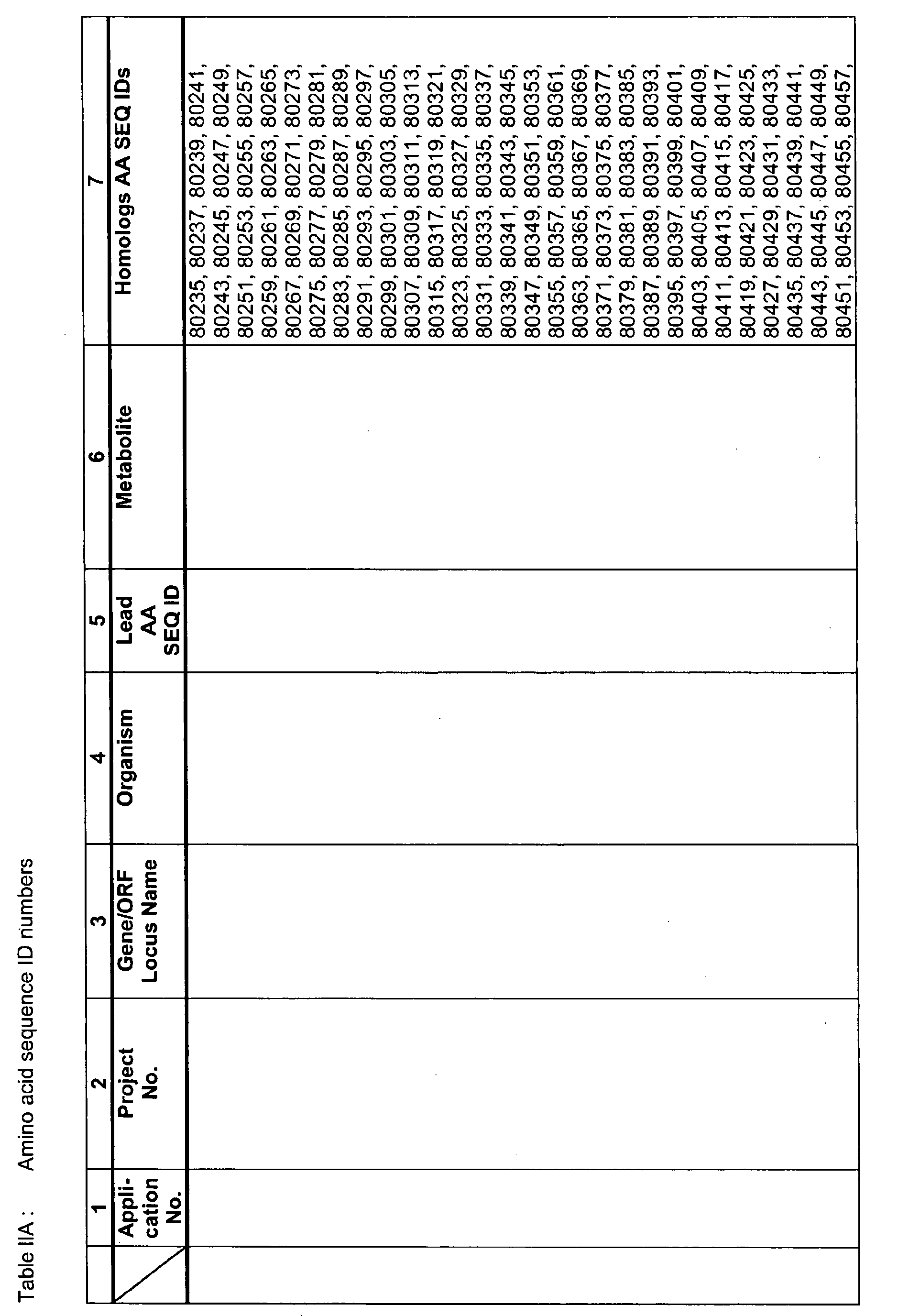

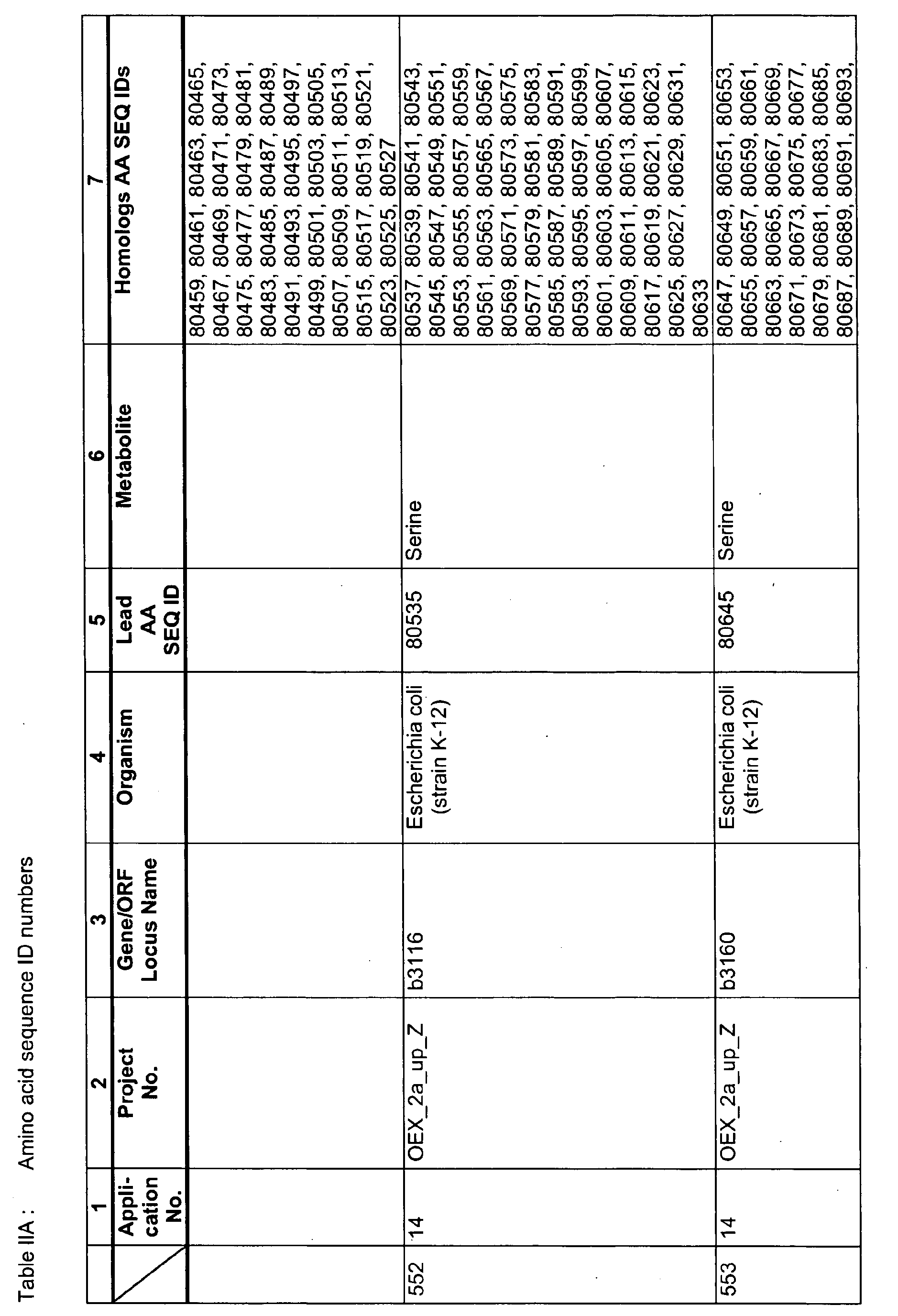

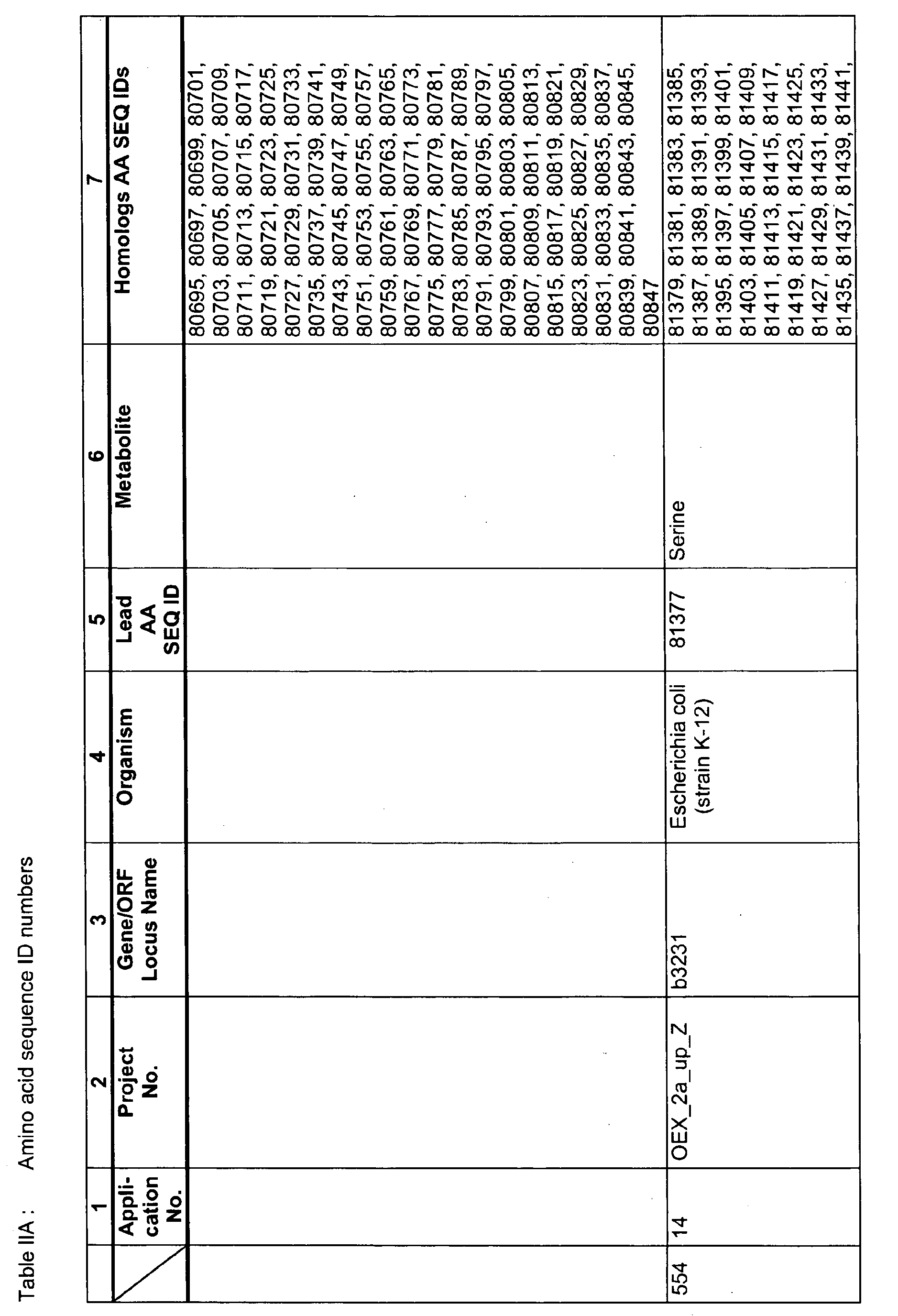

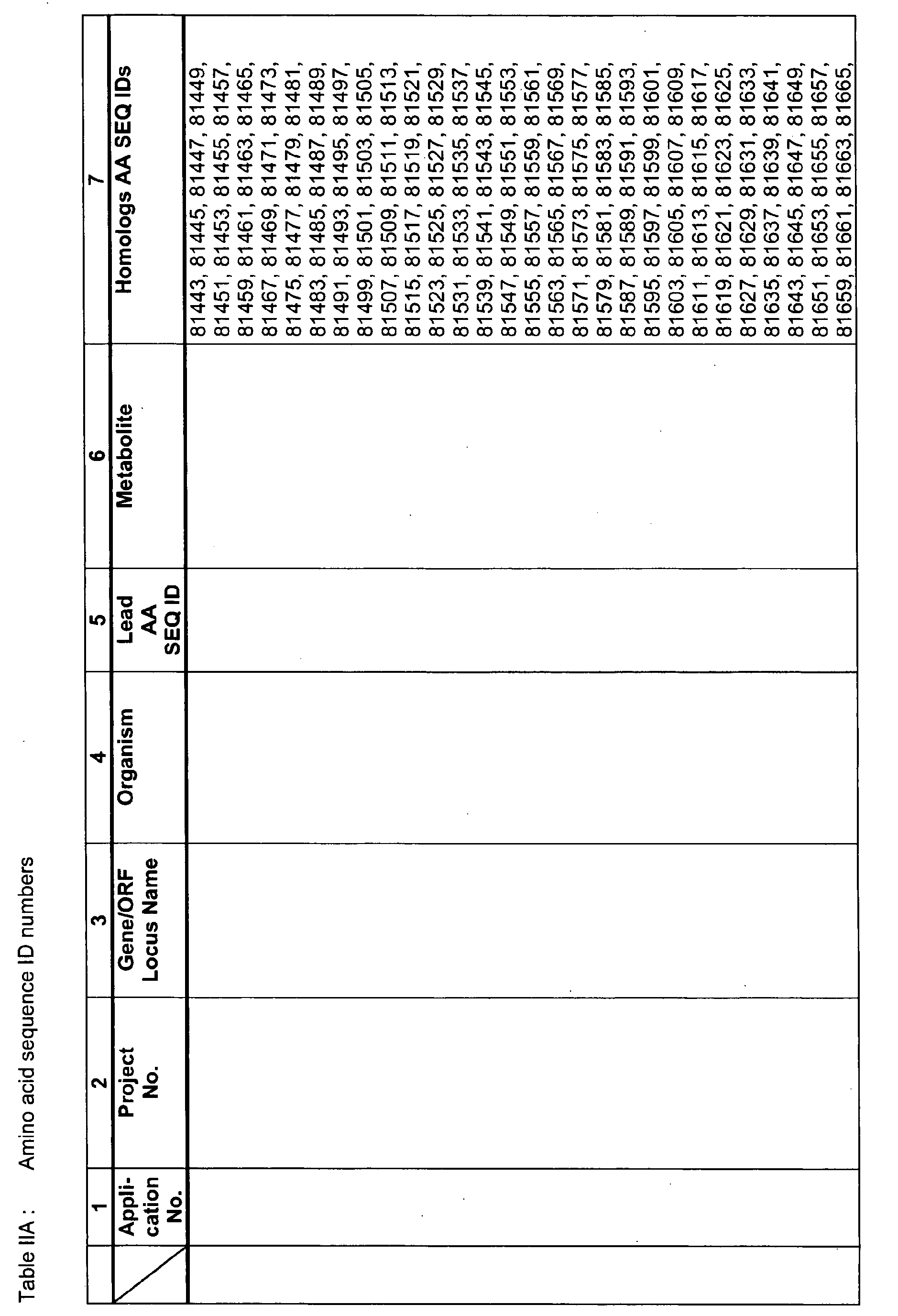

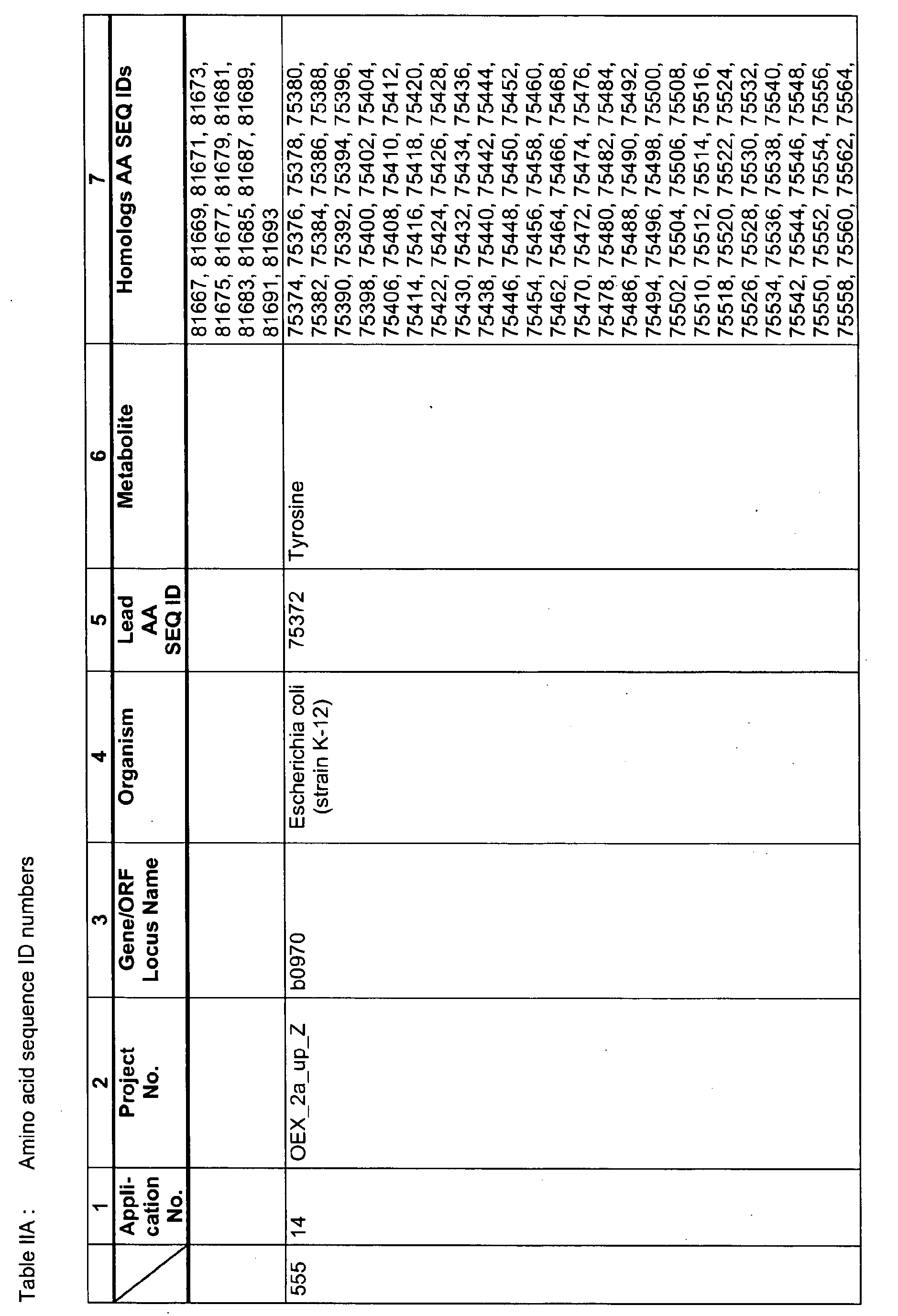

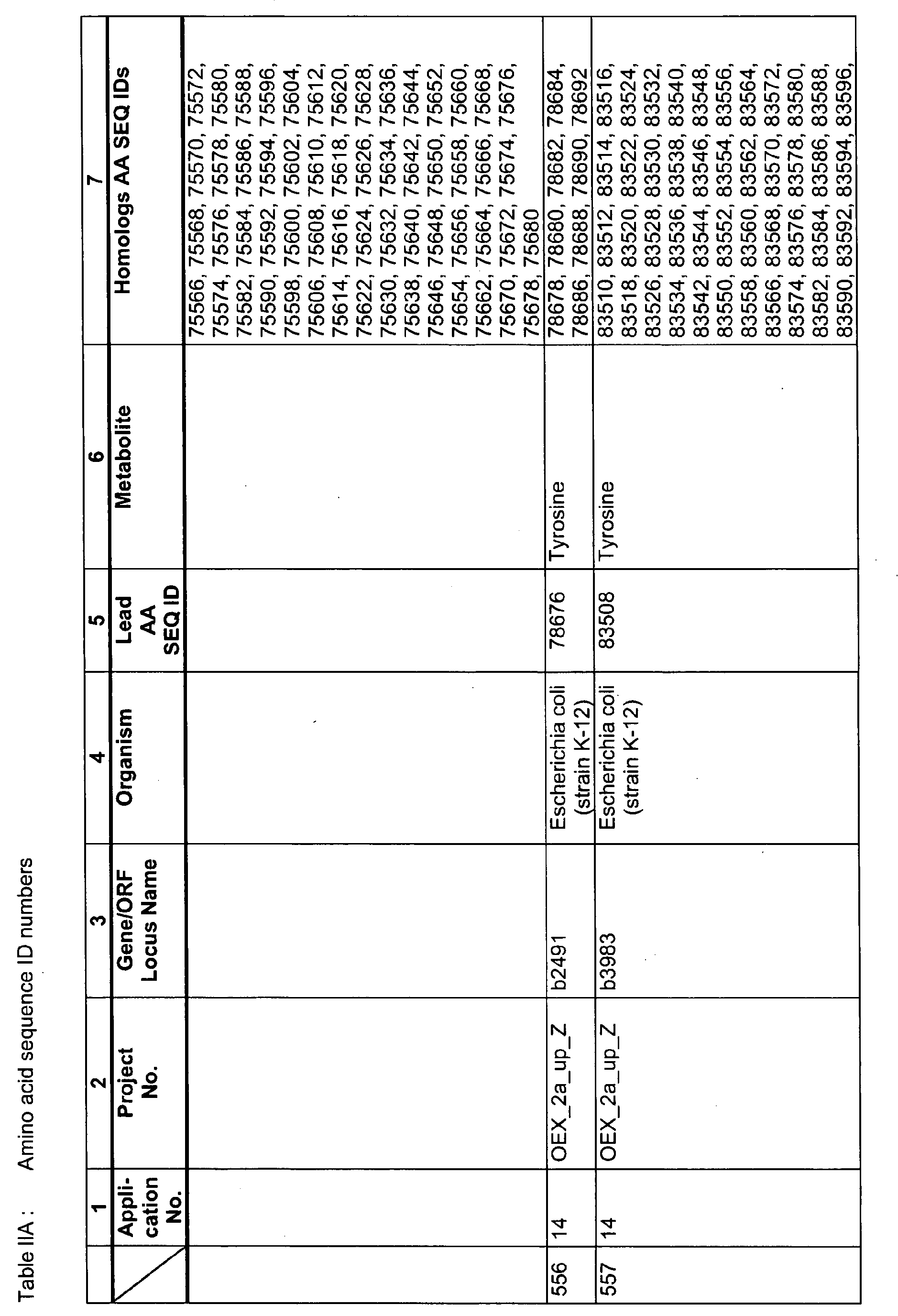

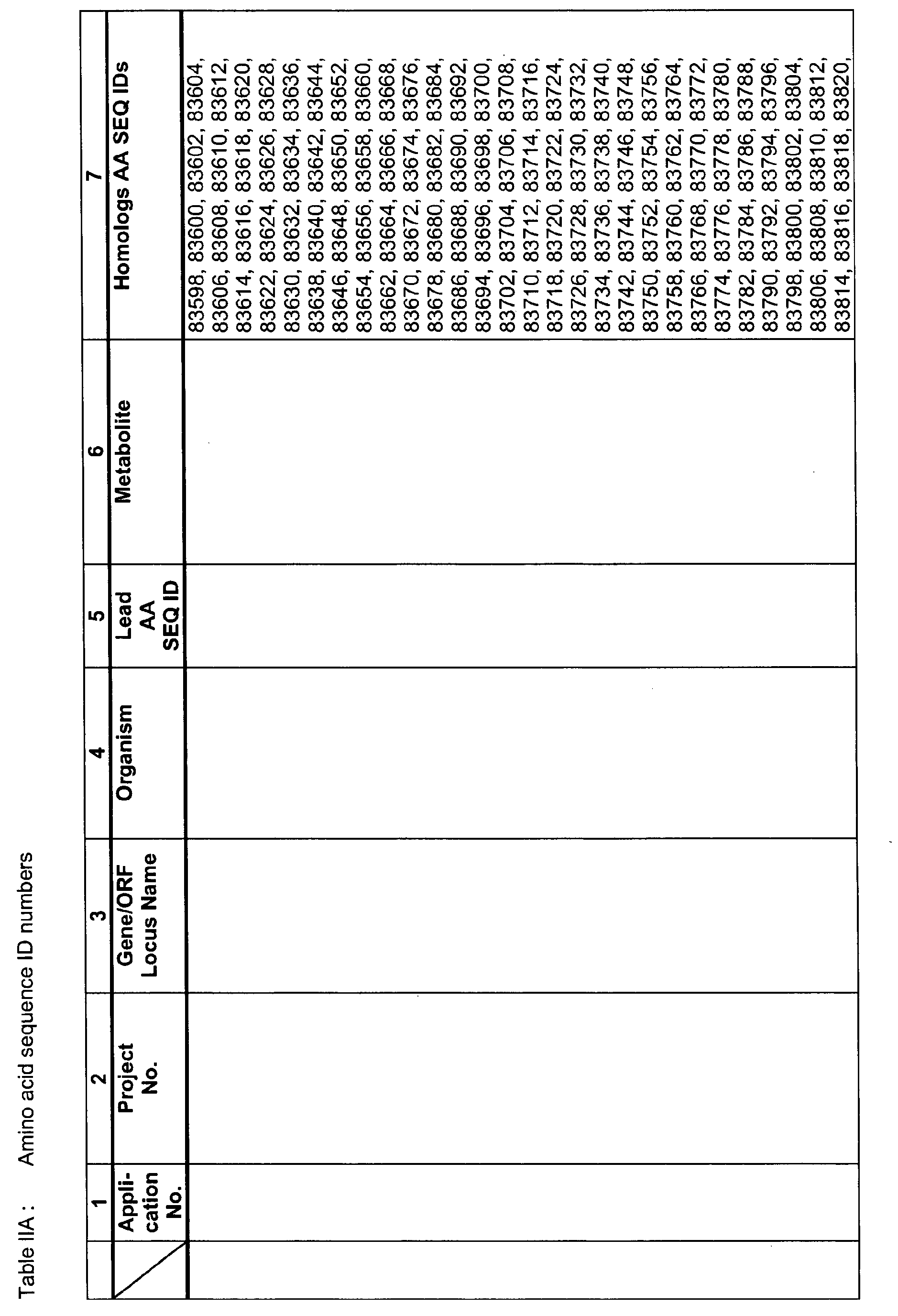

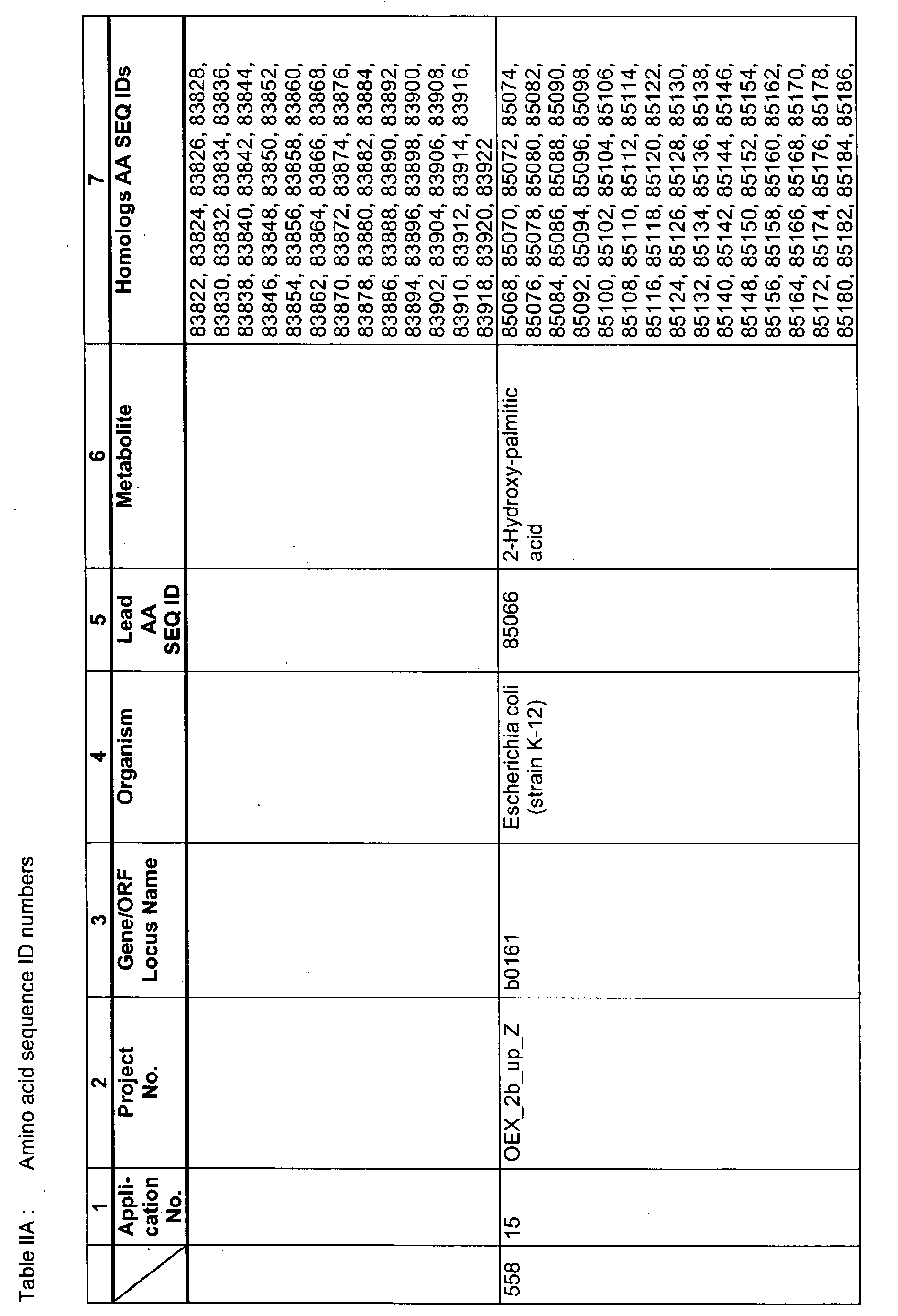

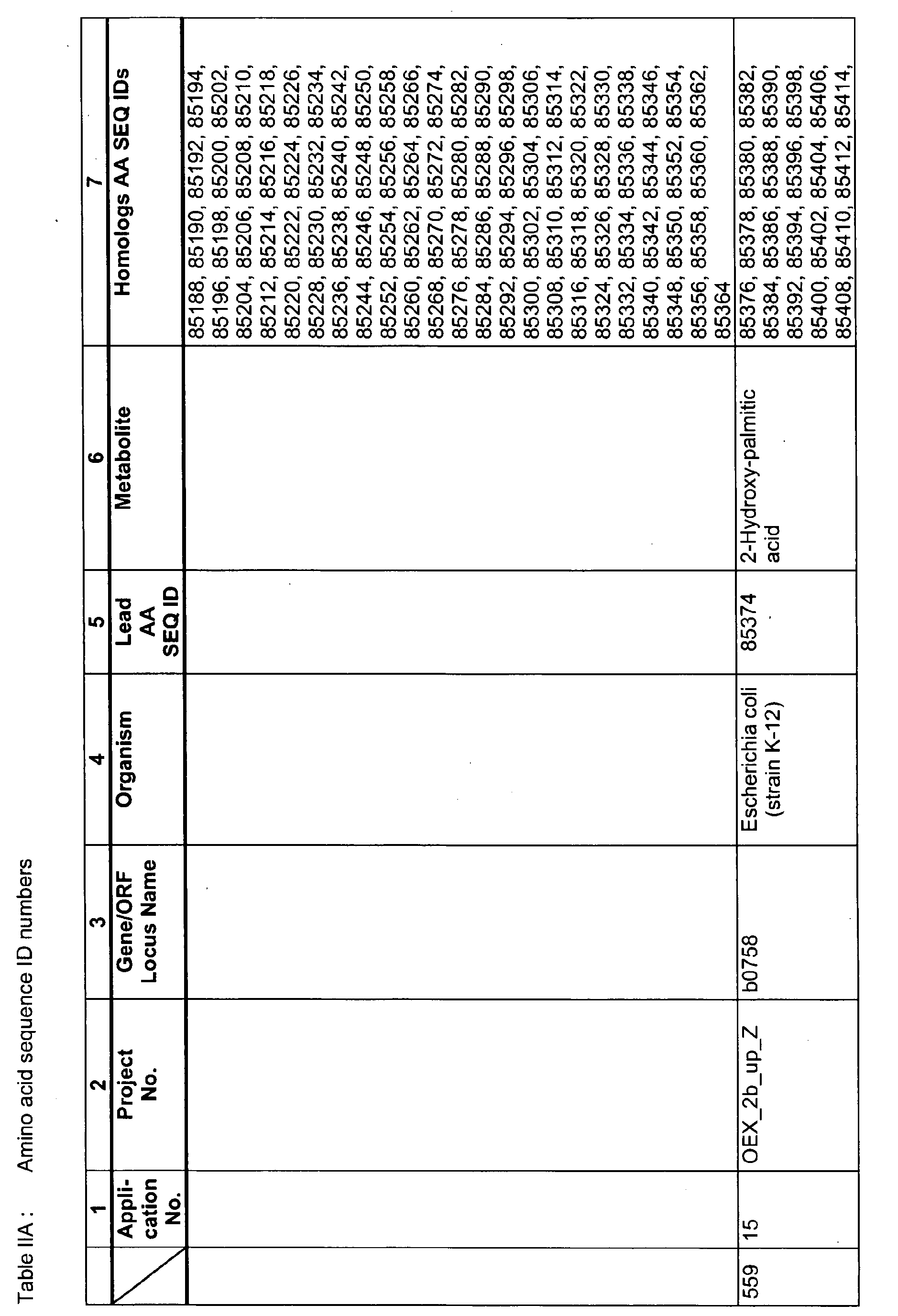

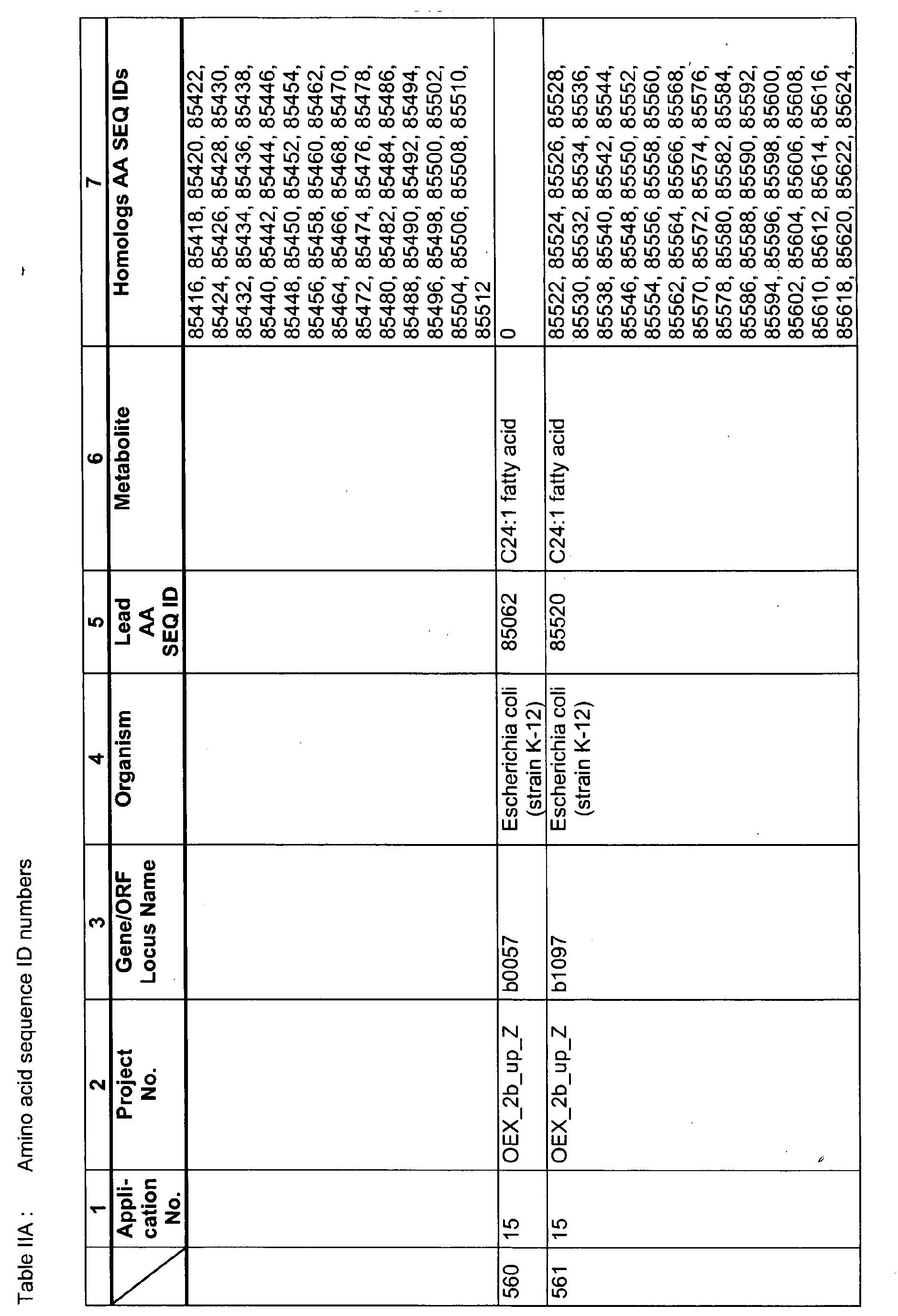

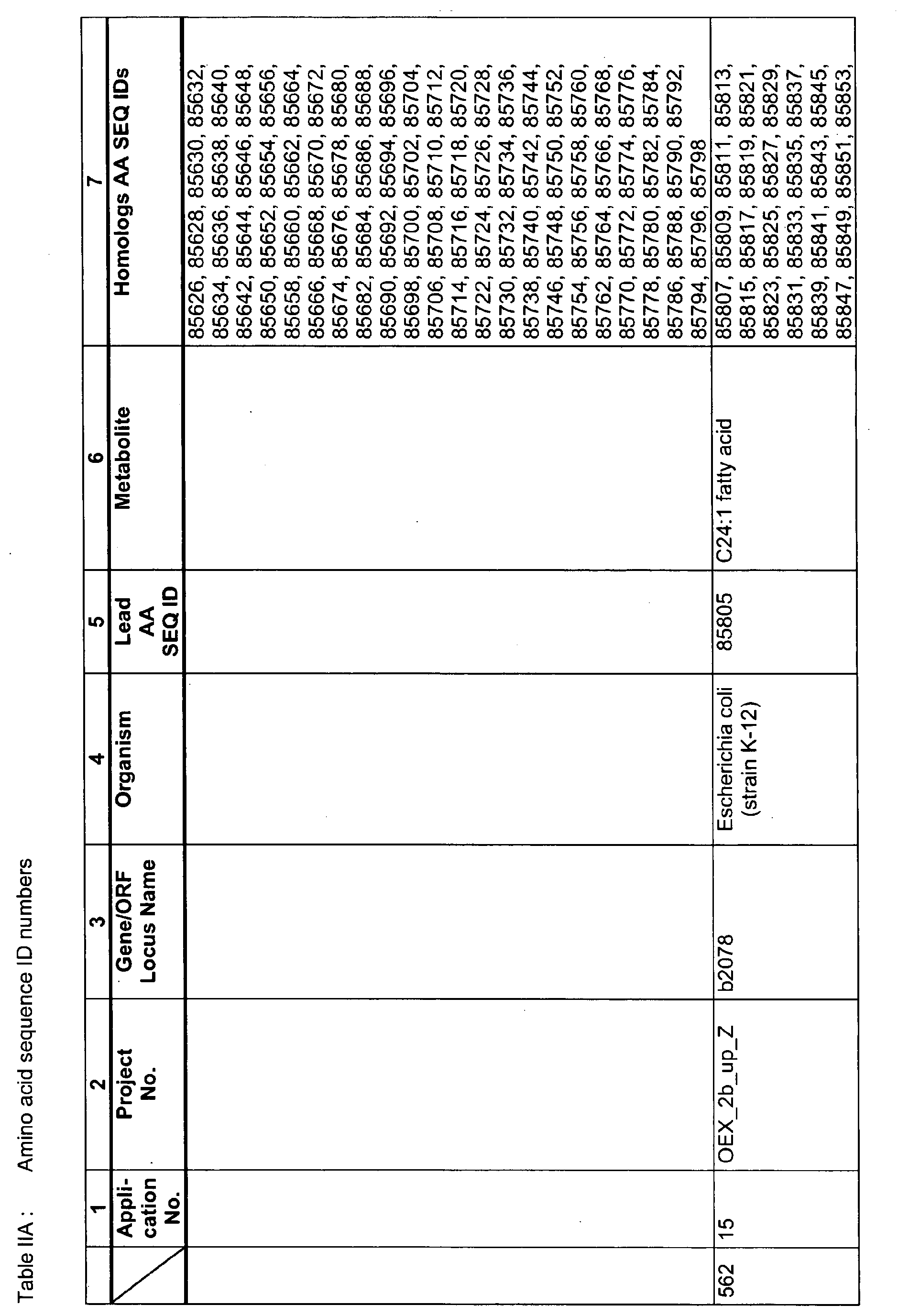

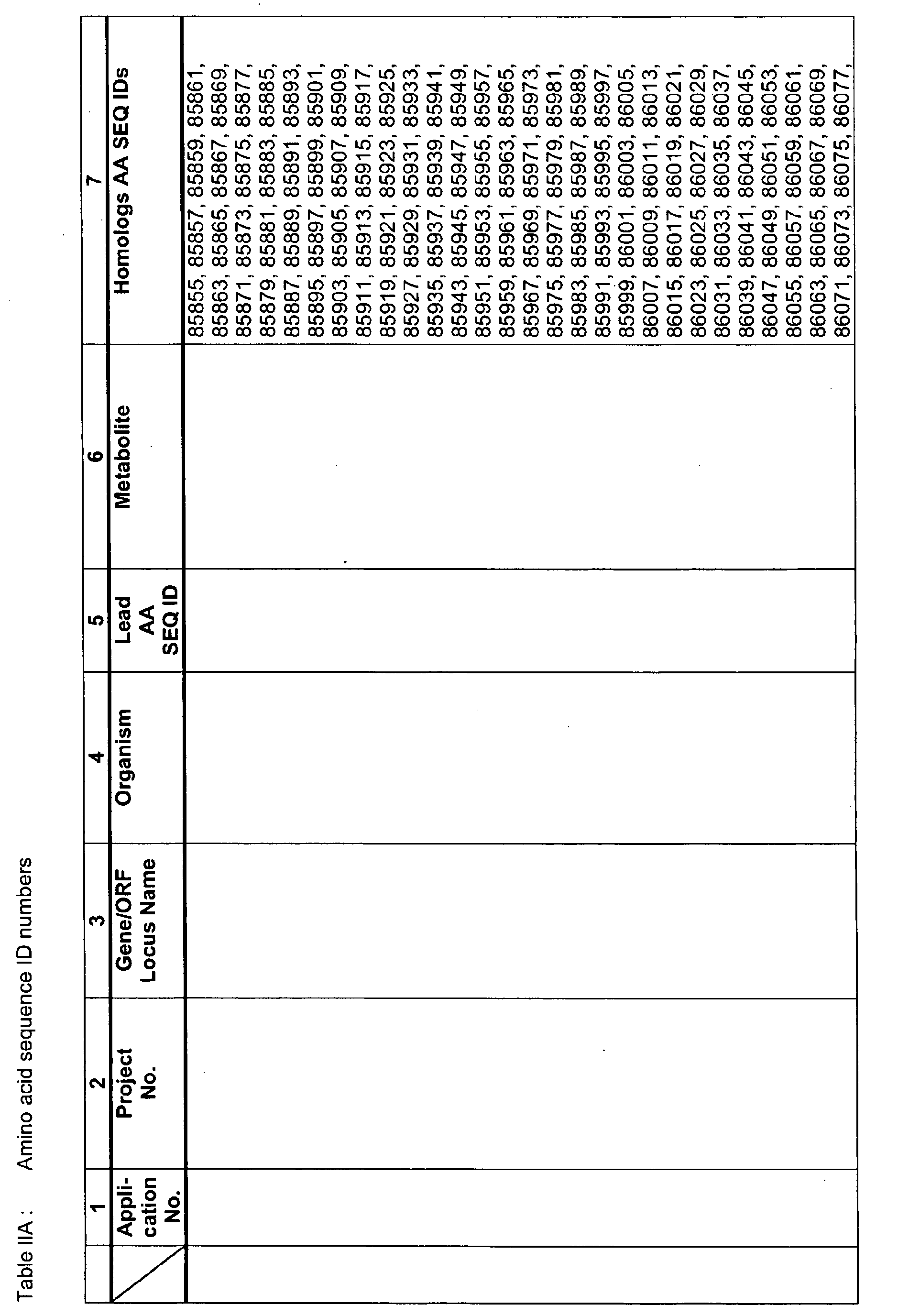

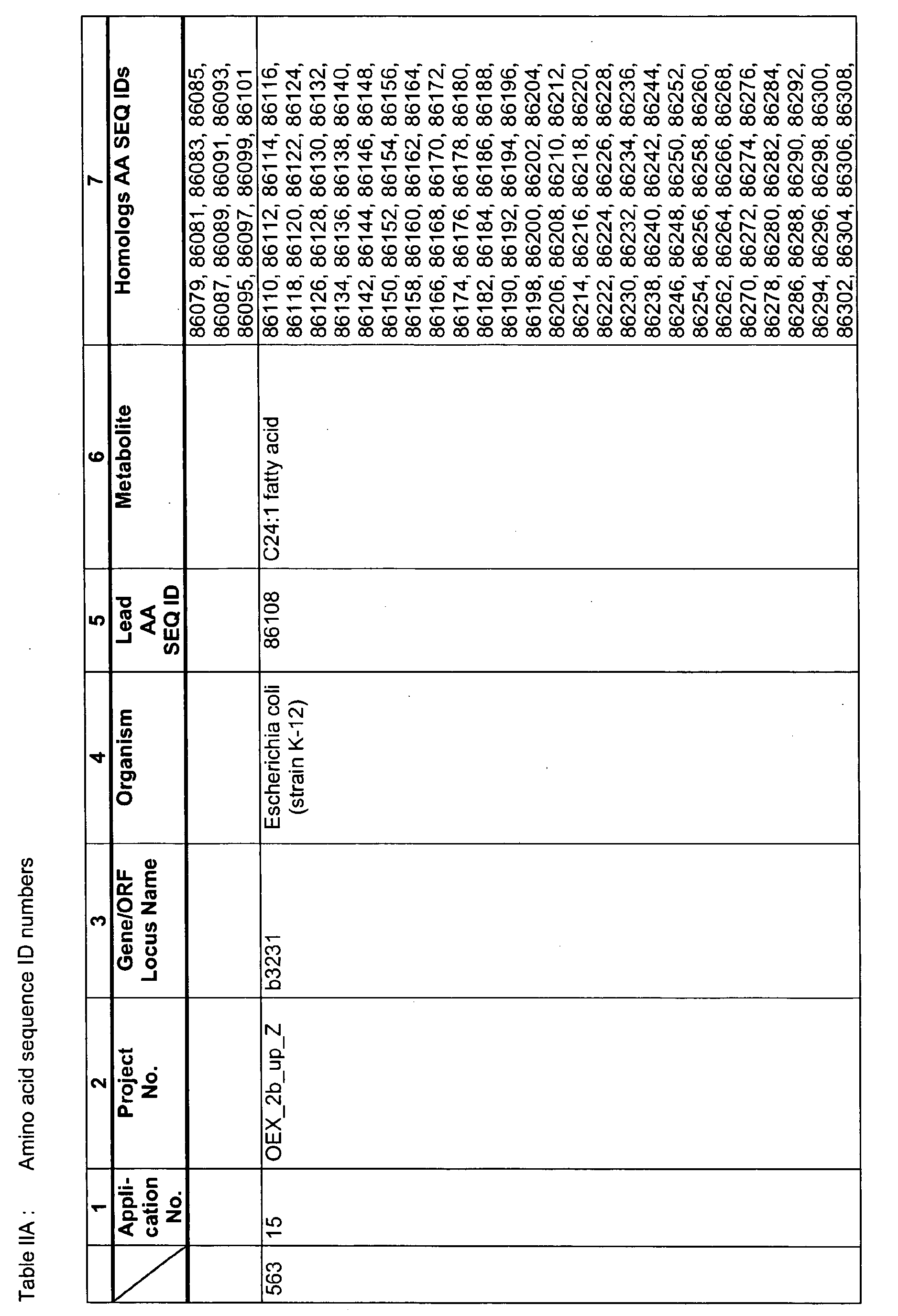

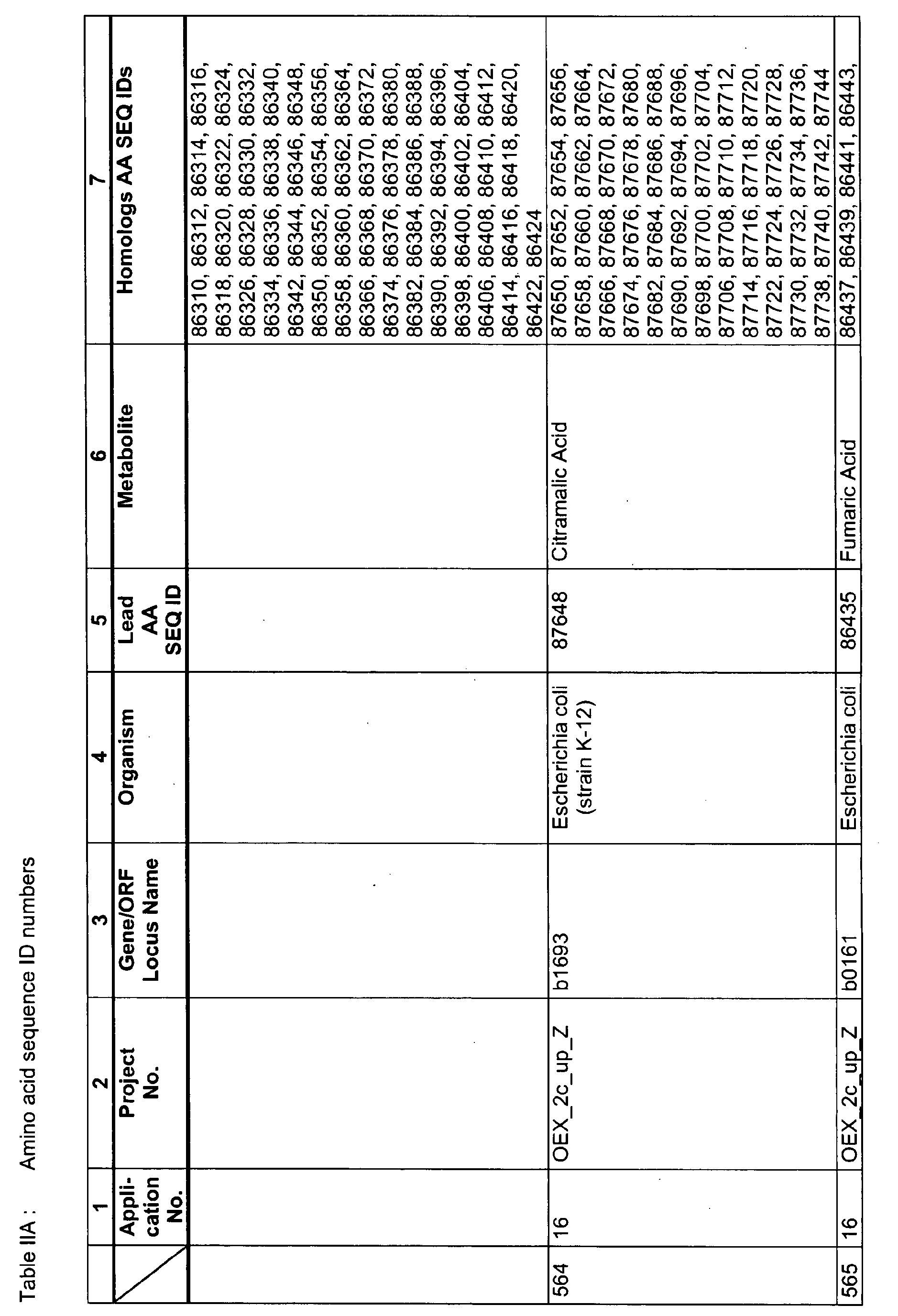

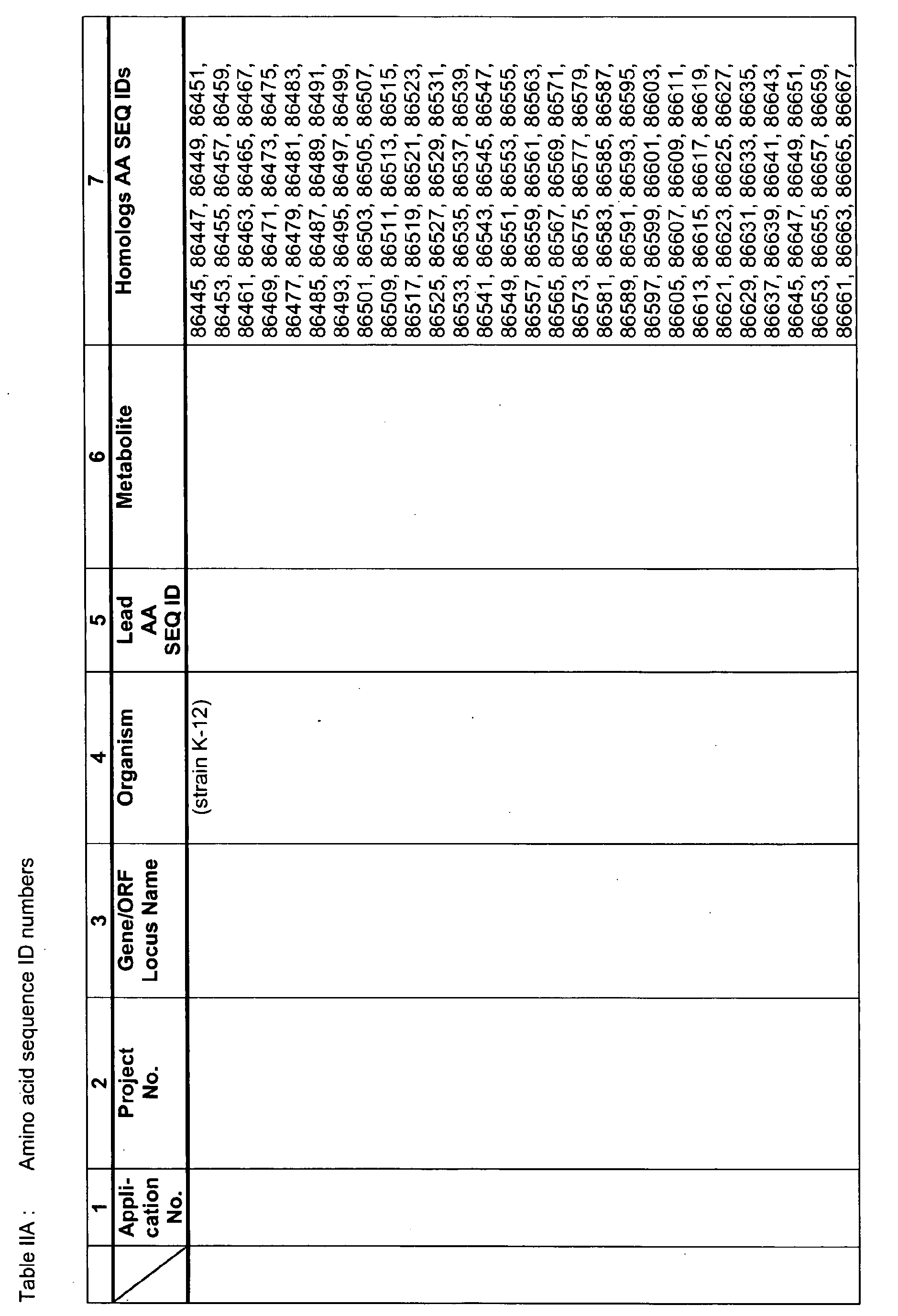

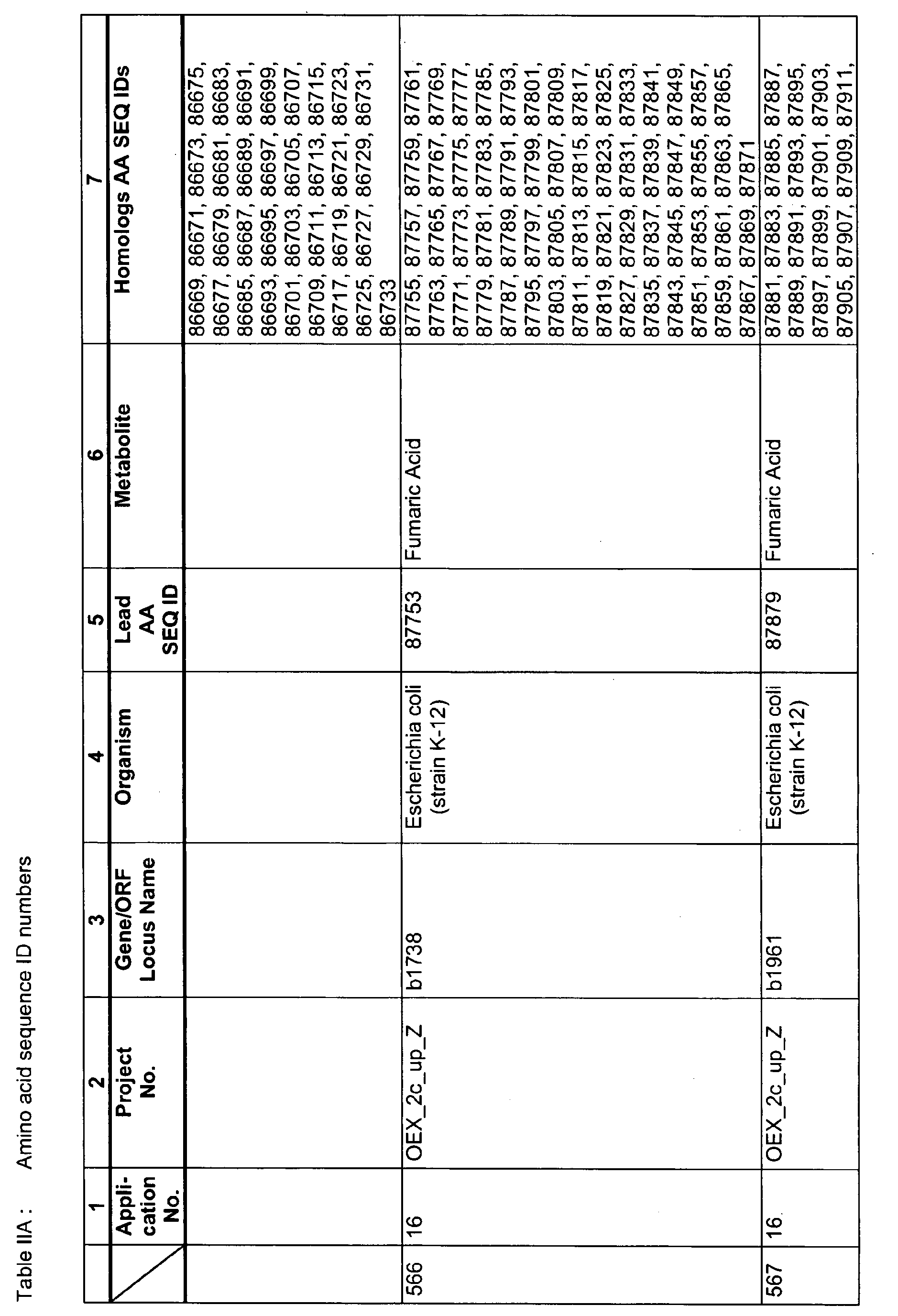

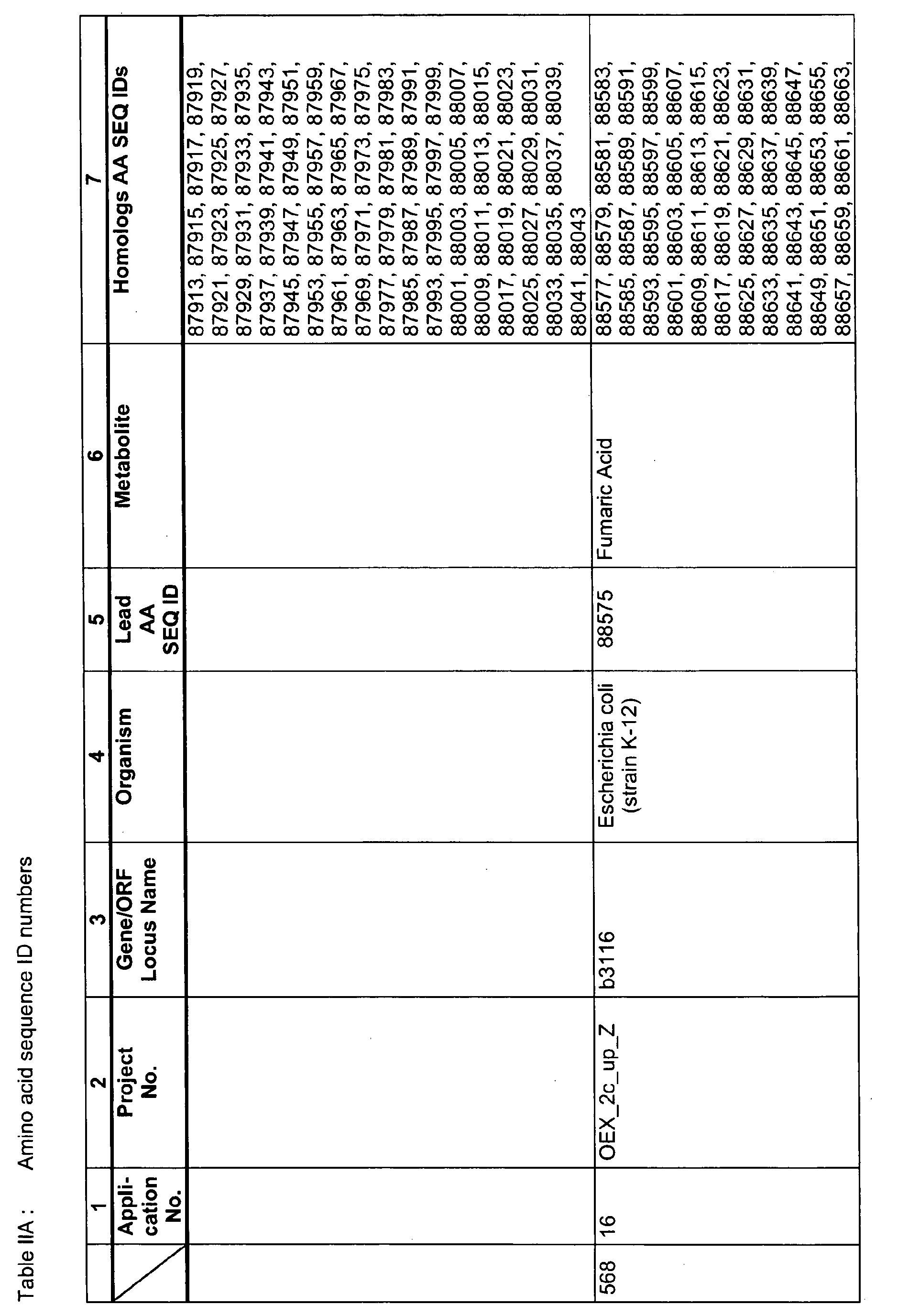

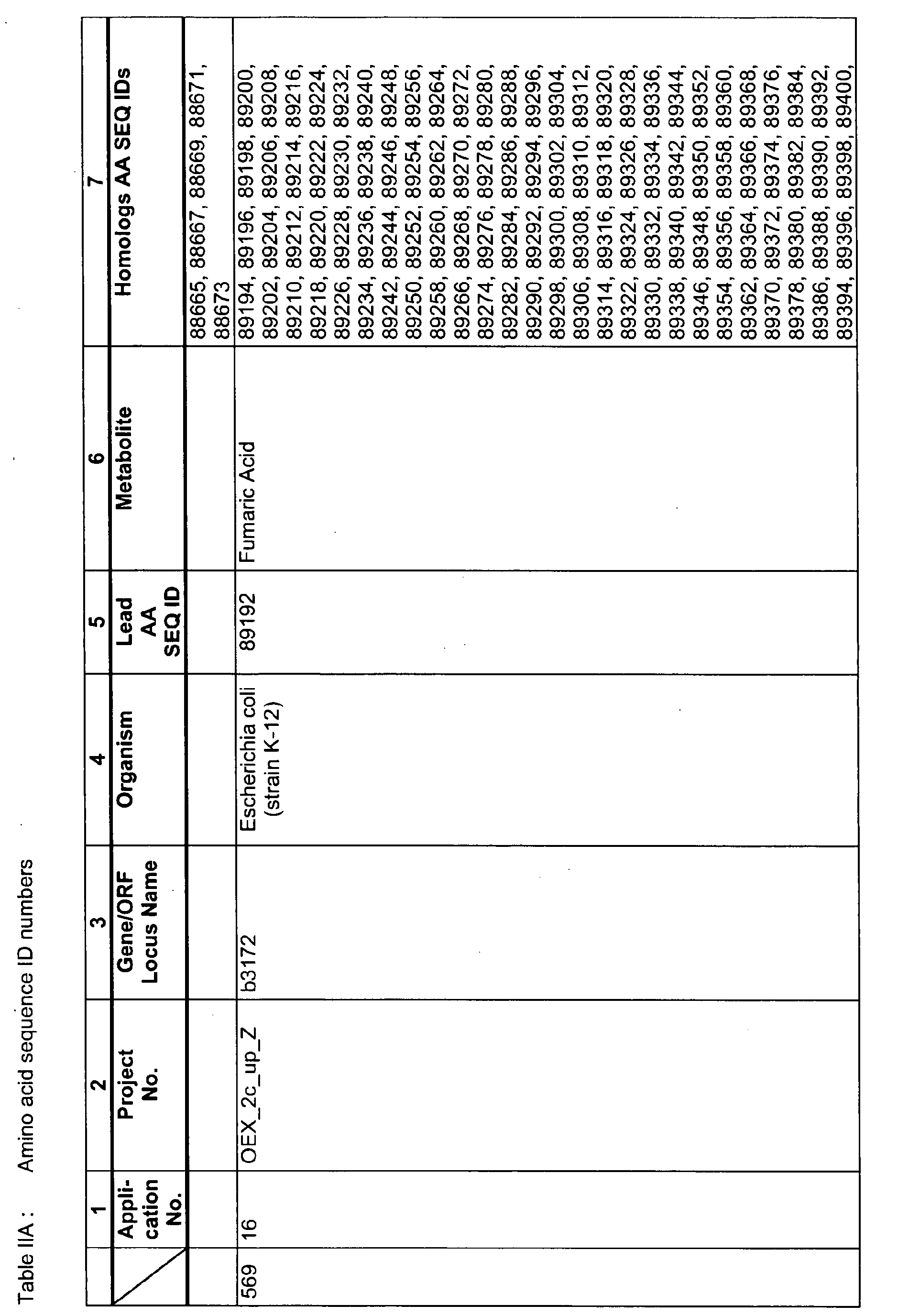

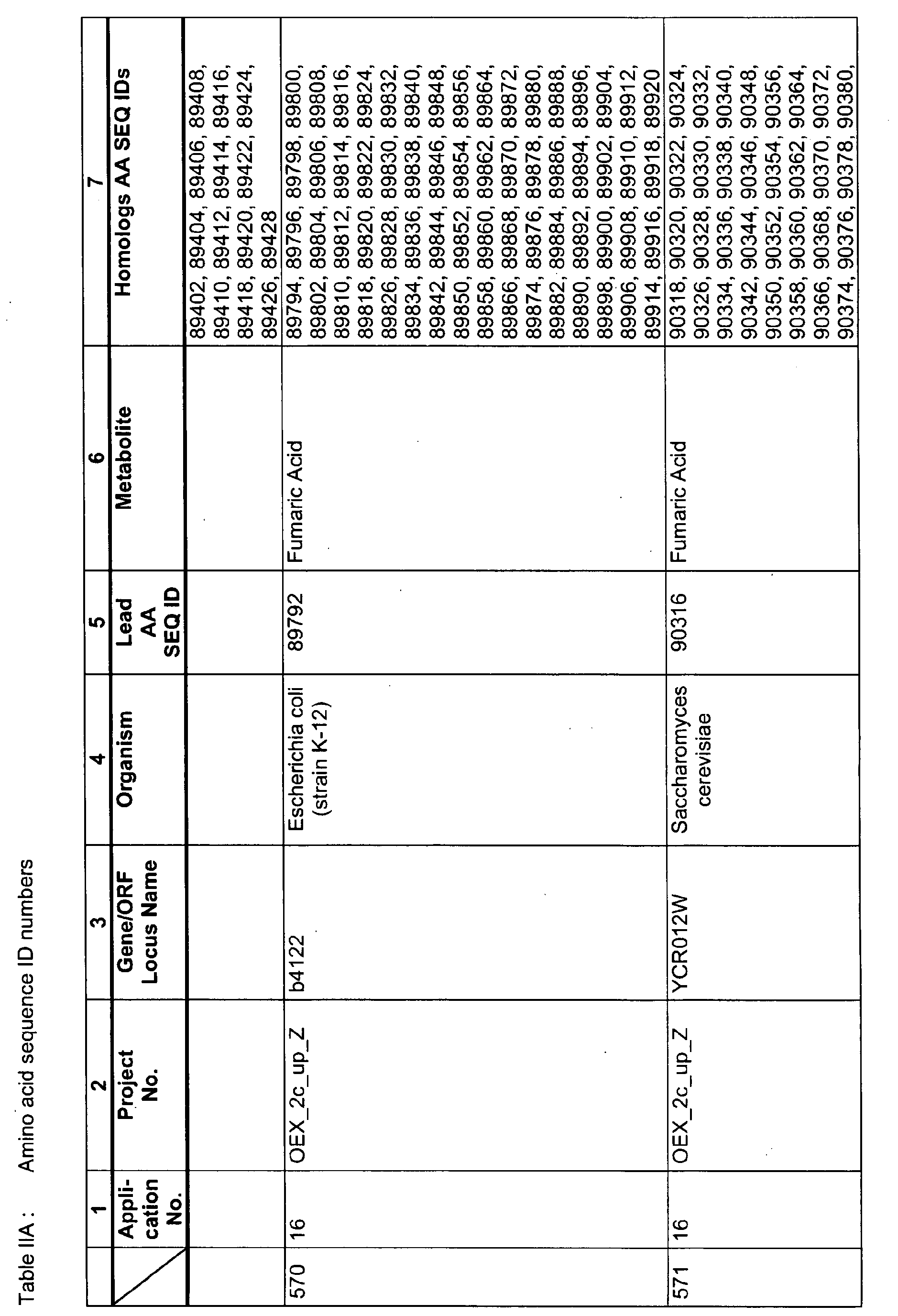

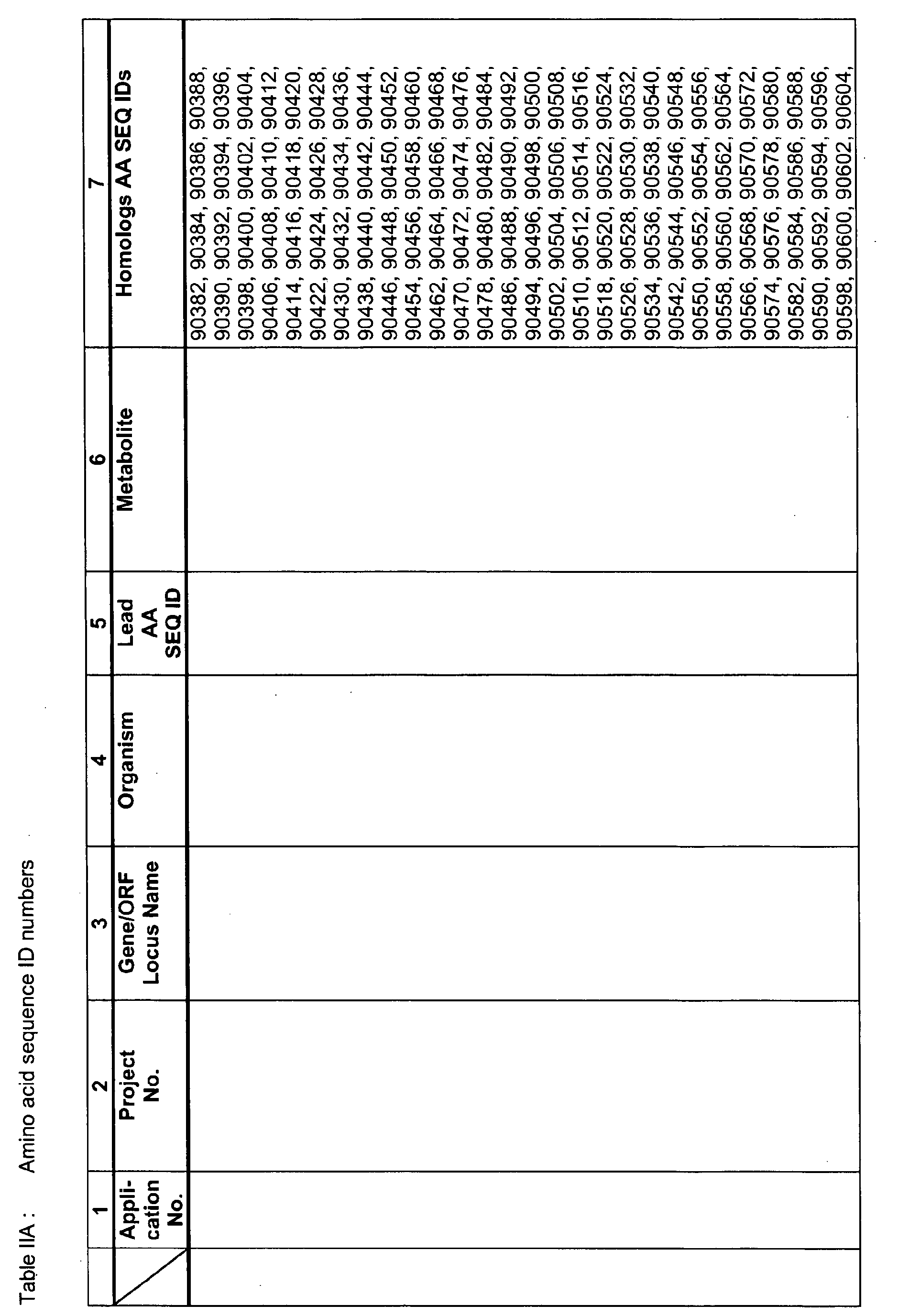

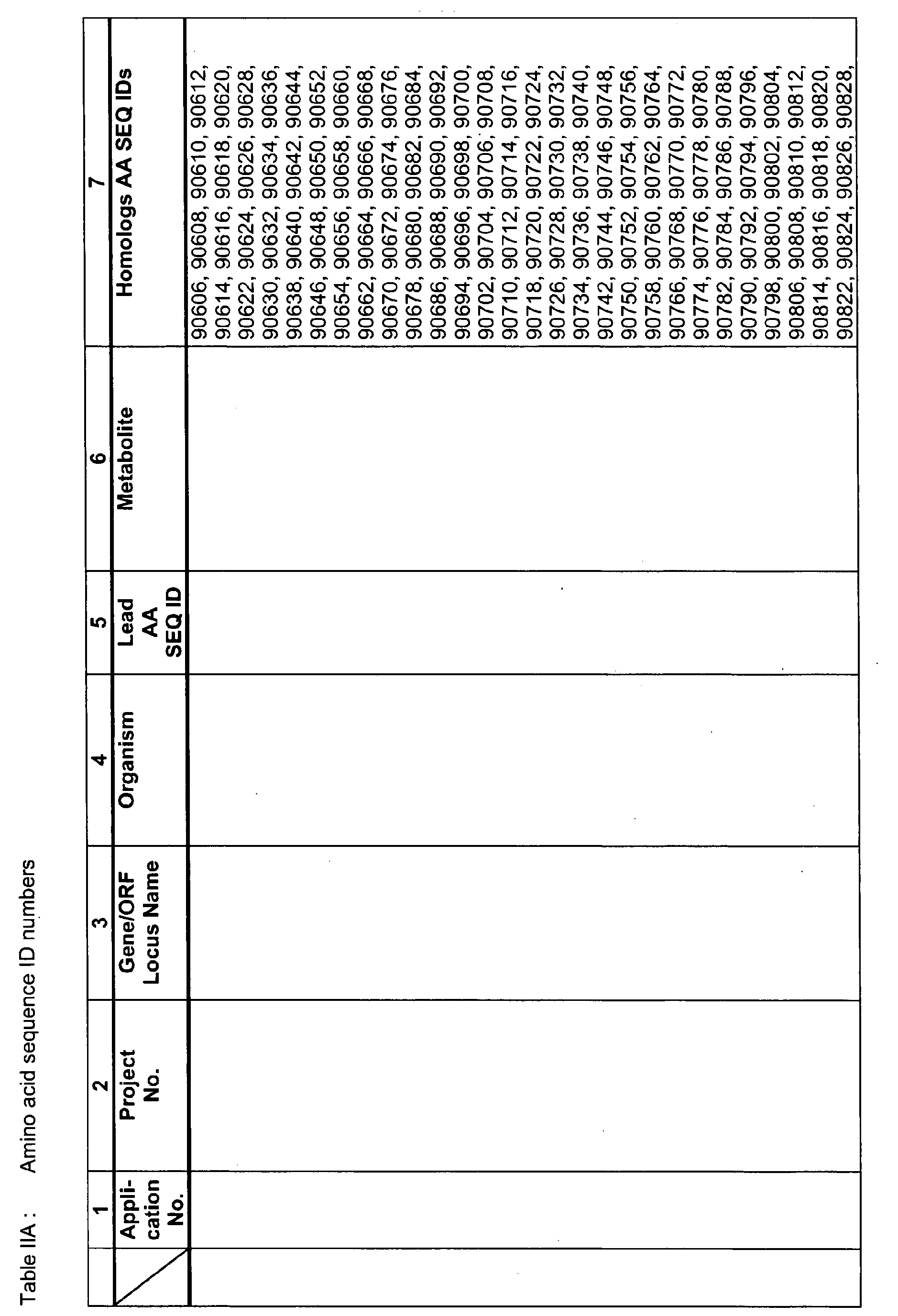

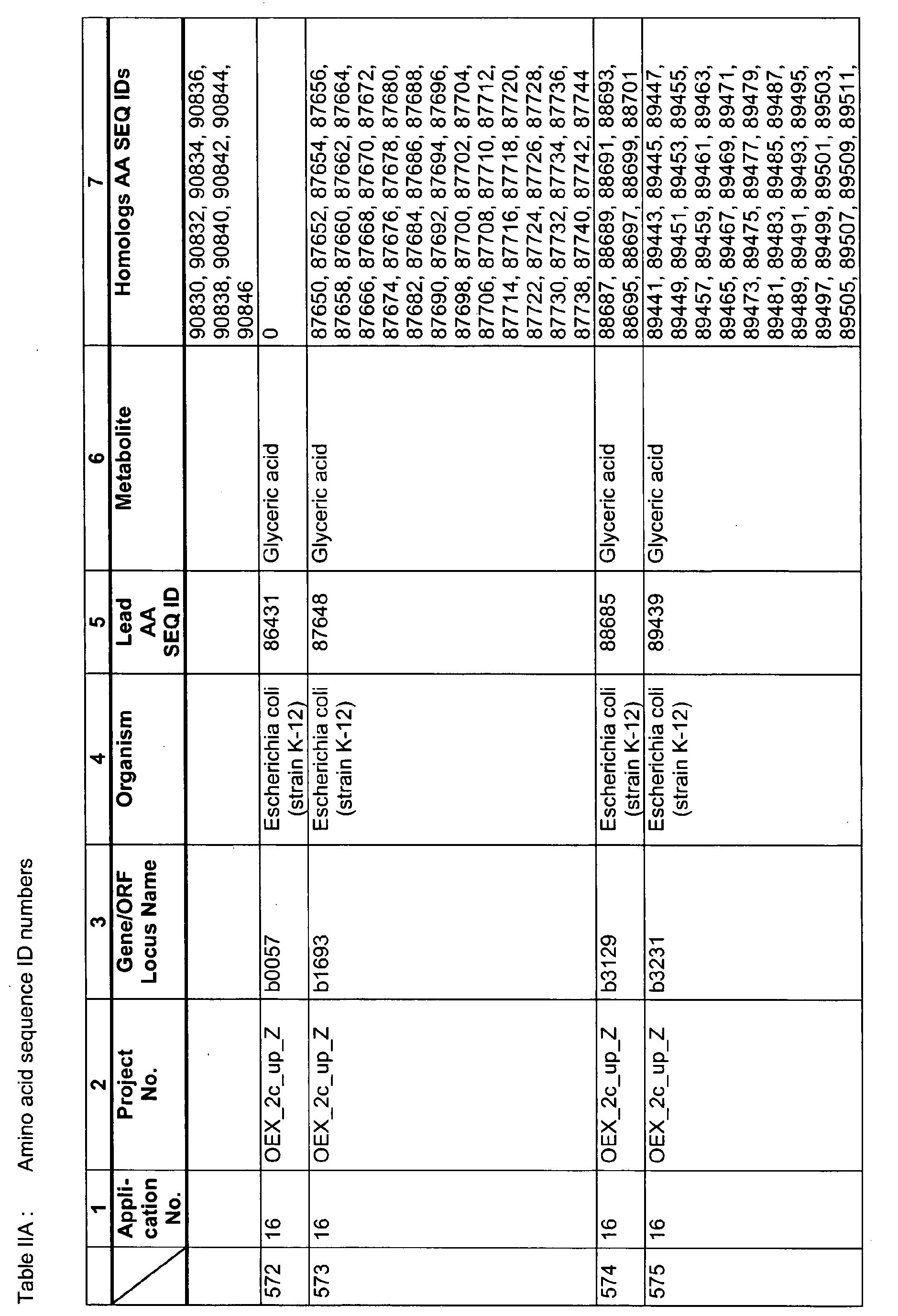

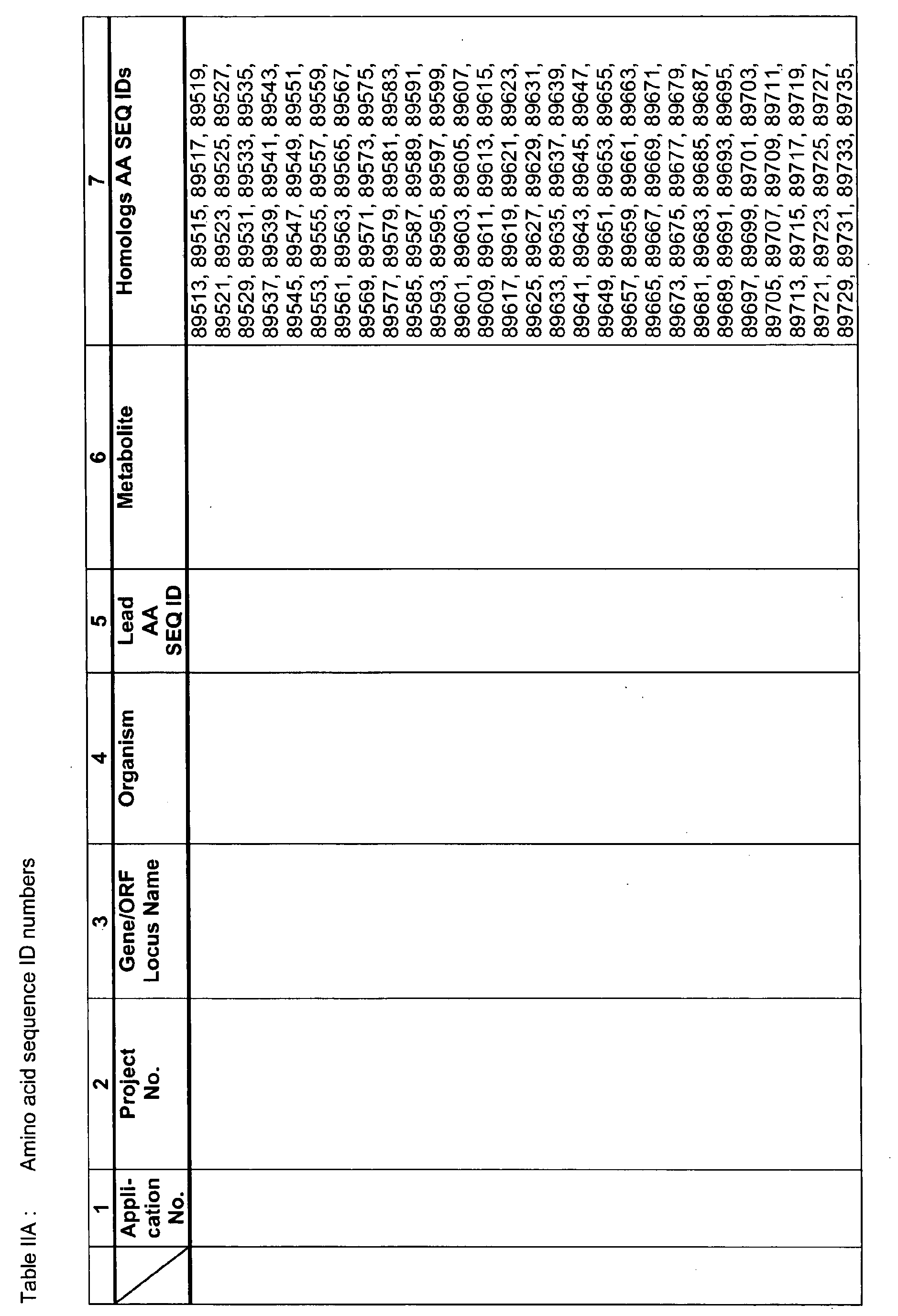

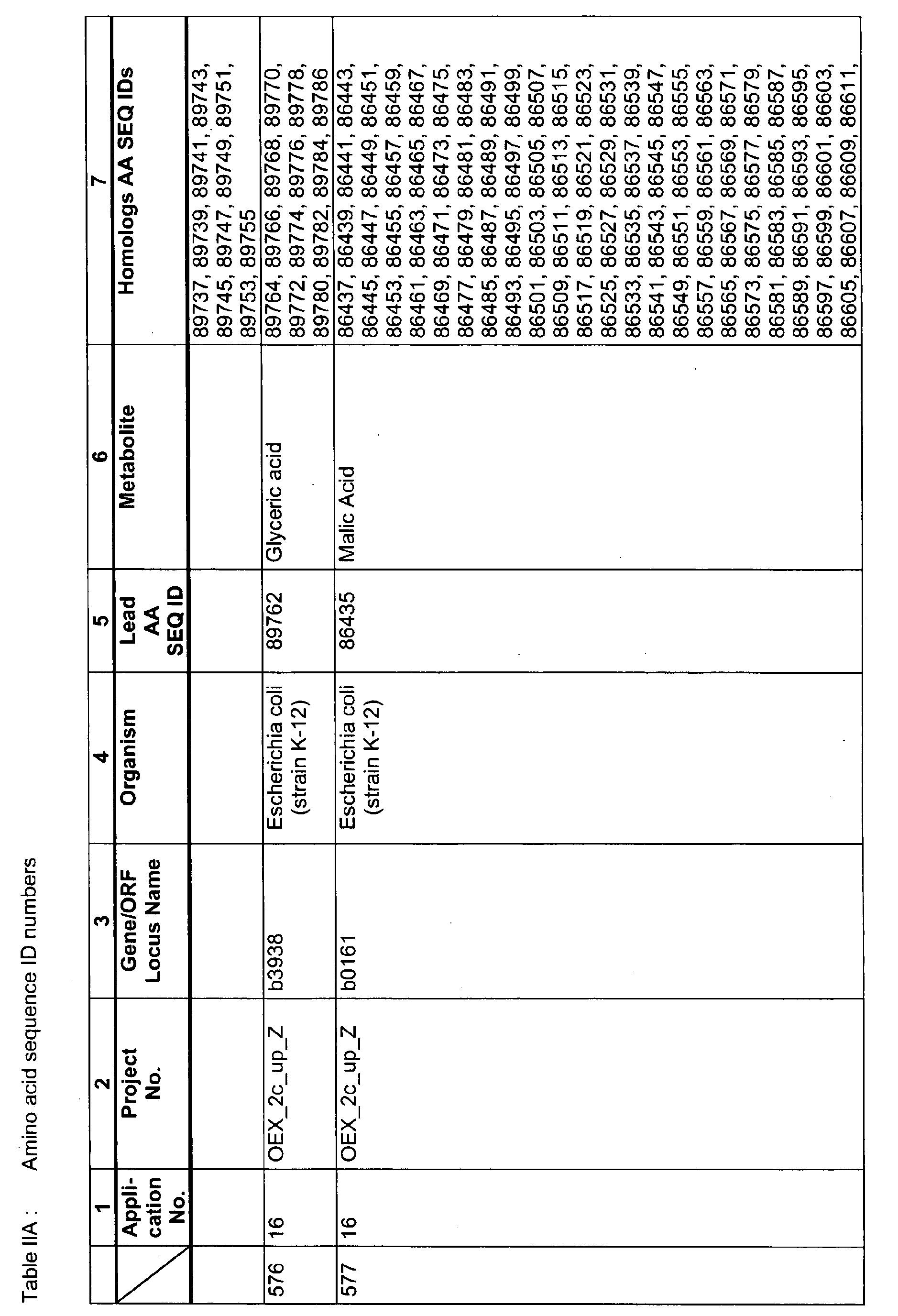

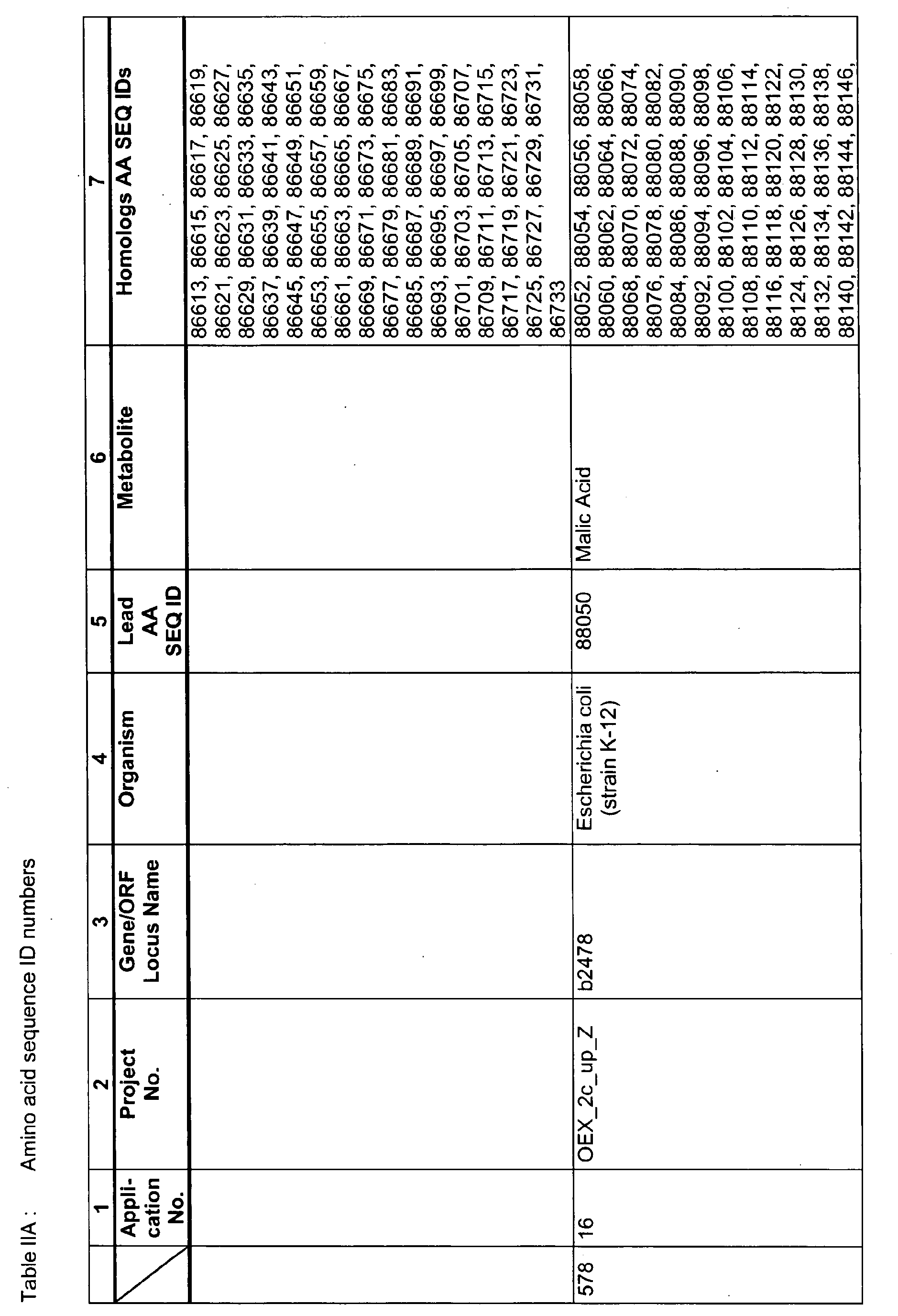

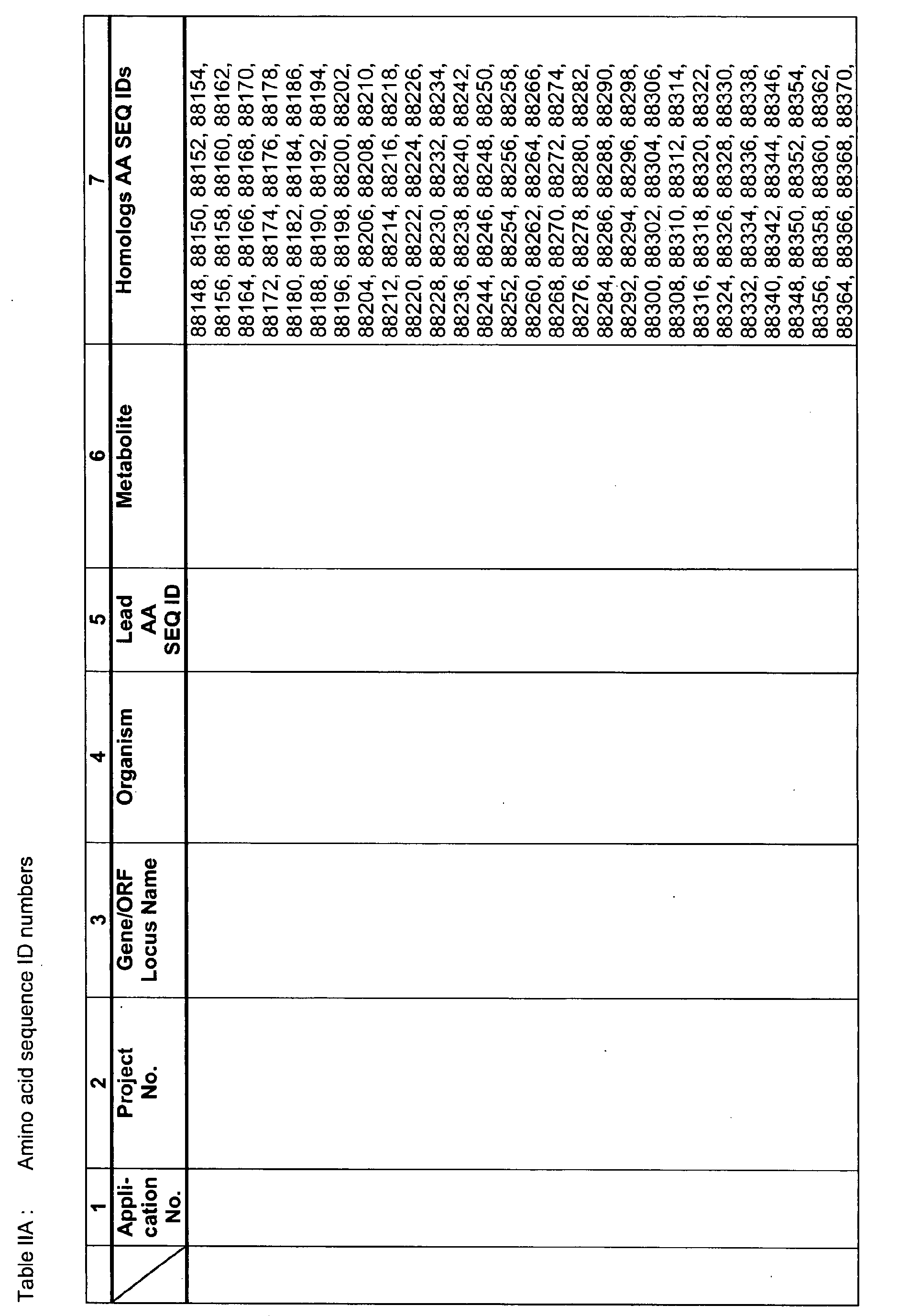

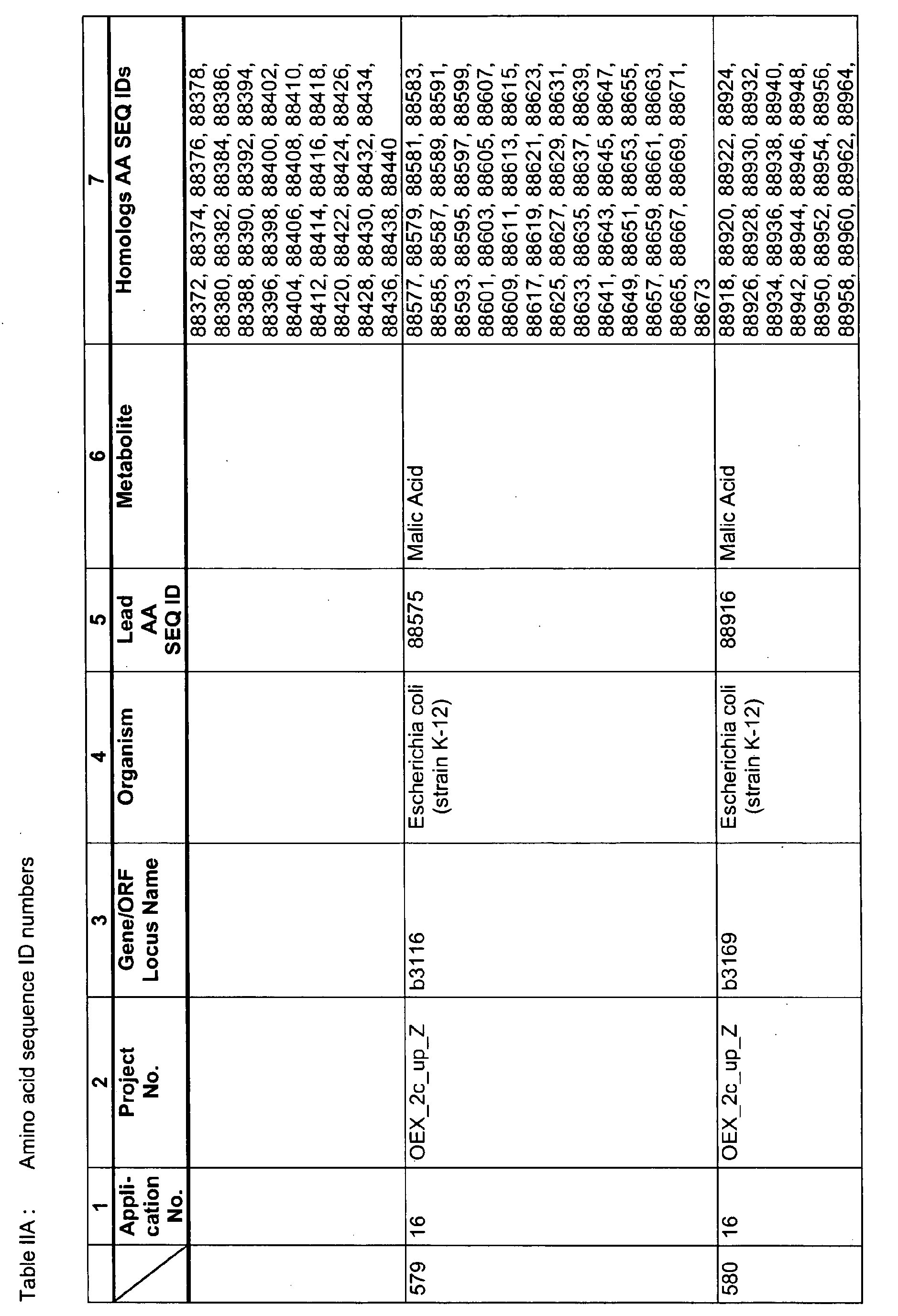

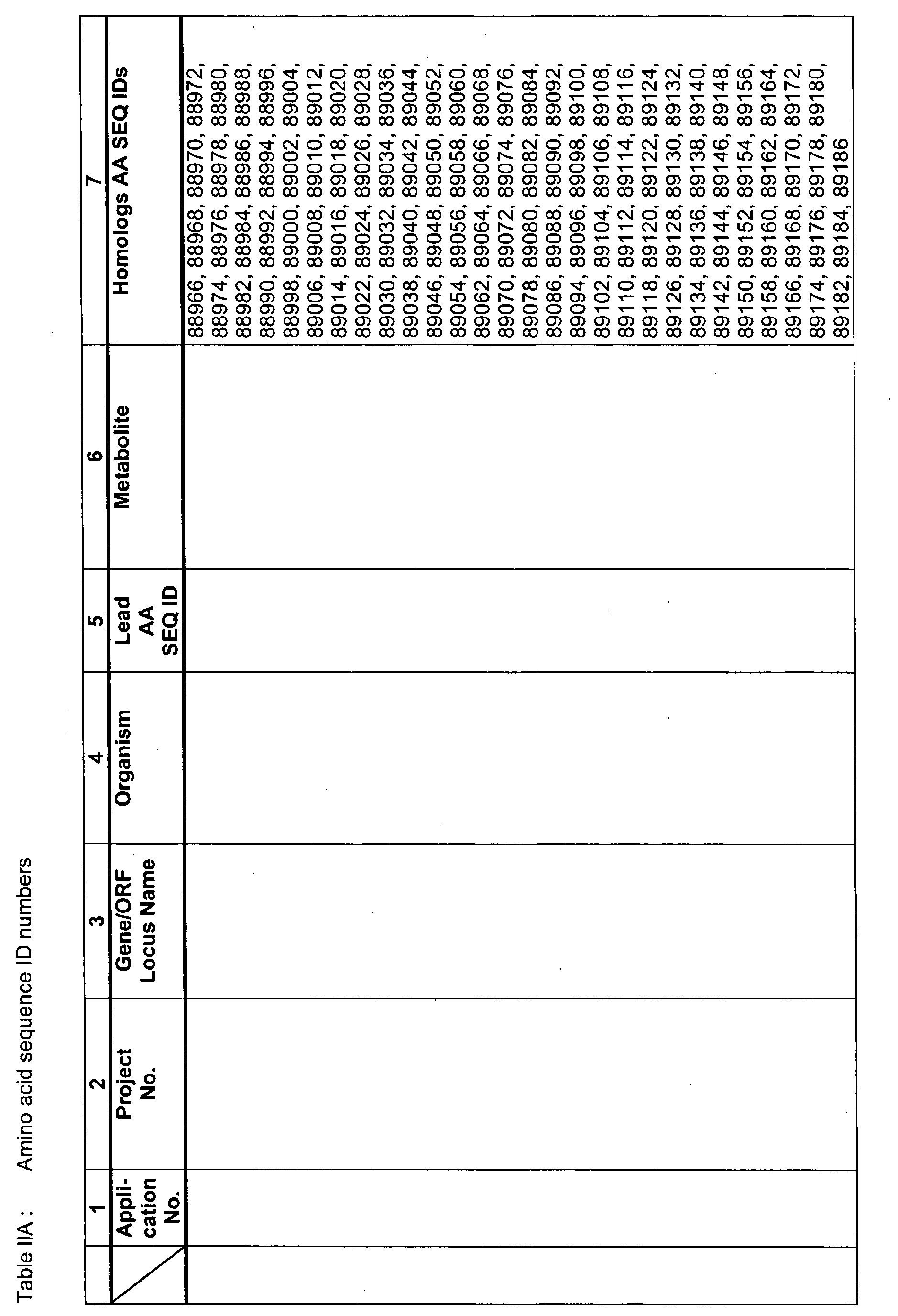

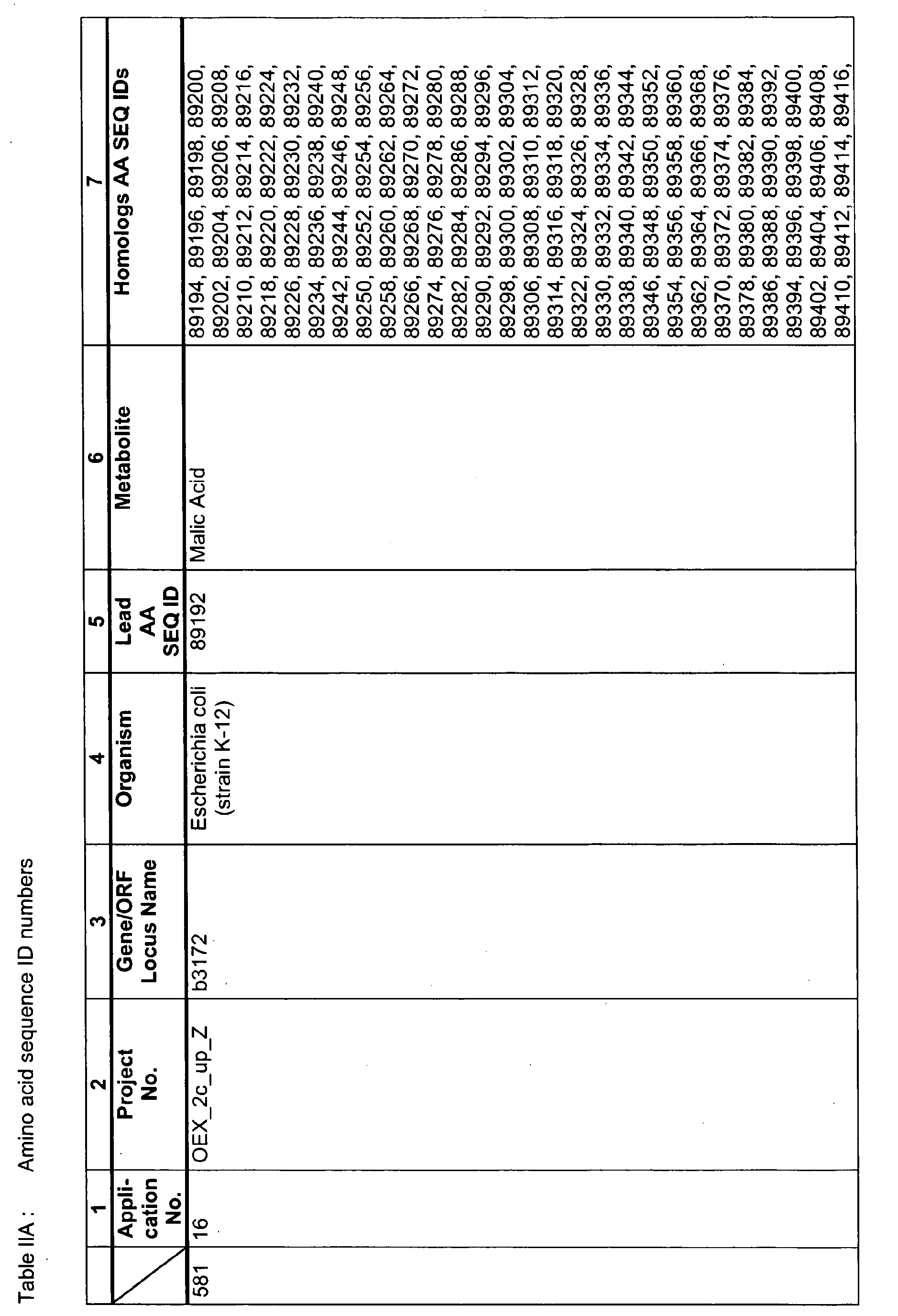

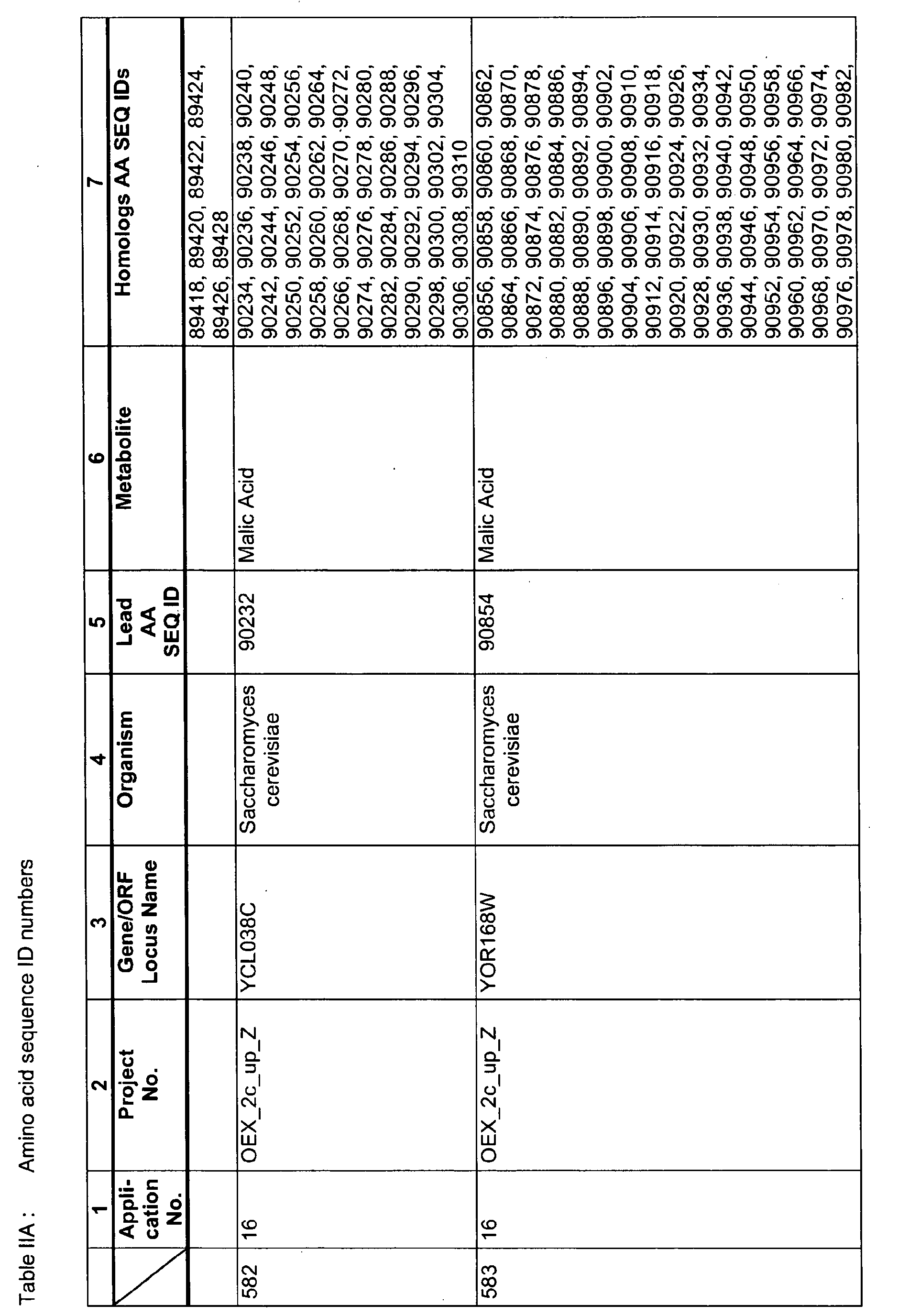

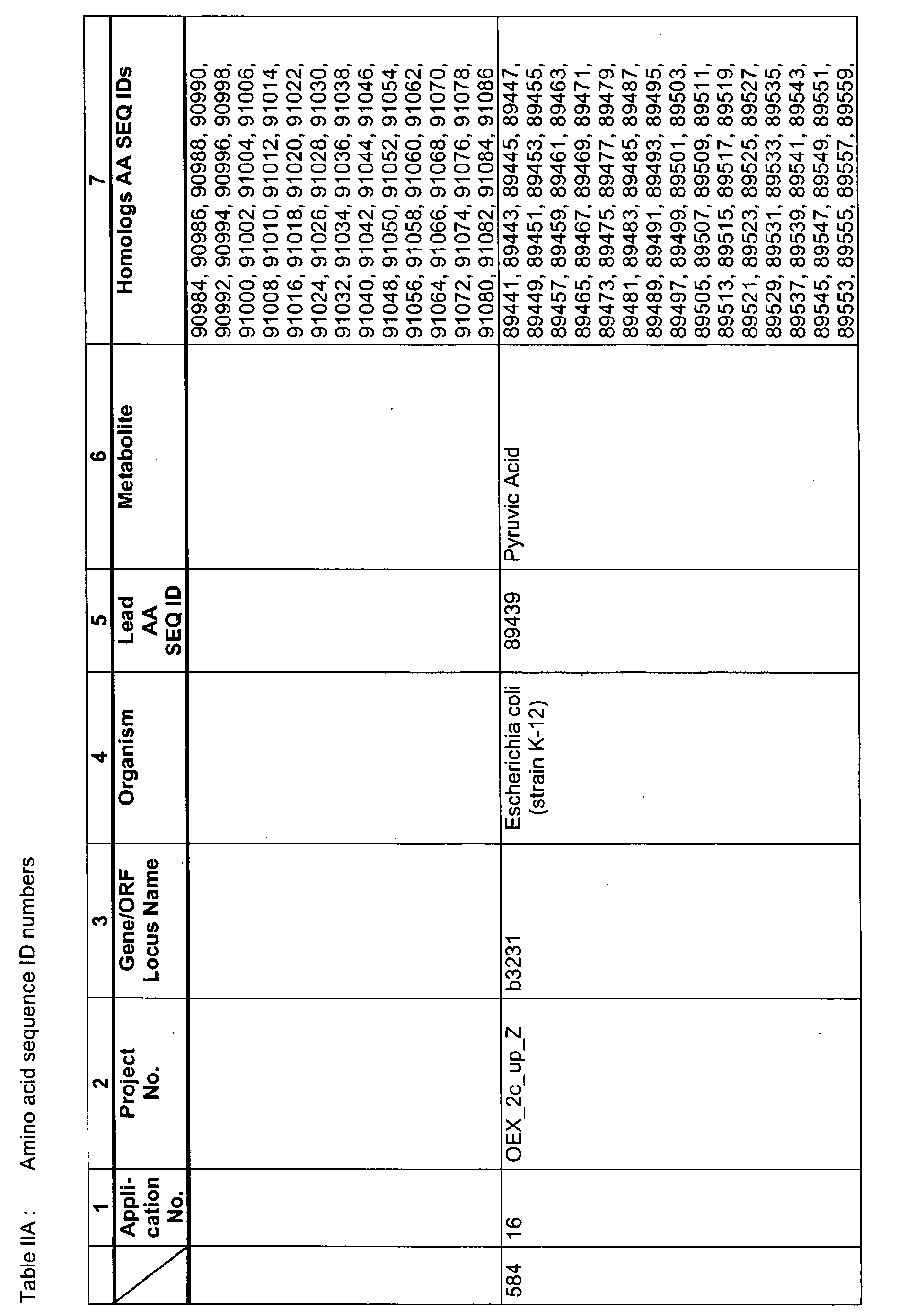

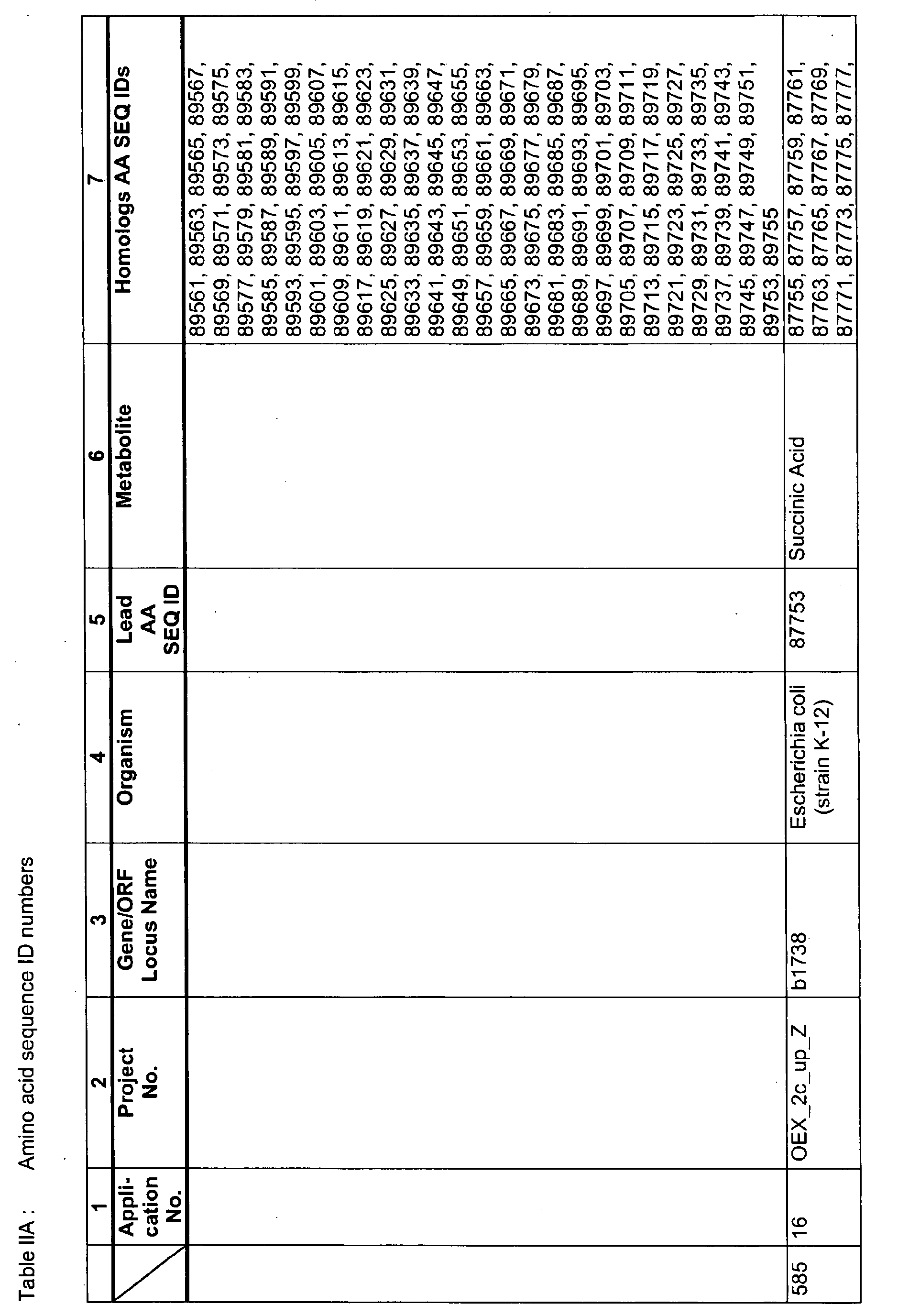

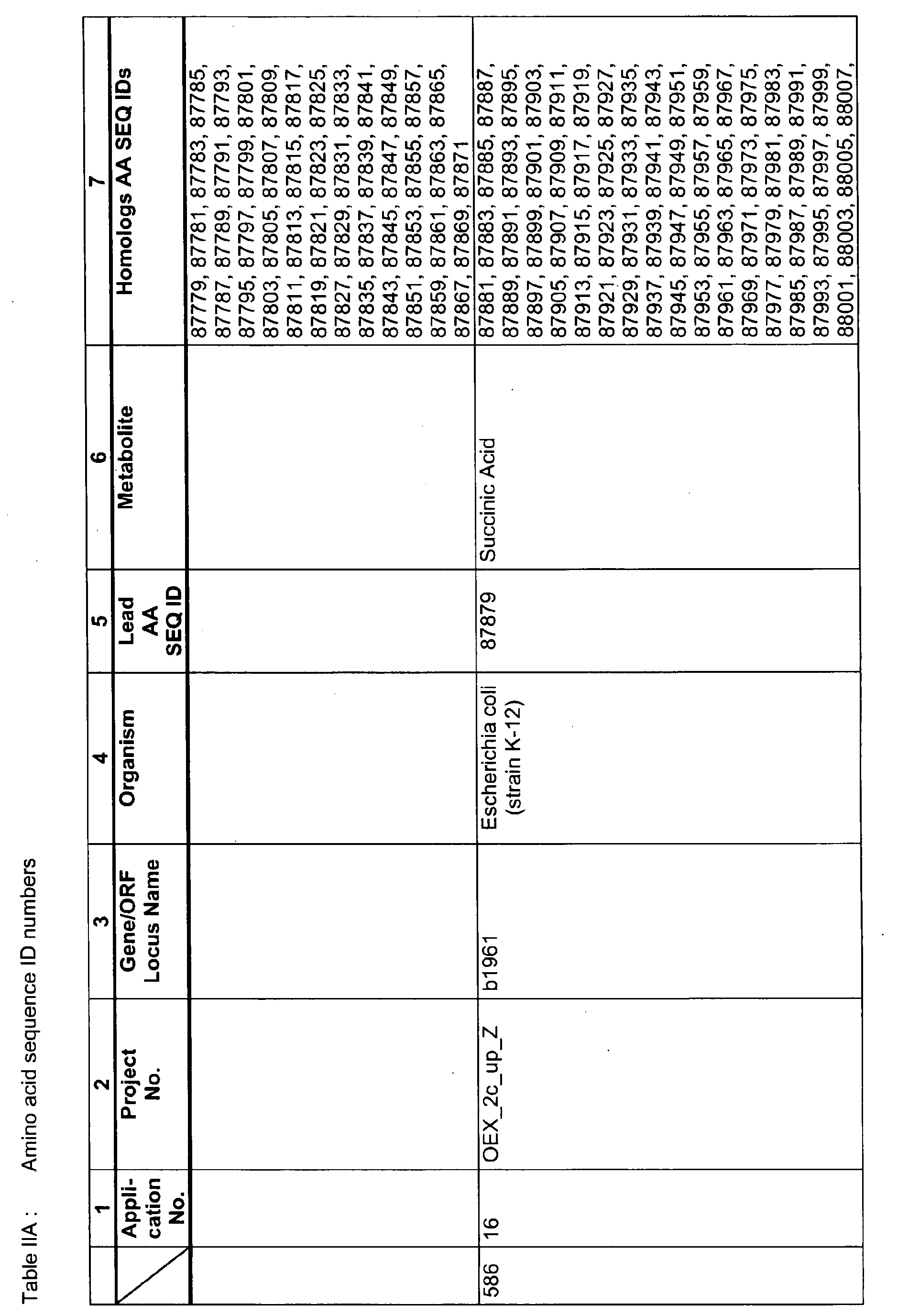

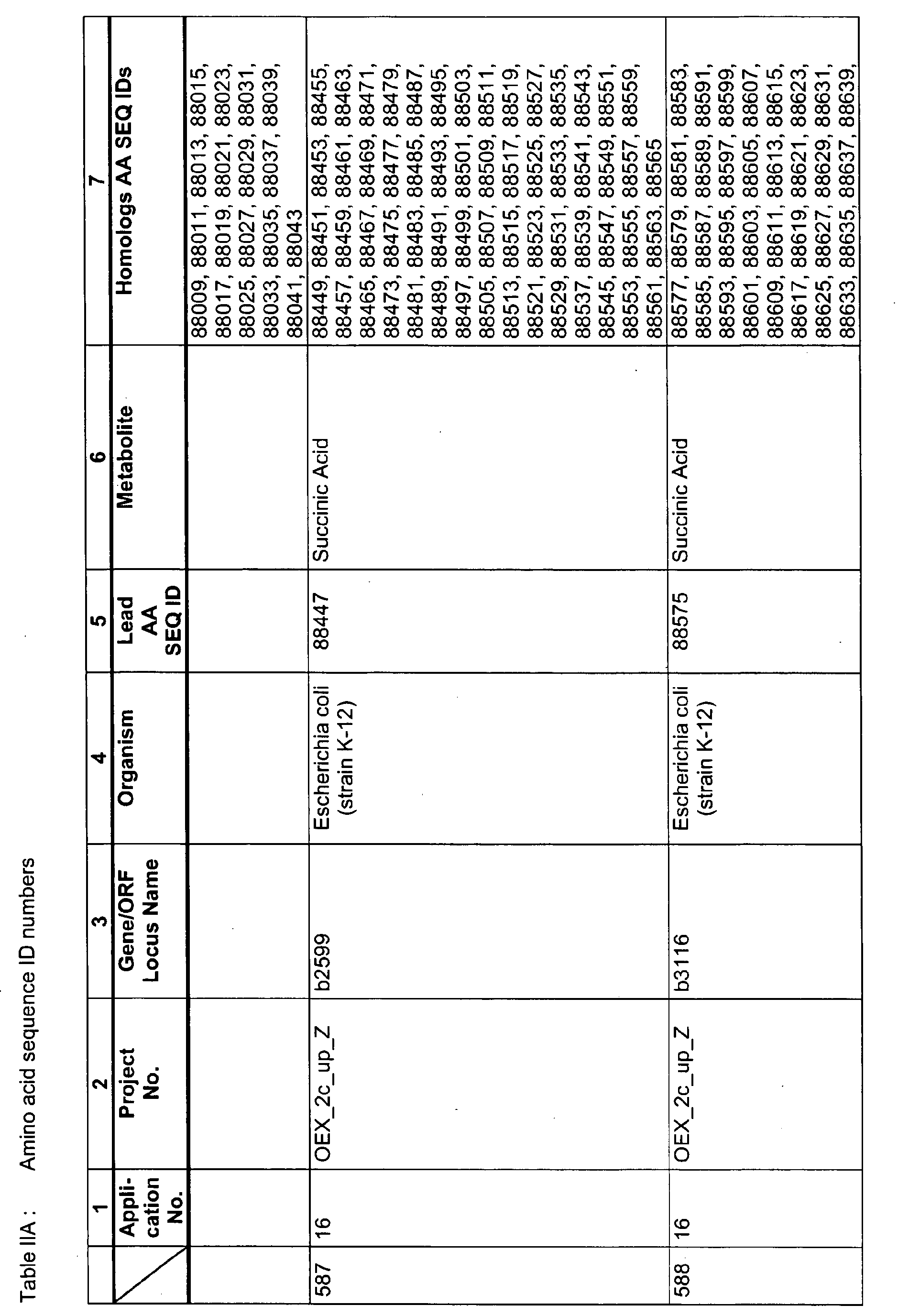

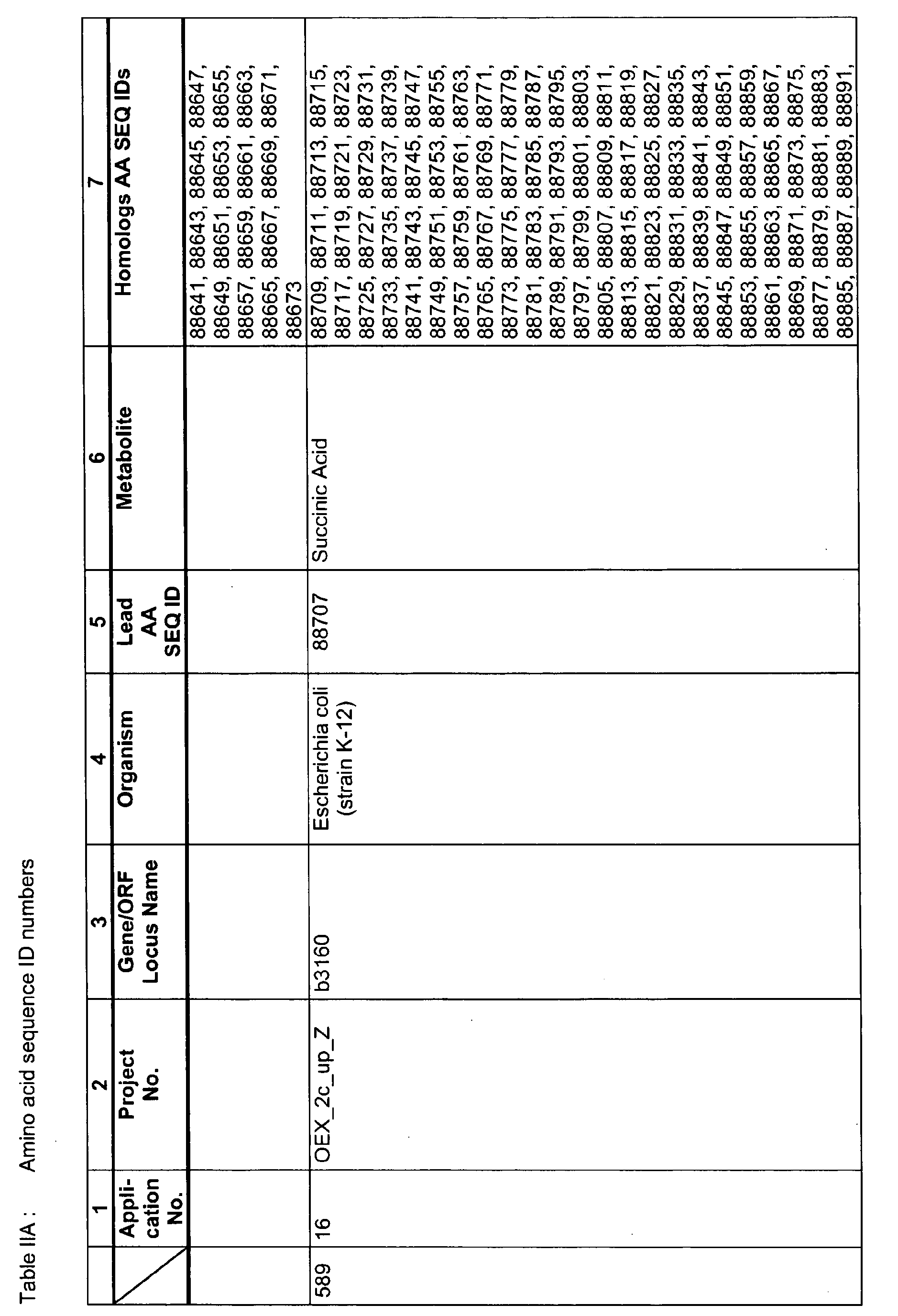

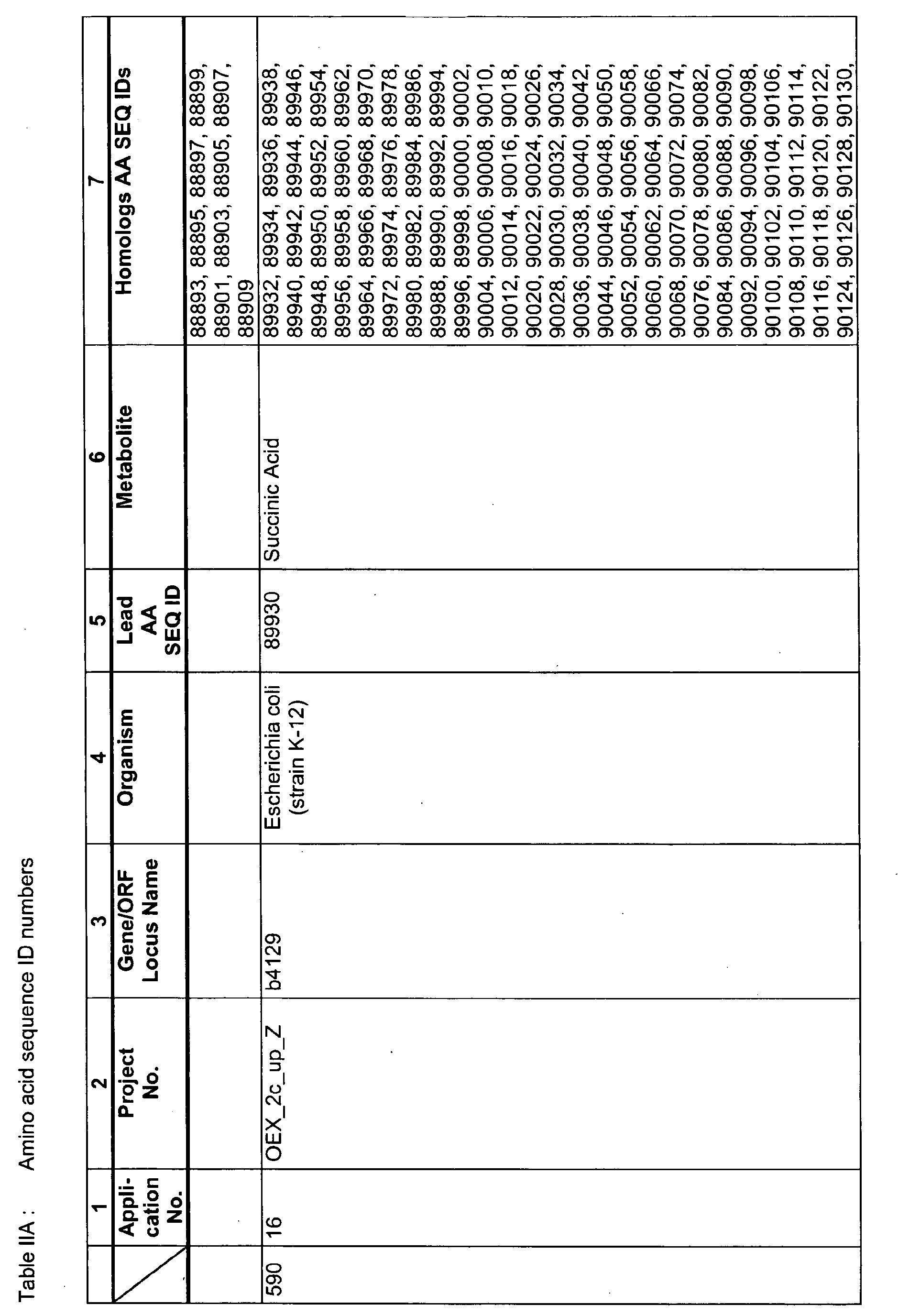

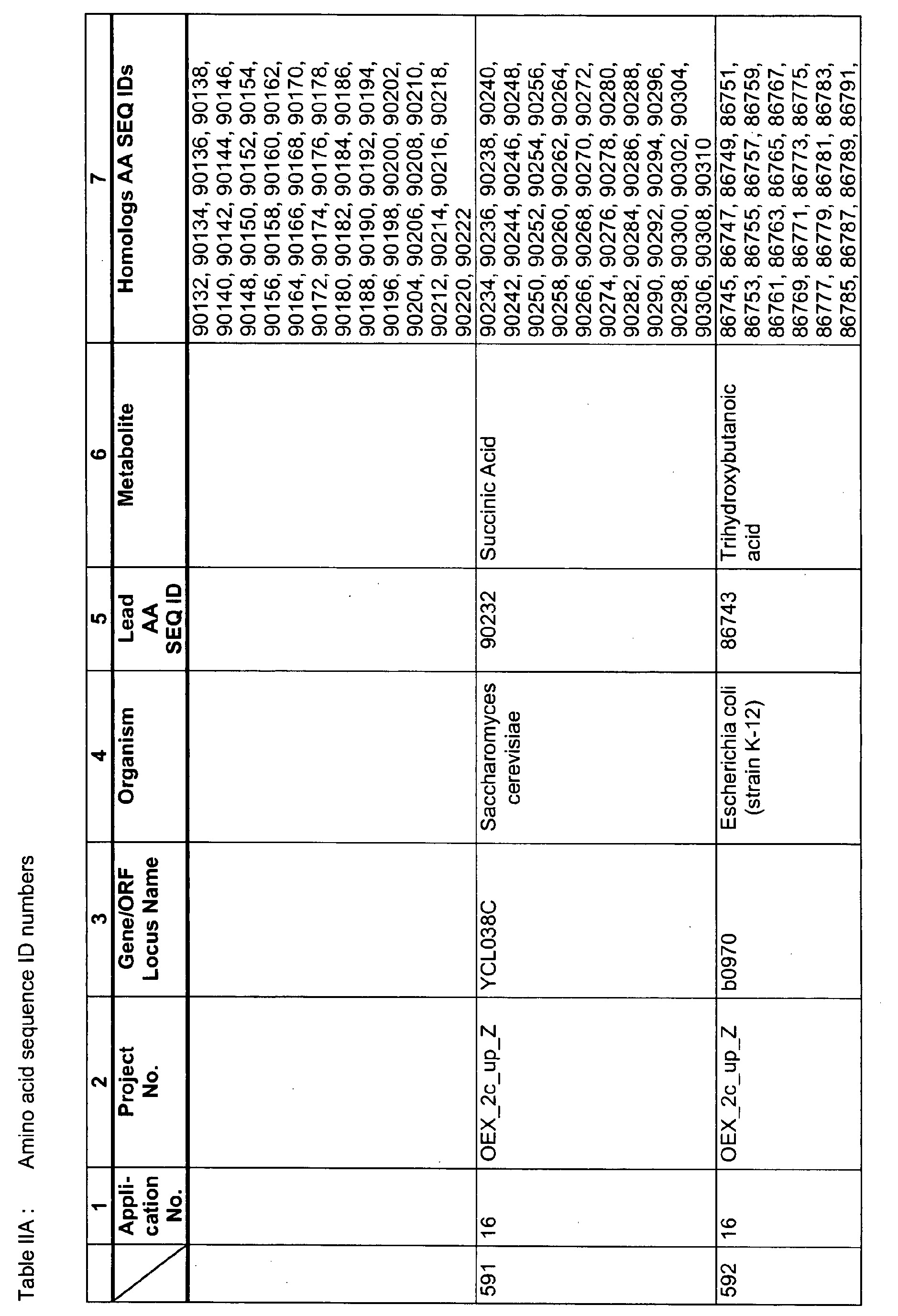

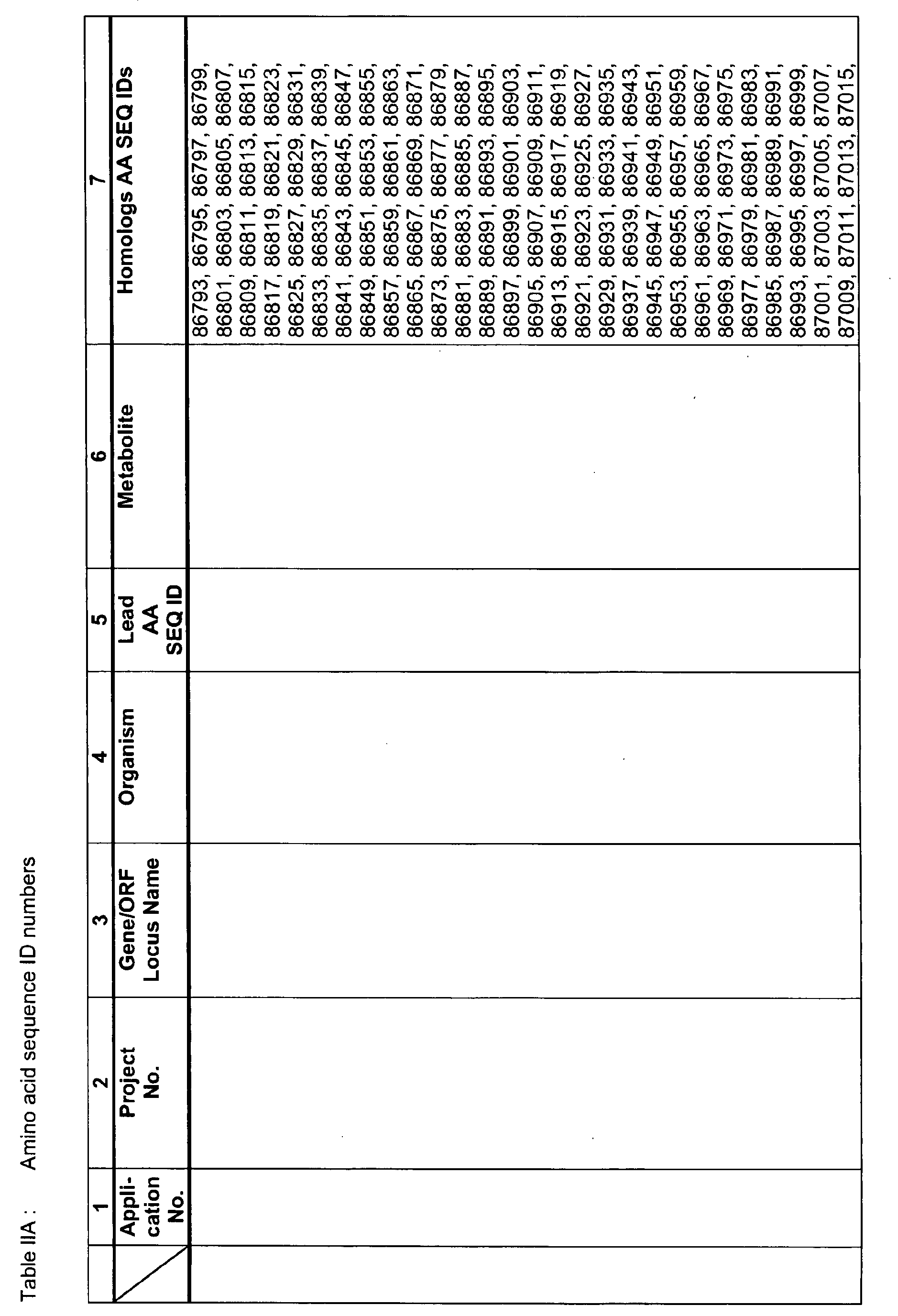

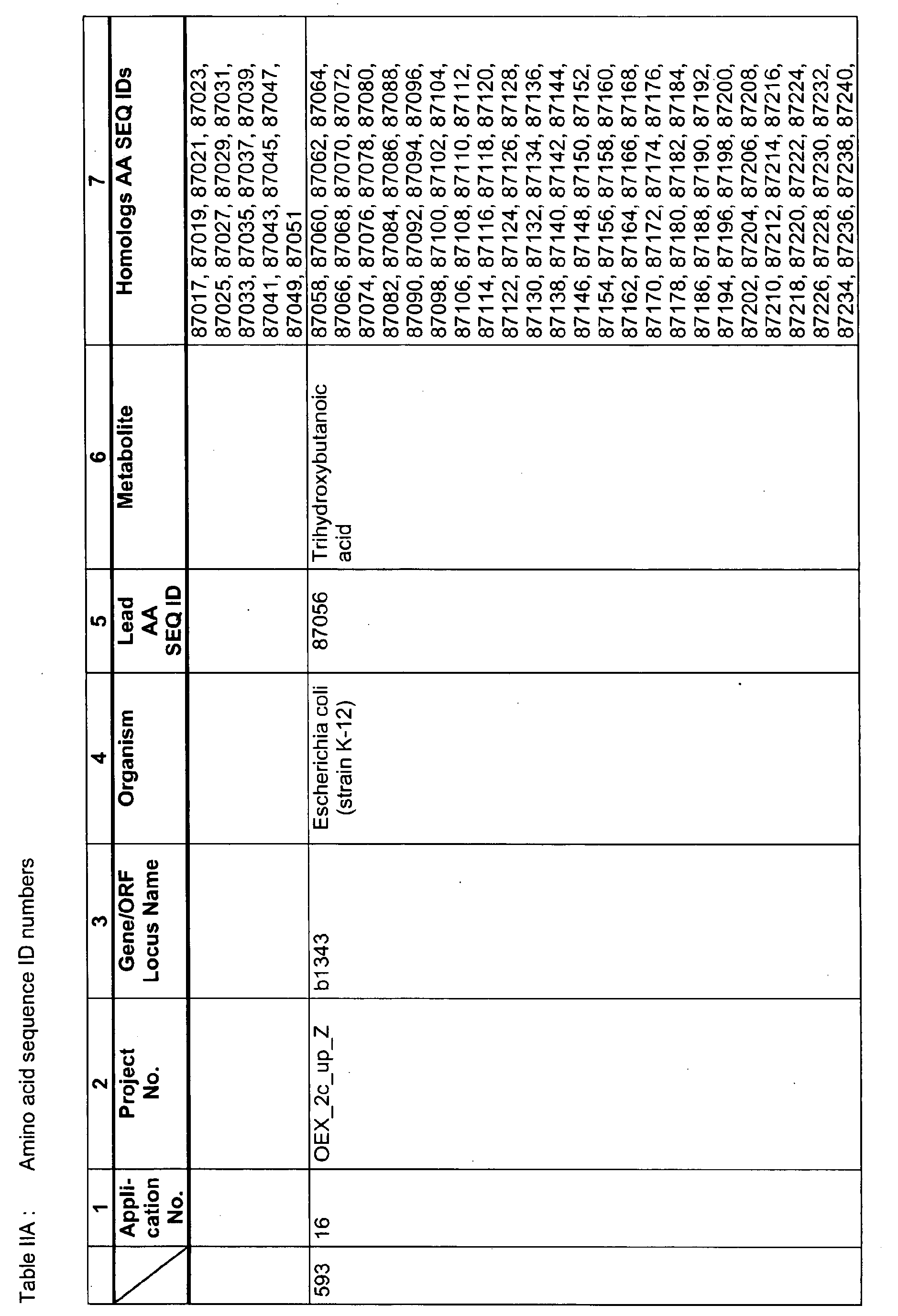

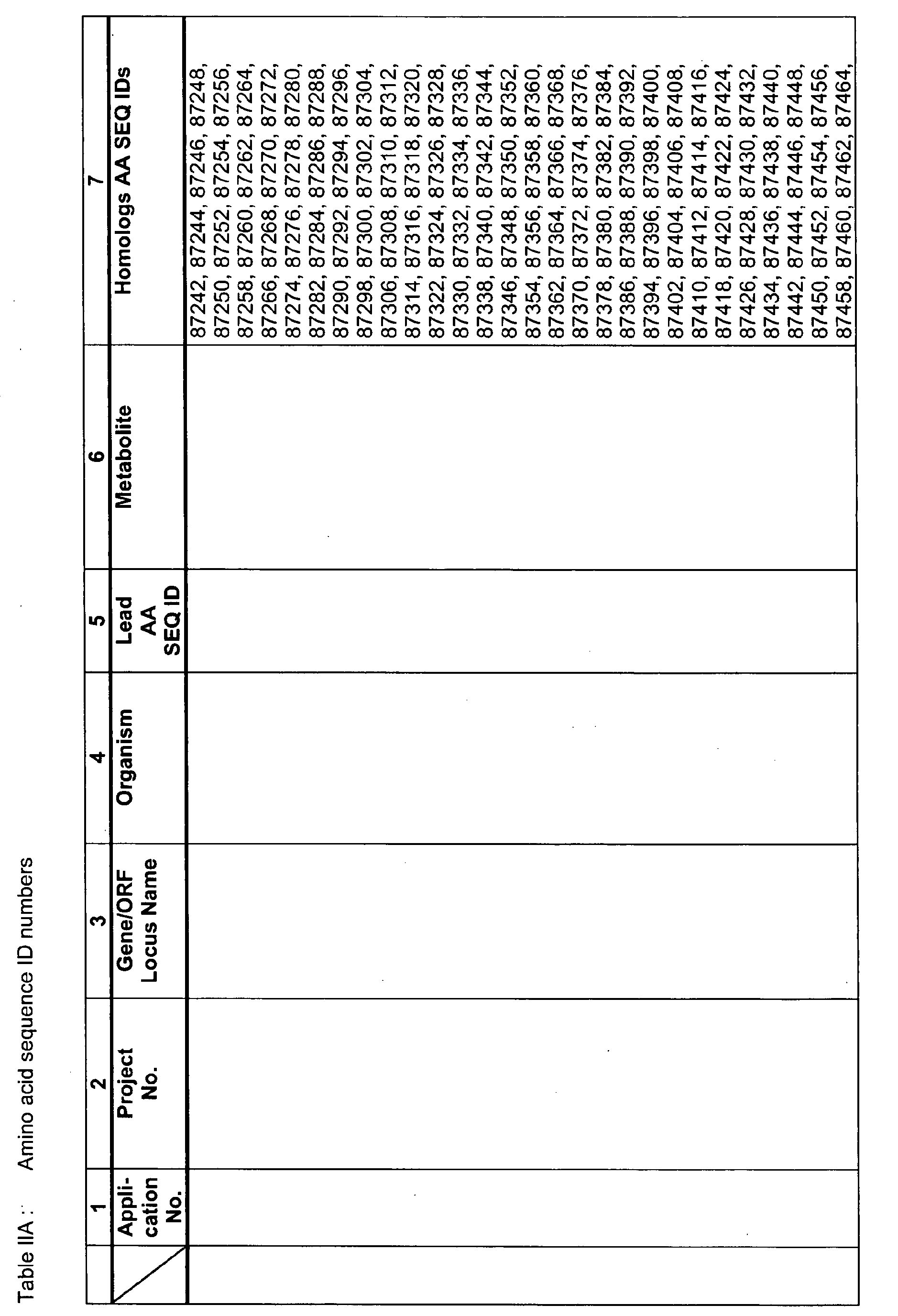

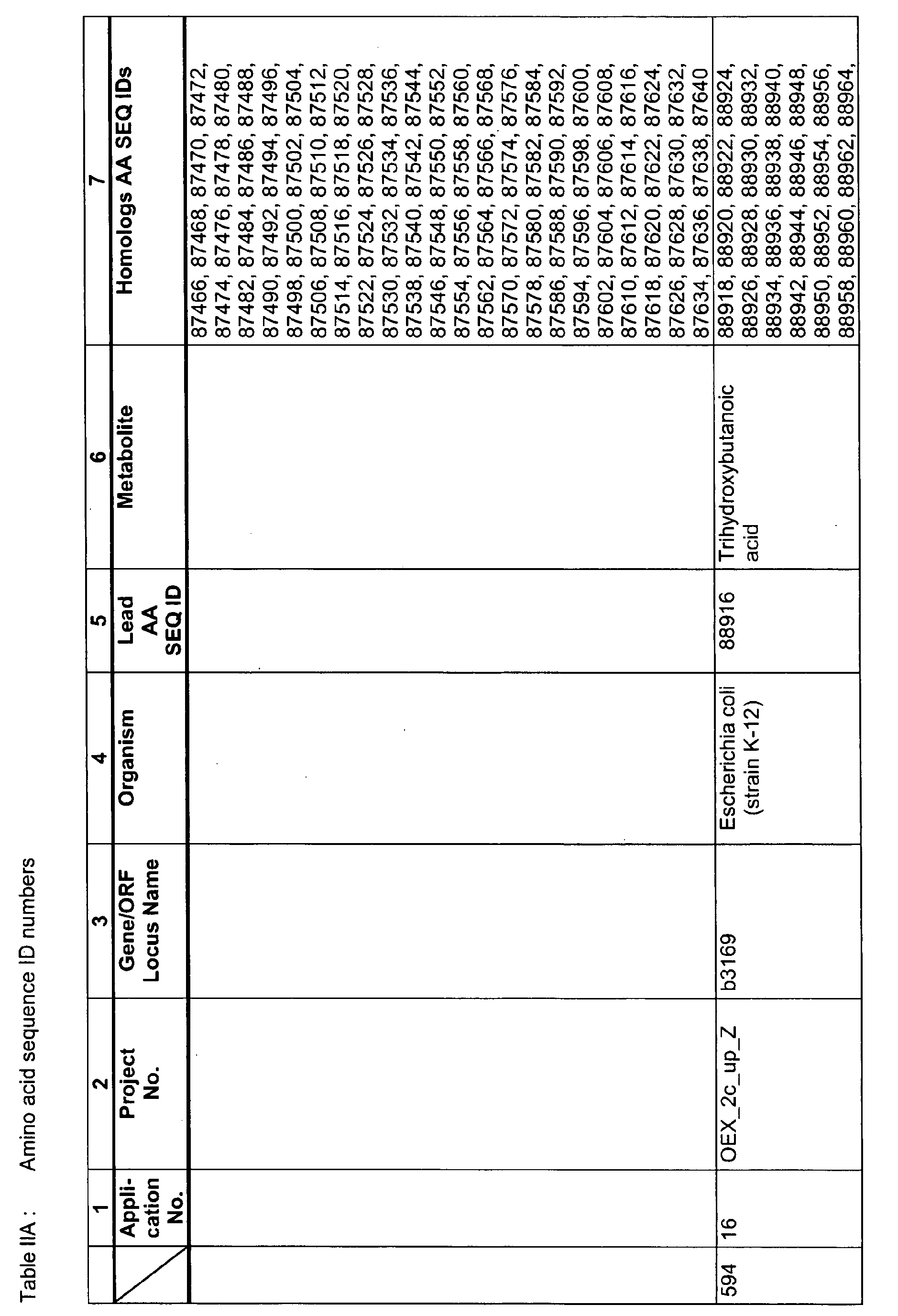

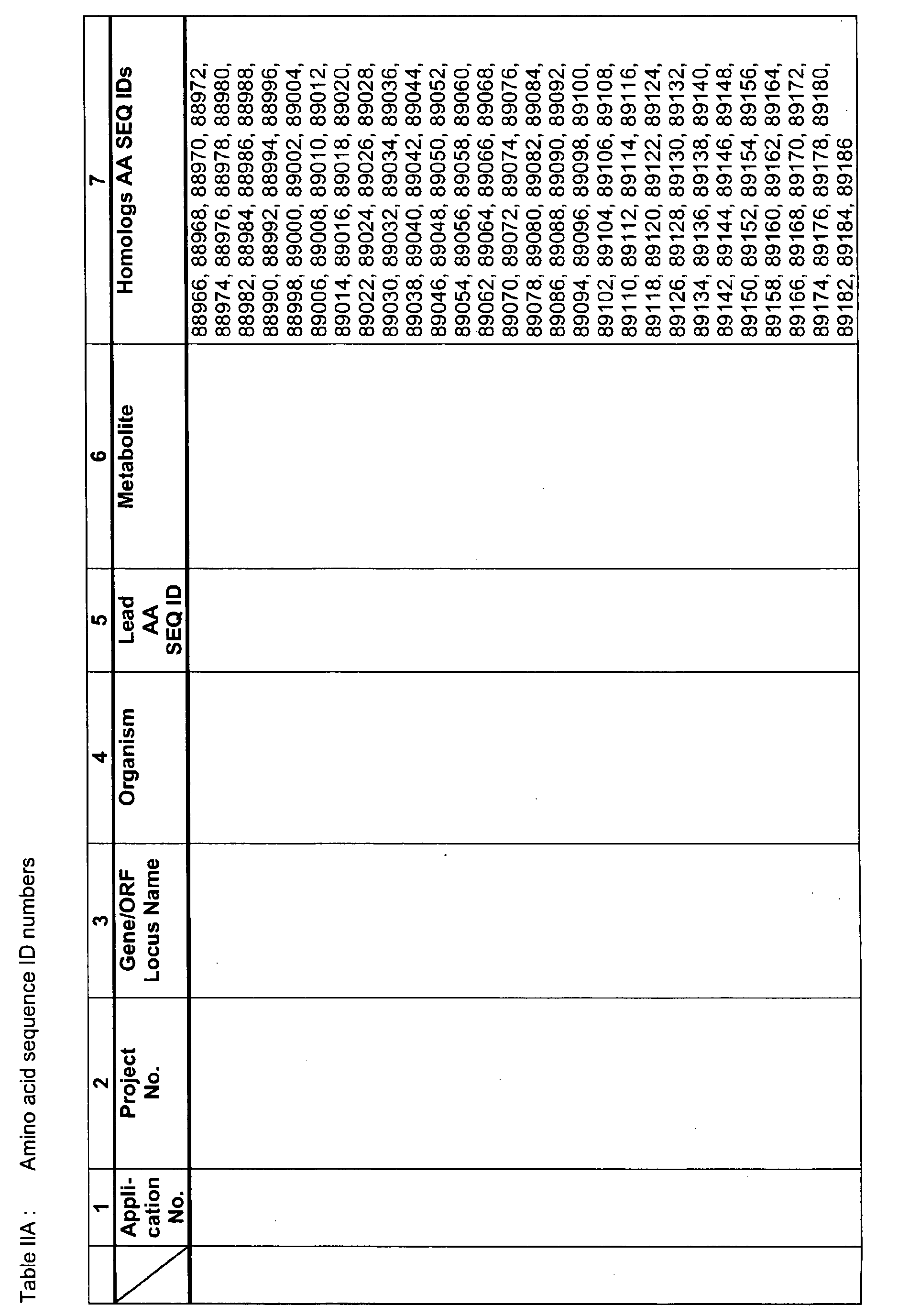

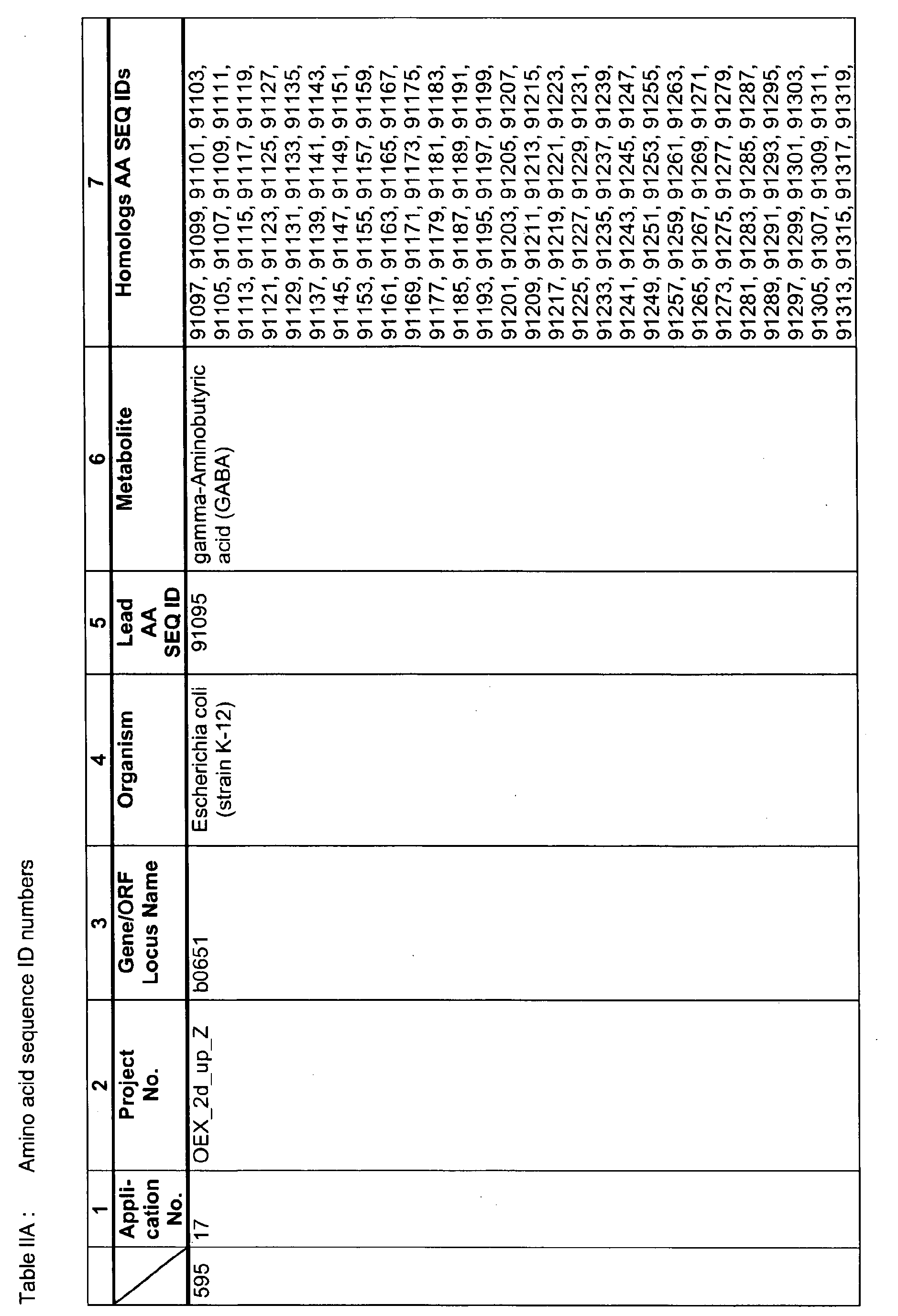

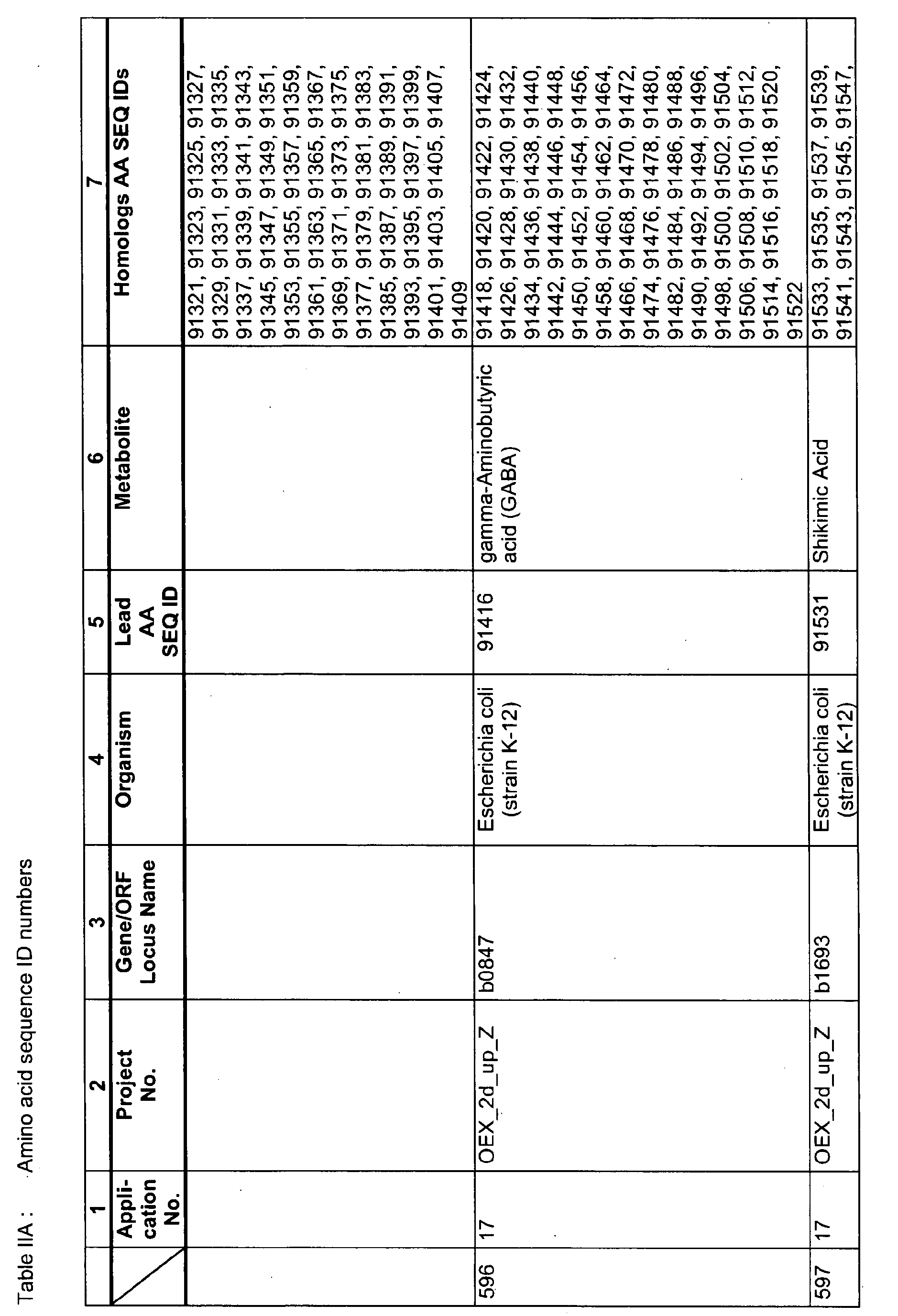

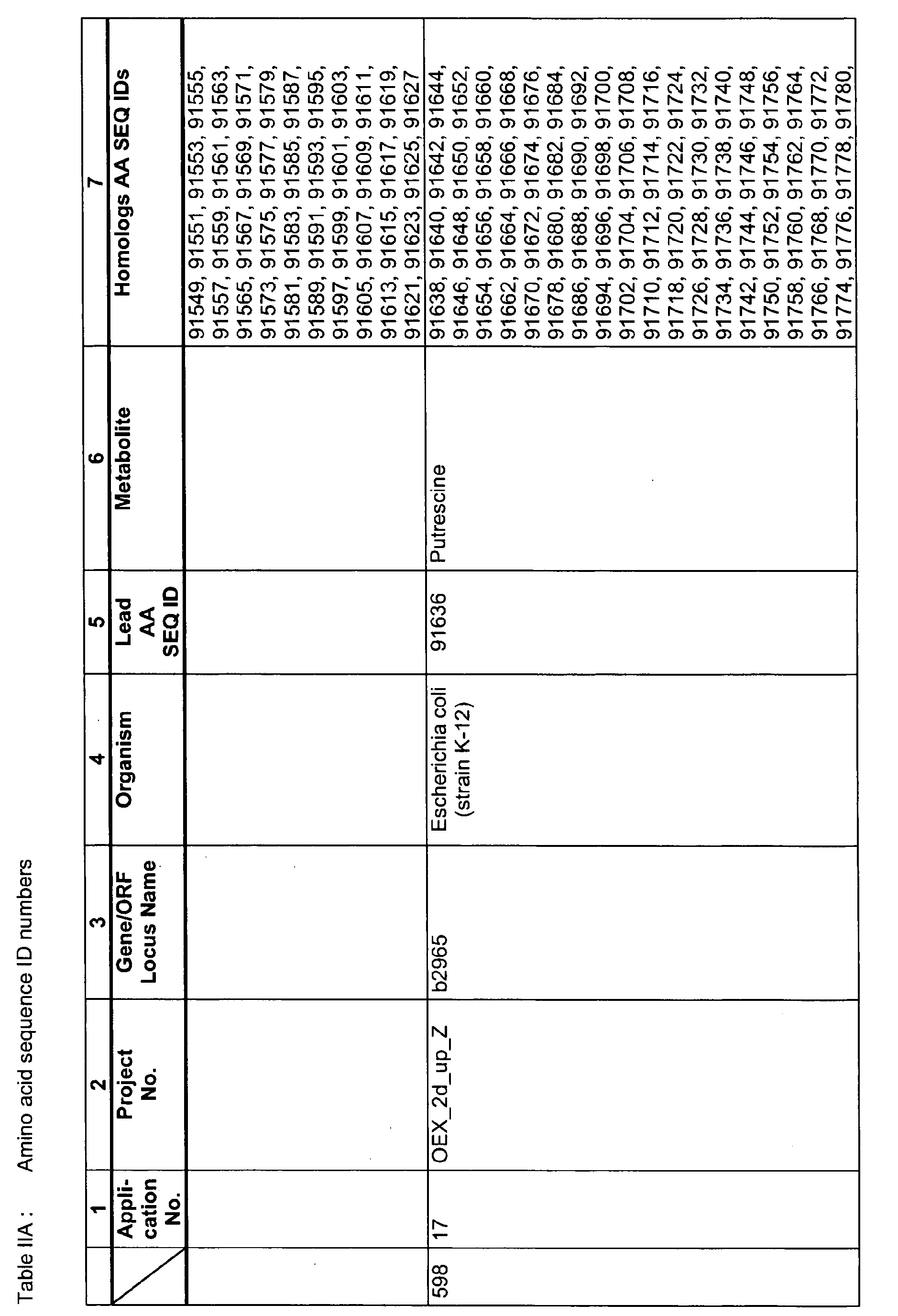

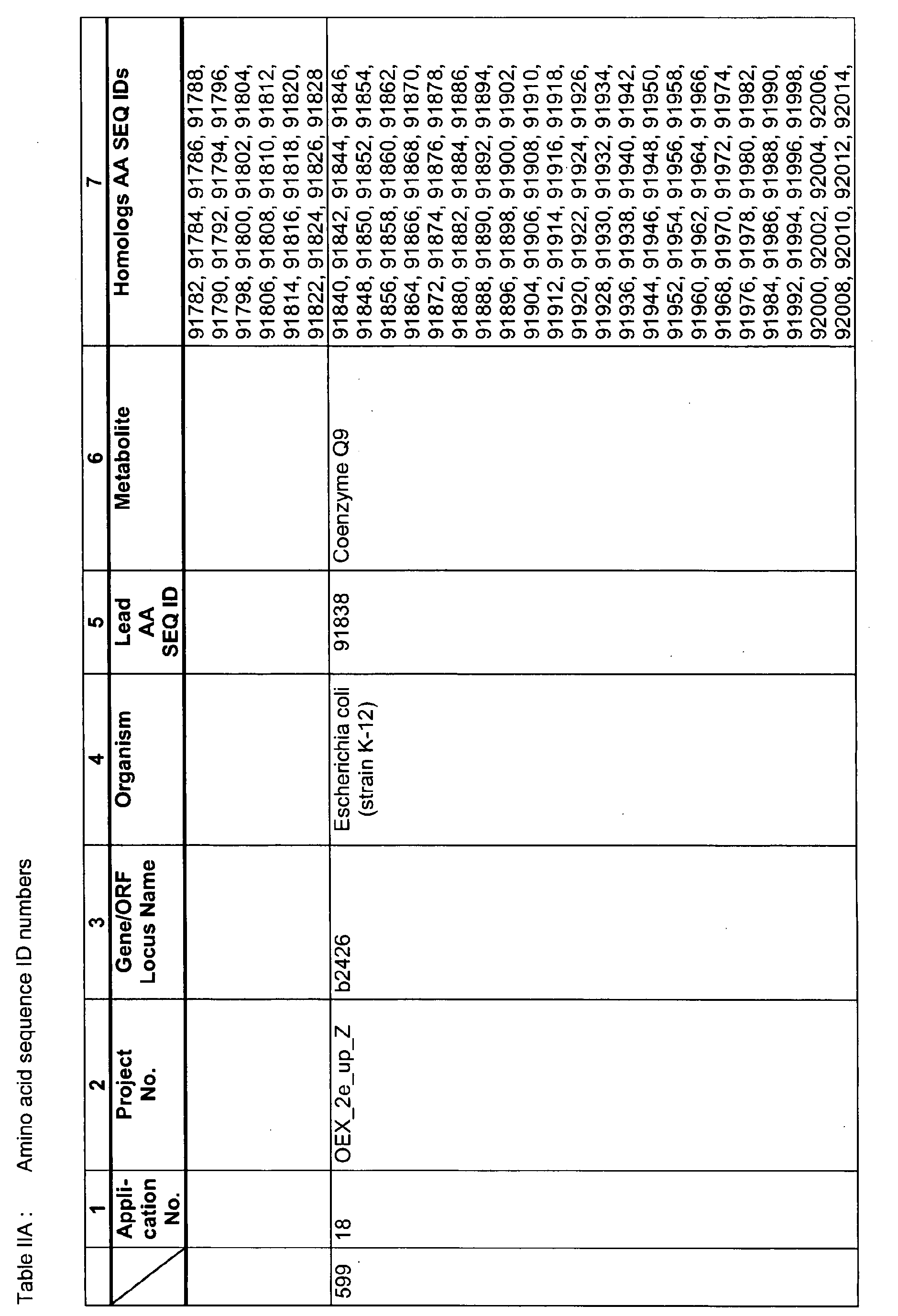

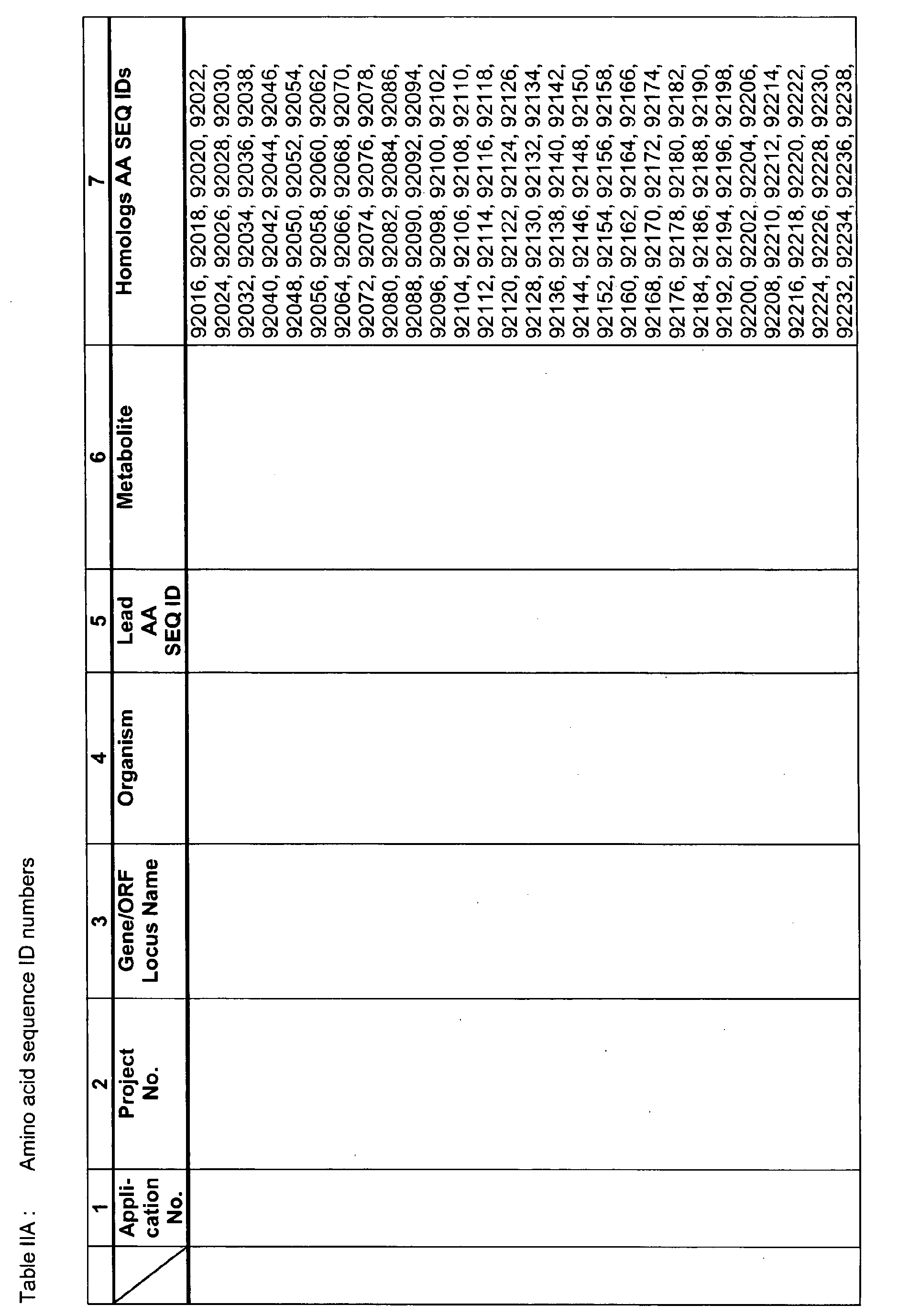

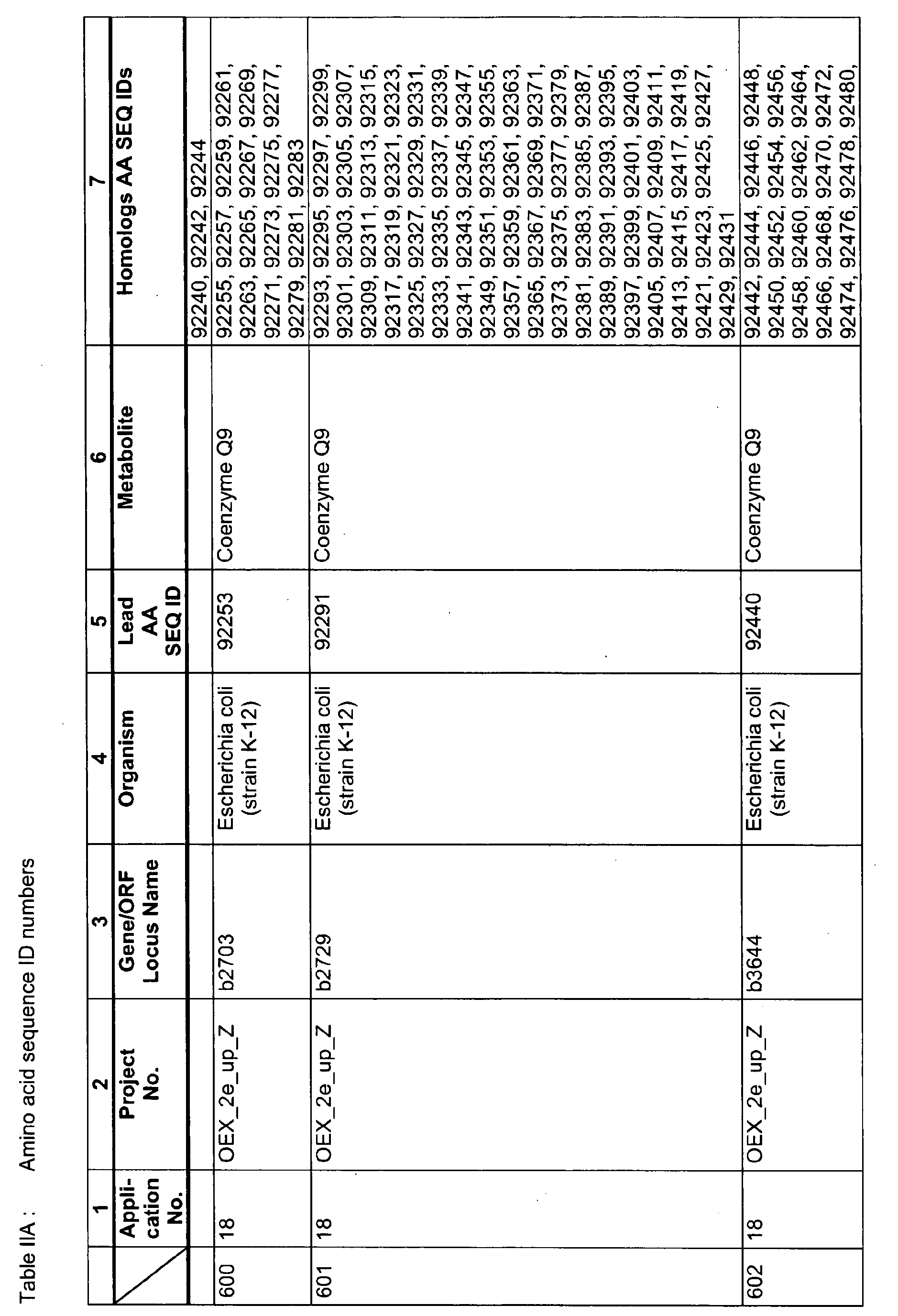

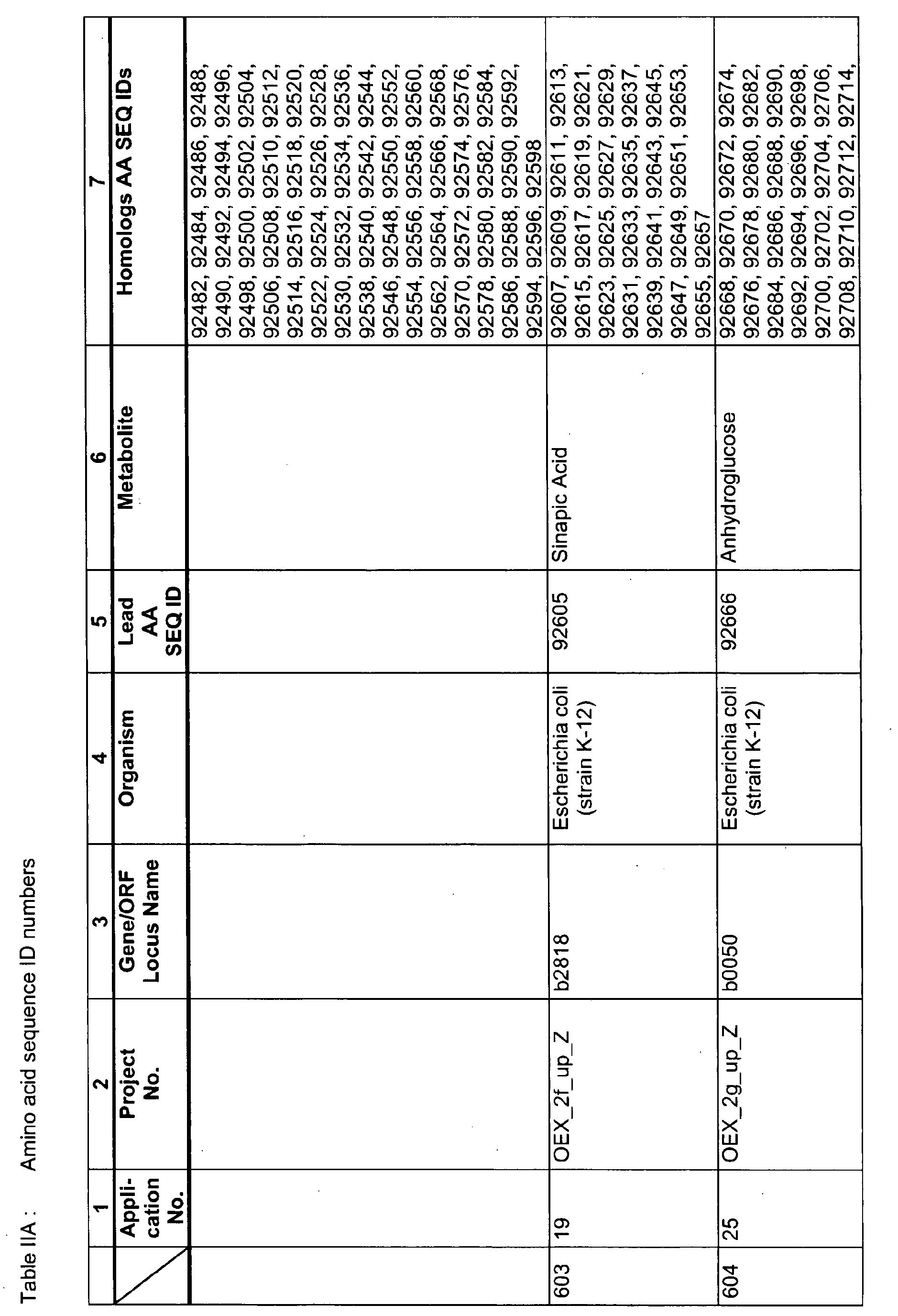

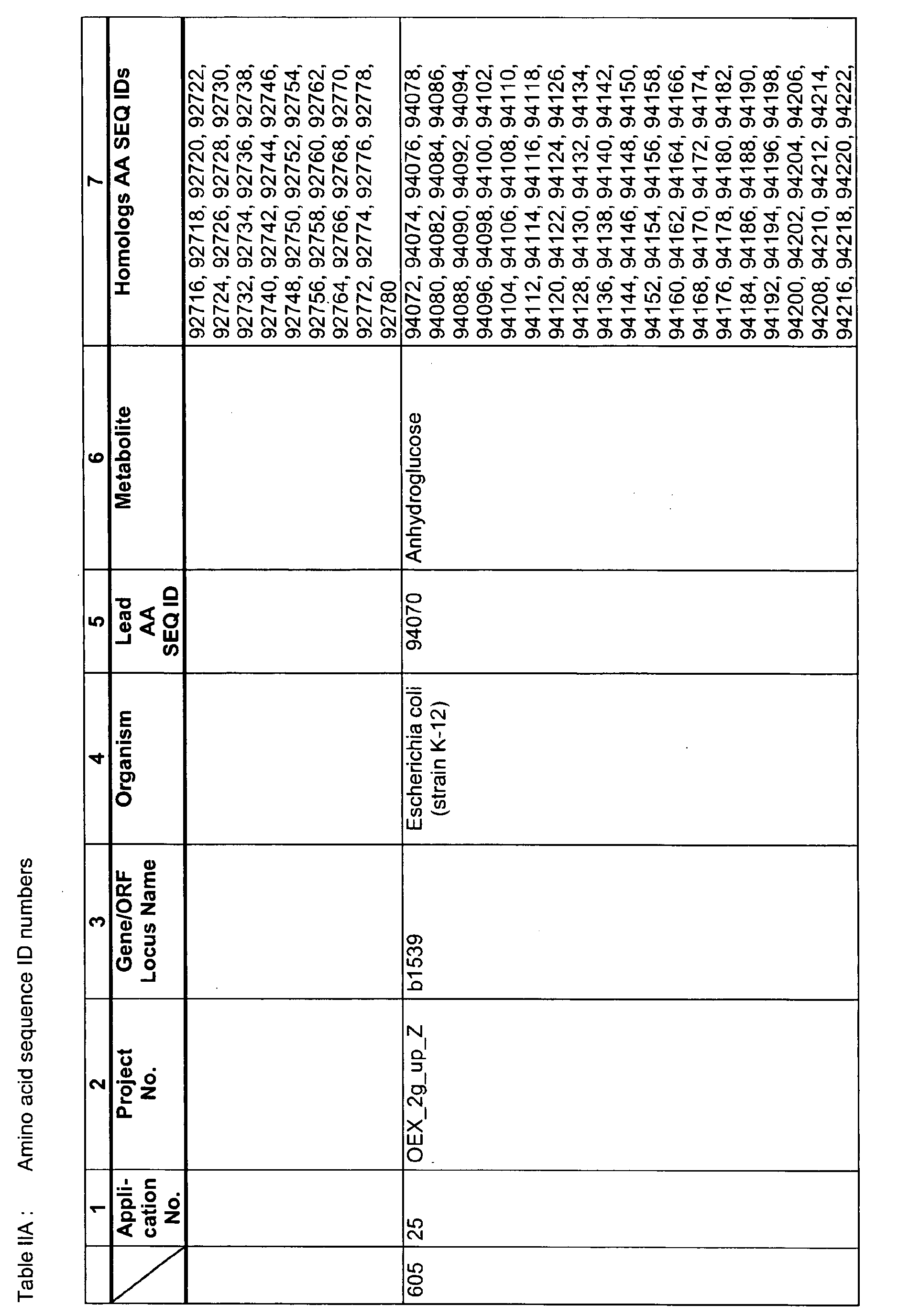

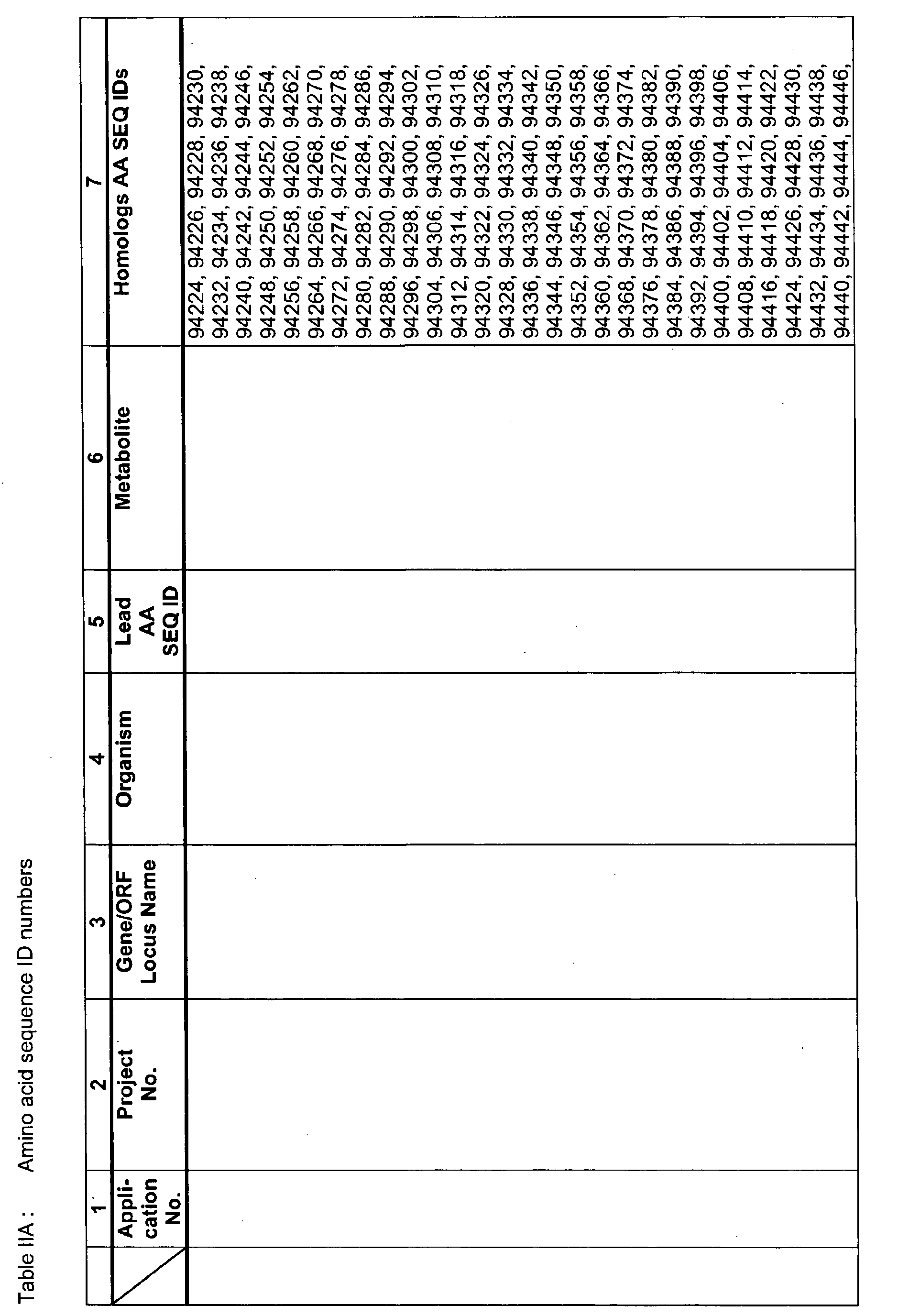

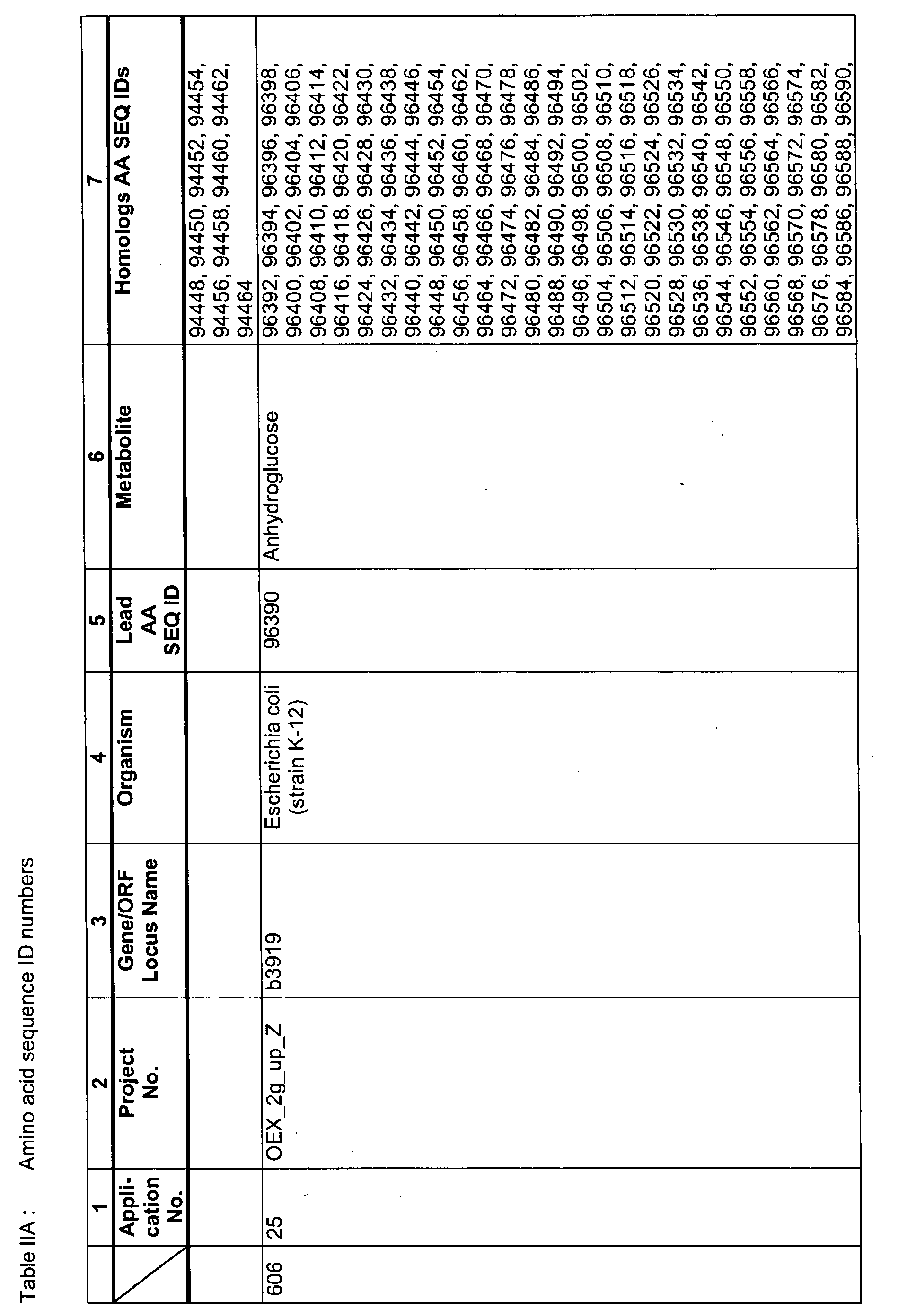

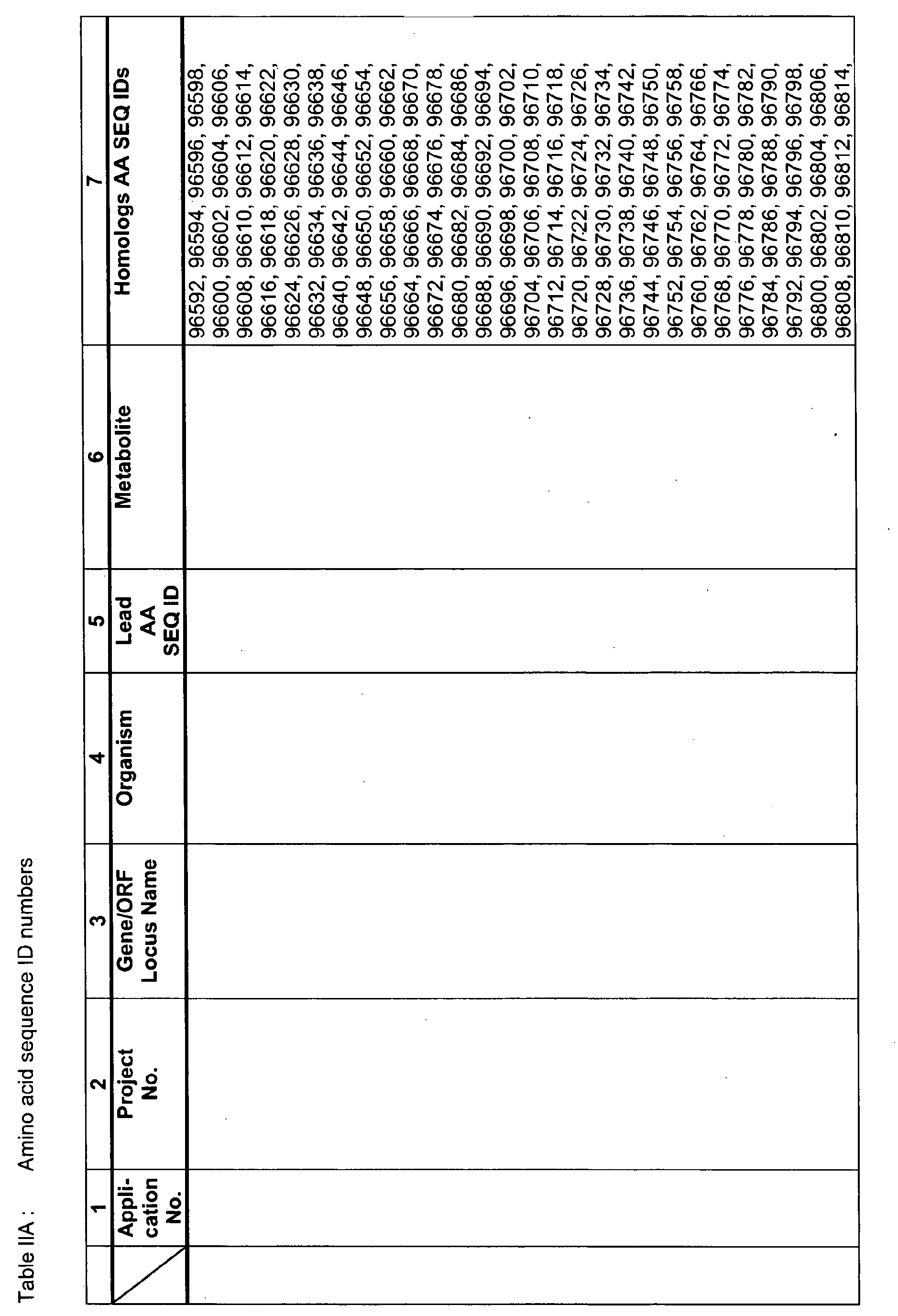

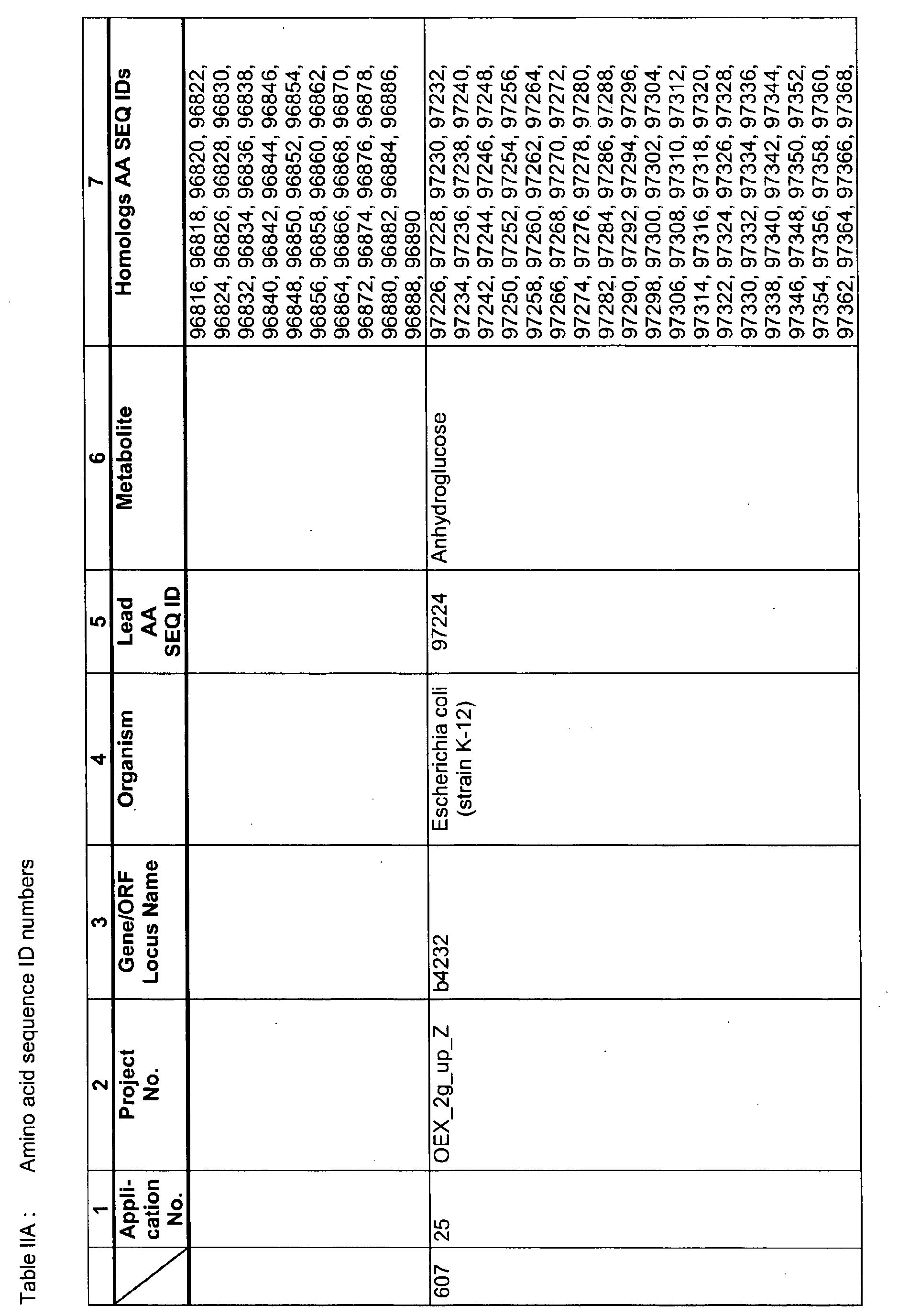

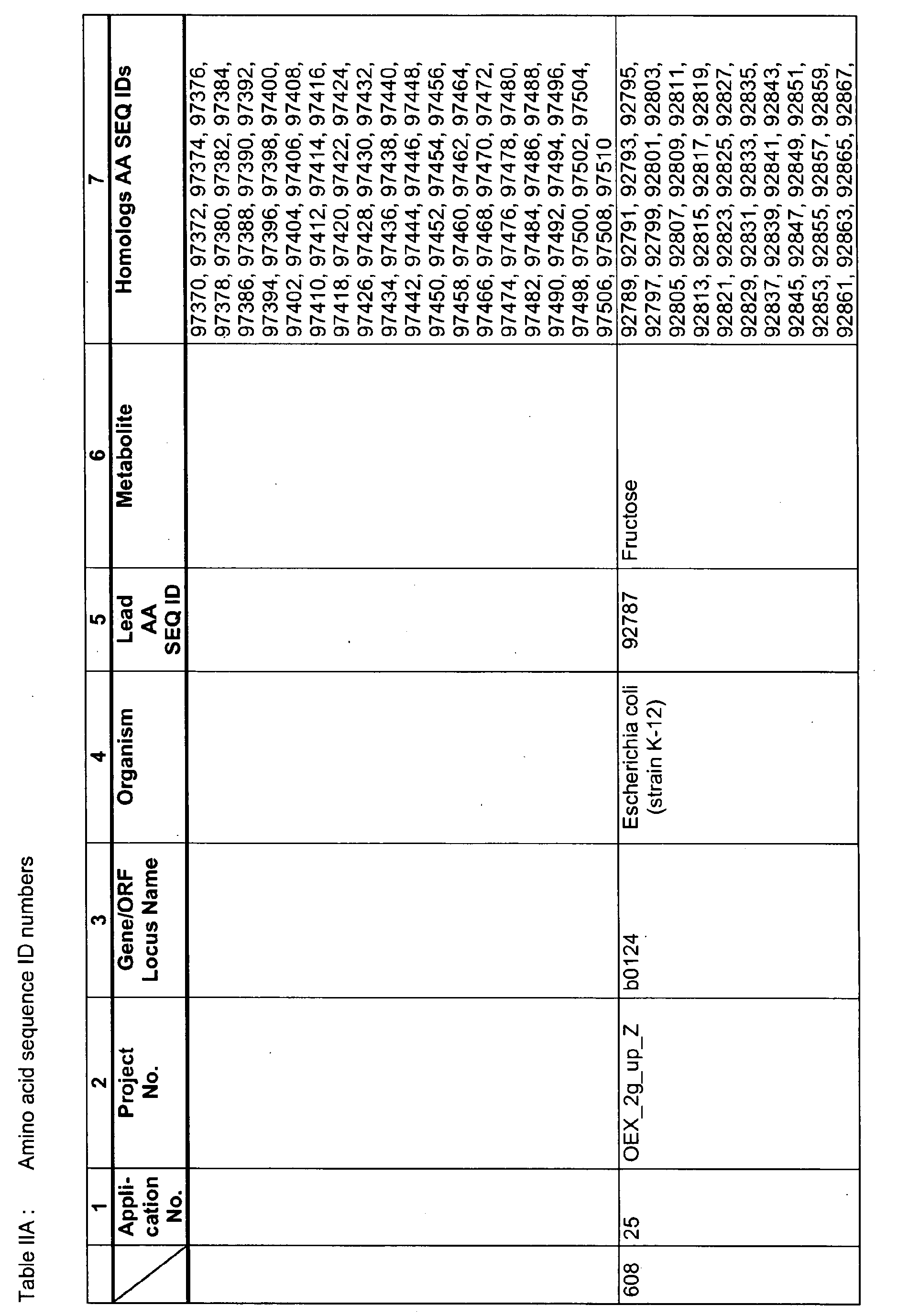

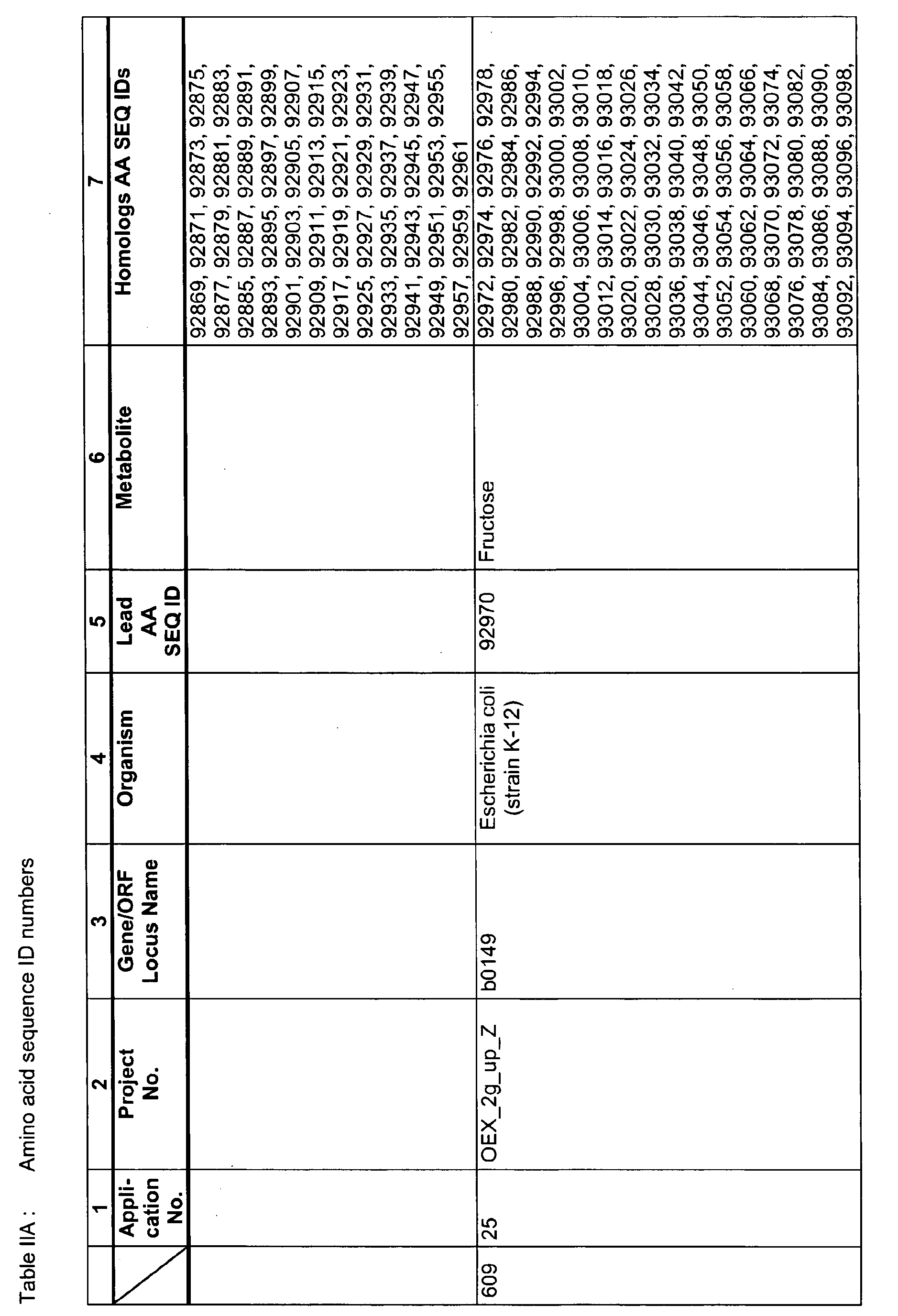

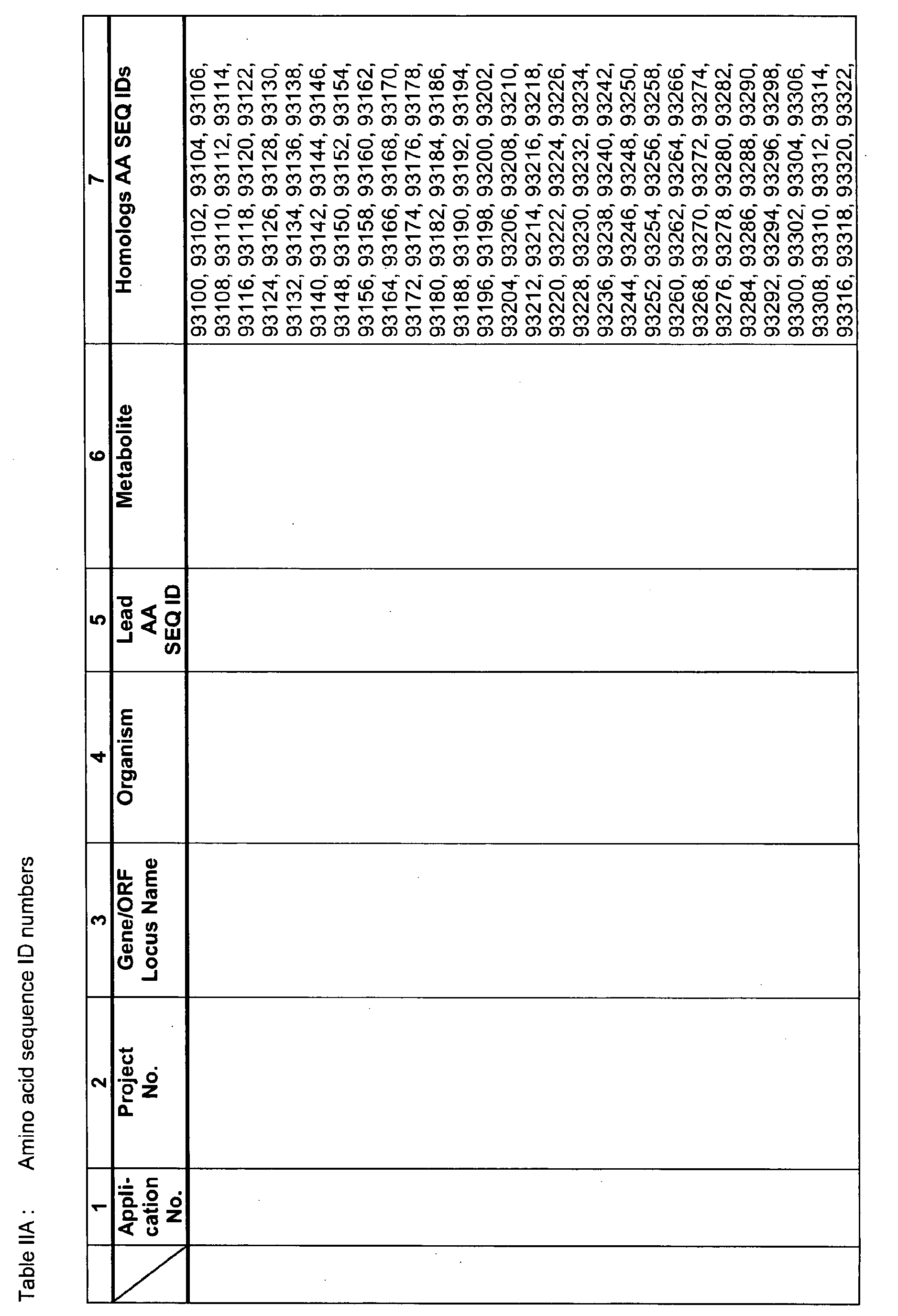

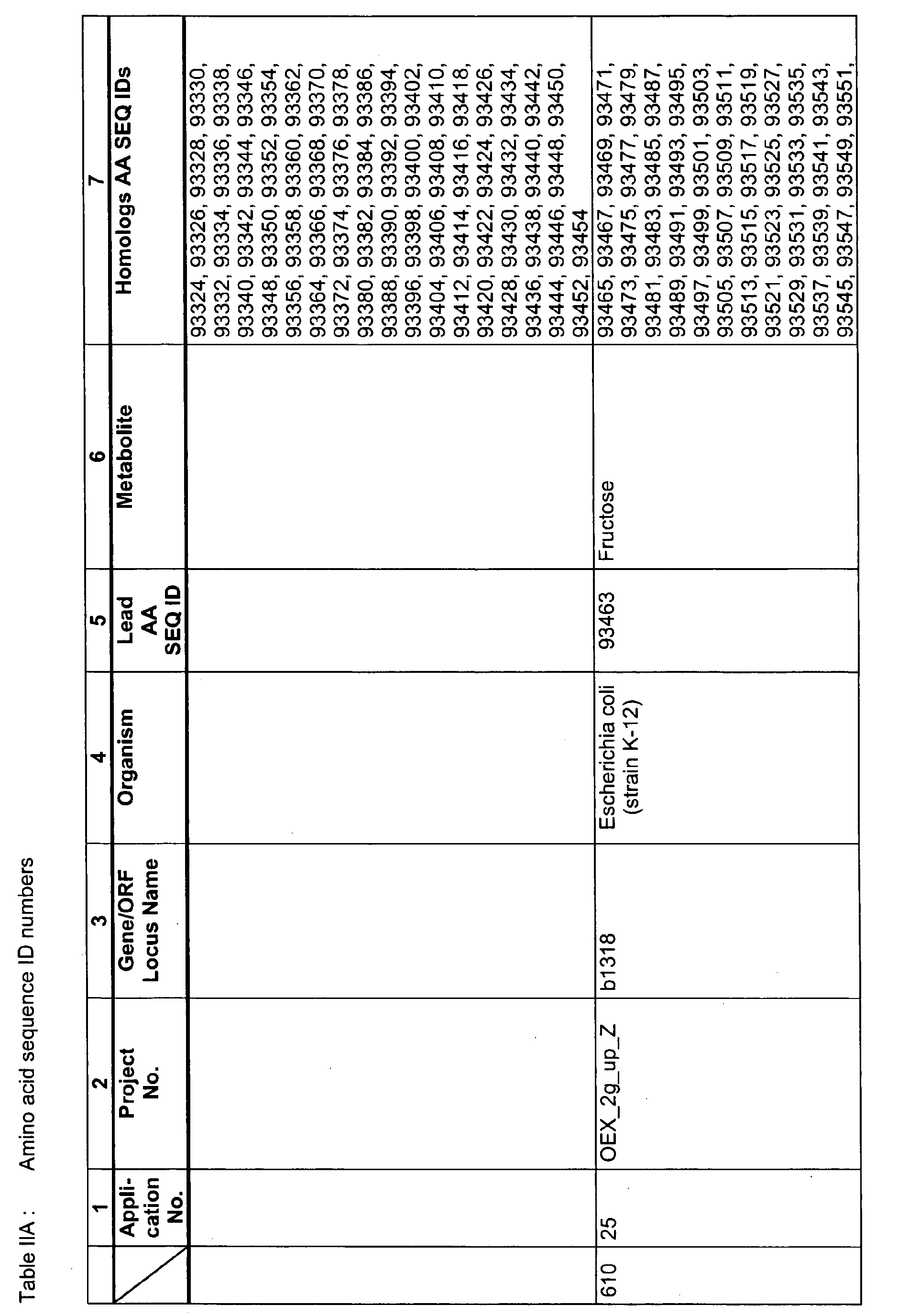

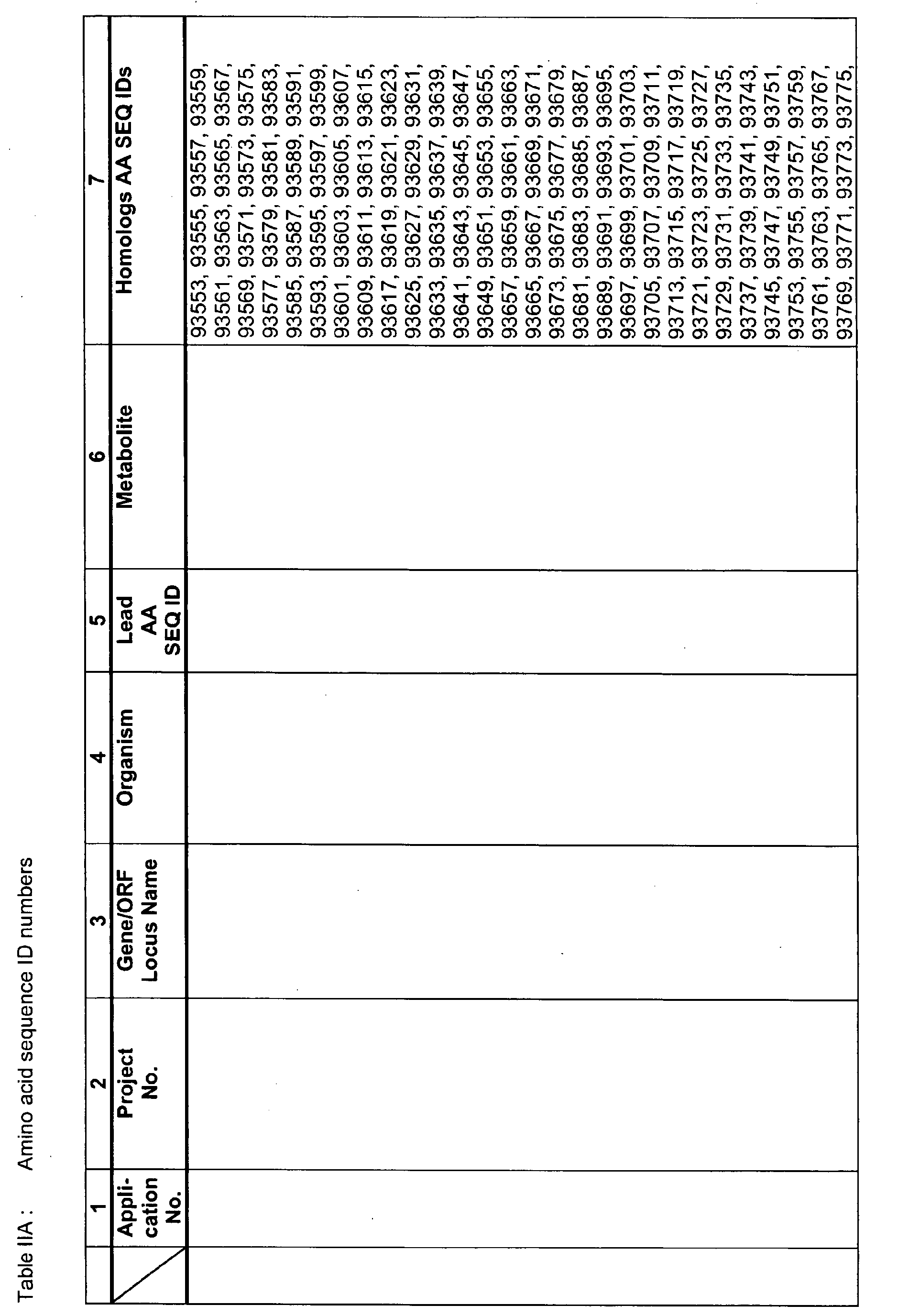

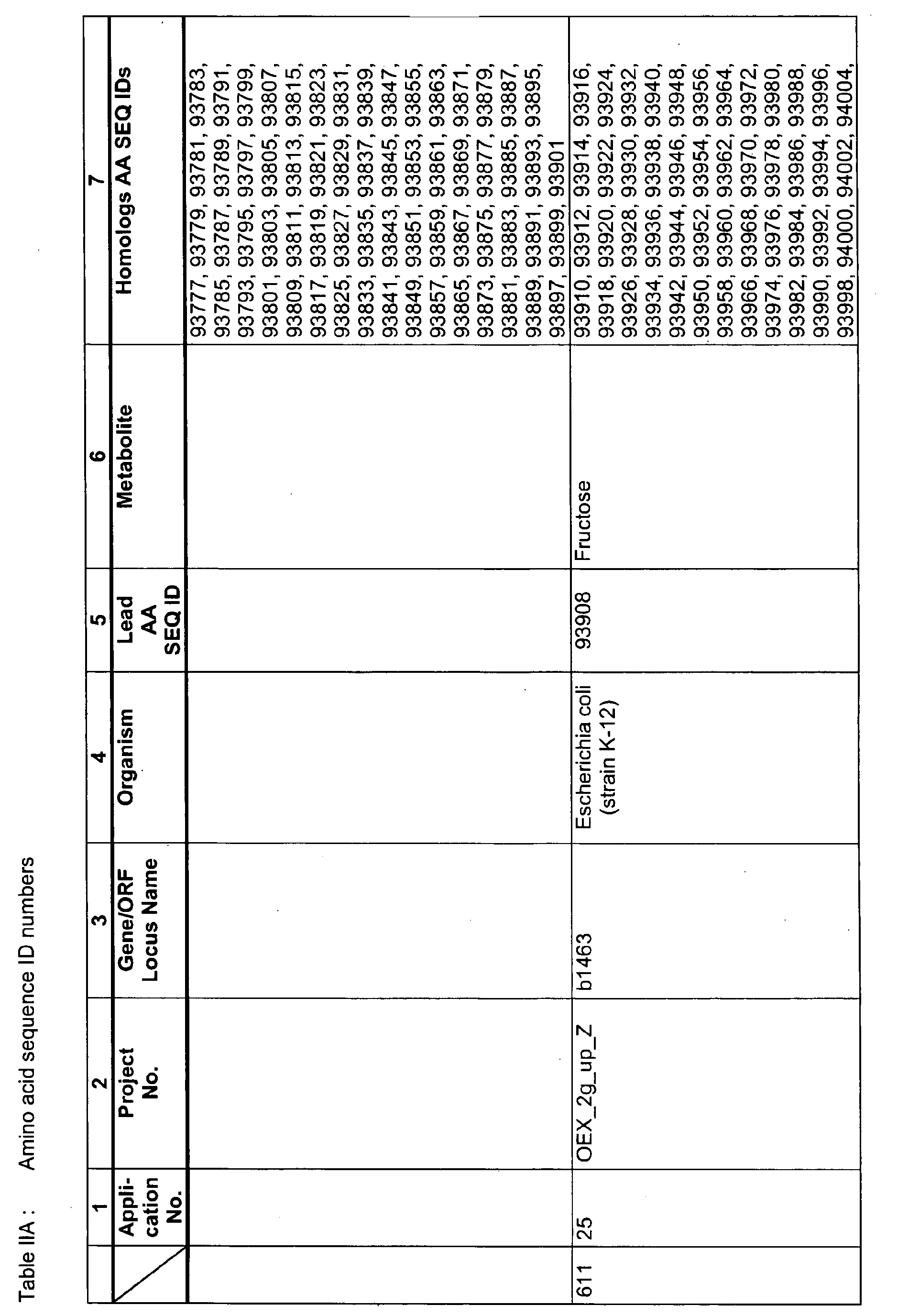

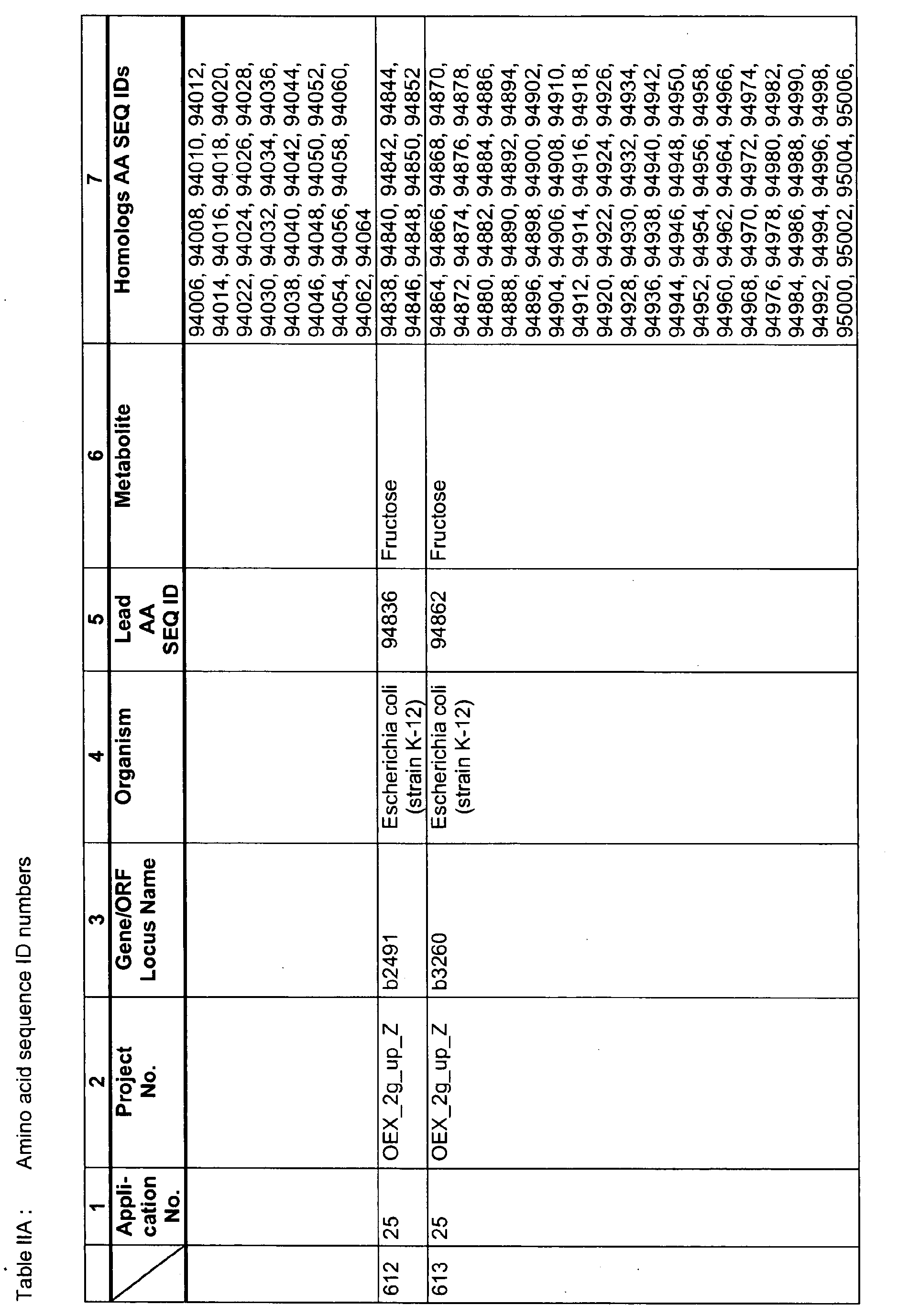

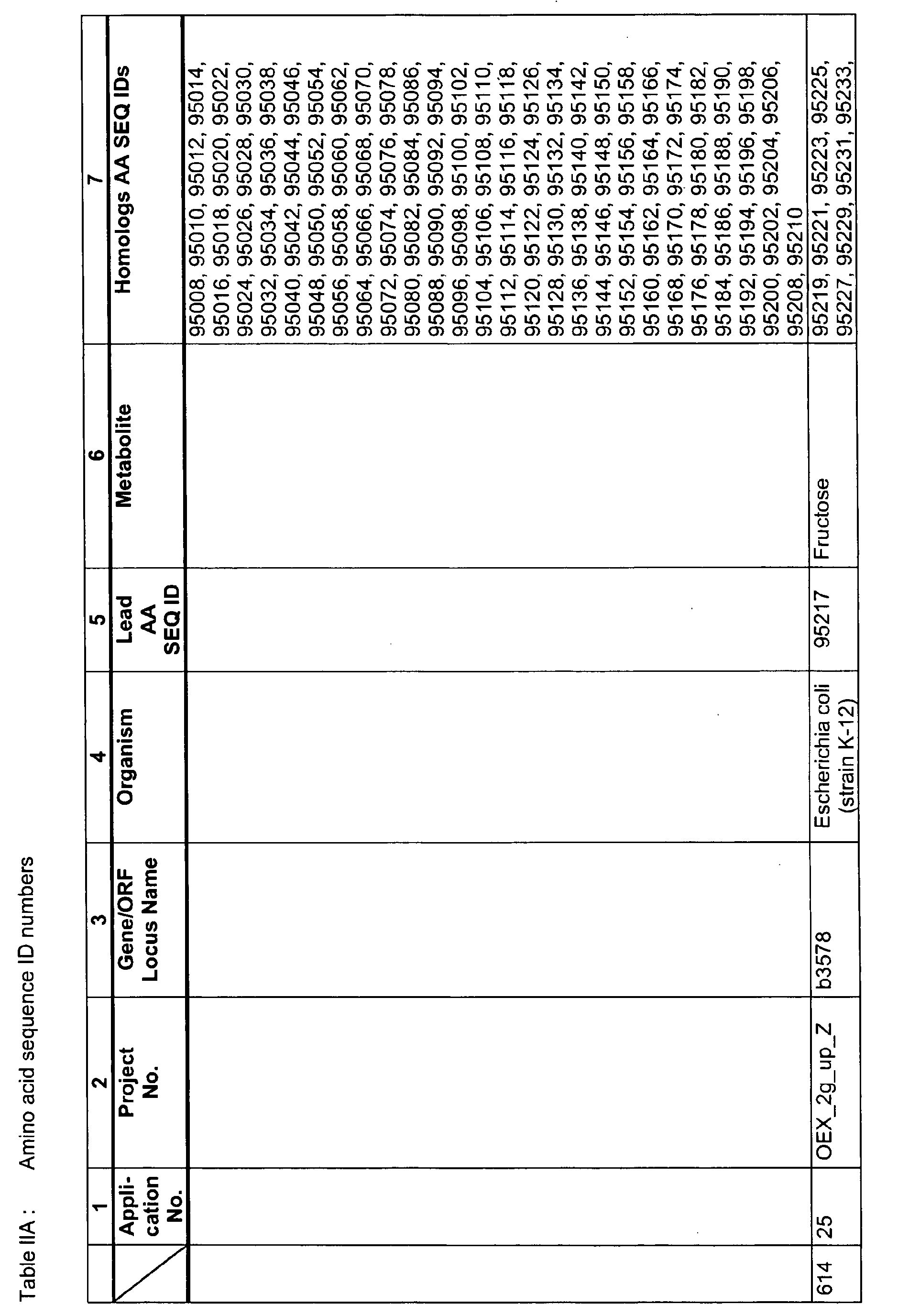

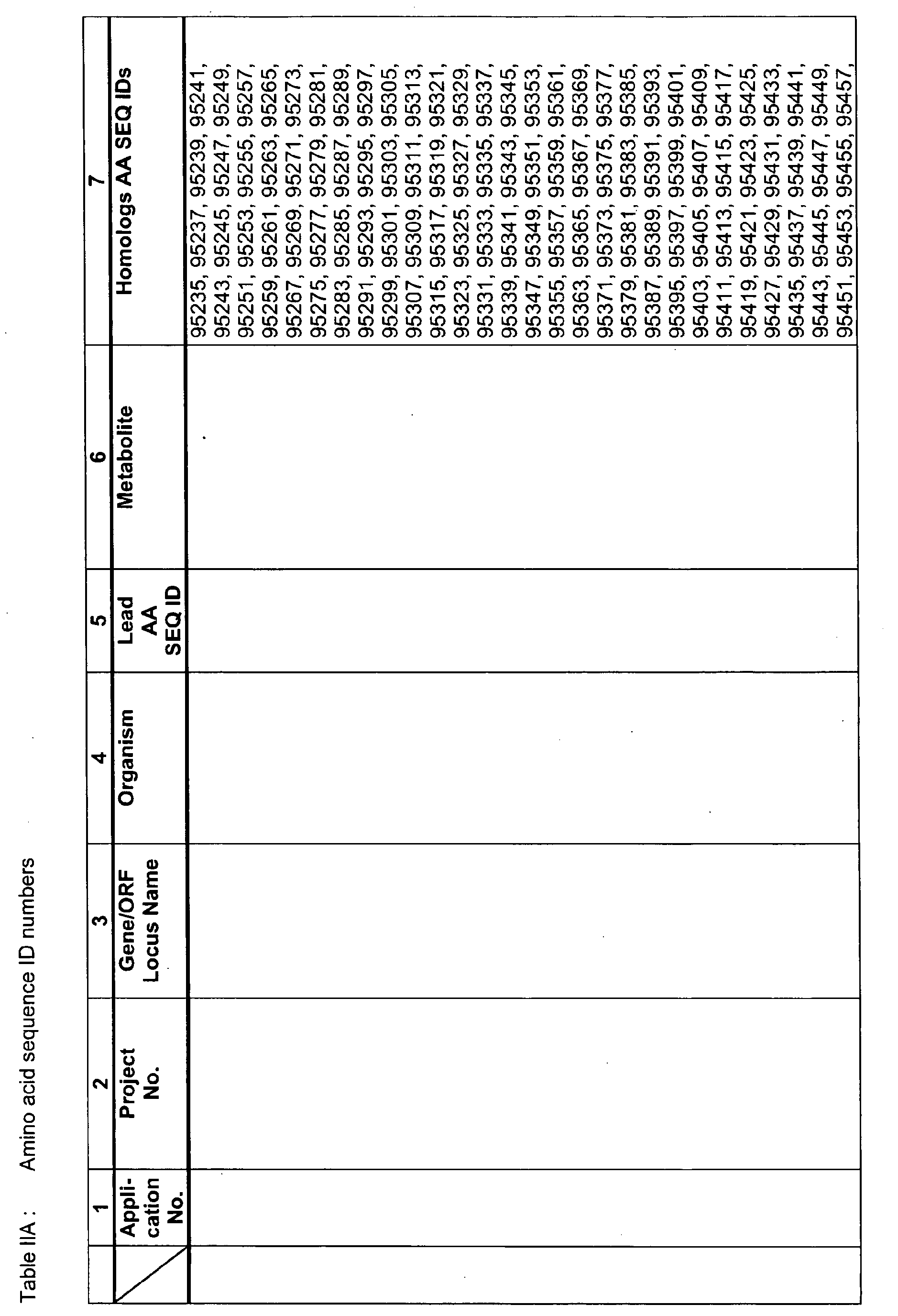

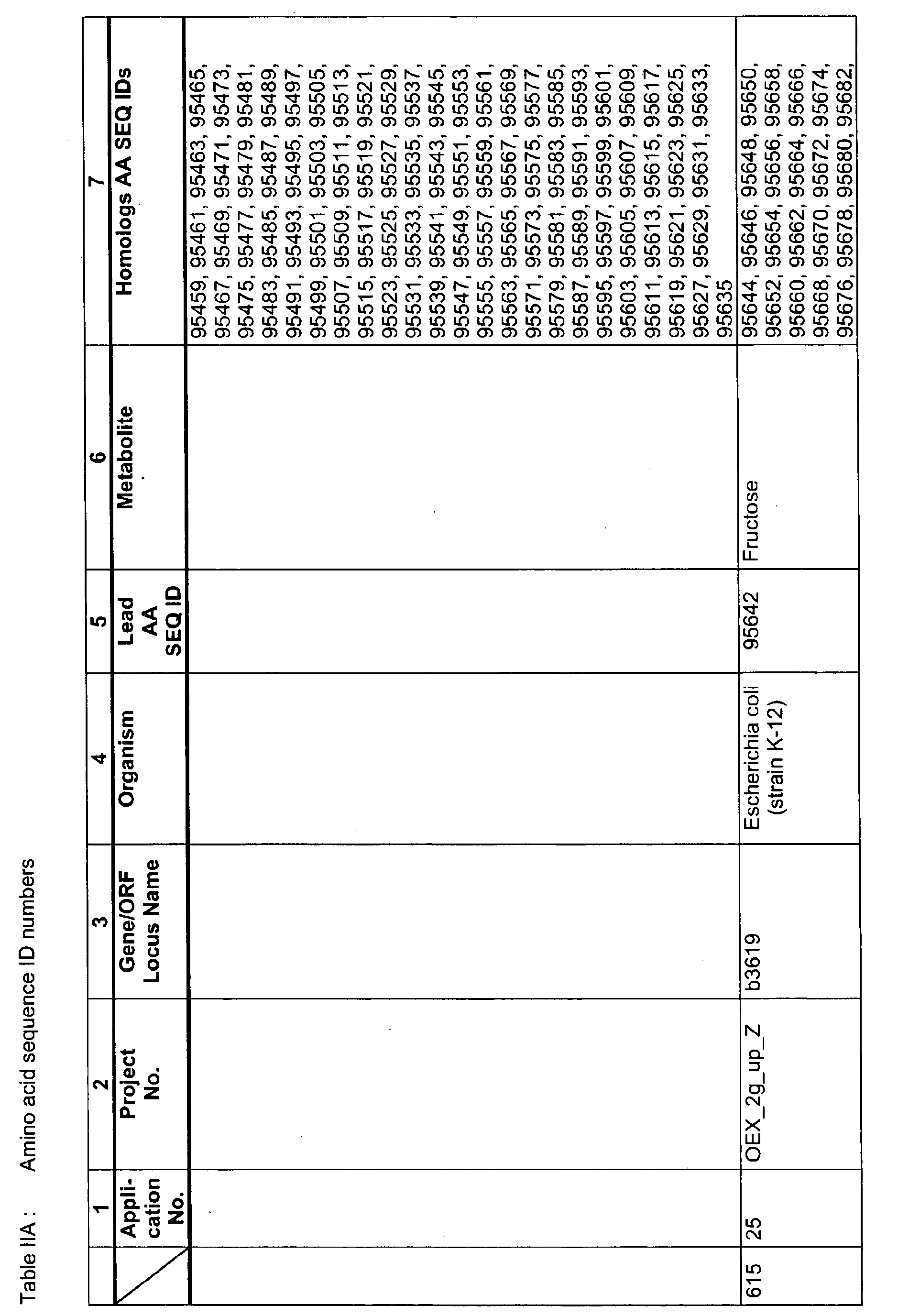

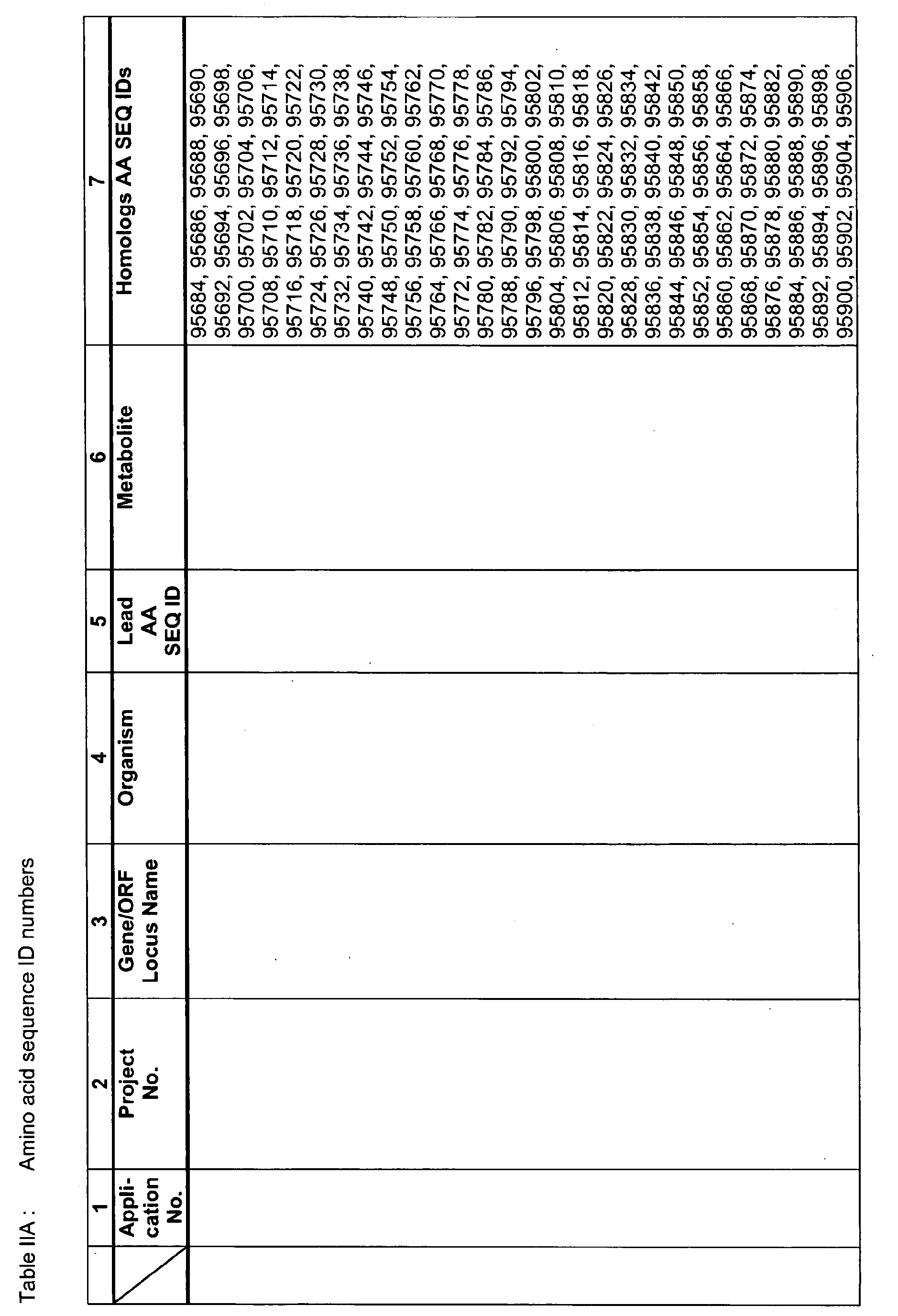

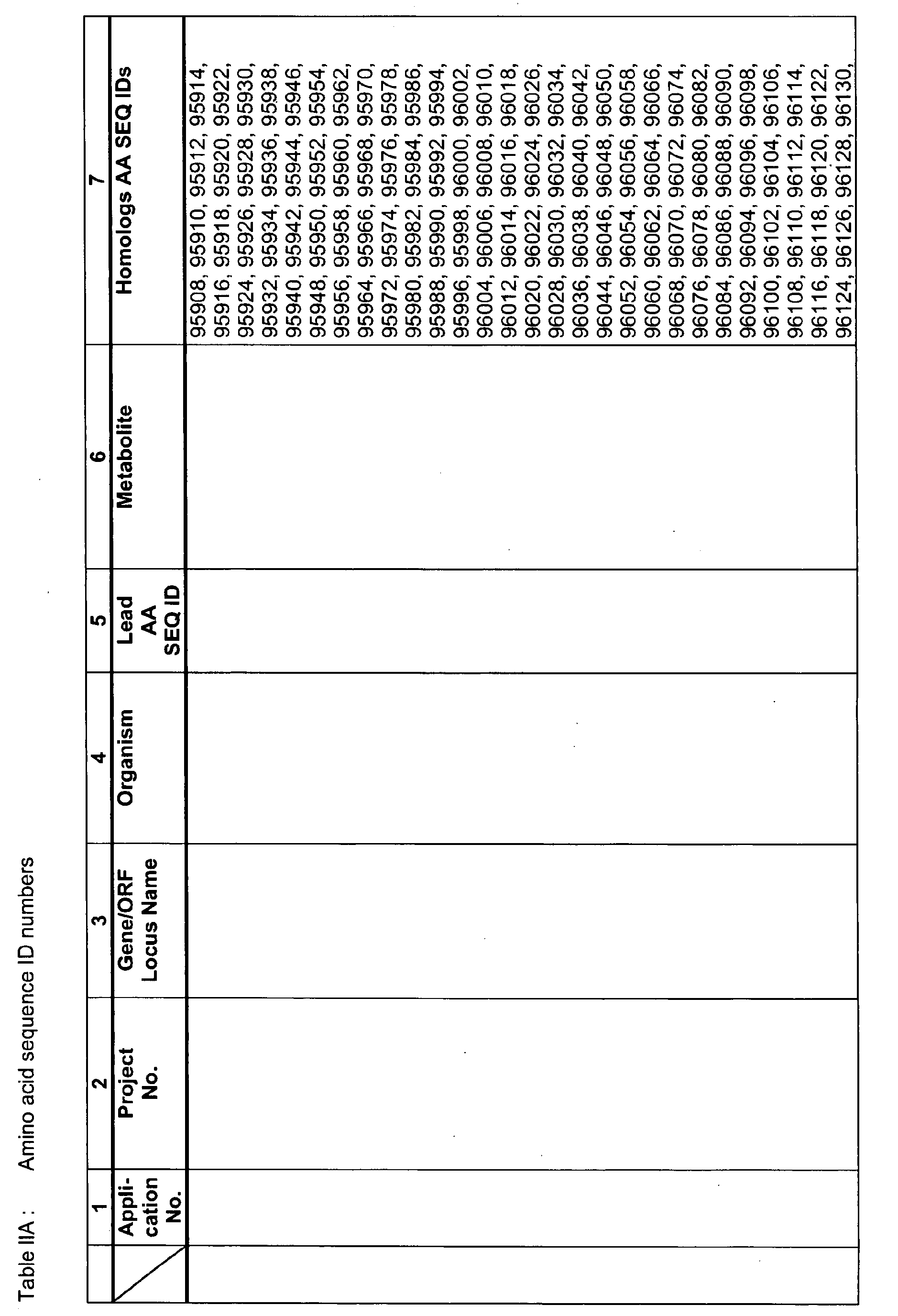

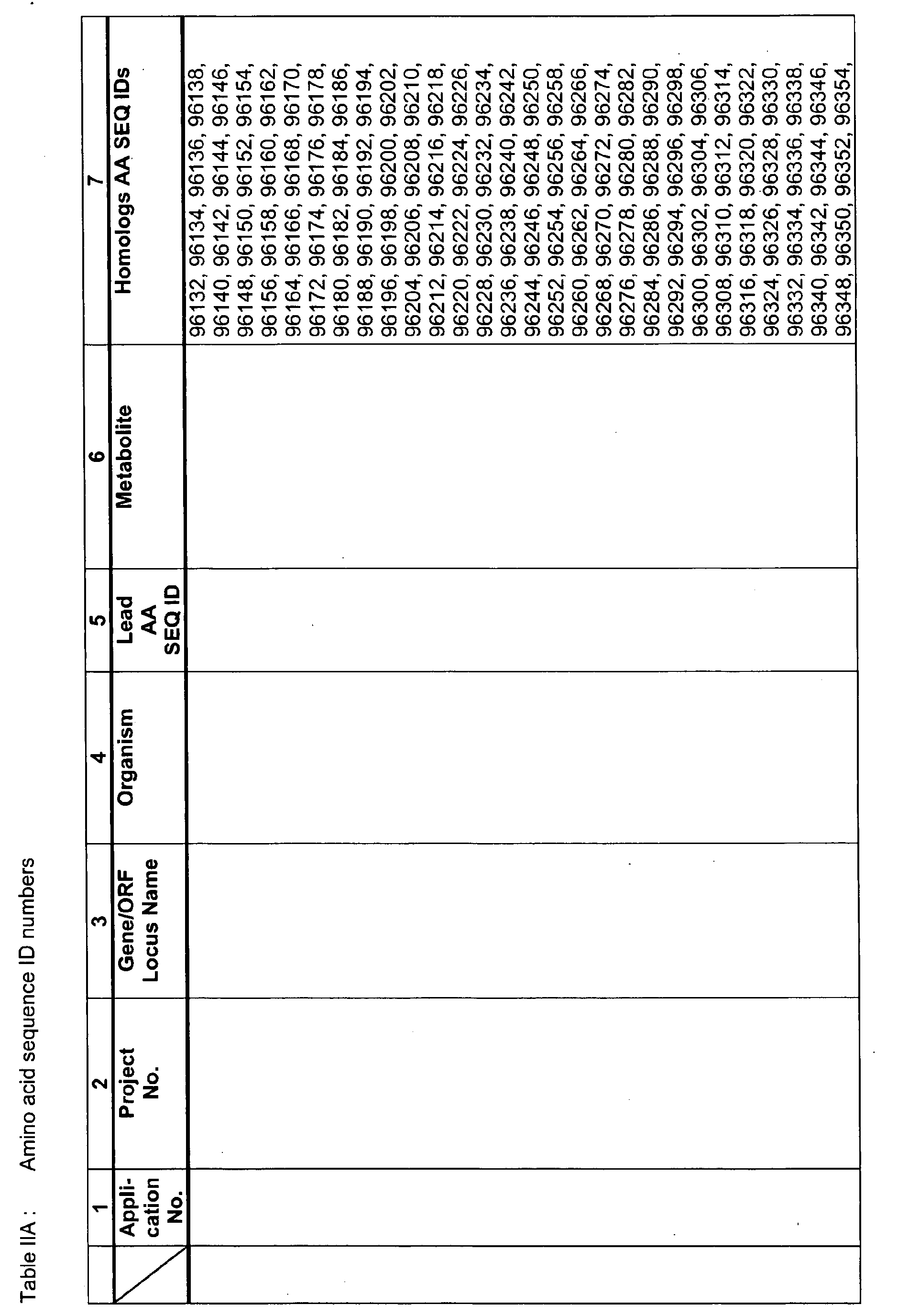

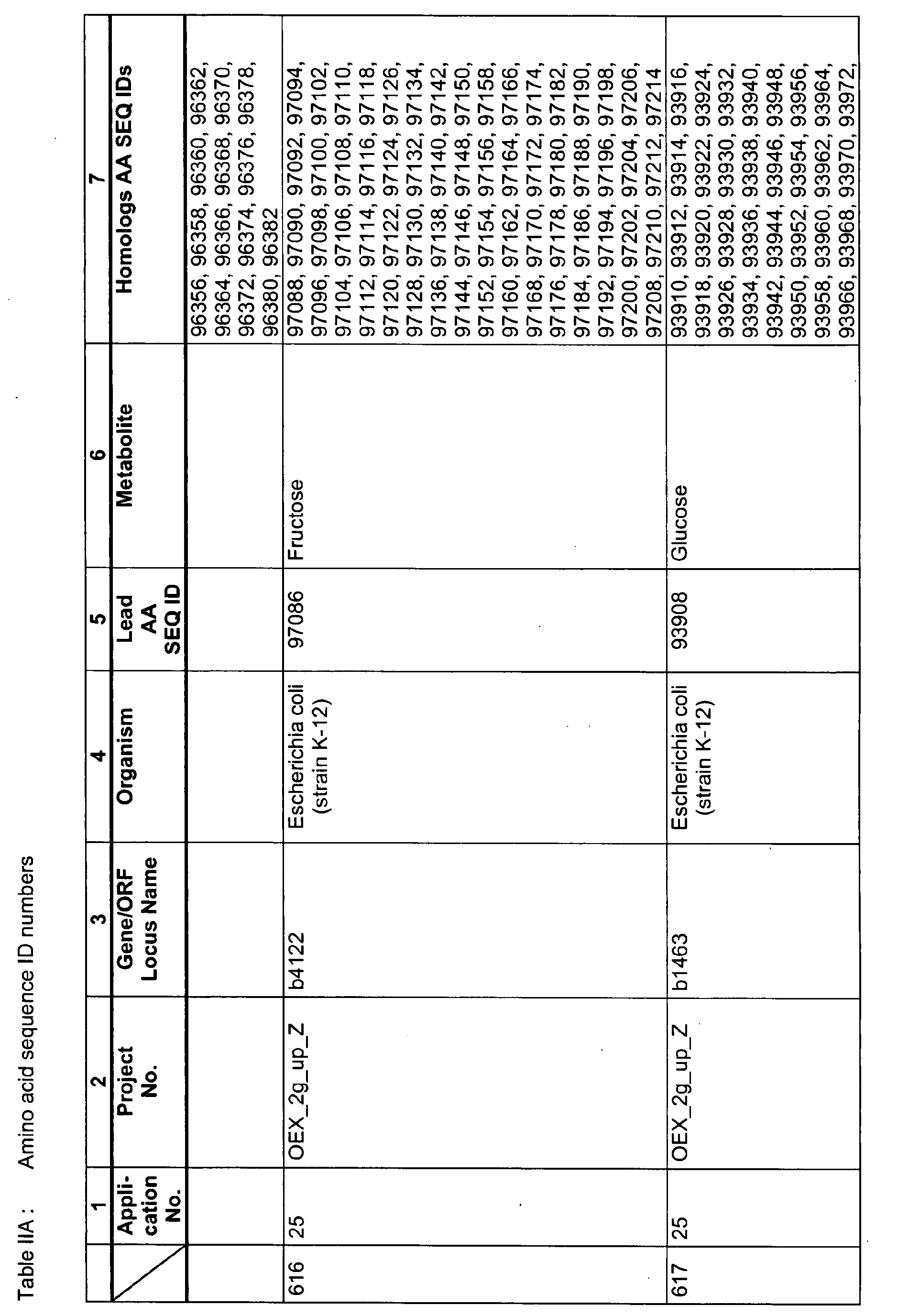

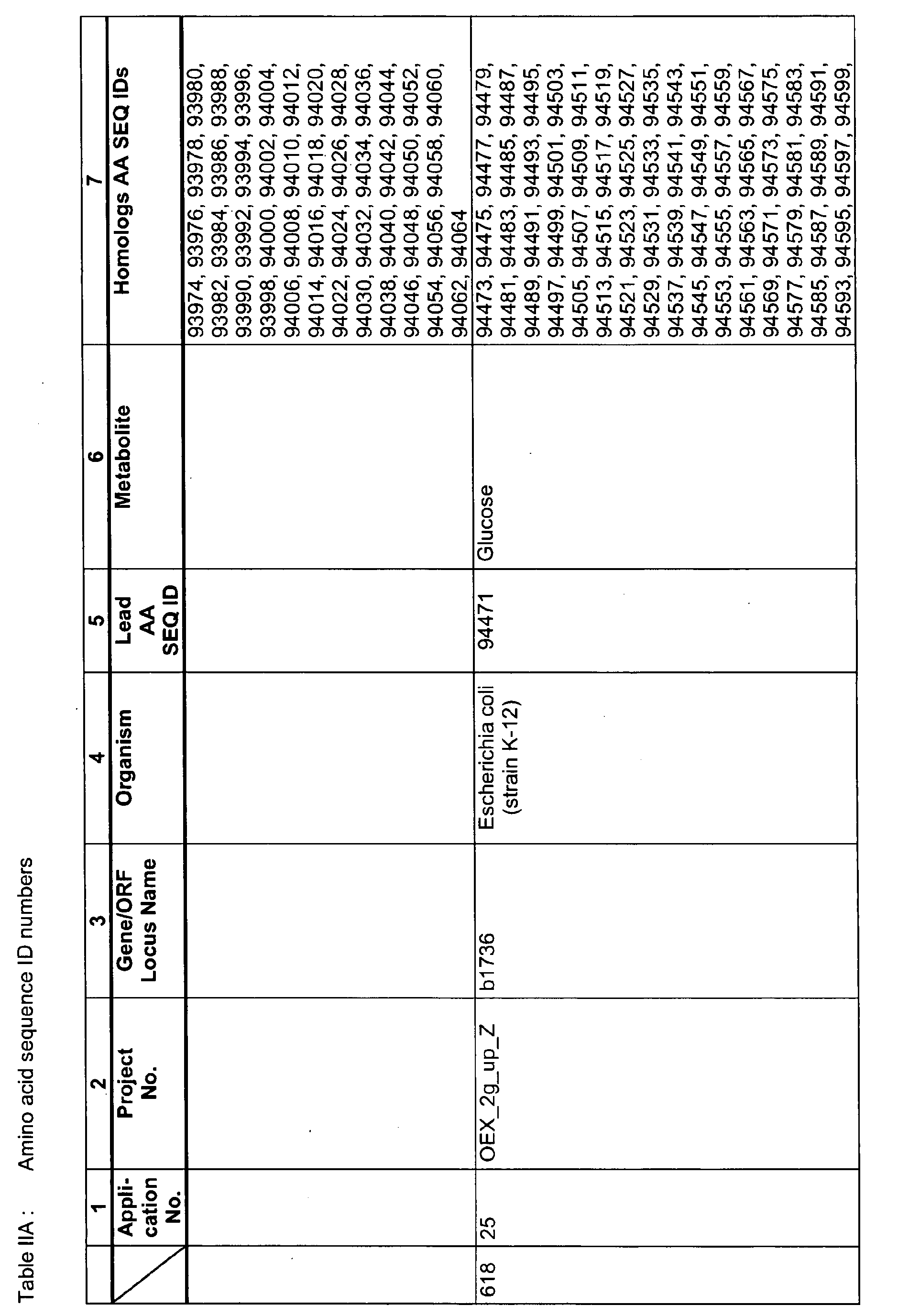

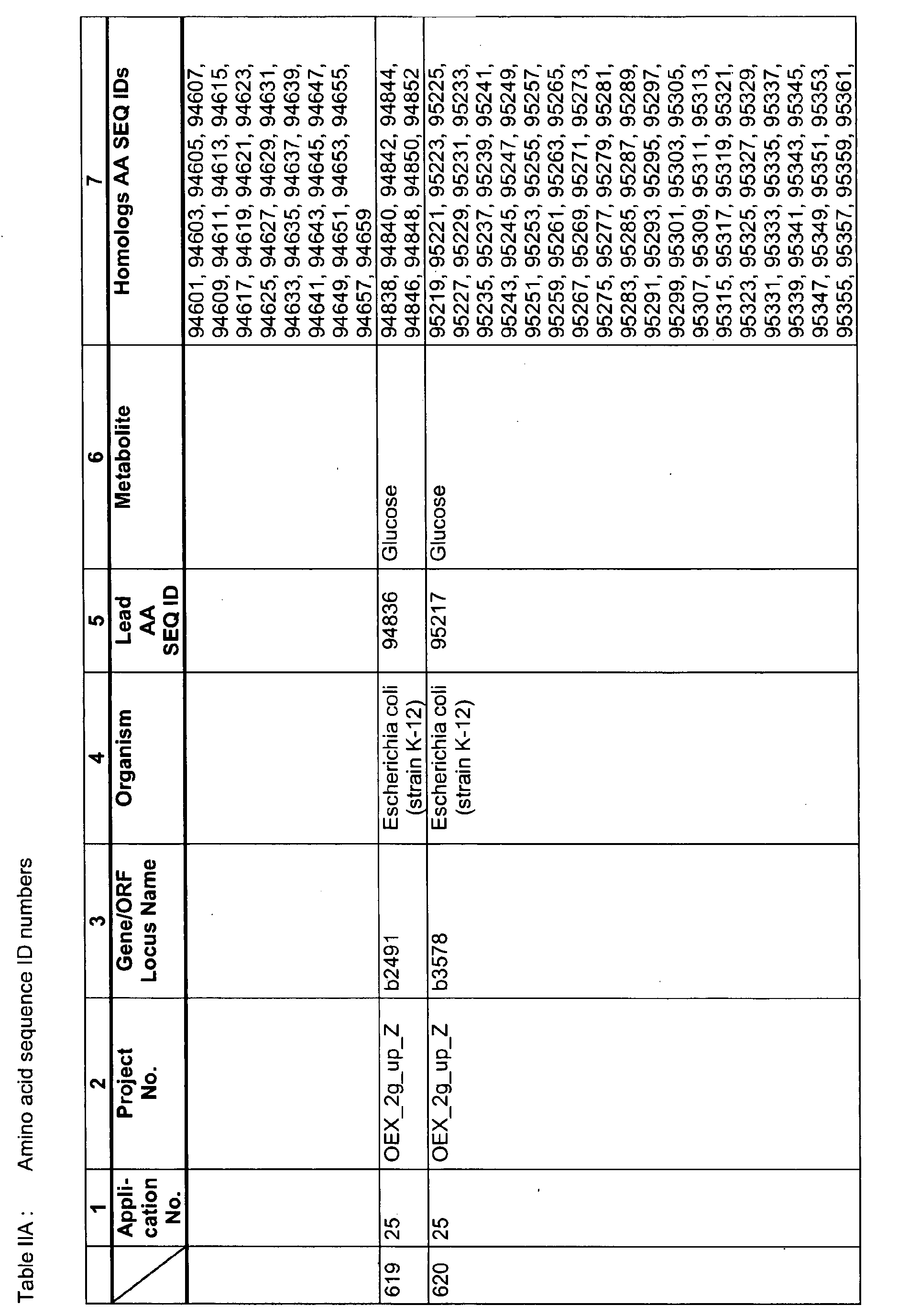

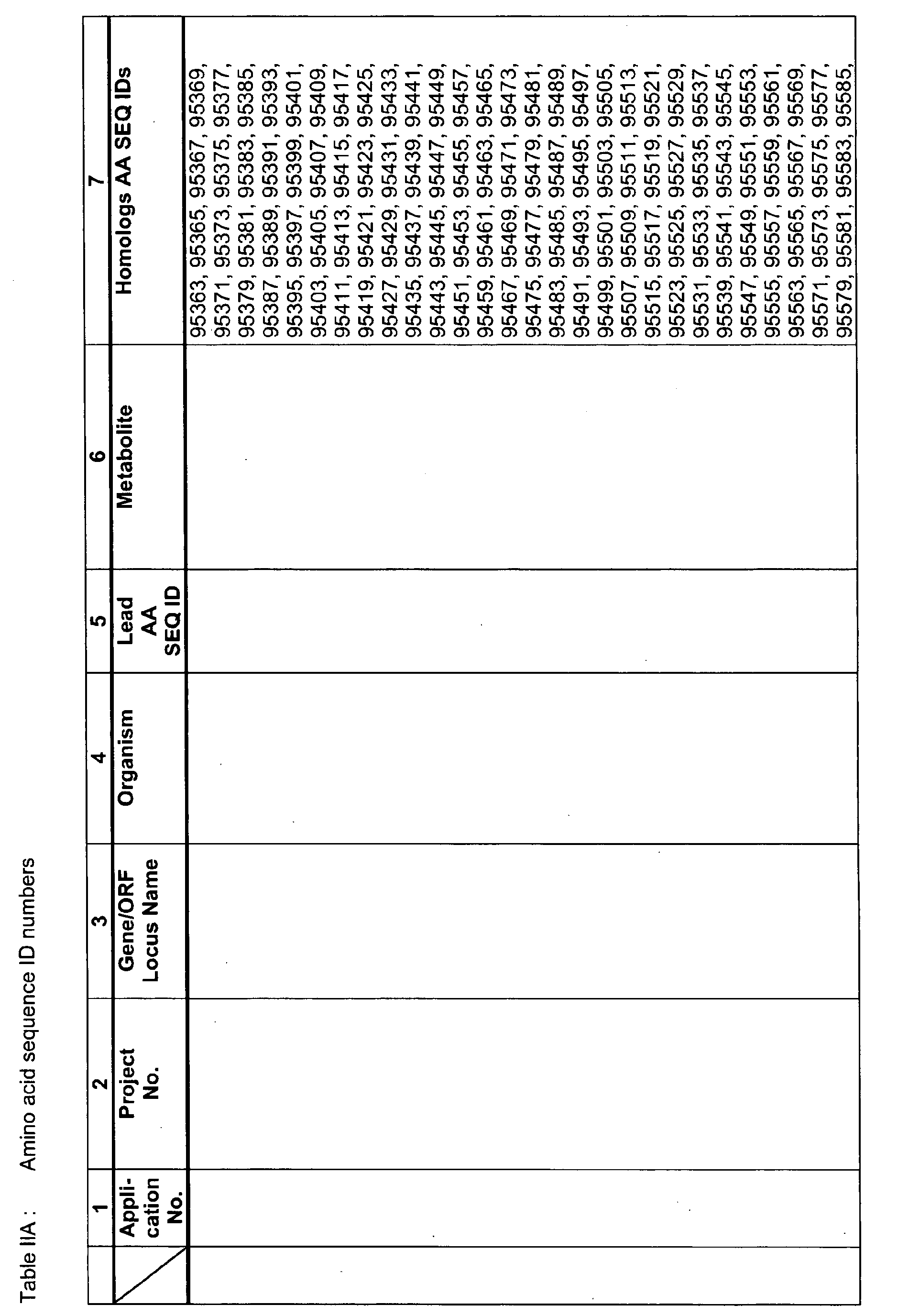

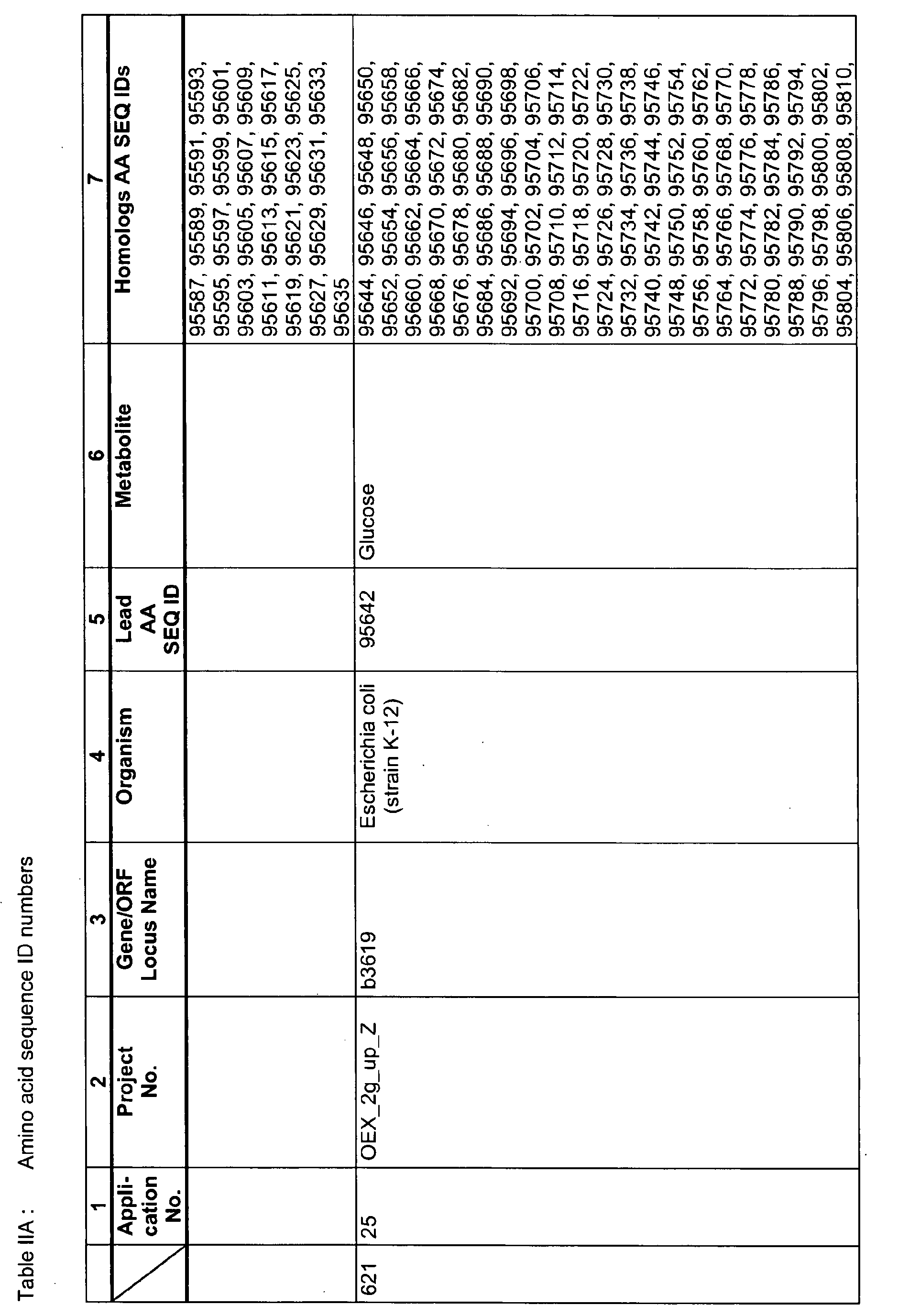

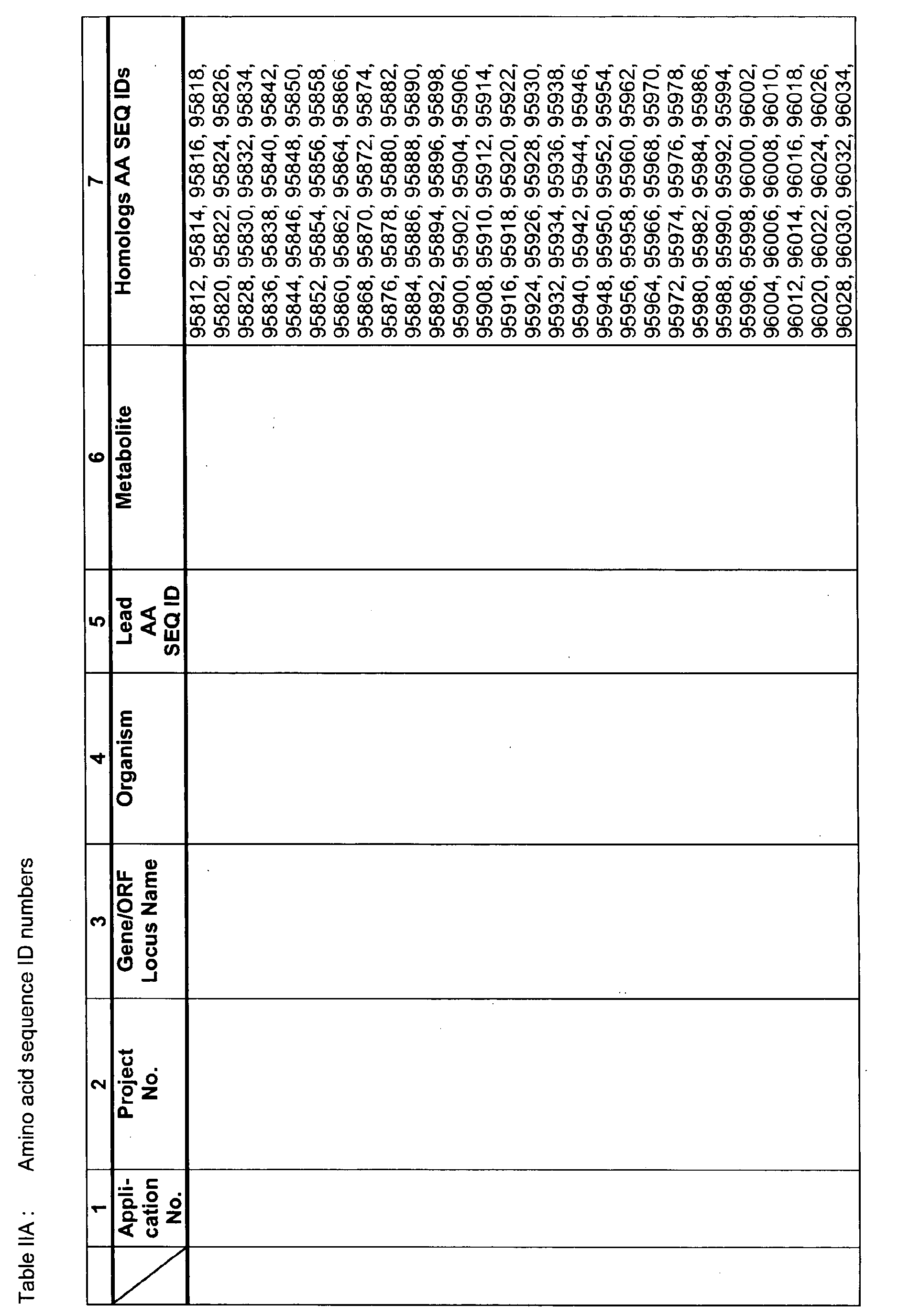

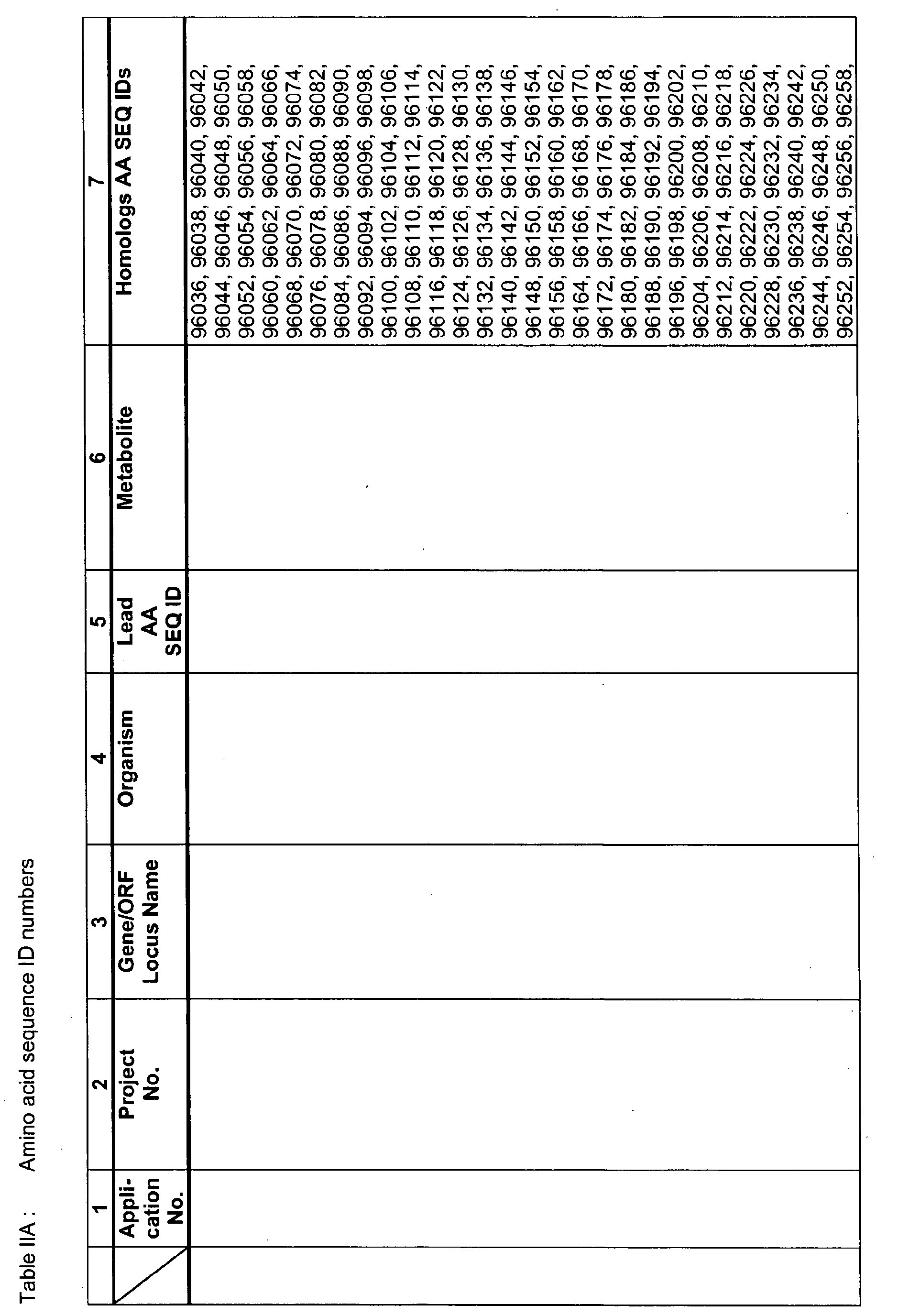

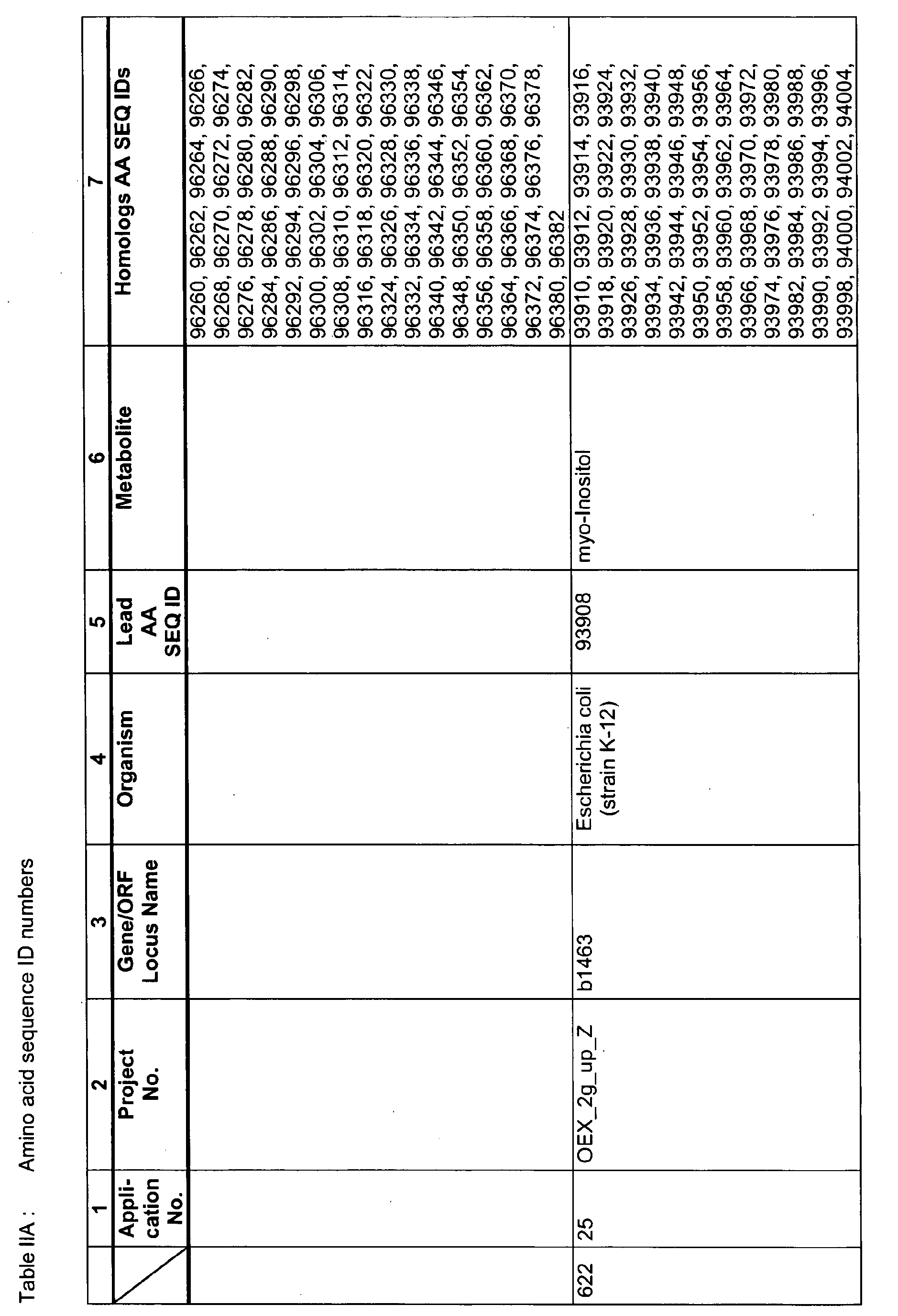

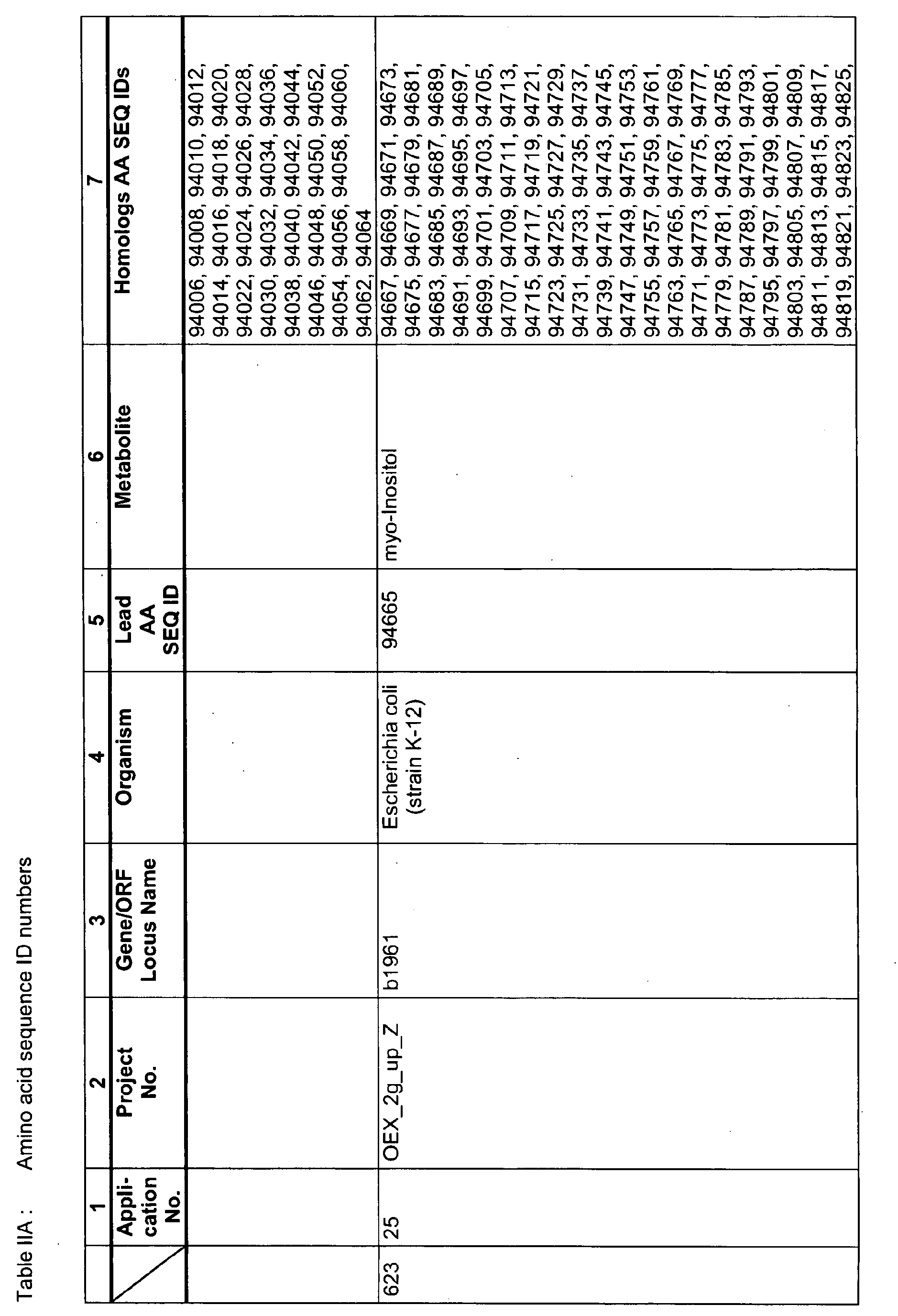

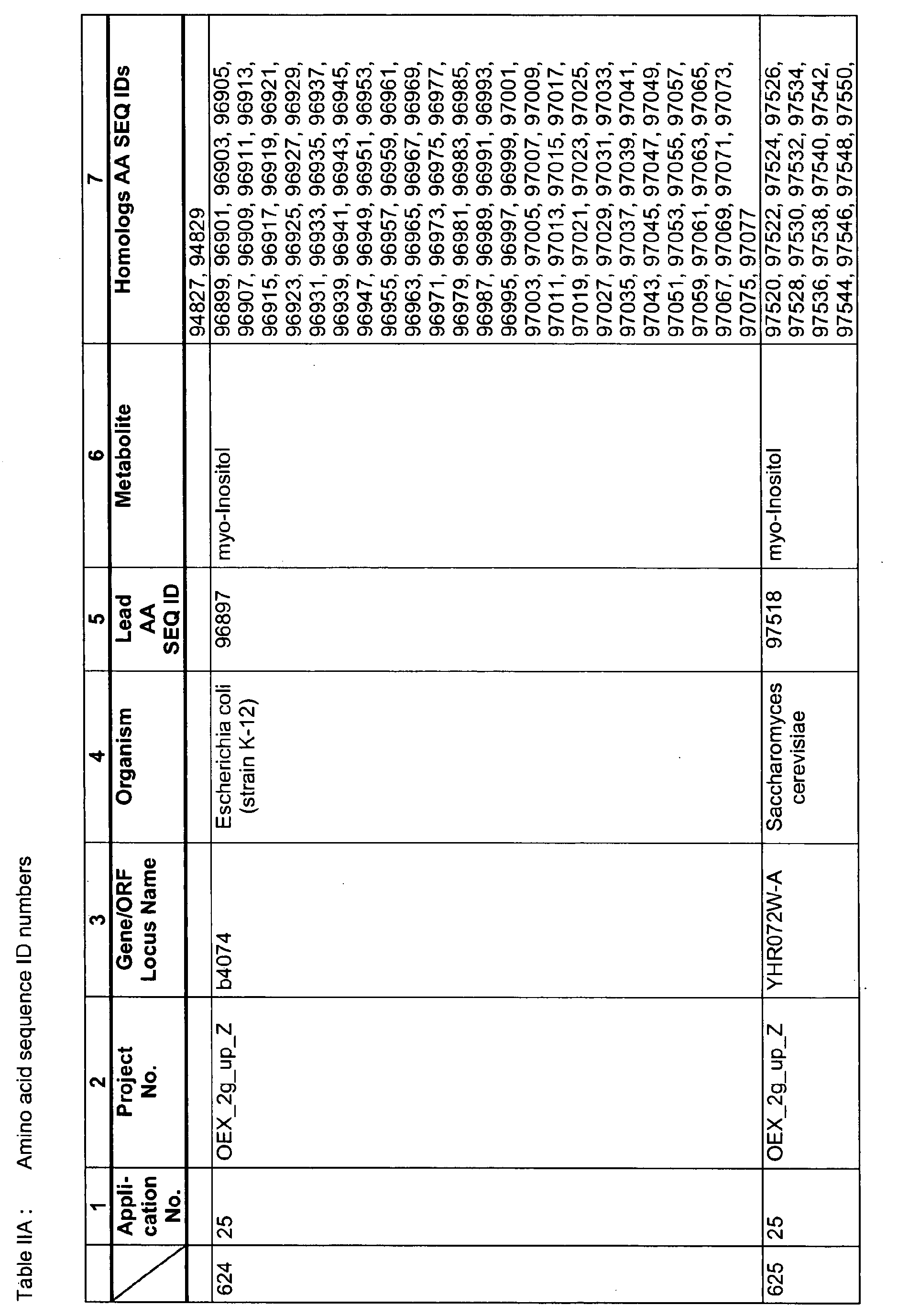

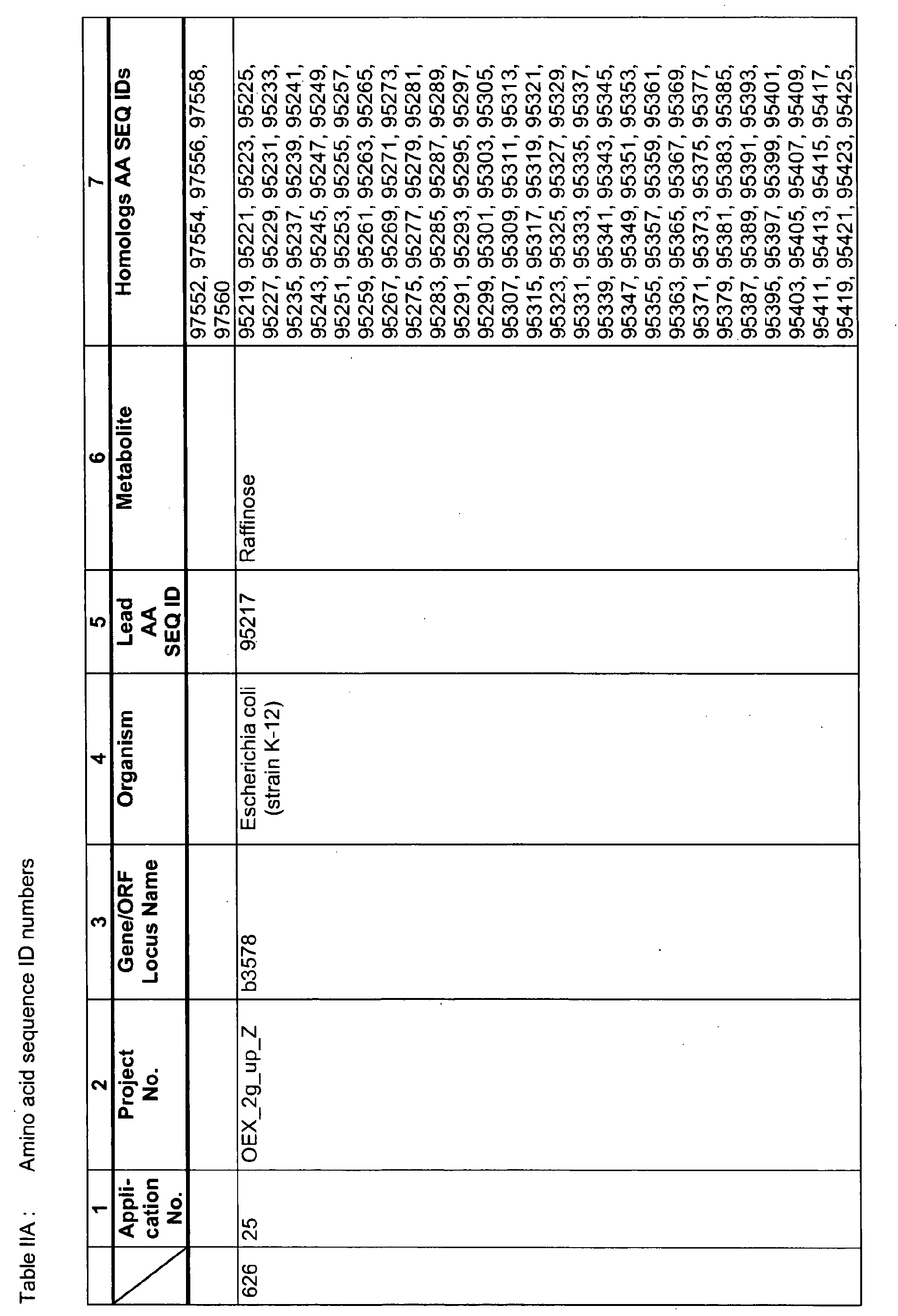

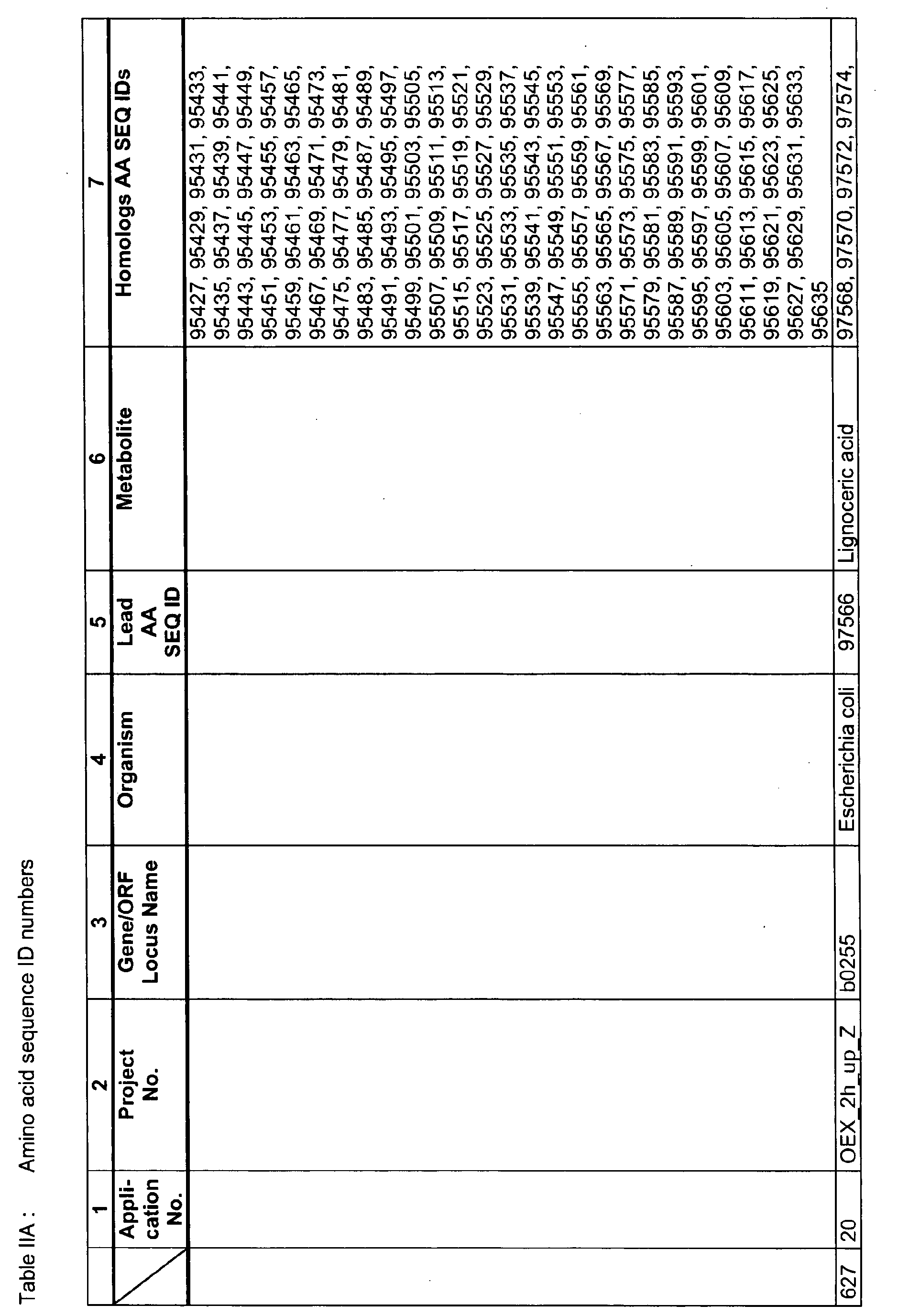

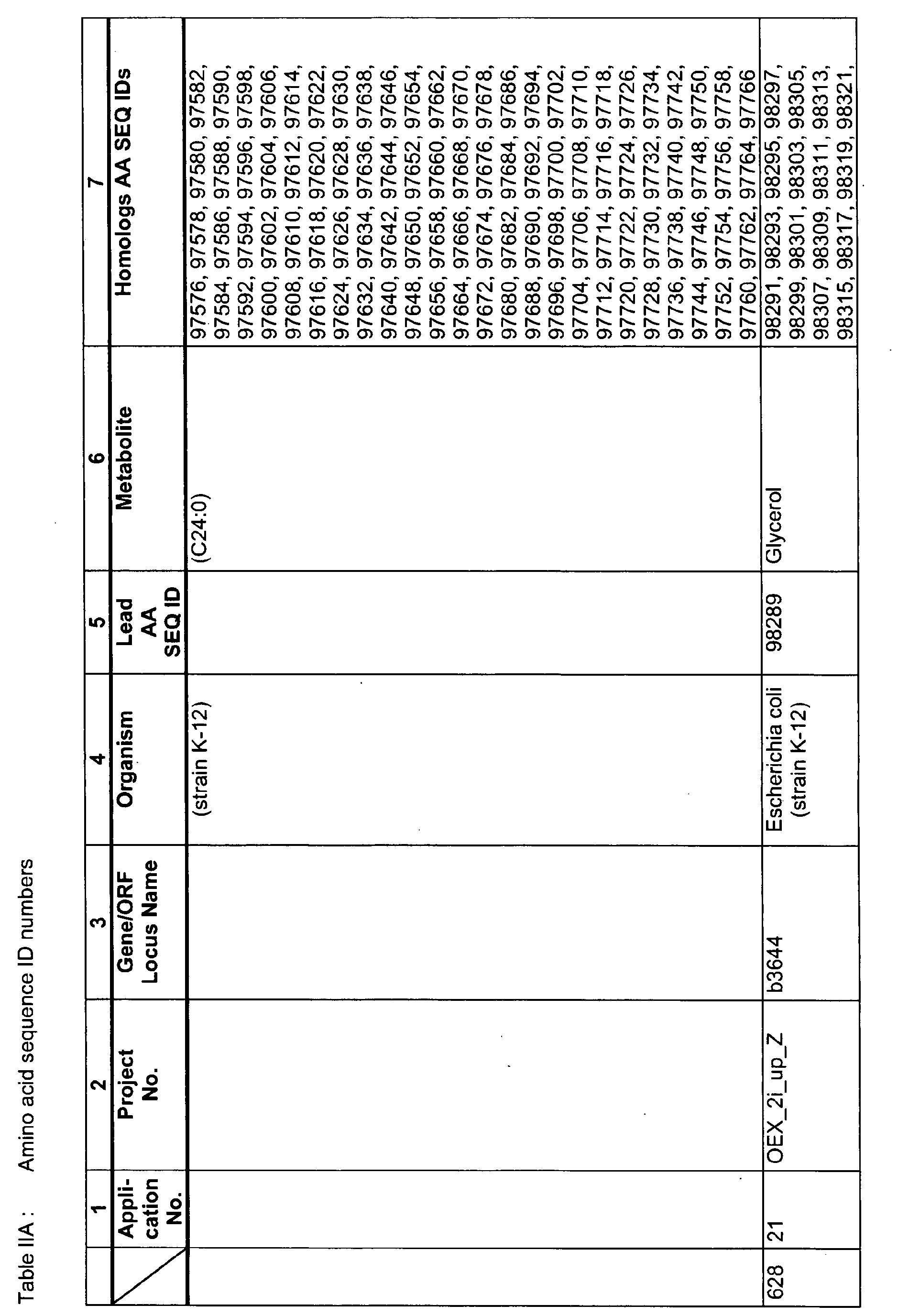

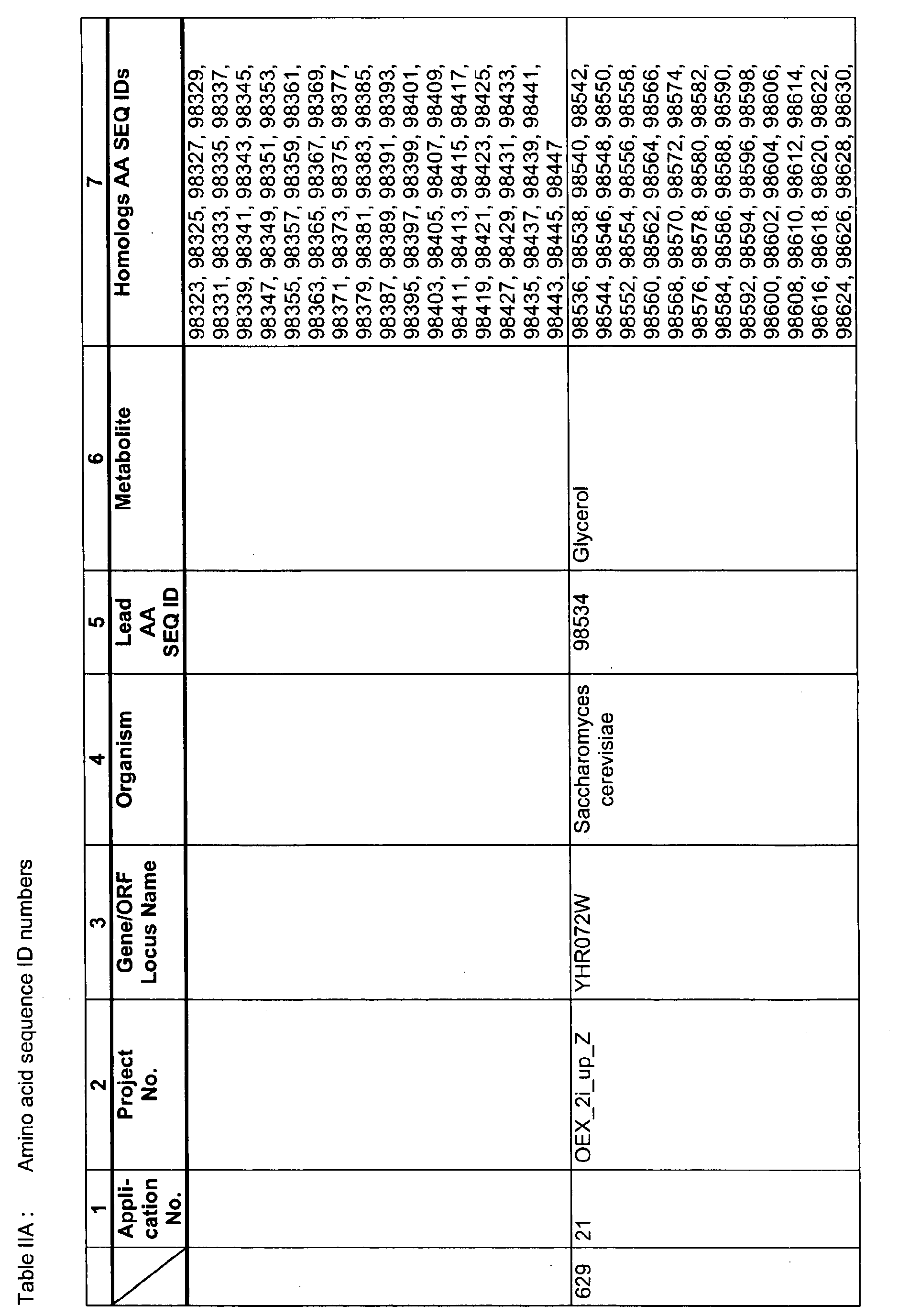

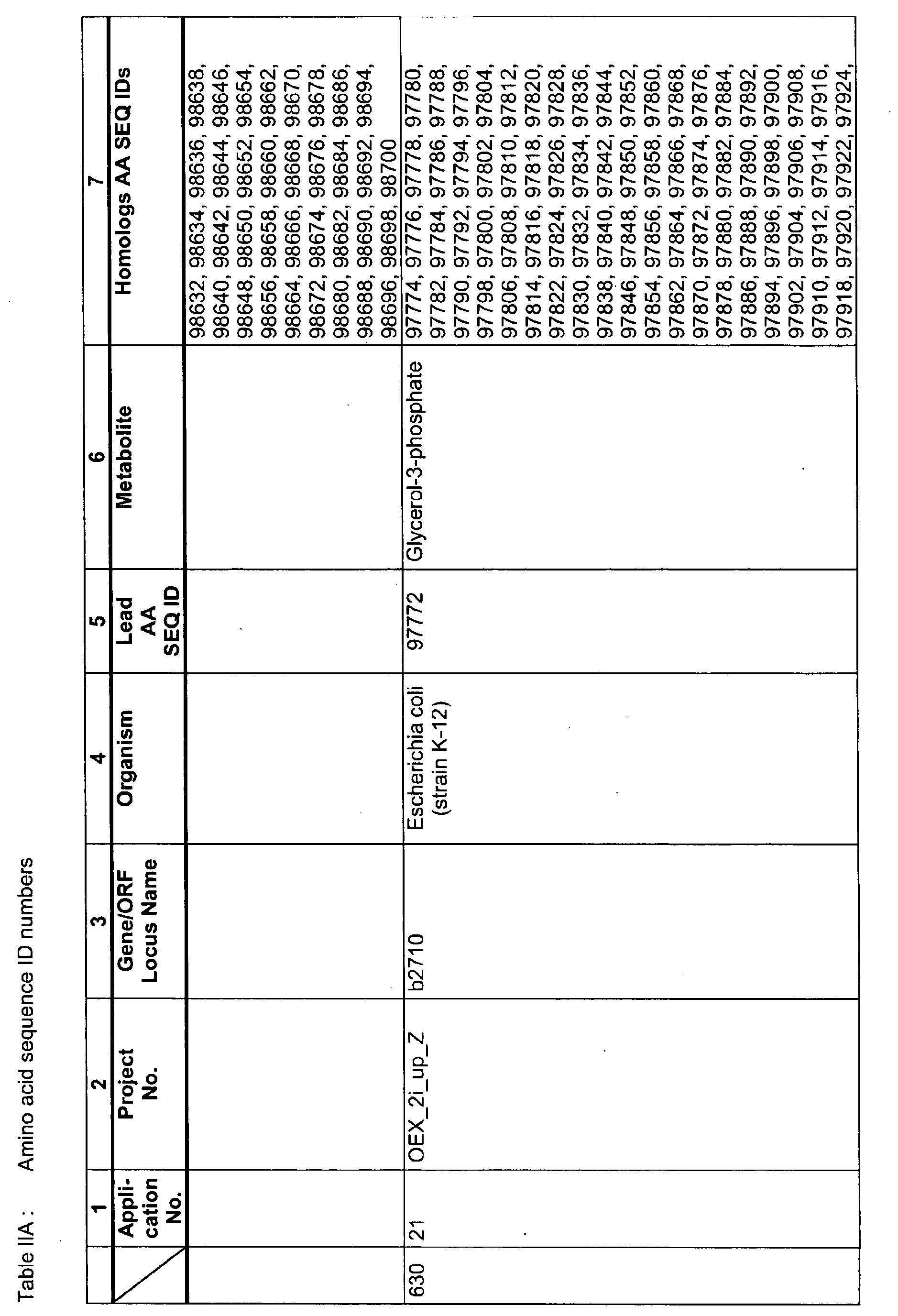

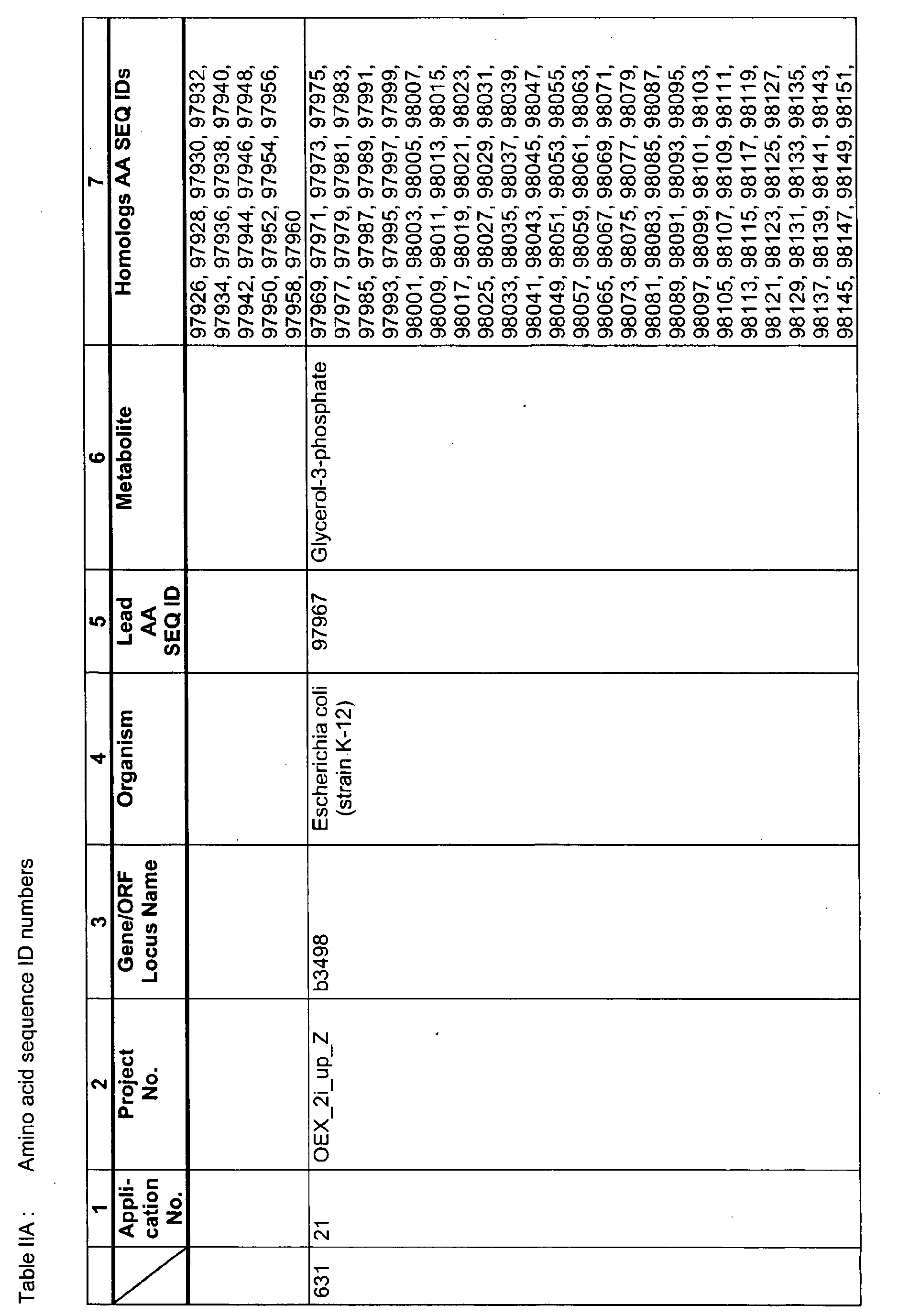

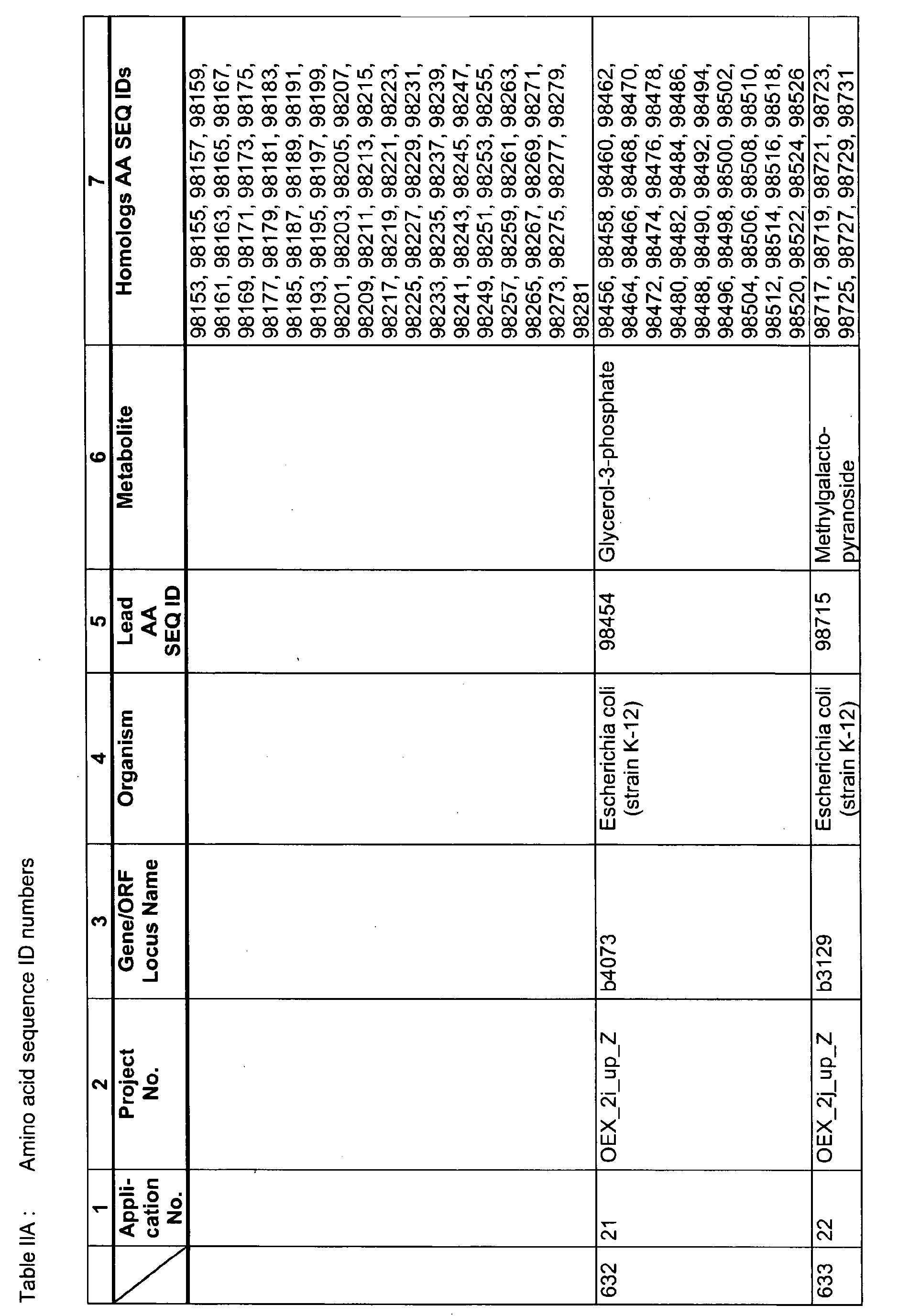

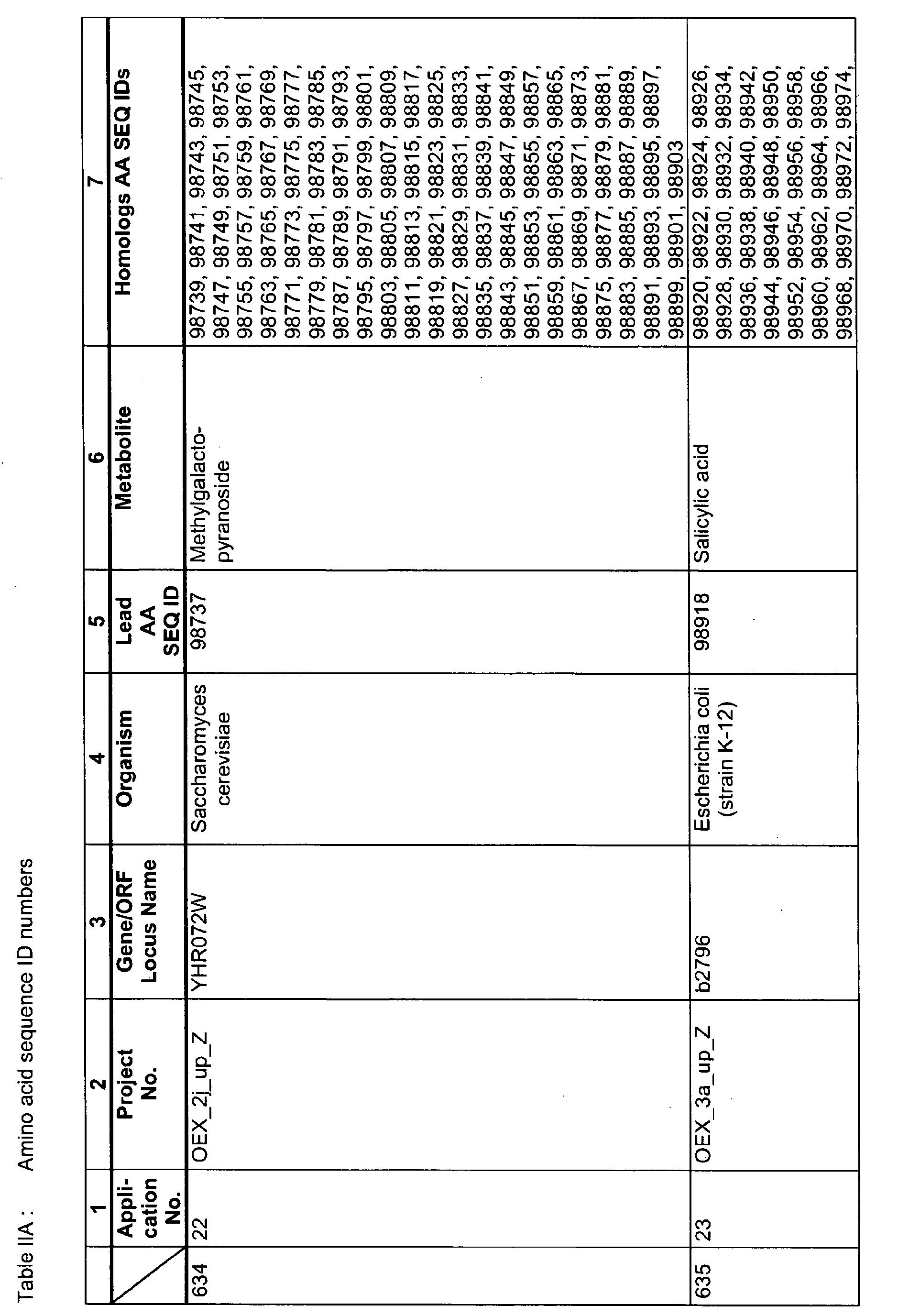

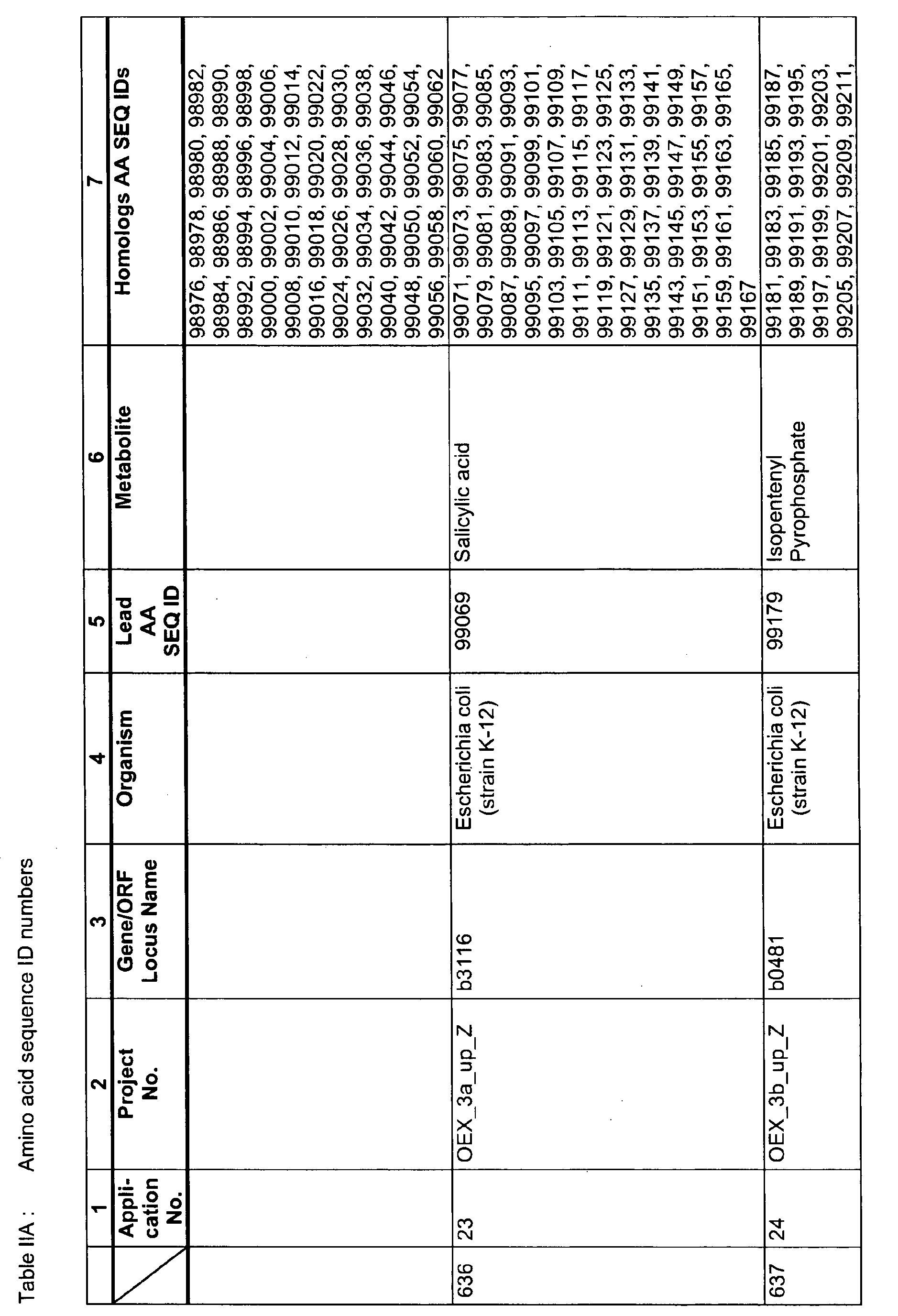

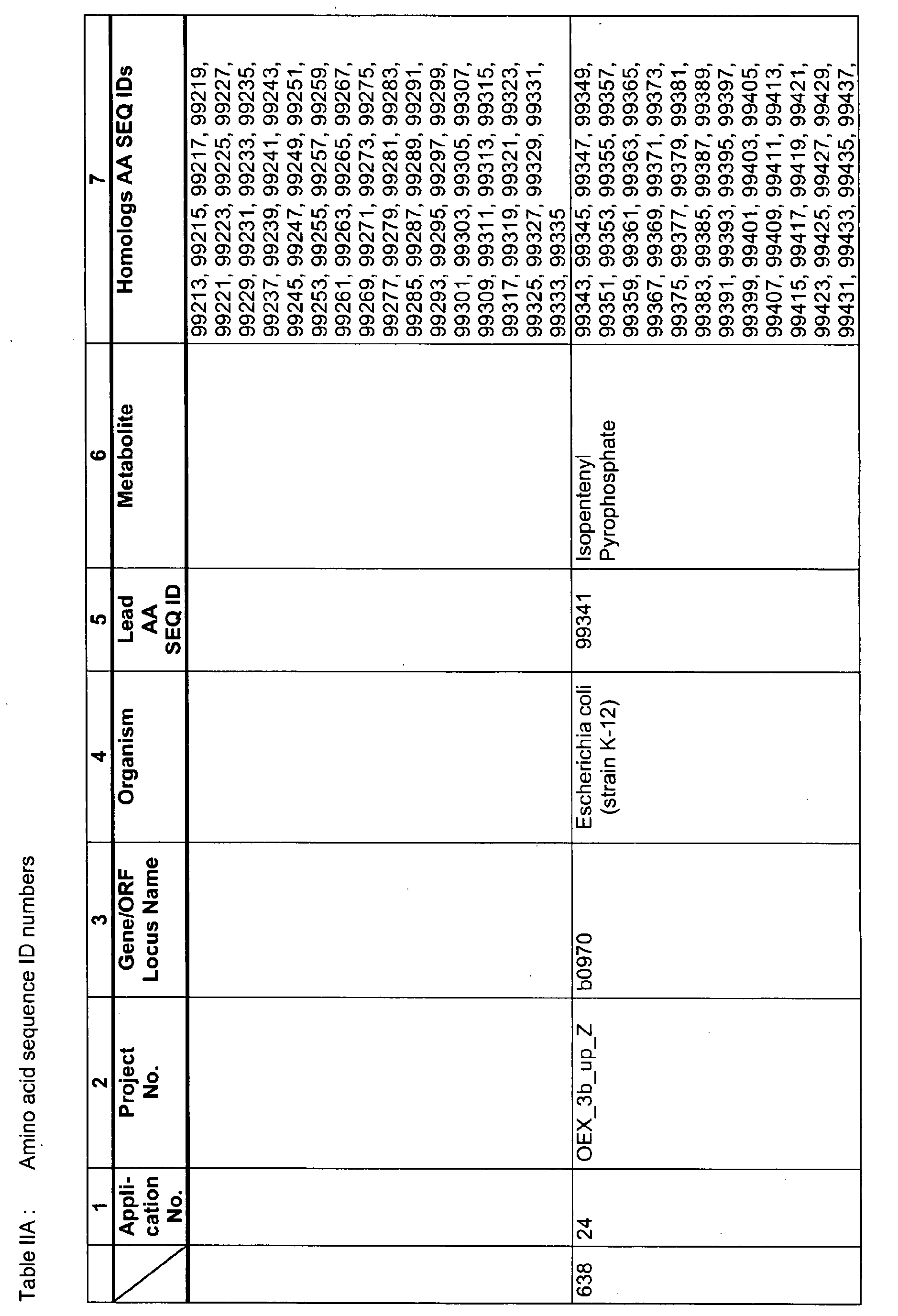

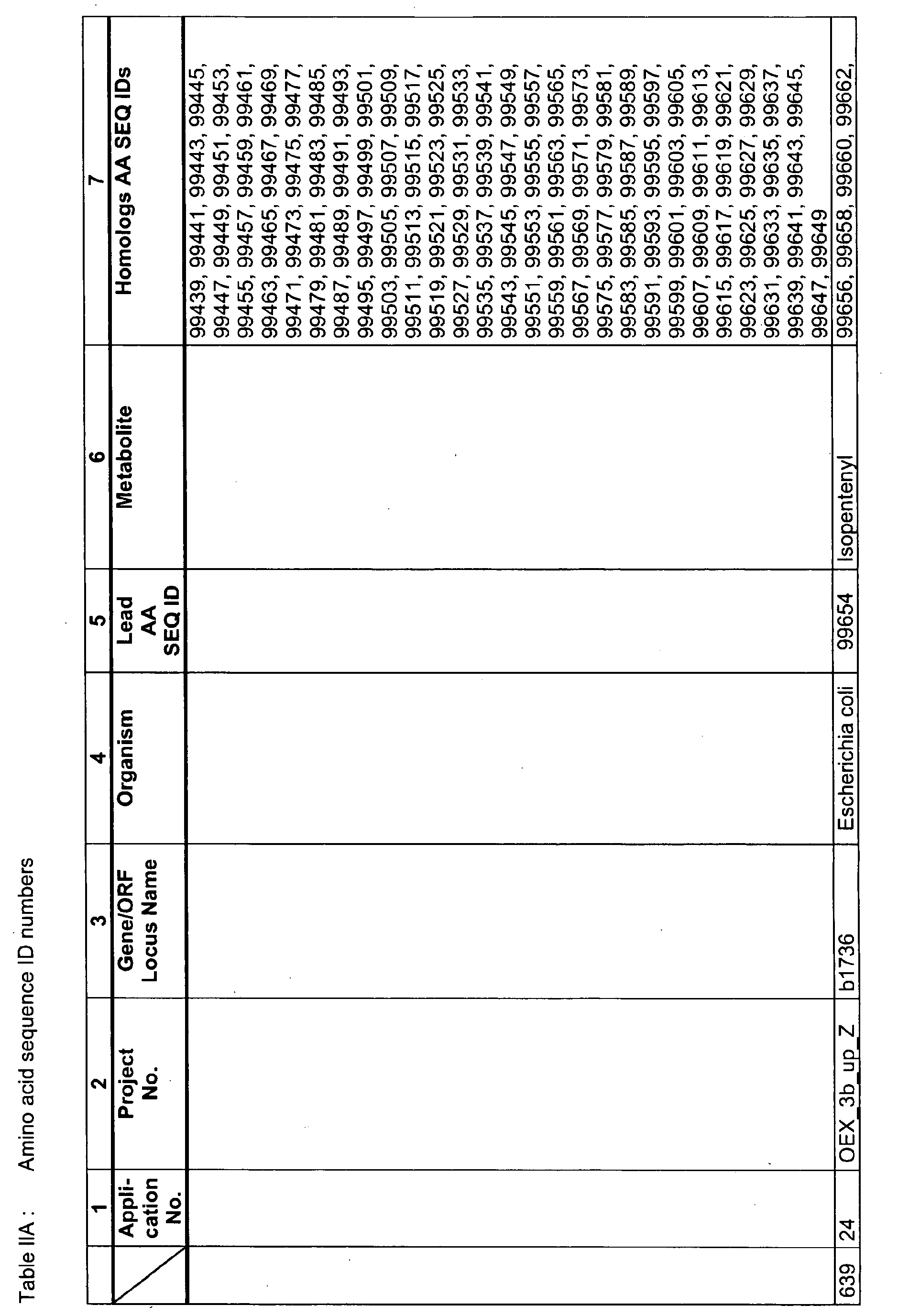

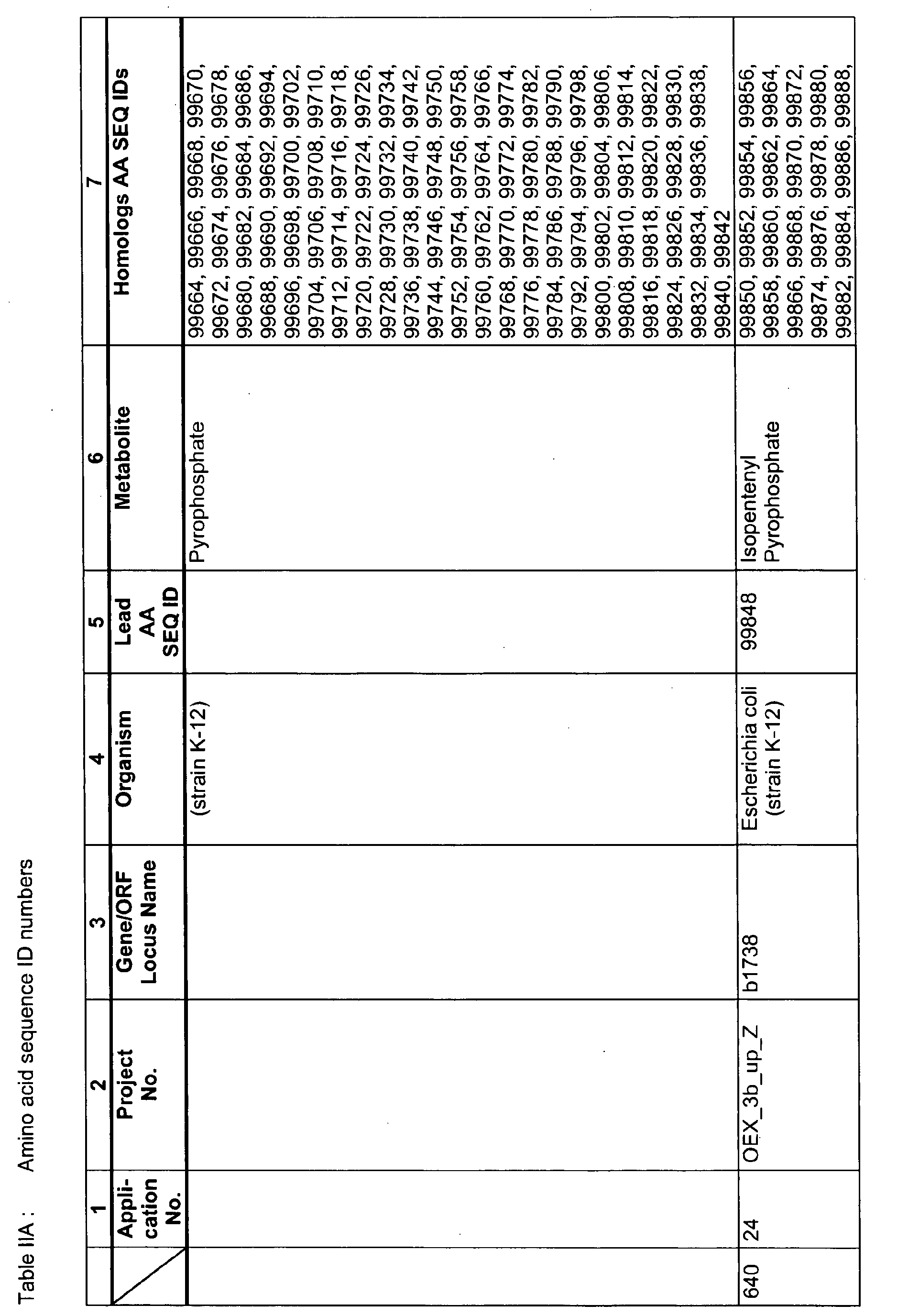

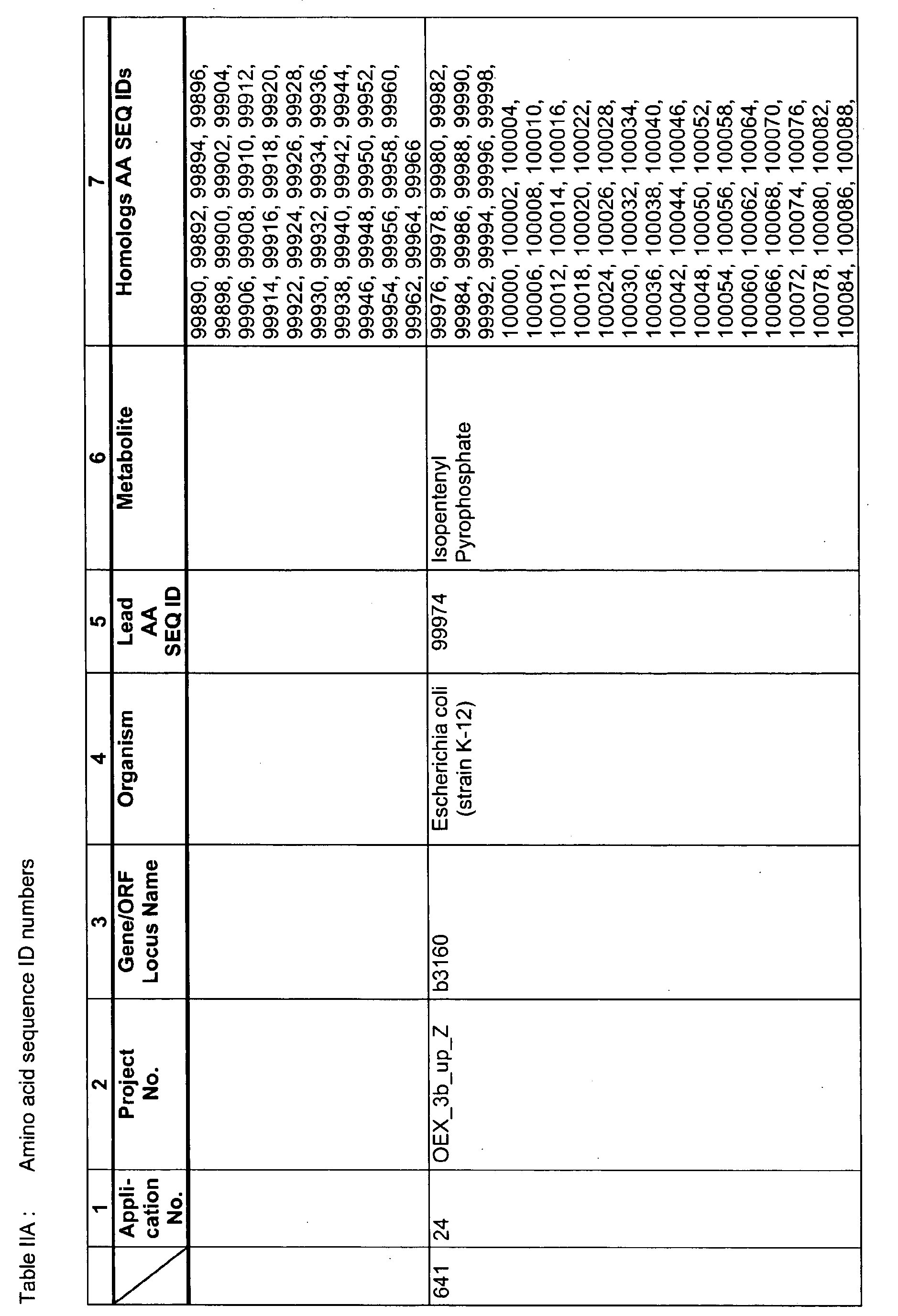

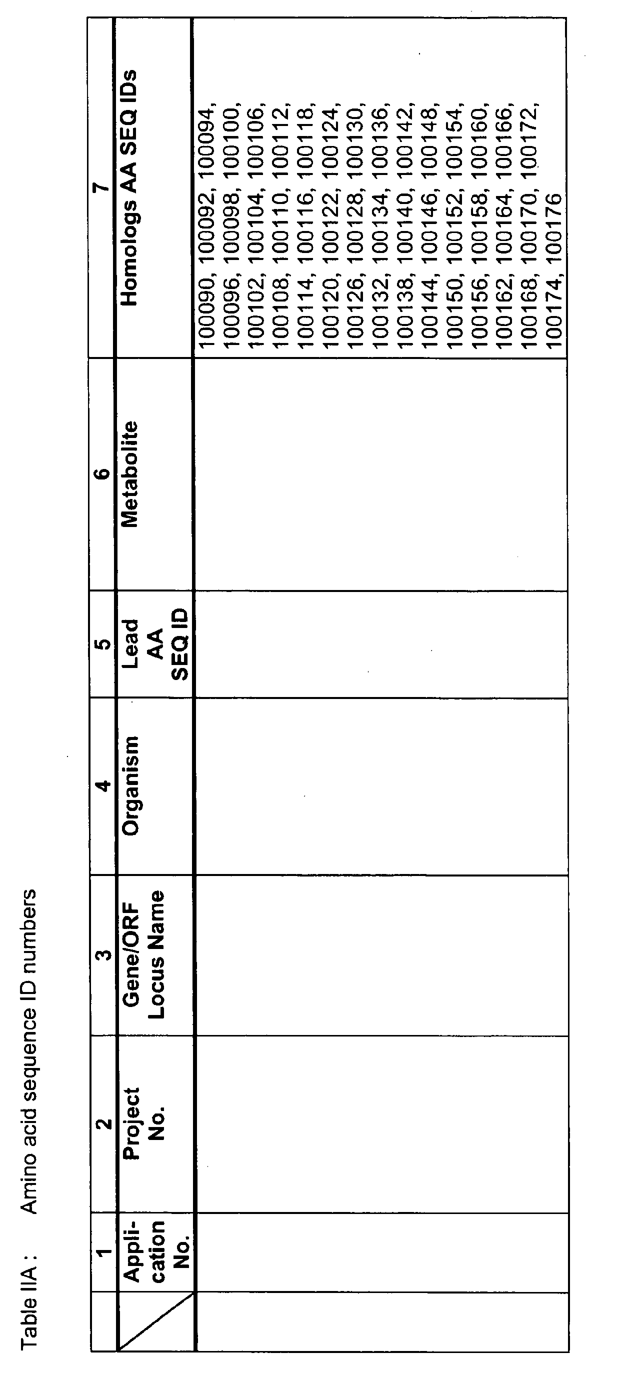

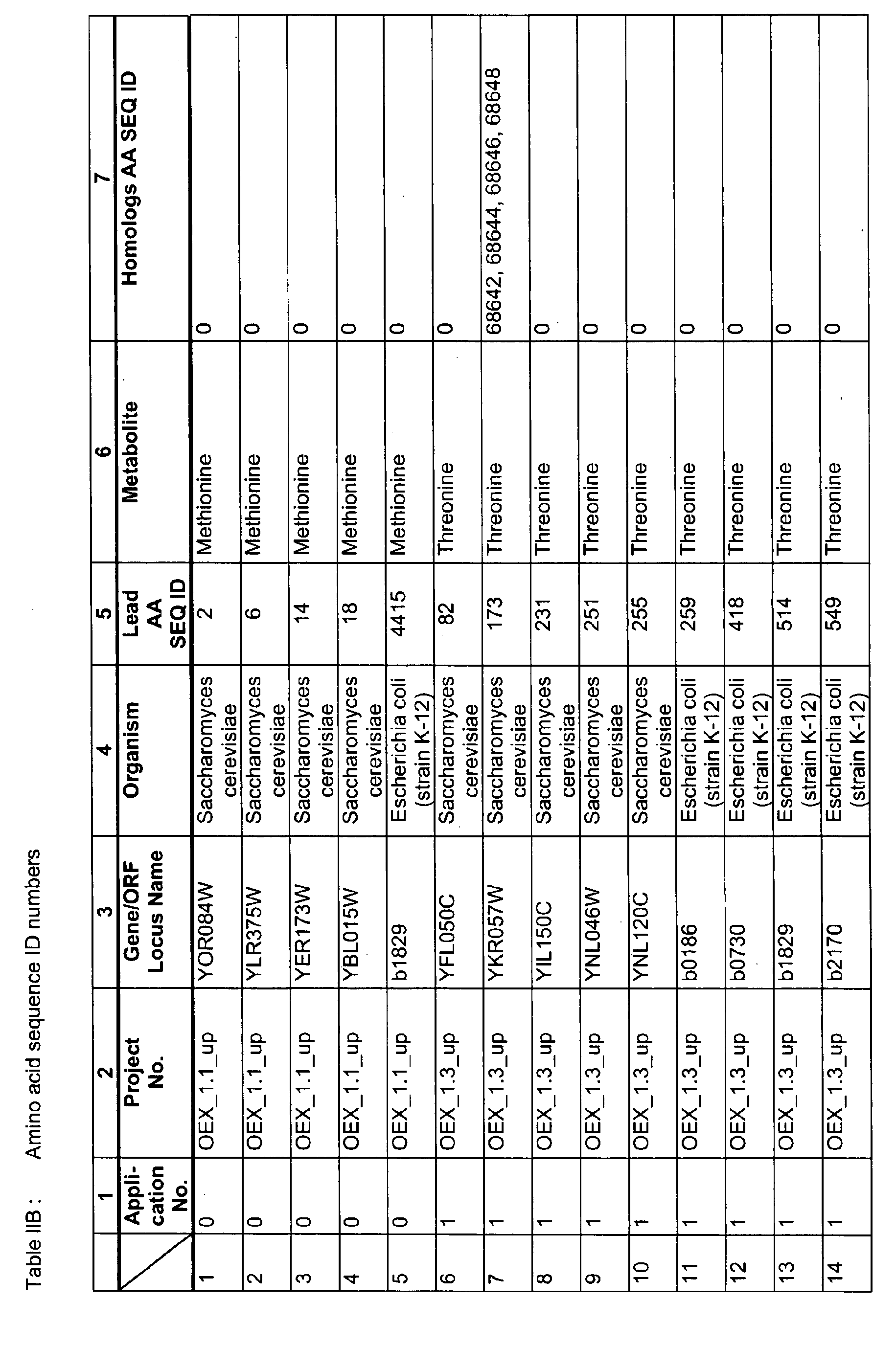

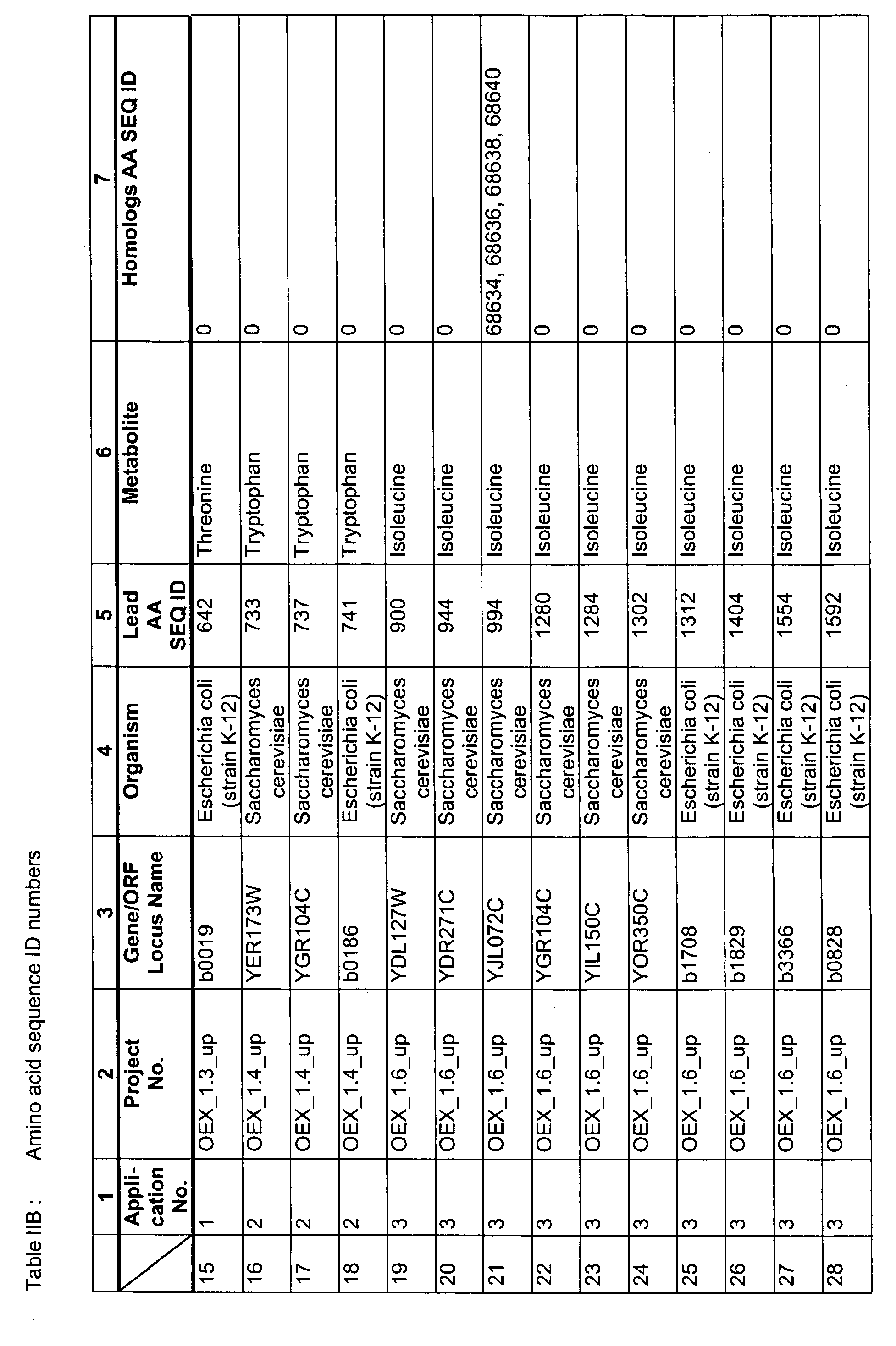

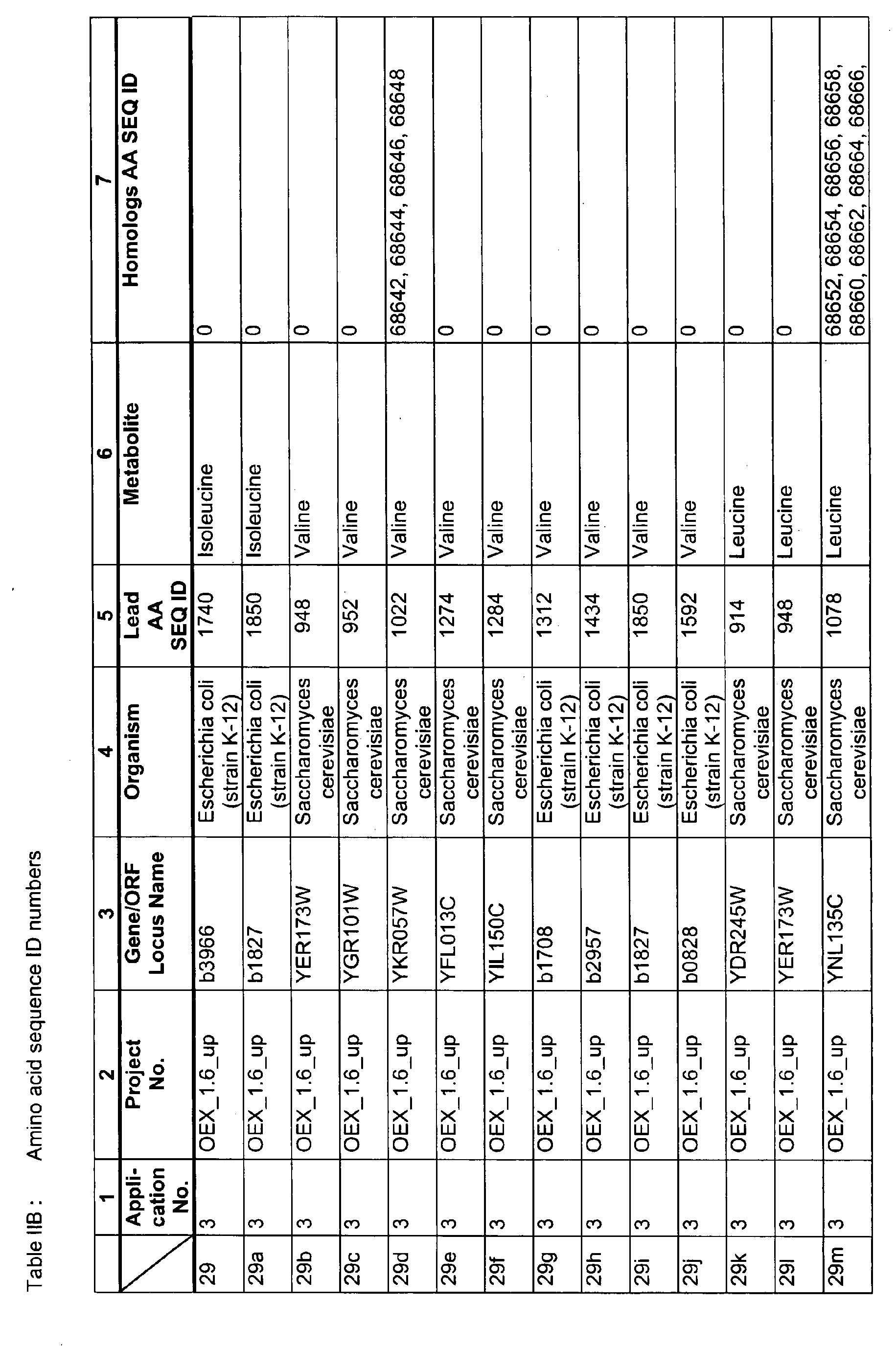

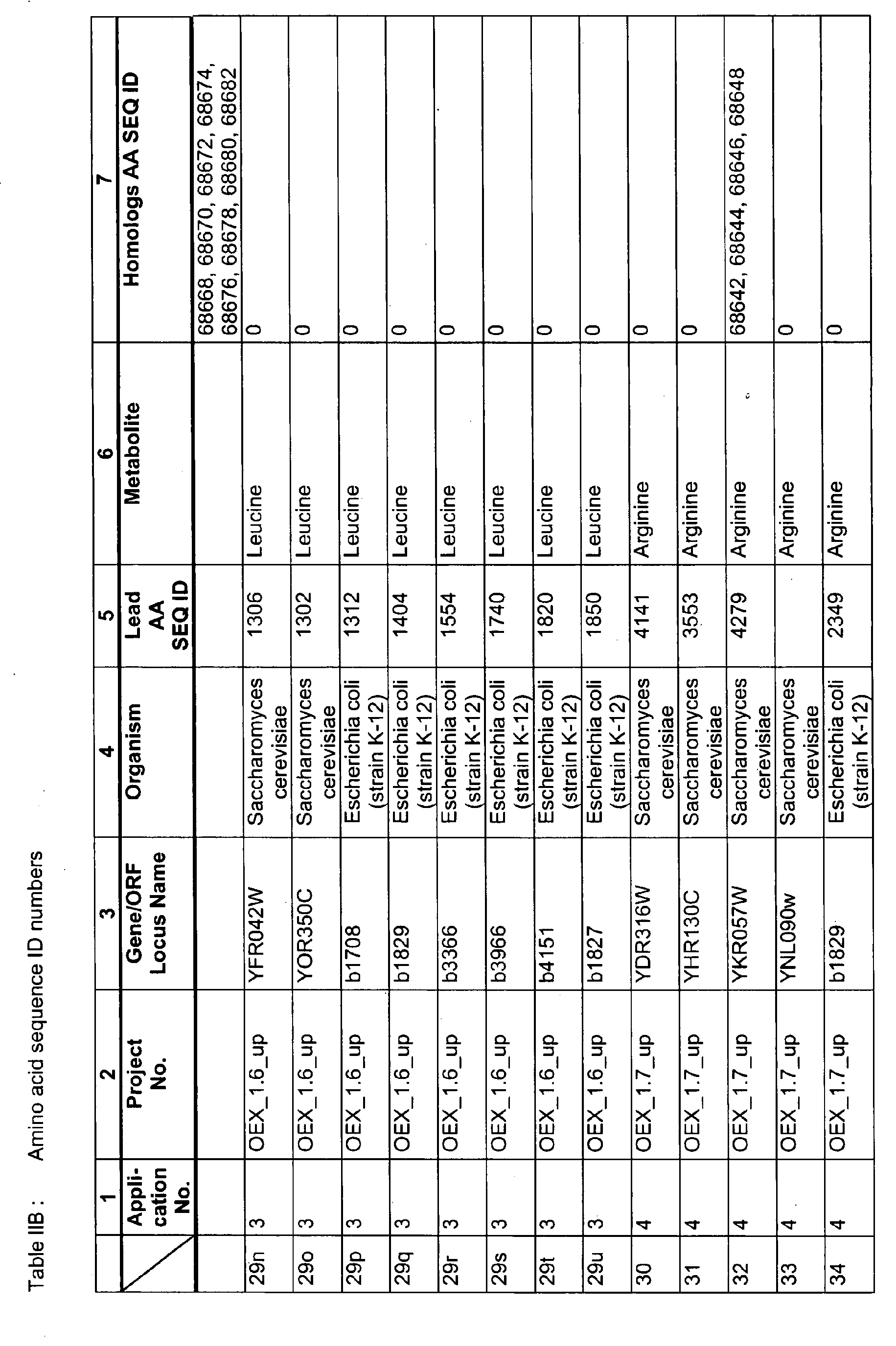

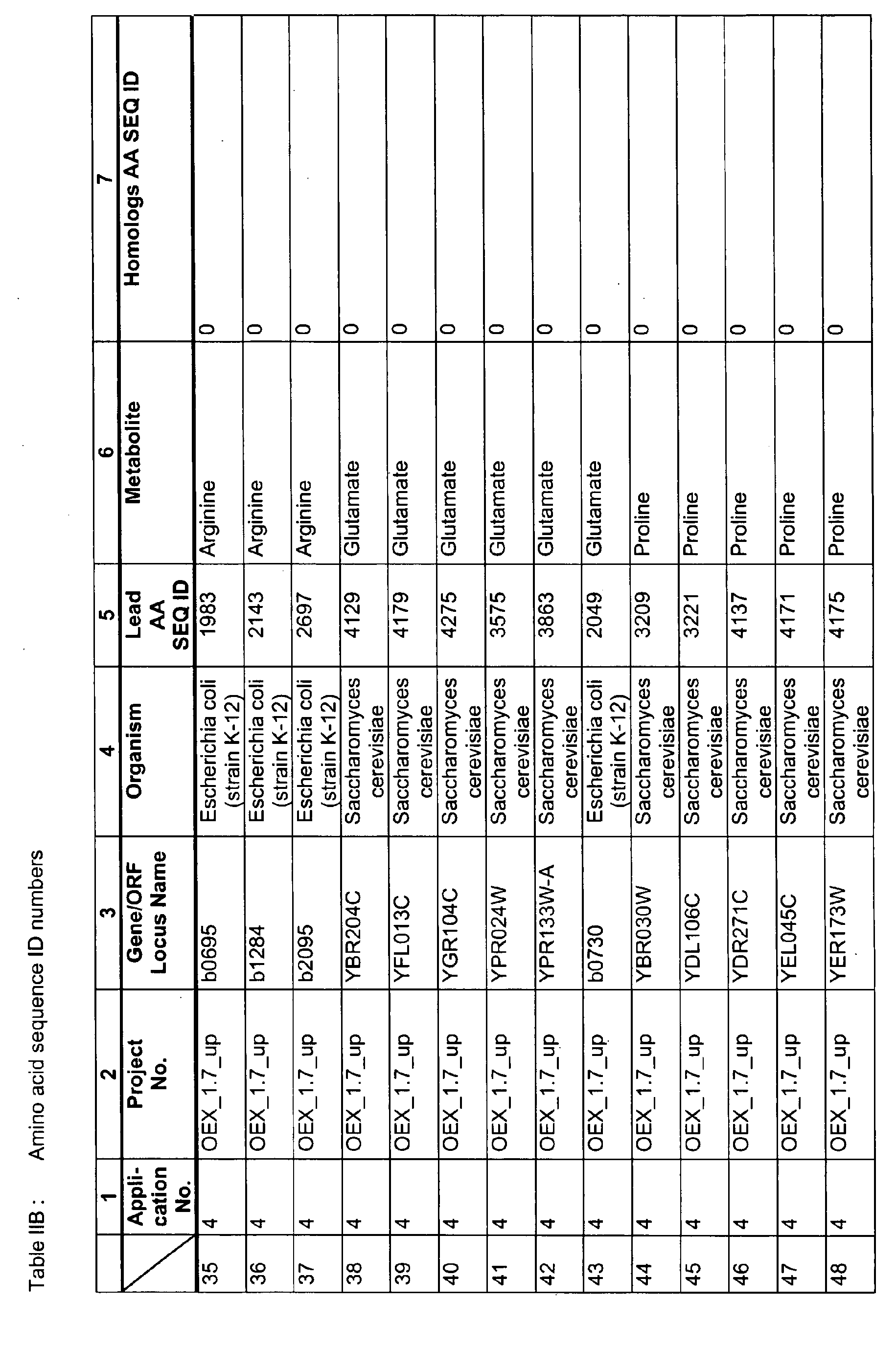

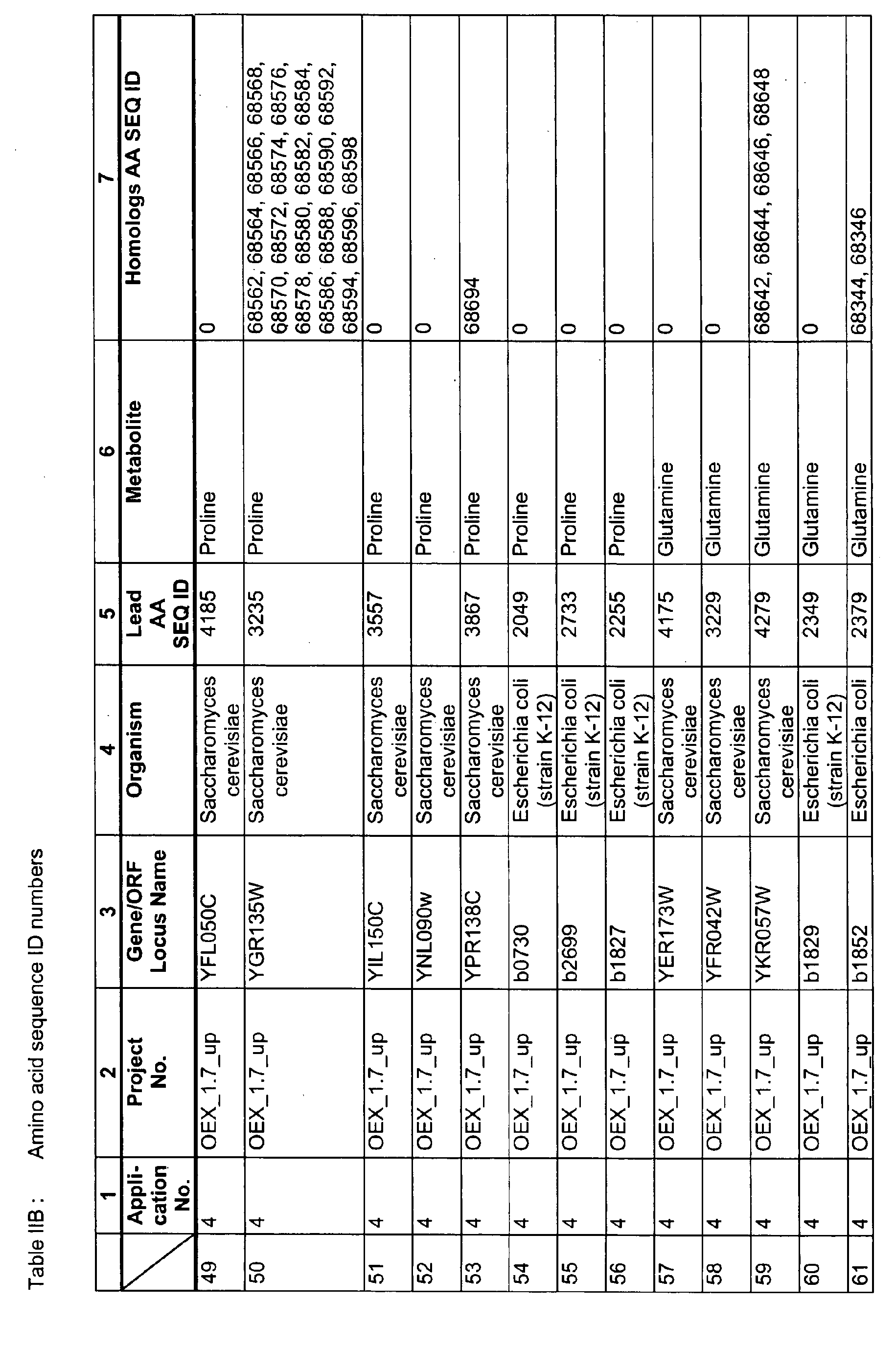

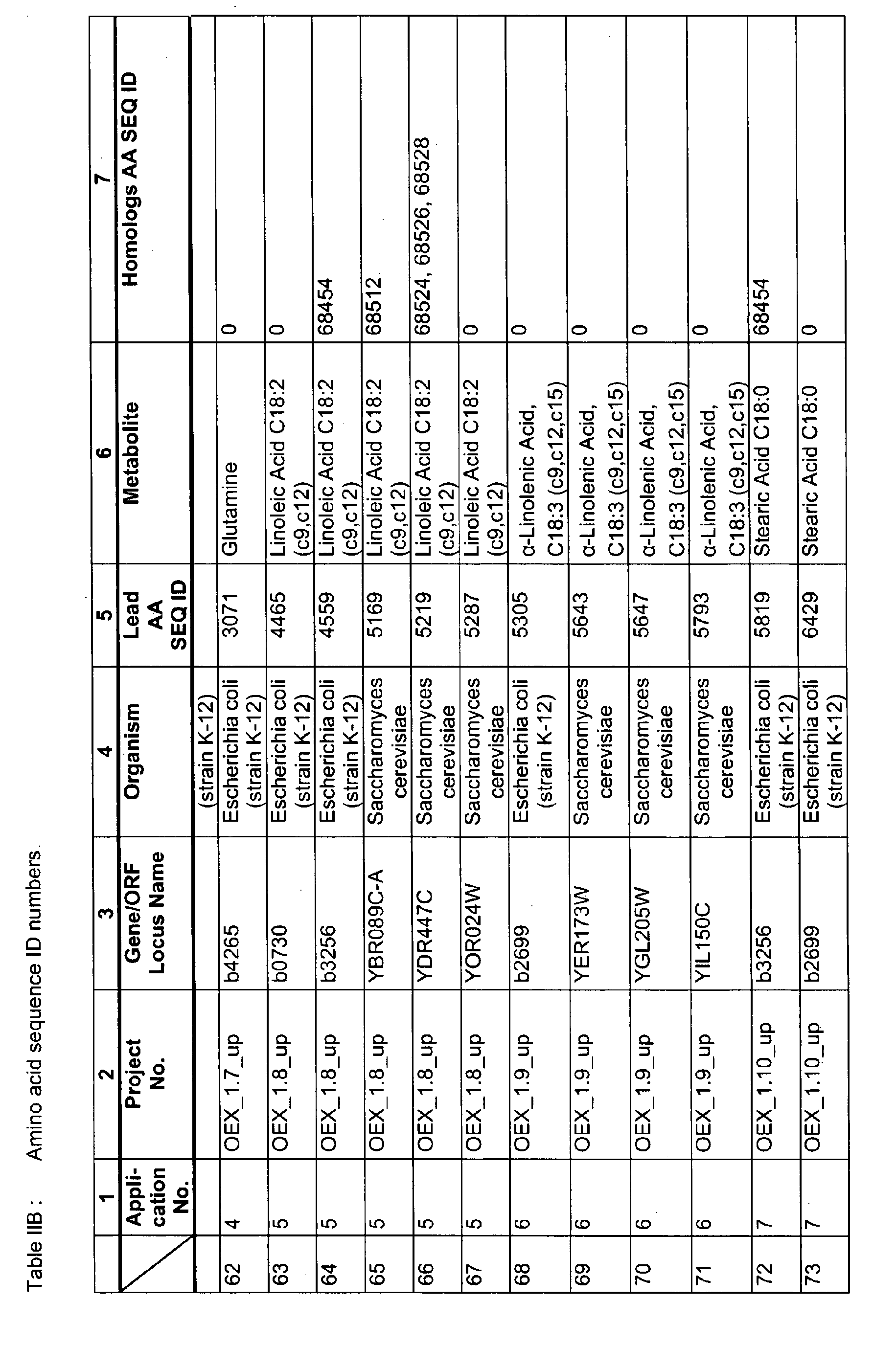

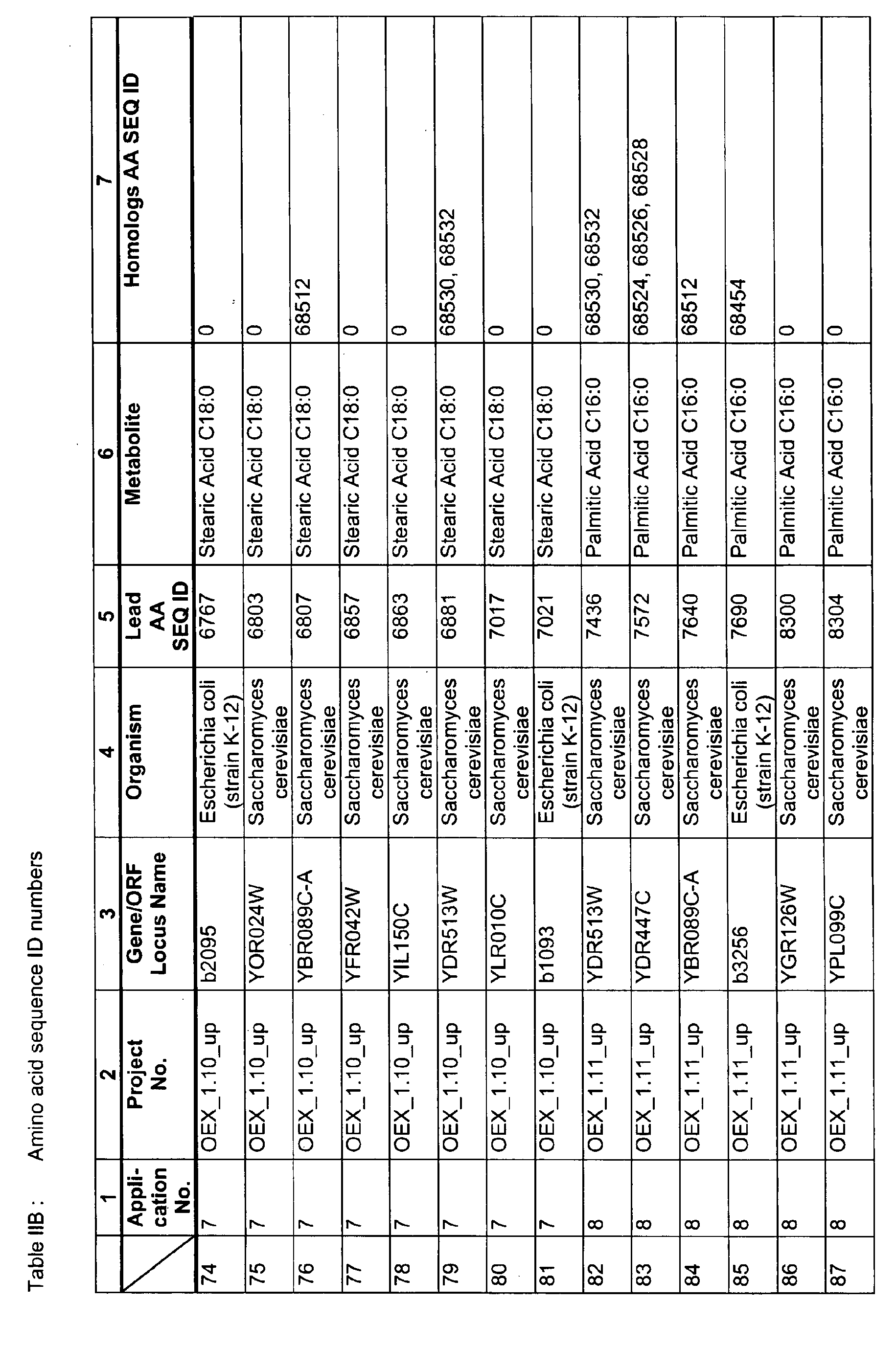

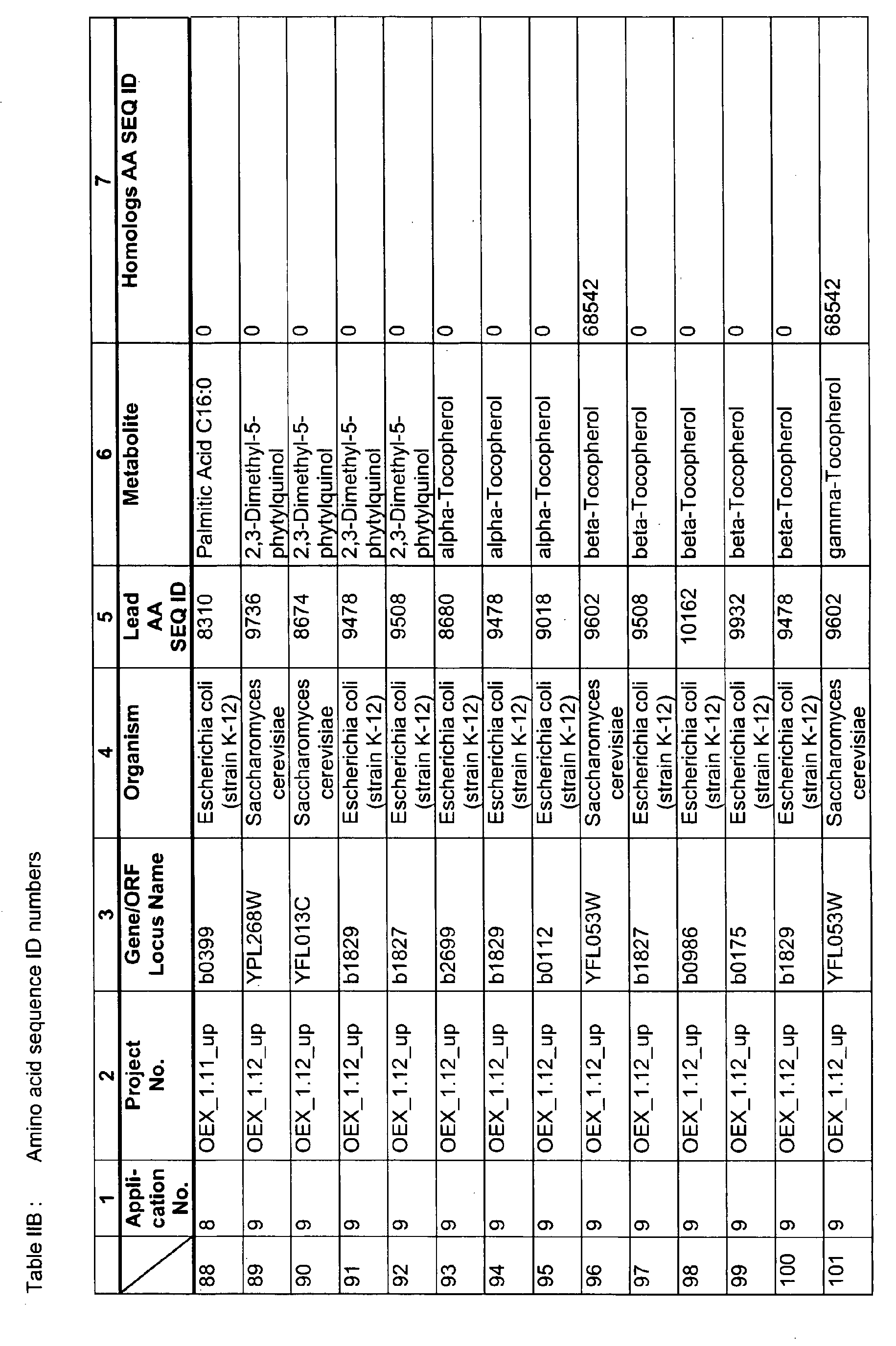

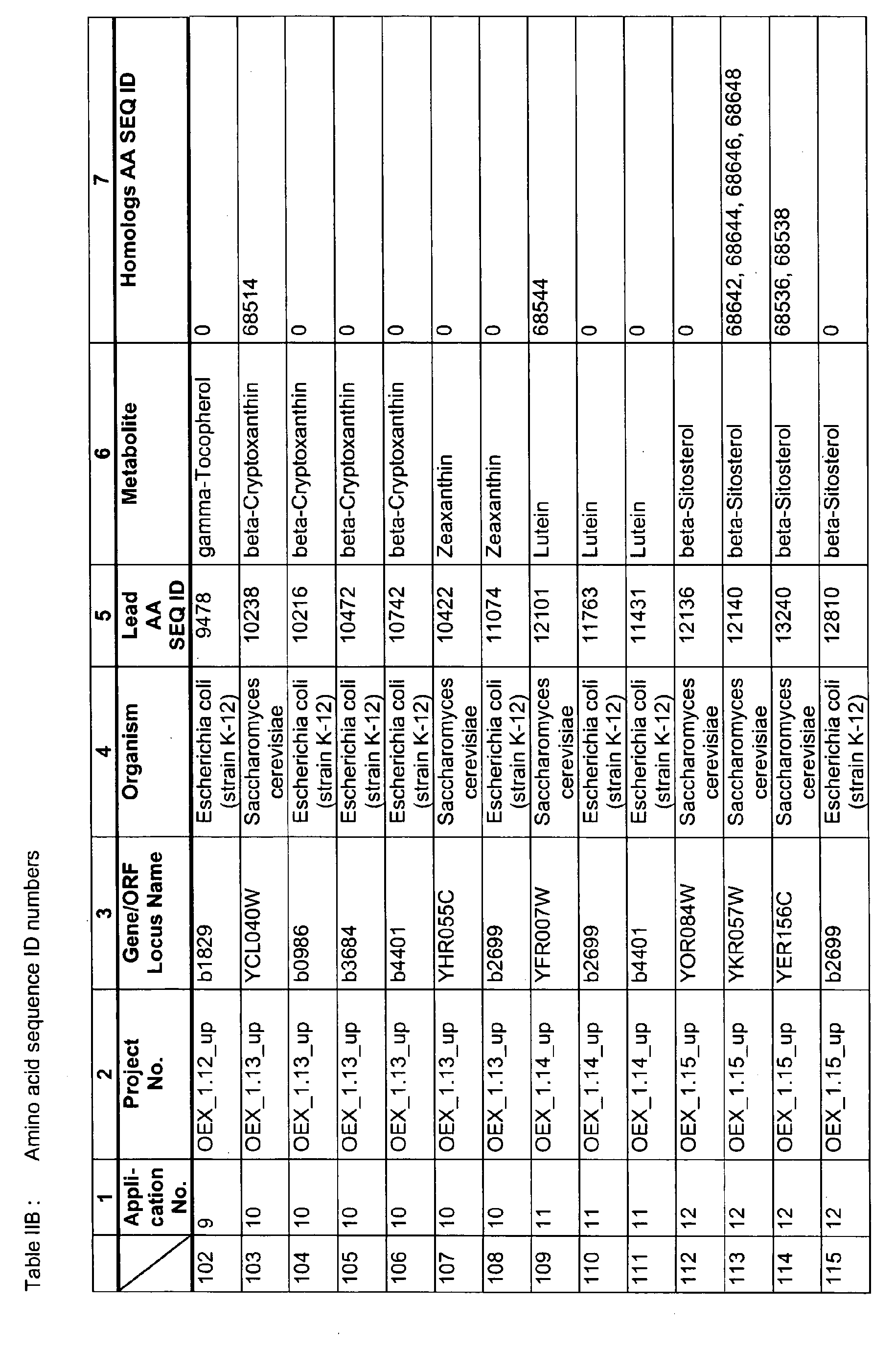

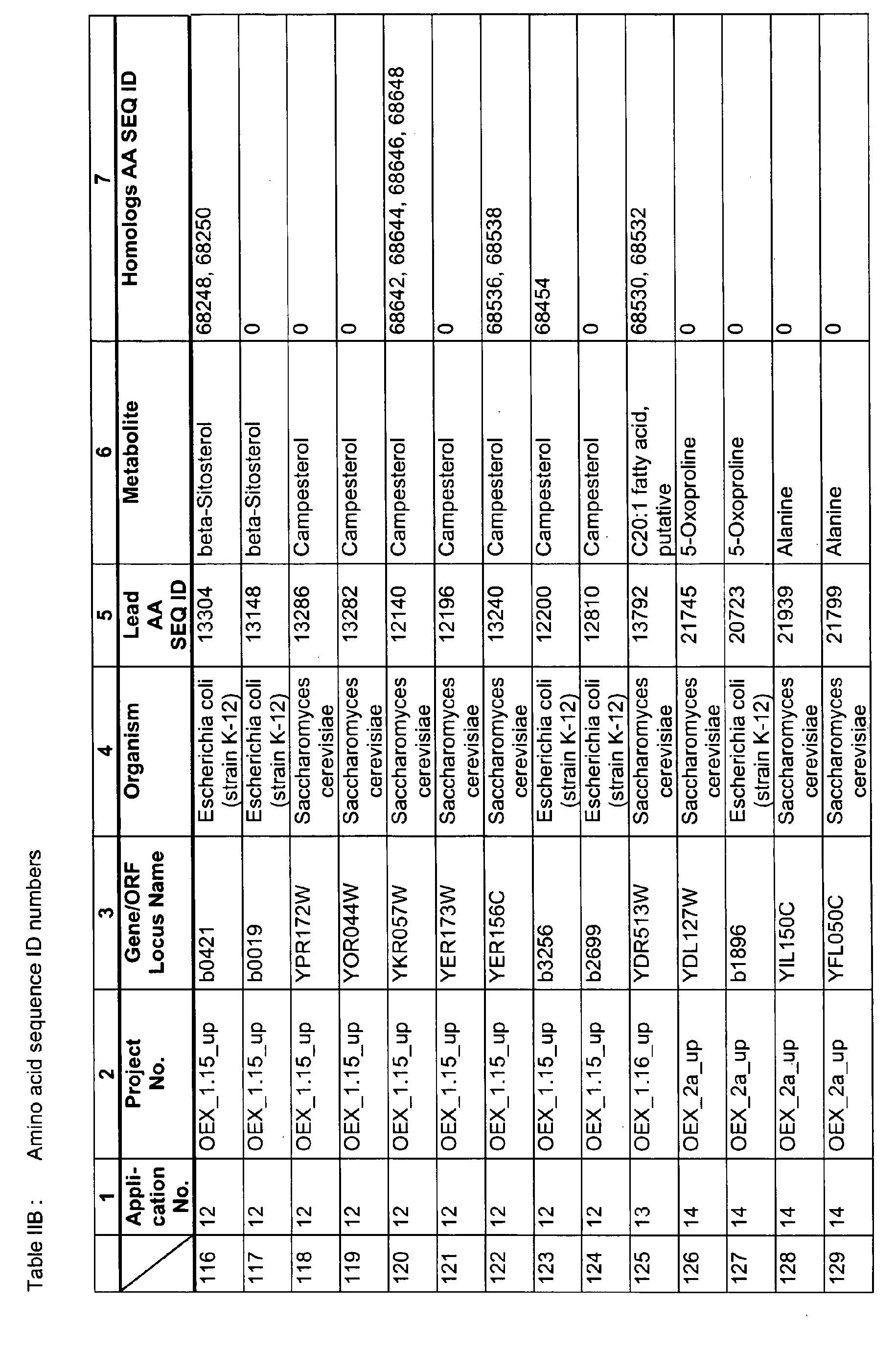

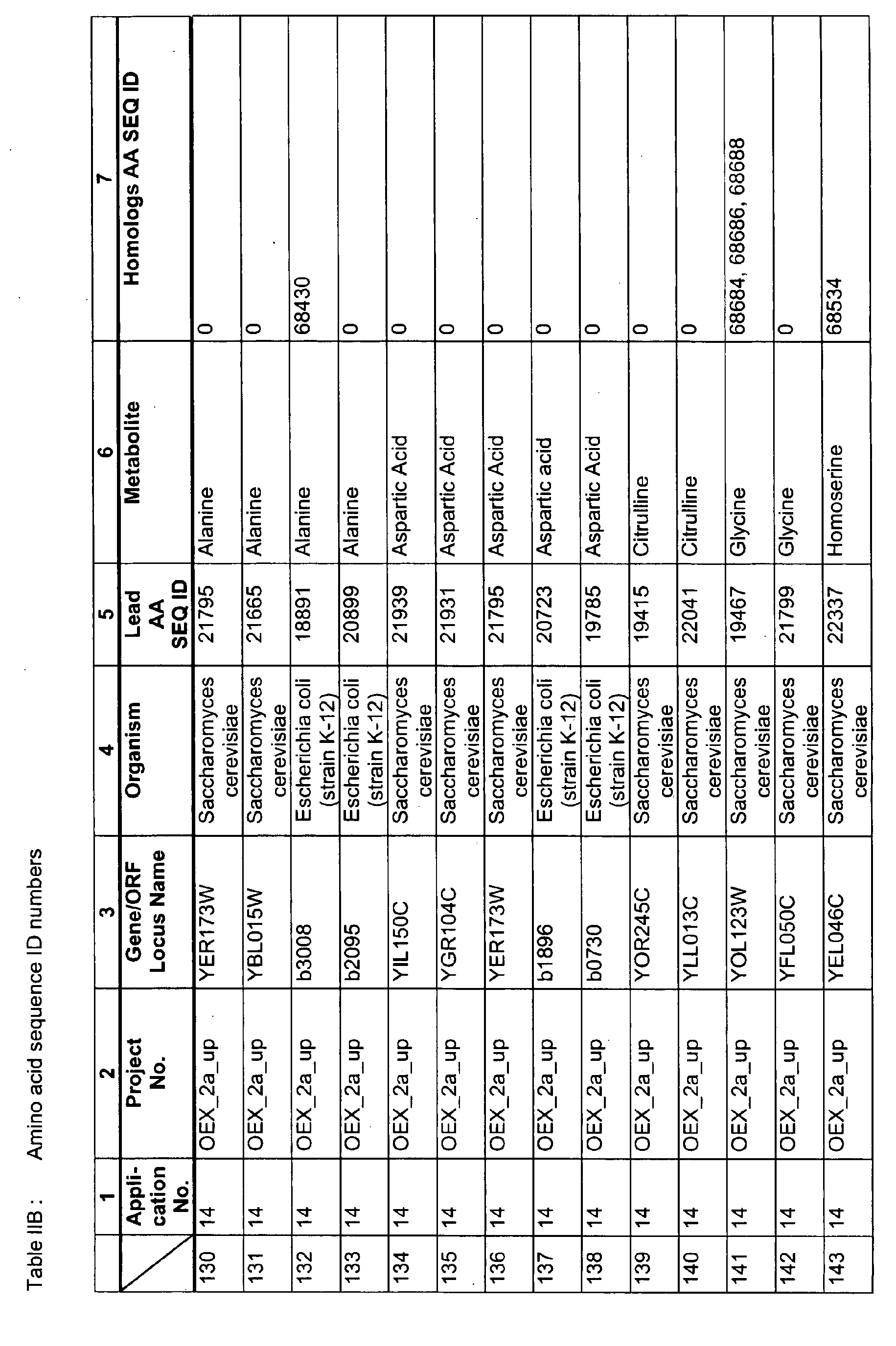

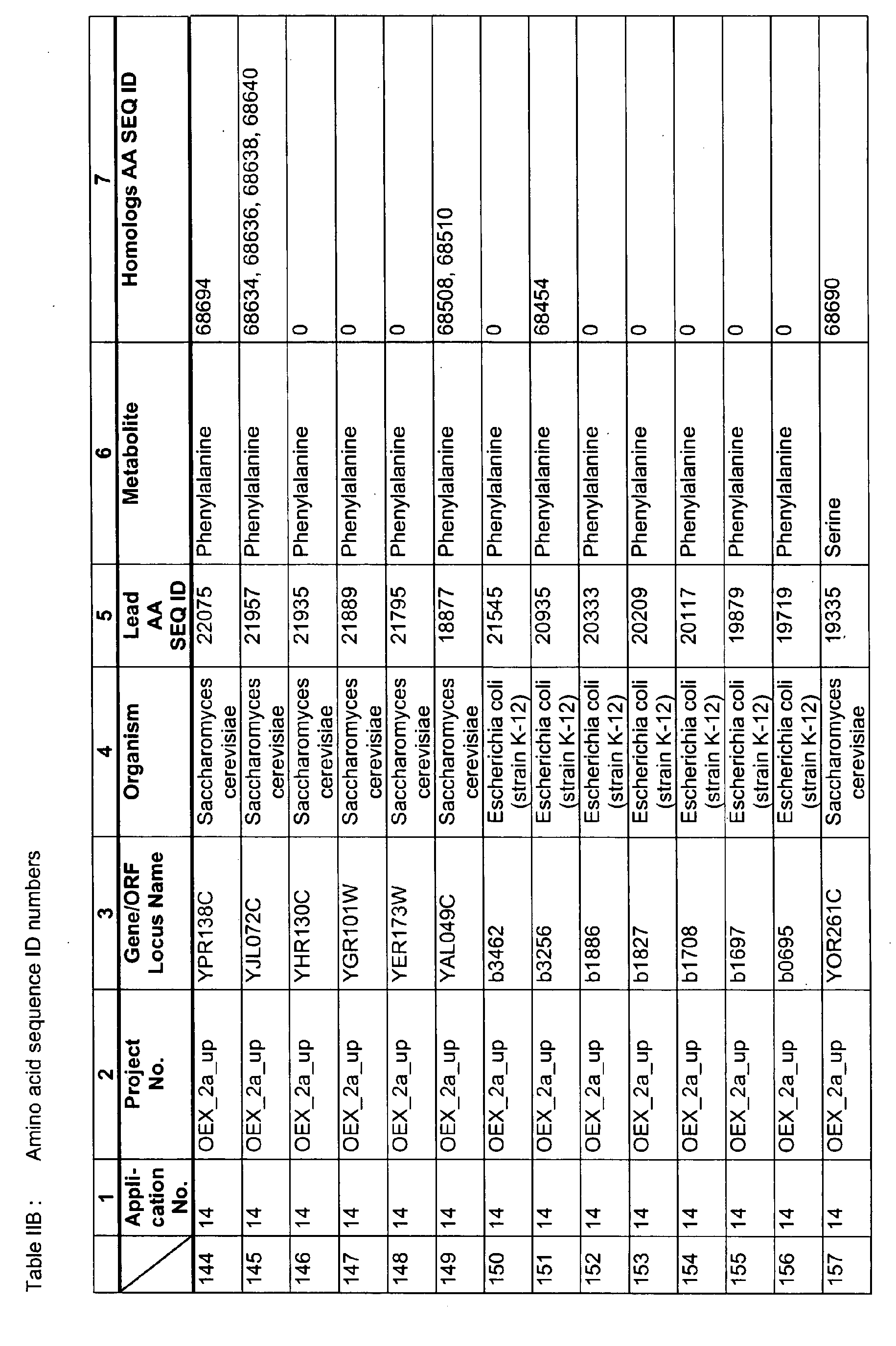

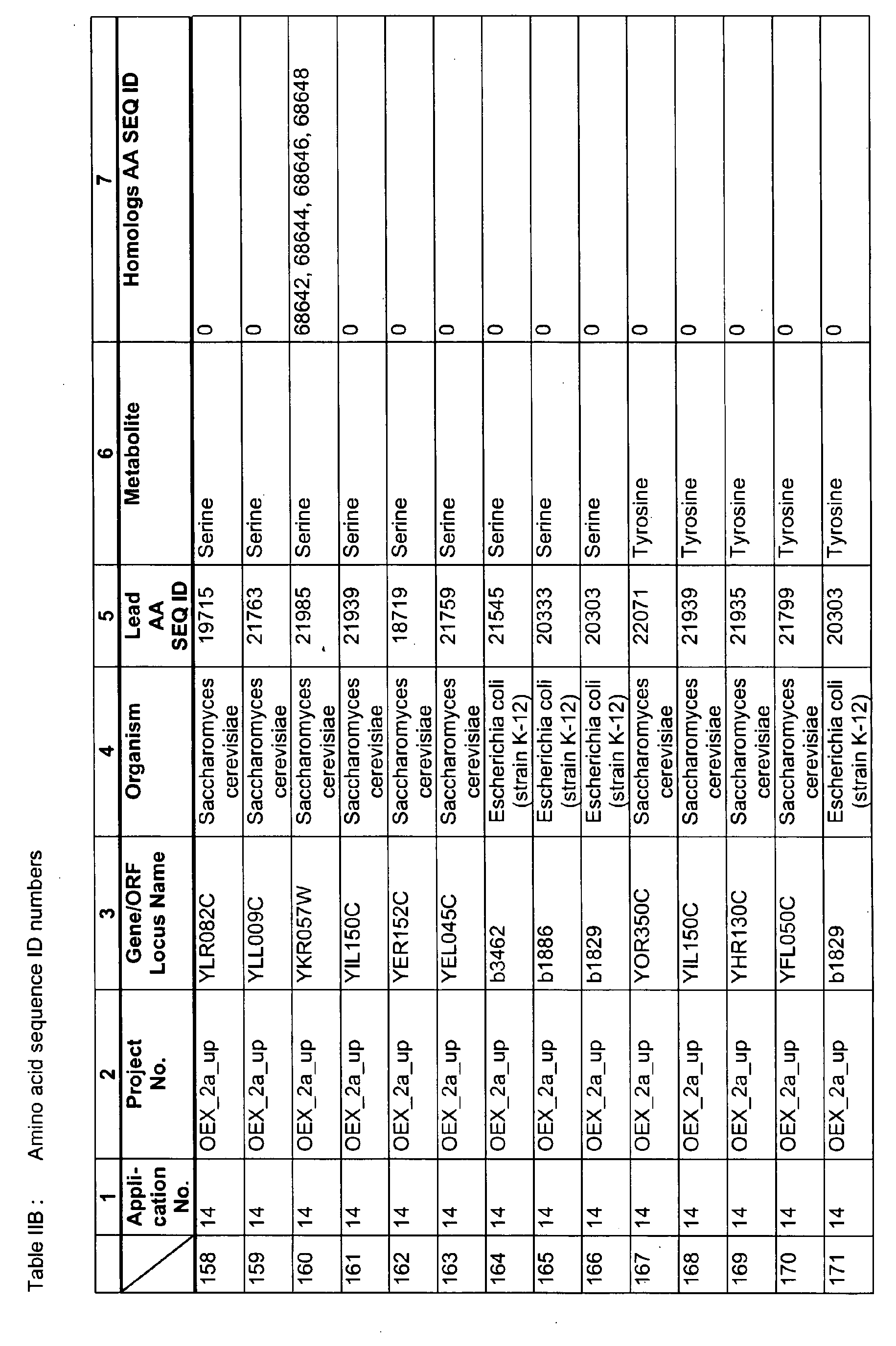

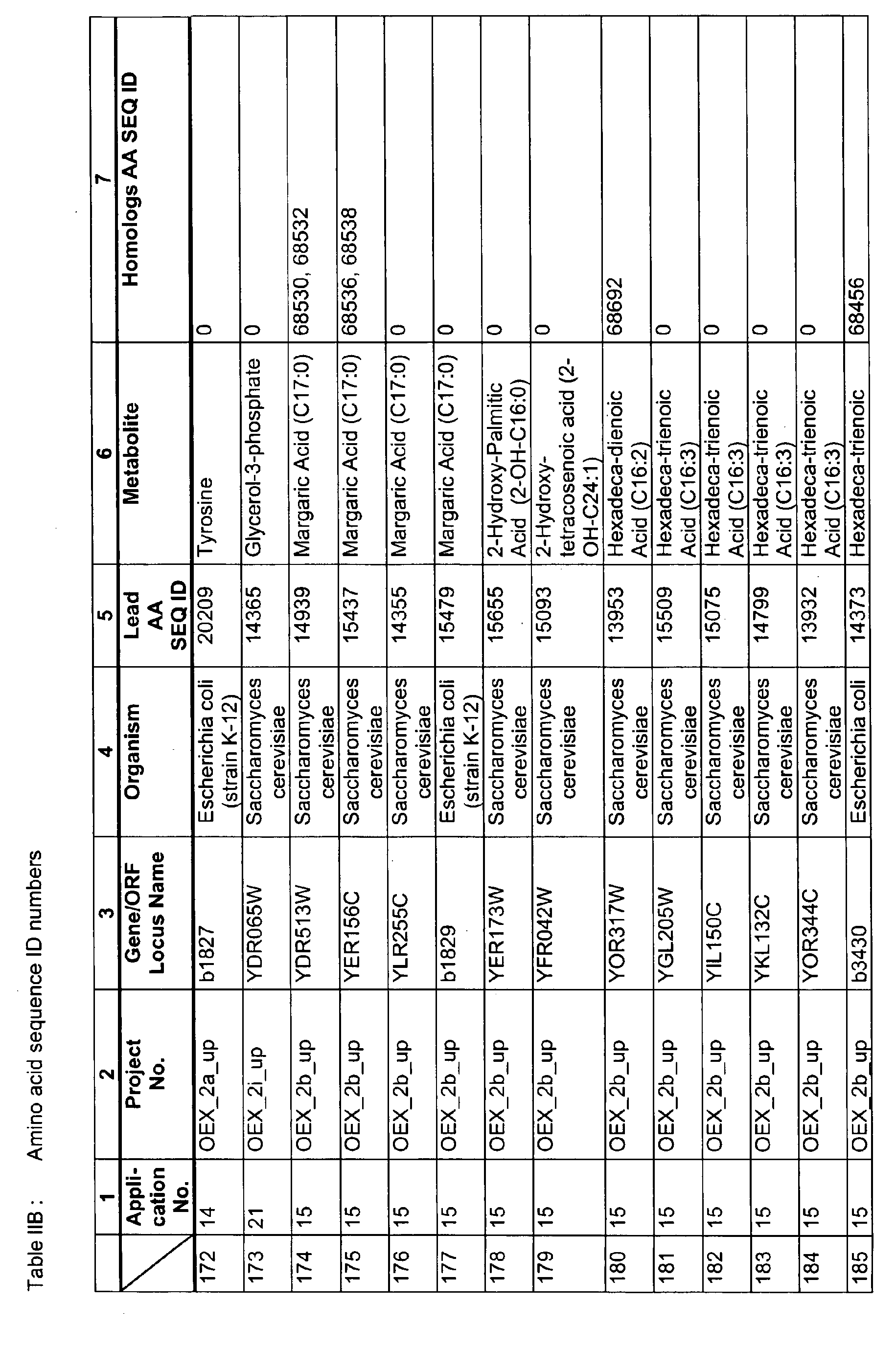

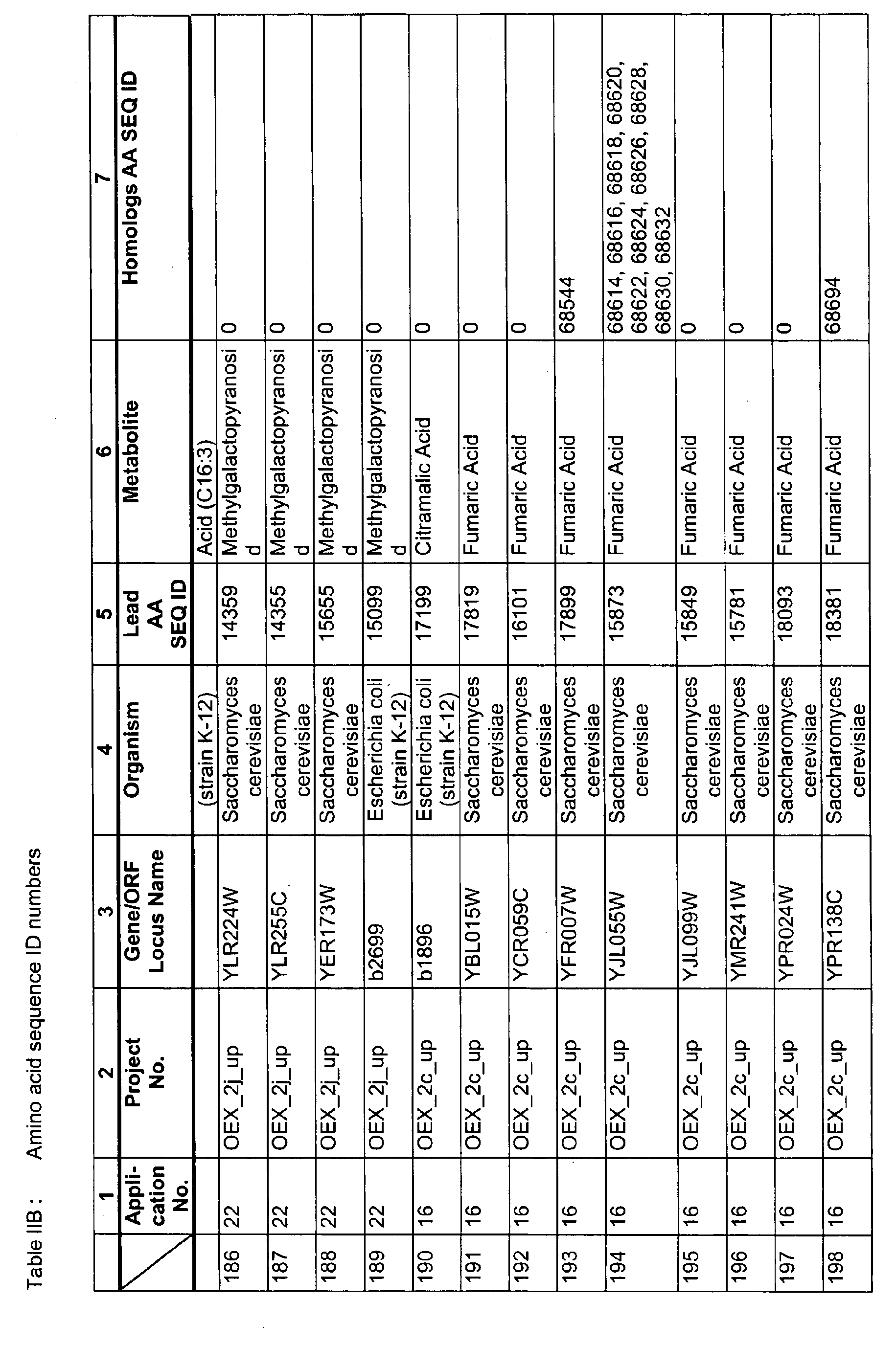

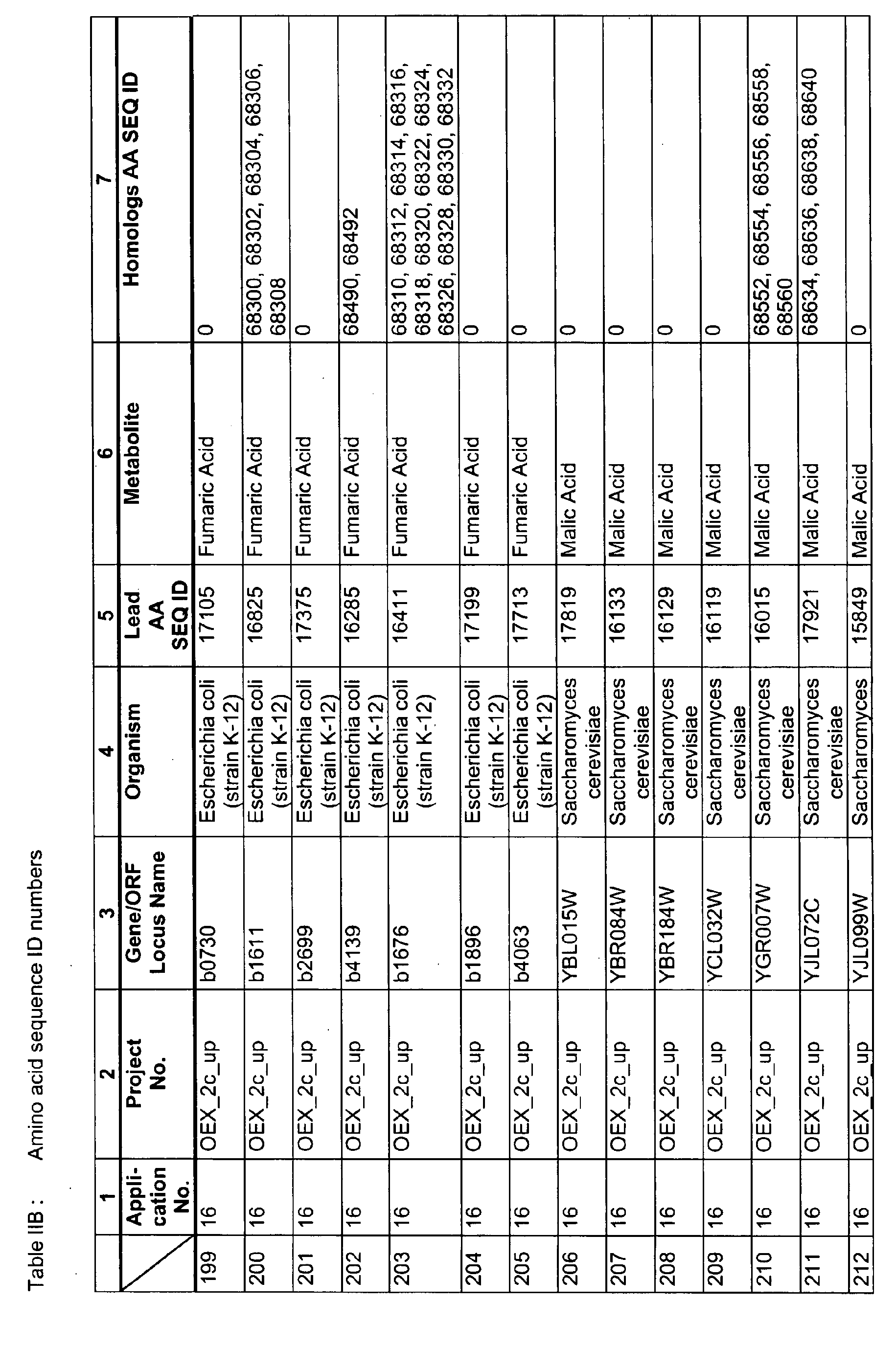

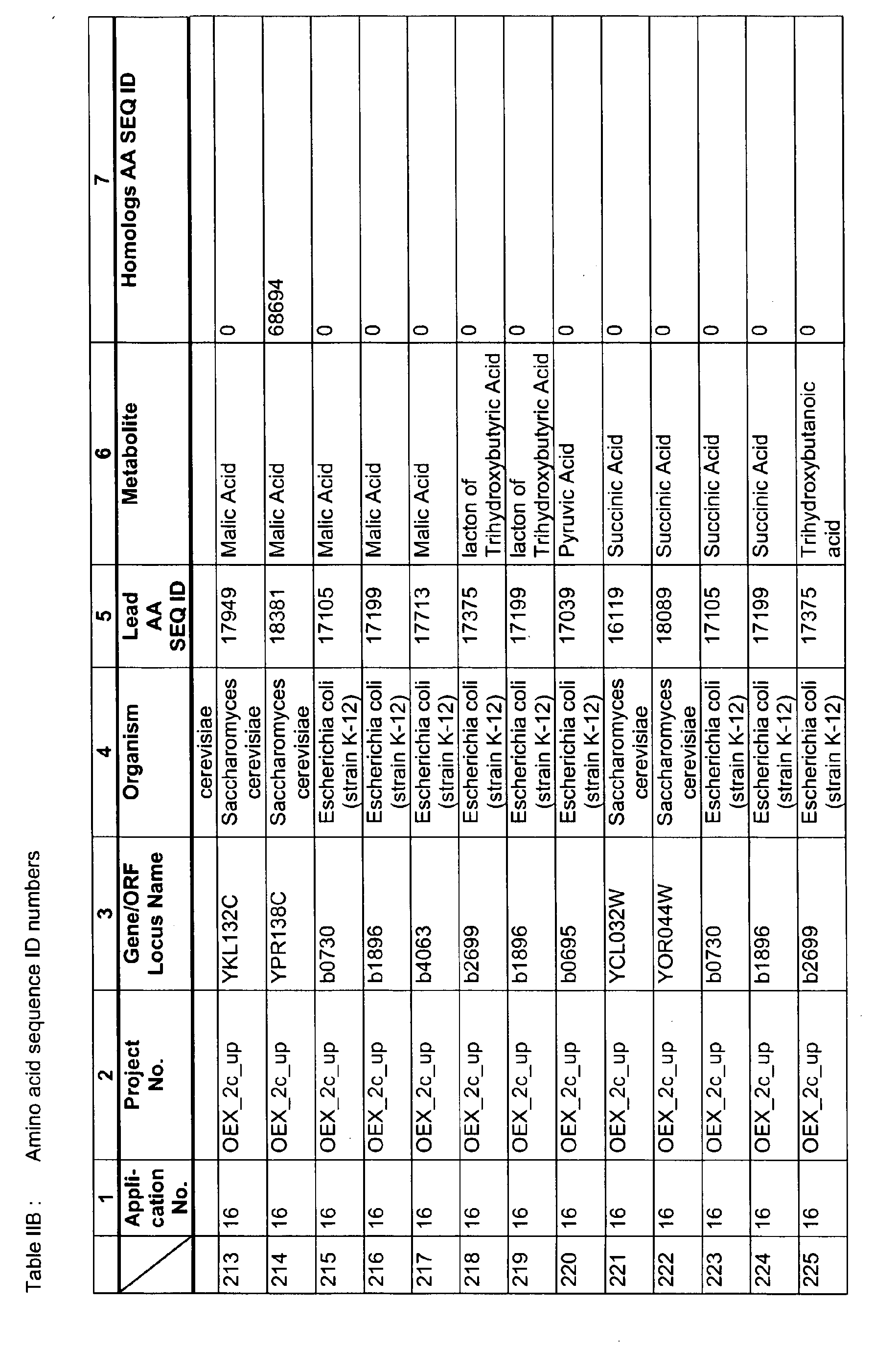

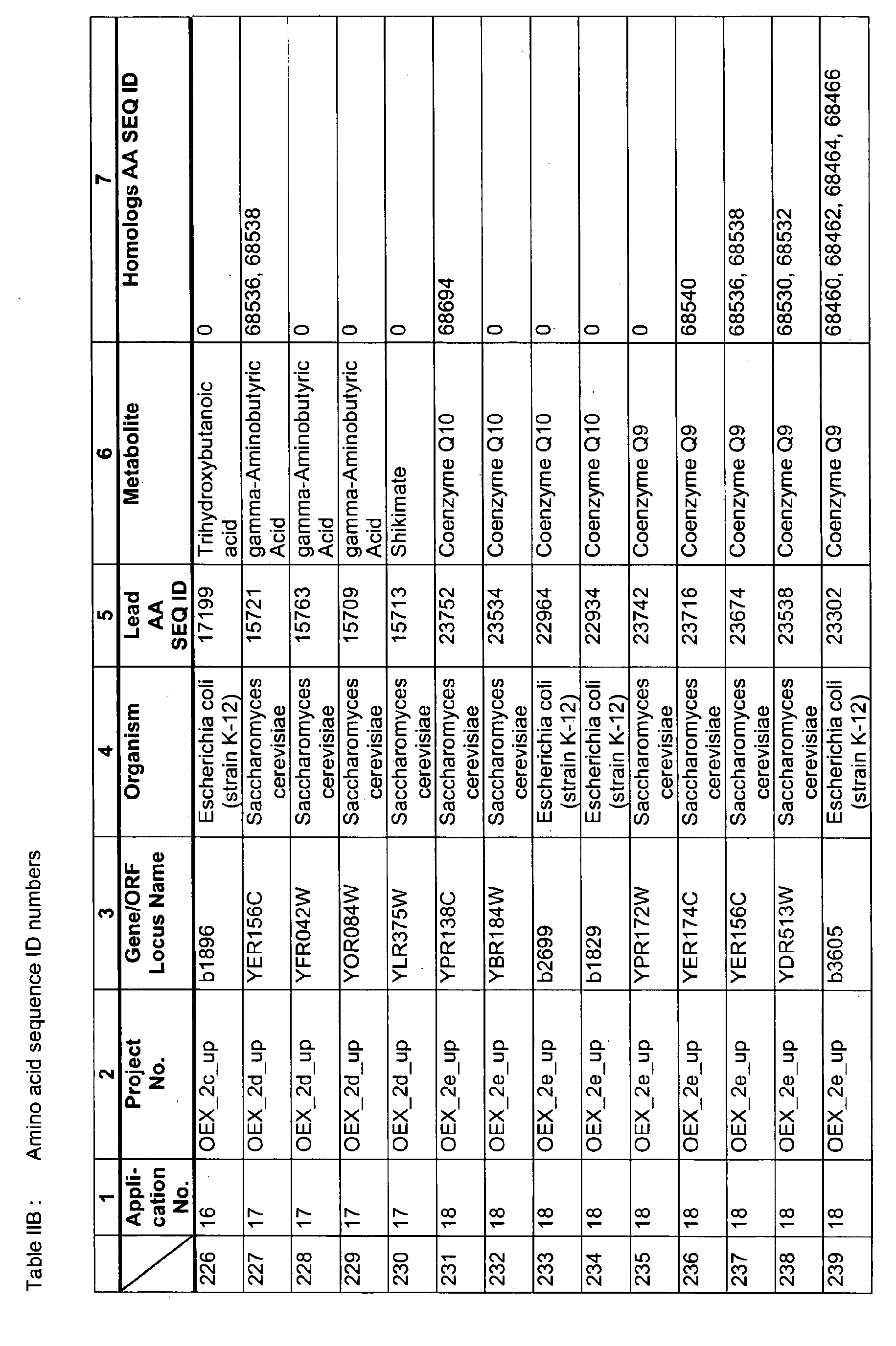

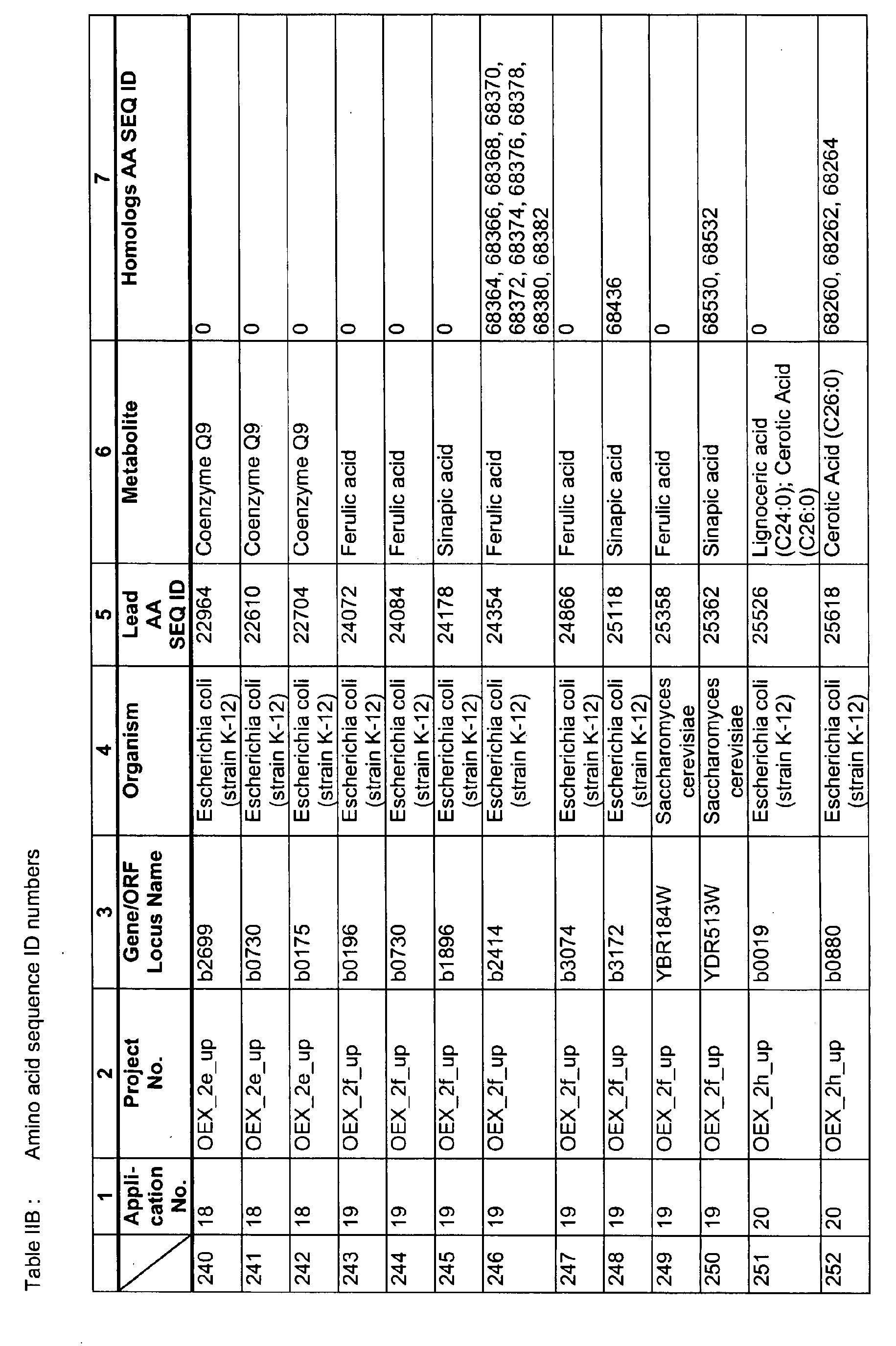

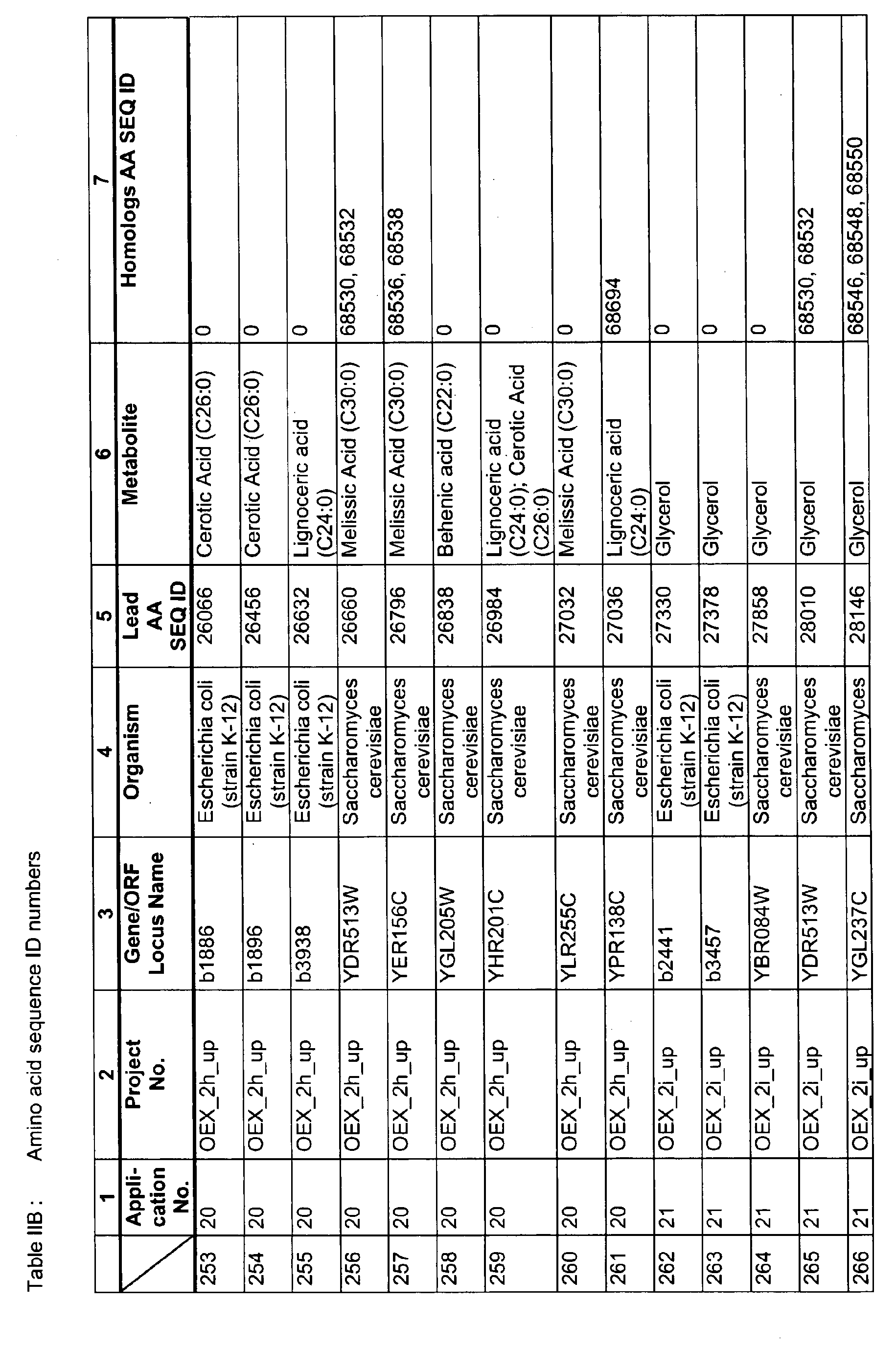

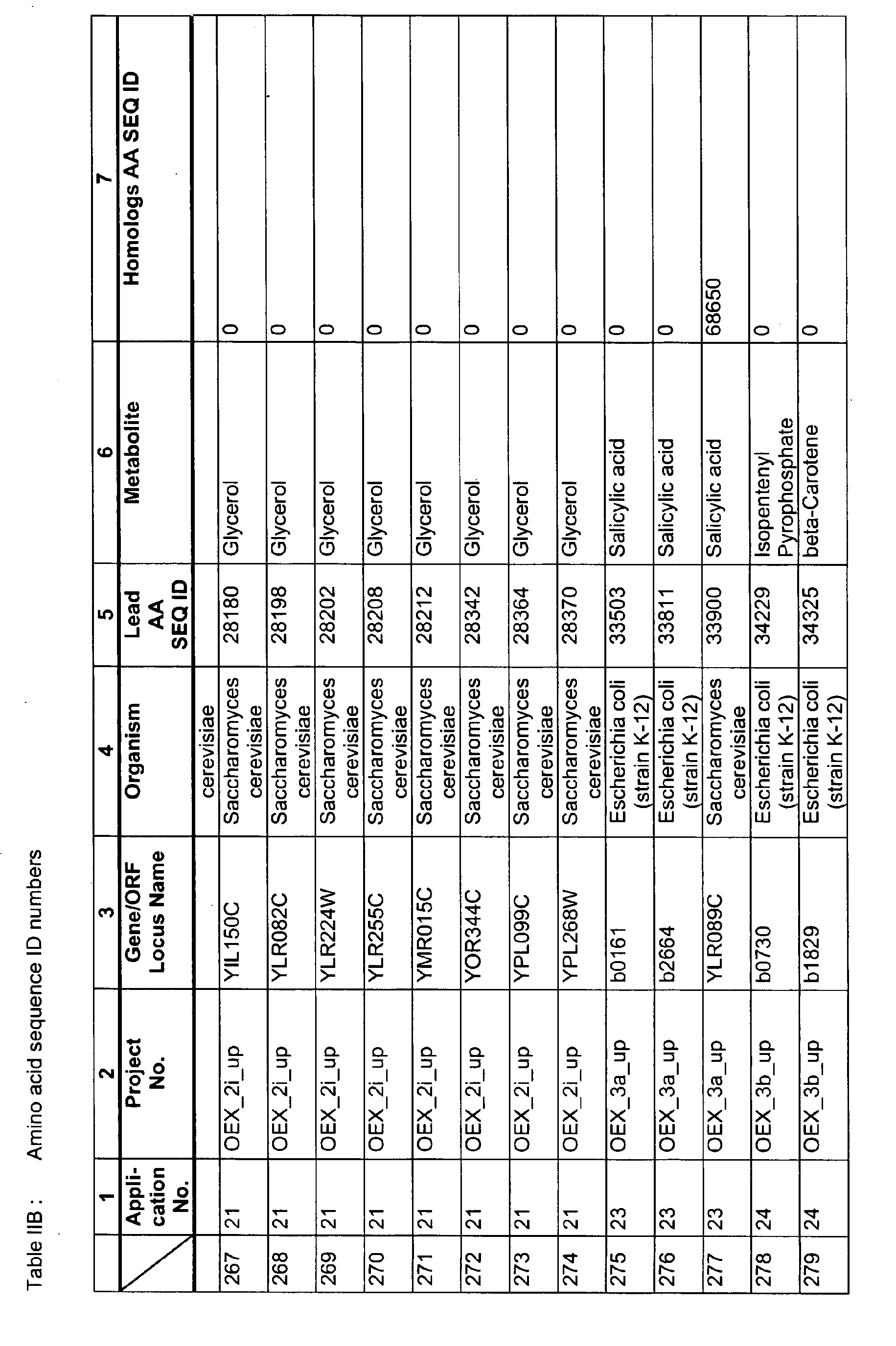

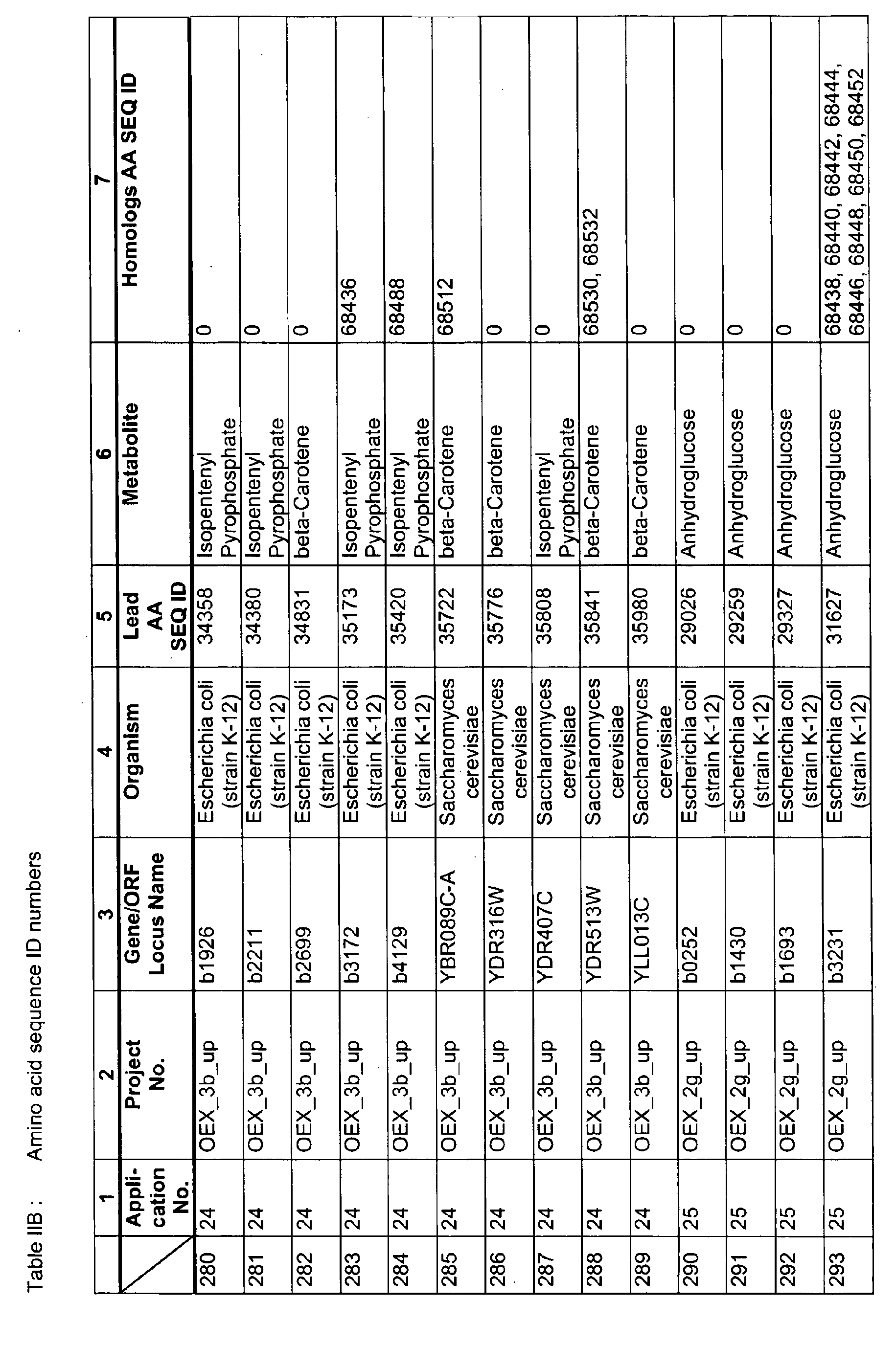

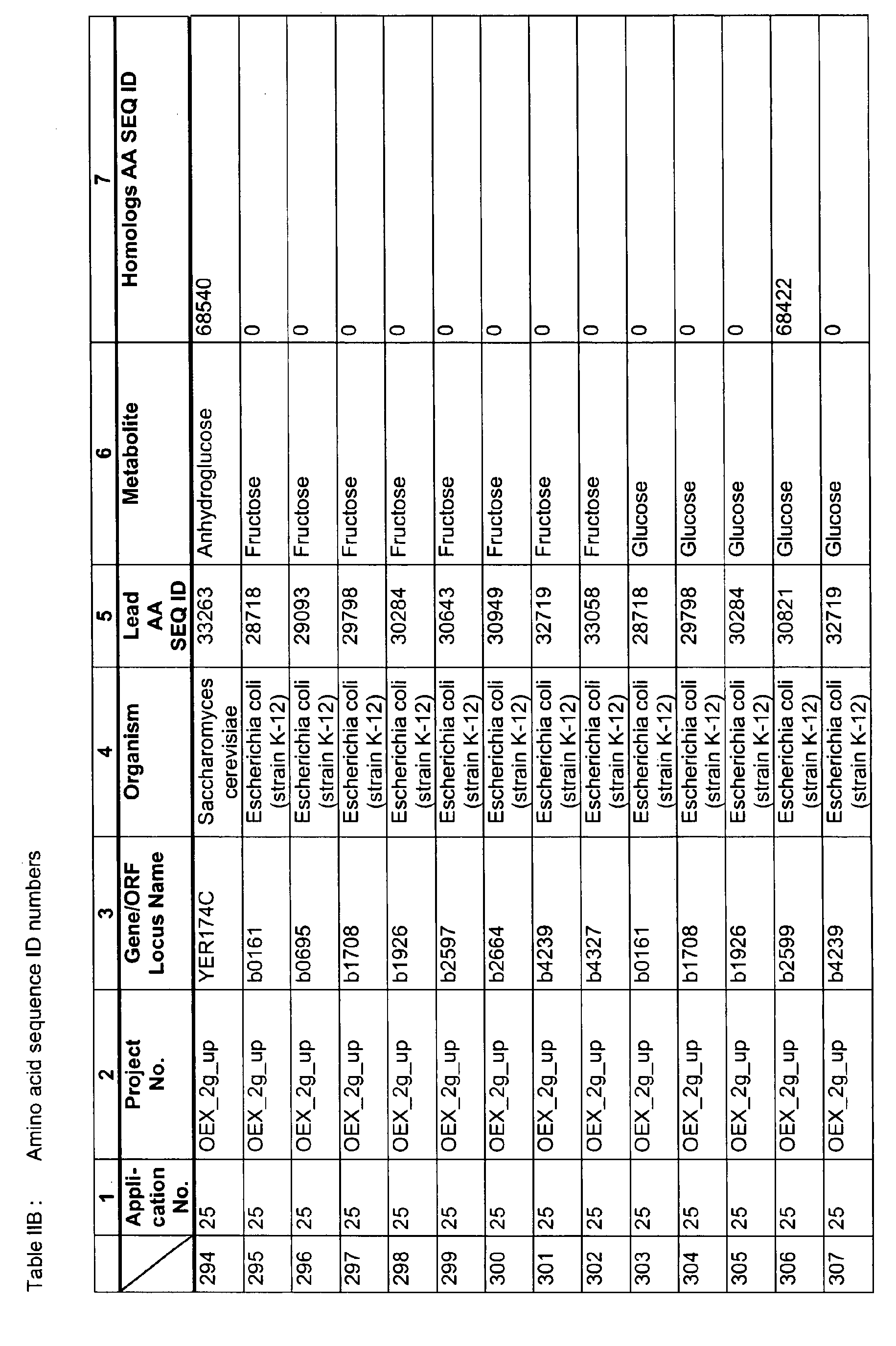

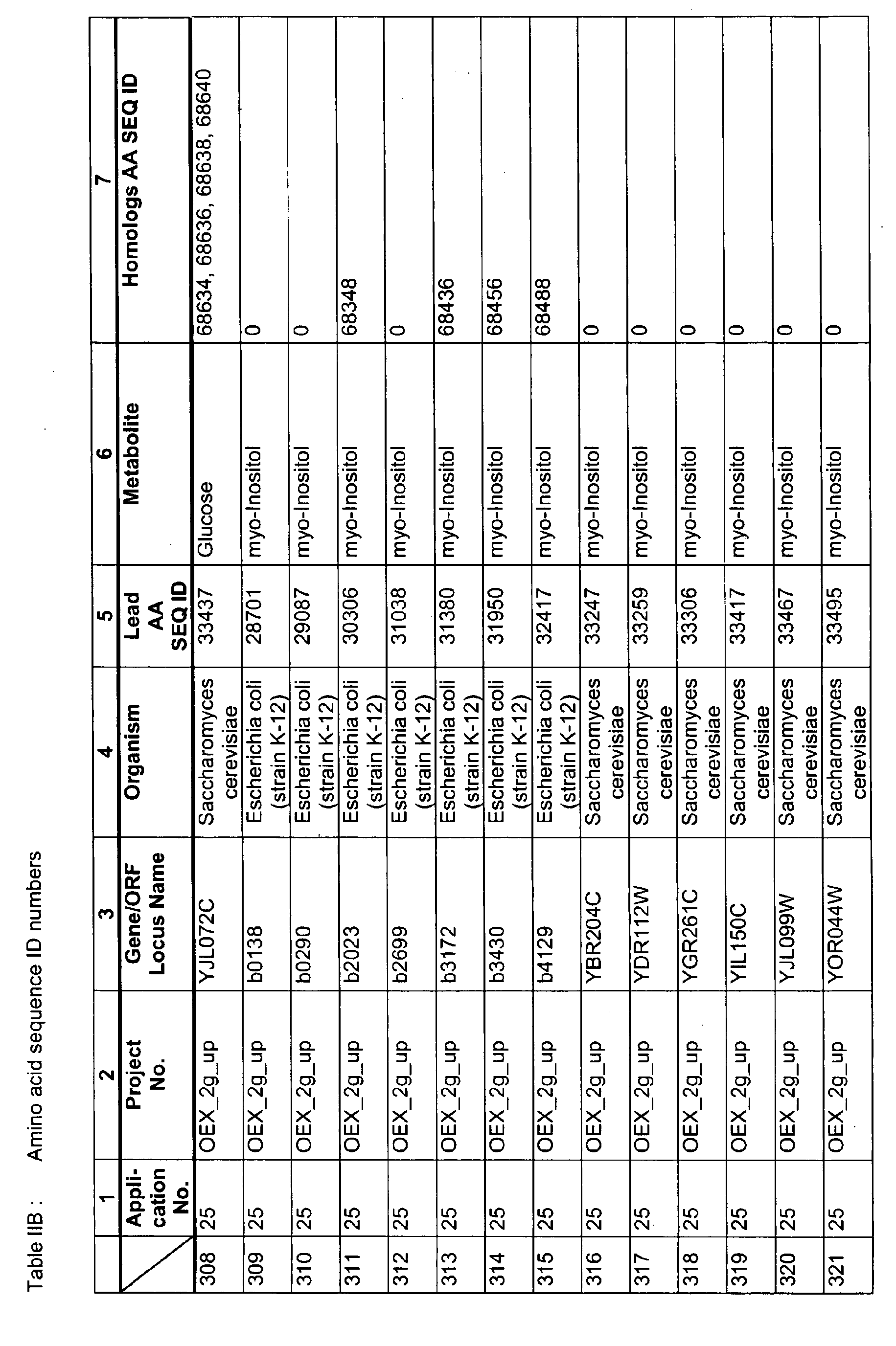

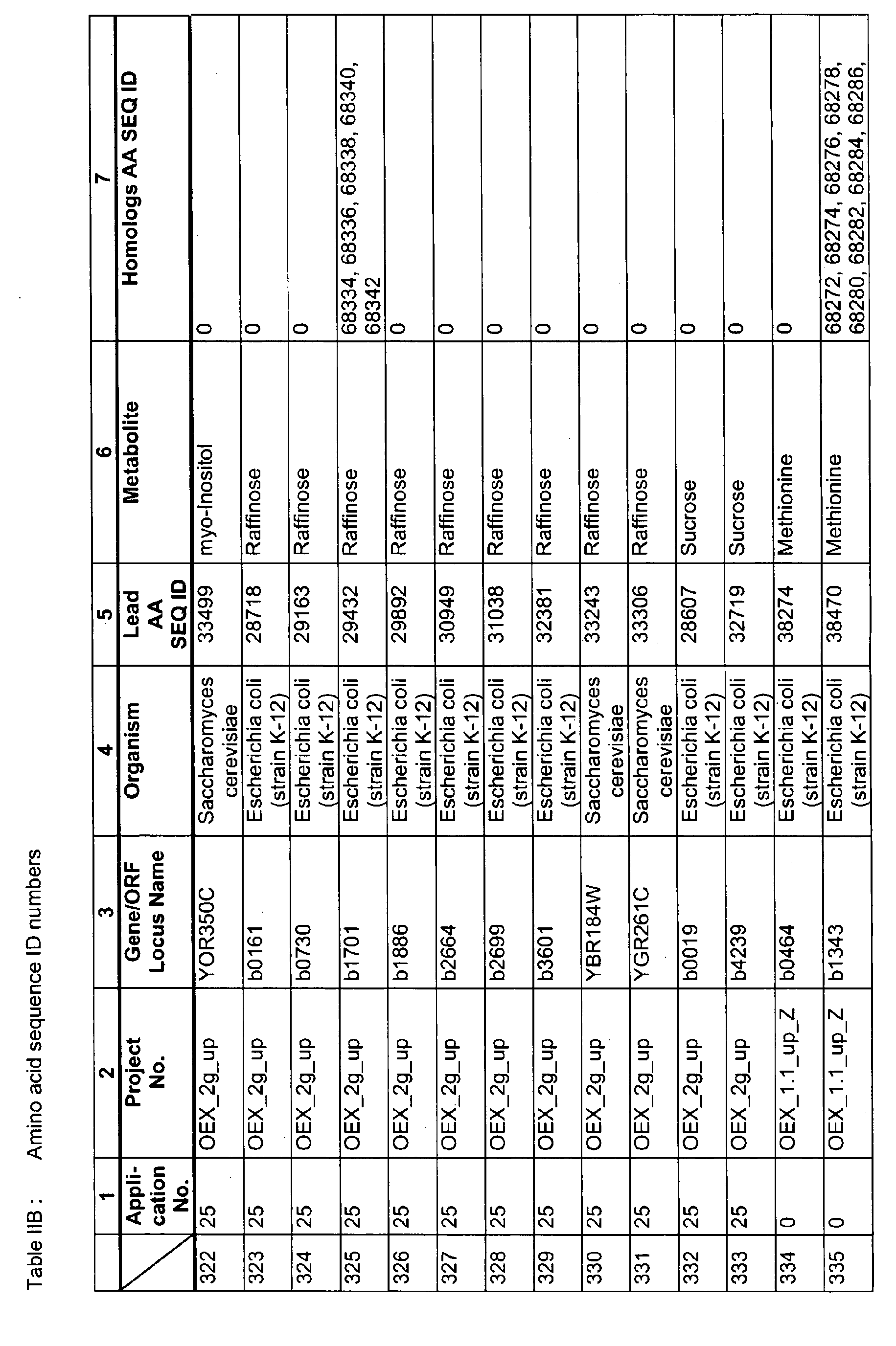

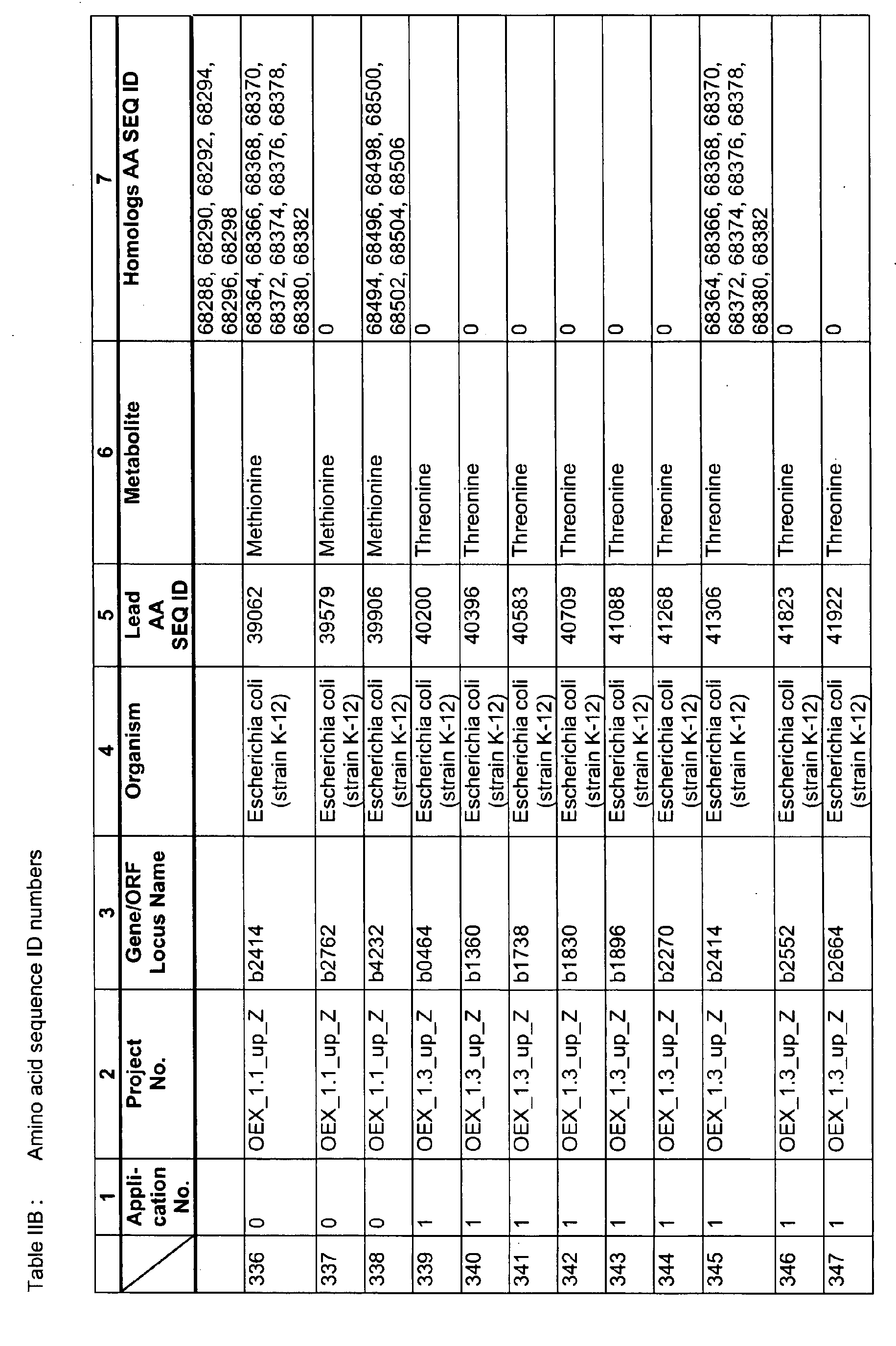

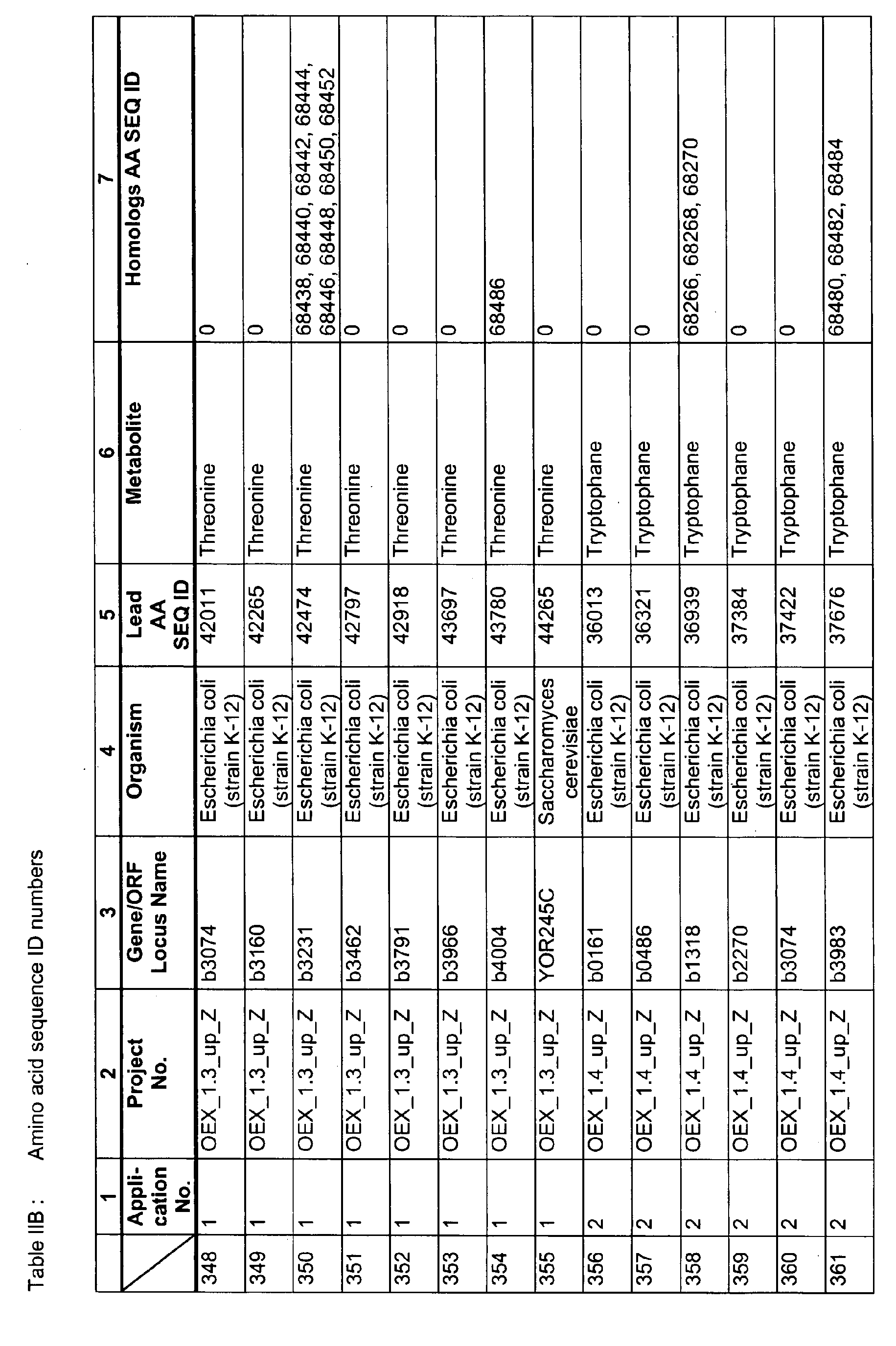

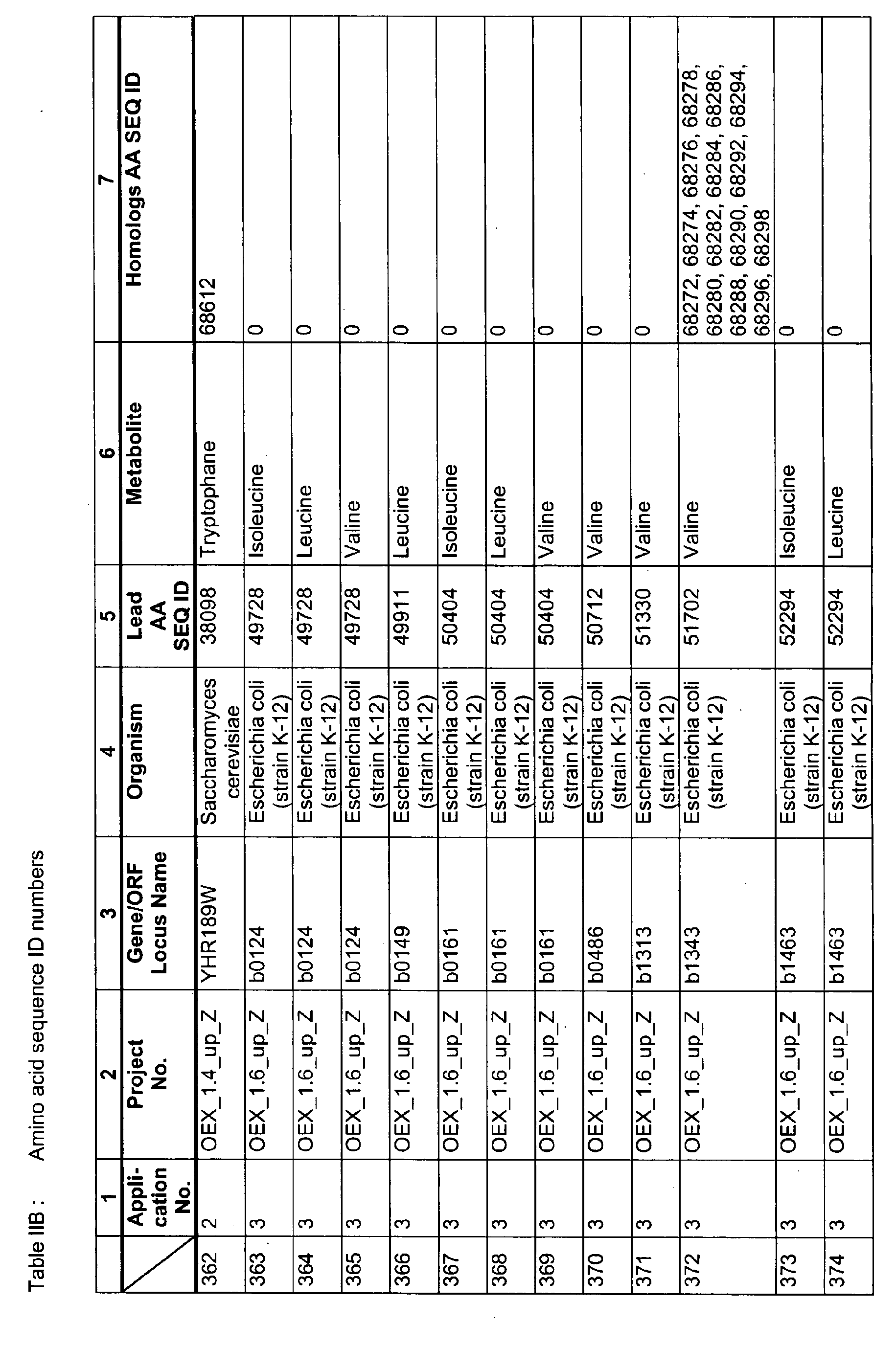

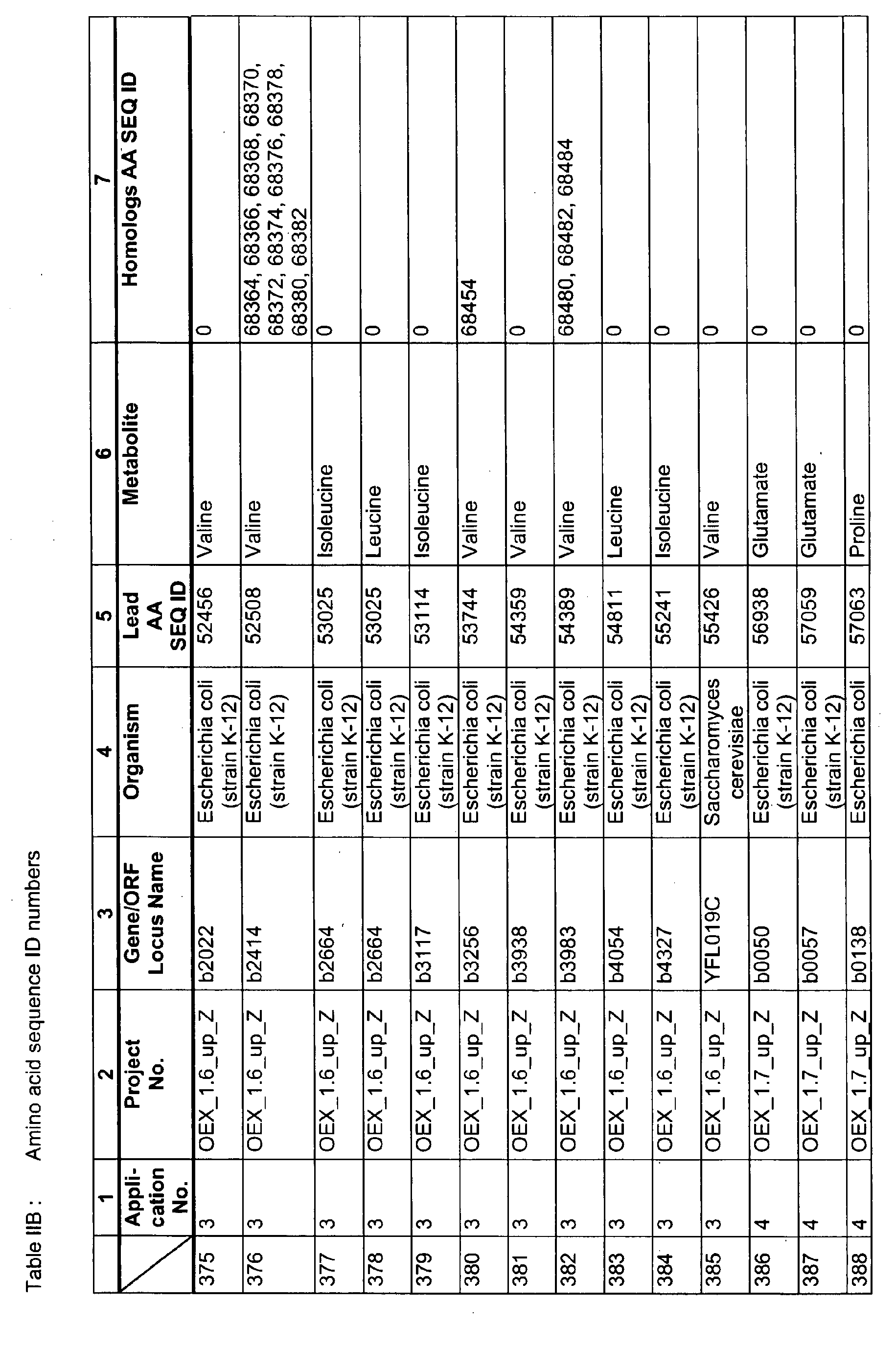

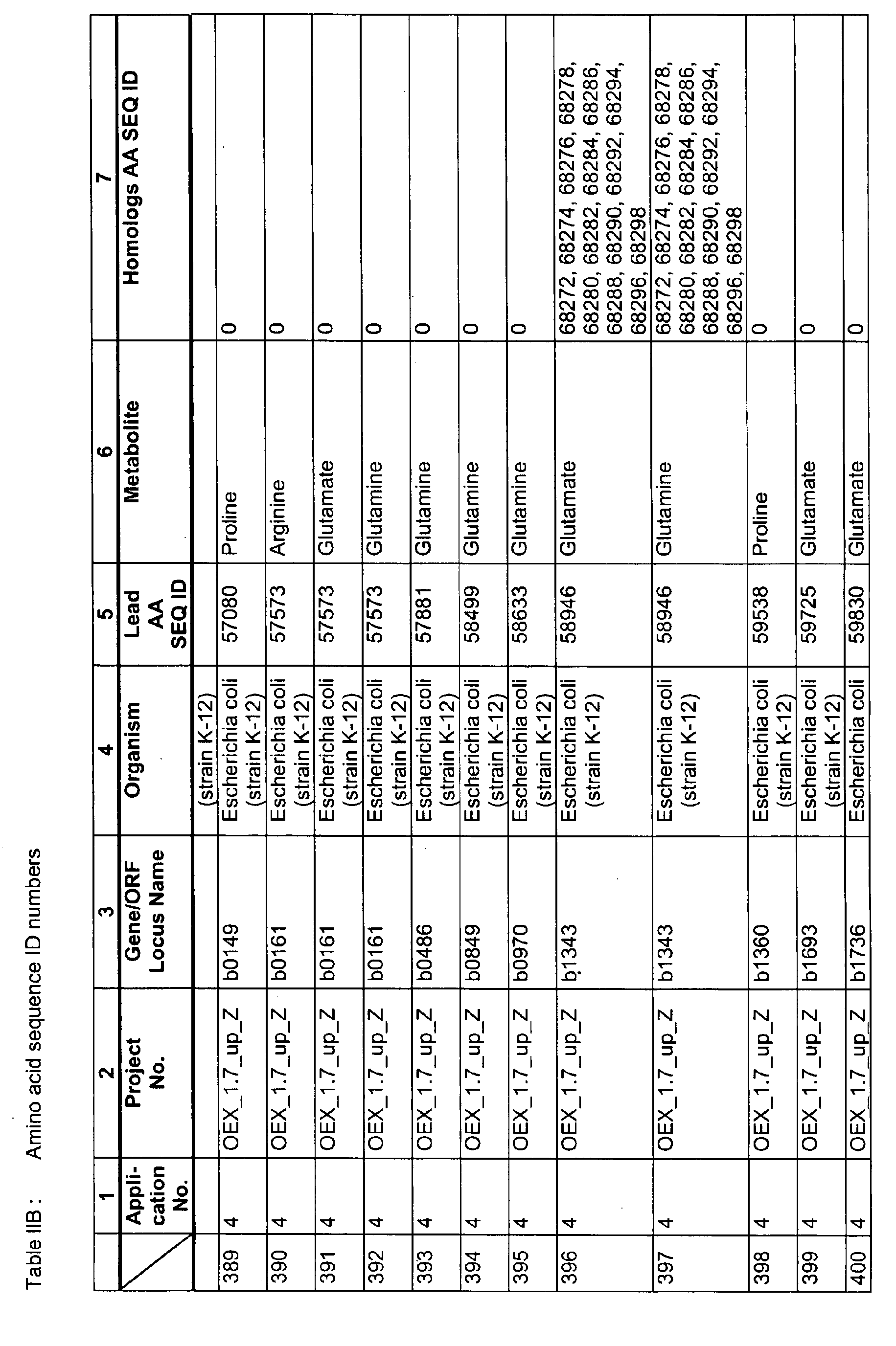

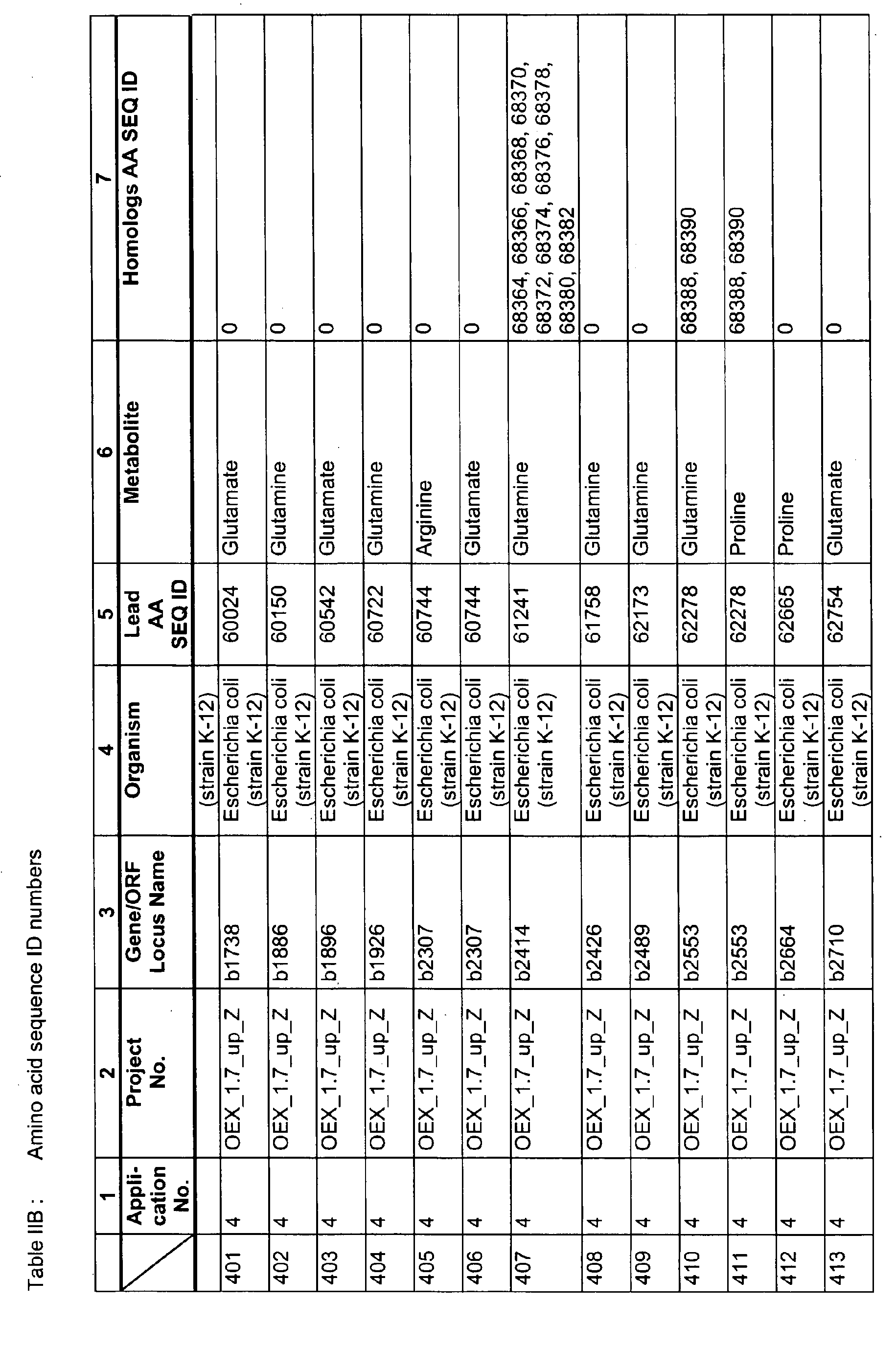

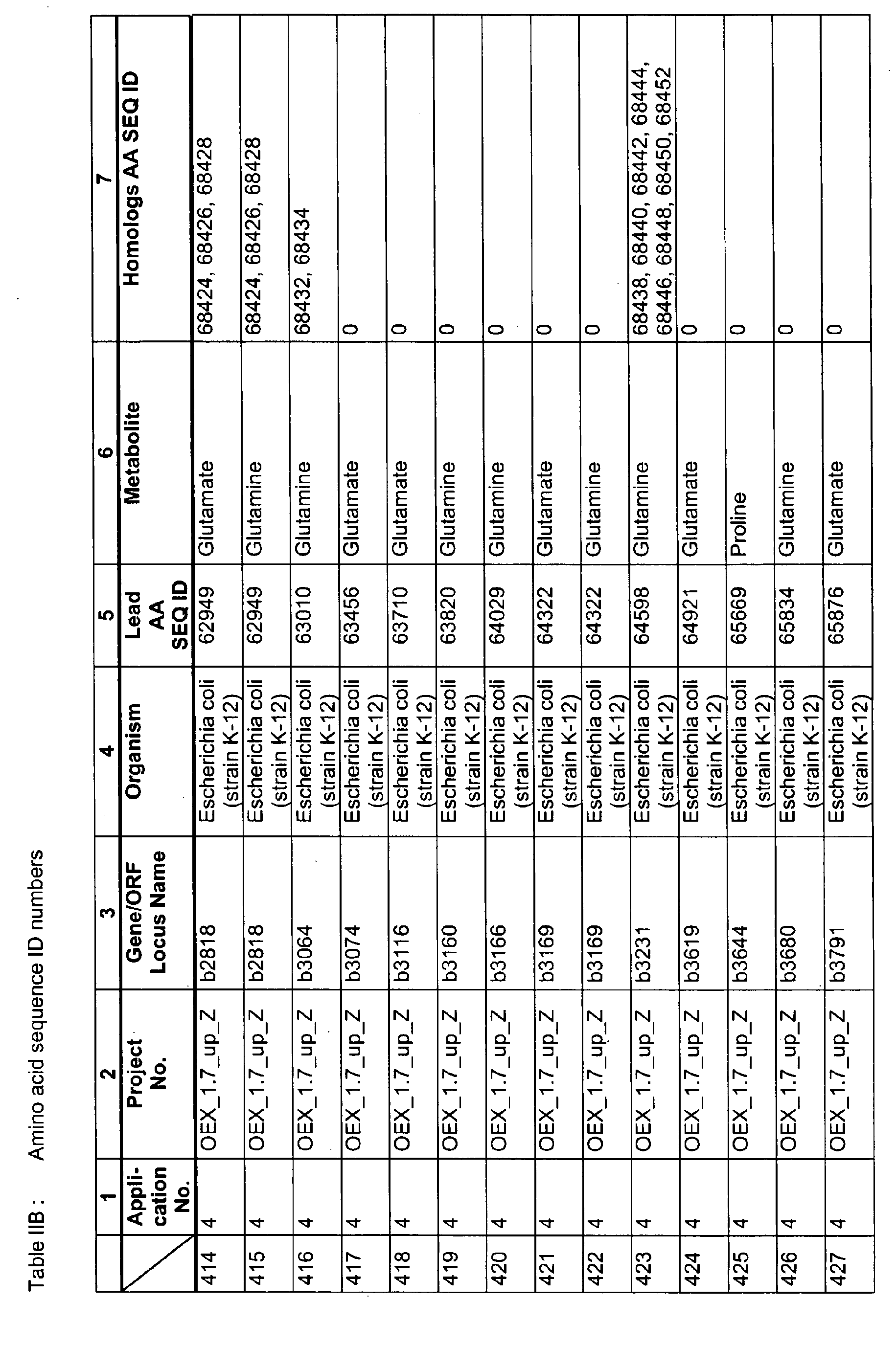

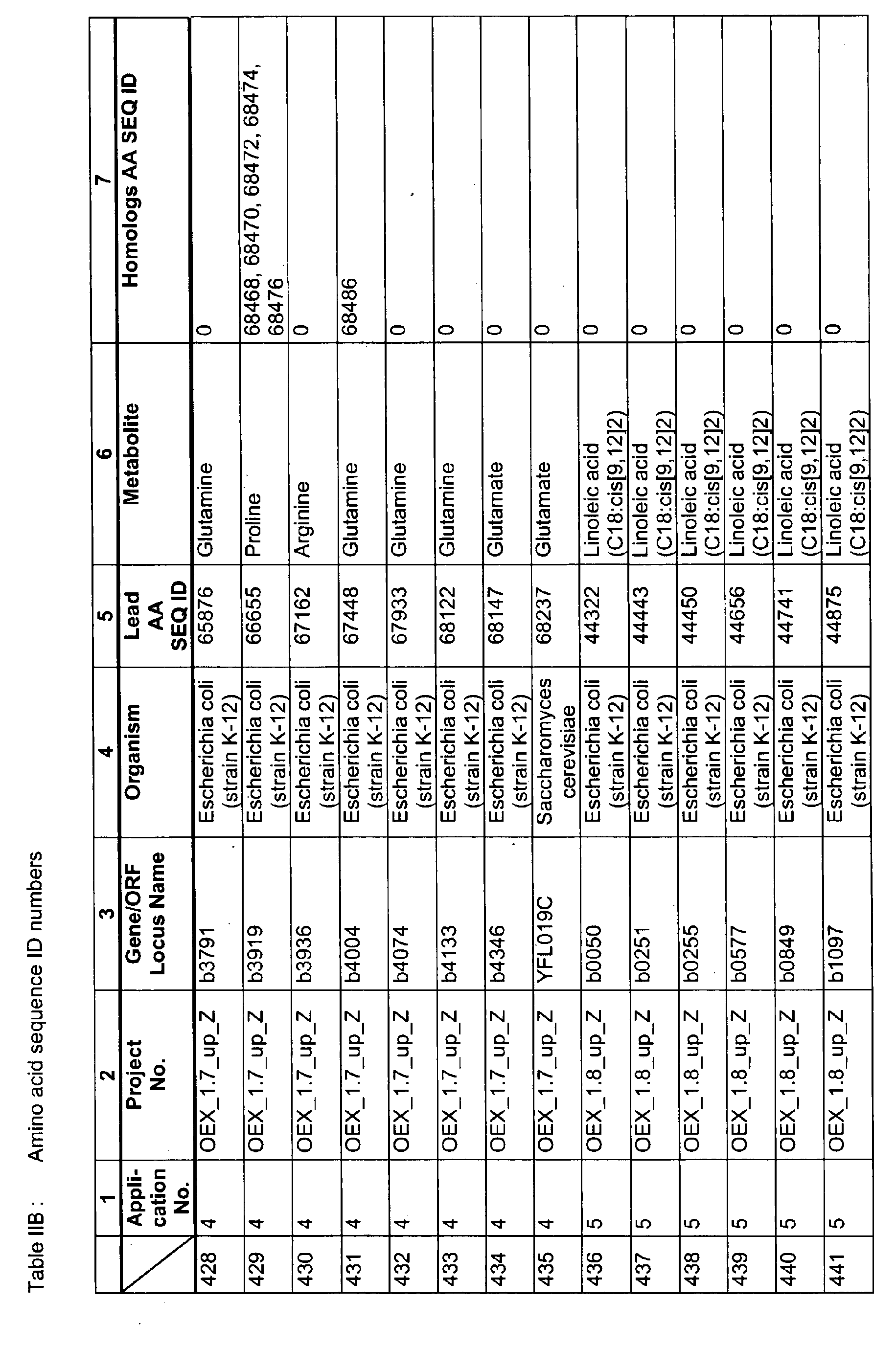

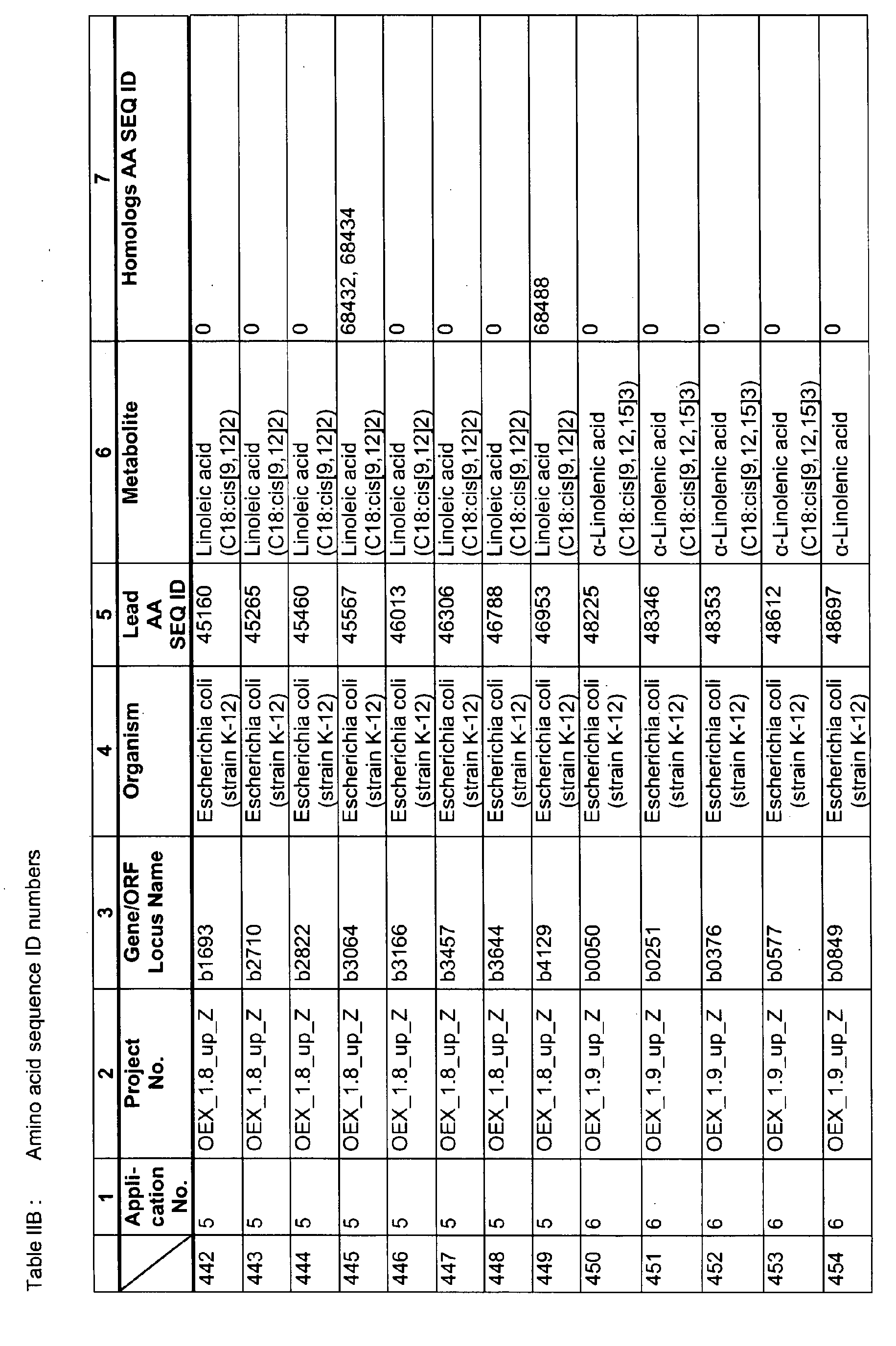

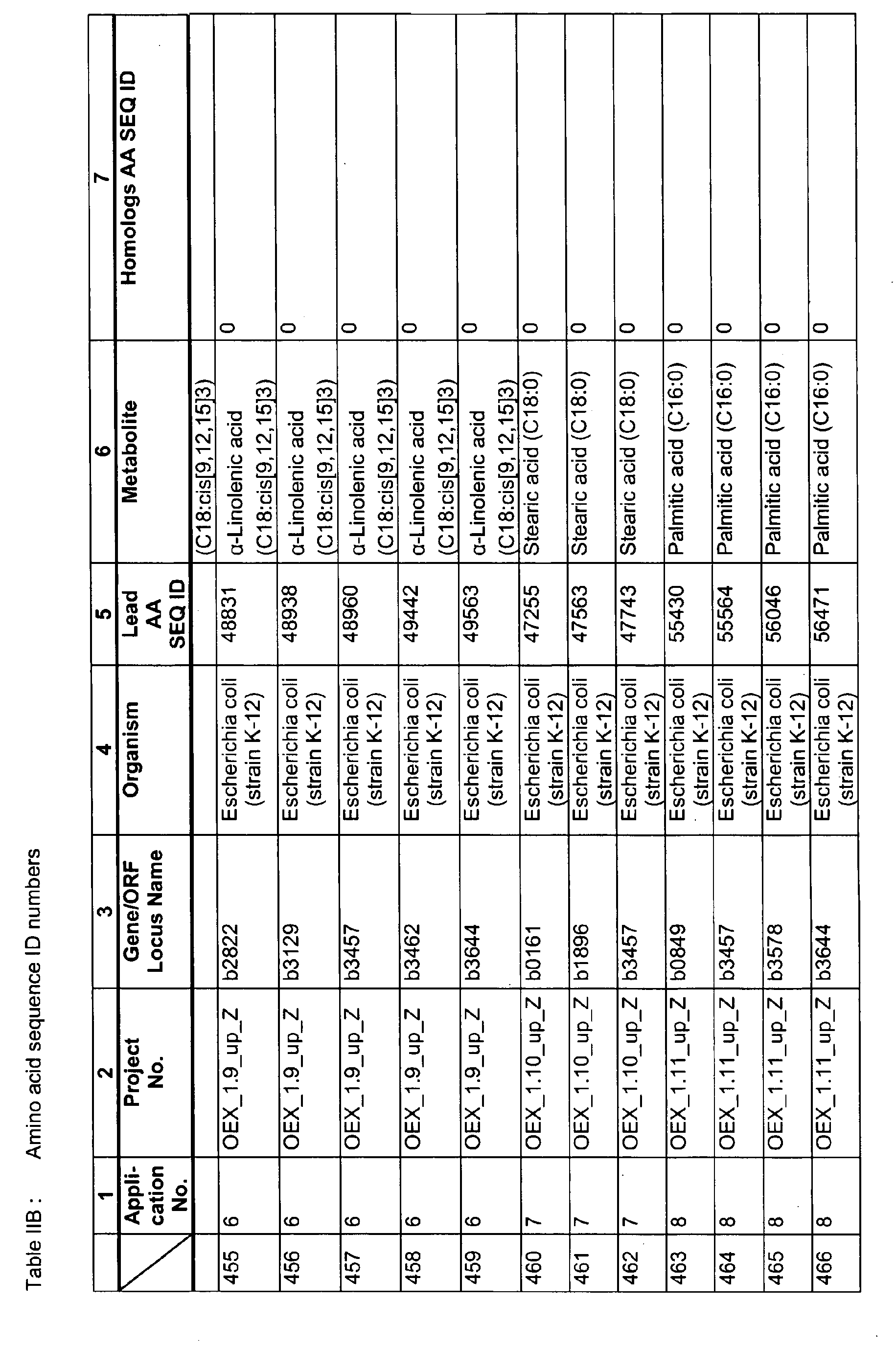

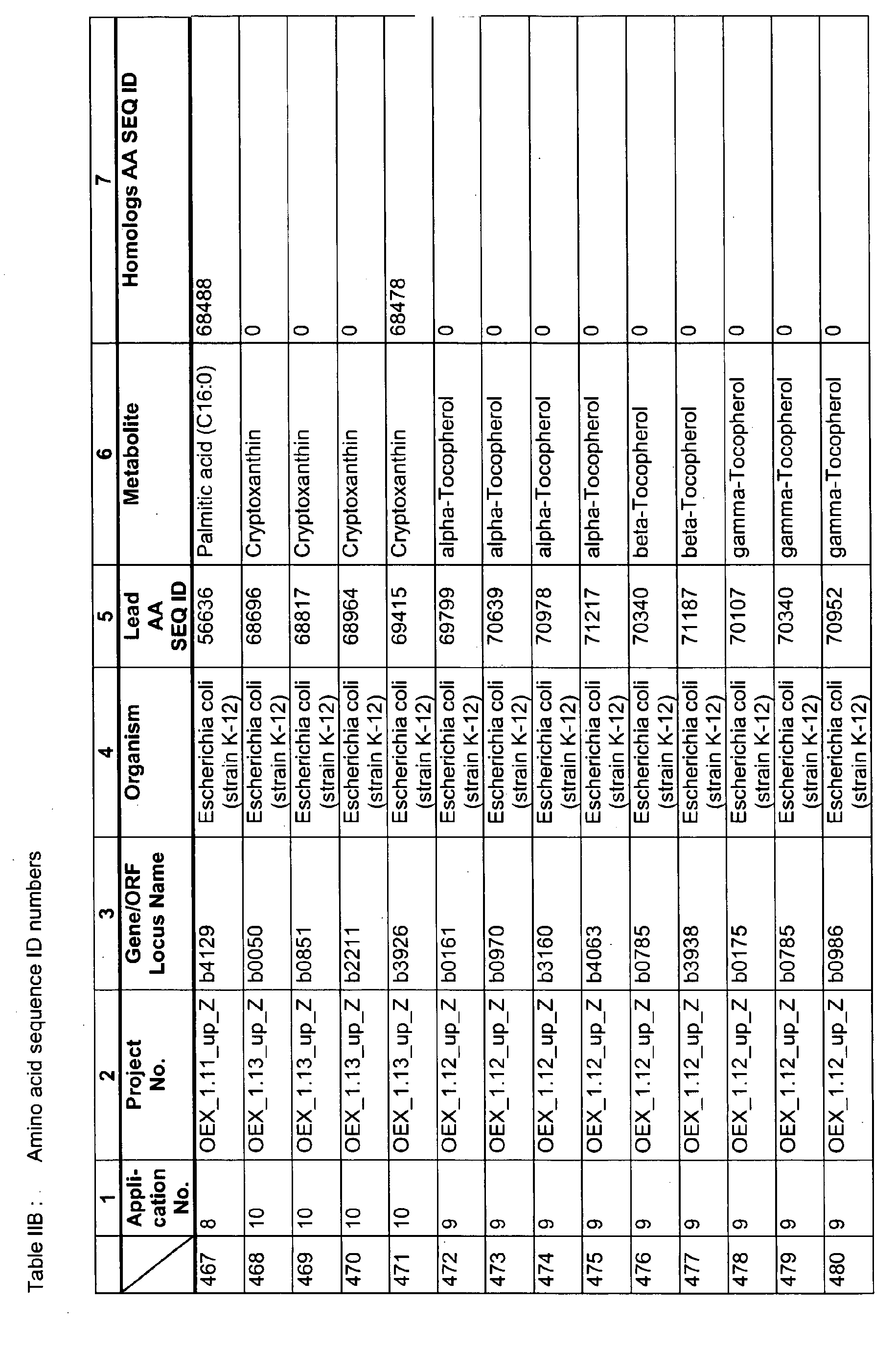

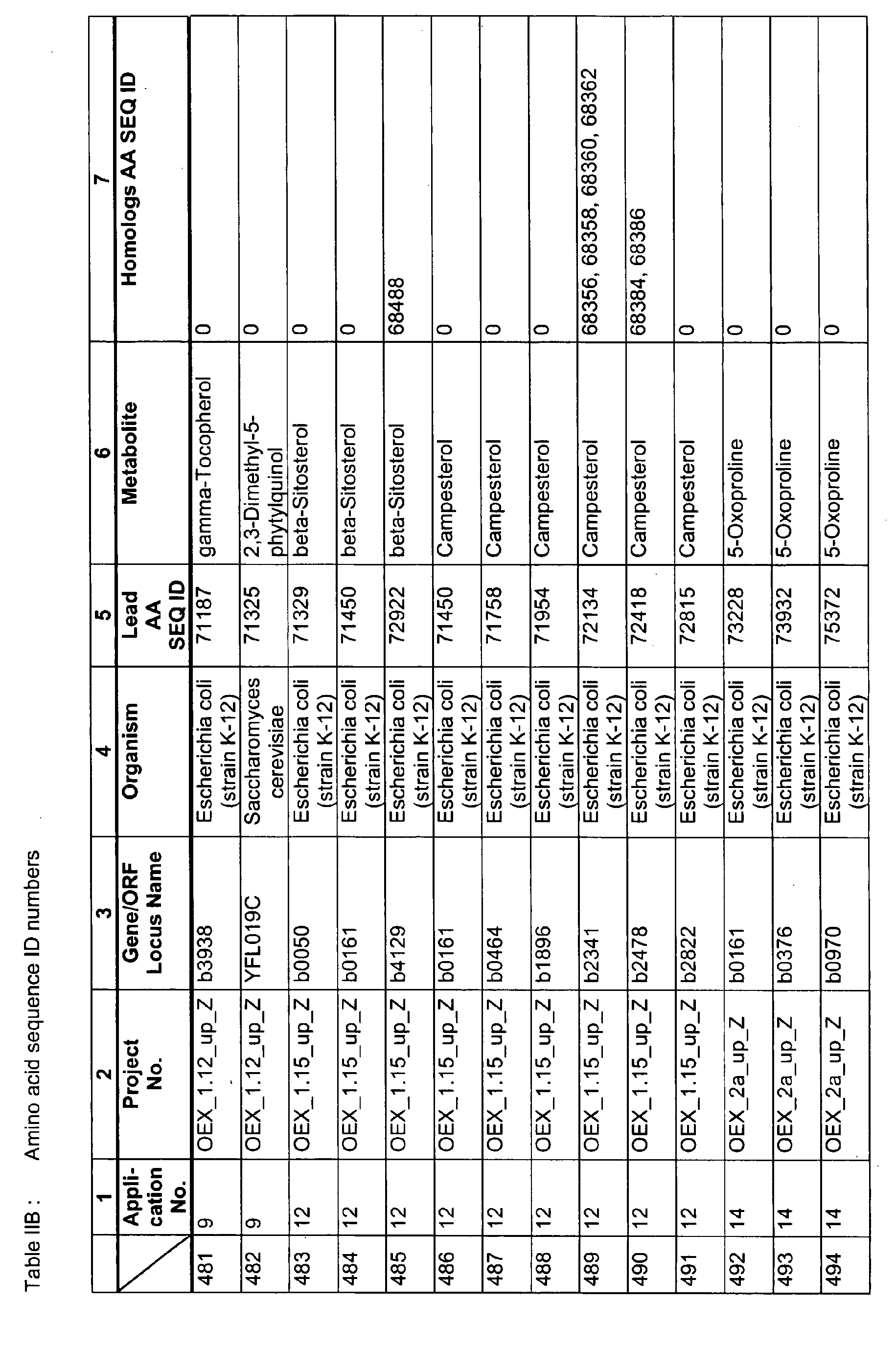

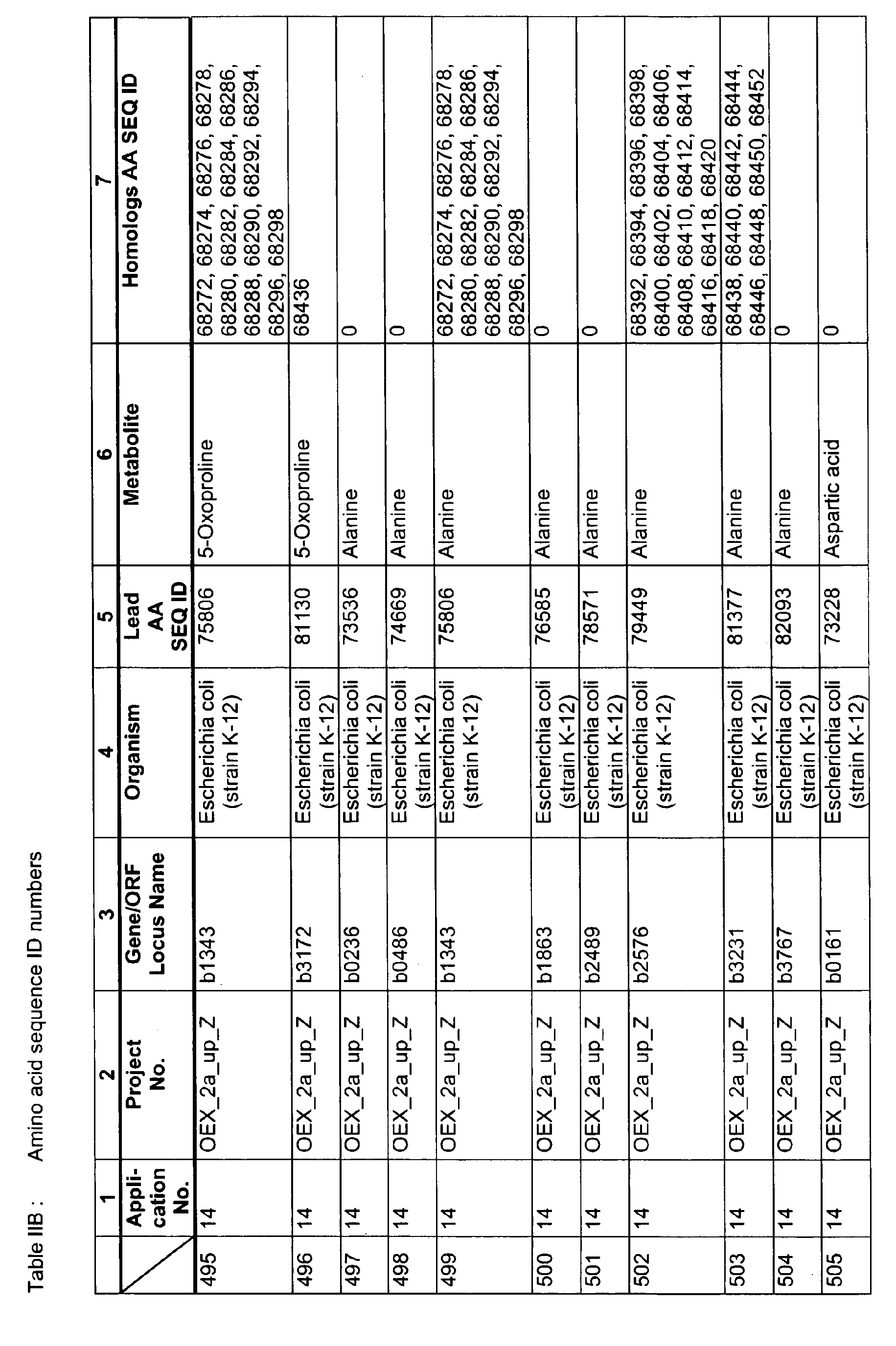

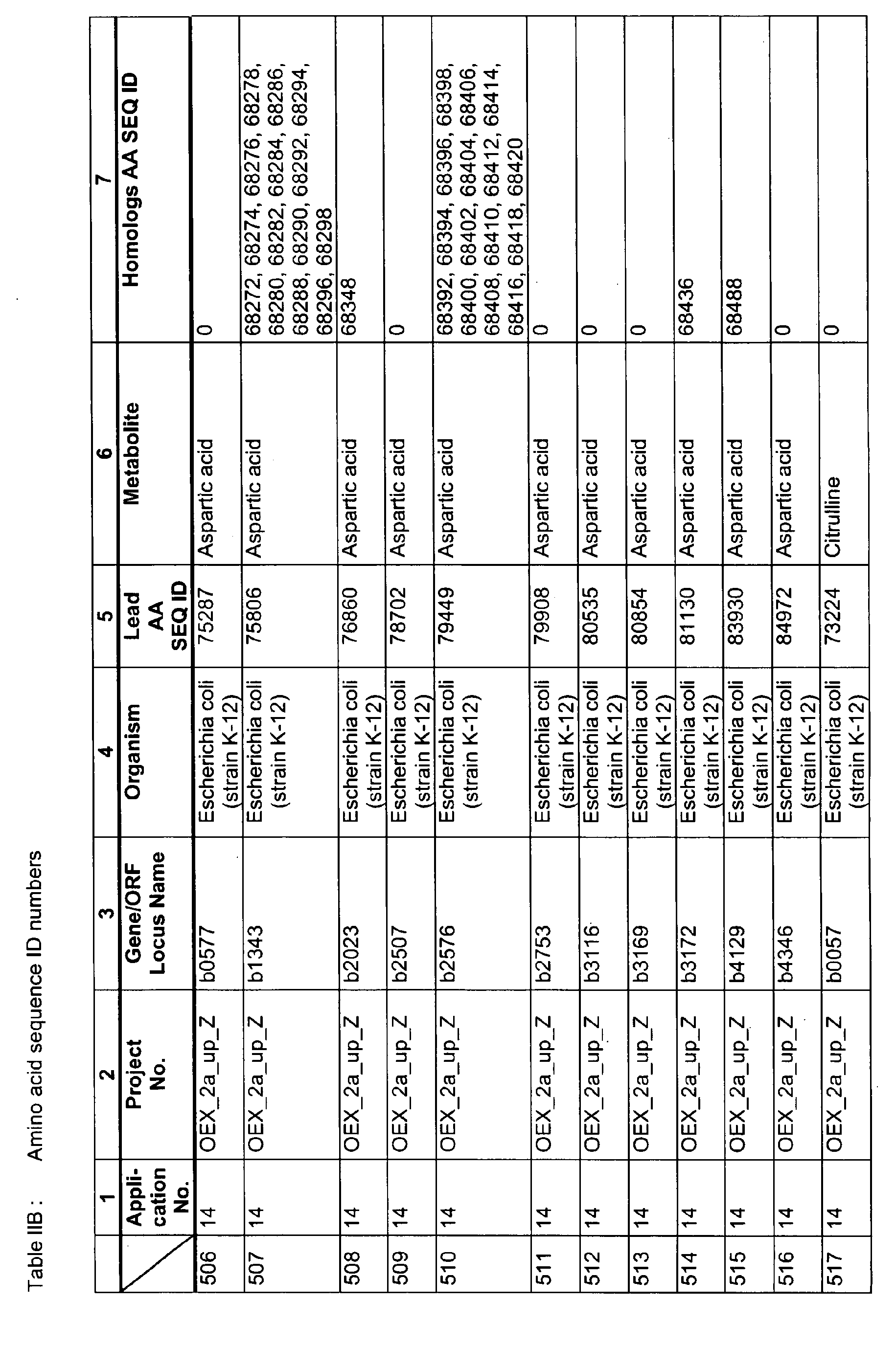

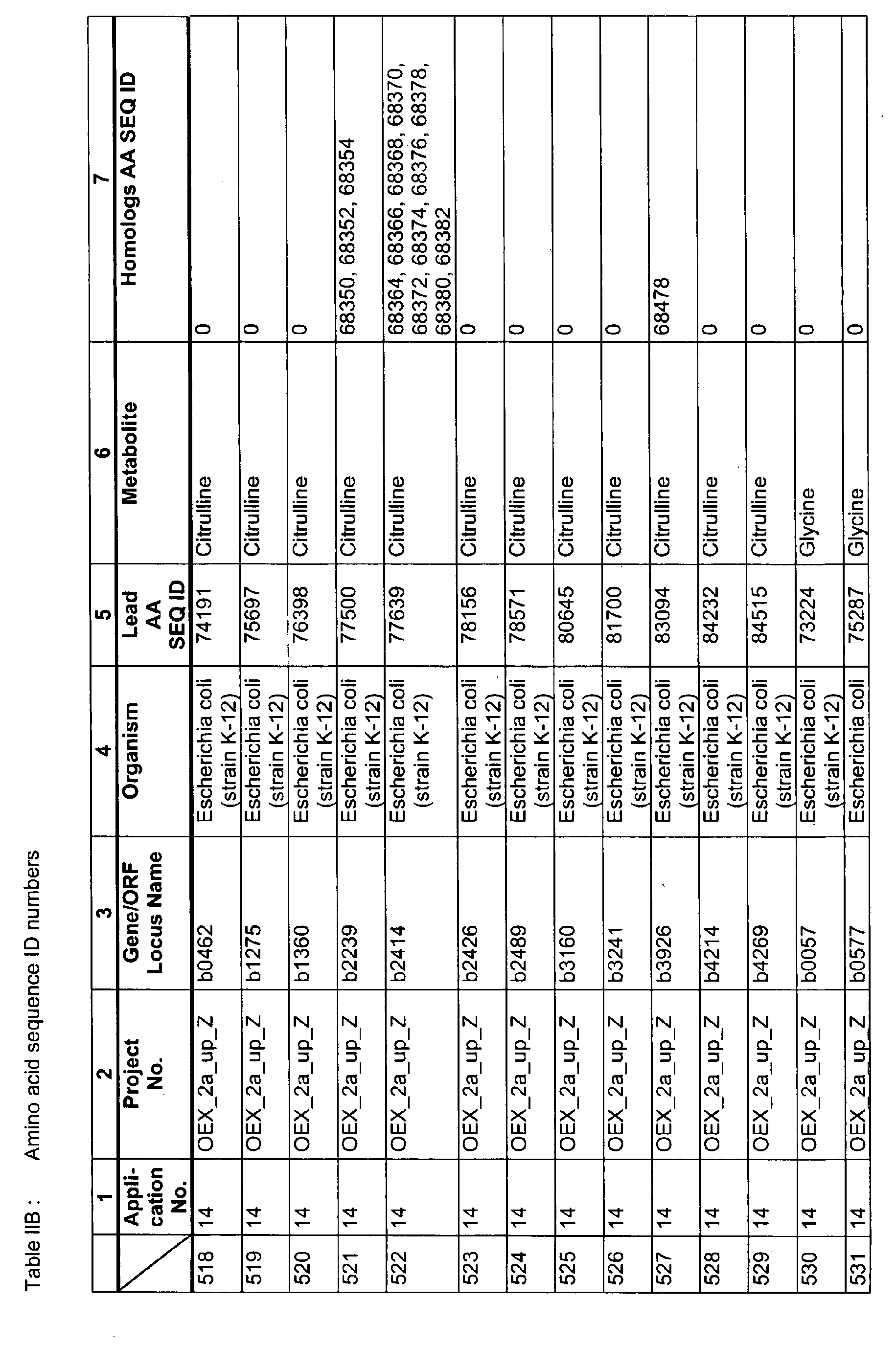

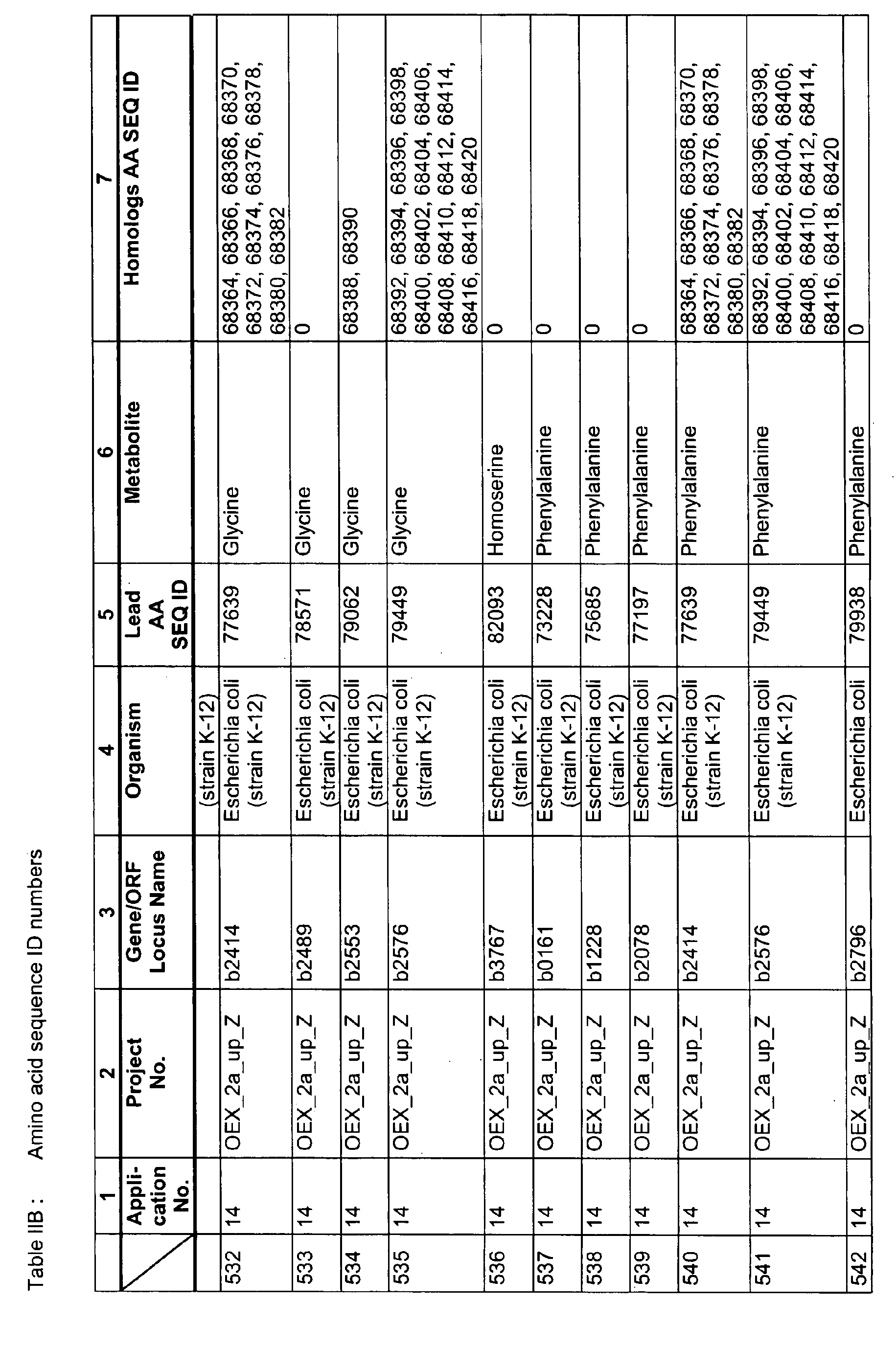

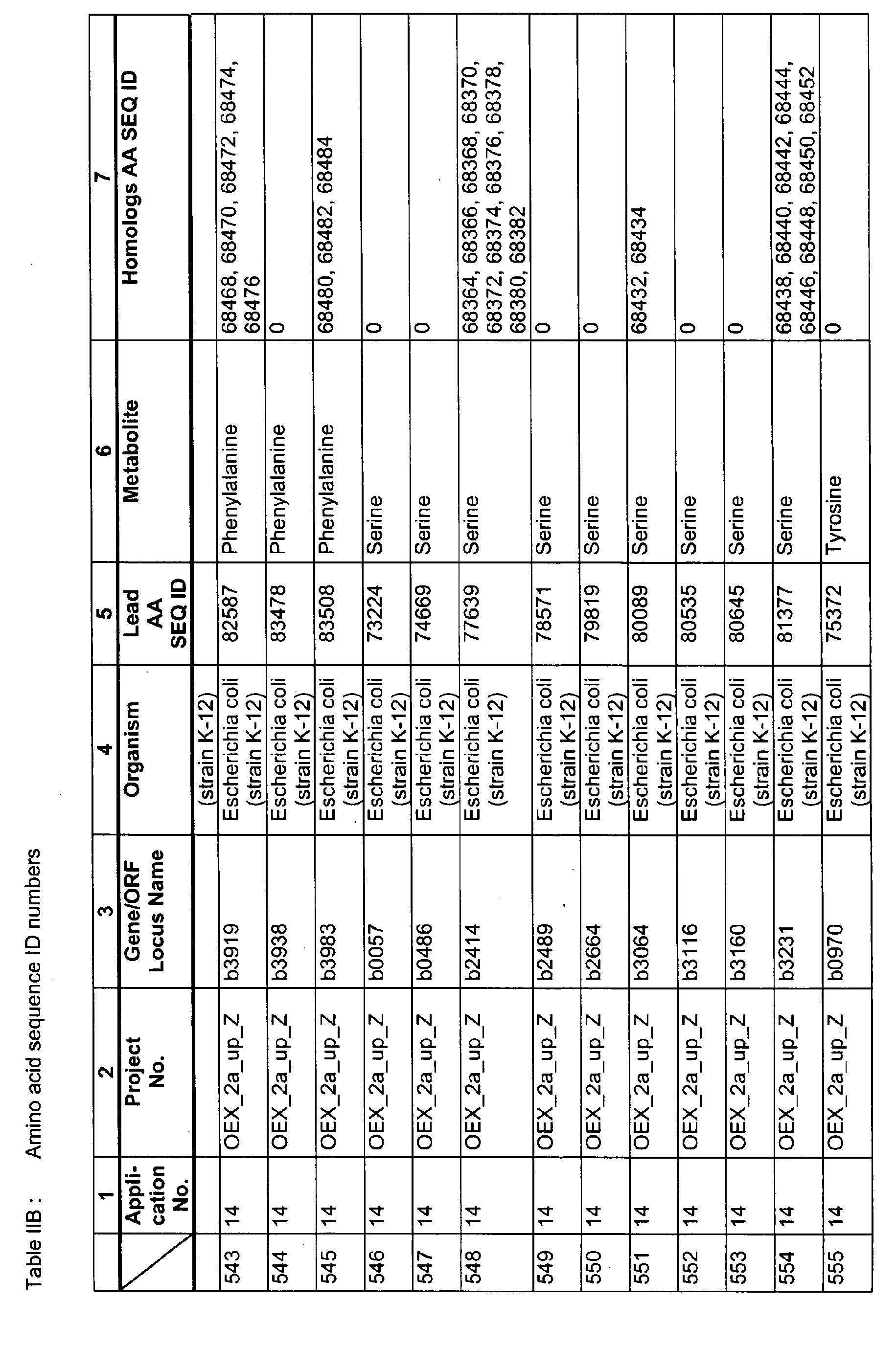

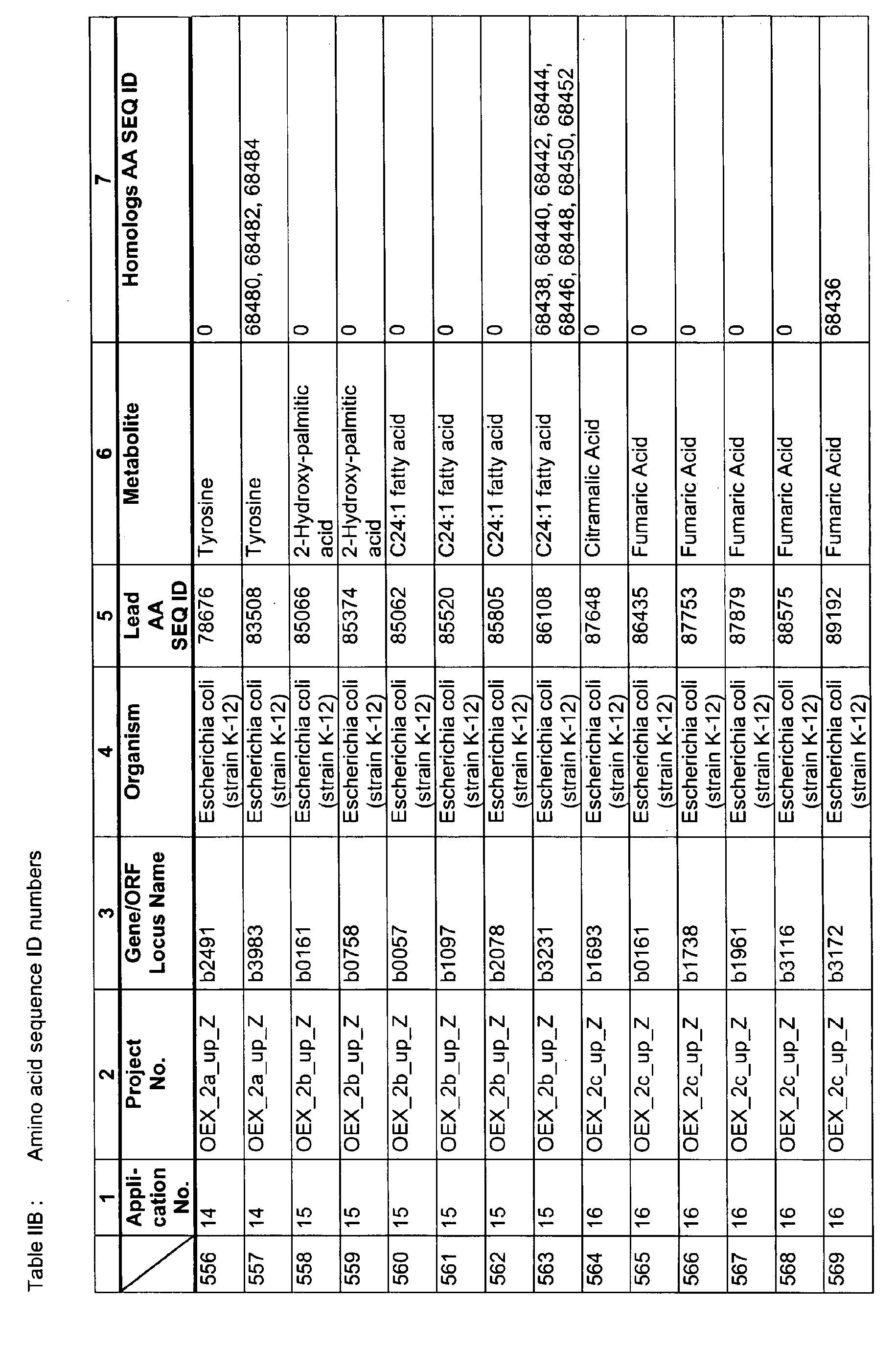

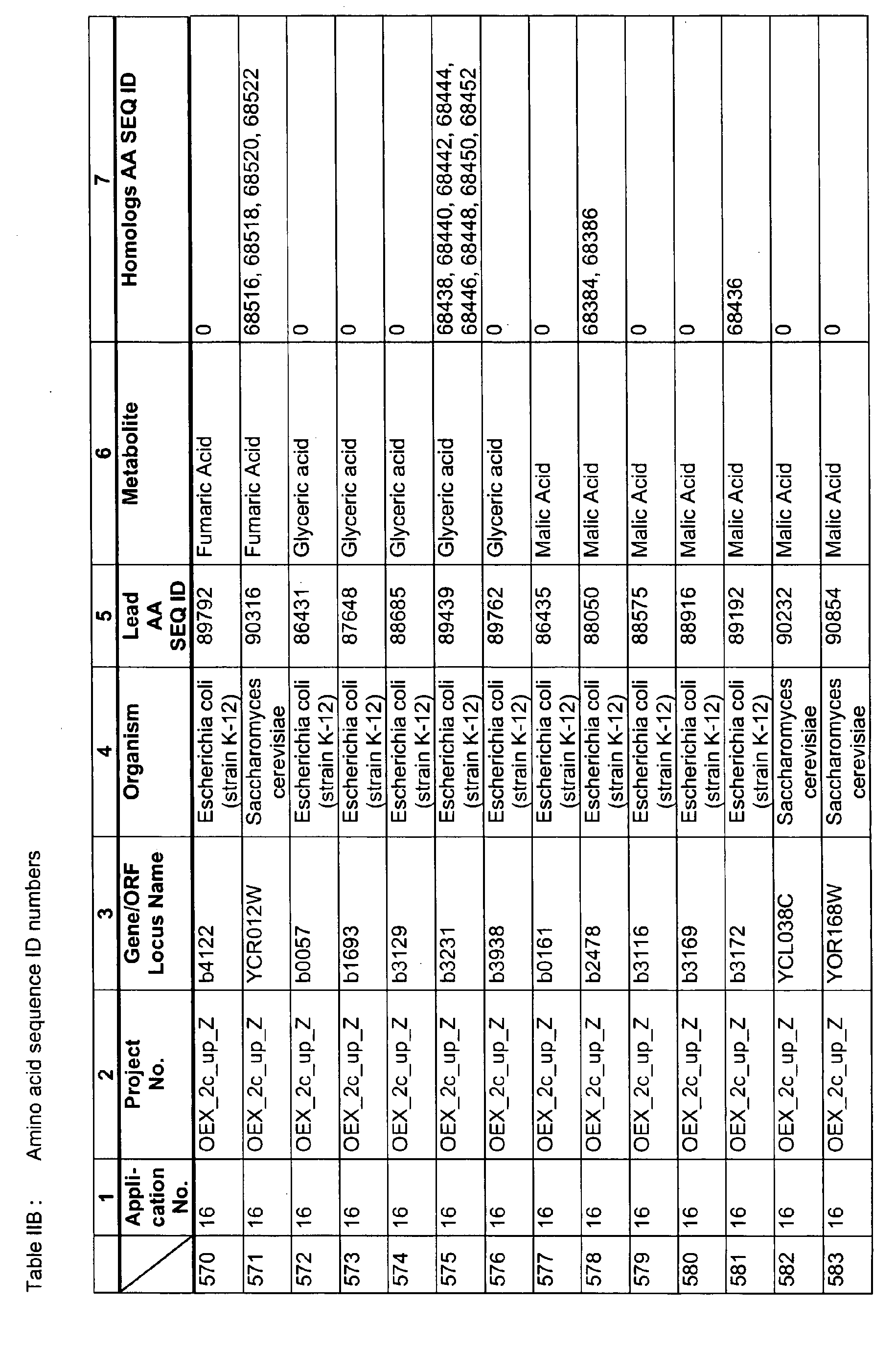

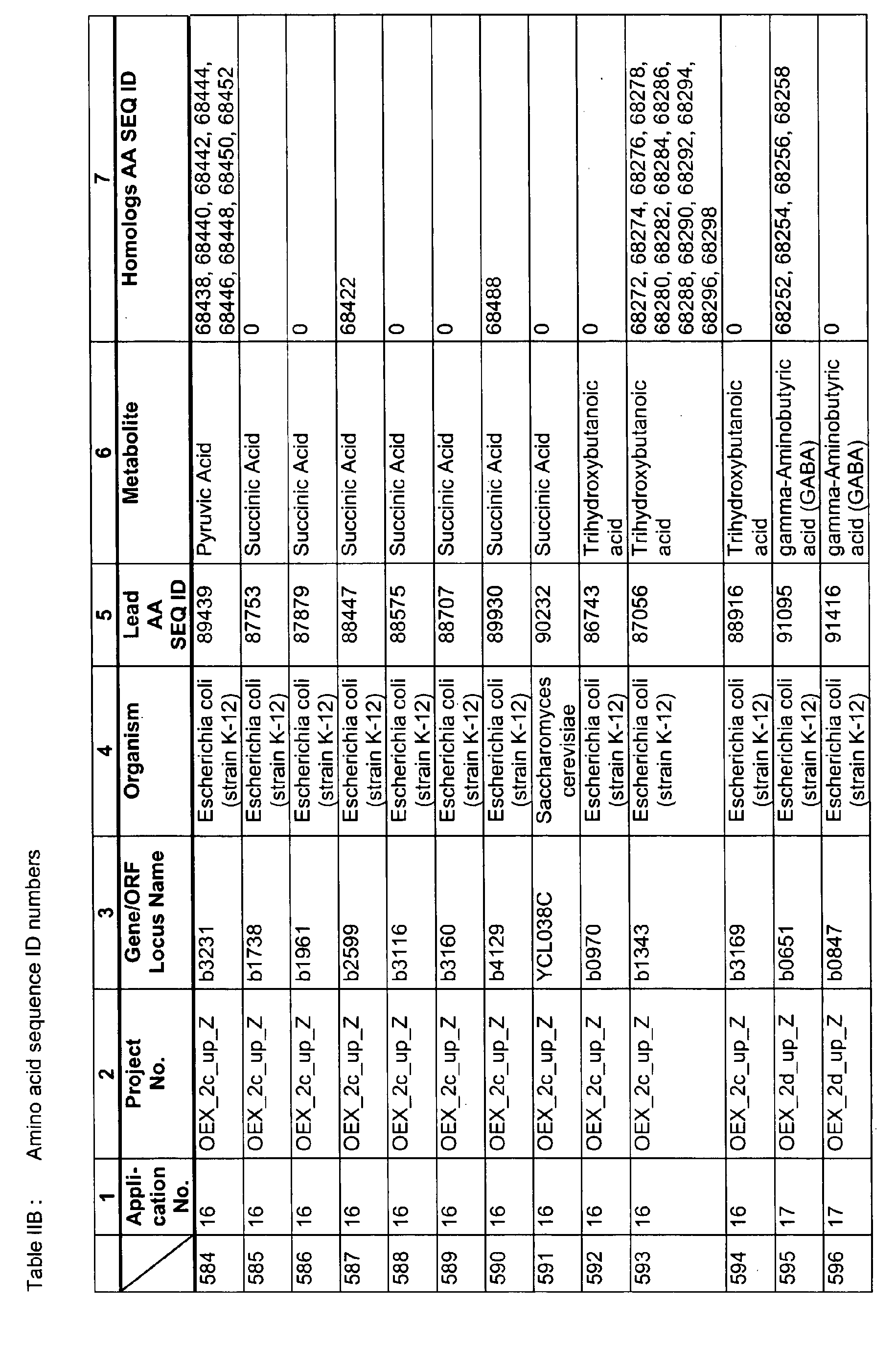

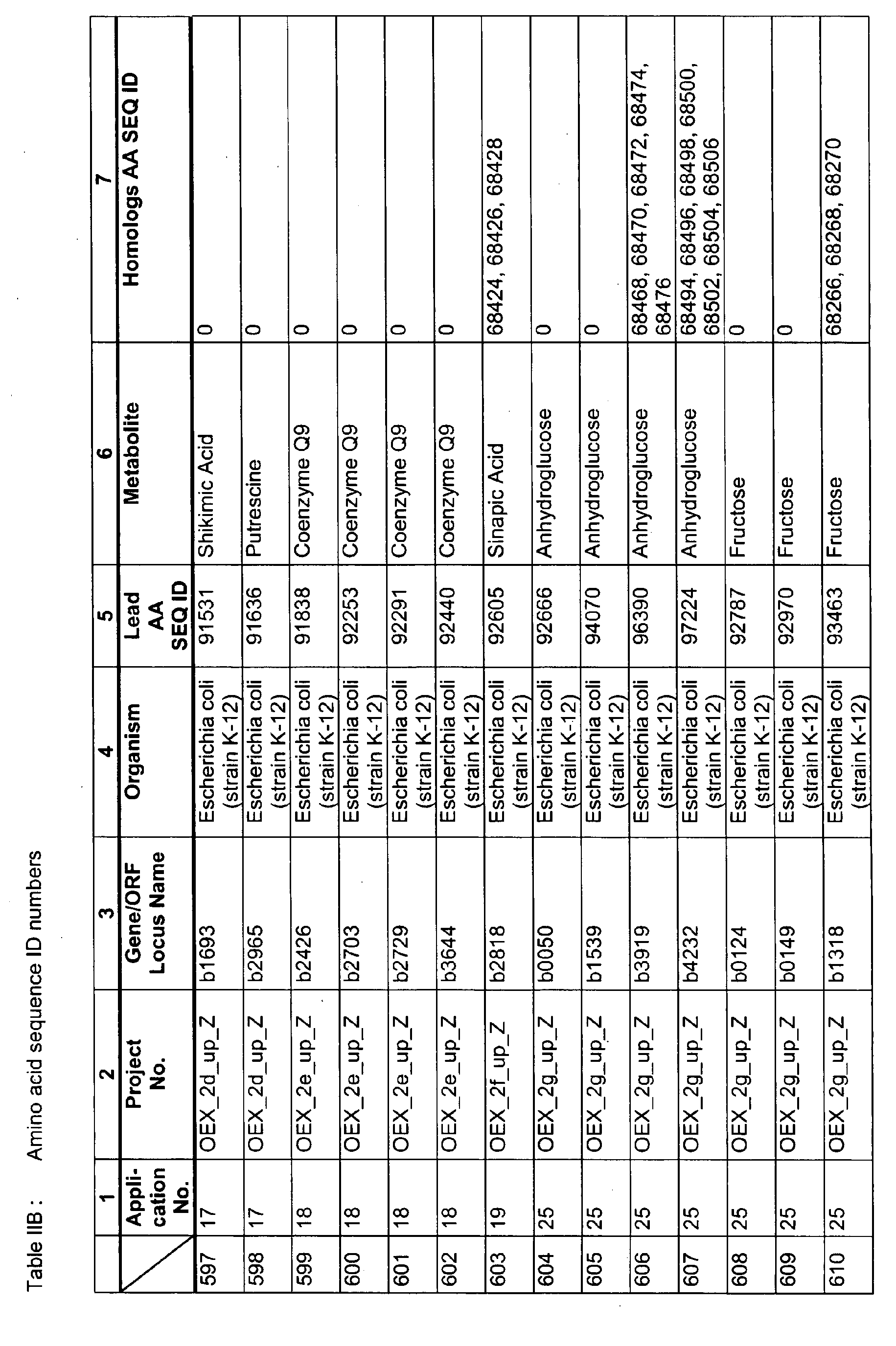

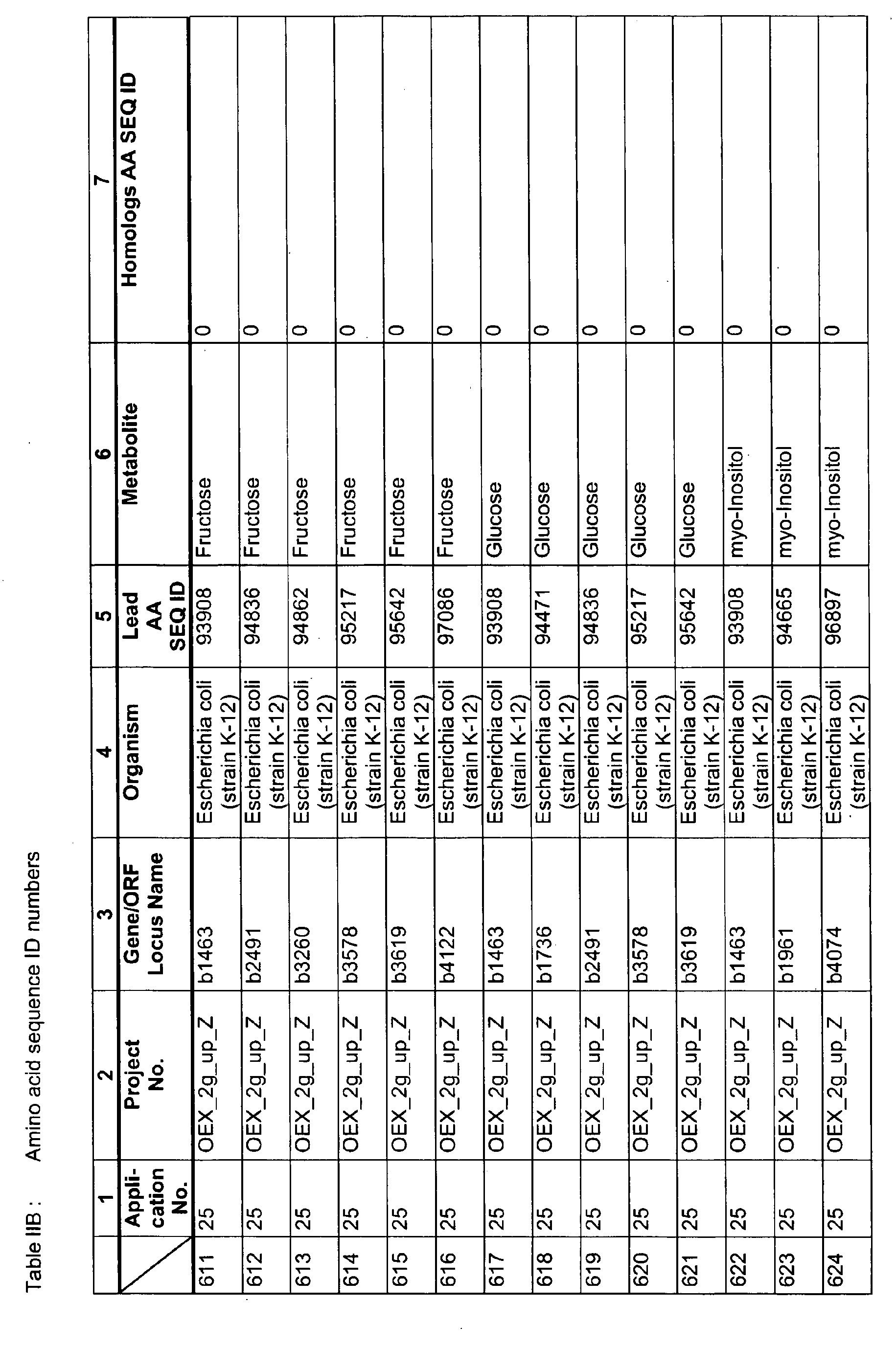

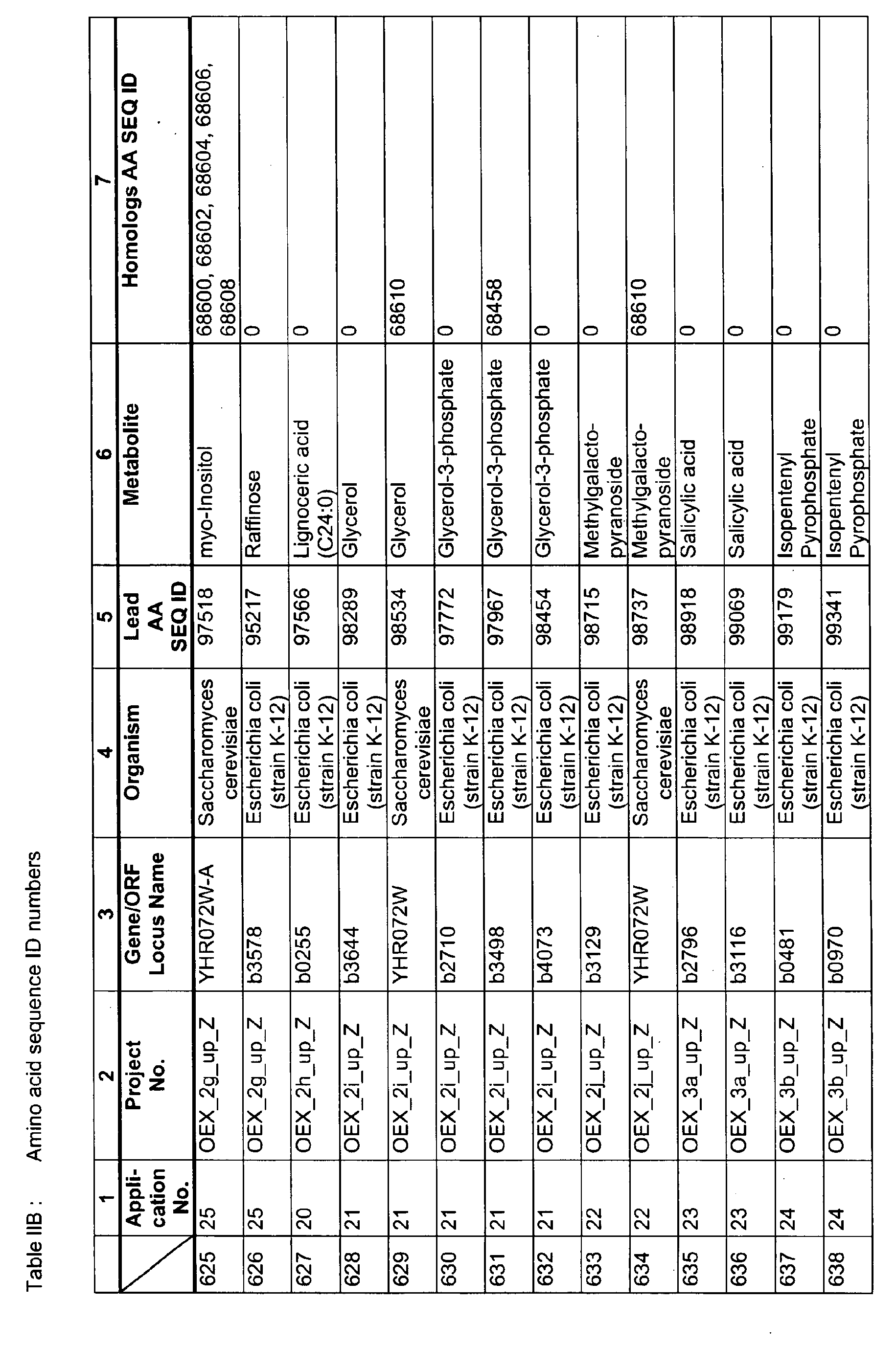

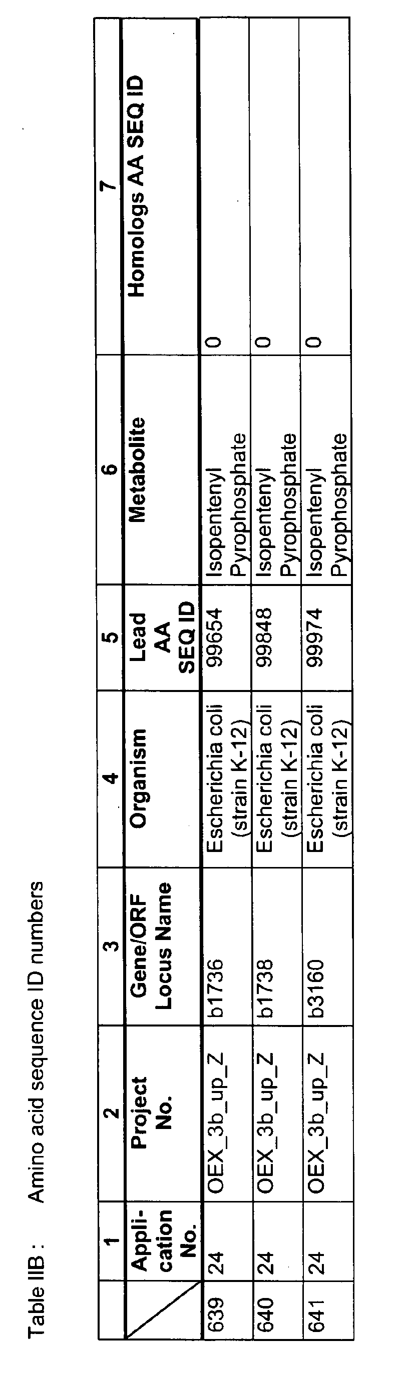

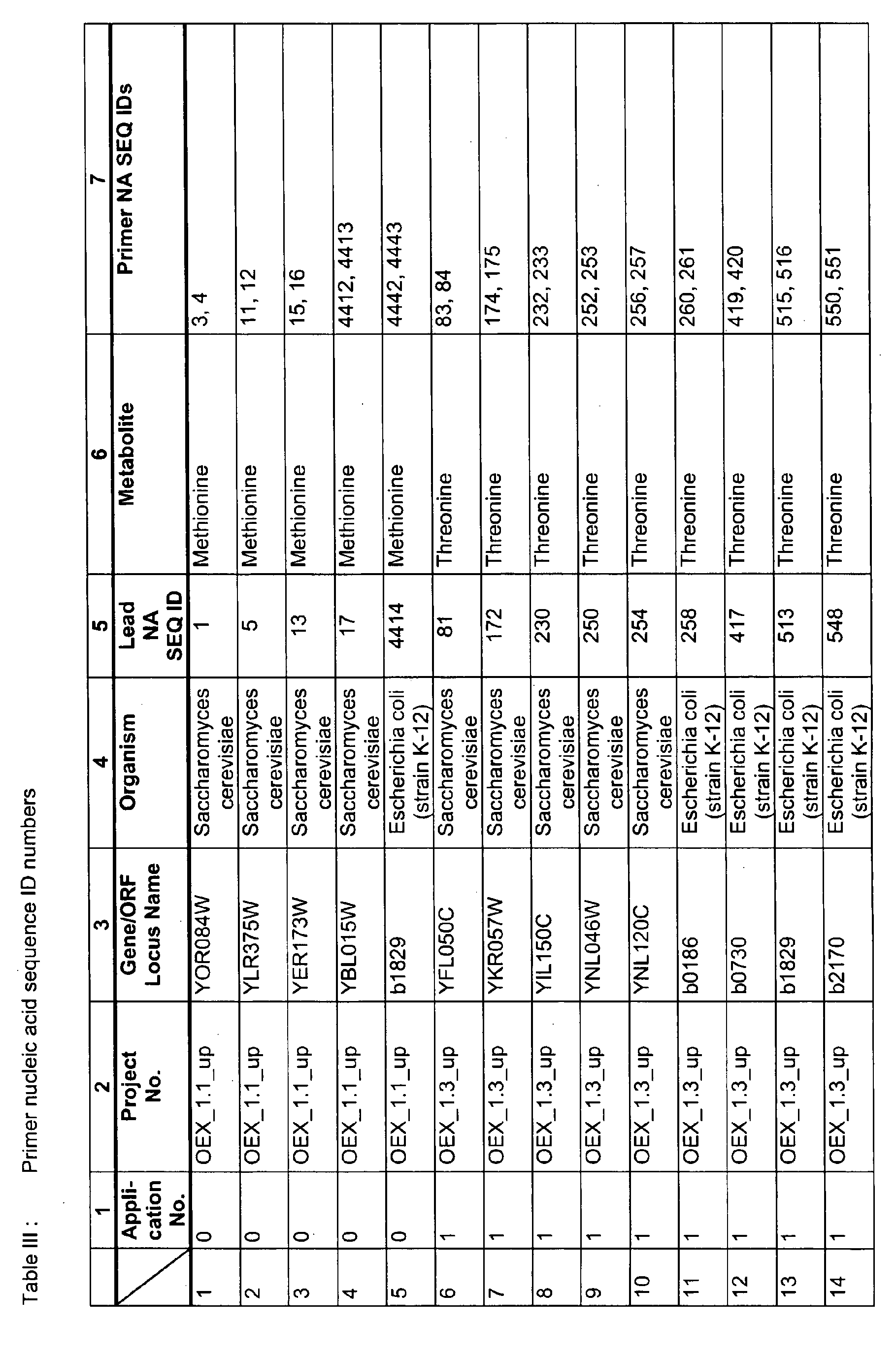

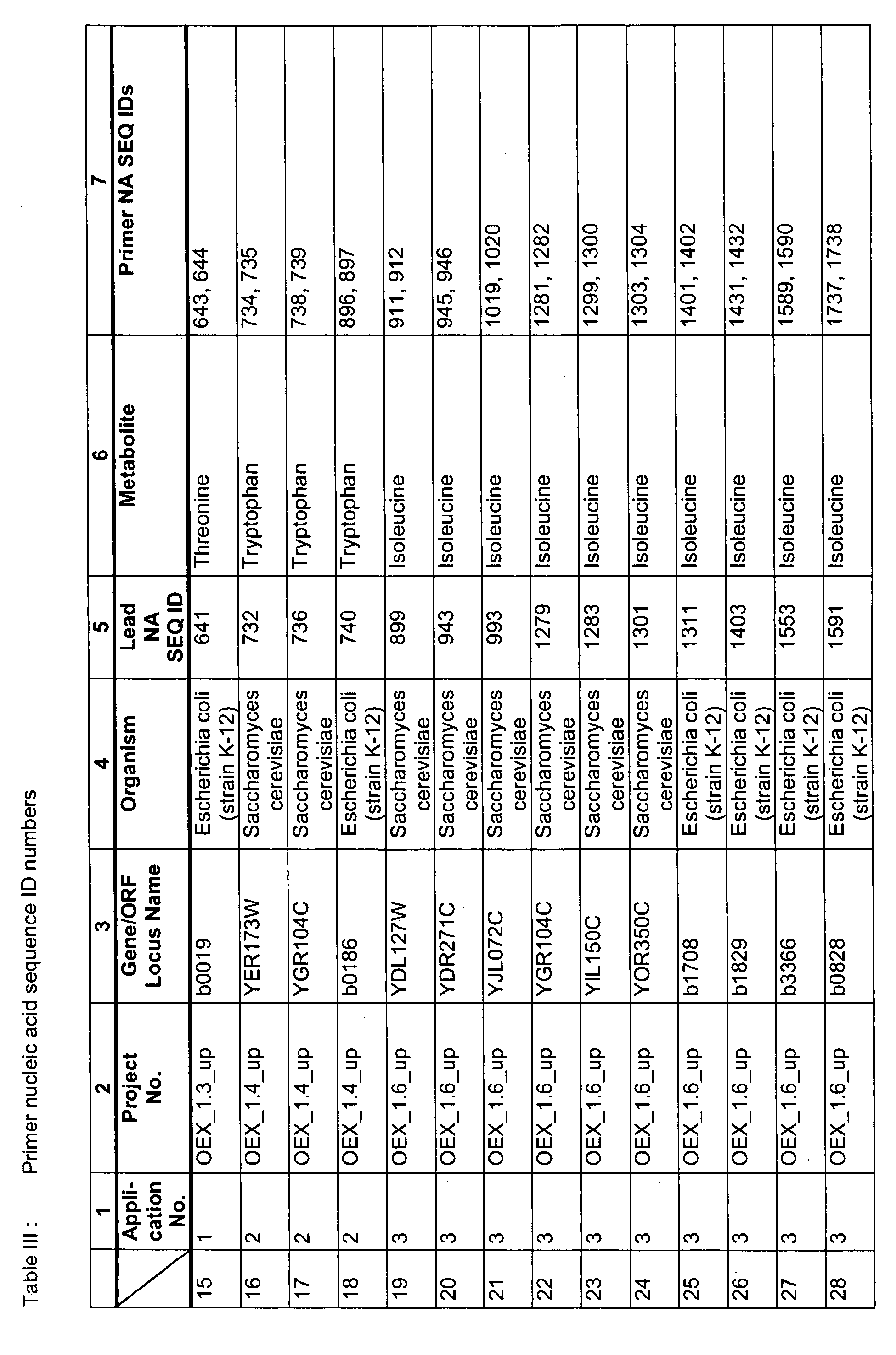

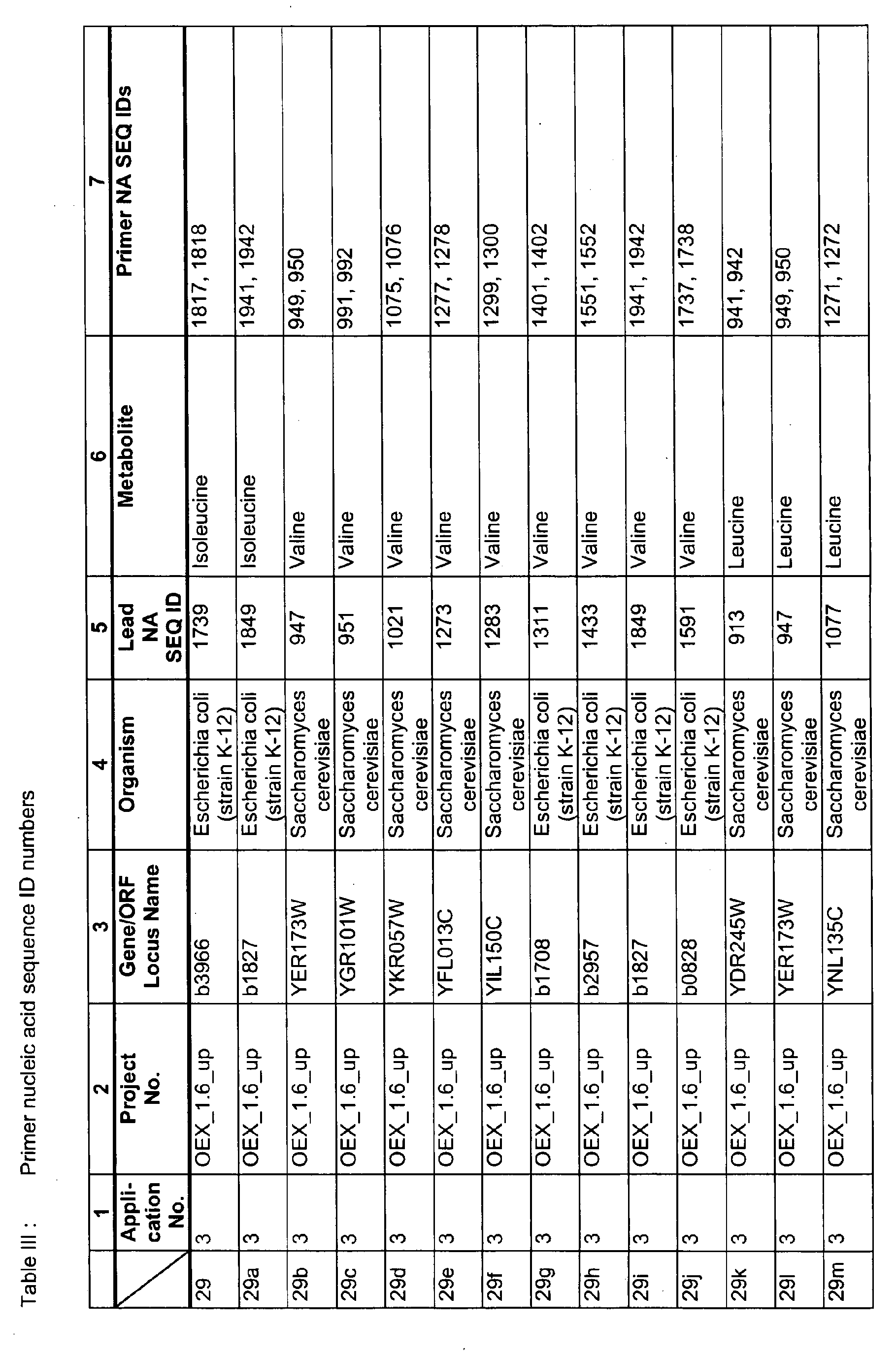

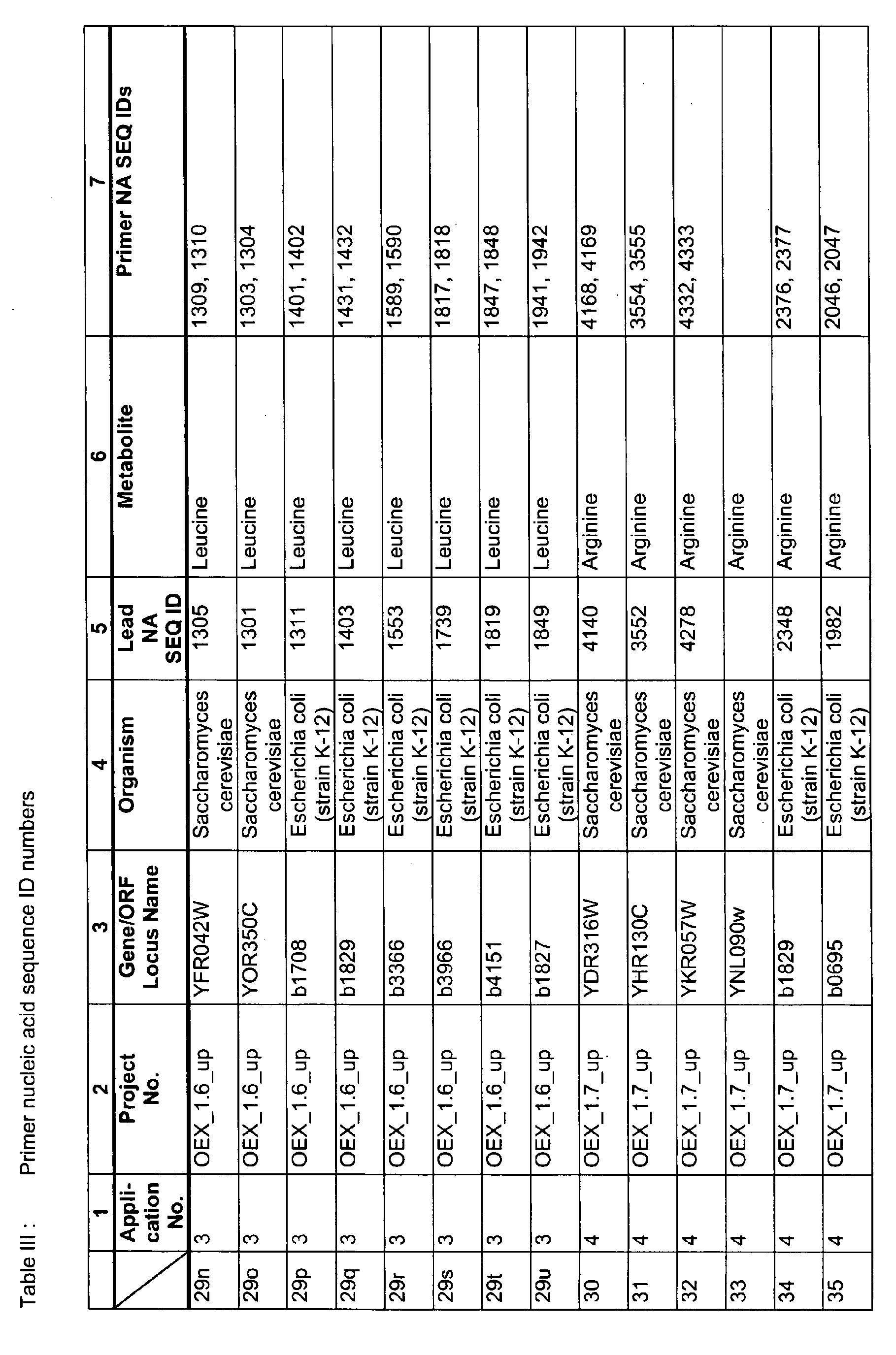

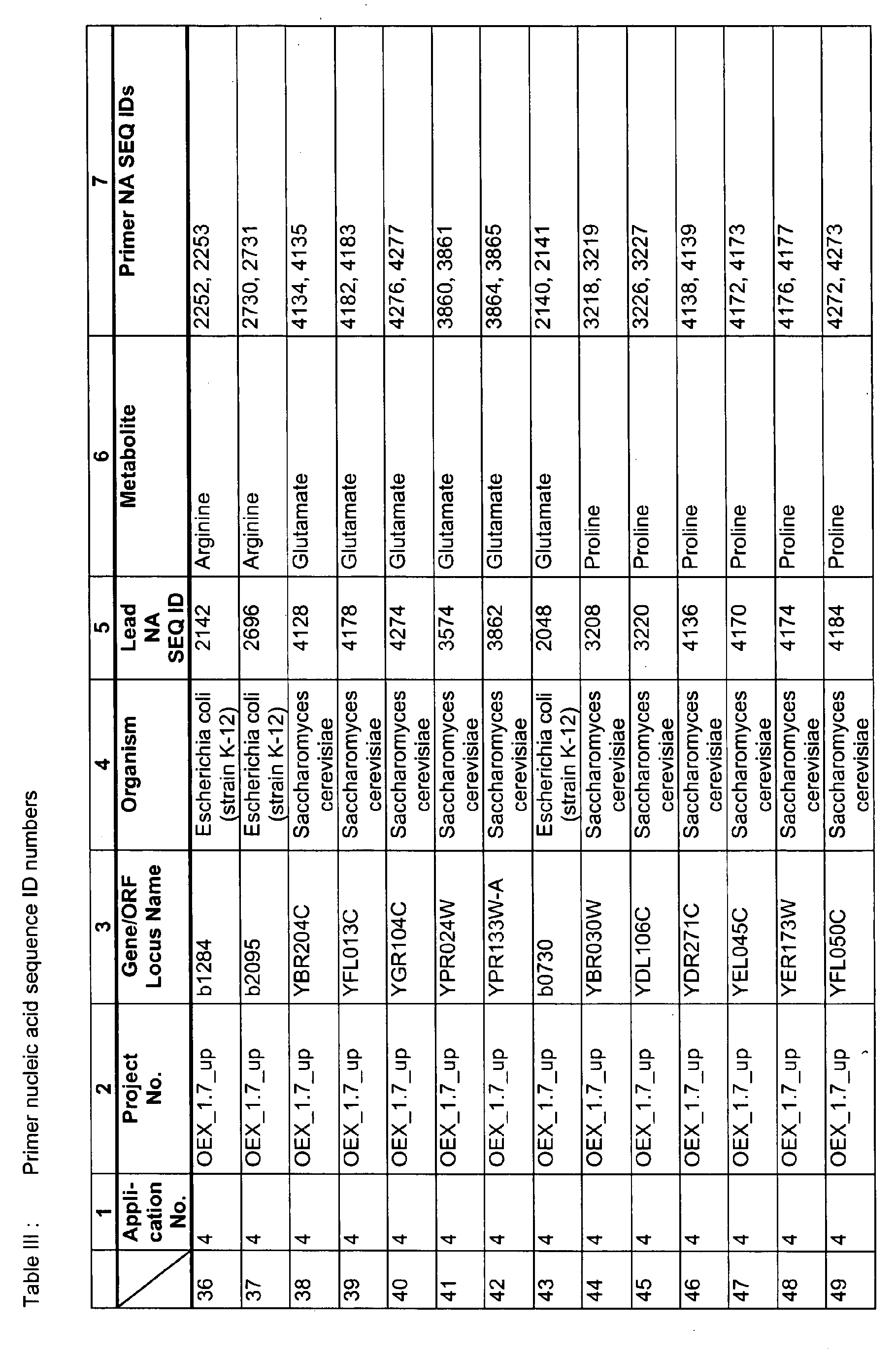

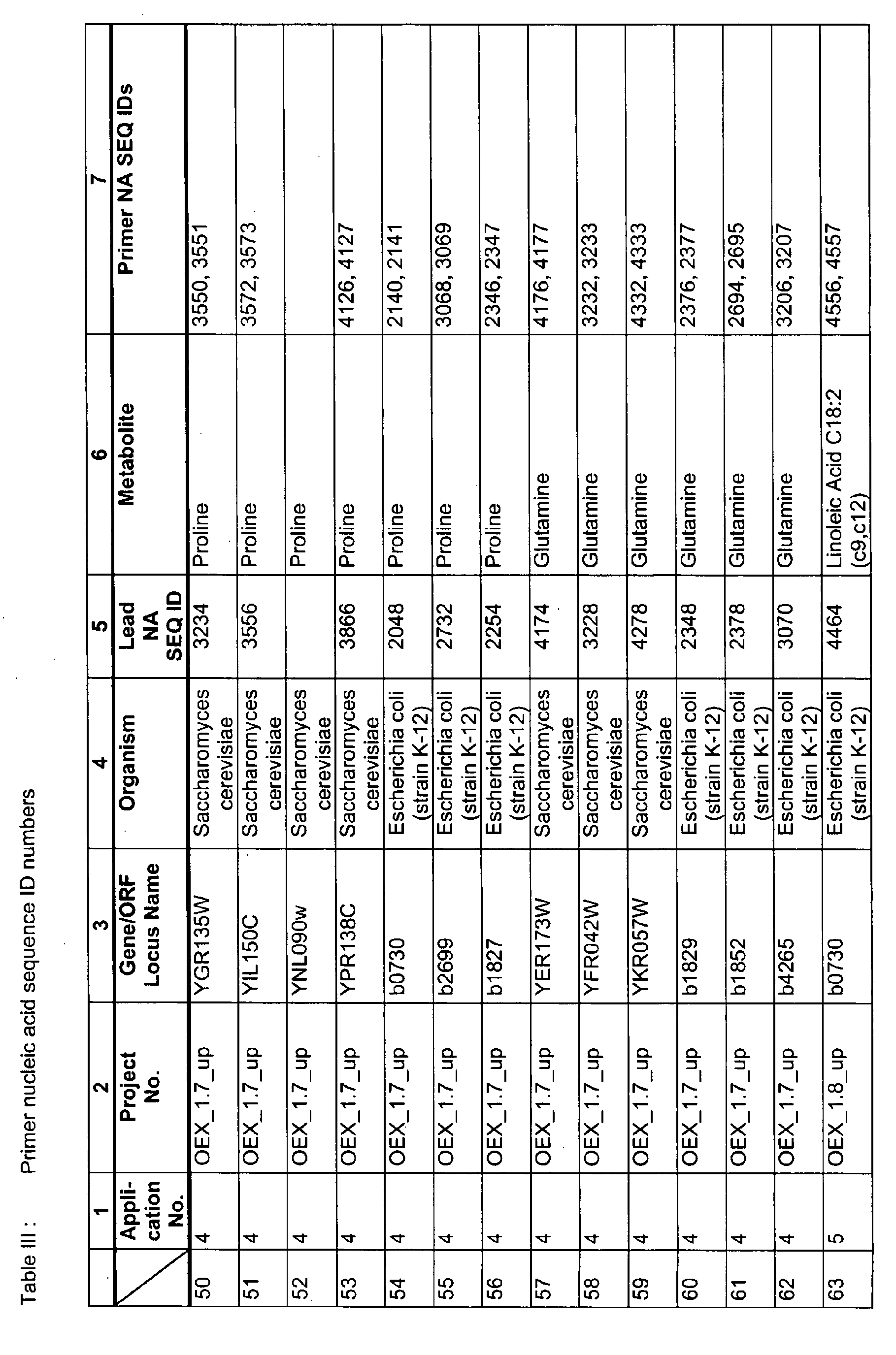

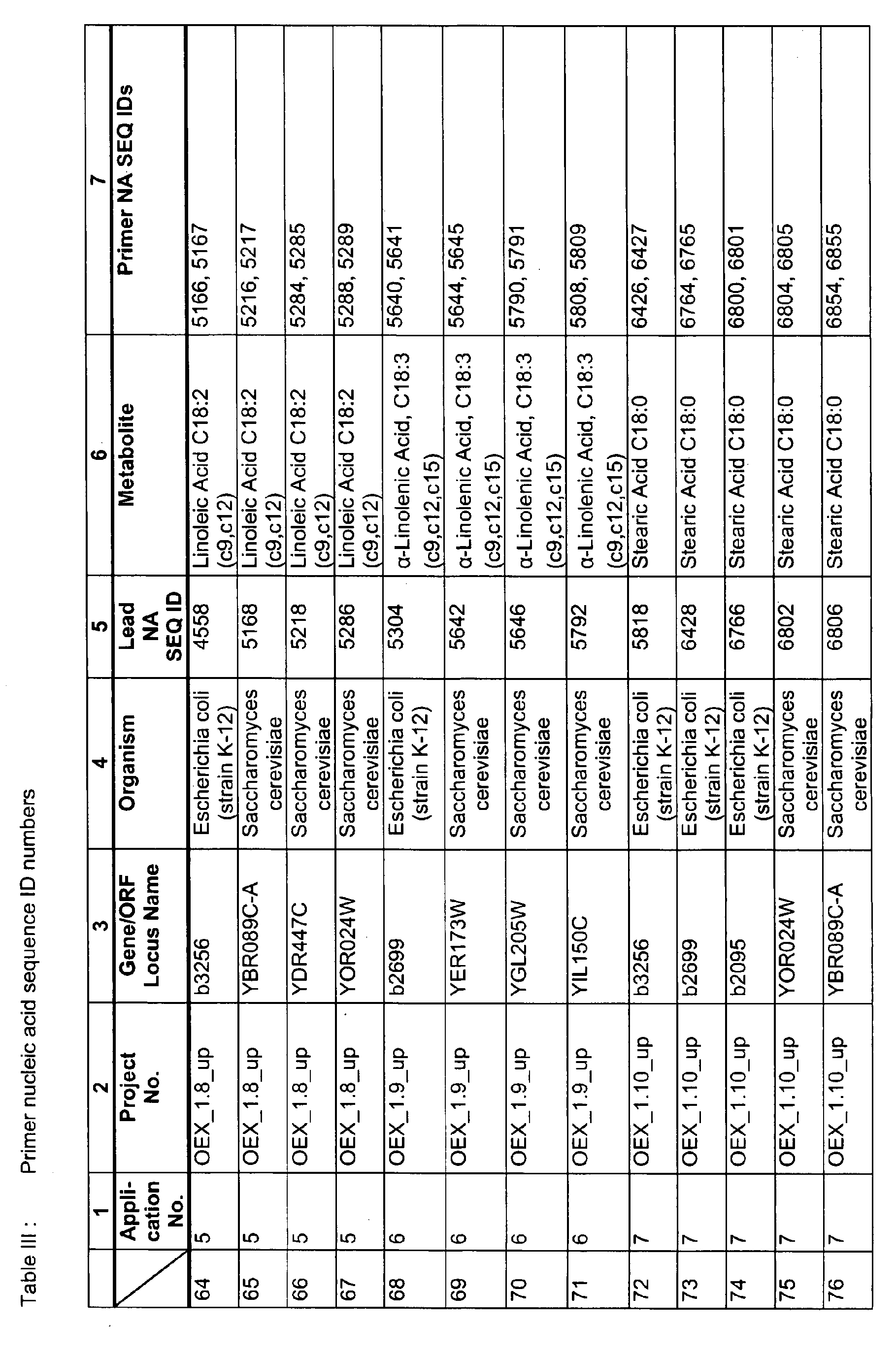

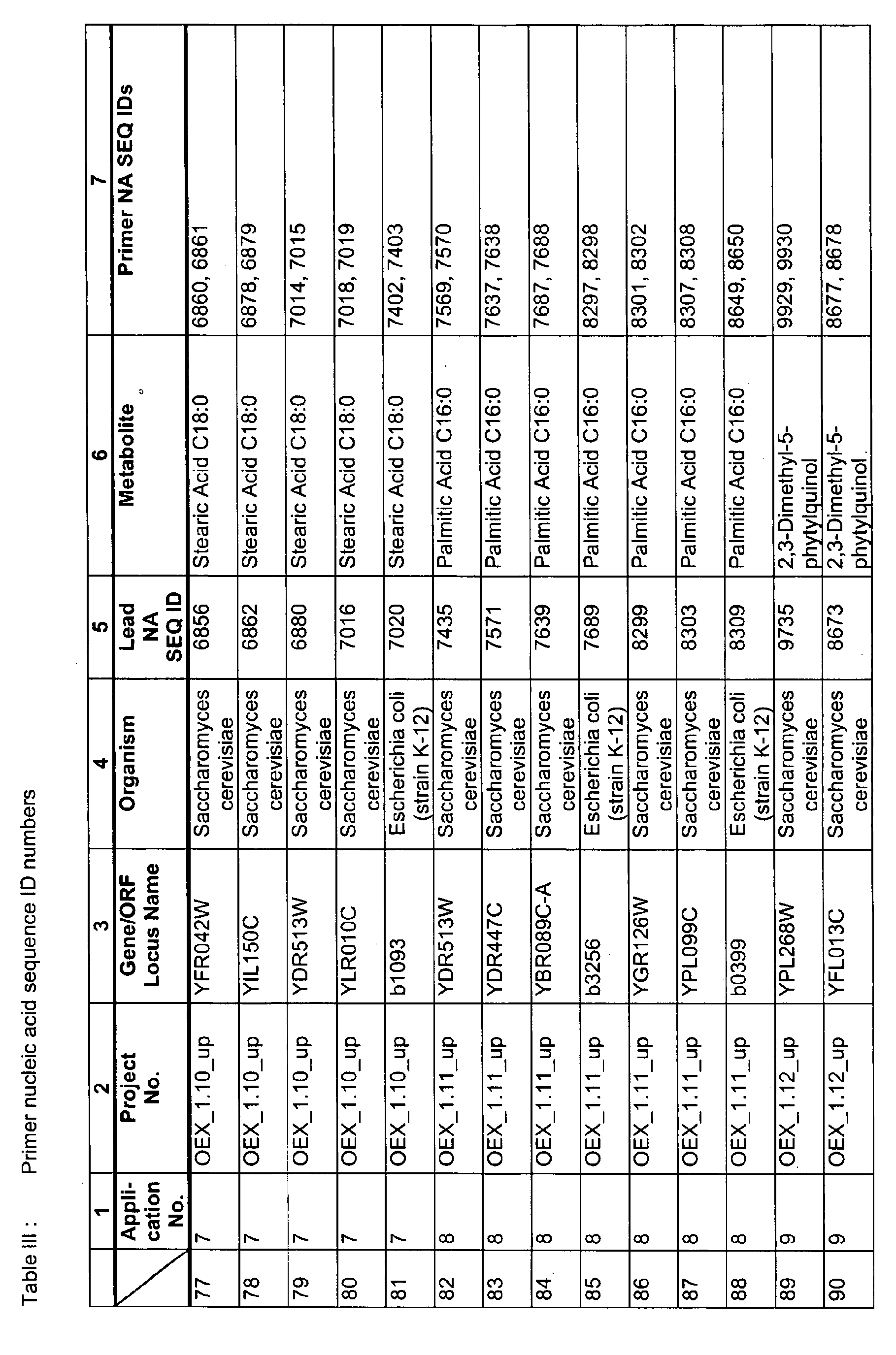

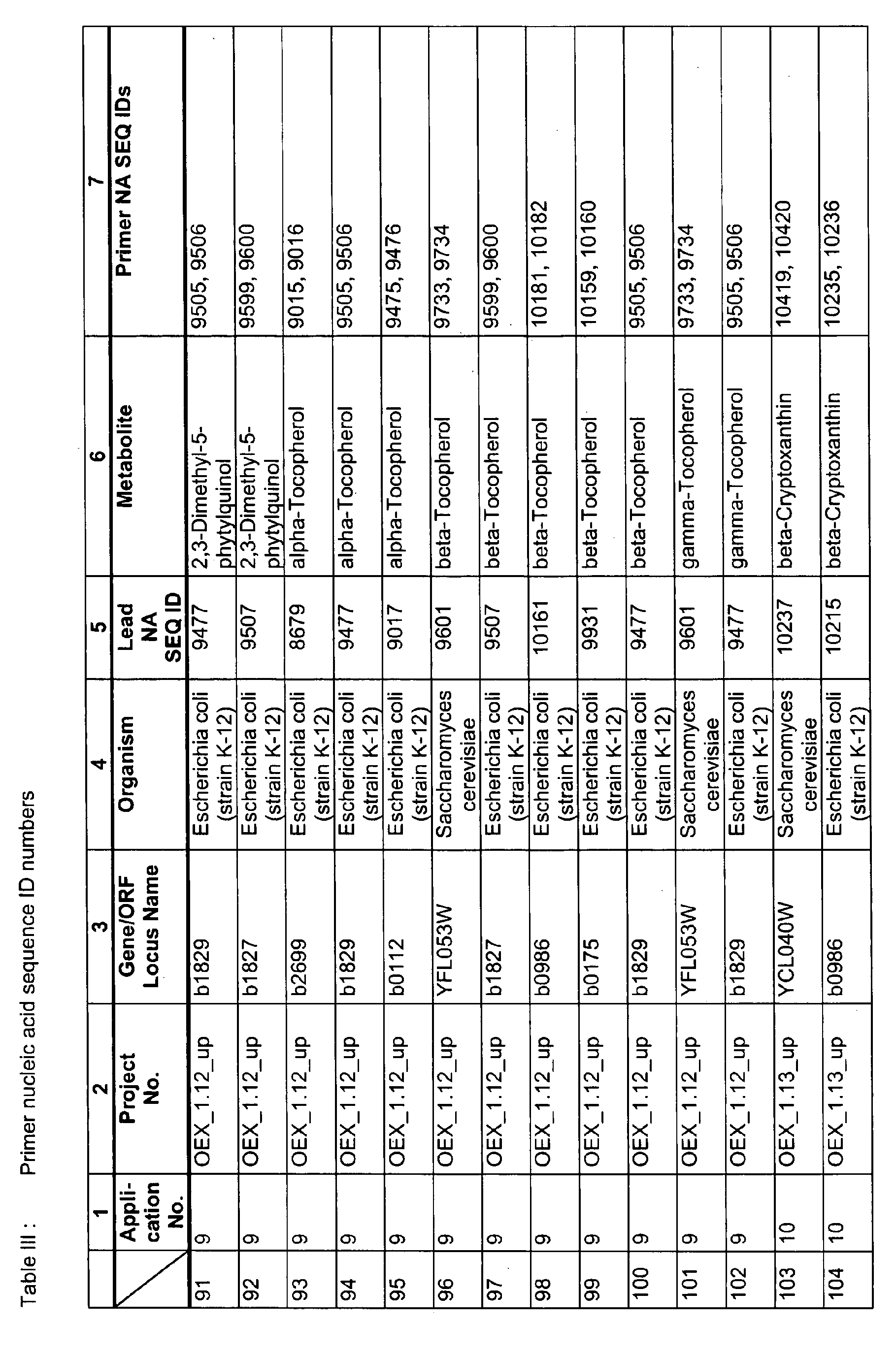

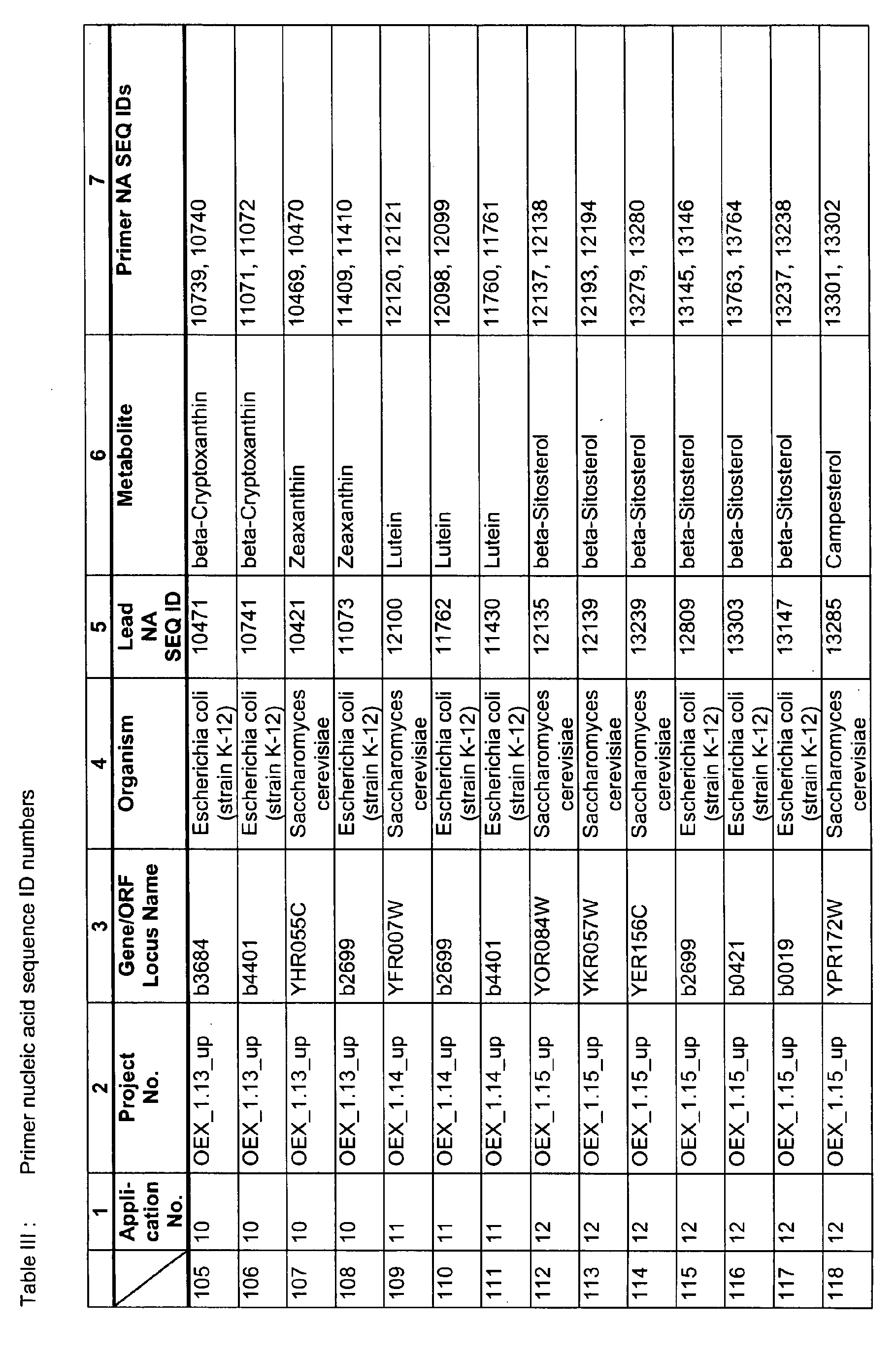

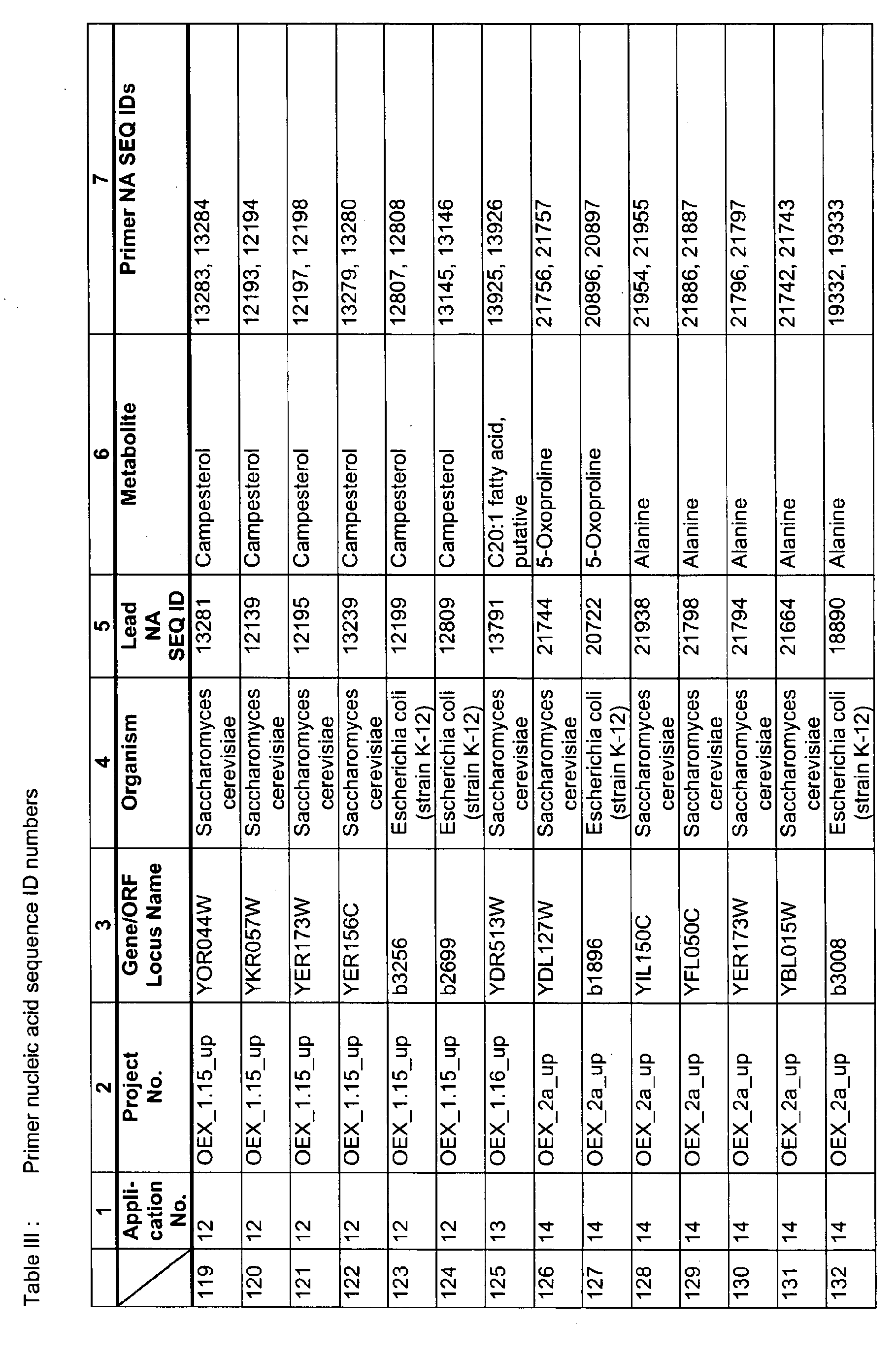

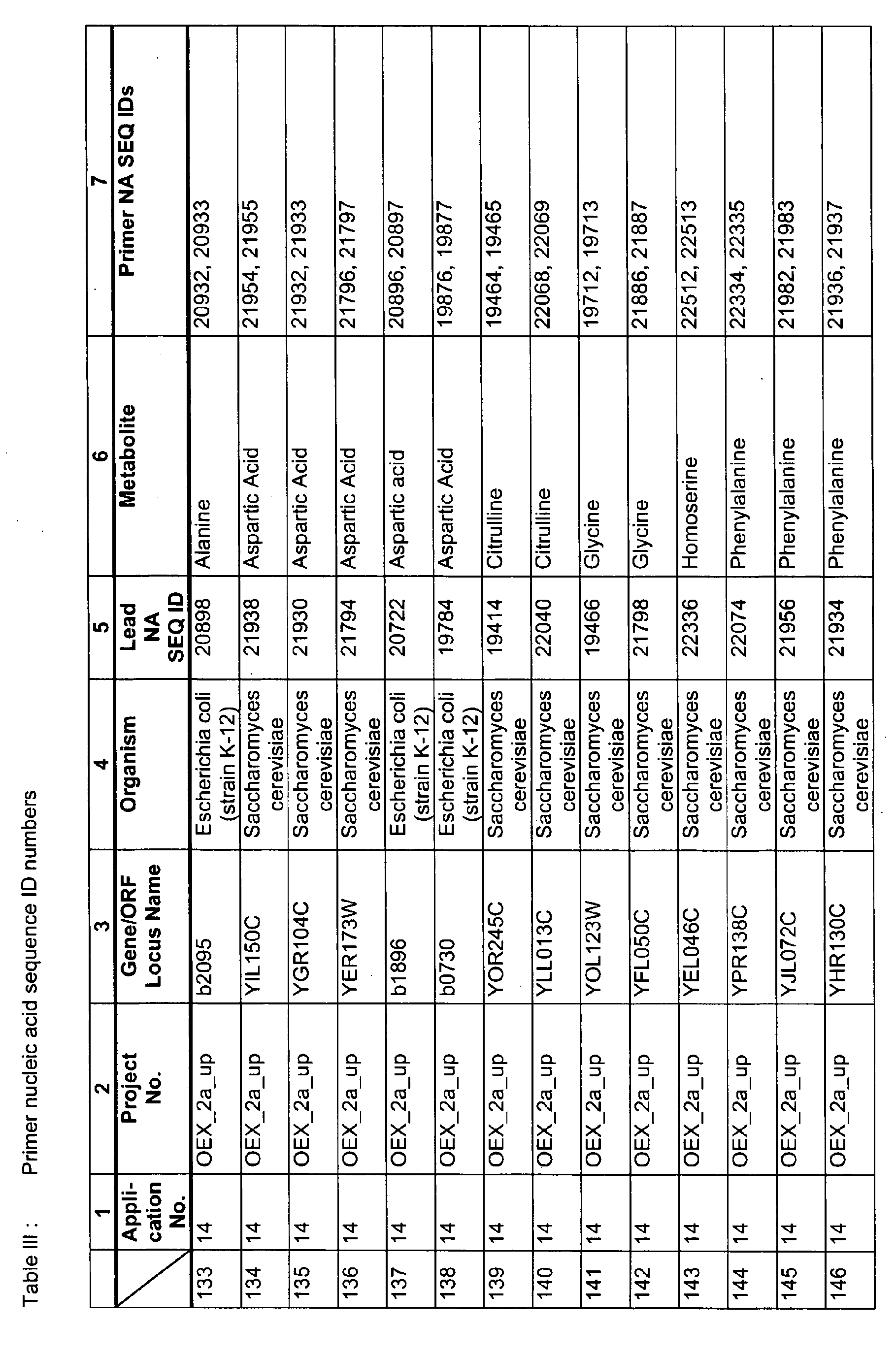

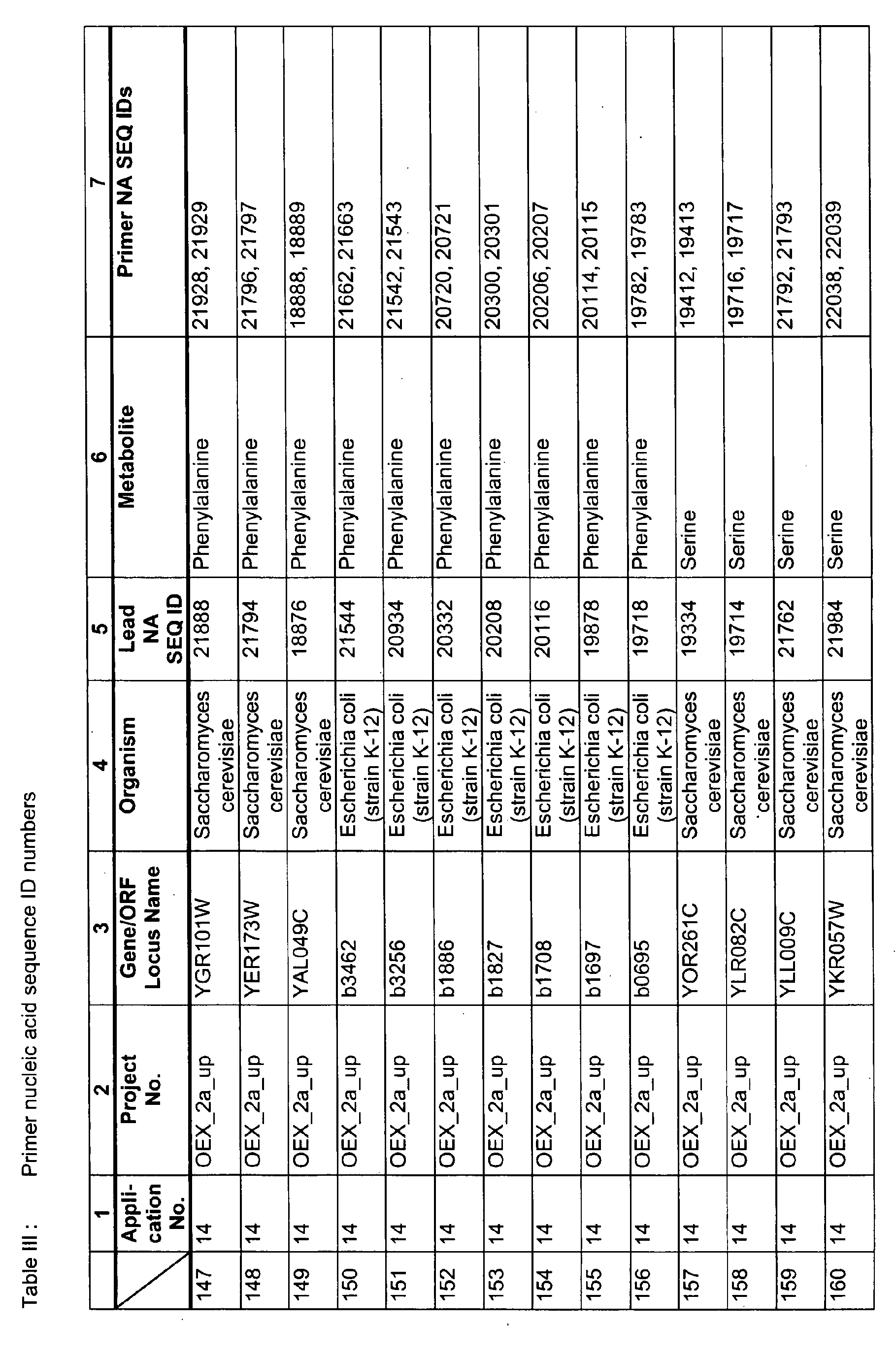

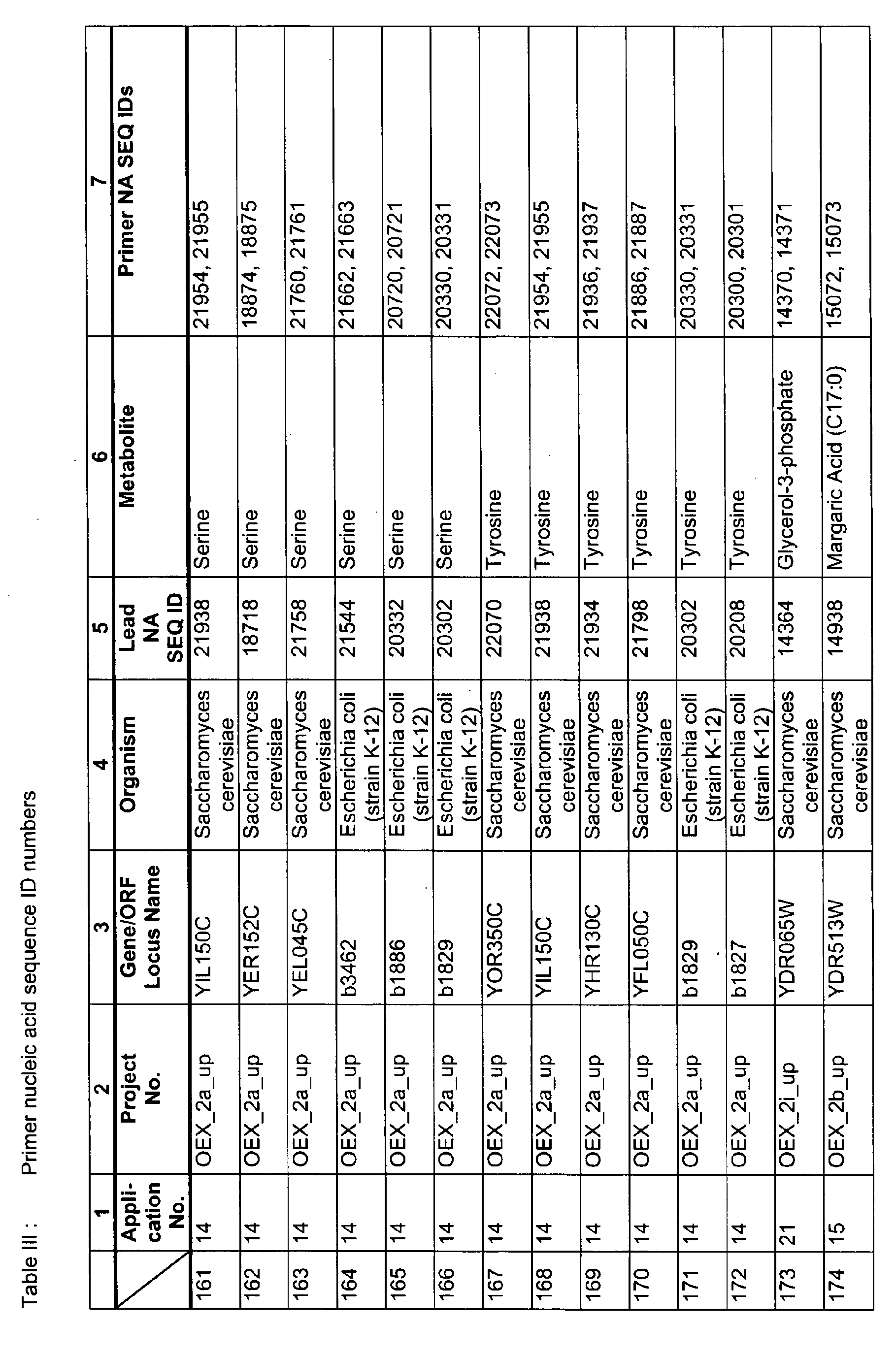

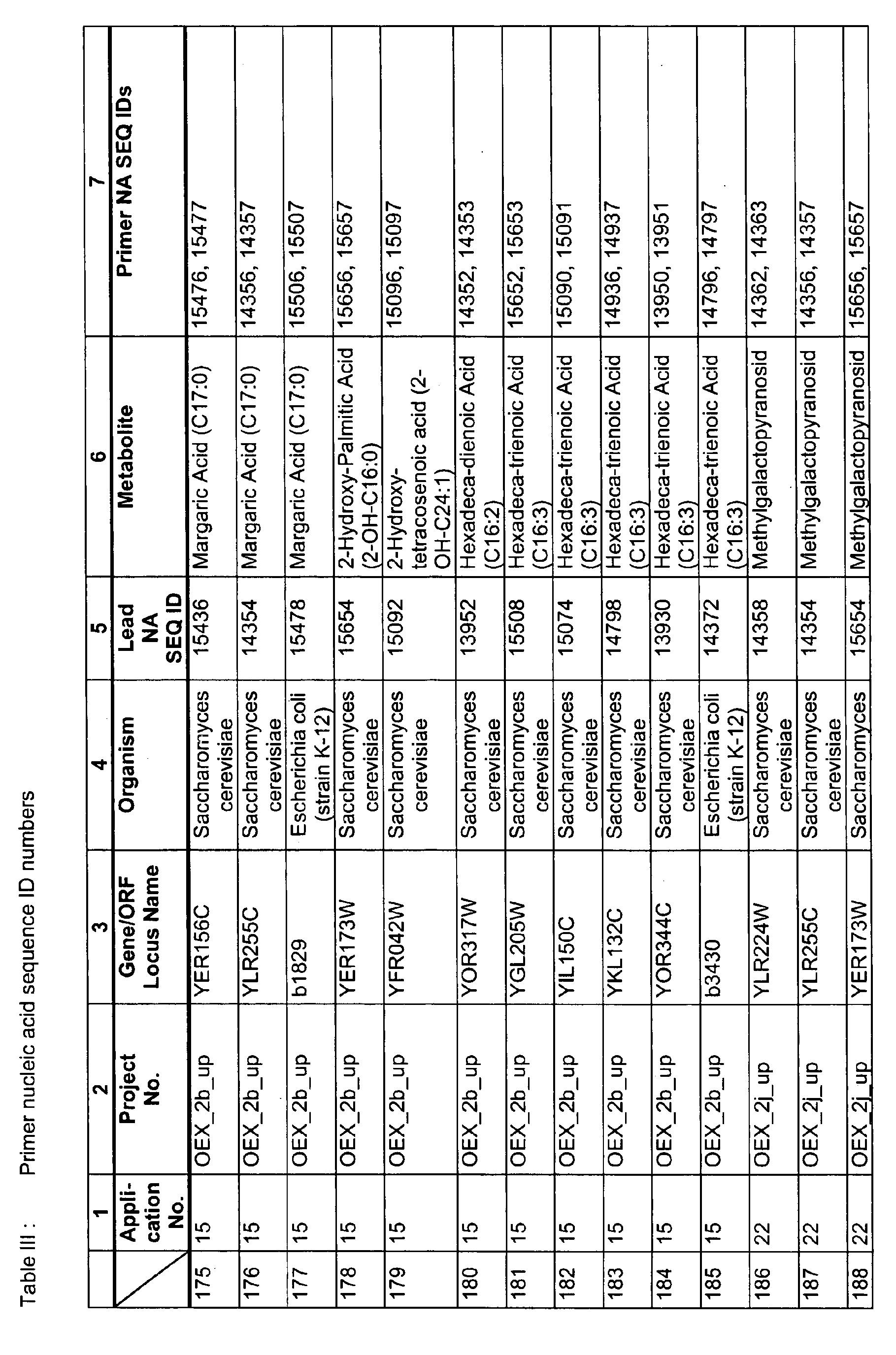

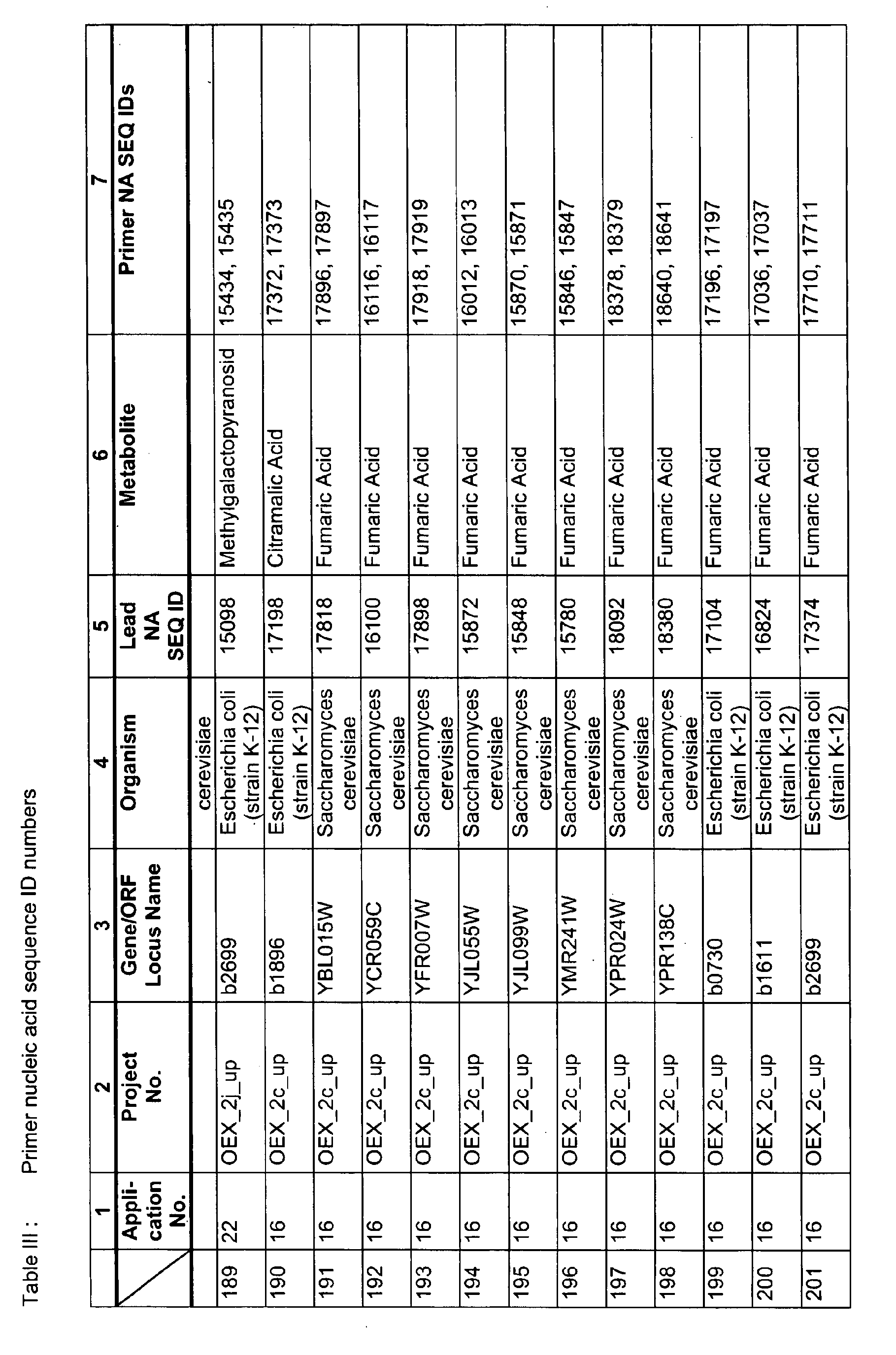

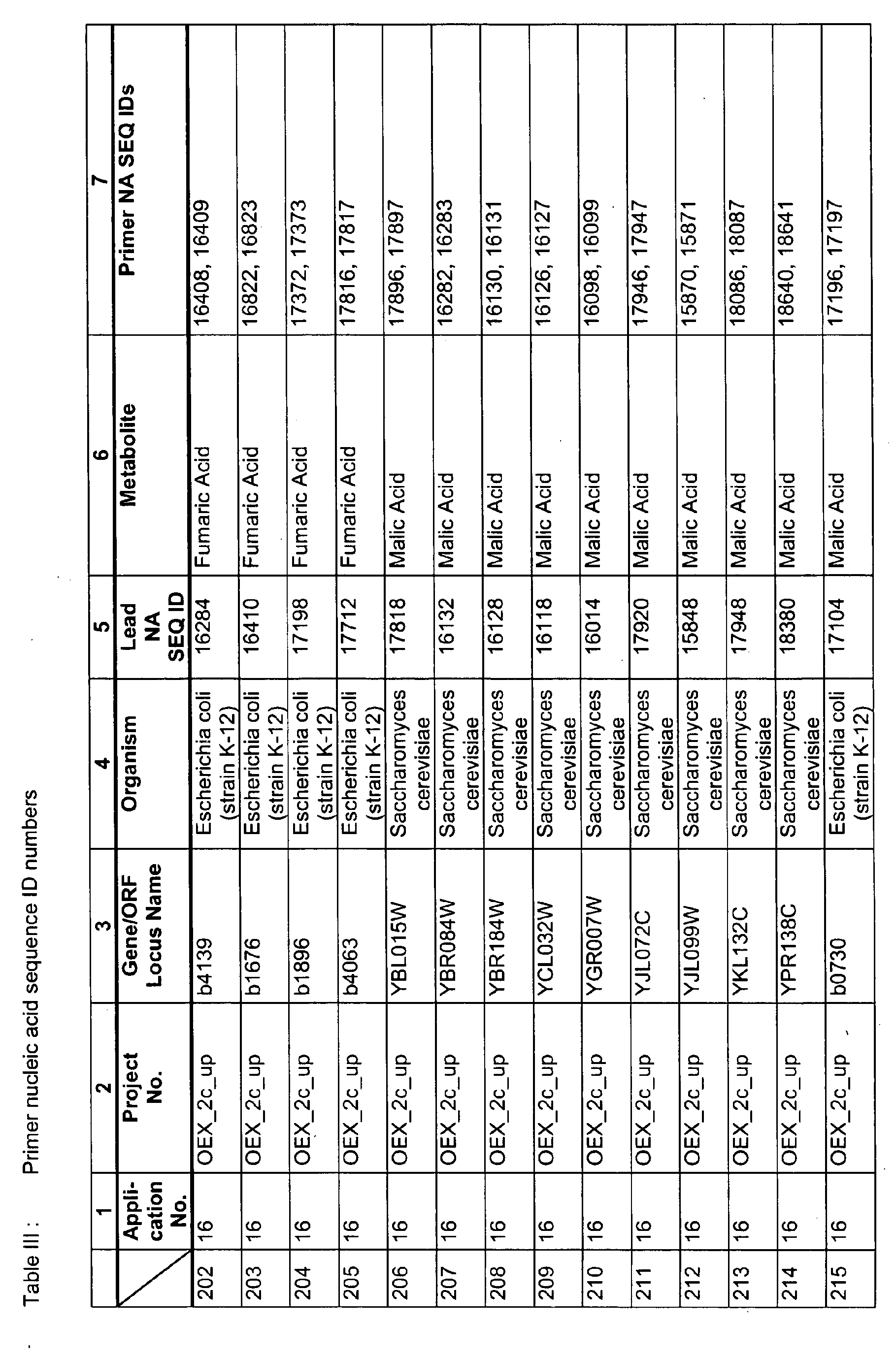

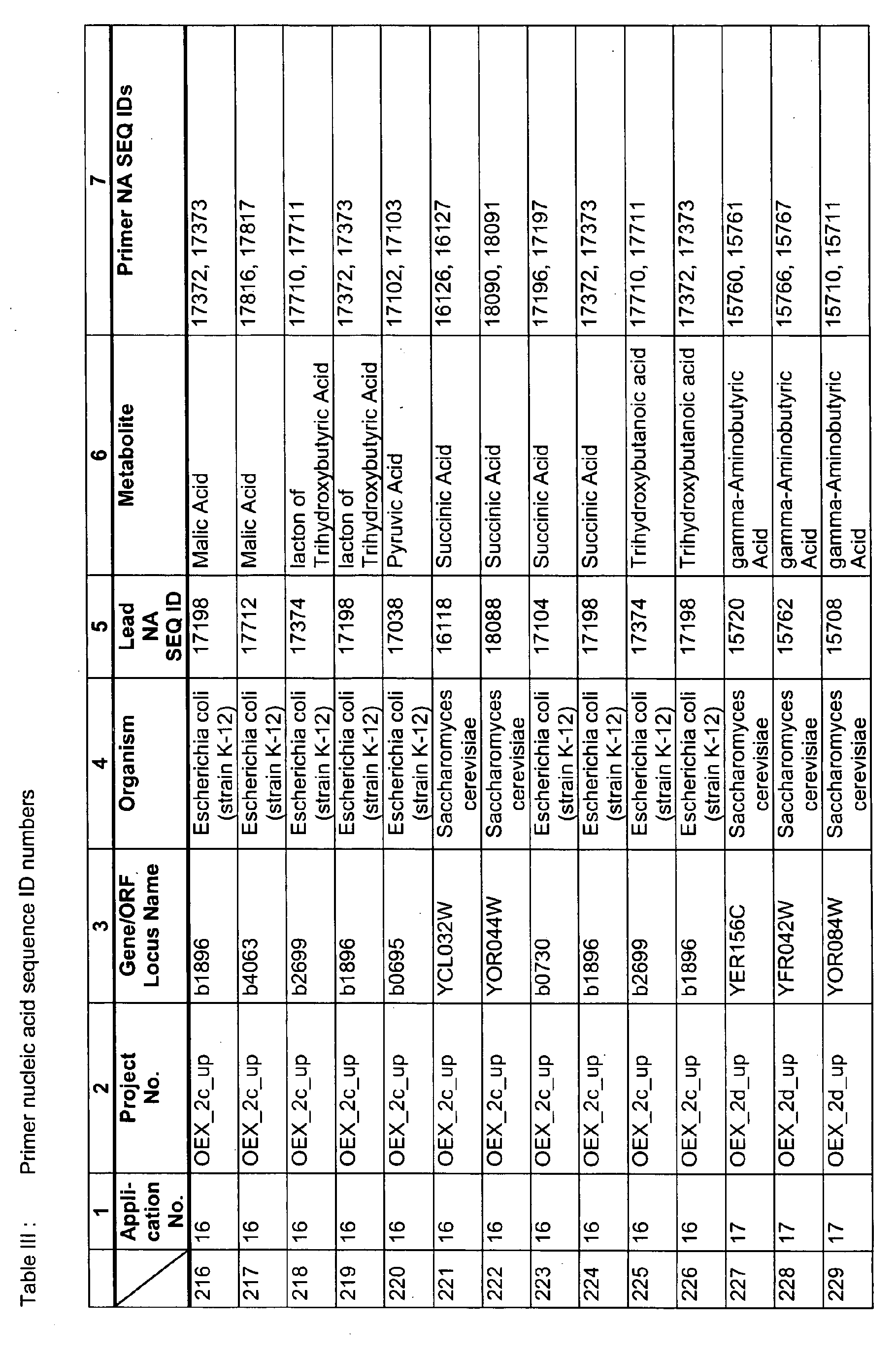

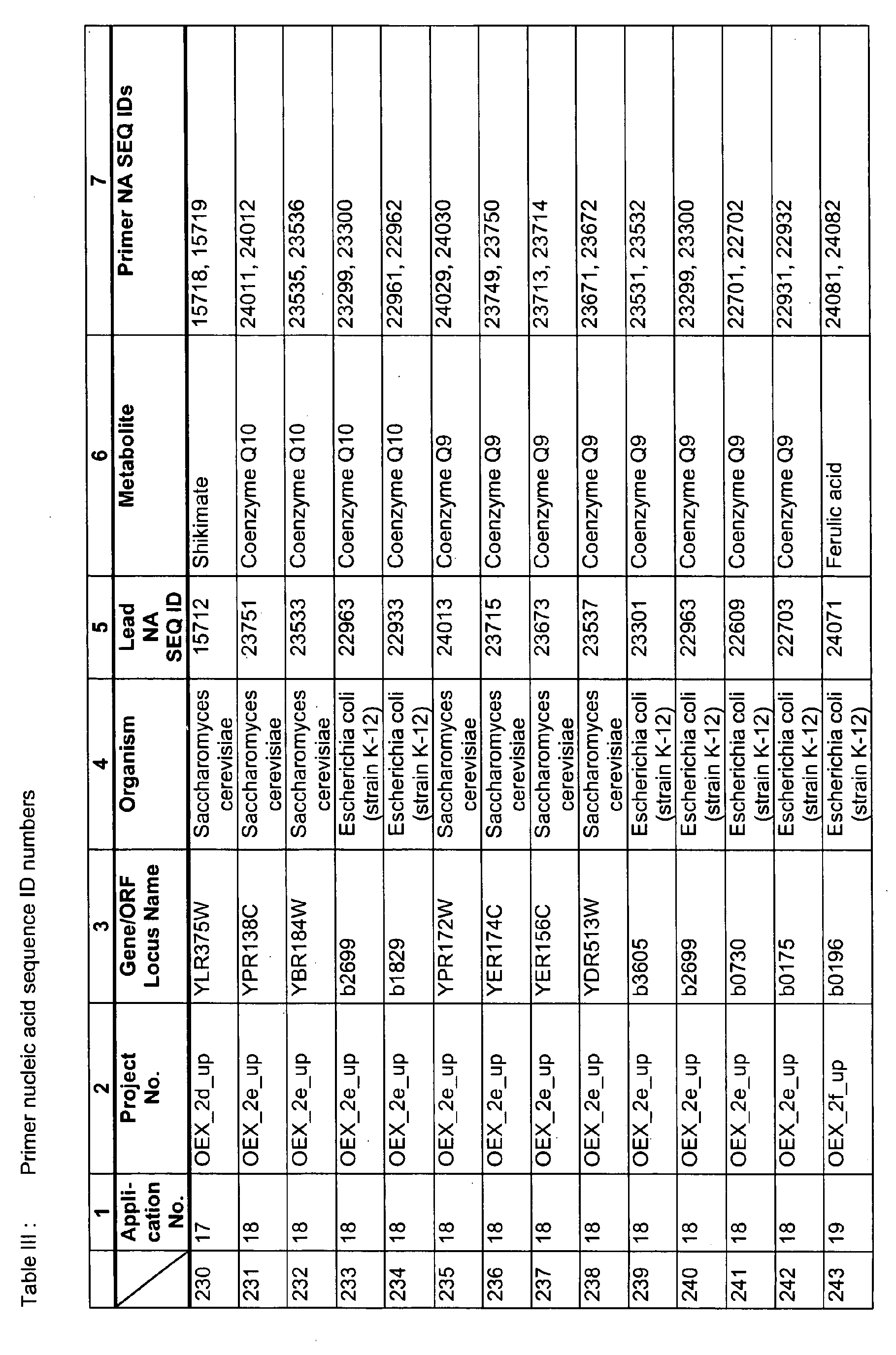

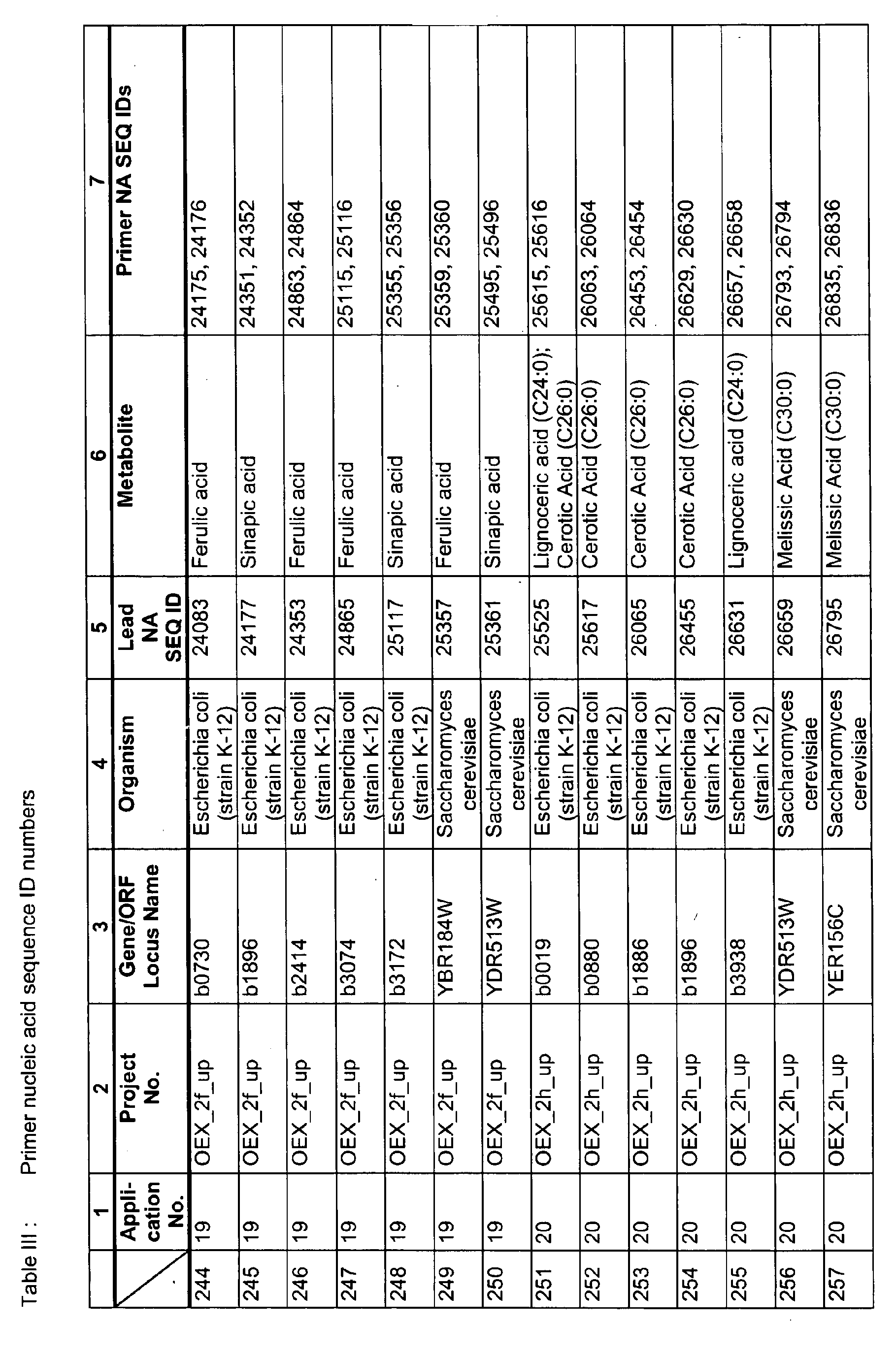

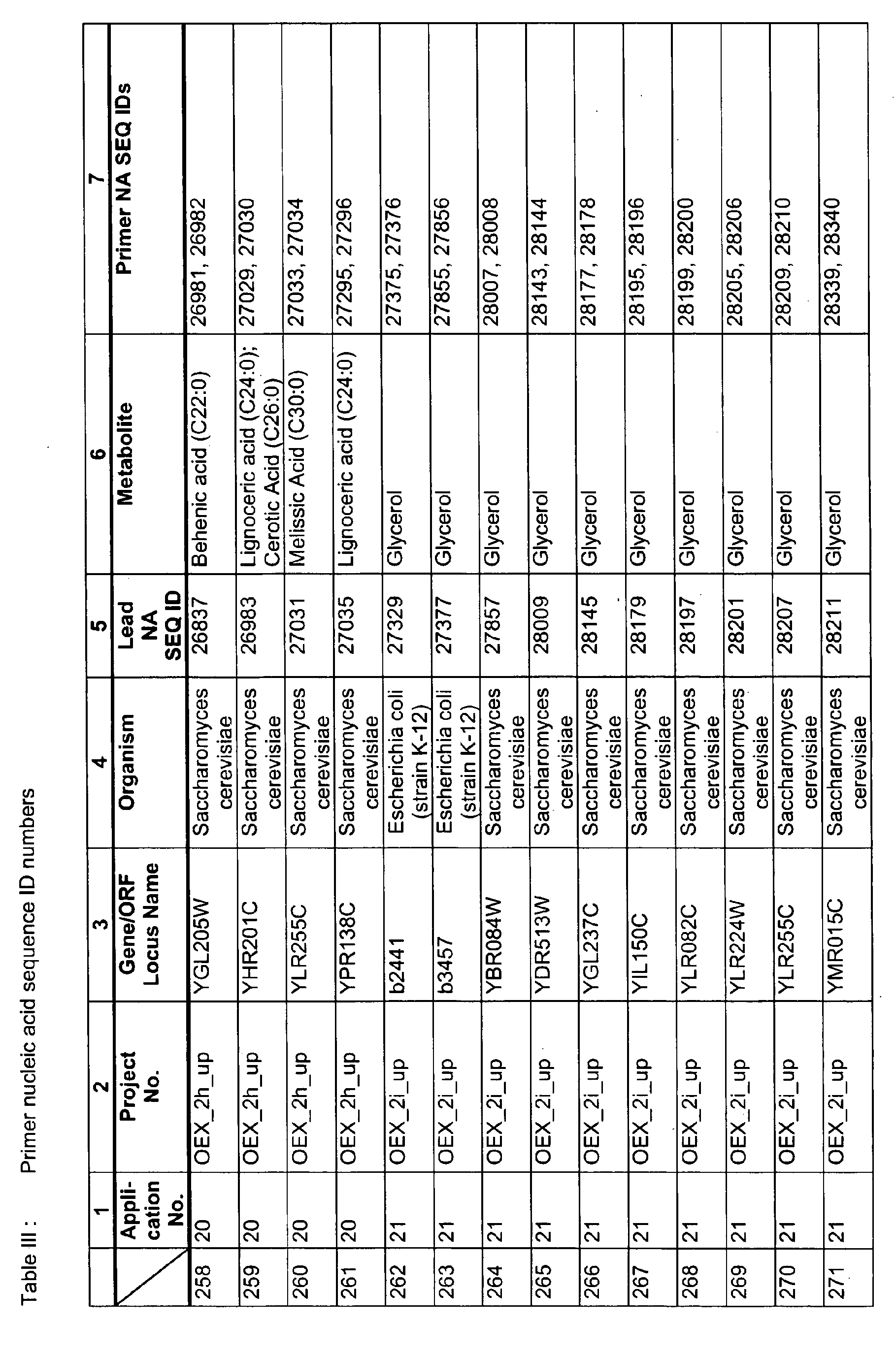

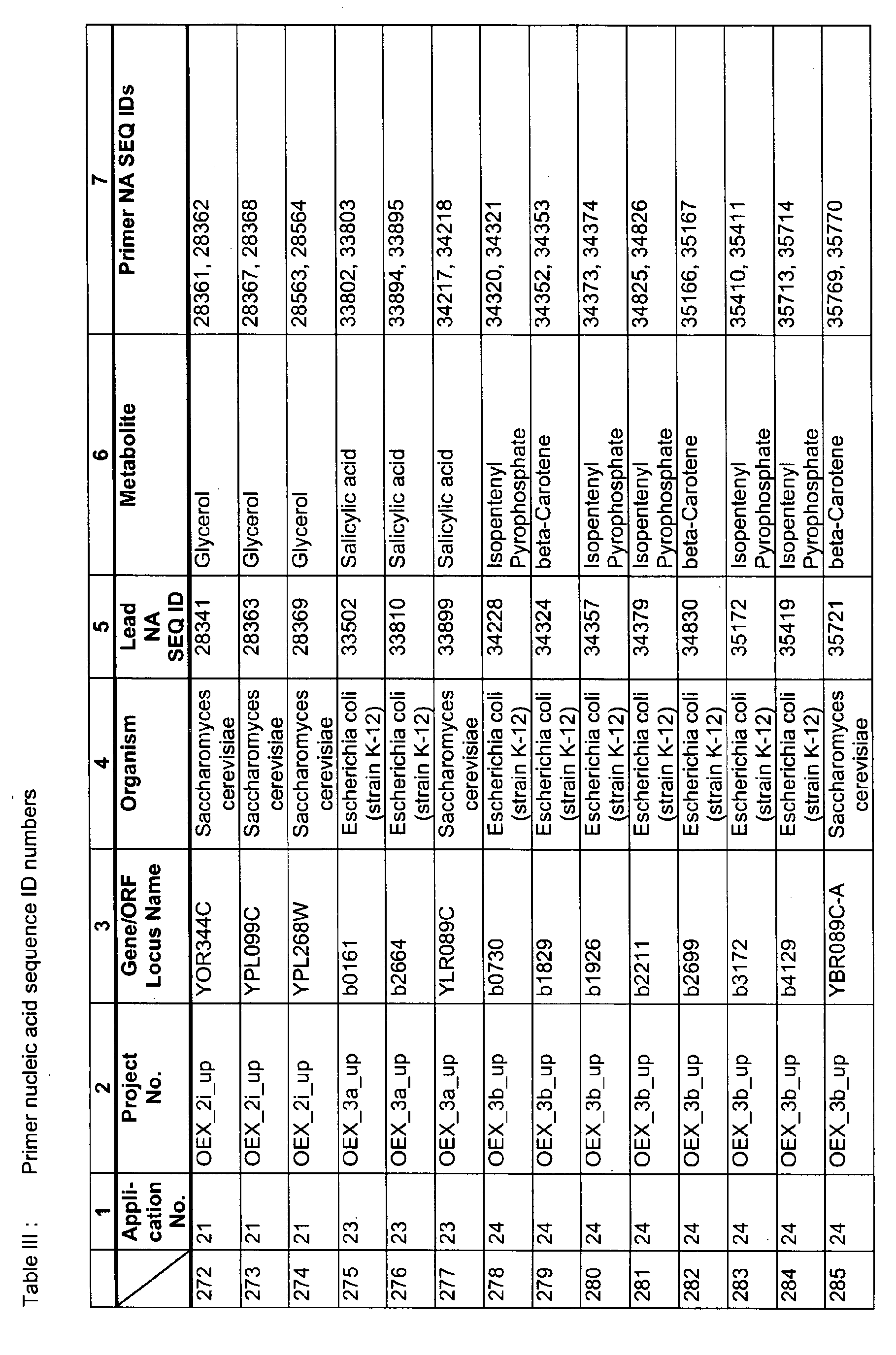

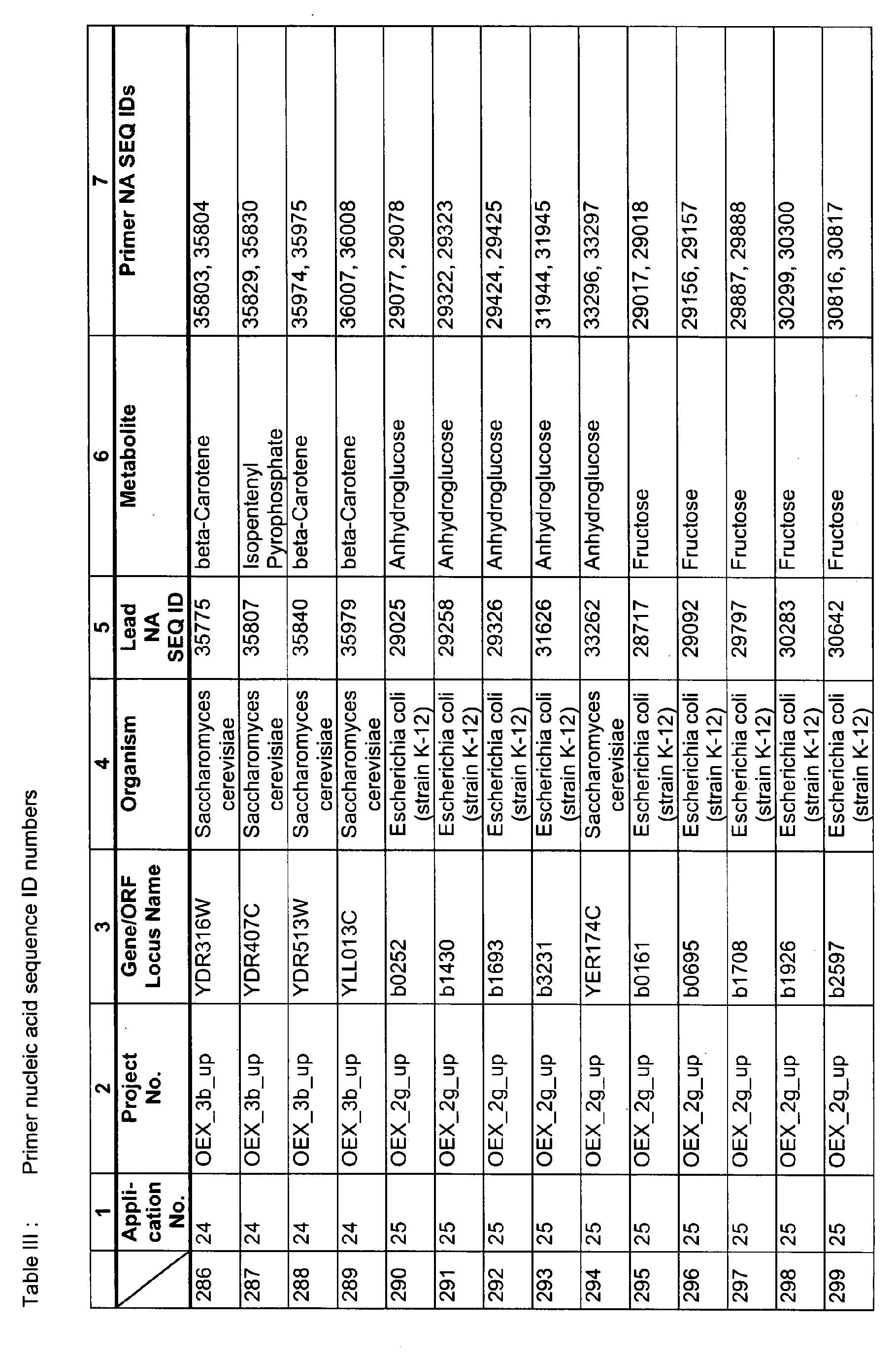

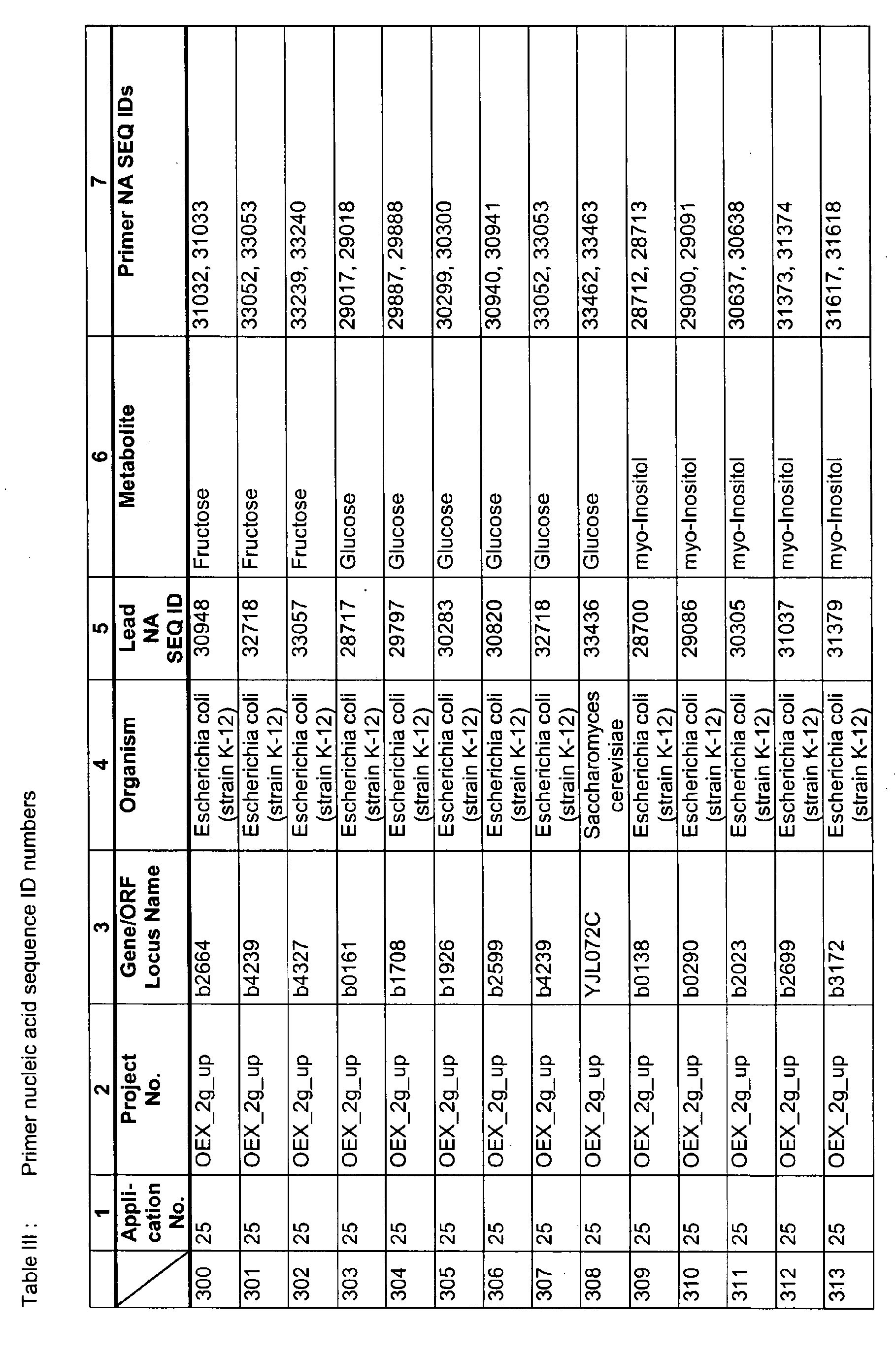

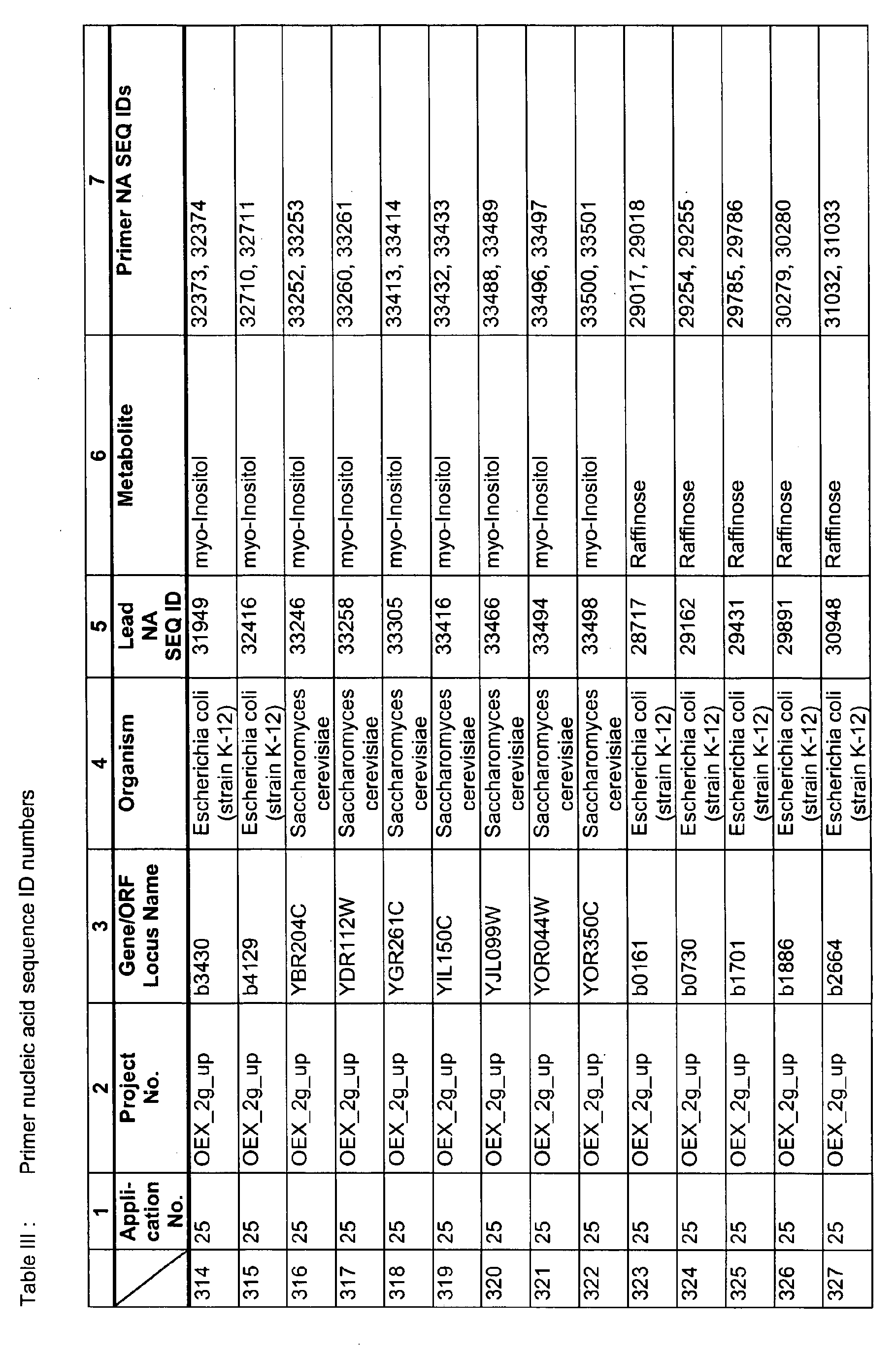

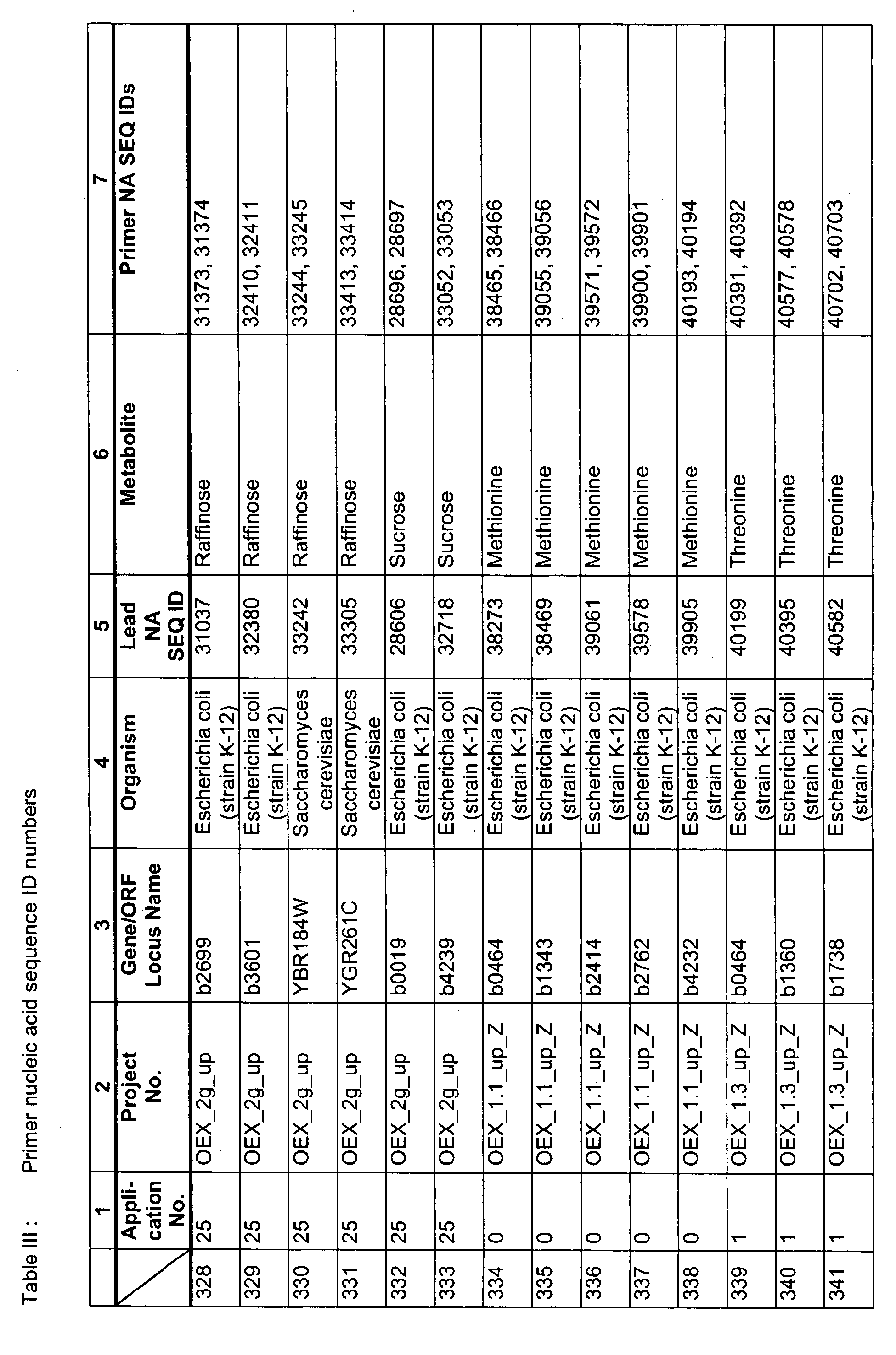

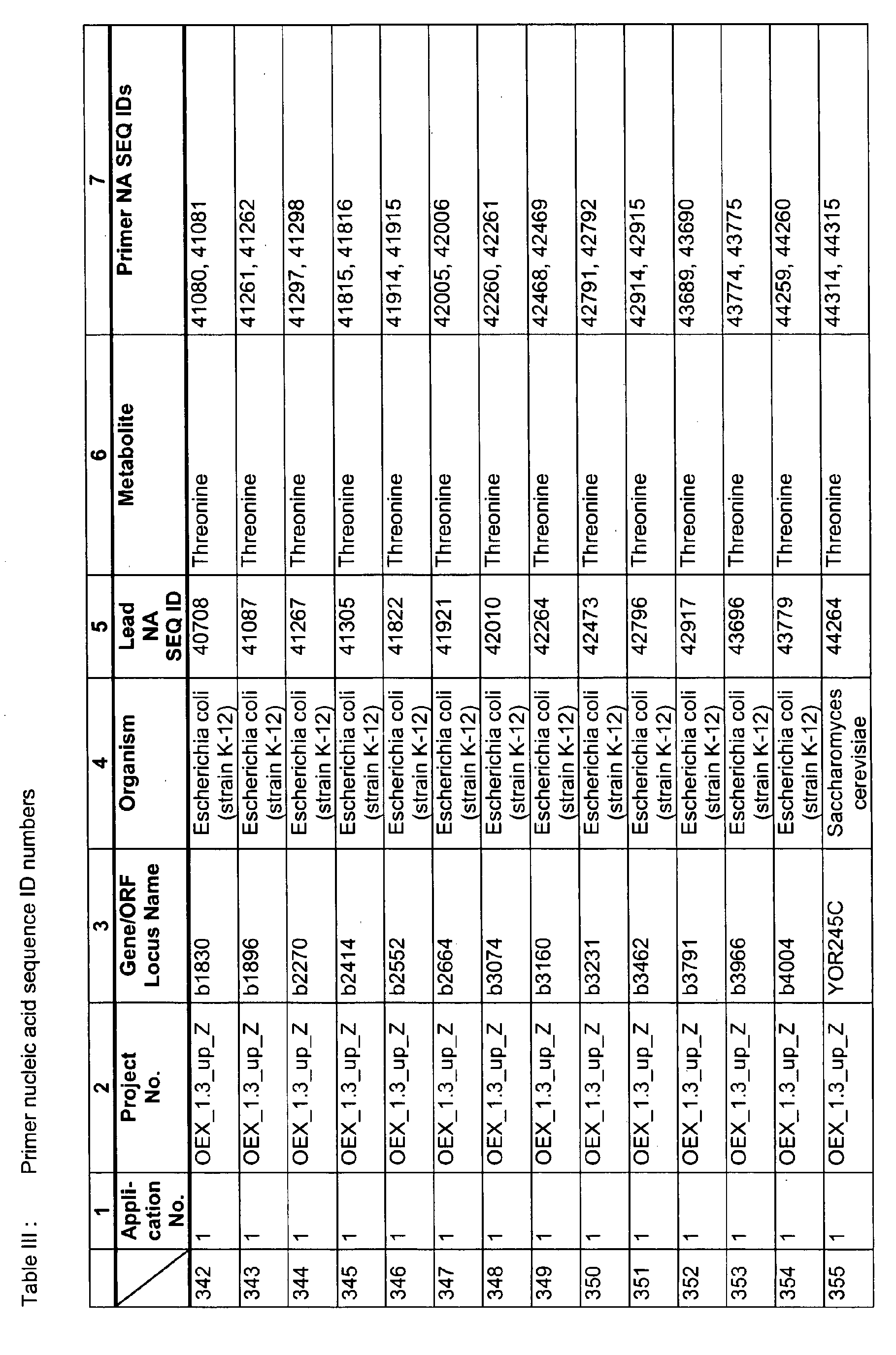

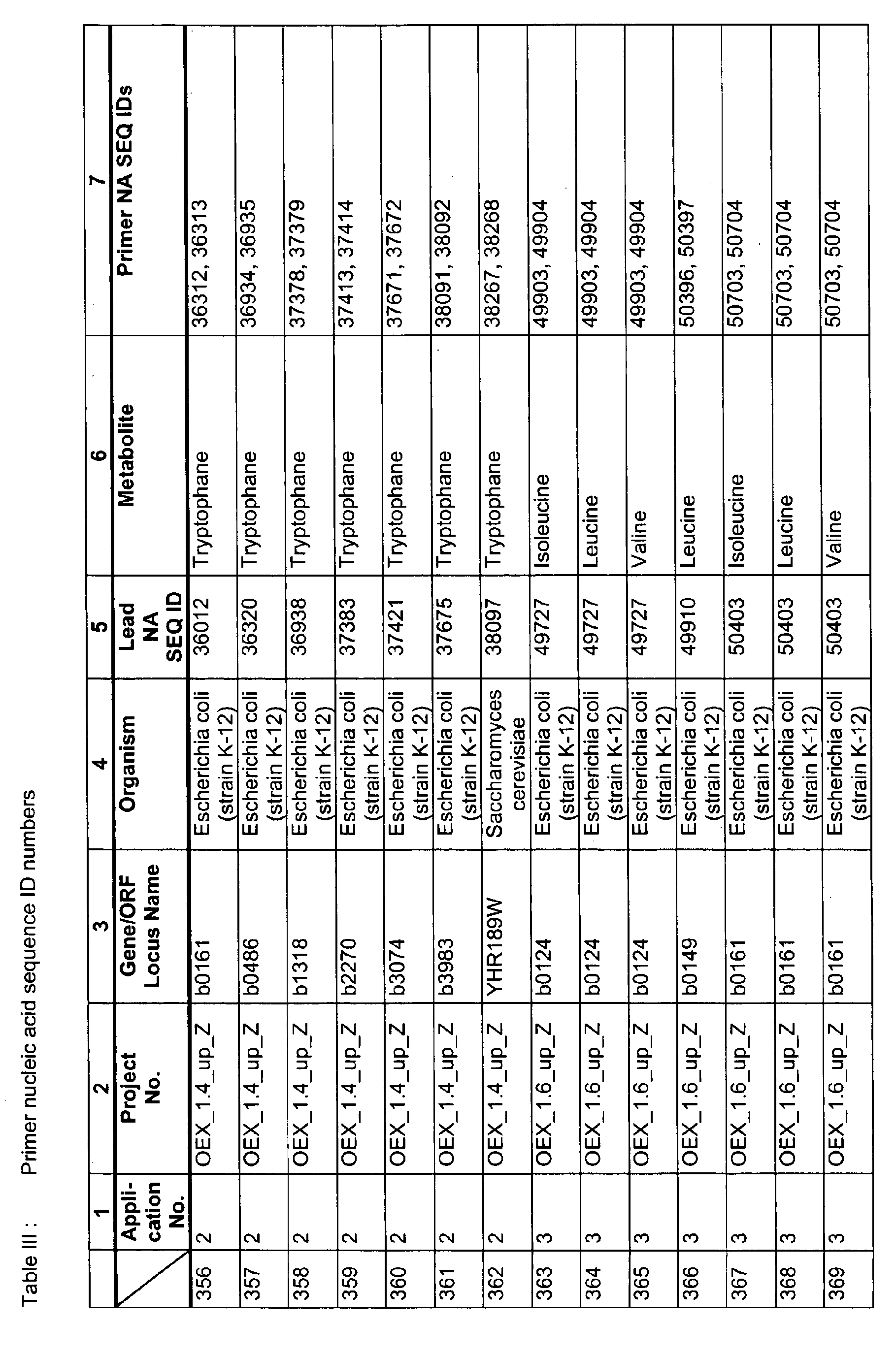

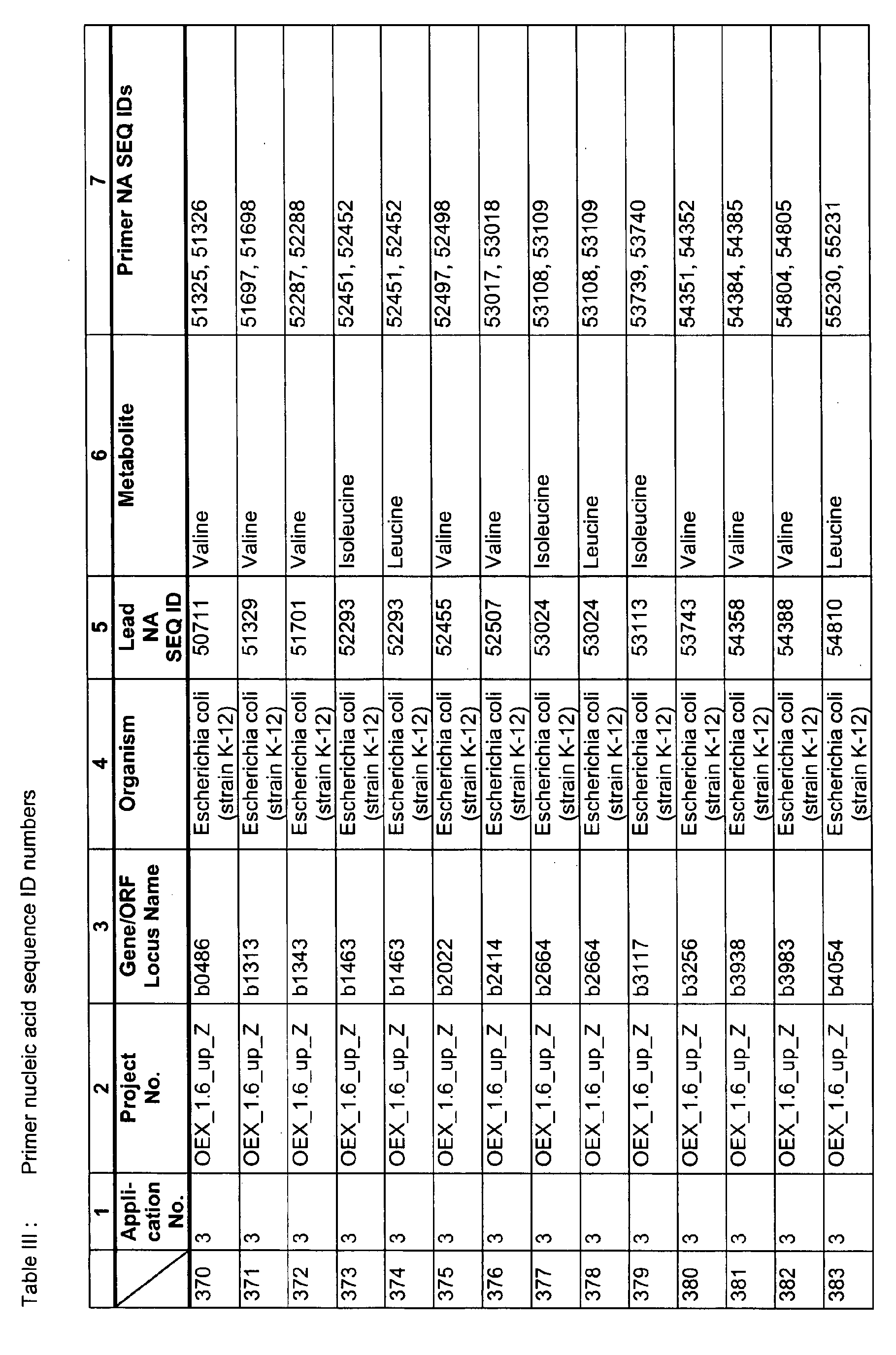

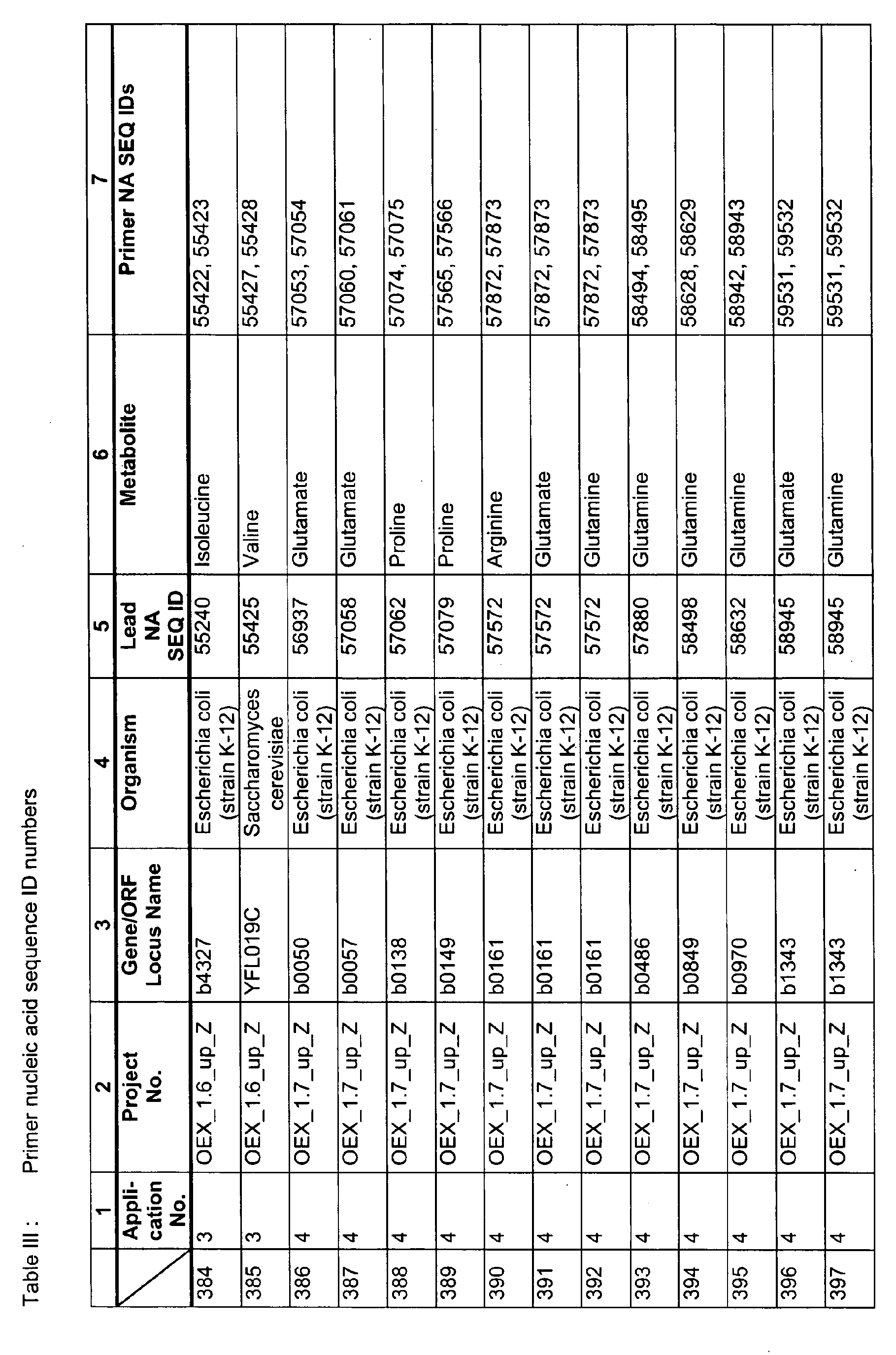

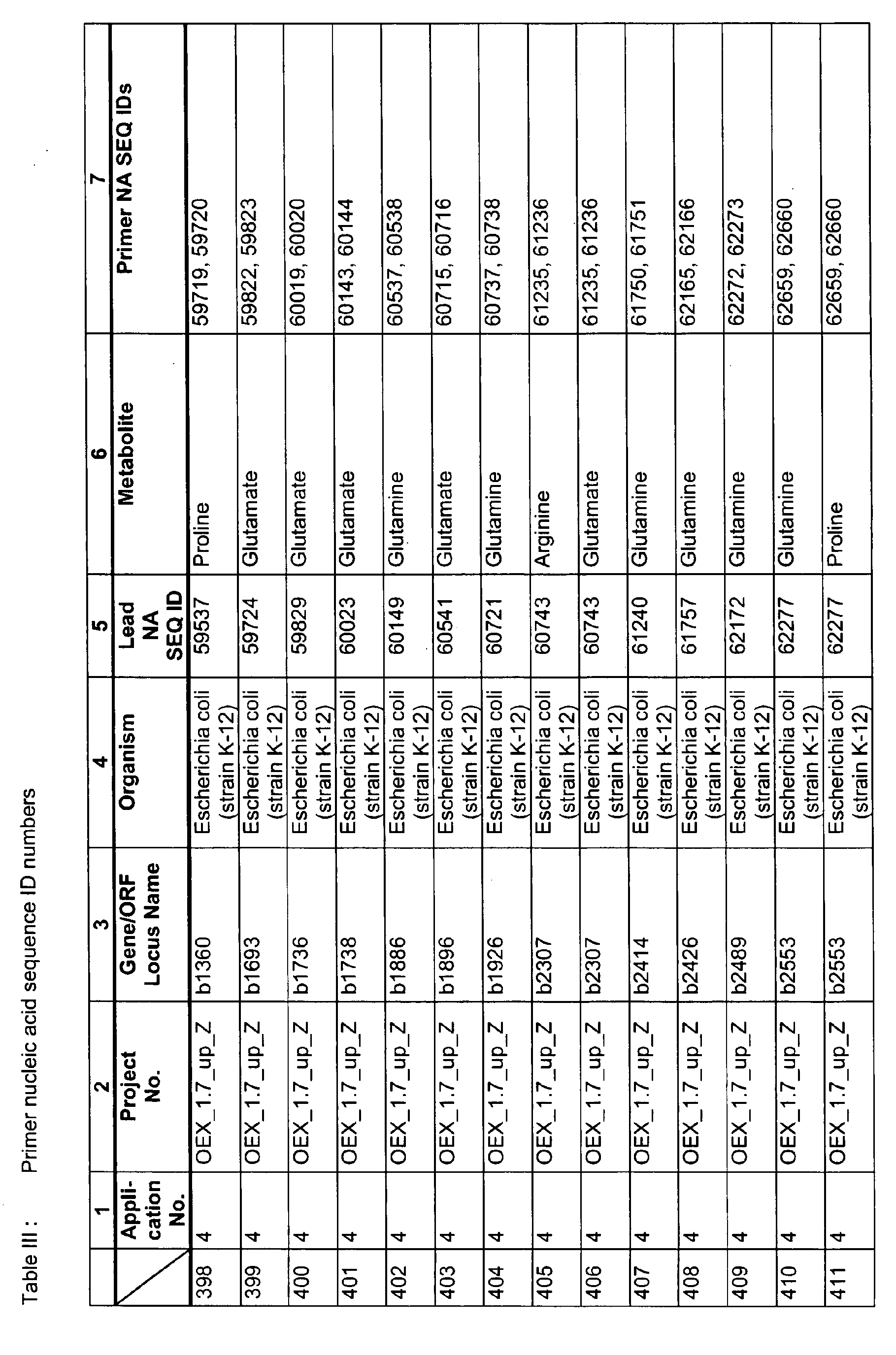

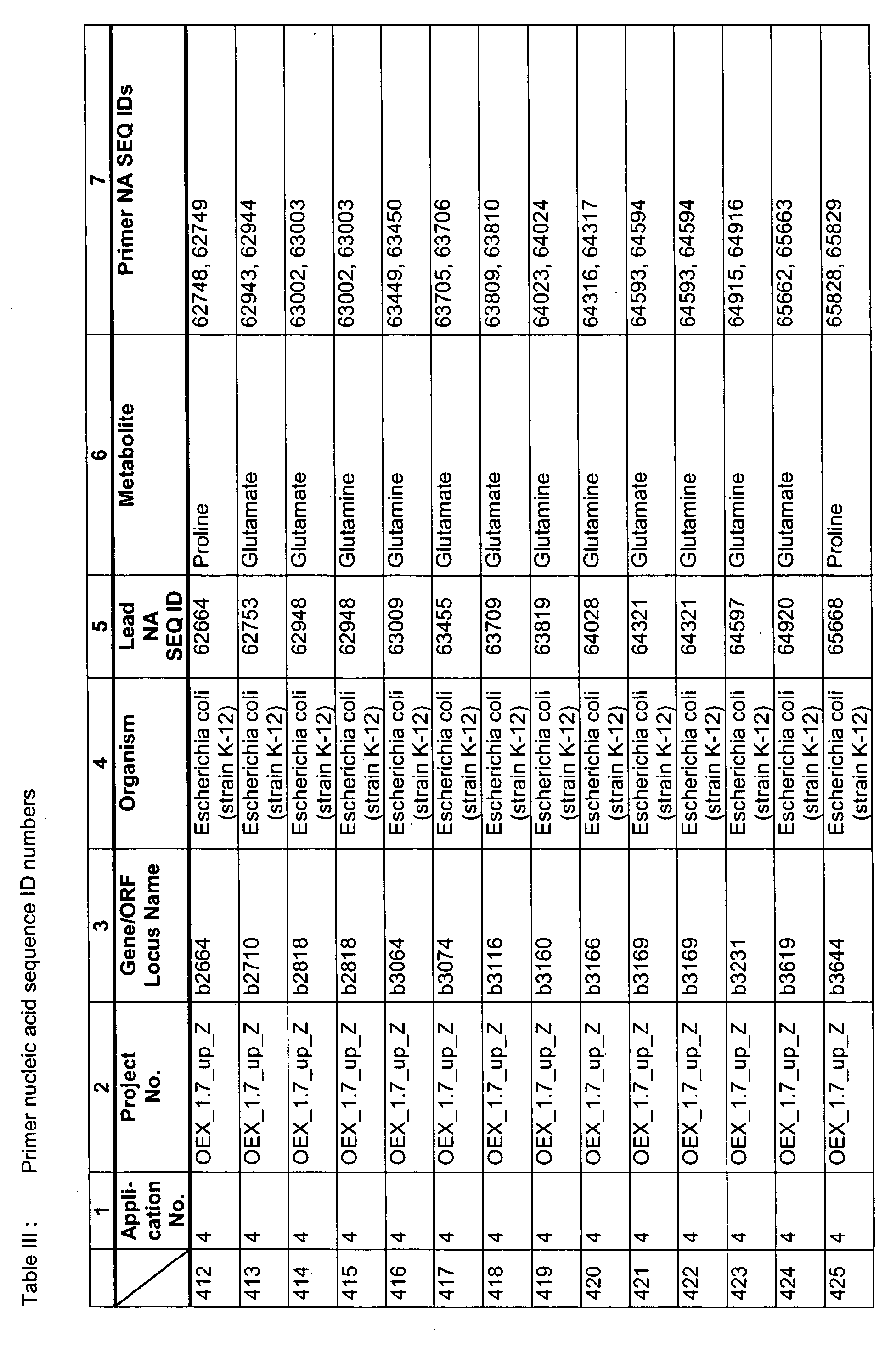

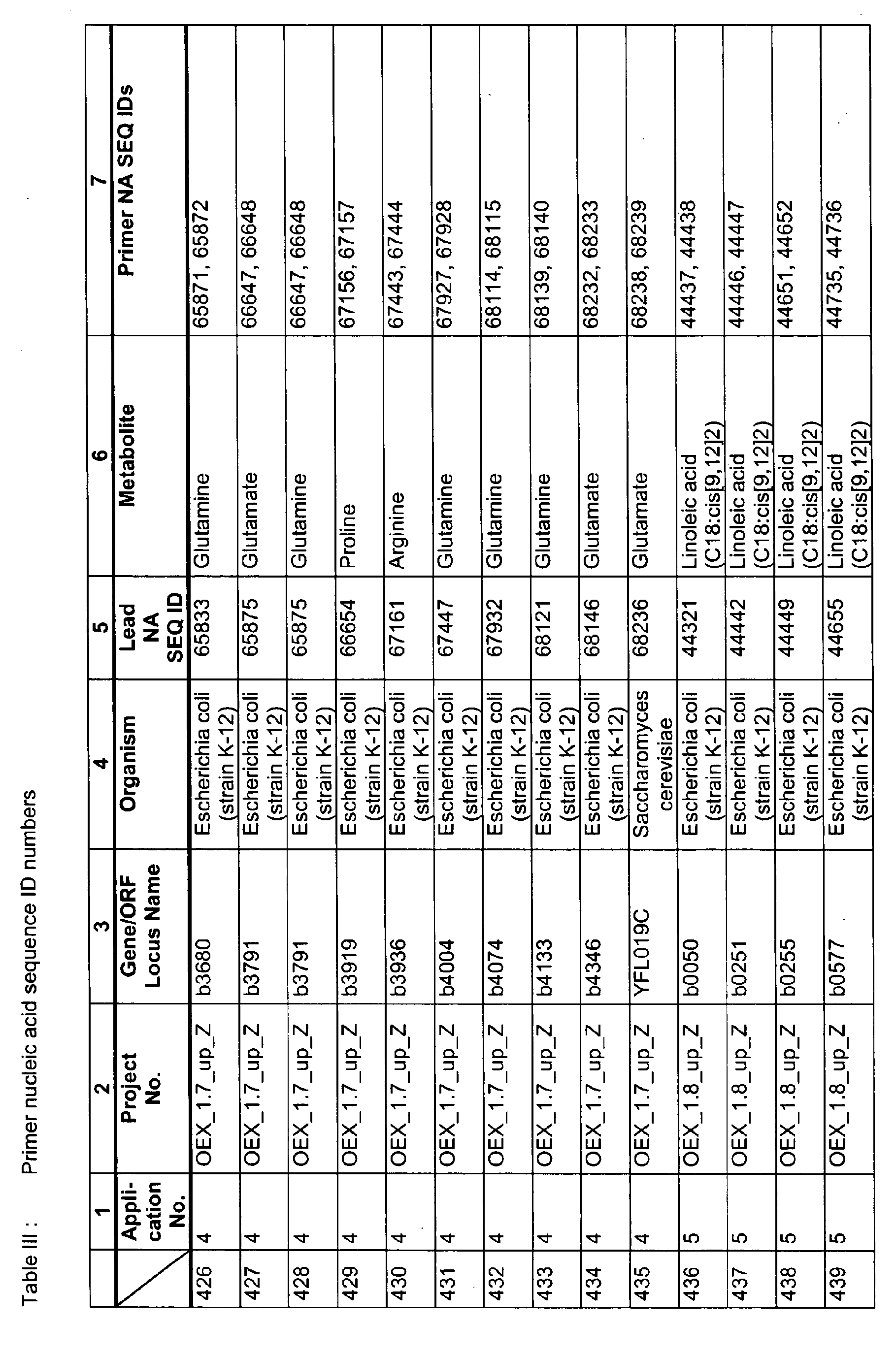

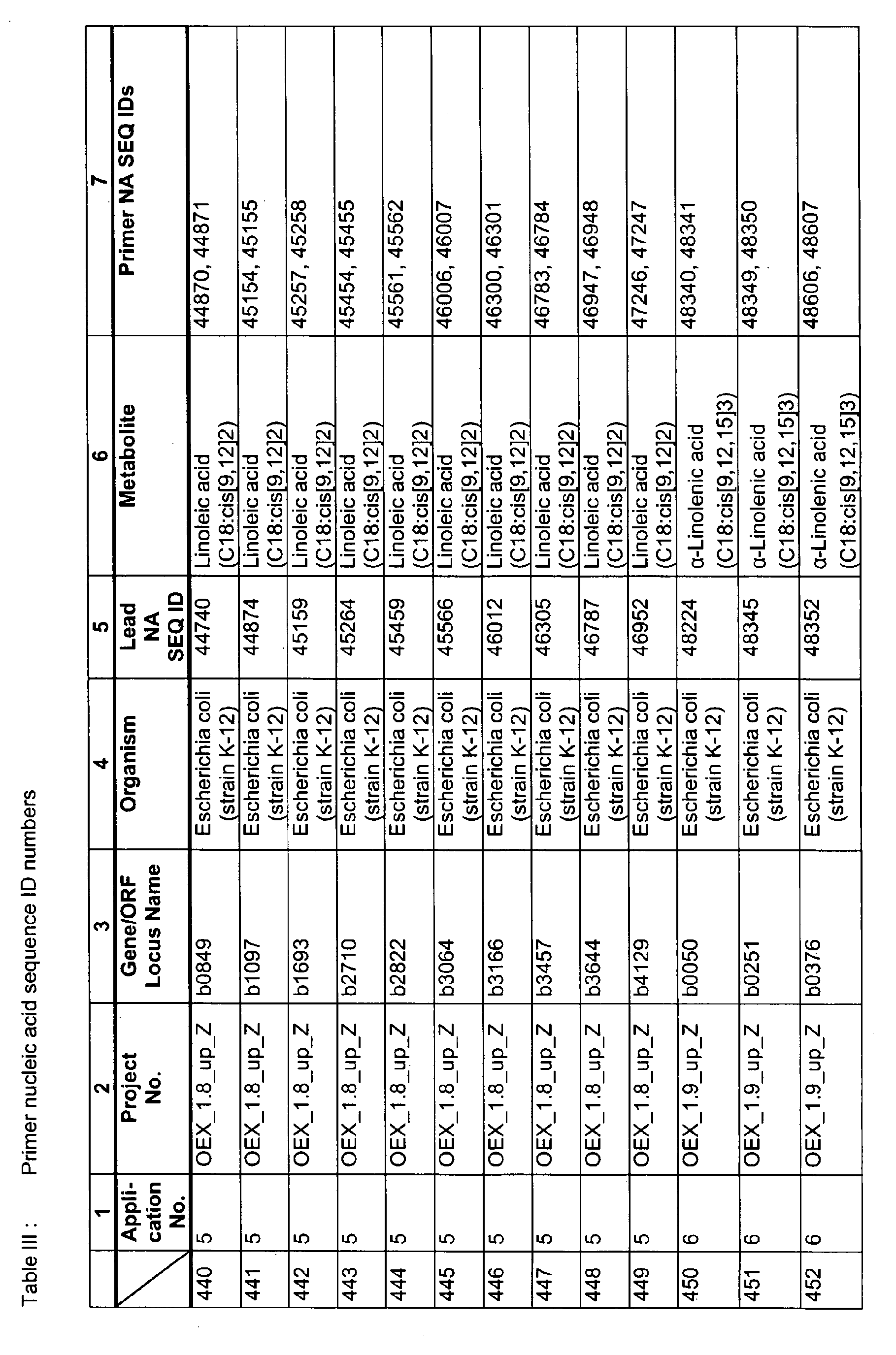

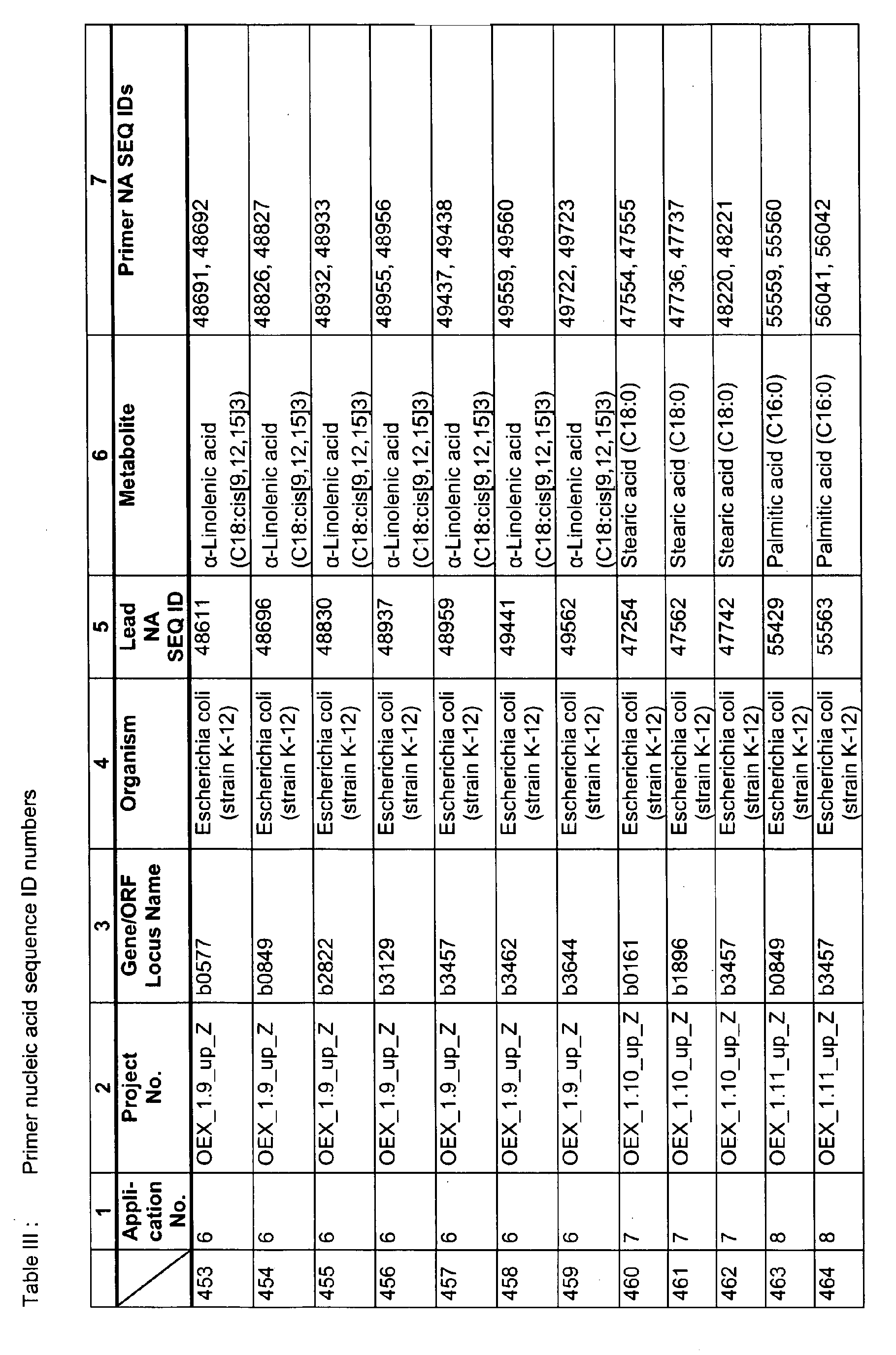

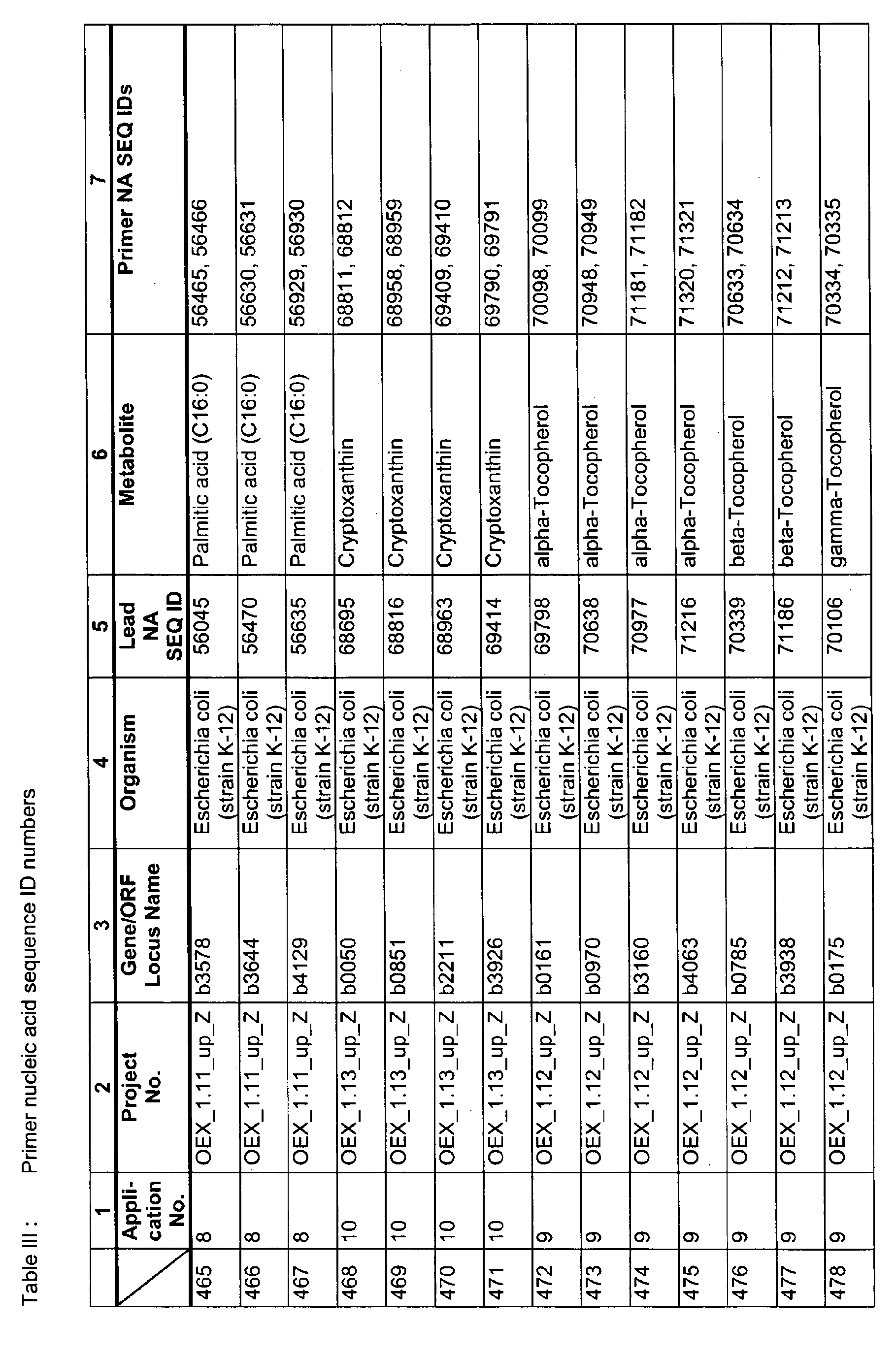

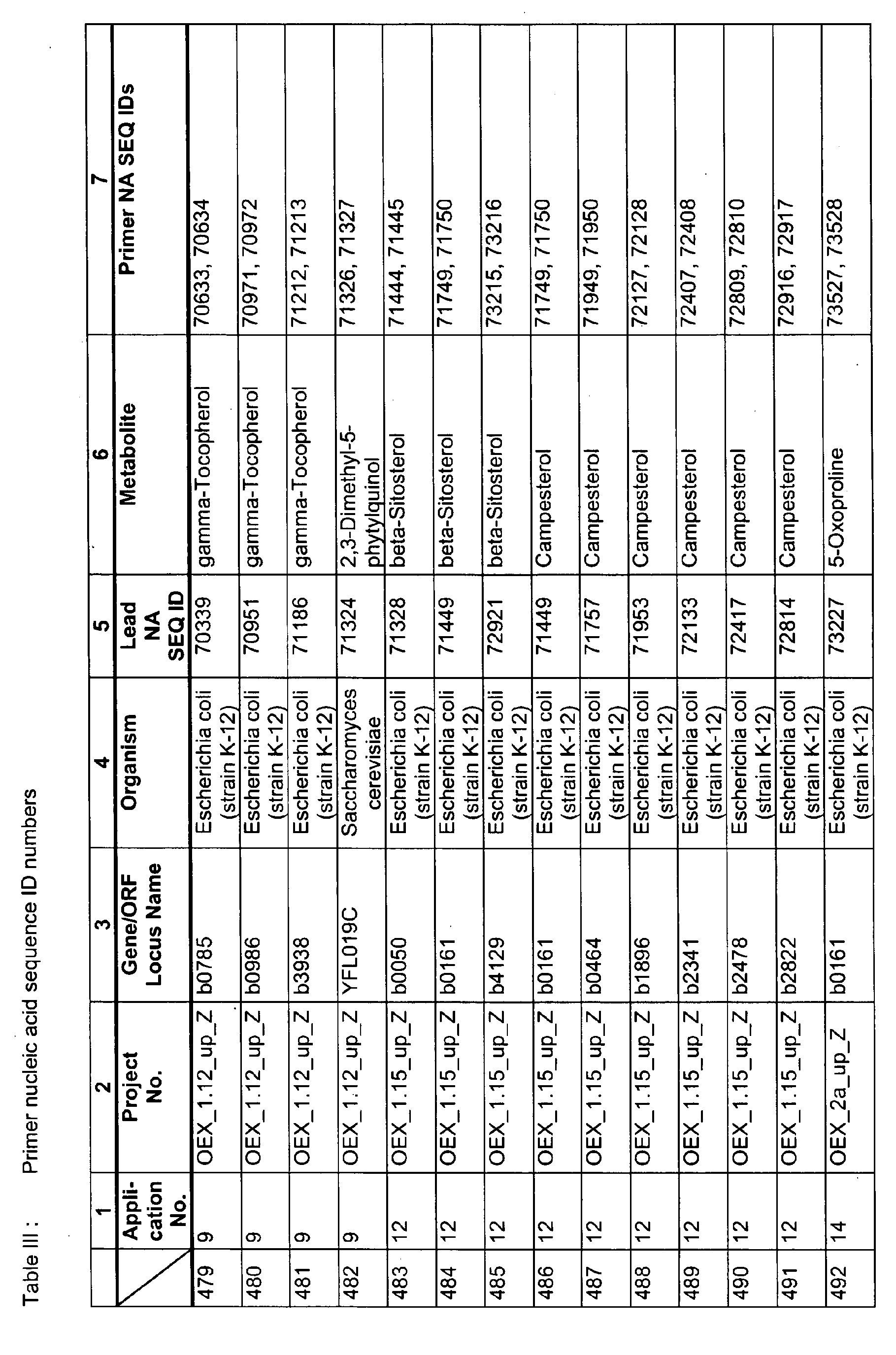

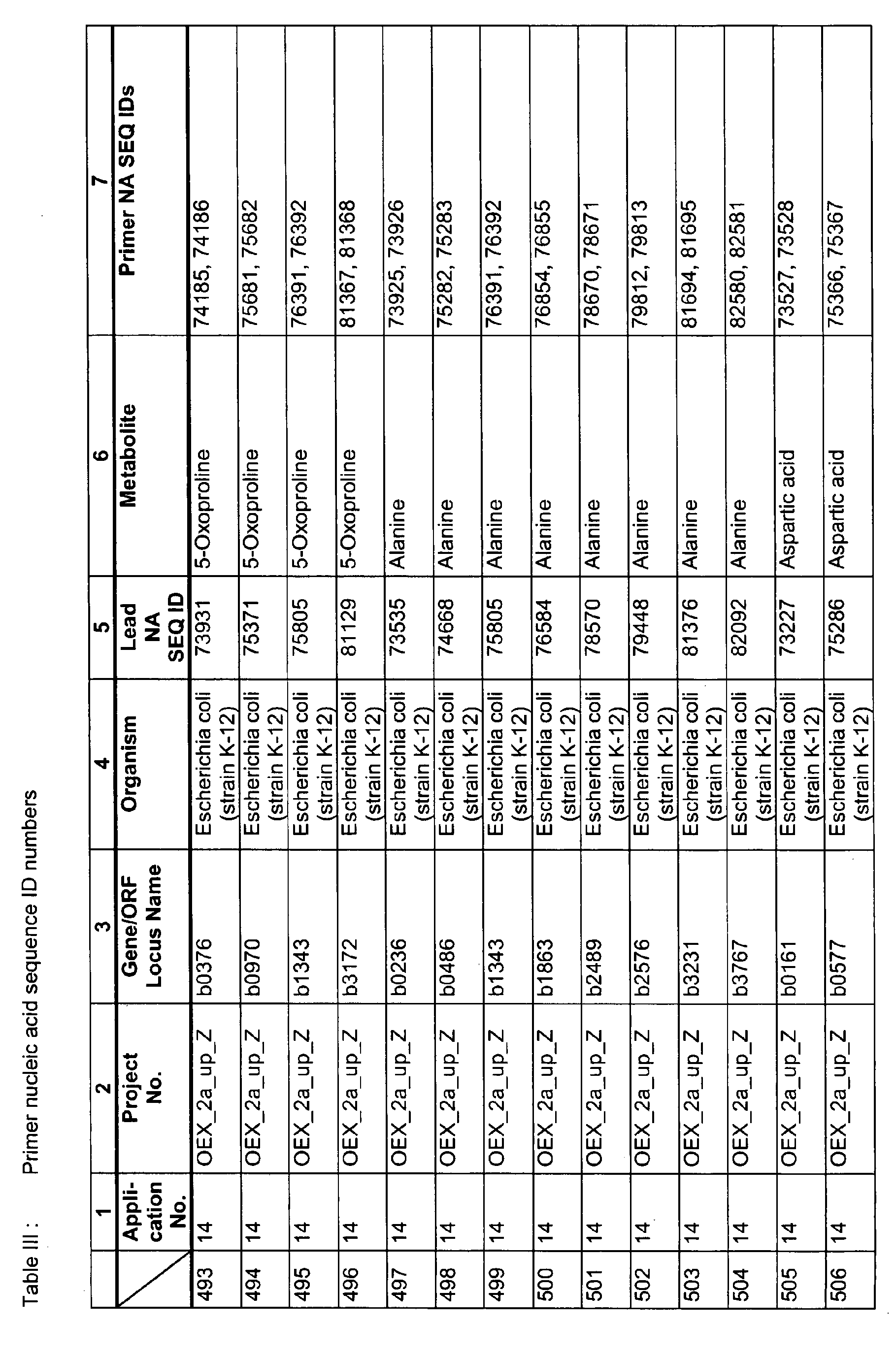

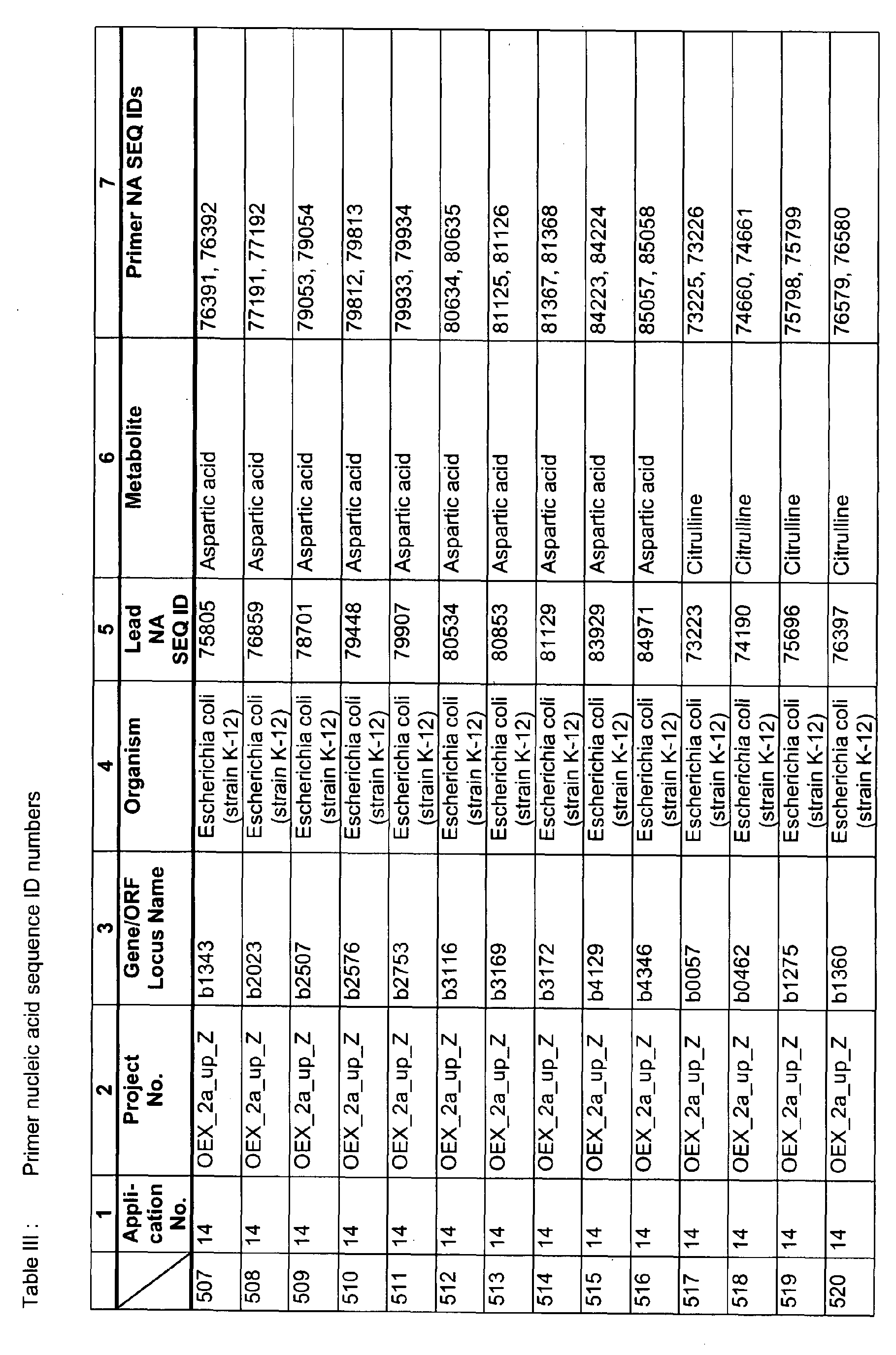

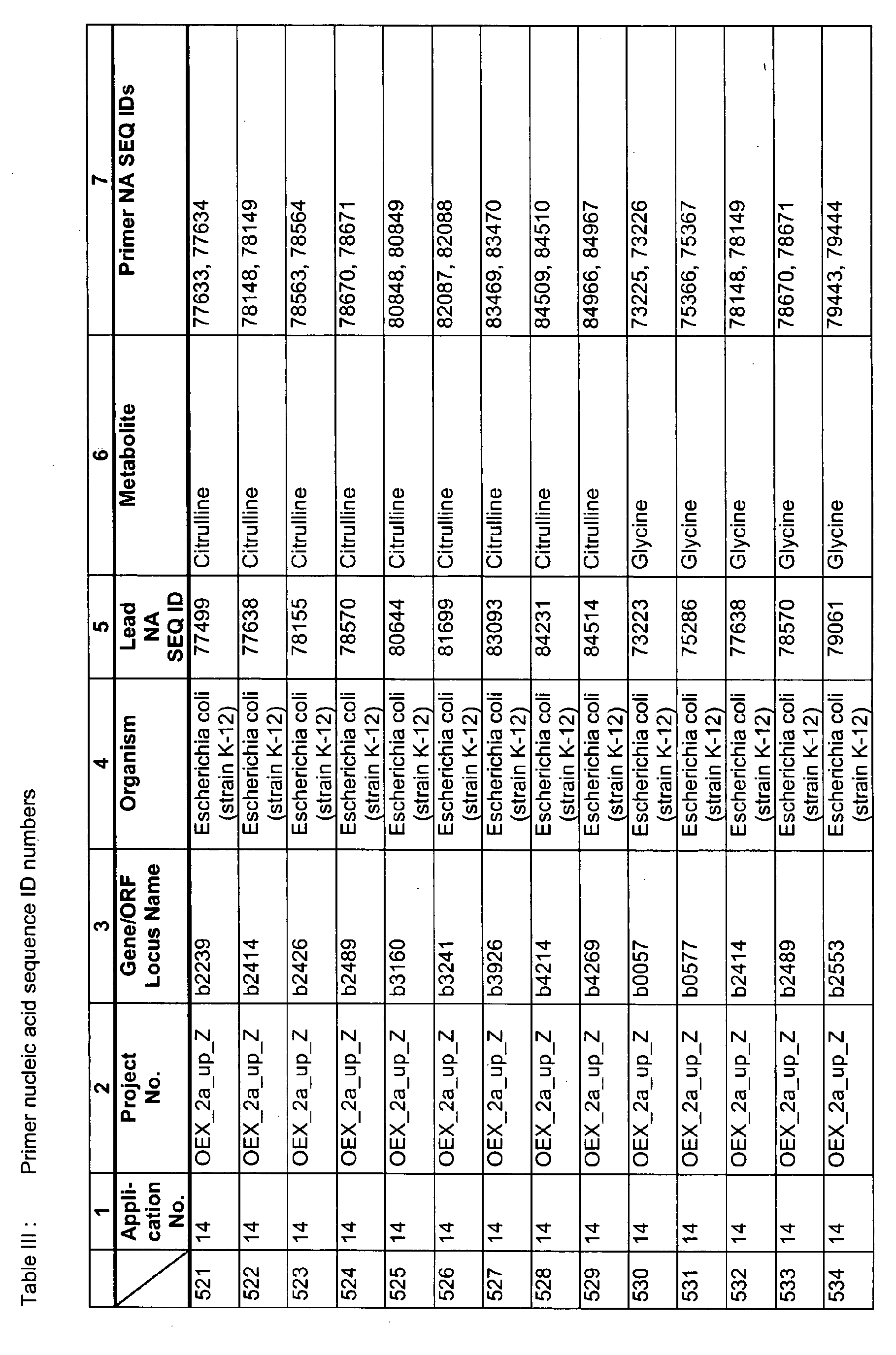

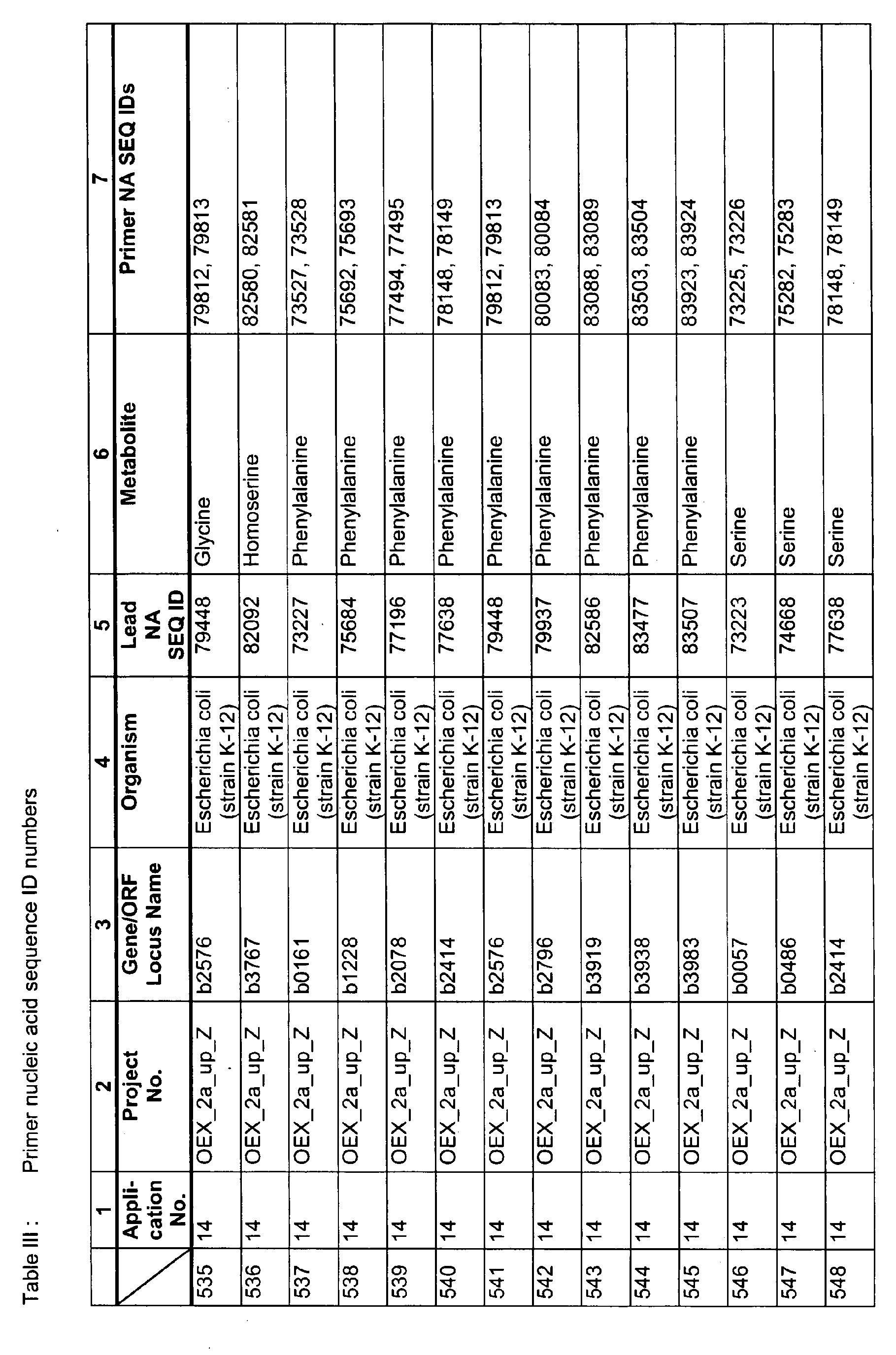

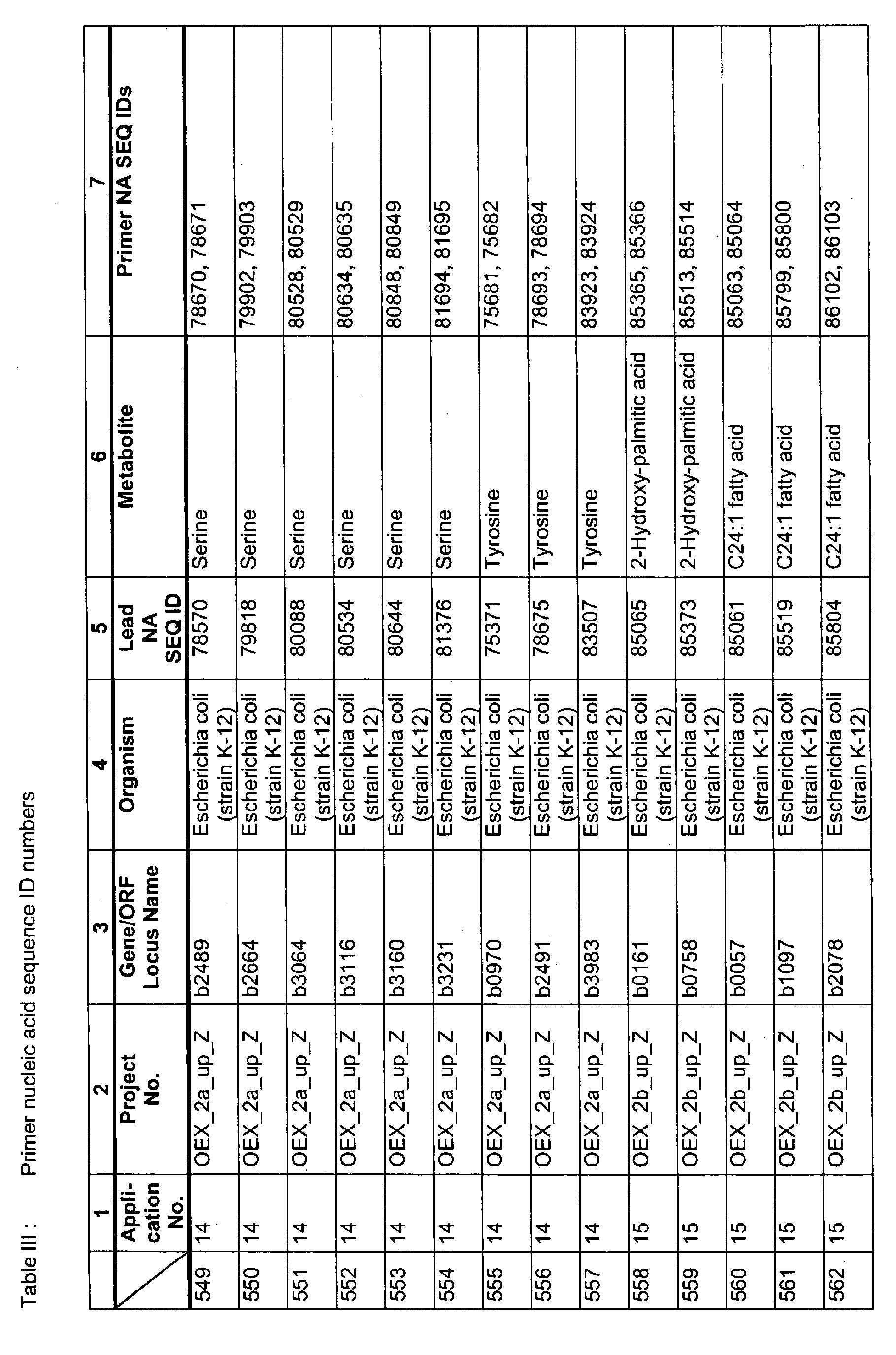

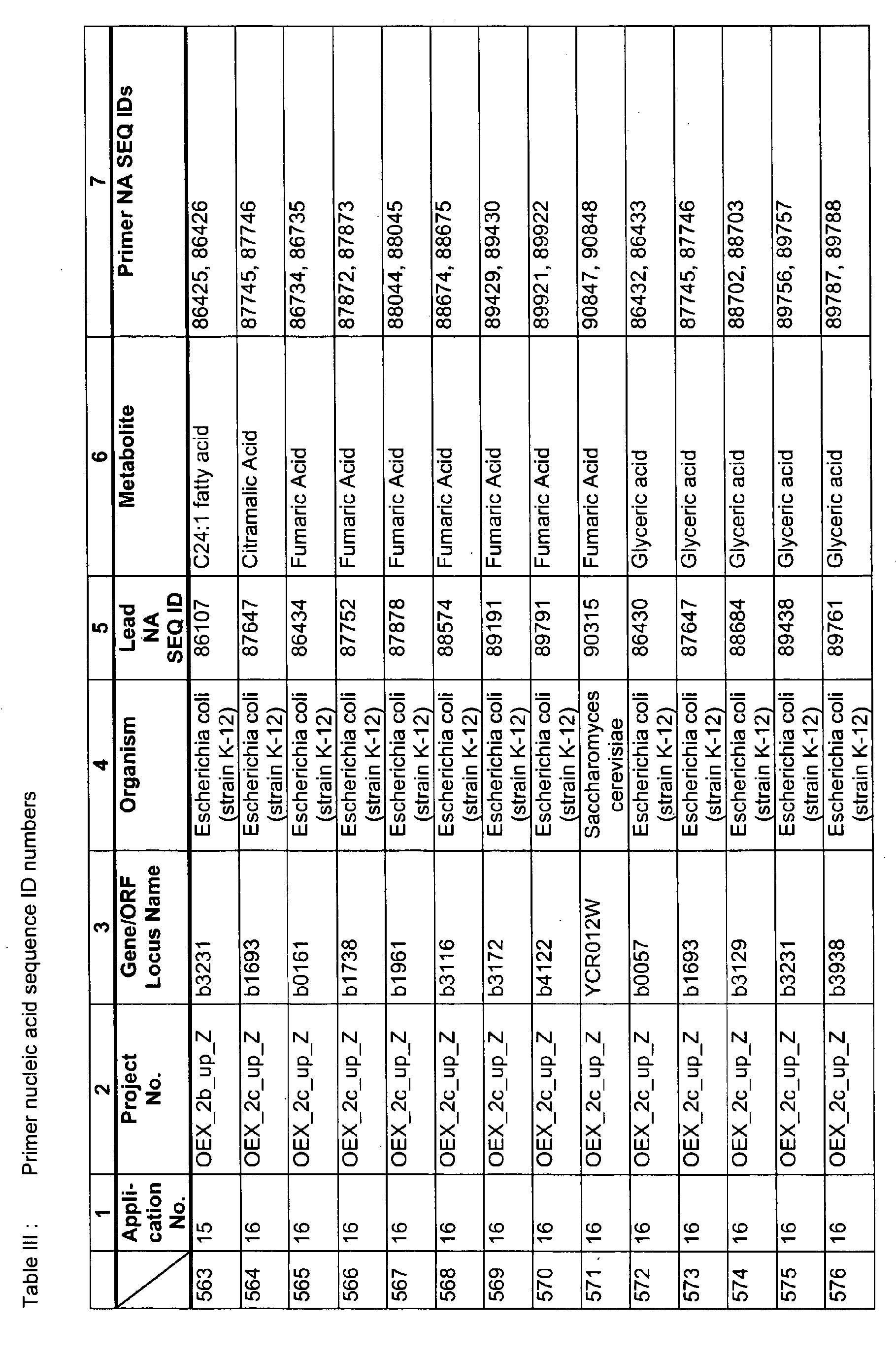

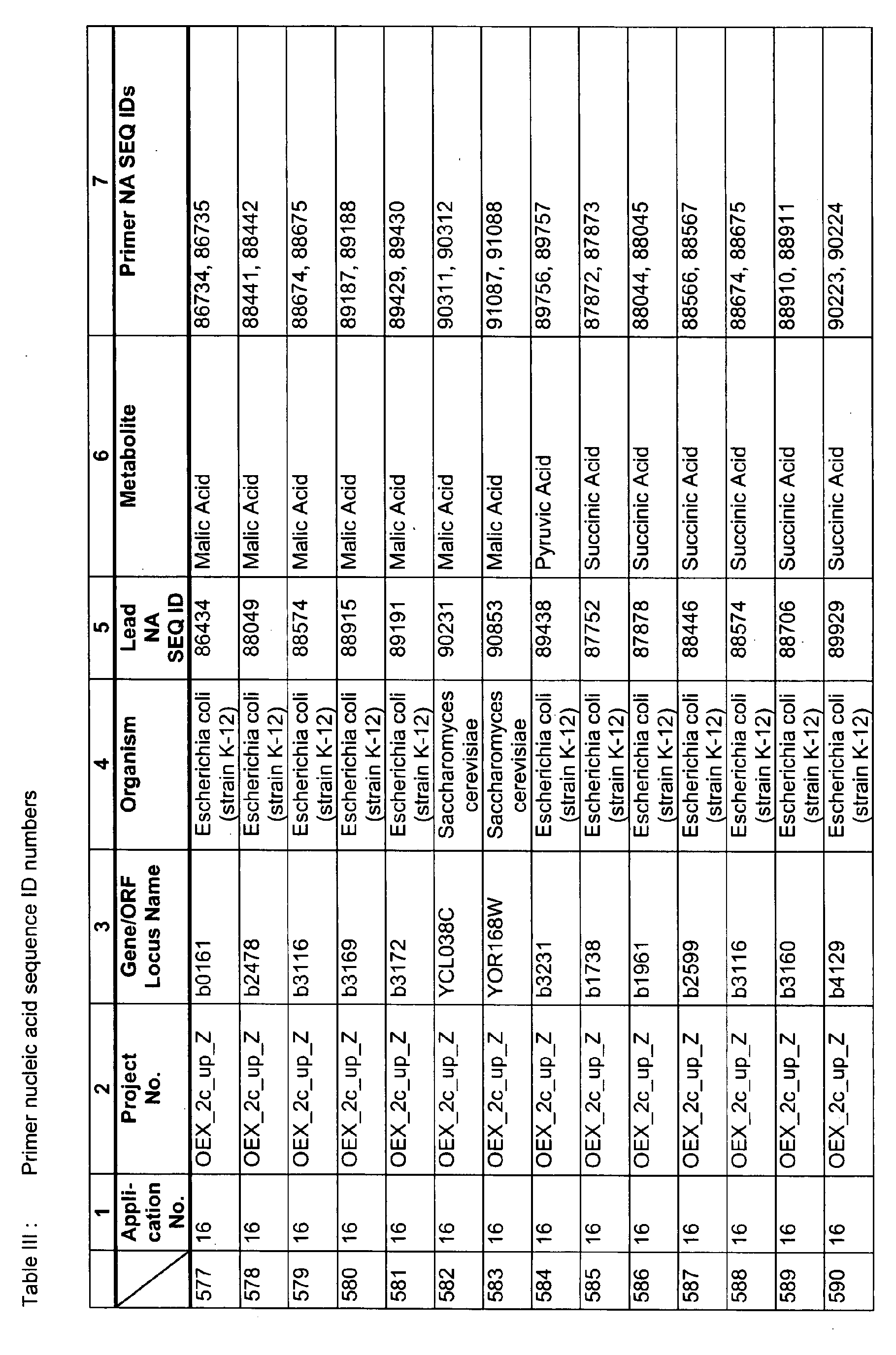

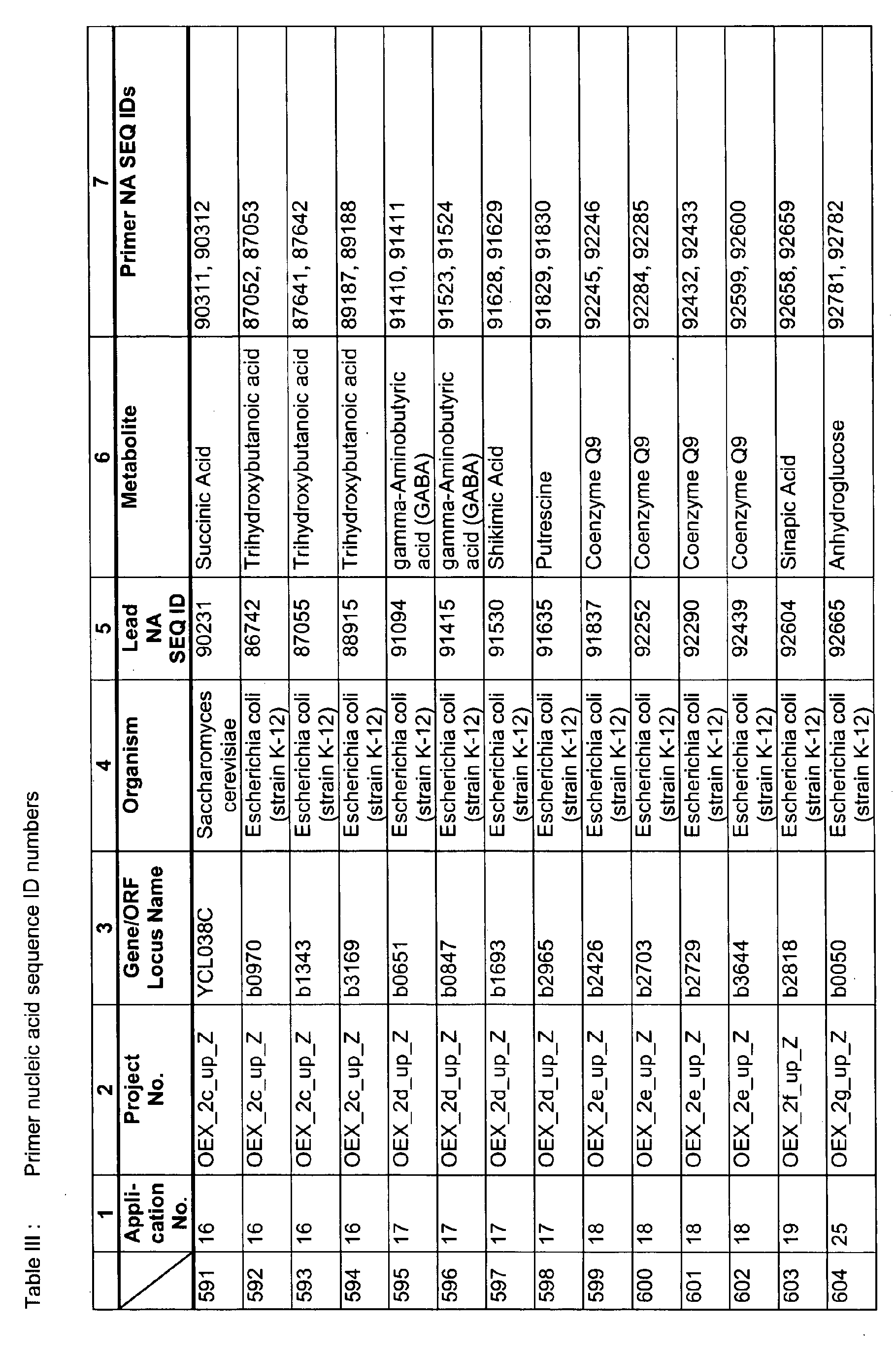

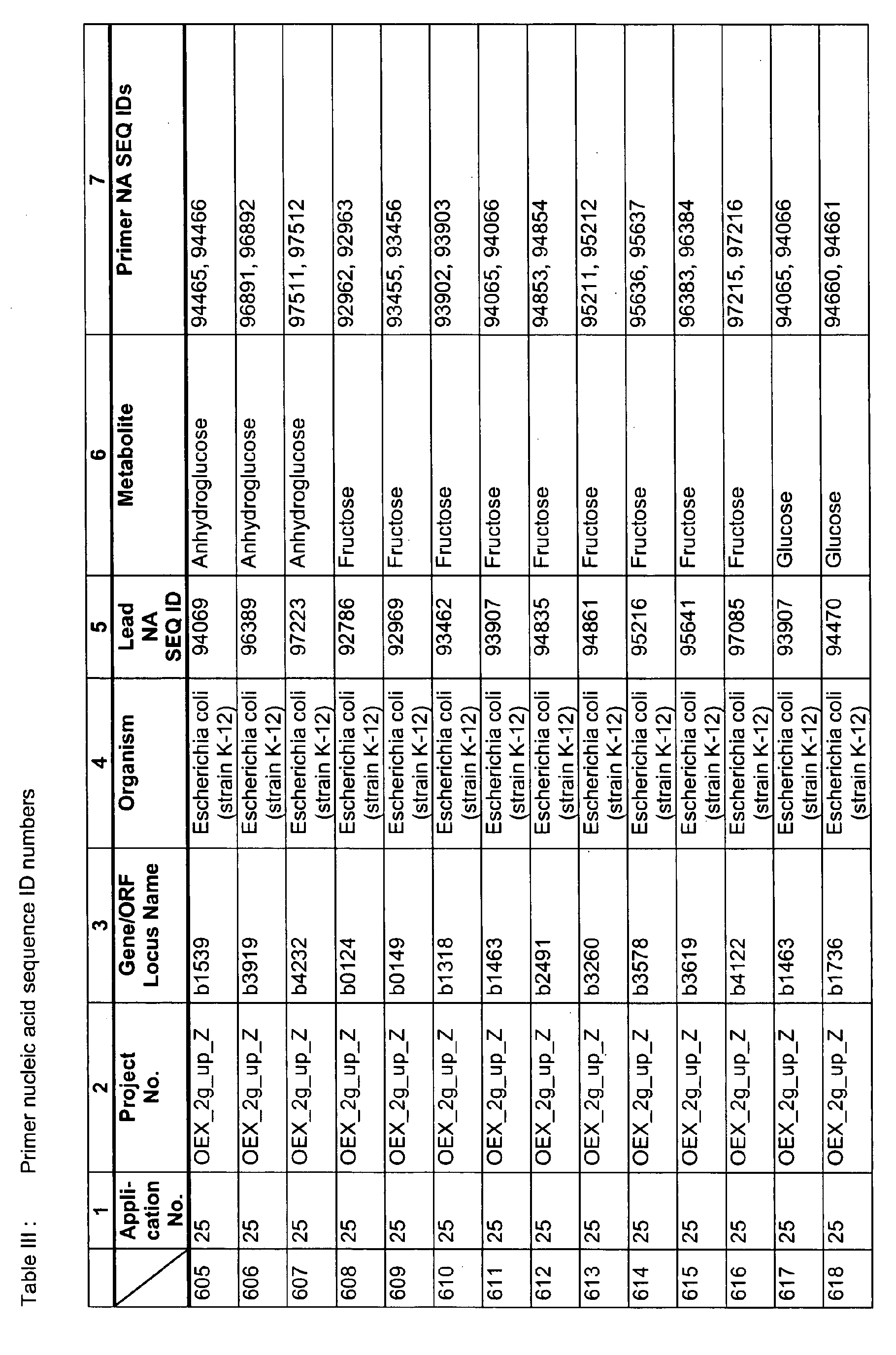

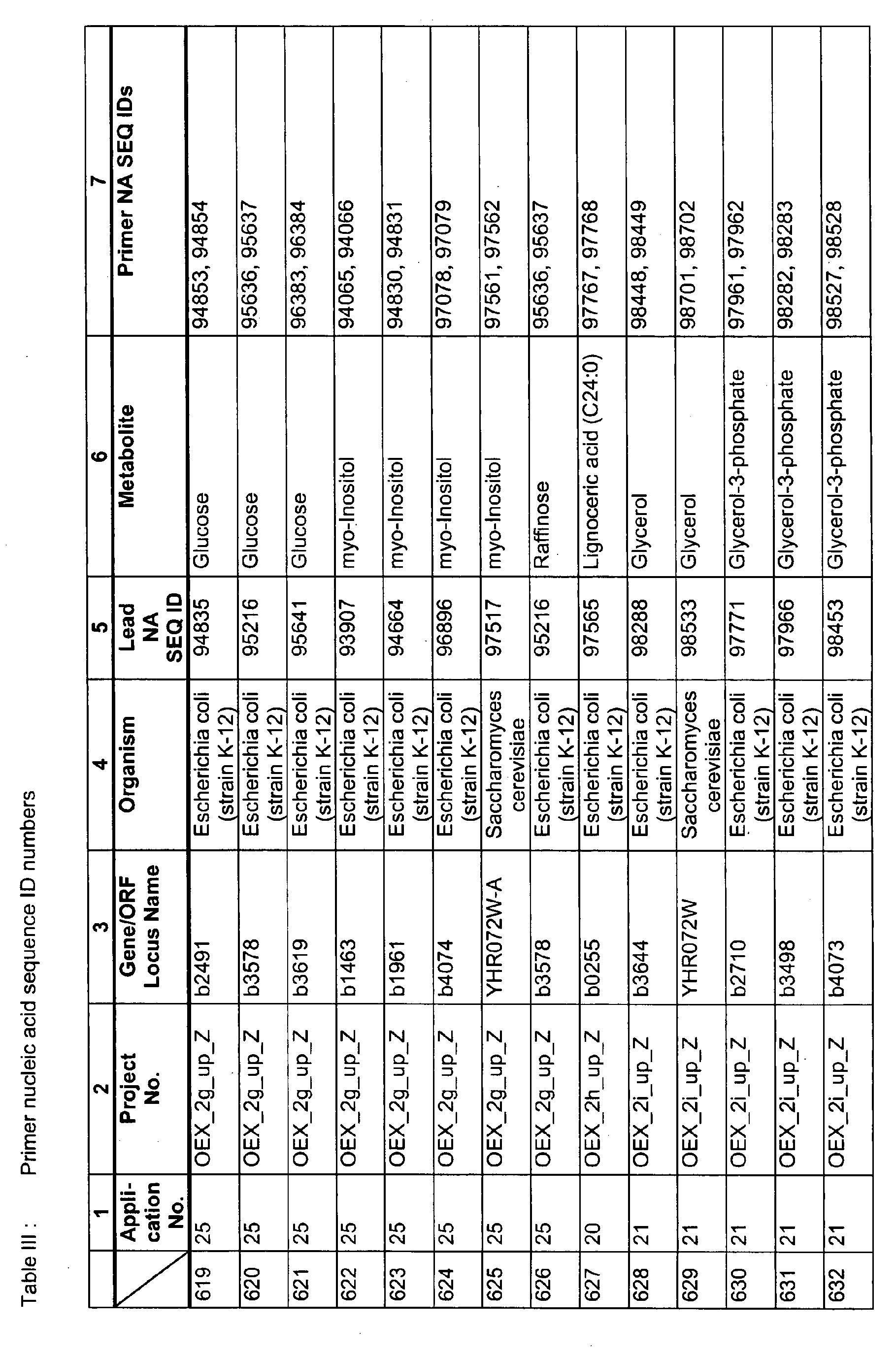

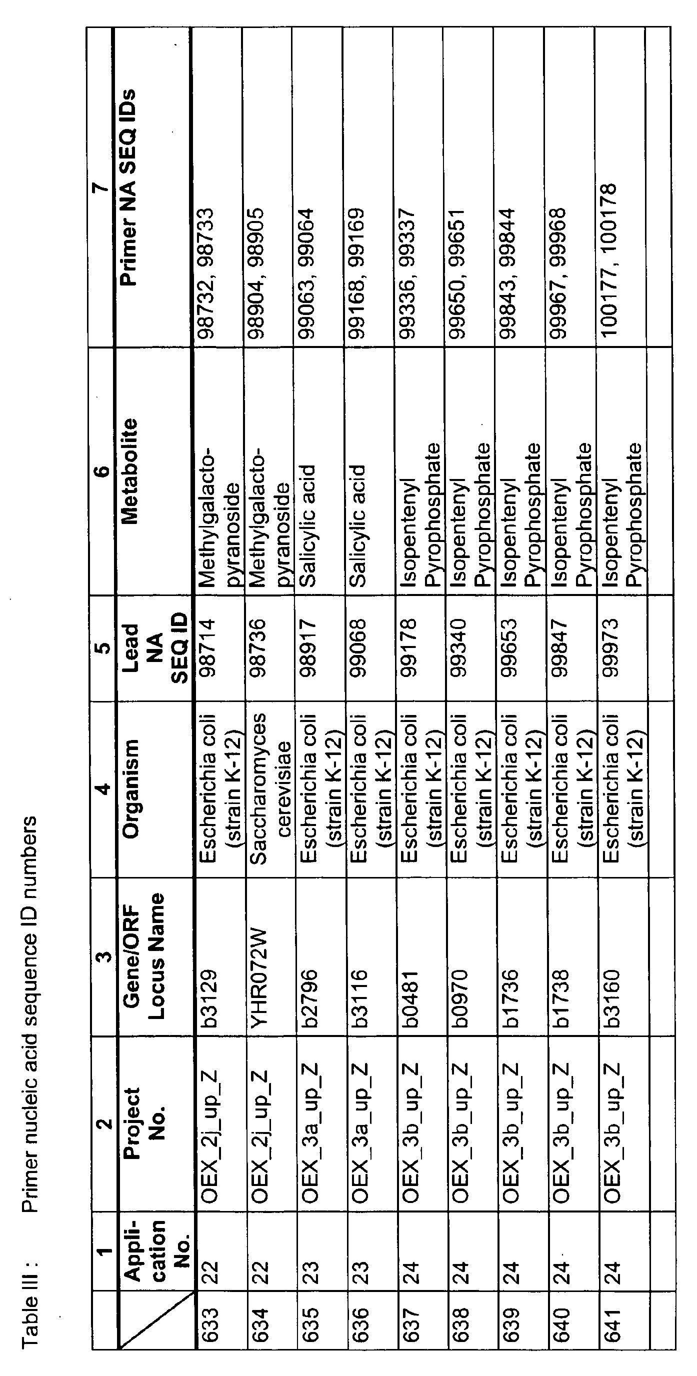

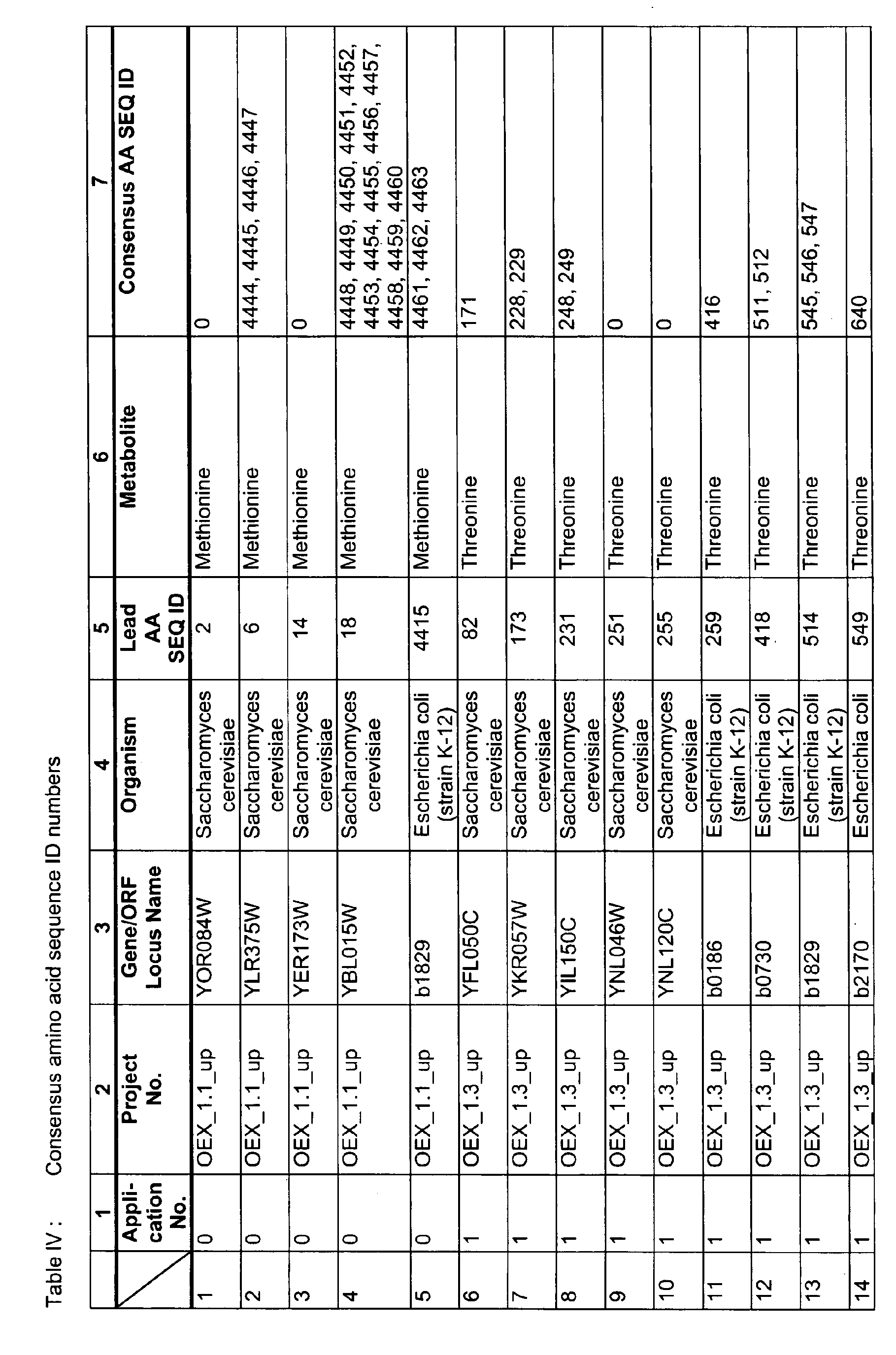

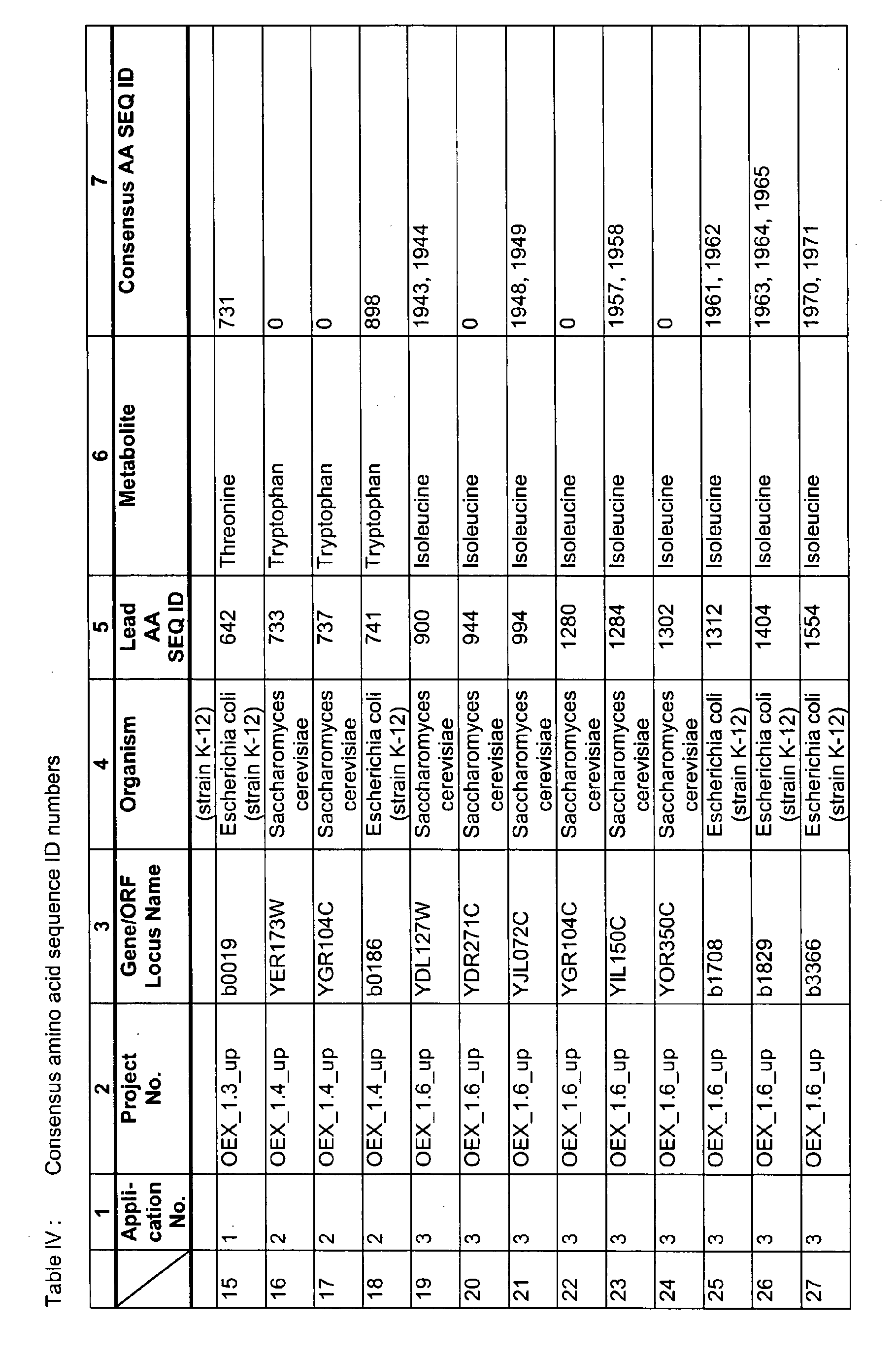

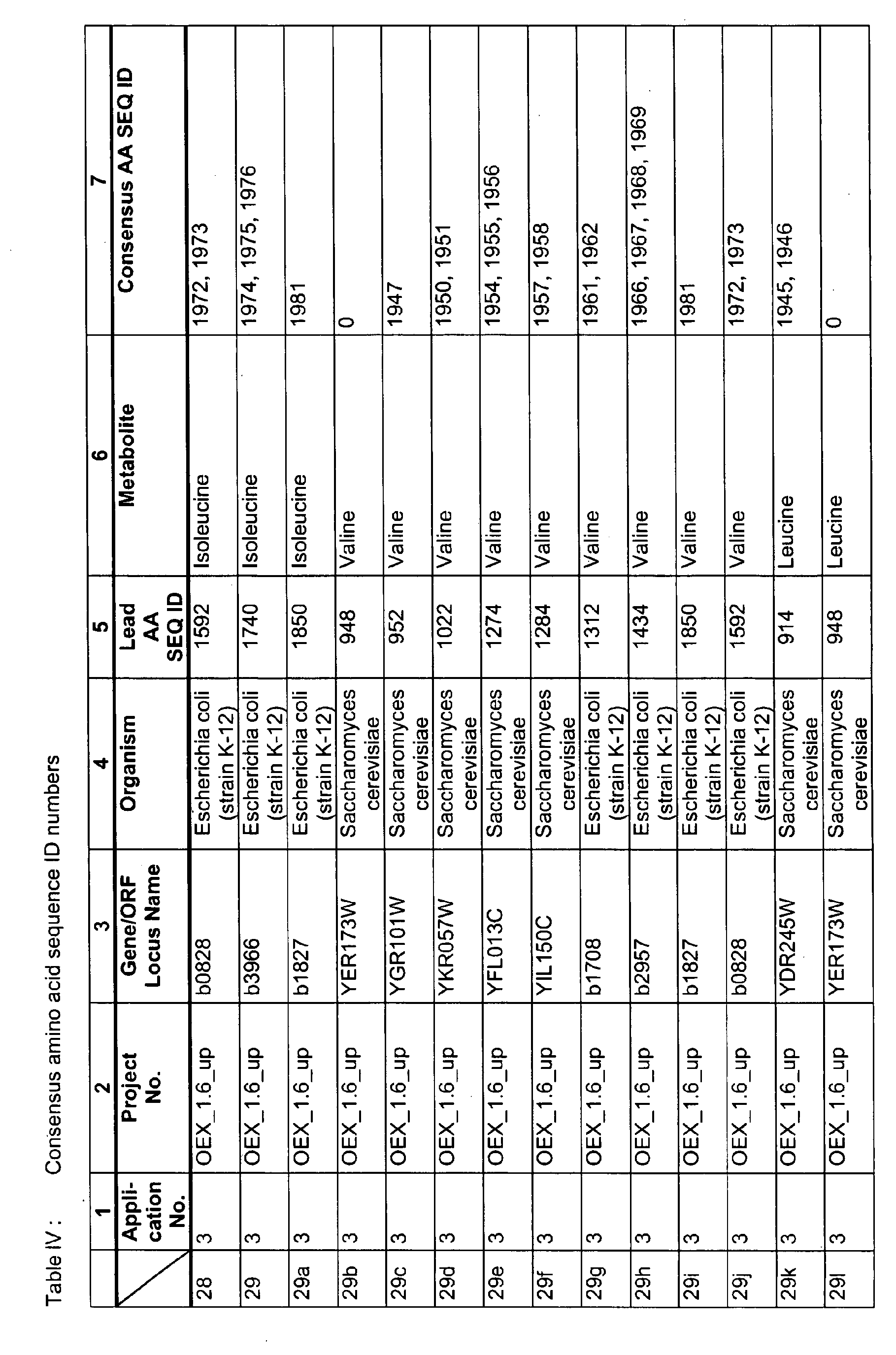

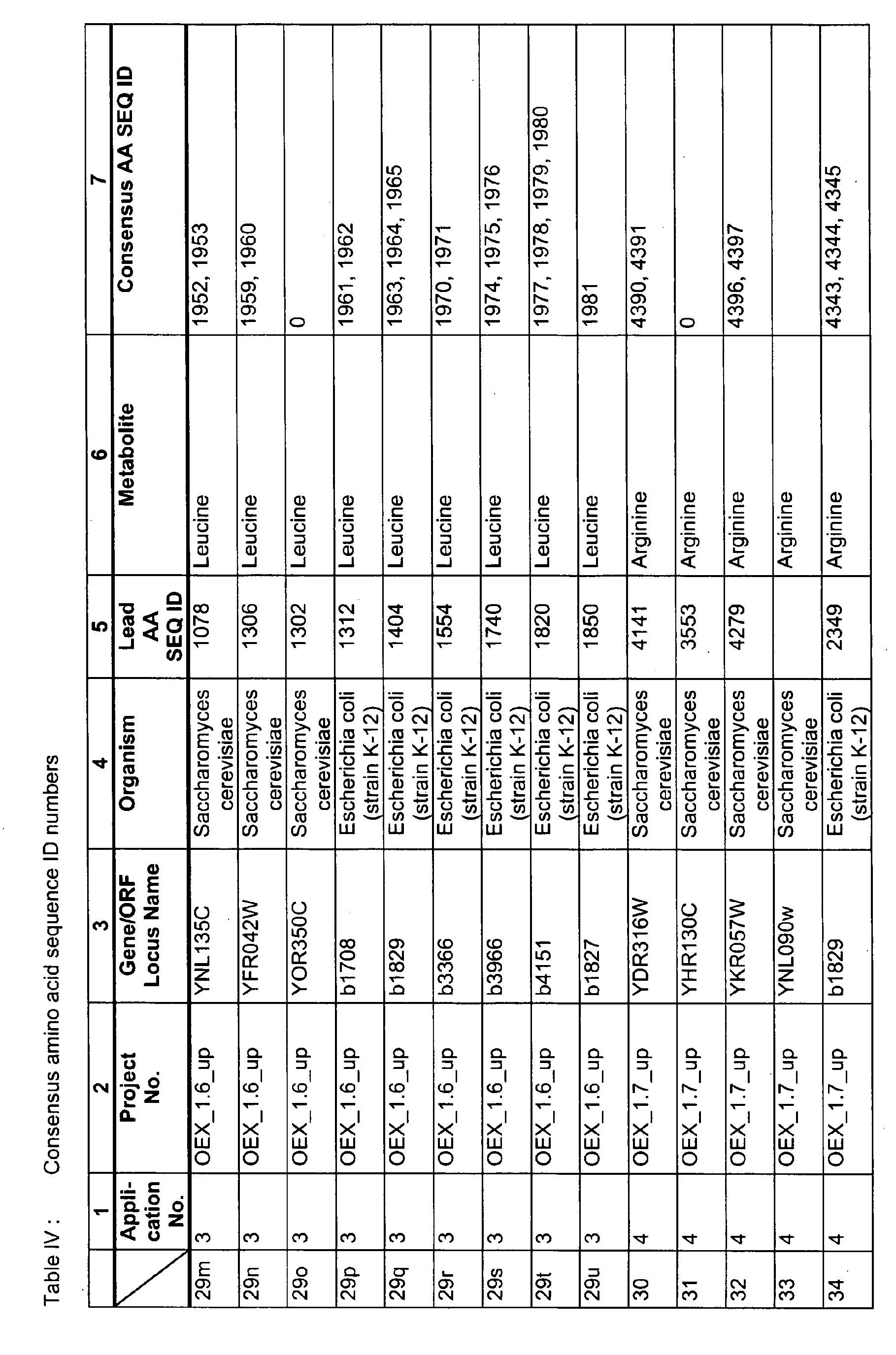

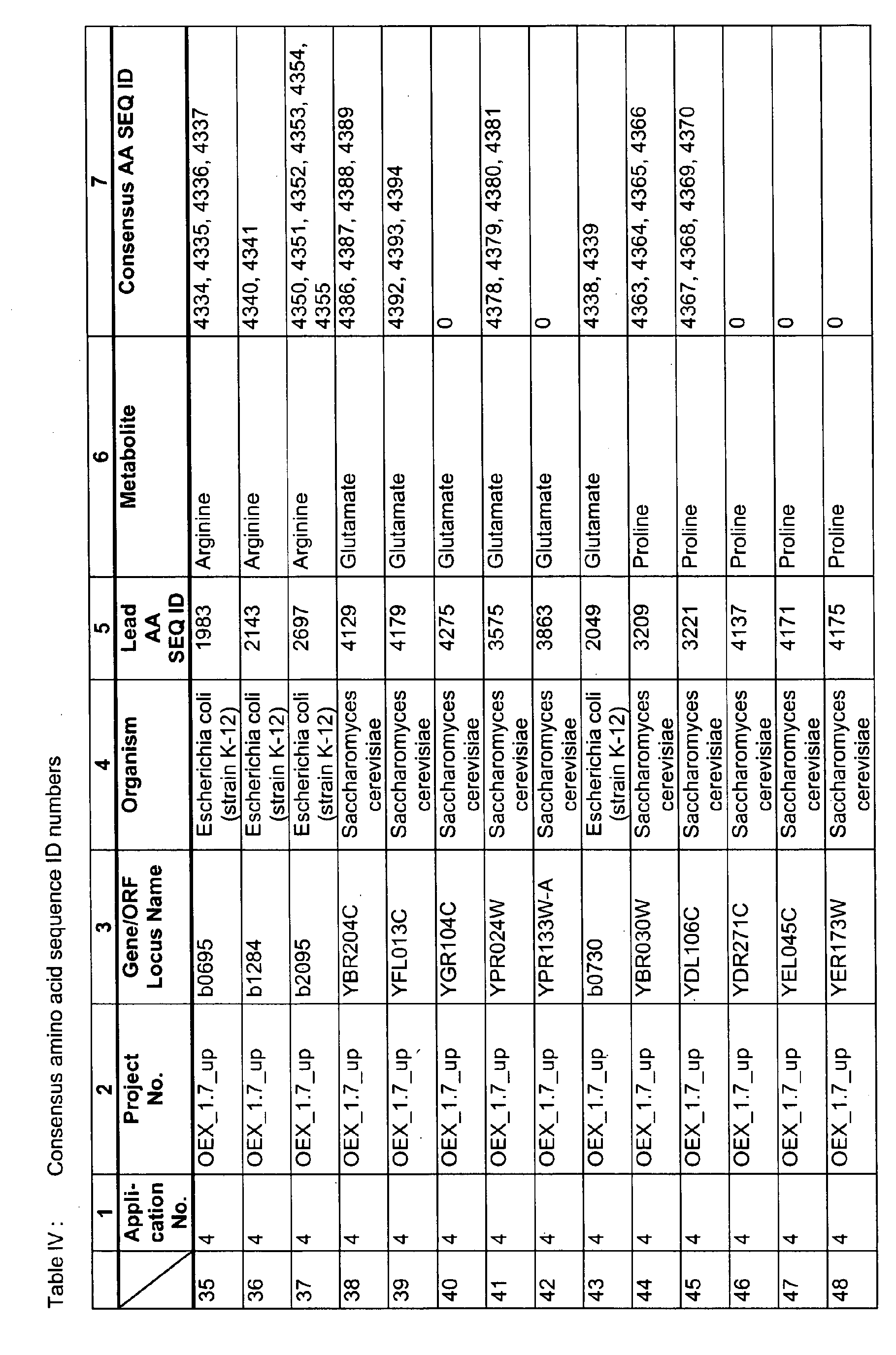

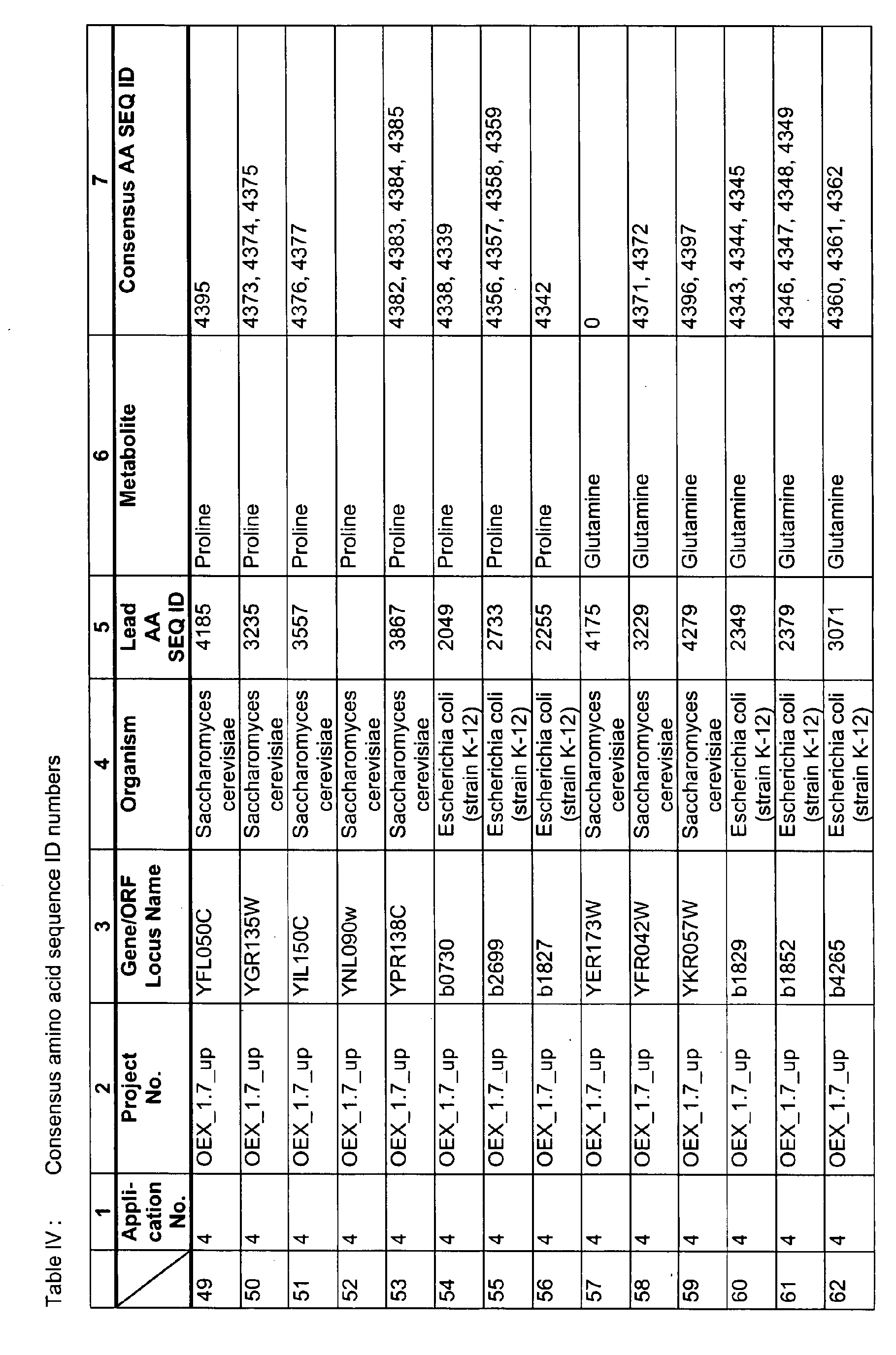

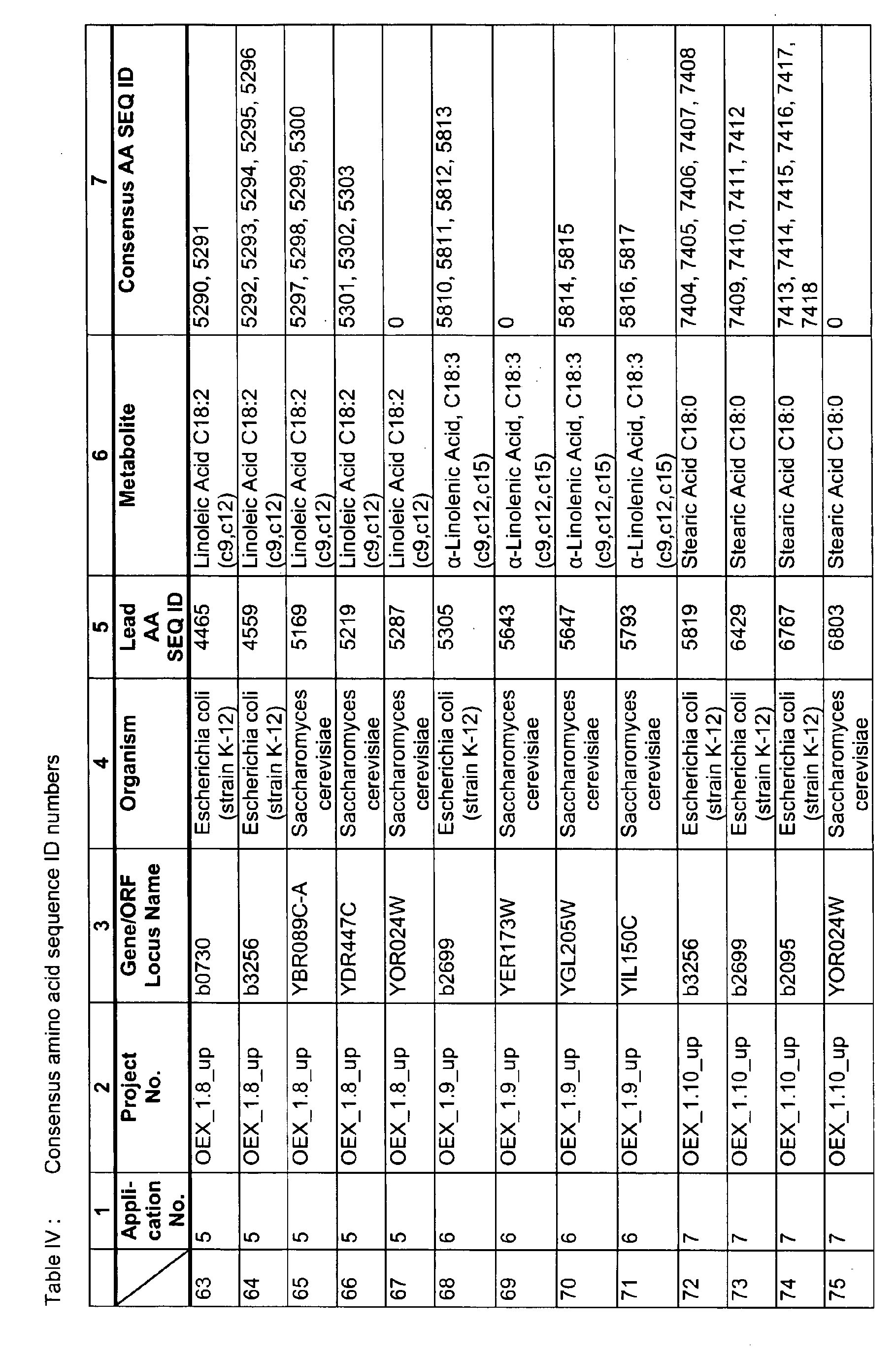

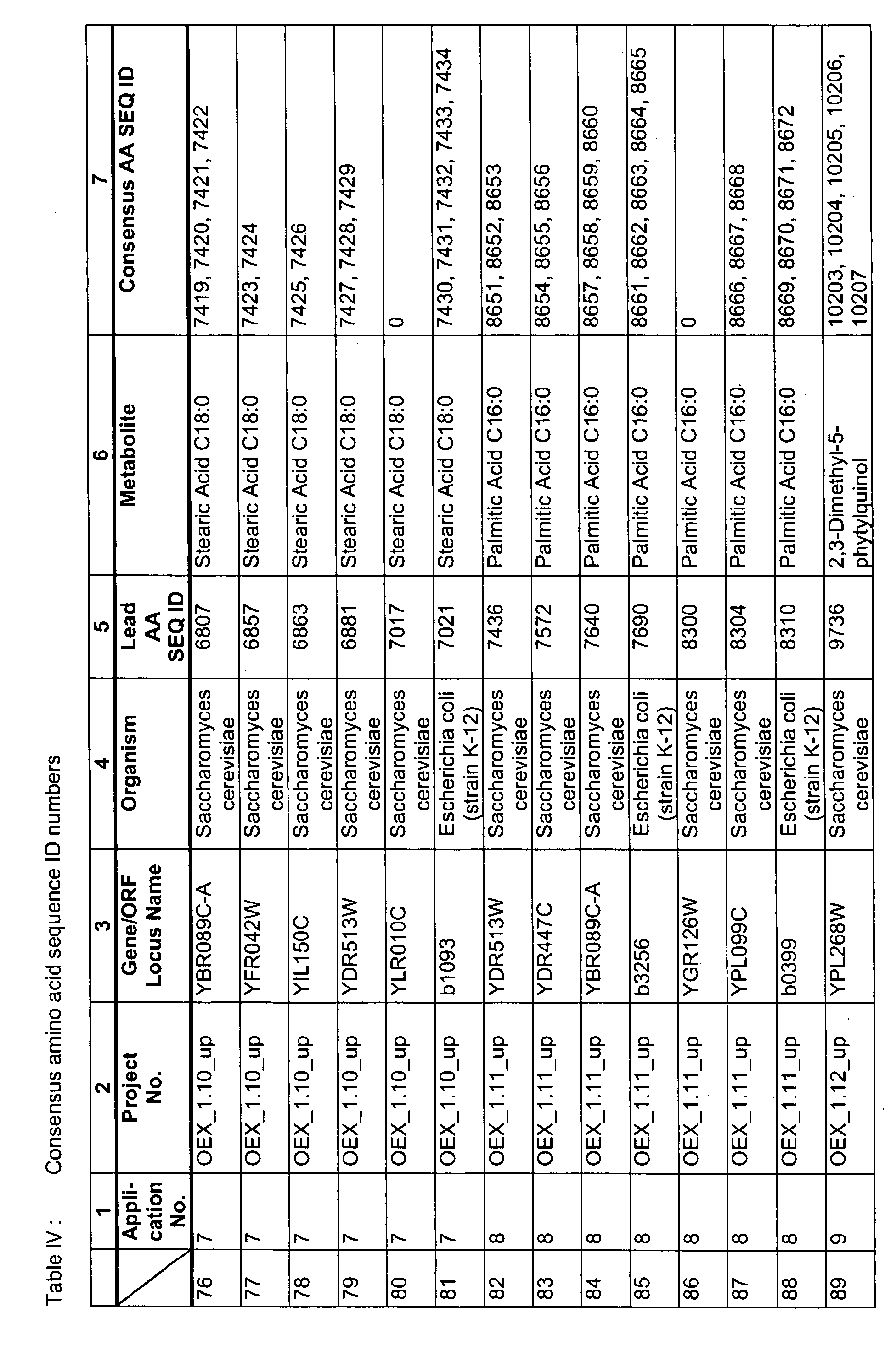

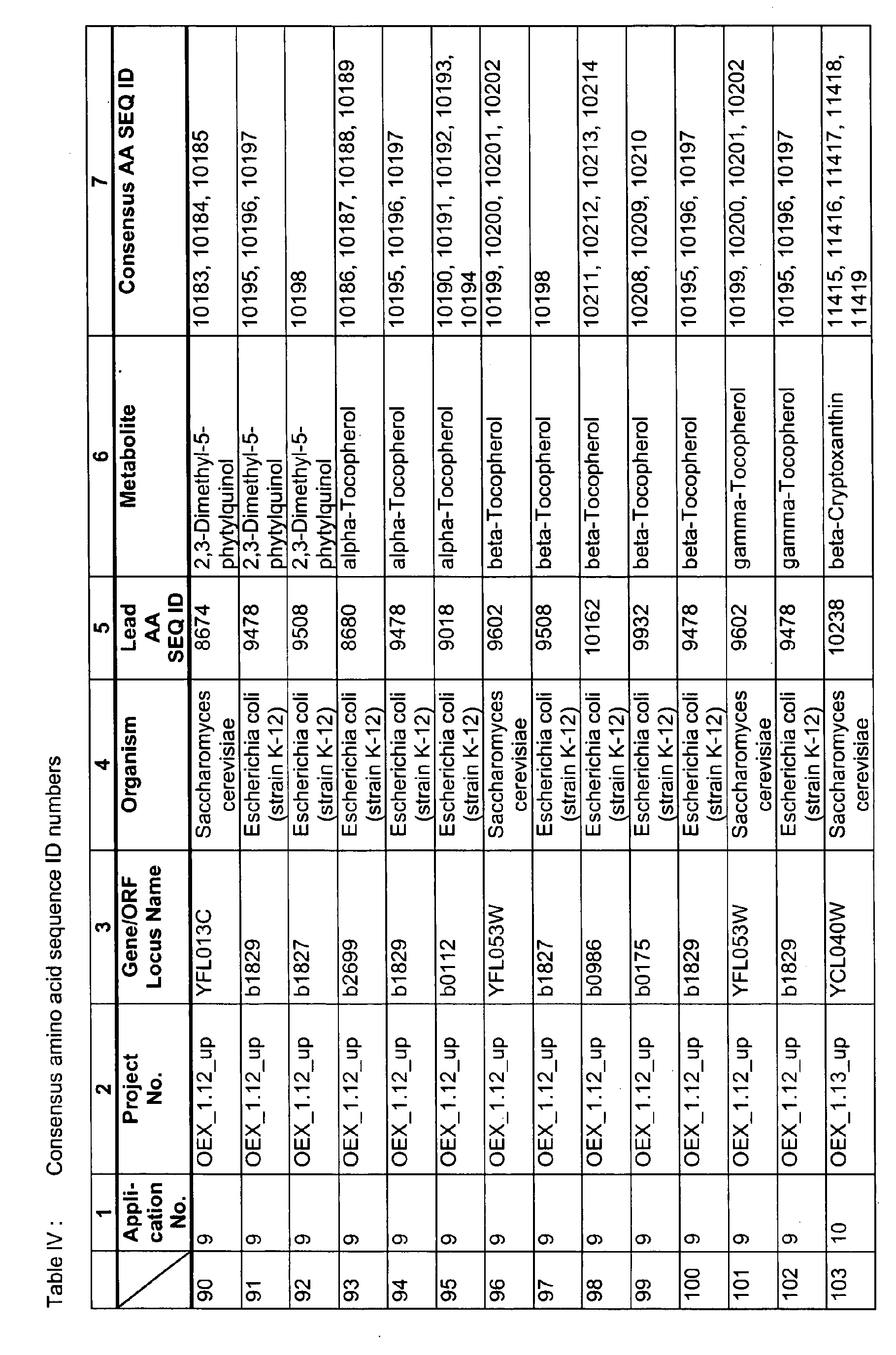

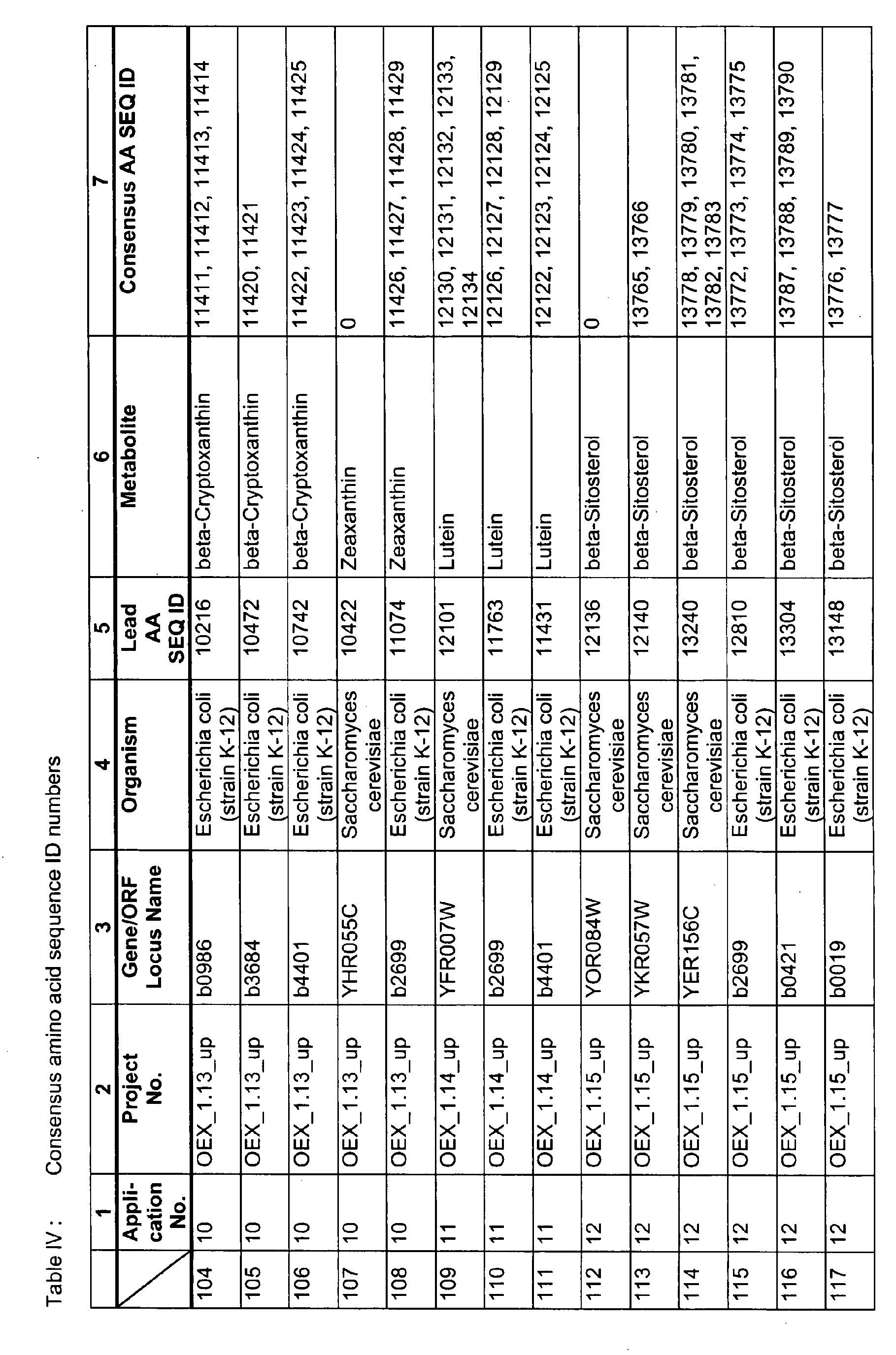

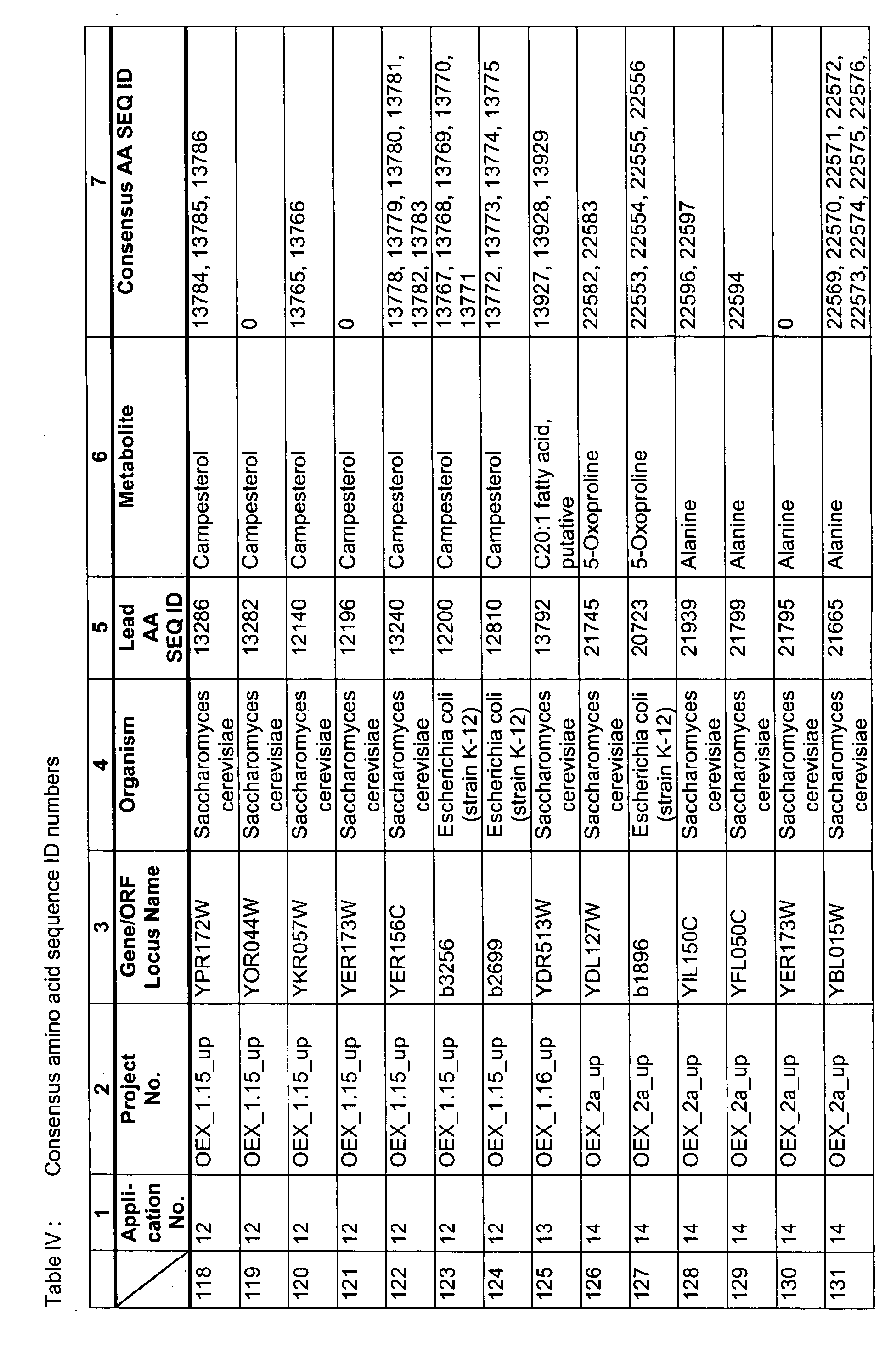

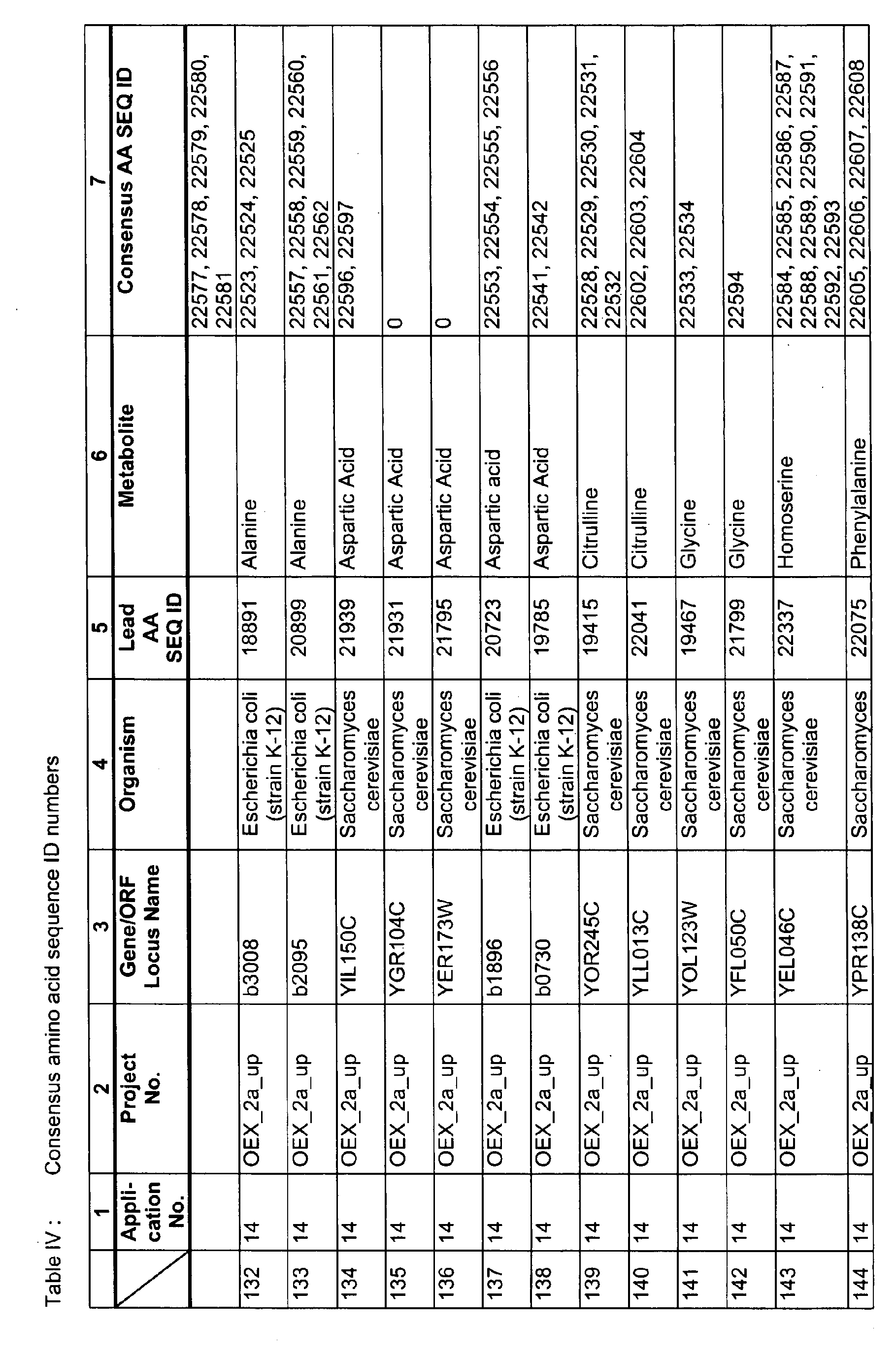

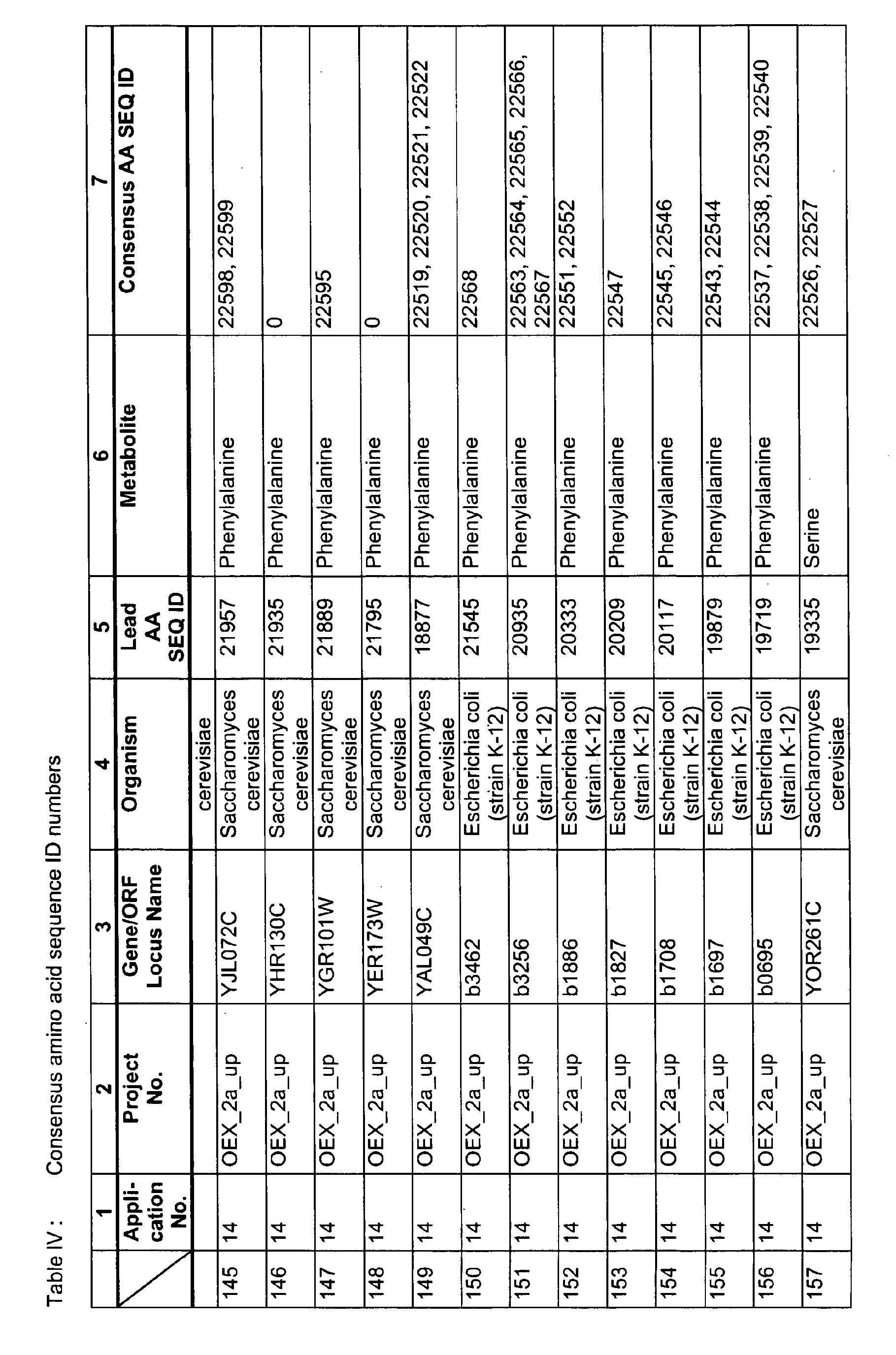

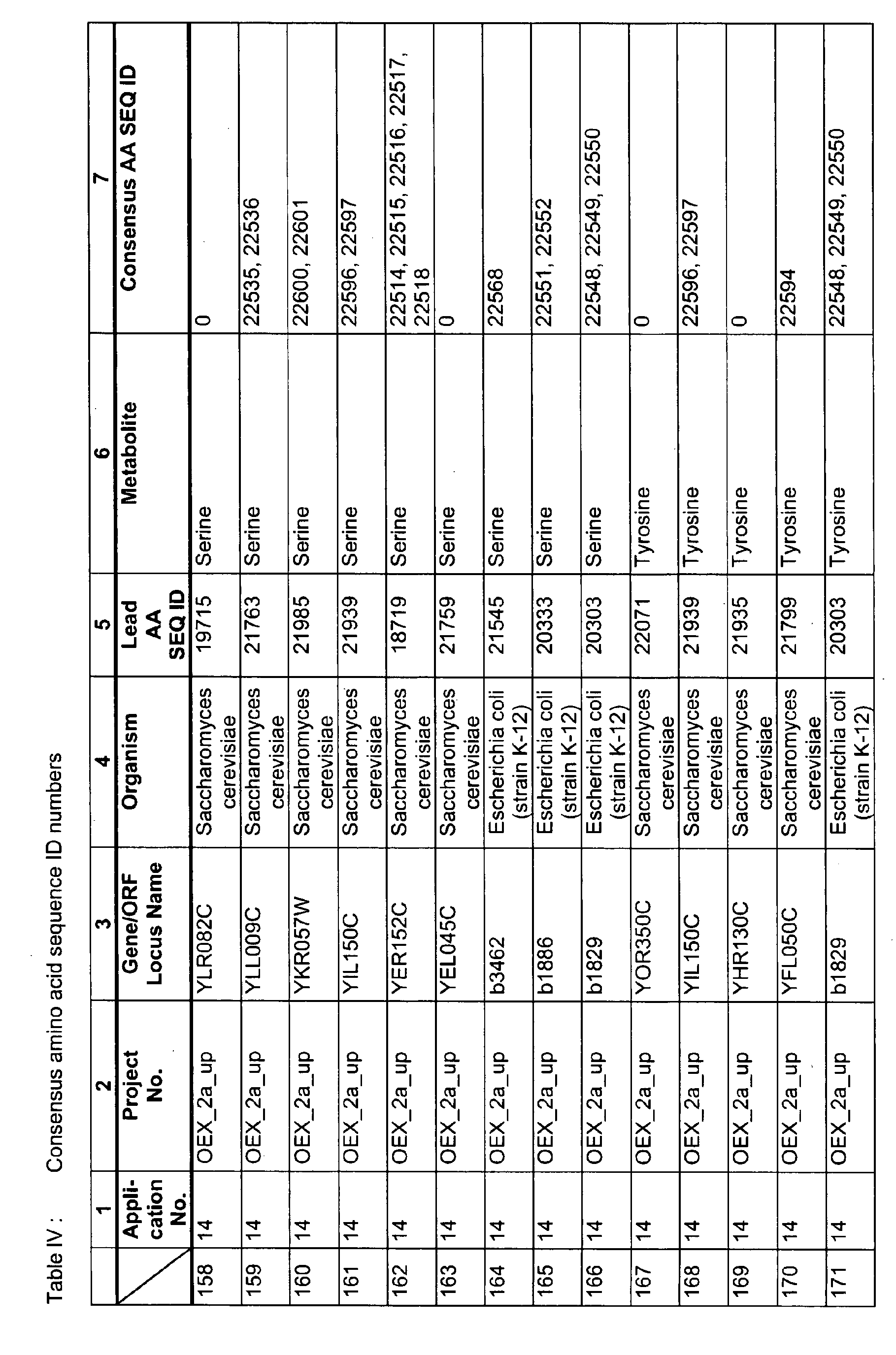

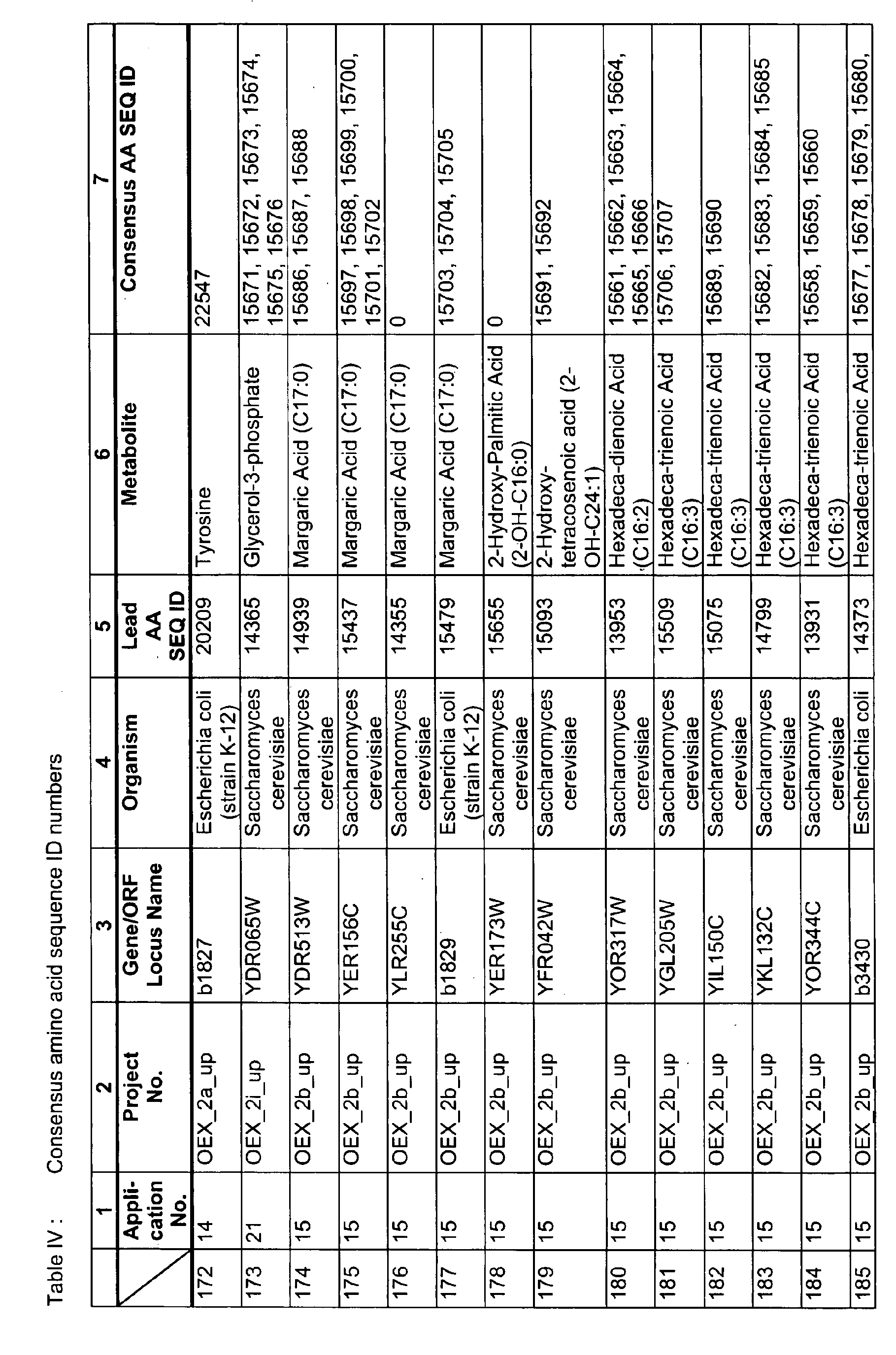

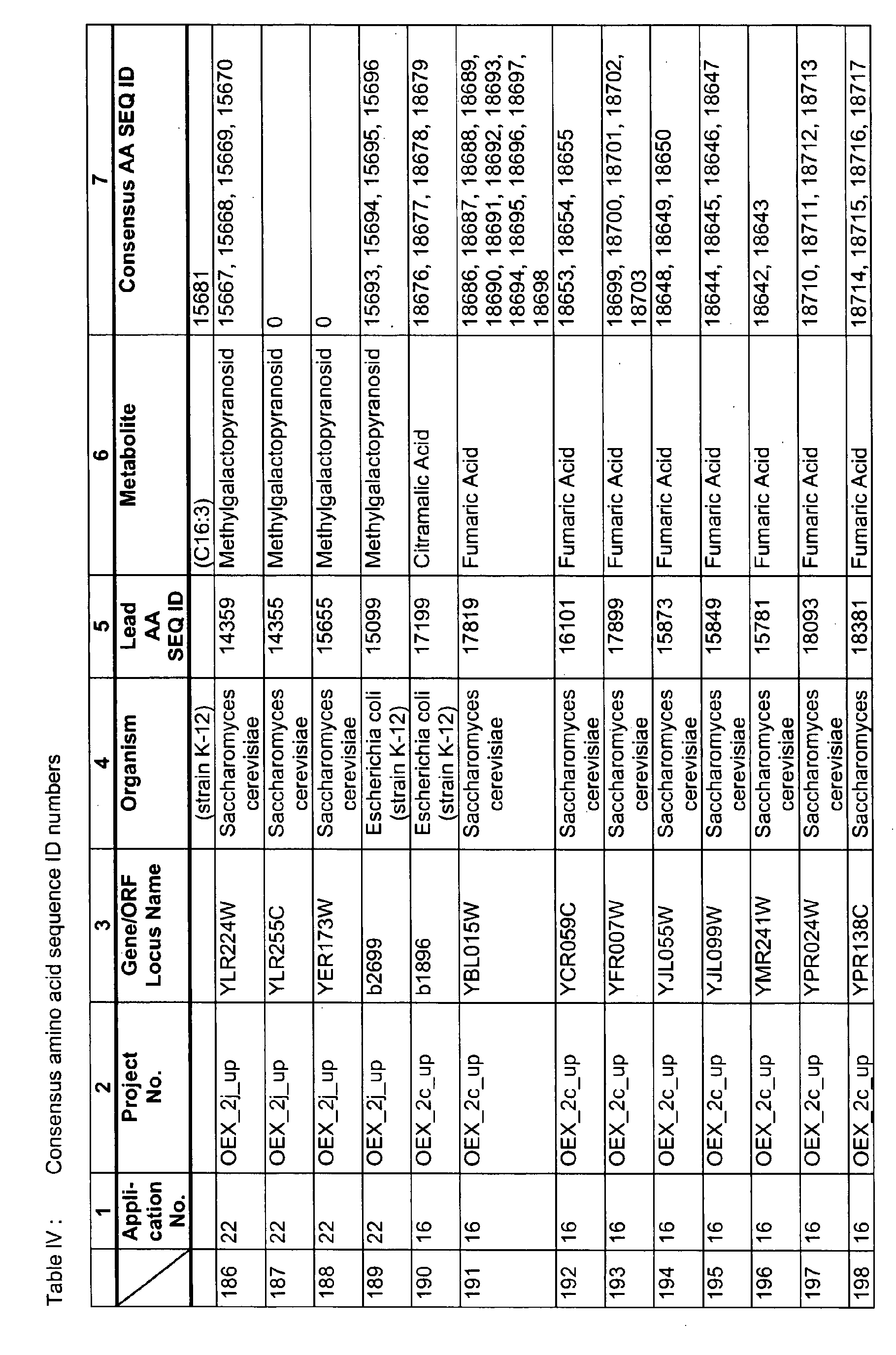

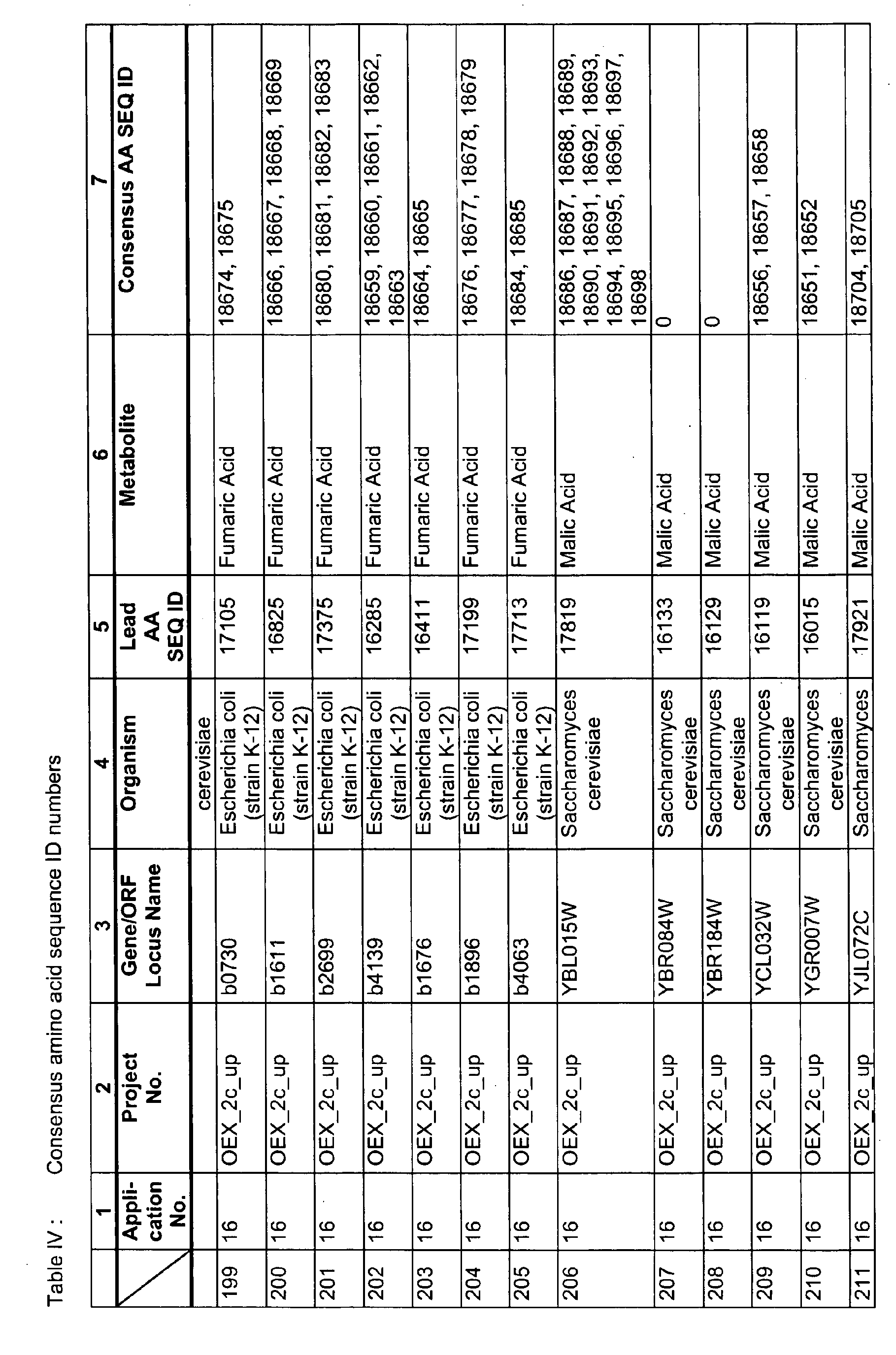

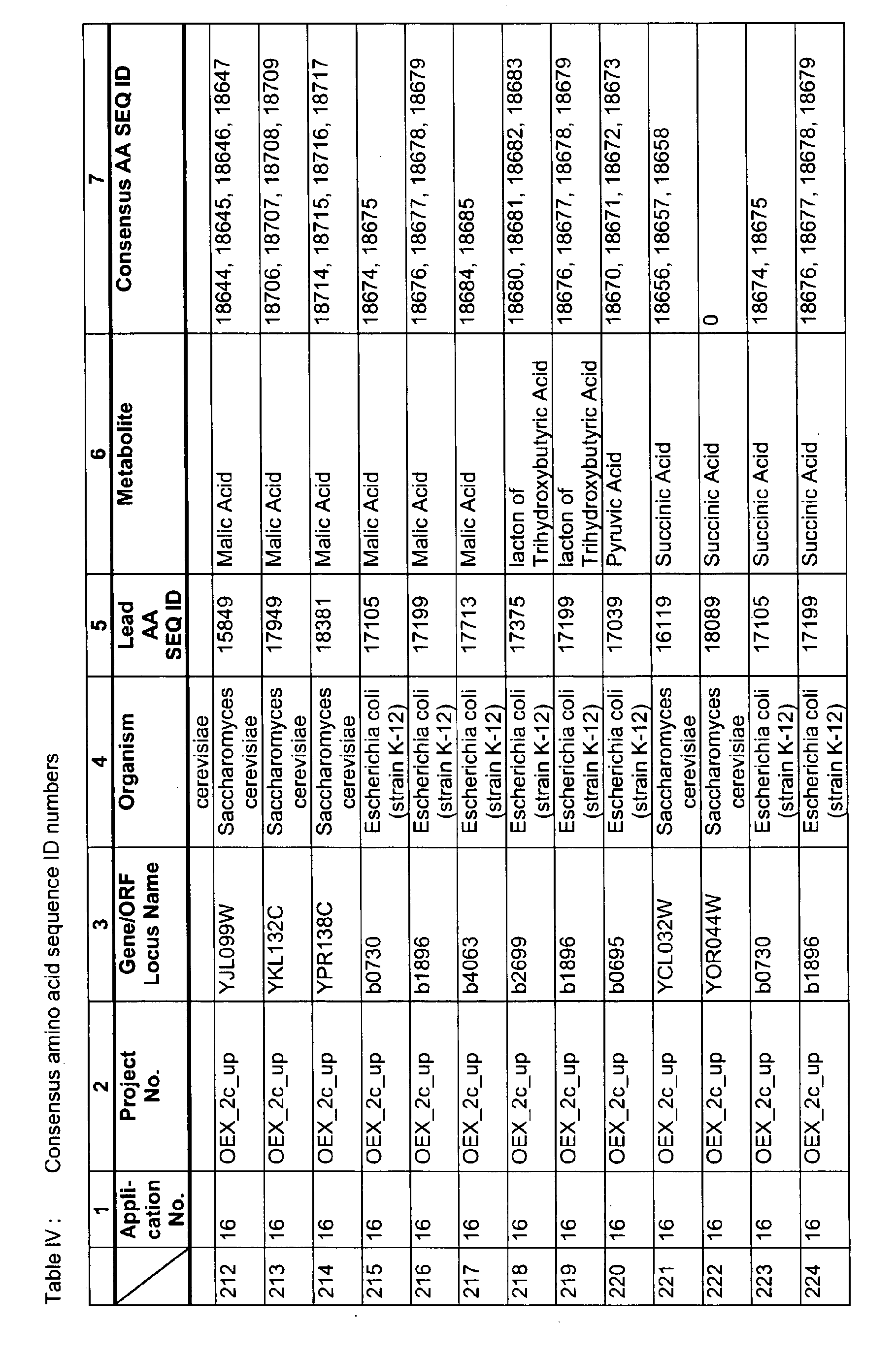

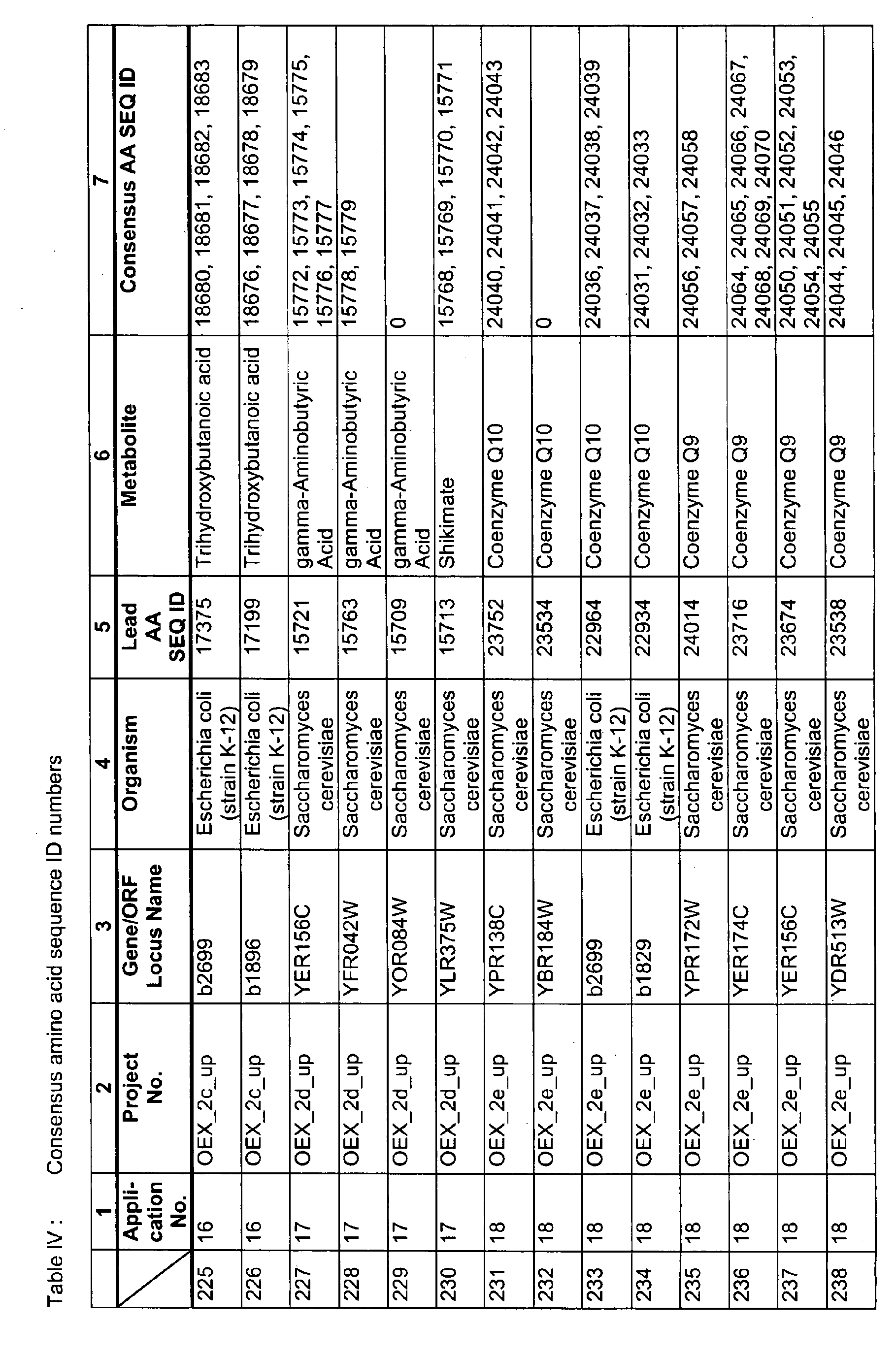

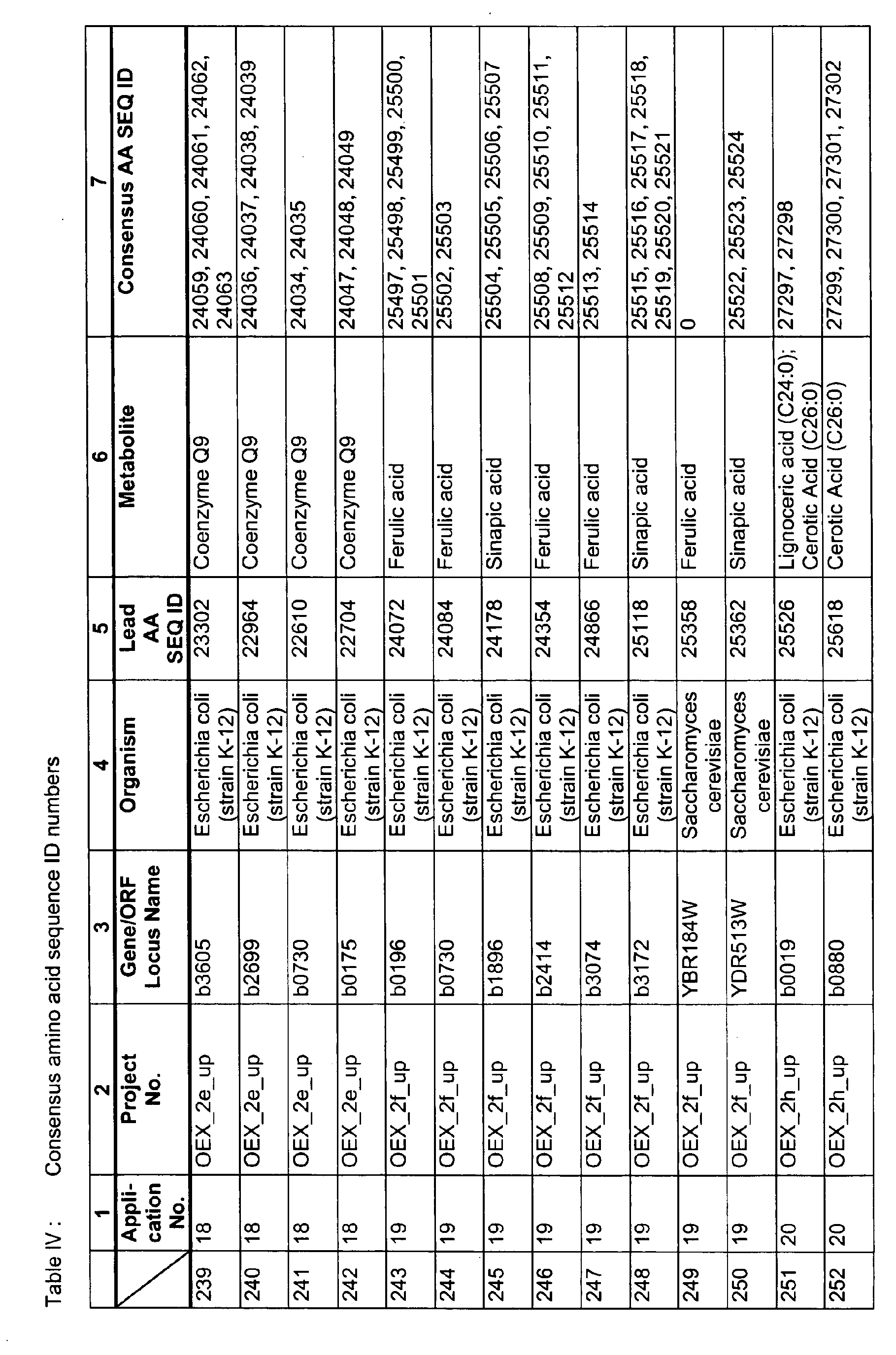

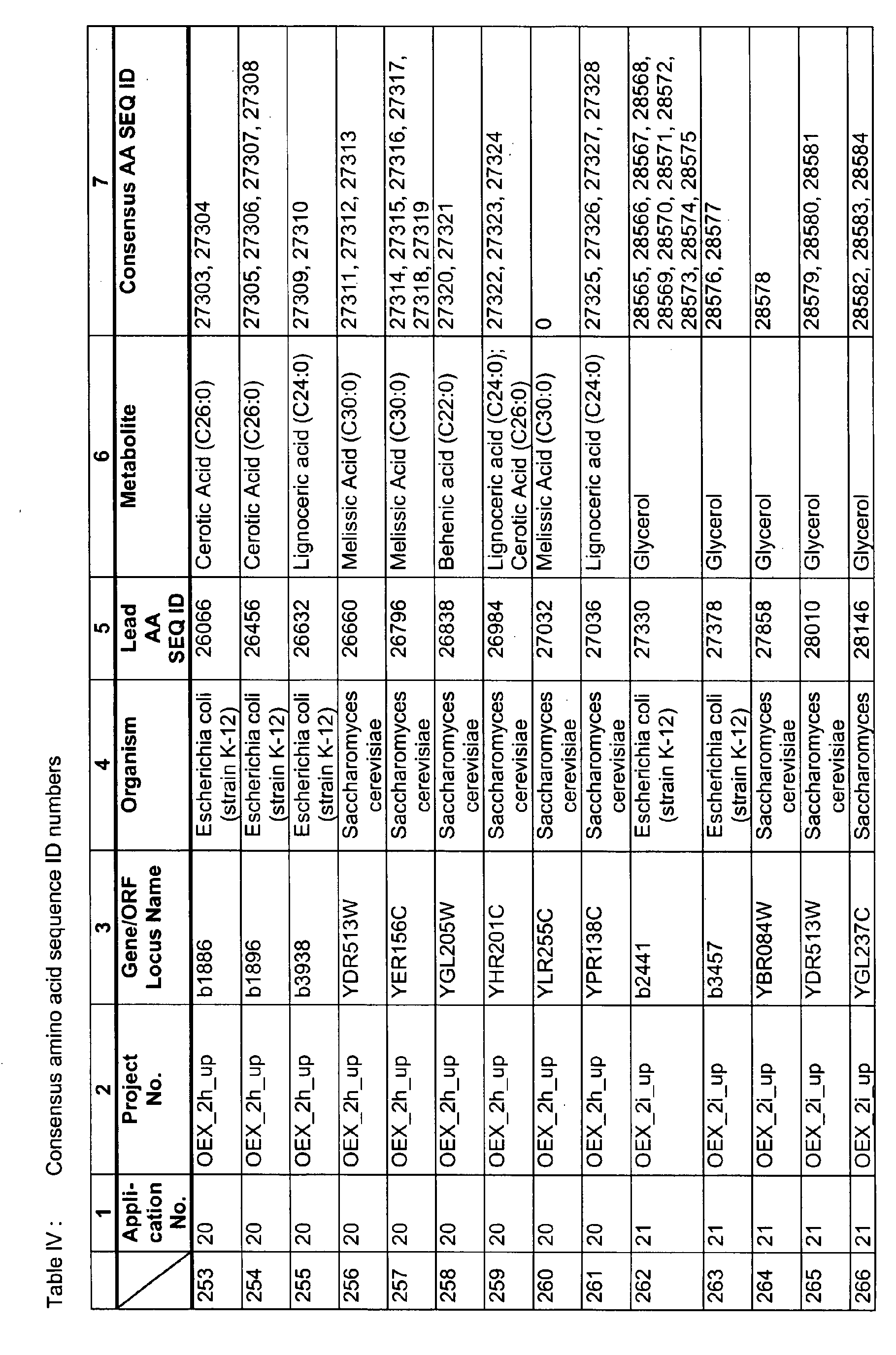

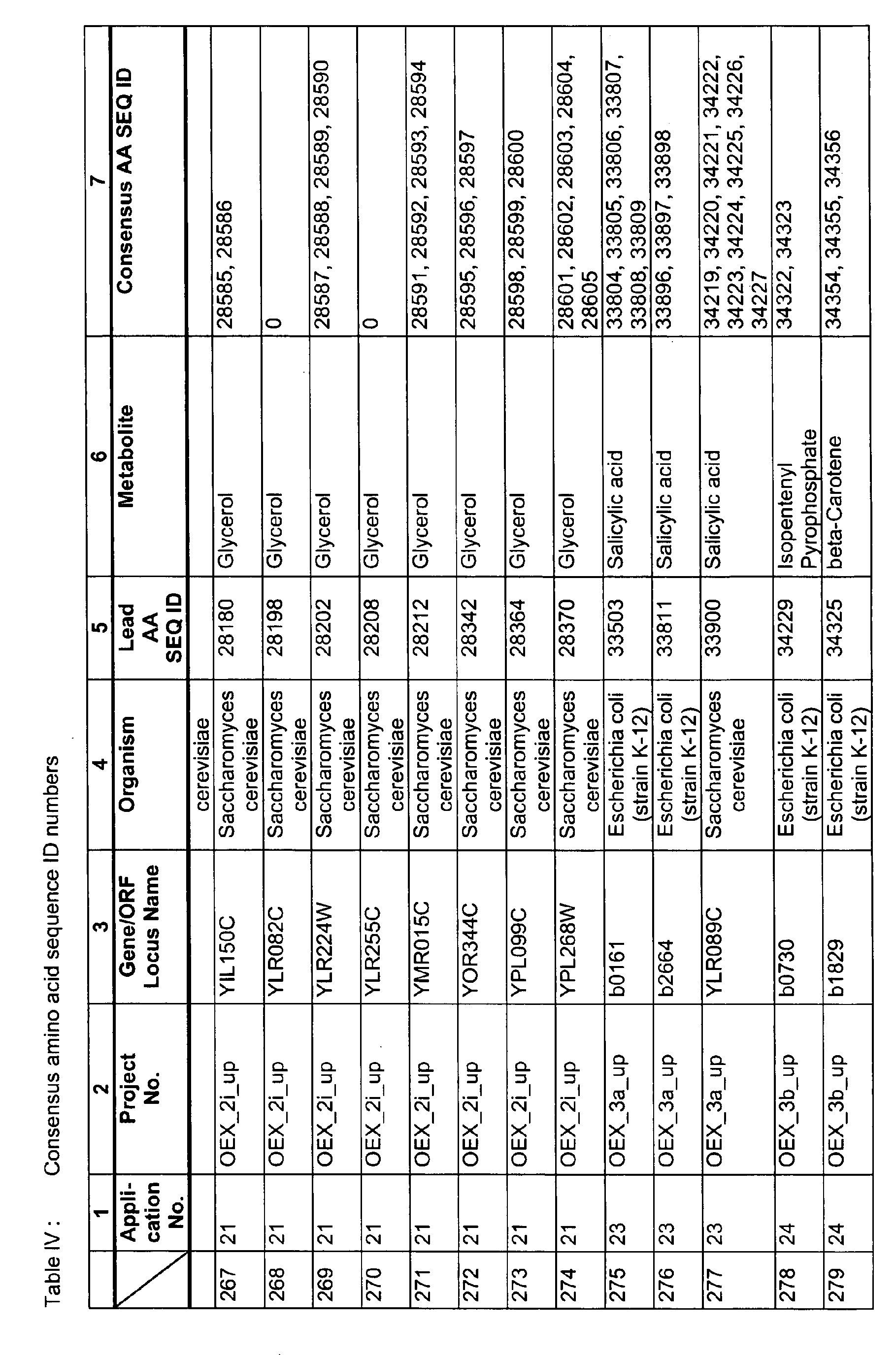

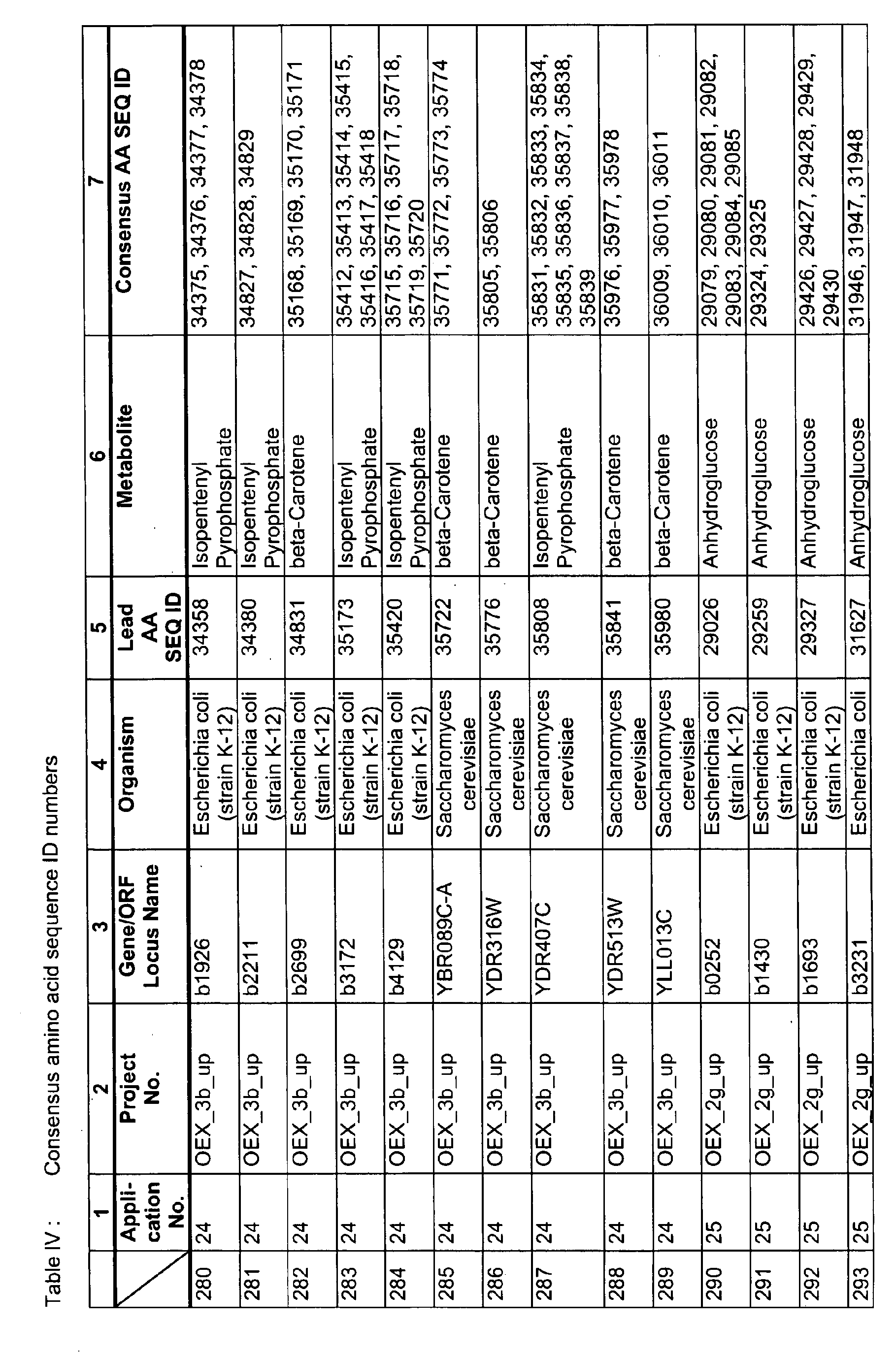

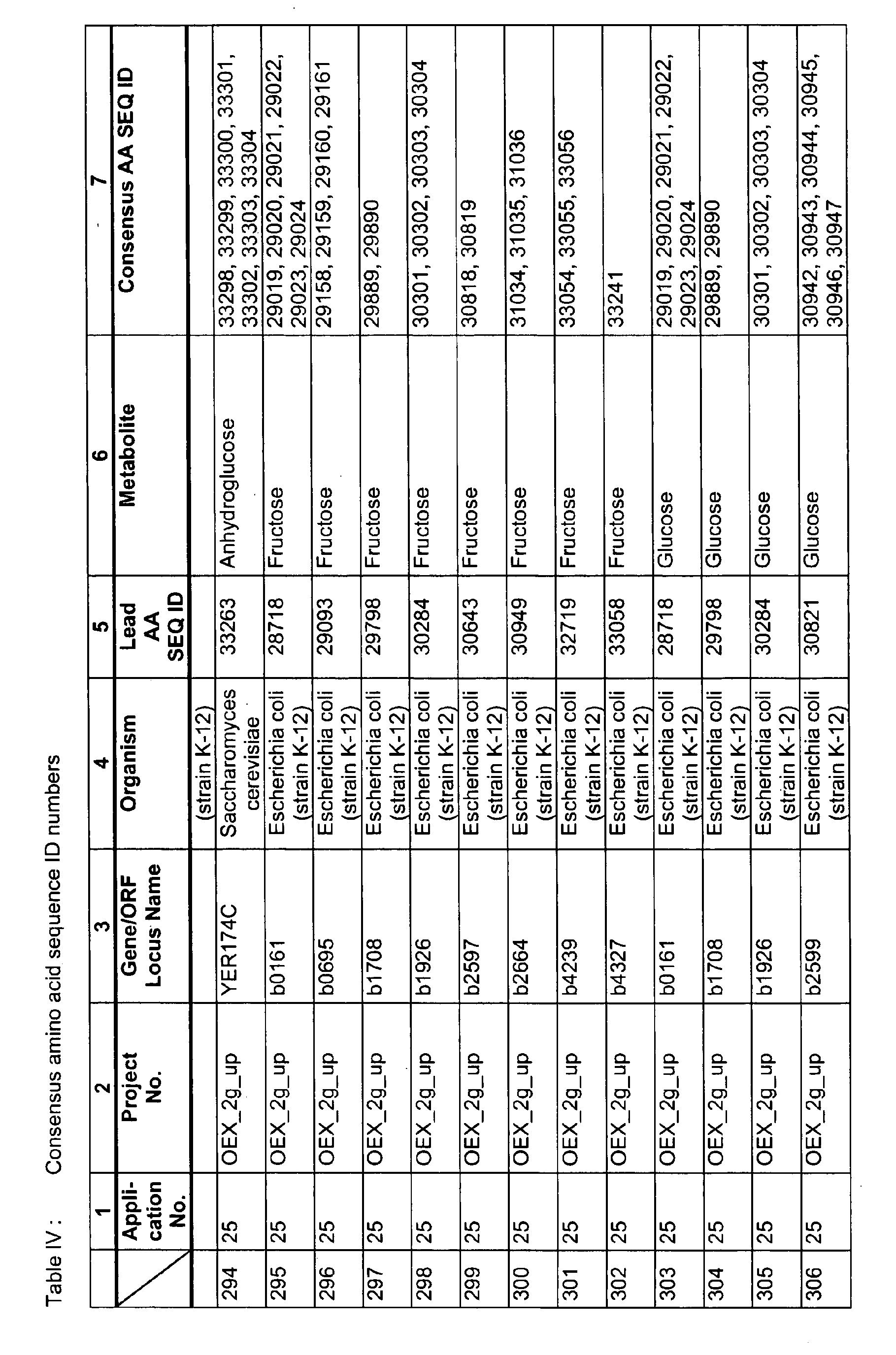

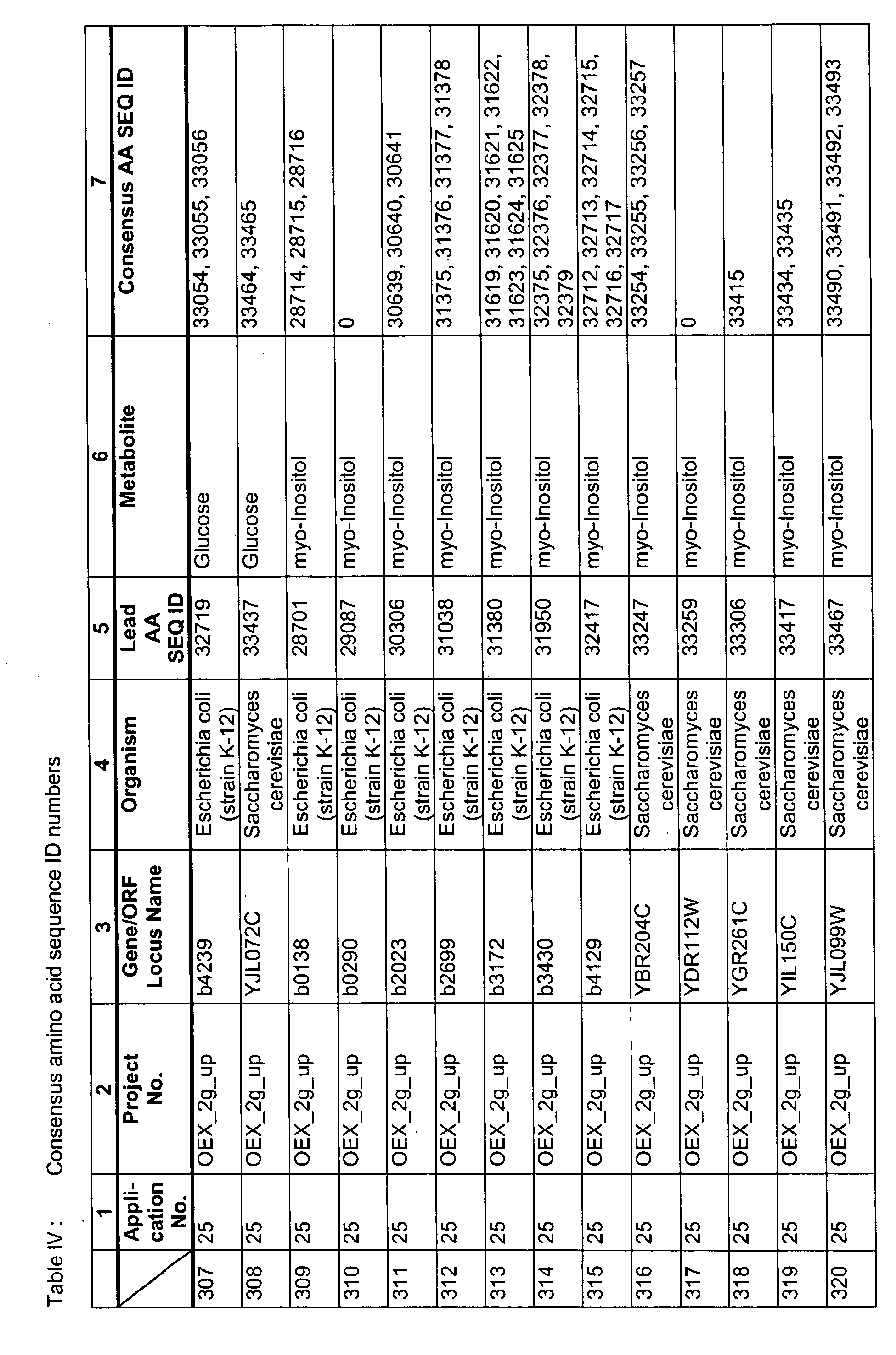

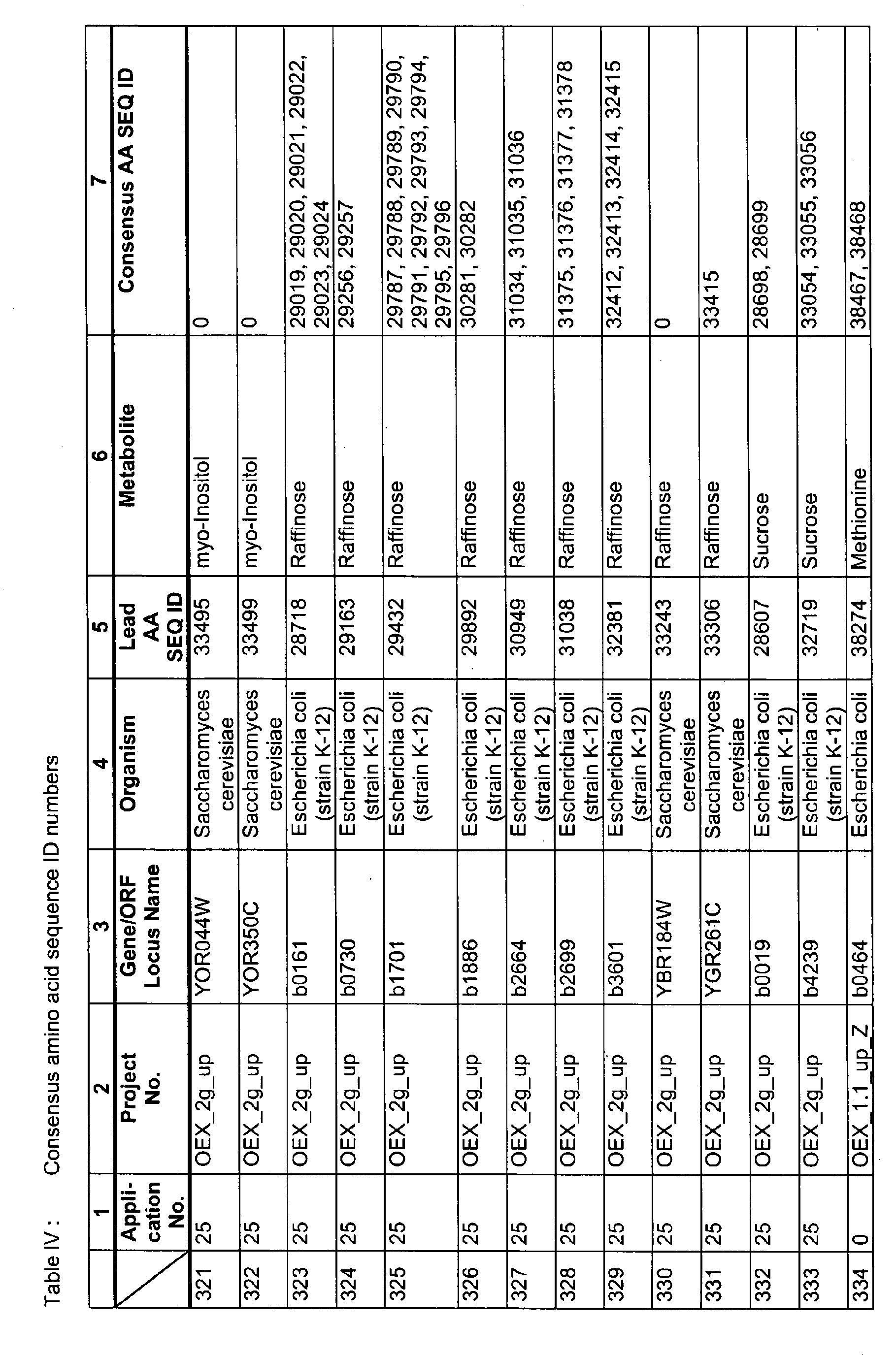

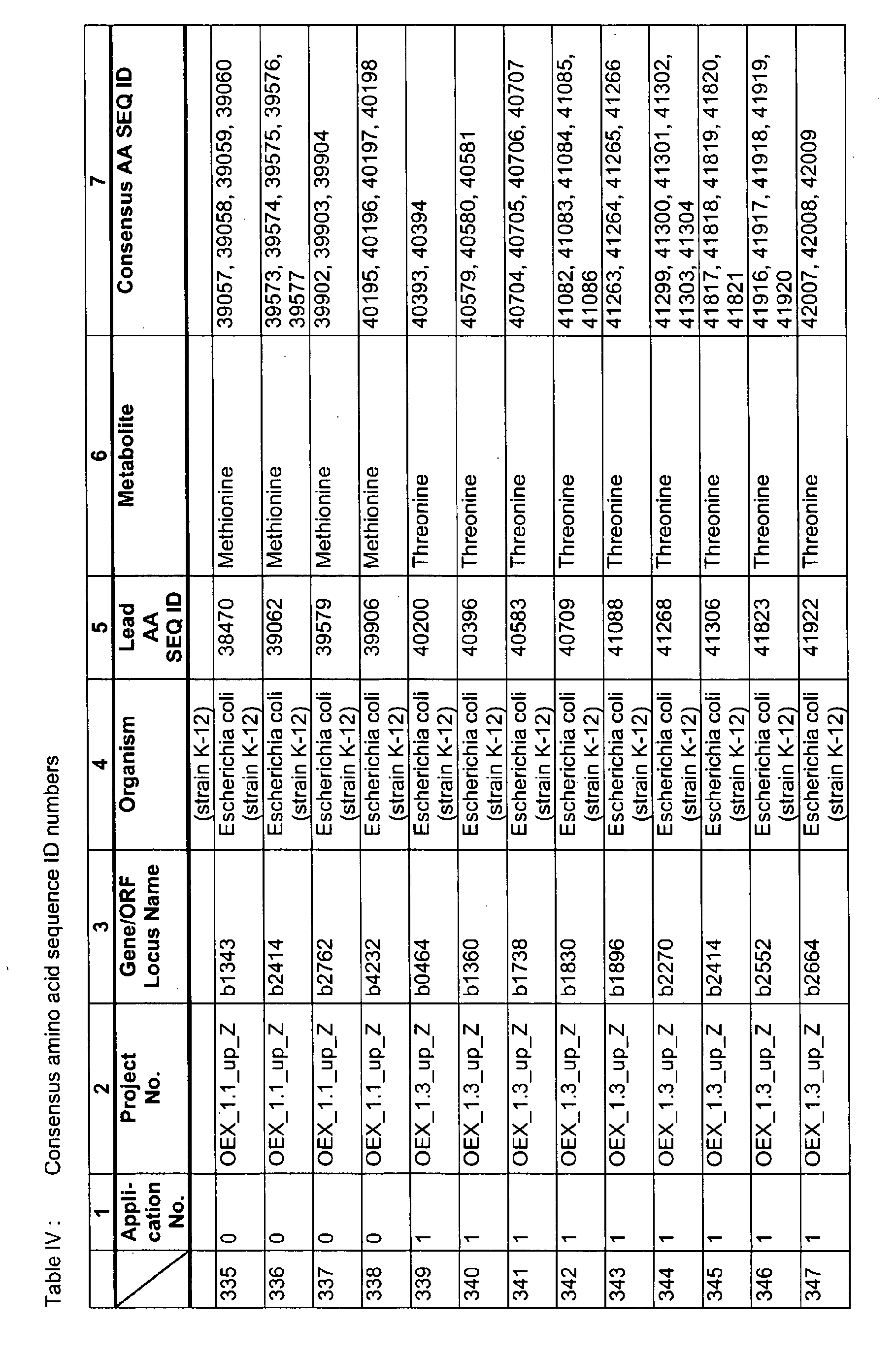

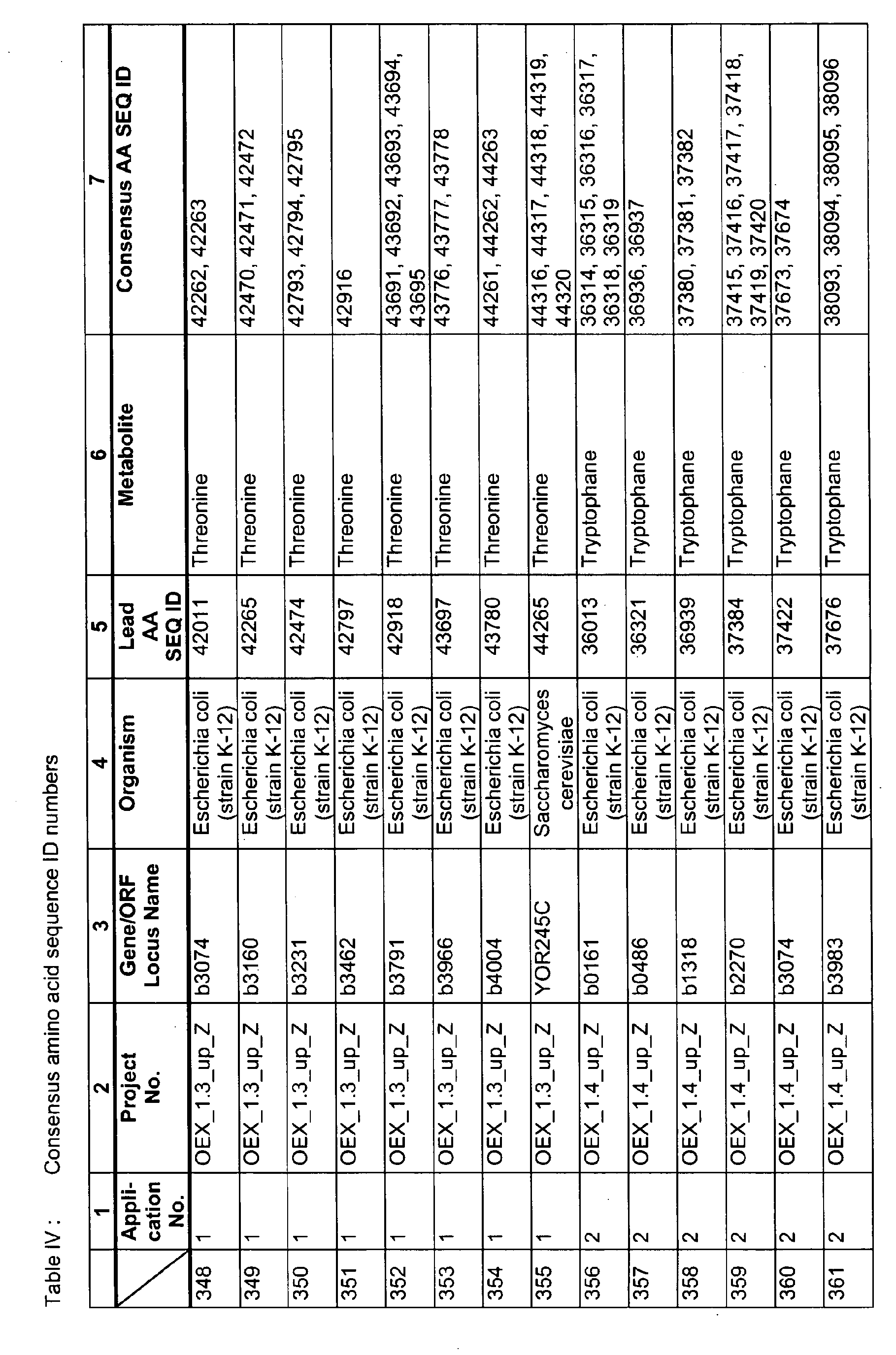

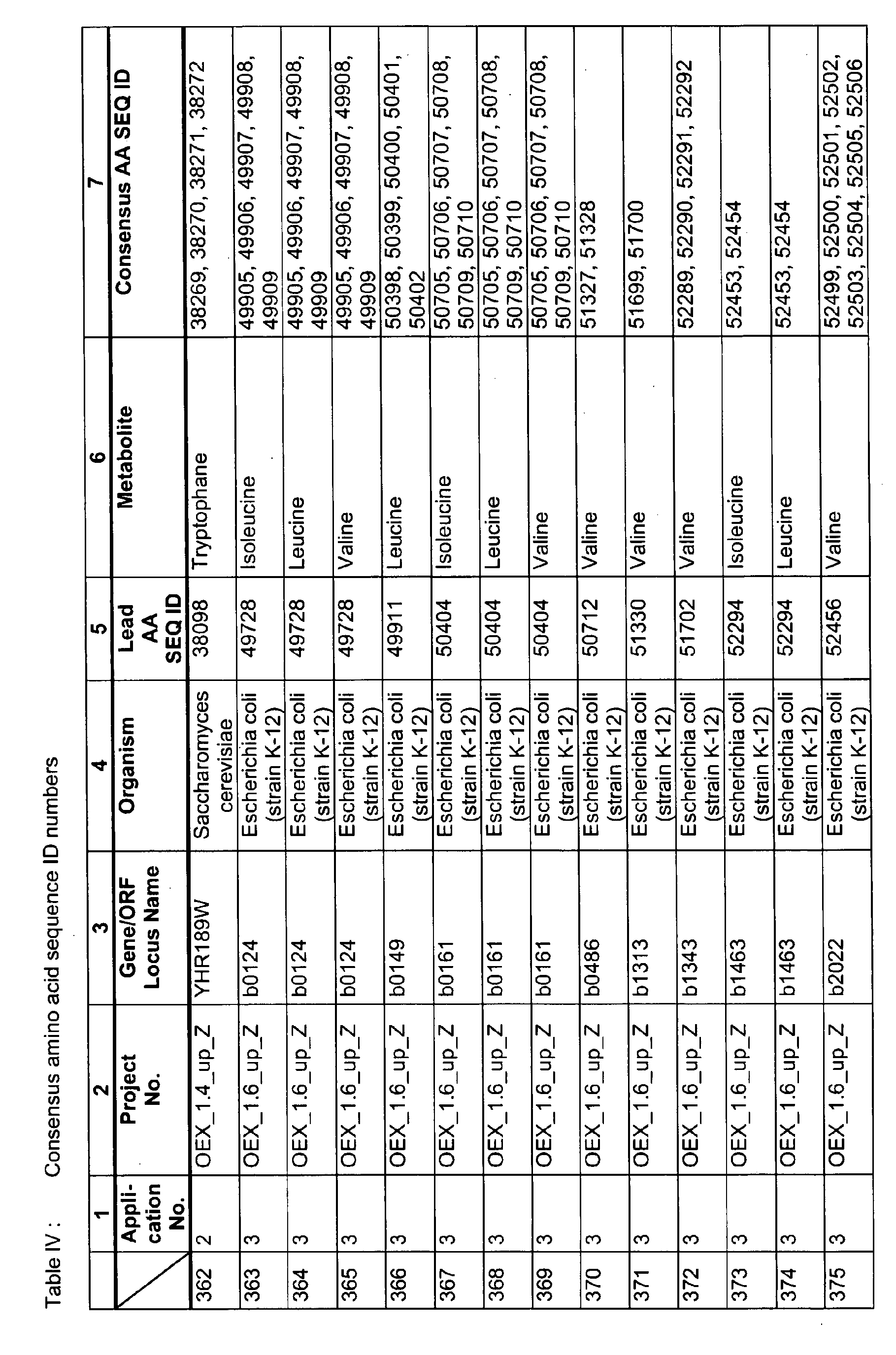

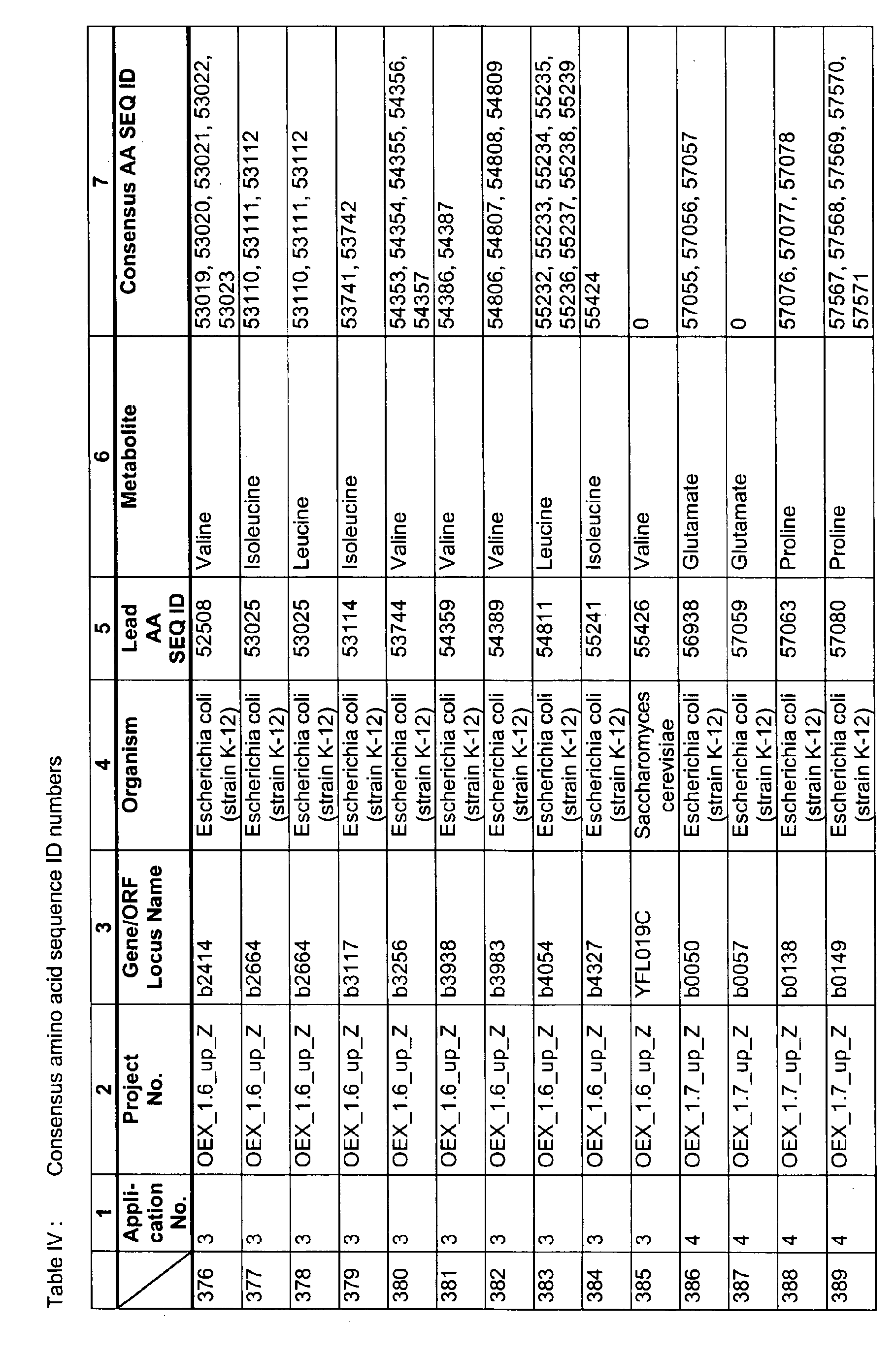

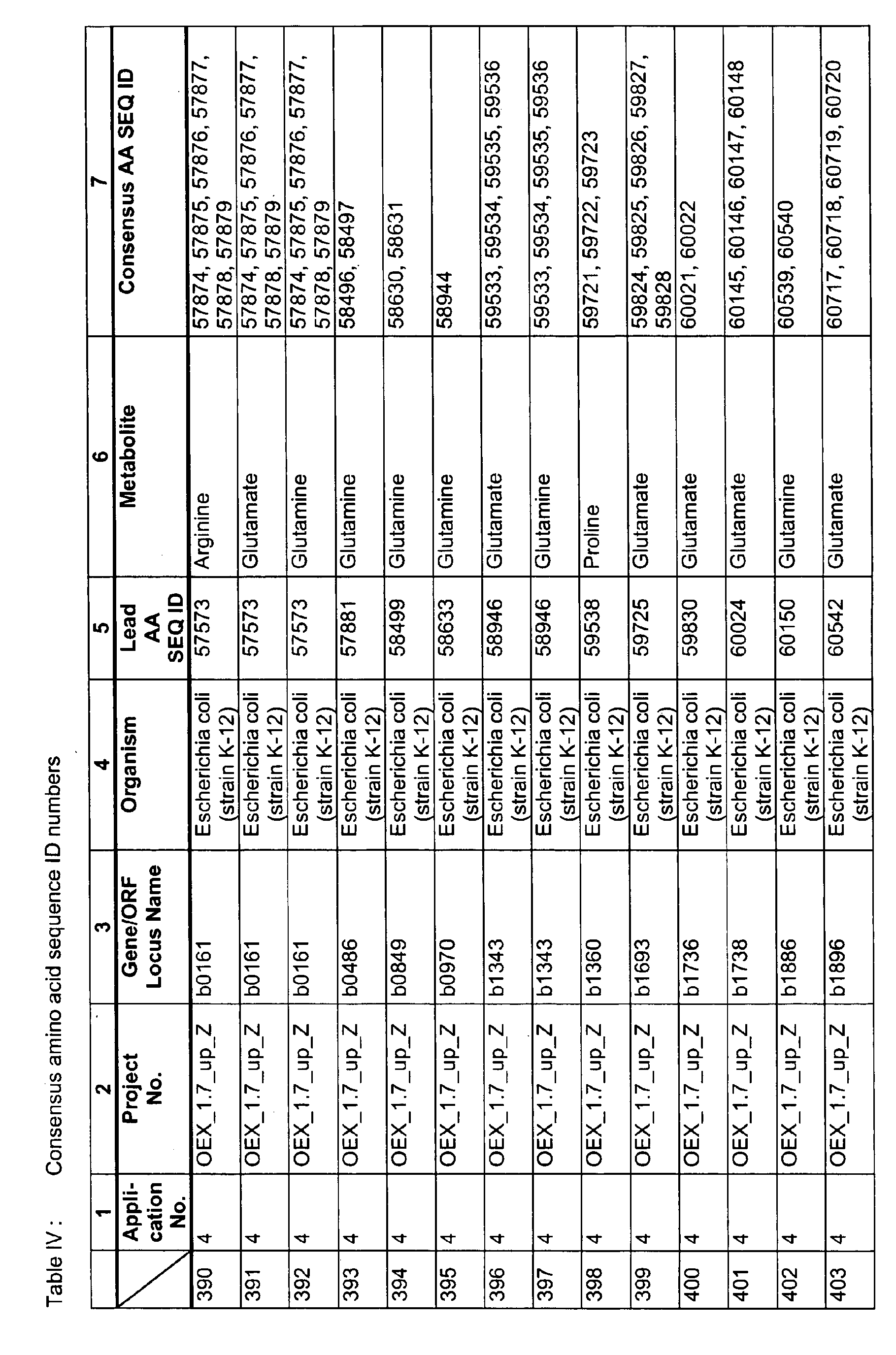

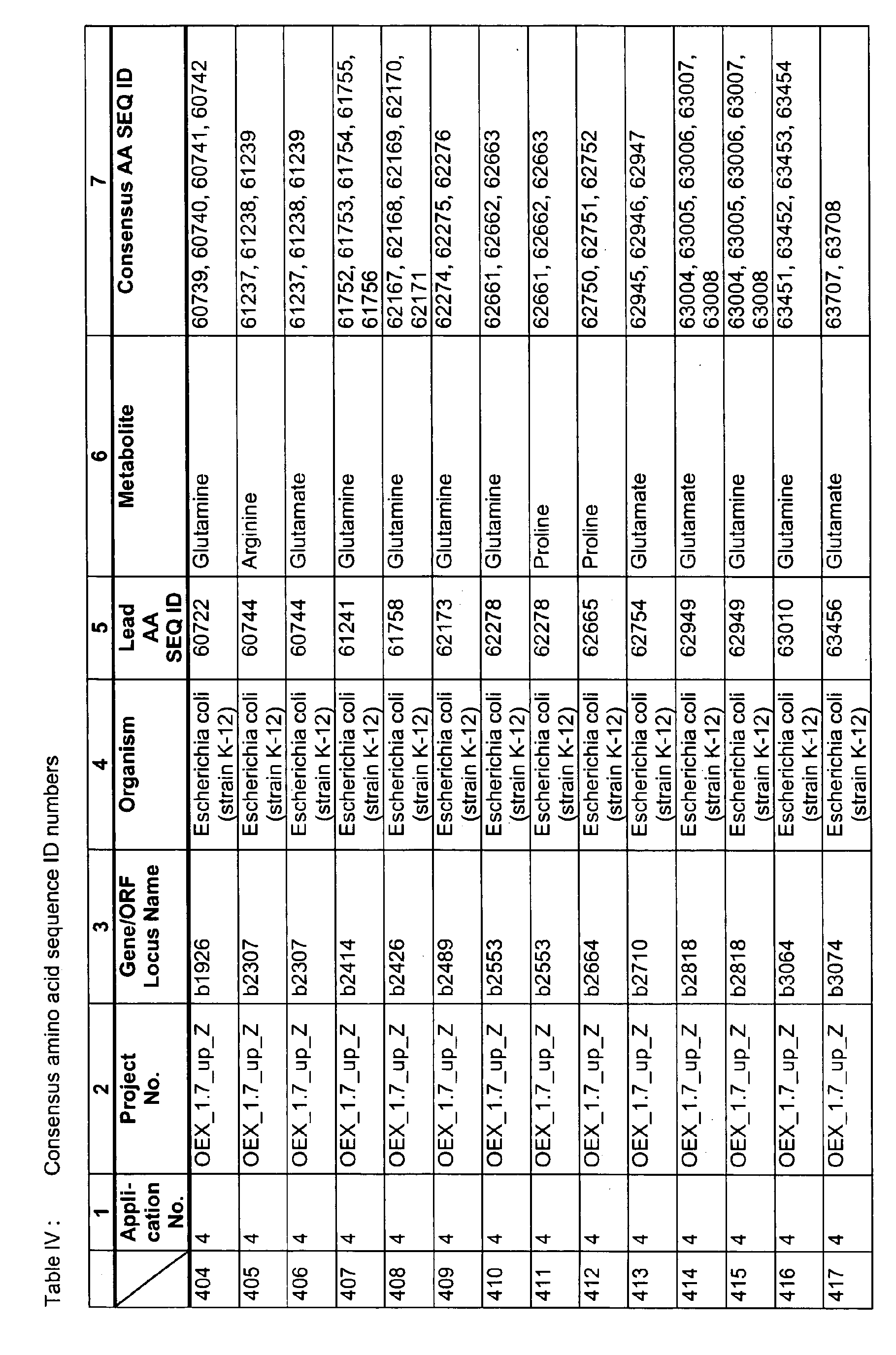

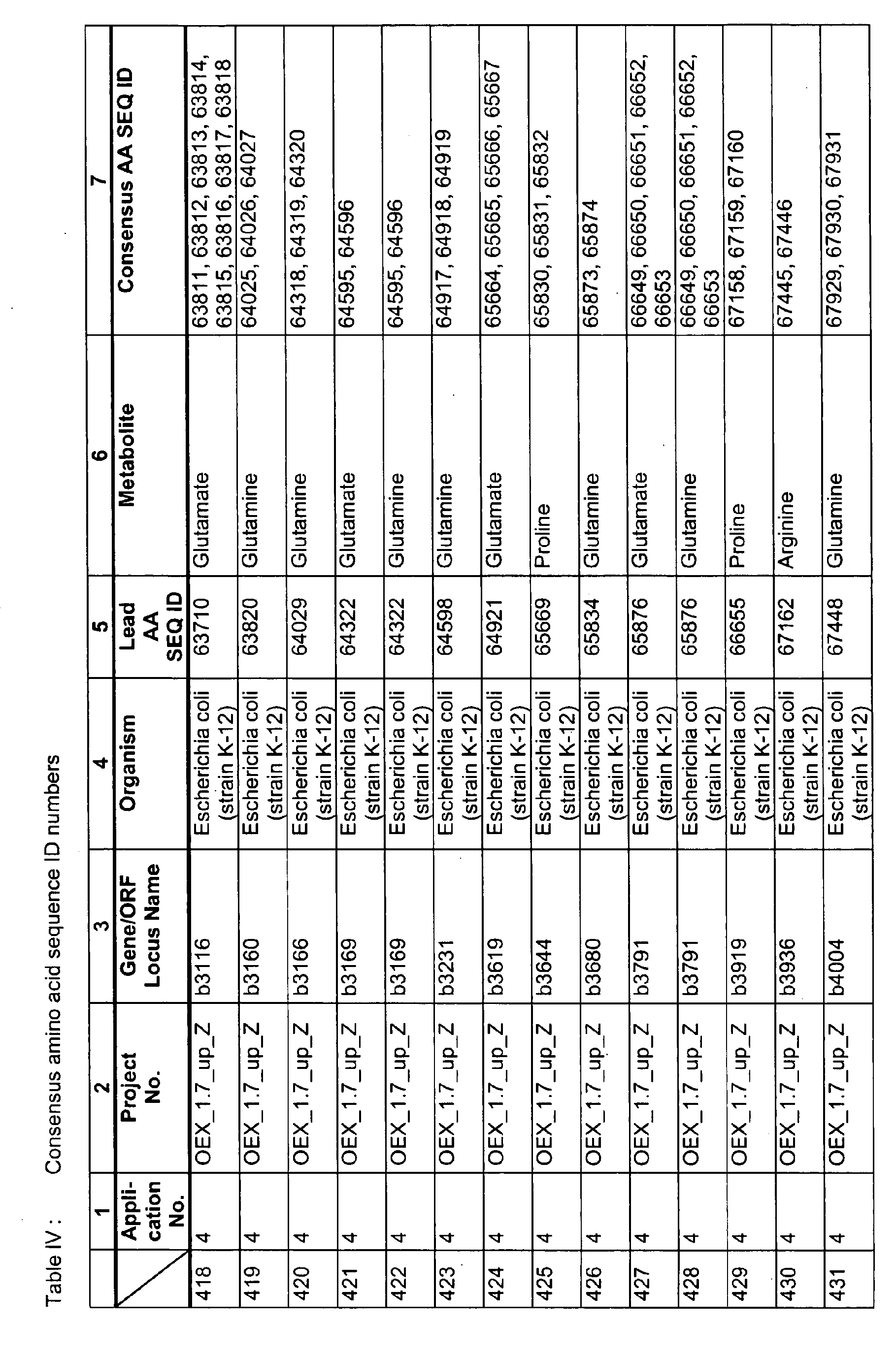

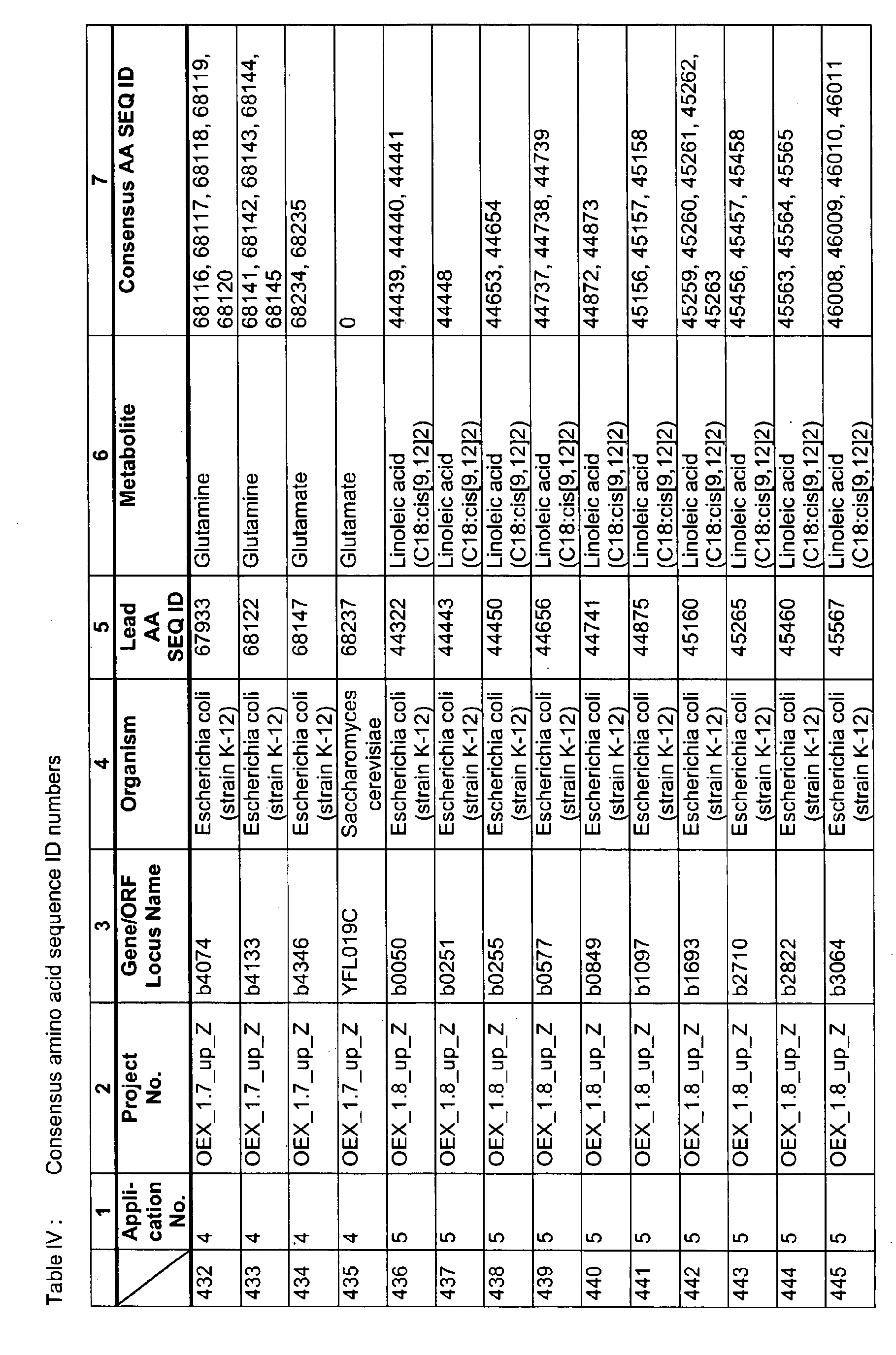

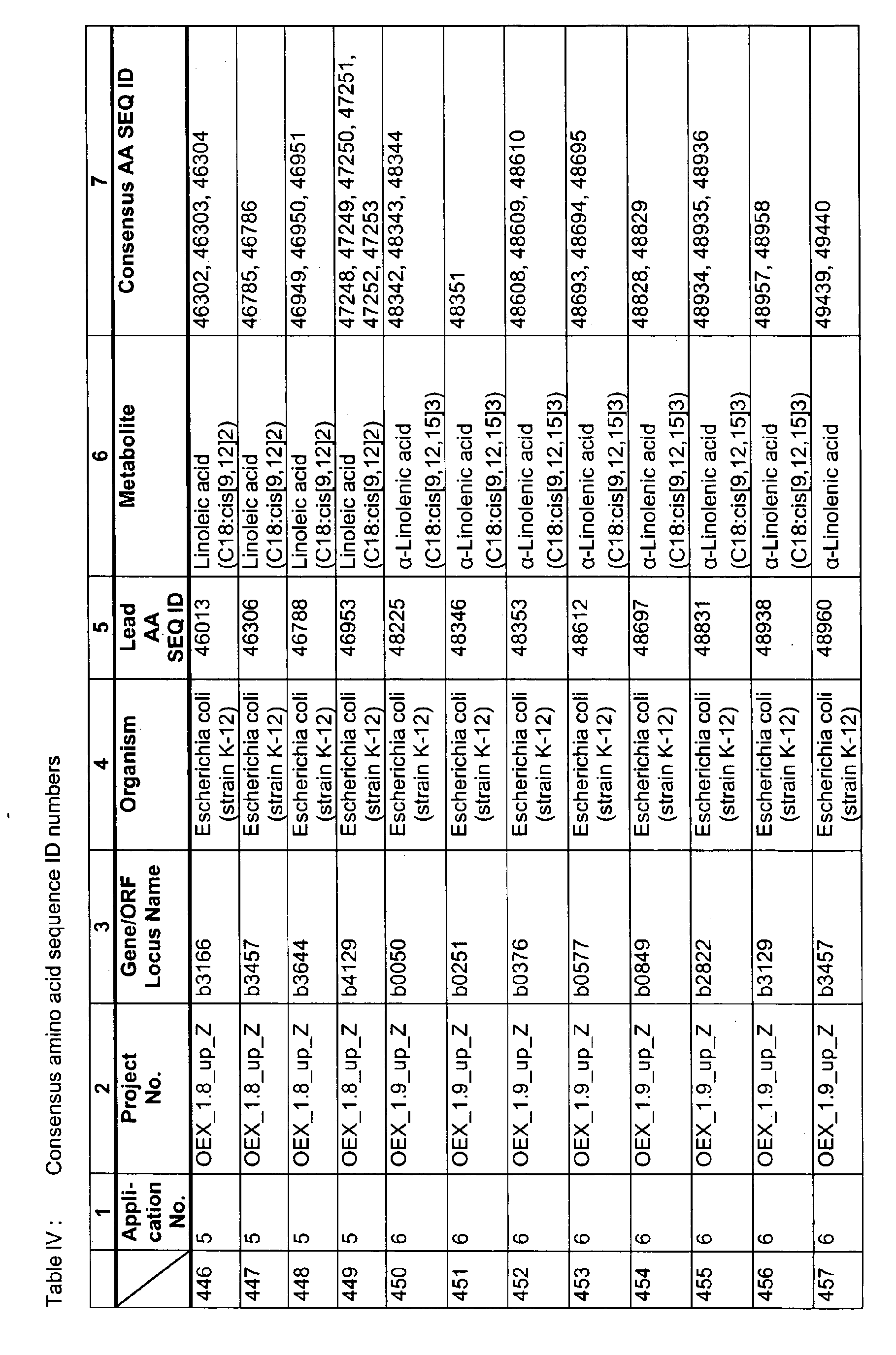

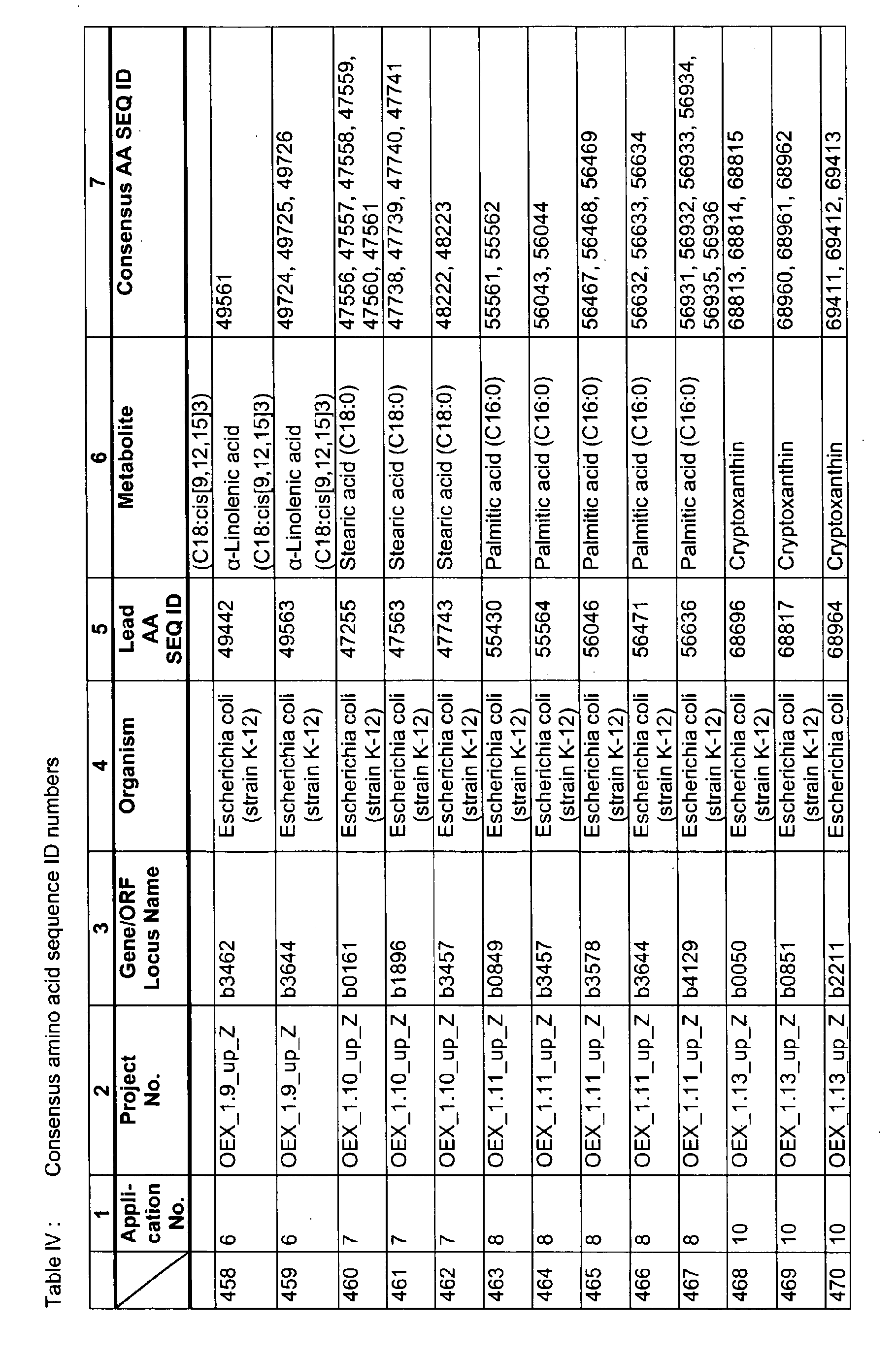

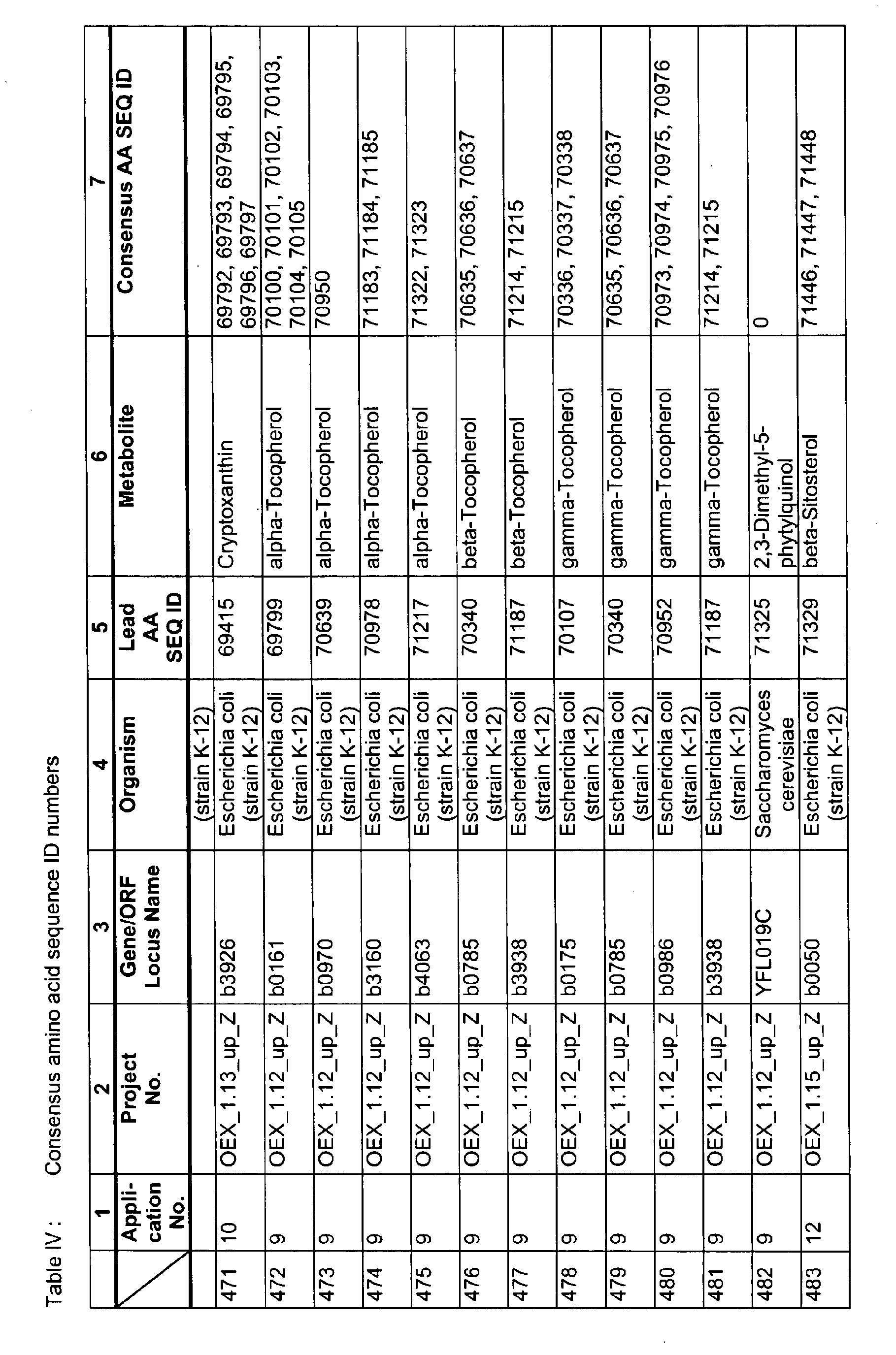

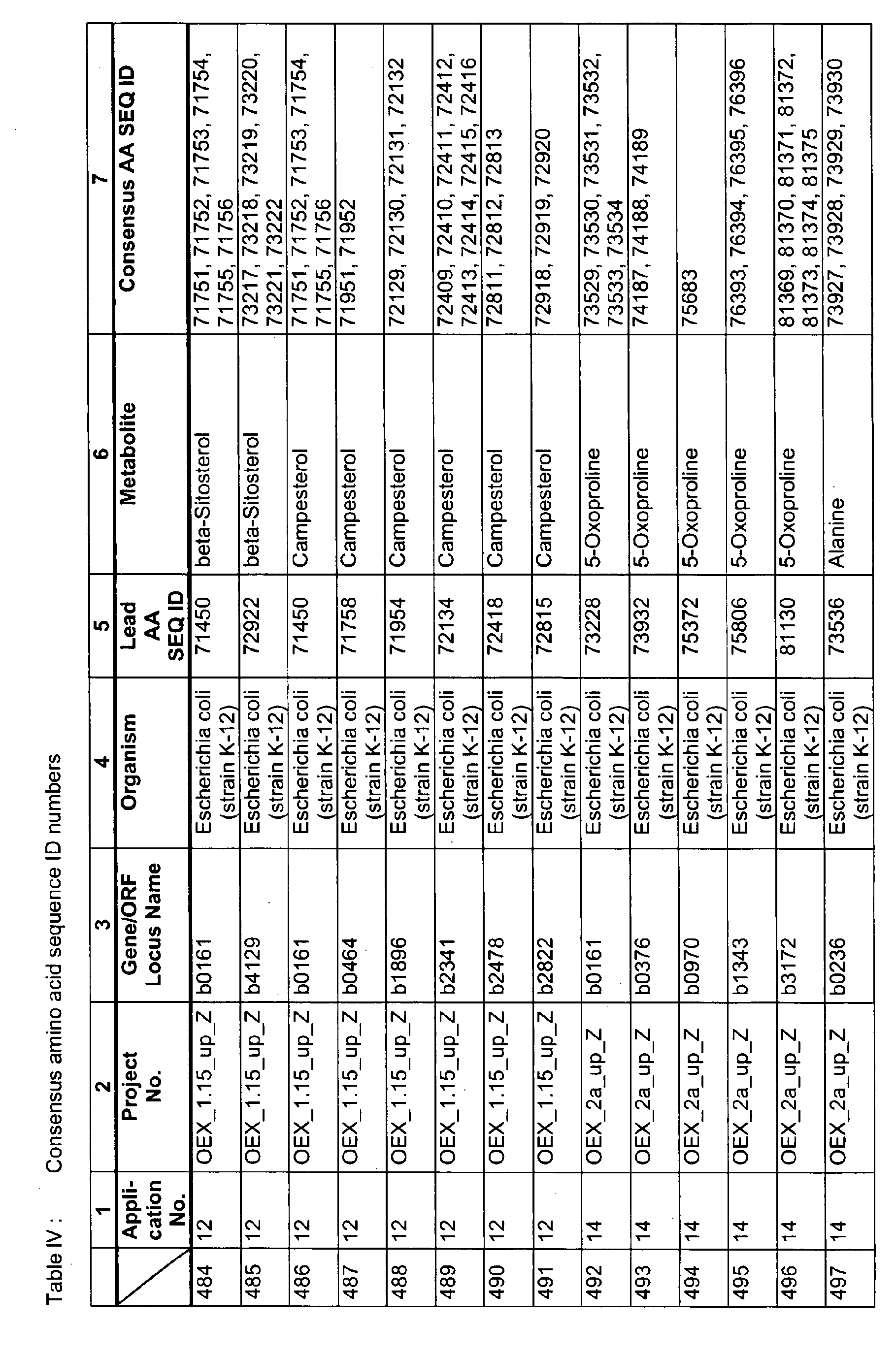

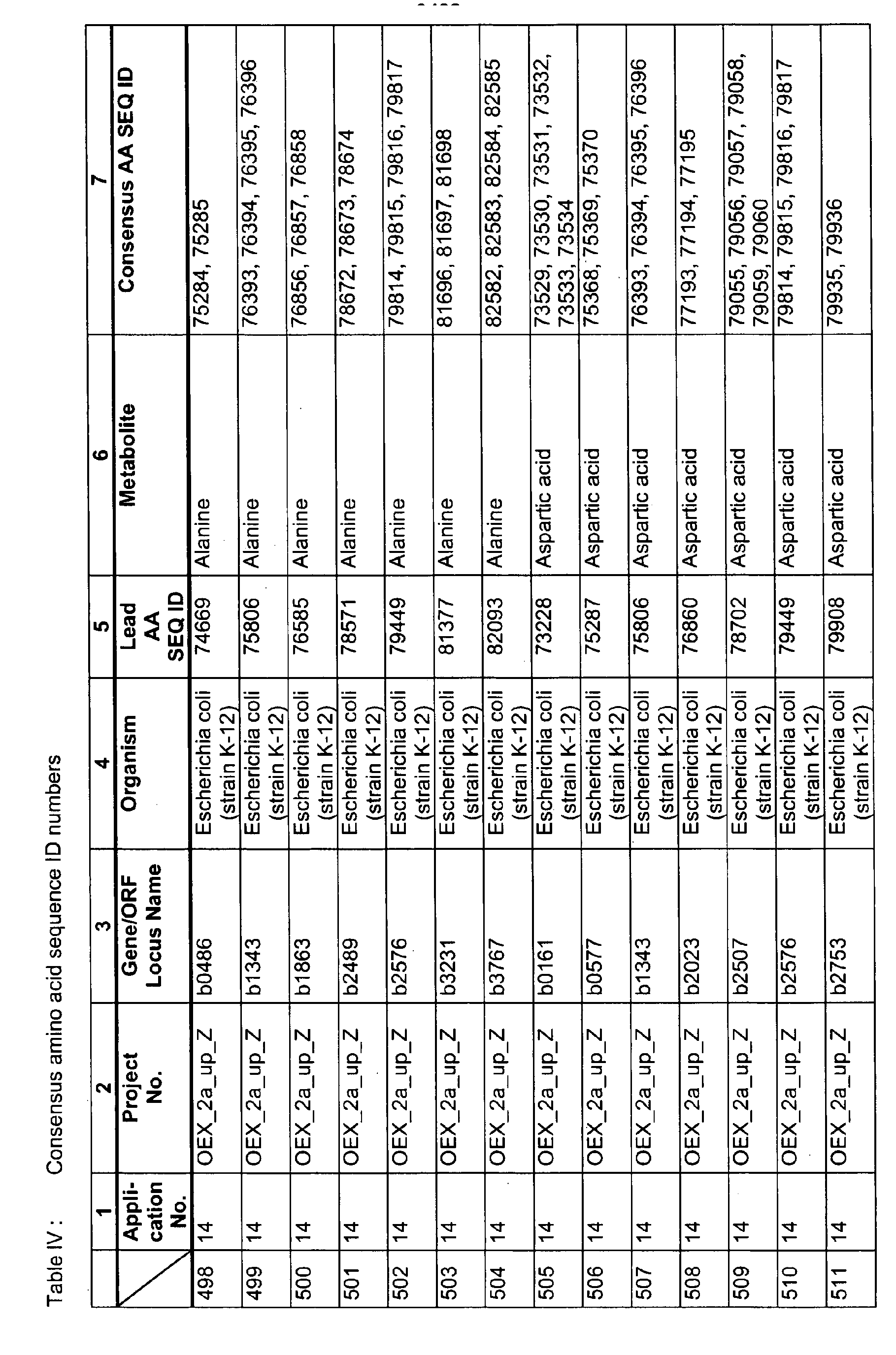

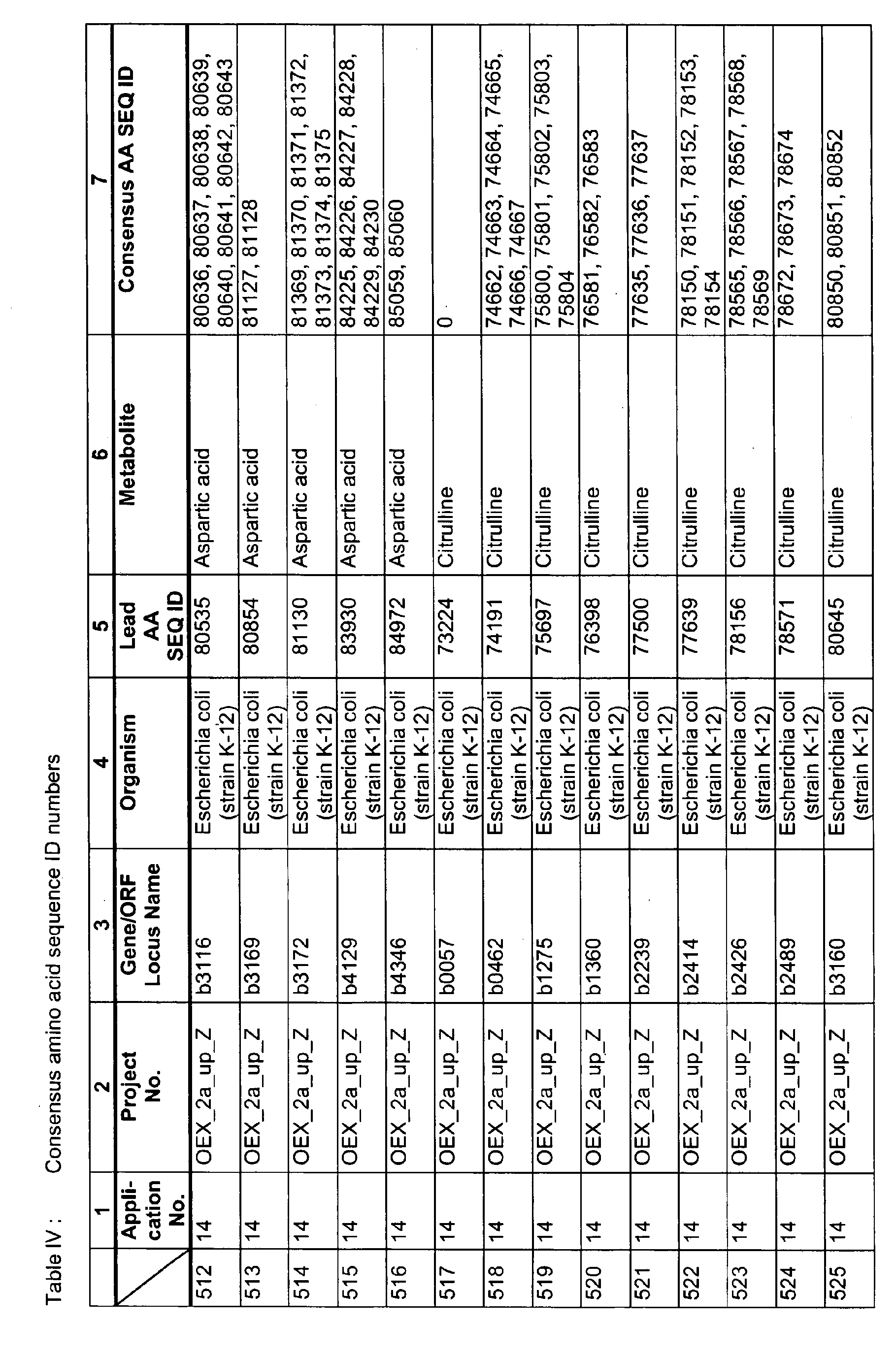

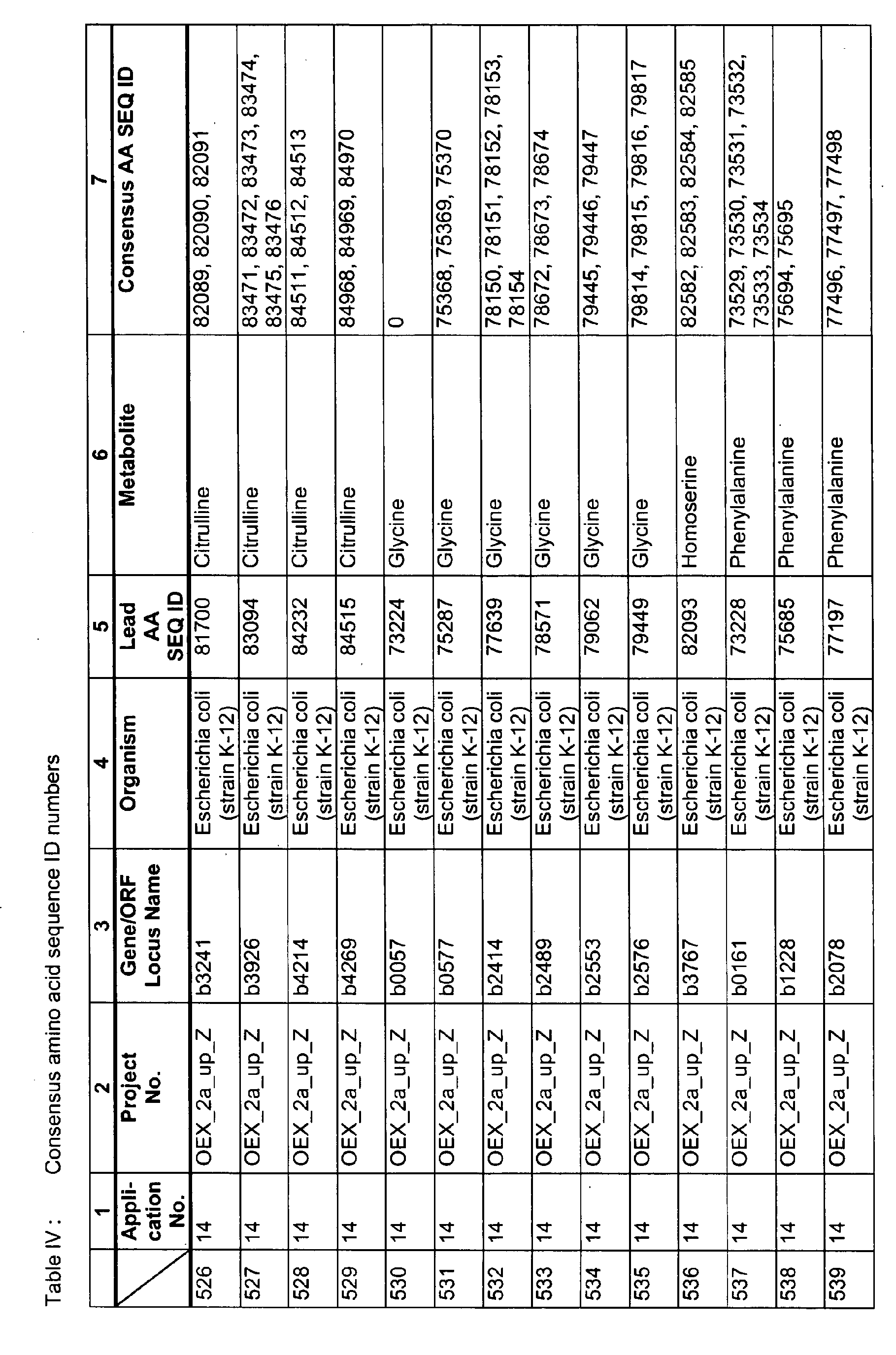

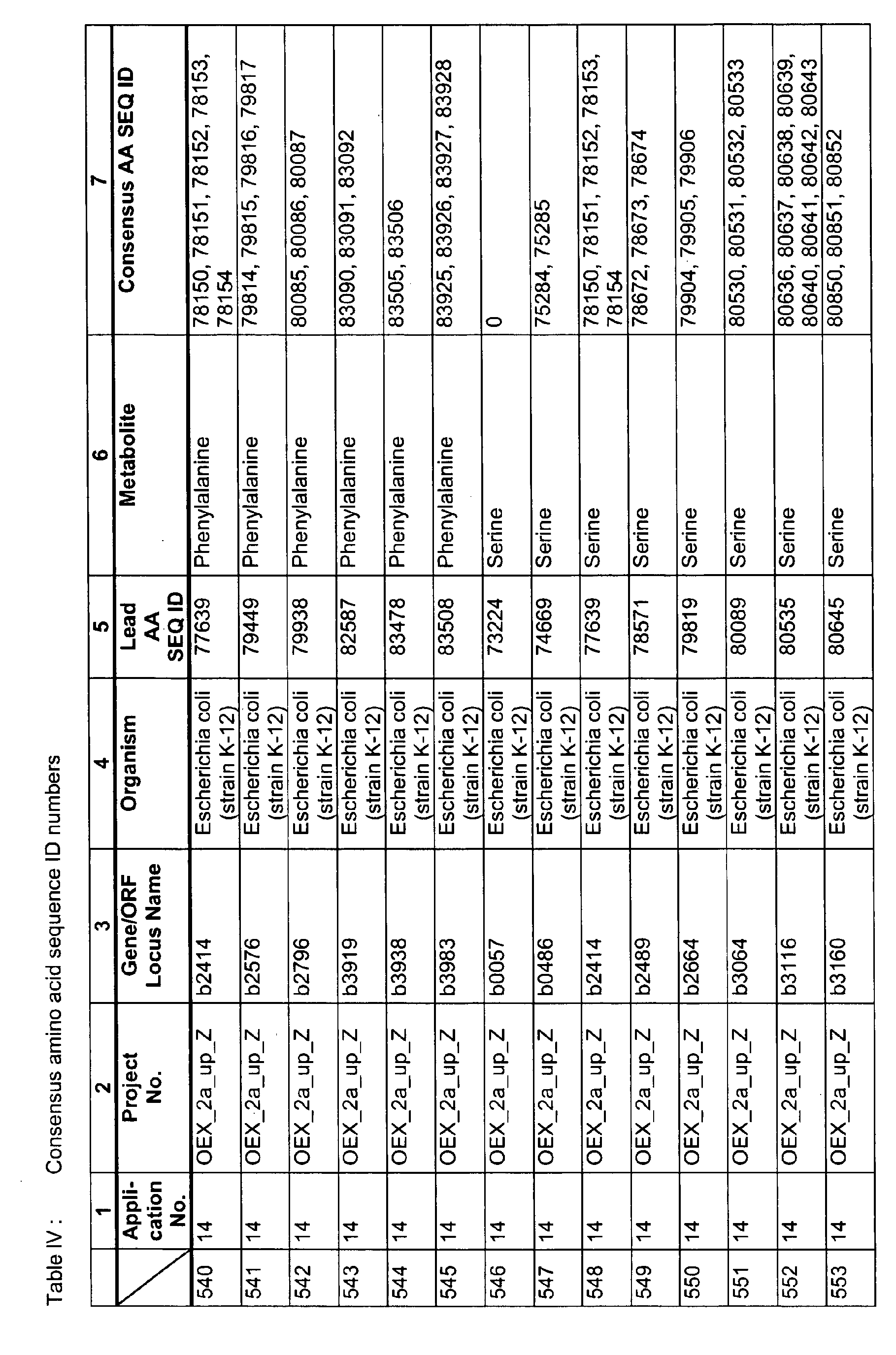

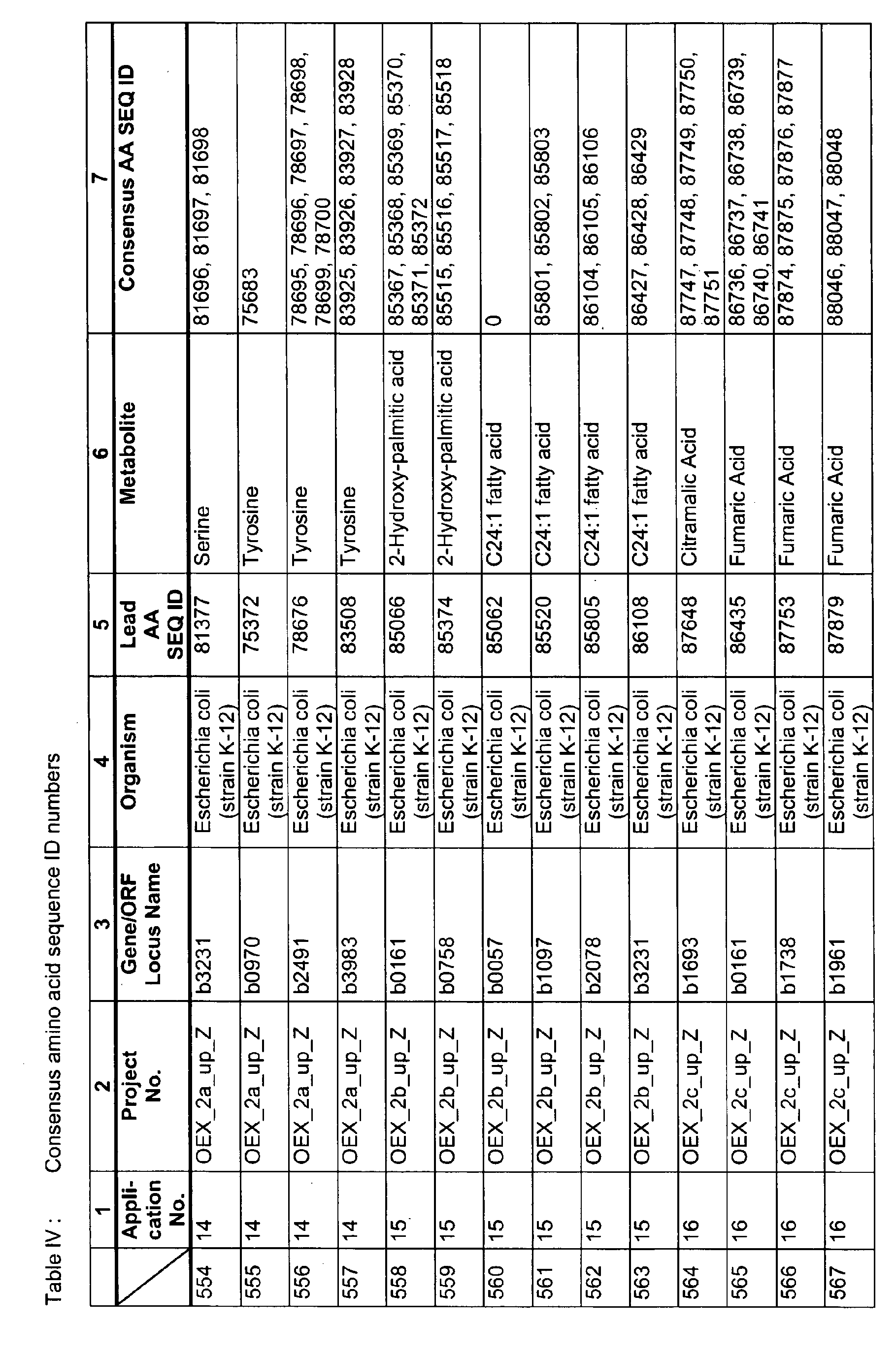

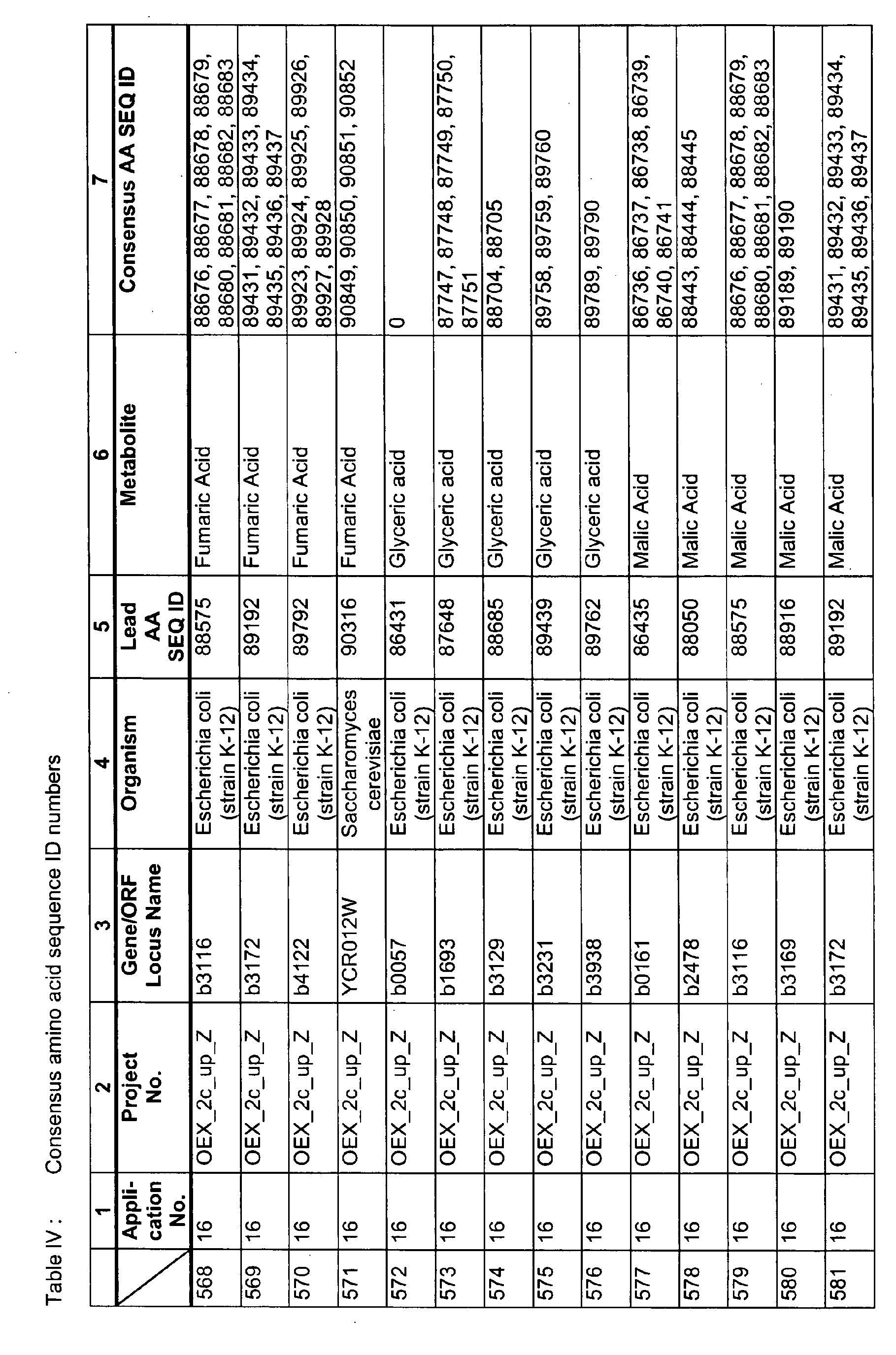

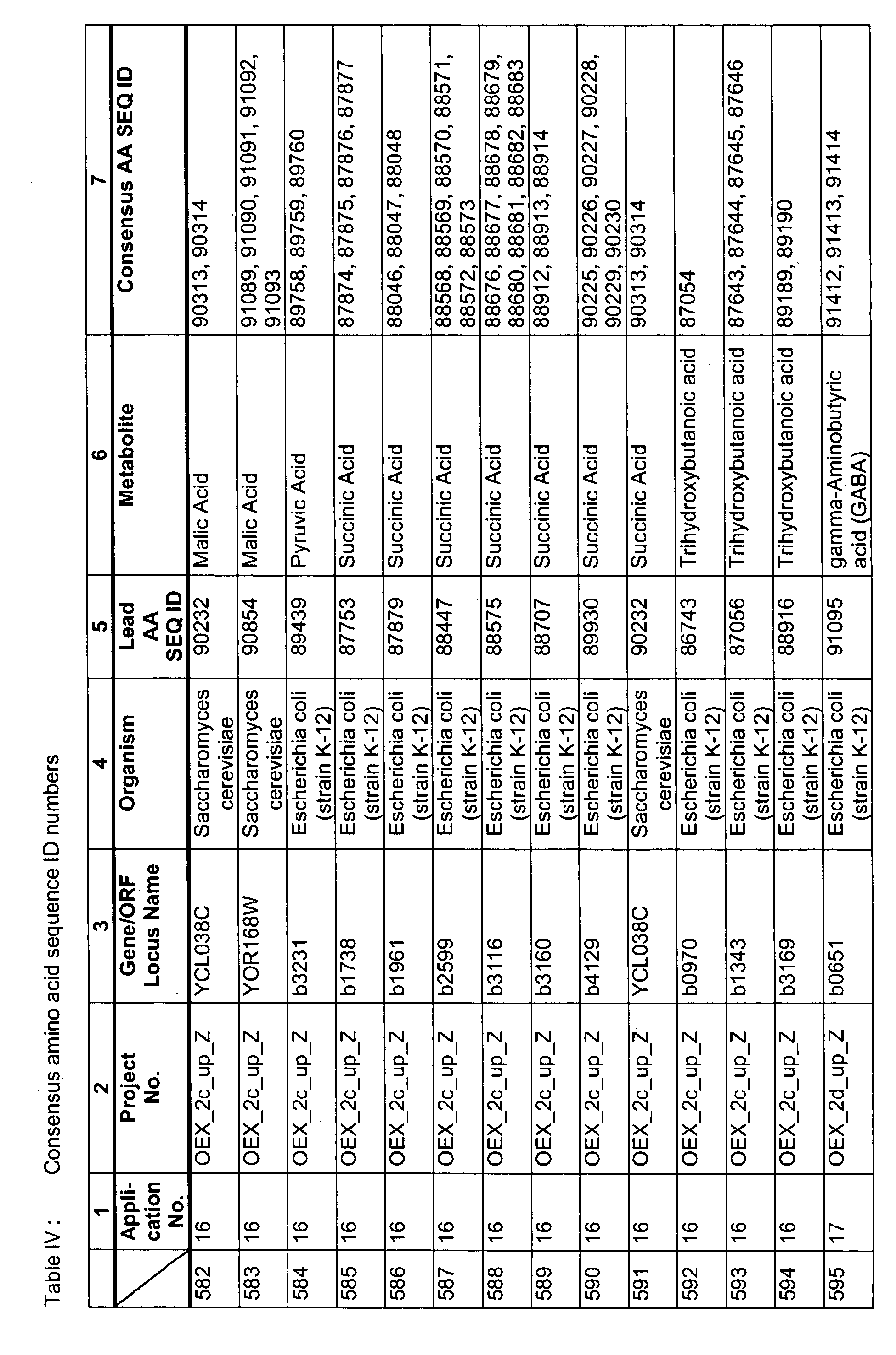

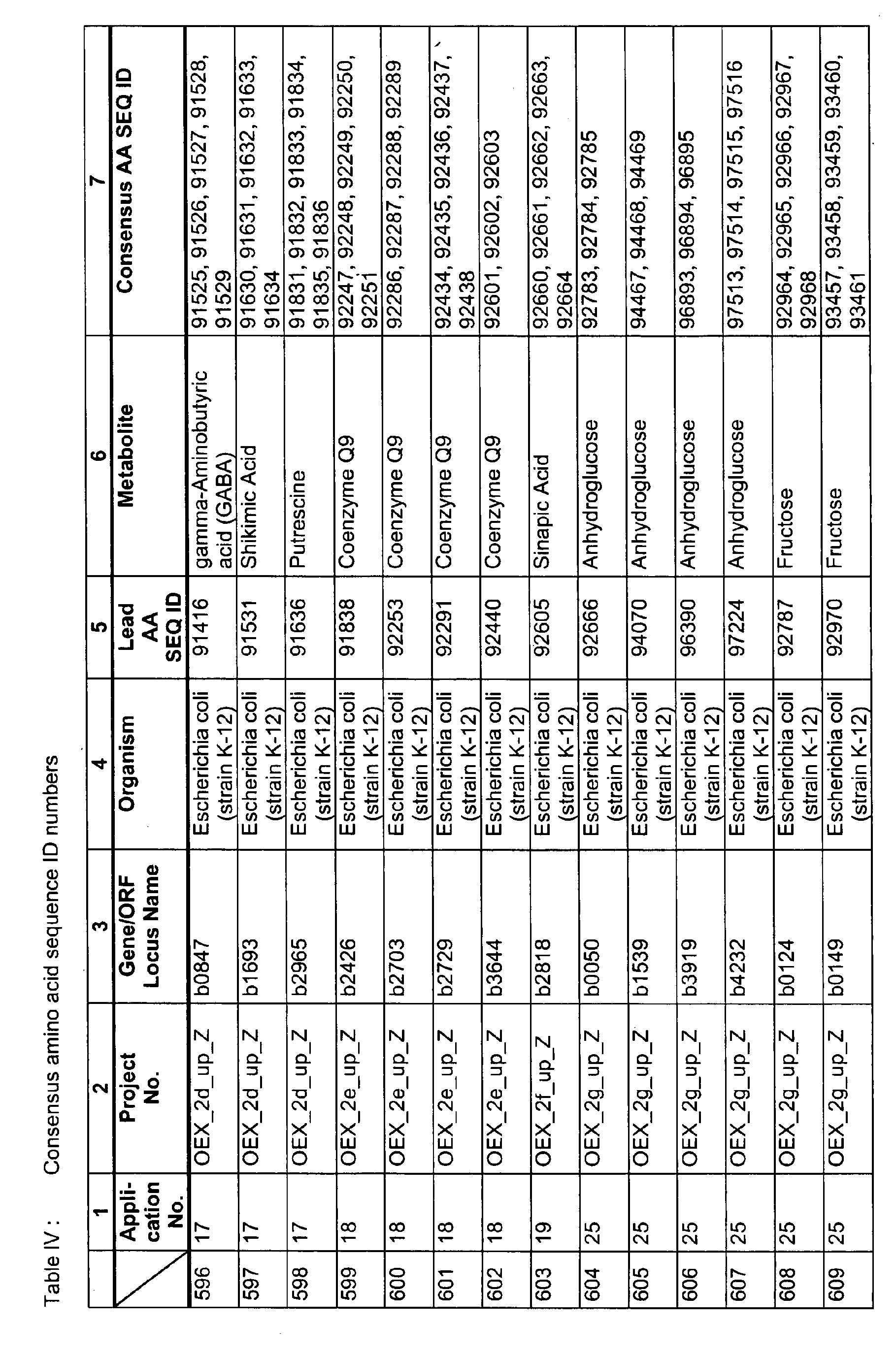

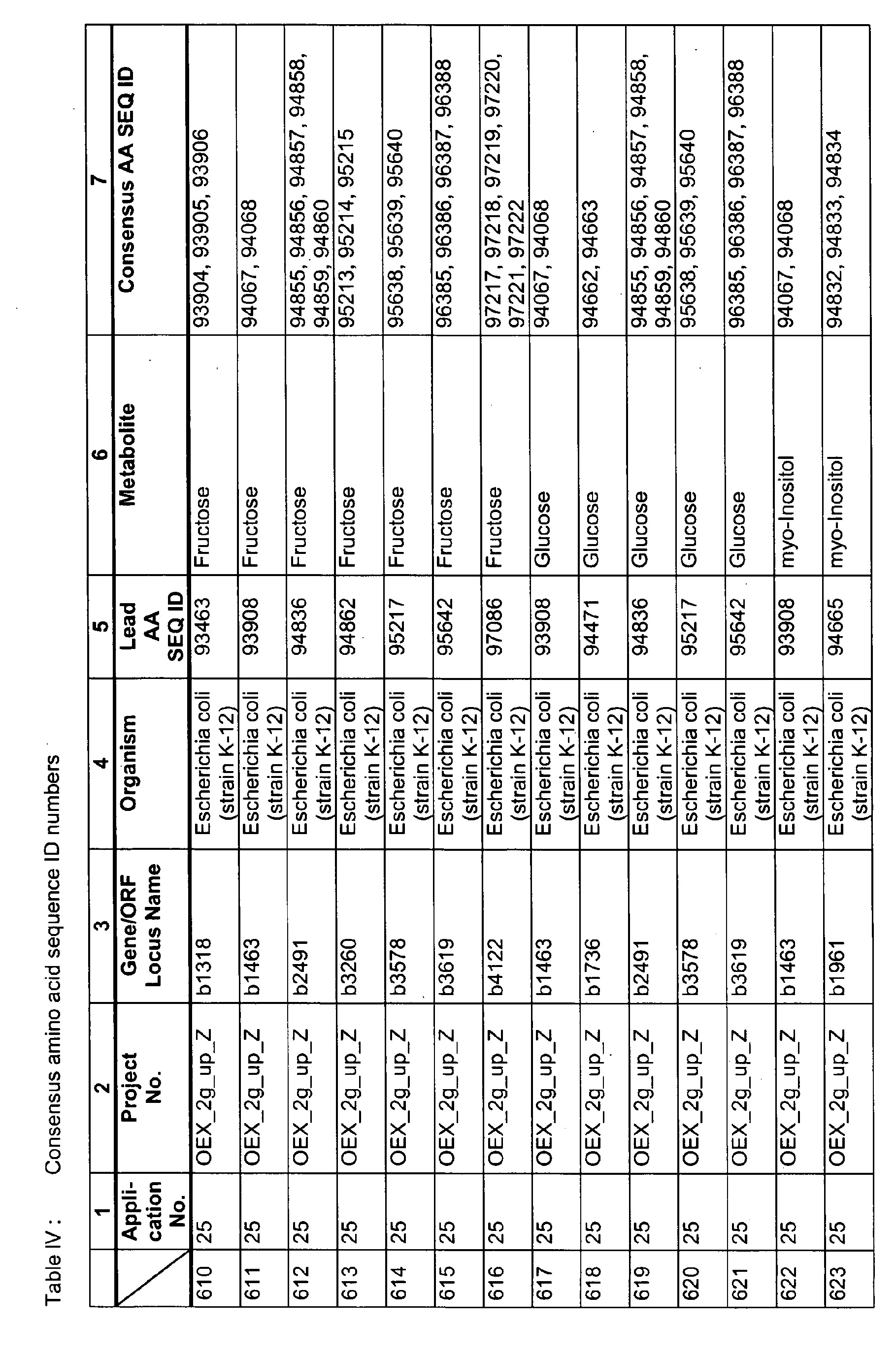

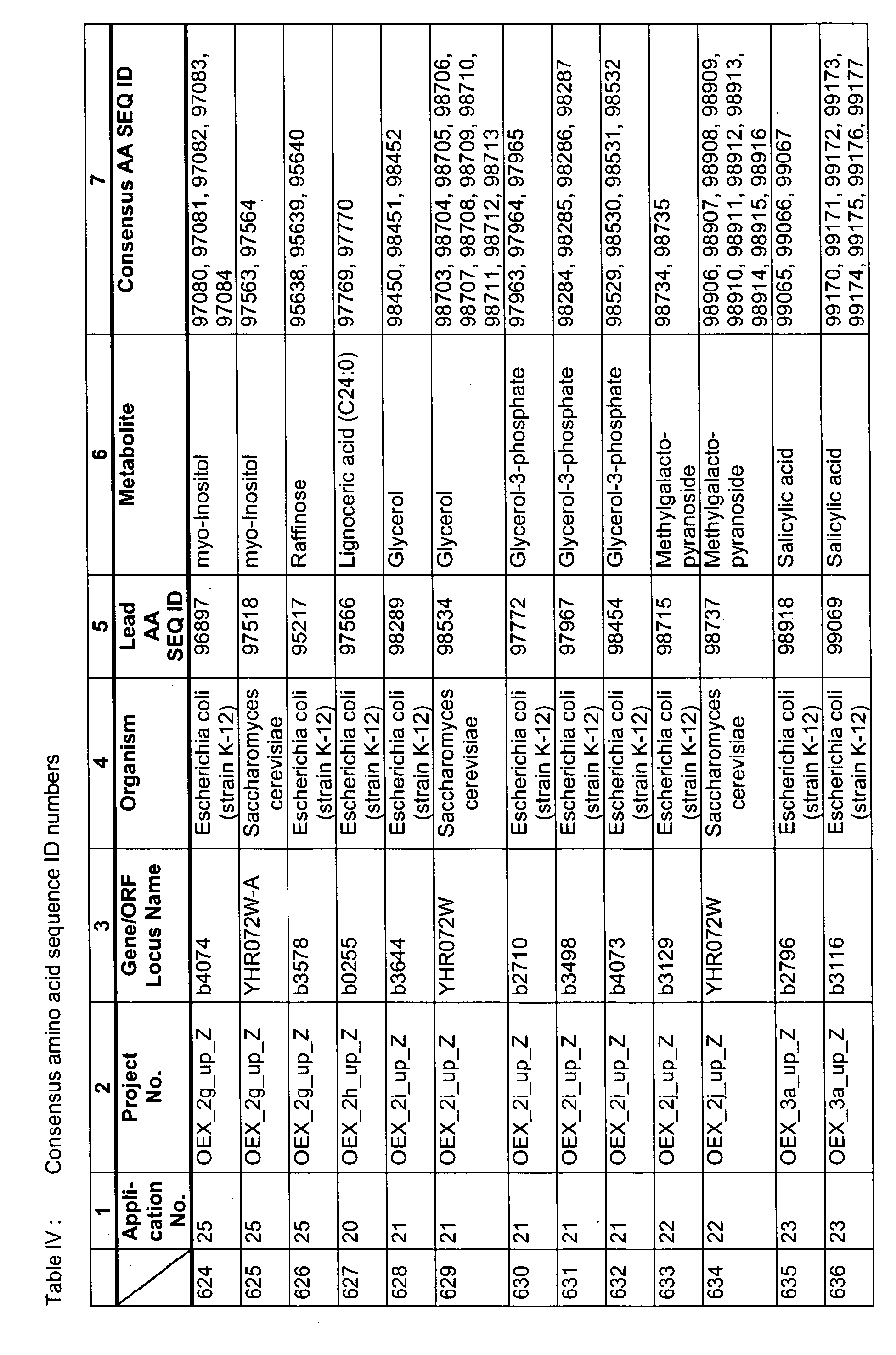

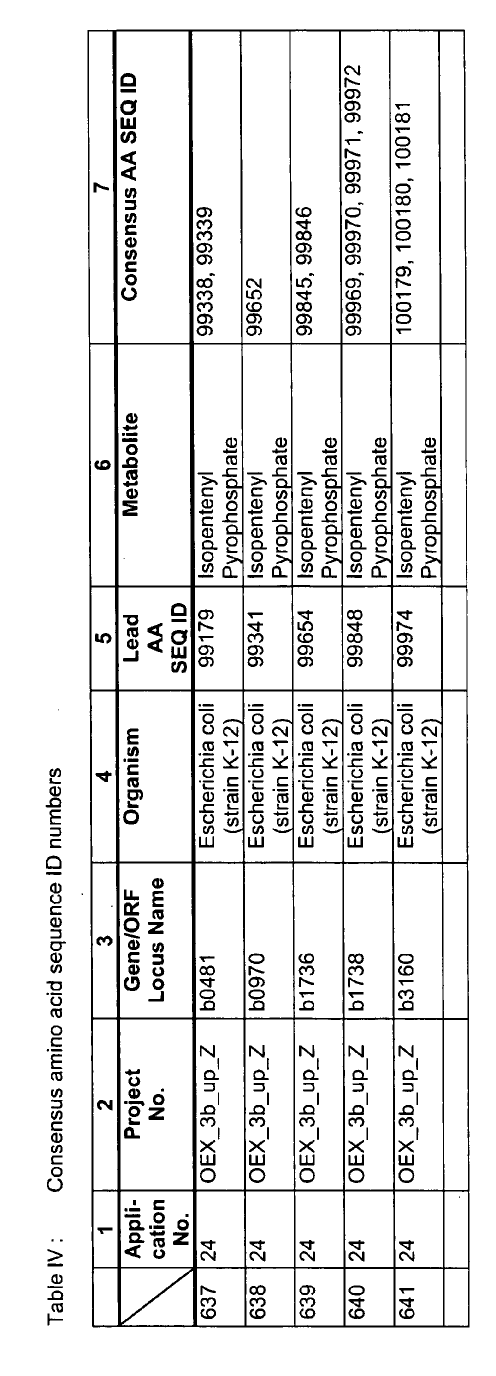

## Claims

1. A process for the production of leucine and/or isoleucine and/or valine, which comprises
(a) increasing or generating the activity of a b0124 protein in a non-human organism, or in one or more parts thereof; and
(b) growing the organism under conditions which permit the production of leucine and/or isoleucine and/or valine in said organism.

2. A process for the production of leucine and/or isoleucine and/or valine, comprising the increasing or generating in an organism or a part thereof the expression of at least one nucleic acid molecule comprising a nucleic acid molecule selected from the group consisting of:
a) nucleic acid molecule encoding the polypeptide shown in Table IIA or IIB, columns 5 or 7, lines 363 to 365 or a fragment thereof, which confers an increase in the amount of leucine and/or isoleucine and/or valine in an organism or a part thereof;
b) nucleic acid molecule comprising of the nucleic acid molecule shown in Table IA or IB, columns 5 or 7, lines 363 to 365;
c) nucleic acid molecule whose sequence can be deduced from a polypeptide sequence encoded by a nucleic acid molecule of (a) or (b) as a result of the degeneracy of the genetic code and conferring an increase in the amount of leucine and/or isoleucine and/or valine in an organism or a part thereof;
d) nucleic acid molecule which encodes a polypeptide which has at least 50% identity with the amino acid sequence of the polypeptide encoded by the nucleic acid molecule of (a) to (c) and conferring an increase in the amount of leucine and/or isoleucine and/or valine in an organism or a part thereof;
e) nucleic acid molecule which hybidizes with a nucleic acid molecule of (a) to (c) under under stringent hybridisation conditions and conferring an increase in the amount of leucine and/or isoleucine and/or valine in an organism or a part thereof;
f) nucleic acid molecule which encompasses a nucleic acid molecule which is obtained by amplifying nucleic acid molecules from a cDNA library or a genomic library using the primers shown in Table III, column 7, lines 363 to 365 and conferring an increase in the amount of leucine and/or isoleucine and/or valine in an organism or a part thereof;
g) nucleic acid molecule encoding a polypeptide which is isolated with the aid of monoclonal antibodies against a polypeptide encoded by one of the nucleic acid molecules of (a) to (f) and conferring an increase in the amount of leucine and/or isoleucine and/or valine in an organism or a part thereof;
h) nucleic acid molecule encoding a polypeptide comprising the consensus sequence shown in Table IV, column 7, lines 363 to 365 and conferring an increase in the amount of leucine and/or isoleucine and/or valine in an organism or a part thereof; and
i) nucleic acid molecule which is obtainable by screening a suitable nucleic acid library under stringent hybridization conditions with a probe comprising one of the sequences of the nucleic acid molecule of (a) to (k) or with a fragment thereof having at least 15 nt, preferably 20 nt, 30 nt, 50 nt, 100 nt, 200 nt or 500 nt of the nucleic acid molecule **characterized in** (a) to (k) and conferring an increase in the amount of leucine and/or isoleucine and/or valine in an organism or a part thereof or comprising a sequence which is complementary thereto.

3. The process of claim 1 or 2, comprising recovering of the free or bound leucine and/or isoleucine and/or valine.

4. The process of any one of claim 1 to 3, comprising the following steps:
(a) selecting an organism or a part thereof expressing a polypeptide encoded by the nucleic acid molecule **characterized in** claim 2;
(b) mutagenizing the selected organism or the part thereof;
(c) comparing the activity or the expression level of said polypeptide in the mutagenized organism or the part thereof with the activity or the expression of said polypeptide of the selected organisms or the part thereof;
(d) selecting the mutated organisms or parts thereof, which comprise an increased activity or expression level of said polypeptide compared to the selected organism or the part thereof;
(e) optionally, growing and cultivating the organisms or the parts thereof; and
(f) recovering, and optionally isolating, the free or bound leucine and/or isoleucine and/or valine produced by the selected mutated organisms or parts thereof.

5. The process of any one of claims 1 to 4, wherein the activity of said protein or the expression of said nucleic acid molecule is increased or generated transiently or stably.

6. An isolated nucleic acid molecule comprising a nucleic acid molecule selected from the group consisting of:
a) nucleic acid molecule encoding the polypeptide shown in Table IIA or IIB, columns 5 or 7, lines 363 to 365 or a fragment thereof, which confers an increase in the amount of leucine and/or isoleucine and/or valine in an organism or a part thereof;
b) nucleic acid molecule comprising the nucleic acid molecule shown in Table IA or IB, columns 5 or 7, lines 363 to 365;
c) nucleic acid molecule whose sequence can be deduced from a polypeptide sequence encoded by a nucleic acid molecule of (a) or (b) as a result of the degeneracy of the genetic code and conferring an increase in the amount of leucine and/or isoleucine and/or valine in an organism or a part thereof;
d) nucleic acid molecule which encodes a polypeptide which has at least 50% identity with the amino acid sequence of the polypeptide encoded by the nucleic acid molecule of (a) to (c) and conferring an increase in the amount of leucine and/or isoleucine and/or valine in an organism or a part thereof;
e) nucleic acid molecule which hybidizes with a nucleic acid molecule of (a) to (c) under under stringent hybridisation conditions and conferring an increase in the amount of leucine and/or isoleucine and/or valine in an organism or a part thereof;
f) nucleic acid molecule which encompasses a nucleic acid molecule which is obtained by amplifying nucleic acid molecules from a cDNA library or a genomic library using the primers shown in Table III, column 7, lines 363 to 365 and conferring an increase in the amount of leucine and/or isoleucine and/or valine in an organism or a part thereof;
g) nucleic acid molecule encoding a polypeptide which is isolated with the aid of monoclonal antibodies against a polypeptide encoded by one of the nucleic acid molecules of (a) to (f) and conferring an increase in the amount of leucine and/or isoleucine and/or valine in an organism or a part thereof;
h) nucleic acid molecule encoding a polypeptide comprising the consensus sequence shown in Table IV, column 7, lines 363 to 365 and conferring an increase in the amount of leucine and/or isoleucine and/or valine in an organism or a part thereof; and
i) nucleic acid molecule which is obtainable by screening a suitable nucleic acid library under stringent hybridization conditions with a probe comprising one of the sequences of the nucleic acid molecule of (a) to (k) or with a fragment thereof having at least 15 nt, preferably 20 nt, 30 nt, 50 nt, 100 nt, 200 nt or 500 nt of the nucleic acid molecule **characterized in** (a) to (k) and conferring an increase in the amount of leucine and/or isoleucine and/or valine in an organism or a part thereof,
whereby the nucleic acid molecule distinguishes over the sequence as shown in Table IA, columns 5 or 7, lines 363 to 365 by one or more nucleotides.

7. A nucleic acid construct which confers the expression of the nucleic acid molecule of claim 6, comprising one or more regulatory elements.

8. A vector comprising the nucleic acid molecule as claimed in claim 6 or the nucleic acid construct of claim 7, especially wherein the nucleic acid molecule is in operable linkage with regulatory sequences for the expression in a prokaryotic or eukaryotic, or in a prokaryotic and eukaryotic, host.

9. A host cell, which has been transformed stably or transiently with the vector as claimed in claim 8 or the nucleic acid molecule as claimed in claim 6 or the nucleic acid construct of claim 7 or produced as described in claim any one of claims 2 to 5, especially whereby the host cell is a transgenic host cell and/or preferably the host cell is a plant cell, an animal cell, a microorganism, or a yeast cell, a fungus cell, a prokaryotic cell, an eukaryotic cell or an archaebacterium.

10. A process for producing a polypeptide, wherein the polypeptide is expressed in a host cell as claimed in claim 9.

11. A polypeptide produced by the process as claimed in claim 10 or encoded by the nucleic acid molecule as claimed in claim 6 whereby the polypeptide distinguishes over the sequence as shown in Table IIA, columns 5 or 7, lines 363 to 365 by one or more amino acids

12. An antibody, which binds specifically to the polypeptide as claimed in claim 11.

13. A plant tissue, propagation material, harvested material or a plant comprising the host cell as claimed in claim 9 which is plant cell or an Agrobacterium.

14. A composition comprising the nucleic acid molecule of claim 6, the polypeptide of claim 11, the nucleic acid construct of claim 7, the vector of any one of claims 8, an antagonist or agonist of the activity of a polypeptide encoded by the nucleic acid molecule of claim 6 conferring an increase in the amount of leucine and/or isoleucine and/or valine in an organism or a part thereof, the compound conferring increased leucine and/or isoleucine and/or valine production in a plant or microorganism, the gene product conferring an increase in leucine and/or isoleucine and/or valine production in a cell, the antibody of claim 12, and optionally an agricultural acceptable carrier.

15. Use of the nucleic acid molecule as claimed in claim 6 for the identification of a nucleic acid molecule conferring an increase of leucine and/or isoleucine and/or valine after expression.

16. Use of the polypeptide of claim 11 or the nucleic acid construct claim 7 or the gene product conferring an increase in leucine and/or isoleucine and/or valine production in a cell identified according to the method of the invention for identifying compounds capable of conferring a modulation of leucine and/or isoleucine and/or valine levels in an organism.

17. Food or feed composition comprising the nucleic acid molecule of claim 6, the polypeptide of claim 11, the nucleic acid construct of claim 7, the vector of claim 8, the antagonist or agonist of the activity of a polypeptide encoded by the nucleic acid molecule of claim 6 conferring an increase in the amount of leucine and/or isoleucine and/or valine in an organism or a part thereof, the antibody of claim 12, the plant or plant tissue of claim 13, the harvested material of claim 13, the host cell of claim 9 or the gene product conferring an increase in leucine and/or isoleucine and/or valine production in a cell identified according to the method of the invention.

18. A plant cell, a plant cell organelle, a plant tissue, a plant or a part thereof, wherein the content of leucine and/or isoleucine and/or valine is increased by 10%, 20%, 30%, 50%, 70%, 100%, 150%, 200%, 300%, 500%, 1000%, 5000%, 10000%, 50000%, or 100000% or more in comparison to the variety deposited at the Institut für Pflanzengenetik und Kulturpflanzenforschung (IPK), Corrensstraße 3, D-06466 Gatersleben, Germany, with the youngest deposition date before June 2, 2005, and whereby content means the weight of leucine and/or isoleucine and/or valine relative to the dry weight of the plant cell, the plant cell organelle, the plant tissue, the plant or the part thereof, and whereby dry weight means the weight of the organic material of the plant cell, the plant cell organelle, the plant tissue, the plant or the part thereof less the amount of water included in the plant cell, the plant cell organelle, the plant tissue, the plant or the part thereof, preferably a plant cell, a plant cell organelle, a plant tissue, a plant or a part thereof, wherein the increase of leucine and/or isoleucine and/or valine is achieved by increasing or generating the activity of a protein as indicated in Table II A and B, columns 5 or 7, lines 363 to 365 or a functional equivalent thereof or a protein comprising a consensus sequence as indicated in Table IV.

19. A plant cell, a plant cell organelle, a plant tissue, a plant or a part thereof, wherein the content of leucine and/or isoleucine and/or valine is increased by 10%, 20%, 30%, 50%, 70%, 100%, 150%, 200%, 300%, 500%, 1000%, 5000%, 10000%, 50000%, or 100000% or more in comparison to a variety selected from the group consisting of
(a) G. hirsutum IPK Accession Number GOS 6 (D 120), GOS 7 (ST 446), GOS 10 (D 1635), GOS 17 (D 4302), or GOS 21 (D 5553), or G. areysianum Deflers, or G. incanum (Schwartz) Hillc., or G. raimondii Ulbr., or G. stocksii Masters, or G. thurberi Tod., or G. tomentosum Nutt. or G. triphyllum Hochr., or Gossypium arboreum IPK Accession Number GOS 13 (D 1634), GOS 16 (D 4240), GOS 18 (D 4505), GOS 19 (D 4506), GOS 20 (D 4750), or GOS 12 (D 1329), or Gossypium barbadense, or Gossypium herbaceum; and
(b) Brassica napus variety Mika, Brassica napus variety Digger, Brassica napus variety Artus, Brassica napus variety Terra, Brassica napus variety Smart, Brassica napus variety Olivine, Brassica napus variety Libretto, Brassica napus variety Wotan, Brassica napus variety Panther, Brassica napus variety Express, Brassica napus variety Oase, Brassica napus variety Elan, Brassica napus variety Ability, Brassica napus variety Mohican; and
(c) Linum usitatissimum variety Librina, Linum usitatissimum variety Flanders, Linum usitatissimum variety Scorpion, Linum usitatissimum variety Livia, Linum usitatissimum variety Lola, Linum usitatissimum variety Taurus, Linum usitatissimum variety Golda, Linum usitatissimum variety Lirima, and
(d) Zea mays variety Articat, Zea mays variety NK Dilitop, Zea mays variety Total, Zea mays variety Oldham, Zea mays variety Adenzo, Zea mays variety NK Lugan, Zea mays variety Liberal, Zea mays variety Peso; and
(e) Glycine max variety Oligata, Glycine max variety Lotus, Glycine max variety Primus,Glycine max variety Alma Ata, Glycine max variety OAC Vision, Glycine max variety Jutro; and
(f) Helianthus annus variety Helena, Helianthus annus variety Flavia, Helianthus annus variety Rigasol, Helianthus annus variety Flores, Helianthus annus variety Jazzy, Helianthus annus variety Pegaso, Helianthus annus variety Heliaroc, Helianthus annus variety Salut RM; and
(g) Camelina sativa variety Dolly, Camelina sativa variety Sonny, Camelina sativa variety Ligena, Camelina sativa variety Calinka; and
(h) Sinapis alba variety Martigena, Sinapis alba variety Silenda, Sinapis alba variety Sirola, Sinapis alba variety Sito, Sinapis alba variety Semper, Sinapis alba variety Seco; and
(i) Carthamus tinctorius variety Sabina, Carthamus tinctorius variety HUS-305, Carthamus tinctorius variety landrace, Carthamus tinctorius variety Thori-78, Carthamus tinctorius variety CR-34, Carthamus tinctorius variety CR-81; and
(j) Brassica juncea variety Vittasso, Brassica juncea variety Muscon M-973, Brassica juncea variety RAPD, Brassica juncea variety Co.J.86, Brassica juncea variety IAC 1-2, Brassica juncea variety Pacific Gold; and
(k) Cocos nucifera L. varietes Maypan, Ceylon Tall, Indian Tall, Jamaica Tall, Malayan Tall, Java Tall, Laguna, King CRIC 60, CRIC 65, CRISL 98, Moorock tall, Plus palm tall, San Ramon, Typica, Nana or Aurantiaca; and
(l) Triticum aestivum L. variety Altos, Bundessortenamt file number 2646, Triticum aestivum L. variety Bussard, Bundessortenamt file number 1641, or Triticum aestivum L. variety Centrum, Bundessortenamt file number 2710; and
(m) Beta vulgaris variety Dieck 13, CPVO file number 19991828, Beta vulgaris variety FD 007, CPCO file number 20000506, or Beta vulgaris variety HI 0169, CPVO file number 20010315; and
(n) Hordeum vulgare variety Dorothea, CPVO file number 20031457, Hordeum vulgare variety Colibri, CPVO file number 20040122, Hordeum vulgare variety Brazil, CPVO file number 20010274, or Hordeum vulgare variety Christina, CPVO file number 20030277; and
(o) Secale cereale variety Esprit, CPVO file number 19950246, Secale cereale variety Resonanz, CPVO file number 20040651, or Secale cereale variety Ursus, CPVO file number 19970714; and
(p) Oryza sativa variety Gemini, CPVO file number 20010284, Oryza sativa variety Tanaro, CPVO file number 20020177, or Oryza sativa variety Zeus, CPVO file number 19980388; and
(q) Solanum tuberosum L. varieties Linda, Nicola, Solara, Agria, Sieglinde, or Russet Burbank; and
(r) Arachis hypogaea subsp. fastigiata cultivar Valencia; and
(s) Arachis hypogaea subsp. hypogaea cultivar Virginia variety 'Holland Jumbo', 'Virginia A23-7', or'Florida 416'; and
(t) Arachis hypogaea subsp. hirsuta cultivar Peruvian runner variety 'Southeastern Runner 56-15', 'Dixie Runner', or'Early Runner'; and
(u) Arachis hypogaea subsp. vulgaris cultivar Spanish variety 'Dixie Spanish', 'Improved Spanish 2B', or'GFA Spanish';
and whereby content means the weight of leucine and/or isoleucine and/or valine relative to the dry weight of the plant cell, the plant cell organelle, the plant tissue, the plant or the part thereof, and whereby dry weight means the weight of the organic material of the plant cell, the plant cell organelle, the plant tissue, the plant or the part thereof less the amount of water included in the plant cell, the plant cell organelle, the plant tissue, the plant or the part thereof,
preferably a plant cell, a plant cell organelle, a plant tissue, a plant or a part thereof, wherein the increase of leucine and/or isoleucine and/or valine is achieved by increasing or generating the activity of a protein as indicated in Table II A and B, columns 5 or 7, lines 363 to 365 or a functional equivalent thereof or a protein comprising a consensus sequence as indicated in Table IV.
